(19) Europäisches Patentamt
European Patent Office
Office européen des brevets

(11) EP 1 605 061 A1

(12) # EUROPEAN PATENT APPLICATION

(43) Date of publication:
**14.12.2005 Bulletin 2005/50**

(51) Int Cl.[7]: **C12Q 1/68**, C12N 15/11, C07K 14/22

(21) Application number: **05075284.9**

(22) Date of filing: **08.03.2000**

(84) Designated Contracting States:
**AT BE CH CY DE DK ES FI FR GB GR IE IT LI LU MC NL PT SE**

(30) Priority: **30.04.1999 US 132068 P**
**08.10.1999 WOPCT/US99/23573**
**28.02.2000 GB 0004695**

(62) Document number(s) of the earlier application(s) in accordance with Art. 76 EPC:
**00910392.0 / 1 185 691**

(71) Applicants:
- **CHIRON CORPORATION**
  **Emeryville, California 94608 (US)**
- **THE INSTITUTE FOR GENOMIC RESEARCH**
  **Rockville, Maryland 20850 (US)**

(72) Inventors:
- **Pizza, Mariagrazia**
  **53100 Siena (IT)**
- **Hickey, Erin, The institute for Genomic Research**
  **Rockville, MD 20850 (US)**
- **Peterson, J., The institute for Genomic Research**
  **Rockville, MD 20850 (US)**
- **Tettelin,Herve,**
  **The institute for Genomic Research**
  **Rockville, MD 20850 (US)**
- **Masignani, Vega**
  **53100 Siena (IT)**
- **Galeotti, Cesira**
  **53100 Siena (IT)**
- **Mora, Marirosa**
  **53100 Siena (IT)**
- **Ratti, Giulio**
  **53100 Siena (IT)**
- **Scarselli, Maria**
  **53100 Siena (IT)**
- **Scarlato, Vincenzo**
  **53100 Siena (IT)**
- **Rappuoli, Rino**
  **53100 Siena (IT)**
- **Fraser, Claire M.**
  **Emeryville, CA 94608 (US)**
- **Grandi, Guido**
  **53100 Siena (IT)**
- **Venter, Craig,J**
  **The institute for Genomic Research**
  **Rockville, MD 20850 (US)**

(74) Representative: **Marshall, Cameron John et al**
**Carpmaels & Ransford**
**43-45 Bloomsbury Square**
**London WC1A 2RA (GB)**

Remarks:
This application was filed on 03 - 02 - 2005 as a divisional application to the application mentioned under INID code 62. The sequence listing, which is published as annex to the application documents, was filed after the date of filing. The applicant has declared that it does not include matter which goes beyond the content of the application as filed.

(54) **Neisseria genomic sequences and methods of their use**

(57) The invention provides methods of obtaining immunogenic proteins from genomic sequences including *Neisseria*, including the amino acid sequences and the corresponding nucleotide sequences, as well as the genomic sequence of *Neisseria meningitidis B.* The proteins so obtained are useful antigens for vaccines, immunogenic compositions, and/or diagnostics.

EP 1 605 061 A1

Annex to the application documents - subsequently filed sequences listing

SEQUENCE LISTING

<110> CHIRON CORPORATION

<120> NEISSERIA GENOMIC SEQUENCES AND METHODS OF THEIR USE

<140> EP 05075284.9
<141> 2000-03-08

<150> US 60/132068
<151> 1999-04-30

<150> PCT/US99/23573
<151> 1999-10-08

<150> GB 0004695.3
<151> 2000-02-28

<150> EP 00910392.0
<151> 2000-03-08

<160> 109

<170> SeqWin99, version 1.0.4

<210> 1
<211> 2242716
<212> DNA
<213> Neisseria meningitidis

<400> 1
taaaccttat ccacatccaa acgcataacc gtaacccatt caccgttatg gaaatgtcgc 60
ccgacaacca cccagccgaa tgattcataa aatatttgca catcaggcgt ataaagatac 120
aagaacttta tccccagcga acgcgctgcg cctatgcagt gggcgaccag cctcctgcca 180
atgccttttc cgcgatattc aggtaaaaca aagacatccc ccaaccaata ttcataccgt 240
ggaaaacttt ccatatcatg ccgcttgacc gcagccgaac ccaacaggat tccggaatca 300
tccacagccg caaatgccag cggcagttcg tcatccttca aacacctgcc gtaataggca 360
tgaatcttat ccacagaaga ccacggttca aatccgtgcc actcctcaaa caacgcctga 420
accaacctgc cgatatgccc ggctttcagc cgtgtaatga aaacagtatt gtccacaaag 480
agggaattca tcggtcaatt ccccgacgcc ttcgttcccc ctgcgccgta aaccgcattc 540
caagcatggt ccaaacgcac tccgatttgc ctcaaatctt cagcctgccg ggctttttgc 600
gccattgctg caggaatttc cgcttccaaa cgggcgatgt ctgcctgagc cgtctgcaaa 660
cgccggcgcg catcttccaa atccgactgc atcccgatga tttttccgtc cagattgttt 720
tgcttttgca ataaggcgcg gtaaccggat tggatgctga gcagattgtc ttcagcatcc 780
cctgcccata cgcttgtaga aaaaacaacc atcagaaaat aaaatatttt tttcattttt 840
aacttccatt taaatgctgt ctgaagccgt attccgacat cagacggcat cgcccacgcc 900
tgtggataac ttaagcgcgg atgcgtttca acacttcttc tttgccgatt aatgccaaca 960
cagcatcgac gctgggggtt ttcgccgtac cgcagacggc aaggcgcagg ggcatgccga 1020
gtttgcccat tttaatgcct tcttcgtcgc agaagggttt gaagaggtcg tggatggctt 1080
cggcattcca gtcttccagc ccttcgaggc gttcggcaaa gcgcagcata cgggcggcgg 1140
cttcatcgtc ccagtgtttc tgcacgtctg cttcggcagg cgtttgtttg acgtagaagt 1200
agaagcactc gtcggcaagc gtgttcaagt cttgggggcg gtctttgacc agttccaaca 1260
catcttccaa agcaggtttt tcggtttcat gaatatcgcg caacgcaagg cggggtttga 1320
cgagttcggc gagtttgccg ttgggtgtga ttttgatgtg ttcgccgttg atccagtaga 1380
gtttttttcaa gtccatacgg cttggagacg gggaaacgtc tttcaaatca aaccattcga 1440
tgaattgttc cattgtgaag aattcatcgt cgccgtgcgc ccagcccaag cgtgccagat 1500
agttgagcat cgcttcgggc aggatgccca ttgcgccgaa atcggtaatg gcaacggtat 1560
cgccgctgcg tttggagatt tttttgcctt gttcgttaag aatcatcggc aggtggccgt 1620
attcgggcag gttcgcgtcg atggctttta agatgttgat ttgtttcggc gtgttgttca 1680
catggtcgtc gccgcggata acgtgggtaa cgcccatgtc gtagtcgtct acgacaacgc 1740
agaagttgta ggtcggcgta ccgtcggcgc gggcgataat caggtcatcg agtgcttcgt 1800

```
tggggatgga gatttcgcct ttgaccaagt ctgtccattt ggtcacaccg tccaaaggcg 1860
ttttgaaacg gacaacgggt tgtacgtcgg acgggatttc gggcagggtt ttacctactt 1920
ccggacgcca gcggcggtcg taagtcgccg agccttcttt ttcggctttc tcacgcatgg 1980
cttccagctc ttctttgctg caatagcagt agtaggcatg gcctttttct aaaagttcgg 2040
caatgacctc tttgtagcgg tcgaaacggc gagtttggta aacgacgttg tcggcgttgt 2100
cgtaattgag accgacccat ttcatgccgt cgaggatgat gttgacggat tcggcggtag 2160
aacgcgccaa gtcggtgtct tcaatacgta ataggaactc gcctttatga tggcgggcaa 2220
acgcccatga aaacaaggcg gtgcgcacgc cgccgatgtg caggtagccg gtggggctgg 2280
gggcgaaacg ggttttgacg gtcatgatgg ctccgaaatc tttgaaagcg tttattttac 2340
tggttttacc gtgcttgggc atcaaaaatg ccgtctgaac cctgcctgcg gataaagttt 2400
cagacggcat tttccttgtt ttcaatgctt cggcacgcgg aacagtgtat cacgcgccgc 2460
cgaccgaatt ccttcgggat tgcgtccaaa aaaaagttca atgaaacagc taattgaaaa 2520
aatcccgccc ccatttttcc aaacggtaga gggataacgc atatccctct tgcagcataa 2580
agattttttt cttatttccc gcatcaaacc gcgtggtcgg cgtggcagac atataaacgc 2640
ggacacccaa atcctccgcc atttccgccg cccgcgccaa atggtaggga tcgctgacaa 2700
tcaccacgct ggcaataccg ttggcacgca aaaccggacg gatgttgttc aggttttcat 2760
aagtgttgcg cgaagtgttt tcaaacagga tgttgcgcgc cggaaccccc tgtttgagtg 2820
cgtaccgccg cccgacctcg gcttcggtca tatagccttt tttggtccgg cctcccgtaa 2880
acacgatttt gcctaccctg cggctctgat aaagtgcgat ggcatggttg atgcgttcgc 2940
ggaaaacagg agaagggcgt ttgtcccacg cggcggcgcc caacaccagc gcggcatccg 3000
cccggacata cggcggcaaa acctgcccac ccgtccgata aaccgcccaa acggatgagg 3060
caaacaccag caaaagcgga aaaacactca aacagaaacc gcccaacagg taatagcgca 3120
agccgttgcg gctgcaaaac agccgtttgt tcacaatacc gcttcgatat tttccagcgg 3180
tctgccgaca gccgccttac cgtttgccaa aacaatcgga cgctccaaca gggcgggatg 3240
atcggcgatg gcacgcagca gcgcgtcatt gtccaaattg gggttgtcca aacccaattc 3300
cttatacaaa tcatctttca cgcgcatcat cccgcgcgcc gatgccaagc ccaatttgtt 3360
gaaaatatcc ttcaattcgg acaagtcggg cggcgtatcc aaatatttga ccacttcggc 3420
agcaatgccg cgttcttcca atagggacaa ggcggcacgc gatttgctgc aacgcggatt 3480
gtggaaaatt ttgatttcag gcatgacatt tccttgcttc tcgacaatcc ccttattatc 3540
ggcttacaca gggtttttact caatatcccg cctacaaccg taccaaacgg tttacaatac 3600
ccgaatcgac atacaaagga caaaacgatg aaatacttga atcttgccgc aatcaccctt 3660
gccgccacat ttgccgcaca taccgcctcg gcagacgaac tggccggatg gaaagacaac 3720
accccgcaaa gcctgcaatc gctcaaagcc cccgtacgca tcgtcaacct ttgggcgact 3780
tggtgcggcc cgtgccgaaa agagatgcct gccatgtcca aatggtacaa agcgcagaaa 3840
aaaggcagcg tcgatatggt cggcatcgcg ctcgacacat ccgacaatat cggcaacttc 3900
ctcaaacaaa ctcctgtttc ctacccgatt tggcgttaca ccgggggcgaa cagccgaaac 3960
tttatgaaaa cctacggaaa cactgtcggc gtactgccct ttaccgtcgt cgaagcaccg 4020
aaatgcggat acaggcagac cattaccggg gaggtaaacg aaaaaagcct gaccgacgcc 4080
gtcaaactcg cccattcaaa atgccgttaa acgccggatg ccgtctgaag ccgcttcaga 4140
tggcattttt cttttccacc cgcctgccgg tgcaaactta tccactatct aaaaacaggc 4200
ggaatcttta taatcggcac tgtcttacct attgttcaga cggcatatcc ctgcggacgc 4260
aaccgcccga aacgatatgc cgcccttcct tacaggacct cctatgatcc gtttcgaaca 4320
agtttccaaa acctatcccg gcggttttga agccctgaaa aacgtcagct tccaaatcaa 4380
caaaggcgaa atgatattta tcgcgggaca ctccggttcg ggcaaatcca ccatcctcaa 4440
actgatttcg ggcattacca agccgagcag gggcaaaatc ctgtttaacg ggcaggacct 4500
cggcacattg tccgacaacc aaatcggctt tatgcgccaa cacatcggca tcgtgttcca 4560
agaccacaaa atcctctacg accgcaacgt cctgcaaaac gtcatcctgc cgcttcggat 4620
tatcggctat ccgccgcgca aagccgaaga gcgtgcccgc atcgccatcg aaaaagtcgg 4680
cctgaaagga cgagaattgg acgatcccgt aaccctctcc ggcggtgaac aacaacgcct 4740
gtgcatcgcc cgcgccgtcg ttcaccagcc cggcctgctg attgccgacg aaccctccgc 4800
caacctcgac cgcgcctacg cgctcgatat tatggaattg ttcaaaacct tccacgaagc 4860
gggaactacc gtcatcgttg ccgcacatga cgaaaccctg atggcggact acggacaccg 4920
catcctgcgc ctctcgaaag gacgactcgc atgagcatca tccactacct ctcgctgcac 4980
gtcgaatccg cgcgcaccgc gctcaagcag ctcctgcgcc aacccttcgg cacactgctt 5040
accctcatga tgctcgccgt cgcgatgacc ctgccgctgt ttatgcatct gggcatccaa 5100
agcgggcaaa gcgtgttggg caaactcaac gagtcgccgc aaatcacaat ctatatggaa 5160
acctccgccg cacaaagcga cagcgatacc gtccgcagcc tgctggcgcg cgacaaacgg 5220
ctcgacaaca tccgcttcat cggcaaagaa gacggtctgg aagaattaca gtccaatctt 5280
gaccaaaatc tgatttccat gcttgacggc aaccccctgc cggatgtctt tatcgttacc 5340
cccgacccgg caaccacgcc cgcccaaatg caggcaatct accgagacat taccaaactg 5400
cctatggtcg aatccgcgtc tatggatacc gaatgggtgc aaacgctgta ccaaatcaac 5460
```

gagttcatcc gcaaaatttt gtggtttctt tccctgacgc tggggatggc gttcgtcctt 5520
gtcgcacaca acaccatccg cctgcaaatc ctcagccgca aagaagaaat cgaaatcacc 5580
aaactcttgg gcgcgcccgc gtcgtttatc cgccgcccat tcctttatca agccatgtgg 5640
cagagcatcc tttccgccgc cgtcagcttg gggctttgcg gttggctgct ctctgccgtg 5700
cgcccattgg tcgatgccat tttcaaaccc tacggactta atatcggctg gcggttcttc 5760
tacgctggcg aactcgggct ggtgttcggc ttcgtcatcg cgttgggcgt attcggcgcg 5820
tggcttgcca ccacccagca cctgctcggc ttcaaagcca aaaaataaaa caccgtcaaa 5880
aatgccgtcc gaacccgttt tcagacggca tttcaatttg ccagtataat ggcgcatttt 5940
tccaacaagg aacctaccat gctgacctcg gaacaagtaa aagccatgat tgaaggcgtg 6000
gcaaaatgcg aacatatcga agtagaaggc gacggacacc attttttcgc cgtcatcgtt 6060
tcatcagaat ttgaaggcaa ggcacgcctc gcgcgccacc gcctgattaa agacggactc 6120
aaagcccaac tggaaagtaa cgaactgcac gcactttcca tttcggttgc cgccactccg 6180
gcggaatggg cagccaaagc acaataatcg ccacacaaaa atgccgtctg aaaccatttc 6240
gtttcagacg gcattttttt tatatcaaac cgcttacgcg ccgcgttttt ccaaagcggc 6300
tacggcaggc agctctttgc cttccaagaa ctcaaggaac gcgccgccgc cggtggagat 6360
gtagccgatt tgttcggtaa cgccgaattt ggcaatcgcc gccagcgtgt cgccgccgcc 6420
cgcaatcgag aacgctttgc tttgggcaat ggcttcggca agggctttcg taccgcctgc 6480
gaattggtca aactcgaaca cgccgaccgg cccgttccaa acgaccgtac cggcggcttt 6540
aagcaaatcg gcaagcgcgg cagcggattt cggaccgatg tccaaaatca tctcgtcttc 6600
ggcaacgtcg gcaatgtctt tcaccacagc ttccgcatcg gcggcaatgt ctttcaccac 6660
agcttccgca tcggcggcaa aggctttggc aacgacgaca tcggtcggca gcggcacaga 6720
accgcctttt gccgccattt tcgccataat ttttttggat tcttccacca aatcgtgttc 6780
cgccaaagat ttgccgatgg ctttgccttc cgccaacagg aaggtgtttg cgataccgcc 6840
gccgacgatg agttggtcga ctttgtccgc cagcgattcg aggatggtca gcttggtgga 6900
cactttgctg ccggcaacga tggcaaccat cgggcgcgcg ggctgtttca aggctttgcc 6960
caaagcgtcg agttcgcccg ccatcaatac gccggcgcag gcaacgggcg cggcttgggc 7020
gacggcttcg gtcgaggctt gggcgcggtg ggcggttccg aacgcgtcat tgacgaacac 7080
gtcgcacaaa gaagcgtagg ctttacccag ttccaaatcg tttttcttct cgcctttgtt 7140
gatgcgcacg ttttgcagca tgacgacatc gcccgcgttc agggcgggtt tgttttcacg 7200
ccagtcgttc aatactttca cgtctttgcc caacaggctg cccaagtgcg cggcaacggg 7260
ggcgacatcg tcttcggggt ggaactcgcc ttcggtcggg cggccgagat gggtcatcac 7320
gataacggac gcaccgttgt ccacgcagta tttaatggac gcgagcgagg cgcggatacg 7380
ggtgtcgtcg ctgattttgc cgtctttgaa cggtacgttc atatcggcgc ggatgaggac 7440
ggttttgccc tgcacgtttt gttcggtcag ttttaaaaat gccataatca gtccttttca 7500
atcagtgttt gcgatacgga aacaattgat gccgtctgaa ggcttcagac ggcatcgcaa 7560
cccgatcagc cggatacgcg ctcgattttc gcgccgacgc tgccgagttt tttttcaata 7620
ttttcataac cgcgatccaa gtggtaaatc tgttcgacca cggtttcgcc tcgcgccgcc 7680
aaaccggcga taacgaggct ggcggacgca cgcaaatccg tcgccttgac gactgcgccg 7740
gaaagctgtt ccacaccctg cacaaatgcc gtattgccct cggttgtgat gttcgccccc 7800
atccggttca actcggggac gtgcataaag cggttttcaa aaatcgtttc caccacgcgg 7860
cagcttccct ccgccacggc attcaatgcc ataaactgcg cctgcatatc cgtggggaag 7920
ccggggtgga cgaccgtgcg gatgtccacc gccttcggac gctgccgcat atcgatggcg 7980
atccaatcgt cgcccgcctc aatcaccgca cctgcctcaa ccagtttgtc caacaccact 8040
tccatcgttt tcggcgcggc attccgcaaa accaccctgc caccggttat cgccaccgcg 8100
cacaggaacg tccccgcctc gatccggtcg gggacgacgc tgtgttcgca gccttgcagc 8160
tcgtccaccc cttccacaat cattgtggac gtaccgatgc cgctgatttt cgcgcccatt 8220
ttgaccaggc attccgccaa atcgaccact tcaggctcaa tggcgcagtt ttccaaaacc 8280
gtcgtacctt ccgccagcgt cgccgccatc agcaggtttt ccgtgccgcc gacggtaacg 8340
acatccatcg ccacgcgcgt acctttgagt ttgcctttgg ctttgacgta accgtgttcg 8400
ataacaatct cagcacccat cgcttccaag cctttcaaat gctgatcgac ggggcgcgaa 8460
ccgatggcgc agccgcccgg caggctgact tgcgcctcgc cgaaacgcgc cagcgtcggg 8520
cccagcacca aaatcgaagc gcgcatcgtt cggaccaact cgtaaggggc gcaggtattg 8580
tttaccgtac cgccgttgat ttcaaattcg ctgatattgt cggtcaggac gcgcgcgccc 8640
atcccctgaa gcagcttttg cgtggttttc acatctgcca gcatagggac gtttttcagg 8700
cgcaacgtac ccgatgtcag caaacccgcg cacatcagcg gcaatgccgc gtttttcgcg 8760
cccgagaccg ttatttcccc gttgagcggg ccgtttgcgg agattttcag tttgtccacg 8820
tttgttcttt cctggtgggt acttgtatag tgaattaaca aaatcggga caaggcggcg 8880
aagccgcaga cagtacagat agtacagaac cgattcactt ggtgcttcag caccttagag 8940
aatcgttctc tttgagctaa ggcgaggcaa taccgtactg gtttttgtta atccactata 9000
atatttcaat tctcgggaca acgcataaag catcacccga tgaaggttgc agaggcggaa 9060
ttataaggga ttttcgggaa aaatacggaa gccgcaccaa agaatttgac gaaatgccgc 9120

gctttccgaa caaggattgt cggaagacaa aaaagccgag ttttgaaaac tcagcttttt 9180
tgctttatct ggtgggtcgt gagcgattcg aacgctcgac caacggatta aaagtccgct 9240
gctctaccgg ctgagctaac gacccgataa gtttggaatt ttacagaccg gccgaaaccc 9300
tgtcaagccc cttgcgggcg gacgggcgtt atatccgctt atcggcctgt ttttttcgta 9360
taaaccaaag aagtcaacac cgatgcaccc aatgcgccga acacgaccga cagcgaaacg 9420
gaaatcggga tatgcaccca atgcattacc agcattttca caccgataaa acccaacacg 9480
aatgccaatc catatttcag gaagataaag cgttccgcca catccgccag caggaaatac 9540
atcgcccgca agcccagaat tgcgaaaata ttggaagtca gcacgataaa cggatcggtg 9600
gtaacggcaa agacggcggg gatgctgtcc acggcaaaca cgacatcgct caattcaatc 9660
atgaccagca ccaaaaacag cggcgtggcg attttttgc cgttttcgac ggtaaaaaat 9720
ttctcgccgt gaaattccgt gccgaccgga acgactttct tgacggtatt cagcagcctg 9780
ctgtttgcca aatcctcttt ctcatcgcct tcgggcttca tcatgtgtat accagtatag 9840
agcaggaacg cgccaaacag atacagaatc cactcaaact gctgaaccag tgccgcgccg 9900
acgaaaatca tgacggtgcg caataccaat gcgcccaata cgccgtacag cagcacgcgg 9960
tgctgaaact gtggtgcgac tttgaagtag ccgaatatca tcaggaacac gaaaatattg 10020
tcgactgcca acgatttttc caaaatgtag ccggtaaaga attccaatac tttttctttt 10080
gcgactgccg cgccgtagcc gggattgccg gcgagttcaa aatacagcca gcccgcgaac 10140
aggcaggata cggcaaccca caagccgctc catgccaagg cttctttgac gccgacttta 10200
tggctgccgt ttttcttcag cgaaaacata tccaaggcaa tcatgaccag cactgccgca 10260
aaaaaaacgc cgtaaaacaa cggcgacccg atgccgggat attctgtcat ggttcaatct 10320
cctgatttga aatgtaattg tgttaccagc tgatataaaa catcgctttt gccaaaaaga 10380
caatcagcag catatgggta aagacgacgg cgtgtatgta tttcgaccaa ccgaccgtca 10440
gtgtggaacg cgccattttg acgacggcga tggcgaagtg cgccaatacg ctgaacgcca 10500
acaggatttt cagcgtcagc atcgtaccga aggaagtggc aaacggttcg cccaatatag 10560
aaagatagcg gtttgccgcc atcacgatgc cgctggcgaa cagcagtccg accacaaacg 10620
gcatcaccct gacggcgcgg taagacattg ccttttccac ttcgcgccgc gcctcgcgcg 10680
acacccgtcc cgtatgcagg acggacaaaa ccagcacttc aaaaaacacg ccgccgacaa 10740
aggcaatagc gcaatacaga tgaacgatgt gcgcgacggc ataaatactc atacgatgct 10800
ccaaacggaa aactcggata cggattgtat cactatcgcc cccgatatcc gcataccgct 10860
tcccgcaccg cctcggcgat tctcgcgccc gctccgcgat gttgtgcgat aaagccgtcc 10920
acgcgcgcct gcatctgcat cccccccccc tcggacgata aggtttttc aacggcttcc 10980
cgccacgcat ccgccgattc gacttgaacc gccgcacccg atgccaaggc gtgtcggcag 11040
gcttcggaaa aattgtaggt tgaaaagccg aatatcgtcg gaacgccgca ggaaagcggt 11100
tcgatgatgt tctgacaacc cgaatcgacc agactgccgc cgacaaaagc gacatcggcg 11160
cacaggtaat acgcatacag ctcgcccata ctgtcgccta tccacacctg cgtatcaggt 11220
tcgaccggca aaccgtcgct gcgccgctga accttaaacc cgaagcgttt tgccgtttca 11280
aataccgtct gaaaatgctc gggatggcgc ggcacgacga ccagcagcgc atcgccgcga 11340
tattgttgcc acgccgccag cagtttttcc gcctcgtctt caccccgata aacgcgcgtg 11400
ctgccgcaca cggcaaccgg ccggcctccg atgcgttttt caaactgccc cgccagcgtt 11460
ttcatctgtt ccgacggtat gatgtcgtat ttggtattgc cgcacacctg cacggatgcc 11520
gcgcccaatt tcgccaaccg cgccgcatcc gcctctgtct gcgccagaca ccccgtcagc 11580
gaagcggcgg caggacggat caggcggcgg actttcagat aaccgttcaa cgatttttcc 11640
gacagccgcg cattcgccaa aaacagcggc acacccgcgc gccggcattc cctcatcagg 11700
ttgggccaga tttcggtttc catcaaaatg ccgaacatcg ggcggtgttc gcgcaaaaac 11760
tgccgtaccc acgttttttt gtcatacgga agatagcggc attgcgcatc gggaaacaga 11820
acttgcgcgg tttcccgccc cgtcggggtc atctgcgtca tcagcagcgg cgcatcggga 11880
aaacgccgcc gcaactcgcg tatcaaggac tgggcggcac gcgtttctcc gaccgaaacg 11940
gcgtgtatcc aaaccgcgcc ggtaacggga ttcggatacg gcttgccgaa acgctcgtcc 12000
cgatgcgccc gatatgccgg ggcacttccg gagcgtttgt ccaaataacg ccgtatccat 12060
atcggcgcaa gcagccacaa tacatcataa agccattgga acatctttct atttcctgca 12120
aaacaaatgc cgtctgaacg gttcagacgg catttcggca acggaatcaa atatcgtagg 12180
ttgtcgaagc ggtatctccg cccttgcccg tccagttggt atggaaaaac tcaccgcgcg 12240
gtttgtcggt gcgctcgtaa gtgtgcgcgc cgaagtagtc gcgctgtgcc tgcaagaggt 12300
tggcaggcag acgttcggtc gtgtagccgt ccaagaacgt aatcgccgaa gccatgcagg 12360
gcatagggat gccgcattcg accgccttgg caaccacctt gcgccacgcc ggcaggcagt 12420
tttccaaaat atttttgaaa tacggatccg cacccaagaa caccaaatcg ggattgtttt 12480
catacgcgtc gcggatattg cttaagaatg cgctgcgaat gatgcacccc tcgcgccaca 12540
gcagcgcagt gttgccgtag tccaaatccc agccgtagct ttcgcccgct tcgcggatca 12600
gcataaagcc ttgtgcgtag gaaatgattt tagatgcaag cagggcctgt ctcaacgcct 12660
cgacccattc ttgtttgccg ccttcgacgg gcgtaacggt tcgggcgaac agtttgccgg 12720
tctgcacgcg ctgttctttg aacgacgaaa cgcagcgggc gaatacggct tcggaaatca 12780

gcgtcagcgg aatacccaaa tccaaagcat tgatgcccgt ccatttgcct gtaccttttt 12840
gccctgccgt atcgaggatt ttctcgacca gcggttcgcc gccttcgtcc ttatagccca 12900
aaattgccgc tgtgatttca atcagataag aatccagctc ggttttgttc cactcggcaa 12960
acacgcggta catttcgtcg taagacagcc ccaagccgtc tttcatgaac tggtacgctt 13020
cgcaaatcaa ctgcatatcg ccatattcga tgccgttatg caccattttg acaaaatgcc 13080
ccgcaccgtc tttgccgacc cagtcgcaac acggttcgcc ctgcgacgtt ttggcggcaa 13140
tcgcctgaaa aatcggcttg accgcatccc aagcgcgctt atccccgccc ggcataatgg 13200
acggcccgcg ccgcgcccct tcttccccgc cggacacgcc cgcgccgaca aacaaaatcc 13260
ctttttcagc aaggtaatgt gtccgccgtg tcgtgtcggg gtaattggca ttgccgccgt 13320
cgataaggat gtcgccttct tccaacagcg gaagcagttg ttcgataaat tcgtcaacca 13380
ccgaaccggc acgaaccatc atcataattt ttcgcggttt ttccagctta tcgaccaaat 13440
cttgcaaaga atacgcgccg ataatattag ttccttttgc cgcgccgttt aaaaattcgt 13500
ccaccttggc agtcgtgcgg ttgtaggcaa ccaccttaaa tccgcaatcg ttcatattca 13560
aaatcaggtt ttgccccata accgccaaac cgattacacc aatatcgccg ttcattgcag 13620
gaagctccgt tatagattta atttatcgac cgcaactcta cccgatttac acttgtttaa 13680
caatccttaa ctttttaatt ttttgaaaag atgcctttac gctttgctgt accgttttgc 13740
tgaagggtta taaataaaat ataaaattta aataataaaa cgatgattat attgatagga 13800
gaaattttct gtgggtaact ttttttttatt ttaaaaatca tcaggatttc tttttttttag 13860
ggtgtcggta aggcggattc ccttttgtgc atacctgtgg attgtttttc atgaagaata 13920
gtttttgtgg acagtttgct tgttgtgcaa atggcatcct actttcttt accgaatggc 13980
tgccgatgtc tttaagaacc ggaatactgt ggaggtttga gaggaaagtg tgtttggaac 14040
ttgtggaaat ggtcaggtgt cggcacgaat gtcttatttc tgcatatcgg cagagtgcgc 14100
atccgaattt gtgtataagt ggtggaaaaa atgagatttg cgggtaaatc tcacaatatt 14160
tcagtcagat aactttggat tgcttgtgta taagtaaact ttcggatggg gatacgtaac 14220
ggaaacctgt accgcgtcat tcccacgaac ctacattccg tcattcccac gaaagtggga 14280
atgatgaaat tttgagtttt aggaatttat cgggagcaac agaaaccgct ccgccgtcat 14340
tcccgcgcag gcgggaatct agaacgtaaa atctaaagaa accgtgttgt aacggcagac 14400
cgatgccgtc attcccgcgc aggcgggaat ctagaccatt ggacagcggc aatattcaaa 14460
gattatctga aagtccgaga ttctggattc ccactttcgt gggaatgacg ggatttgaga 14520
ttgcggcatt tatcggaaaa aacagaaacc gctccgccgt cattcccgcg caggcgggaa 14580
tccagacctt agaacaacag caatattcaa aggttatctg aaagtccgag attctggatt 14640
cccactttcg tgggaatgac gggattttag gtttctgatt ttggttttct gtttttgtgg 14700
gaatgatgaa attttgagtt ttaggaattt accggaaaaa acagaaaccg ctccgccgtc 14760
attcccgcgc aggcgggaat ccagacctta gaataacagc aatattcaaa gattatctga 14820
aagtccggga ttctagattc ccactttcgt gggaatgacg gcatcagtct gccgtttaca 14880
gcacggtttc tttagatttt acgttctaga ttcccgcctg cgcgggaatg acgaatccat 14940
ccatacgaaa acctgcacca cgtcattccc acgaacctac atcccgtcat tcccacaaaa 15000
acagaaacct caaatcccgt cattcccgcg caggcgggaa tctagacttg tcggtgcgga 15060
cgcttatcgg ataaaacggt ttcttgagat tccgcgtcct ggattcccac tttcgcggga 15120
atgacgaatt ttaggtttct gttttggttt tttgtccttg taggaatgat gaaaatttaa 15180
gttttaggaa tttaccggaa aaaatagaaa gcgttatcca caagttctga tgttcagctc 15240
gtgaaatgcg tcgggcaaat catcgctgtc ggcaaattcc acccggtcgt aagccgtttc 15300
gtctgccaaa accgcgcgca agagtgcgtt gttgatggcg tgtcccgatt tgtagccttc 15360
aaatgcgccg acaatcggat gtccgacgat atacaaatca ccgatggcat caaggatttt 15420
gtggcgcaca aactcatcgg gatagcgcaa gccttcagga ttcaggacat ccgtgtcgtc 15480
aatcacgatg gcgttgttca aattgccgcc caaacccaga ttgtgggcgc gcatcatttc 15540
cacttcgtgc ataaagccga aagtgcgcgc gcgcgcgatt tcgtcgatgt aggatttgcc 15600
ggcgaaatcg atttcaaaag tgggcgagct gcggttgaaa accggatggt cgaattcgat 15660
ggtcagcgtt accttaaagc cgtcatacgg cgtaaagcgc acccatttgc ccgcttcttt 15720
gatttcgaca ggcttgagga ttttcaaaaa acgcttttgc gccttttgat cgaccacgcc 15780
cgcatcttgc aaaaggtaaa taaacggcag gctggagccg tccataatcg ggatttcggg 15840
cgcgttcagc tcaatcagcg cattgtcgat gccgtaggcg gacagcgcgg acataatgtg 15900
ttcgatcgtg ccgacgcgca cgcctttgtc ggtaacgatg gtggaggaaa ggcgggtatc 15960
gttgatcaaa taaggggtca gcttgatttg ttcgcccatc tcgccgtcca aatcggtacg 16020
gcggaaggaa atcccgctgt tttcaggcgc ggggtgcagg gtcagcgcga cgcgttcgcc 16080
cgaatgcagc ccgacgccgg taacgctgat ggatttcgcc aaagttcttt gcagcataaa 16140
ccgcttcctt atcaaggggg taagttttgg aataatacga taaaaccgga aaaacaggct 16200
atgtttttcc atagtatttg ccaatgtatc cgttttcaat acgtaagccg cataaaaatg 16260
tatagtggat taacaaaaat caagacaagg cgacgaaccc acccccctcc tgaaaaacgc 16320
aaaaaatgcc gtccgaaaac ctttcggacg gcattttcgc gtaaaccgtc attcccacaa 16380
ggacaaaaaa ccaaaacaga aaccaaaaa cagcaaccta aaattcgtca ttcccgcgca 16440

ggcgggaatt tggaatttca atgcctcaag aatttatcgg aaaaaaccaa aacccttccg 16500
ccgtcattcc cacgaaagtg ggaatctaga aatgaaaagc agcaggcatt tatcggaaat 16560
gaccgaaact gaacggactg gattcccgct tttgcgggaa tgacggcgac agggttgctg 16620
ttatagtgga tgaacaaaaa ccagtacggc gttgcctcgg cttagctcaa agagaacgat 16680
tctctaaggt gctgaagcac caagtgaatc ggttccgtac tatttgtact gtctgcggct 16740
tcgtcgcctt gtcctgattt ttgttaatcc actatatcta gccgaattac tttatttttt 16800
gatacgtaac cggccggttg ccgtcattcc cgcgcaggcg ggaatctaga cattcaatgc 16860
taaggcaatt tatcgggaat gactgaaact caaaaagctg gattcccact ttcgtgggaa 16920
tgacgcggtg caggtttccg tacggatagc ttcgtcattc ccgagtaggc gggaatctag 16980
tccgcttgtt cggtaaatga gagggcggat tgcgcgcctg tcagataaac cacgtgttta 17040
aacgggcggc aatgaggtac gcgcagagcc ttgaagcgca atcgatatat tattttcagc 17100
caaaacggac gcccccgctt gccttgcaaa cctttaaaaa ggaagccacc cggattaatc 17160
cgagtggccg tggaaaatca cttaccgctt gatttattta aaatttatgg tataatttac 17220
cttagctggc atcacttgcg tcgcggcagg ttgacggcag gtgcttggtg tcaatcttct 17280
taccgttggc ggcggcggcg gcggtaacgt cgtcgttggc ggctttggct ttgtcgcgcg 17340
taaccggctg tccgcagaac cattttaccg aaccgttttg acgcttggcc cacagggaga 17400
gttttttgcc tttgatttcg ttgtttacgt tgcttgaagc catttgggcg gtaacgacgc 17460
cgtttttgac ttcaacgctt ttaacatatt tgcctttgat ttcagaggag gttgccacgc 17520
cggcagaagt gttgttgccg ggccattcgc cgtgattcag gtaatactcg gtaacggctg 17580
atttttgacc ttcggccaaa agaatggctt cggaaacttg tgcgcgggct gtgtagtctt 17640
gataagcagg aagggcgact gccgccaaaa tgccgacgat ggcaatcaca atcatcagct 17700
cgataagggt aaaacctttt tgaagggtgt tcataaaatt actcctaatt ggaaaggaaa 17760
tgcctcaagc ttacgccatc ggcattatgc aatgtatttg accatcggta ttttgttgcg 17820
atacctgtgt attataaagc aagattggta ccaagtttgt attttgaggt gaaaatttat 17880
gcgtttatct ctatgtaatt gtttttattt tacattttct ttcgtttggc gtggtttgag 17940
taattagggg gttgccgttt tttgtcagca gtgttgaaaa ttgtcagttt tagtgccgat 18000
tttcggcact tttttattgg cgtgggggtat ctctattggc atggggcatc gggtgtgttg 18060
attgggtcgg aatttgagat tttgaatttt gcgcggtagc atagggtggg ttgggtggga 18120
aattttaaat ttaattttta aaaatttccg ttttcttgga aagtgattga aatcggcgcg 18180
tggtgttcct gtgcaaccgg cagttgaatc atcgcggcag gtttccgtgc ggatggcttc 18240
gtcattcccg cgcaggcggg aatccagcct tgttggtacg gaaacttatc gggaaaacgg 18300
tttcttgaga ttttacgttc tggattccca ctttcgcggg aatgacgcgg tgcaggtttc 18360
cgtatggata gcttcgtcat tcccgcgcag gcgggaatcc aggtctgtcg gcacggaaac 18420
ttatcgggta aaaaggtttc ttgagatttt tcgtcctgga ttcccacttt cgtgggaatg 18480
acgggatgta ggttcgtggg aatgacggtt taggtatttt tatagaaagc cgtaggtggt 18540
gtttctatgc aaacgacaga tgaatcatcg cggcaggttg acggcaggtg cttggtgtcg 18600
atttgtcggg tgccggtggc ggcggcggta acggcgtcgt ctttggcgtt gtcggcgcgc 18660
gtaaccggca gtccgcagaa ccattttacc gaaccggctt gacgcttggc ccacagggag 18720
agttttttgc ctttgatttc gttgtttacg ttgcttgaag ccatttgggc ggtaacgacg 18780
ccgtttttga cttcaacgct tttaacatat ttgcctttga tgtcggcgga ggttgccacg 18840
ccggcagaac tgttgttgcc gggccattcg ccgtgattca ggtaatactc tgtgacggct 18900
gatttttgac cttcagccaa aagaatggct tcgtcattcc cgcgcaggcg ggaatctagg 18960
tctgtcggca cggaaactta tcgggaaaac agtttcttga gattttgcgt tctggattcc 19020
cgctttcgcg ggaatgacgg gattaaagtt tcaaaattta ttctaaataa ctgaaattca 19080
acgaactaga ttcccacttt cgtgggaatg acgaatttta ggttgctgtt tttgtgggaa 19140
tgatgaaatt ttaagtttta ggaatttatc gaaaaaacag aaaccgctcc gccgtcattc 19200
ccgcgcaggc gggaatccag cctcgtcggt acggaaactt atcgggtaaa aaggtttctc 19260
tagtttggtg tcgattttct tgtcgatgct gttgacggca ggtgcttggt gtcgatctgc 19320
ttgccgttgg cggcggtgtc ggctttgacg gcgtcggcgc tggcgttgtc gcgcttaacc 19380
ggctgtccgt agaaccattt taccgaaccg tcttgacgct tggcccacag ggagagtttt 19440
ttgccttgga tttctttgtt tacgccgctt gaaagcattg tggcggtaac gacgccgttt 19500
ttgacttcaa ctttctcaac atatttgcct ttgatgttgg cggaggttgc cacgccggca 19560
gaactgttgt tgccgggcca ttcgccgtga ttcaggtaat actcggtgac ggctgatttt 19620
tgaccttcag ccaaaagaat ggcttcgtca ttcccgcgca ggcgggaatc tagaccttag 19680
aacaacagca atattcaaag attatctgaa agtccgggat tctagattcc cactttcgtg 19740
ggaatgacga attttaggtt gctgtttttg gttttctgtt tttgagggaa tgatgaaatt 19800
ttaagtttta ggaatttatc agaaaaaaca gaaaccgctc cgccgtcatt cccgcgcagg 19860
cgggaatcca ggtctgtcgg tacggaaact tatcgggtaa aacggtttct ctagtttggt 19920
gtcgattttc ttgtcggtgc tgttgacggc aggtgcttgg tgttgatgtt ggcggtgccc 19980
ttgccggtgg cggcggtgac ggcgtcgtct ttggctttgt cgcgcgtaac cggctgtccg 20040
cagaaccatt ttaccgaacc gttttgacgc ttggcccaca gggagagttt tttgcctttg 20100

atttcgttgt ttacgttgct tgaagccatt tgggcggtaa cgacgccgtt tttgacttca 20160
acgcttttaa catatttgcc tttgatttca gaggaggttg ccacgccggc agaactgttg 20220
tcgccgggcc attcgccgtg attcaggtaa tactcggtaa cggctgattt ttgaccttcg 20280
accaaaagga tagcttcgtc attcccgcgc aggcgggaat ccagccttgt cggtacggaa 20340
acttatcggg taaaacggtt tctttagatt ttgcgttctg gattcccact ttcgtgggaa 20400
tgacgggatt aaagtttcaa aatttattct aaataactga aactcaacga actagattcc 20460
cgcttttgcg ggaatgacga attttaggtt tctgttttgg gttttctgtt tttgagggaa 20520
tgatgaaatt ttaggtttct gtttttggtt ttctgtcctt gtgggaatga tgaaatttta 20580
agttttagga atttatcgga aaaaacagaa accgctccgc cgtcattccc gcgcaggcgg 20640
gaatccagcc tcgtcggtgc ggaaacttat cgggaaaacg gtttctttag attttacgtt 20700
ctggattcct actttcgtgg gaaagacgaa ttttaggttt ctgtttttgg ttttctgtcc 20760
ttgtgggaat gatgaaaatt taagttttag gaatttatcg gaaaaaacag aaaccgctct 20820
gccgtcattc ccgcaaaagc gggaatccag cctcgtcggt gcggaaactt atcgggtaaa 20880
aaggtttctt tagtttggtg tcgattttgt cggtgccggt ggcggcggca acgtcgtctt 20940
tggcgttgtc ggcgcgcgta accggctgtc cgcagaacca ttttaccgaa ccggcttgac 21000
gcttggccca cagggagagt tttttgcctt tgatttcgtt gtttacgccg gttgaaagca 21060
ttgtggcggt aacgacgccg tttttgactt caacttcctt aacatatttg cctttgattg 21120
ttgaagaaga tgccacgccg gcggcatcat taaatcccgt cattcccact ttcgtgggaa 21180
tgacgggatt aaagtttcaa aatttattct aaataactga aactcaacga actagattcc 21240
cgcttttgcg ggaatgacga attttaggtt gctgtttttg gttttctgtc cttgcgggaa 21300
tgatgaaatt ttaagtttta ggaatttatc gaaaaaacag aaaccgctcc gccgtcattc 21360
ccgcgcaggc gggaatccag cctcgtcggt gcggaaactt atcgggaaaa cggtttcttg 21420
agattttgcg ttctggattc ccgctttcgt gggaatgacg gtttaggtat ttttatagaa 21480
agccgtaggt ggtgtttcta tgcaaacgac agatgaagcg tcgcggcagg ttgacggcag 21540
gtgcttggtg ttgatgttgt cggcggtctt ggcggcggcg gcgacggtgt cggctttggc 21600
gtcggtgcgc gtaaccggct gtccgcagaa ccattttacc gaaccgtctt gacgcttggc 21660
ccacagggag agtttttttgc cttggatttc tttgtttacg ccgcttgaaa gcattgtggc 21720
ggtaatgacg ccgtttgcga ctgtaacttc cttaacatat ttgcctttga ttgttgaaga 21780
agatgccacg ccggcagaag tgttgttgcc gggccattcg ccgtgattca ggtaatactc 21840
tgtgacggct gatttttgac cttcggccaa aaggatagct tcgtcattcc cgcgcaggcg 21900
ggaatccagg tctgtcggta cggaaactta tcgggtaaaa cggtttcttt agattttgcg 21960
ttctggattc ccactttcgc gggaatgacg ggattaaagt ttcaaaattt attctaaata 22020
actgaaacca acgaactaga ttcccacttt tgcgggaatg acgaagtttt tctgccattt 22080
gccgtgattc gggcaatact cggtaacggc tgatttttttg aaagtgtttg aaatcggcgc 22140
gtggtgtttc tatgcaaccg gtagatgaat catcgcggca ggttgacggc aggtgcttgg 22200
tgttgatttt gtcgtcggtc ttgccgttgg cggcggcgac gtcggtggcg gtggcggtgg 22260
cggtgtcgtt gcgcgtaacc ggctgtccgc agaaccattt gaccgaaccg ttttgacgct 22320
tggcccacag ggagagtttt ttgcctttga tttctttgtt tacgccgctt gaaagcattg 22380
tggcggtaac gacgccgttt ttgacttcaa ctttctcaac atatttgcct ttgatgtcgg 22440
aggaggatgc cacgccggcg gcatcattaa atcccgtcat tcccgcaaaa gcgggaatct 22500
agaactcagg accggagaaa cctttttacc cgataagttt ccgtgccgac agacctagat 22560
tcccgcctgc gtgggaatga tgggattaaa gtttcaaaat ttattctaaa taactgaaac 22620
tcaacgaact agattcccgc ttttgcggga atgacgaatt ttaggtttct gtttgtgggt 22680
ttctgttctt gtgggaatga tgaaatttta agttttagga atttatcgga aaaaacagaa 22740
accgctccgc cgtcattccc gcgcaggcgg gaatccagcc ttgtcggtac ggaaacttat 22800
cgggtaaaaa ggtttctcta gtttggtgtc gattttcttg tcggtgctgt tgacggcagg 22860
tgcttggtgt tgattttgtc ggtgtcgggt gtggcggcgg tgacttcgtc ggtgccggct 22920
ttggcgttgg cggcgttgcg cgtaaccggc tgtccgcaga accattttac cgaaccgtct 22980
tgacgcttgg cccacaggga gagtttttttg ccttggattt ctttgtttac gccgcttgaa 23040
agcattgtgg cggtaatgac gccgtttgcg actgtaactt ccttaacata tttgcctttg 23100
attgttgaag aagatgccac gccggcagaa gtgttgtttt tcggccattc gccgtgattc 23160
gggtaatact cgggtgtttt tgtgcaaacg gcagatgctg cgtcgcggca ggttgacggc 23220
aggtgcttgg tgttggtttt cttgttgccg gtgttgtcgg cggcgacggt gtcgtcggtg 23280
ccggcgcgcg taaccggctg tccgcagaac cattttaccg aaccgttttg acgcttggcc 23340
cacagggaga gtttttttgcc ttggatttct ttgtttacgc cgcttgaaag cattgtggcg 23400
gtaacgacgc cgtttgcgac tgtaacttcc ttaacatatt ttcctttgat tttagaggag 23460
gatgccacgc cggcggcatc attaaatccc gtcattccca cgaaagtggg aatctagaac 23520
tcaggaccgg agaaaccttt ttacccgata agtttccgtg ccgacagacc tggattcccg 23580
cctgcgcggg aatgacgaag tttttcggcc attcgccgtg attcgggcaa tactcgggtg 23640
ttttgtgcaa acggcagatg ctgcgtcgcg gcaggttgac ggcaggtgct tggtgtcaat 23700
cttcttaccg ttggcggcgg cggcggcggt aacgtcgtcg ttggcggctt tggcgttgtc 23760

gcgctcaacc ggctgtccgc agaaccattt taccgaaccg gcttgacgct tggcccacag 23820
ggagagtttt ctgcctttga tttctttgtt tacgccgctt gaagccatta tgtcagacgg 23880
tattgcccgg gcagctttat tcgtacactt tcagcagctc gacttcaaat atcaaagtgg 23940
cgtgcggggg aatcacgccg cccgcgccgt gtgcgccgta gcccatttcc gaagggatgg 24000
tcagcttgcg tttgccgcct tccttcatgc cgccgaagcc ttcgtcccag cctttgatga 24060
cttgtccgac accgagcgtg atggtcagcg gctggcggcg gtcgaggctg gagtcgaatt 24120
tggttccgtt ttccagccaa cctgtgtaat gcacggtaat ctctttgcct ttaactgctt 24180
cttttccgaa gccttcttgc aagtcttcaa taatcaggcc gcccatattt gtcctttcgt 24240
tgcttgttgg tcaaaacggc aagggtaaca taccgtccgt cgaagtcaaa tgccgctcaa 24300
acgtcagctg catcggtgca gctgaaacgg ctgtgtttgt ttgactgttt tatttttttc 24360
gtaaaggttc catgcttttt catggaaata gaaaacgacg gtgttgatta ggggttcgac 24420
cagcgcaact gctcccgata cgcctatact gcccgtcagt acataggtta cactgaaggc 24480
gacgctgaaa tgcagtgcgg caaaagtcag ggttttaagc atcatcctct cccggattgg 24540
acattgacgg agagatgata aagattatca taaggctgcg cggtttaaat ttgctatttg 24600
ttgttagtgt agataaatcg ttttttaaat aaggatagga attatgaatc ataaaaagat 24660
cgttgttttg gatgcggata ctttgcccgg ccgggttttt cattttgatt ttccgcacga 24720
gcttgcggtt tacggtacga caggtgcgga tgaaacggca gaacgggtgc gcgatgcaca 24780
tattgtcatt actaacaaag tgatgatttc tgccgatatt attgcggcta atccgcagtt 24840
ggagctgatt gccgtcagtg cgaccggcgt gaacaatgtc gatattgggg cggcgaaggc 24900
ggccggtgtt gcggtatgca atgtccgcgc atacggaaac gaatcggttg cggaacacgc 24960
ctttatgctg atgattgcgt taatgcggaa tttgcctgcc tatcagcgtg atgttgcggc 25020
aggattgtgg gaaaagtcgc cgtttttctg ccattacggc gcgccgattc gggatttgaa 25080
cggcaaaacg ctggcggttt tcggacgcgg caatatcgga cggacgcttg ccggatacgc 25140
gcaggcattc ggtatggggg tggtgtttgc cgaacacaaa cacgcgtccg ctgtgcgtga 25200
aggctatgtt tcctttgaag atgcggtacg ggctgctgat gtgttgtcgc tgcactgtcc 25260
gctaaacgcc caaactgaaa atatgatagg cgaaaacgaa ttgcggcaga tgaagcctgg 25320
cgcggtttta atcaattgtg ggcgcggcgg gctggtggat gaaaacgcgc tgcttgccgc 25380
actcaaatac gggcagatcg gtggggcagg tgtcgatgtt ttgacgaatg agccgcccaa 25440
aaacggcaat cccttgctga atgcacgatt acccaatctg attgttacgc cgcataccgc 25500
gtgggcaagt cgtgaggctt tggacaggct gtttgatata ttgttggcga acattcacgc 25560
ctttgtgaaa ggagaggcgc aaaaccgcgt ggtttgaacc tgtcgggatt gcggaaaaaa 25620
atgccgtctg aacgcctcaa gggttcagac ggcatttctt gagattcccg tttaaccgac 25680
tttgtcgccc ggctgcgcgc ctgtatccac atccaagagc ttcagtttcc cgtctgccgt 25740
ggcggcactc aaaatcatgc cttcagatac accgaatttt gccattttgc gcggggcgaa 25800
gttggcgacg gcgatgacca tgcggccgtt caattcggca gggttcgggt aagacgcggc 25860
gatgccggag aagatgatgc gtttttcaaa accgaaatcg aggtcgaatt tcaaaagttt 25920
ggtgctgcct tcgacagctt cgcagttcaa tactttggca acgcgcatgt cgattttcat 25980
aaagtcgtcg aaactcgcct gttcggcgac tttttcgtat ttgccctctt cggcggcagg 26040
tgcggctgcg gcggcgatgc tttgtttgtt ggcttcgatt aaatcgtcca cttgtttttg 26100
ctccactcgt tgcattaaat gttcgtattt gttgatggcg tgtttgccca aggtatcgcg 26160
tgtatttgcc caagtgatgg cttccaaatt caggaatttg gcggcgtttg cggcggtttg 26220
cggcaagacg ggggcgaggt aggcggtcaa catggtgaag gcgttgatga gttcgctgca 26280
tacttcgtgc aggcgttcgt cttggccttc ttgtttggcg agttcccacg gcttgttggc 26340
atcaacgtat tcgttgacaa tgtctgccaa ggccatgatg tcgcgcaggg ctttggcgta 26400
ttcgcggctt tcgtagcatt cggcaatggc ttcgctttgc gcagtcagtt ttgccagcaa 26460
ttcgctgtcg gcaacatctt tcagacggcc ttcaaagcgt ttggcgatga aacctgaggc 26520
gcgggcggcg atgttgacgt atttgccgac gaggtcgctg tttacgcggc tgataaagtc 26580
ttgcaggttc aaatcgatgt cttcgatttt gctgttgagt ttggcggcga tgtagtagcg 26640
catccactcg ggttcaggc cttgttccag ataggatttg gcggtaataa acgtgccgcg 26700
cgatttggac attttttgtc cgtcgacggt caaaaagccg tgtgcgtaca cgccggtcgg 26760
ggcgcggtgg ccggagaaat gcagcatagc gggccagaac agggcgtgga aatagagaat 26820
atctttgccg atgaagtggt acatctcggt ttggctgtcg gctttgaagt attcgtcaaa 26880
atcgacgccg atgcggtcgc acaggttttt aaacgacgcc atgtagccga cgggcgcgtc 26940
cagccagacg tagaagtatt tgcccggcgc gtcggggatt tcaaaaccga aatacggcgc 27000
gtcgcgggaa atatcccagt cggacagggt ggtttcttca ccttcgccca gccattcttt 27060
cattttgttg agggcttcgg cttgcagatg gggcttgccg tcgtgcgggt tgttgccgga 27120
agtccatgct ttgaggaagt cggcgcattc gcccagtttg aagaagaagt gttcggattc 27180
gcgcaattcg ggtttcgtac cggaaacggc ggaatacggg ttaatcagtt cggtcgggga 27240
ataggtcgtg ccgcagactt cgcagttgtc gccgtattgg tcttgggcgt ggcatttcgg 27300
gcattcgcct ttgacgaagc ggtcgggcag gaacatttgt ttttcggggt cgaaaagctg 27360
ctcgatgacg cggctctcaa tcttgccgtt ggctttcagc gcgcggtaaa tgtcttggga 27420

aaactgtttg ttttcagggg aatgggtgct gtaataattg tcgtaaccga tgaaaaagcc 27480
agtaaagtcg gcgaggtgct cttcgcgcac tttggcaatc atgtcttcgg gcgcgatacc 27540
ttgtttttgc gcggcaagca ttacgggcgt gccgtgggtg tcgtcggcgc agcagtagtg 27600
gcacgcgtgg ccgcgcagtt tttgaaagcg cacccaaacg tcggtttgga tgtgttcgac 27660
catgtggccg aggtggatgc tgccgttggc atagggcagg gcggaggtaa ctaagatttt 27720
gcgtgtcata ttgtgctttg caaacaatgg gtaaaggcgg attataccgc aaatcaaacg 27780
gggaaatgcc gtctgaagcc tgaaaaatcg ggcttcagac ggcatttttg ccaaccggcg 27840
ggagttattc gacggttacg gatttcgcca ggttgcgcgg cttgtccaca tcggtaccgc 27900
gtgcgagggc ggtgtggtag gcgaggagct gcacggggat agtatgcacg acgggggaca 27960
gtttgccgac gtggcgcggt gcgcggataa cgtgcacacc ttcggtggca ttaaaattgc 28020
tgtcgaggtc ggcaaagacg aaaagttcgc cgccgcgcgc gccgacttcc tgcatattgg 28080
ctttgacttt gtccaacagg ctgtcgttgg gtgcgatgac gacgacgggc atattttcgt 28140
ccaccagggc aagcggcccg tgcttcagtt cgccggcagg ataggcttcg gcgtggatgt 28200
aggtgatttc cttcagcttc aacgcacctt cgagggcaat cgggtaatgg atgccgcgcc 28260
ctaaaaacag cgcgctggtt ttcttggcaa actgttgcgc ccatgcggca atttgaggtt 28320
cgaggttcag agcgtgctgc acgctgccgg gaagctggcg gagttcttcg gtgtaacgcg 28380
cttcgtcttc ttcggaaacc aaaccgcgca ctttcgccag cgttaccgcc aaaccgaaca 28440
gcgcaaccag ttgcgtggta aacgctttgg tcgaggcgac gccgatttcc gcaccggcac 28500
gggtataaag cacgaggctg ctttcgcgcg gcagggcgga ttccatcacg ttgcaaatgg 28560
agaggctgtg gcggtgtccc aaggatttgg cgtatttcaa cgcctccatc gtgtccagcg 28620
tttcgccgga ttgggaaatg gtaatgacca gttggtcgga atcagcaatc acgctgcggt 28680
atcggtattc gctggcgatt tcgacgtcgg acgggatttt tgcgatggat tccaaccaat 28740
atttggcggt cagcgcggcg taataggacg tgccgcaggc aaggattttg acgctgcgga 28800
tgctttcaaa cacgcttttg gcatctttgc cgaagttttc ggggatgaag ccgccgtcga 28860
ggaaaacctc cgccgtgtct gcaatcgcgc ggggctgctc gtggatttct ttttgcataa 28920
agtggctgta cagtcccagt tccaaagagg cgagcgagag ttcggatacc ttgactttgc 28980
gttcggcagg caggccgttt ttatcggtca gccttttgat gccgtctgaa gccagcagcg 29040
cgatgtcgcc gtcttcgagg tacgccacgc ggcgcgtaaa ggcgatgacg gcggatacgt 29100
ccgaagcgat aaaggtttca tcgtcgccca aagcgaccaa aagcgggcag cccatacgcg 29160
ccacaactaa ttcatcaggc ttgtcttggg caataaccgc gatggcgtat gcgccgtgga 29220
aacgtttgac cgcttcttgt accgcttcaa acagcctgcc gccgttttgc gcgtattcgt 29280
gattgatgct gtgtgcgatg acttcggtat ccgtttgcga ttcaaaacgg tatcccaaac 29340
cttccaaacg tttgcgttcg ctttcaaagt tttcgatgat gccgttgtgt acgaccgcaa 29400
tcataccgcc gctgatgtgc gggtgggcgt tcggctcagt aacgccgccg tgtgtcgccc 29460
aacgcgtatg tccgatgccg atgccgccgc tgatgccttt ttcgcgtgcc gcgtcctcca 29520
taagctgcac gcgtccgacg cggcgcacac gtttgatttt gccgtcggtg ttgacggcaa 29580
tgcctgatga gtcataaccc cggtattcga ggcgtttgag accgtcggtc agaaaatcga 29640
cgacgttgtg atgggcgcgg atggcgccga cgataccgca cataactgtt ccttagtatc 29700
cggttgaaaa aaaacaggcg cggacggctt ccgtgccgca ccttcctctt cggattataa 29760
accgcctccc gcgccggaaa acagcaaaat gccgtctgaa ggcttgggct tgctcaaaaa 29820
aaggagggat ttccctgttt atccaggatg ggcgttcaga cggcattacc tgctgctggt 29880
ttatagtttt tgcaaatcaa cattgacaag ctgaaaaaaa aaaacaatat actcgctcgg 29940
tcttaatgtt aacggagtat ggaaatgaaa caaatgcttt tagccgtcgg cgtggtggcg 30000
gtgttggcgg gctgcggcaa ggatgccggc ggttacgagg gttattggcg cgaaaagtcg 30060
gacaaaaaag agggtatgat tgccgtcaaa aaagaaaaag gcaattactt ccttaataaa 30120
atccacgtgg ttacaggcaa ggaagagtcc ttgcttttgt ctgaaaaaga cggcgcgctt 30180
tcgataaaca cagggatagg ggaaatcccg atcaaacttt ccgacgacgg gaaagagctg 30240
tatgtcgaac gtaggcagta tgtcaaaacc gatgcggcga tgaaggacaa aatcatcgcc 30300
catcagaaaa agtgcggaca aacagcacag gcataccgcg acgcgcgaaa tgcgttgccg 30360
tcaaaccaga cgtatcagca gcatctggcg gcgatcgagc aattgaaacg gcggtttgaa 30420
gccgagtttg acgaattgga aaaagaaatc aaatgcaacg gcagaagccc ggcattgttg 30480
ctttagtagg ggacaaccgg gaggatgccg ccgtccgaat cggatgtgcg gtttctgtac 30540
cggtacgggc gggcaggaat gtccgccttt tttgttcgga tgcgtttgaa tacccgtttg 30600
attccgaccg tttgcaaggg gtatttccgt tcgggcggaa attatagtgg attaacaaaa 30660
accagtacgg cgttgcctcg ccttagctca aagagaacga ttctctaagg tgctcaagca 30720
ccaagtgaat cggttccgta ctatttgtac tgtctgcggc ttcgtcgcct tgtcctgatt 30780
taaatttgat ccactataat tccgtcaaat aagaaaggaa ttttgtgcct gcggtatcgc 30840
aaaacttcgc cttaatgcgc ccgattgcct agggatgggc ttcagatggc attgttttcc 30900
ggtttacggg cggtattcgg gcttcatacc gttgggtagg agctgccaga catatcccgt 30960
ggttttctgt ttgccggcaa gttcgccggc ttcgtcgccg tatccccaaa aataatccac 31020
gcgcaccgcg cctttaatcg cgctgccggt atcctgcgcc ataatcaggc ggttgagggc 31080

```
tttgcgggta accggatggg cggtggcgac aaataagggc gcacccaagg taatgtagtg 31140
ccggtcgact gcgccggcat attccccat cagcggcgtg cccagtgcgc cgacagggcc 31200
gtcattgctg cttccggcaa gctcgcggaa aaagatatag ctggggtttt gacccaaaac 31260
ttcggcgagg cgttgcggat tttgccgcat ataagactta atgccctgca tggaggtttg 31320
tccgagtttg aggtagccct tatccgccat atagcgtccg atggaaacgt agggatgttc 31380
gtttttgtcg gcatagccga tgcggatgta tttgccggac ggggttttca gacggcccga 31440
gccttggatg tgcataaaaa aaagttcgac agggtcttcg gcgtaaccga gtatcggggc 31500
tttgccgtca agcgcgccgc cgttgatttg gttgcgcgtg tggtagggga ggaagcggct 31560
tccttcaaac ctgcctttga ttgctgttgt gcgcgcggtg atggggaatc gggagaggtc 31620
ggcggtatgt gtgccgccgg tattgtcgat tgtgccgctg ttttttcccg tctgcctgat 31680
gcggacaagg gcttttccgc tccgcaaacc ggcaggcagg gggacggaga taaaatcgtc 31740
gggaataccg taaatcggga agcgggcttg tgccgtccgc ctgtcgtcgc ccttcagcac 31800
cggttcgtaa tagccggtaa ccgtaccggc aaggcttccg ttgcctgcaa cctgccacgg 31860
cgtgaaatag cgttcaaaaa actgttttgc ctgaaaggaa tggacggggg tttgaaaggc 31920
ttgggcgcac acatcctgcc agccttggcg gtttttcaaa ttggcgcagc cgaggcggaa 31980
ggattgcagg cttttggcga aatcctgcgc cgcccagtgg ggcagggaca ggtgcggtac 32040
aacggtatag acggccccgc cgccgccgac cgtcgttccg gcggggtcgg ggatgccgac 32100
cggccggtcc gggccgttga tgacggatgt gtcgggttgc ggaaaggttt ggatgctctt 32160
gctttggcag gcggcgagga tggcggcggc gatgccgtac agggcggcgc ggaataggta 32220
ttttttcata atggacaatg ttgccggcag taataagaaa gatggtttcg ggcggcgttg 32280
cggcagccgt ggagagggga ttttaacaca gggcgcagct gcagcctgcg gaactttccg 32340
ccgcgcggta ctgcagataa aaataacttg catttgtatt tacaagcaat gaaaatattc 32400
cgataatata ttattcatca tccttgttcg ttcgcgttta tgctggtcgc ttttttaatt 32460
atgttgcgcg agggtattga agccgcgctc attgtcggca tcgttgccgg ttttctgaaa 32520
cagtccggac attccaaact gatgcctaag gtctggttcg gggtcgtcct tgcttctttg 32580
atgtgtttgg ggctggggta cggcatccat tcggcaacgg gcgagattcc ccagaagcag 32640
caggagttcg tcgtcggcat tatcggtttg gttgccgttg ccatgctgac ttatatgatt 32700
ttatggatga aaaaggcggc gcgttcgatg aagcggcagc ttcaggattc tgtgcaggcg 32760
gctttgaacc gtggcagcgg tcaaggatgg gccttggtcg gtatggcgtt tcttgccgtg 32820
gcgcgcgaag gtctggagag tgttttttc ctgcttgccg tattcaaaca gagcccgacg 32880
tggcagatgc cggccggcgc ggtagcgggg gttttggctg ccgccgtgat tggcgcgttg 32940
atttatcagg gcgggatgcg cctgaatctg gcgaagtttt tccgttggac gggggcgttt 33000
ctgattgtcg ttgccgccgg cctgcttgcc ggctcgctgc gcgcgctgca tgaggcaggt 33060
atttggaacg cgcttcagga cattgtgttc gactcatcaa aatatttgca cgaagacagt 33120
ccgttgggcg tgctgctcgg cggatttttc ggctataccg accatccgac gcagggcgag 33180
accttggttt ggctgctgta ccttattccc gtcataactt ggtttttgtg cggcagcagg 33240
ccgtctgaaa ctttaacccg taaagaggag ctgaaatgag aaaattcaat ttgaccgcat 33300
tgtccgtgat gcttgcctta ggtttgaccg cgtgccagcc gccggaggcg gagaaagctg 33360
cgccggcagc gtccggtgag gcgcaaaccg ccaacgaggg cggttcggtc agtatcgccg 33420
tcaacgacaa tgcctgcgaa ccgatggaac tgaccgtgcc gagcggacag gttgtgttca 33480
atattaaaaa caacagcggc cgcaagctcg aatgggaaat cctgaaaggc gtgatggtgg 33540
tggacgagcg cgaaaacatc gcccccggac tttccgataa aatgaccgtc accctgttgc 33600
cgggcgaata cgaaatgact tgcggtcttt tgaccaatcc gcgcggcaag ctggtggtaa 33660
ccgacagcgg ctttaaagac accgccaacg aagcggattt ggaaaaactg tcccaaccgc 33720
tcgccgacta taaagcctac gttcaaggcg aggttaaaga gctggtggcg aaaaccaaaa 33780
cttttaccga agccgtcaaa gcaggcgaca ttgaaaaggc gaaatccctg tttgccgaca 33840
cccgcgtcca ttacgaacgc atcgaaccga ttgccgagct tttcagcgaa ctcgaccccg 33900
tcatcgatgc gcgtgaagac gacttcaaag acggcgcgaa agatgccgga tttaccggct 33960
ttcaccgtat cgaatacgcc ctttgggtgg aaaaagacgt gtccggcgtg aaggaaattg 34020
cagcgaaact gatgaccgat gtcgaagccc tgcaaaaaga aatcgacgca ttggcgtttc 34080
ctccgggcaa ggtggtcggc ggcgcgtccg aactgattga agaagtggcg ggcagtaaaa 34140
tcagcggcga agaagaccgg tacagccaca ccgatttgag cgacttccaa gccaatgtgg 34200
acggatctaa aaaaatcgtc gatttgttcc gtccgctgat cgaggccaaa aacaaagcct 34260
tgttggaaaa aaccgatacc aacttcaaac aggtcaacga aattctggcg aaataccgga 34320
ctaaagacgg ttttgaaacc tacgacaagc tgggcgaagc cgaccgcaaa gcgttacagg 34380
cctctattaa cgcgcttgcc gaagaccttg cccaacttcg cggcatactc ggcttgaaat 34440
aagccgcaag cgttcagacg gtatttggcg gcagataccg tctgaagttt tatagtggat 34500
taacaaaaac cagtacggca ttgcctcgcc ttgccttgcc gtactattta tactgtctgc 34560
ggcttcgtcg ccttgtcctg atttttgtta atccactata tccgccatat attgcagggc 34620
gggatttcaa cctgccgcta tcggttaatg gaaaaacggc gtgcagggat acccatcctg 34680
ctgcacggat attgaaggaa acaccatgag caaaaaacaa cccgcacaac cgaccaggcg 34740
```

cactcttttt aaaaccgcga tcgcagccgg agcagtcggc gcaatcggag gttatctcgg 34800
cggcaaaaaa cagggcgaaa ccgccgaacg caccgccgaa agccaacact cgccccaagc 34860
ctatccctgc tacggcgaac atcaggcagg catcgttacg ccgcagcagg cgttttcgat 34920
tatgtgcgcc ttcgacgtaa ccgcgcaaag tgccaagcag ctggaaaacc tgttccgcac 34980
gctgaccgcc cgcatcgagt ttctcaccca aggcggcgaa taccaagacg gcgacgacaa 35040
acttccgcca gccggcagcg gcattttggg caaagccttc aaccccgacg ggttgaccgt 35100
taccgtgggg gtgggcagca gcctgtttga cggccggttc ggactcaaag acaaaaaacc 35160
gattcatttg caggaaatgc gcgacttctc caacgataag ctgcaaaaaa gctggtgcga 35220
cggcgatttg agcctgcaaa tctgtgcctt cacccccgaa acctgccaag ccgccctgcg 35280
cgacatcatc aaacacaccg tccaaaccgc cgttatccgt tggagtatcg acgggtggca 35340
gcccaaatcc gaacccggcg cgatggcggc gcgcaacctg ttgggcttca gggacggcac 35400
gggcaacccc aaagtttccg atcccaaaac tgccgacgag gttttgtgga cgggggtggc 35460
cgccaacagc ctcgacgaac cggagtgggc gaaaaacggc agctatcagg cagtccgcct 35520
tatccgccac tttgtcgagt tttgggacag gacgccgctt caagagcaaa ccgacatttt 35580
cgggcggcgc aaatacagcg gtgcgccgat ggacggcaaa aaagaagccg accaaccgga 35640
ttttgccaaa gaccccgagg gtgatatcac gcccaaagac agccatatac gcctggcgaa 35700
tccgcgcgat cccgaattcc tcaaaaaaca ccgcctcttc cgccgcgcct acagctattc 35760
gcgcggactc gcctcaagcg gacagcttga tgtcgggctg gtgttcgtct gctatcaggc 35820
aaaccttgcc gacggattca tcttcgtgca aaacctcctc aacggcgaac cgctggaaga 35880
atacatcagc cccttcggcg gcggctattt cttcgtcttg cccggcgtgg aaaaaggcgg 35940
ctttttgggg caagggctgc tgggcgtata aatccgccat ataaaaaacg ccgtccgaac 36000
cttgccaaac gggttcggac ggcgtttctt gtttttgggc ggtcaggctt ttttgacgaa 36060
ttcggatttt aaattcatcg cgctgccgtc gattttgcag ccgatgttgt gatcgccttc 36120
ttgcaggcgt atgcctttga cttttgtgcc ttgtttgatc accatcgagc tgccttttac 36180
cttgaggtct ttgatgagga tgacggtatc gccgttttgc agcactgcgc cgttggcatc 36240
gcgcacttga gccgcaaggt cggcggcgga ttcggtttca ttccattcat gggcgcattc 36300
ggggcagatg tattgtccgc cgtcttcata ggtgtattcg gaggcgcatt gcgggcatgg 36360
gggtaatgac atggtttgcc gtccttatcg gatgtttgtt ttggggtgcc gtctgaaacc 36420
tgaaaccggc ttcagacggc atagctttat tgtttgtctt tttcaggacg cacccagcct 36480
tcgatgacgg tttggcgggc gcgggcgagg gcgagtttgt tgtcttcgac attgcgggta 36540
atcgtgctgc ccgcgcctgt ggttactttg ttgccgaggg taacgggggc gactaggacg 36600
cagtttgaac cgatgcgcac ttcgtcgccg atgacggttt tgtgtttgtg cacgccgtcg 36660
tagttggcaa taatcgtacc ggcgccgaag ttggttttgc agccgacttc ggcgtcgccg 36720
atgtaggtga ggtggttggc tttggtgcct ttgccgatgg cggcgttttt gatttcgacg 36780
aagttgccga cgtgtacgtc gtctgcaagg cgggcttgcg gacgcaggcg ggcgtacggg 36840
ccgattcggt tgttttcgcc gacttcgcag ctttcgaggt gggagaaggg ggcgattttg 36900
ctgtttgcgc cgattttggc gtttttgatg acgcagtttg cgccgatttc gacgttgtcg 36960
ccgagctcga tgtcgccttc aaagatacag ttcacatcaa tcacgacgtc ttgcccgtgt 37020
ttcagacggc ctcgtaaatc gaaacgtgcc ggatcgcgca gggttacgcc tgctttgagc 37080
aattcttgcg cctgttcggt ttggaagatg cgttcgagtt cggtgagctg gagtttgttg 37140
ttcacgccgg cggcgaggtg ggaggcgcgc acttggacgg gatgaacttt aataccgtcg 37200
gcaacggctt tggcgatgag gtcggtcagg tagtattcgc cttgtgcatt gttgctggaa 37260
aggctgttca gccagttttc gagtttggcg ttgggcagga cgaggatgcc ggtattgatt 37320
tctttcacgg ctttttggac ggcgtcggcg tctttttctt cgacgatggc ggttacgctg 37380
ccgttgctgt cgcggatgat acgccccaag cctgtcgggt cgttgggaac gtcggtcaac 37440
agcccgactt cgttgcctgc ggcttcgagc agggtttcga gggtttcaac gtcaattaaa 37500
ggaacgtcgc cgtacaacac cagcgtgcgg ccttcggcgg aaaggtgggg cagggcggtt 37560
ttgacggcgt ggccggtacc gagctgttcg gtttgttcaa cccaaacgac atcgcgtttg 37620
acggtgtcca agacttgctc tttgccgtgg ccgatgacga cgcagatgtt ttgcggattc 37680
agtgcggctg cggtgtcgat aacgcgcccg accatgggct tgccgccgat gcggtgcagc 37740
acttttggca ttttggaata catgcgcgtg cctttgccgg cggcgaggat gacgatgttt 37800
aaagtgtttt gcggcatgac ggtttcctgt gcaatgccgt ctgaagcggc ttcagacggc 37860
atagggtagg tttatcggtt ttgaaacttt ggtttttgcc agtgttggcg atgctcttcg 37920
tcggcgttgt tgccggtttg attgggtaac acggcatggc gttcgggacg gtattggttg 37980
tagttcatat ttttcgagta gctgccgtct tggtaataaa cgggcgtgcc ggcgggatat 38040
ttttgacgga cggcggtctt gccgttgccg tcttgataag tttcccacgc gcagcccgac 38100
aaaagggcgg cggcagcggt caggaagagg aaggttttac gcatggcttt tctttcgtat 38160
tttcgggggg taggggtat tgtaatgatt ttggcggtgt tctgacaaag tttctgcata 38220
ccgagccagt tgcgccatat cgcttacgga ggcatcgata aagggcagcg cgtgggattt 38280
tgcaccgaac cggacggttt tcatacccag cgcctttgcc tgatgcaggt tgtccgcgct 38340
gtcgtccacc ataatgcagc attcgggcgg tacgtccaac aggcggcaga cattgagata 38400

cgcttgcgga ttgggtttgt acagcagccc gaaatcatcc gtgccgaaaa gcgcgtcgaa 38460
acggttttcc aaaccgagtg cgttgacaac ggcacggacg taaaacgacg ggccgttgga 38520
aaaaaccgcc ttgcgccctt ttaggcggct cagggtgttt tgtgtttcag gcatgccgtg 38580
cagcctggtc aggattgcat cgatcggatg gctttcgcgc aaaaattcgg cgatgtcgat 38640
ttcgggatgg tggatttgca gtccggcgag cgttgcgccg tagcggtgcc aatagtcttg 38700
acgcaggtcg gacgcggcag attcggagag tttgaggcgg cgtgccatat agcgtgtcat 38760
agcgcggttg atgagtgtga agatgcctgc gtcggcatcg tgcagcgtgt tgtcgaggtc 38820
gaacagccac acggtcgggt ttcttgcat gttgaaccgt gaaaatttgt tagaatgtta 38880
ttttacagcg aatagaggag gactcggaat gaaacggaaa atttggctgc tgccgctgct 38940
ggcggtttcg gcatacctgc aggcgcagac ggaagtcagg ctggcggtgc ataagtcgtt 39000
cagcctgccc aaagggttga ttgcgcgctt cgagcgggca aacgatgcga aggtgtcgat 39060
tattcaggcg ggcggcgcga acgaaatgct caacaaactg attttgagcc gcgccaaccc 39120
gattgccgac gcggtgtatg gtttggacaa cgccaatatc ggcaaggcgc gggaaatggg 39180
cattttggcg gcggcgcaac ccgaatccgc ccccgtcgcg gtcgggctgc cttcggcttt 39240
ggcggtcgat tacggctatg tgtccatcaa ttacgacaaa aaatggtttg aaggcaaaaa 39300
gctgcccctg ccgcaaaccc tgcaggattt gacccgcccc gaatataaaa acctattggt 39360
cgtgccgtcc cccgccacgt cgtccccggg gctgggcttc ctgatggcga acatcagcgg 39420
tctgggcgaa gaaagcgcgt tcaaatggtg ggcacagatg cggcagaacg gcgtgaaggt 39480
cgccaaaggc tggagcgagg cgtattacac cgacttttcg cacaacggcg gcgcgtatcc 39540
gctggtggtc ggttatgccg ccagcccggc ggcggaagtg tatttttcca aaggcaaata 39600
cagcgagccg ccgacgggca acctgttttt aaaaggcggc gtattccgcc aggtcgaagg 39660
cgcggcggtc ttgaagggcg cgaaacagcc ggaattggcg gcaaaactgg tgcaatggct 39720
gcaaagtcgg gaagtgcagc aggcggttcc gtccgaaatg tgggtttacc ccgccgtcaa 39780
aaacacgcgc ctgcccgacg tgttccgctt cgcccaagcc ccgacgcaca ccaccgcccc 39840
cgcgcagcgc gatattgatg cgaaccagcg cggatgggtt tcccgttgga ttagaacggt 39900
tttgaaataa aacaaacata cctccccgca gggcttcata cggcattttt acacctgtgc 39960
cgattacgcc gcacggggcg gatgttcgat caagaggaaa acaatggact tcaaacaatt 40020
tgattttttta cacctgatca gtgtttccgg ttgggagcat ctggctgaaa aggcgtgggc 40080
gttcgggctg aaccttgccg ccgcgctgct tattttttttg gtcggaaaat gggcggcgaa 40140
acgcattgtc gctgtgatga gggcggcgat gacgcgcgcg caggtcgatg ccacgctgat 40200
tagttttttg tgtaatgttg ccaatatcgg cttattgatt ttggtgatta ttgccgcatt 40260
gggcagattg ggcgtttcca caacatccgt aaccgcctta atcggcggcg cgggtttggc 40320
ggtggcgttg tccctgaaag accagctgtc caattttgcc gccggcgcac tgattatcct 40380
gttccgcccg ttcaaagtcg gcgattttat ccgcgtcggc ggttttgaag gatatgtccg 40440
agagattaaa atggtgcaga cttctttgcg gacgaccgac aacgaagaag tcgtgctgcc 40500
caacagcgtg gtgatgggca acagcatcgt caaccgttcc acactgccgc tgtgccgcgc 40560
ccaagtgata gtcggcgtcg attacaactg cgatttgaaa gtggcgaaag aggcggtgtt 40620
gaaagccgcc gtcgaacacc ccttgagcgt tcaaaacgaa gagcggcagg ctgccgccta 40680
catcaccgcc ttgggcgaca atgccatcga aatcacatta tgggcttggg caaacgaagc 40740
agaccgctgg acgctgcaat gcgacttgaa cgaacaagtg gtcgaaaacc tccgcaaagt 40800
caatatcaac atcccgttcc cgcaacgcga catacacatc atcaattctt aaacgccgtc 40860
tgaaagagga gtgggaaatg gacgcgctgc acaccatcgc ccgaaacctg acgaaaaaac 40920
gtcaaaccgt aagctgtgcc gaatcctgta cgggcggaat gcttgccgcc gcattcacaa 40980
gcgttgcagg cagttcgcaa tggttcgacc agagttttgt aacatacagc aacaaagcca 41040
aagaagaccg cttgggcgtg ttgcccgaaa ccctgctcga acacggcgcg gtcagccgcc 41100
aaaccgtcta tgagatggcg cgcggcgcga aagccgtggc gcaggcggat tacgccgtcg 41160
gtatttccgg catcgccggt ccgggcggcg gcagcgaaag caaacccgtc ggcacggttt 41220
ggttcgggtt tgcctttccg ggcggaagtt gcgaagcaat gcgccgtttt gacggcaacc 41280
gcgaatccgt ccgcgcgcag gcggtcgcct tcgcgttgga acggttggcg gggctgattg 41340
aaaacggcgg cgatgctgtc taaacaaaat ctccgtctga acaaaatccc catcggataa 41400
aaaatgccgt ctgaaacgtt tcgggtttca gacggcattt tgtcggggta ggcggcggtg 41460
cggcttattt cactttacct ttcaacgcgc catagcctgc cgcgtccatt tgttccagcg 41520
ggatgaattt caagctcgcg ccgttgatgc agtagcgcag tccgcctttg tcgcgcgggc 41580
cgtcggggaa gacgtgtccc aaatgcgagt cggcggcgtg gctgcgcact tcggtgcggc 41640
gcatgttgta gctgaaatca tcgtgttcgg taacggattt tgcatcaatc gggcgcgtga 41700
agctcggcca gccgcagccg gaatcatatt tgtcggcgga gctgaacaaa ggttcgccgc 41760
tgacaacgtc cacataaatg ccgggtttga acaaatggtc gtattcgtgg ctgaaggcat 41820
attcggtcgc gctgttttgg gtaacttggt attgctcttc ggtcagggtg cgtttgagtt 41880
cggcgtcact cggtttttta tacgttgccg cgtcgaagcc tttgccttgc ggggcggtct 41940
tggttttgcc cggcagcggt tcgtcagctt tgcggatgtc gatgtggcag tagccgttgg 42000
ggtttttaat caagtagtcc tgatggtatt cctcggcatc gtagaagttt ttcagcggct 42060

cgttttcaac aacgaggggc agttggtatt tttgctgctc gcgtttgagg gcggcggcga 42120
tgacggcttt ttcggcgggg tcggtgtagt acacgccgct gcggtattgc gtaccggtgt 42180
cgttgccctg tttgttgagg ctggtcggat caacgacgcg gaagaaatat tgcaggatgt 42240
cgtctaggct gagtttgtcg gcatcgtagg tcactttgac ggtttcggcg tggcccgtat 42300
ggcggtagga cacgtcttca tagctcggat ttttcgtgtt gccgttggcg tagccggata 42360
ccgcgtcaac cacgccgtcg atgcgttgga aataggcttc caagccccag aagcagccgc 42420
cggcgaggta aatggtgcgc gtgttcatga tttttgaatc ctttttctga gtgtcgggtt 42480
tgtagaacga atgtttcaag ctgcccaaat cggcattcgg gtcgcggatt aacgccaacg 42540
cctgcgcttc gttgatgctg cctttgacga tgcgctgcac gtcgctgtct ttaccgatta 42600
acgcccacga ggggtaaacg ctgatattca ggctttgggc gatcgtgccg ccgttgtcgg 42660
ttacgacggg cagcttggga taattcaaac cggcatacca tttttggaag tcgccgtctt 42720
ttttctcgtg caaaaagccc ggggaggcga cggtaatcag gttggcggag ctgaattttg 42780
catcttgcgc ccatttttcg gtctgtccca attcggacag acacaaagga caccagctcg 42840
cccaaaattt aatcagcgtc ggtttgtctt ttttcaagta aacactggcg gggcggttgt 42900
ccgcagtttt caaagtggat aaagtgtgcg gcacggtcgc ggctccggca tcgacgattt 42960
tgggcgaaca agcacccagc gcaagcaggc agccgaactt ggcgcaaagg gaaaagaaag 43020
tacggtgttt cattttgatg tttcctgtgt ggacggtttg catgattaga cgtttgagat 43080
gccgaaacct tacagcccgg attttcagac aaccttaccg cgtaaaatac gctacaatac 43140
gccctgtttc aagtttctaa aattaaaagg aaaattcaat gttcagcttc ttccgtcgca 43200
agaaaaaaca ggaaacgccg gctctcgagg aggctcaaat tcaggaaacc gcagcaaaag 43260
cagaatctga acttgctcaa atagttgaaa atattaaaga agatgctgaa tctttagcag 43320
aaagcgtcaa agggcaggtc gaatctgccg ttgaaaccgt cagcggtgcg gttgaacagg 43380
taaaggaaac cgttgccgag atgctgtctg aagcagagga agcggcggaa aaagcagcgg 43440
aacaagtcga agcggcaaaa gaagccgttg ccgaaaccgt cggcgaggct gtcgggcaag 43500
ttcaagaagc cgttgcgaca actgaagaac acaagctcgg ttgggcggcg cgtttgaaac 43560
aaggcctgac caaatcgcgc gacaaaatgg cgaaatcgct ggcgggcgtg ttcggcggcg 43620
gacaaatcga cgaagattta tacgaagagc tggaaaccgt gctgattacc agcgatatgg 43680
gcatggaagc caccgaatac ctgatgaaag acgtgcgcga ccgcgtcagc ctcaaagggc 43740
tgaaagacgg caacgaattg cgcggcgcgt tgaaagaagc cttgtacgac ctgattaagc 43800
ctctggagaa acctttggtt ttgcccgaaa ccaaagagcc gtttgtcatc atgcttgccg 43860
gcatcaacgg cgcgggcaaa accacgtcta tcggtaaact cgccaaatat ttccaagcgc 43920
agggcaaatc cgtattgctg gcggcaggcg atactttccg tgccgccgcg cgtgagcagc 43980
ttcaagcttg gggcgagcgc aacaacgtaa ccgtgatttc gcaaaccacg ggcgattccg 44040
ccgccgtgtg cttcgatgcc gtccaagccg ccaaagcgcg cggcatcgac attgtgctgg 44100
ccgacaccgc cggccgcctg cccacgcagc ttcatttgat ggaagaaatc aaaaaagtga 44160
aacgcgtgct gcaaaaagcc atgcccgacg cgccgcacga aatcatcgtc gtgcttgatg 44220
ccaatatcgg gcaaaacgcc gtcaaccaag tcaaagcctt tgacgacgca ttggggctga 44280
ccggtttaat cgttaccaaa ctcgacggca cggcaaaagg cggcatcctc gccgcgcttg 44340
cctccgaccg ccccgttccc gtccgctata tcggcgtggg cgaaggcata gacgacctgc 44400
gcccgtttga cgcgcgcgcg tttgtggacg cactgctgga ttgagccgaa atgccgtccg 44460
aaaacagcag accgatgccg tcattcccgc gcaggcggga atccagacct tgggataacg 44520
gcaatattca aaggttatct gaaagtccga gattctggat tcccactttc gtgggaatga 44580
cgggatgtag gttcgtggga atgacgtggt gcaggtttcc gtatggatgg attcgtcatt 44640
cccgcgcagg cgggaatcta gaacgtaaaa tctaaagaaa ccgtgttgta acggcagacc 44700
gatgccgtca ttcccgcgca ggcgggaatc tagaccattg gacagcggca atattcaaag 44760
attatctgaa agtccgagat tctggattcc cactttcgtg ggaatgacgg gatttgagat 44820
tgcggcattt atcggaaaaa acagaaaccg ctccgccgtc attcccgcgc aggcgggaat 44880
ctaggtttgt cggtgcggaa acttatcggg taaaacggtt tctttagatt ttgcgttcta 44940
gattcccact ttcgcgggaa tgacgaagag ttgcgggaat gatggaaagc tatgggaata 45000
acgaagggtt aaagtaatca cgggatggtg ttcgcgggaa tataaattga aataattcaa 45060
aagggtatta tatgcagcct gcggtttata ttttagcaag ccaacgtaat ggcacgttat 45120
acattggcgt tacatctgat ttggtgcaac gtatttacca acatagggag catttgattg 45180
agggatttac atcacggtac aacgttacta tgctggtttg gtatgaactg catcctacga 45240
tggagagtgc aattactcgg gaaaaacagt tgaagaaatg gaacagggct tggaaattgc 45300
aactgattga agaaaataat gtttcttggc aggatttatg gtttgatatt atttagcccg 45360
tcattcccgc gcaggcggga atccggcttg ttcggtttcg gttttttttt tgaggtttcg 45420
ggcaacttct aaaccgtcat tcccgcgtag gcgggaatct agaccttggg ataacggcaa 45480
tattcaaagt ttataaaaga cccgttattc ccgcgcaggc gggaatctag accttagaac 45540
aacagtaata ttcaaaggtt agctgaagct ttagagattc tagattccca ctttcgtggg 45600
aatgacggga tgtaggttcg cgggaatgac gggatttgag attgcggcat ttatcggaaa 45660
aaacagaaac cgttctgccg tcattcccgc gcaggcggga atccggcttg ttcggtttcg 45720

gttttttga ggtttcgggc aacttctaaa ccgtcattcc cgcgcaggcg ggaatctaga 45780
ccattggaca gcggcaatat tcaaagatta tctgaaagtc cgagattcta gattcccact 45840
ttcgtgggaa tgacgggatg taggttcgtg ggaatgacgg gatttgagat tgcggcattt 45900
atcggaaaaa acagaaaccg ctctgccgtc attcccgcgc aggcgggaat ccggcttgtt 45960
cggtttcggt ttttttttt tttgaggttt cgggcaactt ctaaaccgtc attcccgcgc 46020
aggcgggaat ccagaccatt ggacagcagc aatattcaaa gattatctga aagtccggga 46080
ttctagattc ccactttcgt gggaatgacg ggatgtaggt tcgtgggaat gacgggattt 46140
gagattgcgg catttatcgg aaaaacagca accgctccgc cgtcattccc gcgcaggcgg 46200
gaatctagac cttgggataa cagcaatatt caaaggttag ctgaagcttt agagattctg 46260
gattcccact ttcgtgggaa tgacggaatg taggttcgtg ggaatgacgg gatttgagat 46320
tgcggcattt atcggaaaaa cagcaaccgc tccgccgtca ttcccgcgca ggcgggaatc 46380
tagaccttgg gataacagca atattcaaag gttagctgaa gctttagaga ttctggattc 46440
ccactttcgt gggaatgacg gaatgtaggt tcgtgggaat gacgggatta gagtttcaaa 46500
atttattcta aatagctgaa actcaacgca ctggattccc gcctgcgcgg gaatgacgaa 46560
ttttaggttt ctgattttgg ttttctgttt ttgagggaat gacgggattt gagattgcgg 46620
catttatcgg gagcaacaga aaccgctccg ccgtcattcc cgcgcaggcg ggaatctaga 46680
ccttagaaca acagcaatat tcaaaggtta gctgaagctt tagagattct agattcccac 46740
tttcgtggga atgacggaat gtaggttcgt gggaatgacg cggtgcaggt ttccgtatgg 46800
atgggttcgt cattcccgcg caggcgggaa tccggcttgt tcggtttcgg ttttttttt 46860
ttgaggtttc gggcaacttc taaaccgtca ttcccgcgca ggcgggaatc tagaccttag 46920
aacaacagca atattcaaag attataaaag acctgtcatt cccgcgcagg cgggaatcta 46980
ggtctgtcgg cacggaaact tatcgggtaa acggtttctt gagattccgc gtcctggatt 47040
cccactttcg tgggaatgac gggatgtagg ttcgtgggaa tgacgcggtg caggtttccg 47100
tatggatggg ttcgtcattc ccgcgcaggc gggaatctag accttagaat aacagcaata 47160
ttcaaagatt atctgaaagt ccgagattct ggattcccac tttcgtggga atgacggaat 47220
gtaggttcgc gggaatgacg cggtgcaggt ttccgtgagg atggattcgt cattcccgcg 47280
caggcgggaa tctagacctt agaacaacag caatattcaa agattataaa agacctgtca 47340
ttcccgcgca ggcgggaatc cagaccttag aacaacagca atattcaaag gttagctgaa 47400
gctttagaga ttctggattc ccactttcgt gggaatgacg ggatgtaggt tcgtgggaat 47460
gacgcggtgc aggtttccgt gcggatggat tcgtcattcc cgcgcaggcg ggaatccaga 47520
ccttgggata acagcaatat tcaaaggtta taaaagaccc gtcattcccg cgcaggcggg 47580
aatctagacc ttagaacaac agtaatattc aaaggttagc tgaagcttta gagattctgg 47640
attcccactt tcgtgggaat gacgggatta gagtttcaaa atttattcta aatagctgaa 47700
actcaacgca ctggattccc gcctgcgcgg gaatgacgaa ttttaggttt ctgattttgg 47760
ttttctgttt ttgtaggaat gatgaaattt tgagttttag gaatttatcg gaaaaaacag 47820
aaaccgctcc gccgtcattc ccgcgtaggc gggaatccag accgttgggc atctgcagcg 47880
gtttgctaaa aaccgcttta ctgtgataag tgcgcagggt tagaatggcg cggtaacctt 47940
atatattgta ccccgtcaaa ggggcgcatt gcttttctta acattcccct ttggcagcca 48000
agtgaaaggg cttttcaatc agcaattcgg cgggcgcgga atcgggcggt ttaccgaacc 48060
ccggcgttcg cggcgcgccg ccccgtcccg tgaaggcaaa ctcaaggaat aaaagatgaa 48120
taaaacttgg aaacggcagg ttttccgcca taccgcgctt tataccgcca tattgatgtt 48180
ttcccatacc ggcggggggg ggggcaggcg caagcgcaaa cgcaaacgca aacgcataaa 48240
tacgctattg taatgaacgc gcaaaatctg cccgaggtaa agtgggggga tcaatatcag 48300
tcattgacgc acaaaagcaa tgaacgcgaa gttatccata cgagtggttt tggtttggca 48360
aaaaagagca ttagtttctc attcaataat accgatgaag ttgttgctga aaaaaaagat 48420
actgtcgttt tcggcgcggc gacctacctg ccgccctacg gaaaggtttc cggttttgat 48480
accgctaagc tgaccgagcg caaaaatgcc cttgatcaga ttggtacgac caaaacgggg 48540
ctggtaggct acagctacga aggtagcaca tgctccagcg gaggttgtcc tacagttgcc 48600
tatagaaccc aatttacctt cggcaattcc agtttggcaa aaaaggcaaa cggcggcggg 48660
ctggatatat acgaagacaa aagccgcgac aattcgccca tttacaaatt gaaggatcat 48720
ccttggttgg gcgtgtcttt caatttgggc ggagagagct ccttcaaacc aaagagacaa 48780
ggttctttgg tatcttcttt tagcgaggac gtgacgcagc aaaatggtgc gggcagccaa 48840
cacaaagaca aaaacctcgt ttatacgaca gacgattaca agagtcagaa taataaaaac 48900
catcaggaca aacaccacgc cgtcgccttt tatctgaacg ccaagctgca cctgctggat 48960
aaaaaacaca ttaaaaatat cgtgcaaggt aaaacagtta atttgggtat cttgaaaaca 49020
cgcatcgagc cgacggaagc atggaaaaga cggaatagta acttttttaa cggtagttgg 49080
acgtatgaag agaaaggaac agtcagcgtc aaactcaaat tgccggaagt caaagcaggc 49140
cgctgcatca acgcaaataa ccccaataag agtaccaaag ccccttcccc cgcactgact 49200
gcccccgcgc tgtggttcgg acctgtgcaa aatggtaagg tgcagatgta ttccgcttcg 49260
gtttccacct accccgatag ttcgagcagc cgcatcttcc ttcaaaatct gaaaagaaaa 49320
accgacccca acaaacccgg ccgccattcc ctcgcagact tggctaagtc ggatattgaa 49380

aatcgacagc cgaatttcac agggcggcaa accatcatcc gattggatgg cggcgtacag 49440
cagatcaaac tgggtagaaa caatgatgag gtcgccaatt ttaatggaaa tgacggcaaa 49500
aacgacactt tcggcattgt tagtgaaggg agcttcatgc ctgatgccag cgagtggaaa 49560
aaagtattgc tgccttggac ggttcgtgct tccaatgatg acggtcaatt taacacattc 49620
aacaaagaag aaaaagacgg caagccaaaa tacagccaaa aataccgcag ccgcgacaac 49680
ggcaagcacg agcgcaattt gggcgacatc gtcaacagcc ccatcgtggc ggtcggcgag 49740
tatttggcta cttccgccaa cgacgggatg gtgcatatct tcaaacaaag cggcggggac 49800
aagcgcagct acaatctgaa gctcagttat atcccgggta cgatgccgcg caaggatatt 49860
caaaacaccg aatccaccct tgccaaagag ctgcgcgcct ttgccgaaaa aagctatgtg 49920
ggcgaccgct acggcgtgga cggcggcttt gtcttgcgca aagtcgaacg gaacgggaaa 49980
gaccatgtgt ttatgttcgg cgcgatgggc tttggcggca gaggcgcgta tgccttggat 50040
ttaagcaaaa tcgacagcgg caacggcaac ctggcagacg tttccctgtt tgatgtcaaa 50100
catgacaaga atggcaataa cggcgtgaaa ttaggctaca ccgtcggcac gccgcaaatc 50160
ggcaaaaccc acgacggcaa atacgccgct ttcctcgcct ccggttatgc gactaaagac 50220
attaccagcg gcgacaataa aaccgcgctg tatgtgtatg atttggaaag cagcggcacg 50280
ctgattaaaa aaatcgaagt acccggtggc aagggcgggc tttcgtcccc cacgctggtg 50340
gataaagatt tggacggcac ggtcgatatc gcctatgccg gcgatcgcgg cggcagtatg 50400
taccgctttg atttgagcaa tcaagatcct aatcaatggt ctgtacgcgc catttttgaa 50460
ggcacaaaac cgattacttc cgcgcccgct atttcccaac tgaaagacaa acgcgtggtt 50520
atcttcggca cgggcagtga tttgagtgag gatgatgtac tcagtacgag cgaacaatat 50580
atttacggta tcttcgacga cgatacggtg gcgaataacg taaatgtaaa actcagcggt 50640
ttgggaggcg ggctgctcga gcaagagctt aagcaggagg ataaaacctt attcctgacc 50700
gattacaagc gatccgacgg atcgggcagc aaagggtggg tagtgaaatt gaagggcgga 50760
cagcgcgtta ccgtcaaacc gaccgtggta ttgcgtaccg cctttgtaac catccataaa 50820
tatacgggta cggacaaatg cggcgcggaa accgccattt tgggtatcaa taccgccgac 50880
ggcggcaagc tgaccaagaa aagcgcgcgc ccgattgtgc cggccgagaa tcaggctgtc 50940
gcgcaatatt ccggccataa gaaaggcatc aacggcaaat ccatccctat aggttgtatg 51000
caaaaaggca atgaaatcgt ctgcccgaac ggatatgttt acgacaaacc ggttaatgtg 51060
cgttatctgg atgaaaagaa aacagacgga ttttcaacaa cggcagacgg cgatgcgggc 51120
ggcagcggta tagaccccgc cggcaagcgt tccggcaaaa acaaccgctg cttctcccaa 51180
aaaggggtgc gcaccctgct gatgaacgat ttggacagct tggacattac cggcccgacg 51240
tgcggtatga aacgaatcag ctggcgtgaa gtcttctact gatttgcacg cgaaaatgcc 51300
gtccgaaagg ttttcggacg gcattttttg cgtttttcgg gaggggcggg ttcgtaaaag 51360
gcgggctata gggtaggctt catctcgcca atctcactga atccatcaat ttccacaatt 51420
caattaaata ccgtcaaacc gatgccgtca ttcccgcgca ggcgggaatc tagaccttag 51480
aacaacagca atattcaaag gttagctgaa gctttagaga ttctggattc ccactttcgt 51540
gggaatgacg ggatgcaggt ttccgtatga atggattcgt cattcccgcg caggcgggaa 51600
tccagacctt agaacaacag taatattcaa agattatctg aaagtccgag attctggatt 51660
cccactttcg tgggaatgac gggattttag gtttctgatt ttggttttct gtttttgtag 51720
gaatgatgaa attttgagtt ttaggaattt accggaaaaa acagaaaccg ttctgtcgtc 51780
attcccgcgc aggcgggaat ctagacattc aatgctaagg caatttatcg ggaatgactg 51840
aaactcaaaa aactggattc ccactttcgt gggaatgacg ggatttgaga ttgcggcatt 51900
tatcgggagc aacagaaacc gctctgccgt cattcccgcg caggcgggaa tccagacctt 51960
agaacaacag taatattcaa agattatctg aaagtccgag attctggatt cccgcctgcg 52020
cgggaatgac gaattttagg tttctgattt tgtttttctg tttttgtggg aatgatgaaa 52080
ttttgagttt taggaattta tcggaaaaaa cagaaaccgc tctgccgtca ttcccgcgca 52140
ggcgggaatc tagaccttag aacaacagca atattcaaag attatctgaa agtctgagat 52200
tctagattcc cactttcgtg ggaatgacgg gatgtaggtt cgtgggaatg acgtggtgca 52260
ggttcgtggg aatgacgtgg tgcaggttcg taggaatgac gtggtgcagg tttccgtgcg 52320
gatggattcg tcattcccgc gcaggcggga atctagacct tagaacaaca gcaatattca 52380
aaggttatct gaaagtccga gattctggat tcccactttc gtgggaatgg cgcgattaga 52440
gtttcaaaat ttattctaaa tagctgaaac tcaacgcact ggattcccgc ctgcgcggga 52500
atgacgaagt ggaagttacc cgaaacttaa aacaagtgaa accgaacgaa ccggattccc 52560
actttcgtgg gaatgacggg atgcaggttt ccgtacggat ggattcgtca ttcccgcgca 52620
ggcgggaatc tagacattca atgctaaggc aatttatcgg gaatgactga aactcaaaaa 52680
actggattcc cactttcgtg ggaatgacgg gattagagtt tcaaaattta ttctaaatag 52740
ctgaagctca acgcactgga ttcccgcctg cgcgggaatg acgaagtgga agttacccga 52800
aacttaaaac aagcgaaacc gaacgaactg gattcccatt gtcgtggaaa tgacgggatt 52860
ttaggtttct gtttttggtt ttctgttttc gtgggaatga cgggatgtag gttcgtggga 52920
atgacggttc agttgctacg catttaccct gcgcaaagct ttatccacta tcttgtaacc 52980
tgtctgacaa tctgtcctct cttacaaaat gccgaaactt tttcaggctg cattttgggg 53040

ctgcctgtgc ggaatttggc ggtaggcgcg gtagtagggt tcgagctgtc gggcgatgag 53100
ttggagctgt tggaggagga tgtggctttg tgttccgctg ctgtgggtgc ggagggtgtc 53160
gagttcgccg cgcagtgtat ccagtgctgt ctgaaagtcg tcgggttcgg tttcgggcag 53220
gtgttggaag atgtgggcgg tgtgttcggc ggcgaggtgg aactgtgcgg taaagtcggg 53280
gctgcattct tcgtgcattt cgctgcggta tgcgccgagg gcggagatgt agccggtcag 53340
ggcgtagccg gttttgagca gggtaaagcc gggttgcagg ctgtcggcga attttgcggg 53400
ttcgctgctc atgtcggaaa gggtgctgct gagggcggcg gtgtgttcgt gggcgcggcg 53460
gcgggtggcg cggtattcga cgtcgtcgcc ggtttcgccg cttttgaggc gttcggtgat 53520
tttttcgaga taggcaccgt tgctgcatac ggcaagggcg gcggtgcgtt cgagcgtgag 53580
gtatttccag tctggccaca ggtagctgac tgccgcccag gcaagggatg cgccgataat 53640
ggtgtcgatg atgcgtacgg gcatggcggc gtatacgtcc aaacctgcga gggagaggct 53700
ggtcagggct tgaatggtaa tgaagaaggt ggagaaactg tatttgtagg tgcgggtcat 53760
gaaaaagagg gtggtactgg cgatgacaat ccagagtttg gtttcgacag acggggtgaa 53820
gtaggggacg agcgagccga cgattacgcc gagtacggtg ccggcgatgc gctggcggac 53880
gcggcttttg gtggcggtgt agttgggttg gcagacgaaa agggcggtca gtagtatcca 53940
gtagccgagg ttgaggttga gggcttcgac gatggtgcag gcggcggcaa cgacgaggga 54000
caggcggacg gcatggcgga atacgcctga ttcgaggttt agctgcggac ggattgcctg 54060
ccaggtgttt ttgaggctgc tggtttcgag ggcggcgatg cgggtgtcgc ccatgcggtc 54120
gttttctgcc tgcaggccgt tgtgctggag ttggcggaac tgctggtcga cgctgccgag 54180
gttgtcgaga aggcggcgca ggtggcggat gtcgggactg tcgttgctgt ctgaaaggag 54240
gcgcagcgat tggcggcagc cttcgatggc gcggccgagg cgtttgctgt aaacgtagtc 54300
tttgcttgcg cgcagggctt gggcggtgtt gcggcaggct tgtccctgca tttcgagcag 54360
gcggtggatg cggaagatga tgtcggtgtt tttgaatttt tcggacattt cctgataatc 54420
gacgtgggcg gagctgatgc gttcgtgtat gtcttgggcg gcaaagtagt aacgcagcat 54480
tttggcggtg cgcgggtggc ggtgtttgcc gcgaaggcgg taaaacaggg cggaacggca 54540
ttggttgaag gcggtgatga cgccggtgtt gctcatggcg aggtcgatgt ggcggttgcc 54600
tatccaggct gcctcatcgg ggtcgaagaa gtcggctttg gcttcgaggt agccgccgag 54660
tgcgtcgtag gcgttggcga cgctttcttg gacggggcgg tggggcagga cgatttggaa 54720
caggaggatg gcggtgctgt acagtacggt gccgcataaa atcatgaagg ggttggtcag 54780
ccagtaggtt tcgggggtgt aggtaagtgt ggtgtaggtg gcgacggcga gtgcaccgaa 54840
ggcgaaggtg cggtatttga gcccgaccgc gcctaaaatg gtgaagccga aggtcatcag 54900
ggtcatggcg aggatgaagg gcagccctgt gccgagggtg ctttgtgccg tgagcgagga 54960
gagggtgaac agggcgacgg tggtgatgat gtttttcagc cgtccggtca ggcggttgtc 55020
caaatcgaca aggccgccgg cgatgatgcc gagtacgaag ggcatggcga gcttgggttc 55080
gcctagctgc cagacgatgg aggcggcggt aaaaacactg gcgaaaacgg gaagcgaggt 55140
aatgagcaga ggcttgagga gtggggtttt catggtttta ccggtttatt gttatgaagt 55200
gaatatagtg gattaacaaa aaccagtacg gcgttgcctc gccttagctg aaagagaacg 55260
attctctaag gtgctcaagc accaagtgaa tcggttccgt actatttgtg ctgtctgcgg 55320
cttcgtcgcc ttgtcctgat ttttgttaat ccactataaa tttaatccac tataaagtgt 55380
agcacatgaa tggggcggat aaaatcatgc cgtctgaaaa cggggatgcg gttttcagac 55440
ggcattgggt tttgcggatc aggaaatgag gttgagaccg ttgaccctgt cgtaaaggag 55500
ttcgggcgtt ttgccttctt tgtgcagttg gatgtgcaat cgcaggttgt tggcggaaac 55560
ggactggcgc agggcttctt cgtaactgat gatgccgtga cggtacagtt cgaaaaggtt 55620
ttgatccatc gtctgcattc cgtcggtttt ggcggtttcc atgattttac tgatgttcat 55680
caggtcgccc ttcaggatga agtcttggat ggcgggcgtg ttgatgagca agtcgacaac 55740
cgccgtcctg cccgttttgt cttgtttgag ggcgaggcgt tggcagatga tgccggtcag 55800
gttgagggcg atgtcgatca gtatttggtt gtgctgttct ttggggtaga agttgagtat 55860
gcgttcgagc gactgcggcg cggtgttggc gtggagcgta aaaatgcaca ggtggccggt 55920
ttgggcgagc tgcatcgcgt attccatact ttccctgctg cggacttcgc cgatgcagac 55980
cacgtcgggg gattggcgca tagcgttttg taccgccgtc tgccagttta tggtgtcgac 56040
gccgatttcg cgctgggtaa agatgcagcg gcgcggtttg tagataaatt caatcgggtc 56100
ttcgatggta acgatatggc tgggcagggt tttgttgcgg tgttcgagca tagtcgccat 56160
cgtggtggat ttgcccgaac cggtaggccc gacgataatc agcagcccgc gcggtgcgac 56220
ggcgaggtct ttgagttttt cgggcaggcc caattcctgc atttgcggga tgacgtggtt 56280
gatgcgccgc aaaaccaaac ctgcgctgcc ttggctgtgg taggcgttgg cgcggtagcg 56340
cgtgccgctg cgcgactgga cggagtagtt gatttcgccg tcgcgccgga atatttccga 56400
ttgttcggcg ttcatcgtcg atgcggcgat ggcggcggtt tcctcgcccg tcagcgcctt 56460
ttgcggctgc ggggttaatg cgctgttgat tttcaacgag ggcgggaatc ctttgctgat 56520
aaggatgtcg gacgcgtttt gtgcttctgc ggtttcgcac aggcggtcga gcagcgggtg 56580
gaagtgtgcg ccgatttcgg ccggggtttc ggatcggctt tgtttttttt gagaatacac 56640
ttgaaccatt tcgtccaaga tgtcgtgcag gttatcggta ttcatcgtta gcttcttttc 56700

ggtttaagcc ttgcagtttg cggcggcagg tttcaacagg aaggcggacg cttcttgttc 56760
ggaaaggtag ccgggcggga tgctgcgtcc cgccccgcgt gtttgcgcct tgttttcccg 56820
ccggtatggc cggaaagcgg ttgtgtgtca gaaactcata ctttcgctgt tttgcgcgcg 56880
tctgcgtgcg acttccggtg cgatcagccc ttggcgcacc agcgattgca gcgattggtc 56940
cattgtctgc ataccgctcg cctgcccggt ttgcaggacg gagttaatct gcgtgatttt 57000
gttttcgcgg atgaggttgc ggacggcggg gttggcaatc aggatttcgt gcgaggcgac 57060
acggccgttg ccgtcgtgcg ttttcagcag gttttgggag atgacggcgg tcagcgattc 57120
ggacagcata gagcgcacca tttctttttc tcccgccggg aatacgtcca caatacggtc 57180
gacggttttt gctgcgccgg tcgtgtgcag cgtgccgaaa accaagtgtc cggtttcggc 57240
ggcggtcagt gccaagccga tggtttctgg gtcgcgcatc tcgccgacaa ggataacgtc 57300
ggggtcttcg cgcaatgcgg aacgcagcgc gttggcgaag ctgagggtgt gctggtgcag 57360
ctcgcgctgg ttaatcaggg attttttgct ttggtggacg aattcaatcg ggtcttcgat 57420
ggtcaggatg tgtgccggct gggtttcgtt gatgtagttg atcatcgcgg caagcgtggt 57480
cgatttgccc gaaccggtag ggccggtaac caaaaccatg ccgcgcggcg attctgcgat 57540
tttttggaaa atgctcgggg ctttcaattc ttccagcgat aagacggtgc tgggaatggt 57600
gcggaatacg gcggcgggac cgcggccgat gttgaaggcg ttgacgcgga atcgggcgac 57660
gttgggcagt tcgaacgaga agtcgacttc caagttttgc tggtagattt tccgctggtg 57720
gtcgttcatc accgaagtta ccatattacc gacctcttcc gcgctcattt cgggaaggtt 57780
gatgcgccgc atatcgccgt gaacccgaat catagggggat atgcccgaac tcaggtgaag 57840
gtcggatgct ttgtttttag cgccgaaggc gagtaagtcg gtaatctgca taatgcggct 57900
ctgtttagta taatgtttcg attggttgga atggttctaa caaccttgat tgtaccgccc 57960
tgactggagg ggtttcaact gtttaatcat ttttaattag gggataatct atgacggtgt 58020
tgcaagaacg ttattgtgag gtgtccgacc gtatcggaaa attggttctg caggcgggca 58080
gggagccgca ttccgtcagc ctgattgccg tcggtaagac tttcccttca gacggcatcc 58140
gcgaagttta cgccgccgga cagcgtgatt tcggcgagaa ctatattcag gagtggtacg 58200
gcaaaacgga agagttggcg gatttgaccg acatcgtgtg gcacgtcatc ggcgatgtgc 58260
agtccaacaa aaccaagttt gtcgccgaac gcgcgcattg ggtgcatacc gtatgccgtc 58320
tgaaaaccgc cgtccggctg agcgggcaac gtccttcctc aatgccgcct ttgcaggtgt 58380
gtatcgaggt gaacattgcg ggcgaggcgg tgaagcacgg tgtcgcgccc gaagaagcag 58440
tcgcgcttgc tgtggaagtg gcgaagctgc cgaatatcgt cgtacgtgga ctgatgtgtg 58500
ttgccaaagc caacagcagt gaaacggagt tgaaggtgca atttcaaacg atgcggaaac 58560
tgcttgccga cctcaatgcg gctggcgtta aggcagacgt gctgtctatg gggatgtcgg 58620
acgatatgcc tgccgccatt gagtgcggtg cgacacacgt ccgtatcggc agcgcgattt 58680
tcgggaaaag gggctgatgg aaattcgggc aataaaatat acggcaatgg ctgcgttgct 58740
tgcatttacg gttgcaggct gccggctggc ggggtggtat gagtgttcgt ccctcaccgg 58800
ctggtgtaag ccgagaaaac cggctgccat cgatttttgg gatattggcg gcgagagtcc 58860
gccgtcttta ggggactacg agataccgct ttcagacggc aatcgttccg tcagggcaaa 58920
cgaatatgaa tccgcacaac aatcttactt ttacaggaaa atagggaagt ttgaagcctg 58980
cgggctggat tggcgtacgc gtgacggcaa acctttgatt gagacgttca aacagggagg 59040
atttgactgc ttggaaaagc aggggttgcg gcgcaacggt ctgtccgagc gcgtccgatg 59100
gtaaaaaatt gggaatgaat ttagtaaggt aattttgaat agggtagaaa taatgaatgt 59160
ttattttctc ggcggcggca atatggcggc tgccgttgcg ggcggattgg tcaaacaagg 59220
cggttaccgc atctatatag ccaatcgggg tgcggaaaaa cgcgaacgtt tggaaaaaga 59280
gttgggggtc gaaacttcgg caaccctgcc ggagcttcat tccgacgatg ttttaatcct 59340
tgccgtcaaa ccgcaggata tggaagctgc gtgcaaaaat atccgcacca acggcgcatt 59400
ggtgctttct gtcgcagccg gattgtcggt cggtacgctc agccgttacc tcgggggaac 59460
acgccgcatt gtccgggtta tgccgaatac acccggaaaa atcgggctgg gcgtatctgg 59520
tatgtatgcc gaagcggaag tatcggaaac agaccgcagg attgccgatc gaatcatgaa 59580
atcagtcggt ttgactgttt ggttggatga tgaggaaaaa atgcacggca ttaccggcat 59640
cagcggcagc ggaccggctt atgtgtttta tctgctggac gcattgcaaa atgccgccat 59700
ccgacaaggg tttgatatgg cagaagcacg cgcgctcagt ctggcaacgt ttaaaggagc 59760
ggttgccctt gccgagcaga cggtgaaga tttcgagaag cttcaaaaaa atgtaacgtc 59820
aaaaggcggg acaacccacg aagccgtgga agctttcagg cggcatcgtg tcgccgaagc 59880
cataagcgag ggcgtttgtg cctgtgtgcg ccgttcgcag gaaatggaac ggcaatatca 59940
ataatgtaaa gaaaataaaa aaaccaatcc aaaacgtgtt atgatgcgcg ttttcaaaaa 60000
cgccttaggc aataagcctt ataaaaatca aaggaataaa gccactttgt ggtgctttgt 60060
tttttgcggt gaaccgagag gatatacatt atggcaaagc tgacagaaca agatattttg 60120
aattggagcg ggccggaaga cgattatatg aatgacgacc atttggcttt tttccgcgaa 60180
ttgctggtaa aaatgcaaga cgaactcatc gaaaatgctt ccgctacgac agggcatctc 60240
caagaacacg aatcagcccc cgatcctgcc gaccgtgcca cacaggaaga agagtacgca 60300
ttggaactcc gtacccgcga tcgggaacga aaacttctca gtaaaataca ggcgaccatc 60360

cgcaatattg atgaagggga ttatggattc tgtgccgata cgggagagcc tatcggtttg 60420
aagcggctgc tggcacgccc gacagccact ttatctgttg agtcccaaga acgccgagag 60480
aggatgaaaa aacagtttgc cgactgatgg cggcaaacaa aatgccgtct gagtccccga 60540
gtttcagaca gcatattcac aaaggcgcac cagccggagg agggagagga aaggattgtt 60600
ggaggcggcg cagtatttag cagaaataaa aaaccttatc cgacagcgac atgacgaatt 60660
tccccaaaaa aatcccgctg aaagcattga ccgtttttcc ctgtgggcgt atagttcggt 60720
tcttcgctgc tgcagaagtg gcggacgaac tgaaaagtat agcacagaat gttggggata 60780
tcgagagata tcttgacagg cggaaggaat actttataat tcgcaacgct ctttaacaaa 60840
acagattacc gataagtgtg agtgccttga gtctcacact gtttgaaaga cagacaagat 60900
aatgttttga acattgtcct gttggtttct ttgaagcaga ccagaagtta aaaagttaga 60960
gattgaacat aagagtttga tcctggctca gattgaacgc tggcggcatg ctttacacat 61020
gcaagtcgga cggcagcaca gagaagcttg cttctcgggt ggcgagtggc gaacgggtga 61080
gtaacatatc ggaacgtacc gagtagtggg ggataactga tcgaaagatc agctaatacc 61140
gcatacgtct tgagagagaa agcaggggac cttcgggcct tgcgctattc gagcggccga 61200
tatctgatta gctagttggt ggggtaaagg cctaccaagg cgacgatcag tagcgggtct 61260
gagaggatga tccgccacac tgggactgag acacggccca gactcctacg ggaggcagca 61320
gtggggaatt ttggacaatg ggcgcaagcc tgatccagcc atgccgcgtg tctgaagaag 61380
gccttcgggt tgtaaaggac ttttgtcagg gaagaaaagg ctgttgctaa tatcagcggc 61440
tgatgacggt acctgaagaa taagcaccgg ctaactacgt gccagcagcc gcggtaatac 61500
gtagggtgcg agcgttaatc ggaattactg ggcgtaaagc gggcgcagac ggttacttaa 61560
gcaggatgtg aaatccccgg gctcaacccg ggaactgcgt tctgaactgg gtgactcgag 61620
tgtgtcagag ggaggtagaa ttccacgtgt agcagtgaaa tgcgtagaga tgtggaggaa 61680
taccgatggc gaaggcagcc tcctgggaca acactgacgt tcatgcccga aagcgtgggt 61740
agcaaacagg attagatacc ctggtagtcc acgccctaaa cgatgtcaat tagctgttgg 61800
gcaacctgat tgcttggtag cgtagctaac gcgtgaaatt gaccgcctgg ggagtacggt 61860
cgcaagatta aaactcaaag gaattgacgg ggacccgcac aagcggtgga tgatgtggat 61920
taattcgatg caacgcgaag aaccttacct ggtcttgaca tgtacggaat cctccggaga 61980
cggaggagtg ccttcgggag ccgtaacaca ggtgctgcat ggctgtcgtc agctcgtgtc 62040
gtgagatgtt gggttaagtc ccgcaacgag cgcaaccctt gtcattagtt gccatcattc 62100
agttgggcac tctaatgaga ctgccggtga caagccggag gaaggtgggg atgacgtcaa 62160
gtcctcatgg cccttatgac cagggcttca cacgtcatac aatggtcggt acagagggta 62220
gccaagccgc gaggcggagc caatctcaca aaaccgatcg tagtccggat tgcactctgc 62280
aactcgagtg catgaagtcg gaatcgctag taatcgcagg tcagcatact gcggtgaata 62340
cgttcccggg tcttgtacac accgcccgtc acaccatggg agtgggggat accagaagta 62400
ggtaggataa ccacaaggag tccgcttacc acggtatgct tcatgactgg ggtgaagtcg 62460
taacaaggta gccgtagggg aacctgcggc tggatcacct cctttctaga gaaagaagag 62520
gctttaggca ttcacactta tcggtaaact gaaaaagatg cggaagaagc ttgagtgaag 62580
gcaagattcg cttaagaaga gaatccgggt ttgtagctca gctggttaga gcacacgctt 62640
gataagcgtg gggtcggagg ttcaagtcct cccagaccca ccaagaacgg gggcatagct 62700
cagttggtag agcacctgct ttgcaagcag ggggtcatcg gttcgatccc gtttgcctcc 62760
accaatactg tacaaatcaa aacggaagaa tggaacagaa tccattcagg gcgacgtcac 62820
acttgaccaa gaacaaaatg ctgatataat aatcagctcg ttttgatttg cacagtagat 62880
agcaatatcg aacgcatcga tctttaacaa attggaaagc cgaaatcaac aaacaaagac 62940
aaagcgtttg ttttgatttt ttattctttg caaaggataa aaatctctcg caagagaaaa 63000
gaaaacaaac acagtatttg ggtgatgatt gtatcgactt aatcctgaaa cacaaaaggc 63060
aggattaaga cacaacaaag cagtaagctt tatcaaagta ggaaattcaa gtctgatgtt 63120
ctagtcaacg gaatgttagg caaagtcaaa gaagttcttg aaatgataga gtcaagtgaa 63180
taagtgcatc aggtggatgc cttggcgatg ataggcgacg aaggacgtgt aagcctgcga 63240
aaagcgcggg ggagctggca ataaagcaat gatcccgcga tgtccgaatg gggaaaccca 63300
ctgcattctg tgcagtatcc taagttgaat acatagactt agagaagcga acccggagaa 63360
ctgaaccatc taagtacccg gaggaaaaga aatcaaccga gattccgcaa gtagtggcga 63420
gcgaacgcgg aggagcctgt acgtaataac tgtcgagata gaagaacaag ctgggaagct 63480
tgaccatagt gggtgacagt cccgtattcg aaatctcaac agcggtacta agcgtacgaa 63540
aagtagggcg gggcacgtga aatcctgtct gaatatgggg ggaccatcct ccaaggctaa 63600
atactcatca tcgaccgata gtgaaccagt accgtgaggg aaaggcgaaa agaaccccgg 63660
gaggggagtg aaacagaacc tgaaacctga tgcatacaaa cagtgggagc gccctagtgg 63720
tgtgactgcg taccttttgt ataatgggtc aacgacttac attcagtagc gagcttaacc 63780
gaatagggga ggcgtaggga aaccgagtct taatagggcg atgagttgct gggtgtagac 63840
ccgaaaccga gtgatctatc catggccagg ttgaaggtgc cgtaacaggt actggaggac 63900
cgaacccacg catgttgcaa aatgcgggga tgagctgtgg ataggggtga aaggctaaac 63960
aaactcggag atagctggtt ctccccgaaa actatttagg tagtgcctcg agcaagacac 64020

```
tgatgggggt aaagcactgt tatggctagg gggttattgc aacttaccaa cccatggcaa 64080
actaagaata ccatcaagtg gttcctcggg agacagacag cgggtgctaa cgtccgttgt 64140
caagagggaa acaacccaga ccgccagcta aggtcccaaa tgatagatta agtggtaaac 64200
gaagtgggaa ggcccagaca gccaggatgt tggcttagaa gcagccatca tttaaagaaa 64260
gcgtaatagc tcactggtcg agtcgtcctg cgcggaagat gtaacggggc tcaaatctat 64320
aaccgaagct gcggatgccg gtttaccggc atggtagggg agcgttctgt aggctgatga 64380
aggtgcattg taaagtgtgc tggaggtatc agaagtgcga atgttgacat gagtagcgat 64440
aaagcgggtg aaaagcccgc tcgccgaaag cccaaggttt cctgcgcaac gttcatcggc 64500
gtagggtgag tcggcccccta aggcgaggca gaaatgcgta gtcgatggga aacaggttaa 64560
tattcctgta cttgattcaa atgcgatgtg gggacggaga aggttaggtt ggcaagctgt 64620
tggaatagct tgtttaagcc ggtaggtgga agacttaggc aaatccgggt cttcttaaca 64680
ccgagaagtg acgacgagtg tctacggaca cgaagcaacc gataccacgc ttccaggaaa 64740
agccactaag cttcagtttg aatcgaaccg taccgcaaac cgacacaggt gggcaggatg 64800
agaattctaa ggcgcttgag agaactcagg agaaggaact cggcaaattg ataccgtaac 64860
ttcgggagaa ggtatgccct ctaaggttaa ggacttgctc cgtaagcccc ggagggtcgc 64920
agagaatagg tggctgcgac tgtttattaa aaacacagca ctctgctaac acgaaagtgg 64980
acgtataggg tgtgacgcct gcccggtgct ggaaggttaa ttgaagatgt gagagcatcg 65040
gatcgaagcc ccagtaaacg gcggccgtaa ctataacggt cctaaggtag cgaaattcct 65100
tgtcgggtaa gttccgaccc gcacgaatgg cgtaacgatg gccacactgt ctcctcctga 65160
gactcagcga agttgaagtg gttgtgaaga tgcaatctac ccgctgctag acggaaagac 65220
cccgtgaacc tttactgtag ctttgcattg gactttgaag tcacttgtgt aggataggtg 65280
ggaggcttag aagcagagac gccagtctct gtggagccgt ccttgaaata ccaccctggt 65340
gtctttgagg ttctaaccca gacccgtcat ccgggtcggg gaccgtgcat ggtaggcagt 65400
ttgactgggg cggtctcctc ccaaagcgta acggaggagt tcgaaggtta cctaggtccg 65460
gtcggaaatc ggactgatag tgcaatggca aaaggtagct taactgcgag accgacaagt 65520
cgagcaggtg cgaaagcagg acatagtgat ccggtggttc tgtatggaag ggccatcgct 65580
caacggataa aaggtactcc ggggataaca ggctgattcc gcccaagagt tcatatcgac 65640
ggcggagttt ggcacctcga tgtcggctca tcacatcctg gggctgtagt cggtcccaag 65700
ggtatggctg ttcgccattt aaagtggtac gtgagctggg tttaaaacgt cgtgagacag 65760
tttggtccct atctgcagtg ggcgttggaa gtttgacggg ggctgctcct agtacgagag 65820
gaccggagtg gacgaacctc tggtgtaccg gttgtaacgc cagttgcata gccgggtagc 65880
taagttcgga agagataagc gctgaaagca tctaagcgcg aaactcgcct gaagatgaga 65940
cttcccttgc ggtttaaccg cactaaagag tcgttcgaga ccaggacgtt gataggtggg 66000
gtgtggaagc gcggtaacgc gtgaagctaa cccatactaa ttgctcgtga ggcttgactc 66060
tatcatttga agaacttcaa gagataaaag cttactgact gattcagtca ttaccgaata 66120
tattgattaa ggctttaccg atttgtaaca gtttaagttt ggcggccata gcgagttggt 66180
cccacgcctt cccatcccga acaggaccgt gaaacgactc agcgccgatg atagtgtggt 66240
tcttccatgc gaaagtaggt cactgccaaa cacccattca gaaaaccccc gattattcgg 66300
gggtttttgc tttgcccgga aaaaatgttt gctttgcccg gaaaaaatgt cggtgatggc 66360
gggacggcat ccgtacggtg tccggtcggg tttgcggagg aacggcttga aactttggga 66420
tattcatttt agaatgactc gttttatcgt cgcaagatgc ggtttattgt ttgcaaccct 66480
taaaggaaaa accatgaaga aaatgttcgt gctgttctgt atgctgttct cctgcgcctt 66540
ctcccttgcg gcggtaaaca tcaatgcggc ttcgcagcag gagttggagg cgctgccagg 66600
cataggccct gcggtgctgg cgaagctgaa ggatcaggct tccgtcggcg cgcccgcacc 66660
aaaaggccca gccaaaccag tgctgcccgc ggataaaaaa taaaataggg ggaagtctgc 66720
agccgcatca aatgccgtct gaacatgcgt tcgggcggcg tttttataac aaaaacactt 66780
catggcggtt ggttttatgc ctatctaagt ttttgtgtcg tgcatacctg aagatttcag 66840
acggcatcgg tttatgctgt ctgaaaagtg tattccgttt cagtttgtaa gctatggcag 66900
tctgtttgtc ttgtgttttg cgcaattgcc cttattttga gccgtgattt tattttgaat 66960
tagatgaaaa aatgagtaat caagattttt atgcgacgct gggtgtggca agaacagcta 67020
ccgatgatga gattaaaaaa gcctaccgga aattggcgat gaaataccat cccgaccgca 67080
atcctgacaa taaagaggcg gaagagaagt ttaaagaagt acaaaaggcg tatgaaactt 67140
tgtccgacaa ggaaaagcgc gctatgtacg accagtatgg tcatgcggcg tttgaaggcg 67200
gcggacaggg gggcttcgga gggtttggcg gatttggcgg tgcgcagggt tttgactttg 67260
gggatatttt cagccaaatg tttggaggcg gttcggggcg cgcccagcct gattatcagg 67320
gtgaggacgt tcaagtcggt atcgaaatca cgcttgaaga agccgcaaaa ggtgtgaaga 67380
aacgcatcaa tattccgact tatgaagcgt gtgatgtctg taacggcagt ggcgcgaaac 67440
cggggacatc cccggaaacc tgcccgactt gcaaaggttc gggtacggtg cacatccagc 67500
aggcgatttt ccgtatgcag cagacttgtc cgacctgcca cggtgcgggc aaacacatta 67560
aagaaccttg cgtcaaatgc cgtggcgcgg ggcggaataa ggcggtcaag acggtggaag 67620
tcaatattcc cgccggtatc gatgacgggc agcgtatccg tttgagcggc gaaggcgggc 67680
```

cgggtatgca cggtgcgcct gccggcgact tgtatgtaac cgtccgcatt cgggcgcata 67740
agattttcca acgcgacggt ctggacttgc attgcgaact gccgatcagt tttgccacgg 67800
ctgctttggg cggggagttg gaagtgccga ccttggacgg aaaggtcaag ctcaccgtcc 67860
ccaaagaaac ccaaaccggc aggaggatgc gcgtgaaggg taagggtgtc aaatctttac 67920
gcagcagcgc gaccggcgat ttgtactgcc atattgttgt cgaaacgcct gtcaatttga 67980
ccgaccgtca aaaagagctt ttggaagaat ttgagcggat ttctaccggc ttggaaaacc 68040
aaacaccgcg caagaaatcg tttttagaca agctgcgcga tttgtttgat tgattttaag 68100
gttcggaaac aagcagccgt atcggggaat ctccttgata cggctgtttt tatttgttta 68160
aaaatagttt ttattttcaa tggggtatga ggcagggtgg gataactgtt tttaactgtt 68220
ctttttaaaa cttgacatca tggcgtgatg ccaacaatat gtgaacgtct gttgtcaaag 68280
gaagaataat gaataaatct ttatccagtt cggtagaaga ataccgcgag ctgacgctcc 68340
gaggcatgat actcggtgca ttgatcactg taatttttac tgcgtccaat gtttacctcg 68400
gtttgaaagt cgggctgacc tttgcctcgt cgattccggc ggcggtgatt tcgatggcgg 68460
ttttaaagtt tttcaaaggc agcaatattt tggaaaacaa catggtgcag acccaagcct 68520
cggctgcggg tacgctttcg accatcatct tcgtcctgcc cggtttgctg atggcgggct 68580
actggagcgg tttcccgttc tggcagacga cgcttttatg tattgccggc gggattttgg 68640
gggtgatttt caccattcct ctgcgttacg caatggtggt gaaaagcgat ttgccttatc 68700
cggaaggtgt ggcggctgct gaaattttga aagtgggcgg tcatgaagaa ggggataacc 68760
gtcagggcgg cagcggcatc aaagagctgg cggccggcgg tgcgttggcg ggattgatga 68820
gcttttgcgc cggaggtctg cgcgtgattg ccgacagcgc gagttattgg tttaaaagcg 68880
gtacggcgat tttccagctg ccgatgggct tttcactggc attgttgggc gcgggctatt 68940
tggtcggact gacgggcggt atcgccatcc tgttgggcat ttcgattgct tggggcattg 69000
ccgtgccgta tttctcctca cacattccgc aaccttccga tatggaaatg gcggcgtttg 69060
cgatgaagct gtggaaggag aaagtgcgtt ttatcggtgc ggggactatt ggcattgcgg 69120
cggtttggac gctgttgatg ctgctcaagc cgatggtgga aggcatgaag atgtcgttca 69180
agagttttgg cggcggtgcg cccgctgcgg aacgcgccga acaggatttg tcgcctaagg 69240
ctatgatttt ttgggtgctg gcgatgatgt ttgttttagg cgtgtcgttt taccacttta 69300
tcggcgattc gcacattacg ggcggcatgg cttggctttt ggtggtcgtt tgcacgcttt 69360
tggcttccgt catcggcttt ttggtcgccg ccgcctgcgg ttatatggca ggtttggtcg 69420
gctcgtcttc cagcccgatt tccggcgtgg gcatcgtgtc cgtcgtcgtt atttcactgg 69480
ttttgctgct ggtaggcgaa tccggaggtt tgttggcgga tgaggctaac cgcaaatttt 69540
tgctggcact gactttgttt tgcggctcgg cagtaatctg cgtggcttcg atttccaatg 69600
acaacctgca agacttgaaa accggctacc tgctcaaagc cacgccttgg cggcagcaag 69660
tcgccctgat tatcggctgt atcgttggtg cgctggttat ttcgcccgtg ttggaactgc 69720
tttacgaagc ctacggcttt accggcgcaa tgccgcgcga aggcatggac gcggcgcagg 69780
ctttggcagc ccctcaagcg actttgatga cgaccatcgc gtcgggcatt ttcgcccaca 69840
accttgaatg ggtctatatc tttaccggta tcgtgattgg agcagtatta atcgtcgtcg 69900
atttggtgtt gaaaaaatca tcaggcggca aacttgccct gcccgtcctt gcggtcggta 69960
tgggtattta tctgccgccg tccgtcaata tgcccatcgt ggcaggcgcg gtgttggcgg 70020
cggtgttgaa acacatcatc ggtaaaaaag cggaaaaccg cgaaggccgt ctgaaaaacg 70080
ccgagcgcat cggaaccttg ttctccgccg gcctgattgt cggtgaaagc ctgatcggtg 70140
tgattatggc gtttattatt gccttctccg tgaccaacgg cggctcggat gcgccgctcg 70200
cgttgaatct gcaaaactgg gatgccgccg cttcttggct gggtttggcg ttcttcgtta 70260
ccgggatgtt tttctttgca cagcgcgtac tgaaggcggg caagtaggct gtcggaaaaa 70320
atgccgtctg aaacgttcag acggcatttt ttatcggtaa agcggaaggc ggagcttttc 70380
ggcttgcgcc cacgttttgc cggcaaggtc tttgggcgac agcagcggcg cggtttgaag 70440
cggccagcct atgccgactg tcgggtcgtt ccatattaaa acctgttcgg cttcaggctt 70500
gtaatagtcc gtgcatttat agacgaactc ggcttcatcg ctcagtacat agaagccgtg 70560
tgcgaaacct tcgggtaccc acagttggcg tttgttttct gcggacagaa tttcgcctac 70620
ccatttgccg aaagtggggg agtctttacg catatcgacg gccacgtcga atacttcgcc 70680
gacaaccacg cgtacgagtt tgccttgtgt gttttcagtt tgatagtgca ggccgcgcaa 70740
tacgcctttg ccggatttgg agtggttttc ctgcacgaag gtgcgttcgc agacttgggt 70800
tttaaaccac tcgtcgcgga aggtttccat aaaaaagccg cgcgcgtcgc cgaagacttg 70860
gggctcaagc agttttacgt caggaatggc ggtatcaatg atgttcatct ttttatcttt 70920
catctaaagg ccgtctgaaa agtttcagac ggcctcaaac attatttttt caacaggcgc 70980
agcaaatatt ggccgtattg gtttttcgcc atcgggcgcg ccaattcttc cagtttttca 71040
tcggaaagcc aaccgttgcg ccaagcgatt tcttcgaggc aggcgatgtg caggttttgg 71100
atattttgca cggtttggac gaatgaagcg gcttcgtgca ggctctcgtg ggtgccggtg 71160
tccagccacg cgaaaccgcg tcccaatatt tgaacggaga gcgagccgtc ttccaaatac 71220
atccggttga ggtcggtaat ttccaattcg ccgcgtgcgg acggtttgag ctgtttggcg 71280
aactcgacgg cgcggttgtc gtagaaatac aagccggtta ccgcccaatc ggatttgggc 71340

cgttgcggtt tttcttcgat ggaaacggcg cggaagtttt cgttaaattc aaccacgccg 71400
aaacgttcgg ggtttttgac ctgataagca aacacggttg cgccgtgcgt ttgcgctgcc 71460
gcctgtttca atgtttgcgt aaacgactga ccgtaaaaaa tattgtcgcc caaaaccaag 71520
caaacattgt cgttgccgat aaattcttcg ccgatgataa atgcctgtgc caagccgtcc 71580
ggactgggtt gcacggcata actgatggaa atgccgaaat cgctgccgtc gccaagcagg 71640
cgtttgaaag aggcgttgtc ttcaggcgcg gtaatcacca aaatatcgcg gattcccgcc 71700
agcatcaaaa ccgacaaggg gtaataaatc atcggtttgt cgtacacggg caggagctgt 71760
ttggatacgc cgcgcgtgat ggggtagagg cgcgtgccgc tgccgcctgc cagtatgatg 71820
cctttcatct tttctttctt cctttgcgat gggttttcag acggcattgc gtcgggatgc 71880
cgtctgaaaa ctattttcca gtacctaaac gttccaaacg atagctgccg ttcaatacat 71940
tttgccacca ggttttgttg tccagatacc attgcacggt tttgcggagg ccggactcga 72000
aggtttccaa aggcagccag cccaaatccc gcctgatttt ggctgcgtcg acggcgtagc 72060
gtacgtcatg gccggggcgg tcttgtacga aagtaatcaa atcttcataa cgcgccacac 72120
cggccggttt ttcgggagcg agttcttcca gcagggcgca gatggttttg acgacttcaa 72180
tattggcttt ttcattgtgg ccgccgatat tgtaggtttc gccgacaaca ccttcggtaa 72240
caacctgata cagtgcgcgc gcgtggtctt cgacaaacag ccagtcgcgg atttgcatac 72300
cgtcgccgta cacaggcagc ggtttgccgt caagcgcgtt cagaatcatc aaaggaatga 72360
gtttttccgg aaaatggtaa ggaccgtagt tgttggagca gttggttaca atggtcggca 72420
agccgtaagt acgcaaccac gcgcggacga ggtggtcgct ggacgcttta gaggcagagt 72480
aggggctgga cggcgcgtag ggcgcggttt cggtaaacaa atcgtccgtg ccgcctaaat 72540
cgccatagac ttcatcggtg gaaatatggt ggaaacggaa ggcttcgtgc tgttcagacg 72600
gcatttgttg ccagtaggcg cggctgcttc caagcagatt gaatgtgccg acgatattgg 72660
tttggataaa ctcgcctgcc gaaccgatag agcggtcgac atggctttcc gccgccaagt 72720
gcatcacggc atcaggccgg tattgcgcga atacgcggtc gagttcggcg cggtcgcaaa 72780
tatccacttg ttcaaaagca tagcgaggat tatcggctac ctcagtcaaa gattccaaat 72840
tgccggcata agtcagctta tcgacattga cgacagcgtc ccgggtgttt cggataatat 72900
gacggacaac ggcagaaccg ataaagcccg cgccgccggt aacaaggatt tttctcataa 72960
atttcagagg atagccaaaa aatataaaca gattatagca gacagaatgt gtgtttttca 73020
gataaagagg ccgtctgaaa acatctcttt cagacggcct gtatcaggtc aacttaatcg 73080
tcgtagccat tcggattatt actcacccag cgccatgagt cttccatcat ttgggttaaa 73140
tcacgctggg tttgccagcc gatttgcgcc tttgtatagg aagggtcggc atagaagcac 73200
gccaaatcac cggcacggcg cggtttgact tcatacggaa tcgtcaaacc cgaagctgct 73260
tcaaatgcgc ggatgatttc caacaccgaa gaagcgcggc cggagcctaa gttcagcaaa 73320
tgcgtgcctg ctacattact ttttgcctgc atagccgcga catggccttc tgccaaatcc 73380
atcacatgaa tatagtcacg catccccgtg ccgtcggggg tagggtagtc atcgccaaat 73440
accgccaatt gcggcagttt gcctgccgcc acttggcaga tataaggcaa caaattattc 73500
gggatgccgt ttggctgctc gccaatcaag ccgctttcat gcgcgccaat cggattgaaa 73560
taacgcaaca aaatcatgct ccagcgcgga tcggcttttt gaatgtcagt gagaatgcgc 73620
tcaaccatcg atttcgatgc gccgtaaggg ctggtggtgt cgcccggtgg catatcctcg 73680
gtataaggca ctttgcccgg atcgccataa accgtcgccg aagaactgaa cacaatgcta 73740
aacacgcccg cacgcgccat ttcttccgcc aacaccaagc tgccggaaac attattatca 73800
taatatttca tcggctcggc cacactttca cccaccgctt tcaagccggc aaaatgaatc 73860
accgaatcaa tgcggttttc cgcaaaaata cggcgcaaaa tctcacgatc gcggatatcg 73920
ccttgataaa acggaatctc ttggccggta atcgttttca agcgtggcag gatattgatg 73980
ctggaattgc ataggttatc caaaatcacg acttgatggc cgcttttcag caaagaaaca 74040
acggtatgcg agccgataaa accggtgccg ccggtaacga gaattttttt catagaataa 74100
aatactaaaa atactttgat agattgataa taatggttgt aaaatcttaa tgaaataatt 74160
atccctgaag tagcagtaga tttcttcaga ttttttttggt taagtatatt tgatatctaa 74220
ggtaaaatac tataatttta ttcatatggt gtagaattaa gggaaaatag tgaaaaaagt 74280
attactaatt gccagttatg actcgttcct taactcgggc tatgctgttg caaaagagat 74340
aaaagatgct caaattgata tttatatcca caaaagtcga gaaaacattc tttcaaatcg 74400
acagttatta gaatcaggga tagataaaga ccaagcaatt tttttttca tattgatgat 74460
tactttatta agaatatgca tcaatattat gacgcagtaa ttttatcggt tggaaatggg 74520
ttgttaaaaa ggttctttaa gcagaatgcg caattaaata ttgcttcaag gccattgatt 74580
attaccttgt ttccaggtgt agtattcggt gatcaggcaa gtattctatc tcgtatgggg 74640
gctgatattg ttttatataa taataagcat gattttagaa ttgcagagga atataagaaa 74700
caatataaat taagttgtca aaatatactt tatggttatc caatttttcg ccatgcttcg 74760
aaaggttgtc atggagagaa aatttacttt attgaccaag ttaaaatccc atttaaaaaa 74820
gaagaaagaa tttatacatt aaaaaaattg attgccttgg ctgaaaaata ccctgagaaa 74880
gaatttacta ttttgctaag ggttgcagat aaagatatta ctgtgcatca ggataaacat 74940
tcgtatatag agctggcaaa gcagtttcag ttgccgagta atttgacaat agagcgaaaa 75000

agtaccgcgc aagccttcca agaaatgggg tattgtttat cttattcatc tactatgctt 75060
tttgaagctg aatgtaaggg tatccctgtt ggtgttgttg cagacttagg cttttctaaa 75120
tcctatgcaa atcagcattt tttaggtagt ggggttttag tttattttga tcaaatagat 75180
ttcacttccc caaaaatagc agatccggat tggcttgatt gctatgctac taaaaaggtg 75240
attacaactg atgagtttaa taagctatta aagcaggttg tgccattgca acatgattac 75300
caagaatatt tatctgcagg aattcgatat caagctttgg ctaacacaca cgccattcca 75360
accaatagtt ttctcggcat aaagccatgc tctgacgctt aaatgcacta atgccttaaa 75420
aaaacattaa agtctaacac actagactta tttacttcgt aattaagtcg ttaaaccgtg 75480
tgctctacga ccaaaagtat aaaacccttta agaactttct tttttcttgt aaaaaaagaa 75540
actagataaa tctctcatat cttttattca ataatcgcat cagattgcag tataaattta 75600
acgatcactc atcatgttca tatttatcag agctcgtgct ataattatac taattttata 75660
aggaggaaaa aataaagagg gttataatga acgagaaaaa tataaaacac agtcaaaact 75720
ttattacttc aaaacataat atagataaaa taatgacaaa tataagatta aatgaacatg 75780
ataatatctt tgaaatcggc tcaggaaaag ggcattttac ccttgaatta gtacagaggt 75840
gtaatttcgt aactgccatt gaaatagacc ataaattatg caaaactaca gaaaataaac 75900
ttgttgatca cgataatttc caagttttaa acaaggatat attgcagttt aaatttccta 75960
aaaaccaatc ctataaaata tttggtaata taccttataa cataagtacg gatataatac 76020
gcaaaattgt ttttgatagt atagctgatg agatttattt aatcgtggaa tacgggtttg 76080
ctaaaagatt attaaataca aaacgctcat tggcattatt tttaatggca gaagttgata 76140
tttctatatt aagtatggtt ccaagagaat attttcatcc taaacctaaa gtgaatagct 76200
cacttatcag attaaataga aaaaaatcaa gaatatcaca caaagataaa cagaagtata 76260
attatttcgt tatgaaatgg gttaacaaag aatacaagaa aatatttaca aaaaatcaat 76320
ttaacaattc cttaaaacat gcaggaattg acgatttaaa caatattagc tttgaacaat 76380
tcttatctct tttcaatagc tataaattat ttaataagta agttaaggga tgcataaact 76440
gcatccctta acttgttttt cgtgtaccta ttttttgtga atcgataccg tcgacctcga 76500
ggggggggcc ggtacccaat tcgccctata gtgagtcgta ttacgcgcgc tcactggccg 76560
tcgttttaca acgtcgtgac tgggaaaacc ctggcgttac ccaacttaat cgccttgcag 76620
cacatccccc tttcgccagg caaaaaaccg gttatatttt tttgcattaa atattttttt 76680
agcatattca ggaaagggga catgcaatat gtcaaaatga tctatatatc ctttaatatt 76740
aagattattt ccaatcaaat aacgttctaa ttttgttgga tgatatgaaa atgattctaa 76800
taaaggagca tatgttccag tcccttcatc aattaaatga gtcgtaatat tcttttttttt 76860
tgcaatacta atcagatagg agtagtggcc tgtaaaagac agcatataga gatgagcagg 76920
ctgtataata ttaaggattt ttttgtaact tctataaata taaagtaatt ttttaggagt 76980
tatattatta gggcttctag gaagctcaaa tagataaata gattcaaata gattcttgtt 77040
agctgattga tgaactaact taggcatttt taagttttta gaagtatata aaattactag 77100
taaattattg gttaattttt gtattttaat taggctttgg acttggttaa gctgacctaa 77160
attagatatg acaaataaat tgttacgtgg gggggtaaga taaaatggag atgttgtcaa 77220
ccacattgaa tcttgaaaaa actttttagg ctgaaaaaga gcttttttta ttttctttag 77280
cattattgta tctcttaaaa attaatgaga attagctata tgtaatagcc aatcctctgt 77340
taataaagta actaagttaa taagcattat tcaatatcag ttttttttgat ttgagcacct 77400
ttgcgaatat tgcaagcagc gaccttacca aataatgttt catattcgtt gacgctgaag 77460
tctccattgc ctgggcgttt aacccatagg ttatctccgg acaacagttc tccttttttta 77520
atgtctttat ctgctacgac agatgcaaag gcgaaatctt tagttggctt ttctcccgcg 77580
ataatcgtgt cttttttgcc gccgcgtgcc aattttaaag catgagcgcc ttgcttgagc 77640
tctttaaaag tatccggatt catagagcat acaatatccg gacctgggcg atccatgcgg 77700
tcagtaaagt gacgctctaa aatcgaaccg cctaaagcta ctgctcctaa gcaagcatag 77760
ttatctaagg tatggtcaga caggccaatg attgcgtctg gaaaggcttc agataaatcg 77820
ttcataccac ccaatcgaac atcttcgtaa ggggttgggt agatgttggt acagtgaagc 77880
aaagcataag gtacccctgc ttctcgaata atttctaccg actttttgat gctttcaata 77940
gaattcatgc cggtagagag aataataggc ttaccaaaag aggccaccag tttaattaat 78000
gggtagttat tacattcgcc agagccgatt ttatatgctg gaatatccat acgttgtaat 78060
cgtaaagcag ctgcacgaga gaaaggagta ctgataaaaa tcataccctt actctctacg 78120
tattctttta atttaatctc atcttcttca ttcagggcgc aacgttccat aatttcataa 78180
atagagacat ctgcattgcc tggaatgact tgtttggcct catcagacat ttcgtcttca 78240
acgatgtgtg tttgatgttt aacaacttca gcgcctgcat tataggcagc atcaaccatt 78300
tcaaaagctg tttttaaaga gccttcatga ttgatgccga tttcacagat aatcaatggt 78360
tcgtggttgt aacctactga acgattacca attttaaatt cgttgttgtt ttgcatttag 78420
ctttccttgt gattaagaat gttttctgcc tgttgtaaat caagctcagt atcaatatcg 78480
atagagtctt gatgagacat aatataaagt ttggttgggg cgataaaaaa acaattattt 78540
gcaattagtg aagcagtatc attaatgtaa attgcaccat taggcctaaa tgcctgaggt 78600
aattgttggc gaggctgctc caaatcgctt agatggcgca tgggggcata ttcgccatta 78660

ttgatttgaa gcagggtttt tagtggatga tgctccattg ggcatgcaga gacaacggat 78720
ccttttattt tctcatcaaa tagagaaaaa gcttcacgaa tatgagcccc tgtgcgtaat 78780
ggactggttg gttgtaatag ggttactgtg ccggaattac tgccaattgt ttctaaagca 78840
tgtattacac ctgaaataga gctggctgta tcggaggcca gctctgcagg gcgtaggacg 78900
acttcgacac cgaaattttt agcttcttct gcaattaacc cgccatcagt cgaaacaatt 78960
atgcggtcaa aacactttga tgatatagca gcattaattg tatgaccaag taatgatatg 79020
ccattcattt tccggagatt ttttaatggc aatcctttgg agttttggcg cgcaagtata 79080
accgcaatat tttgtttttc cataatttaa agattcaaat cgataaaacg tttttgagca 79140
gaaacattcc acgtttcagg attgttgatt acttcagcaa atctttctgt gctggtgcga 79200
gtatctccgc cattaaaggt atcatctgct tcaaatttgc ctaaactgca tgcttgttga 79260
atcgcatcaa agatattttt agtttcataa tctgtatgaa taatagattt tcccatatgg 79320
cggttacttt ggcgtgtacc aacatcaatt gaagggacac cgtagagagg agcttctcta 79380
atacctgcac ttgagttgcc gaccataaat ttagcatgtt tcaataagac taaaaaatat 79440
tcaaatcgaa tggaaggaaa tgcaataaat ttatcagatt gatattttaa taattcttgc 79500
agaatacttt cagtgccagt gtcattatta gggtagatgc taatgatatt ttggccactt 79560
aattctaatg ctttgaaata ttgggccgca tattgtggca ttaaatgtgc ttctgtagtc 79620
acggggtgaa acatagaaat accataattt tcgtatggta aaccgtaata ttctttgact 79680
tcttctaagg atgggagggt ggaagaggcc ataacatcta aatcggggga gccgatgatg 79740
tgaatatgct ttcttttttc tcccatttgc actaggcgag tgacagcttg ttcatttgct 79800
accaagtgga tatgagaaag tttactaata gaatgacgaa tggagtcatc tactgtacca 79860
gatagttcac caccttcgat atggcaaact aaacggctgc ttaatgcacc tacagctgcg 79920
cctgctagtg cttctaaacg gtcgccgtga atcatgacca tatcaggttc aatttcatca 79980
gatagacgag agataaacgt aatggtattg cctaaaacgg cacccattgg ttcaccttgg 80040
atttgatttg aaaacagata tgtatgttga tagttttctc gagttacttc cttgtaggtt 80100
ctgccatatg ttttcatcat atgcatacca gttacaatca aatgcaattc aaggtctggg 80160
tgattttcaa tataggctaa taaaggtttt agcttgccga agtcggctct ggtacctgta 80220
atgcaaagaa ttcttttcat gattttagaa tctataagta tataagtata aggaagttgg 80280
aaagaagaat actaattata ctctacgtac tcataaattt atttcgatta agtgctataa 80340
ttaggccatt tataattata ttaggatttg gcttgtgttt aaagtgaaat tttatattcg 80400
tcacgcagta ttattattgt gtggaagttt aattgtagga tgctctgcga ttccttcatc 80460
aggccccagc gcaaaaaaaa ttgtctcttt agggcaacaa tctgaagttc aaattcctga 80520
agtggagctg attgatgtga atcatacggt tgctcagtta ttatataagg ctcagataaa 80580
tcagtcattc actcagtttg gcgatggtta tgcttcggct ggtacgctaa atattggtga 80640
tgtattggat attatgattt gggaagcgcc gccggcagta ttgtttggtg gtggcctttc 80700
ttcgatgggc tcgggtagtg cgcatcaaac taagttgcca gagcagttgg tcacggcacg 80760
tggtacggtt tctgtgccgt ttgttggcga tatttcggtg gtcggtaaaa cgcctggtca 80820
ggttcaggaa attattaaag gccgcctgaa aaaaatggcc aatcagccac aagtgatggt 80880
gcgtttggtg cagaataatg cggcgaatgt gtcggtgatt cgtgctggga atagtgtgcg 80940
tatgccgctg acggcagccg gtgagcgtgt gttggatgcg gtggctgcgg taggtggttc 81000
aacggcaaat gtgcaggata cgaatgtgca gctgacacgt ggcaatgtag tacgaactgt 81060
tgccttggaa gatttagttg caaatccgcg acaaaatatt ttgctgcgtc gcggtgatgt 81120
ggttaccatg attaccaatc cctatacctt tacgtctatg ggtgcggtgg ggagaacaca 81180
agaaatcggt ttttcagcca gaggcttatc gctttctgaa gccattggcc gtatgggcgg 81240
tttgcaagat cgccgttctg atgcgcgtgg tgtgtttgtg ttccgctata cgccattggt 81300
ggaattgccg gcagaacgtc aggataaatg gattgctcaa ggttatggca gtgaggcaga 81360
gattccaacg gtatatcgtg tgaatatggc tgatgcgcat tcgctatttt ctatgcagcg 81420
ctttcctgtg aagaataaag atgtattgta tgtgtcgaat gcgccgttgg ctgaagtgca 81480
gaaatttttg tcgtttgtgt tctcgccggt taccagtggc gcgaacagta ttaataattt 81540
aactaattaa tgtgagtaat taagatgtct gagcaacttc ctgtggcagt tgccactgaa 81600
accaaagccg agcgtaaaaa gccgaaaaag aaaagttgga ttaaaaagct aagcccttta 81660
ttttgggtaa cggtgattat ccctacggta atttcgttgg tgtatttcgg cttcttcgct 81720
tccgatcgtt ttacgtcgca atcgagcttt gtggtgcgct cgcctaaaag ccaatcttct 81780
ctcaatggcc tgggtgccat tttgcagggc acaggttttg cccgtgcgca agatgatatt 81840
tacacggttg ggagtatat gcgttcgcgc tcgtctttgg atgaactgcg taaaatcttg 81900
ccggtgcgtg agttttatga aaccaaaggt gatgcgttca gccgctttaa tgggtttggg 81960
ttccgtggcg aggaagaggc tttttatcaa tactataaaa atcaggtgat gatcaatttt 82020
gatacggttt cgggtatttc cacgttgaat gtaacttcct ttgatgcgct ggaatctaag 82080
aaaatcaatg aggctttgtt aaaacaaggt gaagcattga ttaaccagtt gaacgatcgt 82140
gcacgtgctg atacggtgcg ctatgcggaa gaagtagtga aaacggcggc agagcgggta 82200
aaggaagcct ctcagaatct gacggattac cggattgcca atggcgtttt tgatttgaaa 82260
gcgcaatcgg aagtgcaaat ggggttggtt tccaagctgc aagatgaatt gattgtgatt 82320

caaacccagc tggatcaggt gaaagcagtc actccggaga atccgcagat tccgggtttg 82380
caggcgcgtg agcagagctt gcgtaaagaa attgaccaac agttacgtgc catttcgggc 82440
ggtgggcatt cttcgttgtc taatcaggct gccgaatatc agcgtgtgta tttggaaaac 82500
cagttggcag agcagcagtt ggcagccgcc atgacttctt tggaaagtgc caaggttgaa 82560
gcagaccgtc agcagcttta tttggaagtg atctcgcaac cgagcctgcc ggatttggca 82620
catgagccta aacggttata caacattgtt gccactctga ttatcggctt gatggtttat 82680
ggtattttga gcctgttgac tgccagcatt cgtgagcata aaaactgatg aaagccttgc 82740
ataaaacatc attttgggaa tctttagcca ttcaaaggcg cgtaatcggt gcgctgttga 82800
tgcgggaaat tatcacccgt tacggtcgca ataatattgg ctttttatgg ctgtttgttg 82860
agccgttgct gatgacattc gttatcgtct tgatgtggaa atttttaagg gcagaccgat 82920
attcaacttt gaatattgtc gcatttgcga ttactggcta tccgatgttg atgatgtggc 82980
gtaatgcctc aaaacgggca gttgggtcga tttcttcaaa tgccagcttg ctttatcacc 83040
gcaatgtaag agttttggat accatcttgg cgcgcatgat tttggaaatt gctggtgcaa 83100
ccattgcgca gattgtgatt atggcggtat tgattgcgat tggctggatt gaaatgccgg 83160
cagatatgtt ttatatgctg atggcttggc ttttgatggc tttttttgcg attggtttgg 83220
gtttggtgat ttgttcgatt gcctttaatt tcgagccgtt tggcaagatt tggggcacat 83280
tgacttttgt gatgatgccg ttatccggtg cgttcttttt tgtgcataat ttgccgccca 83340
aggtacaaga atatgcatta atgattccga tggtgcatgg cacagaaatg ttccgtgccg 83400
gatattttgg cagcgatgta attacctatg aaaatccttg gtatatcgta ttgtgcaatc 83460
tggtgttgtt gttgtttggc ttggcgatgg tcagtaaatt cagtaaagga gtcgagccgc 83520
aatgatttca gttgaacacg tttccaaacg ctatctgacc cgccaaggtt ggcggacagt 83580
cttgcacgat attagcttca aaatggagaa gggcgagaaa atcggtattc tcggccgcaa 83640
cggtgcaggt aaatcgacgc tcatccgttt gatcagtggc gttgagccgc cgaccacggg 83700
tgaaatcaag cggacaatga gtatttcttg gcctttggca ttctccggtg cgtttcaagg 83760
cagtctgacc ggtatggaca atttgcgttt catctgccgg atttacaatg tcgatatcga 83820
ttatgtgaaa gcgtttacgg aagaattttc ggagctgggg caatatttgt atgagccggt 83880
gaaacgctat tcttcaggta tgaaagcgcg tttggctttt gcgctgtcgt tggcggtgga 83940
gtttgactgt tacctgattg acgaagtgat tgcagttggt gactcgcgtt ttgccgataa 84000
atgtaagtac gagttgtttg aaaagcgcaa agaccgttcc atcatcttgg tgtcgcacag 84060
ccacagcgcc atgaagcaat attgcgataa tgcgatggtg ctggaaaaag ggcatatgta 84120
ccagtttgaa gatatggaca aagcctacga atattataat tcgctgcctt aaagcgattg 84180
tttttaaatc aggccgtctg aaatttcaga cggcctgtcc gttggaattc tattgatgaa 84240
cattactcaa attctttccc aagaactctc cgcgactgcc gcgcaaatca ccgccgccgt 84300
cgagcttttg gacgacggcg cgaccgtgcc gtttatcgcc cgctaccgca aggaagcgac 84360
gggcgggttg gacgatacgc agttgcgccg gcttgccgag cggctgcaat atctgcgcga 84420
gttggaagag cgcaaagccg ttgttttaaa aagcattgaa gagcaaggca agctttcaga 84480
cgacctcagg gcgcaaatcg aagccgccga taacaaaacc gcgctggaag acctgtatct 84540
gccctacaaa cccaaacgcc gcaccaaagc gcaaatcgcg cgcgaacacg gtttgcagcc 84600
gctggcggac gtgttgcttg ccgagcagtc gcaggacgtg gaagccgccg cacaaggcta 84660
cctgaacgaa aacgtccccg atgccaaagc cgcgttggac ggcgcgcgtg cgattctgat 84720
ggagcagttt gccgaagacg cggaacttat cggcacgctg cgcgacaagc tgtggaacga 84780
agccgaaatc cacgcgcaag tcgttgaagg caaagaaacc gaaggcgaaa aattcagcga 84840
ttatttcgac caccgcgaac ccgtccgcac tatgcccagc caccgcgcgc tggcggtttt 84900
gcgcggccgc aacgaaggcg tgttgaacat cgcgctcaaa taccagcccg acgacacgcc 84960
gattacccgg caaagcgaat acgagcaaat catcgcctgc cgcttcaagg tttcagacgg 85020
ccacaaatgg ctgcgcgata ccgtgcgtct gacttggcgc gcgaaaatct ttttgtcgtt 85080
ggaacttgaa gccctaggcc gtctgaaaga agccgccgac accgacgcga ttaccgtgtt 85140
cgcccgcaat ctcaaagact tgctgctcgt cgcgcccgcc ggacggctga ccacgctggg 85200
tctcgacccc ggctaccgca acggcgtgaa atgcgccgtg gtggacgaca ccggcaagct 85260
gctggatacc gtcatcgtct atttgcatca agaaaacaat atgttggcaa cgctgtcgcg 85320
cctgattaag caacacggcg tgaagctcat cgccatcggc aacggcaccg ccagccgcga 85380
aaccgacaaa atcgcgggcg aactggtgcg cggaatgccg gaaatggggc tgcacaaaat 85440
cgtcgtgtcc gaagccggcg cgtcgattta ttccgcgtcc gaactggcgg cgcgcgagtt 85500
ccccgacttg gacgtttccc tgcgcggcgc ggtgtccatc gcccgcaggc tgcaagaccc 85560
gcttgccgag ttggtcaaaa tcgaccctaa atccatcggc gtgggccagt atcagcacga 85620
tgtgaaccaa aaccagctcg ccaaatcgct ggacgcagtg gtcgaagact gcgtgaacgc 85680
cgtcggcgtg gacgtgaata ccgcctccgc cccgctcttg gcgcggattt ccggcttgaa 85740
tcaaaccctt gcccaaaaca tcgttgccta ccgcgatgaa aacggcgcgt tcgacagccg 85800
caaaaaattg ctgaaagtac cgcgtttggg cgaaaaaacc ttcgagcagg cggcaggctt 85860
tttgcggatt aacggcggta aagagccgtt ggacgcgagc gccgtccacc ccgaagccta 85920
tcccgtcgtc gccaaaatgc tggcgcaaca aggcattagc gccgccgaac tcatcggcaa 85980

ccgcgagcgc gtgaagcaaa tcaaagcgtc cgacttcacc gacgaacgct tcggcctgcc 86040
gaccattttg gacatcctgt ccgaactgga aaaacccggc cgtgatccgc gcggcgagtt 86100
tcagacggca tcgtttgccg aaggtatcca cgaaatcagc gacttgcaag tcggtatgat 86160
actcgaaggc gtggtttcca acgtcgccaa cttcggcgcg ttcgtggaca tcggcgtcca 86220
tcaggacggc ttggtgcaca tctccgccct gtccaacaag ttcgtccaag acccgcgcga 86280
agtggtgaaa gctggcgacg tggtgaaagt gaaagtgctg gaagtcgatg ctgcacgcaa 86340
acgcatcgcg ctgaccatgc gcttggatga cgaaccgggc ggcgcaaaac ataaaatgcc 86400
gtctgaaaac cgcagccgcg aacggacagc cggccgcaaa ccccaacgca acgaccgcgc 86460
cccagccaat tcggcgatgg cggatgcgtt tgcgaagctg aagcggtaaa ataatcgaag 86520
agtttatgga ttttgactta tgcacacacc acttacctat attgaccttt tctcaggagc 86580
aggaggccta tccttgggtt ttgaacaagc cggattccaa caattgcttt ctgttgaaat 86640
ggagtctgat tattgtcaga cttaccgtac caacttcccc catcatcaat tactgcaaaa 86700
agatttaacc acactaaccg aacaagattt aatcaattgt cttaacggac aagcagttga 86760
tttgattatt ggaggaccac cttgtcaagg ttttagtatg gcaggaaaga ttggacggac 86820
atttacagat gacccacgca accatttatt taaagagttt gtccgaatag ttaaaattgt 86880
ccaaccatat ttttttgtta tggaaaatgt agcgcgactc tatacacaca attcaggtaa 86940
aacacgtatt gagattattc aagcatttca gaatatcggt tattcggtgg aatgtaagat 87000
actgagtgca gccgatttcg gtgttcctca gatacgtagc cgagtgatat ttatcgggag 87060
gagggataaa ggcaaaattt cctttcccga acctttgcag atttcccatc agactgttgg 87120
atcagcaata ggacattttc caaaactggc tgctggcgaa agcaatccac acgttgcaaa 87180
tcatgaagct atgaatcatt cggcacaaat gttagaaaaa atggcatttg ttaaaaatgg 87240
aggtaaccgt aacgatattc ctgaaccatt acgtccgaaa acaggtgata tccgtaaata 87300
catccgttac aacagcaaca aaaccagccg tttgtattac aggagatatg cgcaaagttt 87360
ttcactatga acagaatcgg gcgttaaccg ttcgtgaatt agctgcctta caatctttcc 87420
ctgataattt tattttttgc ggcagcaaaa ttgcccagca gcagcaggtt ggtaacgccg 87480
taccgccttt attggcaaaa gctattgctg aaagtatttt aaaaatgagt gaaaatgaat 87540
aagcaatatc cgaaaattaa ctatatcggt aataaagaga aaatagcttc ctggatttgt 87600
gaccagcttc cgtctgatgt agatacagtt gcagatgtat ttagtggagg ctgttccttt 87660
gcctacgaag ccaaaaaacg cggctatcgt gtgattacta acgatatttt ggcaattaat 87720
taccaaattg ctttagcatt aatagaaaac aaccatgaaa cattaaatga cgatgatgtc 87780
gcaatgattt tttcaggcag cccgcatgcc ggttttatga gtcagcgtta tgccgaaaaa 87840
ttctattttc acgatgaata ccaacaactt gatttgtaac gtaaaaatat agggaaactg 87900
gataaccagt ataaacgcgc tttggcgttt actttaatgc gtcgcgccat gatacgtaaa 87960
atgccctata cggaagatat gcgcccaggc gataccgcta atccttatgg tgcgtccaaa 88020
gcgatggtgg aacggatgtt aaccgacatc caaaaagccg atccgcgctg gagcatgatt 88080
ttgttgcgtt atttcaatcc gattggcgcg catgaaagcg gcttgattgg cgagcagcca 88140
aacggcatcc cgaataattt gttgccttat atctgccaag tggcggcagg caaactgccg 88200
caattggcgg tatttggcga tgactaccct acccccgacg gcacggggat gcgtgactat 88260
attcatgtga tggatttggc agaaggccat gtcgcggcta tgcaggcaaa aagtaatgta 88320
gcaggcacgc atttgctgaa cttaggctcc ggccgcgctt cttcggtgtt ggaaatcatc 88380
cgcgcatttg aagcagcttc gggtttgacg attccgtatg aagtcaaacc gcgccgtgcc 88440
ggtgatttgg cgtgcttcta tgccgaccct tcctatacaa aggcgcaaat cggctggcaa 88500
acccagcgtg atttaaccca aatgatggaa gactcatggc gctgggtgag taataatccg 88560
aatggctacg acgattaagt tgacctgata caggccgtct gaaagagatg ttttcagacg 88620
gcctctttat ctgaaaaaca cacattctgt ctgctataat ctgtttatat tttttggcta 88680
tcctctgaaa tttatgagaa aaatccttgt taccggcggc gcgggcttta tcggttctgc 88740
cgttgtccgt catattatcc gaaacacccg ggacgctgtc gtcaatgtcg ataagctgac 88800
ttatgccggc aatttggaat ctttgactga ggtagccgat aatcctcgct atgcttttga 88860
acaagtggat atttgcgacc gcgccgaact cgaccgcgta ttcgcgcaat accggcctga 88920
tgccgtgatg cacttggcgg cggaaagcca tgtcgaccgc tctatcggtt cggcaggcga 88980
gtttatccaa accaatatcg tcggcacatt caatctgctt gaagcagccc gcgcctactg 89040
gcaacaaatg ccgtctgaac agcacgaagc cttccgtttc caccatattt ccaccgatga 89100
agtctatggc gatttaggcg gcacggacga tttgtttacc gaaaccgcgc cctacgcgcc 89160
gtccagcccc tactctgcct ctaaagcgtc cagcgaccac ctcgtccgcg cgtggttgcg 89220
tacttacggc ttgccgacca ttgtaaccaa ctgctccaac aactacggtc cttaccattt 89280
tccggaaaaa ctcattcctt tgatgattct gaacgcgctt gacggcaaac cgctgcctgt 89340
gtacggcgac ggtatgcaaa tccgcgactg gctgtttgtc gaagaccacg cgcgcgcact 89400
gtatcaggtt gttaccgaag gtgttgtcgg cgaaacctac aatatcggcg gccacaatga 89460
aaaagccaat attgaagtcg tcaaaaccat ctgcgccctg ctggaagaac tcgctcccga 89520
aaaaccggcc ggtgtggcgc gttatgaaga tttgattact ttcgtacaag accgccccgg 89580
ccatgacgta cgctacgccg tcgacgcagc caaaatcagg cgggatttgg gctggctgcc 89640

tttggaaacc ttcgagtccg gcctccgcaa aaccgtgcaa tggtatctgg acaacaaaac 89700
ctggtggcaa aatgtattga acggcagcta tcgtttggaa cgtttaggta ctggaaaata 89760
gttttcagac ggcatcccga cgcaatgccg tctgaaaacc catcgcaaag gaagaaagaa 89820
aagatgaaag gcatcatact ggcaggcggc agcggcacgc gcctctaccc catcacgcgc 89880
ggcgtatcca aacagctcct gcccgtgtac gacaaaccga tgatttatta ccccttgtcg 89940
gttttgatgc tggcgggaat ccgcgatatt ttggtgatta ccgcgcctga agacaacgcc 90000
tctttcaaac gcctgcttgg cgacggcagc gatttcggca tttccatcag ttatgccgtg 90060
caacccagtc cggacggctt ggcacaggca tttatcatcg gcgaagaatt tatcggcaac 90120
gacaatgttt gcttggtttt gggcgacaat attttttacg gtcagtcgtt tacgcaaaca 90180
ttgaaacagg cggcagcgca aacgcacggc gcaaccgtgt ttgcttatca ggtcaaaaac 90240
cccgaacgtt tcggcgtggt tgaatttaac gaaaacttcc gcgccgtttc catcgaagaa 90300
aaaccgcaac ggcccaaatc cgattgggcg gtaaccggct tgtatttcta cgacaaccgc 90360
gccgtcgagt tcgccaaaca gctcaaaccg tccgcacgcg gcgaattgga aattaccgac 90420
ctcaaccgga tgtatttgga agacggctcg ctctccgttc aaatattggg acgcggtttc 90480
gcgtggctgg acaccggcac ccacgagagc ctgcacgaag ccgcttcatt cgtccaaacc 90540
gtgcaaaata tccaaaacct gcacatcgcc tgcctcgaag aaatcgcttg gcgcaacggt 90600
tggctttccg atgaaaaact ggaagaattg gcgcgcccga tggcgaaaaa ccaatacggc 90660
caatatttgc tgcgcctgtt gaaaaaataa tgtttgaggc cgtctgaaac ttttcagacg 90720
gcctttagat gaaagataaa aagatgaaca tcattgatac cgccattcct gacgtaaaac 90780
tgcttgagcc ccaagtcttc ggcgacgcgc gcggcttttt tatggaaacc ttccgcgacg 90840
agtggtttaa aacccaagtc tgcgaacgca ccttcgtgca ggaaaaccac tccaaatccg 90900
gcaaaggcgt attgcgcggc ctgcactatc aaactgaaaa cacacaaggc aaactcgtac 90960
gcgtggttgt cggcgaagta ttcgacgtgg ccgtcgatat gcgtaaagac tcccccactt 91020
tcggcaaatg ggtaggcgaa attctgtccg cagaaaacaa acgccaactg tgggtacccg 91080
aaggtttcgc acacggcttc tatgtactga gcgatgaagc cgagttcgtc tataaatgca 91140
cagactatta caaccccaaa gccgaacact cgctgatttg gaatgatccg accgtcggca 91200
tcgactggcc gttgcaaggc gagccgaacc tgtcgcctaa agacttggca ggcaaagtat 91260
tgtctgaagc ggtaacgttt taaaaataat tcaggccgtc tgaaagaatg ttcctctttt 91320
cagacggcct acaatccatt aataacaata atcgacgaaa acgcattgtg aaaaacgcct 91380
acatcccctc tcgcggcatc cgcaaaatcc cccatctctc caccctattg cctgaatttc 91440
atatctgcaa agacgggaaa gaagcagagg ctgttgtcgg ctggggtttg cgcccgacga 91500
cacacaaagc gcgtgctttt gccgctgaac accagcttcc ctttattgct ttggaagacg 91560
gctttttacg atcgctcgga ctgggtgtcg ccggttatcc gccctactct atcgtctatg 91620
acgacatcgg catctactac gacaccacac gtccttcgcg tttggaacaa ctgattcttg 91680
ccgccgatac catgccgtct gaaaccttgg ctcaggcgca gcaggcgatg gatttcatcc 91740
tgcaacacca cctgtccaaa tacaaccacg cgcccgaact ttcagacgac catcctttac 91800
gttccccatc caaacccgaa accgtcctca tcatcgacca aaccttcggc gatatggcca 91860
tccaatatgg cggcgcagac gcctctacgt ttgaactgat gtttcagacg gccttaaatg 91920
aaaacccgca agccgatatc tgggtaaaaa cccatcccga tgttttgtgc ggcaaaaaac 91980
aaggctatct gacccaactg gcgcagcaac accgcgtcca tcttttggca gaagacatca 92040
atccgatttc tttgttgcaa aacgttgata aagtttattg cgttacctcg caaatgggtt 92100
ttgaggcgct tttgtgcggc aaaccgctga ccactttcgg cctgccgtgg tatgccggat 92160
ggggtgtaag cgacgaccgc catcctgaaa tcaaccgcct tgttcaaacc caacgccgcg 92220
ccacccgcaa cttgctgcag ctcttcgccg cagcctatct gcaatacagc cgctacctca 92280
accccaatac cggcgaagca ggcagcctct ttgatgtcat cgactatctg gcgacggtca 92340
aacgtaaaaa cgacaaattg cgtggcgagt tatattgcgt cggtatgtct ttgtggaaac 92400
gcgcggttgc caaaccgttc tttaacgtac cctcttgccg tctgaaattt atctcttcca 92460
cccaaaaact ggcaagggtc aaactgtccg acgatgcacg catcctggct tggggcaacg 92520
gcaaagaggc catcgtccgc tttgccgaac aacaccacat ccccctgctg cgcatggaag 92580
acggctttat ccgctcggtc ggactcggct ccaacttagt gccgccgctg tcgctcgtta 92640
ccgacgatat gagcatttat ttcaatgccg aaaccccgtc ccgtcttgaa tacatcctac 92700
aaaaccaaaa cttcgacgat caagactttc agacggcctt gaagctgcaa aaaatgctga 92760
ccgaaaacca catcagtaaa tacaacgtcg gcagctcaga cttcaccgcc ccgtcaaccg 92820
acaaaaccgt gatcctcgtt cccggccagg ttgaagatga tgcgtctatc cgctacggtt 92880
cgccccaaat ctaccgcaat ctggatttgc tccgtaccgt acgcgaacga aaccccaatg 92940
cctatatcat ctacaaaccg catcccgatg tagtcagcgg taaccgcatc ggccatattt 93000
cccctgaaga tgctgcacga tatgccgacc aaaccgccga acaagccgac atcctgacct 93060
gtctccaata cgcagacgaa atacatacca tgacttcgct gaccggtttt gaagccttgt 93120

tgcgcggcaa aaaagtcagc tgctacggcc tgcctttttta cgcaggctgg gggcttaccc 93180
aagatctgct ccccatcccg cgccgtagcc gcagacttga gctttggcag ctgattgccg 93240

```
gcacgctcat ccactatccc gactacatcc accccgaaac ccatcaggcc ataaatgcag 93300
aaaccgcagc ccaaatcctg atacgacaaa aaaatatgca aaaaaacaac aacggattac 93360
atcgcgggtg ctttgccaaa aaattaggta aaatcaaaca actatatcga tctttcaaat 93420
aaataccatc aaagttaacg atgcgtcata aacttgcctc tattgcggca tcattgcctt 93480
tgcatcgtta attctcttgg cgtatgcttg aaagttcaac ctaaaactat tacataaaaa 93540
acaaaaccac attgcaacat gaaacagacc gtcctcaaaa ataacctgca aaacctgctt 93600
gaaagcgcag aaaatatcct gctgcttcaa ggccctgtcg gcgatttttt tctgcgcctt 93660
gccgactggc tgactgcaaa cggcaaaacc gtacataaat tcaactttaa tgcaggcgac 93720
gactattttt atccgcccac tcaagcgcat accgttgttt ttaacgacaa ctacgatgcc 93780
tttcctgagt ttttgcaaga atacatcact caacatcaca tccaggccgt tgtctgcttt 93840
ggcgacacac gcccttatca cgtcattgca aaacgcattg caaacgaaaa ccaagccagt 93900
ttctgggcgt ttgaagaagg ctatttccgc ccctactaca tcaccttaga aaaagacggc 93960
gtcaacgcat tttccccgtt gccgcgccgt gccgactttt ttcttgaaca attccctaag 94020
cttgcccagc aagaatataa agcgccaacg ccggtacacg gcggttttac gcccatggca 94080
aaaaacgcta tccgttacta tatcgagttg ttccgcaatc cacgcaaata ccccgactac 94140
atccaccacc gcgcacccaa tgccggccat tacctcaaac cgtggtcgct ctccatcctc 94200
aagcgtttga actactatat tgaagacatc caaatcgcca aacgtgtgga agcaggcaaa 94260
tacggcaagt tttttattgt tcccttacag gtattcaacg acagccaagt ccgtatccat 94320
tgcgactttc ccagcgtccg cagcttcctg ctccatgttt tgagttcatt tgccgagcac 94380
gcgcctgccg ataccaacat catcatcaag catcatccga tggaccgcgg ttttatcgac 94440
tactggcgcg acattaaacg ctttatcaaa gaacaccccg aactcaaagg ccgtgtgatt 94500
tatgtccatg atgtccccct gcccgttttc ctgcgccacg gtctcggcat ggtcaccatc 94560
aacagcacca gcggcctgtc cggactgatt cacaatatgc cagttaaggt tctcggccgt 94620
gcctattatg atattcccgg cattactgac caaaatacct tggcagaatt ttggaatcat 94680
ccgacaccgc ctgacaaaga gctgttccat gcctaccgaa tgtaccacct caacgtgacc 94740
caaattaacg gcaacttcta cagtcaggtg tttttcccca acaaaaaaac ctccaactct 94800
tccacaccag taatctgact tagcgaagga agttcaggcc gtctgaaaac atttcagacg 94860
gcctgaaaca atcaatacct tagctactgc catgtaaata aaacacaaaa atctgcattt 94920
atcattaaca ataaattaca aaaacagtat aatgaccgag ctgccatgag cgcataccga 94980
ctcaacctga gccctttgta acacacaaaa tatggatata tccctaggca aaacaatata 95040
acaagccaaa catcctaaag ataagccggc aaggcaatac actctataaa actatgccga 95100
gcaaaatttt tacaaagccc tcaaccggta tcgccgccca tatgccgcag catccgtctt 95160
ccactttata tccgcccgca aaccatgacc gccgctcctg atatcctcta ccggcaagcc 95220
gccgcccttt tggaacaatc caataccgcc caagccctgc ccctgttgca acaggcggca 95280
gagcaaggtt atgcggaagc tgctttcgta ttgggcaacc atctgctgca aaacggccaa 95340
ccggagcagg cactttcatg gttggaagcc gccgcggccc aacgccatcc caaagcactc 95400
ttctccctgc tgcaacaacg cgaacacaac ggcaccccga ccggacagct tctcaacgac 95460
tatgcctggc tgggtgagca ggggcactca gaagcccaat taatcctcat gcgttaccac 95520
gcgcaacgca acgatccaca atcgctctac tgggcggaac ttgctgccgc ccgatatgcc 95580
gcacctgcgt attaccatct ggcacgccat catcaacgcc aaggcgacgt tgaaacagcc 95640
atcgaacaat acgaaaaagc ggcagcactc ggcgtaactg ccgcctgctg gcaacttggt 95700
caaatctact tctacggtac aggtgtcagc ccccaaccacg cacaagccga acactatctc 95760
gaaccagccg cacaagccgg ccacatcgcc gcacaaacgc tgctggctga ccttcttgcc 95820
gcccaacgca aacctgaagc cttggaatgg tatcgtcgtg ccgccgataa ggaacaagcg 95880
gaagcacagt ctaagctggc ccaatacgcc ctgaccggcg aactttccga acgcgatccg 95940
ttccaagcgg cacgatatgc caaagccgct gccgagaaaa accatcctga agccctgaaa 96000
atcatgggcg acctctaccg ctacggtctc ggtatcaaag ccgacaacca tatcgcgcaa 96060
gattactacc accgtgccgc cgcgctgggt tctgccgccg cagcacaaaa actcatcagc 96120
gacgccgcgc tgtaccatcc gcaacaatac gaacaaatca aaactgccgc ctgcaacaac 96180
aacaaaccga aaccatctac cgtttggcgg aagcacaagc ctgcgccatc ggccgtcccg 96240
ccgactacaa tgccgcgcga aaaaattaca tggaagctgc cgggttccac cataaaaacg 96300
cagcggcagc cttaggccgc atctaccatt acggcctcgg tacggcgcaa gatcctcggg 96360
cggctgcaca ctggtacgcc attgctgccg aacaaaacca cccttccgcc caataccacc 96420
tcgcctgttt ttactatcac gggcaaggtg tcggctgtca tgttccgacc gcctgctact 96480
ggctgcaggc cgccatcggc aacggccaca cttcggccga atcattaata tccctattag 96540
aacaatggcg acgcgaagca caccatgcca tcggacaaaa ggccgtctga aaagatttac 96600
actcgcattt tttgacaatc tttaactatt cccctaatat ttgccagtta tttttcacgg 96660
acacgccatt gttttcattt ctttctgaaa acaccttgtc cgcgcatcaa taccatgaca 96720
ctcggcggat aacgccaagc gttgaaacac actacatccg gaacaaaaac ggatgctcgg 96780
aaaaatattt ctaggaggtg aaacaacatg gaatgggaat tcaacagtta ttacacactg 96840
attgccgcca cgctcgtgtt gctggttggt aaatttctgg ttcaaaaaat caaattctta 96900
```

cgagacttca atattcccga gccggtagcc ggcggtttga ttgccgctat cgtcctgttc 96960
gccctgcacg aggcgtacgg cgtgagcttc aaatttgaga aaccgctgca aaatgcgttt 97020
atgctgattt ttttcacgtc catcggcttg agcgcggatt tttcccgttt gaaggcgggc 97080
ggtttgccgc tggtggtttt taccgcgatt gtgggcggat ttatcttggt gcaaaacttt 97140
gtcggggtcg gactggctac ggctttgggt ttggatccgc tcatcggtct gattaccggt 97200
tcggtgtcgc tgacgggcgg acacggtacg tcaggtgcgt ggggacctaa ttttgaaacg 97260
caatacggct tggtcggcgc aaccggtttg ggtattgcat cggctacttt cgggctggtg 97320
ttcggcggcc tgatcggcgg gccggttgcg cgccgcctga tcaacaaaat gggccgcaaa 97380
ccggttgaaa acaaaaaaca ggatcaggac gacaacgcgg acgacgtgtt cgagcaggca 97440
aaacgcaccc gcctgattac ggcggaatct gccgttgaaa cgcttgccat gtttgccgcg 97500
tgtttggcgt ttgccgagat tatggacggc ttcgacaaag aatatctgtt cgacctgccc 97560
aaattcgtgt ggtgtctgtt tggcggcgtg gtcatccgca acatcctcac tgccgcattc 97620
aaggtcaata tgttcgaccg cgccatcgat gtgttcggca atgcttcgct ttcgcttttc 97680
ttggcaatgg cgttgctgaa tttgaaactg tgggagctga ccggtttggc ggggcctgta 97740
accgtgattc ttgccgtaca aaccgtggtg atggttttgt acgcgacttt tgttacctat 97800
gtctttatgg ggcgcgacta tgatgcggca gtattggctg ccggccattg cggtttcggc 97860
ttgggtgcaa cgccgacggc ggtggcaaat atgcagtccg tcacgcatac tttcggcgcg 97920
tcgcataagg cgtttttgat tgtgcctatg gtcggcgcgt tcttcgtcga tttgattaat 97980
gccgcgattc tcaccggttt tgtgaatttc tttaaaggct gattttccgc ctttccgaca 98040
aagcacctgc aaggtttacc gcctgcaggt gcttttgcta tgatagccgc tatcggtctg 98100
caccgtttgg aaggaacatc atgtatcgga aactcattgc gctgccgttt gccctgctgc 98160
ttgccgcttg cggcagggaa gaaccgccca aggcattgga atgcgccaac cccgccgtgt 98220
tgcaaggcat acgcggcaat attcaggaaa cgctcacgca ggaagcgcgt tctttcgcgc 98280
gcgaagacgg caggcagttt gtcgatgccg acaaaattat cgccgccgcc tacggtttgg 98340
cgttttcttt ggaacacgct tcggaaacgc aggaaggcgg gcgcacgttc tgtatcgccg 98400
atttgaacat taccgtgccg tctgaaacgc ttgccgatgc caaggcaaac agccccctgt 98460
tgtacgggga aactgctttg tcggatattg tgcggcagaa gacgggcggc aatgtcgagt 98520
ttaaagacgg cgtattgacg gcagccgtcc gcttcctgcc cgtcaaagac ggtcagacgg 98580
catttgtcga caacacggtc ggtatggcgg cgcaaacgct gtctgccgcg ctgctgcctt 98640
acggcgtgaa gagcatcgtg atgatagacg gcaaggcggt gaaaaaagaa gacgcggtca 98700
ggattttgag cggaaaagcc cgtgaagaag aaccgtccaa acccacgccc gaagacattt 98760
tggaacacaa tgccgccggc ggcgatgcgg gcgtacccca agccgcagaa ggcgcgcccg 98820
aaccggaaat cctgcatcct gacgacggcg agcgtgccga taccgttacc gtatcacggg 98880
gcgaagtgga agaggcgcgc gtacaaaacc agcgtgcgga atccgaaatt accaaacttt 98940
ggggaggact cgataccgac gtgcaaaaag agttggtcgg cgaacaacgc aagtgggcgc 99000
aggaaaaaat cagcaactgc cgacaagccg ccgcgcaggc agaccggcag gaatacgccg 99060
aatacctcaa gctgcaatgc gacacgcgga tgacgcgcga acggatacag tatcttcgcg 99120
gctattccat cgattagggg caaaccgatg aataccgtcc caaaaagcag gattcccgtc 99180
aaaccgctgc ccgaaaaaac cacagacgaa gccaaagtcg aaaaatggcg gcagctcggt 99240
gcggaacacg gtttgtcggg cgaatgggca gttgccgtca gattgggcga aaacggtttt 99300
accgaagaac agatggaaaa tatcgccaac ctgttcggca gataaagaga aaattgacgg 99360
aaatgccgtc tgaaaccctg ttatcggttt cagacggcat tttgaccaat acggtacgca 99420
ggcgcaaaac agccggcttt tcctgtgttg cctatgctga tgtttcaaca cacaggacga 99480
tacaaaaaac gtcgccctat gtgccgtcct gattcggaag ggttacgctc cttccaaata 99540
tagtggatta acaaaaaccg gtacggcgtt gtctcgcctt agctcaaaga gaacgattct 99600
ctaaggtgct gaagcaccaa gtgaatcggt tccgtactat ctgtactgtc tgcggcttcg 99660
ttgccttgtc ctgatttttg ttaatccact ataaatcgag cctaaaacaa tgccgtctga 99720
aacggaaatc tgtttcagac ggcattgtta cattcaaacg gcgggccgtt tatttgaatt 99780
tgtaggtgta ttgcagaccg atgatgtcgg cgtggttttt gaaacgtgcg gaagacgcgc 99840
ctttgctgtc cacatcgttg ccgcttgcct tcgccgtgcg gtagctggtg tcgttgatgt 99900
ggatgtgggt gtaggcggca tcgacgacgt ggtttttacc gatatggtat ttcataccgg 99960
cggagaacca gatgcggttg ccgtcgggta ggctgttcat gcggtagtcg gcgttgcgga 100020
cgggcgattt gtcaaaagcg atgccggcgc gcagttgcag cggttcgctg atttgataag 100080
aaccgccgaa gccgactttg taggtgttgc gccagttggg ggtgatggtg gtgcggtcgg 100140
atttgccttt gacgacggtt ttttcttttt caaaaaccag ttccgcctta tcgaagcggc 100200
tgtggcgcgt ccaagttacg tcgccgaaca ggtcggcttt atcggacact ttgtacatac 100260
cgtgtacgga caaagactca ggcgtaacga ttttaacgcg ggctttttca ttcgccgtgt 100320
agccgtttgc tgcaagcatc gtactccaca ttgctttcgc cgccgcgccg tctgccgccc 100380
attcggcatc gcctttgagc gtgtgcgaga ctttggaacg gtagttcacg cccacgcgcg 100440
cacggtcgtt gatgtcccac atccacgcca gttggtagcc gaagccccaa tcgctgcctt 100500
tgacatcggc gtgtccgtcg gcctgaattt ttgcagcttc ggctacaccg ttaggtttgg 100560

gcggttttgc cgtcaatatc tctgctttac tcttaatccc ccagtcggca tatttgcgca 100620
gttcggcgga agtatgttgg gcgatgatgc ctgcgccgaa ggaatggcgg tcgttgagtt 100680
tccacgcggc gacaggttcg acggcgatgc tggtcagacc gagtttgttg atgttgtggc 100740
gcaacacgga atctttttcg tattcggtgg cagagccgaa ggggacgtac acgcccaagc 100800
ccacggtcag attgtcgttg actttgtatg cgccgtagat gtggggcgcg accgtggttt 100860
tggtgatttt gccgcttttc gaaccttgga cgggaagccc ggtaaagtcg gtggcggaat 100920
ccgcctcata atgaatgctg ggcagcacga tgttggcgtt gacggaaatc tggctgctgt 100980
cgagtttggt caggccggca gggttgtaga agatggtcga tgcgtcggcg gcttctgcgg 101040
cggcggcatt tgccgtgctt tgcgcgttga ccgactgtgt gccgaagtgg tagccggatg 101100
cgtggacgga tgcggcggca aaggcagtgc cgagcagcag gacggttttt ttcagtgcgg 101160
aaggggtcat ttcggtttcc gtaaaaaggc ggacggtgga taaatatagt ggattaacaa 101220
aaatcaggac aaggcgacga agccgcagac agtacagata gtacggcaag gcgaggcaac 101280
gctgtactgg tttaaattta atccactata aaaaaggcag tcggaaatgc cttgtttcgc 101340
tttagtatag gtactcgatt ttatccgatg ttgccggatt tgcacaattt tttcagagtt 101400
tgcccgaacc gccgcgccgc cgcaaaaaat gccgtctgaa gcctcgggca tcggcttcag 101460
acggcatttt ccactcaggg cggattattt gacgcgcagc acttccagtg tgttggtcga 101520
accggattcg cgcatttgcg aaccgctggt aatgatgtat tggtcgccgg aatgcaggat 101580
gttgtgttcc accagcatcg tttcgacttc gtttaacgcc gtgtcgtggt cggtactggt 101640
tgccaaaatc agcgggcgca cgccccggta catcgccata cggcgttggg cggaaacgct 101700
cggggtcagc gcgaaaatcg gcagggtgat gttgtggcgg ctgatttcaa aggcggtcga 101760
accgctttcg gtcagggcga cgatggcttt ggcgtgaacc gcgcgcgcca cgctgaccgc 101820
accgccggca accgccaggt tggtgctgac cgcttcggga tactcgacct gttcggcaac 101880
gccgttgagc gaatcctgct ctttttccgc agccgcgcag ataatcgcca tttggctgac 101940
ggtttcaaac ggatacgcgc cgacggcggt ttcggcggaa cacatcaccg catcggtacc 102000
gtccaatacc gcgtttgcca catcgctgac ttccgcgcgg gtcggtacgg ggttggtaat 102060
catcgattcc atcatttgcg tcgccgtaat gctgaagcgg cgcaactcgc gggcgcggcg 102120
gatcatccgt ttttgcaggg cggggacggc ggcgtgtccg acttcgaccg ccaagtcgcc 102180
gcgcgcaacc ataatgccgt cgccggcgag gatgatttcg tccaagtttt caatcgcttc 102240
cacgcgttcg attttggaaa ccaaaccggg gcgcacggcc gtgctgccct tcatttcttc 102300
ttcgactttg gcgcgcgcga tatgcaaatc ttcggcggat ttcacaaagc tgatggcgag 102360
gtagtcgcaa ccgatggcaa tcgcggtttt caggtcgcgg aagtcttttt cggtcaacgc 102420
gcctgcggac agaccgccac cgcgtttgtt gatgcccttg ttgcttttca ggacgtggct 102480
gttttccacc cttgtgataa tcctgctgcc ttcgacggat tccacggtca gggtcagcag 102540
gccgtcgtcc agccacaaga catcgcctgc ggcaacgtcg tcgggcaggt cgcggtagtc 102600
caaaccgacc gcctcgcgcg tgccttcgcc ttcgagcgcg gcatccagta ccagcgtttc 102660
gcctttgttc aattcgatgc cgccgccggc gattttgccc acgcggattt tcgggccctg 102720
caggtcggca atgatggcga tttcctgtcc ggcgcgtttt gccgcctcgc gcacgatgag 102780
ggcgttttcc tgatggaatt cgggcgtgcc gtggctgaag ttgaagcgga cgacgttcag 102840
accgccgacg cggatcatgt cttccaacag ttcgacgttg ttgctgcccg gcccaagggt 102900
ggcgacgatt ttagtgttgt ggctgatgcg ggtcagatcg cggcttgtct ggttcatatg 102960
aaagtccttt tggtctcaat cgggtgtttt gcggtatttt gttacaaaat tacagaaatt 103020
tggaaccggt ttgatgtcca tttgatgaac gcggcggaat attctgtaaa aatatgattt 103080
aaattaatag tttgatattt tacctgcaaa ccgccttttt tggcgcaaaa ttacacggtt 103140
ttatgactta ggctaaattt attttggggc tgtcctagat aactagggaa attcaaatta 103200
agttagaatt atccctatga gaaaaagtcg tctaagccgg tataaacaaa ataaactcat 103260
tgagctattt gtcgcaggtg taactgcaag aacagcagca gagttagtag gcgttaataa 103320
aaataccgca gcctattatt ttcatcgttt acgatgactt aatttatcaa aacagcccac 103380
atttagaaat gtttgatggc gaagtagaag cagatgaaag ttattttggc ggacaacgca 103440
aaggcaaacg cggtcgcggt gctgccggta aagtcgccgt attcggtctt ttgaagcgaa 103500
atggtaaggt ttatacggtt acagtaccga atactcaaac cgctacttta tttcctatta 103560
tccgtgaaca agtgaaacct gacagcattt tttatacgga ttgttatcgt agctatgatg 103620
tattagatgt gcgcgaattt agccatttta gcttcgctga aacttcgttt tcgtatcaat 103680
cacagcacac attttgccga acgacaaaac catattaatg gaattgagaa cttttggaac 103740
caggcaaaac gtcatttacg caagtctaac ggcattccca aagcgcattt tgagctgtat 103800
ttaaaggagt gcgaacgacg ttttaacaac agtgagataa aagttcttgt tccattttaa 103860
aacaattagt aaaatcgagt ttatcttagt tatctaggac agccccgttt gtgtactgaa 103920
atgcttcaaa acaccaaacc aagtttcgtt ttctaaaata cgaaaccatt actgctgcct 103980
aaattttttt ggattgctaa attatggcag tatgattttg gattttaaat tgaaaggcaa 104040
gaaaaatgtc aaaaaatgat gtagttaaag taattggtat attcccccta ttgtccgaac 104100
aatagagcag acttcccggc aggctgccca catcagaacg cccgttcgct ggtttgtacg 104160
tcctgaaaaa gctcttgcat taagttaatc ataatgggaa atttaaattt ttttaatgct 104220

tacttaaaca aaagccccac tccaccatta ggagtttctt tttcagtata caagtaaata 104280
tttttaaaat attgatttaa tttaaaataa aatacttgca aaaaaagtat taaattaaac 104340
ttaagaaagg ttaattctga tttacatttc caaccatact tctttacagg agaaaatcat 104400
gaaagagtta cacacctctg aattagttga agtgtcaggt ggcaaattcc atatctttgc 104460
acagggtggc ggcaacctag gtaaaaaaga tatggttgct gttggtaaaa ttggtgcttc 104520
ctattcccct aacaatagtg gagtagagtt ttctgttagc aagcaatttg gatatgtaca 104580
aggtcttggt gtacagtttt cgaaacctac ttttggtatt agtaaaaaat ggtaagattt 104640
tttgttttat cctttctgac attaataaat ctatgctcat taagcgcatg caatagccac 104700
tttacaggaa atatcaatcc attaggtact cacaataaag ttgctaatcc caattgtgcc 104760
aatagtgcca atagtcatat cagacaaccc agtaggaaaa actatgatcc aactgaatat 104820
agtgcttggt tacagtatat gcatgattgc aaataatgag taacgatgaa aatttacttt 104880
tttctcaacc acacttaaca aaggtgaata ttatgcaagt tttgactttg aatgaaattt 104940
aacaagtttc tggtgctgct tgtaactggc gtgatttctc aaaaaatacc attggtagtg 105000
cattaggtgg agcagctggt ggggcaattg ttggttcatt tgcaggtggt attggtgcta 105060
ttccaggtgc gaaattcgga gctattggtg gtgcaatcac tggtgctgta caatatggaa 105120
gcacttgttg gtggtaatat tccttaataa aactagggta ttttgatatt ttctattcaa 105180
aataccctag tttttcataa gaacttaaat acaaaaagga acaaataatg aaaaaatata 105240
gtgattattt taaatattta atcttttttt tgattttact cccaacaaat tatctcgtat 105300
ctcattatgt ggtacaaacc tcaatgagta tgttaagcat tttaagttct tctataataa 105360
caacttgcct ttttttgtt tttacaaatt tatcccaatc aaagaaacat aaataagtac 105420
acatgtctaa caatcactca tttttcagac cagaagtctt tgtagctcaa cggaacaagt 105480
ggacaggacc agtaggctgg gttgacgcaa tgggagctgg tattttctct gttgctggcg 105540
gatacaatat cggtcgtggc atgatgaagc cataagataa ttacatcatt aaggaaaagg 105600
taatttcagt tacagcaata tgtattgaag ttaccttttt ctatttagat tgaacaattt 105660
tgaaagagaa aaattatgaa tactgaaacc atttacgcca ctgtcttttg cattttagct 105720
gcaaccattt ctggattatt ggttaaattt aatgtaatta aaatagaaac atcaatcaat 105780
agcaaattta tgttattagg cataagtatt ttaattattg gtatttttct atccattttt 105840
ttttaagaaa taataataaa tgtcccactt attccgaaaa gaagtctttg tagcccaaca 105900
aaataagtgg acaggtcagg ttatcttgac ccgtccattc tcttttttat ttctgacttt 105960
ttgcgctttt ctcattgctc tgtgtatcat tatctttttg atttttggta gctataccaa 106020
taaaacaacc gttgaaggtc aattacttcc aactatgggg gtggttcgtg tttactcttc 106080
cgatatcggc acgattacgc ataaatttgt tgaagatggt aactttgtca aagctggcga 106140
accattgttc aaactttcca catcgcgttt tggcgaaaaa ggaaacgtac aagccaaatt 106200
ggcagcagaa gccaacctta aaaaaacttt ggcattacaa gaattggaac gtttaaagcg 106260
cattcatcaa aatgagcaaa aaaatgttca taacaacatt catcgtttaa acaatcaatt 106320
agagaatatt aaacagcaaa ttacagggca aaatcgtcaa attcgtttag cggaaaaaac 106380
ccttaacaag aacaagtttt tagccagtca aggcgcagta tcccaacaag ataagatgac 106440
cgccgaaagc catttattgg aacaacgctc acgtttggag agcctaaaac gtgaacaaaa 106500
taatgcaatc agggaacttg atgaacagaa aatcacatta agcagcctgc ctgaacgcca 106560
taaaaccgaa ttgagccaac tcaaccgtgc gattacggaa atgaaccaag aaattttgga 106620
ttttgatttg aaatccgaac aaaccatacg agctagtaaa tcaggttgag acctttgcaa 106680
aaataatctg ttaacgaaat ttgacgcata aaaatgcgcc aaaaaatttt caattgccta 106740
aaaccttcct aatattgagc aaaaagtagg aaaaatcaga aaagttttgc attttgaaaa 106800
tgagattgag cataaaattt tagtaaccta tgttattgca aaggtctcag gttatatatc 106860
aacaattaat gttgatatag ggcaacaagt tgaaccgtct aaattgctgt taagcattgt 106920
ccctgaacaa actgaattgg tcgccaatct ttacataccc agtaaagctg ttggttttat 106980
taaaccgaaa gataaagttg ttttacgtta ccaagcgtac ccttaccaaa aatttggaca 107040
tgccacagga gaaattattt cagttgccag aactgctctc ggtaaacaaa agctatcagg 107100
tttaggtatc attttcacta acccaacctt attaaatgaa cctgcctatc ttgtgaaagt 107160
taaattggaa aaacaaacga ttaaagcata cggagaaaac aagccgcttc aaattggcat 107220
gattttagaa gcagatattc tccatgaacg aaaaaattgt acgaatgggt acttgaccca 107280
ctttacagca tttcaggaaa aatcaattaa aaatggatta tttatcaaga ctgtcctttg 107340
gatttaacaa aaagctacct gtcattctgc aaacagaagt tgctgaatgt ggtttagcat 107400
gcctgacatc catcttgtcc tattatggct ttcacactga tttaagaacg ttacgccaaa 107460
aatacaccct gtcattaaag ggcgcaaatc ttgcagacat catgagattt ggcaatgaaa 107520
tgaatttaac gccacgagct ttgcgtttag agttagatga gctgtcaaat ttacaactac 107580
cctgcattct ccattggaac ttaaaccatt ttgttgtact ttgttccatt tccaaagaca 107640
gtatcgtcat tatggaccct gctgtcggta tgcgaaaaat caaaatggac gaagtttcac 107700
aaaaattcac agggattgcc ctagaattat tccccaatac ccattttgaa gagaaaaaag 107760
aaacaaagaa aatcaaaata ttatctctat taaggggggg tcaggcttaa aacgctcttt 107820
aattcaaatg cttatattag ctatttcttt ggaagtcttt gcattggtta gtccattctt 107880

tatgcaatgg gtaatagacc atgtcattgt aactgctgat aaaaatttat tattgaccct 107940
tactttggga tttggtttac tgactatcct gcaacagtta attagcctgt tacaagcatg 108000
ggtaggtatg cacctatcta caactcttaa tttacaatgg aaagccaata tatttaaaag 108060
gttacttgac ttacctaatg actatttcag taaacgacat ttaggagatg tgatttcaag 108120
atttggttca atagatcata tccaagaaac actaacttct acttttttg ttttagtttt 108180
aaatagctta atggctgttt ttactttcgt gttaatgaca atttacagca ctcaattatc 108240
gctgattgtt cttttaacac ttgttttgta catactaatt cgttggcttg catattaccc 108300
attaagaaat gcaacagaag aaaatattgt tcatgaagcc aaacaaaact catatttcat 108360
ggaaaccatt cgtggtatcc aatcagttaa attatttgat aaacattatc aaagacatgg 108420
cacttggatg agcctatttg tgaatacagt caataccaag ctgacaacag ataaactctc 108480
tgctttattt gaattttcaa ataaactgtt gtttagcatg gaaaatgtta tcataattta 108540
tcttggtgca agcgcaattt tagatggttc atttacagtc ggtgttctga tggctttttt 108600
ggcttataaa gggcaatttg aaagcagaac agcttctctc gttgaccaat acatccaaat 108660
caaaatgtta gggcttcatg ctgaacgttt ggctgacatt actttaaatg aaacagaaac 108720
tgaaattatt aagtataatc atatacctaa attagataat gaacaactgg ttcttaaagt 108780
tgaaaacgtc tcattcagat atgctgataa tgagccatat ctttttgaaa acattaattt 108840
ggaatttaaa gataatgaag cagttgtttt aacaggacaa tctggtcggg ggaagtccac 108900
tttgttaaac attttaacag gtagcctaaa acctgaaact ggtacagtta gtattaatgg 108960
gcatgatata tatcaagttt ctccatcctt tattagggga ttgagcggga ttgttcgcca 109020
agatgatgtc ctttttgcag gttctattgg ggaaaatatt tcattttttg atgaaagccc 109080
aaatatggag ctcattgaac aatgtgcaaa aatggcacaa atacatgacg atatacttaa 109140
aatgccaatg ggctatgaga ccttgattgg cgatatggga aatatcttat caggtggaca 109200
aaagcagaga gttatcttgg ctcgtgcatt gtataaacga cccaaaattc tatttttaga 109260
cgaagcaagt agccatttag atgtagaaaa tgaacaaaaa attaaccata acctaaaaag 109320
tcttggtatt atgaaaataa tggttgcaca ccgccaagaa acaattcaat cggcagataa 109380
aattctgaat ttaggttgaa cagaacaaga cttcattttt ctttaacaaa aagtgaagtc 109440
ttttttcaaa taatttaata gaatacatga aaatagcggt ttaacgttcc atttcccaat 109500
catcacgact ggctttgtgt tttggcgatt tttcagtttc ctttttctgt tgaatttgtt 109560
gttttttctg ctcttgttcc catttttggg ctaatttcac ggtctcattt tcagcccatt 109620
ccatcacggc acaacgatgt agcttttctc cgatatcgcc attaaagcca gctccacgaa 109680
cttcaccata aattcttgaa tatttttgat tatattcaat ttcttttcca ttttctttaa 109740
aggatttctc ccacttttca caaacttcat caaaatcttt caaagggata ttttttaagg 109800
ggctgtccta gataactagg gaaattcaaa ttaagttaga attatcccta tgagaaaaag 109860
tcgtctaagc cagtataaac aaaataaact cattgaactg tttgtcacag gtgtaactgc 109920
aagaacggca gcagagttag taggcgttaa taaaaatacc gcagcctatt attttcatcg 109980
tttacgatta cttatttatc aaaacagtcc gcatttggaa atgtttgatg gcgaagtaga 110040
agcagatgaa agttattttg gcggacaacg caaaggcaaa cgcggtcgcg gtgctgccgg 110100
taaagtcgcc gtattcggtc ttttgaagcg aaatggtaag gtttatacgg ttacagtacc 110160
gaatactcaa accgctactt tatttcctat tatccgtgaa caagtgaaac ctgacagcat 110220
tttttatacg gattgttatc gtagctatga tgtattagat gtgcgcgaat ttagccattt 110280
tagcttcgct gaaacttcgt tttcgtatca atcacagcac acattttgcc gaacgacaaa 110340
accatattaa tggaattgag aacttttgga atcaggcaaa acgtcattta cgcaagttta 110400
acggcattcc caaagcgcat tttgagctgt atttaaagga gtgcgaatgg cgttttaaca 110460
acagtgagat aaaagttctt gttccatttt aaaacaatta gtaaaatcaa gtttgtccta 110520
gttatctagg acagcccctt gttttttgtt cggcggcttg cgtggtcggg taaaatgaaa 110580
gttttgaacg gttggtcgga caggaagatg tggcgggttt tgagtgcttt gccgataggc 110640
gtggtgtttt ttgatttgat ctacggtttt gtgttgaatg tgttgcaggg tttggatttg 110700
cagcgtgccg tgccggattc ggaaggcgtg ttggcggtta cgccccgatat tgcattcaac 110760
agtttgcaga ttgtcgccaa cggcggtatg gcggcggtgg tctgtttcgg gttggcggtt 110820
gtgtttttgc tcaaccgttc ggtgcggcgg cggcaggtgt tggaaatcgg ggtgttccgg 110880
atgttggggc tggtggcggt attggcgttc agcgcgccgt cggtgtggga gtgggcgaac 110940
gcgctgccgc tgctgctgaa gggcgcggac gtggtcaata cggggaatgc gcgttatgtg 111000
ctgacggctt tgtgtatgcc ctttccggcg gtgtcgtgcg tcatcgggct ggtggggcgg 111060
ttcaggcttc agacggcatc gggcagggcg gcaaagtcag gggtgcgggg caaggcggac 111120
ggataggacg catttttcag cgggtgcgtc gagaagcagc cgatgtgttt ggcagccgca 111180
gcttgggggg tgtagtgcta atggcggttt ctttgctttt atagtggatt aacaaaaacc 111240
agtacggcgt tgcctcgcct tagctcaaag agaacgattc tctaaggtgc tgaagcacca 111300
agtgaatcgg ttccgtacta tttgtactgt ctgcggcttc gtcgccttgt cctgattttt 111360
gttaatccac tatataaaat aaatgggcaa aaatcggttt attatcgttt ttgccgcatt 111420
tggatttgtt ctaccgtaaa acgtgtttga cgaacgggat tcttattaaa aaacatctga 111480
tttctaacaa aatcagtatt ttttggcacg atggctaaaa tttttccttc catttcgcca 111540

tcacgtgttt tccatgcgct caagaattgt gatttgctca ttgagacgtg ccccagcgat 111600
ggatcagcca gcaaaacagt ttctccgtta ataccgttca ataccgaaaa atggttgttt 111660
ttacggtatt ttaaatacac aattacagga atttttagtt gtaccaactg ttcaaatggc 111720
aaagcataac cttgtgcttc aaaacccagt tcgggcatta tgcgttgcat atcgtcaaaa 111780
gaagcacgca tttgggtttt atccattttg tctaagattt ccgcttcaga ataatgtctg 111840
ccataaaaat tattcagtaa cgtggcaatc gaagccgcgc cgcaagaaaa atccaaatct 111900
tgttttacta tgccggaatc tcgccgtgct ttccaactcc gtacatggat gttttggtaa 111960
gaagcggggg gtcaacaaac ataggccaag caaaaactat atttggggcg aaaccaatca 112020
aagccgcata atttatcaat ttataaagat tttttatcat aatatgtata cgcggaataa 112080
acgcatgaaa cataaaaaaa taaaatcata tgcaatttta ttgcatgaat aaatatgaat 112140
aaaatagata atgatgggag taaatacgcc atgtattttg gaagtttaaa tttattaata 112200
ataaaataat ttatcgtagc gcaaataaat cccaaaattg gaacgattag aaaaaaaatt 112260
aatacaccca tcatcatgca aactctatat taataaatag ctaactaatt ctattgtgga 112320
aattagttag ctaactaaaa gttattaatg attattttcg agaattgact gcattgttgg 112380
cagcattggc accaaaacct agtgcatgaa tacccggtct ccatgccaaa ttcccagcca 112440
atccgcctcc ggcagcagca gccagccctg ttttgccgct actcctgttg ccgcaccgat 112500
tcctgtcgca gtagccgcgc cttgcgcagt tcctaattta ccatgattat acaaattagc 112560
accatgatac ccccatgcac ctaatgcacc gccaaaagca gcggctgcaa taatgggaac 112620
aaattcacct tgtgtttctt tcatttcagc ctgtgataat tgaattgctt tcacattttg 112680
gctgtcaaaa acttggctgt ctaaattttg cgccattaca ggtgtaatca tcatagccat 112740
tacagttgca attttcgttg cgctggtttg cacataaata ggattagcaa attcgctttg 112800
attgcgttca gtgttgatgt agctaatact gctttctagt ttgaatttac ccttgtcagt 112860
caataattct tccaaactta aaggtaagtc agcataagca atttggctgg caacagtcaa 112920
aataaaatct attagacatt tgtgtttttg catcatttcg tttgattttc taggttttga 112980
gaatgataca aagtttttta caaagtaaag agtcactctg aaaaaacttt tttcattata 113040
aatcaaaata ttgatagaat aaatagcgag catcgattca cggtgcgctt tagtgcaaag 113100
cgtacaacgc gagcctgaac caccagccgc aacaggaaaa gaaagccgat agagtgcaat 113160
gcttgccaac gtgcaagcga gcttgcaaga acgcttggct caacgagagc aggcaagaca 113220
gaaagcagaa agcaggatag gagcggtaac gcaaaggtct cgggctttga tttcgccgta 113280
aaccctgctg ccgccttgtc cggaaagggt gcaggcggcg agtgccgaca gggtgcagat 113340
ggggaggggg gttttcattt ggggtcgcaa cggaagtggt atgcgcagat ttcaaaaccg 113400
tttttgaaat acaggcggtg cgcgtcggca cggtcgtggt tgacgtggac gttgaggtgg 113460
atttggtta cccctgtttc cgcgccgatt ttgcggactt cttccaaaag gcgcgaggcg 113520
tagcctttgc ggcggctttg cggcagggta acgatgtcat cgatgtggat gtggcggccg 113580
ctggcgaggg tgcaggcttc gcggaagccg cagacggcga cggcattgtg tttgccttct 113640
tcaaaaatac ccagcaggcg gtagccttgg gggcgttgga ctttgttgat ctgttcggta 113700
aagcggttga tgtcggtcag ggcggaacgc aaaacgctca aggctgcaaa ggcggtggcg 113760
gtgtcgtccg cgccgatttc gcgcaaaacg taggatgcgc ccgaggcggt ctgttcctgt 113820
gctttctcgg cggcgtgttt ttcttcgatt gcctgtgcca gcatgacgtg ttcgtcggca 113880
gggttgtttt gtccgccctg ttcgcgttct tcgagcaggg ctttgcagtc gatgacgcgc 113940
aggtcgttgt cggcggcaaa gtccatcagg aagcggaaca tttggggatt gtcggtttcc 114000
agttttttat cgacggcgac gcagcggatg ttgtccacca gtatggggcg cacccatttc 114060
gaataggaaa gcctgtggtc tttggtgaac gaggacagaa tgccgcacag gcgttccgcc 114120
cagtcgctgg gacggaaaat cttgccggaa ctcgttgtgc cgtggatgac gacttcgtag 114180
gggttgcaga ctaacatggc ggcttcctga aaagaaatgt ctagcgcgat tataccttat 114240
gcttatgcgg gcgtgtttgg atatgccgtc tgaaaagtac gggattcgtg cggtaaaact 114300
ttgcggcggc aaatgtgcga taatacgcgc cgtattgccg cttttgcgaa gctgttccgc 114360
aaacatacgg gcggcgtgga cgacgtataa ccggataccc gcctgacgcg ggttttttac 114420
ggaaggggggg caaaaatgcc taatccgctt tacagacagc atatcatctc catttcggat 114480
ttgtcgcgcg aacagttgga atgcctgctt cagacggcat tgaagctgaa ggcgcatccg 114540
cgcggcgacc tgttggaagg caaacttatc ggttcgtgct ttttcgagcc gtccacgcgc 114600
acgaggctgt cgtttgaaac ggcggtgcag cgtttgggcg gcaaggtcat cggtttctcg 114660
gacggcgcga ataccagtgc caaaaaaggc gagacgcttg ccgataccgc ccgcatcatt 114720
tccggatata ctgatgctat catccaacgc caccccaaag acggcgcggc gcgcgtggca 114780
gcggagtttt cgcgcgtccc cgttatcaac gccggcgacg gcacgaacca gcaccccagt 114840
cagacgctgc tcgacctggt taccatttat gaaacacagg gacgtttgga caagctcaaa 114900
atcgccatgg cgggcgactt gaaatacgga cgtaccgtgc attcgctttg tcaggcgttg 114960
aaacgctgga attgtgaatt tgcctttgtt tcgccgccca gcctagccat gcccgactat 115020
attaccgaag agttggacga agccggctgc cgataccgta tcctcggtag tttggaagaa 115080
gcggcggaat gggcggatat cctgtatatg acccgcgtcc agcgcgaacg tttcgacgaa 115140
caggaatttg ccaaaatcca aggcaaattc aacctcgaag cgtctatgct cgcccgcgcc 115200

aaaccgaacc tgcgcgtgct gcaccccctg ccgcgcgtgg acgaaatcca tcccgatgtc 115260
gatgccacgc cgcacgccta ttatttcgag caggcgacca acggcgttta tgcgcgtatg 115320
gcgatattgt cgctggtgtt gaacgaagaa gtgtgaggaa ccgatatgga aaccccgaaa 115380
ctcagtgtcg aagccattga aaaaggtacg gttatcgacc atattcccgc cggcaggggg 115440
ctgaccatcc tgcgccagtt caaacttttg cactacggca acgcggtaac cgtgggcttc 115500
aacctgccca gcaaaaccca aggcagcaaa gacatcatca aaatcaaagg cgtgtgcttg 115560
gacgacaaag ccgccgaccg cctcgccctg ttcgcccccg aagcggtggt caacaccatc 115620
gacaatttca aggtcgtgca gaagcggcat ttgaacctgc ccgacgaaat cgccgaagtg 115680
ttccgctgtc cgaacacgaa ttgcgccggc cacggcgagc cggtcaaaag ccggttttat 115740
gttaaaaagc acaacgggca gacgcggctg aaatgccact actgcgaaaa aacctacagc 115800
cgggattcgg tggcggaagc ctgacggatt cccttaaacc gagtgggcgg catttcgtct 115860
gccgcctgtt ttgccaatct gaaatggaat gatgatgcac gcttctgtcc aaagccgttt 115920
cgcaccgata ctttatgttt tgattttctt tgccggtttt ttgaccgcgc aaatctggtt 115980
caatcagaaa gcctatactg aagagctgcc tccgcttctg tccgcattgt ccgccgtcgc 116040
gctggtgtgg ctggcgtggg cgttcgtgtc ggcgcgttca aaggccaagg cggaaaagtt 116100
ctaccgcgaa aaaatgatac agaacgaaag catacacccc gtcctgcacg cctctttgca 116160
acacttggaa cacaagccgc aaatactcgc cctgctggtc aaaaaccacg gcaaagggat 116220
ggcggaacag gtcaggttca aggcggaagt gctgcccgac gacgaagacg cgcgcacgat 116280
tgccgccgag ttggcaaaaa tggatatgtt cgcattgggg acggacgcgg tcgcctcggg 116340
cgaaacctat ggacgcgtgt tcgccgatat tttcgagttg tcggcggctt tggaagggcg 116400
cgcgttcaaa ggaatgttga aactgacggc ggaatataaa aacatcttcg gcgatgcctg 116460

ccgttcggaa acggcgttgg agttgggcgc actcaatcag gcgttgcagg agatttcaaa 116520
aacatcggaa aagtccaaac ggatatttta ttgaagatgg aaaaatgccg tctgaaacgg 116580
aaggtgtttc agacggcatt tttgtcggat gattaattat tcggagcggt tgaagccaaa 116640
cttcacgcgg ctgcggccct gatccggtat attgtccaaa tcgcgtcccg gattggcggc 116700
ggtgtcgcct acggaaatat cggagatgtt ttccaaaatg atggcggacg acaggtgttc 116760
ggaggtgcgg taaaccattg ccaagcccac ttcttcggca ggagtggaaa tcagctcgac 116820
ggtatccctg cttttgaaat tgttggagag gtcgacctgc atcgttttct tgcgtttgta 116880
gaggctcaaa accgtgcctt tgtccaaacc gtccgcctcg cctttgtcga tggtgatggt 116940
ttgaaactgg ccggcaatcc ttgtgccttc aaacacggaa acgattttag cctgaaccgg 117000
gcgggacggt tcgtgcggca tcatgttgaa gcggtcggtg tcttccggca ttttcatcag 117060
gtagtcgccc tgctgtattt cggaaatggc ggtttcgacc accagcggct gtatcgaagg 117120
ggtgcgcagc ggggtaatca aaggatgggt gcgggtatgg tattcgttgt ctttcggccg 117180
ttctccagcc tgtttcgagc gttgttcgag gacagagtcg gtatagtcga gggagcgcac 117240
gatgccgctg aatgcgactt cctgcccgag gaatttaccc gtatccggat cggtgatgtt 117300
tttattgatt cggtaggtca ggtagcggcc cggctctttc aggcctttgg tgtaaaccct 117360
ggtgcctttg gtgtacagca gcctgccttc cgggcccgag agcaggcgcg gcgcggcagc 117420
ggtttctttg cgggaaacga tttgcggatg ccgcataaag atgcggtaga agttgacatc 117480
gatggcggga ataccgtatc cggacacttc cttatccgga ctcattttga cgacggggat 117540
gccgtctgtc tgttccaagc cgaggcgcgg ttcgccgtca acgtggcgca acaccaatac 117600
ctggtccgga taaatcaggt cgggattgtg gatttgatcc cggttcgcgt cccacaggcg 117660
gccccattgc cacgggctgt acaggtattt gcccgaaatg ccccacaggg tgtcgccctg 117720
tttgaccgtg tagcgttccg gcgcgttcgg gcgcacctcc aaatttgccg ccaaagtttg 117780
tgttgagaat gccatacctg ccgcgcagag cagggttata atacgacgtt gcataaccgt 117840
tccccttatc tgataaattt cggtttgtct tgcttgattg ggttggaaaa agcggcggca 117900
gcccctcggg atgtgccgcg tgataaaaaa tgttccgcat tttaacatcg aattatccgc 117960
accatcacgg taattatgaa aaacaggcgg cgtatccgcc gaaggaaaga gaaaattatg 118020
gctttattga atatcttgca atatcccgac gagcgtctgc acacggtggc aaagcctgtc 118080
gaacaagtcg acgagcgcat ccggaagctg attgccgata tgtttgaaac gatgtacgaa 118140
tcgcgcggca tcgggctggc ggcgacgcag gtcgatgtgc acgagcgcgt ggtcgtgatg 118200
gatttgaccg aagaccgcag cgaaccgcgc gtgttcatca accccgtcat cgttgaaaaa 118260
gacggcgaaa ccacttacga agagggctgc ctgtccgtgc cgggcattta cgacaccgta 118320
acccgcgccg aacgcgtcaa ggtcgaggct ttgaacgaaa aaggcgaaaa gttcacgctg 118380
gaggcggacg gcttgttggc gatttgcgtg cagcacgagt tggaccacct gatgggcatc 118440
gtgtttgtcg aacgcctttc ccaactcaag caggggcgga ttaagaccaa gctgaaaaaa 118500
cgtcagaaac atacgatttg accctttgc cgtgccgtct gaacgctgca aagttttcag 118560
acggcacggt cttgtccgac aattttacgc acgcgcagga acacgctatg aaagtcatct 118620
tcgccggcac gcccgatttt gccgccgccg ccttaagagc cgttgccgcc gccggttttg 118680
aaattccgct ggtgctgacc cagcccgacc gtccgaaagg gcgcggtatg caactgactg 118740
ccccgcccgt caaacaagcc gcgctggaac tcggtttgcg cgtcgaacag cccgaaaagc 118800

tgcgcaacaa cgccgaagcc ctgcaaatgc tcaaagaggt cgaggcagac gtaatggtgg 118860
ttgccgccta cggtttgatt ctgccgcagg aagtgttgga tacgccgaaa cacggctgcc 118920
tcaacatcca cgcttcgctg ttaccccgtt ggcgtggcgc ggcgccgatt caacgcgcga 118980
ttgaagccgg cgatgccgag acaggcgtgt gtattatgca gatggacatc ggtttggaca 119040
ccggcgatgt ggtcagcgaa caccgctacg ccatccaacc gaccgatacc gccaacgaag 119100
tccacgacgc gctgatggaa atcggtgcgg cggcggttgt tgccgatttg caacagcttc 119160
aaagcaaagg ccgtctgaac gcggtcaaac agcccgaaga aggtgttact tacgcgcaaa 119220
aattgagcaa agaagaggcg cgtatcgatt ggagcaaaag cgcggcggtt atcgaacgca 119280
aaatccgcgc cttcaacccc gtgcctgccg cgtgggttga gtatcagggc aagccgatga 119340
aaatccggcg ggcggaagtg gtggcgcaac aaggcgcggc aggcgaagtg ttgtcctgtt 119400
cggcggacgg tttggtcgtt gcctgcggcg aaaacgcgct gaagattacc gaattgcagc 119460
ctgccggcgg caggcggatg aatatcgcgg cgtttgcagc aggacggcat atcgaagcag 119520
gggcgaagct gtaaatccct tcagacggca ttccgatccg caaacgggaa tgccgtctga 119580
aaccatcagt cgaagaaagc gaatcacata atatgagtat ggcacttgcc caaaaacttg 119640
ccgccgacag cattgcggcg gttgccgaag gacgtaacct tcaggacgtg ttggcgcaaa 119700
tccgcaccgc gcatcccgac cttatggcgc aggaaaacgg cgcgttgcag gacatcgcct 119760
acggctgcca gcgttatttg ggcagtttga aacatatgct cgcgcagatg ctgaaaaagc 119820
cgattggcaa tccgcagctc gaaagcctgc ttttggcggc gttgtaccag ctgcattaca 119880
cgcgcaacgc gccccacgcc gtggtcaatg aggcggtgga aagcatcgcg aaaatcggac 119940
gcgggcagta ccgttcgttt gccaacgcgg ttttgcgccg ctttttgcgc gaacgcgaca 120000
agcttgtggc ttcctgtaaa aaagacgatg tagcgaaaca caacctgccg ctgtggtggg 120060
tggcttactt gaaaaaccat tatccgaaac actggcacaa catcgccgcc gcgctgcaat 120120
cccatccgcc gatgactttg cgcgtcaacc gccgacacgg caatgccgaa agctatttgg 120180
aaaaactggt ggcggaaggt atcgcggcta aggcgttgga cgaatatgcg gttacgttgg 120240
aagaagccgt gccggtgaac cgcctgcctg gtttttcaga cggcattgtt tcggtacagg 120300
acttcggcgc gcagcaggcg gcgtatttgt taaacccgaa agacggcgaa cggattttgg 120360
acgcgtgcgc cgcgccgggc ggcaagacgg ggcatatctt ggaactggcg gattgccgtg 120420
ttaccgcctt ggacattgat gcaggccgtc tgaaacgggt ggaagacaat atcgcgcgtc 120480
tgggctttca gacggcatcg acggcgtgtg ccgatgcaca ggacctgtcg gcatggtatg 120540
atgggaaacc gtttgatgcc gtccttgccg acgtgccgtg taccgcctcg ggcgtggcgc 120600
ggcgcaatcc cgacgtgaaa tggctacgcc gtccgaccga cgcgctcaaa accgcccgcc 120660
agcaggaagc cctgctagat gcattgtggc aggtgctgaa aagcgggggga aggatgttga 120720
tcgctacctg ttccgtgttc gtcgaggaaa acgacggaca attgcaaaaa ttcctcaacc 120780
gccatgccga tgcagaactg atcgaatcgc gggtactctt accgaacaaa caccaagatg 120840
gcttttatta cgcgcttatt caaaagcagt aaatggctga ttgtgccgct gatgctcccc 120900
gcctttcaga atgtggcggc ggagggggata gatgtgagcc gtgccgaagc gaggataacc 120960
gacggcgggc agctttccat cagcagccgc ttccaaaccg agctgcccga ccagctccaa 121020
caggcgttgc gccggggcgt gccgctcaac tttaccttaa gctggcagct ttccgccccg 121080
ataatcgctt cttatcggtt taaattgggg caactgattg gcgatgacga caatattgac 121140
tacaaactga gtttccatcc gctgaccaac cgctaccgcg ttaccgtcgg cgcgttttcg 121200
acagactacg acaccttgga tgcggcattg cgcgcgaccg gcgcggttgc caactggaaa 121260
gtcctgaaca aaggcgcgct gtccggtgcg gaagcagggg aaaccaaggc ggaaatccgc 121320
ctgacgctgt ccacttcaaa actgcccaag ccttttcaaa tcaatgcatt gacttctcaa 121380
aactggcatt tggattcggg ttggaaacct ctaaacatca tcgggaacaa ataatgcgcc 121440
gttttctacc gatcgcagcc atatgcgccg tcgtcctgtt gtacggactg acggcggcaa 121500
ccggcagcac cagttcgctg gcggattatt tctggtggat tgttgcgttc agcgcaatgc 121560
tgctgctggt gttgtccgcc gttttggcac gttatgtcat attgctgttg aaagacaggc 121620
gcgacggcgt attcggttcg cagattgcca aacgcctttc tgggatgttt acgctggttg 121680
ccgtactgcc cggcgtgttt ctgttcggcg tttccgcaca gttcatcaac ggcacgatta 121740
attcgtggtt cggcaacgat acccacgagg cgcttgaacg cagcctcaat ttgagcaagt 121800
ccgcattgaa tttggcggca gacaacgccc tcggcaacgc cgtccccgtg cagatagacc 121860
tcatcggcgc ggcttccctg cccggggata tgggcagggt gctggaacat tacgccggca 121920
gcggttttgc ccagcttgcc ctgtacaatg ccgcaagcgg caaaatcgaa aaaagcatca 121980
acccgcacaa gctcgatcag ccgtttccag gtaaggcgcg ttgggaaaaa atccaacggg 122040
cgggttcggt cagggatttg gaaagcatag gcggcgtatt gtacgcgcag ggctggctgt 122100
cggcgggtac gcacaacggg cgcgattacg ccttgttttt ccgtcagccg gttcccaaag 122160
gcgtggcaga ggatgccgtc ttaatcgaaa aggcaagggc gaaatatgct gagttgagtt 122220
acagcaaaaa aggtttgcag accttttttcc tggcaaccct gctgattgcc tcgctgctgt 122280
cgatttttct tgcactggtc atggcactgt atttcgcccg ccgtttcgtc gaacccgtcc 122340
tatcgcttgc cgaggggggcg aaggcggtgg cgcaaggcga tttcagccag acgcgccccg 122400
tgttgcgcaa cgacgagttc ggacgcttga ccaagttgtt caaccacatg accgagcagc 122460

tttccatcgc caaagaagca gacgagcgca accgccggcg cgaggaagcc gccaggcatt 122520
atcttgaatg cgtgttggag gggctgacca cgggcgtggt ggtgtttgac gaacaaggct 122580
gtctgaaaac cttcaacaaa gcggcggaac agatttgggg gatgccgctt accccccctgt 122640
ggggcagcag ccggcacggt tggcacggcg tttcggcgca gcagtccctg cttgccgaag 122700
tgtttgccgc catcggcgcg gcggcaggta cggacaaacc ggtccatgtg aaatatgccg 122760
cgccggacga tgccaaaatc ctgctgggca aggcaaccgt cctgcccgaa gacaacggca 122820
acggcgtggt aatggtgatt gacgacatca ccgttttgat acacgcgcaa aaagaagccg 122880
cgtggggcga agtggcgaag cggctggcac acgaaatccg caatccgctc acgcccatcc 122940
agctttccgc cgaacggctg gcgtggaaat tgggcgggaa gctggatgag caggatgcgc 123000
aaatcctgac gcgttcgacc gacaccatcg tcaaacaggt ggcggcattg aaggaaatgg 123060
tcgaagcatt ccgcaattat gcgcgttccc cttcgctcaa attggaaaat caggatttga 123120
acgccttaat cggcgatgtg ttggcattgt atgaagccgg tccgtgccgg tttgcggcgg 123180
agcttgccgg cgaaccgctg acggtggcgg cggatacgac cgccatgcgg caggtgctgc 123240
acaatatttt caaaaatgcc gccgaagcgg cggaagaagc cgatgtgccc gaagtcaggg 123300
taaaatcgga aacagggcag gacggtcgga ttgtcctgac ggtttgcgac aacggcaaag 123360
ggttcggcag ggaaatgctg cacaacgcct tcgagccgta tgtaacggac aaaccggcgg 123420
gaacgggatt gggtctgcct gtggtgaaaa aaatcattga agaacacggc ggccgcatca 123480
gcctgagcaa tcaggatgcg ggtggcgcgt gtgtcagaat catcttgcca aaaacggtaa 123540
aaacttatgc gtagcagcga tattttaatt gtagacgacg aaatcggcat ccgcgacctg 123600
ctgtcggaaa tcctgcagga cgaaggttat tcggtcgcat tggcggaaaa cgccgaagag 123660
gcgcgcaagc tgcgccatca ggcgcgcccc gcgatggtgc tgctggatat ttggatgcct 123720
gattgcgacg gcatcaccct tttgaaggag tgggcgaaaa acgggcagct caatatgccg 123780
gtggtgatga tgagcgggca tgccagcatc gataccgccg tggaagccac caaaatcggc 123840
gcgatcgatt ttttggaaaa accgatttcc ctgcaaaagc tgctgtctgc cgtcgaaaac 123900
gcgttgaagt acggtgcggc gcaaaccgaa acggggcctg tattcgacaa gctgggcaac 123960
agtgcggcga ttcaggaaat gaaccgtgag gtaggggctg cggtgaaatg tgcctctccc 124020
gtacttttga cgggcgaggc gggttcgccg tttgaaacgg tggcacgcta tttccataaa 124080
aacggtacgc cgtgggtcag cccggcaagg gtcgaatatc tgatcgatat gccgatggaa 124140
ctgttgcaga aggcggaggg cggcgttttg tatgtcggcg acatcgccca gtacagccgc 124200
aacatccaag ccggtattgc ctttattgtc ggaaaggcgg aacaccgccg cgtcagggtg 124260
gtcgcatcgg gcagcagggc ggcaggttca gacggcattg cctgcgagga aaagctggcg 124320
gaactgctgt cggaatcggt cgtccgtatt ccgccgctgc gtatgcagca tgaagacatt 124380
cccttcctga tacaggggat tgcctgcaat gtggcggaaa gccaaaagat tgcgcctgcc 124440
tcattcagtg aagaggcact tgccgcattg acccgttacg actggccggg aaatttcgac 124500
caactgcaaa gcgtcgttgc aacgctgttg ttggaggcgg acggacagga aatcggcgca 124560
ggggcggttt cttccctttt ggggcagaat gtgcctgccg aggggcggga agatatggtg 124620
ggcgggttta atttcaacct gcccctgcgc gaattgaggg aggaggtgga gcggcgttat 124680
ttcgagtacc acatcgccca agaaggtcag aatatgagcc aagtggcgca gaaagttggt 124740
ttggaacgca cgcaccttta ccgcaaactc aaacagctcg gcatcggcgt ttcgcgccgg 124800
gcgggggaaa aaaccgaaga ataggcccgg acggccggtt taccggctgc gggcttttgt 124860
tttcagacgg catttggtgc aaatgccgtc tgaaatcgta aggggacgga ttttatgaca 124920
gaggacgaac gtttcgcgtg gctgcaattg gcgtttacgc cctatatcgg cgcggaaagt 124980
ttcctgctgc tgatgcgccg tttcggcagc gcgcaaaatg ccctgtccgc accggcggaa 125040
caggtggcgg cactgatacg gcacaaacag gcgcttgagg cttggcgcaa tgcggaaaaa 125100
cgcgctctgg cgcggcaggc ggcagaagcg gcattggaat gggaaatgcg ggacggatgc 125160
cgcctgatgc tgcttcagga tgaagatttt cccgaaatgc tgacgcaggg gctgaccgcg 125220
ccaccggttt tgtttttgcg cggcaacgtg caactgctgc acaaaccttc cgccgccatc 125280
gtcggcagcc gtcatgccac gccgcaggcg atgcggattg ccaaagattt cggcaagtcg 125340
ttgggtggga aaggcattcc cgttgtgtcg ggtatggctt cgggcatcga taccgccgcc 125400
catcagggtg cgttgcaggc agaaggcggc accatcgccg tgtgggggac gggcatagac 125460
cgcatttatc cgccggtcaa caaaaacctt gcctatgaaa tcgccgaaaa aggattgatt 125520
gtcagcgagt tccccatcgg cacgcggccg tatgccggca attttccgcg ccgcaaccgc 125580
ctgattgccg ccctgtcgca agtaacgctg gtggttgaag ccgcgttgga atccggttcg 125640
ctgattactg ccagattggc ggcggagatg gggcgcgaag tgatggcggt acccggctcg 125700
atagacaatc cacacagtaa aggctgccac aaactgatta aagacggcgc aaaattggtg 125760
gaatgcctgg acgacatcct gaacgaatgc ccggggctat tgcaaaatac gggtgcttca 125820
tcatattcta taaataaggg aatacctgaa aagcgcatca ctgccgttca gacggcatcc 125880
gaccagctgt ctctgcctga aggcaaaatg ccgtctgaaa agacggagaa ccgacccgtc 125940
ggcggcagta tcttggacag gatgggtttc gacccagttc atcccgacgt gcttgccgga 126000
cagttggcta tgcctgccgc agatttgtat gccgcactgt tggaattgga attggacggc 126060
agcgttgccg caatgcccgg cggcagatac cagcgtatcc gaacttgaac gcactttata 126120

ttaaggaaca cgaatgaccg aagtcatcgc ctacctcatc gaacatttcc aagatttcga 126180
tacctgcccg ccgcccgaag acttgggtat gctgcttgaa gaagcgggtt tcgatacgat 126240
ggaaatcggc aacaccctga tgatgatgga agtattgctc aacagctccg aattttccgc 126300
cgaacccgcc gacagcggcg cattgcgcgt gtacagcaaa gaagaaaccg acaacctgcc 126360
gcaggaagtg atggggctga tgcagtatct gattgaagaa aaagccgtca gctgcgaaca 126420
gcgggaaatc atcatccacg cgctcatgca cattccgggc gacgaaatta ccgtagatac 126480
cgccaaagtg ctgaccctgc tgcttttatg ggcaaacaag agcgagctgc ccgtgttggt 126540
cggcgacgag ctgatgagcg cgcttttact cgacaacaaa cccacgatga actgaagcgg 126600
cttcagacgg cccgcccgag tccgtctgaa acgtcggcat caaaaccacc atccagagaa 126660
cgacaaatgg cgaaaaacct attaatcgtc gaatccccgt ccaaagccaa aaccctgaaa 126720
aaatatttgg gcggcgattt tgaaatcctt gcatcctacg gacacgtccg cgacctcgtc 126780
cccaaaagcg gcgcggtcga tcccgacaac ggctttgcga tgaaatacca actcatcagc 126840
cgcaacggca aacacgtcga tgccatcgtc gccggtgcca aagaagctga aaacatctac 126900
ctcgccaccg acccggatag ggaaggcgaa gccatttcct ggcatctttt ggaaatcctc 126960
aaatccaaac gcggcttgaa aaacatcaag ccgcagcgtg tcgtgttcca cgaaatcacc 127020
aaaaacgccg tgctcgatgc cgttgcccat ccgcgcgaaa tcgaaatgga cttggtcgat 127080
gcgcaacaag cccgtcgcgc tttggactat ttggtcggtt tcaacctttc gccattgttg 127140
tggaaaaaaa tccgtcgcgg tttgagcgcg ggccgtgtac aaagccccgc actgcgtttg 127200
atttgcgaac gcgaaaacga aatccgcgcg tttgaagcgc aggaatattg gacggtacat 127260
ctagacagcc acaaaggccg cagcaagttc accgccaaac tcgcccaata caacggcgcg 127320
aaactcgaac aattcgacct gccgaacgaa gccgctcaag ccgatgtgtt gaaagaactc 127380
gaaggcaaag aggccgtcgt taccgccatc gaaaagaaaa agcgcagccg caaccccgcc 127440
gcgccgttta ccacatccac catgcagcag gatgctgtgc gcaaactcgg cttcaccacc 127500
gaccgcacca tgcgtaccgc ccagcagctt tacgaaggta ttgacgtagg gcagggtgcc 127560
atcggtctga ttacctatat gcgtaccgac agcgtgaact tggcggatga agccttaacc 127620
gaaatccgcc attacattga aaacaaaatc ggcaaagaat atctgccgag tgccgccaaa 127680
caatacaaaa ccaaatccaa aaacgcccaa gaagcgcacg aagccatccg cccgacttcc 127740
gtgtaccgca cgcccgaaag cgtcaaaccc ttcctgagcg ccgaccagtt caaactctat 127800
caaatgattt ggcagcgtac cgtcgcctgt cagatgacgc ccgccaaatt cgaccaaacc 127860
accgtcgata ttaccgtcgg caaaggcgta ttccgcgtaa ccggacaagt gcaaaccttc 127920
gcaggcttcc tcagcgttta cgaagaaagc agcgacgatg aagaaggcga agacagcaaa 127980
aaactgcccg aaatgagcga aggcgacaaa ttgcccgtgg acaaactcta cggcgaacaa 128040
cactttacca ctccgccgcc acgctacaac gaagccacgc tggttaaagc cctcgaagaa 128100
tacggcatcg gccgcccctc gacctacgcc agcatcatct ccacgctcaa agaccgcgaa 128160
tacgttaccc ttgagcaaaa acgctttatg cccaccgaca caggcgacat cgtcaataaa 128220
ttcctgaccg aacacttcgc ccaatacgtc gattaccact tcactgccaa actcgaagac 128280
cagcttgacg aaattgccga cggcaaacgc caatggattc ccttgatgga caaattctgg 128340
aaaccgttca tcaaacaagt ggaagaaaaa gaaggcatcg aacgcgccaa atttaccacg 128400
caggaacttg atgaaacctg cccgaaatgc ggcgaacaca aactgcaaat caaattcggc 128460
aaaatgggtc gttttgttgc gtgtgccggt tatcccgagt gcagctacac gcgcaatgtc 128520
aacgaaaccg ccgaagaagc tgccgaacgc atcgccaaag ccgaagccga acaggccgaa 128580
ctcgacggac gcgagtgccc gaaatgtggc ggtcgcctag tgtacaaata cagccgcacc 128640
ggcagcaaat tcatcggctg cgtcaactat ccgaaatgca aacacgtcga gccgctggaa 128700
aaaccgaaag ataccggcgt ccagtgtccg caatgcaaaa aaggcaacct cgtcgagcgc 128760
aaatcccgct acggcaaact gttttacagt tgcagcacct atcccgactg caactacgcc 128820
acttggaacc cgcccgttgc cgaagaatgc ctgaactgcc attggccggt cttgaccatc 128880
aaaaccacta aacgctgggg tgtagaaaaa gtctgcccac aaaaagaatg cggctggaaa 128940
gaacagattg aaccgcccgc gccgaaggag taagattagg ttggtttgaa agagaaaagg 129000
tcgtctgaaa aattttcaga cgacctttgc ttttctgtga ttggtttatt tgaatccgcg 129060
tgttgtttta aagtccgata aaatccggtt catttcaggc gcaaacaagg cgatgtaatc 129120
gtaagataga ccgcgactgg cactgggatg gggaaagcag acgacttcgc aatcttcaaa 129180
cgattggaat ttgacattga aacgtgtacc gtcaaattct ttttgcaccg tctccagcgg 129240
tttggtctgc ttaccgacca actgctcgaa gcgtggcagt acattttggt tgttcagaaa 129300
atccgccaac ctgctgccca tgaagaggat gactttcgga cgcagttttt cgatgtggta 129360
gagaaaatta tcgatgtgct cgggttgtgt gaacttgtcg ggattgtcga tagtgttgcc 129420
ctgtgtagca gcccagttgg tttgaaccag ggatttttca aatgcaccgc ccaatccatt 129480
ttcgtctaag ggtgtcccc acatttcaaa ccaatttttt atcgtattgt cgtaacgcca 129540
ctttttgcc tgctctccga aatagaggga tttgtttgca aatgtatggt cgattttgtt 129600
ttcagggagt ttgtattcac ctgctacata agcagcctca tcggctttac tccaacccca 129660
ttcatagcca caaatcatta agccatgttt gtcgttgtag cctttgaaca ggctgttgct 129720
caaattcaaa tccttcatca tgaactcttc cttttaaaat ttaagagcga ttgacttcaa 129780

tgtttttaga tggggtggaa aaatccttgt gtaggcaaca taaattcaat aaatttcttg 129840
ataattcgaa acctactaat agcgcaccta taaaagcttt ttcattacgt tcagcatgac 129900
ggtcacgtcg ttcatatttt ttacgcttgc tgttccctgt tattacagct aagccaagtg 129960
atatggcgag aattgcccaa acaatagtac ttaataacaa ttttccccat actatcaata 130020
aggaaagaaa aaacctttta gtattagatc gataggttat aatccatgcc catgaaaatg 130080
caataagagt tatacataag atacaagctg acaggatttc tttattttta attaaataac 130140
ttagagcgat gaggatgaca ggtgtcaaga aaataatagt tacatcccga tagctataaa 130200
agaaaactgc cctattttga atgtggagat gtgcacagaa tccaatatag ctaaggataa 130260
tagttaatat aataaaaaaa gaccaccaag ggtgaagaga taggaattcc atgttttccc 130320
ttttttgta aaaaggaaaa aatcttatct aatagttaat tgcctaacca gccagaagaa 130380
gtttaaaatc tatcccaata attcaaccat ctatacagaa agttcagctt atggaaaccc 130440
acgaaaaaat ccgcctgatg cgcgaattga ataaatggtc ccaggaggat atggcggaaa 130500
agctggcgat gtcggcaggc gggtatgcca aaatcgaacg gggcgaaacg cagttaaata 130560
tcccgcgttt ggagcagttg gctcagattt tcaaaatcga tatgtgggac ttgctcaaat 130620
cgggcggtgg tgggatggtg tttcagatta atgaaggtga tagtggtggc gatattgcgt 130680
tgtatgcgtc gggtgatgtt tcgatgaaaa tagaattttt aaaaatggag ttgaaacact 130740
gcaaagaaat gttggaacaa aaagacaaag aaatcgagct gctccgcaag ctgaccgaaa 130800
ccgtttaaac agatatgccg tctgaaaaaa gttttcagac ggcatattct ttgacaggtc 130860
ttgtataata ccgtttgaac ttacaggttt ttgattatgg cggcaggcaa acataccaaa 130920
cacagcaacc gggtacgcat tatcggcggg caatgccggg gcaggaaatt gagtttcaca 130980
tccgccgacg gactgcgtcc gacacccgac agcgtgcgtg aaaagctgtt taactggctg 131040
ggacaggatt tgacgggtaa aacggttttg gatctcttcg gaggcagcgg cgcactcggt 131100
atagaagccg cttcgcgcaa cgccaaacgc gtgctgattt cggataacaa ccgccaaacc 131160
gtgcagacct tgcagaaaaa cagtcgcgaa ctgggtttgg ggcaggtgca aatcgtcttt 131220
tcagacggca tcgcatattt gaagaccgta tccgaacagt ttgatgttgt ctttctcgac 131280
ccgccgtttg catggcagga ctggcaaatc ctgttcgatg ccttgaagcc gtgcctgaac 131340
ccccgggcat tcgtctatct cgaggcgggt acgctgccga atattcccga ttggctgacg 131400
gaatatagag aagggaaatc ggggcagagt acatttgaat taagggtttt ccaagtggct 131460
gaataatatg cgctttgata atcatttccg agttgtaaac attcgtttgc aaccgtccgg 131520
ttcaaaaaaa ccttgtgcta taatccgcgc ccgcccggtt ttgataattt agtggaaaag 131580
gaaaagaaat gtcgcttttt attaccgacg agtgcatcaa ctgcgacgta tgcgaacccg 131640
aatgccccaa tgatgccatt tcccaaggcg aggaaattta cgaaatcaac cccaacctct 131700
gcacgcagtg cgtcggacac tacgatgagc cgcagtgcca gcaggtttgc ccggtggact 131760
gcatcctgat tgacgaagaa catcccgaaa cccatgacga gttgatggcg aaatacgaaa 131820
agattatcca gtttaaataa attcttttta aaacatcaaa ttatgtctgt tttgaaataa 131880
aatcaaaaaa aaacttgacg gaaaagcaag ccgctaataa actaacgttc tcttttggag 131940
ggattcccga gcggtcaaag ggggcagact gtaaatctgt tgcgaaagct tcgaaggttc 132000
gaatccttct ccctccacca aaattcttac ttggggcagt agcgagtaat gcgggtgtag 132060
ctcaatggta gagcagaagc cttccaagct tacggtgagg gttcgattcc cttcacccgc 132120
tccaaacaat taggcccatg tagctcaggg gtagagcact cccttggtaa gggagaggtc 132180
ggcagttcaa atctgcccat gggcaccatc tctcgattat tcatttcttt aaggcttaga 132240
tatataggat attgccatgg ctaaggaaaa attcgaacgt agcaaaccgc acgtaaacgt 132300
tggcaccatc ggtcacgttg accatggtaa aaccaccctg actgccgctt tgactactat 132360
tttggctaaa aaattcggcg gtgctgcaaa agcttacgac caaatcgaca acgcacccga 132420
agaaaaagca cgcggtatta ccattaacac ctcgcacgtg gaatacgaaa ccgaaacccg 132480
ccactacgca cacgtagact gcccggggca cgccgactac gttaaaaaca tgattaccgg 132540
cgccgcacaa atggacggtg caatcctggt atgttccgca gccgacggcc ctatgccgca 132600
aacccgcgaa cacatcctgc tggcccgcca agtaggcgta ccttacatca tcgtgttcat 132660
gaacaaatgc gacatggtcg acgatgccga gctgttggaa ctggttgaaa tggaaatccg 132720
cgacctgctg tccagctacg acttccccgg cgatgactgc ccgattgtac aaggttccgc 132780
actgaaagcc ttggaaggcg atgccgctta cgaagaaaaa atcttcgaac tggctgccgc 132840
attggacagc tacatcccga ctcccgagcg agccgtggac aaaccgttcc tgctgcctat 132900
cgaagacgtg ttctccattt ccggccgcgg tacagtagta accggccgtg tagagcgcgg 132960
tatcatccac gttggtgacg agattgaaat cgtcggtctg aaagaaaccc aaaaaaccac 133020
ttgtaccggt gttgaaatgt tccgcaaact gctggacgaa ggtcaggcgg gcgacaacgt 133080
aggcgtattg ctgcgcggta ccaaacgtga agacgtggaa cgcggtcagg tattggctaa 133140
accgggtact atcactcctc acaccaaatt caaagcagaa gtatacgtac tgagcaaaga 133200
agagggtggt cgtcacactc cgttcttcgc caactaccgt ccgcaattct acttccgtac 133260
caccgacgta accggcgcgg ttactttgga agaaggtgta gaaatggtaa tgccgggtga 133320
aaacgtaacc atcaccgtag aactgattgc gcctatcgct atggaagaag gcctgcgctt 133380
tgcgattcgc gaaggcggcc gtaccgtggg tgccggcgtg gtttcttctg ttatcgctta 133440

agtttagagg ccaatagctc aattggtaga gtatcggtct ccaaaaccga gggttggggg 133500
ttcgagaccc tcttggcctg ccaaataaaa aattaaccgg ccttgtgtcg gttaattttt 133560
ttgtatttgt tatttagtaa actctcttgc catttacatg gattgagaat agacagatgc 133620
tatgatggat aaataatatg acagaacata cgcctgaaaa aaagaacgtt aaagtggatc 133680
aactggttgt tcaagataaa gaatctgcat ctaattccgg taaggaaggg tttttttgcat 133740
atttctcaaa ttcttggtcc gaattcaaaa aggtggtttg gcctaagcgt gaagatgctg 133800
tcagaatgac tgtatttgtt atagtgtttg ttgctgtgct ttctatattt atctatgcgg 133860
cagatacagc aatttcgtgg ttattttttg atgtattgct gagaagggaa ggttgagatg 133920
tcgaaaaaat ggtatgttgt acaggcgtat tcggggtttg agaagaatgt ccaacgaata 133980
ttggaagagc gcattgcccg tgaggagatg ggagattatt tcggacaaat tctggtgcct 134040
gtagagaaag ttgttgatat ccgcaatggt cgtaagacta ttagtgaaag aaagtcatat 134100
cctggttatg tgctagttga gatggaaatg acagatgact cttggcatct tgtaaaaagc 134160
acccccccgtg tttccggttt tattggaggg agggctaata gacctacgcc gattagtcag 134220
agagaggctg aaattatttt acagcaggtt cagaccggca tagagaagcc gaaaccaaaa 134280
gttgaatttg aggtcggtca acaggttcgt gtaaatgaag ggccgtttgc ggattttaac 134340
ggggtggttg aggaggtcaa ttatgaacgg aataagttac gcgtgtctgt tcagatattt 134400
ggtagagaaa cacccgttga gctggagttc agccaggttg aaaagattaa ctgattttta 134460
tacttgaaaa aaaagcaata agaggataga atcaaaaatt aacttgggga gcggaaatgg 134520
ttccgcgtct tacccgtttt taggagttcg ttaagtggca aagaaaatta tcggctatat 134580
taaactgcaa attcctgcag gtaaagccaa tccatctcct ccggttggtc ctgctttggg 134640
tcagcgcggt ttgaatatta tggaattttg taaggcattt aatgctgcaa cccaaggtat 134700
ggagcctggc ttaccgattc cggttgtgat tactgcattt gcagataaat cattcacatt 134760
tgtgatgaaa accccgccag cttctatctt gttgaaaaag gctgccggtt tgcaaaaagg 134820
tagttctaat cctctgacca acaaagtggg taaattgacc cgtgcccagt tggaagaaat 134880
tgctaaaact aaagatcctg atttgactgc tgctgacttg gatgcggctg tccgtactat 134940
agcaggttct gctcgctcaa tgggcttgga tgtggagggt gttgtataat ggctaaagta 135000
tctaaacgct tgaaagctct tcgctcttct gtggaagcca ataaattata tgcaattgat 135060
gaagcaattg ctttggtaaa aaaagcagcg actgctaaat ttgacgagtc tgttgacgta 135120
tctttcaact tgggcgttga tccgcgtaaa tctgaccaag ttatccgtgg ttcggtcgtt 135180
ctgcctaaag gcaccggtaa gataacccgt gtggctgtat ttactcaagg tgcaaatgca 135240
gaagctgcta aagaagctgg tgcagatatc gtcggtttcg aagatttggc tgctgaaatc 135300
aaagcaggca atctgaactt tgatgtcgtt attgcttctc ccgatgcaat gcgtattgtt 135360
ggtcagttgg gtactatttt gggtcctcga ggcttgatgc caaaccctaa agtaggtacg 135420
gttactccta acgttgctga agcagttaag aatgcaaaag caggtcaagt acaataccgt 135480
acagataaag caggtatcgt tcatgcaacg attggtcgtg cttctttcgc tgaagctgat 135540
ttgaaagaga actttgatgc gttgctggat gctatcgtta aagccaagcc tgctgccgct 135600
aaaggtcagt atctgaaaaa agttgctgtg tctagcacca tgggtttggg tattcgcgtt 135660
gatacatcaa gcgtaaataa ctaatcttaa ggaattttca agcagtttgg ttttctgggc 135720
tgcttgaatt tgggctactt aaaattaagt agatgtccaa gaccgtaggg atcgtaagat 135780
ttaatcgtaa ctgccctacg cagacggtag tcctgaaaca cattgcaaga ttgcttgtaa 135840
gatgtctttt taggttaccg cgctggtggg atatcgtttt ggtatcctgt ttataaacag 135900
tgggaggtag accttgagtc tcaatattga aaccaagaaa gtggcggtcg aggaaattag 135960
cgcggcaatt gctaatgctc aaaccctcgt agtcgctgaa tatcgcggta tcagtgtttc 136020
cagtatgact gagcttcgtg cgaatgcacg taaagaaggc gtttatttgc gcgttctgaa 136080
aaatactttg gctcgtcgtg cagtgcaagg tacttcattt gcagaattgg ccgatcaaat 136140
ggttggtccg ttggtttacg ctgcttctga agatgctgtt gctgctgcta aagtgttgca 136200
ccaattcgcg aaaaaagatg acaaaattgt cgttaaagcc ggttcttaca atggcgaagt 136260
aatgaatgct gctcaggttg ctgagttggc ttctattccg agccgcgaag agctgttgtc 136320
caaactgttg ttcgttatgc aagctcctgt atcgggcttt gcgcgcggtt tggctgcttt 136380
ggcagagaaa aaagccggcg aagaagccgc ttaatcgatt ttgtttctgt taatcaatta 136440
tttttaata caatatttgg agtaaaatag catggctatt actaaagaag acattttgga 136500
agcagttggt tctttgaccg taatggaatt gaacgacttg gttaaagctt ttgaagaaaa 136560
attcggtgtt tctgctgctg ctgttgcagt tgcaggtcct gctggtgccg gtgctgccga 136620
tgctgaagaa aaaaccgaat ttgatgtcgt tttggcttct gccggcgatc aaaaagtcgg 136680
cgtgattaaa gttgtccgtg caattaccgg tttgggtctg aaagaagcta aagacatcgt 136740
tgacggcgca cctaaaacca ttaaagaggg tgtttctaaa gctgaagccg aagacatcca 136800
aaaacaactg gaagaagcag gcgctaaagt cgaaatcaaa taatttgatg cttcttatga 136860
aggctggcag ttttctgcca gccttatttt gcttcttaaa ataaacatca agtattgttt 136920
acatttattt gcatagtttt tatcaagtca ttgcaaataa atgtaaatat cagattgatg 136980
cgtaccgttg tttcagacgg cctattattg aaaattactt ttcggagtgt gtatgaacta 137040
ttcgtttacc gagaaaaaac gtatccgtaa gagttttgca aagcgggaaa atgttttgga 137100

agttcctttc ttgctagcaa cccaaattga ttcttatgcg aagtttttgc agctggaaaa 137160
tgcttttgac aaacgtaccg atgacggtct gcaggcggca tttaattcta ttttcccgat 137220
tgtgagccat aacggttatg cgcgattgga gtttgtgcat tacacattgg gcgagccttt 137280
gttcgatatt cccgaatgtc agttgcgcgg aatcacttat gcagccccct tgcgcgcgcg 137340
tatccgtttg gtgattttgg ataaggaagc atctaaaccg acggtaaaag aagttcgtga 137400
aaacgaagtg tatatgggcg aaattccgtt gatgaccccg agcggttctt ttgtgattaa 137460
cggcacagag cgtgtgattg tctcccagtt gcaccgttcg cccggcgtat tcttcgagca 137520
tgacaaaggt aagacgcact cttccggcaa attgttattc tccgcccgca tcattcccta 137580
ccgtggttca tggttggatt ttgaatttga tccgaaagat ttgctgtatt tccgtatcga 137640
ccgccgccgt aaaatgccgg taacgatttt gttgaaggct ttaggctaca acaatgagca 137700
aatcttggat attttctacg acaaagaaac gttctatttg tcttcaaacg gtgttcaaac 137760
cgatttggtt gcagaccgtc tgaaaggcga aactgccaag gtcgatatct tggataaaga 137820
aggcaatgta ttggttgcca aaggtaagcg cattactgcg aaaaatatcc gtgatattac 137880
caatgcaggc ctgacccgtt tggatgtaga accggaaagc ctgctgggca aagcattggc 137940
tgccgatctg attgattcgg aaaccggcga ggtattggct tctgccaatg atgaaattac 138000
agaagagttg ttggccaaat ttgatatcaa cggcgtaaaa gaaattacga ccctttatat 138060
caatgagctg gatcagggtg cttatatctc caataccttg cgtacggatg agactgccgg 138120
ccggcaggcg gctcgtgttg cgatttaccg tatgatgcgt ccgggcgaac cgcccaccga 138180
agaggcggtc gagcaattgt ttaaccgctt gttcttcagt gaagacagct acgatctgtc 138240
ccgcgtaggc cgtatgaaat ttaatacgcg cacatacgaa caaaaactgt ccgaagccca 138300
acaaaactct tggtacggcc gcctgctgaa cgaaacgttt gccggtgctg ccgacaaagg 138360
cggttatgtc ctgagcgtcg aagatattgt cgcctcgatt gcgactttgg tcgagttgcg 138420
taacggccat ggcgaagtgg acgatatcga tcacttgggc aaccgccgag tacgttcggt 138480
aggcgagctg actgaaaacc aattccgtag cggtttggcc cgtgtggaac gtgccgtaaa 138540
agaacgtttg aatcaggcgg aatcagaaaa cttgatgccg cacgatttga ttaatgcaaa 138600
acctgtttct gccgctatta aagaattctt cggctccagc caattgagtc agtttatgga 138660
tcagaccaac cccttgtctg aagtaaccca taaacgccgt gtatctgcat tgggtccggg 138720
cggtttgacc cgcgaacgtg caggatttga ggtgcgggac gtgcatccga cccactacgg 138780
tcgcgtatgt ccgattgaaa cgcctgaagg tccgaacatc ggtttgatca actcattgtc 138840
cgtttatgcg cgcaccaatg attacggttt cttggaaacg ccttaccgcc gcgttatcga 138900
cggcaaagta accgaggaaa tcgattactt gtctgccatc gaagaaggcc gctatgtgat 138960
tgcacaggcg aatgccgatt tggattcaga tggcaatctg attggcgatt tggttacctg 139020
tcgtgaaaaa ggcgaaacca ttatggcaac gcccgaccgc gtccaatata tggacgtggc 139080
aactggtcaa gtggtatccg ttgcagcatc cctgattcca ttcttggaac atgatgacgc 139140
gaaccgcgca ttgatgggtg ccaacatgca acgtcaggca gtgccttgct tgcgtcctga 139200
aaaaccgatg gtcggtaccg gtatcgagcg ttccgttgcc gttgactctg ctactgcaat 139260
cgttgcccgc cgaggcggcg tggtcgagta tgtcgatgcc aaccgcgttg tgatccgtgt 139320
ccatgacgac gaagcgactg ccggtgaagt gggtgtcgat atttacaact tggttaaatt 139380
cacccgttcc aaccagtcta ccaatatcaa tcagcgtcct gccgtcaaag ccggcgatgt 139440
tttgcaacgc ggcgatttgg tggccgacgg cgcgtccacc gattttggcg aattggcttt 139500
gggtcaaaat atgaccatcg ccttcatgcc gtggaacggt tacaactacg aagactcgat 139560
tctgatttcc gaaaaagtgg ctgcggacga ccgctatact tcgattcaca ttgaggaatt 139620
gaatgtcgtt gcccgcgata ctaagctggg tgcggaagac attacccgcg atattccgaa 139680
cttgtccgag cgtatgcaaa accgtttgga cgaatccggt atcgtttaca tcggtgcgga 139740
agtagaagcc ggcgatgtgt tggtaggcaa ggtaacgcct aaaggcgaaa cccaactgac 139800
gccggaagaa aaactgctgc gcgccatctt cggtgaaaaa gcatctgacg taaaagatac 139860
ttcattgcgt atgcctaccg gcatgagcgg taccgttatc gacgttcaag tcttcactcg 139920
tgaaggtatt caacgcgaca aacgtgctca atccattatc gattccgaat tgaaacgcta 139980
ccgtttggat ttgaacgacc aattgcgtat tttcgacaac gacgcattcg accgtatcga 140040
gcgtatgatt gtcggtcaga aagccaacgg tggtccgatg aagctggcca aaggcagcga 140100
aatcacgacc gaatatctgg cgggtctgcc gagcaggcac gattggttcg atatccgtct 140160
gaccgatgaa gatttggcca agcagttgga actgattaaa gtgagcctgc aacaaaaacg 140220
cgaagaagcg gacgagttat acgaaatcaa gaagaaaaaa ctgacccaag gcgacgaatt 140280
gcaacccggc gtacaaaaaa tggtgaaagt ttttatcgcc atcaaacgcc gtctgcaagc 140340
cggcgacaaa atggcgggcc gccacggtaa caaaggcgtg gtatcgcgca ttctgccagt 140400
ggaagacatg ccttacatgg cggacggccg tccggtagac atcgtactga acccattggg 140460
cgtaccttcc cgtatgaaca tcggtcagat tttggaagtt cacttgggtt gggcagcaaa 140520
aggtatcggc gagcgtatcg accgtatgct gaaagagcaa cgcaaagcag gcgagttgcg 140580
cgagttcttg aacagactct acaacggcag cggtaagaaa gaagatttgg atgccctgac 140640
tgatgaagaa atcatcgaac tggcctccaa cctgcgcaaa ggtgcatctt tcgcctctcc 140700
tgtattcgac ggtgcgaaag agtctgaaat ccgcgaaatg ctgaacttgg cttatccgag 140760

cgacgatcct gaggttgaaa aactgggctt caacgacagt aaaacccaaa tcacgctgta 140820
tgacggccgt tcaggcgaag catttgaccg caaggttaca gtaggtgtga tgcactatct 140880
gaaactgcac cacttggttg acgaaaaaat gcacgcgcgt tctaccggtc cgtacagtct 140940
ggttacccag cagcctttgg gcggtaaagc ccagttcggc ggccaacgtt tcggcgagat 141000
ggaggtttgg gcattggaag catacggcgc ggcatacacg ctgcaagaga tgctgactgt 141060
gaagtctgac gacgtgaacg gccgtaccaa aatgtacgaa aacatcgtca aaggcgaaca 141120
caaaatcgat gccggtatgc ccgagtcctt caacgtattg gtcaaagaga ttcgctcact 141180
gggcttggat atcgatttgg aacgttacta aacaaaagtt ttcagacggc ctttcagggt 141240
cgtctgaaaa agtggtttca gaataagaat gaagcaatcg gcatttaggc cgtctgaaat 141300
caaaagtacc gtttcccaat atcgaaaatc cgccatgcgg taaaaatact tccttcaagg 141360
agcaaaaatg aatttgttga acttatttaa tccgttgcaa actgccggca tggaagaaga 141420
gtttgatgcc attaaaatcg gtattgcctc tcccgaaacc atccgctcat ggtcttatgg 141480
cgaagtcaaa aaacctgaaa ccatcaacta ccgtacgttc aaacctgagc gtgacggttt 141540
gttctgtgcc aaaatctttg gcccggtcaa agactacgaa tgcttgtgcg gaaaatacaa 141600
acgcttgaaa tttaaaggcg taacgtgtga aaaatgcggc gtggaagtaa ccctgtccaa 141660
agtgcgccgc gaacgcatgg gtcatatcga attggctgcg cccgtcgcac atatttggtt 141720
cttaaaatcc ctgccttccc gcttgggtat ggtgttagac atgactttgc gcgacatcga 141780
gcgcgtattg tactttgaag catttgtggt aaccgatccc ggtatgactc cgctgcaacg 141840
ccgccaattg ctgactgaag acgattacta caacaagctg gacgaatacg gcgacgattt 141900
cgatgccaaa atgggtgcgg aaggtatccg cgaattgctg cgtaccctga atgtagcggg 141960
cgaaatcgaa atcctgcgcc aagagttgga atcgaccggt tccgacacca aatcaaaaa 142020
aatcgccaaa cgcttgaaag tattggaagc cttccatcgt tccggtatga aactggaatg 142080
gatgattatg gatgtgctgc cggtattgcc gcctgatttg cgtccgttgg ttccattgga 142140
tggtggtcgt tttgccactt ccgatttgaa cgatttgtac cgccgcgtta ttaaccgtaa 142200
caaccgtctg aaacgtctgt tggaactgca tgcgcctgac atcatcgtcc gcaacgaaaa 142260
acgtatgttg caagaagcag ttgactcgct gttggataac ggccgtcgcg gtaaagccat 142320
gaccggcgcc aacaaacgcc cgctgaaatc attggcagac atgattaaag gtaaaggcgg 142380
tcgcttccgt caaaacctgt tgggcaaacg tgtggactac tccggccgtt ccgtgattac 142440
cgtaggcccg tacctgcgtc tgcaccaatg cggtttgccg aaaaaaatgg ctttggaact 142500
gttcaaaccg ttcattttcc acaaattgga aaaacaaggt ttggcctcta ccgttaaagc 142560
agcgaaaaaa ttggtagagc aagaagtacc ggaagtatgg gacatcttgg aagaagtcat 142620
ccgcgaacat ccgattatgc tgaaccgtgc gccgaccctg caccgtttgg gtattcaagc 142680
gttcgaacct atcttgattg aaggtaaagc gattcagttg cacccattgg tgtgtgctgc 142740
gttcaacgcc gactttgacg gcgaccaaat ggcggtacac gttccattga gcttggaagc 142800
acaaatggaa gcacgcacgc tgatgctggc ttcaaacaac gtattgtctc cggccaacgg 142860
cgaaccgatt atcgtacctt cccaagacat cgtattgggc ctgtactata tgactcgcga 142920
tcgtatcaat gccaaaggcg aaggcagcct gtttgccgat gtgaaagaag tgcatcgcgc 142980
ataccatacc aaacaggtcg agctgggtac gaaaatcacc gtacgtctgc gcgaatgggt 143040
gaaaaacgaa gcaggtgagt ttgagcctgt cgttaaccgt tacgaaacaa ccgtcggccg 143100
tgcattgttg agcgaaatcc tgccgaaagg cctgccgttt gaatatgtca acaaagcgtt 143160
gaagaaaaaa gaaatttcta aactgattaa cgcatcgttc cgcctgtgcg gcttgcgcga 143220
tacggttatc tttgctgacc acctgatgta caccggtttc ggatttgcgg caaaaggcgg 143280
tatttccatt gccgttgacg atatggaaat tccaaaagaa aaagcggcct tgctggctga 143340
agccaatgcc gaggttaaag aaatcgaaga ccaataccgt caaggtttgg ttaccaacgg 143400
cgaacgctac aacaaggtgg tcgatatttg ggtcgtgcc ggcgataaga ttgctaaagc 143460
gatgatggac aacttgtcca aacaaaaagt tatcgaccgt gccggcaacg aagtcgatca 143520
agagtcattc aactccattt atatgatggc ggactccggt gcccgtggtt ctgcagctca 143580
gattaaacag ttgtccggta tgcgtggctt gatggcaaaa cctgacggct cgattattga 143640
aacgccgatt acctcaaact tccgtgaagg tctgaccgta ttgcaatact ttattgcgac 143700
ccacggtgcg cgtaagggtt tggcggatac cgcattgaaa accgcgaact ccggttacct 143760
gactcgtcgt ctggtagacg taactcaaga tttggtcgtt gttgaagacg attgcggtac 143820
ttcagacggc tttgtcatga aggcagtggt acaaggcggt gatgtgattg aagcattgcg 143880
cgatcgtatt ttgggtcgtg ttaccgcgtc tgacgttgtc gatccgtcaa gtggcgaaac 143940
cttggttgaa gccggtacgt tgctgactga aaaactggtg gatatgatcg accaatccgg 144000
tgtcgatgaa gtcaaagtcc gtacgccgat tacttgtaaa acccgtcacg gcctgtgtgc 144060
acactgttac ggtcgtgact tggcacgcgg caaactggtt aacgccggtg aggcagtcgg 144120
tgtgattgct gcacaatcca ttggcgaacc gggtacccag ttgaccatgc gtacgttcca 144180
catcggtggt gcggcatccc gtgcggcagc agccagccaa gtggaagcca aatccaacgg 144240
tacggcacga ttcagcagcc agatgcgcta cgttgccaac aacaaaggcg agttggttgt 144300
catcggccgt tcttgtgaag tcgtgattca cgacgatatc ggccgtgaac gcgaacgcca 144360
caaagtacct tacggtgcca tcctgctggt acaagacggt atggccatta aagccggtca 144420

aaccttggca acctgggatc cgcatacccg tccgatgatt accgaacacg caggtatggt 144480
gaaattcgaa aacgtggaag agggcgttac cgttgccaaa caaaccgatg atgtaaccgg 144540
tttgtccact ttggtggtga ttgacggtaa acgtcgttcc tctagtgctt ccaaactgct 144600
gcgtccgact gtgaaactct tggacgaaaa cggcgtggaa atctgtattc ccggtacttc 144660
tactccggta tccatggcat tccccgttgg tgcggtgatt accgtacgcg aaggtcagga 144720
aatcggtaaa ggcgacgtat tggcgcgtat tccgcaagcc tcttccaaaa cccgcgacat 144780
taccggcggc ctgccgcgcg ttgccgaatt gtttgaagca cgcgtgccga aagatgccgg 144840
tatgttggcg gaaattaccg gtaccgtttc cttcggcaaa gagaccaaag gcaagcaacg 144900
tctgattgtt actgacgtgg acggtgtagc atacgagacc ttgatttcca aagagaaaca 144960
aattctggta cacgacggtc aagtggtaaa ccgcggtgaa accatcgtgg acggcgcggt 145020
cgatccgcac gatattctgc gtttgcaagg tatcgaagca ctggcacgct acattgtcca 145080
agaggtgcaa gaggtttacc gtctgcaagg tgtgaagatt tctgataaac acatcgaagt 145140
catcatccgt caaatgttgc gccgtgtgaa cattgcggat gccggcgaaa ccgggttcat 145200
taccggagag caggtcgaac gcggcgatgt gatggcggcc aatgaaaaag ctttggaaga 145260
aggcaaagaa ccggcgcgtt acgaaaacgt attgctgggt attaccaaag cttccctgtc 145320
caccgacagc ttcatttctg ccgcatcgtt ccaagaaacg acccgcgttc tgaccgaagc 145380
cgcgattatg ggcaaacaag acgagttgcg tggtttgaaa gaaaacgtca tcgtcggtcg 145440
cttgattcct gccggtaccg gtttgactta ccaccgcagc cgtcatcaac aatggcaaga 145500
ggtggaacag gagactgccg aaacccaagt aacggatgaa taatctttgg tgcatccatt 145560
caataaaaaa ccgcaagcct tgagcttgcg gtttttcttt gtccgattaa ggcaaaaaca 145620
agcgttttcg tcattttgag gcgtgtggat tattccttag gtattttcgg gccggagacc 145680
aacgaggtgg cgggtgtcgt cggtacgtcc ggagaccaaa ataactttgc cagggatgtt 145740
ggtttcggcg gtcaaaaaaa gtagcgtctt aatgttttcc atttaaacaa atgtcgtctg 145800
aaacttcaga cggcatttcc tttaagaaat aaatatgaaa cccagaaatc tctttttttgc 145860
aggctgcctg ctgacttcgg cgacgtttgc cgaggatatc ggcgtacctg tcgaactgat 145920
taacgtcggt aatcggattg cgatgccgtc tgaaggggaa agcctcgccc tcctgccgtt 145980
tgccgaggat gtaccgccgg ttcgcgatgc aatgccgtct gaagttccta aaagcgcggc 146040
aggcggcgat gttcggggtg accggatgag aatgccgatt aacatcggat gagcgcggct 146100
ttatggcata aaaaactgtc gtggaaagga tttacacccc aaataaattt ccgttacaac 146160
aagatcaaca gcaatatgcc cgccttttat tcgcgcagcg gcaaggaacg gtttgtcagt 146220
atagaaaaaa cgtattgaca gtattttctt cagtcgtccg actgattgtg agggatgtcg 146280
gtaaatattt atcggcaaac aagaaaatca tctttcttct tgtcgttatg cttgactgtc 146340
tgcttgcaat aaaaatataa ttccactctt gccgacatgg tgtcggcaag tatttaactc 146400
aacaggacga gaaaatatgc caactatcaa ccaattagta cgcaaaggcc gtcaaaagcc 146460
cgtgtacgta aacaaagtgc ccgcactgga agcttgcccg caaaaacgtg gcgtgtgcac 146520
ccgtgtatac acaactaccc ctaaaaaacc taactctgca ttgcgtaaag tatgtaaagt 146580
ccgcctgacc aacggttttg aagtcatttc atacatcggc ggcgaaggtc acaacctgca 146640
agagcacagt gtcgtattga ttcgcggcgg tcgtgtaaaa gacttgccag gtgtgcgtta 146700
ccacactgta cgcggttctt tggatactgc aggtgttaaa gaccgtaaac aagcccgttc 146760
caaatacggt gctaagcgtc ctaaataatt actgggactt aaataggcac gtcggccgcc 146820
taagctgaac aacggccgag taagtgaata ctcaattggg tattcatggg aatagacccg 146880
actgaataga ttaaaggaaa ttaaaatgcc aagacgtaga gaagtcccca agcgcgacgt 146940
actgccagat cctaaattcg gcagcgtcga gttgaccaaa ttcatgaacg tattgatgat 147000
tgacggtaaa aaatccgttg ccgagcgtat cgtttacggt gcgttggaac agattgagaa 147060
aaaaaccggc aaagtagcaa tcgaagtatt taacgaagcc attgcaaacg ccaaacctat 147120
cgtggaagtg aaaagccgcc gtgtaggtgg tgcaaactac caagttcctg ttgaagttcg 147180
tccttcacgc cgtttggctt tggcaatgcg ctgggttcgc gatgcggccc gcaaacgtgg 147240
tgagaaatcc atggacctgc gtttggcagg cgaattgatt gatgcgtccg aaggccgtgg 147300
cggtgcgttg aaaaaacgtg aagaagtaca ccgtatggct gaagccaaca aagcattctc 147360
tcacttccgt ttctaatttt gaaaggctaa taaaatggct cgtaagaccc cgatcagcct 147420
gtaccgtaac atcggtattt ccgcccatat tgacgcgggt aaaaccacga cgacagaacg 147480
tattttgttc tataccggtt tgacccacaa gctgggcgaa gtgcatgacg gtgcggctac 147540
taccgactac atggaacaag agcaagagcg cggtattacc attacctccg ctgccgttac 147600
ttcctactgg tccggtatgg cgaaacaatt ccccgagcac cgcttcaaca tcatcgacac 147660
cccgggacac gttgacttta ccgtagaggt agagcgttct atgcgtgtat tggacggcgc 147720
ggtaatggtt tactgcgcgg tgggcggtgt tcaaccccaa tctgaaaccg tatggcggca 147780
agccaacaaa taccaagtgc cgcgcttggc gtttgtcaat aaaatggacc gtcagggtgc 147840
caacttcttc cgtgttgtcg agcaaatgaa aacccgtttg cgcgcaaacc ctgtacctat 147900
cgtcattccg gttggtgcgg aagacaactt cagcggtgtg gttgatttgt tgaaaatgaa 147960
atccatcatt tggaatgaag tcgataaagg tacaaccttt acctatggcg atattcctgc 148020
cgaattggtc gaaactgccg aagaatggcg tcaaaatatg attgaagccg cagccgaagc 148080

cagcgaagaa ctgatggaca aatacttagg cggcgacgag ctgaccgaag aagaaatcgt 148140
aggcgcgttg cgtcaacgta ctttggcagg cgaaattcag cctatgctgt gtggttctgc 148200
atttaaaaac aaaggtgttc aacgtatgtt ggacgcagtt gtagaattgc tgccagctcc 148260
taccgatatt cctccggttc aaggtgtcaa cccgaatacc gaggaagccg acagccgtca 148320
agccagcgat gaagagaaat tctctgcatt ggcgttcaaa atgttgaacg acaaatacgt 148380
cggtcagctg acctttatcc gcgtttactc aggcgtagta aaatccggcg ataccgtatt 148440
gaactccgta aaaggcactc gcgaacgtat cggtcgtttg gtacaaatga ctgccgcaga 148500
ccgtactgaa atcgaagaag tacgcgccgg cgacatcgca gccgctattg gtctgaaaga 148560
cgttactacc ggtgaaacct tgtgtgcgga aagcgcgccg attatcttgg aacgtatgga 148620
attccccgag ccggtaatcc atattgccgt tgagccgaaa accaaagccg accaagagaa 148680
aatgggtatc gccctgaacc gcttggctaa agaagaccct tctttccgtg tccgtacaga 148740
cgaagaatcc ggtcaaacca ttatttccgg tatgggtgag ctgcacttgg aaattattgt 148800
tgaccgtatg aaacgcgaat tcggtgtgga agcaaatatc ggtgcgcctc aagtggctta 148860
ccgtgaaact atccgcaaag ccgttaaagc cgaatacaaa catgcaaaac aatccggtgg 148920
taaaggtcaa tacggtcacg ttgtgattga aatggaacct atggaaccgg gtggtgaagg 148980
ttacgagttt atcgatgaaa ttaaaggtgg tgtgattcct cgcgaattta ttccgtctgt 149040
cgataaaggt atccgcgata cgttgcctaa cggtatcgtt gccggctatc ctgtagttga 149100
cgtacgtatc cgtctggtat tcggttctta ccatgatgtc gactcttccc aattggcatt 149160
tgaattggct gcttctcaag cgtttaaaga aggtatgcgt caagcatctc ctgccctgct 149220
tgagccaatc atggcagttg aagtggaaac ccggaagaa tacatgggcg acgtaatggg 149280
cgacttgaac cgccgtcgcg gtgttgtatt gggtatggat gatgacggta tcggcggtaa 149340
aaaagtccgt gccgaagtac ctttggcaga aatgttcggt tactcgaccg acctgcgttc 149400
tgcaacccaa ggccgcgcta cttactctat ggagttcaag aaatattctg aagctcctgc 149460
ccacatagct gctgctgtaa ctgaagcccg taaaggctaa tcagaaaagg ccgtctgaaa 149520
ctgaaaataa attttcagac ggccattgtt ctttaatcga tctttatatg taaaggaatt 149580
agctcatggc taaggaaaaa tttgaacgta gcaaaccgca cgtaaacgtt ggcaccatcg 149640
gtcacgttga ccatggtaaa accactctga ctgctgcttt gactactatt ttgtctaaaa 149700
aattcggtgg cgctgcaaaa gcttatgacc aaatcgacaa cgctcctgaa gaaaaagctc 149760
gtggtattac cattaatacc tcacacgtag aatacgaaac tgaaacccgt cactacgcac 149820
acgtagactg cccggggcac gccgactacg ttaaaaacat gattaccggc gccgcacaaa 149880
tggacggtgc aatcctggta tgttccgcag ccgacggccc tatgccgcaa acccgcgaac 149940
acatcctgct ggcccgccaa gtaggcgtac cttacatcat cgtgttcatg aacaaatgcg 150000
acatggtcga cgatgccgag ctgttggaac tggttgaaat ggaaatccgc gacctgctgt 150060
ccagctacga cttccccggc gatgactgcc cgattgtaca aggttccgca ctgaaagcct 150120
tggaaggcga tgccgcttac gaagaaaaaa tcttcgaact ggctgccgca ttggacagct 150180
acatcccgac tcccgagcga gccgtggaca aaccgttcct gctgcctatc gaagacgtgt 150240
tctccatttc cggccgcggt acagtagtaa ccggccgtgt agagcgcggt atcatccacg 150300
ttggtgacga gattgaaatc gtcggtctga aagaaaccca aaaaaccact tgtaccggtg 150360
ttgaaatgtt ccgcaaactg ctggacgaag gtcaggcggg cgacaacgta ggcgtattgc 150420
tgcgcggtac caaacgtgaa gacgtggaac gcggtcaggt attggctaaa ccgggtacta 150480
tcactcctca caccaaattc aaagcagaag tatacgtact gagcaaagaa gagggtggtc 150540
gtcacactcc gttcttcgcc aactaccgtc cgcaattcta cttccgtacc accgacgtaa 150600
ccggcgcggt tactttggaa gaaggtgtgg aaatggtaat gccgggtgaa aacgtaacca 150660
tcaccgtaga actgattgcg cctatcgcta tggaagaagg cctgcgcttt gcgattcgcg 150720
aaggcggccg taccgtgggt gccggcgtgg tttcttctgt tatcgcttaa ttgaaggata 150780
ttgataaatg gcaaaccaaa aaatccgtat ccgcctgaaa gcttatgatt acgccctgat 150840
tgaccgttct gcacaagaaa tcgttgaaac tgcaaaacgt accggtgcag ttgtaaaagg 150900
cccgattcct ttgccgacca aaatcgagcg tttcaacatt ttgcgttctc cgcacgtgaa 150960
caaaacttcc cgtgagcaat tggaaatccg cacccacttg cgcctgatgg acatcgtgga 151020
ttggaccgat aaaactaccg atgcgctgat gaagctggat ttgccggccg gtgttgatgt 151080
agaaatcaaa gtccaataat tcggactata aaaaatcccc aagcaatcaa tgcttgggga 151140
ttttttatgt tatgccgaga cctttgcaaa attccccaaa atcccctaaa ttcccaccaa 151200
gacatttagg agcaccttct tccagcaaac cgcccaagcc atgattgcca aacacatcga 151260
ccggttccca ctattgaagt tggaccgggt aattgattgg cagccgatcg aacagtacct 151320
gaatcgtcaa agaacccgtt accttagaga ccaccgcggc cgtcccgcct atcccctgtt 151380
gtccatgttc aaagccgtcc tgctcggaca atggcacagc ctctccgatc ccgaactcga 151440
gcacagcctc atcacccgca tcgatttcaa cctgttttgc cgctttgacg aactgagcat 151500
ccccgattac agtcatcaac catattccgg tttgtcggag aaagatgcat acgctgtgat 151560
gaccggatac cgacccgtta aaagagtccg accctatgcc gtctgaaaat tcaaaacgct 151620
tcagacggca tattgaagat atttctgata tttctgttga tatttctttg acttgtcaga 151680
tataatgccg agcttggtac atttgtgcca agtttaactt tgtctgaaag acaggccaat 151740

cgtagcctgt ccctttactt taaaaggaaa ataatcatga ctttaggtct ggttggacgc 151800
aaagttggta tgacccgcgt gttcgacgaa cagggtgttt ctgttccggt aaccgttttg 151860
gatatgtctg ccaaccgcgt tacacaagta aaatccaaag atactgacgg ctatactgcc 151920
gttcaagtta cctttggtca gaaaaaagcc aatcgtgtca acaaagccga agccgggcac 151980
tttgcaaaag caggtgttga agccggtcgc ggtttgattg agtttgcttt gactgaagaa 152040
aaactggctg aattgaaagc tggtgacgaa atcaccgttt ctatgtttga agtcggtcaa 152100
ctggtcgatg taaccggtac ctctaaaggt aaaggtttct ccggcacgat taaacgtcat 152160
aacttcggtg cccaacgtac ttcccacggt aactcccgtt ctcaccgtgt tccaggctct 152220
atcggtatgg cgcaagaccc gggtcgcgtg ttccccggta aacgcatggc cggccaatac 152280
ggcaacacca aagcaactgt tcaaaaattg gaagttgtcc gtgttgacgc agaacgccaa 152340
ctgctgttgg ttaagggtgc tgttccgggt gcggtcaaca gcgatgttgt agttcgtccc 152400
agcgtgaaag taggtgcgta atggaattga aagtaattga cgctaaagga caagtttcag 152460
gcagtctgtc tgtttctgat gctttgttcg cccgcgaata caatgaagcg ttggttcatc 152520
agctggtaaa tgcctacttg gcaaacgccc gctccggtaa ccgcgctcaa aaaacccgtg 152580
ccgaagtaaa acactcaacc aaaaaaccat ggcgtcaaaa aggtaccggc cgtgcccgtt 152640
ccggtatgac ttcttctccg ctgtggcgta aaggtggtcg cgcgttcccg aacaaacccg 152700
acgaaaactt cactcaaaaa gtaaaccgca aaatgtaccg tgccggtatg gcgactattc 152760
tgtcccaatt gactcgtgac gagcgtttgt ttgcgattga ggcgttgact gccgaaactc 152820
ctaaaaccaa agtttttgcc gaacaagtga aaaatctggg tctggagcaa gtgttgtttg 152880
taaccaaaca gctcgacgag aatgtttact tggcttcacg caacttgcca aacgtgttgg 152940
ttttggaagc tcaacaagtt gatccttaca gcttgctgcg ttacaaaaaa gtaatcatca 153000
ctaaagatgc agttgcacaa ttagaggagc aatgggtatg aatcaacaac gtttgactca 153060
agtgattttg gcacctatcg tttctgaaaa aagcaacgta ttggctgaaa aacgtaacca 153120
aatgacgttt aaagttttgg caaatgcaac caaacctgaa attaaagcgg ctgttgagct 153180
gctgttcggc gttcaagttg cagacgttac tactgttacc attaaaggta aagttaaacg 153240
ttttggtcgc actttaggtc gtcgcagcga tgttaaaaag gcttatgtaa gcttggctgc 153300
cggtcaagag ttggatttgg aagccgctgc tgcagctgca gataaggaat aaacaaaatg 153360
gcaatcgtta aaatgaagcc gacctctgca ggccgtcgcg gcatggttcg cgtggtaaca 153420
gaaggtttgt acaaaggtgc accttatgca cctctgctgg aaaagaaaaa ttctactgcc 153480
ggtcgtaaca acaatggtca tattactacc cgtcataaag gtggtggtca taaacatcat 153540
taccgcgtcg tagattttaa acgtaacaaa gacggtatcc ctgcaaaagt agagcgtatc 153600
gaatatgacc ctaaccgtac tgcatttatc gcactgttgt gctatgcaga tggtgagcgt 153660
cgctacatta ttgctcctcg tggtattcaa gccggtgcag tattggtttc cggtgctgaa 153720
gctgcgatca aagtaggtaa cactctgccg atccgcaata ttcctgttgg tacaactatt 153780
cactgtatcg aaatgaaacc aggtaaaggt gcgcaaattg cacgttctgc cggtgcttct 153840
gcggtattgc tggctaaaga aggcgcgtac gctcaagtcc gcctgcgctc tggcgaagtc 153900
cgtaaaatca acgtagattg ccgtgcaacc atcggtgaag tcggtaacga agagcaaagc 153960
ctgaaaaaaa tcggtaaagc cggtgccaat cgttggcgcg gtattcgtcc gactgtacgt 154020
ggtgttgtca tgaaccctgt cgatcacccg catggtggtg gtgaaggccg tacgggcgag 154080
gcccgcgaac cggtcagccc atggggtact cctgctaaag gctaccgcac tcgtaataac 154140
aaacgcacgg ataacatgat tgttcgtcgc cgttactcaa ataaaggtta atttagtatg 154200
gctcgttcat tgaaaaaagg cccatatgta gacctgcatt tgctgaaaaa agtagatgct 154260
gctcgcgcaa gcaacgacaa acgcccgatt aaaacctggt ctcgtcgttc taccattctg 154320
cctgatttta tcggtctgac cattgctgtg cacaacggcc gcacccatgt gcctgtgttt 154380
atcagcgaca atatggttgg tcataaatta ggcgaattct cattgacccg tacctttaaa 154440
ggccacttgg ccgataaaaa ggctaaaaag aaataaggtg aatcatgaga gtaaatgcac 154500
aacataaaaa tgcccgtatc tctgctcaaa aggctcgttt ggtagctgat ttgattcgtg 154560
gtaaagacgt tgcccaagct ttgaatattt tggctttcag tcctaaaaaa ggtgccgagc 154620
tgattaaaaa agtattggag tcagctattg ctaatgccga gcacaataac ggtgcggaca 154680
ttgatgaact gaaagtggta actatctttg ttgacaaagg cccaagcttg aaacgttttc 154740
aagctcgcgc caaaggtcgc ggtaaccgca tcgaaaaaca aacttgtcat atcaatgtga 154800
cagtgggtaa ctaaggaaaa gctatgggac aaaagattaa ccctacaggc tttcgcctgg 154860
cggtaactaa agactgggct tcaaaatggt ttgctaaaag caccgacttt tctactgttt 154920
tgaagcagga tatcgatgtt cgcaattatt tgcgtcaaaa attggccaat gcttcggttg 154980
gtcgagtggt tattgaacgc cctgcaaaat ctgcacgcat taccattcac tccgctcgtc 155040
cgggtgtggt tatcggtaaa aaaggtgagg atatcgaggt tttgaaacgt gacttgcaag 155100
tcttgatggg tgtacctgtt catgtaaata ttgaagagat tcgccgtcct gagttggatg 155160
ctcaaattat tgctgacggt attgcccagc agttggaaaa gcgcgttcaa ttccgtcgtg 155220
ctatgaaacg agcaatgcaa aatgcaatgc gttctggtgc taaaggcatt aagattatga 155280
cttcaggccg tctgaatggt gcggatattg cccgtagcga atggtatcgt gaaggtcgcg 155340
tgccactgca tactttacgt gcaaatgtag attatgcaac cagcgaagcg cacaccacat 155400

atggtgtatt gggtctgaaa gtttgggttt atacggaagg caatattaaa tcttccaaac 155460
ctgaacatga gagtaaacaa agaaaggcag gtagacgtaa tgctgcagcc aactagactg 155520
aaataccgta agcaacaaaa gggtcgcaat accggcatcg ctactcgcgg taataaggta 155580
agtttcggtg agttcggctt gaaagccgta ggtcgtggtc gtttgactgc ccgtcaaatc 155640
gaagctgctc gtcgtgcaat gacccgtcat atcaaacgtg gtggtcgtat ttggattcgt 155700
gtattccctg ataaaccgat tactgaaaag cctattcaag ttcgtatggg tggcggtaaa 155760
ggtaacgtgg aatattacat tgccgaaatt aaaccaggta aagtgttgta tgaaatggat 155820
ggcgttccag aggaactggc tcgtgaagca ttcgagttgg ctgctgccaa attgcctatt 155880
cctacaacct ttgtagtaag acaggtgggt caataatgaa agcaaatgaa ttgaaagaca 155940
aatccgttga gcagttgaat gcagatttgt tggacttgtt gaaagctcag tttggcttac 156000
gtatgcaaaa cgctaccggt caattaggca aaccaagtga attgaaacgt gtacgtcgcg 156060
atattgctcg tattaaaacc gttttaactg aaaaaggtgc taagtaatga gcgaaactaa 156120
aaatgttcgt actttgcaag gcaaagtagt aagcgacaaa atggataaaa ccgtaacagt 156180
attggttgag cgtaaagtaa aacatccgct gtatggtaag attattcgat tatctactaa 156240
aatccatgcc catgatgaaa ataatcaata tggaattggt gatgtggttg ttatatcgga 156300
atcccgtcca ttgtcaaaaa ctaaatcttg ggttgtcagt gagctggttg agaaagcacg 156360
ttctatttaa gaattaaagc aacgtgcttg gaatgggaaa cgaagtattg cagcaaattt 156420
aatttgcgtg taaacttcgt ttcctgtctt tcagtttctt ctggaagttt cttccctttc 156480
ggggtccaag actggtttac ttgaaccgca aggtttcatt taataagcag cggctttgct 156540
gtaagttatc tgaaagtggt aaattaagtt ggttaattta aaggtaataa catgattcaa 156600
atgcagacca tcttagatgt ggctgataac tctggtgcgc gtcgcgtaat gtgtatcaag 156660
gtattgggcg gatctaagcg tcgctacgct tctgttggcg atattattaa agtggcagtt 156720
aaagatgcgg ctccgcgtgg ccgtgtcaaa aaaggcgatg tatataatgc ggtagttgtt 156780
cgtactgcta agggtgtacg tcgtcctgat ggtgcgttaa ttaaattcga taacaatgcc 156840
gccgtgttac tgaataataa acttgaacct ttgggtactc gtatctttgg tccggtaacc 156900
cgtgaattgc gtactgagcg atttatgaaa atcgtttcat tggcacctga agtattataa 156960
ggaatggcac gatgaataaa atcattaaag gcgatagggt tgtagtaatt gctggtaagg 157020
ataaaggtaa gcagggtcaa gtagttcgag tgttgggtga taaagttgtt gttgagggcg 157080
ttaatgttgt aaaacgccat caaaaaccta atccaatgcg tggcattgag ggcggtatta 157140
ttactaaaga aatgcctttg gatatttcta atatcgcaat cctgaatccg gaaactaata 157200
aagcggaccg tgttggtatt aagctgattg aaaatgaagg caaagttaaa cgcgttcgtt 157260
tcttcaaatc aaatggctct atcattgggg cataaggaga taacatggct cggttgagag 157320
agttttataa agagacagtt gttcctgaat tggttaaaca atttggttac aaatcagtaa 157380
tggaagtccc gcgtattgaa aaaattacct tgaatatggg tgtgggtgag gctgttgctg 157440
ataaaaaagt tatggaacat gctgtttccg atttagagaa aattgccggt caaaaaccgg 157500
ttgttactgt tgcccgtaaa tctatcgcag gttttaaaat ccgtgataac tatccggttg 157560
gttgcaaagt aacattgcgt cgtgatcaaa tgtttgaatt cttggatcgt ttgattacta 157620
ttgcattacc tcgcgtacgt gacttccgtg gtgtgagcgg taaatcattt gatggccgtg 157680
gcaattacaa tatgggtgtt cgtgagcaaa ttatttttcc ggaaattgaa tacgataaaa 157740
ttgatgcttt gcgtggtttg aatattacta ttactactac agcaaaaacc gatgaggaag 157800
cgaaagcttt attgtcattg tttaaatttc cgttcaaagg ataatcatgg ctaagaaagc 157860
acttattaat cgtgatctga aacgtcaagc tttggctaaa aaatatgcgg ctaaacgcgc 157920
ggcaattaaa gcggtaatca atgattcgaa tgcaactgag gaagagcgtt ttgaggctcg 157980
tttgaggttt caatccattc ctcgtaatgc ggcacctgtg cgtcaacgtc gtcgttgtgc 158040
tttgacaggt cgccctcgtg gtactttccg taaatttggt ttgggtcgta ttaaaatccg 158100
tgaaatcgcc atgcgtggcg aaattccggg tgttgttaaa gccagctggt aataggagta 158160
attaagaatg agtatgcatg atcctatttc cgatatgttg actcgtatcc gcaatgcgca 158220
acgtgctaat aaagcagcgg ttgcaatgcc ttcttcaaaa ttaaagtgtg ctattgcaaa 158280
ggtattgaaa gaagaaggat atattgagga cttcgcagtt tcatctgacg taaagtctat 158340
attggaaatt caattaaaat actatgcagg tcgtcctgta attgaacaaa tcaagcgtgt 158400
atctcgcccc ggtttgcgta tttataaagc gtctagtgag attccaagtg ttatgaatgg 158460
cttgggtatt gctattgtta gtacttctaa aggtgtaatg actgatcgta aagcacgttc 158520
tcaaggtgtt ggtggtgagt tgttatgcat tgtagcctag tggaggaaaa gaaatgtcac 158580
gtgtcgcaaa aaacccagtg actgttcccg ctggtgtaga agtaaaattt ggagcagagg 158640
cattagttat taagggtaag aacggtgaat tgtcttttcc tttgcattct gatgtagcca 158700
ttgaatttaa tgatggcaaa ttgactttg ttgcgaataa cagcagtaaa caagcaaatg 158760
caatgtctgg tactgctcgc gcattagtca gcaatatggt taaaggtgtt tcagaaggtt 158820
ttgagaaaag attgcaattg ataggtgtgg gttatcgtgc tcaagcacaa ggtaaaatct 158880
tgaatctgtc tttgggtttt tctcatccga tcgtatatga aatgcctgaa ggtgtctccg 158940
ttcaaactcc tagccaaaca gagattgttt taaccggctc ggataaacaa gttgttggtc 159000
aagttgctgc tgagattcgt gcgttccgtg ctcctgagcc ttataaaggt aaaggtgttc 159060

gctatgtagg agaagtagtg gtaatgaaag aagccaagaa aaaataattg aggttcacta 159120
atggataaac atacaacccg actccgtcgt gcacgcaaaa cccgtgctcg tattgcggac 159180
ttgaaaatgg taagattatg tgtgttccga agcaataatc atatttatgc tcaagtaatt 159240
agtgctgaag gtgataaagt attggctcaa gcctctacat tggaagctga ggtgcgcggt 159300
agtctgaaat ctggaagcaa tgttgaagca gctgcaatag ttggtaaacg tatcgctgaa 159360
aaagctaaag cagcaggtgt agaaaaggtt gcttttgatc gttcaggttt ccaatatcac 159420
ggtcgtgtga aggctttggc tgaagctgct cgtgaaaatg gtttaagctt ctaaatattt 159480
ggagactttc agatggcaaa acatgaaatt gaagaacgcg gtgacggtct gattgaaaag 159540
atggtcgctg ttaatcgcgt aactaaagta gttaaaggtg gccgtatcat ggctttctca 159600
gcactgactg ttgttggtga tggtgatggt cgcattggta tgggcaaagg taaatcaaaa 159660
gaagtaccag ttgctgttca aaaagcaatg gatcaagctc gacgctctat gattaaagta 159720
cctttgaaaa acggtactat tcatcatgag gttattggcc gtcatggtgc tactaaagta 159780
tttatgcagc ctgctaaaga gggtagtggc gtaaaagccg gtggacctat gcgtttggtt 159840
tttgatgcta tgggcattca taatatctcc gccaaagtgc acggatctac taacccatat 159900
aatatcgtac gtgcaacatt agatggtttg tctaagttgc atactcctgc tgatatcgca 159960
gccaaacgtg gcttgacagt ggaagacatt ttgggagtta accatggctg aacaaaaaaa 160020
gattagggtt acattggtta aaagcctgat tggtacaatt gaatctcatc gtgcatgtgc 160080
acgcggttta ggtttgcgtc gtcgcgagca tacggtagag gttttagata cccctgaaaa 160140
ccgtggtatg attaataaaa tcagctactt gttgaaagtg gagtcttgat atgtttttga 160200
atacaattca acctgctgtt ggtgctacgc atgctggtcg tcgtgttgga cgcggtattg 160260
gtagtggtct tggcaaaacg ggtggtcgtg gtcataaagg tcaaaagagc cggtctggtg 160320
ggtttcataa ggtgggtttc gagggtggtc aaatgccctt gcaacgacgc ctccctaaaa 160380
gaggttttaa atctttaaca gcatcagcta atgcacagct tcgtttaagt gaactggaat 160440
caattgctgt taatgagatt gatattttgg tcttaaagca agcgggtctg attgcatcta 160500
cagtctctaa tgttaaagtt attgcttctg gtgaaatttc taaggcagtt gctttgaagg 160560
gtattaaagt taccaaaggt gcgagagctg ctatcgaggc tgttggtggt aagattgaaa 160620
tgtaaggttt aatattgtgg ctaatcaaca aacgtcatca ggttcatcca aatttggaga 160680
tcttaagaaa cgtcttttgt ttctatttgg agcattgatt gtttttcgaa ttggtgccca 160740
tatacccgta cctggagttg atgctgttgc tttagctaaa ttatacgaaa gcgctggaaa 160800
cggcatcctg ggaatattga atatgttttc cggtgggtcg ttagagcgct ttagtatatt 160860
tgcaatagga attatgccat atatttcagc ttctattatt gtacagctcg cttctgaaat 160920
tttgccatca ttgaaggctt taaaaaaaga aggggaggct ggtagaaagg taattacgaa 160980
atatactagg tatggtactg ttttgttagc aattcttcaa agtctaggtg ttgcatcttt 161040
cgtatttcag caaggaattg ttgtaacaag ttcatttgag tttcatgttt ccacggtagt 161100
ttctttggta acgggaacca tgtttcttat gtggcttggg gagcaaatta ctgaaagggg 161160
tatcgggaac ggtatttctt taatcattac ggcaggtatt gcttcaggta ttccttcggg 161220
tattgcaaag ctggttacac tgacgaacca aggttctatg agcatgctta cggcgttgtt 161280
tattgtattt ggtgccttat tattaattta tttggttgta tactttgaaa gtgcacagcg 161340
gaagattcct attcattatg caaaacgcca gtttaatggt agggcgggta gtcaaaatac 161400
gcatatgcct ttcaagttga atatggctgg tgttattccc ccaatttttg cttccagtat 161460
tattctattt ccatctactc ttttaggttg gtttggttcg gctgatacaa atagtgtttt 161520
gcacaaaata gctggattgt tacaacacgg tcaattgctg tatatggctt tatttgcagc 161580
gacagttatt ttcttttgtt atttttatac ggctttggtt tttagcccta aagaaatggc 161640
agagaattta aaaaagagtg gtgcttttgt tcctgggatt agacctggtg agcagacctc 161700
taggtattta gaaaaagttg tattacgttt gacattgttt ggagctcttt atattacaac 161760
tatttgttta attccagagt tcttaactac ggttttaaat gtaccttttt atttgggtgg 161820
cacgtctttg ttgattctag ttgttgtaac gatggatttt agtacacaaa taaattcgta 161880
taggcttact caacagtatg ataagttaat gactcgttca gaaatgaaat cattttctcg 161940
gaaatagaat tatggcgaaa gaagatacta tccaaatgca aggtgaaatt cttgaaactt 162000
tacctaatgc aacatttaaa gtaaaacttg agaatgacca tattgtattg ggtcatattt 162060
ctgggaagat gcggatgcat tacattcgta tttctccggg agataaggtc acagtagagc 162120
tgacacctta tgatctaact agggctcgaa tcgttttcag agcaagataa accaataaaa 162180
ggaaaataaa atgcgtgtac aaccatctgt taagaaaatt tgccgaaatt gcaagattat 162240
tcgtcgaaat cgtgtagttc gtgtaatttg tactgatctc cgtcacaaac agcgtcaagg 162300
ttaatggaat atttctttta atgtgattct gtgatatagt gacacacttt gccctaaaaa 162360
ggaaaaaata tggctcgtat tgcagggggta aatatcccta ataacgcaca catcgtaatt 162420
ggtcttcagg ctatttacgg tattggtgct actcgtgcta aattgatttg tgaggctgca 162480
aatattgcgc ctgatactaa agcaaaagat ttggacgaga ctcaattaga tgctttgcgt 162540
gaccaagttg ccaagtatga agtagaaggt gatttgcgtc gtgaggtaac tatgagtatc 162600
aagcgattga tggacatggg ctgctatcgt ggcttccgtc atcgtcgcgg cttaccatgc 162660
cgcggtcaac gcactcgtac aaatgcgcgt acccgcaaag gtccgcgtaa agcgattgct 162720

ggtaagaaat aaattttaag gaattttatt aatggctaaa gcaaacacag cttcacgtgt 162780
acgtaaaaaa gtacgtaaaa ccgtgagtga gggtattgtg cacgttcatg catctttcaa 162840
caataccatc attacaatca ctgaccgtca aggcaatgcg ttgtcttggg ctacctctgg 162900
cggcgctggt tttaaaggtt ctcgtaaaag tacaccattt gcagcacaag ttgcagcaga 162960
agcagctggt aaagttgccc aagagtatgg cgttaaaaat ttagaggttc gtattaaagg 163020
tccaggtcca ggtcgtgaat cctctgtacg tgctttgaat gctcttggtt tcaagattac 163080
cagcattact gacgttaccc cgttgcctca taacggttgc cgtccgccta aaaaacgtcg 163140
tatttaatat tggagtgatt tgaaacatgg cacgttatat tggccctaaa tgtaagttgg 163200
cacgtcgcga aggtacggat ttgtttttga agagtgcgcg ccgctctttg gattctaaat 163260
gtaaaattga ttccgctcct ggtcagcatg gtgcaaaaaa accgcgtttg tcagactatg 163320
gtttgcagtt gcgtgaaaaa caaaaaatcc gccgtattta tggcgtatta gaacgtcagt 163380
tccgtcgtta tttcgcagaa gctgatcgtc gtaaaggttc taccggcgag ttgctgttgc 163440
agttgctgga atctcgtttg gataatgtcg tttatcgtat gggtttcggt tctacccgag 163500
ctgaagcaag acagcttgtt tctcataagg cgatagttgt gaatggacaa gttgtcaata 163560
ttccttcttt ccaagtgaaa gctggtgatg ttgtctcagt tcgtgaaaaa gccaaaaaac 163620
aggtacgtat tcaagaagca ttgggtttgg caactcaaat cggcttgccg ggttgggttt 163680
ctgtagatgc ggataaactt gagggtgtgt tcaaaaacat gccggatcgc tcggaattga 163740
ccggtgatat taatgaacag ctggtggtag agttctactc taaataatgc tagctcagtg 163800
agggacagtt aaatgcagaa tagcacaacc gaatttttga aacctcgtca aattgatgta 163860
aatacttttt ctgcaactcg tgcaaaagta tctatgcagc catttgaacg tggtttcggt 163920
cataccttag gtaatgcttt gcgccgtatc ttactgtcat ccatgaatgg ttttgctcct 163980
actgaagtag ctattgccgg tgtattacac gaatattcta ctgttgatgg tattcaggaa 164040
gatgttgttg acattttgct gaatattaaa ggtattgtgt ttaaactcca tggtcgtagc 164100
caagttcaac ttgtgttgaa gaaatcaggt tcaggtgtcg tatctgccgg tgatattgag 164160
ttgccgcatg atgtagaaat tctgaatcct ggtcatgtca tttgtcattt ggctgataac 164220
ggtcaaattg agatggaaat taaagtagag caaggtcgtg gttatcaatc tgtttcaggt 164280
cgtcaggtag ttcgtgatga gaaccgtcag attggtgcaa tccagttgga tgcgagcttt 164340
tcgcccatca gccgtgttag ctttgaggtt gaacctgcac gtgtagagca gcggacggat 164400
cttgataagt tggttttgga tatcgaaacc gacggttcta ttgatcctga ggaagctgta 164460
cgcagtgcgg cacgtatttt gattgatcag atgtctattt ttgctgattt gcagggtacg 164520
cctgtggagg aggttgaaga aaaagcacct cctatcgacc ctgttctttt gcgtccggtg 164580
gatgatctgg aattgacagt acgttcagct aattgtttga aagctgagga tatttattat 164640
attggcgatt tgattcaacg cactgaaacc gagcttctta aaacgccgaa tttgggacgt 164700
aaatctttga atgagattaa ggaagtattg gcatctaaag gtttgacact gggttctaag 164760
ttggaagcat ggccacctgt aggcttggaa aagccttaat gaagaattaa aggataattg 164820
atatgcgtca tcgtaatggc aatcgcaaat taaaccgtac cagcagtcat cgtgctgcaa 164880
tgctgcgtaa tatggcgaat tcattattga ctcacgaagc tattgtaaca actctgccta 164940
aggccaagga attgcgccgt gtagtagagc cgttgattac attgggtaaa aagccgtcat 165000
tggcaaaccg ccgtttggca tttgaccgta ctcgcgaccg tgatgttgta gtaaaactgt 165060
ttggcgattt gggtcctcgt tttactgctc gtaacggtgg ttatgttcgg gtgttgaaat 165120
acggattccg taaaggtgat aatgcacctc tggcactggt tgaattggtt gacaaaccgg 165180
ctgctgagta attttagtca tataacgcca tctgccgaaa agcaggtggc gttatttttg 165240
caatatctga taggtaatag ggtattggct atcatgttta aaatattaat tgaatagcta 165300
aggtttgcgc ggtaaactta catcattaaa aaattctatg atggtttata taatgaatgc 165360
tttcgatata aagtcgacaa agatggacgt attgtctata tctttgcata cgtcagactt 165420
gtttgatttg gaagatgtgc tggtcaaatt gggcaagaag tttcaagagt ctggtgttgt 165480
tccatttgtg ctggatgttc aagagtttga ttatcccgag tctttggatc ttgctgcatt 165540
ggtttcgttg ttttcaaggc atggtatgca aattttgggt ctgaagcatt ctaatgaacg 165600
ttgggctgct gcggctatga agtatcattt gctgttttgt ctgtctcatt cggaaaatgt 165660
taaagaactg ggtcaggttg aggtgcagaa aacggaggat ggtcagaaag caaggaaaac 165720
agtattgatt acatcccctg tccgtaccgg tcagcaggtt tatgccgaag atggcgattt 165780
gattgttacg gggcggtca gccaggggc ggaattgatt gcggatggca atatacatat 165840
ttatgcgccg atgaggggc gtgctttggc cggtgccaag ggtgatactt ctgcccgcat 165900
atttatccac tccatgcagg cagaactggt ttctgtggcg ggtatttacc gtaattttga 165960
acaggatttg ccgaaccatc tgcacaagca gccggtacag atattgttgc aggataaccg 166020
attggttatc agtgcaattg gctcagagta attgtttgat atttaaaaag gaaatattgt 166080
ggcaaaaatt attgtagtaa cttcaggtaa gggcggtgtc ggtaaaacga ctaccagtgc 166140
cagtattgcg acaggtttgg cattacgcgg atataaaact gcggtaattg attttgatgt 166200
gggtttgcgt aacctcgacc tcattatggg ttgcgagcgt cgtgtcgttt atgacctgat 166260
caatgtcatt caggggggagg cgacgctcaa ccaagctttg attaaagata aaaattgtga 166320
aaacctgttt attttgccgg cttcccagac tcgggataaa gacgctttga cacgcgaggg 166380

cgtagaaaaa gtgatgcagg agctgtccgg caagaaaatg ggctttgagt atattatttg 166440
cgactctcct gccggtattg agcagggtgc attgatggcg ttgtattttg ctgatgaagc 166500
cattgtaacg accaatcctg aggtttccag tgtgcgtgac tccgacagga ttttgggaat 166560
tttgcaaagc aaatcccata aggcagagca aggcggttcg gttaaagaac atctgttgat 166620
tacgcgttat tctcccgaac gtgtggcaaa aggcgaaatg ctgtctgtac aggatatttg 166680
cgatattctg catattcctt tgctgggtgt gattcctgaa tcccaaaacg tcttgcaggc 166740
atccaattcc ggagaaccgg tcatccatca ggacagcgtg gcggcttccg aggcatataa 166800
ggacgttatt gcccgtcttt tgggcgagaa ccgtgaaatg cgtttcttgg aagctgagaa 166860
aaaaagcttc ttcaaacgtc tgtttggagg ataaggtatg tcattaatcg aatttttatt 166920
cggcagaaag cagaaaacgg caaccgttgc ccgcgaccgc cttcaaatca tcattgccca 166980
agagcgcgcc caagaaggtc aggctccgga ttacctgccg actttacgta aagagttgat 167040
ggaagtcctg tccaaatatg tgaatgtttc attagacaat atccgtattt cccaagaaaa 167100
gcaggatggt atggatgtgc ttgagttgaa cattactttg ccggaacaga aaaaggtata 167160
ggacatgacc ttaaccgaat tgcggtacat cgtcgcagtc gcccaagaac gtcatttcgg 167220
cagggcggcg cggcgttgtt ttgtcagcca gcccactttg tctattgcca ttaagaaatt 167280
ggaagaagag cttgccgtct ctttgtttga ccggagcagt aacgatatta ttacgaccga 167340
ggcgggggaa cgtatcgttg cacaggcgcg taaggtattg gaagaggcgg agcttatcag 167400
gcatttggca aatgaagaac aaaacgagct ggagggtgcg ttcaaactcg ggctgatttt 167460
tacggttgcg ccgtacctgc tgccgaaact gattgtttcg ttgcgccgta ctgcaccgaa 167520
aatgcctttg atgttggaag agaattacac gcatactttg accgagtcgc tcaaacgcgg 167580
ggacgttgat gcgattatcg ttgccgaacc gtttcaagag ccgggcattg ttaccgaacc 167640
cttgtatgac gaaccgtttt tcgtgattgt cccgaaaggg cattcatttg aggaactgga 167700
tgccgtttcg ccccggatgc tgggtgagga gcaggttttg ctgctgacgg aaggcaactg 167760
tatgcgggat caggtactct caagctgttc cgaattggcg gcgaaacaac gtatacaggg 167820
gttgaccaat acattgcagg gcagctcgat taatacaatc cgccatatgg ttgccagcgg 167880
tttggcaatc agcgtgttgc cggcaaccgc actgaccgaa aacgatcata tgctgttcag 167940
cattattccg tttgagggta cgccgccaag ccggcgggtc gtattggcgt accgccgcaa 168000
ttttgtccgt ccgaaggcgt tgtcggcgat gaaggcggcg attatgcagt cgcagcttca 168060
cggggtaagt tttatctgcg actaggcgca ggcattgttt tcaaaacgcc atttccctga 168120
gccgacaaca cggtatgcca agatattgcc gtcatcatcg attttgagta tagcatcgcc 168180
acggaaactg ccgtcctgaa gatattcgac ttttgcatca ctgtgaatgt tttcatcagt 168240
gccgatgcaa tgccatgtat agtggattaa caaaaaccag tacggcgttg cctcgccttg 168300
ccgtactatt tgtactgtct gcggcttcgt cgccttgtcc tgatttttgt taatccacta 168360
taaaagaggc cgtctgaaaa acatttttca gacggccttg tttattcaat caaatcagtc 168420
tttcaacttc gccaactgat tttgaacttt tgccattttg tcttccaatt ccgccaaatc 168480
ggctttgtct ttttccacca gatgcgcagg ggctttttcg gtgtagccgg gtttggagag 168540
tttggcgttg agtttgtcca aggctttttg cagcttctcg gcttctttgc tcaaacgggc 168600
ggtttcggcg gctttgtcga tttcgacttt caacatcagg cgcgcgccgt tgcagacggc 168660
gacgggcgcg tcttcgcttt cggtagggc ggcgacttgc tgtgcttcgg tcaggcgggt 168720
catcatcggc aggtatttga ggtagtccgc caagtcgtcc gtgctttcga caaacagcgg 168780
ggcttttacg ttgggctgga tgcccatttc gccgcgcagg ttgcggactg cgccaatcaa 168840
atcctgcaac acggtcattt gctcgaatgc cgtctgaaca atctcgccgc tgtcggcttc 168900
ggggaagcgg gcgagcatga tgctgtcggc ggttttcgcg tcgcacatag gagcgacggt 168960
ttgccacagt tcttcggtga tgaacgggat aatcgggtgc agcaggcgca gggcggcttc 169020
gagtacgcgc aataaggtat ggcgtgtggc gcgttggcgg ctggcgcagc cggtttgaag 169080
ctgcactttg gcgagttcca aataccagtc gcaatagtcg ttccatacga agctgtacag 169140
ggtttccgcc gccaaatcaa agcggtaggt ttcgtaggct tgcgtaacct gttcgatggt 169200
ctgattcaga cggcctacaa tccacatatc ggggaaggag tagccgcgcg gttcggcagc 169260
ggttgcgccg taaccgcagt cttggttttc ggtgttcatc aagacgaagt tggtggcgtt 169320
ccagattttg ttgcagaagt tgcggtagcc ttcggcgcgt ttgaagtcga agttgaccga 169380
acgccccaag ctggcgtagc tcgccatagt gaagcgcaaa gcgtccgcgc ccatactcgg 169440
aatgccttcg gggaagagtt ttttcgtggc ttcttccact ttcggcgcgg tttcgggttt 169500
gcgcaggccg gtggtgcgtt ttaccagcag tttttccaag ccgatgccgt cgatcaaatc 169560
cacagggtca atgacgttgc cttcggattt ggacattttt ttgccttcgt ggtcgcgcac 169620
gatgccgtgg atgtacacgt ctttaaacgg tactttgccg gtgaagtggg tggtcatcat 169680
aatcatacgc gccacccaga agaagatgat ttcgtagccg gttactaaga cattggacgg 169740
caggaaggct ttgagttcgt cggtttcaga cggccagccg agtgtggaga acggcacaag 169800
cgcggaggag aaccatgtat ccaatacgtc ttcttcgcga gtcaagcctg ttttgccggc 169860
ttgtttttcg gcttcttcct gattgcgggc aacatacaca ttgccttcgt tgtcgtacca 169920
tgcagggatt tgatggcccc accacagttg gcgtgagata caccagtctt ggatgttgtt 169980
catccattgg ttgtaagtgt tgacccagtt ttcagggata aagcgtaccg cgccgctatc 170040

aacggctttt ttggctttat cggcgaggct caagcctttg aactcgctgt ccggctcgcc 170100
gccgtttggg gtggcggaca tggcgacaaa ccattggctg gtcagcatag gttcaatcac 170160
cgaacctgta cggtcgcctt tcggcgtcat cagcgtgtgt ggtttgattt cgaccaagaa 170220
accttgttcc tgcaaatcgg caaccatttg tttgcgcgcg gcaaagcggt ctaagcctgc 170280
gtatttttca ggcagggcaa agcctagttg cgcttcgcct ttgaagttga acacttcggc 170340
gtttgccagc actttggctt ccaagttgaa cacattaatc aggcgcgtgt cgtggcgttt 170400
gccgacttcg tagtcgttga agtcgtgtgc aggcgtgatt ttcacgcagc ctgtgccgaa 170460
gtctttttca acgtattcgt cggcaatcac ggggatagta cggccggtca gcggcaggat 170520
taattccttg ccgattaagt gggtataacg ttcgtcttca ggattgacgg caacggcaac 170580
gtcgcccagc agcgtttcag gacgggtggt cgccacgata acggcttcgg cgggattgtc 170640
cgccagcgga tagcggatgt gccacataga gccttgttct tccacgcttt ccacttccaa 170700
atccgatacc gccgtgccaa gcacgggatc ccagttcacc aagcgtttgc cgcggtaaat 170760
caagccttgc tcatacaggc gcacgaacac ttcggttacg gtttcggcgc gcacgtcgtc 170820
catcgtgaaa tactcgcgcg tccagtcggc agagcagccc acgcggcgca tttgttgggt 170880
aatcgtgccg ccggaaactt ctttccattc ccacactttc tccaaaaatt tttcgcgacc 170940
caagtcatgg cgggacacgt tttgcgcagc aagctgacgc tcaaccacaa tctgcgtggc 171000
gatgcccgcg tggtctgtgc cgggaatcca ggcggtgttg cagcctttca tgcggtagta 171060
gcgggtcaga ccgtccataa tggtttggtt gaaggcatga cccatgtgca gcgtgccggt 171120
tacgttgggc ggcggcagtt ggatggagaa agacggtttc gtcaaatcca tatcaggttg 171180
gaaatagccc tgctcttccc agttttgata atgtttggat tcgatttcgg ctggattgta 171240
tttgtctaac atgatggaac tttgtgaaat taaggttatt tttgatgtgc ggattataac 171300
gcaaaaaggc cgtctgaatc atttcagacg gcctttggca tacaggtttt aaaaatggaa 171360
caataccagg ctgacggcaa tcaccgccat acccgttgtc aggccgtaaa cggtttcatg 171420
gccgtctgaa tagcgtttgg cagccggcag cagctcgtcc aacgccaaaa acaccatcac 171480
accggctatc acgccgaata ccgaaccaaa cacggcaggc gacaaaaacg gctgcaaaac 171540
caaatagccc aaagccgccc ccaacggctc ggccaagccg gatagcagac acgcccacac 171600
cgttttctta cggctgcggg tggcaaaata aaccggcgcg gcgatggaaa tgccctccgg 171660
aatattatgg atggcaatcg ccaaggccaa aggcatcccg actgctggat tttccaatgt 171720
ggcaaaaaac gtcgccaagc cttcggggaa attgtgcgca gtaatcgcaa acgccgccat 171780
catgccgact cgcgcgatat ggcggcgttt gctttcttga aacgacgggt cttgcgcgtc 171840
taaagtttca tgcgggttcg gcaccagacg gtcaatcagc gcaatgccgc ccatcccggc 171900
caaaaatgcc atggtcgccg ccgcaaacgc gtggtcttta tcataaattt cagcgaacgc 171960
ctcgctggac ttactgaaaa tctccgtcag ggaaacatat accatcgcac cgccggcaaa 172020
cgccaaacca aacgacaaca cacgcggatt gggcgttttg gaaaacatca ccaagccact 172080
gcctaatacg gtaaacaaac cggcagccaa tgtgatggaa aaggcaacgg ccaaattgga 172140
catcgaaaaa tcgggcatga gaaaacctgc gctaaaagct gggacaggtt cagactaaca 172200
ctttttaatg tatatgataa tagttattat ttattttatt gattggatac acggattttg 172260
aaacaaaagg ccgtctgaaa aatgattttc agacggcctt taaatttgaa atgccgctaa 172320
accttagtgc tttccagctt aagcctgata acgcgacagg ctcaaatcgt cgctgcggat 172380
ttcggtgtct ttgccgctca cgatatcggc ggttaatttt gccgaaccca gcgacatggt 172440
ccagcctaaa gtaccgtggc cggtattcag aaacaggttg tcaaagcggg tgcgaccgat 172500
taacggcgtg ctgtcgggcg tcatcggtct gaggccgctc cagaacgatg cttggctcaa 172560
atcgccgcct tccgggaaca agtcgttgac gaccaaagcc aaggtttcgc ggcgtttttc 172620
gggcagtttg atttcgtagc ccgacaattc cgccataccg ccgacgcgga ttctgttgtc 172680
aaagcgcgtg atggcgactt tgtagctttc atctaaaacg gtggacaccg gtgcgccgtc 172740
tgaattggtg accggcaggg tcaaggaata gcctttgacg ggataaatgg gcagattgag 172800
atccaactgc gccaaaaccg tcctgctgaa gcaaccgagc gcgcagacaa cggcatctgc 172860
ttcaaaccgc cctgtttcgg tttcaacggt tttgatgcgc agcccgttgt ggtcgatgcg 172920
gctgatgttt tggttgaaat gaaaccgtac gccctttttcc tgacacaatt tgtataggtt 172980
ttcagtgaag aggcggcagt cgccggtcgc atctgcaggc aggtgcaggc cgccggcaat 173040
tttggcggta acgcgtgcca gcgcaggctc aaattctgca cattcttcgg gtttcagacg 173100
gcggtacggc acgccgtagc gttccaaaac ggcaatgtct tgttttgccg cttcgacttc 173160
tttggtttgg cggaaaatct gcaacgtccc ttttttgcgt ccctcaaaat tcatgccggt 173220
ttgcgcttca aaacggcgga acatttcacg gctgtattcg gaaatcctga ccatgcgctc 173280
tttattggtt tgatagtgcg ctgccgtgca gttttgcagc atttgccaca gccattcgat 173340
ttgatacagg ctgccgtcgg ggcgaaacag caaaggcgga tggcttttaa acagccattt 173400
cagcgctttg gtcgggatac cgggtgcagc ccaaggcgtg gtatagccgt aagaaagctg 173460
gcctgcgttg gcaaaactgg tttccatcgc cacaccctcg gcgcggtcga tgaccgttac 173520
ttcatgtccg gcctctgcca gataccacgc ggaagacacg ccggcaacac ccgcacctaa 173580
aacaagcact ttcatgtttc tccctccggc tttttcaaaa cagacttaat atgccgtgcc 173640
gtctgaatat tcggattcag acggcctcgg atattaatgc ggcaattcgc cgtttgtgat 173700

tttttgtttg aagtcgcgcg tttcattgac gatgactttc gccatcaata aaagtgcaat 173760
caggttgggc aatgccatca agccgttgaa tgtgtccgaa gccagccaca ccaaatcaag 173820
gctcaacacg gtacccagca taacggaaga aacataaccc acgcggtaca aaccggcaaa 173880
tttctcgccg aaaacataca ccgcgcattt ttcgccgtaa tagcaccagc ccaaaatggt 173940
tgagtaggca aagaaaatca ggccgatggt aacaatccag ccgccgatgc cgggcagcat 174000
tttttggaat gtgacggttg tcagtgccgc gccgctcact tcaggtttga caaactcgcc 174060
gcccgcgccg agcagtccca ttaccaacac gatgccggta atcgagcaaa cgacgatggt 174120
atccaaaaac gtaccggtca tagaaaccaa ggcctgacgg acgggatggt cggttttcgc 174180
ggctgcggcg gcaataggcg cagaacccat acccgcctca ttggagaaca cgccgcgcgc 174240
cacgccgtag cggatgaccg taccgatagc accgcccgcc actgcctgcg cgctgaacgc 174300
atcggagaaa atcagcttga cggcaggcat cagtgcatcg gaattaatcg cgataatgga 174360
aagaccgccc aacacataaa acaccgccat agcaggcacg atgaaagaag cggctttggc 174420
gatgccttta ataccaccta aaacgacaac ggcagtcaga acggtcaacg taatgccggt 174480
ataggcaggt tcgataccga agctggtttg caccgcctgt gcaaccgagt tggactgcac 174540
cgagctgccg ataccgaagg aagcgaatgt gccgaacagc gcaaacgcga cggccatcca 174600
tttccagttt ttgcccaagc ctttttcgat gtaatacatc gggccgccgg acatttcgcc 174660
tttggaattg ttgacgcggt atttcaccgc caacacgcct tcgccgtatt tggtggccat 174720
gccgaaaatg gcggtcatcc acatccaaaa taccgcgccc gggccgccgg ttaccaccgc 174780
agtcgccacg ccggcgatgt tacccgtgcc gatggtggcg gacagcgcgg tcatcaacgc 174840
cgcaaaatgg gaaatatcgc cttcgtggcc ttcgccgctt ttatgcttct ttggcggcat 174900
aaacgcctgt ttcagcgcat aacccaacat cgtgaactgc aaacctttta ataaaacagt 174960
cagcaaaata cccgtgccga ccagcagcat cagcatcaaa ggtccccaaa cccagccgct 175020
gacggtttca aaaaaggctt tgggattgtc taaaaacact tgcatggctt tctcctttgt 175080
ctgttttatt tttaaaacac cacttttgta gtgtccagta atttcagcac agaatatcca 175140
ataagacaat atgttctttt gaaaaatact tttggttttt tcgccgaaaa caggacggtt 175200
caagttgcgg aaattgtttg caattcttta aaagcagcgg cggaggtcac aatgaaatgt 175260
ccgaatgggg atgtggcggg cggcagaaat catcaatgct gccgactgcc atacttctga 175320
aatctacaaa atgatgcatc gatcaaacaa tataccgctt taaaaaaacc gatgccgtct 175380
gaaacgcttt cggggtttca gacggcatca aaagggtacg gtcagcggat gatgccgcgc 175440
gccgattgtg cgaaaaagtc tcggaatacg gcaagctcgg cttgggtttc ggcgcggcgg 175500
agaatgtctg ccttggcttc ttcaaacgga atgccgcgat ggtagagggt tttgtacacg 175560
tctttgacgg cggaaatctg ctctgcggta aaaccgttgc ggcgcatgcc ttcgctgttg 175620
agccccgccg gttcggcgcg gtagcccgat gccataaagt agggcggcac gtctttgtgt 175680
acgcctgcgg caaacgcggt catggcgtag tcgccgatgc ggcagaattg gaaaaccagc 175740
gtgtagccgc ccaaaacgac gtagtcgccg atggtaacgt gtccggcaag cgaggcgttg 175800
ttggcgaaaa tggtgtggtt gccgatgacg cagtcgtgcg cgaggtggca gtacgccata 175860
atccagttgt cgtcgccgat acgggtttcg ccgatgccgg ttaccgtacc taaattaaag 175920
gtggtgaatt cgcggatggt gttgccgttg ccgataatca gcttggtcgg ctcgtcgcgg 175980
tatttttgt cctgcgggat ttcgccgagg ctggcaaatt ggaaaatgcg gttgttttcg 176040
ccgatgctgg tgtggccgtt gatgacggcg tgcggaccga tttcggtatt cgcgccgatt 176100
tggacgttgg ggccgataac ggtgtacgcg ccgactttga cgccggagtc gagttcggct 176160
ttggggtcga tgacggcggt cgggtggatg agggtcatgt ttttcctttc ctgtcgtgtt 176220
gccgcgaaga tgcgcgacgg caacaggttg tctgaaaact ttcagacgac ctttttctga 176280
acactcaaac cacgcgtttg gcacacatga tgatggcttc gacggcaact tgcccgtcca 176340
ctttggcaac ggcgttgaat ttgccgatgc cgcgccggct ggtcagcagc tcgacttcaa 176400
agacgagttg gtcgccgggg atgacttggc gtttgaaacg ggcttcgtct atgccggcga 176460
agaagaagaa ttcgttttct ttgcgcccgc cttcgctcaa aatcgccaac gtgccgcacg 176520
cctgcgccat cgcttcgatg atgagtacgc cgggcatcac gggcaggtcg gggaaatggc 176580
cttggaactg gggttcgttt atggtgacgt ttttaatcgc ggtcagggtt ttcatcggct 176640
cgaaggcggt gatgcggtcg agctggagaa acggatagcg gtgggggatg agtttttgga 176700
tgtctttggc ttcgatgggg agttgtacgt ccatgtctgt cgtattcctt gaataaagtc 176760
ggtttggtta tttgctgtct tgaccggcat ctgaaagctg ctgctccagt gttttgagcc 176820
gtttgttcat ttcgcttaag cggtggatgt aaacagcgtt gcgcgcccat tctttatggg 176880
tggacatcgg gaagatgccg gcgaggtgtt tgccgctttc ggtaatgctg tgggtgacgg 176940
acgtgccgcc gccgatggtg gttttgtcgg cgatttcgat gtgtccgacc gtaccgacgc 177000
cgccgccgat gatgcagtag ctgcctatgg ttacgctacc tgagatgccg gttttggcgg 177060
cgatgacggt gtgcgaaccg attttgcagt tgtgtccgat ttggacttgg ttgtcgattt 177120
tggtgccgtt gccgacggtg gtgtcgctca tcgcgccgcg gtcgatgttg gtgttcgagc 177180
cgatttctac gtcgtcgccc agcgttaccg cgccggtttg cgggattttg aaccacgaat 177240
cgtcggcgaa ggcgagtccg aaaccgtccg cgccgatgac cgcgccgctg tggatttcga 177300
cgcgtctgcc cagtgtgcag ccgtaataaa cgacggcgtt gggatgcagg acgacttcgt 177360

cgcccagttt gcaatcgtgt tggacgacgg cgtttgccaa gatgcggcag ccttcgccga 177420
gcacggtgtt tgcgccgatg tagacgttcg cgccgatttc gcagctggtg ggaacggtcg 177480
cgcccggttc gacgacggcg gtcggatgga tgccgccgcg cgctttgacg acgggtgaaa 177540
acaggcgggc gactttggcg aaatagagat aggggtcgtc ggcgacaatc aggttgcgcc 177600
cttcaaatcc gtctgccgct ttggcggaaa cgatgaccgc gcccgcgctg ctgtcgtgga 177660
cttcggcttt gtatttcgga ttggcaagga agctgatgtg ttccgcctgc gcgtctgcga 177720
gcgggcgcac ggcggtaacg gaaatgtcct cgccgcgcca ttcgccgccg agccgcgcgg 177780
tgatttggga cagggtgtag gtggccggaa tcatggtttt cctgttcggt atgccgtctg 177840
aaagggtcag cgggcgttca tttctttaat gacgctgtcg gtaacgtcgt attgggtgtt 177900

gacgtaaatc acgttctgca aaatgacatc gtaaccttcc tgtttggcga ttttgacgat 177960
gacgcggttg gcgttttgct ggagggaggc aaactcttcg ttgcggcgga ggttgtagtc 178020
ttcttcaaac tgcgcctgtt ttttgcggaa cgctgcgacc agcccgcgcc atttttcttc 178080
ggcttgcgcc ttttttgcgt ttctgagttt gccttcggca agctgccttt ccaaatccag 178140
accttcgcgt tgcagttttt gcaattcgtc ctgacgagcg gaaaattcgc tgtccagcgt 178200
tttttgaatc ttgcgcgcct gcttggattc gaggtagatg cgctcggtgt tgataaagcc 178260
gattttttgg aaggtgtcgg cgtgcgcgcc tgcggtgcag cacaaaccga tcagagccgc 178320
ggcaaacgcg cgggtcaaac gggtcatggt aaaactcctt cgaatgttgc cgcgaaatgc 178380
cgtctgaagg gcttcagacg gcatttgcgg gattagaacg tcgtgccgag ttggaattgg 178440
aagcgttgga tttcgtcttc cggttttttc ttcagcgggt aggcgtagct gaatttcatc 178500
gggcctaaag gcgagagcca ggtaaccgcg ccgccggcgg aatagcgcaa ttcgttggta 178560
aaggtggatt tatgggtatt gccggcgccg taaatgtttt gaaccctgcc gccggtcgcg 178620
gaactgctgt tgtcgtcgta ggttttgccg tcccacacgc tgcctgcgtc ggcaaacagg 178680
ctcaggcgga cggtgcgcgc gtctttcgcg ccgggcatcg ggaagagcag ctcggcggag 178740
acgttggctt ttttgttgcc gccgtagctg attttttcgc cgtattcgtc atagactttc 178800
ggaccgagcg tgccgctttc gtatccgcgc accgaaccca ggccgccgcc gtagaagttt 178860
tcaaagaagg ggatttcttt ggttctgccg tagccgcccg caatgccgac ttcgccgccg 178920
agcatcagcg tgaaggtttt gctcaggggg aagaaccagg tttggttgtg ggtggcggag 178980
tagtattgca gtttgctgcc aggcagggcg atttcggcgt tcacgcccgt caggtagccg 179040
cgcgtcggcc ataacgcgct gtcggttttg ttgcgccccc agccgacggt acctttgtac 179100
agccagcctt tgaagctgcc gtctgtgccg tcggttttgc cgtatttctt gataaagtcg 179160
gcatagtgtt tgggcgcttt gttgtaggtg ttgacggtca ggtgttctgc caccaaaccg 179220
aaattcacgc ggtcgtattc ggtaacaggc acgctcatgc ggatgcctgc gcctgccgtg 179280
gtggttttat attgtttgat gctggtcgat gctttgcgcg ggtcgaaggc ttttccgtaa 179340
acatcgtagc ccaggctgac cccgtctgcc gtgaagtacg ggtcagtaaa cgacagcgag 179400
ccgttaagcg tggttttgct cctggaggcg cgcagtgcgg ccgacttgcc cgtaccgaac 179460
aggttgtctt gggaaacgcc tgcggacatg accaacccgg tatcttgaac ccaacccgcg 179520
ctcaaatcca gggaaccggt ggaacgttcg gtcagactca tgttcaaatc gactttgtcg 179580
ggcgtgccgg caagcgggac agcatcaaac tggacattgt cgaagtagcc caaaagctcg 179640
acgcgctctt tggaacgttg cagcttggag gtgtcgtaag gtgcggattc catttggcgt 179700
aattcacggc ggacgacttc gtcgcgggtt ttgttgttgc cggtgatgtg tatttcgttg 179760
acgtagattt tccggcccgg ttcgatgtgc aggacgaaat cgacggtttt ggtttcagcg 179820
ttcggcagcg gctgtacgct gatttcgctg tatgcgtagc ctgccgagcc catgcggttc 179880
tgaatctcac ccaaaacggc ggtcatctgc tggcgttcgt accatttgcc gggcttcatg 179940
gtcagcagtt tttccagttc ggctttgggg acttcgttgg tgtcgccttc gatggagact 180000
ttgccccaac ggaaacgtcc gccttcgtgg acggtgattt tgatggtctg cttggttttg 180060
tcttcgttgg tttggatgtc ggtatcgagg atacggaaat cgaagtagcc gttattttgg 180120
tagaagtcgg ttactttttc catatcttgg gcaaatttct gctcgttgaa ttggttgctt 180180
cgtgtcagcc atgtccaaat gccgccttcg gtcagggaca tttgccgcat cagtttgcgg 180240
tcggaataga cttggttgcc ttcaaattcg atgtcggtga ttttggcgga tttgccctcg 180300
tcaatcgtga tgtcgatgtc gacgcggttg cgggcgagtt tggttacttt gggcgtgatt 180360
tggatattga gtttgccgcg cccgaggtat tcttctttca ggccggcgac tgcctgattg 180420
agtgtcgcct gattaaagta ttgcgactgc gccagcccga acgattcgag gtttttctta 180480
atggcgtcgt tttgcagcat ttttgcgccg gtgatgttga gcgagccgat ggtggggcgt 180540
tcgataacgg tcagcaggag ctgcccgtcc gcagtttcga cgcgtacgtc gtcaaagaaa 180600
ccggtggcgt acaggctttt gatgatggca ctgccgtgtg tgtcgttgta ggtgtcgccg 180660
actttgacgg gcaggtagtt gaatacggta ctcggctcgg tacgctgcaa gccttcgacg 180720
cggatgtctt ggatggtgaa gtcggcaagt gccaaaggcg atatgcccaa catcatcagt 180780
gcggaagcaa tctgtttcag tttcattgtc agttccttgt ggtgcggaat gcggtttcag 180840
acggcattcc gaaacgtaaa atctaaccga gcagccgggt aacgtcgttg aagaaggcga 180900
ccgccatcat cagcatcatg agggcgagcc cgaagcgcaa accgatgttt tggacgcgtt 180960

cgcccaaagg tttgccgcgt atccattcgg cagtataaaa cacgaggtgc ccgccgtcca 181020
aaacagggac gggcagtagg ttcagcacgc cgaggctgat gctgaccagt gctaaaaatt 181080
ccaaataact ttgcaagccg agttcggcgg actgtccggc aatgtcggca atggtcagcg 181140
gcccggaaat atggctgacg gaggcgttgc cgctgattag tttgccgaaa aatttgaggg 181200
ttgtccacga gtgggaaacg gttttttccc agcccatgcc gaatgcgcgg acaacagacg 181260
gacggtagct gcggcggatt tgcgcgtccc acgccctgtc cggctgcgga cggaggccga 181320
cgcgcccgat cagggtgtgg tcggactgtt cgacagtatc ggggcggatg tcggcggtat 181380
gggtttgtcc ggcgcgttcg tagttcaggg tgattttttt gccggggctt tggcgggtca 181440
ggtttgccca ttcttgccat gaggcgatgg gtttgccgtc ggcggcagtc agcctgtcgc 181500
ccggtttcag gcctgctttt tcggcggggc tgccttttc cacgccgccg gcaacggttg 181560
tgattttaaa gggcatcagt ccgatgtagc cttggttttt tgcgatttta ccggcttccg 181620
gcgtgcctgc ggcatcgatg gtgcggacgg tttgcgcgcc cgatgccgtc tgaacgccga 181680
cggcgacttt gccggcttcg aggttgagga cgatttcggt ttgcgcgctg ccccaatctg 181740
caacgggtgt gccgttgacg gattgtattt tgtcgccgct ttggaagccg gcgcgggcgg 181800
caatggtgtc gggttcgact gtgccgacgt aggggcgcag ttcggttacg ccgaaggaaa 181860
agctcagtcc gtacagcaaa accgccagtg cgaggttggt cagtgggccg gcggcgacga 181920
tggcgatgcg cttggcgggg tgttgtttgt caaaagcgta gggtaaatcg gcttctgata 181980
cttcgccttc gcgcgtatcg accattttga cgtaaccgcc caacggaatc ggggcgaggc 182040
accattcggt gtcgccgcgc tttcgggtga aaaacggttt gccgaagccg acggaaaagc 182100
gtacgacttt gacgccgcac aatctggcaa cgatgtagtg tccgaactcg tgcaggctga 182160
ccaaaatcag gatggcgaag ataaaagcta gaagggtgtg caaatggttt tcctttgata 182220
acggtgttca gatggcatca gcgcagtgtg ccgataaatg ctcgcgcttg tgcgcgtgtc 182280
cgggcatctt gcgccaagag ccccccctata tcgcctatgc cgtctgaaaa gtcttgtgca 182340
agacagtggg cgacggtttt ggcaatgtcg gtaaacttaa tctgtccgtc caaaaaggcg 182400
gcgacggcgg cttcgttggc ggcgttcaat acgcagggcg cggctccgcc tgcgttcatg 182460
gcttcatagg cgagcctcag gcaggggaag cggtcaaagt cgggcttttg gaaggtcagc 182520
gcggacaatg cgtcgaaatc caggtcgccg acacccgaat cgatgcgctc gggcaaaccc 182580
aaacaataag cgatgggcgt tcgcatatcg ggattgccca gttgcgccag cacggagccg 182640
tcgcggtagc gcaccatgct gtgtatcacg gattgcggat ggatgacgac ttcgagtttg 182700
tcgggcggac agttgaacag ccaatgcgct tcaatcagct ccaaaccttt gttcatcatg 182760
gtggcggaat cgacggagat tttgcgtccc atacgccaat tggggtgttt gaccgcttgg 182820
gcgggcgtaa tgcggtcgaa cgtgtttaaa tcggcggtca gaaacgggcc gccggaagcg 182880
gtcaggataa tcgaagcgat gccgtgttcg ttcagacggc cggcgtaatc gcgcggcaaa 182940
acttggaaaa cggcgttgtg ttcgctgtcg acgggcagca ctgccgcgcc gtttgcacgg 183000
gcggtttcca taaacaacgc gccggaaacc accagcgttt ctttgtttgc cagataaatg 183060
gttttgcctt tttgcgccgc tgcgagcgcg gaaggcagcc ccaccgcccc gacgatggcg 183120
cacatgacac cgctgacttc gtcggcagag gcaacgtcaa ccaatgcctg cgcgccgtgt 183180
aaaacctgag tcgccgtgcc gtcgcgtttc aacagggctt caagccgggc ggcgtgttcc 183240
gcatcggcaa cgacggcata ttcggggtgg aacgtttgac attgagccgc caatttctcg 183300
acctgcttat gccctgccag cgcgaatacg cggaattttt cggggtggcg ggagacaacg 183360
tccagcgtgc tttcgcctat gctgccggta ctgcctaata tggtcaggac ttgtggtgtc 183420
ataatgggga taactttata ccggatgccg tctgaagcgt tttcagacgg catagaatca 183480
atttaaaacc gacatcatcg ctgcatagac gctgataacg gcaatcaggc tgtcggtacg 183540
gtcgaacacg ccgccgtgtc cgggcagcag cttgctgctg tctttgatgc ctgccgcgcg 183600
cttgagccag ctttccaaaa ggtcgccgca tacgctgaca acggtcagca ccaaaccgat 183660
taacacggta tcgaaccagc ctgtatcgaa tgccagccag ccggcacttc gtacggcggt 183720
catgtacact gccacgcaaa ccgcgccgcc gattgcacct tcccagcttt tgccggggct 183780
gattgccggc gcgattttgt gtttgccgaa cgccttgccg ctgaaatacg cgcaaatatc 183840
ggcaacccac accaaaccca tcacggcgag cagcggcagg gcatcatcgg gatgcgggcg 183900
cagggatacg agcgcgaacc aaaacggcat gaccagaagc cagccgacgg cataaacctg 183960
ccaaccgccg ttgagcctcc atttgaatct caaccataaa ggcataacgg cgagccaaaa 184020
tgccaaaaca acataccaaa ccaaattagg cagcatccag ccgcccgcat aggcaaccac 184080
gccgaaaacc aaggttgcgg cgaggtaatg gttggtttta attttgcaca aaccgcccat 184140
acgggcatat tcccacaagg caatcagggc aatcagtccg caaaatgcag cccacaacca 184200
ttgcggcgcg taaaacagca tgcccagcat cagcggcagc agccacatgg cggttattac 184260
ccgttgtttc agcatattca gttcctttgc tgttcgatag gcagttgctc ggaggtgcgt 184320
ccgaaccgcc gttcgcgttt ttggaacgaa gcgacggcat cgtccaaagc cttgccgtca 184380
aaatcgggcc acaaaatatc ggtgaaatac agttctgcat atgccatctg ccagagcagg 184440
aaattgctga tgcgcgtttc gccgccggtg cggatgaaca aatccggttc cggtgcatcg 184500
cccagcatca agtgtttcgc cagcgtgtct tccgtaatct cggatacgcc ttcggcaatc 184560
agtttgtttg ccgcctgcaa aatatcccag cggccgccgt aatcggcggc aatgctcagg 184620

gtcaggccgg tattgtttgc cgtcaacgct tccgcctctt cgatgccttg cagaatctgc 184680
cggttgaagc gttcgcggct gcccaatatc ttcaggcgca tattgttttc gtgcaggcgg 184740
cgtacctgtt tttgcaaagc ctgtaaaaac agccccatca ggaacgaaac ttcgtcttcg 184800
gggcggcgcc agttttcggt tgaaaaggca aacacggtca gatattgcac acccagtttg 184860
gcgcaatgct tcaccatatt ttccaatgcg tccaaaccgc gtttgtgtcc cattatgcgc 184920
gggaggaaac gtttttcgc ccaacggccg ttgccgtcca taatcacggc gatatgcttg 184980
ggaatggcgg tgtgttccaa aacggcctgc gtgctgcttt tcatgtctgc ctttcgcggt 185040
tcggcattca aatgccgtct gaacgccgaa ccgtgcaggt taaattgcca tcaaatcttc 185100
ttctttggca gtcaggagtt tgtcggcttc ggtaatgtat ttgtcggtca gtttttgaac 185160
cgcttcttcg ccgcgacgtg cctcgtcttc ggaaatttct ttgtctttga ggagtttttt 185220
gatgtggtcg ttggcatcgc ggcgcacgtt gcggatagag acgcggcctt cttccgcttc 185280
gccgcgtacg actttaatca ggtctttgcg gcgttcctcg gtcagcatgg gcatcggcac 185340
gcggatcagg tcgccgacag ctgccgggtt cagtcccaag tttgaatcgc ggatggcttt 185400
ctcgactttg gccgccatat tgccctcaaa cggtttcacg ccgatggtgc gcgcgtccag 185460
aagcgttacg ttggcaactt ggctgacggg gaccatgctg ccccagtatt cgacttccac 185520
ttggtcgagc aggccggtat gcgcgcggcc ggtacgcact ttcgccagat tttctttcag 185580
tacttcgacc gaacgctgca tcttgccttc ggctgttttt tgaatatcgt tgatcatatt 185640
gttctttcgg tgggataagg tgggcgggag accgtctgaa cgcgtttcaa gccgttcaga 185700
cggcataaag accgttaacc gcgaatagta ccgttattcg ggcataacga caaggtaggc 185760
ggattgggga tgccgtctga agcgacaggc gtttcagacg gcatcgtgtc cgaccgtcag 185820
ccgtgttccc gtgtttcaag caggctttgg cgcaggtgtt ggcgttcgtg ggcatccagc 185880
catttgcggc gggtgcgttg cagcaggatg acgagggcgg aaatttcctg acgcatattg 185940
gtgctgagcc agaggaagcc ctgccattgg tagtggaggt gttcggcgag ggcttccagt 186000
tcggggttga tggcggtgtc gatgcggatg cggcgggcgt gtctgccgtt gataagggcg 186060
acggtttgtt gcaggtcggt ttggagcagt gtgaagtggc ggtcaagcag ccggatttcg 186120
ctgccgttga gtttgggaga ttgcagcttg gcggcggtgg tcaggagcag ctcggtggtg 186180
ttgacgattt tacggtgggc gtgctgcatg gcttccatca tggcggggct gatgcggctt 186240
tcgcccgatg tggcggcgag atggctgcgg cttttgacca tgcgtgcgtt gatttggcgc 186300
atttcgcca tgttctcctc gaggcgttcg cgggtcatgc gcctgccgtt gctgatttcg 186360
gcaatcattt tgctgcagtc ggccaggttg tcggcaagca tgaaacgcca catcagtgtg 186420
gatttcagcg gcagcagttt ggcggcggcg atggcgatgg ccgcgccgat gaggacgttc 186480
atggcgcgca tgagtccgct gtcgagccat tcgctgccgt tgtcgccgat gagcatacac 186540
atcgtcagcc ctgccagcat agggacgtag ccgtttttgc cgaccgccgc ccagccggcc 186600
agtgcgcttg ccgtgccgac ggtgaggtag aagaggaggt tgccgtggaa ataatgctgg 186660
ttcagccata aaacgcccaa acccgcgccc agcccgatga ccgtgccgag catacgttcc 186720
accgccttgg agtaaatcgc cccttgaaac tggagcatgc cgaggacgac gaagacggtc 186780
atccctatcc actcgccgtg ttggaggtgg agcagccggg cggaggcggt ggcgaacagg 186840
acggccccgc cgagccggac ggcgtggatg aggcggcggt agcggtagcg ttcgtaggag 186900
ttgagccagc ggctgacgag gcggttgcgt tgcgaggtgt tcatatcggt tgtgccgtct 186960
gaagcggaaa tgtgaaaaag cacaggcttc ccgaggaagg gagggtctgt gcttggtatt 187020
ggtgccggag aagggaatcg aacccccgac cttcgcgtta cgaatgcgct gctctaccga 187080
ctgagctaca ccggcgtttt ttcgtcatga tatatatgaa cggttgtttg tgcaactttt 187140
cgggcgggcg gcaaggcagt gcgcggtata gtggattaac aaaaaccagt acggcgttgc 187200
ctcgccttag ctcaaagaga acgattctct aaggtgctca agcaccaagt gaatcggttc 187260
cgtactattt gtactgtctg cggcttcgtc gccttgtcct gatttttgtt aatccgctat 187320
ataatgcggt ctgcttcgga agagggggac ggcgatgttt gtgaacgaga aatatcctta 187380
tgcggctctg tttgcgggac tggtgttttt gacgctgccg tttgcgttgg cggtgcatga 187440
tgcctttgcg cttgcgttcg gacggacggg gttgctggtg tcggtgtcgg acggcggatt 187500
cggctggcgt ggcggttggg acggcactgt ttggtttgtg ttcggtgtgt ttgcgttttt 187560
gaatgtggtt gtgtcggcgg gtctgacgaa actggcgtac aaaaagatga tgcggcggca 187620
ttcgcgttac acactgtttc tgtcgggcgt ggcggcttgc gcggcggcag cggtggcttg 187680
gattttcgag ctgctgcttg gcagtggggc tttgggcggg ctgcgggggga ggcggtgttg 187740
gaatatgcgt ttgccgtgtg gctggtggcg atgctgacgc tgcccaaacg cctgacgcgc 187800
gcgccggtgc agccggtggt gtttcacagg aaaaaatagg ttggaacggg aaatgccgtc 187860
tgaaacccga cacgcggttt cagacggcat gtttttccgc taacattacg cctgaatatg 187920
gacaggaagc agatatggaa cgcaaagaac gcctgcgtgc aggcattgcc gcgatggggc 187980
tggatatttc ggaaacggcg caggacaggc ttttggtcta tgtggatttg ttgaaaaagt 188040
ggaacaaaac ctacaatctg accgccctgc gcgacgagga aaaaatgatt gtccatcatc 188100
ttttggacag cctgacgctg ctgccccata tcgagggtgt gcaaacgatg ctggatgtcg 188160
gttcgggcgg cggtcagccc ggcattccgg cggcggtgtg ccgtccggat gtgcaaataa 188220
ccctttttgga tgcgaatacg aagaaaacgg ctttttttaca gcaggcggtt atcgagttgg 188280

ggttggacaa tgtgcgcgtg gtatccggac gcgtggaggc ggtttcggac gtgcgtgccg 188340
atgtggttac cagccgtgcg tttgcagaac tggcggattt tgtgtcgtgg acggtgcatc 188400
tgttgaaaga cggcggctac tgggcggcga tgaagggcgt gtatccgcag gaagaaatcg 188460
gccgcctgcc gcaggatgtg tgcgttgaaa aagtccaaag gctcgacgtg ccgggcttgg 188520
atgcggaacg ccatatcgtc atcctgagca agcgttgagc gcacttcaga cggcatgaat 188580
acctttttg tgcggataaa ggtaaaattc cgcactgttt ttcttttttc aacatcagac 188640
gggacacggg cgggacatga gtgcgaacat ccttgccatc gccaatcaga agggcggtgt 188700
gggcaaaacg acgacgacgg taaatttggc ggcttcgctg gcatcgcgcg gcaaacgcgt 188760
gctggtggtc gatttggatc cgcagggcaa tgcgacgacg ggcagcggca tcgacaaggc 188820
gggtttgcag tccggcgttt atcaggtctt attgggcgat gcggacgtgc agtcggcggc 188880
ggtacgcagc aaagagggcg gatacgctgt gttgggtgcg aaccgcgcgc tggccggcgc 188940
ggaaatcgaa ctggtgcagg aaatcgcccg ggaagtgcgt ttgaaaaacg cgctcaaggc 189000
agtggaagaa gattacgact ttatcctgat cgactgcccg ccttcgctga cgctgttgac 189060
gcttaacggg ctggtggcgg cgggcggcgt gattgtgccg atgttgtgcg aatattacgc 189120
gctggaaggg atttccgatt tgattgcgac cgtgcgcaaa atccgtcagg cggtcaatcc 189180
cgatttggac atcacgggca tcgtgcgcac gatgtacgac agccgcagca ggctggttgc 189240
cgaagtcagc gaacagttgc gcagccattt cggggatttg ctttttgaaa ccgtcatccc 189300
gcgcaatatc cgccttgcgg aagcgccgag ccacggtatg ccggtgatgg cttacgacgc 189360
gcaggcaaag ggtaccaagg cgtatcttgc cttggcggac gagctggcgg cgagggtgtc 189420
ggggaaatag gtcaatccaa atcgggctgc ccgtgccttt atgctgtttg gccgggtgcg 189480
ttatagtgga ttaacaaaaa tcaggacaag gcgacgaagc cgcagacagt gcaaatagta 189540
cggaaccgat tcacttggtg cttcagcacc ttagagaatc gttctctttg agctaaggcg 189600
aggcaacgcc gtactggttt ttgttaatcc actataatat ggcggattaa aataaaaata 189660
cttatatcgt catttatcgt cattcccgca aaaacaaaaa aatcaaaaac acaaaactga 189720
aatatcgtca ttcccgcgca ggcgggaatc taggtctgtc ggtacggaaa cttatcggga 189780
aaaacggttt ttccaaccct gagactccgg attcctgttt tcgcgggaat ccggtttttt 189840
gagtttcagt catttttgat aaattcttgc agctttgagt ttctagattc ccgcttttgc 189900
gggaatgacg cggaaaagtt gctgtgattt cggataaatt ttcgtcacgc ttaatttctg 189960
ttttatccga taaatgcctg caatctaaaa tttcgtcatt cccgcaaaaa caaaaaatca 190020
aaacagaagc ctaaaatttc gtcattcccg cgaaggcggg aatctaggtc tgtcggtacg 190080
gaaacttatc gggaaaaacg gtttttccaa acctgagact ccggattcct gttttcgcgg 190140
gaatccggtt ttttgagttt cagtcatttt tgataaattc ttgcagcttt gagtttctag 190200
attcccgctt ttgcgggaat gacgcggaaa agttgctgtg atttcggata aattttcgtc 190260
acgcttaatt tctgttttat ccgataaatg cctgcaatct aaaatttcgt cattcccgcg 190320
aaggcgggaa tctaggtctg tcggtacgga aacttatcgg gtaaaacggt tttgccagcc 190380
ctgagactcc ggattcctgt tttcgtagga atccggtttt ttgagcttca gtcatttttg 190440
ataaattctt gcagctttga gtttctagat tcccgctttc gcgggaatga cggtttggaa 190500
gttacctgaa attcaaaaaa aaaacggaaa ccggacggat tggattcccg cctgcgcggg 190560
aatgacggat tttaggtttt ttttttgatt ttctattttt cgcgggaatg acggtttggg 190620
ttctttctct ttggagttgc gatgccggaa atgccgtctg aaggcttcag acggcatttt 190680
tgtgccggtt taaaacaagg cctgctgcgc gagcaggttt ctgacggggg cgaagtcgcg 190740
gcggtgttcg ggcagcacgc cgtatttttc gagggcttcc aaatgctgct tcgtgccgta 190800
acctttgtgt ttgtcgaaac cgtattgggg atggcgttgc gccagtgcgt acatttccgc 190860
atcgcgtgcg gtctttgcca aaacggatgc ggcggagatt tcgatgattt tgctgtcgcc 190920
tttgacgacg gcttcggcag ggatgttcaa atgttcagga atgcggttgc cgtcgatgaa 190980
tattttttcg ggacgcacag ccaagccgtc aacggcgcgt ttcatcgcga gcatggtggc 191040
gtgcaggatg ttgaggctgg cgatttcttc gggcgaggcg gcggcaacgt gccactcaac 191100
cgcctgattt tttatcattt cggcaagcgc gtcgcgtttt ttctcgctga gtttttttgga 191160
gtcggtcagt ccgggcaggt cgaatgtttc cggaaggatg acggcggcgg caaacacgct 191220
gccgactaaa ggtccgcgtc ctgcctcgtc cacgccggcg gtcagtatgt gcatgatgtt 191280
tcctgtcggg atggtgggaa tgccgtctga aaagggtttc agacggcatc gcgccgatgt 191340
gtttatttcg cgtctttaaa cccgcgcttc aaatgcacca tcagcaatgc cactgccgca 191400
ggggttacgc cggaaatgcg gctggcttgt ccgacggttt cgggtttgtg ctggttgagc 191460
ttttgctgca cttctgccga caagcctttg actttgccgt aatcgatgcc gtcgggcagt 191520
tttaaggttt cgatgtcgcg gcggctgtcg atttcttcgt tttggcggtc gatatagcct 191580
tggtatttga cttggatttc gacttgttcg atgacttcgg cggagaggtt ttcagacggc 191640
atcgcgcctt cgagcgtcat cagcgcggcg tagtcgaggt ttgggcggcg caggaggtcg 191700
tgcaggttgg cttcgcggct gagtttttgt ccgaacacac ggatttgttc gccttcggcg 191760
agtttttgcg gcgtgtacca cgttgttttc aaacgttgga tttcgcgttc gacggcttcg 191820
cgtttttcgt tgaacatgcg ccattgcgct tcggacacca agccgatttt gtagccgtct 191880
tcggtcaggc gcatgtcggc gttgtcttcc ctgagttgca ggcggtattc ggcgcggctg 191940

gtgaacattc ggtagggttc gttcacgcct ttggtgatga ggtcgtccac caatacgccg 192000
aggtaggctt gttcgcggcg cagcaggagc gggtcttgtc cgcgcacata ttgcacggcg 192060
ttcgcgcctg ccaataaacc ttgcgcggcg gcttcttcgt agccggtcgt accgttgatt 192120
tgcccggcga aaaacaatcc ggcaatggtt ttggtttcga ggcttgcttt gaggttgcgc 192180
ggatcgaagt agtcgtattc gatggcgtag ccggggcgca ggatatgggc gttttccaaa 192240
cctttcatac tgcggacgag cgcgatttgg atgtcgaacg gcaggctggt ggagataccg 192300
ttaggatagt attcgtgcgt ggtcagacct tcgggttcga ggaaaatctg gtggctgtct 192360
ttgtcggcga agcggttgat tttgtcttcg atagacggac aataacgcgg acccacgcct 192420
tcgattttgc cggtaaacat cgggctgcgg tcgaagcctg agcggatgat gtcgtgggtt 192480
tgcgtgttgg tatgcgtaat ccagcaggac acttggcgcg ggtgcatatc ggcgttgccg 192540
cgcacggaca tgacgggaac gggcgtgtcg ccgggctgtt cggtcagttg ggagaagtca 192600
atcgtgcgtc cgtcaatacg cggcggcgtg ccggttttca gacggccttg cggcagcttc 192660
aattcgcgca aacgtccgcc caacgatttg gcggcggggt cgccggcgcg tccgccttcg 192720
tagttttcca aaccgatgtg gattttgccg gacaaaaacg tgcctgcggt caacacgacg 192780
gcgcgtgctt taaactccac gcccatcgcg gtaattacgc cgctgatgcg ttcgccgtcg 192840
agcgttacgt cttcgacggc ttgttggaaa aggtcgaggt tttcttggtt ttccaacatt 192900
tcgcggatgg cggctttgta caggatgcgg tccgcctgcg cgcgcgtggc acgcactgcc 192960
gcgcctttgc tggcgttcag gcggcggaac tggataccgg atttgtcggt tgccaacgcc 193020
atcgcgccgc cgagcgcgtc gagttcgcgc accaaatgcc ctttgccgat gccgccgata 193080
gaggggttgc acgacatttg tccgagcgtt tcgatattgt gtgagagcaa aagcgtctgc 193140
gcgcccatac gggcggcggc gagtgcggct tccgtgccgg cgtgtccgcc gccgacgacg 193200
ataacgtcgt aggttttggg gtaaatcatg tgggtcatag tgtgtattgc ctgacggtgt 193260
ttcagacggc atttatagtg gattaacaaa aaccagtaca gcgttgcctc gccttagctc 193320
aaagagaacg attctctaag gtgctgaagc accaagtgaa tcggtttcgt actgcttgta 193380
ctgtctgcgg cttcgtcgcc ttgtcctgat ttttgttaaa ccactatatt caatatgccg 193440
tctgaaaaac gaaatggatt caaaagtaaa gggttgggat tgtacgcttg ttcgccctgt 193500
ttttacagtg tgcggaaagg gaaaagccgc ttcgcgggga agcggctccg gtaagggcgg 193560
gatttaccaa acgtcggatt tgatacggcg tttcaggccc ggatgttcgg aaagtttgaa 193620
ctcggggtct ttgcccattt tcagcttggc ggtgtaatcg cgcagcagca taaacgccaa 193680
gggcgagagc agcaggatgg cgacaaggtt gatccacgcc ataatgccca tcgccatatc 193740
cgccatatcc cagaccaaag gcacattggc aaccgcgccg aaatagaccc acgccaaaac 193800
cagcatacgg aaaacggcgg taatcagcca atggcttttg atgaattgga cgttggactc 193860
ggcataggca tagttgccga taacggtgga aaaggcaaac ataaacagga tgacggcgag 193920
gaagcccgcg ccccattgcc ccacttggct gacaatcgcc gcctgcgtca gcgccgcacc 193980
gctcaaatcg ccgtaaggct gttggtaaat caagatgatg aaggcggtgc aagaacaaac 194040
gatgatggta tcgacaaaca cgcccagcat ttgaatcata ccttgcgaaa cagggtgttt 194100
cacttcggcg gcggcggcgg cgttcggcgc ggaacccata cccgcctcgt tggaatacag 194160
gccgcgtttg atgcccatca tcatcgtttg cgaaatcaga ccgccgagta agccgcctgc 194220
tgccgcgtcg aatttgaacg cgcccgaaaa aatctgaccg aacacgtccg gaatcatcgg 194280
aatattggtc aaaatgatga aaagcgcgat aaagaggtac aaaaccgcca tcagggggac 194340
gacgatttcc gccgctttag atatgcgcct gatgccgccg aagataatcg gcgcggttaa 194400
aatcaccagg gcgacgccga cataatgagg ctcccaaccc catgccgctt tgacggtatc 194460
ggcgatggta ttggtctgaa ccgcttcaaa cacaaagccg aaacagaaaa tcaggctcag 194520
ggcgaacaac acgcccagcc atttctgccc cagcccttga gtgatgtagt aggcagggcc 194580
gccccggaaa tggtggttgt cgtagtcgcg gactttaaag agctgcgcca gcgaagattc 194640
gacaaacgcc gaactcatac cgattaaggc ggttacccac atccaaaaca ccgcgcccgg 194700
tccgccgact ttgatggcga tggccacgcc cgcgatattg cccacgccca cgcggctggc 194760
aaggccggtt acaaatgcct gaaacggcgt gatgccgtga gggtcgtccc cctgtttgcg 194820
gccgccgagc atttctttga tgctgcgccc gaacaggcgg aattggacaa agcccgtggt 194880
tacggtgaag aaaagccccg tacccaaaag catataaacc aagtatgacc acatcggatc 194940
gttgatggcg ccgacccagc cgtgcagcca ttcggtaaag ttctcgttca tatcgcttcc 195000
ttaaagttga aactcgcaca tattggcggt atgcaagcag ggtttaaatt ttgtaaacgc 195060
ccattctagc agattgtcaa caaaatcaga aaaatttaca tcgccgcgcg gctgcggcgt 195120
tagaatcgca ttttgtttgg agcaaacacg atgaaacagc ctgtttttgc cgttacttcc 195180
ggcgagcctg ccggcatcgg ccccgatatt tgtttggact tggcgtttgc acgcctgccc 195240
tgccgctgcg cggtattggg cgacaaaaac ctattgcgcg cgcgcgccga agccttgggc 195300
aaaagcgtcg tcctgcgcga cttcgatcca gaatcaggcg gcgcggcata cggcgagctg 195360
gaagtgctgc acatccctgc cgtcgaagcg gttgaggcgg gcaaactcaa tcccgccaac 195420
gccgcctatg tgctgcaact tttggacacc gcgctcgcag gcatttcaga cggcattttc 195480
gacggcatcg ttaccgcgcc gctgcacaaa ggcatcatca acgacgcgcg cgcaagcaca 195540
ggtttttca gcggacacac cgaatatctg gcggaaaaaa gcggcacggg gcaggtcgtg 195600

atgatgcttg ccggcaaagg cctgcgcgtc gccctcgtaa cgacccacct gccgctgaaa 195660
gacgttgccg ccgccatcac gcaaccgctg attgaaagcg tcgcacgcat tttgcatcac 195720
gacttaaaac acaaattcgg catcaaaaat cccaaaatcc ttgtcgcckg acttaatccc 195780
cacgccggcg aaggcggaca cctcggacac gaagaaaccg acaccattat ccctgcattg 195840
gaaaacctgc gccgcgaagg gataaacctt gccggcccgt atccggcgga cacattgttc 195900
cagccgttta tgctcgaagg tgcggatgcc gtattggcga tgtaccacga ccaagggctg 195960
cccgtgttga aataccacag cttcggacag ggcgtgaaca tcacgctcgg cctgcccttt 196020
atccgcacct ccgtcgatca cggcaccgcg cttgatttgg cggcaaccgg cagggcggat 196080
tccggcagcc tgataactgc cgtggagacc gccgtcgaga tggcgcgcgg cagcctttaa 196140
agatgataaa agacccgtca tttccgcgca ggcgggaatc cggtctgttc ggtttcagtt 196200
gtttttgggt ttcgggtaat ttccaaatcg tcattcccgc gcaggcggga atccagacca 196260
ttggacagcg gcaatattca aagattatcc gaaagtttga ggttctagat tcccgttttc 196320
acgggaatga cgaaaggtgg cgggaatccg gtctgttcgg tttcggtttt ttttttgag 196380
gtttcgggca acttctaaac cgtcattccc gcgcaggcgg gaatccagac cattggacag 196440
cggcaatatt caaagattat ctgaaagttt gaggttctag attcccgttt tcacgggaat 196500
gacggaatgt tgcgggaatc cggcttgttc ggtttcggtt tttttgaggt ttcgggcaac 196560
ttctaaaccg tcattcccgc gcaggcggga atccagacca ttggacagcg gcaatattca 196620
aagattatct gaaagtttag aggttctaga ttcccgtttt cacgggaatg acggaatgtt 196680
gcgggaatcc ggcttgttcg gtttcggttt tttttgaggt ttcgggcaac ttctaaaccg 196740
tcattcccgc gcaggcggga atccaggcct ttgggcgacg gcaatattca aagattatct 196800
gaaagtttag aggttctaga ttcccgtttt cacggaaatg acgaaatgtt gtgggaatcc 196860
agaccttcgg gcagcggcaa tattcaaagg ttatctgaaa gtttgaggtt ctagattccc 196920
gttttcacgg gaatgacgaa aggttgtggg aatccagacc ttcgggcagc ggcaatattc 196980
aaagattatc cgaaagtttg aggttctaga ttcccgtttt cacgggaatg acgaaaggtg 197040
gcgggaatga cgaaaggttg cggtaatcat gggaatggcg aagtttcaga cggcatcgtc 197100
caccctccgc cgtcattccc gcgcaggcgg gaatccaggc ctttgggcga cggcaatatt 197160
caaagattat ccgaaagttt gaggttctag attcccgttt tcacgggaat gacggaatgt 197220
tgcgggaatc atgggaatga cggaatgttg cgggaatcat gggaatgacg gaatgttgcg 197280
ggaatcatgg gaatgacgga atgttgcggg aatcatggga atggcggaat gtttcggtaa 197340
tcacgggaat ggcgaagttt cagacggcat tgcaggtatc cgaacccatg taaaaaagag 197400
gttctgcgga acagaacctc tttttgccgc cgtcggttca gccttgccgg gtttcgactt 197460
ggatcatttc ttcggcaggg acggttgcga cttcagacgg cttgggctgt tcggaacggc 197520
gcaaaccgcg tccggcttgg acttcgggtt gtgccgccca tgccttcaat gcggcagggt 197580
cggtttcgat caggacgagt ccgccggttt gtgcggtttc ccgtgcctgt tccgccgcag 197640
ccgtaaaggt tgcggtttca gacggcattt cctgtgcttc ggctttcggt gtcgcgcctt 197700
cgggcaggat ggcggcggtg gcacggcgga ttttttccgc cgcatcataa accggtgcgt 197760
cgccgtttga aacggcggga gatgctgtcg gaagatccct ttctgcaacc ggatcggcaa 197820
tgctgacagt aatcggcgcg tttgcgtcgg tttcgccgaa aacgtgcgcg gcggcggaac 197880
ggactttgtc ggcggtgtcg tgaatattca ggtactgctc gattttggcg gcagacggaa 197940
tattgcgttt tttgccgttt tgacggcggt cgcgctgatt gttgcgctcg cggcgttctt 198000
tggcatctcg gctgtcgcgt tcgcggcggt tgcgttcgga tttgggcttg ctgcctttgt 198060
cttctgcggt atgcggttcg gacggcgtgt tttccgctgt ctgaacggtt gtttcggcaa 198120
cggtggcggt ttccggcgcg gtttggccgc gttcgctgcg gctgccgttg cggcggcgtt 198180
ttccggtttg cacttcggtt tcggacggtg cggcatctgc aacggttgcg gcaggctgta 198240
cgttgcggct ttggatttcc gcttcgttgg cgcgttcggc ggcacggtcg ccgcgttcat 198300
tgcggcggcg gttgccgttg ttgcgcgttt cggctttgtc ggcacgcgct tcctgtccgg 198360
cagttttgcc tgccacttcg cggacttcta ctttgctgcc ttcgcgtttg ctgcggcgcg 198420
ggttttggcg gcggttgttg gcgcggctgc cgctgcggtt tgccgtgctg cgtttttcgg 198480
aggtttcggc agcgggcgcg gcttgggttt cgctgccgcc gaaaatgcgt ttgagccatg 198540
ctttgaagct gtcccaccaa gaggtttttt tctcgggggc ggcagtcggg gcggggctgg 198600
tgtggcgcac gcctttgacg gcgggttcgg gacgggcggc tttggctttt tcgccgccga 198660
acggtttggc ggattcgtct tcttccggct cggcgacgcg tttgtagctc ggttcgccgt 198720
cttcttctac gtcgtcggtg cggatgcggt tgatttcgta gtgcggattt tcgaggtgga 198780
tgttcggaat caggacgacg ttgacatcca aacgctcttc catcgcaaac agctcggcgc 198840
gtttttcgtt cagcaggaag gtggcgacat cgacgggcac ttgtgcgcgc acttctccgg 198900
tgttgtcctt catcgcttct tcttgaatga tgcgtaaaac gtgcagggcg gtggattcga 198960
tgccccgaat cacgccggtg ccggcgcagc gcggacaggc gacgtggctg ctttcgccca 199020
aagccggttt caaacgttgg cggctcaatt ctaaaagtcc gaaacgggag agtttgccca 199080
tctgcacgcg ggcgcggtct tttttgagcg cgtcgcgcag gacgttttcc acatcgcgct 199140
ggtgtttggg gttttccatg tcgatgaagt cgatgacgac caagccgccc aagtcgcgca 199200
ggcgcatttg tcgggcgact tcttcggcgg cttccatatt ggttttgaac gcggtgtctt 199260

caatgtctgc gccgcgagtg gcgcgtgcgg agttcacgtc gatggagacg agggcttcgg 199320
tatggtcgat gacgatcgcg ccgccggagg gcaggctgac gctgcgcgaa aacgcgcttt 199380
cgatttggtg ttcgatttgg aagcgggaaa acagcggcgt gtggtcttcg tagagtttca 199440
gacggcctat attgcccggc atgacgtagc tcatgaactc ggcaacttgg tcgtaaactt 199500
cttgattgtc caccaaaatc tcgccgatgt cggggcggaa atagtcgcgg atggctcgga 199560
tcagcagcga gctttccata aagagcaggt aggggtcgtg atgcgctttt cctgcttctt 199620
caatcgcctg ccagagttgt ttgaggtagt tcaagtccca ttccaactct tccgcgctgc 199680
ggccgatgcc ggcggtacgg gcgatgatgc tcatgccgtt cggaatgtcg agttccgcca 199740
tggcggcttt caactcttga cgctcttcac cttcgatacg gcgggatacg ccgccgccgc 199800
gcgggttgtt cggcatcaat accagatagc gtccggcgag gctgatgaag gtggtcagcg 199860
cggcgccttt gttgccgcgc tcgtcttttt cgacttggac gatgacttcc atgccttctt 199920
tgagcacgtc ttggatgcgc gcgcgtccgc cttcgtagtc ttggaagtat gagcgggaga 199980
cttctttaaa cggcaagaag ccgtggcggt cggttccgta atccacgaaa cacgcttcca 200040
gcgacggctc gatgcgggta atgatgcctt tgtagatatt gcctttgcgc tgttctttgc 200100
ccagcgtttc gatgtccaaa tccagcaggt tttgtccgtc gacgatggca acgcgcagct 200160
cttcggcctg cgttgcgtta aataacattc ttttcatgat cacctcgtgg gcaggcggcg 200220
ttcagacggc acatgcccgg ttcggcattc cgtaaggctg ggttttccga tgttttcgga 200280
taaaaccggt aatcagtttt tgagttgaaa atccgcaggg atgcacgttc cggagaaccg 200340
tgtgcggaag ggtcggatac agaaggctat aaagatcgat gcggcggttt gtctgccgcg 200400
ttccgaacgc tgcggtcgga aaaatggggg ccggcttctt cttgttatcg tgatgcctgt 200460
gttttgggcg gtttgcgttt gggacttggg cccggctgcc gtcttacttc cgcgccgaaa 200520
cggcaaaatc aattcaaact tgattacgtt ctgcgcctgc cggctgggaa caggcgcagg 200580
gaaaatgctt tgcggagtgc gtttttaata taaaattccg ttttaaagta aaccgtttca 200640
ggaggcgcgg cgggcgcgct ttttgctgaa acggatgttc ggattataga tgaaaacgca 200700
cgaaataagc aaagattcgg tcagcttgat aggggttgcc gaacatgagg cgggtcaacg 200760
ccttgataac tatctgataa aaatcctcaa gggtgttccc aagagccata tccaccgcat 200820
tatccgcgcc ggcgaggtgc ggttgaacaa gaaacgctgc aaacccgaca gccgtattgc 200880
ggaggggggat acggtgcgga ttccgcctgt gcgcgtggcg gagaaggaaa tgccgtctga 200940
aaggcgtgcc gccgtaccgg cgcgtgcgtt tgacgttgtt tacgaagacg atgcgctttt 201000
ggtcatcgac aaaccgtccg gcgttgccgt ccacggcggc agcggcgtga gtttcggcgt 201060
tatcgaacag ttgcgccgcg cccgtccgga ggcgaagtat ttggagttgg ttcatcgttt 201120
ggacaaggat acgagcggct tgttgatggt ggcgaagaaa cgcagcgcgc tcgtcaaact 201180
tcacgaagcc atccgtaacg accaccccaa aaaaatctac cttgcgctgg gggtgggcaa 201240
actgccggac gacaatttcc atgtcaaact gcccctgttc aaatataccg gcgcacaagg 201300
cgaaaagatg gtgcgcgtca gtgcggacgg gcagtcggcg catacggtgt tccgtgtgtt 201360
aagccgtttt tcagacggca ttttgcacgg tgtcgggctg tcgcacctga ctttggtgcg 201420
ggcgacgttg aaaacggggc gcacgcacca aatccgcgtc cacctgcaat ctcaaggctg 201480
tccgattgcg ggcgacgaac gctacggcga ttatcaggcg aaccgtcgtt tgcagaagtt 201540
gggtttgaag cggatgtttt tgcacgcgtc cgagctgcac ttgaaccatc cgctcacggg 201600
cgagccgctg gtgttgaagg cggagctgcc gccggacttg gcgcagtttg cggtgatgtt 201660
ggaaaacggg acgaaaatgt gaaccccgat gccgtctgaa gccttcagac ggcatcggga 201720
cgtgaaagta tgtggggaca gacgaatatg gctgataaaa aaagcccttt gattgccgtc 201780
agtgtcggcg aagcgtcggg cgacctattg ggggcgcacc tgatacgcgc catccgcaag 201840
cgttgtccgc aggcgcggtt taccggtatc ggcggcgaac tgatgaaggc ggaaggtttc 201900
gagagccttt atgatcagga gcggctggcg gtgcgcggct ttgtcgaagt ggtcaggcgg 201960
ctgccggaaa ttttacggat acgcaggggg ctggtacggg atttgctgtc gttgaaacct 202020
gatgtctttg tcggtatcga tgcgcccgat tttaatttgg gtgtggcgga aaagctgaaa 202080
cggtcgggga ttccgaccgt gcattatgtc agcccgtcgg tgtgggcgtg gcggcgggaa 202140
cgtgtgggca aaatcgtgca tcaggtcaac cgcgtgttgt gcctgttccc gatggagccg 202200
cagctttatc tcgatgcggg cggacgtgcg gagtttgtcg gtcatccgat ggcgcagctt 202260
atgcccttgg aagacgaccg tgaaacggcg cggcaaactt tgggcgtgga tgccggcatc 202320
cccgtattcg ccctgctgcc cggcagccgc gtcagcgaaa tcgactatat ggcgccggtg 202380
ttttttcaga cggcattatt gttgttggaa cgctatcccg ccgcacgctt cctgctgcct 202440
gccgcaacgg aggcgacgaa gcggcgtttg gcggaagttt tgcagcggcc ggagtttgcc 202500
ggattgccgc tgacggtaat cgacagacag tctgaaacag tgtgcagggc ggcggatgcg 202560
gtgctggtaa cgagcggtac ggcaactttg gaggtggcgt tgtgtaagcg tccgatggtc 202620
atcagctaca agatttcgcc gctgacctat gcttatgtga aacgcaaaat caaagtgccg 202680
catgtcggcc tgccgaatat cctgttgggt aaggaggctg tgccggaatt attgcaatct 202740
gaagcaaaac cggaaaaact ggcggcggcg ttggcggact ggtacgaaca ccccgataag 202800
gttgccgcgc tgcaacagga tttcagggcg ttgcacctgc tgttgaaaaa agatacggcg 202860
gatttggccg cgcgcgcggt tttggaagag gcgggatgtt gagcggttaa tggattattt 202920

tcccgaagca gcacgtatta caaaaaaagg gggagaaatt gtgattaatg gcacatcaaa 202980
caataagtat ttaagaggaa ttccaaatga aacagaactg gcccgaatgg gattaaggtt 203040
aaaatataat ggtcagttaa ctgattaatt ttgttatata tgatttatga ttatagctta 203100
tactaatacg cttacttacc ttgtttcatt tgttcttcgt aaatttctat tttaggcaat 203160
tgtgtcagtt caatagggca agttgctccc caccaaaaat gttctacata aaaccaagga 203220
ttatctggaa aatatagcaa catctcttcc atatccggcc aaattcttct taattcatct 203280
acctgtgttt ttggcgaacc agttaatatt tttggaggat tttcacgata atcgcataat 203340
tcaataacac catctgataa aagttcttcc aaaaaatcaa aaaatctaat ttttaaattt 203400
ggatctttga tatccatatt taaataattt ttataaacac caaaaatacc acctaaatat 203460
tcacaatatt ctaaaagatt atattttatc ttcacattca taacgtaacc tttatctaaa 203520
ttttaattct aatctttgcc catgtactga atcaggttga ttcctaaact caatcgtcca 203580
ttttgctcca gtttgttctc ggctagttga aaaattcctt aaaataaagg aagagtttaa 203640
acaactgaaa tttcataaga gtagtagaac caacttggac tcaaaaaatc ttaaactcat 203700
tgtttttgaa aaggtaaaat aatatgacaa cttataccat tccaaaaaaa gattatcaat 203760
ttctgtatat atatgagggc actctattaa actatacttt gaaaaacgat gaattccata 203820
tcatcgtcca gaatgtggat tatccggact ttcctcaaga gattcctaca ccaaattata 203880
cagactgggt aaaaaattaaa ttcaagcagt tcagctatct gaaatttatc tatggatacg 203940
ccacgaagaa ccaagataaa aatatcaaaa atgtattgga acttggagaa ttaaagcagg 204000
atgatgaaat cttggattat ggaggtgcgc tggaagtgat aggcagtagg tatgatcttc 204060
cgaccggttt tagtatagat atagtttgcc gggaaataga gttagaattt ttagatcagg 204120
agagtttcaa ttaaacgagc cgtagcttgt tatgctgagc aggcaacttt atcgtatttc 204180
cttttcggtt gaaaccccgc cactcggaca tctgtccttc ggggcggtag aatcagattt 204240
tatttgggag gggcgtaacc ccttccgaat cagggcaaca catagggcga cgctttatgt 204300
gtcgtcctgt gtgttgaaac attgatatgc cgatacggag cctgtcggca aaatgccgtc 204360
tgaacaatat cttttcagac ggcattttgt atgggggtta acggttgttc agcccgagta 204420
cgtcctgcat atcgtacaaa cccgttttgc cgttgaccca aactgcggcg cggacggcac 204480
cggcggcaaa ggtcatgcgg ctgctggcct tgtgggtgat ttccacgcgc tcgccgtcgg 204540
tggcgaagag ggcggtgtgg tcgccgacga tgtcgcctgc gcggacggtg gcaaagccga 204600
tggtcgacgg atcgcgcgga ccggtgtggc cttcgcggcc gtaaacggcg cattgtttga 204660
ggtctctgcc gagcgcgccg gcgatgactt cgcccatgcg taacgcggtg ccgctggggg 204720
catcgacttt gtggcggtgg tggccttcaa tgatttcgat gtcgtagcct tcgtttaata 204780
cgcgtgcgac ggtgtcgagg atgtggaagg tgaggttgac gccgacgctg aagttggcgg 204840
cgaaaacgat gcctgttttt tcggcggcag tgtggatagc ggctttgccc gtatcgtcga 204900
agcctgttgt gccgatgatg atgttgactt gtttttcaac gcatttttgc aggtgtttga 204960
gggtgggctc ggggcgggtg aagtcgatga gtacgtcgct ttgtgcgaga acggcgtcaa 205020
cgtcgtctga aatggcgatg ccggttttga gtccgacggc gtagcctgcg tccagcccga 205080
gggcttctga gcctgagtgt tcaagcgcac cggaaaggac ggtgtcggga tggttgttga 205140
cggcttcaac caatacgcgt cccatacggc cgtttgcgcc ggcgatggcg attttgagcg 205200
gtgtcatgtg tgttccttat ggtttgtctg tgttttggcg gtctttgagg gcttcggcag 205260
cgttttgcag gacgtcgcct tcggtgcgga cgagtacgcc gttttcaaaa tagacggtca 205320
gattgctgcg ttctttgatg atgccgttgc gggaggtgtt gaaggtatag tcccagcggt 205380
cggtatggaa tgcgtcgcgc agtatggggc tgccgagcag gagcaggact tggtctttgg 205440
tcatgccggg gcggagggcg gcaacggcgc gcggttcgag ttcgttgccc tgtatgattt 205500
tgagtttgta cgaggggaac agtgaaacgc gttcggcact gcacgcggca aggccgagga 205560
gggcggaaag ggcgaggatg agggttttgt tcacggaaat gcctttctgt gcaaatcggg 205620
atgggtagtg taacactgct tgaatatttt ataaaagcga acgataatca tacgattaag 205680
cggtatccgc cctgtccgcg catcggccgc cggtgcggtt ttactattgc aaactgctat 205740
ggtgcgatag tgggcaaaca ggccgaaatt gcgtattata acgtctattg ttttacaggg 205800
gtattgaata ttatggaaaa attcaacaat attgcacaac tgaaagacag cggtctgaag 205860
gttaccggcc cgcgtttgaa gattttggat ttgttcgaga cgcatgcgga agagcatttg 205920
agtgcggaag atgtgtaccg cattttgttg gaagagggtg tggaaatcgg tgtggcgacg 205980
atttaccgtg tgctgaccca gtttgagcag gcgggcattt tgcaacgcca tcattttgaa 206040
acgggcaagg cggtttatga gttggacaaa ggcgaccacc atgaccacat cgtctgcgtg 206100
aagtgcggcg aggtaacgga attccacaat cccgaaatcg aagccctgca agacaaaatc 206160
gcggaagaaa acggctaccg catcgtcgat cacgcgcttt atatgtacgg cgtgtgcagc 206220
gactgtcagg ccaagggcaa acgttaaatc cggacggttt gttgttcaga cggcattcat 206280
gattttggat gccgcctgtg tttttggaga actgtcatgc gtattccgct gcttgcccct 206340
gacaattatg cctttcccga tcctgcctat gctttggccc ggtgcgacgg gctggtcggc 206400
gtgagcggcg atttggatgc ggggcggctg cttgaggcgt atcggaacgg cgtgtttccg 206460
tggttttccc gggacgggtg gttttttttgg tatgcggtcg ggccccgtgc ggtggtgttt 206520
cccgacaggc tgcatattcc gcgctcgctg gcgaaaacgc tgcgcaacgg cagctatcgg 206580

gttgcggtca acggctgttt tgcggaagtg gtcgcgcatt gtgcggcagc ggcgcgcccg 206640
aatcaggacg gaacttggat tgcgcccgag tttcagacgg catatttgaa gctgcacgaa 206700
atggggtacg cgcattcttt cgagtgccat tatcccgatg aaagcggtga aacgaggttg 206760
gcgggcggct tttacggcgt tcagatcggc agggtgtttt atggcgaatc gatgttcgca 206820
ttacaaccgg atgcgtcgaa aatcgcgttt gcctgcgccg tgccgttttt ggcggatttg 206880
ggcgtggaac tgatagactg ccagcaggat acggaacata tgcgccgttt cggttcggag 206940
ctgctgccgt ttgcggattt tgccgaacgt ctgcggatgt tgaacgccgt gccgttgaaa 207000
gaggaaatcg ggcggcgcga agtggcgtgc aaggggcttt gatggcggct tatgctccgg 207060
tcaggttcaa atatggtgga ttatagtgga ttaacaaaaa tcaggacaag gcgacgaagc 207120
cgcagacagt acaaatagta cggcaaggcg aggcaacgcc gtactggttt ttgttaatcc 207180
actataaaat tagaaatgac gacagccgga taaaatcacg gtgaaaatga aaaatgccgt 207240
ctgaaacttg aaaacatcgg gtttcagatg gcattttgtt tgacggtttg ttgcttattt 207300
gagcgggcgc acttcaagtc cgaacatacg gcgtgcggtg ttcagcattt ggcagctgaa 207360
gccccattcg ttgtcatacc aagcgaacac tttgaccatg ttgccgtcaa cgactttggt 207420
cagtgttgcg tcgaagtggc tggcttcggt agtgtggttg aagtccatgg aaaccaaggg 207480
cagggtgttg tagcccaaaa cgcctttgag cgggcctgct tccgaggcgg ctttcatcag 207540
tgcgttgatt tcttcgactg tggtgtcgcg cgcggcttgg aagctcaaat ctaccaatga 207600
tacgttgacg gtcggcacgc ggatggcaag cccgtcgagc ctgcctttca attcgggcag 207660
taccaaaccg acggcttttg ccgcgccggt tttggtcgga atcatgtttt ccacgccgct 207720
gcgggcgcgg cgcaggtctt tgtggcgcac gtcggtaacg gtttggtcgt tggtcagcgc 207780
gtggatggtg gtcatcgcgc ctttgacgat gccgacgctt tcgctcaaca ctttggcaac 207840
cggcgagagg cagttggtgg tgcaggaagc gttggaaacg acggtcatgt cggcggtcag 207900
gacgctgtcg ttcacgccgt acacgacggt tgcatcgaca tcgtcgccgc ccggtgcgga 207960
aatgaggact tttttcgcgc cgctttcgag gtggattttg gctttttctt tgctggtgaa 208020
cgcgccggtg cattccatga ccaaatcgac accgagttct ttccacggca gttcggcagg 208080
gttgcgggtc gagaagaagg ggattttgtc gccgttgacg atgaggttgc cgccgtcgtg 208140
ggatacgtcg gcttcaaagc gtccgtgcac ggtgtcgaat ttggtcagat gggcgttggt 208200
ttcaaggctg ccgctggcgt tgacggcgac gatttggagt tggtcttgaa tctgataatc 208260
gtagatggcg cgcaaaacct ggcggccgat gcgtccgtag ccgttgatgg cgactttgat 208320
gcccatggtt tgttcctttg ttgagggttg ggtagatttt cggggcggat tatagcaaat 208380
ttgtagtggc gtgtaattaa tattttattg aaaacggcgc ggccggaagg gtgggcggta 208440
agatgcggac ggcacgggtg cggcggacgg agagcttgat aaaatgccgt ctgaagcggc 208500
ttcagacggc atatcaggga agggtcagga ggcggtattc tgtgcggctt cctgtttggc 208560
tttgtattgt ttgagatatt cgagggcggc ggctttttcg ctgtcgctgc cgtatttcat 208620
atcgcgttgg gcgcggcgca actcggcgcg ttcgcgggct tcggctatct gtttcgcctg 208680
atagtctttg cggttgtcgg cggcggcgag gcggtcttgt tgggtttgcg cttttgccat 208740
ggctttggcg atgaggtcgg cagggttaaa cgtcggtttt ttcggtgtgt cgggcgtttg 208800
cggacgcgcg ttgcggacgg cggcttcgcg ttcggcaagc atggccttgc gttcgtcggc 208860
ttcgcgctgt ttgcgttcgt tgcgtttgag gtagcgcgtg cgcgcgtgtt cggcggcggc 208920
aaaacggctg tcggcggaca ggctgaagcg gcgcgcgcgg ggcaggacgg tgtcggcaac 208980
gggctgcata tggatgcagt cgacggggca gggggcgacg cagagtccgc agccggtgca 209040
ttcgtcggcg atgacggtgt gcataagttt gcccgcgccc ataatggcat cggcagggca 209100
ggcgcggatg caggcggtgc agccgataca ggcggtttcg tctatccggg cgagtgcttt 209160
ggcttgggtt ttggcaggtg cgacaaaggg tttgccgagc agggcggaaa tgtcccgaat 209220
gacggtttct ccgcccgggg cgcagaggtt gtacgcttcg cctgttgcga ctgcctgtgc 209280
gtagggcagg cagccgtcgt agccgcattc gcggcattgg gtttggggaa gcaggcggtc 209340
tatggcggcg gctgtggcgg tcatgtcggt gtgcggctca aaatcgaaag ggcgtatttt 209400
agcagaattg tatgccgcgc ccgtttcgga tggtgcgcgg tgttttgtta taatgcggcg 209460
gcgtatgccg tttcagacgg catttttctg tattttcctg ttcggacggt ctatgaacga 209520
attttcgctt gcccctattg tgattgtttt gctggtgtcg gtcattacgg tgatcctgtg 209580
ccgcaagttc aacattccct ccatgctggg ctacctgctg gtgggctttt tggcggggcc 209640
cggtatgctc agcctgattc cgaaaagcca tgcgacggat tatttgggcg aaatcgggat 209700
tgtgttcctg atgttcagca tcggtttgga gttctcgctg cccaagttga gggcgatgag 209760
gcggctggtg ttcggtctgg gcggtttgca ggtcggcatt acgatgctgt cggtaatggg 209820
catactgatg ctgacgggcg tgccgttcaa ttgggcgttt gccgtgtcgg gcgcgttggc 209880
gatgtcgtcc acggcgattg tgagccggat tttgtcggaa aagacggaat tggggcagcc 209940
gcacggtcag atggcgatgg gcgtgctgct gatgcaggac atcgccgtcg tgccgctgat 210000
gattctgatt cccgcgctgg cgggcggagg ggacggaaat atttgggcgg ccttgggttt 210060
ggcgtttgca aaaatgctgc tgacgctggg gctgctgttt ttcgtcggca gcaaaattat 210120
gtcgcgatgg ttcaggatgg tggcaaaacg caaatcgtcc gaactcttta tgatcaatgt 210180
gctgctggta accttgggtg tggcttatct gactgagctg gaaggtttgt ctatggcgtt 210240

gggcgcattc gttgccggca tgctgctttc ggaaacggaa taccgtttcc aagtcgaaga 210300
cgacatccgc ccgttccgcg atattttgct cggctttttc tttatcacgg tcggcatgaa 210360
gctggacatt caggcattga tcggcggctg gcggcaggta ttgatgctgt tggcaatgct 210420
gctggtgttg aaggcactgg ttgtgtttgc cattgccttc aaaatgaaac attcggtcgg 210480
cgacagcctc aaaacggctt tgtatctcgc gcagggcggc gagttcggct tcgtgatgct 210540
ggccattgcc gggcagcttg atatggtttc gccagaatgg gaacaggcgg cgacggcggc 210600
ggttctgctg tcgatgatta tcgcgccctt cctcttgggc ggcagcgatg cgctggtcgg 210660
gcgtttggtc aagtcaagct gggacatgaa gtcgctcgat ctgcacagta tgctggtaga 210720
aaccatgagc aagtccgacc atgtgctgat tgtcggcttc ggcaggggcg ggcagacggt 210780
cggacgcgtc cttgcccaag aggatattcc gtatttcgcg ctcgacttgg acattgcgcg 210840
ggtgcaggtt gccagaagtg cgggcgaacc ggtgtcgttc ggcgatgcga aacgcaggga 210900
agtattggaa gccgccggtc tgggacgggc gaaaatggtg gtggttacgc tcaacaatat 210960
gcacgaaacg caacacgttt tagacaatgt gctgtccatg tatcccaata tgcccgtata 211020
tgtgcgcgcc accaacgacg attatgtgaa aacgtttacc gatataggtg cggaagaagc 211080
cgtgtcggac accaaagaaa ccggactcgt gctggcaggc tatgcaatgt taggcaacgg 211140
cgcgtcgtat cggcacgtct atcagacgat ggcaaatatc cgccacagcc gttatgccgc 211200
gttggaggga ctgtttgtcg gtagtgatga tgaggcagga ttcggcgaaa acggcgaaac 211260
cgtccgtcac gcctttcctt tggctgcaga agcatacgcc gtcggcaaaa cagtcggcac 211320
gcttccgatg gcggcttacg gcatcaaact cttgttcgtc cgccgccgca ccggccggat 211380
tgaaaacccg gatgcctcgt ttacattgga aggcggtgac gtgttggtgg tcgcaggcaa 211440
aaaagaagaa attatctctt ttgaaaactg gagtttgcag ggaatataaa tgaaatgccg 211500
aaataaggct tgcgccattt ccggttattt ggtttaataa cgctttcgca aatcgcaagg 211560
gtgattagct cagttggtag agtgtctgcc ttacaagcag aatgtcggcg gttcgactcc 211620
gtcatcaccc accaagtttt ctttcattgt tgcaaacaat ggatgcgcgg tggtagctca 211680
gttggttaga gtaccggcct gtcacgccgg gggtcgcggg ttcgagcccc gtccgccgcg 211740
ccaagtttca aaatactgac tctgtcggta tttttatac acgggtgatt agctcagttg 211800
gtagagcgtc tgccttacaa gcagaatgtc ggcggttcga ctccgtcatc acccaccaag 211860
ttttctttca ttgttgcaaa caatggatgc gcggtggtag ctcagttggt tagagtaccg 211920
gcctgtcacg ccgggggtcg cgggttcgag ccccgtccgc cgcgccaaaa gttaaggaat 211980
accaacctcc ggttggtatt tttttgtttg tatgctttga aaaatgtttg tttccggatt 212040
ttgccattcc catccggttt tgcgctgtac gatgtgtttt agcgcggact tgctcaaaat 212100
cgcatgtgat tccggtattt gaggctttga ttagggatgc ggactttcaa tatattttct 212160
cagctacaac aacgaaggct tgatgtctgt cgggcaggta agggagattt ttgagcgttt 212220
cggcaaatat aatttggttc aaacggaata ccggcgtttt aaggcagata agacagaaaa 212280
ccgtaatcat aaggcaaatt cgatattcga atttctgcat attttagaaa agacctttta 212340
tagtggatta acaaaaacca gtacagcgtt gcctcgcctt agctcaaaga gaacgattct 212400
ctaaggtgct gaagcaccaa gtgaatcggt tccgtactat ttgtactgtc tgcggcttcg 212460
tcgccttgtc ctgatttttg ttaatccact ataaaaattc ttgccggatg ctgcaaacaa 212520
cgccggtttg cattcctgat ggcggtggtt ttcttagacg aacgcccgaa cacgcaggaa 212580
tggataggct tggggctggt tacggcgggc gtgttgacgc tggcactgaa acggtaaagc 212640
cgcaagaaat aaatgaaatg ccgtctaaaa aactgttttc agacggcatt ttcgtttctg 212700
tccatcctca gcactcgacc acgcgcacgg atacggggac ggctttttc cggagcgtgg 212760
cggcgagtcc ggcaagcgag gtttctttgt aggtcgcctt catatcccgg ccggtttgca 212820
gcatggttcg gatgacttcg tcgagcgaga ctttttgtc cgtgccgtct tccaaaagcg 212880
cgagcgtgcc gagtttgagg gctttttcgg cggcgatgcc gttgcgctcg atgcagggga 212940
tttgcaccag tccgccgacg gggtcgcaag tcagccccaa atggtgttcc atcgccattt 213000
cggcggcgtt ttccacttgt ttgggcgtgc cgccgatgac ttcggcgtat gcgcccgccg 213060
ccatcgaaca cgctacgccg acttcgccct gacagccgac atccgcaccg gaaatggagg 213120
cgttggtctt gtagaggatg ccgattgcgc ctgcggtgag caggaagttt tcgacgcgtt 213180
cctgtgtggc gtgcggattg aacttgcgga aatagtgcaa tacggcggga atgatgcctg 213240
ccgcgccgtt ggtcggtgcg gtaacgacgc gtccgccggc ggcgttttct tcgttgaccg 213300
ccatggcgta caccatcggc cagagctggg tgttgacgat ttcggtttcg cgcaggactt 213360
tgagcttggc ggcaagctgc ggggcgcggc ggcggacgtt caatccgctg ggcagttcgc 213420
cgtccgcacc caagccgcgt ttgatgcagc cttccataac ctcggcaacg gcagcggcgc 213480
ggcggcggat ttcggcttcg ccgcatccgg caagcgcggc ttcgtttgcc aacacgactt 213540
cggagatgtc gagccggttc agacggcatc gggcaagcag ttcggcgcaa ctggtatagg 213600
gatagggaac ggctttttcc gtttccgcct gccggtcaaa atcttcttcg gtaacgacaa 213660
agccgccgcc gaccgaataa taaacctgtt cattcaatac cgtgccgtct gaagcatagg 213720
cggtaaaacg caggctgttg ggtgtttgg gcagcacttg attgccgagt atgttcaggt 213780
cgcggtcggg gatgaagcgg atttcttgcc cgttgagccg gaggatgtgc tgcgtgcgga 213840
tgcgttcgag gcgttcggga atgccggcaa gcgggatgtc gtgcggcagg ctgccttcca 213900

aaccgagcat cagcgcgtca aatgtaccgt gtccgtatcc ggtcagtgcg agcgagccgt 213960
aaatgtcgat gacgatgcga acagcctgtg catccaaacc tgccgcaaag gcggcggctg 214020
ccttcatcgg gccgaccgta tgcgaactgg aaggcccgat accgattttg aaaatatcga 214080
aaatgctgat catattttgc tccgacggtt tttcagacgg cacaggttcc gtttgaccaa 214140
ccaaaaagga gacgcggcac gatgcccgtc tcctttttta aaacggcact tatgcgtcga 214200
tattttgggc aatcagcgcg ttgttttcga taaaggcacg gcgcggctcg acctcgtcgc 214260
ccatcagcgt aacgaacact tcgtcggcgg caatggcatc ttcgatgcgc actttcaaca 214320
ggcggcgcac ggcgggatcc atcgtggttt cccacagctg ctcggggttc atctcgccca 214380
agcctttgta tcgttggatg gacatacctt tttgggcaac gctcatcaag atgtccaaag 214440
cggtttcaaa gctgtccgcg tcgtacccgt tttcgccttt gtaaagcttg gcaccctcgc 214500
cgaccatgcc tttgagcgcg gcggcggttt gggtgagggt ttggtaggct ttgctgttga 214560
ggaacttggg ttcgatgtag ctgaccatga cgttgccgtg cagcttgcgc gtgattttga 214620
tgaaccggtg tccttcatga ccttcgatgc gttcgagggc gacttctttt tcgtcaagca 214680
gaccggaaag ttcggcaacg gctttatcgg cgttttcaga cgacgtcaaa tcaatgggcg 214740
acgcgtgtag catggcgcgc aggacgagtt cgtctacgaa gcggctttcc tgttcgatga 214800
cggtttttgc caacaggaat tgtttggcgg tgtcggcaag ttctgcgcct tcgatggtgc 214860
ggccgtctga aatgattttg gctttttcca aggcaagacc gagcagccat tggtcttttt 214920
ccaactcgtc cttgaggtaa cgttcctgtt tgccgtattt cgctttatac aaaggcggct 214980
gggcgatata gatgtagccg cgctcgacca gctcgggcat ttggcggtag aagaaggtca 215040
ggagcagggt gcggatgtgc gcgccgtcca cgtcggcatc ggtcatgatg atgatgcggt 215100
ggtaacgcag tttttcggca ttgaattctt ctttgccgat gcccgcgccc aaagcggtaa 215160
tcagcgtggc gacttcttgg ctggccagca tttttcaaa acgtgctttt tcgacgttca 215220
aaattttacc tttgagcggc aaaatcgctt ggaatttgcg gtcgcggcct tgcatggcgg 215280
aaccgcctgc ggagtcgccc tcgacgaggt agagttcgga cagggcaggg tctttttctt 215340
ggcagtcggc gagtttgccg ggcagtccca agccgtccat cacgcctttg cggcgggtga 215400
tttcgcgtgc tttgcgggcg gcttcgcgcg cgcgggcggc atcgacgatt ttgccggtga 215460
tgattttggc ttcgttcgga ttttcttcga ggaagtcggt cagggcttgg ctgatgactt 215520
cgttgacaac ggggccgatt tcgccggaaa ccagtttgtc tttggtttgg gacgagaatt 215580
tggggtcggg cagtttgacg gacaacacgc aggtcaaacc ctcgcgcata tcgtcgcctg 215640
cggtttccac tttggctttt ttggcgactt cgttggcttc gatatagttg ttgatggtgc 215700
gggtcatcac ttggcgcagt gcggtcaggt gagtaccgcc atcacgttgc gggatgttgt 215760
tggtgaaaca ctgcacgctt tcttgatagc tgtcattcca ttgcatcgcg cattcgacgc 215820
tcatgccgtc tttttcgccg aacgcgtaga agatttttttc gtgcaacggc gtttttttgc 215880
ggttcatgta ttgcacgaaa cccgccacgc cgccggaaag ggcgaagctt tcgtgtttgc 215940
cgtcgcgctc gtcggtcaat tcgatgtcca cgccgttgtt caggaaggaa agttcgcgga 216000
tgcgtttggc aaggatgtcg aagctgtatt cgacgttgcc gaaggtttcc gtactggcga 216060
ggaagcgcac ggtcgtgcct tttttatcgg aatcgccgac aattttcagc ggctcttcgg 216120
tttcgccgcg cacgaagcgg acgaagtgtt ctttgccgtc gcggtagatg gtcagcgtta 216180
cccagtcgga cagcgcgttg acgacggaca cgcccacgcc gtgcaggccg ccggagattt 216240
tgtagctgtt gttgtcgaat ttaccgcccg cgtgcaatac ggtcatgatg acttcggcgg 216300
cggagcgtcc ttctttcggg tggatgccgg tgggcatacc gcgcccgttg tcggcgacgc 216360
tgacggaatg gtcggcgtgt atcgttaccg tgattttgtc gcaatgtccg gcgagtgctt 216420
cgtcaatggc gttgtccaat acttcgaaca ccatgtggtg cagaccgctg ccgtcctgcg 216480
tgtcgccgat gtacatgccg gggcgtttgc gtaccgcttc caagccttcg agcacctgaa 216540
tgctgtcggc gccgtattct tcgtgttttt gttcagtcat attttttgcc ggattttgaa 216600
aagattttgc gatgccgcca aaacaagtcc gcaccttgta gaaaaagcgg gcgggacgac 216660
atatataatt gtgtattata gccgattttg ccgcctaatt cagcgttatc cgcatcagtg 216720
tgccgccggg aaaagatgaa acggtacgtt tgcctccggc atcaggtcgg ggattgtccc 216780
gtaaagtggc aaaagcgttt ttttgccact aaaatctaca ccctatactt ttcggacagg 216840
ggcgcggaaa tggaaatatg gaatatgttg gacacttggc tcggtgccgt cccgatacgt 216900
gcggaggcgg tcgaatccgt ggcggcggtt gcggctttgc tgctggcgcg cgcccttctg 216960
ttgaatatcc acttcaaacg gcatccggat ttcggcatcg aaagcaagcg gcggtttttg 217020
gttgccagcc gcaatataac gctgcttttg gtgctgtttt cgctggcatt tatctggtcg 217080
gcgcaaatcc aaacgctggc tttgtcgatg tttgcggtgg cggcggcggt cgtcgtggcg 217140
acgaaggaac tgattatgtg tctgtcgggc agtattttaa ggtctgccac ccagcaatac 217200
tcggtcggcg actatatcga aatcaacggc ctgcgcgggc gcgtggtcga catcaacctg 217260
ttgaacacgc tgatgatgca ggtcggtccg aacccccttgg tcggacagct tgcgggaacc 217320
accgtttctt tccccaacag cctgttgttg agccaccccg tgcgccgcga caatattttg 217380
ggcgactatg tcatccatac ggtcgaaatc cccgttccca tccatttgga ttcggatgaa 217440
gccgtatgcc gtctgaaagc cgtactcgag cccttgtgcg cgccctacat ccccgccatc 217500
caacggcatt tggaaaacgt gcaggcggaa aaactgttta tcacgcccgc cgccagaccg 217560

cgcgttaccc gcgtgccgta cgatgacaag gcataccgca tcatcgtccg cttcgcttcc 217620
cccgtttcaa agcggctgga aatccaacag gcggttatgg acgaattttt gcgcgtacaa 217680
taccgcctgt taaatcaccc cgccggctcc gaaacacttt aactttcccc gaccgacccc 217740
atttccggct tcagacggca tattgccgat atgctgtctg aaacacaaca cgcaaaggaa 217800
acccatctta tgactgacaa cgcactgctc catttgggcg aagaaccccg ttttgatcaa 217860
atcaaaaccg aagacatcaa acccgccctg caaaccgcca tcgccgaagc gcgcgaacaa 217920
atcgccgcca tcaaagccca aacgcacacc ggctgggcaa acactgtcga acccctgacc 217980
ggcatcaccg aacgcgtcgg caggatttgg ggcgtggtgt cgcacctcaa ctccgtcgcc 218040
gacacgcccg aactgcgcgc cgtctataac gaactgatgc ccgaaatcac cgtcttcttc 218100
accgaaatcg gacaagacat cgagctgtac aaccgcttca aaaccatcaa aaattccccc 218160
gaattcgaca ccctctcccc cgcacaaaaa accaaactca accacgatct gcgcgatttc 218220
gtcctcagcg gcgcggaact gccgcccgaa cagcaggcag aactggcaaa actgcaaacc 218280
gaaggcgcgc aactttccgc caaattctcc caaaacgtcc tagacgcgac cgacgcgttc 218340
ggcatttact ttgacgatgc cgcaccgctt gccggcattc ccgaagacgc gctcgccatg 218400
tttgccgccg ccgcgcaaag cgaaagcaaa acaggctaca aaatcggctt gcagattcca 218460
cactacctcg ccgtcatcca atacgccgac aaccgcgaac tgcgcgaaca aatctaccgc 218520
gcctacgtta cccgcgccag cgaactttca gacgacggca aattcgacaa caccgccaac 218580
atcgaccgca cgctcgcaaa cgccctgcaa accgccaaac tgctcggctt caaaaactac 218640
gccgaattgt cgctggcaac caaaatggcg gacacgcccg aacaagtttt aaacttcctg 218700
cacgacctcg cccgccgcgc caaaccctac gccgaaaaag acctcgccga agtcaaagcc 218760
ttcgcccgcg aaagcctgaa cctcgccgat ttgcaaccgt gggacttggg ctacgccagc 218820
gaaaaactgc gcgaagccaa atacgcgttc agcgaaaccg aagtcaaaaa atacttcccc 218880
gtcggcaaag tattaaacgg actgttcgcc caaatcaaaa aactctacgg catcggattt 218940
accgaaaaaa ccgtccccgt ctggcacaaa gacgtgcgct attttgaatt gcaacaaaac 219000
ggcgaaacca taggcggcgt ttatatggat ttgtacgcac gcgaaggcaa acgcggcggc 219060
gcgtggatga acgactacaa aggccgccgc cgtttttcag acggcacgct gcaactgccc 219120
accgcctacc tcgtctgcaa cttcgcccca cccgtcggcg gcagggaagc ccgcctgagc 219180
cacgacgaaa tcctcatcct cttccacgaa accggacacg ggctgcacca cctgcttacc 219240
caagtggacg aactgggcgt atccggcatc aacggcgtag aatgggacgc ggtcgaactg 219300
cccagccagt ttatggaaaa tttcgtttgg gaatacaatg tcttggcaca aatgtcagcc 219360
cacgaagaaa ccggcgttcc cctgccgaaa gaactcttcg acaaaatgct cgccgccaaa 219420
aacttccaac gcggcatgtt cctcgtccgg caaatggagt tcgccctctt tgatatgatg 219480
atttacagcg aagacgacga aggccgtctg aaaaactggc aacaggtttt agacagcgtg 219540
cgcaaaaaag tcgccgtcat ccagccgccc gaatacaacc gcttcgcctt gagcttcggc 219600
cacatcttcg caggcggcta ttccgcaggc tattacagct acgcgtgggc ggaagtattg 219660
agcgcggacg catacgccgc ctttgaagaa agcgacgatg tcgccgccac aggcaaacgc 219720
ttttggcagg aaatcctcgc cgtcggcgga tcgcgcagcg cggcagaatc cttcaaagcc 219780
ttccgcggcc gcgaaccgag catagacgca ctcttgcgcc acagcggttt cgacaacgcg 219840
gtctgacggc agggttgaag taaaaaatat ggcggattcg atagaaaaac atccgcaccg 219900
tcattcccgc gcaggcggga atccagaccg gtcggtgcag aaacttatcg ggaaaaacgg 219960
tttctttaga ttttacgttc tagattccca ctttcgtggg aatgacgcgg aaaagttgct 220020
gtgattccgg ataaattttc gcaacgttta atttccgttt tacccgataa atgcccgcaa 220080
tctcaaatcc cgtcattccc caaaaacaaa aaaatcaaaa acagaaatcc catcattccc 220140
gcgcaggcgg gaatccaggt ctgtcggtgc ggaaacttat cggataaaac ggtttcttta 220200
gattttacgt tctagattcc cgctttcgcg ggaatgacgg aatatttttg aatttgataa 220260
aaatgccgtc tgaaacggtc aaacaacgct tcagacggca ttttatagtg gattaacaaa 220320
aatcaggaca aggcgacgaa gccgcagaca gtacaaatag tacggaaccg attcacttgg 220380
tgcttcagca ccttagagaa tcgttctctt tgagccaagg cgaggcaacg acgtactggt 220440
ttttgttaat ccactatatt ttccgacatc attgaatcaa acccaaatgc gacaagagcg 220500
tccatgtgcc gatggcaatc aacaccaaac ctccggcaaa ttccgcacac ctgccgaaca 220560
atacgcccaa agcccttccc gccgtcagcc cgaccgccac catcaccgtc gtcgccatac 220620
cgatgattgc ggcggcaaag gcgatgttta cctccataaa cgccaagccc accccgacta 220680
tcatggaatc aatactggtt ccaaaagcag tcaaaaccgt catccatagg ctttcccgtt 220740
tgctttcgcg cacatcttcc gcctcgccgg acagcccttc gcgcatcatt ttcagaccca 220800
gcccgcccag caggacgaaa gccacccaat ggtcccattc gctgataaac ggcttggcat 220860
aaaaaccgcc tacccagcct gccagcggcg tgagcgcttc aaccgtgccg aacaccaaag 220920
ccgttgccgc aattttgcgc ggaggcattc tgaccgccgc accctttgcc aatgcgacgg 220980
caaacgcatc catcgacatc cccagagcaa tcaagagcaa agcataaaaa cccataccgc 221040
acccgtcctc aaaaagggcg gattatagca aaagcaaaaa aatgcaaaaa tgccgcacga 221100
aaacccgcat cccgtcattc ccgcaaaaac aaaaaatcaa aaacagaaat cccgtcattc 221160
ccgcgcaggc gggaatccag agttgtcggt gcggaaactt atcggataaa acggtttctc 221220

caaccccgag tccttgattc ccactttcgt gggaatgacg ggatattttg cgtttaataa 221280
aaaacgcccg ctgaaacggc ggggcgggat gggggaatgc cgtctgaaac ggtcggacaa 221340
tgtttcagac ggcattttta tgcccggtta tttccgatag cggacggcgc gggacaggat 221400
ttcttcaatt tccatccaca taatgccccc ttacagcaaa ccagcctgac ccagtgcggg 221460
atcggtcgcg cgggcggctt gggcatcttc gacagtcagt ccaagggctt tggccacgcc 221520
ttcgccgtat gccgggtcgc aacggtagca gttgcggata tggcggtatt tgatgaagtc 221580
gggcgcgtcg cccattgcgg cggcggtgtt gccgaacaat gcctgtttct gcgcgtcgtt 221640
catcaggttg aacagggcgc gcggttggct gaaatagtcg tcatcgtctt ggcggtagtc 221700
ccagtgtgcc gcgtcgccgt tgattttcaa aggcggttcg gcgaagtcgg gttgttgctg 221760
ccattggccg aagctgttgg gttcgtagtg cggcaggctg ccgtagttgc cgtcggcgcg 221820
gccttgcccg tcgcgctggt tgctgtgaac agggcaacgc ggacgattga cgggaatttg 221880
gcggaagttt acgcccaaac ggtagcgttg tgcgtcggcg taattgaaca aacgcgcttg 221940
cagcatttta tctgggctgg cgccgacacc gggaacgagg ttgctcggtg cgaaggcgga 222000
ttgttccaca tcggcgaaga agttttcggg attgcggttc aactcgaatt cgcccacttc 222060
aatcagcgga tagtcttttt tcggccaaac tttggtcaag tcaaacggat gataaggtac 222120
tttttccgcg tctgcttcag gcatgacttg gatgtacatc gtccatttcg gaaactcgcc 222180
gcgttcgatg gcttcgtata agtcgcgctg atggctttcg cggtcgtcgg cgatgatttt 222240
ggcggcttct tcgttggtca ggtttttaat gccttgttgg gtgcggaaat ggaatttcac 222300
ccaaaaaacgc tcgcctgctt cgttccagaa gctgtaggta tgcgaaccga agccgtgcat 222360
atggcggtag ccggcgggga tgccgcggtc gctcatcacg atggtaactt ggtgcagtgc 222420
ttcgggcagc agcgtccaga agtcccagtt gtttgtggca gagcgcatat tggtgcgcgg 222480
gtcgcgtttg acggctttgt tcaggtcggg gaacttacgc gggtcgcgca ggaagaacac 222540
gggcgtgttg ttgccgacca catcccagtt gccttcttcg gtataaaatt tcaaggcaaa 222600
accgcggatg tcgcgttctg catcggctgc gccgcgttcg cctgccacgg tggtgaaacg 222660
ggcgaacatc tcggtttttt tgccgacttc gctgaagatt ttggcgcggg tgtatttggt 222720
gatgtcgtgc gttacggtaa acgtaccgaa cgcgcccgaa cctttggcgt gcatacggcg 222780
ttcggggatg acttcgcgca cgaagtcggc gagtttttca ttcagccaca aatcctgcgc 222840
cagcagaggg ccgcgaggac cggcggtcag gctgttttga ttgtcggcaa caggcgcgcc 222900
gttgttcatg gtcagatggg ttacagggca tttggaggta gtcatcgctc ttgttccttt 222960
tctcaggttg gtcaaatggg ggtaaacggc ttacagtacg atttggcgga aagcgtattc 223020
gtaaccggtt tcttgattgc aataaatttc ttgaatcgac attttatttc ccttttgtaa 223080
aaactatgga tgcgactata cgccaagatt ttcgctatta aaactatgaa atcgatttaa 223140
tattattata agcaatcggt tcttgatttt cgtttgtttt ttgttatcga acggaatccg 223200
aacccgctca ttaaaaccat ttataatgca atgacgcttt gcggcatttt ttgcgccgac 223260
aggctgaaaa taacaatttt ccccacatta tcatgacctt actcggaata aagctcaaac 223320
agacccagca gctcaaccag cggctgcaac aatctttgcg cgtattgcag atgtcgggta 223380
tcgaacttga acgcgaggtc gaaaactggc tgtcggacaa ccccctgctc gaacgcaaag 223440
acacggatga attttccgat gccgagttca gccattacac tgcgcctgcc cgtcaaatcg 223500
gcggagacga aggcgaagat atgctgtcca acatcgccgg cgagcaggat ttcaagcaat 223560
acctgcacgc gcaagtatgc gaacacccgc tttccgacca agaagccgcc tgtgtccaca 223620
tccttatcga tttccttgac gagcagggtt atctgaccga cagcatcgaa gacatcctcg 223680
accatacgcc cttagagtgg atgttggatg aagcaatgct gcaacacgcg ctgaccgcat 223740
tgaaaaaatt cgacccggca ggcgtggccg ccgccgattt gaacgaatcg ctgatactgc 223800
agatagaaag attgggcgaa tgtgctgcca aacccgccgc cctgcatatc gtccgaaacg 223860
ccctcgacag cattgacggc aaccgcagcc aaaccctcgc acgaataaaa aaacacctgc 223920
cccaaaccga cagcggcaca ctcgaagccg cactcgacct cattgcttcg ctcaatccct 223980
ttcccgccgc cggttttgcc tcgtccacgc ccacgccgta ttctgacgag gcgctcgcca 224040
acctgctggc tttccgcggc atggaggttt ctcgccgcac cattgccaaa tacagagaat 224100
cctttgagat tccggcagca cacaaacgca aaaccgcaga ataattgccg aataatctta 224160
taaagacaac aaaccaaaag ccggcatttc tgcgaaagcg ggaatgccga atccgtccgc 224220
gcggaaacct gcatcccgtc attcccgcga aagagggaat ctagaaacgc aaagctgcaa 224280
gagtttatcg gaaatgaccg aaactcaacg aacctggatt cccgctttcg cgggaatgac 224340
gggggtttgg cgggaatgac gagggtttgg gatttctgtt tttgaatttc tgtttttgtg 224400
agaatggcaa gattttcggt tcttgtatgg ataacgagat tttagatggc gggaatttgt 224460
cgggaaaaca gcaatctgag acctttgcaa aaataatctg ttaacgaaat ttgacgcata 224520
aaaatgcgcc aaaaaatttt caattgccta aaaccttcct aatattgagc aaaaagtagg 224580
agaaatcaga aaagttttgc attttgaaaa tgagattgag cataaaattt tagtaaccta 224640
tgttattgca aaggtctcaa tctttaccgt cattccccacg aaagtgggaa tctagaaacg 224700
caaagttgca agaatttatc ggaaatgacc gaaactcaac gaacctggat tcccgctttc 224760
gcgggaatga cgagggtttg ggatttctgt ttttgaattt ctgtttttgt gagaatggca 224820
agattttcgg ttcttgtatg gataacgaga ttttagatgg cgggaatttg tcaggaaaac 224880

agcaaccctc cgccgtcatt cccacgaaag tgggaatcta gaaacgcaaa gttgcaagaa 224940
tttatcggaa atgaccgaaa ctaaacgaac ctgaattccc gctttcgagg gaatgacggg 225000
ggtgtggcgg gaatgacggg ggtttatcag aaatgaccga aactcaaaag cgggcagcct 225060
tgtttacgcc ttcaaaatat cgagcaattt caaatcgact ttttcggcat cgaatttatc 225120
tttggcaatc gcataacttg cattccccat caggcggacg gcttccctgt tttcgataaa 225180
ataaatcatt ttttcggcca agatgcgggg attccaaggc tcgatcagga agccgttgac 225240
cttgtcggcg accgtttccc tgcatccggg gacatccgtc gtaatcactg ccctgccgac 225300
ggccattgcc tcctgagtgc ttcggggaac gccttcccta taataagacg gcaatacgaa 225360
tatatgatgt tcttttatca cttcggaaac attgttcaca aaaccgggga aacggataat 225420
atcgcgggcg gcaagccgtt ccaaatcgcc cccccccccg cgtgatttgt cgattgcgcc 225480
caaagcggta aaaaccgtat cggggtattt gtccttaacc tgttccgccg cccgaataaa 225540
atcatcaatc ccctttcttt tcagaaatct gccgataaag aggaatttta cgggttcttt 225600
ttcatcggga atatccgcct cggaataagg atattgccgc aaatccagac cgattccgcc 225660
caaaatatgg atgtttttta ttttgatgcc gtatttgtcc gtcagttcgt ctttgtcgtc 225720
ggggtttaat acaatcaggc tttccaacat cggcagggca atgcggtata aggcaatcaa 225780
aatccccttt atgatttttg tttttaacgg tatgccttcc ggctgcgggg taaatgcgaa 225840
tcccaaacct tccagcatcc cgacgattct gggcacgcct gccagttttg cggcaaaagt 225900
gccgaaaatc acgggttttg cgaaataagg gaaaaccaaa tccggcgata ttttttttgag 225960
ttctttaaag atgaggaagg tggattttat atccgaaaac gggttcagcc cgctgcggtt 226020
tgaacggtag gtaacgggtg taacccccat ttccctgata atatccaatt cattgtcgga 226080
aaactccgat acaaaggcat acacctgatg gtttttgccg attaattttt taatgacggg 226140
ggcgcggaaa ccgtaaatgc tggatgcgac tgttgtgata aaaacgattt tcataaggcg 226200
gacaccttga atatggattg gaaatgcggt ctgctacggc agggtttcat cctgtaaccc 226260
agcaaggctt gggtttgcct gcgtattata gtggattaac aaaaaccggt acggcgttgc 226320
cccgccttag ctcaaagaga acgattctct aaggtgctga agcaccaagt gaatcggttc 226380
cgtactattt gtactgtctg cggctcgccg ccttgtcctg atttttgtta atcactataa 226440
aaatgccgtc tgaaacggtt tcagacggca tttcgatgtc ggcggcggct ttgcggaatc 226500
agcctttgaa gcgtttgaag accagcgtgc cgttggtgcc gccgaagccg aaggagttgg 226560
aaatggcaac gtcgatttcc gcgtcgcgcg cttcgttggc gcagtagtcc aaatcgcagc 226620
cggcttcaac gtcttgttca aaaatgttga tggtcggcgg gattttgccg tcgtgtatcg 226680
ccaaaatgct gtacacggcc tccacgccgc ccgccgcgcc gagcaggtgg ccggtcatgg 226740
atttggtcga gctgacgacg gttttgtagg cgtgttcgcc gaacgcgcgt ttgagggctt 226800
tggtttcgtt ggcatcgccc aagggggtgg acgtgccgtg cgcgttgacg taatccacgt 226860
cttcgggatt gatgccggca tctttcagcg cgcgggtaac ggcaagggcg gggccttctt 226920
cgttcggcgc ggtgatatgg taagcatcgg aactcatgcc gaagccgacg atttcggcgt 226980
agattttcgc gccgcgtttt ttggcgtgtt ccaattcttc caacaccaat atgcccgcgc 227040
cttcgccgat aacgaagccg tcgcggcctt tgtcccacgg acgggaagcg gtggcggggt 227100
cgtcgttgcg ggtggagagg gctttcatcg cggcaaaacc gcccacgccc aaagtgctga 227160
ttgcgccttc cgcgccgccg gcaaccatta tgtccgcgtc gccgtattta atcatacgga 227220
gggaatcgcc gatggcgtgc gcgccggtgg tgcaggcgga aaccatcccg tagctcgggc 227280
cgcggtagcc tttgaggatg gtaacgtgtc cggaaatcag attaatcaga gaaccgggga 227340
taaagaaagg gttgatttg cgcgcgccgc cttcgattac ggctttgccg gtgacctcga 227400
tgccgggcag tccgccgatg ccggaaccga tgttcacgcc gatgcggtct ttgtcgaggt 227460
tttccacatc gtccaaaccc gaatcggcga ttgcctgcaa tgcggcggca atgccgtagt 227520
ggatgaatac gtccatccgg cgcgcttctt tcgcgctgat gtattgtccg atgtcgaaac 227580
cgcgcacctc gccggcgaca cggctgttga tgtcggatgt gtcaaagcgg gtaatcgcgc 227640
cgatgccgct tttgccggtg agcagggtgt cccaagcctc tgcgacagtg ttgccgacag 227700
gggaaacctg acctaagcct gtaatgacta ctcttctctg actcatgata acctcgctgt 227760
tggttgtcgg aatgggggca tatgcggctg tcgtgcagat gccgtctgta atttgcggca 227820
ggggttcaaa cagtttgcca tataagggaa aagcctctat tgcgcggtgc agcagaggct 227880
gttgtgtcgg gcgacgaccg gttagccgtt gtgggcattg atgtagtcga tagccagttg 227940
gacggtggtg attttttcgg catcttcgtc ggggatttcg cagccgaatg cttcttccaa 228000
agccataacc agctccacgg tgtccaaaga atccgcgccc aagtcgtctt ggaaggaaga 228060
ttcgtttttc acgtcggctt cgtttacgcc cagttgttca gcaacaattt ttttaacttg 228120
ttgttcgatg tttgacatat cagtcgttcc tttatgcctt gcggcaggtt gtttaaggga 228180
aataaatcgg tggtattgta ccgactttta atagagtttt ctatctaatg actattatat 228240
caatatttgc cgatttgtac atttttgggt gcggcgggtt ttgtcgttca agtttgacct 228300
gtgtgccgta tgtttggcgg gatttcggtt aaaatggcgg catttccatc tgaagcagaa 228360
agccctgtca tgtatccact tgcccgtcgc atcctgtttg cactcgatgc cgaaaaagcc 228420
caccacttca cgctcgacgc gctctacacg gtttataaat tgggtttgat tcctgtaacc 228480
gacaaccgta ccaaacctgt aaaattgatg ggtatggatt tgcccaaccc tgtcggactt 228540

gccgccggac tcgacaaaaa cggcgaatac atcgacgcat tgggcgcgct cggctttggt 228600
ttcatcgaaa tcggcacggt aacgcccaac ccgcagcccg gcaacccgca gccgcgcctc 228660
tttcgcgttc ccgaacacca aggcatcatc aaccgcatgg gtttcaacaa ccacggtatc 228720
gacaccatga tacgcaacat cgaaaaaagt aaattcagtg gcgtattggg catcaacatc 228780
ggtaaaaacg cggttacacc catcgaaaac gctgccgatg attatttaat ctgccttgaa 228840
aaagcctacg cacacgcaag ttacattacc gtcaatattt cctcgcccaa cactaaaaac 228900
ctccgcgcgc tgcaaggtgg cgacgagttg agcgcattgc ttgaggcttt gaaaaacaaa 228960
caggcacagc ttgcctctgt acacgggaaa tacgtcccgc tcgccgtcaa aatcgccccc 229020
gatttggatg aagcacaaat cgaagacatc gcccacgttg tcaaatccgt cgaaatggac 229080
ggcatcatcg ctaccaatac caccatcgac aaatcaagtc tcggcagcca tccgctcgca 229140
ggcgagcagg gcggtttgag cgggctgccc gttcatgaaa aaagtaatcg ggtgttgaag 229200
ctgttggcag accacataga cggcaagctg ccgattatcg gcgtaggcgg cattatggaa 229260
ggcgaggact cggcagataa aatccgcttg ggcgcgaccg ccgtccaagt gtacagcgga 229320
ttgatataca aaggtccggc attggtcaaa gaatgtttga aggctttggc gcgatgacgc 229380
gatccgccca aaatgccgtc tgaacgcacg ttttgccgtt cagacggcat tttcatttcc 229440
tttttccgcc tgacgcccct tgaaaatccc ttacgcgccg ccctgtttga aataaggcaa 229500
accgatgcgt gaacacggag caggcaatcg gagtaaaaaa tgaaccttga tttaaccgcg 229560
caaaaagtcc gtctttcttg gaaggatatt ctgtgggggt atgggaataa atacttgggt 229620
tgggctgatg tggcagctta tgcccgaaaa atgacgcttt cagatcatga tgaacgtgtg 229680
ttcaaactat ctttaatcaa caaatccaat attcttgaat taaagcctgt tctggaagat 229740
ttggcttcgg aaatgaggga ttattcccct aaaaattggc tgtacgtcct cttaagcgat 229800
gtattccata gaaaagaaga atttgaggat cctttggggg aagttgaaaa aatttatgca 229860
gattttgatt atccggaaga aatagaatca tttgtcaggt atatgccgcc caaagacggt 229920
tatattcctt ctgcccacac ctatgaagaa aatattgccc ggttatattc tcactgggaa 229980
cactatttga acaacggcgg agggcagggt taaaaccggc aatccgatgc cgtctgaagc 230040
attatccggc cttcagacgg cattttgttt tccgacagtt tataaactgt cgttgtttct 230100
tgacagaaac aacgacctta tttgaaacga ttggaggaca tgattatggg tttttggaat 230160
ggtgtggcaa aagcagcaaa agcagtggga gagggaatga ttgaagccgg caatgagcat 230220
aaggcgttga aaatggaata tgcggagaaa tcaagtgagg agctgcatga aatcgtcaag 230280
agtgatggtt tttttaaaaa ttccacacgg gagaaaagtg cggcttatgc tattttaaaa 230340
gagcgtggcg aggtgtgaac aggaaacggc ggcatttgcc gctgtttttt attggtaggc 230400
atccgtccga atatcggggc aaggtttcag acgacatcga aggttgctat gatatagtgg 230460
cttgacttta aaccggtacg gcatcccctc gccttgtcct gatttaaagt taatccacta 230520
tctcattccc gtcatccttc caaacggaat ccgaaatgtc cgacaaccgc ctcgacaccg 230580
cccgccgcca ttccctcttc ctcgcccgcc agctcgacaa cggcaaactc aagcccgaaa 230640
tattcctgcc tatgctcgac aaggttttga ccgaagcgga tttccaagcc tttgccgact 230700
ggggcgaaat ccgcgcggaa gaaaacgagg aagaattggc gcggcagttg cgcgagttgc 230760
gccgttatgt ggtgtcgcag attatcgtgc gcgatatcaa ccgtatcagc gatttgaacg 230820
aagtaacccg cacgattacg ctgtttgccg attttgccgt caataccgcg ctggattttg 230880
cctacgccta ttatcgggac atgtacggca cgccgatcgg gcgttatacc aaatcgccgc 230940
agcatttgag cgtggtggcg atgggcaagg cgggcggcta tgagttgaac gtgtcttccg 231000
acatcgattt gattttcgtc tatcccgaat caggcgacac cgacggcagg cgcgaacggg 231060
gcaatcagga atttttcacc aaagtcgggc agaaactgat tgcgctgctg aacgacatta 231120
ccgccgatgg gcaggtgttc cgcgtcgata tgcggctgcg gccggacggc gattcgggcg 231180
cgttggtatt gagcgaaacc gcgctggagc aatatttgat tacacagggg cgagaatggg 231240
aacgctacgc gtggtgcaaa ggtcgcgtgg ttacgccgta tccgaacgac atcaaagcac 231300
tggtgcgccc ctttgtgttc cgcaaatatc tggattacgg cgcgtatgag gcgatgcgta 231360
agctgcaccg ccaaatcagc agcgaagtca gcaaaaaagg catggcggac aacatcaaac 231420
tcggcgcggg cggcatccgc gaagtcgaat ttatcgccca gattttccag atgatacgcg 231480
gcggacaaat gcgcgcgctg caactgaaag gcacgcagga aacgctgaag aagcttgccg 231540
agctgggcat catgctgtct gaacacgtcg aaaccctgct tgccgcctac cgcttcctgc 231600
gcgatgttga acaccgcctg caatactggg atgaccagca aacccaaacc ctgccgacct 231660
cgcccgaaca gcggcaactg ctcgccgaaa gcatgggttt cgacagttat tccgcttttt 231720
cagacggtct caatgttcat cggaacaaag tcaatcagtt gttcaacgaa attttgagcg 231780
aacccgaaga gcaaacgcaa gacaacagcg aatggcaatg ggcatggcag gacaaacccg 231840
acgaagaagg gcggcgatgc cgtctgaagg cgcacgggtt cgatgccgaa accgtcgccg 231900
caaggctcga ccaaatccgc cacggccata aataccgcca tctttccgca cacgcccagc 231960
cgcgtttcga tgcggttgtg ccgctgttcg tacaggcggc ggcagcgcaa agcaacccga 232020
ccgatacatt gatgcggctg ttggattttc tcgaaaacat cagccgccga tccgcctatc 232080
tcgccttcct caacgaacat ccgcaaacct tggcgcaact ggcgcagatt atgggccaaa 232140
gttcttgggt ggcggcgtat ctgaacaaat atccgatttt gttggacgaa ctcatcagcg 232200

cgcagctttt ggataccgcg tttgattggc aggcgctcgc cgccgccctt tcagacgacc 232260
tcaaagcctg cggcggcgat actgaagcgc aaatggacac cctgcgccgc ttccagcacg 232320
cccaagtctt ccgtctcgcc gtccaagacc tcgccggact gtggacggta gaatccctct 232380
ccgaccaact ctccgccctc gccgacacca tcctcgccgc cgccctgctg tgcgcatggg 232440
cggacatgcc caaaaaacac cgcgacacac cgcaattcgc cgtcgtcggc tacggcaaac 232500
tcggcggtaa agaactcggc tacgcctccg acctcgacct cgtctatctc tacgacgacc 232560
cccaccccga cgcaggcgac gtgtacagcc gcctcgcccg ccgcctgacc aactggcttt 232620
ccgccgccac tggcgcaggc agcctctacg aaaccgacct gcgcctgcgc cctaatggcg 232680
acgccggttt cctcgcccac agcatcgccg cctttgaaaa ataccagcgc gaaaacgcct 232740
ggacgtggga acaccaatcc cttacccgcg cccgcttcat ctgcggcacg tccgaaattc 232800
agacggcctt cgaccgcatc cgcaccgaaa tcctcaccgc cgaacgcgac caaaccgcct 232860
tggcaggcga aatcatcgaa atgcgcgaaa aaatgttccc cacccacccg cctgccgaca 232920
gcaacgtcaa atacgcgcgc ggtggcgtgg tcgatgtcga atttatcgtc caatatctga 232980
tacttgccca tgcccgccag tatccgcaac tcttggacaa ctacggcaac atcgccctct 233040
taaacatctc cgccgactgc ggtttgattg acaaaaccct cgccggacaa agccgcaccg 233100
cctatcgctt ctaccgccgg cagcagcaca acaccaaact gcgcgacgcg gcaaaaaccg 233160
aagtaaccgg cgaactgttg gcacattacg gcaatgtcag gaaattgtgg cgggaagtgt 233220
tcggcgaaga agcggcaacc gtctgaacaa aaaatgccgt ctgaagcctg acaatctggg 233280
tttcagacgg tattttcgta ccgtgccgtt ttaaggttgc ggcagagcta aagcgattta 233340
tcgggaatgg ctgaaaccca aaaaccggat tcctctttcg cgggaatgac gggatttcag 233400
gcttctgttt ttgtgggaat gatgggattt tttatccaag caaaaatcaa aacaaacaaa 233460
taagaaccgt ttaaaacccc gccgtttcca ttaaaatagc gcattctact ttttagacgg 233520
ccttggattc ggatttcaag tgcaacacta gtgtattagt ggttggaaca gattcaagaa 233580
taaaacactt ggcgtttcgt agccaagtgt ttttcttggt cggtggttca actcatcttg 233640
aaccctgcgt atctcccgat cactgatgtt acggaaatcg gtttgtttgg ggaagtattg 233700
ccggatgagt ccgttggtgt tctcattcag ccctttctcc caagaatggt aagggcgaca 233760
aaaataagtc tccgctttca atgctttggt tattttggtg tgttggtaga actctttgcc 233820
gttatccatg gtaatggtgt gcaccctgtc tttatgtgcc tttaatgccc taacagctgc 233880
ccgggcagtg tcttcggctt tgaggctatc caatttgcag atgatggtgt agcgggtaac 233940
gcgttcgacc aaggtcaata atgcgctttt ctgtcctttg ccgacaatgg tgtcggcttc 234000
ccaatcgccg atacgggatt tctggtcgac gatagcgggt cggttttcta tgccgacacg 234060
gttgggtact ttgcctctgg tccatgtgct gccgtagcgt ttgcggtagg gtttgctgca 234120
tattctgaga tgttgccaca acgtgctgcc gttgcttttg tcttggcgaa ggtagcggta 234180
aatggtgctg tggtggagcg tgatctggtg gtgtttgcac aggtaggcgc atacttgttc 234240
gggactgagt ttgcggcgga taaagggtgt cgatgtgctg aatcagctgc gaatcgagct 234300
tatagggttg tcgcttacgc tgtttgatag tctggctttg ccgctgggct ttttcggcgc 234360
tgtattgctg cccttgggtg cggtgccgtc tgatttcgcg gctgatggtg cttttgtggc 234420
ggttcagctg tttggcgatt tcggtgacgg tgcagtggcg ggacaggtat tggatgtggt 234480
atcgttcgcc ttgggtcagt tgcgtgtagc tcatggcaat ctttcttgca ggaaaggccg 234540
tatgctaccg catactggcc tttttctgtt agggaaagtt gcacttcaaa tgcgaatccg 234600
ccgacctctt tcagttacag cagcttgatc cctttccctt atccaacggg ggaaggctag 234660
gatagggtgg cttgcaaata tacagaacaa gggacaagag ccaccctctc tccaaccctc 234720
tccctccgta cgggaggggg tggattctcg cgggcgaagc ccacgctacg gttagccttt 234780
accccagcac aaacaattcc cgcccgtgcg ccttcagcca acttttagca ttgtcggtat 234840
gcggcgtcag cgtgttcacc aaatgccaaa agcgcggact gtggtcgggg tggcggaggt 234900
ggcagagttc gtggatgcag acatagtcgg cgacgtattc gggcgtgccg atcagccgcc 234960
agttgaggcg gatgccggtg tgcgggcggc atacgcccca aaaggttttg gcgttgctca 235020
ggtctgtggc ggtgggcgtc agtcctgttt cggctgcgtg tttttcaagg cggggcagca 235080
ggtattcgcg ggcgcgttcg ttcaacaggc ggcgcaggtg gtcgatttgt gcggcggttt 235140
cttttcgggg aagcaggatt tcagacgacg tgatacggat atggctttgg ctgtgggtat 235200
ccagcttggt ctttattccc cgataccaaa tccactcggg taagtttggg tgggaaacag 235260
gatgcacggg cgttttggca agcgtgttcc gcaaaatcgt ttcgtttgcc gccagccagt 235320
ttgctaacgc gtggtcttga aaaaagggtg ggacgttgat gctgaccgtc tgcatattga 235380
cggggcgcag aatcagattt ttcttggcac tgcgtttgag ttcgatttcg atgcacaaac 235440
cgtcggaaag agtataggtg aagcgtttca tagttgtgaa taggtttcag accggataca 235500
tcgtctgaaa caggaatttt ccatatcagg cggcaaactt cggataatat acaaaatcaa 235560
acatctgcgc tacaaggttc agccgaacaa gccgccgata tatttgctga tggtgatggc 235620
gctgagtact gccatcaaac cgaccacaat cacgccggaa acggtgagcc acagcgggtg 235680
tttgtagtcg ccgacaattt tggttttgta ggcggcaatc agaatcagac cgagggaaat 235740
cggtaaaatc aggccgttta atgcgcctac gaacaccagc acctgcgccg gtttgccgat 235800
ggtggaaaat acggcggtgg acacggcgat aaaggcaata atccatttgt ttttattgcg 235860

ttcgatagac gggctgagac cggagaagaa cgacaccgaa gtataagccg caccaatcac 235920
cgaagtaatc gaagccgccc aaatcaccac gccgaaaatc agcaggccga tgtatcccgc 235980
cgcatattca aacggtgtgg aagcagggtt gtcgggattg agctgtacgc cttggctgac 236040
cacgcccaaa accgccaaaa acaatacaat ccgcataatc gaggcaatca ggatcgcccg 236100
caccgagctt tggctcactt ccggcaacgc cgatttgcct ttgatacctg cgtccagcag 236160
acggtgcgca ccggcgaagg tgatgtagcc gccgaccgtg ccgcccacca gtgtaacaat 236220
cgccattgca tcgagttttt ccggcataaa ggtatgcacg gcggcatctg ccagcggcgg 236280
attcgcctgc catgccacat aaaccgtcag cgcaatcatt acgaaaccca tcacttgggc 236340
gaatttgtcc atcactttgc ctgcttcttt aaacagaaac acaccgatgg caatcacgcc 236400
gctgatcacg gcaccggttt ccggtgacag tccggtcagc aggttcagac ccaagcctgc 236460
gccgccgacg ttgccaatat tgaacgccaa accgcccatc acaatcagca cagccaagaa 236520
atagcctgcg ccgggcaaga cctgattggc aatatcctgc gcctgttttt cggaaacggc 236580
gacaatccgc caaatattga gctgcgcccc gatgtcgagc agaatcgaga gcagaatcac 236640
aaagccgaaa cttgccgcca gtgcttgggt gaaggtggcg gtttgggtca gaaagcccgg 236700
gccgatggcg gaagtcgcca tcaggaatgc agcgccgatt aaggcatttc tgcggttttt 236760
ttgatcagac ataatcgctt atcctctata aaattggttg ttgctgtgtt tgggcgaaac 236820
ctgcggtttt agctacgcag aaactcgctt tgctcgtttt ggcgaaacct gcggttttca 236880
gacggcctat gaactgtttt tcaagcagaa actttgatgc ctgccgccag tagttcctgc 236940
cggatttttt cggcaaacac cacggcgtgc ggcccgtctc cgtgcagaca gatgctgtcg 237000
gcttgcacgg caaccaggct gccgtccact gctttgacct gcccgtcccg caccatctgc 237060
aatacttggg cgatggcttc ttcgtcgctg tccacctgcg catcggggcg gctgcgggga 237120
accagcgtac cgtcgggcat atagcggcgg tcggcgaata cttcggaaat cacacccaag 237180
cctgcggctt ttccggcttc caagagcagg ctgccggaaa gtgccatcaa tttcaatttc 237240
gggtcgaaat ccgccacaat tcgggcaacg gtatccgcca gcgcacggtt tttcgccgct 237300
tgattgtaca ttgcgccgtg cggtttgaca taagccattt ccaaaccctg atcacggcac 237360
aaggcctgca atgcgcccaa ctggtaattc agacacgccc gcaaatcggc ttcggacaga 237420
ttcatttcgg tacggccgaa gttttcccga tcgggatagc cggggtgtgc tccgatgcgc 237480
acgccgtttt gttgggcata cgccaatgcc gcccgaatat cggcaatgct gccggcgtgt 237540
tgggcgcagg cgatgttggc cgaagtaatc agctgcaaca aggcttcgtc gctgccgcag 237600
ccttcggcga gatcggcgtt taaatcaacc tgcttcatgg gtgattctcc gtatttggtt 237660
cagataggct tgtttttgcg ccgcagggcg gtggcttctt tcaagccgat tattttgaat 237720
ttgactttgc tgccgaagcg cacctgtgcc agcctgccca aatcggcggc ggcaacggta 237780
gcgattttcg gataaccgcc ggtggtttgc gcatcggcca gcaggataat cggtttgccg 237840
ccgggcggca cctgcacggt tcctgcctga acagcgtggg acagcatttc caaaggttgc 237900
gacagggtca gcggctgtcc gtcgaagcgg tagcccatgc ggttgctatc gctttgcagc 237960
gtccacgttt cccgttccag attcagacgc ccttttttcac tgaaagcggc atattccgac 238020
gaaggaacaa ggtggacggt atcggtaaac ggtatcgggg caatgccgac tttggacaat 238080
tcctgcgcac ctttgccgat ggggagataa tcgccttttt gcagcattct gccctgatgg 238140
ccgccgaaac cggctttcag gtcggtgctt ctcgaaccca tcacttccgg cacatcaaat 238200
ccgcccgcca cgcacacata gccgtacatg ccctgcacgg cacgcaccag tttcaaggtc 238260
tgccctttgc gggcggtata acgccaatac gaatagaccg gttcgccgtc caattccgcc 238320
tgatacacgg caccggtgag acaaaacggc gtatcccgtt caaacaccag cattatcccg 238380
cccaaagcga tttcgattgc ggccgtgcct tcgtcgttgc ccaataaaat attgcccgcc 238440
gccaaagcaa ccgtgtccat cgcaccggca tgaccgatgc cgtaacgccg gtgtccgtag 238500
cgtccggtat cctgaatatg cgccggtgcc tgcactgccg aaacgtgaat catggctcaa 238560
tcctttctgc aacaaagcgg acttggtcac ccgccgccag cagggtcggc ggattcaaat 238620
cggctcggaa caagggtaat tcggttctgc cgataatctg ccagccgccg ggcgaagcga 238680
acggatacac accggtctga ctgccgccga taccgaccga accggcagga acggacgttc 238740
tcggcacggc acggcggggc gtgtgcaatg cttcgggcaa gccgcccaga taagggaaac 238800
cgggctggaa gcccatcata aatacggtat aagtttgcgc cgtatggcgg cggacgattt 238860
cggaaataac cgtctgatgg aaagcagcga cttccgccaa atccgggccg tattcgccgc 238920
cgtagcagac gggaatttcc accagtttgc cctgatggtc tgtaacggcg gtgtgttccc 238980
acacatattg caattcatcg gcaagcgtcg ccaaatcggt atcgaaacgg gtaaacacgg 239040
tcagattgtt catgccgacc accacttcct caatcctgtc gtgctgcccg agcgcagcgg 239100
caaacgccca caacttttgc tgtttgccca gttcggaagg cgcattcagt cggtagacca 239160
aagcggattc gctgattggt gtgatctcta ttctcatttg ttgttcattt tggttatgtt 239220
ttaatgaatc tatatgcagg ggcggcggtt tgtcaatatc ttctgtgctg catcatcaaa 239280
ccgtcgattg gaaaagtgct gccctgccgc tgcacttttt cagacgacct taaaccgttt 239340

ctattaaaat agcgcattcc acttttcaga cggcatcctt atgtttcccg accaatccgc 239400
ccccaacctg ctgcaaggct tgaatcccga acaactctcc gccgtaacct ggccgccgca 239460

atccgcactt gtgctggcgg gcgcgggcag cggcaaaacg cgcgtgctga ccacgcgcat 239520
cgcatggctg ttgcaaagcg gacaagccag cgtgcacagc attatggcgg taacgtttac 239580
caacaaagcc gccaaagaaa tgcaaacccg tttgggcgcg atgattccca tcaatgtccg 239640
cgccatgtgg ctcggcacgt tccacggtct ctgccaccgc tttttgcgcc tgcaccaccg 239700
cgacgccggt ctgccgtctt cctttcaaat cctcgacggc ggcgaccagc tttccctcat 239760
caaacgcctg ctcaaaagcc tcaacatcgc cgaagaaatc atcgcgccgc gttcgctgca 239820
aggctttatc aacgcgcaaa aagaatccgg tttgcgcgct tccgtgttga gcgcgcccga 239880
tccgcacaca cgccgcatga ttgagtgcta cgccgaatac gacaaaatct gccaacgcga 239940
aggcgtggtc gattttgccg aactcatgct ccgcagctac gaaatgctgc aaaacaacga 240000
aatcctgcgc cagcactacc aaaaccgctt caaccacatt ctcgttgacg agttccaaga 240060
caccaacaaa ctgcaatatg cttggctgaa actgattgcc ggcaaccacg cagcagtatt 240120
tgccgtcggc gacgacgacc aaagcattta ccgtttccgt ggcgcaagcg tcggcaacat 240180
gaccgcgctg atggaagaat tccacatcga cgcgcccgtc aaactcgaac aaaactaccg 240240
ctccgtcggc aacatccttg ccgccgccaa tgccgtgatt gaaaacaacg acgaacgact 240300
cggcaaaaac ctgcgcaccg acgccgaagc aggcgacaaa atccgctact actccgcctt 240360
taccgacctc gaagaagccc ggttcatctt ggacgaaacc aaagccctcg aacgcgaagg 240420
ctgggatttg gacgaaatcg ccgtcctcta ccgtagcaac gcccaatccc gcgttatcga 240480
acaaagcctg ttccgcagcg gcattcccta caaaatctac ggcggcttgc gtttttacga 240540
acgccaagaa atcaaacacg cgctcgccta cctgcgcctc gccgtcaatc ccgacgacga 240600
caacgccctc ttgcgtgtca tcaacttccc accgcgcggc atcggtgcac gtaccgtcga 240660
aaatcttcag acggcctcaa acgaacaagg catcaccctc tggcaagccg cctgcaacgc 240720
cggcgcgaaa gccgccaaag tcgtcgcctt cgtccgcctg attgaagccc tgcgcaacca 240780
agtcggacaa ctgtccctgt ccgaaatcat cgtcggcatc ctcaaagaca gtggcttgac 240840
cgaacactac cgcacccaaa aaggcgacaa ccaagaccgt ctcgacaacc ttgacgaact 240900
cgtcaacgcc gccatcgaat tcaaacccga agacagcaac ttcgaaatcc tgcctgaaaa 240960
catttcagac gaccccgcct tccccattct cgccttccta agcaatgccg ccctcgaatc 241020
cggtgaaaac caggcaggcg caggcgaaaa ggccgtccaa ctcatgaccg tccacgccgc 241080
caaaggcttg gaatttaacg ccgtcttcct caccggcatg gaagaaggcc gcttccccag 241140
cgaaatgagc cttgccgaac gcggcggcct cgaagaagaa cgccgcctca tgtacgtcgc 241200
catcacccgc gcccgcaaac gcctctacat caccatggcg caacaacgca tgctgcacgg 241260
acaaacccaa ttcggcatcg tctcccgctt cgtcgaagag atcccacccg aagtattgca 241320
ctacctgtcc gtcaaaaagc ctgcctacga cagttacggc aacacgcgcc aaaccgccgc 241380
atccaaagat aaaatcatcg acgactacaa acagccccaa acctacgcag gtttccgtat 241440
cggacaaaac gtccgccacg ccaaattcgg caccggcgtg attatcgatg ccgcagataa 241500
aggcgaatcc gcccgactga ccatcaattt cggcaaacag ggcgtgaaag agttggacac 241560
caagtttgcg aaattggaag agatgtaaat ttgaaatgta ggtcggatat tcgtatccga 241620
cctacggcaa aaaccttagc aggagagaat agaaacccgt agcgtgggct ttttctatga 241680
atcaagccca aaatttcaga cggcattttt agccgtcatt atcgtggatg aagcccacgc 241740
tacaatgtac acacagagca aatagagatg tgggtcggat attcgtatcc gacaaaaaca 241800
tttgacgcgt ctattgtttc cgaaacaccg ctgttggaaa tgtcggatac aagaatctga 241860
cttacggcaa aaaacgtagt aaggacaaag caaaaggccg tctgaaaacg ggaagggcaa 241920
ttttgccgca accgccgccg tcattcccgc gcaggcggga atccagacct ttcggcacgg 241980
aaacttatcg gataaaaggt ttctttagat tccacgtcct agattcccgc cggaacataa 242040
atgacggacg gtaaaagccg ggtatgaata cccaccctct gttatcactg agatcaataa 242100
ggaagaacat tatgtcccaa gtttttaaag attttgactt gtcctccgta tggaaaacta 242160
atagttgggc agatgaaaac tacaaagaag ccccgtttac ccctgaaatt ttggctgccg 242220
tagaaagtga actgggctat aaattgccgc aaagttttat tgaattgatg gcagtacaaa 242280
acggcggaat atttgtcaaa aactgttttc cgaccacgca gagaaattcg tgggcggaaa 242340
atcatgtgca aatttgcgag gtatcgggaa tcggttttga aaaagaaggg agtttgtgcg 242400
gcgcgatggg gcaaaaactt tggctggaag aatgggaata cccgcctatc ggcgtgtatt 242460
ttgccaacga cccgtcaggc ggtcatgcca tgtttgcctt agactatcgg gcgtgcggca 242520
aagacggcga gccgaaagtg gtgtttgtcg aacaagaatc ggattttgaa atcgtcgaac 242580
ttgcccccga ttttgaaacc tttatccgca gcttgcggca tgaagatgag tttattgacg 242640
aagaaatata aaacggtggt tgaaaaactg aaatcatcaa gagaaaacgg gcgaaataac 242700
gggtaatcgc ttgaatccgt aaggaaaacg gtttggtgga acgcgccatc caagaccttt 242760
gcaaaaaact gtccccgaca gcattgacat tattaacaga acttatcaat tttggagcta 242820
tctcaaatat aattcggtta tcctgttgta tccattaaat catatgcttc aattaattgt 242880
tgttctagct cttataccaa ttttggattg cgaattcctg acacaatctc aaattcttct 242940
gcatctatgc aaacacctgc ataaatttca ataacaaggg aacgcaataa ttgaagctct 243000
tctcttgtta aagaaataat aatgtcatca cctttgtaat tgattatatt cataataatt 243060
ttatttttgt ttgtcaaagt aagttttgcc taaggttggt ctaaatgcag ttccaccatc 243120

ttttgaattt gggtctctga ttacaattgc tccagactta tcatcccaaa ttgctcttat 243180
gtgtttggat tgtaatcttc gaattcccaa gaaaaaaatc gtaataagtt tgaaagtgtc 243240
aaatcccaag tttcttttga gcaatattct aatattttat caatttcact tttaataatc 243300
ttatgatcaa actgttctaa tattaatgca ttagaccaaa aaaaaccttc tttattacaa 243360
tgatgggaaa tccatttagg agaacaaatg caaagtgaaa aaatagatga gccttgttct 243420
ccttcgattc cgatatccaa atctatccat ctatggaaat tatctggaat ttcgggggta 243480
aatttttcaa aatcaatatc atataaattt atgcttttta aatccaattt aatcattagg 243540
gctgtcctag ataaataggg aaattcaaat taagttagaa ttatccctat gagaaaaagt 243600
cgtctaagcc ggtataaaca aaataaactc attgagctat ttgtcgcagg tgtaactgca 243660
agaacagcaa cagagcccga cagcattgtt tatacggatt gttatcgtag ctattcattt 243720
acgcaagttt aacggcattc ccaaagcgca ttttgagctg tatttaaagg agtgcgaatg 243780
gcgttttaac aacagtgaga taaaagttca aatttccatt ttaaaacaat tagtaaaatc 243840
gagtttatct tagttgtcca ggacagcccc attattttta taacaccgtg aagccgcaca 243900
gcagtttgaa cagtgatacg ccgtttgcgg gcttacgagt ttattttccc ggcctgcagt 243960
ttgagcaata cggtgatttc ctacggttaa tacaaatgtt tacacattga tacatttcat 244020
ttatagttcc gcctatttga aaatagaaaa tatgaattcg accgcaagta aaaccctgaa 244080
aggattgtcg ctggtgtttt tcgcctctgg attctgcgcc ctgatttacc aggtcagctg 244140
gcagaggctt ctattcagtc acataggtat cgatttgagt tcgattactg tcattatttc 244200
tgtatttatg gtcggcttgg gtgtaggtgc gtatttcggt ggacgcattg ctgaccgttt 244260
tccttcaagt atcatccccc tgttttgcat cgctgaagta tccatcggtc tgttcggttt 244320
ggtaagcagg ggtctgattt ccggcttggg gcatctttta gttgaggctg atttgcccat 244380
catcgctgct gccaatttcc tcttattgct gcttcctacc tttatgatgg gcgcgacctt 244440
gcccttgctg acctgttttt ttaaccggaa aatacataat gttggcgagt ctatcggtac 244500
cttatatttt ttcaacactt tgggtgcggc actcggatcg cttgccgccg ccgaattttt 244560
ctacgtcttt tttaccctct cccaaaccat tgcgctgaca gcctgcttta accttctgat 244620
tgctgcttca gtatggctgc gttacagaaa ggatggatat agtgaacact aaaccgaata 244680
ctagtttgat ttatatgctt tctttcctta gcggcttatt gagcttgggt atagaagtct 244740
tgtgggtgag gatgttttcg ttcgcagcac agtccgtgcc tcaggcattt tcatttaccc 244800
ttgcctgttt tctgaccggt atcgccgtcg gcgcgtattt tggcaaacgg atttgccgca 244860
gccgctttgt tgatattccc tttatcggc agtgcttctt gtgggcgggt attgccgact 244920
ttttgatttt gggtgctgcg tggttgttga cgggtttttc cggcttcgtc caccacgccg 244980
gtatcttcat taccctgtct gccgtcgtca gagggttgat tttcccgctc gtacaccatg 245040
tgggtacgga tggcaacaaa tccggacgac aggtttccaa tgtttatttc gccaacgttg 245100
ccggcagtgc attgggtccg gtccttatcg gctttgtgat acttgatttc ttgtccaccc 245160
aacagattta cctgctcatc tgtttgattt ctgctgctgt ccctttgttt tgtacactgt 245220
tccaaaaaag tctccgactg aatgcagtgt cggtagcagt ttccctaatg ttcggcatcc 245280
tcatgttcct actgccggat tctgtctttc aaaatattgc tgaccgtccg gataggctga 245340
ttgaaaacaa acacggcatt gttgcggttt accatagaga tggtgataag gttgtttatg 245400
gggcgaatgt atacgacggc gcatacaata ccgatgtatt caatagtgtc aacggcatcg 245460
aacgtgccta tctgctaccc tccctgaagt ctggcatacg ccgcatttc gtcgttggac 245520
tgagtacagg ttcgtgggcg cgcgtcttgt ctgccattcc ggaaatgcag tcgatgatcg 245580
ttgcggaaat caatccggca taccgtagcc ttatcgcgga cgagccgcaa atcgccccgc 245640
ttttgcagga caaacgtgtt gaaattgtat tggatgacgg taggaaatgg ctgcgtcgcc 245700
atcctgatga aaaattcgac ctgattttga tgaatacgac ttggtactgg cgtgcctatt 245760
ccaccaacct gttgagtgcg gaatttttaa aacaggtgca aagccacctt accccggatg 245820
gtattgtaat gtttaatacc acgcacagcc cgcatgcttt tgctaccgcc gtacacagta 245880
ttccctatgc ataccgctat gggcatatgg tagtcggctc ggcaaccccg gtagttttcc 245940
ctaataaaga actgctcaag caacgtctct cccggttgat ttggccggaa agcggcaggc 246000
acgtatttga cagcagcacc gtggatgctg cagcacaaaa ggttgtctct cgtatgctga 246060
ttcagatgac ggaaccttcg gctggggcgg aagttattac cgacgataat atgattgtag 246120
aatacaaata cggcagaggg atttaaccgt cttaaagggt ttcaggcaac gcaggtttta 246180
ggtaacgtcc tgctagttca aaaaaaccgc atcacagcag tcgggacaaa atggtttaaa 246240
cattttgtcc cgaattctta ttcctatata tagtggatta acaaaaatca ggacaaggcg 246300
acgaagccgc agacagtaca aatagtacgg aaccgattca cttggtgctt gagcacctta 246360
gagaatcgtt ctctttgagc taaggcgagg caacgccgta ctggtttttg ttaatccact 246420
ataccacgaa ttacggtgta aaaatttata tgaccttata aaatcaaata agaatcgtta 246480
tcataacatg attgtattta ttgggttttt ttgggcgttt tgccgatatt taccttttaa 246540
tggtttttga aattcgctaa aatacgaaat tattgtagaa attttgttaa cggatttggg 246600
tgtaaccatg ttgtccgctt actttcccgt ctttgtcttt atcctcatcg gcctcgcggc 246660
cggcgtgctg tttatcctgc tcggcacgat tttaggcccg aaacgccact atgccgaaaa 246720
agacgcgcct tacgaatgcg gttttgaagc ttttgaaaac gccaggatga agttcgacgt 246780

gcgctattac ctcgtcgcca tcctcttcat cctgtttgat ttggaggtcg cgtttatgct 246840
gccgtgggca gtcgtgttca aagatttggg cgcgtacggc ttctggtcta tgctggtgtt 246900
tatcgttgtt ctgacggtag gctttgttta cgaatggaaa aaaggtgcgc tggaatggga 246960
atagaaggcg ttttgaaaaa aggtttcatc accaccagcg cggatacggt gctgaactat 247020
atgcgtaccg gttcgttgtg gccggttact ttcggcttgg cctgctgcgc cgtggaaatg 247080
atgcacgcgg gtatggcgcg ttacgacctt gaccgtttcg gtattatttt ccgtccgtcc 247140
ccccgtcagg ccgacctgat gattgtggcg ggtacgctca ccaataaaat ggcgcccgcc 247200
ctgcgccgag tgtacgacca gctcgccgag ccgcgctggg tattgtctat gggctcatgt 247260
gccaacggcg gcggctatta tcactattct tattccgttg tgcgcggtgc cgaccgcgtc 247320
gtgccggtag atgtttatgt gccgggttgt ccgccgactg cggaagccct gatttacggc 247380
ctgattcagc tccaacaaaa aatcaagcgc acttccacca ttgcgcgtga cgagtaagga 247440
gaggacgata tggcaagcat tcaagactta tacgaaaccg tcagccgcgt tttgggcaat 247500
caggcaggca aagtcatttc cgctttgggc gagattaccg tcgagtgtct gcccgagcac 247560
tatatttcag tcatgaccgc attgcgtgac catgaagagt tgcatttcga gcttctggtt 247620
gacttgtgcg gtgtcgatta cagcacttac aaaaacgaag catggcaggg caaacgcttt 247680
gccgtcgtca gtcagttgct ttccgttaaa aacaatcaac gcatccgcgt gcgcgtctgg 247740
gtttcagacg acgacttccc cgtagtcgaa tctgtagtcg atatttacaa cagcgcggat 247800
tggtacgaac gcgaagcctt cgatatgtac ggcatcatgt tcaacaacca tccggacttg 247860
cgccgcatcc tgaccgatta cggcttcgtc ggacatccgt tccgcaaaga cttcccgatt 247920
tccggctatg tggaaatgcg ttacgacgaa gagcaaaaac gcgtgattta ccaacctgtt 247980
accattgagc cgcgcgagat cacgccgcgt atcgtccgtg aggagaacta cggtggccaa 248040
taaattaaga aactacacca tcaacttcgg cccgcaacac cctgcggcgc acggcgtatt 248100
gcgtatgatt ttggagctgg acggcgaaca aatcgtccgt gccgacccgc atatcggcct 248160
cttgcaccga ggtaccgaaa aactggcgga aaccaaaacc tatctgcaag ccctgcccta 248220
tatggaccgc ttggactatg tttccatgat ggtcaatgag caggcgtatt gtttggcagt 248280
agaaaaactt gtcggtatcg atgtgcccat ccgcgcccaa tacatccgcg tgatgtttgc 248340
cgaagtaacg cgcatcctca atcacttgat gggcatcggt tcgcatgcct tcgacatcgg 248400
cgcgatgacc gccattcttt acgccttccg cgaccgcgaa gagctgatgg acttgtacga 248460
agccgtgtcc ggcgcgcgta tgcacgccgc ctacttccgt cccggcggcg tttaccgcga 248520
cctgcccgac tttatgccca aatacgaggg cagcaaattc cgcaatgcca aagtattgaa 248580
gcagctcaac gaatcccgcg aaggcaccat gctcgacttt atcgatgcct tctgcgaacg 248640
cttccccaaa aatatcgaca cactcgaaac cctcctgacc gacaaccgta tttggaaaca 248700
gcgtaccgtc ggcatcggcg tcgtctcccc cgaacgtgcc atgcaaaaag gctttaccgg 248760
cgtgatgttg cgcggttcgg gcgtggaatg ggacgtgcgt aagacacagc cttacgaagt 248820
gtacgacaaa atggatttcg acatccctgt cggcgtgaac ggcgactgct acgaccgcta 248880
cctctgccgt atggaagaaa tgcgtcaatc cgtacgcatc atcaaacaat gttccgagtg 248940
gttgcgtgtc aatccgggtc cggtcattac cacaaaccac aaattcgctc cgcccaaacg 249000
taccgaaatg aaaacaggta tggaagacct gattcaccat ttcaaactct ttaccgaggg 249060
tatgcacgtt cccgagggcg agacctacac cgctgtcgaa catccgaaag gcgagttcgg 249120
cgtttacatc atttcagacg gcgcaaacaa accctaccgc ctgaaaatcc gcgcacccgg 249180
cttcgcccat ctgcaaggca tggacgaaat ggcaaaaggc cacatgctcg ccgacgtcgt 249240
tgccatcatc ggtacgcagg acatcgtatt cggggaggtt gaccgataat gttatccgca 249300
gaatctttaa aacaaatcga catcgagttg gcaaaatatc ctgccgacca acgccgctcc 249360
gcgattatgg gcgcattgcg tattgcccaa accgaaaaag gctggcttgc tcccgagacc 249420
atcgcttttg tcgccgacta catcggcatc acgcctgcac aagcctacga agtcgccact 249480
ttctacaata tgtacgacct tgagcctgtc ggcaaataca aactgaccgt ttgtaccaac 249540
ctgccctgcg ccctgcgcgg cggtatggct accggcgaat acctcaaaca aaaactcggt 249600
atcggctacg gcgaaactac ccctgacggc aagtttaccc ttgtcgaagg cgaatgcatg 249660
ggcgcatgcg gcgacgctcc cgttatgctg gtcaacaacc acagcatgtg cagctttatg 249720
accgaagaag cgattgagaa gaaactggcg gagttggagt aggtcgtctg aaacgacgat 249780
ttaaacgtag gtcggatact tgtagccgac agagtgggta aaaaggcaaa atgtcggatt 249840
taagaatccg ccctactgaa ataccgaaat gccgtcattc ccgcgcaggc gggaatccac 249900
cggtaagatt cggtttctga atttaataag acattgctta ccattgagga tggattcccg 249960
cctgcgcggg aatgacgaca gacaagcaag tggtcgagat ccaacaaaaa cgattaaagg 250020
tcgtctgaaa atatcgattt gataaactag attttatttc agacgacgtt acaagccggt 250080
acacaaagac atcttaaggt cgtctgaaac agcggccgca accgatacga aaacaaacag 250140
gcacaccaaa aatggctatt taccaatcag gcgtgatttt tgaccaagtg gataccgcca 250200
atcccgattg ctggacattg gacgaatacg tcaaacgcgg cggctatacc gccctgcgta 250260
aaattctgtc cgaaaacatc tcgcaaaccg atgtgattga cgaagtcaaa acctccggtt 250320
tgcgcgggcg cggcggtgcg ggcttcccga ccggtttgaa atggagcttt atgccccgtt 250380
ctttcccggg cgaaaaatat gtggtttgca acaccgacga aggcgaacca ggtacgttta 250440

aagaccgcga catcatcatg ttcaatccgc atgccctgat cgaaggcatg attatcgccg 250500
gttacgcgat gggcgcgaaa gccggttaca actatatcca cggcgaaatt tttgaaggct 250560
accaacgctt tgaggccgct ttggagcagg cgcgtgccgc aggctttttg ggtaaaaata 250620
ttttgggttc ggattttgaa tttgaactct tcgcccacca cggctacggc gcatatattt 250680
gcggcgagga aaccgcattg ctcgaatcgc tggaaggcaa aaaaggccag ccgcgcttta 250740
agccgccatt ccctgcttcg ttcggcctgt acggcaaacc gactaccatc aacaatactg 250800
aaacgttctc ctccgttcca ttcattatcc gtgacggtgg acaggcattt gccgataaag 250860
gtattccgaa tgcaggcggt accaaattat tctgtatttc cggccatgtc gagcgtccgg 250920
gcaactatga agtgccattg ggtacgccgt ttgccgaagt cttgaaaatg gcgggcggta 250980
tgcgcggcgg taaaaaactc aaagccgtca ttcccggcgg ttcgtccgcg cccgtattgc 251040
ctgccgacat catgatgcag accaatatgg actacgactc gatctccaaa gcaggctcca 251100
tgctcggttc cggcgcgatt atcgtcatgg acgaagacgt gtgcatggtc aaagcccttg 251160
agcgtttgag ctacttctac tacgacgagt cttgcggcca atgtaccccc tgccgagaag 251220
gtacgggctg gctttaccgc atcgtccacc gcatcgtaga aggcaaaggt aaaatggaag 251280
atttggattt gctggattcc gtcggcaacc aaatggcagg ccgcaccatc tgcgccctcg 251340
ccgatgctgc cgtcttcccc gtccgcagct ttaccaagca tttccgtgat gagtttgtgc 251400
attacatcga acacggcggg ccgatgaaag agcataagtg gggagggtgg taatggtgga 251460
agctaaaatt tttattctat acggtgcagc caacaaaggt aagagtacga cactcaatac 251520
gctttttaat cagatttgtc ggaaattttc taaatttcta gtctttttttg aaagacatgg 251580
aaacggctta gattttgttg cagtatttga tcatgaaggt cagagaattg gtttttattc 251640
atctggtgat aatgaatacg aggttagggg aaatttatac aaactttatt cgcataattg 251700
tgattttatt tttggcacgt caaggacacg ggtggtagt tgcgatgcag taggatgtta 251760
tgcagagtta ttgcatggcg atgtaaatat aattaattgg tgtgaaaagt ttgagcctac 251820
agatgaagac aatgagcgtg ctgttaaaga gttatttaag tcatttaaaa atataataaa 251880
tgagttatag ttttagttgg ttttatattg gttaaaagca aaatgctaaa aatttaactt 251940
tgccgtcatt cccgcgtagg cgggaatcca tagtggaatt tacagaaccc gatatttgaa 252000
aagcagttgc cgaaattcaa aaaatggatt cccgcctacg cgggaatgac ggcgggagta 252060
ggcagatgtt ttcagatgaa aacggttgta aatgatatta aaaaagttgt tgtttatatt 252120
gcaggaaaaa tgaatacgaa accatccgct tactagacaa cctgccgtat atattttggc 252180
aaacggtaaa aatggaacac tctatatcgg tgttaccatg aatttgccgg aaagggtttg 252240
gcagcacaaa aaccatgtca atattgatgg ctttactgcc cgatatgatg tgcatgattt 252300
agtttggtat cagttttttg agaatatgcc tgaagcagtt gccaaagaaa aaacgatgaa 252360
aaaatggcga cgtgaatgga agattaaact gattgaagaa caaaatactg aatgattgga 252420
cttgtcgggc gtgttgtttg tttagtttta tttctggaac tttaaaaact gtcgttattc 252480
cagcccccacc tacgcgcaga caggctacgg cgggaatcac cgcaaaagtt aagaaaccaa 252540
tgtttgaaaa cagttaccga aaacccaaga atggattcac gcctgtgcgg gaatgacggc 252600
aaggtggcag taaacgtttt aaacagtatt gattgtcaat gaaactcaaa aggccgtctg 252660
aaacccattt ttcagacgac ctccataaaa gattatttat caaatacccg taactaggaa 252720
cgaaccatgt tacaaatcga aatcgacggc aaacaagtat ctgtggagca gggcgcgacg 252780
gtgattgaag ccgcgcacaa gctcggtact tatattccgc atttctgtta ccacaaaaaa 252840
ctttccatcg ccgccaactg ccgtatgtgt ctggtgaacg tagaaaaagc cccaaaaccc 252900
ctgcctgcct gtgccacgcc ggttacagac ggcatgattg tgcgtacgca ttcggcaaaa 252960
gcccgagagg cgcaggaagg cgtgatggag ttcctgctca tcaaccatcc gcttgattgt 253020
ccgacctgcg accaaggcgg cgaatgccag ttgcaggatt tggcggtggg ctacggcaaa 253080
accaccagcc gctacaccga agaaaaacgt tccgtcgtcg gcaaagatat ggggtccttg 253140
gtttccgccg aggaaatgag ccgctgtatc cactgcaccc gctgcgttcg tttcactgaa 253200
gaaatcgccg gtttgcagga aattgcgatg gtgaatcgcg gcgaacactc cgaaatcatg 253260
ccctttatcg gcaaaacggt ggaaaccgaa ttgtcgggca acgtcattga tttgtgtccc 253320
gtcggcgcgc tgaccagcaa accgttccgc ttcaacgcgc gtacttggga attgaaccgc 253380
cgcaaatccg tttccgccca cgatgctttg ggcagcaacc tgattgtgca gaccaaagac 253440
cataccgtcc gccgcgtgtt gccgttggaa aacgaagcga ttaacgaatg ctggctgtct 253500
gaccgcgacc gtttcgccta cgaaggcctg tatcacgaaa gccgtctgaa aaacccgaaa 253560
atcaaacagg gcggcgagtg gatggacgtg gattggaaaa ccgcgttgga atatgtccgc 253620
agcgcgattg aatgtatcgc caaagacggc aagcaaaacc aagtcggcgt ttgggcgaac 253680
ccgatgaata cggttgaaga actgtatctg gcgaagaaac tcgccgacgg cttgggtgtt 253740
aaaaactttg caacccgttt gcgccaacaa gacaaacgtc tttcagacgg ccttaaaggt 253800
gcgcaatggt tgggacaaag cattgaatct ttggctgaca acgatgccgt attggtagtc 253860
ggtgcgaact tgcgcaaaga acagccgctc ctgactgccc gcctgcgccg cgccgccaaa 253920
gaccgtatgg cattgagcgt attggccagc agtaaagaag aattgtttat gccgcttctg 253980
tctcaagaag ccgcacatcc cgacgagtgg gcaggccgtc tgaaaaacct gtctgtcaat 254040
gcggaacacg ccgttaccgc cagcctgaaa aatgctgaaa aagcagcggt gattttgggc 254100

gcggaagtgc aaaaccatcc tgattacgcc gcggtttacg ccgccgcgca agagctggct 254160
gacgcgaccg gcgcagtgct gggcattttg ccgcaagccg ccaacagcgt tggtgcggat 254220
gtcttgaatg taaactccgg caagagcgtt gtcgaaatgg taaacgcgcc gaaacaggca 254280
gtcttgctgc tcaacgttga gcctgaaatc gatacggcgg acggtgcaaa agccgtagcc 254340
gcgttgaaac aggcaaaaag cgtgatggcg tttacgccgt ttgtcagcga aacgctgctg 254400
gacgtgtgcg acgtgttgtt gccgattgca ccgtttaccg aaacctcagg cagcttcatc 254460
aatatggaag gccgtctgca atccttccac ggcgtggtac aaggcttcgg cgattcgcgt 254520
ccgctgtgga aagtgttgcg cgtattgggc aacctgtttg acctgaaagg ttttgaatac 254580
cacgataccg ctgcgatttt gaaagacgcg ctggatgtgg aaagcctgcc gtccaaactg 254640
gacaaccgca acgcatggac aggggagggc gttcagacga cctcagaccg cctcgtccgt 254700
gtcggcggcg tcggtattta tcacaccgat tctatcgtgc gccgttccgc accgttgcaa 254760
gaaaccagcc atgccgccgt gcctgctgcg cgtgtaaatc caaatacatt ggcacgcttg 254820
ggcctgcaag acggacaaac cgctgtcgcc aaacaaaacg gcgcaagcgt atcggttgcc 254880
gtcaaagccg atgccggact gcctgaaaac gtggtgcatc tgccgctgca taccgaaaat 254940
gccgcgctgg gtgcgttgat ggacactatt gaactggcgg gagcttgatt atgcaggaat 255000
ggttccaaaa cctctttgcc gcaacgctcg gtctgggcga tttgggtatt actgtaggct 255060
tggtggtatc cgtcatcgtc aaaattgtga ttatcctgat tccgctgatt ctgaccgtcg 255120
cctacctgac ttatttcgaa cgtaaagtca tcggcttcat gcagcttcgc gtcggtccga 255180
acgtaaccgg cccgtggggt ctgattcagc cgtttgccga cgtgttcaaa ctcttgttta 255240
aagaagtaac ccgtccgaag ctgtcaaaca aagccctgtt ctatatcggc ccgattatgt 255300
cgcttgcccc gtctttcgcg gcgtgggcag tgattccgtt caatgaagaa tgggtgctga 255360
ccaacatcaa tatcggtctt ttgtacatcc tgatgattac ctcgctgtcg gtttacggcg 255420
tgatcatcgc gggctgggct tccaactcca aatattcgtt cttgggcgca atgcgtgctt 255480
ccgcgcaaag catttcctac gaaatcgcca tgagtgccgc gctggtgtgc gtcgtgatgg 255540
tgtcgggcag catgaacttc tccgacatcg ttgccgcgca ggcaaaaggc atcgcaggcg 255600
gttcggtatt ctcttggaac tggctgccgc tcttccccat cttcatcgtc tatctgattt 255660
ccgccgttgc cgaaaccaac cgcgcaccgt ttgacgtggc agagggcgag tctgaaatcg 255720
ttgccggtca ccacgtcgaa tattccggct tcgcattcgc gctgttcttc cttgccgaat 255780
acattttcat gattctgatt gccgcgctga catcgttgat gttcctcggc ggctggctgt 255840
ctcccttccc gcaaagctgg ggcattgtcg gtacgccttc cgcattttgg atgttcgcga 255900
aaatggcggc ggttctgtac tggtatctgt ggatacgcgc caccttccca cgctaccgtt 255960
acgaccaaat catgcgcttg ggctggaaag tgctgattcc gatcggcttc gcctacatcg 256020
tgattttggg cgtgtggatg atttcaccgc tgaatttgtg gaaataagtt tcagacggca 256080
tcttgaggcc gtctgaacaa agcgattttg aatacctaac gaaatccctg ttttgaggga 256140
acataatatg gctaacttag taaaaacctt tctgcttggc gaattggtaa aaggtatggg 256200
cgtaacgctc aaaaactttt tcgcccgcaa agacacaatt tatttccccg aagagaaaac 256260
gccgcaatcc gtgcgtttcc gcggtctgca cgcgcagcgg cggtatccga acggcgaaga 256320
gcggtgtatc gcgtgtaagt tgtgtgaggc agtgtgtccg gcaatggcga ttaacatcga 256380
atcggaagaa cgtgaagacg gtacgcgccg caccaagcgt tacgacatcg acctgaccaa 256440
gtgcatcttc tgcggtttct gcgaagaggc atgcccgact gatgcgattg tggaaaccca 256500
tatttttgaa taccacggcg agaaaaaagg cgacttgcac atgaccaagc cgattctttt 256560
ggccattggc gacaaatacg aagctgaaat cgccaaacgc aaagccgctg acgcgccgta 256620
tcgttaatgc tttggggctt cttggaaggt tttaaatatg gaaggactga ttaatgcatt 256680
gaaatattta gccgaacatg agccaataga taattttgaa gaaattagaa ctagaaatag 256740
tccgattgag ttgccaagtg gattaagtaa ttttgaacaa aatatttttt taaaagaaaa 256800
tttatcccca aaattacaaa atgatgatag cttgaagacg cattattgga ttatccgtga 256860
atggggtggg attaaaagtt ttaaacaatc tgctgaaaat agccagctta ttcgtcaatt 256920
tttatcggaa cttaattcgg gaaaattgag tagtggtttg ttgaaaattt catcattatc 256980
taaattggct tcttttatag attgtgagcg attcgccatt tatgattcac gcgctatttt 257040
ttcgttgaat tggttgttgt ttaaatttac aaatgcagat ttgtttttttc agccacaagg 257100
tagaaatagg gaactagaaa tccgaaatat gaacgtattg tttcattttt ctgatatcaa 257160
accgaattat cggaaaccag acgtttcgtt tcatcaatat tgtgggttgt tacaagattt 257220
ggcgaaacaa gtttatggta aacaagcaaa accgtatcac atagaaatgt tgttattcaa 257280
aattgcgaca acgtggattt gtgcggatat ggatcaactg attaagtttg attgtttgcg 257340
taaccaggat tttcagactg cttgaaacca tatttttgat taataaagaa agcatagact 257400
atgactttcc aactgatttt attttatatt tttgcagtga taattcttta tggcgcgctc 257460
aaaaccgtca ccgctaaaaa ccctgttcac gccgctttgc atctggtgct gaccttctgc 257520
gtgagcgcga tgctttggat gctgatgcag gctgagtttt tgggcgtgac gctggtggtg 257580
gtttacgtcg gcgccgtgat ggtgttgttc ctgttcgtcg tgatgatgtt gaacatcgac 257640
attgaagaaa tgcgtgccgg tttctggcgg cacgcgcctg ttgccggtgt ggtcggcaca 257700
ttgttggcgg ttgcgctgat cctgattctg gtcaacccga aaaccgacct tgccgcattt 257760

ggtctgatga aagacattcc tgccgattac aacaatatcc gcgatttggg cagccgtatt 257820
tataccgact atctgttgcc gtttgaattg gcggcggtat tgctgttgtt gggtatggtg 257880
gcggcgattg cgctggttca ccgtaaaacg gttaatccga aacgcatgga tcctgccgac 257940
caagtcaaag tacgcgccga ccagggccgt atgcgtctgg tgaaaatgga agcggtcaaa 258000
ccgcaagtcg aatctgccga agaaagcgaa gtttcagacg acctcaagcc gaaagaggag 258060
ggcaaagcat gattaccttg acgcattatt tggtattggg tgcgctcctg ttcggtatca 258120
gcgcaatggg tatctttatg aaccgcaaaa acgtgctggt attgctgatg tcgatcgagc 258180
tgatgctttt ggcggtgaac ttcaacttta tcgccttctc gcaacatttg ggcgatactg 258240
ccggacaaat tttcgtattc ttcgtattga ccgttgccgc tgccgaatct gccatcggtt 258300
tggcgattat ggtgctggtg taccgcaacc gacaaacaat caacgttgcc gatttggacg 258360
agttgaaagg gtaaaggtag gttgggtcga gacctgacaa gacaccgatg ccgtctgaaa 258420
acccgatagg aaaaacgatg aaatccatag acgaacaaag cctgcataat gcccgccgcc 258480
tgtttgaaag cggcgacatc gaccgtatcg aagtcggtac caccgcgggc ctgcaacaga 258540
ttcaccgtta cctgttcggc ggcttatatg attttgcggg tcaaatcagg gaagacaaca 258600
tttccaaagg cggttttcgt tttgccaacg ccatgtattt aaaagaggct ttggttaaaa 258660
tcgagcagat gcccgagcgg acttttgaag aaatcatcgc caaatatgtt gaaatgaaca 258720
ttgcccatcc gtttttggag ggtaatggca gaagtacccg catctggctg gatttggtgc 258780
tgaaaaaaaa cctgaaaaaa gtcgtgaact ggcaaaatgt aagtaaaacc ctgtatttgc 258840
aggcgatgga acgcagcccc gtcaacgatt tagaactgcg ctttctgtta aaggacaacc 258900
tgactgacga tgtggacaac cgtgaaatca tctttaaagg tatcgagcag tcgtattatt 258960
acgaagggta tgaaaaaggc tgagggtcgt ctgaaaagcg atttcagact gtttcagacg 259020
acctgattcg gtaggtgatc agacgggagc ggatgagaaa agaaattctg ggtaagaata 259080
atccggtctg aaatattgga agaagaatga tggataaaaa tcagttagaa caagaatttc 259140
ataaagccat gttaaatatt tatcaggagg ctttgaattt gccgcaacct tacaaggcga 259200
cacgattttt acaaattgta aatgaatttg gtggtaaaga ggcggcggat aaattattga 259260
gtacggggga aaagaagact cagaccggtt ttacagagct gattttgagt ggtggcggag 259320
tccacgcctt gaaatacagt atggaatatc tggtgttaca aaagccgtgg tgtgatttat 259380
ttactgaaga gcaattagct gtggcacgca aacgattgga gcgtgttgga tttgtttttc 259440
cgaagtaatt ttgtacgaaa caaacataga tttttaaatc aatcggattc aatcaaatga 259500
acgatatgac tttatatttg ataattgccc ttgttccgtt ggcaggctcg ctgattgcgg 259560
gtttgttcgg caacaaaatc ggacgtgccg gtgcgcatac ggttacgata ctcggcgtgg 259620
cggtgtccgc cgtgctgtcg gcttatgtgc tgtggggctt tattgacggc agccgcgcca 259680
agtttgacga gaatgtctat acctggctga caatgggcgg cttggatttc tccgtcggct 259740
tcttggtcga tacgatgacg gcgatgatga tggtcgtggt aacgggcgtg tcgttgatgg 259800
tgcatatcta taccatcggc tatatgcacg atgaaaaagt cggctaccaa cgcttcttca 259860
gctatatttc tttgtttaca ttcagtatgt tgatgctgat tatgagcaac aacttcattc 259920
agctcttctt cggttgggaa gcggtgggct tggtgtcgta tctcttgatc ggtttctatt 259980
tcaaacgccc gagcgcgaca tttgccaacc tgaaagcctt tttgatcaac cgtgtcggcg 260040
acttcggctt tttgctcggt atcggcttgg tgcttgccta tttcggcggc agcttgcgct 260100
atcaagatgt attcgcttat ctgcccaacg tgcaaaatgc cactatccaa ctgttccccg 260160
gtgtggaatg gtctttgatt actgtaacct gtttgctcct gtttgtcggt gcgatgggta 260220
aatcggcaca attcccgctg cacgtctggc tgcctgattc gatggaaggc ccgaccccga 260280
tttctgcatt gattcacgcc gcaaccatgg ttaccgccgg tttgtttatg gtgtcgcgta 260340
tgtcgccgat ttatgaaatg agcagcaccg cgctgtcggt cattatggtg atcggcgcga 260400
ttaccgccct gtttatgggc tttttgggcg tgattcaaaa cgacatcaaa cgtgtagttg 260460
cgtattccac cctgtcgcaa ttgggctaca tgaccgtggc tctgggcgcg tctgcctatt 260520
ccgtggcgat gttccatgtg atgacccacg ccttctttaa agccctgttg ttcttggcgg 260580
caggcagcgc gattatcggt atgcaccacg accaagacat gcgccatatg ggcaatctga 260640
aaaaatatat gccggttact tggctgacca tgctgatcgg taacttgtcg ctgattggta 260700
cgccgttctt ctccggcttc tactccaaag attcgattat cgaagcggcg aaatacagca 260760
cactgccggg cagcggcttt gcctattttg ccgtcctcgc cagcgtgttt gttaccgcgt 260820
tttacgcgtt ccgccaatac tttatggtgt tccacggcga agagaaatgg cgcagcctgc 260880
ccgaacacca ttcagacggc cacggcgaag aacatcacgg tttgggtaaa aacgacaatc 260940
cgcacgaaag cccgttggtg gttaccctgc ctttgatttt gcttgccgtt ccgtccgtca 261000
tcatcggcta catcgccatc gaacccatgc tctacggcga tttcttcaaa gacgtgattt 261060
tcgtcaacgc cgacgcgcat ccgactatac acatcatgaa ggaagagttc cacggcgcat 261120
tggcaatggt gtcccacagc ctgcattcgc ccgtactcta ccttgctatc gcaggcgtgt 261180
tgagcgcatg gcttttgtac gtcaaactgc cgcacctgcc agcgaaaatt gcacagacgt 261240
tccgtccgat ttacgttttg tttgaaaaca aatactacct cgacgccctg tatttcaacg 261300
ttttcgccaa aggcacacgc gcattgggca ctttcttctg gaaagtcggc gataccgcca 261360
ttattgacaa cggtattgtc aacggctctg ccaaactggt cggcgcgatt gccgcgcaag 261420

tgcgtaaagc ccaaaccggc tttatctaca cctacgccgc cgctatggtg ttcggcgtat 261480
tggtcttgct cggcatgacc ttctggggat tgttccgata agaataaggt ttcagacggc 261540
cttaaacctt caggccgtct gaaacgaaga aatatccaca taaacacatt tttattttaa 261600
ccacaggtta accactatgt tttccaacta cctactcagc ttggcaatat ggatacccat 261660
cgccgcaggc gtgctggttt tggcaacggg gtcggacagc cgtgcgccgt ttgcccgcgt 261720
gctcgccttc atgggtgcgc ttgccggttt cttggtaaca ctgcccctgt ttaccggttt 261780
cgaccgtttg agcggcggct atcaatttac cgagttccac gagtggattc cgcttctgaa 261840
aatcaactac gcattgggcg tggacggtat ttcagtgctc tttatcatct tgaatgcgtt 261900
tattacgctg ttggtggtat tggcaggttg ggaagtcatt cagaaacgtc cggcgcagta 261960
tatggcggca ttcctgatca tgtcgggttt gattaacggc gcgtttgccg cgcaggatgc 262020
gattctgttt tatgtgttct tcgagggtat gctgattccg ctgtacctga ttatcggtgt 262080
atggggcggt ccgcgccgcg tctatgcgtc ggtcaagctc ttcctctaca cgctgatggg 262140
ttcgctcctg atgctggttg cgatggttta cctttattat caaacaggca gcttctctat 262200
tgtcgatttc caaaacatcg aacagattcc gttgggcgta caacagcttt tgtttgtggc 262260
gttcttcctg tcatttgccg taaaagtgcc gatgttccct gtgcacactt ggttgccgga 262320
tgcccacgtt gaagcgccga ccggcggttc gatggtgttg gcggccatta cgctgaaact 262380
gggtgcgtat ggtttcttgc gctttatcct gccgattatg ccggatgcgg cacgctattt 262440
tgcccccgtg atcatcgtat taagtctgat tgccgtgatt tatatcggta tggtggcttt 262500
ggtgcaaacc gatatgaaaa aactggtggc gtattcgtcc atcagccata tgggttttgt 262560
aacgcttggg atgtttttgt ttgttgacgg gcagttggac gactgggcat tgaaaggtgc 262620
aatcattcaa atgatttcgc acggtttcgt gtctgccgcg atgtttatgt gtatcggcgt 262680
gatgtacgac cgcctgcaca cgcgcaatat tgctgattat ggcggcgtgg tcaatgtgat 262740
gcccaagttt gcggcgttta tgatgctgtt cggtatggcg aacgcgggtt tgcctgcgac 262800
ttccggcttc gtgggcgagt ttatggtgat tatgggcgcg gtcaaagtga atttctgggt 262860
cggcgcgttg gccgccatga ccctgattta cggtgcatct tataccctgt ggatgtacaa 262920
acgcgttatt tttggtgcga tccacaatcc gcacgttgcc gaaatgcaag acatcaattg 262980
ccgcgaattt gcgattttgg caattttggc ggtggctgtt ttgggtatgg gcctgtatcc 263040
gaacgcattt atcgaagtgg tgcatcaggc ggcaaacgat ttgattgccc atgtggcaca 263100
aagcaagatt tgaggtgtgt aaatgaactg gtctgatttg aatttaatgc ccgccatgcc 263160
cgaaatcgtg ctgctgtcgc tgctggtgtt attgttgctg gcggacttgt gggtcagtga 263220
tgacaaacgc ccgtggacgc attacggcgc gttggcaacg gtggcggtta cggctgtggt 263280
gcagttggcg gtgtgggaac agggcagcac gtcttcgttc aacgggatgt atattgcaga 263340
cggtatgtcg cgtttggcaa aaatggtttt atatgccttg acctttgccc tgtttgtcta 263400
tgccaagccc tacaaccaag tgcgcggtat ttttaaaggc gagttttaca ccctgtcatt 263460
gtttgccctg ttgggtatga gtgtgatggt gagcgcgggg catttttaa ctgcctatat 263520
cggtttggaa ctcttgtcgc ttgcccttta cgccctgatt gccctgcgcc gcgattccgg 263580
ctttgccgcc gaagccgcct tgaaatattt tgttttgggc gcgctggcat ccggcctgct 263640
gctctacggt atttctatgg tttacggcgc aaccggttcg ctggaatttg ccggcgtgct 263700
cgcctcttcc ttcaatgaag aagccaacga atggctgttg aaactgggtt tggtgtttat 263760
cgtcgtcgcc gtcgcgttca aactcggtgc ggtgccgttc catatgtggg tgcccgacgt 263820
gtatcacggc gcgcccactt ctgttaccgc cttggtcggc actgccccga aaatcgccgc 263880
cgtcgttttc actttccgca tcctcgttac cgggctggga accgtgcatc atgactggtc 263940
tctgatgttt gccctgcttg ccgccgcctc gctgctggtc ggcaaccttg ccgccatcat 264000
gcagaccaat atcaaacgta tgttcgccta ttccaccgta tcgcatatgg gtttcatcct 264060
gttggcgttt atggcgggcg cggtcggctt tgcggcgggc ctctattacg ccattaccta 264120
cgcgctgatg gcggcggcag ggttcggagt gttgatggtg ttgtcggacg gggacaacga 264180
gtgcgaaaac atcagcgatt tggcagggtt gaaccaacac cgcgtatggc ttgccttttt 264240
gatgctgctg gttatgttct ctatggcggg cattccgccg ctgatgggtt tttacgccaa 264300
attcggcgtg attatggcac tcttgaaaca aggccatgtt tggttgtctg tatttgccgt 264360
catcatgtcg ctgattggtg cgttctacta cctgcgcgtg gtcaaagtca tctacttcga 264420
tgtgcctgat catgaccagc cggtcggcag caactatgcc gccaaatttg ttctgacggt 264480
caatgccttc ttgctgctcc tgtggggcat catgccgcaa accgttatcg actggtgcgc 264540
caaggcgttg gagaacacgc tgtaagccgc cgcaacggca gccgtgtcag aggctgccgt 264600
ttttgttaag atatgccgtt ccgcaacgcg gttcagacgg catcgccgcc gacaacgcct 264660
aaacagaaag cccaccatga ccgcatccat gtacatcctt ttggtcttgg cactcatctt 264720
tgccaacgcc cccttcctca cgaccagact gttcggcgtg gccgcactca agcgcaaaca 264780
tttcggacac cacatgatcg agctggcggc aggtttcgcg ctgaccgccg ttcttgccta 264840
catcctcgaa tcccgtgcag gatcggtaca cgatcagggt tgggagtttt atgccacagt 264900
cgtctgcctg tacctgattt ttgcgtttcc atgttttgtg tggcggtatt tttggcacac 264960
gcgcaacagg gaatagacaa gcataggaat gccgtctgaa accctttcag acggcatttg 265020
tttcattcaa gtgcaggccg gcatcgctgt gccggcacgt ttcagccggc gatatacgcc 265080

ggttttaata tttgcgggcg actgcaaatt ctgccaactg ccgcaggcgc agggctttgt 265140
cgccgaaggg ttcgagcagc gcgaccgctt cggcaaccag tttgtgtgcg tatgagcgcg 265200
ccgcttccaa gcccatcagt ttcacataag tcggcttgtc gttgtctgcg tctttgcccg 265260
ccgttttgcc caaagtcgcc gtgtccgctt cacaatccaa cacatcgtca atgacttgga 265320
acgccagccc cagttttgcc gcgtaagcgt ccaatacgga aagttccgca tctgacagat 265380
caggacacgc cgtcgccccc aataaaaccg ccgcacggat tagcgcaccc gttttcaggc 265440
tgtgcatctg ttccaaatcg gcttgaacca tttgtttgcc gacattcgcc aaatcgattg 265500
cctgaccgcc cgccataccc ctgctgccgc ccgctttcgc caacaccgac aacattgcca 265560
actggcgtgc ggcgggcagt tctgtcggac ggctcaacac gtcaaatgcc tgtgtctgca 265620
aagcgtcgcc ggtcagaagg gcggtcgctt cgccatattt gatgtggcaa gtcggtttgc 265680
cgcgccgcag gctgtcgttg tccatcgccg gcatatcgtc gtgaaccaaa gaatagacgt 265740
ggatcatttc gattgccgcc attgcctgtt ctactgcttc atgcacggct tcgcctaatt 265800
ccgaagctgc cagaaccagc atcggccgca gacgcttacc gccgtccaaa gccgcataac 265860
gcatcgcttc gtgcagtgtg tgcggtattt ccccctcaga cggtaaaaac cgttcaagca 265920
gcagctctgt ttgcgcctgc gccctctgtt gccacgtttt caaatcattc gtcggattca 265980
aggtttaact ccttcagccc gtctgtgtct aaaacctgta gcttttgttc gacttgtgcc 266040
agtttggttt ggcagtacct gaccagttcg ttgccttcct gataggcggc aagcgcgtct 266100
tccaagggca tttcgccctg catagactgc gtcagcgatt cgaggcgcga caaggcttct 266160
tcaaacgatt tcggggcgtt tttcttcatc gtatttcctt ttcggttgaa accccgccct 266220
ttagggcggc aggatcagac tttatttggg aggggtgtaa ccctttccaa atcagggcaa 266280
tacatagggc ggtgctttat gtgccgtcct gtgtgttgga acatagtttc ggatgttccg 266340
gtaaaaagcg gattgtagca tttttgaaaa acggatgccg tctgaaaccc gaatccggct 266400
tcagacggca ttttttccgc ccaggcggca aggcgttacc cgggcagttc gtcggtgatg 266460
ccctgcaaaa aggcgaggcg ttcggggctt gccgccccgg tttgcgcggc ggctttgaag 266520
gcgcagccgg gttcggcgcg gtgggtgcag ttgtggaagc ggcattgccc gacaaggtgg 266580
cggaaatcgg ggaaatagcg cggcaaatcg gcggcttgga ggtggtgtaa accaaattct 266640
tgcaaacccg gggagtcgat gagttgggtt tcgccgttca aatcataaag ccgggcgtgg 266700
gtggtggtgt gttttcccga gtcgagtgcg gcggaaatgt cgccggtgcg ggcggtttgg 266760
ctgcccaaaa gggcgttggt cagggtggat ttgcccatac cgctctgccc gagcaggatg 266820
ttgctgtgcc cttgcagggc ggggcgcagg ctgccggcgt tttccagtgc gcgggtttcg 266880
atgacgggat aacccagcgt ttcgtagaat ttgagttttt cgcgccaaag ggcggtttcg 266940
ggcaggtcgg ctttgttcag gacgatgacg gcttcaatac cggcggcttc ggcggcaagc 267000
agggcgcgtt gcagcagccg cacgctcgga ctcgggacgg cggcggttac gatgaggagt 267060
tgggtaacgt tggcggcgat gagtttggtt ttccacgcgt cttggcggta gagcaggctt 267120
tggcgcggta aaaaatcttc aatcacaact tgttcggcgt tgacggggct gatgcggacg 267180
cggtcgccgc aggcgaaatc gacgcgtttt ttgcgggtgc tggcttcgta ggttgtgccg 267240
tcgggcgtgc ggacaatgta gcggcggccg tagctggcgg taatttgggc ggtgtcgttc 267300
atggtttctt tggggttggg tgtgggaatg ccgtctgaaa acgggtgttc ggacggcatc 267360
ggttcagtcg tgctgccact cgacgtgttc gttgaggaag ccgccgctct ggtgcgccca 267420
gagtttggcg taaagcccgc gtttttcgag gagttcggcg tgtgtgcctt cttcgatgat 267480
gcggcctttg tcgaggacga cgagcctgtc cattgcggcg atggtggaga ggcggtgggc 267540
gatggcgatg acggttttgc cgtccatcat tttgtcgagg ctttcttgga tggcggcttc 267600
gacttcggaa tcgagcgcgc tggtggcttc gtccaaaaga agaatcggtg cgtctttgag 267660
catcacgcgg gcgatggcga tgcgctggcg ttgcccgccg gagagtttca cgccgcgttc 267720
gccgacgtgt gcgtcgtagc cgcgccgccc tttggcatcg gaaaggtcgg ggatgaagcc 267780
ggcggcttcg gcgcgttcgg cggcagaaac catttcggca tcggtcgcgt cggggcggcc 267840
gtaaataatg ttgtcgcgca cggaacggtg cagcagcgag gtatcttgcg tgaccaaacc 267900
gatttgggcg cgtaaagatt cttgggtaac gccgcttatg tcctgcccgt cgatcgaaac 267960
cgtgccgctt tgcggttcgt agaagcgcaa aagcaggttg acgatggtgg atttgcccgc 268020
gccgctgcgt ccgatcaagc cgactttttc gcccgggcgg atggtgaggt tgaagccgtt 268080
gagcagcggt ttgcccgctt cgtaggagaa atcgacgtgt tcaaatttga ttgcgccttg 268140
cggcacgttc agcggcagtg cccggggctt gtcgaggatg gtgtgcggtt tggacagggt 268200
tgccatgccg tcgccgacgg tgccgatgtt ttcaaacagc cgcgcggatt cccacataat 268260
gtattgcgac aaaccgttga cgcgcaacgc catggcggtg gctgtagcaa ccgcgcccac 268320
gccgacctgc ccgttgtgcc agagccagat gcccagtgcg gcggtggaga gggtcaggga 268380
ggtgttgacg atgaagctgc acgaatgcag cagcgtcgcc agccgcattt gggcgcgcac 268440
cgtaaccata aattcttcca tcgactgctt ggcataggcg gcttcacgcg cgccgtggga 268500
gaagagtttg acggtggcga tattggaata ggcatcggta atgcggccgg tcatcagcga 268560
gcgggcatcc gcctgccatg cggcggtttg ccccaatttg ggaatcagca ggcgcatcac 268620
cgaagcgaaa ccgacaatcc agccgataaa gggcagcagc agccatgagt cgagcgaggc 268680
gagaatcacg ccggaggtaa tgaaatacac cgacacataa acgaccatat cggcaaccgt 268740

catcaccgcg tcgcgcaacg ccagcgcggt ctgcatgact ttggcggaca cgcgtccggc 268800
aaattcgtcc tgataaaaac cgaggctttg gttcagcatc aggcggtgga agttccagcg 268860
caggcgcatg gggaacacgc cctgaagggt ttgcaggcgc acgttggacg cggcaaacgc 268920
ccacgcaacc gaaaatacca tcatcgccgc cattgccgcc agttcccaac ttttttcggc 268980
aaacagttcg gcgggcgcgt atttgccgag ccactccacg attttgccca taaattgaaa 269040
aaccagggct tccataatgc cgatgccggc ggtcagcgca gccagggcgg ctatccattt 269100
ccgcacgccg gccatgctgc tccagacaaa ccgccacaag cctttttctg gcgttttcgg 269160
ggcggcttcg ggataagggt cgattcggga ctcgaaccag gaaaatattt tgttcaacat 269220
tgttttcgat ttcggtaaaa cagtttcaga cggcatcaaa cacaatgccg tctgaaagga 269280
aggacaataa cgccatttta cgggaaaagc cgtcgggaag acagcgcgag gcggaaacgc 269340
agggtttcgt cagggcaaac gccgcgccgc cttcaggcgg cattatttca gcaggttttt 269400
caaagcaagg cgcacgcctt cgcccacgtc cgtcccctcc ggaacgcctt tgaccgccgc 269460
ttttgcttcg cgttcgctgt aacccagcgc aagcagcgtg ctgacgatgt cttccgtttc 269520
gtcggcggcg ggtgcggcgg caaacagccc gtccgttacc gtatgcgcga ccagcttgcc 269580
gcgcagttcc aaaaccatac gttcggcggt ttttttgccg attcccgggg cggaggagag 269640
gcgtttgaca tcttcttctg caaccgcccg cgccagttcg tcggcagtca ttgccgacaa 269700
aatgcccaaa gccgttttcg cgccgatgcc gccgaccttg atcagttggc ggaaggtctt 269760
gcgttcttcc gcagtggcaa aaccaaataa aagatgtgcg tcttcccgaa tgataagctg 269820
ggtaaacagt tgtacgcttt cacccacggg cggcaggttg tagaaggtct gcatcgatac 269880
gtcggcctca tagccgacac cgttgacatc gatgacgatt tgcggagggt ttttttcaac 269940
cagtttgccg gtcagtctgc tgatcatgtg tgccgaatcc tgaagtgtcg ggtgcaaaat 270000
gccgtctgaa accggtttgg gcttcagacg gcacggattg tatcaaattc agtcgtcgcg 270060
gcgggaggaa atcacgcggc cggtacgggc atcgacaacg actttgtatt cctgtccgtt 270120
tttgacgatt tcgacatcat agtgcggacg gccgttgtcg tgttcgagat cgatgtcggt 270180
gattttgccg ccgacacgcg ccaacgctgc tttttcggct tgggcgcggc tgatgatttt 270240
gtcttgtttg ttgtgttggt gtgcggcgtg tccgtggtcg tcatcgccgt gtccgtcgtg 270300
gtgggcgagc gcgggggcgg aaatgctcag cagtgcggtt gcggcggagg tcaagagaag 270360
gtgtttgatg ttcatatttt gcctttgtaa atcgtgggtt ggaaaatgtg gatattaata 270420
aggtatcaaa taaccgtcag ccggcggtca ataccgcccg aaccataccg cgcgcctgag 270480
cttcggcttc ggcggcgcgt tcctgcgagg taaacggtcc cattttgacg acgtattcgt 270540
aacggcgttt ttcaaccgag aggttcgtac ccgatgacga aacggcgaag ttttgggcgg 270600
cttggttcag ataggcttgt gcttcgtgtt ccgtaccgaa agatttcaag tcgataaaga 270660
tgtctttgtt ttcggcaacc ggtgcggatt ggcccgggac gatttgttcg attttgacgt 270720
gtgccgtccc ttggttgaca aagcccaatt tttgcgcggc ggctttggat acgtcgatga 270780
tgcggttgcc gtggaagggg ccgcggtcgt tgacgcggac gatgacgctt ttgccgtttt 270840
tggtattggt tacgcgcaca tagctgggga tgggcagggt tttgtgggcg gcggtaaagg 270900
cgttcatatc gtatcgttct ccgccggaag ttttgcgccc gtgaaacctg ccgccgtacc 270960
acgaggcgtt gccggtttgc gtgaattcgg cgacttggtt tttcggcgtg tagcgttttc 271020
cggcgacttt gtagctgcgg ttggcggagg cgtgcagttt ttctgccttg accactgcgt 271080
cggcggatgc cgtctgaagg gagtgtgtgc cgaatgcggc ggtgagaagg aaaagggttt 271140
ttcgggttaa agtcaaaacg tgttccgttc ttgagttgaa gacgaatggg catcatgccc 271200
gccggatacg ttccgaaccg ccgtacagtg cggacggcgg ttcggaatgt gtccggatag 271260
gttttcagac ggcatgaacc tgcgttcaaa cgccgcctgc gtaaccgtgt tgccgccacg 271320
cttcaaagag aatcacggcg acggtgttgg aaaggttcat actccggctg ccgggctgca 271380
tcggcaggcg gattttttgc gcggcgggca ggctgtcgag gatgtcggca ggcagtccgc 271440
gcgtttccgg cccgaacagt aaaacgtcgc ctttttgaaa cgcggtttca tcggggcgcg 271500
ccgtgccttt ggtggtcagg gcgaaaatgc gcctgcctgc gagtgccttg aggcagtcgt 271560
cgaagttttc gtgcaccgtc aggctggcga actcgtggta gtcgagcccg gcgcgtttca 271620
ttttggcgga atccaatggg aagccgagcg gtttgacaag gtgcaaatcc gcgccggtat 271680
tggcgcacag gcggatgatg ttgcccgtgt tcggcgggat ttccggctgg tataaaacga 271740
tggtaaacat aaatatcaat cacttatagg cgcgtaacct tgccacaagg cggatggggt 271800
gtcaaaaaat ttagttattt tttcattggc gtgcgtgcca gcgtccagca gcagattcgg 271860
tttgcgcccg attttttcag cgtctttgcc aattcgtcca gcgtcgcgcc ggtggtaaag 271920
acatcgtcga ttaacagaat attacagttt tccggtatcg gtgtgcggat ttcaaaggcg 271980
tttttgatgt ttcgccgccg ttcgccgcct ttgagcgtgc tttgcggcgg gcggtggtgt 272040
cggaaaacgg tgtgtcgggg cagtatctgc cagccgtagc gttgtgccag cagcccgacg 272100
atgctttcac tttggttgaa cccgcgttgc agcagccgct ccctgcttag cggtacgggc 272160
aggacgaaat cgaaacattc gtctgcaagc cggtcgggcg gattctgcat catcaggtct 272220
gccagcggct gcaccatgct caaatcagcc aagtgcttca gcgcgtgtat catattgctg 272280
acgggcggtt cgtaatgcag cgaagcccac atccggtcga atgcgggcgg tttttctga 272340
cagccgccgc acaccgatcc gccttggatg tgtctgaaac acagggggca gctgtttgcc 272400

gcgtcggtgc ggtatgccgc caaatcgtcg cggcagccgg cgcagatgcc gtctgaaacg 272460
ccagacgaac cgtggcataa tacgcaacgc ctgatagtgg gcgcgtctgc gatgcgccgc 272520
caacgagaga gaaaatccat gcctgatgcc gtcaaaaaag tttacctgat acacggttgg 272580
ggggcgaacc gccacatgtt cgacgatttg atgccgcgcc tgcctgcaac gtggccggtg 272640
tccgccgtcg atttgcccgg acacggggac gctccgtttg tccgaccttt cgacattgcg 272700
gctgcggccg acggcattgc cgctcaaatt gacgctccgg ccgacattct cggctggtcg 272760
ctcggcggat tggtcgcgct gtatctggcg gcgcgccatc ccgacaaagt ccgttcgctc 272820
tgcctgacgg cgagtttcgc acggctgacg gctgacgaag actatcccga agggcttgcc 272880
gcgcctgcat tgggcaaaat ggtcggtgcg ttccgttcgg attatgccaa acatatcaaa 272940
cagtttctac aattacagct tctgcacacg cctgatgcgg acggaatcat aggcagaatc 273000
ctgcccgatt tggcgcgctg cggcacgcct caagccttgc aggaggcgtt ggacgcggcg 273060
gaaagggcgg atgcgcggca tttgttggac aagatagatg ttccggtact gctggtgttc 273120
ggcggcaaag acgcgattac gccgccgcgt atgggtgaat atctgcaccg ccgtttgaag 273180
ggcagcaggt tggttgtgat ggaaaaggcg gcgcatgcgc cgtttttgag ccatgcggaa 273240
gcgtttgccg cgctgtaccg cgactttgtt gaaggggggtt tgagatgaac catcaggacg 273300
cacgctggca ggttcaccgc catcttgccg aacataccga ccaacggctg acactcgtcc 273360
gcaacgcgcc caagcatatc ctgcttgccg gtgcggatgc ggacatcagc cgcagcctgc 273420
tggcgaaacg ctatccgcag gcggtatttg aagaatacga ttcccgtgcg gattttttgg 273480
cggctgccgc tgccgcccgc aaaggcggtt tttggcaaag gtttacgggt aagggcgtgg 273540
tgcaacactg ccaatccccg atcgcgccgc tgcccgaagc gtgtgccgat atgttgtggt 273600
cgaatctcgg actgttggcg gcggaacaaa tccttcctgt gctgcacaac tgggcgcgcg 273660
ccttgaagac ggacgggctg ctgtttttta cctgcttcgg gcgagatacc ttggcggaac 273720
tgaaatgccg tctgaaagaa aacggcattg aaagccgcag cgcgcttttc cctgatatgc 273780
acgacttggg cgatatgctt gctgaaaacg gcttttacga ccccgttacc gatacggcga 273840
agctggtgtt ggattacaaa aaggcggaaa cgttttgggc ggatatggac acgctgggcg 273900
tttggcgggc gatggcgtgg aacgatgaaa acgccgcgcg ttcgtgtgtc gggacaatat 273960
ttgagcggga aggcggtttg ggcattacgc tggaaacggt gtacggacac gccgtgaaaa 274020
aactgatgct gccgcaaggg gagaacgtgg tgcagttttt tccgaagaga tgatgtgcag 274080
atgccgtctg aagccgtttc caggtttcag acggcatttg tctgtgaaaa ccgacagaaa 274140
taaaggaaat gccgatgtat agtgaattaa atttaaacca gtacagcgtt gcctcgcctt 274200
agctcaaaga gaacgattct ctaaggtgct gaagcaccaa gtgaatcggt tccgtactat 274260
ttgtactgtc tgcggcttcg ccgccttgtc ctgatttttg ttaatccact atatgctgat 274320
gccggagacg tatattgcgt ctataacatc agactgaagc agtacactgc ctgccaggtt 274380
acccgagttg aagaacacgg tggcaaaaaa aacacatgcg accctgctgg ctttggactg 274440
gcagggcaac aaaccgcttg gggcggagga gctggcggat ttgaaatcgc tttacaaaga 274500
cttaaagaat aatattggaa atattgtatg aacaaaaaat taaactatat ttttatgttg 274560
gactgtttag ggttggtgat attgtttact tgtataatag ctacttttga aagagattat 274620
ggatttaaaa tttttactaa ttctaagaga cctgaatttt attattggat tggaatgttt 274680
tattatggaa ttatttcttg ctggtttgat tatcaattaa tttcaacaaa ggcgaattcg 274740
tataaaagaa aagttaaaca atataaaatt ttttcagtaa tattttcagt tttgatattt 274800
atttctacta tagtaaaact ttaaattttg gagcaaaaat ttatgagcga ttcaattgaa 274860
tatgtattgg gaacgcggtc tgcacatgta taaggcaagt gccgtcgtgc cgacgggata 274920
tgtacgggtt gggaataccg cgccgctggt cggcgaagac acgcaacggt atgcctcttt 274980
ttggggcgac ggctacgacg tgtaccgtca gttgagatgg cagcagatac ccgaaaaaca 275040
gagaaaggca ttcaaaaaag ccgccaaaag caaaaagacc gtgatgtttg ccggacggga 275100
atacggcata tccaaacaga atttgagcga tgtttgggat gattttgaag acgcgatgga 275160
actgaaggcg tttccctgcc tgtcttcgct gtttctgacc aaatggcata aaaatctata 275220
tgatagtgga ttaacaaaaa ccagtacggc gttgcctcgc cttagctcaa agagaacgat 275280
tctctaaggt gctgaagcac caagtgaatc ggttccgtac tatttgtact gtctgcggct 275340
cgccgccttg tcctgatttt tgttaatcca ctataaaaac aggaattttt aaatagaggc 275400
aatgccgtct gaaacttggt aacgggcttc agacggcatt tcgttccaat accgccaaca 275460
ccgccgcacc gtaacgtgcg gctttttctt cgcctacgcc gtatacggcg gcaagctccg 275520
ccaagccttc cggctgtttg gcggcaatgg cgcgcagtgc ggctttgctg agaatgcggt 275580
agggttcgga ctgttcgtgt tttgccgttt cgccgcacca ttggatcagg gcgcgcatca 275640
ggcggcgttt gcgtttggcg gtttcatcga tgccgtctga aaacggacgg cagacggcga 275700
ggatgtcccg tccgtatttg gcggcgcgta cgctgcccaa gccgtacacg ccttcgaggt 275760
cggtttcggt ttcgggcgta tcggcaagca tatcggcaag gctttcgtcg gagaggacgg 275820
catgcagggc gcagttttcc gcccttgcct gttcataccg ccaggcttcg agtttttgac 275880
gcagttgttg ttcgcgttcg gtttgcggac ggatgaccgc gtcgcggctg aagccggcgg 275940
cgttgcggca gacttcgagg atgccgtgtc cgaaacggtc gattttggct tcgcccaaac 276000
cgtagatgtc gtgcagaccg ttgaggtctt gcggcatttt ttcgacaagg tcgcgcaggg 276060

ttttgtcgcc gaaaatcata taggcgggga tgccttcggc ttctgcctgt ttcatacgcc 276120
aaacgcgcaa tgcctgccac aggcgttctt cgcgttcggt acgcagccag ttgtctttga 276180
gggtgcgggc ggcgggcttg tcgcgcttga gcggacgcag catcacttcg gtttcgcctt 276240
tgaggacttt tttggcggct tcggtcagtt gcaatgcctg atatcgggta atgttgacgg 276300
tgaggtagcc gaggctgata cactggcgga tgacgctgcg ccattctttg tcggacaact 276360
ccgtaccgat gccgaatgtg gacagttgtt cgtgccggtt gccgcgtatc caatcgtcgc 276420
ttttacctcg taaaatgttg gtgatgtaac cggcggcaaa acgttgtccg gcgcggtaca 276480
cgcagctgag taatttttgc accaacaccg tgccgtcaaa ccgtacgggc ggatgcaggc 276540
agttgtcgca atggccgcag ggttcggatg cttcgccgaa atgtttgagc agcagtacgc 276600
ggcggcaggc ggcggtttcg cagacggcaa gcatggcatc gagtttttgc atttcgattt 276660
gcttttgcac ctcgtcgctg ttgccttcgg caatccgttc gcgcagcaac acccaatcgt 276720
tcaaaccgta acacagccag cttgcggccg gcagcccgtc ccgtccggcg cgccccgatt 276780
cttgatagaa atgttcgaca ctctggggca tatcgagatg ggcgacaaag cgcacgtcgg 276840
gtttgtctat gcccatgccg aacgccacgg tcgccaccac gataatattg tcttcatgcg 276900
taaagcggcg ttggttttcc tcgcgtacgt ccatgctcaa accagcatga tacggaatcg 276960
cgtttaatcc gttttcacgc aaaaactgcg ccacatcttc cacctttttg cggcttaggc 277020
aatacacaat gccgctttgc cccgtcattt ctttgcggat gaaatccagc aattgttttt 277080
tgccgttgtt tttttcgata acctgataat aaatattcgg acggtcaaag ctggagacaa 277140
attcgggcgc atcgtccaag tgcagataat gcttgatgtc ggcgcgcgtg gcggcatcgg 277200
cggtagcggt cagagcgatg cgcgggacgt tcggatagcg ttcggcaagc atgccgagct 277260
gttgatattc agggcggaaa tcgtgtcccc attggctgac gcaatgcgcc tcatcaatgg 277320
caaacagact gacggtttgt tggtcgagaa aacgcaaaaa gcggtcggta accaagcgtt 277380
ccggcgcgac ataaagcagc ttcagacggc cttgggcaag ccggtcggca atctcgcgcg 277440
cctcgtctgc cgatgtgccg ctgttgactg ccgccgcttc gatgccggcg gcgtgcaggt 277500
ttgccacttg gtcgttcatc agcgcaatca gcggcgatac gacaaccgcc acgccttcgc 277560
gcatcagcgc gggaatctgg taacacaaag acttgccacc gcccgtcggc atcagcaccg 277620
tcaaactccc gccgcctgcc aaagtattga tgacagcctc ctgcctgccg cgaaattcgg 277680
gataaccaaa tacttcgtgc agaatctgtt tggcggtcgg tcggtgcatg atggttccgt 277740
gctcggtaag ggtgttgatc ggtcggcggc aatatgccgt ctgaaatcgg gatttagaat 277800
agtttgccca cttctgcttc aatatcgtcg gcacgcataa acgtttcgcc gatcaggaag 277860
gtatgcacgc cgcgcgattg cataaattcc acatccgcct tgcctgtaat gccgctttcg 277920
gtaacgacgg ttttgccttc cagcgcgggc agcagcgaca gggtttggtc gagggagact 277980
tcaaaagtcc tcaggttgcg gttgtttacg ccccacagcg gcgtggtcag gttgcggcat 278040
ttttccaatt cggtttcgtc gtgcagctcg agtaggacgg tcatgcccaa ttcgtgcgcc 278100
accgcttcaa agcgttccaa ttgttcctgt tccagtgctg cggcaatcag caggacggca 278160
tccgcccccc atgcgcgcgc ctgataaacc tggtattcgt cgatgatgaa gtctttgcgc 278220
agcacgggca gcgatacggc ttcgcgcgcc tgtttgaggt attcgggcga accttggaaa 278280
tagggttcgt cggtcagtac ggacaaacac gccgctccgg cgttttcata ggcgcgtgca 278340
atctcggcag ggcggaagtc cggacggatt aaccctttgc tcgggcttgc ctttttgatt 278400
tcggctatga cggcgggcag gtttaggcgg tgtttgccgc gtatcgaatc gatgaagctg 278460
cggacgggcg cggcttctgc ggcaagtgtg cggatgtgtt cggcgttgac ggcggctttt 278520
tgagcggcaa cttcctgtgc tttggtggca aggattttat tgaggatgtc ggtcatgtcg 278580
ggttccgtat tcgtctgggg aaagggggaa tattagcatc aaaccgttaa cgcctgtttg 278640
tgcggaagct gtcgaaatag gacaggacgg tctgcggcag ccattgcagg tgcagcctgc 278700
cgccggtgct gctgacaaag ccgacatgac caccatatgc cggctggaac agggtaacgg 278760
cttcggatac ttcgtctgcg cggggcaggg cttcgggcgg caggaagggg tcgttgacgg 278820
cattgagcag gagcagcggt ttggcaacgt gtttgagcag cggtttgcag gaagtttggc 278880
ggtagtagtc gtgccggtcg gcaaagccgt gcagcggtgc ggtgaagcgg tcgtcaaact 278940
cgcccagtgt tttgcaccct gcggcaaatg ccgtctgaaa accttggagc gattttgctt 279000
tgggtatcag ggtgcggagg aagtagcgcg tgtagagcag ccgcgtgatg ccgctgtcga 279060
agcgtctgcc tgccgcctct gcatcgacgg gggcggagat gacggcagcg gcttgcggca 279120
atgccttttt gccctgttcg cccaaatatt ttgccagcgc gttgccgccc agcgatacgc 279180
cgacggcgta tatttcacgg taacgcgcgg cgaacgtgtc caaagtaaag gcgatttcgg 279240
cggtatcgcc caagtggtag aacaccggag cggtgttggc aatgccgccg cagctgcgga 279300
aatggacgac tacgccgtgc caaccccgat cgcgtaccgc aagcatcagt tcgaccgcgt 279360
aatggctgcg gctgcttcct tccaaaccgt gaaacagcac gaccagcggc gcatcgggcg 279420
aaatgccgtc tgaaaagtcg taggcgactt tggttttacc cgtgctgtcg ggaagcagct 279480
ctcggcggta tgcgggcgcg gggcgttgca ggaatttggc ggcaatcgtg tcggcattgc 279540
cgttgcggag gaaaaagggc gtgtccggcg gtgttaaaat cataaggtat cggttttctt 279600
gttttcagac ggcattgatg atgcggcagc ccgtccggct ggtgcggacg tggggggatgc 279660
gcgcccgaat ataggcgtgg aaaagcgttt gccgaaaaag gatatcggca tcggtcagtt 279720

ttccacgcgt ttgaaatggc gcggacggaa gcccaaagcc gccagtgatg cgaaatacag 279780
tccgccgccg acggcaatca ggatgcagag ctgccccgct ttccgcattc cgccggcgtg 279840
cgcccattca aacggcaggt aagcctgcgc tgcccacagt ccgccgcaca tcacggcgag 279900
cgagagcagc atttttgcta agaacgctgc ccaacccttg ccaggttggt aaataccgtg 279960
tctgcgcaac aggtaaaaca acaatccggc attgatacac gcgcccagac cgatggcaag 280020
cgaaagtccg acgtgtttca gtgggccgat aaaggcaagg ttcatcaact gcgtgcagat 280080
gagcgtgaag atggcgattt tgacgggcgt tttgatgttt tgccgcgcat agaagccggg 280140
tgccaacact ttaatcatga ttaagccgat taaaccgaaa gaataggcaa tcagcgcgtg 280200
ttgcgtcatc tgcgcgtcaa acagcgtaaa ttcgcggtac ataaacagcg tcgccaccag 280260
cgggaacgac aacaccgcca gtccgaccgc cgccggcagc gtcagcagca tgcacaggcg 280320
caaaccccag tcgagcaggg cggaaaactg ttccgtatct tggtttgccg agtgtttgga 280380
caaagtcggc agcaaaatcg taccgagtgc cgcccccagc acgccgctgg gcagctccat 280440
catgcggtcg gcgtaataca tccatgaaac gctgcccgat tgcagataag acgcgaaaat 280500
cgtgttgatc accaaagaaa cctgcgccac gctcacgccc aaaatcgcag gcgccatctg 280560
tttcatcacg cggttgaccg ccgcatcttt gaaactcagt ttgggcagtt tcaaaaagcc 280620
cagtttcgcc agccagggca gttggaagcc gagttgcaaa atgccgccga caaagaccgc 280680
ccacgccagc gcggtaacgg gcggatcgaa atacggcacg aaaaacagcg cgaatacgat 280740
aaacgacacg ttcagaaacg tgggcgtaaa cgccggaatg ccgaacttat gataagaatt 280800
gagtaccgag ccgacaaatg aagacaggga aatcaataat atataaggaa acgtaatccg 280860
cagcaaatcg atggagagct gaaatttgtc ggcatcttgg gcaaaaccgg gtgcggaaac 280920
ataaatcacc caaggcgcgg caagtatgcc cagcgcggta acgataacca gtacaaacga 280980
cagcatcccc gccacatggc ggataaaagc ctccgccgcc tcttttgaac gcgtttcctt 281040
gtattccgcc aaaatcggca caaacgcttg ggcaaacgcc ccctccgcaa acacgcggcg 281100
aagcaggttg ggcagtttga acgcgacaaa aaacgcatcc gtcgccatac ccgcgccgaa 281160
tgcccgcgca atgaccgtat cgcgcacaaa tcccaaaacg cgcgacacca tcgtcaggct 281220
gccgactttt gccaaagctc ccagcatatt catcattgtt cctcaacagt cgtacccgtc 281280
tggggcaacg gcgcgtattg tacgacagaa accgcttcag acggcatcgg gtttgatgcc 281340
gtctgaagcg gtttcctgaa acgaaaacgt ccttttccgg cggcaaactg tatcaatacg 281400
cggaaatgca ataaaatagc cggattccga ttgatttcca acatctgttt ccaacatcac 281460
ggagaaccgt atgaaatcca gacaccttgc cctcggcgtt gccgccctgt tcgcccttgc 281520
cgcgtgcgac agcaaagtcc aaaccagcgt ccccgccgac agcgcgcctg ccgcttcggc 281580
agccgccgcc ccggcagggc tggtcgaagg gcaaaactat accgtccttg ccaacccgat 281640
tccccaacag caggcaggca aagtcgaagt ccttgagttt ttcggctatt tctgtccgca 281700
ctgcgcccac ctcgaacctg ttttaagcaa acacgccaag tcttttaaag acgatatgta 281760
cctgcgtacc gaacacgtcg tctggcagaa agaaatgctg acgctggcac gcctcgccgc 281820
cgccgtcgat atggctgccg ccgacagcaa agatgtggcg aacagccata ttttcgatgc 281880
gatggtcaac caaaaaatca agctgcaaaa tccggaagtc ctcaaaaaat ggctgggcga 281940
acaaaccgcc tttgacggca aaaaagtcct tgccgcctac gagtcccccg aaagccaggc 282000
gcgcgccgac aaaatgcagg agctgaccga aaccttccaa atcgacggta cgcccacggt 282060
tatcgtcggc ggtaaatata aagttgaatt tgccgactgg gagtccggta tgaacaccat 282120
cgaccttttg gcggacaaag tacgcgaaga acaaaaagcc gcgcagtaag cccgtttgaa 282180
aaatgccgtc tgaaacttgg ttttcagacg gcattttgat tgggtttaaa acgtaaagcc 282240
cgtttccagt tcttcatcgc cgaccagttc gaccaagagc gcgtagagcg gggcgagttc 282300
ggcataacgg cgcgatacgc ggcgcagata gtttaagaaa cgcgggattt ccggacggta 282360
tttgtctttg ccgtcgcggt agtacaggcg tgcgaagatg cctgcaacct tcaagtgccg 282420
ctgcacgccc atccattcga accagcggta aaactcgtca aacgcttcgg ggacgggcaa 282480
gccggcagcc cgcgcctttt cccagtagcg gataaccaag tccaagacaa attcttcttc 282540
ccattcgata aaggcatcgc gcaacagcga caccaaatcg taggaaatcg ggccgtaaag 282600
cgcgtcttgg aagtctaaaa cgcccggcct gccgcgcgtc agcatcaggt tgcggacgat 282660
aaagtcgcgg tgcacataga ctttgggctg cgccaacagg ggcggcagca gcgtatcgac 282720
ggtttgctgc caaagttggc gttgtttgaa tgttaattcg cgccccaatt cttttgcgac 282780
aaaccattcc gggaacaggt tgatttcgcg caacatcgtt tcacggtcat attcgggcaa 282840
aaccccttca cggctcgcct tctgcaattc gaccaactcg ccgattgcct ccaaaagcag 282900
ggctttgtgc gccgtttcgc cctgttcctg aagcattgcg gtcaaaaacg tcgtattgcc 282960
caagtcgttc aataccacaa accccagatc cgtgtccgcg tgcaatacct gcggcacatt 283020
gaccatgtca aacagtttct gcactttcaa ataaggtgcg acactcatct tgtcgggcgg 283080
tgcatccatg cagacgacac tgctgccgtc tgaaaacgtt gcacggaaat agcggcggaa 283140
atcagcatcc gccgccgcaa aagtcagatc gaagtcccgt tcgggataaa cggtctgaag 283200
ccaatttttc agtttgattt gtcgttgcat aacagtacta aagcatttca ggttacaata 283260
aacgctattc taactggcaa accgacttga ggggcgattt tggctcgttt attttcactc 283320
aaaccactgg tgctggcatt gggcctctgc ttcggcacgc attgcgccgc cgccgatgcc 283380

gttgcggcgg aggaaacgga caatccgacc gccggagaaa gcgttcggag cgtgtccgaa 283440
cccatacagc ctaccagcct gagcctcggt tcgacctgcc tgttttgcag taacgaaagc 283500
ggcagccccg agagaaccga agccgccgtc caaggcagcg gcgaagcatc catccccgaa 283560
gactatacgc gcattgttgc cgacaggatg gaaggacagt cgcaggtgca ggtgcgtgcc 283620
gaaggcaacg tcgtcgtcga acgcaaccgg acgaccctca ataccgattg ggcggattac 283680
gaccagtcgg gcgacaccgt taccgcaggc gaccggttcg ccctccaaca ggacggtacg 283740
ctgattcggg gcgaaaccct gacctacaat ctcgagcagc agaccgggga agcgcacaac 283800
gtccgcatgg aaatcgaaca aggcggacgg cggctgcaaa gcgtcagccg caccgccgaa 283860
atgttgggcg aagggcatta caaactgacg gaaacccaat tcaacacctg ttccgccggc 283920
gatgccggct ggtatgtcaa ggcagcctct gtcgaagccg atcgggaaaa aggcataggc 283980
gttgccaaac acgccgcctt cgtgttcggc ggcgttccca ttttctacac cccttgggcg 284040
gacttcccgc ttgacggcaa ccgcaaaagc ggcctgcttg ttccctcact gtccgccggt 284100
tcggacggcg tttccctttc cgttccctat tatttcaacc ttgcccccaa tctcgatgcc 284160
acgttcgcgc ccagcgtgat cggcgaacgc ggcgcggtct ttgacgggca ggtacgctac 284220
ctgcggccgg attatgccgg ccagtccgac ctgacctggc tgccgcacga caagaaaagc 284280
ggcaggaata accgctatca ggcgaaatgg cagcatcggc acgacatttc cgacacgctt 284340
caggcgggtg tcgatttcaa ccaagtctcc gacagcggct actaccgcga cttttacggc 284400
aacaaagaaa tcgccggcaa cgtcaacctc aaccgccgtg tatggctgga ttatggcggc 284460
agggcggcgg gcggcagcct gaatgccggc ctttcggttc tgaaatacca gacgctggca 284520
aaccaaagcg gctacaaaga caaaccgtat gccctcatgc cgcgcctttc ggtcgagtgg 284580
cgtaaaaaca ccggcagggc gcaaatcggc gtgtccgcac aatttacccg attcagccac 284640
gacagccgcc aagacggcag ccgcctggtc gtctatcccg acatcaaatg ggatttcagc 284700
aacagctggg gctatgtccg tcccaaactc ggactgcacg ccacctatta cagcctcaac 284760
cgcttcggca gccaagaagc ccgacgcgtc agccgcactc tgcccattgt caacatcgac 284820
agcggcgcaa cttttgagcg gaatacgcgg atgttcggcg gagaagtcct gcaaaccctc 284880
gagccgcgcc tgttctacaa ctatattcct gccaaatccc aaaacgacct gcccaatttc 284940
gattcgtcgg aaagcagctt cggctacggg cagctctttc gcgaaaacct ctattacggc 285000
aacgacagga ttaacaccgc aaacagcctt tccgccgccg tgcaaagccg tattttggac 285060
ggcgcgacgg gggaagagcg tttccgcgcc ggcatcggtc agaaattcta tttcaaggat 285120
gatgcggtga tgcttgacgg cagcgtcggc aaaaaaccgc gcaaccgttc cgactgggtg 285180
gcatttgcct ccggcagcat cggcagccgc ttcatcctcg acagcagcat ccactacaac 285240
caaaacgaca aacgcgccga gaactacgcc gtcggtgcaa gctaccgtcc cgcacagggc 285300
aaagtgctga acgcccgcta caaatacggg cgcaacgaaa aaatctacct gaagtccgac 285360
ggttcctatt tttacgacaa actcagccag ctcgacctgt ccgcacaatg gccgctgacg 285420
cgcaacctgt cggccgtcgt ccgttacaac tacggttttg aagccaaaaa accgatagag 285480
gtgctggcgg gtgcggaata caaaagcagt tgcggctgct ggggcgcggg cgtgtacgcc 285540
caacgctacg ttaccggcga aaacacctac aaaaacgctg tctttttctc acttcagttg 285600
aaagacctca gcagtgtcgg cagaaacccc gcagacagga tggatgtcgc cgttcccggc 285660
tatatcaccg cccactctct ttccgccgga cgcaacaaac gaccctgacc gtcggaaacc 285720
tggcaggagc accgttcccg cacaagacgg cattccaccg acaaccccaa acccgccatc 285780
aaaggcagga ttcaaacgat aaggaaagaa tgatgaaaat caaagccctg atgattgccg 285840
ccgcattgct ggcagcagcc gatgtccacg ccgcaccgca aaaggcaaaa accgcatccg 285900
ccaaagctgc caaagctgcc aaagctgcca aagttgccaa agttgccaaa gttgccaaag 285960
ttgccgccac ggcgcaaaaa gaagccgcac ccgcacaaca gcagggcggt atccgctttt 286020
cagacggcat tgccgccgtt gccgacaacg aagtcatcac gcgccgccgg cttgccgaag 286080
ccgttgccga agccaaagcc aacctgccca aagacgcgca gataagcgaa tccgagctgt 286140
cccgacaggt gctgatgcag cttgtcaacc aatccctgat tgtacaggcg ggcaaacgcc 286200
gcaacattca agcaagcgaa gcggaaatcg atgccgtcgt cgcaaaaaat cccgccctca 286260
aaaacctcag ccccgcccaa cgccgcgatt ttgccgacaa catcattgcc gaaaaagtcc 286320
gccagcaggc agtgatgcag aacagccgcg tgagcgaagc tgaaatcgat gccttcctcg 286380
agcaggcgca aaaacaaggc atcaccctgc ccgaaggcgc accgttgcgc caataccgcg 286440
cccaacacat cctgattaaa gccgacagcg aaaacgccgc cgtcggcgcg gaaagcacca 286500
tccgcaaaat ctacggagag gcccgcagcg gcacagactt ttccagcctg gcgcgccaat 286560
attcgcaaga cgcgagcgcg ggcaacggcg gagatttggg ctggtttgcc gacggcgtga 286620
tggttcccgc ctttgaagaa gccgtccacg cgctcaaacc cggacaggtc ggcgcgcccg 286680
tccgcaccca attcggctgg catatcatca aattgaacga agtgcgcgat gccggcacac 286740
ctcaggaacg tatccgcaat tccgtgcggc aatacatctt ccaacaaaaa gccgaacagg 286800
caaccgtcaa cctgttgcgt gacctgcatt ccggcgcgta tgtcgacatc cgctaaggcg 286860
gtttgaagca aaaagccata ccgatcggca aaaatccggg cggtatggct ttttggattt 286920
cgagttactt ttacaccgtc attcatcatt cccgcgaaag cgggaatcta gaaacgaaaa 286980
gtaacaggaa tttatcggga atggctggag tttaaaggac tggattcccg ccgtcgcggg 287040

aatgacggga ttttgggttg tggtaattta tcggaaaaac aaaaaaacct atgccgtcat 287100
tcccgagcag gcgggaatcc ggttatttaa aactgcagaa atttatccga agcaacaaca 287160
atctttccat cgtcattccc gcgtaggcgg gaatctagga cgtagaatct aaagaaaccg 287220
ttttatccga taagtttctg taccgaagaa tctggattcc cgctttcgcg ggaatgacgg 287280
cgcataagtt cccgtgcgga cagacctaga ttcccacctg cgtgggaatg acgattcaga 287340
agttgcctga aacctaaaaa actgaaaccg aacgagccgg atttccgctt tcgcgggaat 287400
gacgggattt tgggttgtgg taatttatcg ggaaaacgga aacccctatg ccgtcattcc 287460
cgcgcaggcg ggaatctagg acgtagaatc taaagaaacc gttttatccg ataagtttct 287520
gtaccgaaga atctggattc ccgctttcgc gggaatgacg gcgtataagt tcccgtgcgg 287580
acagacctat attcccacct gcgcgggaat gacgattcag aagttgcccg aaaccaaaaa 287640
actgaagccg aacggtctgg attcccgctt tcgcgggaat gacggcgcat aagttcccgt 287700
gcggacagac ctagattccc acctgcgtgg gaatgacgat tcagaagttg cccgaaacca 287760
aaaaactgaa gccgaacggt ctggattccc gctttcgcgg gaatgacggc gcataagttc 287820
ccgtgcggac aggcctagat tcccacctgt gtgggaatga cgattcagaa gttgcctgaa 287880
acctaaaaaa ctgaaaccga acgagccgga ttcccgcttt tacgggaatg acgggatttt 287940
gggttgtggt aatttatcgg gaaaacggaa acccctatgc cgtcattccc gcgcaggcgg 288000
gaatctagga cgtagaatct aaagaaaccg ttttatccga taagtttctg taccgaagaa 288060
tctggatttc cgctttcgcg ggaatgacgg cgcataagtt cccgtgcgga cagacctaga 288120
ttcccacctg cgtgggaatg acgattcaga agttgcctga aacctaaaaa actgaaaccg 288180
aacgagccgg atttccgctt tcgcgggaat gacgggattt tagattgcgg gtatttatcg 288240
ggaacggcgg cttggaagtt cattgaaacg gaaaaacaac ggaaacccaa aaaaccggat 288300
tcccgactgt gggaatgatg agattcaggt ttctgttttt gccggagttt gccgtatcgg 288360
gcttcagacg gcattgcctg ccgttgtacc cgcgggtgcg actgccttga tgtagttgag 288420
cgagacaaac tgcttctcgg catccaattc ggtgattttg aacaatgcct gtgatttggg 288480
cagtgcgtca aacggaatac cggtcgcgcg cgtgaccagc ggcaggcctt cgatgcggac 288540
gaggtcttct ttgaggatgg tcgcggtcag ctcgcttgta ccttgctgtt gcaggtacac 288600
aaggctccag taggcttcca tctgccgttg gaaatcggcg taggcggtat aggcggcatc 288660
aaagtcgcgc agtgcggcga aaagctcggc atcgctgttt tgatacagcg gctcggcagt 288720
gtcgtctatc aggctgatca gctgcttttg gttgatgtag tcggcggcgc ggcgcagcgg 288780
cgaggtaaac cagccgtaat gctgcacgcc catgccgata tgcggctcgg atttggtgct 288840
catgcgtact tttccggtgg gttggacgcg gaagaggccg ggcaggtcgt tgtcatggag 288900
catttgtgcc caagtgctgt tggcaagaat catcatctcg ctgaccagcg tatcgatggg 288960
tgagccgcgt tcgcggcgga cgacggatac cttgccttcc tcatccaatt cgatgctgta 289020
atcgtattgc ggcgcgcggt cgggttcgta tttgccgcgc gctttttgca gggcggtggc 289080
gaattgatag aaccaaatca ggtcttgatg gtgggcgaac atcatttcgc cggcttcgtc 289140
caagccggtt tcggcgttga aatgcggctc gatggcttgg atacgcaggt ttgtggcgat 289200
gttgaccgct tcgattttgc aggtcggcgc gccgacgttg aactcgccgt ccacatcgaa 289260
ataaatgctg acggcagggc ggtgtgcgcc tgcatcaagg ctgaacgcgg caatccagtt 289320
ttcgggcagc atcgtgattt tgccgccggg gaaataaacc gtgctcaagc gttccatgat 289380
gtttttttcc attttgtcgc ccggtttaac ggcaagtgac ggcgcggcga tgtggatgcc 289440
gacacgcttc gtgccgttgt ccaagtcggt caggcttaaa gcgtcgtcca cttcggtggt 289500
tgattcgtcg tcaatggaaa aggcggtaac gtcggccttg ggcaggtcgg gcatttcggg 289560
aagggcaagg tcggggaagc ctgttccttt agggaagtat ttgatttcaa acccgtcttg 289620
caggtattgg ggaatggacg taatgccgcc cgtttttttc gccaattcgt aggcagaggt 289680
tttcagcgcg tcggcggctt tggtaaaggc tttgtaggtc agcgactgct tgtcgggcgc 289740
gtgcaggatg gttttcaaat ccgccgcgat ttcagacggc atctcgccgc gtttcaaggc 289800
ttctgcccaa gcgtcgattt gcgcgtcttg ctgtttttttg cgttcgatgg cggcaagtgc 289860
ttgttttaaa gtttcttcgg gcgcggcttt gaacacgcct ttggcttttt tgtagaaata 289920
catcggcgcg gcgtaaagcg caatcaaagt tgccgccagc tcggttttgg tcggcgcatg 289980
gccgtaatat tcttcggcga tggcttcggc ggtaaattcc tcttcgccgc atacttccca 290040
caataaatcg gtgtcgatgt ccgccgcctg tgcctgcgcg ttttccaaaa acgccgccat 290100
atcgccgtca aactcggcaa agacgttgtt cgccttcact ttggtgcgtt tgccgtgtgg 290160
ggtatcgact tggtaggtgg catcgttttt ttggatgatg gcggcgattt tgaattggcc 290220
ggactcttcg taaaaaatat tcatttttcg gatttttctg tggaaactca agcgggcgat 290280
tttagcagat taccgaaaat gccgtctgaa aaaaggttgg gagagggttg gcgcggcttt 290340
gcggtgcttg cgttatagtg gattaacaaa aaccagtacg gcgttacctc gccttagctc 290400
aaagagaacg attctctaag gtgctgaagc accaagtgaa tcggttccgt actatttgta 290460
ctgtctgcgg cttcgtcgcc ttgtcctgat ttttgttaat ccactatacg tttttgacgg 290520
tgtacaatcg ctgtttttga acggaggatg gaatggagaa tacaaaccgt gtgccggagc 290580
aggcacgtta tgatgccgaa cgcaggcagg cagacgaagc attggcgggc gtgtttccgg 290640
cagtcagtat cttcggcagc gcgcgcacgc cgcagaatca tgcggattat gcgttcgcct 290700

gccgtctggc gcggcggctg tcggattcgg gcattgccgt catttcgggc ggcgggccgg 290760
ggattatgga ggcggcaaac aagggcgcgt ttgcagggaa gtcggtttcg gtggggctga 290820
acatcgtttt gccgcacgag cagaaaccga atccgtatca ggacatcgcc ttgcggtttt 290880
cccgttttgc cgaacgcaag gcggtgtttt tccgctattc ccaagcatat gtcgtgatgc 290940
cgggcggctt cgggacgctg gacgaattgt ttgaaatcct gaccttggtg cagacgggca 291000
aagtgccgcc gcgtccgatt gttttggtcg gaaaggcgtt ttggtcgggc ttggcggagt 291060
ggataaacgc gcagcttttg gcgcgcggtc tgatttccga aggggcggtc tctttgtttg 291120
ccatatcgga cgatgaagac gaaatcgttg cgtatctgtc ggaacacggg cttcagacgg 291180
catagcgtcc tgagagtgat gtataattgc aaacaattta acaatttttg atgtctttcc 291240
cgaacaggat gccgaaatga tcaaccccat cgcctcgctt tccccttttag atggccgtta 291300
tgcccaatcc gttgaagcat tgcgcccgat tttttccgaa tacggcctga tgaaggcgcg 291360
cgtcaaagtc gaattaaact ggctcaaagc cctcgccgcc gagccgaaga ttgccgaagt 291420
gccgcccttc agtgccgaaa cgcttgccga aatcgacacg gtgattgaaa acttttcatt 291480
ggaagacgcg gccgccgtca aagccatcga agccaccacc aatcacgatg tcaaagccat 291540
cgaatattgg ctgaaaaaac gttttgccga agtgccggaa gtcgccgccg tgagtgagtt 291600
catccacttc gcctgcacca gcgaagacat caacaacctg tcccacgctt taatgctgca 291660
agaagcgcgt gaggctgttt tgctgccgaa gctggccgaa atcatcgaaa aactgaccgc 291720
tatggcgcac gaccttgccg ccgtcccgat gatgagccgc acccacggcc agcccgccac 291780
gccgaccact ttgggcaaag aaaccgccaa tgtcgtgtac cgcctgcaac gccagtttaa 291840
aaacctgcaa gcgcaagagt tcctcggcaa aatcaacggc gcggtcggca actacaacgc 291900
ccatatggtc gcctatcctg atgtagattg ggaaacccac tgccgcaact tcgtcgaaat 291960
cagcctcggt ctgaccttca acccctacac catccaaatc gaaccgcacg actatatggc 292020
ggaattcttc caaaccctca gccgcatcaa cacgattctc atcgacttta accgcgacgt 292080
ttggggttat atttcattgg gttacttcaa acaaaaagtc aaagcaggcg aagtcggttc 292140
ttccaccatg ccgcacaaag tcaaccccat cgactttgaa aactccgagg gcaacctcgg 292200
tatggcaaac gccgtattgg gctttttgtc cgaaaaactg ccgatttccc gctggcagcg 292260
cgacctgacc gacagcaccg tattgcgcaa tatgggcgta ggcgtgggct atgccgtatt 292320
gggtttcgcc gcccacctgc gcggtctgaa caagctcgaa cccaaccccg ccgcgcttgc 292380
cgccgatttg gatgccactt gggagctgct cgccgagccg attcaaaccg taatgcgccg 292440
ttacggtgtc gccaatcctt acgaaaaact gaaagacctg acgcgcggca aaggcggcat 292500
cacgcccgaa gtgctgaaag gctttatcgg attgctggaa atccccgccg aagccaaagc 292560
caaattgctt gagctgaccc ccgcgctgta tgtgggcaag gctgaagcgt tggcgaaacg 292620
gatttgagcg tttactgaaa ccgatgccgt ctgaacgcgc gttcagacgg catttttaag 292680
ataacgggac atacgggggc gatatttatg caagctgtcc gatacagacc ggaaattgac 292740
ggattgcggg ccgtcgccgt gctatccgtc atgattttcc acctgaataa ccgctggctg 292800
cccggaggat tcctgggggt ggacattttc tttgtcatct caggattcct cattaccggc 292860
atcattcttt ctgaaataca gaacggttct ttttctttcc gggattttta tacccgcagg 292920
attaagcgga tttatcctgc ctttattgcg gccgtgtcgc tggcttcggt gattgcctct 292980
caaatcttcc tttacgaaga tttcaaccaa atgcggaaaa ccgtggagct ttctgcggtt 293040
ttcttgtcca atatttatct ggggtttcag caggggtatt tcgatttgag tgccgacgag 293100
aaccccgtac tgcatatctg gtctttggca gtagaggaac agtattacct cctgtatccc 293160
cttttgctga tattttgctg caaaaaaacc aaatcgctac gggtgctgcg taacatcagc 293220
atcatcctgt ttttgatttt gactgcctca tcgtttttgc caagcgggtt ttataccgac 293280
atcctcaacc aacccaatac ttattacctt tcgacactga ggtttcccga gctgttggca 293340
ggttcgctgc tggcggttta cgggcaaacg caaaacggca gacggcaaac agcaaatgga 293400
aaacggcagt tgctttcatc actctgcttc ggcgcattgc ttgcctgcct gttcgtgatt 293460
gacaaacaca atccgtttat cccgggaatg accctgctcc ttccctgcct gctgacggca 293520
ctgcttatcc ggagtatgca atacgggaca cttccgaccc gcatcctgtc ggcaagcccc 293580
atcgtatttg tcggcaaaat ctcttattcc ctatacctgt accattggat ttttattgct 293640
ttcgcccatt acattacagg cgacaaacag ctcggactgc ctgccgtatc ggcggttgcc 293700
gcgttgacgg ccggattttc cctgttgagt tattatttga ttgaacagcc gcttagaaaa 293760
cggaagatga ccttcaaaaa ggcatttttc tgcctctatc tcgccccgtc cctgatactt 293820
gtcggttaca acctgtacgc aagggggggat attgaaacag gaacacctcc gcccgttgcc 293880
cggcgcgccc cttgctgcgg aaaatcattt tccggaaacc gtcctgaccc tcggcgactc 293940
gcacgccgga cacctgaggg ggtttctgga ttatgtcggc agccgggaag ggtggaaagc 294000
caaaatcctg tccctcgatt cggagtgttt ggtttgggta gatgagaagc tggcagacaa 294060
cccgttatgt cgaaaatacc gggatgaagt tgaaaaagcc gaagccgttt tcattgccca 294120
attctatgat ttgaggatgg gcggccagcc tgtgccgaga tttgaagcgc aatccttcct 294180
aatacccggg ttcccagccc gattcaggga aaccgtcaaa aggatagccg ccgtcaaacc 294240
cgtctatgtt tttgcaaaca acacatcaat cagccgttcg cccctgaggg aggaaaaatt 294300
gaaaagattt gccgcaaacc aatatctccg ccccattcag gctatgggcg acatcggcaa 294360

gagcaatcag gcggtctttg atttgattaa agatattccc aatgtgcatt gggtggacgc 294420
acaaaaatac ctgcccaaaa acacggtcga aatatacggc cgctatcttt acggcgacca 294480
agaccacctg acctatttcg gttcttatta tatggggcgg gaattccaca aacacgaacg 294540
cctgcttaaa tcttcccacg gcggcgcatt gcagtagcct gccttcttgt cggatattgc 294600
ctttggcagc ctatgccgct gtttgccgtt cggggcggcg gcttttatag tggattaaca 294660
aaaatcagga caaggcgacg aagccgcaga cagtacaaat agtacggaac cgattcactt 294720
ggtgcttcag caccttagag aatcgttctc tttgagctaa ggcgaggcaa cgccgtactg 294780
gtttttgtta atccactata ttttgccgtt ttgaggccgg ggtcggaata accgtttttt 294840
gatgattttc cctccccggc tgtgtcatca aaaccccaat tgcctttcca aactctccac 294900
cagattgtca tccagtttca aagcctgcga caggcgggcg aggaagacgg tttctttccg 294960
cgacaaatcg gcacagacca accttgccgc cagataggcc tccgccgcca acgcctcatc 295020
gttgccgacg gcggcggcga tgtcttcgat gcttgcggga aggcggtatt cggcggcgag 295080
ccatgcggca gtttcggggt ctgtgccgct ttcctgttcg atagtccggc gttcggcttc 295140
gtctatcatg ccgtctgaag cggcggcggc tatcatggtg cgcaatacgg tacggctgta 295200
tgtttcttca gtttctccgg caggttggaa atcgctttgt gttacggttg cccgcccttt 295260
gttttgctgc cacatctgat agccccggta ggcgaggtag cccaaagcgg cggtcgaacc 295320
gattttggtg atggttttgc ggtttttacc gttcagcagc atggaggcga caccggcaac 295380
cagcgcgcct ccgccgaatg aattgagcgg gctgtcggag aatgtgttgc cttttttttg 295440
aaccgtgctt aagacttggt tgagcagtcg ggtaaagttc atgaattttt cctttctgtt 295500
gcgggaaggg cggtatgttt accctatcct tttaaacggc ggcaggccgg ccaataattg 295560
ttggccgtac cgctgtgttt tgatgcggtt gtcgaggatg gttacgcggc cgtagtcttg 295620
ttcggtgcgg atgaggcggc cgacggcctg gatgagtttg atgccggctt cggggacggt 295680
gatttcgatg aaggggttgc cgccgcgctg ttctatccag cggttttggg ttttttcgat 295740
ggggttgtcg ggcatggcga agggaagttt ggcgatgatg acttgcacgc aggcggtgcc 295800
gggcaggtcg agtccttcgg caaagctgtc gagtccgaag atgatgctgg ctttgccttc 295860
ttctatggcc cggtggtgtt tttgcaggag gacggctttg ggtaattcgc cttgtacgag 295920
caagagcggc aggtagtctc cgggcaggcg cagggcgaca tcctgcattt gtttgcgcga 295980
ggaaaacaag acgagcgtgc cgatggcttc ggtgggcgaa ataagcttgg gcagccattc 296040
gatgacggcg gcggtgtggg cttcggggtc tttggggctg gcgtatatgg gggggatgta 296100
gagttcgccc tgtttttcaa agtcaaaggg gcttttgagg gcgagggtgg tggtttcggg 296160
cagccattgc agcccggttt ggcgcagcat caggttgaag ttgcccaagg attgcagggt 296220
ggcggaagtc aataccgcgc ctgccgcacg ccgccacagg ctgttggcaa ggtgggatgc 296280
gctgctgatg ggctggcgt tgaaaatgta gtcgtttttg tcgtcggcgc ggcgggttat 296340
ccatttcgcc aacggttctt caccctcgag gggacagtg gagagcaaat cccaaaccgc 296400
gctgatttgt tcgatacggg cgataaaaag accgaactcg ctggtcaggc ggtcgaggag 296460
cgcgccgtcc tgttcttttt cgcggcgtgc ggcagaaagc gcatcgttca gcccgataac 296520
gtgtttgagc aggctgcgcg cagcaatggc cgtattggaa acggtggttt cgaggccttc 296580
ggggattttg ccgtcttccc acagccaagt cggttcgctg ttggttcgtc tgtcgttttc 296640
agacaccccc agacttaaag acggctcttc cgccaaatgg aattgccatt catgcaggct 296700
gtcgagcaag gatgcggcgg cttcgtcggc taggttggca agttcggctt tatcggtcag 296760
cgcggcaatt ttgccggtca gctgcggcag tttttccagc gtccaaacgg caatattcca 296820
tgaatgttcg gcggcaaaac ggctgagggc ttttttgggc aggtggtgcg cttcgtcgat 296880
gcaatagaaa ctgttttcgg gcgcaggcag aatcacgccg ccgcccatac tgatgtcggc 296940
aagcagaaga tcgtggttgg caacgacgac atcgacggtt tccaagacat cgcgtgctag 297000
gtaaaacgga cattccggac ggttgggaca ggcggttttc aggcagccgt ggcggtcgtt 297060
ggtcactttg agccaaatcg cgtcatcgat tttttccggc caagtgtcgc ggtcgccgtt 297120
gaaccgtcgg gcggaaaatt cgtcggcgat gtcgcgcagc agcttcaatt cttcgggctt 297180
gggtttgctg tcccacaaga cggcgggggc ttcaaagccg agcaggtttt gctgggcatt 297240
gctttgcgtc agtcgataga gtttgtaggg gcagagatag cggccgcgcc ctttggcaag 297300
tgcgaaggtc agttccaaac cgcttttttc gaccagaaac ggcaggtcgc ggtctaccaa 297360
ctgctcctgc aaggcaaccg tcgcgctgct cacaatcagc cgcttgccgc gtgtttgcgc 297420
catgatgccg ccggccaaaa ggtaggccaa cgatttgccc acgccggtcg gcccttcgat 297480
cacggcaatg ctctcgcctt cgcgcttggg cggctcgccg ccttcttcgc gcgccaacgt 297540
ccgcgaaaaa gcgttggcaa ccgccgcaat catttcccgc tgcgaagcac gcggacggaa 297600
accgggcagg tttttgccga tgttttggta atggtcgcgg atggcgtttt tttctaaatc 297660
ggtgagcatg gcgttttgta cggcggtaga agtgggctta ttttaacatt gcacggaagc 297720
ggtacaatat cgttgtcgga atggggggtg aggtgaatcg tgcggacgtg gttggttttt 297780
tggttgcagc gtttgaaata cccgttgttg ctttggattg cggatatgtt gctgtaccgg 297840
ttgttgggcg gcgcggaaat cgaatgcggc cgttgccctg tgccgccgat gacggattgg 297900
cagcattttt tgccggcgat gggaacggtg tcggcttggg tggcggtgat ttgggcatac 297960
ctgatgattg aaagtgaaaa aaacggaaga tattgagtca ttcggacgca atgccgtctg 298020

aaacggaagt tcagacggca tttgttttag gttgccgtac cgcttaggga ataccggcga 298080
caggatgggc gggatagccg tgggtatcga ccgaacaggc aaaccgccaa ggcgtgtgga 298140
cggtgtcggc ggacaggtgg gcaagctcgg gaatgtgccg tctgacaaag gtgccgtcgg 298200
ggtcggtttt gtgtgcggcg gcggcaatgt cggggcaggt gtgccgtgag gcggcaagcc 298260
gccagttgcc ttggttgatt gctgcatcga aatcggtcag ctgtcgggca aaccatatct 298320
cgccttcgcg gcgggggagg tttaaaacgt ggcagaaaaa atccgcgctc aagcgtctca 298380
gggcggggtg gaggctgccg gttttgtgca aacagcgcat cgcggcatcg ataatcggaa 298440
tgccggtccg gccctgctgc caaagcgtca ggcgcagggt gtgttcagga ttgccgtctg 298500
aagggtcgtc atccgtgtgc tgcaaggcaa gttgaaggaa aaaatcgcgg cggatgatgt 298560
tgtccgccca cgcgttcaga cggcgttcga ggctttcccg cgcgagcagg cgcggcgaga 298620
tgcagccggc actcaaatac gcgcccatca gcgaagtgtg tttgcgcgag gggaaatcct 298680
ttaaaacgga gtaggaatcc gcctgttcga gaaaccgccg ccactgccgc caagccgccg 298740
tttcgccgct gttttgcggc aggaagatgc cgtctgaaag cgcggcaggc tgcggggcgg 298800
aaaggttttc ggggaagggt tggcggtatg ccgcgaatag gtccggaccg gcgggggggct 298860
gcttggaaaa gcggtcgagc catacttcgc ggtagcggtc gaaatcggca tatgccgtgc 298920
cgccgtcggg tatcaggtcg gttttgccga aaacggcgcg gtcgttgacg aaggttaacg 298980
cgatgccgtg tttgtccaat tcgtgccaaa gggcgttgtc ggcgagtttg tcggcaaaag 299040
tatgggattc gtcggcgatg acggtgcgga tattgaggcg gacggcgagc cggacgagct 299100
cggcaggaga tgccgccgtg tagagcggga tgccgcgccc tgcaagccct tgggcgagtt 299160
cggcggcgga ttggcggtag aacgcggcgc ggcgagggtt gtctgtttcg gcatcgtcaa 299220
tccaaatgcc gataatgggc aaacttcggc aacggcggcg cataaggcgg cgttgtcgcg 299280
gatgcggagg ttttggcgga accagacgag cgtgtgtgcg gcgcacgtgt ccgcataaag 299340
ggggcgggcg gtttcagacg gcatttcggc agcctttcct gctggcgatt ttttcgttca 299400
gaaaatcgat gaagctgcgg actttcgcgc ttaagaatgc cctgtctgca taaacggcat 299460
tcagccggtc ggtcgggacg gcgtatccgg gcagcagcct caccagcgtg ccgcagcgca 299520
aatcgtgttc cgccgcccaa agcggctgat aaccgatgca cgcgcccgcc ttaatcattt 299580
cgcgcatcat cagcgtgttg tcggtacgga tgacgggggt cagttcaagc cggtattttt 299640
tgccgtccga tttgcgggtg aggtcgagtt tctgctggtt ggtgtaggtc ggcaggacgg 299700
cgggcagccc cgccacttct tccggcgttt ccggcacgcc gttgcgcctc aggaaatcgg 299760
gcgaggcgag cagggcaaat tcgatttccg ccagtgggcg cgcaatcagc gacggggaca 299820
gggtttggga aacgcgcaac gccaaatcca cgccttcggc aatcaaatcg acgtggcggt 299880
tgtccaaaat cagttctaat gccacttcgg gataacgttc gcggtattcc gccagccagt 299940
tgcatatctg gctgccggca aaccacagcg gcatcgttac gcgcagcagc ccctgcggtt 300000
tttccgtccc cccggcggct ttttgcgcgg catcgtcgag cgtgtcgagc gcgtaactgc 300060
attgccggta gtattcttcc ccggcttcgg tcaggctgag gttgcggctg ttgcggtgca 300120
ggagtttggc ttggacggtg ttttccaagt ggctgacgtg tttgcttgcc attgcggtgg 300180
agatgccgag cgcgtcggcg gcgcgggtga agccgccgct ttggacgact tggcggaaaa 300240
ccttgaggct gaacagggtg tccatatttt cttgtgtgga aaagttgtat caataaaagc 300300
agtatatatt tgaaaagggg aaacatctat actctaccgc ctgaaatgaa gacaaatatc 300360
aaaggagctt ttatgtccga ttgctgcaac cgtatccaac cggttttgct ttctgttttg 300420
cgtatcgtaa ccgcctacct gtttttgttg cacggtacgt cgaaaatctt cgccttcccc 300480
attgaaatgg gcagcggttc gcccggcggg ctgttgctgc ttgccggtat tttagaaatt 300540
gtcggcggca ttttgctggt gttgggcctg tttgcgcgcc ctgccgcgtt tgttttgtcc 300600
ggccagatgg cggttgccta ttttatggcg cacgcttccg gaaatgcttt gttcccgatt 300660
gccaacggcg gcgagtccgc agtgctgttc tgcttcgtat tcctctatat cgcggcggcg 300720
ggcggcggag catggtcgct ggacaggctg tttttcaagc gtaaagcctg aatcggactg 300780

cctaaagtgt attttgttga atgtttttga ggaaaagaaa tgacccgtca atctctgcaa 300840
caggctgccg aaagccgccg ttccatttat tcgttaaata aaaatctgcc cgtcggcaaa 300900
gatgaagttg tccaaatcgt cgaacacgcc gttttgcaca caccttcttc gttcaattcc 300960
caatctgccc gcgtggtcgt gctgtttggc gaagagcatg ataaggtgtg gcaatttgtc 301020
gaagacgcgc tgcgtgccgt cgtgcctgcc gacagttttg aaccgaccgc gcaaaaattg 301080
aacctgttta aggcgggtgc ggcaaccatt ttgttttatg aagatcaaaa tgtcgtcaaa 301140
ggtttgcagg agcagttccc tgcttatgcc gctaacttcc ccgtttgggc ggatcaggca 301200
aacgcgatgg tgcagtatgc cgtttggacg acacttgccg cggtcggcgt aggtgcaaac 301260
ctgcaacatt acaatccctt gcccgatgcg gcgattgcca aagcgtggaa tatccccgaa 301320
aactggttgt tgcgcgcaca aatggttatc ggcggtattg aaggggcggc aggtgaaaag 301380
acctttgaac ccgttgcaga acgtttgaaa gtgttcggcg cataatttcg cggtcaaaaa 301440
aatgccgtct gaaccctgtt cagacggcat ttttcagtat caggcggcga gttttccgca 301500
ttctgagacc tttgtttaca aatatcatgt tcaatatagt taaaagaaat tattctcatt 301560
tcctccgtga ggcaatataa ttcggttgtt ttgttaaatt gagtataaaa atgaaaatat 301620

catttcattt agctttatta cccacgctga ttattgcttc cttccctgtt gctgccgccg 301680
atacgcagga caatggtgaa cattacaccg ccactctgcc caccgtttcc gtggtcggac 301740
agtccgacac cagcgtactc aaaggctaca tcaactacga cgaagccgcc gttacccgca 301800
acggacagct catcaaagaa acgccgcaaa ccatcgatac gctcaatatc cagaaaaaca 301860
aaaattacgg tacgaacgat ttgagttcca tcctcgaagg caatgccggc atcgacgctg 301920
cctacgatat gcgcggtgaa agcattttcc tgcgcggttt tcaagccgac gcatccgata 301980
tttaccgcga cggcgtgcgc gaaagcggac aagtgcgccg cagtactgcc aacatcgagc 302040
gcgtggaaat cctgaaaggc ccgtcttccg tgctttacgg ccgcaccaac ggcggcggcg 302100
tcatcaacat ggtcagcaaa tacgccaact tcaaacaaag ccgcaacatc ggagcggttt 302160
acggctcatg ggcaaaccgc agcctgaata tggacattaa cgaagtgctg aacaaaaacg 302220
tcgccatccg tctcaccggc gaagtcgggc gcgccaattc gttccgcagc ggcatagaca 302280
gcaaaaatgt catggtttcg cccagcatta ccgtcaaact cgacaacggc ttgaagtgga 302340
cggggcaata cacctacgac aatgtggagc gcacgcccga ccgcagtccg accaagtccg 302400
tgtacgaccg cttcggactg ccttaccgca tggggttcgc ccaccggaac gattttgtca 302460
aagacaagct gcaagtttgg cgttccgacc ttgaatacgc cttcaacgac aaatggcgtg 302520
cccaatggca gctcgcccac cgcacggcgg cgcaggattt tgatcatttc tatgcaggca 302580
gcgaaaatgg caacttaatc aaacgtaact acgcctggca gcagaccgac aacaaaaccc 302640
tgtcgtccaa cttaacgctc aacggcgact acaccatcgg ccgttttgaa aaccacctga 302700
ccgtaggcat ggattacagc cgcgaacacc gcaacccgac attgggtttc agcagcgcct 302760
tttccgcctc catcaacccc tacgaccgcg caagctggcc ggcttcgggc agattgcagc 302820
ctattctgac ccaaaaccgc cacaaagccg actcctacgg catctttgtg caaaacatct 302880
tctccgccac gcccgatttg aaattcgtcc tcggcggccg ttacgacaaa tacaccttta 302940
attccgaaaa caaactcacc ggcagcagcc gccaatacag cggacactcg ttcagcccca 303000
acatcggcgc agtgtggaac atcaatcccg tccacacact ttacgcctcg tataacaaag 303060
gcttcgcgcc ttatggcgga cgcggcggct atttgagcat cgatacgttg tcttccgccg 303120
tgttcaacgc cgaccccgag tacacccgcc aatacgaaac cggcgtgaaa agcagttggc 303180
tggacgaccg cctcagcact acgttgtctg cctaccaaat cgaacgcttc aatatccgct 303240
accgccccga tccaaaaaac aaccccttata tttatgcggt tagcggcaaa caccgttcgc 303300
gcggcgtgga attgtccgcc atcgggcaaa tcatccccaa aaaactctat ctgcgcggtt 303360
cgttgggcgt gatgcaggcg aaagtcgttg aagacaaaga aaatcccgac cgagtgggca 303420
tccatttgaa taataccagc aacgttaccg gcaacctgtt tttccgttat accccgaccg 303480
aaaacctcta cggcgaaatc ggcgtaaccg gtacaggcaa acgctacggt tacaactcaa 303540
gaaataaaga agtgactacg cttccaggct ttgcccgagt tgatgccatg cttggctgga 303600
accataaaaa tgttaacgtt acctttgccg cagccaatct gctcaatcaa aaatattggc 303660
gttcggactc tatgccgggt aatccgcgcg gctatactgc ccgggtaaat taccgtttct 303720
gatgaaatca ggcaaaggct gaaataaaac taaacacatt ttttcactca aatcgaacac 303780
gccttcaata aaatgccata aaatccgcac attaatctga cacacaagag atacctatga 303840
aactgaaaac cttagctttg acttcattga ccctgttggc attggccgct tgtagcaaac 303900
aggctgaaac cagtgttccg gcagacagcg cccaaagcag ctcatctgct ccggcagccc 303960
ctgctgagtt gaacgaaggt gtgaactaca ctgtattgtc tacgcctatt ccgcaacagc 304020
aggccggtaa aatcgaagta ttggaatttt tcggctactt ctgcccgcat tgcgcccatc 304080
ttgagccggt cttgagcgag cacatcaaaa cgtttaaaga cgatacctat atgcgccggg 304140
agcatgtcgt gtggggtgat gaaatgaaac ctttggcacg tttggcggcc gcagtggaaa 304200
tggccggtga atcagataaa gccaacagcc atattttcga tgcgatggtt aatcaaaaaa 304260
tcaatctggc cgataccgat accctgaaaa aatggctgtc cgagcaaaca gcgtttgacg 304320
gcaaaaaagt attggctgca tttgaggctc ctgaaagcca agcgcgtgcg gctcaaatgg 304380
aagagttgac caataaattc caaatcagcg gcacaccgac tgtgattgtc ggcggcaaat 304440
accaagttga atttaaagac tggcagtccg gtatgaccac gattgaccag ttggtggata 304500
aagtacgcga agagcagaaa aagccgcaat aagttgagga ttgaatgagt aaaggccatc 304560
tgaaaatagg atttcagacg gccttttgta tttaggcttt atagaagaga tgattgctta 304620
aagccttatg gttttaaatc agaatatata gcggattaac aaaaaccagt acggcgttgg 304680
ctcgccttag ctcaaagaga acgattctct aaggtgctga agcaccaagt gaatcggttc 304740
cgtactatct gtactgtctg cggctcgccg ccttgtcctg atttttgtta atccactata 304800
aatcagaata taaaacaaaa acgccgtctg aaatttcaga cggcgttttc tgttaaatcg 304860
gcttacaaac ccgggaacat ccctttttatc cccctcattc ctttcgccat acgcatcagt 304920
ttgcccaagc cgttgccgct gaacatcttc atcatttgtt gcatttgttc aaactgtttg 304980
agcaatttgt tcacttcctg cacggttgtg cccgcaccca ttgcaatacg gcgtttgcgg 305040
ctggctttga gcagggcagg gttggcgcgt tctttagggg tcatcgagtt gatgatggct 305100
tctactttgc ccatcgcttt ttcagccgtt ccttcgggga tttgtttcga gatttgaccc 305160
agttcgcccg gcattttcga catcaggttt tccaaaccgc ccatattgcg catttgctgg 305220
atttgttctt taaagtcgtt gaggtcgaag cctttgcctt tgtgcagctt tttcgccatt 305280

ttagcggcgg cttcttcgtc tatacctttt tgaacgtctt caatcagggt caatacgtcg 305340
cccataccca aaatgcggcc ggcaagacgg tcggggtgga aaggttcgag gccgttgatt 305400
ttttcgccga caccgataaa tttaatcggt ttgccggtta cgtggcgtac ggacaatgcc 305460
gcaccgccgc gcgagtcgcc gtccatcttg gtcaatacga ctccggtcag cggcagggct 305520
tcattaaatg cctgagcagt gttcaccgca tcctgaccca gcatcgcatc gatgacgaac 305580
aaagtttcca ccgggttaac cgccgcgtga agggctttga tttcgttcat catctcttca 305640
tcgattgcca aacggccggc ggtatcgacc atcaatacat cgtaaaaatg tttttggcg 305700
taatcgacgg cggcagttgc aatttcaacc ggtttttggt tggtatcgga cgggaaaaaa 305760
tccacgccga cctgttcggc caacagacgc agctgttcaa tcgcggcagg acggtaaacg 305820
tcggcggata ccaccaaaac ctttttcttc tgatcgtttt tcaacaggcg ggcgagtttg 305880
ccgacggtcg tcgtcttgcc tgcgccctgc aaacctgcca tcaacacgac ggcgggcggc 305940
gcaaccgaca aatccagcgt tttgttttcc ctgcccatca gttcggtcag ggctttgttg 306000
accacgccga taaatgcctg atccggcgtc aggctgcccg ctacttcctg accgagggcc 306060
ttttctttga cgttgttgat gaactctttg acgacaggca gggcgacatc cgcctcaagc 306120
agggcgaggc ggacttcgcg caaggcctct ttaatattgt cttcggtcag tttggcctgc 306180
ccccggatgt ttttgaagac attgctgaag cggccggtta aattgtctaa catactggtc 306240
cttggtctga ataagaatag cttgccccat caggggcatt ctttgttaaa ataaaatcaa 306300
aataatttga tgcggcttgt gtgccggaca gcatatcggc aaatccgtca aggcttgacc 306360
gaaatgggga ttttacaatt ccaacgttaa aagttccaat atttcataag cggccgcata 306420
cggcgcaaca gtatagatag agaaagtcca ccatgccgac agttttcatc tttttgacgg 306480
cggtttacgc aggattgggt gcatttgcat ggcactgcca acagcagggg tgcggccggg 306540
attacccgtg gaagacggaa ttgccggttt tgggtgcggc attgaccgtc cacggcgcgg 306600
cactgcttat gccggtcatt caagacaaaa tcatcattat gggcttcggg tattccggca 306660
gcctgattgt ttggatgatg ctgtttattt attttgccgg cagcttcttt tatccgctgc 306720
gcggagtgca gttgctgctg tatccttgcg ccgcactgat gctgctgtca ggtttggttt 306780
ttcctggaaa attctcggga tatgaaatta ccgaccttcc ctttatgctg catatcggaa 306840
cttcgctgct cgcatacggg ctgttcggca tcgcaacatt attgtccgtt ttgaccctgc 306900
tgctgaatcg gagcctgcac cgcaggagct tctccaagct cgcaggattc ctgccgtcgc 306960
tgctcagttt ggaaaaactc atgttccagg ccatgtgggc aggtttcatc ctgctgacct 307020
attccgtcgt cagtggaaca ttttttgccg aagccgtatt cggcaaaccc atgaccttta 307080
cccataaaac cgtattcggc atattgtcat ggctgattta cggcggactg ctgctcaagc 307140
acagcatgac cgcatggcgc ggcaaaaaag ccgccgtgtg gaccatcatc ggatttgtca 307200
gccttatgat tgcctatatg ggcagcaagt tcgtattgga aatcattctg aaaagataag 307260
aagagccaac agatgccgtc tgagtccccg agtttcagac agcatattca caaaggcgca 307320
ccagccggag gagggagagg aaaggattgt tggaggcggc gcagtattta gcagaaataa 307380
aaaaccttat ccgacagcga catgacgaat ttccccaaaa aaatcccgct gaaagcattg 307440
accgtttttc cctgtgggcg tatagttcgg ttcttcgctg ctgcagaagt ggcggacgaa 307500
ctgaaaagta tagcacagaa tgttggggat atcgagagat atcttgacag gcggaaggaa 307560
tactttataa ttcgcaacgc tctttaacaa aacagattac cgataagtgt gagtgccttg 307620
agtctcacac tgtttgaaag acagacaaga taatgttttg aacattgtcc tgttggtttc 307680
tttgaagcag accagaagtt aaaaagttag agattgaaca taagagtttg atcctggctc 307740
agattgaacg ctggcggcat gctttacaca tgcaagtcgg acggcagcac agagaagctt 307800
gcttctcggg tggcgagtgg cgaacgggtg agtaacatat cggaacgtac cgagtagtgg 307860
gggataactg atcgaaagat cagctaatac cgcatacgtc ttgagagaga aagcagggga 307920
ccttcgggcc ttgcgctatt cgagcggccg atatctgatt agctagttgg tggggtaaag 307980
gcctaccaag gcgacgatca gtagcgggtc tgagaggatg atccgccaca ctgggactga 308040
gacacggccc agactcctac gggaggcagc agtggggaat tttggacaat gggcgcaagc 308100
ctgatccagc catgccgcgt gtctgaagaa ggccttcggg ttgtaaagga cttttgtcag 308160
ggaagaaaag gctgttgcta atatcagcgg ctgatgacgg tacctgaaga ataagcaccg 308220
gctaactacg tgccagcagc cgcggtaata cgtagggtgc gagcgttaat cggaattact 308280
gggcgtaaag cgggcgcaga cggttactta agcaggatgt gaaatccccg ggctcaaccc 308340
gggaactgcg ttctgaactg ggtgactcga gtgtgtcaga gggaggtaga attccacgtg 308400
tagcagtgaa atgcgtagag atgtggagga ataccgatgg cgaaggcagc ctcctgggac 308460
aacactgacg ttcatgcccg aaagcgtggg tagcaaacag gattagatac cctggtagtc 308520
cacgccctaa acgatgtcaa ttagctgttg ggcaacctga ttgcttggta gcgtagctaa 308580
cgcgtgaaat tgaccgcctg gggagtacgg tcgcaagatt aaaactcaaa ggaattgacg 308640
gggacccgca caagcggtgg atgatgtgga ttaattcgat gcaacgcgaa gaaccttacc 308700
tggtcttgac atgtacggaa tcctccggag acggaggagt gccttcggga gccgtaacac 308760
aggtgctgca tggctgtcgt cagctcgtgt cgtgagatgt tgggttaagt cccgcaacga 308820
gcgcaaccct tgtcattagt tgccatcatt cagttgggca ctctaatgag actgccggtg 308880
acaagccgga ggaaggtggg gatgacgtca agtcctcatg gcccttatga ccagggcttc 308940

acacgtcata caatggtcgg tacagagggt agccaagccg cgaggcggag ccaatctcac 309000
aaaaccgatc gtagtccgga ttgcactctg caactcgagt gcatgaagtc ggaatcgcta 309060
gtaatcgcag gtcagcatac tgcggtgaat acgttcccgg gtcttgtaca caccgcccgt 309120
cacaccatgg gagtggggga taccagaagt aggtaggata accacaagga gtccgcttac 309180
cacggtatgc ttcatgactg gggtgaagtc gtaacaaggt agccgtaggg gaacctgcgg 309240
ctggatcacc tcctttctag agaaagaaga ggctttaggc attcacactt atcggtaaac 309300
tgaaaaagat gcggaagaag cttgagtgaa ggcaagattc gcttaagaag agaatccggg 309360
tttgtagctc agctggttag agcacacgct tgataagcgt ggggtcggag gttcaagtcc 309420
tcccagaccc accaagaacg gggcatagc tcagttggta gagcacctgc tttgcaagca 309480
gggggtcatc ggttcgatcc cgtttgcctc caccaatact gtacaaatca aaacggaaga 309540
atggaacaga atccattcag ggcgacgtca cacttgacca agaacaaaat gctgatataa 309600
taatcagctc gttttgattt gcacagtaga tagcaatatc gaacgcatcg atctttaaca 309660
aattggaaag ccgaaatcaa caaacaaaga caaagcgttt gttttgattt tttattcttt 309720
gcaaaggata aaaatctctc gcaagagaaa agaaaacaaa cacagtattt gggtgatgat 309780
tgtatcgact taatcctgaa acacaaaagg caggattaag acacaacaaa gcagtaagct 309840
ttatcaaagt aggaaattca agtctgatgt tctagtcaac ggaatgttag gcaaagtcaa 309900
agaagttctt gaaatgatag agtcaagtga ataagtgcat caggtggatg ccttggcgat 309960
gataggcgac gaaggacgtg taagcctgcg aaaagcgcgg gggagctggc aataaagcaa 310020
tgatcccgcg atgtccgaat ggggaaaccc actgcattct gtgcagtatc ctaagttgaa 310080
tacatagact tagagaagcg aacccggaga actgaaccat ctaagtaccc ggaggaaaag 310140
aaatcaaccg agattccgca agtagtggcg agcgaacgcg gaggagcctg tacgtaataa 310200
ctgtcgagat agaagaacaa gctgggaagc ttgaccatag tgggtgacag tcccgtattc 310260
gaaatctcaa cagcggtact aagcgtacga aaagtagggc ggggcacgtg aaatcctgtc 310320
tgaatatggg gggaccatcc tccaaggcta aatactcatc atcgaccgat agtgaaccag 310380
taccgtgagg gaaaggcgaa aagaaccccg ggaggggagt gaaacagaac ctgaaacctg 310440
atgcatacaa acagtgggag cgccctagtg gtgtgactgc gtaccttttg tataatgggt 310500
caacgactta cattcagtag cgagcttaac cgaatagggg aggcgtaggg aaaccgagtc 310560
ttaatagggc gatgagttgc tgggtgtaga cccgaaaccg agtgatctat ccatggccag 310620
gttgaaggtg ccgtaacagg tactggagga ccgaacccac gcatgttgca aaatgcgggg 310680
atgagctgtg gatagggtg aaaggctaaa caaactcgga gatagctggt tctccccgaa 310740
aactatttag gtagtgcctc gagcaagaca ctgatggggg taaagcactg ttatggctag 310800
ggggttattg caacttacca acccatggca aactaagaat accatcaagt ggttcctcgg 310860
gagacagaca gcgggtgcta acgtccgttg tcaagaggga aacaacccag accgccagct 310920
aaggtcccaa atgatagatt aagtggtaaa cgaagtggga aggcccagac agccaggatg 310980
ttggcttaga agcagccatc atttaaagaa agcgtaatag ctcactggtc gagtcgtcct 311040
gcgcggaaga tgtaacgggg ctcaaatcta taaccgaagc tgcggatgcc ggtttaccgg 311100
catggtaggg gagcgttctg taggctgatg aaggtgcatt gtaaagtgtg ctggaggtat 311160
cagaagtgcg aatgttgaca tgagtagcga taaagcgggt gaaaagcccg ctcgccgaaa 311220
gcccaaggtt tcctgcgcaa cgttcatcgg cgtagggtga gtcggcccct aaggcgaggc 311280
agaaatgcgt agtcgatggg aaacaggtta atattcctgt acttgattca aatgcgatgt 311340
ggggacggag aaggttaggt tggcaagctg ttggaatagc ttgtttaagc cggtaggtgg 311400
aagacttagg caaatccggg tcttcttaac accgagaagt gacgacgagt gtctacggac 311460
acgaagcaac cgataccacg cttccaggaa aagccactaa gcttcagttt gaatcgaacc 311520
gtaccgcaaa ccgacacagg tgggcaggat gagaattcta aggcgcttga gagaactcag 311580
gagaaggaac tcggcaaatt gataccgtaa cttcgggaga aggtatgccc tctaaggtta 311640
aggacttgct ccgtaagccc cggagggtcg cagagaatag gtggctgcga ctgtttatta 311700
aaaacacagc actctgctaa cacgaaagtg gacgtatagg gtgtgacgcc tgcccggtgc 311760
tggaaggtta attgaagatg tgagagcatc ggatcgaagc cccagtaaac ggcggccgta 311820
actataacgg tcctaaggta gcgaaattcc ttgtcgggta agttccgacc cgcacgaatg 311880
gcgtaacgat ggccacactg tctcctcctg agactcagcg aagttgaagt ggttgtgaag 311940
atgcaatcta cccgctgcta gacggaaaga ccccgtgaac ctttactgta gctttgcatt 312000
ggactttgaa gtcacttgtg taggataggt gggaggctta gaagcagaga cgccagtctc 312060
tgtggagccg tccttgaaat accaccctgg tgtctttgag gttctaaccc agacccgtca 312120
tccgggtcgg ggaccgtgca tggtaggcag tttgactggg gcggtctcct cccaaagcgt 312180
aacggaggag ttcgaaggtt acctaggtcc ggtcggaaat cggactgata gtgcaatggc 312240
aaaaggtagc ttaactgcga gaccgacaag tcgagcaggt gcgaaagcag gacatagtga 312300
tccggtggtt ctgtatggaa gggccatcgc tcaacggata aaaggtactc cggggataac 312360
aggctgattc cgcccaagag ttcatatcga cggcggagtt tggcacctcg atgtcggctc 312420
atcacatcct ggggctgtag tcggtcccaa gggtatggct gttcgccatt taaagtggta 312480
cgtgagctgg gtttaaaacg tcgtgagaca gtttggtccc tatctgcagt gggcgttgga 312540
agtttgacgg gggctgctcc tagtacgaga ggaccggagt ggacgaacct ctggtgtacc 312600

ggttgtaacg ccagttgcat agccgggtag ctaagttcgg aagagataag cgctgaaagc 312660
atctaagcgc gaaactcgcc tgaagatgag acttcccttg cggtttaacc gcactaaaga 312720
gtcgttcgag accaggacgt tgataggtgg ggtgtggaag cgcggtaacg cgtgaagcta 312780
acccatacta attgctcgtg aggcttgact ctatcatttg aagaacttca agagataaaa 312840
gcttactgac tgattcagtc attaccgaat atattgatta aggctttacc gatttgtaac 312900
agtttaagtt tggcggccat agcgagttgg tcccacgcct tcccatcccg aacaggaccg 312960
tgaaacgact cagcgccgat gatagtgtgg ttcttccatg cgaaagtagg tcactgccaa 313020
acacccattc agaaaacccc cgattattcg gggtttttg ctttgcccgg aaaaaatgtt 313080
tgctttgccc ggaaaaaatg tcggtgatgg cgggacggca tccgtacggt gtccggtcgg 313140
gtttgcggag gaacggcttg aaactttggg atattcattt tagaatgact cgttttatcg 313200
tcgcaagatg cggtttattg tttgcaaccc ttaaaggaaa aaccatgaag aaaatgttcg 313260
tgctgttctg tatgctgttc tcctgcgcct tctcccttgc ggcggtaaac atcaatgcgg 313320
cttcgcagca ggagttggag gcgctgccgg gcataggccc ggcgaaggcg aaggccattg 313380
cggaataccg tgcgcaaaac ggtgcgttca agtctgtaga cgatttgacc aaggtaaagg 313440
gcatcggccc tgcggtgctg gcgaagctga aggaccaggc ttcggtcggc gcgcccgcac 313500
caaaagcccc agccaaaccg gtgctgcccg cggataaaaa ataggggaac ctgtaaagga 313560
aagggcatcg gccgccgtcg gtgctttttt gtttggaagg gaaatggcta aaatatgtag 313620
cattatgttc tgtatcgttg tttaccgctt ccgcaccttt gtccgcctta aagcaggtag 313680
acaccgcaat gaatcgacgc aaagaaaatg ccgtctgaac atgcgttcgg gcggcgtttt 313740
gttggggggt atcggagcgg aacgtctgaa aaagggtttc aggcggtctt tgggcgtgtg 313800
gtgacagtcg aaaacgtgat aaggctacct gaaaagtttg ggagattttc aggtagcctt 313860
tggtattggg cgcaacagac gcaggtacag attagcggtg tgccgtaatc gtacgaatgc 313920
cgattcaacc taagcagaca tcagtattta ggaagtggat gtttgatgga gcaaaggttg 313980
tacgaagggt ggaaggcaac ctgtgggtgt ttggtatggt cgcgcttgaa aaaacgtgtt 314040
ttaagggaca aatgccgtct gaaaatcggt ttcagacggc attttctgtt tatttaaagc 314100
aaacaggaaa aggcagcaat attctgcagt cttcctattc acacaagcgt tttatagtta 314160
attaaaaaca aaatagtaca atactcaact ttgaaggtct aaccatggca tactctgcgg 314220
acttaagaaa caaagcttta aactaggggc tgtactagat tagcagatat gttaccctcg 314280
aaatatgaag ataacgcact gcaaattaaa gaaaaaagta cagaaagaac tgctccgttt 314340
tttgtgctgg aagttaccgc ccgttctgcc gccgatattt tgggtatcca tcccaattcg 314400
gcagtactgt tctaccgtaa aatccgcacg gttatcaacc atcatttggc cttggctgcc 314460
gatgaggttt ttgagggccc tgtcgagccg gacgaaagcg atttcggcgg acggcgtaaa 314520
ggcagacgtg gtcgcggtgc ggcaggaaaa gtggttgtct tcggcattct gaaacgcaac 314580
ggacggggct ataccgttgt cgtagataat gccaagtctg aaacgttact ccctgtcatc 314640
aaaaagaaaa tcatgccgga cagtattgtt tataccgata gtctgagcag ctgcgacaag 314700
ttggacgtga gcggttttat ccattaccgc atcaaccatt ccaaggaatt tgcagaccgt 314760
cagaaccaca ttaacggcat tgagaatttt tggaatcagg caaaacgcgt cttgcgaaaa 314820
tacaacggaa tcgatcgtaa atctttcccg ctgttcttga aagaatgcga atttcgattt 314880
aacttcggca caccgtctca acagcttaaa atcctgcggg attggtgtgg aatttagggc 314940
taatctagta cagcacctaa caaaaaccag tacggcgttg gctcgcctta gctcaaagag 315000
aacgattctc taaggtgctg aagcaccaag tgaatcggtt ccgtactatt tgtactgtct 315060
gcggcttcgt cgccttgtcc tgatttttgt taatccacta tattttagat aatgcgtgat 315120
ttcaccgtat gggtgtctta cgggaaatgg cggaaaaatt gggacataag gtattgcctc 315180
ttgcacctta ttcacctgag ctcaacccga ttgagaaagt gtgggcgaat attaagcggt 315240
atctgcgaac cgttttgtct gattacgccc gatttgacga tgcactactg tcctattttg 315300
attttaattg actatagaac gttgcggcta cgcggaagcc gtactcgttg gatttggagc 315360
ggcccatttt ggttttgtca ccgtccaaga caatctcacg gggtttgtag attgttttgt 315420
gacggtagta tggatcaaac tcgagaccga cgctgtcggt caactgtttg cctacattca 315480
gaccgatacc gacactccaa cctttggcgc ttttgctgac atcgcgggaa gcacccatct 315540
gggtcgtcat cactttggtt ttgccgcgca aatctgcata tgcatccgcc caaggggtca 315600
gggatcatcc gtcccccaaa tcttggcgga tttcgccatg gactttcaaa gcaaggtttt 315660
catgcttggt aacggtgttt ttccttatcg ccgatgatgg ctttgccttt gccgttagac 315720
tcgggaatat cggctaccgt aacggcggac acggctgcaa gtgagagtgc aagcagggtt 315780
ttttcatgtt tttcttccta taatgaggat aaataaatgg aaaaagtgtg ggaaataccc 315840
gcattcccat taaatctttt ttcaagcaat gagttctttt tgttttcaac attttccttg 315900
agacctttgc aaaaatagtc tgttaacgaa atttgacgca taaaaatgcg ccaaaaaatt 315960
ttcaattgcc taaaaccttc ctaatattga gcaaaaagta ggaaaaatca gaaaagtttt 316020
gcattttgaa aatgagattg agcataaaat tttagtaacc tatgttattg caaaggtctc 316080
tccttgtgta tgaaattttg ccggatgtga aggcggaatc ggcagcgggg gtgttctgta 316140
ccggattgtc gtggaaatgg gaaaacggat gttccgtgca ggtttgtcca aatgaatggc 316200
gggtattgtt tttatcaatc tgtttctttt tatttgaaat aaaatttcta aaataataaa 316260

aatatgaaat ttaaaatcta taaaaaaaga tatatcagtt attttgaaat aaaatagctt 316320
tgtagtaata tgttgcactt gtttgtgcaa ggtaaacgat gtaacctaag ccgcgtataa 316380
aaacccatca ggaaagatgc aagatgacac accattaccc cacagacgat attaagatta 316440
aagaagttaa agagttgttg ccgccgatag cccatcttta cgagctgccg atttccaaag 316500
aggcttcggg cttggttcac cgcacccgtc aggaaatttc cgatttggtt cacggcaggg 316560
acaagcggct gttggttatt atcgggccgt gttcgattca cgatccgaaa gcggcgttgg 316620
aatatgcgga gcgtttgttg aaactccgca agcagtatga aaacgagctt ttgattgtga 316680
tgcgcgttta tttcgagaag ccgaggacga cggtgggttg gaaaggtttg attaacgacc 316740
cgcatttgga cggtacgttt gacatcaatt tcggtttgcg tcaggcgcgc agcctgttgt 316800
tgtcgctgaa caatatgggt atgcctgcct ctaccgagtt tttggatatg attacgccgc 316860
aatattatgc ggacttgatt tcttgggggg caatcggtgc gcggacgacc gaaagccaag 316920
ttcaccgcga attggcaagc gggctgtcct gccccgtcgg ctttaaaaac ggtacggacg 316980
gcaatttgaa gattgccatc gacgcaatcg gtgcggcgag ccattcgcat catttcctgt 317040
ctgtaaccaa ggccgggcat tccgccattg tccataccgg cggcaatccc gactgtcatg 317100
tcattttgcg cggcggcaaa gagccgaatt atgatgcgga acacgtcagc gaggcggcgg 317160
aacaactgcg tgcggcaggg gtaaccgaca agctgatgat agattgcagc cacgccaaca 317220
gccgcaagga ttacactcgg cagatggaag tggcacaaga cattgccgcc caattggaac 317280
aggacggcgg caatatcatg ggcgtgatgg tggaaagcca tttggtcgaa ggcagacagg 317340
acaagccgga agtgtacggc aagagcatta ccgatgcgtg tatcggttgg ggcgcgactg 317400
aagaactgtt ggcattgttg gcaggtgcaa acaaaaaacg tatggcgcgc gccagttgag 317460
atttttgacg cagaatgtca taaaatgtcg tctgaagcgt tcagacggca tttttgtgga 317520
ggaaatatgc tcaaaataac cctaattgcg gcgtgtgcgg aaaacctgtg catcggggcg 317580
ggcaatgcta tgccttggca catccccgaa gatttcgcat ttttcaaagc ctataccttg 317640
ggcaaacccg tcattatggg gcggaaaacg tgggaatccc tgcccgtcaa acccctgccc 317700
ggacggagga acatcgtcat cagccggcag gcggattatt gcgcggcagg cgcggaaacg 317760
gcggcaagtt tggaggcggc attggcattg tgcgcaggcg cggaagaagc cgtcattatg 317820
ggcggcgcgc agatatacgg acaagcgatg ccattggcga ccgatttgcg gataaccgaa 317880
gtggatttgt ctgtggaagg agatgcattt ttccccgcaa tagaccggac gcattggaaa 317940
gaagcagagc ggacggaacg ccgtgtcagc agcaaaggca cgcgctatgc ttttgtgcat 318000
tatttgagat attgaaatat aaactctcta taaaatcccc cgcaaatgat gggctgaaat 318060
agaaaatatt gttattcccc cgaagatggg aatccgggat tttaaagtta gggtaattta 318120
tccgaaataa caacaatctt ccatcgtcat tcccgcaaaa gcgggaatcc ggaaacgaaa 318180
agctaaagca atttatcgga aaaaaccgaa gtttaaagaa ccggattccc gcctgcgcgg 318240
gaatgacgag attttaggtt atggggattt attgggaata atggaacaaa gaaagcagaa 318300
ataaggatat agaggctgtc tttggatttg cgatggttgt cggagaatgc cgtctgaagc 318360
cgtttcagac ggcatttttc cagcttgaga acggatgcct gctcaaataa gcattggtaa 318420
acataccgtc ggcagtgatt tcccgtccca gccagtccgg acggtcaaaa tcggcattct 318480
cgtcgggcaa ctcgatttcc gcgacgacca aaggcgcatt atcgccaaga aaaacatcga 318540
tttcaaacag gctgccgccc catctgaccg gataacgcca tttttccatt ttaaacgggc 318600
acatcgtttc catcatcttt tccgcatcgg caagcgggat ttcgtattca aactcactgc 318660
ggctgatttc cgaaatatag cctttcagcg tcagccacgc ctgttttccg gcaatgcgga 318720
cacggacggt gcgttctttt tcaacagaca gataaccctg cctcaacagc agcggttcgt 318780
cggcgtattg ccgccagttg tcgtttccaa tcaaaaaacg gcgttcgatt tctatcggca 318840
taagatgctc cgtcaaaacg gtttgaacac gaccagatac agcgcggcaa ccatcagcag 318900
cacggggatt tcgttgaaca cgcggtacca gcggtgtgaa aaagcattgc tgtaatcctg 318960
aaaacggcgc agcagcacgc cgcaatacaa ctggtaagcc aagagcatca agcccaaaca 319020
cagtttgacg tgtacccagc cgctgcccca ccagccggcg gcaaacggta tcgccgcgcc 319080
gaacacgacc gcgccgaagc ccaacggcga cataaaacgg tacagccgca ccgccatgcc 319140
cgacagacgc acatactcgg gattgccgcg cggcacatca atcatcgcca tattgacgaa 319200
aatcctcggc aggtaaaaca gccctgcaaa ccacgaaatg acaaaaaaca agtgaaacag 319260
cttgaaccaa gaaaacatca tcgcccacac cctgccgaaa agcggtattg tacaggcaaa 319320
ccgcttggga aacgtgataa aatcaggcgg ataaacaaat cgaataaatc cttaccgcaa 319380
aacggaggca aaatgctcaa atccatcgaa ctcaattccc acatccgcaa ccgccttgca 319440
gaatatctga aaggcagggg tatggatttt cagacggcaa tgcaggaaga aaaaggcaac 319500
aaagaaatcg ccgccatcgt ccacagcggt ttgcccactc tggtccgcaa actgtattcc 319560
gaacaaaaaa tgcagaagtt tttttgggaa aagcgggatt tgattgccga ctacatcagc 319620
cgccggatgc agggataggt ggctgaaatc tgttttcagg caagtgaaaa gacaatatgg 319680
cagattgaaa ttacgcttat cgtcattccc gcccgcgcgg gaatccgact tgtttggttt 319740
cggttatttt tcgtttcgta acttttgagc cgtcattccc gcgcaggcgg taatccggct 319800
tgttcggttt cggttctttt tctcgtttcg ggtgatttct aaaccgtcat tcccgcgcag 319860
gcgggaatct aggtctttaa acttcggttt tttccgataa atttttgccg cattaaaatt 319920

ctagattccc gctttcgcgg gaatgacggc ggagggtttt tagttttccc gaaaatgcac 319980
atcatccaaa atcccgttat tcccacaaaa cagaaaatca aaaacagcaa cctgaaatcc 320040
cgtctttccc gcgcaggcgg taatctgaac acgtccgtag tgaaacctat atcccgtcat 320100
tcgcacgaaa gtgggaatcc aggatgcagg gaaaaccgtt ttatccgata agtttccgca 320160
ccgaaaggtc tagattcccg ctttcgcggg aatgacggcg gagggttttt agttttctcg 320220
ataaatgcac atcatccaaa gtcccgttat tcccacaaaa acagaaaatc aaaaacaaca 320280
atctgaaatt ccgtccttcc cgcctgtgcg ggaatccggc ttgttcggtt tcggttcttt 320340
ttctcgtttc gggtgatttc taaaccgtca ttcccgcgca ggcgggaatc taggtcttta 320400
agcttcggtt tttcttgata aattcttgcc gcattaaaat tctagattcc cgctttcgcg 320460
ggaatgacgg cggagggttt tttgttttcc cgataaatgc acatcatcca aagtcccgtt 320520
attcccacaa aaacagaaaa tcaaaaacag caacctgaaa tcccgtcctt cccgcgcagg 320580
cggtaatctg aacacgtccg tagtgaaacc tatatcccgt cattcgcacg aaagtgggaa 320640
tccaggatgc agggaaaacc gttttatccg ataagtttcc gcaccgaaag gtctagattc 320700
ccgctttcgc gggaatgacg gcggagggtt tttagttttc tcgataaatg cacatcatcc 320760
aaaatcccgt tatttcccaca aaacagaaaa tcaaaaacag taacctgaaa tcccgtcatt 320820
cccgcgcagg cgggaatccg gcttgttcgg tttcggttct tttcttgtt tcgggtgatt 320880
tctaaaccgt cattcccgcg caggcgggaa tccagacctt taaaccccga ccatccttga 320940
taaattcttg cggcattaaa attctagatt cccgctttcg cgggaatgac ggcggagggt 321000
tttttgcttt tcctgatttt tcattgcgat gtagtataat gtagtatata atcattataa 321060
ttttaacact tgacaaagga aaatttctca tgacactgaa agcaagcaag caagcaagca 321120
agcaagcaag caagcggtcg ggttaatcta ttaacattat ctgttttatc gctgttttgc 321180
acgccatatg tttgaggttc ggatgcgtac gatcccgtca aagaagccga gattaaaaac 321240
aaatttattt tagaagcggc ggaagacaga aattcccacg tttggcgcgg cccgtgcagc 321300
atatcttttg attgcttcgg tatgttcaga gctcagcttg gttcaaatac tcgttctacc 321360
aaaatcggcg acgatgccga tttttcattt tcagacaagc cgaaacccgg cacttcccat 321420
tatttttcca gcggtaaaac cgatcaaaat tcatccgaat atgggtatga cgaaatcaat 321480
atccaaggta aaaattacaa tagcggcatc ctcgccgtcg ataatatgcc cgttgtcaaa 321540
aaatatatta cagagaagta tggggctgat ttaaagcagg cggttaaaag tcaattacag 321600
gatttataca aaacaagacc ggaagcttgg gcagaaaata aaaaacggac tgaggaggcg 321660
tatatagcac agtttggaac aaaatttagt acgctcaaac agacgatgcc cgatttaatt 321720
aataaattgg tagaagattc cgtactcact cctcatagta atacatcaca gactagtctc 321780
aacaacatct tcaataaaaa attacacgtc aaaatcgaaa acaaatccca cgtcgccgga 321840
caggtgttgg aactgaccaa gatgacgctg aaagattccc tttgggaacc gcgccgccat 321900
tccgacatcc atacgctgga aacttccgat aatgcccgca tccgcctgaa cacgaaagat 321960
gaaaaactga ccgtccataa ggattatgcg ggcggcgcgg atttcctgtt cggctacgac 322020
gtgcgggagt cggacgaacc cgccctgacc tttgaagaca aagtcagcgg acaatccggc 322080
gtggttttgg aacgccggcc ggaaaatctg aaaacgctcg acgggcgcaa actgattgcg 322140
gcaaaaacgg cggattccgg ttcgtttgcg tttaaacaaa attaccggca gggactgtac 322200
gaattattgc tcaagcaatg cgaaggcgga ttttgcttgg gcgtgcagcg tttggctatc 322260
cccgaggcgg aagcggtttt atatgcccaa caggcttatg cggcaaatac tttgtttggg 322320
ctgcgtgccg ccgacagggg cgacgacgtg tatgccgccg atccgtcccg tcaaaaattg 322380
tggctgcgct tcatcggcgg ccggtcgcat caaaatatac ggggcggcgc ggctgcggac 322440
gggtggcgca aaggcgtgca aatcggcggc gaggtgtttg tacggcaaaa tgaaggcagc 322500
cgactggcaa tcggcgtgat gggcggcagg gccggccagc acgcatcagt caacggcaaa 322560
ggcggtgcgg caggcagtga tttgtatggt tatggcgggg gtgtttatgc tgcgtggcat 322620
cagttgcgcg ataaacaaac gggtgcgtat ttggacggct ggttgcaata ccaacgtttc 322680
aaacaccgca tcaatgatga aaaccgtgcg gaacgctaca aaaccaaagg ttggacggct 322740
tctgtcgaag gcggctacaa cgcgcttgtg gcggaaggca ttgtcggaaa aggcaataat 322800
gtgcggtttt acctacaacc gcaggcgcag tttacctact tgggcgtaaa cggcggcttt 322860
accgacagcg aggggacggc ggtcggactg ctcggcagcg gtcagtggca aagccgcgcc 322920
ggcattcggg caaaaacccg ttttgctttg cgtaacggtg tcaatcttca gccttttgcc 322980
gcttttaatg ttttgcacag gtcaaaatct ttcggcgtgg aaatggacgg cgaaaaacag 323040
acgctggcag gcaggacggc actcgaaggg cggttcggta ttgaagccgg ttggaaaggc 323100
catatgtccg cacgcatcgg atatggcaaa aggacggacg gcgacaaaga agccgcattg 323160
tcgctcaaat ggctgttttg atgcgtcggg aaatgttttg acgcacaggc ggtacaccgg 323220
cacggcaccg cgcgccgccc cgcaaaccaa tccgaaccct gccgccccga agggcggggc 323280
ataatgatga aaccggcgga aaaccgccgg ttttttgccg ccgtttgaaa cccgattctg 323340
gcttcagacg gcattgtcgc ggcatcgggc ggcagggttt ggaacagcgg cataaaaaac 323400
tgatacaatc cgccgattga taatggttat tttttatttt tgtgggaaga catttatgcc 323460
tgcacgaaac agatggatgc tgctgctgcc tttattggca agcgcggcat atgccgaaga 323520
aacaccgcgc gaaccggatt tgagaagccg tcccgagttc aggcttcatg aagcggaggt 323580

caaaccgatc gacagggaga aggtgccggg gcaggtgcgg gaaaaaggaa aagttttgca 323640
gattgacggc gaaaccctgc tgaaaaatcc cgaattgttg tcccgcgcga tgtattccgc 323700
agtggtctca aacaatattg ccggtatccg cgttattttg ccgatttacc tacaacaggc 323760
gcagcaggat aagatgttgg cactttatgc acaagggatt ttggcgcagg cagacggtag 323820
ggtgaaggag gcgatttccc attaccggga attgattgcc gcccaacccg acgcgcccgc 323880
cgtccgtatg cgtttggcgg cagcattgtt tgaaaacagg cagaacgagg cggcggcaga 323940
ccagttcgac cgcctgaagg cggaaaacct gccgccgcag ctgatggagc aggtcgagct 324000
gtaccgcaag gcattgcgcg aacgcgatgc gtggaaggta aatggcggct tcagcgtcac 324060
ccgcgaacac aatatcaacc aagccccgaa acggcagcag tacggcaaat ggactttccc 324120
gaaacaggtg gacggcacgg cggtcaatta ccggctcggc gcggagaaaa aatggtcgct 324180
gaaaaacggc tggtacacga cggcgggcgg cgacgtgtcc ggcagggttt atccggggaa 324240
taagaaattc aacgatatga cggcaggcgt ttccggcggc atcggttttg ccgaccggcg 324300
caaagatgcc gggctggcag tgttccacga acgccgcacc tacggcaacg acgcttattc 324360
ttacaccaac ggcgcacgcc tttatttcaa ccgttggcaa accccgaaat ggcaaacgtt 324420
gtcttcggcg gagtgggggc gtttgaagaa tacgcgccgg gcgcgttccg acaataccca 324480
tttgcaaatt tccaattcgc tggtgtttta ccggaatgcg cgccaatatt ggatgggcgg 324540
tttggatttt taccgcgagc gcaaccccgc cgaccggggc gacaatttca accgttacgg 324600
cctgcgcttt gcctggggggc aggaatgggg cggcagcggc ctgtcttcgc tgttgcgcct 324660
cggcgcggcg aaacggcatt atgaaaaacc cggctttttc agcggtttta aggggaaag 324720
gcgcagggat aaagaattga acacatcctt gagcctttgg caccgggcat tgcatttcaa 324780
aggcatcacg ccgcgcctga cgttgtcgca ccgcgaaacg cggagtaacg atgtgttcaa 324840
cgaatacgag aaaaatcggg cgtttgtcga gtttaataaa acgttctgat tgctgttcct 324900
tttcggagga aaccctgccg gcggcggtat cacggcgggc atcggcggct ttcgggcggt 324960
gctttgcgtg ccgccgcgtg tgcggaaacg cattccggtt tttccggcat aacggcgatg 325020
cgaggtaaaa tgccgtctga aacccgattc gggcttcaga cggcattgtc gcggttgcgg 325080
cgggcgggtt caccagattc cgtcaaaggt tttcgcgccg cgccaaaatt tccacctgtc 325140
ggcgggtttg aaggtcagcg taccgccgtg ttgtccgtcc gtggtgatgt ccagccgttt 325200
gattttgccg gtgcggacgg cttcgtagat tggtgcgaac cagcgttctt cccactgctg 325260
caatattgcc gcataccgct ccctgtcccc tgtcagggcg gtcaggcgca aatcgtccat 325320
aaacaggata tggtgcgtgt cgggcaggtg tgccgccgtt tcttcatagg cgcggaagtt 325380
gtcgggtaat gcgcggcggt cggagtggaa acggctccaa accgtatcgg cgaaaagcgt 325440
gccgccttgc gcgccgccgt ttgtgccgtc ccaaagccat aagccgttca actcgggcag 325500
cccgcgtttc ttgcggttat ggttgacggg gtgcgccgcc agccacattt ggatttcggt 325560
ttggacgcgc agccattcca acgcatcttc tccgtccggc tgatcgtcag cgcccaacaa 325620
tccgcccaag tccaaaacgg gcttcgcgcc ccagcggtac gcgcaaggaa gggaaaccag 325680
ccataattcg ggcaggacgg gaacgaaacg ccatggaatg tcgccgtaaa acgccgacag 325740
gtcgcggcag atccgttccg cttcatccgt accgacgttc agatattccg ccgttagcac 325800
atttgcctga tgcatcccca tcttttgcca gacgggcgtg gcgagcgcga cggcttcaga 325860
cggcatattc aggctttgcg ccgcgcgttc caccagtctg ccgcaccaca aataacgcgc 325920
gtaaaatgcc gaagccgtgc agctttggcg gtgcagcgag ccgtattgca ggattttgtt 325980
gaaagcgtgc aggcatagag gtattcggat ttcgtcttca tccaaattga gcgagggaat 326040
ggcgagggtg agtttcatcg tttgacgttt cagaaatgca ggtcaggcgc aacattatag 326100
aggattcggc gcaaacgccg tcaaaaagga acaatatggc tgtcttccca ctttcggcaa 326160
aacatcggaa atacgcgctg cgtgcgcttg ccgtttcgat tattttggtg tcggcggcat 326220
acattgcttc gacagagagg acggagcgcg tcagaccgca gcgcgtggaa caaaatctgc 326280
cgccgctgtc ttggggcggc agcggcgttc agacggcata ttgggtgcag gaggcggtgc 326340
agccgggcga ctcgctggcg gacgtgctgg cgcgttcggg tatggcgcgg gacgagattg 326400
cccgaatcac ggaaaaatat ggcggcgaag ccgatttgcg gcatttgcgt gccgaccagt 326460
cggttcatgt tttggtcggc ggcgacggcg gcgcgcgcga agtgcagttt tttaccgacg 326520
aagacggcga gcgcaatctg gtcgctttgg aaaagaaagg cggcatatgg cggcggtcgg 326580
cttctgaggc ggatatgaag gttttgccga cgctgcgttc ggtcgtggtc aaaacgtcgg 326640
cgcgcggttc gctggcgcgg gcggaagtgc ccgtcgaaat ccgcgaatcc ttaagcggga 326700
ttttcgccgg ccgcttcagc cttgacggtt tgaaggaagg cgatgccgtg cgcctgatgt 326760
acgacagcct gtatttccac gggcagcagg tggcggcggg cgatattttg gcggctgaag 326820
tcgttaaggg cggcacaagg catcaggcgt tctattaccg ttcggacaag gaaggcggag 326880
ggggcggcaa ttattatgat gaagacggca aggtgttgca ggaaaaaggc ggcttcaaca 326940
tcgagccgct ggtctatacg cgcatttctt cgccgttcgg ctaccgtatg caccccatcc 327000
tgcacacatg gcggctgcac acgggcatcg attatgccgc accgcaggga acgccggtca 327060
gggcttccgc cgacggcgtg attaccttta aaggccggaa gggcggatac ggcaacgcgg 327120
tgatgatacg ccacgccaac ggtgtggaaa cgctgtacgc gcacttgagc gcgttttcgc 327180
aggcggaagg caatgtgcgc ggcggcgagg tcatcggttt tgtcggttcg accgggcgtt 327240

cgaccgggcc gcacctgcat tacgaggcgc gcatcaacgg gcagcccgtc aatcctgttt 327300
cggtcgcatt gccgacaccg gaattgacgc aggcggacaa ggcggcgttt gccgcgcaga 327360
aacagaaggc ggacgcgctg cttgcgcgct tgcgcggcat accggttacc gtgtcgcaat 327420
cggattgaag tttgaaccgg cgacgaaaac aatgccgtct gaaaacctgc aaacaggttt 327480
tcagacggca tttatagtgg attaacaaaa atcagtacgg cgttgcctcg ccttagctca 327540
aagagaacga ttctctaagg tgctgaagca ccaagtgaat cggttccgta ctatttgtat 327600
tgtctgcggc ttcgtcgtct tgtcctgatt tttgttaatc cactatgcag ttgattaaaa 327660
caaaactaag ccaaggaagc actgccgtca ttcccgtacg ggcgggaatc ctgacaccac 327720
ggcacggaaa cccatccgct gtcattccca cgaaagcggg aatctagaaa tacaacgcgg 327780
caggagttta tcggaaatga ctgaaaccca acgtaccgga ttcccgcttt cgcgggaatg 327840
acgaagtggg cgggaatccg gatttatccg ttccgacagt gtttgcaaat aaaagaaaac 327900
ccaaccgtcc cgattcccgg cagggctgtt ttacggattt tgcagcgagg gcgcggggcg 327960
gtcttgcgcc tgtttggttt gcagggttgt cagttttttc gtcagcagat tcagtatcac 328020
gccgtaggcg ggcaggaaga agagggtgca gacggtaagt ttgaacaggt aatcgacaaa 328080
agcgatgccc tgccagtttg ccgccataaa tccatcgctg cttgcgtaga aggcaacggc 328140
gaaaaatacc agcgtatcca aggcgttgcc gatgacggtt gatgcggtcg gtgcaatcca 328200
ccacgctttc agacggcgta atttgttgaa tacaaaaata tcaaggattt gtccgatcgc 328260
gtaggcggca aagctggcta aggcgatgcg tccgacaaag gtgttgaatt cggacagcgc 328320
gcccaagcct gtccaactgc cgttgtggaa caaaacggaa aagacgtagg aaagcaaaag 328380
ggcggggaac atcacccaaa agataatccg ccgtgccaag tgagaaccga aaatgcggac 328440
ggtcaggtcg gtggcaagga agatgaaggg aaaggaaaat gcgcccccaag tggtgtggat 328500
gccgaaaatt tggaaaggga actgcaccag atagttgctg gcggcgatga tgaggatatg 328560
aaaaagcacc agccggaaga gtgccttctg ttgctgtgcg gcggtaaatg cgtacataaa 328620
aatctttcgg aaaggcgttc agacggcata tcgtatcgaa ggaatgccgt ctgaaatatg 328680
ggaaggatgg tttattgtgc gtcgtgctca aacaagcgtt tgcgtgccaa tgtttcgaac 328740
tcggtgcctg cttttccgta gttggcaaac ggatgaatgg cgatgccgcc gcgcggtgtg 328800
aactcgccga atacttcgat gtatttcgga tccatcaggg caatgaggtc tttcatgatg 328860
atgttgacgc agtcttcatg aaaatcgccg tggttgcgga agctgaagag gtagagtttc 328920
agggatttgc tttccaccat tttgatgtgc ggaatgtagc ggatgtagat ggtggcgaag 328980
tcgggctgcc cggtcatggg gcagaggctg gtgaactcgg gacagacgaa tttgacgaaa 329040
tagtcgttgt cgggatgttt gttgtcgaat gcttcgagaa tttcaggcgc gtagccggtc 329100
ggatattggg tttttttgatt gcccaaaaga gagatgcctt gcagctcttc gttgttgcgg 329160
gacatgaggg tttccttagt tttttaatgt gggaggtttt cgaaccacgg gcggcgattg 329220
taatataagc ggcggtatct gtgtagtttt cttcagacgg catggtttgg acggcggcgt 329280
tttccgtgtc atatatagtg gattaacaaa aaccagtacg gcgttgcctc gccttagctc 329340
aaagagaacg attctctaag gtgctgaagc accaagtgaa tcggttccgt actatttgta 329400
ctgtctgcgg cttcgccgcc ttgtcctgat ttttgttaat ccattatata aacgaaatat 329460
attttcagtt ttgccgcctg aagcgttgtt ttttgaatat tgcatctaaa atactgactt 329520
gattgcgtta ttgcgcggat atagaatctg cttcctattg aaagaacatt gtttatatga 329580
aatcaggaaa ttcggaaccc aatcttatgg atacgcacac ggacgaaaca aaacttcaaa 329640
acacgcaagc caaacgcaaa cgccgcctga cggcattgac gctgctgttc gcgcttgccg 329700
ccgcagccgc cgggtcggcg ttttttttat ggtggcagca cgaagaggaa acggaagacg 329760
cttatgttgc cggacgcgtg gttcaggtta cgccgcaaaa gggcggtacg gtgcggaagg 329820
ttttgcacga cgatacggat gccgtgaaaa aaggcgacgt gctggcggta ttggacgacg 329880
ataatgatgt gctggcttac gagcgggcaa aaaacgagct ggttcaggcg gtgcggcaaa 329940
accgccggca aaatgccgcc acttcgcagg cgggggcgca ggttgccttg cgccgggcgg 330000
atttggcacg cgcacaggat gatttgcgcc gccggtctgc tttggcggaa tcgggcgcgg 330060
tgtccgccga agagctggca cacgcccgtg cggcagtgtc tcaggcgcag gcggcggtca 330120
aagcggcttt ggcggaagaa tcttcggcac gtgcggcttt gggcggtcag gtttctttgc 330180
gcgaacagcc ggcggttcag acggcaatcg gcaggttgaa agatgcgtgg ttgaaccttc 330240
agcggacgca aatccgcgcg ccggcggacg gtcaggtggc gaagcgttcg gtgcaggtcg 330300
ggcagcaggt ggcggcaggc gcgccgctga tggcggtggt gccgctgtcg gatgtgtggg 330360
tggatgctaa ttttaaagag acgcagttgc ggcatatgaa aatcggacag cctgccgagc 330420
tggtgtccga tttgtacggc aaacaaattg tttatcgcgg cagggtggca ggtttttcgg 330480
caggtacggg cagcgcgttt tcgctgattc cggcgcaaaa cgcaacgggc aactggatta 330540
aagtggtgca gcgcgtcccc gtccgtatcg tgctgaaccg cgaagatgtg gacaggcatc 330600
cgttgcgtat cggtttgtcg atgacggtta aagtggatac ttccgccgca ggcgcgcctg 330660
tttcaaaaac gccgggtgcg gcattgccgg aaatggaaag taccgactgg tcggaagtcg 330720
atcggacggt cgatgaaatc ctcgggcaat ccgcgccctg atgccgtctg aaacggagga 330780
cacaatggat tatccaccgc ttaagggtgc ggcattggcg tgggttacgc tgtctttggg 330840
gcttgccgta tttatggaag ttttagatac gactatcgcc aatgtcgccg ttcccgtcat 330900

cgccggcaac ctcggtgcgg caaccactca ggggacgtgg gtcatcactt ccttttctgt 330960
ggcaaacgcc gtttccgtgc cgctgacggg ctttttggca aaacgcatcg gcgaggtcaa 331020
attgtttacc gccgccgctg tcggtttcgt catcacatcg tggctgtgcg gtattgcccc 331080
caaccttcag tcgctggttg ttttccgcat cttgcagggc tttatcgccg ggccgctgat 331140
tcccttgtcg caaagcctgt taatggcatc ctatccgccc gcaaaacgga cgctggcact 331200
ggcattgtgg gcaatgaccg tcgttgtcgc ccctgttctc gggccgatac tcggcggctg 331260
gatttccgga aactggcatt ggggttggat ttcttcatt aatatcccta tcggtatcat 331320
atcggcatgg attacatgga aacatttgaa atatcgggaa acggaaaccg ttaaaatgcc 331380
gaccgactat gtcgggctta cattgatggt agtcggtatc ggcgcgttac agatgatgct 331440
ggacaggggt aaggaactcg actggttcgc ctctggagaa atcattacct tgggcgtagt 331500
cgcactggtg tgcttgtcgt attttattgt ttgggaattg ggagaaaaat atccgattgt 331560
cgatttatcg ctgtttaaag atcggaattt taccgtcggc gtcattgcca cgtcattggg 331620
ttttatggtg tatatgggga cgctgaccct gctgccgtta gtgttgcaga ccaacctggg 331680
ctatacctcc acgtgggcag ggcttgccgc cgcacctgtc ggcatcctgc ctgttttcct 331740
gtctccgtta atcggcaggt tcggcaataa aatcgatatg cgcctgttcg taactgccag 331800
cttcctgacc tttgccttta ctttctattg gcgtacggat ttttatgccg atatggatat 331860
tggcaacgtc atctggccgc agttttggca gggtgtcggt gtcgccatgt tttttctgcc 331920
gctgaccacc atcacactgt cgcatatgaa gggcgggcag attgccgccg caggcagcct 331980
gtcgaatttc ttgcgcgtgc tgatgggcgg tgtcggcgta tccgtcgtca gcaccctgtg 332040
ggaacggcgc gaagcgttgc accacacacg ctttgccgaa cacatcacgc cctattccgc 332100
aacattgcac gaaacggccg ctcatttgtc ccagcacggc gtttccgaca ttcaaaccct 332160
aggcatcatc aacaatacca ttacccagca gggttttatt atcggctcga acgaaatctt 332220
tatggcgggc agcttgttat tcattatcat gatacccgtc atatggctgg caaaaccgcc 332280
gttccacaac ggcggcggcg gtggacattg agggatttga aaacttgaaa tgccgtctga 332340
aaatactgga aatatgttcg gacggcattt tgaatgcagc agttcccgaa atccgctata 332400
atcgcgcccc atctgtttcg cacctgcaaa cgttccacag atgcgacaat cggaaggatt 332460
atccgcgcaa aacagccgtt tttctttaaa acacttgaac taacactgtt tttcgtggta 332520
taaatcgcgt tttactattt tagaagtttg gagactgatt atggcacgag tttgcaaagt 332580
gaccggcaaa cgcccgatgt ccggcaacaa cgtatcgcac gccaacaaca aaaccaaacg 332640
ccgtttttttg cccaacttgc aatcacgtcg tttttgggta gaaagtgaaa accgctgggt 332700
tcgcctgcgc gtttccaacg ctgcactgcg taccatcgac aaagtaggca ttgatgtcgt 332760
attggctgat ttgcgtgctc gcggcgaagc ttaatttaaa cactatttaa ttaaggatta 332820
ctgcaatgcg cgataaaatc aaactggaat ccagtgcagg tactggtcac ttctacacca 332880
ctaccaaaaa caaacgcact atgcccggca aattggaaat caaaaaattt gacccagttg 332940
cccgcaaaca cgtagtgtat aaagaaacta aactgaaata atttcagttt gaaagcaaag 333000
cctccgactg ctcggaggct ttgttatttt tatcgtgttt cctttccgct tgaaacatct 333060
gccgtatgcg aatctgctgc aaaccgtctg ccaaggatat gaaaaccgca aaacggttca 333120
taacacaaaa atgccgtctg aaacgtttca gacggcattt cggcagtttt caaccggtca 333180
gttgtttggt gatcagtttc ttcagcggtg ggaaattgtt gctggcacgc aataccaagc 333240
cgcgcaacag ttttgccggt gcggtctcat tggtaaacag tttcagcatc atattggttc 333300
cgtgataaag cggatgggcg tgcagcatat gtttgctgct gtatttttcc aataatgaag 333360
atgcaccgat gtcttgaccg cgctgttcgg cttcgagtat cagttttgcc aaaatatctg 333420
cgctggaaag ccccaagttg aaaccgtgtg ctgtaacggg gtgcataccg acggcggcat 333480
cgccaatcag cgcgctgcgt ttgccgtaga aacgtttggc aatcatgccg acaagggggt 333540
aatggtggat gctgctgacc aattccatat cgccgagcct gcccttgagc tgttctttta 333600
cgcttgccgc caattcttcg ggcgaaaggt tttgaacgct gttgatttta tcggtatcga 333660
cggtaatgac ggtattggtc aggtgctctt ccagcggcag cagtgcgatg gtgcgtccgt 333720
aatggaagca ttcgtaagcg gtatgttggt tggaaagggt atgtttcata cggcagacga 333780
acatggttcg gctgtaatcg tgcatatcgg aggagatacc gagttgtcga cgggtttgcg 333840
agaagcggct gtctgccgcc aaaagcaggc gtgcagtcag tattttgccg ttttccaaaa 333900
tgacttgtgc ttcgttgtca gatgttttga cttctttgac aaccgtatcg gtcagaatgc 333960
tgacattgtc gagttgtgat acgacttcat aggcggcgcg gcggatattg tggttggaaa 334020
tcagatagcc caaacagtcg gcaggttcgc cgcgcgcttc agtcggttgg ggaaagtgga 334080
gctggtagtc ggaacgtccg ttcagcactt tggcatcgcg caaagggtag atttcgtttt 334140
cgggaatttt gtcccacata cccaaacgct gcatgatttc gcgggaaaaa tgggtcaggg 334200
cgatttcgcg tccgtcatat ggaggatttt gcagaacagt cagtgggctg cgttcgatca 334260
gggtaacttt caaaccgctg ccggcaagtt cggctgcaaa acttaaaccc gccgggcctg 334320
cgccgacgac gaggatgtcg ctgtgtaaac tcataaaata tcctttgcat agacggatgc 334380
cgatgatttc agacggtatt tgtaagggtt tgaatgccgt ttgaactatc tgtaacagat 334440
aggcgattat atcaaaaccc actgttgaag aaatatgcag gggagggtgt atgcggattt 334500
ttactttcag cttaatgtgt atcaaatcgg gtgtggggta tgtatagtgg attaaattta 334560

aaccagtacg gcgttgcctc gccttgccgt actatttgta ctgtctgcgg cttcgtcgcc 334620
ttgtcctgat ttttgttaat ccactataaa aagccgcatc gtgaaaagat gcggcttcag 334680
gtatcggttg gattattctt cagaaccggt gtaaggacgg atgctgacag ttttacggtt 334740
cagcgcgcct ttggttttga attcgacata accgtcaact ttggcgaaca aagtgtggtc 334800
tttgcccata cctacgttgt cgcctgcgtg gaatttggta ccgcgttggc gtacgatgat 334860
ggaacctgcg ggaatcagct cgttgccgta ggctttaacg cccaagcgtt tggcttctga 334920
atcgcgaccg ttgcgggtgc tgccgcctgc tttttttactt gccatttgta atgctcctaa 334980
gttttaaggt taggcgattg ccacgatttc gatttgggtg aaattttggc ggtggccttg 335040
gcgtttttgg tagtgtttgc ggcggcgcat tttgaagatg cggacttttt cgccacgacc 335100
gtgtgccact actttagccg ttacttttgc accttcgata aagggtgcgc caacttttac 335160
agattcgccg tcagcaatca tcaaaacttc ggtcagttcg atttggctgt cgagttcggc 335220
tggtatctgt tctactttca atttttcgcc gacggaaact ttatactgtt tgccgccggt 335280
ttttacgacc gcgtacatac tcaactccat aagggttatg gttaatatcc gcacaccatt 335340
gtgcggaact cggcattgta ttgttatttg cctgttttgt caaagtttgc gcggttcgga 335400
taaccatatg ccgtctgaaa agatgtaccc tgatggcttt gctgatataa ttgcccgcta 335460
tttgaatcag ctttcaagcg gtatctgccg tttgacggaa acgtaaacct gagagtctgc 335520
catgctcgag aatctgccct atttccagcg acatctgcct gaagaccttg ccaaagtcaa 335580
tgaagtcatc aaccgtgcgg tgcaatccga tgtcgcactg atttcgcaaa tcggtacata 335640
tatcatcagc gcgggcggca aacgcctgcg tccgattatg acgattttgg cgggtaaggc 335700
ggtcggttat gatgacgaga aactgtattc gctggcggcg atggtcgagt ttatccacac 335760
ttccacccctc ctgcacgacg atgtcgtcga tgaaagcgat ttgcgccgtg ggcgggcaac 335820
ggcaaacaat ctgttcggca atgcggcggc tgtgttggtt ggcgactttt tatacacgcg 335880
cgcctttcaa ctgatggttg cctcgggcag tatgcgcgtt ttggaagtga tggcggatgc 335940
aaccaacatt attgccgagg gcgaagtcat gcagctgatg aacatcggca atacggacat 336000
taccgaagaa caatatatcc aagtcatcca atataaaacg gcaaaattgt ttgaagctgc 336060
cgctcaagtc ggcgcaattt tgggcaaggc ttcccccgaa cacgaacggg cgttgaaaga 336120
ctacggtatg tatgtcggta cggcattcca aattattgac gatgtgctgg actattctgg 336180
cgaaaccgaa gaaaccggca aaaacgtcgg cgacgatttg gcggaaggaa aaccgacttt 336240
gcctttgatt tatctgatgc gtcagggttc cgaacaggtt gcgaacgatg tgcgtactgc 336300
tttggaaaat gcagatcgca gctattttga gaaaatccac gattatgtcg tccgttcgga 336360
tgcgttggca tattcgatag gcgaggcgcg caaagcagtc gattgtgccg ttaccgcctt 336420
ggatgccctg cccgacagcg aagtgaagga tgccatgatt cagctggcga aggaatcttt 336480
ggtcagggtg tcttgaggcg atgaatttca gttttgttcc cctgtttctg gttacgctga 336540
ttctgttggg ggtggtcagc aacaacaatt cgattaccat ctcggcaacc atattgctgc 336600
tgatgcagca gacggcattg atacagtttg tcccgttggt cgagaagcac gggttgaatc 336660
tcggtatcat tcttttgacc atagggggttt tgagtccgtt ggtttcagga aaggcgcagg 336720
ttcctcccgt tgccgaattt ttgaatttta aaatgatatc cgccgttttt atcggtattt 336780
tcgtggcttg gctggcggga cgcggcgtgc cttatgatgg gacagcagcc tgttttaatt 336840
acagggctgt taatcgggac ggttatcggg gtggcattta tgggcggtat ccctgtcggg 336900
ccgctgattg cggccggcat cttgtctttt gtcgtcggaa agggttaaaa tctccttttc 336960
atttcggctc gccatagttc aacggataga acgtatgcct cctaagcgta aaatacaggt 337020
tcgattcctg ttggcgaggt ttgacgattt catttgtctg tttcccgtgt tgcgggaagt 337080
ttccgatata aggcctttca gtgttggagg gctttttttgc catctgaaaa ctttttcttc 337140
ctgcttgaaa aaccgacctt taggacggta gaatcatgaa atgattttca ggcttcgtaa 337200
aagatgttcc ggcttggaaa tctgttgttt tatgatatag tggattaaat ttaaatcagg 337260
acaaggcgac gaagccgcag acagtacaga tagtacggca aggcgaggca acgccgtact 337320
ggtttaaatt taatccacta taaaagctgt acaggtataa caatgaataa atttggggat 337380
aaggtcgtat gagcgtaggt ttgctgagga ttctggttca aaaccaggtg gttactgttg 337440
agcaggccga gcattactac aatgagtcgc aggcgggtaa ggaagtgttg ccgatgctgt 337500
tttcagacgg tgtcatttcg cccaagtcgc ttgcggcatt gattgcgagg gtgttcagtt 337560
attcgattct tgatttgcgt cattatccgc gccacagggt gctgatgggg gtgttgacgg 337620
aggagcagat ggtggagttc cactgtgtgc cggttttccg tcggggcgac aaagtatttt 337680
ttgcggtttc cgatccgaca cagatgccgc aaattcagaa aaccgtttct gccgcaggga 337740
ttgaggttga gttggtcatt gtcgaggatg accagttggc gggtttgctc gattgggtgg 337800
gttcgcgttc gacatcgctg cttcaggagc ttggggaggg gcaggaggaa gaggaaagcc 337860
acaccctgta tatcgacaac gaggaggcag aagacggccc tgttccgagg tttatccata 337920
agactttgtc ggatgccttg cgcagcgggg catcggacat ccatttcgag ttttacgaac 337980
acaatgcccg tatccgtttc cgtgtggacg ggcagctccg cgaggtggtt cagccgccca 338040
ttgcggtaag gggcagcttc gcttcacgga ttaaggtaat gtcgcgtttg gacatttccg 338100
aaaaacggat accgcaggac ggcaggatgc agctgacctt tcaaaagggc ggcaagcctg 338160
tcgatttccg tgtcagcaca ttgccgacgc tgtttggcga aaaggtcgtg atgcggattt 338220

tgaattccga tgccgcgtct ttgaacatcg accagctcgg ttttgagccg tttcagaaaa 338280
aattgttgtt ggaagcgatt caccgtccct acgggatggt gctggtaacc ggtccgacgg 338340
gttcgggtaa gacggtgtcg ctctatacct gtttgaatat tttgaatacg gagtcggtaa 338400
atattgcaac ggcggaagac cctgccgaga ttaacctgcc gggcatcaat caggttaacg 338460
tcaatgataa gcagggcctg acttttgccg ctgctttgaa gtctttcctg cgtcaggacc 338520
cggacatcat tatggtcggt gagattcgtg atttggaaac tgccgatatt gcgattaagg 338580
cggcacaaac agggcatatg gtgttttcca ccctgcacac caataatgcg ccggcgacgt 338640
tgtcgcgtat gctgaatatg ggtgtcgcgc cgtttaatat tgccagttcg gtcagcctga 338700
ttatggcgca gcgtctttta cgcaggctgt gttcgagctg caaacaggaa gtggaacgcc 338760
cgtctgcctc tgctttgaag gaagtcggct tcaccgatga ggaccttgca aaagattgga 338820
aactttaccg cgccgtcggt tgcgaccgtt gccgggggca gggttataag gggcgtgcgg 338880
gcgtgtatga ggttatgccc atcagcgaag aaatgcagcg tgtgattatg aacaacggta 338940
cggaagtgga tattttggac gttgcctata aggagggtat ggtggatttg cgccgggccg 339000
gtattttgaa agttatgcag ggcattactt cattggaaga ggtaacggca aataccaacg 339060
attaggtttg agaatgaaaa tgccgtctga agcgtgtttg tttcagacgg catttgactt 339120
tcagggtgtt tgccgggaag gcggggcggt cagcggtatg ccatgtcggg ttcggatatt 339180
tccggcaaac tttccgtttg gccggaaacc gtatatttcc cgtctgccca tccgcccaag 339240
tcgatcagtt tgcagcgttg cgaacagaag ggcggaatg cgttttcggg tttccatact 339300
actgctgttt gacaggtcgg acatttgact tgaaggcgtg tttgccgcga ttcagtcatt 339360
gtgttttcct tgtgttggtt ttgaggcgaa aatccctgaa taaaacgcgt gcaggcgcat 339420
tgttttctca cgcaggcttt tgaggctgcc gtcattgagc agcacatcgt ctgcaagcag 339480
caggcgttcg gattcggatg cctgatggct gatgacggcc gccacctcgc cgcgcgtcag 339540
cccgctgcgg gccatcaccc tgccgatacg tttttccaca ggggcactta tggtcaggac 339600
acgccgtatc aggctgataa attgacgctt ttccgtcagc agcggaattt cgacaatgcc 339660
gtaagctgca tcagtaaagg tttcttgctg ttttttgatt tctgagaaaa tcagcggcaa 339720
catcacggat tcgagcaagg cttttcgcga tggggaggca aagacttctt tacgcaatat 339780
gtcgcgccgc aacaaaccct gtgtgtcaaa aacggtgtcg ccgaacagcc gcctgatttc 339840
cggcagggcg atgccgtctg aagccgtcag cgagtgcgcc gccgcgtctg catcgatgcg 339900
cggcacgccc aaatcggcaa aacattgcgc ggctgccgat ttgccgctgc cgattccgcc 339960
ggtcagtccg acccataccg tcatcttaca gcaccggatg ggtcagccac cagttgaccg 340020
cccgccatac ggaatcgttt gccgtaaaaa ttatccagcc cgaaactgtc agtgcggggc 340080
cgaaggcaaa atgctgcccc ttggcgacgc gcataacgat tgccgcgacc aaaccgatca 340140
gcgaggaaac aaaaatcagt acgggcaatg cggatatgcc gagccacgcg cccaatgcgg 340200
caatcagttt gaaatctccg ttgcccatac cggtttttcc tgtgagcagt ttatacactg 340260
cacataagag ccataatgaa ccatagccgg cgaccgcacc taaaacggca gactgcaaag 340320
gcacgaagcc gccgtccaaa ttaaatatca gacccagcca aattaagggc agtgtcatcg 340380
agtcgggcag gtattgggtg tccgcatcga taaaggtcag ggaaatcaga aacgcggtca 340440
gtaccaatcc gcccagcgta atccaagacc agccgtattg ccaggcgacc agcccgaaca 340500
atacgccggt cagcagctcg attaagggat aacgtatgct gattttggtt tggcaggaag 340560
cgcatttgcc gcgcaggagc aggtagctga caatcgggat gttctgccac gcgcgtatcg 340620
gcacgcggca tttgggacag caggaatccg gtttcatcag gttgaaggta cggctttcct 340680
cttcggtcag cggcaggttt aaatattctt tggcaaatac cgtccagccg cgttccatca 340740
tgaccggcac gcggtaaatg acgacattta agaaacttcc gaccagcagc ccgaacaccg 340800
ctgccaaagg cacggcaaac ggcgacaata cagacaaatc agacatattt tgttctcaat 340860
gtattcaaaa caaaaacaaa ccggcgcaga gcgaatccgc gccggatctg tgcggcaaat 340920
caggcgacca cgttgcccaa attaaacagc ggcagataca tggcgaccag aagcgtgccg 340980
atgaccaagc ctaaaatcac gataatgatc ggctccatca tagcggacag cctgccgacc 341040
gcattgtcca cctcgtcttc gtaaaattcg gcggctttgt tgagcatatc gtccaaagaa 341100
cccgattcct cgccgatgga agacatctgc aacatcatat tggggaacag ttccgtcgca 341160
cgcatccccg aagtcataga caaaccttgg atgacgcgcg tacggatttc ccgggtggct 341220
tcttcataga ttaaattgcc cgccgcgccg gcagtggagt ccaatacatc gaccaaaggc 341280
acgcctgccg caatcagcgt cgccgtcgtc ctgccccagc gggcaatcgt tcctttgcgg 341340
acaatgtctc cgaaaatcgg catacgcagc agtatggcat ccatacgccg ttggatttta 341400
atcgaacgcg ccttcaattt aaggaagccg tatatggcaa agcccagtgc gatcagcacc 341460
atccagccgt atgagacgaa aaagtcggac atatccatca ctgtttgggt cagtgcggga 341520
agctccgcgc ccatattggc gtaaacttct ttaaaggcgg gcagtacgaa aatcatcatc 341580
acgaatacca aaccgatggc gacggcgatg acggataccg gataggtcag tgcggttttt 341640
acctttttgc ggatggcctg ggttttttct ttgtaaattg ccaatttgtc cagcaggctt 341700
tccaatacgc cgcccgtttc gcccgccgca accagattgc agtagaagcg gtcgaaatat 341760
tttgggtggt ttgagaatgc gcggctcaac gagctgccct gttccacttc gcctcggatt 341820
tccatcagca tttccgtcat agacgggttg ccgtgtccgc gcgccacgat ttcaaatgcc 341880

tgcatcagcg gcaggcccgc tttaatcatc gtggacagct ggcgggtgaa aacggtgatg 341940
tcttcttgtg tgattttgcg cttggagctt gttttcacac gggtaatctg caacgggcgg 342000
atgccgcgtt ttgccagttt tttgcgcgcc tcttcttcgg taaacgcgga tacttcgccg 342060
ttgaccagtt tgtcggaggc ggaatgcctg ccttcaaaga taaagcgttt ttctttcttt 342120
gcgaacaaag aaaatcctcc gtttttagcc atattctagc cccgtaaagt aattggaata 342180
aaatgtaaga aacatcgtta aaaaacagta ccggcgtgtt cccggtaaga tgaaaaccgc 342240
cgacatcccg cctgcgggcg gcaaacggga cagaatcgga tgcgattata ccttatttag 342300
gcggctgtcc ggcatttatg cgtacacaat aaatcttgca ggatattgtt gcgggtcaaa 342360
tgccggccgg agggcatttc cgccatatgg aaataaggtg ctattggacg cggcgggcgg 342420
tgttccggag attcgccaaa gccgctgccg tttgttaaac tacattctgc tacattttaa 342480
tccggttctg aaaaatcaag gaaaacagat gaatgctttt acccgtgcat ggtatgcgct 342540
cgaacgccat tatcaggata cgcgtcatgt ccttttgcgc gaccgctttg cctgcgaacc 342600
ggaccggttt gagcgtatgc acgagcgttt ggacgggatg ttgttcgatt acagcaaaaa 342660
ccgtttgggc gaagatacgc tgcaactgct ctgcaatctt gccgacgcgg cggatttgga 342720
agggaaaatg cgtgctttgc ggacgggtgc gaaagtcaac ggcagcgagg ggcgtgccgc 342780
gctgcatacg gctttgcgcc tgcccgacgg tgcggatgcc gtttatgtgg acggcaggga 342840
cgtgttgccc gaaatccgcc gcgagttaaa tcgtgcgttg aagtttgcac acagtttgga 342900
cgacggttcg tatcagggga taaccggaaa acggattacg gattttgtcc acatcggcat 342960
aggcggatcc gacctcgggc cggcaatgtg cgtgcaggca cttgagccgt tcagacggca 343020
tatcaccgtc cattttgccg ccaacgccga tcctgcctgc ctggatgcgg ttttatgccg 343080
tctgaacccc gaaacgacag tgttttgcgt tgccagcaag tccttcaaaa caccggaaac 343140
cctgctcaat gcacaggcag tcaaggcgtg gtatcgcggt gcagggttct cggaatccga 343200
aacggcgtgc catttttgcg cggtgtctgc cgacactgcg gcagctgcgg cttttggtat 343260
cgcggcggaa cgcgtgtttg cgatgtacga ctgggtgggc ggacgctatt ccgtctggtc 343320
gcccgtcggt ttgcccgtga tggttgcggt cggcggggcg cgtttccgcg agttgttggc 343380
gggggcgcac gcgatggaca ggcatttttt cagtacgccg acgcgtcata atatccccgt 343440
tttaatggca ctgattgccg tgtggtacaa caatttccag cacgcggacg ggcagaccgc 343500
cgttccgtac agccacaacc tgcgcctgct gccggcgtgg ctgaaccagc tcgatatgga 343560
gagtttgggc aaaagccgcg cttcagacgg cagtcccgcc gtgtgcaaaa cgggcggcat 343620
cgtgttcggt ggtgaagggg tcaactgcca gcacgcctat ttccaactgc tccaccaagg 343680
cacgcgcctg attccctgcg attttatcgt cccgatgacg gcgcagggca gagaggacgg 343740
acgcagccgt tttaccgttg ccaacgcctt tgcccaagcg gaagccttga tgaagggcaa 343800
aaccttggac gaagcacgcg ccgaactggc agatttgccc gaagcggaac gcgaacgcct 343860
cgcgccgcac aaagagttcc ccggcaaccg ccccagcaac agcattttga ttgaccgcct 343920
cacgccctac aatttgggta tgctgatggc ggcttacgaa cacaaaacct tcgtccaagg 343980
cgcgatatgg aacgtcaacc ccttcgatca gtggggggtg gaatacggca aacagttggc 344040
aaaaaccatc atcggcgaac tggaaggcgg cacgtccgta cacgatgcct cgaccgaagg 344100
gctgatggcg ttttaccgcg aatgccgtct gaaaggcggc ggcgcggcat aaaagtactg 344160
ccgcctttct gtattgattc gggcgcggaa aaggcaatac ctgccgcctg cccgattccg 344220
aaacgccaat gtttggcaac cgctcgcgta ttgctgacga atatgcgttt gcgtggcaca 344280
atagcgcatt catttcaaat gaacatactg cttgaaaata ccggcaagcg tcccacgaaa 344340
catctcacat aaggaaatat tatgtctttg caaaacatta tcgaaaccgc ctttgaaaac 344400
cgcgcggaca tcaccccgac caccgttact cccgaagtca aagaagccgt gttggaaacc 344460
atccgccaac tcgattccgg caaactgcgc gttgccgaac gtttgggcgt gggtgagtgg 344520
aaagtcaacg aatgggcgaa aaaagccgtg ttgctgtcct tccgcatcca agacaacgaa 344580
gtcctcaacg acggcgtgaa caaatacttc gacaaagtgc cgaccaagtt tgccgactgg 344640
tctgaagacg agttcaaaaa cgcaggcttc cgcgcagttc cgggtgcggt tgcccgacgc 344700
ggcagctttg tggcgaaaaa tgtcgtgctg atgccatctt atgtcaacat cggcgcatac 344760
gtcgacgaag gcgcgatggt cgatacttgg gcaaccgtcg gctcttgcgc gcaaatcggt 344820
aaaaacgtgc acttgagcgg gggcgtcggc atcggtggtg tactcgaacc cctgcaggcc 344880
gcacccacca tcattgaaga caactgcttc atcggtgcgc gttctgaaat cgttgagggc 344940
gtgattgtcg aagaaggcag cgtgatttct atgggcgtgt tcatcggtca atccaccaaa 345000
atctttgacc gtacaaccgg cgaaatctat caaggccgcg taccggcagg ttcggttgtc 345060
gtatccggca gtatgccttc caaagacggc agccacagcc tttactgcgc cgtcatcgtc 345120
aaacgcgtgg acgcgcaaac ccgtgcgaaa accagcgtca acgaattgtt gcgcggcatc 345180
tgatgcctta aaccgtattt gaaacgtcca atgccgtctg aaatccgctt cagacggcat 345240
tgccgtttgc acgctgcaac gtgaaaacac agaaacaggg acaatttgct ataatcaacg 345300
gtttagaacg aaccgaacac tatttgaagg atacaaaatg ggttttctgc aaggcaaaaa 345360
aattctgatt accggcatga tttccgagcg ttccatcgct tacggcatcg ccaaagcctg 345420
ccgcgaacaa ggcgcggaac tggcgtttac ctacgttgtg gacaaactgg aagagcgcgt 345480
ccgcaaaatg gcggcggaat tggattccga acttgtattc cgctgcgatg tcgccagcga 345540

cgacgaaatc aaccaagtgt tcgccgactt gggcaaacat tgggacggct tggacggttt 345600
ggtgcattcc atcggttttg cgccgaaaga agccttgagc ggcgacttcc tcgacagcat 345660
cagccgcgaa gcgttcaaca ccgcacacga aatttccgca tacagcctgc ccgcgttggc 345720
aaaagccgcc cgtccgatga tgcgcggcag aaattccgcc atcgtcgccc tgagctactt 345780
gggcgcggtg cgcgcgattc cgaattacaa cgtgatgggt atggcaaaag ccagccttga 345840
ggcaggcatc cgctttaccg ctgcctgtct gggtaaagag ggcatccgct gcaacggtat 345900
ttccgccggc ccgattaaaa cgcttgccgc ctccggcatc gccgatttcg gcaaactctt 345960
gggacacgtc gccgcccaca acccgctccg ccgcaacgtt accattgaag aagtcggcaa 346020
taccgccgcc ttcctgctgt ccgacctgtc gtccggcatt accggcgaaa tcacttacgt 346080
tgacggcggt tacagcatta atgccttgag caccgaggga taatccgccg ttttcaaatc 346140
cgtgcgccgt ccgtgccgca tatcggtttc gggcggcgtt ttgccgtctg aagcgtattt 346200
ctagggaaat gcccgactta cggcaggcgg gatgggaaat gcggacgctt gttttaaccg 346260
attgcctttg tgccgacttg ctgcaggtgc agcggaaacg gttcggatgc gaaaatgccg 346320
tctgaaacgc caaacgggtt tcagacggca ttttttattt aaagcatcag cacacttcaa 346380
ccagccagcc gtatttgtct tccgccaaac catactggat gtcggtaatc gccttacgga 346440
tggcatagcc gcgttcttgg cttttcactt cgatttcttt gccgccgatg acgaaggaag 346500
taacgggcga gatgacggct gccgtaccgg tcaaaatggc ttccgcaccg ttttccaccg 346560
cagctttgag ttcgtcaacc gtgaaattgc gttcgctgac ggtatagccc aaatctttgg 346620
caaccgtcag tacggaatcg cgggttacgc cgtgcaaaaa ctcgtcggtc agcggtttgg 346680
taatgatttc atcgccgtta atcaggataa agttggacgc gccggtttcc tgcacgtcgc 346740
cgttcgggca gaacaggact tgatttgcgc catattcggc tttcgccttc agcacccagt 346800
gcatggcgga agcgtagttg ccgccgcatt tgacgcggcc catatgcggg gcgcagcgga 346860
tgtgttcggt ttccaccaaa attttgacgg gcgatccgac tttgaaatag tcgccgacgg 346920
gggaagccaa aatatacagc agggcggttt cggaaggaga accggccttg ccgataacgg 346980
gatcggtacc gattaaggtc ggacgcaggt acagggcggc aggcgcatcg ggaatttcat 347040
cggcggcacg tttgaccaat ttgattagcg cgtcaagata agcttcggtt tcggggcgcg 347100
gcaggtgcaa aatgtccgca ctttgccgca tacgcgcgat attggcagtc ggacggaaca 347160
gcacgatttt gccgtctgcc tgacggaagg ctttcagtcc ctcgaaacat tcgctgccgt 347220
agtgcagggc gtgcgcgccc ggtgcgaggg agaggtcttg ggaagattgc cattcggtcg 347280
gctgccattt gccttcgcgg taggcgagga cgggcatttg actgtgaaaa acgctgccga 347340
atacggcggg tacgggtctg ctcatgatgt aaagcctttc ttattctgat atgtttcaat 347400
gaacggtttg aatttgaaga ttgtaaagat acgcctgcaa acagggtttt gacaagtgcg 347460
cggcgggttt ttctgtcgat gcggtgtcca atccgttatt tttcaaatgg aaaggaacgg 347520
tgtatttggt aaaattgtcg gcaatcgcat actccgtatg tcgtccgaac acgctgccgc 347580
atcctatccg aaaccgtgca aatcgtttaa actagcgcaa tcttggttca gagtgcgaag 347640
ctgtctgggc ggcgttttta tttacggagc aaacatgaaa cttatctata ccgtcatcaa 347700
aatcattatc ctgctgctct tcctgctgct tgccgtcatt aatacggatg ccgttacctt 347760
ttcctacctg ccggggcaaa aattcgattt gccgctgatt gtcgtattgt tcggcgcatt 347820
tgtagtcggt attatttttg gaatgtttgc cttgttcgga cggttgttgt cgttacgtgg 347880
cgagaacggc aggttgcgtg ccgaagtaaa gaaaaatgcg cgtttgacgg ggaaggagct 347940
gaccgcacca ccggcgcaaa atgcgcccga atctaccaaa cagccttaag aaagccgata 348000
tggacaacga attgtggatt atcctgctgc cgattatcct tttgcccgtc ttcttcgcga 348060
tgggctggtt tgccgcccgc gtggatatga aaaccgtatt gaagcaggca aaaagcatcc 348120
cttcgggatt ttataaaagc ttggacgctt tggtcgaccg caacagcggg cgcgcggcaa 348180
gggagttggc ggaagtcgtc gacggccggc cgcaatcgta tgatttgaac ctcaccctcg 348240
gcaaacttta ccgccagcgt ggcgaaaacg acaaagccat caacatacac cggacaatgc 348300
tcgattctcc cgatacggtc ggcgaaaagc gcgcgcgcgt cctgtttgaa ttggcgcaaa 348360
actaccaaag tgcggggttg gtcgatcgtg ccgaacagat ttttttgggg ctgcaagacg 348420
gtaaaatggc gcgtgaagcc agacagcacc tgctcaatat ctaccaacag gacagggatt 348480
gggaaaaagc ggttgaaacc gcccggctgc tcagccatga cgatcagacc tatcagtttg 348540
aaatcgccca gttttattgc gaacttgccc aagccgcgct gttcaagtcc aatttcgatg 348600
tcgcgcgttt caatgtcggc aaggcactcg aagccaacaa aaaatgcacc cgcgccaaca 348660
tgattttggg cgacatcgaa caccgacaag gcaatttccc tgccgccgtc gaagcctatg 348720
ccgccatcga gcagcaaaac catgcatact tgagcatggt cggcgagaag ctttacgaag 348780
cctatgccgc gcagggaaaa cctgaagaag gcttgaaccg tctgacagga tatatgcaga 348840
cgtttcccga acttgacctg atcaatgtcg tgtacgagaa atccctgctg cttaagtgcg 348900
agaaagaagc cgcgcaaacc gccgtcgagc ttgtccgccg caagcccgac cttaacggcg 348960
tgtaccgcct gctcggtttg aaactcagcg atatgaatcc ggcttggaaa gccgatgccg 349020
acatgatgcg ttcggttatc ggacggcagc tacagcgcag cgtgatgtac cgttgccgca 349080
actgccactt caaatcccaa gtctttttct ggcactgccc cgcctgcaac aaatggcaga 349140
cgtttacccc gaataaaatc gaagtttaac caccaccgaa aggaacacaa aaaatgcgct 349200

tactccatac tatgctccgc gtgggcaatc tcgaaaatcc ctcgatttct accaaaacgt 349260
tttgggtatg aaactgctcc gccgaaaaga ttatcccgaa ggcagattta cccttgcctt 349320
cgtcggttac ggcgatgaaa ccgacagcac ggttttggaa ctgacgcaca actgggatac 349380
ggaacgatac gacttgggca acgcctacgg acacatcgcg gttgaagtgg acgatgccta 349440
cgaagcctgc gaacgtgtga agcggcaggg cggaaacgtc gtccgcgaag ccggcccgat 349500
gaaacacggc acaaccgtga tagccttcgt cgaagacccc gacggataca aaatcgagtt 349560
cattcaaaag aaaagcggcg acgattcggt tgcctatcaa actgcctgat accgccgccg 349620
ccaatgccgt ctgaagcctt taggggtttc agacggcatt ttgttgccgt cgacctgctg 349680
tttgagcctg tgccggttca aactttatcc gttacaccga taaggcaaaa aagatgccgt 349740
ctgaaacggc atccttgatc tgcgaaaggg cagttgggaa tcaaataccc aattcctgcg 349800
ccaatgcttg ggcacgtttg agtacgtcgc cttccgcttc ttccagcaat ttctgcactg 349860
tctcggcagc ggcatcgcgg tcgccgattt cgagatacat ttcggcaagg tcgtatttcg 349920
cttcggaagg cgcgtcagaa cctacagatt ccgaagggaa actggtatct gcattatttg 349980
ggatattttc ttccgagagg tagatgctcc aatctaccgt ttcctcctcg ccgtctttca 350040
ggaagtcggg caaagcgtct gcctcagagg tgttggaatc aggcgtttcc aaagtgattt 350100
ccgctgcatt ttcctcaacg gccggtgctt cagcaggttg caacagtgcg gacaaatcat 350160
cggcaacggt ttccgctgca ttttcctcaa cggcaggtgc ttcagaaggt tgaagtaatg 350220
cggacaaatc gtctgcggtg gcgttgaaat cgggtgtttc ggcaacggtt tccgttacat 350280
tttcctcaac ggccggtgct tcagcaggtt gcaacagtgc ggacaaatca tcggcaacgg 350340
tttccgctgc attttcctca acggcaggta ctttagaagg ttgaagtaat gcggacaaat 350400
cgtctgcggt ggcgttgaag tcgggtgttt cggcaacggt ttccgttata ttttcctcaa 350460
cggacggtgc ttcggcaggt tgaagcaatg cggacaaatc gtctgcggcg gcgttgaaat 350520
cgggcgtttc aggcgcagtt tccgcgacgg catcggtttc gtacactttc aggaaatcgt 350580
gcaactcttc cggtgtttgg acttcggcaa ctgttttttc caagatggtt tcgggcgagg 350640
aagccttcag gaagcctgcc agtccggagg gtgaggcagg ttttgcggaa gctgtttctt 350700
ctgtgccgat atggttgttt gagggcaggt tgtcggagaa atcggtatcg acggtttccg 350760
gtttgttttc ggcagtttgg gcgacagatt ccggttcggg cgtgtcgatg acgatttcga 350820
cagggttgta cgggttgaag gtctcgggct cgtacacgct gtctgtggat tcgatggcgt 350880
tccaatcggc atccgcgcgt ttttgggttt cttcatcctg cgtaagtgcg ccggataaaa 350940
tgccgttttg cgcggctgcc aggctgtcga aatccaagtc gatgcggttg gaaggcgtat 351000
cggtttcgac atcgaacgtt tgttttgccg ataactcttc ttcagattcc ccatctaagg 351060
caagtgtgtc gtttacatcg tttttcggag cgggttcggg cgttgccgga gtttcgactt 351120
cggcaaaggt gatttctatg ccgtcgtctg ccgcgtcgtc aaggtcaggc tcttcctcag 351180
ggacggattc ttcggtacgg cgcgcgcgtt tggattgggc aaggcgcaaa agcagcagca 351240
gggcgattaa tgccgcgcct ccgccggcaa gcagcaaggt gtacgaaccg ccgaacagac 351300
cgtcaaacag tccgctttcg gtttcttctt cggcagaaac ctgttcgaca ggttcggaaa 351360
cggcgttacc ggtttcgtcg gtcggcgtgt cgatggcaga agcggcggct tcttgggggg 351420
cggattcggc agcggtttcc gatgcggcag tatttgcagc gggtacaggt tcgggtcgaa 351480
cggccggttt ttccgctttt gcttcgggcg cggcaacttt tgcttcaggt ttttcaaccg 351540
gtttctctac cgttgcctgt ttggacggtt cggacggcat ggatgcggtt tcggctttgg 351600
gtttcgccgt ttgcggtttg ggttgttccg ctttgatcct gttcagattc ggaatgtgaa 351660
gcacgctgcc cgcacgcagt ctgccgtgtg cggaaacatt tgggtttgcc ttcagcagcg 351720
catcggcaac ctgttcgagc gtcaggtgtt tcgggcggat ggcggcggca atctgtttga 351780
ccgtttcgcc tttgcggacg gtatgggttt tgccgttgta tgccggtttg acggctgcgt 351840
tcgcgctgtc ttttttatcg gttttgcgga gggctttggc gttttgattt tcttgggact 351900
ctgctgtcgg agcggttttg cggtgtgtct tgccgtctga aagtgcagat ttggttttgg 351960
gcgagtagcc gacaggatcg aggatggcgg tgtattcgcg tacctgtgcg cctgcgccga 352020
tgcggaacac caggacggga tcgcggactg cctgttcgga agaaacggca atgacggctt 352080
tgtcgcccaa cttgtggact ttggcggtca ggcctttttc ggaaacggta acgctgccgc 352140
cgcctagcag ggctttggct tcttcgccgg ttacggtaat gctgccggaa aagggttcgt 352200
caaggttgga ctggatattc agtccgccca gtccagcatg tgcctgaaag gatgcggcaa 352260
ctgcgacgga ggcggcaatc agtttgattt gtctgttgtt tttcaagatg tatcccctgt 352320
gggttggcgg ctgaatacgg tttgaccgcg tacagtctgt aaatttcgtc atcatcggcc 352380
atcggcgggg cagtcggccg gcgggcattt aatatgtgaa tgtaccgacc gccgccacat 352440
tttaaacggc aatcattcgc cgtttttaca aattatgaca tatctccatc ttttttcaaa 352500
aacatctgtg catatttgca tcaatcaaaa caaaatttgt tggttttgca ggtgcaaaaa 352560
cagggttctg cctgtatgat tagcgtttat ttgatttgct ttctcatttg gatatgaaat 352620
tcgtcagcga ccttttgtcc gtcatcctgt ttttcgccac ctataccgtt accaaaaaca 352680
tgattgccgc aacggcggtc gcattggttg ccggtgtggt tcaggcggct tttctgtatt 352740
ggaaatataa aaagctggat acgatgcagt gggtcggatt ggtgctgatt gtggtattcg 352800
gcggcgcaac cattgttttg ggcgacagcc gcttcattat gtggaagccg agcgttttgt 352860

tttggctggg cgcgctgttc ctgtggggca gccacctcgc cggtaaaaac ggcttgaagg 352920
cgagtatcgg cagggagatt cagcttccgg atgccgtatg ggcgaaattg acgtatatgt 352980
gggtcggttt cctgattttt atgggtatcg ccaactggtt tgtgtttacc cggttcgagt 353040
cgcaatgggt caactataaa atgttcggct cgactgcact gatgcttgtt ttctttatta 353100
ttcagggtat ttatctgagt acctgtctga aaaaggagga ttgactgtgg aatattttat 353160
gttgctggca acagacgggg aggatgtgca cgaggcgcgt atggcggcac gtcccgaaca 353220
cctcaaacgg ctggagacgc tgaagtcgga aggccggctg ttgacggcag gcccgaatcc 353280
tttgccggag gactccaacc gcgtttcggg cagtttgatt gtggcgcagt tcgagtcttt 353340
ggatgcggcg caggcttggg cggaagacga tccctatgtt catgcaggcg tgtacagcga 353400
agtgctgatc aagccgttta aagcggtgtt caaataatgc cggccgtcga tttgatccgc 353460
gaacgcctgc agacgctcga tccgctggtg ttggaaatcg gcgatgagag ccatctgcac 353520
aaaggacacg cgggcaatac cggcggcgga cattatgccg ttttggtcgt tagcggccgt 353580
tttgaaggcg taagccgcct gaaccgccag aaaacggtca aatcgctgct caaagatttg 353640
ttttcaggcg gcatgattca cgcgctcggc atccgggcgg ctacccctga cgagtatttc 353700
catacggcgg actgaatgaa gtctgcccga acatttcaat ttaaaattta aagagagaag 353760
attatgaaag caaaaatcct gacttccgtt gcactgcttg cctgttccgg cagcctgttt 353820
gcccaaacgc tggcaaccgt caacggtcag aaaatcgaca gttccgtcat cgatgcgcag 353880
gttgccgcat tccgtgcgga aaacagccgt gccgaagaca cgccgcaact gcgccaatcc 353940
ctgctggaaa acgaagtggt caataccgtg gtcgcacagg aagtgaaacg cctgaaactc 354000
gaccggtcgg cagagtttaa aaatgcgctt gccaaattgc gtgccgaagc gaaaaagtcg 354060
ggcgacgaca agaaaccgtc cttcaaaacc gtttggcagg cggtaaaata tggcttgaac 354120
ggcgaggcat acgcattgca tatcgccaaa acccaaccgg tttccgagca ggaagtaaaa 354180
gccgcatatg acaatatcag cggtttttac aaaggtacgc aggaagtcca gttgggcgaa 354240
atcctgaccg acaaggaaga aaatgcaaaa aaagcggttg ccgacttgaa ggcgaaaaaa 354300
ggtttcgatg ccgtcttgaa acaatattcc ctcaacgacc gtaccaaaca gaccggtgcg 354360
ccggtcggat atgtgccgct gaaagatttg gaacagggtg ttccgccgct ttatcaggca 354420
attaaggact tgaaaaaagg cgaatttacg gcaacgccgc tgaaaaacgg cgatttctac 354480
ggcgtttatt atgtcaacga cagccgcgag gtaaaagtgc cttcttttga tgaaatgaaa 354540
ggacagattg cgggcaacct tcaggcggaa cggattgacc gtgccgtcgg tgcactgttg 354600
ggcaaggcaa acatcaaacc tgcaaaataa ttctgaaaac gggatatggc ggcaagacgt 354660
tcagacaggc gttttgccgc cgcgcaggac agggaatacc atgaaacaga aaaaaaccgc 354720
tgccgcagtt attgctgcaa tgttggcagg ttttgcggca gccaaagcac ccgaaatcga 354780
cccggctttg gtggatacgc tggtggcgca gatcatgcag caggcagacc ggcatgcgga 354840
gcagtcccaa aaaccggacg ggcaggcaat ccgaaacgat gccgtccgcc ggctacaaac 354900
tttggaagtt ttgaaaaaca gggcattgaa ggaaggtttg gataaggata aggatgtcca 354960
aaaccgcttt aaaatcgccg aagcgtcttt ttatgccgag gagtacgtcc gttttctgga 355020
acgttcggaa acggtttccg aagacgagct gcacaagttt tacgaacagc aaatccgcat 355080
gatcaaattg cagcaggtca gcttcgcaac cgaagaggag gcgcgtcagg cgcagcagct 355140
cctgctcaaa gggctgtctt ttgaagggct gatgaagcgt tatccgaacg acgagcaggc 355200
ttttgacggt ttcattatgg cgcagcagct tcccgagccg ctggcttcgc agtttgccgc 355260
gatgaatcgg ggcgacgtta cccgcgatcc ggtcaaattg ggcgaacgct attatctgtt 355320
caaactcagc gaggtcggga aaaaccccga cgcgcagcct ttcgagttgg tcagaaacca 355380
gttggagcag ggtttgagac aggaaaaagc ccgcttgaaa atcgatgccc ttttggaaga 355440
aaacggtgtc aaaccgtaat ggcatttcca ataccgatgc cgtctgaagc ctttcagacg 355500
gcattgcacg ttcaggtaag gaggacggct tatgcgtgcg gtcatacaga aaacggtagg 355560
tgcaaaggtg gatgtcgtgt ccgaagccgg cacggaaacc tgtggcaaaa tcgacggcgg 355620
gtttgtcgtg ttactcggcg taacgcatag cgacacagaa aaagatgcac gctatatcgc 355680
cgacaaaatc gcccatttgc gcgtgtttga agacgaagcg ggcaagctga acctgtcttt 355740
gaaagatgtc ggcggcgcgg tgctgctggt gtcgcagttt acgctttatg ccgacgcggc 355800
aagcgggcgg cggccttcgt tttcccaagc cgcacctgca gaacaggcgc agcagcttta 355860
cctgcgaacg gcggaactgt tgcgcggaca cgggattcat gtcgaaacag ggcgtttccg 355920
cacgcatatg caggtgtcgc tctgcaacga tgggccggta accatactgc tggactcttt 355980
catgacgcgg atttccccaa aaatgaaggt tgttccggat tgaaattgaa tccgcaatga 356040
taaaatatcg acaatgaacg acaatacaca cacccttccc ccgcgccacc tgtccgtcgc 356100
ccccatgctc gactggacgg acaggcacta ccgttacctt gcccgccaga ttacccgaaa 356160
tacttggctg tacagcgaaa tggtcaatgc cggtgcgatt gtttacggcg acaaagaccg 356220
ctttttgatg ttcaacgaag gcgagcagcc cgtcgccctg caactgggcg gcagcgatcc 356280
gtccgatttg gcgaaagccg ccaaagccgc cgaggcatac ggttacaacg aggtcaacct 356340
caactgcggc tgccccagtc cgcgcgtgca gaaaggctcg ttcggcgcgt gtctgatgaa 356400
cgaagtcggg ctggttgccg actgcctcaa cgccatgcag gatgcggtca agattcccgt 356460
taccgtcaaa caccgcatcg gtgtggacag gcagaccgaa taccaaaccg ttgccgattt 356520

cgtcggcacg ctgcgcgaca aaaccgcctg caaaaccttt atcgtccacg cccgcaacgc 356580
ttggctggac ggtctttccc ccaaagaaaa ccgcgacgtt cccccgttga aatacgatta 356640
cgtttaccgc ctcaagcagg agtttcccgg gctggaaatc atcatcaacg gcggcatcac 356700
caccaacgaa gcaatcgcag gacacctgca acacgttgac ggcgtgatgg tcgggcgcga 356760
ggcgtaccac aacccgatgg tgatgcgcga atgggacagg ctgttttacg gcgatacccg 356820
cagcccgatt gaatacgccg atttggtgca gcgtctctac acatacagcc aagcccaaat 356880
ccaagccgga cgcggcacaa tcttgcgtca catcgtccgc cacagccttg ggctgatgca 356940
cggtctgaaa ggcgcgcgga cttggcggcg tatgctttcc gacgcaacgc tcttgaaaga 357000
caacgacggc agcctgattc tcgaagcgtg gaaagaggtc gaacgggcaa atatgcgcga 357060
atagggcggg gctgtatgtg tgaaatgccg tctgaaggct tcagacggca tttgtgcgtt 357120
tgtcgggcgg tgtttagggg gcggtaacgg cgtgtttcgg cactttgtcc atatcccagt 357180
gtgccaccgc ccagtcgagc agttcggcag ggcggtcggt ttccggtgct tcgggcagct 357240
tgaggtaacg gaacacttgg cggaggagtt gttcgcggcg gtttaaatcc aatgcggggg 357300
cgagcgtctg tttcgaccat ttctgccctt gtgcgttggt cagcagcggc aggtgggcat 357360
attgcggtgt cggaacgtcc aaacactgct gcaaatagat ttggcgcggc gtggaaacga 357420
gcaggtcttg tccgcggacg atgtgggtaa cgccctgttc ggcatcgtcg gcaacgacgg 357480
cgagctggta tgcccagtaa ccgtctgcac gaagcaggac gaaatcgccg atgtcgcggg 357540
cgaggttttg ggcgtaaccg ccgacgatgc cgtctgaaaa accgataatg cggtcgggga 357600
cgcggatgcg ccacgccggc tgtttgcctt gcagtgcagg gcgttggccg gggtggcggc 357660
aacgtccgtt atagacgaac ccgtctgcgc cccgccttgc cccggcctgc cagtctttgc 357720
ggctgcaatg gcagggatag accagtccgg cggttttcag gcggcatagg gtttcttcat 357780
acagggcgta acggcggctc tgataggcga cttctccgtc ccactcgaat ccgaatgcct 357840
caagcgtgtg caggatatgg cttgccgccc ccggcatttc gcgcggcgga tcgaggtctt 357900
ccatgcggat cagccatttg ccgccgtgcg cgcgcgcatc ggcataggaa gcgacggcgg 357960
tcagcagcga gccgatgtgg agcagcccgg tcgggctggg ggcaaaacgt cctgtgtaca 358020
tatctggtac agcccccttta tttaagacta ttaatcaaag ccattatctc atctttattc 358080
agttccatcc cgggctcttc aagcaaggtt aaatcatata gggcattata ttgctcttcg 358140
gtagctgaac catccataag agcaggcgag aaaaaatcaa aggctctatc tgcaattctc 358200
tcattacttg catttctact aaccagtttc gtcaattctg tatattttga aaagtttatg 358260
gaaaaataaa acagcgaaaa agttttggtt tcgctgtttt tgatttaatt agcactgata 358320
atcttcaaat tcccacgaaa aaaaacgaag taaataagtc aatgactttt cccaagtttc 358380
ttttgaacat tctttaagaa ttttctcaat ttccgattta ataacagaat gattaaattc 358440
attcataatc atcatacccg ccccccattt aacccttttga ttttggaaac aattatgcaa 358500
aatccattta ggagagcata tgcgaacaga aaatatatct gcagcatcac tatcatcagt 358560
tcctatgtct aaatcaattc ccacacaaaa attgtctttg atttcgggaa cgaaatcttc 358620
aaaggcacaa tcgtaaagat tgatggcttt caattctagg ttaatcattt tatattcaat 358680
agtatgggga ggtaccggat ccttaaaaat cagatctgaa taaatttcat tgggtgaaat 358740
gatttcgatt gcttttgcca tgattctatt tccttttgtg ttagtgggta atgtcgtgca 358800
ttaacttctt gcccattaat atttttaggg tgaatccttg atatgccgca ctgtgtccgg 358860
tcaaacgggc gatgccgtct gaaagccttt cagacggcat cgggaaaatg cctaagccaa 358920
aggcgcgagc agtttttcaa acgcttcttc aaactgtttc aaaccgtctt cctgcaaacg 358980
cgttgccaag gtttcgacat cgatgccgag cgcggcggtt tcggcgagct gcgcttgtgc 359040
ttcttctacg ccttcggtca gcgtggcttt ggctgtgccg tggtcgataa aggctttgag 359100
cgtggcatcg ggaacggtgt tgacggtgtg cgcgccgatc aggctgtcaa cgtagagcgt 359160
gtcgggatag gccgggtttt tcacgccggt agatgcccat aaaagctgca cgcggtttgc 359220
gcctttggtt tccagcgcgg caaattcggg gctgccgaag tattgcgccc agtcttggta 359280
ggcggctttg gcaagggcga tggcgatttt gcctttgagg tggtcgggca gtgttgtgtc 359340
cagcgcgccg tccacacggg agatgaagaa gctggcgaca acttggatat gggcaacgct 359400
ttgtccggct gctaagcgtt tggcgatgcc gcgcgcgtag gcggcgtagg ctttgagggt 359460
ttgggcgcgt gagaacagca gggtcaggtt cacgctgatg ccgtctgaaa cgagggtttc 359520
gagcgcatcg atgcctgcgt cggtggcagg cactttaatc atcgcgtttt tgcacccgat 359580
ggcggcgtag aggcggcgcg cttcttcaac cgtgccttgc gcgtctttgg acaattcggg 359640
cgaaacttcg aggctgacga agccggtttt gccgccggtg gattcgtgtt cggcaaggca 359700
aacgtcgcag gcggcacgca catcggcaac cgccattgtt tcgtagcgtt gtttggggct 359760
gaggttttgc tgcttgaggg cggcgatttc atcggcgtaa agcgcgtcgc cggcgaaggc 359820
tttttggaag atggcgggat tggaagttac gccgcacacg ccctgtttca acatttgcgc 359880
caattcgccg ctttgcacta gcgagcggga aaggttgtcc agccagattt gttgtcctaa 359940
tgctttaacg tccgataaaa tggtcatctc tgattccttt ggatggatag gcggggtttg 360000
agggcttatg ctaccccgat tcggaaattt tgggtagttt tattacagca aaggcggatg 360060
gcaatggcag aaaacggaaa atatctcgac tgggcacgcg aagtgttgca cgccgaagcg 360120
gaaggcttgc gcgaaattgc agcggaattg gacaaaaact tcgtccttgc ggcagacgcg 360180

ttgttgcact gcaagggcag ggtcgttatc acgggcatgg gcaagtcggg acatatcggg 360240
cgcaaaatgg cggcaactat ggcctcgacc ggcacgcctg cgtttttcgt ccaccctgcg 360300
gaagcggcac acggcgattt gggtatgatt gtggacaacg acgtggtcgt cgcgatttcc 360360
aattccggcg aaagcgacga aatcgccgcc atcatccccg cactcaaacg caaagacatc 360420
acgcttgtct gcatcaccgc ccgccccgat tcaaccatgg cgcgccatgc cgacatccac 360480
atcacggcgt cggtttccaa agaagcctgc ccgctggggc ttgccccgac caccagcacc 360540
accgccgtca tggctttggg cgatgcgttg gcggtcgtcc tgctgcgcgc acgcgcgttc 360600
acgcccgacg atttcgcctt gagccatcct gccggcagcc tcggcaaacg cctacttttg 360660
cgcgttgccg acattatgca caaaggcggc ggcctgcctg ccgtccgact cggcacgccc 360720
ttgaaagaag ccatcgtcag catgagtgaa aaagggctgg gcatgttggc ggtaacggac 360780
gggcaaggcc gtctgaaagg cgtattcacc gacggcgatt tgcgccgcct gtttcaagaa 360840
tgcgacaatt ttaccggtct ttcgatagac gaagtcatgc atacgcatcc taaaaccatc 360900
tccgccgaac gtctcgccac cgaagccctg aaagtcatgc aggcaaacca tgtgaacggg 360960
cttctggtta ccgatgcaga tggcgtgctg atcggcgcgc tgaatatgca cgacctgctg 361020
gcggcacgga ttgtatagtg gattaacaaa aaccagtacg gcgttgcctc gccttagctc 361080
aaagagaacg attctctaag gtgctgaagc accaagtgaa tcggttccgt actatctgta 361140
ctgtctgcgg cttcgtcgcc ttgtcctgat ttttgttaat ccactatata aggcgttgca 361200
gccgtttcag acggcatttg tggtaagata tgccgtctga aaacaaggaa atcccatgca 361260
ggcaatttct cccgaattac aggcgcgcgc cgccaaaatc aaactgttga tcctggatgt 361320
ggacggcgtt ttgaccgacg ggcgcatctt tatccgcgat aacggcgaag aaatcaaatc 361380
gtttcacaca ctggacggac acggtctgaa aatgcttcag gcaagcggcg tgcagactgc 361440
gattatcacg ggccgggacg cgccctccgt cggcatccgc gtcaaacagt tgggcataaa 361500
ttactatttc aaaggtatca gcgacaaacg tgccgcctat gaagaattgc gcgcgcaggc 361560
gggcgtggaa gaagccgagt gcgcctttgt cggcgacgac gtggtcgatt tgccggtaat 361620
ggtgcgctgc ggattgccgg ttgccgtccc cggcgcgcat tggtttacgc ggcaacacgc 361680
cgcctatatc acggaacacg cgggcggcgc aggcgcggtg cgcgaagtgt gcgacctgat 361740
tatgcaggcg caagggactt tgggcgcggc tttgaacgag tacatcaaat gaaagtaaga 361800
tggcggtacg gaattgcgtt cccattgata ttggcggttg ccttgggcag cctgtcggca 361860
tggttgggtc gtatcagcga agtcgagatt gaagaagtca ggctcaatcc cgacgaaccg 361920
caatacacaa tggacggctt ggacggcagg cggtttgacg aacagggata cttgaaagaa 361980
catttgagcg cgaagggcgc gaaacagttt ccggaaagca gcgacatcca ttttgattcg 362040
ccgcatctcg tgttcttcca agaaggcagg ttgttgtacg aagtcggcag cgacgaagcc 362100
gtttaccata ccgaaaacaa acaggttctt tttaaaaaca acgttgtgct gaccaaaacc 362160
gccgacggca aacggcaggc gggtaaagtt gaagccgaaa agctgcacgt cgataccgaa 362220

tctcaatatg cccaaaccga tacgcctgtc agtttccaat atggtgcatc gcacggtcag 362280
gcgggcggca tgacttacga ccacaaaaca ggcatgttga acttctcatc taaagtgaaa 362340
gccacgattt atgatacaaa agatatgtaa gctatttgtt ttaatagcat ttttttcggc 362400
gtcccccgct tttgcccttc aaagcgacag caggcagcct attcagattg aggccgacca 362460
aggttcgctc gatcaagcca accaaagcac cacattcagc ggaaacgtcg tcatcagaca 362520
gggtacgctc aatatttccg ccgcccgcgt caatgttaca cgcggcggca aaggcggcga 362580
atccgtgagg gcggaaggtt cgccagtccg cttcagccag acattggacg gcggcaaagg 362640
cacggtgcgc ggacaggcaa acaacgttgc ttattcatct gcaggcagca ccgtagtctt 362700
aaccggtaat gccaaagtac agcgcggcgg cgatgtcgcc gaaggtgcgg tgattacata 362760
caacaccaaa accgaagtct ataccatcag cggcagcaca aaatccggcg caaaatccgc 362820
ttccaaatcc ggcagggtca gcgtcgttat ccagccttcg agtacgcaaa aatccgaata 362880
atcccaaaat gccgtctgaa atataaaccc ggttcggacg gcatatgccg accgaagata 362940
ttgaagagat atttatgagt gcaaacgtca gccgccttgt tgttcaaaac ctgcaaaaaa 363000
gtttcaaaaa acgccaagtc gttaaaagct tctccctcga aatcgaaagc ggcgaagtca 363060
tcggactgct cgggcccaac ggtgcgggta aaaccaccag cttctacatg attgtcggac 363120
tcatcgccgc cgacgcaggc agcgtaaccc tagacggaca agaattgcgc cacctgccca 363180
tacacgaacg cgcccgcctc ggtgtcggct acctgccgca ggaagcctcg atattccgca 363240
aaatgaccgt cgaacaaaac atccgcgcca tcttggaaat cagaaccaaa gataaaaatc 363300
aaatcgacag ggaaatcgaa aaactgctcg ccgacctcaa tatcggacac ttacgccgca 363360
gccccgcgcc gtcgctgtcc ggcggcgaac ggcggcgcgt cgaaatcgcc cgcgtactcg 363420
ccatgaaacc gcattttatt ttgttggacg aaccttttgc cggcgtcgat ccgattgccg 363480
tcatcgacat ccagaaaatc atcggtttcc tcaaatcgcg cggtatcggc gtactgatta 363540
ccgaccacaa cgtacgcgaa accctcagca tctgcgatcg ggcctacatt atttcagacg 363600
gcacggtgtt ggcatcggga aaacctgatg atttggtcgg aaacgaacag gttcgttctg 363660
tttatctggg taagaacttc aaatattgaa aatatttttc agacgggcga cctaatatcg 363720
tcgggcaggc ggcaaaaata cggatttatg ttgtttttac ataaattaat tcaaatttaa 363780

aacattgact taaacctgtt ttcaaagaat attgcccgat atgcttgcat gtcgtcccgt 363840
aatttggttt aatacgcatc tcttaacgag acagacaaag gccagatagc tcagttggta 363900
gagcaacgga ttgaaaatcc gtgtgtcggc ggttcgattc cgcctctggc caccaaaaaa 363960
ccgccttgaa gcggttattt tttttgcctg ccgtttttgg gaagttgtcc gtgtcggaca 364020
cgttttgtgt ctgaccgtta tgtagaaggg caaaaatgat aatgaccgcc ccgttgcgtt 364080
ttggagaaga gggtaaaggc agaaagcata tgccgtctga atgatatttc agacggcatt 364140
ttatattgcg gcggcactca gtccgtgtcg ctttcaggca actctgccga acccatgcgt 364200
ttgagcacga tattggtttt gttgcggagc cgtttgcttt tcggatggtc ggcgtagtag 364260
agcggggcgg ggacgcgcgc cgtcagtttt gccgcctgct gtttggtcag cttggcggcg 364320
ggtatttgat aaaaataccg ggacgcggct tccgcgccga aaacgccgta gtgccattcg 364380
attgagttta aatacagttc aaaaatcctg tctttgtcgg taacggcttc catcatcgcg 364440
gtaatcgccg cttcttcgcc tttgcggata tagctgcggc tttcgtttaa aaacaggttt 364500
ttggcaagct gctggctgat ggtcgagccg cccgccttca ctttgccgct gttccggttg 364560
cgcctgatgg cgttttgaat gccgccccaa tcgaagccgc cgtgcccggc gaaacgggca 364620
tcttcggaag caatcagggc ttttttcagg ttggtggaaa tgcgtttgta gggcatccag 364680
cggtaatcca gtgcgacatc gcgaccttcc tgttcaaact gcttcatccg catcgacata 364740
aaggcagtcc gatggggcgc gacggcgcgg taggtaatga tgttgccgta cacataggca 364800
ttgaaaaaga taaagatgcc gacgggcagg gcaatcagcc atttgatgat gcggaacatg 364860
tttatagggc tttcatgtat tcgataacgg ggcggatatc gggcgtaaat ccgcgccaga 364920
gggcgtagga agccgccgct tgaccgacta gcatacccag tccgtcggca gttttttttcg 364980
cacccgattg tcgtgcaaaa tctaaaaacg gttttgccgc gcagccgtac accatatcgt 365040
aggcaagcgc gcagttttga aaaatatcgg gcggaatatc gggaatctga ccgtttagac 365100
cgcccgacgt gccgttgatg atgatatcaa aaccgccgtt cacgtccgcc atcgggacgg 365160
cttcaatgcc gaaaagctgc gccaattcct cggctttggc gcgggtacgg ttggcaatga 365220
cgatacgggc aggacggtgt tctttcaaaa caggaatcac gccgcgcacc gcgccgcctg 365280
cgcccaaaag caaaatggtt ttgccctcga tggcaatatt tttgacctgc gtgatgtcgt 365340
tggtcaaacc gataccgtcg gtgttgtcgc cacgcagctt gccgtttttc aacggaatca 365400
gcgtattgac cgcacctgcc gccaatgcgc gttcggaatg ctcgtccgcc agatgaaacg 365460
cttcctgttt gaacggtacg gtaacgtttg ccccgcaacc gcctgtttca aaaaatgtcg 365520
aaaccgcctg cgcgaaaccg ccgatgtcgg cgcaaatgcg ttcgtattca atgtcaacgc 365580
cttcctgaag ggcaaattgt tgatgaattt gcggcgattt gctgtgggcg acggggttgc 365640
cgaaaacggc gtagcggggg agggcggtca tggtcgtgtt ccaaaagacg ggaaggctat 365700
tttataacgg cggcgtacag atggaaacga tgccgtctga aaccgccttc agacggcatc 365760
gtttcctgta tcggtcggga aaaatccgga tgcggtgcgc cggcttgtcc gcattgttga 365820
caatcttgcc gtctgaaact atattttccg gcttgaaatt tgacgcaaaa ccggtttcag 365880
acggcatcgg cgtggtaaaa tcgtgccgac tttgcgtcaa gccgccgcgt tccgcatatt 365940
ttgctatttc ccttttccag gagctgaaaa atgtctatta aaaacgccgt aaaattgatt 366000
gaagaaagcg aagcccgctt tgtcgatttg cgctttaccg ataccaaagg caagcagcac 366060
cactttaccg tgcctgcgcg catcgtgttg gaagaccccg aagagtggtt cgaaaacggt 366120
caggcgtttg acggttcgtc tatcggcggc tggaaaggca ttcaggcttc cgatatgcag 366180
ttgcgccccg atgcgtctac agccttcgtc gatccttttt atgatgatgc gactgttgtg 366240
ttgacttgcg acgttatcga tcccgccgac ggtcagggtt acgaccgcga cccgcgctcc 366300
atcgcccgcc gagccgaagc ctatttgaaa tcttccggca tcggcgagac cgcctatttc 366360
ggtcccgaac ccgagttttt cgtattcgac ggcatagaat ttgaaaccga tatgcacaaa 366420
acccgttacg aaatcacgtc cgaaagcggc gcgtgggcaa gcggtctgca tatggacggt 366480
caaaacaccg gccaccgccc gaccgtcaaa ggcggttacg cacctgttgc accgattgac 366540
tgcggtcagg atttgcgttc ggcgatggta aacattttgg aagaactcgg tattgaagtg 366600
gaagtgcacc acagcgaagt cggcaccggc agccaaatgg aaatcggcac gcgctttgct 366660
actttggtca aacgcgccga ccaaacccaa gacatgaaat atgtgattca aaacgttgcc 366720
cacaacttcg gcaaaaccgc cactttcatg cccaaaccca ttatgggcga caacggcagc 366780
ggtatgcacg ttcaccaatc catttggaaa gacggtcaaa acctgttcgc aggcgacggc 366840
tatgccggct tgagcgacac cgcgctctac tacatcggcg gcatcatcaa acacgccaaa 366900
gccttgaacg cgattaccaa tccgtccacc aactcctaca aacgcctcgt gccgcacttt 366960
gaagcgccga ccaaactggc atactccgcc aaaaaccgtt ccgcttccat ccgcattccg 367020
tccgtgaaca gcagcaaggc gcgccgcatc gaagcgcgtt tccccgatcc gaccgccaac 367080
ccgtatttgg catttgccgc cctgttgatg gcgggtttgg acggcattca aaacaaaatc 367140
catccgggcg accctgccga taaaaacctg tacgatctgc cgccggaaga agatgcattg 367200
gtgccgaccg tttgcgcttc tttggaagaa gcactggccg ccctcaaagc cgaccacgaa 367260
ttcctcctgc gcggcggcgt gttcagcaaa gactggatcg acagctacat cgctttcaaa 367320
gaagaagacg tacgccgcat ccgcatggcg ccgcacccgc tggaatttga aatgtattac 367380
agcctgtaag cacgtctggt tttcagaaaa gcaatgccgt ctgaacacag tttcagacgg 367440

cattttgcat ttgaacggca aaccggcggc gcggggcggc atttttcagc aggcgggcga 367500
tatttgctac aataggcttt tgtttttttt gggctgcacg aacgatgact gcatcgaaat 367560
caggttttat cgggcaaatc ttttcccgca atatgcttgt ctgtattttt acggggttta 367620
cctcggggct gccgctgtac tttctgatta acctgattcc ggcgtggttg cgcagcgagc 367680
aggtggattt gaagagcatc gggctgatgg cgttaatcgg tctgccgttt acttggaaat 367740
ttttgtggtc gccgctgatg gacgcggtca ggctgcccgt tttgggacgg cggcgcgggt 367800
ggatgctgct gacgcaggca gggttgctgg cggctttggc ggtctatgcc tttttaaacc 367860
cccgtaatca tctgccgctg attgccggct tgtcggtgct tgtcgctttt ttttccgcca 367920
gtcaggatat tgtattggat gcgttcaggc gcgagatttt gtcagacgaa gaattgggtt 367980
tgggcaactc ggttcatgtg aacgcctacc ggattgccgc cctgattccc ggttcattga 368040
gtttggtgtt ggcagacagg atgccgtggt tagaagtatt tgttatcact tcattattta 368100
tgctgcccgg ccttctgatg acgctgtttc ttgcgcgcga acccgtgttg cctcctgccg 368160
ttcctaaaac gttgaagcag accgtggtag agccgtttaa agaatttttt acgcgcaagg 368220
gcatcgcttc ggcggtgtgc gtgctgctgt ttatcttcct ttacaaactc ggcgacagta 368280
tggcaaccgc gttggcaacg ccgttttatc tggatatggg tttcagcaag accgacatcg 368340
gtttgattgc gaaaaatgca ggactgtggc cggcagtggc ggcaggtatc ttgggcggtg 368400
tgtggatgct gaaaatcggc gtaaacaaag ccttgtggct attcggcgcg gtgcaggctg 368460
taaccgtttt ggggtttgta tggctggcag ggttcggacc tttcgacacg gtcggcacag 368520
gcgagaggct gatgctggcg gcagttatcg gcgcggaagc ggtcggcgtg gggttgggga 368580
cggcggcgtt cgtatcgtat atggcgcgtg aaaccaatcc cgcatttacc gcaacgcagc 368640
ttgcgctgtt taccagcctg tccgccgtcc cgcgcacggt catcaattcc tttgccggtt 368700
atctgattga atggctcggt tatgtaccgt ttttccaact gtgtttcgca ctcgccctac 368760
cgggtatgct gctgctgctg aaagttgcgc cttggaacgg ggagaaaact caggatgcag 368820
gcagatgaac gcgtcaaact ggagcgttta cctgatattg tgtgaaaaca gcgcgttcta 368880
ttgcggcatc agcccgaatc cgcaacagcg gcttgccgcc cacacaaccg gtaaaggcgc 368940
gaaatatacc cgcgtattca aaccggtggc gatgcgtatc gttgcaggcg ggatggataa 369000
aggaacggca ctcaggcagg aaatcgccgt caaaaaactg accgccgcac aaaaacggca 369060
attgtgggag caggcagaaa aaatgccgtc tgaaacctga cggttcaggt tcggacggca 369120
gttggcagca atcagggaaa agcggggcag gcggtaagga aaaccgacgt ttcaacacac 369180
aggacggtac ataaagcgtc gccctatgaa agtgaaggca tatatcagta ttttttatac 369240
gccaacagaa aagaatacga tgaactgttt gttggatttg tattgattaa tcagtatatt 369300
ttttatgccg ggtatttttt ccttatcggt atcccttctt ttatgaggat gcctgccgct 369360
catataaaga acgggaaaat acgatgggaa aatacggtac agccctcgac atcgcacaat 369420
atgtcaactt atagtggatt aacaaaaatc aggacaaggc gacgaagccg cagacagtac 369480
agatagtacg gcaaggcgag acaacgccgt actggttttt gttaatccac tatatttgtt 369540
tgttttatat tgtaagtata cgtataggct ttgtaaaggt aaattgtgaa aaaagcagtt 369600
ttttaaacga atgaaacggc ttcgggctga aatatatgct gatgccctgt ccttcccgta 369660
tatcttgtgt gttgtcaaag tgcaggctgc tttgaaatcg gtattgccat ctatgaacca 369720
ccactttgtt ttatttcagc gggcttgaga tgtgtataag aatattgttt tgaataaatt 369780
taaaaaaatg ataatcgtta ttgaagattt ttaaaggaaa gcgtagagtg ccaattctat 369840
gaagcaatac ggtaagtaac aatgaaaata tctactgctt gggtatagag catatttcac 369900
aacccgtaac tattcttgcg gaaacagaga aaaaagtttc tcttctatct tggataaata 369960
tatttaccct cagtttagtt aagtattgga atttatacct aagtagcaaa agttagtaaa 370020
ttatttttaa ctaaagagtt agtatctacc atgaatatat tctttaacta atttctaagc 370080
ttgaaattat gagaccatat gctactacca tttatcaact ttttattttg tttattggga 370140
gtgtttttac tatgacctca tgtgaacctg ttaatgaaca aaccagtttc aacaatcccg 370200
agccaatgac aggatttgaa catacggtta catttgattt tcagggcacc aaaatggtta 370260
tcccctatgg ctatcttgca cggtatacgc aaaacaatgc cacaaaatgg ctttccgaca 370320
cgccagggca ggatgcttac tccattaatt tgatagagat tagcgtctat tacaaaaaaa 370380
ccgaccaagg ctgggtgctc gaaccataca accagcagaa caaagcacac tttattcaat 370440
ttctacgcga tggtttggat agcgtggacg atattgttat ccgaaaagat gcgtgtagtt 370500
taagcacgac tatgggagaa agattgctta cttacggggt taaaaaaatg ccatctgcct 370560
atcctgaata tgaagcttat gaagataaaa gacatattcc tgaaaatcca tattttcatg 370620
aattttacta tattaaaaaa ggagaaaatc cggcgattat tactcatcgg aataatcgaa 370680
taaaccaaac tgaagaagat agttatagca ctagcgtagg ttcctgtatt aacggtttca 370740
cggtacggta ttacccgttt attcgggaaa agcagcagct cacacagcag gagttggtag 370800
gttatcacca acaagtagag caattggtac agagttttgt aaacaattca agtaaaaaat 370860
aatttaaagg atcttattat gaatgagggt gaagttgttt taacaccaga acaaatccaa 370920
accttgcgtg gttatgcttc ccgtggcgat acctatggcg gttggcgtta tttggctaat 370980
ttgggtgacc gttatgcgga tgatgctgct gcaattgtcg gtaaggatgc aaacttaaat 371040
ggtttgaatt tatggatgaa aaaaggggtg gaaaacctat gggatgatac ggtcggtaaa 371100

aagacccgtt taatgtgtat ttccgttttt tggattgtgg ttttcaattt gtagcgaatc 371160
ggattcggca tatacggcat tgcaaaaagc gtttgactct ccaatgccgt ctgaaaaccg 371220
gtttcagacg gcatttgcgt tcagtgagaa aggtcgcgcc tgccgcccga acgtctcgcc 371280
gcagcctctg cataacggcg cacctctttt tccaaatttt ccaagttcaa aggaaaatca 371340
ggcagtctgt ctccctgttt ctcttcgcgg acaatccgcc cgccatccaa ataccacgtc 371400
tgttgcgcat gataggtctg catatccgcc gttacgccat ccgctttcaa tgctaccgtc 371460
gaagattgtg caataaaaag atttccgttt ttcaaataat attcgaaact ctggcgtttt 371520
tttccattgt cgaaactcca atagactttt tgcggcagac cgtccgcatc atagccgacc 371580
acaagactgt tcgccttcat ccctcggggc atcaattccc gcatattctg ataaaacaca 371640
gaattgcgcg agtccgacgc aattcggttg ctctctttgc ggaagtccca aaccttctgc 371700
tcgtcattcg cgacatcccg gtatttcgcc aaatatacct gggccatctg ataacacccg 371760
aggcaatgct cataaacatc ttccccgatt ttcccgcgcc ccgccgcatc aaataccgaa 371820
ccgtctggtt gccaaacaac ccgatattct cctgtcgttt cataattttc cccgtgaacc 371880
gttccgccgt acacatttac agaaaacgga cgatcgttcc gatacagata ttcggcatta 371940
acaaatgctt ccggcgagcg ttgcgaaagc gaaaccgcaa ccaaaccgcc ctcgccgata 372000
tggtaatcca gccaaacctc tttcccatgt tcctgctccg ttacgtgaaa ccatttcgcc 372060
ttttctttca aacgactgag ccggatagcg agcgcgagat aatccttctc cgactgcaac 372120
ggaccgtcat ccacagttcc ggcaagattt tcctccgtcc ttatcgattc cttcacgatg 372180
acaaccgccc tgtcggcatt tcggaacagg cgggcaagtt tcgccacaaa agcattcgga 372240
tttttaggta cttcagttgc cgtatcgctc aaaaaccaac gcggattaat ctcataggca 372300
atacccgttc ccagccaaaa ggcaaataca agtgcaaaaa atgacaacag taccggtttg 372360
aattttttaa acatatttat ttttcgttta acagaatata tcgattatat cagacgagct 372420
ttgattgccg ggttttgcta ttttttgttg taataatcaa attgcacgtt gactatgtct 372480
ttctcggtaa aaatataacg gagcattgtt ttaagccttt cataacgttc attaattcct 372540
acgctatcag gtagccaagg ggaagcttta atttcaaaaa gtttccaatt tggaaccatt 372600
aagaaatcaa taatggtacc gattccaatg acaacatatc ttggtatgtc catcggataa 372660
ggatattttt ttctaacctc gattaaatca ttctccaact tccaatattc ttcatcatcc 372720
cacaccccgt catcatacca tttgccaata aatgaatttt cgtcataccc ctcaaaacaa 372780
gtaatatttc ttctgaagtt ttttaactca cacataatac acataataat taatctccaa 372840
tacgatttag gtttttatca aatgtaccgt ttcttgtttc ttttctgtaa tgttattcat 372900
cgtagtaagg ttctgttgaa taattgtctt tgcccccggc aatgatagta acaattttcc 372960
cttttgcttc ccaagcttgt actcctattt catcaaactc atagacatat gtcggataag 373020
attcatttga taaataatat ttatcaacac cgtatgattt agggtaatgg aaaagctgtt 373080
taaaatcttc aaaattcaga cctattatat taacgcccat aaaatatagc tcctgataac 373140
aaaatatcga aataatttttg tttttttttt tgacggaaat gagtaaattt gagtcgggag 373200
attcataata ttctgttcta aactcatcag gaggttcata cataaaagtt tccagtatgt 373260
ttttgtactg tgttatatcc gcaccaaaac ggaatattcc tacagaagta aaaggtaaaa 373320
attcgggagt tttaacgacc gcgtcgacca tgctcttctc cttttgtttt tcgattggca 373380
tttttggcaa tatttctgat tttttgctta atctttaagc gttcattttt ggacattccg 373440
ggaataattt tatttgttaa ttcagcaatt tttgattccg ctgatatttg acttcgaccg 373500
ccatctccat gtttttcatt cttggagctt cctgttcttt taggcggaca agaattatga 373560
acccaaaccc cttccgtttc cgcctgatta cccttgacga agtaagtatg ccaatcggca 373620
acggtcagat tgtaggcttt gagcggtttt ggtttgacaa cggttttgcg gacggtttgg 373680
gttctgccgc tttcggataa cagcctgctt cccgctttca aatcttccgc tttaatccat 373740
ttgccgtccg aataaaacgg atggatgcgg ttggaaatca ggatttggct gttgccgatg 373800
ccgtctgaaa gccggatatc gcttcagacg gcattttgat tgccgggttt tgctattttt 373860
tgttgtaata atcaaatcgc acgttgacta tgtctttctc ggtaaaaata taacggagca 373920
tcgttgtgaa tctttcataa cgttcatgaa ttcccacact atcaggcaac caaggggaag 373980
ctttaatttc aaaaagtttc caatttggaa ccattaagaa atcaataatg gtaccgattc 374040
caatgacaac atatcttggt atgtccatcg gataaggata tttttttcta acctcgatta 374100
aatcattctc caacttccaa tattcttcat catcccacac cccgtcatca taccatttgc 374160
caataaatga attttcgtca tacccctcaa aataaggaac gtttcttata atatccttga 374220
actcacacat aataatgtat ctccaatata attaaacttt tcgtctcaat ctacctttac 374280
tatgttgtat tggaaagtaa aaaaatttcc agtcctctac atctagatca gtaaaaatat 374340
aacggagcat taccctgaac ctttcataac gctcattaat tttgacactt ttaggcaacc 374400
aagtagaagc tttaatttca aaaagtttcc aattttgaac cattaaaaaa tcaataatgg 374460
taccgattcc aatcacgatg tcccttggta tatccatcgg ataaggatat ttttttctaa 374520
cctcaattaa atcattctcc aatttccaat attcttcatc atcccacacc ccgtcatcat 374580
accatttgcc aataaatgaa ttttcgtcat actccttaaa acaaggatg tttcttctaa 374640
aatccttgaa ctcgcacata ataattaatc tccaatacga tttaggtttt tatcaaatgt 374700
accgtttctt gtttcttttc tgttcagttt ttcgggtgaa gatgcctctt tccaagcacc 374760

tccattatgt gaatctacat cgcgtgatat ataactcttt cctttttaa aaatagcagc 374820
atcatttctc gttctttctt ttatttttct atatcccaat tcctttgctg ctgcatatgc 374880
ttctgaatca ttcccatata tgggggtaga tggtgttttt cttggcggac aatcattatg 374940
aacccaaacc ccttccgttt ccgcccgatt gcccttgacg aagtaagtat gccagtcggc 375000
aacggtcaga ttgtaggctt tgagcggctg ctgtttgagg gtaatgtttt gaaccgtctg 375060
ttttgcaccg ctttcggaaa gcagggtgtc gccttttttc agacgacctg cctgtatcca 375120
ttttccttga ctgtaaaacg ggtggatttt attggaaatc agggtttggt tgttgccgat 375180
gccgtctgaa atttcaatgt aaacggtttc ttgatacgga ttgccgtatc gggcggtaac 375240
gggtttgtat cccgtttttc cgcttgcctc gtccttggcg aagacgcggt cgccggttcg 375300
gatacgggca atggctttgt agccgtctgc cgttttgacc aaggtgctgc cgtggaagga 375360
gcaggtgtag gatttttaaa gaacttagtt ctcaatatcc tgtttcattc atcaaaatgc 375420
cgtctgaaag ctgaataccg cttcagacgg cattttggtg gttgggtttt taagccaacc 375480
tacgcttact gaaaaccaaa ttgagtttca gacagttttt aggtttgggt gtccaatcta 375540
acttatattt gtccatttaa ttagtcgttt gtatcaaatt tccattattt attttccaat 375600
ttactttata attatcttca taataatcta attcaaaaaa acctgatatt tcaatatcca 375660
attccattat tgttttaata catttttcaa aataaataat gaaataagat tttacgcatg 375720
caccaaaaaa aatatagctg ctccaattaa aactatttgt cgggaaaacc cacccgcttt 375780
tatatatttt tgcagattct ttctcttcga tattaaaggg acaattattc caaaaattat 375840
taatgttttc ttggatgatt tttatatcat cgtcagagca ttcaatccat ccttttatgg 375900
aaacatatga tgccatgttt aatctcctaa acctgtttta acaatgccgc cttttgattc 375960
aatatatgac ttaacttgtg aatgaacacc gtatttaaac caaaattctg cacgttttcc 376020
ctgttggttt gctgcttcga tggttgcttt aatttgcttt ctattttttt gatttaagaa 376080
atttttaggt ttatctattg ctgaaattgt tcttttggct tgtattaaag catcattcgt 376140
aacagcgtca atttctctgc cgttaataaa ttttgatgaa ccatcagttt ttcttctaat 376200
taaatcttca taatgtatat ctagagcttc tctatacttt gcattttgat ataactgtct 376260
cgcactatca gacaaagcca atttcttttt ataagaatca gcaaaatccc cgctaaccgc 376320
agccttccct ggttttgccg cctttgccaa cttcgcgact ttggctgctg cggcaacgtt 376380
gaagacggct tcgacggttt cggcggcatt gggattttcc tgtatccacc ggtcaacggc 376440
ttcgcgcgta ttcttttcaa agcccgccac gctgcccaag ccgccgatga cggcgaattt 376500
gccctcggcg ggcaaggggg cgatgttgcg cattgcggct ttgtctatgg catagcgcgt 376560
tccgtacagt atgtcgccta tgcccaaggc ttcgcccgcg ctgataaagg ggttgagcgc 376620
gccggcggcg acgccgttga taaactccat gctgttgccc cagcggtcga gcttggcatt 376680
gtgctcgaac atttttctgt tggcttcatc ggcgcggtcg gagaaattgc tgccgaggtt 376740
gctgtaattg tcggatatgc gttgccggat gctgcgggtg tcggtcggat tgagtttgat 376800
actgcgggct gtgccgttga cgtgataggt gtattcgtct cgtgcgcccg taggtttggg 376860
gtaattgccg cccttcgggc cgtcgtaggc atcggcggga tgatgttcgt gtccttccca 376920
gttgagccgg tatacggtaa agccttcgtc aacgttgcct ttttcttcgc tcgcgctgtc 376980
ggcggcgtgg ttgtcgaagg gggcgtgttc ttcgtgtccg tgtccggaaa agcgggtgtg 377040
gtagccgatt gtgccgttga tgtttgcctg ttggatgagc aggttgccca tctggtgggt 377100
atagtcttgg atgacgttga ttttgccggt gcggtcggaa acgctgccgc gcgggtcgcc 377160
gaagaggtgg tatttgccgc cgggttcgta gtgctgccgt tgggcgttat cggtaatgaa 377220
cgggtcttgc gccaagtccg ccgcgagggc gggctgtatg agtgcggccg ccgctacggc 377280
gcaggcggca aggaggtttg tcagtctgcg cagcggtttc acggtttatc ctcctttgcg 377340
gcggcggatg acttcgttgc cgacatcggg ttttttaccg ttgttttgtt tgaagtcggg 377400
acggttttgg gcggttgtgt cgccgtaggg ggtaatgtcg gagaaatcga ccatcaggcg 377460
gtctgaggct ttgacggttt tgctgacttt gtaagggccg gtccaaaggg cgtattgttc 377520
ttggtattgg gattcgtagg cggcggtttt aggggtaatc agcagtttcc ggctgtcgcg 377580
gtcaacggcg aaatattcga gcttggtttg ggctttaagg gtttcggcgt tgtagaggtg 377640
cagttcggta cggctgcgga cggtgccgaa tacgtcgacg gttacgaata cgtcggtgtc 377700
ggcgtattcg ggcggtacga cttcgatgcc gcgcaggtag aagacggttt ggatgaggtt 377760
ggtcaggaag gaaacgtcgc gggggttggc gagcagggtt tcgttgcggt agtcgcccgt 377820
gccgttgacg gacagtccgg cggagcgttc gcctttgcgt ccgctgtttt tcgtcagggc 377880
ggcggcgggg gcgttcaaaa gcgatgtgga agtggttacg ctggagagcg cgtcggattt 377940
ggtggtggcg gtagtgtcgt aggcggggta gctgtattgg gtggcacttt cggggttgtt 378000
gtggtagccg ccgcgtatca gtgcgtcgat agagtagcgt ccgccgctta tgttgcccga 378060
accttggtcg cccataacgg agacgtaaag ggcggctttg cgtcctttta gggcggacaa 378120
atccatttct ttgacggcgg cgcgggacga tgcggcgacg agttcttgtt cgacggcaaa 378180
gcgtttgccg ccgccgtggg cgggtatgcc ggtcagtgtg ccgcaggctg tgaggacgag 378240
ggggatgagg aggagcaggg ttttcatagc ggggtttgtt tgatgttgaa cggattttga 378300
gtgtaaaggg attttaaggg tttgtaaaca aaagggcgga aaatgccgtc tgagcggcgg 378360
aaatggcttt cagacggcat ttgcgctcaa taataatatc ccgcgcccag aatacacggt 378420

ttggatgcgc cggttgcttt gtgcggacta ccgggaatgc gattaatcca acacgccgcc 378480
aaccacgcaa atgcggcggc ttccacccat tgcggatcga ggttcaggtc ggcggtgctg 378540
tgcagggaaa cgcgtgtgcc gaaacattct gccaaatccg ccattaaaac aggattgcgg 378600
atgccgccgc cgcaaatgta catttgacgg gcatctgccg ctgcgtgtga gacggcgtcg 378660
caaacggttt gcgcggtaaa acgggaaagc gtccgcaata cgtcgtatcg gttttcgccg 378720
ccgtcaaggt aggtttcgag ccaatttagg gcaaacagtt cgcgccccgt gcttttaggg 378780
tggggttgtg cgaaatacgg gtgggcgagc agcctgtcga gcagttgcgg caatatgttg 378840
ccttgtgccg cctttgcacc gtttttgtcg taaggaagct gccagtgtgc ctgcgtccac 378900
gcgtccatca gcatattgcc cggccctgtg tcgaagccga aggcgggtgc gtcggggggg 378960
agtacgctga tgttggcaat cccgccgatg ttcagtaccg cgcgtgtttc cctgttgtcg 379020
cggaacaggg cttcgtgaaa ggcggggacg agtggcgcgc cttgtccgcc ggccgcaagg 379080
tcgcggctgc ggaagtcgcc gacggtaaaa atccgcgtcc gttccgccag cagcggcaaa 379140
tcggcaagct gtatgctgta accgtgttcc ggcgcgtgtc ggacggtttg cccgtggcag 379200
ccgagggcgg taatgtcgga cggtgcgagg ttttgactgc acagcagttc ggcggcggtt 379260
tgcgcatata ggcggctgag ttcttgcgac aaaatcctgc tgcggtgcag ttcgtctgcg 379320
cctgtgtcct gcaaatccag caattggcgg cgtaacctgc cggggtaggg ggtaaaggcg 379380
tgcccttccg cgcccagcca tttgccgccg tccatccgta tcagtacggc atccgccccg 379440
tccatgctgg ttcccgacat gatgccgatg taaagctgtg tttccatcat cactcccaaa 379500
ctggtgcaaa acgccatttt aacgtgtatt gacgctcgta taccgatttg ccgccgcagt 379560
gtaaataaag tgtaaataaa tgtttcaaga cggatggaaa aatattataa tgcgcccgca 379620
acatccagta gtagaagtgt catacaaacc gtttccggca gcagttttgc attcggtcag 379680
gtttgggggt attcggatgc ggttaggaag gatgcgtctg ccatatcccg aaacggcagt 379740
tcgaccggag gcagcagtac agtgtcggca acactcatga tttccaccac attaaaggaa 379800
gattgccatg gctcaaatcc aaatgagcgc aaatgttaaa accatcaacg ccgtctttgc 379860
cgccatgctg gtaggtacag tcggctattt tatttattgg ggcttgggtt atacccatta 379920
caattacgcc gccttattca ttattgccac gatgttcggc gtgtttatgg cgttcaacat 379980
cggcggcaac gatgttgcca attctttcgg caccagcgtc ggtgcgggta cgctgaccat 380040
cccgcaggct ttgctgattg cggcggtatt tgaggtcagc ggcgcggtca tcgcgggcgg 380100
cgaggtaacc aataccatac gcaaaggcat cgtcgatttg aagggtgttg atttcgaacc 380160
catacagttt gtgtttatta tgatgtccgc gcttttggcg gcggcgttgt ggctgttgtt 380220
tgcctcgaaa aaagggcttc cggtatctac cacccattcc attatcggcg gcattgtcgg 380280
cagcgcggta tgtatggcgg taatgaacga tgccgcatcg ggcgatttga tacgttgggg 380340
caagctgggc ggtattggtg tttcttgggt attgtcgccc gtgttgggcg gcgcggtgtc 380400
ctattttctg ttttcgcgcg tcaagaaaaa cgtcttagat tacaacgctt gggcggaagg 380460
cacgctcaag ggcatcaagc aggaaaaaaa ggcctataaa gaacggcacc gcctgttttt 380520
cgagggtttg tccgaagccg aaaaagtcga gtacgccacc aaaatggcgc acgacgcgca 380580
aatttacgac gaacccgaat tcgatccgca agagctgcaa tcggagtatt accgcggtct 380640
ttatgcgttc gacaaccgta aaaacaatgt cgattcctac aaggcactgc attcttggat 380700
tccctttatc gcttcgttcg gcgcgatgat gatttccgct atgctgattt tcaagggctt 380760
gaaaaacctg catttgggga tgagcaacgt caacagcttc ctgaccatct ttatgatagg 380820
cgcggcggtg tggatgggga cgtttgtttt tgccaaaagc ctcaagcgta aagacttggg 380880
caaatcgacc tttcagatgt tttcatggat gcaggtcttt accgcctgcg gcttcgcatt 380940
cagccacggt gcgaacgata tcgccaacgc catcggtccg tttgccgcga ttatggatgt 381000
tttgcgtacc aacagcgttg ccgcgcaaaa tgtcgtcccc ccgattgcga tgctgacttt 381060
cggcatcgcg ctgattgtcg gtttgtggtt tgtcggtaaa gaggtgatta aaaccgtcgg 381120
tacgagtttg gcggaaatgc atcctgcttc gggttttacc gccgaactgt ccgccgcctc 381180
cgtcgtgatg ggcgcgtcgc tgatggggct gcccgtgtcc agtacgcata tcttggtcgg 381240
cgcggtactc ggtatcggtc tggtcaaccg caatgccaac tggaaactga tgaagcccat 381300
cggtttggcg tgggtcatta ccctgcctgc cgccgccgta ttgtcggttg tctgctactt 381360
ggttttacag gcagtattct gattgtaaaa tactgatgcc gtctgaaccc gtgttcagac 381420
ggcattttgt tgatggaatg tgcgggcttg tgccttatgc acaatctgtt ctgtcgggat 381480
atgccgtttg gtatagtgat taacaaaaat caggacaagg cgacgaagcc gcagacagta 381540
cagctagtac ggcaaggcga ggcaacgctg tactggtttt tgttaatcca ctatatcttg 381600
gtttcggaac ggtcggacac aaaggtgcgg aacgttatga tatgccgccg cctgttcttg 381660
aaaacactta tcctgccggc agcaaaatgc cgtctgaaaa agcctttcag acggcatttg 381720
tacgttagcc acaatcacac tgtttgcgaa tatttcgcct tggtttcttt atggcgcagg 381780
tggtaatcga agaccatggc gatgttgcgg atgaggaagc gtcctttcgg ggtaacggtc 381840
agcccgtggc tgttcaggcg caccaatccc aaaccggcga gtttttccaa atccgccagt 381900
tcgtctttga agtagcggtc gaacgggatg ccgaacatac tttcgtaaat ccgatagtcg 381960
agcgcgaaac ggcacatcaa atcctgaatg atgttgcggc gcaggatgtc gtcctgattg 382020
agctggtagc cgcgcatgat gggcagtctg ccttcgtcga tggcggcata gtaggcatcg 382080

atgtcgcgtt cgttttggga ataggtgctg ccgattttgc cgatggacga cacgccgatg 382140
gcgaccaaat cgcaatccgc gtaggtcgaa tagccttgga agttgcgctg gaggaagcct 382200
tctttgaggg cgatggagag ttcgtcgtca ggtttggcga aatgatccat gccgatgaag 382260
acgtagccgc gttcggttag ggtttggacg cagtattgca gcatatcgag cttctcttcg 382320
ctgtcgggaa cggcggcggt atcgatgcgg cgttgcggtt tgaacacgtg cggcaggtgg 382380
gcgtagtgat aaagggcgag gcggtcggga tcgagcgaca aaacggtatc gatggtggtt 382440
ttgatgcttt ccgaagtctg gtgcggcagg ccgtaaatca aatcgacgct gacggatttg 382500
aaccccgctt cgcgcgccgc atcgatgact tctttggttt cttcgtaact ttggatgcgg 382560
ttgaccgccg cctgcacttt ggggtcgaaa tcctgaatgc cgatgctcat gcggttgaag 382620
ccgagtctgc cgagcatgag gacggtgtcg cggctgactt tgcgcgggtc gatttcgatg 382680
gagtattcgc cggtggggat taactcgaaa tgtttgcgta tcatgcggaa gacacgttcg 382740
atctgttcgt cgctcaaaaa ggtcggcgtg ccgccgccga agtgcagttg ggcaagctgg 382800
tgccgtccgt tcagatgtgg agcgagcagt tccatttctt tttcaagata ttcgatgtag 382860
gcatcggcgc ggcttttgtc tttggtgatg attttgttgc agccgcagta gtagcagatg 382920
gtgttgcaga acggaatgtg aatgtaaagg gaaagcggtt tgtttaacgc gcccataccg 382980
cgcaaatgta aagctttgat atattcgcct tcgcggaaac cgtcatggaa acggtcggcg 383040
gtagggtagg aagtgtagcg cgggccgctg gcgggcaggc tggcaatcag cgcgcggtca 383100
aactcggggc ggtcatcgtt tacattgtga ttgttctgta tctgaatgat tttcatggtg 383160
tgtgtgtgcg gttttatgat gttagtcaaa ttttggatag tttggtagaa tgccacagta 383220
tgataaacct gtcttgatat gtgtcaataa gcacatatag tggattaaat ttaaataagg 383280
acaaggcgag gcaacgccgt actggtttaa atttaatcca ctataatcat gatggggcaa 383340
agcgcacaaa aaggtacggt atggcttcgc ataatactac acatcagatg aaaacgctgt 383400
gttcttcctg ttctttgcgg gaactctgcc tgcctgtcgg gctgctgccc aacgagctca 383460
gccaactcga tgccgtcatc cgtcaaagcc gccgcctgaa aaagggcgaa tacctgttct 383520
gtgtcggcga agcctttacc tcgctctttg ccatccgttc gggcttcttc aaaacaaccg 383580
tcgccagtca ggacggccgc gatcaggtaa cgggtttctt tatgtcgggc gaactcatcg 383640
gcatggacgg catctgttcc catgtgcaca gttgcgacgc ggtcgccttg gaagacagcg 383700
aagtgtgcga actgccgttt acccacatcg aagaactggg gcaaaacatc cccagcctgc 383760
gtacgcactt cttccgcatg atgagccgtg aaatcgtgcg cgaccaaggt gttatgctgc 383820
tgttgggcaa tatgcgcgcc gaagagcgga ttgccgcctt cctgctgaac ctttcccaac 383880
gcctttattc ccgaggtttt gctgccaacg acttcatctt aagaatgtcc cgcgaagaaa 383940
tcggcagtta tctcgggctg aaacttgaaa ccgtcagccg cacattatct aaatttcatc 384000
aggaaggatt gatttccgtc gagcataagc acatcaaaat cctcaatctg caggtgttga 384060
aaaaaatggt gtccggctgc tcgcacgcca tttgattaac ccgtacgaac atttcagaca 384120
gcattctcaa taaacacagg gcagacgaaa acatctgtcc tgtttgttgt atctgccgca 384180
aagtgccgtc tgaaaaccgg cagccgccta aatcgaaaaa tcctcgctga tgggcgtgta 384240
cagaatccta tccaccttct cgcgtgtcag gtgcggcgcg aacgcttgga taaagtcgta 384300
ggcatatccg cgcaaataag tatcgctgcg caaagcaatc cacgtcggcg acggctcgaa 384360
caggtgtgcc gcatccacaa gctgcaaatc gccgtccgta tccgggttgt acgccatttt 384420
cgccatcagt cccacgccca aacccaagcg cacataagtc ttcaatacgt ccgtatctgc 384480
cgcagccaat gcgacatcgg gttgttccaa acgggctttg gaaaatgccc gcgcgatgct 384540
gctgcccgca ttgaatgcaa attcataagt aatcagcgga aacctcgcca aatcttcaat 384600
acggaggggg tttctgcatt cgagcaaggg gtggtcgttc ggtacgataa ccgcatgagt 384660
ccagtcatag cagggaagtt ttcccagttc gggatggtcg tctatccgtt ccgtaacaat 384720
cgccaagtcc gcctcgcctg aggtaaccat acgtgcgatg gcggcagggc tcccctgttt 384780
gatggtcagg ttgactttcg gatagcgttt cacaaaatcg gcaacaatca agggtagggc 384840
atagcgtgcc tgagtatgcg tcgtggcaac cgtcagcgaa ccgctgtcct gtccggtaaa 384900
ctcgctgccg atatttttaa tgttctgaac atcgcgcaaa atacgttccg caatatccaa 384960
aaccaccttg cccggctgcg agaccgaaac cacgcgcttg ccgctgcgga taaaaatctg 385020
aatgccgatt tcttcttcca gcaatttgat ttgtttggag atgccgggtt gcgaagtaaa 385080
caaggcttcg gccgcttcgg aaacgttcag gttgtgctgg taaacttcta aggcgtattt 385140
caattgttgt aatttcatgg cgggtcggtg tgggtctgtg tcgggtggct gaacattgtt 385200
tataatttat catattttct tgccggtacg gtatggggct ttgccgttgt gtttgttgtt 385260
tttgtgcaac ggcaatcgtg cgatatggaa aaaatccccc taaagtaatg acacggaatt 385320
gatttttcgg catgatagac tatcaggaaa caggctgttt tacggttgtt ttcaggcgtt 385380
gagtattgac agtccgcccc ctgcttcttt atagtggaga ctgaaatatc cgatttgccg 385440
ccatgtttct acagcggcct gtatgttggc aattcagcag ttgcttctgt atctgctgta 385500
caaatttaat gagggaataa aatgaccaaa cagctgaaat taagcgcatt attcgttgca 385560
ttgctcgctt ccggcactgc tgttgcgggc gaggcgtccg ttcagggtta caccgtaagc 385620
ggccagtcga acgaaatcgt acgcaacaac tatggcgaat gctggaaaaa cgcctacttt 385680
gataaagcaa gccaaggtcg cgtagaatgc ggcgatgcgg ttgctgcccc cgaacccgag 385740

ccagaacccg aacccgcacc cgcgcctgtc gtcgttgtgg agcaggctcc gcaatatgtt 385800
gatgaaacca tttccctgtc tgccaaaacc ctgttcggtt tcgataagga ttcattgcgc 385860
gccgaagctc aagacaacct gaaagtattg gcgcaacgcc tgagtcgaac caatgtccaa 385920
tctgtccgcg tcgaaggcca taccgacttt atgggttctg acaaatacaa tcaggccctg 385980
tccgaacgcc gcgcatacgt agtggcaaac aacctggtca gcaacggcgt acctgtttct 386040
agaatttctg ctgtcggctt gggcgaatct caagcgcaaa tgactcaagt ttgtgaagcc 386100
gaagttgcca aactgggtgc gaaagtctct aaagccaaaa aacgtgaggc tctgattgca 386160
tgtatcgaac ctgaccgccg tgtggatgtg aaaatccgca gcatcgtaac ccgtcaggtt 386220
gtgccggcac acaatcatca ccaacactaa ggctaggcaa tatcttgccg atgcatgagg 386280
ttagtggatt ttgtaccagg tactgttgca atattcgtga aacgtcggtc ggcatcgatg 386340
atgtgaaaca aaccccccgct tttgcggggt ttgtttttttt gggtggtttt ctgaaacggc 386400
tatcgtcaga atcggggtgc aggttcggat tcggattcag attcagattc agattcagat 386460
tcagattcag gtttgtgtcc cattgccgcg ctttatagtg gattaacaaa aatcaggaca 386520
aggcgacgaa gccgcagaca gtacaaatag tacggaaccg attcacttgg tgcttcagca 386580
ccttagagaa tcgttctctt tgagctaagg tgaggcaacg ctgtactggt ttaaatttaa 386640
tccactatat cggttgaaac tctgatttta aggcggtagg atgtgggttt gcccatagaa 386700
agggaatcct ttctgtatca agccctgaaa gggataattc atacaaattc acgcctttcc 386760
ccctcattgg gaaatggatg gaatcgtgcc agatgtgtgc ggcactgtat gccggatatg 386820
gttttatcat cagccctttt cggttgaaac cccgtcagtt gcagcgattg agcctaatcg 386880
gtggcggaag ttgccgcttt gcattcgggg cggcgtgcag tgcggtgctt tgatatgccg 386940
tttgtgtgtt gaaacagggt ggtcggtgca tacgggtacg gtatggccaa agctaaaagt 387000
gaaatacgct gaaacactga atgagccgct ttattgtttg tacggccttt gctgccttgc 387060
tatgatttaa attggattcg cccgccggat attttgggat atgaaagaat ttgacttcat 387120
caaacggtat ttgcaaacag gcacggataa tgatgtcgta ttgggcatag gcgacgatgc 387180
ggcgattgtc cgcccgcgtg aaggcttcga tttgtgtttc agtgcggata tgcttttgaa 387240
ggacaggcat ttttttgcag atgtcaaacc tgaagacttg gcttggaagg ttttggccgt 387300
caatatttca gatatggcgg cgatgggtgc gataccgcgt tgggtgttgc tgagcgcggc 387360
tttgcccgaa ttggatgagg tatggctgaa acggttttgc ggcagctttt tcggtttggc 387420
aaaaaagttt ggcgtaacgt taatcggcgg cgatacgacc aagggcgata tggcgttcaa 387480
tgtaaccatt atcggcgaat tgccgaaggg tagggcgttg cggcgtgatg cggcggttgc 387540
gggcgacgat atttgggtgt cggggcgtat cggtatggcg gcggcggctt tgaactgccg 387600
tctgaaacgg tgtgtgttgc cagatgaagt gtttgccgaa tgcgaacaaa agctgctcca 387660
tcctgaacca agggttgggc tggggcttgc gctgttgccg tttgccaggg cggcgcagga 387720
tgtttcagac ggcctcgcgc aagatttggg gcatatcctg accgcttctg gcaagggtgc 387780
ggaaatttgg gccgattcgc tgccgtcttt atccgtattg aaagatattt tgccccgagc 387840
gcaatggctg tcttatactt tggcgggcgg cgacgattac gagctggtgt ttaccgcgcc 387900
ggaaagttgc cgcagccgcg tatttgatgc ggcggaacgg tgcggcgtgc cggtaacgcg 387960
catcggcaaa atcaacggag gatgccgtct gaaggtttta gatgccgacg gcagggaatt 388020
ggaactacat tctttaggat tcgatcattt tggctgattt taaacctgac tttgcgtggc 388080
tgttgaaacg gccgttgtgt tttttggctt tcggtttcgg cagcgggctg gctccgttcg 388140
cgccgggcac attcggcact ttggcggcac tgcctttggc gtttgtgctg attttgctcg 388200
gcatagacgg gctactgctg gctttttttgt gtatcgtgct gtttatgtgg ggcatacgca 388260
tttgcgctta tgcggaacgt gaaacgggtg tcagcgacca cggtgggatt gtttgggacg 388320
agattgtcgc catgctgttt gtgctggcgt ttgtgccgtt caggtggacg tggtggctgg 388380
cggcatttgt cctattccgt ctgtttgacg cgctcaaacc gtctcccgtc ggttggtttg 388440
acaagaatct gcacggcggt ttgggcatta tggcggacga tatggcggct gcggtgatga 388500
ctttgattgt cttgaggatt gcaatgctgt tttaaacggt gctgccttgt aaaaatgccg 388560
cctgaaagcc tttcagacgg cattgtttcg gaggttaacg cgttaccggt ttgtatttga 388620
tgcgtttcgg tttcgcgcct tcttcgccca aacggcgttt cttgtcggct tcgtattcct 388680
gatagttgcc gtcgaagaac acccatttag agtcgccttc acacgccaag atatgcgtgg 388740
cgatgcggtc gaggaaccaa cggtcgtgcg aaatcaccat cacgctgccg gcaaattcca 388800
acaatgcgtc ttccaacgcg cgcagggttt ccacgtcaag gtcgttagac ggttcatcca 388860
gcagcaatac attgccgccg ctcaacaagg tttttgccaa gtgcagacga ccgcgttcgc 388920
cgccagacaa ttgacctgca attttgcttt ggtcgctgcc tttgaagttg aaacgcccca 388980
aatattggcg ggcgggaatt tcaaactgac caacctgcaa aatgtcgcgg ccttcggcaa 389040
tgttgtcgaa cacggttttg tcgttttgca aaccttcgcg gctttggtca atcaagctca 389100
ttttcacggt ttgtccgatt ttcacctcgc cggaatcagg ctgctctttg cccgaaatca 389160
ttttgaacag cgtagattta cccgcgccgt tcgggccgat gatgccgaca atcgcgcccg 389220
caggcacttt gaagctcaaa tcgtcaatca gcactttatc gccgaacgat ttggaaacat 389280
ttacaaattc aatcacttcg ttacccaaac gctcggcaac gggaataaag atttcctgcg 389340
tttcattgcg tttttggtat tcgtagttgc tcatttcttc aaaacgagcc aaacgcgctt 389400

tggacttggc ttggcggcct ttggcatttt ggcgcaccca ttccaattcc tgcttcatcg 389460
ccttcacgcg cgcggcttcg gattttgcct cgttttccaa gcgtttttct ttctgctcca 389520
gccaagacga gtaattgcct ttccacggaa taccatggcc gcggtcgagt tccaaaatcc 389580
attcggcggc gttgtcgagg aagtagcggt cgtgcgttac cgcaacgact gtgccgggga 389640
agcgcacgag aaattgctcc agccactcga ccgattccgc atccaagtgg ttggtcggct 389700
cgtccagcaa aagcatatcg ggcttgctca acaagagttt gcacaaggca acgcggcgtt 389760
tttcaccgcc ggacaaatta tcgattttgg catcccattc cggcaggcgc agcgcgtcgg 389820
cggcgatttc caattcgtgt tccgcaccgc cgcccgtgga cgaacctgcc gcaataatcg 389880
cttccaagcg gccctgctct tctgccaacg cgtcaaaatc cgcatcagga ttggcgtact 389940
cggcatacac ttcttccaaa cgtttctgcg cggcagccac ttcgcccaaa ccgctttcca 390000
cttcctcgcg cacggttttt tccggatcaa gctcaggctc ttgcggcagg tagccgattt 390060
tgatgccgcc catcggcacg gcttcgccct caaattcctt atccacgccc gccataatcc 390120
gcagcacggt ggacttgccc gcgccgttca aaccgagcag gccgattttc gcgccgggga 390180
agaaagaaag ggaaatatct ttaatgatgg ttttctgcgg cggcacaacc ttgctcacgc 390240
gcagcataga atagacgtat tgttgggaca tggttttctc gttttcatca aacaaatttc 390300
agacggccat tttaaccgat aatttgattt aagccagttt atccgcgaac cggtattgcc 390360
aaaatcgggc aggattcata aaatccgctt atccctttga aattatatag acaaaaaaat 390420
aataatgata ggggatcgcc gccccggcaa ccatttcgga ttttccaaag caaatatagt 390480
ggattaacaa aaatcaggac aaggcgacga agccgcagac agtacagata gtacggaacc 390540
gattcacttg gtgcttcagc accttagaga atcgttctct ttgagctaag gcgaggcaac 390600
gccgtactgg tttttgttaa tctactatac ttttcaaatc aaaaaaggat ttaccttatg 390660
tcggaatata cgcctcaaac agcaaaacaa ggtttgcccg cgctggcaaa aagcacgatt 390720
tggatgctca gtttcggctt tctcggcgtt cagacggcct ttaccctgca aagctcgcaa 390780
atgagccgca tttttcaaac gctaggcgca gacccgcaca atttgggctg gtttttcatc 390840
ctgccgccgc tggcggggat gctggtgcag ccgattgtcg gccattactc cgaccgcact 390900
tggaagccgc gtttgggcgg ccgccgtctg ccgtatctgc tttatggcac gctgattgcg 390960
gttattgtga tgattttgat gccgaactcg ggcagcttcg gtttcggcta tgcgtcgctg 391020
gcggctttgt cgttcggcgc gctgatgatt gcgctgttag acgtgtcgtc aaatatggcg 391080
atgcagccgt ttaagatgat ggtcggcgac atggtcaacg aggagcagaa aggctacgcc 391140
tacgggattc aaagtttctt agcaaatacg ggcgcggtcg tggcggcgat tctgccgttt 391200
gtgtttgcgt atatcggttt ggcgaacacc gccgagaaag gcgttgtgcc gcagaccgtg 391260
gtcgtggcgt tttatgtggg tgcggcgttg ctggtgatta ccagcgcgtt cacgattttc 391320
aaagtgaagg aatacgatcc ggaaacctac gcccgttacc acggcatcga tgtcgccgcg 391380
aatcaggaaa aagccaactg gatcgaactc ttgaaaaccg cgcctaaggc gttttggacg 391440
gttactttgg tgcaattctt ctgctggttc gccttccaat atatgtggac ttactcggca 391500
ggcgcgattg cggaaaacgt ctggcacacc accgatgcgt cttccgtagg ttatcaggag 391560
gcgggtaact ggtacggcgt tttggcggcg gtgcagtcgg ttgcggcggt gatttgttcg 391620
tttgtattgg cgaaagtgcc gaataaatac cataaggcgg gttatttcgg ctgtttggct 391680
ttgggcgcgc tcggcttttt ctccgttttc ttcatcggca accaatacgc gctggtgttg 391740
tcttatacct taatcggcat cgcttgggcg ggcattatca cttatccgct gacgattgtg 391800
accaacgcct tgtcgggcaa gcatatgggc acttacttgg gcttgtttaa cggctctatc 391860
tgtatgcctc aaatcgtcgc ttcgctgttg agtttcgtgc ttttccctat gctgggcggc 391920
ttgcaggcca ctatgttctt ggtagggggc gtcgtcctgc tgctgggcgc gttttccgtg 391980
ttcctgatta aagaaacaca cggcggggtt tgagcgatga gcgataccc cgctacccgc 392040
gatttcggtc tgatcgacgg gcgtgccgta accggctatg tgctgtccaa ccggcgtggt 392100
acgcgtgtct gcgtgctgga cttgggcggg attgtgcagg aattttccgt tttggcagac 392160
ggcgtgcgcg aaaacctcgt ggtgtcgttc gatgatgcgg cttcctatgc ggacaatccg 392220
tttcagatta acaaacagat agggcgcgtg gccggacgca tccgcggtgc ggcgttcgac 392280
atcaacggca ggacttaccg cgtggaggcc aacgaaggca ggaacgcgct gcacggcggt 392340
tcgcacgggc tggccgttac ccgtttcaac gcggtggcgg cagacggccg ttcggtggtg 392400
ctgcgcagcc gcctgcaaca gtcggccgac ggttatccca acgatttgga tttggatatt 392460
tcctaccgct tggacgagga cgaccggctt accgttagct atcgcgccac cgcgctcggc 392520
gacacggtgt tcgacccgac gctgcacatt tactggcggc tggacgcggg cctgcacgat 392580
gcggttctgc atattccgca gggcggacat atgccggccg atgccgaaaa actgcccgtc 392640
tcaacggttt cagacgacct cgaagtattt gatttcagcc ggcccaagcc gctggatgcc 392700
gccgttgccg ccctgcgccg cgaaacgggt cgggccggtt ttgacgacgc ttaccgcgtg 392760
ccgtccgata taggccgtcc cgccgctgtg ttgcaagccg gacgccgccg tcgtatcagc 392820
atatacagcg accgcaatgg cttggtcatc tttaccgccg ccccgcagga tttcgcgcgg 392880
cacgatgcgg gcgtttacga cgcgctggcg accgaggcgc agacgctgcc cgacagcctg 392940
aattggcccg agttcggcaa tattcgtctg aacaagggtg ataccaggga ggcgacgatt 393000
gcttacggca tcgaatccct ttcttaggag cttcctaaca ccggttgcag acgacctttt 393060

tatagtggat taacaaaaac cggtacggcg ttgcctcggc ttagctcaaa gagaacgatt 393120
ctctaaggtg ctgaagcacc aagtgaatcg gttccgtact atttgtactg tctgcggctt 393180
cgtcgccttg tcctgatttt tgttaatcca ctataagatt tcaccattcc ctcaaatcaa 393240
tccaaacagg agcttcataa atgtacacaa gaatcatgga aatcagccct tggacgctgc 393300
gttcggcaaa actggaaaaa gaacacaaac ggctgcaaga gagcctgacc agcttgggca 393360
acggctatat gggtatgcgc ggcagctttg aggaaaccta ttccgccgac agccacttag 393420
gcacctacat cgccggcgtg tggttccccg acaaaacccg cgtcggctgg tggaaaaacg 393480
gctatcccaa atatttcggc aaagccatca acgcgttcaa tttcagcaaa gtcaaaatct 393540
ttgtcgacgg gcaggaagtg gacttggcga aaaacgacgt tgctggcttc tccgtcgaac 393600
tcgatatgca gcacggcgtg ttgcgccgct cgttcaccgt attcggtgtg cgtttcaatg 393660
tgtgcaaatt cctgtctgtc gcacaaaaag agctggcggt catccgctgg gaagccgtat 393720
ccgttgacgg taaaacccac caagtccgca tcgattccat catcgatgcc gacgtgaaaa 393780
acgaagactc caactacgaa gaaaaattct ggcaggtatt ggacaaaggc gtttcagaca 393840
gtctctccta cattgccgcc caaaccgtcg ccaatccctt cggcgtggaa caattcatcg 393900
tcaacgccga gcaaaccttt gccggcagct tcaaagccct cggcggcagc caaaccgact 393960
ggcaggtctc caattctttt gaatccgaag tcggcagcac acccgaaacc tttgaaaaac 394020
gcgtgattgt taccaccagc cgcgattatc agagcttgga agcagtgaaa gccgcaggcc 394080
gcgccttgtc ggaaaaaatt gcaggcgttg cgtttgaaac cttgctggac gcgcacaaag 394140
caggctggct gcaccgttgg gaaatcgccg acgtggtcat cgaaggcagc gacgaagcgc 394200
agcagggcat ccgcttcaac ctgttccaac tgttctccac ctactacggc gaagacgcgc 394260
gactgaacat cggcccgaaa ggctttaccg gcgaaaaata cggcggcgcg acctattggg 394320
acaccgaagc ctacgccgta ccgctctacc tcgcactggc cgaacccgaa gttacccgca 394380
acctgctgca ataccgccgc aaccaactgc cgcaggcgca gcacaacgcg cgcgaacagg 394440
gcttggcggg cgcactctat ccgatggtaa cgtttacggg catcgagtgc cacaacgaat 394500
gggaaatcac cttcgaggaa atccaccgca acggcgcgat tccttacgcc atctacaact 394560
acaccaacta caccggcgac gagggctatc ttgccaaaga aggcttggaa gttttggtcg 394620
aagtgtcccg cttctgggcg gaccgcgtcc acttctccaa acgcaacggc aaatacatga 394680
ttcacggcgt aaccggtccg aacgaatacg aaaacaacat caacaacaac tggtacacca 394740
acaccctcgc cgcatgggta ttggactaca cccgcgaagc cttggcgaaa tacccgcgtc 394800
cggatttgaa cgtgcgtgcc gacgagttgg aaaaatgggc ggacatcagc gcgaatatgt 394860
accgtccgca tgacgaagaa ctcggcgtat tcgtgcagca cgacggcttc ctcgacaaag 394920
acatccgccc cgtgtccgcg ctttcgcccg acgatttgcc gctcaaccaa aaatggtcgt 394980
gggacaaaat cctgcgttcg ccctttatca aacaggcgga cgtattgcaa ggcatctact 395040
tcttcagcga ccgtttcaat atcgacgaaa aacgccgcaa cttcgacttc tacgaaccga 395100
tgaccgtgca tgaaagctcg ctgtcgccct gtattcactc tattctcgcc gccgaactgg 395160
gcaaagaaga aaaagccgtg gaaatgtacc agcgcaccgc ccgcctggac ttggacaact 395220
acaacaacga caccgaagac ggcctgcaca tcacctccat gaccggctcg tggctcgcca 395280
tcgtccaagg tttcgcccaa atgaaaacct ggggcggcaa actcagcttc gcaccgttcc 395340
tgccgagtgc gtggacaggc tacgccttcc acatcaacta ccgcggccgt ctgattaaag 395400
tcgccgtcgg caaagaaaac gtcgtcttca ctctgctcaa aggcgagtcg ctcgatttgc 395460
aggtgtacgg caaagacatc acgctcgacg gcagccacac cgttgcgttg gaaaaataag 395520
gagggcgcaa aatgactttc actgcagtcc tatttgacct cgacggcgtc atcaccgaca 395580
ccgccgaata ccactaccgc gcatggaaaa agctcgccga agaactgggc atcagcattg 395640
accgcaagtt taacgagcag ctcaaaggcg tgtcgcgcga cgattcgctc aaacgcatcc 395700
tcgcgcacgg cggcaaaacc gtcagcgaag ccgagttcgc cgaactgacc cgccgtaaaa 395760
acgacaacta cgtcgagatg attcaggcag tcaaacccga agacgtgtat cccggcattt 395820
tgcccctgct ggaagcattg agggcaaacg gcaaaaaaat cgcccttgcg tccgccagta 395880
aaaacggccc gttcctgctg gaacgcatgg ggctgaccca cttcttcgac gccattgccg 395940
accctgccgc cgtcgcacat tccaaacccg cccccgacat cttcctcgca gcagccgagg 396000
gcgtagatgc ggacatccgc caatgcatcg gcattgaaga cgccgccgcc ggcgtcgccg 396060
ccatcaaagc cgccggcgcc ttgcccatcg gcgtgggcaa agccgaagac ttgggcagcg 396120
acatcgcgct ggtctccggc accgccgagc tgacctacgc ctacctgcaa agcgtgtggg 396180
aacagtcggg caggtaaaac gcgtcagata aagtgtcaag gaagcaaaag accgtctgaa 396240
cagtgtttca gacggccttt ttgcttttag aacagaatga taacccaact tacgcaaccc 396300
taaaaactaa atgccaatct cttaaccatg ctattcaaat ttatttgaac gattttttt 396360
ctaaccagcc aaccttaaca atcactatta aaatgcgcgc cgatgttctg tctccgcctg 396420
tatgcggctt gggcgacggc gaggctgcat tcgagcaggt tgcggttttc gtattcggac 396480
gcggtgtgcg gttcggcttg gttttgcttc caaagctgca gttgggcgat ggcgcggcgc 396540
aggccggtat cgttgcgtag gatgcctaga tggcgttggt tgaacgtttg caggacgggg 396600
cggctgaatg tgttttgaag gtcgtctgaa aagatgcctg cttcggcgga gaggctttca 396660
gacggccttt ggaatggttc ggcttggaat gcttgtccgt ctgcgatggc ttgggcgcag 396720

agccttgcgg tcacgacgca ttcgagcagg gagttgctgg caaggcggtt ggctccgtgc 396780
agcccagtgc aggcggtttc gcccaaggcg tagagctgcg gcagggaggt tctgccgcag 396840
gggtcggttt ggatgccgcc gcaggtgtag tgttgcacgg ggcggacggg gatggcttgg 396900
cgcgtgatgt ctaggccgca ttgggataaa cagtgtcgat ggatggatgg gaaatgccgg 396960
cggacgaacg ctgcgggttg atggctgatg tcgagcgaga cgaagtcttg cgtttgtttg 397020
gcgatttcgg ctgcgatggc gcgggcaacg atgtcgcgcg gtgcgagttc ggcgcggcgg 397080
tcgtaatgcg gcataaatcg ttcgcccgct tggttggtca ggatgccgcc ttcgccgcgc 397140
acggcttcgg aaatgaggaa ggtgcgtccg ttttcagacg gtcttgccaa gcctgtgggg 397200
tggaattgga taaattcgag gtttccaact gcgcagcctg cgcgtatcgc catggcgatg 397260
gcgtcgcccg tgcattcggg cggcgtggtg gtggcggcgt aaatctgtcc caagccgccg 397320
cctgcgagta cggtatggcg ggcgcggatg cggtaggttt cttgtgttcg gcagtcgagg 397380
acggtcagtc cgcacgccgc gcctgattcg gtttgaatgt ccaacgccat ctgccgctcg 397440
caaacgcgga tgttcgggcg gcggcgtatt tgggcaatca ggctctgcat gacggcttcg 397500
cccgtgtagt cggcgacgtg ggcgattcgt cggcaggtat gcccgccttc acgcgtcagg 397560
tgcaggccgt tatgattccg gtcgaacgcc acgccctgcg ccagcagcca ttcgattgcc 397620
ggtttgccct gcgacaggat ggcgcggacg gcggcttcat cacacaaacc cgcgcccgct 397680
tccaaagtat cggcaacgtg tttttcgatg tcgtcctctc ccgaccacgc cgccgcaatc 397740
ccgccttgcg catgacggct ggcggtgtcg tccagccggt ttttgcacaa aataacgatg 397800
cggaacgatt caggcagcga cagggcgagc gtcagtgccg ccagcccgtt tccggcaatc 397860
aatacgtcgc aatcggtttg catggtgttg tccttgtttg agaggccgtc tgaaacggta 397920
tagtggatta atcaatgccc cgacatatgc gacatggtat tgagaagcac cacgcccagc 397980
aaaatcaaac cgatgctgac aatcccaatg aaatcagctt tctcaccgaa aaacaccacg 398040
ctgactaaag ccgttaaaac cagtcccacg cctgcccaaa tggcgtatgc tgtagccagc 398100
ggcatggttt tcagtgtcat agacaaggcc caaaaacaca ccgaaaagct gactaccacg 398160
ccaatagaag gccacagttt gctaaacccg ccactcagtt tgagcatgga agaaccgcag 398220
acttcgctta aaattgctac agtcagaaag agccagtgca tttgcatgtt tttacctgat 398280
aaatgaaaga aagtataatt atatcaatgc aataaaataa aaaaacagtc ttgttgttaa 398340
agattttttg tgtgcaaatc ccgtcttggg aaagcaggcg ggcggtattt tcaggctgca 398400
cccattacga acgacaaatc aggcggggcc catgccgttg aacacatctt ttttcttcag 398460
ccctgccgca aagtcgagca tacgctgcaa aggcagtttg gcggcttcgc ccagcttcct 398520
gtccaacagg atttcgttac gtccgcttgt cagggcgtat ttgatgccgc ccagcgaatt 398580
catcgccatc cacgggcaga acgcgcagct tttacagctt ccaccgttgc ccgccgtcgg 398640
cgcggcgata aattgtttgt cgggcgcctg cttttgcatt tcgtgcagga tgcccaaatc 398700
ggtcgccacg atgaattttt tttcaggacg cgatacggcg gctttgagca gtttgctggt 398760
cgagccgacc acgtcgccca gttcgatgac gctttgcggc gattcaggat gaaccagcac 398820
caccgcttcg ggtgttccg ccttcaacgc cgccagctct tgccctttga attcgttgtg 398880
aacgatgcac gaaccctgcc acaacagcat atccgcgccc gtttcgcggc agatgtagtc 398940
gccgaggtgg cggtcgggtc cccaaatcag cttctcgccg cgtgatttca aatacgatac 399000
gatttctaac gccaccgaag acgttaccac ccaatcggca cgcgctttca cggcggcgga 399060
agtgttggcg tacaccacca ccgtgcggtc ggggtgttgg tcgcaaaacg ctgaaaacgc 399120
ttcttccggg caacccaaat ccaaagaaca ttccgcctcc aaatcaggca tcagcaccgt 399180
tttttcaggg cagaggattt tcgcgctctc gcccatgaag cgcacaccag ccaccaccag 399240
cgtaccggct tcgtgttccg caccgaagcg cgccatttcc agcgaatcgc ccacgcatcc 399300
gcccgtctcc aaagccaaat cctgaatcag cggatcaacg taataatgcg ccaccaagac 399360
cgcgtttttc tccttcagca aagccttgat ttcgtctttc agacgatctg ccgtctcgcg 399420
gtcgggcgtg tcggcaacct tcgcccacgc ctgacggatt tggcaggcgg aagtcggcgt 399480
ttggatgagt ggcatatcgt agtcgaacga gcggcgggcg gcggtttgca tgatgtttcc 399540
ttgtagctgt ttttcagacg gcatgaaggt ttgccgtctg tttttcaaac tgtttttaca 399600
ttatgctcaa cttgagtata atatgcaagg tcgtctgaaa acaggtttgc aataccgtaa 399660
aaccgacccg cttcgttccg acaaaccgct ttggtttaca ataaagcctt tcccacccgc 399720
agaaagccga gcatggatgc ctaccccgaa gccgaagccc cgccgcaaag catcgtcgag 399780
ctggttcccg tattgattgc cgttaccgac ggcggcctgc gggtattgac cgtcgcccaa 399840
ggcatgctcc tgcccaacgg cccgctctcc cccctgcgca attccttgca ggcaggcgta 399900
aaactgtggg tcgccaagca gacttcgcag cctatgggct atgtggaaca gctttacacc 399960
tttgtcgata cccaccgccg caacgaacac ggcatgcccg tgctgtacgt cagctatttg 400020
gggctggtgc gcgaggcagc cgacagcatc ctgcacccgg atgcgaaatg gcaggactgc 400080
tacggctatt tcccgtggga agacttgcgc accgacggcg ggcagcgcga cgccgtcgtc 400140
ggccgcctgc gcatttgggc aaactcggcg gacacggagg aagtgcgcca aaagcggctc 400200
aagcgcattc atttgtgctg gggggtcgaa ccggaaaact ggtcggaaga atacgttttg 400260
caacgctatg aaatgctgta tgaaagcggc ctgatagcgg aagccgccga gccgcaggca 400320
aacttcgact tcgcgcttac ggggcagccc atgcgccacg accaccgccg cgtactggcg 400380

accgccctgt ctcgcctgcg cgccaaaatc aaataccgcc ccgtgatttt tgaactgatg 400440
ccgcccgaat tcacgctgct gcaactgcaa aacagcgtcg aagccatcag cggcagattg 400500
ctgcacaagc aaaacttccg ccgccagatt cagcagcaaa acctcatcga gccgtcggat 400560
accggcgtat cgggcagcaa aggccgtccc gcgcagcttt gccgcttccg cgacgacgtc 400620
ctgcccgaca ggctgatttc ggacatcgga ctgccgctgg gcagccgtta gcccgttttc 400680
agacgaccta tagtggatta acaaaaatca ggacaaggcg acgaagccgc agacagtaca 400740
aatagtacgg aaccgattca cttggtgctt gagcacctta gagaatcgtt ctctttgagc 400800
taaggcgagg caacgccgta ccggtttttg taaaatgaag ttttgcccca tcggtgcaac 400860
atcaatcttt ttcaacaaag gaaaccccat gccgtctgaa aaaaccctct ttcccctgcc 400920
cgacaccctg ttgcgcccca tagtagaaca agccttgagc gaagacttgg gcaggcgcgg 400980
cgatattacg tccgccgccg tcatcgcccc cgacaaaacc gccaaactct tccttgtcag 401040
ccgcgaagac ggcgttatcg ccggcatgga cttggcgcgt ctcgcctttc agacgatgga 401100
tccgtccgtc cgcttccaag ccgaaatccg agacgggcaa gccgtccgcg caggtcagac 401160
gcttgccgcc gtcgaaggca acgcccgcgc gctgctcgcc gccgaacgca ccgcgctcaa 401220
ctacctcacg cacttaagcg gcatcgccac cgccaccgcg cgtgccgttg ccgaagtcgc 401280
cgaatacggt acagacatcg tgtgcagccg caaaaccatc cccctgctgc gtgtcctgca 401340
aaaatacgcc gtcagggcag gcggcggtgt gaaccaccgc atgggtttgg acgacgccgt 401400
gctcatcaaa gacaaccacc tcgcctattg cggcagcatc gcccaagccg tgcagcaggc 401460
aaaacaggct gtcggagcat tgacctgcgt ggaaatcgaa gtggatacgt tggcacaact 401520
ggacgaagcc atcgcagcgg gcgcggaacg gattttgctg gataacatgg acgacgaaac 401580
cctgaaagaa gcggcaaacc gctgccacac gcaaaccgcc cacccccaca ccatctattg 401640
cgaagcatcg ggcggcatcg gcttcgaccg cctgaagcgc gtggcgcaaa ccggagtgga 401700
cggcatcgcc ctcggctatc tgacccacag cagccgttcg ttggacatag gtttggattt 401760
cgtggcgtga gttttagggt gcgggcggct gtctgatatg tcaggcaagg aaccgcttaa 401820
ccctaatccg gttattgcct cagggaggaa atgccgtctg aaagattctt cagacggcat 401880
ttttcgtaaa ggtcgtgatg ctttagaaaa aacagcattt caggcaggta ttttgtttgc 401940
ccgacagcgc ggcggcatcg gtagggcagg aaaaaggacg gggggcggca gttttatgcc 402000
gtctgaaagc ccgcctttac gcttgtttgc aaaaaaagtg ggaaaaggaa catacaatcc 402060
tgtacaatca tccataaata tttgatttat aatacgattt ataaagataa tcacaatcat 402120
ccatatctgc cgcccgtcaa tccgcttggc gggcggcaaa ggttttagga ataccgatga 402180
acacaatacc gctccacacc atactcaaac ttatggcgca tcccgaacgt atggcgatac 402240
tgattcaatt gttggacagc gaacgcaata tcgccgaact ggcaaaatcc ttatccctgc 402300
cggccaccgc agtttccaac catttgaacc gcctgcgcgt ggaaggtcta gtcgatttta 402360
cgcgttacca ccgcattatc gaataccgcc tggtttccga agaagcggcg gcgattctgc 402420
acacggttcg cgatttggaa aacaaacgcg tggcatagtg ttagaatcct ttccttttgc 402480
cgtctgaacg tttcagacag catttttcgg aaatgttatg aaaatcacca cttggaatgt 402540
caattcgctc aatgtgcggc tgccgcaggt gcaaaacctg cttgccgaca atccgcccga 402600
tattttggtt ttgcaggaac tcaaactcga tcaggacaaa tttccggccg ccgctttgca 402660
aatgatgggc tggcactgtg tttggagcgg gcagaaaacc tacaacggcg tggcaatcgt 402720
cagccgcagc gtgccgcagg acgtgcattt cggtttgccc gcactgccgg acgatccgca 402780
acggcgcgtg attgcggcaa ccgtcagcgg cgtgcgcgtc atcaatgtct attgcgtcaa 402840
cggcgaggct ttggacagcc ccaaattcaa atataaggaa cagtggtttg ccgcactgac 402900
ggagtttgtc cgcgatgaaa tgacccgcca cggcaaactg gtgttgctgg gcgatttcaa 402960
tatcgcgcct gccgatgcgg actgttacga ccctgaaaaa tggcacgaaa aaatccactg 403020
ttcgtccgtc gaacggcagt ggtttcaaaa cctgctggat ttgggactga ccgacagcct 403080
gcgccaagtc catcccgaag gcgcgttcta tacctggttc gactatcgcg gcgcgatgtt 403140
ccaacgcaaa ctgggcctgc gtatcgacca tattttggtg tcgcctgcga tggcggcggc 403200
gttgaaggat gtccgcgtcg atttggagac gcgcgcgctg gagcgtccga gcgaccacgc 403260
gccggtgacg gcagaattcg attggtaaaa gaccgtgttt tgatatggcg ttgacaagca 403320
tccttatctt caatttattc aataggatag ctttctatct gactgaaaaa taattgcctt 403380
tccccggcaa acagccgaaa tcggcggatt gttcaaacac agcctatttt cctgaaaaat 403440
ttatgaaata catagggtta atatcagatt ttggagcagt aaaatttatt atgtacacta 403500
atccaaaaca aaatcaaata ttgaaaacta gatttatttt cgaataaata gaaagccgtc 403560
ttatatatag taataaatta ataaccctgt ttttcctatt gcctttattg tgccatgcag 403620
ttgagtttga tgaaactcaa tataacgact gtaaagataa atctatgtta tgtgctgtca 403680
gaattgattc tcccaaaggc aataactata gtggattaac aaaaatcagg acaaggcgac 403740
gaagccgcag acagtacaaa tagtacggca aggcgaggca acgacgtact ggtttaaatt 403800
taatccacta tataaatcta tgtggtttga caatggcaag ttagtattta tatcctttac 403860
taatcaacaa atggaaaatc aaagtcgccc atctctagcg atgtttatta gtgatgacaa 403920
aatatccagt accaatattg atgaattttt agcatctttc gatcctgata aatatcgaat 403980
atttcatgat ccaagatata aatttttacc tagtatgtcg aactcattgt aatccttatt 404040

ctctttttga tattgatagc aaatataaac ctgatgagaa agataaaatc ttttttcaa 404100
tcccgacaga taacacagat ttttataagg gtttttattt aaataaggat tatatagaag 404160
gtatatatcc tagtaggcat aatggcagct attacaaaat atagtggatt aaatttaaac 404220
cagtacagcg ttgccgtact atttgtactg tctgcggctt cgtcgccttg tcctgatttt 404280
tgttaatcca ctatatctgc atcagtttca tgaaacgcaa gtcggaagcg tcaaacaact 404340
gattgcccat tttgaccggc tgattgacga attggacaaa caaatcgacg accacaccca 404400
cacgcatttt gacggcaaag cccaagtggc agaacaaatc aaaggcatcg gttcgataac 404460
gacggctacg ctgatggcga tgctgcccga attgaggcgg ctgtcgcaca aacggatagc 404520
gggtttggcc ggcattgccc cgcacccgag ggagagcggg gaaaccaaat tcaaaagccg 404580
ctgctttggc ggaaggtctg cggtgcgtaa ggcactgtat atggctaccg tggcagcgac 404640
acgttttgaa ccgcttattc gggatttcca ccaacgcccg ctgtccgagg gtaagccgta 404700
taaggttgcc gttacggcat gtatgcgcaa actgctgacg atatcgaatg cccggatgcg 404760
tgattatttt gccgaaaacg ataccgccga aaacggtatc taaacggctt gatttgagtt 404820
ttggtatttt tgcccgacgg ggtgaaaaat acagttgcta cggctcgatg aatcgtcaga 404880
aatacctgca tcgtcattcc cgcgcaggtg ggaatccaga ccggtcggtg cggaaactta 404940
tcaggtaaaa cggtttcttg agatttttcg tcttggattc ccactttcgt gtgaatgacg 405000
gaatgtaggt tcgtgggaat gacgtggtgc aggtttccgt atggatggat tcgtcattcc 405060
cgcgcaggcg ggaatctagt ctgttcggtt tcagttattt tcgataaatg cctgttgctt 405120
ttcatttcta gattcccact ttcgtgggaa tgacgggatt ttaggtttct gattttggtt 405180
ttctgtcctt gtgggaatga cgggatgtag gttcgtagga atgacgtggt gcaggtttcc 405240
gtgcggatgg attcgtcatt cctgcgcagg cgggaatcca gtctgttcgg tttcagttat 405300
ttccgataaa tgcctgttgc ttttcatttc tagattccca ctttcgtggg aatgacggtt 405360
cagttgctac ggttactgtc aggtttcggt tatgttggaa tttcgggaaa cttatgaatc 405420
gtcattcccg cgcaggcggg aatctggaat ttcaatgcct caagaattta tcggaaaaaa 405480
caaaaccctt ccgccgtcat tcccacgaaa gtgggaatct agaaatgaaa agcaacagga 405540
atttatcgga aatgaccgaa actgaacgga ctggattccc gcttttgcgg gaatgacggc 405600
gacagggttg ctgttatagt ggatgaacaa aaaccagtac ggcgttgcct cgccttagct 405660
caaagagaac gattctctaa ggtgctgaag caccaagtga atcggttccg tactatctgt 405720
actgtctgcg gcttcgtcgc cttgtcctga tttttgttaa tccattataa aaatgccgtc 405780
tgaaaggttt tcagacggca ttggttcacg ggccgcgccc gggtatttcg gcaaaatcag 405840
tcggcgaccg ccatcaggct ggcgttgccg ccggcggctg tggtgttgac gctgcaagag 405900
atttcttcaa acacttgcag gatgtcgagt ccgttttccg aagggaggat gcggatgagt 405960
gcgccgtcgt gggcggcaag ttcctgtttg cgcgcgctgt ccaaaggcga cagggcggca 406020
acgtggctga tgccggcggt ttcgggtttg ccgttgacca gcagcagacc ttccaagtcg 406080
gcagtgtagg aagccaaggg gctgtcgggt tcgaccactg cctgtatgcc ggaggcggca 406140
agttcggtca gtgcggcaaa ggcttgaacc gtgctgccgc cgtgtatcca aacgcgtttg 406200
ggcgcgtgcc atgagatgct gttgcgctcg ccggtcggtc cggtaaggac ggtttcggca 406260
cggcgcaggg tgcggatgcg ggcgtgtccc aaagcggccg ctgcggcttt tttctcttcg 406320
gcgttgaacg gtagtttgtg aaccagtgct tcgaggcgtt tgagtgcggc ttcgtccgcc 406380
tgtccgattt ggctcagggt cggggcaacc cattcgccgg cgcgggtcag tttttgcagg 406440
tagaacgaac cgcctgcttt ggggcctgtg ccggacagac cgtgtccgcc gaagggctgt 406500
acgccgacga ctgcgccgac gatgttgcgg ttgacgtaaa cgttgccggc ttcgatgcgg 406560
ctgcggatgt ggcgtaccgt gccttcgatg cggctgtgta cgccgtgggt cagggcgtag 406620
cctttgctgt tgatttggtc gatgacgttg tcgagttcgt cggcgcggta gcggacgacg 406680
tgcaggacgg gaccgaagac ttcgcgttgc agttcgttga ggttgttcaa ttcaaacagg 406740
atggggcgaa cgaacgtgga ttttttggaa tcgacatcgg cggcggtttt gacttcgtgg 406800
taggacttgg caacaccttt cattttgttg atgtggttca acaggttttg ctgtgcttcg 406860
gcatcgatga cggggccgac atcggtagtg agctgaatcg gtttgccgac gacgagttcg 406920
tccatagcgc ctttgatcat gtcgagcata cggtcggcaa cgtcttcttg gacgcacaaa 406980
atgcgcaggg cggagcagcg ttgtcccgcg ctgtcgaagg cggagttcaa tacgtcggcg 407040
cagacttgct cggcaagtgc ggtggaatcg acaatcatgg cgttttgtcc gccggtttcg 407100
gcaatcagga cgggattgtc gccgcgtttg gcaagggctt tgttgatcag gcgcgccact 407160
tcggtcgagc cggtgaaaat cacgccgccg atgcgggcat cgttggtcaa tgccgcaccc 407220
acgtcgcctg cgccgaggac gagttgcagg gcggaagtcg ggatgccggc ttcgtgcatg 407280
agggaaacgg cataaccggc aatcaggctg gtttgttcgg cgggtttggc gatgacggtg 407340
ttgcctgccg ccaatgcgga aacgacttcg ccggtaaaga tggcgagcgg gaagttccac 407400
gggctgatgg cgacaatcgc gccgacggct tttgcgtctt gaggcagggt atgttcggct 407460
tcgtttgcgt agtagcggca gaaatcgacg gcttcgcgca cttcggcaat ggcgttgttc 407520
agcgttttgc ctgcttcgcg cacggcaagc atcatcagtg ctggggtgtg ctgctccagc 407580
aaatcggcaa aacggcgcag gcaggcggcg cgttcggcgg caggtgtcgc actccattcg 407640
gggaacgcgg caacggctgc gccaaccgct tcttgggcaa gcgcggcatc ggcaaagctg 407700

actgtgccga cgatgtcgtc gtggtcggca gggtttttaa tcggttgcgc ttcgccgaca 407760
tcgcgggctt tgccgttgac gatggatgcg gcgtggaagt cttgcgcggc ggctttgttc 407820
atctgttctt gaagctgctg caatacgttt tcgttgctca agtccacgcc ttgcgagttc 407880
agacggcatt tgccgtacaa atcgcgcggc agcggcaggg cgttgtgcag gtggatgcct 407940
tgttcggcga tggtgtcgaa cgggctgcgg atgagcgtgt cgatgctgat gttttcatcg 408000
acgatttggt tgacgaaaga cgagttcgcg ccgttttcca acaggcggcg caccaagtag 408060
gcgagcaggg tttcgtgtgt gccgactggg gcgtacacgc gcacgcggcg gcctaagttt 408120
tgcgggccga cgacttggtc gtacagggtt tcgcccatac cgtgcaggca ttggtgttca 408180
aaatctttgc ctttacccat ttggtagatt gcgcccaaag tgtaggcgtt gtgggtggca 408240
aattgcggga ataccgcgtc ttgcgcggaa agcagtttgc gcgcgcaggc gaggtaggag 408300
atgtcggtgt ggactttgcg ggtgtaggtc ggatagccgt tcaagccgtc cacttgcgcc 408360
catttgattt cgctgtccca atacgcgcct ttgacgaggc ggatcattag tttttggttg 408420
ttgcggcggg caaggtcgat caggtagtcg ataacgaacg gacaacgttt ttggtaggct 408480
tggacaacga aaccgatacc tttgtagcca gccaagtcag ggtctgaaac caaagcctcc 408540
atcaaatcca aagacagctc cagacggttg gcttcttcgg catcgatgtt gataccgata 408600
tcgtattttt tacccaaaag gaacagctct ttcaggcgcg gcaacagttc gcccatcacg 408660
cggccgtgtt gggtgcgcga gtagcgcgga tggatggcgg aaagtttgac ggaaataccg 408720
ttaccttcgt aaacgccttg tcctgccgca tctttgccga tggcgtggat ggcttcgaca 408780
tagtcgcggt agtagcggtc ggcatcggct tgggtgtagg cggcttcgcc caacatatcg 408840
aaggagaagc ggtagcccat tttttcgcgt tctttgccgt tttgcagggc ttcttcaatg 408900
gtctgtccgg ttacgaactg tttgcccaga agccgcatgg cgtaatttac gccttggcgg 408960
atgagcggtg cgccgccttt gctgatcagg cggctgagtg cggaactcat ttgtttgtcg 409020
tttgtggcgg tcagtttgcc ggtaatcagc aggccccagg cggcagcatt gacgaagagg 409080
gaagggctgt tgttcaaatg gcttttccag ttgccgtctg aaatcttgtc ggcaatcagg 409140
cggtcgcgcg tggcgttgtc ggggatacgc agcagggctt ctgccagaca catcagcgcg 409200
atgccttctt cgctggagag tgaaaactcg tgcatcagcg catccacgcc gccggctttg 409260
gtgcggccgg cgcggacttg ggtaaccaaa cggcgggcaa gctcggaggc ggcgttgcgc 409320
tcttcgtcgc tcatctgtgc acgttgcaac atatcctgta cggcttcgat ttcattacgg 409380
cggtaggcat cggttatcgc ttggcgcagg gcagtttgtg ccggaaatgc aaaatgaaac 409440
atttttttgga ttctccaaag tttttcgggg ggcaggcggc atcggtgcgg cctgaatacg 409500
gtaatatcgt aataaatccg cagatgaaat acaaggcttc aaatgcgggc agggtaggtg 409560
cttccgtttc tttgaaaatg aaacgggtaa aacacaaata aggcctgtat gcaggcaagg 409620
tttatttgtg tttgacccgg aaacgggttc agacggcacg aaccgggatg ccgtgccgtc 409680
tgaaaggggt ttatcgggtg gcgcggtaat ctgcgtcggc tttttcaaag cgttcttggg 409740
tttcgcgcga aggttctttg ttgaacaggg aaaccaacac ggcaacgatc aagcaaacaa 409800
taaagcccgg cacgatttcg tacatcgtca acaagccgct ttctcctgcc gcttgagccg 409860
gttttttcac ccattccgcc catacgacta cggttaacgc acctgcaacc atacccgaca 409920
acgcgccgta ggcagtgatg cgtttccaca atacggacag aatcacaatc gggccgaatg 409980
ccgcgccgaa acctgcccac gcgtaagaca ccagtcccaa tactttgctg ttcggatcgg 410040
aagcaatcag gatggaaatc acggcaatcg ccaagaccat caggcggccg acccatacca 410100
attccgactg ttgcgcgttt ttacgcaaaa agcctttgta gaagtcttcg gtaatcgcgc 410160
tggagcaaac caaaagctgg caggacaggg tggacatcac cgccgccaaa atcgcgctca 410220
aaataatgcc ggcaatccaa gggttgaaca gcagggtgga aagcgcgatg aagatgcgtt 410280
cgtggttgcc gctcatagaa gaaactttgt cgggatttgc accgaaatac gcaatgccga 410340
aataaccgac cgctaccgcg cccgcaaggc acaacgccat ccaagtcata ccgatgcggc 410400
gtgcggatac cagcgatttc gcgctttcgg ccgccataaa gcgcgccaaa atgtgcggct 410460
gtccgaaata gcccaagccc catgcggcgg tggaaatgat gccgatgacg gtcgtaccgg 410520
caaacaggct gccgtattct ttgcccgtgc ctgcggcgac actttgaatc gcggcagaca 410580
tctgttccgc gccgcccaag cccagataga ccatcacagg cgttaaaatc agcgcgaaaa 410640
tcatcaaaga agcctgcagc gtatccgtcc agcttaccgc caaaaagccg cccaagaagg 410700
tataggcgat ggtcgcgccc gcgcccagcc acattgcctg attgtaagtc ataccttcaa 410760
acaggctttg gaacagggtt gcgcccgcca caatgcccga ggcgcaataa atcgtgaaga 410820
aaaacaggat aatcagtgcg gaaaccactt tcatcaagtg tccgcccgcg ccaaagcggt 410880
ggaagaaata atccggcagc gtcagcgcgt tgttggcgta ttcggtatgt acgcgcagac 410940
ggcccgccac caaaagccag ttgaaatacg cgccgaccaa gaggccgatg gcaatccaag 411000
cctcattcaa accgctcaaa taaatcgcgc ccggcagacc catcaaaagc cagccggaca 411060
tatcggacgc gcctgccgac atcgcggtaa caaacgggcc taggctgcgc ccgcccaaaa 411120
tataatcgtc gaaattgcgc gtagaaaaat aggcggcaag cccgatgaga aggactgcaa 411180
ccagatagat tgcaaaagta atgtacatgg gattcatgtg ctattcctcg tctaaaactt 411240
cagaattaca ggctttgaaa ttgcaagcaa cttgcgcctg aaatgttttt ctaataaaag 411300
tacaacggaa aatccggata cccgaaaggg ggattcggat aaattatctt caatcacaat 411360

aagatatgta ataaaactat atgaaattgt aaataatccg tttcaggata acccaatttc 411420
tgttgtttgc aaagcactta atggcttaaa aagccgagtt tgaaacgatg cgcgtcggaa 411480
aaatcattta aaacagcata ttgttttgta gtgtcttgta atcgggcgtt gcgcggaata 411540
tgaaatccgt tttcaggcgg caggtgtttt gaggtgtaat ttagcaaccg caaaggaggc 411600
gcggtatgtt ttgccgatta tccgccgccc gttttcagac ggcatttttc cttatacaat 411660
agccgattga atttgatatg ttcaggaagg atacagatta tgttcggcaa gcagcttttt 411720
gaggaagtcg gctcgaaaat cagcgaaacc atcgccaaca gccctgccaa agatgtggaa 411780
aaaaatatta aggcgatgct gggcggcgcg ttcaaccgta tggatctggt tacgcgcgaa 411840
gaattcgaca tccagcagca ggttttaatc aaaacccgta ccaaactggc ggctttggaa 411900
gcgcgtttgg aaaaactcga agccgcgcaa aatcccgaac gggcagcatt ggaagcggct 411960
gaagccgctg ccgaagaagc cgtcgccgaa atcaggcagc aaaccgaagc cggcgaataa 412020
ggtcgtctga aatatgtcgc ttgccttggt ttacagccgc gccttgagcg gtatgaatgc 412080
gccgttggtc gaagtggaag cccaccttgc caacggcctg ccacatttca acatcgtcgg 412140
actgcccgat atggaagtaa aggaaagtcg cgaccgtgtc cgtgccgcca ttattcaaag 412200
cggttttgaa ttccccgcca aaaaaattac cgtcaacctc gcccccgccg acctgcccaa 412260
agagtcgggg cgtttcgatt tgccgattgc aatcggcatc cttgccgcat cggggcaggt 412320
tgcgcccgaa aaactggagg aatacgagtt tgcgggggaa ttggcactgt cggggctgtt 412380
gcgccccgtg cgtggcgcgt tggcgatggc gtggcagggt atgcaggcaa aacgtgcatt 412440
tgttttgcct gaagaaaatg caggacaagc cgccgtgatg cgcggcatta ccgtttacgg 412500
cgcgcgctct ttgggcgaag tcgccgccca tttgaacggc atcgaacctt tggcgcaaac 412560
cgaatgccaa gttcctcaga tgccgtttga acatggcgga caacctgatt tgtgcgatgt 412620
gaaaggtcag cacaccgcgc gccttgcttt ggaaatcgct gccgcaggcg gacacagcct 412680
cttgatgatg ggtccgccgg gaacgggcaa gtctatgctc tcccaacggc tgcccggcat 412740
cctgccgccg ctgaccgaag acgaattggt agaagtttgg gcattgcgtt cgctcctgcc 412800
caaccaccaa caacaactcg acagcaaccg tcctttccgc agtccgcatc acagcgccag 412860
cgcggcggct atggtcggcg gcggttcgga tccgcgtccg ggcgaaattt cattggcgca 412920
ccacggcgtt ttgtttttgg acgagctgcc cgagtttgac cgcaaagttt tggaagtttt 412980
gcgcgaaccg ttggaaaacg gcgaaatcca catttcccgc gcggcgcgcc aagccgtcta 413040
tcctgccaaa ttccaacttg ttgccgccat gaacccctgc ccgtgcggtt atctcgggca 413100
tcccgtcaaa ccctgccgct gcacgcccga aagcgtcgcg cgttaccgca gcaagatttc 413160
cgggccgctg ctcgaccgca tcgatttgac catcgaagtc ccgagcctgt ccgccgccga 413220
actgatgcag caggaagcag gggaaagcag cgcgtccgtt ttggaacgcg ttatcgccgc 413280
tagagacaaa caatacgcac ggcaaggcaa agtgaatgcc gccttgagtg tcagtgaact 413340
cgacacatcc gcccgcattc aaaaagaagc gcaggaagca ttgggcggcc tgctggaaaa 413400
actctccctt tccgcccgca gcttccaccg cattatgcgc gtggcgcgta cattggcgga 413460
tttggcgggc gacgaagaag tcggcagaag ccacgtcatg aaagccatag gtttccgtcg 413520
tgctttatag gaatgggaat ggaagcaggt tttgcccaaa tatggcgata ttgttagaat 413580
atccgcccgt aagcaaacgg cgttaatgcc gtctgaaaca cattaaggta tgtttatgaa 413640
caaattttcc caatccggaa aaggtctgtc cggttttttc ttcggtttga tactggcgac 413700
ggtcattatt gccggtattt tgttttatct gaaccagagc ggtcaaaatg cgttcaaaat 413760
cccggcttcg tcgaagcagc ctgcagaaac ggaaatcctg aaaccgaaaa accagcctaa 413820
ggaagacatc caacctgaac cggccgatca aaacgccttg tccgaaccgg atgctgcgac 413880
agaggcagag cagtcggatg cggaaaaagc tgccgacaag cagcccgttg ccgataaagc 413940
cgacgaggtt gaagaaaagg cgggcgagcc ggaacgggaa gagccggacg gacaggcagt 414000
gcgtaagaaa gcgctgacgg aagagcgtga acaaaccgtc agggaaaaag cgcagaagaa 414060
agatgccgaa acggttaaaa aacaagcggt aaaaccgtct aaagaaacag agaaaaaagc 414120
ttcaaaagaa gagaaaaagg cggcgaagga aaaagttgca cccaaaccaa ccccggaaca 414180
aatcctcaac agcggcagca tcgaaaaagc gcgcagtgcc gccgccaaag aagtgcagaa 414240
aatgaaaacg tccgacaagg cggaagcaac gcattatctg caaatgggcg cgtatgccga 414300
ccgtcagagc gcggaagggc agcgtgccaa actggcaatc ttgggcatat cttccaaggt 414360
ggtcggttat caggcgggac ataaaacgct ttaccgggtg caaagcggca atatgtctgc 414420
cgatgcggtg aaaaaaatgc aggacgagtt gaaaaaacat gaagtcgcca gcctgatccg 414480
ttctatcgaa agcaaataat tatgaagctc aaacatctgt tgccgctgct gctgtcggca 414540
gtgttgtccg cgcaggcata tgccctgacg gaaggggaag actatcttgt gttggataaa 414600
cccattcctc aagaacagtc gggtaaaatt gaggttttgg aatttttcgg ctatttctgc 414660
gtacattgcc atcatttcga tcctttgtta ttgaaactgg gcaaggcatt gccgtctgat 414720
gcctatttga ggacggagca cgtggtctgg cagcctgaaa tgctcggttt ggctaggatg 414780
gcggctgccg tcaatttgtc gggtttgaaa tatcaggcaa accctgctgt gtttaaagca 414840
gtttacgaac aaaaaatccg cttggaaaac aggtcggttg ccggaaaatg ggctttgtct 414900
caaaaaggct ttgacggcaa aaaactgatg cgcgcctatg attcccccga agctgccgcc 414960
gccgcattaa aaatgcagaa actgacggaa caataccgca tcgacagcac gccgaccgtt 415020

attgtcggcg gaaaataccg cgttatcttc aataacggct ttgacggcgg cgttcatacg 415080
attaaagaat tggttgccaa agtcagggaa gaacgcaagc gtcagacccc tgctgtacag 415140
aaatagccga actcccgtat ccgaaagaag cgcaagcaat ggattttctg attgtcctga 415200
aagccctgat gatgggcttg gtagaaggtt ttaccgaatt tttaccgatt tccagcaccg 415260
gacatttgat tgtgttcggc aatctgattg gttttcacag caatcacaag gtttttgaaa 415320
ttgccatcca gctcggtgca gttttggcgg tagtgtttga ataccggcaa cgtttcagca 415380
atgtgttgca cggcttggga aaagaccgga aagccaaccg cttcgtcctt aatcttgcca 415440
ttgcttttat acctgccgcc gtgatggggc tgttgttcgg caaacaaatc aaagagtatc 415500
tgtttaaccc cttgagtgtt gcagtcatgc tggttttggg cggttttttt attttgtggg 415560
tggagaaacg ccaaagccga gcagagccta aaattgccga tgttgatgca ttgcgtccga 415620
ttgatgcctt gatgatcggc gttgcccaag tgtttgcact ggttccgggt acgtcccgtt 415680
cgggcagtac gattatgggc gggatgcttt ggggcatcga acggaaaact gcgacagaat 415740
tctcgttttt cttggctgtg ccgatgatgg ttgccgcaac ggcttatgat gtcctgaaac 415800
attaccgatt tttcaccctg catgatgtcg gtttgattct gataggcttt attgctgcct 415860
ttgtttcagg cttggtagcg gtaaaagcgt tgctgaggtt tgtttccaag aaaaattata 415920
ttccttttgc ctattaccgc attgtttttg gtattgccat cattatattg tggctgtcag 415980
gctggataag ttgggaatga aaccataaac ccgacctgaa gacattattc gggtcgggtt 416040
tgtctggcgg gctgatatag tgaattaaca aaaatcagga caaggcgacg aagccgcaga 416100
tagtacggca aggcgagcca acgctgtacc ggtttaaatt taattcacta taaaatcagg 416160
acaggcgggg cgataggttt aaagtcgatt gcctgttttg aaggcagtgg tttattcttt 416220
atttgctggc aatcaggcaa taaaaaagca catacctttt tacggtctgt gctttttttat 416280
ctggtggagg taagcgggat cgaaccgctg acctcttgca tgccatgcaa gcgctctacc 416340
aactgagcta tacccccgaa aatttggtgg cgaatcaggg actcgaaccc cggacacaag 416400
gattatgatt cctctgctct aaccgactga gctaattcgc cgtttcgtga agacgctatt 416460
atatgttttt ctgttttttt gacaagccgt attttttaat tttgaattag ttgactgttt 416520
ttaaatgtta aaaagtttat gccgtctgaa gcggattcag gcggcatgag ggttagagtt 416580
tgtggcagat gtcgccgaag cggaatcctg cccagtcgat gccgatattt tttccgaatg 416640
cgatgacttt aaacagttcg cccatttcat gctggtcaat cagtttctga acggcagcag 416700
cttcacagat gtaggctgcc gaatccgttt tccccgtctg tgccaatagc tcggtaatgc 416760
ccaagttcaa taagaaatgg gattggggaa ggtaacctat caaatctaat ccggcatccg 416820
tccctgcttg tgcaatgtcg gtaaagttga catgtgcggt caggtcggcc aatccgatga 416880
agtcaaaagg attgtggata atgtgatgtc ggtagtgtcc gatcagagta ccttgattgc 416940
gttgagggtg gtaatactgc gctgcatcaa aaccgtagtc gatgaatatc atgcagccgt 417000
gttcgagtct tgaggcaagg gtgcggataa aggcatattg ttgcggatgt agttcgctgg 417060
tatagggata atctgtttga ggaaaataga gggaagccaa ggcagatagc tgcaagtcgt 417120
gcagcggtcg tgccgaatag gtaaaacggt cattatctag gcaaacgccg acatgctcga 417180
atgagccgcc ttcatttta cggacgattt cgacaggcat ggcatcgagt acttcgttgc 417240
cgatgatgat gccgtcaaac gcttcgggaa gtgcggtcaa gtggacaact ttttgagatg 417300
cttccggtgc gcgtgcttga atcaggtttt tctgacgtgc tgccagctcc ggcgatattt 417360
caataatata gtaacggctg atgccgtccg aaatgctgcc caacaaatcg gcggcaagct 417420
gtccggttcc cgcgccgaat tcatagatat tgcccgccgt ttgggataga agttcttgaa 417480
gttggcgtgc cagtgtctgt gcaaacagag aggtgagggt cggtgcggta ataaaatccc 417540
cggtattgcc gattttatgg ctgccgccgg tgtagtagcc gtattgcgga gcgtataaaa 417600
ccaattccat aaaacgtgaa aatggaatcc agttgccgtg tttgccgatt ttttcggcaa 417660
tgagggtttg cagtttgagc gagaattgcc gtgcttcggg agaggggagg ggcatgataa 417720
gtgttagctt gtgtaaattt attggatttc ccgacatatt acacgttggt acgggtgctg 417780
tcatggcttt atcttaatac tatatattgt gtttatatta ttaaattaat catatatagt 417840
tgtttattgg ttcgattatt ctgtaccgca cccgccgtgc cgttgtcgtc attttttatc 417900
ttattgtttt taaaaggaat aaaaatttca gatatgttaa tgagttttca tgccctgatt 417960
tgaccgagtg tttaaaattt cttatagtgt cgattggtgg ggaattgtgg ggcaaagtgt 418020
ctcttttacc cttgtgattt tgatttcggc ttgggacatg tcatgttcgg cggcgcacac 418080
gaattaagca tcgacagtaa ggggcggttg gctgttcctg ccaaattccg tgacattctg 418140
tcgcgcctct atacgcctgc cgtagtggta acgctcgagt cgaaacacaa gctgttgatg 418200
taccctgttg cggagtggga aaaggttgcg gcgcaacttt taaacttaaa agtggcggat 418260
aaccctgttt tgcggcggtt tcaaaatctt ttgctgcata acgcggaaat tttggaatgg 418320
gacagcgccg gccgggtgct ggtttctgcc ggactgagga agagggtgga tttcgaccgt 418380
gaagtcgttt tggtcggtcg tgccaaccgt ttggagcttt ggggtcgcga gcagtgggag 418440
gctgagatgg ttcaggcttt ggatgacgat cctgacgaac ttgccttcca gttgagtcag 418500
acggatttgc aattgtgagt ggagcagaaa gttaccggca tatcacggtc ttgctgaatg 418560
aggcggtgga tgcgcttgcc gtgcgcgaag acggtgtcta tgtggacggt acgttcggca 418620
ggggagggca ttcccggctg attttgtcgc gtttgggcga tgcggggcgg ttgattgttt 418680

tcgacaaaga cccgcaggcg attgctgtgg cagaagagct ggcgcgttcg gacaaacggg 418740
tcggtgtcgt gcatggcggt tttgcttcgt ttcagacggc attggacggt ttgggtatcg 418800
gcaaggtgga cggtgcgctg tttgatttgg ggatttcgtc cccgcaaatc gatgacggca 418860
gccgcggttt cagcttccgt ttcgatgccc ctttggatat gcgtatggat acgacgcgcg 418920
gtatgtctgc cgcagagtgg atagcggttg cgtcggaaca ggatttgcac gaggtaatca 418980
agaattatgg tgaagagcgg tttagccgcc ggattgcgcg cgccattgtt gcgcaacggg 419040
cggaaagtcc aatcgataca acccgcaagc tggcgcagat cgtggcacaa aacgtccgta 419100
ctcgcgagcg ggggcaggat cctgcgacgc gcaccttcca ggcggtccgc atctttatta 419160
accgcgagct tgaagaagta ggggcagtat tgccgcaggt catgtgtcgt ctgaaagagg 419220
gcggacgttt ggcggtcatt gctttccatt cgttggaaga tcgcattgtg aagcagtttg 419280
tcaaaaaata ttcgcaacac gcgcccctgc cgcgctgggc ggcggtcagg gaagcggatt 419340
tgcccgagct gcccctgaaa atcgtgggca gggcattaaa gccgggtgag gcggaaattg 419400
ccgccaatcc gagggcgaga agtgcggttt tgcgtgtggc ggagcggact gccggtccga 419460
taccggaaca atcacagaga aaaacgtctg aatggcaatg aacaaattga atttccttct 419520
gctgcttgcg gtgtgcgttt ccgctttttc cgttgtgatg cagcaaaacc agtacaggct 419580
caatttcaca gctttggata aggcgaaaaa acaggaaatc gccttggagc aggattatgc 419640
gcaaatgagg ctgcaacagg cgcgtttggc gaaccacgaa gcgatcaggg cggcggcaga 419700
aaaacaaaac ctccatccgc cggtttcggg caataccttt atggtggagc atcaaagata 419760
gaagcagcct gtgtgccgga atcggattcc tgcgtcagga taataataac gagaagtaaa 419820
aatgttgatt aagagcgaat ataagcctcg gatgctgccc aaagaagagc aggtcaaaaa 419880
gccgatgacc agtaacggac ggatcagctt cgtcctgatg gcaatagcgg tcttgtttgc 419940
cggtctgatt gctcgcggac tgtatctgca gacggtaacg tataactttt tgaaagaaca 420000
gggcgacaac cggattgtgc ggactcaaac attgccggct acacgcggta cggtttcgga 420060
ccggaacggt gcggttttgg cgttgagtgc gccgacggag tccctgtttg ccgtgcctaa 420120
agagatgaag gaaatgccgt ctgccgcaca attggaacgc ctgtccgagc ttgtcgatgt 420180
gccggttgat gttttgagga acaagctcga acagaaaggc aagtcgttta tctggattaa 420240
gcggcagctc gatcccaagg ttgccgaaga ggtcaaagcc ttgggtttgg aaaactttgt 420300
atttgaaaaa gaattaaaac gccattaccc gatgggcaac ctgtttgcac acgtcatcgg 420360
atttaccgat attgacggca aaggtcagga aggtttggaa ctttcgcttg aagacagcct 420420
gcatggcgaa gacggcgcgg aagtcgtttt gcgggaccgg cagggcaata ttgtggacag 420480
cttggactcc ccgcgcaata aagccccgaa aaacggcaaa gacatcatcc tttccctcga 420540
tcagaggatt cagaccttgg cctatgaaga gttgaacaag gcggtcgaat accatcaggc 420600
aaaagccgga acggtggtgg ttttggatgc ccgcacgggg gaaatcctcg ccttggccaa 420660
tacgcccgcc tacgatccca acaggcccgg ccgggcagac agcgaacagc ggcgcaaccg 420720
tgccgtaacc gatatgatcg aacccggttc ggcaatcaaa ccgtttgtga ttgcgaaggc 420780
attggatgcg ggcaaaaccg atttgaacga acggctgaat acgcagcctt ataaaatcgg 420840
accgtctccc gtgcgcgata cccatgttta cccctctttg gatgtgcgcg gcatcatgca 420900
gaaatcgtcc aacgtcggca caagcaaact gtctgcgcgt ttcggtgccg aagaaatgta 420960
tgacttctat catgagttgg gcatcggtgt gcgtatgcac tcgggctttc cgggcgaaac 421020
tgcaggtttg ttgagaaatt ggcgcaggtg gcggcctatc gaacaggcga cgatgtcttt 421080
cggttacggc ctgcaattga gcctgctgca attggcgcgc gcctataccg cactgacgca 421140
cgacggcgtt ttactgccgg tcagctttga aaaacaggcg gttgcgccgc aaggcaaacg 421200
catattcaaa gaatcgaccg cgcgcgaggt acgcaatctg atggtttccg taaccgagcc 421260
gggcggcacc ggtacggcgg gtgcggtgga cggtttcgat gtcggcgcga aaaccggcac 421320
ggcgcgcaag ttcgtcaacg ggcgttatgc cgacaacaaa cacatcgcta cctttatcgg 421380
ttttgccccc gccaaaaatc cccgtgtgat tgtggcggta accattgacg aaccgactgc 421440
ccacggttat tacggcggcg tagtggcagg gccgcccttc aaaaaaatta tgggcggcag 421500
cctgaacatc ttgggcattt ccccgaccaa gccactgacc gccgcagccg tcaaaacacc 421560
gtcttaatcc gagtatcaac gagattgttt tatgttcagc aagttaaccc ctttggctga 421620
aaccggcatc ccgactctgt cgtgtgcaaa cgcggcaggg cgtttgttgc attcagacag 421680
ccgccaaatc aaacaaggcg atattttcgt tgcctgtccg ggcgaatatg ccgacggacg 421740
cagttatatc cccgccgccg ttgccaacgg cgcggctttt gttttttggg acgacgacgg 421800
caaatttgcg tggaatcccg aatggaaagt ccccaatcaa ggcatcaaag atttgaaaca 421860
ccgtgccggc atattggcgg cgcaagttta cggcaacgtt tcagacggcc tcaaagtttg 421920
gggcgtagcc ggaaccaacg gcaaaacctc catcacacaa tggctggcgc aagctgccga 421980
tttgttgggc gaaaaaaccg ccattgtcgg cacggtcggc aacggctttt ggggtgcatt 422040
ggaagaaacc acgcatacca cacccgcccc cgtcgatgtc caaaccctgc tctaccgttt 422100
ccgtcaacaa ggcgcaacag tcgccgcgat ggaagtctcc agccacgggc ttgaccagtc 422160
gcgcgtcaac ggcgtgtcat tccgcagcgc aatctttacc aacctcaccc gcgaccacct 422220
cgactaccac ggcacgatgg aagcctacgg tgccatcaag tcgcgcctgt tttactggca 422280
cggcttgaaa cacgcagtca tcaacgtgga tgacgaatac ggcgcggaac tcgtaggtcg 422340

tctgaaaaaa gactgtcccg atttggccgt ttacagctat ggtttcagcg aacacgccga 422400
catccgcatt accgacttta ccgcctcttc agacggcata gcagccgtat tccaaacccc 422460
gtggggcgaa gggaaatgcc gcacgcgcct gctcggacgg ttcaacgcgc aaaacctcgc 422520
cgcctgcatc gccttgctgt gcgccaacgg ctatccgctt gataaggtat tggatgtgct 422580
ggcaaaaatc cgtcccgctt cagggcgcat ggactgcatc atgaacagcg gcaagccctt 422640
ggtcgttgtc gattatgccc acacgcccga cgcattggaa aaagcactcg ccaccttgca 422700
ggaaatcaaa ccgcagggtg cggctttatg gtgcgtattc ggttgcggcg gcaaccgcga 422760
tcgcggcaaa cgcccgctga tgggcgcggc agccgtacag ggcgcggata aagtcgtcgt 422820
caccagcgac aacccgcgtt tggaaaatcc gcacgacatc atcaacgaca tcctgcctgc 422880
cgttcccgcg cccgaatgcg tcgaagccga ccgtgccgcc gccgtccgtt atgcggttga 422940
acaagccgcc gcaaacgaca tcatcctgat tgccggcaaa gggcatgaaa actatcagga 423000
tgtacaaggc gtgaagcacc gtttttccga tcttgaaatc gtcggacagg ctttgttaac 423060
tcgtaaataa tgggatattc ggacggcatc gtatgaaaca atccgcccga ataaaaaata 423120
tgaatcagac attaaaaaat acattgggca tttgcgcgct tttagccttt tgttttggcg 423180
cggccatcgc atcaggttat cacttggaat atgaatacgg ctaccgttat tctgccgtgg 423240
gtgctttggc ttcggttgta tttttattat tattggcacg cggtttcccg cgcgtttctt 423300
cagttgtttt actgatttac gtcggcacaa ccgccctata tttgccggtc ggctggctgt 423360
atggtgcgcc gtcttatcag atagtcggtt cgatattgga aagcaatcct gccgaggcgc 423420
gtgaatttgt cggcaatctt cccgggtcgc tttattttgt gcaggcatta tttttcattt 423480
ttggcttgac agtttggaaa tattgtgtat cggggggggg tatttgctga cgtaaaaaac 423540
tataaacgcc gcagcaaaat atggctgact atattattga ctttgatttt gtcctgcgcg 423600
gtgatggata aaatcgccag cgataaagat ttgcgagaac ctgatgccgg cctgttgttg 423660

aatattttcg acctgtatta cgatttggct tccgcgccgg cacaatatgc cgccaagcgc 423720
gcccacattt tggaagcagc aaaaaaagcg tcaacatggc atatccgtca tgttgcgccc 423780
aagtataaaa attatgttgt ggttatcggt gagagcgcgc gttcggatta tatgaatgtt 423840
tacggtttcc cattgcccga tacgcctttt ttgagtcaga ccaaagggct gttgataaac 423900
ggttaccaat cgaccgccca cgcgacgaat ctttcgctgc cgcagacttt ggggctgccg 423960
ggagaaccga acaataacat cgtcagcttg gcgaagcagg cgggttttcg gacggcgtgg 424020
ctgtctaatc aaggaatgtt ggggcatttt gccaacgaaa tttccaccta tgccctacgc 424080
agcgattatc cgtggtttac ccaaaggggt gattatggca aaagcgcggg gttgagcgac 424140
cgccttttgt tgccggcgtt caaacgggtt ttgataggaa atgcaggcac gaagcctcgg 424200
ctgattgtga tgcacctgat gggttcgcac agtgattttt gcacacgttt ggataaggat 424260
gcgcggcggt ttcagtatca aactgaaaaa atatcctgct atgtttccac catcgcgcaa 424320
accgataaat ttttagaaga tacagttaag atattgaatg aaaataaaga aagctggtct 424380
ttggtttact tttccgacca cggtttgatg catgtcggta aaggcggcga gcgaacgttg 424440
acacatggtg cgtggaagcg tcaaagctac ggcgtgccgc tggttaaaat ttcgtccgat 424500
gacacgcggc gcgaaatgat taaagtgagg cgcagcgcgt ttaatttttt acgcggattc 424560
ggcagttgga cgggtatcga aaccgacgag ttgcccgatg acggctatga tttttggggg 424620
aatgttcccg atgtgcaggg cgaaggcaat aaccttgcct ttatcgacgg actgcccgac 424680
gaccccgcgc cgtggtatgc gggaaaaggc aaatcgacta aaaatacgtc taaaaaatga 424740
tacgtacaga aaaaatgccg aatgagaatg ggaaaataat ctgtgtttta ccacagcaaa 424800
acaggcgata aaaaaatcag ccgctaccga tgtgtccgcc gcccgaatat taacgaaagt 424860
aaatatgaaa ccactggacc taaatttcat ctgccaagcc ctcaagcttc cgatgccgtc 424920
tgaaagcaaa cccgtgtcgc gcatcgtaac cgacagccgc gacatccgcg cgggcgatgt 424980
gtttttcgca ttggcgggcg agcggtttga cgcgcatgat tttgttgaag acgtattggc 425040
tgctggtgcg gcggcggttg tggtttcgcg cgaagattgt gctgcaatgg atggcgcgtt 425100
gaaagtcgat gacacgcttg ccgcattgca aacgctggca aaggcgtggc gtgaaaatgt 425160
gaatccgttt gtgttcggca ttaccggttc gggcggcaag acgacggtga aggaaatgct 425220
ggctgcggta ttgcgccgcc gtttcggcga tgatgccgtg ttggcgacgg caggcaactt 425280
caacaaccat atcggattgc cgctgacttt gttgaagtta aacgaaaaac accgctatgc 425340
cgtgattgaa atgggcatga accatttcgg cgaactggcg gttttaacgc aaatcgccaa 425400
accaaatgcc gcattggtca acaacgccat gcgcgcccat gtcggctgcg gtttcgacgg 425460
agtgggcgat attgccaaag cgaaaagcga gatttaccaa ggtttatgtt cagacggcat 425520
tgcactgatt cctcaagaag atgccaatat ggctgtcttc aaaacggcaa cgcttaattt 425580
gaatacgcgc actttcggca tcgatagcgg cgatgttcac gcggaaaata ttgtgctgaa 425640
accgttgtcg tgcgaatttg atttggtgtg cggcgatgag cgcgccgccg tggtgctgcc 425700
tgttcccggc cgccacaatg tccacaacgc cgccgctgcc gccgcgctgg ctttggctgc 425760
gggtttgagt ttgaacgatg tggcggaagg tttgaaaggc ttcagcaata tcaaaggccg 425820
tctgaacgtc aaatccggaa tcaagggcgc aaccctgatt gacgatactt ataatgcgaa 425880
ccctgacagc atgaaagctg cgattgacgt gttggcgcgt atgcctgcgc cgcgtatttt 425940

cgtgatgggc gatatgggcg aactgggcga actgggcgag gacgaagccg ccgctatgca 426000
cgccgaagtc ggcgcgtatg cccgcgacca aggcatcgaa gcggcttatt ttgtcggcga 426060
caacagcgtc gaagcggcgg aaaaatttgg cgcggacggt ttgtggttcg ccgccaaaga 426120
cccgttgatt caagtgttgc gccacgattt gcccgaacgc gccaccgtgt tggtgaaagg 426180
ttcgcgcttt atgcagatgg aagaagtggt cgaggcattg gaggataagt gaaaatgaaa 426240
agccgacgtt tttttaaagc cttattgctg attgccgcgc tggtcggcgc gttttatgcc 426300
ggaatgcgga cgcaggcgta tctttatgaa gatttatgtt tagacttggg cggcggtaaa 426360
aatccgggga gttacccaat ttgcgtgatt gagaaagtcc ctgcacgtta atctgcaaaa 426420
gccgtccgaa accttgccgg gcggcaagcc aacctcaaac gggcgcaggc ccgatgtata 426480
gtggattaac aaaaatcagg acaaggcgac gaagccgcag acagtacaaa tagtacggaa 426540
ccgattcact tggtgcttca gcaccttaga gaatcgttct ctttgagcta aggcgaggca 426600
acgccgtact ggtttttgtt aatccactat aacgacaaaa caaaaaaagg aagccccatg 426660
tttttatggc tcgcacattt cagcaactgg ttaaccggtc tgaatatttt tcaatacacc 426720
acattccgcg ccgtcatggc ggcgttgacc gccttagcgt tttccctgat gttcggcccg 426780
tggacgatac gcaggctgac cgcgctcaaa tgcgggcagg cagtgcgtac cgacggtccg 426840
caaacccacc tcgtcaaaaa cggcacgccg acgatgggcg gttcgctgat tctgaccgcc 426900
attaccgtgt ccaccctgtt gtggggcaac tgggcaaacc cgtatatctg gattctcttg 426960
ggcgtattgc tcgccacggg cgcactcggt ttttacgacg actggcgcaa agtcgtctat 427020
aaagacccca acggcgtgtc cgccaaattc aaaatggtgt ggcagtcaag cgttgccatt 427080
atcgccagtt tggcattgtt ttaccttgcc gccaattccg ccaacaatat tttgattgtc 427140
ccgttcttca aacaaatcgc cctgccgctg ggcgtggtcg gctttttggt gttgtcttac 427200
ctgaccatcg tcggcacatc caatgccgtc aacctcaccg acggcttgga cggccttgcg 427260
accttccccg tcgtcctcgt tgccgccggc ctcgccatct tcgcctatgc cagcggccac 427320
tcacaatttg cccaatacct gcaattacct tacgttgccg gcgcaaacga agtggtgatt 427380
ttctgtaccg ccatgtgcgg cgcgtgcctc ggtttcttgt ggtttaacgc ctatcccgcg 427440
caagtcttta tgggcgatgt cggtgcattg gcattgggtg ccgcgctcgg taccgtcgcc 427500
gttatcgtcc gccaagagtt tgtcctcgtc attatgggcg gattatttgt cgtagaagcc 427560
gtatccgtta tgcttcaggt tggctggtat aagaaaacca aaaaacgcat cttcctgatg 427620
gcgcccatcc atcaccacta cgaacaaaaa ggctggaaag aaacccaagt cgtcgtccgc 427680
ttttggatta ttaccatcgt cttggtgttg atcggtttga gtaccctcaa aatccgctga 427740
acctatgccg tctgaacatc tttcagacgg catttgaacg cgcaataaac ctgcggcgac 427800
aatccgccca gccctatcgt taacggtggc tgaaacccgc cttatactaa aacagaagta 427860
aaaccatgaa acagacagtc aaatggcttg ccgccgccct gattgccttg ggcttgaacc 427920
gagcggtgtg ggcggatgac gtatcggatt ttcgggaaaa cttgcaggcg gcagcacagg 427980
gaaatgcagc agcccaatac aatttgggcg caatgtatta caaaggacgc ggcgtgcgcc 428040
gggatgatgc tgaagcggtc agatggtatc ggcaggcggc ggaacagggg ttagcccaag 428100
cccaatacaa tttgggctgg atgtatgcca acgggcgcgg cgtgcgccaa gatgataccg 428160
aagcggtcag atggtatcgg caggcggcag cgcagggggt tgtccaagcc caatacaatt 428220
tgggcgtgat atatgccgaa ggacgtggag tgcgccaaga cgatgtcgaa gcggtcagat 428280
ggtttcggca ggcggcagcg caggggggtag cccaagccca aaacaatttg ggcgtgatgt 428340
atgccgaaag acgcggcgtg cgccaagacc gcgcccttgc acaagaatgg tttggcaagg 428400
cttgtcaaaa cggagaccaa gacggctgcg acaatgacca acgcctgaag gcgggttatt 428460
gaacagctcg cgatgccgtc tgaaagcggc ttgggcaggg gcggacatct cctgctcaat 428520
atgatttgtt ttaggacaaa ccaaaatgac ttttcaaaac aaaaaaatcc tcgtcgccgg 428580
actcggcggt acgggtattt ccatgattgc ctacctgcgc aaaaacggcg cggaggttgc 428640
tgcgtatgat gcggagctga agccggaacg cgtgtcgcaa atcggtaaga tgtttgacgg 428700
gttggtgttt tacacgggcc gtctgaaaga tgcgctggac aacggtttcg atattctggc 428760
tctcagtccc ggcatcagcg agcggcagcc ggatattgag gcgttcaagc aaaacggcgg 428820
acgcgtgttg ggcgacatcg aattgctggc ggacattgtg aaccgccggg acgacaaggt 428880
aattgcgatt accggcagca acggcaaaac cacggtaacg agcctggtcg gctatctctg 428940
tatcaagtgc gggctggata ccgttatcgc gggcaatatc ggcacgccgg ttttggaggc 429000
ggaatggcag cgcgaaggca aaaaggcgga cgtgtgggtg ttggagcttt ccagcttcca 429060
actggaaaac accgaaagcc tgcgtccgac tgcggcgacg gtgctgaaca tttccgaaga 429120
ccatctcgac cgctacgacg acttgctcga ctatgcgcat accaaagcca agattttccg 429180
tggcgacggc gtgcaggttt tgaatgcgga cgatgcgttc tgccgcgcga tgaagcgtgc 429240
cgggcgcgag gtaaaatggt tttcgttgga acacgaagct gatttctggt tggaacgcga 429300
gacaggccgc ctgaaacaag gcaatgaaga tttgattgtc acgcaagaca ttccgttgca 429360
aggtctgcac aacgccgcta acgtcatggc tgccgtggct ttgtgtgagg ccatcggttt 429420
gtcgcgcgaa gcattgctcg aacacgtcaa aaccttccaa ggcctgccgc accgcgtgga 429480
aaaaatcggc gagaaaaacg gcgtggtgtt tatcgacgac agcaaaggca cgaatgtcgg 429540
cgcgactgcc gccgcgattg ccggtttgca aaatccgctc ttcgtgattt tgggcggcat 429600

gggtaaaggg caggacttca cgcccctgcg cgatgcactg gtaggcaagg caaaaggcgt 429660
gttcttgatt ggtgtcgatg cgccgcaaat ccgccgcgat ttggacggct gcggcttgaa 429720
tatgaccgac tgcgccactt tgggagaagc cgttcagacg gcatatgccc aagccgaagc 429780
aggcgatatt gtgttgctca gccccgcctg cgcgagcttt gatatgttca aaggctacgc 429840
gcaccgttcg gaagtgttta tcgaagcgtt taaggctttg tgatgccgtc tgaaatgcaa 429900
acgccgtcat tgttgggcgg caagtaaaga tttagaatac cgatttggga tgtatcgtat 429960
gttcggacgg cattgtctgc cgtctgaaat ttttgccctt tgcggcaggt gcaaacagac 430020
tggcaggtgg ttttttttgaa gatttcggaa gtattggtaa aagtgggcga cggtgtccac 430080
actctgctgc tcgacaggcc gattgtgcgc gacggcagga aattcgacgc gccgcttttg 430140
tggatggtgg tgctgatgac ggcgttcagc ctgctgatga tttattcggc ttctgtgtat 430200
ttggcatcaa aagaaggcgg cgatcagttt ttctatttga ccagacaggc ggggttcgtc 430260
gttgccggct tgatagcgag cggtttgtta tggtttcttt gcaggatgag gacatggcgg 430320
cggcttgtgc cgtggatttt tgccctatcc ggcctgttgc tggtagtcgt attgattgcc 430380
gggcgcgaaa tcaatggcgc gacccgttgg ataccttttgg gtccgttgaa tttccagccg 430440
accgagctgt tcaagctggc ggtcatcctt tatttggcaa gcctgttcac gcgccgtgaa 430500
gaagtgttgc gcagcatgga aagtttgggt tggcagtcga tttggcgggg gacggccaat 430560
ctgatcatgt ccgccaccaa tccgcaggca cgtcgtgaaa cattagaaat gtacggccgt 430620
ttccgggcga tcatcctgcc gattatgctg gtggcgttcg gtttggtgct gataatggta 430680
cagccggatt tcggttcgtt tgtcgtcatt accgtcattg ccgttggaat gctgtttttg 430740
gcaggattgc cgtggaaata ttttttcgtc ctggtaggca gcgtcttggg cgggatggtg 430800
ctgatgatta ccgccgctcc ctaccgtgtg cagcgggtag tggcattttt ggacccgtgg 430860
aaagacccgc agggtgccgg ctaccagctt acccactctc tgatggcaat cgggcgcgga 430920
gagtggttcg gtatgggttt gggtgcgagt ttgagcaaac gcggctttct gccggaagcg 430980
cataccgatt ttatttttgc catcatcgcc gaagaattcg gtttcttcgg tatgtgcgtg 431040
ctgatattct gttacggctg gctggtggtg cgggcgtttt ccatcggcaa gcagtcgcgc 431100
gatttgggtt tgactttcaa cgcctatatc gcttcgggta tcggcatttg gatcggtatc 431160
caaagtttct tcaatatcgg tgtgaacatc ggtgctttgc cgaccaaagg tctgacgctg 431220
ccgttgatgt cctatggcgg ttcgtcagtc tttttcatgc tgatcagcat gatgctgctg 431280
ttgcgtatag attatgaaaa ccgccggaaa atgcgcggtt atcgggtgga gtaaatcatg 431340
ggcggtaaaa cctttatgct gatggcgggc ggaacgggcg gacatatttt ccccgcgctg 431400
gcggtggcgg attcattgcg cgcgcgcggc catcatgtga tttggctggg cagcaaggat 431460
tcgatggaag agcgtatcgt gccgcaatac ggcatacgct tggaaacgct ggcgattaaa 431520
ggcgtgcgcg gcaacggcat caaacgcaaa ctgatgctgc cggttacttt gtatcaaacc 431580
gtccgcgaag cgcagcggat tatccgcaaa caccgtgtcg agtgcgtcat cggcttcggc 431640
ggcttcgtta ccttccccgg cggtttggcg gcgaagctat taggcgtgcc gattgtgatt 431700
cacgagcaaa acgccgtggc aggtttgtcc aaccgccacc tgtcgcgctg ggcgaagcgg 431760
gtgttgtacg cttttccgaa agcgttcagc cacgaaggcg gcttggtcgg caaccccgtc 431820
cgcgccgata ttagcaacct gcccgtgcct gccgaacgct tccaagggcg tgaaggccgt 431880
ctgaaaattt tggtggtcgg cggcagtttg ggcgcggacg ttttgaacaa aaccgtaccg 431940
caggcattgg ctttgctgcc cgacaatgcg cgtccgcaga tgtaccacca atcgggacgg 432000
ggcaagctgg gcagcttgca ggcggattac gacgcgctgg gcgtgaaagc cgaatgcgtg 432060
gaatttatta ccgacatggt gtccgcctac cgcgatgccg atttggtgat ttgccgtgcc 432120
ggcgcgctga cgattgccga gttgacggcg gcgggattgg gtgcgttgtt agtgccgtat 432180
cctcacgcgg ttgacgatca ccaaaccgcc aacgcgcgtt ttatggtgca ggcggaggcg 432240
ggattgctgt tgccgcaaac ccagttgacg gcggaaaaac tcgccgagat tctcggcggc 432300
ttaaaccgcg aaaaatgcct caaatgggca gaaaacgccc gtacgttggc actgccgcac 432360
agtgcggacg acgtggcgga agccgcgatt gcgtgtgcgg cgtaaactgc cgaaccatgc 432420
cgtctgaaaa gccgttcaga cggcatggat gtttttttatt tcaatccgct atatatttgt 432480
cagaaaacta tggcgcgcaa acggtcagcc ctttaaaata acgcctttac gcatcgaaaa 432540
tccaccggaa cgcaacatta tgatgaaaaa tcgagttacc aacatccatt ttgtcggtat 432600
cggcggcgtc ggcatgagcg gcatcgccga agtcttgcac aatttgggct ttaaagtttc 432660
cggttcggat caggcgcgaa atgccgctac cgagcatttg ggcagcctgg gcattcaagt 432720
ttatcccggc cataccgccg aacacgttaa cggtgcggat gtcgtcgtta cctctaccgc 432780
cgtcaaaaaa gaaaatcccg aagttgtcgc tgcgttggag cagcaaattc ccgttattcc 432840
gcgcgccctg atgttggcgg agttgatgcg cttccgtgac ggcatcgcca ttgccggcac 432900
gcacggcaaa accacgacca ccagcctgac cgcctccatc ctcggcgcgg caggacttga 432960
cccgactttc gttatcggcg gcaaactcaa cgccgcaggc actaacgccc gcttgggcaa 433020
aggcgaatac atcgttgccg aagccgacga gtcggatgca tcctttctgc acctgacacc 433080
gattatgtcc gtcgttacca atatcgacga agaccatatg gatacctacg ggcacagcgt 433140
cgaaaaactg catcaggcgt ttatcgattt catccaccgt atgcccttct acggcaaagc 433200
ctttttgtgt attgacagcg aacacgtccg cgcgattttg cccaaagtga gcaaacctta 433260

tgctacttac ggtttggacg ataccgccga catctacgcc accgacatcg aaaacgtcgg 433320
cgcgcaaatg aaattcaccg tccatgttca aatgaaagga catgagcagg ggtcgtttga 433380
agtcgtgctg aatatgcccg gcagacacaa cgtgctgaac gcattggcag ccatcggcgt 433440
ggcgctggaa gtcggcgcat cggttgaagc gatccaaaaa ggcttgctcg gctttgaagg 433500
cgtcggccgc cgcttccaaa aatacggcga catcaagttg ccaaacggcg ggaccgcgct 433560
cttggtggac gactacggac accaccccgt cgaaatggcg gcgacccttg ccgccgcacg 433620
cggcgcgtat ctggaaaaac gtttggtact cgccttccag ccgcaccgct atacccgcac 433680
gcgcgatttg tttgaagact ttaccaaagt cctcaatacc gttgacgcgc tggtgctgac 433740
cgaagtttat gccgccggtg aagagccgat tgccgccgcc gattcccgcg ctcttgcccg 433800
cgccatccgc gtgttgggca aactcgagcc gatttactgc gaaaacgttg ccgatctgcc 433860
cgaaatgctg ttgaacgttt tgcaggacgg cgacatcgtg ttgaatatgg gcgcgggaag 433920
catcaaccgc gtccccgccg cgctgctggc attgtcgaaa cagatttgag gcacacccgc 433980
ctgacagacg gaacatcata taaagatcgt ctgaaaccgc aaatcaggtt tcagacgacc 434040
tctggcaaca agcataaagc aatcaggaaa gaacaaaaac aatgcagaat tttggcaaag 434100
tggccgtatt gatgggcggt ttttccagcg aacgagaaat ctcgctggac agcggcaccg 434160
ccattttgaa tgctttaaaa agcaaaggca tagacgcata cgccttcgat cctaaagaaa 434220
ccccattgtc tgaattgaag gcacaaggtt ttcagacggc attcaacatc cttcacggta 434280
cttacggcga agacggggcg gttcagggtg cattggaact gttgggcatt ccctataccg 434340
gcagcggtgt cgccgcatcc gccatcggca tggacaaata ccgctgcaaa ctgatttggc 434400
aggcattggg attgcccgtt cccgagttcg ccgtcctgca cgacgacact gatttcgatg 434460
ccgtcgaaga aaaattgggc ctgccgatgt ttgtgaaacc ggcggccgaa ggcagcagcg 434520
taggcgtggt aaaagtcaaa ggaaaaggcc gtctgaaaag cgtttacgaa gaattgaaac 434580
accttcaggg cgaaatcatt gccgaacgtt ttatcggcgg cggcgaatat tcctgccccg 434640
tcctgaacgg caaagggctg cccggcatac acatcattcc cgcaaccgag ttttacgact 434700
acgaagccaa gtacaaccgc gacgacacca tttatcaatg tccttcggaa gatttgaccg 434760
aagccgaaga aagcctgatg cgcgaactgg cggttcgcgg cgcgcaggca atcggtgcgg 434820
aaggctgcgt gcgcgtcgat ttcctcaaag ataccgacgg caaactctat ctgttggaaa 434880
tcaacaccct gcccggtatg acgagccata gtttagtacc gaaatccgct gccgttacgg 434940
gcgtgggttt tgccgattta tgtattgaaa ttttgaagac cgcacatgtg ggataatgcc 435000
gaagcgatgg aacggctgac gcgctggctg cttgtcatga tggcgatgct gcttgctgcg 435060
tccgggctgg tttggtttta caattcgaat catctgcccg tcaagcaggt gtcgctgaag 435120
ggcaacctgg tttattccga taagaagaca ttgggcagtt tggcgaaaga atacatccat 435180
gggaatattt tgaggacgga catcaatggc gcacaggagg cctaccgccg gtatccgtgg 435240
attgcgtcgg tcatggtgcg ccgccgtttt cccgacacgg ttgaggtcgt cctgaccgag 435300
cgcaagccgg tcgcgcgttg gggcgaccat gccttggtgg acggcgaagg caatgttttt 435360
gaagcccgct tggacagacc cggaatgccg gtattcagag gcgcggaagg aacgtctgcc 435420
gaaatgctcc gccgttatga cgaattttcg actgttttgg caaaacaggg tttgggcatc 435480
aaagagatga cctatacggc acgttcggcg tggattgtcg ttttggacaa cggcatcacc 435540
gtcaggctcg gacgggaaaa cgagatgaaa cgcctccggc tttttaccga agcgtggcag 435600
catctgttgc gtaaaaataa aaatcggtta tcctatgtgg atatgaggta taaggacgga 435660
ttttcagtcc gctatgcttc cgacggttta cccgaaaaag aatccgaaga atagtgggaa 435720
caggtatcgg acagattacg gccgtgccgt ctgaaacggt gcgacgcaaa tttcaatcag 435780
ttttaagagc agacgaacaa tggaacagca gcaaagatac atcagcgtac tggatatcgg 435840
tacgtctaaa gtcctcgcac tgatcgggga agttcaagat gacgacaaaa tcaacatcgt 435900
cggtttgggg caggctcctt cacggggctt gcgcgcgggc atggtaacca atatcgatgc 435960
caccgtccaa gccatcaggc aggcggtcaa tgatgccgag ctgatggcgg ataccaaaat 436020
tactcacgtt accacaggta tcgcaggcaa ccacatccgc agtctcaatt cgcaaggtgt 436080
ggttaaaatt aaagatgggg aagtcacgca ggcagacatc gatcgcgcca ttgaaacggc 436140
aaaggcaatc aatatcccgc ccgatcaaaa aattctcgat gccgtggttc aagactacat 436200
tattgacacc caacttggcg tgagggagcc catcggtatg agcggtgtgc gtctggatac 436260
gcgggtgcac atcattaccg gtgcaagtac ggcagtgcag aatgtccaaa aatgtatcga 436320
gcggtgcggt ttgaaaagcg atcagatcat gcttcagccg ttggcaagcg ggcaggcggt 436380
gctgactgaa gatgaaaaag acctcggcgt atgcgtcatc gacattggtg gcggaacgac 436440
cgatattgcc gtttatatga acggtgccat ccgccatacg tccgtcattc cggccggtgg 436500
taatctgatt accaaagatt tgtccaaatc gttgagaaca cctctcgatg ccgccgagta 436560
cattaaaatc cattatggcg tggcatcatg cgatacggaa ggcttgggtg agatgattga 436620
agttccgggc gtgggtgacc ggacatcgcg tcaggtttcc agtaaggttc tggcagcaat 436680
catcagtgca cggattcagg agatttttgg cgtagtgctg ggcgagctgc aaaaatcggg 436740
tttccccaaa gaagtgctga atgcgggtat cgttctgacc ggcggtgtgt ccatgatgac 436800
cgggattgtg gaatttgccg aaaaaatctt cgatttgcct gtacgcaccg gtgcacccca 436860
agaaatgggc ggtttgtccg accgcgtccg cacaccgcgt tttctaccg ctatcgggct 436920

gcttcatgca gcatgcaagc tggaaggaaa cttgccgcag ccggaaaacg gtgcagtgca 436980
agagagggaa gggggcggcg gtttgttggc aagattgaaa cggtggattg aaaacagctt 437040
ctgaacaggt ggattgccgt ttgacaggtg agaagtattt tgccagcagc aagatacttc 437100
ttatataatg aataataatt tatttaaacc gtcctctgaa tggggcgagc aggagttttt 437160
gaatggaatt tgtttacgac gtggcagaat cggcagtcag ccctgcggtg attaaagtaa 437220
tcggcttggg cggcggcggt tgcaatgcaa tcaataacat ggttgccaac aatgtgcgcg 437280
gtgtggagtt tatcagtgcc aatacggatg cgcagtctct ggcaaaaaac catgcggcga 437340
agagaatcca gttgggtacg aatctgacac gcggtttggg cgcgggcgcg aatcccgata 437400
tcggccgtgc ggcagcccag gaagaccggg aagccattga agaagccatt cgcggtgcga 437460
atatgctgtt tatcacgacc ggtatgggcg gcggtaccgg taccggttcc gcgccggttg 437520
ttgctgagat tgccaagtct ttgggcattc tgaccgttgc cgtggttacc cgaccgttcg 437580
catatgaagg taagcgcgtc catgtcgcac aggcagggtt ggaacagttg aaagaacacg 437640
tcgattcgct gattatcatc ccgaacgaca aactgatgac tgcattgggt gaagacgtaa 437700
cgatgcgcga agccttccgt gccgccgaca atgtattgcg cgatgcggtc gcaggcattt 437760
ccgaagtggt aacttgcccg agcgaaatca tcaacctcga ctttgccgac gtgaaaaccg 437820
tgatgagcaa ccgcggtatc gctatgatgg gttcgggtta tgcccaaggt atcgaccgtg 437880
cgcgtatggc gaccgaccag gccatttcca gtccgctgct ggacgatgta accttggacg 437940
gagcgcgcgg tgtgctggtc aatattacga ctgctccggg ttgcttgaaa atgtccgagt 438000
tgtccgaagt catgaaaatc gtcaaccaaa gcgcgcatcc cgatttggaa tgcaaattcg 438060
gtgcggctga agacgagacc atgagcgaag atgccatccg gattaccatt atcgctaccg 438120
gtctgaaaga aaaaggcgcg gtcgattttg ttccggcaag ggaggtagaa gcggttgctc 438180
cgtccaaaca ggagcaaagc cacaatgtcg aaggtatgat ccgcaccaat cgcggtatcc 438240
gcacgatgaa ccttaccgct gcggatttcg acaatcagtc cgtacttgac gactttgaaa 438300
tccctgcgat tttgcgtcgt caacacaatt cagacaaata atgtgctgtt tgcccgtaaa 438360
cctgctgcct cccgaatcgg tttgtccggt ttgggaggta tgtttttcaa gatgttgcaa 438420
tttcgtacgg tttgcggtcg gcggattcag atttttccac ttgatacaga ctttcagata 438480
tggacacttc aaaacaaaca ctgttggacg ggatttttaa gctgaaggca aacggtacga 438540
cggtgcgtac cgagttgatg gcgggtttga caactttttt gacgatgtgc tacatcgtta 438600
tcgtcaaccc tctgattttg ggcgagaccg gcatggatat gggggcggta ttcgtcgcta 438660
cctgtatcgc gtctgccatc ggctgttttg ttatgggttt tgtcggcaac tatccgattg 438720
cactcgcacc ggggatgggg ctgaatgcct atttcacctt tgccgtcgtt aagggtatgg 438780
gcgtgccttg gcaggttgcg ttgggtgcgg tgttcatctc cggtctgatt tttatcctgt 438840
tcagcttttt taaagtcagg gaaatgctgg tcaacgcact gcctatgggt ttgaaaatgt 438900
cgattgctgc cggtatcggt ttgtttttgg cactgatttc cctgaaaggc gcaggcatta 438960
tcgttgccaa tccggcaacc ttggtcggtt tgggcgatat tcatcagccg tccgcgttgt 439020
tggcattgtt cggttttgct atggtggtcg tattgggaca tttccgcgtt caaggcgcaa 439080
tcatcatcac catcttgacc attaccgtca ttgccagcct gatgggtttg aatgaatttc 439140
acggcatcat cggcgaagta ccgagcattg cgccgacttt tatgcagatg gattttgaag 439200
gcctgtttac cgtcagcatg gtcagtgtga ttttcgtctt cttcttggtc gatctatttg 439260
acagtaccgg aacgctggtc ggcatatccc accgtgccgg gctgctggtg gacggtaagc 439320
tgccccgcct gaaacgcgca ctgcttgcag actctaccgc cattgtggca ggtgcggctt 439380
tgggtacttc ttccaccacg ccttatgtgg aaagcgcggc gggcgtatcg gcaggcggac 439440
ggaccggcct gacggcggtt accgtcggcg tattgatgct cgcctgcctg atgttttcac 439500
ctttggcgaa aagtgttccc gcttttgcca ccgcgcccgc cctgctttat gtcggcacgc 439560
agatgctccg cagtgcgagg gatattgatt gggacgatat gacggaagcc gcacctgcgt 439620
tcctgaccat tgttttcatg ccgtttactt attcgattgc agacggcatc gctttcggct 439680
tcatcagtta tgccgtggtt aaacttttat gccgccgcac caaagacgtt ccgcctatgg 439740
tatggattgt tgccgtattg tgggcactga aattctggta tttgggctga ttgattcgat 439800
attaaaaatg ccgtctgaaa ggttttcaga cggcattttg tttgccgata tatttaattt 439860
ttattaaatt atataaaaat caaatacata ataaaataca tcggattgct taaaaataat 439920
acattgtttt tatgtataaa atattttata agttttcagg attttgatta tcaaaaattt 439980
ttcttgattt cctgacaatt ttattgaaac aaataattca aaattaatct agtttaatca 440040
tggaattaaa ataaaatatt aaaattatgt aatgagtctc cttaaaaatg tttgacattt 440100
tcagtcttgt gttttagatt atcgaaaaat aaaactacat aacactacaa aggaacatta 440160
ctatgaaacc aattcagatg ttttcccctt ttctgaataa tccccttgtt ttcttcttgt 440220
ctgcggtttt gccgcataat tccgaacggt ctgctgtttt tctttgattc gttttaaata 440280
tcaataagat aatttttccc atatattttt aatgattgga ttgggatgcc cgacgcgtcg 440340
gatggctgtg ttttgccgtc cgaatgtgat ggaagcctgt ccatactgaa aaaaagtcta 440400
taaaggagaa atatgatgag tcaacactct gccggagcac gtttccgcca agccgtgaaa 440460
gaatcgaatc cgcttgccgt cgccggttgc gtcaatgctt attttgcacg attggccacc 440520
caaagcggtt tcaaagccat ctatctgtcc ggcggcggcg tggcagcctg ttcttgcggt 440580

atccctgatt tgggcattac cacaatggaa gatgtgctga tcgacgcacg acgcattacg 440640
gacaacgtgg atacgcctct gctggtggac atcgatgtgg gttggggcgg tgcattcaat 440700
attgcccgta ccattcgcaa ctttgaacgc gccggtgttg cagcggttca catcgaagat 440760
caggtagcgc aaaaacgctg cggccaccgt ccgaacaaag ccattgtatc taaagatgaa 440820
atggtcgacc gtatcaaagc tgccgtagat gcgcgcgttg atgagaactt cgtgattatg 440880
gcgcgtaccg atgcgctggc ggtagaaggt ttggatgccg ctatcgaacg cgcccaagct 440940
tgtgtcgaag ccggtgcgga catgattttc cctgaagcca tgaccgattt gaacatgtac 441000
cgccaatttg cagatgcggt gaaagtgccc gtgttggcga acattaccga gtttggttcc 441060
actccgcttt atacccaaag cgagctggct gaaaacggcg tgtcgctggt gctgtatccg 441120
ctgtcatcgt tccgtgcagc aagcaaagcc gctctgaatg tttacgaagc gattatgcgc 441180
gatggcactc aggcggcggt ggtggacagt atgcaaaccc gtgccgagct gtacgagcat 441240
ctgaactatc atgccttcga gcaaaaactg gataaattgt ttcaaaaatg atttaccgct 441300
ttcagactgc ctttcaacaa atccgcatcg gtcgtctgaa aacccgaaac ccataaaaac 441360
acaaaggaga aataccatga ctgaaactac tcaaaccccg accctcaaac ctaaaaaatc 441420
cgttgcgctt tctggcgttg cggccggtaa taccgctttg tgtaccgttg gccgtaccgg 441480
caacgatttg agctatcgcg gttacgacat tctggatttg gcacaaaaat gcgagtttga 441540
agaagtcgcc cacctgctga ttcacggcca tctgcccaac aaattcgagc tggccgctta 441600
taaaaccaag ctcaaatcca tgcgcggcct gcctatccgt gtgattaaag ttttggaaag 441660
cctgcctgca catacccatc cgatggacgt aatgcgtacc ggcgtatcca tgctgggctg 441720
cgttcatcct gaacgtgaaa gccatccgga aagtgaagcg cgcgacatcg ccgacaaact 441780
gatcgccagc ctcggcagca tcctcttgta ctggtatcaa tattcgcaca acggcaaacg 441840
cattgaggtt gaaagcgacg aagagaccat cggcggtcat ttcctgcaac tgttgcacgg 441900
caaacgccca agcgaatcac acatcaaagc catgcacgtt tcactgattc tgtatgccga 441960
acacgagttc aacgcttcta cctttaccgc ccgcgtgatc gccggtacag gctctgatat 442020
gtactccagc attaccggag caatcggcgc gttgaaaggt ccgaaacacg gcggcgcgaa 442080
cgaagtggct tacgatattc aaaaacgcta ccgcaatgcc gacgaagctg aagccgacat 442140
ccgcgaacgc atcggccgca aagaaatcgt gatcggtttc ggtcatccgg tgtacaccat 442200
ttccgaccct cgcaacgttg tcattaaaga agtggcacgc ggtttgagca aagaaaccgg 442260
cgatatgcgc ctctttgaca ttgccgaacg tttggaaagc gtgatgtggg aagagaaaaa 442320
aatgttcccg aatctggact ggttctctgc cgtttcctac caaaaattgg gcgtaccgac 442380
cgctatgttc acaccgctgt tcgtaatttc ccgtacaacc ggttggagcg cacacgttct 442440
tgagcaacgc aaagacggca aaatcatccg tccgagcgca aactacacag gccctgaaga 442500
tttggcgttt gtggagattg aagaacgata attgaagaat gcaatagcag tttgttcttt 442560
aatttcggta tgcaaagcta aggatttcag acgaccttgc cttattggaa aggttgtctg 442620
aaataagttt aatctaatag gagaagataa tcctgtattg gcgcaagtaa caggataaga 442680
aacatggaag atttatatat aatactcgct ttgggtttgg ttgcgatgat tgccggattt 442740
atcgatgcga ttgcgggcgg gggtggtttg attacgctgc ccgcactctt gttggcaggt 442800
attcctcccg tgtcggcaat tgccaccaac aagctgcaag cagccgctgc tacgttttca 442860
gctacggttt cttttgcacg caaaggtttg attgattgga agaaaggtct cccgattgcc 442920
gcagcatcgt ttgtaggcgg cgtggccggt gcattatcgg tcagcttggt ttccaaagat 442980
attctgctgg cggtcgtgcc ggttttgttg atatttgtcg cactgtattt tgtgttttcg 443040
cccaagctcg acggcagtaa ggaaggcaaa gccagaatgt cttttttttct gttcgggctg 443100
acggtcgcac cgcttttggg tttttacgac ggtgtgttcg gaccgggtgt cggctcgttt 443160
tttctgattg cctttattgt tttgctcggc tgcaagctgt tgaacgcgat gtcttacacc 443220
aaattggcga acgttgcctg caatcttggt tcgctatcgg tattcctgct gcacggttcg 443280
attattttcc cgattgcggc aacgatggcg gtcggtgcgt ttgtcggtgc gaatttaggt 443340
gcgagatttg ccgtccgctt cggttcgaag ctgattaagc cgctgctgat tgtcatcagc 443400
atttcgatgg ctgtgaaatt gttgatagac gagagaaatc cgctgtatca gatgattgtt 443460
tcgatgtttt aaacccttttc agacgacccc ttcaaaacgt cggctgaaac ctcaaaccac 443520
aagaaaaaca gatccacagg agaaccgaca tggctgccaa ccaacgttac cgcaaaccgc 443580
tgcccggtac ggatttggaa tactacgacg cgcgtgcggc gtgtgaggac atcaagcccg 443640
gctcttacga caagctgcct tacacgagcc gcattttggc ggagaatttg gtcaaccgcg 443700
cggacaaagt cgatttgccg acgctgcaaa gctggctggg gcagttgata gaagggaagc 443760
aggaaatcga ctttccgtgg tatccggcgc gggtggtgtg ccacgatatt ctggggcaga 443820
ccgcgttggt ggatttggca ggcctgcgcg atgcgattgc cgaaaaaggc ggcgatcctg 443880
ccaaagtgaa tccggtggtg caaacccagc tcatcgtcga ccactctctg gcggtggagt 443940
gcggcggtta cgatcctgat gccttccgca aaaaccgcga aatcgaagac cgccgtaacg 444000
aagaccgttt ccacttcatc aactggacaa aaaccgcgtt tgaaaatgtg gacgtgattc 444060
cggcgggcaa cggcatcatg caccaaatca atctagaaaa aatgtcgccc gtcgtccaag 444120
tcaaaaacgg cgtggctttc cccgatacct gcgtcggtac tgactcacat acgccgcacg 444180
tcgattcatt gggcgtgatt tccgtgggcg tgggcggatt ggaagcggaa accgtaatgc 444240

tgggacgcgc gtccatgatg cgcctgcccg atattgtcgg cgttgagctg aacggcaaac 444300
ggcaggcggg cattacggcg acggatattg tgttggcact gaccgagttt ctgcgcaaag 444360
aacgcgtggt cggggcgttt gtcgaattct tcggcgaggg cgcgagaagc ctgtctatcg 444420
gcgaccgcgc gaccatttcc aacatgacgc cggagttcgg cgcgactgcc gcgatgttcg 444480
ctattgatga gcaaaccatt gattatttga aactgaccgg acgcgacgac gcgcaggtga 444540
aattggtgga aacctacgcc aaaaccgcag gcttgtgggc agatgccttg aaaaccgccg 444600
tttatcctcg cgttttgaaa tttgatttga gcagcgtaac gcgcaatatg gcaggcccaa 444660
gtaacccgca tgcccgtttt gcgaccgccg atttggcggc gaaagggctg gcgaagcctt 444720
acgaagagcc ttcggacggc caaatgcccg acggctcggt catcatcgcc gcgattacca 444780
gttgcaccaa cacttccaac ccgcgcaacg ttgttgccgc cgcgctcttg gcacgcaatg 444840
ccaaccgtct cggcttgaaa cgcaaacctt gggtgaaatc ttcgtttgcc ccgggttcaa 444900
aagtagccga aatctatttg aaagaagcgg gcctgttgcc cgaaatggaa aaactcggct 444960
tcggtatcgt cgccttcgcc tgcaccacct gcaacggcat gagtggcgcg ctggatccga 445020
aaatccagaa agaaatcatc gaccgcgatt tgtacgccac cgccgtatta tcaggcaacc 445080
gcaacttcga cggccgtatc cacccgtatg cgaaacaggc tttcctcgct tcgcctccgt 445140
tggtcgttgc ctacgcgctg gcaggcagta tccgtttcga tattgaaaac gacgtactcg 445200
gcgttgcaga cggcaaggaa atccgcctga aagacatttg gcctgccgat gaagaaatcg 445260
atgccgtcgt tgccgaatat gtgaaaccgc agcagttccg cgatgtgtat gtaccgatgt 445320
tcgacaccgg cacagcgcaa aaagcaccca gtccgctgta cgattggcgt ccgatgtcca 445380
cctacatccg ccgtccgcct tactgggaag gcgcgctggc aggggaacgc acattaagag 445440
gtatgcgtcc gctggcgatt ttgcccgaca acatcaccac cgaccacctc tcgccgtcca 445500
atgcgatttt ggccgtcagt gccgcaggcg agtatttggc gaaaatgggt ttgcctgaag 445560
aagacttcaa ctcttacgca acccaccgcg gcgaccactt gaccgcccaa cgcgctacct 445620
tcgccaatcc gaaactgttt aacgaaatgg tgaaaaacga agacggcagc gtgcgccaag 445680
gctcgttcgc ccgcgtcgaa cccgaaggcg aaaccatgcg catgtgggaa gccatcgaaa 445740
cctatatgaa ccgcaaacag ccgctcatca tcattgccgg tgcggactat ggtcaaggct 445800
caagccgcga ctgggctgca aaaggcgtac gcctcgccgg cgtagaagcg attgttgccg 445860
aaggcttcga gcgtatccac cgcaccaacc ttatcggcat gggcgtgttg ccgctgcagt 445920
tcaaacccga caccaaccgc cataccctgc aactggacgg tacggaaacc tacgacgtgg 445980
tcggcgaacg cacaccgcgc tgcgacctga ccctcgtgat tcaccgtaaa aacggcgaaa 446040
ccgttgaagt tcccgttacc tgctgcctcg atactgcaga agaagtattg gtatatgaag 446100
ccggcggcgt gttgcaacgg tttgcacagg attttttgga agggaacgcg gcttagaggt 446160
cgtctgaaaa gcaagacgta gcgtgggtcg ggttcaacat tttgctcatt cacgtaattc 446220
tcgatatggc aggcatctac tgtaaatcgt cattcccgcg caggcgggaa tccagaaagt 446280
ggaattgagg aaaccttatt tatccgatga gtttctgtgc ggacaaattt ggattcccgc 446340
ctgcgcggga atgacggggt ttaataatct gccgtatcac aacacagtag ccgtagattg 446400
tggcgaaccc cgacagtttg cggaatcaaa cggctttgtc ggagtggcag cctaatgtac 446460
ttctggaaag tgggtgtagc gtgggctttg cccgcgaaat aaaggctgaa ttgacatggt 446520
atagaggatt aacaaaaatc gggacaaggc ggcgaagccg cagacagtac agatagtacg 446580
gaaccgattc acttggtgct tgagcacctt agagaatcgt tctctttgag ctaaggcgag 446640
gcaacgctgt actggttttt gttaatccac tataaattta atccactata ctgtaaatcg 446700
tcattcccgc gcaggcggga atccagaaag tggaattgag gaaacctttt tatccgatga 446760
gtttctgtgc ggataaatct ggattcccgc ctgcgcggga atgacggggt ttaataatct 446820
gccgtatcac aacacagtag ccgtagattg ggcgaaccc cgacagtttg cggaatcaaa 446880
cggctttggt cggagtggca gcctaatcca ctataaaaat cgtgggcaga gcccacgcta 446940
cataaggaga atctagaaat gccgcaaatt aaaattcccg ccgtttacta ccgtggcggt 447000
acatcaaaag gcgtgttttt caaacgttcc gacctgcccg aggcggcgcg ggaagcggga 447060
agcgcacgcg acaaaatcct cttgcgcgta ctcggcagcc cggatcccta cggcaagcag 447120
atagacggtt tgggcaacgc cagctcgtcc accagcaagg cggtgatttt ggacaagtcc 447180
gaacgcgccg atcacgatgt cgattacctt ttcgggcaag tttccatcga caaacctttt 447240
gtcgattgga gcggcaactg cggcaacctc accgctgccg tgggcgcatt ctccatcgaa 447300
cagggcttgg tcgataaagg caagattcct tcagacggca tctgcacggt caaaatctgg 447360
cagaaaaaca tcggcaaaac cattattgcc catgtaccga tgcaaaacgg cgcagttttg 447420
gaaacaggcg attttgagct cgacggcgta acgttcccgg cagccgaagt acaaatcgaa 447480
tttcttgatc cagccgacgg cgaaggcagt atgttcccaa ccggcaattt ggtcgatgaa 447540
attgatgtgc cgaatatagg ccgtttgaaa gccacgctca tcaacgcggg cattccgacc 447600
gttttcttga atgccgccga cttgggctac acaggcaaag agttgcaaga cgacatcaac 447660
aacgatgccg cggctttgga aaaattcgag aaaatccgcg cttacggtgc gctgaaaatg 447720
ggtctgatca gcgacgtatc cgaagctgcc gctcgcgcgc acacgccgaa agtcgccttc 447780
gtcgcgcccg ccgccgatta caccgcctcc agtggcaaaa ccgtgaacgc cgccgacatc 447840
gatttgctgg tacgcgccct gagcatgggc aaactgcacc acgcgatgat gggtaccgcc 447900

```
tctgttgcca ttgcgaccgc cgccgccgta cccggtacgc tggtcaacct tgccgcaggc 447960
ggcggaacgc gtaaagaagt gcgcttcggg catccttccg gcacattgcg cgtcggtgca 448020
gccgccgaat gtcaggacgg acaatggacg gccaccaaag cggtcatgag ccgtagcgca 448080
cgcgtgatga tggaaggttg ggtcagggtg cctgaggatt gtttttaaat tgacgtagca 448140
tgggtttgcc cgcgagccat aaaaaggtcg tctgaaaaac aagtaaacat caaatcactg 448200
accattcctt tcccttgccc tgtggcggaa ggcggcaaat cacaaggaag aacacggaaa 448260
ccccgataaa agacagcttc ccgtattacc gtcattcccg cgcaggcggg aatccagacc 448320
tgtcaatatg gaggattggc aggggaaaac aggtttcgtg agttctacat tctggattcc 448380
cgccacagcc tgtcctcgcg taggcgggga cggaataacg atagaaaatg cggcatacgc 448440
tttgcccaaa gaggccgtct gaaacacctt gcgcctgatg tctgcctttt tcagacgacc 448500
ccacaccaaa aaaacaacca caaactacaa ggagaaacat catgtccgac caactcatcc 448560
tcgttctgaa ctgcggcagt tcatcgctca aggcgccgt tatcgaccga aaaagcggca 448620
gcgtcgtcct aagctgcctc ggcgaacgcc tgaccacgcc cgaagccgtc attacgttca 448680
acaaagacgg caacaaacgc caagttcccc tgagcggccg aaattgccac gccggcgcgg 448740
tgggtatgct tttgaacgaa ctggaaaaac acggtctgca cgaccgcatc aaagccatcg 448800
gccaccgcat cgcccacggc ggcgaaaaat acagcgagtc tgttttgatc gaccaggccg 448860
taatggacga actcaatgcc tgcattccgc ttgcgccgct gcacaacccc gccaacatca 448920
gcggcatcct tgccgcacag gaacatttcc ccggtctgcc caatgtcggc gtgatggata 448980
cttcgttcca ccaaaccatg ccggagcgtg cctacactta tgccgtgccg cgcgagttgc 449040
gtaaaaaata cgctttccgc cgctacggtt tccacggcac cagtatgcgt tacgttgccc 449100
ctgaagccgc acgcatcttg ggcaaacctc tggaagacat ccgcatgatt attgcccact 449160
taggcaacgg cgcatccatt accgccatca aaaacggcaa atccgtcgat accagtatgg 449220
gtttcacgcc gatcgaaggt ttggtaatgg gtacacgttg cggcgacatc gatccgggcg 449280
tatacagcta tctgacttcc cacgccggga tggatgttgc ccaagtggat gaaatgctga 449340
acaaaaaatc aggtttgctc ggtatttccg aactttccaa cgactgccgc accctcgaaa 449400
tcgccgccga cgaaggccac gaaggcgcgc gcctcgccct cgaagtcatg acctaccgcc 449460
tcgccaaata catcgcttcg atggctgtgg gctgcggcgg cgttgacgca ctcgtgttca 449520
ccggcggtat cggcgaaaac tcgcgtaata tccgtgccaa aaccgtttcc tatcttgatt 449580
tcttgggtct gcacatcgac accaaagcca atatggaaaa acgctacggc aattcgggca 449640
ttatcagccc gaccgattct tctccggctg ttttggttgt cccgaccaat gaagaactga 449700
tgattgcctg cgacactgcc gaacttgccg gcatcttgta gccaaaaaag ggacgagtcc 449760
gcaaaaatgc cgtctgaaac cccaaacgcc cgattaggct gatgaggatt ttagacggca 449820
ttgttcattt ttttgttatc ttgcattttt gtgcggacgg tggaatttca tcctgtaaac 449880
ataaatattt gtcggaaaac agaaaccctc cgccgccatt tctacgaaag caggaaacca 449940
gcaacgcaaa gcgacaggga tttgttggaa atgaccgaaa ccgaacgaac cggattcccg 450000
cctgcgcggg aatgacggga ttttctgttt ttgtggaaat gacgggattt tgaatttcgg 450060
gcgtacaata cggaaaacat gacgataagg aaacaaacca tggcacagtt tttcgctatt 450120
catcccgaca atccccaaga acgcctcatc aagcaggcgg ttgaaatcgt caataaaggc 450180
ggcgtggtcg tttatccgac cgattcctgt tatgccttgg gctgcaaact cggcgataag 450240
gcggcgatgg aacgcatact ctccatccgc aaaatcgatt tgaaacacca cctgaccctg 450300
atgtgcgcag atttgagcga gttgggcaca tacgccaaag tcgacaacgt acagtttcgt 450360
cagcttaaag ccgccacacc cgggccttat acttttattt tacaggcgac gaaggatgtg 450420
ccggcgcgca cgctgcaccc gaaacgcaaa accatcggc tgcgtattcc cgataatgcc 450480
attgcacaag ccctgctggg ggaattgggc gagccgcttt taagctgcac cctgatgctg 450540
cccgaagacg gcgaaccatt gaccgatcct tatgaaatcc gcgagcgttt ggaacacgcc 450600
gtcgatttgg tgattgacgg cggctggtgc ggaaccgagc cgaccaccgt cgtcgatatg 450660
accgacggca cggaattggt gcgccaaggt tgcggcgata cggcggtgtt cggtttgtag 450720
ggaaaccgat gccgtctgaa gcatcggctg ttcagacggc attgcgcgcc ttgccggcgg 450780
cagtccgaaa tgccggcgcg tatcgcgctc ggtcggaata tccgtttgaa acggcatttt 450840
gatgcattac tgcaccgcaa tcggaattct cggttcgtag agcaggtcgt aggtcggctt 450900
gttgagcagg tcttggagcg tgaaaccgtc cagatacgtg aaaaacgact tcatcgcgcc 450960
gccgagtatg cccgtcagcc ggcaggacgg tgtaatcagg cattcgttgt tctcgcccat 451020
gcactcgacc agctgcatcg gttcgaggtg gcggacaacc gagccgatgt tgatgcggtc 451080
gggcggtgcg gcaagccgca gaccgccgcc ttttccgcgc acactgtgga ggaagccgcc 451140
tttgaccagc gcggtaacga ccttcatcag atggcttttg gaaatgccgt aggttacggc 451200
gatggtactg atgttgacca gcgcatcgtc gttgatggca gtgtagataa ggacgcgcag 451260
cccgtagtcc gtatgttgtg tcaaatacat gatttctcg gtatggattg ttattcttat 451320
cggtacggtt taaggttcac ggacaatacc ttaatggttg aaaccctgtc cgtcggggcg 451380
gtagaatgca gcctgtctgc ggcggtatgc cgtctgaaac atccgcgcta ccgtttgaga 451440
atttgttatt gtaactcaaa atcatgaaac cgttgaaacg acatcccgcc cttatcgggc 451500
tttcgcgtga ccaccaccat tcgctttccc tgtgcgtgcg tctgttgcgg acgccggaag 451560
```

aaaggcatcg ggacgaactc gaaccgcatt tttccgaatt ggaaacccat tttcgcgaag 451620
aagaaaccaa gtttgcccca atttggcaga atgtcgcccc cgaattgaaa caacgtttcg 451680
agaaagacca cgcccgactg cggcagatga tggcaagccc cgaatacggt aacgcggcgt 451740
ggaataccgc ttttgccaca accctgcgcg accacgcgcg ctttgaagaa cgcgagctgt 451800
ttcccgccgc cgaaccgttt ttgccggcat gattccgttt tgcggtaaat atattaatga 451860
taaacaagga acacacatga aatttaccaa gcaccccgtc tgggcaatgg cgttccgccc 451920
attttattcg ctggcggctc tgtacggcgc attgtccgta ttgctgtggg gtttcggcta 451980
cacgggaacg cacgagctgt ccggtttcta ttggcacgcg catgagatga tttggggtta 452040
tgccggactg gtcgtcatcg ccttcctgct gaccgccgtc gccacttgga cggggcagcc 452100
gcccacgcgg ggcggcgttc tggtcggctt gactatcttt tggctggctg cgcggattgc 452160
cgcctttatc ccgggttggg gtgcgtcggc aagcggcata ctcggtacgc tgtttttctg 452220
gtacggcgcg gtgtgcatgg ctttgcccgt tatccgttcg cagaatcaac gcaactatgt 452280
tgccgtgttc gcgctgttcg tcttgggcgg cacgcatgcg gcgttccacg tccagctgca 452340
caacggcaac ctaggcggac tcttgagcgg attgcagtcg ggcttggtga tggtgtcggg 452400
ttttatcggt ctgattggta cgcggattat ttcgtttttt acgtccaaac gcttgaatgt 452460
gccgcagatt cccagtccga aatgggtggc gcaggcttcg ctgtggctgc ccatgctgac 452520
tgccatgctg atggcgcacg gtgtgttggc ttggctgtct gccgtttttg cctttgcggc 452580
aggtgtgatt tttaccgtgc aggtgtaccg ctggtggtat aaacccgtgt tgaaagagcc 452640
gatgctgtgg attctgtttg ccggctatct gtttaccgga ttggggctga ttgcggtcgg 452700
cgcgtcttat ttcaaacccg ctttcctcaa tctgggtgtg catctgatcg ggtcggcggg 452760
tatcggcgtg ctgactttgg gcatgatggc gcgtaccgcg cttggtcata cgggcaatcc 452820
gatttatccg ccgcccaaag ccgttcccgt tgcgttttgg ctgatgatgg cggcaaccgc 452880
cgtccgtatg gttgccgtat ttcttccgg cactgcctac acgcacagca tccgcacctc 452940
ttcggttttg tttgcactcg cgcttttggt gtatgcgtgg aagtatattc cttggctgat 453000
tcgtccgcgt tcggacggca ggcccggttg agacaaaccg ccgcagattt cgggtctggg 453060
cggtttgctt ttcagacggc agggcggtca gttgccgtcc agccagcggt cgcgtgtggt 453120
tttggcttct tcaaaatagc ggtacagggc ttcgcggtcg tcggtggtca ggatgtttgc 453180
caaaacgtcc aactgtttgc ccaagccttg aaccagttgc agcaggctgt ctttgttggc 453240
aaggcagatg tccgcccaca cggcgggatg accggaggcg atgcgggtga agtcccgaaa 453300
gcccgtggcg gcgaatttca gatattcctg tccgtcgggg tggtcgagaa tctggtggac 453360
ataggcgaag gcggtcaggt ggggcatatg ggagacggcg gcgaaaaccg cgtcgtggcg 453420
ttgcgcgtcc atcgtataaa tttccgcacc gaccgcgtgc cacaggtttt ctaccaaggc 453480
aatgccgtct gaatgttcgc cgccgtgtgg cgtgatgatg agtttctgt ggcggaacag 453540
cccgaactgc gcggcttgcg caccgcttct gtccgaaccg gcaattgggt gggcggcgat 453600
gcagtggtgc aggcggtcgg gcagacagcg gcggaaggct tcgatgaccg aagatttggt 453660
gctgccgaca tcggaaatcc aagtgtgttc cggcaaaacg gggcgcagcg cggtcaaaat 453720
ggcgggaacg gtggcgacgg gcgtggcaat cagtaccaag tccgcaccgc cgatgctgtc 453780
cgcgtcgatg gcaacggaag cctggtcaat cacgccgcgt tccaatgcac gttcgaggtt 453840
gtcgcggtcg gtgtcgatac cggtaacggt gcggacgagt ccctgccttt tgaggtcgag 453900
aacgaacgaa ccgccgatca gccctacacc gatgagggca atatggttca aaatgggcat 453960
ttgtgtaaac ggttttcgca aagtaccgtc atggtagcct atcggcggaa tatgccgcaa 454020
ggtcggcagg aaaaaggaga agaaatggac aaaatcagag ttgccgccgt gcagatggtg 454080
tcgggcgtgt cgccggaaac caacgtcgcc gccatgaaac gcctggtcgc acgggcggcg 454140
gagcagggtg cggattgggt gctgctgccc gaatattggg tgctgatggg cgcaaacgat 454200
accgacaaac tcgcgcttgc cgagcctttg ggcggcggac gctttcagac ggcattgagc 454260
gaaacggcga aagaatgcgg cgtggtgctg ttcggcggga ctgtgccgct gcaaagctgc 454320
gaggcgggta aagtgatgaa tacgctgttg gtgtacggac gggacggcgt aaggacgggg 454380
ctgtaccaca aaatgcacct cttcggtttt tccggtttgg gcgaacgcta tgccgaagcc 454440
gataccatcc gcgcgggcgg ggatgtgccg cacttgtcgg cagaaggcgt gccggtggcg 454500
gcgggcattt gttacgatgt ccgctttccc gaatttttcc gacgccagtt gccgtttgac 454560
gtattgatgc tgcccgctgc gtttacgcac acgacgggca aggcgcattg ggagctgctg 454620
ctgcgcgcgc gtgccgtcga aaaccaatgt tacgtcgtgg cggcggcaca gggcggtttg 454680
cacgaaaacg gacggcgcac gttcggacac agcatgattg tcgatccgtg ggcgacgtg 454740
ttggacgtat tgcccgaggg cgaaggcgtt gttacggcag acatcgatgc caaccgcctg 454800
aacagcgtcc gcaaccgcct gcccgccttg aaataccggg ttttggatgc cgtctgaagg 454860
ttcagacggc atcggtgccg gggaatcaga agcggtagcg catgcccaat gagacttcgt 454920
gggttttgaa gcgggtgttt tccaagcgtc cccagttgtg gtaacggtat ccggtgtcca 454980
aggtcagctt gggcgtgatg tcgaaaccga caccggcgat gacaccaaga cccacgctgc 455040
tgatgctgtg gctttcgtga tagggaggtt tgctgggatc agtttgtata atagggcctc 455100
cctgtggaga gccgttcttt ggtttagagg taatagtcgt ggtttttgtt tccaccgaat 455160
gaacttgatg tttaacgtgt ccgtaggcga cgcgcgcgcc gatatagggt ttgaatttat 455220

cgttgagttt gaaatcgtaa atggcggaca agccgagaga agaaacggcg tggaagctgc 455280
cgtttccctg atgttttgtt tgggtttctt tgtagttgtt gtttatctct tcagtaactt 455340
ttttagtaga agaattactt tctttccatt ttctgtaact ggcataatct gccgctattc 455400
tccagccgcc gaaatcatag ccgaccgaca cccgggggtg gatggaatgc gcacggatgt 455460
ttctgaaata atcgcttacc gtgcttgtgt tgtttgcacc ggttgcttgc ggataatcgt 455520
gggtaatgcg ttcggcggca taagctaaat ccgcctgcac ataatacggg ctgcggctgc 455580
cgtcttcact tgccgcctgc gctgcggaag agaagagaag agaagagaag agaagagaag 455640
agaagagaag agaagagaag gttttttggg ggctggattc attttcgact ccgtattcgg 455700
ttttaactga ttaaaaagaa agattttcac tgatgttgca ggggtggatt gtatcgggtt 455760
tggggcgatg tttcaacaca atatagcgga tgaacaaaaa agagaacgat gctctaaggt 455820
gcccaagcac caagtgaatc ggttccgtac tatagtggat taacaaaaac cagtacagcg 455880
ttgcctcgcc ttagctcaaa gagaacgatt ctctaaggtg ctgaagcacc gagtgaatcg 455940
gttccgtact atttgtactg tctgcggctt cgtcgccttg tcctgatttt tgttaatccg 456000
ctataaagac cgtcgggcat ctgcagccgt cattcccgcg caggcgggaa tctagacctt 456060
agaacaacag caatattcaa agattatctg aaagtctgag attctagatt cccacgaaag 456120
tgggaatcca ggatgtaaaa tctcaagaaa ccgttttatc cgataagttc ctgcactgac 456180
agacctagat tcccgcctgc gcgggaatga cgggatttta ggtttctgat tttggttttc 456240
tgtccttgtg ggaatgacgg gatgtaggtt cgtaggaatg acgtggtgca ggtttccgtg 456300
cggatggatt cgtcattccc gcgcaggcgg gaatctagac cttagaacaa cagcaatatt 456360
caaagattgg cggattcgca tttgaagtgc aactttccct aacagaaaaa ggccagtatg 456420
cggtagcata cggcctttcc tgcaagaaag attgccatga gctacacgca actgacccaa 456480
ggcgaacgat accacatcca atacctgtcc cgccactgca ccgtcaccga aatcgccaaa 456540
cagctgaacc gccacaaaag caccatcagc cgcgaaatca gacggcaccg cacccaaggg 456600
cagcaataca gcgccgaaaa agcccagcgg caaagccaga ctatcaaaca gcgtaagcga 456660
caaccctata agctcgattc gcagctgatt cagcacatcg accccccttat ccgccgcaaa 456720
ctcagtcccg aacaagtatg cgcctacctg cgcaaacacc accagatcac gctccaccac 456780
agcaccattt accgctacct tcgccaagac aaaagcaacg gcagcacgtt gtggcaacat 456840
ctcagaatat gcagcaaacc ctaccgcaaa cgctacggca gcacatggac cagaggcaaa 456900
gtacccaacc gtgtcggcat agaaaaccga cccgctatcg tcgaccagaa atcccgtatc 456960
ggcgattggg aagccgacac cattgtcggc aaaggacaga aaagcgcatt attgaccttg 457020
gtcgaacgcg ttacccgcta caccatcatc tgcaaattgg atagcctcaa agccgaagac 457080
actgcccggg cagctgttag ggcattaaag gcacataaag acagggtgca caccattacc 457140
atggataacg gcaaagagtt ctaccaacac accaaaataa ccaaagcatt gaaagcggag 457200
acttattttt gtcgtcctta ccattcttgg gagaaagggc tgaatgagaa caccaacgga 457260
ctcatccggc aatacttccc caaacaaacc gatttccgta acatcagtga tcgggagata 457320
cgcagggttc aagatgagtt gaaccaccga ccaagaaaaa cacttggcta cgaaacgcca 457380
agtgttttat tcttgaatct gttccaacca ctaatacact agtgttgcac ttgaaatccg 457440
aatccaagat tatctgaaag tctgagattc tagattccca ctttcgtggg aatgacggga 457500
ttttaggttt ctgattttgg ttttctgtcc ttgtgggaat gacgggatgt aggttcgtag 457560
gaatgacgtg gtgcaggttt ccgtgcggat ggattcgtca ttcccgcgca ggcgggaatt 457620
tggaatttca atgcctcaag aatttatcgg aaaaaaccaa aacccttccg ccgtcattcc 457680
cacgaaagtg ggaatctaga aatgaaaagc agcaggcatt tatcggaaat gaccgaaact 457740
gaacggactg gattcccgct tttgcgggaa tgacggcgac aggggttgctg ttatagtgga 457800
tgaacaaaaa ccagtacggc gttgcctcgc cttagctcaa agagaacgat tctctaaggt 457860
gctgaagcac caagtgaatc ggttctgtac tatttgtact gtctgcggct tcgtcgcctt 457920
gtcctgattt ttgttcatcc gctatacttt tgtatgacca tctgacttta tcactcacta 457980
tgttttacca aatccttgcc ctgattatct ggagcagctc gtttattgcc gccaaatatg 458040
tctatggcgg catcgatccc gcattgatgg tcggcgtgcg cctgctaatt gccgcgctgc 458100
ctgcactgcc cgcctgccgc cgtcatgtcg gcaagattcc gcgtgaggaa tggaagccgt 458160
tgctgattgt gtcgttcgtc aactatgtgc tgaccctgct gcttcagttt gtcgggttga 458220
aatacacttc cgccgccagc gcatcggtca ttgtcggact cgagccgctg ctgatggtgt 458280
ttgtcggaca ctttttcttc aacgacaaag cgcgtgccta ccactggata tgcggcgcgg 458340
cggcatttgc cggtgtcgcg ctgctgatgg cgggcggtgc ggaagagggc ggcgaagtcg 458400
gctggttcgg ctgcctgctg gtgttgttgg cgggcgcggg cttttgtgcc gctatgcgtc 458460
cgacgcaaag gctgattgca cgcatcggcg caccggcatt cacatctgtt tccattgccg 458520
ccgcatcgtt gatgtgcctg ccgttttcgc ttgctttggc gcaaagttat accgtggact 458580
ggagcgtcgg gatggtattg tcgctgctgt atttgggttt ggggtgcggc tggtacgcct 458640
attggctgtg gaacaagggg atgagccgtg ttcctgccaa tgtttcggga ctgttgattt 458700
cgctcgaacc cgtcgtcggc gtgctgctgg cggttttgat tttgggcgaa cacctgtcgc 458760
ccgtgtccgc cttgggcgtg tttgtcgtca tcgccgccac cttggttgcc ggccggctgt 458820
cgcatcaaaa ataaagttgg gaagcggtat ttgatgattg ccgaataggc tgaaatcttt 458880

ccatctccat tcctgcgaaa gcgggtatcc ggaacgaaaa gacggatatt tatccgaaat 458940
aacgaccatc tttgcgtcgt cattcccgcg caggcgggca tccggttttt tgagtttcgg 459000
ttatttccga caaattgctg cagcgttgga tgtccggatt tccgcctgcg cgggaatgac 459060
gggattttat agtggattaa caaaaatcag gacaaggcgg cgagccgcag acagtacaga 459120
tagtacggaa ccgattcact tggtgcttca gcaccttaga gaatcgttct ctttgagcta 459180
aggcaaggca acgctgtact ggtttttgtt aatccactat atcgttccgg ttcgtccggt 459240
tttgccgggg cttttgttgc cgcctgtttg tgccggtgtg ttaaaatttt ccgtttccgc 459300
gtattgtgtt ttccgccgcc gggcggtttg tttgcgaatc ggacgagaat ttatgccttc 459360
tgcccattat cctgaaatga gcgaaaaact gatggcggtt ttgatggcga tgctggttac 459420
gctgatgccg ttttccatcg atgcctacct gcccgcgatt cccgaaatgg cgcaatcgct 459480
gaacgcggat gttcaccgca tcgaacagag tttgagtttg tttatgttcg gcacggcgtt 459540
cggacaggtg gtcggcggtt cggtgtccga catcaaaggg cgcaaacccg tcgccctgac 459600
cggtttgatt gtatattgcc ttgccgttgc cgccatcgta tttgtttcga gtgccgaaca 459660
gctcctcaac ctgcgcgtcg tgcaggcatt cggtgcgggc atgactgtgg tcatcgtcgg 459720
cgcaatggtg cgcgattatt attccggacg caaagccgcc cagatgtttg cccttatcgg 459780
catcattttg atggttgtgc cgctggtcgc acccatggtc ggcgcattgt tgcagggctt 459840
gggtggctgg caggcgattt ttgtttttct ggcggcgtat tcgctggtgc tgctcggttt 459900
ggtacagtat ttcctgccca agcccgccgt cggcggcaaa atcggacggg acgtgttcgg 459960
gctggtggcg gggcggttca agcgcgtatt gaaaacccgt gctgcgatgg gttatctgtt 460020
ttttcaggca ttcagcttcg gttcgatgtt cgcctttctg accgaatctt ccttcgtgta 460080
ccagcagctc taccgtgtta cgcctcatca atacgcttgg gcgtttgcac tcaacatcat 460140
cacgatgatg tttttcaacc gcgttaccgc gtggcggctc aaaaccggcg tgcatccgca 460200
aagcatcctg ctgtggggga ttgtcgtcca gtttgccgcc aacctgtccc aactcgccgc 460260
cgtgctgttt ttcgggttgc ccccgttttg gctgctggtc gcgtgcgtga tgttttccgt 460320
cggtacgcag ggcttggtcg gtgcaaacac gcaggcgtgt tttatgtcct atttcaaaga 460380
agagggcggc agcgcaaacg ccgtattggg tgtattccaa tctttaatcg gcgcgggggt 460440
gggtatggcg gcgaccttct tgcacgacgg ttcggcaacc gtgatggcgg caacgatgac 460500
cgcgtccacc tcttgcggca ttgcgcttct gtggctctgc tcgcatcgtg cgtggaaaga 460560
aaacgggcaa agcgaatacc tttaacggaa aatgccgtct gaaaccgttt cagacggcat 460620
ttgatgttag aatgcacgat aaattactgt tcaggcgaaa ttatgtccca aactatcgac 460680
gaactcctcc ttccccaccg caacgccatc gacaccatcg atgccgaaat cctgcgcctg 460740
ctcaacgaac gtgcgcaaca cgcccacgcc atcggcgagc tgaaaggcac gggcgcagtg 460800
taccgccccg aacgcgaagt cgccgtgttg cgccgcattc aggatttgaa caaaggcccg 460860
ctgcccgacg aatcggtagc acgcctgttt cgggaagtga tgagcgagtg cctcgccgtc 460920
gaacgcccgc tgaccatcgc ctatctgggg ccgcagggca cgtttaccca gcaggcggca 460980
atcaaacatt tcggacacgc cgcgcacacc atggcgtgtc cgaccataga cgactgcttc 461040
aagcaggttg aaacgcgtca ggcggattat ctggtcgccc ccgtggaaaa ttcgaccgaa 461100
ggctcggtcg gtcgcacgtt agacctgctt gccgttaccg cgttgcaggc gtgcggcgaa 461160
atcgttttgc gcatccacca caacctttg cgtaaaaaca acggcagcac cgaaggcatt 461220
gccaaagtct ttcccacgc gcaggcgttg gcgcagtgca acgactggtt gggcagacac 461280
ctgcccaacg ccgaacggat tgccgtgtcc agcaatgccg aagccgcaag gctggttgcc 461340
gaatcggacg acggtacggt tgccgccatc gccggacgca cggcggcgga aatctacgga 461400
ctcgatatgg ttgccgagtg catcgaagac gaaccgaaca acaccacgcg cttcttggtg 461460
atgggacatc acgaaaccgg tgcaagcggc agcgacaaga cttcgctggc cgtttccgcg 461520
cccaaccggg caggcgcggt tgcctcgctg ctgcaaccgc tgaccgaatc gggtatttcc 461580
atgaccaagt ttgagagccg tccgagcaaa tccgttttgt gggaatacct gttcttcatc 461640
gacatcgaag gacaccgccg ggacgcgcag attcagacgg cattggaacg cttgggcgaa 461700
cgcgcttcgt tcgtcaaagt catcggttcg tacccgaccg ccgttttgta gcggcggcag 461760
cgttcagacg gcatttcccc aacgattatg tccgaatacc gagtcaacca tgaacccgtt 461820
tttatgctgg catcttcgcc ctggcgcgaa agcagcctgt gggttgaagc attcagccgc 461880
cgttacgggc gtgtggcttt gctggcgcgc agcgcgcgca aaaggcagag cgagctgcgc 461940
ggcgtattgg tgccgttcgt gcccgtcagc gtgtcgtggt acggcagtca ggaactcaaa 462000
accctacacc gcgccgaatg ggtcggcggt tggcggcagc ctcagggcag ggcgttgttc 462060
ggcggattgt atgtgaacga gttggtgttg aaactgaccg cccgcgaaga cccggtgccc 462120
gagttatacg acgcgttggc ggaagtgatg gaggcggtgt gctgcaaagc cgcttatatc 462180
gacgacttgc gccgtttcga gtggcggctg ctgaacctgt tgggcgttgc cccgatttg 462240
aaccgcgacg gggacggcgg gacgattgcg gcaggcggca cataccttgt ccgcccggaa 462300
acagccgtct tccccgtcgg aaaaggattt gccgtaccgc cgcacgccgc cggcgttgtc 462360
gcccccgggc agagcctgat cgatttgcgc gaaggcagtt tccgcactgc cgaaagcctg 462420
caacaggcat tgaaaatcac acggcttttt atccgccacc tgttgcccga ggggctgaaa 462480
tcgcggcagg tgttggaaca gatacggcag tttgaccgca aagaaaccgc ccgggaaacc 462540

gtcccgactt cggacggcac ggcttcaaat gccgtctgaa ggcagaaata aaaggaaaga 462600
ttatgctttt aggtgtcaac atcgaccaca tcgccaccgt ccgcaatgcg cgcggtacga 462660
cttatcccag ccccgtggag gcggcactgg ttgccgaaac gcacggtgcg gatttgatta 462720
ccatgcacct gcgcgaagac cgccgccaca tcaaagacgc ggacgtgttt gccgtcaaaa 462780
acgccatccg cacgcgcctg aaccttgaaa tggcgttgac ggaagaaatg ttggaaaacg 462840
ctttgaaagt gatgccggaa gacgtgtgca tcgtgcctga aaaacgtcag gaaatcacga 462900
ccgaaggcgg tttggacgta ttggcgcaac aggaaaaaat cgccgggttc accaaaatcc 462960
tgaccgacgc aggcatacgc gtgtctttgt ttatcgatgc cgacgacagg caaatccaag 463020
ccgcccgtga tgtcggcgcg cccgttgtcg agctgcacac aggcgcgtat gccgacgcgc 463080
gcagccacgc cgaacaaatc aggcagttcg agcgcatcca aaacggcgcg catttcgccg 463140
gcgatttggg cttggtcgtc aacgccggac acggactgac catacacaac gttacccca 463200
tcgcccaaat cctcgccatc cgcgaactga acatcgggca ttcgctgatt gcccaagccc 463260
tcttcctcgg actgcccgaa gccgtgcgcc aaatgaagga ggcgatgttc agggcaaggc 463320
tgctgccgta agggcaggca aaccctttca gacagcattt cacgacaggg atatgttata 463380
gtggattaaa tttaaatcag gacaaggcgg cgaagccgca gacagtacaa atagtacggc 463440
aaggcaagcc aacgccgtac tggtttaaat ttaattcact atatgaatca aaagtatatt 463500
ttatctgcaa acaataatag tttgatagaa gaaattcaca atacagtaca gagtattggg 463560
tattgtattg ttcgaggtct taatctaaac catcttgatg gcagccggag aaacaagaaa 463620
ttatttgact ttctatctca attaggaatg ctgacaaacc acaaaggcga tggttttaaa 463680
tctatatttt gggatattaa atattgaggc gatgattatg taatatagtg gattaacaaa 463740
aatcaggaca aggcgacgaa gctgcagaca gtacagatag tacggaaccg attcacttgg 463800
tgcttcagca ccttagagaa tcgttctctt tgagctaagg cgaggcaacg ccgtactggt 463860
ttttgttaat ccactataaa taatgatata actttctcgg aagatgttgg agaatgtcca 463920
cttcatagtg attcatcttt tagtgaaaac ccggaaagtt atttggttat gtatgtagta 463980
aaatcagcca atgatggagg taattcccta tttttaagtt catcagatat tgtcaatcag 464040
ttatctaaaa cagaaaccgg taaaaaacac ttaaaaacat taacgggcaa tttatatcca 464100
tttaaaacac cagcatcatt tgataaaaaa caaggtgtga gatggggtaa tatcttatcg 464160
gtcaatactc aaatgattag atttagaagt gattgtatct ataaaggtat tgaagaaaat 464220
agaaataaag tatcaaagga aatggtactt gcacttgatt atcttataaa tgttataaaa 464280
aatgcgagtg atattcaaga attttctgca caagatgatg gtttgattat tattgacaat 464340
gtcaatggct tgcatgccag aactgattat acggataaaa acaggcatta tattagagca 464400
agaattactg tataaaggac ggttatgcaa gaaataatgc aatctatcgt ttttgttgct 464460
gccgcaatac tgcacggaat tacaggcatg ggatttccga tgctcggtac aaccgcattg 464520
gcttttatca tgccattgtc taaggttgtt gccttggtgg cattaccaag cctgttaatg 464580
agcttgttgg ttctatgcag caataacaaa aagggttttt ggcaagagat tgtttattat 464640
ttaaaaacct ataaattgct tgctatcggc agcgtcgttg gcagcatttt gggggtgaag 464700
ttgcttttga tacttccagt gtcttggctg cttttactga tggcaatcat tacattgtat 464760
tattctgtca atggtatttt aaatgtatgt gcaaaagcaa aaaatattca agtagttgcc 464820
aataataaga atatggttct ttttgggttt ttggcaggca tcatcggcgg ttcaaccaat 464880
gccatgtctc ccatattgtt aatatttttg cttagcgaaa cagaaaataa aaatcgtatc 464940
gtaaaatcaa gcaatctatg ctatcttttg gcgaaaattg ttcaaatata tatgctaaga 465000
gaccagtatt ggttattaaa taagagtgaa tacggtttaa tatttttact gtccgtattg 465060
tctgttattg gattgtatgt tggaattcgg ttaaggacta agattagccc aaattttttt 465120
aaaatgttaa tttttattgt tttattggta ttggctctga aaatcgggca ttcgggttta 465180
atcaaacttt aattcattat taaatgcctt aactccttat taaataattg gcacgatgtt 465240
ttagaatttc aaatgcaaaa ggttacagtg aaaattgtta ccgacaaaac cccaaaagtg 465300
gatattcacg ccattttaac gccccaagaa attgacggca ttcatcatca cattcatcac 465360
tacccgcaac caagggcgaa ggagcgcaaa tatgatttac ggcatcggca cagacattgt 465420
ttccctcaag cgcatcatcc gcttaaacaa aaaattcgga caggcgtttg ccgggcgcat 465480
cctcactccg gaagagctgc ttgaatttcc gcaagcgggc aaacccgtca actacctcgc 465540
caaacgcttt gccgccaaag aagcctttgc caaagccgtc ggcacgggca tacgcggcgc 465600
ggtttccttc cgcaacatcg gcatcgggca tgacgcattg ggcaagcccg aatttttcta 465660
cggccccgcc ctgtccaaat ggctggagga acaaggcatc agccgcgtca gcctcagcat 465720
gagcgacgaa gaagacaccg tattggcgtt tgtcgttgcc gaaaaataat gccgtctgaa 465780
atgcggcaaa cccgttgacg gcattgcccg tccctcattt gcactccgac cgaccaaccg 465840
cgtacccgcc atgattcaag acacccgacc ccttatccgc gtcgttgccg gcatcctgct 465900
cgattcagac ggcaactacc tgctcagctc gcgccccgaa ggcaaaccct atgccggata 465960
ttgggaattt gccggcggca aggtcgaagc gggcgaaacc gacttccaag ccctgcaacg 466020
cgagtttgaa gaagaactcg gcatccgcat cctcgccgcc acgccttggt tgaccaaaat 466080
ccattcctac gaacacgccc gcgtctgcct gaaattccta tgggtcaacc ccgaccaatg 466140
gacgggcaaa ccgcaatccc gcgaagggca ggaatggtct tggcagaagg cgggtgattt 466200

taccgttgcc cccatgctgc ccgccaacgg cgcgcttttg cgttcgctgt ccgtcccgcg 466260
ccgtttgtac ggcagcctga aaacgggttt gcacggagaa aacagtatgg gcgcgtaccg 466320
cgtcctgcct ttgggttcgg cagagggaag cggtgcgaac gttttgatgg aggcggcgca 466380
atggcaggac agacccgaac acgccgacag cgtgtggatg gtggtgcaga cccgcgaaca 466440
atggcggcgg gcgcaggaaa agggcgcgga tgcggtcgtt tggcgcgtgt gcgatgatgt 466500
tcaggcacaa gaggcggcag aagccctgcg gcagggcgta tccgtgccgc tcgtacttgc 466560
agcaaacgga cagacggttg cacgttatgg aaaactatgg ctcggattgg gggcgcacgt 466620
ggtggtaagg gatgaaacaa tagggaagaa tcatgaataa aaaccgtaaa ttactgcttg 466680
ccgcactgct gctgattgcc tttgccgccg tcaagctcgt tttgttgcaa tggtggcagg 466740
cgcagcagcc gcaagctgtg gcggcgcaat gcgatttgac cgagggttgc acgctgccgg 466800
acggaagccg cgtccgcgcc gccgccgttt caaccaaaaa accgtttgat atttatatcg 466860
aacacgcgcc cgccggcacg gaacaggtca gcatcagctt cagtatgaaa aatatggata 466920
tgggtttcaa ccgctatatg ttcgagcggc aaccgtcggg gacttggcag gcagtacgca 466980
tccgcctgcc catctgtgtc gaaggcaggc gcgattttac ggcggacatt acaatcggca 467040
gtcggacatt tcagacggca tttaccgccg aataaacctt tcaatccgcc attgccggaa 467100
catccgtccg gaaaggacac gttatgaata ctttatatac acttttcgcc acctgcccgc 467160
gcggcttgga gaccgtttta tctcaagaac tcgaaagcct cggctgtacc gatgtacaag 467220
tgtttgacgg cggcgtttcc tgccggggcg gattggaaca ggtttacgcc gccaacctgc 467280
attcgcgtac tgccagccgt atcctgctgc gcctgaccaa agggacatac cgcaatgagc 467340
gcgacatcta caaactcgcc aaaaatatca actggtttaa ttggtttact ttacagcaga 467400
cgttcaaagt caaagtcgag gcaaagcgtg ccaacgttaa gagcatccaa tttgtcggac 467460
tgaccgtcaa agatgccgtc tgcgacgctt tccgcgacat ttacgacgca cgtccgagcg 467520
tggacaaagc cgcgcccgat gtccgcatcc acgccttttt gaacgaacgc aatgtcgaaa 467580
tctttattga cacttcgggc gaagccctgt tcaaacgcgg ctaccgcctg gataccggcg 467640
aagccccgct gcgcgaaaac cttgccgccg gactgctgct ctcggcaggc tacgacggca 467700
cgcagccgtt tcaagacccg ttttgcggca gcggcacgat tgctatcgaa gccgcttgga 467760
ttgccgcccg ccgcgcgccg ggtatgatgc gccgtttcgg ttttgaaaaa ctgcaaaatt 467820
tcgataaaac gctgtggtcg gatttgcggc gccgcgccga agcgcaaacc cgccccgtcc 467880
gcgccccgat tgcaggcagc gacaacgacc gccgcatcgt tcagacggca ttggacaacg 467940
cacgccgcgc cggggtggac gacatcgttt ccttcagcgt tgccgacgcg cagtccgtcc 468000
gaccgaacgg cgaaaacggc attatggtgt ccaatccgcc ctacggcgtg cgccttgagg 468060
aagtccgcgc cttgcaggca ctgtatccgc agttggggac gtggttgaaa aaacattacg 468120
caggctggtt ggcggcaatg tttaccggcg atagggaaat gcccaaattc atgtgcctgt 468180
cgcccaagcg gaaaatcccg ctttataacg gcaacatcga ctgccgcctg ttcctgattg 468240
atatggtgga aggatcgaac cgttgaggaa agtgtacaaa aatgccgtct gaaaaatgtt 468300
cagacggcat ttatttttcg gaatcaaccc cgcttcaata cggatgtatt gatgtagcgt 468360
tggacacccg aggcaatgga ttgggcgcac tgccggcgga aggattcgct gcccagcagc 468420
ttctcttcgg caggattgga caggaaggcg gtttcgacca ggatagacgg catatcggt 468480
gcgcgcaaaa cggcgaaatt ggcttcgtcc accctgcctt tgtgcagatg gttgagcctg 468540
cccaattctt caagcaccag tttgccgagt ttgcggctgt cgcgcagcgt ggcggtttgg 468600
gtcatgtcga gcagggcggt atcgacattg cggttgccgc tggtcggtac gccgccgacc 468660
gcgtcggcat tgttttgcgt ctgttccaag aatttggcgg cagagctggt tgcgcctttg 468720
gtgtttaaca tataaacccc cgtgccgcgc gcggaggggc tggtgaaggc atcggcgtgg 468780
atggagacaa atacgtccgc ccgccgtgct cgccctttgg cgacacgcac gcccaatggg 468840
atgaacacgt cttcgttgcg cgtcataaat acattgtaac ctaatgcttc caactgattt 468900
ttggtttccc tggcaatgga taggacgaca tgtttttcct gtagaccgcc cgggctgatg 468960
gcgccggggt cttcaccgcc gtgtcccgga tcgagcatga tgacgggtct gcgcccgttt 469020
ctgccgcgcc cgggttgggg cgtggtgttt tgggcgaggt cggcttcggg agagccgcgc 469080
agggttttat tcaggctacc gttgagcagt gccatcatcg gatcgtcggc atccatcccg 469140
tgcggataga ggtcgacgac gaggcggttc ttaaagccgc cgacgggcgg aagcgcgaag 469200
acttgtgcgt gggtgggctg tttcaaatcg atgacgaggc ggacggtggt cggcgtgttc 469260
tgacccgcgc gtatgctgcg gataaagggg tcgtctgcca tgactttctg agacagtccg 469320
tgcaatacgg tattgatgtt cgcgttttgt atgtcgacga ccagcctgcc cgggttgtcg 469380
agcgtgaagt gctggtattt gagcgcggcg gtgctttcca gcgtcaggcg ggtgtaggtg 469440
tgcgacggcc atatccgtgc ggcggtgaat tgcggggcgc gtaccgtttt ggcaacggcg 469500
gatgcgatgg ggcttagggc gaacagtgtg ccggcggtgc ggcggatgat ttgtcttcgt 469560
gtcagtttga tcatagcggc aggctttcgc gtcctcgttc ggtatgggcg gtcagcaggc 469620
attttctgcc gtcgccgtcg tgtgtcaatg ttgcggtgat gtcggcgggc ggcgtaaatt 469680
ccccgccctg ttgcggccat tcgatcaggc agacgctgtt tgcggcaaac agttcgtcaa 469740
gccccgcgtc ttcccattct tcggggaacg agaagcggta gaggtcgaaa tggtgcaggg 469800
tgaagcgttc cagcggataa gattcgacga tggcgtaggt cggacttttg actgcgccct 469860

gatgacccaa tccgcgcagg atgccgcgtg tcagcgtggt tttgcccgca cccaaatccc 469920
cttcgagata aatgaccagc ggtgcgttta aacgggaaga ccacgccgcg cccaaatcga 469980
gtgtggcggc ttcgtcggca aggaatcggg agatagaggg taaatcagac atggaaacgg 470040
tttgttgtaa ggtctagggt attatgggca gttttgcagg ttttgcaaac tttgcacccg 470100
aggggcggat gcttcttgtc cgagcattat aacagccaaa tccgcgttct gctttcagac 470160
ggcaacggct gtcaagaaaa agcggcgcgt gtacaatacg cggattgtat gtttaggacg 470220
gattggaaaa agaatggaaa atatcggcag gcagcgaccc atcggcgttt ttgactcggg 470280
aatcggcggt ttgaccaatg tgcgagcgct gatggaacgg ctgccgatgg agaacatcat 470340
ttatttcggc gacacggcgc gcgtgcctta cgggacgaaa tctaaggcga ccatcgaaaa 470400
tttctcgatg cagattgtcg atttttttatt ggaacacgat gtcaaggcga tggttatcgc 470460
gtgcaatacg attgcggcgg tggcggggca gaaaatccgt caaaaaaccg gcaatatgcc 470520
cgttttggac gtgatttccg ccggcgcgaa agccgcgctg gcaacgacgc gcaacaataa 470580
aatcggcatt atcgccacca atacgacagt caacagcaat gcttatgcgc gcgccatcca 470640
taggaacaac cccgacacgc tcgtccgcac gcaggccgcg ccgctgctcg tccctttggt 470700
ggaagagggc tggctggaac acgaagttac ccgcctgacc gtatgcgaat acctcaaacc 470760
attgcttgca gacggcatcg atacgctggt gttgggctgc acgcactttc ccttgctcaa 470820
gcccttaatc ggcagggagg cgggcaatgt cgcgttggtt gattctgcaa ttacaacggc 470880
cgaagaaacc gcacgcgtcc ttgctcagga aggattgctc aataccgaca acaacaatcc 470940
cgactaccgt ttttacgtca gcgatattcc tttgaaattc agaaccatcg gcgagcgttt 471000
tctgggcagg acgatggagc agattgaaat ggtgtctttg ggttaaaacg atgacggaaa 471060
gctgcccgag attacagaaa cctaaaatcc cgtcattccc acgaaagtgg gaatctagac 471120
ctgtcggtgc ggaaacttat cggataaaac ggtttcttta gattttacgt tctagattcc 471180
cactttcgtg ggaatgacgg gattagagtt tcaaaattta ttctaaatag ctgaagctca 471240
acgcactgga ttcccgcctg cgcgggaatg acgaatttca ggtttctgtt tttggttttc 471300
tgtttttgtg aaaataacgg gatttcagct tgtgggtatt taccggaaaa aacagaaacc 471360
gctccgccgt cattcccgcg caggcgggaa tctagacatt caatgctaag gcaatttatc 471420
gggaatgact gaaactcaaa aaactagatt cccactttcg tgggaatgac ggaatgtagg 471480
ttcgtgggaa tgacgggatg caggtttccg tatggatgga ttcgtcattc ccgagcagac 471540
gggatctaga cattcaatgc taaggcaatt tatcgggaat gactgaaact caaaaaacta 471600
gattcccact ttcgtgggaa tgacgggata taggtttcca tgcggacgcg ttcggattca 471660
cgactgcgcg gaaatgacgg gattttggtg tattccctaa aaaaataaaa aaacatttgc 471720
aactttgtta aaaataaagg ctgtgtttta acgatgtgtt gatatttaat tttagaaagg 471780
tagctattta atagttacct tttcttattt aaaaatagct ttctcaaatt ccatgaacgc 471840
ctcaatacga tatgcagatg ctctatcgaa attaagtttc aacattttgt ttattaaaca 471900
ttttatttta gccattttc aatataccccc caaatatacc cccaatttgc acaagtcaaa 471960
attttaggca ggggttttgt tgctcccatg atacgcgggc ggatgccggc ttttcgcggg 472020
ggaaatacaa ggggtctcgg ttcgggtgtc aaaatccctg tttcgtgtta gtcatgtggg 472080
ggggaagagg gggttagaat gaagtaaagc tgttgccctc tccccgcaat agttccatta 472140
ggcgcggatg aatgaatagt ttgtccctgc cgatgacgat ttcttgcagc acacctatgt 472200
ctgaaagctc tttcaggtac ttagaggccg tctgccgttt ggctatccct gccgcttcta 472260
ggttggcaat gcgtgtatat ggctgctcaa acagaagatt taccagttcg tgcgtgtaga 472320
ttccttgtgc gtgtgtccgt atgtgttgcc gtgtctgctc gaacaggcgg cgtatcgcat 472380
ctattttcga taccgtccaa tcggcggtgt cagctacgcc gtctaagatg tagattatcc 472440
agctttccca gtcctgccgt tcggttacgc ctaaaagcag gcggtaatag tccgccctgt 472500
tttcgatgat gtagcggctc aaatacaaaa taggcaaatc caaaagccct ttttcaatca 472560
atagcaggct gttcaatatg cgccccgtcc gcccgttgcc gtccgtaaac ggatggatgg 472620
cttcaaattg gtaatgtgcc gccgccatga tgataagcgg gtctaaatcg ccgctttcgt 472680
gaataaaccg ctcccaattt gccagcttgc cgcgtatggt ttcttctcct tcgggcgggg 472740
tatagacaac atttccgctg ttgcctcctt ttagggctgt gccgcctgtt ttgcggatgg 472800
ccatttcgta ggggtgcttg atggcgttgc agaccatgat ggcggtttgt gtgcataaag 472860
ggcggctcgt cagtgattca tagcctgcaa acagggcggt gcggtattgc agggcttctt 472920
tcgtggcagg gtcttgccgt tccgtatcca tttgcaggga ttgaaacagc ttgtccgtgg 472980
tggttacgat gttttcaatt tccgaacttg cacgggcttc cataacagga agggtgttaa 473040
tcagcatggc ttgattcggt atcaattctg ccgcctgctt taaacggcca agggatgcac 473100
gggcggctat acaacgtttc aggatggttt tgctttcaat atcctgtttt ggcggcaggg 473160
gtggtaaatc gttataggga atattgggtt tccagttgct catatttaaa atttcggaaa 473220
atttaaagat gtttccagta tatgtttacg ccgtgtatat atcaaggata tatgtttaaa 473280
aatttggctt ttgtaagtat atgggaggta aaaccgcccg caaaagtcaa tttgcatggg 473340
gttaattcaa aatccgcccg cccttcatct gcccgcttcc ctaaaaaaac cgtatatata 473400
taactgtccg cattctatcg ctccggcgac gatacccata tttccaagtt tgtgtatcaa 473460
aattgtatat cgggcataga ctatttcggc gaggacgaag atatagattt ccacgattga 473520

atacatggaa gccaagtacg tctatcaaca ctatattaaa acacagcctt ttttttgag 473580
gtttcgggta acttttaaac cgtcattcct acgaaaacag aaaatcaaaa acagaaatct 473640
caaatcccgt cattcccgcg caggcgggaa tctagacatt caatgctaag gcaatttctc 473700
ggaaatgact gaaactcaaa aaactggatt cccactttcg tgggaatgac ggaatgtagg 473760
ttcgtgggaa tgacgtggtg caggtttccg tatggatgga ttcgtcattc ccgcgcaggc 473820
gggaatctag acattcaatg ctaaggcaat ttatcgggaa tgactgaaac tcaaaaaact 473880
ggattcccac tttcgtggga atgacgcgat tagagtttca aaatttattc taaatagctg 473940
aaactcaacg cactggattc ccgcctgcgc gggaatgacg aagtggaagt tacccgaaac 474000
ttaaaacaag cgaaaccgaa cgaactggat tcccactttc gtgggaatga cggaatgcag 474060
gttcgtggga atgacggaat gcaggttcgt gggaatgacg tagtgcaggt ttccgtatgg 474120
atggattcgt cattcccgcg caggcgggaa tctagacatg caatgctaag gcaatttatc 474180
gggaatgact gaaactcaaa aaactggatt cccgcctgcg cgggaatgac gaagtggaag 474240
ttacccgaaa cttaaaacaa gcgaaaccga acgaactgga ttcccacttt cgtgagaatg 474300
acgggatgca ggttcgtggg aatgacgtgg tgcaggtttc cgtatggatg gattcgtcat 474360
tcccgcgcag gcgggaatct aggtctgtcg gtgcggaaac ttatcgggta aaacggtttc 474420
ttgagatttt gcgtcttgga ttcccacttt cgtgggaatg acgcgattag agtttcaaaa 474480
tttattctaa atagctgaaa ctcaacgcac tggattccgc ctgcgcggga atgacgaagt 474540
ggaagttacc cgaaacttaa aacaagcgaa accgaacgaa ccggattccc actttcgtgg 474600
gaatgacgaa tttcaggtta ctgtttttgg ttttctgttt ttgtgaaaat aatgggattt 474660
cagcttgtgg gtatttaccg gaaaaaacag aaaccgctcc gccgtcattc ccgcgcaggc 474720
gggaatctag gtctgtcggt gcggaaactt atcggataaa acggtttctt gagatttttc 474780
gtcctggatt cccactttcg tgggaatgac gcgaacagaa accgctccgc cgtcattccc 474840
gcgcaggcgg gaatctagac attcaatgct aaggcaattt atcgggaatg actgaaactc 474900
aaaaaactgg attcccactt tcgtgggaat gacgtggtgc aggtttccgt atggatggat 474960
tcgtcattcc cgcgcaggcg ggaatctaga ccttcaatac taaggcaatt tatcggaaat 475020
gactgaaact cgaaaaactg gattcccact tttgtgggaa tgacgcgatt agagtttcaa 475080
aatttattct aaatagctga aactcaacac actggattcc cgcctgcgcg ggaatgacga 475140
agtggaagtt acccgaaact taaaacaagc gaaaccgaac gaactggatt cccactttcg 475200
tgggaatgac ggaatgtagg ttcgtgggaa tgacggcgga gcggtttctg ctttttccaa 475260
taaatgaccc caacttaaaa tcccgtcatt cccgcgcagg cgggaatcta ggtctgtcgg 475320
tgcggaaact tatcgggtaa aacggtttct tgagattttg cgtcctggat tcccactttc 475380
gtgggaatga cggaatgtag gttcgtggga atgacgggat ataggtttcc gtgcggacgc 475440
gttcggattc atgactgcgc gggaatgacg ggattttggt gtattcccta aaaaaataaa 475500
aaagtatttg caaatttgtt aaaaataaat aaaataataa tccttatcat tctttaattg 475560
aattggattt attatgaaca atccattggt gaatcaggct gctatggtgc tgcctgtgtt 475620
tttgttgagt gcttgtttgg gcggaggcgg cagtttcgat cttgattctg tcgataccga 475680
agccccgcgt cccgcgccaa aatatcaaga tgttttttcc gaaaaaccgc aagcccaaaa 475740
agaccaaggc ggatacggtt ttgcaatgag gttgaaacgg aggaattggt atccgcaggc 475800
aaaagaagac gaggttaaac tggacgagag tgattgggag gcgacaggat tgccggacga 475860
acctaaggaa ctccctaaac ggcaaaaatc ggttatcgaa aaagtagaaa cagacagcga 475920
caacaatatt tattcttccc cctatctcaa accatcaaac catcaaaacg gcaacactgg 475980
caacggtata aaccaaccta aaaatcaggc aaaagattac gaaaatttta aatatgttta 476040
ttccggctgg ttttacaaac acgccaaacg agagtttaac ttaaaggtgg aacctaaaag 476100
tgcaaaaaac ggcgacgacg gttatatctt ctatcacggt aaagaacctt cccgacaact 476160
tcccgcttct ggaaaaatta cctataaagg tgtgtggcat tttgcgaccg atacaaaaaa 476220
gggtcaaaaa tttcgtgaaa ttatccaacc ttcaaaaagt caaggcgaca ggtatagcgg 476280
attttcgggc gatgacggcg aagaatattc caacaaaaac aaatccacgc tgacagatgg 476340
tcaagagggt tatggtttta cctcaaattt agaagtggat ttccataata aaaaattgac 476400
gggcaaactg atacgcaaca atgcgaatac cgataacaac caagccacca ccacgcaata 476460
ctacagcctt gaggctcaag taacaggcaa ccgcttcaac ggcaaggcaa cggcaaccga 476520
caaaccccaa caaaacagcg aaaccaagga acatcccttt gtttccgatt cgtcttcttt 476580
gagcggcggc tttttcggcc cgcagggtga ggaattgggt ttccgctttt tgagcgacga 476640
tcaaaaagtt gccgttgtcg gcagcgcgaa aaccaaagac aaacccgcaa atggcaatac 476700
tgcggcggct tcaggcggca cagatgcggc agcatcaaac ggtgcggcag gcacgtcgtc 476760
tgaaaacggt aagctgacca cggttttgga tgcggtcgag ctgaaattgg gcgataagga 476820
agtccaaaag ctcgacaact tcagcaacgc cgcccaactg gttgtcgacg gcattatgat 476880
tccgctcttg cccgaggctt ccgaaagtgg gaacaatcaa gccaatcaag gtacaaatgg 476940
cggaacagcc tttacccgca aatttgacca cacgccggaa agtgataaaa aagacgccca 477000
agcaggtacg cagacgaatg gggcgcaaac cgcttcaaat acggcaggtg ataccaatgg 477060
caaaacaaaa acctatgaag tcgaagtctg ctgttccaac ctcaattatc tgaaatacgg 477120
aatgttgacg cgcaaaaaca gcaagtccgc gatgcaggca ggagaaagca gtagtcaagc 477180

tgatgctaaa acggaacaag ttgaacaaag tatgttcctc caaggcgagc gcaccgatga 477240
aaaagagatt ccaagcgagc aaaacatcgt ttatcggggg tcttggtacg gatatattgc 477300
caacgacaaa agcacaagct ggagcggcaa tgcttccaat gcaacgagtg gcaacagggc 477360
ggaatttact gtgaattttg ccgataaaaa aattactggt acgttaaccg ctgacaacag 477420
gcaggaggca acctttacca ttgatggtaa tattaaggac aacggctttg aaggtacggc 477480
gaaaactgct gagtcaggtt ttgatctcga tcaaagcaat accacccgca cgcctaaggc 477540
atatatcaca gatgccaagg tgcagggcgg tttttacggg cccaaagccg aagagttggg 477600
cggatggttt gcctatccgg gcgataaaca aacgaaaaat gcaacaaatg catccggcaa 477660
tagcagtgca actgtcgtat tcggtgcgaa acgccaacag cctgtgcgat aagcacggct 477720
gccgaacaat caagaataag gcctcagacg gcaccgctcc ttccgatgcc gtctgaaagc 477780
gaagattagg gaaacactat gcaacagcaa catttgttcc gattcaatat tttatgcctg 477840
tctttaatga ctgcgctgcc cgcttatgca gaaaatgtgc aagccggaca agcacaggaa 477900
aaacagttgg ataccataca ggtaaaagcc aaaaaacaga aaacccgccg cgataacgaa 477960
gtaaccgggc tgggcaagtt ggtcaagtct tccgatacgc taagtaaaga acaggttttg 478020
aatatccgag acctgacccg ttatgatccg ggtattgccg tggtcgaaca gggtcggggc 478080
gcaagttccg gctattcaat acgcggcatg gataaaaacc gcgtttcctt aacggtggac 478140
ggcgtttcgc aaatacagtc ctacaccgcg caggcggcat tggcgggac gaggacggcg 478200
ggcagcagcg gcgcaatcaa tgaaatcgag tatgaaaacg tcaaagctgt cgaaatcagc 478260
aaaggctcaa actcggtcga acaaggcagc ggcgcattgg cgggctcggt cgcatttcaa 478320
accaaaaccg ccgacgatgt tatcggggaa ggcaggcagt ggggcattca gagtaaaacc 478380
gcctattccg gcaaaaaccg gggcttacc caatccatcg cgctggcggg gcgcatcggc 478440
ggtgcggagg ctttgctgat ccacaccggg cggcgcgcgg gggaaatccg cgcccacgaa 478500
gatgcaggac gcggcgttca gagctttaac aggctggtgc cggttgaaga cagcagcaat 478560
tacgcctatt tcatcgttaa agaagaatgc aaaaacggga gttatgaaac gtgtaaagcg 478620
aatccgaaaa aagatgttgt cggcaaagac gaacgtcaaa cggtttccac ccgagactac 478680
acgggtccca accgcttcct cgccgatccg ctttcatacg aaagccggtc gtggctgttc 478740
cgcccgggtt ttcgttttga gaataagcgg cactacatcg gcggcatact cgaacacacg 478800
caacaaactt tcgacacgcg cgatatgacg gttccggcat tcctgaccaa ggcggttttt 478860
gatgcaaata aaaaacaggc gggttctttg cccggtaacg gcaaatacgc gggcaaccac 478920
aaatacggcg gactgtttac caacggcgaa aacggtgcgc tggtgggcgc ggaatacggt 478980
acgggcgtgt tttacgacga gacgcacacc aaaagccgct acggtttgga atatgtctat 479040
accaatgccg ataaagacac ttgggcggat tatgcccgcc tctcttacga ccggcagggc 479100
atcggtttgg ataatcattt tcagcagacg cactgttctg ccgacggttc ggacaaatat 479160
tgccgcccga gtgccgacaa gccgttttcc tattacaaat ccgatcgcgt gatttacggg 479220
gaaagccaca ggctcttgca ggcggcattc aaaaaatcct tcgataccgc caaaatccgc 479280
cacaacctga gcgtgaatct cgggtttgac cgctttggct ctaatctccg ccatcaggat 479340
tattattatc aacatgccaa ccgcgcctat tcgtcgaaca cgccccctca aaacaacggc 479400
aaaaaaatca gccccaacgg cagtgaaacc agcccctatt gggtcaccat aggcagggga 479460
aatgtcgtta cggggcaaat ctgccgcttg ggcaacaata cttatacgga ctgcacgccg 479520
cgcagcatca acggtaaaag ctattacgcg gcagttcggg acaatgtccg tttgggcagg 479580
tgggcggatg tcggcgcggg cttgcgctac gactaccgca gcacgcattc ggacgacggc 479640
agcgtttcca ccggcacgca ccgcaccttg tcctggaacg ccggcatcgt cctcaaacct 479700
accgactggc tggatttgac ttaccgcacc tcaaccggct tccgcctgcc ctcgtttgcg 479760
gaaatgtacg gctggcgggc gggtgttcaa agcaaggcgg tcaaaatcga tccggaaaaa 479820
tcgttcaaca aagaagccgg catcgtgttt aaaggcgatt tcggcaactt ggaggcaagt 479880
tggttcaaca atgcctaccg cgatttgatt gtccggggtt atgaagcgca aattaaagac 479940
ggcaaagaag aagccaaagg cgacccggct tacctcaatg cccaaagcgc gcggattacc 480000
ggcatcaata ttttgggcaa aatcgattgg aacggcgtat gggataaatt gcccgaaggt 480060
tggtattcta catttgccta taatcgtgtc cgtgtccgcg acatcaaaaa acgcgcagac 480120
cgcaccgata ttcaatcaca tctgtttgat gccatccaac cctcgcgcta tgtcgtcggc 480180
ttgggctatg accaaccgga aggcaaatgg ggtgtgaacg gtatgctgac ttattccaaa 480240
gccaaggaaa tcacagagtt gttgggcagc cgggctttgc tcaacggcaa cagccgcaat 480300
acaaaagcca ccgcgcgccg tacccgccct tggtatattg tggacgtgtc cggttattac 480360
acggttaaaa aacactttac cctccgtgcg ggcgtgtaca acctcctcaa ctaccgctat 480420
gttacttggg aaaatgtgcg gcaaactgcc ggcggcgcag tcaaccaaca caaaaatgtc 480480
ggcgtttaca accgatatgc cgccccccggt cgcaactaca catttagctt ggaaatgaag 480540
ttctaaacgt ccaaacgccg caaatgccgt ctgaaaggct tcagacggca ttttttacac 480600
aatccccgcc attttccatc atccccgata caccgtaatc tcgaaacccg tcattcccgc 480660
gcaggcggga atccagtccg ttcggtttcg gtttttttga ggtttcgggt aacttctaaa 480720
ccgttattcc cgcgaaaaca gaaaatcaaa aacagaaacc tcaaatcccg ttattcccga 480780
gcagacggga tctagggcgt aaaatctaaa gaaaccgttt tatccgataa gtttccgcac 480840

cgacagacta gattcccgcc tgcgcgggaa tgacgttata tttttcgcat ttgataaaaa 480900
agaccgtttg aaattttttc agcggacgca aagtattgcg taaaatgctg cttataagaa 480960
acgcaaggcg ggcgtaggat gtgcagcgtg gacatagggc tggctgtagg gtgggcttca 481020
gcccaccaat cccaccgttt ccacctattc ccccaactcc gtcaatgtta tccattccgc 481080
ccattcccac cgaaaaccga aaccgccgta ttcccaaaaa cctttgatgc ggtgaaattg 481140
gtgggctgaa gcccacccta cagcccaccc tacggctcgc cgaaatttcg tcattcccgc 481200
gcaggcggga atccaggtct gtcggtgcgg aaacttatcg gataaaacgg tttcttgaga 481260
ttttacgtcc tagattccca cttccgtggg aatgacggga tgcaggtttt cgtgcggacg 481320
cgttcggatt cacgactgcg cgggaatgac gggatttcag tttgcggatt gatttgaagt 481380
tgcaaaatcc caacggatcg gattaccgct ttcgcgtttc aaagttacgg cgttatcgga 481440
aaaacagaaa atcaaagctg caagaattta tttaaaacaa ccgaatttca acggatcgga 481500
ttctcgcctg tagggaatga cggcggaagg ttttttgtct tttctgacag atgtccgcaa 481560
tctgaaatcc tgaccgtggg aacgacggta tagtggatta acaaaaacca gtacggcgtt 481620
ggctcgcctt agctcaaaga gaacgattct ctaaggtgct gaagcaccaa gtgaatcggt 481680
tccgtactat ttgtactgtc tgcggctttg tcgccttgtc ctgatttttg ttaatccact 481740
atataaatat ttctatttca atccaatata aaatgccgtc cgaacatcgt tcggacggca 481800
tttttaatgc ttcaaatcag ttggtgccga ctttgatttt ctgccacagg ctgacagaca 481860
gttttttcgc atccgtgctc atttgcggca tcacgaaacc gtctttcata tcctgctcgt 481920
tcgggaagat ggaacgggtg ttcaccagct cggcaggcat tttttcgcgc gccggtttgc 481980
tggcgggggc aaaggttacg gcgatgccgt ttttcgccgc gatttcgggg tcgagcgtgt 482040
agttgatgta tttgtgggca ttggcgacgt ttttcgcatc ggcgggaatc agccaagact 482100
caatccagaa gcccatacct ttcggtgtca gcacttcgat gccgacgttg tttttcactt 482160
cctcggaacg tgctttcgcc aagttcaaat cgccgccgtt gcctgccgcc aggcagatgt 482220
cgccgcgtgc cagctcgtcg atgatggacg ggctgaaacg tttgacatcc ggacggatag 482280
acttcaacac ttccgccgcc gccttcaagt cttcaggatt cgagcctttg gggtctttgc 482340
ccaagtagtt cagcaaaatc gggaacattt cactcggggt gtcccacagg gcgatgccgc 482400
aggatttcag cttgtgggtg tattcgggtt tgaacagcaa atcccagccg ttttcgggca 482460
gcttgccgcc caaaagctct ttgcccttcg ccgtaatcgc aatcgtgttc acgccggaga 482520
aataggggac ggcatactgg ttgcccgggt cggcggtttc cagcattttc aagagttcgg 482580
gatcgatgtt tttatagttg ggaatcaggt ctttgttgac tttttgatac gcgcccgcct 482640
cgatttggcg cggcaggaag gcgatgcccg gcacgaccaa atcgtaaccg gatttgccgg 482700
tcagcatttt ggcttccagc gtttcattgt tttcgtacaa gtcgtaagtc agcttcagat 482760
tgttggcttt tttaaagtct tcgaccgtac tctcatcaac atagttcgac cagttgtaga 482820
tgttcagagt atcggtggca gcggcttcgg cattggcagc agacgcagcg tctgcttgag 482880
gttgcacggc gttttttttcg ctgccgccgc aggctgccag agacagcgcg gccaaaacgg 482940
ctaatacgga tttttcata cgggcagatt cctgatgaaa gaggttggaa aaaaagaaat 483000
ccccgcgccc catcgttacc ccggcgcaag gtttgggcat tgtaaagtaa atttgtgcaa 483060
actcaaagcg atattggact gattttccta aaaaattatc ctgtttccaa aaggggagaa 483120
aaacgtccgc ccgattttgc cgtttttttg cgctgtcagg gtgtccgacg ggcggataga 483180
gagaaaaggc ttgcatataa tgtaaacccc ctttaaaatt gcgcgtttac agaatttatt 483240
tttcttccag gagattccaa tatggcaaac agcgcacaag cacgcaaacg tgcccgccag 483300
tccgtcaaac aacgcgccca caatgctagc ctgcgtaccg cattccgcac cgcagtgaaa 483360
aaagtattga aagcagtcga agcaggcgat aaagctgccg cacaagcggt ttaccaagag 483420
tccgtcaaag tcatcgaccg catcgccgac aagggcgtgt tccacaaaaa caaagcggca 483480
cgccacaaaa gccgtctgtc tgcaaaagta aaagccttgg cttgattttt gcaaaaccgc 483540
caaggcggtt gatacgcgat aagcggaaaa ccctgaagcc cgacggtttc ggggttttct 483600
gtattgcggg ggcaaaatcc cgaaatggcg gaaagggtgc gattttttat ccgaatccgc 483660
tataaaatgc cgtttgaaaa ccaatatgcc gacaatgggg gcggagatga atacacggaa 483720
tatgcgctat attcttttga caggactgtt gccgatggca tccgcttttg gagagaccgc 483780
gctgcaatgc gccgctttga cggacaatgt tacgcgtttg gcgtgttacg acaggatttt 483840
tgcggcacag cttccgtctt cggcagggca ggaagggcag gagtcgaaag ccgtactcaa 483900
tctgacggaa accgtccgca gcagcctgga taagggcgag gcggtcattg ttgttgaaaa 483960
aggcggggat gcgcttcctg ccgacagtgc gggcgaaacc gccgacatct atacgccttt 484020
gagcctgatg tacgacttgg acaaaaacga tttgcgcggg ctgttgggcg tacgcgaaca 484080
caatccgatg taccttatgc cgctctggta caacaattcg cccaactatg ccccgggttc 484140
gccgacgcgc ggtacgactg tacaggaaaa attcggacag cagaaacgtg cggaaaccaa 484200
attgcaggtt tcgttcaaaa gcaaaattgc cgaagatttg tttaaaaccc gcgcggatct 484260
gtggttcggc tacacccaaa gatccgattg gcagatttac aaccaaggca ggaaatccgc 484320
gccgttccgc aatacggatt acaaacctga aattttcctg acccagcctg tgaaggcgga 484380
tttgccgttc ggcggcaggc tgcgtatgct cggtgcgggt tttgtccacc agtccaacgg 484440
acagagccgt cccgaatcgc gttcgtggaa caggatttac gccatggcag gcatggaatg 484500

gggcaaattg acggtgattc cgcgcgtgtg ggtgcgtgcg ttcgatcaga gcggcgataa 484560
aaacgacaat cccgatattg ccgactatat ggggtatggc gacgtgaagc tgcagtaccg 484620
cctgaacgac aggcagaatg tgtattccgt attgcgctac aaccccaaaa cgggctacgg 484680
cgcgattgaa gccgcctaca cgtttccgat taagggcaaa ctcaaaggcg tggtacgcgg 484740
attccacggt tacggcgaga gcctgatcga ctacaaccac aagcagaacg gtatcggtat 484800
cgggttgatg ttcaacgact tggacggcat ctgaaccgcg tgttcagacg gtatatcaag 484860
ttgccgtgcc gtctgaagcc gccggcggtt tggcggggcg ggataaaaat ttcgtttgaa 484920
tccgggcaaa cgcgtataat gtgcggtttg tacggacttt gtcggaatca agcaagatga 484980
cggaacctgc ggccgaaggc ggcaaagctg ccaaggcgtt aaaaaaatat ctgattacgg 485040
gcattttggt ctggctgccg attgcggtaa cggtttgggt ggtttcctat atcgtttccg 485100

cgtccgatca gctcgtcaac ctgctgccga agcaatggcg gccgcaatat gttttggggt 485160
ttaatatccc ggggctgggc gttatcgttg ccattgccgt attgtttgta accggattgt 485220
ttgccgccaa cgtattgggt cggcagatcc tcgccgcgtg ggacagcctg ttggggcgga 485280
ttccggttgt gaaatccatc tattcgagtg tgaaaaaagt atccgaatcg ctgctgtccg 485340
acagcagccg ttcgtttaaa acgccggtac tcgtgccgtt tccccagccc ggtatttgga 485400
cgattgcttt cgtgtcaggg caggtgtcga atgcggttaa ggccgcattg ccgaaggacg 485460
gcgattatct ttccgtgtat gttccgacca cgccgaatcc gaccggcggt tactatatta 485520
tggtaaagaa aagcgatgtg cgcgaactcg atatgagcgt ggacgaagca ttgaaatatg 485580
tgatttcgct gggtatggtc atccctgacg acctgcccgt caaaacattg gcaggaccta 485640
tgccgtctga aaaggcggat ttgcccgaac aacaataaag ccgccgttca gacggcattt 485700
tctgttttca gtttaaatca ataaaaggtg attttatgcg taccaactat tgcggcctga 485760
tcagtgagca atacttagac caaaccgtta ccgtcaaagg ctgggtacac cgtcgacgcg 485820
accacggcgg tgtgattttt atcgacctgc gcgaccgcga aggcatcgtc caagtcgtga 485880
tcgatcccga cacgcccgaa gcgtttgccg ctgccgattc ctcccgcaac gaatacgttt 485940
tgagcattac cggccgcgta cgcaaccgtc ccgaaggcac gaccaacgat aaaatgattt 486000
ccggcaaaat cgaaatcctt gccaaagaaa tcgaagtctt gaacgccgcc gccacgccgc 486060
cgttccaaat cgacgatgaa aacatcagcg aaaacgttcg cctgaccaac cgcgttatcg 486120
acttgcgccg tccggtgatg caacgcaacc tgcgcctgcg ttaccaagtt gctatgggcg 486180
ttcgccgcta cttggacgcg caaggtttca tcgacattga aaccccgatg ctgacccgct 486240
ccacgcctga aggcgcgcgc gactacctcg tgccgagccg cgttcatccg ggcgagtttt 486300
tcgcgctacc gcaatcgccg caattattca aacaactgtt gatggtggcg ggtttcgacc 486360
gttactacca aatcaccaag tgcttccgcg acgaagacct gcgtgccgac cgccagcccg 486420
aatttaccca aatcgacttg gaaacctcgt tcttaaacga ggatgaaatc atggacatca 486480
ctgaaggcat ggccaaacaa gtcttcaaag atgctttaaa tgtagatttg ggcgacttcc 486540
cacgcatgcc ttactctgaa gccatgttct actacggctc tgacaaaccg gatatgcgca 486600
tcaacttgaa atttaccgag ttgaccgacc tgatgaaaac ggaagaattc aaagtcttcc 486660
gtggcgcagc cgacatgaaa ggcggccgcg tggtcgctct gcgcgtgccg aacggcgcag 486720
aattcagccg caaagaaatc gacgaataca ccaaatttgt cggcatctac ggcgcgaaag 486780
gtctggcata catcaaagta aacgatgtca gcaacctttc caacggcgaa gacagcggcc 486840
tgcaatctcc aatcgtgaaa tacctgtccg aaaacgccct gaaagaaatt atcgcgcgta 486900
ccggcgcgca aaacggcgac atcatcttct tcggcgcaga caaagccaaa gtcgtgaacg 486960
aagccatcgg cgcactgcgt atcaaagtcg gcttggagca cggcaaagac aacggctatt 487020
tcacagacga atggaaacct ttgtgggtcg ttgatttccc aatgttcgaa tacgacgaag 487080
aagccgaccg ctacgttgcc gtacaccatc cgtttaccgc gccaaaagaa ggtcatgaag 487140
acctgatggt ttccgacccg gcaaattgtt tggcacgcgc ctacgatatg gtattgaacg 487200
gctgggaaat cggcggcggc tctatccgta ttcaccgcgc agacgtacaa gagaaagtgt 487260
ttgccgcgct gaaaatcagc cctgaagagc aacaagagaa attcggcttc ctcttggaca 487320
acctgaaatt cggcgcacct cctcacggcg gtcttgcatt cggcctcgac cgtctggtaa 487380
cgctgatgac cggtgccgaa tccatccgcg acgtgattgc cttcccgaaa acacaacgcg 487440
cccaatgcct gctgaccaac gcgcccaaca gcgtggacga caagcagttg cgtgaattaa 487500
gtttgcgttt gcgccagaag gcaaccgaaa ctaaagaagt ataaggaaaa cggagccgtt 487560
tgacggctct gttttttttca gacggcattt acgcttcttg acttccctct aattcaaacc 487620
taattttgct gtgttttaat ggcggtattg aaaaacacat cttgttcaat caaccgataa 487680
aaaaaggact gaaaatgaaa aaactgttat tggctgccgt tgtttctctg agtgccgctg 487740
ccgcatttgc cggcgactct gccgagcgtc agatttacgg cgatccccat tttgaacaaa 487800
accgcacaaa agctgtgaaa atgttggagc agcgcggtta tcaggtttac gatgtcgatg 487860
ccgacgacca ttggggtaag cctgtgctgg aagtggaagc ctataaagac ggccgcgaat 487920
acgacatcgt gttgtcttac cccgacctga aaatcatcaa agagcagctc gatcgctgac 487980
tcctttgatg gaaagatgaa ccaaaatgcc gtctgaagcg ttcagacggc attttgcctg 488040
ttcctcatca ggtatgaggc aggcttttct tattaaaaaa atgacatttc acgctgattt 488100

gttataatca ttccttttca acacgacaga cggagcaggt ttattatgcc tatccttacc 488160
atccgtgaag tgtgcaacat taatcattgg ggcataggtt attatgatgt tgacgattcc 488220
ggcgaaatca tcgtccgccc caatccctcg caacacaatc aaactgtttc actgcaaaaa 488280
ctgactgaag ccgtgcaaca aaaacatcag gcgcgcctgc ctgttttgtt ttgttttccg 488340
caaatcctcg aacaccgcct ccgcgacatt aaccgcgcct ttcagacggc acgggaagag 488400
tgcggctata agggcggtta ttgtttggtt taccctatca aggtcaacca acaccgccgc 488460
gtcatcgaat cgcttatgtc aagcggacaa ccgcatggtt tggaagctgg ttctaaagcc 488520
gaactgatgg cggttttggc acacgccggc aaccggcaaa cattaatcgt ctgcaacggc 488580
tataaagacc gtgaatatat ccgtttcgcc ttgatgggcg aaaaactggg gcatcaggtt 488640
tatttggtga ttgagaagct gtccgaaata caaatggtat tggaagaggc ggaaaaactc 488700
ggcatcaagc cccgtttggg tgtgcgcgcc agactggctt cccaaggttc gggaaaatgg 488760
cagtcttcgg gtggggaaaa atcaaaattc ggcttgtcgg cttcccaagt tttgcaactg 488820
gtcgatattt tgaaacaaaa aaacaggctg gattgcctgc agcttttgca tttccatttg 488880
ggctcgcagc ttgggaacat ccgtgatgtt gccacaggtg tacacgaatc ggctcggttt 488940
tatgttgagt tgcacaaact gggggtaaat atccgctgtt ttgatgtagg cggcgggctt 489000
ggcgtggatt acgaaggaaa ccgcacacaa tcggattgtt ccgttaatta cagcctcaac 489060
gaatatgccg ccacagtcgt atggggcatc agtcaggctt gtctcgaaca cgggctgccg 489120
catccgacaa tcatcaccga gagcgggcgc ggcattaccg cacatcacgc cgttttggtt 489180
gctaatgtta taggcgttga acgttacaaa ccgcgccggc tggatgcgcc atcgcccgaa 489240
gcaccgcgtg tgttgcacag tatgtgggaa acttggacgg atatttccgc ctcgcgggaa 489300
aaacgttcct tacgcagctg gatacacgaa gggcagtttg atcttgctga tgtgcataat 489360
cagtataatg tcgggctgtt gagtttggcg caacgtgcgt gggcggagca actgtattta 489420
aatatctgtc atgaagtcgg cgaattgttt aatgaaaaac accggtctca ccgaaccatt 489480
attgacgaat tgcaagaacg ttttgccgat aagctgtatg tcaatttctc actcttccaa 489540
tctttgcccg atgcttgggg catagatcaa cttttccctg tttgtcccat taccggtttg 489600
aatgaaccga ttgcgcgccg cgccgtgttg ttggacatta cctgcgattc agacggtacg 489660
attgaccact acatcgacgg agacggcatc gccggtacga tgcctatgcc tgattatccc 489720
gaagaagagc cgccgctttt aggctttttt atggtgggag catatcagga aatactcggc 489780
aatatgcaca atcttttcgg cgacactgcc actgccgatg ttgttgtagg ggaagacgga 489840
caatttaccg tcatcgatta cgatgaagga aacaccgttg ccgatatgct cgaatacgtt 489900
tatcaagatc cgaaagagct gatgaaacgc tatcgcgaac aaatcgaaca ttcagacctt 489960
cctgcctcgc aggctatgtc tttcttaaaa gaactcgaag cggggcttaa tggttatacc 490020
tatttggaag acgaatagac gcatcaaggc atcggatatg tcgtctgaag cccgattttc 490080
ttactcaaac accaatcatc acgaccgatt gaaaccaatt acaaggaatc attacgatgc 490140
aatacagcac actggcagga caaaccgaca actccctcgt ttccaataat ttcgggtttt 490200
tgcgcctgcc gcttaatttt atgccgtatg aaagtcatgc cgattgggtt attaccggcg 490260
tgccttatga tatggcggtt tcagggcgtt ccggcgcgcg tttcggtcct gaagccatcc 490320
ggcgcgcctc cgtcaacctc gcttgggagc accgcaggtt tccatggaca tttgatgtgc 490380
gcgaacgcct gaacattatt gattgcggcg acttggtttt ttcttttggc gacagcaggg 490440
attttgtcga aaaaatggaa gcgcacgccg gcaaattact ttcttccggc aaacgctgtt 490500
tgagtttggg cggcgaccat ttcattaccc taccgttgtt gcgcgcccac gcccgctatt 490560
tcggcaaact cgcactgatt cattttgacg cgcacaccga cacctacgac aacggcagcg 490620
aatacgacca cggtacgatg ttctataccg cccccaagga aggcctcatc gacccgtccc 490680
gttccgtaca aatcggcata cgcaccgaac acagtaaaaa attgcctttt actgtgttgt 490740
ccgcccctaa agtcaatgaa gacagtgttg aagagaccgt ccgtaaaatc aaagaaaccg 490800
tcggcaatat gcccgtttac ctgactttcg acatagactg cctggacccg tcgttcgccc 490860
ctgggaccgg tacgcccgta tgcggcggct tgagcagcga cagggcatta aaaatcctac 490920
gtgggctgac ggatctcgac atcgtcggta tggatgttgt agaagttgcc ccctcttacg 490980
accaatccga cattaccgct ttggccggtg ccacaattgc cttggaaatg ctttaccttc 491040
aaggtgcgaa aaaggactga acgtccggca tcccccgggt tttcgccgtg ccgttcaaac 491100
ggcgtattca gtctaatgaa aattcaaata ctgaaacaaa agttgcccgg agccgcatat 491160
cggaaagacg gtgaaatatc agaatatatc ttataaaaca attagttaaa tattattttt 491220
ccgatttttc gggacggtct tttttacgga ggtcaatatg atgaaattgg gtttcaaacc 491280
gatacccctc gccattgccg cagtattgtg cgccctggtt ttggcactgc ccgtacccga 491340
cggggtcaag cctcaggctt ggacgctgct ggccatgttt gtcggtgtga ttgccgccat 491400
tatcggcaag gccatgccgt tgggcgcgct gtcgattatt gccgtcgggt tggtcgcagt 491460
aaccggcgta accgccgaca aaccgggcgc ggcgatgagc gatgcgttga gtgcgttcgc 491520
caatccgttg atttggctga ttgccatcgc agttatgatt tcgcgcggtt tgctcaaaac 491580
agggctgggg atgcgtatcg gatatttgtt tatcgccgtt tttggaagaa aaacgctggg 491640
catcggttac agtctcgctc tttccgaact gctgctggct cccgttaccc cttccaatac 491700
cgcgcgcggc ggcggcatta tacatccgat tatgcagtcg attgccggca gttacggctc 491760

caatcccgca aaaggcacag aaggcaagat gggtaaatat ttggctttgg tcaactatca 491820
ttccaatccc atttcgtcgg ctatgtttat tactgcaact gcccccaacc ctttaatcgt 491880
caacttgatt gccgaaaatt taggcagtag tttccgtctt tcttgggggg cgtgggcgtg 491940
ggcaatggct gttcccggcg ttatcgcctt tttcgttatg cctttgattt tatatttttt 492000
gtatccgcct gaaattaaag aaacgcccaa tgccgttcaa tttgccaaag accgtctgag 492060
ggagatgggt aaaatgtcgg cagacgaaat cattatggcg gtcattttcg gtatcttgct 492120
gctgttgtgg gcagatgttc ccgcccttat taccggcaat cacgctttta gtatcaacgc 492180
caccgccacc gcatttatcg gattaagcct gcttttgctt tccggtgtat tgacttggga 492240
cgatgttttg aaagaaaaaa gcgcgtggga tacgattatt tggtttggcg cattgattat 492300
gatggccgca tttttaaata aactcggact gattaaatgg ttctccggag tgttggcgga 492360
aagtgtcggc ggtttgggcg ttagcggcac ggctgcgggc gtaatcctcg tgcttgctta 492420
tatgtatgcg cattatatgt ttgccagtac tactgcacat attaccgcta tgttcggcgc 492480
atttttcgct gctgccgttt cactgaatgc cccggcgatg ccgaccgcgc tgatgatggc 492540
ggccgcatcc aacattatga tgaccctcac tcattatgcg accggtactt cgcctgtgat 492600
tttcggttcg ggctacacca caatgggaga atggtggaag gcgggtttta tcatgagcgt 492660
agtcaatttt ctgatttttt tcgttatcgg cagcatttgg tggaaagttc tggggtattg 492720
gtaagggaaa aataaaataa atttccaatc tgtgtttatt tgattgggcg actattatcg 492780
tgaaatatgc cgtctaaagc cttcagatgg catatttgtg cgcttgaatg ttgcagaaag 492840
cggcaggcgg cggtgtagga aaagccaaac aaaaaccaaa ccgcctatca acttctgata 492900
aacataagca ttaaataatc agaaggttat tcaattacct aaacgcaaat ttccctgccg 492960
tatcacatct attgaaaata atacatcaac cggctcggaa gcagcctgat caggtgtttc 493020
tacttgcggc gatgaatcgg cagccggttc ggtataggca gtcggcgtgc cgtcggattg 493080
ggtatttaaa tgcggttgtg cctcgggcgt gtgtacatca ggcacttcgg gcgtgcgtgt 493140
ctcggatatt tcggcagagt tggtttcctc agtttgttca atgacttcag cttggctgta 493200
tgaggaagaa ccctgtatcc acgccagcga tttgagcggc atcttcatct tgccgttttt 493260
gccgcaggtc aggcagacgg ccgatccggt gcggtctttg agtacagcat cgactttctc 493320
gggcgcgatg gttttaccct gtgtttttgc ccattgcgca atactttttt tcagagaggc 493380
gatgtcaagg ttgttcttac cgtaagggtc gcggaaggcg gcaagccaca ggttgcgatc 493440
cgcatcttca ttgagacccg ccatttgata gatgacggag gcgggcgagc cgctggcgat 493500
ggttttggca aactcgagcg cgtcgggcga tttcatgcgg acgcagccgt gactccgaac 493560
cccggggacg ctggccggcg cattggtccc gtgtatgccc aaaccgagtt tggggtcgcc 493620
taagcggaca aaaaccggcc ccaaagggtt gtccgggccg gcggctatgg tttttacgcc 493680
gtcgccgcgt tctttctgta tggatttggg gatgtaccaa acagggttat aggctttcgc 493740
accgatttta tgttcgccta gattggtttg cgtcatcgcc cgacctactg caacgggata 493800
aaccttggtc agtttgccgt cggtgtagag gaacaggcgt tgctgaggga tgttaatgaa 493860
gacatgttga ccttgtgcga cgggggagac atcgggaatg atggtgtttg cgtatgaaaa 493920
accgcttatc aatagtgcag cagtgcggca gattgtttta ttcatatcaa aatatggtgt 493980
gtgtccgata ggttttcggc aaatcatacc tgaaaccgta ccaatttgtg cgaaaatatg 494040
cgcttcggta cagtgcggac ggattgggta atggcaacgg aaacaaatgt cgcggaaatt 494100
tccgccttgg attatgaagg caggggtgtg gcaaaggtcg gcggcaaaac ggtttttatt 494160
aaaagggcat tacttgattg tttgatgctg ggttggttca ggctttaact caggaatatt 494220
tacatcataa tgaaggtttt taaacaacag cttgaacaac tcggcgcgca aaaccaatat 494280
cgttcgattc cggatttgat tcatcaaggg cggtatatta cgcgggaaaa ccgcaaaatg 494340
ctgaatatgt cgtctaatga ttatttgggt ttggcatcag atgaaaactt gcgccggtct 494400
tttttgcagc aatacggcgg taattttccc tcttttacca gttcttcatc gcgtttatta 494460
acgggcaact ttcctattta taccgatttg gaagagcttg tcgcacaacg tttccaacgg 494520
gaaagcgcgt tattgttcaa cagcggctat cacgccaatc tcggtatttt gcctgctttg 494580
acgacgacga aaagtttgat tttggcagat aaatttgttc acgccagtat gattgacggc 494640
atccgtttga gccggtgtgc gtttttccgt tatcgtcata atgattatga acatttgaaa 494700
aatctgcttg aaaaaaacgt cggaaaattt gaccgcactt ttatcgttac cgaatctgtt 494760
ttcagtatgg acggcgatgt ggcggatttg aaacagcttg tccaattaaa aaaacagttt 494820
cccaatactt atctttatgt ggatgaagcc cacgcaatcg gtgtttatgg gcaaaacgga 494880
ttggggattg ccgaacggga taatttgatt gccgagattg atttattggt tggcactttc 494940
ggtaaagcct tagcctcggt gggggcgtat gccgtctgca accaagtatt gaaagaatgt 495000
ttgattaatc aaatgcgccc attgattttt tcaaccgcat tgccgccgtt taatgtggct 495060
tggacttatt ttatttttga acgattgccg caattctcaa aagaaagaag ccatcttgag 495120
cagttaagcg catttttacg gcgggaagtg gcgcatcgga cgcaaataat gccgagccaa 495180
acctgtatcg tcccctatat tttaggcggg aatgaagcca ccccttgccaa agcggaatac 495240
ctgcaaaggc agggttatta ttgcctgccc atcagaccgt cgacagtacc caaaaacaca 495300
tccagaatcc gcctgtcttt aacggcagat atgacaacgg atgaagtgcg gcagtttgcg 495360
gcgtgcctgt aaggatatga tatggaaaca aaattttaca atcatcaagg cggacattta 495420

atcctgtatt ttgcaggttg gggaacgccg cccgatgctg taaatcattt gattttgccg 495480
gaaaatcacg atttattgat ttgctatgat tatcaagatt taaatttgga ttttgatttt 495540
tccgcctatc ggcacatccg tttggtggcg tggtcaatgg gcgtttgggc ggcagagagg 495600
gcattgcaag gaataagatt aaaatccgca acggcagtga atggcacagg tttgccttgc 495660
gatgataatt tcggtatccc ttgcaccgtt tttaaaggca cattggagaa cctcacggaa 495720
aacacccgtt taaaatttga acgcagaatg tgtggcgata aagcatcttt tgaagattac 495780
caacaatttc ccgcacgccc gtttggcgaa attcatcaag aacttatcgc actttttgcg 495840
atgatcgggc aagatagacg tacagatctt atccgctgga caaatgcctt ggtcggatcg 495900
ggcgataaaa tttttatgcc tgccaatcag caccgatatt ggacaccgcg ttgcaccgtt 495960
cgggaaattg acgtcggaca ttacctgttt tcaagattca cccattggtc ggcactatgg 496020
aatcactgac tgccataaat aaatcgcgca ttcggcaggc tttccaaaaa gcattaaacg 496080
attatgaccg gcacgcctta atccaacaaa aaatgacgat taatttaatg acgcatttgc 496140
aagattattt gccggatatg ccattggaaa acgtgttgga attgggctgc ggctcaggaa 496200
tgttgagtgc cttgctgcaa aaacagattt cagcgaatta ttggttattt aatgatttgt 496260
gcaatgtgca gccccaactg gctgaaaaac tgccgcaatc ctttgatttt tattgcggcg 496320
atgcggaaaa ctttcctttt caacgacaat ttgacttaat cgcaagcgca tctgccgtgc 496380
aatggtttca tcaacccgac gcttttatca cccattgcaa aacaggcttg aaaacaaacg 496440
gattattggc ggttgcaacc tttggcaaag acaatttaaa agaagtccgc caaattacaa 496500
atataggctt aaattacccg actttatccc aatggcaggc ttggttagcc aaagattttg 496560
agcttttatg gtgtgaggat tttacggtaa tactagactt tgatacgccg tcagatgtac 496620
tcaaacacct taaatataca ggcgtaacag ccacgaacca aaaaaattgg acaagaaaaa 496680
atctcaatgg atttattggc gattacttgt cggcgttcgg tatgccgtcg ggcaaagtgc 496740
gcctgactta tcatccgtta tttttatcg cgcgctactc tgcggcgggc aggcaataag 496800
gcagcttatg ggcaaagtta tttttatatc gggtattgat actgatgtgg gtaaaaggta 496860
atatggcgag gcttgtgcag aaggcatatt gttaaacgtt aaattatggt atgatttaaa 496920
acttacaagt ctatttcagt aaatcgttaa taataaaagc ggacaatggc cgttgcaggc 496980
ggtcagctat ccgctcgtta cggtatcggg agtatccttt ggaaaactca ttaattatcg 497040
gtttggcact ggcggtattg ctgatgcttt tggttatgcg tgcgaagcag ggtaagaaaa 497100
caccgaagcg caaatcccaa ggtgtcggca atacgcaggg gcagacaccc ggaagcaatg 497160
attcggactg ggtcgatcag attggacaat cggtttcaga cggcacgcaa cccgactggt 497220
cttggaacga aagtgccgag accgcatccg ccgccgtatc cgcgcaagaa gtcgatccgc 497280
ttacggagta tcaggtttat aagcaattcg gttatcaggg caaggctgcc gaatctttgg 497340
ctgcctatct ggacggcatt ccggatggtg aagcgaaacc tgaaaacctt atccgcgagc 497400
tgctcgatat caatctcgaa gtgggggatg tcgatgtttt ggcagacaat ctgcaaaaat 497460
acggcaaact gattctttcc gaacttttgg caaaatatat cgaacaggca ttacagcgcg 497520
attcaaacca tttgcgtatc cgcgtcttgg cggaagaagg tttgggatgg ggtactcagg 497580
agattgaaaa acgtgcggaa ggcggttctg cgacggcagc ttccgcatcg cccccgccgg 497640
atgccggcgg taaggcttat gaagccgaag aaatcaagcg catcccgatt gtgcggggca 497700
aaaaagacgt gtccggaatc agtcaagagg aaatcggtgc gattgccggt ttggtccgtg 497760
ccgatcaagg tgcgaaaatc cttaaagaca aagtcagcta tgaaacggca tcgaaacaat 497820
acgaccgtgc catccaaact tccgaaaaac ctgcaaacct gattatcgat gcgttgaaac 497880
tcgattacca acacgcggac atagaccgtt ttgccggaca tttgtggaaa ctttaccaaa 497940
cgttgggcaa ctacggcagg caggttaaag agcggatgct ggggtggggg tacagcttgg 498000
gttaccatga agttttcgat gatttggaaa aagggccgaa cgaccggcaa atcaaagaca 498060
tcggtatggg gcacgggtat ctgccgaaaa atatacagaa attcaaatcg caacatcggg 498120
atttggtgct tcaagattct tcgttgatta acaccggttc gtctccggca gacgatgcgg 498180
ttaaggaagt agagtcgttg ctgatgtatg gtcagattga agcggcaatg gatgtgttgg 498240
agcaggcggt attgaaatat cccgacgagt cccagcttta tattacgttg atcgatattt 498300
atgaacgtac tgaagattgg gataggttgg ggcagttttt aagggtattg agggaacgtg 498360
cggacaggct tcctgaagag gtcgttatgc tgatgagccg gctgctgcag cgtatgaatc 498420
aaaatattaa aaaaataaaa cggtacggaa aataaaaatg gaagttcaac tgccgaaaat 498480
taaaacagta cgcgtaatgt tggcggggat gacggcgcag caggaatccg ttttcaaaat 498540
ggcattcaaa atgcacaata ccacccgtta tgaaacagta tccccttcag acggcagtgc 498600
cgtgcccgat ttggttttgg cggataccga tgccgagggc ggttttgaac tttggaaaga 498660
gcttgccgag cgttataagg atatacccgt cgccgtctgt tcggagaaag ttcccgattc 498720
tgaagttccc tacctgccca aaccgattcg gtttgaaaca ttgtttccta tgctccgcaa 498780
attgttgcag ggcgagaatg tttatgggaa atcgtttatt gcacccgcag accggtcggc 498840
gaaaaataac gggaatgtgc agcgtacggt tacgatacgc cagtttaacc cgaataaagg 498900
attattgggg gcgttgcggt ttgcggaaaa gaacaggcag gacatcgcta tcttgcatgg 498960
aaataagccg gtccttattg ttttcccctc gatacaacgg gttttgctga cagaaagtgt 499020
gcaaaaactc gaagaattgt gcaaagacga aaatttgcag gtcagctgca agactgttcc 499080

```
cgataacccg caatggcgcg aaaaggctaa agtaggcatt atgtcctgta tgtggcagtt 499140
ttccatttgg acagcgcagg gcaggttgat ttatccgatt tctcccgata ctccgtttac 499200
gttgaaatct tggccaaacc tgacccggtt ggcaaatgtg ccggggtcga tacgcttgtc 499260
ggcatttctg accaaggcat ccgtcaacct taacgtgttg tataaagtga tgcctttaaa 499320
cctcaatgat attctgaatt atcttgcggc aacctataca accgggtttt tgtcggtaga 499380
tttaaaaacg gtttcacaac aggcatactc cgatatggcg gataaaataa atatcggagc 499440
cgattctgcc tctgatagtg aaatgatgaa aaaagcggaa aaaatcacaa caccatccca 499500
atcccagtcg cgcggccttc tgcaaaggct gatgaaaaaa ctgttgggca gctaagaggc 499560
ggagagatga gagaaaataa aattattttc acaggacctg tcggcgtagg gaaaaccact 499620
gccattgcgg ctatttcgga cgaagcactc gttcagaccg atgcttccgc atccgatatg 499680
actttggata ggaaaaggaa tacgacagtg gcgatggact acggggccat cagcttggat 499740
gaggatacca aagtccattt atatggtacg cccggtcagg aacggttcaa ctttatgtgg 499800
gaaatcttaa gccaaggcag tatgggtttg gtcttgcttt tagataatgc ccgaaccaat 499860
ccgttgaaag atttggaatt ctttttacat tcgtttcgag ggctgctgga gaaggcaccc 499920
gtcgttgtcg gtattaccaa gatggatata cgctctcagc ccggtatcga cgtgtatcac 499980
aaatatcttg caaaacataa tcttaatgtt ccggtttttg aaattgatgc ccgtaaggaa 500040
gatgacgtaa aacaattggt tagcgcaatg ttattttcta ttgatccggg actggaggtt 500100
taatatggaa tcaacacttt cactacaagc aaatttatat ccccgcctga ctcctgccgg 500160
tgcattttat gccgtatcca gcgatgcccc cagtgccggt aaaactttgt tgcacagcct 500220
gttgaaagca gatgcggacg aaatggtcag cagtgagaag ctgcttactt gggcggacac 500280
cgccgacatc gataccgctt tgaacctgtt gtaccgtttg caaaaactcg aattcctcta 500340
tggcgatgaa aacggtcatt cagacggcat caatttgtcg gacgagcaat tgccgttgct 500400
gatggaacaa ttgtccggca gcggtaaggc gttattggtc gatcggaacg gtctgtatct 500460
tgccaacgcc aatttccatc atgaggcggc ggaagagttg gggttgttgg cggcagaagt 500520
cgcacagatg gaaaagaaat accggctgct gattaagaac aacctgtata tcaacaataa 500580
cgcttggggc gtttgcgatc cttccggtca gagcgaattg acatttttcc cattgtatat 500640
cggttcaacc aaatttattt tggttatcgg cggcattccc gatttgggca aagaggcatt 500700
tgttactttg gtaaggattt tataccgccg ttacagcaac cgcgtgtaaa acttgggaga 500760
gaggaggggt tatgcagcaa ttattgattt caatccttga agatttaaac aatacatcta 500820
cggatattat cgcgtctgcc gttatctcaa ccgacggatt gccgatggcg acaatgcttc 500880
cttcacattt gaattcggac agggtagggg cgatttctgc cactttgctt gctttgggga 500940
gtcgctcggt gcaggaactc gcctgcgggg aattggaaca agtgatgatt aaaggaaaat 501000
caggctatat ccttttaagt caggcgggta aagatgccgt gttggtgctg gtggcaaaag 501060
aaaccggcag acttggttta atcctattgg atgccaaacg tgcggcaagg catattgcgg 501120
aagccatata acatataaag attgcgggct tgcagataaa gtgcaatcga ttgtcaattt 501180
atattgacac gttcggtatt tctgttttat tattcgcgct tgttccccga tagctcagtc 501240
ggtagagcga cggactgtta atccgcaggt ccctggttcg agcccaggtc ggggagccaa 501300
atttcaaaac cctctaagta ttttcttaga gggttttgtt ttaccggcgg tcagaaacgc 501360
atttttgaga tgattgtttg gagatggaat aaaatctttg caaaattcct ttcgtgatgg 501420
ttatgaaaaa ataggggctg tcctggacag ctaggataaa ctcgatttta tagtggatta 501480
acaaaaacca gtacggcatt ggctcgcctt agctcaaaga gaacgattct ctaaggtgct 501540
gaagcaccga gtgaatcggt tccgtactat ttgtactgtc tgcggcttcg tcgccttgtc 501600
ctgatttttg ttaatccact atactaattg agacctttgc aaaattcctt tccctcccga 501660
cagccgaaac ccaaacacag gttttcgtct atttccgcta ccaatcactc cctaattcta 501720
cccaaatacc cccttaatcc tccccggata cccgataatc aggcatccgg ggtacctttt 501780
aggcggcaac aggcgcactt agcctgagac ctttgcaaat ttgtcggttt cggggtcgta 501840
ttggtagcct cgtgcctgta tgtcttcttt gaaagtttcg tatacgtcgt gggctaaaag 501900
ggctgttccg acatagggaa ccgcccttgt gctgaatttc gcgcctaagc gggcaagttt 501960
gccgaccccc gccaatacgc cggcgcggga tacgctggcg gttattttgg cgttgattcg 502020
ggcttttgcg cccgtaggga tgtgtgttaa atctaccgtt tttattaaat cagatgaata 502080
agttttacta tttttaggta caaacttatg aattttcgca ccttgtccgg tatcaactga 502140
aacagtttca gatattttta ctgcatttgc attcgcttca aacgaataca tcatcaaaat 502200
tgcaattatc gacaatttcg caaaattcaa atttgtatat tttatgacca tctttcaggg 502260
attctttaat taccatttct gaattatcag aaaatgagat tagccaaata tcatgtttaa 502320
ttcttctatt ccagaaaaaa gagaaacaat caataacatt ttcagactta ttaatcttcg 502380
caaattcaac aaattcagat tgcgctataa ccgccatcga ttgcccaaaa tacttgctgg 502440
acggctgata tttataaagt gccaactgcg cctgagtgat aaacggcttg ttcatggttc 502500
tgcctttcaa tgattgtttt gaaagcctga ttttgacacc ataacttcat gcgctcaatt 502560
cttaaacaga accgccccga ttaatacggg tacggaaacg ccgagataaa aataaaaatc 502620
catcatttca aaaccttttt cagcagggaa acaaagtaaa cggacgcgag gatgccgaat 502680
actatccagc ctgtttcaag accgctttgc aggttgtctt tcggactgca ttccgccaat 502740
```

aaaagcctta gcggctgacc gtccgacatc ttccacaggc tgccgttata ttccggcctg 502800
acaatctgtc cgttttcttt gattcttggt actaccaagc tgaaataaag gttttcagcc 502860
tggtgcttct caagacattt atttccgact tggtagtaca tgccgtctta cttcatcact 502920
ctcttaacga tggaaaatac aaaaagcgcg gcgaaaatgc ccactacaat ccaaccggct 502980
tccataccgt ccgcttttgc ggcttccaaa gcgtttttg ccgtatcggg caacgttgca 503040
tttgcatgtg cggccaaagc caggggagca gctgttacaa cagccagttt tgcgccgtat 503100
ttacggcagg tgttaataaa tttcatgata ttttccttca aaaagtgttt ggcggtaatg 503160
gatggagcgt tttcagacg accgccgaac atccgaaaat cagtctttca aaaatccgaa 503220
tacgacaaat tcgtattggt tgccgatttc ttccaaacct gcgttaatcg cttcttcgaa 503280
gtcgtagaaa taatcggcat tggtgattaa tttggtatgt ccgatgtcgc ccgtttcagg 503340
agagagatac agaaagtccc ctgttgatac ggactggaca acatagactt tctgcattca 503400
atcagccttt cttcacgagt tgaaaaccga tgactttcag tttttgggtt ttgcccgtag 503460
tgacgatttc tacgttcagg tttgcttcga tcggaaattg ggcgtttcgg aactgctcga 503520
aattggcaga gccgccgaaa tcgtattcag tagtagagct gcccaatgcg ttgccttggg 503580
agctgtctaa gggtgtggcg acaatcaggc agcaatagtc gaagctcttg ccttcgattt 503640
gtccgttgat ttttttaacg ccgacgatgt ggccttgaag ttggatgttc attttttggt 503700
ttccttgtgt gattaaacgt ctttcgggca gacactttaa gcccatgaaa tcggtagtct 503760
tgcgaatttg tcgtaaatga agttgttata gctttcttca ttgttgacgt gtttttgctg 503820
ttcaagctgt ttttcaagat tctcgtaata ttcgtacata tagtaagggt ctttgtacgg 503880
tttgaatgcg ggctgttcat gaatggcttg agctttcaaa aaggcgcagt cgtaggcttc 503940
gggagccaaa gacttgggca gcttgtgatg actcggctca atcagttcaa acagtttggc 504000
tttgtccaat tcgggaaaaa tgaatttcag accgtttgcc gcacgtccga actgtttttt 504060
tacccattca aggtagcggt cggctgaaat gaccttatct tccttaaccg cgtgtatgcg 504120
cgttgccttt tgggcgaatc gttcgcaaat cggatatgcg ccgccgaaat attcgcccgg 504180
attctgcaaa acttcgaaag ggataacgat gtcttttgct ttgaattcaa tttcaaatcg 504240
cgtccatgtg cttgttttat cgcccaactg cttgcctttt tcatagacgc ggacatattt 504300
ggacgattca cgggagccga taccataggt cttgcctttg gtcattttgg cttcatcgtc 504360
ttcttcccaa tctgacccca aacattcgcc ttttggtttg acgtgatgac aggtaaacat 504420
acctttattt cggtcttcac gggcttggtt cgggctgtat tcgccgttga aaaagtcttt 504480
tgcgatgtca acgcgtgtga tttttgggcg gattgcatta gtcaggaatg cgaaaagtcg 504540
tgattcccag ccttcttttg cgacgccgca accggtgccg gtcagttcga aaagaatggt 504600
attttgttgg ccgccaaaat ggacgcgacc gtatagggcg tcttccgaac ccatcaacca 504660
acagcgctca tagaaacgac cgcccgaacc tttggattct ttgtagatac cgaaaccgaa 504720
aacttcttcg gcgagcatgg acgcggcgcg aataaaatct tcgtcttcca aaagacttac 504780
acgaacgccg tatttatcga aaaaggtttt ttcatgaaat gaaaagctaa tttgatcaat 504840
gaaagccgaa tctgatacac cgcgccgaag aggaacgcct aacaggtttc ctttaccgtc 504900
cgttatgtac gtttcgtaac attcgaagac ttcctgaacc ctgcctgccg tttcggtttc 504960
tgtccccccc tgttagataa ggggggaaga tttgaagcgg ttgtcggctt cctgccgtcc 505020
gctagcgcgt ccgtcatcac gccggcaacc gcctttgtca tcccttgctt atcttccatg 505080
gtgcgaatcc tcaaaaacgg gcaaaaaaaa gccctgttac ttgtagaaag taaaggacgt 505140
taatttttgt taatcgtccc ttcttaggga cgcaatatat aaggccgtct gaaacggttt 505200
ttctgttttt agacggcctc ttggcttaga ccttgagaac cgcatgcgtg cttaatttat 505260
tatctaatga aaaaagtttc cggctttcag acgacctttt gtaatattat cggcagcggc 505320
tcaatgccaa ctttaaacct gctccgattt cttcagggct gttatccaat gataaaatta 505380
catcgtctgc atcaatggca tcccacgctt ccagcttgac atggcggctc gggctgattt 505440
tcaggcagcc gttgtgcagc caaatatcta cgctcatcat gtttttaaat agggcgcgtc 505500
tggttttata gcccaagttc ccgcatagct tggcaaccca atcctcatag cgttgccgaa 505560
ttttttcggt atcaaaaaaa tcttggtctt ctggactgtc ataaacgaaa gtcctgctgt 505620
ttgccaatgc ttgcaagacc gttgtgccta aagtttcatt gtcggtatcc aatggcagga 505680
tatggggggg atataggtgg tctggagcat atcgcccaaa tcctgaccat gtttgaataa 505740
tcaaggctct ttcatttgcc ttatagccag cccaataatc ttgttcttga ttgaaagtca 505800
tttattcgat ctccgtaatt ttgactgtaa tgttttgact tttgccatac tctaccacac 505860
gttgcaactg caatctttgc tccttattag tttgtgcggg tatggccaga tggatttcgc 505920
gctgtttgat catgtctgcc cttaacggta cttctgataa ttcataactt ttgaaatttg 505980
ccgtcttatc gatgtaccct ttcatggtac tgtaaagctg ttcgggtttg gacaggcgtg 506040
ccgtagtttg cgtatccaga gttttggcac tgattgccgt gcctgtacca cgatcaaaat 506100
aatcaaatgt tttaaaattt ttaggtaacc ttgcattggc agacaagccc ttaccgacat 506160
aatcctccca aggcattccc tgtccttcaa tccccttgcc ccacttgata ccgacttcgg 506220
attgggacag gatatttcgc tgtacgtcag cagttttcgg agtcaaggaa gttttcacac 506280
ccgttgccaa gtttcccaat ttgcgcgtaa tcagcgtttc caatccccat acggctagat 506340
ttttcgcatc agatactaac ggcgattcgt attctattgg tgtacccaaa gataggacaa 506400

ctttttttacc aaaatcggac tggtaagtgc caaacaattg ccggcttcct tctgaggcct 506460
gtgtataacc accggtcata cctgccgttg caataagtcc accggccgca cagccaatcc 506520
cggtactgca cagacctccg cctatagcac ccgaaccgac aaaagtcgtt gcacccaatc 506580
ccatattgcc cgcaccctta atttggtgg cagcacggtc gtaactgctg cgtatatcat 506640
tcaggctgtt ccatgttcca tatttaaatg catccgtccg catcaatacg ttttgttccg 506700
ctacaaactg tttaccggca tcttggaggt tttttagtcc tttataaaga gggtcgaagt 506760
caggtacgcc ttccgcgcac cgggttaatg cacatgcagc ggcttttagg cggtactgtt 506820
cttcagccga tttgcctttt gacagtttgt taaggatttg tgtttctttc ggatgcagct 506880
gcctattgtt ccaatcgaca ttcgccccta ctgccgccgt accgacattc ccgcccgccg 506940
catagccgat tgccgccccg ccccgtgcag tatgctcctg cctatgccgc cttctttcca 507000
ggtgtcgtag cggctttggt tttcggcaag ataggcgttt acttggccga gggatgcgcg 507060
gaaggcggct ttttcggctt cgctgtccgt gttttgcagt tcggcctcca gcagggttcg 507120
ggcttcctga taccgttcgt aactttgggt attgccgagt ttgtcggcaa cggccgctac 507180
ggcttgggtg gcgtttctgc cgaactcctt cgttacttcc ctttgcaggt tgatctcttt 507240
ggcgaccgcg tctttgtcga agctgttttt cagacggcct gagtgttgat ccgcagtttc 507300
ggtgtcgatg ccggtgtaga tacgcgcttc ggtttctttt gcagtcctgc ctgttcgggc 507360
aagttgtccc gcttcgtcgg tgatgtgtat gttgcgggtg ttgatgccgc ttttcgtgat 507420
gctgctttga ctgtcgctgt cgctgccgta gccggctgcc aggcttatcc tgtcggtagg 507480
tctgccttgt ttgtcggtaa ccgtgccgtc ccagccgccg ttcaggtcga aactgccgcc 507540
tatgccgaag cttttgcctt cgtagcggct gtggttttga atgtcgctat gggtgagggt 507600
ggccgtctga aaaaggtttt tgcccttatc ttctgcgctt tggctagacg tgatgatacc 507660
gcccttgagg tctgtgttgt ctctgacttt gatttgatag ccgtcttctc cggcataaat 507720
accgctttgc tcggttaccg aagcatggtc ggctcggatt ttgctttggc tgtaatcgcc 507780
actggcactg aagccataac ctacggtcac ttgtgcactg gcgttttgtt gtttgctttg 507840
ataggtttca gtatcttgaa cactttctat atgcaggttg cgcgtatctg cctgtatgcc 507900
tttgccgatg agctgcgcac ctttgagggt ggtatccccg ccgcttcgaa tggtagtttt 507960
accggttgtg ctgccgacat gggtgtggcg gtgggttgct tttgtctgat tgctgtggtg 508020
gcttgttgaa agaaaggctg tctgaaacgt atttgttgtt tcagacagcc tcctggctca 508080
aaccttgaaa actacatatg tgcgttccgc acatcctacg tattgagttt aggtttcaca 508140
tgagctacgg cttgctatgc cgtctttttt ccaggtgtgg ccgcggcttt ggttttcggc 508200
aaggtaggcg tttacttggt cagatggcgg atttttttgtt cgtcgtagat gatggagacg 508260
ctgataccgg agtaagcgta gattgggtct tgacctcaaa cctacacttg ttttacataa 508320
aatttcgtgt ctctatttga aaaatctaaa taacaacatt ctactttacc tattgaattg 508380
attatagttg aaacaggaat attaagaagc ctaataccca aatcatcaat ttcaaaatca 508440
ttaattccac tcttataaag atagcttatt atttcatcat taatttttcc aagccaatta 508500
aaagaaatat cttctaaaaa aaacttattt ggttcaaata tctctatcgc ttcaagctga 508560
tttttatcat cataaaaaca atggatattc aattcgggaa aaacatccat aggaacccga 508620
gagtatgatg acttataaat ttcttgtaca tcagaactaa atattgcacg aacttgtttt 508680
cgattcagat ttgatgagat aatatttctt tctatttttt ttgaactaaa ataaaaaatt 508740
gccataattt ttttgcctaa acaatattac cattttcgta agatgcatag aacaaaccat 508800
gtcttatcca ttttgttcca tcggcagaca gataacgact atatctaaat tttatttttc 508860
actctcataa aaaatttctt gcaatattca atatatttac tttcttaacc atagcgtaaa 508920
ttcctcaggc ttatatattt cagtataagt atgacttaaa ggatatgacg ccgcgtgtta 508980
cgagttgctt cttttgattt cagggtttat ataagttatg gcttgcctgg gctcgaagtg 509040
ataaagagag tatttacttt tcaactataa aaatatgaga tagttccatg ggaaaaccgt 509100
aatttaagtt ttaataaagc accttctagg cgatataaaa attttctata attttcattt 509160
ggtttatatt tatatataag ctgtatttca atagtctcat agctactttc tccaaaatct 509220
tcaaaaattt gacctgattc aatatattga atatatgaat ttattttttc ttgcaacaaa 509280
aacaaatgct cgttatccca ttttaagtga tcagaaatag ttaatatgag tattccgttt 509340
tcaatagagg ctgaatcaat tacattctct tccaaaatag acattatctt ttcctttcaa 509400
ttataacttt agtaggttca attttggtcc cctttggata gcccggtttt cccttaccga 509460
ccactgttgc tcccgttctt tcaatttcag gaaaagcttt tttctgattt ttagtaagtg 509520
gcgcagttat tgaagcctta cactctgtac aaacatcaag accaccttct ttcgaaataa 509580
tatcaagcct agtttttaca ccactttttg ttttaactgt aatctgtctt tgcggtttaa 509640
agccttgttt aactttcttc tgataaattt ccatctcaaa atcctcacca gattttttat 509700
ttttttccag ttgatcttta cgatttttat gtttgattcc cttgctagcc aatgccgtat 509760
ccggaatcct gtccccttc gcaacattgc cgtttgcagg gatacggata ttccccgcac 509820
ccgccaataa gggatcgctg ccggtaactg tcggcttgat gtttttcagg ttgcggatgc 509880
ctgcaagaat cgggactttt atcctcggat tggggttgac aaggctcgtc agtccttcgg 509940
caacgttcag tgcaaactct tcgtttttcc gctctccctg cctgattttg gtgtctttcg 510000
tcccgctgtg cagccaggtc aggttgcggt attcgggttt gtcctgtcgg cggtattcct 510060

caaacagctt cggatcgttg taggtttgcg gatttcttgc gccgtagtcg cggtagtccc 510120
aagtataacc caaggctttg tcttcgcctt tcattccgat aagggatatg acgctttggt 510180
cggtatagcc gtcttgggaa cctttgtcca cccaacgcag tatctgcctg cggattctca 510240
ttgccgcttc ttggctgctg atttttctgc cttcgcgttt ttcaacttcg cgcttgaggg 510300
cttcggcata tttgtcggcc aacgccattt ctttcggatg cagctgccta ttgttccaat 510360
ctacattcgc acccaccaca gcaccaccac taccaccagt tgcatagccg atggccgcac 510420
cgcccagtgc gttgaccgcc gctttgcccg ccggaccgag gttttccgcc gctttgtcca 510480
aatacggtgc ggcaagggaa gtgccgccgc cggccagtat gccgccgagg ctgccggtcg 510540
tcagtccgcc tgccgccccg tgcagtatgc tcctgcctat gccgccttct ttccaggtgt 510600
cgtagcggct ttggttttcg gcaagatagg cgtttacttg gccgagggat gcgcggaagg 510660
cggcttttc ggcttcgctg tccgtgtttt gcagttcggc ctccagcagg gttcgggctt 510720
cctgataccg ttcgtaactt tgggtattgc cgagtttgtc ggcaacggcc gctacggctt 510780
gggcggcgtt tctgccgaac tccttcgtta cttccctttg caggttgatc tctttggcga 510840
ccgcgtcttt gtcgaagctg tttttcagat ggcctgagtg ttgatccgca gtttcggtgt 510900
cgatgccggt gtagatacgc gcttcggttt cttttgcagt cctgcctgtt cgggcaagtt 510960
gtcccgcttc gtcggtgatg tgtatgttgt gggtgttgac gccgctgcgg gtggtgctgt 511020
ttttgctgtc tccgtcgctg ccgtagccgg ctgccgggct tatcctgtcg gtaggcctgc 511080
cttgtttgtc ggtaaccgtg ccgtcccagc cgccgttcag gtcgaaactg ccgcctatgc 511140
cgaagcttct gccttcgtag cggctgtggt tttgaatgtc gctggcagta agggtggccg 511200
tctgaaaaag gtttttgccc ttatcttctg cgctttggct agacgtgatg ataccgccct 511260
tgaggtctgt gttgtctctg actttgattt gatagccgtc ttctccggca taaataccgc 511320
tttgcccggt tacggaggca tggtctgctt tgactttgct ttggcggtaa ctgccgcttg 511380
cactgaatcc gtaaccgaca gtaacttgga cattgccgtt ttgctgtttg ctctgatagg 511440
tttcagtatc ttgaacactt tctatatgca ggttgcgcgt atctgcctgt atgcctttgc 511500
cgatgagctg cacacctttg agggtggtat ccccgccgct tcggatggta gttttgccgg 511560
ttgtgctgcc gacatgggtg tggcggtggg tagtacttcc cccttgctct ttacctttac 511620
cgatatttcc tccggcggta attccaaacc tgatgccgtt gcctattttg acggctacgc 511680
ctgcattcca accactgctt ttgtttttgc tttgctcgct gccgtcctgt ttggcagatt 511740
ggagtctgat atggttgtcg gcaatgaggg cagtacctgc atggccgatg acatcggaac 511800
ctgtaatatt gatattggac tgctccccac ttcctgttgc cgcaagtgtg gtttgccctt 511860
tgccgataat ttgacttgct gccgcttcgg tgtaatgtct tttttgctcg ttacgacttt 511920
tctgttcgcc gtaggtaatg gacacactga tactggggct ttgattgttg ttttgacctt 511980
gtcccgcact gctgcttgga gcaaattgtt gcatttgttg ggttgcttga taactctgcc 512040
atgcagcatt ggctgcagcc atggcattaa cgcgtttatt tttacttttg cccacatttt 512100
gggctgcttg tatgaagttt tgtgcagctt ggacaaccgg gacattgagg gcgacggtaa 512160
ggccttttttg ttcctgggta tgggcgtagt cagtggcata ccggttgttt gcgaactcta 512220
catctatgct tttggctgtg acggtattgc gcccctcggg gctggagacg gtactgccgg 512280
tttgtcggta gcggtttcct gcaactgtaa cggtgtctcc attcaggctg cctataatgc 512340
tgcctgtatg gacaatattg gtacgatcag tgtcatcggt agttttccgg ttaccgatag 512400
taaagcccaa tccgccagta cccatgacgc ctgatttttt gctctcgtgg tattcattgc 512460
cggtatagcg attatgggca gtagaaatat cgatgtcgtg tcctgctttt aaaacaatgc 512520
ccttatcaga aataaggttg ctgccgcgta cattgatatc ctgcccggct gcaacaatca 512580
ttttgccgcc gccgatgttg ctgccgactg cttcatcatg actgaagcgg tagcggtcgt 512640
gtgttttggt actggaaagg atgcctttgc tttttccgct taccgaggta tccagttcgg 512700
ttatttggcg tccttcgctg atagtgacat cacgtcctgc ggcaaggacg gttttgcctt 512760
cttcggcctc cagttcgcct tggcggattt ttaagtcgtt accggctcta agcagtgcgc 512820
cgttttgcgt gcggatactg ctgccgactt cggtactttg gcggacatgg cgatggttct 512880
cgtcatctaa tgtaccatag gcttcgcgat gttcggtacg gatggtgccg aggttgagat 512940
tattgccggc ggtaatttgg gtagtgccgt ctttaacttg gttagagacg gtggccgcat 513000
tgaggttgat atcgttgctg gcatgcaggg ataggatgcc gtctgaagtt ctgttatcta 513060
cttgttcagt atggcttccg accacgttaa tgccggccat acgatcgatg gcggtattgc 513120
cgttacgttc attaccggaa gtttgggttg taccgttaag gttgatattt tgcgcttggg 513180
cagtcagcag tctgcctgct tgtacctgcc cgccgtcgat attgatactt ttttcagctt 513240
ttaagccgat ttggtcggct tgaatgttac cgttgctgtt aatattccgt gcctggatga 513300
gtacggcctg tcgccccgca atggtaccgc tgttagtcag gttgccgttt tgcagtttaa 513360
gtaagacttg ttcggcacta atcaggccac cggaggtatt gagatcacct ttgcgcgcca 513420
gggcatagac tttaggaacc agtacggttt gagtcgaacc gtcagacagg gtgacggttt 513480
gattttccat ccaaacgata tctgaagtta agcgggcaac ttgctctgca ctcaaggcga 513540
tacctggggt gagaccgaat gttttggcag cagtaaggcc gttgtccatc agagctttga 513600
attgttcttc atcactcctg tagccgtcga gtcggcggta gcctgttaac tgatggattt 513660
gttcattaac aagtttttgt tcgtagtagc cgtcgccaag ccgtttgtgt agatgattgg 513720

tgtccaattg cagttgttgc aacatgtagt cgctgcccaa ccagcggcgg tagtctgcaa 513780
attgaggatc ggtttcaacc aaccagcctt tattgtcagg atgggtggta tagaggctgc 513840
tgttaggcag agtaacagta gcgttattta acgagaccac attaccggta tggatgcgct 513900
gaccattgac ggctgccgtg gatactccgt caatcagttt gattgcagat gcggcgggtt 513960
gaaaggaagg ggaggcggca ttctgttgga tgacggatac aggcgtgtcg aagtcgtggg 514020
taaatagttg ggtatcatgg taaggagtat ggtttctttc agtacggcgt tgtctttttc 514080
taccgctgta ccatcctttt tttgtaactg aatcccactg tgtgccgaca gcatctgtgc 514140
gacctttgcc tgttgtactt tgattggtaa tttctttctg gtttaaatca tcagtgataa 514200
tacgcccgcc tactacaatc cggctgtctt tgttcagcca attttgacct gaggcagtca 514260
aatcaccgcc cacagtaatg tgtgccggcc ggttttcgat gatgcgttct ttataagtct 514320
cgatgtggta gtctcggaca tgccattggt tggcctcaat acgagaacca ttttttaaat 514380
ggaacgtagc agtagtttgg tctttttgtc cttgcgagtt gtcgaataaa ccgtcttttc 514440
ccgcctgata gtaggtattt tgccccagta cggtgtagtc gcggacttgc ttttccgctt 514500
tggctaagta tgtctctgtt ttaaagtgat tattgatatt ctgcatattc cgaacggaca 514560
tcaatgcatc accttgtact tccaaaccgg cactgccatt aacaaaggta tcggccatgc 514620
ctgccgcatg atgttgttca tccagtcgat tacctacggc aaaaatacct tcgctggata 514680
gtagggcacc ttcttggtta tgaatctctt tcgctccaat atccaaacgt ttccttgcag 514740
ctattgcccc cgctttggta ctgccttccg tcgtttcttc ccggttaagc agtatttgcg 514800
cgtccagggc aatatggttg ccatagattt tgcctgtccc ggtgttggtc agggtttgac 514860
ctgcaccgat gtgggtcaaa ccgtcgctgt tgatcaagcc cctgttgtca acatgctgtt 514920
cggatgtgat gtccgtttgt tctccaccaa taattttgcc tgtaacttgg ttatctatat 514980
tgccggcatt gagtttgagc gtatggcctg cttgtagggt atgggtattt ttcagacggc 515040
cttttatgct tagatttaat tgtttgcctg cagtgaggtc gcgctctacg acgaaatcgt 515100
ccgtcaaagc aatatccagt ttgttaccgg ctgttaatgt gccattgttg gcgagtgatt 515160
tggcttgtag cgatacatta ccggcagatt gaatcgtgcc atccgcattg tttaacgcca 515220
aagtgttttg atttttatcg tgaatagaca actgccggtt ggtggcaatt tcaccatgtt 515280
ggttgtatag gccgtctgaa acagctaatt gtgcttggtt tgcagatagg agtttgccgt 515340
tttggttgtc tacatttcga ctattgatag tcagttgttc ggtagcagta atatggccgc 515400
tttggttagt cagttgctga ctttggatgt taaccgtttc agcctctata cgtccgcgcg 515460
tattatctag agtctgacct tcggtattca gtcgtgcaac agaaactttt cctgtattgc 515520
gaagattatt cttggtggta acggttccac tatcggcaag aatgttacct gcattatgta 515580
aaccggcggt atcaagctgt aaatgtgcag ctcgaatatt gcctttttta tcattgctca 515640
agcggcccga atgaatgagt gtcagattgt tggcggcaat ttctccggca ttatgcagtt 515700
cacggttatc aatcttgccg gtttgagtta ataagtgacc gctgttttta gcagtttgag 515760
tgttaacagc cagatcgtgt gcctggagtt tgccttttac cgtattgtta aatgaatcgc 515820
ctgatactcg tagtttagcc gcattcagac tacccgaatt tcccaaaccg ttttgggcgg 515880
caatgtcaat ttgcccaccc gcattaattg atcctgcatt gtcaaatgct cctgttgttt 515940
gaatgcgtcc tacggcgtag ttttttgcag gtgctgtagg tgaaacggga ttgtttgaac 516000
caggcttaga taccgagaca gtgctgctgc ctgaacctgt tgcagtactc ggaatctgtg 516060
gtatgactga tggattggga ttcaaaccgg tctgtggaac gtcacttaca ccaatcttgc 516120
cactgttatc gaatttgccg gcagatacca aatccaatgc ttgtgaacct gtttgagtaa 516180
tattacctgt gttatccaaa ccacctgttg acatatctaa gcgggcagcc tggatcgtac 516240
cgttgttttg gtttttcaga cggcctaaat tacgaacagt caatcgacct gaggataaga 516300
ccgtacctga attgtccagc gtctggctgt gaatattggc atcatcctgt gaggcaaccg 516360
taccgctatt atgaacattg cgggcatgaa gtgaaaccgc atgattttct cccgtcgctg 516420
caatcatgcc cgtgttgacc agtttaccct cagcattcac tgccacattg ccggctgagg 516480
caaaccattg cccttgatta cgaatgcctg cttgctcgac cgtactgatc aaggtgattt 516540
tgttggcata catacctcct aatttgcctg tatcaatcgc aaataaaggg atatgtgtgc 516600
cgttgttggc tgtattgttt gacgtattgg cagcagcatt attgagaata ggcgaatgtg 516660
catcacctgt tgcggccaca tcgttttgtc ccgcgacgac acgaacatct tgtccccata 516720
cgggtgcatc aattttggaa tgataactga gaatacgtgt gtaatcggta tcacgtgcat 516780
ccaaaccgtg tccggcgatt acaacattgc cttgccttat cttaaagccg ctaaggtctc 516840
ctgcttgata ttgcggttgg gctgtcgtca aagtggcacg ggaagcattg ataaaaccac 516900
caccattgac tgcaatccct gccggattgg caataacgac ttctgcacgt cgtccgccca 516960
cttcaatata gccattcagt tgtgaagaat ggctgctgtt gatttggttt acaaccacac 517020
gtgcttcgcc ccttgccaac caaggattgc cttgaatcca accgcctagc tgtgtttggg 517080
tgttgctgcg actgttgttt aaaatcgccc cgcgattacc cacatcaaac tgggcgtatt 517140
gattaacaga aacccctgcc gaagtagggg tttgaatatt gacttgcggt atgccgttac 517200
ctgtttgcag gatggtaggc tgttgctgtg caggtgcgga tttgtcggca acgatacctt 517260
gggcagtagc agaagaagaa gtcaggataa gggcagaacc gagcagtaat gaaagggaga 517320
atgagataac agagatagaa tggataaaac ccgcaaagcc cgcaatatca tttggcaaaa 517380

tacctacagc ttgggtgtcg gctgtgtttt tgccctcgcg tttggcattt tcagcaacgg 517440
ctatcatgca gtttcgatgt ttgttaaata caactttgta cagggtgcgg ttcatagtaa 517500
gggctttctt aataatattt ttataatcgt aaattagatt aatttttagg ggctgacgta 517560
gattaacagt tatgccaggc tacgaaaata aagataacca attgtaaatt aaacaataga 517620
gttcaaaaga aactgcttga atttttcgta ctccaagcta ccgcccgttc cgctgccgat 517680
attttgggta tggcgctgcg ggcaatttcc gttcccactt cggcgagttg gcgcataatg 517740
gaacgctcgc gcacgatttc ggcatggcgc cggatgttgg cggcagacgg agtattttgc 517800
gccagcgtaa tcagatattc gaatcccccc gccgcttcca gctcttcgtt ccgctgcaaa 517860
tcttcctgaa ccgtgatgac atcggcagga cggctctcat tgatcagttt ggcaatggat 517920
cggaaaatca ggcggtgttc gtggcggtag aaatcctctc ccgaaaccac atcggcaatc 517980
ctgtcccaag ccggattttc cagcatcaac ccgcccaaaa cggattgttc cgcctccatt 518040
gagtgcggcg gaagcgataa tgagccgatt cctccgtctt cagacggcat ggctgtgtaa 518100
tcgttcatgg tacatccgac aaaattgcaa tcttctattg tagcgtaaag caggttcaat 518160
tggtttccgt accgcaaaac aggtagaata cgcgagttgc cgggttaaat accttcctca 518220
accatcacag ttaacatagg aaataatttg gcaatctgag aatcggctat ccacctgttt 518280
gtcccttcag tcctaagcat acctgaatct ttaacccaaa ttgttccatc cttgtcctta 518340
aaacgtgtgc cattagaaat cttttcccat tcgtttaaaa cgacttttgc atttttgttt 518400
tcaggatttt tggccccatt atctttagcc acatcttcaa atccccaacg ttcctctacg 518460
gctttttttca gaatattcag cctatgggct ttagtcacgt tctgaccttt tgcaatgagc 518520
gaagcgatat atgcttccgc cctgacccgt atcgttccgg cttccaaatc agtcattccg 518580
gcaaaaagtt ccgattgatt ttcaagaggg atgtctttcg accctatttt atgtaggatt 518640
gagaatgtaa aacctacaat ttttcgtcct tctttatgct gctcgtaggt aatggaaata 518700
tccgttttat cattgatctg cttgacggcg aaatccaaaa ccttacgttt gaatagctcc 518760
attttttgat actcgtcagg catcataccc aaacgttcgc gcaactccat tgtactgaac 518820
atcggtgtct taccggctgc acgccatgaa ataataattt cgtagagccg caccgcgtat 518880
ttactgctca acgatgagac ctgatcaagc tcgtagcttg tgaagttttt ttctagcatc 518940
gtaatcaaag gggcaacatt tggtgcaaaa actaactcta ccgttgcctg ttgttcaata 519000
taggcgactt gagataccca ccttgtccgt actacctttt cccctttttgg tgttttttcg 519060
ataaaactga attggcgttc aaaaaggttg ttacaggcat ctttcaaagc cttatacgcc 519120
gtattacggt tggtatggaa attattaacg atgcagaact tatccgttcc atgcagcgtc 519180
agcagcacat agatgctgaa ttgttaactg atgcaaatgt ccgtttcgag caaccattgg 519240
agaagaacaa ttatgtcctg agtgaagatg aaacaccgtg tactcgggta aattacatta 519300
gtttagatga taagacggtg cgcaaatttt cttttcttcc ttctgtgctc atgaaagaaa 519360
cagcttttaa aactgggatg tgtttaggtt ccaataattt gagcaggcta caaaaagccg 519420
cgcaacagat actgatcgtg cgtggctacc tcacttccca agctattatc caaccacaga 519480
atatggattc gggaattctg aaattacggg tatcagcagg cgaaataggg gatatccgct 519540
atgaagaaaa acgggatggg aagtctgccg agggcagtat tagtgcattc aataacaaat 519600
ttcccttata taggaacaaa attctcaatc ttcgcgatgt agagcagggc ttggaaaacc 519660
tgcgtcgttt gccgagtgtt aaaacagata ttcagattat accgtccgaa gaagaaggca 519720
aaagcgattt acagatcaaa tggcagcaga ataaacccat acggttcagt atcggtatag 519780
atgatgcggg cggcaaaacg accggcaaat atcaaggaaa tgtcgcttta tcgttcgata 519840
accctttggg cttaagcgat ttgttttatg tttcatatgg acgcggtttg gcgcacaaaa 519900
cggacttgac tgatgccacc ggtacggaaa ctgaaagcgg atccagaagt tacagcgtgc 519960
attattcggt gcccgtaaaa aaatggctgt tttcttttaa tcacaatgga catcgttacc 520020
acgaagcaac cgaaggctat tccgtcaatt acgattacaa cggcaaacaa tatcagagca 520080
gcctggccgc cgagcgcatg ctttggcgta acagacttca taaaacttca gtcggaatga 520140
aattatggac acgccaaacc tataaataca tcgacgatgc cgaaatcgaa gtacaacgcc 520200
gccgctctgc aggctgggaa gccgaattgc gccaccgtgc ttacctcaac cgttggcagc 520260
ttgacggcaa gttgtcttac aaacgcggga ccggcatgcg ccaaagtatg cctgcaccgg 520320
aagaaaacgg cggcgatatt cttccaggta catctcgtat gaaaatcatt actgccagtt 520380
tggacgcagc cgccccattt attttaggca aacagcagtt tttctacgca accgccattc 520440
aagctcaatg gaacaaaacg ccgttggttg cccaagataa attgtcaatc ggcagccgct 520500
acaccgttcg cggatttgat ggggagcaga gtcttttcgg agagcgaggt ttctactggc 520560
agaatacttt aacttggtat tttcatccga accatcagtt ctatctcggt gcggactatg 520620
gccgcgtatc tggcgaaagt gcacaatatg tatcgggcaa gcagctgatg ggtgcagtgg 520680
tcggcttcag aggagggcat aaagtaggcg gtatgtttgc ttatgatctg tttgccggca 520740
agccgcttca taaacccaaa ggctttcaga cgaccaacac cgtttacggc ttcaacttga 520800
attacagttt ctaacctctg aattttttac tgatatttag acggtctttc cttatcctca 520860
gaccgtcaaa ctttacctac gtacttggcg cgcagtacgt tcatcttcaa aatggaatag 520920
acatgaataa aggtttacat cgcattatct ttagtaaaaa gcacagcacc atggttgcag 520980
tagccgaaac tgccaacagc cagggcaaag gtaaacaggc aggcagttcg gtttctgttt 521040

cactgaaaac ttcaggcgac ctttgcggca aactcaaaac cacccttaaa actttggtct 521100
gctctttggt ttccctgagt atggtattgc ctgcccatgc ccaaattacc accgacaaat 521160
cagcacctaa aaaccagcag gtcgttatcc ttaaaaccaa cactggtgcc cccttggtga 521220
atatccaaac tccgaatgga cgcggattga gccacaaccg ctatacgcag tttgatgttg 521280
acaacaaagg ggcagtgtta aacaacgacc gtaacaataa tccgtttgtg gtcaaaggca 521340
gtgcgcaatt gattttgaac gaggtacgcg gtacggctag caaactcaac ggcatcgtta 521400
ccgtaggcgg tcaaaaggcc gacgtgatta ttgccaaccc caacggcatt accgttaatg 521460
gcggcggctt taaaaatgtc ggtcggggca tcttaactac cggtgcgccc caaatcggca 521520
aagacggtgc actgacagga tttgatgtgc gtcaaggcac attgaccgta ggagcagcag 521580
gttggaatga taaaggcgga gccgactaca ccggggtact tgctcgtgca gttgctttgc 521640
aggggaaatt acagggtaaa aacctggcgg tttctaccgg tcctcagaaa gtagattacg 521700
ccagcggcga aatcagtgca ggtacggcag cggtcgcac tgggcggtat gtacgccgac 521760
agcatcacac tgattgccaa tgaaaaggc gtaggcgtca aaaatgccgg cacactcgaa 521820
gcggccaagc aattgattgt gacttcgtca ggccgcattg aaaacagcgg ccgcatcgcc 521880
accactgccg acggcaccga agcttcaccg acttatctct ccatcgaaac caccgaaaaa 521940
ggagcggcag gcacatttat ctccaatggt ggtcggatcg agagcaaagg cttattggtt 522000
attgagacgg gagaagatat cagcttgcgt aacggagccg tggtgcagaa taacggcagt 522060
cgcccagcta ccacggtatt aaatgctggt cataatttgg tgattgagag caaaactaat 522120
gtgaacaatg ccaaaggccc ggctactctg tcggccgacg gccgtaccgt catcaaggag 522180
gccagtattc agactggcac taccgtatac agttccagca aaggcaacgc cgaattaggc 522240
aataacacac gcattaccgg ggcagatgtt accgtattat ccaacggcac catcagcagt 522300
tccgccgtaa tagatgccaa agacaccgca cacatcgaag caggcaaacc gctttctttg 522360
gaagcttcaa cagttacctc cgatatccgc ttaaacggag gcagtatcaa gggcggcaag 522420
cagcttgctt tactggcaga cgataacatt actgccaaaa ctaccaatct gaatactccc 522480
ggcaatctgt atgttcatac aggtaaagat ctgaatttga atgttgataa agatttgtct 522540
gccgccagca tccatttgaa atcggataac gctgcccata ttaccggcac cagtaaaacc 522600
ctcactgcct caaaagacat gggtgtggag gcaggctcgc tgaatgttac caataccaat 522660
ctgcgtacca actcgggtaa tctgcacatt caggcagcca aaggcaatat tcagcttcgc 522720
aataccaagc tgaacgcagc caaggctctc gaaaccaccg cattgcaggg caatatcgtt 522780
tcagacggcc ttcatgctgt ttctgcagac ggtcatgtat ccttattggc caacggtaat 522840
gccgacttta ccggtcacaa taccctgaca gccaaggccg atgtcaatgc aggatcggtt 522900
ggtaaaggcc gtctgaaagc agacaatacc aatatcactt catcttcagg agatattacg 522960
ttggttgccg gcaacggtat tcagcttggt gacggaaaac aacgcaattc aatcaacgga 523020
aaacacatca gcatcaaaaa caacggtggt aatgccgact taaaaaacct taacgtccat 523080
gccaaaagcg gggcattgaa cattcattcc gaccgggcat tgagcataga aaataccaag 523140
ctggagtcta cccataatac gcatcttaat gcacaacacg agcgggtaac gctcaaccaa 523200
gtagatgcct acgcacaccg tcatctaagc attaccggca gccagatttg gcaaaacgac 523260
aaactgcctt ctgccaacaa gctggtggct aacggtgtat tggcactcaa tgcgcgctat 523320
tcccaaattg ccgacaacac cacgctgaga gcgggtgcaa tcaaccttac tgccggtacc 523380
gccctagtca agcgcggcaa catcaattgg agtaccgttt cgaccaaaac tttggaagat 523440
aatgccgaat taaaaccatt ggccggacgg ctgaatattg aagcaggtag cggcacatta 523500
accatcgaac ctgccaaccg catcagtgcg cataccgacc tgagcatcaa aacaggcgga 523560
aaattgctgt tgtctgcaaa aggaggaaat gcaggtgcgc ctagtgctca agtttcctca 523620
ttggaagcaa aaggcaatat ccgtctggtt acaggagaaa cagatttaag aggttctaaa 523680
attacagccg gtaaaaactt ggttgtcgcc accaccaaag gcaagttgaa tatcgaagcc 523740
gtaaacaact cattcagcaa ttattttcct acacaaaaag cggctgaact caaccaaaaa 523800
tccaaagaat tggaacagca gattgcgcag ttgaaaaaaa gctcgcctaa aagcaagctg 523860
attccaaccc tgcaagaaga acgcgaccgt ctcgctttct atattcaagc catcaacaag 523920
gaagttaaag gtaaaaaacc caaaggcaaa gaatacctgc aagccaagct ttctgcacaa 523980
aatattgact tgatttccgc acaaggcatc gaaatcagcg gttccgatat taccgcttcc 524040
aaaaaactga accttcacgc cgcaggcgta ttgccaaagg cagcagattc agaggcggct 524100
gctattctga ttgacggcat aaccgaccaa tatgaaattg gcaagcccac ctacaagagt 524160
cactacgaca aagctgctct gaacaagcct tcacgtttga ccggacgtac aggggtaagt 524220
attcatgcag ctgcggcact cgatgatgca cgtattatta tcggtgcatc cgaaatcaaa 524280
gctccctcag gcagcataga catcaaagcc catagtgata ttgtactgga ggctggacaa 524340
aacgatgcct ataccttctt aaaaaccaaa ggtaaaagcg gcaaaatcat cagaaaaacc 524400
aagtttacca gcacccgcga ccacctgatt atgccagccc ccgtcgagct gaccgccaac 524460
ggcataacgc ttcaggcagg cggcaacatc gaagctaata ccacccgctt caatgcccct 524520
gcaggtaaag ttaccctggt tgcgggtgaa gagctgcaac tgctggcaga agaaggcatc 524580
cacaagcacg agttggatgt ccaaaaaagc cgccgcttta tcggcatcaa ggtaggcaag 524640
agcaattaca gtaaaaacga actgaacgaa accaaattgc ctgtccgcgt cgtcgcccaa 524700

actgcagcca cccgttcagg ctgggatacc gtgctcgaag gtaccgaatt caaaaccacg 524760
ctggccggtg cggacattca ggcaggtgta ggcgaaaaag cccgtgccga tgcgaaaatt 524820
atcctcaaag gcattgtgaa ccgtatccag tcggaagaaa aattagaaac caactcaacc 524880
gtatggcaga aacaggccgg acgcggcagc actatcgaaa cgctgaaact gcccagcttc 524940
gaaagcccta ctccgcccaa actgaccgcc cccggtggct atatcgtcga cattccgaaa 525000
ggcaatttga aaaccgaaat cgaaaagctg gccaaacagc ccgagtatgc ctatctgaaa 525060
cagctccaag tagcgaaaaa cgtcaactgg aaccaggtgc aactggctta cgataaatgg 525120
gactataagc aggaaggctt aaccagagcc ggtgcagcga ttgttaccat aatcgtaacc 525180
gcactgactt atggatacgg cgcaaccgca gcgggcggtg tagccgcttc aggaagtagt 525240
acagccgcag ctgccggaac agccgccaca acgacagcag cagctactac cgtttctaca 525300
gcgactgcca tgcaaaccgc tgctttagcc tccttgtata gccaagcagc tgtatccatc 525360
atcaataata aaggtgatgt cggcaaagcg ttgaaagatc tcggcaccag tgatacggtc 525420
aagcagattg tcacttctgc cctgacggcg ggtgcattaa atcagatggg cgcagatatt 525480
gcccaattga acagcaaggt aagaaccgaa ctgttcagca gtacgggcaa tcaaactatt 525540
gccaaccttg gaggcagact ggctaccaat ctcagtaatg caggtatctc agctggtatc 525600
aataccgccg tcaacggcgg cagcctgaaa gacaacttag gcaatgccgc attaggagca 525660
ttggttaata gcttccaagg agaagccgcc agcaaaatca aaacaacctt cagcgacgat 525720
tatgttgcca aacagttcgc ccacgctttg gctgggtgtg ttagcggatt ggtacaagga 525780
aaatgtaaag acggggcaat tggcgcagca gttggggaaa tcgtagccga ctccatgctt 525840
ggcggcagaa accctgctac actcagcgat gcggaaaagc ataaggttat cagttactcg 525900
aagattattg ccggcagcgt ggcggcactc aacggcggcg atgtgaatac tgcggcgaat 525960
gcggctgagg tggcggtagt gaataatgct ttgaattttg acagtacccc taccaatgcg 526020
aaaaagcatc aaccgcagaa gcccgacaaa accgcactgg aaaaaattat ccaaggtatt 526080
atgcctgcac atgcagcagg tgcgatgact aatccgcagg ataaggatgc tgccatttgg 526140
ataagcaata tccgtaatgg catcacaggc ccgattgtga ttaccagcta tggggtttat 526200
gctgcaggtt ggacagctcc gctgatcggt acagcgggta aattagctat cagcacctgc 526260
atggctaatc cttctggttg tactgtcatg gtcactcagg ctgccgaagc gggcgcggga 526320
atcgccacgg gtgcggtaac ggtaggcaac gcttgggaag cgcctgtggg ggcgttgtcg 526380
aaagcgaagg cggccaagca ggctatacca acccagacag ttaaagaact tgatggctta 526440
ctacaagaat caaaaaatat aggtgctgta aatacacgaa ttaatatagc gaatagtact 526500
actcgatata caccaatgag acaaacggga caaccggtat ctgctggctt tgagcatgtt 526560
cttgaggggc acttccatag gcctattgcg aataaccgtt cagtttttac catctcccca 526620
aatgaattga aggttatact tcaaagtaat aaagtagttt cttctcccgt atcgatgact 526680
cctgatggcc aatatatgcg gactgtcgat gtaggaaaag ttattggtac tacttctatt 526740
aaagaaggtg gacaacccac aactacaatt aaagtattta cagataagtc aggaaatttg 526800
attactacat acccagtaaa aggaaactaa ctaaatatga gtaactttga aaaaaaatat 526860
attttagaat taaatgatgc tttaagccat ttaaatcata actctacctc atttgattta 526920
ttgaaagttt tgatttcatg gttatcaaac gatattgtca ttgataaatt taaaatttta 526980
ggttatgact ttagtaaata tatcgaaatg aatcccgatg actatccggt tgaaaaatct 527040
atattgaata gagaggaaat tatttatctc aaaaacaata tttatcgtaa aatatcatca 527100
ggaaatttta aatttcaata ctttgtacaa tatattagag atattttaga atatttattt 527160
attgaacata ttgaaagagt ctgtccttac tgcgaatggg gtgaaatgca aaaattagaa 527220
gaacaaaata cgcatgaaac ggtgtatctc tgtactcaat gtggatgtgc tttttataac 527280
gataattcac aatttttatt aaaaacccct ttaaccattc caatgaaacg tgatgaattt 527340
aaataaacaa gccgtagcct gcatgaaccc taaaatccac gtgtagcgtg tgtgcgccag 527400
cacgcatgcg ttccatgatt tacggctcaa tgccgtctga aaagctcaca atttttcaga 527460
cggcatttgt tatgcaagta aatattcaga ttccctgtat gctgtacaga cgcgggagtg 527520
ttaagccccc cttgtttgaa gctccgcggc tcctgccgag cttcaccgac cccgttgtgc 527580
ccaagctctc tgctcccggc ggctacattg tcgacatccc caaaggcaat ctgaaaaccg 527640
aaatcgaaaa gctggccaaa cagcccgagt atgcctatct gaaacagctc caagtagcga 527700
aaaacgtcaa ctggaaccag gtgcaactgg cttacgataa atgggactat aagcaggaag 527760
gcttaaccag agccggtgca gcgattatcg cgctggctgt taccgtggtt actgcgggcg 527820
cgggagtcgg agccgcacta ggcttaaacg gcgcagccgc agcagcggcc gatgccgcct 527880
ttgcctcact cgcttctcag gcttccgtat cgctcatcaa caataaaggc gatgtcggca 527940
aaaccctgaa ggaactgggc agaagccgca cggtaaaaaa tctggttgta gcggcggcaa 528000
cggcaggcgt atccaacaaa ctcggtgcct cttcccttgc cacttggagc gaaacccctt 528060
gggtaaacaa cctcaacgtt aacctggcca atgcgggcag tgccgcgctg atcaacaccg 528120
ctgttaacgg cggcagcctg aaagacaatc tggaggcaaa tatcctggcg gcattggtga 528180
ataccgcgca tggggaggcg gcgagtaaga tcaaaggact ggatcagcac tatgtcgccc 528240
acaaaatcgc tcatgccgta gcgggctgtg cggctgcagc ggcgaataag ggcaaatgtc 528300
aggacggcgc gatcggtgcg gctgtgggtg agattgtcgg ggaggctttg gttaaaaata 528360

ccgattttag cgatatgacc ccggaacaat tagatctgga agttaagaaa attaccgcct 528420
atgccaaact tgcggcaggt acagttgcag gcgtaacggg aggagatgtc aatactgctg 528480
cacaaaccgc acaaaacgcg gtagaaaata atgcggttaa agctgttgta actgctgcaa 528540
aagtggttta taaggtagcc agaaaaggat taaaaaacgg gaaaatcaac gttagagatt 528600
taaaacagac gttgaaagac gaaggttata atttagccga caacctgacc accttattcg 528660
acgaaacatt ggattggaac gatgccaaag ccgttattga tattgtcgtc ggaacagagc 528720
tgaatcgcgc taataaaggg gaagcggcac aaaaggtcaa ggaagtttta gaaaaaaatc 528780
gtccttatat ccctaataaa ggtgctgtac cgaatatgag tacatacatg aaaaataatc 528840
cttttggaaa acagctggct caaatttcag aaaagacaac gcttccgacg cagcaagggc 528900
agtctgtctt cttggtaaaa agaaaccaag ggttattaaa aaccggtgat aggttttatt 528960
tagatggcca acataaaaat catttagagg tttttgataa aaatgggaac tttaagtttg 529020
ttctaaatat ggatggttcg cttaaccaaa tgaaaactgg ggcagcaaaa ggtcgtaaat 529080
taaacttaaa ataggaaatt ttatggaaac attgaatgat ataaaaaaaa tcttgattaa 529140
tgtggggctt tatcaagggt ttgatttgac agatccaaaa gtatcagaag aagttaatca 529200
tgaaacagct aatatgaaat ggattaaaga ttatacttca gacgggaatt gggataatga 529260
atttaaggag gatttaaaaa actttttaga ttatatggaa gtatgccaat tagccctaaa 529320
cgataaaaat ttcaaaattg ccagtaattc tttatttatg gctatgattt acgcaggtaa 529380
tctatctctt atatttgatt caataaaaac tgatatatca acattattga gtgctgagta 529440
taaaaagaat agtttttcat ggccatctct tgatgaatag aaagcaagtt gtagcctgca 529500
tgaaatctaa aacccatgca taaggtgtgg gcttcagtat acgcgttcca tgatttacgg 529560
ccatatgccg tctgaaaagc tcaatttttt cagacggcat ttgttatgaa agtaaatatt 529620
tagattccct gtatactgtt tagactcgtg tgtgctgagt aagctgtagt ctgcatgaaa 529680
cctaaaactc gctcaaaatt aagctaagac attagcaggg caagggcgaa aattgaatct 529740
taaataaggt gattcagatg aaaaatttta atgtagtaaa agaaagttta agagagttag 529800
gaattaaaca aggatttgat ctttatgaga aagccacaac tgaaaaattg aatagtgaag 529860
atcctcttga cttacaatgg ctttctaact attcatctga ttggaatgat gaattagaag 529920
aagactttga ttcttttttt cagcatatga aggaatatca atatgctatt gacaatgaag 529980
acattaaatc tgcatgtagt tcactatgtg aagctatgct ctatgttggt aatattaaaa 530040
attttttga gtttctcaaa agcgatatga ttagactgtt gagaggtgaa agtaaaacaa 530100
cagactttca atggccgcaa tttgatgaat agcagcaagc tgtagcctgc atgaaaccta 530160
aaatccatgc gtaaggtgtg tgcttcagca cgcacgcgtt ccatgattta cggctcaatg 530220
ccgtctgaaa agctcacaat ttttcagacg gcatttgtta tgcaagtaaa tattcagatt 530280
ccctatatac tgcccagatg cgtgcgtgct gaagacaccc cctacgcttg ctatttgaaa 530340
cagctccaag tcaccaaaga cgtcaactgg aaccaggtac aactggcgta cgacaaatgg 530400
gactataaac aggaaggctt aaccggagcc ggagcagcga ttattgcgct ggctgttacc 530460
gtggttactg cgggcgcggg agccggagcc gcactgggct taaacggcgc ggccgcagcg 530520
gcaaccgatg ccgcattcgc ctcgctggcc agccaggctt ccgtatcgct catcaacaac 530580
aaaggcaata tcggtaacac cctgaaagag ctgggcagaa gcagcacggt gaaaaatctg 530640
atggttgccg tcgctaccgc aggcgtagcc gacaaaatcg gtgcttcggc actgaacaat 530700
gtcagcgata agcagtggat caacaacctg accgtcaacc tggccaatgc gggcagtgcc 530760
gcactgatta ataccgctgt caacggcggc agcctgaaag acaatctgga agcgaatatc 530820
cttgcggctt tggtgaatac tgcgcatgga gaagcagcca gtaaaatcaa acagttggat 530880
cagcactaca ttacccacaa gattgcccat gccatagcgg gctgtgcggc tgcggcggcg 530940
aataagggca agtgtcagga tggtgcgata ggtgcggctg tgggcgagat agtcggggag 531000
gctttgacaa acggcaaaaa tcctgacact ttgacagcta aagaacgcga acagattttg 531060
gcatacagca aactggttgc cggtacggta agcggtgtgg tcggcggcga tgtaaatgcg 531120
gcggcgaatg cggctgaggt agcggtgaaa aataatcagc ttagcgacaa agagggtaga 531180
gaatttgata acgaaatgac tgcatgcgcc aaacagaata atcctcaact gtgcagaaaa 531240
aatactgtaa aaaagtatca aaatgttgct gataaaagac ttgctgcttc gattgcaata 531300
tgtacggata tatcccgtag tactgaatgt agaacaatca gaaaacaaca tttgatcgat 531360
agtagaagcc ttcattcatc ttgggaagca ggtctaattg gtaaagatga tgaatggtat 531420
aaattattca gcaaatctta cacccaagca gatttggctt tacagtctta tcatttgaat 531480
actgctgcta aatcttggct tcaatcgggc aatacaaagc ctttatccga atggatgtcc 531540
gaccaaggtt atacacttat ttcaggagtt aatcctagat tcattccaat accaagaggg 531600
tttgtaaaac aaaatacacc tattactaat gtcaaatacc cggaaggcat cagtttcgat 531660
acaaacctaa aaagacatct ggcaaatgct gatggtttta gtcaaaaaca gggcattaaa 531720
ggagcccata accgcaccaa ttttatggca gaactaaatt cacgaggagg acgcgtaaaa 531780
tctgaaaccc aaactgatat tgaaggcatt acccgaatta aatatgagat tcctacacta 531840
gacaggacag gtaaacctga tggtggattt aaggaaattt caagtataaa aactgtttat 531900
aatcctaaaa aattttctga tgataaaata cttcaaatgg ctcaaaatgc tgcttcacaa 531960
ggatattcaa aagcctctaa aattgctcaa aatgaaagaa ctaaatcaat atcggaaaga 532020

aaaaatgtca ttcaattctc agaaaccttt gacggaatca aatttagatc atattttgat 532080
gtaaatacag gaagaattac aaacattcac ccagaataat ttaaaggaaa aattatgaaa 532140
aataatattt ttctaaactt aaataaaaaa tctataaata acaaccattt tgttatttcg 532200
attttttttg aaacaattta ccaatttgaa actaaagata cgcttttaga gtgttttaaa 532260
aatattacaa ctaccggaca ttttggagta ataggtgctc aatatgaaaa aatagatgct 532320
accagatgga ttggagatta tgaagaggta aatggatttg agtatattga taaagctcct 532380
tctatttatt tttcagttgg agatgatttc aatcctgaag aattaattat acctattaat 532440
ttagcatatc attactttaa tattgcaata tctgatttct taatagctca ccctgaatat 532500
caaaaaaagt gtaaagaaat acaaaaaaca tattctcaaa caaactgtag cctgcatgaa 532560
acctaaaatc catgcgtaag gtgtgtgctt cagcacgcac gcgttccatg atttacggct 532620
caatgccgtc tgaaaagctc acaatttttc agacggcatt tgttatgcaa gtaaatattc 532680
agattcccta tatactgccc agacgcgtgc gtgctgaaga caccccctac gcttgctgca 532740
gaactttcgg gtaaaaccgg tgtgagcatt agcgcaccgt atgccaatga gaacagtcgc 532800
atcctgctca gcaccacgga tatcagttcg gaaaacggca aaatcaaaat tcaatcttac 532860
ggtgaccaat attactatgc gagacagagc gaactctata cctttgaacg ccgcagctac 532920
aaaactggca aatggtacaa ccgcaaacac attaccgaag tcaaagaaca caaaaacgcc 532980
aagcccgacg cagtaaccct cagcgcatcc caaggcatcg acatcaaatc tggtggcagc 533040
atcgacgcct acgccaccgc attcgatgcc cccaaaggca gcattaacat cgaagccggg 533100
cggaaattga cactctatgc cgtagaagag ctcaactacg acaaacttga cagccaaaaa 533160
aggcgcagat ttctcggcat cagctacagc aaagcacacg acaccaccac ccaagtcatg 533220
aaaaccgcgc tgccctcaag ggtagttgca gaatctgcca atctgcaatc aggttgggat 533280
accaaactgc aaggcacaca gtttgaaacc acactgggtg gcgcaaccat acgcgcaggc 533340
gtaggcgagc aggcacgggc cgatgccaag attatcctcg aagggatcaa aagcagcatc 533400
cacacagaaa ccgtgagcag cagcaaatct actctatggc aaaaacaggc aggacggggc 533460
agtaacatcg aaaccttgca attgccgagt ttcaccggtc ccgttgcgcc cgtactgtcc 533520
gcacccggcg gttacattgt cgatattccg aaaggcaatc tgaaaaccca aatcgaaacc 533580
ctcaccaagc agcccgagta tgcttatttg aaacaacttc aagttgcgaa aaacatcaac 533640
tggaatcagg tgcagcttgc ttacgataaa tgggactaca aacaggaggg catgacaccc 533700
gcagcagcag ctgtcgtcgt tatcgtcgta accgtattga cctacggcgc actgtccgcc 533760
ccggcagccg ccggaacggc gggcgcggca ggcgcaggag cgggaggagc cgcagcagga 533820
acggcagccg gaactggagt agcagcagga acggcagcca caaccggagt agcagcaggc 533880
acatcagctg cagctatcac cacagccgca ggcaaagccg cactggccag tctcgccagc 533940
caagccgcag tttccctcat caacaacaaa ggagacataa accataccct gaaagaactg 534000
ggcaaaagca gcaccgtcag acaggccgcc accgccgccg taaccgcagg cgtactgcag 534060
ggcataagcg ggctgaacac ccaagcagcc gaagccgtca gcaaacattt tcacagtccc 534120
gcagcaggca aactgaccgc taacctgatc aacagcaccg ctgccgcaag tgtccatacc 534180
gccatcaacg gcggcagcct gaaagacaac ttgggcgatg ccgcactggg tgcgatagtc 534240
agtaccgtac acggagaagt agcgagcaaa atcaaattta atctcagcga agactacatt 534300
gcccacaaga tagcccatgc cgtagcaggc tgtgcatcgg cggtagcaaa taaaggcaaa 534360
tgtcgggacg gcgcaatcgg cgcggcagtc ggcgagatgg tgggagaaac cctgttggac 534420
ggacgcgatg taggcaaact gtcaccccaa gaacgccaaa aagtcatagc ctactcgcag 534480
attatcgcag gcagcgcagt ggcattggtt aaaggggatg tgaatacggc ggcgaatgcg 534540
gctactgtgg cagtggagaa taatagtctt ttagctcgca ggagggtaaa tatacgttgg 534600
acttcgcgac aagaattgga acatgaatat gccattcttg aaatccaggc cattaccaat 534660
caaatccgaa ggctggatcc gaaatttaac gggattgcta ttatgaggaa tcctagagag 534720
ccgtggacaa gacatgatgt acaaacatac aggcaatatt ataatcaatt aagggaatcc 534780
agaggctttg ctgttgaccc aatttataga atcaggataa acaacggcaa tgaatttaac 534840
cgtatcatgt catcaaaata cccttataat gagctttatg tagccaatcc taaatcggcg 534900
acggggtatt ttagggtaga ttcgtataat cctgcgacag aggaaattat ttcaagaaaa 534960
tttacccaat tttctcaaat ccaagaaagt acggggattg gttatatcaa ggaggctgtt 535020
agaaaatata gccctggtgc tgtcatttcc aatgttccaa gtacacctac tacgataaga 535080
ggaagaaagc ttgaaggaaa acttatttta gaagttcctg ctcaggtcaa tccaattcca 535140
caatctgtat taagggcggc acaagaagaa aatgttatca ttagagatac aacaggaagg 535200
atttacaaat gaagaaagat atttttttatt gtgagcagtg gtcttatggt tataagaaac 535260
ttcataagcc tttttctgag aaacaagctg aggaaaaaca tcttaaaggg gagttatata 535320
ctgccgtaat aggttcggcg acacaacctg aatatgtaat taccttgcga gaggaagtag 535380
gtttttttc ggtacatttt ttcgataaat ttggaaggga ttatttaacc catcaatttc 535440
aaaaatattc caattcgaat tattattttc tttctatggc tgtatggaga gattatataa 535500
ctttggaatc tcatgactta gcagaaggat atacttattt cttcaatgaa aatacggatg 535560
attgctatgt tttgaaagag gattttatta ataatgagcg atatgaaaaa acagaattat 535620
attcccaaaa agataaggta attctatttc caaagtttgg cgaatatgat ttggtgttaa 535680

atccggacat tatttaattg agttttaagg ccgtctgaaa aaatttcaga cggcttttat 535740
tattgggttt ggaatctgag gataaagctg ataaaaacca ggaaattatc aggttgctat 535800
atacgtattg ttgtacagac taaaggcagc aatcaaatca ctactgctta cccacaaaaa 535860
taaatcgatt atatggagta atcatgaata agagaatgaa aatgtgtcct gcttgtcaac 535920
aaggctatct ctaccattcg aaacctaaat atcttcatga tgaaattatt ctgtgtgatg 535980
aatgcgatgc agtatggctc aaaggtatga atatatttta tggagaatat gaaaaagatt 536040
tttattctta tgttcctttc atggaatccc aaggtataac gagtgaatgt atttgggaag 536100
gagatttgtt tgatcatcca tattatgaag atgaaaactc aaatgatatg gattgatgga 536160
aattttaagc ctgcgtaggt acgattagcc atcaaacggc gtaatcatac gcaagattat 536220
caacagagag ggctggcagc gatataccac ccacaagatt gcccatgcca tagcgggctg 536280
tgcggcagcg gcggcgaata agggcaagtg tcaggacggc gcgattggtg cggtcgtggg 536340
ggagattgtc ggggaggctt tggttaagaa taccgatttc agcggtatga ctgcttctga 536400
aattgaaaaa gctaaagcga atattactgc gtatgcaaaa ttggtagccg gagcgactgt 536460
aggtgttaca ggaggcaatg ttgatgtggc ggcaaatgct tccgaaacag ctgttaaaaa 536520
taatgcatta gatattattt gggatattgg caacctcgta tgggacggcg gtaaatggat 536580
ttacgccaaa tctattggcg ataagcagat ggctcgagaa gcggcgattg attttggtgt 536640
ggatgccgcc gcagctgccg ttccctttgt tccggcaggt gcgactaaaa tcagccgagg 536700
cggggcttat gttctgaagg cgggagacga agcagttgat acggctaaag ccatacagga 536760
aattcagaag cagaccggaa tcaagcttac ttatgataag gttaataagg tttggacaac 536820
accggcgggg ttagattatg ggttagatgc taagcatggt aataggatta aacatgtttt 536880
agcccataca attccaaatc caaacaaacc tgttcattct gtttttaatg tgtcccgtaa 536940
agaagttttg cctttggttg atgaagcttg gagaatgaaa ggaaatcctt tgccaaatga 537000
ttcatccgta tatcttgtag atatgaagaa acctattgga acaaaaggag aaacaaaagt 537060
gcggattgtt gtgcaaaaag gaacaaataa aatcatttct gcatatcctc agaaataatt 537120
aagaaaggaa tctcttatgg ataaagaaat taaaatttgc ccaagatgtg agcaaggcta 537180
cctttatcat gcaaagccta aatatttctc tggggaggtc attttatgcg atgaatgtta 537240
tgctatgtgg cttggggata tgaaaatttt ttacggacaa tatggaaaag atttttatga 537300
ttatcatgag tttatgaaag ataaaggcat agaagaaata aatatgtggg aaggagagct 537360
ttttgatcac ccatattatg aggatgaaaa atttaaataa ttgattttct gttccccgaa 537420
tttgggaaat acgatgatat tttaaaccca aatattattt aaagtagcaa taggccgtct 537480
gaatatccgt ttttcagacg gcctcaatgc aactgctggc agccgaaggc attcaccaac 537540
accaattgaa tgttcagaaa agtacccgtt tcatcggcat caaagtgggt aaaagcaatt 537600
acagcaaaaa cgagctgaac gaaaccaaac tgcccgtacg cgttatcgcc caaacagcca 537660
aaacccgttc cggctgggat accgtactcg aaggcaccga attcaaaacc accctttccg 537720
gagccgacat acaggcaggg gtgggtgaaa aagcccgagc cgatgcgaaa attatcctaa 537780
aaggcatcgt taaccgcatc caaaccgaag aaaagctgga atccaactcg accgtatggc 537840
aaaagcaggc cggaagcggc agcacggttg aaacgctgaa gctaccgagc tttgaagggc 537900
cggcactgcc taagctgacc gctcccggcg gctatatcgc cgacatcccc aaaggcaacc 537960
tcaaaaccga aatcgaaaag ctggccaaac agcccgaata tgcctatctg aaacagcttc 538020
agacggtcaa ggacgtgaac tggaaccaag tacagctcgc ttacgacaaa tgggactata 538080
aacaggaagg cctaaccgga gccggagccg caattatcgc actggccgtt accgtggtca 538140
cctcaggcgc aggaaccgga gccgtattgg gattaaacgg tgcggccgcc gccgcaaccg 538200
atgcagcatt tgcctctttg gccagccagg cttccgtatc gttcatcaac aacaaaggca 538260
atatcggtaa caccctgaaa gagctgggca gaagcagcac ggtgaaaaat ctgatggttg 538320
ccgtcgctac cgcaggcgta gccgacaaaa tcggtgcttc ggcactgaac aatgtcagcg 538380
ataagcagtg gatcaacaac ctgaccgtca acctggccaa tgcgggcagt gccgcactga 538440
ttaataccgc tgtcaacggc ggcagcctga aagacaatct ggaagcgaat atccttgcgg 538500
ctttggtgaa tactgcgcat ggagaggcag caagtaaaat caaacagttg gatcagcact 538560
acattgccca taagattgcc catgccatag cgggctgtgc ggcagcggcg gcgaataagg 538620
gcaagtgtca agatggtgcg atcggtgcgg cggtcggtga aatccttggc gaaaccctac 538680
tggacggcag agaccctggc agcctgaatg tgaaggacag ggcaaaaatc attgctaagg 538740
cgaagctggc agcaggggcg gttgcggcgt tgagtaaggg ggatgtgagt acggcggcga 538800
atgcggctgc tgtggcggta gagaataatt ctttaaatga tatacaggat cgtttgttga 538860
gtggaaatta tgctttatgt atgagtgcag gaggagcaga aagcttttgt gagtcttatc 538920
gaccactggg cttgccacac tttgtaagtg tttcaggaga aatgaaatta cctaataaat 538980
tcgggaatcg tatggttaat ggaaaattaa ttattaacac tagaaatggc aatgtatatt 539040
tctctgtagg taaaatatgg agtactgtaa aatcaacaaa atcaaatata agtggggtat 539100
ctgtcggttg ggttttaaat gtttccccta atgattattt aaaagaagca tctatgaatg 539160
atttcagaaa tagtaatcaa aataaagcct atgcagaaat gatttcccag actttggtag 539220
gtgagagtgt tggtggtagt ctttgtctga caagagcctg cttttcggta agttcaacaa 539280
tatctaaatc taaatctcct tttaaagatt caaaaattat tggggaaatc ggtttgggaa 539340

gtggtgttgc tgcaggagta gaaaaaacaa tatacatagg taacataaaa gatattgata 539400
aatttattag tgcaaacata aaaaaatagg agttagtatg aaatatatga ttagttttct 539460
aaaaaaaaca tttgaattaa tgagttgggt gttagtcata ctaataattg ggacatttta 539520
tgactattat caaataaggc aatatgctga attagaaaag aaatctatat caaatatctt 539580
gctatatgcc caaaaagaaa aatttcgctt agagagtaaa gataaataca tgcgaggagg 539640
atatacaaaa tataaattta ttttttcaga atatagtaat actacttttt taaatttcat 539700
aaatgacctg aaaaaagata attatttacc acttgacggc tatggacatg gttttctatg 539760
tagaaaagga gagtctatat caatcaatat ataccctgaa attaataaat ttattttagt 539820
atggggatac cctgaaaatc tttgcgctga ttctaattaa gtaagcaaga ataggttata 539880
gggaaataaa attcaaatga gaaaattgaa taatcacgat gttcataaac ggtatcaaga 539940
tcgtttagaa gaggatgtag agttcactat caactatgag cttcctttga gttgtttgtg 540000
gtcaaccatc aaagactttt ccagcgattt tgaggaaaaa actgaagcgt tctttattct 540060
tttcaaagag ctgctgcgca gaggtcatct gaaactgcaa cgcgacgggc aaattatcgg 540120
gcatacgccc gaagaatggg aacaaatatt tagggaagta tggcctgaat atgaaatcga 540180
acccaatcca cttcccggct atgccccatt tgatattgga atgtggctta cggtcgaggc 540240
tcctgcctac gccgtatgga tagatcccga agacggtagc gaatactggg cgggataaaa 540300
taccaatgtt tggaataaat cccgtctgaa aaacagcttt ttcagacagg atttattcca 540360
attatcggtg atatacagag ttttgtacaa gcacagaccg ctgccgatca cctgtttgct 540420
ttgctgggtg tggttccggg tatcggtgaa tcgatacagg cctataaagt agcgaaagcg 540480
gcaaaaaatt tacaaggcat gaaaaaagcc ttggacaagg cagcaaccgt tgccactgca 540540
cagggctatg tcagtaaaac caaaatcaaa atcggtcaaa ctgaattaag ggttactgca 540600
gcaactgaca aacaattgct gaaagctatt ggcgaaggaa ggacacgaca ggtaaaatga 540660
ccgagcagtt atttgactct ttagctaaac aaaatggctt cagagtgctt tcgggcggca 540720
aatacggcgg aaataacggt tttgatcatg tatggcaggc tgccgatggt agtgttgttt 540780
tgattgtaga aagtaagcag attaggaacg gtacggtaca gctgaatccg aatggtgcgg 540840
gtggatatac gcagatgagt cgtgaatgga ttaaacaagt tgtaaaaagt ttacctgatg 540900
gtagtcctgc taaggcagtt gtcttaaaag caaatcagaa cggcaaatta aaaacggcaa 540960
tagcaggcgt tgatcgtcaa acaggtaagg ccgttattct ttctgtcaaa gttccttcta 541020
aaaccaatat aaggagataa caatggggca caatatgatg accacccaaa aatggtatga 541080
acatattact aatgtaatca taggcaatac tgctaatttc aatagcggtt gccccgaatc 541140
tatagattat gtagatgaaa aaaaaggcgt gccgcttgca gcgatgaaat acattttaat 541200
gtacactgaa gctgcggctt cccatgccta tctatttgaa catgatctta agaaattcaa 541260
gcaatatgct tatgttgcag gaaagttggg tattttgcag agtgtagatg atgaagaccc 541320
cgaacccttc ttctttccct gcgacatgct caacattcaa gatccgatgt ttctgatgct 541380
gatgagcgac agcccgcagc tgcgcgagtt tttggtgcgc aatatcgaca acatcgccaa 541440
cgatacagaa gccttcgtaa accgatacga cctcaaccgt catatgattt acaatactct 541500
gctgatggtg gagggtaagc agcttgatcg gttgaaacaa cgtagcgaga aagtcttggc 541560
gcatcccacc cctagcaaat ggctgcaaaa gcggttgtac gattaccgct tcttcctcgc 541620
tttcgccgaa caggatgccg aggcgatgaa ggccgcctta gagccgcttt ttgataaaaa 541680
aaccgcgcgt atggctgcca aagaaacatt gtcctatttc gatttctacc tgcagccgca 541740
aatcgttacc tacgccaaaa tcgcatccat gcacggtttc gatttgggca tagaccacga 541800
aatcgcgccg agggatttga ctgtttacga tccgctgccg gcagacgaat atcaagacat 541860
cttcgatttt atgaaacagt atgacttgtc ttatccgtat gaatatctgc aggattggat 541920
agattactat acgttcaaaa ccgataagct ggtatttggt aacgcgaagc gagagtgagc 541980
cgtaaaactc tgagctcctg ttttatagat tacaacttta ggccgtctta aagctgaaag 542040
attttcgaaa gctataaatt gaagcccttc catagtacat agatctgtgt tgtggcgagg 542100
ctttaccacg ctgattgccg gagaagaact caacctgctg gcaaaacaag gcatgagatc 542160
tttgcaataa catgagttga gacctttgca aaaaagccct tccccgacat ccgaaaccca 542220
aacacaggat ttcggctgtt ttcgtaccaa atacctccta attttaccca aatatcccct 542280
taatcctccc cggatacccg ataatcaggc atccgggctg ccttttaggc ggcgcgggcg 542340
cacttagcct gttggcggcc ttcaacaggt tgagaccttt gcaataacat aggttactaa 542400
aattttatgc tcaatctcat tttcaaaatg caaaactttt ctgatttttc ctactttttg 542460
ctcaatatta ggaaggtttt aggcaattga aaattttttg gcgcattttt atgcgtcaaa 542520
tttcgttaac agactatttt tgcaaaggtc tcaggttcaa acacatcgcc ttcaggtggt 542580
ttgcgtactc actttgtcat ttccaatgtt ccaagtacac ctgctccgct aagaggaaga 542640
aaacttacag gaaaacttat tttagaagtt cctgctcagg tcaatccaat tccacaatct 542700
gtattaaggg cggcacgaga agaaaatgtt atcattagag atacaacagg aaggatttac 542760
aataggtggt ggtttagtat taggtggttg tgcaggtgca catcttgcaa gaaaagaacc 542820
attgatacta acagggaaaa caggggcagg tgcgtcagca attgcaaatg caagcattgg 542880
atatcaatgg actgtcaatt tgtcaaagcc aaaagaagga gctaaataat aatgcattcc 542940
cactatatat ttggtatttt gatgatttca tatgttttcg caatgttatt taattttata 543000

atatcatata aaatatttaa agaagaaaaa ttaattaatg gtttttttga ttttctaatt 543060
aaatcaagct accttaactt taaatatttc aatatattat ttggaaaata aaaaatctca 543120
aatatttttt atttgaaatt attaagaatt aatctggcgt tgggggtttt tatcttatcc 543180
ttaataatta taaatatttt ttgtttttag taaaaatatg gtacagatat gtacagctag 543240
ctttgtttca gtaaggtata actgtatata atactcagat ttttcacgtt gggctataca 543300
tggaaatata tctgtgatta aagatgttaa tggtaagtat cgattagcac ctgaaaagca 543360
tgattttaaa atgcattcct ttggggggag aaaaaagtaa tgtaaaaaca atatttagaa 543420
atatggaaac tataattggt agcccagggt aaggggtacc tttcaggatt gaatttaaag 543480
gagaggtaaa tattgttaac taagttgaaa attttgctat ttttgttctt atttgttttt 543540
gtattggcta ttaatttgct tttcttcttt tttagttcgg atatcgagag tttcgggaac 543600
tatcagtttg aatatgttta cgataaaggt tggcctgcta attatatttt agtcatgaaa 543660
gatggaaatg aagggaattt tgataaaata atatccggat tggttttaga atattataag 543720
gaggatgata acatttattt ttcttatatt gacgggcaag gatttgcttc agactcttgc 543780
tattacaaac cggaaatttt atatggaaaa attattttaa ataaaaatca tatcattaat 543840
attaatagca tggaaaaaaa taattttctt tcagaagata aaataatgaa gggaacaaga 543900
aattggctag cagaccctaa aaataaatgt aatatacaga ctctagacta aacgcgtctt 543960
gcgaaaatac aacggaatcg atcgtaaatc tttcccgctg ttcttgaaag aatgcgaatt 544020
tcgatttaac ttcggcacac cgtctcaaca gcttaaaatc ctgcgggatt ggtgtgggat 544080
ttagggctaa tctagtacag ccccttgttt tttcgatacg gaaccggata gaggaaaaat 544140
cgaacattgc gcctgccttg ctatgattca cgaagaaatc tccgccatgc ctatgggcta 544200
tgaaaccttg atcggcgata tgggcagcgc actgtcaggc ggacaaaaac aacgcatcgt 544260
attggcgcgg gccttaatat tgcgaaccga aaatcctatt tttagatgca gcgaccagcc 544320
atttggatat tgccaatgaa aaagcagtca atgcaaactt gaatggcttg tctatcataa 544380
aaattatggc ggcacacaga aaggaaacgg tggaatcagc agataggaaa atgtctttag 544440
gataaaaata cagtttcaaa aatactcaag actactgccg ttttttcgcc tgagcgtcaa 544500
actctgccag cgtcatgttc aaagtctgca aacacggtgt cattaccgca tcgacagctt 544560
ggttcacatg atccctttcc acaggcaacg gacggtaaac gaagagcttg aagagttcgt 544620
tcaactcaat cgaatccgcc cccgttttca acacccaacc ctgtctgccg gaatagatgt 544680
agccgtgccg cgccagcttt tccaaaagct cgcccaactc gtcgtagccc atattgatat 544740
gccgtctgaa ctcctgaaca ggcaaggctt tgccttcttt ttgcgccgca tccagaagca 544800
gcaggatttt caacacgtcg tcaaaccgtc cgcgcgagtc gaagcccctg cggaacgctt 544860
ctccctgcca gtaggagagt gaagaagtca gcaccgcgcc gcccaagacc agcgtccaca 544920
acaggttcag ccacaacaga aaaaacggca cggcggcaaa cgcgccgtaa atcgagcggt 544980
agccgtcgaa attgcccata taccaagtga agagggagcg cgcggtttcc agacaaaacg 545040
ctgttgccaa agccccgaca aacgcctgcc gcgcgggaac gaagcggttt ggcacgaagc 545100
ggtacagccc ccacagcaaa agcgtcatga aggtcagcgt cgccgccgtt cgcaacgcgc 545160
ccgaccactg cggcgcacct gaggcaagcg cggcatcctg taccgagccg accataaagg 545220
aaatgcccac gcccaaagac agcggcccga acgtcagtaa agcccaatag acgagaaact 545280
gcatcatcca cggacgctgg gaattgaccc gccagatgcg gttgaacgta ttgtctatcg 545340
tccgaatcag catcagcgag gtaacgacca gcatcacgct gccgattgcc gtcagccggt 545400
tcgcctgctc gcggaacgca ttgatatagt cgaacaccat gtccgcgccc tgcggcacaa 545460
tggtttggtt gacgaaggag acgaacgaat ccgaccagcg gtcgaacacg gggaaaatcg 545520
aagcgaccgc caccatcacg gtcagcacgg ggacgagtgc cagcagcgtc gtaaacgtca 545580
tgcttgccgc cgcctgcggt acgcgttctt catcaaagcg gcggacgacg aaccatgcaa 545640
acgcacagat tttattgtct gccaaacctt gcaaacgttg taaaaaggtc ataatttctt 545700
gcccggtcag taagttgggc attgatgccc gatgttatag ccaattttgc cgtcaggaac 545760
aaatgcctga actgcggctg tttcagacgg catcggaaca actgttatgc cgtctgaaga 545820
ccgaaccatt ttaacggaat ccgcccatga acccaaatcc ccctcaaaat cctcgtcctc 545880
tactatttcc aaaacggcag cacccgcaat cccgcactcc gaatcgctcg cggcatcgac 545940
agcgttgaag gttgcgaagc cgtattgcgc accgtcccca aagtctccgc cgtctgcgaa 546000
gccgtcaaaa aagatattcc cgacagcggc tcccgtcctg accgccgaag aaaacaatat 546060
cgccttcgca caaagcaaac gcttggcgga actcgccgtc aagtcggcat aagccgcgtg 546120
ttcagacggc atggcgttca gatgccgtct gaacacgttt gcctgtataa tccgcatctt 546180
tactgtccaa cttcgcggtt cgcaaacctc ccgcgttacc aaaactagga ttcgatatgt 546240
caaaccaaca agccttggtc atcttttcgg gcggtcagga ttcgaccacc tgcctgattc 546300
aggcaatcca aacctacggg cgcgaaaacg tccaagccat tactttccaa tacgggcaac 546360
gccatgccgt cgagctggaa cgtgcccgct ggattgcgca ggatttgggc gtcaaacaaa 546420
ccgtactcga cttgagcctg atgcggcaga ttacgcacaa tgccctgatg gacgacaccg 546480
ccgccatcga aactgccgaa aacggcgttc cgaatacctt tgtagacggc cgcaacgcgc 546540

ttttcctgct ctatgccgcg atttacgcca aagggcaggg gatacggcac atcatcgcgg 546600

gcgtgtgcga aaccgacttc tccggctatc ccgactgccg cgacgtgttt gtcaaatcga 546660
tgaacgttac ccttaatttg gcgatggact atgattttca aatccacacg ccgctgatgt 546720
atctgaccaa ggcgcaaacg tgggcgttgg cggacgaaat gggcgtgctg gactatatcc 546780
gcgagcaaac ccacacctgc tataacggca tcgtcggcgg ctgccgcgaa tgcccgagct 546840
gtatcttgcg cgaacgcggg ctggcggaat atctggaaag taaaaaggcc gtctgaacac 546900
gcgcaaacca taaggaatac gatatgccca agctccatat gttttacctc ggcggcaatg 546960
ccggcaggtc gaatatcgaa gtgcacgaca tccaatttgc cgtgtgcgac aactaccgcg 547020
aggccgtccc cgcgctcaaa gccgcgtggt tcggcgatgc ggacaaaatc cacatcgacg 547080
gctggcagat tgtcgaatgg gcggacggtt acgacatcgc cgtatccgaa acgcccaaaa 547140
cgaaaatgcc gtctgaacac gccccgcgcc tgtatttcgc caatgtcggc ggttatcgcg 547200
cgggtcagct tgccgaggca cacgctttcg ggctgttcgc cgccgccacg cctgccgaag 547260
ccaaacaaaa agccctgcaa accctgttga ccgacagcta tgttcagcag cataaagaca 547320
acttaaaaga cgtggacaac ctgcttgcgc tcgaccgcat cggcaatttc catatccgcc 547380
tgaccccgaa tccgcacggc aaacccgccg aaatcggctt tcaaggctat ttgcccattt 547440
gagaacccat gaaaatcacc aaaatcttca ccttcgactc ctcgcatatg ctcgacgggc 547500
atgacggcaa atgccaaaac ctgcacggac atacctacaa actcgaaatc accgtttcag 547560
acggcattat caaaggcggc gcgaaagacg gtatggtgat ggactttacc gacttgaaag 547620
ccattgtcaa acaacacatt accgacccct tcgaccacgc cttcatctac cacggcggca 547680
acagccgcga atgccaaatc gccgcgcttt tggagggctg gaacatgaaa accctgcgcc 547740
tgccctgccg caccactgcc gaaaatatgg cggtcgaaat gtacggccgt ctgaaaaacg 547800
cggggctgaa cgtgtgccgc gtgaaattgt gggaaacgcc gacatcgtgt gcggagtatg 547860
aaggggagta gggaatatct tgaacgtatc gatatagtaa attccaataa gacatgccca 547920
accgcgtcat tcccgcgcag gcgggaatcc agaccttgat ttatcaggaa tatttaaaaa 547980
ttgcagcaat tccaactctc tggattcccg cctgcgcgga aaggacggtt tagagcgtcc 548040
ttatttgaat ttaccgtaaa acggtttttt ctcctgtacg gattccccgt tttttcagac 548100
gaccttccat atcaaataca cccattaaaa ggaataccca tgaaactcct cttcatcctc 548160
ctagtcctct tcgtcgccgt cgaacatttc tacatcgcct ggcttgaaat gacacagatt 548220
cccagcgaaa aagcggcgga aatattcaag ctgccttatg aatttatgga acaaaagcaa 548280
gtgcagacct tgttcagtaa tcaagggctg tataacggct ttctcggcat cgggctggtg 548340
tggtcgcggt ttgccgcgcc ggacaacgcc gtttacggcg cgacgactct gtttctcggt 548400
ttcgtattga ttgccgccgc gtggggcgcg ttttcgtccg gcaacaaagg catactcgtc 548460
aaacaaggac tgcccgcgat gctggcggcg gcagcggtgt tggcggtatg aaaaaaatca 548520
atgttgcccc cgaaaatccg caataccgta tcgtcgaaat tttcgagagc ctgcaaggcg 548580
aaggctggaa cacgggcatg cccgccgttt tcgtccgctt gggcaaatgc aatctggcgt 548640
gcggctggtg tgataccgat tatttgacat tcggtatgat gggcttgtcc gatatcttag 548700
gccgtctgaa aacctacgcc gcccgcaaca tcatcatcac cggcggcgag ccgaccatac 548760
agccgcatct cgatatgctg ctggacacgc tcaaggcgga aggctatttc ctctgtctcg 548820
aaaccaacgg actcaatccc gcgccgccgc aaatcgacta cgtcgccacc agccccaaag 548880
cctgctacgc cgccaaatat gaaaatagct gtatcgaaac agccgacgaa gtgcggattg 548940
ttgccgatgg tgatgtcctt gcgttctgcg aaaacatgga acgcaaaatc cgcgcacatc 549000
attactacct ttcgccctgt gagcaagacg gtgcgatgaa catctacgac accatccgcc 549060
aaatcggtat tttaaacagt cgccccgacg catccgtgca ttggcagttg agcgtgcaga 549120
cgcacaaatg ggcgggaata gagtagttta agcagtgtaa ctcaaaggga cggcgtacgg 549180
ttttaccgat gtttgacata cggggaaagt gtgccgcttc tgcgtggaaa tgccggcatt 549240
tccaccgccc aatcaggacg gagccttact gaataagatg ctgccgttgg gtacaagctc 549300
ggcttcctaa attccgatgg tcttttgaac cttgccgata ctctgtgcca gtgcgcgcaa 549360
atggcagggt tagggaaaac gaaatgccgt ctgaaacagc attctgtttc agacggcatt 549420
tttctgttgc cgccaaaagg aaaaaccgcc tcggcaatgg atgccgaggc ggtttgaata 549480
tggtcggaat gagaggattc gaacctccgc ccccttcgtc ccgaacgaag tgcgctaccg 549540
ggctgcgcta cattccgaat taagtaaggc gtgattatag cgcaaaaagt gcggcgtgcc 549600
tataccgttt tgcctttttg ccgcgtgtcg ggcggattta aaacgttgtg tttgaataca 549660
gtgttgataa tcatcattat ctttaagtaa ttcaataaga taactttcta cctgaccgaa 549720
aaaatcattg cctttccctg acaaacggtt gatgaaatcg gcagattgtt gaaacgcagc 549780
cggtttaaaa ggcttcgccg actttcacgc cgcccgccgt gtcctgcggc gaggcaaggc 549840
cggcaacaaa ggcttgcgcc gcttggaaat ccgccgtctg catcacggct tgcgcggcgg 549900
cactgccgag cgtgttggcc atatattgcc aacgttgcgc caaagtggga ttgtcaggaa 549960
tgcggaaatc ttcgcgcagt tcatccacaa ggtcgggacg gttgcagacg aggacgatgt 550020
cgcaacctgc ctcaaaggaa atgcgggcgc gttctttgat gccgcctgcc ccgcacgcgc 550080
cctccatagt caaatcgtcc gagaaaatca cgcctttgaa cccgatgtcg cggcgcaaaa 550140
tttgtttgag ccagatttcg gaaaaccctg cgggctttgt gtccacttgt ggataaacga 550200
cgtgggcggg cataaccgcc gccatacctt cgcggctcat aatgcggaag gggcgaggt 550260

cggcggtttc gagttcggac aggctgcgcc agtcttccgg caagaccaga tggctgtctc 550320
cttcgacaaa tccgtgtccg ggaaaatgtt tgccgcagga tttcataccg ccttttgtca 550380
aacctttttg aagggcgagg gcgaggcggg cgaccgcttc gggattgcgg tggaaactgc 550440
ggttgccgat gacggggcag tttccccagt ccaaatctaa gacgggcgtg aaggacaaat 550500
cgatgccgca ggcggaaagc tcggttgcca aaacccggcc gacttgtccg gcggcggttt 550560
cggcggcgga cgcgccgtct ttgtcccaaa tctcgccgag cgtactcatt gcgggcaggc 550620
gggtgaagcc ttcgatgaaa cgttgcaccc tgccgccttc gtgatcgacg gcgataatga 550680
gttcgggtgt gcgcagggct ttgatttcgg cggtgagtgt tttgagttgt tcgatgtttt 550740
ggaagttgcg gcggaagagg atgatgccgc ctacggcggg atcgagcagg cgttgctttt 550800
cctcttcggt caggcggaag gcggcaatgt ctgccatgac ggggccgcgc ggaatatggg 550860
ggacggtcat tgcggtttgc tccaaaaagc ttcagacggc atatgccgtc tgaacaggga 550920
aaggggtcag gcgttggcgc gttttttatc tttcaacaga aaaatcagca ccgccaatac 550980
aatgcctgtc gtgccaaagc ccaacagcgc ggattttgtc agacccaatg cgaggtagcc 551040
cgatgcggcg gcggcggcaa cggttaaggc gtaaggcagt tgcgaggtaa cgtggtcgat 551100
gtggttgcag cgcgcgccgg tggacgacag gatggtcgtg tcggaaatgg gcgagcagtg 551160
gtcgccgcat accgcccccg ccattactgc ggacatacac gggataatca gcgcgggttc 551220
gactttgacc gccatggcgg cggcaatcgg cagcataatg ccgaacgtcc cccagcttgt 551280
gcctgtggca aacgccatca cgctggcgag caggaagagg atgacgggca ggaagccggg 551340
atggatgttg cccgcaacca gtgtggagag gtaatcgccg gtgtgcattt cgccgacaac 551400
cgtactgatg agccaagcga ggattaaaat ggcgattgcg ccgaacatag atttcgcacc 551460
ctgccaaacg gctttgggat agtcggcggt tttaatcgtg ccgagcgtgc agagaacgac 551520
ggcaaggacg ccgcaagtgc cgccgaatac cagcgaagtg tttacgtccg tgttttcaaa 551580
tgcccccaaa atgctgaagg tttcgcttgc ctgcgcgccg gtgtagatca tggcggaaac 551640
cgttgaggcg attaaggcca aaacgggaat aatcagtgcg taaacacgac ctttggtagc 551700
gtctgaaacg gcagtttcat cgtgggcttc gttcaacgcg gcttgttcga aacgtgccat 551760
cgagccgatg tcgaaggaaa accatgcgac gacgaacacc ataatcaggg caaacagtgc 551820
gtaatagttc atcaggctca tggcgacaaa cgtccccatc ggcgtgtatt cggtgatttt 551880
gtaggtaacg agcagtccgg caagcgtggc gataatcgac gcgcccccagc ttgaaacggg 551940
catcagcacg cacataggag cggcagtgga gtcgaggatg taggcgagtt tggtgcggga 552000
aactttaaac ttgtcggtaa cggggcgggc aatcgcaccg acggcgagac tgtggaaata 552060
gtcgtcgata aaggttacga acacgaggca ggcggtcagc attttcgcgc cgcgccggtt 552120
tttaatgtgc cgttttgccc agtcggcaaa cgcctgattg ctgccggagt aggtcagcag 552180
ggaagtaaaa atacccaaaa gtatcaggaa aaccaagatt tttggtttgc ccagcgacca 552240
atcgccgtct gaccaagcca agccgacgac catgtctttc aggtgtgtca gaccgtcgac 552300
ggggttgccg ccgaccaaaa aggcaacgcc gaccagaata ccgatgccta aagacagcag 552360
tacgcggcgg gtaatgacgg caagtgccag tgccaaaaag ggtggcacaa ccgagaaaaa 552420
tgaatgtgaa tagtcgatca gctgcatggt tatgggggtg ttaagcgtcc ggatgggagc 552480
gtatctgtcc gcctccggtt tgggttttgt tggcaaaatg ggcggaaata tttttttgtcg 552540
taaaaaatat ttgtttaaaa tcaaccaact gatttttgta aaatgcccgt taatcggtat 552600
tgacgggcat tttatcattt aaaaaatatt ttggttaaat tatgtgtgtt attgcaggtt 552660
taatgcgata aacagcgtgt tgccacggcg catgatcagc agggggacgt tttgcctgc 552720
cttgtccata gctttgcgga aaccggcttc gtcattgacg gggacttgcc cgacggcaag 552780
aatttcgtcg ccgcgcctca agcctgcgcg ttctgccgcg tcggaaaccc gtacgacgac 552840
gaggtgtccg ccgctgctgt cggtatgtgt ctgaagggta atgcctgcgg attcgaccga 552900
gaacgtaccg gattgctgtt cggtgtaggg ggcttcatct gttttggatg atgcgccgat 552960
atgctcggcg gcgttgccca gcttgacttt gattgtgatt tcttcgcctt tgcgccatac 553020
gccgaggctg acttcttttc ccggcgtaat ggcgccgacc ataacgggaa ggtcgccgga 553080
agaacgtatt tctccgccgt cgaggctgag gacgatgtcg cccgcctgca ggccggcacg 553140
ttctgcgggg ctgccgggca ggattttggc aatcagtgcg ccgccggctt tgtccaaacc 553200
gaacgattgt gccaaaccgt aggatacttc ttgaataatc acgcccagtt gtccgcgttg 553260
gactttgccg gtgtttttca gctgttcggc gacattcatg gcaacgtcaa tcgggatggc 553320
gaaggaaatg cccatgaatc cgccgctgcg gctgtatatt tgcgagttga tgccgacgac 553380
ctgtcctttt aagttgaaca gcgggccgcc ggagttgccc ggattgatgg caacgtcggt 553440
ttggatgaag ggtgtgtagc tttcgttggg caggcttctg cctttggcgg acacgatgcc 553500
ggcggtcacg ctgttgtcga agccgaaggg cgcgccgatg gcggcgaccc attcgcccgg 553560
tttcaaatct ttgggattgc cgattttgac gacgggcagc tcttccgttg cgtcgatttt 553620
cagaagggcg acatcggatt ggacatccga accgatgagt ttggcggtat attcgcgctt 553680
gtcgttgagc aggactttga tactgcccat gccggtaacg acgtgggtat tggtcaggat 553740
gtagccgtct ttgctgatga tgaagcccga accgaagttc aatccgccgt catctgcttc 553800
ttcttggggg atttcgggca tattcgggac gaggcgtttg aaaaattcgt agaacgggtc 553860
gttgtcggca atcgggtcgg aatcgttttc ggcattgccg ctgccgtttt gggtgcgcgg 553920

ggcgggggct gcctgaatat tgacgactgc cggaccttca ctttgaacca gttgggcaaa 553980
gtcgggcagc agcatactga cgctgccgtc gtctttggtg tgttcgatgc gttctacgaa 554040
ggatgcttct tttttgtccg caccgaaaaa gctgcctgcc ttgtcgcagc ctgccagcga 554100
ggcggcacac agtgctgcca aagcgaggta ttggtatttt ttgaacacgt tttgtccttt 554160
gtcggatgcc ggtaccggct ttaatgccgt ctgaagcgca ttttgtcggc ttcagacggc 554220
ataggttgaa attctacaac gtccgtccga attttcaagc gtttcatttt gaagggcggc 554280
ggcggtcagg ctttggcggg atattcgcac aaatcgttga tgatgcaggt ttggcattgc 554340
ggtttgagtg ccttgcaggt gtagcgtccg tgcaaaatca gccagtggtg cgcgtccatc 554400
agaaattctt taggaatgaa gcgcatcagt ttgtcttcga cttcgcgcac atctttcccg 554460
ggggcgattt tggttcggtt ggatacgcgg aaaatatgcg tatcgaccgc catgacggga 554520
tggccgaacg ccgtgttcaa tacgacgttt gccgttttgc gccccacacc cggcaatgat 554580
tccaaagcct cgcggtcttc cggcacttcg ccgttgtatt tttccagcag gatgcggcag 554640
gtttgcataa tgtgtttgga tttggtttta tacagcccga tggttttcgt gtattccatc 554700
acgccgtcca aacccaaatc cagcatcgcc tgcggcgtat cggcaacggg aaacagcttc 554760
gccgtcgcct tgtttacgcc gacatcggtc gcctgcgctg aaagcagaac ggcaattaaa 554820
agctcgaaag gggagttgaa attcagctcg gtggtcggat gggggttggc ggcgcggaag 554880
cgttcgaaga tttcttggcg gatgtgtctg ttcatttttt tatacggtgg gtttgtgtgt 554940
tcggcattat aacgtatggt tcaggcggcg taatattgca ttccccacag aatgaaggcg 555000
taacgcgccg ttttgccgat aaccagcatc agcccgcttg tccacggatt caaccgcagc 555060
cagccggcgg caagcggcag tgcgtcgccg acgacgggca gccaggtaaa cgcaagcagc 555120
caaataccga aacgccgcat cagattcagt gtttttttcag acggcatttt tcgggagggc 555180
agcaaacgcc ccatccaata ggaaaccata ctgcccaatc cgttggcaag gccggcgcac 555240
agcaacgcgc cgtatgcgtg ttcgggaaag cggtggacga acagggcaaa ggcggcttcg 555300
gatgtgccgg gcaggagggt ggcggaagtg aatgcggaaa aggcgagggc ggcgtaggtg 555360
taggagggta tcattgcaaa cagtctcaaa caggtaacaa tcggcgacgg attgtacggt 555420
atagtggatt aacaaaaacc agtacggcgt tgcctcgcct tagctcaaag agaacgattc 555480
tctaaggtgc tgaagcaccg agtgaatcgg ttccgtacta tttgtactgt ctgcggcttc 555540
gtcgccttgt cctgattttt gttaatccac tctattttca cgcccccgcc gaagggcgga 555600
ggacggtgca aaaaatacgg cacagccgta tgcccctttt ttgtcgggca tacgacattc 555660
tttccgctcc ggttttgatg ccacgatgcg gcatttccga attttccgga tacggcggcg 555720
gattttcatt ttattgggaa cggtttttgc aagtccgccg gaattttttta aaatctatta 555780
aaatctatgc aagcaactgt aaaatattaa tttctgctgc ttgaatttca gatcggcgca 555840
ttgcctgcat ccgataaagt ttgcaaaatg ttcaaatatc agtatgattt gcattgccgt 555900
taagaaatgt caatttctat tttcttgaaa cgggtaatat tccgacacca cgaaaggcaa 555960
atcatgtctg cgcaatcaca aaacaatcat acgtccccat tggtcgtctt gaccacgctg 556020
ttcttcatga tgggttttat tacctgcatg aacgacatcc ttatccctca tttgaaagaa 556080
attttcgacc tgtcttacgt tcaggcgatg ctgatccaat tctgtttctt taccgcctat 556140
gcggtgatgt ccatcccgat gggggctttt gtcggcaaag tcggctacaa aaacggcgtt 556200
atcggcggct ttctgctgac ggcggtcgga tgcctgctgt tttatcctgc tgcgggcagc 556260
cattcttacg cggtattttt gggcgcgttg tttattttgg cttccggcgt aacgctgctt 556320
caggtcgccg gtaatcctta tgttaccctg ctggcgaaac ccggcaagga atcggcaaca 556380
ctgacgctgg ttcaggcgtt taacgctttg ggtacgacca ttgcgccgca aatcggcgcg 556440
ttcctgattc tggcggacgc aacccaaacc gtcagcaagg cggaacagat ttcttccgta 556500
cagattccct atttgggact ggcggggctg ctgattatcc ttgccgtttt cgtgaaaatg 556560
atccggctgc ccgacgcgcg caaaattgcc gccgaggaaa gcgcgcacaa ccacgacggc 556620
aaaaccagcg tatggcaata caaacatctc gtgttcggta cggcaggcat tttctgctat 556680
gtcggcgcgg aggtgtctat cggttcgttg atggtcaacg tattgggtta tctgaaaggg 556740
ctggatcatg cttctgccgc gcattacctg tcgttctatt ggggcggcgc gatggtcgga 556800
cgtttcctcg gttcggcggt gatggcgaaa ttcgcgccca accgttattt ggcgtttaac 556860
gcatcggctg cggtcgtact gcttgccgtc gcgatggcga cgggtagcgg caatgcggat 556920
gtggcgatgt ggtcgctgct tgccatcggt tttttcaact cgattatgtt tccgacgatt 556980
ttctctttgg caaccaaagg attgggaaaa tttaccaacg cggcttccgg tgtactgtgt 557040
accgcgattg tcggcggtgc ggtcgttcct gtcgtgcagg gctgggtggc agatacttac 557100
accctgatgt cttcgtttgt cgtttccgtc atctgttatc tgtatatcgt gtttttttgcg 557160
gtgtacggat atagggcgga caaataatct ttttcttgag aaatgtcgtc tgaacatctt 557220
tcagacggca tttttgcgta ccggtgtttg cggcgtgtgt gccgaggttt taatacttca 557280
atccataaaa gtcttatatg tcaacaaaca aaaaaataaa aaattatatt tcaaaaaaat 557340
taatttaaat tgagaaaatt gccgttttgt ttctgtccgg cttttgtaaa acgctaaaat 557400
gccgtctgaa aacgtcgggc ggattcggta tggtgtgtta gaatccgtta actttatatc 557460
aaatcgggca aagaatcatg ttcgctttca aatccttact cgatatgccg cgcggtgagg 557520
cacttgccgt cgtcgtcgct ctgattgccg cgatgggcta taccatcatt tcattggagt 557580

ggttgccgca tatgtccatt attgccgcca tcgtcgtgct gattttgtac ggcttggcgc 557640
gcggtttgaa atacaacgat atgcagcagg gcatgatagg cgcgttgaat cagggtatgg 557700
gcgcgattta cctgtttttc ttcatcgggc tgatggtcag cgcgctgatg atgagcggcg 557760
cgattccgac gctgatgtat tacggtttcg gactgatttc cccgacttat ttttattttt 557820
cctccttcgc gctgtgttcc gtcatcggcg tgtccatcgg cagcagcctg accacctgcg 557880
ccactgtcgg cgttgccttt atggggatgg cggcggcgtt tcaggccgat atggcgatga 557940
cggcgggcgc gattgtttcg ggcgcatttt ttggcgacaa aatgtccccg ctttcggata 558000
cgacgggtat ttccgcgtcc atcgtcggca tcgacttgtt tgagcacatc aaaaatatga 558060
tgtacaccac catccccgcg tggctcatta gtgcggcact gatgctttgg cttttgccga 558120
atgtcgccgc gcaggatttg aacagcgtcg aatccttccg cagccagctt gaagccacgg 558180
gattggtgca cggctattcg ctgattccgt ttgcgctgtt ggtcattttg gcattgatgc 558240
gcatcaacgc cgtcgtcgcc atgctcttta ccgtcatggt tgccgttgct gtaacgtatc 558300
tgcacagcac gcccgatctg cgtcagctcg gtgcgtggtt ttacggcggc tacaaactcg 558360
aaggcgaagc gtttaaagat gttgtcaaac tgatttcgcg cggcggtttg gaaagtatgt 558420
ttttcacgca aaccatcgtg attctcggga tgagtttggg cggactgttg tttgcgctcg 558480
gtgtgattcc ttccctgttg gaggccatcc gtaccttctt gacgaatgcc ggacgcgcga 558540
cgttcagcgt tgccatgact tcggtcgggg ttaatttcct gatcggcgag caatatttga 558600
gtattttgtt gtcgggtgaa acgttcaaac ccgtttacga taagctcggt ctgcattcgc 558660
gcaatctgtc gcggacgctg gaagatgcgg ggacggtgat taacccgctc gtaccgtgga 558720
gcgtatgcgg cgtgttcatc agccacgcgc tgggcgtgcc ggtttgggaa tatctgccgt 558780
atgccttttt ctgctatttg agtttggctt tgaccctgtt attcggttgg acggggctga 558840
ctttgagcaa aaaataagcg gataagcgaa atgccgtctg aaacttgcaa cggtttcaga 558900
cggcattttt atgtttggcg gatggggcgg attgaaacag aaaacgcccg taccgtcatc 558960
ctaaactgtg cagaaacggc ggtgcttact tcacgcgggt cgccatcagc gtatgcaggc 559020
ggcggttgtc ggcgcgtgcg acggtgaact gcaaaccgcc gataaggact ttttcgccgc 559080
gcacgggcag atgtcccaac tcttgaatga ccaggccgcc aatggtgtcg gcttcttcgc 559140
tgctgtattc cgtgccgaag aaggtgttga tgtcttcgat ttcggtagct gcatggatgc 559200
gccagcgttc ggaagaaacg gcatggatat tgtcggcgct atcgtcttcg tcaaactcgt 559260
cttcgatttc gccgacgatt tgctcgatga tgtcttcaaa ggtgaccaag ccggatgtgc 559320
cgccgtattc gtcgatgaca atcgccatat ggttgcgctg ttcgcggaac tcttttaaaa 559380
gggcggtcag cgatttgcct tcggggacga agacggcggg gcggagaatg gatttgaggt 559440
ggaactgctc ggggttaaac atatatttga gcaggtcttt ggcgtgcaaa atgcccaaaa 559500
cttcgtcttt gtcttcgccg atgacgggga agcgcgaatg ggcggtatcg ataacgtagg 559560
cggtgatgcg ctcgatgctg tcgttttctt ttaaaacgtt catacggctg cgcgtaatca 559620
tcgcgtcgcg cacttccaaa tcggaaaaat cgaggacttt ttccaatctt aaaagcgtat 559680
ccgcatcaaa aacttcctgc tcgtgcgcct gccgaagcag gtttaatacg tcttcggcgg 559740
aatcgggttc gcgggcgagt cgggcaatca ggcgttcaaa aaaattcgtt ttcggttgtg 559800
cgccgtccat tttaatgtca gtcctcttgg taggggttgg ggaagcctgc cgcccgcatc 559860
aggcggattt cttcggcttc cattatttcg gcttcgtcgt cttcgatgtg gtcgtagccc 559920
atcaggtgta aagtaccgtg tatggtcagg tgggcaaaat gctgctcggg tgttttgcct 559980
tgttcggcgg cttctttcaa aaccacttgc gggcagataa tcaaatcgcc gtacagtttt 560040
tccgaaactt ggcagggcag gatttcgcct tcgttgagcg cgaaactcaa tacattggtg 560100
gcgtaatctt tgccgcggta gtcgcggttg taggctcggg cttcttcttc gtccagaaga 560160
atcaggctga tgtcggcgcg gcggtattca tttttcaagg cagaccacgc ccagcggtag 560220
aaatcgcgtt cggctgggat gccggcggcg gaagaggcgt tttcaaagtt caaatggaaa 560280
cgttgccgct gcaacgttaa gaaagggtat tttttggtgc gtttcattgt ggcgggtttc 560340
gtgttttgtg ggtgtaaata taacatagac ctgacggtgc cgtctgaaga aacgttcaaa 560400
atatgataga cttcacgccg tttccattct ttgaacgcat tgaacatgaa cccgaaaaaa 560460
ctcgtcatcg ccagccgcga aagcctgctt gctatgtggc aggcaaagca tatccaaggc 560520
cgtctgaagg ctctgtatcc cgattgcgaa gtcgagattt tgggcatgac cacgcgcggc 560580
gatcagattt tggacaaaac tttgtcaaaa gtcggcggta aaggcttgtt tgtcaaagag 560640
ttggaacagg ctttatatga cgggcgcgcc gatttggcgg tgcattcgat taaggacgtg 560700
ccgatggatt tgcctgaagg tttcgcgctt gccgccatcg gcgaacgcgc caatccgttt 560760
gacgcgtttg tgtccaacca atacacgcgt ttggaagaaa tgcccgaagg cgcggttgtc 560820
ggcacatcca gcctgcgccg cgaagcccag ttgcgtgcgc gctatccgca tttgcttatc 560880
aaacctttgc gcggcaatgt gcaaacccgt ttgtccaaac tcgataacgg cgaatacgac 560940
gcaattatct tggctgccgc cggtttgcag cgtctgaaat tggacggacg catccgcatg 561000
attttgtcgg aatccgacag cctgcctgcc gccggacaag gcgcattggg tatcgaaatt 561060
gccgcgcacc gcgaagattt gtatgaagtt ttgaaaccct tgaaccacgg tgttaccaat 561120
gcctgcgtta ccgccgaacg cgccctcgca cgcgctttgg gcggaagctg ccaagtgcct 561180
ttggccgcat attgcacgga agaaaacggc ttgctgacct tgcgcggctt ggtcggacac 561240

cccgacggtt cggttgtgtt gcgggcggac gcgcaagccc ctgccgaata tgccgacgcg 561300
ctcggacgcg ctgtcgctaa gaaattggcg gacgacggtg cgcgggaatt gattggagca 561360
gtattgaata cggaaaattg attttatcga aaatttaaat aaaataatat aagttattgt 561420
ttttaatcaa tttgtttcat cagtttcact cgccttattt cgtcattccc gcgcaggcgg 561480
gaatccagtt tgctcggttt cagttgtttc taatcaattc ttgcagcatt ggattcccgg 561540
attcccgcct gcgcgggaat gacggcggaa aggtttttgt ggcttcggat aatactgtgg 561600
cgttcaaatt ttgaatttga gaatgatgat attcgtattt tttatttgag ttcatcatat 561660
ttggttgatt ttatagatgt ttttagcttg tttgaaattg ttatggttta ttgtttttta 561720
acaaaaaaca gatgccgtct gaactggtta aggttcggac ggcattttca tatggctgtg 561780
ctttttacag tactttcacg atgctttcgc acagataatc gatgttgttg tcggtaatgc 561840
cggcgacgtt gatgcggccg gagcggacgg cataaatggc aaactcgttt ttcaggcggt 561900
cgacttgttc gggagtcaag ccgctgaaag agaacatacc gttttgtttg ataatgaaat 561960
caaagttttg gcttgcacct ttggctttga gcaacccgac aaatttttgg cgcatggctt 562020
tgatgcggcc gcgcatttca tcgagttcgg caatccattg tgctttcaaa tcatcatttt 562080
tcaacaccag cgcaatggtg ttcgcaccgt gtgaagccgg gttggaatac aaggtacgga 562140
tgatggtttt gacttggctg tgggcgcggg ctgctgtttc ttcatcttcg gccaccaaag 562200
tgaacgcgcc gacgcgctcg ttgtacatac cgaagttttt ggaataagag ctggcaatca 562260
gcaattctgt attgtgtttc aagaacacgc gcaagccgta ggcatcttct tccaaaccat 562320
tgccgaagcc ttggtaggca aagtcaaaca gcggcaacca gcctttttcg gcagaaagtt 562380
ttgccaaagt ttcccattgt tcgggcgtag ggtcgatgcc ggtaggattg tggcagcagc 562440
cgtgcagcag gacgatgtcg cctttttgcg cttggctcaa gtcctcaatc atgccgtccc 562500
aatccaaacc gtgtttggcg gcatcatagt aacgataagg tttgtcttgg ataccgaccg 562560
ctttggcgat ggcgttgtgg ttgggccaag tcggattgga aatccagatg gtttgcgcgt 562620
tcaactggcg tttggcaaac tcggccgcaa tacgcaatgc gcccgtaccg ccgaggcttt 562680
gcgctgtttt ggcgcgacgg ctggcgatga tttcgtggtc tttgccgaac agcaggattt 562740
gggtttgcgc gttgtagtcg gcaacgccgt cgatggtgag gtagtttttg gtggtttcgc 562800
tttccaacag gcgtttttcg gcttctttga cggctttgac gaggggtgtc gcgccggatg 562860
cgtctttata aacgccgatg ccgaggttga ctttttcggg gcgggtttcg gctttgaacg 562920
cttcgcccaa accgagaatc ggatcggcgg gggcggcttc gatgtgcttg aagaacatag 562980
cttgctcctt gatggggacg gaaggtcatt cgggtttgcc gattttacgc tgttttacac 563040
gggctggaaa cagacgcaat cacgcctgcc cgatatgggc gaaggtttcc cagtttgact 563100
gtatgtgttc tgcaagcagg ggcaggtctt gttcggcggc ttcgtagtat gcgccgtccc 563160
attcttcaaa ttcggggaac tgtttgcgca gggaagggat gtctgcgcct ccgtcggcga 563220
gcgtttcgat ggttttgccg tgttgttcgt agagggcttt ggctttgtcc aagactttcg 563280
gcacggcttt gattttccag cggcgcaggc tgtcggcaag cgggttgcgg aaaatatatt 563340
cgccgtaacc ggatgcgatg agttgcacga agccgccttc ttcgacttgg ctgtcgaggt 563400
agcagaatgc ggtcagcgtg tgctggtcgt cggacaggca ggagagggat tcgtcgccgg 563460
tttgggcggt gtgttcgagg taggcggaaa cgagggtata gagcagggcg gatggctctt 563520
gttggcggat gtcttccgga agggtaagcg cagtcatggt atgccgtctg aaaagtgggg 563580
attatagcgg attgcggctt tgcgccgaaa atatccttta gcctgccgat ggcgtaaaat 563640
aggcgcacgc caaccacgca aaggaaaatc aaatggacaa tctgaatccg caggaaattt 563700
ccgtgttgcc ggaaaatctg ccgctgtatt gctcgggacc cggcaacgag cagtggaacg 563760
ggcatccgag agtgtttttg cctttgtgcg aaggagaatc gggcagcgtt gcctgcccgt 563820
attgcggcac gcgctaccgc cttgacggca agatgccgca tcatcattac gcctgaacgc 563880
aaacggcgga aaaatgccgt ctgaagcctt tttcggtttc agacggcatt tgtttggcgg 563940
ggcgggcttg ttccggcacc gggattctgc ccgacgcgcc gcccagacgg tgaacggcgg 564000
tttccgttcc ccgcgtgctg ccgctatgga tggtggcgtt tcgcctagag gaagaaaatc 564060
attgccgcga cgacggcaat cacgccgtaa atcgccatcg ggataacggt tttcttgata 564120
atcgcacctt cggaattttt cacatccaat acggtacata cggcgatgat gttgttgagg 564180
cacaccatat tgcccatcgc gccgccgacg gactgcaacg ccagaatcag ggtaacggac 564240
aggccggtat ccaaggcgat ttgctgctga atcgggccga aggtcaggtt ggacacggtg 564300
ttggaaccgg agaagaacgc accgatcgcg cccagatacg gcgagaaata aacccagtgt 564360
tcgcccgcca ttgcggcaaa ttccttaccg atgattttca ccatcgaatt gtcgccgccg 564420
accagcatca gctgaaccat aatcagcgcg cccatcaggg caagcagcgg ttttttggtt 564480
tgattgaagg ttacggaata aatcgtccag gcatctttga atttggtttt atacagcagg 564540
atgcaaatcc aaacggtcag cacaaacgga atccaagccg ggacgtacag cgtttggtaa 564600
gacgcgctga catcttgtcc gaaaatattg ccgaaggtaa tcgtcaggga gtcgctgacg 564660
gtgattttgg acaaatcaaa cggcagttgg aagctgaacc attcttcttt gctggtcaaa 564720
atgcctttga tgccgagctg tttgatgcgc gtaaccacca gcatgccgat cagcataccc 564780
aaaggggcga gtgctttggc gacttgggcg aacggcactt tttcggcatt cgggtctttg 564840
gcgtggtctt tgctcaagcc ccagccttgg ttggcggcga atacggacac catcaggccg 564900

atcgcgccgg cgacgagcga cgggaattct tcgttgacca tcgccaatgc gacataagga 564960
atggtgcagg agaagacggc aatggcgacg aagcccaagt ttttgcggat ttcagaccaa 565020
ggtacgatga agcccaagcc gatgacgggg atgacgaaac ctgcgaagaa gtgcattacg 565080
ccggtctgcc tgccgatggc gaggatgtct tcggcactca ggttcagcgg tgcgaaaccg 565140
aaccaggtcg gcgtaccgac cgcgccgaaa gagacgggga cggagttcat caccaaagtg 565200
aaaatcgcca ctttcaacgg gttgaagccc aagctcatca gaatcggcgc ggcaatcgcg 565260
gcaggcgtac cgaagccgga tgcgccttca atcataaagg caaaagccca gccgataatc 565320
atcagttgcg ctacggggtt cgggctgatg gtcgccagcc atttgcggat gacatcgatg 565380
cagcccgtgg tttccatcat acggttgaac ataatcgcgc cgaaaatcac ggtaatcggc 565440
gtgagcgttt tgacgaggcc ggaagcggcg gtggcgttga gcagcatgcc cgcatcgtcg 565500
aagtagaaaa gtttgatggc gtaaatcagc actgcggtaa tcggcagcgc gacgtaggag 565560
ggcatactgt tttttttcac catcagccaa atcagcagga cgatggggaa tatgctgagg 565620
aaaagtgcca taacgaatcc tttttaggca tttgcatcat aaggcgcgtc gaggtttgga 565680
aagacgttca aatcccgtac acccgatatt ttggttaaaa gataaattgg taagaccaat 565740
tgttatgcgt ttgcacactt tacgtaatct tatgtaatcg gtcaagcatt ttatcgataa 565800
tggctattaa tgcgggttaa ggatagtgat gatgcggcgg agggagtgtg ggaaaggggc 565860
gtgtaaaaaa agccgcccga aaggcttcag acggcatttt cagtattttt ccagcggcac 565920
gaataccgcg ccgtccccgt ccacgcggag gattgaagca tagtcgttat gccagtcgcc 565980
caaaacgatg cgggtaaagc cgttttcgtg atggatatgc tcgcggtggg tgtgtccgtg 566040
tatcagcctt tccgcaccga aggcgcgtac ctgccgcgcg gtaaaggcgg cattgacatc 566100
cataatatcg gcgggcttga cctgtttttc cattttgctg acacgcctga ttttggtggc 566160
aaggcgcgtg cgccacttca ggggcagcat taggaacagt tttgcagcc gcttccgatg 566220
cacgattttg cggaaacgtt ggtatgccct gtcatctgta cacagagtgt cgccgtggca 566280
gatgagggtt ttgcagccga acaagtccaa aaccgagtaa tccggcagca gcgtcatgcc 566340
cgcctgccgg caaaaatcct gaccgatcag gaagtcgcgg ttgcccctga cgaagaacac 566400
ggcaacgcct ttgtcggaca atttcctgat ttcacgcgca accgaagtat tcaactcgga 566460
aacttcgtca tcgcccaccc aaaaatcaaa caaatcgccc aaaatgtaaa tcgcccgcgc 566520
ctgcccggcg gcggaagaac gtaaaaaacg cagcagcagc gcggtcagtt cgggctgctt 566580
ttcgctcaaa tgcaggtcgg aaatgaaata ggcgggtttc ataggcaggt ttccaatcgg 566640
gcggatgtcg gggcggatta taacgcgccc ggcggcgggg caatacggca aatgccgcgc 566700
caagcatcgg gcattggcgg aaccggggtt cgggcgcgtt aaaaatgccg tctgaaggct 566760
tcagacggca tcgagggtgc gggatgcggt aaggttttgc cggcaagata tggggtggtg 566820
cggcgggatt tccgttaaaa tacgcttctt ttttattttt tccgaccatt atgcgcctga 566880
cccatatcaa actctccggc ttcaaatctt ttaccgaccc gaccacgatt catgtgccgg 566940
ggcagcttgt cgcggttatc gggcccaacg gctgcggcaa gtcgaatgtg attgacgcgg 567000
tgcgctgggt gttgggcgag gcttcggcga agcagcttcg tggcgagagt atgcaggacg 567060
tgatttttaa cggtgcggcg acgcgccgtc ctgcgccgag ggcttcggtg gagctggtgt 567120
ttgacaacag cgaccacagt ttgcaggggg cgtgggggca gtatgccgag gtgagcatca 567180
agcggcagct gacgcggcag ggcgaatcga cttatttcat caacaatcag accgtgcgcc 567240
gccgcgacat taccgatttg tttctgggta cgggcgtggg cgcgcgcggt tatgccgtta 567300
tcgagcaggg gatgatttcg cgcatcatcg aagcgcggcc ggaggagttg cgcgcctata 567360
tcgaggaggc ggcgggcgtg tccaaatata aggaacgccg caaggagacg gaaggtcgtc 567420
tgaaagacac gcgcgagcat ttgcagcgtt tgggcgattt gcagaacgag ttggcgcgtc 567480
aggtggaaaa gctggaaaaa caagcggaaa ccgccgaacg ctacaaatcc ctgaccgcgc 567540
agctgaatca gcaacaggat ttgctcgatt acgcccaatg gcggcaatcg cttgccgccg 567600
ccgataaggc gaccgcgcag catcaatctt tgcaggcgca gcaggacgaa accgccgcgc 567660
aggttcaggc gttaaacgac gaagtacacg ccttgcagac tgccgaacag tcgcagcagc 567720
aggcagtgca tgaattgagc aacaagcgcg gcgtgttgcg cgagcagatt gcccgtttgg 567780
aagaacaaat ccgccatcag caaaacctgc accaacgcat cgaacgcgac aagcaggcag 567840
cgcaggcgca gttacaacgc attcatcaag agcagcagca aatccgcgtg cagcttgaag 567900
aaaacgagtt gcaggtcgaa gaaaaacaaa ccgagctggc ggaatgggcg atgcaggttg 567960
ccgaacacga ggagcgtctg cccgaattgg aagaagccca agccacgctc aacgccgcct 568020
tccaaaccca gcaggacgag gcaaaccgca tccgccgcga actggcgttg aagcagcagc 568080
agcttgccca tgccgaacaa acgattgcca agcacgaaga gcgcaaaggt cgtctgaaac 568140
aggaaaacca agccttaaac ctgcccgacg aagccgaaac cgccgccgcg caggaagcag 568200
ccgccttgtt gcaaagtcag caagagcatt acgaagaaca aatcattgcc gccgaagaag 568260
ccttacacgc cgcccgcgag gcgtttcaga cggcctcaaa ccgcttccaa agcctgaagc 568320
agcaacacat caccttgcag gcgcagcagc aggcgttgtc gcaaatcctg tcgcaacagc 568380
aggaagccgc cgatttctgg caggcaaccg accacgccgc cgcgccgcaa ctgtggcaac 568440
acatcaccgc gcccgccgag tggcagcacg ccttgtccgt cattcttgcc gaacgcctgc 568500
acgcccgcgc cgtgccgcaa ggtttcgtgc cgcccgagcc tttgccgcag gggcaggcgg 568560

catggctttc agacgacctc tcaggcggca tcaaaaaatc cctgcccgta caggcattgc 568620
tgaaccaaat ccaagcgcag ccgccgtttc agacggcatt gcactactgg ctcgacggcg 568680
tattgtgcgc gcccgatttg agctatgccc tcgcgcatca aaacgatttg ggcgcacacc 568740
aaatctggct cacgcccgaa ggtcatcagg tcgataaagt cagcgtcctg ctctatgcca 568800
aacccgcgca ggaaagcctg attgcccaaa aagcgcgcct cgacggcatc gcgtccgaac 568860
tggaaaacct cgcccccgaa ctttccgccg ccgaagccgc gttcaaacag gcggaagctg 568920
ccgtgcgctc gtctgaagtg caacataaaa acctgatgca gcagcaacag cagcacacgc 568980
gccaatacag tcaagcacag caacgcgccg ccgaactctt agcgcgtacc aaccaagggc 569040
aaatccgccg cgaacacatc gagcgcgaac tggcgcagtt ggcggaagaa cagaccgtgt 569100
tgcaacacac gtccgacggg ctttcagacg acatcgttac cttgcaggaa gccgccgccg 569160
aactcgaaca ccagcagcaa accaccgcgc acagccgcca agagcagcaa ggccgtctga 569220
aacaggcgca gcttgccctg ttggaagcca accgccaata cgggcttgcc gaagtcgccg 569280
tccacaaact caaccagcaa aaacaaaact accggcagca aatcgcccag cttgaacagc 569340
aaaccctcga ctggcaggaa cgccagcaag agcttgccct cgcctatgaa accgagttcc 569400
aaaacgacga gcagcacatc aagcttgaag aattaagcga agccgtacag accttggacg 569460
aagaatatat tgttgtgcaa gagaaactcg cgcagattca ggaacagggc agggagcaat 569520
acgctaaagt gcaaaccctg caaaccaagc tgccgcagct tcaggccgcc acccaaaccg 569580
ccttgttgca gcagcaggaa gccctgatca acgccaaacg ctaccatcaa aacctgaccg 569640
aacgcgccgc cgatttggac gcgctcgaag cgttggcgaa agaatcgccg aaagtattga 569700
acagcagcat cggcagcctt tcgcaacaaa tcgaagcact cggcgcggtc aacctcgccg 569760
ccctgcaaga actcgaagaa gcgcgcgaac gcgacggcta ctaccgcagc caaagcgaag 569820
acgtgcaggc agccatcacc cttttggaag aagccatcgc ccaaatcgac gacaaaacca 569880
aagcgcgttt caaagaaacc ttcgatgccg tcaacagcaa agtccaaacc ttcttcccga 569940
ccctgttcgg cggcggcgaa gccactctca aaatgatagg cgacgaccta ctgaccgccg 570000
gtgtgtccat tatggcgcgt ccgcccggca agaaaaacag caccatccac ctcctctccg 570060
gcggcgaaaa agccctcacc gccatgagcc tcgtgttcgc tctgttcagc ctcaaccccg 570120
ctccgttctg ccttttggac gaagtcgatg ccccgctgga cgacgccaac acctcgcgtt 570180
tctgcaggct ggtcaaagaa atgtcggcgc aaacccagtt cctctacatc tcccacaacc 570240
gcctgacgat ggaaatggcg gagcagctgg tcggcgtaac catgcaggaa aaggtgtct 570300
cgcgcgtcgt cgccgtggac atcaaacagg cgttggaaat ggcggaagcc gtttgaacgg 570360
gttgcagaac ggctgaatct tgccgttttt aatgaagtgt tgcgatatgg gttttcagac 570420
ggtatttcaa acagaacaga ttaaaatcaa atccaaatcc ataaaaaatg ccgtctgaac 570480
agcgttcaga cggcatttcg atgtgtactg ccacgtcaaa tcagtggtga tggccgcagc 570540
cgcattcttt tttcatatcg atcaccatac ggccggtgat tttgccttcg cgcatttctt 570600
ggaaaatggc gggtgcttca tccaaagcac gcagttggac tttcggcaca accaaacctt 570660
ccgcgccgaa ttggaaggct tcttccaaat ctttgcgcgt gccgaccaaa gagccgacca 570720
cttcgatgcc gtccaaaacc aaacgcggga tggacaaatc catcgattcc ggcggcagcc 570780
cgatggcaac cacacgtccg cccgcgcgga cgcaattcac ggcagagttg aatgcggcag 570840
cagatacggc ggttacgacc gcagcgtgtg cgccgccggt tttttcctga atcactttgg 570900
cagcgtcttc tttggcggcg ttgacaacca aatccgcgcc ggtttctttg gcaaacgcca 570960
gtttgtcgtc gttgatgtcg atggcgacaa cgtgcgcgcc gaatactttt ttcgcgtatt 571020
ggaccccaa gttgcccaaa ccgcccgcgc cgtagatggc aatccactgt cccggacgaa 571080
cgccggaaac tttaatggct ttataagtgg ttacaccggc acaagtaatg ctggaagctt 571140
gcgcaggatc caaaccttca gggactttga ccgcgtaatc ggcactcacg atacagtggg 571200
tcgccatacc gccgtcggcg gtgtagcccg cgttcaatac ggaacggcac agggtttcgc 571260
ggccggtatt gcagtattcg caagagccgc agctttggaa cagccaagcg atgctgacgc 571320
ggtcgccgac tttcagattt ttcacaccgt cggcaacttc tttaaccaaa ccgatgcctt 571380
cgtgtcccaa cacgcggccc ggtttttcgc cgtagtcgcc tgccgcaacg tgcaggtcgg 571440
tgtggcacac gccgcaatat tcgacttcga ccaatgcctc gccgtattcc aacgggcgaa 571500
cctcgcgttc gattacttcc acatcgcccg ctacattttt attcacaaca actgcctgca 571560
ttttcatgat tgcgctcctt agttacggca aaaaaacctg taaacggaat gttgtccgat 571620
ataagggtaa gcatacgctt ccgcatctca caggtcaagt ggtatgttgt tgaaaaatat 571680
agattatatg ttatattata acatcttgga aaggcacggc atcggggcgg ttgccggatg 571740
aggggcggca ggtttcaagt ttgaaaaacc ggacggcaaa cccgtaaaga taccgtctga 571800
agctgtgtcc ggacggcatc tttacgggtt tgcgggcttc ggcggaggat tagtcgaagc 571860
cggggcagga ttggtttgta ccggaagcgg caatggtacc gccgtcgttg agcgtaacga 571920
cgcaggtttc gccgtcgttg gtggtgggat tggggtcggc ctgaagggta aagtggtcgg 571980
ggctgacttc gcttaaagtg atatcgaaat attcgttttg tttcagtttg tttttgtcgt 572040
aggttttaaa cgtccctttt tggcggtagt aacgttccat ggtctgcgcg ttgtgcagca 572100
gggtcgtcct gacttccgac aggcggacgc gccggatgta ggttttatag gaagggtagg 572160
tgatgagcgt caggatgccg aggatggcga cggcaatcat cagctcgagc agcgtaaagc 572220

cttttttgaac gtttttcata gcaatgtgtt tccatttgtt tgtcggtcga cggcattata 572280
acgccgtatc ggaaatggcg gaatatgtaa acggattgaa attttcggga aagcagattg 572340
tataagccat ttaaaacaaa tggttatttt tattgtcggc agtttgccgc cttggatggg 572400
gcagggactt gcggtagaat ccgcttccga tttatgggat tgacgcatac agagaattga 572460
aaacatggca aaaatgatga aatgggcggc tgttgcggcg gtcgcggcgg cagcggtttg 572520
gggcggatgg tcttatctga agcccgagcc gcaggctgct tatattacgg aaacggtcag 572580
gcgcggcgac atcagccgga cggtttctgc aacaggggag atttcgccgt ccaacctggt 572640
atcggtcggc gcgcaggcat cggggcagat taagatactt tatgtcaaac tcgggcaaca 572700
ggttaaaaag ggcgatttga ttgcggaaat caattcgacc tcgcagacca atacgctcaa 572760
tacggaaaaa tccaagttgg aaacgtatca ggcgaagctg gtgtcggcac agattgcatt 572820
gggcagcgcg gagaagaaat ataagcgtca ggcggcgtta tggaaggaaa acgcgacttc 572880
caaagaggat ttggaaagcg cgcaggatgc gtttgccgcc gccaaagcca atgttgccga 572940
gctgaaggct ttaatcagac agagcaaaat ttccatcaat accgccgagt cggaattggg 573000
ctacacgcgc attaccgcaa cgatggacgg cacggtggtg gcgattctcg tggaagaggg 573060
gcagactgtg aacgcggcgc agtctacgcc gacgattgtc caattggcga atctggatat 573120
gatgttgaac aaaatgcaga ttgccgaggg cgatattacc aaggtgaagg cggggcagga 573180
tatttcgttt acgattttgt ccgaaccgga tacgccgatt aaggcgaagc tcgacagcgt 573240
cgaccccggg ctgaccacga tgtcgtcggg cggttacaac agcagtacgg atacggcttc 573300
caatgcggtc tactattatg cccgttcgtt tgtgccgaat ccggacggca aactcgccac 573360
ggggatgacg acgcagaata cggttgaaat cgacggcgtg aaaaatgtgc tgattattcc 573420
gtcgctgacc gtgaaaaatc gcggcggcaa ggcgtttgtg cgcgtgttgg gtgcggacgg 573480
caaggcggcg gaacgcgaaa tccggaccgg tatgagagac agtatgaata ccgaagtaaa 573540
aagcgggttg aaagaggggg acaaagtggt catctccgaa ataaccgccg ccgagcaaca 573600
ggaaagcggc gaacgcgccc taggcggccc gccgcgccga taaacgaata tgccgtctga 573660
acacggaaac ggtttcagac ggcatttgtt attgatttac ggaatattat gagcttgatc 573720
gaatgtaaaa acatcaaccg ctatttcggc agcggcgaga accgcgtcca tattttgaaa 573780
gacatcagcc tgtcgataga gaagggcgat tttgtcgcca tcatcgggca gtccggttcg 573840
ggcaagtcca cgctgatgaa catactcggc tgtttggata ccgccggttc cggttcgtac 573900
cgaatcgacg gcatcgaaac tgccaaaatg cagcctgacg agctggcggc attgcgccgc 573960
gaacgcttcg gtttcatctt ccaacgctac aacctcttaa gctcgctgac cgcaagggac 574020
aacgtcgcgc tgccagccgt ctatatgggc gcgggcggca aagagcgttc cgcgcgggcg 574080
gacaaactct tgcaggattt gggtttggca agcaaagagg gcaacaagcc cggcgaactc 574140
tcgggcggac agcagcagcg cgtctccatc gcccgcgccc tgatgaacgg cggagaaatc 574200
atcttcgccg acgagccgac cggcgcgctc gataccgcca gcggcaaaaa cgtgatggaa 574260
atcatccgca ggctgcacga agccgggcat accgtcatta tggtcacgca cgaccccgac 574320
atcgccgcca atgccaaccg cgtcatcgaa atccgggacg gcgaaatcat ttccgacacc 574380
tcgaaaaatc ccgaaatccc cgcaagcaat gtcgggagga ttcgggaaaa agcttcgtgg 574440
tcgttttatt acgaccagtt tgtcgaagcc ttcagaatgt cggtgcaagc agtattggcg 574500
cacaaaatgc gttcgcttct gacgatgctc ggcatcatca tcggtatcgc gtcggtggtt 574560
tccgtcgtcg cattgggcaa tggttcgcag aaaaaaatcc ttgaagacat cagttcgata 574620
gggacgaaca ccatcagcat cttcccgggg cgcggcttcg gcgacaggcg cagcggcagg 574680
attaaaaccc tgaccataga cgacgcaaaa atcatcgcca aacaaagcta cgttgcttcc 574740
gccacgccca tgacttcgag cggcggcacg ctgacttacc gcaacaccga cctgaccgcc 574800
tcgctttacg gcgtgggcga acaatatttc gacgtgcgcg gactgaagct ggaaacgggg 574860
cggctgtttg acgaaaacga tgtgaaagaa gacgcgcagg tcgtcgtcat cgaccaaaat 574920
gtcaaagaca aactctttgc ggactcggat ccgttgggta aaaccatttt gttcaggaaa 574980
cgccccttga ccgtcatcgg cgtgatgaaa aaagacgaaa acgctttcgg caattccgac 575040
gtgctgatgc tttggtcgcc ctatacgacg gtgatgcacc aaatcacagg cgagagccac 575100
accaactcca tcaccgtcaa aatcaaagac aatgccaata cccaggttgc cgaaaaaggg 575160
ctgaccgatc tgctcaaagc gcggcacggc acggaagatt tcttcatgaa caacagcgac 575220
agcatcaggc agatagtcga aagcaccacc ggtacgatga agctgctgat ttcctccatc 575280
gccctgattt cattggtagt cggcggcatc ggcgtgatga acatcatgct ggtgtccgtt 575340
accgagcgca ccaaagaaat cggcatacgg atggcaatcg gcgcgcggcg cggcaatatt 575400
ttgcagcagt ttttgattga ggcggtgtta atctgcgtca tcggcggttt ggtcggcgtg 575460
ggtttgtccg ccgccgtcag cctcgtgttc aatcattttg taaccgactt cccgatggac 575520
atttccgcca tgtccgtcat cggcgcggtc gcctgttcga ccggaatcgg catcgcgttc 575580
ggctttatgc ctgccaataa agcagccaaa ctcaatccga tagacgcatt ggcacaggat 575640
tgaggttgga caaagatgcc gtctgaagct gcaggaccgg tcattttgga gcagaaactt 575700
attggataaa aacggtttct tagattctac gttccagatt cccacttgcg tgggaatgac 575760
ggcggcgggg gttcgatgat tgcacacaac gctcgagtcc cgtcattccc gtaaagacgg 575820
gaattcggtt cgttcggctt tgcttgtttc ggataaatca cggtaactca atattccaga 575880

ttcccgcccg cgtgggaacg gcggcggggc ttcgtattgt tcaatttatt attttcaatc 575940
attcaatggg ttaggatgtg tttgttggct tgctaacttt cagggcggat tggttttcag 576000
gcggcatttc ccgcaaaaaa ggcttggaat tttccaagcc ttttttgcgg atggattatt 576060
gatttttgcg gatgatttcc tccagttggg gcatcgggct gtagccgctt tggctgcgcc 576120
cgttggggaa gacgagggtc ggcgtgccgt tgaagccgaa ttgttcgccc aaggaagtgg 576180
tttccgcgac gggattgtcg cagatgctgc cgccgaccgg gaatttgcct ttacgcatcc 576240
aatccgtcca cgctttggcg cggtcgggct gacaccataa gatttgcgcc ttgcgcgcgg 576300
catcggggtg caggccggca atgggcatca taaagctgta aaccgtcacg tcggtcattt 576360
tttcaaactc gtgttccaag cgtttgcaga acggacaatc ggggtcggag aagacggcga 576420
ctttcagctt gccgttgccg cgcacttctt tgatggcttt gtccaaaggc agggaggcga 576480
agtcgatttt gttcaaatcg gcggcgcgtt cttcggtcag gtttttgcgc gtgtcgatgt 576540
tgatgagttc gccgacgaac atatagccgc cttcggcatc ggtgtagata atctgcctgc 576600
cgctgacgac gacttcgtaa atgcctttga ccggtgtttc gctgacgctc aacactttca 576660
aatcttgggc ggaataggtt ttttccaaac gcgctttcaa agaggcggca acggatttgc 576720
cggcggactc ggctttgacg gcgggttcgg cgttggcatt ggaaacgggc gtttgcccgc 576780
aagccagcag cgggaggacg gtaaaggggg tcaagatttt gattaacttg gttttcatat 576840
aaagatgatt gcgcgtgttg gaaaagcgga attgtatcaa atctctgttg cgcctgcatt 576900
gcgcctaggc tcaatttatc gtctgaaaat agcttccggc tgttaaaata cgcaaaaaat 576960
gatttgcttg tttgtatgat ttaccaccgc atcgccgtaa acgtgccgct ttcagacggc 577020
cttttgactt attcccattc cgatccgctt cctccgggaa cgcgggtgct tgtgcctttc 577080
cgcaataaaa ccgtggtcgg gatggtgtgg gaaacggata ttgcgcccga tatggatatg 577140
gcgcggattt tgagtgttca gacggccttt gtggaagaaa agccgttgcc tgaaagctgg 577200
cgtgatttgt tggcatttac gtcgcgttat taccactatc cgactgggca ggcggtgttt 577260
gccgcgctgc cgcagggttt gaaggaaacg cgcgcggtgg aaatgccgca gccgccgttg 577320
ttttatgctt tgaacgaagc gggcagggcg caaacgccgc caccagctcg gttcaacaaa 577380
aaagcggctt tgtgggacgc actgctttcg ggcggaatga cgatggcagc gttgaagcag 577440
gtaaacgcgc aggcggcgaa attgattgaa gattgggcgg agcagggttg gattgaaaca 577500
acggaagcgg cgaaacctgt attgaggtcg taccacgggc aggcttcgca ctctgaattt 577560
gtgttgaatg ccgaccagca acaggcttcc gatgaaattc agacggcctt cggcagcttc 577620
cagccgtttt tgctgtacgg catcaccggc agcggcaaga ccgaggtgta tttcgatgcg 577680
atggcgaaag tgttggcgca ggggcggcag gtgttgtttc tgttgcccga aatcaacctc 577740
acgccgcagc ttttgaagcg ggtggaaaac cgttttgccg acgtgccgac cgccgtgttg 577800
cacagtcaga tggcggcagg caagcgcacg caggattatt tgcgcgcgat gttggggcag 577860
gcgaaattgg tcatcggcac gcggctggcg gtgttcacgc cgatggatga tgtcgggctg 577920
attgtggtcg atgaggaaca cgacggctcg ttcaaacagg acaacgaatt gcgctaccac 577980
gcccgcgatt tggcggtgtg gcgggcgaag cagggcggct gcccgatcat attgggcagt 578040
gccacccccca gcttggagag ctggcacaag gcgcaaagcg gcgcgtaccg cctgctgcaa 578100
ctgaccgaac gcgcccatac cgccgcgcaa ctgccgcaag tggacatcct caacgtaggc 578160
cgtctgaaac ttgacaacgg cttctcgccg caagccttgc agcttttgaa acagaacttt 578220
gaagcaggtg gcatgtcgtt ggtgtacctc aaccgtcgcg gcttcgcgcc cgcgctgttt 578280
tgcggcgact gcggttatac cttcggctgc ccgaactgct ccgccaaaat ggtgctgcac 578340
caacgcgccc gccaactgcg ctgccaccac tgcgaccacc gcgaacccat cccgtacaaa 578400
tgccccgact gcggcaacca agacctgacc gccgtcggcc acggcacgca gcgcgtcgaa 578460
gaaaccctgc gcaccttcct gcccaaggca gccgtcgtcc gtgttgacag ggacagcacc 578520
gcgcacaaaa acgactgggc ggatttgtac cgccgcatcg ccgacaacaa aatcgacatt 578580
ttggtcggca cgcagatgct cgccaaaggg catgatttcg cgcggctcaa cctcgttatc 578640
gtgttgaacg ctgacggcag cctgtacagc gcggactttc gcgccccgga aaggctgttc 578700
gccgagctga tgcaggtgtc cggcagggcg gggcgcgccg acaaacccgg caaggtgttg 578760
atacagaccc aactgcccga acatcccgtc ttcgccgccg tcaaagcgca ggactacgcc 578820
gtgtttgccg aaaacgaatt gaacgagcgg caaatgttcg ccatgccgcc cttcggtttc 578880
cagaccgccg tccgcgccga cgcgccgcgc gttgccgatg cgatggagtt tctcaatgcc 578940
gccaaagaaa ccctcgcccc gcttttgccc gaaagcgttt cacagttcgg cgccgccccg 579000
atgctgatgg tgcgcctcgc cgaacgcgaa cgcgcgcaaa tcttcctcga atctccgtcc 579060
cgacaggatt tgcaccgtgc cgtgagtttg tgggcgcagg tgttgcagca aaaccgcgac 579120
ggcaaaatca gatggtcggt ggatgtcgat ccgcaggagg cttgattatt ggcaatccga 579180
tgccgtctga aaaccgtttc agacggcatt tttattccgg atcgtctgta aacgcattcg 579240
cccgaaatat cggtataaac gtgaaaagat acagtacgaa tacggcggcg gtcagaatcg 579300
caggaacggt aatgaaaaat atcgggttca cgttcatcaa gaaagcgcgc gagacggcgg 579360
cggcgaaaag gatggggacg gcaatgcggc agagtttggg gtagtcgagt ttggtaaagc 579420
cgctgtgcca cagtccggcg gtcagccaca ccatcatcac gccgcccatc atgccgccga 579480
gggtaatcag gtgcaggggc gcggaggcgg gcaggttttg taatttcgcc gcgcctgtcc 579540

acaaatagcc tgcggcggca aagagttgga gcaggtaata agtgcggacg tagtgtttac 579600
gtaagagttc gtgatggtga agctcacgca gcttggcgag caggatgaag ccgacggcga 579660
gcgcggtaaa accggcggtt tgcgcgggca gccaaagttc ggcggcggcg tgcaagagca 579720
ggaaagtaat ggcgatgttt ttataaacga tatttggaat aaaaacaggg tctttcagac 579780
ggcattcttt cagggcttcc gcgcccaaaa gaatactgac gcgcacggat acgaacatca 579840
ccgccgccat atttagatgc acttgcgcgc gcaacaggtt caaatcgccg ctgacggcat 579900
atgccgtctg aaaaacagtg aacgcggcaa gtaacattag cagggcgaag ttgtcggtgt 579960
ttcggtctag ccaaatcagc cgggcgcaga acagcagcaa caccagccaa taggcggcga 580020
cgaaaaacga ggcagtttgc ggcgaaaagg gcagtatagc ggatgcggcg agcaataatg 580080
ccgccatcaa agtcgcgaca ggtttcaggt tacccgaaaa acccgtccag tccaacaaag 580140
ccgcagtcaa aaaaccgccg tatgccgccg gcagcataag ttccaagaaa atttggcggt 580200
gcaggacgat ggcaccgggg ttgatgaaaa acaccagcgc accgagtatg gcaagcaccg 580260
ccgcgccgac gaaaaacggc cgcatagcaa ctgtattttt caccccgtcg ggcaaaaata 580320
ccaaaactca aatcaagccg tccggatacc gttttcggcg gtatcgtttt cggcaaaata 580380
atcacgcatc cgggcattcg atatcgtcag cagtttgcgc atacatgccg taacggcaac 580440
cttatacggc ttacccttgg acagcaggcg ttggtagaaa tcccgaataa gcggttcaaa 580500
acgtgtcgct gccacggtag ccatatacag tgccttacgc accgcagacc ttccgccaaa 580560
gcagcggctt ttgaatttgg tttcctcgct ctccctcggg tgcggggcaa tgccggccaa 580620
actcgctatc cgtttgtgcg acagccgccc caattcgggc agcatcgcca tcagcgtagc 580680
cgtcgttatc gaaccgatgc ctttgatttg ctccgccact tgggctttgc cgtcaaaatg 580740
cgtgtgggtg tggtcgtcga tttgtttgtc caattcgtca atcagccggt caaaatgggc 580800
aatcagttgt ttgacgcttc cgacttgcgt ttcatgaacc taatgcagac ggtttttctc 580860
ggcagtccgc atatccacca gttggttgcg gcggttaacc aaggcttcca acacttcttc 580920
cacttcggtg ggcgggtggt agggcatggt ttgcgaacct tctttctgcg tcatcatctg 580980
tgcgaagaag gcgagcattt tggcatcttt ggcgtcggtt ttggtcagcg gctgcgattg 581040
ggcaaactga tgcgtctgac gcgggttggc gataatcacg gctatgcctg ctcggcggat 581100
ggctttggcg gcggggattt cgagaccgcc ggtactttcc gtcacgacga gggcgacctt 581160
gtgtttttta aggtattcga tagtatgggc gatacctttg gggttgttgg tttcggtttt 581220
ggttttagac aaagacgaaa cggcgatgac gaagtttcgt ttggcgatgt cgatatagtg 581280
aattaacaaa aatcaggaca aggcggcgag ccgcagacag tacggatagt acggaaccga 581340
ctcacttggt gcttcagcac cttagagaat cgttctcttt gagctaaggc gaggcaacga 581400
cgtactggtt tttgttaatc cactataaca gcaaccctgt cgccgtcatt cccgcaaaag 581460
cgggaatcca gtccgttcag tttcggtcat ttccgataaa ttcctgttgc ttttcatttc 581520
tagattccca ctttcgtggg aatgacggcg gaaggttttt gtttttttcc gataaattct 581580
tgaggcattg aaattccaga ttcccgcctg cgcgggaatg acgattcata agtttcccga 581640
aattccaaca taaccgaaac ctgacagtaa ccgtagcaac tgaaccgtca ttcccacgaa 581700
agtgggaatc tagaatctca gactttcaga taatctttga atattgccgc tgccttaagg 581760
tctggattcc cgcttgcgcg ggaatgacga atccatccgc acggaaacct gcaccacgtc 581820
attcctacga acctacatcc cgtcattccc acaaggacag aaaaccaaaa tcagaaacct 581880
aaaatcccgt cattcccacg aaagtgggaa tctagaaatg aaaagcaaca agcatttatc 581940
ggaaataact gaaaccgaac agactagatt cccgcctgcg cgggaatgac gaatccatcc 582000
gcacggaaac ctgcaccacg tcattcctac gaacctatat cccgtcattc ccacaaggac 582060
agaaaaccaa aatcagaaac ctaaaattcg tcattcccgc gaaagtgtga atctagaaat 582120
gaaaagcaac aggcatttat cgaaaataac tgaaaccgaa cagactagat tcccgcctgc 582180
gcgggaatga cggctgcaga tgcccaacgg tctttatagt ggattaacaa aaatcaggac 582240
aaggcgacga agccgcagac agtacagata gtacggaacc gattcacttg gtgcttcagc 582300
accttagaga atcgttctct ttgagctaag gcgaggcaac gctgtactgg tttaaattta 582360
atccactata taaaaaattt ccagagaacc gatacaacag ttggaacttg ggtttgggaa 582420
tattacggta gatgaacttg gaacctctgt tatgctatgg tcttttatct caattgaaaa 582480
aagcgcgaat caaacggttc gcgctttttt cagacggtat taattatttt ttgtcgtctt 582540
ttacttcttc aaagtcggca tctacgacat catcgtcttt ctttgcagaa gcattggctt 582600
gttcgctttc gcctgcttgg gcttcagctt gtgcttgagc gtaaaccatt tcccccagtt 582660
tttggctggc tgcgcccagc gcctcggttt tggcatcgat agcggctttg tcgtcgcctt 582720
taactgcttc ttcggcttct ttcagcgcgg cttcgatttt ttctttctcg gctgcgtcga 582780
gtttgtcgcc gtagtcggcc aaagattttt tcacagagtg aatcagggct tcggcttggt 582840
tgcgggaagc gaccaattca gtcagttttt tatcttcctc ggcattggct tcggcatctt 582900
tcaccatgcg ttcgatttct tcttcgctca aacctgaaga accttggatg gtgatgttgg 582960
ctgctttacc ggtgcctttg tctttggcgg aaacgtgcag gatgccgttg gcgtcgatgt 583020
cgaaggttac ttcgatttgc ggcataccgc gcggtgcagg tgcgatgtcg cccaagttga 583080
actgacccaa agatttgttg gcagaagcgc gttcgcgttc gccttgcagt acgtggatgg 583140
ttactgcgct ttggttgtct tcggcggtag agaacacttg cgacgctttg gtcgggatgg 583200

tggtgttctt ctgaatcagt ttggtcatca cgccgcccat ggtttcgata cccaaagaca 583260
gaggagttac gtccagtagc aatacgtcgc tgcggccgcc gctcaatact tcgccttgga 583320
tcgctgcgcc tacggcaacg gcttcgtcag ggttcacgtc tttgcgcggt tctttgccga 583380
agaaggcttt aacggcttct tgtactttcg gcatacggga ctgcccgccg accaagatta 583440
cgtcgtcgat gtcgccggtg ctcaagccgg catctttcaa tgcaattttg caaggttcga 583500
tagagcgggt aatcaggtct tcaaccaggc tttcgaattt ggcgcgggta attttcatcg 583560
ccaagtgttt cggccggtt gcgtccatgg tgatgtacgg caggttaatt tcggtttgct 583620
ggccgctgga caattcgatt ttggcttttt cggcagcttc tttcaggcgt tgtagagcca 583680
tcacgtcttg tttcaaatca atgccttgtt ctttttgaa ctcggcgatg atgtggtcga 583740
tgaggcgttg gtcgaagtct tcaccgccca agaaggtatc gccgttggtt gccaatactt 583800
cgaattgttt gtcgccgtcg aggttggcga tttcgatgat ggaaatatcg aaagtaccgc 583860
cgcccaagtc atatacggct actttgcggt ctttgttgtc gcctttgtcc ataccgaatg 583920
ccaaagcggc tgcggtcggc tcgttgatga tgcgtttcac gtccaaaccg gcgatacggc 583980
ctgcgtcttt ggtggcttga cgttggctgt cgttgaagta ggcagggacg gtaatcacgg 584040
cttcggttac tttttcgccc aagtaagctt cggcggcttc tttcatttta cgcaggactt 584100
ctgcggaaat ttgaggagga gacagctctt tgccttgtgc ttttacccat gcgtcgccgt 584160
tgttggcttt gatgatttcg aaaggcatag attcgatgtc gcgttggact tctttgtctt 584220
caaatttgtg gccgatcaaa cgtttggcgg cgtaaatagt gtttttggcg ttggttaccg 584280
cttggcgttt ggcaggcgca ccgacgagga tttcgccgcc gtccaaataa gcgataacgg 584340
acggcgtggt gcgtgcgcct tctgcgtttt cgatcacttt ggtttgaccg ttttcggaaa 584400
tggccaaaca agagttggtt gtacctaagt cgataccgat tacttttgcc atgtggataa 584460
tcctatttga ttttgcttat tttgagaaat atgttggaac attttgtccc gatgggctgt 584520
aaatagggcg ggcggcgggc tgtttcaagc tacagcatgg ctataagtat ataactttat 584580
gaatatattg gtttatatt tgatttaata catttggctc caatgcattc aagcataatg 584640
tttcaaatgg caggcaggtt tattcataga cgatgccggc gagcatttcc tgttcgttca 584700
agttgccgta ctctttttcc cagtcgtgtg aagactcgat gatgtcgcat tctttggaaa 584760
gggagacttg ttctgcatcc atatctttgg cgttcagtat gttgaattgt tcgcacaggg 584820
atgcggataa agtgatgtcg ggctgtttgg cttcagaacg gttttcttgg aaggcaaagc 584880
agaatgcggt aaatgccgca gtatagataa gatatttgcc ggtttcttc atttttctat 584940
cctttttctg tcaattcagg attaaaccta tggaaaaatc tgaaaaatta tgtattaagt 585000
aagaaaaatc ataatttaaa tttagtttat cataattgtt ccgtttttg gatagctaag 585060
gtaaaatata tttcatgttt actttagatg attgaatgaa ggggagtgga aggatattta 585120
tggaaacctt taaagacaga ctggtttttt tatggaaaag cgaagcgagg caggcaaaaa 585180
tcgcatccga tattgaaatg acgattgcgg gcttcagcag gatatggaat gaaggcggtc 585240
tgccaaagtc tgaaacattg aaaaaaatca agcagttgaa ggggtgtagt atcgattggc 585300
tgctgaccgg ggagggtaat ccgtttccgg atgaagcccc aaaaaaatcc cttgcttacg 585360
atactttggg caatgaagtc gatacggacg agtttgtctt cgtgccgaga tatgatattc 585420
gggcggctgc gggatacggg cagtttgtcg atcatgagga accggtattt acaatggcgt 585480
tcagacggca ttggattgag aattatgtta cccgcgatac gaaaaacctg tctgtaattt 585540
ccgtcaaggg ggattcgatg gagggggttt tgaatgacgg cgattcgatt ttggtcaatc 585600
atggtgaaaa tacgccgagg gacggtctgt atgtgttgcg gattaatgaa aatctgctgg 585660
ttaaacgttt acagattgta ccgggcggga ttatcaatgt gatttctgca aacgaggctt 585720
atcctgcttt tgaaatcaat ttgaacgatt tgaccgatga tgtggagatt atcgggcgtg 585780
tcgagtggtt cggcaggacg atttgagttt ggggcttgaa attgcaggcg gtcaaactta 585840
tctattggaa caattccttt ttcaaaggcg aagcctgctt gcctttgaag gggtttgag 585900
agagaatgca gaaaatatta tattaaggaa taacaccatg tcggatgaaa gccctattat 585960
ttttactgac agctgctgtg ccaaagttgc cgatttgatt gccgaagaaa acaatcccga 586020
tttgaaattg cgggtttttg tcaatggcgg cggctgttcg ggtttccagt acggatttac 586080
ttttgacgaa atcaaaaacg acgacgattt tgaaattgag aaaaacggtt tggtcttttt 586140
ggtcgatccg atgagctatc aatatctggt cggtgcggaa atcgactata cggaaagttt 586200
gcagggttcg caattcgtca tccgcaatcc gaatgcggaa acaacctgcg gttgcggatc 586260
gtcgttttcc gtatgaccgc ttggtttgtg tgatgccgtc tgaacgttca gacggcattt 586320
ttacttttag aaaatatatt atcgggatga attcacatat aatccgattg tttgaagatg 586380
aatcgggttt cccgaaagga acgggcggaa cggtatcagg cgtatttgtt cccttatgat 586440
tgagatgagt aaagattacc gaaacgattt gtacgatgta tatgtttctt acccgcccca 586500
agtggatcgc gggcttatcc gggagtgcct taaggagaat ctcggcgagg aaaaggcgga 586560
aggattgatc gaatcgctcg attccaaacc tcaagtgctg gttgaggaaa aatgcacttg 586620
ggcgaaacgg gaagagttgc atgattattt cagctatttg ggtttggata ttattacccg 586680
gagatatatg gagttggaaa cggtcgtgcc gccggaggaa ggggagggcg aaggagaagg 586740
cgcggatggg gaaatgcccg aatatcttga acttcacggc gggcgggaag atgatatttc 586800
cgcaccttcg caacccgaac cgccgtcccg caatatcaaa ctgctggttt tcgggctgct 586860

gattgccttt ttgggctatc tgctcggtaa gatttttga ttgtccgata aatgctgtat 586920
tcgggatttt atatatgaaa tggttgaaac gcctgacggt tattgtcggg actttttacc 586980
gctatcggct ggcaggtctg tgtgcttcgc tgatgggtag cggttggata tgcgctctgc 587040
tgaaaatgat gccgcagtcg tccaaattga aaaacgaacc gcctgctgtc cgtctgcgcc 587100
ttgccttgga aagcctgggg ccgattttca tcaagttcgg gcaggttttg tccacacgcc 587160
ccgatttgat tccgcacgat tacgccgtcg aactggcaaa gctgcaagac aaagtgccgc 587220
cttttgacgc gcggctttcg cgtgaacaaa tcgaaaaatc gttgggtcag tccatcgaaa 587280
agctgtatgc ggaatttgaa accgagccca tcgccagcgc gtccatcgcc caagtacaca 587340
aagcccgcct gcattcgggc gaacaagtgg cggttaaagt tttgcgcccc aaccttttgc 587400
ccgtgatcga acaggatttg tcgctgatgc gctttggtgc aggctgggtc gagcgtctgt 587460
ttgccgacgg caagcgtctg aagccgcgcg aagtggtggc ggagttcgac aaatatctgc 587520
acgacgagtt ggacttgatg cgcgaagccg ccaatgccag ccagctcgga cgcaatttcc 587580
aaaacagcga tatgctgatt gtgccgaagg tgttttacga ctactgcacc agcgacgtgc 587640
tgaccatcga atggatggac ggcacgccgg tttccgacat cgccaaactc aaagcagacg 587700
gcatcgattt gcacaaactc gccgattacg gcgtggaaat cttcttcacg caagtcttcc 587760
gcgacggctt tttccacgcg gatatgcacc ccggcaatat tttggttgcc gccgacaacc 587820
gctacatcgc cctcgatttc ggcatcgtcg gcacgctgac cgattacgac aaacgttatc 587880
tcgccatcaa cttcctcgcc ttcttcaacc gcgattaccg gcgcgtcgcc accgcccaca 587940
tcgaatcggg ctgggtgccc gccgacacgc gcgcggaaga gttggaagcg gctgtccgcg 588000
ccgtgtgcga accagtgttc aacaaaccga tttcgcagat ttccttcggc ttggtgctga 588060
tgcgcctgtt tgaagtcagc cgccgcttca atgtcgaaat ccagccgcag ctggtattgc 588120
tgcaaaaaac gctgctcaac atcgaaggct tgggacggca gcttgatccc gatttggact 588180
tgtggaaaac cgccaaaccg tttttggtga aatggatgaa cgggcaggtc ggccctaaag 588240
ccctttggcg caacctcaaa aacgaagccc ccgactgggc gcaaatcatc ccttcattgc 588300
cgcgcaaaat cagtgcgttg attgatgaaa accgccagca ggaaatgcgt gatgcctata 588360
tccatttggt caaagtgcag cagcggcaaa gcctgtggct ggctgtgatt gcggttgttt 588420
tgctgctgat tttgcttttg aaataggctt tgtccgaatc atcgcccgac tccgcccgtt 588480
tataaggaaa tcggttatag tggattaaca aaaaccagta cggcgttgtc tcgccttagc 588540
tcaaagagaa cgattctcta aggtgctgaa gcaccaagtg aatcgattcc gtactatccg 588600
tactgtctgc ggcttcgtcg ccttgtcctg atttttgtta atccactata tttccggttg 588660
cgtgggaatc gggtgtattg aataaaaggc attttgtccg actggcaagt gccgacatcg 588720
gcggcatatc aaggcgcagg cttgaagcgg gcaatgtcgt ctgaagcccg tttggcgttt 588780
cagacggcat tggtgcggat attcaaatca taaagtcgat ttcggtaaac tggatatttt 588840
gatccatatc cgccgacggt gttttgagcg atcgcgccac gggtttggcg ggtacgccga 588900
caaccgtgat ggacggcggc acgtctgaaa ccacgacgct gcccgccccg attttggcat 588960
tgctgccgat gcggatattg cccaatatcg aggcgtttgc gccgatcatc acgccgtcgc 589020
cgattttagg gtggcggtcg ccgccttctt tgcccgaacc gccgagcgtt acgccgtgca 589080
aaatcgaaat attgttgccc aacacggcgg tttcgccggc aacaaagccg gtggcgtggt 589140
cgagcatcag cccgtatccg aaacgggcgg cgggatggat gtccacgccg aatacttcgg 589200
acatacggtt ttgcaggaaa tacgccagcg ttttgcgccc gtcgagatac agccgatggt 589260
tgatgcggtg tgcctgaatc gcgtggaagc ctttgaaata taaaagcggc agcgaatatt 589320
cgtcgcaggc gggatcgcgt tcgtagatgg cttttaagtc tgcttcgacg catttgccga 589380
tttgggtgtc gctgcccaac gcctgctggt agatttcaaa cagcgcgcgc acgtccataa 589440
tcgggctgcc gagtttgctg gaaaggtggt aggcaaggac ggagccgagg gactcgtggc 589500
gcaacacggt ttggtgcaaa aaacttgcca gcatcggttc ggcggagacc gcggccgcgg 589560
tttcttcgcg gatggtgtgc cagaggtcga aaccggttgt gtttaaatgg tctttttca 589620
tgagtgatga cgtttgaaaa tcgatatggt cggcagtatc ttaccgtcta tattattttt 589680
tcggtagggg atttgaaaat gaatttgaaa ttctctgctt ttgcttgaag tttcttgaaa 589740
atgtccttat cttgcgcggg taataactgg attttgattt ccaatttgtt ttaagggata 589800
cgatatgagc gaacagacac agcagcaaaa cagtgaagaa gcggttgaaa atgtggaggc 589860
ggtggaaacc gtcgagacag taggaaatgc ggacggtgtg caggaacagg ctgccgcaga 589920
gccggcttat gaggatttgc aggcgcggat tgccgagctg gaagcgcagt tgaaagacga 589980
gcagctgcgc gctttggcaa acgagcaaaa cctgcgccgc cgccaccagc aggaaattgc 590040
ggatacgcac aagttcgccg gacagaagtt tgccgtggaa atgctgccgg tcaaggatta 590100
tctggaaatg gcgcttttgg atcagagcgg caatttcgat gcgctgaaaa tgggcgtgca 590160
gatgactttg aacgagttgc agaaagcatt tgatgctacg caaatcaagg aaatcaaccc 590220
taaagcgggc gataagctcg atccgaatat ccatcaggcg atgcaggcgg tggcaagcga 590280
acaggagccg aataccgtgg tgggtgtgat gaagaagggt tatacgctgt ccgaccgcgt 590340
gttgcgcccg gctatggtta cggtggcgca gaaggaagcc tgaaggcgtc tggggaataa 590400
tctgatttat ttcctgaagc gcgttttgcg tataaaccga tcgaagtaaa gcggcaatgc 590460
cgtctgaacc cgcctgtcgg gcttcagacg gcattttata gtggattaac aaaaatcagg 590520

```
acaaggcgac gaagccgcag acagtacaga tagtacggaa ccgattcact tggtgcttca 590580
gtaccttaga gaatcgttct ctttgagcta aggcgaggca acgctgtact ggtttttgtt 590640
aatccactat atttcggcgt taacggtcaa cccgtatgcc gcctgcctgt ttttcttcat 590700
ccagtttctt ttgcagggtg tcgcaaggtg tgtcgcagtc gcacattttt ttcataccca 590760
aggcagtaat gccgccgcaa ctgcctttga tgctgcgttt ggagaaaata tagccgaccg 590820
ccataccgat gatgacggtc aggaagatgc cgaaggtaag gagcagggtt ttcatggtgt 590880
ttcctaatcg gtttgtatgt ttagcggagc agtttttcaa attcggaaga catggcggtg 590940
cggtagccgc ctttatccct gacaatcagg aaaacagcga gtttttcgcg ctctgccagc 591000
tttaaggctt cggtttcgcc caatacgaat aatcctgtgg acaagccgtc cgccgtcatc 591060
gcactgtctg cgaccacgct gatggaggcg aggttgtggc tgatgggtcg tttgttgttc 591120
gggttgatga tatgggagag gcgtttgccg tttttatcga cgtggaaaat acggtaatcg 591180
ccggaagtgg caagcgaacg gttgttcagc gggacgataa tctgcgtatt gccgccttgg 591240
acgatattgg gctgctcgat accgatgcgc cacggttcgc cgcgcgcgtt tttgcctttg 591300
ccgtgcaact cgccgccgat ttcgaccaga taattttgaa tgccgtattt ttccagttcg 591360
cccgcaactt tatcaacgcc gaagcctttg gcaatcgaag ataaatccaa ataggccttg 591420
gggtgggttt tgctcaagga agcgtaatct ttgccttgtt tcaaaatgat tttgtctatg 591480
cccgtataag atgccgcctg tttgatttgt tccggcgacg gttcacgggt aacggatttg 591540
tcggggccga atccccaaag gttgaccaag ggccgacgg ttacgtccag cgcgccgtgt 591600
gtcaggcggt tcaggcggac ggcttcggca gtaacgtgtg cgaagtcgct tgaaatgcgg 591660
aggggcttgc cggctgtgtg ttggttgaac cggctgattt cggagtcggg ctgataggtg 591720
gacatctgcc ggttgacttc tttaagcgcg tcatcgatgc gtttttgtat ttcggcaggt 591780
gaggggagtt tgtcccgatt atttgaaagg tatttgacgg tataggtcgt gcccatcgtt 591840
tcgccttgca gggtaacggt ttgcgcggtt tgttccgaac aggcgttcag gaagatgaaa 591900
cccagggcaa atatcaagac gcggataaag ttcggcaggc gtgtttcaga cggcatagtg 591960
tttgacggtt ttggcaaatg gtttgaatta tatcgcaaaa cggccggtat gtttctatgc 592020
cgatgccgtc tgaagggtgt tcggatggca tcggcataga aaaaggaaga aaccgaggtt 592080
tcttcctttt gtatttgaag ccgaatattt aaccgccgaa atcgtccaag aggatgtttt 592140
cgtcttccac gcccaagtct ttgagcattt tgatgacgga ctggttcata atcggagggc 592200
cgcacatata aaattcgcag tcttccggtg cttcgtggtt tttcaggtgg ttttcgtaaa 592260
ccacgttgtg aatgaagccc gtgtagccgt cccagttgtc ttccggcagc gggtcggaca 592320
gggcgacgtg ccacgtgaag ttcgggaact ctgccgcgag ttggtcaaag tcttcgacat 592380
agaacatctc gcgtttggaa cgtgcgccgt accagaaggt aatcttacgt ttggagttca 592440
aacgtttcaa ctggtcgaaa atgtgggaac gcatcggagc catacccgca ccgccgccga 592500
taaataccat ttcggcatcg gtgtctttgg cgaaaaattc gccgaacggg ccggaaatcg 592560
taactttgtc gccgggtttg agcgaccaga tgtaggacga catttgtccc ggaggcgcat 592620
caggtacgcg cgggggcggc gtggcgatac gcacgttcag cataatgatg cctttttctt 592680
caggatacga agccatagag taggcacgca aaatcggctc gtccactttg gaaacgtatt 592740
gccacaaatt gtatttgtcc cagtcttcgt gatattcctt aggaatgtcg aagtctttgt 592800
aggcaacagt gtgaggagga gcttcaattt gaatgtagcc gccggcgcgg aaggggactt 592860
cttcgccttc gggaatggca agcttgagtt ctttaatgaa cgtggctttg ttatcgttgg 592920
agatgacggt gcattcccat tttttcacgc cgaacacttc ttcggggact tcgatgtcca 592980
tgtcggtttt gacgttgact tggcacgaca gacggcagcc ttcgcgtgct tcgcgtttgc 593040
tgatgtggga cagctcggtc ggcaggatgt cgccgccgcc gctttttacg acgacgcggc 593100
attgtccgca cgaaccgccc ccgccgcagg cggaggggat aaagatgcct tcgttggcaa 593160
gcgcgcccaa gagtttgccg ccggcgggca tcgtcagctc tttttcgccg ttgactttga 593220
tggtgatgtc gccttcgctg accagtttgg atttggcaaa cagaatcatc agtgccaaaa 593280
ccaaaacgat gacggtaaac atcacgatac ctaaaataat ctccataccg atccctttct 593340
tataactgga tgccagagaa cgacataaac gccatcgcca tcaggccggc ggcgataaag 593400
gtaatgccca agcctttgag gcctttggga gcgtccgaat atttcatttt ttcggtaatg 593460
cccgccaaag cgacaatcgc caacatccag cccaagcccg cgccgaagcc gtatacaacg 593520
gactcgccga agttgtattc gcgttgcgcc ataaacgata cggcgccgaa aatcgcgcag 593580
ttcacggtaa tcagcggcag gtagatgccc aatgcgttat agagggcggg gacgaattta 593640
tccaagaaca tttccaaaat ctgcaccaaa gcggcaatca cgccgatgaa ggtgatgaat 593700
ttcaaaaagg tcaaatccac gccttcggca atcgcgccgt ctttgagcag cgagtaaacg 593760
agttggttga cagggacgga cagcccgagt acgaaaatta ccgccacacc caaaccgaat 593820
gcggtggata cttttttgga taccgccaaa aacgtgcaca tacccaaaaa gaaggatagt 593880
gccatatttt caatgaagac ggatttgatg aagaggctca aatagtgttc catagcttat 593940
tcctccgcct gttcgggttt ccaggtacgc agtccccaaa tcaaaaagcc gatgatgaag 594000
aacgcgctgg gggcgagcag gaacaagccg ttggtctgat accagccgcc gtcctgcacg 594060
gtttggaaaa cggtgtagcc caagagtttg cccgagccaa tcagttcgcg gacggtggcg 594120
acgacaagca gcattatccc gtagcccgcg ccgttgccga tgccgtcgat caggctttcc 594180
```

agcggcggct ctttcatcgc aaatgcttcg gcgcggccca tcacgataca gttggtaata 594240
atcagaccga cgaatacgga aagctgtttg gacaattcgt aggcaaatgc ctgcaagagt 594300
tggtcgacca gcgtaaccag cgacgcgata atcgccattt gcacgataat acggatgctg 594360
ttggggatgt agttgcgtac cagcgaaatg aagaagctgg aaaaaccggt taccaaagct 594420
acggaaatac ccatcacgat ggccgtctga agtttggtgg taaccgccaa agccgaacaa 594480
atacccaaaa cctgcaaggc aatcgggttg ttgtcgataa agggtgaaaa catcaaatgt 594540
ttcaagcgtt tcatatcagc cattattgcg ctcctgctga tttcaatttg ttcaggtagg 594600
ggatatagcc gttttcgccg aaccagtagg cgaacgaacc ttgcacgcct ttggatgtca 594660
gcgatgcgcc ggagagggca tctacgccgt gttctttgtc cgaacccgcg cctttgccga 594720
cgtgcagggc gagtttgcct tgtccgtcaa acagtttttt gccgacgaat tttgctgcc 594780
acaacggatt gccgatttcg ccgcccaagc ccggggtttc gccttgttcg tagtaggtaa 594840
tgccgttgat ggtgttgccg tcgggctgga tggcgacaaa gccgtacatg accgaccaca 594900
aaccgttacc gtgcataggc aggatgattt gcccgatttt gccgtcttcg ccttttacca 594960
aataaacctc ggtgtatttg gcacggcttt tgatgcctgc caaatcgtct tcggttttga 595020
tgcggatgct ttgggcaggg tctttgcctg cgatgcgcgc gctgaagtct ttaggcgcat 595080
cggcgacgta ttcgccggtc gccaaatcga caacacgttg ctcgatacgc tcggcaaagg 595140
ttttaccgat gtcggtgtcc ttatccatca aaccggctac gctcaagata tagccttgtt 595200
tgtcttggag tttttgtttc tcttggatgg gtttcaagcc gacgaccgca ccggcaacga 595260
tgaccgagca aatcaggctg accgccaaca cgacaatcag cgtgccgctg aagctgtctt 595320
tatcgaattt cttagccatt gctgcgcgcc tttctgcgtt tgatgttcgc ttgtgcgacg 595380
aaatagtcga aaatcggggc aaacaggttg gcaaacagaa tcgccaacat catgccttcg 595440
gggtaagccg gattgaccac gcggattaat acgcacatca caccgatcag tgcgccgtac 595500
caccatttgc cgacattggt aaaggaagcg gaaacagggt cggtcgccat aaacagcata 595560
ccgatggcga agccgccgac caccaagtgc cagtaccaag gcatagcaaa catagcgttg 595620
gtgtccgaac cgatgaagtt gaacagcgaa gacatcgcaa tcataccgat catcacgccg 595680
gcaataatgc gccaagaagc gatgcgggca aacacgataa acgcgccgcc gattaagagt 595740
gccaaagtgg agacttcgcc aatggagccg ggcagtttgc cgataaacgc gtccatccaa 595800
gtgatggttt gaccggttac ggcgtttttc aggccgtctg caccgtgtgc cgcccattgc 595860
gccagtgcgg ttgcgccgga atagccgtca accgccgtcc aaaccgcatc gccgctcaag 595920
ttggcagggt aggcgaagaa caggaaagca cggcctgcca gcgcagggtt catgaagttt 595980
ttacctgtac cgccgaatac ttctttcgca accacaacgc cgaaagaaat acccaaagcc 596040
gcctgccaca gcggcagcgt gggcggaacg attaaggcaa acagaatcga agtaacgaag 596100
aaaccttcgt tgatttcgtg tttgcgcacg gtggcgaaca aaacttccca gaaaccgccc 596160
acaacaaata cagtcgcgta aatcggcagg aagtaaatcg cgccaaacag cattttgtcc 596220
gacacgcccg cttcagacga catattgatg cccaaagcgt tggcaaaggc gtaatgccag 596280
tcgttggcga tgttttgttg cagcaaatca ggcgttaacg caccgaatgc ctgcgcgccg 596340
acgttgtaca taccgtagaa catggcaggg aacaaagcca gccacaccaa aatcatcatg 596400
cgcttggagt cgagcgcgtc gcggacgtgc gccgctttgc gcgttaccgc gccggatgtg 596460
tagaaaattg tcgccgcagc ttcgtagagg gcataccatt tttcatgttt gccgcccggc 596520
aggaagtgcg gttcgatttt ttccagaaaa tgtttcaagc ccataatcag ccttccttct 596580
caatggtttc cagcactttg cgcaacagcg ggccgtattc gtatttgccc gggcagacga 596640
agctgcacaa agcgaggtct tcttcgtcca attccaagca acccaatgcc tgcgcgctgt 596700
cggtatcgcc gacgattaaa tcgcgcaaaa gcagggtggg caggatatcc aagggcatca 596760
cgcgctcgta agtaccaatc ggcaccatgg cgcggtcgcc gccgttgacg gctgtgttga 596820
acttgaagag tttgtttttc aggaaatggc cgagggttgt acgcgtgatg gagtatttgt 596880
ccggctgcgg cgcaacccag ccgaacagct ctttgctgcg gccttcttcg ataacggaaa 596940
tctgattgtg gtagcgtccc aaataatcgt gcgcgccttg tgtaatcgcg ccgttcaata 597000
ccgaaccgga aatcacgcgg ttgtctgtgt caaccaattc gcccgcagta atttgcgata 597060
ctttcgcacc caaaacggta cgcaagaggc gcggtttgtt gacttgagaa ccacctaggg 597120
caatcacgcg ctcggtgttc agacggcctg ttgcaaacaa acggccaatg gtaattacat 597180
cttgataatt gatggtccac acggttttat tcgcgccgac cggctcgatg aaatgaatgt 597240
gcgtgccact caaaccggca ggatgcgggc cgccgaattc atgtgtttcg atgttggcag 597300
cattttcaga cggcacgtct gcgccagctg ccttacaaac atggattttg cgttcggtca 597360
aacggctcaa taccaacagg ccgcgtttga aatcctcggc ggcttctttg ataatgaccg 597420
tagggtcggc agccagcgga ttggtgtcca tcgcattgac gaagatggcg aacggctcgg 597480
catcgacggc aggaattttg ctgaacggac gggtgcgcag cgcagtccac aaaccggatt 597540
ggatcaggtt gcggcgcact tcttcgccgc ttaagtttgc cagcgcttca ggtgcgtagc 597600
gttcaaactc gatttcgtcg ttgccttcaa cggcaatcac gactgactga agtacgcgct 597660
tttcgccacg gtgaatcgcg gcgattttgc ctgaagccgg cgcagtaaac accacgcccg 597720
gattcttttt gtcttcaaac agcacttggc ctttttttgac ggcatcgcct tccttgactt 597780
tcatcgaggg gcgcataccg gcatattctt cgccaagcaa cgcgacttcg gtaatggccg 597840

ggccgtcgta aacggcttgc tccggtctgc ccgcgatggg caggtttaga cctttttga 597900
ttttaatcat atatttgcat tacttgtgat ggttaaggta aaaacggcgt gttttgatac 597960
cgtgtcgcgt ggcatcaaaa gcattgaata aattaatgta gcaaagtgtt agattctatc 598020
aggaattgta cctgtttgtc agatttgctg cttttttcct tgcggaagcc gtttttatag 598080
tggattaaat ttaaaccagt acggcgttgc ctcgcccttg ccgtactatt tgtactgtct 598140
gcggcttcgt cgccttgtcc tgatttttgt taatccacta taaattgtcg gaagggggga 598200
tattgatttg attatgccgg aatttaaaat gccgtctgaa tgttcagacg gcatagcgtt 598260
tacagcagtt tgaaaacgaa aaagataagg gtatgtacga tgaagacggg tgtcaggaag 598320
gcgaccgacc acatcatata gccgaagaaa gtcggcatcg gtacgccgcg ctgttcggca 598380
atggccttga ccatgaagtt cggtgcgttg ccgatgtagg tcagtgcgcc catgaatacc 598440
gaacccatag aaaccgccag cagcgaatga aacagggtac ccgtcatcaa ggcttgggca 598500
tcgccgcccg ccatattgaa aaaaacgaga taagtgggcg cgttatccaa gaatgccgac 598560
aatatgccgc tcatccaaaa atacatcaca ttaatcggat gacctgccgt atcgtgaacc 598620
agcgatacca ccccgcccag cgcgcctgcc tcgcctgctt tcagaatgct caggacggga 598680
aagatggtga tgaagatgcc gaggaagagt ttgcccactt cggcgatggg ttcaaagttg 598740
aattcgttgc ctgcgcggac ttgtttgggc gtgattgcca tagatacggc ggtcaatgca 598800
atcaggatga catcgcggac gaggttttgc agggcgtaac ggctgccgag gatttcaaat 598860
cccgggtgtt cgggtttcca aaggccggac attagaaccg cgccgaccac gcccgaaagc 598920
aggaggaagt tccatttgcc gaagatggcg attttttcgg gtttttcctg ttgtgccggc 598980
gtatcttgtg caatgctttc ctgtttgaag aaacggttgt cgatgaaata gaaggcggtc 599040
aacaggacag cggtgctgat caggacgggg gcgaacatat gtttgaccgt ccacatgaaa 599100
tctacgcctt tgaggaagcc gaggaagagt ggggggtcgc ccaaaggggt cagaccgccg 599160
ccgatgtttg caaccaggaa aatgaagaag atgacgatgt gcacgcggcg ggtacggttt 599220
tggttggctt tcagcagcgg acgaatcatc agcattgctg cgccggtcgt tcccatgata 599280
gaggcaagtg ccgtaccgac ggcaagcagg gcggtgttga gcttgggtgt gccgttcaag 599340
tcgccccaaa ccaaaatgcc gcctgaaatg gtgtacaggg caagcagcag caggatgaaa 599400
gggatgtatt cttcaacgag tgcgtgtgcg acggtatgga taccggcgga cgcgccaaaa 599460
accaaactga acgggatgag gaagagcaat gtccaaaagg cggtaatttt gccgtaatgg 599520
tgatgccagg tatgcgaaaa aaacaaggga cccaatgcga tagacagcaa aatcagggca 599580
aagggcaggc cccacagcag gtttaggttt gcgccgtcca aatctgcggc gtaaaccgat 599640
gctgggaaaa gcattagtga aaacaggggt aggtggcgca tcgtgtttcc tcgattcaag 599700
cactgccttg cgcggcgcgt gggagtgata caggcaccgt gccgccccgga cataggcgga 599760
ctgtgctgcc tgtttgtttt tgaaaaaagt tttccgattg attgtaaagt aaataatcag 599820
gataaaccag tttcccaaac cggaaggcgg cgggaaggcg gattgctgtg cttgggaata 599880
tatgttcttt ttgataaata attttattta aaacaaataa atatatgtct ttaataaata 599940
atctatcgaa aacgaaaaat gaatttattt taacatatat ttgcaatgaa acaggtttgc 600000
cccccccgt ttgtttgccc ttatcccttt cagtacggca ttcaagattc gggcctgcgc 600060
cacatccata tggcgacaag ggaacaaaaa accgatgaaa ccgccccgac ccaccagcgt 600120
tggggaaact gccaaaacat tatcaggcag gatgcggtca tcatactgat ggcgaatatt 600180
ttggctttgc gcggcactgc gccgttttgt tcccagttat gaaccatcgg gccgaaatag 600240
cggtgccggt gcagccagcg gtaaaagcgc ggggatgcct ttgcccagca ggcggcggag 600300
agcagtacga acggcgtggt cggcaacagc ggcaaaaaaa tgccgatgat acccaacagt 600360
agggaaatgc agccgcaggc aattaaaaga taacgtatca ttttgaaata tttttcttat 600420
tgtgcggata agggcaggat gtgataccga gttttgccca gccttcatgt cccatttttt 600480
ccagcagggc gatattgcgt tcgaatattg ccgaagcgtc gggaaaggct tgtgcggctt 600540
tggcaatgct gtcttcgcgg atgaggtgca gcgtcggata gggagaacgg ttggtgtagt 600600
tgccaatgtc gtctgaatcc gtgccttcaa attggaaatc gggatgaaac ggggcgattt 600660
ggacgatgcc ttctaagccg ttttcgacaa cggcggcatc ggcaatgtcg agcatatcgt 600720
tgaatacgtc gaaatcgggg aatagggtcg ggtgaaccag cagggtggtt tccagttcgg 600780
tggcgggtgt attgcccagt cgctgcagtt cttcgtccaa gtcttccaaa aaaccgtcaa 600840
ggtgtttggc ttcgctgatc gcgatgcgga caaggttttt aacgtggggg gctttggcaa 600900
agggacacag gttcagaccg atgacggctt tttccaacca ttgtccggtg tgttcggcaa 600960
cagcatcttt attttcggaa gtattgatat tcattattgt catgtaaatg tgtttgcaga 601020
ttgcacgtgc gggaaaatcg ggaagggcac tattccttca gcaggtggtt gagcggcagg 601080
gaggtggtgt gtttgatttc ttttaaaaca aagctcgatt gcgcatcttg tacgccgtgg 601140
tgggacagga gcgtatccaa aacaaaatgg gaaaacgcgt tcatatcggt aaaaaacgcc 601200
tgaagcaggt agtcggtttc ccctgtcagg gcgaagcagc tcaagacttc aggccatttt 601260
cgaaccgatg cggcaaagtc ttcccgcgcg tcttttgctt tgcggatgga aacgcggata 601320
aatgcctgaa gtcccaagtt gacagattcc ggagacagca gcgcggcata ttggcggacg 601380
ataccggcat cttccaactg cttcagacgg cgcaggcacg gagaaggcga aagtgcgaca 601440
cgttcggaca gttcgacatt ggtcagcctg ccgttttcct ggagaacctg taagatttta 601500

atatcggttt tgtctaaagt gagttggggc atatttgcgt tccgttttaa ggaattcgga 601560
ttgtctgtcc gtatgtttgc ggcaatccgc acagatggag accatattaa catataaaaa 601620
gttataccgt catccgggac aaattttgtt ttcggaaaat catgtgaaaa cagaggcggt 601680
cggtttgcat ctctttaaga cggcttgccc aaaccgccga ttcaagacat aatcgggaaa 601740
tgtgcaggag agtgttacac ccaactacaa tgtaaccacc gaaggcgcag acacccttaa 601800
atcgctcagg tatcagggac tgcacattga aacaaacaat ctggagagcg gcgttggaat 601860
aacgtccacc gaaggggaga aggccgtctg aaccaccatt cagacaaccg cgcaaagcag 601920
tgagcagact ggtttgccat catgcggata cagccgaaaa tctcaggttc aaggacagat 601980
agggtcatcc gcgcacaggt gcgcgggcgg catctgaaca aaaaatccgg agaaacttga 602040
gaatgactgc tctgaaaacc accccatttc atcaagccca tcaagatgca ggcgcgaagc 602100
tggtcgattt tgccggctgg gagctgccca tccattatgg ttcacaaatc gccgaacacg 602160
aagccgtgcg caccgacgcc ggtatgtttg acgtatccca tatgctcgtt accgacgtag 602220
caggcgcaaa tgccaaagcc tttttccgca aattgattgc caacgatgtc gccaagctcg 602280
cttttgtcgg caaagccctt tattccgctt tgctcaacga caacggcggt gtgattgacg 602340
acttaatcgt ttaccgcacc aatgaagccg aaacccaata ccgcatcgtg tccaacggcg 602400
cgacccgcga aaaagacacg gcgcaattcc acaaagtcgg acaagagttc ggcgtcgcct 602460
tcaatccgcg ctacgacctc ggcatgctcg ccgtacaagg ccctaaagcc attgaaaaac 602520
tcctgaccgt caaacccgaa tgggcagatg tcgtccataa cctcaaaccg ttccaaggcg 602580
cggatttggg caacgactgg tttgtcgccc gcaccggcta caccggcgaa gacggcgtcg 602640
aagtcatcct gcccggcacc gaagccgtcg cattcttcaa agccctgcaa caagccggcg 602700
tacagccctg cggcctcggc gcgcgcgaca ccctgcgcat ggaagccggc atgaacctct 602760
acggcaacga tatggacgac gacaccagcc cgctcgaagc aggtatgggt tggaccgttg 602820
acttgaaaga cgaaagccgc gacttcgtcg gcaaagccgc cttgctggca ttgaaagaaa 602880
aaggcgttgc cgtcaaacag gtcggcctgt tgctcgaaaa aggcggcatc ctgcgcgcgc 602940
atatggaagt gttgaccgac aaaggccaag gcgaaaccac cagcggcgta ttctccccaa 603000
gcctgaaaca atccatcgcc atcgcgcgcg taccgaaaga ttttgacggc gataccgcca 603060
aagtgctgat gcgtggcaaa gaagtggacg tgcgtgtact gaagctgccg tttgtccgca 603120
acggacagaa acagtttgat tgatgcggtt tcagacggca ttttcatttc atatgccgtc 603180
tgaaagcagg ttttaattgt tgtccgatac ggacgtttgt agaaagcatt gaacaaggca 603240
tctgtggata ttgattcatg cagatgccgt ctgaaaataa cccctatcaa tggagtatca 603300
aaccatgagc aacaacatcc cggccgaact gaaatacgtt gccagccatg aatggctgcg 603360
ccttgaagaa gacggtacga ttaccgtcgg cattacccac cacgcgcaag agctgttggg 603420
cgacatcgtg ttcgtcgagc tgcccgaagt cggcgcgaac cttgccgctg aagagcaagc 603480
cggtgtggtt gagtctgtaa aagccgcgtc cgacgtgtac gcaccgattg caggcgaagt 603540
cgttgccgtc aacgaagatt tgccaagcgc tccggaaact gccaacagcg atccttacgg 603600
tgcaggctgg ttcttcaaac tcaaaccggc aaacgttgcc gattacgaca gtctgctgac 603660
tgccgaacaa tacgcgggcg aagtggatta aaccgcccgg ctgcccgacg gcaaccgccg 603720
gacaaacgga aactgcacct tcagacggca tttttgcggt cggaggtgca gtttttttgtc 603780
cgtgttttaa ggaagcagtt aggctataat aacggtctat attcatcttt accgattttt 603840
tcatgcaact taccgctgtc ggactcaatc atcaaaccgc acctttaagc atacgggaaa 603900
agctggcgtt tgccgccgcc gccctgccta aagccgtccg caatcttgcc cgaagcaatg 603960
cggcaacgga ggcggtaatc ctttctacct gcaaccgcac cgagctttac tgcgtcggtg 604020
attcggaaga aatcatccga tggcttgccg attaccacag tttgccgatt gaagaaatcc 604080
gtccgtatct gtacgcgctg gatatgcagg agactgtgcg ccatgctttc cgcgtcgcct 604140
gcgggctgga ttcgatggtg ttgggcgagc cgcagatttt aggacagatt aaggatgccg 604200
ttagggttgc tcaagagcag gaaagtatgg gtaagaaact caatgccctg ttccaaaaaa 604260
ccttttccgt tgctaaagag gtccgtaccg atactgccgt cggcgaaaac tcggtttcca 604320
tggcttccgc ttccgtcaaa ttggcggaac agatttttcc cgacatcggc gatttgaatg 604380
tcttgtttat cggcgcaggc gaaatgattg agctggttgc cacttatttt gccgccaaaa 604440
gtccccggct gatgacggtt gccaaccgga cgctggcgcg tgcacaggag ttgtgcgaca 604500
agctcggtgt caacgccgaa ccgtgcctgc tgtccgatct gcctgccatt ctgcacgatt 604560
acgacgtagt ggtttcttca acggcaagcc agttgcccat tgtcggcaaa ggcatggtgg 604620
agcgtgcatt gaaacaaagg cagagtatgc cgttgttcat gcttgatttg gcagtgccgc 604680
gtgacattga agcggaagtc ggcgatttga atgatgccta tctttatacg gtggacgata 604740
tggtcaatat cgtccaaagc ggcaaggagg caaggcagaa ggccgccgcc gccgccgaaa 604800
cgctggtgtc cgagaaagtt gccgaatttg tcaggcagca gcagggcagg cagagtgtcc 604860
ccttgattaa ggcgttgcgg gacgagggcg agaaagcgcg caaacaggtg ttggaaaatg 604920
ccatgaaaca gcttgccaaa ggcgcaacgg cagaagaggt tttggaacgg ctgtccgtcc 604980
aactgaccaa caagctgctg cattcgccga cccaaacctt gaataaggcg gggaagaag 605040
ataaagattt ggttcatgcc gtcgcgcaga tttatcattt ggacaaataa cggtgcgccg 605100
ggaaaaatgc cgtctgaaga ggtttcagac ggcatttttt tgtgccgcct gacaacatcg 605160

```
tgaaatccca cattatatcg atgtaatcac aaagtatagt ggattaacaa aaatcaggac 605220
aaggcgacga agccgcagac agtacagata gtacggcaag gcgaggcaac gctgtactgg 605280
tttaaattta atccactata ttatcccgta tgcggattgg ttttaagatt tgtaaatttg 605340
atttgcatca aaaaatcgcc gatagatgat tcatataata tcaatattaa agagtatcgg 605400
tatatcgggg atagtcatgt cctgtttttc aatcaaacgt atgtccgcgt ttcgggcgcg 605460
gataacggcg ttttttgccg cctttgtctt tttgacggcg gcactgcccg cttatgcgga 605520
gcgtctgcct gattttctgg cgaaaataca gccttcggaa atttttccgg gtgcggaccg 605580
ttacggcaag ccggaaggta agcctatggt tgcccgcgtt tacaaaggcg atgagcagtt 605640
gggcttggtc tatatcacga ccgatgcggt caatacgcgc ggttattcga gcaaaccgat 605700
tgatacgctg atggtgttgg caaacgacgg cacgatagcc ggggcgaaac tggtcgacca 605760
tcacgaaccg attatgctga tcggtatccc gcatttgccc gcgcccgggc gggcgatacg 605820
ctcaaactgg cttccggcgt atataaaacc aaacttcaca ttgacaaacc gattacgatt 605880
gaagggcctg ccgaccgttc cgcaaccatc gaaggcgaca ggagcgggcg taccatagcc 605940
gtacacgcgc cggacgtaac gctccgcaac ctgaccgtta cccgttccgg tatgagcctg 606000
cccgcaatgg atgccggtat ttatctcgaa gaaactgccc cgcgcgccct gattgaacac 606060
aacaatattt tggataattc ggtcggcgta tatctgcatg gttctgccga tgcgatggtg 606120
cgcgagaata aaatcgtcgg cgacgcgact ttgcgcgtga acgagcgcgg caacggcgtt 606180
accgtttgga acgcacccgg tgcgcaggtc gtcggcaacg acatttccaa agggcgggac 606240
ggcatttttt ccaataccag cacgcacaac acctacaaaa acaaccgctt cagcgatttg 606300
cgtttcgccg tccactatat gtacaccaac gacagcgaaa tcagcggcaa tatttccgtg 606360
ggcaacaata tgggctatgt gctgatgttt tccgagcggc tcaaagtatt cgacaatatc 606420
gccgtcggca gccgcgatca gggcattatg ctcaactatg tcaactattc cgatattcac 606480
gacaacatta tcaacaaggc aggcaagtgc gtatttgcct ataatgccaa ctacgataaa 606540
cttttcgcca atcattttga aaactgtcaa atcggcatac actttaccgc cgccatcgaa 606600
ggcacgtcct tgcatgacaa ttcctttatc aacaacgaaa gccaggtcaa atacgtcagc 606660
acgcgctttc tcgattggag cgagggcgga cacggcaact attggagcga caacagcgcg 606720
ttcgatttga acggcgacgg cttcggagac agcgcgtacc gccccaacgg catcatcgac 606780
caaatcatct ggcgcgcgcc cgtatcgcgc cttttgatga acagtcccgc aatcagcatc 606840
gtcaaatggg cgcaggcgca gtttcccgcc gttctgcctg gcggcgtggt ggacagcaaa 606900
ccgctgatga agccttatgc cccccaaatt caaacccgtt atcaggcgat gaaggacgag 606960
ctactcaaag aagtcgaaac gcggcagtcg gaatggggca gggcggaaaa cggttctttg 607020
aactagtctg cttcagacgg catccggatt caaatgccgt ctgaaaacac aaaaggaaca 607080
accatgacca cacatcatgt cgaattgagg aaggtaacca aacggttcgg ggcgcaaaaa 607140
gccgtcaacc aagtcgattt ggttttgaag gcaggagaaa gcgtcgggct tgccggacac 607200
aacggcgcgg gcaagtccac cattatgaag ctgatactcg ggctgattac cccgaccgaa 607260
ggcgaagtga tgcttttggg cgaacgtacc ggtagcaaag cgggggcgcg gcttcgcagc 607320
caaatcggct acctgcccga aaccgttgcg ctgcaccctt cgctgatcgg catcgaaacg 607380
ctggattttt atgccaaact taaaaaacag ccgctcacgc agaaccgggg gctgcttgag 607440
cgcgtcggca tttcacaggc ggcacaccgc cgcgtcggca cttattctaa aggatgcgc 607500
caacgccttg ccttggcaca agccctgctg ggcgagccca aagtcctgct gtttgacgaa 607560
ccgacaaccg gtcttgaccc tgcatcacga caaatgtttt acgaagtcgt gcgcgaactc 607620
aacgggcgcg gcgcgaccgt attgctcagc acccacgccc ttgccgagtt ggacgggcac 607680
gccgaccgca ttatcgtgga ttaaatttaa tccactatat gcgggtatgg cgggtttgag 607740
cggacaaatc agcctgaccg tccccgtttt gctgaccgct caggttttat gggttatcat 607800
tccgcttgtt ttggcagccg gaatttttag aaagcgacaa atatgaaaaa aaccctgttg 607860
gcaattgttg ccgtttccgc cttaagtgcc tgccggcagg cggaagaggg accgccgcct 607920
ttaccccggc agattagcga ccgttcggtc ggacactatt gcagtatgaa cctgaccgaa 607980

cacaacggcc ccaaagccca gattttcttg aacggcaaac ccgatcagcc cgtttggttc 608040
tccaccatca agcagatgtt cggctatacc aagctgcccg aagagcctaa aggcatccgc 608100
gtgatttacg ttaccgatat gggcaatgtt accgattgga cgaatcccaa tgccgacacg 608160
gagtggatgg atgcgaaaaa agccttttac gtcatcgaca gcggctttat cggcggtatg 608220
ggtgcggaag acgcgctgcc gttcggcaac aaagagcagg ctgagaaatt tgcaaaggat 608280
aaaggcggta aggttgtcgg tttcgacgat atgcctgata cctatatttt caaataatat 608340
tgaaaaaacc ggccgaaact ttaaaaacga gtttcggtcg gtttttcttt tcttgttcgg 608400
tatagtgtcg gcaggaaaga accttcacat cccgccgtaa ttcggcccgc tcgcgccttc 608460
ggggcaaatc caagtgatgt tttgcgtcgg gtctttgatg tcgcaggttt tgcagtgcac 608520
gcagtttgcc gcgttgattt gcaggcgcgg attgccgttt tcttcaacaa tttcgtacac 608580
gccggccgga caatagcgcg tttcgggcga ggcgtattct ttgtagttca cgtctatcat 608640
cgtttgcgga ttgttcagca ccaaatggtc gggctggttt tcttcgtgcg cgagattggc 608700
aaggaagacg ctgctcaagc ggtcgaaggt caacacgccg tcgggtttcg gataatcaat 608760
```

cggcttacac gcggcggctt ttttaagctg ctcgttgtct ttgccgtgat gtttcaaggt 608820
ccacggggct ttgcctctga aaatcatctg atcgatgccg gtgtagattg agccgaggta 608880
aacgccccat ttgaatgacg gacggacatt gcgcgcggcg taaagctctt gatacagcca 608940
gctttgttca aaacgttgct gataatccgc cgcctctttg ccgctgtcga aaccctccac 609000
ttcttcaagg ttttccaaca aggggaacac ggcttcggcg gcgagcatgg cggatttcat 609060
cgcggtatga atgcctttga tgcgcggcat attgaggaaa cccgccgcat cgccgaccaa 609120
aatgccgcct ttgaacgaga gcttcggcaa actttgcaaa ccgccttcaa tcagcgaacg 609180
cgcgccgtaa gcaatgcggc ggccgccttc aaaggttttg cggatttcgg gatgggtttt 609240
gaaacgttgg aactcttcaa acggcgacag ataaggattt tgatagtcca aaccgaccac 609300
gaagccgacg gcgactttgt tgtcgtcgaa atggtaaaca aacgcgccgc cgtaggtttt 609360
gctgtccagc ggccagcctg cgctgtgcac caccaaaccg ggctgatgct gttcggacgg 609420
cacttcccaa acttctttaa tgcccaagcc gtaagtttgc ggctggctgt tttggtcgag 609480
ttggaaacgt tcgatgattt gtttggaaag cgaaccgcga caaccttcgg caaacagggt 609540
ttgctgcgcc caaagctcca tgccgggttg gaatgaatcg gtcggctcgc cgtctttgcc 609600
aatgcccata ttgccggttg caatgccttt gaccgaaccg tcttcgtgat acagcacttc 609660
ggcggcggca aagcccggat agatttccac gcccatattt tccgcctgct ccgccaacca 609720
gcgcacgact tcgcccaagc tgacgatgta gttgccgtga ttgtcgaaat tcggggtaat 609780
cggcaggttg aacgcttttt tctcggtcag gaacaacact ttgtcctgcg ttactgtgcg 609840
tgtcagcggt gcgccttttt ctttccagtc ggaaatcaac tcattcagcg caatcggatc 609900
gataactgcg ccagccagcg aatgcgcccc cacctccgaa cctttctcca ccacgcaaac 609960
gctgatttcg cgcccgtttt gttcggcaag ctgcttgagt ttgatggcgg cagacaaacc 610020
cgacgggcct gcgccgacaa tcacgacatc gtattgcata ctgtcgcggg tgatggattc 610080
tgtcatggcg gttcctgtgt atttattatt gaattgcaaa tccgtaatta tacaacggga 610140
acatatagtt accaaataca acaaaggtcg tctgaaaacc atattttcgg ttttcagacg 610200
acctttgtcg aaatttcaat aagcacgcca ccatttttacc tgtccgaccg caaactccgt 610260
ctgacgtttc ggactgcgtg tgaaaaacgc cttatccccg ccggcatccc tccctttcgg 610320
cacaaccgcc aaaatcttac ctgccaaatt tccctcacgg gtttgccaag catccaaaaa 610380
ctgcgccctg ctcattgaaa catgacccag cgacgggtcg gcaagcaaaa ccgtattgcc 610440
gtctataccg cgcaataccg agaaatgatc atccttgcgg tatttcagat acacgatgac 610500
ggggatttgc aactgtgcaa gctgctcgaa agacagggca tagcctttcg cttcaaaacc 610560
caaatcaggc ataatgcgcc gcatatcctc aaacgacgcg ggcatctgct ccttatccag 610620
ttttttttaac acgtcctctt ccgtcagctt ttgcccgtaa aaattgttca aaagcgtcac 610680
caccgaagcc gccccgcagg aaaaatccaa atcctgcttt acaatattga aatcgcgcct 610740
ttctttccaa ctctgcactt tgatttttcc ataagcaaca ggattatagt ggattaaatt 610800
taaaccagta cggcgttgcc tcgccttgcc gtactatctg tactgtctgc ggcttcgtcg 610860
ccttgtcctg atttttgtta atccactata ggtttccgtg cggacgtgtt cagattcccg 610920
ccttcgctgg aatgacggcg gagcgatttc tacttttccg ataaatgacc gtaacttaaa 610980
atcccgtcat ccccacgaaa gcaaaaatcc cgcctgtcgg atttcggttt ttttgggcgt 611040
ttcgggaaac ttataaatcg tcattcccgc gcaggcggga atccggtttg ctcggtttcg 611100
gtttttcggg cgtttcggga aactgatgaa tcgtcattcc cgcgcaggcg ggaatctaga 611160
acgcgggacg gcggcaatat tcaaaggttg tctgaaaatt cagaggttct agattcccac 611220
tttcgtgggg atgacgggat ataggtttcc ctacggacgt gttcagattc ccgctttcgc 611280
gggaatgacg gcggagcgat ttctactttt ccgataaatg accgtaactt aaaatcccgt 611340
catccccacg aaagcaaaaa tcctgcctgt cggatttcgg tttttttcgg gcgtttcggg 611400
aaactgatga atcgtcattc ccgcgcaggc gggaatctag aacgcgggac ggcggcaata 611460
ttcaaaggtt gtctgaaaat tcagaggttc tagattccca ctttcgtggg aatgacggga 611520
tataggtttc cctacggacg tgttcagatt cccgctttcg cgggaatgac ggcggagcga 611580
tttctgcttt tccgataaat gaccgcaacc taaaccccat ccttcccgca aaaacagaaa 611640
aacaaaaacc taaaatcccg tcatccccac gataacagtt gcgtaattgc gtagagtggg 611700
cttcagccca ccgtttttttc tttttcggtc gttgattggt gggctgaagc ccacccttgt 611760
atatcggaac tcccgtatca tagcaacaaa ccgcccggcc gccacccgcg cccacccaag 611820
gcacacaacc gttgcgtagc acagggagcg gcagggcaac ccatcgacac aaccggacag 611880
ttgccggaca acacaaccga atgtaaggca ggttgatgat gagtacccga taccattacg 611940
caggtatagt gaattaaatc taaggggctg tactagatta gccctaaatt ccacaccaat 612000
cccgcaggat tttaagctgt tgagacggtg tgccgaagtt aaatcgaaat tcgcattctt 612060
tcaagaacag cgggaaagat ttacgatcga ttccgttgta ttttcgcaag acgcgttttg 612120
cctgattcca aaagttctca atgccgttaa tgtggttctg acggtctgca cactccttgg 612180
aatggttgat gcggtaatgg ataaaaccgc tcacgtccaa cttgtcgtag ctgctcagac 612240
tatcggtata aacaatacta tccggcatga ttttcttttt gatgacaggg agtaacgttt 612300
cagacttggc attatccacc acaacggtat agacccgtcc gttgcgtttc agaatgccga 612360
aaacaaccac ttttcctgcc gcaccgcgac cacgtctgcc tttacgccgt ccgccgaaat 612420

cgctttcgtc cggctcgaca gggccctcaa aaacctcatc ggcagccaag gccaaatgat 612480
ggttgataac cgtgcggatt ttacggtaga acagtgctgc cgaattggga tggataccca 612540
aaatatcggc ggcagaacgg gcggtaactt ccagtacaaa aaacggagca gttctttctg 612600
tacttttttc tttaatttgc agtgcgttat cttcatattt cgagggtaac atatctgcta 612660
atctagtaca gccccaaaaa tataccaaaa acagcaaaac aaattgtaag gatacgtata 612720
ggctttgtaa aggtaaattg tgaaaaaagc agttttttaa acgaatgaaa cggcttcggg 612780
ctgaaatata tgctgatgcc ctgttcttcc cgtatttctc gtgtgttgtc aaagtgcagg 612840
ctgctttgaa atcggtattg ccatctatga accaccactt tgctttattt cagcgggctt 612900
gagatgtgta taagaatatt gttttgaata aatttaaaga aaatgataat cgttattgac 612960
gatttttaaa ggaaagcgta gagtgccaat tctatgaagc aatacggtaa gtaacaatga 613020
aaatatctac tgcttgggta tagagcatat ttcacaaccc gtaactattc ttgcggaaac 613080
agagaaaaaa gtttctcttc tatcttggat aaatatattt accctcagtt tagttaagta 613140
ttggaattta tacctaagta gtaaaagtta gtaaattatt tttaactaaa gagttagtat 613200
ctaccataat atattcttta actaatttct aggcttgaaa ttatgagacc atatgctact 613260
actatttatc aacttttttat tttgtttatt gggagtgttt ttactatgac ctcatgtgaa 613320
cctgtgaatg aaaagacaga tcaaaaagca gtaagtgcgc aacaggctaa agaacaaacc 613380
agtttcaaca atcccgagcc aatgacagga tttgaacata cggttacatt tgattttcag 613440
ggcaccaaaa tggttatccc ctatggctat cttgcacggt atacgcaaga caatgccaca 613500
aaatggcttt ccgacacgcc agggcaggat gcttactcca ttaatttgat agagattagc 613560
gtctattaca aaaaaaccga ccaaggctgg gttcttgagc catacaacca gcaaaacaaa 613620
gcgcacttta tccaatttct acgcgacggt ttggatagcg tggacgatat tgttatccga 613680
aaagatgcgt gtagtttaag cacgactatg ggagaaagat tgcttactta cggggttaaa 613740
aaaatgccat ctgcctatcc tgaatacgag gcttatgaag ataaaagaca tattcctgaa 613800
aatccatatt ttcatgaatt ttactatatt aaaaaaggag aaaatccggc gattattact 613860
cattggaata atcgagtaaa ccaggctgaa gaagataatt atagcactag cgtaggttcc 613920
tgtattaacg gtttcacggt acagtattac ccgtttattc gggaaaagca gcagctcaca 613980
cagcaggagt tggtaggtta tcaccaacaa gtagagcaat tggtacagag ttttgtaaac 614040
aattcaagta aaaaataatt taaaggatct tattatgaat gagggtgaag ttgttttaac 614100
accagaacaa atccaaacct tgcgtggtta tgcttcccgt ggcgatacct atggcggttg 614160
gcgttatttg gctaatttgg gtgaccgtta tgcggatgat gctgctgcaa ttgtcggtaa 614220
ggatgcaaac ttaaatggtt tgaatttatg gatgaaaaaa ggtgtggaaa acctatggga 614280
tgatacggtc ggtaaaaaga cccgtttaga gaaatttgat cgggttgcat tgcaacattt 614340
cagccaatat gtagatctaa ttaatgaaaa taatggtaga ttacctaaca ctagtgaaat 614400
tgagagaagt tactataaag ccgttaccga aaatggtgtt tcttctagtg cagctattga 614460
tttagttatt aatcgctcac ttccggatat ggcagatggt tattgggcat taggtttggg 614520
gatagaagcc gaacgtatcc acaatgagca agcagtaaat aatccgaacg gtagcgaaag 614580
ggataataga aagcagttaa tatctgcttt agataaagga tttgatggat cttttaaaga 614640
gaagcatttt actttttttac aatctgtgat aatggatgta acaaagttag gtgttgaata 614700
tacaatagat ggttggcaaa aaattggagg ttggggtaat gggataatca atgatttata 614760
taaaagtgtt gtaaaaagag agtggactgg aatatttgag atcgttaata ataacatcaa 614820
gcaatttaga gatctgttcc caaatccgga aggctggatc gatgatggtc accaatgttt 614880
cgctccttgg gttaaagaaa ctaaaaaacg caatggcaaa tatcatgtct acgacccccct 614940
tgccctagat ttggacggag acggcataga aactgtcgct gccaaaggct tttcaggcag 615000
cttatttgat cacaccaaca acggtatccg caccgccacc ggttgggttt ctgccgatga 615060
cggtctgctt gtgcgcgatt tgaacggcaa cggcatcatc gacaacggtg cggaactctt 615120
cggcgacaat accaaactgg cagacggttc ttttgccaaa cacggctacg cggctttggc 615180
cgaattggat tcaaacggcg acaacatcat caacgcggca gacgccgcat tccaatccct 615240
gcgtgtatgg caggatctca accaggacgg catttcccaa gctaatgaat tgcgtaccct 615300
tgaagaattg ggtatccaat ctttggatct cgcctataaa gatgtaaata aaaatctcgg 615360
taacggtaac actttggctc agcaaggcag ctataccaaa acagacggta caaccgcaaa 615420
aatgggggat ttacttttag cagccgacaa tctgcacagc cgcttcaaag acaaagtgga 615480
actcactgcc gaacaggcaa aagccgccaa tcttgcgggc attggccgtc tgcgcgattt 615540
gcgcgaagct gccgcattgt ccggcgattt ggccaatatg ctgaaagctt attctgccgc 615600
cgaaactaaa gaagcacagt tggcattgtt agataatttg attcacaaat gggcggaaac 615660
cgattcgaac tggggcaaaa aatcgccaat gcgactttca accgattgga cgcaaacggc 615720
taatgaaggt attgcactga caccatccca agtagcacaa ctaaaaaaga acgctttagt 615780
ttccctttct gataaagcta aagcagctat tgacgccgcc cgcgaccgca ttgccgtgct 615840
tgatgcctac acggggcagg attccaacac actctattac atgagcgagg aagatgcgct 615900
taatatcgtc aaagtaacca acgatacata cgaccatctc gccaaaaaca tctaccaaaa 615960
cctgttgttc caaacccgtt tgcagccata tttgaatcaa atcagtttca aaatggaaaa 616020
tgatacgttc actttggatt ttagtggtct tgttcaagca tttaaccatg tcaaagaaac 616080

taatccgcaa aaagcttttg tggatttggc cgagatgctt gcatatggcg aacttcgttc 616140
ttggtatgaa ggccgaagac taatgaccga ttatgtggag gaggcaaaaa aagcaggtaa 616200
atttgaagat taccagaaag tgttgggtca ggagaccgtt gcattattag ctaaaacatc 616260
gggtacgcaa gcagatgata tcctgcaaaa tgtaggcttt ggtcataata aaaatgtttc 616320
tttatatggt aatgacggca acgacactct aatcggcggc gccggtaatg actatttgga 616380
gggcggcagc ggttcggata cttatgtctt cggcgaaggc ttcggtcagg atacggtcta 616440
taattacgac tacgctaccg gacgcaaaga catcatccgc tttaccgacg gtattacagc 616500
cgatatgctg acttttaccc gagagggcaa ccatcttctt atcaaggcaa aagacggcag 616560
tggacaagtg actgttcagt cctatttcca gaacgatggc tcaggtgctt accgtatcga 616620
tgagattcat ttcgataacg gcaaagtact ggatgttgcc actgtcaaag aactggtaca 616680
gcaatccacc gacggttcgg acagattgta tgcctaccaa tccggaaata ccttaaatgg 616740
cggattgggc gatgactatc tgtacggtgc cgacggggat gacctgctga atggtgatgc 616800
aggcaacgac agtatctaca gtggcaatgg caatgatacg ctcgatggag gagaaggcaa 616860
cgacgccctg tacggctata atggtaacga tgcactgaat ggtggcgaag gcaatgatca 616920
tttgaacggc gaagacggta acgacactct aatcggcggt gcaggcaatg attacttgga 616980
gggcggcagc ggttcggata cttatgtctt cggcaaaggc ttcggtcagg atgcggtcta 617040
taattacgac tacgctaccg gacgcaaaga catcatccgc tttaccgacg gtattacagc 617100
cgatatgctg acttttaccc gagagggcaa ccatcttctt atcaaggcaa aagacggcag 617160
tggacaagtg actgttcagt cctatttcca gaacgatggc tcaggtgctt accgtatcga 617220
tgagattcat ttcgataacg gcaaagtact ggatgttgcc actgtcaaag aactggtaca 617280
gcaatccacc gacggttcgg acagattgta tgcctaccaa tccggaaata ccttaaatgg 617340
cggattgggc gatgactatc tgtacggtgc cgacggggat gacctgctga atggtgatgc 617400
aggcaacgac agtatctaca gtggcaatgg caatgatacg ctcgatggag gagaaggcaa 617460
cgacgccctg tacggctata atggtaacga tgcactgaat ggtggcgaag gcaatgatca 617520
tttgaacggc gaagacggta acgacactct gatcggcggt gcaggcaatg attacttgga 617580
gggcggcagc ggttcggata cttatgtctt cggcgaaggc ttcggtcagg atacggtcta 617640
taattaccat gtggataaaa actctgacac tatgcacttt aaaggattta aagcagcaga 617700
tgttcatttt atccgttccg gaagtgattt ggtgcttagc gcttctgaac aagacaacgt 617760
acgtatttcc ggatttttct atggtgaaaa ccatcgtgta gatacatttg tctttgatga 617820
tgcagctatc agtaatccag attttgccaa gtatattaat gctggcaata atttggtaca 617880
gtctatgtct gtgttcggtt ctaatactgc tgcgacagga ggaaatgtgg atgccaatat 617940
acaatccgta cagcagccgt tattggtaac gccatctgca taaggagcct aattacattc 618000
atggcttaaa ctgaaaaaca gcaatcaagt ttattttgat tgctgttttt cttaatattg 618060
ggataagggt cgtattttaa ttaaccttaa tcggtgcact tctagcaata tagtggattc 618120
acaaaaacca gtacagcgtt gcctcgcctt accgtactat ctgtactgtc tgcggcttcg 618180
tcgccttgtc ctgattttg ttaatccact ataattttca gacggccttt tgccttttca 618240
aattcaaacc aatcaaacgg ttttattgct tcatcgcgtt ggtcaaggct ttgatgttgt 618300
ggcggtacat tccgatgtag gtgtctgcgg gcgcgttgcc gagtgcgtcg gaatacagtt 618360
tgccgctgac gttgacaccg gtttctttgg cgatacggtc aaccatacgg gtgtccttga 618420
tgttttcggt aaagacggct ttgatgcctt cgcgtttgat ttgtcggatg atggcggcga 618480
cttgtttggc cgaaggctcg gcttcgctgc tcacgccttg cggggcgatg aattcgatat 618540
ggtaacgttt gcccatatag gaaaaggcat cgtgcccggt caggactttg cgtttggcag 618600
cagggacggc attaaatgcg gcttgtgcgt cgctgtgcag ttttttgagc tgcatttggt 618660
agttgcccaa gcgttgttga taataaactt tgccttcggg atcggccttt atcagggctt 618720
tggcaacgtt ttgggcatag gcggacataa ggacggggtc gttccagacg tgcgggtcat 618780
attcgccgtg gtcatggtgg tgtccttcgt ggtcatgatc gtggtcgtga tggtgtccgc 618840
cttcttcttc ggctttgagg ggttggatgc ctttggtcgc ttcggtatag gatactttgc 618900
tttgtttgac ggcgcgttgc acatcggcag cttcaagtcc taagccgttg agcaggacga 618960
gttttgcact gcggattttt ttaatgtcgc cactggtcat atgataggcg tgcgtatctt 619020
ggttggctcc gaccaaactt tgtatggata cgcgctctcc gccgatttgt ttggctacgt 619080
cgcctaaaat gctgaagctg gttacaaccg gcaggggggc ggcagttgcg gaggcggtca 619140
gcaatgcggc aataagggtg agtttgaggt gtttcataac tgttctcctg tgatataacg 619200
taacatctgt tatggtaaaa caagccgcct gtttgttcaa gcggcttgcg gggtcaggtg 619260
gtgtggtggc ggtggttttt gagccatttg gtcagaatgc cgccttcttt gccgagtatg 619320
acggaaaaga gataaaggac gctgcaacag aggatgatgg cgggaccgga aggaatttcg 619380
atgtggtagg aaatgagcag tccgctcaag ccgcacagca gggctgtcag aacggatagg 619440
aggatgagtg cgcccatatg cttcgcccac aggcgggcgg taatggctgg cagcatcatg 619500
agtccgacgg acatgagtgt gccgagggct tgaaagccgg atacgaggtt catgacgacc 619560
aggacgagaa agaggacgtg ccaaagcccg cctttgccgc cgacggattt gagaaacagg 619620
gggtcgatgc tttcgagtac gagcgggcgg tagatgacgg caagggtaat gagcgtgagg 619680
ctggagacgg cggcgatgag ctgcagggca ggaatatcga cggcaagtac agagccgaaa 619740

aggaggtgga gcaaatcgac gctgctcccg tttttgctga cgaggactac gccgatggcg 619800
aggctgctga gataaaaggc ggcaaagttg gcatcttctt tcagggtggt gaagcggctg 619860
acgagtccgg caagcagtgc catcagcatg cctgcggcta cgccgcccaa acccatggcg 619920
ggcaggctca agccggcaaa catgtagccg acggcggcac cgggcaggac ggcgtggctc 619980
aatgcgtcgc ctatcaggct catacggcgc atgacgagga atacgccgac gggtgcggca 620040
ctgagggaca ggcagaagac ggatgcgagg gcgtagcgca taaagtcgaa ttctgcaaag 620100
ggggcaagga gcaggtcgta gagattcatg gtttttcggt ttcagacggc atttatgagg 620160
cgcaccagtc ggggctttcc tgttgctgca ttttggcgtt ggcttgggcg aggtagggtt 620220
ctgtcagaat ggtctcggtt gcgcctgccg caattttttc gcgggcgagc agcagggtat 620280
tgggaaagta ggcacggact tgttcgtaat cgtgcagtac ggcgatgatg gcgtgtccgc 620340
cgcaatggca tttctgcaat acgtcgagaa gctcgtaggt tgtccgtgca tcaacggcat 620400
tgaagggttc gtcgagcagc aggaatttgg cattttgaac cagcattcgg gcaaaaagga 620460
cacgctgaaa ttgtccgttt gagagatagg caatctgacg gtcggcaaac cgttgcattc 620520
cgacgcgctc caaggcttcg tgaacgcgtt gtttttgagc ggtatttatc cctttgaaaa 620580
agccgatttc ataccatagc cccattgccg ccaagtcgaa aacggtcata ggctgggagc 620640
ggtcgatatc ggactgctgg ggaaggtagg cgatgttctg acgggtcaat ccgtccagcc 620700
ggatgctgcc tgtatcgata ggctgcaatc ccatcaagga tttgagaaag gtggatttcc 620760
ctgcgccgtt gggaccgaaa accgcccaca tactatgttc ttcaaaagta atgtccacat 620820
ggtgcacggc aggtcggcgg cggtagctga cggtcaggtt ttcgacaatg atgctcatgc 620880
ggatactgcc caaaagtaaa cgccccataa aagggatacg gcaatcaggg caaggatgag 620940
gcggaaggtc aatcctgata gtaaaaggga aggtgtcatg atgatttgcg gttttgaaag 621000
ggaaggcggt aaagcgttta tcgttatatg gctgatatga tactgtataa cgtttggtct 621060
gtaaattatg cttgaatagg cgggagtgat tgttaatcaa ggtggatgag gggcaggcat 621120
atcgttgact tgccggcatc gcagcaataa gaaatgccgt ctgaaggttc agacggcatt 621180
gggggaaaac ggtttgaatc aacctttgcg tgcaggcagt ttttctttga tgcgtgcagc 621240
tttaccggtc aggccgcgca ggtagtacag tttggcacgg cgtacgtcgc cacggcgttt 621300
gacttcgatt ttttcgacgg tcggagagta cagttggaaa gtacgttcaa caccttcgcc 621360
gctggagatt ttgcggacga tgaagttgct gttcagacca cggttgcgac gggcaataac 621420
cacgccttcg taggcttgca gacggctgcg ggtaccttcc acgacgcgta cggatacgac 621480
tacggtgtcg cccggtgcga attcggggat ttctttattc aggcgggcaa tttcttcttg 621540
ctcgagctgt tgaatcaggt tcattgtttt tttcctaaat tatgattgga tttcccgttg 621600
ctcttgccgg atggtttcta agaggcggga ttcctttggg attaaaacgc gcttttccaa 621660
aagatcgggt ctgcgctcca aggtgcggcg cagcgattgt tccaaccgcc attccgctat 621720
caagccatga ttgccggaac gcaatacttc cggaacagcc ataccttgaa attctaaggg 621780
tttggtgtag tgggggcagt ccaaaatgcc gcttgagaac gaatcctgtt cggcagactg 621840
catatcgccc aatacgccgg gtacgagcct caataccgca tccatcagca tcatggcggg 621900
aagctctccg ccggaaacaa cgaagtctcc gatgctgatt tcttcatcga cgctgctttg 621960
cagaagcctt tcgtctatgc cctcataccg tccgcacagc agaatcagat gcggaagttc 622020
tgccagttct accgcttttt ggtgtgtcaa gcggtttccc ttgggggctg aggtagatga 622080
cttttgcagc ttgggaggat tgtgttttgg cgtgttctat tgccgcatga agcggcggag 622140
ccatcataat cattcccggg ccgccgccga acgggcggtc gtcgatgtag cccaatctgt 622200
tgtcggcaaa ctttcgggga ttgactgctt caaactgcca gattccctgt ctgttcgcgc 622260
gtcccgttac gccgtagcgg gtaatgctgt cgaacatttc ggggaaaatg gtaactgcct 622320
ggataagcat cagtagtcca aaccccagtc ggcagtaatg gtcttgctgc cggtatcgac 622380
ggtttcgata tattgggaaa cgaacggaat cagaatctgc ccgtgttctc cgtcaatcat 622440
caatacgtcg tttgcgccgg tttccatcag gttgcttacc ttgcctaaaa cggtatggtc 622500
tttgttgaca acggtcatgc cgaccaagtc tgtccagtag tattcgtctt cttctgtcgg 622560
ggcgaatgct tcacggggta tttcgatggt gtaaccgcgc aatgagaatg ccaagtcgcg 622620
gtcgtttatg ccttcgaatt tgacttggag ttcgccgttg acgacttttc cggcttcaag 622680
ggtaacgctg atggttttgc cgtccttgac caaatgccac tcggggtagt ccaaaaggct 622740
gtcggaatat tcggtgttgg cggcaatttt caaccagcct tttatgccga atacgccttt 622800
gatgtagccc atggctaccc ggttttgagt gtctgtcatg gcggcaaatg cggattaggc 622860
ggctttttgt tctttaatca gttttgcaac ggagtcgctg acttgcgcgc cttgtgcaat 622920
ccagtggttc aggcggtctg cattgaggcg gacgcgctct tgtttttcgt tggctacggg 622980
gttgtagaag cctacgcgtt cgatgaagcg gccgtcgcgg cggctgcgtg agtcagtaac 623040
gatgacgttg tagaagggc ggtgtttcga gccgccgcgt gccaaacgga taactaccat 623100
tttgagtcct tttgagaaaa tcggatatat ggaaactgcc gattttaggt tattttgtgg 623160
tcggtgcgca agtttttatt tgttttttct gttgttttgt ctgccgcaag gttcagatat 623220
gcgcggtaca ggttttttc ggtgtccgat tccttgaggg taaatcctga tttttcagca 623280
agtttgatca tggggtatt ggttttgaga atgtcggcac tcatagtccg gtagccttgc 623340
tgtgcggcgg tttggatgat gagttccatc atttctgtg ccagtccgct gccgcgcata 623400

tgttccgcca gtgtgatgcc gaattcgcat tcgttgcgat tcaggcggct gtggcggacg 623460
acggcgacga tgttgctgtc ggcatccttt gccgtccatg cggcttcaca gtggtaatcg 623520
gggttgcaca ggcgtgccaa cgtggctgcg ggcagttcgt tggtgtgggt catgaagcgt 623580
gtgtaccgtg cttcgggacc gaggctgcgg acgaactgct gtttggcttc tgcgtcttcg 623640
ggcaaaatgg gggtaatggt aacggtcgtg ttgtttctta gggacagtgt tttggggtat 623700
gctgcgggat agggggcaag tacgttgggt acggctgctc cggtttcggt tttgctgccg 623760
agcagttctg cggcggcttc gcttgtgtgg cggagaaatt cggcggctgt cgggtttttg 623820
tgtttcaggt atgcggcggc actctgcatt tttgcggcgg catgttcgag ggtttgggcg 623880
gctttgcctg tgttcttgcg tttgggcgtg tcgtgtgttt cgggtgtctt taagaggaaa 623940
tcgctgctgt attgtccgcc gttgaggttg agggtgatgc cgagaatgtg ttggcggtat 624000
tcgggaatga cggtcagtgt gtgcaggaac tggtcgaggg tttgtgtgcc gtcgagttcg 624060
gcaaagcggg caaggtggcg gctgtcgagc gtggtaaacg gcgggagtac ggcagtggtt 624120
tgtccgttgc agcgtgcggt caggatgtcg ccatagaggg ggtggctgtc gaattggaat 624180
tgtacggcgt tatgggtggt gtgccggtag ggggggaggt gcagggcttc ggcgagcagg 624240
gaggggtttg ccgctgcaag ggcttttttg atgttttggg gttgcggtgt tttcagacgg 624300
catggctgcg gcggtgcaat gtcgagctgt gcctgtttca gggcggcggc ggtgttgcgg 624360
taggaaaggg tgcggattgc ctgagtgggg gtgtcgaaat gggttatgcc gtctgaaaag 624420
gggctgctga cgagcagggg tttggcggtc tgttcggaca ggcggataag ggcgcgtgct 624480
gttttttttgt aatcctcgtg tccggaggga ctgaggatgg ttaggacggc ttgggtgtcg 624540
gggtgggcaa gctgacgtga ggcgatgtcg tggcagattg agggtgtggg tgtgccggtc 624600
aggtgtccgt tgcggatgtg gtggggaagg ttgggaaagt ggagggtgag gtttttttggc 624660
gcgtgcgcgt gcagccattc ggcaggcgtg tcggacagga tgtcgagtcg ggacaggggt 624720
ggaaggtcgg acagttgggc gcgcagtgcg gcttcgaggt cgtcggcgtt gaaactgacg 624780
aggaagttgc agtgtcgggc gaggcagtgc agtacggcac ggtcggtttc tgtcgtgcgg 624840
caggtgatgt ggagaatcag cggcgtatgg cgggtaaatt ggcggattgc gctgaacagt 624900
ttgcgctgat cctcttcagg gttgtggtgt aggacggcgg ttttggtgtg caggctgtgt 624960
ccgaagcggt tgagccaatc ggcggatgtg atggggctga tgccgggatg caggctgatg 625020
tggcgggatg tgccttgacg gagtttgttc aggatgttgt cgatttggcg gctgacggcg 625080
gcattgccgg tcagtatggc ggtatggcct gcggcgtatc cgtcttgggt actgatgttg 625140
agtccgagtg agggcagttg gatgcctgcg gtggtgcagg cggtgatgtt gagtccgttg 625200
ccgtggtgtt tgcggatggc agtttcggcg gtgtgcagtt ctgcggcaga caggttgtcc 625260
cagtcctgta tgaggatgat gtgtcggagc tgctttttgc ggcaggtttt gaagagggtg 625320
tcgtaactgt cgggtagggt aacggcaata atcaggtctg cattgccggg gattttgttg 625380
aggctggtgt aggcgggcag tccggctatg gtgtggtggc gcgggtttac gggggtgatt 625440
tttccttgaa agggcgtact cagcaggttg ctgagtacac gttcgcccag gctgtacggt 625500
tgttcgctcg cgcctatcag gatgatgtgg ttgggcatga agaagtagcc cggatcggtt 625560
tgtgccgaca tgatatattc ctttgcggac ggtatgtgcg tgatttttgg agagacaccc 625620
gctgtgtgtt tgttttgggg taactgtttg tgcaatgccg tctgaagccg gttcagacgg 625680
tattatggtc agttcgcact tttttctgtt ttggaaccgg tttttttctt gggcaggata 625740
aagcgcatcc gcagaccgtt cggtttgatg ttttcggcga tgattttgcc gcagtgctgt 625800
tcaataatat gttgggtcaa tgcaagcccc agtcctgttc cgggtttgtt ggcactggag 625860
tctgcacggt agaaagcggt gaagatgtgc gggagctgca tttcgtccac gccggggccg 625920
ttgtcggtaa cgtcgattat ccagtgtttg tggtcttgtc cgatgttgat caggatggtg 625980
ctgccttcgg gactgtagtt gacggcgttg cggatgacgt tgtcgaaggc gcggtacagg 626040
tagctttcgt tggcaaggat ggttgtgttt tcggggattt ttccgtcggc agacagggta 626100
accgtttgtc cgtttttctg ggcaatgctt tgattgtctt ctaccaggtt gcccaggaag 626160
ggcaggagtt tcaggctttc tttttccaaa gccatattgg aagtttcgag acgggacagg 626220
gttaacagtt ccccggccag cgtatccatg cgggtcagtt cgccttccag ccgtttgaga 626280
tattgctcct gtttttgggg ctgcgcctga atcagtccga caattgcctg catgcgcgca 626340
aggggagaac gcatttcatg ggagacgtga tggagcaggt ggcgttcttt ggcaacgagt 626400
ttttcgagtt tttccaccat tttgtcgaat tggatggcaa gatgggacaa ttcgtcgtcg 626460
cggtcgtcga cctgttggga gatacgggtt tcaagttctc cgtttgccac cctgtccatg 626520
ccgttgccta agattctgat gggtttggca atgttgccgg cgaggatata tgccatcagc 626580
agtccgacga tgatgatgaa ggacaatatg atgagttcgt gccaaatcgg ggcgagcggc 626640
aggccgggga tcaacagggg gctgggcagg cggcgggctt ggagtttgtc ccagtctttg 626700
gtgaagaaca ggtattcttc gccgaagcgg tcgtattcga tatggacgag gttggaatgc 626760
gggtgtccgg cggcgaaaag ccgggcgcgt tcgatggtat agctgtcgat ataccggttc 626820
aggatatctt ttttctcgtc gccctgtata acgtacacgc ccgatgagac ggggctgtct 626880
ttccattccg tcaggatttc gcgcgcaccc gcgtccccgc gtgcccggaa tgcggaaatg 626940
atgctgccca tcaaagtggt ttcgatggtg cggcgttggt tgaactggtt ttcggcaagg 627000
gtgttctgca ccagccagaa agaaaaactc gccacaaaga ttgcacagac gataaccgcg 627060

caaaatgtgg cgaaaatgcg ttggaacagt ttcatttatc tgtttatttc agtttttgac 627120
aaacaggtag cccaagccgc gtacggtttg aatcagagag gcatcgccca acttgtggcg 627180
gatgctggag atgtgtacgt cgatactgcg gtcgaatttt gccagcttgc ggtcgagtgc 627240
ttcgacggac agggtttctt tgctgactac ctgtccggca tggcgcatca ggacttcgag 627300
caggttgaat tcggtgctgg tcagttcgag cggcatgtct ttgacggatg cctggcgttt 627360
ggcggggtac aggacgacat cgctgacgga gatgctgttg ggtgcgttgt tctgttcgcc 627420
gctgtgttgt gcgcggcgca ggatggcatt gatgcgtgcc aagagttcgc gtggtgtgca 627480
gggtttgggg acatagtcgt ccgcgcccat ttccaagccg atgattcggt cgatgtcgtc 627540
gcctttggcg gtcagcatga tgatggggac ggtgcttcgg gcgcgtacgt ttttcaagac 627600
atccaagccg ttcattttgg gcatcatgga atccaatacg actacatcgt actgcccgct 627660
caggatttcc tgtacgcctg cttccccgtc gggaacgctg cggacgttca gaccttcggc 627720
gctcaggtat tcggtcagca gttcggttag cagggcatcg tcatctacga gtaatacgcg 627780
gctcatggtg tttccttttc gtaagggtat gccccgaccc tgtttcgggc ggggcgtgaa 627840
aagattgttt gacggtttat cttaacacgg ctgcaatgtt ttttgatagc gtatttccct 627900
accggtttgc tgttttttgc aatgtcttgc atggagcttt acatttcggg cggtatccgc 627960
atccgccggc gcgggtcatt tgcagggttt tgcttccgga tgaccgggcg cggcggcgaa 628020
ggctttgcag tctttgagca gttcgggtag cagcggcgcc catacgggca gtttgcggat 628080
ttcgtcggcg tatcggggca tcaggtaggg gtaataggac tgtgtcgccc gcatccattg 628140
ttttgcttct gcaactttgc cttgccgcat caggtagagg gcgatgcggt aggtggcgga 628200
gtgggggcgg tattttagtg atttgagggt tgcttcttcc gcccaagtct gggtttcggg 628260
gtattccggc agggcgaagt ttacgaggga gaagtcggca taaaaggaca gcatcggact 628320
gtttgcggaa atatagcgca actcgttgat tttccggttg agggttttgg cactgtcgtc 628380
agtggcgggg gaaaaggcgt taaccagccg ggtgtatgtc cagtccaagt gcagcaatcc 628440
tgcgaatatg gcggcggagg cggtcagtat gccgagattg gcggcttttt tgaaggcgat 628500
gccgtctgaa gcctctgcgg gggacaggaa gagcatcagt ccgaaaggga tgaggaaata 628560
gacataccac aaaggatatt cgagcatact gtggcacata ctgacggcaa gcgtgcagat 628620
taggaaaagc gatgcggggg tcaggggcg tttaagcagc ccggcaatgc ccgtcagcag 628680
ggttgcggca accagaagcg tgccgctgat tcccatctct gcaaggagtt ggaggacgat 628740
gttgtgggaa tgggtgaaca agttgctgag gaggttgtcg tatatgttgt gctgttcggc 628800
attgatgagg aaggtttgtt gggcaaaact gttccagccg tgcccgaata tcggggcgga 628860
ctggaaggcg gcaagggctt tattccattc gatttggcgc ggcaagtctg tgaaaccgcc 628920
gttggcgacg cgttcgacgg cagtttcgta gcggatgcca gtaaaggttt ccagaatggt 628980
gttcatggaa aattggaaca gcgcggtaag gaatacggct gcggctatgc cgagcatcgt 629040
ccgcctgttg gatttgtccg aacggaaata ccagaaggga aggatgaggg cgatggcggc 629100
tatgtaggtc aagatggtgc gcgagttgac caaacctaaa acggcggtct gcataatcag 629160
gcagattacg ccgagggcgg cggggatttt tcgttgtccg ttgaggtagg cggcggcgag 629220
tatgccccac atgaggtagt gtccgaggtt gttgcgctgc ccgatgtgtc cgattacgcc 629280
ttgcccgctg taaacgatga tgttttgaaa cagaggggtg tcttcccagc cggcaaactg 629340
gatgacgacg atgcaggatt gaagcaggga gccgataagc agcgaccagg caaacagggt 629400
cacgatgcgt tcttgtccga agtgtgcgac caagctccgg caggcccacg cgctgacggc 629460
gagcaagatg aaaatccaag agacgatgtc gttcataccg gggtaaatca ggttcatcag 629520
gcgtgcctga agataccaaa acgccgccat tgcaaacaga aggaagctga tggcggggat 629580
tttgacatca aacagttttt ttcctgccgt gaggaacaac aggacaatca ggccggctgc 629640
ggcggcggca tcgtggtaaa agtcgggcga cggtttcagt ttgagcgcga aggtaaaggg 629700
gacgatgcct atccaaagga agcagggcag gatgtaaatc ggcagtttgg cggcggggtg 629760
cgcgccggat acggtcgttt cagcgggcat tgtttgtttc cttgtattgt ttgacgaacg 629820
acaggcagga tatgaagaag atgatgctga atactgcgga aagcgcggcg caaatctgtt 629880
ctgcgggata ggcgtcgaac aggcgcatga cggcttcggc aaagtaaatc agaaccagca 629940
tggagctgta ttggtaagta tagattttct ttttcaagat gcctgaaagc ggcagacaga 630000
gggggagggc tttgagcgcg agccacgagc cgcccgggcg caacggtgca atccacagtt 630060
cccaggaaag ggacaggatt atcagtgcga tcaggctgaa agaggcaagg aggtaagcgg 630120
tttgtctgtt cacggcggtc tttacggttt aagggcggac aagggggagc ggtatcccaa 630180
atcctgcaac atcgaaacgg tttcataaac gggcagtccc ataatgccgc tgaagctgcc 630240
ttcgatagat tggataaaga tgccgcctat gccttgtacg gcgtaggcac cggctttgtc 630300
catcggctcg ccgctttgca cataggcgga aatttcttcc gaactcaggg gcttgaaaac 630360
gacgcggttg gtttggacgc ggcttgacgt tttgccgcga taatgaatgc agacagcagt 630420
caggacggta tgttgtttgc cggacaatcg gtttaaaaat tcgattgctt cggcttggga 630480
gcggggtttg cccaatatga tgccgtctga aacgacgcag gtgtcggcgg taatcagggg 630540
gaaatcgggc attgtgccgt tggtttcgca aaagagggtc agggcggttc ggttttttc 630600
ttctgccatc ctttgaacgt aagcgaaagg tgtttcgccg gctttaacgg attcgtcgat 630660
gccggcaggc agttggatga cgcggtagcc caactgtgtc aggatttcca ttcggcgcgg 630720

gctgtttgaa cctaaataga gggtattcaa aggtattcct taatctgttg cggtatgagg 630780
cggaggttcg gacggcatag tgtcaggttg ttgcaggcgg ccgtatgtcg ccatcctgtt 630840
ctgaacgtgg cgtgaaaaag cgtccgaacc aaatacctgc ttcgtataag agaatcagcg 630900
gaatggcaag cagggtttgt gaaatcacat cgggcggcgt gatgatggcg gcaatgacaa 630960
acgcgccgac aatcacatag gggcgggcgc gtttgagctg tccggttgtt accacaccaa 631020
ttttggttaa caggataacg acaatgggga cttcaaacgt tgtgccgaac gcaacaaaca 631080
tccccaagat gaaggagagg tatttgtcga tgtctgtcgc catattgaca ccgacagggg 631140
taacgctggc aaggaatttg aaaatgacgg ggaaaaccaa aaagtaggca aatgccatgc 631200
cgatgaaaaa caggctgacg ctggagagga cgagcggcgt aatcaggcgt ttttcgtttt 631260
ggtagagtgc gggcgcgaca aatgcccaga tttggtagag cgtatgcggc agcgaaatta 631320
aaaatgccgc catcagggta actttgaccg gcacgaaaaa tggtgcgatg acatcggtgg 631380
caatcatgct ggtgtctttg ggcaggtttg ccatcagcgg gtcggcgata aaagtataga 631440
gttgttgggc aaacggcatt aggccgaaaa agcagactaa gatgccgaca accgtccaca 631500
tcaggcggcg gcgcagctcg atgagatgct cgacaagcgg ttggacgggt tgttcgtttt 631560
gtgtttcgga caccggattg ctctctttat gatttacgga cgcgcaattt aggtttggcg 631620
cggtgtttcg gacgaaaatc gcgtttgcgg cttattgcct gtttgcgcag ggaagtggtg 631680
tgcggaacag gcgtttcaac agcagtatcg atatagctga cttcgacggt ctgtacgacg 631740
ggtgcggcgg cagaagcagt caggtattcc cgccatgcgc ggtcttggtc ggtttccgcg 631800
ggttcggctg tactgccggt ttgcccgctg tccccaaggg tttcggcgga agcgtaggaa 631860
cgttcggacg gcataacgtc ggaaatgccg tctgataggg tgtttgccgc atcgggaagc 631920
ggattgccgt tttcatcgac accgaaatcg gcaggtgtcc gctgttcggg cagtttttcc 631980
caaggcttca gaccgtcgga aatgtcgtgc agattgcctt ccatatccgt accggtttct 632040
ttgaggctgt ctcgaacctg agcggcggca gcttcaaatt cctgctttgc cttcctcagt 632100
tcttccagtt cgatttgagt gtcaaattcc tgtttgacgc tgccgacaaa gcgttgcagc 632160
ctgccgatga gccgtccggc ggtgcgggcg gcctcgggca ggcgttcggg gccgaggaca 632220
atcagggcga taatgccgac aaaaaccagc tcgcccaaac cgaaatcaaa cataaattac 632280
gctttgtctt cgtctttttt gtgttcgatt acatcgtctt tttgggcttc tttgccgtct 632340
gtaccttcgt tcagcccctg tttgaagtca tgaaccgcac cgccgaggtc tttgccgacg 632400
ttgcgcagtt ttttggtgcc gaatatcaaa acgacgataa tcagtacgat aatccagtgc 632460
gtcagagaaa aactgcccat gatgtatcct taagtaagta ttaggggttg attgtgaaat 632520
aacggtttat acgggtgtac ccatgatgtg tatatgcagg tggaagacct cttgtccgcc 632580
gccttttccg gtattgatca gggttttgaa gccgtctgcc agtcctgccg ctttggcgat 632640
ttcgggaact ttcaacatca ttttgcccag cagcatctga tgttcgggcg cggcgtgtgc 632700
caacgaatcg aaatggactt tgggaatcag cagcagatga accggagcag cggggttgat 632760
gtctttgaaa caaaccattt cgccgtcttc atagacggtt tgcgccggaa tgtctttggc 632820
ggcgattttg cagaaaatac agttgtccat aacggctccg atgccgtctg aaaagcggtc 632880
agacggattg aatgtgggaa agtgcggatt ttaatataaa ttcaagattc tgtgcgagcg 632940
gctttttcga ccagccccga cagcccctga cggcgcgcaa gttcgtccaa tacgtcttcc 633000
gccttcaggt cgtggtgtgt cagaagaatc atggtgtgaa accataagtc ggcaacttcg 633060
taaaccaggt gggacgggtt tttgtctttg gatgccatca acacttcgcc cgcctcttca 633120
atcacttttt ttaggatttt gtcttcgccc ttatgcaaga gctgtgcgac gtaagattcg 633180
gacggattgg cagattttcg ctgggtgatg gtttgttgga tggcggatag tacggaatct 633240
cccatgattt tccttctgtt tgtttctgtt tgttcggaat gataggctaa acggctgctc 633300
tcgggcaata cgcctgttgc gcttcgttgg aaaatgccgt ctgagcgttt cagacggcat 633360
ttgtgctgtt gcaaatgtaa tttgcttaca ggtttggact cacaataatt ttaacggcgg 633420
attcgttgtt gtgaatcaga cgctcgaagc ctttggaaac cagctcgtcc agcttgatgc 633480
gctgggtgat gaaaggctca aggttgattt tgccttcttc gaccagtttg atggtttcgg 633540
cgtggtcgtt gcagtaggca atcgtgccgc gcacgtccaa ctctttcatc acgacgctgt 633600
ggacgttgat ggtggcgggg tggctccaga tggatacgat aaccaaattg gcggcaggtt 633660
tgcaggcttc gaccaaagta tccaacactt tgttgacgct ggtgcactca aatgccacgt 633720
ccacgccttc gccgttggtc agtttttttca cttctgcaac aacatcgact tcggacgggt 633780
cgaggatgta gtcggcaacg ccggattcgc gcgctttgtc tttgcgtgct ttactcaact 633840
cggtgatgat gactttgatg cctttggctt tcaacacggc agccaacagc aaaccgatcg 633900
gacctgcacc gccgaccaat gcgacgtcgc cttctttcgc gccgctgcgt acataggcgt 633960
ggtgtccgac agacagcggt tcgatcaaag cggcttgatc caacgggatt ttgtcggaaa 634020
tcggatgcac ccaacggcgt ttgacggcga ttttttcgga cagaccgccg ccgcagccgc 634080
ccaagccgat aaagttcata tctttggaga ggtggtagtt gctgccttct ccggtcggta 634140
cgtcatcgcg gatgatgtag ggttcgacca cgacgtgttg gccgactttg atgtcgtcca 634200
cgccttcgcc gacggcatag accacgccgg agaactcgtg tcccatcgtt acgggtgcgg 634260
actcgccgga aatcgggtgc ggatgaccgc aaggcggaat gaaaatcggg ccttccatga 634320
attcgtgcag gtcagtaccg cagatgccgc accaggcgac attgatgccg acagtgccgg 634380

gggcgacggt cggttcgggg atgtcttcga tgcggatgtc gcctttgtcg taaaaacgtg 634440
ctgctttcat tgtaacgctc cttgttttca agtaggaata ccgtctgaat ctggcaggcg 634500
gcggttgaaa tgggaatggc gtgaagaagc ttgaccgttt ccagttgaat ctgtttagat 634560
attttactac aagaggagac ctttgcaata acataggtta ctaaaatttt atgctcaatc 634620
tcattttcaa aatgcaaaac ttttctgatt tttcctactt tttgctcaat attaggaagg 634680
ttttaggcaa ttgaaaattt tttggcgcat ttttatgcgt caaatttcgt taacagacta 634740
tttttgcaaa ggtctcaaga gatgtgttta agcacgcgga aggctttctg tttgcgtcag 634800
gtcaaataat gatgtcgtct gaaaaccgaa tcggcttcag acggcattta tagtggatta 634860
acaaaaacca gtacggtgtt gcctcgcctt agctcaaaga gaacgattct ctaaggtgct 634920
caagcaccaa gtgaatcggt tccgtactat ctgtactgtc tgcggcttcg tcgccttgtc 634980
ctgatttttg ttaatccact atatgtcgta acggtcggat tgggtaggtt ggcgcacctg 635040
tccggttttc ggtttggcaa accgtttttt tgttgggtcc agtgttttct gataggcggt 635100
tgcggcatcg gatttgccca gccctgccag cacgcggata tgctcggcag cagattgtgc 635160
cagaggttca agggtgtagc cgccttcgag tacggatatg attttgccgg ggcagcccga 635220
tgccgtctga atgattttgt gtgtcagcca ggcaaaatcc gcctcgtgca ggttgagcct 635280
gcccgattcg tctagacggt gtgcgtcgaa tcctgccgac agcagcacca gttcgggttt 635340
gaatgcggca agtcggggta gccactgcct gcggacggct tcgcggaatg tgcggctgcc 635400
cgttcctggc ggcaagggca ggtgcaccat attgccgccg tcgggcatat cgttgttttc 635460
ggggaagggg aaaaggtcgg tttcaaacag gttgaaaaac aggatgcgcg gatcgtcttt 635520
gaatatttct gccgtaccgt cgccgtagtg gacatcgaaa tcgatgacgg caatgcgttt 635580
caggcggtat tcggcaatgg catgcatgac gccggcggca acgttgttca gcaggcagaa 635640
tccgccggct ttgccgctgc ccgcatggtg tccgggcggg cgggcggcgc aaaaggcatg 635700
ccatgcttta cggttcatga ccatgtcgac tgcctgaact gccgaaccgg cggcaaagcg 635760
tgcggcagac agcgatcctg tgctgattgc agtgtcgtta tccaggcggg aaatcttgcc 635820
tttttggggc aggcaagatt ccaaacggtt cagatatttg ctcgagtgga caagtgcgag 635880
gcgcgtatcg ctgatttctt ccgcctctat ggtttggagg tgctgccaaa taccggcgcg 635940
gcgcaatgcc tgctcgatgc agaggatgcg gtcgggcgaa tcgggatggt ttgcgccggg 636000
ttcgtgcccg gcacaggcgg gatgcgaaat ccatgcggtg cgggcgtttt tgcccaaaaa 636060
aaggcgcaac agtgcataga atttcaagat taggcgggtc aaggacatgg gtttgtggac 636120
gggcaggctg cggtatacgg tcggtacgga cggcaaaccc gatatattgt ttacggtctt 636180
atgttattat atacccctct cgattttcaa ccatattaga aagaacggat aaattatgaa 636240
tcaagctgtt gcacaatttg ctcctttagt gttgattatg gtggtgttct acttcctgat 636300
catgcgtccg cagcaaaaga aattcaaagc gcatcaggca atgcttgccg ccttgaaagt 636360
cggcgacaaa gtggtcttgg cggcaggttt caagggtaag gtaaccagag tcggcgaaca 636420
gtttttttacc gtggatatcg gacagggtac aaaaatcgag gtcgaagtgg aacgcaatgc 636480
gattgccgca aaagtcgatt gatttgtgcc gacaagccgc atctggaaag cccgaatgcg 636540
gcactttgtt ttgaattcca accgaaggct tgaccatgtt ccgacacgca gggcggcata 636600
ttcaggatgc cgctttccgg tcttgcctgg ctgggaaggg tttttgcctc ttctgaaata 636660
gcccgattcc gacaccaccg aaagggtggg gttccaacca ttaaggaaca atgatgaacc 636720
gttatccttt atggaaatat ctgctgattg tgttcacgat tgcggttgcc gcagtgtatt 636780
cgctgcccaa cctattcggc gaaacacccg ccgtgcaggt atcgaccaac cgacaagcca 636840
tcatcatcaa cgaacagact caattcaaag tggatgccgc gctgaaaaac gcaggtattc 636900
agaccgacgg gatgtttgtt gtggacaatt cactgaaagt gcgtttcaaa gacacagaaa 636960
cgcagcttaa agcgcgcgac gtcatcgaaa acactttggg cgaagggtat attaccgcgc 637020
tcaacctgtt ggcggacagc cccgaatgga tggcgaaaat caaagccaat ccgatgtttt 637080
tgggtttgga cctgcgcggc ggcgtgcatt tcaccatgca ggtcgatatg aaagcggcga 637140
tgcagaaaac gtttgaacgt tattcgggcg acatccgccg cgaactgcgc cgcgaaaaaa 637200
tccgcagcgg cacggtgcgt caggctggaa acagcctgac cgtccctttg caggatgcag 637260
gtgatgtgca aaaggctctg ccgcagttgc gcaagctgtt tcctgaagca acgctgaatt 637320
cagacggcag caatatcgtc ttgacgcttt cggaagaggc ggtcaataaa gtgtgttccg 637380
atgcggtcaa acagaacatc actaccctgc acaaccgtgt gaacgagttg ggcgtggccg 637440
agcccgtcat ccagcagtcc ggtgcagacc gtatcgtcgt gcagcttccg ggcgttcagg 637500
atactgccaa ggcaaaagac atcatcggcc gtaccgcgac tttggaattg cgtatggtgg 637560
aggacgatcc tgccaagttg cgcgaggcat tggaaggcaa cgtgccgagc ggttatgagc 637620
tgctttcaag cggcggagat cgtcccgaaa ttctgctgat cagcaaacag gtcgagctga 637680
cgggcgacaa catcaacgat gcgcaaccga gtttcgacca aatgggcgca cctgccgtca 637740
gtctgagctt ggacagcgcg ggcggcagca ttttcggcga actgactgcc gcaaatgtcg 637800
gcaaacgcat ggcgatggtt ttgatcgacc aaggaaaatc cgaggttgta accgcgccgg 637860
ttatccgtac tgccattacc ggcggacgcg tggaaatttc cggaagcatg acgacagccg 637920
aagccaatga tacgtctttg ctgttgcgtg ccggttctct tgccgcaccg atgcagattg 637980
tcgaagaacg taccatcggt ccgtctttgg gtaaggagaa catcgaaaaa ggcttccatt 638040

cgactttatg gggttttgcc atcgttgctg cattcatggt ggtttactat cgtctgatgg 638100
gtttcttttc taccattgca ttgagtgcca acatactgtt cctaatcggt attttgtctg 638160
ccatgcaggc aacgttgacg ttaccgggta tggccgcgct ggcgttgact ttgggtatgg 638220
caatcgactc caacgtcttg attaacgaac gtatccgcga agaattgcgt gccggcgtgc 638280
cgccgcagca ggcaatcaat ctcggtttcc aacacgcatg ggcgaccatt gtcgattcga 638340
acctgacttc gctgattgcc ggtatcgcgc ttttggtatt cggttccggc ccggtacgcg 638400
gttttgcggt cgtacactgt ttgggtattc tgacttcgat gtattcatcc gtcgtcgtat 638460
tccgtgcgtt ggtcaatctg tggtacggac gcagacgcaa attgcagaat atttccattg 638520
gttcggtgtg gaagccgaaa gccgaaatgg caggaggcaa ggagtaagct atggaactct 638580
ttaaaatcaa acgcgatatt ccgtttatga gctacggcaa actgacgacc ttcatttcgt 638640
tggttacgtt tatcgctgcc gtgttctttt tggttaccag aggtctgaat ttctctgtcg 638700
aatttaccgg cggtacggta atggaagtcc aatatcagca gggtgcggat gtcaataaga 638760
tgcgcgaacg cctcgatacg ctgaaaatag gtgatgtaca ggttcaggca ttgggtacga 638820
acaaacacat catgatccgc ctgccgaaca aagaaggtgt tacttccgca cagttgtcca 638880
atcaggttat ggatttgctg aaaaaagaca gtcccgacgt taccttgcgc caagtcgaat 638940
ttatcggccc gcaagtcggt gaggaattgg taagtaatgg attgatggct ttaggttttg 639000
tcgttatcgg catcattatt tacctgtcga tgcgttttga atggcgtttt gccgtatctg 639060
ccattatcgc caatatgcac gacatcgtga ttattctcgg ctgctttgcc ttcttccaat 639120
gggaattttc gctgaccgtc ttggcgggta tccttgccgt attgggctat tctgtgaacg 639180
aatccgtcgt cgtcttcgac cgtatccgtg aaaacttccg caagccggcg atgcgcggac 639240
atgccgtgcc ggaagtcatc gacaacgcga ttaccgcaac gatgagccgc accatcatta 639300
cccacggttc gaccgaggcg atggtcgtat ccatgctggt gttcggcggt gcggccttgc 639360
acggcttttc tatggcgttg accattggca tcgtgttcgg catttattct tccgtattgg 639420
ttgccagccc gcttctgcta atgttcggtt tgagccgcga caatatcggt aaagaaccga 639480
agaagaaaga agaaatcgtg gtttgaagcg catatgccgt ctgaacattg ccgtctcaag 639540
cagacaatgc ttcagacggc atttttaacg gttacttcca cggtcttaaa atattgtgca 639600
gaaatgcggg aattgtgtca taatgccacg ttgtcctatc ttgggcatag ggagtttgcc 639660
gttgtcttca ggcttggcaa acttgtctga atccctatgg ggattcttat atttttggag 639720
ttttcattat ggcactgacc gtagaacaaa aagcacaaat cgttaaagat ttccaacgca 639780
aagaaggcga caccggctct tccgaagtac aagtcgctct gttgactttc cgcatcaacg 639840
acctgacccc ccacttcaaa gccaacccca aagaccacca cagccgtcgc ggcctgttga 639900
aaatggtcag ccaacgccgc cgcctgctgg cctacttgcg ccgtacccag cccgatacgt 639960
atcgcgcgtt gattacccgc ttgggtctgc gtaaataatt acgctttccg acaccgccca 640020
gaaaaatggg cggtgttttc ttttctgttg ctttccgaca agctcaaatc catatttata 640080
gtggattaaa tttaaatcag gacaaggcga cgaagccgca gacagtacaa atagtacggc 640140
aaggcaacgc aacgctgtac tggtttaaat ttaatccact atattgcccg aaaaccgcat 640200
aaactaatat aatataaagt tctttggaat cttgttccat ttcatgctgc ccgtgcgctt 640260
tacaagagtt tcagacggca tcaaacgttt aactcccgcc agcaatcaaa cagcttttta 640320
tcacccattc gaaaatccgt tttgccggta ctcgtctttt tattggagta ttgccattat 640380
gaccgcaacc actgcgtctt cagccaaacc ttatctcaaa atccaaggtt tggtgaaaaa 640440
gtttggtgac aattacgctg tcgataacat cgacttggac atttatcaac acgaaatctt 640500
cgcccttttg ggcagttccg gcagcggaaa atctacactg ctgcgtatgc tggcgggtat 640560
ggaaagtccc aatcagggaa aaattatcct tgatggtcag gatattacca aacttgcacc 640620
ctatgatcgc cccatcaata tgatgttcca aagttacgcg ctttttccgc atatgaccgt 640680
agaacaaaac attgccttcg gtctgaaaca ggacaaaatg cctaaaggcg aaatcgccgc 640740
gcgcgtcgaa gaaatgctcc gcctggttca gatgaccaaa tttgctaaac gcaaaccgca 640800
ccaattgtcc ggcggtcagc agcagcgcat tgctttggca cgcagtctgg caaaacgtcc 640860
gaaaattcta ctgctggatg agcccctcgg tgcattggac aaaaaactgc gccaacaaac 640920
ccagcttgag ttggtcaata cgctggaaca agtcggcgta acctgtatta tggttacgca 640980
cgaccaagaa gaggcgatga cgatggcgac ccgcatcgcc attatgtctg acggtcagtt 641040
gcagcaagtc ggcacaccca gcgacgtgta cgactatccc aacagccgct tcactgccga 641100
gtttatcggc gaaaccaaca tctttgacgg tgtggtgatt gaagatcatg ccgactatgc 641160
cgttatcgaa tgcgaaggtt tggaaaacca cgtccgcatc gatcacggtt tgggtggtcc 641220
gagcgagcag gacctttggg ttagtattcg accagaggat attgatttat ataaagaaaa 641280
acccgaatat ttgggcgact acaactgggc gaaaggcacg gtaaaagaaa tcgcctattt 641340
gggcagcttc gccatttacc atatcaagct cggcaacggg cgcgtcgtca aaagccaagt 641400
ccccgcccct tactggtatg tgcgcaacat tacaccgccg acttgggacg aaaccgtcta 641460
tatcagctgg ccggaaaacc aaccgactcc gttgttccgt tgatttaagg ggaatgcaat 641520
gaaccttaat aaactgaaaa acaaactgtt ccgccgtccg gggcagcgtg cggtgattgc 641580
cgtaccgtat atttggcttt tggtgctgtt tctgattccg ttcgccatcg tgctgaaaat 641640
cagctttgcc gaacaagaaa tcgccatccc gccgtttact cctttaacga cgatagatga 641700

ggatttgggt cgtctgaata ttgctgtcag ctaccaaaat tatgcagaca tcttccaaaa 641760
tttttggagt acgctcaatc cgttcggcga cggtgaaaac agcaatatct atctgatgac 641820
ttattggtct tcaattaaga ctgcgctgac tacgacggta atttgtctgt tggtcggtta 641880
tccgaccgcc tatgcgattt ctcgtgccaa tccttctgtc cgcaatggtt tgctgcttgc 641940
cattatgctg cccttttgga catcgttcct gttgcgcgtc tatgcgtgga tgggtctgct 642000
cgggcataac ggcattgtaa acaacctgtt gattaaaatg ggtattatca gcgagccttt 642060
ggatttgttc tacaatgcct tttcgctcaa tttggtgatg gtttacgcct atctgccgtt 642120
tatgattctg ccgctataca cgcaactggt gaaactcgac aaccgcctgc ttgaagcggc 642180
ttccgatttg ggcgcggggc cggtcaaatc gttcttgacg attaccctgc ctttgtcgaa 642240
aaccggcatt attgcaggct ccatgctggt tttcgtccct gctgtcggcg agttcgtcat 642300
tcccgagctg gtcggcggtt cggaaaacct gatgattggt aaagtcttgt ggcaggcgtt 642360
cttcgatcaa aacaactggc cgctggcttc cgccgtcgcc gtcgtgatgg tcgcgctgct 642420
ggtcgtgccg attgccctgt ttcagcatta tgaaaaccgc gaattggaag aaggagccaa 642480
ataatgcaga aatccaaatt atcttggttc ttgaaactga tgttggcact gtcgctggcg 642540
tttctgtata tcccgctggt tgttttggtc atctattcgt ttaacgaatc caagctggta 642600
accgtttggg gcggcttttc gaccaagtgg tacggcgcat tgctggaaaa cgacaccatc 642660
ttggaagccg cttggctgtc gctgcggatt gccgttgtgt cttcgcttgc cgccgtcgtt 642720
ttgggcacgc tggcaggcta tgcgatggcg cggattaaac gttttcgcgg cagtaccttg 642780
ttcgctggca tgatttccgc acctatggtg atgcccgacg tgattaccgg tctgtctatg 642840
ctgctgctga ttattcaggt acagatattt ttgcagggca gcgaatggtt acaacatctc 642900
tacttcgatc gtggcttttt caccatcttc ctcggacata cgacgctgtg tatggcgtac 642960
attaccgttg ttatccgttc gcgtctggtt gagcttgacc agtcgctcga agaagccgca 643020
atggatttgg gcgcgcgccc gctgaaaatc tttttgtca tcactttgcc tttgattgcc 643080
cctgccatcg cttcaggctt tctgctcggc attaccctgt ctttggatga tttggtgatt 643140
acctcattcc tctccggccc cggttcatcc acattgccgc aggtgatttt ctccaaaatc 643200
aagttgggtc tcgatcctca gatgaatgtc ttggcgacca tcctaatcgg catcatcgga 643260
acattggtca tcatcgtcaa ttattggatg atgaggcagg caaccaagcg tgaccgagaa 643320
gcggcagaag cctaccgcca ggaaaaattg gctgccgaga aagcaaatta attaataagg 643380
caggctgacc gcatgactgg gtcagcctgt tttcttcaac cgattttctg tttggacgat 643440
atggcccgac agcctgtatc attccgtccg aaaatacacc tgataaagca aacacaatga 643500
ttcgccctga ttttcaagaa tatctgcctt cttattattt cagttcggtt aatcctcata 643560
ctgtttatcc gaaacttcaa tgccgtctga aaaccgatac ctgtatcatc ggcggcggat 643620
tgggtggttt gtgcactgca ttgcccttgg cggagcaggg acatgaaacg gttgtgttgg 643680
aagccgcgcg tatcggtttc ggcgcgtcgg gacggagtgg cgggcaggtt atcagcgatt 643740
acgcctgcgg tatgggggaa attgaaaaac aggtcggctt ggagcaggcg caatggtttt 643800
ggcaacagtc tttgcaggcg gtcgaactgg tggacgaacg cgtccgcaaa catgccgtcg 643860
attgtgattg gcagcgcggt tatgccacgg ttgccgtccg tccgcagcat tgggaagagt 643920
tgcagcagtg gcatgaacac gcccaacggc attacggtgc gagtcattat caactttggg 643980
ataaagccga gttgaaacag cagcttgaca gcgatatgta ccaaggggca caattcgacc 644040
ccttatccgg acacctgcat ccgctcactt acactttggg catcgctcgt gccgctgccg 644100
aagccggtgc gcagattttc gagcaatccc cgatgacgtg catcgaaccg catcaaaacg 644160
gttggctggt ttacacgccc gaaggcagcg tcgagtgcaa aaatgtggtc tatgctgtca 644220
atacttatgc aggtttgaac ccgatattcc ggcctttgga acgcaaggcg attgctgtca 644280
gcacctttat tattgcgacc gaacccttgg gggcgcgcgc aaaagggctt atccgtaaca 644340
atatggcagt atgcgacaac cgccatattt tggattatta ccgcctcagc gcggacggca 644400
gactgctttt cggcggtaag gataacgagt ttatcgacaa tcctgagcgt atgaccgagc 644460
ttgtccgcca agatatgctt aaagtttttc cgcagcttgc cgatgtcaaa atcgaatatt 644520
cgtggggcgg ggagtgcgac attaccgcca accttgtccc gcatttcgga cgtttagccc 644580
cgaatgtttt ttatgcgcaa ggttattccg gacacgggat ggcgataaca ggcattgcag 644640
gtctggcggt tgccgaagca attttagggg acgaatgccg tctgaagccg tttgagcggt 644700
tgcgccagcc gaatattatc ctgcaaccgt ttttgcgcaa actcggttct ttcctcggct 644760
cgaaatatta tcagtggaaa gacagccgtt aagcgtcgca ggcagtatag tggattaaca 644820
aaaaccagta cggcgttgcc tcgccttagc tcaaagagaa cgattctcta aggtgctgaa 644880
gcaccaagtg aatcggttcc gtactatctg tactgtctgc ggcttcgtcg ccttgtcctg 644940
atttttgtta atccactata tgtttatcca tcggcggcaa acgtgaaaaa tgccgtctga 645000
aacccgattt tcaggcttca gacggcatag ccgcccttat tccacgcgtt cgccgtggat 645060
attcagatcc aaaccttcgc gttcgacatc cttgccgacg cgcaggccgc cgcagatttt 645120
ccccacgacc ttcaaaatcg cccaactcat tagcccgctg tatgccgcca taacgacccc 645180
gtcttttacc tgtatccaca actgctgcca aactgccgca tccccgccga aaatgcggtt 645240
gtcgaaaaag atgccggtca atattccgcc caccagcccg ccgaatccgt gtatgccgaa 645300
agcgtccaaa gaatcatcgt aacgcaattt gtgtttgacg acggtgacgg acacaaagca 645360

cgcggcggca gtcaatatac cgatggcggc cgcgcccgac gggccggtaa agccggcggc 645420
aggggtgatg ccgaccagac cggaaaccgc gccggaagcc agccccaaag cggaaggttt 645480
gtgtcccgct attttttcgc aggcaagcca gcctgccgcg ccgaatacgg ccgacacctg 645540
cgttaccgcc atcgccatac ccgccgccgc gtctgccgca agcgccgatc cggcgttaaa 645600
gccgaaccag ccgaaccaca acattgccgc gccgatcagt gtcatcgcca tattgtgcgg 645660
aggcatcgcc tcgcgcccgt agcctatgcg cctgcccaaa accaaggcgg cgacgagtcc 645720
cgcgataccg gcattgatgt gcaccaccgt accgccggca taatccaata cgccgccctt 645780
gctcataaag ccgccgcccc acacccaatg cgcgcccggc acataaacca ataaaaacca 645840
tatgcccgaa aacagcatca ttgccgaata tttcatccgt tcggcaaacg cgccggtaat 645900
aatggcggtc gaaataatgg caaacgtcat ctgaaaaaac ataaataccg gttcgggaac 645960
agtcggcgca ttgggcgaca cggtcagcat ctgtgcggta gcgtctatct gcatcccgct 646020
taaaaatacg cgccccaaac cgccgataaa ggcatttccc ggcgtgaacg ctaaagaata 646080
gccgacggcg acccaaagga tgcccaccaa tgtcgcgatg gaaaagctgt gcatcatcgt 646140
cgagagcagg tttttttttcc gcaccatacc gccgtagaat aaagccagcc cgggaagcgt 646200
catcaacagt accaaggcag ccgcagtcat cacccaggcg gtatcgcccg aattgacggc 646260
ggaataaggc ttccaccagt ttaaaggttc tgccgatagg gatgccggca gcaaagatgc 646320
cgcccatatg tgttttttca ttttgactaa agtttcctta atggttgagc ccgtctttcg 646380
gaaaggcggg gtcggggctt gtccgggagg gacgcaagcc ctgccggacc ggggcggcgc 646440
ggggattttg ccgatgtgcc gccaatccct tgtttgaata tggaaatatc gcatccgatc 646500
ccttgcaccc gttgtccggc gggaggattt atccttaggc ggcgcatatg tgggcgtatg 646560
gattgtcaac aatttactgt aggaaaatat acagaggttt gggcgataag gcaaaatatt 646620
gttgacaata tttttatttt ataaaattaa tttattgatt aatatattaa aaatttttaa 646680
ttggaaatat agtggattaa caaaaatcag gacaaggcga cgaagccgca gacagtacaa 646740
atagtacgga accgattcac ttggtgcttc agcaccttag agaatcgttc tctttgagct 646800
aaggcgaggc aacgccgtac tggtttttgt taatccacta taaaaattta tggggctgtc 646860
ctagataact aggataaact cgattttact aattgtttta aaatggaaat ttgaactttt 646920
atctcgctgt tgttaaaacg tcgttcgtac ccctttaaat acagctcaaa atgcgctttg 646980
ggaatgccgt caaacttgcg taaatgacgt tttgcccggt tccaaaagtt cccaattcca 647040
ttgatatggt tttgtcgttc agcaaaataa ctttcatctg cttctacttc gccgtcaaac 647100
atttccaaat gcggactgtt ttgataaata agtaatcgta aacgatgaaa ataataggct 647160
gaggtacttt tattaacgcc tactaactct gctgctgttc ttgcagttac acctgcgaca 647220
aacagttcaa tgagtttatt ttgtttatac cggcttagac gaatttttct catagggggca 647280
actctaactt aatttgaatt tccctagtta tctaggacag ccccaaattt atacaaaaat 647340
gagtgcggtt cggcgcaacc ttgaatcaag ttcccgcatc ggttttcatt gccggtacgg 647400
atgcgttcaa gccggctttg caaaggccgc gctttcggca agcggacacg gacactgccg 647460
acggttgcgc cgttaacggg gggagggagg agctgcgccg accgtgtgaa tgaaagtgcc 647520
gtctgaaacc cgattttcag gcttcagacg gcatttcgca ttaatgcggg cggcgcgttt 647580
atttgccgcg catcagttca aagaaatcgt cgttgttttt agaggctttg attttcccga 647640
ttaaaaattc ggctgcctcg atttcgtcca tcgggtgcag gaacttgcgt aagagccaca 647700
tacgttgtaa ctggtcgttt gggacaagca gctcttcgcg gcgcgtgccg gatttgttga 647760
tgttgatggc ggggaagagg cgtttttccg ccatacggcg gtcaaggtgc aattccatat 647820
tgccggtgcc tttgaattct tcgtaaatca catcgtccat acggctgccg gtttcaacca 647880
atgcggtggc gatgatggtc agcgaaccgc cttcttccac gttgcgcgcc gcgccgaaga 647940
aacgtttggg acgatgcagc gcgttggcat cgacaccgcc ggtcaggatt ttgcccgagg 648000
taggcacgac ggtattgtag gcgcgggcaa ggcgggtaat cgaatccagc aggatgacca 648060
cgtctttttt gtgttccacc atacgcttgg ctttttcaag caccatttcg gcaacttgga 648120
cgtggcgttg agccggctcg tcaaaggtgg aggagactac ttcgccacgg acggagcggc 648180
tcatttcggt tacttcttcg ggacgttcgt caatcaagag gacgatgagt tcgacttcgg 648240
gatagtttgc ggtaacggcg tgggcaatgt tttgcagcat cacggtttta ccgcttttgg 648300
gcggggcaac caagagggcg cgctgacctt tgccgatagg ggaaatcagg tcgatggcac 648360
gtccggtcag gttttcttcg gactttaagt cgcgttccag cttcaactgt tcggtcggaa 648420
acagcggggt caggttttca aacaggattt tatggcggca tacttccggg tggtcgccgt 648480
tgatggtatc aagcctgacc agggcaaaat agcgttcgtt gtcttttggg acgcgcacgc 648540
ttccttcgat ggtgtcgccc gtatgcaggt tgaagcggcg gatttgggtg ggcgagacat 648600
agatgtcgtc ggggccggca agataggacg tgtccgcgct gcggaggaag ccgaagccgt 648660
cgggcaggat ttcaagcgtg ccggagcagg tgaaaccctc gcctttttttc atcatctggc 648720
ggacgatggc aaatacgagg tcttgtttgc ggaatcggtt ggcgttttcg atgccgtgtt 648780
cttccgccaa ttctaagagt ttggaaatgt gcagggtttg taattcggag acgtgcataa 648840
taatgatgta ttttgaagag gaaaaagaca ggcagatgcc gtctgaaaga agaagctgac 648900
cgttgccggt tgctcgggga agggggaatt gtaggcagtc ggcgcgtggg tgtcaaatat 648960
tatcgcggac ggggcatcgg caggaaatgc cgtctgagcg gagctgcttg gaaaaaaata 649020

cccccgcgct tttcaggctc gggggtatgg gcattgatta tttgttcaat tcattcgcca 649080
aatatagcca agtttcgatg acggtatccg ggttcaggga aacgctttca atgccttcct 649140
caaccagcca tttggcgaag tccggatggt cggacgggcc ttgaccgcag atgccgacat 649200
atttgttctg cttgcggcag gcggagatgg caaggtgcag catcactttg acggcagggt 649260
tgcgttcgtc aaacgattcg gataccaagc cgctgtcgcg gtcgagaccg agggtcagtt 649320
gggtcatgtc gttcgagccg atggagaagc cgtcgaagta ttgcaggaat tgttccgcca 649380
ataccgcgtt gctcggcagc tcgcacatca taatcaggcg caggccgttt ttgccgcgtt 649440
ccaagccgtt ttctttcagg gctttgacaa cggcttcggc ttcgcccaaa gtgcggacga 649500
acggaatcat gatttcaacg ttggtcaacc ccatttcatc gcggacgcgt ttcaaggctt 649560
tgcattccaa ggcgaaacag tctttgaagt tgtcggcgac ataacgcgcc gcaccacgga 649620
agcccaacat cgggttttct tcatgcggtt cgtatacgtt gccgccgacc aggttggcgt 649680
attcgttgga tttgaagtcg gacatacgga cgatggtttt acgcggataa accgatgcgg 649740
ccaatgtcgc cacgccttcg gcgattttat cgacgtagaa gtcgacaggg gacgcgtaac 649800
cggcgatacg gcgggtaatt tccgctttta attcgtcgtc ttgtttgtca aattccaaca 649860
aggctttggg gtggataccg atttggcggt tgatgataaa ttccatacgc gccaagccga 649920
tgccttcgct gggcaggttg gcgaagctga atgcgagttc gggattgccg acgttcatca 649980
tgacttttac aggtgcttta ggcatattgt ctaaggcgac atcggtaatc tgtacgtcca 650040
acagaccggc atagataaag ccggtatcgc cttcggcaca ggatacggta acttcttgac 650100
cgtttttcag caattcggtt gcattgccgc agccgacaac ggcaggaatg cccaattcac 650160
gcgcgatgat ggcggcgtgg caggtacggc cgccgcggtt ggtaacgatg gcagaagcac 650220
gtttcatcac gggttcccaa tccggatcgg tcatgtcggt aacgagtacg tcgccggctt 650280
cgacggaatc catctcggaa gcatctttaa tcaggcgcac cttgccctga ccgactttct 650340
gaccgatggc gcggccttcg cataatacgg ttttgtcgcc gttgatggcg aagcggcgca 650400
ggttgcggtt gccctcttct tgggatttta cggtttcggg acgggcttgc aggatgtaga 650460
gtttgccgtc caagccgtcg cgtccccatt cgatatccat cgggcggccg tagtgttttt 650520
cgatggtcag tgcgtaatgc gccaactcag taatttcttc gtcggtaatg gagaagcggt 650580
tgcggtcttc ctcggggaca tcgacgttgg ttacggattt accggcttct gctttgtcgg 650640
taaaaatcat tttgatgtgt tttgaaccca tggttttacg caggatggcg ggcttgcccg 650700
ctttgagcgt gggtttgaac acataaaatt cgtccgggtt gaccgcacct tgtacgacgt 650760
tttcgcccag accgtaagag gaggtaacaa agacgacttg atcgtagccg gattcggtgt 650820
cgagggtgaa catcacacct gatgcgccgc tgtcggaacg caccatgcgt tgaacgccgg 650880
cggaaagggc gacgatgtcg tgttcgaagc ctttgtggac acggtaagaa atggcacggt 650940
cgttatacag ggaagcgaat acatggtgca tcgcttcttt aacgttatcc aagccgttga 651000
tgttcaagaa ggtttcctgt tgtccagcga atgatgcgtc cggcaggtct tcggcagttg 651060
cggaagaacg tacggcaacg gaaatgtccg caccgccggc atcggcaacc attttgttcc 651120
atgccgcttc gatttcggca tcgagctgtt cggggaaagg cgtatccaaa atccattggc 651180
ggatttcttt gccgacgcgt gccagttcgg caacgtcttc gacatccaat tttgccagtg 651240
cggcggaaat gcgttcgctc agaccgttgt gtgcgaggaa tgcgcggtag gcttcggccg 651300
tggtggcaaa gccgccgggg acgcgaacgc ctttttcggt cagctgactg atcatttcgc 651360
ccagcgaggc gtttttaccg cccacgcgtt caacatctgt catacgcagg ttttcaaacc 651420
agattacgta gttgtcggcc atttgtgtgt ccaatccaaa atatgttaaa aaagaaacaa 651480
atccgcgtgc ttattttaag cgattcgttc ctctgctgtc atgtgtttta tccgttttaa 651540
aatcatgatg ccgtctgaaa aattgcggtt tcggcgtgtg tagcggtttg aaacttacag 651600
ccggtatact tcttttttg ggtattttct ttgtaaaaca ggtggtttga ataggttaat 651660
gtttttctg tttgattttt ttgtttattt tttaaaattt tctgccaaaa aatactttat 651720
ataaataatt ttatttcaaa attatattgt gtctgtttgg gtgtaatccg aggtaggtgt 651780
gctgcgggtg ctttccttgt gtctgctgct gctgttatga tgggatttta aacctgtgtt 651840
ttaaggatgg aagatgagca gtccgcgcca tgtgttttac atttccgacc gtaccggtct 651900
gactgctgag aatatcggcg aggcgttgct gaaccagttt ggcaatctgt cgttcaaacg 651960
ccatacgcat ccgtttgtcg atacgccgga aaaggcgcgc gcggtggtgg agaaggtcaa 652020
tcggagccgg caggaaaacg gtcagcgtcc gattgcgttt gtcagtgtgg ttgatgacga 652080
aatccgtcgg attatcaaag gggcggatgc ttttcagatt aatttctttg agactttttt 652140
gggactgttg gagaaggaac tcaataccga agccacggca tccgggcagg ggcatcacag 652200
tatcggtaat acgaagcgtt atgatgcgcg tatggaagcg gtcaattttt ctttgaacca 652260
cgacgacggg gtcagcgata agaaccttca ggaagcggat gtaatcttga tgggtgtatc 652320
gcgttcgggc aaaacgccga cctgccttta cctcgccctg caatacggca tccgtgcggc 652380
aaactatccg ctgattcccg acgatttgga atcggccgat ctgccgcgta tggtcaagcc 652440
ttatagggat aagctgttcg ggttgaccat ccagccggaa cgtttgcagg ccatccgcca 652500
agagcgccgc ccgaattcaa cttatgccaa aatcgataca tgccgcagcg aggtggcgga 652560
cgcgcagagt atgttcagac ggcatgggat tccgtttgcg aatacgacgg ataagtcggt 652620
tgaggaattg gcggtacaca tccttcaggc gtgcaagctc aaacgcaggt tttgacgggc 652680

tttgattcgg tttgaaggcg gaactgccgt ctgaaatcag gtttcagacg gcagttttat 652740
agtggattaa caaaaatcag gacaaggcga cgaagccgca gacagtacaa atagtacgga 652800
gccgattcac ttggtgcttc agcaccttag agaatcgttc tctttgagct aaggcgaggc 652860
aacgccgtac tggtttttgt taatccacta tatgtttgtg gggcggatat ttttcagggc 652920
tgtattttgt ccagacattc gagcagatcg agtggcgtgc ggatgtggaa atccgcctgc 652980
catgagccgg tatcgtcttc gggagcgatg tagccccatt cggcgaggac ggtcgtcata 653040
ccggcgttgc gccccgcctg tatatcgcgt tccgcgtcgc cgacgtagag tgtgtgttgc 653100
gggtcggcgt ggatttgtcc gcacgcatac agcatgggtt tgacgctggg cttgggctcg 653160
ccgcaggtgt cgccgctgac gacgacggcg ggtgggatga tgaagccgag tttggggacg 653220
agtttgtcgg tgaagcgcat gggtttgttg gtgatgatgc cccatttgat gccgcgtttt 653280
ccgagttcgg cgatgagttc gtttacgccg tcgaagaggg tggtgtcttg ggcgtagcgg 653340
ctgtcgtatt cgtcaaggta ttcggtgcgc catcgggcat agtcgggatg gtcgggggtg 653400
atgcctgcgc cgagcttgat cagtcctgcc gcgccgtggc tggcttgggt gcggatttcg 653460
tccatgcttt ttgcaggtag tccgtggcgg gcgagcaggg tgttgagtgc gccgccgagg 653520
tctagggcgg tgtcggcgag cgtgccatcg aggtcgaaca atacggcttg tatcatgtgt 653580
gttccttttt tataaagtgc gggacgaagg gtttcagacg gcatgtttat tttgtttcaa 653640
accctgctcg aaatcttcca acatatccaa ttcaaagcgg ctgaagcctg ctttttcgcg 653700
cgcttcgatg ttcacatagc ccggaagat aaacatatcg taacgggcaa tcaggctgcg 653760
gaacagggcg acaggctcca aaccgcgttc gcggcaaagg tgttgatacc accggttgcc 653820
gatggcgacg tgtcccactt cgtcgcggta aatgatgtcc aacacgccgc aggtttccga 653880
atcaccgcgc tgcgccacct tcgcgcgtat gccgggcgta acgtccagcc cgcgcgcttc 653940
caaaacgcgc ggcactaaag ccatacgcaa caaaggatcg taggcggttt tgtatgccat 654000
atcccataaa tgattgtgtg cttcaaaatc gccgtaatcg aagccgaaag cgcgcagcct 654060
ttcgcgcatc aggcggaaat ggtacacctc ttccttcgcc actttcaccc agtcgcggac 654120
aaactgaaac ggcagcgtgc ggaaacggta tgccgcgtcc aaagccagat tgatggcgtt 654180
gaattcgata tgcgcaatcg cgtgcagcat cgccgcatag ccttcggttg tgttcatttt 654240
gcgtggcgtc agctgcgacg gcgcgaccaa aacaggcttg tccggtcgtc ccgcgcgggg 654300
gaagtccgcc ggcggtgcgt ttgtttccgc cccgtccgca ttttgaacgg cggcaaacgc 654360
ctcatccgtc agccgtcctt tttcatctgg gtcgcccgaa agcagggcgc gttccagcaa 654420
agcataaata tcgggtttca tctcaagtcc gccgtgttcg gaaaacaaat attatagcgt 654480
ttaaaaaaaa caagatgagg catataatct ccgcgattcg gcattccgcg cccaaaccgt 654540
caaatatagt ggattaacaa aaaccagtac ggtgttgcct cgccttagct caaagagaac 654600
gattctctaa ggtgctgaag caccaagtga atcggctccg tactatttgt actgtctgcg 654660
gcttcgtcgc cttgtcctga tttttgttaa tccactataa cgcggcacac attaaagggc 654720
agcgtggcgc gccgcctttt ccggtgggca aaaaatcagc cctcggaaaa cgcggtttgc 654780
aaaatgcaaa ccgcccgtaa cgccgcccgt atgattgttt tgctgcgccg atactttacg 654840
ccacactcat cccgacaagg aaaaataatg atgaaaccgc acaacctgtt ccaattcctc 654900
gccgtttgct ccctgaccgt cgccgtcgct tccgcacagg cgggcgcggt agacgcgctt 654960
aagcaattca acaacgatgc cgacggtatc agcggcagct tcacccaaac cgtccaaagc 655020
aaaaagaaaa cccaaaccgc gcacggcacg ttcaaaatcc tgcgaccggg ccttttcaaa 655080
tgggaataca ccaaacctta caggcaaacc atcgtcggcg acggtcaaac cgtttggctc 655140
tacgatgttg atctggcaca agtgaccaag tcgtcccaag accaggccat aggcggcagc 655200
cccgccgcca tcctgtcgaa caaaaccgcc ctcgaaagca gctacacgct gaaagaggac 655260
ggttcgtcca acggcatcga ttatgtgctg gcaacgccca aacgcaacaa cgccggctac 655320
caatacatcc gcatcggctt caaaggcggc aacctcgccg ccatgcagct taaagacagc 655380
ttcggcaacc aaacctccat cagtttcggc ggtttgaata ccaatcccca actctcgcgc 655440
ggcgcgttca agtttacccc gcccaaaggc gtggacgtgt tgagcaactg atgccgtccg 655500
ccccgatgcc gtctgaaagc cgccgaggct tcagacggca tttttacgca ggcggaacaa 655560
tgtcccgcat taccgccccga tcgggcaccg gaacggcaaa ccggtgaaaa ttaacggttg 655620
cgccccggct gtttttgccg tttaatgcaa accttgctgc accaagggcc aagaaagccg 655680
accggccgcc cccacagctt ccgatgcagg cggcccgtcc gtccctgcaa tgttttttat 655740
ttttgaacga aaggtcgaaa accatgaaaa aaacactggt ggcggcggca atcctgagcc 655800
tcgccttgac tgcgtgcggc ggcggaagcg ataccgccgc ccaaaccccc tccgccaagc 655860
ccgaagccga acaatcgggc aaactcaaca tctacaactg gtcggattat gtcgatcccg 655920
aaaccgttgc cgcctttgaa aaagaaaccg gcatcaagac gcgttccgat tattacgaca 655980
gcaacgaaac actggaggca aaagtcctga ccggcaaatc cggctacgac ctgaccgcgc 656040
cgtccatcgc caacgtcggc cggcaaatca aagcgggcgc gtatcagaaa atcgacaagg 656100
cgcaaatccc ccattacggc aacatcgata aagatttgct gaaaatgatg gaagccgtcg 656160
atccgggcaa cgaatacgcc gtcccctatt tctggggcat caataccttg gcaatcaata 656220
cccagcaggt gaaaaaagca ttgggtacgg acaagctgcc cgaaaacgaa tgggatttgg 656280
tgttcaaacc cgaatacacc gccaaactca aatcctgcgg catcagctat ttcgacagcg 656340

caatcgaaca gattcccttg gcgttgcact atttgggcaa agaccccaac agtgagaatc 656400
ccgaagacat caaagccgcc gtcgatatga tgaaagccgt ccggggcgac gtgaaacgct 656460
tcagctcttc cggctatatc gacgatatgg cggcgggcaa cctgtgtgcc gccatcggtt 656520
acggcggcga tttgaacatt gccaaaaccc gtgccgaaga agccgcaaac ggcgtggaaa 656580
tcaaagtatt gaccccgaaa accggcgtgg gcgtgtgggt ggattccttt atgattccgc 656640
gcgacgcgca aaacgttgcc aatgcccacc gctatatcga ctacacgctc cggcccgagg 656700
tggcggcgaa aaacggcagc ttcgttacct acgcgcccgc cagccgtccc gcgcgcgagc 656760
tgatggatga aaaatacacc tccgacgcat cgattttccc gaacaaagaa ctgatggaaa 656820
aaagtttcat cgtatcgccc aaatccgcag aatccgtcaa actgggcgtg aagctgtggc 656880
aagggctcaa agcgggcaaa taaccggaat ccctgccgtc tgaaaccttt cgggcggcag 656940
gaaacggcgc gtccgttatc aaacaggggg gcgtttcccc tcctgccggt tatgattggg 657000
ttaagattaa aatgatttag taaaatgaga aagatatgga tttaagtatc gtagttccta 657060
tttataatgt cgaaagttat ttggaagcgt gtttaagttc catagaatct atattaagta 657120
atgaaaatgt cgaacttatc cttgtgaatg acgggtcaaa agacggaagt gaagatatat 657180
gttacaaata tatagataaa atatcaaaca gcaaacaaca aacaacaaac aacaaacaac 657240
aaacaacaaa caacaaacaa caaacaacaa acaacaaaca acaaacaaca aacaacaaac 657300
aacaaacaac aaacaacaaa caacaaacaa caaacaacaa acaacaaaca acaaacaaca 657360
aacaacaaac aacaaacaac aaacaacaaa caacaaacaa caaacaacaa acaacaaaca 657420
acaaacaaca aacaacaaac aacaaacaac aaacaccgga caccaaatat caaatatata 657480
tatcaggata accaagggtt gtcggaggcg agaaataccg gaataaaaaa ttcaaacgga 657540
aaatatatag tctttattga ttcggatgat tttattaact gtcagatttt gctggatttt 657600
cttagtaaag atgatactga tatgccggat gtggtgtttt taaatgcggt taaatatgat 657660
aagggaagtg tttcatattt tggcgaagat tatcagcctg aaaaaatact caatcaatcc 657720
aaagtcgaag ttttgaaagg attatgccga tttagaaaat ttccgggttc ggcgtggaat 657780
aagattataa aaagagaatt gattattaga gaaaaactgt tttttgaaag gggaatttat 657840
tctgaagata tcgaatggtc aatgaggtta tttaatgcgg caacaacttt ttcttatttg 657900
gacggttgtt attactatta tcggcaggga agaaaagatt ctattacggg aactgtttcg 657960
gaaaaaagta taaagtcatt attatatatt ttggagaaaa atgcggaaat ggaatttaat 658020
agggatatat cgagttatct ttattctttt ctttcctacg aatatctcgt tttgcttttt 658080
ataatgacga gtaaaaatat agagtgtgat gctgatataa aaagaagggc gtatcattta 658140
aggtttatgc tgttaaagtc caataaattg atatataagc tgatattccc gataatcaca 658200
ttactcgggg tcgatattac aggcaggatt ttaaaagcaa tcagggggaa tatttaataa 658260
atcctttaac aatatatacc ttaccgaagg aggaaaaatg aacgcaatcc gaactttcca 658320
aaaccgcacg cccgaaatcc acgaaacctg tatgatagac gaagcctgcg tcgtcattgg 658380
cgaagtgtcg cttgccgaag atgtttccgt gtggccgtgc gccgtgttgc gcggcgatgt 658440
gaacagcatc accgtcggcg cgcgcagcaa tatacaggac ggcagcgtct tgcacgtttc 658500
ccacaaaacc gccgccaaac ccgaaggatc gccgctggtt atcggcgaag acgttaccgt 658560
ggggcacaaa gtgatgctgc acggctgccg tatcggcaac cgcgtcctgg tcggcatggg 658620
gacgacggtt ctggacgatg ccgtgattga ggacgaagtg atgatcggcg cgggcagcct 658680
cgttccgccg cgcaaacgct tggcgggcgg ctatctttat gtcggttcgc cggtcagaca 658740
ggtgcgcgtg ctgaccgatg aggaaaaagc ctttttgaaa tattccgccg cgcattatgt 658800
gaagctgtcg aaacagtacg ggatgtgaaa tcacatcggc gttcttgcgt cagccccaaa 658860
ttcatgcgga tgggacgcat ccgataacgg tatccgatgc gccttgattt tgaccgtctg 658920
cgtttgaatt gcaggcaaaa atgccgtctg aaagcctttt ttcgggttca gacggcattt 658980
tattgccgat tgttttttaa agtttgaccg aatgttcgcg cgtttcgtgg aacacgatgt 659040
ccggccagcg ttcttgcgtc agccctaaat tcatgaggac gggatgcccg ataacggtat 659100
ccgatgcgtc ttgattttga tcggtgcatt tgagttgcag gcaaaaatgc cgtctgaaag 659160
cctttttcg ggttcagacg gcattttatc gccgattgct ttttacagtt tgaccgaatg 659220
ttcgcgtgtt tcgtggaaca cgatgtccgg ccaacgttct tgcgtgagtc ccaaattcac 659280
gcggttgggg gcgaggtagg cgaggttgcc gcctgcgtcg atggcgaggt tgcccgcgtt 659340
ggctttttca aattcagcca gttttttctt gtcgtcgcac gatacccagc gcgccgacca 659400
gatggatgcg ctgtcgaaca cggcttctac gccgtattcg ttggcgaggc gcgaggtaac 659460
gacttcaaac tgcaacacgc cgaccgcgcc caaaatcaaa tccgcgccgc tcatcggttt 659520
gaacacctgc accgcgcctt cttcgccgag ctgttgcaag cctttttgca gttgtttgat 659580
tttcagcggg tttttgatgc gtacgctgcg gaacagttcg ggtgcgaaga atgggatgcc 659640
ggtgaacgcc agttgttcgc cttcggagaa gctgtcgccg atttggatgt tgccgtggtt 659700
cgggatgccg ataatgtcgc cggcgtaggc ttcttcaacc agctcgcggt cgtgcgacat 659760
gaaggtaacc acgctggagg cggcgatttc gcggttgata cgcaggtgtt tcatcttcat 659820
gccgcgctcg aatttgccgg agcagacgcg caagaaggca atacggtcgc ggtgtttcgg 659880
gtccatattg gcttggattt tgaagataaa tccggaaaac ttcggctcgt ccggctcgac 659940
catacgtacg gtcgcgtcgc gcggtttcgg cgcgggcgcc cagtcaatca atgaattgag 660000

gatttcctga ataccgaagt tgttaatcgc agagccgaag aatacgggcg tgagttcgcc 660060
ggcgaggaat tcgtcgagat taaactcgtt ggaagccgcc tgcaccaatt cgatttcgtc 660120
gcgcaactgc tggatttcca acggaaagcg ttgttccaat tcaggattat cgatgccttt 660180
gatgatgtcg aactcgtgcg gcaggcgttc gccgccagct tcaaagagat aaatttcatc 660240
gttcaggatg tggtacacgc ccttgaagtt tttgcccata ccgatcggcc aggtaacggg 660300
cgcgcagcgg atttttaaaa tgttttccac ttcgtccaaa agttccaggg aatcgcgcac 660360
ttcgcggtcg tatttgttca taaacgtaac aatcggtgta tcgcgcaggc ggcagacgtt 660420
taagagcttg atggtttgcg cttccacgcc ttttgccgcg tcgatgacca ttaatgcgct 660480
gtccacggcg gttaaaacgc ggtaggtgtc ttcggagaag tcttggtgtc ccggcgtgtc 660540
caagaggttg acggtgtggt ctttgtaatc gaactgcatc acacttgatg ccacggaaat 660600
gccgcgctgc ttctcgattt ccatccagtc ggaagtggcg aatttgccgg ttttcttgcc 660660
ttttaccgta cccgcgctct gaatcgcgcc cgaaaacagc aagagttttt cagtcaacgt 660720
ggttttacct gcgtcagggt gggagatgat ggcaaacgtg cggcggcggc gcacttggtc 660780
gaggatttct tgggacatgg ttttctttgc aaaaaggttc aggccgcttt tcagacggcc 660840
cggacagtgt ttgagacggc gaaattgtac aaaaaaatgc ctgataattc aatgttggag 660900
gcggtcagtg cgtgctgccg taaatctctt tttcgtcttt caggacggca tcggcggttt 660960
cccacgcgct gccacgccag actttgtaaa agcagctttc tcgcccggtg tggcaggcga 661020
tgccgccgtt ttgggcgatg agcatcacaa tggcgtcgcc gtcgcagtcg aggcgcagtg 661080
cgcggacttt ttgcgtgtgt cccgactctt cgcccttcat ccattgtttt tggcgcgaac 661140
ggctgtaata gtgggcaaag ccggtttcga cggttttttg cagggcttcg gcgttcatcc 661200
acgccaccat taaaatacgt ttggtttcgg catcttgggc gatggcgcaa accaaacctt 661260
tttcgtcaaa tttgacggct tcaagcaggt ttttatccat atttcctttc agacggcata 661320
gtcgaggcgg tcagaggcgc acttcgatgc cggcttcgcg catagcgcgt ttggcttcgc 661380
ggatggcgat ttccccgaaa tggaaaatgc cggcggcaag tacggcatcg gctttgcctt 661440
cggttatgcc ttcaatcagg tgccggacat tgccgacccc gccggaggcg atgacgggga 661500
tgtcgacggc ttcggcaacg gcgcgggtca gcggcaggtt gaaaccctgt ttcgtaccgt 661560
ccctgtccat accggtgagc aggatttcgc ccgcgccgcg tttttgcatt tcgaccgccc 661620
attccaccgc atccaaaccg gtcggatttc gcccgccgtg ggtaaagatt tcccagcgtg 661680
tgttttcggg gttggcggct ttggcatcga cggcggcgac gatggcttgc gaaccgaaaa 661740
atccggcggc ttcgtcaatt aaatcgggac gggtaacggc ggcggtgttg atgctgactt 661800
tgtccgcgcc ggcattgagc aggcggcgga tgtcggcaac ggtgcgtacg ccgccgccga 661860
cggtcagggg gatgaagact tgtccggcaa cctcttcgat gatgtgcagg atggtgtcgc 661920
ggttgtcgga tgaggcggtg atgtcgagga aggtcaattc gtccgcgcct tcgccgttgt 661980
agcgtttggc ggcttcgacg gggtcgcccg cgtcgcgcaa accgatgaag ttcacgcctt 662040
tgacgacgcg cccgtctttt acgtcgagac aggggatgat gcgttttgcc agtgccataa 662100
tcggatgcct ttagtcgagg gaatctgcca gttgctgcgc ttgggcaaaa tcgatgctac 662160
cctcgtaaat cgcgcggccg gtaatcgcgc ctgctacgcc atgtttttcg gcggcacaca 662220
gggcgcggat gtcgtccaag ccggtcagtc cgccggagga gatgacggga atgcggacgg 662280
tttgggcgag tttgaccgtc gcgtcgatgt tcacgccgct catcataccg tcgcgcccga 662340
tgtcggtgta gatgatgctg ttgacgccgt cgtcttcaaa gcgttttgcc aaatcaatta 662400
catgatgccc ggttacggtt gcccagccgt cgatggcggc cataccgtct ttggcatcca 662460
gcccgacaat aatcctgccg gggaaggctt tgcacgcctc gcgcacgaag tcggggtttt 662520
tgaccgccgc cgtgccgata atcacgtcgt ttaagcccaa atccaaatat tgtccgatgg 662580
ttttcaaatc gcgtatgccg ccgccgagct gtacggggat gtctttggcg acagcggcaa 662640
ggatgtcttt gatggcgggc aggttttgcg gaacgccggc aaacgcgccg ttcaaatcta 662700
ccagatgcag gcggcgcgcg ccttgtttga accagtgcag cgcggtttcg gcgggcgaat 662760
cggaaaagac ggtcgcctct tccatcagcc cttgtttcag gcggacgcag cgtccttctt 662820
tcaaatcgat ggcgggtatc agcagcataa tttttctcct tgtgcggggc cgtgtccggc 662880
ttaccagttt aaaaagtttt tcaacatcgt cagcccggca tcgtggcttt tttcggtgtg 662940
aaattgcgtg gcgaatacgt tgtctttgcc gacgatgcag gcaaacgggg acgggtagtc 663000
gctttcgccc aatatggttt cggattttc ggggcgaaa tagtagctgt ggacgaagta 663060
aaaacgcgtg tcttggggaa tatctttaaa cagcgggtgg ttttgggttt ggcgcacggt 663120
gttccagccc atatgcggga ctttcagacg gcatccctgc gggtcgcgga ggtcgcgctc 663180
aaagcgtctg actttgccgc cgaaccagcc caagccgtcg gtgtttcctt cttcactgtg 663240
gtcgaataaa agttgcgcgc cgacgcagat tccgaaaaac ggtttgtttt ttaaggcatc 663300
tttgactgcc tcgtccaaac cgtctcgttt taatgccgcc atacagtcgg gcatcgcgcc 663360
ctgaccggga aaaatgactt tgtcggcgcg ggacacgcgg tcggggtcgc cgcttaaaaa 663420
gatttcggta ttttttccgg caagctgccc cgccgtccgg acggatttca atacggaatg 663480
caggttgccc ataccgtaat cgataatggc ggtttgcatg gcttcctcct cttttttgc 663540
aatatggctg cgattttaac aaacaaatgt gccgtgctga taaaaatgcc gtctgaaaac 663600
gggagtctgt cttcagacgg catagggttt aaacccggaa agccgtttgt cagccttcca 663660

tttgttttgc ctgaacggca gtcagggcga tggtaaacac gatatcttct accagtgcgc 663720
cgcgggagag gtcgttgacc ggtttacgca ggccttgcag cagcgggccg acgcttaaga 663780
cgttggcgtt gcgttggacg gctttatagg tgcagttgcc ggtgttcagg tcggggaaga 663840
ccaaaacggt tgcctgtcct gccaccgggc tgcccggagc tttggatttg cccacacccg 663900
gcacggttgc cgcatcatat tgcagcgggc cgtcgatggc gaggtcgggg cgtttttccc 663960
gggcaagttt ggttgcttcg atgacggtat cgacatcggg gccgctgccg gagttgacgg 664020
tggagtagga aatcatcgcc actttcgggt cgatgccgaa ggcttttgcg gaatcggcag 664080
actggatggc gatgtcggca agctgttgcg cggtcgggtt cggattaacc gcgcagtcgc 664140
cgaagacgag gacttggttg ggcagcagca taaagaatac gctggacacg aggcttgcgc 664200
ccggtgcggt tttaatcagt tgcaaagcgg ggcggatggt gttggcggtg gtgtgaaccg 664260
caccggatac caaaccgtcc acatcatttt gcgccatcat catcgtaccg agtaccacgg 664320
tgtcttgcag ttgcttgcgc gcgtcttcgg gtgtcaggcc tttggatttg cgcagttcgc 664380
acatcggctc gacgtattgt tcgaccaatg aggcgggatc gatgatttcc aaagagtcgg 664440
gcaggctgat gccgcgttct ttggcaacgg cttcgacttc ttcgcgtttg gcaagcagga 664500
cgcagcgggc aatgcctttt tcgtggcaga tggcggcggc ttggacggtg cggggttctg 664560
cgccctcagg caggacgatg cgtttgtcgg cttggcgggc gaagtcgatc aggttgtagc 664620
ggaattgcgc cggcgacagg cgttttgctt cgcggcctgc caatacggat acgtctttca 664680
gcgcgtcgct cgaaccgaag aaggtcaggc cggtttttttc ggctgccgct tcggcaacgg 664740
aggctgccgc gccgtccacg acaaaacctt ccaatacgcc cggcgcggcg gcgaagaact 664800
gtttggcaag gttcacccga tttgccagtt cgtcggcatc ggcgttgtcg gaacggacgg 664860
cgaagacggc tgccgcgtca agggacaatg ccagttcgac gtttttgcct gcgaggtaga 664920
ttttgtcggc atcgggcgcg atgccttcga tgacgaggtt ggcggcatcg agtgcggcaa 664980
ctttgccgac cagtgcgtcg aaccagtcgt cgcttttgcc ttgcgcgagc agggtttcgg 665040
cggttgcgtc aacggcttgg aaaatttgtg cgtccagtgc ttttgcaaag gcttgtgcgg 665100
cggcggaggc gtccagtccg gcagatacgg gtacgatgag tacttttgcc atgatatatc 665160
ctttcgtatg ctgcggtgtg cggcatatgt ggttggaagg ggcggcatat aggcagaaac 665220
ggctgcctgc gtgccgtgcg tgccgtgttt ggcttgaggc gcgcaggttg aatatagcaa 665280
acaaattctg tttccaacaa gataaatatc cgcaggcttg tggatgctgc cgcctttcag 665340
agggtatttc cggggaagaa cagggcggga ccgtccaaat ggaggacggc ggaaatgccg 665400
tctgacaggg tgggggcgga agggaggttg agcgtgagga cggtttgtcc gacccggagg 665460
ctgatttcgg tatgccgcgc tttgggcgtg gttttgagaa ccacggcgtg aatggaggcg 665520
gtgggtgcgg aatgggggtg aaggctgaac tgttccggac ggatgagcag tgtgccgcgt 665580
gtgcctgcgg gtgcgccgct ttgaacgggc aggcggccca atccgcaatc ggcggtgccg 665640
tcggcgttga gcgcggcggg gaacacgatg ccttcgccga taaacagggc ggcatcaagg 665700
tcggcaggtt gtcggtacaa ttcgtgaggg cttgcagttt ggaggatgcg cccctgtttc 665760
atcacggcaa tccggtcggc gtattgcagg gcttcttcgc ggtcgtggct gacgaaaacg 665820
gcagatttgc cgttggcgcg cagggcggca atcatgtctt cgcgaatctg gcggcgcaac 665880
tgttcgtcca gcgcgctgaa gggttcgtcc aacaaaatca gttcgggatc gggtgcgagg 665940
gcgcgggcga gggcgacgcg ctgttgctgt ccgcccgaaa gttcgtgcgg atagcgtccg 666000
gcaagttcgg aaatgccggt caattccaac atagcttcga tgcgctgccg ctcttgcgcc 666060
gtcttgcctt tgccgttgcc cagcccgtag gcggtgttgc ggtaaacggt caggtggggg 666120
aacagcacac cttcctgtac gacataaccc aaacggcgtt cgcggacggg gaggttggta 666180
tttttcgaga agatggttct gccggaaagc gaaatttcgc caaaatcggg ttgttcaaaa 666240
ccggcaaggc agcgtaaaag ggtggttttg ccgcagccgg acgcgccgac gataaagagg 666300
atttcgcccg ggtcgaggct gagcgaaatg tcgtttaaaa ctggggtgtt ttgaaaactt 666360
ttggacaggt gtccgatgtg cagggcggcg gtcatggcgg tacttcctca agctgttatt 666420
tgaaggcgta tttcttcagc aggaatacgg ggatgccgga aaataatacc agcatcagcg 666480
cgtaaggggt ggcggcggcg tattgtgcgt ccgatgtgta ttcccaaacg gcggtggaga 666540
gtgtgtggac atcgtcggtg gtcagcagca gggtggcggt cagctctttc atcagtttga 666600
ggaagacgag tgcgaatgcg gcggtaatgc cgggcaggat ggacggcagt accaacgtcc 666660
tgaaaataaa gaagtgtccg cgccccaatg ttgcgccgac ctgttccatc ccttttggga 666720
gttgttccaa ggaagtcctc agggtggttt gcgccatcgg caggtaaagc atgaaatagg 666780
caaggatgac gacgataaag gtttggtaaa cggcaggggt gtagttgatg ctgaaataaa 666840
ccaaggatag ggcgataacc aaaccgggga cggcgtgcag taaaaacggc agcctgtcta 666900
tccaaacggt taaaaaattg cgatagcgaa ccgatgccca aacaaggggc aaggcacata 666960
atatagtcaa aatcgcacct aaagccgata cgcttaagga acggataaag gcatcaaata 667020
cggatacgag cgcgaatgtg ccggaagtgc cgaccatcat ccaatgtatc aatacgccaa 667080
aggggataat aatgcccaaa gtcaacaagc tgcttaaaaa aacaatcgcg ccaatctgac 667140
cgggcagttt gagggttttg acgggataag gacgggcaac gcctttgccg ctgtggtaaa 667200
tcttggcttt gccgcgaaat atgctttctc caaatacgac gatgccgcac accgccatta 667260
aaacagcgga aagcagggcg gcggtattgt tgttgtagga catttcgtat tcttggaaaa 667320

tggcggtggt aaaagtgggg tagttcaaaa tggataccgc gccaaattcg accagcatat 667380
gcagggcaat cagtaacacg ctgctgccga tggcgggttt gagctggggg aggatggcgg 667440
aaaaaaaggt ttgcaggcgg cttttgccca aggacaggct gacttcttcg taagacaggc 667500
tgatgcgttt gagtgccgcc tcgacgggca ggtaggcgag cgggaacgag gacaggctca 667560
taatcatcac tgtcccccaa aagccttcga cacggaaggt caggctgatc caggtgaaac 667620
agctgacaaa tgcggggatg cacaaaggca gggtgattgc cgtctgaaaa aaggttttgc 667680
cgaagaagcg gtaacgttgg aacaaaaggg cgcaggcaat gcccaaaaca atggaaatca 667740
gggtaacgcc cgccatcatc gtcaaggtgt tggagagcaa atcccacata cgcgggcgga 667800
acaacagttc gacggcgcgg ttgatgccga cctgccacga acgcatagcg acatataaaa 667860
aaggcagggt aagcggtagg gcaatcagta ggatgaggcc ggtaagccaa atgggtattt 667920
ttttaggaga catagtgttt tttatcggca aaacgggcgg acagtataaa tgtccacccg 667980
tttgacaatc cgaaaacggc ttatttcata ccggcttgct caagcagccg ggtggcgtgt 668040
tctttttcgg aaacagtggt ggcggacact tggggtgctt ccaacttggc gatgggttcc 668100
aaattgaaag tggataccac gtgcggattc aaaggatatt cggcacggac ggcggtcagg 668160
gcgcgctgtc cttccttgct ggcgaggaag gcgacgaatt ttttcgcctc atccttgttt 668220
tgggaggatt ttaacacggc tgcgccggaa taggtaacga gtgcgccggg atctctgtgg 668280
cggacgaaat tcaggcgggt gtggacattt tgtacgcctt tttcacgcgc aaaagcgtgc 668340
cagtagtagt tgttgatgag ggcggcatcg atttcgccgt tttcaaccgc ttgaagggcg 668400
acggagtttt tagcgtaagg cttgccgtat tctttcagac ctttgagcca tttcaatgcg 668460
gccgcttcgc ctttcagttt gacgatggcg acaacctgtt ccaagaacgc gccggaagtg 668520
ggggcgtaac cgatgcggtt tttccatttc ggcgtggcgt aattcaggac ggatttttcc 668580
aaatcttttt cagacagttt gcgggtgtcg taaacgacga cgcgcgaacg tccgctcagt 668640
gccacccagt ctttttggc ggcaaccggc acgcccttgc cgcgtgtttc gttgatggtg 668700
gaggcgggca ggggctctag gaggttggcg gcggaaaggg tggcgagtgc cgggatttgt 668760
tcggaataga atacgtcggc ggggcttcgg ctgccttctt ctttgatttg accggcaagc 668820
tggtcgcctt tggcgctgtt gagtttgact ttgatgccgg tagcccgggt aaaggcatct 668880
gcaacggctt gtgctgcttc tttgtgttgg ccgttgtaca cggtaatgtc tgccagcgcg 668940
ggggttgcgg cggtcagggc tgcggcaagc agtgcgtatc ggatagatgt tttcatatcg 669000
attttctcct aatgaatgag agtgtatacc ttgttaagac ataacggtgt gtagtgtatt 669060
ccttcttttt tataaatgca aataattatt ttttaaattt gttgttgtcc gatccggtta 669120
ttgtttgttc tgacttgtat tttttccgtg agtctcgccc gtaaggcgga agtggcgggc 669180
aatgcgtggc ggaatgtggg taaaggcggc attttgattt gtcggaatgc ttgagaaccc 669240
ctctctttaa aacacccttg gattcggatt tcaagtgcaa cactagtgta ttagtggttg 669300
gaacagattc aagaataaaa cacttggcgt ttcgtagcca agtgtttttc ttggtcggtg 669360
gttcaactca tcttgaaccc tgcgtatctc ccgatcactg atgttacgga aatcggtttg 669420

tttggggaag tattgccgga tgagtccgtt ggtgttctca ttcagccctt tctcccaaga 669480
atggtaagga cgacaaaaat aagtctccgc tttcaatgct ttggttattt tggtgtgttg 669540
gtagaactct ttgccgttat ccatggtaat ggtgtgcacc ctgtctttat gtgcctttaa 669600
tgccctaaca gctgcccggg cagtgtcttc ggctttgagg ctatccaatt tgcagatgat 669660
ggtgtagcgg gtaacgcgtt cgaccaaggt caataatgcg cttttctgtc ctttgccgac 669720
aatggtgtcg gcttcccaat cgccgatacg ggatttctgg tcgacgatag cgggtcggtt 669780
ttctatgccg acacggttgg gtactttgcc tctggtccat gtgctgccgt agcgtttgcg 669840
gtagggtttg ctgcatattc tgagatgttg ccacaacgtg ctgccgttgc ttttgtcttg 669900
gcgaaggtag cggtaaatgg tgctgtggtg gagcgtgatc tggtggtgtt tgcacaggta 669960
ggcgcatact tgttcgggac tgagtttgcg gcggataagg gggtcgatgt gctgaatcag 670020
ctgcgaatcg agcttatagg gttgtcgctt acgctgtttg atagtccggc tttgccgctg 670080
ggctttttcg gcgctgtatt gctgcccttg ggtgcggtgc cgtctgattt cgcggctgat 670140
ggtgcttttg tggcggttca gctgtttggc gatttcggtg acggtgcagt ggcgggacag 670200
gtattggatg tggtatcgtt cgccttgggt cagttgcgtg tagctcatgg caatctttct 670260
tgcaggaaag gccgtatgct accgcatact ggcctttttc tgttagggaa agttgcactt 670320
caaatgcgaa tccgccaccg tctgaacagg gttgctggaa tagtattgcc atcccagcag 670380
atacagtttg tcggggtctt gccaatattg ttcatccaga ctgttcagca gtgaggcggt 670440
tttgttgtcg agatgttata tcccacattt ctttcaggtt tttaccttcc gattggaggc 670500
ggcggatttc tttgtccaat gcgtctgccg cccgatggat cagcattgcg ctgtgtaccg 670560
cgccgatttt tttcagaacg gaacaagtct tttcggcggc ggggaaaccc cagttgcaga 670620
caaattgcag tatgctgccg ttaccgatat cggcttctgt ccgccaaatc aaaaccagct 670680
cctgttcccg cccgtccatg ctttcgagtt tgccgtcatg ctgttcaaaa agtttgtcca 670740
ccgcctgcca catcatctgt tcgaagcggt cggcttgggt atccgtatcg gtcatcgtgt 670800
tcttgccttt taaaaatgcc gtctgaacat ttcttcagac ggcatttggg ggttaagcca 670860
acatttcccg ccagcgtttc acttggaagc ggacttgttc cggcgcggta ccgcccaagt 670920

ggttgcgggc gtttaagctg ccttcgggtg tcagcacgcc gtaaacgtcg tcggcaatca 670980
aatcgctgaa accttgtaag acttcgagcg gcagttcgct caaatcgacg cccgcttggt 671040
cggcgtggcg cacggcttgg gcgacgactt cgtgggcatc gcggaaaggc atgccttttt 671100
tgaccagata atccgccaag tcggtggcgg tagcgaagcc ctgcatcacg gcggcgcgca 671160
tattgtcggg tttgacggtt acgccgcgca tcatatcggc gtaaatccgc aacgtgtcga 671220
taagcgtgtc ggcggtgtcg aacaagggtt ctttgtcttc ctgattgtct ttgttgtacg 671280
ccaagggttg ggatttcatc agggtaatca gaccgataag gtgtccgatg acgcggccgg 671340
atttgccgcg cacgagttcg ggcacgtcgg ggtttttctt ctgcggcatg atggacgaac 671400
ctgtgcagaa acggtcggcg atgtcgataa agccgaaacg cgggctcatc cacaaaatca 671460
attcttcaga caggcggctc aggtgaacca taaccagcga ggcggcggct gtgaactcaa 671520
tggcgaaatc gcggtcggat acggcatcga gcgagttctg gcagatttgt tcaaagccca 671580
atagctcggc ggtgatttcg cgctgaatcg ggtaggtcgt cccggcaagg gcggctgcgc 671640
cgagcggcat acggttgacg cggcagcggc agtccgccat ccgttcgtta tcgcgtccga 671700
gcatttcgac gtaggcgagc atatggtgtc cgaagctgac gggctgggcg acttgcaggt 671760
gggtaaagcc tggcatgacg gtttcggcgt tttgttccgc caaatccagc aatgccgtct 671820
gaaggctttg aatcaggctt tgtataacgg taatctggtc gcgcagccac aggcggatgt 671880
cggtggcgac ttggtcgttg cggctgcggc cggtgtgcag gcgtttgccc gcgtcgccga 671940
ttttgtcggt caggcggcgt tcgatgttca tatggacatc ttccaaatcg gacgaccatt 672000
cgattttgcc gctgcggatt tcttcgagga tttccgccat accccggcgg atgtccgcca 672060
aatcgccttc gtccaacacg ccggtttctt tcagcatttg cgcgtgtgcc agcgagcctt 672120
ggatgtccca ttcggcaagc cgtcggtcga aaccgatgga ggcggtgtat tgtttgacga 672180
gttcggaaac gggttcgttg aaacgtccgg accaggtttt gtcgtgcata aggattcctt 672240
gatggggtta ttcggtgcgg tatttttcca aaagccggcg gaagggttcg ccggtttcgg 672300
gatgtttcag accgtaggcg acggtggctt cgaggtagcc cagtttgctg ccgcagtcgt 672360
agcgcgtacc ttcaaagggg tgcgccagga caaattcgtg atcgagcagc ttggcgatgc 672420
cgtctgtaag ctggatttcg ttgcccgcgc cgcgcggaag attggttaag aggtcgaaaa 672480
tgcgcggggt gaggatgtag cgtccaacaa cggcaaggtt ggagggcgcg tcttcgggct 672540
tgggtttttc gacaatgccg gtaatgcgtt ggaactgttt gagctgttcg gtttcgacga 672600
tgccgtatga gccggtttgc gatgcttcaa cggtttctac gcccaaaatg ctgttgccgc 672660
tgcgcccgta cacttcgacc atttgtttga gcgcgccttt gggggcatca atcaggtcgt 672720
cggcaaggat aacggcaaag ggttcgtctc cgatggcggc gcgggcgcac aagacggcgt 672780
gtcccaagcc cagtgcttcc gcctgacgga tgtagaggca ggtaatgttc ggcggcagga 672840
tgttgcggac gtgttccaac aatttgtctt tatggcgcat ttccaactcg gtttcgagtt 672900
cgtatgcctt gtcgaaatgg tcttcgatgc tgcgtttgtt gcgtccggta acaaacacca 672960
tttccgtgca gccggcttcc acggcttctt ctacggcgta ttggatcagc ggcttgtcga 673020
cgatgggcag catttctttc gggctggcct tggtggcggg caggaagcgg gttcccatcc 673080
ctgcgacggg gaaaacggct ttccgtatgg gtttcattct ttttcctttg tattgttttg 673140
atgtttaaag ggcgagtttg cgtaagagtt cggcaagtgc ctgcgcgcgg tggctttcgc 673200
ggtttttgac ctccgtatcc aattcggcgg cggttttgcc gtgttcgggc agataaaaat 673260
acgggtcgta accgaaaccg ttttgcccga gcggcgtgtc gttccactgc ccgtgccata 673320
cgccctcggc gataatcggg cgcgggtcgt ctttatggcg gacaaaaacc aatacgcaga 673380
catagcagca gcttttgtct gccttgccga caagttcggc ggcaagtttc aggttgttgg 673440
cggtatcgga tttgggattg tcgcccgcgt aacgtgcgga atggatgccc ggcgcgccgt 673500
ttaaggcggc ggcacagatg ccgctgtcgt cggcgagtgc gggcagcccg ctgtatttgg 673560
cggcatggcg tgcttttgcc agcgcgtttt cgacaaaggt gggatagggt tcggggcatt 673620
cgggtatgcc gaatgcggat tgcggcaata cggtgatgct gtaaggtttg aataagttgc 673680
cgaactcttc gagcttgccg gcattgccgc ttgccaaaac gatttttttcc ggtttttcag 673740
acatagcggt tttcctttgt ggcggattgg gcggcgcgta gggatttgtg ccgcaggtag 673800
agggcgaggc tgccgatttg tccgaacagt gcgccgaatg cgaaaagaaa ggaggcgacg 673860
gcgaaggctt tgctgccgac ggtcagcaga taagcaccca agaggacgag cagcataaat 673920
gccagtgtga agagggcaac agacaacaga tagatttttc ggcggttcat ggcgttcggt 673980
cggaaacggt atgttcggat tatagccgat tgggacggta ttccctagag cttggaaaaa 674040
tgccgtctga aacggcgttc agacggcatg gcgggcgtta tgcctgtttg ttccaacgtt 674100
cgatggattc tttgatgact tttttcgctt cttccgcatc gccccacgat tcgactttgg 674160
tcgtacctgc ttttttcagg tctttgtaat ggttgaagtg gaactcgatt tgtttgatga 674220
gctgttgcgg caaatcggac aaagttttgt aggcgttgcc gttgttgcgg tcgtcggcag 674280
gaacgcagac gattttgtcg tccacttcgc cgtcgtcaac gaatttcatc acgccgataa 674340
cgcgcgcttc caagaatacg ccggttgcca aaggttgttc ggtaacgagc agcacgtcca 674400
attcgtcgcc gtcttcgtcc aaagtttggg gaatgaagcc gtagttggtc ggtttggcga 674460
agatggcggg ttcgacgcgg tcgagttgga atgcggccag tttgcggttc cattcgattt 674520
tgtggttgct gccggcgggg atttcgttga caacgttgat gatgccgccg tccacgtcgc 674580

cgggggtcag gatttggttg aagtctgcca tttggtttcc tttgtttggg aaagtgtgaa 674640
gtttgaaagt atagcacaaa cgtccggctg aaaatgcgcc cgatgcctct gaaagggtgt 674700
acgggcgcgt gttaccgttt gcccaaaaac ctgcccagtt ccaaaatcgc gcgcctgttg 674760
gacggggaaa atactttttc cgctgcttct tccatatcga accaaccgta ggagacgtgt 674820
tcttcgggtt gcagggcgat gggcgtatca cgcgggattt cggcagagaa gaggtgttcg 674880
cggttttcaa acacgccttt tggatagcgg tgccgccagt ggtggtagat ttcgtaaacc 674940
gtgctgtcgt gccagtcttg aagctgcccg tccgccagca ggatgccggt ttcttcccaa 675000
acttcgcgcc ttgccgtttg ggcgacggtt tcgcccggtt cgaggctgcc ggttaccgac 675060
tgccaaaatc cttccggatg cgtgcgttcg atgagcagga tgccgccgtc cccgctataa 675120
aggacgacca gtgcggaaac ggggtatttg agcggttttg ccatcggcat ctttcggcgg 675180
gctgcggtaa tgaaggggcg gattatagca aacgccgcac gttatggcgt ttatcctttt 675240
ccgtatcctt ttttctgcac ggatgggacg cgccggtgtt tgccggtaaa ttttccgttg 675300
tgtcaaaaag ataagggcgg ttgtgatttt aatgcttgcc aaagcgtcgg gcggaaacta 675360
taatccgaaa cttatcgagt cggagtgtgg cgcagtctgg tagcgcactt gcatggggtg 675420
caaggggtcg aaggttcgaa tcctttcact ccgaccaaaa attccgaaag ccgctttcaa 675480
aagcggcttt tttgccgtcc gtatgattat gatgtagagt acgcggcgac agacattcaa 675540
atgccgtctg aaaaccgttc agatggcatc tctttatctt agtttcattc cgtaccatct 675600
taaggaacat caaattgggc atttcccgca aaatatccct tattctgtcc atactggcag 675660
tgtgcctgcc gatgcatgca cacgcctcag atttggcaaa cgattctttt atccggcagg 675720
ttctcgaccg tcagcatttc gaacccgacg ggaaatacca cctattcggc agcagggggg 675780
aacttgccga gcgcagcggc catatcggat tgggaaaaat acaaagccat cagttgggca 675840
acctgatgat tcaacaggcg gccattaaag gaaatatcgg ctacattgtc cgcttttccg 675900
atcacgggca cgaagtccat tccccttcg acaaccatgc ctcacattcc gattctgatg 675960
aagccggtag tcccgttgac ggatttagcc tttaccgcat ccattgggac ggatacgaac 676020
accatcccgc cgacggctat gacgggccac agggcggcgg ctatcccgct cccaaaggcg 676080
cgagggatat atacagctac gacataaaag gcgttgccca aaatatccgc ctcaacctga 676140
ccgacaaccg cagcaccgga caacggcttg ccgaccgttt ccacaatgcc ggtagtatgc 676200
tgacgcaagg agtaggcgac ggattcaaac gcgccacccg atacagcccc gagctggaca 676260
gatcgggcaa tgccgccgaa gccttcaacg gcactgcaga tatcgttaaa aacatcatcg 676320
gcgcggcagg agaaattgtc ggcgcaggcg atgccgtgca gggcataagc gaaggctcaa 676380
acattgctgt catgcacggc ttgggtctgc tttccaccga aaacaagatg gcgcgcatca 676440
acgatttggc agatatggcg caactcaaag actatgccgc agcagccatc cgcgattggg 676500
cagtccaaaa ccccaatgcc gcacaaggca tagaagccgt cagcaatatc tttatggcag 676560
ccatccccat caaagggatt ggagctgttc ggggaaaata cggcttgggc ggcatcacgg 676620
cacatcctat caagcggtcg cagatgggcg cgatcgcatt gccgaaaggg aaatccgccg 676680
tcagcgacaa ttttgccgat gcggcatacg ccaaataccc gtccccttac cattcccgaa 676740
atatccgttc aaacttggag cagcgttacg gcaaagaaaa catcacctcc tcaaccgtgc 676800
cgccgtcaaa cggcaaaaat gtcaaactgg cagaccaacg ccacccgaag acaggcgtac 676860
cgtttgacgg taaagggttt ccgaattttg agaagcacgt gaaatatgat acgaagctcg 676920
atattcaaga attatcgggg ggcggtatac ctaaggctaa gcctgtgttt gatgcgaaac 676980
cgagatggga ggttgatagg aagcttaata aattgacaac tcgtgagcag gtggagaaaa 677040
atgttcagga aataaggaac ggtaatataa acagtaactt tagccaacat gctcaactag 677100
agagggaaat taataaacta aaatctgccg atgaaattaa ttttgcagat ggaatgggaa 677160
aatttaccga tagcatgaat gacaaggctt ttagtaggct tgtgaaatca gttaaagaga 677220
atggcttcac aaatccagtt gtggagtacg ttgaaataaa tggaaaagca tatatcgtaa 677280
gaggaaataa trgggttttt gctgcagaat accttggcag gatacatgaa ttaaaattta 677340
aaaaagttga ctttcctgtt cctaatacta gttggaaaaa tcctactgat gtcttgaatg 677400
aatcaggtaa tgttaagaga cctcgttata ggagtaaata aaaatggcaa tttggaatta 677460
tcggtactac cttatcccat cagctgctat cagaaataaa tttaatgcaa ataaagatat 677520
catacttgat gagtatcgat ctaatggttt tcagaatttt aatgagaata aaagttttga 677580
aaattacttt atcgataatg atgttatatt attatcaata ataaatgaag caaaaaaaca 677640
gcttaaattg aaagaatctt gggataaaga cgcaatcatg ttttgtgata attttggtaa 677700
tagtcttacc gtttggccag atgatataga gtgcgaactt gatttaagat ttgattatac 677760
taaatttatt cagaaaacca ttgattgggc aataaaatat aattgtctac ttgtaataga 677820
aaaaacagga aatgtagttt cccctaatat aaataatctg atgtatgaaa taaaagcata 677880
tttggaaagc aagccgtggc ccatatgaaa cctaaactca acaagtagga tgtgtgcgga 677940
acgcacgtat gcggttctca aggtttgagc taagaggccg tctgaaaaca gaaaaactgt 678000
ttcagacgac ctttctttta accagttgcc acagcaaccg gacaaaagca gcctacctcc 678060
acatccatat aggcaataca ggggagatat tttgtaaatt ctacgaatat tttacctgct 678120
aaacagggta ggatatggta tgaagcgaac attggcttaa taaacactat gtcaagatcg 678180
aatcaggctg gtactagatt gttgtattcc aattatggat tgctatatat aacaactgat 678240

cattatatct ctgcaacacg gtttgtagct tggaaatagg agtataactt atgcaattag 678300
agattatcgg tagtaaaatt tatacggaac aagattttca taatcaaatt tcaaaaatat 678360
tttctataca agattattat gggaacaatc ttgatgcttt atgggattta ttaagcacaa 678420
atgtagaacg accgattact ttggtatgga aagatgctat gttctcaaaa aatcaattag 678480
aaaatatatt tattgaaatc gtaaatgttc tagaaagagt taagaaacaa gatgaggatt 678540
atggattcga agaaaaattt aattatattt tagagtaagt aaaccctaat tacattacgt 678600
acacaggctt aaaactcccc agagccaatt aagcaagccg taacccatat aaaacttaaa 678660
ctcaacaagt agcatgtgtg cggaacgtac gcatgcggtt cttaaagttt gagctaagag 678720
gccgtctaaa aacagaaaaa ccgtttcaga cggtctttgt ttaacgccac cgatccagcg 678780
ggttacaaag cgcagtcaat gccgctgcgc cttatgcctc cgaagcaata ggcagaacat 678840
ttggacacgg tgaaaacaaa aacgaaaccg cccaagccgt cggacatttc cttttaggag 678900
cagctattgc ccgcgtcaac ggtggtaatt ttgctgccgg cggctcggca gcagttgcag 678960
ctgaaaaggc ggcggaacat cttgccccaac agtataacga cggtaaaacc gcaatcgatc 679020
cgcaaacagg cgagttcaat gccaacctgc tgccggaaca tatcaaagag gaaatcaaat 679080
caaagagcgg ggtgattgca tcgctgacgg gcgcggccgt gggcggcacg ccggtagatg 679140
cgcaaaccgg aggtgcggtc ggacagaatg cggtggaaaa caacctctat ctgacatcgg 679200
aagccttaaa gaaggacaag cagacagctc gtaaaattta ttccgtcata aaagagcaag 679260
tcaagcatga atgcagttcc acaggaagaa ttaccgaatg tcgtcaaaat ataggacgca 679320
ttatcgaatt tacccaagac aaacgctttg acagtaggtt taaggactta aaaaaagaat 679380
ccttatatta cctaaataaa catcctgatt tagtagcctc ttatttgaag gctgaatacg 679440
aaaagctgga tagggaagac aaaagtatcc tgcaccgcta catctcaccc ggggctgaaa 679500
tcgtttcggg cagtttgggg gttgttcttt caggagtagc cggaggcgga tcttgtgccg 679560
agactttcgg cttaggctgt gccgccgctt tggttggtgt aacgtcttcc tacgatcatg 679620
tcattactgg gacgaaaaac ttcggaaaaa aagccagcga gcaacgaccg acgattgcgg 679680
ttcaggcctt gaagcagttg gggctgtcgg agcaggctgc ggaatatgtt cagttctcta 679740
tagatttgtt cagtgtgggt aaatcggggg gcggtatacc taaggctaag cctgtgtttg 679800
atgcgaaacc gagatgggag gttgatagga agcttaataa attgacaact cgtgagcagg 679860
tggagaaaaa tgttcaggaa acgagaagaa ggagtcagag tagtcagttt aaagcccatg 679920
cgcaacgaga atgggaaaat aaaacagggt tagattttaa tcattttata ggtggtgata 679980
tcaataagaa aggcacagta acaggagggc atagtctaac ccgtggtgat gtacgggtga 680040
tacaacaaac ctcggcacct gataaacatg ggtttatca agcgacagtg gaaattaaaa 680100
agcctgatgg aagttgggag gtgaaaacga aaaaaggtgg gaaagtgatg accaagcaca 680160
ccatgttccc aaaagattgg gatgaggcta gaattagggc tgaagttact tcggcttggg 680220
aaagtagaat aatgcttaag gataataaat ggcagggtac aagtaaatcg ggtattaaaa 680280
tagaaggatt taccgaacct aatagaacag catatcccat ttatgaatag taatatttat 680340
gaaaaattag gagattaatg atgaaaagaa ttaagtgctt ttgtgataaa tttccatcag 680400
gagatacatt tagaatgtgt atcattctgg atgactatga taatagggtt gattattatg 680460
taggaatata tgattacatt acgtctacct taatgagcga tatttactat cgatccacga 680520
ttgatgagca tttcaagatt atagaattaa tagaaaataa tccaaatgaa atttatgatg 680580
atggcggtgg tcaacaattt tgcctagaat ttcatcatga taaggtcatt ttttaccaca 680640
atgaatttga tgaagaagat ggttatccag tattaagctg ttcgctgcat acttttaaaa 680700
ctgctttaat tgcttggaat gctttttttgc aattgcctaa aagtattcat tcggtggtgg 680760
agactgtgat tgaggaataa gcataattag cttaatgaat agaatcagcg atatagattg 680820
gactgcaaat ccacgcttat acgctgtgcc atgattaaga tgttagaact tgtattgaat 680880
acaagttctc ataaacgaat ggcagtaagc atttgattta gataaaatcc ttgaattaga 680940
ataatcaggt ctaagagctc gacaggacaa atgaggctgg caaccaagga tttggcggaa 681000
gccattagga aaggacaggt tcgcaaatca agctttaaca cagaacaatt aagggcaatt 681060
gaaaaaggag aatctaaaat accggattac acttggcatc atcatcaaga tacaggaagg 681120
atgcaattga ttcgtgaagg cttgcatcat gataccggcc atattggttg ggaagcaatg 681180
aacaaaggaa ggtaactatg tggaaaatca taaaagagga tagtgatgat ttagaatttg 681240
caattaaatg cttattctct cagtctattg atttaaatga attcaagtta tggattgaac 681300
aagtaatacg cgatatgccc atcgaggaca tcccttttta tatttttgat ttggcggatt 681360
ttgatggggg aattgccgat attgacaata ttgtaggttt tgtttcaagt tgcagactat 681420
caaaatcgaa aaaaaatgcc ttgaccggca ttgccttctt aaggggggata gatgtctatg 681480
atccgcctat ttcaaaagaa aaagcattaa aagccttaga gaaacatcct gaaatttatc 681540
agaaatttca gcatttcttt ccgtttgtag aactgccccc gctttaaaca gtcaaaatgc 681600
cgtctgaaac gatattcggc tttcagacgg tatttttgat ataaagcggg taactaaaag 681660
agcgtttgac ggcaaaggaa gataattatg tggaaaatca taaaagagga tagtgatgat 681720
ttaggatttg caattaaatg cttattctct cagtctattg atttaaatga attcaagtta 681780
tggattgaac aagtaatacg cgatatgccc atcgaggaca tcccttttta tatttttgat 681840
ttggcggatt ttgatggggg aattgccgat attgacaata ttgtaggttt tgtttcaagt 681900

```
tgcagactat caaaatcgaa aaaaaatgcc ttgaccggca ttgccttctt aagggggata 681960
gatgtctatg atccgcctat ttcaaaagaa aaagcattaa aagccttaga gaaacatcct 682020
gaaatttatc agaaatttca gcatttcttt ccgtttgtag aactgccccc gctttaaaca 682080
gtcaaaatgc cgtctgaaag ccatttccgc cgctcagacg gcattttcgc cccttttgtt 682140
tacaaaccct taaaatccct ttacactcaa aatccgttca acatcaaaca aaccccgcta 682200
tgaaaaccct gctcctcctc atccccctcg tcctcacagc ctgcggcaca ctgaccggca 682260
tacccgccca cggcggcggc aaacgctttg ccgtcgaaca agaactcgtc gccgcatcgt 682320
cccgcgccgc cgtcaaagaa atggatttgt ccgccctaaa aggacgcaaa gccgcccttt 682380
acgtctccgt tatgggcgac caaggttcgg gcaacataag cggcggacgc tactctatcg 682440
acgcactgat acgcggcggc taccacaaca accccgaaag tgccacccaa tacagctacc 682500
ccgcctacga cactaccgcc accaccaaat ccgacgcgct ctccagcgta accacttcca 682560
catcgctttt gaacgccccc gccgccgccc tgacgaaaaa cagcggacgc aaaggcgaac 682620
gctccgccgg actgtccgtc aacggcacgg gcgactaccg caacgaaacc ctgctcgcca 682680
acccccgcga cgtttccttc ctgaccaacc tcatccaaac cgtcttctac ctgcgcggca 682740
tcgaagtcgt accgcccgaa tacgccgaca ccgacgtatt cgtaaccgtc gacgtattcg 682800
gcaccgtccg cagccgtacc gaactgcacc tctacaacgc cgaaaccctt aaagcccaaa 682860
ccaagctcga atatttcgcc gttgaccgcg acagccggaa actgctgatt acccctaaaa 682920
ccgccgccta cgaatcccaa taccaagaac aatacgccct ttggaccggc ccttacaaag 682980
tcagcaaaac cgtcaaagcc tcagaccgcc tgatggtcga tttctccgac attacccccct 683040
acggcgacac aaccgcccaa aaccgtcccg acttcaaaca aaacaacggt aaaaaacccg 683100
atgtcggcaa cgaagtcatc cgccgccgca aaggaggata aaccgtgaaa ccgctgcgca 683160
gactgacaaa cctccttgcc gcctgcgccg tagcggcggc cgcactcata cagcccgccc 683220
tcgcggcgga cttggcgcaa gacccgttca ttaccgataa cgcccaacgg cagcactacg 683280
aacccggcgg caaataccac ctcttcggcg acccgcgcgg cagcgtttcc gaccgcaccg 683340
gcaaaatcaa cgtcatccaa gactataccc accagatggg caacctgctc atccaacagg 683400
caaacatcaa cggcacaatc ggctaccaca cccgcttttc cggacacgga cacgaagaac 683460
acgccccctt cgacaaccac gccgccgaca gcgcgagcga agaaaaaggc aacgttgacg 683520
aaggctttac cgtataccgg ctcaactggg aaggacacga acatcatccc gccgatgcct 683580
acgacggccc gaagggcggc aattacccca aacctacggg cgcacgagac gaatacacct 683640
atcacgtcaa cggcacagcc cgcagtatca aactcaatcc gaccgacacc cgcagcatcc 683700
ggcaacgcat atccgacaat tacagcaacc tcggcagcaa tttctccgac cgcgccgatg 683760
aagccaacag aaaaatgttc gagcacaatg ccaagctcga ccgctggggc aacagcatgg 683820
agtttatcaa cggcgtcgcc gccggcgcgc tcaacccctt tatcagcgcg ggcgaagccg 683880
ttgaccagtg gatgcaggaa aaccccaatg ccgccgaaac cgtcgaagcc ctggtcaacg 683940
tcctgccgtt tgccaaagtc aaaaacctga caaaggcggc aaaaccgggg aaggctgcgg 684000
ttagtgggga tttctcagac tcctacaagc ataacactgc ttcaagatta tctcagtctg 684060
tagatggaga aatgtttcaa acccgcaatg ttgattttaa agcaaaatct attgggacta 684120
aaattcatga tggagctcaa gggaaacata tttcaggaca tagaaactac attgaaggta 684180
agagtacttt aaatcaaaac attaatcctc aagaattgtt gaacggaata cattcaggtg 684240
cttatccagt tatttctaaa ggagcaagag gaaatcctgt tgttgatttt gggtatccta 684300
taggcagcga tgggaaatca ggattaagta ccaattttgg tacgattcat tcaggtaaaa 684360
atggagttca cattgttccg gctaacccta aaaccattaa aaaggtgcaa tagttatgaa 684420
tatattacca agctggctgc gagtcggtat gaatatagca atgctgggca tgatacactc 684480
agatatcagg ttaattaccg tagattacga ggaaggaaga aggtttttaa aaatcaaaaa 684540
ttatttatca agagaagcca tcacagaaga ccatgaagat atggaatatt tgattacaga 684600
gttatggtct atgtgtggag aatattttga tgaagctgac tttgaatgta tttattctaa 684660
tcattcttct atggagttaa accaaataaa tggtgcagta ttcaggagaa aggaattaat 684720
ttcgcaagcg taggttaaaa aaaccaacaa tcacaatgtc ttctgaaacc gtgtttaatt 684780
ttcagacggc atttccttca tttgaaatag gatattgaga actgagttct tcaaaaatcc 684840
tacacctgct ccttccacgg cagcaccttg gtcaaaacgg cagacggcta caaagccatt 684900
gcccgtatcc gaaccggcga ccgcgtcttc gccaaggacg aggcaagcgg aaaaacggga 684960
tacaaacccg ttaccgcccg atacggcaat ccgtatcaag aaaccgttta cattgaaatt 685020
tcagacggca tcggcaacaa ccaaaccctg atttccaata aaatccaccc gttttacagt 685080
caaggaaaat ggatacaggc aggtcgtctg aaaaaaggcg acaccctgct ttccgaaagc 685140
ggcgcaaaac agacggttca aaacattacc ttcaaacagc agccgctcaa agcctacaat 685200
ctgaccgtcg ccgattggca tacctacttc gtcaagggca gtcaggcgga aacggaaggg 685260
gtttgggttc ataatgattg tccgtatgat aaaggcaacc aacgatataa agacgcttct 685320
tatcatggca aaaatgataa ttctgtgaaa agtagagcac caacaaacgg acaagcagct 685380
cttgataatt ccgttcaagt taaatcaact tctcctcgaa gagttggggt tgataaagcc 685440
aataatgaaa tcgttgtatt aaacaaaact caaactttta ataacggttc tgcggaatat 685500
cacgggcatg tcagaagttg gcaagatttg cataccgatc agaaaaatgc tttaaaaaaa 685560
```

gcaggattgg attagttaat tcaaaaggaa aaattaaaaa atgactgata aaagtaaaac 685620
agaaaagttg atttcttctg atgataaaca aagtgttata gatggcattc ttgatatggt 685680
atttaattcc aaagcatatg aagtaccgtg gatttctgag aaattgatgg aattatcgaa 685740
aaataaagac ttggatattg ccggattatc gctaacctgt ttcggacatc tcgccaggct 685800
acattcaaat atcggtgatt acgataaagt tattccttta ctacattcaa agcaagatga 685860
tccagagctt caaggtaggg ctgaagatgc gttagaagat atttctttat ttttatctga 685920
aaatcattag gaaccgtagg tcgggttgaa aacccaacaa tcaaaatgcc gtctgaaacc 685980
gtgtttaatt ttcagacggc atttctttca tttgaaatag gatattgaga actgagttct 686040
tcaaaaatcc tacacttgct ccttccacgg cagcaccttg gtcaaaacgg cagacggctg 686100
aaaagcaaac accgtccgtc gtgttgccgt ttgcggatga gtacgggtca accccaatgc 686160
cgccgaaacc gtcgaagccg ccttcaacat tgccgccgcc aaagccgcaa agttggcaaa 686220
aacggtaaaa ccggggagat aaaagccgat ggcaggaaag taaatgtgag gatagacagt 686280
acggaggcag acctgcttta tccggcaggg caataagaaa acaaaaatta gatatggaaa 686340
acgattgtga agattaaacc attacaattt tctaacaata atcacagatt ttatgtggac 686400
aaaatttgtg ttaccgaaca agatgtgaac attttggcat ccgaccgaaa ttatgaatta 686460
aatattgaaa tatttattga caatataatt cattttcaaa taacggatga atcttataaa 686520
gtaaaatttt cagaatattt atttgaaaat aaagaaaaaa atgattggga tagaaatcct 686580
gctataaatt attttttcga gataatagat gatagttata tggactggtt gaaagaagaa 686640
agttttgatt tttttgaaaa gaaatattat aaggcttata ttttcttttt tagcgattct 686700
gtaatagaag ttatcagctc gacagaacct gtattttatt caaaataaca aattatcaaa 686760
caaagctctg attaaaaacc caacaatcaa aataccgtct gaaacgatat tcggctttca 686820
gacggtattt ttgacacaaa gcaggtaacc aaaggagtgt ttgacggaaa aggagaagct 686880
aaaataccgg atgtatcggt tgggaagcaa tggataaagg taaataatta tgtggaaaat 686940
tagtaaagaa aattgtgaag atttaggatt tgcaatagtc tgtatgttct atgatgctat 687000
taatctttct gaatttaaat tatggttgga tatagttgtc agagatattc ctattgatac 687060
aattccattg tatatttttg atttgattga ttttgataag agtatagggg aaatttatga 687120
tgtaattgga gtcgttaatt atggttacat ttcaaatgat caaaaaaatg cattaacggg 687180
cattgccttc ttaaggggga tagatgtcta tgatccgcct atttcaaaag aaaaagcatt 687240
aaaagcctta gagaaatatc ctgaaattta tcagaggttt cagcatttct ttccttttgt 687300
agagcttccg ctttttttaaa agacaatatg ccgtctgaaa agttttcaga cggcattttt 687360
tatttcttcc agtaggcggg ggtgaagagg atgaagacgg tgaagatttc cagcctgccc 687420
aagagcatgg cggtaacgca gatccatttc tgcatcacgt ccaaaccggc gtaattgccg 687480
gcgggcccga cttcgcccag gccggggccg gcgttggtga tgcaggcgat gacggcggtg 687540
aaggcggtgg taaattccat accgctcgcc atcagcagga agctgaagag gacgacggtc 687600
ataaagtaga tgaagatgaa ggacataacg gtcagcgcga ggcggtcggg tatggccttg 687660
ccgctgattt tgacggtgcg gacggctttg ggtgcagca gcaccatcat ttcgcgcagg 687720
ctgaatttga acaggacgag ggcgcgtatg gttttgatgc cgccgccggt cgagccggag 687780
ttggcgagga tgttggcgag gaaaaacatc cacagggaaa tcaggagcgg ccattgtgcg 687840
aagtcggtgt tggccagccc gtttgccagt ccgatggaga cgaagttgaa ggcggtgtag 687900
cgcagggatt cggtaaaacc ggcgtaatag ccggtgtgcc acaggtacag ggcggcggca 687960
aggatgctgc cggagagcag cagcagcatc gtccggcatt cttcgtcttt ccaataggtt 688020
ttgaggctgc ggctgttgag ggcggcgaaa tggttggcaa aattgatgcc gccgacaatg 688080
gtgaaaacga tgatgaccgc ttcgatgagg ggggagttgt aataagctat gctggcatcg 688140
tgggtggaaa acccgcccag cgagagggta gccatcgcgt gacagacggc atcgaaccag 688200
cccatcccgg caaaatgcag gcaggctgcc gcgaggatgg tgatcagggt gtagccgaac 688260
caaagttttt tcgccacttg ggaaatgcgc ggcgacattt tgctttcttt gtcaatgccg 688320
gggatttcgg ctttgaataa ctgcgtgccg cctacgccga gcataggcag gatggcgacg 688380
gcaaggacga tgatgcccat cccgcccagc cagttgagca tatgccgcca aaagttgacg 688440
gagggggcga gcccgtcgac gtgggggatg acggtcgcgc cggtggtggt cagtcccgac 688500
atcgattcaa aaaatgcgtc ggtaaagccc atattcggga aatacaggta catcggcatc 688560
gcagccatag cggcaaacgc cagccacaac atcaggacga gggtaaagcc gtcgcgcggg 688620
cgcagttcgc gcctgaaccg gagggtggcg agccggacga tgcacgagcc ggaaagggta 688680
acggtcgcgg tggtggcgaa ggcggtgtac gcgccgtccg aaaaggcgta ggagagggcg 688740
gcgggtatca gcaggataaa ggaaaacagc atacccagtc gggagaggac gtgggcgatg 688800
ggcaggattt tgtgcatagt ggggcggtcc gttattttgc gaagcttttc cagtctatgc 688860
cgccggcggc ttggacttgg gtaatttctt ccgtttcgag gttgacggcg gtcagctgtc 688920
cgcccccacag cgcgccggtg tccagcgaga tgacgttgtc ggcattcgtg tagcccagcg 688980
aggaccagtg tccgaagatg atgtgcgtcg aggttttgcc ggtcgggggc tttgaaccac 689040
gggcgcaggt aaggcggcat ttttttcact gtggatttgt agtcgaaatc cagttcgttt 689100
ttaaaggtca gggcgcgcat ccgcgtgaag gcgttgacga tgaagcgcag gcgggcatag 689160
cctttcaaac cttcgtccca cgcggccggt ttgttgccgt acattttgga gaagaatttg 689220

```
acgtattttt tgccgcgcag ttcggcttcg gcttctctag cgagcgattc ggctttggtt 689280
atgcgccatt gcggcaggat gccggcgtgt accatcacgc ggctgccctc gcgtatcaac 689340
agcggttgcg cacgcagcca gtcgagcatt ttttttccgt cggggtgttt gagtatgggt 689400
tcgattgtgt cgctgcgttt gggcgcacct tcgccgcagc cgacagcgag caggtgcagg 689460
tcgtggttgc cgaggacgat ttgcacgctg ttttcgtgcc ggatgcagaa ttgcagcgtt 689520
tcgagggatt tcgggccgcg gttgacgatg tcgcccgtca gccagagggt gtccgtgccg 689580
tggttgaaac cgattttgcc gagcagcgcg gtcagttcgt cgaaacagcc ttgtatgtcg 689640
ccgattgcgt aatgtgccat tgcagatgtt gtgaagtggg aaagtgttgc ggttcggacg 689700
gcatggtttt gaaatatcat gcagtccgaa cgtggaatta tgcgttcaaa acgaggacgg 689760
cttcggcttc gacctgcacg cctttgggca gcgaggcaac gccgacggcg gcgcgggcgg 689820
ggaagggctc ggcgataaat tccgccatga cttcgttgaa gacggcaaaa ttgcccaagt 689880
cggtcaggta ggcgttgagt ttgacgatgt cggccagcgt gccgcctgcc gcttcggcga 689940
cggcttgcag gttttggaac acttggcgcg cttcggcgcg gaaatcgccg ttgccgacga 690000
cggtcatcgt ggcgggatcg agggggattt gaccgctcat gtaaacggtg tcgcctgctc 690060
ggacggcttg gctgtacgcg ccgatggcgg cgggggcttt gtcggtgtgg atgatggttt 690120
tggacatttc ggattcctca aaaaataggg cggcagaagc cgcagcattc gggattatcg 690180
tacaaaaccg ccggcttgtg tagttgcggt ggcagaaaac aaaaccgccg aaggctcggc 690240
ggtttgcaga ataaggcgca tatcagaatt tgacgcgcac accggcggac agttcgccgg 690300
aacggacgtt tttgacagtg ttgactttgc cgatgtagtt gtagcggtag ccggcatcca 690360
aatcgacatt cggggtaacg gcatagctta cgcccgtcaa tacgccgagg ccgatggagg 690420
tttggctgaa gctgtcgctg ccgcccaagt cgacggaggc gcggttgagg ctcaagcgcg 690480
cgccgagata cggtttgacg ggcgattggg tgtcgaagtc gtaaatggcg gacgcgccga 690540
tgctgtaaag tttgaaatcg gtggatgggg ctttatagtt tttgtagcgc gtgtaatcga 690600
cggcgaagcg gaggtcgttg atgcggtagc ctgcggagat gcgcgggctg aagcctttgg 690660
cagaacctaa agagcttgag gcttttgcgt gtgcggcatc ggcttggacg taaaagccgg 690720
atgcgccttc cgccagtgcg gcggccggga gagcgagggc aatcagtgtg gcaagtgctt 690780
ttttcatatt ttggttcctt tatggtcagt tagaaaaatt gttaagaatc cgttaaagaa 690840
tcctgctgta ttatacttaa attttctttt tgcatcgtaa tattttcaat acttcaagat 690900
acgtagcggt atccggctgc tttgccgacg gcaaagccgt taacccgcgc gttgccttta 690960
aatggtggcg gcggcatcac gcggcggatg ggtgaaactt gcaaacggtt tggaaaaaac 691020
agcggtatct gtcggattgt tgcaggtgca ggcatacggt tttgtgtgcg tctgtgcctt 691080
aagcgtcgga catttccggc ggcggctgtg ccgtctgaaa cgcccggcgg gggatgcggc 691140
tgcgttttcc atcgataagc atattttccg gacgcgttcg gggcgggttt tcccgggcgg 691200
ccgccgattt gtttgcgctt atatagtgga ttaacaaaaa tcaggacaag gcgacgaagc 691260
cgcagacagt acaaatagta cggaaccgat tcacttggtg cttcagcacc ttagagaatc 691320
gttctctttg agctaaggcg aggcaacgcc gtactggttt ttgttaatcc actataaaaa 691380
acactgcagc aaatcgttta aaaacaagcg tccttttttcg gtcgggcgga atacggcagg 691440
gtcggtttcc agcaggcctt tttgccttgc cgtttcgatt tgcgccatga ttttggcact 691500
cggtacgccc gtgcgctcct gcaacatcgc ggcgggtacg ccgtcggtca ggcgcagggc 691560
gttcatcatg aattcgaacg gcaaatcttc ggcagcgacg gttttgcgtt cgacggcttc 691620
actcggttgg ctttgcatta aggcgaggta gtcgttgggg tggcggcggc ggacggtgcg 691680
ctcgatgcgg tcgggatagg aaattttgcc gtgcgcgccc gcgcctatgc ctaaataatc 691740
gccgaactgc cagtagttca aattgtggcg gcactgcatg gctggtttcg caaaagccga 691800
tgtttcgtag tggacaaaac ccgcgccttc cagcgcgccg tgtaccgcgt cttcgatgtc 691860
gagggcggct tcgtcttgcg gcaaaccttt cggcggcgta tgaccgaacg gcgtgttcgg 691920
ttccatcgtc aggtgatacg cgctgatgtg ggttgcgccc gtagcgatag cggtttgtac 691980
gtcgtccaat gccgtctgaa cggtttggtt cggcagggca tacatcaagt cgatattgac 692040
tttatcaaat aatttcaagg cggtatcgat agcggttaag gcttccttac cgttgtggac 692100
gcgccccagc cttgagagca tatcgtcgtt gaaactctgt acgccgatag aaagccgcgt 692160
aatacccgcg tctttaaatc cttgaaactt ctcgatttca aatgtccccg gattggcttc 692220
caaggtaatt tccgcttccg gctgcaagcg caacagcgaa cgcacgccgc ttaacaaacg 692280
gtcaatcgat tccgcctgaa acaggctggg cgtaccgccg ccgaaaaaga tcgtttccac 692340
cggcctgccc caaatattgg gcaattcaag ctgcaaatcg gtcagcagcg cgtcgatata 692400
ggcggcttcg ggcaatccgt ttttcaggct gtgggaattg aagtcgcaat acgggcattt 692460
tttgatgcac cacgggatgt ggatgtaaag cgacaggggc ggcagggcgg tgagtcgggt 692520
gcggtttgga aaggaaatgg tgtgcatggt gtggttcgga aaagtgggca atgccgtctg 692580
aaggcggttc agacggcatg ggttcagccg agcagggtaa gcagttcggc ttcgctgagg 692640
acggaaacgc ccaaggcatt ggctttttcc agcttgctgc ccgcggcttc tccggcgacg 692700
acgtaatcgg tttttttgga cacgctgccg gaaactttgc cgcctgcggc ttcgattagg 692760
gattgggctt ggtcgcgttt gagggtgggc agggtgccgg ttaacacgaa ggttttgccc 692820
gccacggctt tattgatgcc gtctgaacct tgcgccgcct cgtcttcaga cggcatttgc 692880
```

gcgaagaagg ttttcaggtt ttcgagcagg gcggcgtttt gtggttcgct gcgccacgcc 692940
tgccagtcgg tggggagggc tttgtcggtt tgcagccctt ctatgttttt gccggcgagt 693000
tcccataagg cttgggcttt gttttcgctg attttgaaac cgggcaggcg ggcgatccag 693060
cgttgcggtt cggcgtggcg ggcgggcggg atggtaacgg cttgggtttg cggggcaacg 693120
cctgcggcga gcagttcgtc tatcatcgcc tgctgttcgg cttgggcgaa gaagtgggca 693180
atggaacgcg ccactaccgt accgatgtcg ggcaggcagg cgaggacggg ttcgggggcg 693240
cggcggacgc gttccaatgt gccgaatgcc tgtgccagcg ttttggcggt gcgttcgccg 693300
acgtggcgga tgccgagcgc gaacaggaag cgggcgagtt cgggcgtttt gctggcttct 693360
atgcctgcga ggatgttttc cgcccacttg actggttgtt ttttatgttt gcccgacgcg 693420
ccgaccgaac tgccttcaga cggcatttga tccgattcgg caacggtttt gtccgctgtt 693480
tccttcattt tttgcaaggt cgggatgtcg aggcggtaga gatcggcgaa gtggcggacg 693540
aggtcttgcg cgacaagctg ttcgatttgt ttttcaccca agccgtcgat gtccatcgct 693600
ttgcgcgagg cgaagtggat taagccttgc gcgcgttgtg cctgacaaag cataccgccg 693660
ctgcatcggg cgacggcttc gccttcttcg cgttcgattt cgctgcggca gatggggcag 693720
tgggtcggca ggcggtaggg cttgtggagc ggaacggatt gggtttgatt ggcggacggt 693780
gtttcggcaa acaaatcgtc ctgccgatgc ccgatgccgt ctgaaacggc aacggcggtt 693840
tcccgcatcg ggcggcgttc aaaaatcacg cgcacaactt cgggaatcac gtctccggca 693900
cggcgtacga cgacggtatc gccgacgcga acgtctttgc gcgatacttc gtcctgattg 693960
tgcagggtgg cgttggtaac agttacgcca ccgacgaata cgggctgtaa tcgggcaacc 694020
ggcgttaccg cacccgtcct gccgatttgc acgtcaatcg cttcgacaat ggtcagggct 694080
tcttcggcag ggaatttgtg ggcaaccgcc caacgcggcg cgcgggagat gaagccgagt 694140
tcgtgctgtt gcgccaagct gttgactttg acgaccatgc cgtcgatttc gtagggcagt 694200
tcggggcgtt tttgctgcat gtgttcgtaa aacgccaata cttcgtcgat atttttgaaa 694260
cagccgaaat tgccattggg cagactgaag ccgagtgctt ggaaataggc gagttcctgg 694320
atgtgttctt ccgcgacgaa accatcttgc tggcgggcga cggagtaggg gaaaaagtgc 694380
agtttgcgtt gcgcggtgat gcgcgaatcg agttggcgta ggctgccggc ggcggcgttg 694440
cgcggattgg caaagggttt ttgcccgttt tcggcttgtc ttttattgag ggcgacaaaa 694500
tcggctttga gcatcagcac ttcgccgcgt acctcgatga gttcgggcgt attttcgccg 694560
tgcagccgca aggggatgtt ggatacggtt ttgatgtttt gggtaacgtc ttcgcccgtc 694620
gtgccgtcgc cgcgcgttgc cgcctgcacc aatacgccgt cgcggtagag caggctgatg 694680
gcgaggccgt cgaatttggg ttcgataacg tattcgggat tgccgccgtc caagccgtcg 694740
cgcacgcgtt ggtcgaaggc gtacatttcg gcatggtcga acacgccgtt ttcatcttgc 694800
ggggaaaaag cgttggtcag cgacagcatc ggcacttcgt ggcgtacttc ggcaaatccc 694860
gccaaaggct cgccgccgac gcgctgggtc gggctgtcgg gcagtttgag ctcgggatgg 694920
tttaactcca acgcttcgag ttcgcggaac aatttgtcgt attcggcatc gggtacgctg 694980
ggcgcgtcga gggtgtagta ttcgtaggcg tagcggttga ggaggtcggt gaggcggcag 695040
atgtgttgtg cggcaaattg ttttatatca ctatcagacg gtttaagaag attggtaaag 695100
ttagtgttat gttttgagtt tggattcatg agagaaggtt ttcagacgac ctttgtctga 695160
tacgggatga aacgggcaaa ggtcgtctga aaaatgatag gttgaaaaca gctgaatttt 695220
acccgaaaaa aagcggatat gccgtaacga catatccgct ttgattgcat tcgattttag 695280
gagaacaggc gcaatgcggt tttgccgccc ggttcgatac cgactttgag catctcggac 695340
tgacgcgcca atacataagt gcgcacgtct ttgagccatt gggtcgaaac ttcttccatt 695400
ttgtcgttga ccagattcag gttcaactgg ccggacaggc gtaccgccaa atccataaac 695460
aaatcgtcga aggttttttc gcctgccgga gagtgcggga tgtcgagcag catactgaag 695520
cctttgtagg actggttgtc caaaagggcg ttggtaaacg gctcgttgtt gagcgagcag 695580
atggagaaca tggtcgagcc cgacgtgtcg gtatagtgga acgcgccgtc gtcttccaaa 695640
acgaaaccca cgcccgttac ggcggaacgc agttctacgc cgctgatgct ggtcggggaa 695700
accaaatgga tggcgatggt ctggtcgacg cgcgcgcaga atgcgtccag tgcggaagcc 695760
acttcgataa aggcggcaag gtcggtgtgc agcgtctgac cgcccatgct ttgtgcgaat 695820
gcgtccacct ggcggttgaa tgcggagagt tcttcctgcg aggcaagtcc gttgcggctg 695880
actgcctgaa tacccacgat aaatgcctga tagcggatgc ccgggatggg ttcggcaatc 695940
tggaaatggt cgtccatggt gcagccgaca atctggtagc ggcagcggtt ggaaaggcgc 696000
ggcagtgcgt gcagttcttt ggcttcggtc agcgcgatat aggagatgaa gtcgaagcgc 696060
acgtcaaacc agggtaattc gacttttgac agttctttga gcgtaatcag cggttttgca 696120
ggtgtttgcg gaacgggtgc aggttttgcc ggcgcgtcgg caggtttcgg tgcggaatgt 696180
ccggtttggg gttcggaaac ggtgtgggcg gagttgccga taatgccgct ttcttccaag 696240
gcggtttcga tttcggtttt gaacggggag gcttttgcct gtttctgctt ggcgatgtag 696300
acggcatcct gttcttgcag gttgcgcatg gcggggtctt ggggttttgc cgtttttttg 696360
accgccggtt ggggtttcgg catcatgact gacccgccgg acggtttgcc gtcgcggaca 696420
tggctggttt tgctgttgag cagggcatct ttgtcggagt gtccgaactg gtcgcgcact 696480
tttttgcggt attggttttc ctgatacatg ttgtaggcga caacggcgag gacgacagct 696540

agaaacagta cgatgtaaat catggcaatc acttgttaaa tttcgggatg caggatacgc 696600
aaagtgcggg tactgcggtt aaatcgggct tgcactgcgg ttaaatcggg cttgcgtttc 696660
cggcagtctg acggaacggc cgattataac gtttgaatta taacgaaaat tgcagggtct 696720
gacagcagtg tgtcgaaata agcggaaatt ttccgaaatg ccgtctgaaa tctgtggttt 696780
tcagacggca tttctgtcca cgagaaaccc tttctcccgt atccgccgcc agtcgaaaaa 696840
atggccgggg tcggttttgc ggccgggcgc gatgtcttgg tgccccgtta ccgccgtgac 696900
ggggtagtgg cggcagattg cgtccaacaa ggcttcgagc gaacggtatt gcgcttcggc 696960
aaacggttcg aaatcgcagc cttccagttc gatgccgatt gaaaatgcgt tgcatttttc 697020
cctgccgccg aatgaagata cgccggcatg gtatgccata ttgtcgcagg aaacgaactg 697080
taccgtttct ccgtcgcgtt tgattaagaa atggctggat acgcgcaaag tgtgtatcag 697140
gctgaagaac ggatgtccgt cggggtcgag ccggttggca aacagctttt ccaccgcatc 697200
cgtgccgtat tcgaacggcg gcagcgaaat gttgtgcaac acgatcaggg aaaccgtttc 697260
ccccgtttcc ctcgggctga aattgggcga cggggtatgg cgtatgcttt gaagccagcc 697320
gttttgccag tgtgcttcgg cgtgattgtc catgatgttc ttcctgtccg gcgggcaatt 697380
tgggttatac tgtcgcccga attttaagac gtattccgaa tgctgggaat cctaccatgt 697440
tgagaaaatt gttgaaatgg tctgccgttt ttttgaccgt gtcggcagcc gttttcgccg 697500
cgctgctttt tgttcctaag gataacggca gggcataccg aatcaaaatt gccaaaaacc 697560
agggtatttc gtcggtcggc aggaaacttg ccgaagaccg catcgtgttc agcaggcatg 697620
ttttgacggc ggcggcctac gttttgggtg tgcacaacag gctgcatacg gggacgtaca 697680
gattgccttc ggaagtgtct gcttgggata tcttgcagaa aatgcgcggc ggcaggccgg 697740
attccgttac cgtgcagatt atcgaaggtt cgcgtttttc gcatatgagg aaagtcatcg 697800
acgcaacgcc cgacatcgga cacgacacca aaggctggag caatgaaaaa ctgatggcgg 697860
aagttgcgcc cgatgccttc agcggcaatc ctgaagggca gtttttcccc gacagctacg 697920
aaatcgatgc gggcggcagt gatttgcaga tttaccaaac cgcctacaag gcgatgcaac 697980
gccgcctgaa tgaggcatgg gaaagcaggc aggacgggct gccttataaa aacccttatg 698040
aaatgctgat tatggcgagc ctggtcgaaa aggaaacagg gcatgaagcc gaccgcgacc 698100
atgtcgcttc cgtcttcgtc aaccgcctga aaatcggtat gcgcctgcaa accgacccgt 698160
ccgtgattta cggcatgggt gcggcataca agggcaaaat ccgtaaagcc gacctgcgcc 698220
gcgacacgcc gtacaacacc tacacgcgcg gcggtctgcc gccaaccccg attgcgctgc 698280
ccggcaaggc ggcactcgat gccgccgccc atccgtccgg cgaaaaatac ctgtatttcg 698340
tgtccaaaat ggacggcacg ggcttgagcc agttcagcca tgatttgacc gaacacaatg 698400
ccgccgtccg caaatatatt ttgaaaaaat aaaccatgcc gtctgaaaag tttgtgtttt 698460
cggacggcat acccttaccg gaactgcaag catgaaaccg caattcatca ctttggacgg 698520
catagacggt gccggcaaat ccaccaacct tgccgtcatc aaggcatggt ttgaacggag 698580
ggggctgccc gtgctgttca cgcgcgagcc gggcggaacg ccggtcggtg aggccttgcg 698640
cgaaatcctg ctcaaccctg aaaccaaagc cggtttgcgt gcggaaaccc tgatgatgtt 698700
cgccgcgcgt atgcagcaca tcgaggaagt catcctgccc gcgctttcag acggcataca 698760
cgtcgtgtct gaccgtttta ccgacgcgac cttcgcctat cagggcggcg ggcgggggat 698820
gccgtctgaa gacattgaaa ttttggaaca ttgggtgcag ggcggtttga agccggattt 698880
gaccctgctg ctggatgtgc cgctcgaagt gtcgatggcg cgtatcgggc agacgcgcga 698940
gaaagaccgt ttcgagcagg agcaggcgga tttctttatg cgtgtgcgcg gcgtttatct 699000
cgaccgagcc gccgcctgtc ccgaacggta cgccgttatc gacagtaacc gcaacttgga 699060
tgaagtcaga aacagcatag aaaaagtgtt ggacggacat ttcggctgct gatgcggcaa 699120
atattgaaac aagcgcatcc gcccgcgccg aaaatcaaac ggcagtgccg caggtgaaaa 699180
tggcggtatg cgccaaactt tcggcatgat agaattacgc tcggttacaa ggcaggatgc 699240
gtcggcaata ttaacgaacc gcccgtaaca tgatgacccg aaagcgtttc ggacagtccg 699300
attcaaatct ttttctcgca acaggattga cacatggaaa actcattgaa agaagccgcc 699360
ctcaagttcc acgaattccc cgtgccgggc aaaatttccg ttaccccgac caaatctctg 699420
gcgaccgaca aagatttggc gttggcgtac tctccgggcg tagccgctcc ttgtatggaa 699480
atccatgccg atccgcaaaa tgcctacaaa tacaccgcca aaggcaactt ggtcgctgtc 699540
atttccaacg gtacggccgt tttgggcttg ggcgacatcg gcgcgctggc gggcaaaccc 699600
gtgatggaag gcaaaggcgt attgttcaaa aaattcgccg gtgtggacgt gttcgacatc 699660
gaaatcgatg aaaaagaccc gcaaaaactc gtggacatca tcgccgcttt agagccgacc 699720
ttcggcggca tcaacctcga agacatcaaa gcacccgagt gtttctacat cgaacgcgaa 699780
ttacgcaaac gctgcaaaat ccccgtattc cacgacgacc agcacggcac ggccatcatt 699840
accgccgccg ccgtattgaa cgccctgcgt tttaccggcc gtaaaatcga agaagcgact 699900
ttggtgtgtt ccggcgcagg tgcggccgcg attgcctgct tgaaccaatt gctggatttg 699960
ggcttgaaac gcgaaaacgt gaccgtttgc gactccaaag gcgtgattta ccaaacccgc 700020
gaagacaaag accgtatgga cgagtccaaa cagttctacg ccattgaaga caacggccag 700080
cgcgtgcttg ccgatgccgt caaaggcaaa gacatcttct tgggcctctc cggcgcgaac 700140
ctgctgacgc ctgaaatact gaacaccatg aacgaaaaac ccatcgtgtt cgctatggcc 700200

aacccgaatc cggaaatcct gccgccgctg gcgaaagaaa cccgtccgga cgtggttatc 700260
ggtaccggcc gctccgactt cccgaaccaa gtgaacaatg tattgtgctt cccgttcatc 700320
ttccgcggtg cgttggatgt cggcgcgacg accatcaacg aagaaatgaa acgcgcctgc 700380
gtgtatgctt tggcggattt ggcgatggaa gaagtaaccg aagaagtggt tgccgcttac 700440
ggtaagaaat ttgaattcgg cgcggaatac ctgattccga ctccgttcga ttcccgcctg 700500
ctgccgcgcg ttgctacggc tgccgccaaa gcagcgatgg aaagcggtgt ggcaacccgt 700560
ccgattgcag atttggaagc ttacgctgcc aagctgagcg aatggaagct gtaagccgtt 700620
tgcggtttaa aatgccgtct gaactgtttt caggcggcat tttgctgtca gattgatata 700680
gtggattaac aaaaatcagg acaaagcgac gaagccgcag acagtacaaa tagtacggaa 700740
ccgattcact tggtgtttca gcaccttaga gaatcgttct ctttgagcta aggcgaggca 700800
acgccgtact ggtttttgtt aatccactat aaatgaaaga tactgaaaaa tgaaagagat 700860
gaaacctgtc cgttatcata ttggcgatat gcccgagact tcaaaacaaa ccgcctcccg 700920
tcatgacgac agggcagtgg gtgttgacga tgattgtttt catgattcct ttggtcaatt 700980
tttgtttggg tgttcggcag aggcaacccg aaccgcgcca atttctgtaa agcgcagttg 701040
cttatttacc tgattggttc gcttatcggt ttggtcttcg cgttgtttat aggtgggtct 701100
gtatcaggta cgcatgatta atgcccgggg ctgattttgc ttcgaggatt tgtatcgaat 701160
atgccgaatt gtttcaaatt tcataccgtt atcgaacggc attggcaaaa accttatccg 701220
gttttgtctt ttctgcttaa gccgctctcc gggctgtttg ccaaaattgc ggcaaaacgg 701280
cggacggatt ttttatcggg aaaacggcaa agcgaaaagc tgcccgtgcc tgtggtcgtg 701340
gtcggcaata ttcacgcggg tgggacgggg aaaacgccga ttgttgccgc gctggtgtcg 701400
ggtttgcagg aaaagggcgt caaggtcggc atcatcagcc ggggctacgg gcgcaagagc 701460
aaggcggttc atgtattgaa tgctgagagc cgagcggaag atgcgggcga tgagcctttg 701520
ctgctgttcc gcaaaaccgg tgcgccgacg gcggtgggca gcagccgtgc agaggcaggc 701580
agggcgttgc tggcggcgca tcccgacatc ggactgattg tggcggacga cggtttgcag 701640
cattacgccc tgcggcgaga tgtggaaatc gcggtgtttc cggcggcgga tacggggcgc 701700
acggatttgg atttactgcc caacggcagt ttgcgcgaac ctttgttgcg gctggattcg 701760
gtggatgcgg tcgtcgtcag cggcggcaag gcggatgcgc tgtttaggcc gtctgaaaat 701820
atgtttcaca gccgtatcga agcgggacgg atttaccgtt tgaacaatcc gtccgaaata 701880
ctggacacag gccgtctgaa aaatcaaacc gtcgtcgccg tggcaggtat tgccaagccg 701940
gcgcggtttt ttgattcgtt gcggaatatg ggcattaccg tgaagcgaac cgtcgcgctg 702000
cccgaccacg ccgacatttc ggcggcagat ttgcccgatg cggacgcggt cattattacg 702060
gagaaagatg cggtcaaatt ttcagacggc atttgcaccg ataatgtttg ggtgttgccc 702120
gtttgtgcga taatcgaacc tgatttggcg gcgtttgtgt tggagcggtt ggaagatgta 702180
ccgaaggccg tctgaaagca cggtttgggc ggagtgatta cggatttgaa taagaacgcc 702240
tcgcgccatc attcccgcgc aggcgggaat ctaagtctcg aattttcagg aatgcctagg 702300
aggctccaga aatcccaaat ctccggattt ccacttggac aggaatgaga aaaccggtcg 702360
tattttttat ctgcattaat cattcattaa aggattgaat attaaactga aaaccttgtt 702420
attgcccttc gccacgctgg cattgtgcac caatgctttt gccgccccgc ccagcgacgc 702480
gtcgttggcg cgttggctgg atacgcagaa ttttgaccgg gatatagaaa aaaatatgat 702540
tgagggcttt aatgccggat ttaaaccgta tgcggacaaa gcccttgccg aaatgccgga 702600
agcgaaaaaa gatcaggcgg cagaagcctt taaccgttat cgtgagaatg ttttgaaaga 702660
tttgattacg cccgaagtga aacaggctgt ccgcaatact ttattgaaga atgcccgtga 702720
gatatacacg caagaagaaa ttgacggcat gattgccttt tacggttcgc ctgtcggtca 702780
gtccgtcgtt gccaaaaatc cgcgcttaat caagaaatcg atgagtgaaa tagcggtatc 702840
ttggactgca ttgtcaggga aaatcgcgca acatcatctg cccgagttta cggaagagtt 702900
gcggcgcatc atctgcggcg gtaaaaatcc cgatgcgggc tgtaaacaag ccggacaggt 702960
tgggaaaagg catcagaaat aaatgatagc cgtctgaaat attgaagagg gcatccgatt 703020
gattgaacca tcaaacccga aagcaaccct atggaaaaaa aattcttaga catcctcgtc 703080
tgccccgtta ccaaaggcag gctggaatat catcaggaca aacaggaatt gtggagccgt 703140
caggcgaagc ttgcctatcc gattaaagac ggcattccct atatgctgga aaacgaagcg 703200
cgagcgttga gcgaagagga actcaaagca tgaccgaatt cgtcgtattg attccggcgc 703260
ggctggattc gtcgcgcctg cccggaaaag ccttggcgga catccacggc aaaccgatgg 703320
tcgtgcgcgt tgccgaacag gcggcaaaaa gtaaagccgc gcgcgtcgtc gttgccaccg 703380
accatcccga tattcagacg gcctgtcagg cgcacggtat cgaagtcgtc atgacttcaa 703440
accggcacga aagcggcacg acgcgccttg ccgaagcctc tgtcgcgctg aagctgccgc 703500
cgcatttgat tgttgtgaac gtacagggtg acgagccgct gattgccccc gaactcatcg 703560
accgcaccgc cgaagtactc gtcgaaaaca acgtccaaat ggcgaccgcc gcccacgaat 703620
tgcacgattt cgacgaattg atgaatccca acgccgtcaa agtcgtcctc gacaaaaacc 703680
gcaacgccat ctacttcagc cgcgccccga ttccctatcc gcgtgatgcg atacgtgccg 703740
gaaaacgcga aatgccgtct gaaaccgccg tcctgcgaca tatcggcatc tacgcttacc 703800
gcgccggctt cctgcaacgc tatgccgaaa tgagcgtttc gccgctggaa accatcgaat 703860

cgctggaaca gctgcgcgtc ctgtggcacg gttatcccat tgccgtcgaa accgccaaag 703920
aagccccgc cgccggtgtg gatacgcaag aggacttgga cagggttcgc gccgtatttc 703980
agaccgtata aaacaggttc aaagggaaaa gatatgcagc aacatattga aaagtggcaa 704040
cacttgagcc gggaagaaca gaaaatcctt gctgaagtat ggggtctcgt gcaaaacgac 704100
gatcaggagg ttcactatga aatgctcaaa ttgaacgcac ccgatgaagc cagcggcgaa 704160
ttttggttca gaatggcaga aacactcagc accctgccgc ccaaccgttc cctcggcctt 704220
agaatgaacg gcggcaggct ggcgaccgcc gtatccatcc tttccgtcat gattgaagac 704280
aatcccgaca taccgcagct ttgggcgcaa aaaattaccg cgctcaatta tagtggatta 704340
aatttaaacc agtacggcgt tgcctcgcct tgtcgtacta tctgtactgt ctgcggcttc 704400
gtcgccttgt cctgattttt gttaatccac tatatttggc acacgggcac aaagcccgtg 704460
ccgacggttt ggcacaacag cccgacaaag cggcagaagc caacgaggag gaatacctga 704520
ccaaagccct gtcgcaaaac ctgctgtcaa cattggatgt cgcgcttgca cgttttcctg 704580
aagacgcgtg gtttcaggaa atcaaacagg atgcacaaaa gcattttgct tgaggatgtg 704640
gcagtcagga atatttccat tcaggaagaa aagaagtgcc tgattgggta taatcagggt 704700
aaatcttatt ttatttcaaa agattaatat ttgctttctg tttttccttg acggtatcgg 704760
aaaagttgat tatagttaca gcttccttag gagtaatggc tgagaggctg aaggcacttc 704820
cctgctaagg aagcatgtgg ggtcaacctg catcgagggt tcgaatccct cttactccgc 704880
cagataaaaa atagacgctg tgttttacag cgtctatttt ttatgcaatt ttatagcggg 704940
ttggtgcaaa accagtatgg tattgccctg tcttgattct gaattttgtt atagtggatg 705000
aacaaaaacc agtacggcgt tgcctcgcct tagctcaaag agaacgattc tctaaggtgc 705060
tggagcacca agtgaatcgg ctccgtacta tttgtactgt ctgcggcttc gtcgccttgt 705120
cctgattttt gttaatccac tataatccga gatgcttgcc gtttatttcc gcctcgttca 705180
aacggcggct ctgatttgcg cggtttctgt ttgccgtatt cgcctatccg taccgcaaat 705240
gttatactgg gaaaaattta ctgattgtgt tttacggcat atttgccgat aggatggaag 705300
agacaaatga gcagaatccg gcaggctttt gccgctttgg atggcggaaa ggcattgatt 705360
gcctatatta cggtgggcga ccccgatatt cggacaactt tggcattgat gcacggcatg 705420
gttgcaaacg gtgcggatat tttggagttg ggtgtgccgt tttccgatcc gatggcggat 705480
gggccggtta ttcagcgtgc ggcggagcgg gcgttggcaa acgggatttc gctgcgcgat 705540
gtcttggatg tcgtcagaaa attccgtgaa accgacacgc aaacgccggt tgttttgatg 705600
ggatatttga accctgtaca taagatgggt tatcgggagt ttgctcagga agccgcaaag 705660
gcgggtgtgg acggcgtgtt gacggtggat tcccctgtcg aaaccatcga tccgctctat 705720
cgcgagctga aggataacgg ggtcgactgt attttcctga ttgcgccgac gacgacggaa 705780
gaccgtatta aaaccattgc cgagctggca ggcggatttg tctattatgt ttcgctcaag 705840
ggcgtaacgg gcgcggcaag tttggatacg gatgaggttt cgcgtaaaat agagtatttg 705900
catcagtata tcgatattcc catcggtgtc ggtttcggca tcagcaatgc ggaaagtgca 705960
cgcaaaatcg gccgggttgc cgacgcagtt attgtcggca gccggattgt gaaagaaatc 706020
gaaaacaata caggcaacga ggctgccgcc gtcggtgctt tggtaaaaga gttgaaggat 706080
gccgtgcgct gacggcggtt cctcatcctg aatattttag gagttgtcca tgagctggtt 706140
agataaaatc ctgccaccca aaatcaagaa tcgcggaaaa gacggttctt ccaatgttcc 706200
cgagggtcta tggcacaaat gcccgtcttg ttcggcaacc gtttattcta ccgagttgca 706260
gcagaacaat caggtttgtc cgaaatgcaa ccaccacaat ccgttgtcgg cacgacaacg 706320
cctgaacctg cttttggatg aggatggcag ggaggaagtt gccggaaatg tcaaaccgac 706380
agatcctttg aagtttaaag acggcaaaaa atatccggat cgtttgagtg cggcacgcaa 706440
gctgaccggg gaagatgatg ctttggtggt gatgaaaggc aagatgaacg gcctgcccgt 706500
cgtcgttgct gcgtttgagt tccgctttat cggcggttcg atgggttcgg ttgtgggcga 706560
acgattcgta caaggtatcc gtcgggcggt tgccgacaat tgtccgttcg tctgtgtggc 706620
ggcttccggc ggcgcgcgta tgcaggaggg tgtaaactcg ctgatgcaga tgacgaaaac 706680
cagtgccgcg ctgcatttgc tgacggaaaa acgcctgcca tttatatcgg tgttgaccga 706740
tccgactatg ggcggcgtat ccgccagctt cgcatttttg ggcgatgtcg tgcttgccga 706800
accgaacgcg ctgatcggtt ttgccggtcc gcgcgtgatt gagcagacgg tgcgcgaaac 706860
gctgccggaa ggcttccaac gcgccgagtt cctgctggaa aaaggcgcaa tcgaccagat 706920
tgtcgaccgc cgcgatatga agcggcgcat cagtgatttg attacgctgt tgtgccgtca 706980
ggacaaagtt tccgccgcct gatggctgat gaatcgagta ccgtctgaaa ccgatgtttc 707040
agacggtatt tttgtgtctg gttatttgtt gtgcggcttt atcgatgggg catagcgtcc 707100
ggcacgttct ttcaggcgtt gtaccaaacc tttcgtgtcg gcgggtacac cgccctcgca 707160
gaatgcctga tacaggacgg tgcgcagtgc gtcgttgcgg cttaatgtac cgcctatcgg 707220
tttccattcg gcgtttcggg gctgtatcca gcggcggttg accgtgtcgc cgtagccgaa 707280
cactttatag gaggaaggtt tgccgttgcc gaacctgatg gagaagcggg cgcagaatat 707340
gcctttggca gtcaggttgt cgtagccttt gtcggagcgg atattgagaa tgtagcggat 707400
gctgccgtcg ggcgcgggca taatttgcag gctgtcgagc aggattttcg gctgtttgcc 707460
gtaattttca tccacataaa tgtcgaacca gccgtccgag tgcgtatcgg gcagcggcgg 707520

cagtttggcg gtatgttctt taaattcgcg ggcggcggcc tcttcgggcg tttcgcggta 707580
gcgggtgttg atcggcgtgt ctttttggct gaagccggca gcgagggacg tgccggcggc 707640
gagtgcgagc agcaggaggg cggtgcggcg cataagtttc tccaaattga aaacggcgtt 707700
attttatggg ttggcaaagg gggctgcaag caactggggt ataatctccc cccgattccc 707760
attttttaaa cggtacaaac gatgaacagc gaaactttag acgtaaccgg attgaaatgt 707820
cccctgccga ttttgcgggc gaaaaaggct ttggcgcaaa tgcagcaggg cgacgtgttg 707880
accgttctgg caaccgacgg cggcgcaccg ggggattttg aggctttttg ccgccaaacc 707940
ggtcatgtgc tgttggatgc ttccgaacag gacggcgtgt tcacgctggt cgtccaacac 708000
aaataaatgc cgtctgaaat gcggatgtcc cgcccgacgg cgttgttttt gaatatcgta 708060
tgtgccgcgt gccgtttcaa aaacaaaccg tccggcgtgc agtgtcggca tttcgggcgt 708120
atcgccgccg atttgttccg gaatggctgt taaccgcctt gccgtcggca aagaagcaaa 708180
acccaagcac aatcaaaatc taaaggctgt gtttgaagat tccgttgatg caacccaatc 708240
agtcggacaa aatgcctttc atccaatgga accggtttcc gaccggacgg aataaccggc 708300
tttcccccgg caaacggatg gaatcgaccg ggtattcaaa cgcagccaaa acctaaaaag 708360
gaacaaccat gcaaaccctg accattatcc gccccgacga tatgcacctg cacctgcgcg 708420
acggcgacgc gctcaaagcc gttgcccctt ataccgcccg ccaaatgggg cgcgccgtca 708480
ttatgcccaa cctcaaaccg cctgtcgtca gtgtagccga cgcgcttgcc tacaaagcgc 708540
gcattatggc ggcgttgccc gaaggtagcg cgtttgagcc gttgatgacg ctttatttga 708600
ctgataacgc cacgcccgaa cttgtacgcg aagccaaagc cgccggcatc gtcgccttca 708660
aactctaccc tgccggcgcg accaccaatt ccgattccgg cgtaaccgac ctgttcaagc 708720
tcatccccgt gttggaagaa atggcgaaac agggcatttt gttcctcgtt cacggcgaag 708780
taaccgaccc cgaaatcgac atcttcgacc gcgaagccgc ctttatcggg cgcgtgatga 708840
aacccgtttt ggcgcaagtg ccgaatctta aagtcgtgtt cgaacacatc accaccgccg 708900
aagccgcccg cctggttttg gaagcaggcg acaacgtagc cgccaccgtt accccgcaac 708960
acctcctgct caaccgcaac gacctcttgg tcggcggcgt gcgcccccat catttctgcc 709020
tgcccgtact caaacgcgaa acccaccgtc aggcattggt cgccgccgtt accggcgaga 709080
aggcgcataa attcttcctc ggcaccgact ccgcgccgca cgccaaatcc gccaaagaaa 709140
acgcctgcgg ctgcgccggt atgttcagtg cgatgaccgc tatcgagctt tacgccgaag 709200
tatttgaaaa agcaggcgcg ttggacaaac tcgaagcctt cgcctcaaaa aacggcgcaa 709260
ggttctacgg cattcctgaa aataccgaca cgatcaccct cgtcaaacaa agccaaaccg 709320
ttcccgcaag tgttccctac ggcgacggcg aacttgtccc gatgcgcgcg ggcggcgaaa 709380
tcggctggac ggtgcagtat tgatgggctg gaaacaaaat gccgtctgaa gggagggctt 709440
tcagacggca tttgtgttgc tgactgattg atacgtcaac ggcgtttgag tttgttacgt 709500
ttcggttatt tccgataaat tcccacaatt ttcaaatttc gccattccca cgaaggcagg 709560
aatccagaaa ttcgatgcga ccagagttta tcaaaaacgg cagcaactca aaaaaccgga 709620
ttcccgcctg cgcgggaatg acgagattga agtttcagaa tttatttgaa atacccaaaa 709680
ttcaaaaaac caaattccca cctgcgtggg aatgacgaaa caaagaaagc agaaataagg 709740
acatagaact ttctttaaat ttgtgatgca tcaacggcgt ttgggctcgt cggggcggat 709800
ttgggcggcg agtttgtcga ggatgccgtt gacgaatttg tgcccgtccg tgccgccgaa 709860
ggttttggta acttcgatgg cttcgttgat aatgacgggg tagggcgttt cgggcatggc 709920
ggacagctcg tggcaggcgg tcagcaaaac ggcgcgttcg atggggttga ggtctttttc 709980
gtccctgtca agtagcgggc ggatttgtcg gatatactct gccgcattgg tttgcgtgcc 710040
gaagaaaagt ttgttgaaca attcttcgtc tgccttggca aagtcggaca tttcgcggat 710100
gtttttagca atttcgggcg cggcggtgcg gttgataagg gattggtaaa cggcttgtac 710160
ggcaagctcg cgggaacggc ggcgggctgt tttcatgatt tttccttgaa acggttgggc 710220
ggcacggtat gccgtctgaa acggaaaggg tgtattggtg tacgccctgt ttgttattct 710280
tcgtcttcaa actgttcttc gagcagcagg ttgacgaggt tggcgcattc gacggcgact 710340
ttggcggcat ccgaggcttt ttcttcaatc cgttcgattg cctgcgcgtc gttttcggtg 710400
gttaggacgg cattggcaat cgggatattg tagtcgagtg cgacgcggct gacgcctgct 710460
ccggattcgt tggaaaccag ctcgaaatgg taggtttcgc cacggatgac gacgccgatg 710520
gcaatcagtg cgtcaaactt ttcggaagag gcaaagttca tcagcgcgat ggggatttca 710580
agcgcgccgg gtacggtggc gacggtaatg ttttcgtctg ccacgcccaa ttcttggagg 710640
gtgcggcagc agactttgag catttcgctg ccgatttcgt tggtgaagcg tgcctgtacg 710700
atgccgatgc ggaggtgttt gccgtcgagg ttgggggcga tggtgttcat tgggtgtcct 710760
ttggtattcg gaggtttcgg aatgccgtct gaaggtttca gtcttgcggc tgccagtcgg 710820
cgacggtttg gaatgtgccg tcttcggcaa gctcccatgc gctgccttcg ggttgggaga 710880
gcagtgcggc ggtttcaggg ttggttttgg cgatgtcggc gaggctgacg atgctgaagt 710940
tgtccggatc gtcggtgtat tcgtcggtct cgtcgccgct gaagaaacgc cagccgctgt 711000
cgttttcaaa aacgggggct tcgcggtaga ggaagccgac gggccggttt tgtttggcga 711060
cggtgttggt ggcgatacag cggtcgagtg ccgaggaaag tgcttgtgca aatgcgttca 711120
ttacgggaat acgttggggg aaaacttacg gattttacca cgattcgtgc gttgtcggca 711180

gacggcggcg gtttggtggt acaatgtgcg ccgtttgcag ccttaaggtg tttctgtatt 711240
tttggagtat ggaaacgcat tcgggctgtt ttttgcggaa gacggtaatg aaagacgatg 711300
ttttgaaaca gcaggcacac gcggcgatac agaagaaact gggctacgcg ttccgcgata 711360
tttcgctttt gcggcaggct ttgacgcaca ggagccatca tgcgaagcac aacgagcggt 711420
tcgagtttgt cggtgattcg attttgaatt atacggtggc gcggatgctg tttgacgcgt 711480
ttccgaagtt gaccgagggc gagttgtcgc ggttgcgggc aagtctggtc aatgagggcg 711540
tgctggcgga aatggcggcg gaaatgaatg tcggcgacgg cctgtacttg gggggcgggcg 711600
agttgaagag cggcggcttc agacggcctt cgatactggc agacgcgatg gaggcgatgt 711660
ttgctgccgt cagcttcgat gccgatttca acacggcgga aaaggtggtg cgccatttgt 711720
ttgccgatcg cgtccggcgc gccgattttc aaaatcaggc aaaagacggc aaaactgctt 711780
tgcaggaggc gttgcaggcg cgccgtttcg ccttgccgaa ataccgtatc gaagagcaaa 711840
tcggttatgc caacgacagt atgtttgtca tttcctgcga tttgggcgaa ctgggtttcg 711900
tgtgccgtgc caaagggacg agccgcaagg cggcggagca ggaagcggcg aaagaggctt 711960
tgaaatggct ggaagagaag ctgccgctga agaggaaaaa gaaatgaggc ggcgcgtgaa 712020
tatgccgtct gaacatatgg atacgaaagc aaatatggat attgaaacct tccttgcagg 712080
ggaacgcgcc gccggcggat accgttgcgg cttcgtagcg attgtcggcc gtccgaacgt 712140
gggcaaatca acgctgatga accatctcat cggtcagaaa atcagtatta ccagcaaaaa 712200
ggcgcagacg acgcgcaacc gcgtaacggg gatttatacc gacgataccg cgcagttcgt 712260
gtttgtcgat acgcccggct ttcaaaccga ccaccgcaac gcgctcaacg acaggctgaa 712320
tcaaaatgtt accgaggcgc tcggcggcgt ggatgtggtg gttttcgtcg tggaggcgat 712380
gcgctttacc gatgccgacc gcgtcgtgtt gaaacaactg cccaagcaca cgccggtcat 712440
tttagtggtc aacaaaatcg acaaggacaa ggcgaaagac cgttacgcgc tggaggcgtt 712500
tgttgcccaa gtgcgcgccg aatttgaatt tgcggcggcg gaggcggtca gcgcgaaaca 712560
cggattgcgg attgccaacc tgttggagct gattaagccg tatctgcccg aaagcgtgcc 712620
gatgtatccc gaagatatgg ttacggacaa atcggcgcgt tttttggcga tggaaatcgt 712680
gcgtgaaaaa ttgttccgct atttgggcga ggaattgcct tatgcgatga acgtcgaagt 712740
ggagcagttt gaagaggaag acggtttgaa ccgcatctat atcgccgttt tggtcgataa 712800
ggaaagccaa aaggcaattt taatcggtaa aggcggagaa cgtttgaaga aaatttccac 712860
cgaagcgcgg ttggatatgg aaaaactgtt tgataccaaa gtatttttga aggtctgggt 712920
caaagtcaaa tccggttggg cggacgacat ccgcttcctg cgcgagctgg gtttgtagtt 712980
tttcttgctg aactttacgc aaatgccgtc cgaacaggtt tcagacggca ttttgtttca 713040
atcgggaata tctttgttaa aaacgggttg atattatctg tgcatattat agtggattaa 713100
caaaaaccag tacggcgttg cctcgcctta gctcaaagag aacgattctc taaggtgctg 713160
aagcaccaag tgaatcggtt ccgtactatt tgtactgtct gcggcttcgt cgccttgtcc 713220
tgatttttgt taatccgaga cctttgcaaa aatagtctgt taacgaaatt tgacgcataa 713280
aaatgcgcca aaaaatttc aattgcctaa aaccttccta atattgagca aaaagtagga 713340
aaaatcagaa aagttttgca ttttgaaaat gagattgagc ataaaatttt agtaacctat 713400
gttattgcaa aggtctcaat ccactataaa gaccgtcggg catctgcagc cgtcattccc 713460
gcgcaggcgg gaatctagtc cgttcggttt cggttttttt ggctagtgcc gcaacattaa 713520
atttctagat tcccactttc gtgggaatga cgcgattaga gtttcaaaat ttattctaaa 713580
tagctgaaac tcaacgcatt ggattcccgc ctgcgcggga atgacgaatt tcaggttgct 713640
gtttttggtt ttctgctttt tccaataaat gcccccaacc taaaatccgt cattcccgcg 713700
taggcgggaa tctagacatt caatgctaag gcaatttatc ggaaatgact gaaactcaaa 713760
aaactggatt cccactttcg tgggaatgac gaagtggaag ttacccgaaa cttaaaacaa 713820
gcgaaaccga acgaaccgga ttcccacttt cgtgggaatg acgggatgca ggtttccgta 713880
tggatggatt cgtcattccc gcgcaggcgg gaatctaggt ctgtcagtgc ggaaacttat 713940
caggtaaaac ggtttcttga gattttgcgt cctggattcc cactttcgtg ggaatgacgc 714000
gattagagtt tcaaaattta ttctaaatag ctgaaactca acgcactgga ttcccgcctg 714060
cgcgggaatg acgaatttca ggtttctgct tttccaata aatgccccca acctaaaatc 714120
cgtcattccc gcgtaggcgg gaatctagac attcaatgct aaggcaattt atcggaaatg 714180
actgaaactc aaaaaactgg attcccactt tcgtgggaat gacgaagtgg aagttacccg 714240
aaacttaaaa caagcgaaac cgaacgaacc ggattcccac tttcgtggga atgacgggat 714300
gcaggtttcc gtatggatgg attcgtcatt cccgcgcagg cgggaatcta ggtctgtcag 714360
tgcggaaact tatcaggtaa aacggtttct tgagattttg cgtcctggat tcccactttc 714420
gtgggaatga cgcgattaga gtttcaaaat ttattctaaa tagctgaaat tcaatgaacc 714480
ggattcccgc ctgcgcggga atgacgaagt ggaagttacc cgaaacttaa aacaagcgaa 714540
accgaacgag ccggattccc gcttgcgcgg gaatgacggg attaagtttt caaaattcat 714600
cagaaattac tgatttaata gcataaattt tttagattat agtggattaa caaaaatcag 714660
gacaaggcga cgaagccgca gacagtacaa atagtacgga accgattcac ttggtgcttc 714720
agcaccttag agaatcgttc tctttgagct aaggcgaggc aacgccgtac tggtttttgt 714780
taatccacta taagtcattc cggcggcaat tttgttgct ttaacgggat aggcggttgg 714840

cggttgcgat aaaggcggcg actttggcgg catctttttt gcctttagac gcttccacac 714900
cgccggatac atcgaccgat tccgctccgg tgatgcggac ggcttcgccg acgttttcag 714960
gggtcagccc gccggcaagc acccacggtt tgcccgaata ttccgccagc agcgtccagt 715020
cgaagcggtt tccggtgccg ccgtattccg aaggatggta ggcatcgaac agcagtgcct 715080
gagcgtcggg gaagcgcgtg gcggcgtttc ggatgtctga tgccgtctga acacgaatgg 715140
ctttgatata ggggcggtgg aactggcggc agaatgcgtc gtcttcgtcg ccgtggaatt 715200
ggatgatgtg tatcggcact tcggcaagga tgcggcggat gttttgcgcg ctttcgttga 715260
cgaaaagggc gacaacgctg acaaacggcg gcagtgcggc ggtgattttt ttggcgcggg 715320
caatatcgac ggcccggctg ctgccttgga aaaagaccag cccgacggca tccgcacctg 715380
ccgctgcggc ggcagctgcg tcttccggtg tggtgatgcc gcagattttg gtgcggattt 715440
tcctcattcg gtattccttt atttgggaaa cggcgcgctt ttgccgtttc agacggcatt 715500
cccgatcagt cgattttgat gtattcgaca gaaaggattt caatttcctc acgcccttcc 715560
ggcgtgttca aaaccacttc gtcgccttcg cgcgctttaa tcagacaacg agccagcggc 715620
gaaatccaag aaatttttgtt ttgcgcggta tcgatttcat cgatgccgac gattttgacg 715680
gtttgctcgc gcccgtcgtc gcgcaacagt ccgaccgtcg cgccgaaaaa cacttggtcg 715740
gtcgcttcgc gcaattcggg atcgacgacg acggcagcct ccaaacgttt ggtcaggaaa 715800
cggatgcggc ggtcgatttc gcgcatacgg cgtttgccgt aaagatagtc gccgttttcg 715860
ctgcggtcgc cgttgcctgc cgcccagttg acgatttgga cgatttcggg gcgttctttg 715920
ttgaccagtt gataaagctc gtctttcaat gcctgccatc cggcgggcgt aatgtagttt 715980
ttggtttcgg tactcatatt gtgtgcggat gaaacgggaa atgtgatgcc gatatgggaa 716040
atgccgtctg aaaacccggc gttcggattt cagacggcat cgcggtttgg gaagccttat 716100
tcttcgtcgc ccgcatcgct gatgctgatg ctgtgttcca tcctgctcgg gtggattttc 716160
agaccgccgc agccggattt ctcggcagac aggcggtcga ggtaggcatc atcgatgtcg 716220
ccggtctgat aaatgccgtt gaaacaggac gaatcgaagg attcgatttt cggattgagt 716280
gctttgacga cggcttccaa atcgcccaag tcttgaaata cgatgccgtc cgcgccgatt 716340
tcggcggcga tttccgccgc gctgcgcccg ttggcaatca actcttcgcg cgtgggcata 716400
tcaatgccgt acacattggg atagcgcact tcgggcgcgg cggaggcgat atagactttg 716460
cgcgcgcccg ccgcgcgtac catttcgacg atttcgcggc tggtcgtccc gcgcacgatg 716520
gagtcgtcca ccagcaacac gcttttgcct gcaaattcgg tttccatcgg gctgagtttt 716580
tggcgcacgg attttttgcg cgtcgcctgt ccgggcataa taaaggtgcg gccgatatag 716640
cggtttttaa tcaaaccctc gcggtagggt ttgtcgagat ggacggcaag ctccatcgcg 716700
ctggggcggc tggtgtcggg aatgggcatc acgacatcga tgccgtccac gggcagctcg 716760
cgtttgattt tttccgccag cgacacgccc atatccaagc gcgattggta aacggatacg 716820
ccgtcaatca cagagtcggg gcgggcaaaa taaacatatt caaaaaggca ggggctgagt 716880
ttggcacggt cgctgcattg gcgggcaatc attgtgccgt caaagccgac aaataccgct 716940
tcgcccggcc ggatgtcgcg ttccaaatcg taggtaagcg cgttgaaggc gacggattcg 717000
gaggcgacgg cataggattt tctgccttcg ctgtcggttt gcgaacccaa taccagcggg 717060
cggatgccgt aagggtcgcg gaaggcgagc ataccgtagc ccgcaatcat ggcaatcaca 717120
ccgtatgcgc cgcgcaccag gcggtggact tgggcaacgg cgttgaaaat attgtcggca 717180
ttgagccggt gcgggtcggc gtttttagag acttcgcggc gtaattcgtg cgcgaatacg 717240
ttgagcaaga cttcggaatc ggagctggtg ttgacgtggc gcaggtgttt gttacacacg 717300
ttttcataca gttcggcagt gttggtgagg ttgccgttgt gcgccaaaac gatgccgaac 717360
ggcgagctga cgtagaaagg ctgcgcctcc gcgctgctgc ctgcgttgcc cgcagtagga 717420
taacggacgt gggcgatgcc ggcgttgccg gtcaaatcgc gcatattgcg tgtgcggaac 717480
acttcgcgca ccatgccttt gcctttgtgc atatggaagg taccgccttc cgccgttgca 717540
atgcccgccg catcctgccc cctgtgctgc aacatctgca agccgtcgta cagaagctgg 717600
ttcacgggtt catgactgac caaacctaat acgccgcaca tatcgtcttc tccgattcga 717660
ggtttaaggg taaaacggaa ttataaagta aacggtggtt ttttgcctga attgttgaca 717720
atatttgagc gaaggacaga taggtgggtt tatggagaat aagatttata gtggattaaa 717780
tttaaatcag gacaaggcga cgaagccgca gacagtacaa atagtacggc aaggcgaggc 717840
aacgccgtac tggtttaaat ttaatccact ataatctgtg atatggctga ggaaaggaaa 717900
aaacatttca gacggcataa aagaggatgc cgtctgaaat atccgtatgg caatcaatcg 717960
tcttccggag tttccgccgt gccgccgcta tggttcaaca cggcttcgga aagcgatacg 718020
aaaaacggca gtgtgtaaga ttgccgccat tcttcggtat cgggcaggtc ggtttttgaa 718080
gcaagcatga ccagcagggt aacaatcaaa acgcctttca atgcaccgaa tacgccgccc 718140
aaaatgcggt tggcaaagcc caaaccgacc gccgaaactg cgctggtcag cagcgaacgg 718200
agcattttct ggatcagaca ggcaatgacg aacagggaaa tgaacgacag agccaatgca 718260
aacaggcggg gttggaacga ggcaaaggcg aggtcggcga aggaggcggc aaagagtttg 718320
gcgaaaaaga aggaaaccac ccatgccgcc attgagcctg cctccgcaat cacgccgcgc 718380
atcgcggata gcacgatgca ggcggcgatg acggcggaga cgaggaggtc ggcaatgggg 718440
aggctattca ttcgttacct gaccggcgat accgtgtacg cgcaatttgt tcaaatcgcg 718500

ttcggcatcc cttgcgtttt tatagttgct tgatttgacg cggtaaactt tgccgttgtc 718560
ggtcataatt tcggtgatgg tcgaatcgat acccgccgcc ttcattttgc gctggaggct 718620
taaggcgcgt tctttttcgg cataacctgc ctgaatggcg gcttttttac cggatttttc 718680
cttctcggcg gttttggtct tgtccgcttt gtcggttttt tgtgccgttt cgtgtttttt 718740
gccgtccgaa cggtcttttt cggctgtttt tttgctttca gccttgtcgg ctttttttcgc 718800
ttcttttacc gcgctgtcgg attttgccgt atccggtttg gtttttttcgg cggcagtttt 718860
cggtttgtcg gcaacttttt cggcggtttt ggtttctttg gctttgggct tggctttggc 718920
agtgcgttcc gctttttgcg gttttgtttc ggcagtgcgt ttcggttttt caaccgctac 718980
cgtatccgta ctgtcggcag ttgccggcac tttttcggca gcgcgttgtt ttgcctgctt 719040
cggtgcggtt ttggcggttt ctgcctgttg cagtttctcg gatgcttcca aacctttgat 719100
gttgctgtct tcgaggcgct cgttaatcag caccagcggc gcgcctacgt tttcaggctc 719160
gctgatttcg ctgtcggcgg cagaaggctt gtcttcgcct gccaagtcct gcggtttgtc 719220
ggcggcggat ttcaaggcag gggtttgtgc cgcacctgcc gctttgtttt ctacgccgct 719280
tgtttcgccg gcagtctgtt cggcagggcc ggaactgagg gcggctgcca gcaggatgca 719340
ggaggcggca accaggcaac ttgccgttac gaggcggcgg cggttgcgcc gtttgagttg 719400
ttcgtaaccg ctcaggactt cgttttgttt gttttcggac atagaagttt ccttttaaag 719460
taccgacatg acatcggcaa cggtatgaaa tgagccgaaa acgacgattc tgtcgttctc 719520
gcccgctttt gaggctgccg cccggtatgc ttcgcggacg gcggcgaatg tttgtatgtt 719580
ttcgatgctg tgttcgtgca gtttgttttg caggctctcg agcgtcatgc cgcgcggtac 719640
atccaacggt gcgatatacc actcgtcaaa ctggtcttta acggtttcca acacgccgtc 719700
tatgtctttg tcggacaaca tgctgaacac ggcggtgcgt ttttgcgcaa acgccaaatt 719760
aatcaggctg cggcgcaggg cgcgggcggc gtggggattg tgtccgacat ccaaaacggt 719820
cagcggccgg ccgggcagga cttggaagcg tccgggattt tcaaccagca acaaaccgcg 719880
cttgatggca ccgatgtcca ccggcaattt gtcgttcaag cattccaata cggtcagcgc 719940
gcaggcggca ttggaaagct ggtatgtgcc gcgcaatgcg gggaagggca gggcattgcg 720000
gttgcgcgcg gggtcgtctg aatgttgcgg ccggaagcgg tagttccatt ggatgttttc 720060
catcgcgtga aactcgaaat cgcgctgcac catcagcagt ttcgcgccta tggcttcggc 720120
gtgtgaaagc aacgatttgg gcgcggggtt ttgaccgcag atggcgggtt tgccgctacg 720180
gaacacgcct gctttttcaa agccgacctg ctcgaccgta tcgcccaaaa atgcctgatg 720240
gtccaaatcc acgctggtaa ccaccgcgca atcgccgtca aacgcgttga ccgcgtccaa 720300
gcggccgccc aagccgactt ccaatatcat cacgtcaacc tgttcgcgca tgaagatgtc 720360
gacagccgcc aaggcattga attcaaaata agtcagtgat atttcgccgc gcgcggcttc 720420
gatgcgctcg aaagaggcaa taatcgtatc gtccgaaacg ggttcggcgt tgatggcgat 720480
gcgttcgttg taacgcaata aatgcgggct ggtcagcgta ccgattttgt agcccgcctg 720540
tttgtaaatc tgtgtcaggt aggcacagac cgaacctttg ccgttggttc ccgcgacaac 720600
gacgacgggg cattgcggct cgagcttcat gcgttttttc acttcgctcg tgcgctccaa 720660
acccatgtcg atcaaaccgc cgctgtgggc ggtttccaaa tgcgagagcc agtcttgtag 720720
tgttttcatg agtgtttcgt tttcaaatgc cgtctgaaat cagtctgatg tatcggtttc 720780
ggcggttttt ttcggctgcc gccaaagtac ccaaactttc agcttgcggt aggattcttt 720840
gtccgtcatg tcgggcatga tgcattggcg gacggttttg ccgccggtgt cccattgtaa 720900
gaataaggca taaggcgtaa ccatactgct gcccgacagt gccgccgcct ttgccgtttt 720960
gtctttgccg gatacgattt ccgcctgtcc gtcgcggtct atggtaatgg cggttatggc 721020
atggcggtgt ttcagattcg ttatcctgag cgagtatgcg taacttgcca ccaaagccgc 721080
caaaccgaac cacatcatcc ggccgtaaaa ccaagtcagg cagacggcaa gggaggcggc 721140
gtgaagcgat acagtcagga tgttcaggat gcgggacggc ctcaatgccg tctgaaaggc 721200
gcgcacagcc ttacatcatg ttgtcgaaca cgggggtaat gttcaattcc gcttcttcca 721260
tgttcaacac tatatcgtgg atttcgatgt cgaaaaattc ccaaaacgcc ttcagcccca 721320
tatcttgcgg ccatttatcc ttatcgatgt cccaacctgc cagctccgcc tcgaaaatct 721380
gccggtagcg ttcgtcgaag taggaaacga cggcttccgg ttcgtcgaac tgcggaacga 721440
ggaagacgga acagttggca cgaagctgct ctatggtcag gtcgggcata ttttcgtcgg 721500
tgcttttgag ccattccaaa aagcgcgcgg tcggcttgag gacgacggcg gtgcggtcaa 721560
caaaatacat ggttttcttt ctcaatcatc ttgcggtgtc gggatatgct gtctgaacgt 721620
tcggttttca gacggtatag catcagtggg tcatgacctg ttgcaggaac tgcttggcgc 721680
gttcgtgttt cgggttggta aaaaacgctt cgggcgtttc gtcttcgagg atttgccctt 721740
tatcgacgaa aatcacgcgg tcggcaactt cgcgggcaaa ccccatttcg tgggttacgc 721800
acatcatcgt catgccgctt tctgccaagt ctttcatcac tttcaacact tcgccgacca 721860
tttcggggtc gagtgcggag gtcggttcgt caaacaacat tacgcgcggt tccatcgcca 721920
aaccgcgtgc aatcgccacg cgttgctgct ggccgccgga aagttgggaa gggaaggcgt 721980
ctttttttgtg tgccagtccg acgcgttcca aaagctccat tgcctttttc tccgcctgtt 722040
ccgcattttg cccttaacct tcatcggtgc gagggtaatg ttttccaaca cggtcaggtg 722100
cgggtagagg ttgaagcctt ggaatacgaa gccgacttct tcgcggattt tgttcaaatc 722160

ggttttgggg tcggcaacgt tgacaccgtc cacccaaatc tcgccgcttt cgatgctttc 722220
aagctggttg acagtgcgga tgagtgtgga tttgccgctg cccgaaggcc cgcagacgac 722280
gaccacttcg cctttttga tttccaagtt tacgccgttg atgacgtgca ggtctttgaa 722340
atgtttgtgt acgtttttga atttgatcat gtcttttctt tcagacggca tttgccgttg 722400
caggttgtcg ttacgggagc tccatatgat gaagcgtgta gcgtctgccg tcaaaaaaac 722460
ggtcgttcgg attggtcagg caggctgcaa gcggcagtat caggggtaaa agcaggtatt 722520
tcgtcatcgg cttactccct tttcagacga ccttgcccgc cagataattg ctcaacgcca 722580
cgtatttctc tggcgcgata tgttcggcgc ggtcttgcgg attgatgccg actgcctgca 722640
aatcatcgtc gcctgcaagc tctttcagat tgttgcgtat ggttttgcgg cgttggtgga 722700
aggcgagttt cacgagtttg gcaaaatgct cgaaatcgtc cgccttgccg atgcggtgtt 722760
tcaccggaat catacggacg acggcggaat ccactttcgg cgcagggtcg aacgattcgg 722820
gcggtacgtc aatcagcatt tccatatcga aaaaatattg cagcatcacg cccaagcggc 722880
cgtagtcgtt gcttttcggc gcggcaacca tacgctcgac cacttctttt tgcagcataa 722940
agtgcatatc gacgacatcg tccgccacct ccgccagctt gaacaaaagc ggtgtggaaa 723000
tgttgtacgg gaggttgccg acgattttct ttttgcctgc gatgccgttg aaatcaaact 723060
gcaatacatc gccttcgtga atcaccagtt tatccgcaaa cggcagcgtt ttcagacggc 723120
atacgatgtc gcggtcgatt tcgacaacgt gcaggcggtt cagcttttc gccaaaggtt 723180
cggtaatcgc cgccaaaccc gggccgattt caatcacgac atcatccgcc tgcgggcgca 723240
cggcgttgac aatatcgctg ataatccgcg tgtcctgcaa aaaattctgc ccgaaacgct 723300
tgcgggcttt gtgttctttc atcgtgtttc cttttcggtt gaaaccccgc cctttagggc 723360
ggtagaatca gactctattt gggaggggcg taactctttc caaatcagga tggcacatag 723420
ggcggtgctt tatgtgtcgt cctgtgtgtt gaaacataaa tgtgtttaca gtatccgttt 723480
gatgtcggca ttgtaaccga aaacggcagg gcgtgataat gctgtttgaa ggcttgccgt 723540
gtttggcggt ttggtgcaaa aaccggctgt ctgccgtttt gcctgttgga ggattgaacg 723600
tgtctgaaaa tctgcttgaa atcgaaaccc atcccttcga tcccgtgttg ccgccgaagg 723660
ctgctgtcat gatgatgggg acgtttccgc ccaaggaaga caaacgcgcg atgcagtttc 723720
attatccgaa tttccaaaac gatatgtggc gcgtttatgg gctggtgttt tttaatgatg 723780
cggcgcattt ccaaaggttg tctgaaaaag cgtttgatgc cgagaaaatc aaggcgtttt 723840
tgcacgaacg ggggattgcg tcctgtccga ccgttttgaa ggcggtacgt cagcacggca 723900
atgcgtccga caagttttta aaggtagttg aaaccgtcga tttggcggcg gtgttggcaa 723960
aaatacccga gtgccgccat atttgtacga caggcggcaa ggcgacggaa atcctgctcg 724020
atattcaggg cggcggtatc aaaatgccga aaacgggcga aaccgtgccg tttccgtttg 724080
ccggacggga tttgacgctg acgcgcctgc cttcgacttc gcgcgcctat cctttgagtt 724140
tggcgaaaaa agcggcggcg tatcgggcgt tttttgaaat ggcgggcttg tgtgaaaaac 724200
agttataatt gccgacaatt tcccgttcag acggcatgtt tgcaaaaacg gaaatgccgt 724260
ctgaaaattt gaagcacaag gaagaatccg atgaagaact accacgcgcc cgacgagaag 724320
ggctttttcg gcgaacacgg cgggctttat gtctccgaaa ccctgattcc cgccttgcaa 724380
gagctggcgg atgcctataa ggcagcgaaa aacgatcctg aattttggga agcgttccgc 724440
catgatttga aacattatgt cggcaggccc agccccgttt accacgccgc gcggttgtcc 724500
gaacatctgg gcggcgcgca aatctggttg aagcgcgaag acttgaacca caccggcgcg 724560
cacaaagtca acaacaccat cggtcaggca ctgttggcaa aacgcatggg taaaaaacgc 724620
gtcatcgccg aaaccggcgc gggtcagcac ggcgtggcga gtgccaccgt tgccgcacgc 724680
ttcggtatga cttgcgacgt gtatatgggc gcggacgaca tccaacgcca aatgcccaac 724740
gtgttccgta tgaaattatt gggtgcgaac gtggtcggtg tagaaagcgg cagccgcacg 724800
ctgaaagacg cgatgaacga agccatgcgc gaatgggtcg cccgcgtgga cgacacgttc 724860
tacatcatcg gtaccgccgc cggccccgcg ccgtatcccg aaatggtgcg cgatttccaa 724920
tgcgtgattg gcaacgaagc taaagcgcag atgcaggaag ccatcggcag acagcccgac 724980
gttgccgttg cctgcgtggg cggcggatcg aacgccatcg gtttgttcca cccgtatatc 725040
ggcgaagaaa acgtgcgcct cgtcggcgtg gaggctggcg gtttgggcgt gaacaccccc 725100
gatcacgccg cgccgattac ttcgggcgca ccgattggcg tattgcacgg tttccgcagc 725160
tatctgatgc aggacgaaaa cggtcaggtt ttgggtacgc actctgtttc cgcaggcttg 725220
gattaccccg gcatcggccc ggaacacagc catctgcacg acatcaagcg cgtcgaatac 725280
actgttgcca aagacgacga agcactcgaa gcctttgact tgctctgccg cttcgagggc 725340
atcatccccg cgctcgaatc cagccacgcc gttgcttggg cggtgaaaaa tgcgccgaaa 725400
atgggtaaag accaagtgat tttggtcaac ctctcaggtc gtggcgacaa agacatcaat 725460
accgtcgcga agctcaaagg gattaaactg taacctcgtc cgtctgatat agtggattaa 725520
caaaaaccag tacggcgttg cctcgccttg ccgtactatc tgtactgtct gcggcttcgt 725580
cgccttgtcc tgatttttgt taatccacta taaaaatgcc gtctgaagcc tgagttcaga 725640
cggcatttta ttttgctatg aatttagtat tttagaaacg aatctgtatt ttaatttgtc 725700
cggatttttg tttttccaat tgttttcctt ttgtaatact gccatttacg tttaatgtaa 725760
cattacggta cagtaacgcg gcacctgctg aatattgctg ttgattatct gctttataga 725820

cgaaggaatt accgcccaca ttcacgccgc ctttgccata attggcaaag taagctgcag 725880
ataacaaggg ttttacggta aggttgccga ctttaaaccg ataagcaaaa tccagtccgg 725940
ccgttagtgt tttcactgac atagaactta ctttaacact gtcgttaccc aacttgtaat 726000
ctgcagatga caggcggctg taacggatcc ccgcactggg gacaatctcg aattgattga 726060
ttttcagcgt attgcccaaa gtaaggccgg tttggatgct tgctcggtta aagtttgctt 726120
tttgctgcgt ttgtaaccgg cttctcaagc tgcctgcacc aatatcgccg gccacatacc 726180
aagcatcatt taaataatac ttaccataaa ggttggcttg cacaaaagta tttttgccgc 726240
tcgcctgatc aaaagtatgc tgactgtcag agtaagtcaa tacgccgcct atctgcatat 726300
tttcggacaa gctgcggtca atgccgattt gtgtttgcgt gcgtttcgaa ctaaaccggc 726360
gatattgtgc ggaagcataa tcacgaccat aaccggtgtt cgacatccaa acactgtttt 726420
tttcggcatc agcgcgtgat ttttgtgcaa tgtgccgtgt taatgaagca cctgtatcca 726480
acaagataga ttgcgtgctt gccattgcgt cagataaagc cgagttggta ttggtgctga 726540
ctgcatcggc ttgcgcggcg gcttgggctt gcagctgagt ggcggcttgg gctcgcggct 726600
gcgcggctct tggttgcaaa ctcactgtct caactttttc atgaagttcc gtattgtctt 726660
gaggctgttt gtctgatgtg tcaaccgatt cggatacatc ttcatcttcc aacgcatcca 726720
aggggatttc ttcataatca ttttcatatt tttcatgaag ttccgtattg tcttgaggct 726780
gtttgtctga tgtgtcaact gattcagata cattttcatc ttccaacgca tccaagggga 726840
tttcttcata gtcattttca taccagtccg gattatgcaa ggccctgggt gctgcgtaag 726900
ctgacgaatc aaatgatggc gagggcggtg ccggcagagt agatctgcgt ccgcgacgtt 726960
tcggacgatt ttgggaagct gccatataat cctgaggtgc ggcacggcgt tttcggcgtt 727020
gcggctggga ttgggctgct tgacggtgct cttcttcctc agcttttcgt ttcgccgatt 727080
ctgccgcttc tcgatctgtt tcggcttttt gttttgccga caactctgct gcttggcgtt 727140
ccgcttcttg acgacgtgca agttcggcgg tctggcgagc ttcttgctgg cgttttgctg 727200
cttcggctgc tgccctttgc ttggcaagtt cggcggtttt gcgttcggtt tccgcttttt 727260
gtttgacggc taactctgca gcttttcgtg cttcttcctg ttgacgcgca aggccttttt 727320
gttggcgtgc cgccaatgct tgggcttcag ctgcggcttt ttggtttgcc gacaattctg 727380
ccgctttgcg ttctgcttcc tggcgatgtg caagttcggc agcttggcgt tttgcctctt 727440
cggcttctgc ttttcggcgc gcagccaatg cttgagcttc acgttcggct tctgcccttt 727500
gttttgccaa caactctgcc gctttgcgtt cttcctgctg tcggcgcgca agttctgctt 727560
gggcttgggc aatttccaca ttgttttgct gtaccgaacg gtgtccgcgg cgtttaggac 727620
ggtcttggga agctgccata taatcctgcg gtgcggcacg gcgtttgcgg cgttgcggct 727680
gggattgggc tgtttgacgg cgttcctctt ccccgaccct ttgttttgcc gacaactctg 727740
ccgcttcgcg ttctttttca tgccggcgtg caagctctgc agcttggcgt tttgcctctt 727800
cggcttctgc ttttcggcgt accgccaatg cttgagcctc acgttcggct tcgacttttt 727860
gttttgccga caactctgcc gcttcgcgtt ctttttcatg tcgacgtgca agttcggcgc 727920
tgctgcgttc ttgttctgct ttttggcggg tcgccagctc tcttgcttcg cgttcggctt 727980
ctgctttttg ttttgctaac tctgccgatt tacgctctgc ttctgcttgc tgacgcttca 728040
cttgctccgc ttttgcttgc tggcgttttg cttcttcggc ttgatttgcc tgcgggctag 728100
gcggtgcgac gacgatattt tgaggcttgg caatttgtgc accgtccgtt tgtgttgcct 728160
tttgtgcttg agaagccgtg tttgtggcag gagacggggc cggtttgact cggcggcggt 728220
tctcggcata aggattgtac aatcgggtaa taccgttttc tgttttgatt gtataacgca 728280
atgcccctaa atctacatgg ttattcgcca aggaaacaga aaggcgggag cggtcttgta 728340
cggatgatgc atcaaagaga ttcagccctt cctgattggg ttcgcctgtt ttatcttgaa 728400
catggagctg ataatgaccg gatgcggatt ccttcacaag cactttatcc ccaagatttt 728460
tcgccaggtg cgtcagataa tgaaaatgcc cgttaccgga taaatgattg atttgagcg 728520
tgtggtattt atttgcactt tgcgcatcgg aggcattgtt caaatgaata tggctatccg 728580
ccaatgacag attgtgtact tggctgtcgc cggtcaaatg ccatttgcta tgttggttta 728640
ggctgacacg gctgtttcct tgcccttgaa tttgccccca taatgcagcc ttgcccaaga 728700
ccaatgccgc attctggttc agattcacat tgccgttaat ctgtgtcgct ccaaagctgt 728760
ttaaagcctt atcggataag ttgcctgtgt tgcaggtaac gtaaccggta tagtccgagc 728820
gcacgcaaac ctcatcgccg tttttgtaac ccaaatttac tttggcgttg tctgttgcgg 728880
tgatgttggc ggtgatgtcg gatacatttc tgccggaaga gaatgatgcg gattgattaa 728940
ccgcaatttc tgtggctttg aatgtgcggt ttatccagtc gtcttcaaat acgacttcat 729000
tgtttttgga gaaatgtgcg tcttttcggg ctgaagattt gttcacaaaa tctcttgcgt 729060
gtggtgttgg acgacctgat aacaagacat tgccttgagt tacgcttatt tttccgttta 729120
aattagtgcc gcctgttaac aagaaacggt tttgcgcgct tttgccgttg aaattaaggt 729180
ttaatgcacc gttatgtcct tttccgtttt cttcgccaaa gaaaccgcta aaaccgctaa 729240
tacgctgatt gtttttgtgg ttcatcgcgt ttttcttggc ttcttcttgt gtgctgccca 729300
ttaaaatcca gtcgttattt tccgtttgtc cgttttcggg catcggtgcg ttcacgctgc 729360
cgccggattt tagggcgtaa taacggtagt ttttgaaata aagatctttg ccttgtggaa 729420
tcggtttcct agggcggtaa tagtaataac cagcatcgtc atcatcatta ttttgaatat 729480

aatgaataga gatggttttg ggatcggtaa tcaaagattt acccgttagc gtgattgtgg 729540
aggcgtggcc tgtgttgtgg ttgacaatgc gcgcgccttc atccacgttg cggatgtgtt 729600
caaaagtcaa gtcattgcca ttggcatcca aacgaccgcc acggaaaccg aaatataggt 729660
tatcgggatt aatctgattt gaactattta ataccaatgt accgcgtccg ctgacaatgc 729720
cgacttggga gaaagcctgg actttttgt cggcatcggc ttgttgattc agaataaccg 729780
taccgtcgcc gacttttaat tgcccttggt taacgcctgt gccgtttatt tctaatgtgc 729840
ctttgccgat ttttgccaat ctgtcgccat tcggattttt gacttgccaa acgacttttt 729900
tgccgtcggc aacatcaatc cccgcaccta gccaagtgat gtcattattt atacctttga 729960
ctgtgtaatc gcctttgaaa aacagaccgc ccgcgccttg gttgatgttt tgatccaata 730020
ccaaagtgcc gttgttttca aaggtaacat tctgtccgtt gtttgcatcc ctttcattgt 730080
tggcaagcct taccgctgtc gaaccgatat ggctgtttgt gcccgtggtt ttccaatgat 730140
gttctccatt acctttgatg gtgccggcgt tatcgcgttg tttgatttca tctgcaaatt 730200
ctttttata gatattccat tcttgccaag agttttttg atagcctgcc caataatcgt 730260
aagcacctaa aaagacccag cgtttttctt gtttatcata agcaaataat ggtgaaccgc 730320
tatcgcctaa cacagcatag ttagtcaatg cgttttgaga taaaacttct ttgagttttt 730380
ctggggaatg atgttttgaa ttatcaccga agccaatcaa gccttcttga tttagattcg 730440
atgtgacatt cacgtcttga taaggcgtac ctgcaatggc ataacgataa gctcgtgaaa 730500
gctctgtcat attgtagcgg ctgttatatt caaattgcgt acctgctcca actcgcacaa 730560
actcagaaaa acggttttta tctttatagg tttcaacgcc gccgcctgca ctggttggtg 730620
caataggtgc gacttctgtt acgaatttat taaggcgtgc catgttgtag tcttcaagac 730680
gaccttgatt accgtgatgc caatttttat ttggttcgta gtcattttgt gcaactgaac 730740
gatattcgtt ttcatcattg ctaacatcta agtgcccatt gtgatgcccg taataagaga 730800
tttcgtctcc ttttacatgt ttgacgctga cggcatactg gggatcgatg acggttaatg 730860

tacgtctgtt gacatctgca acgctaaaat caatcatcgg tacgttggat aatgcgttgc 730920
cgatgttttg accttgtttg tttttcactg ataaatcggt tgcgccgaca aaaaatttgc 730980
ctttgttttc tgcaaagtca cggaatattt gataatcgac atcgtctctg accaatgccg 731040
cttctgagta tggcgtaagg gcataggcaa gaaagatgga taaggatatg gcgttaattt 731100
taaaacgttt ggttttcata aggttttacc gttttaaggg tgataatgtt ttgtatttta 731160
cgccggtttg gtggtgttga ctatttttg ttgtgtaaat cattcatttt ggtgttttat 731220
tgccaattta aaaaaagaat cccgatgttt ttatttccgc ttcctttgtt ctgttattca 731280
agcgaaggcg ggaagccgat tttcggggtt cggttcttcc gttaaatttc tgcggctttt 731340
tgtttttgga ttcccgcttt tgcgggaatg acgggatttt aggtttctga ttttggtttt 731400
ctgtttttga gggaatgacg ggatgtaggt tttcttaacc ctgagtccta gattcccgct 731460
ttcgtggtaa tgacgagatg ggggttcgtg ggaatgacgc ggtgcaggtt tccgtacgga 731520
tggattcgtc attcccgcgc aggcgggaat ctagacctta gaacaacagc aatattcaaa 731580
gattatctga aagtccgaga ttctggattc ccactttcgt gggaatgacg ggattttagg 731640
tttctgattt tggttttctg tttttgtagg aatgatgaaa ttttgagttt taggaattta 731700
tcgggagcaa cagaaaccgc tctgccgtca ttcccgcgca ggcgggaatc tagaccttaa 731760
ggcagcggca atattcaaag attatctgaa agtccgagat tctagattcc cgctttcgcg 731820
ggaatgacga aaagtggtgg gaatgacggt tcagttgcta cggttactgt caggtttcgg 731880
ttatgttgga atttcgggaa acttatgaat cgtcattccc gcgcaggcgg gaatctagaa 731940
cgtggaatct aaagaaaccg ttttacccga taagtttccg caccgacaga cctagattcc 732000
cactttcgtg ggaatgacgg gattttaggt ttctgatttt ggttttctgt ttttgaggga 732060
atgacgggat gtaggttttc ttaaccctga gtcctagatt cccgctttcg tggtaataac 732120
gggatgtggg ttcgtgggaa tgacgatgga aagtttgccg ttgtctcgga taatactgag 732180
gcttttcgtt tgcattctta tagtggttta acaaaaacca gtacagcgtt gcctcgcctt 732240
gtcgtactgc ttgtactgtc tgcggcttcg tcgccttgtc ctgatttaaa tttaaaccac 732300
tatatcattt tcaaatcttg ttatgacggt ttttcggatt tgctttatta tccgtttatt 732360
tttgaaatat ctggggtggg gagacgtgtt ccgtcgttgg tttttgccgt gttgggttgt 732420
cggcgcggcg gcttcgtttg cgctgccggt cgtgccgcat tggctgtttt ggctggcggc 732480
ttttgcggtt tttgctgtgt ttgcaaggcg ttttgcgttt gccggtctga tgctgtgcgt 732540
gttggcgggc gcggcttacg gcgtattcag aacggaagcg gcactgtctt cgcaatggcg 732600
ggcggaggcg gtttcaggtg tgccgttgac ggtggaagtg gcggatatgc cgaggtcgga 732660
cgggcggcgc gtgcagtttg cggcaaaggc tgtggacagc ggtggtcgga cgtttgattt 732720
gctgctgtcg gactacaaac ggcgcgaatg ggcggtcggg agcagatggc ggataacggc 732780
acgtgtgcac cccgtcgtcg gcgaattgaa cctgcgcggt ttgaaccgtg aggcgtgggc 732840
attatccaac gggataggcg gcgcggggac ggtcggtgcg gacagggttt tgctgcatgg 732900
cggaagcggt tgggggattg cggtttggcg cagccgcatc agccgtaatt ggcagcaggc 732960
ggatgcggac ggcgggcttt cagacggcat cgggctgatg cgcgcgttga gcgtgggcga 733020
acagtcggca ttgcgccccg aattgtggca ggcgttccga ccgttggggc tgacgcattt 733080

ggtcagtatt tcgggtttgc acgttacgat ggtggcggtg atgtttgcgt ggctggcgaa 733140
gcggctgctt gcctgttccc cgcgccttcc tgcccggccg cgcgtgtggg ttttggcggc 733200
ggggtgtgca ggcgcgctgt tttacgcgct gcttgccggt ttttccgtgc cgacgcagcg 733260
cagcgttttg atgttggcgg cgtttgcgtg ggcttggcgc aggggaagat tgtcggcgtg 733320
ggcgacgtgg tggcaggcgt tggcggcagt gctgctgttc gaccctttgg cggtcttggg 733380
tgtggggact tggctgtctt tcggtttggt ggcggccctg atatgggcgt gttcggggcg 733440
tttgcacgaa gggaaacggc aaaccgccgt gcgcgggcag tgggcggctt cggtgttgtc 733500
gctggttttg ctcggttatc tgtttgcttc gctgccttta atcagccctt tggtcaatgc 733560
ggtggcgatt ccgtggtttt cttgggtatt gacgccgctg gcgttgctgg gttcggtcgt 733620
gccgtttgcg cctttgcaac agttgggggc atttttggcg gaatatactt tgcggttttt 733680
ggtgtggctt gccgatgtgt cgcccgagtt tgccgttgcc gccgcacctt tgccgctgtt 733740
ggtgttggcg gtgtgtgccg ctttgctgtt gctgctgccg cgcggcttgg gtttgcgtcc 733800
gtgggcggtg ttgctgttgg cagggtttgt gttttaccgt tcacccggcg tgccggaaaa 733860
tgaggttgcg gttacggttt gggatgcggg gcagggtttg tcggtgtcgg ttcagacggc 733920
aaatcatcat cttttgtttg acactggaac tgcatcggcg gcacagacgg ggattgtgcc 733980
gagtttgaat gcggcgggtg tccgccgttt ggacaagctg gttctgtcgc atcacgacag 734040
cgaccacgac ggcggttttc gggcggtgag gaatattccc gccggcggga tttatgccgg 734100
gcagccggaa ttttatgagg gggcgcggca ttgtgcggaa cagcgttggc aatgggacgg 734160
cgtagatttc gagtttttga ggccgtctga acgcaaaaac atcgatgata atgggaaaag 734220
ttgtgttttg cgtgttgtgg cgggcggtgc ggcactgctg gtaacgggcg atttggatac 734280
gaagggcgag gaaagcctgg tcggcaagta tggaggcaac ctgtacagcc aggtgttggt 734340
gttggggcat cacggcagca atacgtcctc gtcgggcgtg ttcctcaatg ccgtttcgcc 734400
cgaatatgcc gttgcttcaa gcggttatgc caacgcctac aaacatccga ccgaagcggt 734460
gcagaaccgt gtccgcgcac acggcattaa actgctgcgt accgatttgt cgggtgcgct 734520
gcaattcggc ttgggacgcg gcggcgtgaa ggctcaacgt ttgagagggt ataaattcta 734580
ttggcagaaa aaaccgtttg agtgaggttt gaaacataaa atgccgtctg aaacggattc 734640
agacggcatt ttggcgttaa cgccggttcg tgctggcaag gcatatcgtt tgattttcag 734700
tgtgcgtcaa aaacagaaag ggcgttgcgg ttaacggcag ggagaaccgt ttgaatgaac 734760
ggaaaggttt gcgccagaag gggaaatgcc gtctgaaagg gcttcagacg gcatccggac 734820
atcggtgcgg aatcagtgcc agtaacgcca ccagggcata tcgtcagatc gccacggctg 734880
ctttaagaac gggcttttcg ggaagttggt ttccaacacg cggcgcgtat cggcggcaag 734940
ccggggcttg tccaacttct tgtaggcaag ttccaatatg gcgagcgatt cttcgacata 735000
gcgtgtattt tgatagctgc cgataatttt ttgggcgcgg ttggcggcgg cgatatatgc 735060
gccgcgtttc atgtagtaac gcgctaccga catttcattg ccgcccaaag catcgaccag 735120
tttgaccatg cgtgcggtcg catcggcggc gtatttgctg ttcgggaagc gttggacgag 735180
ttccgcaaag gcctgatacg cttcgcggtt ggctttcggg tcgcggtcgg accagtcttg 735240
cgaggccagc ttgttcaaga aagattgatc ttcgttgaac agtaccaaac cgcgcaggta 735300
tagcgcgtag tccatattcg ggtgttgagg gtgaaggcgg cggaagcggt caatggcggc 735360
cagcgcctta tccttctcat catctttata gtaggcgtat gccgtatcca gttgggattg 735420
ctgggcatgg cggctggtag ggaagcgcga ttccaagatt tcgtataatt tgacagctcg 735480
cgtataattg ctgctgttca gctcgtcctg ggcttcggca tagagttttt ccacactcca 735540
gtcttgggta atctgggcat ctttatctac cgtaccttga gtggcacagg cactcagtgc 735600
caaacctaat gaaaccgtta aaagaatttt tttcatgcag aatacttcct ttgataatga 735660
atccgattat agcgacgatt cagactttgc gtcagcttcc gaaactgaaa accgtatcgg 735720
tctgaccgtt ccgctcgagc ttgcaggcgg gcggttggat gcggtattgg cgaaacttct 735780
gcccgactac tcgcgcagcc gcctgacatc atggattaaa gaaggcgcgg ttattgtaaa 735840
cgataaacct tcgcaaccca aagacaaaat gataggcggc gagcaaattt gtgtaaccgt 735900
ccgtccgagt gaggaaaatc tggcgtttgt tccagagcct atggctttgg atattgttta 735960
cgaagacgat accgtcatcg tcgtcaacaa accggccgga ctggtggtgc atccggcggc 736020
gggcaactgg acggggacgc tgctcaacgg cctgttggcg cactgtcccg aattgagcca 736080
agtaccgcgc gcgggcatcg tacaccgttt ggacaaggaa accagcgggc tgatggtggt 736140
tgccaaaacc ctgccggcgc aaaattccct cgtgaggcag cttcaagagc gcacggtcaa 736200
acgcatctac cgcgccgtcg ccaacggcat cgtccccttc gacggtaaaa tcgaaaccca 736260
aatcggacgc gatccgcaca accgcctgaa aatggcagtc gtcaaattcg gcggcaaacc 736320
agccgttacc cacgtcaaag tgttggaacg ctatcttacc cacagctata tcgaatgctc 736380
gctcgaaacg ggcaggacgc accaaatccg cgtccatatg cgcgaggcca accatccgct 736440
tgccgccgac ccggtttacg gcaacccgcg ccatccgtgc ggcgacacgg tgaaagaagc 736500
cgttaaaagt ttgggtgcgc gtcaggcgtt gcacgcctac cgcttgagtt tcacccatcc 736560
ggaaagcggc gaaaccgttt cgtttgaagc accgattcca aacgacatat atcatttgtt 736620
atccgtcctc cgtcttgaag ccggtttgga ttcgtctttg agcaatgaag aagaatggca 736680
ggacaaattc ggcgcggacg acgacgatga ttggaacgaa gacgactatg atgtcgaagt 736740

ggtttatgta agggagtgag gcggcttgaa aggcggggcg aacgcaggca gccgaatcgg 736800
agcagccggg caatcgtccc cgccgatttc aaacaaaggc cgtctgaagg gaccgggcag 736860
aaaccgccgg ttttgtttgc cccgttcaga cggcattatg ataaaaggcg tttagggttt 736920
tttatgttta ccggctttgg ccgcccaata agttgccagc agcgagccgg agatattgtg 736980
ccacacgctg aacaatgcgc ccggaacggc aacgaccggc gcggcggcaa agtgtgcggc 737040
ggcaagcgcg gcggccaggc ccgagttttg cataccgact tcgatggtca gcgtttttttg 737100
tgcatcataa ggcaggccgg tccatttggc ggcaaagaag ccgagcaggt agccgatgcc 737160
gttgtggagt acgacaaccg caaaaatcag caggccgctt tccataatct tgcctttgct 737220
tgccccaaca accgcgccga taatcagcac gatggcggca acggaaacca gcggcagcgc 737280
atcggtcagc ttttcggttt tactgcccaa aaccttatgg acaatcaaac ccaaaacaat 737340
ggggagcaaa accattttga cgatggacat caacataccg gccgcttgga tttccagcat 737400
ttcgccggca agcatcagga agatggcggg agtcagcaat ggggaaatca gggtggaaac 737460
agacgtaacg gcaaccgaca aagccacatt gccacgcgcc agataggtca tcacattgga 737520
agccgtaccg cccgggcagc agccgaccaa aatcacgccg accgcgattt cggcaggcag 737580
gttcaacagt ttggacagca gccaggcggt tgccggcata atggcgaatt gtgcgattac 737640
gccgatgatg acgactttgg gatgtttgaa caaaatatcg aagtcggaag gtttgagcgt 737700
caaacccata ccgaacataa taatgcccaa cagccaagga atataaggcc ccgcccattt 737760
gaaggtgtcg ggcgcgaaaa aagcggcggc ggcaaagagc gcggcccaga gggaaaatgt 737820
ttttccgata aagctgctga ttttactgag gatattcata aataatgcgt tgcgtgtttg 737880
tttttaaggg aaggcaagca tacacgcctt aaccttaatt tgcaaaatga ccgtgcctaa 737940
acaatgccgt ctgaaagtgg agattggttt tcagacggca tcgcccgaga gatgtcggaa 738000
atggacttta tccccattcc ttttcggttg aaacccgtct gtttatggcg atagaatcta 738060
atcggagggt agtctcgttc gggcaacacg cagtgcggtg cttgatgtgc cgtcccctgt 738120
tgaaacatat aaagctcgga gaaagtatag tggattaaat ttaaaccagt acggcgttgc 738180
ctcgccttgc cgtactattt gtactgtctg cggcttcgtc gccttgtcct gatttaaatt 738240
taatccacta tatataaggg catcattcct gcaccggcaa gaatccgaac ccgaacgttt 738300
gaaaacaatc ccgaatctcc gaattcccgc ctgtgtggga atgacgaaaa aacaagcatt 738360
catttgcccc gaaggcagtt aatcaaccct ttccgccaca cacctattcc aatatccaat 738420
gaaaaccatc acagaaaccc taaatctcgc cccgaaaggc aaaaacttcc tgaccgccga 738480
ttggcccgcg cccgccaatg tgaaaaccct gattaccacg cgcaacggcg gcgtgagcca 738540
aggtgcgtat cagagtttga acctcggtac gcacgtcggc gacaatcccg aagccgtgcg 738600
ccgcaaccgc gaaatcgtgc aacagcaggt cggactgccc gttgcctacc tcaatcaaat 738660
ccacagcacc gtcgtcgtca atgctgccga agcgttggga ggcacacccg atgcggacgc 738720
ttccgtagac gacacgggca aggttgcctg tgccgtgatg accgcagact gcctgcccgt 738780
tctattttgc gacagggcgg gtacggcggt tgccgccgca cacgcgggct ggcgcggttt 738840
ggcgggcggc gtactgcaaa acaccatagc cgcaatgaag gttccgcccg tcgaaatgat 738900
ggcgtatctc ggccccgcca tcagtgcgga tgcgtttgaa gtcggacagg atgtgtttga 738960
tgcgttctgc acgcccatgc ccgaagccgc caccgcattt gaaggcatag gcagcggcaa 739020
attccttgcc gacctttacg cgctcgcccg cctgattctg aagcgcgaag gcgtgggcgg 739080
cgtatatggc ggcacgcatt gtacggtttt ggaacgggat actttctttt cctaccgccg 739140
cgacggagcg acagggcgta tggcgagcct gatttggctg gacggcaatg ccgtctgaac 739200
acgccgctga tataatctac cgactttgtg tttttgagaa aggcaagcca tgaacaaact 739260
gtttcttact gccgcagtgc tgatgctggg cgcgtgcggt ttccacctga aaggtgcaga 739320
cggcatttct ccgccgctga cctaccggag ctggcacatc gaaggcggac aggcattgcg 739380
gtttcctttg gaaaccgcgc tgtatcaggc ttcgggcagg gtggacgatg ctgccggcgc 739440
gcagatgacc ctgcgtatag acagcgtttc ccaaaacaag gaaacctaca ccgttacccg 739500
tgcggcagtc atcaacgaat atcttttgat attgacggtt gaagcgcagg tattgaaacg 739560
cggcgagccg gtcggtaaac cgatgaccgt gtccgtccgc cgcgtccttg cttatgccga 739620
caacgagatc ttgggcaaac aggaagagga agcggcattg tgggcggaaa tgcggcagga 739680
tgccgccgaa cagattgtcc gccgcctgac ctttctgaag gcggaatgac gtggcggcac 739740
atatcggacg cattgatacg gacgcgcctt tgaaacccct gtacgtcatc cacggcgagg 739800
aagaactgtt gcgtatcgag gcattggacg cattgagggc ggcggcgaag aaacaaggtt 739860
accttaatcg ggaagtttat acggcagaca atgccttcga ttggaacgag ctgctgcaaa 739920
ccgcaggcag tgcgggtctg tttgccgatt tgaagctgtt ggaactgcat atccctaacg 739980
gcaagcccgg caaaaccggc ggcgaggcgt tgcaggattt tgccgcccga ttgccggaag 740040
atacggtaac gctggttttg ctgcccaaac tggagaaaac ccagctccag tccaaatggt 740100
ttgccgcatt ggcggcaaag ggggaagtgt gggaagccaa accggtcggc gcggcggctt 740160
tgccccaatg gatacgcgga cggctggaca aaatcggttt gggtatcgag gcagacgcat 740220
tggcactgtt tgctgagcgc gtggaaggca atctgttggc ggcgcgtcag gaaatcgaca 740280
agctcgggct gctgtatccg aaagggcata ccgtcaatat cgatgaggcg caaaccgccg 740340
ttgccaacgt cgcccgcttc gacgcgttcc aactggcagg cgcgtggatg aagggcgatg 740400

tcctgcgcgt atgcaggctt ttggacggat tgcgggaaga gggcgaagaa ccggtgctgt 740460
tgctgtgggc ggttgccgaa gacgtgcgga cgctgatccg gcttgctgcc gccctgaagc 740520
aggggcagag catccaatcc gtccgcaaca gcctcaggct ttggggcgac aagcagacgc 740580
tcgcaccgct tgcggtcaag cggatttccg tcgtccgcct gcttgacgcg ctcaaaacct 740640
gcgcccaaat cgaccgaatc atcaaaggtg cggaagacgg cgacgcatgg acggtattca 740700
aacggcttgt cgtgtcgctg gcggaataaa gcggtaatcc ccaaaatccg aaaatactgt 740760
aaaataccgt taatcctgaa aagtattcac caatccgtcc gaaaacattt cagacggcac 740820
gaccacctca ataaaggaac attaacccta tggacaataa gaccaaactg cgcttgggcg 740880
gcctgatttt actgaccacc gccgttttaa gcctcattat cgtattgatt gtcgattcct 740940
ggccgcttgc catcctgctt gcagccgtca ttgtcgctgc cgctgcgggc ggttttgttt 741000
ggacatcccg ccgacagcaa cgccagttta tcgaacgcct gaaaaaattc gacatcgatc 741060
ccgaaaaagg cagaatcaac gaggcaaacc tgcgccgtat gtaccacagc ggcggacaac 741120
accagaaaga tgcgattacc ctgatctgcc tgtcgcaaaa atgttcggtg gacgaggcgc 741180
acgctatgtt caaaaaacgc ccgacacgtc aggaaatcaa tcaaatggcg gcaaaacagt 741240
cgcgcggtca gaaacgtccg caccgttaac cgccgcaagg catctttgca taaatgccgt 741300
ctgaagcctg ttggcgtttc agacggcata ttctgattga aagatgatg acactgaaaa 741360
ccgccccgct caaacgccgc tttgccgcca tgctgtacga aatgctgctg gtcggtgcgg 741420
caacctgttt ggcagcattg attgccggta ttgccgccat ttttctgaat cccgtttcta 741480
tcgcggtttc tgcattggta acaagtatcc tgataatggg agcatggtgg ctttatttcc 741540
gcgccaactg gcatggtcag gggcagacct tggcgatgag gacatggaaa atcggcttgt 741600
gcgaccttaa cggcatacag ccgtctttgc acctgctgcg cctgcgcttt atttgggcgt 741660
gcatatttat cgtatttatc cctatgttag cctatgccgg attacgccac ttcctcggca 741720
ttccgcccaa gggcgcggcc ggcgcggcat tgatttggct gattttaccg tgggggttcg 741780
cactgctgaa tcccgatcgg cagtttctgt atgatttttct tgcaggaaca agattggtgg 741840
cggtcaaagg aaagccttaa gcctttatac cgcaaaggtt tcaacctgaa aaaatgccgt 741900
ctgaaagggc tttcagacgg aatttgctta tcggggaaac cgattattcg atattctgca 741960
cttgttcccg catctgctcg attaagactt tcagttcgac cgaggcttgg gtgcattcgg 742020
cggcaatgga tttgctgccc aaagtgttgg cttcgcggtt taattcctgc atcaggaagt 742080
ccagccgttt gccgctgctg cctttgtgtt cggtaacgat acggcgcact tcggcaatgt 742140
gggtgcgtag cggctgaact cttcgtcgat gtcggatttt tggataaaga gggcaaattc 742200
ctgttgcagg cggtcgttgt cgatgctgcc gaccgcttcg acgaggcggg cgcggatttt 742260
ttctttatgt gtttccaaca gggtaggaaa gagttcgctt aatgcatcta tgatttcttc 742320
catagcctca aggcgttgca gcaggtgctc gcctaatttc ttaccttccc gcttgcgtgc 742380
ggcagtaaag tcttttaacg ctttttcggt cagttcggta atgctttttg ccaattcttc 742440
cgtattttcc ctttggcttg ccaatacgcc ggggaaacgc aggatgtcgg caacgcccag 742500
ttttgccaaa tcgtgatgct tgcggaggtc tttgttgatt tcggcaagct gtccgaccaa 742560
gtcgcgattc agttccaagg actgactgcc gttttccgca tcttgaattt ggattttgca 742620
ttcgactttg ccgcgtgcga tatgggatga aattttctcg cggataccgc tttccaaata 742680
gcgcaaatcg tcgggcatcc tgatttgaat atctagaaaa cggtggttga ccgcacgcag 742740
gtcgagattg atgcgtttgc tgccgcactc tgccgccgcg ttggcaaatc cggtcatgct 742800
gtggatgtgg atatttccgc tgctcatgtc gttctccgaa gcccgttaaa atggaatcaa 742860
tatatcacat ctgtatggcg gcaagcgttt tcgggtgtga aaaattgaag attttgcagc 742920
ggcagattgg aatcacgcgc ttttgttgct gcaaggaagg gaaatgtata gtggattaac 742980
caaaaccagt acggcgttgc ctcgccttag ctcaaagaga acgattctct aaggtgctga 743040
agcaccaagt gaatcggttc cgtacgattt gtactgtctg cggcttcgcc gccttgtcct 743100
gatttttgtt aatccactat atcaattccg ccaatctgtc ggaaaagcag ctgatgcggc 743160
agtgtctggt gcatgtctgc tttttgattt cggcaattgc aacggcgtgg acggataaaa 743220
tcgtgtacag cacgacgcac aaaccgcatt gatgtttacc aaataaaata cccgacaaaa 743280
caatttgtcg ggtattttat tgcgtatatt tcaaaccgct tcggcttctt cggtcaggaa 743340
accacgcagt ttctgcatgg cttttgcttc gatttggcgg atgcgttcgg cagatacgcc 743400
gtattcggcg gcaagctggt gcagcgtcag cccgccgtcg tcttgaagcc agcggctttc 743460
cacaatacgg cggctcctgt catccagttg cgccaaagcg ttttgtaaac cttctgtttg 743520
cagggcgtaa tgcgcctgtt tcgatagttg tcggctcggt tcggaatcgt ggtcggcaag 743580
ccagtcgatg gggcgaaac tatcctcgtc gtcgctgttg tctgccatga tggcgatgtc 743640
gtgtcccgtc attcgctgtt ccatttccag aacttcggaa agtttgacac ccaaatcgtc 743700
ggcgatgtct tgtgcctctt tgggagacag ggcgttgagg tttttacgca tgctgcgcag 743760
gttgaaaaac agcttgcgtt gcggtttggt ggtggcaacg cgaaccaaac gccagtttct 743820
caaaataaac tcgtggattt cggctttaat ccagtgtacg gcaaatgaaa acagacgcgc 743880
gcctctaccg ggctcgtagc gtttgaccgc cttcatcagt ccgatattgc cttcctgaat 743940
caggtctgcc tgattcagcc cgtagccgtc atagccgcgc gcgatggaaa cgacgacgcg 744000
caggtgggac aggatgagtt gtttggcggc gttgaggtcg cctttgtgtt ggcgttcggc 744060

aaggcgtgtt tcttcctctt gggtcagcat gggaattctg ttgacggtgt ggatgtattg 744120
ttcgaggctg ccgttgccgc tttggatggc gggtaatgcg aaagcgttat tcatttggga 744180
catttccttt cggctgaaac tgcgtatcgg cggtttgctg tgttgggatg cagtatatca 744240
ctgcttggct tgtattttgt atatttggca ggagatatgc gctaaggttt gaaagacagg 744300
aaaaattttg taaggcaagt ttgattgatt ttgtaaacct gatggctcaa ttcgattttg 744360
gaattatatt acatacgtgg ttgtatgtaa atagccgttt tgaaaaaaga cagcccgtcc 744420
ggacgggctg tgcaggtatc agtgttcttt gtttcggaag atgaaaagaa tcagtgcggc 744480
tagggccaat atgcccatca accaccatga actgccggtt ttcatatagg gcgtttcgcc 744540
gacatagcct ttgatgtgtc cttccaatac ggtttccgta tcgggttggg cttgggcgat 744600
gatgttgcct ttgggggaga tgatggcggt tgcgccggtg ttggtggcgc ggaccatata 744660
gcgtccgagt tccatagccc gcgcctgcga ttgttggagg tgctggtaca tggcgttgga 744720
ttttccgtac cacgccatat tgctggcatt ggcaagcagg gtggcatctt ttgcggcggc 744780
aatcagttcg tcgccgaatc cgtcttcgta acagatgttg aaggcgattt tttggttttt 744840
catcagcagg gcggattgct tgccgccgcc tttgcggaag tcggaaaggg gcatatccat 744900
cattttgtaa agcggcgtgg tcaggaaagg cagcggtttg tattcgccga aggggacgag 744960
gtggtttttg gcgtagtagg ggataccgtc ctgattgttt tcctgataac cggtcaggtt 745020
gatgacggcg ttttcgtaac cgttgccgtc cgaagtgtat tggctgatgc cgacggcgag 745080
cgcgctgccg ttgttttgcg cctgttcggc aaatttcgcc agtatgtttt ccggcaggtt 745140
ttggcgcata acggggatgg cggtttcggg caggatgacg atgtcggcgg tggttttgcc 745200
gacttgttcg taatatttct gtatggtcgg gataacttgg tcttcacgcc atttgagggt 745260
ttggtcgatg ttgccttgaa gcagggcgac ggtgctgcgg ctgccgtcgg ggcgggtgaa 745320
gtcggtttgt cgggcggtgt agcctgcggc aagcagggcg gcaatcagga taatcggaag 745380
caggcgtttg cccgaacgtg cggtgttatt actcgccaaa accagccaga caccgagaaa 745440
ggcggttgcc agtgtaacca tgtggatgcc gcccaatggg gcaaagccgg cgagcgggct 745500
gtccggggtg atttgggagt agccgattgc gccccagccg aatccggtca ggaaacgttc 745560
gcgggcaaac tcggtcagcg tccacaggat gggcagtacc aaaccgattt ttatgccccg 745620
aggcagggta aatttttttcc acagccagaa acacagtgcc ggataaaggg caaggtaggc 745680
ggggagtagg aaggtcagcg gtacggcata gaggtcgggc aggccggaaa cgtcgtgcag 745740
ggcggtgtgt atccagtaga actgtgtcgt gtatgcggtc aggccgaaca ggtaggcgga 745800
agagacagca aaacgcggac gcagttcgat gaggcggacg aaggcaccga aaatcaaggg 745860
catcagccaa aagtggtagt agggtgcgaa ggtaaagggg gtggcggcgg caaaaaggat 745920
gagcaaaggc cagtagaggg cggggtgctg ccagtatttg tccagtttgg aaaccatatt 745980
catctgtctg ttcggaagat accgtctgaa catctttcaa acggcatcgg tatttgaaaa 746040
aggaatcaat gcctgccgaa acgattcatc agcggcaagg cgggggcgca ggcaatcgaa 746100
ccggaaagca gcccgacaac gaggttggcg aagtactccg cccagccagc gtcccagtgt 746160
tgcgcgtgca ggaaatcgtg cagaaggccg aggttgtggg cgaccagtac gccgccgata 746220
agaaacatgg caagcgtgcc gaccacgctc aaaccgcgca taaagcaagg cataaaggca 746280
gtcagcattt gccccaaact gcgcgaaaag gtttgtgggc ggcgcatcag cagcatgcct 746340
aagtcgtcga gtttgacgat gacggcaacg attccgtaca ccaaaacagt catgccgatg 746400
ccgattgccg ccattacgag catgcgcgtc atgtcggtgc agaaacttgt gcagcagctt 746460
ttctacgcct tcaaagcaca gataaatgcc gcctgccgtc aaaagcggcg taatgagttg 746520
cggcaggaag gcggaaagca gcagggccgc aggcaccaaa accggcttgt tggaaaaaga 746580
acctttcgcc atcgaccaaa caatcggcaa ctcgcgttct gccgatacgc ccgtaacccg 746640
gttggcattg ggtgccaaat cgtcgccgac cacgccggcg gttttctttg cggcggcttt 746700
ggtcatcagg gcaacatcgt ccaaaacggc ggtgatgtcg tccggcaggg taaatagtga 746760
ggcaaatgcc attaaagaat cctgaaatgc ggcgcaaagt ccgacattat ataggagaac 746820
gcggatttgg gcggtttcag gcggcatgaa acaggaaaat gccgtctgaa cgctgtggcg 746880
gacgtgaagt aaagtttcgt gaaaagaaaa taccgtgtta cagtctttcg attttaattt 746940
catgaatttt aagggagaat cgttagcgtg gattggatgg gcagtctgtt cctgccgggt 747000
ggcgcactgt tgtttctgag cgtggtttcg accactttgt ccgcacgttt gggaatgcct 747060
ttgctgctgg tttctcctgc caacgtgttg gacagggcgg cggaagcctt ggcgattgcg 747120
gcgttcctga tgctggtcgc gcgtccgtcg gcagtgttcg gcggtttgtg gaaattcaat 747180
tacagcctgc gtgaaaaggc gtatagccga atagaaatgc agtccgacac cgtgcttcag 747240
gcggggattt ggcgtggtac atcctgcccg acggcaaggt cgatatagtg aattaacaaa 747300
aatcaggaca aggcggcgag ccgcagacag tacaaatagt acggaaccga ttcacttggt 747360
gcttcagcac cttagagaat cgttctcttt gagctaaggc gaggcaacgc cgtactggtt 747420
taaatttagt tcactataaa atggcgaaat actttaccga gacgggtatt agcgtccgtg 747480
agcattttga tttcttcggt gagtttgtcg tttcgccggc agcacgttcg ggtgatttgg 747540
cacttactta cggtttgagg ctggaagcgg gcgaagaggg tttgagcctt gccgagcttt 747600
tcgataagcg ttccgatagt caggagccgg tcgagggcgg ccgtattgac atcggcggct 747660
ttatgctgac cgcaaaggag gttgacggtg gcggcaatat cgggtctatg ggctgaaag 747720

tgctgcgtta gaaaggtttg atttgaatgc cgtctgaagc cggattgccg gtttcagacg 747780
gcattttgtc tgtttagttt tttttgcttt ttgcctgttt tacgtctttt tcggtaacgc 747840
ttccgccgcc gttgtcaaag gcgttcatga tataagtggc gacggcggca atgtccgcat 747900
cgctgatggc ggttgcgggc atgaatccgt tgtaggtttt gccgttgact ttgattgtac 747960
cgttgatgcc tttgaccatg ctgtgcagca gcacctgcgg ttttttcatg atgaagtcgg 748020
agcggtagag cggcggaaac atggttccgc ggccttcgcc ctttttgccg tggcaggcga 748080
cgcagttgga ttcgtacact ttttgccctt ttgtcatgat gctgttgtcg gcggcagaag 748140
cggcggcgca gaagcagccc aagacgaggg cggtcggcag tcgggttgtg ttcattggtg 748200
tttccttcat gtttgaaacc ttgttgttga ttttgcgtag cgggtgaaag atttttttgc 748260
cgaatcagta gtatagtgga ttaacaaaaa tcaggataag gcgacgaagc cgcagacagt 748320
acaaatagta cggcaaggcg aggcaacgcc gtactggttt aaatttaatc cactataagg 748380
ttgcacttga tgttgttgtc cagcatagat gccatcatac gctaaagtag cgggaaaatg 748440
ccgtctgaac acggcgttca gacggcattt tagacatggg tcaaacagtt tcaacgccag 748500
ctgccaaggt ttcttcggc aagtgcgacg agtgcatcta tccagtcggg gttgtcgttg 748560
aggcagggga tgtagcggta gcttttgccg cctgcttcat aaaactgttc ccgccccatc 748620
agggcgattt cttccatggt ttccaaacag tctgccaaaa agcccgggca aaatacgtcc 748680
agctcggtta cccccctgttt gggcagtttg ccgaacaaat cctgcgtgct cggtgtaacc 748740
cattttgccc tgccgaattg gctttggaac gatacgacat attggtcttc ggtcagttcc 748800
agtgcttcgg caagcagttt ggcggtgtgg cggcactcgt cgggataggg gtcgcccgagg 748860
tcgtggtgct tctgcggtac gccgtgaaaa ctcaacatca gttttttccc gcgcccgtgt 748920
tccgcccaat atcggaggat gtggtttttc atcgcatcaa tgtagccggt atcgtcataa 748980
aagcgcgaaa cggtgcggac gctcatttgg ttccgttgca gcagtaattg ttcgcacacc 749040
ttatctactg ccgctccgct gctggaagcg gcatattgcg ggtacatcgg gatgaccagc 749100
agtctgcccg cgccttgcgc cttcagttcc gacaatacgt ctgccaccga aggattgccg 749160
taggtcatgg cgtggcggac gatgaggtcg ggcatacgtt tggcaagcgc ggcagcttgg 749220
cgtgctgtgt aaacttctaa gggcgaacct tccttaaacc agattttttc ataggcgtgc 749280
gcgctttttt tggggcggag cgtcagtacc agaccatgca gaatgggata ccacagccat 749340
ttgggcagtt cgacgacgcg ccggtcggtc agaaaggact tcagataagg tcgtaccgcc 749400
tgcgcggtcg gcgcgtcggg cgtgccgagg ttcaacagca aaacggcggt acggttttgt 749460
tgcgtatagg aaagggaggg ttctggaaag aatggaagca tgatcggttt ctgaaaaata 749520
gtgcgggtag ggtaaagcgg caaaatgccg tctgaagcgg cttcagacgg cattgcaggg 749580
aatcagtctg tgccgcgtgc gcggttttcg tggaatcgcg cctgccagtc ggcaaatttg 749640
ccttgttcga cggcttcgcg catttccgcc ataatgactt ggtagaaatg cagattgtgg 749700
atggtgttca actgtgcgcc caagatttcg ccggtgcggt gcagatggtg caggtaggcg 749760
cggctgaagt tttggcaggc gtagcaggtg cagctttcgt ctatcggacg cttgtcgagc 749820
ttgtgtttgg cgtttttgat tttcaaatcg ccgaaacggg taaacagcca gccgttgcgt 749880
gcattgcggg tgggcatcac gcagtcgaac atatcgatgc cgtgtgccac gccgtacacg 749940
aggtcttccg gcgtgcctac gcccatcagg taatgcggct tgtgttccgg cagaatcgga 750000
ccgacggcgc gcagcatacg gtacatttcg ggcttgggtt cgccgacgga caaaccgccg 750060
acggcaaggc cgggaaaatc aaactgttcc aaaccgcgca gcgattcttc gcgcaaatcc 750120
tcatacatcg cgccttgcac gatgccgaac agcgcgttcg ggtttttcaa atcttcaaag 750180
gctttttgc tccgttccgc ccagcgcagg ctcatttgca gcgattttcg cgcctgttcg 750240
cgcgtcgcct cgcccggcgt gcattcgtcc aactgcatcg cgatatccga gttcaaaacc 750300
gtttggattt tcatggaaat ttcaggcgat aaaaacagct tgtcgccgtt aatcgggctt 750360
ttgaacgtac agccttcttc cgtcagcttg cgcatatccg acaaagaaaa aacctgaaaa 750420
ccgcccgagt cggtcagaat cggtttgtcc cagccgataa aaccgtgcag gccgccgaat 750480
tgcccgataa cttccaaacc cggacgcagc cacaaatgat aagtgttgcc caaaataatt 750540
tgtgccttga tatcgtgcag gttttgcggg ttcatcgcct taaccgaacc gtaagtaccg 750600
acaggcataa ataccggcgt ttcaattttg ccgtggttca actccagcgt gccgcgtcgg 750660
gcgagaccgt ctttttgtg taaggtaaat ttaagcataa gattgaatgt cagttgggcg 750720
acaggggtcg aaatatattt taaaagacgg cattataaat gatttcccac ggtttttcag 750780
acgacatccc caaatcttgc cgcaatgttg cataaagaaa cgcacatacc tcttgcaaaa 750840
attaaaacga cccgataaaa tgcaaaaatt ctttgaaggc acgtagctca gttggttaga 750900
gcaccacctt gacatggtgg gggtcgttgg ttcgaatcca atcgtgccta ccaaattccc 750960
ataacggcat ttatgccgtt attttttaat ctttcggagc gtttgatgtt gaatattacc 751020
ttgccggacg gctcagtccg ccaatacgaa tccccgtta ccgtggctca aattgctgcg 751080
tctatcggtg ccggtttggc gaaggcgacg gtggcaggca gggtaaacgg caaattggtc 751140
gatgcgtgcg acccgattgt tgaagattct gctgttcaaa tcattactcc gaaagatcag 751200
gaaggcatcg aaatcatccg ccattcctgc gcgcatcttg tcgggcatgc cgtcaagcaa 751260
ctctatccta atgcaaaaat ggttatcggc cccgtcattg aagagggctt ttattacgac 751320
atcgccacgg aaaaaccgtt tacaccggaa gatgttgccg ccattgaagc gcgtatgaaa 751380

gaattgattg cccaagacta tgatgtggtc aaaatcatga ctccgcgtgc ggaggcgatt 751440
aaaatttttc aagagcgcgg cgaagaatac aaactgcgcc tgattgacga tatgcccgaa 751500
gtggaagcga tggggatgta tcatcaccag gaatatgtcg atatgtgccg cggcccgcac 751560
gttccgaata cccgtttcct gaaaaacttc aaactgacca agttggcggg cgcatactgg 751620
cgcggcgaca gcaataatga aatgctgcaa cgcatatacg gtacggcttg ggcgacaaaa 751680
gacgaattaa aagcctatat tcaacgtatc gaagaagccg aaaagcgcga ccaccgcaaa 751740
cttggcaagc aattggatct gttccacctg caagacgaag cgccgggcat ggtgttttgg 751800
catcctaaag gctgggcttt gtggcaagtg attgaacagc atatgcgtaa agagctgaac 751860
gccgccggtt ataaagaggt caaaacgcct caaatcatgg ataaaacctt ttgggaaaaa 751920
tccggccatt gggacaacta caaagataat atgttcgtaa ccagttcgga aaaacgcgaa 751980
tatgcggtta aaccgatgaa ctgtccgggt catgttcaaa tttttaacaa cggtttgcgt 752040
tcgtatcgag atttgccgat gcgtttggcg gaattcggtt cttgccaccg caatgagccg 752100
agcggtgcgc tgcacggtct gatgcgggtt cgcggttttg tgcaggatga tgcgcatatt 752160
ttctgtaccg aagatcaaat cgtcagcgag gctcgtgcgt tcaatgaatt gttgattcgc 752220
atctacaaac agttcggttt ccatgatgta tccgtcaagc tttctcttcg ccctgaaaaa 752280
cgcgcaggtt cagatgacgt gtgggataag gcagagcagg gtttgcgcga ggcattgact 752340
gcctgcggcg tggaatgggg cgaattgccg ggcgagggtg cgttttacgg gcctaaaatc 752400
gaatatcatg tcagagatgc cttgggtcgt tcttggcaat gcggtacatt acaactggat 752460
ttcgtattgc cggagcgttt gaatgccgaa tatgtaactg aaaacaacga ccgtgcgcgt 752520
cctgttatgt tgcatcgcgc cattttaggt tctttggagc ggtttatcgg cattctgatt 752580
gagaaccatg caggctcatt cccgttatgg ttggctccgg ttcaattggt aattatgaat 752640
attaccgaaa atcaggcaga ttattgtcgg gaagtggctg ccaaattgca ggcggcagga 752700
ttccgcgccg agttggattt gcgtaacgaa aaaatcggtt acaaaatccg cgacaacagc 752760
caataccgtt tcccttatca aatcgttgtc ggcgataagg agaagcagga aaacaaagtg 752820
gcggtacgcc gcaaagcaga agatttgggt tctttggatt tggatgattt cattgcgcaa 752880
ttgcagcaag aaatcactga tgccctcgtc aatcattaat ttttatagga gtattcatca 752940
tcgctcaaga acgcgaagca cgaatcaacg gcgaaattac cgccaaagaa gtgcgtttaa 753000
tcagtgagtc aggcgaacag cttggtgtcg tttcagttcg tgaagctttg gctatggccg 753060
aagggcagga tgtcgatttg gtagagattt ccccaactgc taaaccgcct gtgtgcaaac 753120
tgatggatta cggtaaatac aaataccagc aggccaagaa acgcgacgaa gccaagaaaa 753180
accaaaagca ggtgcaaatc aaggaaatca aattccgtcc gggtacggat gagggcgatt 753240
atcaaatcaa gatgcgcaac attaaccgct tccttgccga cggcgataaa gtcaaagtga 753300
cattgcgttt ccgcggccgt gaaatggctc accagcaact cggcgcgcaa cttttggaac 753360
gtgtaaaaga agatttggct gaagtggcgc aaatcgagtc ctttcccaaa atggaaggtc 753420
gccaaatggt gatgatgatt gcgccgaaga aaaaataaag ctataattct ccgcttactc 753480
cgattgccgc ttcggagtaa gttttcaatt gcggcaaaaa accgtgtcat tgtgggttca 753540
agtgtttgaa accgatgttt taaaaccccc taatgcctta tccgataacg aatggagttt 753600
tcccatgcct aaaatgaaaa ccaagtctag cgcgaaaaaa cgctttaaag tactgggtaa 753660
cggcggtgtg aaacgcgctc atgcgttcaa acgccacatc ttgactaaaa agaccaccaa 753720
aaacaaacgc caactgcgcg gtacctctat ggtaaatgat cgcgatttgg cttctgttgc 753780
taaaatgtta ccctacgctt aaggagttta gaatatgcca cgcgtaaaac gcggtgttac 753840
cgctcgtgcc cgtcaccaaa aaatcttcgc gttagccaaa ggctaccgcg gccgtcgtaa 753900
aaacgtttac cgcgttgcca agcaggcggt aatgaaagcc ggtcaatacg cgtaccgtga 753960
ccgccgccaa cgcaaacgcc aattccgtca attgtggatt gtccgtatca atgcaggtac 754020
gcgtgaaaac gggttgtctt acagcaaatt tatgaacggt ctgaaacgcg cctctattga 754080
aatcgaccgc aaagtattgg ctgatttggc cgtgttcgat aaagccgctt ttgcacaatt 754140
ggttgaaaaa gccaaagctg ctttggctgc ttaatccaaa aaattgaaaa ggaagctgcg 754200
gcttcctttt ttctttgttt gcagaaattc tatgtgattg attttctttc tttaaagtct 754260
atttttttaa ataaatttgc gttaaaatat agtggattaa atttaaatca ggacaaggcg 754320
acgaagccgc agacagtaca gatagtacgg caaggcgagg caacgccgta ctggtttaaa 754380
tttaatccac tatacagaaa atttatccaa tggattgacc gtgaagaaaa taaggtcgtc 754440
tgaagagtct gatatgtcag gctatacagg cggcctcgtt gtttcaggtg gtatatcatt 754500
aattgacaga cttgatatta tggaaaatgt aaaccgcatc gttgcagaag gcattgccgc 754560
agtagaagct gcgcaagact tcaacgctct agaacaaatc aaagcccgtt atcttggtaa 754620
aaccggcgag ttgaccggac ttctgaaaac tttggggcaa atgtcgcctg aagagcgcaa 754680
aaccataggt gcgcatatca atgaatgcaa aaaccggttt cagacggctt ttaatgccaa 754740
acgcgaagcc ctcaacgaag tcaagctgca agcccgactt gccgccgaag ccctcgatat 754800
taccctgccc ggacgcgctc aggaaggcgg cagcctgcat cccgtaaccc tgaccttgca 754860
acgtgtggtc gaactctttc acggaatggg tttcgaagtg gcggacgggc ctgaaatcga 754920
agacgatttt cacaatttcc aagccctgaa catccctgca aaccatcctg cccgtgcgat 754980
gcaggatacg ttttacgttg aaaacggcga tgttttgcgt acgcacactt ccccgattca 755040

aatccgctat atgctcgata aaaaagagcc gcccatccgc attatcgccc ccggccgcgt 755100
ttaccgtgtg gacagcgatg ccacgcactc gcctatgttc catcaggcgg aaggtttgtg 755160
ggtagaagag ggcgtaactt ttgccgactt aaaagcagtg ttcacggatt ttatccgtcg 755220
cttctttgaa cgcgatgatt tgcaagtacg tttccgcccg tctttcttcc cgttcaccga 755280
accgtctgcc gaaatcgaca ttatgggcga aaacggcaaa tggctggaag taggcggttg 755340
cggtatggta catcctaacg tgttgaaaaa cgtcaatatc gaccctgaaa aatataccgg 755400
tttcgccttt ggtattggtc tcgaccgctt cgctatgctg cgttacaacg tgaacgactt 755460
gcgcctgttc ttcgataatg atttgaactt tttgaagcag tttgcgaaat gatcgtgcag 755520
actgcctgaa tatggaaaag cagcctactc ttggttttca ggctgcttag gaaaattcaa 755580
atgtaagata taaaacattt gatattttgt tgtgaaatta cattcctaat tttgtttaaa 755640
gaggcataat ttattgcttt gtagagatta tatagttaat ttgggtttgg ttctatgatg 755700
ataggggctt ctttgttttc gagtgcaggg attgcagaaa cctacttgca taatgcgggt 755760
attaagatta tagctgcaaa tgaattggtg ccagaacgtg ctaatttata taaagctcta 755820
tatcccgaaa gtaaaatgat tataggtgat atacttcatg aggaagtgtt tcaaaattta 755880
atacagagcg tgccgaatcg attagatttt ttaattgctt ctcctccttg tcaaggcatg 755940
agtgttgcag ggaaaaatcg taacattcaa gagatggcta atgataaacg taatcattta 756000
atattcagag taatcgaatt tattttatta aaacgcccaa cttttgtttt gattgaaaat 756060
gttccatttt ttttaaaaat taagttacct tataagggga cattacaaac agtagaagta 756120
attttgcaag atttatttgg ttgcgaatat tatattcaaa ctcatatttt tgattctgcc 756180
gattatggtg ttgcacaaca tcgtaaacga gctattattc gtatgaataa acattcaact 756240
atttggggaa tgccggaaaa agttacaaaa accatttctg ttcgtgatgc tattagtttt 756300
ttgcctagta ttgagtctgg acaaaagtct aatgtgaaat ggcattttgc acgtacacat 756360
gctccggagc acattatatg gctaaaaaat acgccaacag gacgatctgc ttttgataat 756420
atagaacatt atccaaagaa aaaaaatggt gaaaaaatta aaagttataa tacaacttat 756480
cgccgtatgg agtgggatgc tcctgcccca actattacta ttcgtaatga cgctatcagt 756540
tcacaattaa atgttcatcc tggacggtct atgcctgatg gaacatattc agatgcaaga 756600
gttttaacac cattagagtt aatgctttta acatcattac ctattgattg gaatattccc 756660
gacgatacat cagaattatt aattcggcaa tgtattggtg aatctattcc tccattgtta 756720
attaaaaaaa ttgtagagag aataggaaaa tagatatgac aactgcgcgc tgggtaatag 756780
ataaacattt acagaatttt catattttat gtaaatttgc aggtattttg aaaacaaatt 756840
cttttatatc tgtagaggat aaagctaagt tatctgaaaa attggaaaaa ctagatttat 756900
accatagacg aaatacaggt aaatcattgg atgctactac tcataaaata aaagaattat 756960
cattctatat gtttggttat cgtgatgtgt gtgggcaagt tacacagaaa ttcctgttca 757020
gtccattggg taatttattt ttgaaacact tggataataa tgaatatatt caaaaaattt 757080
ttcttactat gttgtgggcg ataccatttc ctcatccgta cattaagact gatgaaagta 757140
ttcaattata tcccatgaga ctaatattta agttgttatc tgatgaaaga ttggattgta 757200
aactattttc ttatgaatat atctatttaa tttcatttgt gaaatctgct gatcagaata 757260
gctatgaaaa attagtacaa gacattttgg tgttacgaac atgtgctgaa gtaaaaatta 757320
aacatcaatt aactgcggaa aatagtcgta gtcatgctta tgtaaatgca gcacatgagt 757380
gggaatctta tttttcaaaa acattgactg atgcaggtgt tttgcaaaaa acagatggta 757440
aaattatttg ccgtctaaag catggtaaga ccgaaacata tcgtaaagta acatcaagtg 757500
agttttcgat tcctaagcaa cttcaggaat ttgtgaaaaa attgcaaagt gcttattcgt 757560
tttcagaaat gccattaaat ctgaacgata gtgatcgttt gaaaattgat gtcattaagg 757620
aaatttatag cttctatcca aaagagttat tggaggaaat tggtgagctt aaggatgaag 757680
cagcatatga attattgcac ttacctaggt tgattgaaca atatgcagat aataataatg 757740
gaacagaggc atatctattt gaagatgttc tagaaatggg gttcaatatg ttttataacg 757800
tagaagctaa aaaaattggt ggaccaggta atacggattt agagtgctta tatattacgc 757860
aaaagagaaa atttgcagtg gaggcaaaat caactaaaaa taagttatca ggtattaatt 757920
caggaagatt ggaagatcat aaaaataaaa ttaaggccat ttacacaatt gttgtcacac 757980
cacgttatgt ccctgccgta ttatccgata ttcgtaattg cccaattgta attattcgtg 758040
ccaatacatt tgctgaattt ttatataatt gtttgattaa tcgctccagt attccagaga 758100
ttgattatcg gtattttgat gaaattatta ttaaaaatct tggaaaagat attagttcag 758160
aaatttccaa tttgactatg caacagtttg caagtaacac cacaatggaa gcgtatagta 758220
catgataact atttcaaatg aagataacat gatcttaatg tctcggtatc ctgacaagta 758280
ttttgatttg gcaattgtag atcctcctta tgggattttg aataaaacta aacgtggtgg 758340
tgattataaa ttcaatatga atgaatactc acaatgggat attaagccag accaaactta 758400
ctttaatgaa ttatttcgcg tgtcaaaaaa tcaaattatt tggggtggga attattttgg 758460
cgagttatgg ttgagaagtg aatataataa aggatttatt atttgggata agaatcaacc 758520
agagacatta aataattttt ctatggcgga aatggcttgg tcgtcattcg ataggccatc 758580
taaaattttc cggtttagtg tgcggaaaaa tcgtaataaa actcacccaa cacaaaaacc 758640
agtcgaatta tatcagtggt tgttaaaaat gtatgcaaag cagggtgata agattttaga 758700

tacacattta ggaagtggaa ctcttgctat tgcatgctgc attgcacagt ttgatttgac 758760
agcttgtgaa atcaattccg attattacca acaatcgatt gagaaaataa aaaataattt 758820
acctgaagct agaatcagtt ttgggcatcc aggttattgt attattgaat aacttaaaaa 758880
tatagagaaa ttaaccatgc aattctccta ctcatggctg aaaacccaag ccgataccga 758940
actttcctcc gataagctgg aacatctgtt aacgatgtcc ggcttggaag tggaagaggc 759000
tgaaactgcc gcgcctgcgt ttgcgggcgt ggtgattgcc gaagtgaaat ccgttgaaaa 759060
acatccggat gcagaccgtt tgaacgttac ccgagttgat gcgggtacgg gcgggttggt 759120
gcagattgtg tgcggtgcgc cgaatgtgaa agcgggcatc aaagtgccgt gttcgctgcc 759180
gggtgccgtt ttgccgggta atttcaaaat caagccgacc aaaatgcgcg gcgaggtgtc 759240
ggacgggatg ttgtgttcca ccgacgaact cggtctgccc gacgacggtg tgaacggcct 759300
gcacattctg cctgaagatg cgcccgtcgg taccaatatc cgcgaatact tggatttgga 759360
cgatacgctg tttacgttga aaattacgcc taaccgcgcc gactgcttga gcatcaaagg 759420
cattgcgcgc gaagtgtccg cattgacggg gtgcgcgttc aggcagcccg aaatccatac 759480
cgcgccgatc acgggcagtc gaaaacagcc cgtgcagatt aacgcgcctg ccgattgcgg 759540
ccgttttatc agccgtgtga ttgaaaacgt gaacgcgcgc gctactacgc cggattggat 759600
gaaacaacgt ttggagcgca gcggcatccg cagtatttcc gcgctggtgg acatcggcaa 759660
ttatgtgatg ctggaaatcg gtcagccgat gcacgttttt gatgccgaca aactttccgg 759720
cagcctgcac atccgccgcg cgcgcgaagg ggaaacgctg gaatgcctga acgagaaaac 759780
cgtttccctg tctgaaaaca cgctggtcgt ggcggacgaa aaaggcgtgt tgagtttggc 759840
gggcttaatg ggcggcgcgg cgagcgcggt ttcagacggc acgcaaaata tcgtgctgga 759900
agcggcttgg tttgcgcccg aaatcatcgc cggcaaatcg cgccaatacg gtttcggttc 759960
ggattcgtcg ttccgcttcg agcgcggcgt ggattaccgt ttgcaggcgg atgccattga 760020
acgtgctacc gaattggtgt tgcagatttg cggtggtgcg gcaggcgaga tggtggaagc 760080
gcaaggcgaa ttgcctgaag cgaagcaggt tggattgcgt ttggaccgtc tgaaaaccgt 760140
gttgggcgtg gacattcctg ccgaacaggt ggaaaccatt ttgcaacact tgggcctgca 760200
gcccgagaaa acggcggaag gcttccgcgt taccgcgccg agcttccgtt ttgacatcga 760260
aattgaggct gatttgattg aagaaatcgg acgcgtttac ggctatgaaa acatccccga 760320
cgattacacg tcaggccgtc tgaaaatgct ggaactgccc gaaacacgcc gcccgcgttt 760380
tgccgtttac aacgaaatgg cggctcgcgg ttaccgcgaa gtggtcagct atgccttcgt 760440
tgacgagcag tgggaacaag attttgccgc caacgccgac cccatccgcc tgcaaaaccc 760500
gctggcggcg cagtatgccg tgatgcgttc cacgctcatc ggcggcttgg tggaaattct 760560
gcaaaacaat ctgaaccgca aacaaaaccg cgtgtgcgtg tttgaaatcg cccgcgtgtt 760620
cagcaaaggt tcagacggcc agtttgtcca aaacgaacgc atcggcggat tgtggtacgg 760680
cgcggtcatg ccggaacaat ggggcgggaa aacgcgcaat gcggattttt acgacatcaa 760740
ggcggacgtg gaaaatctgt tgaaaaacaa agcagtcgag ttcgttaaaa ccggacatcc 760800
cgccctgcat cccggacgtg ccgccaatat cgtttcagac ggcaaagtca tcggctttgt 760860
cggcgaactg catccgaaat ggctgcaaaa atacgacctg ccgcaagcgc cgctggtatt 760920
tgaaatcgat atggcggccg tgttggaatg cgggaaaacg cgctatcggg tcgtatcgaa 760980
attccagccg gtgcgccgcg atttggcgtt tgtgatgccg gaagctatga gccatgatga 761040
tttgctgctt gtcttgaaag gcgcggcaaa caagttggta caggaaatca gcgtgtttga 761100
cgtgtatcgc ggcacgggac tgcccgaagg gatgaagagc gtggcggtca aagtgatttt 761160
gcaggatatg gaaaacacgc tgacggatga ggcagtcgag ccgcttatcg gaaaactgat 761220
tggcgcggca acggcggcag gggcgcggct tcgcagttaa aaaataaata ttcgcttgaa 761280
ttttaaataa aaattggtaa taatccacaa ctgttacaac agaaggtaat catatgactc 761340
tcactaaagc agaactggcc gatattttgg tagacaaagt cagcaacgtc accaaaaacg 761400
atgccaaaga aatcgtcgaa ctcttttttg aagaaatccg cagcactttg gcaagcggcg 761460
aagaaatcaa aatttccggt ttcggaaatt tccagttgcg cgacaagccg caacgcccgg 761520
gtcgtaatcc taaaaccggc gaggaagtgc cgattaccgc ccgccgcgtg gtaactttcc 761580
atgccagcca gaaactcaaa agcatggtgg aacactacta tgacaaacaa cgttgatttg 761640
aaggttcccg caaaacgcta tttcacgctg gacgagttgt gcggactgtt gcaaatcagc 761700
ccctatggtt ttgcgcaatg gcagcatgat cacggtgtgg ttgtcggtta cggcggcgaa 761760
cgctacaccc gtttggatgt ggtgaaactg ttgaaattgc agagcacgtt tgcaccgtat 761820
gcagaaggtg cggaatcggg ttcggacggc aaccgtccgg ttacgcttca ggaaatcgga 761880
gacgctctga aagacctgtt ggcggatttg gataaggaat tgtgctgatt tgaggccggt 761940
tgcaggtatg cagccggttt tgttttacac gctaaaaaat aattatagtg gattaacaaa 762000
aatcaggaca aggcgacgaa gccgcagaca gtacaaatag tacggaaccg attcacttgg 762060
tgcttcagca ccttagagaa tcgttctctt tgagctaagg cgaggcaacg ctgtactggt 762120
ttttgttaat ccactatatt gcgtgatttc acattgtttc ggcttgaagc acatggtttt 762180
gtaatcattt acaggcagct cgcttggagt cctgttcggg cggtttgctg tttacttaaa 762240
tataaggatg acggtcaatg agatttttcg gtatcggttt tttggtgctg ctgtttttgg 762300
agattatgtc gattgtgtgg gttgccgatt ggctgggcgg cggctggacg ttgtttttga 762360

tggcggcagg ttttgccgcc ggcgtgctga tgctcaggca tacggggctg tccggtcttt 762420
tattggcggg cgcggcaatg agaagcggcg ggagggtatc cgtttatcag atgttgtggc 762480
ctatccgtta tacggtggcg gctgtgtgtc tgatgagtcc gggattcgta tcctcggtgt 762540
tggcggtatt gctgctgctg ccgtttaagg gaggggcagt gttgcaggca ggaggtgcgg 762600
aaaatttttt caacatgaac caatcgggca gaaaagaggg cttttcccgc gatgacgata 762660
ttatcgaggg agaatatacg gttgaagagc cttacggcgg caatcgttcc cgaaacgcca 762720
tcgaacacaa aaaagacgaa taaatatgaa tggaatgccg tctgaaggtt cagacggcat 762780
ttttccggtt tgaaaatata gtagattaac aaaaaccagt acggcgttac ctcgccttag 762840
ctcaaagaga acgattctct aaggtgctga agcaccaagt gaatcggttc cgtactattt 762900
gtactgtctg cggcttcgtt gccttgtcct gatttttgtt aatccactat aaaatagggc 762960
tgtaaccttc aatcggaatt tgttgcctgc gggatatacg gtatgaatgt ttggtatata 763020
tgggacagga tggtggaaat ctatcataag tataagaagc cgtgcctggt tttggcggtg 763080
gattttgtga tgggtatggt attcatagag ccgaatgagg agccgtgcat cggtaggtgc 763140
tatgcgccta tgtcggagtc ccctgatttt gctaacgctg ttgcgatggc tgttgctatg 763200
atctgtatcg tatggattgc cgttttatag tggattaaat ttaaaccagt acggcgttac 763260
ctcgccttgc cgtactattt gtactgtctg cggcttcgtt gccttgtcct gatttttgtt 763320
aatccactat atctatgact gattgaagcg ttgggcggag gctgcgtgaa acggtattgg 763380
gcgttgggcc gtctgattcc aatcgggctt ggggaatgcg aaacggtgtg cgcttatact 763440
gcggacgatt tgtttcgcgg ttttgcgccc gaaacggatg gagaggtgtg ggaaacggtc 763500
tgtcggagta gaatacgcgt tttgcgtttg aatacagtaa gaagaaaaga gagaaactta 763560
tgccgtctga acatcaacac atatcatcat tgcttgattt cgaccgtacc catctgcttc 763620
atccctatac ttccatgacc gatccgctgc ccgtttatcc tgtcaaacgt gcagaagggg 763680
tgtttatcga attggcggac ggcacgcggc tgattgacgg gatgtcctcc tggtggtgtg 763740
cgatacacgg ctacaatcat cctgttttga atcaggcggt tgagacgcag atgaaacaaa 763800
tggcgcacgt gatgttcggt ggtttgacgc acgagccagc ggtggagctg ggcaagttgt 763860
tggtcgggat tttgccgcag gggctgaacc gtattttta tgcggattcg ggttcgattt 763920
cggtggaagt tgcgctgaag atggcagtgc aataccagca ggcgcggggt ttgacggcga 763980
agcagaatat cgcgacggtg cgccgcgggt atcacggcga tacttggaac gcgatgtccg 764040
tctgcgatcc ggaaacgggg atgcaccata ttttcggcag cgcgttgccg cagcgttatt 764100
ttgtcgataa tccgaaaagc cgtttcgacg atgaatggga cggggcggat ttgcagcctg 764160
tccgcgcctt atttgaagtg catcatgcgg atattgccgc ctttatttta gagccggtcg 764220
tgcagggcgc gggcggcatg tatttttatc atccgcagta tcttcgcgga ttgcacgatt 764280
tgtgcgacga atttgatatc atgctgattt ttgacgaaat cgccactgga ttcgggcgca 764340
cgggcaagat gtttgcctgc gaacacgcgg aggtcgtgcc ggatattatg tgtattggca 764400
agggtttgag cggcggctat atgacgctgg cggcagcaat cacttcgcaa aaagttaccg 764460
aaacgatttc gcgcggcgaa gcgggcgtgt ttatgcacgg cccgacgttt atggcaaacc 764520
cgctggcgtg tgccgttgcc tgcgcttcgg tcaaactgct tttgtctcaa gactggcagg 764580
caaatatccg ccgcattgaa agcatcttaa aaggccgtct gaaagccgcg tgggacattc 764640
gcggcgtgaa agacgtgcgc gttttaggtg ccatcggggt gatcgagctg gaaaaaggcg 764700
tggatatggc gcgttttcaa gcggactgcg tggcgcaggg catttgggtg cgcccgttcg 764760
gcaggctggt gtatctgatg ccgccctata tcatttcaga cggcgttttg accaaacttg 764820
ccgacaaaac cgtgcaaatc ttgaaggaac acagcaaatg aaaggcgttt acttcgtcag 764880
cggcatagac acggacatcg gcaaaaccgt cgccaccggc gtgttggcaa aacaattgtt 764940
gcagcagggc aaaagcgtga ttacgcaaaa gcccgtgcaa accggttgcc aaaacattgc 765000
cgacgacatc gccgtccacc gcaaaattat gggcataccg atgcaggaag ccgacaaacg 765060
gcggctgact atgcccgaaa tcttcagcta tcccgcttcg cctcacctcg ccgcccgact 765120
ggatggcagg gctttggact tggacaaaat ccgcaccgcc acacaagaat tggcggcgca 765180
gtacgaagtc gttttggtcg aaggcgcggg cggattgatg gttccgctga cggaaaacct 765240
gttaaccatt gattatatcc gtcagcaagg ctatcccgtc atcctcgtta ccagcggacg 765300
gctcggcagt atcaaccaca ctttactcag tttcgccgcg ctcaaacaat acggcattcg 765360
cttgcacagc ctcgtgttca accacatcca cgacagccgc gacgcacaca tcgcccaaga 765420
cagcctgagc tacttgaaat gccgtctgaa agccgatttt tccgaagcgg aatggatgga 765480
gttggcaaaa acagacgcgg tataaagatt gggaaaaata tggaacacct atttgggaaa 765540
tggctgcccg acttgcccgc cgccatttca gacggcatca gcctgccgat ggtgcggctg 765600
ctgcacaccc ggtcgctgac cgccgcattg cgcgccttgc cgcatacatt ttcggtggaa 765660
ctgctgaaac tgggcgaatt ggagacggaa tgcggaggga ggctggtgcg cgaagttttg 765720
ttgaagctgg accgtatccc tgttgttgag gcaaggagcg aatgccgtat cggttcggcg 765780
ttttggcaaa acattttgga ctgcggcacg cgtcctttgg gcgagcgtct gtttcaagcc 765840
gatttggaag gggcgcgttc ggcgtttgag tttgccgttg ccggcgaagg atgcggacgg 765900
tactttgccg cgcggcgttc tcggttttcc cgtcacggcg aggaaatgct gctgaccgag 765960
tattttctgc ccgaactgaa acgttttatc ggataaaata ccgttttttc aagctgcgcg 766020

gcaatatgaa tcctaaatcc cctttatttt tacgcctgtc cgaccgtttg gatgtgtacc 766080
tgcgcctgat gcgggcggac aagcccattg ggacgctgct tttactgtgg ccgacctact 766140
gggcattgtg gctggcttca gacggcattc ccgatttggc ggtattggcg gcgtttacaa 766200
tcggcacgtt tttaatgcgc agtgccggct gcgtcatcaa cgactttgcc gaccgcgatt 766260
ttgacggtgc tgtcgagcgt acaaaaaacc gtccgttcgc acagggcagg gtcaagaaaa 766320
aagaagcgct gctgctgacg gcatttttgt gcctgcttgc cgcattgtgc ctgattccgc 766380
tgaatcatct gacttggctg atgagcctgc ccgcgctgtt tcttgcgctg acttacccgt 766440
ttaccaaacg ttttttttccg attccccaac tctatctcgg gcttgccttt tccttcggta 766500
tcccgatggc gtttgccgcc gttgccggaa acgtgccgcc tcaagcgtgg atactctttg 766560
ccgccaatgt gttatggact ctggcgtatg acacggttta tgcaatggcg gacaaagaag 766620
acgatttgaa aatcggcatc aaaacctccg ccgtcacgtt cgggcgttac gacatcgccg 766680
ccgttatgct gtgtcacgga ggctttaccc tgctgatggc agtattgggt gcggttatcg 766740
gtgcggcatg ggcatattgg acggcaatcc ccatcgtcct gctgctgcaa taccgccaat 766800
atgccgccat caaaagccgc gtccggcaaa tctgttttga aacgtttttg gcaaacaaca 766860
gaattggttg ggtgtggttt accgccattt ttgcccatac gtttttcgcg aaataaggca 766920
gggcaatgcc gtctgaagag ccgtaaactg ctttggacgg catttctatc tgtgccgaaa 766980
agcgttaaaa tatgttttta aaacgctgtg ttatgtcagc ccgtaccgta tgcgggattg 767040
agatttgccc cggcagtcgg tacaatcttt ctgttttgcg atgtctgaaa agagaagctt 767100
atgagcctta tcggcgaaat tttgcctttg tcccatattg ttttggatat ggaggtaggc 767160
agtaaaaaaa ggctgtttga ggaagcaggc ctgcttttgg aacgcgaatc ctcattgtcc 767220
catgctgatg ttttcgaatg tctttttgcc cgtgaaaaac tcggttcgac cggtttgggg 767280
cagggcgttg ccatcccgca cggccgtcat gccggcgtga agcaggcgac gggcgcgttc 767340
atccgcacgc gcgaacccgt cggatttgac gcaccggacg gcaagccggt ttccctgatt 767400
tttatcttgc tggttccgga aaacgcaacc ggcgagcatt tggaagtctt atccaaactg 767460
gccggcaagt tttcccaaaa aagcatcaga gaatcgctga tgacggtttc ctctgcggaa 767520
gaagtccgtg ccatcctgac tgaagaataa tatgcccagt atctccgtcc gccgcctgtt 767580
tgatgacaac caatacaaac tgcaactcgc ttgggccgcc ggcaattcgg gtgcggacaa 767640
ccgtatcggc gtagaggcgg acaagcccgt cctcgcccta gtcggacacc tgaatttcat 767700
tcatcccaac caaatccaag tggtcggttt ggcagagtcg gaatatctga accgcctcga 767760
atcgggggaa acgggttatc agtttggcga cctgttcgat atttctatgt ctttggttat 767820
tgtggcaaac ggcttgccgg tttccccggg actgcgcgac tattgtcata aaaacgatat 767880
tccactgctg acttccaaac tcgaaagccc ctatctgatg gacgtgttgc ggatttacct 767940
gcaacgcacc ttggcggcat cgtccgtcaa acacggcgta tttctcgatg tgtttgaaat 768000
cggcgtgctg attaccgggc attccggcct gggtaagagc gaattggcat tggaactgat 768060
ttcgcgcggc cacagcctga ttgccgacga tgcggtcgag ctgttccgca tcggcccgga 768120
aacgctggaa gggcgttgtt cgcctatgct gcgcgatttt ttggaagtgc gcggcttggg 768180
gatactcaat atccgccata ttttcggcga aacttccatc cgcccccaaaa aaatcctgca 768240
actcattatc aatttagtcg aggcggacga cgagtatatg aagcagcttg accggttgag 768300
catccgcacc gaaaccgaat ccatcctcaa cgtcaacgtc cgttcggtta cgctgcccgt 768360
cgccgtcgga cgcaacctcg ccgttttggt tgaggcggcg gtacgcaatt acattttgca 768420
gttgcgcggt aaggacagta cgcgcgaatt tttggaacgc catcagacgc aacttaaaga 768480
aaacgaacaa cacaatgaag atcgtcctga ttagcggcct gtccggttcg ggcaagtccg 768540
tcgcactgcg ccaaatggaa gattcgggtt atttctgcgt ggacaatttg cctttggaaa 768600
tgttgcccgc gctggtgtcg tatcatatcg aacgtgcgga cgaaaccgaa ttggcggtca 768660
gcgtcgatgt gcgttccggc attgacatcg gacaggcgcg ggaacagatt gcctctctgc 768720
gcagactggg gcacagggtt gaagttttgt ttgtcgaggc ggaagaaagc gtgttggtcc 768780
gccggttttc cgaaaccagg cgaggacatc ctctgagcaa tcaggatatg accttgttgg 768840
aaagcttaaa gaaagaacgg gaatggctgt tcccgcttaa agaaatcgcc tattgtatcg 768900
acacttccaa gatgaatgcc caacagctcc gccatgcagt ccggcagtgg ctgaaggtcg 768960
aacgtaccgg gctgctggtg attttggagt ccttcgggtt caaatacggt gtgccgaaca 769020
acgcggattt tatgttcgat atgcgcagcc tgcccaaccc gtattacgat cccgagttga 769080
ggccttacac cggtatggac aagcccgttt gggattattt ggacggacag ccgcttgtgc 769140
aggaaatggt tgacgacatc gaaaggtttg ttacgcattg gttaccgcgt ttggaggatg 769200
aaagcaggag ctacgttacc gtcgccatcg gttgcacggg aggacagcac cgttcggtct 769260
atattgtcga aaaactcgcc cgaaggttga aagggcgtta tgaattgctg atacggcaca 769320
gacaggcgca aaacctgtca gaccgctaat tccgtcaaac cattatgccg tctgaaaccc 769380
cgtcctccgt ttcagacggc atttcaaata ttgaaaagaa agaacagaca tggcaatcca 769440
atggtttccc ggccatatga acaaggcgaa aaaagccatc gccgagcgtg caaaaagcgt 769500
tgatatggtg attgaaatgc tggacgcgcg tatgcccgcc tccagcgaaa accccctgct 769560
tgcccagctt tccaaaggta aacccaaact taaaatcctc aacaaacaag atcttgccga 769620
ccccgagcgc accaaaatct ggctcgaaca ctataacagc cgccccgaca cctgcgccat 769680

cgccctcgat tcctccgaaa caggcgcaca cggcaaaatt acccaagcct gtcgtgccat 769740
gattccccac cgccaaggca tagataaacc cctgcgcgtc ctcatctgcg gcatccccaa 769800
cgttggcaag tccaccctca tcaacggcat gataggcaaa aaatccgcca aaaccggcaa 769860
cgaacccggc atcaccaaag ccgaacaacg cctcttcctc gccgatgact tctggctcta 769920
cgacaccccc ggaatgctat ggccgaaaat catcgtcgaa gaaggcggct acaaccttgc 769980
cgccggcggc gcagtcggac gcaacgcgtt ggacgaagaa gaagtcgccc tcgaactttt 770040
agactacctc cgccgccact acctccctat gttgcaagaa cgctaccaag ccgacaaaga 770100
ccccagcagc cactgggacg aaaacgtttg gctcgaatgg atagccaaaa aacgcggcgc 770160
agtcctcagc ggcggacgga tcaactacca aaaagccgcc gaaaacatcc tcaccgactt 770220
ccgtgaaggc aaaatcggca gaatcaccct cgaaacgccg aaccaatggg aaacttggct 770280
caaaaaagcc cgtcagaaag aagccgaact caaagccata cgcgaagcca gaaaagcaga 770340
gcgtaaaggg cagaagcttc ggaagcataa agaatgccgt ctgaaaaata tttttcaggc 770400
agcttctctc tactccaacc gatttcagac ggcatatcca aacccatgcc gtttcagcac 770460
ggatacccgt atgaccgaca aaatttctcc cgacgcgctg attgaagccg cactgctgac 770520
ccaaaccgaa ccgctgaccg aaaaatctat gcgcgaactg tgtgtgccgc cgttgtcgca 770580
agacaaactg attgatgtgt tggcgcagtt gaaaacgcgt tggcaggata gggcgttgca 770640
actggtgcat acgcaagagg gctggcgttt tcagattgtt cagacggcat tcgagcggct 770700
gggcagcctg caagaacagc gtgcgccgcg ctactcccgc gccgtgatgg aaacactggc 770760
gattatcgcc taccagcagc ccgtaacgcg cggcgacatc gagggcatac gcggcgtggc 770820
ggtgtcgcag aacgtgatgc agacttgcag gatcgggggt ggattgaagt catcggacat 770880
cgggacacat tgggaaaacc cgcattgtgg gcgacaacgg caacgttcct cagcgatttg 770940
ggtttgaaca gcttggaaga actgccgccg ctgaccgaac tgggcgaact ggttttgccc 771000
gatttgatag aaatgccgcc tacggatgaa gaagagccgg aaaccgtacc gtccgatacc 771060
ctgcccaact gaaattccaa atgccgtctg aaacgcacat tgcttcagac ggcattgcaa 771120
caaataagca gataaaaaca agcactaaga aaaattaagg aaaaacttat ttttaattta 771180
aaaaatctta gttataattc gtatatctaa agttgatatt gcttttgtcg gtagaattgc 771240
taaggaatcc tcacgatgct tctaacactt tctttgcgtg attttgtcat tgttgaaaat 771300
ctgaatctgg attttcaaag cggctttacc gtattgaccg gagaaactgg cgcgggcaag 771360
tccattactt tggatgcgat tggtctgctg ttgggcgata aagccgatta cagccaagtc 771420
cgcagcggcg caaaagaagc gcagttgtcg gcgttgtttg atatttccca tttacctgtt 771480
ttaaaagcag aattgtatga acaggggctt ttaaacgacg gagaagaaga actcagtatc 771540
cgccgcatta tcgatgccaa aggcaaaagc cgcagcttta tcaacaatca ggccgctacc 771600
ttggcgcaac tcaaagccgt cggtagccag cttatcgaca tccacgggca aaacgcccat 771660
cattcgctta atcaggaagc cgcccagcgc gaattgttgg acgcatttgc gggtagcagg 771720
gagcaggcgg aaaccgtcag gcagctttat caaaattggg ccaatgcgaa aaaagccctc 771780
caagaggcgc aggaacacgc cgatgccgtc attatcgagc gggagcgtct ggaatggcag 771840
tttaacgaat tgaatcagtt ggacattaaa caaggcgagt gggaagccct cagccaaagc 771900
cacgacagcc ttgcccattc tgccgagctg ttgcaggctg ccgaagaagt cggaagcaag 771960
attgacggcg acaacggcat ccaacgccat atctatcaat gtcaaaaact attggccaat 772020
ctgcaaaaca tcgagccgcg ctttgccgag agcctgaata tgttggcaag catcgaagcc 772080
gaattgggcg aaatcagtgc caatatgcgc gatgtggcag gtcgcagcga catcaatccc 772140
aacgaacttg ccgcacaaga gcagcgcatg ggcgagctga tggggatggc gcggaaatac 772200
cggatcgagc ctgaagagtt gcctgccaag ttggcagaaa tcgaagaacg cctgcaaagc 772260
ctgcaagctg ccgccgattt ggacgcgctc gagcataatg ttgcccacaa ttttgccgaa 772320
tatcaggaag ctgcccacat cctttctgcc atgcgccatc aggcggcaga gcgtttgagc 772380
ggcgaaacga ccgagcatat gcaacacctt gccatgaaag gcgcgcgttt cgacatcgtc 772440
ctgttgcctt cgtcgccgac ggcacacggt ttggagcagg ttcaatttca agttgccgcc 772500
aacaaaggca atccgccccg tctgctgaat aaagttgcct ccggcggcga attggcgcgt 772560
atcagccttg ccttacaggt tgttgccagc caatataccc aagttcccac cctgattttt 772620
gatgaggtcg ataccggtat tggagggggga gtggctgaaa tggtcggcaa ggcattacgt 772680
gcgttgggca gaaaacatca ggtgcttgcc gttacccacc ttccccaagt cgcatcctgc 772740
ggagaaaacc actggcgggt gcgcaagcac agcgagggag agcaaaccgt cagcgaaatc 772800
agtatattgg atgaaatcca acggatcgaa gaggttgccc gtatgttggg cggagaagtc 772860
attaccgata cgacgcggca acatgcggca gaattgctgc aacttgcgtc gaaaaatagt 772920
ttattttaaa atcaatcagt taaaaaataa ctaaaaaataa aagtctaaaa caatagacag 772980
aactcagata aatccgtatt atcacgcttt cttaatcact tgaacaagtg attgtgctgc 773040
acccgtagct cagttggata gagtatctgg ctacgaacca gagggtcggg cgttcgaatc 773100
gctccgggtg cgccagtaag aaaatacaat atgcgcccat cgtctagcgg ttaggacatc 773160
gccctttcac ggcggtaacc ggggttcgat tccccgtggg cgtgccaatt caaaatgcct 773220
ccgattatat cggaggcatt tctcatttct catttctcat ttctcatact gagacctttg 773280
caataacata ggttactaaa attttatgct caatctcatt ttcaaaatgc aaaacttttc 773340

tgatttttcc tactttttgc tcaatattag gaaggtttta ggcaattgaa aatttttttgg 773400
cgcattttta tgcgtcaaat ttcgttaaca gactattttt gcaaaggtct cggattaaca 773460
aaaatcagga caaggcgatg aagccgcaga cagtacaaat agtacggaac cgattcactt 773520
ggtgcttcag caccttagag aatcgttctc tttgagctaa ggcgaggcaa cgccgtactg 773580
gtttttgtta atccactata ttgagtcctc gagaagggaa ataaaaatta acatccttat 773640
atattgagtt cctgagaagg gaagattaac aaaaattaac gccctttact tcatacaatc 773700
aacagggctt tttcattcct tccttatcta acagggggta cagaaaccga aacggctggc 773760
agggttaagg aagtcttcga atgttacgga acattcatct tggacagcaa aggcaatttg 773820
ttaggcattc cttactcctt attttgggaa gaaaacgtta tgggtgtttt cgatatttta 773880
ccgtcaggat tggtatgttt atttgaatat gattttctgt ggtcgggacg gcatgcggca 773940
aagacttaag gggttagatc cttccttctg acgatggcgc ggatgatggt gcggttgggg 774000
tgtagggcgt ggcgcaggcg ttgtgaaaag ggatggggca agcctaggat ttgggctgca 774060
atggcggcgg cgcagatggg ggcggtggcg agtccgcggg tgccgtgcgc ggtgttgacg 774120
taggcattag gcaggtatgg gcatggggtg tcgatgcggt agtttttgtc cagcgcgagt 774180
ttggtgtagg tctgccgcat ggcggcaatg tcgccgagtg cgccgactag gggaaggtgg 774240
tcggggctgt cgcagcgtat ggcggcgtgc ccttggtgtt tttgggggtt tgggttggcg 774300
gcaaacaatg attcggaaag ggcggggtta aggtgtgcca atgcttggcg gtttgaggct 774360
tcttcggctt cgttccatcc ggtatggctg ctgttgggaa taaaactcgc gccgtagcag 774420
tgcagtccgt gccacgacgg gctgatgtag ctttcgcctg aaacggcgca acgcagttgt 774480
tcggaaaacg gggtggacgg tgtgaggccg gtttgtccgc gtatttgcct gagaggcagg 774540
gcggcgaggt tggtttcggg taggtagggg ctgttcgcac cggtgcagta gatgatgtgt 774600
gtggcggtaa atgtgccgtt tggcgtgctt gcaatccact tttccccgtc gtgggaaatg 774660
tcggtcaagg gtgtgtcttc gtgtagtcca atgagcggat ggttgaggag ggtgcggacg 774720
aatgcgggtg gattgagcca tacgccgtgt tgccagtaga gtccgcatga agggtggtcg 774780
tatgggacgg acagtgggat accggcgatt ttttcggctt ctgcagatgt gatgctgcgg 774840
tagaggtggt tatggtgttt ttgcaaaccc aattcgtgat tgcgttgttg ttcggtgcgg 774900
ctgtaattga ggtggatgat gccgttgccg ccccaggttt cggattcggg caggatgtgt 774960
ccgagcaggc gtttggtgta gccgtagccg gcaagcaaaa gttcggtctg ttcggtgtcg 775020
tgcggcgaga ttttggcgta gagcagccct tggcggttgc cgctggcggc ttgggcggct 775080
tttcgggctt ccaatacggt aacggaaatg ccgtgtgatg ctaaggcgtg ggcggttgcc 775140
gcgccggata tgcccgcgcc gataacgagg atgtgttccg gtttttgccg ttcggatgtt 775200
tgtggaagtg caaaccaggg tttgtcgggc ttgctttcgg tttgcgggat ggcttcggtc 775260
tgccaggaaa tgtggtggaa atgttcgtgc aggtggtggg tgcgtgaagg gggaaccagc 775320
cagaagcgga catcgggcag gatgtgttcg atgaggttga tgctgtcgaa ctggaggcac 775380
tgcattgcct gatccaaacg gtgcttcaga cggcattccg cgtccgaagc atcttgtgcg 775440
gtttgaaaat cgggaatctg attatcgggg aggcagataa tcaggttgag cgggggtgcg 775500
tgtttgcgga tggcttggtc gagtgtgcgg atgtcgggaa tgccgtccca tacgagattg 775560
tccatatcaa tgccgtttaa agtgtgggtt tgaatatcgg tatcgggata aagctgttaa 775620
aatacgcgcc gtttgaaggc acgcctgcgc ctgccggata ttgtatgccg aaccgaggtg 775680
ttttttgaat aatattcctg ttgaaatccg tttgttgaaa aaccgtaccg tgttggtttt 775740
gacttatggg gacgaaccta aaaatctgcc tgccgaattt ttacgcgtct attcgccgag 775800
tgcggaagtg cgcggacacg gcgtgggaca ggatgttttg cagaccggca aggcggatgt 775860
ccaaatcgcg gatttgcagc ctgtcggaca gtacgcgctg aaaatcagtt tttcagacgg 775920
gcacgacagc ggtctttacg attgggcgta tctgcacaga ctggcatacg gatacgatgc 775980
gatgtggcag gaatatttgg acaaattggc ggcggcgggc gcgtcgcgtt ttgaagagaa 776040
ataagaccgg tcggatggta atctgacggg caaaggtatc agagaggtgg ttagaatatg 776100
ggcggacaga aaacgcattt cggattcagt acggtcaacg aagatgaaaa agccggcaaa 776160
gtggcggaag tgttccactc cgtcgccaaa aactacgaca ttatgaacga tgtgatgtcg 776220
gcagggctgc acagggtgtg gaagcatttc accatcaaca cggcgcacct gaaaaaaggc 776280
gataaagtgt tggacattgc gggcggtacg ggcgatttgt cgcgcggttg ggcgaaacgg 776340
gtcggcaagg aaggcgaggt ttggctgacc gatattaatt cctctatgct gaccgtcggg 776400
cgcgaccgtc tgttgaacga aggcatgatt ttgcccgtat cgcttgccga tgcggaaaaa 776460
ctgcctttcc ccgacaatta tttcaacttg gtttccgtgg cgttcggctt gcggaacatg 776520
acgcataaag atgccgcgct gaaagagatg taccgtgttt tgaaaccggg cggcacgttg 776580
ctggtgttgg agttttccaa aatctacaaa cctttggaag gcgcgtatga tttctattcg 776640
ttcaagctgc tgccggtcat gggcaggctg attgcgaaag atgcggagag ttaccagtat 776700
cttgccgaat ccatccgtat gcaccccgat caggaaactt tgaaacagat gatgctggat 776760
gcgggcttcg acagcgtgga ttatcacaat atgagtgcgg gcatcgtcgc gctgcataag 776820
ggcgtgaaat tttaaacgga ctggctgtgc agccaatgcc gtctgaacac gtttcagacg 776880
gcatttttgt gtttttttga gaaagagatt ttaaatctta aaattaattc acatatccat 776940
caataattta taaatttttt aaaaaatagg aacaattatc atttgcaaga ttgggagatg 777000

tctgtataat gcagtcaatc cagtaaacaa cgcagcagac gaaaggaggg aaaaatgccg 777060
gaaagtattt tcaaacagat ttcccttgat attttgaaac tgcatcggga ttctgtttat 777120
tcgctgcttg ccacttccgg ctgcaactgt caggtgcatg aagcggcgta tgtcaacatc 777180
gacggcaaat attatattgc gctttcatgc gagcccgagg tgggggaagt caaaacaggc 777240
attttgctga ttgaggatga aagccgcaac cttcgtttga gctgggtcgg cagtgcgcgg 777300
gagcttgact gcaaggataa tgcctacaaa cgcgccctgt ccgcgttgtc cagaaagctg 777360
gggcggtgta aggacaggct gcatacggcg gttcaaccgt ttctgttgga gctggtaccg 777420
gagaaaggca gattttctgt cggcgatgaa gaagtttgga tttctcgaaa cgatttagtg 777480
agggctttat atcctgtcgg atacagtatg cggcaggcag tgtttcagat ttaaagtttt 777540
ggtagtggtt tgtgttcctt ttgcgccccc ttttccaagg ggcgattttt ttgcacgcgt 777600
gttggcggca aaggaaaaat gccgtctgaa aggctttcag acggcatccg cgtgcggaat 777660
tacctgtccg gtaaaagacg gataccttga ttgcccagcc gttttgacaa ttcggcaacc 777720
tttccgtgtt ttcctaaaac aaaatcaggg aggatttctg ccaaagggcg gatgacgaaa 777780
ctgcgttcgt gcgcgcgcgg atgcggcagg gtgagtcggg tgtcgtcgct ggagatgccg 777840
tcaaagtcga taatgtccaa atccaatgtg cgcggcgcgt tgcggaagct gcgttcgcgt 777900
ccgaaatcag cctcgatacg gttgagttcg gcaagcaggg caatgccgtc cagagtggtg 777960
gaaacggtgc agacggcatt gacaaaatcg ggctgattgt cgtaaccgac gggcgcggtc 778020
atatacagtg aggaagcctg tttaagacgg atgtcaggat gggacgacag cgtgtccaat 778080
gcggcgcgta cctgttgggc agggttttca agattactgc ccagggcgat gacggcaaaa 778140
tgtctgttgt tcataacggt gtttcagaaa ggcaggactt tggttttggc aaggtaaacg 778200
atgcaggcga cgcaacacat ggcgagcagg taaacggtgt agaacttggt cgaacgcgga 778260
cgggcgcgca tcatcatcat acccaatgcg atataggcga gcagaagcag gatttttgta 778320
ccgagccaag gcgcgttgaa cggggagaaa tgggtaattt tcatcagcca caatcccgta 778380
aacagcagca tggtgtcgtt aaggtggggc agtgccttcc aaaagcccgc caagggcttt 778440
tctggatttt tccaaagtag gaaaaaacgg atgttgaata ccaaaatggt gatggtaacg 778500
aagatttggt ggctgtattt gacaatcaga tactgcatgg tcggctcgta tcaaaataag 778560
ggttagaatc ggcttatttt accgcaaaca gttatttttg acgcagtttt tcaaatacca 778620
aaagataggg tggctgtttt ttccggttgg taaagccgta acgcaaaacg gcaaactgtt 778680
cttgaggcag gtttttttgcc cattgttcga ttgcttctgc ctcctgtttg ccgttttcgt 778740
gtcccggata gaggacggca ataagcatac cgttttcttt cagtagggat aaggtggcag 778800
aaagggcggc aatgctggtt tccgtgcggg tggtaaggct tttgtccccg ccgggcagcc 778860
agccgaaatt gaaaatggct gcatccagcg gctttggaat atattgcttc aggttttcat 778920
gtccgtccaa gatgagccgt acattgctgt aacctgcttc ctgcagacgg catcgggtgt 778980
tgttcagggc ttgcggctgg atgtcgaatg cccacacttt cccccggatg cctgcggttt 779040
gtgcgaggaa aagggtgtcg tgtccgttgc cggcggtgcc gtccaaagca ttgccacctt 779100
cgggaagtgc cttccgcaaa aggcaatggg cgaatggaag gatgttttgc aataacattt 779160
ttaaatgctg tctgaaaata aaaatacctt accgttgtcc ggtaaggtat tgaaagatat 779220
gacacgtcat gcttcgtgcg gattattcgg caggctgctg gacggtttcc acttggacga 779280
gggtcgaagt cggtgcggct tggggtctgt tttcatgttg taggttgacg gagcgtgccg 779340
gtacgatggt ggaaatggcg tgtttgtaaa ccatttgggt gacggaagtg tttctcagga 779400
gaacaacgta ttgatcgaaa gactcaacct gaccttgtaa tttgataccg ttaactaagt 779460
aaatcgaaac cggaacgtgc tctttacgca gggcgttcag gaagggatct tgcaacattt 779520
gtcctttagc tgtcatattt ttaactccgt tattatgatt gtgaaatcgg gcagacgccc 779580
tgtgcgtgtc gaattgtagc cttgaatagg aaaaattacc aatttttttt gtgtttgggc 779640
ggttttccgc cgggcatttg tatgtcagga gcgttgctgc agcatccacg attcgatttt 779700
gcgggcgtcg ttgacgcgtg tggcggatgt gggcgagttc agcaatacga tggtaacggg 779760
ttggttttga atgttggctt tgacaaccat agacctgcct gcttcgcgta tgtagccggt 779820
tttctgcaat tcgatgttcc acatgccttc tctgaccagg gcattggagt ttttgtagtt 779880
ctgctgcccg tttttggtct gtaccgaggc gtagttggaa gtcgagttgg tgcggatttg 779940
cggatattgg gcggcggcgt tgaccataag gctcaggtct ttggcggtag aaacgttttg 780000
gaagttgagt ccggtcggtt cgtaaaagcg gctgccgtac ataccgaggc tttgggcttt 780060
gcggttcatg gcggcgacaa atgcgcccat gccgccgggg taggttctgc ccaatgcatg 780120
ggtggcgcgg ttttcgctgc tcatcaggct caggtgcagc agttttttgc gtgtaagtgc 780180
cgtacctatg gcaagacggc tgccggtccc tttgatgcgg tcgatttcgt cgggcgtaat 780240
ggtaacggtt tcgttcatgt ccaagtttgc atccaaaacg accatcgcgc tcatcagttt 780300
ggaaatggag gcgatgggca taatcctgtc ggcgtttttc tgatacagta tctgtccggt 780360
tttgttgttg acgaccaggg cagactgtga ggagagaatc agaccgcctg taatggcttg 780420
ggtgttggtg gggtgtatcg tgctttcggc gaatatttct atcggatcgg aggaggtaag 780480
catgttctgt tctaaaaatt gccctaaaat gtcgttgtcg gcaaaaaggt gggctgacgg 780540
cagggaaagg cagagaccga gcagcagcga tgagggcagg cgtttcagag tatggatgga 780600
catttttgat tccatatttt tgagtattgg cgttattttg ttgaaaaaac agccatctgt 780660

aaagtatgtc gtctgacagg agactgcccg taggcgggcg gtcttgttgt gttttgggga 780720
ttgggtttta taacattctg tttttaaatc ggaacatatt ttgtggtttg acatggatat 780780
ttttcatgcc gtcgtgtgtc ggtttggatg tttccggcgg ttgaatcctt gtcctttggg 780840
gcggtagaat cggggttggt ttggcaattg cggcggtgcg tctgcgtgcc gttttgaata 780900
atgggaatat cgggagtagg actatggatg tgaaatatga atttaccctg ccttcgagca 780960
gcggtgcgga ttttcattcg gcagaacatc tgcctttggt cgtgtatttt tatccgaaag 781020
acagtacgct gggctgtacg acggaaggct tggatttcaa tgcgcgtttg gaacagtttg 781080
aggcattggg ttataccgtg gtcggtattt cccgcgacgg cgtaaaggcg catcagaatt 781140
tttgcgccaa gcagggtttc cggttcgagc tgttgagcga caaggatgaa acagtgtgcc 781200
gcctgtttga tgtcatcaaa ttgaagaaac tgtacgggaa agagtcgtta ggtatcgagc 781260
gcagtacgtt cgtcttgaat aaggatggag aaatcgccca tgaatggcgg aaagtcaaag 781320
tggcgggtca cgcgcaggaa gtattggaaa cgctttcccg ataatgtgaa ccatgccgtc 781380
tgaagaagat tcagacggca tttgtttgga acggtatgga agaaggtttg atcgacaggc 781440
tgcttgaaac gctgtggttg gacaggcggc tcagtcagaa tactttaaac ggttaccggc 781500
gcgatttgga aaaaatcgcc cgccgcctgt cccaatcggg cagaatgctg aaggatgcgg 781560
acgaagcgga tttggcggcg gcggtttatg ttgacggaga gcaacggagt tcgcaggcgc 781620
gcgcattatc ggcatgcaaa cgcctgtata tatggatgga gcgtgaaggc ataaggacgg 781680
acaatcccac ccgtttgctg aaaccgccca aaatcgacaa gaatattccg accctgatca 781740
ccgagcagca gatttcccga ctgcttgccg ccccggatac cgacacgccg cacggtttgc 781800
gggacaaggc tttgctcgaa ttgatgtacg cgaccggctt gcgcgtcagc gaggcggtcg 781860
ggctgaactt cggcaatgtg gatttggaca ggggctgtat taccgcgctg ggaaagggtg 781920
ataagcagag gatggtcccg atggggcagg agtcggcgta ttgggtggaa cgctattata 781980
cggaggcacg cccacttctg ctgaaaggca ggaattgcga cgcattgttt gtcagtcaga 782040
aaaagacggg catttcccgt cagttggcat ggatgattgt caaagaatat gcaagtcagg 782100
caggcatcgg gcacatcagc ccgcacagcc tgcgccacgc ctttgccacg catctggtgc 782160
ggcacggctt ggatttgcgc gtggttcagg atatgttggg acatgccgat ttgaatacga 782220
cgcagattta tacccatgtt gccaacgtat ggttgcaggg tgtagtgaag gaacaccatt 782280
cccgaaactg aggcttctac tttggtttaa taagaaatca atcgaaaatg caaacttgat 782340
aaaaaattac caagacaaac cgtgtatacc gaccttgcaa tgcgaaccgt ttttatttat 782400
attcgcatac gataataaaa gccgctatcg gtacgatagt ttgagaacac acggagcaca 782460
aaatgtttgt ctgcatctgc aatgccgtta ccgaccatca aatcaaggaa accatcgccg 782520
ccggcgcgac cacaatgggc gatttgcagt cgcaattggg cgtagcgagc tgctgcggct 782580
gctgcgggga gcttgccgct tcgtttctga cggcgcacaa tgcgcaaccg acggttacgg 782640
cgggtatcaa cgttcaagcg taaaacggtt ttcgaaatgc cgtctgaact gttcagacgg 782700
catttttact gtttttggca ggacttgagt atcatcttcc tcgaaaacat tgtttttttcc 782760
caaatagacc atgattctgc tgcgtctaag gctttggcgt gtgcaaattg acagataagg 782820
aaacgcggat gaaattgacc ttgatgtttc gtgaatattg cagcttgtgc cacaaaatgc 782880
gcgacgaact caaacctttt caggatgaat acgggttcgg gctggaagtg gtcgatgtgg 782940
atgaaaatcc tgttttggaa gaaaaataca atgagctggt tcccgttttg ttggcgggag 783000
atgaggaaat ctgtcactgg tttttggatg aggacaggtt gaaacagttt ctcgaacggt 783060
aaaaaaatgc cgtctgaagc aggacttcag gcggcatttt tttcaaatca acgttcttta 783120
cgtttttgcg gggcggatga cctgccggta aaggaagcac gtttggatgc ttggtaaatt 783180
gctagtcttc ttcgatgttg catattaacc ctttctttat tttatttgtc ggttgggagg 783240
attcttattt attgattttt tcaataaaaa ttagaaaatt tattgtgaga tgttattgtt 783300
ggcaatcata tcatgtttta ctgttgatgg aagcatgatt gtgtaaagat gatatgtgtt 783360
tgtgtaatcg gtagatttta tagtggatta acaaaaatca ggacaaggcg acgaagccgc 783420
agacagtaca aatagtacgg caaggcgagg caacgccgta ctggtttttg ttaatccact 783480
atataagaaa accgccaagg cggtgcttga cggcggaaac agaggggcgg gcggcatcag 783540
agcagcttgg aaacgacctg tgcaaggctt tttgccagtc tgacggtttg atgccgaagt 783600
cgttttcgat tttgcggcag tccaaaatgc tgtatgcggg cctgggggcg gcggtcggat 783660
attccttgtc tgaaacggca gtcaattcgg gaacggggaa ggatgtctgc tgttgcgatg 783720
ccgcttggaa aatatgttgg gcaaattcgt accaggatac ggatttgctg ccggcgtagt 783780
ggtaaatgcc gcgaacggga ttggagtgct gcaacaggcg gatgatggtg gcggacaagt 783840
cgccggcata ggtcgggcag ccgatttggt tgtggacggc ggacagcggg gaacgttccc 783900
gcgcaaggtt cagcatcgtg cggataaagt tgtccccgta ttcgctaaac agccaagaag 783960
tccgcaggat aaggctgtcg ggattggcag acagtgcgag cagctcgcct gcggttttgg 784020
attgtccgta tacattggaa ggattggtaa agtcgctttc ctgataggggt cttttccctt 784080
taccgtcaaa gacatagtcg gttgagatgt ggatgaatcg ggcatgggcg cgatgtgctg 784140
ccaaggcaag gttgtaaacg gcggaagcat tgacggcaaa tgccgctgcc gcatcgcctt 784200
ccgccttgtc gacggcagta taggcagccg tgttgacaat ggcgtcgggt tggaaacttt 784260
tgaccatgtt gcagacggca tcggcatcgg taatgtctag ggatgcggaa tccgtcgcaa 784320

tggtttccca gtcttccgga agacggtcgc gcaggcagcg tgccagttgg cttttcgagc 784380
ctgtcaatag gattctcatg aggtatttcc tttggtaaaa gtgtattgta ggacttgctg 784440
tcggtattat agtgccaaaa ttttgccgac ggttgacggg ttggcttttt gtgccatggg 784500
tattgttttg cgccgacttc ggctagaata tcggtttgtg attcaaacct gtcgggtgtc 784560
gggtcggtat tttcaagata ttttcagggg ggggcgaaat gagtgagatt ggtttggtaa 784620
agatctattt tggaaaagtg cgcgatttat atgaaatcga cgataaacgt atgctgatgg 784680
tcgcttccga ccgcctgtcc gcgtttgatg tgattttgga cgacccgatt ccgagcaaag 784740
gggagattct gacgcagatt tccaattttt ggtttaaaaa actggcgcat attatgccca 784800
accactttac cggtcaaacg gtttacgatg ttttgcctga aaacgaagcc aaagctttag 784860
agaaacgcgc cgtcgtggct aaaaagctca ctccggtgaa agtagaggcg attgtgcgtg 784920
gttatctggc aggcagcggt tggaaagatt atcaaaaaac cggctcggtt tgcggtattc 784980
aactgcctga aggtatgcag gaagcgcaac aactgcctga agtgattttt acgccctcaa 785040
ccaaagccgc agtcggcgat cacgatgaaa acatcagctt tgaagaatgc ggacgcatta 785100
tcggcaaaga attggcggaa gaagtgcgcg ccaaggcggt tcggctttac accgaagcgg 785160
cggaatatgc caaatcgcgc ggtattatta tttgcgatac caaatttgaa ttcggtttgg 785220
atgaaaacgg tacgctgacg ctgatggatg aggtattgac tcccgattcg agccgttttt 785280
ggcctgccga ccaatacaaa gtcggcacca atccgccgtc ttttgacaaa caattcgtcc 785340
gcgactggct ggaacaaagc ggttggaaca aaaaagcccc tgcgccgaaa gtgcctgccg 785400
atgtgattca gaaaactgtc gagaagtatc gggaagcatt gactttgctg actcaggatt 785460
gatttttaag tttgaaggcc gtctgaaaga aatatggttc agacggcctt tttattgtat 785520
caatactgga ttttaaggat ggttgccttt ataatccgca attgctttca gcgtccgaaa 785580
tgccgtctga aagcttgttt ataacctgcc gcacggtctg aaaccctaac tatgcacatt 785640
cggattttag tgtgcattat tagtgtttta gcagtgcggt attttgaaag gaacaatgat 785700
gttcgacaaa cacgttaaga ccttccaata cggtaatcag accgttactt tggaaaccgg 785760
cgaaattgcc cgccaagccg ccgctgccgt taaagtctct atgggcgaca ccgttgtttt 785820
ggttgccgtt accaccaaca aagaagtgaa agaaggtcaa gacttcttcc ccctgaccgt 785880
cgattatttg gaacgcactt acgccgcagg caaaattccc ggcggtttct tcaaacgcga 785940
aggcaaacaa agcgaaaaag aaatcctgac cagccgtctg atcgaccgtc cgattcgtcc 786000
gctgttccct gaaggtttct accacgacat ccaaatcgta gcgatggtcg tgtccgtcga 786060
tcctgaaatc gattctgata ttcctgcaat gttgggtgca tctgccgcgc tggtgttgag 786120
cggcgtaccg tttgccggcc cgatcggcgc ggcacgcgtc ggttatgtaa acggcgtgta 786180
cgttttgaat ccgactaaag ccgaattggc gaaatcgcaa ttggacttgg tggtcgccgg 786240
tacttcaaaa gccgtgttga tggtggaatc cgaagccaaa atcctgcccg aagacgtgat 786300
gctgggcgcg gtggtttacg gccacgatca aatgcaggtt gccatcaatg caatcaatga 786360
atttgccgac gaagtcaatc cggaactttg ggattggaaa gcacctgaaa ccaatgagga 786420
actggttgcc aaagtccgcg ggattgccgg cgaaaccatt aaagaagcgt tcaaaatccg 786480
tcaaaaacaa gcgcgttctg ccaaattgga cgaagcttgg agtgcggtaa aagaagcctt 786540
gattaccgaa gaaaccgaca ctttggcagc caacgaaatc aaaggcattt tcaaacactt 786600
ggaagccgat gtcgtccgca gccaaatttt ggatggccaa ccgcgcatcg acggccgcga 786660
cacccgcacc gtccgtccgc tgaacatcca aaccagcgta ttgccgcgca cgcacggttc 786720
tgcattgttt acccgtggcg aaacccaagc tttggccgtt gcaactttgg gtacttcgcg 786780
cgacgagcaa atcatcgacg cgctgtccgg cgaatacacc gaccgcttta tgctgcacta 786840
caactttccg ccgtactcta ccggcgaagt gggccgcatg ggcgcaccga aacgccgtga 786900
aatcggtcac ggccgtttgg ctaaacgtgc attgttggcc gtattgccga aacctgaaga 786960
tttcagctac accatgcgcg tggtctccga aattaccgaa tccaacggct cttcctctat 787020
ggcttccgtc tgcggcggct gcctgagcct gctgtctgcc ggcgtgcctt tgaaagcaca 787080
cgttgccggt atcgcgatgg gtctgattct ggaaggcaac aaatttgccg tcctgaccga 787140
cattttgggc gacgaagacc acttgggcga tatggacttt aaagtggccg gtacgaccga 787200
aggcgttacc gcgctgcaaa tggacatcaa aatccaaggc attaccaaag aaattatgca 787260
aatcgctttg gcacaggcca aagaagcgcg tctgcacatc ttggatcaga tgaaagccgc 787320
cgttgcgggc ccgcaagagc tgtccgcaca cgcgccacgc ttgttcacga tgaaaatcaa 787380
ccaagacaaa atccgcgaag ttatcggtaa gggcggtgaa accatccgtt cgattaccgc 787440
tgaaaccggt acggaaatca atattgccga agacggtacg attaccattg ccgcaaccac 787500
tcaagaagcc ggcgatgcgg cgaaaaaacg catcgagcag attactgccg aagtggaagt 787560
gggcaaagtg tacgaaggca ctgtggtgaa aatcctcgat aacaatgtcg gcgcgattgt 787620
cagcgtgatg ccgggcaaag acggtttggt acacatcagc caaatcgccc acgagcgcgt 787680
acgcaatgtc ggcgactacc tgcaagtcgg tcaggtggtg aacgtgaaag cattggaagt 787740
ggacgacaga ggccgtgtcc gtctgtccat caaagccctg ctggacgcgc ctgcccgtga 787800
ggaaaatgcc gccgagtaac gcttagggtg aaagtgccgt ctgaacaggt ttcagacggt 787860
atttttacg ggtatcggga atgaatgggg cttacagcca caggacggca agtttccata 787920
atgcccataa tgatacggat aatcccgtac acaggcggat atatcggttt tgcatgattt 787980

ttttcagttg cagggaaaaa atgccgattg ctaaaagatt gggcagcgta cccagtgcaa 788040
aggcaagcat atataacccg cccgttgccg cactaccgct tcccagcgcg taaagcgacg 788100
cgctgtaaac cagtccgcac ggcagccagc cccataatat tccgaccgca aggcaggcgg 788160
gtatggattt tatgggtaac agccggttga gtatcgggtt caggttccgc catatcggtt 788220
tgccgatttt ctcgattttt gccgccaagg aagaaatacc gctcaagtat aagcctaaaa 788280
agagcagcag gaggttggcg gccgtgtata aaatattctg caggacgcgg gtttggtcga 788340
gtgaaacgcc gacctgtccg attaatccga gtatcaggcc gattgccgta tagctgctta 788400
cccgtcctgt gttaagcagc aggatcagcc aaaagcggtt gatatgcggg gggagttgga 788460
gcgcaaacgc gctgcttaat ccgccgcaca taccgatgca gtgcgttccg ccgaagaaac 788520
cgagtaggaa cagggtgagg aaagtgatgt cgtggttcat aggcagtttg aagtcaaata 788580
tttttcggga aaagggatga tttgcggcag tccggcacat aggatccgcc gagggcattg 788640
cccgtgctgt taaagtcttg aataaggatg cagtttgcac cctgtatttc gataattttg 788700
taaaatccgc cctttactgc gccgtcggcg ggtttgccgt gtgcgtcaaa atacaggatg 788760
gtgcggtttt gaagatgcgc gcaatttgaa acggccgggt ttgccggtat gtttcgggtg 788820
caggcggcaa ggattgcaca agggaaaagc aacagtaata tgcggaacat ggtgtttctt 788880
gtaaggggta acaaacagta taatggctga ttttaatcct caggcggcgg gagatggaag 788940
catttccctt cggtgcgggg gatttcggat tcggaagcaa cagacgatac gggatttcgg 789000
aacaatatga acactttgaa atttaccaaa atgcacggtt tgggcaacga ttttatggtg 789060
attgacgcgg tcagtcagga ttttaccccc gaggacgcgc cgattgcgga atgggcggac 789120
cgcttccggg gcgtgggctt cgaccagctt ttggtggtcg ggcgttcgga aaccgaaggc 789180
gtggatttcc gttaccgtat tttcaatgcc gacggcagcg aggtcgggca atgcggcaac 789240
ggagcgcgtt gttttgcccg ttttgttgca gacaagggtt tgaccgataa gaaagaaatt 789300
tgtgttgaaa cggcaaatgg cgttattttt ccgaaattgt ccgataacgg tatggttacg 789360
gtcaatatgg gcaaaccgaa gtttatgccg tctgaaatac cgtttgtccc cgaatcgggc 789420
gagggggatg atgcctgtat ttacggggtg catctcgaat ccggcattca gcctgtcagc 789480
tgcgtcaata tgggcaaccc ccatgcggtg attgtggtcg atgacgtgga atgcgcgccg 789540
gtgcgcgaaa ccggttcgct tatcgaaccg cacaggcagt ttcccgaacg cgtcaatgtc 789600
ggctttatgc aggttgtcgg ccgaaccgcg attcgtttgc gcgtgttcga gcgcggcgtg 789660
ggcgaaaccc aagcttgcgg tacgggcgcg tgtgcggctg tggtggcggg tatccgtctg 789720
gggctgttgg atgaagggaa aacggtagag gtggttttgc cgggcgggac tttatatatc 789780
gaatgggcct gcggcggcga tgtgatgatg accggccctg cggaagcggt gtttgaaggt 789840
gagttggcgt attcatgatt ttgctgcatt tggatttttt gtctgcctta ctgtatgcgg 789900
cggtttttct gtttctgata ttccgcgcag gaatgttgca atggttttgg gcgagtatta 789960
tgctgtggct gggcatatcg gttttggggg caaagctgat gcccggcata tggggaatga 790020
cccgcgccgc gcccttgttc atcccccatt tttacctgac tttgggcagc atattttttt 790080
tcatcgggca ttggaaccgg aaaacagatg gaaacggatg gcaggcagac cccgaacatc 790140
cgctgctcgg gctttttgcc gtcagtaatg tatcgatgac gcttgctttt gtcggaatat 790200
gtgcgttggt gcattattgc ttttcgggaa cggttcaagt gtttgtgttt gcggcactgc 790260
tcaaacttta tgcgctgaag ccggtttatt ggttcgtgtt gcagtttgtg ctgatggcgg 790320
ttgcctatgt ccaccgctgc ggtatagacc ggcagccgcc gtcaacgttc ggcggctcgc 790380
agctgcgact cggcgggttg acggcagcgt tgatgcaggt ctcggtactg gtgctgctgc 790440
tttcagaaat tggaagataa tacggtgcgg ccgtatcgga acaaaaagtt ataaagaaag 790500
aaattttgga tattggtttt ttaggcggca taggtttagg ataaagccat atccgaaatt 790560
tgtttatgtt tcggcgcaaa tcccctgcaa tcggacagga tgcctatggg gattgcgcct 790620
tactgtcgaa accttattat tcaggagcag aagatgaaaa ttgcaaacag cattaccgaa 790680
ctaatcggca acacgccttt ggtcaaactg aaccgtctga ccgaaggttt gaaggcagag 790740
gttgccgtga aactggaatt tttcaatccg ggcagcagcg tcaaagaccg cattgccgaa 790800
gcaatgattg agggtgccga aaaagcgggc aaaatcaaca aaaacaccgt cattgtcgaa 790860
gcaaccagcg gcaatacggg tgtcggtttg gcaatggtat gtgccgcacg cggctacaag 790920
ctggcgatta ccatgccgga aagcatgagt aaggagcgca aaatgctgtt gcgcgcgttt 790980
ggtgcggagc tgattctgac ccctgccgcc gaaggtatgg cgggcgcgat tgccaaagcg 791040
aaatccttgg tggacgcgca tcccgacact tattttatgc cgcgccagtt cgacaatgag 791100
gcaaaccccg aagtccaccg caaaacaacc gccgaggaaa tttggcggga tacggacggc 791160
aaagtcgatg tcttcgttgc cggcgtcggc acgggcggta cgattaccgg cgtgggcgaa 791220
gtgttgaaaa aatacaaacc cgaagttaaa gtggttgccg tcgagcctga agcttcaccc 791280
gtattgagcg gcggcgaaaa aggcccgcac ccgattcaag gcatcggcgc aggctttatt 791340
ccgaccgttt tgaataccaa aatttacgac agcattacca aagtgtcgaa cgaagcggct 791400
tttgaaaccg cccgcgcaat agcggaaaaa gaaggcattt tggtgggtat ttcttccggt 791460
gcggcggttt ggagtgcgtt gcagcttgcc aaacagcctg aaaacgaagg caagctgata 791520
gtcgtgctgc tgccttctta tggcgaacgc tatctctcta cgccactttt tgcagatttg 791580
gcataatgct ttaatcggat tgtcgaaaca ttcagacgca tttttcggta tcggtgtaac 791640

gccgtgccgg aaaatgcgtt tttgcatata tgccgaaaac gccggttgtg ttttaatcag 791700
gtgttggtgt cgccgcatcg cttgagggaa atattttta tagtggatta acaaaaatca 791760
ggacaaggcg acgaagccgc agacagtaca aatagtacgg aaccgattca cttggtgctt 791820
cagcacctta gagaatcgtt ctctttgagc taaggcgagg caacgccgta ctggtttttg 791880
ttaatccact atattcgggt tttatttggc aggacggttt tttgccccaa cggaaaatag 791940
cctgcctgcc cgtaaaatca gccgtttgtc cgggtgcagc cggggctttg ggcttcagac 792000
ggcatatttt cggaatggcg gcattcttgc cgtcggcgcg gcagccgtat ggggaaggga 792060
ggggatattg tggtcggtaa cggcaaaaaa tatgccgcac cattgctggt gctgggttgc 792120
gtggtgttcg gtctgggcag tctgattgtc agatccgtcc ccgtcggttc gtatgcaatc 792180
gcattttggc ggttgctgat ttcggtgttc gtattttggt ttttagcacg gtttttcagg 792240
caaaaattcc caaaaaacag gaaaaccgtc cgatatgccc tgacggcggg cgtgtttctc 792300

gctttcgatt tggcgttgtg gcacgaaagc atacacgcgg tcgggccggg tatttccacc 792360
ctgctcaaca gcctgcaaat ctttttcttg tcggcaatcg gtgttttctt tttcggcgag 792420
cgtttgagcg ggctgaaaaa ggcaggctta atatcggcag ttgccggcgt ggcgatgatt 792480
gccggtgcgg aattcggcta caacggtaat gcggtttggg gattcgccag cggtttggta 792540
tcgggactga tgctcgccct gtcgatggtg tttgtccgca aaacccatga aatcgagccg 792600
gtggcgcttt tcccttcaat gatgattttg agtttgggcg gcgcggtatc gctggttgtt 792660
ccggcattgc tgatggatgg cggcgcgctt tatccgacga cttggaaaga tgcgggtttg 792720
gtgcttgtgt acggcgtggt gatgcagtgc ttcgcgtggg cgatggttgc ctatgcgatt 792780
ccgctgcttt cgctgtcgct gacggggctg ctgcttttgt ccgaaccggt tgccgccctg 792840
ttcatcgatt atttcgggtt gggcaaaccg attgaaggcg tgcagtgggc aggggtggcg 792900
ctgacgcttt cggcaattta cctcggttcg ctgaaacggc agtcttcaca ttgatttcat 792960
cagggcaata ttggcagttc gccgtgcaaa acctgcgtat attggactga taggtaggaa 793020
aatccgacaa cgttagactc gcctgtaaaa gtgaggaata gcaaatgccg tctgaaacta 793080
ttttcagacg gcattcttgg cttcctggcc taacggattg ccgtaccgga cctgccgaaa 793140
tcgccgaagt tcatcaaaat gaacattgcc ttgccgacaa ccagcttgtc atccacaaat 793200
ccccagtagc gcgaatcggc actgttgtcg cggttgtcgc ccatagcgaa atagcgtcct 793260
tcgggaactt tgcacacgaa accgctgccg tcgtcggcat attggcagtg ttccaaaccg 793320
ctttgctcta tggaatatcc gttttcagac ataatatcgg aggtatattt gcccaatacg 793380
ggcagggaaa cggcaggctg tccttctttt ttcagaatat tgaaggattt gccgtctaga 793440
ccgctgcgga acatatccgt gttgtggatt tcggaagggt cggtgtcgtc gggataacgg 793500
tatgtgccgt caggaatgtc ggaagtgggt ttgccattta ccgtcaaaat cttatcccga 793560
tattcgacca catcgcccgg aatgccgaca atacgcttga tgtaggtcat ctccggctgc 793620
agaggataat taaaaacaac gacatcgccc cgttcgattt tgcctgtagg aataaatata 793680
ttgtttaaaa cgggtacgcg caggccgtag gaaaatttgc cgaccaaaat gaaatcgccc 793740
ttgatcaggc ccgggcgcat cgagctggac gggatttgga acggttcggc gataaacgac 793800
cggatgagga acaataccaa aacggtaggg aagaaactgc cgaaataatc gccgaagtgg 793860
ctgctttccg agatttcggg atgagtcttc aggcggtatt tatatacccc ccaagccgta 793920
ccgcacaata caacgaaaat caggaaaacg gcggtaaagc tcataaacag ggacaaagcg 793980
gcaaacacgc cgaccgctgt caggatatag gcgtattcaa ggccggaact ccattccccg 794040
ttttcctgcc gcttcttgtc gcttttgaaa taaaggatga tgccggcaag cagcgcggca 794100
gccgcgcccg acattagcat tgtgttcatt gttgttcctt aatgcttaaa aacccgcctg 794160
tccgtgcaac cgttttaagg cggcaaattg caaaatttgt ttgcgggcgc gtgcccctga 794220
aatcagggcg gtttgagggg tgttcccgac gcgccgccct gtgtgccgga gttatttgtc 794280
gctcacctgc aaaatcgcca agaacgcgct ttgcggaatt tccacattgc ccacttgttt 794340
catacggcgt ttacctgcct tttgttttc aagcagtttt ttcttacgcg taatatcgcc 794400
gccgtaacat ttcgccaaga cgtttttacg cagtgctttg acgttttcgc gggcgataat 794460
ctggctgccg atggcggctt ggacggcaat gtcgaacatt tggcgcggaa tcagctcgcg 794520
cattttcgat gctagctcgc ggcctcggtg aaccgcgctt tgacggtgca caatcaggct 794580
taaggcatcg actttttcgc cgttgaccat aatatccagc ttaatcaaat cagacggttg 794640
gaactctttg aaatgataat ccaacgaagc atagccgcgc gaagtggatt tgagtttgtc 794700
gaaaaagtcc atcaccactt cgttcatggg caaatcgtaa gtcagcatca cttggcggcc 794760
catgtactgc atattgacct gcacgccgcg cttttggtta cacaaagtca tgacgttgcc 794820
gacgtattcc tgcggcacaa ggatggtcgc ggtaataatc ggctcgagta tggtttcgat 794880
gctgccgatg tcgggcagtt tggacggatt ttcgacttcg attttttcgc cgcttttcaa 794940
cacgacttca taaatcaccg tcggcgcggt ggtaatcaaa tccatatcga actcgcgctc 795000
caagcgttcc tgcacgattt ccaagtgcaa cagacccaag aagccgcaac ggaagccgaa 795060
acccaatgct tgggaaacct caggctcaaa tttcaacgaa gcatcgttaa gctgcaattt 795120
ttccaaagca tcgcgcaaag cttcgtagtc gtggctttct acgggataaa gtccggcgaa 795180
tacctggctt tgcacctctt ggaaaccggg cagcggctca gtggcagggt tggcaaccaa 795240

agtaaccgta tcgccgactt tcgcctgtcc caattctttt acgccggtaa tcaaaaagcc 795300
cacttcgccg gcttttagtt cttgtttttg aactgatttc ggtgtgaata cgcccagctg 795360
ctcgacctgc gtttccgcct tggtgctcat aaagcgcact ttgtctttca gtttgatggt 795420
gccgtttttc actcgaatca gcataaccac gccgacatag ttgtcaaacc acgaatcgac 795480
gataaccgct tgcagcggcg cgttttcgtc gccggtcggt gcggggattt tggcaacgat 795540
ttcttccaaa acgtcttcca cgccgatgcc gcttttggcg gaacattgca ccgcgccgac 795600
ggcatcgatg ccgatgatgt cttcgatttc ctgttccacg cgttcggggt cggcggcggg 795660
caggtcgatt ttgttcaaaa ccggcacgac ttccacgccc aaatcaatcg cggtgtagca 795720
gttcgccacg gtttgcgctt ccacgccttg cgacgcgtca acgaccaaaa gcgcgccttc 795780
gcaagccgac agcgaacggg aaacttcgta agagaagtcg acgtgtcccg gcgtgtcaat 795840
caggttgagt tgatacacct gcccgtcgcg tgctttatag ttgagcgcgg cggtttgcgc 795900
tttgatggta atgccgcgct ctttttcgat gtccatggaa tcgagcacct gcgtactcat 795960
ttcgcgcaaa tccaaaccgc cgcagtattg gatgaagcgg tcggcaagcg tcgatttgcc 796020
gtggtcgatg tgggcaatga tggagaaatt tcggatattt ttcattagag ttgttttgaa 796080
tgtcggacag tgggtttggg aaatgccgtc tgaacaaacg gcgttgcgtc cgaatatcgg 796140
gtgcaacgtg gaaatagccc gttattctaa cggaaaaccg ctgttttggc ataagtttga 796200
taaaggtctt ataaagattt gacgatttct gccaccattt ttgcggaatt tgccgccgcc 796260
gttttcaaga actcgtcaaa gctgatgtct gctttttcat ctgccgaatc ggaaaccgcg 796320
cggatgatga cgaaaggcgt ttccaactga tgacaggttt gggcgattgc cgccgcttcc 796380
atttccactg ctttgacttc ggggaagtgc ttgcggattt ccgccacgcc ttcgctgctg 796440
tggacaaagc ggtcgccgct gacaatcagc ccttgttcta ccgccgcgcc ttcaaacgtc 796500
cgcgccgccc gttttgccgc ctcaatcaaa atgccgtctg aagcaaacct tgccggcagt 796560
tgcggcactt gtccccaggc atagccgaat gcggttacgt cgacatcgtg gtgtgcggtt 796620
tccgtgccga tgactacgtc gccgactttc aaacccttgc ccaaaccgcc cgcgctgccg 796680
gtgttgatga cgcagtccgc tgcgaattca cggataatcc aagccgttgc aaccgccgcg 796740
ttgaccttgc cgatgccgct caatgcaagc accatgcgtt ttcccgccaa ttcgccttca 796800
taggcggaaa atctgccgaa agagacggct ttgacatttt ccatcatctc gcgcaaaagc 796860
tcgatttctt gttccattgc gccgataacg gctactgttt tcaaagacat attgctgacc 796920
tgttgtgaat ttcggataga atgcctgatt atacacgcta acacggcagg attgagtgga 796980
ggtggtttgt ccgtgccgtc tgaaacggtt tcagacggca cggcgggttt ttggtagaat 797040
gggaaggtac agattgtttg aagattaggg gacgaggatg tttaccgatg aaaatatgac 797100
cgcaaaggaa gaactgttcg catggctgcg ccatatgaac caaaacaaag gttccgacct 797160
gttcgtgaca acccatttcc cgcccgcaat gaagctggac ggcaaaatca cccgcatcac 797220
ggacgaaccg ctgacggcgg aaaaatgtat ggaaatcgcc ttttcgatta tgagtgcgaa 797280
gcaggcggaa gaattttcat cgaccaacga gtgcaacttc gccatcagcc tgccggacac 797340
cagccgcttc cgcgtcaatg cgatgataca gcgcggcgcg acggcgttgg tattccgtac 797400
gattaccagc aagattccca agtttgaaag cctgaacctg ccgccagtct tgaaggatgt 797460
cgcgctgaaa aaacgcgggc tggttatttt tgtcggcggc accggctcgg gtaaatcgac 797520
ttcgcttgcc tcgcttatcg actaccgcaa tgaaaattcg ttcggacaca tcatcaccat 797580
cgaagacccg atcgagtttg tccacgaaca caaaaactgc atcatcaccc agcgcgaggt 797640
cggcgtggat acggaaaact ggatggcggc gttgaaaaac acgctgcgtc aggcgcctga 797700
tgtcatcctt atcggcgaaa tccgtgaccg cgaaacaatg gactacgcca ttgcctttgc 797760
cgaaacgggg catttgtgta tggcgacgct gcacgccaac agcaccaatc aggcactcga 797820
ccgcatcatc aacttttttcc ccgaggagcg gcgcgaacaa ttgctgacgg atttgtcgct 797880
caaccttcag gcgtttattt cgcaacgcct cgttccgcga gacggcggca agggcagggt 797940
ggcggcagtc gaggtgctgc tcaattcgcc cctgatttcg gagttgattc acaacggcaa 798000
catccatgaa atcaaagaag tgatgaaaaa atccactacc ctgggtatgc agaccttcga 798060
tcaacacctt taccaattgt atgaaaaagg cgatatttcc ctgcaagaag cattgaaaaa 798120
tgccgattcc gcacacgatt tgcgtttggc ggtacagttg cgcagccgcc gcgcgcaaag 798180
ttccagcccc gatttggaac tgctctgatg gcggtatgga tttccggacg gatggtttga 798240
aatgatttat ccgtggcata atgagcaatg gcggcagatt gcggaacatt gggagcgtcg 798300
tcccaatgca tggctgtttg ccggcaaaaa agatacgggg aaaactacat ttgcccgctt 798360
tgcggcgaag gcactgttgt gcgaaacccc tgcaccgggc tgcaaaccct gtggcgaatg 798420
tatgtcctgc catctgtttg gacagggaag ccatcccgat ttttacgaaa tcaccccctt 798480
gtcggacgaa cccgaaaacg gacgcaaact gttgcagatc aaaatcgatg ccgtcaggga 798540
aatcatcgat aatgtgtacc tgacttcggt acggggcggt ttgcgcgtga ttctgattca 798600
tcctgcggaa agtatgaatg tccaagccgc caacagtttg ttgaaagtgt tggaagaacc 798660
gccgccacaa gtggtctttt tgctggtcag ccacgcggcg gacaaggttt taccgaccat 798720
taaaagtcgc tgccggaaga tggttttgcc cgctccttcc catgaagagg cattggcata 798780
tctgcgtgaa aggggtgtgg cggaacctga ggaacgtctg gctttccatt ccggagcgcc 798840
gctgtttgat gaggcggacg gtgtccgtgc gttgcggatt aaactgttgg atattttggc 798900

agaaccaagg ttgttgaaga ttttggatta cgccgcgctt ttcgataagg aaaaacttcc 798960
gctcgccgta tttgtcgggt ggatgcagaa atggctggtc gatttgggat tgtgcctgca 799020
acacatgaaa cccgtctatt atcccgctta tgaagacagg ctgcttcaga cggtatccgg 799080
tttccgtccg cgcaatgtat ttgcggcgga ggatatgctc aaacagcttg ccccctacgg 799140
gtttcatact ttaaatgtca aaatgcagat cgagcatctg ctcatcaact atttggaatt 799200
gaagaaagag aacgggtgaa ttatgtcaga cggacaaaat attccggcaa aaatgatgtc 799260
gttgcagctg aaagacatga atctgctgta cagctcctac atgccgtttt tggaacacgg 799320
cggtctgttt gtgcagacca acgacgtatt ttccatcggg gacgatattc tgcttgccgt 799380
agaaatcctc aacttcccca aactgttcct gccgaccaaa gtcgcctgga tcaatcctgc 799440
gcgtacttcc tccaaaccca aaggggtggg gctggcattc acaaaacacg aaaactgcct 799500
gaaagtcaaa gaccagatcg aagtcgaact gggcaacaca atcggcggca gcagacctac 799560
gtttaccatg taacgccatg catatcatcg attcgcactg ccacctcaat tttgaaggtt 799620
tgaaagaacg cctgcccgaa gttttgtcca acatggaagc aaacggcgtg ggcaggcac 799680
tcgccatcag cgtcagtagg gaaagcttct ccgaagtctt tgccatcgcc gaagcgcacg 799740
aacacatcta ttgcaccata ggcgtacatc ccgacagcaa ggaagccgaa gaattttcca 799800
ttgcggaaat ggtcgaagcc gccgcccatc cgaaagtggt cggcatcggc gagacgggtt 799860
tggattatta ctggtgcaaa ggcgatttgt cctggcaaca caaacgcttt gcagaccaca 799920
tcgaagcagc caatcaaacc ggactgcccg ttatcgtcca tacgcgtgat gcggcggcgg 799980
acaccttgtc tatcctgaaa gaatgccggg ttaattcggg cgttatccac tgtttttccg 800040
aagacatcgg ttttgcccgt gcagcaatgg atttggggct ttatatttct ttctcgggaa 800100
tcgttacctt taaaaacgca cccttggttc aggaggcggc gaaatatgtg ccggacgacc 800160
gcattttggt ggaaaccgat gcgccgttcc tcgccccccgt tcccaaacgc ggcaggcaga 800220
acgaaccggc ttttgtgcgc cataccgccg aacatatcgc caaattgcgg aaccaaacat 800280
tggaacaggt tgcggcatat acgacggaaa acttttaccg gctgtttaaa aaagtacccg 800340
atatgcggac cgtctgaccc tgtaccgacg ataaggaaaa ccatgaaggc aattcatccg 800400
tatgcatgtc cgcgctgctg ccggctgcct gccaacacgt ttcggacagg catggcaaat 800460
tccgcttcca aattctgcat tgccaaaggc ggcagacggg aagcaaaaaa agacgaaagc 800520
ggcggcggat atgccctgtg ccatttgccg gacagcagga ttgtcgagga gtgggaatat 800580
ttccgttcac aatattgata ctgcgcgata tacggcaaat attgtgggaa gtttccgctt 800640
ttgcgtataa tgcgccctac ctgacaaatt ttgtcaactt tatcaaaagg aataagcgat 800700
ggcttccatc cacgaccaaa ttaaagaagt agtaacgaca caccgcgtcg tattgtttat 800760
gaaaggtacg aagcagtttc cgcaatgcgg tttctcttcc cgcgccgtgc aaatcctgaa 800820
cgcggcaggc tgcaccgatt acgttaccgt caacgtattg gaaaatcccg aagtgcgcca 800880
aggcattaag gaatacagcg actggccgac catcccccaa ctttatgtga acggcgagtt 800940
tgtcggcggt tcggacatcc tgatggaaat gtatgaggca ggcgagctgc aagagctgct 801000
gaaagcctga tggattcggc aatgccgtct gaacgtgttt cagacggcat tttcttttcc 801060
ggcaaatcaa aaaaaagtat aatggcgcgt ctcaaaatca cattggaaca ccgcgatgaa 801120
cgttaatgtt atcaaccatc cgctcgtccg ccacaaatta accctgatga gggaggcgga 801180
ttgcagcacc tacaaattcc ggacgcttgc caccgagctg gcgcgcctga tggcatacga 801240
ggcaagccgt gattttgaaa tcgaaaaata ccttatcgac ggatggtgcg gtcagattga 801300
aggcgaccgc atcaagggca aaacattgac cgtcgttccc atactgcgtg caggtttggg 801360
tatgcttgac ggtgtgctcg acctgattcc gactgccaaa atcagtgtag tcggactgca 801420
gcgcgacgaa gaaacgctga agcctatttc ctattttgag aaatttgtgg acagtatgga 801480
cgaacgtccg gctttgatta tcgatcctat gctggcgaca ggcggttcga tggttgccac 801540
catcgacctt ttgaaagcca agggctgcaa aaatatcaag gcactggtgc tggttgccgc 801600
gcccgagggt gtgaaggcgg tcaacgacgc gcaccctgac gttacgattt acaccgccgc 801660
gctcgacagc cacttgaacg agaacggcta catcatcccc ggcttgggcg atgcgggcga 801720
caagattttc ggcacgcgct aactgactga ttttcggagt tgatatgaat tttcaagact 801780
atctcgccac atttccttca atcgaccatc tgggcggttt ggacgttcag gatgccgacg 801840
gcaaaacggt tcaccacatt cctgccgttc agggtaagct cggttcgctc aagctgtaca 801900
atgctttggc ggaacgtttt gacggaaaat tgggtaaaga agcggcagaa cagggtttga 801960
tatggtttgc cgaacacgtt gccgacgcgc gtgcccatcc gggcaagcat ccgaacatcg 802020
atctgctgga aaatgtcgtg caaagcggtg aaacctggtt gctcaagccg ctttccgcgc 802080
aataattttc gaccatgccg tctgaaatcc gtttcagacg gcattttgtc ggaaagaaga 802140
ccgtaaaacg ggcattttct tttctatttc aggatacggg caatgatgtt tcaacacaca 802200
ggacgacaca taaagcgccg ccctatgtgt tgccctaatt tggaaggggt tacacccttt 802260
tcaaataaaa tctgatgctg ctgccacgaa ggacggatgt ccgagtggcg gggtttcaac 802320
cattaaggaa atacgatgaa aaaaatgttc ctttctgccg tattgcttct gtcggctgcc 802380
gcccaaaccg tgtgggcgga tacggtgttt tcctgtaaaa cggacaacaa caaatacata 802440
gaagtccaaa aaatcaaccg caatctttac gaatattcgt tcggcagtgc ggcaaaaaaa 802500
gaaattgcca tacgcaacag caaagctgac ctgttggggc gttccgacag gtggcaaggt 802560

atgggcagcg gtcgttgggc aacgatgaaa ttccaaaacg gcgaatttat gtacaccata 802620
tggacaggct tcgattccgt gactcatacg gaaagcagcg gtgtcgttgt ggagcgtagg 802680
ggcaaggaag tcgcacgggt cggctgtacg ccgaaaaccg cgcaggcgaa tttcaacgat 802740
gacgattttt cctagtaatc ggggcggata aggcgatgga aacagcgaaa cccgtcatgc 802800
tgattgtccg tccgtcgggc agggccgcag aagatgtcga aacttgcctg aatgccggtt 802860
ggcgcgcgga agtattgagt ccggtcgaaa tcgaagcaga tgctgccgga ctggaacttt 802920
tgtccgaaca atatgcccgt gcggatgccg tgttttgggt cagtccgacc gccgttgaaa 802980
ccgccgtccc gtaccttaac ctttcagacg gcataaaggc gcagattgcc gtagggcagg 803040
gcagccgccg cgcattggaa cgctgtttgg tcagaacggt catcgcgcct gatgacggca 803100
acgacagcga ggcggttttg cgcctgccgg tttggaacag tctgcccgaa ggtgcgcgcg 803160
tattgtttgt gcgcggacac ggcgggcggg attttttgat gaatgccttg caggagaaag 803220
gttttcggac ggaggtggca gaagtctatt tcagacggca taaacctttg aactttcaaa 803280
atttccaaac cgaaaatatt gccgccgcct atattacgtc gaccgagctg gtgcgcttgc 803340
tgttcgggca gcttccgccg caattttccc gattcttcaa atccttgcta tactttaccc 803400
atcatccgcg cattgcggag gcattgaagc gcgaaggcgt gtgttcggtc gaaaccgtcc 803460
ctacgctgga agccgcgctt tcccattctt ccatttccgt ttcagacggc atggtctttc 803520
ccggaacctc aaattaataa ggagcaaaac ggtgggcgaa cctgaaaaca aatcatccga 803580
acccgtacgc gagatacagg catcaaaaga aatgccgtct gaaacctctt ccccacgcaa 803640
agaaaacgaa acagaagtac acattcctgc cgctcctttt atcgtcaaac agtccggcag 803700
caacgctttg gcagtctgcg ccctggtatt ggcggcattg ggtttgggta caagtggttt 803760
tttgtttgtc caaggacaga atgtcttgaa aaaccaagag ctggcattca accaaaaaat 803820
cgacaaagcc gccttgggcg agtcggaaaa cgccgccctg ttgaaagaca acctcaaccg 803880
gcaagccgcc atacaatcag agctcgaccg tttggacgga aacgtcaaag caaacggcga 803940
acaaatcttg gaaatgcaaa aatcctatcg cgagttgacc aaaggacgcg ccgattggct 804000
ggtggacgaa accgagacca tactcaatct ggcggcgcaa cagctggtgt tgactggcaa 804060
tatccaaacg gcagtcggcg tattggagca tatcgacagc cgcctgtccc gtttcaatca 804120
ggcagagctt ctgccgatca agcaggcggt cagcagcgac ttggcggaac tgaaaaaccg 804180
tccctatgtc gatatttccg gcacggcatt gcgcctcgac aggctggaaa ccgccgtatc 804240
cggactgccg ctgatgctcg acggcgtgct gaaaccgggc gtacaggtga agaacgaagc 804300
cgcttccgct tcatggtggc agaacgtatg ggaaaaatcc ctcggcacat tgaaggggct 804360
ggtcgaaatc cgacgtttgg aaaacaacga tgccatgctg atttctcccg aacaggcata 804420
ttttgtgcgt gaaaacctgc gcctccgcct tttggatgcg cgcactgcat taatgcagcg 804480
caacagcgaa gtctatcagg gcgatttgaa caatgccgaa gccgccgtca gacagtattt 804540
cgatgccaag tctcccgcca cgcagtcgtg gctgaaagaa ctggcggaat tgaaggcgtt 804600
ggatgtgcgg atgactgcgg atgacggttt gaaaaacagc ctaaatgccg tccgcgccta 804660
tcgcgacggt acgcgcatga cggcggcgga aaatcaagaa gcggaacagg cggcttccga 804720
accggcaaac gaaaaaacag cttccgaacc ggctgccgca tcggatgtga agaccataga 804780
agcaccgtcc ctgccttcgg aacgcaaacc ggaacagcct gcaaaaaaac agaccgtacc 804840
ggaaaaggca gggcgttcgc cgtccgctaa aggagaacgc gcatgaaaac ggtagtctgg 804900
attgtcgtcc tgtttgccgc cgccgtcgga ctggcgctgg cttcgggcat ttacaccggc 804960
gacgtgtata tcgtactcgg acagaccatg ctcagaatca acctgcacgc ctttgtgtta 805020
ggttcgctga ttgccgtcgt ggtgtggtat ttcttgttta aattcattat cggcgtactc 805080
aatatccccg aaaagatgca gcgtttcggt tcggcgcgta aaggccgcaa ggccgcgctt 805140
gccttgaaca aggcgggttt ggcgtatttt gaagggcgtt ttgaaaaggc ggaactagaa 805200
gcctcacgcg tgttggtcaa caaagaggcc ggagacaacc ggactttggc attgatgctg 805260
ggcgcgcacg ccgccggaca gatggaaaac atcgagctgc gcgaccgtta tcttgcggaa 805320
atcgccaaac tgccggaaaa acagcagctt tcccgttatc ttttgttggc ggaatcggcg 805380
ttgaaccggc gcgattacga agcggcggaa gccaatcttc atgcggcggc gaagatgaat 805440
gccaacctta cgcgcctcgt gcgtctgcaa cttcgttacg ctttcgacag ggcgacgcg 805500
ttgcaggttc tggcaaaaac cgaaaaactt tccaaggcgg gcgcgttggg caaatcggaa 805560
atggaacggt atcaaaattg ggcataccgc cgccagctgg cggatgctgc cgatgccgcc 805620
gctttgaaaa cctgcctgaa gcggattccc gacagcctca aaaacgggga attgagcgta 805680
tcggttgcgg aaaagtacga acgtttggga ctgtatgccg atgcggtcaa atgggtcaaa 805740
cagcattatc cgcacaaccg ccgccccgag cttttggaag cctttgtcga aagcgtgcgc 805800
tttttgggcg agcgcgaaca gcagaaagcc atcgattttg ccgatgcttg gctgaaagaa 805860
cagcccgata acgcgcttct gctgatgtat ctcggtcggc tcgcctacgg ccgcaaactt 805920
tggggcaagg caaaaggcta ccttgaagcg agcattgcat taaagccgag tatttccgcg 805980
cgtttggttc tagcaaaggt tttcgacgaa atcggagaac cgcagaaggc ggaggcgcag 806040
cgcaacttgg ttttggaagc cgtctccgat gacgaacgtc acgcagcgtt agagcagcat 806100
agctgatttt gggaaatatc tttatctggg agaatttgat ggggtcttca gattccttta 806160
aggaaaagaa agaaatattt gaaattggaa cgcctgctta tcgccaaaag ttaattgatg 806220

```
tttggaaaaa gagcattaat ggaaacgaaa aatcttgggt gctctttgaa aatgggactt 806280
gcgtcatttt acttgaaccg gaaaaagatt tggcgaaaca agctaaagag atgttaagca 806340
aatggggcaa ggttcaaata ggaacaccat ctgcagattt tggcattatc actttagata 806400
gtggcgatgg atatgccgtt tcatgccatc atcccgaaat ttttacgcta atcctaaaag 806460
aagaaggatt ggatgaagat ttcaaaatcg gtatcgaagg gcgctctcat cgcgattgtg 806520
atgctgaaga acccaaagtt atccatatcg aagataaacg caccattgaa accccatgaa 806580
aacctgctgc cgtttaatca tctactgatg attacttagg caaatgtgcc cgtccctccc 806640
ttttcagacg acctttcatt gcggaaaccg ccgcaaaggt tgtctgaaaa ccgttttcct 806700
tccccgtttt acaaacaaac cgaaagcccc acatgatctc tttgaaaaac gacactttcc 806760
tccgcgccct gctcaaacaa cctgtcgaat acacgccgat ttggatgatg cgccaggcgg 806820
ggcgttatct gcccgaatac aaagccacac gcgcgaaagc gggcagcttc ctcgatttgt 806880
gcaaaaacac cgaattggcg accgaagtta ccatccaacc tttggaacgt ttcgatttgg 806940
acgcggcgat tttgttttcc gacatcctga ccgtccctga cgcaatgggc ttgggactgt 807000
attttgccga aggcgaaggc ccgaaattca aacgcgccct gcaacacgag gccgacatcg 807060
ccaagctgca cgttcccgat atggaaaaac tgcaatacgt tttcgacgcg gtaacttcca 807120
tccgtaaagc attggacggc cgcgtaccgc tcatcggctt ctccggcagt ccgttcacgc 807180
tcgcctgtta tatggtcgaa ggcggcggca gcaaagaatt ccgcaccatc aaaaccatga 807240
tgtactcgcg ccccgatttg ctgcacaaaa tcctcgatac caacgcccaa gccgttaccg 807300
cctacctcaa cgcccaaatc gacgcgggcg cgcaggcggt gcagattttc gacacttggg 807360
gcggcgtgtt gagcgatgcg gcgtttaaag aattcagcct caaatacatc cgccagatcg 807420
tcgccggact caaacgcgaa agcgaaggcc gccgcgtgcc tgttatcgta tttgccaaag 807480
gcggcgggct gtggctggaa agtatggccc aaatcggcgc agacgcattg ggcttggact 807540
ggacgtgtaa catcggcgaa gcacgccgcc gcgtcggcaa gcaagtcgcc ctgcaaggca 807600
acttcgaccc gttcgccctc ttcggtacgc cggaatccat ccgcaccgag gtcgcacgta 807660
tcctagccga ctacggacac ggcagcggcc atgtcttcaa cctcggacac ggcatcaacc 807720
aacacgccga ccccgaacac gccaaaatct tagtcgatac cgtacacgag ctgtctcggc 807780
agtatcacgg cgggtaagcc ggcaggaaac cgcccgatat gccgtctgaa gccgagagat 807840
ggccggttag ggtaaaaata aggcaatgcg gcaatatccg ccgtgtacgg atagtacatg 807900
acggcggcgt tgtcgtattg gcgcaatccc aaccgtccct atgttcagac ggcatttttg 807960
ttttcagatg cagggaaaac cgatggcaaa aacgcttaaa accctttacc aatgcaccga 808020
atgcggcggc acttcgccga aatggcaggg caaatgcccg cattgcggcg agtggaacac 808080
gcttcaggaa agccttgccg cgcccgagcc gaaaaacgcc cgtttccaat cttgggcggc 808140
ggatacctcg accgtccaat ccctctccgc cgttaccgcc accgaagtgc cgcgcaatcc 808200
gaccggtatg ggcgaactcg accgcgtatt gggcggcggt ttggtcgatg gtgcggtcat 808260
cctgctcggc ggcgaccccg gcatcggcaa atccacgctg ctgttgcaaa ccatcgccaa 808320
aatggcgcaa agccgtaaag tgctatacgt ttccggcgaa gaatccgccc aacaagtcgc 808380
cctgcgcgcg cagcgtttgg aactgccgac cgacggcgta aaccttcttg ccgaaatccg 808440
catggaagcg attcaggcgg ccttgaaaca gcatcagccc gaagttgtcg tcatcgactc 808500
tatccaaacc atgtattccg accaaatcac gtccgccccc ggctccgtgt cgcaggtgcg 808560
cgagtgtgcc gcccaactga cgcgcatggc gaaacagatg ggcatcgcca tgatactggt 808620
cggacacgtg accaaagacg gcgcgattgc cggcccgcgc gtgctggaac acatggttga 808680
taccgtgctg tatttcgagg gcgaccaaca ttccaactac cgcatgatac gcgccatcaa 808740
aaaccgcttc ggcgcggcaa acgaactggg cgtgttcgcg atgacggaaa acggtttgaa 808800
aggtgtgtcc aacccgtccg ccatcttcct cgccagctac cgcgacgata cgcccggctc 808860
gtgcgttttg gttacacagg aaggcagccg cccgcttttg gtcgaaattc aggcattggt 808920
cgatgacgcg cacggcttca cgcccaaacg cctcaccgtc ggactggaac aaaaccgtct 808980
tgcgatgctg cttgccgtgt taaaccgcca cggcggcatc gcctgtttcg atcaggatgt 809040
gttcctcaac gccgtcggcg gcgtgaaaat cggcgaaccg gcggcggatt tggcggtcat 809100
cctcgcgatg ctttccagct tccgcaaccg ccctatgcct gaaaaaaccg tggttttcgg 809160
cgaaatcggc ttaagcggcg aagtccgccc cgtcgcacgc gggcaagagc ggctcaaaga 809220
agcggaaaaa ctcggcttca aacgcgccat cgtccccaaa gccaatatgc cgcgcaacgc 809280
caaagagttt ccgaacctga aaatctacgg cgtttcgagt ttgcaggaag ccatcgatat 809340
ttgccgcgac agcagggaat aaacggaaat gccgtctgaa atcgggtttc agacggcatt 809400
tggtttgtgg cggattgaaa caagaaggca taccggcgac agataagatt tgcggcaaag 809460
ttgcctgtga tgtggcaaaa acacacacgc ccgtcatccc cgcaagggtg ggaatccgga 809520
atcgtccgtt tcggcaatga ttgaaaatca cggtaaccca accgattgga ttcccgactt 809580
cgtgggaatg aggggcgtgt gcatttgatt tccatccgcc atatgtcggc gacgggctta 809640
ttcgcctacg gtttttgta tcagtttttc ggcgtttgcc aaagtgtttg ccacttcgtc 809700
gaaaccgatg cggctgccgg cgatgagggc gcgcgtatcg gtataggcgg cgcgtacttt 809760
gccgtccgtt tcggtaacga ggacgcgtag gggcagttgc agggcgaagg cggggtcttt 809820
gaccatcagc ggcgtgccgg ctttgggcgt gccgaagacg atgacttttg ccggctgcat 809880
```

cgttaagccg tttcggcggg cggcttcctg atggtcgatg acggcaaaaa tgtccatccc 809940
tttgcttttt atggcggttt caaggcggct gacggtttcg tcaaaactgt attttgaggt 810000
gagggtatgc gtggtcatag cggtttcgtt ttgggtggac ggttcgctgg caggatgtgc 810060
cgaagcggtt gaaatgcaga gtgcggatgc ggcaatcagg gggagtatgt gtttcatcgt 810120
atttcctttt tcctttttgg ttgaaacggt agaatcagac tttattcggg aggggtgtaa 810180
ccctttccaa atcagggcaa cacatagggc ggtgctttat gtgtcgtgaa acatcattgt 810240
tccttatcgg tttgtttatc aggcttcgga cggggcggcg cgcgccgcgc ccgatgttgc 810300
gccgcgtgcc ggaacgccgt atgccgtctg aaagcctgtc ctttcagacg gcattgcgtc 810360
atttcatccc ttttttgagc aggtcttcat aaccgccgtg attggcaaca tttgtataac 810420
ctgctttttt cagctcttga agggcggctt cggcacgccg tccgctgcgg cagtagaggt 810480
tgaccggcgt gtctttgtcg ggcgcggctt cgtgtatgcg gcggacgatt tggtcgacgg 810540
ggatgttgac cgcgttgtgc aaatgccctt cgctaaattc ctgttcggaa cggacatcga 810600
tccaaacggc cggatgttgc gcggtttggg cggcggatac gggtttttgc ggggctgcct 810660
gcgcggcaaa ggcggctgag gcaatgagtg cggcggtaat caggtgtttg atattcatag 810720
ggttttcctg cggttgttgt ccgaaaggac gggaagttat tttatctgtt ccaaagcggc 810780
ggcatctatg tcccaacgcc aaacgccgcc gcctttgcca tccaatccgc gcaaaaacag 810840
gtagcggacg gaaacggcgg cgggcggttg tccgcgcagc ttgaagtagc gtgcggcggc 810900
aacggcgtaa atcagtgcct gaaggtaata gtggtggtgt gcgacggctt cgtccattgc 810960
ctgttgcgtg taggcggatg cgtccgtacc gaggtggttt gatttgtagt cgatgacgca 811020
gatattgccg tcggggtctt ggcagaccat atcgacaaag ccgtttaaaa agccgttgac 811080
ggtgtggaag tcgagcgttt cggcagcggc acggcagact tcgggcagcc tgatgtcgtc 811140
gcgggcaaac cagtcgcgca ggcgtttgag gctgaagtct tcggtgtgga gggtaaagcc 811200
catttcggga cagcggcact cgggtgagat gtcggacagg tcgtatgccc ccgtcagcgg 811260
cgttttgcgg caggcttccg ccatttcggc aacggcgggc agccatattt cttcaaaacc 811320
gtatttttc agcttgtcgg caatgagggt ttcctgtccg gcggctgctt gtccgaattt 811380
gaaatcttca agaatttcgt gcaggcacag ccccgcctgc gtgcctttcg gaaaatcgtg 811440
tatcgatatg ccgtctgaag ccgtcggcgt ttcagacggc atcgccggca ccgaggtttc 811500
ggcggcatcc aaggacgggc aggcatcttc ttcgccgccg tcgggcgttt gggtatggcg 811560
gcttaaggcg gtaaagctag tgtggcggac aaatcggaat ccgcgttcgg gaatgctgtt 811620
tgcggcaaat tcggcggttt gaccggcgtt gctgcgatag gcggcagggg gcggcgcatt 811680
ttcggtgaag gcgaaatttg tgccggaagg ggcgttgtcc gccacgcgcc gccagttgcg 811740
tttgagcatc gcgatgccgt ctttttcaca cgcataggca cggcggacgg tttcacggct 811800
gtcttggggc gagccttcaa tcaggtaggc gagggggttg tcggcagtat tggtggagta 811860
cgcggcgtag atgttgagct gttcctcggc acgcgtcagc gcgacataaa gcaggcgcag 811920
gcgttccgcc atttcttcat cggcgtattg tttctgttcg tcttccgaca gttgcgcctt 811980
tgccaacagt tcggttcggt ttgcgccttg gtggaggatt tgccagtcgg acggtccggt 812040
atcttgcgcg tcccacgcaa acgggcagta caccagcgga tactgcaaac ctttcgaggc 812100
gtgcatggta acgattttga ccaaatcttc gtcgctttcc agacggatgg cgcggttgtc 812160
gccgctgttg ttttcggcaa ggctgatttg gtcgcccagc catttgtgca gcgcggcggg 812220
gttgcggttt tgcgcgtctt cggcggcaag cagttcgagc agttggaaat aattggtcag 812280
actgcgcccg ttgttccggc ttaagaggcg cgtttcgatg ccgtgtgttt gggaaaattg 812340
ctgcatagcg gcgaaaatgc cgtatttatt ccagttgtcg agtgcggttc gggcagattc 812400
cgcccaatgc aaaatctcgc tttcgttttg gttgaagtcg tgcaattgct gcgcgtcata 812460
accgaatatg ctgcttgtca ggacaaaacg cagcgttccg gcgcggcgcg gttcgagcca 812520
gaagccgatg agtgcggaca gggcggcggc ttcgggcgag gcgaacacag attcgcgcga 812580
aagcaggacg ctttgcacct gccgtttttt cagggcggcg gaaaccatca ccgcctcgtt 812640
gtgcgtgcgt accagcacgg caatatcgcc cgactgcaac gggcagcctt tgaaattcag 812700
acggcctctg gcggcttcgt tgagcgcgtg ggcgatttcg tcggcgcaat agtcggcggc 812760
acggcggcgc aaaacgtctt tgttggcttt ttcattgtcg ttttcgtgca gccaacgaac 812820
ctgtacggca ggacgttcgg gggacagcct gctttcggca cgcgccgcac cgacttccga 812880
atagccgatg ttttccaaaa cgaacgggcg ttctttgagg cggaacagcg cgcctatgct 812940
gccgataagc gcggcgtggc tgcggtagtt ggtggcgagc gtgtagcggt gccgcgcgtc 813000
ttccgccgcc tgaaggtagg cgtaaatgtc cgctccgcga aagctgtaaa tcgcctgttt 813060
gggatcgccg acgaggaaca gcggtcggtt ttgggcgatg aaaatctttt ggaagatttc 813120
gtattgcagc gggtcggtgt cttggaactc gtcgatcagc gcggtttccc agttttcggc 813180
aacggcgcgg gcgagagtgt cggcgtgcgg attgtcggtc agcgcggtgt ggacatcgag 813240
cagcaggtcg tcgaaaccgc gttcgcggcg cgattttttc atctcggcaa ggctgcggtt 813300
gaggtattcg attaaatcca gttgcagccg gatcattgtt gcttcttccg cttcttcgag 813360
tgcgttcaaa tcgcgcccga agtctgccag tttctgcaat tcggcaaata ctgccgcatc 813420
gggcgttttg cctttttca gtccggcttc gagtttgtcg gatgcaagtt tcaagagtct 813480
gtcgtgtgtg tctttgtcca gaaagggcag ttgtccggcg gcggattttt gtgccagttc 813540

tttaaaaagg ttgccgaagc tgtttttgcg gtaactgttg ccgttgaggt cgggatgaat 813600
gcgccaaaag ccggcttcca gttctggcag caggcggcag atggtttgcc atgaggtttc 813660
ggcgttgcgc tgcgcctgtt tcaaatccgc ctgcggacgg cggaaattca ggtacgggcg 813720
ggaaagatag cgcgaaattt gggcaaggac ggtttgcggc acagctttgc gtttaagcgc 813780
caatgcggca agcaccggat cattgctgac gcgttcccgc caaaaatctt gcgccgggat 813840
aagcaggcgg tcgccgtctt cttcggtcat ttcgacatcg aacggtgctt ggcacaggaa 813900
ggcgtagtcg cgcaggatgc gctggcagaa gccgtggatg gtatagatgg cggcgttgtc 813960
gaattgcccg atggcggcct tgaggcggac aatcagacgc gtccggccct ctttttgcaa 814020
agcctgtttt aagagttcgg gcaggaaggt gtcgccttcg tggtgttcgg cgcagtaggc 814080
ggcaatgccg tctgaaagcg tgtcgtctcc aagtttggca attcctttgc tttctaaaac 814140
ttgtaacaca tcgtccaaac gcccgcgcag gcgtgttttc agctcggcgg tggcggcttt 814200
ggtgaaggtt acgaccaata cgcgttcgac gttttttttgt tctaatacga tcaggcgtgt 814260
aaacagggcg gcaatgccgt aggttttgcc ggtgccggca gaggcctcaa tcaggttggt 814320
gccggaaatg gggacggtta gcgggtcgaa tgcttggatg cttgcagaca tagtgcgcgc 814380
tcggaaaacg gttggacggt aaaacgggaa aatgccgtct gaaaaatggt ttcagacggc 814440
atcgtccggc ttagaggttt tgcaggcgtt cgacagacgg cgcgtagtag tatccgcccg 814500
aaacggcggc ggagagatgt cggagcagca ggtcggtttt gccgtccgta tcgccgaaca 814560
tactcaataa ttgcgcttcg atattgtgca gcgtgcggca gtatgcggta aacatcaaac 814620
cgtgttcgcc gctgattttg ccgaagggca ggctgcggcg gacgattttc aggccgactc 814680
cgttttcttt caggttgacg cggccgaggt gcgaatcggg caggcggaca tcgcggccga 814740
attcgtcgtc ggtttccttg ccgcgtccga ccgaggcctc ctgttcggcg acggggacgg 814800
catcccattt tttcaaatcg tgcaggtatt tttgcagcag gacatagctg ccgcccgcat 814860
cgggttttcc ttcggggatg atggcgactt cgcggacatt ttcatcgccc tgcgggtttt 814920
ccgtgccgtc gacgaaaccg tccagcccgc gatcctgata caggcgcaaa ccgtgttctt 814980
cggacgcgac gcatatgctg tcgccgaacg cgcccaaaac ggattgggca agcgcgtagg 815040
cggcgttttg gcggaaggat tggatgtgga tggacatatc gtgctgcgtg gacggcgcaa 815100
gcccgttgcc catttcggag aagggtttga tttcactgcc ttcgtccgta tgtccgaatg 815160
ttgcccaggc tttgctgccg aaggcgatgg tcagacccaa aatatcgtcc ggaaagcggg 815220
ctttcaaggc agttaacgcg tcgagcgaag cgcggcaggc ggctttaata tcgttgaggc 815280
gattggcggc gaagtcggct tcgataaaga tgccggcttg ggcgtggtcg ggaatgatgg 815340
cggattgggg cgtgttcatg agatgttcct ttttggtgtc atctgtttcg gatagattat 815400
agcaccgaat cggcaggcgg atttttgccg gaacggcgtg cgtgaatccg ccgtttacat 815460
acctgatgcc gcttttcggt ttcgtgccgc ccgccgcctt tcccgccccc tttatttccg 815520
cttccggcgg cttcggcata tcttttccat tccgatttgg aataaccata taaaaaaagt 815580
attctttgtg tttgccgcaa tttcacttag aatgccgcac ttgcacactt tttacaggag 815640
aggatgatgt tgaaaaaatt cgtactcggc ggtattgccg cattggtttt ggcggcctgc 815700
ggcggttcgg aaggcggcag cggagcatct tccgcgcctg cacaatcggc agtttccggt 815760
tctttaatcg agcgcatcaa caataaaggc acggttaccg tcggcacgga aggcacttac 815820
gcaccgttta cctaccacga caaggacggc aaactgaccg gttacgatgt ggaagtaacc 815880
cgcgccgtgg cggaaaaact gggcgtgaaa gtcgagttta aagaaacgca atgggattcg 815940
atgatggcgg gtttgaaggc ggggcgtttc gacgtggtgg caaaccaagt cggtctgacc 816000
agccccgaac gccaagcgac attcgacaaa tccgatcctt acagctggag cggtgccgta 816060
ttggttgtcc gtaacgacag caacatcaaa tctatagccg acatcaaagg cgtgaaaacc 816120
gcacaatccc tgaccagcaa ctacggcgaa aaagccaaag ctgcaggcgc agatttggtg 816180
gctgttgacg gtttggcgca atcgctgacc ctgattgaac aaaaacgtgc cgatgcaacc 816240
ctgaacgacg aattggcggt tttggactat ctgaagaaaa acccgaatgc gggcgtgaaa 816300
atcgtttggt ccgcacctgc cgatgaaaaa gtcggttccg gcctgattgt caacaagggc 816360
aatgacgaag ccgtggcgaa attcagtacg gcaatcaacg agctgaaagc cgacggtacg 816420
ctgaaaaaac tgggcgaaca attcttcgga aaagacatca gtgttcaata atttccttgc 816480
ttcgctgccg tttatgacgg aaacacgcgc cgatatgatt gtcagcgcgt ttttgcctat 816540
ggtcaaagcc ggcttcgcgg tctctctgcc tttggcggca gcttctttcg ttatcggtat 816600
gatgattgcg gtagccgtgg ctttggtgcg gattatgccc gccggcggca tcgtgcggaa 816660
aatcctgctg aaattggtgg aattttatat ttccgtcatt cgcggtacgc cgctgttggt 816720
tcagcttgtg attgtgtttt acgggctgcc ttccgtcggc atctatatcg acccgattcc 816780
tgccgccatc atcggctttt cgctcaatgt cggcgcatac gcttccgaaa ccatacgcgc 816840
ggcaattttg tccgtaccta aaggccaatg ggaagcaggt ttctccatcg gcatgaccta 816900
tatgcagacg ttccgccgca ttgtcgcgcc gcaggcattc cgcgttgccg tgccgccttt 816960
gagcaacgag tttatcggtt tgtttaaaaa cacctcgctc gcggcagtcg tgacggtaac 817020
ggaattattc cgcgtcgcgc aggaaacggc aaaccgcact tatgactttt tgcccgtcta 817080
tatcgaagcc gctttggttt actggtgttt ttgtaaagtg ctgttcctga ttcaggcgcg 817140
tttggaaaaa cgtttcgacc gctacgtcgc caaataagga gttgtcatga ttaaaatccg 817200

caatatccat aagacctttg gcgaaaacac tattttgcgc ggcatcgatt tggatgtgtg 817260
caaagggcag gtggtcgtca tcctcgggcc ttccggctca ggcaaaacga cgtttctgcg 817320
atgcctaaac gcgttggaaa tgcccgaaga cggacaaatc gagttcgaca acgagcgacc 817380
gctgaaaatc gatttttcta aaaaaccaag caaacacgat attttggcac tgcgccgcaa 817440
atcaggcatg gtgtttcaac aatacaacct ctttccgcac aaaaccgcct tggaaaacgt 817500
aatggaagga ccggttgccg tacagggcaa gcctgccgcc caagcgcgcg aagaggctct 817560
gaaactgctg gaaaaagtcg gcttgggcga caaagtggat ttgtatccct accagctttc 817620
cggcggtcag cagcagcgcg tcggcattgc ccgcgcattg gcgattcagc ctgaactgat 817680
gctgtttgac gaaccgactt ccgcgctcga tcctgaattg gtgcaagatg ttttggatac 817740
catgaaggaa ttggcgcaag aaggctggac catggttgtc gttacgcatg aaatcaagtt 817800
cgccttagaa gtggcaacca ccgtcgtcgt gatggacggc ggcgttattg tcgaacaagg 817860
cagcccgcaa gatttgttcg accaccccaa acacgaacgg acgcggagat ttttaagcca 817920
aatccaatct accaagattt gattaagcat ttttcctgtg tttacagagg ccagattaga 817980
ttcggattgc tttcgatgac ggctttgaat tggttttgaa tccgctcgat ggcttcttgc 818040
gtatccgcct caaaacgcaa caccagaatc ggcgtggtat tggaagcacg catcagaccg 818100
aagccgtcgg gaaattcaac gcgcagaccg tcgatggtga tgatttcggt tgcgccttca 818160
aattcggctt tggcggcgag ttcgtcgata acctgatggc cgttgctgcc ttcgggcagg 818220
gcgatgttga gttcgggcgt ggaaatgctt tgcggcaggt tgtttaacac ttcggacgga 818280
ttatcggagg cagacaggat ttccaagagg cgtgcgccgg cgtacagacc gtcgtcgaag 818340
ccgaaccagc gttctttgaa gaagatgtgt ccgctcattt cgccggcaac cggcgcgccg 818400
gtttctttca tggcggattt gataaagctg tggccggttt tttccattat ggctttgccg 818460
ccgtgttctt taatccaagg cgcaagcagg cgggtggact tcacgtcgaa aatgactttc 818520
gcgccgggat tgcggttcaa aacgtcttgg gcgaacagca tcagttggcg gtcgggataa 818580
ataatgttgc cgtctttggt aaccacaccc aagcggtcgg catcgccgtc aaacgccaag 818640
ccgatttcgg catcaccgtt tttcagcgcg gcaatcaaat cttgcaggtt tttcggtttg 818700
gatgggtcgg gatggtggtt ggggaaagtg ccgtccacgt cgcagaaaag ctcggttact 818760
ttgttgccca agcctttgta gagtttgccg gcaaacgcgc cgcccacgcc gttgcccgcg 818820
tcaatggcga tgttcatcgg gcgtttgagc ctgatgtgtc cggtaatgtg tttgaggtat 818880
tcgccggaga tgtctttttc ggtgacgctg ccttgtttgc cggcggcagc aaaaccgtct 818940
ttttcaatga tggacaaaag ttcttggatg gcttcgccgg caagcgtgtc gccgccgagc 819000
atcattttaa agccgttgta atcgggcgga ttgtggctgc cggtaatcat cacgccgctg 819060
ccgccgcatt cgttgacggc ggcgaagtag agcataggag tggcaaccat accgacattg 819120
aggacattga tgccgctgtc ggtaaagccg cgccggatgt gttccatcag ttcgggaccg 819180
ctcaagcgtc cgtcgcgtcc gagcgcgatg cgggtaatgc ctttttcggc ggctttggcg 819240
gcgatggctt tgccgataag gtaggcggct tcgtcggtca gggttttgcc gacaataccc 819300
cggatgtcgt aggctttgaa gatgtcgcgg gcgatgcttg ccataaggtt tcctttgtgt 819360
ccgtttagga aaaacgggca tattttaaca tagcggtatg ccgtctgaag gcttgcgtcc 819420
ggttttcaga cggcatagca cggttacatc aaataacatg ccgtctgaaa taaaagcagc 819480
ctttgtgcag gctgctttcg gattgtcggt ttataccgct tcggctttaa tgatgacgac 819540
aggttcgctc ggtacgtcgt cgtggtaacc atgacgtttg gtagaaacgc cttcgatggc 819600
atcgacaacg tcaaaaccgt caacgacttt accgaatacg gcatagcccc agtcttggac 819660
gacggttttg ccgtacagct ctttagaacg gaagttcagg aaagcgttgt cggcagtgtt 819720
gatgaagaat tgcgcgctgg cggaatgggg gtcggaagtg cgtgccatgg cgatggtgta 819780
tttatcgttg ggcaggccgt tggacgcttc gttttgaatc ggatcgcggg tttctttttc 819840
gttcatgttt tcatccatgc cgccgccttg aatcatgaag cctttgatga cgcggtggaa 819900
gattacgccg tcgtagaagc cgtctttgac gtattgctcg aagtttttgg cggtaacagg 819960
ggctttgtcg aaatcgagtt cgattttgat gtcgcctttg ttggtgtgca ggataatcat 820020
gggtttcctt tcgttagaat ctggttttga atgattcgac aaattgtgtc tgaacgacaa 820080
acttcaaggt cgtctgaaaa atattctttc agacggtctt gttgtttagg tcgatggttt 820140
acatcagtac agcataagcc cacagagcaa ccaatactac gcagaggatg ttcagcagta 820200
tgccgacatt catcatttcg cgttgcttga ttaagcccgt gccgaacaca atcgcgttag 820260
gcggtgtggc aaccggcagc atgaaggcac aagatgcgcc gatgccgatg acgaatacca 820320
agacttgttc gggcagcccc atctgcatag cgatgccgga gaaaatcggt acaagcaatg 820380
cggcggaggc ggtgttgctg gtgaactcgg tcagaaaaat aatgaaggcg gcgacgatga 820440
gtatcaccaa aaatgcgggc gcgccggaaa aggtggcggc aacctgctgt cccaaggctt 820500
cggacgcgcc ggatgttttc aacagcgtgc tcaggctgat gccgccgccg aagagcatca 820560
acacgcccca gtcggtattg cgggcgactt ccttccattg cgccacgccg aagacgacga 820620
cggcgacggc ggcactcagg gcgataacgg tgtcgggatt ggaaatgccg aaggcggttt 820680
tgattttgga gctgaatatc cacgcggcgg ctgtggcaag gaaaatcaac agcgcgatca 820740
cgcggtgcag cgtccaaggg atggattcgg ctttgatttc cacgcgttcg ttcaaattag 820800
gtttgaggat gacgtacagg gagagcagca tcaagggcag aatcaacagc atcatcggca 820860

ggccgagctt catccagccg acgaagtcca gatttagggc tttggcggca atcaggttgg 820920
gcggcgagcc gacgagcgtg cccaagccgc cgatgctggc gcaataggcg atgccgagca 820980
ggaggaagac gtaggttttg tgttcttttt cctggtcgag gtggctcagc atacccattg 821040
ctagaggcag catcatcgcg gcggtggcgg tgttgctgat ccacatggac agaaaggcgg 821100
taacgaggaa caacatcaaa accgccactt tcatattgcc gcgcgacagg cgcaacaggc 821160
tgacggcgat tttacggtcc agccgctgca tatgcagggc ggtggcaagc gcgaagccgc 821220
cgaaaaaaat gtagataatc gggttggaaa aatcagccat cgcctttttg atgtccatgt 821280
cggggaaacc gagtacgacg gcgagaatcg gcaccatcag tgcggttacg gtaatgtgga 821340
cggcctcggt aaaccaaagt gcggcaacga aaatcagcag cgcgatacct ttattggcat 821400
cggggctgta aggcaggatg tggtaaatgc cgaaacagac gacggcggaa ataatggtgg 821460
tcagcaggcc cttaaagtcg gtaatcggct tctgcgcact gagcagctcg acgttttcgg 821520
gatgctgggt tttgtccttt gcatgcaggt tcatgaatac tcctttaagg caacaaaatc 821580
ggtttttctt ttgtgtcgtg caatccgaaa cggtttgtgg aatcgccgct tctgcaactg 821640
gttcgagtat atttgtaatc tgatgtagtg taaatatatt gtaaacgatt tgtcggtttt 821700
gtttatgaga tgggattgat atgtaagggg aaaaatagga tatatcggga agaggtgcat 821760
cctgtttggg gaaaataaac cgatttagtc cgggcggcag gcagctcgcg cggtaaagag 821820
ggcaagggct gcgcccgtca ggtcggcaag gacatcgccc aaactgccgg ttctcgttgc 821880
ggtaaaccat gcctgcgcgc attcgctgaa gagggcgaaa cagagggcaa agaccatcag 821940
gctgcgatag gggatggggc ggttgtcggt tctgaatgct ttggtcagaa gccagatttg 822000
tgcgaaaaac agggcgaggt gcgccacttt gtcaaaatgc ggaaaaggcg gtggcgcggt 822060
ttcggcagct ttgaaaagca gtgagtaaat gctgcctgca aaccacaatg ccgagagcag 822120
gataaagcgg ttgcgtggca gattcatgct tgttcctctt caagccatgt ctggcatagt 822180
ttggataggc gcaggaattt tccgccgcgt gcggccagca tatcgcgcca aacggcaatt 822240
tcttcggcgg agggggcatc gtctatgctg cattcgtaga gcaggaaatc gagggtttct 822300
tcgatgacgg ggatggattc ggtttggata agctgcttga gttcggtcat gactgttcgg 822360
atatggaaat cgggaacatg ccgtctgaaa gggcttcaga cggcatcggg tcatttgctg 822420
tgcaggaagc gggttgcttc ttcccatttg ccggcaagga tgtcgggtat ggcttgcagg 822480
gatttggcga cggcatcgtc aatctgtcgg cggtgttccg tactgggttt gttcaggaca 822540
tagccgacga cgaggttgcg gtcgcccggg tggccgatgc cgaggcgcag gcggtaatag 822600
tctgccgtgc cgagttttgc ctgaatgtct ttcaagccgt tgtgtccgcc gttgccgccg 822660
ccgagtttga atttgatccg tccgcaggga atgtcgagtt cgtcgtggac gacgaggatt 822720
tcttcgggtt tgattttgta gaactgtgca agcgcggcaa ctgcctgtcc ggaacggttc 822780
atgaacgtgg caggtttgag cagccaaacg tcgccgtcgg gcagggcggc acgggcgact 822840
tcgccgaaga attttttttc ttctttaaat gaagccttcc atttccacgc cagttcgtcg 822900
aggaaccaaa aacccgcatt gtggcgtgtc tgttcgtatt ctttgcccgg gttgcccaag 822960
ccgacaacca ttttgattgt gtttgacatg atattttccg tgtttctgtc gaatgctgtc 823020
tgaaggcttc agacggcatg gttattcttc ttgattttga acgcgtttgc ggcgcgcttc 823080
tttggggtcg atcaacagcg ggcggtacac ttcgatgcgg tcgccgtcgc gcagcggcgt 823140
gtcgtctttg acggctttgc cgaaaatgcc caaaggcgcg gaatgcaggt ttaaatcttc 823200
aaatatgccg tccaaaccgc tttgcagtgc ggcggcgcgg acggttgttc cctcggcaag 823260
ctgcatggtt ttcaaaacct gtcggtcggg cagcccgtac acaatctcaa tttcaagcat 823320
aacggcggtc tgcctctttg acgaacgctt cgaccagcgt ggtggaaagg tggttgaaga 823380
cgggggaaat taaggcggac aaaacggcat tggaaaaatc gtattccaaa ttgaattcga 823440
ttttgcacat atcgtcgccc aaatcgataa atttccacgt tccacgtaag gttttgaacg 823500
gaccctcgag cagttccata cggatttccc tgcccggaat gttgcggttg tgcgtggcaa 823560
acgattggcg aacgtgcata taatccataa acagccgcgc cttcagttcg ttgccgctac 823620
gcccgatgac ttcggtcttg ctgtaccacg gcagaaagtg cggatagtct tcaaccttgt 823680
cgaccagctc gaacattttg tccgcgccgt gcagcaccaa gatgtttttt tcaacttttt 823740
tcacgggcat caccaatgcg ggcgtgtatt cgaagggcgg tattatagcg gtttaatttt 823800
aaaccggtac agccttgccc cgttctgatt tcaatttaaa ccgccatatc gcagattcgt 823860
aaaaaccgat acggctttgg cgtttcagac ggcattgagt ggaaaatgcc gtctgaaaac 823920
gggatgggaa aacggcagct tgcgggctgc cgtttgttcg ggaagttaag ccttgttttc 823980
aggctgctgc tcggactctg tcgaagcggt gtctgccggg gcaggtgctt cggtttcttc 824040
ccgagtcgga gcgtgcggcg cgttatcttc cgccgcgtcc gcattctcgc gcaggttgtg 824100
gctgtaatct ttgggcgggc tgggttgttt gcccgccatg atttccagta cctgatcgcg 824160
gtcgatggtt tcccattcca tcagggcttt gcacatcgtt tccatcttgt cgcggttttc 824220
atcgaggatt ttgtaggcaa cctgatattg ctcgtccaaa atccggcgga tttccgcgtc 824280
gatgtcctgc tgggttttct cggaaatgtt ttgcgaacgg gttacgctgc gtcccaagaa 824340
gacttcgcct tcgttttccg cataaaccat cacgcccatt ttgtcgctca tgccgtagcg 824400
cgttaccatt tcgcgcgcca tttgggttgc gcgttcaaag tcgtttgatg cgccggtgga 824460
gatgcgtccg acgaagatgt cttcggcaat ccgtccgccg aacaggatgg agagctggct 824520

caacatctga tctttataca tactgatgcg gtcgcgctcc ggaagctgcc aagtcagacc 824580
cagcgcacgt ccgcgcggca taatggttac tttgtggacg gggtcggtaa agggcaggct 824640
ttcggcaaca atcgcgtgtc cggattcgtg atacgccgtc gcacgttttt cgtcttcgtg 824700
catcaccata ctgcggcgtt ccggacccat atagattttg tctttggcgt cttcaaaatc 824760
gctctgatcg actttgactt tattgcggcg gccggcaaac agggcggctt cgttgaccaa 824820
gttcgccaaa tccgcgccgg aaaaacccgg cgtgccgcgc gcgagggaca ataaatccac 824880
agattcgtcc aaaggcactt ttttagaatg gacgttcaaa atctgttcgc gccctcggat 824940
gtccggcagg gggacaacca cttggcggtc gaaacggccg gggcgttgca gcgcaggatc 825000
gagtacgtcg gggcggttgg ttgccgcaat cacaattaca gtctgattgc tctcaaaacc 825060
gtccatttca accaacaatt ggtttaatgt ttgctcgcgc tcatcattgc cgccgcccaa 825120
acctgcgccg cgttggcggc cgactgcgtc aatctcgtcg ataaagatga tgcagggggc 825180
gtttttcttc gcctgctcga acatatcgcg gacgcggctc gcaccgacac cgacgaacat 825240
ttcgacaaag tcggaacctg aaatgctgaa gaacggcacg ccggcttcgc ctgcaatcgc 825300
tttcgccaaa agcgtcttac ccgtacccgg gctgcccgcc agcaggatgc cgcgcggcac 825360
gcgcccgccc aggctttgat agcggttcgg cgctttgagg taatcgacga tttcctgtac 825420
ttcttctttg gcttcgtcgc agccggcgac atcggcaaag gtcactttgt tggcatcttt 825480
gtccagcagg cgggcgcggc ttttaccgaa tgagaatgcg ccgccttttc cgccgccgcc 825540
cgtctgcata cgcatgaagt agaaccatgc gccaatcagc agcaggacgg gcagcaggct 825600
gtaaaacagg gcagccagcg cgctcggttt ttcttccggc gttactttta cgcggacgtt 825660
tttgtcgagc agtgttttaa ttaggttgtc gtccaaaggc gcgttggtga agaaagtgct 825720
tttgtcggtg cgctcgccct taatcaggta gccgctgacg acggatcctt cgatgttgac 825780
gccggatact tcgccgttgt tgacctgttg gatgaactga gagtattcga tttgcccgtt 825840
gtcttctttt ttaccgtcta aagcgttgaa cgcagccatc aggccgatac ccaaggcgac 825900
ccagacaagg attgatttaa aggtgttccc cacttagcaa ggctccataa ttgaggtgta 825960
aaacggaaat gattgtaaag cacgccgtct gtattgtcag cgtttatttt tgcccaataa 826020
ataaatctca ctggagcgat tgcgcgaggc ttcgggtttg cgcgtctgca ccgtgccgaa 826080
aatttcgcgc atggctgcca tgtattcctg atagcctgca ccctgaaaga ctttgaccaa 826140
aaagctgccg ccggttttca ggtgttgcga ggcgaagtct aaagccagtt cgcacagata 826200
aaagctgcgt gcctgatcgc ttacggcgtt tcccgacata ttgggcgcca tatcgcaaat 826260
tacaaggtcg agcgggcggt tgtccaacaa ggtttcgaat tgtgccagta cgtcgttctc 826320
gcggaagtcg ccctgaatga aggagacgcc ccctatggct tccataggca ggatgtccaa 826380
ggcgaaaact gctccggaag tacccgtcag cttggcggca acctgcgacc agcttcccgg 826440
cgcgctgccc aagtcggcaa gtaccgtgcc gggtttgatt aatttgtctt tttcgttgat 826500
ttccaaaagt ttgtatgcgg cacgggcgcg gtagccgtct ttttgcgcca tatggacgta 826560
gtggtcgttg acgtgttcgt gcagccacgc ttttgaggat ttggaacgta cagccatagt 826620
ggttcgcggg tcggaatgga aaccgcgtat tgtacgttaa ttttgccgat gtcgtgccaa 826680
atcgcgtaca atgccgcatt attcttttta ttcaagcagt aggaaaatga cggataccaa 826740
attgaacacc aaagaaattt tggaactgaa agcgcgcgcg caccatctcc atcctgttgt 826800
gatggtcggt cagcagggtc tgacggacgc ggtcatcaag gaaaccgatg cggcattgac 826860
ggcgcatgag ctgattaaag tgcgcgtatt cggcgacgac cgtgccgaac gtatcgaaat 826920
ctgcactgcc ttatgtgagg cggttgatgc gcaactggtt cagcatatcg gaaaactttt 826980
ggtattgtgg cgtaagaata tcgaagcctg acagcctgaa gcagttgttt tgctattgtt 827040
ctttaacggg cgggacgccg tccttcggcg cggcatttcg gcgggccgaa accctttccg 827100
gtgaaaacgg attttgattg ccgcccgatg ctgtctgcaa gttgcggcgg cttccgtatg 827160
gtttgaattg ttgacaggat gattggaggg cttatgcagt ttccttaccg caatgttccg 827220
gcttcgcgta tgcgccgtat gcgcagggac gatttttcac gccgcctgat gcgcgaacac 827280
acgctgaccg ccgatgattt gatttatccg gtgttcgtat tggaggggtc ggcgcgcgag 827340
gaggatgtgc cttctatgcc gggtgtgaag cgtcaaagtt tggacaggct gctgtttacg 827400
gcggaagagg cggtaaagct cggtattccg atgttggcac tgttccccgt ggttacggca 827460
aacaaaaccg agcgtgcgca ggaggcgtac aatcccgaag gactcgtgcc gtcaactgtc 827520
cgcgccttgc gcgagaggtt tcccgaactg ggcattatga cggatgtcgc gctcgatcct 827580
tatacggttc acggtcagga cgggctgacg gacgaaaacg gttatgtgat gaacgatgaa 827640
accgtagagg ttttggtcaa gcaggctttg tgccacgctg aagcgggcgc gcaggtggtt 827700
gccccttccg atatgatgga cgggcgtatc ggtgcgattc gcgaggcgtt ggaggatgcc 827760
gggcatatcc atacgcggat tatggcgtat tccgccaaat atgcttctgc attttacggc 827820
cctttccgtg atgcggtagg cagttcgggc aatttgggca aggcagataa aaagacctac 827880
cagatggatc cggcaaatac cgatgaggcg ttgcacgaag tggcgttgga cattcaggaa 827940
ggtgcggata tggtaatggt caagcccggt ttgccgtatt tggacgttgt ccgccgcgta 828000
aaggacgagt tcggtgtgcc gacttatgcc tatcaggttt cgggagaata cgcgatgttg 828060
caggcagcga ttgccaacgg ctggctggac ggcggcaaag tggttttgga aagcctgctg 828120
gcattcaaac gtgcgggtgc ggacgggatt ttgacctatt acgctattga ggcggcaaag 828180

atgttgaagc gttgattttc ggccgggtta attgaaatgc cgtctgaaac catggtttca 828240
gacggcattt ttacagttta caaagttgta tcgagtgcgg cggaaatatc gttccaaata 828300
tcgtccgcgt cttcgatgcc gatggagaaa cgcagcagac cgactttgat gcccatttcc 828360
attttcacat catgcggtac gccgctgtgg gactgggaat agcaatggtt gaccaaactt 828420
tccacaccgc cgaggctgga agccattttg accagtttca tgtttttaat cacgctgttt 828480
gccgcttcac gcgtgtcgtt tttgagataa accgtaacca cgccgccgat gcctttgggc 828540
atttgtgttt tcgccagttc gtaatgttcg tgagacggca ggccgggatg gaacactttt 828600
tcaatggcag gatgggcttc caaacggcgc gcgatttcga gtgcgttttg gcaatgggcg 828660
ttcatgcgca gagccagtgt tttgatgccg cgcaacacca gccagcagtc cagcgggccg 828720
gcaaccgcgc cggtatgcac catcatatcg tgcaaaggct gcgccagttc tttggttttg 828780
gcaacgacga tgcccatcaa cacgtcggaa tggccgcaca aatatttggt agcggaatgg 828840
aatacaaaat cgcaacccat atccaacggc tgttgcagat acggcgtggc aaaagtgttg 828900
tcgataccga ccagcgcacc ggctgctttg gcttttgcgg caaggacttt gatgtctacc 828960
aagcgtaaaa gtggattgga cggcgtttcc agccaaacca gtttgacctt gtgcgcttta 829020
agcagttcgt ccaaattatc cggattgcct aaatcggcaa aaacaacgtt caccccccat 829080
ttttgataaa catcgaccaa taaatcataa gcgccgccgt aaatatcggc gacggcgaca 829140
atggtatcgc ccgggcgcag gaaagtgcgc catacggcat caattcccgc cataccgctg 829200
gaaaacgcaa aacctgccgc accgtgttcc aaatcggcaa cggtgtcttc taaaatctga 829260
cgggtcgggt tgctcaggcg cgaataacgg taaggcacat tttcgccaat ctcgtgcaac 829320
gcaaacatac tgttttgata aatcggcggc atcagcgcgc ggttgtgttc gtcgcaatcg 829380
tagctggaat gaatggcttt cgtggcgaat ttcatttggt ctctgcctat gtagatgtga 829440
ataatcgaag attttatcac tatctgaaaa atagaaacaa tcaatgcagc tgctaaaagc 829500
gagtgatata atctcgcatt tgcagattga cggtatattc cccggcggaa acgccatacc 829560
atgcacacat ctcaacaatt acatgaatat taaggaaaaa caactcatga acactattgc 829620
agacgtgcaa tccagccgag atttgcgcaa tctgccgatc aatcaggtcg gtatcaaaga 829680
cctgcgcttt ccgattaccc tgcaaactgc agaaggcatc cagtccacca ttgcccgtct 829740
gaccatgacg gtttacctgc ctgctgagca gaaggggacg catatgtcgc gttttgtcgc 829800
attgatggag caacatgccg aagccttgga ttttgcacaa ttgcgcaagc tgactaccga 829860
aatggtcgcg cttttagatt cccgcgccgg caaaatcagc gtttctttcc catttttccg 829920
caagaaaacc gccccggttt ccggtattcg gtccttactg gattatgatg tctgtctcac 829980
gggcgaaatc aaagacgggg catacggcca cagtatgaag gtcatgattc ccgtaacctc 830040
gctttgcccg tgttccaaag aaatttccca atacggcgcg cacaaccagc gttcgcacgt 830100
taccgtcagc ctgactgccg atgccgaagt cggtatcgag gaagtcatcg attatgtgga 830160
ggcgcaggcg agctgccaac tctacggcct gctcaaacgc cccgatgaaa aatacgttac 830220
cgaaaaagcc tacgaaaacc cgaaattcgt ggaagatatg gtgcgcgatg tcgctacttc 830280
gctgattgcc gacaaacgca tcaagagttt cgtcgtcgag agcgagaatt tcgagtctat 830340
ccacaaccat tcggcttatg cctatatcgc ctacccgtag gcgcgtttgc gatgaaccaa 830400
atgccgtctg aaaggcgttt gggcgttatt ggcgaatctg ccgccgtatc ggaaatcaat 830460
ttgcaataca agtaataaaa ggatgcacga tgacagtatt aagcaaagag caggttctat 830520
ccgcatttaa aaaccgtaaa tcatgccggc attacgatgc ggcacgcaaa atcagtgccg 830580
aggattttca gtttatttta gaactcgggc gtttgtcgcc cagttcggtc ggttcggagc 830640
cttggcagtt tattgtggtt caaaaccctg aaatccgaca ggcaatcaag ccgttttctt 830700
ggggtatggc ggatgctttg gataccgcca gtcatttggt ggtgtttttg gcgaagaaaa 830760
atgcccgctc cgacagcccg tttatgttgg aaagcctcaa acggcgcggc gttaccgaac 830820
cggatgccgt agcaaaatct ttggcaaggt atcaggcgtt tcaagctgac gacatcaaga 830880
ttttggacga ttctcgcgcc ttgtttgact ggtgttgccg tcagacctat atcgcgttag 830940
ccaacatgat gacgggtgcg gcgatggcag gtatcgattc ctgcccggtg gaaggtttca 831000
actatgccga gatggagcgc atattgtccg ggcagtttgg tttgttcgat gcggcagaat 831060
ggggcgtgtc cgtcgccgcg acattcggct accgcgttca ggaaatcgcc acgaaagcgc 831120
gtaggccctt ggaagaaacc gttatttggg cataaggcaa tgccgtctga aaacgcaagg 831180
attttcagac ggcatttttt aatgcttggc ggattcgcat ttgaagtgca actttcccta 831240
acagaaaaag gccagtatgc ggtagcatac ggcctttcct gcaagaaaga ttgccatgag 831300
ccacacgcaa ctgacccaag gcgaacgata ccacatccaa tacctgtccc gccactgcac 831360
cgtcaccgaa atcgccaaac agctgaaccg ccacaaaagc accatcagcc gcgaaatcag 831420
acggcaccgc acccaagggc agcaatacag cgccgaaaaa gcccagcggc aaagccggac 831480
tatcaaacag cgtaagcgac aaccctataa gctcgattcg cagctgattc agcacatcga 831540
ccccccttatc cgccgcaaac tcagtcccga acaagtatgc gcctacctgt gcaaacacca 831600
ccagatcacg ctccaccaca gcaccattta ccgctacctt cgccaagaca aaagcaacgg 831660
cagcacgttg tggcaacatc tcagaatatg cagcaaaccc taccgcaaac gctacggcag 831720
cacatggacc agaggcaaag tacccaaccg tgtcggcata gaaaaccgac ccgctatcgt 831780
cgaccagaaa tcccgtatcg gcgattggga agccgacacc attgtcggca aaggacagaa 831840

aagcgcatta ttgaccttgg tcgaacgcgt tacccgctac accatcatct gcaaattgga 831900
tagcctcaaa gccgaagaca ctgcccgggc agctgttagg gcattaaagg cacataaaga 831960
cagggtgcac accattacca tggataacgg caaagagttc taccaacaca ccaaaataac 832020
caaagcattg aaagcggaga cttatttttg tcgcccttac cattcttggg agaaagggct 832080
gaatgagaac accaacggac tcatccggca atacttcccc aaacaaaccg atttccgtaa 832140
catcagtgat cgggagatac gcagggttca agatgagttg aaccaccgac caagaaaaac 832200
acttggctac gaaacgccaa gtgttttatt cttgaatctg ttccaaccac taatacacta 832260
gtgttgcact tgaaatccga atccaagctt atttaaaacg attcgccggg agcgagataa 832320
cgccatttgc cgggcggcag cctgccgagt ttgaccttgc ccatgcggat gcgtttcagc 832380
ccgacgacgc gcagtccgac cagttcgcac atacggcgga tttgccgctt tttaccctgt 832440
ttcaacacga agcgcagttg gtcttcgttt tgccattcta cttgggcggg acgcagtttc 832500
tcgccgtcca aactcaatcc gtgattcagt aaggcaagtc ctttttcgtc caatttgccg 832560
cgcacgcgca ccaaatattc tttttcactg ccgctgtttt cgccgataag ctgcttggcg 832620
atacggccgt cctgagtcaa taccagcaat ccgaccgagt cgatgtccag cctgccggcg 832680
ggggcgaggc cgattttgtg tttcggatcg aaacggatgc ggccggtatc gccttcccag 832740
tgattttcag ggtaatcag ttcggcggcg gatttatagc ctttttccgc ttgtgcgctg 832800
acatagccga cgggtttgtt caacaggatg gtaatgcgtg ccgcctgctg ttcgtgggct 832860
ttcttgttca gttcgatacg gtctgccggt gaaactttct gaccgagtac ggcggttttg 832920
ccgttgaccg ttacccaacc ctgttcgata tagccgtcgg cttcgcggcg tgaacaaagc 832980
cccagttgcg ccatgcgttt ggagaggcgc acggcatctt ctgtatggtc ggaggaaatt 833040
ttgggattca tggatacttt cgggtaaagg ccggcttaga ctaattgctc gccccagtgc 833100
agtttttggc gcagggtttt gaaatattgg tagtcggtcg ggtgcaaaat gcgtaaggga 833160
tttcggtagc ggcggatggt gatgcggtcg aggttttgca cgtcgatatg ggtttgaccg 833220
tcgaaatgga cgcgcgcgtc gccgccttgg gtaacgagga tttcgatttc ggacgtgtct 833280
ggaatggcga tggggcggtt ggtcatggat tgtgggcaga tggggacgag cgtgaaggcg 833340
tgtaatcctg cctgcatgat ggggccgccg gcggcaagcg aataggcggt cgatccggtg 833400
ggggtggaga caatcagccc gtccgaacgc tgggtataga cgaattcccg attgacgaag 833460
acttcaaact caatcatctg tccggcaccg ccacgggaga ggacggcatc gttgagggcg 833520
atggcgcgtt cggcggtttt gccttcgcgg atgagtgcgg cctcaatcag gatgcgctct 833580
tcggcaaggt atttcccttc taaaacgggc aatagcttgt ccgtcatata ttcgcgggga 833640
atttgggtca ggaagcccaa atgcccttgg ttgatgccga taatcggaac ggcgcgcagg 833700
gcgatttcgc gggcgacgga gagaaaggtg ccgtctccgc ctaaaacggc gaccaggtcg 833760
cagtattgcc ccagttcggt cttgttgacg atatggcagc cgacggtgtc ttgggtatag 833820
atgcagcctt cctttatgcc gacttcgtcg agatagacgg taaagccgtg ctgcttcaaa 833880
aaggtaatca gcgtgtgtgc ggtgtcttgg atgtcgggcg tgttggggcg ggttacgatg 833940
ccgatgttgt gaaaagggct gttcatgtcg gatgccgtct gaaggttagt ctatccaaat 834000
gtcgcgttcg agccggtcga ggcgttcgtt gaggcgttcc acgccgtcgc gcagtctgct 834060
tatttcgtcg aggcagtcgg caagggcttc gttgccgatg tttgcggact cggattcgcg 834120
ggaaaatccg ccgatttgtt cggcgatgtt cctgccgatt tgtttgatgc cgtgtccgat 834180
gtcggcggca cggctgccga tgtctgcctg cgtgccgaaa atccgtgcca attcgtccga 834240
tgcgcgggaa cgcaggctgc cgagcaggga cagtaccgcg atgccgagga tgaggtcgcc 834300
ttcgagcccg atgtcgcccg ccccgggttc gcctccttgg aggattttct gtaccgcgct 834360
gttgcggaag gtaatttcgg tgtctgcaaa gccgtttccc gccgagagca aaccgtcttc 834420
cgtgatgcgt cccgccagtt tcagcccggc aatgttcagg gtcagtgttt tgcctgcaaa 834480
ggcggcaagt tccgagcggc tgtccgggct ttgcagaatc aggcggttga tgatggggag 834540
gagggcggac atattttctg atcggggcgg agaatgcctg tttgttgctg ttgccgctta 834600
ttttacaggc ttaagccgtt atcgcaaacg gtacggatga ttttgcccac gctgtcgtcg 834660
ggtgcaaaat ccggcgcggg caagccgagt ttggcggctt cttccgtata gaattcgcgt 834720
cgtgtcgggt ggcgcggttc gataatgttt ttcagccgcc tgccgccggg gttaaatgcc 834780
gtctgaaaca ggctttcgac ggcgatatta cggtggacga tgttgatggg gcggttgccg 834840
cccgggatgt tttgcttttg aacaaggcgg ccgacgggat ggcgttcggc gcaataaagc 834900
ccgcccagcc gcaggatgtc gatgttcgga acgccgctgt cgagcaggtg ttgttcggcg 834960
gcgaggattt ggcgggcgga ctcggtttgc ggatcgggta gggcgatttc gtcgcattcg 835020
cgcgctgtat cgccgtaaac gctggtactg cttgtgaaaa tcaggtgttg cacgttgcac 835080
gcccgggcaa gttctgccca ttgtttgacg gtatcggcgt aatgtgtcag cgatgatggc 835140
ggcaagaggc agaaccaaac gggtttgttg gcatggtgcc gccaaaagct tgtatctcgg 835200
gcaaggttcg cgctttgaaa cgcgctgtct tgattgaggt cgatggtatc gaggtgtatg 835260
ggcagattga tatcgtccga agtcaggctg cgtttgacgg cggcaacgcg gctgccgtgt 835320
tggtaaaact tttgtgccag cggcaggccg aggtaaccta ggcctgtgat ggagatatgg 835380
ggcggggga ctgcgcgcat tcgctgatac cgtcgggtaa gtgccgtctg aaggctgatt 835440
cggacgctgt gggtttacgg gttgccgttg ccgattttcc ggtcgtattt cttgcggtgt 835500

tcgggcaaaa gataaggacg aaggaggctt aaggtgcggc ggatgccgcg tgctttggag 835560
tcttcggtca gcttggtgcg gccgcgcagg gagctgtcga agtcgaacca gtatttcgtg 835620
accatccaca tattgacggc gagatcgttc atggcggttt ggtcggcttg gatgatgttc 835680
agaccgttga gttgggtgag caggttgacc aagagcgggg agactttggc ttgggtgaag 835740
gtattgtgtt cgcccaacaa ttcggcactg cgtgcaagca gggtgttcac gtcgctgaat 835800
aggaagcggt attcccacat cacatcataa ataccggcca tataattgat ggagtcttcc 835860
acatcagacg gcaacacggc ttcattcagg tatgccagca gggcttcgct gtaacgtttg 835920
aacagttgga cgatgatttc gtctttgttg cggaagtggt aatagaggtt gcccggactg 835980
atgcccaagt gggcggcaat atggttggtg ctgatgttgc gctcgccttc ctcgttgaaa 836040
agcgcaaggc tggcgtcgat gatgcgtgtg taagtattga ttttggcggg gcgggtcacg 836100
ggcatactcc ttggatttac aggctgaacg aagcaggcag ccaattaggt tcggcgtgca 836160
attctacctg aaaccgagca aatactgtaa atttgatgtg ttgcgccaac tgccggacat 836220
cgtttgccga ggcgttgttt ttgttcacta agaccaaagc ctgcctgtca tgtaccgccg 836280
cgccgccgat ttggaagcct ttcagacggc attggtcgat cagccagccg gcggcgagtt 836340
tgaccgaacc gtcgggctgc ggatagcgcg gcatatcagg atgccgctgc aacaaggtgg 836400
cggctttttc tgcgctgacg acggggtttt taaagaaact gccgacattg ccaagcacgt 836460
taggattagg aagtttactg ttgcggattg cacacactgc atcggaaaca tctttcgccg 836520
tcgggaccct gcccgcgctc agttcggcaa cggcggccgc caaatcgccg taacccaaag 836580
tcggcacaaa atgcgttttt aatgcaaata cgaccgaaac aatcacataa cgccctttac 836640
cctcctgctt gaacaggctt tcgcggtagg cgaagcggca gtcggcattg gcaagctcga 836700
caaaggtctc cgtatccaaa tcaaagcagc gcacgctgtg aatcacgtct ttcgcctcca 836760
cgccgtatgc gccgatgttc tgcacgggcg atgcgccgac cgtacccgga atcaggctca 836820
ggttttccaa accgctcaaa cccagcgcaa cggtgtgcag gacaaaatcg tgccaaattt 836880
cgcccgcctg cgcttcaatc agaaccatgc cgtctgaacg cgcaatctcg cgtatgcctt 836940
tgttttccat gtgtacgacc agtccggcgt aatcctgcat caaaaggatg ttgctgccgc 837000
cgcccagcca taaaacagta tcgcggtcga actccagcag tcggacgatg tcgcgcaact 837060
cgtcggcatg ttcgagcgcg ataaaggccc gggcttgggc gcgaagaccg aaggtgttgt 837120
agggggtaag gtcggttcgg tatcggatgg gttgcatggt ttgaacttta actgtatttg 837180
aattgaagtg tactgcgttt tcagacggcc ttatgcgatc tgaccatctc cctactgcac 837240
aattccaagc accacaccaa ggcgatggcg gcgatggtga gcgaggcaat caatgccgtc 837300
cagaagccgt aaatgcccat attgaaacgg taggcgagca gatagcccgg cagcaggccg 837360
cagccccaaa aggcggcggc gtggatgaac atcggcacct ttgtaacttt gtagccgcgc 837420
aaggcgtagg aggcgataca ttgggtgaag tctgccggtt ggaacaagcc ggcgaacagt 837480
aagacggtgg cggcgatgct taaaaccgcc ggatcattgt tgtacatact taccagcggc 837540
gaacggaata ataccaagga aagcacggta atcacggcga gcatccatcc taacaccagt 837600
gacacgcccg aaatataacg cgcccgcgaa aattcgcgcc gcccaagcga aaagccgatg 837660
cgcaccgtcc ccgccgagcc gacgctttgc ggaatcatat agagaatccc cgacaaactg 837720
atgccgacct gctgcgccgc cacataatcc tcgccgaaag gcgcaatcaa aaacacgata 837780
aacgaaaacg cgctggcttc caaaaaataa gacagcccga tgggtgcgcc gattttccaa 837840
atctgtttga acaccgccca atccggtttg ccgaatttcg ccgtcagtcc gaatgggcgg 837900
aagaaatttt ccttggcgat ataaatccac aatgccagcg cgctgaacca aaacaccgcc 837960
atcgtcgcca gtccgcagcc tgcgccgccc aaagcgggca taccgaattt gccgtaaacg 838020
aaaatatagt tcagcggcac gttcaacaca aacgccgcaa agctgaccaa cataatcagg 838080
cgcgggcggt tcaggctgga agtgtaggcg tgcagcgcgc ggtgtaccat tgccgccggc 838140
atcgccaagc tggtgaacaa catatactgc gccatcgtgc cttccacata atcgctcaag 838200
gtcagccagt tgcggaacgg cgtaatcgcc gcccacatca agaccatgcc gaacacgccc 838260
aaaaacagcc cgaaccaaat cccctgccgc cccgtttcgc ccacttcgtc ggttttaccc 838320
gcgccgtaaa gctgggcaat catcgggttc agcgccgcca taatgcccat aaaggtaata 838380
taaaccgtgg caaacgcgct gctgcccaaa gccaccgccg ccaagtcttc cttgcccgca 838440
ccgcccgcca tcacagtatc gacaaaaccg atgcccacct gcgcgacctg cgccaacagc 838500
atgggcaggg caagagtggt cagcaggcgg acttctttca ggaagacggg aaaggaaaag 838560
cggttgaggt cgagcagcat aagtgttcaa tcaacaaaaa tgccgtctga agcagaaaac 838620
ggcagcagga agtgatgaga aataatggtg cacattatat cgtaaaaaaa tgccgtgccg 838680
tcagacggcg gatacagggt ataaaagtat attcagattg tgtgtatttt atggtaaagt 838740
ttggttttaa cgacttgacg gcattgagcc gtcggacagg ggctgttcgg attctgaatc 838800
ggaaagaagc accgccgttt tgacagcggc gtgatgcgtt gcggcaaaga tgccgtctga 838860
ttgcggatcg ggcagtcttt tgtgtttaca ggataaaata gaaggcagat tctcatgcag 838920
acatggcagc ttccggaaca tatcgccgac gtactgccca cgaacgcgcg gcagcttgaa 838980
agcgcgaggg agcagttgtt ggcactgttc cgcgtacacg gttatgaact ggtacagcct 839040
ccgctgatgg agtacgcaca ttccctgctg acgcatatcg atgcggggct ttccctgaaa 839100
accattttgg taacggacag gctcagcggc aggcagttgg gcatacgcgc cgacatcacg 839160

ccgcaggtgg cgcgtatcga tgcccatctt ttatccgcca accaagggat taaccggttg 839220
tgttatgccg gtccggtgtt gcacgcgcag cccgacggtc tgctgaatat gcgcgaaccc 839280
ttgcaggcag gggcagaaat gtacggtttt gctgacatcc gtggcgacat cgagctgata 839340
gacctgatgc tgaaaagcat gaaaattgcc gatatgggca aagtgctgct ttcgctgggg 839400
catatcggca tatttcgcgc cttgtccgat gcggcacatt tggatgcggg gcagtccgca 839460
acgctgcttg ccttgatgca ggataaagat accggggcgg tcgaagcgca ggtcaaggct 839520
tggaagctgg acggcatgtg ggcaaaagca ttctcgctgt tgccgcgcct gtacggcggg 839580
cgtgaagtgt tgtccgacgc gcgcggacgg ttgccggatt tgtcggcggt cggcggcgcg 839640
ttgggcgaat tgcaggcggt gtgcgacgca ttccccgatt gtgaaatcca tatcgacttg 839700
tccgagctgc gtgtcgacaa ttaccacacg ggcttgctgt atgccgccta tgccgccgat 839760
ttccacgacg cggtcgcgcg cggcgggcgt tatgacggat tgggcggata tttcggtagg 839820
gcgcgcccgg caacgggatt cagtttcgac ttgcgcagct ttatcgggcg tttgcccgcc 839880
atcgaacggc agcccgccgt gttggtcgat gcggaagatg ccgaagcggc gcacgaagcg 839940
gtcgaagcct tgcgtgaaca agggcagtgt gtcgtaatcg attacggtat cggacacaat 840000
gtttcggaag agcttgcagg ccgtctgaaa aagacggacg gcgtttggca ggtcgtgaaa 840060
cgctaaatac ccgttcatgg cggatgaaag gcaaatcgtg gcggggcgca aagccgcacc 840120
ggtttgggga tttccgcaat aatttttaat atcgataggt tatatggcta tggctaaaaa 840180
tgttgtagta atcggcgcac agtggggcga cgagggtaaa ggtaaaatcg ttgactggct 840240
ggcggaagaa gccggcggcg tggtgcgctt ccaaggcggc cacaatgcgg gccatacctt 840300
ggttgtcggc ggcaaaaaaa ccattttgcg cctgattccg agcggcatcc tgcatgaaag 840360
tttggactgc ttcatcggtt cgggcgttgt cgtctccccc gaagccctgt tgggcgaaat 840420
cgacgagttg aacgcggcag gcgtgaaaaa cgtcgaaggc cgtctgaaaa tcgcgccgac 840480
ctgcccgctg atcctgcctt accacatcgc gctcgaccaa gcccgcgaag catcgcgcgg 840540
caaaggcaaa atcggcacga ccggccgcgg catcggccct gcctacgaag acaaagtggc 840600
acgccgcgcc attcgcgccg ccgatttgct gcatcctgaa aaactgcgtg aaaaactgga 840660
tgccgtcctt gcctattaca atgtccaact gcaacatctg cacaatgccg agccggttaa 840720
agcggaagac gtgatggcgg ttatcgaaaa agtcgcgccg cgcattgcgc cgatgattac 840780
cgacgtgtcc cgcgtgttga acgagaaaaa caaaaacggc gaaaaactgc tgtttgaagg 840840
cgcgcaaggt gcgttgttgg acatcgacta cggcacttat cccttcgtta cctcgtccaa 840900
ctgtctggcg ggcgcagctt cggcaggcgc gggcgtaggt cctcaaatgc tggattatgt 840960
tttgggcatc gtcaaagcct ataccacgcg cgtcggttcg ggcccgttcc cgaccgaatt 841020
gttcgacgaa gtaggcgtag gtttggcaga acgcggacac gaattcggtt cggtaaccgg 841080
acgcgcacgc cgctgcggct ggtttgatgc cgccgccctg aaacgctcca tccaaatcaa 841140
cggcatttcc ggtatgtgta ttactaaact cgatgtaatg gacggcgttg aaaccatcaa 841200
tatctgcgtc ggctatgaat tgcccgacgg cggcaaaacc gacatcctgc cttgcggttc 841260
cgatgcggtg gaaacctgca agccgattta cgaaaccatg cccggctggc gcgaatccac 841320
tttcggcgtg aaggactacg gcgcattgcc cgaaaacgcc aaagcatatt tgaaacggat 841380
tgaagaagtc tgcggcgcgc cggtcgccat cgtctccacc ggccccgacc gagaagaaac 841440
gattgtgctg catcatccgt tcgcataagg ttttgcagta aaattgccgt ctgaagccct 841500
aatccgccgt tggtcataaa tagtagggta tcaacatttc gggctacaat ggtacgtcag 841560
ccaatgccaa gacgtgccag cctgatttgt tgatgtgtgt actattgcat aggcggttaa 841620
gccatgcagg agatgaaagt gtatatgtcg cgcaaagccc attagccgca agcgaggggc 841680
ggggtgcgcg agagagaggg gggcaggtgc taagtcattg gttatctcct tactatctat 841740
ctaactacgg gtgcattatg cgcctatgcc tgttttttgt caaacactat acagttaaaa 841800
tgtgtaaata tttagtaagg acatataccc ttttctttac atttagctcc atcggatacc 841860
agtgcgttca gagaaaattt gaaaatttct atattttggt tgtataatgc tttcatttta 841920
gaaaggtcta taatgccaaa atcactctcg ctacaagatg ttcagttaag gttttccagt 841980
aaatttcctg ataagacggt tttgaagttc actaaaaact acgaaccggt aacaatccaa 842040
tgccctttgc atgggtcggt tgtttacggg aatctacaag ctgccatgaa atcctcaaca 842100
ggatgtcctg aatgtactcg aaatcccgaa aagccctctc caaacgccgt tgccattagg 842160
ttggaggaca cagagacagg ggaaatctac gagtttgcaa gtacactggc tgccagtaaa 842220
tttatcggtt gctccaacag cactttagct gtacgtttaa gcggacgcac tccgtttgac 842280
cgattgatta ggtatcgtta taggttggta aagtaagttc acaaccacta tggcacttac 842340
atttactcaa gcagtttcta agctaacctc taaatttcca catttgaatc ttgtggagtt 842400
caatggcgtt cgttacccga cggtaatcgt ctgtcctata cacgggaggg ttacttgctc 842460
tacattcaaa agtatgttgg actctaaaag tgggtgtcca aaatgtgcat cttatggtgt 842520
caattcccac aaaattccag aagatacaat agataaatta tctaaaaata cagtattgga 842580
ggatactgta actggcgaaa cacttacatt cccctcaaga gcatctgctg caaggtcatt 842640
gggtataaac ccagcagcta tcactgaccg tataaaaggt cggggttcaca cagagacttt 842700
acttgcaggg aggtataagg ttcacatctg cactaaatga cgtatacact ttttaacagt 842760
gtatacccct cgccactatc aggtgcttct cgaaaaattt gacattttta taaatttgct 842820

attaaatcca tttgacaatc aattttggag tctcaaatgg ccaaatcttt caaccaagca 842880
gcttctgaac ttactgatat attccctaat atctctctaa ccggctttga cggtgtgaat 842940
tacccagtaa ccgttaattg tcctatgcac gggaacgttc gttattcgac atttaatgcc 843000
cttataaagt caaagtacgg gtgtcctgag tgtgctaaga tgtcaaaaac ccaaacacct 843060
ccaaatgtag ggaagcccct cctcatcctc gacacaacga ccaacgaaac actcacgttc 843120
ccaagcgtta ctgccgcagg tgctgcttta ggtgtacatt tccaacaaat caatcacagg 843180
ctcaagggtc gaacgtcgcc cgacaacctt atttcaaaca ggtacaaagt gcttgggtac 843240
gataggtaag gttcacaaca ccctaaatcc tacgaaccaa gctcgacgga cgttcaaatc 843300
ccggttagag ttaatttatg taaagattta gtaaagacgt ataccatttt tctttacatt 843360
gtgcttgcgc aggatttcag gtaggtctca aaaaatttga aatttctat attttggttg 843420
tgtaatccat ttgcacataa cctatggaga caaattatgg gtaagcgaat gactttcgat 843480
accgccaaat cacgctttca agagaaattt ccacatttag aattgttgga gttcagtggc 843540
atttataaac cttccagtgt tagatgtcct acgcacgggg ttgtccaact tttgtattac 843600
gacacagcta taaagtcaaa gtatgggtgt ccggagtgcg ggaaacttaa aatgaaggaa 843660
aatacgcctc cccaaaacca aaaacctgtc tccatcctcg acaccgccac aggcgaaaca 843720
ctcacgttcc ccagcgtaca agctgccgcc aaagccctaa acacccccta cggctctata 843780
cgaaccaagc tcgacggacg ttcaaatccc gacaaccttg tctgtaacag gtataaggtt 843840
ctgctataat caacctatgg aaaatattga agaatacgca ctgctgtctc ccgaagccct 843900
gctggaacgc ctggataccg ttttgagtat cagaatcggc ggcaagggtt gggaatccag 843960
ttatgaccgc caactttgca cagacgctgg tcgaaataca ggacagtctg tacagggttg 844020
tgtcaaccgt ccaatacggg gatgacaacc tcaagcggtt gacagcggac aaacggaagc 844080
agtatgagtt gaacttcaag atttccgagg gttctacgcg tgtagagtcc gactttaaag 844140
agactttggt tcggttcggt agagatatgc ttcaagatat gccccctaaa atccgttcgg 844200
caacgctggt agcgttgacg accctgcttg tcggaggggc gttgggttac ggttatttgg 844260
aatacctgaa gcaggttgct tcggaagggt atcagaccga gcgtctgtat aatgccgtcg 844320
accgtcttgc agaatcccaa gaacggataa cgtccgccat cctgaagggt gctagaggtg 844380
ccgatttcgt gcaaatcggc agacgttcct acagtaggga ggatatatcg gaggcaaata 844440
gacgtgcaga gcgtgtcccg tatggcgcag agttggtttc agacggcaat tttaccgctg 844500
ttttatctga tataggggat taacaaaaat caggacaagg cgacgaagcc gcagacagta 844560
taaatagtac ggaaccgatt cacttggtgc ttcagcacct tagagaatcg ttttctttga 844620
gctaaggcga ggcaacgccg tactggtttt tgttaatcca ctatattttc tgtccggata 844680
cggtttatca gggtatatca atgcggcgta tccggtgcgg aaatggatac ggttggtgtc 844740
ggtatggaaa cctgatgttt tcagacagca tatacaaaaa accgtactgc ttgcgcgtac 844800
ggtttttgcc gtttcaaatc ggtttaaagc gatttgaggc gggcgatacg gttgtccagc 844860
gaagggtggg tgctgagcag ggagtcgcgc gtatctccgg cgatgcccat tgcgttcatt 844920
tcttcgggca aatcgaccgg gttgcctttg agcctttgca gggcggaaat cattttcggc 844980
gcgccgacca gttttgccgc gcccgcatcg gcgcggtatt cgcgttgtcg gctgaaccac 845040
atgacaatta agctggcaag gaagccgaac aggatttgga ataccatgct gaccaggaaa 845100
taagttccct gggactggct gccgtcgttg tttcgggcaa tcaggttggc aataatgcgc 845160
gacaggaaca cgacaaaggt attgaccacg ccttgaatca gcgtcagcgt aaccatatcg 845220
ccgttgccga cgtgtgccat ttcgtgcgcc aatacggctt ccacttcgtc acgcgtcata 845280
tggtcgagca aaccggtgct gacggcgatc agggagctgt ttctcgatgc gcccgtggca 845340
aaggcattgg gttcggggga gtggtagatg gcgacttcgg gcgttttcag gttccattgc 845400
cgcgcttggg cttcgacagt gttcaaaagc caggcttctt cttcggtgcg cggcgtgtcg 845460
ataacttccg cgccgaccga ttgtttggcg ataaatttgg acatcagcag cgaaataatc 845520
gaaccagtga agccgacgac ggcggaatac gccaacaggc tgcccgtgcc gccccggctg 845580
ttgatgccca aaaccgccaa aacaatgttg attacgacca aaacagcgat attggtagcc 845640
aaaaacagaa aaattcgttt cacggatgtt cctttttggt agggtgtgat gttttgaaat 845700
tttgggggat tgtcccaaaa agttgccggc ttgtgaatat cagactcggc aaaggtatgc 845760
aaaacatttg cttgcaaatg gcagtttgtg cagttggttt ttgaactatt gtgccaagcc 845820
gtgtagaatc gtaaaccatc tgtttgattc caataaacac atttcaaagg atcacttcat 845880
gaaagcatta cttttaggcg cgccgggcgc gggcaaaggc actcaggcgc aattcatcac 845940
cgcagcgttc ggcattccgc aaatctctac cggcgacatg ctccgtgccg cgattaaggc 846000
aggcacgccc ttgggtttgg aagcgaaaaa aatcattgac gaaggcggct tggtgcgcga 846060
cgacatcatt atcggcatgg tcaaagaacg catcgcgcaa gacgactgca aaaacggttt 846120
cttgtttgac ggtttcccgc gcacattggc acaagccgaa gcgatggttg aagcaggcgt 846180
ggatttggat gcagtcgttg aaatcgatgt gcctgacagc gtgattgtcg accgcatgag 846240
cggccgccgc gtgcatttgg cttccggccg tacttaccac gttacctaca acccgcccaa 846300
agttgaaggc aaagacgacg taaccggcga agatttgatt cagcgcgacg acgacaaaga 846360
agaaaccgtg aaaaaacgcc ttgccgttta ccacgagcaa accgaagttt tggtcgattt 846420
ttacagcaaa ctggaaggcg aacacgcgcc taaatacatc aaagttgacg gcacccaagc 846480

agtagaagcc gtgaaagccg aagtattggg cgcattgggc aaataaatcg aaaaggtcgt 846540
acccacgggc aggcttcgca ctctgaaaac agaaaatcag gttttcagac gacctgtttt 846600
tgataaacag cgtgttgcaa ccgaaaaata atcatttggc gtcattcccg cgcaggcggg 846660
aatccatttc tgaatttggg caatcgctgt ttaaatctga tgaactgagt tttatcaatg 846720
gattcccgcc tgcgcgggaa tgacggctga tgtaccggtt caaatttatc cgaaacagtt 846780
tgtcggaggc ttgagtccgc gtaggtcgga catcaatgcc cgacctacgg tttgaattta 846840
cgttgttata gtggattaac aaaaatcagg acaaggcgac gaagccgcag gcagtacaga 846900
tagtacggaa ccgattcact ttgtgcttca gcaccttaga gaagcgttct ctttgaacta 846960
aggcgagaca acgccgtacc ggtttaaagt taatccacta tactgcgaaa aagacgataa 847020
aggtcgtctg aaaacccgaa acgaaaacac catgaatcct ttaatctccg acttccaaac 847080
tccgcaacaa cgcacccccg ttatcgtcgc ccttgatttt tccaacgaaa aagacacgct 847140
cggattcgtc cgcaaccttg acccgacatt gtgtcaaatc aaaatcggca aagagctgtt 847200
taccgcgacg ggacgcaatt tggcagaaag cttaatcaat caaggtttca aacttttcct 847260
cgatttgaaa taccacgata ttccccacac cgtcgcgcaa gcctgcaaag tcgctgccga 847320
tatgggcgtt tggatggtcg atatgcacgc atcgggcggc cgccgtatga tggaagccgc 847380
agcagaagcc gttgccggat acggcacgaa gccgctctta atcggcgtaa ccgtgttgac 847440
cagcatggaa caaagtgatt tggcggaaat cggtttgaac accgcccctg aagaacaagt 847500
catccgcttg gcaaaactgg cgcaaagttc gggcttggac ggcgtggtct gttccgccca 847560
agaagccgcg ccgctgcgcc gcgaattggg acaggatttt gtcttggtca cgcccggcat 847620
ccgcttggac gttgccggca ataatgatga ccagcgccgc atcatgacac cggccgaagc 847680
cttggctgcc ggttcgactt atttggtaat gggtcgtcct gtaacccaag ctgccgatcc 847740
ggtagccgta ttgcgcgaag tgaaccgcgt ggcaaacctt gaagcaaact gattttcaga 847800
cggccttaca ggctgaggcc gtctgaaaaa atacaacgga ggcaatatgt ccgccaagtt 847860
ccaacaagaa accctcaaat cccgtttcgc gcaagccaaa gtcctggttg tcggcgacgt 847920
gatgctcgac cgctattggt tcggcgatgt gtcccgtatt tcgcccgaag cccccgtgcc 847980
ggtggcgaaa atcggacgaa tcgaccaacg cgcgggcgga gcggcaaatg tcgcgcgcaa 848040
catcgcttcg ttgggcggca gggcagggct gttgtccgta accggcaacg acgaagccgc 848100
cgacgcgctc gatgcgctga tggtgcagga cggcgtcgcc tcctatctga tgcgcgacaa 848160
acaaatcgcc accaccgtca aactgcgcgt cgtcgcccgc aaccagcagc ttatccgtct 848220
tgattttgaa gaacatccca actgcgaagt gttggaacaa atcaagcaga aataccgcga 848280
aatcttgccc gaatacgacg caatcatttt ttcagactac ggcaaaggcg gcctgtcgca 848340
tatctccgat atgatcgatt gggcgaaaca cgccggcaaa accgtcttaa tcgaccccaa 848400
aggcgacgat tacgaaaaat atgtcggtgc aactctgatt acgcctaacc gcgccgaatt 848460
gaaagaagtg gtcggcagtt ggaaaaacga aagcgagctg accgaaaaag cgcaaaacct 848520
gcgccgccac ctcgacctga ccgccgtttt actgacccga agcgaagaag gcatgacctt 848580
gttcagcgaa ggcgaaccga tttaccagcc cacccgcgcc caagaagttt acgacgtatc 848640
cggtgcgggc gacaccgtca ttgccggaat gggcttgggt ttggcggcag gctgcaccat 848700
gcccgaagcc atgtaccttg ccaatactgc ggccggggtt gtcgtggcga aactcggtac 848760
ggcggtttgc tcgtttgccg aattgatcaa ggcattgtca gggcaatcaa caatgtagtt 848820
ttcatattga taagataaac agaacgatat aagtatgact atttcgacaa tggcacagac 848880
acacgatact cgattacaaa aaactttgct ctttcccagt caacaaggtt ggaaatctaa 848940
aacttttctt aaacctgatt cacatattag attagcaacc gtattcagcg ggattggtgc 849000
ggttgaacag gcattccacc gattaaattt aaaccatacc attgtttttt caggagatat 849060
tgatccatac gttaaaaaaa gttatcttgg aaactataaa ttaaatgaag atttttggca 849120
taacgacatt actcaatttg atgcgagaaa gtttagaaat caagttgata ttttagttgg 849180
aggcagtcct tgccaagcat tttccatggt tggcaaacgt gcaggattag aagatacacg 849240
aggaacatta ttctatgaat ttgctcgcgt tgtggatgaa gtccaaccaa agatatttat 849300
ttatgaaaat gtaaagggct tgcttaatca tgataatgga aaaacttgga aagttgtaaa 849360
aagtgttttt tattcacttg gttatgactt atatttccaa ataatgaata gtaaggatta 849420
tgggattcct caacatcgtg agcgtatttt tgttgttggc tttcataccc ctcctataaa 849480
tggttttcag tttcctgaaa agattgaatt agaacatact atgcaagatt ttttggagga 849540
ctatactgat agcaaatatt ttttacgtga aaagggtgcg aaatttgtta ccagttctaa 849600
aaatagacaa aaacgttata cacagattaa tggagaaatt gccttatgcc aaaaagcaaa 849660
tcaacaattt aattggcatg gtgattttat ttttcaggca gcccgcgaat ctgaatttga 849720
tgactttatt tttgatgtaa ataacgttga ggaaaaatat tatctttctg aaaaaatcaa 849780
aaattatgtt ttggcaggag gaacaaaaaa ttttaaaacc agtacggaaa ctgatttgcc 849840
tgtagctcgc ccattattgc aaactatgca taaaatgcat cgtgccgggg ttgataatta 849900
tgttactcat aatcgtggac gtattcgtaa attaacacct agggaatgtt tgcggctaat 849960
gggttttaga gatgatttta aaattttagt tagtgatact caaatgtatc gccaagcggg 850020
aaacagtatt gttgttgatg tattaatcgc tatattaaaa caaatggata ttacgcttta 850080
tggagagtaa aatgccggat tttcaaagaa ttactattga aaacatagag tattttattg 850140

tcgatagtat tcaaaattta cgtgcggaag atagttttat tcacaggaat aacaagttat 850200
ctgtttttgg tggtaatggg gaagcacgca aatatgtagg tagttatatt gatgatagtg 850260
gtcgaaaact aagtacattt tttgagtatg aaaattgggg gattactgag acaaaaaatg 850320
gaaggagggt agaatatcct ttcattcaga aaaattgttt tttttataag aaaaatttac 850380
agcgttattt ggttgatgca aaagttgaat atcacaaaca agaacagaaa tatcatcatg 850440
atatttcaag gttttatgat gattacgttt taagtattaa taatctgcct acagaaaata 850500
ttttcttttc aatccatgat gtatcagacc atttggaaaa ttctaaaggt taccgtaggg 850560
gttatatccg ttcggaggat gatatatggt caatttggcg taagattgta ttgcccaaaa 850620
ttagttattt atcaattctt aaactattgc ctgtcaaaga tatagaagat tcagaaccat 850680
tattttattt ccgaatattt ttggattatc aatttcgctc tatcgtgcac ccgcaactct 850740
tatcaagaga aaaattagaa ataccggctt cagaatttaa tcaattcgta gagcaagaaa 850800
tcattaaaca gaaaagtaga aaagggcagc agaaatatcg caaggatgtt ataaatcata 850860
tgtcgcaatg tccatttaca ttaattacag atgagatttt gttaagggct agtcatatta 850920
aaccttatat ggtttgtatt actgagaaaa atgagaaaga ggcattagat tatttaaatg 850980
ggttagcttt aacgccgact tatgattggt tgttcgatca aggttatatt actttcttgg 851040
atgatggtcg tttgatttgc ggtactcgac taagccgtta cacatgggaa aaacttaata 851100
tcaatccaaa tgcaaaaaat taagatgaga atatatcccg aaggaagaga aaaatatttg 851160
gaatatcatc gcaaatttgt gtttcaggac aatatcgatg atttcttgta actaagttta 851220
ttaattttac tcaaaacaaa ggaaaccgaa tatgaccatc atcgtaacag gcgcggccgg 851280
ctttatcggc agcaacatcg tcaaagcact taatcaacgc ggtattactg acattgttgc 851340
cgtcgataat ttgagcaaag gcgaaaaatt caaaaacctt gccgagtgcg aaatcgccca 851400
ttatctcgac aaacacgaat tcatccgcca agtgagggaa cacattttac cttatcaaaa 851460
catcgaagcc gttttccatc aaggcgcgtg ttccgatacg atgaaccacg acggtttgta 851520
tatgatggac aacaactacc agtacacgct ggatttgctg gactggtgtc aggacgaacg 851580
catccccttc ctttatgcct ccagtgcggc ggtttacggc aaaggagaaa tcttccgcga 851640
agagcgcgaa ctcgaaaaac cgctcaacgt gtacggctac tccaaattcc tgttcgacca 851700
agtattgcgt cgccgcatga aagaaggtct caccgcccaa gtcgtcggct tccgctactt 851760
caatgtttac ggacaacacg aacaacacaa aggccgcatg gcatccgtcg ccttccacca 851820
cttccaccaa taccgcgaac acggttacgt caacctgttc ggcagtaacg acggctacgg 851880
caacggcgaa caaacccgcg acttcgtcag cgtcgaagac gttgccaaag tcaacctcta 851940
cttcttcgac catcccgaac tttccggcat ctacaacctc ggtaccggcc gcagccaaca 852000
gttcaacgaa ctcgccgccg ccaccgtcaa cgcatgccgc gccgccgaag gcaaacctga 852060
aatgagcttg aaagagttgg tagaagaaga acttatccgc tacattccct tccccgacgc 852120
gctcaaaggc aaataccaaa gcttcaccca agccgacatc accaaattgc gcgaagccgg 852180
atataaggaa gaatttttcg atgtcaaatc aggcgtcgac cgctacgtca aatggatgct 852240
ggaaaatttg gcttaatttg aatgcccgta aaaaaatcgt ctgaaaatat caggcgattt 852300
tgatttgttt aacttttata tggatttcga tgatgaccga aatgcaacaa cgcgcccaac 852360
tgcaccgcca aatttggaaa attgccgacg aagtacgcgg cgcggtggat ggctgggact 852420
ttaaacaata cgttctcggc acacttttct accgctttat cagcgaaaac ttcaccgact 852480
atatgcaggc aggcgacagc agtattgatt acgccgctat gccggacagc atcatcacgc 852540
ccgaaatcaa agacgatgcc gtcaaagtta aaggctattt catctacccc ggccagcttt 852600
tttgcaatat tgccgccgaa gcccatcaaa acgaagagct caacaccaag ctgaaagaaa 852660
tttttaccgc gattgaaagc tccgcctccg gctatccgtc cgaacaggac atcaaaggcc 852720
tgtttgacga cttcgacacc accagcagcc ggctcggcag cactgttgcc gacaagaaca 852780
aacgccttgc cgccgtcctc aaaggcgtgg cggaactcga tttcggcaat tttgaaaacc 852840
accacatcga ccttttcggc gatgcctacg aatacctgat ttccaactac gctgccaacg 852900
caggcaaatc cggcggcgaa tttttcaccc cgcaaagcgt atccaagctg attgcgcggc 852960
tggcggtgca cggacaggag aaagtcaaca aaatctacga cccagcttgc ggctcgggca 853020
gtctgctctt gcaggcgaaa aaacagtttg acgagcacat catcgaagaa ggcttcttcg 853080
ggcaggaaat caaccacacc acctacaacc tcgcccgcat gaacatgttc ctgcacaacg 853140
tcaattacaa ccaattccac atcgaattgg gcgacacact gaccaaccca aagctcaaag 853200
acagcaaacc ctttgatgcc atcgtttcca atccgcctta ttccatcaac tggataggca 853260
gcgacgaccc caccttaatc aacgacgacc gctttgcccc cgcaggcgta cttgccccga 853320
aatccaaagc cgattttgcc ttcatcctgc acgcactgaa ctacctttcc ggcagaggcc 853380
gcgccgccat cgtctcattc cccggcattt tctatcgcgg cggcgcagaa cagaaaatcc 853440
gccaatatct ggtggagggc aactacgtgg aaaccgtgat tgcccttgcg cccaatctct 853500
tttacggcac cggcatcgcc gtcaatatcc tggttttgtc caaacacaaa gacaataccg 853560
acatccaatt catcgacgca agcggcttct ttaaaaaaga aaccaacaac aacgtcttaa 853620
tcgaagaaca cattgctgaa atcgtcaaac tcttcgccga taaagccgat gtgccgcata 853680
tcgcccaaaa cgctgcccag caaaccgtca aagacaacgg ctacaacctc gccgtcagca 853740

gctatgtcga agccgaagac acacgcgaaa ttatcgacat caaacagctc aacgccgaaa 853800
tcggcgaaac cgtcgccaaa atcgaacggc tgcggcgtga aattgacgaa gtgattgcag 853860
agattgaagc atgagcatca tcctatacac cgccaacgac ggcactgccc aatttgcctt 853920
gcaggaattt ggcggacagc tttggctgac gcaggcggac atggcagaac tgtaccaaac 853980
caccaaacaa aatatcagca aacacattaa aaccattctt gcagagcaag aattggaaga 854040
gaaggcaact gtcaacttcc agttgacagt tcaaaatgaa aacgggcgca aggtaaaccg 854100
caaaatcgcc cattattccc tgcccatgat tattgccgtc ggctaccgcg tccgttccgc 854160
gcggggcatc caattccgcc aatgggcaac cgaacggctg gacgaatatc tgaccaaagg 854220
ctttgccata gacgacgaac gcctgaaagg cacaggcggc ggcgactatt ggaaagaact 854280
gctcaaccgc attcgcgaca tccgcagcag cgaaaaagcc ctataccggc aagtgcttga 854340
tttatatgcc accagccaag actacaaccc caaaagcagc gaaagccaaa cctttttttgc 854400
cgccgttcaa aacaaactgc actatgccgc cagccggcaa accgcagctg agctgatata 854460
cagccgtgcc gacagcagca aagactttat ggggctgacc acctttcaag gcgcaatccc 854520
cacgctgaat gaagccaaaa tcgccaaaaa ctatctgacc gaagacgaac tgttccgcct 854580
gaaccgtctg gtttccgcct tcttcgacct agcggaaatc aaagcgcagg agcaaagccc 854640
catgtatatg cgcgactgga tagccgaatt ggacaaattt tccgggctgt acggacaagg 854700
cacattacag ggtgcaggca gcatcagccg caaacaggca gagcagaaag ccgaacgcga 854760
ataccgcgcc tatgaagcgc gcatcctgtc gccggtggag caagcctatc tggaaagcgt 854820
taaagcgttg gaaaaaacag ccgtgcaaca gatcaaacag aaaaaagacc gcacaaaata 854880
actaccattc aggctacctg aaaaagcagc ctgcacacca ttgcaggcag cctgaaagca 854940
ggacggactt cagcccgcag aaataacggc aaacggacag agtgagccga agcaccccgc 855000
aactgcccca catcccgccg caacgggaaa gaaacggaaa acaaccatgg atatgcaaaa 855060
caaagcgaaa aaattgattg agatgattca gacggcaccg gtggagtgga agccgttggg 855120
ggaagtggcg aaagtattaa gaggaaaacg tttgacaaag aaagagctaa ttgaaggtgg 855180
gaaatatccg gtttttcatg gcggattgat tcctcttggt tggtttgacc agtttaatcg 855240
tagagctaat caaacgatga ttattaatac gggaagtatt ggtgaagtta tatggagtgg 855300
cgtagatttc tggtcatctg atggtacttt tgtgattcaa acaccaaact atcttgatga 855360
taagtttata ttctactttt taaaaacaag agaaggatat ataaaatccc aaaagagagt 855420
tggtggagtt cctactattg atagattagt agttgaaaat atttcgatcc ccatcccacc 855480
cctggaaacc caacaaaaaa ttgtaaaaat acttgacaaa ttcacagagc tgtaagctac 855540
gctggaagct acgctggaag cggaattaac cctgcgcaaa cgccaatacc ggtattaccg 855600
cgactttctt ttagatttta acaatcaaat cggggggggat agctgatggc tataaaggcc 855660
gtctgaaaga tgtggtttgg aagacgttgg gggaggtatt taatattttt gctggaggcg 855720
acgtaccaaa agacgctttc tctgaagtgg aaacggaaga attttgtatc cccattttat 855780
ctaatggtat aggaagtaag gcactatatg gctggactaa tgttgctaaa atcaatcaac 855840
ctagcttaac tatatcagct agaggaacta taggttgggc tagctttcag aataaacctt 855900
ttttcccaat agtacgcctg ttagtgttaa caccaaaaat tgaattaaac ctaaaatatg 855960
cctactactt tatgaaaagt attgaatcaa attataaagt tcctgaaagc ggtattccac 856020
agctaacgaa accaatgata aaagatattt caatccccat ccctccactc cccgaacagg 856080
aaaaaatcgt cgccatcctc gacaaattcg acaccctgac ccactccatc agcgagggcc 856140
taccgtacga aattgccctg cgccggaaac aatacgaata ttaccgcggg cagttgttga 856200
gcttcccaaa ggctgcctga aaagtcatag ctggtcttta aatcatgccg tctgaaaaat 856260
attgataagg aaatatcatg ggaaaaagtt taaccgaaat tgctgaggaa ctaaaaggaa 856320
acgataaaaa agtccagcta atctatgctt ttaacggaac agggaaaaca cgtttgtcca 856380
gagagtttaa gaatttaatt gctccaacca gttcagaaga gccagacgga gagccaacaa 856440
gaagaaaatt tctctactat aatgcattta ctgaggattt attcttttgg gacaatgatt 856500
tgttagcgaa cgaagctcca agattaaaaa ttcaaaagaa tagttttacc gactggttgc 856560
ttagggataa tggactggat ggagctgtta ttaaaaactt tcaatattat acagatgata 856620
agttgactcc tgattttaat gatgattttt cagaaattgc attttatttt gctcgtggta 856680
atgatgagca gattgaaaat atcaaaattt ccaaaggtga agaaagtaat tttatttgga 856740
gcattttcta tgtattaatc agacaagtca tcgctgaatt gaatattcca gaagatagcg 856800
aagaaggacg ttccacagat cagtttgacg atttggaata tatttttatt gatgaccctg 856860
tcagctcttt ggatgaaaat catctgattc agcaagcggt tgatttggct gatttgataa 856920
agcttagcaa accgaggtta aagtttatca ttactacaca taatgtttta ttttacaacg 856980
ttctatacaa tgaactaaaa aaattagaaa aggaaaagaa aagttatctt ctgttaaaaa 857040
atgaagatgg tagttttgat attcttgaaa aacaaggtga ttccaataaa agtttttcat 857100
atcaccttca cttaaaagga gttattgaaa aagctatcga gaatcagcag gtagaacggt 857160
ttcattttat gttgctgaga aacctgtatg aaaaaacagc taattttta ggctataagc 857220
aaaggtctga tattttgccc gaagacagca gacgaaacta ttttcaacgt attattaact 857280
ttacaagtca ttctacatta tctaatgagg catttgccga gccaacacca caagaacaag 857340
aaactgtcaa attgcttttg caacacttgc tggataacta taattttttt caagatgatg 857400

aacaaagaga taagccatga acctcgaaac caaacccatc gctgaaacgc cgaatttcat 857460
cgtgctcgac caatatgaaa aaatcgaaca gtcgggcagc taccaatcgg aaaaccggtt 857520
ggaagcggag ttaatcgccg atttgcagaa tcagggttac gaataccgca aggatttgaa 857580
cagccaaagc aggctgctgg aaaacctgcg cgcgcagttg cagcggctga acgatgtggc 857640
gttttcagac ggcgaatggg cgcggttttt gacggaatat ctggacaggc cgtctgaaaa 857700
cattaccgat aaaacccgca aaatccacga cgaccatatt tacgatttcg cttttgatga 857760
cggtccttgg attcggattt caagtgcaac actagtgtat tagtggttgg aacagattca 857820
agaataaaac acttggcgtt tcgtagccaa gtgtttttct tggtcggtgg ttcaactcat 857880
cttgaaccct gcgtatctcc cgatcactga tgttacggaa atcggtttgt ttggggaagt 857940
attgccggat gagtccgttg gtgttctcat tcagcccttt ctcccaagaa tggtaagggc 858000
gacaaaaata agtctccgct ttcaatgctt tggttatttt ggtgtgttgg tagaactctt 858060
tgccgttatc catggtaatg gtgtgcaccc tgtctttatg tgcctttaat gccctaacag 858120
ctgcccgggc agtgtcttcg gctttgaggc tatccaattt gcagatgatg gtgtagcggg 858180
taacgcgttc gaccaaggtc aataatgcgc ttttctgtcc tttgccgaca atggtgtcgg 858240
cttcccaatc gccgatacgg gatttctggt cgacgatagc gggtcggttt tctatgccga 858300
cacggttggg tactttgcct ctggtccatg tgctgccgta gcgtttgcgg tagggtttgc 858360
tgcatattct gagatgttgc cacaacgtgc tgccgttgct tttgtcttgg cgaaggtagc 858420
ggtaaatggt gctgtggtgg agcgtgatct ggtggtgttt gcacaggtag gcgcatactt 858480
gttcgggact gagtttgcgg cggataaggg tgtcgatgtg ctgaatcagc tgcgaatcga 858540
gcttataggg ttgtcgctta cgctgtttga tagtccggct ttgccgctgg gctttttcgg 858600
cgctgtattg ctgcccttgg gtgcggtgcc gtctgatttc gcggctgatg gtgcttttgt 858660
ggcggttcag ctgtttggcg atttcggtga cggtgcagtg gcgggacagg tattggatgt 858720
ggtatcgttc gccttgggtc agttgcgtgt agctcatggc aatctttctt gcaggaaagg 858780
tcgtatgcta ccgcatactg gcctttttct gttagggaaa gttgcacttc aaatgcgaat 858840
ccgccgtcgt ctgaaaaaca tttatctgct ggacaagaaa aaccttgccc gcaaccatgt 858900
gcaggttatc aaccagtttg agcagacggg cacgcatgca aaccgctatg acgttaccgt 858960
gttggtaaac ggcctgccgc tggtgcagat tgaattgaaa aagcgcggtg tggcggtgcg 859020
cgaggcattc aatcaggtgc accgttacag caaagagagc ttcaacagcg aaaattcgct 859080
gttcaaattc ctgcaaatct tcgtgatttc caacggcacg gacacgcgct atttcgccaa 859140
caccaccaag cgcgacaaaa acagcttcga tttcacgatg aattgggcgc ggtcggacaa 859200
tcatccgatt aaggatttga aagactttac cgccacgttc ctgcagaaaa gcgtattgct 859260
gggcgttttg ctgcattaca gcgtgttcga tgcgaatgat acgctgctga ttatgcggcc 859320
gtatcagatt gccgccgccg aacgcatttt gtggaaaatc aacagctcgg cgcaggcgaa 859380
gaattggagc aaaccggaaa gcggcggcta tgtctggcac accacgggca gcggcaaaac 859440
gctgaccagc tttaaggcgg cgcgtctggc gacggaatcg gcatttatcg acaaggtttt 859500
cttcgtggtg gacaggaagg atttggacta tcagacgatg aaggaatacc aacgtttttc 859560
gcccgacagc gtgaacggtt cggaaagcac ggcaggcttg aaacgcaatt tggaaaaaga 859620
cgacaacaaa atcatcgtta ccaccatcca aaagctgaac aacctgatga agggcgaaga 859680
taatctgccg gtttaccatc agcgagttgt ctttattttc gacgaatgcc accgctcgca 859740
attcggcgaa gcgcaaaaaa acctgaaaaa gaaatttaaa aaattctgcc agttcggctt 859800
taccggcacg ccgatttttc ccgaaaacgc tttgggcgcg gaaaccacgg cgggcgtgtt 859860
cgggcgggag ctgcattctt atgtgattac cgatgccatc cgcgatgaaa aagtattgaa 859920
attcaaagtg gattacaacg acgtgcgccc gcagttcaaa gccgtggaag cggaacagga 859980
cgagaagaaa ctgagtgccg ccgaaaacca caaagccctg ctgcaccctg aacgcatccg 860040
cgaaatcacg caatatatcc tgaatcagtt caggcagaaa acgcaccggc tgaatgcggg 860100
tggcaaaggc tttaacgcga tgtttgccgt cagcagcgtg gatgcggcga agtgctatta 860160
cgaagcgttc aaaacacaac aggcaggcag cttgcacccg ctgaaagtgg ccaccatttt 860220
ttcctttgcg gccaacgaag agcaaaacgc cgtcggtgaa attgtcgatg agacttttga 860280
accggaagcg atggacagca gcgcaaaaga atttttgcag gctgccatca acgattacaa 860340
cgcctgtttc aaaaccaatt tcggcacgga cagcaaagcc tttcaaaact actaccgaga 860400
tttggcaaaa cgggtgaaaa atcaggaaat agatttgctg attgtggtcg gcatgttttt 860460
gacgggtttt gacgcgccga cgctgaacac gttgttcgtc gataaaaacc tgcgctatca 860520
cggcctgatg caggcgtttt cgcgcaccaa ccgcatttac gatgccacca aaaccttcgg 860580
caatattgtc tgcttccgcg atttggagca ggcaaccatt gatgcgatta ccttgtttgg 860640
cgacaaaaac accaaaaacg tggtgctgga aaaaagttac gaagaataca tgaacggcta 860700
taccgacagc cagaccggcg aagcacggcg cggttatctg gatgtggcaa aagaattgcg 860760
cgagcgtttc cccgatcccg acaaaatcga aacggaaaaa gacaaaaaag attttgccaa 860820
actcttcggc gaatacctgc gggcggaaaa cgtattgcag aactacgatg aatttgccgc 860880
gctgcgcgag ttgcagagtg tggacgcggc ggacgaagat gcgatgaagg cgtttcaaga 860940
aaaatactac ctgagcgatg aagacgtgca ggaaatgcgg caagtgccga tgccgtctga 861000
aagggcggtg caggactacc gttccgccta caatgacatc cgcgactggc tgcgccgcca 861060

aaaagcaggc gaacagaaag agcaatcaaa aatcgactgg gacgatgtgg tttttgaggt 861120
ggatttgctc aaatcacagg aaatcaatct ggattacatc ctgcaactgg ttttcgaaca 861180
ccacaaaaaa atcaaaggca aagcggagct ggtggaagaa atccgccgca tcatccgcgc 861240
cagcatcggc caccgcgcca aagagggtct gattgtggat ttcatcaacg atacggattt 861300
ggacaaagta cccgacgttc ccgccatact ggaaaccttc tacacctacg cgcaagaggt 861360
gatgcggcac gaagcggcag gattgattgc cgccgaaggc ctgaacgaaa ccgccgccaa 861420
acgctattta atcagctcgc tcaaacgcgg ctatgccagc gaaaacggca cggaactgac 861480
cgaaaccctg ccgaaaatga gtccgctcaa cccgcaatat ctgacgaaga aacaaagtgt 861540
ttttcaaaag attgcggcgt ttgtggagaa gtttgccgga atagggaccg atatttgaca 861600
aaatgccgtc tgaaatttca gacggcattt ttgattttat gcggaggcgg tttttatttt 861660
gaccttgctt ttcttaaact tcaacacggc ttcttctttt gccgcatccc agtctatccg 861720
tacgaagccg ccgtcggata gtttgccgaa caggagttcg tcggcgagcg gtttgcggat 861780
tttttcctga atcaggcggt gcatcgggcg cgcgcccatt tgcgggtcaa aaccttttc 861840
cgccagatat ttgtgcaatg ccgacgtgaa ttcggcttcg actttttttgt cgaggagccg 861900
gtgttcgagc tggagcagga atttgtccac gactttggtg atgacgggtt cggataaggg 861960
cgcaaacggg ataatcgcat ccaagcggtt gcggaactcg ggcgtgaaga gcttgttgat 862020
agcctgcatt tcgtcgccgc gctcgcgttt ggcggtaaag ccgaggctgg gtcggctgag 862080
actctccgca cctgcgttag tggtcataat taggatgacg ttgcggaaat cggcactctt 862140
gccgttgttg tcggtcagtt tgcctgcgtc catgacttgc aggaggacgt tgaaaatgtc 862200
ggggtgggct ttttcgattt cgtccaagag caatacgcaa tgcggctgtt tgttgatggc 862260
ttcggtcaaa aggccgcctt gttcaaagcc gacgtagccc ggtggtgcgc cgatgaggcg 862320
cgatacggcg tggcgttcca tatattcgga catatcaaag cgttgcagcg gtacgcccat 862380
cgagtaggca agctgtttgg cgacttcggt tttgccgacg ccagtcggac cggagaagag 862440
gaaactgcct atcggtttgt cgggcagggc aaggccggaa cgcgacattt tgacggcagc 862500
aaccaacgcg tcgatggcgt tttcctgacc gtaaaccatg tttttcaaat cgcggccgag 862560
gaattgcagc acctgtttgt cgtcgtgcga cacggttttt tctggaatcc gcgcgacttt 862620
ggcgatgacg gtttcgattt gcgctttgcc gatgactttt ttctgtttgg atttgggcag 862680
aatccgttgc tccgcgcctg cttcgtccat cacgtcgatg gctttgtcgg gcaggaaacg 862740
ctcgttgatg tagcgtgcgg agagttcggc ggcggcttcg agtgcgcctt gagtgtagcg 862800
gacttggtgg aaggcttcaa acatcggttt caagccgcgc aggatttgaa cggtttcgga 862860
aacggtgggt tcgaccacgt cgattttttg gaagcggcgg cttaaggcat ggtctttgtc 862920
gaaaatggtg cggtattcgt cgtaggtggt cgcgccgatg cagcgcagcg aaccttttgc 862980
cagcgcgggt ttgagcaggt tggacgcgtc catggtgccg ccgctggtgc tgcccgcgcc 863040
gatgatggtg tggatttcgt cgataaacaa aatggcgtgc gggatttttt cgagctgttt 863100
caagacggat ttgacccgcg cttcaaagtc gccgcggtat ttcgtgcccg ccaacagcga 863160
gcccatatcc agcgcgtaca cttcggcatc tttaagcgcg tctggaatgc cgccgttgac 863220
gatttgatgt gccaaacctt ccgccagcgc ggttttgccc acgcccgctt cgccgaccaa 863280
aagcggattg tttttgcggc ggcggcatag gatttgcacc agccgttcca tttcgtgttt 863340
gcgaccaatc aaagggtcga tacggccggc tttgacttcg gcgttgaggt tgacggtgta 863400
cgccgataaa gggtttttgc ccggtttggt gcggtttcca ttatcgtcgt ccatgccgtc 863460
tgaagaatag ttgccatcgt cttcatcttc atcttcatct tcatcttcat cgggagagcc 863520
gtgggcaata cagcgcaaaa cttcaaaacg cgtaaccgat tgcagcttga ggaaatagac 863580
ggtgtggctg tcggtttcgc tcatcagcgc gaccaaaacg tccaacggtt cgactgcggc 863640
ttttccggca gactgggtat gcaccatcgc ccgttgcatc acgcgttgga agccgagcgt 863700
gggccgggtt tcgaccgtgt ctaaaaggtg ttcgggaatc agggggggtgt tttcggcaac 863760
gctggcggcg agctgttcgg acaccacttt caaatccgcg ccgcagagct tgaggacgtt 863820
gtggacggag gcatcttctt cgatgagtac caaaagcaga tgctcgaggc tgataaattc 863880
ataatgagcc ttacgcgcct cgcggtaaag ctgctgcaaa atctgttcca attcgggtgc 863940
aagcatatta aatctcctcg acaatacatt gcagcggatg cccttcggct tttgcccgct 864000
gcatgacttg ttgttgtttg gtttgggcaa tatcgcgcgt gtaagtgccg cacaggcctt 864060
tgccttcgtg atgaaccaag agcattaccg ctaccgcctg ttcttgtccg agcataaaga 864120
tttcggtcag gatttcgacg acaaattcca tcgtggtgta atcgtcgttc aataggaaaa 864180
cgccgtaacg tttcggcggc agggtgttca gacggtgcaa gagcgtgtcg gattggtgtt 864240
gcgcggtcat agtgtgtccc atttgaaagc cgcgttcaga cggcattttt gttgtatttt 864300
cggtactttt gcctattttc ccactttttt gaaaacatag cttgacgttt tgtcttaaca 864360
aatgtaaaaa gacggttaag caggattggg catccgccga gtatatcaag ctggaaggaa 864420
cgccagacgg ctcggtttgc cgtatgcctt gttttgctga ttttgttaat ttttgagtat 864480
aggaagtttc taatggcaac cggtatcgta aaatggttta acgacgctaa aggttttggt 864540
ttcatcacgc ctgatgaagg cggcgaagat ttgttcgctc acttctcagc aatcaatatg 864600
gaaggtttca aaaccctgaa agaaggccaa cgcgtctctt tcgacgtaac caccggccct 864660
aaaggcaaac aggccgccaa cattcaggct gcttaattcc tgatgtacgg tcaaatgtat 864720

atttgaaaac ggcgggacag gcaatgtccc gccgtttttg tctgccgttt tgccggcggc 864780
ggaaaaaccc caatccccgc acgccttatc ctgaacttgt gtgtaccctg ttgtggacaa 864840
gtggcttagt attttgacgg ataagggaaa atcagtgctg atgaaaaaat gtgcaatgtt 864900
gtcggcaaaa ggcggtgcgg cataaaacgg caaacgggta ggcacggggc aaaacgtgct 864960
gccttcgtct tcagacggca tcggcagggc gttcagcttc cggcaaccgt catccccgca 865020
atcagaatcg agccgatttt gttggacgaa cgccgcaaag cgtcatccgc cacgccgaca 865080
atgtcgcggt acatatcctg caagcgtccg gctacggtaa tctcgtggac ggggtaggca 865140
atcacgccgt tttccaccca aaaacccgcc gcgccgcgcg agtagtcacc ggtaatggtg 865200
ttcgcgccct gtcccatcag ttcggttacc aacaatcccg tccccatttc tttcagcagg 865260
tcggattgcg tttcgtgcgt atggttcaaa tacaggttgt gcgcgccgcc ggcgttgccc 865320
gtggtctgca taccgagttt gcgcgcgctg taactgctga ggaaatagcc ttcgacaatg 865380
ccgttttgaa tcacgaagcg cggtgcggtg gcaacgcctt ccgcatcaaa atagctgctg 865440
cggaaagagc gggggatgtg cggttcttcg cgcaggttga ggaaatcggg caggactttt 865500
ttgccgatgc tgtcgatcag gaaactgctt tggcggtaga gcgcgccgcc ggagagtgcg 865560
ccgacgaggt gtccgataag accgcccgaa acggtggtat cgaagaggac ggggtagctg 865620
cctgtcggga tgctgcggct gccgagtcgg cgcaaagtgc ggcgggcggc ggtttgaccg 865680
atggtttcgg ggctgtccat atccggatgg cggcaggcgg aatcgtacca gtagtcgcgc 865740
tgcatgccgt tttcgtcggc ggcaacgacg ctgcaggaaa tgctgtggtg cgtgccttgc 865800
cggtgtgcgg caaaaccgtg ggtgttgccg taaacgtatt ggtaatggcc ggtttgcacc 865860
gccgcgcctt cggagttttc gatgcgctca tcctcgttca gggcggcttg ttcgcattgt 865920
tttgccaagc cgacggcggc ttccgtatcc aaatcccatt cgtggtaaag gtcggggtcg 865980
ccgatgtgtt ttgccatcag acaggcatcg gcaagtccgg cgcaaccgtc ttcggcggtg 866040
tggcgggcga tgtcgatggc ggctttgacg gtgtcttgca gggctttttc ggagaagtcg 866100
gcagtactgg cgcggccttt gcgtttgccg acgtaaacgg taatgtccag cgacttgtcc 866160
tgctggaact cgatttgttc gatttcgccc agccgcacgc tgacgctttg tcccaatgat 866220
tcgctgaaat cggcttcggc ggcggttgcg cccgtcgctt ttgccaagtc gagcgtgcgg 866280
cggcagaggt cgaggagttc ggaagcggtg tggttgaaca gcatagaaat ctttcttgat 866340
gatgctttgc ggcattttaa ccgtttcggg cggcaggggc aaaagcgcgc cgtttgcagg 866400
gcggacggtg caaaatgccg tctgaacgcg gcggcattct gttaaaatgc gctattggaa 866460
aaattcgaga atcaagatgt ttgaacaaga agacgaatgg atcagcaaaa cccaaatgaa 866520
aaagcagatg aacgatttgc aggatttggg tatggcgttg accaagctct caaacgatac 866580
gctgaaaaaa atcggtttgg atgcggattt gtacgaggcg gtaaccgcct ataaaaaaat 866640
cacatccaac ggcgcgctca aacgccaggc acaatttatc ggcaggctga tgcgcgatac 866700
cgatcccgcg cccatcgagg cgtttcttgc caagctgcgc ggcgacgatg cggcgcacaa 866760
cgcctttttg caacgcgtgg aacaggcgcg cgtacggctg ttggcagacg acggcgcgtt 866820
gacgcagttt atgtcggatt ttccgcatgc ggacgcgggc aagctgagga cactcatccg 866880
caataccaaa aaagagcagg agcaaaacaa accaccaaaa aatttccgcg ccctgtttca 866940
agagttgaaa accgtgatgg aaaacgggga cgcggaaatt taggcatatt ttcagacgac 867000
atccgccgtt atttagattg gaggataaaa tgttgttccg taaaacgacc gccgccgttt 867060
tggcggcaac cttgatgctg aacggctgta cgttgatgtt gtggggaatg aacaacccgg 867120
tcagcgaaac aatcacccgc aaacacgttg acaaagacca aatccgcgcc ttcggtgtgg 867180
ttgccgaaga caatgcccaa ttggaaaagg gcagcctggt gatgatgggc ggaaaatact 867240
ggttcgtcgt caatcccgaa gattcggcga agctgacggg cattttgaag gcagggctgg 867300
acaaaccctt ccaaatagtt gaggataccc cgagctatgc tcgccaccaa gccctgccgg 867360
tcaaactcga atcgcctggc agccagaatt tcagtaccga aggcctttgc ctgcgctacg 867420
ataccgacaa gcctgccgac atcgccaagc tgaaacagct cgggtttgaa gcggtcaaac 867480
tcgacaatcg gaccatttac acgcgctgcg tatccgccaa aggcaaatac tacgccacac 867540
cgcaaaaact gaacgccgat taccattttg agcaaagtgt gcctgccgat atttattaca 867600
cggttactga agaacatacc gacaaatcca agctgtttgc aaatatctta tatacgcccc 867660
cctttttgat actggatgcg gcgggcgcgg tactggcctt gcctgcggcg gctctgggtg 867720
cggtcgtgga tgccgcccgc aaatgaacag caatgccgtc tgaaaagctt tcagacggca 867780
ttttaagcac acacgcacag taaaacccca cgttatgtca gtcaaaatcc aaacccgatc 867840
cgtcaatacc gacgttttta atcatttgct caccgccggt gccgatcctt taatcgccca 867900
gctttgtgct tcgcgcggtg tgcaaagtcc tgccgaattg gacgacaaac tcgcttccct 867960
cctgccttat caatcgttga cgaattgcga agccgccgcc cgccgtttgg cggatgcggt 868020
tgggcgcaag gaaaaaatcc tgattgttgc cgactatgat gccgacggtg cgacggcgtg 868080
tgccgtcggt atgagcggtt tggcggcgat gggggcgaaa gtggatttcc ttgtgcccaa 868140
ccgctttgaa cacggctacg gcttaacgcc cgaacttgcc gaaatcgctg ccgcgcaggg 868200
cgtggatttg ctgattacgg tggataacgg tatcgccagc atcgcaggcg tggcgagggc 868260
gcaggctctg ggtttggatg tcatcgttac tgaccaccac ttgccggccg agaccgtgcc 868320
cgactgcatc atcgtcaatc cgaaccaaaa aggctgcggt tttccaagca aaagcttggc 868380

gggcgtgggc gtgatttttt atgtattgat ggcgttgcgt gccgaattgc gccgccgcaa 868440
ttatttttca gacggcatca aagagccgaa tttgggcgaa cttttggatt tggtcgcact 868500
cggcacggtt gccgatgtcg tccctctcga ccacaacaac cgcatcctcg tgtcgcaagg 868560
tttgaaacgg atgcgctccg gcaaaatgcg ccccggtatc cgcgccttgt ttgaagtggc 868620
gcggcgcgat tggcgcaagg cgcagccgtt tgatatgggt tttgcgttgg gcccgcgcat 868680
caacgccgcc ggacggctgg acgatatgtc ggtcggcatc gcctgcctgt tggcgcgaga 868740
tgattccgaa gctcaggaac tggcggctca gttaaacaac ctcaatatcg agcgccgcga 868800
aatcgagcag tctatgctgc aagacgcact gaatgatttc cccgaaaccc tgccttcagg 868860
tcagatgact ttggtggcgt atcgcgacga cttccatcaa ggtgtggtcg gcattgtcgc 868920
cagccgcctc aaagaccgtt tttatcgtcc gaccatcgtg tttgcgcctg ccgacaacgg 868980
cgaagtacgc ggttcgggac gttccattcc caatttgcac ctgcgcgatg ctttggactt 869040
ggtgtccaaa cgccatcccg atttgatttt gaaattcggc ggacacgcga tggcggcggg 869100
tttgagcata cttgaacaca acattcccgc gtttcagacg acctttgaag aagccgtgcg 869160
cgaaatggtg tgcgaagacg atttgtcgca aaccttcatc accgacggca gcctgcccgc 869220
ctgcgacatc acgttggaac aggcgcaaaa ccttgcccgt cacgtttggg ggcagggctt 869280
cgcgccgccg agctttaccg acgagttcca cgtcgtccgc cagcaacctt tgggcgcgga 869340
gggcaaacac aaaaaagtct ggctgcaaaa agacggctgc gaatttgaag cgatgttttg 869400
gcgttgcagc gaagacattc ccgaatacat ccgcacggtt taccgccccg ttgccaacga 869460
atggcgcaac aatctcgaat tgcagctgta tatcgattac tgggaagccg cgtagaggcg 869520
gcggaacact gtttgaatgt gatttctgtt ccttcatttg cctgtttgta cgacgggaat 869580
gttcccaatc ggagaaggcg catcaaattt cagactctgc cacaaaagca gggtctgatt 869640
tttttggagg gcaatctgtt ataatgacgc gttgccgccg cgagggcggc gtgattcgga 869700
cggcgtagtt tctacgcctt ttgtttatgg ttacggcatc ttgcaaaccg cgcctgatgc 869760
cgtctgaaca cggttgcctg tggagatgcc gctcttcggg tcagaatatt tatgctgaaa 869820
aaatggttga ataagatgct gccttccggt cggagcagta aaaaagcgga aagtaaaacg 869880
gtcattcctg ccgaaagaca caacatccgt gccgaaatgt tgagctttgc cgccgaaaac 869940
gtcatacgcc gcctgaaagg gacggggttt caggcttatg tggtcggcgg cgcggtcagg 870000
gacctgctgc tcggcatcga acccaaagat ttcgatgtcg caaccgatgc cacgcccgaa 870060
caggtgcaca aactcttccg ccgcagccgc atcatcggca ggcgttttca gattgtccat 870120
gtgatgaacg gtgcagagac tatcgaagta acgacgtttc gcggcggtgc gaaagtacat 870180
cagaatgcac gcggcaggat tatgaaagac aatacctatg gcagcatcga agaagatgcg 870240
atgcggcgcg attttacctg caatgccttg tattacgatc ctgaaaaaga agagattttg 870300
gatttccaca acgggattgc cgatgttgcc gcccacaggc tggttatgat tggcgatgcc 870360
gccgaacgct atcaggaaga ccctgtcagg attttgcgcg ccatccgcct gtcgggcaaa 870420
ttgggctttg agctgtcgga agaaaccgcc gcaccgattg ccgaatcgat atgccgtctg 870480
aagcacgaac cggtagcgag gctgttcgac gaaattatga aattgctgtt ttcagggcac 870540
gctcgcgagt gtctaaaacg tttgaacgga tttgacatac cggacgacat ccatctgctg 870600
ctcaatgcct tgcgcgtttc agacggcatc gccggaaaaa tgacggtgct tgccctgaaa 870660
aataccgatg agcggctgcg tgccgacaaa tcggtttcgg tcggttttgt attggcggct 870720
ctgatgtggc ccgagttgga acgccattgg aaaagcaatc tgcaacaggg tttgaaaccc 870780
gcgcccgccc tgtccgatgc aatcaatacg atgcgcgaaa ccgtcgaacg cggttggggc 870840
gtgccgcaac gcttttccgc cacgatgcgc gaaatttgga tgttccagcc gcagtttgaa 870900
aaccgcaaag gcgcaaggcc gcacaaactg tttgcacagg cgcgtttccg tgccgcctat 870960
gatttcctgc tcttgcgcgc cgaaaccggc aatgcggacc gcgcccttgc cgagtggtgg 871020
acggcgtttc agacggcatc gacggaacag cggtcggaga tgaccaaaaa cgaagccgcc 871080
gcccgacacg aaaaaaacga aggacaggcg aaaaaacgcc gccgtcgcag gcgcaaaccc 871140
aagccgaagg ttgtgggaac ggattgggaa taagggtcaa cagacatgga gcaatgaagt 871200
ttcaacacat gggatgaagc ataaagtgcc gttctatgca ttatcctgat ttgtaagggg 871260
attcatcccc gtaaataaag tctaaccctg cctctcggaa aaaggatgtc cgggtgggca 871320
gggttcaagc aacaaggaaa aattgatgaa aaaatgtatt ttgggcattt tgaccgcgtg 871380
tgccgccatg cctgcatttg ccgacagaat cggcgatttg gaagcacgtc tggcgcagtt 871440
ggaacaccgt gtcgccgtat tggaaagcgg cggcaatacc gtcaaaatcg accttttcgg 871500
ttcaaattcc accatgtatg tatgcagcgt tacgcctttt cagaagacgt ttgaggcaag 871560
cgatcggaat gaaggcgtgg cgcggcagaa agtgcgtcag gcgtgcaacc gcgaaacttc 871620
ggcaatgttt tgcgaagatg aggcaatccg atgcagaaaa ttcgattgat gtatcggttg 871680
gacggataaa gaaacggata cggatacgga gcttggcttc cgtatctgtt tttctctgcc 871740
tgattttcca tgcatcgggt ttcagacggc attggaatgt cagtcgtgtt ctgccgattc 871800
gtaggcttcg acgatttttt gcaccaaagg atgccggaca acgtcttcgc cggtaaaggt 871860
gtggaaatac agcccttcca cgttgtgcag tttctcacgc gcatctttta atcccgattt 871920
gatgtttttg ggcaggtcga tttggctggt gtcgccggta atgacggctt tcgcgccgaa 871980
gccgatgcgg gtcaggaaca ttttcatttg ttcgggcgtg gtgttttgcg cttcgtcgag 872040

gatgatgtat gcgccgttga gcgtcctgcc gcgcatatag gcgagcgggg cgatttcaat 872100
caggcctttt tcaatcagct tggttacacg gtcaaagccc atcaggtcat agagggcatc 872160
ataaagcgga cgaaggtagg gatcgacttt ctgggtcagg tctccgggca ggaagcccag 872220
tttctcgccg gcttcgacgg ctgggcgcac taaaatgatg cgttcgactt ggtgtttttc 872280
catcgcatcg acggcggcgg caacggcgag ataggttttg cccgtacctg ccggcccgag 872340
accgaatacg atgtcgtggt tgagcagggc gcggatatag ccgttttgcc gtggcgttct 872400
gccgccgatg ctgccgcgct tggtgcggaa ataataggcg tggtcatggt tttttcttg 872460
atgaccggca tcttcggttt gggcttcgac ggcggcaagc ctgatgtcgc cgtcgtttag 872520
gtcgcgcgtc tgcgccgttt ccaagagttt gagcagtgcg cgtttgccgg cgtgtgcaaa 872580
tgcgccgttg aaagtgaaat gttcaaaacg gcggctgatg tggatatcga gtgctttggc 872640
aagtaaatca aggttgttgt caaaagaacc gcacagacgc tgcaacgcca agttgtcggt 872700
ttcttctaaa tgcaggtgga cggtatgtgt catatgaagg tccgaatagt tggatattgt 872760
gtgattttaa tctatagtgg attagattta aaccagtacg gcgttgcctc gccttagctc 872820
aaagagaacg attctctaag gtgctgaagc accaagtgaa tcggttccgt actatttgta 872880
ctgtctgcgg cttcgtcgcc ttgtcctgat ttttgttaat ccactatatt tcaccggtat 872940
tttcttaccg tattctgcga ttgcctgtcg gaaaatgccg atcaacctgc ctataacggc 873000
attttcgcca aattcgttca gacagttttc tctaagtcgg gcaggttcga aatcagagtg 873060
gtgttcacac attttgatga gtgcgtcggc aagggcatcg tcgtcgtcaa caggaaccaa 873120
atatccgttg ccgtctgaaa caatagattc cgcaccgccg cagcgtgttg caatgacggg 873180
caatccttgg gacagtgctt cgatatagac tacgccgaag gtttctgtgc ggctggcaag 873240
gacgaatgcg tcgctgttcc tcatcaaatc caagactgct tcgggctgca atgcgcccaa 873300
aaatgtaacg gcatgggtaa tgcccaagtc tgccgcctgc tgtttcagcc gctgttcttc 873360
ctgtccgctg ccgccgatgt tcaggcgcag ttgcgggcat tgtgccaacg cccgggcaaa 873420
ggcagtgagt aggacatcgt gtcctttgag acggcgaagg tgcgagacgg tgcagaacac 873480
gaaatgcggt ttgttgtttt ttttctcttt gttcaaacgg tcggttgaat attccgccca 873540
gtatgttggg ggaggtactg ccattcgcag ccgtatttgt gttgcaggac gtgtgcgaaa 873600
tggcggctga cggcgagacg tgcggcggcg tgtgccgccg catttttcat aggctgccat 873660
tggtgcgggc gcaccaaacc gcgcgtaatg gtgctgctgt gttccgtgac gacatagggg 873720
atgccgtatt tttgggaaat cttgaaggca agtatgccgg catagttcat acagtgggcg 873780
tgaatcaggt cgggcagccc gttttcgcgg atgtagtgtt tgaaagcttt caaacccgca 873840
cacacccagc ggatgcggtc gatgtcgatg aacggaaagc gggggaagaa atacatgccg 873900
tgccatgcat agatgtccaa accgctttgc cgatatagtg gattaacaaa aatcaggaca 873960
aggcgacgaa gccgcagaca gtacagatag tacggcaagg cgaggcaacg ctgtactggt 874020
ttaaatttaa tccactatat tttgcaaaac cgtaagggtc ggtcaggatg cttgctgttt 874080
ctttccgcaa gtagcggaac atcggtgcaa gcacggcggt tttgatgcct ttcctctgca 874140
atgccagtgc ctgattttga aaaaaatccc gtccacatcc tgttcggatt gcggatacca 874200
tgaggggatg acgaggacgt gcaagggttc gggcatagtg ggattccgta tcggaaaggc 874260
ggttattata agacagacgc agaccgaata tttaaattgt tgccttacgc taatgcaatt 874320
tggcgcgccg gtgtgttaga ttggcagttt tatcggtaag gaggcggata tgttgcgtct 874380
tgttttggcg gcttcgctgt cggcggtatc ttttccggca gcggctgaag cattgaatta 874440
caatattgtc gaattttccg aatcggcggg tgtcgaggtg gctcaggata caatgtccgc 874500
acgtttccaa gtgacggcgg aaggacggga caaaaatgcc gtcaatgctg agtttgttaa 874560
aaaattcaac aagttcatca gaaaatcgaa aaatggtagc tttaaaaccg aattggtatc 874620
gcgcagtgcg atgccgcgct atcaatatac caacggcaga cgcattcaaa caggctggga 874680
ggagcgtgcg gaatttaagg tcgaaggtag agattttgat gagttaaacc gttttattgc 874740
cgatattcaa gcagatgccg cgttggaata tacggatttc catgtgtcgc gcgaacgccg 874800
caacgaggtc atcgatcagg tcagcaagga tgccgttttg cgtttcaagg cgcgtgccga 874860
aaagttggcg ggcgttttgg gtgcgtccgg ttataaaatc gtcaaattga atttgggaca 874920
catcggcagc catatcgcgg gaggggggagc tgctcaggca aaaatgcttc gtgccatgcc 874980
gatggcggca agcgtcaata tggagggtgc ggattccgcc gcgcctggtg tggaggaaat 875040
cagcatcagc gtcaatggga cggttcagtt ctgatttgag gtgaacggca aatgccgtct 875100
gaaacccgac gataagggtt cagacggcat ttatatttca ggctttgggc agggtaacgc 875160
cggtttgccc catatatttg ccgttgcggt ctttgtatga ggtttcgcag atttcgtcgc 875220
tctcgaagaa gaggacttgc gccacgcctt cgcctgcgta gattttggcg ggcagggggg 875280
tggtgttgga aaattcgagg gtaacgtagc cttcccattc cggttcgaac ggggtaacgt 875340
tgacgataat gccgcagcgg gcgtaggtgg atttgcccaa gcagacggtc aggacgttgc 875400
gcgggatgcg gaaatattcg accgtgcgcg ccagtgcgaa ggaattgggc gggatgatgc 875460
agcagtcgtc ttcaacggta acgaagtttt tcgggtcgaa gtttttggga tcgacgatgg 875520
tgctgttgat gttggtgaag attttaaatt cgtttgcgca gcggatgtcg tagccgtagc 875580
tggacgtacc gtaggagatg atgcgtttgc cgtcggcttc tttgatttgg ttcggctcga 875640
aagggtcgat catgccgaat tcttcgctca ttcggcgtat ccatttgtcg gacttgatgc 875700

tcataatgtt ttccttgttt cttgcagtgt tcggacaaag cattggggga tgccgtctga 875760
aaacggggct tatttgtttt tgggcagttt cacttcttta atcatgccgt tttcgcattt 875820
cataatgaag agggtttcgc cgttttgcga gacggtaaat ctgccgtgtg ccaagccttt 875880
tttgaacgtg cccgagagta ccatgttgcg gaatttggta ctgtcggaat tgaacggttc 875940
gataaatatt tcgcggttgg cggcaacggt ataaacgcct tgcccgtcga atttgccgtt 876000
tttaaacgaa ccggtatagt tgcgcccgtc ttggcagcgc catgtgcctt tgccggcggg 876060
tttaccgtct ttgccgacat tgccgtcgta ggtgcagcct ggttcttgat aggaagtcag 876120
gacggcggcc gaagtgggga gggcgaacat catggcgggc agtaggaatg cgagatgttt 876180
aagcataagg gttattccat tggattttgg ttgacggtat tttgtcgtga aaaagccgtc 876240
tgaaaaatca atcttgccag ccgcccaaat aggaaaccag ttcttccaac atggtgctga 876300
cggattccgc catcagaatt tgcgaggcga aggcaaggcc ggcggcatcg tcgccgttgc 876360
tttcggcttc ttcctgcaat acgtcgaggt attggatgcg cttgagtgtg aagtcttgag 876420
tgaggataaa ggcaatttgt tcgcgccaca ccaagcccag ttgggtaacg gttttaccgt 876480
ttttgacgtg ttgaaccact tcgtcggcgg ttaaatcttg tttggatact ttgacgacgg 876540
gaacaatatc gcccgtacct ttgagttcgc aatcgctgtc taattcaaaa ccgccttcgc 876600
aatgcccttg caacagccag ccggtcatca aggaagaggg cgattgcttg gtattcggca 876660
gcgaggcttc caaaccgccc aaagcttcgc gcagcttggt caggatgttt tctgctttgg 876720
cggaagccgc gttattgacg agcaggtagc cgtggcgggt gttaaacacc gcttctgtac 876780
ggctgctgcg ggtaaacgct cggggcagca ggtcgtctgt aatttgctct ttaagctctt 876840
gtttttcttt acggccgaca ttgcgggctt cattgttttg gatttccgct accttctctt 876900
ccaaaatatc gcggatgacg ccggcaggca ggactttttc ttcttttttc agggcgacgc 876960
gcaaggtaaa gtcggcaggg aaaacgagtt caggggagaa tgaaaccggt gcggtaaagc 877020
cttcgctgaa ccagtctaag ccttggcaat gggtaaattc agcttcagca agtttgtcgg 877080
caagtacgtc tgcctcaggc agcttttctt tgttgagcgg ataaaaacta atctgcttga 877140
accacataat gtttcctatt gtttgaaatg tcgggaatta tttgctgaat tgttttttca 877200
cactgacttt ggttttcttc ttgaagcggt ttttctcttc ctttcggcct tcgcgtttct 877260
caatacggtt gctcaggctg acgcggcgca gcggtttttt cttcggtttg tcttccgcat 877320
tttcatacgg gttttccgaa acattgtatt gaattctgag cggcgtgcct tgcagattga 877380
aggctttgcg gaacgtttgg gtcagatagc gcgtatagct gtcggaaatc gcgtgcagcg 877440
aattgccgtg taccacaatt acgggagggt tcatgccgcc ttggtgggca taacgcattt 877500
tcggacgcac caagccggca cgcggcggtt gctgacgctc gatcgcgctt tgtaatacgc 877560
gcgtgatttt cggcgtcggc atcttaatca tcgccgcgtt gtaggcagcc tgaatgctgt 877620
caaacaaacc gtctataccg cgctctttca atgcggaaat aaagtggaac ttggcaaaat 877680
cgaggaaata cagtttgcgg ttgatatcgc gtttcacttg ctcgcgacgt tcttcgctga 877740
tgccgtccca tttattgacc gccaccacca aagcacggcc tgcctccaaa gcgaaacctg 877800
caatcgtcgc atcttggtcg gcgatgtcct gctgcgcgtc caataccaaa acagcgacgt 877860
ttgccgcttc aaccgcctgc atcgctttga taacggagaa tttttccact gcttcatcca 877920
ctttgccgcg acggcgcaca cctgcggtat cgatgatggt aaacggtttg ccttcgcgct 877980
cgaaatcgat atggatactg tcgcgcgtcg tacctgccat atcgaaggtg atgacgcgct 878040
cttcgccgag aatggcgtta accagcgtag atttgccgac gtttggacga ccgataacgg 878100
caaaaacagg atgtcttgca tcggcttctt cggcttccgg ctcggggaat ttttccaaaa 878160
tatcttcaat cagataatac acaccatcgc cgtgtgcacc tgaaataaca taagggtcgc 878220
ccaaagcaag ttcgtagaac tcggcggcaa gtacagccct attgcccccc tcgcctttat 878280
tcacggccaa ataaacaggg cgcggacttt ggcgcaaacg gtcggcaata atcttgtctt 878340
gcggtgttaa accggtacgg ccgtccacca aaaacacaac tgcatcagct tcatcgacag 878400
cctgtaaggt ttgttttgcc atttcgtgca aaatgccgct gtccacaacc ggctcgaaac 878460
cgccggtatc gatgaccaaa taaggtttgc tgccgacttt gccgtgtccg taatggcggt 878520
cgcgcgtcag accgggcagg tcatgcacga gcgcgtcttt ggtgcgcgtc aaacggttga 878580
ataaagtaga tttgccgacg ttggggcgac caacaagcgc gatggttggt ttcatgattg 878640
agtctttctg tgtcaagtgc cgttcgggag aactgaacac gagcaggtgt ccgttggaca 878700
cggcggatgg gttttacggg aattgccgta ggatagtgtt gtctgaaatg ccgtctgaag 878760
agagggtggc atttcagacg gcatttattt cagcgaatca agtttcattt gaaccaattc 878820
gcgaccgaca gaatcttgag gcattttttc taaagcctgt ccgtagtttt ttaaggcttc 878880
ctggcttttt ccctgtgcgg catagacatc gcctttggtt tccatcagca ggggggcgaa 878940
gtccgcttca accggcgtat cgagcgcggc aagcgcggca tcgtattttt tttgttgcaa 879000
caacacaacg cccagacgct gcgccgccaa cgcttgaatc aggctgtctt tttggttgga 879060
caacacccat ttcaaatggc cttcggcaac atcgtaacgc tgcgcgtcaa attcggttgc 879120
cgccgccatc agtgtggctt gggcggcgga aatggaatgc gggtagcttt gttggagttt 879180
ggtcaattcg gcattgattt cgctttgcgg ggctttgctt tgcgcctttt ctacgatgtt 879240
tgccagcacc gccgccgctt cctgattttg ggaaacttta cggttttggt aaaccgtgta 879300
tcccaagtag ccgagtgccg ccaaaatcag caaggcaaac agccatttgc ccgtggtttt 879360

ccaaaaatat ttaaagttgt ctaactcttg ttgttcttcg agatgggctg ccatttatgc 879420
gttcttccat tgttgtaaag taggggttaa atcctcggcg gcgacagttt gctgaccgtg 879480
tgcgccgttc atgtctttga gcgtaaccgt accgttcgcc agttcgtctt gcgcgacaat 879540
cagggcaaag cgtgcgccgc tgttgtcggc ttttttcatt tgcgctttca ggctttgata 879600
gccggaatgc tgcattacat tgaaaccttg cgcgcgcaag gcttgtgcgt atttcatcac 879660
ctgcaagtcc gccccttcgc cttggtgcat tgcatagaca tcaggcgcag cgttcacttc 879720
cagagagccg tattcgctca ccaaaagcag cagccgctcg atacccattg caaagccgat 879780
agacggcgca ggtttgccgc cgagttcttc aatcaagcca tcgtaacggc cgccgccgca 879840
cacagtcgcc tgcgcgccga gtttgtcggt cgtccactca aaaaccgtct gattgtaata 879900
atccaaaccg cgaaccaagc gcggattttc aatatattgg atacccaaac catccaacat 879960
cgccttgaag cgtacatagt ggttttgcga atcctcgccc aagtaatcca ccaaacgcgg 880020
cgccgcgttg cagatttcct gcaaatctgg gtttttcgta tccaaaacgc gcaaaggatt 880080
ggttttcaga cggcgtttgc tgtcttcatc caatttatct tcataacggg tcagatattc 880140
aaccaatgcc gcacggtgtg ccgcgcgttc ctcacggttg cccaagctgt tgatttccaa 880200
agtcaggtat tcgcggatac ccaatttttc ccataagtcg gcagacatcg cgatgatttc 880260
cgcatcaata tccggccctt caaaacccaa agcctcgata ccgacctgat ggaactgacg 880320
ataacggcct ttttgcggac gctcgcggcg gaacatcggc cccatatacc acagcttttg 880380
cgggctattg tacagaaggt tgtgttcgac caccgcacgc aggcaggaag ccgtaccttc 880440
aggacgcaag ctcaaactca aagaatcgtt tgaatcggag aaggtgtaca tttccttgcc 880500
gaccacatcg gtttcctcgc cgatggagcg gacaaacaaa ccggtttgct cgacaatcgg 880560
cgtacggatt tgctgataac cgtaagcgcg tgtccagcgg ccgaccgtat cttcaaacgc 880620
ctgccaaaac gcagccgtca gtttgaaatc tttttgcttg acaggcagaa ggtcgttcat 880680
gcctttgacg gattggattt tttgtgccat ttcaagtaag aatgcttaaa tcaaattgcg 880740
ggcgattata gcggatttta aagggtttgt gaggttggag gtggtttgcg gacggcattt 880800
gacttactct gcacgtgctt gcctgatttg tccgactgta aactccgtct gccgttttgg 880860
gtgttgtgtg aaaaacaatt tatttgaaat tgtctcggct tttttcggta tgacagccaa 880920
aatcttacct gccaaatttc cctcacgggt ttgccaagca tccaaaaact gcgccctgct 880980
cattgaaaca tgccccagcg acgggtcggc aagcaaaacc gtattgccgt ctataccgcg 881040
caataccgaa aaatggtcgt ctttgcggta tttcagatac acgatgacgg ggattttcaa 881100
ctgcgcgagc tgctcgaaag acagggcata gcccttcgcc tcaaaaccca aatcaggcat 881160
aatgcgccgc atatcctcaa acgacgcgcg catctgctcc ttatccagct ttttcaacac 881220
ttcttcttcc gtcagcgttt gcccgtaaaa attgttcaaa agcgtcgcca ccgaagccgc 881280
cccacaggaa aaatccaaat cctgctttac aatattgaaa tcccgccgcg ctttccaact 881340
ctgcactttg atttttccgt aaacaacagg attatcgtta aacatcggtg cagcattcaa 881400
acgataagat aaagaaacga caacacacgc caacagaaaa acatatttga acttcatcat 881460
attgtccaca taaagggcag cctgaaaatc tttcaggctg cccttgtcaa attattccta 881520
gctttcggct tttttggcaa accaaacaat ccgattaccc gcataatact ttccatttat 881580
tgaaatccga caagccgcgc ccaaaaaatg ccatgcactg tcgatttccg cagcaatctt 881640
tgtaccgttt tcttcaaatt ccaaatattc acccaataat aaacttgaaa cagaacgcgt 881700
gggcagcaag tgcccaccct acgcttattc aaataatttg attaaataaa gaataaagaa 881760
aatgcatcag gaacaaaatt ataatctgcc acctgactca caccgctttt aaagtaaggg 881820
gcatcaaaat caaaaccgca aaaaaaataa tttttgcatt gatttttaat agatttaaaa 881880
ttcgaatata gtgtttctct agatttaaaa agtttatctt tatctatata tttgatgctt 881940
tccctatcca aaataaatat ttcaaacatt aaaaaatcat tacatgacca agccaaatca 882000
taaatttgat tcaaatgggt atcataaaga taaaaataat aaggtttggg aacaggtaaa 882060
atatttaatg gaaaaggagg actaattttc ttaatatctg atgactgata tccatatctt 882120
tctatattct taaaaatttc atctttctta agataacaag acattctgac aatcacaacc 882180
tgttcatcgg atatattatc tatttgcatg gcgcgtataa cacgccatgc ctgattaaaa 882240
ttagtctccc ttaccttaaa atctattatc ttttccaaag atgaaaatgc cccttatctt 882300
gaactctcca gttagaacct ggaagtttcg tacctcttaa atgtgtatta atatttctta 882360
tagtttcatt aaaatgccac gcgctgccga tttcaacggt aattttcgta ccgctttctt 882420
caaattccag gtattccccc attagctaac gcaaagaagc agacgccatt tcggcttcgt 882480
tatgataaac ccgccttccg ttgatataga cttccgcccc tgtccgctcc aaattccaaa 882540
aattccgtac tgaaatttcc atatcccgat attgtgcaga ccatgttttt tcgaaggttt 882600
tcataaaatt tcctatacct gtccaatcgg cacatatcaa ttgcattatt acatctcaat 882660
acgataaata tttcttaagt caaaatgcaa gcctgaccgt accttaactg tcaaaatttt 882720
attatttttt attgatttta aaacaatttc tgtaaaattc tcttcgcttt ctctcttttt 882780
tagaagcaca taagaaaaaa taaaacttcc ccgattaaat tcataaatat gtttcaacca 882840
ttcgcctcct ctttctgtaa gacaagattc agtttcattc ttccttattg tataaatatt 882900
tccttcacaa aatctgaaat aaatccataa atccatctta tccataatta aagaaaaagt 882960
ttcacctcga gattttgtca acaattcgca aggttgcgat gttgcaatca aatagccgaa 883020

agacattttt tacctcatac atggtcgaaa tcagtttctg ttagttcaga atccattttt 883080
tcgtcaacaa ctgaatccgc atttttgaat taacgttttc atcagctgcc gtttatctaa 883140
accggcaggt tcagtttcaa aataagcctt atatgaagac tgtaagcatt tcagaaaaag 883200
atcatcagaa gacatatctg ccgaatcaaa tacaactgtt ttgattttgg tacttaccca 883260
aaaccctttt tgctcttttt ctactatacg gaaattcaga atatttccaa ccgaatcaaa 883320
agcacggtaa acatcatcca tcaaatcctg cggctctatt ttcttttcca attccgacaa 883380
tccttgaaat atatccaaag acacatcttc aaatagaaaa aaaggaggag ttagaagcgg 883440
tttttccatg atctgtccgt agattttgat tcccaagggc gatgacgacc aattccctgt 883500
ccaggcaaag tcttgcccgt attatccgta actcgacgat gataatgggg aaattttcca 883560
ataggatgac ctgttctatt accgaaaggg gctatccgca tattattgcc gattttaatc 883620
tcacgtccat atttagcaaa ggaaacaacc tttcctgcgg cgcctacacc accaggaatt 883680
gcgcctaatc cgccagcaat agcaacatct ctaacagaag ctggtctgcc tgtcgttgca 883740
taactaaaac catgctgtgt ccacatacca atggcagcac cacccaagat agccaatgga 883800
agaaacgccc cctctgtctc cttcatctcc ttttgagaaa gctccgccaa ctgcatcggt 883860
gcatctgccc gcgtgtggaa cacttggtct tcaaatgcct gattgtccaa gccgtttgcc 883920
attgcggggg caatcataga cagcatcatt acggctgcgg tgatttgttt tttcataata 883980
actcctttgg attacaaggt tggaaaatca aagccctgct tagaacgtat gttgcacacc 884040
caatttcaat aggtaaatca gattgcaaat ccagcaattt gaatattgtc attgttccgt 884100
gcaaaaggga tttttattga tgagttgtgt actgggtttc agcttggctt tttagataat 884160
cttttcacaa gaacatacat caatactgtc caaaaaaaaa gatttgataa atatattgca 884220
acatatttta ttccacctcc atccgaaaca gaaaccaata tatttttaat taatacatag 884280
ctgattatta catttattat tcctataata tataaggatc ttgccaaaat ttttttttgat 884340
tttattttag gaatatctct tatccatgct aaaatacagc ccaatgtcga aaagaaaata 884400
acagcaaaca ctaccttacc tcctctcact tccaaccaaa ccgatagcag gtttggttgg 884460
aagttggttt atttattatt taacggcgaa tgtcagtgtt cttacccgta gaacctgcat 884520
aaccatttat gccaccagca acagtcccca ctgctacacc tcgcatacca ttaatcagaa 884580
ttcttcctat tttttcgaat ccactatttc ttccaaagat gcagcaaacg caggttgagc 884640
ctagacccaa tctacgtttg ctgccgcatc tgatccctat tgtttcttat ccttacatct 884700
tcctgccttg tcaatcaaat aaagacagaa gactatacaa aaactgaccg acatactgaa 884760
aatacctatc cccttccatg caatctccgc accattgacc caatagatta gactgagaaa 884820
caacaaagcc acagtataaa tcaacgtaaa aaatattgcg taaagcacaa taaagggaac 884880
ttttatctca cggttgtttt ttataatata ttcagcaact tgatttccga atatacctga 884940
taaaaaatat agtattagat catccatttc gtttatcttc tatgttttcc cattgcggcg 885000
ctagaagact gatttaatgc tgcaccattt gcacgaataa ctgcattagg aatagcattt 885060
cccccttcc aagcagaacg ggcaaaaaca ctagtagtaa tccctgcacc gcgcaacatc 885120
gcactgctat atccgccgcc tatcatacca ccagcggttg cagccaaggt acttcttgtt 885180
gaagcaaatt gccctgtttt aattttagaa atgccgtgat tagcccaagc actaattgcc 885240
cctcccatca aagcacctgc cacaatagga agaaacgccc cctcagtctc cttcatctcc 885300
ttttgagaaa gctccgccaa ctgcatcggc gcatctgcct gcgtgtggaa tacgtatttt 885360
tcaaatgcct gattgtccaa tccgtttgcc attgcggggg caatcataga cagcatcatt 885420
acggctgcgg taatttgttt tttcataata actcctttgg attacaaggt tggaaaatca 885480
aaacctgctt aaaatgtatg ctgtacgcca aatttcagtt cggaactgct ttgccctgaa 885540
acgttgaaac gtgcggatgc gtttaaagcc gtggttttgg tgaaaccgaa acctgcgccg 885600
aaatgggcgt aggtggatgt gtttctggag gattcccgtt tgccgtccgt ccggtcgggc 885660
tgcctgccca gccattggat gcctccggtc aggctgattc tgtcgttggc agcaaatgag 885720
atgttggggt tgagcagcag gtagttgccc gatttgtagc ggatgccgtc tgaaagggtt 885780
ttgctgccgt tgatgcggta ggcggcggtg agggaaagga caatcggatc tatggctttg 885840
taggtggtgg cgccgatgag ccaggatttt cccgacgagg ctttgttgcg cgatttttcg 885900
taaaccgtgc tttcaagaaa gctgattagg gcggggtttt tgtcgtcttt aaggaaagtg 885960
tggctgatgc cgagggatac gtcggacatc cgtttgttgc gggttttgct gttgccgtcg 886020
agtttgcgtt cttcgtgcca cagatagctg ccgctgccgt aaatgtcggt attcccggtc 886080
agtccgtagc gcaaaccgag cgtgccgacg agcatatcgg tattgctgcc gttttcttgg 886140
atttcggtcg gaatggggat aaacgaggtt gcgccggttt gaatgtaaac cggtgcggca 886200
agttcggcgc ggttgttttc gctgttcagg taggtaaggg aagtttccag tttccatttt 886260
cccttgtcgg tcattatgtc ttcaatcgtc aagggcaggt cggcataagt ggataaaggc 886320
aggatggcgg gcaaggcggg caaaaagatg cgcttcatat ttctcctatt gatattaatt 886380
cttatttact aataataatg aatatccgat aatcttgtct aaataaattt ttaaatcata 886440
aaaaacaata tgtttgtttt tgtcaattat gtttcgatat gccgtctgaa aagtttgtga 886500
aaaacggtta caatccgccg catgaaaaaa cgcaataatc ctcttccgct gttgaacggt 886560
gtcaaaccca gttatttggt gctgccgcat gaaaagcagt tttacgggct gccgctgctg 886620
cattttctgt gcatccgctt tccttttgtg ggcgcggacg attggcgcag gcggttgaac 886680

agcggttttg tggtcggttc ggatggtgcg gcgttggacg aacattcttt gttcgagccg 886740
ggtaaggtgg tgttttatta ccgtgaaacc agccgtgaga gcgagccgcg tattccgttt 886800
gaagaaaaga ttttgcatat tgatgagcat ttgattgtgg tggacaaacc gcattttctg 886860
cccgtcatcc ccagcggcag gtttttgcgg gaaaccctgc tcacgcgcct gcgtttgcgg 886920
cctgaattgc agcatttgaa tgttgaggac attacgccgc tgcaccgctt ggacaaggat 886980
acggcaggcg tgatgctgct gtcgcacaat cctgccacgc gcggagccta tcagacgatg 887040
tttcaaaaca aaacggtatg gaaaacgtat gaggcgcttg cgccgacaag gacggatttg 887100
ccgtatccgc tcgatgtggt ttcgcgtttg gtgaggggtg agaaattttt tacgacgcaa 887160
gaggcggaag gcgaaccaaa cgcacatacg acggtcgaac tgattgaaaa caggggggaa 887220
ttcagccttt accgccttac gccgcatacg ggcaagaaac accagttgcg cgtgcatatg 887280
atgggtttgg gtatgccgct gttgaatgac gcgctctatc ccgtgccgtc tgaagcgggc 887340
agcgaggatt atcggaaacc tttgaagcta ttggcaaaaa agattgcgtt tgcagatcct 887400
ttgtcgggtc gggaaagggt gttttgcagc ggattttgcc tataatgaga gggaacgcaa 887460
acccgtatcg gcggatgccg ccgatacggg ttttacatta tcgatgccgt ctgaaagggg 887520
ttttatccgt tcaggcatac gggcgcgaag cggaggatgg tgtcgcgcag gacgatgagt 887580
ttgccgtgaa agaaatgcgt ggaaccggcg atggtaatga cgggcaaatc ttgcggttcc 887640
gcccatttca gtgctttccc gatttcgacg acttcgtctt ccgcgccgtg tatcatcagc 887700
gttttggcaa cgttgggaac ggcggacggt tccggacggt cggtatagtg gcagactgcc 887760
gcgccgatga gcagcaataa atcgggtgtg cgcgcttgtg cggcaaatgt ggcgacataa 887820
ccgccgaagg agaagccgga taaggcaaat tcgggggctt cggggtgttg ggcgcgggca 887880
taatcgatga cggcgaggca gtcttgcgtt tcgccgcgtc cgtaatcatg tgtgcctccg 887940
ctgccgccta cgccgcgaag gttgggcagg tagcagtgga agccgagttt gcttaaggct 888000
ttggcggcag tttggatgac tttgttggtg tttgttccgc cctggagggg gttgggatga 888060
ttgatgacgg caacaccgcg tgccggtact tgttcggacg ggatatggat ggtttctaaa 888120
atgccggcag gaccggatat gtgtatggtt tcgggtttca gcataatgat ttcctcttga 888180
ttttccgcat gttccgatgc agccgtgaag atagttgctt aaaggtggaa atgcttgccg 888240
ttgttggggt tggacgcttt caggccggca agcatacggt ggaagtccaa tccgtatgcg 888300
ttgctcagtt tgtcggcacg tttttgagt ttgtctatgt tttgctcttt ggggctgctt 888360
tggaaggcca ttaccgcgac attgccgtgg ctttcggcag gaagttccaa gacgcgccct 888420
tcaaaaacgc tcaacaaccg ttcgatgaag cgttggtagc gtttgtcgcc gctccaccag 888480
ttggttacga atatgccgtc tgaagagagt gcgttgcggc agtctcggaa gaacggttct 888540
tcaaccagcg catcgatgat ttgttcgccg tcgaacccgt ccaccaaaat cacatcggtg 888600
ttgtggcgga agactttgat atattctgca ccgtctgctt caataatttc aaatttctcg 888660
ccctcgaaag gcaactcgaa caggttgcgg gcaatggcga tgacctgcgg attgatgtcc 888720
acggcggttt ggcgcgtgtc gggcaggtag gtatctatcc agcgtgcaaa cgagccgccg 888780
cccaagccga tttgggtgat atgttgcgga agagcgtcgg taaacagcag ccagcccatc 888840
atcgcgcggc tgtaagagag caccagctcg gacgggtggt cgaggttcat cgagctttga 888900
acggtgtcgc tgcccaagtg aagcgaacgg atattgcctt cttcggaaat gccgacttcg 888960
ggaaagccgg attttgcagg acgcaggcgg cggtagggat gtcttgccat cggtgtgaac 889020
ggtcggtttg aaaggcggat attttatcag aatgccgatg cttgttctgt ttcaaattaa 889080
tttcttttca aataaattac ttattcggat ttgccggggc tttcggataa attccttgcc 889140
aaggtgcggc attgcctgca taattcgctt tctttgccgg tatagctcag ttggtagagc 889200
acctgacttg taatcagggg gtcccgagtt cgactcttgg tgccggcacc atttctgctg 889260
atggtgttgc aaaacatttc agtaagccgg tatagctcag ttggtagagc acctgacttg 889320
taatcagggg gtcccgagtt cgactcttgg tgccggcacc agatttgaca gtccgattgt 889380
gtaaaacagt cgggctgttt tgcatttgcg gcacggtcgg tggcagggct gcgggcattt 889440
cactataata gcccgtttca aactgaaaag gccgtctgaa aagggcgggg taacaatatc 889500
tgatgattac tgtgaacaca ctgcaaaaaa tgaaggcggc gggcgaaaaa atcgctatgc 889560
tgaccgctta cgaatccagt tttgccgcgc tgatggacga tgccggcgtg gaaatgctgc 889620
tggtcggcga ttctttgggg atggcggttc aggggcggaa atcgacgctg cccgtcagcc 889680
tgcgcgatat gtgttatcac accgaatgtg tagcacgcgg tgcaaaaaat gcgatgattg 889740
tcagcgattt gccgtttggt gcatatcagc agagtaagga gcaggcgttt gccgccgccg 889800
ccgaactgat ggctgccggc gcgcatatgg ttaaactcga aggcggcgtg tggatggcgg 889860
aaacgactga atttttgcaa atgcgcggta ttccggtttg tgcgcacatc ggtctgaccc 889920
cgcaatccgt gtttgccttc ggcggatata aagttcaggg gcgcggcggc aaggcgcagg 889980
cgttgcttaa cgatgccaag gcgcatgacg atgcgggggc ggcggtcgtg ctgatggagt 890040
gcgtactggc ggaactggca aaaaaggtaa ctgaaactgt ttcctgtccg accatcggca 890100
tcggcgcggg tgcggattgc gacgggcagg ttttggtgat gcacgatatg ctcggcattt 890160
tcccgggtaa gacggcgaaa ttcgtcaaaa actttatgca ggggcatgat agtgttcaag 890220
cggcggttcg ggcgtatgtt gccgaagtca aagccaaaac cttccctgct gcggaacata 890280
tttttgcaga ttgagcggtt tacatgcagc atgccgtctg aaagccgttt cagacggcat 890340

tttgttttgc cgtgcgcggc gcgtataatc ggcgcgtttt gtcgggcagg aagcccgaag 890400
gataaggatt accgttatgc aaatcataca taccattcga gaactgcgcg cgtggcgtaa 890460
aaatgcggga aaggtggcat ttgtgccgac catgggcaat ctgcatgaag gacatcttgc 890520
gcttgtgcgt gaggcgaaaa aacgcgcgga cagtgtcgtg gtcagcattt tcgtcaatcg 890580
cctgcaattc ggtcagggcg aggatttcga caaatatccg cgcactttgc aacaggatgc 890640
ggacaaactt gccgccgaag gcattgccgt tgttttcgcg cccgatgaga aagaactcta 890700
tccgaacgtg gaacagcgtt acaacgtcga accgcccaat ctgcaaaatg agttgtgcgg 890760
caaattccgc ccggggcatt ttcgcggtgt ggcaacggtt gtttctaaat tgttccacat 890820
cgtttccccg gacattgcct gttttggtaa gaaggattac cagcagcttg ccgtgattaa 890880
aggttttgtc gaagatttga attttgatgt tgaaatagtg cctgttgata cagggcgcgc 890940
ggaagacggg ttggcactgt cgagccgcaa ccagtatttg agtgcggcgg aacgcgacga 891000
agcaccgcgc ctgtaccgcg aattaaaggc tgttgccgaa tccttggtgc agggcagttt 891060
ggattatgca ggtttggaaa aacgtgccgt ccaatccctg acagaatacg gctgggtggt 891120
cgattatgtc gaaatccgcc gcgccgatac gctcgaagtg gcgcgggcgg gagataagaa 891180
actggtggtc ttggccgccg cctgtctggg gacgacgcgc ctgattgaca atttggaaat 891240
aaaactccct taaaccgcaa gcgtcgggaa tgccgtctga agcggatttg cgtttcagac 891300
ggcatttatt ttttgaacgg ggtttcgcaa atctacaaac gattctgctt gtgataaagt 891360
tacgcctgat tatatgccgt ctgaaggttc ggacggctgt cggataaagg atgattatgt 891420
tacctaaccg tttcaaaatg ttaactgtgt tgacggcaac cttgattgcc ggacaggtat 891480
ctgccgccgg aggcggtgcg ggggatatga aacagccgaa ggaagtcgga aaggttttca 891540
gaaagcagca gcgttacagc gaggaagaaa tcaaaaacga acgcgcacgg cttgcggcag 891600
tgggcgagcg ggttaatcag atatttacgt tgctgggagg ggaaaccgcc ttgcaaaagg 891660
ggcaggcggg aacggctctg gcaacctata tgctgatgtt ggaacgcaca aaatcccccg 891720
aagtcgccga acgcgccttg gaaatggccg tgtcgctgaa cgcgtttgaa caggcggaaa 891780
tgatttatca gaaatggcgg cagattgagc ctataccggg taaggcgcaa aaacgggcgg 891840
ggtggctgcg gaacgtgctg agggaaagag gaaatcagca tctggacgga ctggaagaag 891900
tgctggctca ggcggacgaa ggacagaacc gcagggtgtt tttattgttg gcacaagccg 891960
ccgtgcaaca ggacgggttg gcgcaaaaag catcgaaagc ggttcgccgc gcggcgttga 892020
aatatgaaca tctgcccgaa gcggcggttg ccgatgtggt gttcagcgta cagggacgcg 892080
aaaaggaaaa ggcaatcgga gctttgcagc gtttggcgaa gctcgatacg gaaatattgc 892140
cccccacttt aatgacgttg cgtctgactg cacgcaaata tcccgaaata ctcgacggct 892200
ttttcgagca gacagacacc caaaaccttt cggccgtctg gcaggaaatg gaaattatga 892260
atctggtttc cctgcacagg ctggatgatg cctatgcgcg tttgaacgtg ctgttggaac 892320
gcaatccgaa tgcagacctg tatattcagg cagcgatatt ggcggcaaac cgaaaagaag 892380
gtgcttccgt tatcgacggc tacgccgaaa aggcatacgg caggggggacg gaggaacagc 892440
ggagcagggc ggcgctaacg gcggcgatga tgtatgccga ccgcagggat tacgccaaag 892500
tcaggcagtg gctgaaaaaa gtatccgcgc cggaatacct gttcgacaaa ggtgtgctgg 892560
cggctgcggc ggctgtcgag ttggacggcg gcagggcggc tttgcggcag atcggcaggg 892620
tgcggaaact tcccgaacag caggggcggt attttacggc agacaatttg tccaaaatac 892680
agatgctcgc cctgtcgaag ctgcccgata aacgggaggc tttgaggggg ttggacaaga 892740
ttatcgaaaa accgcctgcc ggcagtaata cagagttaca ggcagaggca ttggtacagc 892800
ggtcagttgt ttacgatcgg cttggcaagc ggaaaaaaat gatttcagat cttgaaaggg 892860
cgttcaggct tgcacccgat aacgctcaga ttatgaataa tctgggctac agcctgctga 892920
ccgattccaa acgtttggac gaaggtttcg ccctgcttca gacggcatac caaatcaacc 892980
cggacgatac cgctgtcaac gacagcatag gctgggcgta ttacctgaaa ggcgacgcgg 893040
aaagcgcgct gccgtatctg cggtattcgt ttgaaaacga ccccgagccc gaagttgccg 893100
cccatttggg cgaagtgttg tgggcattgg gcgaacgcga tcaggcggtt gacgtatgga 893160
cgcaggcggc acaccttacg ggagacaaga aaatatggcg ggaaacgctc aaacgtcacg 893220
gcatcgcatt gccccaacct tcccgaaaac ctcggaaata atgcaggtcc atcctttcag 893280
acggcataag gtttgccggg aagccggggc attcgggcaa acggcacgca gttcgcacgc 893340
gttttgcacg gcacgccgaa cccatcggcc ggcaggatgg catccgttaa ggaaattctg 893400
atgaaacaca ccgtatccgc atcggtcatc ctgcttttga ccgcttgcgc gcaattacct 893460
caaaataacg aaaacctgtg gcagccgtcc gaacacatca gcagttttgc agcagaaggg 893520
cggttggcag tgaaagcgga agggaaaggt tcgtatgcaa atttcgattg gacataccaa 893580
ccgcccgtgg aaaccatcaa tatcaatacc cctttgggca gtacgctcgg gcagttgtgt 893640
caagacaggg acggcgcatt ggcagtggac ggcaaaggaa atgtctatca ggcggaaagt 893700
gcggaagaat tgagcaggca gctggtcggt ttcaaactgc caatccaata tctgcatatc 893760
tgggcagatg gcaggcgtgt ggcgggcgcg ccttaccgca tcctgccgga cggcatattg 893820
gaacaatacg gttggactgt cggcagaacc gccgacagtg gggggcaagt ccgaacgttg 893880
caactgaata acggaaattt gaacatcagg ctggttttca ccgaaatcgg tatgccgtct 893940
gaaaccgaaa ccccggaacg ctgtgcggcg cgcacgagat aaggcggaca gatgaatatt 894000

gcggacggac ggcaggcgtt ttccgcacct gcaaaactga atctcgattt gaggattacc 894060
ggcaggcggg aagacggtta tcacaatatc gaaagcatat tctgcctgat agatttgcag 894120
gataccgtat atttgaaacc gagggacgac ggcaaaatca tcctgcacaa tccggttgat 894180
ggcatgccgc aggaagtaga tttgagctac cgtgccgcat cgttgctgca aaaatatgcg 894240
cgcaaccccg ccggcgtgga aatatggctg gacaaaaaaa tcccgacagg ggcgggtttg 894300
ggcggcggaa gctcggatgc ggcaaccgtt ttgctggtgt tgaaccgttg gtggcagtgc 894360
ggtctgacgc agcggcagct cattgattcg ggcgcggctc tgggggcgga cgtaccgttt 894420
tttattttcg gcaaaaatgc gtttgcgcgg ggtataggcg acaggctgga cgaaatggat 894480
attccgaaac agtggtatgt catcgtcaaa ccgcccgtcc acgtttccac tgcaaaaatt 894540
ttcacacacg aaagcttgac acgaaattcc gcctcaagca taatgccgac tttccaaaat 894600
ctgcaaccgt ttagaaatga tatgcaggca gttgtattta aagaataccc tgaagtttgg 894660
aaagcctatt ccgagttgtc ccgatatgga tttgccttaa tgacaggttc cggtgcgtgt 894720
gtattcacgg cgtgtcaaga taggaatagc gcatacaata tataccgaca agtttcagat 894780
ttgtacgagg catatttggc agagggtctt tcaaaacatc ctttgttgtc cgtataaaca 894840
ttgttgggga gtcgtcaagc ggttaagaca ctggattttg attccagcat gcgaaggttc 894900
gaatccttcc tccccagcca agtcaaacga gttggggagt cgtcaagcgg ttaagacact 894960
ggattttgat tccagcatgc gaaggttcga atccttcctc cccagccagc ccttttttggg 895020
gagtcgtcaa gcggttaaga cactggattt tgattccagc atgcgaaggt tcgaatcctt 895080
cctccccagc caaataaaag cgtgtaagcc tgcttacacg cttttatttc atagaaataa 895140
aaatattgaa atgcctttgt ttgtgtcgga tgttgcaggt ataatgtcgg gcttggtaca 895200
agcagaggga agcattgtgt tttctgagcg gaagttaaac ataaaatcag gtgagaatat 895260
ggctgcgtac gacagtttga tggtatttac aggcaatgcc aatcccgaat tggcacaacg 895320
tgttgtcagg catttggaca tttctttggg caatgcttcc gtatccaagt tttcagacgg 895380
cgaagttgcc gtcgaactgt tggaaaacgt acgcgggcgc gatgttttca tccttcagcc 895440
gacctgtgcg ccgaccaatg acaacctgat ggaaatcctg acgatggcgg atgcactgaa 895500
gcgtgcttcg gcaggtcgta ttaccacagc cattccgtat ttcggctatg cgcgccaaga 895560
ccgccgtccg cgttccgtcc gcgttccgat ttctgccaaa ctggtggcaa atatgctgta 895620
ttcggcaggg atcgaccgtg ttttgactgt cgatttgcat gccgaccaga ttcaaggttt 895680
cttcgatatt ccggtggaca atatttatgc cacccccgatt ctgttgaacg acatcaaaca 895740
acagcggatt gaaaatctga ccgtcgtcag cccggacatc ggcggtgtcg tccgcgcccg 895800
cgccgtggca aaatccctga atgccgactt ggcaatcatc gacaaacgcc gcccgaaagc 895860
caatgtggcg gaagtcatga acatcatcgg cgatattcaa ggtagaacct gtctgattgt 895920
ggacgatatg attgacactg caaatacgct gtgcaaagcc gccgtcgccc tgaaagagcg 895980
gggggctgaa cgtgttctag cctatgccag ccacgccgta ttctccggag aggcggtcag 896040
ccgtatcgcc tcatccgaaa tcgaccaggt ggtcgtaacc gataccattc ctttgtctga 896100
agcggctaaa aactgcgacc gtatccgtca ggtaacgatt gccggtctgt tggccgaaac 896160
cgtccgccgc attagcaatg aagaatccgt ctcatatctt ttcaatgaag aagtgatgac 896220
aggcagcatg ttgctgccat aagcccgaag ccgtcttaag ctggtcgcgg ccgatgacgg 896280
cgattttacc taacttggag tatttaacat gacttatgaa attcaagcct ctgttcgtga 896340
agcacaaggc actggtgcga gccgccgcct gcgtcgcgaa ggccaaatcc ccggcattct 896400
gtacggtgaa ggtcaagagc ctgttgcaat cgctgtggat cacaaaaccg tattctacgc 896460
attggaaaaa gaatctttcc atactgcgtt gattaagttg tctctgaacg gtgaaaccaa 896520
agacgttatc gtccgtgatt tccaaatgca cccgttccgc cgcgaagttc aacacatcga 896580
cttccaagct gtgaaagccg atcaacttgt acgcatccgt gttcccctgc acatcgttaa 896640
cgctgaaaat tcccaagcgg tcaaactgca aggcggccgc gtatctctgt taaacacttc 896700
tgttgaagta gttgctttgc ctgccaacat ccctgctttc ttggatttgg attgtgctga 896760
agtggttgcc ggcgacattc tgcacttgtc agacatcaaa ctgcctgaag gtgtagaaag 896820
cgtttccctg aaacgtaacg aaaatctggc tgttgctacc gttaccggta agaaacgcta 896880
attgatttca gcagcagggc ggcgcgtatg caatacgtac cgccctgttg ttttatgccg 896940
tctgaaccgt gtttcagacg gcatttcttt atttgttgga aaaacgggat atttgaaacg 897000
gcagattact gccctgtcag acacgcccaa agcctttgcc accggcttct ttttttttaca 897060
ttttccagtg cgacgatttc tttttcggca atggtgtatc cgttttgtct gattttgatt 897120
tttcctaaaa tttgcccttt ttttactggg gcgggaatcg gctgtatggt ttctagaatt 897180
tgttctgcca ttttcgcttc cttatgtggc agagtgatgt aggcttcttt gaggaagcct 897240
gcgcggacgg ttttttttgct gcctccggaa atttggattt gggcaacggt tttgcctttc 897300
ggatatattt tgggcgtatc gaaggcctgc aatgcccagt tcagcagctt gctgttgtct 897360
gatgcgcgtg tttccgccga ttccgaaccc aatgtgatga caaggatgtg cctgccgttg 897420
ccggagtatg acacggcaag gttgtagccg ccgctttctg tgtgtccggc tttcagaccg 897480
tttacattgt tgtccctata taaaaggata ttgcggttgt tttgttctat atttttgaat 897540
ttgaaagatt tgatggaaaa cagcgggtaa tattccggaa agtcgcgcat caatgcttca 897600
gacagcaggg cgaggtcttt ggcggtggaa acctgtcctt ctctactcaa gcctgtcggg 897660

tttttgaata cagtgttctt catgcccaag cgtcgggctt ctttgttcat ttgttgcaca 897720
aaattttcaa tcgagccgtt gcccagccgg ccggcaaggg ttagggcggc atcgtttgcg 897780
gatagtgcaa tcatgccttt taagagtttg tcggtgctga ccgtatcgcc gggacgtaca 897840
aacattctgc ttccttctga agcccatgcg gattcgggta tttttaagtt ttcttcagat 897900
tggatattgc ccgatttcat gtttttgaaa accagatatg cggtcatcag ttgggttagt 897960
gccgccggtt caacaggggt attgatgttt ttggcggata aaatctgttt gctttgaagg 898020
tcgataacga tgtgtgccgc tgtgagggtt tcgggtgttt ggaacgtggg ggcggcgtgt 898080
accgtcggtc tgttgggcgc gggcgatgca gccgttgcgt gagaaacgcc taagatgatg 898140
gaaagcagga cgggcaggat tttatgtgct gtcatgaaat attctaattg tgtgcgtgtt 898200
tcagtctgcc gattatacgc ttagggtgtc tgatcgggcg gatttttctt gatttcgcgc 898260
cgtcttgggc gtatggtttt gggttttgcg attttaataa accgattatc ccatattgaa 898320
ttatgaacac gccccttcct tattccgatt acctcatccg catcctgacg gcatctgtct 898380
atgatgtggc ggtcgaaacg cctttggaac cggcacgcag cctttctgta cgtttgaaaa 898440
acaacatcct tttgaaacgc gaagatttgc agccggtttt ttcgttcaaa atacgcggcg 898500
cgtacaacaa aatgtccaag ttgccgaaag atgcgctcgc ttgcggcgtg attgcggcaa 898560
gcgcgggcaa tcatgctcaa ggcgtggcat tgtccgcaca gcgtttgggc tgccgtgccg 898620
ttatcgttat gccggagact acgccgaaaa tcaaagtgga tgcggttaaa agccatggcg 898680
gcgaggtggt tttgcggggc gtttcataca acgatgccta cgattatgcg atggagttgg 898740
cggaaaaaga agggttaacc tatatcgcgc cgtttgatga tcctgatgtg attgcgggac 898800
aggggacggt ggggatggaa attgtcagcc agcatcccga tccaatccgc gccgtattcg 898860
taccgatagg cggtggcggt ttggcggcgg gcgtggcggc atttatcaag caggtccgtc 898920
ccgaaatcaa agttatcggc gttcagacca acgattcctg ctgtatgaag cagtcggtcg 898980
aagcgggtga aatcgtccat ttgaaagatg tcgggctgtt ttcagacggc actgcggtca 899040
aagtcgtcgg aaacgaaacc ttccgcctct gcaaagaact tttggatgaa atcattacag 899100
tcgataccga tgcggtttgc ggcgcggtca aggatatttt cgatgacacg cgcagcatta 899160
ccgagccggc gggcgcgttg gcgttggcgg gtctgaaagc ctatatcgcc cgagaaggcg 899220
cggaaaacca aaccctgatt gccgttacca gcggtgcgaa tatgaatttt caccgtttgc 899280
gccacgtttc ggaacggagc gaattgggcg agggcaacga aggtattttt gccgttacca 899340
tccctgaaga acgcggcagc ttccttaagt ttgtcaatat attgggaaat aggaatatta 899400
ccgagttcaa ctaccgctac ggagacgatg aaaaagcgca tatctttgtc ggacttcaag 899460
cggcaggccc gcaggatttg gcggttatcg gcagccggtt ggatgaggcg ggattgccca 899520
atgtcgattt gaccaacaat gagattgcca aaatccatat ccgctatatg gtcggagggc 899580
ggacggacaa agtagaaaac gagcgtttgg tcagttttga gtttccggag cgtccggggg 899640
cattggcacg ctttttgaac catatgcagg gagggtggaa tattacgctt ttccattacc 899700
gcaaccacgg tgcggattac gggcggattt tggtcggtat cgatgtgccg ccgcacgatg 899760
ccgccgcatt tgacggtttc ttggaaagtc tgggatacag ctatcacgag gaaacgcaaa 899820
atgccgcgta caagctgttt cttgcctgac gcttgaaagc acaatgccgt ctgaaagcct 899880
ttcagacggc attgcgcttt catggttaaa tcgaatattc aatcagttcg ttttgagaga 899940
agacataaac ctgtttcgga atcagcttca attctttgcc ttcggcgatt gggtaacgcg 900000
cggcatcgct gcctgccagc gtgatatgta cgtcctgttt gtcgtgtttt accagaatat 900060
gcgtcaatgc gccgacggcg tggatttttt cgatttcggc acaaatcatc ggtgtttcgt 900120
gttcggcggc gatctgccat tcgtgcgggc ggatatagcc ggtggcggtt tgttcctgcc 900180
atttgtattg cgcgtccaat ttccacgcga agccgttgta atgccagaag cctttttcga 900240
tgcgtccttc aaaagcgtcg gtttcgccga ggaactcggt aacgaaggca ttttcgggtt 900300
tgcggtaaat agcttcggcg ctgccggttt gttcgatttt gccgtggttc atcacgacga 900360
tttcgtcgga aacttcgagg gcttcttctt ggtcgtgcgt aaccagaatg ctggttacac 900420
ccaggttgtg atggatgtcg cgcagccagg tgcgtaattc tttgcgtact ttggcatcca 900480
acgcgccgaa gggttcgtcc aaaagcaaga gtttgggttc gaccgcaagc gcgcgggcga 900540
gggcgatgcg ctggcgttgc ccgccggaga gttggtgcgg ataggatttt gccaaatgag 900600
agagctgcac gagcttgagt aattcttcga ctttggcgcg gatttgtcct ttggacgggc 900660
gttcggactt gggcaatacg gtcaaaccga aagcgacgtt gtcaaacacg ttcatatggc 900720
ggaaaagggc gtagtgttgg aacacgaagc cgactttgcg ctcgcgcaca tgtttggcgg 900780
ttacgtcttg cccgtcaaac aggatattgc cgccgtcggc gttttccagt ccggcgataa 900840
tgcgtaaaag tgtggttttg ccgcagccgg acgggccgag cagggaaacg agtttgccgg 900900
tggggacgtt gaggttgatg tttttcagcg cgtgaaaatt gccgaagtgt ttgtttaagt 900960
tttggatggt gatactcata ttgcattcct ttcggcggcg gcgagttttt tgtcttgtaa 901020
tttggtaatg atgttctgca ccgccagcgt cgccagtgcc aaaagtgcca atacgccgga 901080
gagggcgaat gcgccggtga agttgtattc gttgtagaag atttcgacca aaagcgggac 901140
ggtgttggtt tcgccgcgta tgtgtcccga taccacgctg accgcgccga actcgcccat 901200
cgcgcgggcg ttggtgagga tgatgccgta gagtaacgcc catttgatgt tgggcagggt 901260
aacgcgccaa aacatctgcc agccgcttgc gccgagtatc aatgccgcct gttcttcgct 901320

gtcgccctgt gcctgcatca gcgggatgat ttcgcgtgcg acaaagggga aggtaacgaa 901380
cagcgtcgcc aaaacaatac cggggatggc gaagataatc tgtatgcctt gcgcttcgag 901440
ccagccaccc aatgccgtat gcgcgccgaa caataagacg aacatcaaac cggccaccac 901500
gggcgatacg gaaaacggca aatcgagcag ggtggtcagc aactgcttgc cgcgaaaatc 901560
aaaacgggtc agcagccacg ccatcgccac acccaatacg gcattgacgg gaacgacaat 901620
cagcgcggta atcagcgtca atttgatggc agaccacgct tcgggatcgt ttaaggattt 901680
caggtacaaa tcccaaccgc cttttaaggc ttcgtaaaac acggcgacga gcggcacgac 901740
cagcatcagc agcagaaagc ccagcgcggc ggcaatcagc aacacgcgca gccggcgcgg 901800
ttcggtcagg ttgggattgg cggaataggg tttcatggcg gtggtgttct ctggttggtt 901860
ttaaaatgtt tggatgccgt ctgaaaaacc gtttttgtg gggcggattc gttttcagac 901920
aacctttat tgattaaggg aataaaaacg tctagctacc gtcattcccg cgcaggcggg 901980
aatccaccgc agggcaacag gaaaacagaa aataaataag gcagccgaaa ttcaccaatg 902040
gattcccgcc tgcgcgggaa tgacggtaac aggtatttca gacgacctca acccttcgcg 902100
cccgaacgcc tgcccaacgc ccactgcatc acgttcagcg caaacagaat cacaaacgaa 902160
accagcagca taaacaacgc caccgccgac gcgccctgca cgtcgaactg ttccagcttg 902220
cccgtaataa tcagcggcag gatttcagaa accatcggaa tgttgcccgc gataaaaatc 902280
accgaaccgt attcccccgt tgcccgcgca aacatcattc ccgcgccggt caagagtgcc 902340
ggtgtgattt caggcaagag gacacggcga aacgtagtcc aacggcttgc gcccaaagtt 902400
gccgccgctt cctcatattc gcccgacaat tcttccaata ccggctgcac ggcgcggacg 902460
ataaagggca ggctgacgac gaccagcgca atccaaatgc cgacgggtgt aaacgcgatt 902520
ttgatgccca aaggctcgaa aaaacggcct atccaaccgt tgggcgcata cagggttgcc 902580
aacgcgatac ccgtaaccgc cgtcggcagc gcaaacggca aatcgaccag cgcgttcgcc 902640
agacccttgc ccgggaattc ataacgcacc aatacccacg ccaccagcgt gccgaacacg 902700
acattggtca gcatcgcata aaacgacatc cgcaagctca gccataccgc cgccaacacg 902760
ttcggctcgg caatcgtgtt ccaaaagccg ccccagccga tttccgccgc cttcgccgcc 902820
atcatcgcaa acggcaagac cacaagcagc gacaggcaca atacggtcag accaaggctg 902880
agtttgaagc cgggcagtac gccgggcgtt ttgagcgcta acataaaaca atgctgaaaa 902940
taaggaaaag gaaggactac tttaacgatg ccgtccgaaa aacggaaaga atggaaagtt 903000
tggtgcaaag acgaatttgt tataaagcgg ttggcagttt ctcaagcggg cgcgatgttt 903060
taaaatatag tggattaact ttaaatcagg acaaggcgac gaagccgcag acagtacaaa 903120
tagtacggca aggcgaggca acgccgtact ggtttaaatt taatccacta taacaccttg 903180
ttttgacgga aaaccatcat ataaaggaac acttatgcag attttatctt ttcaaccgga 903240
cattgcggaa cgtatgctgg aaggtacgga aggcgagtcg gtcaacgaaa acgcacaatt 903300
cgtccgtacg gacaacggct attggattgc gtggcatgaa ggcgtagcgg cactgcttgc 903360
gcccgatatg ccgccgggca ttccctgttt ttgggtggaa ggcgcggaaa gccttgaaga 903420
gttgtgcgtc atggtggaac gcggcgagtt tgacgaagtg gaagagtttg acggcgatga 903480
cgacgaatgg ctcgaaacgg cacagggttg cgggcaccac ggcgacgctt gcgcctgcgg 903540
acattaaagg cattgcaggc ttgccgcaag gggggcaagg ctttgccgtt ttttaaataa 903600
tcggtggatt gcatctgttt gcaatgggct gaacgcttcc ctttgtattt tattgaataa 903660
aaaaacttat cttggtatta taattaaggc agcatcaatt attttgggat ggcaataaac 903720
gcaaagcatt gatttgcgcc gattgcagac ttattatagc aggttgcggc gcggacttaa 903780
tgggaattgt ttatagagca tgatggtctg aaacatcatt aattaaaaat accattaatc 903840
cgatttatat ttatttcaat ttcaatggaa aaacatcaat gacaatgatt ttaagcattt 903900
taagcctgtt ttttatcatc agactgttat ttttagccgt ctctattaaa catgaaaaag 903960
ccttgattgc caaaggggcg aaacaatacg gaaaaaccaa ttccacgctg cttgcggcag 904020
ttcatacgct ttattatttg gcgtgttttg tttgggtatg gctttctgac actgctttta 904080
atggcatatc cttgattggt acgctgacgg tgatggcttc gtttgtgata ttgtcattga 904140
ttattaagca gttgggggag atttggacgg ttaaaatcta tattttacca aatcatcaaa 904200
ttaatcgttc gtggttgttt aaaacattcc gccaccccaa ttattttta aacatcatac 904260
ccgaactgat tggcatcgcc ttattatgtc aagcgtggta tgttttattg attggcctgc 904320
ccatttattt gctggtctta tttaagcgta tccgacaaga agaacaggcg atggcaacac 904380
ttttttaacc cgtttcatca attatagcgg attaacaaaa accagtacgg cgttgcctcg 904440
ccttgccgta ctggtttttg ttaatccgct atattccgcc atctctaaga tttacagcga 904500
tacacgggta atttaaggaa tgcccgaacc gtcattcccg ccactttccg tcattcccgc 904560
aaaagcggga atctaggacg cagggttaag aaaacctaca tcccgtcatt cccgccactt 904620
tccgtcattc ccgcgaaagc gggaatctag aatctcggac tttcagataa tctttgaata 904680
ttgctgttgt tctaaggtct agattcccgc ctgcgcggga atgacgattc ataagtttcc 904740
cgaaattcca acataatcga aacctgacag taaccgtagc aactgaaccg tcattcccac 904800
gaaagtggga atctagaaat aaaaagcaac aggcatttat cggaaataac tgaaattcaa 904860
taccgcaaaa atctacccga aatgatatag cggattaaca aaaatcagga caaggcggca 904920
aaacgtttgg cgacttcgtc ccagttgacg atttcccaaa aacctttcag gtagttggga 904980

```
cggctgttgc ggtagtcgat gtaataggcg tgttcccaca cgtcgcaggt cagcagcggc 905040
gtgttttcag tggtcagcgg cgtagcggcg ttggaagtag aaaccaaatc caatccgccg 905100
gcaggggttt ttaccagcca cgcccaaccg gagccgaaag taccggccgc gcaggcattg 905160
aacgcttctt ggaatttctc gaagctgccc catttcgcgt cgatggcggc ggccagttcg 905220
ccggcaggtt tgccttgacc tttggacgtg aaacccagcc agtagaaggt gtggttccaa 905280
gtttgtgccg cgttgttgaa cacgccgcct gaagattttt tcacaatctc ttccaaaggc 905340
aggttttcaa attcggtgcc tttgatttga ttgttcaggt tggtgatgta ggtttgatgg 905400
tgtttgccgt agtggaactc caaagtctct ttgctcagat gcggggacaa tgcgtccagt 905460
tcataaggca gttgcggcag cttatgttcc attttgtact cctgaatatt gttttaaatg 905520
ttgtattttg gcagtgttgc tgcaaataac tcggcagccc gtgtattcta cctgttttgc 905580
ggtgcggaaa ccaattaaac ctgctttacg ctataataga agattgcaat ttcggcacga 905640
cagataggat gtaccatgaa cgattacgca gccatgccgt ctgaagaccg tgaggtcggc 905700
gcattgtctc tgccccccaca ctcaatggag gcggaacaat ccgttttggg tgggttgatg 905760
ctggaaaatc cggcatggga caggattgcc gatgtggttt ccggtgaaga cttctaccgg 905820
catgaacacc gcctgatttt ccgatccatt gccaaattga ttaatgagag ccgtcccgcc 905880
gatgtgatta cggttcagga agatttgcag cggaacgaag aattggaagc ggcaggcgga 905940
ttcgaatatc tgattacgct ggcgcaaaac accccgtctg ccgccaacat ccgccgctac 906000
gccgaaatcg tgcgcgagcg ttccattatg cgccaactcg ccgaagtggg gacggaaatc 906060
gcccgcagcg catacaatcc gcaaggcagg gacgcggggc agcttttgga cgaggcggaa 906120
aacaaagtat tccaaatcgc cgaaagcacc gccaaatcca agcagggctt tttggagatg 906180
cccgatttgc tgaaagaagt cgtacagcgc atcgatatgc tctactcgcg cgacaatccc 906240
gatgaagtta ccggcgtgcc gacggggttc atcgacctcg acaaaaaaac ctcgggtctg 906300
caacccggcg acctgattat cgttgccggt cgtccgtcta tgggtaagac cgccttttct 906360
atcaatatcg ccgaacacgt tgccgtagaa ggcaggctgc ccgttgctgt tttctcgatg 906420
gaaatgggcg gggcgcaact ggtcatgcgt atgctcggct cggtcggacg gttggatcaa 906480
agcgttttga aaaccggcag gctcgaagac gaacactggg gtcgcctgaa cgaagcagtc 906540
gtcaaactct ccgacgcgcc cgtgtacatc gacgagaccc cgggtctgac cgcgctcgaa 906600
ctgcgcgccc gtgcccgccg tctcgcccgt caatttaaca ataagcttgg attgattgtc 906660
atcgactacc tgcaactgat ggcaggatcc ggccgttccg acaaccgagc ttcggagctg 906720
ggagagattt cacgttcgct caaagcgttg gcgaaagaat tgcaagtccc catcatcgcc 906780
ctgtcgcaat tgagccgcac ggtcgaatcg cgtaccgaca aacgccccat gatgtccgac 906840
cttcgcgagt ccggcgcaat cgagcaggat gccgacctga ttatgttcat gtaccgcgac 906900
gaatactaca accaggactc acccatgaaa ggccttgccg aatgtatcat cggcaaacac 906960
cgcaacggtc ccgtcggtaa aatcttcctc acatggacgg gacaattcac caaattcgac 907020
aatgctgcct atattcccga ggaggcaaag atagaggatt aaatggctat ataaaaattt 907080
attaggcgaa atcaggcaaa atcgtttaaa atcatgctga gagattgccc taaaaaataa 907140
aacgcggtct tgaggcattt ttgcattcag cccgcatata attgaaaata tagtggatta 907200
acaaaaatca ggacaaggca acgaagccgc agaccgtaca aatagtacgg aaccgattca 907260
cttggtgctt gagcacctta gagaatcgtt ctctttgagc taaggcgagg caacgccgta 907320
ctggtttgaa tttaatccac gatacattac cagttaacgt tctattgctt atgtgtacac 907380
gaaaacaaca aggtttcacg ctaacagagc tgctcatcgt gatggtcatt gcagccatta 907440
tggcgatgat agccctcccc aatatgagcc aatggattgc atcccgccgc attgccagtc 907500
acgcggagcg gattgccaac cttttgcgtt tctccagggg cgaagccgtc cggctcaatc 907560
tccctgtcta tatctgtcct gttcaagtta aaaaagacgg tacgcccaac aataaatgtg 907620
actccggcaa gaagggggcag ggaatgttgg ctttcggcga caaaaacggc aataagggat 907680
atgacaatga tacggaggat gttcttctcc gcagtgtggt attgaatgat gatatcaatg 907740
ataagcggat taattatgcc ttcaaccata tcgctttcgg tcagactcag ccgaccaccg 907800
accgtgtagt ttggacattc aatcaaaacg ggacgttcgg ttatacgaaa gaccagcatc 907860
ttacaaaaca atccagcttt ttttattctg acggttatat ccaaatcgtg ttgacagatg 907920
cgaaggcggt ttctgccgat gaaaagaaat tccgttcggc ggtggttttg attaacagca 907980
gcggcagggt cgaagtttgc cctaggggtg atcggcgtac tatgtgccag tataaataac 908040
agtttcagtt ttaaaaatga atatgaagaa taatgattgc ttccgcctga aagattccca 908100
gtccggtatg gcgctgatag aagtcttggt tgctatgctc gttctgacca tcggtatttt 908160
ggcactattg tctgtacagt tgcggacagt cgcttccgtc agggaggcgg agacacaaac 908220
catcgtcagc caaatcacgc aaaacctgat ggagggaatg ttgatgaatc cgaccattga 908280
ttcggacagc aacaagaaaa actataatct ttacatggga aaccatacac tatcagctgt 908340
ggatggcgat tttgcgattg atgccatgaa aactaagggg caattggcag aggcacaatt 908400
gaagagattt agttatgagc tgaaaaatgc cttgccggat gcggcagcca tccattacgc 908460
cgtctgcaag gattcgtcgg gtaacgcgcc gacattgtcc ggcaatgctt tttcttcaaa 908520
ttgcgacaat aaggcaaacg gggatacttt aattaaagta ttgtgggtaa atgattcggc 908580
aggggattcg gatatttccc gtacgaatct tgaggtgagc ggcgacaata tcgtatatac 908640
```

ttatcaggca agggtcggag gtcgggaatg agacgtaaaa tgctaaacgt accaaaaggc 908700
agttatgatg gtatgaaagg ttttaccatt attgaatttt tggttgcggg cctgctcagt 908760
atgattgtcc tgatggcggt cggatcgagt tacttcacat cccggaaatt aaatgatgcg 908820
gcaaacgagc gtcttgccgc gcaacaggat ttgcggaatg cggcaacatt gattgtccgc 908880
gatgcgagaa tggcaggcgg cttcggttgt ttcaatatgt ccgagcatcc tgcaactgat 908940
gttattcccg atacgacgca acaaaattct cctttttcct taaaaaggaa cggtatagat 909000
aaacttattc ccatagcgga atcttcaaat atcaattatc agaatttttt ccaggttggt 909060
agcgcattga tttttcaata cggaatcgat gatgttaatg caagcaccgc gactaccgtc 909120
gtcagcagct gtgccgcaat atcgaaaccg ggcaagcaaa tccctacttt agaagatgca 909180
aaaaaagaat tgaagattcc ggatcaggat aaggagcaaa atggcaatat agcgcgtcaa 909240
aggcatgtgg tcaatgccta tgcggtcggc aggattgccg atgaggaagg tttgttccgc 909300
ttccaattgg atgataaggg caagtggggt aatcctcagt tgctcgtgaa aaaggttaga 909360
catatgaaag tgcggtatat ctatgtttcc ggctgtcctg aagatgacga tgccggcaaa 909420
gaggaaacat tcaaatatac ggataaattc gacagcgccc aaaatgctgt tacgcccgcc 909480
ggggtggagg ttttattgag tagcggtact gataccaaga ttgccgcttc ttcagacaat 909540
catatttatg cttaccgtat cgatgcgaca atacgcgggg gaaatgtatg cgcaaacaga 909600
acactttgac gggaatcccg acttctgacg gacagagggg gtttgcactg tttatcgtgc 909660
tgatggtgat gatcgtcgtg gcttttttgg ttgtaactgc cgcgcagtct tacaataccg 909720
agcagcggat cagtgccaac gaatcagaca ggaaattggc tttgtctttg gccgaggcgg 909780
ctttgcggga aggcgaactt caggttttgg atttggaata tgatacggac agtaaggtta 909840
catttagcga aaactgtgga aaaggtctgt gtgccgcagt gaatgtgcgg acaaataatg 909900
ataatgaaga ggcttttgac aatatcgtgg tgcaaggcaa gcccaccgtt gaggcggtga 909960
agcgttcttg ccctgcaaat tctaccgacc tgtgcattga caagaaaggg atggaatata 910020
agaaaggcac gagaagcgtc agcaaaatgc cacgttatat tatcgaatat ttgggcgtga 910080
agaacggaga aaatgtttat cgggttactg ccaaggcttg gggtaagaat gccaataccg 910140
tggtcgtcct tcaatcttat gtaagcaata atgatgagta ataaaatgga acaaaaaggg 910200
tttacattga ttgagatgat gatagtcgtc gcgatactcg gcattatcag cgtcattgcc 910260
ataccttctt atcaaagtta tattgaaaaa ggctatcagt cccagcttta tacggagatg 910320
gtcggtatca acaatatttc caaacagttt attttgaaaa atcccctgga cgataatcag 910380
accatcgaga acaaactgga aatatttgtc tcaggctata agatgaatcc gaaaattgcc 910440
aaaaaatata gtgtttcggt aaagtttgtc gataaggaaa aatcaagggc atacaggttg 910500
gtcggcgttc cgaaggcggg gacgggttat actttgtcgg tatggatgaa cagcgtgggc 910560
gacggataca aatgccgtga tgccgcttct gcccaagccc atttggagac cttgtcctca 910620
gatgtcggct gtgaagcctt ctctaatcgt aaaaaataag gttgttttgc caataccgtc 910680
tgaaaatcaa tgttcagacg gtatttttat gggtatagtg gattaacaaa aatcgggaca 910740
aggcgacgaa gccgcagaca gtacagatag tacggaaccg attcacttgg tgcttcagca 910800
ccttagagaa tcgttctctt tgagctaagg cgaggcaacg ccgtactggt ttttgttaat 910860
ccactataca tcccgtcatt cccacgaaag tgggaatcta gaaatttaat gttgcggcac 910920
tagccaaaaa aaccgaaacc gacaggtcta gattcccgcc tgcgcgggaa tgacgaatcc 910980
atccgtacgg aaacctgcac cacgtcattc ccacgaacct gcatcccgtc attcccacga 911040
aagtgggaat ctagtttttt gagtttcagt catttccgat aaattgcctt agcattgaat 911100
gtctagattc ccgcctgcgc gggaatgacg aacctatccg tacggaaacc tgcatcccgt 911160
cattcccacg aaagtgggaa tctagttttt tgagtttcag tcatttccga taaattgcct 911220
tagcattgaa tgtctagatt cccgcctgcg cgggaatgac ggattttagg ttggggtcat 911280
ttattgggaa aagcagaaac cgctccgccg tcattcccac gaaagtggga atctagaaat 911340
ttaatgttgc ggcactagcc aaaaaaaccg aaaccgaacg gactagattc ccgcctgcgc 911400
gggaatgacg aatccatcca tacggaaacc tgcatcacgt cattcccacg aacctgcatc 911460
ccgtcattcc cacgaaagtg ggaatctagt tttttgagtt tcagtcattt ccgataaatt 911520
gccttagcat tgaatgtcta gattcccgcc tgcgcgggaa tgacggattt taggttgggg 911580
tcatttattg ggaaaagcag aaaccgctcc gccgtcattc ccaggaaagt gggaatctag 911640
aaatttaatg ttgcggcact agccaaaaaa accgaaaccg aacggactag attcccgcct 911700
gcgcgggaat gacggatttt aggttggggt catttattgg aaaaagcaga aaccgctccg 911760
ccgtcattcc cacgaaagtg ggaatccagt tttttgagtt tcagtcattc ccgataaatt 911820
gccttagcat tgaatgtcta gattcccgcc tgcgcgggaa tgacgaacct atccgtacgg 911880
aaacctgcac cgcgtcattc ccacgaaagt gggaatccag ttttttgagt ttcagtcatt 911940
ttcaataaat tgccttagta ttgaatgtct agattcccgc ctgcgcggga atgacgaatc 912000
catccatacg gaaacctgca ccacgtcatt cccacgaaag tgggaatcca gtttttttgag 912060
tttcagtcat tttcaataaa ttgccttagc attgaatgtc tagattcccg cctgcgcggg 912120
aatgacggat tttagtttgg ggggcattta ttggaaaaag cagaaaccgc tccgccgtca 912180
ttcccacgaa agtgggaatc tagtttttttg agtttcagtc attcccgata aattgcctta 912240
gcattgaatg tctagattcc cgcctgcgcg ggaatgacga ttcatatagt ggattaacaa 912300

aaatcaggac aaggcggcga agctgcagac agtacagata gtacggaatc gattcacttg 912360
gtgcttcagc accttagaga atcgttttct ttgagctaag gcgaggcaac gctgtactgg 912420
tttttgttaa tccactataa aaaggcatat tgaatgcggg caaaccggct gctttccgtt 912480
tttggatttc ggagaatgcc atcgcccagc tttcatcaca cataaaaaac agtgcgggca 912540
cggctttttt cagcggtatt cctttcaggt gcggggcaag cgccgccccc atcaggatat 912600
gccgagaatt aatcataaag gttacggtgg cgataagcag tatcggcaga ggttccgccc 912660
acaggttgac cgtggcaaac tcggagccgc cggcgaagtt catactggtc atcaacaaca 912720
tttccagcca gctcatgcct ttttgtccgc cctgcatacc gagtattaat gcccaaggca 912780
gcagcccaat cagcataggc gaactttctt tgatgccgcg tataaattcg ttatgcgggg 912840
aaggtgtgca taatgttcgt cttcataacc ggaagggcgg gaattataca ctggcaacgg 912900
atttcaaaac aaaaccgatt tgccgtgttt cagcgtaaac acggcttgtg tataatctcc 912960
catctttgaa accggccgta tgcaggagca agacgatgaa tattgaagta gaaatgaaag 913020
tattggacga acggatggcg gatgttgtcc ctgtctatgc aacggagggt tctgcaggtt 913080
tagatttgcg cgcctgtttg gatgaggaag tcgttttgca gccgggtgaa acgtttcttg 913140
tgccgacggg tttggcaatt tatttggcga atcccgcata tgccgccgtt ttgctgcccc 913200
gttccggctt ggggcataaa cacggcattg ttttgggcaa tttggtcggt ttgattgact 913260
ccgattatca aggggaattg aaggtgtcgt tatggaacag aagcagcgaa ccgtttactg 913320
tcaaaccgtt tgagcgtatc gcgcagatgg ttgtcgtgcc aatcgtgcag gcgggcttca 913380
aacgtgtcga ggagtttgtc ggaagcagcc ggggtgaggg cggcttcggc agtacgggtt 913440
ctcactaaaa atatagaatg ccgtctgaaa gacacgtcag gttcagacgg catatcttcc 913500
gatataccga caaccggaga aaatcatgaa taccttactc aaccaactca aaccatatcc 913560
ctttgcccga ctgcgtgaag cgatgcaggg catttccgcg cccgaaggtc tggaagccgt 913620
ccccctgcac attggcgaac cgaaacatcc gacaccgaaa gtcattacgg atgcgctgac 913680
cgcctcattg cacgagttgg aaaaatatcc gctgacggcc ggtctgcctg aactgcgtca 913740
ggcgtgtgca aactggttaa aacgccgtta cgatggcttg acagtggatg cggataatga 913800
aattctgccg gttttaggca gtagggaggc gttgttttct tttgttcaaa ccgtgttgaa 913860
ccctgtttca gacggcatca aacccgcaat tgtcagcccg aatccctttt atcagattta 913920
cgaaggtgcg acacttttgg gcggcggtga aatccatttt gccaattgcc ccgcgccgtc 913980
tttcaacccc gattggcgca gtatttccga agaggtttgg aaacgcacca aactggtgtt 914040
cgtctgctcg cccaacaacc ccagcggcag cgtgctggat ttggacggct ggaaagaagt 914100
ttttgattta caggataaat atggtttcat tattgcctcg gatgaatgct attccgaaat 914160
ctatttcgac ggcaacaaac ctttgggctg cctgcaagcc gctgcacagt tgggtcgaag 914220
caggcaaaaa ctgcttatgt tcaccagttt gtccaagcgt tccaacgttc cgggcctgcg 914280
ttccggtttt gtcgccggcg atgccgaact gcttaaaaac tttctgcttt acagaaccta 914340
tcacggcagt gcaatgagta ttcccgtgca gcgcgcaagc attgccgctt gggatgatga 914400
acagcacgtt atcgacaacc gccgtatgta tcaggaaaaa tttgagcgcg ttattcccat 914460
tttgcaacag gtatttgacg ttaaattacc ggatgcctcg ttttacatct ggttgaaagt 914520
ccctgatggc gacgatttgg cacttgcacg caatttatgg caaaaagcgg ctatccaagt 914580
attgcccgga cgtttttttgg cgcgggatac cgaacagggc aatcccgggg aaggttatgt 914640
gcgtatcgct ttggttgccg atgtcgcaac ttgtgtcaaa gctgcggaaa ccattgtttc 914700
cctatatcgg taaagaataa aaaaatgccg tctgaacttt tgttcagacg gcatttttca 914760
atattttacg gttgaatttg ctataacggt atttatagtg gattaacaaa aatcaggaca 914820
aggcgacgaa gccgaagaca gtacagatag tacggcaagg cgaggcaacg ctgtactggt 914880
ttaaatttaa tccactatac ttcactttta atcggcttgc ccgcaaacac gtttaaactt 914940
aaaatccccg tgtttgacac aataccgagc agattatgtt ttttgtcctt tcccctgcga 915000
agaaccttaa tgaaaaagac cctgcccctg tcagcgagtt tacccaaccc gacctgctgg 915060
cagagtccga cattctaatg cagcagttgc gcgagcttgc gccgcaacag attgccgaac 915120
tgatgcacgt ttccgacaaa attgccctct taaacgcgca gcgcaatgca gaatggaaca 915180

cgccgtttac gccggaaaac gccaaacagg cggtctttat gttcaacggc gatgtttacg 915240
aaggtatgga tgcaaacaca ttggatattg gacagatacg ctatctgcaa aaccatgtcc 915300
gcctgctgtc cggtctgtac ggtcttcttc gcccgttaga cctgatacag ccctatcgtt 915360
tggaaatggg gacggcattt gccaatttgc gcggcaagaa tttgtatgag ttttggggcg 915420
acatcattac caacctttta aatgatacgc ttgcccaagc aggcagcaat acgcttgtca 915480
accttgcctc acaggaatat ttcaagtccg tcaacacgaa aaaacttcgg gcgcggctga 915540
ttaccccaat atttaaagac gaaaaaaacg gtaaatataa aatcatcagt ttctatgcca 915600
agcgcgcgcg tggattaatg gtgcgctatg cggcagaaca tcatattacc gaccctgaaa 915660
tgctgaaaaa ttttaattac gaaggctacg cattcaatga cgcggcttca aatgaaagcg 915720
aatgggtttt tatgcgttcg gaacaaataa agtgaaaaca ataaattaag tattttccga 915780
aaaaagtgct tggcaaaatg tataaatttc attattattc ctaatcttca agaagacgga 915840
agcgtggcag agtggtttaa tgcaacggtc ttgaaaaccg tcgagggttg atagccctcc 915900

gtgagttcga atctcaccgc ttccgccaat ttttgagcgt aaaaccaaat aagaatgcaa 915960
tagccgcaaa tattgtattt ttatttggtt ttactgcatt atcggaaacg tggcagagag 916020
gctgaatgca gcggactcga aatccgctga gggtgcaaat cctccgtggg ttcgaatccc 916080
accgtttccg ccacaaaaca aaaccgccct gattcggggc ggttcttttt tgttcaagtt 916140
gtatcaatta ccatataaaa atcatcggtt tgccctatca taacgcatca aagcaaatca 916200
tttgcaatct tgcggcatct tcttattgca tttttttatg gtaatgtgta tggtaatttt 916260
tggataaatg ggaaattacc ataatggcga aaatcattac gccgctgtcg gcaaatcagg 916320
ttaaaaatgc gaagccgcgc gataagctgt ataagttgtc agacggggcg ggctggcttt 916380
gtgggtctac ccgacgggcg ggcggagttg gaagctgtcg tttgtgcagg atggaaggca 916440
gcagacaatt tcgctggggc ggtatcctga tttttcgctg gccgatgcgc gggaatggcg 916500
ggaggaggtg cgccgaaaac gggcgcacgg ggaaaatgtc gtcaataaga aggtgcgggc 916560
ggattttgct tttgagaagg tggcgcgtga ttggtttgtg cgttggtcga aggggcggtc 916620
tgaaaagtat gccggacagg ttatgcggaa ttttgagcgg tgggtttttc cggctatcgg 916680
caatcttgat attcgtcaaa tcaggacggc ggatgtggtc ggctgtctgc gtgtgatgga 916740
ggcgcgcggt atcgttgata cgttgcgcaa aacgaaaaac agtctgaaga tggtgtttgc 916800
gtttgcggtc ggttcgggaa tgatggaaat caaccctgtc gcgcaaatcg gttcgggtgt 916860
gtttgaacgg gcgaaaacgg ggaatatggc agcgttgagt ccgtctgaat tgccgcgcct 916920
gattgatttt ttggagcagc gcaatgaatt tgcggtttat gcgggcaggg tgcgtatcca 916980
tcctgtaacg cggctttgta tctattggct gctgttgaca atgacgcgga ttcaggaggc 917040
ggcgttgatg gagtggtcgg agttggacgg ggaggtttgg cgtatccccg ccgaacggaa 917100
aaaggagcgg cgggggcatg atgtgccgct gtcgcgggcg atgcagtggg tgttggatca 917160
ggcgcgggcg ttgaatgtga acgggcggtt tgtgtttgaa agtgtgaatt ttcaagggca 917220
tatcaataag gaaagtccgc gcgtggcgat gcagcgggcg gggctggata cgacggcgca 917280
cggtttgcgc tcgcttgcgc gtacttattt gcgcgaggtt ctgaaggtgg atagtattat 917340
gcggaaacgc aaataaaaaa ccgtttccgc atttttattg gaaggctttt ttgcaaccgc 917400
tttacacaaa ggcggttttt tgtgtaagaa ctgctataat agcagcccgt catcgtcagg 917460
agcggctaat gcctttaaaa ttccaaccaa gggaacgttc ggttatcatg tgcgactttc 917520
gcggttatga agaaccggaa atggtcaaga aacgccctgt cgtcgtcata gcgcgaaaca 917580
ggcacaacgg caaactggta acggtcgtac ccttaagcag cacagaacct gtccctttgg 917640
cggactacca ccacaaaatg agtggaaacc ccttaccgga caagccgcac atccaatgtt 917700
gggcaaaatg cgacatgacg gcaacagtcg gattggcacg attagaccga tacaaaccca 917760
aagggcgcga ccgctgcatt ccaataatca gtgaagagga ttttcaggcg attaaaacag 917820
ccgttgccaa ggcattcaaa ctgtactaga ataaaaccgt tcccttaaag gggcttgcaa 917880
gactgttctg aaatatgggc agccgcgcac gggcgacagg cgatgacaag ccgtccgtgc 917940
gtgtgatggg gcgcggaatg cgccccttgt cgtatctgca aacgcctaca aatccccaat 918000
cagcctttca atcaaggctg ttttggacaa acccgccttt gccgcctcct gttccagttt 918060
ggctatcgtt gtgtccgcct taacgggcga tttaagaacc gctttacacg aaggcggttt 918120
ttttgtatag tccggttcac gaggtacaga atcttgaaaa tagtcaagca atgccgtata 918180
ttccgacgca aggatttatt ttcaacatca gcttaagggg atgacaatgg gacatattta 918240
ttcggaaagc cgtgatttcc gcctttcaga cggctgggac ggcaatgact gacaacctaa 918300
tacagatagc aacgccgata ttgactgtta tcggcgtttt tgttgccgct tacggcatca 918360
tgaggaatac agaaaacgcc aaaaagcgcg ctgatcatgg ccgaacgtaa caatgccgcc 918420
cttcaagaag ccataaccat agtaaacggg ctggcaaaaa cagacggatg catactcgcc 918480
acctatacat cggatacccc ggacaagaag aaagaccgtg aagccatact gacagtttta 918540
aaccagcgcg aatttgtctg tgcgggcgta ttaggcggag cactgcacga gaaaatgtat 918600
aaagatttcg aatactccat gctgttacgt gactgggaca acctaagcag ctttattttt 918660
gaaatacgcc gtatcaggag cgcaccgacg gcctttcaag aatttgaagc cgtagcccga 918720
aaatggaaga aaaagcctct gaaaaccaaa tagcttaata gcttaacatc cgccgcaaca 918780
taggccgtct gaaattcaga cggcctttca gtttgccgcc tacggttttt tgggaaaccc 918840
cttgcatgtg caggggggttt tgttttatat tcctgttcgt ggcgtcagaa accacactac 918900
agcggcaatc actccgtcaa tgtgattttt tcatgtctat agtttctttt ccttgttgtt 918960
tgtttcgata gcaggaagtt tctatgaccg cgtgggcgac gaatacaaga cccgaaaggg 919020
gaataagtcc gccctcctat gtgggttctt aaccgcgtgt ccgcccattt gggctaattc 919080
tcttgacaca tttccataac tctatataat atttcccacg gtgcttgaaa acacctgaca 919140
aacagcgtat atccaacacg atagagtgga attttttacg tctatacgta tcaaatcgat 919200
ttactcctat gtgggggtgc gcctacccgt aaggctggcg gcgcgcctgt ttgcgtgttt 919260
tcaacacccc tgcgcccaat ttgggcattc ctaaatccta catgctgttg aagaccgcga 919320
ccctatccgc cacatggcgg cttttttatg cttgcagaaa atagaaagat tggatatatt 919380
acgaaacacg aggcgtcgaa aacctctact agaacggcat ttaccccgtc agcgtgaatt 919440
ttttacgtcc ataagttttc ttgtttggtt gtttgtttcg atatatccga actagtttcc 919500
tatggtcggg agggtgcgga atacaatacc cgcaagggga ataacgccgg ccttttctag 919560

```
taggttttcg aacctcccga ccacccattt gggtctttcg aaactaaact aggaaactat 919620
catgaacgta tctgttctca attttggtaa cacccctgta tctttccgtc aagacggttt 919680
tttaaatgca accgccattg catctcactt tggcaagtta cctaaagact acctaaaaag 919740
tgaacaaact caacaatata tctctgcact tgctgagaat ttaagcgtta ggagaaaaat 919800
cctaacggaa gcaaatcaaa tagttatcgt gaagcgtggt ggcagtgagc aaggcacatg 919860
gctgcatccc aaactcgcta ttcactttgc ccgttggctt aatccgaaat ttgcggtttg 919920
gtgcgatgag cagattgaaa ttttacttaa cggcaaaatt tcagacggca taaaaacagt 919980
taccccaaa cccacccgcg cccttccgga cggcttgacc ggcgaacaaa tcgaagccgt 920040
caaaaaactg cacaacgccc tgaccaaatc cgcacccaaa gaagcgcagg cgcgtatcgc 920100
cattaccctt tggtctgccg tcaaaagcaa gttcggatgc agctacaaag aagtacctgc 920160
cgaacagttc cccgaagttt taagcgtgat gggccgcgtg gcagttgaaa acggcgtgct 920220
gtacggcgaa gtcctcgacc gcgaaccatt gcccgcaccg caacctgccc tgcccatcag 920280
cggcaacgcc ctgtacgacc tcgccgttgc cgtcagatac ggcgcgtggg ccatccaaat 920340
gggcagagac gtttccctgc cgctgaagca gctcggctgc aaacaggcgg taacgatgtg 920400
gacggtctgg gcggaaacac gcagccgcct caaagccgcc gcaaacgccc tcgaagcctt 920460
aaacgcacac gccgacgcgg aacacgcggc aaaaatccgc ccgatgctgc ccgaaatccg 920520
caacctgtcg tcggtttgat gcagtaggga atacaaaagc cgtctgaatg tgaaaacgcc 920580
ctaatcgggc gttttttat tgctgtaacc ccagggcttc caaaacttcg cgggtgtccc 920640
atacggggga tgtgagtggg gttttcagac ggcacggtat ccgtccgtct ttttccatac 920700
gcagcagtgt cgagtttgaa atggggcggt tgcggcaggt tgcgtaggca atcagttcgc 920760
ggatggtcgg gcggtctatc cttgcgccca gttcgttgat gttcattttt ttactctcct 920820
gtaatgactc ggtttctgga agcggcgtaa tacggcatcg gcggccgtga tgaaaagcca 920880
taccgccaat gcctcagcgc cggaaaatga cagcgcgatg atgattttta atagggtgtc 920940
catcaggctt tcctttttc tctagtttcg tactcataaa tgaacatcgg gaacgcctgc 921000
ccgcttctga caagatttaa atccggtatc tgatcggtaa tcagacaaga aaaccgcccg 921060
tccccgccgt ttcccgtcga acagcaaatc acaaggttgc cgccaaatgg aatcgtatct 921120
tgattcgtgt tcatctttac tgctccggca aatacgtttt aaaaatcaaa tcttcaagtc 921180
ggcggtaaac cttcccggtc tgcaccaatg actcggtttc gggaagcgcg gccaaaagct 921240
tccgcatatg accggcaatc gcctgtaaca caagctgctc gccgcgcgtt ttgttgtgtt 921300
tggcaaccat cgccgccagc ccgtaaaccg ccaagtccat cattgcctcc acgctgtcgg 921360
cttcagcgga atgcaagaga ttaatctcta tgtcattctt gatgttctcg ttttcttcct 921420
gaaagttcat ttttccagct ccggcacttc cgcgtcgccg tgtatccatc ggtagtcttt 921480
caattcttcg gcttcgcgtt gtctgatttc ggcgtcgatt tgggcgttca acccgtcaat 921540
ctctgcctgt ttgtcggcga cggctaagcg catagcggtc aggctgtttt cagccttgac 921600
ttgcactgcg gcttcggatt gcggttggca ggcgtagatg ccggcggctg cgactgcgag 921660
taaggcggtg cggatcaagt atttcatttt gattcctcat tattggggta acggcttaat 921720
atcaggcagc gtttttgagg ttgtcttttg tcagacagat gagcgccttt ctgacggcgg 921780
caaagggggt tttgtactct ggctctatgc cgacaagtag ctccccgtct gtgttgatga 921840
tgtcgcatcc gtagcgttcg gtggggtagc cgtagtcgtt tttcccctgt tctgaggtta 921900
ctcggatgtc gatggagtat tcttctgtga tggtcatttt ggggtctttc ggatttgggt 921960
ttttgtggag gtcgtctgaa ggttcacgat ttcagacgac ttttttttag gcgattacgc 922020
cgaaatagat gtcgatttca gggaaggctt tttgtacggc ttcggaaatg tcttttgccg 922080
cttgttcgat tacagcatcg atttgctgca attcgtacca caggacgagt gcgccgctgt 922140
ttttgtcgat gcggaatttc aacagggctt caacaaagta ggatgcaccg ccttgatgcg 922200
gggtgaactc gatgccgaaa cgttcaaaca ttttgaggtt tttctctgtt tgccctgagt 922260
cttcggattg gaaggtaaag ttgattctgc cgtcctgttc acgatagcct tgtttgaagg 922320
tggtttttc ggtgtactcg agattgagcg cgaaatccaa tacttcggcg gcggtcgggt 922380
aaacggaatt ttcgttaccg ggatttttgg atacgatgtt gcgggcattg ttggtcagga 922440
aatgggaaaa ctccatctga ttcatgcggt gcgcattgtt gttcagccag ttggatgccg 922500
atgtagtgtg ttgcggggtg tatacggcat aaaaatcgag ccagcccgga gcttgttgcg 922560
tatggccatt gatgacggcg gtgacatcaa tacgccctga tttgaaatcg gcatcaatgt 922620
agatttgtgt gccgtcctgt ttgtgttttt gtacaaactt aataagactg gcggtatcgt 922680
gcatgaggaa tttgccgcac ttgcggtacg ggttttgcat caattcgggg tgtgatttgt 922740
atctccagcc accgtcttgg tctggtgtga atacaagcgg agtattgttc ggtgcaaact 922800
caaaaaaagg tttttgagct gcttgtaagg cggttttaat catgttttct tgggtttcca 922860
ttttagattt ccttttgtgt ttgagttagt tggatctgac cattttcaac gtgcttgacg 922920
tattggaaac ttgtttgagt ttcaattttc cttgtgccgg atcgtcggct tggatgttgc 922980
cgtcaggtgt agcaaagacg atgccgcctt cgcgtttttc tttgggcagt ttggttgcta 923040
catcgtggct gatttttacc gttccgcttt ggatgttttg gggttggatt ttgagcttga 923100
cagtaatttc tgactgttta ccgtgagcaa ggcaggcacg caccgcttca ctcatggctt 923160
cgcctaattc acggtctaac cagccgctat ttacggtcgg tatttgcttg gaggcaggaa 923220
```

caaatttctt ttcttgggtt tccatttttg attttccttt aaagagaggt tagttaggtt 923280
gccggtatca cgggatgaca ataccgagga ggttatttaa ttttgagaat ttccaaagat 923340
tccacggtgg agcgggctat tccaaactcg ttaagtgcgg caaagccggc tgctggtatg 923400
gtggatgctc cgtaatttat ttttggaata aaattttcgt tcagggcgac ggcggttctt 923460
gcgagattta gcaaagcgtc gaaattttct ttagggatgg tgatggtttc catgtcggta 923520
ctccatgtgg ctgttgtttg tttcgatggg tgtatttaaa catagcgttt aataatatgc 923580
aacaatctgt ttaagatttt tgtttaaggt ttataaacat tttgattatt aaaagaattt 923640
atttttgaga tttcgcaggc gcaaaaaaac cgcctattaa ggcggctttg tcggttttgt 923700
gttgttttca ggttcggcgg ggcatgaaaa aagcccgcat aatgcgggcg ggttgtttaa 923760
tctgtggggt cgtatgccac atcaactatc agcttcgttt tccttaggtg ttggttgata 923820
gcataaattt cgtgccatgc cttgcttaga taatactgcc gaagcatcgg gattttagat 923880
agccggtgtg gcattgggat atttcgtgcg caccatagcc gcaacagagt gtcttcgtca 923940
tcgatttgga tatgttttaa gattaagccg cttgaaccga cgggcaatag ataccttgcg 924000
tcagggttgg caaaatgctt accggaacca tcaaagtcat acaatacatt tggcttgagg 924060
tctttgtagt tctcttttct gcgcggcgca gatacttcgc caagccactc aaagcacagg 924120
gttgctgttc tgtgatgcag ctcgtgaacc ttcaactctc cttgtacgcc aaggaaattc 924180
ataccggcat cgtactcacc aggccaccaa ggggaatcaa aacgcctatt tttgacaatc 924240
ttgaaagccc tatcaatatt atctcgtcgt aatagcaaca tttttcaatc cagcacgctc 924300
caccaaaata ccctgccgat aacggtcagg ctgtccaagg gggcgttttc gtcgccatag 924360
aaaccgctgt tgtggctgcg tatcagaacg ctgttgccag gctgccgtat caggtacttc 924420
acgcggaaca taccgtcctg ggcgaaggcg tagattttgc cgtcgcgtat ggcggtttcg 924480
cccgtatcta cggcaattgc cgcgtcttct gcgatttttt cctccatact gtcgccggtc 924540
agggtgcagc aaaacacgtt gtcgggattg atgcctttgc gtttaagcgt ggatttgccg 924600
aacggcaggc ggtagccgtt gtaatcgggg atttcatacg tgcctactcc gcctttgaag 924660
cagctctctt tgaggtaggg gacgaaaaca taatcatcgt cgggcagcgg gtcgttgctg 924720
ctccacgtca tcgggcggtg gatgtctttg acttcgtggg gtaggtcggg gtcaataagg 924780
acgggcgcgg ttcggctgcc ttcacctgtt ctcagccatg tttcagatac accgaatgct 924840
tttgctactt caggcagcgc ctttgccgct atgccacgac tttcccagtt tttcaaagcc 924900
tgttggctga tattcagacg ctctgctatg tcagccggct ttaaaactcc ctgctctttg 924960
gctatctcaa aaagtctgtc agttgtctcg tgcattgtca tttttaatct tattcgcggt 925020
tggcttaatt attctcccat atttaaacaa aatgttgtta cacaagactt gatttttatc 925080
taaacatagt gtttaatatt agcattagat ttaaacattt ggtttattta tggataaaag 925140
agtcaatgaa gacaaacgcc tgttgcaatc aatcggcagt tacgcggaag ttggtcgaat 925200
aacagggaat agccctcaat gcgttttcaa ttggacgaag cgcgggatac ctgcacgaat 925260
aaaacttaag tatcccgacc tgtttttgaa ctcaaagaaa ccagacgacc aacccaaata 925320
aaaaaagcct gtcgtggaaa gctcgccgcg ggctggggaa gccgcattga tgacgataat 925380
ttttaatatt gcttggattc ggatttcaag tgcaacacta gtgtattagt ggttggaaca 925440
gattcaagaa taaaacactt ggcgtttcgt agccaagtgt ttttcttggt cggtggttca 925500
actcatcttg aaccctgcgt atctcccgat cactgatgtt acggaaatcg gtttgtttgg 925560
ggaagtattg ccggatgagt ccgttggtgt tctcattcag ccctttctcc caagaatggt 925620
aagggcgaca aaaataagtc tccgctttca atgctttggt tattttggtg tgttggtaga 925680
actctttgcc gttatccatg gtgatggtgt gcaccctgtc tttatgtgcc tttaatgtcc 925740
taacagctgc ccgggcagtg tcttcggctt tgaggctatc caatttgcag atgatggtgt 925800
agcgggtaac gcgttcgacc aaggtcaata atgcgctttt ctgtcctttg ccgacaatgg 925860
tgtcggcttc ccaatcgccg atacgggatt tctggtcgac gatagcgggt cggttttcta 925920
tgccgacacg gttgggtact ttgcctctgg tccatgtgct gccgtagcgt ttgcggtagg 925980
gtttgctgca tattctgaga tgttgccaca acgtgctgcc gttgcttttg tcttggcgaa 926040
ggtagcggta aatggtgctg tggtggagcg tgatctggtg gtgtttgcgc aggtaggcgc 926100
atacttgttc gggactgagt ttgcggcgga taagggggtc gatgtgctga atcagctgcg 926160
aatcgagctt atagggttgt cgcttacgct gtttgatagt ctggctttgc cgctgggctt 926220
tttcggcgct gtattgctgc ccttgggtgc ggtgccgtct gatttcgcgg ctgatggtgc 926280
ttttgtggcg gttaagctgt ttggcgattt cggtgacggt gcagtggcgg gacaggtatt 926340
ggatgtggta tcgttcgcct tgggtcagtt gcgtgtggct catggcaatc tttcttgcag 926400
gaaaggccgt atgctaccgc atactggcct ttttctgtta gggaaagttg cacttcaaat 926460
gcgaatccgc cgtgcgctga aattcgacca gttgatactc gaatttcctg agcgcgggga 926520
cggcgcatgg gtacacatcg gtttccgacg caacagcccg caacgcaacc agatactgac 926580
cgcaaccaag aaaaacggca aaaccgtgta tctgcccggg ctgcatcctt gaggtcgtct 926640
gaaatggata ttttattgaa atactggaag ccggtaggtg tattgctgct aatcgtcctg 926700
atttttaccg catggcattt cgaccgtgcc gaaaaatacc gcatgggacg ggaggctgct 926760
gctgccgaaa tctcgaatcg tctgaaagac ggctatatcg agcaggcaaa gcaggcgcgt 926820
tctgccgagc agaaggccgc tgccgcgttt gccgaacgac aaaccaaatt agaagaggaa 926880

aaacaaaatg ctgaaaaaac tgttgccgct atgcgtcttg agcttaaccg cctgcgccac 926940
tacgccgccg cccaaaatcg caacagaaac ctgcccgcaa ccgctaccgc cgccaccgca 927000
tctgatggcg cgtcagattc ccaaggctgg ctattattcg gacagtgcgc tgaaaaatat 927060
gcaggactgg cagaaacagc cgacggacat gcagccgatt taagggaatg gcaggcatat 927120
ggtcatgcag taagcagtca gcgtgccgaa taagcaaccg cccgaacctg taagaaaaga 927180
ttacaggttc gggcggtttc agcatttaat cgaataagac ggcgccgatg cgcgccagca 927240
catcgtccaa tacataatcg ggtacagttt ctttaaaaga agccttgcgg atttgccagt 927300
ctagagattt gagctggtgg cagtggacat tgccctgcgt ttccgttcct gcaccgagta 927360
aggttgaaat catgccgctg cttcgtgcag ctgctgcatt cccctgtgaa atggggcagg 927420
caaaaaccaa tcccgttgcg cggttgaatg cttttggaga cagagccagc gcaaaccgcc 927480
cgccccttgat ttccttgccg ctggaagggt cgaaattcaa atggaaaata tcgcctttgt 927540
cgggaatata catttcagac gacctcgttg ccggcatcat ccaagatttc ccagccttct 927600
acgcgcggcg gggtttcttc catttcggca agcaagtctg ccaagcggaa acgtcgggca 927660
gcacgcacac ggagttcgcc gttatgtact tccgctacca aagcgtcgcc gattttaaaa 927720
tccaattgtt tcagcatgtc ggcaggcagt cggacggccg ccgagttccc ccatttttgg 927780
acacgcaaca taatcttcac ctttattgta tctacaaagt agatacatat taccataaaa 927840
tttcagttgt tcaaatactt gtgcagaata cgccaaaagc cgtccgaact gtttcggacg 927900
gcttttgtac tgtatttgcg ccttcaggca atattttgtt atccattttc aagatgcaaa 927960
agctttctaa ttgcttgatg tcggatttcg gttgtttagg gatacaaaac caagtaaact 928020
aaaactgttt gattggaaaa tgctccgcaa ggagaaaatt atgttcaaaa aatcacttta 928080
taaggctgct ttggcgtatt tcggcgattg cgtggctgcc catatttcag aacagttttg 928140
actgtttatt cacaaatcag atgcctttag gggtttgct ttccataatg caaccaaaat 928200
ttccaactct ctaaatattg tgtctttgcg ttcttcttcg cgtattttca taacaagagg 928260
cgaaactgca ttccacaatt ttatgaagtt ggtgcaatgc agccgtttaa acaaatcctc 928320
atccaatact cccgagttga ttgcgcacgc ataaaactcg tgccgattga ttacggtaag 928380
taaatgccct ctctcttttt cccattgtcc gcccttattt tgataaattt catacaggtc 928440
gatccgcgcg ttgttgtcgt caactgccgt gccgcgaata taaggcgaaa tatgattgtc 928500
ggcttctaca aattgtgcat cttggtaatc attcaaaata acatctaatg tcgccttttg 928560
ctttgaagtt ttcttattga tgaatattgt ccccagtgca atgacggcgg ttactgaaac 928620
aacggtcagt tgccaaaaca tcagccattc ggccgtccct catagggttaa actgtacgct 928680
gtgcctgaat atgtaaaata aagtgaatac agatacaaac aaggaggtta gggcaattac 928740
tgacaaccaa cctccttgtt ttatttgttt ccgcacctta ccagccatta aaaccctcat 928800
ttttgtattt catcgtattt ccttaatggt tttaaccttg cttgtcgata tctgtatatt 928860
aatgccgtgc gcccgttctt tcaaggggtg atttaaaaat caggcatcct tgacatcctc 928920
tcctgcttaa aggcgggggg actcctgccg tgtaaaccaa tgccgtctga agggctttca 928980
gacggcataa aaaaaccgcc tttgtgtaaa gcggttgaag aaaagccttt caataaaaat 929040
gccgtctgaa tttcagacgg cattgttgtc ggatatgcct attccttatc cagccggcgc 929100
agggtttcgg cgagctgttt gaagtcggtt tctcccgccg ccaaactgac aatcaagtcg 929160
tcaagccctt ggtcggcggc aatgctgatg ccctgcaaat ccagataggt caacattgtt 929220
aaaagcgcgg tgcgcttgtt gccgtcggga aaggcgtggg ctttggctat ggcttgtgca 929280
tagagggcgg cgatttcgta gatgtcctca aggttttcat actgccgcca gttggcaatc 929340
cgcgacaatg cgccgtccaa gcgcgccata tccgcccgcc ctttcaaacc cgcttcatcc 929400
gccaatacgg tttgatggat aagcgcgacc agttcgccgt ctatcatttg tcggcaagtg 929460
ccttgacggc tttttgatgg gttttggcaa tgcggcgggc ggcggcaagc agtacgcgtt 929520
tgcctgcttc gcctttaagc tcgattttga cggggcgtgt attttggttt tgcatcgctg 929580
ctccttgtct gtttgcgggc attttagctt ttttccggca gcttgggaaa tgccgtccga 929640
aaacacttca gacggcattc ttctaatagt gtaatgcaat agttactcca agatttttgt 929700
atatggctgt ttttatggta aattaaattt tcataagaaa taaatatatt taaatcaatg 929760
aaataaaatt tagtcgaatc ccaccgtttc cgccacaaag caaaaccgcc ctgattcggg 929820
gcggttcttt tttgttcaag ttgagacctt tgcaaaattc ctttccctcc cgacagccga 929880
aacccaaaca caggttttca gctgttttcg ccccaaatac ctcctaattt tacccaaata 929940
ccccccttaat cctccccgga tacccgataa tcaggcatcc gggctgcctt ttaggcggca 930000
gcgggcgcaa atcagtccga aataggccgc ccgggcgtag cggaatttac ggtgcagcgt 930060
accgaagctt tgttcgacca cataacgggt cttagataaa taccggttgc gtttggtttg 930120
cgtttccgtc agcggacggt tgcggcaggc tttgcgcata atgccgtcct gcaactgatg 930180
ttcttccaga tgttgccggt tttccgcact gtcgtagcct ttgtcggcat agatggtcgt 930240
accttcgggt aacccttcca acaacggcga caggtgtttg cactcatggg cattggcggg 930300
agtaatgtgc agtttctcga tatagccttc cgcatcggta cgtgtatgtt gtttgtaacc 930360
gagtttgtag aggctgtttt tctttgtcca acgggcattt ttgtccttac tcagtgtggt 930420
ttgaccgctg acttgtccct cttcatcgac ttctatggcc tggcgctgtt tgctgccgac 930480
ggtctgaata atggtggcgt caacgacggc ggcggatgct ttctctactt ttaagccttt 930540

ttcggtcagt tggcggttaa tcagttccaa cagttcggac agggtgtcgt cttgcgccag 930600
ccggttgcgg tagcggcata aggtgctgta atcggggatg ctcagttcgt caaaacggca 930660
aaacaggttg aagtcgatgc gggtgatgag gctgtgttcg agttcgggat cggagaggtt 930720
gtgccattgt ccgagcagga cggctttgaa catggacaac aggggacagg cgggacgacc 930780
gcggtggtct cggaggtaac gggtttttg acggttcagg tattgttcga tcggctgcca 930840
atcaatcacc tggtccaact tcaatagcgg gaagcggtcg atgtgtttgg caatcatggc 930900
ttgggcggtt tgttgaaaga aggtgctcat gagaaatccc ctaaatgtct tggtgggaat 930960
ttaggggatt ttggggaatt ttgcaaaggt ctcgaccttg tgtttttaa ggtattcgat 931020
agtatgggcg atacctttgg ggttgttggt ttcggttttg gttttagaca aagacgaaac 931080
ggcgatgacg aagtttcgtt tggcgatgtc gatatagtga attaacaaaa atcaggacaa 931140
gacgacgaag ccgcagaaag tacagatagt acggaaccga ttcacttggt gcttcagcac 931200
cttagagaat cgttctcttc gaactaaggc gagacaacgc cgtaccggtt tttgttcatc 931260
cactataaca gcaaccctgt cgccgtcatt cccgcaaaag agggaatcca gtccgttcag 931320
tttcggtcat ttccgataaa ttcctgttgc ttttcatttc tagattcccg cttttgcggg 931380
aatgatgacg gaagggtttt ggttttttcc gataaattct tgaggcattg aaattccaga 931440
ttcccgcctg cgcgggaatg acgattcata agtttcccga aattccaaca taaccgaaac 931500
ctgacagtaa ccgtagcaac tgaaccgtca ttcccacgaa agtgggaatc tagaatctca 931560
gactttcaga taatctttga atattgccgc tgccttaagg tctggattcc cgcctgcgcg 931620
ggaatgacga atccatccgc acggaaacct gcaccacgtc attcctacga acctacatcc 931680
tgtcattccc acaaggacag aaaaccaaaa tcagaaacct aaaattcgtc attcccgcga 931740
aagtgtgaat ctagaaatga aaagcaacag gcatttatcg aaaataactg aaaccgaaca 931800
gactagattc ccgcctgcgc gggaatgacg gctgcagatg cccaacggtc tttatagtgg 931860
attaacaaaa atcaggacaa ggcgacgaag ccgcagacag tacagatagt acggaaccga 931920
ttcacttggt gcttcagcac cttagagaat cgttctcttt gagctaaggc gaggcaacgc 931980
cgtactggtt ttttatatcc aatgggtgcg gcgtttaatc ataatcaggc agatagggat 932040
aactaatgcc gtctgaacga cgaatgttca gacggcattt ttacctttgt gcttataagg 932100
cgtttagtgc ctgattaaag gttacgctcg gacgcatcac ttgtgcggct ttttcaggat 932160
tggcggcgta gtagccgccg atgtcggccg ctttgccttg tacggcggaa agctcggcaa 932220
cgattttcgc ttcgtcggcg gtcaaagcgg ctgccaatgg cgtaaatgcg gctttcagtt 932280
cggcatcttt gtcttgcgcc gccaattctt gcgcccagta gagggtgagg tagaaatggc 932340
tgccgcggtt gtcgagttcg ccggctttac gtttaggcga tttgtcgttc aacagcagtt 932400
tttcggtggc tgcatccaaa gtgtcggcga ggacttgggc tttggcattg ccggtttttt 932460
gcgccaaatg ttcaaacgat acggcgagtg cgaggaattc gcccagcgag tcccagcgca 932520
aatggttttc ttcgaggaat tgttgaacat gtttcggtgc agaaccgccc gcgccggttt 932580
caaacatacc gccgccgttc atcaatggaa cgatagacag cattttcgcg cttgtgccga 932640
gttccaaaat tgggaacaag tcggtcaggt agtcgcgcaa gacattaccg gttacggaga 932700
tggtgtcttc gccgtttttc agacgaccca agctgaactt ggcggcttct tcaggagcga 932760
ggacgcggat gtcgaggcca ttggtatcca gttcggcaag gtaggcttta accttggcga 932820
gcaggctctt gtcgtgcgga cggttttcgt cgagccagaa cacggcaggc gtgttgctca 932880
gacgggcgcg gttgacggca agttgtaccc agtctttaac cggagcgtct ttggtttggc 932940
acatacgcca gatgtcgccg gcttcaacgt cgtgctgcat taggactttt cctgccgcat 933000
caatgacttg gacttggccg tcggcttcga tttcaaaggt tttgttgtgc gagccgtatt 933060
cttccgccgc ttgcgccatc agtccgacgt tgggcacagt acccatggtt gtcgggtcaa 933120
atgcgccgtg ttcgcggcag aagtcgatgg ttgcttggta aacgccggca tagctgctgt 933180
cgggaatcac ggctttggtg tcttgcgctt tgccgttttt gtcccacata cggccggaat 933240
tgcgaatcat cgcaggcata gaggcatcga cgatgacatc gctgggaacg tgcaggttgg 933300
tgatgccttt gtcggaatca accatcgcca aatcggggtt ggcagcgtaa acggcggcga 933360
tttcggcttc gacggcggtg cgggtgtccg catccagttt gtccagattg gcaagcaggt 933420
tgccgaagcc gttgttaacg ttgacgccgg cagcagccag tttgtcgccg aatttttcaa 933480
aaacaggcgc gaagaatact ttgacggcgt gtccgaagat aatcgggtcg gacactttca 933540
tcatagtggc tttcatgtgc agcgagaaca acacgccttt tgctttcgca tcttttactt 933600
gttcggcaag gaaggcgagc agggcttttt tactcatcac ggtcgcgtcg atgatttcgc 933660
cggctttcag ggcgacgggc tcgcgcagct ctttttttgtt gccttgtttg tcggtgaata 933720
cgatggatac ggaagtcgct tcaggtacga taacagattg ttcgttatga aaaaagtcgc 933780
cgctttgcat ggtggcaacg tgggttttgg agtctttggt ccatgcgccc atgctgtgcg 933840
gatttttttt cgcaaagttt ttcactgctt taggggcgcg acggtcggag ttgccttcac 933900
gcaggacagg gtttaccgcg ctgcctttga tgcggtcgta gcgttcgcgt acggcttttt 933960
cttcatcggt ttgcgggtcg gcgggatagt cgggaacggc aaagccttta gattgcaatt 934020
ctttaatcgc ggcagtcagt tgcggtacgg acgcgctgat gttcggcagt ttgattacgt 934080
ttgcatcggg ttgtttcacc agttcgccca attcggcaag cgcatcaggt acgcgttgcg 934140
cttcggtcag gtattcggga aacgccgcca aaatacggcc ggagagagaa atgtcgctgg 934200

ttttgacatc aatatcggcg tggcgggcaa aagcctgcac aatcggcagc agcgattggg 934260
tcgccagcgc gggtgcttcg tcggtatggg tataaacaat ggtggatttt tgagtcatag 934320
gattattctc ttgtaggttg gttttttctt ttggaacaca ttgcgcgggg aatgtgcgtg 934380
gctattatgg catattttgg cggctttgtt cgcgctttgt tcgatcttgg cgtgtttgaa 934440
cgcggcggcg tgaaaggaag ggggaaatgg ttttcccgcg tttggcggcg gtcggaggtg 934500
ctgtgcctga tgtgcggcgg catattttcg gtgaaattga ttttatagtg gtttaaattt 934560
aaaccagtac agcgttgcct cgccttgtcg tactgcttgt actgtctgcg gcttcgtcgc 934620
cttgtcctga tttaaattta aaccactata atattcggta actgtcggaa tatctgctaa 934680
aattccgcat ttttccgtcc cgggacactc ggggcgtatg ttcaatttgt cggaatggag 934740
ttttagggat atgggcttga aaaaggcttg tttgaccgtg ttgtgtttga ttgtttttttg 934800
tttcgggata ttttatacat ttgaccgggt aaatcagggg gaaaggaatg cggtttccct 934860
gctgaaggag aaactttttca atgaagaggg ggaaccggtc aatctgattt tctgttatac 934920
catattgcag atgaaggtgg cggaaaggat tatggcgcag catccgggcg agcggtttta 934980
tgtggtgctg atgtctgaaa acaggaatga aaaatacgat tattatttca atcagataaa 935040
ggataaggcg gagcgggcgt actttttcca cctgccctac ggtttgaaca aatcgtttaa 935100
tttcattccg acgatggcgg agctgaaggt aaagtcgatg ctgctgccga aagtcaagcg 935160
gatttatttg gcaagtttgg aaaaagtcag cattgccgcc tttttgagca cttacccgga 935220
tgcggaaatc aaaacctttg acgacgggac aggcaattta attcaaagca gcagctattt 935280
gggcgatgag ttttctgtaa acgggacgat caagcggaat tttgcccgga tgatgatcgg 935340
agattggagc atcgccaaaa cccgcaatgc ttccgacgag cattacacga tattcaaggg 935400
tttgaaaaac attatggacg acggccgccg caagatgact tacctgccgc tgttcgatgc 935460
gtccgaactg aagacggggg acgaaacggg cggcacggtg cggatacttt tgggttcgcc 935520
cgacaaagag atgaaggaaa tttcggaaaa ggcggcaaaa aacttcaaaa tacaatatgt 935580
cgcgccgcat ccccgccaaa cctacgggct ttccggcgta accacattaa attcgcccta 935640
tgtcatcgaa gactatattt tgcgcgagat taagaaaaac ccgcatacga ggtatgaaat 935700
ttataccttt ttcagcggcg cggcgttgac gatgaaggat tttcccaatg tgcacgttta 935760
cgcattgaaa ccggcttccc ttccggaaga ttattggctc aagccggtgt atgccctgtt 935820
tacccaatcc ggcatcccga ttttgacatt tgacgataaa aattaatcgc atagcaaatc 935880
aaaatagaaa atggcggagt gcgtggggta aaaataagga tagcgttttt tcatttggat 935940
tgacgataat ttctgattgc tttgcgtgtg ctgaaatggc aaagaaaatg ccgtctgaag 936000
tcttcagacg gcattgtttt gttttggatg ttattcgggc gcgcggaaac tgtcgtggca 936060
ggatttgcag cttgcgccgg tttcgccgta ggcggctttg atttcgtcca gtttgccggt 936120
ttgggcggcg gcgttgagtt tttcgacggc ggcggcgaat tttgtttttt cggcttcaaa 936180
ttttgcacca tccgaccaaa cggcgggcag tgcgcggccg ttgccttgcg gatcggactc 936240
aaaaagtgtg aacggtttct tgctgctttc ggcaaacgac gctgccgcct gtttgaattt 936300
ttcgacatcg taaggttctt cgtctttgac cattttgccc atgcgtgtga attcgggcat 936360
catggatttg aacgcggcgg tgcggttttc ggaaatttcg cctttgggtt gggaaggtat 936420
tccgctgcct ccgcaggcgg aaaggagcag tgtgatggcg gcagcagcaa ggctgatttg 936480
ggttttcata ttgaatgtgt cctgtcgtgg tggtatggtt gttgtcattt tcagtcggcg 936540
cgatgccgtc tgaagggtgt tatgatacct gaaacaggtt tgggaaacga gaaagaaagg 936600
aaaaacaatg gctgttttaa ttaccggtgc ttcggcagga ttcggcgaag cgatgtgccg 936660
tgcgtttgtc ggggcgggat accgcgttat cggtgcggcg cgccgtgcgg acaggcttca 936720
ggccttggcg gatgaattgg gtgctttgtt ttaccctttg gaaatggacg tgtcgcgacg 936780
cgagtcggtg gaaaacgcct taaacggcat ccccgatgaa ttttccgaca tcgactgcct 936840
catcaacaat gccgggctgg ctttgggtct ggacacggcg gacaaggcgg attttgaaga 936900
ttgggaaacg atgattcaaa ccaatgtttt gggtttgacg ttcctgacgc gcaaaatttt 936960
gccgcaaatg gtggaacgcg gcggcggtta tgtgatgaat ttgggttcga ttgcaggcaa 937020
ttatgcttat gccggcagca acgtttacgg ggcgaccaag gcgtttgtgc gccagttcag 937080
cctgaatttg cgcgcggagt tggcggataa gaacatccgc gttaccaata tcgagccggg 937140
tttgtgcggc aatacggagt tttccaatgt gcgcttcaaa ggcgatgacg agagggcggc 937200
gggcgtgtat gagggtgtgg aatttatccg ccccgaagat attgcggaaa ccgcattgtg 937260
gctgtaccag cggccggcgc atatgaatgt gaacacgatt gaaattatgc ccgtggcgca 937320
gacttttgca ggaatgaaag tgataaaaaa agccgtgccc gaagtgcggg aagactttga 937380
aaaacagagt atgtcgctgt tttcccgcat caggtcctgg ttcaaatgat gcaaaatgcc 937440
gtctgaagac agtttcagac ggcattttta cgggtatttt tacggagtag gcaataagcc 937500
cgccaatttg gggttgcctt ctttcggaat cgggcgcgga ttgccttccg catcgatggc 937560
aacataagtg aacacggctt cggttacgag gtagcggtct tcggtaacgc aatcgttcat 937620
caaagttttc acccagacgt cgactttaag ctggagggaa gtgttgccca cgcggacgca 937680
atgcccgtag cagcagacga cgttgccgac cttgaccggg cggatgaagt tcatttcctg 937740
aacggcgacg gtaacgatgc gtccccgcgc gatttccgcc gccaatatgc cgccgcccaa 937800
atccatctgc gacataatcc agccgccgaa aatgtcttgg ttgggattgg tatcgcgcgg 937860

catagcgacg gtacgcagga gcagttcgcc ttgagggcgt tggcggttgc cttcttcgtg 937920
ctgcataaag tttccttgtt ttattgaaat ataaatcgaa cctgcacccc tgcccgaaac 937980
gggaagtcgg gaagtgtgta gtttaaccca tttgagacat acaagggcgg gcgcggaaca 938040
gattcgcaag gcgtattgta gggcggggct gtagagtggg cttcagtccg ccaatcccgc 938100
caaatcctac cctaagcaac tgaaccgtca ttcccacgaa agtgggaatc tagaacgcgg 938160
ggtttcagtc atttccgata gattcccgcc gcgtcggggg tctggattcc cgcctgcgcg 938220
ggaatgacga atccatccat acggaaacct gcaccacgtc attcccacgg aagtgggaat 938280
ctagaatctc ggggtttcag tcatttccga tagattcccg ccgcgtcgga ggtctggatt 938340
cccgcctgcg cgggaatgac gggttttaag attacggtgt tgtcggacgg atttcaagat 938400
tacggtgttg tcggaacgca actgaaccgt cattcccacg aaagtgggaa tctagaatct 938460
cggggtttca gtcatttccg atagattccc gccgcgtcgg gggtttggat tcccgcctgc 938520
gcgggaatga cgaatccatc catacggaac ctgcaccacg tcattcccac gaaagtggga 938580
atctagaacg cggggtttgg gcaactgttt ttatccgata agtttctgtg cggacaggtc 938640
tggattcccg cctgtgcggg aatgacgaat ttcaagattg cggtgttgtc ggacgggttt 938700
tgagattacg gtgttgtcgg agcgcaactg aaccgtcatt cccacggaag tgggaatcta 938760
gaacgcgggg tttcagtcat ttccgataga ttcccgccgc gtcaggggtc tggattcccg 938820
cctgcgcggg aatgacgaat ccatccatac ggaaacctgc accacgtcat tcccacggaa 938880
gtgggaatct agaatctcgg gggtttcagt catttccgat agattcccgc cgcgtcaggg 938940
ggctggattc ccgcctgcgc gggaatgacg aatttcgaga ttacggtgtt gtcgggaatg 939000
acgaatccat ccatacggaa acctgcacca cgtcattccc acggaagtgg gaatctagaa 939060
cgcggggttt cagtcatttc cgatagattc ccgccgcgtc gggggtctgg attcccgcct 939120
gcgcgggaat gacgaatcca tccatacgga aacctgcacc acgtcattcc cacggaagtg 939180
ggaatctaga atctcggggt ttcagtcatt tccgatagat tcccgccgcg tcggaggtct 939240
ggattcccgc ctgcgcggga atgacgggtt tcgagattgc gttgttgtcg ggaatgcaac 939300
tgaaccgtca ttcccacgga agtgggaatc taggacgtaa aatctaaaga aaccgtttta 939360
tccgataagt ttctgtgcgg acaggtctgg attcccgcct gcgcgggaat gacgggtttc 939420
gagattacgg tgtatcggga atgatgggaa acggtgggaa ttgtgtaaaa aatgccgtct 939480
gaaggttcag acggcatcgg tatcggggaa tcagaagcgg tagcgcatgc ccaatgagac 939540
ttcgtgggtt ttgaatcggg tgttttccaa gcgtccccag ttgtggtaac ggtatccggt 939600
gtccaaggtc agcttgggcg tgatgtcgaa accgacaccg gcgatgacac caagacccac 939660
gctgctgatg ctgtggcttt cgtgataggg aggtttgctg ggatcagttt gtataatagg 939720
acctccctgt gcagcgcctt gcgttggttt agaggtaaca atcgtggttt tggtttccac 939780
cgaatgaact tgatgtttaa cgtgtccgta ggcgacgcgc gcgccgatat agggtttgaa 939840
tttatcgttg agtttgaaat cgtaaatggc ggataagccg agagaagaag aggcgtggaa 939900
gctgccgttt ccctgatgtt ttgtttgggt ttctttgtag ttgttgttta tctcttcagt 939960
aactttttta gtagaagaat tactttcttt ccattttctg taactggcat aatctgccgc 940020
tattctccag ccgccgaaat catagccgac cgacacccgg gggtggatgg aatgcgcacg 940080
gatgtttctg aaataatcgc ttaccgtgct tgtgttgttt gcaccggttg cttgcggata 940140
atcgtgggta atgcgttcgg cggcataagc taaatccgcc tgcacataat acgggctgcg 940200
gctgccgtct tcacttgccg cctgcgctgc ggaagagaag agaagagaag agaagagaag 940260
agaagagaag agaagagaag agaagagaag agaagagaag agaagagaag agaaggtttt 940320
ttgggggctg gattcatttt cgactccgta ttcggtttta actgattaaa aagaacaatt 940380
ttcaatgatg ttgcaggagc ggactatatc aggtttgtgg cgatgtttca acacaatata 940440
gcggatgaac aaaaaagaga acgattctct aaggtgctga agcaccgagt gaatcggttc 940500
cgtactatct gtactgtctg cggcttcgtc gccttgtcct gatttttgtt aatccactat 940560
aaagaccgtc gggcatctgc agccgtcatt cccgcgaaag tgggaatcta gaaatgaaaa 940620
gcagcaggaa tttatcggaa acgaccgaaa ccgaacggac tggattcccg cctgcgcggg 940680
aatgacggga ttttaggttt ctgattttgg ttttctgttt ttgagggaat gacgggatgt 940740
aggttcttag gaatgacgtg gtgcaggttt ccgtgcggat ggattcgtca ttcccgcgca 940800
ggcgggaatc tagaccttag aacaacagca atattcaaag attatctgaa agtctgagat 940860
tctagattcc cactttcgtg ggaatgacgg ttcagttgct acggttactg tcaggtttcg 940920
tttatgttgg aatttcggga aacttatgaa tcgtcattcc cgcgcaggcg ggaatctaga 940980
ccttagaaca acagcaatat tcaaagatta tctgaaagtc cgagattcta gattcccacg 941040
aaagtgggaa tccaggatgt aaaatctcaa gaaaccgttt tatccgataa gttcctgcac 941100
tgacagacct agattcccgc ctgcgcggga atgacgggat tttaggtttc tgattttggt 941160
tttctgtttt tgagggaatg acgggatttt aggtttctga ttttggtttt ctgtccttgt 941220
gggaatgacg ggatgtaggt tcgtgggaat gacgtggtgc aggtttccgt gcggatggat 941280
tcgtcattcc cgcgcaggcg ggaatctaga ccttagaaca acagcaatat tcaaagatta 941340
tctgaaagtc cgagattcta gattcccgct ttcgcgggaa tgacgaaaag tggtgggaat 941400
gacggttcag ttgctacggt tactgtcagg tttcggttat gttggaattt cgggaaactt 941460
atgaatcgtc attcccgcgc aggcgggaat ctagtctgtt cggtttcagt tatttccgat 941520

aaatgcctgt tgcttttcat ttctagattc ccgcttttgc gggaatgacg gcgacagggt 941580
tgctgttata gtggattaac aaaaaccagt acggcgttgc ctcgccttag ctcaaagaga 941640
atgattctct aaggtgctta agcacgagtg aatcggttcc gtactatccg tactgtctgc 941700
ggctcgccgc cttgtcctga tttttgttaa ttcactatat cgcgattttt cggcatttgc 941760
ctttcggggc ggcttgtgtc tcgtgcgtga tgttgcgtgt gggaatgttc ggattgtcag 941820
aagcaatatg ggagaagatg atgtatgaga taaaacagcc ttttcatagc ggatacttgc 941880
aggtgtctga aattcatcaa atttattggg aggaatcggg caatcccgac ggtgtgccgg 941940
ttattttttt acatggcggg ccgggcgcgg gggcttcgcc tgaatgtcgg ggttttttca 942000
atcccgatgt gttccgcatc gtcatcatcg accagcgcgg ttgcggacgt tcgcgcccgt 942060
atgcttgtgc ggaagacaat acgacttggg atttggtggc ggatattgaa aaagtccgtg 942120
aaatgctggg tatcgggaaa tggctggtgt tcggcggttc gtggggcagc actttgtcgc 942180
tggcttatgc ccaaacccat cctgaacggg taaagggatt ggtgttgcgc gggatatttt 942240
tgtgcaggcc gtctgaaacg gtgtggctga acgaggcggg cggtgtgagc cggatttatc 942300
cggaacaatg gcaaaaattt gtcgcgccga ttgctgaaaa tcggcggaac cggctgattg 942360
aggcgtatca cggattgctg tttcatcaag atgaagaagt gtgcctgtct gccgcgaagg 942420
cttgggcgga ttgggaaagc tatctgatcc gtttcgagcc ggaggaagtg gatgaagatg 942480
cttatgcctc gctggcaatc gcgcgtttgg aaaaccatta ttttgtcaac ggcggttggt 942540
tgcagggcga tagggcgatt ttgaacaata tcggcaaaat acggcatatc ccgactatta 942600
tcgtacaggg gcggtatgat ttgtgtacgc cgatgcagag tgcgtgggcg ctgtcgaaag 942660
cctttcccga agcggaattg agggtggttc aggcagggca tcgtgcgttc gatccgcctt 942720
tggtggatgc gttggttcag gcagttgagg atattttgcc ccatttgttg taaaaagttc 942780
cgcataaaaa agcagcttct gtttggaagc tgcttttgtt ttgaatggtt taacgcagtt 942840
cggaatggag tttgcccaat aatgcggatg cgtctttgcc ggcatatgcg ctgccgtctt 942900
tgttgagcag gacgatgcgc gagccgttgg cgacaggttc tgcatagaca atcagttccg 942960
gctgttcggc aggtttctcc gctttgcctt tgcccagcag gcgtttgaac aggccgggtt 943020
tttgttcggt aactgcattg ctttcgttcg gggctttttg aaccaggaag gcgtggcgtt 943080
cggtgttttg accgacgacg gtcagcccga tgcggtcgag ggcgagcacg gtgcgccgcc 943140
agtttctgcc gtagtcgcca aagacaatca ggcttttgcc ttcgatacgc gccatttcgt 943200
tggcggcggg aagggtaggt ttttttgccg atgcgttttc cgcctgctgt ccgtcaacgc 943260
ccaaatattg cataaagcgc gtcaggaaag cggcttcgag gttgggatcg gacggggagg 943320
gctgccatac ggtcgtgtct ttgtctttgc cgccgtacac ttctttcatg gctttgtggg 943380
cgaagaagat gtcggaaacg ccgtttttgc cctgttcgat acggacgatg aatttgtcgc 943440
gctcgccggt ggagtagatg ccgcccaagc cgactttgtc gaagaggcgg cgcaagctgt 943500
cttgggggat tttggcgcgg ttttccgccc actcggtttc catttgtccg atggcgggtt 943560
cttcggattt gatgtcgaag ccgttttcct gccaaaaggc tttcaggagc ggccagattt 943620
cggcaggaga cttgccgtcg acaacgagcc agcgttggct gccgtcgcgc tcgaggcgga 943680
cacctttgac gcttttcaat acttcggcat cggcaggctg ttggacggcg ggtgtgcggc 943740
gtttttccaa atcgctggcg cggacggcgc ccgaaccggc aggcaggcgg tagaggttgc 943800
cttggtcggg gttgttcaaa tcaggtggga cttcaagttt gatcaggcgg tgcgaccggc 943860
tttggtagtc gagcttgggc tgttcggttt tgctgccgga gcaggcggca agcccgatga 943920
gtgcgagcgc ggcaatgacg ggtttgatat gggtcatcgt gtcatcctgt gtgatggata 943980
ttaaagtgtt tgttgcgtta tgccgtccga acggttcgga cggcatggct atatttaaag 944040
ttgtcctgag gctttcaggg cggcgcggac ttttgcttgt ccgttttccg tcagcggaac 944100
gagcggcagg cggacgtgcg gttcgcatct gcccagggcg gataccgccc atttcggtgc 944160
ggcggggctg ggttcgcaga acatggtgtc gtaaatcgga atcagtcggt cgttgagttc 944220
gcgtgcaagg gcgatatcgc cttgaagcgc ggcgcggcac atatcggcaa agagcttggg 944280
cgcggcgttg gcggctacgg taatcacgcc gtgtccgccg cagagcatga acggcagggc 944340
ggtgtggtcg tcgccggaaa ggacgacgaa gccttcgggc gcgcggttga tgagttcgat 944400
gttgctgccg atgttgccgc tggcttcttt cacgccgacg atgttgggga tttcggcaag 944460
gcgcaggata gtgtcgttag tcatgctgac gacggtacgg ccgggcacgt tgtagataat 944520
catcggaatc gaagtggctt cggcgatggt tttgaaatgt tggtaaatgc cttcttggga 944580
gggcttgttg taatagggga cgacggagag ggtgtagtcc gccccggctt tttcggcggc 944640
ttgggaaagg gcgatggctt cgacggtgtt gtttgcccct gtgccggcga tgacggggac 944700
gcgtttggca acgtgtttga cgacggcttc gatgacggcg gtgtgttctt cgacggagag 944760
ggtggcggat tcgcctgtcg tgccgacggc aacgatgccg tccgtgccgt tttcaatgtg 944820
ccagtcgatt aagtcgcgga gttgttcgta atggatgctg ccgtcttgat tcatcggggt 944880
aatcagggca accaagctac cttgtaacat acagaacctt ttatcagttg tggtgtaggg 944940
gcggtaatgc ttccgattgt agcctacttt accgcaggtg tgaaatccgg cgggttgcag 945000
atgtggggcg tttgcgccga aaggtatggt ggaaattgat ttttcctgtt tgaaatcatt 945060
ttattatatt cgccggttta tgccggtgcc gtcggattta tagtggatta acaaaaacca 945120
gtacggtgtt gcctcgcctt agctcaaaga gaacgattct ctaaggtgct gaagcaccaa 945180

gtgaatcggt tccgtactat ttgtactgtc tgcggcttcg ccgccttgtc ctgatttttg 945240
ttaatccact ataaaatgtg gtaaacgtgt ggaccagacg gatgccgtct gaaatgcaaa 945300
ttgaagccgt gcggcagatt cgctacaatc cgcgcttgga tttttcaacc tttaaaataa 945360
ggaaatacaa tgagcggtca gttgggcaaa ggtgcggatg cgcctgattt ggtgtacggt 945420
ttggaagaca ggccgccgtt cggtaatgcg ctcttgagcg cggttaccca tcttttggcg 945480
atttttgtgc cgatgattac gcccgcgctg attgtgggcg gcgcgctgga attgccggtg 945540
gagatgacgg cgtatctcgt gtcgatggcg atggttgcgt cgggtgtcgg cacttatttg 945600
caggtcaacc gcttcgggcc ggtcggttcg gggatgctgt ccatccagtc ggtgaatttt 945660
tcgttcgtta ccgtgatgat tgcgctgggc gcggggatga aagagggcgg tttgactaag 945720
gatgcgatga tttcgacgct cttgggcgta tcgtttgtcg gcgcgttttt ggtgtgtttc 945780
tcggcgtggc ttctgccgta tttgaaaaaa gtgattacgc cgacggtcag cggcgtggtc 945840
gtgatgctca ttggtttgag tttggtacac gtcggcatta ccgatttcgg cggcggcttc 945900
ggcgcgaagg cggacggcac gttcggctcg atggaaaact tggggctggc atcgctggtg 945960
ttgctgattg tgttggtgtt caactgcatg aaaaacccgc tgttgcgcat gagcggcatt 946020
gcggtcgggc tgattgccgg ctatatcgtc gcgctgtttt tgggcaaggt ggattttcc 946080
gcgctgcaaa acctgccgct ggttacgctg cccgtaccgt ttaaatacgg ttttgctttc 946140
gactggcacg cgtttattgt ggcgggcgcg atttcttgt tgagcgtgtt tgaggcggtc 946200
ggcgatttaa ccgcgacggc aatggtgtcc gaccagccga ttgaaggcga ggaatacacc 946260
aaacgcctgc gcggcggcgt gttggctgac ggcttggtgt cggtgattgc gacggctttg 946320
ggttcgctgc cgctgacgac gtttgcgcaa aacaacggcg tgattcagat gaccggcgtg 946380
gcttcgcgcc atgtgggcaa atatattgcc gtgattttgg tgctgttggg tctgttcccc 946440
gttgtcggtc gcgcgtttac gacgattccg agtccggtgt tgggcggcgc gatggttttg 946500
atgttcggct taattgcgat tgcgggcgtg cggattttgg tcagtcacgg catccgcagg 946560
cgcgaagcgg tgattgcggc aacgtcggtc ggtttgggct tgggtgtcgc gtttgagccg 946620
gaagtgttta aaaacctgcc cgtcttgttc caaaactcta tttccgccgg cggcattacg 946680
gcagtcttgc tgaatttggt cttgcccgaa gataaaaccg aggcggcggt caagtttgat 946740
accgaccact tggaacactg attttgaaaa tgaatgccgt ctgaaacaga atccctgttt 946800
cagacggcat tgtttttgag gcttatactt tttcgttttt taatacgcgt tgtcggcgtg 946860
tttcacttaa taccattccg gcagacacgg agacgttcat gctttcgact gtgccgaaca 946920
tgggtataga caccagcatg tcgcaatgtt cgcgcgtgag gcggcgcata ccgtcgcctt 946980
cgttgcccat tacccacgcc gcgctgtcgg gcagattgca atggtaaagg tcggactcgc 947040
cgctcatatc ggtgccgata atccaaatgc cgtattcttt caattcgcgc agggtgcggg 947100
cgaggttggt tacggtgata taggggacgg tttccgccgc accgcaggcg actttgctga 947160
cggtggcgtt cagccccgcg cttttgtctt tcggtgcgat gacggcgtgt acgcccattg 947220
cgtcggcggt acgcaggcac gcgccgaggt tgtgcggatc ggtgatgccg tcgagtatca 947280
gcagcagcgg cggttcgctg aggttttcca atacgtcttc gaggtggacg tggtttttgg 947340
aggcatcgat aaatccgacc acgccctgat ggcgcgcgcc tttgctgatg gcgttgaggc 947400
ggtcggcatc ggcaaaatat acgcggatgt tttcgtttgc cgccttttcc aacacttcgc 947460
gcgtgcgtgc gtctgatttg ccttcttgga tgtagagttc gacgatggat ttggggtttt 947520
gccacaatcg ggcgttgacg gcgtggaagc cgtagatggg tctttggttt gccatgatgg 947580
tgctttgtaa aaagggttca gacagcatta tagcaatttg ccggtatgcc gtctgaaagg 947640
gttaaaacag gtaggcgatg tatttcacca acaggataaa caagatggat acggcgcagc 947700
cgattttgaa cgccgtgccg acgacaagcc ccaacagcgt acccaagccc gctttacctg 947760
cctgaagcat attgcgccgt tcgatcagtt cgcctgccgc cgcgccgata aagggaccga 947820
gtattagtcc gggaagggag aaaaatatgc cgatgatgct gccggccaat gcgccgcgaa 947880
cggcgagctt gcccgctccg gtatattttg tcccccatat gcctgccaca tagtccgcca 947940
gtatgccggc aaggctgatg agtccgaccg tccacaaaac gcccgcgccg tagatttggt 948000
agccgccggc ataggcaagc agccatgttc cggcaaacat caatgccaat ccgggcaggg 948060
cggggtaaac gatgcccgcc gtgccgacgg ctatcagggc gagggcgagg atgacggtca 948120
gtacggtcat aggttcaacc ttttcttttg ttttgaaaaa aacggcttaa cacggcgcgg 948180
cattcttctt gcaggattcc gccccgtatg gcggtgtgcg tattgaggcg tttgtcggca 948240
aacaggttga cgatgctgcc tgccgcgccg gttttgggtt ctgccgcccc gtagatcaca 948300
cgcctgattc gtgcctgtat cagtgcggac gcgcacatgg cgcagggttc gagggtgata 948360
tatatgtcgc atccgtcaag gcggtagttt tgtatttctc tgcctgcctg tgccaaggcg 948420
ttgatttcgg cgtgtcggct gacattgcag tcggcaatgc aggtgttgtg tgccgatgcg 948480
atgattttgc cgtctgaaac gatgactgcc ccgacgggta tttcgccgtc ggcggaggat 948540
tgttctgctt ggcgcagtgc ttcgcacatg aagtgttcca tttcttcctg cggcggaaag 948600
gcggcgacgg gcggatggtt ttttaactcg gcaagcaggc gggctttatg cgcttgggac 948660
atttcttgcg gcggcgtgcc gtccagcagc gactcgagtt gccacagtgt gcttttcgtg 948720
agggtcaaac ccgatgcttt gagcagcaga aaggctttga ccgaaccgtt ttgccgcagt 948780
tcttcgagtg tacggatacc gagcctgtgc agggcggcga cggttttggg ggcgagcggc 948840

ggtgtggtca gcatggttta tgcgccgaaa aaccgttttg ccgcctcaat caggcgtgtg 948900
catgaagtgc agtctgaaaa cgggtcggca acgcagtcta aaggtgtttt gcgcaaccaa 948960
gtcagttggc gtttggcaag ttggcgggtg gcggcaatgc ctttctcgac aaatgccggg 949020
aaatcggttt ttccatccag atatttccat gcctgacggt agccgacgca gcggatggcg 949080
ggggagtagg cggtcaggcc gggatagcgg cggcgcaggt tttctacttc gccgataaag 949140
ccctgttcaa gcatcaggtg gaaacgcagg gcgatgtttt catgcaggcg ggcacggttt 949200
tcgggaatca gggcggcggt atgcaaatca aaagggagcg tatgggaggt caggctgccg 949260
agatgtgtgc tcatcggttt gccggttaaa taataaactt ccaaagcgcg tccgatacgc 949320
tggctgtcgt tcggtttcag acggcatgcg gtttcagggt cgactttttg cagggtgcgg 949380
tagaggaaat ccaagccgta catctgtttt tgttcgtcca agtcggcacg caggcaggcg 949440
tcggcttcgg gcaaatcgtt caaaccttgg gtcagggcgc ggaaatacat catcgtgccg 949500
ccgacaataa gggcaaacct gccgcgtgag gaaatttccc cgaccaagcg cgtgcagtct 949560
tcgacaaagc gggcggcgct gtatgattcg gtaggcggga tgatgtcgat aaggtggtgc 949620
gggacaaagg cgcgttcgga ggcggacggt ttcgccgtgc cgatgtccat atcgcggtaa 949680
accagcgcgg aatcgaggct gatgatttcg acaggcaggg tttcggcaat tttgagggcg 949740
agcgcggttt tgcctccggc ggtcggcccg agcagggcaa aggctttcgg ggtcggcata 949800
acgtttcagg tttggaaaaa tacggattat agcggaaagc gtgccgacgt tatattttgg 949860
tttgcggaag cacgccgacg gcaagggggc gtgtttaccg tatgccttta tatagtggat 949920
taacaaaaac cagtacggtg ttgcctcgcc ttagctcaaa gagaacgatt ctctaaggtg 949980
ctgaagcacc gagtgaatcg gttccgtact atctgtactg tctgcggctt cgtcgccttg 950040
tcctgatttt tgttaatcca ctataaaatt tcaaaccgac gcgccgggtt ttcaatatgc 950100
ccgcgcccga tgccgccttg tccgcaggca tcagcggcag tgtccgattt tttggggaat 950160
gcccgtcccg ggcgtattta aaggttcggc ggtgcggcgt tttcctgcgg caaggcttca 950220
gacggcatct ctggtgcgtc cgttagacaa ggcgtgcgct tggggcgata atggcgtttt 950280
gctttttttga aagccttgca atgtcccgaa acctgcttgt ccgctggctt gccgtctgcc 950340
tcatcccgtt ggcgacgctt gccgttttcg ccgccaatcc gcccgaagac aaactccagc 950400
atctgatcaa cggcatcatc cttgcctgcg aagcgacgtt tttgtttaaa ttcgtccttt 950460
tcgacaccat caagcatcat ttgaaacaag agtttgattt gaaacgtcaa actatgttgc 950520
tgtttattcc gattattttg ctgattgtgt atttgttcca ctattttggc gcgttttagc 950580
ccgtttccgt tatttctatg aatactcctc cttttgtctg ttggattttt tgcaaggtca 950640
tcgacaattt cggcgacatc ggcgtttcgt ggcggctcgc ccgtgttttg caccgcgaac 950700
tcggttggca ggtgcatttg tggacggacg atgtgtccgc cttgcgtgcg ctttgccctg 950760
atttgcccga tgttccctgc gttcatcagg atattcatgt ccgcacttgg cattccgatg 950820
cggcagatat tgataccgcg cctgttcccg atgtcgtcat cgaaactttt gcctgcgacc 950880
tgcccgaaaa tgtgctgcac attatccgcc gacacaagcc gctttggctg aattgggaat 950940
atttgagcgc ggaggaaagc aatgaaaggc tgcatctgat gccttcgccg caggagggtg 951000
ttcaaaaata tttttggttt atgggtttca gcgaaaaaag cggcgggttg atacgcgaac 951060
gtgattactg cgaagccgtc cgtttcgata ctgaagccct gcgagagcgg ctgatgctgc 951120
ccgaaaaaaa cgcctccgaa tggctgcttt tcggctatcg gagcgatgtt tgggcaaagt 951180
ggctggaaat gtggcgacag gcaggcagcc cgatgacact gttgctggcg gggacgcaaa 951240
tcatcgacag cctcaaacaa agcggcgtta ttccgcaaga tgccctgcaa aacgacggcg 951300
atgtttttca gacggcatcc gtccgcctcg tcaaaatccc tttcgtgccg caacaggact 951360
tcgaccaact gctgcacctt gccgactgcg ccgtcatccg cggcgaagac agtttcgtgc 951420
gcgcccagct tgcgggcaaa cccttctttt ggcacatcta cccgcaagac gagaatgtcc 951480
atctcgacaa actccacgcc ttttgggata aggcacacgg tttctacacg cccgaaaccg 951540
tgtcggcaca ccgccgtctt tcggacgacc tcaacggcgg agaggcttta tccgcaacac 951600
aacgcctcga atgttggcaa accctgcaac aacatcaaaa cggctggcgg caaggcgcgg 951660
aggattggag ccgttatctt ttcgggcagc cgtcagctcc tgaaaaactc gctgcctttg 951720
tttcaaagca tcaaaaaata cgctagaata gcgcgtttta cgacaaccga tttgattgga 951780
aaatcacaat gaaaacagca caagaactgc gcgccggcaa tgtatttatg gtcggcaacg 951840
atcctatggt cgttcaaaaa accgaataca tcaaaggcgg ccgctcttcc gccaaagtca 951900
gcatgaaact gaaaaacctg ctgaccggcg cggcttccga aaccatttac aaagccgacg 951960
acaaattcga cgtggtcatc ctgtcccgca aaaactgtac gtacagctac tttgccgacc 952020
cgatgtacgt ctttatggac gaagaattca accaatacga aatcgaagct gacaacatcg 952080
gcgacgcgtt gaaattcatc gttgacggta tggaagacca atgcgaagta accttctacg 952140
aaggcaaccc tatctccgta gaactgccca ccatcatcgt gcgcgaagtc gagtacaccg 952200
agcctgccgt caaaggcgat acttccggca aagtgatgaa aaccgcccgc ctggtcggtg 952260
gcaccgaaat ccaagtgatg tcttacatcg aaaacggcga taaagtcgaa atcgacaccc 952320
gcaccggcga attccgcaaa cgcgcctgat ttgccgcatt gaaaaatgcc gtctgaaaac 952380
gtttcagacg gcattttttt tatattcgcc ccgtgtttgg attgaagtag atgttttttt 952440
cgtaaacgac aatacgcgtg attttgccat tttcgtcaaa atggatgtct aaaaacggtt 952500

tgtcgggatt gcgttcacgg ttggacagcc ggaagagtga ttggttttca agcatttctc 952560
cgtgtatttt cagatagccg ggcaattggc tgatgtattg gaaattgccg ccaagtccgt 952620
tgttgtgccg gctcgaatag gcaggggttg ccgaaacttc aaaataacgc gtgcgtttcg 952680
gctcctcgcc ttgtccgcgg gtttcttgac tgccctgtat cagcaacagc gtcgcttctc 952740
ggaggcggcg ttcctgttcc atcaaggcat tttgtgcctg ccggctgatc gtgcgctttc 952800
cgtaacgcaa gtcttcaatt gccagtatga ttttctgatg gtagtcggga tcttcagggc 952860
ggatgtcggg aaacagcagc cgctgtatgt tgtcatagcc accgtcgggt aagccgaaac 952920
gggtttccag ttcgtgatgg gaaagggcaa acagacagtc cgagtcaatg tgttcggcga 952980
tttgctgcat gagatcgtca atgccggtgg cggggtgcag gctggtgcaa tttgacggcg 953040
gcgcgctttc tgcctgcttg acgttatcag gggcggatgc ggtcggttgc aaatcggttt 953100
gggaaggagc tgatgacgcg gaggacgaag cagcggatgc accgacttct ttggttttat 953160
cgtctttgct cggagaacac gcggtcagca tgattgcggt aagccataag agaagtgatg 953220
aggttttgtt tttcattcta ttgtttccag tattaaagag gccgtctgaa aacctaccgt 953280
ttcatttttc agacggcctg ttgttaatag aaccgaagaa cctgttaatg ccgacaaggt 953340
tctcaacctg tcttacccga cgcggtaaaa cgccaggctg cccaaaaggt tggggaaatg 953400
tttgacctgt ctgttgcccg tcatcacggc gcgctcgagg atgcggatgt tgtttttggc 953460
gcacaataaa tcaaagtctt tgagcgtgca ccaatggata ttgggcgtgt cgtaccaatg 953520
gtagggcata cgttcggaaa ccggcatatg tccgccgagt gcgatttgga cgcggttgcg 953580
ccagtagccg aaattcggga agctgacaat cgcctgtttg gcaacgcgca tcaggcagcg 953640
caggattttt tcggtattct gcatcgcttg gatggtttgg ctcaacacaa tcacatcaaa 953700
actttgatcg ttgaatgcgg ttaaaccttc ttccaaatcg gcttggataa catttacgcc 953760
gcgcgacatc gcggcgatga cgctatttgt gtcgatttcg atgccgtagc cgctgcattt 953820
tttgtgttcg accaatgcgg caagcagttc gccgtcgccg cagcccaagt ccaagacgcg 953880
gctgccttcg ggtatccggt cgtaaatcag ttgcaaatca tcgcgcaggt tcattgctga 953940
cattccttat aaacgttgtt catataggcg gcgaccgcac gcatataggc ttcgtcttcc 954000
attaaaaagg catcgtgccc gtgtgcggat ttgacttcga tatactgcac ggatttttgg 954060
gcggcaatca gtgccttgac cagttcgtgc gaacgttcgg gtgcgaaacg ccagtcggtg 954120
ctgaagctgg cgacaaagaa tttcgctttc acattttgca gggcgcgggt caggctgtcg 954180
ccgaaatctg ccgccggatc gaaatagtcc aaagccttgg tcatgagcag gtaagtgttg 954240
gcgtcgaacc gtccgacgaa tttgtcgccc tgatagcgaa gataggattc cacttcaaat 954300
tcaacaccaa agccgtattg ataaccgttg gaacgcaaat cgcgtccgaa ttttttgcct 954360
aaaccgtctt cggcaagata agtgatgtgt cccatcatgc gggcaatccg caagccccgt 954420
gcaggaacgg tattgtggct gcggtaatgt ccttcattga aatcagggtc ggtcaaaatt 954480
gcctgacgcg ccacatcgtt aaacgcgata ttttgcgtgg acagtttcgg cgcagacgca 954540
atcactaaag catggcgcac gcgctcggga taggaaatcg tccactgcaa ggcctgcata 954600
ccgcccaagc tgccaccgac aatcgccgcc cattgttcga taccgagata gtcggcaagc 954660
gcggcttggg atttaccca gtccttcacc gtaaccaccg gaaaatccgc gccgtattcc 954720
ctgcccgttt caggattaat cgacaaaggc ccgctgctgc cgtcgcagcc gcccagatta 954780
ttcaaaccga ccacgaaaaa acgttccgta tcaatcggtt tgccaggtcc gaccatattg 954840
tcccaccagc ccgtatattt atcttccgcc gaatgcctgc ccgcaacatg atggttgccc 954900
gacagcgcgt ggcagattaa aaccgcattg tttttttcag cattcagctc gccgtaggtt 954960
tcaatcatca gatcgaaacg cggcaaagtt ttaccgtttt ccaaaaccag cggcatctca 955020
aacggaattt tttggggcat tacaatgccc accgaggcat tttgactcat atcctgttcc 955080
aacaaatgcg gcgaaaagcg ttattatatc gcaaacggca tgacttttttg acacggtcgg 955140
acaagcagcc ggacgcgttt gaccctcatc cgccgcacac gaatcatact ttttcagacg 955200
acctccaccg cttcccgaca tgataggcag acttttccgt attttttct ttttcgcact 955260
tgccgcgttg attatcaacc gccttttcag ccgcaggcaa aaacgcgccc tgcgcgaagt 955320
cgccgaaatc agcgcatggg tactgctcgg tgcagccgcc gcgatgctgt tttggtatct 955380
gtttatgctg tatttcaaac acattccgga ttcgtattga cggaaaaaat gccgtctgaa 955440
acgcattttt ctgtttcaga cggcatattt gatgaaaagg gcttgcggta ggaggtgctt 955500
tatagtggat taactttaaa ccagtacggc gttgcctcgc cttgccgtac tatttgtact 955560
gtctgcggct tcgtcgcctt gtcctgattt ttgttaatcc actatacaac cgaagcagga 955620
agggcagggg gtcagcgttg gcgcgcttta aaacgcggat tgcttttgca gatgacgtaa 955680
actttgcccc tgcgcctgac gatttggcag tcgcggtggc gttgtttggc ggttttgagt 955740
gaagacagaa cctgcattat ttgtcctttc taaacgatga cattacggat tggaaacgtt 955800
ggttgaattt gctggcacgg ccttcggtgt tgacgttgcg ctgtttgcca gtatagacgg 955860
gatgggatgc ggaagaagta tccagcgaaa acagcggata ttctttgccg tctgtccaaa 955920
ccatcgtttt tccgtgtgtt tcggcacagg agcggattaa ccagccttca ttggcgctgc 955980
tatcgaaaaa aaggacggtt cggtaattgt cgggatgaat attcggtttc atatattgcc 956040
ttgctttcag tgttataaca taacaaactc tagcatagtt tagaagggct gtacaaggaa 956100
atttaactat ttttgtaata tattagaaat tttcatgata aatctgaaaa ttttgaaatt 956160

gactcatgtt tggcgcaact ttattatgtt gcctgaaaca tcatataaaa gataataaaa 956220
ggtacgcagc catgaattac gcaaaagaaa tcaatgcgtt aaataacagc ctttccgatt 956280
tgaaaggcga catcaacgtt tcattcgaat ttttcccgcc gaaaaacgaa caaatggaaa 956340
ccatgctgtg ggattccatc catcgcctgc aaaccttgca cccgaaattt gtttccgtaa 956400
cttacggtgc aaactcaggc gagcgcgacc gcacacacgg catcgtcaaa cgcatcaaac 956460
aggaaaccgg cttggaagcc gcgcctcacc tgaccggtat cgacgcttct cccgacgaat 956520
tgcgccaaat tgccaaagat tattgggaca gcggcatccg ccgcattgtc gccctgcgcg 956580
gagacgagcc ggccggttat gagaaaaaac cgttttacgc cgaagacttg gttaagctat 956640
tacgctccgt cgccgacttc gacatctctg tagcagcata tcccgaagtg catcccgaag 956700
cgaaatccgc acaagccgac ctgattaatt tgaaacgcaa aatcgatgcg ggcgcgaacc 956760
acgtcatcac ccaattcttc ttcgatgtgg aacgctacct gcgcttccgc gaccgctgcg 956820
tgatgttggg tatcgatgtg gaaatcgtcc ccggtatttt gcctgttacc aacttcaagc 956880
agctcggtaa aatggctcaa gtaaccaacg tcaaaatccc aagctggctg tcgcaaatgt 956940
atgaaggttt ggacgacgac caaggtacgc gcaatctggt ggcggcaagt atcgccatcg 957000
atatggtcaa agtcctgtcc cgcgaaggcg tgaaagattt ccacttctat acgcttaacc 957060
gcagcgagct gacttacgcc atttgccata ttttaggcgt gcgcccttaa agccgtatca 957120
aacagtttca gacggcatct aaggtgtcta aaaagcaaaa caccgcccca tccgagccat 957180
tctgatttac aataccggcc gattcggatt gaaccggtcc ttacaaaatc caactggaga 957240
gttcaacatg acaacattac atttctcagg cttcccgcgt gtcggcgcct tccgcgaatt 957300
gaaattcgca caagaaaaat actggcgcaa agaaatcagc gagcaagaat tgctggctgt 957360
tgctaaagac ttgcgcgaga aaaactggaa acaccaggtc gctgccaacg ccgatttcgt 957420
tgccgtaggc gatttcactt tctacgacca catcctcgac ctgcaagtcg ccaccggcgc 957480
gattcccgcc cgcttcggct tcgacagcca aaacctgtct ttggaacaat tcttccaact 957540
ggcgcgcggt aacaaagacc aattcgctat cgaaatgacc aaatggttcg acaccaacta 957600
ccactacttg gtgcctgaat tccacgccga taccgaattc aaagccaatg ccaaacacta 957660
tgttcaacaa ctgcaagaag cccaagccct cggtctgaaa gccaaaccga ccgttgtagg 957720
tccgttgact ttcctgtggg tgggtaaaga aaaaggcgcc gtcgaattcg accgtctgag 957780
cctgttgcct aaactgttgc ctgtttacgt tgaaatcctg actgctttgg ttgaagccgg 957840
tgccgagtgg attcaaatcg acgagcctgc tttggctgtc gatttgccta aagaatgggt 957900
ggaagcctac aaagacgttt acgctacttt gagcaaagta agtgccaaaa tcctgttgag 957960
cacttacttc ggttctgttg ccgaacacgc cgcattgttg aaagccctgc ctgttgacgg 958020
tctgcacatc gacttggtac gcgcccccga gcaactggac gcgttcgccg actacgacaa 958080
agtcctgtct gccggcgtga ttgacggccg caacatttgg cgcgccaacc tgaacaaagt 958140
tttggaaact gtcgagcctc tgcaagccaa actgggtgac cgtttgtgga tttccagctc 958200
ttgctcgctg ctgcacactc catttgactt gtcagttgaa gaaaaactga aagccaacaa 958260
acccgacctg tactcttggt tggcattcac cctgcaaaaa acccaagaat tgcgcgttct 958320
gaaagctgca ttgaacgaag gccgtgattc tgttgccgaa gaactcgccg ccagccaagc 958380
tgctgccgac tcccgtgcca acagcagcga aatccatcgt gcagacgttg ccaaacgcct 958440
ggccgatttg cctgccaacg cagaccaacg caaatctcca tttgccgacc gtatcaaagc 958500
gcaacaagca tggttgaacc tacctctgct accgactacc aacatcggtt ctttcccgca 958560
aaccaccgaa atccgccagg cacgctcagc cttcaaaaaa ggcgaactgt ctgccgccga 958620
ttacgaagcc gcgatgaaaa aagaaatcgc cttggtggtt gaagagcaag aaaaactgga 958680
cttggacgta ctggtacacg gcgaagccga gcgtaacgac atggttgaat acttcggcga 958740
attgttgagc ggttttgcat tcactcaata cggctgggta caaagctacg gctcacgctg 958800
cgtgaaacca ccgattatct ttggcgacgt aagccgtcct gaagccatga ccgtggcttg 958860
gtctacttac gcacaaagcc tgaccaaacg cccgatgaaa ggtatgttga ccggccctgt 958920
aaccattctg caatggtctt tcgtccgcaa cgacattcct cgctctaccg tgtgcaaaca 958980
aatcgcactg gctctgaacg acgaagtatt ggatctggaa aagccggca tcaaagtcat 959040
ccaaattgac gaacctgcca tccgcgaagg cttgccgctg aaacgcgccg attgggatgc 959100
ctacctgaac tgggcgggcg aatccttccg cctgtcctct gccggttgcg aagacagcac 959160
ccaaatccac actcatatgt gttactccga gttcaacgat atcctgcctg cgattgctgc 959220
aatggatgcg gacgtgatca ccatcgagac ttcacgttcc gacatggaac tcttgaccgc 959280
gttcggcgaa ttccaatacc cgaacgacat cggcccgggg gtttacgaca tccacagccc 959340
gcgcgtaccg acagaagccg aagtggagca cctgttgcgc aaagccatcg aggttgtacc 959400
ggttgaacgt ctgtgggtta acccggactg cggcctgaaa acacgcggct ggaaagaaac 959460
tctggaacaa ctccaagtaa tgatgaacgt aacccgaaaa ctgcgtgccg aattggcgaa 959520
ataagccgag accgtatgaa taaataccgt ctgaaagcct ttcagacggt attttgtcct 959580
gatttgcggc gcaagggcgc agttgccgga aaatcttttc attgcagctt gtttttttct 959640
aattcggctt tatatgtggg aaacaggcaa atcggagttg tgtttgatag ttttaaataa 959700
tttatattat ttgaactata aattatacaa atcattttgc atggggtaga atgcccagcg 959760
attcacaatt atttctcaaa ccaatctatt aaggagctta aaatggcttt gcaagatcgt 959820

accggtcaaa aagtaccttc cgtagtattc cgcacccgcg tcggcgacac ttggaaagat 959880
gtgtctaccg atgatttgtt caaaggcaaa aaagtagtcg tattctccct gcccggtgca 959940
tttaccccga cttgttcttc ttcacacctg ccgcgttaca acgaattgtt cggcgcgttc 960000
aaagaaaacg gcgttgacgc aatctactgc gtatctgtaa acgatacgtt cgtaatgaac 960060
gcttgggctg ccgaagaaga atccgacaac atctacatga ttcctgacgg caacggcgaa 960120
tttaccgaag gtatgggtat gctggtcggt aaagaagact tgggcttcgg taaacgctct 960180
tggcgttact ccatgctggt taacgacggc gtggttgaaa aaatgttcat cgaacctgaa 960240
gaaccgggcg atccgttcaa agtatccgat gcagatacta tgctgcaatt cgttgctccc 960300
gattggaagg ctcaagagtc tgtggcaatt ttcactaaac caggttgcca attctgcgct 960360
aaagccaaac aagctttgca agacaaaggt ttgtcttacg aagaaatcgt attgggcaaa 960420
gatgcaaccg tcacttccgt tcgcgccatt accggcaaga tgactgcccc tcaagtcttc 960480
atcggcggta aatacatcgg cggcagcgaa gatttggaag cttacttggc taaaaactga 960540
tagctgtttg cttaaggcgg tttaattaaa ctgtctgata taccggatag agttattcgg 960600
gcggttctac actaccgctc cgaataactc tatatttata agagaatttg gatattgttg 960660
cactcaatcg aaattttgtt tttatttatc tgaatgatgt ttttgattgg gaaaatattt 960720
aaatgccgtc tgaaaccgat atgttctgtg tcggcaatgt ttcagacgaa aacggaagga 960780
caaagattat gaaaaaaatt caagcggatg tcgtcgtaat cggcggcggt actgccggta 960840
tgggtgcgtt tcgcaatgcc cgtttacatt cggataatgt ttacctgatt gaaaacaatg 960900
tgttcggcac gacctgcgcg cgcgtgggct gtatgccttc caaactcttg attgccgccg 960960
cagaggcgcg tcatcacgca ttgcataccg acccgttcgg cgtgcatttg gacaaagaca 961020
gcatcgtcgt caacggtgaa gaggtcatgc agcgcgttaa atccgagcgt gaccgttttg 961080
tcggctttgt cgttgccgat gtggaagagt ggcctgccga caagcgcatt atgggttcgg 961140
ctaaatttat cgacgagcat accgtccaaa tcgacgagca tactcaaatt acggcaaaaa 961200
gtttcgtgat tgctaccggt tcgcgtcccg tcatcctgcc gcaatggcag tctttgggca 961260
atcgtttgat tatcaacgat gacgttttct catgggatac gctgcctaag cgcgttgccg 961320
tgttcgggcc gggtgttatc ggtttggaac tgggtcaggc attgcaccgt ttgggcgtga 961380
aagttgaaat tttcggtttg ggcggaatca tcggcggcat ttccgacccc gtcgtttcag 961440
acgaggcgaa cgccgtgttc ggcgaagaat tgaaactgca tctggatgct aaaaccgagg 961500
tcaaactcga tgcagacggc aatgtagaag tccattggga gcaggatggc gaaaaaggcg 961560
tatttgttgc cgaatatatg ctggcagccg tgggccgccg tccgaacgtt gacaatatcg 961620
gtttggaaaa tatcaatatc gaaaaagatg cgcgcggcgt acctgttgcc gacccgctga 961680
ccatgcagac cagtattccg catatcttca tcgcaggcga tgcgtccaac caactgcctc 961740
tgctgcatga agctgccgac caaggcaaga ttgccggcga taacgcgggc cgctacccga 961800
atatcggcgg cggtttgcgg cgcagcacca tcggcgtggt gtttaccagt ccgcaaatcg 961860
gctttgtcgg tctgaaatac gcgcaggttg ccgcgcaata ccaagccgac gaatttgtca 961920
tcggcgaagt atcgttcaaa aaccaaggcc gcagccgcgt gatgctggtg aacaaaggcc 961980
atatgcgcct gtatgccgaa aaagccaccg gccgctttat cggcgcggaa atcgtaggcc 962040
ctgccgccga acatttggcg cacctgttgg cttgggcaca tcaaatgaag atgaccgttc 962100
cgcaaatgct ggatatgccg ttctaccatc ccgttatcga ggaaggtctg cgtaccgcgt 962160
tgcgcgatgc cgatgcgaaa ttgaaagcct gaccgatatg gcaaaacaat gccgtctgaa 962220
attttttcag acggcatttt gtttttgggg atggggtcgg atgctgatac cgtgtcggga 962280
agggggcggc aaaactaaaa atctttcttt taatctgctg tttccacgcg tgtttgtcaa 962340
aatctatcag tttgttttta aaatacactg ttcaaaatgg gataaaacag gtaaattaac 962400
gtttatgtaa cccagtgtag caatgggttt acggtttttg agtcgatata taactacaga 962460
ggaattgact atgtctgcca aaccgcgtcc tgtttatctg gatttgccga acatccgtct 962520
gccgataccc gggatagttt ccatccttca ccgcatcagc ggggtcgggc tgtttattat 962580
gctgcctttc ctgctgtatt tcctgtccgg taccctgagt caagagtctg catttgaaac 962640
ttaccgtgcc attgtttccc atcctttggt caagctggtt ttaatcggtg tattgtgggc 962700
ttatctgcac cattctctcg ccggtatccg ctttttattt ttggatgcgc acaaaggcct 962760
tgagctgaat actgcgcgca ataccgctaa agccgtattt gcttctgcat tggttttgac 962820
tgtcgttttg ggagcgttgt tatggtagaa cgtaaattga ccggtgccca ttacggtttg 962880
cgcgattggg tgatgcaacg tgcgactgcg gttattatgt tgatttatac cgttgcactt 962940
ttagtggttc tattttccct gcctaaagaa tattcggcat ggcaggcatt ttttagtcaa 963000
acttgggtaa aagtatttac ccaagtgagc ttcatcgccg tattcttgca cgcttgggtg 963060
ggtatccgcg atttgtggat ggactatatc aaacccttcg gcgtgcgttt gtttttgcag 963120
gttgccacca tcgtttggct ggtcggctgt ctcgtgtatt cagttaaagt gatttggggg 963180
taagtatggg ttttcctgtt cgcaagtttg atgccgtgat tgtcggcggt ggtggtgcag 963240
gtttacgcgc agccctccaa ttatccaaat ccggtctgaa ttgtgccgtt ttgtctaaag 963300
tgttcccgac ccgttcgcat accgtagcgg cgcagggcgg tatttccgcc tctctgggta 963360
atgtgcagga agaccgttgg gactggcaca tgtacgatac cgtgaaaggt tccgactggt 963420
tgggcgacca agatgcgatt gagtttatgt gccgcgccgc gcctgaagcc gtaattgagt 963480

tggaacacat gggtatgcct tttgaccgtg tggaaagcgg taaaatttat cagcgtcctt 963540
tcggcggcca tactgccgaa cacggtaaac gcgcggtaga acgcgcctgt gcggttgccg 963600
accgtacagg tcatgcgatg ctgcatactt tgtaccaaca aaacgtccgt gccaatacgc 963660
aattctttgt ggaatggacg gcacaagatt tgattcgtga tgaaaacggc gatgtcgtcg 963720
gcgtaaccgc catggaaatg gaaaccggcg aagtttatat tttccacgct aaagctgtga 963780
tgtttgctac cggcggcggc ggtcgtattt atgcgtcttc taccaatgcc tatatgaata 963840
ccggcgatgg tttgggtatt tgtgcgcgtg caggtatccc gttggaagac atggaattct 963900
ggcaattcca cccgaccggc gtggcgggtg cgggcgtgtt gattaccgaa ggcgtacgcg 963960
gcgagggcgg tattctgttg aatgccgacg gcgaacgctt tatggaacgc tatgcgccga 964020
ccgtaaaaga cttggcttct cgcgacgttg tttcccgcgc gatggcgatg gaaatctacg 964080
aaggtcgcgg ctgcggtaaa aacaaagacc atgtcttact gaaaatcgac catatcggcg 964140
cagaaaaaat tatggaaaaa ctgccgggca tccgcgagat ttccattcag ttcgccggta 964200
tcgatccgat taaagacccg attcccgttg tgccgactac ccactatatg atgggcggca 964260
ttccgaccaa ttaccacggc gaagttgtcg ttccgcaagg tgaagattac gaagtgcctg 964320
taaaaggtct gtatgcggca ggtgagtgcg cttgtgcttc cgtacacggt gcgaaccgct 964380
tgggtaccaa ctccctgttg gacttggtgg tattcggtaa agctgccggc gacagcatga 964440
ttaaattcat caaagagcaa agcgactgga aacctttgcc tgctaatgca ggtgagttga 964500
cccgccaacg tatcgagcgt ttggacaacc aaaccgatgg tgaaaacgtt gatgcattgc 964560
gtcgcgaact gcaacgctct gtacaactgc acgccggcgt gttccgtact gatgagattc 964620
tgagcaaagg cgttcgagaa gtcatggcga ttgccgagcg tgtgaaacgt accgaaatca 964680
aagacaagag caaagtgtgg aataccgcgc gtatcgaggc tttggaattg gataacctga 964740
ttgaagtggc gaaagcgact ttggtgtctg ccgaagcacg taaagaatca cgcggtgcgc 964800
acgcttcaga cgaccatcct gagcgcgatg atgaaaactg gatgaaacat acgctgtacc 964860
attcagatat caataccttg tcctacaaac cggtgcacac caagcctttg agcgtggaat 964920
acatcaaacc ggccaagcgc gtttattgat gcgttttcag acagtcttcg cctcaaaggt 964980
cgtctgaaat ctaaccatac ccacattgaa ctgcttgaat ttataataca aaatcattgg 965040
gcagttgatg agaaaaggaa cacttctcat ggaaaaaatg agttttgaaa tttaccgtta 965100
caacccggat gttgatgcca agccttatat gcagcgttac gagttggaat tggaaccgac 965160
cgacgtgaaa cttttggatg ctttggtacg cctgaaagca caagacgata ccttgtcttt 965220
ccgccgctcc tgccgcgaag gcatttgcgg atcggacggt atgaacatca acggcaaaaa 965280
cggcttggcg tgtttgaccg atctgcgtgg cttgaaacag ccagttaaaa tccgtcctct 965340
gccaggtctg cctgttatcc gcgacctgat tgtggatatg acccagttct tcaaacaata 965400
ccattccgtc aaaccttatg ttgtcaacga taatccgatt gatgcggaca aagagcgtct 965460
gcaaactcag gaagagcgta aagagttgga cggtttgtac gagtgtattt tgtgcgcctg 965520
ctgttcgact gcctgcccgt cattttggtg gaaccctgat aaattcgtcg gtccgtccgg 965580
tttgctgaat gcttaccgtt tcattgcgga cagccgtgat accatcacta atgagcgttt 965640
ggataatctg aacgacccat accgtttgtt ccgttgccac accattatga actgcgtaga 965700
cgtatgtcct aaacacttga atccgacccg agccatcggt aagattaaag agattatgtt 965760
gaaacgggcc gtttaagaaa tgatggtttt tgacgatatt gccaaacgga aaatccgttt 965820
tcaaacccgc cggggattgt tggaattaga tttaatcttc ggcaggttta tggaaaaaga 965880
attcgagcat ttgagcgata aagagctgtc cgagttttcc gaaatccttg aatttcaaga 965940
tcaagaattg cttgccttga ttaacgggca ttcggaaacg gacaaagggc accttatccc 966000
gatgcttgaa aaaatcagac gggcatgagg catctgaaat ctgcctgcag gcagatttca 966060
aaatgcaaaa gccgtctgaa ggcaaagaac gtgctgcgga tgcagtaacg tgggttataa 966120
cttgcaaagg agcaataata tgtccaaatc aatcaaactc aacgtaccgg gtcaggcagg 966180
tttggagctg ccggtattgg aagccagcat cgggcacgat gtggttgaca ttcgggggct 966240
gacaaaaaat acaggtttgt tttccttcga ccccggattt gtttcaaccg caagctgtga 966300
gtctaaaatt acttacatcg acggcgatca aggcttgctt tattatcgcg gataccccat 966360
cgagcagctg gccgaaaagt ccgattattt ggaagtctgc tacctgttga tttacggcga 966420
actgccgact cccgagcaaa aggcagaatt tgacaatacc gtccgccgcc acacgatggt 966480
gcatgaacag ctgacttggt tcttccgggg gttccgccgc gacgcgcatc cgatggcgat 966540
gatggtcggc gtggtcggcg cactgtctgc gttctaccaa gacagcttgg acattagcaa 966600
tcccgaacac cgcaaaatcg cgatttaccg cctgatttct aaaatcccga ccattgcggc 966660
aatgtgctac cgctattcaa acggtctgcc gttcaattat ccgaagaata atctttctta 966720
ttccgaaaac ttccttcata tgatgttcgc cacgccgtgt gaagactaca aacccaatcc 966780
cgttttggca cgcgcgctcg accgcatctt tattttgcat gccgaccacg agcaaaacgc 966840
ctcaacttca accgtccgtc tggcagggtc ttcgggtgcg aacccgtttg cctgtattgc 966900
tgccggtatc gcctgcctgt ggggtgcttc gcacggcggt gcgaacgaag ccgtgttgaa 966960
aatgttggac gaaatcggcg atgtgtctaa tgttgccgca tacatggaag gtgtgaaaca 967020
acgcaaatac cgtctgatgg gcttcggtca ccgcgtgtac cgcaatatgg atccgcgtgc 967080
cagcattatg cgcgaaacct gctatgaagt tttgaaggaa ttgggcttgg aagacagtcc 967140

gaaattcaaa ctggcgatgg aattggaaca gattgcgctg aaagacccgt tctttatcga 967200
acgcaaactg tatccaaacg tcgatttcta ttccggcatc gtcctgtccg cgctgggcat 967260
cccgaccgaa atgtttaccg tcatcttcgc cctgtcgcgc agcgtgggct ggatttcgca 967320
ctggcacgag atgattagcg atccttcgct gaaaatcggc cgcccgcgcc agctttatac 967380
cggttcggaa cgccgcgatt atgtgccgcc aggcgagagg taacatacat taatatattg 967440
tcaaacaggc aatatcagag aaccggattg tttcccgaat ccgtctgatt gtagtcggat 967500
gaaatcaaga caagcaatcc ggtttaaaat agggtagaat aaaatgtctt ttcaggcggc 967560
atcagtttag ccgtcaggac gcggacttct accctttgtt tatattttaa agaaaagagc 967620
gcacgccatg atggacgaaa aactcaattt ctcttacctg ttcggttcaa acgcacctta 967680
cattgaggaa ttgtacgagg ctttttgga aaaccccgat gcggttgatg aaaaatggaa 967740
gcagtatttc accgatttga gcaaacagcc ggggacggtt gctgtcgatg tcgcacacac 967800
accgattcgc gaatcatttg ttactttggc gaaaaagaaa attgcatctg ccgttgcggg 967860
cggtgcggat gaggcaatgc tgaaaaagca agtcagcgtt ttacggctga tttccgccta 967920
tcgtatccaa ggcgtgggtg cagcccaact tgatccgctc aaacgtatcc ccccgcgcga 967980
tattgaagcc ctcgatccga aattccacgg tctgtcagat gccgatatgg cgcttcaatt 968040
caatatgggc gagggcgatt ttgccaatcg cggcaaactg cctttgtccc aaatcatcag 968100
caacctcaaa caaacctact gcggccacat cgcattggaa tatatctata ttcccaatac 968160
cgaagagcgc cgctgggtac gcaattattt tgaaagcgta ttgtccacac cgcattacaa 968220
tgccgatcaa aaacgccgta tcttgaaaga gatgactgct gccgagactt tggaacgtta 968280
tctgcatacc aaatatgtcg gtcagaaacg tttcggtgtc gaaggcggcg aaagcgcgat 968340
tgccggtttg aactacctga ttcaaaacgc cggtaaagac ggtgtggaag aggtcatcat 968400
cggtatggcg caccgtggcc gtctgaatgt tttggtgaac attttgggca aaaaacccgg 968460
cgatttgttt gccgaatttg aaggtcgtgc cgaaatcaaa ctgcccagcg gcgacgtgaa 968520
ataccatatg ggcttcagct ccgatattgc cacgccgcac ggcccgatgc acgtttcttt 968580
ggcgttcaac ccgtcacact tggaaatcgt caacccggtg gtggaaggtt ctgcgcgcgc 968640
caaacaaaaa cgtttgggcg aaaacggccg cgacaaagtc ttgccggtat tgattcacgg 968700
cgactccgca tttatcggtc tgggagtcaa ccaagcgaca ttcaacctgt ctaaaacgcg 968760
cggttatacc accggcggta cggttcatat cgtcatcaac aaccaaatcg gctttaccac 968820
ttccgatatc cgcgataccc gttcaaccgt acactgtacc gatatcgcaa aaatggtttc 968880
cgccccggtt atccatgtga acggcgatga tcccgaacgc gtttgctttg ctatccaagc 968940
cgctttggat taccgcaaaa aattccataa agacatcgtg attgacgttg tctgctaccg 969000
taaatggggt cacaacgagg gcgatgatcc gaccttgacc caaccgatga tgtacaaaaa 969060
agtatcgcaa caccccggtg cgcgtgcttt gtacaccgag caactgattg ccgaaggcgt 969120
ggtaacccaa gccgaggctg acggttacat ccaagcttac cgtgatgctt tggacaaagg 969180
cgagcacgtc gagcaaacaa cgttgagcaa cttccaacgc acgcaaatcg actggagcaa 969240
ataccaaggc aaagattggc gcgaacacat cgaaaccggt ttgcctgccg cagatattga 969300
acgtctcact gagaagttta ccgccgtacc ggaaggcttt gccctgcatc cgactgcaaa 969360
acgtgtgatt gaagcgcgta aagccatggc atccggcaaa caggccatag attggggtat 969420
ggccgaaacc ttggcatacg ccagcctcgt aaccaaaggt cacggcgtgc gtatttccgg 969480
cgaggactcg ggacgcggca cgttctcgca ccgccacgcc gtattgcacg atcaaaaacg 969540
cgaaaaatgg gacgacggta cttatgttcc tctgcgccat atgggcgaag gcatgggcga 969600
gttcctggtt atcgactcca ttttgaacga agaagccgtg atggcgttcg agtacggctt 969660
tgcctgctcc gcacctgaca aactgaccat ttgggaagct caattcggtg acttcgccaa 969720
cggcgcgcaa gtgactattg accaattcct gtcttcaggc gaaaccaagt ggggtcgttt 969780
gtgcggtctg actaccatcc tgccgcacgg ctacgacggt caaggccccg agcactcttc 969840
tgcacgcgta gaacgttggt tgcaactgtg ttctgagaac aatatgcaag tcattatgcc 969900
gtctgaagcg tcgcaaatgt tccacctctt gcaacgtcaa gtcttgggtt cataccgcaa 969960
accgctggtg attttcatgt ccaaacgcct gttgcgcttc aaaggtgcaa tgagcccgct 970020
ggaaaacttc accgaaggtt cgaccttccg tccggttatc ggcgataccg cagaacgcgc 970080
aagcaacgac agcgtgaaac gcgtggtatt gtgtgccggt caggtttact atgacttgga 970140
agccggccgt gccgagcgta aactggaaga tgatgttgct attgtccgcg ttgagcagct 970200
gtatccgttc ccatatgacg aggttaaagc tgaactggcg aaatatccga acgcaaaatc 970260
tgtggtttgg gcacaagaag agccgaaaaa ccaaggcgcg ttctaccaaa tccgccaccg 970320
catcgaagat gttattagcg aagagcaaaa actgtcttat gccggtcgtc caagcagcgc 970380
atcgcctgca gtgggctact caagcaaaca cattgctcaa ttgaaacaat tggttgaaga 970440
cgctttggca ttgtaaacca agtagcattc cgtctgagtc tgctcagatg gaatgcccat 970500
atgcagaatt aaaaacacac aacaggccgt ctgaaagggc cattggagac acaaaatgat 970560
tattgatgta aaagtaccta tgttgtctga aagcgtatct gaaggcacgc tcttggaatg 970620
gaagaaaaaa gttggcgaag ccgttgcccg tgacgaaatc ctgatcgata tcgaaacgga 970680
caaagtggtt ttggaagtac cttctccaca agccggcgta ttggttgaaa tcgtagctca 970740
agacggtgaa accgttgttg ccgaccaagt tttggcgcgc gtcgatacag ctgctactgc 970800

cgctgctgaa gccccagccg ccgctcctgc agaagctgcc ccagctgccg ctcctgctgc 970860
tacacaaaac aacgccgcta tgcctgctgc cgccaaactg gctgccgaga ccggtgttga 970920
cgtgaacgca ttgcaaggtt ccggccgtga cggtcgcgta ttgaaagaag acgtacaaaa 970980
tgccgctgcc aaacctgccg gagccgctgc tcctgctgtt gcacttcctg ccggcgcacg 971040
tcctgaagaa cgcgtaccaa tgagccgcct gcgtgcccgt gttgcagaac gcctcttggc 971100
ttctcaacaa gaaaacgcca ttctgactac attcaacgaa gtcaacatga aaccaatcat 971160
ggacttgcgt gcgaagtaca aagaaaaatt cgagaaagaa cacggcgtga aactgggctt 971220
tatgtccttc ttcgttaaag ccgctgttgc cgccctgaaa aaatacccgg ttgtgaatgc 971280
ttctgttgac ggcaaagaca tcgtgtacca cggctacttc gacatcggta tcgcaattgg 971340
cagcccacgc ggtttggttg tgccaattct gcgtgatgcc gaccaaatga gcattgccga 971400
catcgaacaa gcaattgttg attacgcgaa aaaagccaaa gacggcaaaa tcgctatcga 971460
agatctgacc ggcggtacat tcagtattac caacggcggt actttcggtt ctatgatgtc 971520
taccccgatc atcaacccac ctcaatctgc gattttgggt atgcacgcca ctaaagagcg 971580
cgctgtggtt gaaaacggcc aagttgttgt ccgtccgatg atgtatctgg ctctgtctta 971640
cgaccaccgt atcattgacg gccgcgaagc tgtattgacc ttggtagcca ttaaagacgc 971700
gttggaagac ccggcccgcc tgttgttgga tctgtaatcg tttcagacgg ccttttattt 971760
gttaatgaaa aggccgtctg aatttttata gtggattaaa tttaaaccag tacggcgttg 971820
cctcgccttg ccgtactatc tgtactgtct gcggcttcgt cgccttgtcc tgatttaaat 971880
ttaatccact atatttagat gtagcgtaat gtagtatcgt gctacaatag gctcaacgaa 971940
cgattgaggc cgtctgaaac atttgattcg aatgaatcgg cagatatgga ctttcagacg 972000
gccttttctt aaaaccatca aaacgcagtc attcaaaata aaaaagaaac aaaaagtatc 972060
gtttttattt tgagatactg ttaaaagcaa aggatgacac gatgtctcaa tatgatgtag 972120
tagtgattgg tgcaggcccg ggtggatacg ttgccgccat ccgtgccgcg caactgggtt 972180
tcaaaaccgc ttgcgtcgat gcaggcgtta acaaagcagg caatgcccct gcattgggcg 972240
gtacttgctt gaacgtaggc tgtatccctt ctaaagccct gttgcaatcc agcgaacatt 972300
tccacgctgc gcaacacgag tttgccgaac acggtatcac tgtcggcgac gtaaaattcg 972360
acgcggccaa aatgattgag cgcaaagatg ccatcgtgac caagctgacc ggcggcgtca 972420
aattcctgtt ccaaaaaaat aaagtaacca gcctgttcgg tacggcttcc tttgccggta 972480
aaaatggcga tgcttaccaa atcgaagtcg ataacaaagg cgagaaaacc gttatcgaag 972540
ccaaacacgt catcgtagcc accggttccg taccgcgtcc gctgccacaa gtcgctatcg 972600
acaatgtgaa cgtattggac aacgaaggtg cattgaacct gaccgaagta cctgccaaac 972660
tcggcgtgat cggttccggc gtgattggtt tggaaatggg ttccgtatgg aaccgcgtgg 972720
gtgcggaagt taccattctt gaagccgcgc cgactttcct ggctgccgcc gaccaacaaa 972780
tcgccaaaga agccttcaaa tacttcacca aagagcaagg tctgagcatc gaattgggcg 972840
tgaaaatcgg cgacatcaag tctgaaggca aaggtgtttc cgttgcttac gaaactgctg 972900
ctggcgaagc caaaaccgaa gtattcgaca aactgatcgt tgccatcggc cgtattccaa 972960
acaccaaagg cctgaacgcg gaagccgtag gcttggaaaa agacgagcgc ggctttatca 973020
aagtagatgg cgaatgccgt accaacctgc ctaacgtatg ggcaatcggc gacgtggttc 973080
gcggcccgat gttggcacac aaagccagcg acgaaggcgt tgccgttgcc gaacgcattg 973140
ccggtcaaaa accgcatatc gacttcaaca acgtaccgtt cgtgatttac accgatcctg 973200
aaatcgcttg ggtgggtaaa accgaagagc agctcaaagc cgaaggcgtg gagtacaaaa 973260
aaggtacttc aggttttggt gcgaatggtc gcgcattggc aatgggcaaa gccaaaggta 973320
cggttaaagt gttggcagat gccaaaaccg accgcatctt gggcgtacac atgattggtc 973380
cggttgtcag cgaattggtt accgaaggcg tgactgcgct cgaattcttc gccagcagcg 973440
aagacatcgc ccgcattatc catgcccacc caaccttgtc cgaagtggtt cacgaagctg 973500
cattggcggc cgacaaacgc gctttgcacg gttgatagac attaaggccg tctgaaattt 973560
ttcagacggc cttaaggcct tcgacaaatt gaatgttccg agagctccgt tttctgattt 973620
ataattccgt cagacaaaca aacagcattt acattcatta tgaacaaaga aatagtcggt 973680
attttcttta taccggcggg catcatcagc atgtgtatgg ccgcattgtg gcagatgtat 973740
gtgatgatga ccgaaactta tacgctcaac cgtttcaaag ataaagaatt ggtttggcgc 973800
gtggcattgt tgtttatcag tttcagcctt gccgtttatc tgctctgtcc gaattcgcgt 973860
aaaaaaggca tcgtcttttt tattctcggg ggaggcggtg cagccatgta tctgctggcg 973920
cggatgtggt tgcctttcag caagtgaaac gacgattttc cgaccgccga aaggtagtct 973980
gaaacgcacg ggcttgccat ttggaggcag actcggggca ttccactaat ctaaaggaga 974040
aatccatgaa tttacacgag tatcaggcta aagaactgct ggctagctac ggtttgcccg 974100
tacaaggcgg tattttggca cacaacggcg aagaagccgc tgcagcttac gacaaattgg 974160
gcggcaaatt cgctgttgtc aaagcacaag tacacgccgg cggccgcggt aaagcgggcg 974220
gcgtaaaagt cgttaaaagc cgcgaagaag ctaaagaagt ggctgaaagc ctgattggca 974280
ccaacttggt aacttaccaa accgatgcca acggccaacc tgtcaacagt gttttggttt 974340
gcgaagacat gtatccggtt caaaccgagc tgtacttggg cgcagtggtt gaccgttcta 974400
cccgccgcat tacattcatg gcctctaccg aaggcggcgt ggaaatcgaa aaagttgctg 974460

ccgaaactcc tgaaaaaatc ttcaaagtaa ccgttgatcc gctggtcggc ctgcaacctt 974520
gccaagctcg cgaagttgcg ttccaactgg gcttgaaaga caaacaaatc aacgagttcg 974580
tcaaactgat gaccggtgcg tacaaagcgt ttgtcgaaaa tgacttcgcc ctgtttgaag 974640
tcaacccgct ggcagttcgc gaaaacggcg cgctcgcctg cgtggacggc aaaatcggca 974700
tcgacagcaa cgcgctctac cgcctgccga aaatcgccga attgcgcgac aaatctcaag 974760
aaaacgaacg cgagttgaaa gcttctgaat ttgacctgaa ctatgttgcc ctggaaggca 974820
acatcggctg tatggtgaac ggtgccggtt tggcgatggc cactatggac atcatcaaac 974880
tgaaaggcgg ccaacctgcc aacttcttgg acgttggcgg cggcgcaacc aaagaccgcg 974940
tggttgaagc gttcaaactg attctggaag acaaatccgt tcaaggcgta ttgatcaaca 975000
tcttcggcgg tatcgtacgt tgcgacatga ttgcggaagc catcgtggca gccgttaaag 975060
aaatcaacgt caacgttcct gtcgttgttc gtttggaagg caacaacgcc gaactcggcg 975120
cgaaaatcct gaacgaatca ggtctgaaac tgacttctgc agacggcctg aatgacgcag 975180
ccgaaaaaat tgttgcagcc gtaaacgcct aaggagaaaa gaatgagcgt attgattaat 975240
aaagacacta aagtattggt tcaaggtttc accggtaaaa acggtacttt ccactccgaa 975300
caagctctgg cttacggcac taaagttgtc ggcggcgtta ccccgggcaa aggcggtcaa 975360
acccacctga acctgcccgt gttcaacacc atgaaagaag ccgttaaaga aaccggcgcg 975420
gatgcatccg tgatttacgt tcctgctccg tttgtgttgg attctatcgt tgaagcagtt 975480
gattcaggcg taggcttggt cgttgtgatt accgaaggcg tgccgacttt ggacatgctc 975540
aaagccaaac gctacttgga aaccaacggt aacggaacac gtttggtcgg ccctaactgc 975600
ccgggcgtga ttactccggg cgagtgcaaa atcggcatta tgccgggcca catccatact 975660
cccggccgca tcggcatcat ttcccgttcc ggtacattga cttacgaagc cgtggcacaa 975720
accaccaaac tgggcttggg tcaatcaacc tgtatcggta tcggcggcga cccgattccg 975780
ggtatgaacc aaatcgacgc actgaaactt ttccaagaag acccggatac cgacgccatc 975840
atcatgatcg gtgaaattgg cggtactgcg gaagaagaag cagccgaata catccaatcc 975900
aacgtaagca aacctgttgt cggctatatc gccggtgtta ccgcacctaa aggcaaacgc 975960
atgggtcacg ccggtgcgat tatctccggc ggcaaaggta ctgcggaaga aaaattcgcc 976020
gctttcgaaa aagccggtat cgcttacacc cgcagccctg ccgagttggg cactaccatg 976080
ctggaagtgt tgaaagcaaa aggtttggca taatcaggtt tgacaactga ttgaacatca 976140
aatgccgtct gaaaccggaa atcgggtttc agacggcatt ttgtttgtca tttcaaaaag 976200
aggcagcctc aacataccca cattattttt gcccttttgg ggcagtcaga gacctttgca 976260
aaattcccca aaatccccta aattccctaa attcccacca agacatttag gggattttgg 976320
ggaattttgc aaaggtctcg ggctaagtgt gcctgtttgc gcctaaaagg cggcccggat 976380
gcctgattat cgggtatcct gggaggatta agggggtatt ggggtaaaat tagtggatat 976440
ttgaaacgaa aacagccgaa aacctgtgtt tgggtttcgg ctgtcgggag ggaaaggaat 976500
tttgcaaagc tctcgtattg gctttgaagt tccgtgtaat tcacaggtag ggcgtgtggc 976560
acagccacgc acgcggtcgg ttgggtatgc aggctacggc tttctctgtt gaaactgcaa 976620

cacgtcagct accaaccgtg tgcgtagcgc acacggttgg ttgggataca agttactgtt 976680
tgttcttaaa tggagtacca acatcaaggg cttttgataa tcctgaaaat attaaatatt 976740
cagtttcagt ttttatttta ggagaaatat ttgcataatt tctatcttta aagcaccaat 976800
ggatatatgg tttcgtttca tcttctgggt tatctaaata agcaataaca ttcaagtcaa 976860
ataaaaattg caaaaactca ttagcagtac tcataaattt aggtatttcc actgatgttg 976920
tttgtaagtg ctttttcaaa cgttcaaatg cttttaaaaa atcactatat ttaaatctat 976980
ctttcccgtt taaaaattca aaaaatttca ggaaattttg ataatcactt tgactataat 977040
aaaacaaaag atgatctttg atttcaccaa gtaaatatat cgagtattct ctttgaaaag 977100
aagtattatc aaaatcttct gctacgacat aatcttcctt acttttctta tttttttgta 977160
gcaaagtaag catctgaaga atatcgcgag gtcgataata cgattttctt aggaagctaa 977220
taaatgaagt taaattttta tactcatcat gtaaattagg agcattccat ggaaaataat 977280
aatcccatga gttgcctttt tctaaactat cttgtttttc ttgctgggtt ctcaaaagat 977340
gatcaaaaac gccaaaaatc tttgaacttc tataagattt ataatccgtc ctccagtcta 977400
aaaatactga attatcttga agtttggtat tttgattttg taaacctaat gaatcaaaga 977460
tatcaggtct aatcaataac acaactctca tccttccctt actatcttta atggaaggga 977520
agatatcatt atttaacatc catatggcgt tagcaagacc ttttacacac tcatgatatt 977580
catcaaatgg aatctgtgat ggtctaatat ctatcccatc aataaacaaa atatgattat 977640
cttttagctt taactgagat aaagcatctt taaattttct ttcaataaaa cctaaatttg 977700
cttggaattt actttctgta aaagttattt gttgggattc ctcttcacct agtttaacaa 977760
attttccaaa aatcatttcc gcagcttctt ttgaattttc tattaaagtt attgcttgta 977820
caatttccgg atcaaaagcg ccataataat attcatttat agcctcatct aaggctttaa 977880
atttattaaa tattgaagat aatattccgt tttctttaca tttgatttga tttgatatca 977940
acagatataa aatgactttc caaatacttg taaaatctga aacagttaag tgtcttgctt 978000
tctttagctg aataaatttt gaataatcgg tttcacgaac aaacttagta gtggcatgta 978060

tgtttttata gaagttatta gttaaataaa cagcatatgc tgtctttcca gttccctttt 978120
ctccgattaa aaacgaaata tttggttcac ataattcatc caaatattct ccttttacaa 978180
atattcggtt aaataaatct ttattttctc ttcttctgta gtttgcagca tccacaaatc 978240
caaattctaa tgtttttaac ggtttcatct taataatctc ctatttaatt ttgaattaaa 978300
cttacctcaa aaccaccttc aaatacttcc cagtataact ccccttaact ttcgccacct 978360
gttcaggact acctttagca ataatcctcc ccccgccatc tccgccttcc ggccccaagt 978420
ccacaatcca atccgctgtt ttaatcacat ccagattatg ctcgataatc actatcgagt 978480
tgcctttgcc tttcagacgg cctatgactt ccagcagcag ggcgatgtcg gcgaagtgca 978540
ggccggtggt gggttcgtcg aggatgtaga gcgttctgcc ggtgtcgcgt ttggagagtt 978600
ccaaggcgag tttcacgcgt tgggcttcgc cgccggagag ggtggtggcg gactgtccga 978660
ggcggatata gcctaggcct acgtccatca gggtttgcag tttgcgcgat acggtgggga 978720
cggcgtcgaa aaattcgcgg gcttcttcga cggtcatgtc gaggacttgg ctgatgtttt 978780
tgcctttgta ttggatttcg agcgtttcgc ggttgtagcg tttgccgtgg cagacttcgc 978840
aggggacgta cacgtcgggc aggaagtgca tttcgacttt aatcacgccg tcgccttggc 978900
aggcttcgca gcggccgcct ttgacattga aggagaatct gccgacgttg tagccgcgtt 978960
cgcgagagag ggggacgccg gcgaagagtt cgcggatagg ggtgaacagg ccggtgtagg 979020
tggcggggtt ggagcgagga gtacggccga tgggggactg atcgacgttg atgactttgt 979080
cgaggtgttc gaggccgtgg atgtcgtcga atggggcggg ttcttcttgg gcgcggttga 979140
gttcgcgggc ggtaattttg gcgagggtgt cgttaatcag ggtggatttg ccgctgccgg 979200
acacgccggt gatgcaggta atcaaaccga gcggcagctc aagggtaacg tttttgagat 979260
tgttgccgcg tgcgcctttg aggacgagca tccggtcggg attgacgggc gtgcgttcag 979320
acggcacggc aatggatttt ttgccgctga ggtattgtcc ggtaacggag ttttcgcatt 979380
gggcgacgtt ttcgggcgtg tcggcaatca gtacgttgcc tccgtgttcg cctgcgccgg 979440
ggcccatatc gaccacgaaa tcggcttcgc ggatggcgtc ttcgtcgtgt tcgaccacaa 979500
tcacgctgtt gcccaaatcg cgcaggcgtt tgagggtggc cagcaggcgg tcattgtcac 979560
gctggtgcag gccgatggag ggttcgtcca gtacatacat cacgccggtc aggccgctgc 979620
cgatttggct ggcgaggcgg atgcgctggg cttcgccgcc ggagagggtt tcggcggagc 979680
ggcttaaatt caggtaatcc agcccgacgt taatcaggaa gccgaggcgt tcggtgattt 979740
ctttgaggat tttttcggcg atttgttttt tgttgccgtc taaatccagt gtttcaaaga 979800
attggtgggt tttggtgagc ggccaggcgg aaacttcgtg caacggctca ccgctgacgt 979860
aaacgtagcg ggcttctttg cgcaaacgtg cgccgccgca gcttgggcag gcgcggtggt 979920
tttggtattc gcgcagtttt tcgcgcacgg tttcgctgtc ggtttcgcgg tagcggcgtt 979980
cgagattggg gatgatgcct tcaaaggcgt ggctgcggtt gaaggtggtg ccgcgttcgg 980040
acaggtaagt gaaatcaatg acttctttgc ctgagccgtg cagcacaact tttttcactt 980100
tttcaggtag tgtttcccaa gcagcctgca catcgaaacc gtaatgccgc gccaatgatt 980160
gaatcatttg gaaatagaat tggttgcgct tgtcccaacc gtcaatcgca cctgttgcca 980220
gcgacaattc gggatgggcg accacttttt cggggtcgaa gaaattggtg ttgcccaagc 980280
cgtcgcaagt cgggcaggaa cccatcgggt tgttgaacga aaaaaggcga ggctctaatt 980340
cgggcaggct gtacgaacac acggggcagg cgaaacgtgc ggaaaaccaa tgttcttcgc 980400
cgctgtccat ctccatcgcc agcgcacgct cgttgccgtg gcgcagcgcg gtttcaaaac 980460
tttccgccag ccgctgcttg atgtccgcct tcactttcac gcggtcgatg accacgtcga 980520
tattgtgctt gatgtttttt tccagcttcg gcacttcgtc caactgatag acctcgccgt 980580
ccacgcgcac ccgcgcaaaa ccctgcgcct gcaagtcggc aaagaaatcg acaaactcgc 980640
ccttacgctc gcgcacggtg ggggcaagaa tcatcacacg cgtgtcttcc ggcagtttca 980700
atacggcatc gaccatctgc gatacggttt ggctcgacag cggcagcttg tgttcgggac 980760
aatacggggt accgacacgg gcgtataaaa gacgcagata gtcgtggatt tcagttaccg 980820
taccgacggt ggagcgtggg ttgtggctgg tggatttttg ctcgatggaa attgcaggcg 980880
acagaccttc aattaaatcg acatcgggtt tgtccatcat ctgcaaaaac tgccgcgcat 980940
aggcggaaag gctctcgaca taacgccgtt gcccttcggc atacagcgtg tcaaacgcca 981000
gcgacgactt gccgctgccc gacaatcctg ttaccaccac gagtttgtgg cgcggaatgt 981060
ctaaatcgat gtttttcaaa ttatgcgtgc gcgcgccgcg gatgcggatg gtgtcgttgt 981120
cgtgcgaatg ttggggatga tggttgcaca taatggatgc cgcctgaaaa ataaaggaaa 981180
accggtattg tagcactttc tcggatgccg tctgaagccg cgttcagacg gcatttgcca 981240
gcggagtacg gcagattccg ctataatgtc ggcaatttta acccgcttga acaaaaggat 981300
gacaaatgaa ccgtctttac ccccacccga ttatcgcccg tgagggctgg ccgattattg 981360
gcggcggttt ggctttgagc ctgctggtgt cgatatgttg cggctggtgg tctttgccgt 981420
tttgggtgtt taccgtattt gcattgcagt ttttccgcga ccctgcgcgt gagattccgc 981480
taaatcctga agcggtgttg agcccggttg acggccgtat cgtggtggtc gaacgcgcac 981540
gcgatccgta tcgtgatgtc gatgctttga aaatcagtat ttttatgaac gtgttcaacg 981600
tgcattcgca aaaatcgcct gccgattgta cggtaacgaa agtggtctat aacaaaggca 981660
aattcgtgaa tgcggatttg gacaaagcca gcacggaaaa cgaacgtaat gcggtgttgg 981720

cgactacggc ttcaggtcgt gaaattactt ttgttcaagt ggccggtttg gtggcgcgcc 981780
gtattttgtg ctacacccaa gcaggtgcga aactgtcccg cggcgaacgt tatggcttta 981840
tccgcttcgg ttcgcgcgtg gatatgtatc tgcctgtcga tgcgcaggcg caagtggcga 981900
ttggcgataa agtaaccggc gtcagcactg tattggcgcg tttgccgctg actgcgccgc 981960
aaactgaatc tgagcctgaa tctgagcctg ctttacaaac tgctccggtt gaaacagcgg 982020
caaacccatc tgccgaacaa cggcaaatcg aggcagcggc ggctaagatt caggcggctg 982080
tgcaagatgt gttgaaagat taattttgcg gactgaaata gaaaatatca gtaccatcat 982140
tcacacgaat gaggaagttt ggttttttga atttttgcta atgttcacac cgtcattccc 982200
acgaaagtgg gaatctagaa acttaacgtt acgacgattt atcggaaacg actgaaaccg 982260
gacggactgg attcccgcct gcgcgggaat gacgacttat tagttaccta acacttaaaa 982320
aacagaaacc tttccgcgtc attcccacga aagtgggaat ccgggaactt aacgttacag 982380
cgatttatcg gaaacggctg aaaccgaacg aattggattc ccgcctgcgc gggaatgaca 982440
actcattagt tacctaaaac ttaaaaaacg gaaaccttta cgccgtcatt cccacgaaag 982500
tgggaatccg ggaacttaac gttacagtga tttatcggaa acggctgaaa ccgaacgaat 982560
tggattcccg cctgcgcggg aatgacaact cattagttac ctaaaactta aaaaacagaa 982620
acctttacgc cgtcattccc acgaaagtgg gaatctagaa cccaaatgct aaggcgattt 982680
atcggaaacg gctgaaaccg aatgaattgg attcccgcct gcgcaggaat gacaactcat 982740
tagttaccta aaacttaaaa aacagaaacc tttacaccgt cattcccacg aaagtgggaa 982800
tctagaaccc aaatgctaag gcgatttatc ggaaacggct gaaaccgaat gaattggatt 982860
ctcgcctgcg cgggaatgac gacccattag ttacctaaaa tttaaaaaac agaaaccttt 982920
ccgcgtcatt cccatgaaag tgggaatcta gaacccaaat gctaaggcga tttatcggaa 982980
acggctgaaa ccgaacgaat tggattcccg cctgcgcggg aatgacggga tcttgggttt 983040
ctgcttttga tttttctgct tttgcgagaa tgacggcgtg aaagtaagaa tgatgaaaca 983100
aaaaaaatgg gaatgatggc atagtggttt gttctttgtc tttgccatat ttcctaacaa 983160
attgattaaa aagaaaaaag gttttcagaa tgccgtctga aaaccttttt tgtttgcctg 983220
tccgatttta aaacttcacg ttcacgccgc cggtaaagct gcggcccatt tgcggcgtat 983280
cagagagaaa gctgctgtgg gcgtaaacgg attggttgag caggttgtcg gctttgacgt 983340
accaattcca ctcgccatag cgcgtattgc ggcggtagtt tgcgccgagg ttgagcatat 983400
ggtgtccggg cgtgcgcgtt tcgtagcggg cgagtttgtt ttgggcgaac acgcggtagt 983460
agtccaaatt ggcatcgata cggtcggtca gcgaggcttt caggtggaag ccgaggcgcg 983520
cagccggaac acgggggggca ttttggtcgt cctgtgcgat gaaaggacgg ttgccgtagg 983580
catcttctct gccgggtagg gaaggcaggt ttttcagacg gcctcgtaca tagtcgccgg 983640
aaacgccgat gcggtagcgs ggtgtcggtt tgaagtagat ttcgccttcc gcgccgtaga 983700
agtcggcgcc ggattggttg tagcgcacga gcttcatttc gctgtcgtct tcgatggatt 983760
tggggccgcg tccgtcgttt aaggtttggg cgtaaatgta gttaccgaag cggttgcggt 983820
agagtgccag attgtattgc cagcggtcgc cttcgtagcc cagcgcgagt tcgatattgt 983880
tggaacgctc tttgttgagg tgtttgttgc cgacttcaaa ggtgttggtg gcgacgtgtt 983940
tgccgtgtgc gtacagctct tgcgttgacg gcaggcgttc ctgatgggag gcggtcaggc 984000
tgagtttgtg ttgtggcgtg aaataccagt tgcccgaaag tgcgaatgag cgggcggttt 984060
ggcggtgcgc gccgaggtcg ggcagggggt ggttgtagta gttttcccga tcaatcaatg 984120
ctttgtcgta ctgaatggag gctttttgtt tttccacgcg tacgcctcct tcaagcgtga 984180
agttgtccca gtttgcctgt tctacaccga aaaagctgta atgttgcact ttgttgtcaa 984240
gcagcatcgg ttgtttaacc gcttcggata tggcagataa agcactggat ttttgttgta 984300
aatattgcac gccccagctg cctttcagac gacctatggg ttggtggcgc aactcgatgc 984360
gggcgttttg cgtttggttg ttaaaaaagt tttcgactgc atcgcctgct ttttcgtcgt 984420
ggcggtagtc gttgcggttc aggtgtacgc gcagggcttc aaaaccgggg aacggttgct 984480
tccattcggc acggagttcg tagcgtttgt tgcgcaggtc tatccacggt ctgccgctgt 984540
gggtgtgtgc gtgtgcatta tcgtcgtcgt ggaagccgca gctcaagccc ggattgtcgt 984600
aatcgatgtc ttcttcggtc aacaggtgcg gataaagctg taaatagcgt ttgttaatca 984660
agctcttttg ccagatgatg tcggcgtggc aatcatcgta ttcgtggctg tgggcaggca 984720
gaccatattg gtcgcgacgg tcgctgtacg ctacgccgat aaaacctttt tcgccaaccc 984780
aagacagccc gatgctgccc gtttgcgaat cggcgtggct gtcgggcagg cgtttcagat 984840
tgcggtaacg cggtacggcg taatcccccg atttgcggta cagcccttcc gtgtgcaata 984900
caaagttttt gcccaaaccg atattgatgc cgccggacgt gagtttttcc agattgccgc 984960
tgctcaaacg caatccgagt tcgcccgata cgccgttttc aggcattttt tcggggattt 985020
tgccatcggc aacatcgacc agccccgcca cattgcccga gctgtacaag agcgtaaccg 985080
gcccgcgcag gatttcgacc tgttgcgaca aggcggtatc taccataatg gcgtgatcgg 985140
gcgaaaaatc cgccatatcg cctgtttcgc cgtgatggtt caacacttta ayccgcctgc 985200
ctgtttgacc gcgaatgacg ggagcagacg cgccgccgcc gtattgcgaa gcgtggatgc 985260
ccggtacgcc gtctaaagcg tcgcccaagt tgacggcttt ttggcgcaag gtatcgccgg 985320
agatgatttt gtcggaggcg gtcgaagtgt gcaacagccc cgacgtggcg cgcggacggc 985380

ttttgccgac gacgctgacc gtttccaaat ccaccgattg ctcagtttca tgcgcttggg 985440
cgaggagggg tgtgttgatt aaaagaattg ataaaacaat gggtttgagt gtagtttgtg 985500
ccattttggc ttctcgtcgc atttcaaaag tttgttatta tataacatta catttttttat 985560
atcataagat tttgagaaca ctcagagggc ataggcaaaa gtttttcaaa tgaaacggtt 985620
gcggcatcgg gcggtgtcca tttgtatccg ccgtccttcg gggcgcggt tgatgttgac 985680
gcagattccg ctgtgtttgc ccattatgtt cggctgcggg ataaccaaaa tttatgagtg 985740
cataaaaacg gcacgttccc gaacggtcga aaaggtggca aaatggcgta cttgtcagaa 985800
cgcgaggctc tgcgccaggt tcacgagggc gcatcgggca cgctaagata taagagggta 985860
tggatggggg tatcggaaat gcagttaata acaaacaaat tataaatcaa taggttaatc 985920
acaaaatgct tttgtttatc gacaattacg acagttttac ttacaacatc gtccagtatt 985980
tcactgaatt ggggcaggaa gttgccgtgc gccgcaacga tgatattacg ttggaggaaa 986040
tcgaggcatt gaatccgcaa tatctcgtta tcggccccgg cccgtgttcg cccaaagaag 986100
cggggatttc cgtggcggcg atgcgccatt tcgccggccg gctgccgatt atgggcgtgt 986160
gcctcgggca tcagacgata ggcgaggcgt tcggcggcag gatagtccgc gccaaaacgc 986220
tgatgcacgg taaggtgtcg cccgtgtccc attcgggcaa gggtatgttt aagggtttgc 986280
ccaatccggt tacctgtacg cgttatcaca gcctcgttat cgatcggaat acgatgcccg 986340
aatgtttgga agtaacggct tggactgagg acggcgagat tatgggtgtg cgccataagg 986400
aatatgccgt cgagggcgtg cagttccacc ccgaagccct cttgaccgag cacggacatg 986460
atatgttaaa caatttttta atcgaatttc aaaacttcaa accgcaaaaa atctgacgtg 986520
atgccgtctg aagcccttca gacggcattt tcgtccgaat attgaacgga ggacaaaaaa 986580
tgattacacc gcaacaggcc atcgaacgat taatcagcaa taacgagttg ttttacgatg 986640
aaatgaccga cttgatgcgt cagattatga gaggacaggt tctgccggag cagatagcgg 986700
ccattttgac aggattgcgt atcaaggttg aaaccgtttc cgaaattacc gcagctgcag 986760
ccgtcatgcg cgagtttgcg acaaaagtgc cgctggagaa tgcagagggg ctggtcgata 986820
tcgtcggtac gggcggggat ggcgcgaaaa ccttcaatat ttcgacgact tcgatgtttg 986880
ttgccgcagc ggcaggcgcg aaggttgcca aacacggagg ccggtcggtc tcttcctcca 986940
gcggtgcggc tgacgtggtg gagcagatgg gcgcaaacct caacctgact cccgaacagg 987000
ttgcccaaag tatcaggcag accggcatcg ggtttatgtt cgcgcccaat caccacagtg 987060
ccatgcgcca tgtcgcccct gtacgccgtt cgctcggttt ccgaagtatt ttcaacatat 987120
tgggtccgtt aacgaatcct gcgggcgcgc cgaaccagct tttgggcgtg ttccacaccg 987180
atttgtgcgg cattttgtcg cgggtcttgc aacaacttgg ttcaaaacac gttttggttg 987240
tttgcgggga gggcggtttg gatgaaatta cactgacggg caaaacacgc gttgccgagc 987300
tcaaagacgg aaaaatcagc gaatacgaca tccgcccaga agatttcggt atcgaaaccc 987360
gccgcaattt ggatgaaatc aaagttgcca atactcagga atctttgttg aaaatgaatg 987420
aggtgctgga aggaagagaa ggggctgcgc gcgatatcgt attgctcaac accgccgccg 987480
ccctgtatgc cggaaatgtc gctgcttcgc tttcagacgg catatctgcc gcacgggaag 987540
ccatcgattc aggcagggcc aaatcgaaaa aagaggagtt tgtcggtttt caaccacaac 987600
aaagatgcca ttttcttgga aagatggagc ttgggtgatg ccgccatgat tatggaactt 987660
ttgtggcaaa acataagcac ttcacgaaga gaacttacca aactgttttt atataaaaac 987720
ttggggctgt actagataac cagaccaaat tcccattaac taattgtctt aaaatctgaa 987780
tttgagattc tatttaaaat gccattggca tttctttaaa tgcagcccca aatgctcttt 987840
gggaatgccg ttaaacttac gtaaatggct aaattcacta atatcaagca catcataact 987900
acgaaaggta tccgtgtata caatgccatc aggcttaact ttctttcgga taattggcaa 987960
caatgtcgct gattgcgcat taggaacaac gacggtataa accttatata ttgcgtccct 988020
aagaagggac gattaacaaa aattaacgtc ctttactttc tacaagtaac agggcttttt 988080
tttgcccgtt tttgaggatt cgcaccatgg aagataagca agggatgaca aaggcggttg 988140
ccggcgtgat gacggacgcg ctagcggacg gcaggaagcc gacaaccgct tcaaatcttc 988200
ccccttatc taacaggggg ggacagaaac cgaaacggca ggcagggttc aggaagtctt 988260
cgaatgttac gaaacgtaca taacggacgg taaaggaaac ctgttaggcg ttcctcttcg 988320
gcgcggtgta tcagattcgg ctttcattga tcaaattagc ttttcatttc atgaaaaaac 988380
ctttttcgat aaatacggcg ttcgtgtaag tcttttggaa gacgaagatt ttattcgcgc 988440
cgcgtccatg ctcgccgaag aagttttcgg tttcggtatc tacaaagaat ccaaaggttc 988500
gggcggtcgt ttctatgagc gctgttggtt gatgggttcg gaagacgccc tatacggtcg 988560
cgtccatttt ggcggccaac aaaataccat tcttttcgaa ctgaccggca ccggttgcgg 988620
cgtcgcaaaa gaaggctggg aatcacgact tttcgcattc ctgactaatg caatccgccc 988680
aaaaatcaca cgcgttgaca tcgcaaaaga ctttttcaac ggcgaataca gcccgaacca 988740
agcccgtgaa gaccgaaata aaggtatgtt tacctgtcat cacgtcaaac caaaaggcga 988800
atgtttgggg tcagattggg aagaagacga tgaagccaaa atgaccaaag gcaagaccta 988860
tggtatcggc tcccgtgaat cgtccaaata tgtccgcgtc tatgaaaaag gcaagcagtt 988920
gggcgataaa acaagcacat ggacgcgatt tgaaattgaa ttcaaagcaa aagacatcgt 988980
tatccctttc gaagttttgc agaatccggg cgaatatttc ggcggcgcat atccgatttg 989040

cgaacgattc gcccaaaagg caacgcgcat acacgcggtt aaggaagata aggtcatttc 989100
agccgaccgc taccttgaat gggtaaaaaa acagttcgga cgtgcggcaa acggtctgaa 989160
attcattttt cccgaattgg acaaagccaa actgtttgaa ctgattgagc cgagtcatca 989220
caagctgccc aagtctttgg ctcccgaagc ctacgactgc gcctttttga aagctcaagc 989280
cattcatgaa cagcccgcat tcaaaccgta caaagaccct tactatatgt acgaatatta 989340
cgagaatctt gaaaaacagc ttgaacagca aaaacacgtc aacaatgaag aaagctataa 989400
caacttcatt tacgacaaat tcgcaagact accgatttca tgggcttaaa gtgtctgccc 989460
gaaagacgtt taatcacaca aggaaaccaa aaaatgaaca tccaacttca aggccacatc 989520
gtcggcgtta aaaaaatcaa cggacaaatc gaaggcaaga gcttcgacta ttgctgcctg 989580
attgtcgcca caccccttaga cagctcccaa ggcaacgcat tgggcagctc tactactgaa 989640
tacgatttcg gcggctctgc caatttcgag cagttccgaa acgcccaatt tccgatcgaa 989700
gcaaacctga acgtagaaat cgtcactacg ggcaaaaccc aaaaactgaa agtcatcggt 989760
tttcaactcg tgaagaaagg ctgattgaat gcagaaagtc tatgttgtcc agtccgtatc 989820
aacaggggac tttctgtatc tctctcctga aacgggcgac atcggacata ccaaattaat 989880
caccaatgcc gattatttct acgacttcga agaagcgatt aacgcaggtt tggaagaaat 989940
cggcaaccaa tacgaatttg tcgtattcgg atttttgaaa gactgatttt cggatgttcg 990000
gcggtcgtct gaaaaacgct ccatccatta ccgccaaaca ctttttgaag gaaaatatca 990060
tgaaatttat taacacctgc cgtaaatacg gcgcaaaact ggctgttgta acagctgctc 990120
ccctggcttt ggccgcacat gcaaatgcaa cgttgcccga tacggcaaaa aacgctttgg 990180
aagccgcaaa agcggacggt atggaagccg gttggattgt agtgggcatt ttcgccgcgc 990240
tttttgtatt ttccatcgtt aagagagtga tgaagtaaga cggcatgtac taccaagtcg 990300
gaaataaatg tcttgagaag caccaggctg aaaaccttta tttcagcttg gtagtaccaa 990360
gaatcaaaga aaacggacag attgtcaggc cggaatataa cggcagcctg tggaagatgt 990420
cggacggtca gccgctaagg cttttattgg cggaatgcag tccgaaagac aacctgcaaa 990480
gcggtcttga aacaggctgg atagtattcg gcatcctcgc gtccgtttac tttgtttccc 990540
tgctgaaaaa ggttttgaaa tgatggattt ttatttttat ctcggcgttt ccgtacccgt 990600
attaatcggg gcggttctgt ttaagaattg agcgcatgaa gttatggtgt caaaatcagg 990660
ctttcaaaac aatcattgaa aggcagaacc atgaacaagc cgtttatcac tcaggcgcag 990720
ttggcacttt ataaatatca gccgtccagc aagtattttg ggcaatcgat ggcggttata 990780
gcgcaatctg aatttgttga atttgcgaag attaataagt ctgaaaatgt tattgattgt 990840
ttctcttttt tctggaatag aagaattaaa catgatattt ggctaatctc attttctgat 990900
aattcagaaa tggtaattaa agaatccctg aaagatggtc ataaaatata caaatttgaa 990960
ttttgcgaaa ttgtcgataa ttgcaatttt gatgatgtat tcgtttgaag cgaatgcaaa 991020
tgcagtaaaa atatctgaaa ctgtttcagt tgataccgga caaggtgcga aaattcataa 991080
gtttgtacct aaaaatagta aaacttattc atctgattta ataaaaacgg tagatttaac 991140
acacatccct acgggcgcaa aagcccgaat caacgccaaa ataaccgcca gcgtatcccg 991200
cgccggcgta ttggcggggg tcggcaaact tgcccgctta ggcgcgaaat tcagcacaag 991260
ggcggttccc tatgtcggaa cagccctttt agcccacgac gtatacgaaa ctttcaaaga 991320
agacatacag gcacgaggct accaatacga ccccgaaacc gacaaatttg caaaggtctc 991380
aggctaagtg cgcctgttgc cgcctaaaag gtaccccgga tgcctgatta tcgggtatcc 991440
ggggaggatt aagggggtat ttgggtaaaa ttaggaggta tttggggtga aaacagccga 991500
aaacctgtgt tggggtttcg gctgtcggga gggaaaggaa ttttgcaaag gtctcttttc 991560
gtcattcccg ccacttttcg tcattcccgc gaaagcggga atctagaatc tcggactttc 991620
agataatctt tgaatattgc tgttgttcta aggtctagat tcccgcctgc gcgggaatga 991680
cgatgcaggt atttctgacg attcccggct atgatgttga ggcagaaatc gaaaaattcg 991740
tttggatgga tgctgtgatt tggcagatgc cgggctggtg gatgcacgag ccttggacag 991800
tgaaaaaata catagacgga gtattaaccg ctggacacgg caaactctac caaagcgacg 991860
gcagacacag cgtcaatccg actgagggct acggcacagg cggcttgttg caaggcaaaa 991920
aacatatgct ttcactgact tggaatgcgc cgattgaggc gtttacccgc gaaggcgatt 991980
tctttgaagg caaaggcgtt gatgttttgt atatgcactt ccacaaagcc aacgagtttt 992040
tgggtatgac ccgcctgccg acattcttat gtaacgatgt ggttaaaaat ccgcaagtgg 992100
aaaaatactt ggcagattat caggcacact tggaaaaagt gttcggctaa ttaaaaatcc 992160
atcttcaaca cggagatgga ttttgtttgt ttcgttgatt ttgtgtcagt ttcagatgta 992220
ggtgcttatt cggacgggcc gtctgaaaat gtttgcccca atgcaaaaaa atcactgcaa 992280
accttcataa acggggtttg cagtgatttt ttcaaatcaa acagattgaa aacctgcgcc 992340
gaattgttca gacggcatta ttttttcagt tcggacagaa tgtcatctac ggttttcttc 992400
gcatctccga aacacatcac gctgttttcg ttgaagaaca gtgggttttg tacacctgcg 992460
tagccggtat tcatcgagcg tttgaagacg acgacttctt ttgccttcca cacttccaac 992520
acgggcatac ccgcaatcgg gctgttcggg tcggtttggg cggcggggtt gacggtgtcg 992580
ttcgcaccga tgaccaagac cacatcggtt tcggggaagt cgtcgttgat ttcgtccatt 992640
tccaaaacga tgtcgtaggg gactttggct tcggcgagca gtacgttcat atgaccgggc 992700

aggcggccgg cgacggggtg gatgccgaag cgtacttcgg tgccgttttt acgtaaaagc 992760
tcggtgattt cggcaacggg gtattgcgct tgtgcgactg ccataccgta gcccggggta 992820
atgatgacat tgtttgcgcc tttcagcatt tcggcaatat cggcagcttt gacttctcgg 992880
tattccccta tctcttggct gccggaagat aatgtgccgc tgtcgctgcc gaaaccaccg 992940
gcaattaccg agacaaacga gcggttcatg gctttgcaca taatgtagga cagaatcgcg 993000
ccgcttgagc cgaccagcgc gccggtaacg atgagcaggt cgttggagag catgaagcct 993060
gccgctgcgg ccgcccagcc ggagtaggag ttgagcatgg acacgaccac gggcatatct 993120
gcgccgccga tggaggcaac caagtgccag ccgaatgcga gggcaatcag ggtcataatc 993180
agcaggatga agccgctgcc gtcaatgccg acaaatacga gcagcaacac aaacgatacg 993240
gcaagtgcca gtgcgttgag cttgtgtttg gcgggcagtt gcagcgggct gctgctgatt 993300
ttgccgttga gtttgccgaa tgcgaccagc gagccggtaa aggttaccgc gccgatgaag 993360
atgcctaaat acacttcgac cagatggatg gtgtgcatat cgtgcgaaac gttgcccggc 993420
gcgatatagc tgttgaagcc gaccaaaacc gccgctaggc cgacgaagct gtgcagcagg 993480
gcaatcagtt cgggcatttc ggtcatttcc acctttttgg ctttgtagat gccgattgcc 993540
gcgccgatga gcatggcgat gatgatccag cccagtccgt gggtattgtc ggaaaaaaca 993600
gttacaaaaa gggcgaccgc cataccggcg ataccggaat agcagccctg tttggcggtt 993660
tcctgtttgg acagccccgc cagtgagaag atgaataaaa ttgcggcaac gatatacgcc 993720
gctgttacga gtcctgaaga catagaaatt ctccgatttt cgatgatttg ttttcaatgc 993780
cgtctgaaaa attgacgttc gtgttttcag acggcatctg tttcaagcag ccgcgacaaa 993840
cagcccgacg gtgcaggcaa atccgcctgc ccacatcagt gagcgcatag cggctttgtc 993900
ggcgatatag caccagataa aggcgaggcg gaacaggatg aacaggcagg caagcgtgtt 993960
gatggtcgat tgcgccgcat tgccggttgc gtgtgccgtc aaaacggcgg cggcaaacgg 994020
tgcaaaggct tcaaaaccgt tttgctgtgc ggcgtgggca cgggcggctg cgccttgcgt 994080
gtgcgctaga aaaccgcgcg gattgtggtt gtctttaaac cggaatccgc ccgctttttt 994140
ggcatacgcc gcacaaaaaa gcggcaatag gcaggcaatc agaatacacc aataggcgaa 994200
agtcatggct taccctttct taaacatatt cagcatacgc cgtgttaccg caaagccgcc 994260
gaagatgttg atgccggcaa tcaggatggc aacaaacgac agcagcgaaa cgaagccgtt 994320
gccctgaccg atttgcagca gcgcgccgac gacgatgatg ccggagatgg cgttggttac 994380
cgacatcagc ggtgtgtgca gcgagtggct gacgttccag acgacgtagt agccgatgac 994440
gcaggcgaga acgaacacga taaagtggtt caggaatgct gcgggtgcga ccgcgccgac 994500
ccacagtacc aagacggcgg cgatgacggc gggcgcgagt tttttccaca ggggaacggg 994560
ttttggctcg ggcttggcgg caggcacggc tttttcagac ggcgtttgct gcggctgggc 994620
ggaaacttga atcggcggag gcgggaaggt gatttcgccg tcgtgggtaa cggtcatgtt 994680
gcggataatc acgtcttcga agtccaacgt gatttcgccg tctttgttcg ggcttaacag 994740
cttggtcagg ttgaccaagt tggtggcgta aagctgggaa gactgtccgg caaggcggtt 994800
tgccatgtcg gtgtagccga tgattttcac gccgttgccg gttacggaca attcgcccgg 994860
gcgggtgagt tcgcagttgc cgcccgtcgc cgccgccaaa tcgacgatga cggagccgga 994920
tttcatgctt tccaccattt ctttggtaat cagcttgggc gcgggtttgc ccggaatggc 994980
ggcggtggtg atgatgatgt ccacttcttt cgcctgctcg gcaaagagct tcatctcggc 995040
tgcgataaat tcgtcgctca tcactttggc gtagccgtct ccgctgccgc ccgattcttg 995100
tgggaagtcg agtttcagga acttgccgcc catcgattcg atttgttccg ccacttccaa 995160
gcgggtatcg aacgcgcgta ccactgcgcc gagcgagttt gccgtaccga tcgccgccaa 995220
acctgccaca cctgcaccaa tcaccaaaac ctgcgcgggc ggcactttgc cggcggcggt 995280
aatttgaccg gtgaagaaac ggccgaaggc gttggcggct tcaattacgg cgcggtagcc 995340
gctgatgttt gccatcgaag acaaagcgtc caaagcctgc gcgcgcgaaa tgcggggcac 995400
catatccatc gccagcgcgt tcactttctt ggcgcgcaag gcttcgacca aagcctcgtt 995460
ttggcgcggc cacaggaagc tgacgatggt ttgaccttcg ttcaaaagcg gcagttcctg 995520
ttcggacggc gcgttgacct tataaatcaa agggcagacc caaaccgccg ctttgtcggc 995580
aacggttgcg cctgctgttt ggtaagcggc atcgtccaaa cttgccgcca aacctgcacc 995640
gctttcgaca acggtttcaa agcccagttt gcccagcagg gcgacggtgg cgggcgtaca 995700
ggcgacgcgg gtttcgccgg ataatgactc gcgtgggata ccgattttca tctctgaatc 995760
ctttttcggg ttgtttatat gtatcgtggg ttaaatttaa atcggggcgg ggcggagcaa 995820
cgccgtaccg gtttaaagcc gactcacttc aaatgttaat atattttaga taatcccctt 995880
ataacgaatt ttcatcaggc tggcaatagt tgcggcattt tcccgtgttg tccgacacat 995940
actgccgttt gtgtttgaaa tcgttttccg attgcagtcc cgcccgccgt aacattcttt 996000
gccgtttcac tatataatcc gcatttttttg agccgccgtt atgccgcacg ccctcgtcct 996060
ccaatttccc tccgccgcag ccctgccttc cgacttcccc ttacgcctgc ccgaacctga 996120
ttgcgccgat gaaaagcgta tgcgttttat cgttgaagaa gggttttctt taagcgaaaa 996180
agacgcggcg ttgcttggca gccgtcaaat cgaccacgcc gtgttgccgg atatggattt 996240
cgacgaactc ggtttgattg tcagcgatat ggattcgacg ctgattacca tcgaatgcgt 996300
cgatgaaatt gcggcaggcg tgggtttaaa aaacaaagta gcggaaatta ccgagcgttc 996360

gatgcgcggc gaactcgatt tcgaacagtc tttacgcagc cgcgtcgcgc tgttggcggg 996420
attggacgaa cgggttttgg cggacgttta tgaaaacgtt ttgaagctct cgcccggtgc 996480
ggaatttttg ttggacgaat gcaaaaggca cgatgtgaaa ttcctgctgg tgtcgggcgg 996540
cttcacgttt tttaccgaaa ggctgcaaca acgcctcggc ttcgaatacc aacacgccaa 996600
tgttttggaa attgaaaacg gcaggctgac cggccgtctg aaaggcagaa tcatcgacgc 996660
gcaggcaaag gcagatttgt tgcgcgaata ccgcagccgc ctcggattgc agccgcatca 996720
ggtgttggcg gtgggcgacg gtgcgaacga tattccgatg ctcaaagaag cgggcatagg 996780
cgtggcttac cgtgccaaac cgaaagcgcg ggccgccgcc gatgcctgta tcaacttcgg 996840
cggtttggag cgtgtacgcg gcctgttcgg ataggcggat aggaaacgga tgtcgtccga 996900
aaggttttca gacggcattt gaacggcagg aacgacagtg ggacgcagaa aactttggtt 996960
tgccctggca gcagcgggcg ttatcggcgg tttggtcggc attgtgctga cggaactgat 997020
gcacttcata cagcatacgg catacggtta tggcgcggac ggcgtgtaca cttcgttccg 997080
cgaaggcgtg gcacaggctt ccggtatgcg gcgcgttgcc gtgctgacgc tgtgcggcgc 997140
ggtcgcaggc agcggctggt ggttgctgaa acgtttcggc aagccgcaaa tcgaaatcaa 997200
agccgccttg aaacagccgt tgcaggggct gccgtttctg acgacggttt tccatgttct 997260
gctgcaaatc ataacggtcg gactcggttc gccgctcgga cgcgaagtcg ccccgcgcga 997320
aatgaccgcc gcgtttgctt ttgccggcgg caaacgcttg ggtttggatg aaggcgaaat 997380
gcggctactg attgcttgcg cttcgggtgc gggtttggcg gccgtgtata acgtgccgct 997440
cgcctccaca cttttcattc tcgaagccat gctgggcgtg tggacgcagc aagccgtcgc 997500
cgctgcattg ttaacttcag tcatcgccac cgccgtcgcg cgcatcggct tgggcgacgt 997560
gcagcaatat catccggcca accttaccgt caatacttca ttactttggt tttccgccgt 997620
catcggcccg atactgggcg tagccgccgt ctttttccag cgtaccgccc aaaagttccc 997680
ctttatcaag cgcgacaata tcaaaattat tcccttggcc gtctgtatgt ttgcactcat 997740
cggcgtgatt tccgtttggt ttcccgaaat tttgggcaat ggcaaagcag gcaatcaact 997800
gacctttggc ggattgaccg attggcaaca cagccttggg ctgaccgccg tcaaatggct 997860
ggtcgtctta atggcgcttg ccgtcggcgc atacggcggt ctgattaccc cgtccatgat 997920
gctcggcagt accatcgcct ttgctgctgc caccgcgtgg aacagtgttt ttcctgaaat 997980
gtcctctgaa agcgcagcca ttgtcggcgc cgcagttttc ctcggtgttt cccttaaaat 998040
gcccctgacc gccatagcct ttattttgga gctcacctac gcccctgttg ccttgctcat 998100
gccattatgt acaggcatgg caggtgcagt atgggtggca aagaaaatgg gatttaaata 998160
ggcaaaagca aaaggccgtc tgaaaccaag tttcagacgg ccttttacaa taaaattgtt 998220
aacaatattt gcaaaaacct actgccaaaa atgcgaaact gggggataat accgccctga 998280
aaattcatcc catactgatt aaaccttcaa caaaggaaat ccaaatgtct tccatcaaac 998340
gcgccctgat cagcctatcc gacaagacag gcgcagtcga atttgcccaa accctgcaca 998400
aactcggtgt cgaaattctt tctaccggcg gtacagcaaa actcttggct gatgcaggcg 998460
ttcccgttat cgaagttgcc gactataccg gttttcccga aatgctcgac ggccgcgtga 998520
aaaccctgca tccgaaaatc cacggcggta ttctcggtcg tcgcgatttg gacgaacacg 998580
tcgccaagat ggaagaacac ggcatcggca atatcgacct cgtgtgcgtc aacctctacc 998640
tcttcgctgc caccatcgcc aaaccaaact gcacgctgga agacgcgatt gaaaacatcg 998700
acatcggcgg cccgaccatg gtgcgctctg ccgcgaaaaa ctggaaacac gtcgccatcg 998760
ttaccgacac cgccgatttc ccggccatag ctgccgaact cgaagccaac aacggcgcat 998820
tgagcgacaa aacccgtttc aacctctcgc gcaaagcatt cagccatacc gcccaatacg 998880
acggtatgat ttccaattac ctgacctcgc tttcagacga cgtcttgagc ggcacgcccg 998940
aaatcgccgg attccccggc cggttcaatc aaagctggat taaagtgcaa gacatgcgct 999000
acggcgaaaa cccgcatcag cgcgccgcgt tctaccgcga tattgacccc gccgcaggca 999060
gcctcgctgc atacaaacaa ctgcaaggca aagaattgtc ttacaacaac atcgccgatg 999120
ccgatgccgc atgggaagcc gtcaaatcct tcgacgtgcc cgcctgcgtg attgtgaaac 999180
acgccaatcc gtgcggcgta gccatcgcct ccaatacctt ggatgcctac aaactcgcct 999240
acgccaccga caccaccagc gcgttcggcg gcatcatcgc tttcaaccgc gaagttgacg 999300
gcgcaaccgt caaacaaatt accgacaacc agtttatgga agtcctcatg gcgcctaagt 999360
tcaccgccga agccctcgaa atcgccgccg ccaagaaaaa cgtgcgcgta ttggaagtgc 999420
cgcttgaggc aggcgcaaac cgcttcgaac tcaaacgcgt cggcggcgga ctgttggtgc 999480
aaacgcccga catccaccgc atcagccgcg ccgatttgaa agtcgtctcc aaacgccaac 999540
cgaccgagca ggaatggaac gatttgctgt tcgtctggaa cgtcgccaaa tacgtcaaat 999600
ccaacgccat cgtattcggc aaaggcggtc aaacctacgg catcggcgca ggccaaatga 999660
gccgcgtgga cagcacccgc atcgccgccc gcaaagcgca agatgccggt ctcgacctca 999720
acggcgcgtg tgccgcatcc gatgccttct tccccttccg cgacggcgtg gacgtgattg 999780
ccgaacaggg catcaaagcc atcatccatc cggcaggctc gatgcgcgat caggaagttt 999840
tcgacgcagc cgacgaacac ggcatcgcca tggtcgtaac cggcatccgc catttccgcc 999900
attgatgcag ataaacaagg taatgccgtc tgaagggctt tcagacggta ttttgcgcta 999960
ttttgcgaag gtagggatga cggttcgggt attcctgaca gggtggattt tcaaggtgtt 1000020

gtatagggtg taggaggatt cgtaaaaggt gggatgcagg gtgtgcttca gcccgctgca 1000080
tcaaaaattt ttggagaacc ggcgggagtc ggcggttttg gtttcggcgg ggacggtgga 1000140
aatgggtaac attgacggaa tcgacggaag cggtggactg aagcccaccc ttgtatattg 1000200
gaatcaccgt atcatagcaa caaaccgccc agccgccacc cgcgcccacc caaggcacac 1000260
aaccgttgcg tagctcaggg agcggcaggg caacccatcg acacaaccgg acagttgccg 1000320
gacaacacaa ccgaatgcaa ggcaggttta tgatgagtac ccaataccat tacgcaggta 1000380
tagtgaatta aatctaaacc agtacagcgt tggttcgcct tagctcaaag agaacgattc 1000440
tctaaggtgc tgaagcacca agtgaatcgg tttcgtacta tttgtactgt ctgcggctcg 1000500
ccgccttgtc ctgatttttg ttaattcact atatcgacat cgccaaacga aacttcgtca 1000560
tcgccgtttc gtctttgtct aaaaaccaaaa ccgaaaccaa caaccccaaa ggtatcgccc 1000620
atactatcga ataccttaaa aaacacaagg tcgccctcgt cgtgacggaa agtaccggcg 1000680
gtctcgaaat ccccgccgcc aaagccatcc gccgagcagg gccgtgatta tcgccaaccc 1000740
gcgtcagacg catcagtttg cccaatcgca gccgctgacc aaaaccgacg ccaaagatgc 1000800
caaaatgccc gccttcttcg cacagatgac ggcacagaaa gaagattcgc aaaccatgcc 1000860
ctaccacccg cccaccgaag tggaagaagt gttggaagcc ttggttaacc gccgcaacca 1000920
actggtggat atgcggactg ccgagaaaaa ccgtctgcat taggttcatg aaacgcaagt 1000980
cgaaagcgtc aaacaactga ttgcccattt tgaccggctg attgacgaat tggacaaaca 1001040
aatcgacaac cacacccaca cgcattttga cggcaaagcc caagtggcag agcaaatcaa 1001100
aggcatcggt tcgataacga cggctacgct gatggcgatg ttgcccgaat tggggcggct 1001160
gtcgcacaaa cggatagcga gtctagtcgg cattgcccca cacccgaggg agagcgggga 1001220
aaccaaattc aaaagccgct gctttggcgg aaggtctgcg gtgcgtaagg cactgtatat 1001280
ggctaccgtg gcagcgacac gttttgaacc gcttattcgg gatttctacc aacgcctgcc 1001340
gtccgagggt aagccgtata aggttgccgt tacggcatgt atgcgcaaac tgctgacgat 1001400
atcgaatgcc cggatgcgtg attattttgc cgaaaacgat accgccgaaa acggtatcta 1001460
aacggcttga tttgagtttt ggtatttttg cccgacgggg tgaaaaatac agttgctttt 1001520
ttatgtctgt ccgtttcgca aaaaacatcg gcttaatact atatattgtg ttttatgggt 1001580
ttgagatgcg ccgggcgttg attgcgaaaa ttaagattgc tcaaaaggag ctgggcttgg 1001640
atgacggtac ctatcgcgcg gtgttggagc gtgtgacggg caagcggtcg tgtgcggata 1001700
tggatgtttc cgaacttgag tctgttgtcg ctgatatgcg gtcgcacgga tttaagccta 1001760
aagcaaaagg taacccacac ggcaaaccgc atctgcgtcg gacatcatca gcggcaatgt 1001820
tggacaaagt cgaagccctg ctgaccgtcg gcggcaaaca ttggaactat gcacacgcaa 1001880
tggcgcggcg gatgtttggt aaggataagg tcgaatattt agacgatacg cagctacata 1001940
aactggttgc tgcgttgcag attgcggaaa acaggaaaac ggaaaaagcg ggtggggatg 1002000
atggggttcg aaaaagttga acatttattg ccggataccg tgttggacat tgtggatgtc 1002060
atcggactgg cagcgacgga acagctggtc aaggcgattg gcggggcgcg gtttaaattt 1002120
ggtaagggca aggtggacac cgagcgtttg gcaattttgg tcgaagccat cggcgaagtg 1002180
aaaacacatg agctgttgca ggtatatggt ggcgaggaat tgtatgtccc acggtgcggc 1002240
aaggcgttaa tacagttgag aaaccatagg ttttatcagg agtttgtcaa attgcgcgat 1002300
attgataaga agagcgggct tatggcgatg acgaagctat gccctaaata cggcatctct 1002360
tcacgaacgg gatatacgat tatcaatgaa atgagccgac ctgcggcaca gcaggcagct 1002420
ttattttagg cagtgatgtg tgaccaggct ttggccgtct gtattcagac ggtctttttt 1002480
ttggtttgca ggggtgaaac atctaccgtt cgggacgatg ggttaaagac ggtttaatgg 1002540
ggttttcaaa tgttatagtg gattaacaaa aaccagtacg gcgttgcctc gccttagctc 1002600
aaagagaacg attctctaag gtgctgaagc accaagtgaa tcggttccgt actatctgta 1002660
ctgtctgcgg cttcgtcgcc ttgtcctgat ttttgttaat ccactatatg caatcaatat 1002720
tttttggaga tgatgaatgg gcaaaaccgt aaccttaacc gctggacaca gcaacaccga 1002780
cccgggtgcg gtcaacggaa gcgaccgtga ggcggacttg gcgcaggata tgcgcaacat 1002840
tgtggcttca atcctgcgta acgattacgg cctgaccgtt aaaaccgacg gcacgggcaa 1002900
aggcaatatg ccgctgcgcg atgcggtcaa gctgattcgc ggctcggatg tggcgattga 1002960
gttccacacc aatgcggcgg cgaacaaaac ggcgacaggc atcgaagcct tgtccacgcc 1003020
gaaaaataaa cgctggtgtc aggtgctggg caaagccgtt gccaagaaaa ccggctggaa 1003080
actgcgcggc gaagacggct ttaagccgga taacgcaggg caacattcgc gcctggctta 1003140
tgcgcaggca ggcggcattg tgtttgagcc ttttttcatc agcaacgaca ctgatttggc 1003200
cttgtttaag acgaccaaat ggggcatctg ccgcgcgatt gcggacgcga ttgcgatgga 1003260
attgggagcg gcgaaggtat gaaaaagtct ttgattgctt tatgtgttgc ccattgtgca 1003320
aagttgaaaa acgattttgg cgtaccaccg ttacctgaaa tcaaaatcac gccaagccct 1003380
gttcgggtag gctctttgaa acaacatccg agcctgcgct tgggtaaatc aggcgtggcg 1003440
gctgctaaac gtgcggcgcg caaacgcaag aatcgtcgtt aatcatggga caggttgcgt 1003500
tttacgaaaa gatgattggg ctgtggtcgg ccaaaagccg tgaggcaagc gaacaggcgg 1003560
acttggctgc gtttgaattt gcggagggcg aactggccaa ttatcgggaa atgctgaaac 1003620
ggcacctgca aaccaaaagt gtggaatagc aatgcgtatt ttggatattt ttaaaaaccc 1003680

ggcgacaggc aatgtgtcgc actcgaaact gtgggcaaac gttgcctgcg cggctgggac 1003740
gtttaagttt gtgatgttgc ccgatccgtc ggcggaaatt tgggcggtgt atttgggcat 1003800
tgtcggcggc tatgcggtgg cgcgttcatt tgtcagcgtg aagcgtcagg aggtcgagaa 1003860
tgaatctcgt gaaactgctg gcgaataact ggcaaccgat tgccattatc gcgcttgtcg 1003920
gcacgggctt ggctgtgtcg caccatcaag gctacaagtc ggcatttgcg aagcagcagg 1003980
cggtcatcga caagatggag cgcgacaagg cgcaagccct gctgttgtcg gctcaaaact 1004040
atgcgcgcga actggaactg gcacgcgcgg aagctaaaaa atatgaagtc aaggcgcacg 1004100
ctgtcggcat ggctttggcg aaaaaacagg cggaagtcag ccgtctgaaa acggaaagag 1004160
acctttgcaa aattcctttc cctcccgaca gccgaaaccc aaacacaggt tttcggctgt 1004220
tttcgcccca aataccgcct aattttaccc aaataccccc ttaatcctcc ccggataccc 1004280
gataatcagg catccgggct gccttttagg cggcagcggg cgcacttaac ctgttggccg 1004340
ctttcaacag gttcaaacac atcgccttca ggtggctttg cgcactcact ttaatcagtc 1004400
cgaaataggc tgcccgcgca tagcggaatt tacggtgcag cgtaccgaag ctctgttcga 1004460
ccacatatag tggattaaat ttaaaccagt acggcgttgc ctcgccttgc cgtactattt 1004520
gtactgtctg cggcttcgtc gccttgtcct gatttaaatt taatccacta taacgggtct 1004580
tcgataaata tcggttacgc ttggtttgca cttccgacag cgggcggttg cggcaggctt 1004640
tgctcataat gccgtccaac aactgatgtt cttccagatg ttgccggttt tccgcactgt 1004700
catagccttt atcggcatag acggtcgtac cttcgggtaa cccttccaac aacggcgaca 1004760
ggtgtttgca ctcatgggca ttggcggggg tgatgtgcag tttctcgata tagccttccg 1004820
catcggtacg ggtatgttgt ttgtaaccga gtttgtagag gccgtttttc ttgatccaac 1004880
gggcatcgct gtccttactc ggtgtggttt ggccgctgat ttgtccttct tcatcgactt 1004940
ctatggcctg acgctgttcg ctgccggcgg tctgaataat ggtggcgtca atgacggcgg 1005000
cggatgcttt ctctactttt aagccttttt cggtcagttg gcagttaatc agttccaaca 1005060
gttcggacag ggtgtcgtct tgcgccagcc agttgcggta gcggcataag gtgctgtaat 1005120
cggggatgct cagttcgtca aaacggcaaa acaggttgaa atcgatgcgg gtgatgaggc 1005180
tgtgttcgag ttcgggatcg gagaggctgt gccattgtcc gagcaggacg gctttgaaca 1005240
tggatagcag gggataggcg ggacggccgc ggtggtctcg gaggtaacgg gttttttgac 1005300
ggttcaggta ctgctcgatc ggctgccaat caatcacttg gtccaacttc aatagcggga 1005360
aacggttgat gtgtttggca atcatggctt gcgcggtttg ccggaagaag gtgctcatga 1005420
gaaatcccct aaatgtcttg gtgggaattt aggggatttt ggggggattt tgcaaaggtc 1005480
tcaggcggca aatcgccacc cttcccttca aaccttccgc ctgtcccaac agcagacagg 1005540
cgaaaaagcc cttaccactg ataaccgaca gatgcggaag caccgaaatg gccgcgcgaa 1005600
ttgccggaag ccgtgccttt gataatccaa tttccgccgt cggaaatact ggagtagccg 1005660
atggcgtaac cggcttcgcc gcgataagtg ccgccgccga tcgccatcat actcttgccg 1005720
ggcaaatacg cctgaaccag acctgcggtt gcaatcgctt gggcgatgcc cgcacgcgcg 1005780
ttgccgtcca cattgtcgat gcggttgttc aagttttgcg ccacgccttt aagttgtgcg 1005840
acgtttgtaa catccccctc tttaacgccc ggggcgacat tggtaatgcg gacgggtttg 1005900
ttgtccttct tgctgccgac attcaatgcg tccccatcca cgctcaaagt gggcgcatcc 1005960
gcccccgcgc cgagcgaaac gctggaaaac tgcggggtca tcgaagtggc gatgtcgata 1006020
tttttaccgt tgcgggtaat ctcgatgttg ttgccggcat taatgttgac ggtttcatcc 1006080
atctttccct tgctcggcga aacattgccg ctgatgactt tgcccgaaga acctgcaacc 1006140
gctttggaat ccaaattcca accgctgttt tgcagctgat tgacgtttag ggcatcgccg 1006200
acatttacat catacataac agtgatgttg ccttgatcat ctttacttac agtcgcagtt 1006260
gtacctttac cactagcaaa ggttacattt gtgcctgatg taacggtttc aaacttgtca 1006320
gcttgacctg tttgaccatt agcggttgtt gttttcattc tccaaccagc cttgtttact 1006380
gcatcaatca cttcttttgc agtcactaag ccttcgcctt cgtctgtaga agaaccattc 1006440
tcgcctttgt ctttaccagt aaccaactta ccgtcttttt ctttaataac agaagtcttc 1006500
gcaccgattt taacttcggt tttcttgccg ttgtctttgc tttccacatt aacagtcgtt 1006560
gttttcgtat ctgcgctcaa gaactcgact gtgtcgtaag tgcggacgaa atcaacgtta 1006620
tcggaagctg ttgtaccggg tttaacgcct ttaatgttcc agccagcgtt taatacgtct 1006680
ttaacgcttg ccgcacgttt tttctcgtca tcggtaacgt tgtcgttggt tacgtttgtg 1006740
gtcgctccgg tattcagcag cgtatcggtc aaagtcgaac caataccgtt cagatgaacc 1006800
gtggtgtcgc cgttcgtccc agccgtttct ttcgcaaaat tcaagccttt ggtgtcgctt 1006860
gtgatgttga ctttattgcc gtttgcgcta aacgataatt tttcagttcc aacactggtc 1006920
agatctgtga ggtctttttt cagcgagtag gtgaagtttg tgccgttttg tttgattttc 1006980
aggttgtcgc cggctttgag ggtgatttct ctggctgtta gtactccttt ctcgttgaaa 1007040
tatactgccc aatctgaatt ttcttctact ttttcttttt ctcccgtgcc ttctttatcg 1007100
gaattgacta tcaacacggc aacagtgcgt tgtacggggt ctaaatataa atcttcttct 1007160
tgctcttcat tgttagcact tgcctgaacc gttgcaaaca acagtgtcgc caatacggcg 1007220
gtcttcacgg ttgcggaggc gcgtttggtg tggttgcgtg tgagctcgga tacgacgacc 1007280
caggcattga gggcactatt ccaaatgatg cggtatattt tgttcatttt gtttttttctt 1007340

ttggtttgtt tgaatggtta aatcggggtt tgggggcgga tggtgcggca tccgcccggt 1007400
ttttgggggt tgggggtttt ctgataaatt cccccaactt aaaatctcgt cattcccgcg 1007460
aaggcgggaa tctgggacgt ggaatctaag gaaactgttt tattcggtaa gtttccgtgc 1007520
cgacgggtct ggattcccgc ttttgcggga atgacggcgg tggggtttct gtttttttctg 1007580
atagattcct gtggtttttc tatggattca atcattcctg ataaattccc ataatctaaa 1007640
atctcgtcat tcccgcgaaa gcgggaatct aggacgtgga atctaaggaa actgttttat 1007700
ccggtaagtt tccgtgccga cgggtctgga ttcccgcttt tgcgggaatg acggtcggtg 1007760
gggtttctgt tttttccgat aaagtcctgc cgcgttgtgt tgctggattc ccgcctgcgc 1007820
gggaatgacg gcggtggggg tttctgtttt ttctgataga ttcctgtggt ttttctatgg 1007880
attcaatcat tcctgataaa ttcccataat ctaaaatctc gtcattcccg cgaaggcggg 1007940
aatctaggac gtggaatcta aggaaactgt tttatccggt aagattccgt gccgacgggt 1008000
ctggattccc gcttttgcgg gaatgacggc ggtggggttt ctgtttttttc cgatagattc 1008060
ctgttgcgtt gcgtttttgg attcccgctt ttgcgggaat gacgcggtgg gggtttctgt 1008120
tttttctgat agattcctgt ggtttttcta tggattcaat cattcctgat aaattcccat 1008180
aatctaaaat ctcgtcattc ccgcgaaggc gggaatctag gacgtggaat ctaaggaaac 1008240
tgttttatcc ggtaagattc cgtgccgacg ggtctggatt cccgcttttg cgggaatgat 1008300
ggcggtgggg gtttctgttt tttccgataa agtcctgccg cgttgtgttt ctggattccc 1008360
gcttttgcgg gaatgacgcg gtgggggttt ctgtttttgc tgatagattc ctgtggtttt 1008420
tctatggatt gaatcattcc tgataaattc ccataatcta aaatctcgtc attcccgcga 1008480
aggcgggaat ctaggacgtg gaatctaagg aaactgtttt atccggtaag tttccgtgcc 1008540
gacgggtctg gattcccgct ttcgcgggaa tgacggcggt ggggtttctg tttttgctga 1008600
tagattcctg tggtttttcg gttgctggat tcccgctttt gcgggaatga cggcggtggg 1008660
gtttcggttt tttccgataa attcctgttg cgttgcgttt ttggattccc gcttttgcgg 1008720
gaatgacggt cggtggggtt tcggtttttt ccgataaagt cctgctgcgt tgtgttgctg 1008780
gattcccgcc tgcgcgggaa tgacggccgc cggacggcaa acgaccatac acaattattg 1008840
acaaccccat ttattgcgaa agtcagccta ggagaatcga tctaattgtc aacattccct 1008900
tatgatcaaa gggattacac tttattttac gcaaatgcgg gggggggggt gatttttgac 1008960
gtttttttgcc gaaaatttac attcggacga cgaaaaggaa aaagccgtgt cgcatctgtg 1009020
caacacggct tggcgggcgc aaacggatat agtggattaa caaaaaccag tacggcgttg 1009080
cctcgcctta gctcaaagag aacgattctt taacaagtga attggttccg tactatttgt 1009140
actgtctgcg gcttcgtcgc cttgtcatga tttttgttaa tccactataa aacggtgttc 1009200
cctgccgccg caggcggaac gccggatgac ggggttttcc ctaagggtgc ggctgccgct 1009260
atatcacgaa atccaacagg tagaaatctt ctttgcccac gccgcattcg gggcatttcc 1009320
agtcgtcggg gatgtcttca aacttggttc cgggggcgat gccgtgttcg gggtcgccgt 1009380
gttcttcatc gtaaatccag ccgcaggggc cgcacatata ttgcgccatt tgtgtttcct 1009440
tgttttttgt atagtgggtt aacaaaaacc ggtacggcgt tgcctcgcct tagctcgaag 1009500
agaacgattc tctaaagtac tgaagcaccc gtactatttg tactgtctgc ggcttcgccg 1009560
ccttgccctg atttttgttc atccgctata aatcagggtt tgggagaatg gtgcggtatc 1009620
cgcccggttt ttttggggtt ggttttttttc gatagattcc tgtggttttt cgattactgg 1009680
attcccactt ccgtgggaat gacggtttgg aggtttcggt tttttcgatg aattcctgtt 1009740
gcgttagggg gggggctgga ttcccgcttt tgcgggaatg acggtttgag ggtttctgtt 1009800
ttttccgatg gattcctgtt acgttggggg ctggattccc gcttttgcgg gaatgacggt 1009860
ttgagggttt ctgtttttttc cgatggattc ctgttacgtt gggggctgga ttcccgcttt 1009920
tgcgggaatg acggtttgag ggtttctgtt ttttccgatg gattcctgtt gcgttggggg 1009980
ctggattccc gcttttgcgg gaatgacggt ttgagggttt ctgtttttttc cgatggattc 1010040
ctgttgcgtt gggggctgga ttcccgcttt cgcgggaatg acgcggtggg ggtttcggtt 1010100
tttccgcctg tttattttgc ggcttcgatt gccgctattt ctttgcgtag gtgtttgata 1010160
gcgggggtta cgatggcaac aaacattgct tcgcggacgc gcctttgggc gggactgcgc 1010220
atcaggtagc cttttgcgcc ggaatgcagg gcggcggatt gggcggcggc aagtgccagt 1010280
acggcggcgg cttcgcgcag cttgagggta gcaaggttgt cgggcgtgcc gctccaagcc 1010340
aagccggcga gccgttcggt ttctgcccac gcgccgtcca gccttgtttt gaggctgtcg 1010400
tagccgtcgt tgaggtagtt gttgacttcg gcgttgacga cgttggcgag gcggatgatg 1010460
ccgaggctgc cgtcgattac gcccgcgccg atgccgattt gcaggaggat aaagcctgct 1010520
ttgatgcttt ggatgtagtc ggcaaactgt tcgggcgcgg cgatgatgtc ttcgtcgggg 1010580
ataaatacgt ctttgaaatt caggctgaag gtgcgcgtac cttcgagggc gcaaaattcg 1010640
gggcagtttt gcaggcttac gccttcccat tgtccgcctg tgatgaacat aacgtagccg 1010700
tcgccgattt gggcggtatt cgcccagatg tggtcttcac cgatgttgga cacccacggc 1010760
agcgcgccgt tgactgtgta gccgccttcc acgcgttcgg cttggaggtt gtgtttttcg 1010820
atgtcggcaa ggtgtttgac ggtattggac atgcccgtac ccgccaatac tttgccttgc 1010880
aggatgtcgg caaggtattt gtctttgacg gcccggttgg gcgtttggtg cagataccac 1010940
gcgcaagccg cctgacacca cgcactgaaa gaggttgcgc cgcattcttt gccgatttcg 1011000

cgcaatacgg cgatttgcgt tgccaaaccc aagccgttgc cgccttcggc ttctgtaccg 1011060
actgcgccga atccaccgat tgcgccgagt tcgcgcataa atgcttcggg gtagtatcct 1011120
ttgcggtcga tgtcgtccac tatgggtttg agcttggttt tgacgaattc ggcaacgttg 1011180
gcaatcaggg tttgggcgtt catctttgtt ccttaaggtt tgcggggaaa tcgggggcgc 1011240
gcctgatgcg cggctgcttc cctgccgctt aggcggcttg tttttgtttg agggggtgtt 1011300
tttgaaaaac cgcccgatat tcgggcggtt tgccgtatca ggcgtaagcc tgcaattcgg 1011360
ggttgatttc ggtttgtccg aggttgttga cgtagttgca cagggttgcc aaggctacgc 1011420
ccatcacgac ttcgactgcc tgctgctggt tgtagcccgc atcgaaaaat gctttgagtt 1011480
cctcgtcgga taccgcgcct tttttcgcca ttacggcttg ggtgaaggcg gcgagcgcgc 1011540
cgagtttggc atcgtcaaat tcgcctgctg ccaaagcgcg cgcggctttg acggattgtt 1011600
cggacaggag ttttttcagg gttgcgagtt tggtgtgccc tgccacgcaa aaaccgcatt 1011660
ggttggtacg ggcggcgatg atctggatga cttcgacttc gccggcggtc aggctgttgg 1011720
cggcgttgag cttgccgact tcttggtaaa acgccaaggc ttcgggggcg tttgataata 1011780
cgccgataag gttggggata aagccgttgt tttgaagtac cgcctcgacg cgcgctttgg 1011840
cggcttcggg ggcggtttcg agggtgtgta cggttaaacg tgccattttc tttccttgtt 1011900
cgcaaatatt gggtacgggc gcatggtatg catttcggaa cggaatagga aagactgatt 1011960
ggttatgtgc tgcaaacaaa aggttataag aaatgccgtc tgaacatttt tcagacggca 1012020
tgatggaaaa gaaaacgcgc ttatcggccc ccgcgcccga aatattgcgc caatgcggct 1012080
tggggattgg ctgcctcaac cgtttcgggc aattcggtat agccgcgcga taaaaggtgg 1012140
cgtgcataaa ggctgttgcc ctttgcgccg aaccagacgg aaaccgccaa gcccaatacg 1012200
ttaaacgccg agtctgtggc gggcgtgtcg ccgtaaacca gttgggcaaa cacagccagc 1012260
acgaatatcg cgcccgtcag ccccagcccc aaaccccaca ttcttttgaa acacgcccac 1012320
agcgtgccga acaagagccc cggccatgac cagccttgtt tgacggcttg gggcggcagg 1012380
gcgggatggg tgtagatttt gtatggtttc atcgtgtttc cttttcggtt gaaaccctgc 1012440
cctttgggaa ggtaggatca gactttatag tggattaaat ttaaaccagt acggcgttac 1012500
ctcgccttgc cgtactatct gtactgtctg cggcttcgtt gccttgtcct gatttaaatt 1012560
taatccacta tatttgggag gcgcgcgcgc ctgtgccggc atacggcttg aaagcgatta 1012620
cccgatgggg aacttcaaac ccgacaatgc cgtctgaacg gtgtcttgcc ttcagacggc 1012680
attgcctgcc ttcaaagcgg acgcgcttat tccgcccagt ttttcttttt gctggttttg 1012740
cctacgcccg ggttgaagct gttggtcggg tcaagtttgc ggtaaaactg tttgagcgcg 1012800
ggcttggctt cgtacaaatg gccgacgttg tgttcggctg gatattgcgc gccgcgttga 1012860
tccaagagat gcagcatttc gtgttccaat gccatgcagt cgttgccttt tttgatgatg 1012920
taatcctgat ggaaaacgtg gcacatgaaa tgtccgtagt agagcttgtg gatgatttta 1012980
ttgtcgattt ccggcggcag tttttcaaac cagtcgcggt cgtcgcggcg cagggcgatg 1013040
tcaagcgcga ccaagtcctc cacttcgtcg tcgtgtacgg cacggtagcg gatggcggct 1013100
gaggcgacgg cgaaacggtg cagcatcgcg gcttgggttt cttcggcgtt gcactcgaaa 1013160
aacgcgccgc cgtgatgtgc aaaatactct ttcaggaacg cgcgcgcctc atccacgcct 1013220
tttccgccca ttttcagaat caggtggtgt tcgtatttgt cgcggtaatc gcgcatggat 1013280
ttgggcagat ggtcaggcag gaatttgctg acgaactgca ttgccttgtc ggaaaaatgt 1013340
ttgggcagga agctgacttt tttgccgaac ctgtccacgc gtgccttcaa atcaaataat 1013400
ttcggcagtt ggtgcgtacc gaattttttg atgacgtaaa acgtatcttt gccgtacacg 1013460
tcggcaatgt cgaaagcgtg gcggtggatg tattcgccgg aaacgggcag gctttcaaat 1013520
tcgcccaagg cggcgcggcg gatgtcggtc agctcgttga tgtcgttcgt gccgatgtag 1013580
aacacggccg tttgtttttc ttgcggaaag gtatccaagc ggacggcgaa taccatcagc 1013640
ttgcccgcgc agcccgaggc ttcgtaatgg cgcgctgggt cggcattgaa acgcgcggcg 1013700
gtcggttcgt ccacttggcg gacatgttcg caataggcgt ggtcgtgtcc tttgcccgcg 1013760
tcttgcgtga tgtctttgtt ttgataatga tgaccttgaa gattggtcag gatttcttcg 1013820
ggcgtgttgc ccaagtctat gcccaagtgg ttgaccagtt ccaacctgcc ttcttcgttg 1013880
atttgggcga acaacgccat ttcggtgtag gccgggccgc gctgtaccaa cgcgccgcca 1013940
gagttgttgc acacgccgcc caagacggac gcgccgatac aggatgagcc gataaccgaa 1014000
tgcggttcgc gccccaaagg tttcagcagc aattcgagct ggttcagggt cgagccgggc 1014060
aggcagacga cttgttcgtt gttgttgatg gtttggatga tgttcatccg catggtgttc 1014120
acaatcacga tgtcgcggtc gtaatcgttg ccgtcggggg tcgagccgcc cgtcaaaccg 1014180
gtattcgccg cctgcgtaat cacaatcacg tctgcttcga cgcacgcctg cagaattttc 1014240
cacatttcca gaatgcttcc ggggcgaacc accgccaacg ccttaccctc gccgaagcgg 1014300
taaccttggc ggtattgttc ggttttcgcg gggtcggtga tgatgtattt ttcgcctacg 1014360
gtttgggtca gtcttgacag taattgtgat gcgctcatgg cagtttcctt aaaattgtcg 1014420
gcaggtgcat tgcacattgg aattgttttc acattgtagt tatacgttat ggcaaagtaa 1014480
agaaaatgcc gtctgaacgg ctttcagacg gcatcggtgc gatacgggaa cgccggaaca 1014540
tcgaagctcc ggcgtttcaa atagggcggc gggccaaacc cccggcactg gcgcattgga 1014600
gtgggctgct ggcttccgcc cctgacccgg tgttccgatt tgccatgcgg ggagacccgc 1014660

ctcagagaaa cggcattata acgggttttc tgaaaaactc aaccgttttg atacggtcat 1014720
acgccggaaa caccacctaa aatttatatt tgataatatt gtcaacaatt tctcaaagcg 1014780
ttattttgtt tctataaggg tatttcctgt ttcggcattg aaaagtatca aaaattgaac 1014840
tacattatcg ccttttcaaa ctcgcctgaa accgactttt cagacggcat tcaaataaaa 1014900
actgccaaac acggacacac catgaccacg actaccgccc ctcagcgtat tcgggaaatc 1014960
ccctacaact atacttccta caccgaccgc gaaatcgtca tccgattatt gggcgacgaa 1015020
gcgtggcaaa tcctgcaaga cctgcgcggt caacgcaaaa ccgggcgttc ggcgcggatg 1015080
ctgtttgaag tgttgggtga tatttgggtg gtcgtgcgca atccgtatct ggtcgatgac 1015140
ttgctggagc acccaaaacg ccgcgccgcg ctggtacgtg aaatgcgcca ccgcttaaat 1015200
gaaatccgca aacgccgcga cgataatcgg caagtggatg tgttggttgc cgcagcagaa 1015260
aaagcagtcg agcgttttga tagcagtttt gatgaaacca gccaaaaacg gcggcagatt 1015320
ttggagcgtt tgagcaaaat caccaagccg cacaatatta tgttcgacgg gctggcgcgg 1015380
gtaacgcacg ttaccgatgc aaccgactgg cgcgtggagt atccgtttgt cgtcgtcaat 1015440
cccgacacgg aggctgaaat cgcgcctttg gtgcgcgcct taatcgagct ggatttggtc 1015500
attattccgc gcggcggcgg cacgggttat accggcggcg cgattccttt ggacgcaaac 1015560
agcgcagtca tcaataccga aaaactcgac aagcatcgtg gtgttgaata cgttgagctg 1015620
gcaggcttgg acggcaagca tccgattatc cggtgcggcg cgggcgtggt tacgcggcgg 1015680
gtggaagaaa ccgcgcatca ggcaggtttg gtgttcgccg tcgatccgac ttctgccgac 1015740
gcgtcatgcg tgggcggtaa tgtggcgatg aacgcgggcg gcaaaaaagc cgtgctgtgg 1015800
gggacggcgt tggacaacct cgcctactgg aacatggtta accctcaagg cgaatggctg 1015860
cgtatcgagc gcgtgcgcca caatttcggc aaaatccacg acgaagaaac cgccgtgttc 1015920
gacgttcaca cgctggattc agacggcatc aatatcgtta aaaccgaacg cttggaaatc 1015980
cccggccaca aattccgcaa agtcggtttg ggcaaagacg ttaccgacaa attcttgagc 1016040
ggcctgcccg gcgtgcaaaa agaaggtaca gacggcatca tcaccagcgt tgccttcgtg 1016100
ttgcataaaa tgccgaaata cacgcgcacc gtgtgtatgg agtttttcgg tacggtcgcc 1016160
accgccacgc catctattgt cgaaatccgc gactttttgc ttgcccatga aagcgtgcgg 1016220
ctggcgggtt tggaacattt ggactggcgt tatgtccgcg ccgtcggcta cgccaccaaa 1016280
gcggcgggca agggacgacc gaaaatggtt ttgctggcag acgtggtttc agacgacgaa 1016340
gccgccgtag aggcagccgc cgaacacatc tgtgaactcg cacgcgcccg cgacggcgaa 1016400
ggctttatcg ccgtatcgcc cgaagcccgc aaaaccttct ggctcgaccg cagccgcacc 1016460
gccgccatcg ccaaacatac caacgccttt aaaatcaacg aagacgtggt catcccgctc 1016520
gaaaggctcg gcgagtattc ggacggcatc gaacgcatca acattgagct ttccatccaa 1016580
aacaagctca aactctgtgc cgccttggag caatatcttt cgggcaaact ccccatcgac 1016640
aaaatgggca ctgacctgcc gaccgccgaa ctgttgggcg aacgcggcaa acacgccctg 1016700
gcccacgttt ccgccgtcaa aacgcgttgg gaatggctgc tcgccaatct tgacacgccg 1016760
cttgccgact acaaagcccg ctacggcgca gccgtccacg ccgcacccga agccaaaaac 1016820
aatgaaagct gctttattgc cttccgcgat ttccgcctgc gcgtgtctgt caaagcggac 1016880
gtaatgaaac cgctttctga aatcttcagc ggcaaaaccg acaccaaaat tatccaaggc 1016940
ttgggaaaaa tccacgcaaa aaccgtacgc agccgcgtct ttgtcgccct gcatatgcac 1017000
gccggcgacg gtaacgttca caccaatatt ccggttaact cagacgatgc cgaaatgctt 1017060
cagacggcat accgctcagt cgaacgcatt atgaaaatcg cccgttcgct taacggcgtg 1017120
atttccggcg aacacggcat cggcattacc aagctcgaat ttttaagcga cgaagaaatg 1017180
cagccgtttt gggactacaa aaaccaagtc gatccgaaac acaccttcaa ccgtcacaaa 1017240
ctgatgaaag gctcggactt acgcaacgcc tacacgccgt ccttcgagct gttgggcgcg 1017300
gaatcgctga ttatggaaaa atcaaacctc ggcacgattg ccgattccgt caaagactgc 1017360
ctgcgctgcg gcaaatgcaa acccgtctgc tctactcacg ttccgcgtgc caacctgctg 1017420
tacagccccc gcaacaaaat cctcggcgtg ggcttattga tcgaagcctt cttatacgaa 1017480
gaacaaaccc gccgcggcgt ttccatcaaa cactttgaag aactcatgga catcggcgac 1017540
cactgcaccg tgtgccaccg ctgcgtcaaa ccctgccccg tcaacatcga cttcggcgac 1017600
gttaccgtag ccgtccgcaa ctatcttgcc gattccggcc acaaacgatt tgcgcctgcc 1017660
gcagctatgg gtatggcgtt tttgaacgcc accggcccga aaaccatcaa agcccttcgc 1017720
gccgccatga tacagatcgg cttcccagcg cagaatttcg cctacaaaat cggcaaactt 1017780
cttccaatcg gcacgaaaaa gcaaaaagcc gaacccaagg caaccgtcgg caaagccccg 1017840
attaaagaac aggttatcca tttcatcaac cgcccactgc ccaaaaacgt acccgccaaa 1017900
acaccgcgct ccttattggg catcgaagac ggcaaaagca tccccatcat ccgcaacccc 1017960
gccgcgcccg aagatgccga agccgtgttc tacttcccgg gttgcggctc tgagcgtctg 1018020
ttcagccaaa tcggacttgc cgttcaagcc atgctctggc acgtcggcgt acaaaccgtc 1018080
ctgccgcccg gctatatgtg ttgcggctat ccgcaagacg caggcggcaa taaggcaaaa 1018140
gccgaagaaa tgagcaccaa caaccgcgtg gctttccacc gtatggcgaa caccctcaac 1018200
tacctcgaca tcaaaaccgt cgtcgtcagt tgcggcactt gttacgacca gctcgaaaaa 1018260
taccgctttg aagaaatctt ccccggctgc cgaatcatcg acatccacga atacctgctc 1018320

gaaaaaggcg tgaaactcga cggcgtaaaa ggtcagcaat acctctacca cgacccctgc 1018380
catacccccca tcaaaaccat gaacgccacc caaatggcca gcagcctgat ggggcagaaa 1018440
gtcgttttaa gcgaccgctg ctgcggcgaa tccggtatgt ttgccgtcaa acggccagac 1018500
atcgccactc aggtcaagtt ccgcaaacaa gaggaaatcg agaaaaacct caaagagctg 1018560
ccgcagggcg aacccgtcaa aatgctgacc tcctgccccg cctgcctgca aggcttgagc 1018620
cgctacgccg acgacaacaa tatgcctgcc gactacatcg tcatcgaaat ggcgaaatac 1018680
atcctcggcg aaaactggct ggatgagttt gtaaaaaaag ccaacaacgg cggtgtagag 1018740
aaagtgttgc tgtaacaacg gacacggaaa tgccgtctga acgccgaaag ccttcagacg 1018800
gcattgtttg aaccaaatat agtggattaa caaaaatcag aacaaggcga cgaagccgca 1018860
gacagtacaa atagtacggc aaggcgaggc aacgctgtac tggtttaaat ttaatccact 1018920
atacctaccc aaatcatgat tgacagacaa acaaacgaac ccaaacaaaa aacccgaatc 1018980
atccttgccc ctatgcaggg tctggtcgat gacgtgatgc gcgacctgct gacgcgtatt 1019040
ggcggctacg acgaatgcgt cagcgaattt gtacgcatta cccataccgt gcattcccga 1019100
tccatatggt taaaatatgt ccccgaaatc gccaacggaa acaaaacgtt ttccggcacg 1019160
ccttgcaccg tccaactttt gggcagcgat gcggacaata tggcggcgaa tgcgctggaa 1019220
gccgtccgct tcggtgcgaa caaaatcgat ttgaacttcg gctgccccgc gcccaccgtc 1019280
aacaaacaca aaggcggcgc aatcctttta aaagagccgg aactgatatt ccacatcgtc 1019340
aaaacgctgc gcggacgttt gcccgcacat attccgctca ccgcaaaaat gcggctcggt 1019400
tacgaagaca aaagccgggc tttggaatgc gcctgtgcga ttgccgaagg gggcgcatgc 1019460
ggactgaccg tacacgcgcg taccaaagcc gagggttacg aaccgccggc gcattgggaa 1019520
tggataagga aaatccgaga cagcgtcaat attcccgtta ccgccaacgg cgacgttttc 1019580
agcctgcaag actatatcgg catcaaaaca atcagcggct gcaacagcgt gatgctcggt 1019640
cgcggcgcgg tcatccgccc cgatttggcg cggcaaatca agcaatacga gaacggcggg 1019700
ccggtcaaag acacggattt tgccgaagtt tccaaatgga tacggcagtt tttcgagctg 1019760
tgcctgacaa aagaggcaaa caacaaatat ccgctggcgc ggctgaaaca gtggctgggt 1019820
atgatgaaga aagaatttgc agcagcacaa aatctgttcg accgcgtccg aacggttaag 1019880
gatgcggacg aagttcggaa catcttggct gaatttgagc gagaaatgaa tacttgaata 1019940
tgtatagtgg attaacaaaa accggtacgg cgttgcctcg ccttagctca aagagaacga 1020000
ttctctaagg tgctgaagca ccaagtgaat cggttccgta ctatttgtac tgtctgcggc 1020060
ttcgtcgccg tgtcctgatt tttgttaatc cactatatcc gctccaaagc aaatgccgtc 1020120
tgaaaacctt tcagacggca tttgttgtct ttattgccgt ttttcgtccg tatccggatt 1020180
tttgtttttc agcttcgcac ccaagcccaa acgcctttca taatccgatt gcggagtatc 1020240
gtcttcctgc ataccgaacg cgccggcatt gacccacagc gacagcagcg cgacgacaaa 1020300
ggcgcaaaag ccaatcacat accaaaacat tgcccctccc gatttgttaa aatcatatca 1020360
aatacagtgc cgaatttatc acaaacgcac gggcaaatat agtgaattaa atttaaatca 1020420
ggacaaggcg gcgagccgaa gacagtacaa atagagacct ttgcaaaatt ccccaaaatc 1020480
ccctaaattc ccaccaagac atttaggagc accttcttcc agcaaaccgc ccaagccatg 1020540
attgccaaac acatcgaccg tttcccacta ttgaagttgg accaggtgat tgattggcag 1020600
ccgatcgagc agtacctgaa ccgtcaaaaa acccgttacc tccgagacca ccgcggtcgt 1020660
cccgcctgtc ccctgttgtc catgttcaaa gccgtcctgc tcggacaatg gcacagcctc 1020720
tccgatcccg aactcgaaca cagcctcatc acccgcatcg atttcaacct gttttgccgt 1020780
ttcgacgaac tgagcagtat agtggattaa caaaaaccag tacggcgttg cctcgccttg 1020840
ccgtactatt tgtactgtct gcggcttcgt cgctttgtcc tgattttttgt taatccacta 1020900
tactttatgc cgctaccgca actggctggc gcaagacgac accctgtccg aattgctcaa 1020960
actgattaac tgccaactga ccgaaaaagg tttaaaaata gagaaagcat ccgccgccgt 1021020
cgttgacgcc accattattc agaccgccgg cagcaaacag cgccaggcca tagaagttga 1021080
cgaagaagga caaatcagca gccaaaccac accgagtaag gacagcgatg cccgttggat 1021140
caagaaaaac ggcctctaca aactcggtta caaacaacat acccgtaccg atgcggaagg 1021200
ctatatcgag aaactgcaca tcaccccccgc caatgcccat gagtgcaaac acctgtcgcc 1021260
gttgttggaa ggtctgccca aaggtacgac cgtctatgcc gacaaaggct atgacagtgc 1021320
ggaaaaccgg caacatctga aagaacatcg gctgctggac ggcattatgc gcaaagcctg 1021380
ccgcaaccgt ccgctgacgg aaacgcaaac caaacgcaac cggtatttgt cgaagacccg 1021440
ttatgtggtt gaacaaagct tcggtacgct gcaccgtaaa ttccgctacg ctcgggcagc 1021500
ctatttcgga ctgatttgcg cccgctgccg cctaaaaggc agcccggatg cctgattatc 1021560
gggtatccgg ggaggattaa gggggtattt gggtaaaatt aggaggtatt tggggagaaa 1021620
acagctgaaa acctgtgttt gggtttcggc tgtcgggagg gaaaggaatt ttgcaaaggt 1021680
ctcagagtga gtttattttg gggcggcggc aggtcggggg caagcggcgt ggggcttggt 1021740
tgtggttttt aggtttttgg gggtaaaaaa tgccgtctga acttttcaga cggcgtttgt 1021800
tttttctatc caatcgagga actgccgcca tttttccagc ggcatatcgg cccgacgggt 1021860
ttcggtatcg ggttcggctt cccagcggcc tacctgtatg tagtgcttca ccccgacgat 1021920
ttgcgccaac tcggcctgtg tcagtttgca gcggttgcga agggtgcgga ggttgtaagg 1021980

cgtgtagccg agttccatgt cgttgcggtt tattttgttt cgcatatttt tttgactgcc 1022040
cggcggcagg tttcggtaag gatggcggca aatcgggctt cgtctgccgg tttgccgtcg 1022100
aaaaaaataa aatcgtaaag ggtgatgccg ttttcggaat ggcggtaatg cctgcccggg 1022160
cattcgccgt ctgtctgcct gtggattttg gcaatcaggc ggggatagcg gcaatgggta 1022220
atgaaacggc ttgcgccgtc ttgcccgata atccattcgg ggtagcggtt gaatagtgcg 1022280
gctctgttca ttttgttcgt gggataaagc cctcgcgggg gcttgtggtc aggcaaattt 1022340
gaatatcagt gccaacacgg cggcgatggc ggtaaccagc ccggtggccg ctatcatggg 1022400
ataccagcgg gactcttggg ctatttttac ggattcggcg ttgattttgt gcgcgtcggc 1022460
aatgattttg gcgatttcgg catctatttt tctcagactg gagtgtttca tttcttgttc 1022520
gattatgttc atcgtacttc ctttcgtttt tggcggttgc cgccgcttgt cggatggtag 1022580
gatgtctgcc atgtgtatat attgatacct tttaggtttt attgcaagtg ttttgggcgg 1022640
cggcttcgta tgcttggcgg tggcggcggc tgtaccattc ggcgagttcg cgccgctctt 1022700
ggaggcggta gctgtcggcg gtcaggtagt ggtgcaggct tgagaagccg gcgcgttgga 1022760
gggcggcgcg gctgatgtgg ccgagggcga ggtctgttgt gagggtgtct tttccggcgg 1022820
tcaggctgat gttggtgaag aggtggcgga atccgtgcat tgtgtgtttg gatttgccgg 1022880
gggtgctgcc gtcatagccc agtcgtcgga tggcgttgtg ggcgaatttg atgctgatgt 1022940
ggtcgggatg gggggcgggt ttgcgccgtg ggcggatgcc ggggaaaagg tggatgttgt 1023000
cgccggtctg tgtgtgcagc tctcggagta tttctaccgc ccagtccgac agtaggacgg 1023060
taaaggggtg tttggtcttc atgtcggcgg cggggatgtg ccataaccgg gcggtgaggt 1023120
cgatgtcttg ccagcgggcg gaaagcagtg cggacggacg ggatgacttc cccggtttcc 1023180
ccatcaatcc gcgccgcctc gatttttccg ttggcgcgca gcatttccac cgcctgccgc 1023240
accgacatca accgcttgcc gttgcgcctc atcgcctcca ccaaaaccgc cgaaatcaat 1023300
ttggcttctt ccggtttaag ctccgtcttg cccgcatcgc tgcgccgttt gcgcgtcggc 1023360
ttgacgctga ccgcctccag cttgcggtat agcgtggcaa ggctgatgcc caattcctgc 1023420
gcctgctgct taagatatgc agagcgtgcg ccgcgttcca ttgcttccgc ctgattctcg 1023480
actgccttaa gacgctcaat cattgccgga ttcatcgcct tctcccgttt caccgcccaa 1023540
ccattccggc acattgtctg tcggtgcttc agtcggcagg gcatagcttt cgcgcagttg 1023600
ctcgcagtcc aaaataattt gattgagcgt gccgaccatc tttgcctgat ggctgatccc 1023660
gtgtgcctca ctgtgcgcat taagttggtc gaacaaatct ttcagacggc tcacttgact 1023720
gcggataccg acctcaaggc ttgttaactg catcgccaac tcgctgccta cgtcttccgc 1023780
cttcggctct ctgacaacgg tttgcttctt agccagcttc tcggccagct catcaatttt 1023840
ggctgttttg gttttcatca cttcgtcttt ggcggcgagg ttttcgcggc tttcgcgcag 1023900
ggcgacgcgc agctcgcgca ccgtcattcg gtccacatcg tcaaaggtca tgccgttgac 1023960
ttcttcccct tcggccaaac ccaccagcgt aacgtcttct tcgaccagca gctcaagcag 1024020
cttcgacttg cccaaatcca tcagcttcgg cgcggctttc tgcatttgcg gggtcgcaaa 1024080
gcggcgagtg gctgacatca gacgtgatgt ttctgcgata cccagcccaa attggctttt 1024140
cacaatttcc ataaaccgtc cgtgttccgt atgctctttt aaaataatca gcgcacgtcc 1024200
cagttcgaac atgccttcca tcgtctgccg tactgcttga cgaccgcgtt caacccaacg 1024260
ctcttcgcta taagtctcgc cgttacccca ctgctccatc accagcacac tgcgcatcgc 1024320
tgcctgattg cttactttgt cggttgcgat aatttcaatt tctgtattca ttttttatct 1024380
ccaaagtttc cgacgtcgga aactttcaaa atccgttaat ccacatcgac ccgcttgccg 1024440
atttcggcaa tcttgctttg cagccgttca tgctgctgcc tgaaccgctc tgcgatttgc 1024500
agggttttga tgccgtaggc gtagttgccg ttttcaagtt tgatgaccaa tcccgaggca 1024560
accaaatcat caatatccct gctgacttgc gatggcgtca gccccagtcc gaccgataaa 1024620
tccttattgc tcagaccgat aatcggatgc tcgtcaagcg cgataaagac cctcaatagc 1024680
cgttgtaccc ttttactttc tgccatccgc atcctcctta tttcagtccc agcttcttgg 1024740
caatttcgtg ccccttgccg taattgcctt tgcgctgtcc gccgatcacc agatacacat 1024800
cgcgcggctt aaagccgttc tctcttgccc actgcgccag cgtctggccg tttttggcaa 1024860
aattttcttt caattcgtct gccgtaagca tttctttggc ctgtacgcgg gaaaatttca 1024920
gtatccgtcc gtacggttgg ttaatacact catgattaat catcgctttt ctccttcatc 1024980
cccagcaaaa ccgccgcttc gtggcttttg ccaaaattgc ctttcagctt gccgcgcaag 1025040
aggtgctcca ccgtggtgcg ctccagattg aaatatttcg cccaatgcgc cttgcacacc 1025100
ccgttgcgct taaaccaccc ggccgcgctc tcgcgcgttt gcggatagga aataggcttg 1025160
aaattcaaag gtttttccat attatttatg cctttcgtgt gatataatgt gtttgttttt 1025220
aggtctcttt gcacgataaa catccggtct gttgtgcagc agcagggtct tggcttgttt 1025280
taaagtaaac aagcttttc tcgtagctgg gtgtatgtga tccatcatga gctgtcgcgc 1025340
atgagccttc aaagtgttca ttttttcgtc ctttctcgtg atgatttagg gtgtttgtgt 1025400
ttcgatgtgg aaattatagg aagaattctt cctattttgc aaggaatatt tatgaataac 1025460
tcttcctttt ttggcaaccg attgaaagaa gaaagaaaaa aattaaaaat gactcaagct 1025520
gaaatcgctg aaaaatgtgg ggtttcagga agaatgtggg gggattatga acgtggcatc 1025580
agccagccaa aagcggaact tttcttccaa tttgaaaagg tgggtataga cgttcaatac 1025640

gtcatgcacg gcagacgcgg cgaaacagcg gtcatgccgt ctgaaaccct gaacgccgaa 1025700
gaacaagaac tgctggtctt gttccgcgag gcggcagctg ccgaccgtga aatgattctg 1025760
atggttgcgc gcagggcaga gaaaaaagcc caaactgcgc ttggtaaagt gagtaatgga 1025820
taaaatggat caattcgaat tgtgccaaaa agaacatgtc aatccatttg ccttgtcaaa 1025880
gcaatatcta ttggttgtaa cattcgtcaa atccagcagt aaaaattttc aggcagcatt 1025940
actttgggca agaagtgcca aattatttga gaatcttgag attggaaaag aaaccatcta 1026000
ttgctgcgct ttcgataaaa cagcagaaca ggctgggatg gccggggtat ttttgaatta 1026060
tattgaaaat tggaatggca aacagattta catcaatggc cgaatccata gtggcagtat 1026120
ttatgatttg ttaggggttt tagactgcta tcaaaaatca cagtcctgtc ccaaccctaa 1026180
aagccactgt tgctttgttt cagacgacat tttctatgg catggatcaa gaccaacgtt 1026240
tgaaatcagt cttgatctaa ctggaaagaa aaaagaaaca tcctctgcaa agaaatttgt 1026300
gatgccttgt attaatttcc gtcaccatag gattgaaaaa gaaacctact taggaaattg 1026360
gaatgaacaa attgccgcat tggcagtaaa acaaaatata gattggtgtc caagttttga 1026420
tattgagaat tttagacagt atgaataatt actatctata taggaattgc agcagcgatg 1026480
tgttatgggt caaacgtatc caacgccaaa tcgacggcag cctactcttg atttctgaca 1026540
attcaaccta tccacccatg cccttggcac tggcggaaca ccccgatatt caaatcatcg 1026600
ggcaggtagt gcaggtatca aaagacttga actagacaca atcaaaaagg gaaatagaat 1026660
gaaaatactc gctttattaa ttgccgctac ctgtgcttta tctgcgtgtg gcagccaatc 1026720
tgaagaacaa ccggcatctg cacaacccca agagcaggca caatccgaat taaaaaccat 1026780
gccggtaagc tataccgact atcaatcagc agccaataaa gggctgaatg accaaaaaac 1026840
cggtctgacc cttcctgaac atgttgtccc tatcgacaat gcggaaggaa agaatctgct 1026900
gcatgacttt tcagacggcc tcacaatctt aaccgttgat accgataaag ccgacaaaat 1026960
tactgctgtc cgagtagtct ggaatacaga tgcaatgcct caaaaagcgg aaaaactgtc 1027020
caaagctgcc gcagccttga ttgcggcaac cgctccggaa gaccgcacaa tgctgcgtga 1027080
taccggcgac caaatcgaaa tggcgattga cagccataat gcgcaaaaag agccaacccg 1027140
agaatgggcg cgtggtggga ttgcttataa agtcactgtt accaatttac cgagcgtggt 1027200
tttgacggca aaagctgagt aaatctatta agtagaaaaa atagaaaggg aaatgatgat 1027260
tgagaaaagt atttctattg tagatggaaa ggaatactcc gtttttgctg tatcacacga 1027320
gtttcgttat acctttgatg agcctatttt agtcgctgac ttgattagtt ctctaaaagc 1027380
ttatgaaaca ctgacaagta gttatcttcc agcaattttg aatcagctgt ttgatgtcaa 1027440
aatccaaaaa atcaaagtag ctgtatctga aattgaaaga ggatctttcc ttgaaaaact 1027500
gattttcaat ttattcttca aagatgaaga tgcttataat gaattttgtc ttaaaatacg 1027560
aaaatttcta ggaacagaaa atcaggacgg aagtattaat atgtccaaaa tcattatgtt 1027620
tgcaatgact acactttttag gggtaggtgc tggttatctc ttgtttaaaa acccgccaca 1027680
agagaagcag gcaataacca acaacatcgt taccgtcatt aatgctgata gttctgtcgc 1027740
actggatggt gaacatttgg tttcagtggt aaaagaagta acaggaagca gcaagcaaaa 1027800
aactgcagaa aatgtggcaa aagtatatgc tccagcaagt aaaaataatg gcagtattac 1027860
ccttgggaca gatgatgttc ggattgaacc tgttgcacaa caaactgtag caactttgcc 1027920
taaagatgtg gacttacgtg atacgccatt gactgaagat tacaccgata ttgatgtgca 1027980
aattcgtgct actgaccgtg ataaaaattc agggtggtat gcagtcatag accaaattgt 1028040
tccatcacgt gttcgattag aactgcctga agatattgat ttgaataggc tggctaacaa 1028100
tgctactatc cgtgcaaatg taacagttga gtttgactta aagcaaaatg gctctcgtaa 1028160
gcctaaaaaa atcatcctca catctctctc tactgattaa gttttaaccc gtattaaagg 1028220
cttagtcaga cggcctttcc tacaatccct gtattgattt ttaattcaat acagggattt 1028280
ttccatgtca gacaagttca accaattcat caaccgcgtc ctctctcacg agggtggtta 1028340
cgccaaccat cccaaagacc ccggcggcga aaccaattgg ggcatcacta agcgcaccgc 1028400
acaggcaaac ggctacaacg gctccatgcg tgccatgacg cgtgaacagg caatcagcat 1028460
ttaccgtaaa gcgttttggg agcgttaccg cgccgaccaa atgccggaag cggtcgcgtt 1028520
ccaatttttt gatgcctgcg tcaaccacgg ttacggcaat gccgcccgta tgctgcaacg 1028580
cgccgcaggc gtaccggacg acggcgttat cggagcagtc agcctcaaag ccatcaattc 1028640
ccttcccgaa aacgaccttt tattgcggtt caacgccgag cgtctggtct tttataccaa 1028700
gctcggtacg ttcacctctt tcggcaaggg ctgggtacgc cgtgtggcgc aaaacctgat 1028760
tcacgcgtct gcagataaca ctgattaaag ggagataaac catgtcaaaa aagtcactca 1028820
tcgccctaat gaccgcagcc atgcagcccg atttcagcca cagcgaccta ggcattcgct 1028880
acgccatgcc gactcaggga tgttggacgc aagcccaccg caagagcggg gtagccgccg 1028940
cgaaacgcgc agccaaaaaa aagcgtcaca aataaccgcc tttttccgat ggctgggcgg 1029000
cttggtctct aatccggcca caggaaaaat cagccatacc aaactatggg caaacgtcgc 1029060
cgcagccgcc atgacttgga agttcgtgca ggcggcggac gcgcccgaat ggctctagtg 1029120
ggcttatggc gcattggtcg gcgggtatgc attaatcaaa cgcggcatcg cggcgattcc 1029180
gcagttggca gaaatcaaaa aatccgcaaa tcaggaaggg gggcggcaat gattgaattt 1029240
gtccgagcca aaaaacggct gctttgggca tttgtgcttt tgcttgtgtg gacgtgcggt 1029300

taccgatacg ccgccgacaa ggccgaagcg aaacaaaccg ccctgattgc cacctatcgg 1029360

cattcttcta tggttgcggc ggaacaatat gccttgcagc ttaaaaaagc gcaggacgaa 1029420
aggcagcggt ggtacgactt ttcccaaaaa caaggaagaa agcccgtgaa aaaacagtat 1029480
ccgccgcaaa cgaaaaaagc cggctatctg aaaaccaagg aagaactgct tgcggaattg 1029540
gcttgcctta aagcggaaat ggttgcccta aaaaagcccg atgccttaat ccatgggaaa 1029600
gaagtgcggc agaaagaacg caactcgtcg cagggttaag gcaatgccat ccgttgaact 1029660
gctgttggag attgtccttc tattaccaat tggccgtcca atcggcagaa gacaaatatg 1029720
ccgatttgaa acggcatatc catgatattt atcgacgaca taagggaaga tacggctacc 1029780
ggaggattgc ggcagccatc cgtcacgcag gaacaccggt caatcacaag aaagtcagcc 1029840
gtctgatggc gaagacgggg ctgaaggcag tgatacggcg gcgcaaatac cgctcgttca 1029900
aaggagaagt cggcaaaatt gcgccgaata tcctgcgacg ctgtttccat gcagaaaagc 1029960
cgaatgagaa atgggtaacg gacgttaccg agttcaatgt aggcggagaa aagatatacc 1030020
tttctccgat tatggatttg tttaacgggg aaatcgtcag ttaccgtatt caaatccgcc 1030080
cgactttcga tttggccggc gagatactga aaggtgcgcc ggagaaaccg ggatcgtctg 1030140
aaaagccgat actgcattcg gatcaaggtt ggcaatatca gatgtttttt atcaaaagca 1030200
gttgaaaggc aacggtctgg ttcagagtat gtcccgcaag ggaaactgct tggacaatgc 1030260
ggcaatggaa agtttcttcg gaacgttgaa atcggaatgt ttccatacgt gcaaatatga 1030320
ttccgttacc gaatcgtaag cggcactgca cgaatatatc cgttactaca acaacgatag 1030380
aatcaagttg aaattaaaag gactgagccc tgttcagtac agaactcagt ccctgaaagc 1030440
cgcttgatta aactgtccga ctttttgggg tcagttcggc ttcggcattt ttttatccgt 1030500
tttgggggta acttgtttgg aaagctgcaa gcttataaat aaaggattac atttaagttt 1030560
tgggtaacct ttttaaaaaa atgcgtgatg acttttgcat ttttaaggcg ttttttgggg 1030620
taattcgtga aaagttaccc caaaagttac cccataaatg gcgaaaactc aagcatacgc 1030680
cagcatcctg caacacaaaa aagccttgaa actgttgaag ttcaaggctt ttttgtgttg 1030740
caggatgctg ctgaaaatag ggtatggtgg aggcgggggg aatcgaaccc ccgtccgaaa 1030800
gtcctctaca aagcgttcta catacttagt tgtgtctatt tgaaaatctt atttccatca 1030860
tgccgaccaa caggcctttt ggaaaccagt taccttaagt cttatttcct gccaagtaac 1030920
ccggtaggaa accagtcaat gtaagatgac gttgcggtgg ctttcgccac acagcccatt 1030980
gaccgactgc tgcaacggct agccttaagc ggctaaagcg taagtttcgt cgtttgcgac 1031040
tatttgaatt cagtgtttta cgggaatctg agaccccggt atgcccgcat ctgcttcgca 1031100
accctcgtcg aaaccaaggt cgcccccaga aatggtttgc aaattatacg gatattgtgc 1031160
ggtgctgcca agtctgtcgg agaaatttgt cagtcttgct gccttaattt gcgtttgagc 1031220
aggatgcgga cgcagccgtc gttgccttcc cggggttcgg cgtaggcgag tacgtcgggg 1031280
tgttgcatca gccagtttcg ggtcatattt ttcagaacgg gtttgtagcc tttggaacct 1031340
aatccgctgc cgtggatgat ttcgccgcat acgccgcgtt tttgggtgaa tgcgatgaat 1031400
tcgttgagga ctttttgggc ttcttcctgt gtgtagccgt gcaggtcgac atcggtaacg 1031460
acgggatagt atccgttttt caggcgttgg atgtcgtttt ttccctgtcc gtttttgctg 1031520
aagctggcgg gcgggtcgtt gtatgtgctg ccgatgtaga agtagttttc ttcgtcggcg 1031580
cggttgtctt tgggacggac tttgatgggg gttttgtcgg gcggcgcata atattgctgc 1031640
cggtttttta atggggagag ttgtccgact gcttgtgaaa aatcgaaatc ctgttcttgt 1031700
ttttgcttgt ttttttcggc tgcctgtttt tctgccgctt ctttttgggc ttgtttgccc 1031760
agttgtttga ggatgttttg gaagtcggta ttcatatttt ttcctgttat ttgtccgatg 1031820
gctgtttcgg gcggggtttt aatttgccgg aatgtttgcc aatcggggga ggatgatttt 1031880
gttgcctgcg tatgtttttt gaaagtgtga ttgtatatca aaaagaaatg cggcaaccgt 1031940
cggcagtgtt gattgccgga aatgcggacc ggtcgaaccg atatgcccga acgcctgata 1032000
aagttttaaa aacctgcctt gcgaagcagg ctgacgtgtt ttgccaatct tgaattgccg 1032060
gaaacgcgaa acacggaaat ctgatgtttt atagtggatt aacaaaaatc aggacaaggc 1032120
gacgaagccg cagacagtac agatagtacg gaaccgattc acttggtgct tcagcacctt 1032180
agagaatcgt tctctttgag ctaaggcgag aacgctgtac tggtttttgt taatccacta 1032240
taaatgttcc gatacgaact gcaaaatatt ggttttgttt ctgacaggca aaagcactgt 1032300
ttatttggct gtcaaaagga tggttaagga aagttatgcg cccctgaagc gggccccaga 1032360
taaggatggt tgcgccgacg gcttcagacg gcattttggc ggcggtgttg ggttttgtat 1032420
ccggtttgcc gttgtgtttt gtgatgatga ttttgggcgc ggtttttctg ttttgatgtg 1032480
tgaaatgccg tctgaaaggc ggttcagacg gcatagcggt catttttgtg cggtcaggcg 1032540
gtcgaatatg ccgccgtcgg cgaagtaggt tttcatgatg ttgtcccatc cgccgaattt 1032600
tttttcggga gagaaggtgt ctaagtctgg gaagtcggct ttgtgtcttg ccaatacttc 1032660
ggggttgcgg gggcgcaggt agagtgaggc ggcgagttct tgcgccggtt cgctccaaag 1032720
gtattcgaga taggcgcggg cggttttttg cgtgcctttt ttcgcgacga cgctgttgac 1032780
gacggcgacg gggctttcgg cggaaatggt gtagctcgga tagacgattt caaactgtcc 1032840
ttgggtcagt tttttgctga cgtagttggc ttcgttttca aaagtgatga gtacgtcgcc 1032900

gatgttgcgt tgtgtgaagg tggtggtggc ggcgcgtccg ccgttttcaa aaacgggggt 1032960
gtttttgagg atggatgcga cgagtttttg ggcttcctgt tcgttgccgt tggtggtttt 1033020
cagaccgtaa ccgtatgcgc cgaggaaggc gtagcgtccg ttgcccgagg ttttgggatt 1033080
ggcgatgacg atgttaacgc cgtctttggc aaggtcgttc caatcgcgga tctgtttggg 1033140
gttgtttttt cggacaagga aaaccatagt gctggtgtag ggcgcggcgt ggtcggggag 1033200
ggcttgttgc cagccttttt ctaccagtcc tttttttttcg agcaggtcga tgtcggagga 1033260
ttggttcatg gttacgacat cggcttgaag gccgttggct acggataatg cctgtttgct 1033320
ggagccgccg tgggactgtt ggatgctgac ggatgtgccg gggtgttcgg attggtatgt 1033380
tttgataaat aaggggttgt attctttgta aaaatcccgt gccacatcgt atgaggcgtt 1033440
gagcagggta atgtttttttc cgtcggattc ggtattggcc ggggcatttt gtccggacgg 1033500
atggtttgaa tcggctgcgg ggctgcaggc ggtgagcagg gctgcggtat agagtgccgg 1033560
tgcgtaggtt ttcatatgct tgtcctgtcg gttggtagat ggggcaactt tatacggctg 1033620
tctgcgcttg tggaaataat gtttgatttg aagattatca gttttggtta taaggacgga 1033680
tcagaggtgt ttccgcatca gttcgcattt gattttgatg ctggggtcaa gctgcaatac 1033740
tgccgaaccg agcgattcgt agcggttgag aaggtaaaac gggacggagt tgagcgatgc 1033800
gtagagtttc agaaagctca aaccggattt gtgggcgatg gtttccgcct gatgaagtag 1033860
ggcagtgccc agtccgaggt tgtggaacag ggggtggacg taaagtgcat cgagttgtgc 1033920
ttcttggcag tcgatttgga aaaatccctg tatgttgcct ttgtattcgg caacccaaag 1033980
tgctttgtcg ggatcggaaa tggtcggcag gtagctttct gtgtttagca agccttccca 1034040
tacttttagg gcgtgttcgt tgtagctgag gatgcaggtg tattggacgg agtgcaggtg 1034100
gactttgaag atgtctttgc agtcttgcac ggtggcgggg cgtaacagtg tcagcaggct 1034160
catggcggta tgtcggcggc ttcagacggc atctgtgccg ttggtcggat tatagggact 1034220
gatgcagttt ttttgcttct tgaaatgcgg tgtccgaatc ggtggttaaa acggtaaagt 1034280
gtcccatttt ccgccctttg tgcgcggttt ttttgccgta aaggtgcagg tgtgcattcg 1034340
gatggctttg caagggcagc caatccggtt cgccgccgtc ttcctgccaa acgtcgccca 1034400
aaatatttgc catacagcaa gaactcagta atttggtatc ggcaggcggc aggttgcaca 1034460
taatgcgtac ctgctgctgg aactggtctg ctgcgcaggc atctatcgta tggtgtccgg 1034520
aattgtgcgg gcgcggggcg atttcgttga cgaccaattc atgcgtgtca ccgacaacaa 1034580
acatttctac cgccaatacg ccgacataat ccaattcgtc cgccaagcgt tgcgccatct 1034640
gccgcgcctg ttgctgcacg tcggcactca gtcgcgcggg gacgatggaa taagccaaga 1034700
tgccgttttc gtggatgttt tcggcagggt cgaaagtttg cacgttgtca ttgttcaaac 1034760
ggcatacgat tacggaaatt tcactgcgca aatccaccat tttttccaaa acgcaatcca 1034820
cgccgccgtg ttcggcaaac gcggctttga gttcatccaa tgtttttacg cggatttgac 1034880
ctttgccgtc gtagcccaac gtagccgttt tcaggatgcc gggcaaaaat tgcgcgcttg 1034940
cttcagtgat gtcttcagcc ttacaaacca cttgatacgg cgcggtttgc aatcccgctt 1035000
tgcgtatcca tgccttttcc tgaatgcggt tttgtgcaat cgccacacaa tcgccgctag 1035060
gggaaacatt ggtatgtttt gccaaaaagc gcatcgcatc ggcattgacg ttttcaaatt 1035120
cagtggtcac cgccgcgcat tttgccaatt cgtccaaagc agcttggtcg ttaaacggcg 1035180
cgcacaaatg gcggtcggca aattctgctg ccggcgcgtc cggatcgggg tcgagaacgg 1035240
ttactttgta gcccatggtt ttggcggcaa cggtaaacat tctgcctaat tgtccgccgc 1035300
cgaggatgcc aagcatggcg ggcggagaaa gagatatgtt tttcatgctg actcttcaaa 1035360
ttgtacaagt tgatagctat aactaattct tgacggatgt cttgtatcgc tggaattacc 1035420
agtttcagaa atacagaata ctttttccat aaatttttct gcttttagaa attccagtat 1035480
tctgtttttt tcatccttat aagcaccgcg gtctgtaccc catgcaagaa taatcatatc 1035540
agcatcttcc aaacatcctt taaatttgga aaaatcggtt tgggtgtttg ccctaattcc 1035600
tgtttgctgt gtagagtaac tggaaaaaat atttaacatt ttgaagttgg taaaaccgta 1035660
catatccaag aaacgtgcaa gttgggtaag ggttttgtcg cttctttcat catttgcttt 1035720
acttggatta attcctatag cgacagctga gaaattttta ggattttctg tgttgccgct 1035780
ccatctaacc gttaggattt ctcgattttt ttcattatct gtatagagac cgtctttggt 1035840
agtcggtctg agtgtttggc gaagctcata aagtttttca taagtcattt atccaaccct 1035900
tcctgtacca tttgcgcggc gtgtatgacg gctcgggctt tgttttgtgt ttcctgccat 1035960
tcggaggccg gatcggagtc ggcaaccacg cccgcgccgc tttggacgta tagcgtgttg 1036020
ttttttacta cggcggtgcg gatggcgatt gccaaatcca tgtcgttgtt gaaacccccat 1036080
acgccgacgg caccgccgta gatgccgcgt ttgctcggtt cgacttcttc gatgatttcc 1036140
atggcgcgga ctttgggtgc gccggagagt gtgccggcag ggaaggtagc ggcgaggatg 1036200
tccatgttgg tcatgccgtc tttcagacgg ccttcgacgt tggaaacgat gtgcattaca 1036260
tgggagtatt tttcaatcac cattttgtcg gtaactttga cttcgccggt tttactgatg 1036320
cggccgacgt cgttgcgtcc taagtcaatc aacatgacgt gttcggcgat ttctttggca 1036380
tcgcttaaca aatcttgttc gttggcaagg tcttcggcgg gggttttgcc gcgcaggcgc 1036440
gtgccggcga tggggcggac gataacgtcg ttgcgttcgc gtcggacgag gatttcgggc 1036500
gaggagccga cgatgtggaa atcgccgaaa tcgtagtaaa agagataagg cgaagggttg 1036560

agcgtacgca gggcgcggta gagggcgagc gggctgtcgg tgaattccat gctcatgcgc 1036620
tggctgggga caacctgcat gcagtcgcct gcgaagatgt agtctttgat tttgttaacg 1036680
caggctttga acggctcttc gccgaattcg ctgacggctt cggtgtgttt gctgccgagc 1036740
gagagcggga tggcgcagct ttggcgcaac tgggtgcgga tgtcttcgag gcgttcgcgg 1036800
gcgcgttcgt agccgtcggg ctgcgacgga tcggcgtaaa cgacgagatg gattttgccg 1036860
ctcaaattgt cgatcaccgc caattcttgc gacagcatca gcaagatgtc gggcgtgccg 1036920
agcgggtcgg ctttggtggt gtttttcagg cggtgggcga agtgttcaaa attgtagatg 1036980
gtttcgtaac cgaagtagcc gaccagtccg ccggtaaagc gcggcaggct tgggatttcg 1037040
ggtgttttga agcggttgtg gaaggcttcg ataaagggca gggggttgcc gtcgtgttgc 1037100
tcgacaattt cgccgttttg ataaacatcg acgtgtttgc cgctggcttt gagatagtgg 1037160
ctgcaaggca ggccgataaa agaatagcgg ccgaaacgtt cgccaccgac aacggattcg 1037220
agcaggtagg tatagggggcg gttggcgagt ttgagataga gggaaagcgg cgtatccaaa 1037280
tcggcaagga gttcttgcac gagcgggatg cggttgtagc cttgggcggc ttgggcttgg 1037340
tattcttgtt tgctgatcat ttctgctttc ccaaagggcg gtttcggacg gcgcggcaac 1037400
gggcgcgagt atagcatttt atcggaattg ttgacagtct gaccggagat gcccttggat 1037460
tcggatttca agtgcaacac tagtgtatta gtggttggaa cagattcaag aataaaacac 1037520
ttggcgtttc gtagccaagt gtttttcttg gtcggtggtt caactcatct tgaaccctgc 1037580
gtatctcccg atcactgatg ttacggaaat cggtttgttt ggggaagtat tgccggatga 1037640
gtccgttggt gttctcattc agccctttct cccaagaatg gtaagggcga caaaaataag 1037700
tctccgcttt caatgctttg gttattttgg tgtgttggta gaactctttg ccgttatcca 1037760
tggtgatggt gtgcaccctg tctttatgtg cctttaatgc cctaacagct gcccgggcag 1037820
tgtcttcggc tttgaggcta tccaatttgc agatgatggt gtagcgggta acgcgttcga 1037880
ccaaggtcaa taatgcgctt ttctgtcctt tgccgacaat ggtgtcggct tcccaatcgc 1037940
cgatacggga tttctggtcg acgatagcgg gtcggttttc tatgccgaca cggttgggta 1038000
ctttgcctct ggtccatgtg ctgccgtagc gtttgcggta gggtttgctg catattctga 1038060
gatgttgcca caacgtgctg ccgttgcttt tgtcttggcg aaggtagcgg taaatggtgc 1038120
tgtggtggag cgtgatctgg tggtgtttgc acaggtaggc gcatacttgt tcgggactga 1038180
gtttgcggcg gataaggggg tcgatgtgct gaatcagctg cgaatcgagc ttatagggtt 1038240
gtcgcttacg ctgtttgata gtctggcttt gccgctgggc tttttcggcg ctgtattgct 1038300
gcccttgggt gcggtgccgt ctgatttcgc ggctgatggt gcttttgtgg cggttcagct 1038360
gtttggcgat ttcggtaacg gtgcagtggc gggacaggta ttggatgtgg tatcgttcgc 1038420
cttgggtcag ttgcgtgtag ctcatggcaa tctttcttgc aggaaaggcc gtatgctacc 1038480
gcatactggc ctttttctgt tagggaaagt tgcacttcaa atgcgaatcc gccgccgtct 1038540
gaaacgccaa acgggcttca gacggcattt ttgacggcgg aggtctatga gccgcaggtt 1038600
ttcggcttgt tcgccagaat attgatgact ttgcgttcgg ctttttgcgg ctcgattttg 1038660
atttcgctct cgtcttcttc gctgccgtct gaaaaacgtt cgggcatttt ttcgctgtca 1038720
aacgccaaat cgccgccgtg tttcaggctt tgaccgcgtt ccaatccgac aaagtcgaag 1038780
agttcggtat cggcaaggtg ggaagggacg acgttttgca gggcggagaa catcgattcg 1038840
atgcggccgg ggaagcgttt gtcccaatcg cgcagcatat cgccgatgac ttggcgttgc 1038900
aggttgggtt gcgagccgca gaggttgcac gggatgatgg ggaattgttt taattcggcg 1038960
tatttgatta agtctttttc tttcacatac gccagcgggc ggatgacgat gtgttcgccg 1039020
ttgtcgctca ccagcttggg cggcatggct ttgagtttgc cgccgtaaaa catatttaaa 1039080
aacaaggtgg cgaggatgtc gtcgcggtgg tgtcccaagg cgattttggt gcagcccaat 1039140
tcttttgcag tgcggtagag gatgccgcgg cgcaggcggc tgcacagcga acaagtcgtt 1039200
ttgccttcgt ctaatacgcg tttgacggtg gagtaggtgt cttcttcaac gattttgtag 1039260
ggaacgccga tgctttcgag ataggtcggc aatacttctt cggggaagcc cggctgcttt 1039320
tggtcgagat tgacggcaac cagttggaaa tcaatcggcg cgctggcttg gagctggcgc 1039380
aggatgtcta acagggcata gctgtctttg ccgccggaga ggcagaccat gattttgtcg 1039440
tccggctcga tcatattgaa atcgttaatc gcgtcgccga cggcgtggcg caggcgtttg 1039500
ctgagtttgt tgttttcgag ttcttgtttg gtttttttgg acatggcggt ttgggtttga 1039560
aaattagaaa ggcggcattg taaccgattg gcggggcggc aatgccgtct gaagggcttc 1039620
agacggcatc ggcggcttat tctgcatttt cggttttaaa gaagagatga accgctttga 1039680
agataccgcc gtttgggacg gtcagtgttt tttgtgcggc ggagaattta atcacggtaa 1039740
gggcggtaaa gttttccgaa tcttgaacgc tgtcgagtac gatgccggcc tcttcgccgt 1039800
ccgccgtcag cagggttcct gcttcgacgg ccgaatttcc cgacaatacc gccaagccgc 1039860
gtttgacctg cccccgatac tgggcacggg caatgatttc ctgtcccgga tagcagcctt 1039920
ttttgaagtg tacgccgccg atgatgtgct ggttgagcat ttgggcgacg gcggtttctt 1039980
tggtagccgc gcatatccac ggataaccgc tacggatttc gtgcagccgc cacgcgtttt 1040040
cggcggcggc atcataaggc ggcaaggcgt tttgggggc gatgtgcaaa atgccccgat 1040100
gtggcaggac gacggaacag atgccgtctg aaccgcattc ggcggtaaag gcgaggctgg 1040160
gttcttgcgc ggcaagcggt tcggcggatg cttctaattc cgcgccgacg gcgtaatctt 1040220

caaggatttc aaaaacggct ttggcgcgta acacaaacat ccgcaaacgt ttgaccgttg 1040280
cttcaagcag gtcttgcgcc ataatcagca gcaaatcgcc gcctcggttg acgacaatca 1040340
tattggcgat gacgcggcct ttgggcgtgt tgtaagtcgc ataacacgcc tgcccggtct 1040400
gaaggtggtt gatgtcgttg gaaagctgtc cgtgcaggaa ggtttggcgg tcttcgccgc 1040460
tgacgcgcac cacgccgaaa aagggcagta aggttttcat catttgcgta ctctgaaata 1040520
taaaggaaat ctgtttatgc agttgccgcg tctctcttca cggcggttat tttgatttcg 1040580
acgcgccact ccggacgggc gagccgtgct tccacgcagg cgcgggcggg cgttctgccg 1040640
gcggcaaccc aagcgtccca cacgccgttc atttccgcat agtcgcccat atcgcgcaga 1040700
tagatgaccg catccaaaac gtgcgctttg tccgagccgc attccgccag ccagcggtcg 1040760
atttgggcaa gcacgtcggc agtctgttcg gcagccgttt caccgttttc gggaaccatg 1040820
ccggagagga aaatcaagcc gtttgcgccg acggcttcgg aatagcgggg cgttgtgccg 1040880
aaatatcgga tatccatatc ggtttccttc gataaagggg atatatggta acattgcgct 1040940
tgaccgattt ccatgttttg catgacgaaa aatgagtaaa cacacttatc cgataacacc 1041000
tgccgtgcgc gttttgcgtg aaaacggcat cgaatttgaa ccttttacct atgcctatga 1041060
ggaacacggc ggcacggcgc agtttgcccg actattcggc aaagacgaac acttggtcat 1041120
taaaaccatt gttttgcaag atgaaaacgg taaggggctg attgtcttga tgcacggcga 1041180
caagcagatt tcaacccgca atctggcgcg gcatttgggt gcgaaacaca tcgaacccgc 1041240
cacgcccgta caggcaaaca agtggacggg ctatctggtc ggcggcacaa cgccgttcgg 1041300
catccggaca aagttggata tttacgtcga acagtcggtg atggatttgg aaaccatcta 1041360
tatcaacggc ggaaaacgcg ggttcattat cggcatccgt cccggagatt taaatatttt 1041420
gaacccgaaa acaatacagg cggcggtttg acgggaaagt ataaaggaac aatatggaca 1041480
aagatttgta tgccgtattg ggcgtgtcgc cgcaggcggg agcggacgaa atcaaacgcg 1041540
cctaccgcaa gctggcgatg aaatatcatc ccgaccgcaa tccgggcaat ccgaaggcgg 1041600
aagaaaagtt caaagaaatc caacgggctt acgatacgct ttccgacctg tcgaaacgga 1041660
tgcaatacga cgcgtccttc agacggcatg aggaacgcgg gcggcaggaa gaggcattcc 1041720
gccgcgaaca ggcgcgcagg gagcagtttt accgcgaaca gatgcgccgc gaacaggcgt 1041780
tcagacaggc gtttgaacgg caggcatcac gttcgtgcca tacttacgaa ccgtccggcg 1041840
gcggaagcgg gcgcaactat gtcctcgccg cctacatcct gttcggtttg ggtgcaatca 1041900
tgctgttcat gcccatagtc ggcgtgattt tcgcctatat gcccatagtc ggcgtgattc 1041960
tcgcctatat gaaacggaac agtttggaca gcattgtcta tgccgcacat accgaatacc 1042020
tgattaaaac cttttggcgc acattttggc tttatatttt gggtgcgctg actgcccttt 1042080
tgggtatcgg cgtgctgatt attattgcaa cgaacgtctg gtatttctac cgcatcatcg 1042140
ccggctttat ccgcttcaac ggcggcaggg cggttgcacc cgagaaatgg atatagtatg 1042200
gcttacctgt taatcagcat cgtgttcagc gtgtcggttt ccattttgct gaaaatggca 1042260
aggaagaaaa aaatcgacat cgcgcaggcg gtcgccgtca attatgtggt cgcggtcata 1042320
ctgaccctgc tggtattgaa gccggatatc ggcaatatcg gcgcattttt gccgacgtgg 1042380
ccgctgtttg ccgctttggg cgtgctgctg ccgtccgtat tcgtgataat gggcaaatct 1042440
gtggaagccg ccggtatcgt caaatccgac gcggcgcagc gtttgtcgct gtttttgccg 1042500
attgttgccg ccttgacgct gtttggcgaa aaactcagcg aaggcaaact aatcgggctg 1042560
tgcctcgcat ttgccgcact gttctgcctg ctttggaaac acagcggtgg caaaaaatca 1042620
ggaagcgcgt ggcggcaggc ggcattgctg ctgggcgtgt gggcaggtta cggcattatc 1042680
gatatcctgt tcaaacagct tgccaaaagc ggaacggcat ttgcgggcaa cctgctggtt 1042740
gcatttgtgc tggcgggtgt gctgatgttt gcctgcctgt ttgccaaatc ggtcagatgg 1042800
cgtgttgaga gtgtggtcgg cggcatattc ttgggcggtt tgaattttat gaatatcgta 1042860
acctacatca ccgcgcacca aatgatgaag gataatccga ccttggtttt tgccggtatg 1042920
aatatcggcg tgattgtttt gggtacgctt tcgggcgcat tgttctttaa ggaaaaaatc 1042980
aacacaatca atacggcggg aatcgtgttg gcactgtgtt ctatcgcctg cctgttttat 1043040
tggggggaag tcaaggtact gttcggcata taatccggct gattgatata acaaaatgcc 1043100
gtctgaagca gcatccctgc ttcagacggc atttgtctgc aacgttacag atggggggttc 1043160
atcaggttct cgggagagag gatgcggttg agttcttctt cgctcaacag cccgcgttcc 1043220
aagacaacct cgcgcacgcc tttgccggtt tgggcgcaga ttttgccgac caaatcgccg 1043280
ttgtggtgtc cgatatacgg attcagataa gtcaccaaac cgatggagtt gaaaacgtaa 1043340
cgttcgcaga tttcgcggtt gaccgtaatg cctttgacgc atttgtcgga caggttgact 1043400
gcggcattgc ccaagaggga aatggtttca aacatacatt gggcgatgac cggctccatc 1043460
acgtttaatt gcagttgccc ggcttcggcg gcgaaggtaa tcgtcgtgtc gttgccgatg 1043520
actttgaagc agacttggtt gaccacttcg ggaatcacgg gattgacttt ggcgggcatt 1043580
atggaagaac cggcctgcaa ttcgggcagg ttgatttctt tcaagccggc acgcgggccg 1043640
gaagagagca ggcgcaagtc gttgcagatt ttggagagtt tgaccgccgt gcgcttcaat 1043700
gcgccgtgta ccatcacata tgcgccgcag tcggaggtcg cctcaatcag gttttcggtc 1043760
agtttgcaag gcaagccgct gacttcggag agttttttga ccaccagttc ggcgtagcct 1043820
ttgggcgtgt tcacgcccgt gccgattgcc gttgcgccca aattgacttc caacagcagt 1043880

tggcgggtgc ggtcgaggtt gaggatttct tcttccaaca acacttggaa agattggaat 1043940
tcttggccgg cagtcatcgg cacggcatct tgaagctggg tgcggcccat tttcaaaacg 1044000
tctttaaatt cttcggcttt ggcggcaaag gcgtttttca ggacagtcag tttgtcgagc 1044060
aattcgccga tgctgtaata cacggcaagg cggaagcccg tgggataggc atcgttggtc 1044120
gattggctgg cattgacgtg atccatcgga ttgacgatgt cgtagcggcc tttttcgtat 1044180
cccaagactt ccaatgcgag gttggcgatg acttcgttgg tgttcatatt gaccgaagta 1044240
cccgcaccgc cctgatacac gtcggacggg aattggtcga ggcagcggtt gttcagcaga 1044300
acttcgtcgc aggctttttc aatggcggcg gcgatttcgg gcttgaccgc gcccaactca 1044360
ccgtttgcct gtgccgtcgc ctttttcacc atcaccatac tgcggacaaa ctgcggcacg 1044420
tcagaaattt tttgtgtgga gattttaaag ttttcaatgg cgcgcagggt gtggatgccc 1044480
caatacactt cggcgggaat ctcgcggtcg cccaataaat cgtgttcgat acggacagtc 1044540
atgtttttac ctttgtaagt cggataatta atattgaaaa aatgcgccat cggaaagatg 1044600
ccgccgcagg atgaacacta taccggccgg atgaaattgt ccatatcgta tgccgtctga 1044660
aaacgggaaa cgttgttttc gggtgttata gtggattaac aaaaatcagg acaaggcgac 1044720
gaagccgcag acagtacaaa tagtacggaa ccgattcact tggtgcttca gcaccttaga 1044780
gaatcgttct ctttgagcta aggcgagcca acgccgtact ggtttttgtt aatccactat 1044840
actttccgga ctttccggca agccctgcct gccgctgaaa tatctttcgg cggattgtgc 1044900
tccgccatat cggcttaccg ttggcggggc ggtttgatga agacggcaca aatgccgttt 1044960
gaaggacgtt cagacggcat ttgtgctgat tcgcatcaag atttattgtt tggctgcctc 1045020
gatttggatg tcgatgcgga cgcttttggt cataccaacg ttaacgaggt agtccatgcc 1045080
ccatttggtg cggtcgatgg tggtgctgaa gtcgccgcca caaacttcgg ttttctccat 1045140
cgggctttgg tagcagttga atttttcggc tttgagtttg acgggggcgg ttttgccgtg 1045200
catggtcagg ttgccgtcaa cggaaaccag ttttttgccg ttgaagttga atttggtgga 1045260
aacaaagcgg atgtccggat attgggcggc atcgaagatg tcggctgatt tcaggtggtc 1045320
ggtaaagtgt tgcgaaccgc tttgcaggtt ggcaatgggg atggtgatgt cgattttacc 1045380
gtcgcgtttt gcttggtcga actcgacgga accggtcaga ccgtaaaaac cgccgacgtt 1045440
ggtgctggtg ttgaaatggt cgatggcgaa acgggcgttg gcgtgatatt cgtccacttt 1045500
gtaggtggcg gcggaggcag tactgatggc ggcggctgcg agtgcggcga agatgatttt 1045560
tttcatgatg ataatccttt gtgtgggccg gtaaaggcgt ttatcctaac ataggcaggg 1045620
atttatggca tttcctgccg gtaaacggtg ggtttgcagg cggttgacga acggggcggg 1045680
cggaaaaggg cggatgaaaa agcgcggtgt gatccgcgct ttgttttttt acaaggcggc 1045740
gagtaccgca tcgcccattt cggagcagga aacgagtttt gtgccttctt cgtaaatgtc 1045800
gctggtacgc aagccttgtt gcagcacttt ttggacggcg ttttcaactt gttgcgcgcg 1045860
cgcttcgtcg ttcaggctgt aacgcagcaa cattgcgagc gagaggatgg tggccagcgg 1045920
gttggctttg ttttgtccgg cgatgtcggg ggcggagccg tgagacggtt cgtacaggcc 1045980
tttgccgttt tcgtccagcg aggcggaagg cagcataccg atggagccgg tcagcatgga 1046040
ggcttcgtcg gagagaatgt cgcccgaagat gttgccggtg gcaatgacgt cgaattgttt 1046100
gggcgcacgc acgagctgca tggcggcgtt gtcgacgtac atatgggaaa gctcgacatc 1046160
agggtactct ttgccgattt cttcaaagat ttcgcgccac aattcggtgg tttccaaaac 1046220
gttggctttg cctacggaac agactttttt gctgcgtttt tgggcggatt ggaaggcaac 1046280
atgggcaata cggcggattt cgctttcgct gtatttcatg gtgttgtagc cttcgcgttc 1046340
gccgttttcc agaacgcgga tgccgcgcgg ttcgccgaaa tagatgtcgc cggtgagttc 1046400
gcgcacaatc aaaatatcca aaccggcaac gatttcaggc ttcagcgtgg aggcgttggc 1046460
taattcggga tataaaacag caggacgcaa attggcaaac aggttcaaat ccttacggat 1046520
tgccaacagg ccgcgctcag ggcgcaacgg acggtcgagg ttgtcgtatt gaggagaacc 1046580
gactgcacca agcaggacgg catcggcttt gcggcagagg ttttgcgtaa attcgggata 1046640
aggatgaccg tattcgtcat aggcttcgcc gcccaatggg gcgtattcgt agccggcatc 1046700
caaaccttgg gcgatgagtt tgtcgagtac gcggacggtt tcggcgacga tttcgggacc 1046760
gatgccgtca cctcggagga tggcgatatg tttggtcatt tcaagtttcc ttatgggttg 1046820
atggttgaag ggttatttct ttttgtattt gtgtgcaatt tcgtgccaac gaggtatgga 1046880
aatcgatcgg ttgtagtgtt ttttataggc ttcctcaaat ttcttttttcc ataaggatgc 1046940
gttatgccgt gttgccgggt tttgataaac ggtttcttca attgcggaaa caaaatcttc 1047000
caagcattca aacataggca tattcttttt atttatgctg taccaaacac ccgggcggat 1047060
tttatgcacc acgccttttt tgacttgaag attaattgcc cacccatcca tgcttctcgt 1047120
aatttgccat actttttttaa gccataaatc cttgatggtt acattgccgt tgtcgtccat 1047180
atcgtaaccg aatattaaat agtctacatc cagcatatag ggtttatgaa tgatttcatc 1047240
agaatacatt ttaaaatctg caatatcaaa acccggactg gcatttcggt tgaacgcctt 1047300
tacttccaac aattctctac tgcggtcttt tttatttaaa aagaaatcgg ggggcatttg 1047360
ggtattggtt gaaacatcaa attcaatttc ccttttttctc aaccatccgc cgagccattc 1047420
ctgaatgatg ttgccgacga catctttttg tttgacgata atatccacat cgcccaagaa 1047480
aaatctaatt tgaccattga ccgataagat tttttcctca ttcagcaact tatcaaatat 1047540

ttgttgtgca gtaagtttta ccattttatc cctatcggtt tataaagtat gcagaagcct 1047600
ttcagatacc gccttaatca caggaacggc aacggtattg cccaataaat cgtatttgtc 1047660
ttttttagga atatcaaacg aataatcgtc cggatagccg aataagcgta aaccttcttt 1047720
tccggtaagt gtgcgcaaac cgccgttgtc aacgacgaaa aggtgctcca tatccattgc 1047780
aactaaggtt ggcgcaacat catttgggtc taatatttta ttgatttcaa atgatttttt 1047840
tcctgaaaca atattgtagc ctttgggcag ggattcatct ttgattcttc gcccgccaat 1047900
tttttgtttc ggatgctcca aaaccaaata gcctttatct gtcaggctgt ccaaaatatt 1047960
ttgaagattg gggtgtttat agaaagttga aatttgcgct tttgtcaaag gcatcccatc 1048020
catccaatcg atgccgattt ctgaagccca tttttcttc ctccgttctt ttagaagcat 1048080
atttaataat tgcttctctt cttcggttac tgtgcctttt aattcaatat cccaactgtg 1048140
gatattgttt ttccctcccc gtttgtcctt tactgatttt ccgtacagtt cggacggggg 1048200
aaatttcttt agcaattttt tgatgaaagg actgctttcg gtaggcagtc ccgattccaa 1048260
aatattttt aatttcggac ttagggttgt ttcaaaagat aagtcgggtt tggatttcaa 1048320
actgcctgtc agataaatac gcttcctgtt ttggggaatg ccgaaatctt ttgcatttaa 1048380
aactttccaa gaaacatagt agcccaatgt ttccaaggtt tccaaaataa cggtcagggt 1048440
gcgtcctatt ttttgtgtcg gatcttttct atcgtgcgtc accaatcctt ccacatttc 1048500
caaaataaaa ccttttggtt tttttgcctt taaaatcctt gccacatcaa agaaaagcgt 1048560
tccccgcgta tcttcaaagc ccaatctttt tccggcgaaa gaaaaagcct ggcaagggaa 1048620
gcctgccaac aagatgtcaa aatcgggaat atctcccgtt tcaattttcg ttatatctcc 1048680
atacggcact tcatcagggt agttttgctt caatacttcc aaagctgccg gtttgatttc 1048740
tgaggtaaaa acacattcgc aagcaaccga ctgtttccga caggcttgtt caaatccttt 1048800
cctgataccg ctcatcccgg aaaataagtc aataaattta atttgttgca tattaaaaat 1048860
ctaaaaattt atttgaaatg gagagttgca ttattgcatt aatttagagt gtcgctaagc 1048920
ccgcttaaaa gatgaaagca atttatcgcc cctctgttta cattagccgc aacaattata 1048980
tgttatcagg aatgccgtct gaacggcctt cagacggtat aggttttaac cgttaaacag 1049040
ccaaggctgg ctttggcggc gtttttcttc aaaggcgtga atttcgtcgg cgtgttgcag 1049100
ggtcagaccg atttcgtcca agccgtttaa gaggcagtgt ttgcggtgtt cggtaatgtc 1049160
aaatgtgaac gtttcgccgc ttggtgtggt cagggtttgt tcggcaaggt cgatggagag 1049220
ctgatagcct tcgttggctt caacttcttt gaaaagtcgg tcaacccgtt cttcggtcaa 1049280
cacgataggt aaaaggccgt ttttgtagca gttgttaaag aagatgtcgg cgaagctggg 1049340
ggcgatgacg gcgcggaagc cgtagtcgtc caatgcccaa ggggcgtgtt cgcgtgaaga 1049400
gccgcaaccg aagtttttac gcgtcaacag gatttgcgcg ccttggtaac gcggctggtt 1049460
cagcgagaaa tcagggttca acgggcgttt gctgttgtcc atgcctggtt cgccgtggtc 1049520
gaggtaacgc cattcgtcaa aggcattggg gccgaagccg ctgcgtttga tggattttaa 1049580
aaattgtttg gggatgatgg cgtcggtatc gacgttgctg cggtcgagcg gggcgacgat 1049640
ggcggtaatt ttggtaaagg ctttcatggg tttgcgtctt gtgctgacga tgccgtctga 1049700
agcggtttca gacggcatcg cgaatcggtt attcggtggc gttttcgatt tttccgccga 1049760
gatgggaaat gccgcgtccg acggcattgc cgcctttttt gacggcttct ttggttttgt 1049820
cccagccttt ttcgacggcg ttgcctgttt gttcggcggc gcgttcggcg gcggcctgtg 1049880
ttttgtcaag gttgcgggcg gtgtcttgtt tcgcgccctc ccaagtgccg gcgcaggcgg 1049940
acaaggcgag ggcggacagg gcggtaatga aaagtttgtt catggttaaa ctccttggtt 1050000
tgaatattaa aggtgtttct gccttacggg acatatttca gacggccgcg tcaaattctt 1050060
aaagaccgcc tgaaaatact tacgccatca tgcggatgtc ggtaaagcgg ccggtaacgg 1050120
cggcggctgc tgccatagcg gggctgacga ggtgggtacg tccgccgttg ccttgacggc 1050180
cttcaaagtt acggttggag gtggaggcgc agcgttgccc cggggtcagg cggtcggcgt 1050240
tcatggcgag acacatcgaa cagcccggtt cgcgccattc aaaaccggct tcgatgaaaa 1050300
ttttgtccaa gccttctttt tcggcttgtt ctttaaccaa accggagccg gggacgatta 1050360
acacgcgctg tacgttggcg gctttttgc ggtctttggc gatggcggcg gcttcgcgca 1050420
agtcttcgat gcggctgttg gtgcaagagc cgatgaatac gatgtcgacg gggatttcgt 1050480
ttaatggcgt accggcttcc aagcccatgt attcaagggc gcgttccata ccgctgcgtt 1050540
tgaccggatc ggtttcttcg gcaggattcg gcactttgct gctgatgtct aaaaccattt 1050600
caggcgaggt accccaagtg acttgcggtt cgatgtcttc ggcgttgaaa cggtattctt 1050660
tgtcgaatac cgcaccttcg tcagacacca gcgtacgcca gtactcgacg gctttgtccc 1050720
acgcttcgcc ttcgggtgcg aagggtttat cttttacgta gtcgatggtg gtttggtcga 1050780
cggcaaccat gcctgagcgc gcgcctgcct caatcgccat attgcataaa gtcatgcggc 1050840
tttccataga aaggctgcgg atggcttcgc cgccaaactc gatggcgtag cctgtaccgc 1050900
ctgccgtgcc gatttgcccg atgatgtaga gcgccacgtc tttggcggta acgcccgctt 1050960
ttaatttgcc gtcaacggaa atcagcatgg atttggattt tttcgcggta atacattggg 1051020
tcgccatggt gtgctcgact tcggaagtgc cgatgccgtg cgccagtgcg ccgaatgcgc 1051080
cgtgggtgga agtgtgcgag tcgccgcaga cgacggtcat accgggcagg gtcgcgcctt 1051140
gttcggggcc cataacgtgt acgatgccct gacctttgtc cataaacgga aaataggcga 1051200

gtgcgccaaa ctctttaatg tttttgtcca aagtatcgac ttgcagcttg gaaatcgggt 1051260
cttggatgcc tttgtcccaa tcgccggtcg gggtgttgtg gtcggcggtg gagacgacgc 1051320
tgtcgatgcg ccacagcttg cgccccgcca ttttcaagcc ttcaaatgcc tgagggctgg 1051380
taacttcgtg caccaaatgg cggtcgatgt agagcaggac ggtgccgtct tcttcttcgc 1051440
ggacgacgtg gctgttccaa agtttgtcgt agagggtttg tgctgtcatg atgttgttct 1051500
tttggataaa tggtaatgcg gattgggcgg attttagacg tattctttat accgcgcaac 1051560
agattttgtc taatttttga gtcggtgtta ttttgtaaac aattttaaca aaaaaattag 1051620
acatattgtc catttcagta agcagttata tctaaagcat gattcgatac gaaagaatac 1051680
ttgtcgtcat tctttcaaag gcattatcat ctgcatcttg tcaaaaaaca cacagaggta 1051740
gacgaaagat gaaattaccg gtgatgtcgc ccgaacattc ggcgcaactt caggcgtttg 1051800
aggcaaaaat cctgtccaat cacgccaaaa tcgaggcgtg gttccgcacg cagtggagcg 1051860
tacaccgccc gccgttttac ggttcggtcg atatacgcaa tgccggttac aaaatttcgt 1051920
ctatcgatat gaatttgttc cccggcggct tcaataatct gaatcccaac tttatcccgc 1051980
tggcggcggt tgccgcgcaa gatgcggtgc aacgcgcctg cgaaacggcg aaatccgtat 1052040
tgattattcc tgaaaaccac acgcgcaata cgttttacct gcaaaacgtt tacgccctcg 1052100
gcgagatttt gcgttcggca gggtatgaag tgcgcttggg cagcctgaat ccggaagtaa 1052160
ccgaaccgac cgaatttgaa accgcattgg gcgacaaaat cctgttggaa cctttattgc 1052220
gtacccgcga tcgcgtccat cttgcagacg gcttttcgcc ttgcgtggtt ttgttgaaca 1052280
acgatttgtc cgccggcatt cccgacatcc tcaaaggcat cagccaaacc gttttgccgc 1052340
cgttgcacgg cggttggacg acgcgccgca aaacaaatca tttcggcgcg tacaaccaag 1052400
ttaccgccga atttgccaag ttaatcggca tcgacgaatg gcaaattaac ccttattttg 1052460
aaaaaatcgg cggtttggac ttccaagggc gtgaaggcga agacgcgttg gcggaagcgg 1052520
tagaacgtgt gctggcgaaa attcaagcca aatacgacga atcgggcatt accgacaaac 1052580
ctttcgtcat cgtcaaagcc gatgccggca cttacggcat gggcgtgatg agcgtcaaat 1052640
ccgccgacga agtgcgcgga ttgaaccgta aaaaccgcaa taaaatggcg aaagtcaaag 1052700
aaggcttgga agtcagcgaa gtgattgtcc aagaagggat ttatacttat gaaaccttaa 1052760
acggcgcggt gtgcgaaccc gtcgtgtata tgatggaccg cttcgtcatc ggcggctttt 1052820
tccgcgtaca cgaagggcgc ggtgcggacg aaaacctaaa cgccggcggt atggtgtttg 1052880
ttccgctgtc taacagcatt cctaccggta acggcgataa ttcccaagaa gcgcccgaag 1052940
cctgcaagcg cgtattcgaa caatgggact cgctgggtat gccgcgctct gaaaaagact 1053000
gcgacgtgga caacgaacac aaccgcctct acgtttacgg cgtaatggca cgcctgtcgc 1053060
ttctggcggc ttcaatcgag ttggaagaaa cggcgtaaga ctgttttgaa atacagatgc 1053120
cgtctgaagc ggaaatccgg ttcagacggc atttcggata tttggcgtgt gggaacatct 1053180
gtttcagacg gcatctcaga ctatttaaaa aagggaaaac atgagcatca agcaatggcc 1053240
ggaaggcgaa agacccaggg aaaagctgtt ggaacgcggg gcggcggcgt tgagtgatgc 1053300
cgaacttttg gcaatcctgc tgcgcgtcgg cacgcgcgga atgagtgcgg tcgatttggc 1053360
gcgttatttg ctgcaggagt tcggcagttt ggggaggctg atgagcgcgg aggtcggcaa 1053420
actgtcggca tacaaaggga tggggacggc aagtttcaca cagtttgccg tggtcaggga 1053480
aatcgggcgg cggatattgg cggaagaatt gcaggagagc atcgtcctgt ccgatccgga 1053540
tacggtggcc gattatttac gctttcattt ggggcaggaa aaagtcgaag tcagcgtcgc 1053600
gctgctgctg aaccgccaaa accaactgat tgcggtcaga gagctgtcgc gcggtacggt 1053660
tgcggaaaac acgatttaca tccgcgaaat cgtcaaactg gcattggacg aatatgccga 1053720
cagcctgatt attgcgcaca accatccggg cggctcgccc gaaccttcgc aggaagacat 1053780
catgttcaca aggcggctgg cacaggcaat gtcgctggtc gatgtgtcgc tgctcgacca 1053840
ttttatcgtt acctcgcaaa gcgtctgttc gttcagacag ctcgggttga tgccctgaca 1053900
ctctgtttta catgcggcgg ctctgataaa atagccgctt caaccgtatt caacagatat 1053960
tgttaagtta atggaaacac aaaacaaacc taccgttacc gacattgacc gccctatact 1054020
cgtcccgccc ggtggacata aaaaagtctt gctgcattcc tgctgcgccc cgtgcagcgg 1054080
cgaagtgatg gaagccatgc tggccagcgg catcgactac accatttatt tttacaatcc 1054140
caatatccat ccgcacaaag agtatatgct ccgaaaagag gaaaacgtgc gctttgcgga 1054200
aaagttcggc attcctttca tcgataaaga cgacgactac gaaaacgacc gcaaagaatg 1054260
gtttgccaaa gccaaaggca tggagtttga gccggaacgc ggcatccgct gcaccatgtg 1054320
tttcgatatg cgttttgaaa aggcggcgca atacgcgcat gaacacgggt tccccgtctt 1054380
taccagttcg ctgggcattt cacgctggaa aaatatggcg caaatcaacg actgcggaca 1054440
ccgcgccgcc gcgccttacg atgatgtggt gtattgggat ttcaactggc gcaaaggcgg 1054500
cggcagcgcg cgcatgattg aaatcagcaa acgtgaaaac ttctaccagc aggaatattg 1054560
cggttgtgcc tattccctga gggattccaa tgcccaccgc aaatcacagg gcagaatccc 1054620
catcaaactc ggcgtgctgt attacggcga cgaatcgaca caatacgaac ctgcccccat 1054680
ccgggtggac aaataaacac ccgatgccgt ctgaaggttc agacggcatc gggttcggca 1054740
tcggcacggg gaaaggtttg ccggtttggc aatctgcaat cggaaaccgc attggcaagt 1054800
ttgccgtttt gataaaacac cccgttgccg cgtcgggagg acggcattat gaaatccctt 1054860

tttattcggc tgctcctgtt gggttcggcg gcaggcgttt tctaccatac ccaaaaccaa 1054920
tccctgcccg cgggcgaact tgtctatccg tccgcaccgc aaatcaggga cggcggcgat 1054980
gcgctgcact acctcaaccg catccgagcc caaatcggtt tgcacaagct ggcacacgcg 1055040
ccggttttgg aaaactccgc ccgcaggcac gcaagctacc tcacgctcaa tcccgaagac 1055100
ggacacggcg aacaccatcc cgacaatccg cactacaccg cacaaaagct gaccgaacgc 1055160
acacgccttg ccgggtatct ctacaacggc gtgcatgaaa acatcagcac ggaagaagaa 1055220
gccgccgaat cgtccgacag cgacatccgc acgcagcaac gccaagtgga cggattaatg 1055280
agcgcaatct accaccgcct ttccctactt gaccgccata cggatgaggc aggagcggca 1055340
tttgtgcgcg aaaacggtaa aaccgttctc gtattcaatc agggcaacgg caggtttgag 1055400
cggcattgcg cccaaggcag aaatcagccg gaagcaggac ggaaatatta ccgcaacgcc 1055460
tgccataacg gtgcggtcgt gtacaccgac gaagccatgc ccgcacagga gctgctctat 1055520
acagcctatc ccgtcggcag cggcgcactg ccttatttcc acggcgagcg tccagacccc 1055580
gtgccggaat atgaaatcac gggcaatcct gccagcattg atttttccga ggcggcaggc 1055640
aaaattacga tgaaaagttt caagctgtat cagggtaaaa acgaaatccg ccccgtcagg 1055700
gttttaaccg ccggcaacga ccccaacggc aggctgaccg cgtaccaatt cgcgcttttt 1055760
ccgctcaagc ctttggaata cggcacgctt tatacggcgg tattcgacta tgtccgcaac 1055820
ggacggcgag cgcaggcgaa atggcagttt agaacccgaa aacccgatta cccttatttt 1055880
gaggtaaacg gcggcgagac acttgcggtt agaaaaggcg aaaaatattt catccactgg 1055940
cgcggacgct ggtgtttgga agcgtgtacc cgttatacct accggcagcg acccggcagc 1056000
cgcctgtcca taggaaggca cgaggcgggc ggcatcgtct tcagcgttga cggaatggcg 1056060
ggcagccgca tcacgcttgc accggaagga gaaacggaac gaggcgtaac cctttattta 1056120
caggattgaa tacatgacag gcagaacagg cggcaacggc agtacccaag cgcaacccga 1056180
acgcgtcatg ctggtgggcg taatgttgga caaagatggt acgggcagta gtgccgcccg 1056240
tctgaacggt tttcagacgg cattggcgga agctgtcgag ctggtcaaag cggcgggcgg 1056300
cgattccgtg cgcgtggaga ctgccaaacg cgaccgtccg cacaccgcgc tgtttgtcgg 1056360
cacgggcaag gcggcggagc tgtcagaagc agttgccgca gacggcatcg atttggtcgt 1056420
attcaaccac gaactcacgc ccacgcagga acgcaacctt gaaaaagaac tgaaatgccg 1056480
cgtattggac agggtagggc tgattctggc gattttcgct cgccgcgccc gcacgcagga 1056540
aggcaggctg caagtcgagt tggcgcaatt gagccatttg gcgggacgct tgatacgcgg 1056600
ttacggccat ctgcagagcc agcgcggcgg tatcggcatg aaaggccccg gcgaaaccaa 1056660
actggaaacc gaccgccgat tgatcgccca tcggatcaat gccttgaaaa aacagcttgc 1056720
caacctcaaa aaacagcgcg ccctgcgccg caagtcccgc gaatcgggca caatcaaaac 1056780
gtttgcgctg gtcggctata ccaatgtcgg caaatccagc ctgttcaacc ggctgaccaa 1056840
gtcgggcata tatgcgaaag accagctttt cgccacactc gacacgacgg cgcggcggct 1056900
gtacatcagt cccgaatgca gcattatcct gaccgatacc gtcggattcg tcagcgatct 1056960
gccgcacaaa ctgatttccg ccttttccgc cacgctggaa gaaaccgcgc aagccgatgt 1057020
gctgctgcac gtcgtcgatg ccgccgctcc gaacagcgga cagcagattg aagacgtgga 1057080
aaacgtactg caagaaatcc atgccggcga tattccgtgc atcaaggtgt acaacaaaac 1057140
cgacctgctg ccgtctgaag aacaaaacac gggcatatgg cgcgacgctg cgggaaaaat 1057200
tgccgccgtc cgcatttccg ttgctgaaaa taccggtata gacgcactgc gcgaagccat 1057260
tgccgagtct tgtgccgccg caccaaacac agacgaaacc gaaatgccat gaaaaaaacc 1057320
tgtttccact gcggtctgga tgttcccgaa cacctccacc tgactgtccg ttacgaaaac 1057380
gaagaccgcg aaacctgctg cgccggctgt caggcggtcg cacaaagcat tattgacgcg 1057440
ggcttgggca gttattacaa acaacgcacc gccgacgcgc aaaaaaccga gctgccgccc 1057500
caagaaatcc tcgaccaaat ccgcctgtac gacctgcccg aagtccagtc cgactttgtg 1057560
gaaacccacg gcggcacgcg cgaggcggtt ttaatgctcg gcggcatcac ctgcgccgcc 1057620
tgcgtctggc tgatcgaaca gcagcttttg cgtacagacg gcatcgtccg catcgacctc 1057680
aattacagca cgcaccgctg ccgcgtcgtc tgggacgacg gcaaaatccg cctttccgac 1057740
attctgttga aaatcaggca gataggctac accgccgcac cctatgacgc gcaaaaaatc 1057800
gaagccgcca accaaaaaga acgcaaacaa tacatcgtcc gcctcgccgt tgccgggctg 1057860
gggatgatgc agacgatgat gttcgcgctg ccgacctacc tttacggcgg cgacatcgaa 1057920
cccgatttcc tgcaaatcct ccattggggc ggctttttaa tggtgctgcc cgtcgtattc 1057980
tattgcgccg tcccgtttta tcaaggcgcg ctgcgcgact tgaaaaaccg ccgcgtcggc 1058040
atggatacgc cgattaccgt cgccatcatc atgaccttta tcgccggcgt ttacagcctt 1058100
gcgacaaatg cggggcaggg gatgtatttc gaatccatcg cgatgctgct gtttttcctg 1058160
ctgggcggac gctttatgga acacattgcc cgccgtaagg caggcgatgc cgccgagagg 1058220
ctggtgaagc tgattcctgc gttttgccat catatgcccg attaccccga tacgcaggaa 1058280
acctgcgagg cagctgtcgt caaattgaaa gcgggcgata tcgtgctggt caaaccgggc 1058340
gaaaccatcc ccgttgacgg cacggtgctg gaaggaagca gtgccgtcaa cgaatctatg 1058400
ctgaccggcg agagcctgcc cgtcgccaaa atgccgtctg aaaaagtaac cgccggcaca 1058460
ctcaacacgc aaagccccct gattatacgc accgaccgca ccggcggtgg cacgcgactg 1058520

tcgcacatcg tccgcctgct cgaccgcgcc ttagcgcaaa aaccgcgcac tgccgagttg 1058580
gcggaacaat acgcctcgtc tttcatattc ggcgaactcc tgcttgccgt ccccgtcttc 1058640
atcggctgga cgctgtacgc cgacgcgcac accgcattgt ggattaccgt cgccctgctg 1058700
gtcattacct gcccctgcgc cttatcgctt gccacgccga ccgcgctggc agcttctacc 1058760
ggtacgctgg cgcgcgaagg tattttaatc ggcggaaagc aggcaatcga aaccctcgcc 1058820
caaaccaccg acatcatctt cgacaaaacc ggcacgctga cccaaggcaa acccgccgtc 1058880
cgccgtatct cattgttgag aggcacagac gaagcctttg ttctcgcggt ggcgcaggct 1058940
ttagaacaac agtccgaaca tccccttgcc cgcgccatcc tcaactgccg catttcagac 1059000
ggcagcgtcc ccgacatcgc tattaaacaa cgcctcaacc gcatcggcga aggcgtgggc 1059060
gcgcaactga ccgtcaacgg cgaaacacag gtttgggcat tgggcagggc atcctatgtc 1059120
gccgaaattt caggtaaaga accgcaaaca gaaggcggcg gcagcgcggt ttacctcggc 1059180
agtcaaagcg gtttccaagc cgtgttctac ctgaccgacc ccttgaaaga cagcgcggcg 1059240
gaggcggtgc ggcagttggc aggcaaaaac ctgaccctgc acatcctcag cggcgaccgc 1059300
gaaaccgccg ttgccgaaac cgcacgcgcc ctgggtgtcg cgcactaccg cgcccaagcc 1059360
atgcccgagg acaaactgga atacgtcaaa gccttgcaaa aagaagggaa aaaagtgctg 1059420
atgataggcg acggcatcaa cgacgcgccc gttttggcgc aggcagacgt atccgccgcc 1059480
gcagcgggcg ggacggatat tgcgagggac ggcgcggaca ttgtgttatt gaacgaagat 1059540
ttgcgtaccg tcgcccacct gctcgatcag gcgcggcgca cccgccatat tatccggcaa 1059600
aacctgatat gggcgggcgc gtacaatatc attgccgtac cgcttgccgt tttgggctat 1059660
gtccaaccgt ggatagccgc actgggtatg agcttcagtt cgctggcggt tttgggcaac 1059720
gccctgcgcc ttcacaaacg ggggaaaatg cagtctgaaa aaatgccgtc cgaacaatga 1059780
cggacggcgt tgctttagac gtatagttga tgaaaacaaa aataagacga tgaagaattg 1059840
caaacttaaa gtatgtattg ttaccgctca aacacgttgg cgttcaaaat ttgagatcga 1059900
attgctaggt atttgatttt attaaataag agattgtatt aatgaggaaa aaaattaaaa 1059960
agtggattta tgcgataaat ggaataatga tgtcaatatg gattattttt tttccaaata 1060020
ccggttctgt gtatgacggt ggcagaacaa ccattttcaa tgaaaaccat ccttttcatt 1060080
ttattttctg cataacattt cttattggga caatttttct tatatatcat gaatataatg 1060140
ataactaatt tttaacatcc ttattgttat atcatgatga aatgacaata aggatggttt 1060200
tctgctttgg ctactgcaga acaccgtcgt cagtctcgcg tagggggggaa tccatatgct 1060260
tggtttttct tttattttca aatgctaatt aacggatagg tctggattcc cgcctgcgcg 1060320
tgaatgacgg aaatgtgcat ttctaatttt tacccactat atagtgaatt aaatttaaac 1060380
cggtacagtg ttggctcgcc ttgccgtact atttgtactg tctgcggttc gccgccttgt 1060440
cctgatttaa atttaattca ctataaaaac cccgaatcct gattggcagg attcggggtt 1060500
tttgattgct ggtgccgttc agacgggatt ttcaaacagc ttattgatct acaaacgcac 1060560
gctcaatcag gtaatcgccg cgtacgcctg ttttcggaga gacggtcagt ccgaaatcgt 1060620
ccaaaacttt gcaggtatct ttcagcatcg cggggctgcc gcacagcatg gcgcggtcgt 1060680
cttgcgggtt gattttgggc aggccgatgt cttcaaacag tttgccgctc accatcaggt 1060740
cggttaggcg accgtggtgt tcgaattctt cgcgcgaaac aatcgggtag taaatcagtt 1060800
tttctttaac caagtcaccg aggtattcgt gttcgggcaa ttctttggta aagcggtcgt 1060860
agtacgccaa atcttttttg tagcgcacgc cgtgtacgag gatgattttt tcaaattgct 1060920
cgtaaatttc ggggtctttg gtgatgctca agaaaggagc gatgccggta ccggtgctca 1060980
acaagtaaag gtgtttgccc ggattcaggt cgccggcaac cagggttccg gtcggttttt 1061040
tgctgattaa cacgtcgtcg ccgactttga ggtgttgcag gcggctggtc agcgggccgt 1061100
cttggacttt aatgctgaaa aattcgaggt gttcttccca gttggcggag gcgacgctgt 1061160
atgcacgcat cagcggcttg ccgtccacca tcaatccgac cataacgaac tgtccgtttt 1061220
caaagcgcaa cgattcgtcg cgggtgcagg taaaggtaaa atatgcgtct gtccagtggt 1061280
gtacggacaa tactttttgg gtattgaatg ctgccatttg ggtttcctgt cagtaaaaga 1061340
aatggatagt gcttgttcgg gaggtgcggc agagtggaaa tgtctgcccg attcgggata 1061400
aagccaaaat tctaaacgaa acggatggtt gcgacaatgc ttgatgcgcg ttggttatat 1061460
gccgtctgaa gggcttcaga cggcatcgcg gggcaggcgg cggcttacgg cggcatatcg 1061520
gcaagggaaa tcagggaaat cgaggctgcc agcctgagtg cgtcccgtcc ccagcggggg 1061580
tgttccagcc tgcgtatcgg gaggatcggt ttgacggtgg cggcaatcag cttttgtatt 1061640
tcggcaggct gatctgacaa tacgccgccc catttttccg cagccgcttc cagcagtccg 1061700
ccgtttttca atatcagcgc ggtcgaatgg atgtagcaga gccaatcgcg ggcttggcat 1061760
tgcgctatgg tcaggacttc ggaagggtcg tcttcaaaat ccaaaaagct gatgtttttt 1061820
ccgtccgaca tcatatttcg cgcaaacgcc tgactgagga actgccgttt tttatgcacg 1061880
cgtgcaatgg cttccaaacc ggcaagccaa gcgtccgact ttccagcctc ggcttcttgg 1061940
cggatttgcg catcgagcgg gatgccttcc aaattgccga acataagggc atttttcctg 1062000
acggcgagca attcgggaac ggctatcccc gccgagcgca attcgtacag gcgttttgat 1062060
tcggttgcaa tggcaggctc gccgccgagg ctgggaaccg gcttcaacac ccccagtttc 1062120
aaatatcggg caaccatacc gagcagcgcg taacgccatc gtgcattgtg cctgcctgct 1062180

ttgcgtatcc ataccttggt tccgtcggca agcaggtggg gggcgatggc tgcctcctgt 1062240
tttgccgcca attcgtctag cagtatggaa aaacgggttt cctgcatagg taaggtcatt 1062300
ttctttcaat cttaagttcg gacggaatgc ccctgtacgg aatatcaggc aagggtttgt 1062360
tcgatgatgc cttttaccgc atccgcactg ttcggcacgg tttgcacgcg ctgcggcagg 1062420
ttttccaaac cttccagcgc ggcagggcgc ggaatggcgg catcgccgac ggcttcgcgt 1062480
atggtcgcat cgaatttcgc cgccaacgcg gtttccaaac aaaccaccat ttcaccttct 1062540
tcgcgcactt cgcgggcgac ttttacgccg tcggcagtgt gcgggtcgat gagttcttgg 1062600
tcttgctcgt aaacctgctt gatggtggcg aggcggtcgg cgtgggtgga tttgccagag 1062660
gtaaaaccgt atttgccacc gactttgtcc aaggcaaatc gcaggtcaaa gcctttgcct 1062720
gcagccactt ccgcccacag cgtattgatt tccgcaggat cgcgatccat caggtcgaac 1062780
acgaaacgct cgaagttgga cgctttggaa atgtccatag acgggctgga ggttacataa 1062840
gtatgcgcgc tgttgcgcgg gcggtatgca ccggttttga aaaactcgtc caacacatcg 1062900
ttttcattgg tcgcgacaat caggcggcgg ataggcaggc ccatttgttt ggcaatgtgt 1062960
cccgcgcaaa cattgccgaa gttgccgctc ggtacgcaga agctgacggt ttcgtcattg 1063020
cttgaagtgg cgttgaaata gcctgcaaag taataaacca cttgcgcgac gatgcgtccc 1063080
cagttgatcg agttgaccgt accgatatgg tatttttcct tgaacgcggc atcgttctgc 1063140
accgccttca caatatcctg acagtcgtca aacattccct tcacggcgat attgtggata 1063200
ttctcgtctt gcaggctgta catttgcgcg cgttggaacg cgctcatttt accgtcgggc 1063260
gacaacataa atacgttcac gcccttttttg ccgcgcaagg catattccgc agccgaaccc 1063320
gtatcgccgc tggtcgcgcc caagatattg agttttttgc cttctttgtt taaaacatat 1063380
tcaaacgcat tgcccaaaaa ctgcattgcc atatctttga acgccagcgt cgggccgttg 1063440
gacaaggctt ggattttgat gccgtctgaa agcgtgcgga cgggggtgat ttccttagta 1063500
ccgaacgccg cttccgtgta agtacggttc agaatgtcgc gcaaatcgtc ctccggaata 1063560
tccgtaacaa acaggcgcat aatttcaaac gccaattcgg gataagctaa accgcgccat 1063620
ttgtccaagg tttcgcgccc gatttgcgga taatgttccg gcagcatcag gccgccgtcg 1063680
ggggcaagcc ccatcaataa aacttcgctg aacggtttgt gtgcggtttc gccgcgcgtg 1063740
ctgatgtatt tcatgatttt tctcgtctgt cgaaattgca ggaaaacggc ttcagacggc 1063800
atctgcctca tgccgtctga agaaggttag cggtacaggt gtttgaagca ggcggaaacc 1063860
gttttggcgg tcagggcggc aagtgcctga ttgcgcgtgg acggagccag catctgcatc 1063920
acatcgttgc cggtcatccg ttcgggtgct tcttgggcga cgcaagcgca aatcttgttt 1063980
tcccactccg cctgtttttc ggcactcatc gccagcgcgg tcaaacgcca ttcgctgcgt 1064040
ttgtccaatt ccgcacggca ttggctccca accgccattt tgacgatgct gccgcccatg 1064100
cctgtgccac cgtctaagct gccgaatgtg ttaccgcctc cggcggcgca gccgccgagt 1064160
aagattgcca ccggcaaaat agacaaggtt ttattcatct caattccttt tcggttgaaa 1064220
ccccgccttt tatggcgata gaatctgatt agccgccccg ttcgggataa cgcgaagggc 1064280
ggcgttttat gcgccgttcc gagtgttgga acaaaccgtt ttgaatatcc ggttgaagcc 1064340
cggcaacatt atacttcaat cgggaaaata aaaaatcccg ccgccgtcat tttgcctgtt 1064400
tgcaaaaatg ccgtctgaaa gcggttcaga cggcatttcc gatttcagcc tagcccaaag 1064460
atttgaagtg ttccaaaaac ggcgggatac cgggcagcat cccgaccgca cccatcgcca 1064520
cacacaagat caagaaacct actgcgggta tcaacacgcg tccggcgaaa cctaattgcg 1064580
cactgcgctc tttgcagccg attaagccca aattatccaa cagcatcgtc aacgcccagc 1064640
cgaaaaccgg attgaccaag gcggaagaga acaccacgat ggcggcggat tgggtggttt 1064700
tgcctttgcg cgtcatttcc atgcccgctt ccaaaagcgg taagtatacg cctacgacca 1064760
aggctacgct caataccggc tgccaaatcg ccaagtccat cggatagccc cataacccgg 1064820
cgataataca taaaaccgcc gttaaaaccg caccgcccgg aatggggcgt ttggcaatcg 1064880
atgccggtac gatataagtt ccccaagaag aggtaaaatt tgcacccccct aaaatagaac 1064940
ccactgcttg acggacagaa caacttgtca tggtgtcgtc tatattcatc aataccttat 1065000
cggttttttc cggatagctg attttttgga acacttgatg tcctaaaaaa tcgggcgacc 1065060
acattgcaac agccaatacc gcaaatggaa agacaaccaa aaaactttct gccgtcggca 1065120
accccagatg ccagccgctg ttttcacccc accaataagc aggactcatt ggaggcaggc 1065180
cgggggcggt gtgaaactca aacggcgcac ccaatgcaaa tgccaccaca ccggcaatca 1065240
agcatcccaa aggcacggct aaccagcgtt ttttccaatg ctccaacaaa gcgtacatca 1065300
caatcgttac aataatgacg gtaaaagcga tgtagggcat attaaaaccg cctgcccacg 1065360
aaaacaattt ttttacctgc cccgtcgtgc cgataaagcc caaatagagt aataatccgc 1065420
cgcatacgcc gttgcttgtc agcttcgcca taatactgcc gccgcgaaat aaagccatca 1065480
gcagacctaa aaccgcaatc gaaatgccga acgccaaagg atgcccgcct gccgacacaa 1065540
cgatgggaat catcggaatc agcggcccgt gcgtaccggg caggttggcg ccgggcagaa 1065600
aaaagcccga taccaataag ataaacgcgg cggcgattaa aagctcatag cgcacatttt 1065660
ccagtacaaa gctgtcaggc agccccaaag gtgcggcaaa cgccgccgcc accgccccca 1065720
ccatcaccac ttttccaatc gttcccgcca tcgcaggaat caaatcctcc cactcgaagc 1065780
ggtaatcgcg aaagggcagg ttgggccgcc agcgttttgg ttgcataatc tgcaattcat 1065840

gttccaaata ttcgtcccgc gtcgcaaatt ccgaagctgg acggtgcaaa tcccgataag 1065900
tcccattatg tttttccata accttcctcc ttatatatcg cgcctcgtaa aaggggcgca 1065960
tgacttttct ttttgatacg ggctgcgttc ggaagccgta accccattta aagcccaaac 1066020
aggcaataaa accaatcttt ttttttgata accatcatcc ggaaaactga tacaatttac 1066080
aaaccacttg attaaaaagt taattttcag caacaatcca cctaaaagat ttcgattgca 1066140
caaatataga aaacatccgc acaaggaggg atatatggat gccgtacaat taaaatcatt 1066200
tgtcgccgtc gcgcacgagg gcaaccttac ccaagccgcc aaacgacttt tcctttccca 1066260
gcctgccgtt tctgcccaaa ttaaagccct tgaagaatat gtcggcacgc cgctgttcag 1066320
gcgcacgggg aaaggcatgg tattgacgcg ggcgggcgaa atactgttgc ccgaagcgga 1066380
atccctgctg caatacaaac acaagctgga gcattttgcc aaaacgctgg caggcgatta 1066440
ttcggaagag accagtttgg gcattatcca ccccatcgat tcggcaaaac tcgtcgcgct 1066500
gacggacaat atcggtcaaa cagcccccaa aacgcgcctg cacatccaat acggaatgag 1066560
cggcgaaatc ctctcgcgca tccaacacaa aaccctgcac ggcggcttta tactcggcaa 1066620
cgccgcccaa cgcggcatcc gcagcgtatt cctgcaaaac ctgacctacg cgctgatttg 1066680
cccgcaaagc caatatcccc atctgacccg ctcccttccg cagagcctgc aagaatgcgt 1066740
atggatagaa atgtcgggcg tgtccggaag taggaagcac ctgcaccagt tttggcgcag 1066800
caaccggctc tcacccaaaa aacagatctt gtgcgactac ccccaaacca ttatcgattt 1066860
ggttgcaggc ggtataggtg tggcaatggt gccgggaaac aaagccgaag cggcggcaaa 1066920
agaaggcgcg ggcgtggcta ttatcgaatc gtgccgccac agtatgccgc tcaatttcat 1066980
ttatgcggaa gaatacgagg ataatcccca cgtctcactc ctgctcgagt gcattgaaaa 1067040
agtatgggga gtgcaggcgg tgcagccgcc cgttgtctcg gacaactgaa ataaatcctg 1067100
ctttgctgat tgttttaaaa tagaaatttg aattttatca cgctgaaaac actgaaaacg 1067160
ccatccgcat tctctcaaat acggcttaaa atgccctttg gaaatgccgt tatagtggat 1067220
taacaaaaat caggacaagg cgacgaagcc gcagacagta caaatagtac ggaaccgatt 1067280
cacttggtgc ttcagcacct tagagaatcg ttctctttga gctaaggcga ggcaacgccg 1067340
tactggtttt tgttaatcca ctataaactg acgcaaatac cgttttgcac aattccaaaa 1067400
gttttcaatt ccgttaatgc gattttgccg tttggcgaaa tgcgtactgt tccagtcgtg 1067460
gattgaaccc ccaccctgta tagttctttc gaagcattgg ggtattgttt tttcaaagca 1067520
tcttggattc ggatttcaag tgcaacacta gtgtattagt ggttggaaca gattcaagaa 1067580
taaaacactt ggcgtttcgt agccaagtgt ttttcttggt cggtggttca actcatcttg 1067640
aaccctgcgt atctcccgat cactgatgtt acggaaatcg gtttgtttgg ggaagtattg 1067700
ccggatgagt ccgttggtgt tctcattcag ccctttctcc caagaatggt aagggcgaca 1067760
aaaataagtc tccgctttca atgctttggt tattttggtg tgttggtaga actctttgcc 1067820
gttatccatg gtaatggtgt gcaccctgtc tttatgtgcc tttaatgccc taacagctgc 1067880
ccgggcagtg tcttcggctt tgaggctatc caatttgcag atgatggtgt agcgggtaac 1067940
gcgttcgacc aaggtcaata atgcgctttt ctgtcctttg ccgacaatgg tgtcggcttc 1068000
ccaatcgccg atacgggatt tctggtcgac gatagcgggt cggttttcta tgccgacacg 1068060
gttgggtact ttgcctctgg tccatgtgct gccgtagcgt ttgcggtagg gtttgctgca 1068120
tattctgaga tgttgccaca acgtgctgcc gttgcttttg tcttggcgaa ggtagcggta 1068180
aatggtgctg tggtggagcg tgatctggtg gtgtttgcac aggtaggcgc atacttgttc 1068240
gggactgagt ttgcggcgga taagggtgtc gatgtgctga atcagctgcg aatcgagctt 1068300
atagggttgt cgcttacgct gtttgatagt ctggctttgc cgctgggctt tttcggcgct 1068360
gtattgctgc ccttgggtgc ggtgccgtct gatttcgcgg ctgatggtgc ttttgtggcg 1068420
gttcagctgt ttggcgattt cggtaacggt gcagtggcgg gacaggtatt ggatgtggta 1068480
tcgttcgcct tgggtcagtt gcgtgtagct catggcaatc tttcttgcag gaaaggccgt 1068540
atgctaccgc atactggcct ttttctgtta gggaaagttg cacttcaaat gcgaatccgc 1068600
cgtcgtttga acattttttt cttcctgttt gatttcagac ggcattgccg ttccgtttgg 1068660
tttccagcag ctcccagcgt tccagctttt ccaaaagcag catttcgatt tcttcggcgc 1068720
ggttttgcaa tgcacctgct ttttcgtaat ctttgaaaat ttcaggatag gaaagttggg 1068780
tattgatttc agcctgctcg gcttccaaag cggcgatttc gtcgggcagg gcatcgagtt 1068840
cgcgctgctc tttgtaggac agtttgaccg tgcggttggc tttgggtttt tctttggcgg 1068900
gttcggcatc ggatgctttg ggtgcggatg ccgtctgaat tttatcttcc cgcgattttg 1068960
cgtcgatata gtcctgatag ccgccgatgt attctttcag acggccttgt ccttcgaaaa 1069020
caatgctttg ggtaattacg ttatcaagga acatacggtc gtgcgagaca aggaatactg 1069080
tgccttgata atcgcgcaac aggtcttcga gcagctcttg ggtgtcgatg tctaagtcgt 1069140
tggtcggttc gtccaagacc aggatattgg caggacgggt aaagagtttt gccagcaaaa 1069200
ggcggttgcg ttctccgccg gagagcgatg aaacagggct ttgcgcacgg gctggatgga 1069260
acaggaaatc ttccaaatag ctcatgacgt gttttttctt accgccgact tcaacgtaat 1069320
cgtttccctg tccgagggtg taaaacacgg tgtcgttttc attcaacgcg ctgcggaact 1069380
ggtcgaaata ggcgacttcc tgcttactgc cgatacggat tctgccgtag gtcggctgca 1069440
attcgcccaa aatcagctta aggaaggtgg ttttgccgat gccgttgggg ccgattaggc 1069500

cgattttgtc gccgcgctgc aagatagcgg agaatttgtc cataatgact ttgccgccat 1069560
aggcaaacga agcgtgttcc aattcggcga tgattttgcc acttttctca ccgctatcga 1069620
gcttgaagtt gacttgtccc tgtacgttgc ggcgttctgc acgctggcgg cgcagctctt 1069680
ccaaacggcg cacgcggcct tcgttgcggg tacggcgcgc ttcgatgcct ttgcgtatcc 1069740
atgcttcttc ctgtgcgtgg aatttgtcaa agaggcggtt gtgttccgct tcgactgcca 1069800
actcttgcgc ttttttctcg ctgtatttag agaacgagcc gggataggaa cgcaaaatac 1069860
cgcgatcgag ttcgacaatc cgcgtggcga tattgtccaa aaaacggcgg tcgtgggtaa 1069920
tcacaaccaa gctgccttca aacgctttga gcagattttc cagccaaata atcgcgtcga 1069980
tatccaaatg gttggtcggc tcgtccagca gcaatacgtc gggcttttgc acccaagcct 1070040
gagccaaggc gacgcgcttt ttctgaccgc cggaaaggtt gccgattttt tcattttccg 1070100
gcaaaccgag ttcccccaaa gtctgcttga ctgccgcatc cagtttccag ccgtccttcg 1070160
cttcgatttc aagttgcaat tcgttgagtt ctttcaacaa agcctcactc gaaccatttt 1070220
ccaactcatg gctgacatga tgataacggc gcaataaatc acgaatttcg cccaaacctt 1070280
cggcaacggt atcaaatacg gttgcgtcct tatcaaaaaa ggattcctgc ggtacataaa 1070340
cgattttgag gttgttttga acaataatct gcccgtcgtc gagcttttgc aaaccggcga 1070400
ggatttttaa aaacgaagac ttgcctgcgc cgttgcgtcc gattaagccg actttttcgc 1070460
cgctgtcgag ttgaaaagaa gttttgtcga gcaaggcaac gtggccgatg gcaaaagaag 1070520
cgttttctac agataatata ttcatgatac aaattctcaa cagttaccgt ttggatttta 1070580
ccgcaagttt ggcgcgggca atttcaaccg cacccggcag gacggaaaca ataatgatgc 1070640
cgcccatcac caagcccaga ttgtttttta cgacggggaa gttggcaaag aaatagcccg 1070700
cgtaagaaaa caggataacc cacaacaagc caccgatgat gttgtagcgg ataaatttgg 1070760
catagtgcat tttccccata ccggcgacga agggggcgaa ggtgcggacg atgggcataa 1070820
aacgggcaat gatgatggtt ttgccgccgt gtttttcgta aaaacggtgg gttttatcga 1070880
gatattcacg tcggaagatt ttagaatcgg ggttggcgaa cagcctgccg ccgaaatatt 1070940
tgccgacggt aaaattgagc gcgtcgccga gtatggcggc aaggcttaat aatgcaacca 1071000
tcaaatgaat atccataccg cccagcgcgg caatcccgcc ggcggcaaac agcagcgaat 1071060
cgccgggcag taagggcgta acaatcaggc cggtttcgca aaaaacaatc aaaaacagaa 1071120
tcgcataaat ccacacaccg tattgcgccg acagcgcgag caggtgttgg tcgatatgga 1071180
ggatgaagtc gatggcggag gcaagcacgg cgcgttccta aaaaaacaaa ccgcgtattt 1071240
taaccgattg gaaaaatgcc gtctgaaaag tttcagacgg catcggctat tcaaattcat 1071300
ttcacgtaaa aaccgcaaac caaaatagtt tgcggtttgg catttaaagt gacaatgatg 1071360
atttcaaatc atcagaattt tatgccgacg cgcaagccgt attcacgaat actggttttc 1071420
gggatggtga gcgatacgtc gccactcttg gttgttacac taaactcgcc ggattctttg 1071480
taagtgcgtt gtttgtagaa cggccccgcc tcgatgctgg cggattcgcc cagtttttta 1071540
ccaatatttg cacccaatcc aaagccccag ccgttggttt tttgattgat gtcttttttg 1071600
agattggtaa cgccggtgtt taatttatag cgggaattga ggtcaaattt cacttcagac 1071660
caagggttga tataccagcc gttacccagt tgggaaagca aatccgcgtg aactttggct 1071720
aaccacgact gacggctgct gtgaagcgta tgcttggtgg ttttaatgct gtcttttgaa 1071780
gattcaaaac ccaagccggc acccacacgg aaatttaaag aatcacttaa cgtttgggtg 1071840
taggtgtagc ctgtgtaaag atcgatacgg ttttcaggaa cgccggtggg cagttttaca 1071900
tttttagtct tacccagctt gttctcatct gtttccaaat taataatatt ttttttgctg 1071960
cgcccgaaac cggcttccaa gcggatgcct tggttggcat caaaaggaat atcagcacgc 1072020
acgctgatgt gtttggcagc tttgtgtttt tctttcagga aagcacgagt tgaagaaatg 1072080
gaagagaggt cggtgtggac ggtaaactca ttagcggttt gaagctcttg tgcagcggcg 1072140
gcagtaccgg tcagggcaat catggcacat gtaaaaactg tttttttcat agttaaaacc 1072200
tctaaaattt ggattgtagt cggatatggt aacataacgt aaataatcgt tacgcttaca 1072260
attatattct taagctttcg gggggggggg gattttacat atattaataa aaattaacaa 1072320
atagttattt gtttacaacg aattgttatt ctcacttggt tttctgtttt ttatgggaat 1072380
gacgaaattt tagtttgtgt gtatttatcg gaaaaacaga aacccgccgc cgtcattccc 1072440
gcgcaggcgg gaatctagaa cccaacgcga caaaaattta tccgaagcga caacaatctt 1072500
ttcatcgtca ttcccgcgca ggcgggaatc tagaacgtaa aatctaaaga aaccgtttta 1072560
cccgataagt ttccgtgccg acaaacctag attcccgcct gcgcgggaat gacgggattt 1072620
taggtttctg atttcggttt tctgttttaa gggaatgacg agacttgaga tggcggcatt 1072680
tatcgggagc aactgaaacc accctgccgt cattcccgcg aaagcgggaa tctaggttcg 1072740
tccggtttcg gttatttccg atagattcct gccgcgttgg gggtctggat tcccgcctgc 1072800
gcgggaatga cgggacttta ggtttctgtt tttgtttgag acctttgcaa aattcctttc 1072860
cctcccgaca gccgaaaccc aaacacaggt tttcggctgt tttcgcccca aataccgcct 1072920
aattttaccc aaataccccc ttaatcctcc ccggataccc gataatcagg catccgggct 1072980
gccttttagg cggcagcggg cgcacttaac ctgttggccg ctttcaacag gttcaaacac 1073040
atcgccttca ggtggctttg cgcactcact ttaatcagtc cgaaataggc tgcccgcgca 1073100
tagcggaatt tacggtgcag cgtaccgaag ctctgttcga ccacatatag tggattaaat 1073160

ttaaaccagt acggcgttgc ctcgccttgc cgtactattt gtactgtctg cggcttcgtc 1073220
gccttgtcct gatttaaatt taatccacta taacgggtct tcgataaata tcggttacgt 1073280
ttggtttgcg tttccgtcag cggacggttg cggcaggctt tgcgcataat gccgtccaac 1073340
aactgatgtt cttccagatg ttgccggttt tccgcactgt catagccttt gtcggcatag 1073400
acggtcgtac ctttgggcag tccttccaac aaaggcggca ggtgtttgca ctcatgggca 1073460
ttggcggggg taatgtgcag tttctcgata tagccttccg catcggtacg ggtatgttgt 1073520
ttgtaaccga gtttgtagag gccgtttttc ttgatccaac gggcatcgct gtccttactc 1073580
ggtgtggttt ggccgttgat ttgtccttct tcatcgactt ctatgacctg acgctgtgtg 1073640
ctgccggcgg tctgaataat ggtggcatca atgacggcgg cggatgcttt ctctactttt 1073700
aagccttttt cggtcagttg gcggttgatc agttccaata attcggacag ggtgttgtct 1073760
tgcgccaacc agttgcggta gcggcataag gtgctgtaat cgggaatgct cagttcgtca 1073820
aaacggcaaa acaggttgaa gtcgatgcgg gtgatgaggc tgtgttcgag ttcgggatcg 1073880
gagaggctgt gccattgtcc gagcaggacg gctttgaaca tggacaacag gggataggcg 1073940
ggacggccgc ggtaatctct gaggtaacgg gtttttgacg gttcaggtat ggttcgatcg 1074000
gctgccaatc aatcacccgg tccaacttca atagcgggaa acggtcgatg tgtttggcaa 1074060
ttatggcttg tgcggtttgc cggaagaagg tgctcatgag aaatccccta aatgtcttgg 1074120
tgggaattta ggggattttg gggatttttg caaaggtttc cgcctgaaac attatgagat 1074180
ttcaggcggc attggattgc ttggcggaat atttttaaaa aggcttacgc gccgtaaacg 1074240
gggtatttat tgcacaaagc agttacttgt ttgcggactt tggcgaggtt ggcttcgtct 1074300
tcggggttag acaatacgtc ggcaaccaag ttcgccaata cgcgcgcgtc ggcttcgtta 1074360
aaaccgcgtg tggtcatggc agcggagccg atgcggatgc cggaggtaac gaagggtttt 1074420
tccggatcgt tcggaatggc gtttttgttg acggtgatgt gcgctttgcc caaagcggct 1074480
tcggcggctt tgccggtaat tttcatcggt tgcaggtcaa cgaggaaaac gtggctttcg 1074540
gtgcggccgg aaacgatgcg caaaccgcgt ttaaccaact cttccgccat ggcggctgca 1074600
ttgattttca cttgttttgc gtattgtttg aactcgggtt gcaatgcttc tttaaacgcc 1074660
acggctttgg cggcgataac gtgcatcagc ggaccgcctt gcaggcttgg gaagatggaa 1074720
gagttcaacg ctttttcgtg ggtattgtcg cggcacaaaa ttacgccgcc gcgaggaccg 1074780
cgcagggttt tgtgggtggt ggtggtcacg aagtcgcaga acggcaccgg gttgggatat 1074840
tcgccgccgg caaccagacc ggcatagtgc gccatatcga caaagaggta tgcgccgact 1074900
ttatcggcga tttcgcggaa ttttgcccag tcgatttgta acgcgtaggc agacgcaccc 1074960
gccacaatca ttttgggttt gtgttcgagc gcgaggcgtt cgacttcggc ataatcgagc 1075020
acttcgtttt catccaaacc ataagtaacg gcgttgtaga gtttgcctga gatattaacg 1075080
ctcgcgccgt gggtcaggtg gccgccgtgc gctagagaca tacccaaaat ggtgtcgcct 1075140
ggttttaaaa cggaagcgta cacggcttgg ttggcttgcg agccggagtg cggttggacg 1075200
ttggcatagg ctgcgccaaa cagttctttt acgcggtcaa tcgccaattg ttcgacaata 1075260
tcgacgtatt cgcagccgcc gtagtagcgt ttgccggggt agccttcggc gtatttgttg 1075320
gtcagctggg aaccttgcgc gtccattacg gcgcagctga cgtagttttc ggaagcaatc 1075380
agctcgacgt ggtcttgctg gcgttggtct tcttgggcaa tggctgctgc caaatcgggg 1075440
tcgtattgtg cgagggtaac gctttttgaa aacatgttct cggctccttt gtgtaatcag 1075500
ggtatcatga gtgttttttg tataaaaaaa tatttcaaaa cctaaggcag atagcccata 1075560
atgcgtaaat tttctttggc attatcaggt aatttattta acatgctggt ttttagcgtc 1075620
tcattacctt tatttagtac aagactaatc aaaagcaata cattaaatgg taaattttcg 1075680
gcagtttgtg caaaatcaat caaataacca gcaccaacca catctttatc agccataacc 1075740
cgctgataca ccacttgcta agacaactct cctgccccac catgtcttaa aatcagatga 1075800
atggcttgta atgctgtgtt tggtgtctca caaattgcca agatctgctc agaagcttgc 1075860
ttgatggctt gtatttttgt ttgattactc acaatttccc cctattttaa taattaactt 1075920
aaatgcggtc aaattcacaa aatacaagct ttacctctaa tcacccatca ctcgaccttc 1075980
tcggcgtgga tcggcaccac caaccagcct gcttggctcg ataataatgg cttgaacacc 1076040
tgaatttagc tcacgcacat cagtcttata gcccaaatca tttaatgctt gttgccactg 1076100
gacggcggtt gtacccgttt ctagttcata gctaccaaag cgatttaata aattgggtgc 1076160
actgatggca ttttggatat ccatattcca gtcactatgt gccacaatcg tcttagcgac 1076220
atagccaatg atacggctac cacctgggga gccgattgcc atataaggct tgcctgcttt 1076280
aaatacgatg gttggtgcca ttgaggagcg tggtctcttg ccgggctcga cacgattggc 1076340
gacctgtttg ccctgcttta ttggctcaaa actaaagtct gtcagctcat tattcagcag 1076400
gtagccattt gccatcaaag ttgagccaaa cgcattttca atggaagtcg tcattgatag 1076460
cacattgccc gccttatcca caattgatat atgactggta gaaggtaact caatcgcttg 1076520
tgaggacacc cactcatgaa taaaatcgcc tgcagatacg ctaggcaatg ccttatccga 1076580
ctgctcaagc agctggctgc gatgttttag gtagtcttta gaaatcaact ggcgaatggg 1076640
tactggtaca aaatcagggt cgcccaaata tacatcacga tccgcaaacg caagcctaga 1076700
agcgtcgccc aagagacgta aaccttcagc atcatacccc acctgattgg gtgaaaattc 1076760
atttaaaatc cccaaaatct gacccacagc aatcccacct gagcttggtg cacccatacc 1076820

gcatacttca taaatacgat aagtcacaca aacaggcggg cgttccacca cttgataatc 1076880
agataaatct tgtaaggata attgaccggg gttatcctta gcattttgga caactgaaac 1076940
gatattttgg gcatatttac cagtatgcag agcttttgca ccttgagctg ctaacgcctg 1077000
aacactgtca gcaaattcta aatttttcag caagctgcct gcttgtagcg gcacaccatt 1077060
cggcaaaaaa taagcggctg tttttggata gcgtgccaaa tgctgctgat tttgctcaac 1077120
cgagatggca agccttggcg acacctcaaa gccttgtttt gccaagcgga tcggtgtatc 1077180
aaataatttt ccccaaggca atacaccgta tcgctgatgt attgtctcca tcagtttagg 1077240
gatagcaggc gtacccaccg agcgaccacc gaccaccgct tccataaatt tcaatggttg 1077300
accatcttta tccaaaaata attccggcgt cgcacgcatc ggtgccgtct cacgcccatc 1077360
aaatgtggtc aatgttttgg cggtattatc ccaatacaac acaaatgcac caccgcccaa 1077420
gcctgacgac tgtggctcta ccaagcttag tgtcgtctgc accgccacca tcgcatctgc 1077480
agcgctaccg ccttgcttta agatatcata gccagcttgt gttgctaatg gattggctga 1077540
cgctaccata aaatcacttg caatcacctg cttttgttcg gtcagtcccg ttgcatgttc 1077600
aggcgtgtga gcgtctgcac ctgtgatgac agcagaatga gtattaacct taccttgatt 1077660
ggcatggatg acttgacatc cagagattgt catagacatt atcaatgcag tcaataaata 1077720
tgttttagcc acaagcactc cttcgcctga gtttgattga tgattcatac aaggcatgct 1077780
gattattgta tttaatatgg ctaaataatt caatccaaac tatcaatctt gaccatcaaa 1077840
aaaagaccgc taatgtcatc agcagtcttt tttgatattt attttaagat attaagtaat 1077900
cagacctttg ggctatgctc ttcaatgagt ggttttagct cacctgattg gtacatttgt 1077960
aggataatat cactaccacc gattaactca ccattaaccc aaagctgtgg aaaggttggc 1078020
cgactggcga tgagtggtag agtactgcga atttctgggt tttctaggat attaacaaaa 1078080
gcaaagggtc tgccaattgg gtcagcacct ctactgcacg cgctgaaaat ccacattggg 1078140
gaaactgggg cgtgcctttc atatatagtg gattaacaaa aaccagtacg gcgttgcctc 1078200
gccttagctc aaagagaacg attctctaag gtgctgaagc accaagtgaa tcggttccgt 1078260
actatttgta ctgtctgcgg cttcgtcgcc ttgtcctgat ttttgttaat ccactataca 1078320
gtaggaaagg ctgaaaattt atgcgtaaag cgtgatattg tcaacgtttt tatcaaccgg 1078380
acggcggtgt taaaagaaaa ttttgccgta tccgataaaa cactggataa aaatattatc 1078440
tttgttataa ttaatgtaaa gattcaattt gactttttaa ccgtaaacca agagaggaaa 1078500
gcgatatgtt cccagaatac cgtgatttga tttccaaatt gaaacaggaa aattcccgct 1078560
tcgcccgtct gttcgacgaa cacaacgagc tggacgataa aattaccggt ctggtcaaca 1078620
atccggttac cagcggtgcg gaaaccatcg atgagctgaa aaaagccaaa ttgaaactga 1078680
aagacgagtt gtacgccatc ctgcaaaaag cagcgggaaa ataattcggg tttgagtttt 1078740
tgaaatgccg tctgaaatgt gttcagacgg catttttgtc atttgaccgg aaggcttgtg 1078800
ctgtttgaaa taacggcggc ggtatcggat tgccgccgcc gtgtacttgt gtgaacggct 1078860
gtctgtctat tttgcgtgca ggcggtcgag ataggcgact tcttcgctgc tgcccatgaa 1078920
gacggcgacg cgttggtgca ggttttcggg ctgtatgtcg agcatggctt gatatgcgtt 1078980
gcttgccgat gcgcccgcct gttcgagtat caggctcata gggttggctt cgtacatcag 1079040
gcgcagtttg ccgggtttag cggggtcgcg tttgtcttgc ggatacatga acacgccgcc 1079100
gcgcatcagg atgcggtgga tttcggcaac catactggct acccagcgca tattgtagtt 1079160
tttgccgcgc gtaccggttt cgcccgccaa gagctcgtcg atgtattgtt ggacgggggg 1079220
cagccagtgg cggcggttgg acatattgat ggcaaattct ttggtacttt cgggtacttt 1079280
cgggttttct ttggtcagca caaattcgtt ttcggcattg agcgtgaaca tatatacgcc 1079340
atgtccgaat gtgaatacga gctgggtttg aggcccgtaa agaacgtaac cagcggcaag 1079400
ctgctgtctg cccgtttgaa ggaatgattc ggttgccaat gcgccttcgg gtttttcaag 1079460
gatggagaaa atcgtaccga cggaaatgtt gacatcaata ttggacgatc cgtctaaagg 1079520
gtcgaatagg acgagatagc gtccgttttc accggcattt acgaaagtgt cttcttcctc 1079580
gctcgccagc ccggcaacgg cagaattggc tttgagtgtg tcaatcatga tgttgttggc 1079640
gataacatcc agttttttttt ggtcttcgcc ctgaatattg cccgtgcccg ccatacccaa 1079700
tacgccggcc agtgcgccga ggcggacttt ggcgttgatt tcggtgcagg cggaaacaac 1079760
ggacagtaaa acgccgccga gtgcttcggg cagctggttt tgttgcaggt gttcggggag 1079820
gaatcgggtc agtgtgtcca tagtttgctc gtttcggaaa ggtttgtgcc gtctgaaagg 1079880
cggcaggtta ttgtggcgta ttcctttggt gcgttttgca ggatagtcta ggggattgta 1079940
gtgtaaaaaa acggcatggg gcaagtcgga acgcggcagg cggatgaggg gatatttatt 1080000
cttaaaagtg ccgactgccg gtatatcgtc cggttttgtt tatttgacgg gagatgttgt 1080060
ctgaagggtt tcagacggca tcggggtcag cggattttgc tgtccaaaag gtagcgcgag 1080120
ccttcgtctt gcgccagcag ccgcgtcagg gcggggaggt ttgccgccaa ttgttccgcc 1080180
aacagatagg gcggattgat gacgaacatt ccgctgccgt gcataccgaa accgtcggct 1080240
ttcggcgcgt ggacgtgaag ttcggcgtga aggtagttgt cgggcaggag ttttttcaat 1080300
tcttcgggca gcttgcggct ttcttcgcgg ctgaggcagg gataccaaat gagataacag 1080360
ccggactcaa accgttttaa agcggctttc agcgtttccg ttacacgccg gtagtcctgt 1080420
ttttcctcat agggcgggtc gatgaggacg gtggcgcggc gcggcggggg cggcagcagg 1080480

gaaatcagcc ctttgtaacc gtcttcgcgt aatacttgtc cgcgtttgcc caatcctgct 1080540
tcgcccatat tgttttgcag atggacaaag tcggtggggt gcagctcaaa caggcgtaat 1080600
ttgtcgccga cgcgggtcag cgattgcgcc agccacggag aaccgcagta aagtttgggc 1080660
gagggcagga ttttttgtat gtgcgcggca aagtcagaga gttcggcagg caggttttgc 1080720
gcctgtcgga gcagggcgat gccttgtcgg tattcgccga ctttctgcgc ctcgctgcct 1080780
tcgagattgt acacacccgc gccgccgtgc gtgtcgatgt accagtaggg cttgtctttg 1080840
cggttgaaat attgcagcac taaaaacaag gtgaaatgtt tgagcatatc ggcgtggttg 1080900
ccggcgtgga atgcgtgtct gtaactgagc atagtcggta aaacggcggg atattcggat 1080960
gccgtctgaa gcggggttca gacggcataa acagggatgg atgggaaatc agcggctgcc 1081020
gccgattttg ctttctctgc cttcgagcag cttgacgata ttacttttgt ggcggaacaa 1081080
caccagcaaa gcaatggcga cggtcgccca aacccacgag acgtgcggca taaagaagga 1081140
tgcggcgacc ggtgcggcga ttgtggcggt taatgcggca agggaggaca ccttgaagcc 1081200
gaatgccata acaagccaaa tcaacgcgca gaccaaggca gttgcgggag agagtgccag 1081260
aagcacgccc aatgccgttg ccacgccttt gccgccttta aatccgaaaa acaccggcca 1081320
catatgcccg accagcgcgg cgagtgcgac ggccgcgatt gcgctgtcgg ataaaccgag 1081380
cggttcttga agcacgcgtg caagcaaaac ggcaactaaa cctttggcgg catcgcccaa 1081440
gagcgtcagc gcggccgcct ttttttgcc gctgcgtaaa acattggttg cccccggatt 1081500
gcccgatccg taggtgcgcg ggtcgtccat gccgtaatac ttggacacga tgacggcgaa 1081560
agaaagtgag ccgatcagat aggaaacagc aacagccggt atgttgaaca tttgcggtac 1081620
tttacttaga atggtgcggt tattttagca aaaaacgggg cggattatgg ataaaatctt 1081680
tttgcacggc atgaaggcag atacgcttat cggcgtgtac ggctgggaac gcgaacggtt 1081740
gcagaccctg attgtcgatt tggacatcgg tgttcccgag aaagcgggtt cggacgacga 1081800
tattgccaat acggtgcatt atgccgaggt atgcgaaacg ctgcgccgac atctgaaaga 1081860
acaggatttc ctgcttttgg aagcgttggc ggaatatatt gccgatttgg ttttgggata 1081920
tttcggcgcg gtgtgggtgc gcgtgaaaat cgtcaagccg ggtattttgg aaggcgtgcg 1081980
cgaggttggc gtggaaatcg agcgcggcaa gcgtgaagat tgaacggcag aataggaaac 1082040
ggaaaggaga tatgaagtgg atttgaggga agtaaaatta ggcggcgaaa ccatttacga 1082100
gggcggtttc gtcagtatca gcagggataa ggtcaggttg cccaacggca atgaagggca 1082160
gcgtatcgtc atccgccatc cgggtgcggc atgcgtgttg gcggttacgg acgaagggaa 1082220
agtggttttg gtgcggcagt ggcgttatgc ggcaaatcag gcgacattgg aacttcctgc 1082280
gggcaagctg gatgtggcgg gcgaggatat ggcagcgtgt gcgctgcgag aattggcgga 1082340
ggaaacacct tataccgccg acagcgtacg cctgctttac agtttttata cggcggtcgg 1082400
tttttgcaac gaaaaaatgt atctgttcga agcggaaggc gtgcgtttgg gcagtacgct 1082460
tgccaatgac gaagacgaga ttacggaaac cgtattgatg tcgaaagaag aagtccgtca 1082520
ggcattggca aacgatgaaa ttaaagacgg caagacatta atcggtttgc aatactggtt 1082580
gatgaaggat tgacaggatg ttggacttgc ccgccggatt ggatcggcgg gcggtttgtt 1082640
tggcggatgg gatatgcctt ttcggcttgt atctgggcgc gtcctttaaa gtcattcgtg 1082700
ctttagtaat aagagagaaa aggggatgat aattacctaa aagaacgtga taatttttaa 1082760
aatggttaat aatgaatatc tttgttacta atttttgtta ttggtttatt agtttattgg 1082820
ctatttctta tataccatct attaatgcat ggcatgatga attaatagat gatattaatt 1082880
ttggcaaaag ggttatgatg gttacttttt ttgcattttt aggcacggta atagagcgtt 1082940
tttttaagaa aaagccttgg tggttttatc ctgccaaggc tttttctttg ttacagacct 1083000
aaaagctcaa tttgaatttg agaacggtaa ttggcacagc cagtatttaa acaagcgaag 1083060
ctaatttata gattatgtca aaacaaaggg aggcaatttg ttgcggttat ttgactgccg 1083120
cccctatctt cagcccgagc caggtcagca gcagcgaacc tgccgtgtgc aggaaaatat 1083180
tggcaagtgc tgaagcggga cggttcaatt ggagcagggt tacggtttcc agcgaaaatc 1083240
cggaaagcgt ggtcaggctg ccgagaaaac cggtaatcag cagcagcttc cattgcgggt 1083300
ggttgacggt ttcggcaaag attccgataa gaaaagcgcc tatccagttg gcaaacaggt 1083360
tgcctgtggc gggaggtatt gatgcgggaa cggcgaggtt gagcagccaa cgcgccgttg 1083420
caccgagtgc cgcaccgatg gaaaggggga tgatgttgga aagcatggtt ttgcctgtct 1083480
atgccgtctg aaggctaccg ccatatgccg cggtcggact taagatagcg gttgtcgtcg 1083540
aaagtgttaa tccaatgggg cttcagtgca acaaatatgg cagttgaaat gccgctgagg 1083600
aaggcttccg cccacgccag cagaataaag acgggcaggg cggtcgtcca caatatttcg 1083660
gacggaaaag cgtttgcggc atccaaaata ccggtcagca ccagcccggt cagcagaatg 1083720
ccggcggcgg aagcgagaaa gccgttgacg aaaataaaga tgaaaatatt gggcggcagg 1083780
cggttgacca gcatacgcga caggcggttg acggtcagcg cgggcagtat cagcaccaaa 1083840
gcgttcggcg gatatgcgcc gacagaaccg gcaaacagca ggcagtaggg cagcatcagc 1083900
agcgcggcaa gccaaagggc ggcggaagtg cccatcatca gtgcaaccaa attgacggcg 1083960
agcaggtggt agttcatctg ggcaagctgt ccgccgccgg cagaggcgtt cagacaccat 1084020
gctgcggaaa aaattacggt acacagggga agggcggaac ggtagcgggc aagcgagcgg 1084080
aatgccgacg gcgcggaagc tgccagtatc aggataagga caatccacga aaccgacagt 1084140

accatatctg aaaaccagac tgtttggaaa atcatggcaa tgccgcaaag attaagggaa 1084200
gggacggcta ttatactgtc ggcgggggca aaccgaaagc cgaatcggtt tcggcagaat 1084260
tgccggccgg ttgttttttt tgggatggaa acacgttaaa ataaacccgt ttaatcgttt 1084320
gtcttgcggg aaacggcata tgttttggat agttttgatc gttattttgt tgcttgcgct 1084380
tgccggcttg ttttttgtcc gcgcacaatc cgaacgcgag tggatgcgcg aggtttctgc 1084440
gtggcaggaa aagaaagggg aaaaacaggc ggagctgcct gaaatcaaag acggtatgcc 1084500
cgattttccc gaacttgccc tgatgctttt ccatgccgtc aaaacggcag tgtattggct 1084560
gtttgtcggt gtcgtccgtt tctgccgaaa ctatctggcg cacgaatccg aaccggacag 1084620
gcccgttccg cctgcttctg caaaccgtgc ggatgttccg accgcatccg acggatattc 1084680
agacagtgga aacgggacgg aagaagcgga aacggaagaa gcagaagctg cggaggaaga 1084740
ggctgccgat acggaagaca ttgcaactgc cgtaatcgac aaccgccgca tcccattcga 1084800
ccggagtatt gctgaagggt tgatgccgtc tgaaagcgaa atttcgcccg tccgtccggt 1084860
ttttaaagaa atcactttgg aagaagcaac gcgtgcttta aacagcgcgg ctttaaggga 1084920
aacgaaaaaa cgctatatcg atgcatttga gaaaaacgaa acagcggtcc ccaaagtccg 1084980
cgtgtccgat accccgatgg aagggctgca gattatcggt ttggacgacc ctgtgcttca 1085040
acgcacgtat tcccatatgt tcgatgcgga caaagaagcg ttttccgagt ctgcggatta 1085100
cggatttgag ccgtattttg agaagcagca tccgtctgcc ttttctgcag tcaaagccga 1085160
aaatgcacgg aatgcgccgt tccaccgtca tgcagggcag gggaaagggc aggcggaggc 1085220
aaaatccccg gatgtttccc aagggcagtc cgtttcagac ggcacggccg tccgcgatgc 1085280
ccgccgccgc gtttccgtca atttgaaaga accgaacaag gcaacggttt ctgcggaggc 1085340
gcgaatttct cgcctgattc cggaaagtca gacggttgtc gggaaacggg atgtcgaaat 1085400
gccgtctgaa accgaaaatg ttttcacgga aaccgtttcg tctgtgggat acggcggtcc 1085460
ggtttatgat gaaactgccg atatccatat tgaagaacct gccgcgcccg atgcttgggt 1085520
ggtcgaacca cccgaagtgc cgaaagttcc catgaccgca atcgatattc agccgccgcc 1085580
tcccgtatcg gaaatctaca accgtaccta tgaaccgccg tcaggattcg agcaggtgca 1085640
acgcagccgc attgccgaga ccgaccatct tgccgatgat gttttgaatg gaggttggca 1085700
ggaggaaacc gccgctattg cggatgacgg cagtgaaggt gcggcagagc ggtcaagcgg 1085760
gcaatatctg tcggaaaccg aagcgttcgg gcatgacagt caggcggttt gtccgtttga 1085820
aaatgtgccg tctgaacgcc cgtcctgccg ggtatcggat acggaagcgg atgaaggggc 1085880
gttcccatct gaagaaaccg gtgcggtatc cgaacacctg ccgacaaccg acctgcttct 1085940
gcctccgctg ttcaatcccg aggcgacgca aaccgaagaa gaactgttgg aaaacagcat 1086000
caccatcgaa gaaaaattgg cggagttcaa agtcaaggtc aaggttgtcg attcttattc 1086060
cggccccgta attacgcgtt atgaaatcga acccgatgtc ggcgtgcgcg gcaattccgt 1086120
tctgaatctg gaaaaagatt tggcgcgttc gctcggcgtg gcttccatcc gcgttgtcga 1086180
aaccatcccc ggcaaaacct gcatgggttt ggaacttccg aacccgaaac gccaaatgat 1086240
acgcctgagc gaaatcttca attcgcccga gtttgccgaa tccaaatcca agctgacgct 1086300
cgcgctcggt caggacatca ccggacagcc cgtcgtaacc gacttgggaa aagcaccgca 1086360
tttgttggtt gccggcacga ccggttcggg caaatcggtg ggtgtcaacg cgatgattct 1086420
gtctatgctt ttcaaagccg cgccggaaga cgtgcgtatg attatgatcg atccgaaaat 1086480
gctggaattg agcatttacg aaggcatccc gcacctgctc gcccctgtcg ttaccgatat 1086540
gaagctggcg gcaaacgcgc tgaactggtg tgttaacgaa atggaaaaac gctaccgcct 1086600
gatgagcttt atgggcgtgc gtaatcttgc gggcttcaat caaaaaatcg ccgaagccgc 1086660
agcaagggga gaaaaaatcg gcaatccgtt cagcctcacg cccgacgatc ccgaaccttt 1086720
ggaaaaactg ccgtttatcg tggtcgtggt cgatgagttt gccgacctga tgatgacggc 1086780
aggcaagaaa atcgaagaac tgattgcccg cctcgcccaa aaagcccgcg cggcaggcat 1086840
ccatttgatt cttgccacac aacgccccag cgtcgatgtc atcacgggtc tgattaaggc 1086900
gaacatcccg acgcgtatcg cgttccaagt gtccagcaaa atcgacagcc gcacgattct 1086960
cgaccaaatg ggcgcggaaa acctgctcgg tcagggcgat atgctgttcc tgctgccggg 1087020
tactgcctat ccgcagcgcg ttcacggcgc gtttgcctcg gatgaagagg tgcaccgcgt 1087080
ggtcgaatat ttgaaacagt ttggcgaacc ggactatgtt gacgatattt tgagcggcgg 1087140
cggcagcgaa gagctgcccg gcatcgggcg cagcggcgac gacgaaaccg atccgatgta 1087200
cgacgaggcc gtatccgttg tcctgaaaac gcgcaaagcc agcatttcgg gcgtacagcg 1087260
cgccttgcgt atcggctaca accgcgccgc gcgtctgatt gaccagatgg aggcggaagg 1087320
cattgtgtcc gcaccggaac acaacggcaa ccgtacgatt ctcgtcccct tggacaatgc 1087380
ttgatttttt gcaaatggaa atgccgtctg aagactgttt cagacggcat ttttatagtg 1087440
gattaacaaa aatcaggaca aggcgacgaa gccgcagaca gtacaaatag tacggaaccg 1087500
attcacttgg tgcctcagca ccttagagaa tcgttctctt tgagctaagg cgaggcaacg 1087560
ccgtaccggt ttaaagttaa tccactatat cagacatttg aattcggatt attccctgac 1087620
ctgtcccgtg ccttgtacga tgtatttgta actcgtcagc tctttcaaac ccatcgggcc 1087680
ccgggcgtgg agtttttgcg tggagatgcc catttcgcaa cccaagccga attcgccgcc 1087740
gtcggtaaag cgcgtggacg cgttgacata cacggcggca gaatcgatat gagtcgtgaa 1087800

atagtccgca gcgtggcggt tttcggtaac gatgccgtct gaatggtgtg tgctgtgggt 1087860
ttcgatgtgc cagaccgcct cttcgaccga agcgacggtt ttcacagcga ggatgtagtc 1087920
taaaaactcg gtatcgaaat cgtctgcacc cgccgcttcg ccgccgatat gccgcgccgc 1087980
ctgcggatcc aaacggaagc ggatgggcgg cagtccggct tctatgcggt cgcgaaccaa 1088040
cagccgttcg agcttgggca ggaagtcggc agcaatgtct tcatgtacca gcagcacttc 1088100
catcgagttg cacacggacg gacggctggt tttggcgttg tacacgatac ggagcgcctt 1088160
gtcccaatcc gcgtccttgt cgatataaat gtggacaatg cccgttcccg tttcaatgac 1088220
cggcacgacg gcattttcaa ccaccgcccg tatcagcccc gccccgccgc gcggaatcag 1088280
caggtctaga taatctttcg ccctcatcat ttcgtaactg ctttcgcgcc cggtgtcttc 1088340
aatcagttgg agcgcgtcgg ggtcgatgcg ggtttgcgcc aaccccgttt tcagggcggc 1088400
aacgatggcg cgtgcggatt ggaatgcatc tttgccgctg cggagtacga ccgcgctgcc 1088460
gcttttcagt gccaaagccg ccgcatcgga agtaacgttc gggcggcttt cgtaaataat 1088520
gccgataacg cccatcgcca cgcgcttttt gacgatttcc aagccgttgg gcaaagtcga 1088580
ggtttccagt atttcgccca cggggttggg cagcgcggca accgccctga tgccgtccgc 1088640
catcgcgcaa atgcgtttgc cgtccaacaa aaggcggtcg gtcatgcttt cgggaatgtt 1088700
gcctgccgcg gcttccaagt cttgacggtt tgccgccaaa atatctgccg tcgccgcttc 1088760
caagctgtcc gccatcgcaa gcagcgcgcg gttttttctt tccgtatccg ccgtgttgac 1088820
ggattttttt gccgctttgg caagggcaag ctgtttttgt gtgtttgaca tgggtttcct 1088880
tttctaaaat tcggtcagaa gcaggcgtat ttcgggcgtg atggaaatcc agtcgtcccg 1088940
atggatgaac acgcctttcg ccttacgcga tttgagcagg tcttcggcgg cggcagagcc 1089000
gaacaggacg cgccctttgc ccaggggctg tttggttgcc ttgctgtaca cggttacggt 1089060
gtccatacgg gaaaaatgcc cttcgattcc ggcaatgccc gacatcagca ggcttttccc 1089120
ctgttcggac aaagcgtgtt ccgcaccttc gtccacataa acgctgcccc ggctttcgga 1089180
atagaacgcc agccattgct tctgcgtccg caaacctttg gcacggggga cgaaaaacga 1089240
gccgtccgcc tgatgttcgg cagcttcggc aagtgcatcg ggtttgagcg aggaacagat 1089300
atacaccggt acgccggatt cggcggcgat ggttgccgct ttgattttgg tcagcatacc 1089360
gcccgtgccg tttgccgaac ccgagccgcc cgccatttcg atgatttcat ggttgatgtg 1089420
ttcgattttg tccagccgta cggcatcggg attgctgttc gggttgcccg tgtaaagacc 1089480
gtctatgtcg gtcagcagca ccaagaggtc tgcctgtatc atcgccgcca cttgcgcact 1089540
caatgtgtcg ttgtcgccga ttttcaattc ctcaaccgaa accgtatcgt tttcattgat 1089600
gatggggacg gcgcggcgtt gcagcagcac ggaaagtgcg ccgccggcat tttggtagcg 1089660
gcgtttgtcg gcaaagtcgg cgcggctgag caggatttgc gcggacacga tgccgtctga 1089720
agacaggttt gccgtatatt cttccatcag cagcccctgc ccgacggcgg cggaagcctg 1089780
tttgtcggcg attttgaccg gacgtttttt gaaacccagc gcaccgaacc ctgccgcaac 1089840
cgcgccggaa gacaccaaga ccagctcgtg tcccgcatga tgcaatgcgg caagctggca 1089900
ggtgatggtt tggattttgc cgcgcgagag actgccgtcc gaatgggtaa tcgaagatgt 1089960
gccgacttta aatacgattc ttttgtattt cattgtttcc gtccttgttg gtttgtcctg 1090020
tctcgttgcc accttgtgcc gccgaatttg ccctgttctg ccgcaattgt caacaatcac 1090080
gccgcgtctg caataaaatg gacaaaatgt ataaaattaa taaaatctat ggcggcttat 1090140
tgagattttt caaatttata ttgccgtttt gtccaaaatg cgtataatcc tgtccatatt 1090200
tctgctgtag gctgatttat tttagacaag gactaccatg caattagata tagaccgctt 1090260
ggttgcttat ttcggcggcg tgaacgcgct tgccgaagcg ttgaaacagc acgatcccga 1090320
aaatgccgcg acgaccgccg ccatctataa atggcgcacg cgcggctcgc tgcctctggc 1090380
gcaactgcaa aagctgaccg cgttggcgga agcgcaaggc aggccgctgg atttgaatgc 1090440
ttttttacaa aaaaacgaat ctctggagag aacagaaatg acacagacca accgcgttat 1090500
cattttcgac accaccctgc gcgacggcga acaatcgccc ggcgccgcta tgaccaaaga 1090560
ggaaaaaatc cgcgtcgccc gccagctgga aaaattgggt gtggacatca tcgaagcggg 1090620
ttttgccgct gccagcccgg gcgatttcga ggcggtcaat gcgattgcga aaaccattac 1090680
caaatcaacg gtctgttcat tgtcccgcgc catcgagcgg gacatccgtc aggcgggtga 1090740
ggccgttgcg cccgcgccga aaaaacgcat ccacaccttc atcgccacca gccccatcca 1090800
tatggagtac aaattgaaga tgaagccgaa gcaggtgatt gaggcggcgg tcaaagcggt 1090860
gaaaatcgct cgtgaataca ccgacgatgt ggaattttcc tgcgaagacg cgttgcgttc 1090920
ggaaatcgat ttccttgccg aaatctgcgg cgcggtgatt gaagcgggcg cgaccaccat 1090980
caatattccc gataccgtcg gctattccat cccgtataaa accgaagaat ttttccgcga 1091040
actgattgcc aaaacgccca acggcggcaa agtcgtttgg tcggcacact gccacaacga 1091100
tttgggcttg gcggttgcca attcgcttgc cgcattaaaa ggcggcgcgc gtcaggtgga 1091160
atgtactgtc aacggcttgg gcgaacgtgc aggcaatgct tcggttgaag aaatcgtgat 1091220
ggcgttgaaa gtgcgccacg acttgttcgg cttggaaacc ggcatcgata ccacgcaaat 1091280
cgtgccttcg tccaaactgg tgtccaccat tacgggctat cccgtgcagc ccaacaaagc 1091340
cattgtcggt gccaatgcct tttcgcatga atcgggcatc catcaggacg gggtgctgaa 1091400
acaccgcgaa acttacgaga ttatgtccgc cgaatcggtc ggctgggcaa caaaccgttt 1091460

gagcttgggc aaattgtccg gccgcaacgc cttcaaaacc aagctggcgg atttgggcat 1091520
cgagttggaa agcgaagagg cactgaacgc ggcatttgca cgcttcaaag aactcgccga 1091580
caaaaaacgc gaaatcttcg atgaagacct gcacgcactg gtatccgacg aaatgggcag 1091640
catgaatgcc gagagctaca aattcatctc ccaaaaaatc agcaccgaaa ccggagaaga 1091700
accgcgcgcc gacatcgtgt tcagcatcaa aggtgaagaa aaacgcgctt ccgcaaccgg 1091760
ttccggcccc gtggatgcga ttttcaaagc gattgaaagc gtggcgcaaa gcggcgcggc 1091820
tttgcagatt tattccgtca acgccgtcac gcaaggtacg gaaagccagg gcgaaaccag 1091880
cgtccgtctg gcgcgcggca accgcgtcgt caacggtcag ggcgcggata ccgacgtttt 1091940
ggtcgccacc gccaaagcct acctttccgc tttgagcaag ctggaattta gtgccgccaa 1092000
accgaaagcg cagggcagcg gtacgatttg agcgtgaaaa cagacgatgc cgtctgaagc 1092060
ataaaaaggc ttcagacggc attgcggcga taatagggcg caaaacccat ttgaaaagga 1092120
aaatgatgga ttcccgaaaa tttaccgaag catccaaacg gcggttgagc gaattgttgg 1092180
atgccaaaag cgaacaaggc aacacgatgc gttgcgacga ggttcaaggt tttatgacgg 1092240
cgctgttgag cgggccggac aaattgacac cgctcgactg gctgcccgaa gtgttgggcg 1092300
acgaatcgca atttaccgcc gccgaacgtt ccgaaatcga acggctggtt ttggcaatgg 1092360
cgatggaaac aaccgccgcg atgtcggata aaaaactgcc cgatttgtgg ctgtatgaaa 1092420
acgaagacgg cggcagcgat ttttacacat ggtgcaatgc ttatctttac ggtttggata 1092480
ttgtgccgac cgattggttt gaagccgtcg atgatgaagc gtttgaagag ttgttttatc 1092540
ccatcatggc attgggcggt atttacgacg aagaggaaaa cggcgctatc cgtctgcaat 1092600
tcacagaagg cgagctggcg gaactggaat ccgagttgcc ttatgcattg gcggatatct 1092660
accgctactg gcaggcagtc atcaacaaac cgcaaaccgt ccgcagggaa ggcgaaaaaa 1092720
caggcaggaa cgatccctgt ccgtgcggca gcggcagaaa atacaaggcg tgttgcggta 1092780
agaattgaag cgtttgtttc catgaaccaa acgtaaaaat accgtctgaa accggatttc 1092840
catgtttcag acggtatttt tcacaggcgg tcagtgctgt tttttcatgc cgaaccggac 1092900
aaagccgacg atacccaaaa caatcatcgg gacgctcaac cattgcccca tcgacagccc 1092960
caaggtcagc agcccgagat agtcgtcggg ttggcgtgcg aattcggcaa tgaagcggaa 1093020
tatgccgtag ccgccgagga agagcgaggc gacttgtccg gtcgaccgct gtttttttaga 1093080
gaacagccaa atgacggtga acaggcagat gccttcaagt gcaaactgat aaagctgcga 1093140
gggatgacgc ggcagcatac cgtattgttg cagccattct gcccaaagcg gattgtgcgc 1093200
ggcggcttcg gcatcttcgt aacgcgcctg cgggaagccc attgcccaaa atgcgttgat 1093260
gtcggtaacg cgtccccaaa gttcgccgtt gatgaagttg ccgatacgtc ccgaagcgag 1093320
acccagcgga acgagcggtg cgaccgtatc catcagtttg aggaagccga tgccgtgttt 1093380
gcggccgaac aaccgtatgg caataactac acccaaaaag ccgccgtgga acgacattcc 1093440
gccttcccat accttgaaaa tatcaagcgg atgggcgagg tagtcggaaa acttgtaaaa 1093500
caggacgtaa cccaaacgcc cgcccaaaat tacgcccaaa atgccccatg tcaggaagtc 1093560
gtcgagcgat tctttggtaa aaacggacaa gccttgcgcg atgcgccttc tgccgagaaa 1093620
ggtaaaaaga ataaatccga ggatgtagct tagggcatac cagcggacgg caagcgggcc 1093680
gatactgata aggacgggat cgaattgggg atgggtaatc ataacgggct ttcgttttca 1093740
aatgccgtct gaaaggcatg atgcttcaga cggcatttct gcaataaggg tttcagcgca 1093800
aatcgccgat gacgttgagg atagcggaca acgcggcttc gcccagccgt aaagaacgct 1093860
gaccgttcca gccgaagtcg tcgtcgggca gattggcatt gtctttgaac ggcatttcca 1093920
gcgtataggc aaggcagttg aaacggttgc cgacccagtt ggtcgccaag gtcatattcg 1093980
cttcgcccgg cgcatctttt tcgtaaccgt attcgtcttg gaaatcgggg ctggcgttta 1094040
aaagggcatt tttaaactgc gcttccaacg cggcgatgcg cggattgtag ttcggcacgc 1094100
cttccgtacc tgcgacaaag acaaagggca gcccttcgtc gccgtggatg tccaaaaaca 1094160
aatccactcc ggtttccagc attttttcgc gcacgaagaa cacttccggg ctttttttcta 1094220
ccgtcgggtt ttcccactcg cggttgaggt tcgcgccggc ggcgttggta cgaaggttgc 1094280
ccagtgccga accgtcgggg ttcatattgg ggacgatata gaacgtggcg cggtcgagca 1094340
aggcgcgggc ggtagggtct tgcgggtcga gtaatctgcc gagcagcccc tcgataaacc 1094400
attccgccat ggtttctccc ggatgctggc gggcggtaat ccagattttc aaatcgcttt 1094460
cgacctgatt gcctatggtc agcagattga tgtcgcgccc ttgcacggtg ctgcccaagt 1094520
cgtcgatgcg gcacaggccg ctgccttgcg cgtcgccgag gaggtttaaa tgctgttctt 1094580
cggagtaagg ttcgaaatag gcgtaataca cgctgttgga cagcggagta tgattgacgg 1094640
tcagtacgcc gttttcgtag gaagtcggta cgcggaacca gttgcggcgg tcgtatgagg 1094700
cacacgcctg atagccttcc cagcctttcg ggtaggcggc ttctgccgcg ttttcaaaat 1094760
gcatgatgca gttttgatat gccgcgcctt gcagccggaa gtagaaccat tgtgcaaaat 1094820
cggaggcgtt gtcgggacgc agggcgaggc ggatgttgga aggatcggtc aggtctttga 1094880
cgacgaccga gccggcatcg aagcgggtgc tgattttaat catgggaaag tccttgctgt 1094940
cgccggtttc tcgaaccgga taaaccgcga ttttaccgcc cgtatcgcaa ggcttcaacc 1095000
tgcccgaaag tctgccggat gccgtctgaa gattgtttca gacggcgttt ggcgttaaca 1095060
taagccgaaa ttgtcaacaa tagggagccg ttatggagtc tgaaaacatt atttccgccg 1095120

ccgacaaggc gcgtatcctt gccgaagcgc tgccttacat ccgccggttt tccggttcgg 1095180
tcgccgtcat caaatacggc ggcaacgcga tgaccgaacc tgccttgaaa gaagggtttg 1095240
cccgcgatgt cgtgctgctg aagctggtcg gcattcatcc cgtcatcgtt cacggcggcg 1095300
ggccgcagat caatgcgatg cttgaaaaag tcggcaaaaa gggtgagttt gtccaaggaa 1095360
tgcgcgttac cgacaaagag gcgatggata ttgtcgaaat ggtgttgggc gggcatgtca 1095420
ataaagaaat cgtgtcgatg attaacacat atggcggaca cgcggtcggc gtaagcggac 1095480
gcgacgacca tttcattaag gcgaagaaac ttttgatcga tacgcccgaa cagaatggcg 1095540
tggacatcgg acaggtcggt acggtggaaa gcatcgatac cggtttggtt aaagggctga 1095600
tagaacgtgg ctgcattccc gtcgtcgccc ccgtcggcgt aggtgaaaaa ggcgaagcgt 1095660
tcaacatcaa cgccgatttg gtagcaggca aattggcgga agaattgaac gccgaaaaac 1095720
tcttgatgat gacgaatatc gccggtgtga tggacaaaac gggcaatctg ctgaccaaac 1095780
tcacgccgaa acggattgat gaactgattg ccgacggcac gctgtatggc ggtatgctgc 1095840
cgaaaatcgc ttctgcggtc gaagccgccg tcaacggtgt gaaagccacg catatcatcg 1095900
acggcaggtt gcccaacgcg cttttgctgg aaatctttac cgatgccggt atcggttcga 1095960
tgattttggg cggtggggaa gatgcctgaa gcaaagtcgg aaaatgccgg ctttggcgga 1096020
aaacctgttt gtctggtttc tgtttttggg gtttcgggca atttccaaac cgtcattcct 1096080
gaaaaaatat agtggattaa caaaaaccag tacggcgttg cctcgcctta gctcaaagag 1096140
aacgattctc taaggtgctg aagcaccaag tgaatcggtt ccgtactatt tgtactgtct 1096200
gcggcttcgt cgccttgtcc tgatttttgt taatccacta tagaaacaaa aacagaagcc 1096260
taagatccgt cattcccgcc gggcatctgg ttttttgaaa tccggttgtt tgggataaat 1096320
tctccggctt tgattttttg tttttccgat aacgccataa ctttgaaatt tcgtcattcc 1096380
cgcgcaggcg ggaatctaga cctgtcggca cggaaactta tcgggaaaaa aggtttcttt 1096440
agattttata gtggattaac aaaaaccagt acggcgttgc ctcgccttag ctcaaagaga 1096500
acgattctct aaggtgctga agcaccaagt gaatcggttc cgtactattt gtactgtctg 1096560
cggcttggtc gccttgtcct gatttttgtt aatccactat acgtcctaga ttcccacttt 1096620
cgtgggaatg acgggatgtg ggtttttgtg cggatttgaa ccggtaaggg tggtgtggga 1096680
ttggtggttt gcttaggatc ttttggattg tattttgtat atacatttac ttgttgataa 1096740
aagataaaat aaaattagaa actaaaagtg agaaaaaatt aataataata gggatgtata 1096800
aatgtaaagg ctccgtttca tagctaaggt tatctgaata tatggaaaaa aagtaaaagt 1096860
ccataaaact aaaatatata gataatgcta atgataatag aattatcact ttcctagagt 1096920
aaggataata tttttttatc gtaaaaaaat ataaaatagg ataaataatt gccatataga 1096980
tagattttgt aacaaaatta aaaataaata ataaaaataa tgtaataaag tatatttttt 1097040
ggctatgaaa taaaattgta cataattgaa cgagcagatc aaaaaatgaa ctacatataa 1097100
caataaataa taacgtattt accatactaa atttaatagg tctcattatc atatttaata 1097160
accacttcat agtatagtgg attaaattta aaccagtaca gcgttgcctc gccttgccgt 1097220
actatctgta ctgtctgcgg ctttgtcgcc ttgtcctgat ttaaatttaa tccactataa 1097280
atgcagagtg ggtggaaaca ctcactttat ggtttgctac gctctgctca attagcaacc 1097340
cgataaccca atatggataa tagggtaatt aatccaatct aatttgtcag catccgttaa 1097400
tttattgcaa aataaagtat tgaattatgt cgggtgcaaa tgacgaaata taagtttccg 1097460
tgcggacgga tcaagattcc cactttcgtg ggaatgacgg tggaaagatt gttgtttttc 1097520
ccgatgaatt cctgtgtttt ttgtttttcc ggataaattc ctgtggcttt gagtttttttg 1097580
gatttcagcc tcaatgccgt ctgaacgccg aatcgggctt cagacggcat tgcgtcattt 1097640
gaaattcaaa accggccagc ctttttcttt ggcttctttt tccagctcgg catcggggtt 1097700
gacggcgacg ggttcgctga caaggcgcag cagcggcagg tcgtttttgg agtcgctgta 1097760
aaaataggtt ttgccgtagc tttggagcgt ttcgccgcgt tcggcaagcc attggttcag 1097820
gcgggtgatt ttgccttctt tgaggctggg cgtgccgatg taattgccgg tgtagcggcc 1097880
gtcagaaccg gtttcgagtt gtgtgccgat gatgttggtg atgccgaaaa ggtggcagac 1097940
gggggtgatg atgaactcgt tggttgagga aatcacaagg gtttcgtcgc ctgccatttg 1098000
gtggctctgc accagcatac gctgcatagg cgagatgtgg gggatgatgt attccgccat 1098060
aaattcgcgg tgaaactctg ccagctcttc tttgctgtaa cgagcgagcg gggcaaggtg 1098120
gaatttgagg aatgcgtcga tgtcgaggca gccgttttgg tagtcgcggt agaatttttc 1098180
gttttgcgct tcggtttcgg cagcgtcaac caagcctttt ttgatgaggt attgcggcca 1098240
ggcgtggtcg gaatcggtgt tgatgagggt gttgtcgagg tcgaagatgg cgaggttttt 1098300
cattgggttt cctgttgttt caaaagctgg cgcaaaagcg gcagggtgat gcgtttgccc 1098360
atcgtgacgg cgtagttgtc cagcgtgtcg agcatcatca tcaggctgtc catatcgcgc 1098420
cgccagtgtt tgagcaggta ttcgaaaatt tcggaatcga cggttacttg gcgtgccgcc 1098480
gccatactgg cgagcgcgtc gattttttct tggtcggtta agggtttgac ttcgtaaacg 1098540
aggcagtacg ccatacgcgt ccgcaaatct tcgcggatga caagctgctg ggcgtgtat 1098600
tccgaaccga gcagcaaaaa gcctttgccg ctgttgcgga agcggttgaa gatggaaaaa 1098660
agcagggctt gttcttcgtt gcccagtttt tcgacttgat cgacggcgag gtattccgcc 1098720
tcgaacgcgg catcggtcag cggcatggag gcggcatcga tataggcggc gtttttgccg 1098780

gcttcgagcg cctgtgcgac ccacgcctgc aaaagatggc ttttgcccgc gccttcttca 1098840
ccccagacat agataaactg tccgtgtttg tgtcggagga catagaccag ttccgcgttt 1098900
tccgtgccga ggaatttgtc gaaactcgga tagtcgtgtg cggcaaagtc gaaaataagc 1098960
tggttcacgg ttcggcattc cgaggggtgg taaacgggtt tattgtacgt tgttttcgcg 1099020
cgcctttcca atttgaacga tgccgtctga aaacggcttc agacggcatc gttcaaccgc 1099080
aggcaacgtt gccgacatcg aggcgcatat tgtggaacgc gttgagcgtg ctgcggtggc 1099140
cgatgctgat gatgatgctg tcgggcagtt tttgtttcag tgcgcggtag agcagggcct 1099200
cggtcggttc gtccaaagcg gcggtggctt cgtcgagcag gacgattttg ggcttggaaa 1099260
gcagggcgcg gacgaaggcg acgcgttgca gttcgcccgg ggagagtttg tgttgccagt 1099320
cgtcggtttt atctaattta tcaaccagat aacccaagcg gcaggtgttc atggcttcgg 1099380
ctaactcggg atgctgcttg tcaatgtcgg ggtaacaaac cgcgtcgcgc aggctgccct 1099440
gtgccgtgta cgggcgttgc ggcaggaaga ggatgtcttg atgcggcgga cggctgactt 1099500
tgccgctgct gccgaacggc caaagccccg ccagcgcgcg caacagcgag gttttgccgc 1099560
aaccgctcgg gccgcgtatc agcagggaat cgccgttttt gaggtttatg ttgatgccgc 1099620
tcaacaggat ttcgccgttg tggcggaaca gagcgacgtt ttcctgtggg ggactgttag 1099680
tttttgcaca aggaacaaat agagtaaaaa aacgctgaaa tcttcggaag acgtggattt 1099740
cggcgttttt ttgtatccgg aaaagttacg ccagcttttt cacaaaaccg cgccggaatg 1099800
cgcggttttc tgtttaaagc tgacgagatt agggaatttt taaaactgtt ttaagaggtt 1099860
tttaaaatgg atttaatcaa tactccggcc ataccattca acacggccta tgatggcgat 1099920
gtcgtcttgg gcattgctca aatctatttc aaacggtgcg taacgtggat tttcagacgt 1099980
tacaagcagt ttgcccggta tacgttgcac acgtttgaca aagaggtcat tgcctatacg 1100040
caagacatat aggccgtcac gcgggtcagt ttcggcgtgg ttgatgagaa tggaatcctc 1100100
atgattgagc acgccctcca ttgaatcgcc tttaacggta attacagaca gtttttccgg 1100160
ctgtttggtc acatagttgt caatccaata tttccggaaa gccaagcaga ataaaggttc 1100220
ttcgccgaag actggtgcgc cataccctgc tgctgcggct acgttgtagc gcggcacgaa 1100280
tacaaactcg gacaggtcga caggattgcc catagtgtcg gtgattccat cagaatttct 1100340
acttacagag aatgctccgg cgttttccgg cctggcttta tcgagatacg gcaagccttt 1100400
tccggtcagc agccagttta aatcacaact gaatttttac taggtaatcg gctgttggga 1100460
tagctccctc tttccaaact ctattaaatc cagaagccga catttctatt ttgttataga 1100520
tgtcagatgg cttagcccca tgaggccaaa gaaatttgag cctatctaaa aaagtatcca 1100580
tagtaatcct aatttaactc atttaagcaa aacattaagc aaaaaaagaa actcttttgc 1100640
ttaaataaga ttactcaaat aatcaatatt ttgtaaaaat aattacgttt ttgagaaaat 1100700
attttagcaa aagagtttca tgaagctgtt ttgctaaatg taattcactc atttgctaaa 1100760
tgacggcggt taataaacct acttaattaa ggaattgcga gaatgaaaaa aagcaagaaa 1100820
gcatgactga ttggcatcgt gctgacattg tggctcgtct taaaaaggca ggctggtctg 1100880
tgagagcgtt atctatcgag gctaatttag caccaaatac attagggaaa gctttagatg 1100940
ctccttatct gaaaggcgaa agaatcattg cagcagcgat tggagtaccc gcagaagaaa 1101000
tctggccatc tcgttttgag aaacgaaacc ataagccaac cttcccaaga tctataaata 1101060
gataactgtt ttgctaaata gttccaaaag agtaccgcat ttaagcaaaa atagaaagcg 1101120
gaaaaaatga aaatatctgc atctgatatt gcgaaattag gaattccgag cctaccaact 1101180
gatagacaag ggattgaata ccatgccaag aaaaataatt ggcaacactg ttttgagcaa 1101240
aaaggaagag gccgtcctaa aaaactgtat gaaatcgctt ccctccctgc cgaaatccga 1101300
gcagccatca tgaaacggca gtcggacgag ctggcggaga agatgccgaa aatgctgccc 1101360
aaagtcagac cggggacggc gatgtcggct caagcactgg ctgaagcggc caagctgttg 1101420
aacgagaaac aacggtcggt ggcggatgcg cgatgtgcgg tggtagcggc ggtattgggg 1101480
attaaatacg aatacgattg ctctgccaag gctgcggtgg ctcagttttt gggcttgctg 1101540
gcagaaggta aattggacgc ggtcacgctt gggaacttgg aaaaggccaa tgaccgcagc 1101600
cggacggcga aggtcggcga acgtacttta gacggctgga tttctgctta tttgaaagcg 1101660
gaaaacgcga cggagcggtt ggttgctttg gctccgaaga cgacgaaggc ggtcaagccg 1101720
attgaaagtt acggatggtt gccgatgttt atgcagtttc acaatattcc gtccgcgcca 1101780
aagctggcgc acagctaccg ctggtttgtg cagtgggcgg aagcggaaaa tatgccggtc 1101840
aatgatgtgc ctaacttgag tatggtgcgg cgcgtttggg aaaagctccc gttgattatg 1101900
caggagcgcg gcaggaaaac gggggcggct tataaatcgc tgctgcctta tgtgaaacgt 1101960
gattgggggg ctttgaagcc gaacgatgtt tggatcggcg acggccatag ctttaaggca 1102020
aaggtggcgc atccggtaca tggcagacca tttaagccgg aagtgacggt gattattgat 1102080
ggttgtacgc ggtttgtggt cgggttttcg gtctctcttg ctgaaagttg tgtggcggta 1102140
tcggacgcta tgcgtatcgg ggtcaagcat tttggtttgc cgattatcta ttactcggat 1102200
aacggcggcg gccaaaccgg caagacgata gaccatgaaa tcacgggtat tacgtcccga 1102260
ccgggtatcc gccatgaaac gggtatcgcg ggcaacccgc aagggcgcgg catcattgag 1102320
cgatggtgga aagacaatct gattgagatg gcgcgccagt atgagacgtt tgcgggtgca 1102380
gggatggaca gcagcacgaa gaacctgatg taccgcaaga tggaaagtgc gtttaacgct 1102440

```
ttggaaaaag gcaaggattt gacggaggaa caacagaaat atttgaaaaa actgccgagc 1102500
tggtcgcgtt ttatagcgga tgtggtcaag tgtatcgacg aatacaacaa ccgcccgcac 1102560
ggcgagctgc cccgacatcc tgacggcggg cattatacgc ctaaggctta tcgggaaatg 1102620
aggctggaac aggacggtat cgcgccggat atgttgtcgg cgcaagagct ggcgacgatg 1102680
tttatgccgc aagaggtgcg aaaggtacag cgcggttggc tggatttgtt taacaactct 1102740
tatttctcaa ccgagctggc ggagtatcac aaagacgagg tacgggtcag ctacgatttg 1102800
agcgatgcgt cggcggtcaa tgtgtttgat atggacggca agtttattac taaggcgcag 1102860
gccaacggca atacccgcga ggctttcccg acggctcgta tcgaccaact ggcggaaaaa 1102920
cgtcgaaaag gcaaaataaa gcgggcggaa aatgcaatca agctcgcaaa cgcggaagtc 1102980
aatcctgctc tggaacaggc tgcggtttgg gacgagctgg gacatttggg cggaaacgac 1103040
atcgaggcgg agtatgcggt attgccgaaa acgggcacag acgattttgt gttgtttgag 1103100
gcggatagat aaaggaaaac atgatggaca aacagcaaaa tgcagcgttt tcggccgagc 1103160
ttgttgaaaa attgaaactc aagcgagctc ttgggcggat tcaacgagct caagcaaaga 1103220
ttcaaggtgt tcccgctgaa cggaatcagg ctcaaacgtt tttgcctgcg cttgaaggaa 1103280
actgcgaacc tgctcaatcg aagtcggctc ttgacgggta atccgctgga gcagccagga 1103340
aagtacgaaa gaatcggcaa gtgacctgtc ttccaagtct tgaacggcga cttccagcat 1103400
gatcaggcgt ttttctaaat cgggaaactc tttcatttca gacggccttt aaaggttgtt 1103460
taaaactcaa ggatattaaa aatgaaacaa attaatcaag cattgcaaca aaaactggtt 1103520
gaatttaaag aaaaatcagg catgaaccaa acccaactgg cacgcggtat cggtacttcg 1103580
ccggcatcca tcagtatgta tctgaacggc acttatgcgg aaaaaggcgg caattatgaa 1103640
accatcgagc cgaaaatcga ggcgtttttg gagatgcagg acagtaaagc gcaacgcgaa 1103700
gagctggtgt tgggttttgt atcgactaag acgacccgcc gtattgcaga agtgatgcgc 1103760
gatgcgcacg aaggcggcga aacagtggtg atctacggtc aggcgggatt gggcaagact 1103820
caggcggtca aaaactactg cgagaaaaac cctgcggcca tcttgattga ggctaatccg 1103880
agctttacgg ctttggtctt gatgcgcaag ttggcgactg cggcgaaggt atcggcgatg 1103940
ggcagcctga atgatttgtt tgagtctgta tctgaccgcc tgcgcgattc gggccgtctg 1104000
attgtggtcg atgaagcgga aaacctgcct ttacgcgccc ttgaaattgt acgccgtctg 1104060
cacgacgaga ctggctgcgg cttggtgttg agcggtatgc cccgactggt ggccaacctg 1104120
cgcggtaagc atggcgaact ggtacagctt tacagccgcg tgtctgttgc gctgaatttg 1104180
ggcgaatctt tgccggatga cgaactcttt gagattgcga aagcggcttt gcctgatgcg 1104240
gtcaagcatc tgctccctga tagtgtacaa gcgttgatta cggtcatcgg gtttaatgaa 1104300
acgctggagc tggtgcgcct gatgggcggt acgacttatc ctttgcggca gggttatacg 1104360
aaaaacagtc aatcccgtgt tgcatacttg gaagagatta tcggcagtga ggcggccggt 1104420
cggctggtgg aggcaatggc tccgtgcaat ctgtttatac cccgttgcga gacggccttg 1104480
tatgagttgc gaaaccgtaa aatccgcagt cagtttgacc ggcagacggc aggcggtacc 1104540
cctgcttatg aggccgttaa cgatttggcc ttggcacacc gcctaagcga ccgccatgtg 1104600
tggcgaattt taaagcaggc ggataaggaa gcggagcagg agaatttgtt ttagaatgga 1104660
atgccatgca gatgtatggc attttatttt ggagaaaaat atgaaaaagt tttattttgt 1104720
gctgctggcg ttgggtttgg cagcgtgtgg gcaagaacaa tcgcagaaag ctgatgcgga 1104780
gcagtatttt tttgccaata aatatcaatt tgcagatgag aaacaggctt tttattttga 1104840
acgcgccgcc cgtttccgtg tattgcaaca aggccttggc ggggattttg agaggttttt 1104900
aaaaggagaa atacctaatc aagaaaatct tgcaaagtat cgtgaaaata ttactcaagc 1104960
agtcgcttat tatgcggaca cgaatggaga tgatgaccca taccgcgtct gcaaacaggc 1105020
tgcgcaagat gcagaaatcc tgatgaagag tatggtaaca agcggtggag gcggtacaac 1105080
tgatttagat aaggaaagtt atcaaaatta ccgaaaatca atgcaagaat gccgtaaaac 1105140
aataacggaa gctgaagcca atttgccgaa aaaataaaat aaacgattct aaggccgtct 1105200
gaacaacagg cggctttttt gttgcctact gacactgttt cgcccgctgc aaaagccatg 1105260
ccgtttgaaa atgtaagcct ctgaaagtgc attttaatct gattttgagg gaggctttaa 1105320
tgagcaaaat tatttgtctg actgccggac acagtaacac cgacccgggc gcagtcaacg 1105380
gaagcgaccg tgaggcggac ttggcgcagg atatgcgcaa cattgtggct tcaatcctgc 1105440
gtaacgatta cggcctgacc gttaaaaccg acggcacggg caaaggcaat atgccgctgc 1105500
gcgatgcggt caagctgatt cgcggctcgg atgtggcgat tgagttccac accaatgcgg 1105560
cggcgaacaa aacggcgaca ggcatcgaag ccttgtccac gccgaaaaat aaacgctggt 1105620
gtcaggtgct gggcaaagcc gttgccaaga aaaccggctg gaaactgcgc ggcgaagacg 1105680
gctttaagcc ggataacgca gggcaacatt cgcgcctggc ttatgcgcag gcaggcggca 1105740
ttgtgtttga gccttttttc atcagcaacg acactgattt ggccttgttt aagacgacca 1105800
aatggggcat ctgccgcgcg attgcggacg cgattgcgat ggaattggga gcggcgaagg 1105860
tatgaaaaag tctttgattg ctttatgtgt tgcccattgt gcaaagttga aaaacgattt 1105920
tggcgtacca ccgttacctg aaatcaaaat cacgccaagc cctgttcggg taggctcttt 1105980
gaaacaacat ccgagcctgc gcttgggtaa atcaggcgtg gcggctgcta aacgtgcggc 1106040
gcgcaaacgc aagaatcgtc gttaatcatg ggacaggttg cgttttacga aaagatgatt 1106100
```

gggctgtggt cggccaaaag ccgtgaggca agcgaacagg cggacttggc tgcgtttgaa 1106160
tttgcggagg gcgaactggc caattatcgg gaaatgctga aacggcacct gcaaaccaaa 1106220
agtgtggaat agcaatgcgt attttggata tttttaaaaa cccggcgaca ggcaatgtgt 1106280
cgcactcgaa actgtgggca aacgttgcct gcgcggctgg gacgtttaag tttgtgatgt 1106340
tgcccgatcc gtcggcggaa atttgggcgg tgtatttggg cattgtcggc ggctatgcgg 1106400
tggcgcgttc atttgtcagc gtgaagcgtc aggaggtcga gaatgaatct cgtgaaactg 1106460
ctggcgaata actggcaacc gattgccatt atcgcgcttg tcggcacggg cttggctgtg 1106520
tcgcaccatc aaggctacaa gtcggcattt gcgaagcagc aggcggtcat cgacaagatg 1106580
gagcgcgaca aggcgcaagc cctgctgttg tcggctcaaa actatgcgcg cgaactggaa 1106640
ctggcacgcg cggaagctaa aaaatatgaa gtcaaggcgc acgctgtcgg catggctttg 1106700
gcgaaaaaac aggcggaagt cagccgtctg aaaacggaaa ataaaaagga aatcgaaaat 1106760
gtccttactc aagaccgtaa aaatgcaagc ggcggttgca ttgacggctt tggctctcac 1106820
ggcctgcagc tctacaaccg cgccctcggc tacggaaatt aaggttgtcg aaaaggcagt 1106880
catgccgacc ccgcctgctg cattgatggt cgcgccggta cgcccgaatc cgccgaaaga 1106940
cggcaagacg gccacgctgt tggaacacgc cgctgagttt ggtggctatg tggcggagtt 1107000
ggaaaatcaa aatcaggctt ggcgcgactg ggcgggcaat cactcccgca aagtcggaaa 1107060
ctgacaaaaa agcccgcgta gggcgcgggc tgagggtgaa agcggatttt atacctcttt 1107120
tacaggggta gcggcggtag tgcttttcag caaatcgact gcgtgctgac agttttgctt 1107180
gctggtgtag ccttcgccct gagcgatgat ttcatggttg gctgctttca aacgccaacg 1107240
gtattcgcct tttgcgtctt tatagatttc aaaatacata aggtttctcc tatgaatgag 1107300
tacacgtttt cttaccgctt taacggcaag tcctggtcat tgagcatttg ggcggacaac 1107360
cctgaagaag ccagggcgaa atttcgggct gcacgagaaa atgcgcacta tgacggcgaa 1107420
gttgtagcaa aggtttatac atttgtaaat atttcgtggg ttaagaaatt gtacaagcgg 1107480
acaaaatatt taatgggtat caaagaatga cctaccgtga attagttgaa cgtcagttgg 1107540
ctgtgcgcca tgccgatttg gaattgggct taagccgcgc acgcgagcaa gagccgtttg 1107600
tcattcatgt ttccgatctg ttggataagg caggcattga gtacgcggta cgcatggata 1107660
aggattttca gacgacgttt caccttgaat atccaattac gaactatgac acctttaaac 1107720
gtgcggtttg gcaaactttg gggcgtattt actgtgtttg taatgatggt gatggactgg 1107780
agattgccag caatcgccct gacggttacg ccgtccgtat cgtattcggc gatgtgccgg 1107840
tttaaagggg ttttaaatgg actttgaatt tggtttcaga accctgtggc cgattgcgac 1107900
ggcggcattt tggttttggg tcaacggcat ttcaggccgc ctgaaagagg cggacaagcg 1107960
tatcgacgac cttaaagagg agttgcacgc ggtcaagctc tcttatcaca ccaaggcgga 1108020
cgccaaggca gacagcacta atattgcggc ggccttggag cgaattgaaa acaagttaga 1108080
aaaagtaaac gaaaaactgg acaggaaagc agacaaatca tgagcgaccc gattttggat 1108140
gccttggcgc gtattgaaaa caagactgat caaacgctga aaaatcagaa ggaaatgcag 1108200
gcggaaattg cgcaaattcg ccaagacacg aaacgcacgg ccattacatt cggcgcactg 1108260
ggcggcggcg tgattacggt cggctgggaa ttgcttaaag cgaaaatggg actgtaatta 1108320
tggctcaccc gcaagaaatc cgtgaaaagt tacgccggct ctatgtgagc ggcgagcaaa 1108380
ctttggaaac ggcggccttg atgtgcgaaa tcccgcagac cactgcgcgt gcgtggaaac 1108440
gtgcggataa ggaaaaaggc gacgactggg ataagatgcg cgccgcttac actttggccg 1108500
gtggcggtat tgaggatttg agccgtgcga tgttggccgg ttttatggtg cagtacaaca 1108560
gcacgatgac gatgctgcag gattcgagta ccgaagattt gccaccatcc gaccgcgcca 1108620
agctgttggc cagcctggcc gatgcgttta cgaaaaccgt atcggccaat gcgcgtgtga 1108680
tgccggaaac gtcaaaactg gcgacggctt tggaattgat tgagttcttg atggcgtttg 1108740
tacaagaaaa acatcccaaa catttgcctg cctttgtgga ggtattggag ccgtttgggg 1108800
tggaagtgga gaagaagttt ggttagaggc caacaaattt ttttaaaaga gtaatgaggg 1108860
tggcggcagg gatagcatta attgcatttt ccgtaagcgt acttaatgca gtgtccttga 1108920
ttttccctaa ttcttttttc aaccagcttt tttctgaatc agaaatctct gcttggtcta 1108980
tttttgccgc aattaaagcc tgaatagtgt cgctgtgtag tttgactgtg acaacaccaa 1109040
gaatggcgga taggccgcca tcatcggtaa ggaagtctat gcccttatga ttaattttgc 1109100
agtcaaaatt tttatgaagt gaatctatcg aagtaatttc aattaaacca gattcttcta 1109160
agtaataaat attttttaaa aaatattgga attcatctga ttgtaaagtt gctaggtgtt 1109220
gactttgaat ttcacaacca agagtcaaag ccaagcccaa tccttgttta tcaacaggga 1109280
tgttagaagt aataggtaaa gaactactag ggaaaagaga gttatacacc ttggttgctt 1109340
ttaggcagtt cgggtaatta tcacttaaaa ctcgtaagat tttttcctga atacctctat 1109400
ttaaccagtt cataaattat tcctcatgaa aacaaaagaa ttcctcaaat cccttgccga 1109460
actggccgcc agtttgcgcc aagtcatcga agcggaagtg gacggcttcg atgcgtcgcc 1109520
caaggctatt gctgcacgcc gtgccaaggt gtttgacccg gtaggcggtt acgagtattt 1109580
cgtgaatacc tacttccccc attatatccg ctcgcctgag aaatccgaac tgcatgcgtt 1109640
tttattcagc cgtctgccgg agattatccg ctcccccaaa ggggaaaatg aggcggtggg 1109700
tgcgccgcgt ggagagggta agtcgacgaa ggttactcag ttgtttacgc tgtggtgtat 1109760

tgtgaccggc caaaaacatt atgctgttat tgtgatggac agtatcgacc aggcatatcc 1109820
gatgctggaa gccatcaagg cggaacttga atttaacccg cgcttgaaaa ccgactttcc 1109880
ggaagtatgc gggcagggcc gtgtatggca ggccggtacg attgtgacgg ccaatgacgt 1109940
taaagtccaa gtggccggta gcggtaaaaa gctgcgcggt ttgcgtcacg gcccttaccg 1110000
tcctgactta actgttttgg acgatattga gaatgacgag caagtccgca accccgaaca 1110060
gcgcgacaag ctcaatgcgt ggctgactaa gaccgtattg cctctgggcg gtgtcggtca 1110120
gaaatacgat gtgatttata tcggcacgat tttgcattac gacagcgtac ttaaccgcac 1110180
tttgaataac ccgttttggc acggtattaa gtttaaggcg atgaaacgct ggcctgaccg 1110240
catggatttg tgggacaggt gggaggaact tttccgaaac gacggcgaga cggtggccga 1110300
ggcgttttat caggcaaaca aagacgagat ggagcgcggc gcggtcactt cttgggcggc 1110360
gcgtggcgta ctcgcgctga tgaaaatccg tgcgcgtgac ggccatgcga cgtttgattc 1110420
agaatatcaa aacgatccgg tcagtggcga agatgcgccg tttgccaagt cgatgaagtt 1110480
ttggaacgac ctgccgtccg atttggtgta tttcggtgcg ctcgacccgt cactcggaaa 1110540
ggccggggcg agccgtgacc cgtccgcgat tatcattggc ggttatcaac gtgtaaccgg 1110600
caaactgtat gtcgtggagg ctcagattaa aaaacgtctc cctgatttga ttattgagga 1110660
cgttattcga ttgcaccgtc aatatcgttg caaactgtgg tttgttgaga ctgttcaatt 1110720
tcaggaattt ctgaaagacg agctggtcaa gcgcagcgcg gcgcgtggaa tacctgtccc 1110780
agcgcgggcg gtcaaaccgg tatcggacaa gctgttgcgg atcgagactt tacagcctca 1110840
catggcgaac ggtttgattc tgttgaatga gagccaacaa acgctgatac agcagttccg 1110900
ccattttcca aaggctgatc atgatgatgg tcctgatgcc gtgcatatgc tctggtcggg 1110960
ggcggtggcc aattgtgtgc cgatagaatg gcaaagccct accgataacg attttgatga 1111020
cgagataaaa agtaaatgga gccgataatg gcaaaaaaga acaataaaac taaaatccaa 1111080
aagcccgaag ctgcattgca gacggacgtg gctcaaatta cggcgaccgg tcgggttatc 1111140
gccgagcatc cgtccaattt tattacgccg caaaagatgc gggccctctt cgaggacgca 1111200
gaaagcggcg acatccgcgc ccaacacgag cttttcgcgg acattgagga gcgcgacagc 1111260
gacatcgcgg caaatatggg gacgcgcaaa cgcgcgctgc tgacgctcaa ctggcgcgtc 1111320
gccccgccgc gaaatgcgac gcccgaagaa gaaaagctgt ccgaccaagc ctacgaaatg 1111380
atggacagcc tgcctaccct cgaagacctg attatggatt tgatggacgc ggtagggcac 1111440
ggattttctg cgttggaggt cgagtgggta ttttcagacg gcctttacct accccgaaac 1111500
tttatccacc gcccgcaaag ctggttcaaa tgggacaaag acaacgggct gctgctgcgt 1111560
acccgcgaaa atccggaagg cgaagcgttg tggccgctgg gctgggtcgt tcatacccaa 1111620
aaatcgcgca gcgtccagca ggcgcgcaac gggcttttcc gcacgctttc ctggctgtat 1111680
atgttcaaac actacgccgt ccacgatttt gccgagtttt tggagctgta cggcatgccc 1111740
atccgtatcg gcaaatacgg cgcgggcgca accaaagagg aaaaaaacac cctgcttcga 1111800
gcggtggcgg aaatcggtca caacgcggca ggcatcatgc cagaaggtat ggaaatagag 1111860
ctccacaacg cggcaaacgg tacgacggca accagcaatc cgtttttgca gatggccgac 1111920
tggtgcgaaa aatcggcggc gcggctgatt ttggggcaaa cgctgaccag cggtgcggac 1111980
ggaaaatcca gcaccaacgc gctgggcaat atccacaacg aggtacgccg cgatttgctg 1112040
gtgtcggacg caaaacaggt ggcgcaaacc atcacaagcc aaatcatcgg accgttcctg 1112100
caaatcaact atccccatgc cgacccaaac cgcgtgccga aatttgaatt tgacacgcgc 1112160
gagccgaaag acatcgcggt ctttgccgac gctatcccga aactggtgga tgtcggcgta 1112220
caaatccccg aaagctgggt gcgcgacaaa ctggtcattc cagatgtgca ggagggtgag 1112280
gctgtgttgg tgcggcaggt accggacaat ccggtaaaca gaactgcatt ggcggcttta 1112340
tccgcccaca ccgtaccatc taaggctacg ggcaggcatc aggaaatatt ggacggcgcg 1112400
ttggatgacg cgctggttga gcccgatttc aattctcagc tcaacccgat ggtgcgtcag 1112460
gcggttgccg cacttaatgc ttgcaacagc tacgaggagg cagatgccgc actgaatgcg 1112520
ctttatccga atttggacaa cgcgaaactg cgtacctata tgcagcaggc cttgtttatc 1112580
agcgatattt tgggacaaga ccatgcccgc gcctgatttg ggatttgcct taagtctgcc 1112640
gccaaaaaag gcaatcgagt ggctggaaag taaaaaggtt acggcggaga gctaccgcaa 1112700
tctgacagcc tccgaaattg ccaaagtcta tacgattgcc cgcatgaccg acttggatat 1112760
gctcaacgac atcaaaactt cgatggttga atcggcaaaa agtggacagt cgtttgacga 1112820
ttggcgaaaa ggtatcttga atctgctcag caacaagggc tggctgcatc cgaacgggca 1112880
taacggtaag gatatcatcg acccagccac cggcgaggta ttcggttcgc cgcggaggtt 1112940
ggagacgatt taccgtacca atatgcaaac tgcctacaac gccggtcaat atcaaggata 1113000
tatggcaaat attgatgcac gaccttattg gatgtatgac gcggtaggcg acagccgcac 1113060
ccgtccggcg cattcggcaa tagacgggct ggtgtaccgc tacgacgacc cgttttgggc 1113120
aacgttttac ccgcccaacg gctacaactg ccgctgctcg gtcatcgcgc tgtcggagcg 1113180
ggatgtggaa cgccaggggc ggattgttgg gcaaagcacg gcggacaatc tggtcgagac 1113240
ccataaaatc tacaacaaaa aaggcgatac ttatctgacc cttgcctata aagcaccgga 1113300
tggcagtctg tacacgaccg atcgaggatt tgattacaac gccggacgaa tgaactaccg 1113360
ccccgattta gacaagtacg accgtgcgtt ggcgcatcaa tttgccaaag cggaaatggg 1113420

tggtgcggat tttaaaacca gctttaaaca gcttgaaaaa gagttttatg aagtcaagca 1113480
acgtttggat attgatggca agcccgataa agagcagaaa atcaaaatcc gaaatgcgct 1113540
atcaagacag cttaaatttg ctgcgggtgt attgagcaag gaaacgcaag aattggcagg 1113600
tatgacacga gcgacggtgt ggctgtctga tgatacgttg gttaaacagg tagacagccg 1113660
tgaggggcag aatttcgatg actcctacta tgcttttttg ccggatatgc tgcaaaaccc 1113720
tgaacatgtc atccgcgaca atcgtgaatt gattttcaca gctcgctata aaggctcggc 1113780
attgtgggca gttttaaaat atattaagga ggtggatgag atttatctac agtcgtaccg 1113840
aatcagtaac gacaaagaga ttgccaaatt tatggcgaag aagaaagtat tgaaatagac 1113900
gttgggcaag gctcgaaatc acttgcacac gctctcggac gccctaacgg gcaggctgcg 1113960
gtatcgaggt tatcaccgct tttccaacgt ctgtacggaa taataccatg attgatgtca 1114020
aaatagacaa tatctttgtc gtcctaaacc aaatcgagcg gcttggcaac gggatcgaaa 1114080
accgctacct gctgatgcgc cgactgtccg aaaccatgca cacggcggtc aagctcaatt 1114140
tccgctacgc aggccgtccg aaatggttgg gctaaaatac cgcgacggca agccgctttc 1114200
ggattcgggt cgtctgaaag acagtttttc cacactgtca gacaacgata cagcccttgt 1114260
cggtacgaat atcgtctatg ccgccatcca caacttcggc ggtatggcgg ggcgcaaccg 1114320
caaagttcgg attccgcaac gggaattttt gacgctgacg gacgacgaca aacaggcttt 1114380
gatggacgat gtgcaggatt atttttcggg tctgataccg tgaatttata aaaccctcaa 1114440
aaacgcgctt tttagcgcgt ttttttatgc gggtaataca aacccctgcc caagatataa 1114500
aaatcaatcc tagacgcttc taaaaagccc ctgaaaacga ttaattgtgt atcgcgcgga 1114560
caggttttaa aaaaatggcg ggagggtttg aagcacgcct actctttgtt gtttttttcaa 1114620
ataggcaaaa tgaccgtatt gagagaggta cacatgtcca aaaatgcaca aaaaaaccta 1114680
cttgccgtgt gcagtttcga ggtgcagcca aaagacgggc gaatccaact gctgccatat 1114740
ggcgaatttc gcgcagtaga cggtcgtccg actgatgtcc ctgcgtggta tctgaccgaa 1114800
gaaaacggtc atgatgtcgc gttgttggcc aacagctcgc gcaatcagtt ggttgtcgat 1114860
tatgaacacc agacgctcta caaagagaaa aacggacaac ctgcacctgc cgccggttgg 1114920
atgcgttggc tggagttcac gcctaaaggc atgtttgccg aagtggagtg gacggacaag 1114980
gcggctgcgg caattgccgc aaaagagtat cgctacatct ctgctgtgtt ttcctatgac 1115040
acaaagggat atgtaagcaa aatttttcac gccgcgctga caaatttccc cgcgttggac 1115100
ggtatggacg aggtgctggc ggcagcgtcg gcgcaaattt taaaaccgga aacggagcaa 1115160
aaccctatga aagagttgtt acagcaactg ttcgacctgc ctgatgcggg cgaagaagaa 1115220
ctgaaggcgg cattgtccgc gctcgtggaa gccaagccga aagacgtggc attgtctgcc 1115280
gacgtgttcg cgcagctggc ggaaaaagac agccgcatcg cggcattgac ggcgcaaacc 1115340
gccaagcctg atttgactaa atacgcgcct atctcagtgg ttcaagagct gcaaagcaaa 1115400
gtcgccgcgc tgactgccaa gcaggaagca gacaaaggca acgaattgat taccgccgcg 1115460
ctgacttcag gcaaattgct gcctgctcag aaggagtggg caaaaggcgt attgaaacag 1115520
ccgggcggct tggcattttt gaccggcttt attgaaaacg cccagccggt cgctgcactg 1115580
gcaggctcgc aaacgggcgg caaagcaccc gacgaacgcg tcgccgcact gactgcggaa 1115640
gaggcagccg cagcaaaaat gctgggcatg tccggcgaag aatttgtaaa aatcaaagaa 1115700
agcgaaggta agtaatggac aaatcagcga ttttgaccgc aatcacggca gcattccgca 1115760
aagaatttca aacggccttg gattcggatt tcaagtgcaa cactaaggta ccagtggttg 1115820
gaacagattc aagaataaaa cacttggcgt ttcgtagcca agtgtttttc ttggtcggtg 1115880
gttcaactca tcttgaaccc tgcgtatctc ccgatcactg atgttacgga aatcggtttg 1115940
tttggggaag tattgccgga tgagtccgtt ggtgttctca ttcagccctt tctcccaaga 1116000
atggtaagga cgacaaaaat aagtctccgc tttcaatgct ttggttattt tggtgtgttg 1116060
gtagaactct ttgccgttat ccatggtaat ggtgtgcacc ctgtctttat gtgcctttaa 1116120
tgccctaaca gctgcccggg cagtgtcttc ggctttgagg ctatccaatt tgcagatgat 1116180
ggtgtagcgg gtaacgcgtt cgaccaaggt caataatgcg cttttctgtc ctttgccgac 1116240
aatggtgtcg gcttcccaat cgccgatacg ggatttctgg tcgacgatag cgggtcggtt 1116300
ttctatgccg acacggttgg gtactttgcc tctggtccat gtgctgccgt agcgtttgcg 1116360
gtagggtttg ctgcatattc tgagatgttg ccacaacgtg ctgccgttgc ttttgtcttg 1116420
gcgaaggtag cggtaaatgg tgctgtggtg gagcgtgatc tggtggtgtt tgcacaggta 1116480
ggcgcatact tgttcgggac tgagtttgcg gcggataagg gggtcgatgt gctgaatcag 1116540
ctgcgaatcg agcttatagg gttgtcgctt acgctgtttg atagtccggc tttgccgctg 1116600
ggctttttcg gcgctgtatt gctgcccttg ggtgcggtgc cgtctgattt cgcggctgat 1116660
ggtgcttttg tggcggttca gctgtttggc gatttcggtg acggtgcagt ggcgggacag 1116720
gtattggatg tggtatcgtt cgccttgggt cagttgcgtg tagctcatgg caatctttct 1116780
tgcaggaaag gccgtatgct accgcatact ggcctttttc tgttagggaa agttgcactt 1116840
caaatgcgaa tccgccgccg tctgaaacaa ggagtcatca tggcaaaaac caacaacaaa 1116900
ccagaaaccg ccgaaaccgc cgccccgtcg tttgaagaca tcaaagccga attggacgcc 1116960
gtgcaggcag agcttgccgc cgcccgaaac gatgtcgaaa tgctgaccac agccttggaa 1117020
aaagccgaag acgacaaaaa ggcactgtcc gccgaacttg ccgaactcaa agtgcagcat 1117080

acgcaacgtg ccgccgacgc tttggcggac agccgcgatg tgatgctcgt cagtaccggc 1117140
gcagacggca aagaattttg gcgcggcggc ctgctgtttg acggcggctg gcgcgaagtg 1117200
aagcgcgccg aagtcggcga agcggtgtgg aaggcaatct gcgccgagcc tatgctgcaa 1117260
cgcaaggcgg tcgagtaatg gcatacgcga cggttgagga tatggttgcg cgtttcggtg 1117320
agctggaagt cttgcagctc accgaccgca acaacgaggg gctgattgac cgcgaggtcg 1117380
cacaaaccgc gctggtggac gccactgccg aaatcgacgc gtatctgggg cggttcagac 1117440
gacctttga ggatctgccg cccatcttgg tgcgcctttg ctgcgacatt gcccgctacc 1117500
gtctgacggc ggctcagggc gtgttgatta ccgacgaaat ccgcaaccgc tacaaaatcg 1117560
acgtgctcga cctgctgcgt gctatggcca aaggcgaagt gcagctgggc gtggatgata 1117620
gcggcgaaga agtggccgcg ggcgaagacg gtattgtgtt tgtaaacggt aaaaataagg 1117680
tgttcgggcg tgatcactga tattgagcaa gcgataacag accgtctgaa acggggcttg 1117740
ggtcgcatgg tgcgcacggt taaaagctac aacggcgagg ccgacgattt ggcggggcaa 1117800
atccatacgc tgcctgcggt ttgggtaacg tatggcggca gcaaagttga gcctgccagc 1117860
accggcggcg tatgcggacg ttatcaggat accgccgaat ttgtggtgat ggtggcggcc 1117920
cgcaatctgc gcaacgagca ggcgcagcgg caaggcggca tcgacagccg cgaaatcggc 1117980
agcaacgatt taatccgcgc tgttcgccgc ctgcttgacg gccagcggct cggttttgcc 1118040
gatagccgcg gcttggtgcc caaagcggtg cgcgcgattg ccaatcatgt gctggtgcaa 1118100
aacgccgcag taagcatata tgcggttgag tatgccatcc gctttaacac ctgcgggttg 1118160
gaaaatgacc gctaccccga acgcaccgac aatcccgacg accccaacca tatctttacc 1118220
aagtatcagg gtacattgag cgagccgtgg cctgatttcg aggggttgga cggcaaaatt 1118280
tacgacccgc aatccgccga tgaaatacct gtaaacctaa cccttaagga taagcaatga 1118340
gcaaaatcaa agtaacggcg gcagatggcc tgcgtgtgcc gaccgaacac aacccgcacg 1118400
aatatatcgg ccaagagccg gtggaggtgg acggcaacag cctgtattac cgccgcatga 1118460
ttgatgacgg cgatttggtg gtggttgagg atgccgcccc aaataccaaa acccgcaata 1118520
ctaagggaga gtaatgatgc cccatattga ttttgacacg attccgggca gcatccgcgt 1118580
gcccgggcag tatattgaat ttaacacccg caatgccgta caaggtttgc cgcaaaatcc 1118640
gcaaaaggta ttgatggttg cacccatgct gaccgcgggc atacagcccg ccttagagcc 1118700
ggtgcaacta tttagcgatg ccgaggcggc cgatttgttc ggacaaggct cgctggcgca 1118760
tttgatggtg cgccaagcat ttgccaacaa cccttatttg gatttgaccg ttatcggtat 1118820
tgccgaccac agcgcaggcg tgcaggcaac cgcaaccgtt accctttccg gcacggccac 1118880
cgcgccgggc gtggtggaaa tcacgattgg cggcaagcag gtaagcacgg ccgttaacac 1118940
cggcgagacc gccgccacag tggcagaccg tctgaaaacc gccatcactg ccgccgatgt 1119000
aaccgttacc gcatccggca gcggcgcagc cgttacgctg acggccaaac acaaaggcga 1119060
gatcggcaac gagagcggct taaccgtgag caccggcaat accggcctaa cttatcaagc 1119120
caatgccttt accggcggtg ccaaaaatgc ggacattgcc acggccttgt ccaaagtggc 1119180
gggcaagcat tatcacatta tttgcagccc gtttagcgat gacgccaacg ccaaagcctt 1119240
gagcaaccat attaccaacg tatccaacgc catcgagcag cgcggctgta tcggcgtatt 1119300
gggtatgagt gcggccttga gcacggccac caccgctacc ggcgaaatca acgacggccg 1119360
catgacctgt gcttggtaca aggtgcggt agagccaaac ggcatcatcg ccgcaggtta 1119420
tgcggcggtg ttggcctttg aagaagaccc tgccaagccg ctgaacacgc tggaaatcaa 1119480

agggctggcc gttacacctg atgcgcaatg gccgctgttt gcagaatgca acaatgcgct 1119540
gtacaacggc ttgaccccgc tcacagtggt caacaaccgc gtgcagatta tgcgtgccgt 1119600
atccacctat accaagtcgg ccaacaacac cgacgacccg gcactactcg acattaccac 1119660
catccgcacg ctggattatg tgcgccgcag cgttaaagag cgcattgccc tgcgttttcc 1119720
gcgcgacaaa ttgagcgacc gcctgctgcc caaggttaag agcgagattt tggacgtgct 1119780
gattaagctc gaccaagccg aaatcatcga aaacgccgag gccaacaaag gcaagctggt 1119840
ggtggcgcgt gcgcaaaacg accccaaccg tgttaatgcc attatccccg ccgatgtggt 1119900
caacggcctg cacgtctttg ccgggcgcat tgatttgatt ttgtaaccct tttcagacgg 1119960
cgtttaaaac aggtttaaag gccgtctgaa accttaaaaa aggataaagc atgagcgacg 1120020
ctacctatgc cgacgcggtg attatggaga tgaacggccg cgatatcgag attgtgagca 1120080
tcaagccgca aaccactaca ggccgcaagc cggtcaaaac gatgaaccgc aacggccgag 1120140
tcaacggtta ttgtgacggc gtaaccgaac acaaattaag cgttaccgcc gccattccga 1120200
tcgacggtac ggaaatcgac tgggacaaca tcaccaaggc gaaaatcacg atttacccca 1120260
tcaacgacga agatcgccgc acttcctacc tcgactgctt taccgtcgat accggcgagc 1120320
aatatgaagt cgataacgag gcacgcatcg acattgagat gattgctttg cacaaaatca 1120380
aggagtaatg cgtgattacc gtcaaactga cccacgggct gacctacaat ggcaaagtcg 1120440
tatctgaatt acgcctcaag ccactgaccg tcggcggcga actggcggcg ttcgccctga 1120500
ttgatgactt gcccgagctg cccgaaaacg ccacaaaagc cgaactgctg caacgcgacg 1120560
tcctagagac gctgacctac tggtcgcagc agattgaagc ccaaggtatc ccatccgaca 1120620
tcctgacggc gcagtggctg atggaaaacc tctctaccga agactaccat accgtgatgg 1120680

cggctcagga ggatttgcgc ctaaaaccgt ccgccgctac ggcgagcccc gatgcgccgt 1120740
cggcggcgga gcagtaaaac gcagctacct gacggcacac aaaagctacc gtcaggcggt 1120800
catcctgatg gcaaggcggg gataggggcg gacgcagtgc gcgatatgtg ccacgccgag 1120860
ctgtcggcat ggtttgaaga catcctgtcg agcctgggca taaaaacgcc gaaggaagag 1120920
ggagtgattg tgtcgaaacg gctgagaaag ccggagggaa aataaaaagg tcgtctgagt 1120980
ttcagacgac cttttttta tttgacggtt agggttgttt tctgccgatt attgcaatgg 1121040
tgtttgtttc tttttcaaaa acaacgctat ataaaatacc atctgccgga acgtcttttt 1121100
gcgctgttgc tcctgtctga ttggattctt tgacgacttc ggttaaagct gtaaaaagtt 1121160
gttttcctgc ttccgattcg cccaatttgt ctgtggtagt taaagacaga actgcctgaa 1121220
tactgtgtaa tccatttatg gcattattga ctagattttg tcctttctct ccgtcggttt 1121280
ttaaaatgta gcctactgag atagctctaa ttttttgctg ctcgtccagt ttgagcgcaa 1121340
tcgccccaaa atccattacc aaagtaaggt cataaccaca atcggtcttg acaatatttt 1121400
tattatggat ttttgttgag acgttctgag ttttaaatgc ttgctccaaa ttgatgagat 1121460
attgttcaac tgtgatgtcc attggggcgg tacaatcagc agcctggata ctttggacag 1121520
gttcttcggc ttgtgccgtt tttgattgtt ggttgcaggc tgataaaaca attgaaacac 1121580
aaattgcgga aatcaatttt ttcatattca taaaatatcc ctttgaataa aatggttatc 1121640
attctagtat tataacgcaa caaacaaata aagcacgaaa acggggttga agcccatacc 1121700
gcctccctta aacagccttt aaacgataat tgaccttgag ttaatacgtt taaaggctgc 1121760
tttttatggc aaacgggaac atgaaactgt cgttggtgtt aaccgcccga gatgacggag 1121820
cgagacggct actggctgat actcaacgac aattagatcg taccgcgaaa tcgcgggcgc 1121880
aacttgaacg gcaaagccat acttatgcgt tgaccggcat ccgctcagaa aaacagattc 1121940
aacgcgaaat catgctgaca caggctgcgt ttaaccgttt ggcgcgcagc ggcaaggcat 1122000
cacaaaatga tttggcacgg gcggcggtcg ctacgcgtaa ccgaattcgc gagctgaacg 1122060
cggaactgaa acagggcacg ggatttgcgg acaagatggg aaaaatcgga agattcggtg 1122120
cagctgcggt ggctggtggc gcggcagcgt atacggtgct taagcctgct atggacaaca 1122180
gaaagcagct tgatgagaac atcaaccgcg tgtccagaca ggcatttatt gaggataaca 1122240
gtaaatcggc agcgtggatt gcaactgaag gtgcgcaaca gatcaaggat ttggcacttg 1122300
aacttgtcga gaaaaatggc gggacccacg ataaggcttt ggatttaatc agcggcatga 1122360
tgaccaccgg tctgaatttt gcccaaacca agaatgaagc gcaggcggca tatgcttttg 1122420
cacttgcctc agaaggcagt ggcgaggata cggcaaaact gattaaaacc ctgaaagatg 1122480
gcggcatgag cggtaaagac ctgcaactcg ggcttgagca cgtcttgcaa tcgggtttag 1122540
acggcacttt cgaggtgcgg gatatggttc gggagctgcc gagcctgctc tctgccgcgc 1122600
aacaggcagg gatgaatggt gtcggcggtt tggactacct gctctcactc ttacaatctg 1122660
cggcgaataa atcgggcagt cctgccgaag cggcgactaa tgtgcaaaat cttttgagta 1122720
aaactctgtc gcctgacacg ataggtcgtc tgaagaagat ggcaaatccg aatgacccga 1122780
agaaaggtgt cgattggata ggctcggttg tgcaaggcaa gcaaaacggc gaaaacgcag 1122840
tgcaggtgtt gtcccgtctt gccgatgcca tgctagtaaa ggataagcaa taccaagatt 1122900
ataagaaacg cgcggctgca ggcgataaga cggcggcgga gcaggcaaat atgcttaagg 1122960
gcgcgctttt ggcgcaactg ctgcctgatt tgcaggcaaa acaaggtttg ctggctgcaa 1123020
cggatatgac gcaaatccgt gaatatatgg cttcgttggc tggcgtaacg ttggataacg 1123080
gaaaaattgc taagaacaac gaggcgcgaa tgttgtcggc agcggcgcaa caagagcaac 1123140
aggaatcgct ggcaatgttg cgggaaagtc tgacgggaac attggtggat atggaaacct 1123200
cgtttaaaaa gctggcagcg gaatacccta atgccactct agccctgcaa gcattgacga 1123260
cggcggcaac agcggcgtct gccgcaatgt tattaaccgc cggtggcggt aaaggtgcag 1123320
gctttctgaa agatgtaggt agtaaagcgt tgggatgggg taaggcttcc gcaggcggcg 1123380
tggcagcagg tgccacagcg gcaggcggta agttgctgtc atggggaaaa tctgccggta 1123440
gcgggctcat gaataatcca gcgttagtta aacgggcggg tttgttaggt atgttgctgt 1123500
attccgagtc tttgggtgac ggcacattgc caaagggttt gcgtggtacc aagacaactc 1123560
ctgaaatgat taatcgtctg aaaaacaacg gtatccgatt tgaacctgcg ccgaagcggg 1123620
aacaggcgcg gggtggtgtc cctcagtatt tggctgctcc gtcagcgcag cctaccgata 1123680
agatgttgtc tccgttgttt tcaactcaga cggcggcgta tcaggcagcc attcagcagc 1123740
agacggcggc gtatcaggca gcattggcgc aggatacggc tgcagttaca acaggtttgg 1123800
cacaagtgca aagtgcgatg gcgtcggcaa gtcagaccat caataccaat gtgagcctga 1123860
atatcgacgg acgtgttatc gcgaatgagg tatcgcggta tcaagtggcc atgttcggcc 1123920
gtggagcggg tcaataatga gcggatggca taccttattg caggacgcat cttacaaggg 1123980
cgtcggcttt gatattgagg tggtggacga gagcaacggc aaggcattgg ccgagcatgc 1124040
gcggccgttt gtgcagggta tcgaccttga agacatgggc atgaccgggc ggcaggtgca 1124100
gattaatgcg gtgttttggg gcaagggcta tgcaggccgt ctgaaaaagc tgctggatgc 1124160
gctggagcag ccgggcggcg gcgtgctggt gcaccctgtt tgggggcgga tgcacaacat 1124220
gattgcggca tcatggagtt accgacatga ggccgattat gtggattatg cgggcatcga 1124280
tattactttc cgcgaggcgg ccgaagcgca ggaaatcttt gtttttgaaa acgccttttt 1124340

```
ggtcgagctt gaggcgttga ttgctaatat cgacacctac cgcgaggcgg ctatcggctt 1124400
tgttgatgcg gtgttggcgg tggatgcggg cgtatcagct ttatggggca gcgcgctggg 1124460
catttggagt gcggcatcgg gtacgtttgg cgcggtgcgc cgtttgtttg atttggacaa 1124520
aattgccttt cccgatcggg gcggatacag tgcagcggcg tttaaaaacg gctcggccaa 1124580
gctgtttgcg gatatatcgg tcatggtaga tactggcata cgccgtgagg cgggtttggc 1124640
cgataatgcc atgcaccatg ccggttggtc gccgcgacag cggtttgacg gggctgcggc 1124700
tgttgccgac cgcgccgccg ctatccctga taatttgctg accggccgct tttcagacgg 1124760
cctgcaaaac cgcctgaacc ggttaaccgc caaacaggtg cagccggtag cgcaggcggt 1124820
gcgcctgtta tccacgtcat cgctgttgtc ggtggcaacg gcattaatcg aggcgcatgg 1124880
cgaagagatg accgcgcccg atttgattga ggttaaccgc gccatgcgcc gccgtatgca 1124940
ggccgagatt gccgccttgc gggcggtgca gacggctgct gccgagtctg gtgggctgac 1125000
ggccaacgcc gtgtataccg aggcttacca aacggcagaa tccctgcgcg cggcggcagg 1125060
ccgtctgaat gcgttggttg cggcggtcat caaccaaaag ccgccgctga ttgtgcgcca 1125120
agccccaatc gacggtacga tacaccaaat cgcccacgag ttttacggcg atatagcccg 1125180
cgcagcagag ctggtgcggc tcaatcccca tatccaccac cccgcgttta tcaagcgcgg 1125240
cactttggtc aacagctatg caaaataatt catacggcta tgccgtgtcg gtgcgcgtgg 1125300
gcggtaaaga gcaccgccac tgggagcgct acgacatcga cagcgacttt ttaatccctg 1125360
ccgacagctt cgattttgtc atcggcaggt tgggaccgga ggcggccata cccgatttaa 1125420
gcggagagag ctgcgaggta gtgatagacg ggcaaatcgt gatgacgggc atcatcggca 1125480
gccagcgcca cggcaaaagc aagggcagcc gcgagttgag cttgagcggg cgtgatttgg 1125540
ccggtttttt ggtggattgc tccgcgccgc agctcaatgt aaagggcatg acggtattgg 1125600
atgcagccaa aaagctggcc gcgccgtggc cgcagattaa agcggtggtg cttaaggccg 1125660
aaaacaaccc cgctttgggc aaaatcgaca tcgagccggg cgaaaccgta tggcaggcat 1125720
taacccatat tgccaactcg gtcgggctgc atccgtggct ggagccggac ggcacgttgg 1125780
tggtgggcgg tgcggattac agcagcccgc cggtggcgac attgtgttgg agccgcaccg 1125840
acagccgctg caatatcgag cgcatggaca ttgagtggga taccgacaac cgcttttccg 1125900
aggttacttt tttggcgcaa tcgcacggcc gcagcggcga cagcgccaaa cacgatttaa 1125960
agtgggtgta caaagacccg acgatgacgc tgcaccgccc taaaacggtg gtggtgtccg 1126020
atgccgacaa tttggccgca ttgcaaaagc aggctaaaaa gcagctggcc gactggcggc 1126080
tggagggatt tacactcacg ataaccgtgg gcggccataa aacccgcgac ggcgtattgt 1126140
ggcaacctgg cctgcgtgtg catgtgatcg acgacgagca cggtatcgat gcggtgtttt 1126200
ttctgatggg gcggcggttt atgctatccc gcatggatgg tacgcaaacc gagctgcggc 1126260
tcaaagagga cggtatttgg acacccgacg cttaccccaa aaaggccgag gcggcgcgca 1126320
agcgcaaagg caaacgcaaa ggcgtgagcc ataagggcaa aaaaggcggc aaaaaacaag 1126380
cagaaacggc ggtgtttgaa tgagtttgag taaattggcg aaaaaaacgg cacaaactgc 1126440
taaaaatatc ggcgaaaccc tgcgcgcggc ctttcgggga aaaatcacgc tggtggtgtc 1126500
gtccgagccg atacagcgcg tgcagttgag cggcttggcc gacgaaaccc tgcaagacct 1126560
tgaacatttg caggaatacg gctttgccag ccatccgccc gacggcagcg aagcggtagt 1126620
gataccgctg ggcggcaata cttcgcacgg tgtgattgtg tgcagccagc acggcagcta 1126680
ccgcatcaaa aaccttaagc ccggcgagac ggcgattttt aatcatgagg gtgcaaaaat 1126740
cgtgattaag caaggcaaaa tcattgaggc cgattgcgac gtgtaccggg ttaactgcaa 1126800
acaatacgag gttaatgcgg ccacggatgc caaatttaac gctccgttgg tggagaccag 1126860
tgcagtgttg acggcgcaag gccaaatcaa cggcaacggc ggcatggccg tcgagggcgg 1126920
cgacggagcc acctttagcg gcgatgttaa ccaaacgggc ggcagcttta acaccgacgg 1126980
cgacgtggtg gccggcaata tatcgttgcg ccagcacccg cataccgaca gcatcggcgg 1127040
caaaacctta ccggcggaac cggcatagac aagcagacct ttggcagcct tcgggctgct 1127100
ttttttgtgc gtgtgggatt gaagcccgtg tactccgtga ggccgtctga aaacggcaaa 1127160
atgccaacat ggacaaagag ctaaacccca gcatcggcga ctataccggc cgcaccgtcg 1127220
atacgctgca aaatgccgtg tatatccgct tgatgacacc gttgggcagc tggtgggcgg 1127280
ataaaacgct cggctcgctg ctgcatttgt tgcagcgcga aaaagacctg caacgggtca 1127340
gcctgttggc cgagcaatat gccgatgagg cactgcaacc gattgttaag agcgggcgtg 1127400
ccgacaagat taccgtgcgc gcagagcagc cgcacgacgg ccgcctgatc ctgcatatcc 1127460
ggatggatac ggcggcgggc gggtttgatt accgccacga agtgcccgtg atttaaagag 1127520
gttttaaacg tgtttgaaac gccgacattt gagcaaatcc gcgagcgtat cctgcgcgat 1127580
accaaaagcc tgtggccgga tgccgatatc agccccgaca gcgaccatta tgtgcacgcc 1127640
agccgtttgg ccagctgcgc cgaagggcaa tatgcgcatc aaagctggat tgtgcggcag 1127700
attttccctg ataccgccga ccgcgagtat ttggagcggc atgcctccat gcgcggcttg 1127760
agccgccgca atcctaccac ggccagcggc acgctgaccg taagcggtat tgcgcaatcc 1127820
atgctttcag acgacctgca agtgcgtatc ggccagcgtt tttaccgcac taccgcccgc 1127880
gccgttatcg gcagcggcgg cacggcggaa ataccggcaa tcgccgacga gccgggcgcg 1127940
gccgccaatg tggcgacggc gaggcgcaac tgatggccgc ccccgccggt gtggccaccg 1128000
```

aatgccgcct taccgtacaa ggcggcaccg accgagaaag cgatgcctca ctgctggcgc 1128060
gtctgttgga aatcatccgc cgaccgcccg caggcggcaa ccgttacgac tataaaaact 1128120
gggcgttgag tgttgacggc gtaaccagcg catatgttta tccgctgcgc cgcggcttgg 1128180
gtacggtgga tattgccatt acctccgccg acggtgtgtc gtcggaagaa actgtgcgcc 1128240
gcgtacaggc ttatatcgac gagatgcgcc cggtaacggc aaaaaatgcg ctggtactca 1128300
agccaaccgt aacggcggtg cctgttaccg tgcaagtcaa gctcgacggt atcgacttgg 1128360
acgaggccaa gcgccgcata cggacggccc taaaagaata tttcgacacc ctgatccccg 1128420
gcgacggcct gactgtgtcg caaatcgagg ctgctatcag caatgtggat ggtgtgatcg 1128480
accgccgtct gactgcgccg acggccaacc gtgccgccga tacggttaac cgcatcgagt 1128540
ggtttaaagc gggcgcgatt aatgtaacgg agatgccgtc atgagctatc aagacatctt 1128600
gcggggcctg ttgccccccg tgtcgtatgc ccgcaatgcc ccgcgtgtgc gggcgcaggc 1128660
agaaatagac ggcgcagcgc tggatgcggt ggcggaatcg gctcaaagcg ttgccgatgc 1128720
cgtcgacccg cgcagcgccg gccaaatgct ggccgattgg gagcgcgtat taggtttgga 1128780
cggtacgggc aaaaaccgcc agcaccgtgt gttggccgtc atggccaagc taaacgaaac 1128840
aggcggcttg agtattcctt attttgtgcg tttggccgag gcggcgggct atcaaatcca 1128900
aatcgacgaa ccgcagccgt tccgcgccgg tgtaaaccgc gccggcgacc gtcttgcgcc 1128960
gcaggaaatc atgtgggtgt ggcacgttaa cgtgcgcggc ggcaacaacc gcattacccg 1129020
attccgcgcc ggtatctcgg cggcgggcga caggctgacc gattacagcg atgccgtgat 1129080
cgagagcctg ttcaaccgcc tcaagcccgc ccacaccgct atccgattta cctaccgcta 1129140
aaggacgatt tatgcacccc atcgaaaccc ccgataagac cttccacgac ggcgacggcg 1129200
tgtccgaatt gggcaccatc ctgcccgcgt ggtggctcaa ccaagtgcaa tccgagctgc 1129260
tggccgtgct gactgcggcc ggtatccagc cggataagtc gcagcctaat caattactgg 1129320
cagcactaaa taggctggct gtagttatca ccggcgacca aaccgtcaac ggccaaaaaa 1129380
cctttaccgc ccaaacccaa ttccaaagcg gcatccattt atccgccaac cagacgaact 1129440
ggaacggcgg ccacaaagcc tacatcggcg cggatgccga caacgcccac atcgtcttcg 1129500
gcgacgacac cctccgcctg cacggcgcaa acaaccgcat ttcctacaac aaccacgaca 1129560
tcttccacaa agccaacaaa ccgcgttttg ccgaagacat ccaaggcaaa ccgaacacac 1129620
tgtccggata cggcatcggc aatttcaaag tcgaaacatt ccggggcgat ttgaacaccc 1129680
tcaaaacaga cggcatctat tccctgccga cggcggtcgg cagctccaac ctgcccgttg 1129740
aaaacaccgc ctgccatatc caagtcatcg ccggcacgaa acacggctgg tgcaggcagt 1129800
tgggttatcc cgcctacacg tccgacgtgt acgaacgcca ccaaacgagc agcgcaaacg 1129860
acaactggtc cgcatggaaa aaactcaatt cggacggcat ccccgtcggc gcgatcgtat 1129920
cctttcccaa agccgtacaa aaccccgcag gctatctcaa agccaacggc acgacctttg 1129980
cacaaaacac cttccccgac ctttaccgcg ccttgggcaa cagcaaccgc ctgcccgatt 1130040
taagccgtac cgacatcggc atcaccgcgt ggtttccgtc cgaccaaatc ccgaccggct 1130100
ggctggcgtt tgacgacatc cgcacgcgcg taaccgaaac cgcttatccc gagctgtacc 1130160
gtctgctgac cggaaaatac ggcagcatcc aaaacgtccc gcaggcggaa gaccgcttta 1130220
tccgcaacgc gggcaacagc ttggcagtcg gaacgaagca ggaagacgaa atcaaacggc 1130280
acgtccacaa agtattttca cactggacaa accacacaga cgcggcagcc ctcggttacg 1130340
aagaccgcaa cgaaaggcag agaagcgcgc tcgtatcgac ttggacggac gaaaatttaa 1130400
acgacaacgg ctttttaacc ccgcgctcgg acagcaaaat ggcgacaggc ggcgacgaaa 1130460
accgccccaa agccctggtt ttaaaactgt gcatcaaagc cgccgacacc ttgggcgaag 1130520
ccgtgttttg gataaagtcc cacggcgaaa ccatcaacgc aggcgcgctg gacgcgggca 1130580
cgctggcgca aggtttgcaa gacaaagccg accgagacca cacccacacc gccgcccaaa 1130640
tccaagggct ggacgaaaaa atcagcaccg ccgttgccgc gcaattcaca cgccaaacca 1130700
tcggcggcgt ggatattgtc agattccccg acggcacaat gatacagacc ggcagctaca 1130760
ggttcacacg aagcggcggc cccatcgaaa acgaagtcgt cttccccgtc gcctttgccg 1130820
acggcaacgt caaatgcttc gtatccgaac gccattcgga acgcgttacc ggcgatcgaa 1130880
ggcaacacaa ctggctgttt atccgcgcaa aaaaccacgc cgccgccatt atcaccaact 1130940
ggtacgaagg cagttgcgac tggatggcca tcggcaaagc cgcctcggga aacgccgcca 1131000
gctccccgat aggccccgaa atacctgaaa ccaacgaaga accgcaaaga gagagtggaa 1131060
gaacatcaac cggaccccga aaccgccgcc gccgagacgg cttgctcgag gcactgcaag 1131120
actagcgggc tgtagagatg gctgtagaga cgggctgtag agatggctgt agagacgggt 1131180
tgtagagacg ggttgtagag acgggttgta gagatgggtt gtagagatgg ctgtagagat 1131240
ggctgtagag atggctgtag agatggctgt agagatgggc tgtagagatg ggttgtagag 1131300
atgggctgta gagatgggct gtagagatgg gctgtagaga tgggctgtag agatgggctg 1131360
tagagatggg ctgtagagat gggctgtaga gatgggctgt agagatgggc tgtagagatg 1131420
gctgtagaga tggctgtaga gatggctgta gagatgggtt gtagagatgg gttgtagaga 1131480
cgagttgtag agatgggctt cagcccgccg atccaagcaa tccgaccgaa accggccgcg 1131540
ccgccaatcc cccgaaacct atgccccgcc aatcctgcca ctcttcgtca ttcccgccgc 1131600
tttcgtcatt cccgcgaaag cgggaatcca gaccccccga cgcaacagga atctatcgga 1131660

aaaaccgaaa cccccgccac cgtcactccc gcgaaagcgg gaatccagcc cccaaacgcg 1131720
gcaggaatct atcagaaaaa acagaaaccc ccgccgccgt cattcccgcg caggcgggaa 1131780
tccagacccc aaacgcggca ggaatctatc ggaaaaaaca gaaatccccg ccgccgtcat 1131840
tcccgcgcag gcgggaatcc agaccccaaa cgcggcagga atctatcgga aaaaaccgac 1131900
cccccgccac cgtcattccc gcgcaggcgg gaatccagac cccaaacgcg gcaggaatct 1131960
atcggaaacg gctgaaaccg aacggactgg attcccgcct gcgcgggaat gacggcggca 1132020
ggggtttcgg gattcccgcc ttcgcgggaa tgacggaaag tggcgggaat aacgaaaggc 1132080
gggaatgacc gcgcaaaaag ccgctgcccc cttcggacgg caccggcaac aaaaaaccgc 1132140
acggccgaaa ccgcgcggga aaggatagtc gggcgcgccc gataagcagc ggccgccccc 1132200
gttatttcaa ttgggcgata tattggcgca aaacctcgtt gatgcgcgtc tgccagccct 1132260
tgccgccggc gcggaatttt tcgaccacat cggcggacag gcgtatggta acgagttgtt 1132320
tcggggtttt gcctgtgttt cgtttttgca ttacgccctt ttcttccaat tgtttttgat 1132380
gggaaaaaag cacctgtgcc aagtcttcgg gcagggcttc ggcaatgggg cgggcaagcg 1132440
caaaatcttc ggcggcaagt tcccgcactt cgccgtcagc gtttgttaag gattgacgtt 1132500
gcatattttt taacctctct tttattcgct ttgcgaaaac tgatgacacg gatgccgtct 1132560
tttatcggcg taaaacagac aatgtgcagg cgttgcgtat cgcctagata agcagcggca 1132620
acataacgcg gttcggggta atcaaagcgg acatcgggca caataacggc cgttgtccag 1132680
cgtatttgcc cgactgattc aaagggcaaa ttccgctctt cgatattgcg ttgatttttt 1132740
tcggagtcaa attcaatctt cattgcagct tgcagcgtat tttgtcgtta cattataagc 1132800
ggcaaaaaac caaaatgtaa atacaaaaaa ggaaacccca aaatgaccat ctatttcaaa 1132860
aacggctttt acgacgacac attgggcggc atccccgaag gcgcggttgc cgtccgcgcc 1132920
gaagaatacg ccgccctttt ggcaggacag gcgcagggcg ggcagattgc cgcagattcc 1132980
gacggccgcc ccgttttaac cccgccgcgc ccgtccgatt accacgaatg ggacggcaaa 1133040
aaatggaaaa tcagcaaagc cgccgccgcc gcccgtttcg ccaaacaaaa aaccgccttg 1133100
gcattccgcc tcgcggaaaa ggcggacgaa ctcaaaaaca gcctcttggc gggctatccc 1133160
caagtggaaa tcgacagctt ttacaggcag gaaaaagaag ccctcgcgcg gcaggcggac 1133220
aacaacgccc cgaccccgat gctggcgcaa atcgccgccg caaggggcgt ggaattggac 1133280
gttttgattg aaaaagttat cgaaaaatcc gcccgcctgg ctgttgccgc cggcgcgatt 1133340
atcggaaagc gtcagcagct cgaagacaaa ttgaacacca tcgaaaccgc gcccggattg 1133400
gacgcgctgg aaaaggaaat cgaagaatgg acgctaaaca tcggctgaaa aaatacgttt 1133460
accacctgtt ggtagccatc gaccaactgt tcaacgccct aaccggcggc gcggcggacg 1133520
aaaccctctc aagccgcacc tatcgccgcg cgcggctcgc ccaaaagccc aaaacccgct 1133580
ggaagatttt atataccctg atcaacggcg tgtttttcga ccgccaccac tgccggcagg 1133640
cgtatatcag cgaactgaaa ggcaggcagc acgacgcgcg gttcaaccaa agccgcgccg 1133700
ccggggaaaa ggggacgcga tgaactactt cggcatggta gagtttctgc gcctgatggc 1133760
aatggtgcgg ccgccatttg tcttcttctc cggcacccgc agcgaactgc ctgcttatct 1133820
tgacctcgtg gcagaactgc gcctgaccgg atgggagcgt tttgcaggca gccaaacctt 1133880
gactgtgagc agcacatcaa cagcaattca agctacgacg accacctgat ttataagttc 1133940
tgaccgcaag tagcgtacta cttttaaagg cataagataa tccccgttta acagaccatt 1134000
aaacggggat aaatttgtgc aaaagctaat acaatttcct acgcttcggc ggtgcaaaag 1134060
ctgccgccaa ttcgtgcaaa agctgccgcc gccttacatt ccagtgcaat gccgtctgaa 1134120
acttcgctaa tctcgggttg ccgcgcgctg tgttgttctt cggtactcag caaaaagccg 1134180
tacagacgct ccaatcgggc gcggtaggcg gtgaatttgt tgtagaacat ccggaagaaa 1134240
gaaagcgcgt tttgcagtcg cgcgaaggct tggacggtct gctggatgtc gccgattttg 1134300
atttgtccgg caaacaggcg cggcgcttgc aaaataatgg ggaagagctt gatgccgttg 1134360
gtgaacatat cattaaagcc gctcaggcag acgctttgtc gcgcaatacg ccagcggttg 1134420
cggataatgg ctttaaaacg gtcagaaagc tggtcgtgtt cgtgtttttc gccgctgtaa 1134480
aacgccacgc tttcggcgtg gtcgcgcacg cggatgaggg aataacggta gtcgccgttg 1134540
agtttttcgt tttcataatt gtaacgaatc aaagggttgc ctatccacat ggcgataaag 1134600
gtcgccaaaa tcacaaatat atagacaaac caaacgatgc cgtgcggaat gtcgaagccg 1134660
aacacggtca ggatgccagc caagccccgc aaaacaacgg caaattccag agaagtaacg 1134720
accgaattga ccatgccgcg cacaaattcg atggtcgaag cgataaattc ctgcgcgtcc 1134780
tgttggatac gctggtcgat gttgtccggc gcgtggcggc gcatttgcag gcggtagtag 1134840
tttttgtcgg caagccagcg tgctgtcagc acttcgttga gccgctccga ccatttaatc 1134900
gccaagcctt gatcgaggaa gtcgttgacg acgttgttaa acgcccgtat cagcaccacg 1134960
cccgcgttca ttgcagcaaa catccaaaat gccgaagcat ttcaaatcct gcatcgagtc 1135020
gtaaagccct ttggacataa aggtactcaa cacattcagc cgcatttcgg ttaacaccag 1135080
cgtaatcatc gccgtaatca gcagcaagac tttgaccgcg cttttcggtg tcagacaaag 1135140
ccaaagcggt gtggaataaa gctcggtttg ccatttctgc atgggaaatt tcttacggta 1135200
tcaatgccgt ctgaaaaaga cgggtacagt tgattttttg atgaagtttg gggaagtttt 1135260
gccggtcagg gtacattgcg tgttaattta tagtggatta aatttaaacc agtacagcgt 1135320

tgcttcgcct tagctcaaag agaacgattc tctaaggtgc tgaagcacca agtgaatcgg 1135380
ttccgtacta tttgtactgt ctgcggcttc gtcgccttgt cctgattttt gttaatccac 1135440
tataccatac aaccacgccg gaattaagtt taaatttgaa taaaaggttc gggttctgca 1135500
aaatacagaa cccgaacctt gttcggatat tgaaaccggc tgcccgattt tgggcggtgc 1135560
ggcttgcaag tatcaagatt cgcatatgcc gtctgaagct cggagaggtt cagacggcat 1135620
atgcttattt gggctgctct tcaacgaatc tcggaccttt caagatgccg ttgtgagaat 1135680
agggcgacag caggttgtat gcggcggttt tggaaacctg ataaccgcgg tcggtcaggc 1135740
tgttggcaat ctgattgacc actgcgctga ccaaagcccc caacaggccg ctgttgctgt 1135800
tgttgctgcc ttcgcggatg ctggccgaac ccgaccacaa ctcttttccg ttgcgggaat 1135860
cgaccagccg tgctttggcg gatacggtcg tcacgctgtc taaaatttga tatgaagtgc 1135920
cgtattcggt aaccgtaatg tacaaaaccg catcattgcc gaaaatctga tgcagttttt 1135980
ccggccggac ggcgtgaata tcggcggcat tggtcaagcc gttttgtttg aaggtttcct 1136040
ccacgactgc ggcggggaag acgtaatagc cggcttcgga aagcggcgcg gcggtcgaag 1136100
ccagtacacc ccatgttccg ttgacatcgg gcgattcgtt cagcggcgga accaccaaaa 1136160
ttgaagccgg tttgctttcc ttgaatgacg tgtagtcgaa atcgggcgct tttgaactt 1136220
ggcaggcaga cagcgccaac acggcggcaa gccctaaaat caaaggtttc atcgcttgcc 1136280
tcctttaccg gttttcatca ggaagtccat aaatacgccc gattcgggaa acagcctttt 1136340
ctcttcttca aactggcgga acgcgccctc tttgtctccc gctatttagg tgacgcgtta 1136400
gaatactcaa gctatgcatc aagcttggta ccgagctcgg atccactagt aacggccgcc 1136460
agtgtgctgg aattcgccct tccgggctgg atgtgttcgg gaataccgtt tttcagatag 1136520
ccgcgtatgc ctgcccagcc ttggacgaaa cgggaaaaac gggcggtatc gctgccccaa 1136580
atgccccagc agaggcgaaa tacgagcagg aaaaggacga acagcccgac gcgcgtgtgc 1136640
cattgcagca tatcgccgcc ggctttcgcg ctataccaca taaagggcag ggacgcggca 1136700
agcagccagt ggaaaaggcg ggtggggagg tcccagactt tggttttgtt tttcataatc 1136760
ggtttccggc ggtagaatcg gtttgttttc gagccttatt ttaaccgatt ggaggggcaa 1136820
tgtttcccgt ttttcatctt tcaggcgaga gccgccgcca gatgcttcag acggcattgc 1136880
gttttcccca tgttttcaaa gcccgtgcgg aagattcgca caaagggact ttcggcacgc 1136940
tcgccgtagt cggcggatcg gcagggatga gcggcgcgcc cgtattggcg gcatcggcgg 1137000
caatgtatct cggctgcggc aaagtgtggg cgggtttcaa tcaggatacg ctacctttg 1137060
ccgttattgc cggttttccc gagattatgc tggatacggc ggacagtttg gccaaacgtc 1137120
aagatataaa cgcctgggtt gtcggttgtg gattgggtac aggtagggcg gcggtcggaa 1137180
cgcttgccgg aattttgacg gaacacacgg acaagcccgt cgttttggat gcggatgcgc 1137240
tgaacatatt atcaaccgat gccgaaaccc gaaatctggc gcgcgggtgt aaaaaacctga 1137300
ttttaacgcc acaccccgcc gaagccgcgc gcctgcttgg aacgacggtt gcgcaggttc 1137360
aggcggatcg gacggcggca gtgaggaaga taggggcaat tttcggcgca accgtggttt 1137420
taaaggggca caaaacattg gttgcctcac ccgatacgga aatctatgtc aacgaaagcg 1137480
gcaacgcggg attggcaacg gcgggcagtg gcgacgtatt gggcggcatc atcggcagtc 1137540
tgctcgcaca gggcgtgccg gtttttgaag ccgcctgcgc gggcgcgtgg ctgcacggcg 1137600
cggcggcgga tgtcataaaa gaatcggcag gcattgcggc agggctgttg gcaggggaaa 1137660
tcgctccggc ggcaaggtgg ctgcgcaacc ggataactaa aagtatgtaa gaagatatag 1137720
tggattaaca aaaaccagta catcgttgcc tcgccttagc tcaaagagaa cgattctcta 1137780
aggtgctgaa gcaccaagtg aatcggttcc gtactatttg tactgtctgc ggcttcgtcg 1137840
ccttgtcctg atttttgtta atccactata ccatacaacc acgccggaat taagtttaaa 1137900
tttgaataaa aggttcgggt tctgcaaaat acagaacccg aaccttgttc ggatattgaa 1137960
accggctgcc cgattttggg cggtgcggct tgcaagtatc aagattcgca tatgccgtct 1138020
gaagctcgga gaggttcaga cggcatatgc ttatttgggc tgctcttcaa cgaatctcgg 1138080
acctttcaag atgccgttgt gagaataggg cgacagcagg ttgtatgcgg cggttttgga 1138140
aacctgataa ccgcggtcgg tcaggctgtt ggcaatctga ttgaccactg cgctgaccaa 1138200
agcccccaac aggccgctgt tgctgttgtt gctgccttcg cggatgctgg ccgaacccga 1138260
ccacaactct tttccgttgc gggaatcgac cagccgtgct ttggcggata cggtcgtcac 1138320
gctgtctaaa atttgatatg aagtgccgta ttcggtaacc gtaatgtaca aaaccgcatc 1138380
attgccgaaa atctgatgca gtttttccgg ccggacggcg tgaatatcgg cggcattggt 1138440
caagccgttt tgtttgaagg tttcctccac gactgcggcg gggaagacgt aatagccggc 1138500
ttcggaaagc ggcgcggcgg tcgaagccag tacaccccat gttccgttga catcgggcga 1138560
ttcgttcagc ggcggaacca ccaaaattga agccggtttg ctttccttga atgacgtgta 1138620
gtcgaaatcg ggcgcttttt gaacttggca ggcagacagc gccaacacgg cggcaagccc 1138680
taaaatcaaa ggtttcatcg cttgcctcct ttaccggttt tcatcaggaa gtccataaat 1138740
acgcccgatt cgggaaacag ccttttctct tcttcaaact ggcggaacgc gccctctttg 1138800
tctcccgaac gggaaagcag cagtcccaga tgggcgtgcg cacccggggc ggcattcatt 1138860
tttttgttgc cggcttccac aaagtatttt tccatctttt cggtctgctt gcccaacgaa 1138920
gtgtcgtcgt ttttcaaacc ttcatagacg gtatcgggat agccgccgta ataatacagg 1138980

gatttttgcc cgttgccgcc gcaggcggtc agagccaaga ccgccgcaca cagcgacaaa 1139040
cggctcaagg ttttcggatt catcatttct ccttaacggt tgggttgcca tgcgccgttg 1139100
tcaacagcct gaaccaggct gttgacggct tcgcggattg ccaagtctaa aactttgccg 1139160
ttcaaagtcg catcgtagcc ggaagtgccg ccgaaaccga tgatttcacg gttggaaagt 1139220
gcgtattcgc ccgcgccctg tgcggaatag acgatttcgg aagtattgac gttgacgata 1139280
ttcagagcca cttttgcata ggcgatttgc gatttgccgc gacccaaaat gccgaagagc 1139340
tgatgatcgc cgacatctct gcgtccgaat tcggttacat cgccggtaac gacataatct 1139400
gcgcctttca ggttatgcgc tttgccggaa atgccggatt cctgttttaa tgcgttcaaa 1139460
ttggtgcggt tcagtacgtt gaagcggttg gtctgttgca ggtgcgttac tagaatggtt 1139520
tttgcctggc tgcccaaacg gtcttccccg tcggagaaaa tgccttttttg gaagctggag 1139580
cggttgtcga atgttccgac ggaaatcggg gtacgaacac cgtgatattg cgtattgtag 1139640
gaggcgactt tctctacctc gagactgcgt gaggattcgg tcgcacagcc ggtcagtgaa 1139700
acggcagcgg cggcaaggac aacggcggtg gaaacggttt tcataaaatt taccctaagg 1139760
tcaagttaag gaaataacgg gttgtcatta ttgtccttat gtaaatttaa gtcaaggtgt 1139820
ttgtctgtgc gggacggatg cgcgcggaag gtacggatat ttttcaaacg gaatcgcggc 1139880
gcgggcggga tgcctgcctt ttccggattt agaggtaaac gatttcgcca ctccgcccctt 1139940
tgctttcggc acttgcccac cagacaaatg cgggcagcac gtccccgtag cttttgcgtt 1140000
cgcttttggc ttcgcccgga tgggatttga tgcgttgcgg ggaattgatg gggccgggga 1140060
cgaggacgtt ggcgcgcagg ttgccgaagc gttcccattc gtcggcggcg actttgcaca 1140120
ggtagttcaa cgcggctttg gacgcgccga agccgcccca gtaggctttg ggtgtttcgc 1140180
cgtggctttc gccgacgaag atgacggacg cgtcgggcga ctgcttcagc agcgggaaca 1140240
gggcgcgggt cagccccata ggtgcgacgg tgttgatgcg gtattggttg acccattcgg 1140300
cgacggtttg gaaatccagc ggcgagaggg cgtaaaaata gccggcgcag tggacgatgc 1140360
cgtccagttt gccttgcgtg gcttcggcaa tggtggcggc gaaatgttca aattcttttt 1140420
cttccgcgct aataaggtca aagcagatgg cgaatggttc ggggtatccg gcttcgacaa 1140480
tcgcgtcata cactttttcc agttttttct gatgacgggc aaccaaaatc acggttgcgc 1140540
ctgccgccgc ataggctttg gcgacctgtt cgcccagacc ttgcgatgcg ccggttacta 1140600
agatggtttt gtcggacagt gtcgccatac tttttccttt ttggttgtcg gttaaggtat 1140660
tttagcgttt tgccgcacct tgtaaagcgt cccgatgtcg gccggcgttt tcagacggca 1140720
tcgctcaggc tttgcaggca ggcttccgcc gacgcgaaac ctgattgtac ggcggcttcg 1140780

agcgtggcgg ggtagtccgg gtggaggtag tcgccggcgg ggaagatgcg gtgccggtgc 1140840
aaccacgaca agtccggcgg cggggcatcg gctgcggttg tggcgcgttt ttcggtgatg 1140900
acgcgcacgg cttcgggttc gcccaaatgc ggaaggatgc gtttgaggtc ggcgtgggct 1140960
ttgtccgccc acgcccggtt tgcaaacgcg ccgacgcggt cggaaacgct gatgacggcg 1141020
gacacttcgt tttcaggcag tccgagcctg ccccggcaaa gcagccattg caccgtgccg 1141080
tcggcaaggc cggtcagcgg ggcgggcagg cggacgggtt cggcgtagcg cagatagacg 1141140
gtggtgatgg cgtggtagcg aaggttttga tatgccgtct gaacgtgttc gggcgtgcct 1141200
tcgggcagga gcgcggcggc gtggtagggc gcggtggcgg ggacggcggc atcgaaagct 1141260
tcgccgttga cgagcacttt cccgtccggg agggtgttca gacggcatac gcgcgtttcg 1141320
aggcggatgt ccgcgccgag ccgttgaaga tccgccaagg cgggttcggc gacgattgcg 1141380
cccaaatcct gcttgggtag gagatagtcg ctgccggatt ttttcgtcag cacgccgtcg 1141440
gacaaaacgt tgcacaacac gcgcaggctt gcggtttcca aaggcgtgtt gagcgcgccc 1141500
caaaccaagg gctgccaaaa ctgcatcacg gcggcacgcg gcacgttccg ctgtttcagc 1141560
cattgcgcca ctgtcgtgtc gggctgtccg aggcgtgcgg acttctgcaa atcggacata 1141620
tcggcaagca gtttggcttt gaatgcagtc ggtgcacgcc gggcaagcag cacgccgccc 1141680
aaaatatgca gcggcgcggg caggggggagg gcgcggaact gcaaaccgcc gtgcatatgc 1141740
cagtgcagcg gtacgcgcaa aaaggcggca cggggatccg aaccgatggt tttcatcagg 1141800
cgcaacacgc cccggtatgc gccgagcaaa atgtgctgcc cgttgtccaa aaaaccgaaa 1141860
ccgtcggtat ttccggccag tgtgcgcgcc ctgccgcccg cctgccggcc ggcttcaaac 1141920
agggtaacgt cggcgtgccg cgccaaggtg acggcggcgg acagtcctgc ccagcctgcg 1141980
ccgatgacgg cgattttcgg gcgcggatgc ggcgtgttca tcatttattc ctccaatggt 1142040
tttgcagccg tatctatttc cgtttccgaa aatgacggta gaaaaggata caggctaaaa 1142100
ataaaggcag cagaaagcgc gcatagggat accacggatg gtctgcatgc cagtatccgt 1142160
accgtccctg tggaacggta tcatagcggt aactgtcggg gaaaaagttg atccaaaccg 1142220
ttactgccag ccataaggct atatccctga ctgctttcat ttacttctgc tcctgtttaa 1142280
attcccagca attccatttc aaagcgcgaa cgccaacggg attgcgcggt tacgatgcag 1142340
actttcagac gatggttgaa accccgtccg gcggggctgt gggaatcggg tttgcctgac 1142400
gggcggcggg cggtatgcgc cttatgcccg ttccggcgtg ccggggcgcg gtttgaatcc 1142460
gaataaccag gttttcaggg caatgcgttt tttgcgcggc gaagggaggg cgattttgta 1142520
tttgaggacg ttttgtgcgc cgtctcggtc gatttcgttc aatagctcgt aataaaccgc 1142580

cgccatgacc agtccgactt tttgggcttt tttatcggca tcaggcagca gcgatacggc 1142640
ttcgcggtag gtttcgcggg cgcgtttgat ttggaacgcc atcaattcgg caaaattgcc 1142700
cgtcgggctg cattgcaaaa tcacgcttgc gggtacgtca aaccgccgca tttcctccat 1142760
cggcaggtaa atccgccccc tgcgcgcgtc ttcgccgaca tcgcggatga tgttggtcag 1142820
ttgcagcgca agtcccatct tgtcggcgta ttccagcgtt tggtcgtctg aaaaccccaa 1142880
aatccgcgca atcaggcagc cgaccacgcc tgcgacgcgg tggcaataca gtttcaattc 1142940
ttcaaaactg ccgtaacggg cttgaaccaa atccatctgc atcccgtcga ttaaggcttc 1143000
cagttcatat ttcggcagct tgaaggtttc cttaacttgc cgcaaggcct gattgacggg 1143060
gtgttccggc atcgcgccgc cgaatacctt gtccaaatcg ccgcgccacc agttcaatgt 1143120
cgcctgtgca acatcggggt tggaacattc gtcaaccaca tcgtccaatt cgcggcaaaa 1143180
agcatataaa accgttaccg catcccgttt ttcctgagtc aggaaacgga agcccgacaa 1143240
aaaactggag cggctttctt ctgctttttg gcggcaatag tcgagtcctt tcacgattta 1143300
tattcctaat gatgggcggg aaaggcggat tttatcggca tttggcggta gagggcaatt 1143360
tcggcggcac gacctaatcc ttagcggttt gctcaactat cggcgcaaat tctgttaaaa 1143420
tgccgctttc cttcctttac acaccgcacc gacaggcaga atttatggct cttttgcaga 1143480
tttcagaacc gggtatgtcc gccgccccgc accggcaccg tttggcggca ggcatcgatt 1143540
tgggtacgac caacagcttg gtcgccaccg tccgcagcgg cagtgccgcc tgcctgcccg 1143600
atgccgaagg gcgcgttacc ctgccttccg tcgtccgcta tctggaaaac ggcggcattg 1143660
aagtcggcaa aaccgccctg tccgcccaaa aaaccgaccc gctgaacacc gtcagctccg 1143720
ccaaacgcct tatcgggcgg acgcttgccg atctgcatca aaatacgcac tacctgcctt 1143780
accgtttcgg cgacaatcaa cgcgttatcg aactgcatac gcggcagggg gtgaaaacgc 1143840
ctgtcgaagt gtcggcggaa atcctcaaaa cccttaaatc gcgcgccgaa gaaaccttgg 1143900
gcggcgattt ggtcggcgtg gtgattaccg tccccgccta tttcgacgat gcccaacgcc 1143960
aggccaccaa agatgccgcg cgtctggcgg gtttgaacgt attgcgcctg ctcaacgaac 1144020
ccaccgccgc cgcaatcgcc tacgggctgg acaacgcctc ggaaggcacg tttgtcgtgt 1144080
acgacttagg gggcggcaca ttcgacgtat ccgtattgca actgaccaaa ggactgtttg 1144140
aagtcaaagc caccggcggc aacagcgcgt tgggcggcga cgatttcgac caccgcctgt 1144200
tctgccgcct gctcgaacaa aacggactct cccaactcaa cgaacaagac agccaactcc 1144260
tgctctcgct cgtccgcgcc gccaaagaac aattaaccac gcaaaccgaa gcgcgcattc 1144320
aggcgacgct ttcagacggc atggcaatcg acacaagcat cagtcgcgcc gagttccaca 1144380
acctgacgca gcatttggtg atgaaaacgc tcgaaccggt cacacaggcg ttgaaagatg 1144440
ccggtgtcgg taaaaacgaa gtcaaaggcg tgattatggt cggcggttcg acccgtatgc 1144500
tgcacgtcca acaggcagtc gccaccttct tcggacaaac cccgctgaac aacctcaacc 1144560
ccgacgaagt cgtcgcgctc ggcgccgcca tacaggcaaa cgtcctcgca ggcaacaaaa 1144620
ccgacggcga atggctgctg ctggacgtta cgcccttgtc gctcggtttg gaaacctacg 1144680
gcggcttggc ggaaaaaatc atcccgcgca attccaccat ccccaccgcg cgcgcgcagg 1144740
actttaccac cttcaaagac ggtcagaccg cgatgacgat acacgtcgta caaggcgaac 1144800
gcgaactggt tgccgactgc cgcagccttg ccaaattcac cctgcgcggc attccgccta 1144860
tggcggcggg tgcggcgcgt atccgcgtaa ccttccaaat cgatgcggat gggctgctgt 1144920
ccgtttccgc ccaagaacaa agcaccggcg tacaggcgca aatcgaagtc aaaccctcct 1144980
acggcttgga cgacgacacc atcacccaaa tgctcaaaga cagcatgagc aatgccgccg 1145040
aagatatggc ggcacgcgcc cgtgccgaag ccgtagtcga agccgaaagc ctgaccgatg 1145100
ccgtcaacgc cgccctcgag ttggacagcg atttgctgga tgccgaagaa ttgcaacaga 1145160
ttcggcaagg catcgccgat ttgcaaggcc gtctgaaaga cggaaaagcc gaagacatcc 1145220
gtgccgccgc cgccaaactc ggcagcatca ccgacaattt cgccgccaaa cgcatgaacc 1145280
gcaacatcca acgcgcgctg acaggccaga gtgtcgataa tatttgatac ttaaacggtt 1145340
tcagacggca tagaaataat ccgatgccgt ctgaaggctc gaaaacactt gaaaaacatc 1145400
gatatggaaa agtcaggcat tgtctatctg atgaaaaccg tcatcaaggg cgtgtataaa 1145460
atcggcattt cggatgtaag caattttgaa ggcagaatgc gccatttgga aaacaacggt 1145520
tatgcgaacg ttgccggatt ggaacgcatc ctcgccgtca aaaccgacaa ttacaaagaa 1145580
aaagaaaacc tgctccatga aattttcagc aaaagcagga taggcgatac cgaattgttc 1145640
gccgtggacg aaaaccttgt gaaacgtttg tttttatcgc ttcgcggcga aatcgtgttc 1145700
ccgaaaaacg aaacggcgga atcggaattt gaaaaaagcg tccacgaacg caggcaggaa 1145760
gggaatgccg ggtcaggccg caaacaactg cttgatttgg tacggcgcgg acaccgggaa 1145820
tacccttacg cgctgccccg gcttttggcg ggcgcggcat tctacaagcc gaaaaaatcg 1145880
aaaatccgcc tttttaaaga agcatatttc ggcaaaagcg gcacgaggct gaccgacgaa 1145940
attgcagacg gcatccatat ttacacctgt ttttcgcggg cggatttgga aaaagcctat 1146000
tccgaatatt tggaactttt caaatccgaa tcggatgccg aaggcagaaa gccgcagtaa 1146060
ggtgcaaaca gataccgtac acgttgagga gcagatatga tgggcgattc cgtcatttat 1146120
tatgtagaac aggcagacga accggtaaac cgtgccgacg aacgcgcccg taaaacattc 1146180
aaatattttt ggcgcgagct tttttgggaa cgccgccgca ttatttccgc cttggatttt 1146240

gccatggtca aagtcccttt tttccaagac ggcgaagacg gcgaaatttg cgaacatatg 1146300
tggatcgacg atatttattt tgacggtctt tatatttacg gtgtgctgaa caatgaaccc 1146360
ggcgaactga ccaatgtcga acaaggcgaa agcgtttgcg ttccggttga cgacatcagc 1146420
gactggatgt tcgtgtgcaa cggcatcccc tacggcggct ttaccataca ggcaatgcgc 1146480
gggcagatga cggaagagga gcgcaccgaa cacgatgccg catggggaat cgatttcggc 1146540
gatcccgggc agatattgct ggtgtatgaa gaaaaagaac atcccgaaaa tctggaagag 1146600
catccgatgt gccggaactg tattgacgat tttcggcaac agttgtccca aaactcggat 1146660
tatctgcggg aacaggacga agacggctat acgccgcttc atcatgaagc catcgcagga 1146720
aatgcactta tggttcaagc catgcttgaa tacggcgcaa atcctgcctc aacgacatcg 1146780
gaaggctata ccgccctcga ttttgcctgc ctgacgggct ggcaaaatgt tgccgacctg 1146840
ctcgaaccgc gacattaggc agacagtttt ccgaaaacga acacaaacac tttttacaga 1146900
aagacaataa aaatgcccaa aatcaccgta cttccacaca cgacattatg ccccgagggt 1146960
gcagtcatcg ataacgcacc cgaaggtaaa accgtccttg acgtgctgct cgaccatgat 1147020
atcgaagtcg atcacgcctg cgaaaaatcc tgcgcctgca caacctgcca cgtgattatc 1147080
cgcaaaggtt tcgacagcct agaagagccg accgaattgg aagaagacct gctcgatcag 1147140
gcttggggtt tggaagccga ttcgcgcctg agttgtcagg cggttgtcgc cggcgaggat 1147200
ttgattgtgg aaatccccaa atacaccatc aaccacgcgc gcgaagaaca ctgaaaacag 1147260
gccgtctgaa gccggcacgc ttcagacggc attgttgcgc ggataaggcg caatcgcccg 1147320
aaaacaggcg ttcgtacagg cggaactttc gattctatag tgaattaaca aaaatcagga 1147380
caaggcggcg agccgcagac agtacagata gtacggcaag gcgaggtaac gctgtaccgg 1147440
tttaaattta attcactata tattgatttt tatcggtttt ctgacggaat aatccagtgc 1147500
ggcatccgag gcggattact cggacgcgat gcaccggtat ttatcggttt tgcagccgga 1147560
aaaaccgccg gcgggttata gtggattaaa tttaaaccag tacagcgttg cctcgccttg 1147620
ccgtactatc tgtactgtct gcggctcgcc gccttgtcct gatttttgtt aatccactat 1147680
acttttaggg cgacggtcgg gcagtatgcc ggatagcgtt ccactctcgc ttctatattg 1147740
attttgattg gttttctgac ggaatgaccc gatgcggcat ccgggacggc ttgtgttttt 1147800
tcctgcccgc ctgccggatt ttcccatcct tgcgtgaaac cgaaagagac ggcggcgcgg 1147860
cggacaagct cgagatagcg tccttcaagc tccggacagg cggcggacac gctttctacc 1147920
gtaaccgtga aaccgccgcc cgacccggca aggcgttccg caatttgcgt gtagatgagc 1147980
caacgttggg cgcgcaacat tgccgaatcg caaccggcag ccgctttatc caaggcaatc 1148040
aggtcgcgcc ggttttggtg gtgaaggcaa accgttactc tggagaggat atgttgcccg 1148100
tccaatattt gatacagttt gcgtatcagc agaatcaggc ggtcaaactc ctccatctcc 1148160
gacagggaat caggatggtc ggaagcggta taaaccagtt tggacaattt tgcggcaaac 1148220
atattgttca tcaatcttcc ttgtcggttg aaaccccgct cttcggggcg gtagaatcag 1148280
atttgtttgg gagggacaac tcctcccaga tcaggacgac acataggctg gtgcttgatg 1148340
tgttgtccgg cgagttgaaa cattcagcaa tcctcaaggg gcggcagttt tgccgaaaca 1148400
tattctacac ggcttcaatg ccggacgata aaaggaaatt catatgaaat ggaccgacac 1148460
ccagcgcatc gccgaagaac tctatgacct gcacggcgaa accatcgatc ccagaaccgt 1148520
gcgctttacc caactgcgcg acctgattat ggcattgccc gaatttgacg acgaccccgc 1148580
ccgttgcggc gaacgcatcc tcgaagccgt gcagcaggca tggatagacg aggcggaata 1148640
agtttcggga atgccgtctg aaatgcggcg gtacgcggtt cgtgcttctg tttgcagcgg 1148700
gaatggtttt accagtctcc ttttttcagc ctgtccagtt ggcggcggtc gcgcttggtc 1148760
ggtctgccgt cgggataggc ggaagtgatg cggctgaatt ggtcgagctg tttgcgctct 1148820
tccctcaatg ttgccgtttt cgcgtcctct tcatacagaa gccgcgcctc ggatgccggg 1148880
cggcgttggt ggttcaaacc tttaaccttg attttatagg gaagggaatt gagcgtcagg 1148940
tcgataatat cgccgatgtc tatggtttta ctgtttttga ccttcgagcc gtttacttga 1149000
accctaccca gttcgatgtg cttttgcgca agggaacggg tcttgaaaaa acgtgccgcc 1149060
caaagccatt tgtccagccg catggcggaa gaatcgtgct tgtctttcat acgattttgt 1149120
ttgaaataat tgaatttgtt tcgagtttag cataagatac gccgccttat aactagtata 1149180
tatgcactaa tccactgttt tccatgctgt ccgaacacaa aaagagggta tggaaaagcc 1149240
gttttggaca ataaattaac tgcggaatat gcacaaatag cgtatgatag cggcagaatc 1149300
tgttgatgag agcttcattc tatgaaacct gttttttttgg attttgaaca acccatagcc 1149360
gaactgacca acaaaatcga tgagctgcgt ttcgtccaag acgagtctgc cgtcgatatt 1149420
tcggacgaaa tacaccgttt gcagaaaaaa agcaacgacc tgaccaaatc gattttcagc 1149480
aaactcacac ccgcccaaat ttcacaggtt tcccggcatc cgcagcgtcc ctatactttg 1149540
gattacattg aggcactgtt taccgatttt gaagaactgc acggcgaccg ccactttgcc 1149600
gacgattatg cgattgtcgg cggattggcg cgtttcaacg gacaaagcgt gatggtcgtc 1149660
gggcatcaga aagggcgcga caccaaagaa aaaatccgcc gcaacttcgg tatgccccgt 1149720
cctgaaggct accgcaaagc cctgcgcctg atgaagacgg cagaaaaatt cggcttgccc 1149780
gtaatgacct ttatcgatac gccgggcgcg tatcccggca tcggcgcgga agaacgcggg 1149840
cagtcggaag ccatcggcaa aaacctgtac gaactgacgc gcctgcgcgt tcctgttttg 1149900

tgtaccgtca tcggcgaagg cggttcaggc ggtgcgttgg cggtcgccct aggcgattac 1149960
gtcaatatgc tgcaatactc gacctattct gttatctccc ccgaaggctg cgcgtctatt 1150020
ttgtggaaaa ccgccgaaaa ggcggcggat gcggctcagg ctttgggcat tactgctgac 1150080
cgcctgcaaa agctggactt ggtcgatacc gtcatcaaag aaccattggg cggcgcgcat 1150140
cgggatttcg ggcaaaccat gaaaaacgta aaagccgttt tggaaaaaca actgcacgaa 1150200
gcgcaaagca tcccgcttgc cgatttgctt tcgcgccgtt tcgaccgcat tatggcttac 1150260
ggcaaatttt cggaacaata attcaggtag aacaagcagc aagcagtttg tctgaaactg 1150320
cttgcttttt ctttatcggg acggaaccgt gctgacttta gatgcgtttg agcaatgctt 1150380
gaaggattgt tttcctcaag gtctgaatgg aaaaaaaaca gcggtggcat taagcggcgg 1150440
cttggattcc gtcgttttgc tgcatctgct tgtccgcgcc ggaaaaaagg gcggttttat 1150500
tccggatgca ttgcatatcc atcacggctt gagtccccgt gccgacgatt gggcagattt 1150560
ctgccaaaac tattgcgata tgctcggggt ggggctggaa acggttaagg tctgcgtgga 1150620
aaaaaacggt ttgggcatcg aggcggcggc aaggcaaaag cgttatgccg cgtttgccga 1150680
aaaaggcttt gacgttttgg cgttggcgca ccacagggac gatcaaatcg aaacctttat 1150740
gctggcggtc gcgcgcggcg gcggtttgcg cgctttggcg gctatgcccg ccgtccgccc 1150800
ttttggggaa aaaggcatca tctggcggcc cttgctgcct ttttcacgcc aagatatatg 1150860
ggattatgcc caaaaacacg gtttgccgaa tatcgaggat gaaagcaata ccgatacggc 1150920
ttatttgcga aaccgcttcc ggcaccgtat tttgcccgaa ctttcggcgc agattcccca 1150980
tttcgggcgg catgtgctga acaatgtccg cgctttgcag gaagatttgg ctttgttgga 1151040
cgaggtcgtc gttcaggact gccgttgggt ttgcggggcc ggttatttcg atacggcgcg 1151100
gtggctgacg ttttccccgc gccggaaaac ccatattttg cggcattttc tgaaggaaaa 1151160
cggcattccc gtgccgaatc agaatgccct tgccgacatt gcccgggttt tgacggaggc 1151220
aaaaaccgga cgttggaact tgcaaggctt tgaattgcat cattatgcag gcaggctgtt 1151280
tgtgttccga ctggaaaaaa cggataaact gcggtttttg aaagacaggc agataagcgg 1151340
aaatttaagg gaaatattga cggggcaggg atttgtgttg aagcggcatc cgtttgggct 1151400
tcctgagcat cttttggagc aggacggaat tttgaggacg gtagcggcat cggatacgtt 1151460
ggccatgggc ggcatccata aggatgtgaa aaaaatcctt caggggaaac gggttttgcc 1151520
tgtcctgcgc ccaatttggc cgcttgttgc cgacagcgga aaccgtccat tggcgttggc 1151580
aaactgttgt gcggatttcc aatactcggt ttcagacggc attttgcccg tccatcctga 1151640
ctttcccatt ttattttgat aatatcgcaa acagatttcg gcggcgttca gtcgggtatt 1151700
gtccggttgc atatttctaa aaggcttgtg aagtgaaaca catcagttcg accaataatg 1151760
aacacatcag acacctgcac cgcctgttgt cgcaaggaaa gttcagacgg caatacgccc 1151820
aaaccgtttt ggagggcgtg cacctgcttc aggttttcct gcaatccggc gggatgccgg 1151880
tcggggtata tattcccgaa gcgaaaatgc cgtctgaaga agtccgtaaa ttgacggcgg 1151940
ttttgccgga agacgggttt ttttccgttt cagacggcat attgaagaaa atcagcagcc 1152000
tgacttgtgc ggatgatgtg cttgcgctga ttgatattcc agatgcgggt gctttgccgg 1152060
ccggcggcga ttgcgtggtt ttggacggcg tgcaagaccc gggcaatgtc ggcacggtgt 1152120
tgcgaagcgc ggcggcggcg ggaatcggcg cggtcatttt gggcaaaggt tgtgcggacg 1152180
cgtggtcgcc caaagtgctg cgagccggaa tgggcgcgca tttcttgtcg gagatttatc 1152240
cgcaggcgga tttggaaata tggttggtgc gctataaagg ccgtgtgttt gccaccgcct 1152300
tgcgcgagga aaagcaggcg gttttgtacg gcgaagattt gtgcgaaccg acagcctggg 1152360
tgtttggcaa cgaaggcgcg ggggtcggta aagcagtttt agatagggcg gacaagtgtg 1152420
tcaggatacc gatgcacgat gcaaccgagt ctttaaatgt cgcgatggcg gcgacaatct 1152480
gcctgtttga acaaatgcgc caacgggcgg cgtattgagg aagagaaatg ccgtctgaaa 1152540
aaatctatta cggcgtattg attttcttat gtatcgcttc tatgctgctg tcgccgtttt 1152600
tttatgcggg tgctttgaag cccaagaagg cggcattgcg gaaggacggg cagtggaaac 1152660
tcatctgatt gtccaatgcc gtggcggcgg cggttttggc ttgggtgtgg tggaaatggt 1152720
tttgacagat attgctcaaa atcgtgctaa tggaatccga acaaataaag aatttggtaa 1152780
aaaatttgtt aaatcaacga attaaagttt tgtggaaaac aaaacagctc taagcaaata 1152840
gggcgtttgt cggtaaatac ggaagagttg cggcattatc gggcatcttt aacaagtagt 1152900
gccgtcttga caggcaatcg gtttttatgg gcagcttgca aaatcgcgga tataaaattg 1152960
cgaatcggtt aaagtgtggg gacgctatga aaaattgcga attttttatg acccgacaag 1153020
ggcaatctat gatagcggtg cagattactt aactagggaa aaacatagat tagtcgtaat 1153080
tgcaaatagt gcttgggggc tattgcttaa tttatcttgt tattatgacg aggttttgga 1153140
aaagcggaaa ataccgttcg gcaaacagga aattgatgac gatatggaca aagtgtccgc 1153200
ccttaagcgg aagtttaaag atatttctga aatcaaagta ggggatggtt gggaataccc 1153260
gttcaattat gagcagggaa tgaaagaatt agatgaagtg ctattgaaat acattccctt 1153320
ttttgaagaa gaacgataaa ggaggttgat atgcgcgtat ctaaaataat tggaagtatg 1153380
ttgcttgtta cagcggttca gaccgtattt tcggcaaatg tttacgcgtg ccgccataat 1153440
ggtaaaacca gttacagcca aactccggga aaacattgta ccaacgcggg tttggggcgg 1153500
gaccgggtgt acagttcggt tagaccggca gtaaaagaca gggcggaaga cgcaggggtc 1153560

ggcgattatt cggacacggt gagggacgaa cacgtccaaa atccgaaagg aaatgcacag 1153620
aaagacggtt cggctgccgg catcaagccg cactgattga agccgaatca gcccttgcgc 1153680
tgtcggacgg caaaatttga acgattgggg aactttgatg aaacacatcc atattatcgg 1153740
tatcggcggc acgtttatgg gcgggcttgc cgccattgcc aaagaagcgg ggtttgaagt 1153800
cagcggttgc gacgcgaaga tgtatccgcc gatgagcacc cagctcgaag ccttgggtat 1153860
agacgtgtat gaaggcttcg atgccgctca gttggacgaa tttaaagccg acgtttacgt 1153920
tatcggcaat gtcgccaagc gcgggatgga tgtggttgaa gcgattttga acctcggcct 1153980
gccttatatt tccggcccgc aatggctgtc ggaaaacgtg ctgcaccatc attgggtact 1154040
cggtgtggcg gggacgcacg gcaaaacgac caccgcctcc atgctcgcat gggtcttgga 1154100
atatgccggc ctcgcgccgg gcttccttat tggcggcgta ccggaaaatt tcggcgtttc 1154160
cgcccgcctg ccgcaaacgc cgcgccaaga cccgaacagc caatcgccgt ttttcgtcat 1154220
cgaagccgac gaatacgaca ccgccttttt cgacaaacgt tctaaattcg tgcattaccg 1154280
tccgcgtacc gccgtgttga acaatctgga attcgaccac gccgacatct ttgccgactt 1154340
gggcgcgata cagacccagt tccactacct cgtgcgtacc gtgccgtctg aaggcttaat 1154400
cgtctgcaac ggacggcagc aaagcctgca agatactttg gacaaaggct gctggacgcc 1154460
ggtggaaaaa ttcggcacgg aacacggctg gcaggccggc gaagccaatg ccgacggctc 1154520
gttcgacgtg ttgctcgacg gcaaaaccgc cggacgcgtc aaatgggatt tgatgggcag 1154580
gcacaaccgc atgaacgcgc tcgccgtcat tgccgccgcg cgtcatgtcg gtgtcgatat 1154640
tcagaccgcc tgcgaagcct tgggcgcgtt taaaaacgtc aaacgccgga tggaaatcaa 1154700
aggcacggca aacggcatca ccgtttacga cgacttcgcc caccacccga ccgccatcga 1154760
aaccacgatt caaggtttgc gccaacgcgt cggcggcgcg cgcatcctcg ccgtcctcga 1154820
accgcgttcc aacacgatga agctgggcac gatgaagtcc gccctgcctg taagcctcaa 1154880
agaagccgac caagtgttct gctacgccgg cggcgtggac tgggacgtcg ccgaagccct 1154940
cgcgcctttg ggcggcaggc tgaacgtcgg caaagacttc gatgccttcg ttgccgaaat 1155000
cgtgaaaaac gccgaagtag gcgaccatat tttggtgatg agcaacggcg gtttcggcgg 1155060
aatacacgga aagctgctgg aagctttgag atagcccggg cgatgccgtc tgaaagccct 1155120
tcagacggca tcgcccggct gcgcggcaca aaggcggaaa aaccgtttgc cccgtatttt 1155180
caaacgcgtt acacttgccg ccgctgtttt cagccatttg attacccgca accgccgtca 1155240
ttgcgccggc ggtttgcctg tcagcgtcat tgcgccgctg taaatacgaa agaacacatt 1155300
atgaccgtat cccccgtcgc cttgcgccgt aagaccgagt gcaagcctca tcccaccgcg 1155360
cgctattgga aaaaatgcga tgtcgaagcc ctgttcggac ttcccttcct cgacctcatt 1155420
taccaagccg ccgaaatcca ccgccaaaat ttcaacccgc gcgaaatcca gctttccacg 1155480
ctgttgtcca tcaaaaccgg cggttgtccc gaagactgcg cctattgtcc gcaatcggcg 1155540
caccacaata ccaatctggg caaagagcag atgatggatg tggatgaaat cgtcgaaaaa 1155600
gccaaaatcg ccaaatcgcg cggcgcaagc cggttttgta tgggcgcggc gtggcgcggc 1155660
cctaaaccca aagacgtgga gacggtttcc gcaatcatca aagccgtcaa gggcttgggt 1155720
atggaaacct gcggcacgtt cggtatgctc gaagaaggta tggcggaaga cttgaaagag 1155780
gcgggcttgg attattacaa ccacaacctc gacaccgacc ccgaccgcta caacgacatc 1155840
atccacaccc gccaacacga agaccgaatg gacaccttgg gcaaagtccg caacgccggt 1155900
ttgaaagtct gctgcggcgg catcgtcggg atgaacgaaa cccgcgccga acgtgccggg 1155960
ctgattgcca gtctcgccaa tctcgacccg cagcccgaaa gcgtgccgat taaccggttg 1156020
gtcaaagtgg aaggcacgcc gcttgccgat gccgaagatt tggactggac ggaatttgtc 1156080
cgcaccatcg ccgtggcgcg gattacgatg ccgcaaagtt atgtccggct gtcggcaggg 1156140
cgcagcaata tgcctgaagc aatgcaggcg atgtgcttta tggcgggcgc gaactcgatt 1156200
ttttacggcg acaagctgct gaccacgggc aatcctgatg aggacggcga cagaatcctg 1156260
atggaaaagc tcaacctgta tcccttgcag tttgaaccgg aaggcgaggt cgccgaagtg 1156320
gaaaaagcct ctgggattaa agtggattat tgacgattga aaaatgccgt ctgaaacccg 1156380
gaaaaaggct ttcagacggc atttgtccgg acggcatttc caatatcttt ttaccggcgc 1156440
gtgatgctgc cgtcgggcga gacatccagc ccgttcccct tggggaaatc gctgcggttc 1156500
agataaataa tccgcccgat aacggttttc tcggggtcga ttttggggat tttatcgccg 1156560
acttgagtga tggggataat gttgccggaa acgaaccgcc cctgtttgtc ggtgataatt 1156620
ttaaaaatgg gggcgatgcc gctgatgccg gaggtgttga ttgcgccgta ggtggcaaag 1156680
ttgccgccgc tgtaggagat gaagcggtcg cggtaaagtt cgacggcgcg agtaacgtgc 1156740
ggcccctgcc cgaatacgac atccgcgccg gaatcgacgg caagccgcgc aaactcaacg 1156800
acgttgcccc tgttttcccc atagaagatt tcggtatcga acggcaggtg ttccgcctgt 1156860
ttcccttccg cgccgccgtg gaacatcaca atgacgatgt cggctttctg tttggttttg 1156920
gtaatccgtt ttctaacttt ggcataatcg ttcagtttga cggcggcaag gttgggggcg 1156980
aaggagacga agccggatct tacgccgttt ttcttcagga tggcggtttc aaacctgttt 1157040
tcgatgcccg aatatttgat gttcaattcg tcaaggttcg ccctttatgc cgtttccgca 1157100
ccgcaaaccg ccggggtcaa gccctcggcg catcctgccg gaacggaatc cccgtgctgc 1157160
cgattgacgg ttcaaacccc gcgcccgttt caaataccgg cgatgtggac ggacaggatg 1157220

cgcctgacga aaagacagcc gataccgttt ccattatcgg cgtgggcgac attatgctcg 1157280
gcagcaatta tccggtcgat tacctgcccg ataccaatat tctgaaaaac gtcgaatctg 1157340
ccttgcaaga cgcggacatt accgtcggca acctcgaagg cacgctgttt gacgaaggcg 1157400
gtacgccgaa aaaatgtgca aacccccaaa atatgctatg cattccgaac gccctccgca 1157460
tacgggcaat accttgccga cgcgggattc gactacctca gcttcgccaa caaccacagc 1157520
aacgacttcg gcgcgcaagg catcacggca acggcggcga gcggcagctc ttttacatac 1157580
tcgatcgcgc taaagccgct gccgataacg aggccaaaat tgcggaaaat accgccatcg 1157640
cccagataaa tttgtccatc atcagacctt tactgttcag acgagacagc atttgccgca 1157700
cgttttgggg cttatctttc gatttgcgct acgtcgcgca ccgcgccttt gtcggcggaa 1157760
gtcgccatcg cgccgtaagc tcttaatgcg gcggagacgt agcggtcgcg gtttttaggc 1157820
ttccatgctt tgctgccgcg cgcttccatt tcggcacggc gtgcggcaag ctcttcatcg 1157880
gaaatggcaa ggtggatgct gcggttgggg atgtcgattt cgacggtatc gccttcgtgt 1157940
accaaaccga tcgcgccacc ttccgccgct tcgggcgagg cgtgtccgat ggacaaacct 1158000
gatgtgccgc cggagaagcg tccgtcggtt aagagagcgc aggctttgcc gaggccttta 1158060
gatttcaggt aggaagtcgg atacagcatt tcctgcatgc ccgggccgcc tttcgggcct 1158120
tcgtagcgga tgatgacgat gtcgccagcg acgatttggt tgcccaaaat gccttctact 1158180
gcgtcttctt ggctttcaaa cacgcgggcg cggccggtga atttgaggat gctctcgtcc 1158240
acgcctgcgg tttttaccac gcagccgcgc tcggcgatgt tgccgaacaa gaccgccaaa 1158300
ccgccgtctt gcgagtaggc gtgtgccacg tcgcggatac agcctttttc gcggtcgagg 1158360
tcgagggttt tccacatacg gttttgcgag aacgcttggg tggtgcgtac gccgcccggc 1158420
gcggctttga agcgttcgat ggcacgggtg ttttcgggat tggtcacgtc ccattgttca 1158480
atcgcgtctt tcagcgtcgg cgcgtggatg gtgtgcacgt cggtgtgcag tttgcccgct 1158540
ttgtccagtt ctttcaggat ggcgaagata ccgccggcgc gatgcacgtc ttccatatag 1158600
tagtcgtggt tgttgggtgc ggttttgcag atgcagggca cgacgcggct taagcggtcg 1158660
atgtctgcca ttttgaaatc gacaccggct tcgttggcaa cggccaacag gtgcaaaatg 1158720
gtattggtgc tgccgcccat cgcaatatcc atcgtcatag cgttttcaaa cgcttttttg 1158780
gtggcaatgc tgcgcggtaa cacggtttca tcgttttgct cgtaatagcg tttggtgatt 1158840
tcgacaatca tacggccggc ttcgaggaac aattctttgc ggccggcgtg ggtcgccaaa 1158900
tacgaaccgt tgccgggcag ggaaaggccg agtgcttcgg tcaggcagtt catcgagttt 1158960
gccgtaaaca tacccgaaca cgagccgcag gtcgggcagg cgttttgttc gacttcctcg 1159020
acttgccggt tgctgacatt gtcgtccgcc gattcaatca tcgcgtcaat caagtccaaa 1159080
cggcgttcgg gctggatgtt tgccacgccg ataaccttgc ccgcttccat cgggccgccg 1159140
gagacgaaga tggtggggat gttcaggcgc atcgcggcaa tcagcatgcc cggggtgatt 1159200
ttgtcgcagt tggaaatgca caccagcgcg tcggcgcagt gggcgttgac catatattcg 1159260
atagagtcgg caatcaaatc gcggctgggc agggagtaca gcatgccgct gtgtcccata 1159320
gcgatgccgt cgtcgatggc gatggtgttg aattctttgg cgattgcgcc ggctttttcg 1159380
atttcgcggg caaccagctg gcccatattg tgcaggtgga catggccggg cacgaattgg 1159440
gtgaaggagt tggcaacggc gatgatgggc ttgccgaagt cggtttccat cacgccggtg 1159500
gcgcgccaca atgcacgtgc gcccgccata ttgcggccgt gggtggaggt tttggagcgg 1159560
tattcaggca tagtgtgttt ccttgtgcct ataccgtctg aaagacaggg ctgtttcaga 1159620
cggtatcggg tacggttttt tagagtggga aaagagggta ttttatacca agtatcggaa 1159680
ttttgcggga ttgaaacggc gtgcggcaaa aaagaaaatc cccgcaggaa tgcggggacg 1159740
ggttcaggcg cgggcaatcg cgacggcttt ggatgcgtcc caaaaatcaa cgggtgcatt 1159800
taatacgggt ttgacgatgc ccgtccgtat ggcgaaatcg ccgaaacctt cgccgatatt 1159860
gcgttctgcc gcccatttgc cgatcaggtc gtccaattcg gcaaggattt cgggcagggt 1159920
gatgttttct ttgtaaagac gggggatgcg tacgccttca cggtcgccgc cgatatggag 1159980
gttgtagcgt ccgacggctt tgccgaccag tccgatttcc gccaacatcg cccgtccgca 1160040
gccgttcggg cagccggtaa tgcgggtaac gatgtagtcg tccgacgtgc cgtgtttcgc 1160100
cataatctta tccagctcgc cgatgaagtc cggcagcacg cgttcggctt ccgccattgc 1160160
cagcgggcag gtcggaaagg aaacgcagga catcgcattt tcacgcagct tgctgacatc 1160220
gttgcggatt aatccgtatg ttcgggcaaa ttcttcgatt tttgctttgt ctgcttcggc 1160280
gacatttgcc acgatgaggt tttggttggc ggtgatgcgg aaatcgcctt tgtggatttt 1160340
ggcgatttcc aacacgccgg tcagaagctg tttcccgcct tcgtcaacca aacgcccgct 1160400
ttcgatgaaa agggttaaat gccagttgcc gtctatgcct ttcacccagc cgatgcggtc 1160460
gccgcgcccg gtaaatttga acgggcgtac gggttcgaac ggcataccca tacggcgttc 1160520
aacttccgcg cggaagttgt ccaagcccat attttgaatg gtgtagcggg tgcgggcgtt 1160580
tttgcggtcg ctgcggttgc cgaagtcgcg ctgcgtggtt accaccgctt cggcggcctt 1160640
cagcgcgtgt tccggaggca cgaaacccag ttccagtgaa atgttcggat aggttttggt 1160700
gttgccgtgt tccatcgaaa gcccgccgcc tgccaaaaca ttgaagccgg caagctgtcc 1160760
gttaccgtct gaaacggcga cgaaatccaa atcgttgccg tagcagtcca catcgttcaa 1160820
gggcgggatg acgactgcgg ttttgaattt tcgcggcaga taggttttgc ccaaaatcgg 1160880

ctcgtcttct tgaaggaagt cgtcggaact ttgaactttt ttgccgtcca cccacacatc 1160940
cagataaccg cgcgtgcgcg gcagcaggtg ttcggaaatc tttttcgcgt attcgtaagc 1161000
ctgccggtgc agttcggact cgatcgggtt ggacgtgcaa agcacgttgc ggttcatatc 1161060
cgccgccgtg gcgatggaat ccaaacccag tttgtgcaag aggcggtgca tcgtctgcaa 1161120
cttggctttc ggcacgccgt gaaattggaa ggtttgccgg ttggtcagcc ggatggagcg 1161180
gtaatgactg ttttcccggg caaatttgtc cagttctatc cattgggacg gtttgatgat 1161240
cccgcccggc agccggcagc gcaaaagcat aaatttcaag ggctcgagtt ttgcctcggc 1161300
gcgttcggcg cggatgtcgc ggtcgtcctg ctcatacata ccgtggaagc ggatgagttg 1161360
gaagttgtcg cctttgaagc cgcccgtgag cgggtctttc aaatcgtcca aaatcgtgcc 1161420
gcgtaaaaaa ttgctttcgg ttttcagacg ttcgttgtcg gatagcggtt tttcttgcca 1161480
cgccaaacct tttgtcttgg tctgtacggt cattttgtgt tcctcccgat tatatttaat 1161540
caataaacat cacgctgata gcgtttttct tcgcgcagca tatccaaata ttcttctgcg 1161600
ccctcttcgt ccaaatgtcc tgccccgata atcacatcca gcaaggcggc ttccacgtct 1161660
tttgccattt ttgccgcatc gccgcacaca tagatatgcg cgccttcctg cagccattgc 1161720
caaagtcctt ccgcctgttc gcggattttg tcctgcacat agatttttc ttcctgatcg 1161780
cgggaccagg cgaaatcgta cctgtgcagg aagccgtctt tggcaaactg ctgccattcg 1161840
gtttgataga gaaaatcacg ggcaaaatgc ggattgccga aaatcagcca gtttttgcct 1161900
tccgcatttt ctgcggcacg ttgttggacg aaagcgcgga acggtgcgac gccggtgccc 1161960
gagccgatca tcacaatcgg cttgcggctg tcttcgggca gcctgaagcc gtcgttgcgt 1162020
tccacaaaca cgcgcaccgt gccgtcctct tccagccggt cggcaaggaa acccgatgcg 1162080
ccgcccgttc tggcgcggcc ttcgtgttca aaacgaacca cgccgacagt taaatgcact 1162140
tcatcgccca cttccgcctg tgctgaagaa atcgaataca aacggggtgc aagcggacgc 1162200
agtaaacgga tgaattgttc tgccgtcagg cttgccggga agcggtgcag cacatcgaca 1162260
ataggcgtgt tttgcacgaa atcctgcaaa acggcgttat cggcaatgat tttatcgagt 1162320
tcttcataat gggcgaacgc ggcatagcct ttgacgaaag ccggagtgtt ttgcgtgagt 1162380
tcgaaatgag atgaaagtgc gcgcgcaacc ggcatcatct ttccgcccgc ctgtatttcc 1162440
gttgccggat cgatgccgag caggtctagg atttccctga ccagtgccgg atcgttgtca 1162500
aaccaaacgc cgagcgcgtc gcccgggagg tagtgcaaat ccgaaccgct caaatcgatt 1162560
tcgatgtggc gcacgtcttt atcggattgg cgggcggtga ttttctgatt ggccagcagg 1162620
gcggcgggaa agggggctgc cttgcagtac ctgccatccg gtgccgtctg aaggccggcg 1162680
gggggcgttg tctgcggcgc gggcgttgcc cggtttttttg cggcttcttc ttttaagagt 1162740
gcggcgatat tatctgtcca ggcgtttgcg gaggcggtaa agtccaaatc cgcatcaacg 1162800
cgttcgagca gccgttttgc gcccaattct tcaaaacgcc ggtcgaaatc tttacctgcc 1162860
tgacagaaat tcggatagga actgtcgccc aaacccagta cggcaaattg gagtttgtcc 1162920
aatttcgggg cttttttgcc gttcagcagt ttgtgcagca cgacggcttc tttcggcggt 1162980
tcgccttcgc cttgggtgga ggtaaccagc agcaggcggc gttcgccggc gatgtttttc 1163040
gccttatagt ctttcagttc ggcgcgactg acttggatgc cggcggcttc caggctgtcc 1163100
gccgctttgt cggcaacgga tttcgcattg ccggtttgcg aggcggaaag gacggttacg 1163160
gaaaaaggtt ctgccgccgg caatgccgtc tgaagcgcgg gcagtcctgc agatgccccg 1163220
tttcctgctt ttgcccaagc gtagccggac agccacgccc attgtgccgc gtccagcccc 1163280
gacaggagct gcgtgatttc gggcggcaga ggcggtaatg gcggatttgt gttctgcata 1163340
tcgtgttcac tcataaaatc atacctgccg caacagtgcc gtatgtcgct tcgtctatca 1163400
ggataaacga accggcggcg gtgttttccg cataaggcgt tgccgtaacg ggtttttgaa 1163460
ggttgatgcg gactttggcg atgtcgttca tcttcaagga ttccgcgccg gcctcttgtt 1163520
ccagcgtgcg gacatccaaa acgctttcaa tttccccgac ttttgccggc acggtttgcg 1163580
tgccgtgctt gagcaggtat ttgcgcgcgg tgttgagcgg acgttcgtca aaccagcaaa 1163640
gcgtggcttc cagatgtttt tgcggggcga gcggggaatt tttatcgaca aaaaggtcgc 1163700
cgcgcgaaac atcgatgtcg cggtccagcc ggagggttgc cgcctcgccg gcaaaagcct 1163760
gcgccacttc ccctttcggc gtgatgattt cggacacttc ggcggtcagc ccgttcggtt 1163820
cgatgcggac ggtttgcccg acggcgaccg aaccgcgttc gatgcgcccc tgatagcctc 1163880
ggaaatcatc ggccttgtcg gcatcttggc ggacgaccag ttgcacgggg aaataaaaat 1163940
cgtcggcggt gcggctgact tcgtccgccc ccggcagggt ttccaaaatg gacaataagg 1164000
gttcgccttt ataccaaggc atattgccgc cggggtaaac aatgttgtcg cccaagagtg 1164060
cggacatcgg tacgaaatgc gcgtctttca aaccgagctg ttcggcaagt cggcggtatg 1164120
cctccacaat ggcgttgaat ttgtcttcgc tgtaatccag caggtccatt ttgttgaccg 1164180
ccaccacaat atgcgggcag ttgagttggc ggaggatggc ggaatggcgt ttggtctgcg 1164240
gcagaagctg caagggctgc gcgccgaaat ccagttggga tgcgtcaacc agcacgactg 1164300
ccgccgaagc ggtgcttgcg cccgtaacca tattgcgcgt gtattgttcg tgccccggcg 1164360
tgtcggcgat gatgaatttc cgtttcgccg tggaaaaata gcggtatgcc acatcgatcg 1164420
taatgccctg ttcgcgttcg gcttccagtc cgtcggtcag gatggagaag tctatggctt 1164480
ctttcaaacc tttgcttttg ccggattcca aggttttgat ttggtcggac agcagggctt 1164540

tgctgtcgta gagcagtcgt ccgatcaggg tgcttttgcc gtcatcgacg ctgccggcgg 1164600
taatgaagcg gagcggggtt tggtgttgtg ccgtcatatt ttcttcctca tatctgctta 1164660
aagggttttt gaaatttaga aatagccttc ttttttgcgt ttttccattg ccgcctcgct 1164720
tgcctgatcg tccagccggg tcgcgctgcg ttcggaaatg tcggcaaccg ctgtttctct 1164780
gataatctcc gtcggcgtgg acgcggtgct ttctaccggg caggtgcagc tgatgtcgcc 1164840
gacggtgcgg aagcggacat caaggatttc ggaggtttca gacggcattt tcggggtgag 1164900
cggcgttaca gggaccagca gccccctgcg tctgaccact tcgcgcctgt ggctgtaata 1164960
aatcggcggc agctcgaggt tttcgcgggc gatgtattgc cagatgtcga gttccgtcca 1165020
gttggaaatc gggaagacgc gcatattttc gcctttgtgc agcctggtgt tgtacagcga 1165080
ccacagctcg gggcgttgcg ccttcggatc ccattgtccg aactcgtcgc ggaacgagaa 1165140
aatccgttct ttggcgcggg ctttttcttc gtcgcgccgc gcgccgccca taagcgcgtc 1165200
gaagccgttt gcctcgatgg tttccaacaa ggtaaccgcc tgtgccgcat tgcgcgaatc 1165260
ggtttctttg cgtaagacca ccgtgccttt ggcaatggag tcttccacgc gccccactat 1165320
caggcgggca ttgagttttg ccgcctgcgc gtcgcggaag gcaatcactt cggggtagtt 1165380
gtgtcccgtg tcgatatgca ccagcgggaa gggcagtttc accggccggc tgcccagccg 1165440
gaaggctttg caggcgaggg cgagcaggac cacggaatct ttgccgccgg aaaagagcag 1165500
ggcggggttt tcgcattctg ccgccacttc gcggatgatg tggatggatt cggattccaa 1165560
ccagtcgagt tgggcgttgt tcggttcggt tttcgtcata ccatattcct tatttcttct 1165620
gtctgatatt tatgaattat ttgtgcagcc cgcattcttt gctgtttctg ccttcccacc 1165680
accaccgccc ggcgcggatg tcttcgcccg ccttgacggg gcgggtgcag ggtcgcagc 1165740
ctatgctggg aaatccttgc cggtacaaat cgttgtaagg cacattgttg gcgaggatgt 1165800
atgcccacac gtcgtgttcc gaccagtcga aaatcgggtt gtatttgccg atgccccgtc 1165860
cggcatcgta ttcggcaaac ggcagttccg tgcgtgtggc ggattgttcg cggcgttgcc 1165920
cggtaagcca ggcgtccgcg cctgcaatgg cgcggttgag cggttcggtt tttcggatgc 1165980
ggcagcattc gcggcgcgct tcaacgctgt cgtaaaaggc aaacctgcct ttgctttcca 1166040
cataacggtc ggcatcttct cgaaccggcc ggaaacgctt tatccgcaaa tggggatatg 1166100
cgcgtccgag cctgtccagc aggttcaggg tttccgtgtg gagcagcccc gtatccaagg 1166160
taaaaatgcc gatattgagg ttttcgccgg cgataaggtc ggtaatcacc atatcttctg 1166220
ccgcaaggct gctggcaaac cgtgcatccc ggtggctgcc gacaatccgg tgcaggcgtt 1166280
gtttgagggt ttccgttttt tccgcaaggg cggtttcgcc gccggatccg atatgcggta 1166340
tctgccacag ggcgggtttg aacagtgtcg tttccatttt tcccgcctta tgccgcccgt 1166400
tgtccggcat tcagtccgcc caatgcggga tacgtctgcc cgacccggtt ttctccttcg 1166460
ccgttttcac cgaaccaggc gagtttttcg tgcagcccca ccacttcgcc tatgacaatc 1166520
aatgccggat tcggcgcggt ttcggcgagt tcggcaaggt tggcgagcgt gccggttgcg 1166580
gtttttttgag ccggcagcgt gccttggctg ataacggctg ccggcgtgtc gggcgagcgt 1166640
ccgtgctgtt gcagccgttc ggcaatcagg gcggctttga gcgcacccat ataaatcacc 1166700
aaggtctggc ggctgcgggc gagggtctgc cattcgatgt cgggcgcatc cgccttgcgg 1166760
tggccggtta cgaaaaccgc actttgggca taatcgcggt gcgtgagcgg gatgccggca 1166820
taggcggtcg cgccgacggc ggcggtaatg ccggggacga ccgaaaacgg aatctgatgg 1166880
cgtgccaagg tttccaattc ttcgccgccg cgtccgaaca cgaaagggtc tccgcctttc 1166940
aaacgcacca cgcgcctgcc ttcgcgggcc agcctgacca taagcgcatt ggtgtcctct 1167000
tgcggggtgc gctcgccccg ggcgcgcttg ccgacaaaaa tccgttccgc atcgcggcgg 1167060
acgagggaca gtatgccgtc tgaaaccagc gcgtcgtaaa gcaccacgtc tgcctgctgg 1167120
atttcctgca gccctttgag cgtcagcagc cccgcatcgc cgggacccgc gccgaccagc 1167180
gagacggagc cgccttgatc attttgacga ctttgttcca attggcctgc caattcccgt 1167240
tcggcaaggg tgttttgccg gtttttgacg agggcggcga aacgtccgtt aaactgcttt 1167300
tcccaaaagc ggcggcgttc ggtaacggat ttcagtttgc ccttgacggc atcgcgccac 1167360
cttcctgaaa tttccgccat atcgcccaaa gacggcggca gcagggcttc cagcctttca 1167420
cgcagcagtc gggcgaggac gggcgcgctg ccggagctgg aaacggcaat ctgaaccggg 1167480
ttgcggtcga taaccgacgg gaagatgaag ctgcaatggt cgcggtcgtc caccacgttg 1167540
accggctttt ggcagctttc ggcaagatgg aaaacgcgcc ggttgagggc ttggtcgctg 1167600
cttgccgcaa tgatgaggaa aaccgtgcgg atgtgttcgg cacgaaattc ttcggcaagc 1167660
cacaggattt tgttttccgc cgccaacgcg gagagttcgg cattcaggtg ttttgcggca 1167720
accctgacct ctgcgcccgc cttcagcagc aggctgattt tgcgtgcggc gaccgcgccg 1167780
ccgcctacga ccaatacggg gcggccggcg aggttggcga aaatagggaa ataattcact 1167840
ggctgactcc tttgctgttt gcccgcacct tgtttccgat acggtgcgtc gcggcatttt 1167900
tgtcggaatg cgggtcattt tagacaaaag gattttcccc ggttaaataa taaaaaggta 1167960
tttgttagaa gctgaaagct atatgggggc ggctgcggat gcggcggttt tccgttttat 1168020
aacggtttcg gaagaaaaac ggcctgaagc cgtttcgggc attcagaccg tttgcgtggt 1168080
gaggggatgc cgtccgaagg gcgaaaaggg cttcagacgg cattgatgtc gggtttcagg 1168140
acaggagcag gatggcggct gcggcaagcg aggcaaccga taatgcggcg gcaagcgcgg 1168200

ctttgcctgc aaagcggatt gaggttttgc cttcgatgta tttgaagccg gttatcatcg 1168260
ggaggatgag gtttttcttt ttgaatacgc ggtatgcggc gacggcggcg atgtggattg 1168320
cagaaaaaac ggcgagcagc ttgaaaaagt tgaggtggat tttccgcata aggctgcccg 1168380
tatgttcgga aaccaaatgg ttgaggtagc cgttggtgct gaaggtgttt tcatcggcgg 1168440
caaaaagccc ggtgccgact tggaaggaca cggcggccaa aagcgcaacg accatcagtg 1168500
cgcccaaggg gttgtgtccg ggctggatgt gttcgggaat accgtttttc agatagccgc 1168560
gtatgcctgc ccagccttgg acgaaacggg aaaaacgggc ggtatcgctg ccccaaatgc 1168620
cccagcagag gcgaaatacg agcaggaaaa ggacgaacag cccgacgcgc gtgtgccatt 1168680
gcagcatatc gccgccggct ttcgcgctat accacataaa gggcagggac gcggcaagca 1168740
gccagtggaa aaggcgggtg gggaggtccc agactttggt tttgtttttc ataatcggtt 1168800
tccggcggta gaatcggttt gttttcgagc cttattttaa ccgattggag gggcaatgtt 1168860
tcccgttttt catctttcag gcgagagccg ccgccagatg cttcagacgg cattgcgttt 1168920
tccccatgtt ttcaaagccc gtgcggaaga ttcgcacaaa gggactttcg gcacgctcgc 1168980
cgtagtcggc ggatcggcag ggatgagcgg cgcgcccgta ttggcggcat cggcggcaat 1169040
gtatctcggc tgcggcaaag tgtgggcggg tttcaatcag gatacgctac cttttgccgt 1169100
tattgccggt tttcccgaga ttatgctgga tacggcggac agtttggcca aacgtcaaga 1169160
tataaacgcc tgggttgtcg gttgtggatt gggtacaggt agggcggcgg tcggaacgct 1169220
tgccggaatt ttgacggaac acacggacaa gcccgtcgtt ttggatgcgg atgcgctgaa 1169280
catattatca accgatgccg aaacccgaaa tctggcgcgc gggtgtaaaa acctgatttt 1169340
aacgccacac cccgccgaag ccgcgcgcct gcttggaacg acggttgcgc aggttcaggc 1169400
ggatcggacg gcggcagtga ggaagatagg ggcaattttc ggcgcaaccg tggttttaaa 1169460
ggggcacaaa acattggttg cctcacccga tacggaaatc tatgtcaacg aaagcggcaa 1169520
cgcgggattg gcaacggcgg gcagtggcga cgtattgggc ggcatcatcg gcagtctgct 1169580
cgcacagggc gtgccggttt ttgaagccgc ctgcgcgggc gcgtggctgc acggcgcggc 1169640
ggcggatgtc ataaaagaat cggcaggcat tgcggcaggg ctgttggcag gggaaatcgc 1169700
tccggcggca aggtggctgc gcaaccggat aactaaaagt atgtaagaag atatagtgga 1169760
ttaacaaaaa ccagtacatc gttgcctcgc cttagctcaa agagaacgat tctctaaggt 1169820
gctgaagcac caagtgaatc ggttccgtac tatttgtact gtctgcggct tcgtcgcctt 1169880
gtcctgattt ttgttaatcc actataccgt ctgaaaggca agggcttcag acggcatctt 1169940
catttcccaa atactgtccg gtaaagcgtg gtataatgcg tagttatctc ttacagtttt 1170000
tgaaaaacat taattatgaa acaaatccgc aacatcgcca tcatcgccca cgtcgaccac 1170060
ggcaaaacca cattggtcga ccaactgctg cgccaatccg gcacattccg cgccaaccag 1170120
caggttgacg agcgcgtgat ggacagcaac gaccttgaaa aagaacgcgg catcaccatc 1170180
ctcgccaaaa acaccgccat cgattacgaa ggctaccaca tcaatatcgt cgacacgccg 1170240
ggacacgccg acttcggcgg cgaagtagag cgcgttttgg ggatggtgga ctgcgtcgtc 1170300
ttgttggtgg acgcgcagga aggcccgatg ccgcaaaccc gtttcgtgac caaaaaagcc 1170360
ttggctttgg ggctgaaacc gattgtcgtc atcaacaaaa tcgacaagcc gtccgctcgt 1170420
ccgagctggg ttatcgacca aactttcgag ctgttcgaca acttgggcgc gaccgacgag 1170480
cagttggatt tcccgattgt ttacgcttca gggttgagcg gtttcgccaa attggaagaa 1170540
accgacgaga gcaacgacat gcgtccgctg ttcgatacta tcttaaaata tacgcctgca 1170600
ccgagcggca gcgcggacga aacgctgcaa ctgcaaattt cccaactcga ctacgacaac 1170660
tacaccggcc gcctcggtat cggtcgtatc ttgaacggac gcatcaaacc cggccaaacc 1170720
gttgccgtca tgaaccacga tcagcaaatc gcccaaggcc gcatcaacca gcttttgggt 1170780
ttcaaaggtt tggaacgcgt gccgcttgaa gaagccgaag ccggcgacat cgtgattatt 1170840
tccggtatcg aagacatcgg tatcggcgta accatcaccg acaaagacaa tcccaaaggc 1170900
ctaccgatgt tgagcgtgga cgaaccgacg ctgacgatgg actttatggt caacaccagc 1170960
ccgctggcgg gtacggaagg caaattcgta accagccgcc aaatccgcga ccgcctgcaa 1171020
aaagaattgc tgaccaacgt cgccctgcgc gtggaagata ccgccgatgc cgacgtgttc 1171080
cgcgtatccg ggcgcggcga gctgcacctg accattttgc tggaaaacat gcgccgcgaa 1171140
ggctacgaac tcgccgtcgg caaaccgcgc gtcgtgtacc gcgacatcga cggtcaaaaa 1171200
tgcgaaccgt atgaaaacct gaccgtggat gtacccgacg acaaccaagg cgcggtaatg 1171260
gaagaactcg gccgccgccg tggcgaactg actaatatgg aaagcgacgg caacggacgc 1171320
acccgcctcg aataccatat tccagcgcgc ggcttgatcg gtttccaagg cgaatttatg 1171380
accctgacgc gcggggtcgg gctgatgagc cacgtgttcg acgattacgc gcccgtcaaa 1171440
cccgatatgc ccggccgcca caacggcgtg ctggtgtccc aagagcaggg cgaggcagtc 1171500
gcttacgcct tgtggaatct ggaagaccgc ggccgtatgt tcgtatcgcc caacgacaaa 1171560
atctacgaag gcatgattat cggcatccac agtcgcgaca acgatttggt ggtcaacccg 1171620
ctcaaaggca aaaaacttac caacatccgt gccagcggta ccgacgaagc cgttcgcctg 1171680
accacgccaa tcaagctgac gctggaaggt gcggttgagt ttatcgacga tgacgaactc 1171740
gttgaaatca cgccgcaatc catccgtctg cgcaagcgtt acttgagcga attggaacgc 1171800
cgccgccact ttaaaaagct ggattgatgt ttactggatt aatgtttaaa tgatacgcga 1171860

tgccgtctga aaaatttcag acggcatttt ttattcggac gggctttgcg gcttcttgaa 1171920
gctgtttcag acggcgtttt tcctacccaa tcaagaaact gccgccattt ttccagcggt 1171980
atatcgcccc gtcgcgtttc ggtatcgggt tcggcttccc ggcagcatgt cgaacaatgc 1172040
cgtttgaagg aatacgcctt ttgagtcctt acaggctgag gaagagggta aaacataccg 1172100
caaaacatac cacaaaaaac ggtaacggat agttgtaagc ggttgatgac gattatagac 1172160
aataacgggt tttcccaatg aaattattgt ttgaataata aaaaatccca aaccgtaaaa 1172220
gtttgggatt tgtatttggc agagaggaag ggattcgaac cctcgatacg ctattcacgt 1172280
atacacgctt tccaggcgtg cgacttaaac cactcatcca cctctctaat ggcggaaatt 1172340
atcccaatcg ggataattta ttatttggtg cccgggagaa gactcgaact tccacaccca 1172400
tgaggatacc agcacctgaa gctggcgcgt ctaccaattc cgccacccgg gcaatctaaa 1172460
tattgaaata aagcaaagca tttgatttgg tgcccgggag aagactcgaa cttccacacc 1172520
cgtgaggata ctagcacctg aagctagcgc gtctaccaat tccgccaccc gggctttgct 1172580
ttatttgctg ttttgcggta cagtgtcctg ccgcaaagaa gtcgctatta tatagttcat 1172640
aaagagaatg tcaacagtcc aaatgaataa aaatattaaa tctttaaatt tacgggaaaa 1172700
agacccgttt ttaagtcgtg aaaaacagcg ttatgaacat cctttgccca gtcgggaatg 1172760
gataatcgaa ttgttggagc gcaaaggtgt gccttcaaaa atcgaatcgc ttgcgcgcga 1172820
gctgtcgatt acggaagacg agtatgtctt ttttgaacgc cgtctgaagg cgatggcgcg 1172880
ggacggtcag gttttaatca accgtcgggg cgcggtttgc gcggcggaca aattggattt 1172940
ggtcaaatgc cgcgtcgagg cgcataagga cggtttcggt tttgccgtgc cgctcacgcc 1173000
cgccaaagac ggtgattttg ttttgtatga acgccagatg cgcggcatta tgcacggcga 1173060
tattgtcact gttcgtcctg ccggcatgga ccgtaggggc cgccgcgaag ggacagttct 1173120
ggatattgtc gaacgcgcgc aaagcaaagt ggtcggccgt ttctatatgg ataggggcgt 1173180
ggcgattttg gagccggaag acaagcgtct gaaccaaagc atcgtattgg aaccggacgg 1173240
cgtggcgcgt ttcaaacctg aatccggtca ggtcatcgtc ggcgaaattg aggtttatcc 1173300
tgagcaaaac cggccggcag tggcaaaaat catcgaagtt ttgggcgatt atgccgacag 1173360
cggcatggag attgaaattg ccgtgcgcaa gcatcatttg ccgcaccaat tcagtgaagc 1173420
gtgtgccaaa gctgcgaaaa aaattcccgt ccatgtacgc aaaagcgatt tgaaaggccg 1173480
cgtcgatttg cgcgacctgc ctttggtaac gatagacggc gaaacggcgc gcgatttcga 1173540
cgacgcggtg tttgccgaaa aagtcggacg caattaccgt ctggtcgtgg cgattgcgga 1173600
tgtcagccat tatgtccgcc ctgacgatgt gattgatgca gatgctcaag aacgcagtac 1173660
cagcgtatat ttcccgcgcc gtgtgattcc gatgctgccg gaaaacctgt ctaacggcat 1173720
ttgctcgctc aatcccgatg tcgagcgttt gtgtatggtg tgcgatatgg tcgttaccta 1173780
tgcgggcaat atcaaagaat accgcttcta ccccgccgta atgcgctctc atgcccgcct 1173840
gacctacaac caagtttgga aatggatttc agacggcatc gaccatccgt acaaagccca 1173900
aatcgacacc ctttacaaac tcttcaaaat ccttcagaaa aagcgtttcg aacgcggcgc 1173960
ggtggagttt gaaagcgtcg aaacccagat gattttcgat gacaacggca aaatcgaaaa 1174020
aatcgtcccc gttgtccgca acgatgccca caagctgatt gaagaatgta tgctggcggc 1174080
gaatgtttgc gcagcggatt tcctgttgaa aaacaagcat acggctttgt tccgcaacca 1174140
tttgggcccc acgcccgaaa aactcgccac cctgcgcgag cagctcggtc tgttggggct 1174200
tcaacttggc ggcggcgaca acccgtcgcc gaaagactat gccgcgcttg tcgaacaatt 1174260
caaaggcaga cctgatgccg aattgctgca agtcatgatg ttgcgctcca tgcagcaggc 1174320
ggtttacgaa ccgcattgcg acggacactt tggtcttgcc tacgaagcat acgcccactt 1174380
cacctcgccc atccgccgct atcccgacct gaccgtacac cgcgccatca aagccgtgtt 1174440
gaatcagcaa acctacacgc caaaaaaaag ctggcaggct ttgggcgtgc atacctcgtt 1174500
ctgtgagcgc cgtgccgacg acgccagccg cgacgtggaa aactggctga aaacctatta 1174560
tatgcgcgat aaggtcggcg aagtattcga aggtaaaatc tccggcatga ccagttttgg 1174620
tatctttgta acactggacg gcatccacat tgacggcttg gtgcatatca gcgatttggg 1174680
cgaagactat ttcaacttcc gccccgaaat catggcaatc gaaggcgaac gcagcggcat 1174740
ccgtttcaac atgggggaca gggttgccgt ccgggtcgcc cgtgccgatt tggatgacgg 1174800
aaaaatcgat tttgtcctga ttgccggggg gagcggcagg gggcggaaag ttaaatcatc 1174860
cgcgtctgcc aaaccggcag ggacggcggg gaaagggaag ccgaaaaccg ccgccgagaa 1174920
aaaaacagcc cgaggcggca aagtaagggg aaggggcgcg tctgccgccg cagaatcgag 1174980
gaaaaaggca aagaaaccgg ttccgattaa ggtaaaaaaa cggaaaggca aatcataatg 1175040
ctgacggggt ggcttgagga ggcggggcat attttcccga agcctccacc cgattttttg 1175100
atgtgggcgg acggagcgtt atttccagca aattgaaacg cccgtattga aagattgcgt 1175160
ttatttccac cgccgtttta aaggccggcg gtattcggca gacggggcgc aaacggcgtt 1175220
cagacggcat tttcattctt tcggcgtgtc cgtccgaatt gctttgcccg tccgcgcaat 1175280
cagcccgtgc ggcttgcctt gaacggacaa aaaatgccgt ctgaaacccg aaaatcaggt 1175340
ttcagacggc atttttcctt gaaaaggctg ttcaaatcag cgatggtagt tcggtgcttc 1175400
tttggtaatt tgaacgtcgt gaacgtgcga ttcgctcata cctgcggaag tgatttccac 1175460
aaattctgct ttttcgtgca tttcggcaat attggcgcaa cccaaatacc ccatgctgga 1175520

gcgcagtccg ccggtcagtt ggtggatgat gttcacaatc gggcctttgt aaggaacgcg 1175580
gccttcgatg ccttcgggga cgtatttgtc ggtgctgtcg gttttgtctt ggaagtagcg 1175640
gtcggcagaa ccttggctca tcgcgcccaa ggaacccata ccgcgatagg atttgtatga 1175700
gcggccttgg tagagttcga tttcgcccgg cgcttcttcc gtgcctgcaa acataccgcc 1175760
gagcatgacg ctgtacgcgc ctgcggcgag ggctttggcg atgtcgccgg agaagcggat 1175820
gccgccatcg gcaatcagcg gaacgcccgt gcctttgagg gcttcggcaa cgttgtgaat 1175880
ggcggtcagt tgcggcacgc cgacacctgc cacgatacgg gtggtgcaaa tcgatcccgg 1175940
accgataccg actttgacgg catccgcgcc ggcggcgacc aaatccaaag cggctttggc 1176000
agtggcgatg ttgccgccga tgacttggat gtgcggatag gtttctttga cccaacgcac 1176060
gcggtcgatc acgccttggc tgtgcccgtg ggcggtatcg acgacaatca cgtccacgcc 1176120
ggcctcaacc aaggctttga cgcgctcttc ggtgtcgccg ccggtgccga ctgccgcacc 1176180
gacgcgcaga cggccttcgg agtctttgtt ggcattggga aactcggtgg tttttaaaat 1176240
atctttgacg gtaatcagac ctttgagttc gtctttttcg ttcagaacca aaacgcgctc 1176300
gactttgtgc gtgtgcatca gttcgcgcgc ttcgtctatg cttgtgcctt cggggacggt 1176360
aaccagacgt tcgcgcgggg tcataatggc ggaaacgggc aaatcgacgc ggttttcaaa 1176420
acgcaggtcg cggttggtta cgatgccgac gactttgccg ttttcaacga cgggcaggcc 1176480
ggacattttg cgtttgcgct gcgcacgcat ttccaagact tcgcggatga gcgttgtcgg 1176540
tgcaacggtt acggggtctt tgaccacgcc gctttcgtgg cgtttcactt tggaaacggc 1176600
gcgcgcctgc atttcgggcg gcatgttttt atggatgatg ccgatgccgc cttcttgtgc 1176660
catcgaaatg gcgaggcgcg cctcggtaac agtgtccatc gcggcggaaa gcagggggag 1176720
gttgagtgtg atttcgcggg tgagcttggt ttgaagttta acgtctcgcg gcagcacggt 1176780
cgaatgtgcg ggaaccaaca aaacatcgtc gaaagtatag gctttttcta cgatacgcat 1176840
aatgctcggt ctttcagttt gtgcaagatg cacggcatta tagcacgtta ccggcggctt 1176900
gacagtttat caggtttaat tttggtcccc tttgaatagc tcggttttcc tttgccgacc 1176960
actgttgctc ccgttctttc aatttcagga aaagcttttt tctaattttt ggtaagtggc 1177020
tcagttattg aagccctata tcgggcggta acttccagca caaaaaaacg gagtagttct 1177080
ttatttattt tttcctttaa ttttcagtat attatcttaa tatttcgagg gtaacatatc 1177140
tgctaatcta gttacagccc catatattat agattcaatt gaaaaataac agattcaact 1177200
gtacctttct tatacctgat ttctttaaag tttttcccat tgtcaaaact ataaaaaagt 1177260
ctctcttcct caccagaata tcccatttct aatactaaac aatctatttc acaattaccc 1177320
cctccactca tatatgtagt atcaatagaa aatatattgc ataattccag agaattatat 1177380
ttatctatat caaatagttg ctctccaaaa tctaaaccat tgtttccacc tgcataatac 1177440
ttctcttcaa aggatgactt caaactatta aaaactgcat ttagcgtaaa atatccatat 1177500
gggcatatct taggatatat attctctcct attattatct gaatttttcc ataaataaaa 1177560
ccattacatt cagagttttc ttcccataaa atcccaaatt ttcttgttga gtccataata 1177620
atattcatat aaatccttat attaataaat tatttacaat atcccccgct ttcagacggc 1177680
atacggcgtg gcggcggaat gccgtctgaa ggcgggcgtt atgataattg ttccagcagc 1177740
gtctgcttca atgaggactg gatttttggg tttttcaggt cggggctaaa aacggtaaaa 1177800
ctgtcttcgg cgcgttcatc cagtgtggag attttggcat agcgcaggct gacgttgtgg 1177860
gcgaaaaaga cttccgccat atcggcgagc aggaaggggc ggttgacggc ggtgatttcg 1177920
acggaatacc agtcgggata gtcttcttcg gggtgatggg tgatgctcgg tgcgatcggc 1177980
atatagcggc tgcggcggct gatgcggcgg ctgcggcttt gggtttcggc aacggtgtgt 1178040
ccgtggataa agctgttgag ttcggcttcg agcgcgcttt ggatgtcggg gtagtcttcg 1178100
ggggcgtgct gcgaggggat ttgcacgatg aaggtgtcga ggatgtagtc gtgttcggtg 1178160
atgaaggcgc gggcggcgag gatgtcgaag ccgtggcggc tgaagatgcg gcagaggcgg 1178220
gcgaacaggc gcgggccgtt gggcatgaaa accatgactt gaaagctgtc gcttttgggc 1178280
aggatgcggc tgcggacgat gggggtttca aagtcgtgga ctaggttggc ggcgtgccac 1178340
aggatttcgc gggactggtg gcgggcgaag taggcggaac cgagcgcgtt ccatagtttt 1178400
ttctgctgtt tttcggggac ggcggcgcgg gtgagtaagt cggcggcttc ctgccggcgg 1178460
cggccgaaga gggtgtgcgg gttgccgccg ttgcctgtaa ggtagcgtcc ggcggcatgg 1178520
aagaggcttt ccagcaggct ggcgcgccat gcgttccaca gcttgggatt ggtgccgcgt 1178580
atgtcggaaa tggtcagaag gtagagcgcg ctgaggcgtt cgtgggtttg cacgcgtttg 1178640
cagaaggcat cgagtacgct ggggtcttgg atgtcttctt tttgggcgac ggcagacatc 1178700
agcaggtggt tttcgaccag ccaggcgagc aggtcgcttt cttctccggt caggaagtgg 1178760
tcagcggcaa attggcgcgc gtctgcgatg ccttgtatgg catggtcgcc gccgcgtcct 1178820
ttggcgatgt catggaaaaa ggcggcaagg tagaggatgt cttgtttttc aaaggactgc 1178880
atcagtgcag aggcgtaggg cagctcgtgg ctgtgcatat ccaaggcaag gcggcggacg 1178940
ttgcggacga cggtgaggat gtggtcgtcc acgggataga tgtggaacag gtcgtgttgg 1179000
agcaggccga tgattttttc ccacgcgggc aggtagcggc ccaacacgcc gtagaggttg 1179060
agaaagcgca gggtctgggt cagcccgttg ccgttgcgga aaaaaccggc gaagcggcgg 1179120
cggttttcgg gattttggta gaagctgcgg ttgattttgc gcgtcgcgcc ccaccaggcg 1179180

cgcagggttt gcggttcgag cgcggtaatg tcgttgcgct gctgcatgat ttcgacgatt 1179240
ttgaaaatgt gttcgggccg tctgaaaaaa atatcggtgt gccgcgcggc gatttggttg 1179300
ttgacttgga tgtagtcgtc gtcaatccgc agggtaacgc gcaacggggt ggaggaaacg 1179360
cggctttgca gcataggcgt gaggatgccg cccagttgtt tgacggtttt aatcgcgcgg 1179420
taaaacacgc gcatcagttc ttcgctttgg cggcggaggt tcaagccttc ataacccatg 1179480
ctttcggcga cttgcggctg caaatcgaac agcaggcggt cttcggcgcg cttggcgttt 1179540
aaatgcagat ggatgcggat gtgggcgagg cggcggtagc cgtgcgaaag cataccggct 1179600
tcggcacgcg tcaaaatccg ctgtttgagc aggtcgggca ggtcggtcgc caagccttgc 1179660
gccttcgcta tccaaagcag ggtgtggata tcgcgcagac cgcccggaca gcttttgata 1179720
ttcggctcca atactgcccc cgaaccttgc gatttggcgt ggcggtgttc catctccacc 1179780
agttttgcct cgacaaacgc cgccacattg cgctgcgcgt tcattttttc cgccagttta 1179840
tccaccgttt ggcggttgcc aaacaaaaac ctagcctcta aaaacgctgt gtcccccgta 1179900
atatcattgc gcacgctttc acatagttca tcaacgctgc cgctttttac agacggcatc 1179960
agtttgcagt cccacagggt ttgaacaaac cgggcaatct gttcctgaat gccgtctgaa 1180020
agcggggcag gggagacaac cgccaaatcc acatccgaac agggatacag ttcgccgcgt 1180080
ccgaagccgc ctaccgccat caggcataac gcgctgtttt gaaaatactc tgcccacaat 1180140
gccgccagta aggtttcgac tgccgccgtg tattctctga aaaataccga cacgcggttg 1180200
gctttcaaat aatgcgcttc ggcggcatcg cgctgctgtt tgaaggtttc cagtgctgaa 1180260
gacaggtttg caggcatttt tattctttcg attggcggga aaaagggagg cggatggttc 1180320
ggcggtcaaa taccgctttc agacggcatt tgtcgggtat cggcggaatt ggtcatcagc 1180380
ttcaaccgtt cctgcggcgg cagcagcaac tgatacgccg ccacgcccaa agccgccgcc 1180440
attttttcga tattcgacag ggcgatgttc cagcgtttgc gctcgactgc cgacacataa 1180500
gtcctgtcca aaccgcattg ccgcgccaat tcttcttgcg accaaccctt gttcacgcgg 1180560
aaaagccgca tattgtatgc caataccgcc cgcaaatcct gttcgtcagg cagttcggca 1180620
ggcagagtca atttgttgcc catcatttgc ttccagataa atggttaaag ttaagcattt 1180680
gctgttacgg attttacttc acataaaagc caaaaaaaag gggtggcggc agccacaccc 1180740
aaacacacac acatcaagag gaaagagata aaatcaagac agattgggaa acagcaaaca 1180800
agcaccctga atttcatatc ggtattatca aacaaaaacc cttatcaggt attgattcaa 1180860
atcaatttag attttattcg caaaccgaaa aagaaaaacg gcatatccgt ttatacggat 1180920
atgccgtctg atggtgcggg ggactgctgt atttggcagt ggtttgtgtt ttagtcctct 1180980
tgcgccgctg tttgcaggta gttcggcaga ccgattttgc cgataagctc ttgctgggtc 1181040
tccagccagt ctatatgttc ttcgttggtg tctttttgtt tttccaacaa atcgcggctg 1181100
acgtaatcct gttgcgcttc tgctgtggcg atggcggcaa gcagggcttc gtgtttttcc 1181160
tgttctttgg tcaaatcgca ggcgatgatt tcttcggtgg actcgccaat cagaagcttg 1181220
cccagttctt gcaggttcgg cagaccttcg aggaacagaa tgcgctcgat caaatcgtcg 1181280
gcagctttca tttcaacgat ggactgtttg aagaaatgtt cgcccagttc ttcaaagccc 1181340
cagtttttca aaatacgggc gtgaaggaaa tattggttga tggttaccag cagcaagcct 1181400
aagtttttgt tcagctcgcg gataaccaaa cggtcgcctt tcatacggac tccttttatt 1181460
ccgaatacgg attacagttg gctttggtag tagttgcctt cgccaattaa ctcgatcagg 1181520
cgaagctgct gttccagcca gtgggcgtgg tcttcttcgg tatctttcag ttgggcaacc 1181580
atcaggtcgc gcgtaacata gtcttgagcc tcttcgcaca gtttgatgcc tttttttcaac 1181640
gcatcacgta cttcatattc ggtttgcagg tcggctttga ggcaggaaac cacgtccgtg 1181700
ccgatattca gttcggcgcg tgccattttc ggcgtaccgc ccagcatcag gatgcggcgg 1181760
atgaagtctt cggcgtgtgt ggtttcttct tccatctcgt ggttgagacg ttcaaaaagt 1181820
ttggtgtagc cccattcgga gtagaggcgg gagtggataa agtattggtc gcgtgccgcc 1181880
agctcgccag acagcaattc gttcatataa tcaacaacag cttgattgcc ttgcataata 1181940
tctctctttc tgtaacttgg ttttcggtat gtctgaacag catctgtgtc gctattcggg 1182000
atgcgtgcat tgtatgcaaa agctgtcggc atgacaaatt ttctatttaa aatacaaaca 1182060
attatcaaaa gaaatagggc gtattccccg atgccttcca aatcagtatg ctgtttctta 1182120
tcggtttttt tgctgctaaa actaagaatc catctcatcc ataaagaaca tttacactta 1182180
ttaaccatag caaaaaacaa atagagcacg gttttttatc aaaatttata atgaatcgtt 1182240
ctcattaacc gacagattct tttgagatta tcggattttg ggaataaatt atgccgtctg 1182300
aggggctttc agacggcatg cctgcccgga cagacgctgc ttggagcggc tgtcggtttg 1182360
aaggtgtgtt taatgcgaag ggcaatctgt cgtatgatgt gtttgcggca ggggctttga 1182420
agaagcccgg tattttagga cgaagcgcgc ggctgccggg gtcaatatcc gctatttatt 1182480
ccaaacctta ttaaaactta aatttaaaat catgaagata gaaaatatcg atattatttc 1182540
gccggaactg ttcccgcagg aaacattcaa tgaaaccgaa gcattcggcg ctttggtatg 1182600
gttgtgggca gtttcgccta tttatcagca tgccggcgta caagaagccg ccgtcaatat 1182660
cttgccggta ttgaaaaacg ggcaatttgc cttgttcagc agcaacggac accccgtcgc 1182720
ctactgcact tgggcttatt tcgatgaaga aaccgaatgg caatatctcc aatccaatga 1182780
tgttctgcgc cactcggaaa actggtgcag tggaaacaga atgtggctta tcaactggtt 1182840

cgcacctttc ggcgacagcc gtatgatgaa aagaattctg gtgcatctgt ttccgaaacg 1182900
tgaaataagg tggctgtacc accgggggag tgaaaaagga aaacggatta tgaggtttcc 1182960
agcgttgtcg aaacaataaa acaaggctgc ctgaaagttt cccttcaggc agccttgttt 1183020
cagttctctc gaggccattc gtatttttg ttttcgggat aaatgtcgtc acctacacga 1183080
taccaacctc tttttctcat gcaggcatct gctttacttc ctcctccccc acctattggg 1183140
tcatagccgc actctttcca gtcttttttt ccttagcaaa aagatcatct atttgtttgc 1183200
tatattcttc ataagaatat atgcgtaaat tgatattttt actcatcatt gccggatatt 1183260
cttgttctat acgggaatac ttccagtata ccgagtcatc gggcggaggt ttgaatcccc 1183320
caaacgaaca ggcagccaat cccatagcca aagagattga tacgatatat ttcattttgt 1183380
ttttccttct tctcgaggcc attcgtattt tttgttttcg ggataagggt cgaaaccctt 1183440
acgacaccag cctttttttcc tcaggcaggc atctgcttca gactttccgc cgtctattgg 1183500
gtcatagccg cactcttgca tgtctttcag ttgccttcgt cttgctttcg atggatattg 1183560
gtcaagtgcc gccgaggctg atcccggata gggattggcg taatttttca attcccaaaa 1183620
tgacgcggcg tcccacggat ttggtttaaa tccccccaaac gaacaggcag ccaatcccat 1183680
agccagagag attgatacga tatatttcat tttgtttttc cttcttctcg aggccattcg 1183740
tattttttgt tttcgggata aatgtcgtta cctacacgat accaacctct ttttctcatg 1183800
caggcatctg cttcactccc accgccccca cctattgggt catagccgca ctctttccag 1183860
tctttttttt ctctagcaaa agaatcatgc agttgtcttc gtctctcttc aggtgggtat 1183920
tggtcaagtg tcgctgaggt taatcccgga taaagtttgg cgtaatttgt caatctccaa 1183980
aatgccgagt catcgggcgg aggtttgaat cccccaaacg aacaggcagc caatcccata 1184040
gccagagaga ttgatacgat atatttcatt ttgtttttcc tttcggatgt tgcagatcat 1184100
aaatgcgtac aggacggtac ataatttcga tttcgttgga cactcctctt tcaaatacac 1184160
ctgtcggact ttgggtttgt ttattttcag ggtctcgata accatctgtt acacattgtt 1184220
tattagccat accgtagcaa ttatgcggcg aggtgtaatc ccctataata tcccttatag 1184280
cctgccattg gctttttttgc cgggtattgg ttccgactgt tgccggattt ctgccaatca 1184340
gcatgccgat aaggtctaat tcgtggtttt ctatttggat ggactgacgg gcgaaatctg 1184400
ccgttctggg tgtcgttacc ccctgctgca gttgaaacag ccttttatct gcccggacaa 1184460
cgttggcggc agggccgacc attttcagtt cggtattgga taaaattttc ttatcccgat 1184520
tggcttgggt attcaaggta ttgagtacat tgtcgaccac cagggttcca cggctgtgcg 1184580
agccgacaaa caggccgttt ttatccttcc cgtagtcttc catgatattg cctaaagcca 1184640
agcctgaatt gctcaagccg ataacggtct tattgcctat ttttgcccct tcgagcattt 1184700
tatgaaaggc cgcaacggcg atttcgggca attttgaaaa gccccgccca ttggtttccg 1184760
gattgtgcag gaaataaaca ttttcatagg tgcgttcata tcggtttttc tcgggattga 1184820
aacgccccac atattgttgg gcggcaaatt tggcggctgc ttgtatatta ttgaaaatgc 1184880
cgttgacgcc gacaacgatt ttttcaacgg ttttgccggt ctccgggtcg gtgtaacggg 1184940
cagttttcag atttttccgt tcctggtcgg atacttcgcg caattcgtag atatttcccc 1185000
caatggcttt ataggcttcc caataatctt taaatgaggc aaattctttt ttcaacttta 1185060
ctgagttgtc aaactgttta tttatatcct cttcaagctg cttatcttca atcggctcgc 1185120
cgttttcatc cattttaaag gtcatcaggc ggtgttctgc aataaactgg ctgcggtagg 1185180
cttcgtctgc aatgccgccg atcagcgtct cattgataaa ctctttggca acatccctat 1185240
tgagttcgac ttctttcagc aagccttcgc ggtctgcttt tgccaatgcc ttatgtgcgg 1185300
atgaagtgcc tttttcaata ccggcaatat ttttcctgcc ctgcgcggaa gcaatttgcc 1185360
aatctccctc actgatgacg gagcgggtaa tggattggcg gctttctgat tctttggcat 1185420
tgcccaacag attgcccaaa cccatttttg ccaaggcgta tttgtcgttg ctgtgtatat 1185480
cgttttgctt caaaccgaat ttcaagccgc cggcgttgtt ttgatttaaa tttgcctctt 1185540
taaaggtttc cccttttttca atacgctcac ttcgatattt ttcgttcaac tcgtcgtctt 1185600
tcgccgaaac ctggtcaaaa cgcatcaggc tgaccggttt gccgccgatt ttaccgattt 1185660
ctgcttcttt tttggtggaa tactgtccgc tggtaggctt cgggctgtat gaaaaaccgc 1185720
cgctcaagcc caaggcggaa gcagccgccg aagcatggtt ttgaatatct ttatgccaga 1185780
tttcgcttgt tttcagcagg ttttttgatt tgtcggcatc tgaaacaaca gcggcgccga 1185840
ccaatccggt tttgccgttt acgcgaatcc gatagccgtc tcctcctgca aagataccgc 1185900
tttgctcgtt gacggatgca taatccgagc tgcttttcga gcggttatag ctgccaccga 1185960
cactaaagcc gtagcctacc gtaacttggg cggaaacatt ttcctgtttg cctttaaaca 1186020
cggcggtatc ctgcaaactt tcgatatgca gactctctgc cgttacgcca acgcctttgc 1186080
ctttaagctg cccgcctttg atgacggtat cgccaccgct ttcaatagcg gtttggctgt 1186140
ctttgctgcc gatatggctg ttgcggtagg cggtttcgtc gccgttgccg taacctttgc 1186200
cgtagtttgc tccggctgtg aagccgaaac tgatgccttt gttgatggcg atggcgactc 1186260
cggcattaaa gcctgcggat ttgttttcgc tgcgttcctg atgcgtttgg cgggcggctt 1186320
caatctgaac ggcattttct gctttgaggc gtgttccttt gccgccgtac acatcggagc 1186380
cggtaatcgt gatgcgggag tctttgcctg cgcctgaagc agtcagggaa actttgccgc 1186440
cgccggtgat tttgccctct tgcacctgcg tgcctttgat gcggctttcg gaggtgttct 1186500

tctgttcgcc gtaggtaacg gagacactga tgccctgacc ggctgctttt ttgggatttc 1186560
gggcggcatt atagagtgcc acgccggaat ctactccttt attcaaggcg ttggcagcag 1186620
ccatggcatt gacccggctg tttttgcttt tgccgacggt ttggactgct tttacggcgt 1186680
caaccgcgcc cattacggta ttcacaaccg gaacgctaat ggcgacggtt acgccttttt 1186740
gttcgtaaac ctgcttactc tcttggctgt aacggttttg tgcggcatcg atgctgattt 1186800
ttccggagga aatgccgaca tcgccttggg gcgaggatat ggtcgaaccg gtttgggtgt 1186860
aatgttttcc tgccgaaatc agggtattgc cgttcaggct gccgacgacg ctttctgtgt 1186920
ggctgacggt ctcggatcgg ttggtttgcg tgtctttttt gctgcccgcc gtaaagccga 1186980
tgccgccgct gcccatcagt ccggattttt ctttttttgtt catttcggca ctgcggctgc 1187040
gtgtttcggc tgctttaagg acgatattgt tttttgccga gagaatggta tggttgtctg 1187100
caatgatatt gctgccagta acggtaatat cgcgtcctga aaccagaatg atttctttgc 1187160
cgtccagcgt gccggatacg gcttgtccgt tttggttctt gagatggcgg gtcatcttct 1187220
gtttgatgcc gcccccgctt ctaccggtgt atttcagggc atcttcggtt tcggtatggg 1187280
ctttgccggc ttcgactttg atatcccgtc cggctgccag tttcagacgg ccttgttcgc 1187340
tgccgacctc tgctgcacgg atacggatgt ctccttttgc attcagactg agattgcccc 1187400
gggtgcggat ggtgctgccg acttcgtttt gttctttgcg aatcacatag ttgtcggaat 1187460
caaagatagt gttctgattg cgggaaatgc ccgtcgtatc cgagcggatg tcgccgccgg 1187520
cattcagtac ggtttgaccg tcttcagatt gattggtcaa ttcggaagcc gtcaggacga 1187580
tattgttgcc tgcatccagc aagacgcttc cattctgcct gccggtcaga taaatgcctg 1187640
ccacccggcc gatattgcgt accgagcctt gctcattctg attgctgcgg gtctcgctgc 1187700
ggctttctat attgttactc gctttgagca gcagggcgtt ttctgcgccg atgctgccgg 1187760
tattcgtaat gtcgctgcct gctgcggcga agatgttttt gccctgcaaa tcaccttgca 1187820
gatttttaat attctgtgcg tttaaaatta gtgcttcgcg cccggcaatt aagccgcccc 1187880
ggttttcaat ggcgccgctg ccgatatcaa caacgctgcc ggacagcaac gccccttgtc 1187940
cgttcatatc tttggggcgt gcgcggacat agactttggg tttcaatacg gtttgagttg 1188000
tcccgtcggg cagggtaacg gtctcgtttt ccagccaaac aatgtcggaa gtcagacggg 1188060
caacctgttc ggcagacagg gcaatacccg gagtaagctg caattctttg gctatggtaa 1188120
tgccgttatc catcaaagcc ttgaattgct cttcgtcatt ggtataaccg tccaagcggc 1188180
ggtagcctgt cagcttggcg atttgttcgt ttaccagttt ctgctcgtaa tagccgtcgc 1188240
ccaaacgctt gtggatatgg ttcgggtctt gttgcagtgc ggcaagcata tagccgctgc 1188300
ccagccattt gcggtagtcg gtaaaggcag ggtcggtttc aatcaaatag cctttgttgt 1188360
ttggcgcaat ggcaaacaag ctgctgttcg gcagagtaaa tgtaggatgg atgccgtttt 1188420
cagcaacaac gggtacaaca gtgccgggta tatcagatgc ctgttggggc gcatagcctt 1188480
tataggctga aatacccata cggatggagc tgacttcggg ggccggttcg tagggagacc 1188540
gactgtatcc cgtagaatcc cgccctttct tgtgatgacg gtggtaacgg tgcaagtccc 1188600
ctttatcggt tatggttttt gttcccaaag tttcatcatt gtccaatgcg gattcgggcg 1188660
taccgacgat caatctgccg ccggcaataa tccggctgtt atgatttttc agcttggcgg 1188720
catctaaaac caaattgccg ccactgatga tttgaccggg cttggattcg gtaattttgc 1188780
tctcggcggt gattcgttcg taatcgtatt tgtaccagac agagtggggg ctgccgtccg 1188840
gagtccgcat atgtaaagac tcatcttcaa atatttccca gcccaactct ttttgagaac 1188900
cttccggata gcgttctgtt ctgccttccg cctgatattc gatacggtgt tcgcgatggg 1188960
tttcttccgt atggaaacgc agatgctcat tggtattctg caaatctttt gtagcgatac 1189020
ggatattgcc tgatgattcg attgccgcac tgcggttgtg cagtgatgta tttgctcctt 1189080
gcacctgtcg gcttccattc aatgcagaac cgatatgaag atcgccggag ctggacaata 1189140
atgccgcctc tcggttctca atttcccgcg ctccaatatc caaccgctcc cgcgctgcaa 1189200
ttaccgccgc tttggtttcg ccgttgaccg tttcttcccg gttcaacaag gtatctgcct 1189260
caactgccac acggctgccg taaattctgc ccgtgccggc attgtccgac ttggcttcgg 1189320
tttgcagcag cgttataccg ttgctgttga ttaaacccct gttggtgatg ccgtttttgc 1189380
cgtttaaccc ggtgcggttt ccggattgga ttttaccgga gacagtgttg tcgatttggg 1189440
cggcggtcag ggatacggta tcgcctgcct gtatgatgcg ggtgtttttc agacggcctt 1189500
gggtggagaa cgtgagtgtg cgtccggctt caatatcgcg tttgccggca aaatcatcat 1189560
gaagagaaac ggaaacatcg cgtgcggcgg ttaatatgcc gtcgttgtcc agtgattttg 1189620
cctgtaagga aacatctttg cccgcaataa tcgtgccgtc tgtgttattg atatgcaggc 1189680
tgcttttgcc tgtatcgcga atatccagca aaccggccga gccgattaag cctgcttgat 1189740
tatttatgcc gtctgaaacc ttcaatacac tgcctttgcc gctgcggata aagccttggc 1189800
ggttatcaac ggtttgagcg gaaatggtga tttcggcagc atcaatcgaa ccgctgttgc 1189860
tcaatttgcc gtctgcgctt aacgtaacgc cgcctgttgc ggcaaaaatc cgacccttgt 1189920
tgcggattac ggcgccgttg tcggtgctga ttaaagtgat tttgtctgcg tacatcccac 1189980
ccagtgtggc ggtgtcgatg gcaacggtag gagtaacaga atccgaagaa gatggcgcag 1190040
aagctgtttt ggcaagagag ccgtcaaaat ccaatttgtt cttacccgaa accaccttga 1190100
catctttacc ccaaacgccc gcattgattt cagcagcacg actaaggata cgggtgtaat 1190160

cggcatcaga ggtatccaaa cctttgcccc caatcacgac tttacccgaa gaaacatcaa 1190220
agcccgtcag attgccgtta ttcaaaacag gaacgcccga agtcagcgta accgaagcgg 1190280
cattaatcaa tccgccgcca ttcacacgga tgcccgacgg attggcaacg actacttcgg 1190340
cgcgtttgcc gccgacttcg atataaccgt tcaacaacga aggattactg ctgtcaatct 1190400
ggttcacaat tacccgcgct tcgccgcgtg ccagatgggg attgccttga atccatccac 1190460
cgagttgcgt ttgcgtattg ctgcggctgt tgtttagtat tacgcctttt tcatcaacat 1190520
cgaactgctt gaatcggtta acagaaacgc cttgggatga cggagtttga atattgactt 1190580
gcggcaaacc gtttgctgtc tgaagaataa cggcttgttg gtttttaggg gcggatttgt 1190640
cggcaatgat gccggaagca ggggcagggg aaaacgcagc aacacccaaa gccaacatga 1190700
cagaaaaggc agccatacgg aaaccgaagg ctgcccgggc agaagaaaca gaagcggcac 1190760
cggtcactcg aaccgaagcc gcctcactat cctgcatact cttgccgtca cgatgaacat 1190820
tctctgctac agccatcata caactgcgtt tcttgttgaa gataaccttg tagcatcgct 1190880
tgttcatgat gggttttctt aaatgaaatg taaggaatag ttaaggacaa aatataggaa 1190940
atttgaatta aattgtcaat aaaaaactga cagcgtaccc tgataatcag atgttgtacg 1191000
ctaattgtga gaagtatgtc gggatgatta ttttgaagtt ttctttttat tcaaaagagt 1191060
tactatgaaa gttaaccggg cgtaaatagg cttcaataaa caggttatcg ttatttttca 1191120
attgggaaaa aatcttccct aacccatcca ttcgccaaat tattatgtgg gaattcggca 1191180
atttcaataa cggctgttga tccatatctg ctcggtcatt tagcgttata aagtcgcaaa 1191240
tagcgtcaga atttacaaga ttatcggatt ttgggaataa attatgccgt ctgaagggct 1191300
ttcagacggc ataggcggct aacacgggtg cggcttgcgc caaacggcgc ggcaggcgca 1191360
gggaatcgct ttttactttg gcgcggtgtt ccgggtttaa tgatgcccgc aaccgccgtg 1191420
gctttccctc aaggcttccg ccgcctgttc gtcggcgtgg tagctggaac gtaccatcgc 1191480
gccgatggcg gcattgctga agcccagttc gtatgcttct ttttcaaaga ttttgaactg 1191540
ctcgggcgta acgtagcgca ggacgggcag gtgtccgtct gaaggctgga ggtactgtcc 1191600
gatggtaatc atttcgatat tgtgcgcccg catatcgcgc ataatttcac gcacgtcttc 1191660
gtctgtttcg cccaagccga ccatgatgcc ggatttggtc gggatgtgcg gcatcatttc 1191720
tttataacgt tttaataagt ctaaagaatg ttgataattg gcaccgggac gggcttttct 1191780
gtacaggctc ggatgggttt ctaggttgtg gttcatcacg tcgggcgggg tttcggcaag 1191840
gattttgagt gcgatgtcca agcgtcctcg gaagtcgggg acgaggattt cgattttggt 1191900
gttcgggctg gtttcgcgga tggctttgat gcagtcggcg aaatgctgtg cgccgccgtc 1191960
gcgcaggtcg tcgcggtcga cggaggtgat gacgacgtaa cgcaggttca tggctttgac 1192020
ggattcggcg aggtttctcg gttcgtcggg gtcgagcata ttgggccgac cgtgtcccac 1192080
gtcgcagaac gggcagcggc gggtgcagat gtcacccata atcatgaagg tcgccgtgcc 1192140
tttgctgaag cattcgccga tgttggggca ggaggcttcc tcgcaaacgg tgtgcatctt 1192200
ttgttcgcgc aaaatgtctt tgatttcaaa gaatttgcgc gatgggagtt tggcgcgtat 1192260
ccattcgggc tttttcagtt tttcctgaag ggggacgact ttgatgggga tgcgcgcggt 1192320
tttgtccgcg cctctgagtt tgatgccgcg tttggggtcg tcggttttga tttcactcat 1192380
tgttgtctgc tttcggtgtg aattgtgttt caaggtgtgc ggtgagtttg gcggcgactt 1192440
cgtccggcgt ggggcagggt tggacaaaat ccgcgatttg cgtcatttcc ataccggcgt 1192500
agccgcaggg gttgatgtgg gtaaacgggc ttaaatccat attgacgttg agcgcaagcc 1192560
cgtgatagac ggagccgttt ttgatacgca gccccagtga ggcgattttg cgttctccga 1192620
cataaacgcc ggggcgtttg gggtctgccg ccgcttcgat gccgtattct gccaatgtgg 1192680
cgatgatgct gttttcaagc gcggaaacga tgtttctaac actggttttg cgccgtttga 1192740
aatcaatcat cgtataaacg accaattgcc cgggcccgtg ataggtaatc tgcccgcccc 1192800
ggtcgatttg gacgacggga atgtcgtcgc gaatcagcag gtgttcgggt tttcccgcca 1192860
gtccttgtgt gaacacgggc gggtgttcga cgacccacag ttcgtcttcg gtgtcggcat 1192920
tccgtccggc attaaaggtt ttcatcgctt caaaagtcgg cagatattcg accaaacctt 1192980
tgtgtatgat tttcatctca aagtaccact ttgaccagtt cgtgcgaagt cagcgcacgg 1193040
tagatgttgt ccaattgttc ttggttttca acctttacct gtacggtggc gccagtatag 1193100
ttgcctttgc tgctcggacg cgtggtgatg tggtgcgcct gcgtgtcggg ggcgtggagg 1193160
cggacggtgt ctaaaaccgc ctgctcgaac tcgggatgca ccgcgcccat tactttcaat 1193220
gggaaggtgc agggaaattc gatgagggat gttttgtttt tttgttcggt catgatgtgc 1193280
tgccttgtcg tgtacggtat gccgtctgaa ggcgggtttg cctttcagac ggcatcggat 1193340
gtgcgttatt ttagcctaaa ccgcgataac aggctatcgg gaaggcggag gctttttga 1193400
cggcgcggcg gttctgctat actggcgcac aatattattt ttagaagggt ggtttttatg 1193460
tatcggagga aagggcgggg catcaagccg tggatgggtg ccggtgcggc gtttgccgcc 1193520
ttggtctggc tggttttcgc gctcggcgat actttgactc cgtttgcggt tgcggcggtg 1193580
ctggcgtatg tattggaccc tttggtcgaa tggttgcaga aaaagggttt gaaccgtgca 1193640
tccgcttcga tgtctgtgat ggtgttttcc ttgattttgt tgttggcatt attgttgatt 1193700
atcgtcccta tgctggtcgg gcagttcaac aatttggcat cgcgcctgcc ccaattaatc 1193760
ggttttatgc agaacacgct gctgccgtgg ttgaaaaata caatcggcgg atatgtggaa 1193820

```
atcgatcagg catctattat tgcgtggctt caggcgcata cgggagagtt gagcaacgcg 1193880
cttaaggcgt ggtttcccgt tttgatgagg cagggcggca atattgtcag cagtatcggc 1193940
aacctgctgc tgcttccctt gctgctttac tatttcctgc tggattggca gcggtggtcg 1194000
tgcggcattg ccaaactggt tccgaggcgt tttgccggtg cttatacgcg cattacaggc 1194060
aatttgaacg aggtattggg cgaattttg cgcgggcagc ttctggtaat gctgattatg 1194120
ggcttggttt acggttttggg attggtgctg gtcgggctgg attcggggtt tgccatcggt 1194180
atgcttgccg gtattttggt gtttgtccct tatctcgggg cgtttacggg attgctgctt 1194240
gccaccgtcg ccgccttgct ccagttcggt tcgtggaacg gcatcctatc ggtttgggcg 1194300
gtttttgccg taggacagtt tctcgaaagt tttttcatta cgccgaaaat cgtgggagac 1194360
cgtatcgggc tgtcgccgtt ttgggttatc ttttcgctga tggcgttcgg gcagctgatg 1194420
ggctttgtcg gaatgttggc gggattgcct ttggccgccg taaccttggt cttgcttcgc 1194480
gagggcgtgc agaaatattt tgccggcagt ttttaccggg gcaggtaggc ggttccgaaa 1194540
catatagtgg attaacaaaa atcaggacaa ggcgacgaag ccgcagacag tacaaatagg 1194600
gcaacgccgt actggttttt gttaatccac tatatttgaa gcggaataca accttgcccg 1194660
ggtttaaata aaaatgccgt ctgaaccccg aaaactggac ttcagacggc attttcatca 1194720
cggcttattt ggcggttttg ctgctgtcga taattttcat accggcagaa atcaggctgc 1194780
cgatgtcggc aacattggcg ggcataatca gcgtattgct ttctttggca agattgttga 1194840
acgcagcgac gtattgttcc gcaatcttca gattgaccgc atccgcaccg ccttgggttt 1194900
gaagggcggc ggcaatttga cggatggctt cggcattggc ttcggcaaca aggcgcaagg 1194960
attccgcttc acctttggcg cggttgatgc gggcgatttt ctcggcattt gacgcattga 1195020
ccgcagcctg agcctcgcct tcggattgtt ggatttcggc ttcgcgctga ccactggcaa 1195080
ggttgatttg ttcgatttta cgaccttcgg attcggcgat acgggcgcgt ttttcgcgtt 1195140
cggcagtaat ttgcgcctgc attgagcgaa ggatttcttg cggcggaacc aagtctttaa 1195200
tctcataacg caaaaccttc acaccccaag ccccggccgc ctcgtccaaa gccgcaacaa 1195260
cagtactgtt gatttcgtcg cgttcttcaa acgttttgtc caactccata cgcccgataa 1195320
cggaacgcag cgtcgtttgg gcaagctggg taatcgccat aatgtagttg ctcgaaccgt 1195380
atgaggcgag tttggggtcg gttacttgga aatagatgat gccgtcaaca gtcagctgcg 1195440
tattgtcgcg cgtgatgcag acctggctgg gtacgtctaa agggatttct ttcagcgaat 1195500
ggcggtaggc gacgcggtcg ataaagggaa tcaaaatatt caaaccggcc gtcagggcgc 1195560
gatggaaacg ccccagcctt tcgacaacgt ggacttcctg ttgtgggatg acaacaaagg 1195620
atttgaaacc gaaaacggcg acggctacca acaagataat gaaaaattcc ataattcctc 1195680
cgagtgttaa gggtgtgtga taataagaag gttgccttcc ttgcggacaa tgagggcgcg 1195740
agttcctggt tcaagctctt cttgccccgt attttgagcc tgccagtgcg taccgcgata 1195800
aaaaacttcg taacggttgc cgcctgtgtg tcggaggatt tcgacatatt gtccggcatc 1195860
caaatcctga tatgaatccg tttcaacttt tctaacggcg gttttggcgt gtacgaacca 1195920
aatacccagc gcggaaagca gagcggcggt caagacggcg gcaggcgtac tgccggtcag 1195980
cccgtaagca atgcccgaac ccgccaaagc cgcgctgaca accaaaagat aaaccgttcc 1196040
cgtcaataat tcgatgatta agacggcaac agcggcaaca aaccatacag tcatacattt 1196100
ccccacaaag cgcgtcgttt gacaaaataa cgcaatatca gcagtatagc cgaatttgaa 1196160
aggatagggc agatatggac acttggcacg atgcactcgg cggcgaaaaa cagcagccgt 1196220
attttcagga aattttaaat gcagtcaggc aggaacgttt gtcgggacaa atcatctatc 1196280
cgccggcggc ggatgtgttc aacgcattcc gcctgacagc gttcgaccgg gtcaaagccg 1196340
tcattctcgg acaagatccg tatcacgggg cagggcaggc gcacggtttg gcattttccg 1196400
tccggcaggg tatccgcata ccgccgtctt tactcaatat ctacaaggag ttggaaaccg 1196460
acatcgaagg cttttccatt cccgcgcacg gctgtctgac agcgtgggcg gagcagggcg 1196520
tattgcttct gaacacggtt ttgacggtgc gtgcaggaca ggcgcattcg cacgcccttt 1196580
taggctggga acgctttacc gataccgtta tcaggcagct tgcgacacac cgcaagcacc 1196640
ttgtcttcat gttgtggggt gggtatgcac aacaaaaagg gaggctgata gacagtcaaa 1196700
atcatttgat attgaccgca ccgcatccgt ctcctctgtc ggcatatcgc ggttttttcg 1196760
gctgccgcca tttttcacag gcaaacagct atttgagccg gcacggtatc gatccgataa 1196820
actggaagct gtgaatgccg atatagccgt tgccgccggc gtgttaaaat cgcgtttgat 1196880
ttgtaatttc catttattag gcaaaacctt atggcaatca aaaaaaaatc agctttatgc 1196940
atcactttgg gcgggctgcg acgagttgcg cggcggtatg gatgcgagcc tttataaaga 1197000
actatgtgct tacgctattg tttttaaaat atgtttctga taagcataag tacggcggcg 1197060
gcatgattga gctgcacgcc ggtacgactt ttgacgacat cgtcaaactc aaaaacaccg 1197120
ccgacatcgg cgaccgcctg aataagatta tcgcccaaat tgccgaagcc aacgacttaa 1197180
aaggcgtgat cgacgttacc gacttcaacg acgaagacaa actgggtaaa ggtaaggaga 1197240
tgatcgaccg tttgagcagg cttgtcggca tttttaaaaa gctcaacctt tcttccaacc 1197300
aagccgaaga cgacgatttg ttaggtgatg cctacgaata cctgatgcgc cattttgcga 1197360
ccgagtcagg caaatccaaa gggcagtttt acacgcctgc cgaagtctcc cgcattatgg 1197420
cgaagattat cggaatcagc gcagattgcc gtccagcacc agcgtttatg acccgacctg 1197480
```

cggctcgggt tcgctgttgc tcaaagccgc cgcccaagcc ggcagccaaa tcagccttta 1197540
cggtcaggaa aaagatgtgg caaccgcgtc ccttgcccgt atgaatatga ttttgcacaa 1197600
caacgaaacc gccgaaatca acaccgggaa caccttgtcc gattcgtctt tccgtgatga 1197660
aaacgacggg cttaagacct tcgattttgc cgttgccaat ccgccttatc ccgcccgaaa 1197720
aaaacggcga ttacgccttt ttgctgcatc tgctcaaaag cctgaaacca agcggcaaag 1197780
gtgcgattat tcttccgcac ggtgtgctgt ttcgcggcaa tgccgaagcg cgtattcgca 1197840
cggaattgct taaccttgac cttattaaag gcattatcgg gctgcctgcc aacctgtttt 1197900
acggcacggg cattcctgcc tgcatcatcg tcatcgacaa agaacacgcc caaaccgccc 1197960
aatttgccga agagggaaca aaccaagtta tcagcggcgg cagcgtgttt atgattgacg 1198020
catcgcgcgg cttcattaaa gacggcaaca aaaaccgtct gcgtgagcaa gacattcaca 1198080
aaatcatcga cactttcaca aacctcgtta cagccgtatg gtgcatttaa gcgaaatcgc 1198140
agcacaagat tacaacctta atctgcctcg ctatatcgac agcggagaag tcgaagacct 1198200
gcaaaatctc gccgcctatc tatctttatg gcggcatacc tgcgcacgat atggacgcat 1198260
tggaagccta ttggcaagtt ttaggccgta tgaaaaacga gttgtttgcc gaacacgatg 1198320
gccactttac cactatacaa cggaatcgat tgcaaatctt tcccactctc aacagcttaa 1198380
aatcctgcgg gattggtgtg gaatttaggg ctaatctagt acagccccaa atttaatcca 1198440
ctataaaatc gaaagcagcc aaatcaaagc ccatatattg gcgcaccccg attacgccgc 1198500
cttcaaagcc ggacacctag caaagtttgc cgcgtggcac actcaaaacg accttgccgc 1198560
catccaaccg ggcaggctta tccggaaatg gagcgaaagc ctgctggacg cgttcaaacc 1198620
cggcagcctg attgaagaat acgatttcta ccaaatcctg acggactact gggcggaaac 1198680
cctgcaagac gatgtttatc tcatcgccca agatggctgg aaggcggtta aaaacctggc 1198740
cgaaatcacc aaagaaagcg atgaagccgc gaacctgacc gtcgtctttg aggaaaccga 1198800
aaccgacaaa aaaggcaaag ccaaaaccaa gcgcatcagc aaaaaatacc gcagcgaagt 1198860
catcgccccc gagctggttg cccgccgcta cttttcagac ggcatcgcca agctggaaga 1198920
aaaacaaagc gagctggaac gcctaagcca agaattggaa aaccacatag aagaacacgg 1198980
cggcgaagag ggtgcgctga acgacgtatt ggatgcaaaa ggcaaacttt ccgccaaact 1199040
tctgaaaacc gcattggaag aaagcggcat agaagaaggc gaacgggctg ttttacaaac 1199100
cacccaaaca ctgatgacgc aggaaaaagc cgcgaaagac gcagtcaaaa cccaaatcga 1199160
agccctgaac cttgccgtat tcaaacaatt tggccgactt tccgaagccg aaatcaagca 1199220
gcttgccgtt caagacaaat ggcttgccga tttacaaagc cgaatcgaaa atcgcttgga 1199280
aaacagtatt cagcagctta tcagccgctt gaacacgctg gaagaccgct accgcagccc 1199340
gatggccgag cttgcccgag aagtggaaaa gtggcaaagc aaagtcaatg cccaccttga 1199400
aaatatgggt tttggaggct gaaatggcag cacagacagg ctataaggcg agcgggtttt 1199460
gagacctttg caaaattccc caaaatcccc taaattccca ccaagacatt taggggatcg 1199520
cggttcgggt gtccgcaccg cttaatacgt cgtcgtccac gaactgaccc atttgctcga 1199580
acgccatcgc aacgcccgtt ttatggcgca tatggacaac tttctcccaa actggcaaag 1199640
catcaaacaa cagcttaatg ccttggagtt atttgcacaa atatataatt taacataata 1199700
tacattatgc gaactatcgg aaacagttgt acgtgtccct gtggtctttc caagtaggaa 1199760
aattaaagta tggccaatgc ggctgaatgt atagcccgga gcatccgcgt atccgaaggt 1199820
actgaacgac atgccctgca tccctttcga cgcttccatc cagccggtat tgggaaatac 1199880
gttgaccttc aatataatat acaggcagcc gacacacagg atatgccgct gctttttcat 1199940
catttcttct gtcaaatcct tggaacggtc gatttgaaaa cacggcttta catacgcccc 1200000
agttcccttt gcagggcttg aatattttgc tgcctgtcca atacattgct ttgtaatgca 1200060
tttatttctt gatggttgat gttgccgccc tttgccagac gtgcttgtga taacattttt 1200120
tgggcttcaa ccaatgcttt gcgttcgttg ctcaattctg tttcgagaat ggagcgtctg 1200180
ctgttgtttg agggtgcttg ttgcggcggc ggcgtattgg attttgccgg cttggatact 1200240
gttttgaccg gggctttata tttgacaacc tgtccgccgt ttgacggtga tgataccggt 1200300
tcgggcgttt ggggcgggat atagcgttcg ctgctgtagt tgccgattgg gggcaaatcg 1200360
gttgagtggc agcttttgga cggcttggtg gtgtaaacgg tttctccgtt gattgtgcag 1200420
gtgtagattt tggccgcatt cgcacccaat gggcttgaaa tcagggaaaa gttgattagg 1200480
attaagagga gttttgattt cataatgtgc ttgattttcg gataatcatt tgattttttg 1200540
gtatttttttg taatgctttc ttggcgtttt acgggctttt cttttcggtt ggttgctttt 1200600
gattttcttt cggggctttg gcagcctgct tgttcttttt cccggcaggt tttttgtttt 1200660
tctttttact ttctatgtgt attttggctt cttaactgag ttttttaatt ttcaggcggt 1200720
atccgcctcc ctgatggctg ctgattttag gtaaatccgc atcggcgcac aatcctgctg 1200780
gggctgatgt atatacgctt ttgccatttg agttgcaatg gtatacggag gcttgcgccg 1200840
ccgcgccgga aagggacagg agacccaagg cggcaaaaag tctgatgttt ataccggtaa 1200900
aaggcggtga taacgcccga attataccgt tattggcagg caaagataag caccctgccc 1200960
gcgcttcttt atcgctcggc aaactgtttc tcgatttcag tttcaatctg ttccaattct 1201020
tcttggcaag ccagccaaac ctcttcgatt tgggcaagtt gtgttttgac ttttgccagc 1201080
tcggataagg tgtcctgcaa tttttctttg ttttcctcga agtaagcttc ttcttgtgct 1201140

aaaaatgctt cacatgccgt ctgaatttcg gaaagctgcg ccatttcttt ttcggcacgg 1201200
tctattttct gctgtatcgg cttgccgcgt cgggcttttt cctgacggat ttgcgcttcg 1201260
atgcgcttgg tgtctttgcg gctttggctt tgtgcggatg ctgcgggcgc gacggcggcg 1201320
ttttcctgtg ccaaacgcca ttggcggtag tcgttcaaat cgccgtcgaa gttcttcaga 1201380
cggcctttat cgatcaggag gaagctgtcg gtcgtggctt caagcaggct gcgatcgtgc 1201440
gatacgacga ttaaggcgcc ttggaaactt tgcagagcga gcgtcaaggc gtggcgcata 1201500
tccaaatcca aatggttggt cggctcgtca agcagcagca ggttcggctt ttgccagata 1201560
atcatggcaa gagcgagtcg ggctttttct ccgccggaaa atggttcggt tttctgcaac 1201620
gccatatcgc cgacaaaatt gaagcctccg aggaaatttc ggatttcttg ttcgcgtact 1201680
tcgggagaaa gctgctgaat atgccaaaca gggttttggt cggagcggat ggtatcgagt 1201740
tggtgttggg caaaatagcc gatattgagt ttttcggaac ggacgatgct gccggagagt 1201800
aaatcgattg tgcctgccaa agctttgata aaggtagatt taccgctgcc gttgacaccc 1201860
aataaaccat agcgcgcgcc gctttccagc gacagggtaa tgtcgtgcaa aacagttttg 1201920
ccttcgtaac ccaaatctgc gtgttctagc tttaacaaag gattgggcag atggtcggga 1201980
tggtaaaact caaaggaaaa ctcgctgtcc agatgcgcgg gagcgatgcg ttcgagcttc 1202040
gccaaagcct tcatgcggct ttgcgcttga acggctttgg tggctttggc tttgaagcgg 1202100
tcgataaagg attgcaaatg tttgatttgc gcctgctgtt tgacataggc agcttgttgt 1202160
tgcgcgagac gctgcgcacg ttcgttttgg taaaaatcgt aattgccgcc gtattgcgtg 1202220
agtttttgct gcgataattc aatggtttgg gtagtttccg cgttgagaaa atcgcggtca 1202280
tgggaaatga tgatttgcgt gcagggtaaa gaagcaaggt ggttttccag ccacaagacg 1202340
gtttccaaat ccaagtggtt ggtcggttcg tcaagcaaga gcaaatcggc gcggcaaatc 1202400
agggcttgcg caagattcag gcgcatacgc cagccgccgg aaaaggattt gacggggcgg 1202460
ctgtgttctt cttgcgaaaa acccagcccg ttcaacaatt ttgccgcacg cgccggcgcg 1202520
gtataagcgt cgatttcttc caatttagca tgatattccg cctgcttcat gccgtcattt 1202580
tgcgcttctg cctgcctcaa tgccgtctga aaagcctgca actcggcatc gccctgcaaa 1202640
acgtaatcca aagcggaaat atccaaatcg ggcgtttctt gggaaacgga agcgagccgc 1202700
cagtttttcg gaatcgagac atcgccgccg tcctgagtga tttcaccctt gattaaggca 1202760
aacaggctcg atttgcccgt tccgtttttg ccgatcaaac cgacgcgctg accgggattg 1202820
acggtagcgt tggctttgtc gagcaggact ttcaaaccgc gttgcagggt gaggttttg 1202880
atttcaatca taacggaaac atcgtcgggc gggaaaagcc cgtattttac ctgaaagtca 1202940
gtgccgatgc cgtctgaaac gggaaattta cggctgaagc caagcccaag ccctgcgccc 1203000
ttccgagtgc aggaaaacca atgtcctgaa tgccgaatcg gtattcatgc attccacgct 1203060
gattccgatt cgggaaaaat ccgccatgat tttgggatgg ataaactcct gagccgcgcc 1203120
cgtcccgata atcaatattt ccggatagtc aacaggtttg acgtcggaca acaggtttc 1203180
cggagtcaga tcggacaagg ttcggcattg cgacaggcag accgaatcct tatgtacaag 1203240
cacgggttta tggaaacttt gccccgccag ccggattccg cccgcaccgc attcatattc 1203300
cgcaaactgt ccgtctatcg gattttcttc aaacaacatt tttttacccc gttgccgcat 1203360
catctacacc gaaagggatg caaaatcaga caaattcatg taggattggc agatttcatc 1203420
tgacccgcct gccgatttca gacggcattt gattcaaagt gcggcacaat tatatcggca 1203480
gcggatattt tcgtctttca atatttacat ttcagtcggc ttacaaggag acacaatgaa 1203540
gccagtaaac atcggtcttt taggtttggg tacggtcggc ggcggtacgg ctgccgtgtt 1203600
gcgggacaac gcggaggaaa tttcccgtcg cttggggcgc gaaatccgta tttctgccgt 1203660
gtgcgatttg agtgaagaaa aagcccgaca aacctgcccg tccgcagcct ttgtcaaaga 1203720
tccgttcgaa ctggtcgcac gtgaagacgt cgatgtcgtc gtcgaattgt tcggcggtac 1203780
cggcattgcc aaagatgcgg tgttgaaagc cattgaaaac ggcaaacaca tcgttaccgc 1203840
caacaaaaaa ctgctcgccg aatacggcaa cgaaatcttc ccgctggcgg aaaaacaaaa 1203900
cgtcatcgtc caatttgaag cggcagtagc gggcggtatc ccaatcatca aagccctgcg 1203960
cgaaggtttg gcggcaaaca ggattaaatc catcgccggc attattaacg gcaccagcaa 1204020
cttcatcctc tccgaaatgc gcgaaaaagg cagcgcgttt gccgatgtac tgaaagaagc 1204080
gcaggcattg ggttatgccg aagccgatcc gaccttcgac atcgaaggca acgatgcggg 1204140
ccataaaatc accatcatga gcgcactggc attcggcacg ccgatgaact ttccgcctg 1204200
ctacctcgaa ggcatcagca aactcgacag ccgcgacatc aaatacgccg aagaacttgg 1204260
ctatcgcatc aaactgttgg gcattacccg caaaaccggc aaaggcatcg agctgcgcgt 1204320
ccaccctacc ctgattcccg aaagccgcct cttggcaaac gtcaacggcg tgatgaacgc 1204380
cgtgcgcgtc aacgccgata tggttggcga aaccttatat tacggcgcgg gcgcgggcgc 1204440
attgccgacc gcttccgccg tggttgccga tatcatcgac atcgcccgcc tggttgaagc 1204500
cgataccgcc caccgcgtac cgcatctggc gttccaaccc gcgcaagtcc aagcgcaaac 1204560
catcctgcct atggacgaaa ttaccagcag ctactacctg cgcgtccaag ccaaagacga 1204620
accgggcacg ctggggcaaa tcgccgcgct gttggcacaa gaaaacgtgt ccatcgaagc 1204680
actgattcaa aaaggcgtga ttgatcagac cactgccgaa atcgtgattc tgacccacag 1204740
cacggtcgaa aaacacatca agtcggcaat cgcagccatc gaagcactgg attgcgtgga 1204800

aaaaccgatt accatgatcc gcatggaaag cctgcatgac tgagccgaaa cacgaaatgc 1204860
tgacgaaaga gcaggttgcc gcgcgcaaaa aagcaaaagc caaaatccgc accatccgca 1204920
tttgggcgtg ggtcattttg gcgttgctcg ctttaaccgc cctgctctcc caatgcgcga 1204980
tgtccaaacc gcaggcaaaa cagaaaattg tcgagtcttg cgtgaagaat attccgtttg 1205040
ccgaaaaatg gcaaaacgat ttgcgggccc gcggtttaga ttcaaacaat acccgcctcg 1205100
ccgtcgacta ctgcaaatgt atgtgggagc agcctttgga cagattgagc gagaaacaga 1205160
ttagatcctt cggcaaactc ggcgcacaag aacagcttga cctgctcggc ggcgcaaatg 1205220
cctttgaagc acgtgacaag cagtgtgttg ccgatttgaa atcagaataa tgtggaccga 1205280
taaaaaagcc gattctttaa agaatcggct tttttcataa aaaacggctt acagtgcgtc 1205340
tttcaaagct ttgccggcgc ggaatttagg cgttttggcg gcggcaatgg tcagaggctc 1205400
gccggttttg gggttgcggc cttggcgttc cgcacgttcg cccacgtaga aagtaccgaa 1205460
accgaccaaa gtaacggtgt cgccttgttt cagggcggtg gttactgcat tggtagtggc 1205520
atccaaagct ttttgtgcgg cggctttgga aatgtcggct tcttgagcaa tcgcttcgat 1205580
caattcagac ttgttcacaa tcagtccctt cctgtcttaa aaaatgatga aatgcccgaa 1205640
tactcggggt ttgtactgct tgagcaactt tcgctttata gcaattctga aattgccgtg 1205700
tcaagcaaaa aatacggaat caccctattt gacaggcttt caggacgaaa ccgcattttt 1205760
acaacacatt tcctgcgttt caatgtttgg ttgccctgct gcggggtttt ggttttgaag 1205820
cggattccgc cgccgcttcc gcaccagaag gttctgccca aggctcaggc tggctttcca 1205880
aacccagagc caatacctcg tctatccatt tgaccggatg gatggtcagg ccggttttca 1205940
cgttttcagg gatttcttcc aagtctttga cgttgtcttt cggaatcagg acgtgtttga 1206000
tgccgccgcg caaggcggcc aacagttttt ccttcaaacc gccgatgggc aaaacttcgc 1206060
cgcgcagggt aatttcgccc gtcatcgcca catcggcgcg taccgggatt ttggtaaagg 1206120
cagataccgc cgccaaggtc atcgcaatac ccgcactagg gccgtctttc ggcgtcgcgc 1206180
cttcgggaac gtggatgtgg atgtcttttt tctcgtaaaa atcaggagcc aaacccactg 1206240
attccgcacg ggagcggaca accgaccacg ctgcggacac ggattccttc atcacatcgc 1206300
ccaactggcc ggtgcactga atcacgccct tacccggcaa tgctgcggct tcgacggtca 1206360
gcaattcgcc gccgacttcc gtccacgcca aaccggtaac ctgcccgata cggttttcgc 1206420
tttcggcaac gccgtaatcg aagcggcgca cacccaaata gtcgtgcaga tttttctcat 1206480
ttactttaac cgctttaggt ttggctttgc tggttttctt ggtttcagac aacctcttct 1206540
tatcttcgtc caaggtaatc tgcatcacca ccttgcggca gattttggca atttcgcggt 1206600
cgagcgaacg cacgcccgcc tctcgggtgt aataacggat aatatcgcgc accgcgcttt 1206660
cttcgattgc caattcccct tcttttacac cgttgcgctt catttgcttc ggtacgaggt 1206720
actgcatcgc gatattgatt ttttcgtctt cggtatagcc ggacagacgg atgatttcca 1206780
tacggtcgag caacggagtc ggaatattca gactattgga tgtggcgata aacatcacat 1206840
cactcaaatc gtaatccact tccgcataat gatcggcaaa cttgttgttt tgttcgggat 1206900
cgagcacttc gagcaacgcg ctggcgggat cgcctcggaa gtcgttaccc aatttgtcga 1206960
tttcgtcgag caggaacaag ggtttttca cgccggcttt tgccatattc tgcaaaatct 1207020
taccgggcat agagccgata taggtgcggc ggtgtcccct gatttcgctt tcgtcgcgca 1207080
cgccgcccaa agccatgcgg acatatttcc gccccgttgc tttggcgatg gattcgccca 1207140
aagaggtttt gcccacgccc ggagggccga ccaggcacag aatcgggcct ttgagtttgt 1207200
ccatacgttt ttggacggcg aggtattcca aaatccgttc tttgactttt tccaggccgt 1207260
agtggtcggc atccagcacc agtccggctt tggcgatgtc tttgctgacg cgggattttt 1207320
tcttccacgg cagctcgagc aaagtgtcga tgtagttgcg tacgacggtg gattccgcag 1207380
acatcggtgg catcattttg agctttttca gttcggacag gcatttttct tccgcttctt 1207440
tggtcatacc cgccttttg atatctgctt ccaaggcatc cagttcgccg ttttcgtctt 1207500
cttcgcccag ttctttgtgt atcgctttaa tctgttcgtt cagataatat tcgcgctggg 1207560
atttttccat ttggcgtttg acgcgtccgc gtatgcgttt ttcggcctgc ataatgtcga 1207620
gttcggattc cagctgtgcc agcaggaatt ccatccgttt gccgatttcg ggaatttcca 1207680
aaatctgttg gcgttgcgcc agtttcaact gcaaatgcgc tgcgaccgta tcggttagcc 1207740
ggctgttttc ggcaatgccg ttgatgctgc cgataatttc ggcggggatt tttttattga 1207800
gtttggcgta ttgttcaaac tgcgccaaca gggtgcggcg cacggcttcg aggtcggtat 1207860
tgccgcccgt gtcttcttcc acgaccgtct ctatatggga aacgaacaga ccgcccgtgt 1207920
cttcaatggt cagaacacgt ccgcgataca gcccttcgac caatactttt accgtgccgt 1207980
cgggtagttt caacacttgc aggacttgtg cgaccgtacc ggtctgatac aggtcggcgg 1208040
caatcggttc ttctaccgcc gcatcggttt gcgccaacag gaaaaccggc tcctcgcggg 1208100
taatggcgtt ttccagtgcg gcgatggatt tcggtctgcc gacaaacagc ggcagaacca 1208160
tatgcgggta aacgacgaca tcccgcaaag gaagggttgc caaggcggca tattcctcaa 1208220
aatgcttttc tttttgtgtc ataggtactc tcttgtgtct gacagattgc cgattttcgc 1208280
gtacattggg gttgaaggta ttatttcaag catatgtggt ttatttatgg agtttgatgc 1208340
gatgccgtct gaaacattcc ggcttcagac ggcatgggct tggaaagaca aggcgggaac 1208400
aaaaaactgt tctgtgttgc cgctccttgc tgtaccatcc gtatggtttg cggttctgcc 1208460

gccctatttt caaaacatga cgcaggtatc ccatgtcttc ttttattttt cccgatgacg 1208520
ggttcgaatg gcgcaacgaa agtctgcaaa agccgcccaa aggttggcat tatgtccgcg 1208580
aaagcggtgc agacggcatt ttgaaggctg cctatcaaaa tattgcaact gtctagcagg 1208640
gcgatttcca caatgccaaa caggtgcttt ctgcaatgaa gaagagggtt cgcaaacctg 1208700
ccgccgtccg ttccgatgcg gatatcgccg cccttttcca tgcccaccgt atgaagcagg 1208760
cgcagcagag ccgtattctg aatatgcttg ccgttgaaat ccgcccccggt tttgtgttgg 1208820
acaacaaacg cgcgcccgat atacgctccg ctttgctcga cgtgtacgga gaggcggacg 1208880
gcaaaccgtt tttcctgccg ctcaatctgc tgctggggtt tatgggtgcg cacgagtggc 1208940
ataagaaagg ggttgccgtt ccgcagctgg gcggcagcat acacgttcct ttcggcgtat 1209000
tctcgccgtt gcgcggcgaa tacctcgacc tgctcgccca tgcgccgtca acgggttttc 1209060
agacggcatt cgatatcggg acaggctccg gcgtgcttgc cgccattttg gcgaaacagg 1209120
gcattccttc cgtcatcggc acggatacca atccgaaagc cgtcgcctgc gcccgtgcca 1209180
atattgcccg tttgggcttt gaaaaacagg ttgagatacg ggaaaccgat ctgtttcccg 1209240
aagggtttgc cgatctgatt gtctgcaatc cgccctggct tcccgccaag ccgacttccg 1209300
ccgtcgaatc cgcgttatac gaccccgaat ctgcgatgct ggctgcgttt ttgcgggatg 1209360
cgccgaaaca tctgaatccc gacggagaaa tccgcctgat catttccgat cttgccgaac 1209420
atctgcacct gcgtccatcc gattttctgg ataaggcatt tgctcaggcg ggtttgcgtg 1209480
ttgccgatat gatgaaaacc aagccgaagc acaaaaaagc cgcgaatccg agcgatccgc 1209540
ttgcttttgc gcgaacccgg gaaaccactt tcctataccg tttgaaaaag gcataagggg 1209600
cggcggcgcg gcattcgggc ggattattct tgtgaaaata cccgctcgag catactgccc 1209660
aatgccgtct gacgcgtttt gacggtggcg gcagcttgcc ggaaggcttc ttccccgccg 1209720
aagaatacga acttctctct cgcgcgggta atggcggtat ataacagctc cttactcaat 1209780
ccggacaatg catcgtcccc ttcgtccgaa ggtgcggcgg aaggcggcag cagccatact 1209840
tcccggtatt ccgaaccttg gcttttgtgg acggtcatgg cgaatgcggg ttcaaattcg 1209900
ggcaggcagc ttaccgctac ctttttaaat ccgtccgcat cggcaaaata ggcggcaagg 1209960
ctgccctgcc gtccgacatc ttccataatc agtccgatgt cgccgttgaa cagttcaagc 1210020
gcgtagtcgt tctgcctgat cataatcggc tctccggcaa aatatgccaa atgttccggt 1210080
atgttcattt tgcggcgtac atggcggcaa taggcttcgt tgaagtcttc cgcatcctgc 1210140
cgccaagctg ccagaaccac gatatccgaa atgcccgcgt atgcggcttc gatattgccg 1210200
tcttttaccg cctgccaata ggctttgtgt gcccggtaca acctttcgac tcgagcgttc 1210260
ggactgcatt ccgaatgttc cagttcgtcc ggaaaccggt caaacaatgc ccacgcccct 1210320
tcatcgcccg atacggcggc acgggcaagg cagccgatgc cgctgttgtc gccgaagcgg 1210380
tggctgaacg acagatgggc ggtgttttgc gccaacacgg gtggatttgc gctgacgctg 1210440
aaaccgtgtt ccggaaggaa gccggccagc ctttggtgcg tttctccgtc caaaacggtt 1210500
ttttgtgaca aaacggacag caccgcccct attccgacgg acgggagctg gttttcatcc 1210560
cccagcagaa tcacgcgcgc gccggttttg accgctttta aaagttgcag catcaatgcc 1210620
gtatccagca tagaggcttc atcgataacc aatacgtcaa acggcagcgg gtaaacaggg 1210680
ttgaacgccg cctgcatttt gggcgggcgc agcttcagca gtcggtggac ggtttgccct 1210740
tccagtttga gcaaatggcg gcggacggcc tccggcgcgt caaaaccgtt gattgcacgg 1210800
tgcagcgcgc gcgccatatg tgccgccgct ttgcccgtcg gtgcggcaag cgcgatgttg 1210860
ggaagatttt cgtcttcacc gcaaatcagc gccagcagtt tggcaaccgt tgtcgttttg 1210920
cccgttcccg gcccgccggt aatcaccata aaagactgca acagtgccaa ggcggcggca 1210980
tcgcgctgcc cttcgctgcc cgtgccttga aaccattttg cgaggttttg cctcgcgcct 1211040
gccgcgtcgg gggcggatgt gccggctgcc gccaagcgtt ttatctcggc agccaaatcg 1211100
tattccaact gccacatcct gcccaaaaac agccttctgc cttccaaaat caaaggcgcg 1211160
gcggatgttc cgacaacggg tgcgagtgcc gacagcgcgt cagcctcgcc accgctcaaa 1211220
cggataaacg aatgaccgtt ttgcaatgcc tgaaacaggc gttcggtgca gtttgcaagc 1211280
acttcgtcgc cggaacccgc atagtgttcc aaaaaacgga ttgccgccct tgccgccgct 1211340
tgggcaaatt catctgtctg ccgttccatt ttattcctta tccaaatgcc gtctgaaggc 1211400
gtggggcttc agacggcgcg gtgttgtttc ggctgtttag gcgtttgcgc cctgtttggg 1211460
gtgcaggctg ccgtctttca tgaccatcac gcgctcgaag cggccggcga gttcgtcgtc 1211520
gtgcgttacg accaccagcc ccgttcccaa ttctgttttc agttccagca tcatatccaa 1211580
aacattcctg gcgttcgcac gatcgagatt gccggtcggt tcgtcggcaa gcaggcattt 1211640
gggttgggta accaaggcgc gtgcaatggc ggcacgctgc cgctcaccgc ctgaaagttc 1211700
gcccgcgcgg tgcgtcgaac ggtgtttcag tccgaccttt tccagcatcg ccattgccgc 1211760
ctccgcagcc tcttcacggc tttttttgcc gatcagaagc ggcatcatca cattttccag 1211820
tgccgaaaat tcaggcagaa gatggtggaa ctggtacacg aaaccgagat ggcggttacg 1211880
taaatcgccc aaacgccgct ggtttaaggt acgcaaatcc tcgcccatca gcagcaccct 1211940
gccttcagac ggcatatcca gcccgcccaa aatatgcagc agcgtcgatt tgccgctgcc 1212000
cgaagaaccg atgatgccgg tgctttcccc tgcgtggatt tccaaatcca agccgtgcag 1212060
cacccgaacg tccaaaccgc cgtcccgata gcgtttgccc acgccttcgc atttcaaaat 1212120

caactcactc ataacgcaaa gcctccgccg gttgggtttt tgacgcgcgc cggctcgggt 1212180
agagcgtggc aacgaaagac agtcccaaag aaatgcaggc aatcagggca acgtcgccca 1212240
tatcgacatc gctgggcagg tagtcgataa aataaacctg cgaattgatg aggtggacac 1212300
cgagcaggtt ttcaaaaaac gccacgaccc tgccgacgtt ccaacccaaa agcacgccgc 1212360
agaccacacc cgccagcgtg ccgaaaaagc ctgaaaacgc gccctgcacc ataaaaatct 1212420
tcatcacgcc agcaggggaa agacccaaag tccgcaaaat cgcaatgtcc gcctgctttt 1212480
ccgtaaccgc catcaccagg gaagagacaa ggttgaacgc cgccacagcg ataatcagcg 1212540
tcaggatgat gaacatcatc cgtttttcca gttcgaccgc ttcaaaatag ctgcggttgc 1212600
tgtacgtcca atcgcgcacc caaaccgcgt ccctttgcgc ctccggaatc agtgttgccg 1212660
tcaaggcggg agcgttttgc ggatcggcga gcttcagccg cagccccgca acttccttat 1212720
ccaaacggta cagcacgcgc gcgtcttgga tatgcgtcat tgccaatgag ttgtccactt 1212780
cgtaaacgcc cgtcttaacc agaccgacca cggtaaactg tttcaacctc ggtacgactc 1212840
cggcgggcgt aacattgccc tccggcgtga tgacggtaac tttattgccg acttccgccc 1212900
ccaaagcctc cgccaagccg acaccgagga taatgtcaaa ctcgccccgga atcagatctt 1212960
caaatttgcc tgccggcatt ttgtcgccgt attccaccac tttgcgttct tcagacggca 1213020
aaatgccgcg catctgcacg cccctgattt cgcccgcatt ggccagcaat gcctgattgg 1213080
aaacataggg cgcggcagcc aaaataccttt tgcggttttc ggtaaaccga agcaggttgc 1213140
gccaatccgt atccgtatta tcgatatagc cgatttcggc gtgcggcgcg acattcagga 1213200
gctgcccgcg tatttctttc tgaaagccgt tcataaccga caagacgaca atcagcgcgg 1213260
ttacgcccaa ggcgattccg gcaatcgaaa ccatcgtgat aaacgacata aagccgttgc 1213320
gctttttcgc cctgagatac ctcaagccta tccaagcctc tagagaaaac ataacgctac 1213380
cttaaaaatg tctgcaaacg tgccgccccg gacggcggtt tgggggcggc ggcaaaagtt 1213440
tattgtaccg taaaacccag gcagcgtccg aacgcccgag tgcgggggac ggggcggcag 1213500
agcgggcgga aaacttgcac aacgcgtcaa actgccctat cctttccttc agaaaaaccg 1213560
tttcttgagg aaaacaatga atatccgaac tgcttttgct ttgtgcgcca tcgccttatc 1213620
cgccgctgcg gctgcctacg ccaaagaaat caaaatcgat gccaacaaca cgccttattc 1213680
cgaagccgac gcgcaaaagc tggcggcaac ggcagtcggt atgggtgtta aggaacctat 1213740
cagcctgaac ggcggcagcg gcagcattac cgtgtccggc agcagcgcga cgcagtgcgt 1213800
gttcaaagtc ggcaacggag gcgcattgca gattcaaggg ctaaactgca agtaaaccgc 1213860
ccggaaaaat gccgtctgaa ggcttcagac ggcattttgc attggcggcg ttatgccccg 1213920
ccttctttaa tcaggcggcg ttcgtacacc gcctgcgcca gcgttcccgc atcgacatat 1213980
tccaattcgc cgcccaaggg aatgccctgc gacagcctgc tgactttgta aggcaggttt 1214040
ttaaaaaact cggacaggac atacgccgtc gcattgcctt ctgcggtaaa agcggttgca 1214100
ataatgattt cttcgacttc cccgccgccc agccgttgcg ccagcctgtc caatgcgatg 1214160
gcggatacgt ccattcccaa tgccgtattg atttgcccca tcaggacgaa atacagcccg 1214220
tcgtggcagt ttgccgcttc catattcgac acgtcggcag gcatatgcac caccatcagc 1214280
cgccgcccgt cgcgtgtttc atcggcacaa atatcgcaca atccgccttc gcaaaacgtg 1214340
ttgcacatcg cgcaatggta aacctgcttc aatgccgtct gcaaggcatc caccagtttt 1214400
tcagcctctt tgcgcttgtg ttggagcaaa tgatacgcta tccgctgtgc cgatttcggc 1214460
ccgacgttgg gtaaaacctt cagcgcgtcg atcaatcctt ggaaggcatc ttgtttttttg 1214520
tggctcatca tattccgccg tatgggaaaa cggccggaat attccgaccg ttattttgtc 1214580
aacaaaagtg tcaattactg accgtcgccg ttgtcgaccg attgcgctcc tttggtctgt 1214640
ttgattttgc cgttgaaata acgtatcaac aagtcgaaag tattggcaga ctgctgttgc 1214700
gccaaagcct gttttgcaag cggaagctgt gcggcgatat catccggcgg ggttacagcc 1214760
tgtacttcga caatcacggg tgccggcaga ccgatcagcc tgacgtaggc gggtttgccg 1214820
tttgccggtt ttgctttcag cagttccgca taagcctcgg gcggcatgga ctgccttgcc 1214880
tgctgtgcgc ccaaaacgga cacttccgac catttcacgt caacagcctt gccgccgttc 1214940
agttgggtaa gcacgtcttt tgccttgttt tcggcaagtt tggcggcttc ggtacggata 1215000
taagcctgac gtaccgcgtc tttggcttcg gcaaacggca gggttttctc ttcgcggact 1215060
tctttggcgc ggacgaccca cgcggtttcg ctgttgatgg tcagcacttc ggaattgtgt 1215120
tttttcttca atacgtcgtc gctgaatacg gcattgatca ggttttcggg cataccggac 1215180
atttgcgcgt cctgcctact cagccaagtt tcttgggttt cgactttcaa accgctgttt 1215240
ttggcggctt cggcaagcga ggaaggatgg ttgaacgcat cgtcgcccaa tttttctttt 1215300
gccttgttga agtcggcaac cgcctttttc attttcaatt cgttttcgac ggcggctttt 1215360
tcctgctcga aagaaggttt ggcttcattt gccggcaaac gcgccacgcg ctcttcaaat 1215420
gcatttttca cttccgtttc actgacggtc tgcttgtctg caaaatcctt cagattcaag 1215480
gcgacatatt ccaatttgac cgcctgcggc agcagatagt ctttttttgtt cgcattataa 1215540
aatttctgca aatcggcttc agacactttg acttgggcga tgaactcgtc ggggttgaaa 1215600
gtgtgcgaac ggatggtgcg gttgacctgt gtcagcctga tcagctgttc cgcctgcgcg 1215660
tcgccgacca atacgccgtt ttggacgagg tttaccaaat tctgcaaggc aaactgatcg 1215720
cggatttctt cgacaaactg gtcttcagac atatggcgtt gggaaaggta gcggtttaaa 1215780

agcgcgtggt cgaatttgcc gtttgcgtcg tggaaattgg gatcgtccac gataatttgc 1215840
ttgatttgtt cggaagaaac cgaaatgccc atcagcttcg cgccctgttt caggtaggcg 1215900
cgttgcagca gggattggaa caccgcgtcg cgcgaagggc cgccgccgtc cgcctgttcg 1215960
ttctgtatgg cgttgttgat ggagtggtcg ctgatttttt cgtcgcccac ttggacgatg 1216020
tagtcggcac ccggatggga taccgtgctg accccgaagc cgacgaaggt taatgcaatc 1216080
aggcccaaaa ggacttgggc gggcgttctg tatttttcga tggaatggaa catattttaa 1216140
atcgggatat agaatgggaa cgggaaattc aagtcgggta ttgtaacggt ttttatccct 1216200
gtctgcacgg ggcttgccgg ttgaagatgc cgtcgtaggt ttcttcgctg aagccggcgt 1216260
aaaccttgcc gccgcactcc aatacgggac gcttgatcag gctcggcatt tcggacatca 1216320
gtttgacggc ctccgccgtc gaggacagca ctttttgctg tgtttcggca tcgagtttgc 1216380
gccagcttgt cccgcgtttg ttgagcaggg ttgccaaagg cacttgttcc agccacgagc 1216440
agatttccgc ttcagacggc ctctgttttt taaaatcccg aaattcaaac tccaagccgt 1216500
atccggcaag ccggtttttg gctttttttga ccgtatcgca atttgggatg ccgtgaagga 1216560
ctatcatttg gaaacctttt gtctgaaata ataaaacgga tattttacta taagtgtctg 1216620
aaaatttgcc cgtctgtttc agacggcggg gcggttatgt tacaatccga aaattcgaaa 1216680
aatttaatct cttgttcaat aaaggcttta ccaatcatga tttctaccaa cggcatcacc 1216740
atgcagttcg gcgcaaagcc gctgtttgaa aacgtatccg tcaaattcgg cgaaggcaac 1216800
cgttacggct tgatcggcgc caacggctca ggcaaatcca ccttcatgaa aatcctcggc 1216860
ggcgatttgg aacagacggc cggcgaagtg gcgattgaaa acggcgtgcg tttgggtaaa 1216920
ttgcgccaag accagtttgc ctacgaagac atgcgcgtgc tggacgtggt gatgatgggg 1216980
cataccgaaa tgtgggcggc gatgaccgaa cgtgatgcga tttacgccaa tcccgaagcc 1217040
accgaagacg actacatgaa agccgccgaa ctggaagcca agttcgccga atacgacggc 1217100
tacaccgccg aagcgcgtgc cgccgaactg ttgagcggcg tgggcatttc cgaagatttg 1217160
cacaatgcga aaatggcgga agtcgccccg ggcttcaaac tgcgcgtatt gctggcgcag 1217220
gcgctgttct ccaagccgga tgtattgctc ttggacgaac cgaccaataa cttggacatt 1217280
aataccatcc gctggttgga aggcgtgttg aaccaatacg actccacgat gattatcatc 1217340
agccacgacc gccacttttt gaacgaagtc tgcacgcata tggcggattt ggactacaac 1217400
accatcacca tctatccggg caactacgac gactacatgc tcgcctccgc ccaatcgcgc 1217460
gaacgcgccc tgaaagacaa tgccaaagcc aaagagaaac tgcaagagct gcaagagttc 1217520
gtcgcccgct tctctgccaa caaatccaaa gcccgtcagg caaccagccg tctgaaacag 1217580
gccgacaaaa tcaaatcgga gatggtcgaa gtcaaacctt ccacccgtca aaacccgtat 1217640
atccgttttg aagccgatga aaaagccaag ctgcaccgtc aggctgtgga agttgaaaaa 1217700
ctggcgaaac gctttgaaac ccagttgttt aaaaacctga acttcatcct tgaagcggga 1217760
caacgcctcg ccatcatcgg cccgaacggc gcgggcaaat ccaccctgct gaaactcttg 1217820
gccggcgcgt acaaccccga atattcagac ggcctgttgc cggacgaagg caccatcaaa 1217880
tgggcggaaa aagccagtgt cggctactat ccgcaagacc atgaaaacga cttcgacgtc 1217940
gatatggacc tgagcgaatg gatgcgccaa tgggggcagg aaggcgacga cgaacaagtc 1218000
atccgcggca ctttggggcg tttgctcttc ggcagtaacg atgtcgtgaa aaaagtgaag 1218060
gttctctccg gtggtgaaaa aggccgtatg ctttacggca aactgttgct gttgaaaccc 1218120
aatgtcttag tcatggacga accgaccaac catatggaca tggaaagcat cgaatccttg 1218180
aacatggcac tggaaaaata caacggcacg ctgattttg tctcccacga ccgtcagttc 1218240
gtttcctcct tggcaaccca aatcatcgaa ctggacggca aaggcggata tgaacactac 1218300
ttgggcgatt acgaaagtta cttggagaaa aaaggcgtag cataaccgcc ggttggaaca 1218360
atgccgtctg aagccgcttc agacggcatt gttgataact ttaaaatagg aagcatatgc 1218420
agacttatct cgtcggcggt gccgtccgcg attatctttt gggcttgccc gtcaaagacc 1218480
gcgattgggt ggtcgtcggc gcagacgcac aaaccatgct ggcgcaaggc ttccagccgg 1218540
tcggcaaaga ttttcccgtg tttctccatc ccgaaacaca cgaagaatac gccctcgccc 1218600
gcaccgagcg caaaaccgcc aaaggttacg tcggtttcag tttccacgcc gacaaagacg 1218660
ttacgctgga gcaggatttg atgcgccgcg acctgaccat caacgcgatg gcgcaagatg 1218720
cggacggcaa gattatcgac cctttcggcg gacaacggga tttggcggca ggcattttgc 1218780
gccacgtttc cccagccttt gccgaagacc ccgtccgcat cctgcgtact gcccgctttg 1218840
ccgcgcgtta caagtttgaa atcgccgaag aaaccataaa gctgatgcgg cagatggtgg 1218900
aaaacggcga agcggacgca ttggttgccg aacgcgtctg gcaggagttt gcgaaaggtt 1218960
tgatggaaaa aaatccgcgc aaaatgattg aagtgttgcg cgaatgcggc gcgctcaaag 1219020
tcttgctgcc cgaagtcaat gccctcttcg gcgtgccgca acgcgccgac taccatcccg 1219080
aaatcgacag cggcatccat accctgatga cgctgcaacg cgccgccgat atgggcttga 1219140
gcctgcccga acgctatgcc gccctgctgc acgacttggg caaagccaaa acaccgtccg 1219200
acatcctgcc gcgccaccac ggacacgacc tcgccggtgt cgaacccgtg cgcgaagtca 1219260
atcagcggct gcgtgcgccg aaacattgcg ccgagcttgc cgaattggtt tgccgttggc 1219320
acattatttt ccaccaagtc ggacagctta aaagccaaac cattctgaac gttttgaaaa 1219380
aaaccgacgc tttcagacga cccgaacgct ttcagacggc attgaacgtc tgcattgccg 1219440

acacgcaagg ccgtctgaac cgcgaacaca cgccctaccc gcaacgcgcg cactggctcg 1219500
ccttactcga agccgccaat caggcggatt cgggcaaaat cgccgccgaa tgccgcgcac 1219560
agggaaaagc gcaccttatc gccgaacaaa tcgaccgggc gcggctggca caaatcgccc 1219620
cattgcaaaa agcgtttcga gcggcgcaag acaaaacaga aaaacattaa aacgtccaat 1219680
gcagccactt ttatagtgga ttaacaaaaa tcaggacaag gcgacgaagc cgcagacagt 1219740
acagatagta cggcaaggcg aggcaacgct gtactggttt ttgttaatcc actataaagt 1219800
tttgaggacg atacccaatc caagctttgc aacagccgcc gccatatccg ctataattca 1219860
cgcttcagcc attccgcccc cgacataaaa tcatgaccct gaaaaccgat ttattgccta 1219920
aaatcaacaa cgaagattat caacgcctca tcctcaaaca cagtgcggaa tttagcggtg 1219980
gcgaaatccg cctgttgaac gaaatcctcg aaaaattcaa tttcgacgtt gttcaggcgc 1220040
aggcattggc gcaagccgtc atgcagcaaa tccgcttcga ccccaacgcc taccacatcg 1220100
acagcgacga cgaagacacc accggcatct gcccccactg catcaacccg cctatgccgc 1220160
ccctgcgcga ctatctcgtt tggcgcgaaa cccgcggata aaacgctttt gaccgttatc 1220220
ttttcaatgc cgtctgaaac gccgccgacc gttcggacgg catacccgac aaagggaaca 1220280
ctatgctgca aaccgacaac ctgaccgccg cgcaaccgca acgcatcgtt gccgcccaaa 1220340
ccgcctccgc acaggaagaa ctgctcgaac gcgccctccg ccccaaaacg ctggacgact 1220400
acatcgggca agacaaagcc aaagaacagc ttgccatttt catccaagcc gccaaaaaac 1220460
gcggcgaagc actcgaccac gttttgctct tcggcccgcc cggactgggc aaaaccacac 1220520
tggcgcacat catcgccaaa gaattgggcg taaatttgcg ccaaaccagc ggccccgtcc 1220580
tcgaacgcgc aggcgacctc gccgcccttt tgaccaacct tgatccgcac gatgtattgt 1220640
tcatcgacga aatccaccgc ctcagccctg ttgtcgaaga aatcctctat cccgcgctcg 1220700
aagactaccg gctcgacatt atgataggcg aaggacccgc cgcccgttcc gtcaaaatcg 1220760
acctgccgcc cttcacgctc atcggcgcga ccacccgcgc cggtatgctg accaatccgt 1220820
tgcgcgaccg cttcggcatc gtctcccgcc ttgagtttta cgaaaaccga gaccttacca 1220880
ccatcgtcag ccgttcggca caactgttgc agctcgatat gtccgaagaa ggcgcggaag 1220940
aaatcgccaa acgcagccgc ggtacgccgc gcatcgccaa ccgcctgttg cgacgcgtgc 1221000
gcgatttcgc cgacgtgaaa aacaacggca caatcgacgg cggcatcgcc gatgccgctt 1221060
taagtatgct ggacgtggac gcgcaggggc tggacgtgat ggacaggaaa tttctcgaag 1221120
ccgttttgca caaattcggc ggcggcccgg tcggtttgga caatgttgcc gccgccatcg 1221180
gcgaatctac agacaccatc gaagacgtta tcgaacccta ccttatccaa caaggcttcc 1221240
tgcaacgcac cccgcgcggc aggatggcga ccgaacgcgc ctacctgcat ttcgggctgc 1221300
ccgtcgaaaa ataacgcaat gccgtctgaa acagagctaa ttttcagacg gcatttctat 1221360
ttcaatcatt ggcgcaaggt tcagcctgcc gcttttttcc agttccgccc tcatcgcatc 1221420
aatcaccgcc ttatagtctg gtttgccgaa aatcgcagaa ccggcaacaa aggtatccgc 1221480
accagctcgg gcaacggcgg caatattgtc ggttttgatg ccgccgtcca cttcgatggc 1221540
gatgtgccgc ccgctttgtg cttcgtaccg atccagcatc gcccgcaccc ggcggatttt 1221600
ttcaagggtg tgcgggatga agctttgtcc gccgaatccg gggttgaccg acatcagcaa 1221660
aaccatatcc agcctgtcca atacgttttc caacagatat acgggcgttg ccggattcaa 1221720
caccagcccc gcctgacagc ccatatcacg aatcaggctc aagctgcggt cgatatggcg 1221780
gctcgcctcg ggatggaacg tgatgattga tgctcctgct ttggcaaacg actgaatcag 1221840
gtcgtcaacg ggttcgacca tcagatgcac atcaatcggc acgcttgcat aaggcttcaa 1221900
cgccgcgcaa accatagggc cgaaggtcag gttcggcaca taatggttgt ccatcacgtc 1221960
aaaatggatc agatctgcac ctgccgcaat gacgctttcc acctcttctc cgaggcgggc 1222020
aaagtctgcc gataaaatgc tgggtgcgat acggtaagta gtcatgtttt ttccttcaat 1222080
atccttttat agtggattaa caaaaatcag gacaaggcga cgaagccgca gacagtacag 1222140
atagtacgga accgattcac ttggtgcttc agcaccttag agaatcgttc tctttgagct 1222200
aaggcgaggc aacgctgtac tggtttttgt taatccacta tacgttccga ttccgccgtt 1222260
atgtctgcct gccggacata cgtacgcatt attaacaaaa gttaaccgcg ataataccat 1222320
ctttcacacg tcaatctagt atatttccta aaatttccaa caagaggaaa agccgtgcca 1222380
ctgcctgctc cctgccgttt tgccaaacct gccgcctctt ttttaagtat ggctttgctt 1222440
tcctgtcagc tttcccacgc cgccacggct tatatccccc cgaacgattt tcaaccgaac 1222500
tgcgacatac gccgactcgg gctgacccaa agtcagcaca atgagctgcg taaaatccgc 1222560
accgccttca aaatggcggg cgacagggcg cgtttgaagg ttatgcattc cgaacacagc 1222620
cgccgccggt ctgtcgtcga aatcatttcc tcggatgttt ttaatcggaa cgaggcgcgc 1222680
gattatgtcg aaagccgcta tttgtccggt atggattttg cggtggacga attggaaatc 1222740
caacaccggt tcttccatat cctcacaccg caacagcagc aaatgtggct ttcttcctgc 1222800
ctcaaataat ccccgaaacg ctcacaacgc ccgttgtttc ggcagcctgc ccgcccagtc 1222860
gcaggcaaac tgccacgcgg aacgtcccga acggctgccc cgcgtctgcg cccactgcaa 1222920
tgccgccatc tgtgcggttt catcatacgg cacgccgaaa tcttccagcc aattttgcac 1222980
cgccgccaga taatcgtttt gatcgaacgg ataaaaactg agccacaatc cgaatcggtc 1223040
ggacagggat attttttctt ccaccgcttc tttctgatgg atttcccccc gcatccccgt 1223100

cgtaccggca ttctcgtcaa aatattcggg catcaggtgc cgtctgttgg aagtcgcgta 1223160
aaccaaaacg ttggcgcaac gttgagacag accgccgtct aacgcggttt tcaatgcctt 1223220
ataggtttca tcgccgcttt caaacgacaa atcgtcgcaa aatacgataa atttttccgg 1223280
acattccttc aaaagcgtca acaggtaagg caggccgatt aaatcgcttt tatcgacttc 1223340
gatcaggcgc aatcccttat ccgcatattc gtgtagcagg gctttgacca gcgaggattt 1223400
gcctgttccg cgcgcgccgc tcatcaatac attgttcgcg ggtctgccga caatgaactg 1223460
ttcggtatta cgcaccagca attcggtttg cctgccgact cccgccagcc ttaccaaggg 1223520
aaaggtgtgc ggatcgggca agtgttccaa aaaacctttt ttgcccgcac tctgccagcg 1223580
gaaggcaagc gcgttccaat ccgtatgccc gggttcgggc ggaagcacgg catccaaacg 1223640
ccgcaaaacg gcataggctt tatcgagaaa ttcgttcaat tccatctctg cctcactttg 1223700
catatctttg cgccatcagc cgttcgacgg tatcgacgat tgcctgcgta ttcggatcga 1223760
tttcgatgtt gatcctgtcg ccgacctttc tgctgccgaa cagcgtccgt tccaaagttt 1223820
cgggaatcag atggacattg aaacggccgt cttcgacttt gcctatggtc aggctgcaac 1223880
cgtccaagcc gacgaaccct ttggtcagga tatagggttt gagttcatgc gggagcgaaa 1223940
accaaaccgt gcggttgaac ccgtcccgtt cgatttcgac aataggcacg gttgccataa 1224000
tgtgtccgct catgacgtgt ccgccgattt cgtctccgaa acgcgccgcc cgttcgatgt 1224060
tgacgcaatc gccttccttc agcagcccca aattggtttt tgccaaagtt tccgccatta 1224120
aatcgaaact gacgcggttt ccttcgattt cggtaatcgt caggcagcag ccgttattgg 1224180
cgaccgatgc gccgcgttgc agattgtccg ccgcctcttg cggaagctcg acgacataag 1224240
tgtgaaatgc ctccgacggg cggtggattg ccgtcagttt tcccaatcct tgaacaatgc 1224300
ctgtaaacat aatcctgttt ccctgtgtcg gtaaaaatgg tgcaaattgt agcatctccc 1224360
cgcgaaaaat gccgtctgaa atgccttcag acagcattat gcctccgatt cgggcaaaaa 1224420
ccgcccggta tggcttgacc tttcctttcc acgccggtcg gcggtcttgc ccttatccct 1224480
cctgcaaatc gatttgcgtg ttcaagtcgg caaaatgccc gtcaaactcg aatctgaccg 1224540
gccgcgccct ttgctgctgc aaccagcttc ttaatgtttt catacccgaa aacagatagg 1224600
gaatcgcgct ttgcagaatc tgcgggcgga tatacataat gttgtagtgc atcgttatcg 1224660
gcgtttccac ataatacgca ttgcacaacg gtgtgcgttt cgacacggtt tcaaacctcg 1224720
ccaccaaatc gtccggcaga tacggcatat cgcacggcac aaccaaaagc cagtcagcag 1224780
ccgccaactg caaatcgttg gctgcggtac acaatgccga aagcgggccg aaatgctgcc 1224840
actgccgcgc atcgggaaaa atatgcggac ttcttcgagc atattcttcc aaattccggt 1224900
tggtgctgat ggcgatatgg ctgacctgcg gcctgaccct gtcgatgaca tggtctatca 1224960
gtgccttacc cccaaaagag caagcccttt gtcctcgcct cccatacggc tcgcctgacc 1225020
gccggccagt atcagggcaa aagttttcat tgcggatgtt ctcttggaaa agttcgaggt 1225080
tttcatgatt gcagtctgcc gttcccaatg actcaaaatg ccgtctgaag cagacggcaa 1225140
ataaattcat attatctgaa ttttatcata acatgattta acactgaagc ggtgcggatt 1225200
aagttttcct taaccaattc tttctgagca gttgtatcta attccaaaga atgatattgt 1225260
ttgcattatt tggaacaatt tttcgccgag catgatactg ccagcccgtt tttcagacgg 1225320
catcagcctt tccctgcgcc tgaaactcct gaccggactg tgggtcgggt tggcggcatt 1225380
gtctgtcgtt ttgacactgc tgctctcttt gcgtctggaa aacgcggcct ccgtcatcga 1225440
agaggcgggc aacttgagaa tgcaggcata ccgtctggca tacatggcgg gtgaaggctc 1225500
gccccgtgcg caaattgaca atcaggttgc cgaatttgaa aaaagtttaa aacgcattgc 1225560
ccaaagcgat gccatccatc cgctgattcc ttcggacacc cctcttgctt atgatttgat 1225620
acaatccatg ctgattatag attggcaggc acacatcctc cccccgctcc agtcctaccg 1225680
gcgaccgact caggtcgatc tctaccgctt tgccggaaac atcgaactgt ttttgcaggc 1225740
attggaaaat gccaacgaaa aaaacacatg gtggctcagg cgttttcaat gggcaattat 1225800
gttgatgacg ctggtgtcgt ctgtactgat gctgttttgg caccagattt gggttatccg 1225860
gccgctgcag gcgttaaggg aaggtgcgga acgcatcgga cggaggtgtt tcgatattcc 1225920
ggttcccgaa ggcggtacgc cggaattcaa acaggtcggg cgttgtttca atcaaatggg 1225980
cggcaggttg aaaattttat atgatgattt ggaaggacaa gtcgccgagc agacacgcag 1226040
tctcgaaaaa caaaatcaaa acctgaccct gctgtaccaa actacacggg acctgcacca 1226100
atcctacata ccgcaacagg ctgcagaaca ttttctaaac cgtatcctgc ccgccgtagg 1226160
agcagattcc ggcagagttt gtttggacgg cggatccgat gtttatgttt ccattcatca 1226220
tgcggattgc ggcacagcag cttcggattt ggggaagtac catgaggaaa tcttccccat 1226280
tgagtaccag aacgaaacat tgggcaggct gttgctcagc tttccaaacg gcatttctct 1226340
tgatgaagac gaccgcatcc tgcttcaaac actaggcagg caattgggcg tatcgcttgc 1226400
cggcgcaaaa caggaggaag aaaaacgcct gcttgcagta ttgcaggaac gcaacctgat 1226460
tgcgcaagga ttacatgaca gcatcgcaca agcattaacg ttcctaaacc tacaggtaca 1226520
gatgctggaa accgcctttg ccgaaaacaa acgggaggaa gccgcagaaa acatcagctt 1226580
tatcaaaaca ggcgtgcagg aatgttatga agatgtccgc gaactgctgc tcaacttccg 1226640
taccaaaatc agcaataaag aatttcccga agccgttgcc gacctattcg cccgctttac 1226700
gcaacaaacc gggataacgg tcgaaaccgc ctgggaaaac ggttcgttcc tgccgcctca 1226760

ggaagcgcag ctccaaatga tttttatcct gcaggaaagc ctgtccaaca tccgcaaaca 1226820
cgcccgcgcc acccatgtaa aattcaccct ttccgaacac ggcggacgct ttaccatgac 1226880
catccaagac aacggacaag gtttcgacac ggagaaaata ggagaaccca cgggcagcca 1226940
tgtcggactg cacatcatgc aggagcgtgc caaacgcatc catgccgttt tagaaatccg 1227000
ttcccaagct caacagggaa ccaccgtctc attgacggtt gcatctgaag aaagcttgaa 1227060
atgactatta aaattattct gatagacgac catacccctct tccgcagcgg cattaaagcc 1227120
cttttgtcgc gccaacacgg ttttgaagtc atcggcgaag ccgcagacgg cctctcgggt 1227180
atcaaaatga tcagtcggct gcaacccgat gtcgtcctgc ttgaccttga tatgcccggt 1227240
atgaacggac gcgaagcact ctcccaaatc atcagcatca atccgcagca ggcagtgatt 1227300
atgctgaccg tttccgaaga cagcgacgat ttgaccgaat gtatgcgcat cggcgcgcgc 1227360
ggctacctgc tgaaaaacat caacgccgac tttctgctcg aaagcatacg caaagccgct 1227420
gaaggcgata atgtattctc gcccgagatg accgccaaac tcgtcaaaag cctgatttcc 1227480
ccccaacctg cccaagggac gcaggcactc tcctcactta cccctcgtga actggaaatc 1227540
ttgggctatc tcgccgcagg acacagcaac aaaatcatcg cccgccacct cgatcttgcc 1227600
gaatccaccg tcaaagtcca cgttcaaaac ctgctccgca aactcaacct cagcagccgg 1227660
gtgcaggccg ccgtttacgc catccggcac aacgtccccc aacctgtgcc ggaataggcg 1227720
ttcagacggc atattagggg ttttaatccc cgtacggtca ttcggataac agaccaagca 1227780
tgtaagttta tgcccccata agtacgcttg gcatagcagt aatattgttc ggtttagtgt 1227840
tttccgtttg cccctatctg atactgcaat atcagctatg ccgtctgaaa acgcatcatc 1227900
atgatatttt cagacggcat aataaaaagc ggaaatacta atgcagggta aaatgttcca 1227960
ttccgaatcc cataaatata caatggctta tatcgtttaa gcatgtgtaa cccaccctca 1228020
tatcaatcaa tatatagtgg attaacaaaa accggtacgg cgttgcctcg ccttgccgta 1228080
ctatctgtac tgtctgcggc ttcgtcgcct tgtcctgatt tttgttaatc cttggattcg 1228140
gatttcaagt gcaacactag tgtattagtg gttggaacag attcaagaat aaaacacttg 1228200
gcgtttcgta gccaagtgtt tttcttggtc ggtggttcaa ctcatcttga accctgcgta 1228260
tctcccgatc actgatgtta cggaaatcgg tttgtttggg gaagtattgc cggatgagtc 1228320
cgttggtgtt ctcattcagc cctttctccc aagaatggta agggcgacaa aaataagtct 1228380
ccgctttcaa tgctttggtt attttggtgt gttggtagaa ctctttgccg ttatccatgg 1228440
taatggtgtg caccctgtct ttatgtgcct ttaatgccct aacagctgcc cgggcagtgt 1228500
cttcggcttt gaggctatcc aatttgcaga tgatggtgta gcgggtaacg cgttcgacca 1228560
aggtcaataa tgcgcttttc tgtcctttgc cgacaatggt gtcggcttcc caatcgccga 1228620
tacgggattt ctggtcgacg atagcgggtc ggtttctat gccgacacgg ttgggtactt 1228680
tgcctctggt ccatgtgctg ccgtagcgtt tgcggtaggg tttgctgcat attctgagat 1228740
gttgccacaa cgtgctgccg ttgcttttgt cttggcgaag gtagcggtaa atggtgctgt 1228800
ggtggagcgt gatctggtgg tgtttgcaca ggtaggcgca tacttgttcg ggactgagtt 1228860
tgcggcggat aaaggggtcg atgtgctgaa tcagctgcga atcgagctta tagggttgtc 1228920
gcttacgctg tttgatagtc cggctttgcc gctgggcttt ttcggcgctg tattgctgcc 1228980
cttgggtgcg gtgccgtctg atttcgcggc tgatggtgct tttgtggcgg ttcagctgtt 1229040
tggcgatttc ggtgacggtg cagtggcggg acaggtattg gatgtggtat cgttcgccct 1229100
gggtcagttg cgtgtagctc atggcaatct ttcttgcagg aaaggccgta tgctaccgca 1229160
tactggcctt tttctgttag ggaaagttgc acttcaaatg cgaatccgcc atcctctata 1229220
aaaatgccgt ccaaacccat gtttgagacg gcatttcgct atagaagcaa tcaggcaacc 1229280
tgggtttgat gctcgtctcc ctgacgctca cggatcaaac ctaaacggta aactgtttca 1229340
ccttgttcac ccaagagacc ctgaaccgca tcggcatctt cggcagcaac aataacgacc 1229400
atgccgatgc cgcagttaaa ggttcggtac atttcttggg tttccacatt gcccgccttt 1229460
tgaagccatt ggaagagctt gggcaattcc cacgatttag catcgatttg tgcaaccgtg 1229520
ttttcaggca acacgcgcgg cacgttttcg gtaatgccgc cgccggtaat gtgtgccata 1229580
cctttaatgg taaatttttc caaagcggca agaatcggtt tcacatacag acgggtcggc 1229640
gcaataacag cctcccgcaa ggttttgcca ttatcaaact cggcatccag atcgggattg 1229700
tcgcgttcga tgattttacg gataagggaa tagccgtttg aatgtgcgcc gttggaagcc 1229760
aaacccaata ccacatcgcc tacgccgatg ctgcggccgg taatgacatt ctctttttcc 1229820
accacgccga cggcaaaacc cgccaaatcg tattctccga cgggatacat acccggcatt 1229880
tcggcagttt ccccgccaat cagggcgcaa ccggattctt cgcaaccttg ggcaatgcct 1229940
ttaataacat cggtcgcgcg cggaacatcc aatttaccgc aggcaaaata gtccaagaaa 1230000
aacaagggct cagccccttg aaccaaaata tcgttgacac tcattgcaac aaggtcgatg 1230060
ccgaccgtat catgtttatc ccaatcaaag gcaagcttga gcttggtacc cacgccgtcc 1230120
gtaccgctga ccaatacggg attttgatat ttcttgccga tttcgaccaa tgcgccaaaa 1230180
ccgcccaaat cccccaatac ttccgggcgc atcgtgcgtt tggcaaacgg tttgatgttt 1230240
tcgaccagtt ggtcgcctgc gtcgatatcg acacctgcat cgcggtaact caatgaagta 1230300
ctcatcgttt ttccttggta aatggggatt ggacggtaaa ataacggggc gtattctacc 1230360
ttatttcacg tttgcaggtt cagattttta gacaatattg taaacagtcc gccatatgcc 1230420

cgcgcgtgtc gggtttggcg ggaccgtccg caggattaac gggcagaaac ccgcctgccc 1230480
ttcccctcaa ttccttatat atcgcgttcc atcaaaagac gcattgcttt tcttaaccat 1230540
tccttttggc agacgagcgg aaggggtttt ttgatgccat catcaaaatc aatattttct 1230600
tctttccggt tgaaaccccg gcattagggg tggtgaatct gattgcgtgc ggaagcaccc 1230660
gtttccgatt cggtgcggaa caaatggcgg cactttatgt accgttctgc gtgttgaaac 1230720
atataggcag ataaaaaagc cgcccgctga aaagcagacg acttatgttt tgtggcacta 1230780
atttgtcccg ataagcatta actatataat ttatttatca ttattggtgc ggacggagag 1230840
actcgaactc tcacacctct cggcgccaga acctaaatct ggtgcgtcta ccaatttcgc 1230900
cacgtccgca tgggaattgg acgattatac agattttgtt tttttgtgca aggttttcgg 1230960
cggggctgtt gatggcttgg ggtttggggc ggtaaaatct gtttttcgtc cgcctgacat 1231020
cggaatcggg cggttttttg tttttattga cggaatttgg gtatgcctgc tgctttgatt 1231080
aaggattttc tgctgactca gggtttgaag ctgccgcttg acgaggttcg ggcggcgtat 1231140
ctgacggcgc agacggtaat ggatatgggg acggcttcga tagaccgttc ggttttgtgg 1231200
cgcagtgatg agggttggaa acttgccgat tacctgtcgt gcgacaatgt ccgcgaagat 1231260
gcactgaaac ggcttttcat ggctttggat tcggtgtttt cgcgctcgac aggcgtgcgg 1231320
agtgcggcgg tctatgcctt gatgccatct gaaaaccagg ctttccaact gatatgcctg 1231380
tcccgacagg gcgaggtttt ggaaaacctg tgggatttgg atgaagcggc aggcaaggtt 1231440
tcgctggctt gccgttcggc gcaaagcggt tggatgaatg ttgcctcgga tgtacgccgt 1231500
tggttggatt tgggggagct ttcgggagaa cgcaatcatg cttcggcggc gcaaatttcc 1231560
attccggtct gcacggaaag tggcggtgtg ttgggcgtgg ttcatgtgga atttgaatgc 1231620
gcagagtgtg cgggtacggc agcacaggtg gaatgggtgg ctcttgcctt ggctttgtcc 1231680
gaacctttga aactgctgtt gggtatgtct gccggaaaag atgggagtga agatgtctga 1231740
aatgttgaac catgtggcat cctgccgcct gccgaccgaa tggggcgtat ttacgatgca 1231800
cggctttgaa gaggcaaacg ggcaggaaca cgtcgcgctg accgtcggta atttttcaga 1231860
cggcaatccg gttctgacgc gcatccactc cgaatgcctg acgggcgatg cgctgttctc 1231920
gagaaaatgc gactgcggac ctcaacttga agcggccatg agggcggtac agacagaggg 1231980
gcgcggcatc atcgtctatc tgcgtcagga aggacgcggc atcgggctga ttaacaaaat 1232040
ccgcgcctat catctgcaag accaaggtat ggatacggtt gaagccaatt tggcactcgg 1232100
gctgcccgtc gatgcccgcg atttccgttt ggcgcaatct atctacgaat atctgggcat 1232160
ccgctcggtc aaactgctga ccaacaaccc cgaaaaaatc caaaccctga aagatgcggg 1232220
gattaacgtg gtcgaacgca tccccctgca cgtcggggaa aaccttgaaa acaaacgcta 1232280
cctccaaacc aaagcagaca agctgggaca tctgatgtcg gaataaggca aagttgcagg 1232340
gaacgggcat cctgcgccgc ctttcgggaa acaggtttcc ataccttgat aaagcaataa 1232400
gttttatagt ggattaacaa aaaccagtac agcgttgcct cgccttagct caaagagaac 1232460
gattctctaa ggtgctgaag caccaagtga atcggttccg tactatttgt actgtctgcg 1232520
gcttcgtcgc cttgtcctga tttttgttaa tccactatat aaagttacag ggtgcggatg 1232580
caaacgcatt gcgagcgcgg gtttgaggca tacgcgcaaa catcttaata taatggattg 1232640
atatttatga ttttctccat catcgtccct atttacaatg tggaaaatac ctccgctgct 1232700
gcgtggattc cgtgcttgcc gaaaattttg ccgattatga aatgattttg gtcgatgacg 1232760
gttcgccgga cggctgcggg aagatttgcg acgaatatgc aggcaaatat ccgcatataa 1232820
aagtgattca tcaagaaaac ggcgggctgt cggatgcccg caacgccggt atccgggcgg 1232880
caaaaggcga ttacctaatc tttttggaca gcgacgatta ttgggccgat accaaccgtt 1232940
caaaaaacgc gggggggggat tctctttgat ttacaacaac ttgcagacaa aaaggttgat 1233000
ttgatcctgc atccctcgtc cttcaattac cgcgacatcc ccaaaggggc ggacttttcg 1233060
gataatgatt ttgtccgcca ttttgaaacg ctggtggagg ggcggtacta tatcgccaac 1233120
gcgtggacaa agattgtcag gcgggaaatc atcattaaaa acaatctgtt tttcccaaaa 1233180
ggatacattc acgaggattt cccgtacagt ttgcaattgg cgcgttttat caagactttt 1233240
gccttttacg ataacccttt ttaccagtac cgcgttctcg gcggctctat cagccacaac 1233300
atcaaataca aaaatttcag cgatgtgctg acgcatctcg actggggtgt ggatttttta 1233360
gtcgaaaaca aaaattcccc catctacggc ggtttgcaaa aatttgtctt cgacaatatc 1233420
ggctatctga ggtctatatt ggtaaggctt tatttttcca aaaacattat cctcatctac 1233480
cggaaatatt tttcatttaa agaaaaatgc agaaagatat tcggcgcgaa ggcaatccgt 1233540
ccggttttta tcgggaagac cgcattcatc ataggattgc cgatattgcg cctgctcgta 1233600
ccgcctatgc tgtacccggc aatcaaggcc gtttatcaga aattttttc ggaataagct 1233660
tccggcaaac cccgaatcgg aagcgggcgg gaagaaacag ccgccccgcc ggcggggatt 1233720
gcggcaatgc cgtctgaagc cacgaatccg gcttcagacg gcatctgttt accaaaaagc 1233780
aaataattcg gtttggcgaa aaaaacagat ttgcttttg gtaaatacgc gattacaatc 1233840
cgctacatcc gatttctaca aaggatgaaa cgatgaccga cacagccggt ctgcgccgcc 1233900
acaacctgcg gcagtggata gaaaaatact acggcggttt gcaaactcgt tttgctgaag 1233960
ccgttgccct caacacaggc gaactctccg ccctttgaa aaacaaatcc ttcggcgaga 1234020
aaaaagcccg taaaatcgaa caggcggcaa aaatgcccgc cttttggctc gataccgaac 1234080

acaccgcccg cccgtccgaa cacacaggaa aacacaccat gtcccatatc tcccccatcc 1234140
ccgaaatcct agccgacatc aaagccggca aaatggtcat catcaccgat gccgaagacc 1234200
gagaaaacga aggcgacctg ctgatggcgg cgcaattcgt cacgcccgaa gccatcaact 1234260
tcatgatcaa acacgcgcgc ggcttggtct gcctgccgat ggacggcgaa atggtcgaaa 1234320
aactcgggct gccgatgatg acccaaaaaa acggcgcgca atacggcacc aactttaccg 1234380
tctccatcga agccgcacac ggcattacca ccggcatttc cgccgccgac cgcgccctga 1234440
ctattcaaac cgccgtttcc ccgaccgcta aacccgaaga catcgtccaa cccggtcata 1234500
tctttccgct tcgcgcccaa aaaggcggcg tactcgtccg cgccggacac accgaagccg 1234560
gcgtcgacct ggcgcaaatg aacgggctga ttcctgcctc cgttatttgc gaaatcatca 1234620
acgacgacgg cacgatggcg cgtatgcccg aactgatgaa attcgccgaa gaacacaagc 1234680
tcaaaatcgg cacgattgcc gacctcatcg aataccgcag ccgtaccgaa agcctgcttg 1234740
aagacatggg caatgcgcct gtacaaaccc cgtggggcga gttccaacaa cacgtttacg 1234800
tcgacaaact ctccggcgaa acccacctcg ccctcgtcaa aggcacgccc gccgccgaca 1234860
ccgaaaccct cgtccgcgtc cacgaaccct tcagtgtgat ggacttcatt caagccaacc 1234920
cgcgccattc atggtcgctg cccaaagccc ttgagcacat ccaacaagcc gaaagcggcg 1234980
tcgtcatcct cttacaccgc accgaagacg gcgcatccct gctcgaccga accctaccca 1235040
aaggcgcaaa ccaagcctac aaatgggaca gcaaaagcta cggcatcggc gcacaaatcc 1235100
tcgccggcct caacgtcaaa aaactgcgcg tcctcgggca gccctcatct ttcaccggcc 1235160
tgaccggctt cggtttggaa gtcgtcggct ttgaagaagc ggaaaaaataa tatagtaaat 1235220
tcaaatactt tatatttgct ttatttattg cattatttcc gtgcaaacga aaacccggtc 1235280
tgttgggttg gattttgttt tttcaaattt cgggtaactt ctaattcgtc attcccgcgc 1235340
tggcgggaat ccggttcgtc gggtttttgt catttccgat aaattcctgt ggctttggtt 1235400
ttttggattc tctctttcag ggaaagaacg gcataagtat tttccaaacc aaacaaaatg 1235460
ccgtctgaaa ggctttcaga cggcatttta agtttgaccg gtttcattca tcggtattta 1235520
tgaattgaat ttcaacatcg ccaatctatc cttaatctct ttttccaatt cggcagattc 1235580
ggcgaaaagt ttatccaaat ccgctgaaaa tcccgccatt ttttgcgcaa attcgtcggc 1235640
ggaaatatcc acataatcaa tctttacctc gaaatactgc cccgccgaca agctgtgatt 1235700
cttcgctttg atttcatcgt agccgattac cacactgaaa tcttccactg cctgtttgtg 1235760
cgtgaaggta ttgcagattt ttttgttctt cttcgcggga aagtacggtt tttttgccgt 1235820
ctttaatttt ttcgcccaag cccgatgcgt cgattaatat agtggattaa caaaaatcag 1235880
gacaaggcga cgaagccgca gacagtacaa atagtacgga accgattcac ttggtgcttc 1235940
agcaccttag agaatcgttc tctttgagct aaggcgaggc aacgccgtac tggtttaaac 1236000
ttaatccact ataccacgtt gtctttattg gctttatcaa taaacaggat aagacctgaa 1236060
aaaaagccga tacgcctttt tggtgtaccg gctttgccat actgttctgc ttcagacagc 1236120
attgcttcat tttgccttta atacttcttc gtccagcgat ttcaaccatt ccagcttttc 1236180
gccgattttg atttccaacc cgcgcgggac gggttggtag aagtccggtt cgtccaagcc 1236240
gtcgggcata tagctttcgc cggcggagta ggcgttcggt tcgtcgtggg cgtagcggta 1236300
ttcgcgtccg tagcccaatt ccttcatcag cttggtcggg gcgttgcgca ggtggacggg 1236360
cacttcgtcg ctagcgtttt ctttgacgaa gtggcgcatt tggttgtatg ccttgtagcc 1236420
cgcgttggat ttcgcggcgg cggcaagata caataccgct tgcgccaaag ccagttcgcc 1236480
ttcgggcgag cctaagcgtt cgaaggtggc ggcggcatcg ttggcgattt ggaaggcgcg 1236540
cgggtcggca agcccgatgt cttcccaagc gatacgcacg atgcggcggg cgaggtagcg 1236600
cgggtcggtg ccgccgtcga gcatacggca gaaccaatac agcgcggcgt tcggatgcga 1236660
accgcgcacg gatttgtgca gggcggagat ttggttgtag aaactctcgc cgcctttgtc 1236720
gaaacggcgg atttgcgccc cgagactgtc ggcgagaaat tcggcggtta agtttttcag 1236780
acgacgtgta tcggcggcgc gtaaaagttg ttccaacaaa ttcaacaatc tgcgcgcatc 1236840
accgtcggcg gtattcacga gtaattttttg cgcatccgtt tcaatcgtaa actcttggta 1236900
ttcaggcaaa gccaatacct tggcaatcag ctttttcagg tcgtctgaag acaagggttg 1236960
caaaacatac acctgagcgc ggctcaacag cgcgggattg acttcaaacg acggattttc 1237020
cgtcgtcgca ccaataaagg ttagcaaacc gctttcgaca tgcggcaaaa acgcgtcctg 1237080
ctgcgccttg ttgaagcggt ggacttcatc gacaaacaaa atcgtcgcgc gtccctgctg 1237140
caaagcgatt tcggctttat cgattgcctc gcggatgtcc ttcacgccgg aaaatacggc 1237200
ggaaacaggc aaaaactggg cgttgaaact ctgcgccaaa atccgcgcca acgtcgtctt 1237260
gcccacgccc ggcggccccc acagcaacat agaatgcggc ttgccgcctt ctaccgccac 1237320
gcgcaaaggt ttaccttcgc cgatgaggtg ttcctgcccc accacgtcgt caagcgtatg 1237380
cggacgcaat cgttcggcaa gcggcgcgtc gggttctcgg gcaaacaaat cggtcataac 1237440
ggctccgtca acaggttttc aaacaatatg attatacggc agggaacggc ggcgtgccgc 1237500
atacggattc cgcccctccg tttgccttaa gccgatatta ggcgcatact ggaaaagacg 1237560
agagacttca cacaatatat ccggcacgga gaccgattcc gcatcggcat gacaataccc 1237620
aaatcagcgt ttcaattaaa cattaaggag actaaaatag aaaatttgct ttatctacca 1237680
ttgctttgtt gatttaatcg gcattatgtt ttgaggcgga agcccatgaa tatactaata 1237740

ttcaagagat ggaatgggtg tctttatttt ctgatccgca aagagacgat gatagtctta 1237800
taacccttaa agatgaaaaa atcactgtaa aaaactatat tgtgccttgg tggaaaaaag 1237860
gtgaaaactt tagaaaatta gaacttggcg gattcgcatt tgaagtgcaa ctttccctaa 1237920
cagaaaaagg ccagtatgcg gtagcatacg gcctttcctg caagaaagat tgccatgagc 1237980
tacacgcaac tgacccaggg cgaacgatac cacatccaat acctgtcccg ccactgcacc 1238040
gtcaccgaaa tcgccaaaca gctgaaccgc cacaaaagca ccatcagccg cgaaatcaga 1238100
cggcaccgca cccaagggca gcaatacagc gccgaaaaag cccagcggca aagccggact 1238160
atcaaacagc gtaagcgaca accctataag ctcgattcgc agctgattca gcacatcgac 1238220
ccctttatcc gccgcaaact cagtcccgaa caagtatgcg cctacctgtg caaacaccac 1238280
cagatcacgc tccaccacag caccatttac cgctaccttc gccaagacaa aagcaacggc 1238340
agcacgttgt ggcaacatct cagaatatgc agcaaaccct accgcaaacg ctacggcagc 1238400
acatggacca gaggcaaagt acccaaccgt gtcggcatag aaaaccgacc cgctatcgtc 1238460
gaccagaaat cccgtatcgg cgattgggaa gccgacacca ttgtcggcaa aggacagaaa 1238520
agcgcattat tgaccttggt cgaacgcgtt acccgctaca ccatcatctg caaattggat 1238580
agcctcaaag ccgaagacac tgcccgggca gctgttaggg cattaaaggc acataaagac 1238640
agggtgcaca ccattaccat ggataacggc aaagagttct accaacacac caaaataacc 1238700
aaagcattga aagcggagac ttatttttgt cgcccttacc attcttggga gaaagggctg 1238760
aatgagaaca ccaacggact catccggcaa tacttcccca aacaaaccga tttccgtaac 1238820
atcagtgatc gggagatacg cagggttcaa gatgagttga accaccgacc aagaaaaaca 1238880
cttggctacg aaacgccaag tgttttattc ttgaatctgt tccaaccact aatacactag 1238940
tgttgcactt gaaatccgaa tccaagggca ttttaaaatc ccgaagcaga cggcacgcgc 1239000
cccgaacatt cgttctttaa cgcccgtttt cagaatgccc gcctgcgggc atattttgcc 1239060
cgatcagttc ggctatcctc tcgccgtcaa actgcgtttt gaacaccacc ttgatttctt 1239120
tggaaatctc ggcaaacagc ttttcggcat gtttgatttt ccgctcttcg ctttttcgca 1239180
aatcttccgc cccgtcagtc cctttggctt caatcacaaa gttcaggatc tcgccgcttt 1239240
tggttttcac gatataggca aaatcgggcg aatacgtgcc gccgccggca acagggattt 1239300
tgatggagtt tctcggtatt ttggtaaata cgattacgcc ttcaatttgg ttgttggcga 1239360
cattttcatg ttctatatcc gaatcgtaga aaatctcgcc gaagagatag ccggcggcag 1239420
gccggtgctc cgtatcttca aatctgccca aatctgcttt tttcaccgcg cgcggtttgc 1239480
cgtctttatt ggtaaatttg gtcggatgga ttttgctgcc gacaagccgg taatccagtt 1239540
cgaatttatg gaaggaatga tgaagcaaaa accggttgaa gccgtttttg atttgggcga 1239600
tggtttgcat attcaaaaaa tcgccaatgt tcagttcgtt gcggatgcag taaaacgcct 1239660
gatgcaaagt ctgcatacgg atttttgccg tttgtgccag tttttccaga aactctcggt 1239720
aagtcattgt gttgaaacgg ataaaatctt catcttcaaa actgtctatg cggcgggaaa 1239780
gcataagccc gttgttgata taagcttcgt ttaccgccgt gcgtatgcct gcctgtggga 1239840
atttggcagc gttttcatgc aaataggcgg taaataaatc ggcaaattcg gcttcatcct 1239900
tgattttata ctgcaaaacg gctttatggt gaatcagctc ccacaaggct ttgagttctt 1239960
catatttgcc ttcgcgcatg atgatggtgt ctttgccttc gtctttggcg ttgctgactt 1240020
tgcctttgtc caaacctttg gggaaggctt cgggataggc ggcttttaat ttgtcatagc 1240080
cgtcttcggc aaagttttca ttgtcgtcaa tgatgccatc taaaaacagt tggtttacca 1240140
ataccagcgg tttgatatcg gggtattttt gcaatatttt ttgtttcagc tcttcggtaa 1240200
actttttgga gatttcttcc tgaaaagaat tgtcgttgat ttcgccgaca agctgcttca 1240260
caaagtcttt ttcgctgcta tcgacaaaat aattcagttt gtacggtaca tcgcgcaccc 1240320
gcgccatcag ctcgtttacc ggcaggcgca ggccgcgtcc gacttcttgc agcttggaag 1240380
tcgtgctgcc gctggaacgc agtttgcaaa tctggaaaac gttgggattg tcccagcctt 1240440
cgcgcagcgt ccatttggaa aaaataaagc ggcgcgggtt gtccaaagac agcagttttt 1240500
ccttatcgtg caggatttca ttgatttcct gctcgattct atcgtcgctg tctgtattgt 1240560
ctttggaaaa atagccgccg tggcaggcgg atacatcgtc caacgtcttt tgcaggtaat 1240620
cgcggtaaaa cgggtcgctt tccgttttca gacggcgtgc cgcttccgcg cgaatccagc 1240680
tttcaaattt atctttcagg ctgcctgaaa gctcgttgcc gctgcggtag cccgcgatat 1240740
cgtcaataaa aaacagcgtc agcggcttga ttttgggctg tggcgcgcgt tctgccaaaa 1240800
gcgcgcgttc cagcttgaaa tgttcggcaa ccgcccgctg catcatcgca tcctgcaccg 1240860
tttgcgaata ggaataaggg ttgatgacgg cacccgtttt caactccaag ccgttgctta 1240920
acaccaccac ggttttattc attttgtcga ttttcaaatc cgaaatagcc ggatggattt 1240980
gcgccaaatc ttcgcctttt gccagtttga acgtctgctt tttgtccttt tcgtttaatt 1241040
caaatttcgc ttctttgccg tccgacgaca ccagttttac cgccgcatcc atgccgccct 1241100
gcatttcttc ctgaaacacg cgcacgcctt tgaccagccc gtcgttaaac gcgtctactg 1241160
ccgtcaaacg gtaaagcaag ttgtaatatt catcgttaaa tgttgcaccg tagcgcaaaa 1241220
tatattgcgg ttttaagcgt ttgatattgc cccacgtttt cgcgctatct cgggtcggga 1241280
atttatgcgg ttcgtccaca atcataaacg ggcgcacggc agccaatgca tcaacgggat 1241340
tgtcaaacaa atccttcaat gccttgtcgc ccgtatcgtt catggacgac gaattaacca 1241400

tgcccgcgtt aatcagcagc acatgaattt cctttttgtt ttccgctttg acaaattgct 1241460
caatcgttat gggcgcattg gactttttgc ccttattctt tttcgcgctt tccaccacat 1241520
aggttttcag gcgtacgcct tcataatcgc cgccgaaatc ctgttcaaaa tgctctgcca 1241580
aagccttgct ttgcaaaaac tgctgtgttc ccgccttaat ggacaaagtc ggcacgacca 1241640
cgataaattt gaacacgccc agccaacggt gcagctcgaa catggtttgt gtgtaggtat 1241700
aggttttgcc cgtgcccgtt tccatggaaa tatcaaggat attttggtcg tccgaacggt 1241760
cggggaatcg gccgtctata ccgttttggc tttgcacttt caggatattg tttgcgtatt 1241820
gttttgcagc aaacaaaagt tcgggatttt cgtctgccgt ccgatatttg ggcgttgccc 1241880
cgtcaaacac gcccaaaacc gccgaaaccg cccgcatttg gtgcggctgg tttttctcgt 1241940
aattaaaacc gctcatgaat tgcctccgtc aaaccctgat aaccacattc aactcaatct 1242000
ctttttattt ggcataaccg cgaaccgcct ggtcaagttc gtgctgcatg gcgcttgcca 1242060
tattgctgcc gaatacaatc acgcggttgg gattgaaatc cgcatcgtcg tccagcttgc 1242120
ggataaacgc caacaaatcg gcggaagtaa aaccggcatt catcagatac agccgttttt 1242180
cgcacagata cgccgtgtaa gcccctaacc gcacaggctc aaccggcgtg gtcagtgccg 1242240
ccccgtcata cagcgtccag gtggtcagaa gcgtttgcag ctgttcttcg cttaattcat 1242300
cgttaagcgg caaatccggt tgttcgggcg aaaaatcctt gtccggatgc tgcctgaaat 1242360
tgtctgccgt ttgaaagatt ttgaagcccg aatcgcccgt gtaatcggga tgttcgacgc 1242420
ggattttggc ggcggctttt tctatgcggg ctttggtgat gtcgaagatg gtcgggtagc 1242480
ctgctttacg ggcttcggat ttttcagcgg ttttttcggg aagctgtaca cagatatagc 1242540
ggcggttacc gttttgtcct tcggcgttaa gctgcatcac ggcgtgggcg gttgtgccgc 1242600
tgcctgcgaa gaagtctagg attaggtcat tactctttga acttattgaa actaaaaatt 1242660
taatcaattg actaggcttg gggaaggtaa atattttgct accaaataaa tctgtgattt 1242720
cttttgtgcc ttctttagtc attccgatat tttcaggtag cgtcctacta aaaatagcca 1242780
aatattcggc tactgcatcg gctaatgtac caacattttc aggtaatcta ctgcttacag 1242840
ctactttgcc aaaatcctcg ccagcttttt tcatgtcgtc atctttaaag taacgcataa 1242900
ctggattgct gatatttaag aaatcataat catcagggaa aacgattttt cctttattat 1242960
aataatcttg aaatgtatct ttggttacac gccaagttgc atttggattt gctggatatt 1243020
tttttcctgt cttgggatca accattgtga aaaaactatt tggcctttcc gccgcagttg 1243080
tttgtttcgt taagtcgtgg gtacgccaag gacgatcggg gaaatcatca gtctcataat 1243140
agcgtcgttc cttgccttta gttgctgcaa taaattggca agattttgcg aatacaaata 1243200
tccattcata atcctgcgaa ataccaaaag gcacatctga tttagctgtt ctttttcgcc 1243260
aaggcaattg tgcaacaaaa ttcccttccc caaacacttc atcacacaac aatttcaact 1243320
gcgccgcttc gttatcgtca atcgagataa aaatcacacc gtcgtccttt aacagttcgc 1243380
gggcgacata caggcgcgga tacataaagg tgagccatgc gctgtgcgag tttgagcctt 1243440
tgtcggtgaa atctaaaatc cgcgcggctt cgtcttcatc aatattggct aggcgggcaa 1243500
gttcagcggg tgtgaatttg cggtcgtcct gatagacaaa gccgtctgat ccggtgtttt 1243560
agggcgggtc gatgtaaatc atcttcacgc tgtttgtgta ggcgtttttt aagtgtttca 1243620
acacttctag attgtcgcca cgaatcagca ggttttggct gcctgcattt tcgggcttgg 1243680
cgttgtgcgt cttgtcttca cttatcaggg tttcgggcgg caggttgcga agcaggcgag 1243740
catatgattt gcccagccag ttcatttcgt aaaattcgcg tccgatgtcg gtctgcggcg 1243800
cgatttcggc ttgtaatctg tcgataagga aatttccgtc tgcgtcaaaa caggcgggaa 1243860
acagtttttt gagctgttcg agttgggtag agttggcggt aatgccgtct gaagtgtaga 1243920
ttgcctcagt gttcgccccg gctgtgtcgc ttagatcagg gcttgggttg ggttgggttg 1243980
ggttgggttg ggttgggttg ggttgggttg ggttgggttg ggttgggttg ggttgggttg 1244040
ggttggcagc attttaaaat cctcggtttg aatttgtcaa tatcaactgt ctgttttaaa 1244100
atattttttt actttaaacg gcgtttttttg ggaaacgggc gacgccgtct gaacgtctgt 1244160
ctgcgtgtta ctgcccgaca acaacgcgac ggattttgac gggctgtacg ggtacgtttt 1244220
gataaaagcc gcgcgtggcg gttttgacgc gggcgatttt ggaaacggtg ttcatgccgc 1244280
tttcgaccct gccgaaaacg gtatagccgt attgtccgtt tttgtagtcg agcgaagcgt 1244340
tgtccgccag attgataaag aattggctgg tggcggaatc gggggctgtc gtccgcgcca 1244400
tggcgatggt gccggcggtg tttttcaagc cgttgccgga ttcgttggca acggccttat 1244460
cgcttgcctt ttgtgccaag tcctcggtca atccaccgcc ctggataaca aaaccgtcga 1244520
taacgcggtg aaaaacggtg tcgtcgtaaa agccttttcg ggcatagcgc acgaaattag 1244580
caacggtttt gggggctttg gattcgtcca aaaccaaacg gatattgccc atatcggttt 1244640
ccatcaaaac atgggttgcc gccatagacg gcagggaaac cgccaaaagc agcgcggtta 1244700
aaacggtttt gaatttgggt ttcatcccgt cctcctcaga ccttcagaca gcattttcat 1244760
ttcctatgcc gtctgaaggc tcgttaacgc tattccaatg cgtctttgag tttttgttcg 1244820
attaaatccg catcaaacga tttggcaatc aattcaaaac gcgagtcgcg ccgccaagac 1244880
acttggttcg caccccattg cccgtccacc cagttgagcc acacccacgt tcccaatact 1244940
tggaacacgc ctttggcacg gacgagtcct tcggtcatat tgggcaaatc attgaagaag 1245000
ttggtcaatt tttcaccgtc gaaatcgcgt ccggcgggga atgtgaaacc ttgcgactgg 1245060

aagcccatcg tgttgtccgg cagggctttg aggcggtagc gtgatttttc gatgacgggg 1245120
atgtcaagcc attggatatc gagttgtgcg ttttgaactt cgaccacttt agccttgggc 1245180
gggaacagtt ttgcggcttt gtcgtgaaat tcggcaagct gttcgggggt gcataaatcg 1245240
gttttgctgg caaccaatac gtcgcagatg ccgatttggt ctttatacaa tgcctgctgc 1245300
gcgtaatcgg ggttgatgaa ctggcgcgga tcgacgacgg taaagactgc gccgatttcc 1245360
aaaaggctgt ccagcggttt ggttttcagt tcatcaatga cactggcggc gtgcgccagt 1245420
ccgcttgctt caatcatcag gcggtcgggc ttggcgtcgc gcagcatttt ctgcacggtt 1245480
acgcccattt gcgggccggc ggtgcaacac aaacagccgc cggcgatttc tgccacaggg 1245540
atgccgttgt cgctcaatac cgcgccgtca atgccgattt cgccgaactc gttgacgatg 1245600
atgacccatt tttcgttcgg gtctttttgc gccatcaggc ttttgagcgc ggtggttttg 1245660
cctgttccca gaaaacctga aatcaggtgg actttggttt ttttcatttc tatgtgatgt 1245720
cccactttaa aatttgaaga tagggtgttt taaatgatta aataatgtta aaagtgatgg 1245780
cagctgttat catgttcttc atcaattgac aattgttcca gcaaattcga tatatcggct 1245840
gacatcgccg gtatgacgtt caaaaccgtc tcccaaacga cagacaggtt catttcaaag 1245900
tagccatggg caatcctatt gcgcaatacc ccctcatccc tatccaattc aaatatttgg 1245960
tttcctcggc aaattccgga tacgatttaa ggatatgggt tgcgttttca ccgataagga 1246020
acaaattaaa aacgactgcc tgcacagtct ttgtgtcggc agaaaattgc ccataatcca 1246080
ttttgtcggt atatagccgg atatattgcg ctgcctgcaa tatctgtttc aaatagaccg 1246140
acaattcttt ctgcattttc ataatgcgac agcctcagtc aacaccttat ctctaaagtg 1246200
ggccgagatg tcatcgggtg tcagcaaatc gacctttatg cccaagagtt tctgcaattc 1246260
ctcttgcagc ccgcccaaat ccaataatgt tgtccctgtt tttgcatcca ccaacaaatc 1246320
aatgtcgctg ttttccgtat catctccgcg agaaaccgaa ccgaataccc ttggattgca 1246380
aatcaatgga tatttcccga aaactgccaa tatttctttc tttctgcttt gcaacaaaag 1246440
agacggtttc attatctgct cctttcgaaa ggcttattat caatgcaaac caccgattac 1246500
tgctgacatt ttttacaaat cccggttaaa acaacgtgtt cttctttcag cgcaaagccg 1246560
ctttcggcaa cgcctgcgcg cagtgccgcc cactcgtggc tcagggtttg ctcgtccgcc 1246620
gtgccacatt cggtgcagac caaaataaac gcgctgtggt gcgcttcggc ttcttcgtgg 1246680
tcgtggcaat ggtcgtcgca ctcgtgctgc gcgtggctgc acaaaatata gccgttgacc 1246740
gccgccactt tgtgcaaaac gccctgctcc gcccaaaaat caagggcgcg gtaggcggta 1246800
ggcggtgcaa gcacgccctc gctttgctgc tgcatctgcg acaagacgtt gtaggcttta 1246860
atcacgccgc tttgctgcaa gacaatatct aatacctgct cgcgcaaagc ggttacctgc 1246920
aagccttcgc tgcgtgcctg ttcgataatt ttctgtttga aatttgtttt cataaattct 1246980
ctgtttatgc cgtctgaaca accgatacgg caggaggcgg ttttatattt gtattcaatt 1247040
gctttatttg gaaatctttt ccaacaatgc ccgacagccc gcatccgcaa gcctgaaggt 1247100
ttcttcaaaa tcgcccgtat accacggatc ggggacatgg tcgtaaccgc tttcgggtat 1247160
caggtcggtc agcttgaata ttttttccgg ccgcctgccg aaggttcttt ccaattcgga 1247220
caaattcttg ccgtccatcg cgatgatgca gtcaaacgcc gccgcatcgc tttggcggat 1247280
tttgcggctg gtaaagcctg aagcatcgat accgtgtttt ttcaatatct ttgccgtctc 1247340
gcggtgcata tcttcgccgt cgtgccagcc cgatgtcccc gcgctgtccg cttcaagggg 1247400
aatgcccgct tcggcggcgc ggcggcgcaa aatgtattcc gccatcggcg aacggcagat 1247460
gttgccgagg cagacaaaaa ggattttcgg ttttttcata tcccctccct gttccggcgc 1247520
gatgccgtct gaagcggaaa ccctttcaga cggcatcggt cggttaatcg taaccgcata 1247580
aggcagaacc agccaaaaca tccatctgga agaaatggtt tggctaaatc ttaggcatat 1247640
ttaataagtg tccaatatta gaagccgtat gctccaaata gaggctggca tttttcaaac 1247700
tatcttctaa aggttcactt ttctccaaaa tagaaaaggc agcttggata ttttcaaatg 1247760
gcagggaagg caaatcttca acgagactgc cacaaatagc gacaacagga actccgacag 1247820
gggttctttt tgctacacca ataggcgctt tccctgctaa actttgacga tctagtcttc 1247880
cttcaccaac gataaccaag tcaacatctg acactttctt atcaaagtca atcaagtcca 1247940
ggcaggtgtc aattccagat acgatacttg cctgagcaaa ggcgcacaaa ccaccagcga 1248000
tgcctccacc agctcctgct cctttaagtt ttaatgttgc aggggagact ttttcataaa 1248060
aatcttgtat tgcctgatct acggcctcaa acatagtaga atccaaccct ttttgcttgc 1248120
caaacgtata ggtcgcacct tggtgtccac ataagggact cacaacatct gctaaaatac 1248180
gaatgtgaac atcttcagga atttcatagc gattttctgt tgaaacagaa gctaggttta 1248240
gtaaggattg accgcaaacg ggtaaggcat ttccatcctc atcataaaat tgataaccta 1248300
aaccagcagc aatcccaata cctccatcat tactggccgt accgccaacg ccaatataga 1248360
tttctttaat ttcttgacta atgaggtggc gaatcaattc tccaatacca cgagtttgga 1248420
tttgtaatgg atttcgtttc tctagcggga tttttccaag accaaccaaa tcagcaactt 1248480
cgaatagggc tagttgttct ttttgaaaat agcgcatgac ttctttttgt ccaaaaggtc 1248540
ctgtcacttg gagacatttt tcttctaggt caagagaatg tcggatagca tctacagtgc 1248600
cttctccccc atcaccgaca ggacagagga gacattccac atctgctatc gattgttgga 1248660
agcctctttt tattgcttca gctacctgtt gagctgtcaa gctttcctta aacgaatccg 1248720

gtgcaattac aatcttcata tttataattc atcctttcgt ttcactcaag gcacaacaca 1248780
gaatgaaaaa gtgttgtgct ctttattttg atttattata taaatgagaa agcctatcac 1248840
tactacaaat cactatgcgc tgaaaaacgg attgtgccct tcccgtttca atgcttccgc 1248900
atagctcggg atgctttcct gttcgcccaa gggattgtgc aacaggtaaa cgtgttccac 1248960
gcggcactcc gccatcaggt cgaggaaatt ttgctggatg acggcgatgt tgtcggtaaa 1249020
ggaaagctgc cacgaaaaca cctgcggcac gacttcaaac gcgccgtcgg tcgcattaaa 1249080
cccgaataaa atctgtccgc ccgcctttgc cgcagcgacc acgccgacgc gcggactctg 1249140
catacggacg gcgcgtatcg cgtttgccgc aaacacatcc atcgccatac gttggcgcgt 1249200
gtggctgccg tctgaaagcg cgcgtaccgg agaatccggg gaaagcggat tggcaaacag 1249260
cgtaacgcct tccgatgcaa gattttcctg ccatacctgc atcaggggca gacctgcctg 1249320
aagaaggttc tgacccaatg ccgtctgaac tttgccgctg ccgaaaccca gcgcgtaaac 1249380
gacgtggacg gactgccctt cgggcaacag caaagggcgc ggggcgaaac ggcgcaagtg 1249440
ttgcgccgcc gcttcggtat tttgtttggc acgccaccac tcatccggcg cgaccgcgct 1249500
caaatcggaa gaatgcacaa gataaggcag ccattgcgtc tcttgcgtca acgcgcgcag 1249560
gtgcggatag ttttgcaggc aggcaaacaa tgcctccgtc ggcagcttca tctcgattgc 1249620
acgttctttt ttgcagccgg acaccagcac gaccggcagg gcgaaccact gcacttcgcc 1249680
ttctttctcg caatcgagta ccgcgttcac actggaaagc agcgcggcat aagttccggc 1249740
atcgggcgac atcgtcagcg cgagggaaag gttgatatag tggttttgct caagcattcc 1249800
cctgatttcg gtttgaagtt ggccggacga gagtttgcgc gaagcctggg aagaattatg 1249860
cgccaactgg taggcattga gcagcaggtg gtttttaatc ggattttggg gatacgggcg 1249920
cgtatcgggc aaggtaaatg tctggttcat atgtgtcgtg caccgctttt cgcagtgtgt 1249980
gtctttcctg tctgaaaata tatcggacgg attgccgccg ggacttgccc gtcaatccgc 1250040
cgaaacgaga aaatgcctgt ctgccaagtc tgccaatatt tcttccacat acacttcggc 1250100
aggcggatgg aatgtcaaac cgtcgggcgt ggtgctgacg atgttcaagt tttcggcaac 1250160
cgtttcaatc aatgcctgtt tgctcgtcgg ccgcgacaat tggcgcataa tgtacaaatg 1250220
cccgtatccg aatgaggggg tgctttcgtt gtaacggttg gcaggccgga caaacgcttc 1250280
cgcgccgtgt tccacaaagg cggcggcata gtttgtaaag cgcgccggca cataggtttt 1250340
atggtcttca aaaaatggtt tgcccgcatt gtctgatgaa acagaaaaac gcccgagaat 1250400
ggtttgcaat aaaagctggg aattgatttt catgcggtca aattccaaac cgggaaatcg 1250460
gcgggcgaga ttttccgcaa catcttccgt tttaaacgcc tctccggttt tcatcacatc 1250520
gcgtatgccc gaaagcagga tatcgttttc atcaaagttg attgtttccc ctcttgccgg 1250580
gcggaaattc aaactttcta tcacttcgac ggcaaccgac tcatcacgcc tgacagtatc 1250640
cccgacttcc tcacggcata aaagcgaacg gcggaattgg cggtcggata aaatatcact 1250700
gtaaaattct ttggcaatat aatcgtcccc tgccaatgcc agaatccgct cccgcgtatg 1250760
ctccgccatc caagaaacaa aagacacgtg caaattggta tccccgatat atgcgagcct 1250820
gtggcggtta gcccattcga tgaagccgtt gacgtaaatc gggtcgttaa acgcctccat 1250880
atattcgtgt gcgatgtaat aaaaattatg attcaatatt ttttgaatcg ccggaagttt 1250940
gccgccgccg tccaagccct tgtcgttttc caaaatttcc gccagcgcct tgagcgcgtc 1251000
caagcctttc cgcgtccgcg cttccaaggg ttcttcaagc acatccctgc cggcaaagta 1251060
cataatttcg cgcaactgct cctgccgttt ccagccgggg taaacattgt atgaaatata 1251120
ggcaatgccg tgtttggtca ggttgttcca gcaaatcgaa aaaattttgt ctttaactgc 1251180
gtcaggcacc cacgaccaaa tgccgtggac gatgatatag tcaaacttcc cgaatgactc 1251240
atcgatggtc aaaatatctt tttcttccag acgcacattt ttcaagccca ttttttcaat 1251300
gatggcgttg ccctgtgcaa cctgcctgcc ggacaggtcg ataccgacaa attccgcatc 1251360
cgggtaataa agtgcctgcg tgatgatgtt tccgcccatc gaacagccca gctccaagac 1251420
cttggcattg gcggcgggcg cgggctgcaa acccatcagg cgggcgcgcg cctccaaatt 1251480
attgatggcg gtttgagaga atgcgccgga ttcgtacatc aaatcatcat atgaattttt 1251540
gatgttggac acgtccggca caccgttctc cgttgcagca cgcgcaaagg cggctttttt 1251600
cttcttgttc ggcatatttt gtttgtctga aggcactatt gcccgcaagt ttaaccaatt 1251660
catcctaccc gttcaactaa atcaaatgcc atctgaaggc gcggagcgta cttcagacgg 1251720
catctgggag gcgcgaaggc ttcagacggc atttttgccg ctttatttca acgcggcatc 1251780
atagccgccg tcttcaacgg cttcaatcaa cgcccctgca tcggtttgcg cggggtcgta 1251840
tccgacggtc gcacttttgt tttcaaggct gacttcgacg cttgccacgc cttttacgcc 1251900
ttccaatatc cgggtaacgc ttttgacgca gccgccgcag ctcatgccgc cgatgtcgag 1251960
gataagggtt tccatgattt ttcctttcgt tggtactgca ttctgacggg cgttattgta 1252020
agtcggggcg tgaacttggg caaacgcgga aacggtgcgg cggtttgaaa aaatacggac 1252080
gcttgcgcat aatggcggca attcccatca ggacaacaac aatgaacgct tcgcaaaaac 1252140
cctggttgag catcattgcc ttggcaatcg gcgcatttat agtggattaa caaaaaccag 1252200
tacgtcgttg cctcgcctta gctcaaagag aacgattctc taaggtgctg aagcaccaag 1252260
tgaatcggtt ccgtactatt tgtactgtct gcggcttcgt tgccttgtcc tgatttttgt 1252320
taatccacta tacaacgtcg gtatcggcgg cggcgcgctg ctggggcatt gggttacgca 1252380

atactcgggc atttcctgca tcggcgttgc gggtatgctg acggcggcgg caggtttgtg 1252440
ggtctgcctg aacctgaacc gccatatccg aacctgaccg cacgatgccg tctgaagccc 1252500
ctcccgccct tcagacggca ttttgattga agaaatatcc acccctgctt aaaataacgg 1252560
gctttgccgt gttttaacgc ctattttttg ttccaaggat ggtttatgcc gctgctgtct 1252620
gtcgagttcg cactgttctt tctcgccttc ctgccgattt actggggctt ggcgaaatac 1252680
ccgtccgtcc aaaacctgct gcttttggct gccggtatgg gctggctcta ccatatcggc 1252740
cctgtatttg cggcaatcat cgtcctttat tcctcctgcg tgtacctttt gggcgaactg 1252800
ctccgttccg atcgcgaaaa tacgcgccgt ttctggctgg ggtgcggcat tgccgcctcg 1252860
ctgaccgtct tgggcttttt caaatatttc gactttttcc gcccgatgat tgcccaatat 1252920
gccggaaaag gcggcgcaat cgacatcctg atgccgctgg ggctttcgta ttacaccttc 1252980
cagtcgctcg cctatctggt ttactgcttc cgcgccccgc acgccgcgcg tttcagctgg 1253040
cacgagctgc tgctgcacct gagtttttttc cccaccgtta cctccggccc gattatccgc 1253100
gccgccgcat tcaaaagcgc agacggcgag caggcaggcg cattggcgca aatccgtacc 1253160
cgccgagcgc gttcgcccgt ccgccccgca ctcgccgttt ccctgattct gctgggtatt 1253220
gccaaaaaat ggtggctggc ggggatgctg gcggaaaact gggtgtcgcc cgtatttgaa 1253280
aatcccgccc aattcgacgg ctggggcgta ttgggcggcg tgtacggcta taccttccaa 1253340
ctctttttag acttttccgg atattccgat ttggttatcg gcatggcgat gctgctgggc 1253400
tttaggctgc ccaaaaattt ctccgcaccg cttcgtgctt taaacatccg cgcattttgg 1253460
gacaaatggc acatcagcct ttccacctgg atacgcgact acatctacat ccccttgggc 1253520
ggcagcaaaa aaggcttttt acggacacag ctcaacctga tggcggcaat ggtgctctca 1253580
ggcatctggc acggctacgg ctggaacttc ctcatttggg gcgcgctgca cggcacggca 1253640
ctggtgctgc tcaacacggg cgaccgctat ttcggacgcg acgcgctatg ccgtctgaaa 1253700
tacttcgcgc cgctctcatg gctcattacc ttccatttcg tctgccttag ctttgtcgtc 1253760
ttcaataccg caaatcccga cgatgcaggc gcagttttca gtgccctctt tgccaatgcc 1253820
aacggctgga atgcgccgca acaggcaaac atgctgttgc ttgcctcgtt tgcatccgtg 1253880
atgctgctct acccttacct gcaacgcgct ttcgacggcg cggtcaaagg tttggaaaaa 1253940
atcccgatgt ggctgtggtt tatccccgtt tccgccgtcc tgctgctgat tatcgtcctc 1254000
gccccctcgg ggatacccgg ctttatttat gccaattttt aagggtttgg acatgaaaaa 1254060
ctttctttcc cttttctcct ccatactgat gtctgccctg attgccgtgt ggttcagcca 1254120
aaaccccatc aacgcctact ggcagcagac ctaccaccgc aacagcccgc tcgaaccgct 1254180
tgccgcctac ggatggtggc ggagcggtgc ggcgttgcaa gaaaacgcct acgccctttc 1254240
agacggcatc aaagccttcc tgtccggcga aacgccgccg acggctcaag acggcggttc 1254300
ggcagatatg ccgtctgaag ccgccgcatc cgaagccgtc cctcaaaccg gtgaaacaga 1254360
atggaaacaa gacaccgaag ccgccgccgt ccgcagcggc gacaaagtct tttttgtcgg 1254420
cgactcgctg atgcagggcg ttgccccctt cgtgcaaaaa agcctgaaac agcaatacgg 1254480
catcgaatcc gtcaacctca gcaaacaaag cacggggctg tcctacccct cattcttcga 1254540
ctggccgaaa acgattgaag aaaccctgca aaaacatccc gaaatcagcg tactcgccgt 1254600
cttcctcgga ccgaacgacc cgtgggattt ccccgtcggc aaactctatc tcaaattcgc 1254660
ttccgacgaa tgggcgcaag aatacctgaa acgtgtcgac cgcatccttg aagccgcaca 1254720
cacgcaccgc gtccaagtcg tctggctcgg catcccctac atgaaaaaag ccaagctcga 1254780
cggacagatg cgctacctag acaaactgct ttcggaacat ttgaaaggca aaatcatcct 1254840
gattcccacc acgcacaccc tgagcggcgg gaaagaccgc tacaccgact ccgtcaacgt 1254900
caacggcaaa cccgtccgct accgcagcaa ggacggcata cactttaccg ccgaaggaca 1254960
aaaactgctg gcggcaaaaa taatggaaaa aatcgttttt gaaccaagta cgcaaccatc 1255020
aagtacacag ccatgaaccc caaacacctc atcgcatttt ccgccctatt cgccgccacg 1255080
caggcagaag ccctacctgt cgcctccgtc agcctcgaca ccgttaccgt ttccccgtcc 1255140
gccccctaca ccgatacaaa cgggctgctg accgactacg gcaacgcctc cgcctcgcct 1255200
tggatgaaaa aactccaatc cgtcgcacaa ggcagcggcg agaccttccg tatcctgcaa 1255260
atcggcgact cgcataccgc cggcgacttc tttaccgaca gcctgcgcaa acgcctgcaa 1255320
aaaacttggg gcgacggcgg cataggctgg gtttaccccg ccaacgtcaa agggcagcgc 1255380
atggcggccg tccggcacaa cggtaactgg caaagcctca ccagcaggaa caacaccgga 1255440
gacttcccgc tcggcggcat cctcgcccac accggcagcg gcggcagcat gaccctgacc 1255500
gcatcggacg gcatagcaag caagcagcgc gtttccctgt ttgccaaacc cctgcttgcc 1255560
gaacaaaccc tgaccgtcaa cggcaacacc gtctccgcca acggcggcgg ctggcaggta 1255620
ctggatacgg gcgcggcact gcccctgacc atacacaccg aaatgccgtg ggacatcggc 1255680
ttcatcaaca tcgaaaatcc cgccggcggc attaccgttt ccgcgatggg catcaacggc 1255740
gcacaattaa cccagtggtc gaaatggcgt gccgaccgta tgaacgacct cgcccaaacc 1255800
ggcgccgatt tggttatcct ttcctacggc accaacgaag ctttcaacaa caacatcgac 1255860
attgccgaca ccgaacaaaa atggctggat accgtccgcc aaatccgcga cagcctgcct 1255920
gccgccggca tcctcatcat cggcgcaccc gaatccctga aaaacacgct cggcgtatgc 1255980
ggcacacgcc ccgtccgcct gaccgaagtc caacagatgc agcggcgcgt cgcccgtcag 1256040

gggcagacga tgttctggtc ttggcaaaac gccatgggcg gcatatgcag catgaaaaac 1256100
tggctcaacc aaggatgggc cgccaaagac ggcgtacact tctccgccaa aggctaccgg 1256160
cgcgcggcgg aaatgctcgc cgacagcctc gaagaactcg tccgctccgc tgcaatcagg 1256220
caataatcgg acaggaggcg gacggtattt ccgcaacagg gggatgccgt ctgaaacgca 1256280
taccttcata ttgcttcaga cggcatagcc accccgcgca cggtttgccg gacgcaaccg 1256340
gcattcgcct caggcatcgg aaggacgcag gcgaacctcc ggcatacggc gcaaaggcgg 1256400
cgtttgatat gccgtctgaa ggcaaagatg ataaactgcc gccttccgtt ttcagacggc 1256460
atattgtttt caaatgaggg cgttctccgt ccgcaaccat aaaggaagtt tcatgaaccg 1256520
gacttatgcc aatttctacg aaatgctcgc cgccgcctgc cgcaaaaacg gaaacggcac 1256580
ggcagtgttc gacggcaagg aaaaaaccgc ctaccgcgcg ctcaagcagg aggccgaagc 1256640
cgtcgcggcg tatctgcaaa atatcggcgt gaagttcggc gacacggtcg cgctggcggt 1256700
ttccaattcc acagaattta ttaccgccta tttcgccatc tccgccatcg gcgcggtcgc 1256760
cgtaccgatg aacacatttt tgaaaaacag cgaatacgcg tatatcctga acgactgcaa 1256820
ggcgcgcttc ctgttcgcct cggccggcct gtcaaaagaa ttggcgggct tgaaggcgca 1256880
aacgcccgtc gaaaaaatca tttggacgga caaaagccgt ccgaccggcg aaacggcgga 1256940
aggcgatgcc ttttttgaag acgtgcgccg cttccccgaa aaacccgact tgggccgcca 1257000
accccggata aatgatttgg cacacatcat ctacacctcc ggcacgacgg ggcatcccaa 1257060
aggcgcgcta atcagttacg ccaacctgtt cgccaacctg aacggcatcg aacgcatctt 1257120
taaaatttcc aagcgcgacc gctttatcgt tttcctgccg atgttccaca gcttcacgct 1257180
gacggctatg gtgctgctgc cgatttatat ggcgtgttcg attattttgg tcaaatccgt 1257240
ttttccgttt tccaacgttt tgaaacagac actgctcaaa cgcgcgaccg tgtttttggg 1257300
cgtacccgcg atttacaccg cgatgagcaa ggcgaaaatc ccttggtatt tcagatggtt 1257360
caaccgcatt cgcctgttta tcagcggcgg cgcgcctttg gcggaacaaa ccatcctcga 1257420
tttcaaagcc aagttccccc gcgccaaatt gctggaaggc tacggactga gcgaagcctc 1257480
tcccgtcgtc gccgtcaata cgcccgagag gcaaaaagcc cgcagcgtcg gcatccccct 1257540
gcccggtttg gaagccaaag ccgtcgatga agaattggtc gaagtgccgc gcggcgaagt 1257600
gggcgaactg atcgtcaggg gcggttcggt gatgcggggc tacctcaata tgcctgccgc 1257660
caccgatgaa accatcgtca acggctggtt gaaaacgggc gatttcgtta ccatagacga 1257720
agacggcttt atctttatcg tcgaccgcaa aaaagatttg attatttcca aaggtcaaaa 1257780
tgtctatccg cgcgagattg aagaagaaat ctacaaactc gatgccgtcg aagccgccgc 1257840
cgtcatcggc gtgaaagacc gttatgccga cgaggaaatc gtcgccttcg tccaattgaa 1257900
ggaaggtatg gatttgggcg agaacgaaat ccgccgccac ctgcgtaccg tgctggcaaa 1257960
tttcaaaatc cccaaacaaa tccactttaa agacgggctg ccgcgcaacg ctacgggcaa 1258020
ggtattgaaa cgggtgttga aggagcagtt tgacggaaac aaatgaacgc cgtgccgtcc 1258080
gaagccccgt ccggcaaaaa aatgcggtga atctgattca ccgcattttt ttgacaaaca 1258140
ccggagggag ggcatttact ctgccaattc cacctgctcg tgcgccatca gttcctgacc 1258200
gacatcatcc aacaacatca aataacgttc gtagtttttc aaaatgtcgg caatcagctc 1258260
gtccttgttc atatactgca catcgtaccc gacgcgcccg tcgaaaaaat aagcgtaggg 1258320
tttgtaagtt gtctgatgcc ggatatgcgg cagcttgccg tcgttaatca actggtcgga 1258380
tacatcctgc ccgacagact taatcccgta cataaaatcg cgcatcgtct ctttccgaat 1258440
gacgaactcg attgcgggct cgtcccgatg aaacatttta tcgacccgga cgctcaagcc 1258500
gtattcttcc gaaagctccc gttgcaactc gtgcatagcg ggcgatgcag tctgtttgag 1258560
gaattttaaa atatcctgct cctgcgtctg gctcattatc tgcaccagcc gttctttcca 1258620
cttgccgccc gtccaaaata cactggtagg gttaacccgg gtctcaaaat atttcttatc 1258680
cgcactcaag cctttccaca ggctgaaaca cattatcagc atcagcaggg caaacggcag 1258740
ggaaacaatc agggtcatag actgcaggtt gccgagtccg cccgagcgca tcagcaaaac 1258800
ggcaacggca gacatcagca cgcccccacat aaccgcctgc caccgtggcg cgctcaagcc 1258860
tttgtcccga gaggtaatat tgttcaggac ataaatcccg gaatcggcag aagttacaaa 1258920
aaacagagaa atgaccagca ggctgacgat gctcgtcaat tcgggcaggg ggaggtaatt 1258980
aaagaattta aaaagcagcg tttccggaga ggaggtcatc ttttcgagca ttccccccgc 1259040
aaccccgtca ttcagccaaa tcgccgtatt gccgaagacg gtaaaccaca aaacgccgaa 1259100
caggccgggg atgagcaaaa ccccgaagac aaactcgcgg atggtgcgcc cctttgaaat 1259160
gcgcgcgata aacaaaccca caaacggcgc ccaagaacac caccacgccc aataaagcac 1259220
cgtccaagat tcaaaccacg gcttgtgttc ccgttcgtac gcataagttt taaaactgag 1259280
gcgcaccaga tttccgaggt agttccctat gttgtcgccg aatgccgaca acaggtaaac 1259340
agtgggtccc gccgccaaaa caaaaaacag cagcaaaaac gcaaggccca ggttcaactc 1259400
gctcaacacc ttcacgccct tccccacgcc ggatattgcc gaaacgacgg cgagggacat 1259460
gacggcggcg ataatcaaaa cctgcacgct gaagctgttt tcggcaatcc agcccatttc 1259520
ctgcaatccg gcgcccagtt gcgaagcccc gaaccccaat gtggtgatga tgccgaaaaa 1259580
agtagcaagc aacgccataa tatcaatggc atcgccgaac cttccggaaa ttttttcttt 1259640
caacaggggg taaaaacaag aacgcagggc aagcggcagc ttgtagcgga aaccgaaata 1259700

agccaaagcc aatgcaatcg taccgtacac cgaccaagcg tgaacgcccc aatggaacac 1259760
cgtgtgcagc aatgcctgct gctgcctgtg ttccggcgtg ccggccgtaa tgtccgaaaa 1259820
ataatgcatc aacggctctg ccacgccgaa aaacatcaga cccacgccca tcccggccgc 1259880
aaacagcatc gccagccacg acaggaagcc gaattccggc acatcttcat cccgtccgag 1259940
cctgatgttt cccaaactgc tgaccgagag tatcagcagg aaacccagaa aaatggaaaa 1260000
cgttaaaaca taaaaccagc tgaactcggt aaaaatgact tcttttgccc gatcgagcca 1260060
catctgcacc tgatccggca cggttaaaac caataccacc aaaacacaca caaaaaacaa 1260120
agtcgtcaaa ataaccatcg gattaaatga cgttcggcgt tctataaatt cagacaggga 1260180
caaaccttct cactcctttg ttaaaaacag acaaacccgg tcatcgggca aagcggtcaa 1260240
aacctgccgg taaaataccg gcttccggat gacgaaatgc acaaaccggc cgaaattgtt 1260300
ataatcgaaa aaattaaaaa tcaatacgga ttattctatc agattgattt atcgatattt 1260360
attatatcat taatatagtt tgatttcaaa ccggcggaaa atgcgccgcc caccgcaggc 1260420
gcgccctccc cgctatcggg gcgtttaacg ccggattgcc gatgcggtac aatggctgat 1260480
atgaagaaaa ttacccctca aaacctgcgc cccctgcttt cggaaagctt gggacatacc 1260540
gattttgtca acgtcctcaa cgcactgatt aaatttttgc gccgtggcgg caaaaaaatgt 1260600
gcgggggaac gtttcgacct gattatcgac acattcaaac aagacaggga attactgtcc 1260660
cgcttcagcc ggtgttttta catttggctc gcgcaaatac acatttatcc ggcactcatc 1260720
aaactcggca tcttctcgcg ccacagcttt gcccgggaaa tgggcatacg catctacgaa 1260780
cgcttcagcc cgtcatataa agattttgcc aacttgggcg aagtcttcct ttatcttttc 1260840
cattccgaaa acgacgacaa atggctgcaa acgctcaata tccgccaatg gctggtttta 1260900
tacgaactca tccggagcca cgccgagccg tccaaattgc agacggcggg catccgcctt 1260960
gccgatgcgc gtttgcgcgc catcgaaatg ctgtctgtct ggacggcatc cgaagccatc 1261020
gaacccgacc tcatccgcat cgccccgcgc ctgctggaag ccgattcttc cttcgtcgcc 1261080
ctccaacgcg aaaccgccaa actggtcgaa cactaccgca acggcaccac gccttacgac 1261140
accgcccacc tcgaagtgat gttcgaccaa tgtttcagcc agattgacta tttgcgccgc 1261200
aaagggacgg gcgccggctc cggttcgtcg gtcaaagtcg cccacctgct cgaacggctc 1261260
cggcagaccg tagaccgtct gaagctgctc accgacatcc aaaccggcgc cggcaacagc 1261320
aaccgcctga ccatcgcgct gatgaactcc ctcatctacg cggcggtcga acaatacagc 1261380
acccgccacc tgcgccgcag cagcatccgt atgctcgccc gcagcattac cgaaaacaaa 1261440
agccaccacg gcgaacacta catcacccgc aaccgcaaag aatatttcaa aatgttctac 1261500
tcggcggcag gcggcggcat catcatcgcc ctaatggcgc tgctcaaaat ccgcatcggc 1261560
tcactcggcc tcagcccctt cctcacttcc ttgtcggctg ggttcaacta cggcatcggc 1261620
tttatgatca tccatatgct gcactgcacc gtcgccacca agcagcccgc gatgactgcc 1261680
gccagctttg ccgaacaggt cgatctcaac gaaggcggca aagcggtgga caacaaactc 1261740
gccaagctcc tcatcgacgt atgccgctcc caaagtgtcg ccgtcttcgg caacgtttcc 1261800
atcgccatcc ttttggcgtg cgccatatcg ttcggctatg cccatctgta ccggctgccc 1261860
atactcgatg cccacaccgc cgcctaccag ttcaaatcca tagacatcat cgcttacccg 1261920
acgctgtggt atgccgccat tgcaggtctg tggctgttct gctccggcat catcgcaggt 1261980
tttttcgaca accgcgccga ctacctcaac ctgcgccaac gcctgccctt caaccccttg 1262040
ctgcgtaaaa tcatgcgccc cgggccccgc cgcgtcctcg ccgcctacat ccacaaacac 1262100
tacggctcgc tggtcggcaa cttcatcttc gggatgctct tgggtatgac cggctatttc 1262160
ggacacctcc tcgggctgcc gctggacatc cgccacgtcg ccttttcctc cgccaacctc 1262220
ggctatgccg ccgtcagcgg caacgtcggt ttgggcacgt tcgtactcgg cattttcagc 1262280
gtcctcgcca tcggcctggt caacctctgc gtcagcttca gcctcgccct cttcgtcgcc 1262340
ctgcgctcgc gcggcacgaa aatcggcagc atccgcaatc tgattaaaag tttttggaat 1262400
cagattaaaa gcaatccctg catacttttc ctcccgcccg ccaaagaaca gggacatcct 1262460
ccttcggaca agccttgacc ggcaatgccg tctgaagcgg gattcgcccc gaataccgcc 1262520
ctgatgcggg aaatccccat aaaaggatgc aaaaatgccg tccgaaccga aacgtggttc 1262580
agacggcatt ttaaaaaaca ttacaatccc gggccgccaa gcgaattggc aatgtccctg 1262640
accgccgtta catacatccg gctgtggttg tactgccata ccgtataaaa attgttcaag 1262700
cccaaataat attcaaacac gcccggtgcg gtttccagtt tgaacaaaac cgccttttca 1262760
tcatctgcaa gctcttcgcc ggggatgatg ccgtacgcct tcaaatccgc caccgtccgc 1262820
gtcagggcgg ttttttcgcc aatgattgcc tgaacatccg cacccggcgc caatgttgca 1262880
gacaccagca ttttcccgcc cgtgcgccaa ccgtgctgct tcatataatt ggcaaccgat 1262940
gccgcgacat cgccgacgtt gccccatatg tcccgatgtc cgtccccgtc ataatccacc 1263000
gcccatttcc ggtagctcga aggcataaat tgcggcatcc ccattgcgcc cgcatagctg 1263060
cctttaaagg cgaaaacatc gccgccttct tcttttgcca gctttaaaag ctcgaccaat 1263120
tctttttgga aaaacccggc gcggcggggg taatcaaagc ctaaggtcgc caatgcgtcc 1263180
gccacacgga aactgcccgt atttttgccg taattcgttt caatcccgat aaccgccacg 1263240
ataagttcgg caggcacgcc gtatttttgc gccacatcat cgataagcgc gcggttttcc 1263300
gcataaaacc ggcgcgcgcc gcgaaatttc gccttgcccg aatttcccgt gcggaacaca 1263360

```
taccacggac gcgatgtgga ggggcggtgc ataatcttga cgatgtccgc cttgtaagcc 1263420
gctttgtcaa aaaaatcctg ccattccgcc cgggaaaaat cccctttccc gacttcatcg 1263480
tccacaaaac ggcggacatt tgcattggcg gcaaacccgc tgtcggatac cggtacggct 1263540
gccgcgtcaa atacggctgc cgcgtcaaac gcggggcggc tttctttttt catttcaacc 1263600
gcgcgggggg cttgggcttc atttgcccgg ggtgggcgtg cctccatcgc cgtacaggca 1263660

gacaaagccg ccaaacaaat tgccagcggc agtatttttc tctttttcat aaatatgttc 1263720
cgaacaaata gggtaagtgg gaaagcggca caagggggcg gcgcgaaatg cggcatccgc 1263780
cgcaatcggc ggctttgcgg aatgccgcac gttgcctctt gcaccgcccg aaatccgtat 1263840
gtcgtcgccg aaaatgccgt ctgaaggcac ttcccctttc agacggcatt gcctgccgcc 1263900
gtatttgccc gctacccgca atatcggcag tccaatatat ctttgcggat gtcgttcagc 1263960
aggaaggctg cggtgtcttc gtgtttcgcc tgtacgaaga caaagagtcg ggcgatgatt 1264020
ttgccgataa agactttttg cagggcgcag gcatcggtca gggtgtgttc ctgcaggaag 1264080
ctgcggatgt cttcaggcaa agtgtagtcg cgggcgacgg cggcaaagcg ggcatcggtt 1264140
tgcaggcggt cgagcagggt ttggtttttg tccaacttca tgcctgcttc tttttcttcg 1264200
gcggtggcgc ggacaaactg gtcgtaaatt tcggcataaa gcatatcgtt cgggccttca 1264260
aaaatcgtga aggggcggat gtcgatagcg atattgccgg cggtgtgtcc gcgttcaaaa 1264320
cccttcgcac ccaagagttt ttgcaacatt tgcgcggcgg cgtaagtgta ttccgtggcg 1264380
agggttttga cgatgttcgc ctccatcagc tgatgggcga cgggggcaac aggcgaaacg 1264440
gaatggcaga cgtagcggta aagaatctcg gaaacctgat ggcggcgccg gatttcgcgg 1264500
cgttcgtaat cgacgaattt gatgtcgttg cggacgtatc gttccagatt ttcaaggatg 1264560
tattccataa tgccgtgcgt catgccgatc agttgcaggc ggctgcggat aaagatgttt 1264620
tggaacgcgc gcaaaccggc agcgtcgctc tgggagagtt tcatcacggc ggttgcaggc 1264680
atttcggcat cgatgcggtt gacggcgtaa cggacggcgc gcaagccttc ggatgcgagg 1264740
gtttcgcagc ggatgtatgt tttggggacg agcagcaggt cgatgacttt ggcgagtttg 1264800
ccgtttttgc gctctttggc ggcaacgagg aggaagtcgc tttgcgagtt gccctgccag 1264860
tatttcgcgg cgttgacgta aatggtttgt ccgtcgatat attcgtagta ggactgcatt 1264920
tcgcgtgcaa tcgccgcgcc ggaggtttcg ggttcggtaa cacccaaacc gccgccctcg 1264980
cctttgaaaa tcatctccaa accttgcgcg acttgcgctt catcgccgaa ctcttgcagt 1265040
ggctgcaaca ccagcgcgcc ttcgatgccg gtacgcagcg taacgggcac gccgtaatgc 1265100
cccgcaatcc gcaggacttc ttggatttca aactggctgc ccttgcgccc gccgtatttt 1265160
ttgtcgagga agggcaacag caaacccgcc tgcttcaagg caagccattt gtcttcgggc 1265220
aggtatcgca tcaggtcgat accgtctgaa aaaatgcggc ggaatgcgga ttcgatgtgc 1265280
tttaaaaaag cagccgtgtc catagttgac ggctgcgcgc tcggttcggt gtgtatcatc 1265340
ggcttcctct gtcggttccc attaatcggc ggccggtcaa accgcctgcc acagtttaga 1265400
gttgattttc taaactttac cacaaagtgc gccgggcaac aatccgccga cctttcagac 1265460
ggcatcgcgt cccctcccgt gctaaaatga ccgtttgcat cactgtccgc cgattgccgc 1265520
actatgacct accccatccc caaacccccgt gaaaaatccc gttggcccaa tctttcgcaa 1265580
ggctcgctgc ccttggcttt ggcgcgttat ctgccgcaca agcggctcaa ggtcgtgctg 1265640
acccaagatg cggaacaggc gttgcgcctt cagacggcat ggcggttttt ccgtccgcac 1265700
gacacggcgg tgttcctgcc ggactgggaa acgctgcctt acgagcgttt ttcgccgcat 1265760
caggatttgg tgtcggagcg gctgtcggcg ttgtggcaga ttaaaagcgg cgcggcggat 1265820
gtgttgttcg tgccggttgc cacggcgatg cagaagctgc cgcccgtgcc gtttctggca 1265880
gggcgcacgt tttggctgaa aacggggcag actttggata taggccgtct gaaaagtgat 1265940
ttggtggatg cggctacaa ccatgtttcc cacgttgtcg cggcgggcga atttgccgtg 1266000
cgcggcggta tagtcgattt gttcccgatg ggcagcgaaa tgccgtaccg catcgatttg 1266060
tttgacgatg aaatcgacag catcaaaacc ttcgataccg aaacgcaacg caccatttcc 1266120
cccgtttccg aaatccgcct gctgccggcg cacgagttcc ccaccgacag cgaggcgcaa 1266180
aaaatcttcc gcagccgctt ccgcgaggaa gtcgatggta atccgaacga tgcggctgtg 1266240
tacaaagccg tcagcaacgg tcatttcggc gcgggcgtgg aatactacct gccgctgttt 1266300
tttgaaaacg agttggaaac gctgtttgac tatatcggcg aagatgcgct gtttgtctct 1266360
ttagacgatg ttcatgccga ggcaaaccgt ttttggagcg atgtcaaatc gcgttacgcg 1266420
atggcgcagg gcgacgaaac ctatccgcct ttgcttccac agtatttgta tctctctgcc 1266480
gatgtgttcg caggccgtct gaaaaactac ggacaggtgc tgcccgatgt ttccggcaag 1266540
gaatacaccc tgcccgacct tgccgtcaac cgccaagcag atgagccgtt gcaggcattg 1266600
aaggattttc agacggcgtt tgacggacgg attttgctgt gcgccgaaag tttgggacgg 1266660
cgcgaaacta tgctcggttt cttgcagcaa aacggtttga aagccaaacc cgtgtccgac 1266720
tggcagggct ttttatcggc acacgagccg ctgatgatta cagtggcgcc gttggcatac 1266780
gggttcaaac tgggcggact gcaatcgccg aaccaacagc aacctactcc ctcccccgtg 1266840
ggggagggtt ggggagaggg caaagcagtt gccgctcaaa ctgaattttc cgcagccgca 1266900
ataaaccctc tccctagccc tctcccacag gagagggaac aaagtgcagc cgccgtttca 1266960
```

gacagtctga aagcagccgc cgtttcaacc gaaagcagcc tgcccctcgg tacaagtaat 1267020
ctgcacgggc aaatccgaca gcaacctgcc ccttcccccg tgggggaggg ttggggagag 1267080
ggcaaagcag ttgccgctca aaccgaattt cccgcatccg caacaaaccc tctccctagc 1267140
cctctcccac aggagaggga acaaagtgca gccgccgttt cagacgacct gaaaaccaaa 1267200
agcagcctgc atcccgtcgc aaataatctg cacgggcaaa tccgacagca acctactccc 1267260
tcccccgtgg gggagggttg gggagagggc aaagcagttg ccgctcaaac cgaattttcc 1267320
gcagccgcaa caaaccctct ccctagccct ctcccacagg agagggaaca aagtgcagcc 1267380
gtcgtttcag acagtctgaa agcagccgcc gtttcaaccg aaagcagcct gccccccggt 1267440
aaaagtaatc tgcacgggca aatccaacag caacctgccc cctcccccgt gggggagggt 1267500
tggggagagg gcaaagcagt tgccgctcaa agtgccatcg ccgtcatcac cgaatccgat 1267560
ctctaccaat acgtcgcccg ttcgcgcatc cacaaccgcc gcaagaaaca cgccgccgtt 1267620
tcagacgggc tgttgcgcga ccttgccgaa atcaatatcg gcgaccccgt cgtgcacgaa 1267680
gaacacggca tcgggcggta tatgggcttg gtaacgatgg acttgggcgg cgaaaccaac 1267740
gaaatgatgt tgctcgaata cgcaggcgaa gcgcagcttt atgtgcctgt ttcgcaactg 1267800
catttaatca gccgctactc cggtcaggcg catgaaaaca ttgccctgca caagctcggc 1267860
agtggcgcgt ggaacaaggc gaagcgcaaa gccgccgaaa aagcgcgcga caccgccgcc 1267920
gaattgctca acctctacgc ccaacgcgcc gcccaatcgg gacacaagtt tgaaatcaac 1267980
gagttggact atcaggcgtt tgccgacggc ttcggctacg aggaaaccga agaccaggcc 1268040
gccgccatcg ccgccgtgat taaagatttg acgcaagcga agccgatgga tcgccttgtg 1268100
tgcggcgatg tcggcttcgg caaaaccgaa gtcgccctgc gcgccgcgtt tgtggcggtg 1268160
atgggcggca aacaggtcgc cgtacttgct ccgaccacgc ttttggtcga gcagcacgcg 1268220
caaaacttcg ccgaccgttt cgccgatttc cccgtgaaag tcgccagcct ttcgcgtttc 1268280
aacaacagca aagccaccaa agccgcgctg gaaggcatgg cagacggcac ggtcgatatt 1268340
gttatcggta cgcacaaact ggtgcaggac gacatcaaat tcaaaaactt aggtttagtg 1268400
attatcgacg aagaacaccg cttcggcgtg cgtcagaaag agcagctcaa acgcctgcgc 1268460
gccaatgttg atatccttac catgaccgcc acgccgattc cgcgtacttt aagtatggcg 1268520
ttggaaggac tgcgcgactt ctcgctgatt accaccgcgc ccagccgccg cctcgccgtc 1268580
aaaacctttg tcaaaccctt tagcgaaggc agcgtgcgcg aagccgtgtt gcgcgaactc 1268640
aaacgcggag gacaggtatt tttcctgcac aatgaagtag atacgattga aaatatgcgc 1268700
gagcggctgg aaaccctgct gcccgaagcc cgcatcggcg tggcgcacgg acaactgcgc 1268760
gagcgcgagc tggaacaagt catgcgcgac tttttgcagc aacgatttaa cgtgttgctc 1268820
tgttccacca tcatcgaaac cggtatcgat atccccaacg ccaacaccat catcatcaac 1268880
cgcgccgaca aattcggact ggcgcaactg caccagcttc gcgggcgcgt cggccgcagc 1268940
catcaccaag cctacgccta cctgctcacg cccgaataca tcactaaaga cgcagaaaaa 1269000
cgcctcgatg ccattgcggc ggcagacgaa ctcggcgcag gttttaccct agccatgcag 1269060
gatttggaaa tccgtggtgc aggcgaaatc cttggcgaag gacaatccgg cgaaatgata 1269120
caggtcggct tcacgctcta caccgaaatg ctcaaacaag ccgttcgcga cctcaaaaaa 1269180
ggccgccagc ccgacctcga cgcaccgttg ggcatcacca ccgaaatcaa actgcacagc 1269240
cccgccctgc tgcccgaaga ttactgcccc gacatccacg aacggctcgt cctctacaaa 1269300
cgcctcgccg tctgcgaaac cgtgcaacaa atcaacacca tacacgaaga actcgtcgac 1269360
cgcttcggcc tgcccgaaca acccgtcaaa acccttatcg aaagccacca cttacggctt 1269420
atggcaaaag aattgggtat cgatgccatt gatgcggccg gcgaagcggt aacggtaacc 1269480
tttggtaaaa acaataatgt cgatccaacc gaaatcatcc tgctgattca gaacgacaaa 1269540
aaataccgcc ttgccggcgc cgataagctg cggtttaccg cagagatgga aaatatcgag 1269600
gtcagaatca acaccgtaaa aaacgtttta aaaaccttgc aaaacagatg cctgcccaaa 1269660
taaagccgac accgcaatgc cgtctgaaac accgttttcc ttgtccgaaa gccgccatta 1269720
tgaatttgaa ggaaactcca ctataatacg gcattcagat ttccagacgg catcgcgccc 1269780
gtcaaaccgc acacaaacca aaaggaaata catgttccgt accatgcttg gcggaaaaat 1269840
ccaccgcgcc accgttaccg aagccgattt gaactatgtc ggcagtatta ccgtcgatca 1269900
agacctgtta gacgcggcag gcatctaccc caacgaaaaa gtcgccattg tcaacaacaa 1269960
caacggcgaa cgttttgaaa cctataccat tgcagggaaa cgcggcagcg gcgtgatttg 1270020
tctgaacggt gctgcagcca ggctggtaca gaaaggcgat atcgtcatca tcatgtctta 1270080
cgtccaactc tccgaacccg aaatcgccgc acacgaaccc aaagtcgtct tggtagacgg 1270140
aaacaacaaa atccgcgaca tcatctccta cgagccgccg cacaccgtgc tgtaattccg 1270200
caaacggaca tcgattatgg atattaaaat caacgacatc accctcggca acaactcgcc 1270260
cttcgtccta ttcggcggca tcaacgtttt ggaaagcttg gattccaccc tccaaacctg 1270320
cgcgcattac gtcgaagtta cccgaaaact cggtattccc tatatcttta aagcctcttt 1270380
cgacaaggca aaccgttcct ccatccattc ttatcgcggc gtaggcttgg aagaaggctt 1270440
aaagattttt gaaaaagtca aagcagagtt cggcatcccc gtcattaccg acgtacacga 1270500
accccatcag tgccaacccg tcgccgaagt gtgcgatgtc atccagcttc ccgcctttct 1270560
tgcgcggcag accgatttag tggttgccat ggcaaaaact ggcaacgtcg tcaacatcaa 1270620

aaaacctcag ttcctcagcc cctctcaaat gaaaaacatt gtggaaaaat tccacgaagc 1270680
cggcaacggg aaactgattt tatgcgaacg cggcagcagc ttcggctacg acaacctcgt 1270740
tgtcgatatg ctcggtttcg gcgtgatgaa acagacttgc ggcaacctgc cggttatttt 1270800
cgacgttacc cattccctgc aaacccgcga tgccggttct gccgcatccg gcggtcgtcg 1270860
cgcacaggct ttggatttgg cacttgcagg catggcaacc cgccttgccg gtctgttcct 1270920
cgaatcgcac cccgatccga aactggcaaa atgcgacggc cccagcgcgc tgccgctgca 1270980
ccttttagaa gattttttaa tccgcatcaa agcattggac gatttaatca aatcacaacc 1271040
gattttaaca atcgagtaac acggtttcgc cttatgatgc agactttccg aaaaatcagc 1271100
cggtatgtcg caaccttgtg gctcggtatg cagattatgg cgggttatat cgccgcaccg 1271160
gtgctgttca aaatgctgcc caaaatgcag gcgggcgaaa ttgccggcgt attgttcgac 1271220
atcctctctt ggagcgggct tgccgtttgg ggcgcggtac tggctgccgc ctttgccgcc 1271280
ctaacccggc ggcaaaccgc cctgctgctt tttttattgt ccgcccttgc cgccaaccga 1271340
ttcttgatta cacccgttat cgaggcactg aaatacggac atgaaaattg gctgttgtcg 1271400
tttgtaggcg gatccttcgg aatgtggcac ggcatttcca gtattgtttt tatggcaacc 1271460
gccctacttt cagcagtttt aagttggcgg ctttccggca aagatgccgt ctgaagccct 1271520
cccatttttt tacctccctt cacttcactt ggagaacatt catgagcgca atcgttgata 1271580
ttttcgcccg cgaaattttg gactcacgcg gcaaccccac agtcgagtgt gatgtattgc 1271640
tcgaatccgg cgtaatggga cgcgcagccg taccgagcgg cgcgtccacc ggtcaaaaag 1271700
aggctttgga acttcgcgac ggcgacaaat cccgttattc gggcaagggc gtattgaagg 1271760
cggtcgaaca cgtcaacaac caaatcgccc aagccctcat tggtatcgat gccaacgagc 1271820
aatcttatat cgaccaaatc atgatcgaat tggacggtac tgaaaacaaa ggcaatttgg 1271880
gtgcgaatgc gactttggcg gtttctatgg cggttgcacg cgccgctgcc gaagactcag 1271940
gcctgccgct ttaccgctac ttgggcggcg caggcccgat gtccctgccc gtaccgatga 1272000
tgaacgtcat caacggcggc gaacacgcca acaacagcct gaacatccaa gagtttatga 1272060
ttatgcccgt cggcgcaaaa tctttccgcg aagcgttgcg ctgcggtgcg gaaattttcc 1272120
acgccttgaa aaaactgtgc gacagcaaag gcttcccgac cacagtcggc gacgaaggcg 1272180
gtttcgcccc caacctgaac agccacaaag aagccctgca actgatggtc gaggcgaccg 1272240
aagccgccgg ctacaaagcg ggcgaagacg tattattcgc attggactgc gcctccagcg 1272300
agttctacaa agacggcaaa taccacttgg aagccgaagg ccgctcctac accaacgcgg 1272360
aatttgccga atatctggaa ggcctggtca acgagttccc catcatctcc atcgaagacg 1272420
gcatggatga aaacgactgg gaaggctgga aactgctgac cgaaaaactg ggcggtagag 1272480
ttcaattggt tggcgacgac ttgttcgtaa ccaatccaaa aatcttggcc gaaggcatcg 1272540
aaaaaggcgt agcaaacgca ttgctggtca aagtcaatca aatcggtact ttgagcgaga 1272600
ccctgaaagc cgtcgactta gccaaacgca accgctacgc cagcgtaatg agccaccgct 1272660
ccggcgaaac cgaagacagc accattgccg acttggcagt cgccaccaac tgtatgcaga 1272720
tcaaaaccgg ttctttgagc cgttccgacc gcatggcgaa atacaaccaa ctgctgcgta 1272780
tcgaggaaga attggcggaa gccgccgact accccagcaa agccgcattc taccaactgg 1272840
gcaaataaaa aaggttaagg tatgaagtgg gtaactgtcg ttttatcctt cgcacttgtc 1272900
tgttgccaat acagcctctg gttcggcaaa ggcagcatcg gacgcaacag cagtctgaga 1272960
gaacagattg ccgttcaaga agaaaaaaac cagacactcg ccctacgcaa tcattccctt 1273020
gccgccgaag tctatgattt ggaaaacggt caagaagcca tttcggaaat cgcccgggta 1273080
gaactgggtt atatccaaga cggtgaaacc ttttaccgac tcatcaggca taaccggtaa 1273140
taccgtcaaa aagccgtccg aaccaatgtt cggacggctt ttatttcaac aaactgtcag 1273200
acagcccctc atcctccccc gacaaaccgc aatccagcct gacatccccc tcgacgcaac 1273260
agcagcacgg cagtatctcg tcccgcccca aaaaagccaa aggcggctcc cgataagtaa 1273320
cgcttccctc caaaatcttc actcggcacg atccgcaata tccgcttcgg cactgatatt 1273380
ccaccatatg ccccgtccgt tccaagcctt ccaacagagt ctcgccctcc aagagttcaa 1273440
aaaaaccctt attcgtacca atgcgcgcca tttccgacca atcaaaaata tagtggatta 1273500
aatttaaacc agtacggcgt tgcctcgcct tgccgtacta tttgtactgt ctgcggcttc 1273560
gtcgccttgt ccggattttt gttaatccac tataatccac tataatccac tataaaagga 1273620
acaataaccg atcctacccg ctgtttttcc catcatacaa catacaaatg ccgtctgaaa 1273680
catccggctt cagacggcat tttttcaaaa aacatttaca actcaaaatc gcccaaatca 1273740
tccgtattca cttcagaatc tatctgaccg atcaaataag aggatatttc cacttcctgc 1273800
ggcgcgacct gtacgttgtc ggacgacagc cacgcattaa tccacggaat cgggttttga 1273860
tttgcgcctt caaatccggc cggcaacccc accgcctgca tacgcagatt ggtaatatat 1273920
tcgacgtatt gggataagat ttctttgttc aaaccaatca tcgaaccgtc tttaaacaaa 1273980
tatgccgccc attctttttc ctgttccgcc gctttttttga agagttggaa acattcgtcc 1274040
tgcaactcgg cggcaatttc tgccatttca gaatcatcaa caccagaacg catcagatta 1274100
agcatatgct gcgtgccggt caggtgcagg gcttcgtcgc gggcaatcag tttgatgatt 1274160
ttggcgttgc cttccatcaa ctcgcgctcg gcaaaagcaa acgagcaggc gaatgaaacg 1274220
tagaaacgga tggcttccaa cacgttgacg cacatcaggc agagatagag ttttttcttc 1274280

aacccgcgca aagacacggt aacgggtttg ccgccgacat tgtgcacccc ttcgcccaac 1274340
aggttgtaat actgggtgta ttcgattaag tcatcgtaat agcaggcaat gtcttcggcg 1274400
cgggcggtaa tgtattcgtt ttcgacaata tcatcaaaca cgaccgacgg atcattcaca 1274460
atattgcgga tgatgtgggt atagctgcgc gagtgtatgg tttcgctgaa gctccacgtt 1274520
tcaatccacg tttccaactc gggaatcgaa accaaaggca gcaaggcaac attcggactg 1274580
cgcccttgga tggaatcgag cagtgtttgg tatttcagat tgctgatgaa aatatgtttt 1274640
tcgtgttcgg gcagattggc gtagtcgata cggtcgcgcg agacatcgat ttcttcaggc 1274700
cgccagaaga aagacaattg tttttcaatc agtttttcaa atacttcgta tttctgctgg 1274760
tcataacggg caacattaac cggctgacca aaaaacatcg gctcattcag cgcgtcgttt 1274820
ttggttttgg gaaaggtgct gtatgacata gttaagtgtt cgcaggacat aatctattct 1274880
ctatattaat taaaaatgat ctgtaagttt taactaacct gctgagactt tatatattta 1274940
ataaaatttt ctaaagttaa aacatgggtt ttcccaaaag ttccaaatgt ttttacggat 1275000
tctttaggga caactaaggt taaattctca tcactcattt cctgaatttg agtatcagat 1275060
ataccttctt gtagagtaaa taaatgctta tgaggaattc gatctgcttc atttaaaact 1275120
tggcgccagc gatccttaca agtagtcttt gaacctagca ttgtcagctt ttcagtttga 1275180
aatatgaaat tccctgattc atccatcgca tggtactctt cacttccagg aaataaaaaa 1275240
tctggtttct ttttcccttc tgtcttcgct tgcgtctcaa attttaagga aaattcgtca 1275300
aatattcttg ataaatgtaa ctctaaagat tttcctgctc ttgcttttct gcgatttgta 1275360
aaagaatgtg caaaattaat gaagctatcc aaatcagtaa tttttgattt aataatttca 1275420
aattcacgtt tttcaaaaat ggaaaataat tcatattcct ttgaaaccca ttgttttaac 1275480
acattactaa cgttagtttt atttaatgca atattttccc ttgccaaaac tgccatttct 1275540
tcagttttgg ggaagttagg aaatagtgaa aataattttg ttaggttatc ctctacttct 1275600
tgttttttag gtaaatataa acttcctggt aaaatgttag tttctgcgat aaatatttca 1275660
atatcttcat ctgaagataa aacaaaagca tggaataata aatctttata acaagctctg 1275720
cacaatacca ataaggaacc actatattta tcacttaaaa attcaaaatt cttgccaaaa 1275780
cccgtaattc tcgcctcatt tctcgtgcct tggccgtaat atttaaaatt gctattagtg 1275840
acgcttccat cttgccaatt aattttgatt gaaatagttt tatttgttcc cttttggcaa 1275900
actacatcaa agaggtcgtc tgaaaaatgt ttttgaatat aaaatcctga ttggtgacta 1275960
ccagtagtac caacatcatt gggacgaata taacgacagt atactgcaat tgatttattt 1276020
gctgtctgta ttgctgaagc tactaagtca tcggatttat ttactacttg tattgtctga 1276080
gctgccaagt cattcatttt ttaactcatt tataatttgt ttagcaatag cttgaaccaa 1276140
cggtacagca attgaattgc caaactgctt gtatgcagct gtcttggata ctgcatcaat 1276200
aacaaaatct ttaggaaatc ccattaaacg cgagcactcc ctaggtgtca gcttcctagg 1276260
atttttcct ttctgaggga tgagtatttc ggaaccatct ttgtaatatc gtgcagatag 1276320
agttcgtgat attccatcta aatcaactaa tccaaaacca aatccattac cctttgcctt 1276380
atgtttttta gcgtaatttt gaaggtaaag ccataagtta tcagaaagag taaaagaatt 1276440
atctacatca tcttccaaaa tttgctttaa ttttggttgt gattctggtg gggaaggaaa 1276500
attgaaattt atttccttat taaaatattg tctatcaaaa cctacaataa aaatacgctc 1276560
cctattttga ggaacataat attttgcatt cataacttga taaaatatct gatagtcaag 1276620
ctcttctaaa gtccctttaa ttactttaaa tgtatttcct ttgtcatgcg aaacaaggtt 1276680
tttcacattc tctaaaagaa aaattttagg tcgatgtttt ccaataattt cagcaacatc 1276740
aaaaaataga gttccctgcg ccttatctaa gaagcctgtt tctcgtccta ggcttttttt 1276800
ctttgaaaca ccagctatag agaatggctg acacgggaat cctgctgtta atacatcaaa 1276860
cttacttgga atagctgctt tggtttcctt taatgtaata tctccataag gaatatcatt 1276920
aaaatttact tggtaggttt gacgggcttt atcatcccat tcactagaaa atacacatcg 1276980
cccaccaaca ttctccattg caatgcgaaa accacctatt cccgcaaata aatcaataaa 1277040
agtaaatttc tcattattta aggtaactat attatctaat tgattttgta ttttattttt 1277100
catattttat atttcagatg atttattaat ctaaaggcca tctgaaaact ccccctttca 1277160
tcaaatctta caagccccgc ccgcgcagcc gtcatcctga atatcggtct gcgtatcgtc 1277220
cgcaccgtcg cgggtgttat ggtagtacag ggttttgacg ccgtatttgt aggcggtcag 1277280
caggtctttg agcatttgtt tcatagaaac tttgccgcct tcgaatttgc ccgggtcgta 1277340
ggcggtattg gcggaaatcg attgatcgac gaatttttgc atcacgccga caagtttcag 1277400
gtagccttcg ttgccgggaa gctgccacag ggtttcatag gcatttttca gggtttcaaa 1277460
ctccggcacg acttgtttca aaatgccgtc tttcgatgct ttgaccgtta ccaatccgcg 1277520
cggcggctcg atgccgttgg tggcgttggc gatttgagag ctggtttcag acggcatgag 1277580
cgcggtcaga gtagagttgc gcaggccgta tttgacgatt tcggcacgca ggctttccca 1277640
gtcgtaatgc aaaggctcgc cgcagacggc atccaaatct tttttgtaag tgtcgatggg 1277700
cagtttgcct tgcgaataaa cggtttggtt aaagagcgtg cacgcaccgt attctttggc 1277760
aaggtttgcc gatgctttga gcaggtaata ctgtatggct tcaaaggtac ggtgggtcag 1277820
accgagcgcg gaaccgtcgc tgtagcggac accgtttttc gccagataat aagcatagtt 1277880
aatcacgccg atgccgagcg aacggcggcc catagtagag gtacgcgcgg cttctaccgg 1277940

atatccctga taatctaaaa gtgcatcgag cgcacgaacg gtcaagtcgg caagcccttc 1278000
caattcgtcc aagctgttta atgcgcccaa gttaaaggca gacagtgtac acagggcgat 1278060
ttcgccgttc ggatcgttga tattgtccag cggtttggtc ggcagggcga tttccataca 1278120
caagttggac tgatgaacag gcgcgacgcg cggatcgaac gggctgtgcg tattgcagtg 1278180
atcgacgttt tgaatgtaga tgcgcccggt tccggcacgc tcctgcatca gcgtggaaaa 1278240
caggtcggca gccggaatga tgcgcttgcg gatatcaggg tcttgctcgt atttcgtata 1278300
gagccgctca aattcgtctt ggtcggcaaa aaacgcttcg tacaatcccg gaacctcgtt 1278360
gggcgaaaac agcgtaatgt tgccgccctt aatcaggcgg gtgtacagca ggcggttgat 1278420
ttgcacgccg taatcaagct gacggatacg gttgtcttcc acaccgcggt tgtttttcaa 1278480
caccagcagg ctttcggctt cgatatgcca caaggggtag aacaaggttg ccgcgccgcc 1278540
gcgcacgccg ccttgcgaac aggatttgac cgccgcctga aacattttaa agaagggaat 1278600
gcagccggta tgccgcgctt cgccgccccg gatttcgctg tccaaaccgc ggatacgtcc 1278660
ggcattgatg ccgatgcccg cacgctggga aacgtatttc acaatcgcgc tggtagtggc 1278720
attgatggaa tccaaactat cgtcgcattc aatcagcaca cagcttgaga actggcgcgt 1278780
aggcgtacgc acgccgctca taatcggagt cggcagcgat actttaaatg tagaaacggc 1278840
atcgtaaaac cgtttgacgt aacccaagcg cgcctctttc gggtatttgc tgaaaaggca 1278900
catcgccacc aaaacatata aaaactgcgg cgtttcgtaa atttggcggg taacgcggtt 1278960
ctgtaccaga tatttgcctt cgagctgttt gacagcggca taggaaaagg acatatcgcg 1279020
ttcgtggtcg atataggcgt tcagttcgtc aaattcttcg cggctgtaat cctcaaggat 1279080
atgcctgtcg tattttccgg catcggtaag ttttttaacg tggtcgtaaa ggtgcggcgg 1279140
ctcgtactcg ccgtaggcta ttttacgaag atggaaaatc gccaaacgcg cggcaaggta 1279200
ttggtagtcc ggggtatctt ccgaaattaa atcggcagcg gctttgatga tggtttcgtg 1279260
gatgtcgtcg gtgcggatgc cgttgtagaa ctggatgtgc gatttcaact cgacctgcga 1279320
cacggaaaca ttttccaatc cgtccgccgc ccaagtgacg acacggtgaa tcttatccaa 1279380
atcaatggct tctaatcttc cgtctcgttt ggttactttc aaatcagtcg gtgtattcat 1279440
cgcttcctct tccactcttg atattcaaga cacagtcttt tcaaataaat taaggcagac 1279500
aatatagtgg atttttggca ttttctccag tcttgacaac ggttgtattt ttcagtttca 1279560
ggcaatgcgc gataaaaccc gcctgtcgta tttttcctat aatatttgtt ttatctgata 1279620
attctttacc gataaaaaac gggtaaattt tttgcctttt gaccggctcc ggctacaagg 1279680
cggtgaaata aggattttcc gacgaaaaag gaaagcttcc tgttttctgc ccctgcaaat 1279740
tgttaaattt tgcaaagtat gattttgccg cgccgccgcc gacaaattcc attttcttac 1279800
cgattggaat ttattattga gattaatgtg ttatttgaat ctgcatatca aacggcaagt 1279860
tttgtcggct gaatgagtct gaaactgccg acaatttgcc cgttccatcc ttccatccta 1279920
tactggaaag aatgacaaac tgaaaggatc gtcatgtctg tcaaaaagat gcttgccata 1279980
ctgttgtctg caatattggg actggtatcg acaactgccg ctgccggtac gtcagaaccc 1280040
gcccaccgcg ataccaaaca tatccgcaag gcaaacaagc agatgctgca ccccgaatgc 1280100
aggaaatatt tggaacgccg tgccgcgtgg taccgatcgc aaggcaacgt gcaggaattg 1280160
cgcgaaaaca aaaaggcgcg caaagcattc cgctccctgc cttatgcgga acagaaaatc 1280220
caatgccggg cggcttatga ggctttcgat gatttcgacg gcggcagttt ccgccgttaa 1280280
tcccatataa aatatgccgt ctgaacacag gttcagacgg cattttccat agatacaaca 1280340
aactatcctt acgccatttc gtttgaggtc ctcccgttgc cgcagcctta atatagtgga 1280400
ttaacaaaaa tcaggacaag gcgacgaagc cgcagacagt acagatagta cgaaaccgat 1280460
tcacttggtg cttcagcacc ttagagaatc gttctctttg agctaaggcg aggcaacgct 1280520
gtactggttt ttgttaatcc actatacatc cacaaaagcg aatcatactg ccacattccg 1280580
acaaccggtt tcagacagcg atatccgcat cgtcggcgac aacggcacgg atgctttctt 1280640
cgattttatc ttttaaagca taacggtctt cgctttccgc cgcatccgcc acgcaaacga 1280700
aatcgactct tatcgtcaat tttttcatag acacgatgcg ccacaggcag gtcggcaaac 1280760
cgacatcggc atatgaggga cgagccgtcc tttttcccgt ttcgtcataa taacgcagcg 1280820
cgaccgccaa aacctttgcc cccgcatcga tggcggattg gaacagcgcg gctttgaacg 1280880
gcaaaagccc caatccggag gaagtccgcg cttcggggaa aaaactgacg ttttgaccgc 1280940
gttgcaaggt ttcgcagacg gcgcggttaa tcggttcgat gtcgcgccgc gaattgcggt 1281000
tgatgaacac cgttcccgcg ttctgcccca tcttgcccaa taccggccag cttttgattt 1281060
cctgcttggc gataaagctg ctcggataaa ccgcgctcat cgcgaaaata tccagccagg 1281120
acacgtggtt ggcggcaacc aagacaccgt tcggatgttc gggtgcgggt ctgcccacct 1281180
ccaatccgat atccaaagcc gccaaaaccc ccctgcccaa ctcgattacc gcccgattgc 1281240
gcgactcggg gcaaccgccg tcaataccgc gcaggttttt cccggttttg aacagccaga 1281300
ccgccaaacg gcacaagcgg cgcagacgtg taaaaaatga agctttattt gaagacatga 1281360
gcaattcttt cccgataatc ggtgtttgtt gttgaaacgt tcggacggca taccgataaa 1281420
atgccgtctg aaaccgtttt cagtcctatt tctgacagtt cgggcaataa aacgtgccgc 1281480
gctgccccaa agtttctttc acaaccaaac cgccgcaccg gggcacggc tgattgtgcc 1281540
gcccgtacac tgtatattcc tgttggaagt agccgctttt gccgtcgctg tccacaaaat 1281600

ccctcaaggt actgccgccc gtttcaatgg cgcgctgcaa caccgctttg acggtttcaa 1281660
ccaaaagcgc gcactctttc tttttcaggc ggttggcagg acggtggggc gaaatgcccg 1281720
ctctgaacag gctctcgttg gcataaatgt tgcccacacc gaccacgacc gcattgtcca 1281780
tcagggcaag tttgaccgcg cgcttctgcg ccttcagcct tgcatacaga taatccgcac 1281840
aaaatgcctc cgacaaaggc tccggcccca gtttttccaa cagcggatga tgttcttcga 1281900
ttccctcata ccaaagtatc gcgccgaact ttctcggatc gcggtaacgc atgaccgtgc 1281960
cgtctgaaaa cacaatatcg acgtgatcgt gtctgtccgg cctgccgata cgtccgtccg 1282020
acggcgtaaa aatccgcaag ctgcccgaca tccccaagtg aatcagcagc acgcccgttt 1282080
gaaagcggat aagcaggtat ttcgccctcc tgccgcagga caacacctgc cggccggaca 1282140
aaatctcccc caaatcggga ttaatctgcc agcgcagctt caattggcgc aataccacgg 1282200
cttccaccgt tttcccttca atatgcggcg cgatgccgcg caacgtcgtt tccacttccg 1282260
gcaattcagg cataacccct cccgacattt cttctgacag atgccgtctg aaagacggct 1282320
gtccctaatc cgcaaccctt gccgcacccg ccgcaagggc tttgccgccc aaatacccgt 1282380
cccacgcggg caggggcgtt acgcccgctg cccttttgac caaaaccaaa ccgtacactg 1282440
cggcacactg cggccaccaa cggtcgcccg ccttttccat aaaccgccaa aagcgtattt 1282500
gcccgagcga cgaaaccggc ggcagataca ccataaattt cccaaattca atatcgaaac 1282560
cgacatccgc aagccgtctt ttcaactcgg gcagcggcag acaaaaccgt ttttccggca 1282620
ggcgttcgcc gtcaaaccaa cggctgaatc cccagagcga atacggattg aaacccgtca 1282680
gcatcaagcg tccgcacggt ttcaatatcc ggtgcgcttc cgacaggatt tgcgaaggaa 1282740
caccgccttc aagcgtatgc ggaaaaagca gcatatccgc agaaacatcc gccaaagcca 1282800
tattctccgc cgacatcgac atatctcgcg gcacacagac aacatcttca gacaggctca 1282860
gccacggacc gcccacctga accgcacaca ttcccgaaaa acggtatgaa tccagatacc 1282920
gcccgaagaa atcctgttcc aattttgcaa cataccgccc catcgccgta tcttcaaacc 1282980
atgcatccat attgcgtccg tttcaaacag attgcctgcc gatattctat tccaaacagg 1283040
atttctgtca aaaaacacat cggccgccca tttccgaatc cgcataaagt tcctgtaaaa 1283100
cttgacgctt tttcagtcaa acagtaccat cggacgataa aatatgttta ttccgccgaa 1283160
tataaatcat gtccaaactc aaaaccatcg ctctgaccgc atcaggtctg tccgtttgtc 1283220
cgggtttcct atacgcccaa aacacctcat cacaccaaat cggtttggcg attatgcgct 1283280
taaactcttc aatactcgac ctgcccccga caaaacaata tttccaatcc ggcagcctgt 1283340
ggggcgagct gcgccaaggc ttccggatgg gcgaagtcaa tcccgaactg gtacgccgcc 1283400
acgaaagcaa attcatcgca agccacagct atttcaacag ggtcatcaac cggagtagac 1283460
cctatatgta ccatatcgcc aacgaagtca aaaaacgcaa tatgcccgcc gaagccgccc 1283520
tgcttccctt catcgaaagc gcgttcgtca ccaaagccaa atcacacgtc ggcgcatcag 1283580
gattatggca gtttatgccc gctaccggca ggcattacgg cctggaaaaa acaccggttt 1283640
acgacggcag gcacgacgtt tacgccgcca ccgatgccgc actcaactat ctgcaatacc 1283700
tctatggact gttcggcgac tggccgcttg cctttgccgc ctacaactgg ggtgaaggca 1283760
acgtcggacg cgccatcaac cgcgcccgcg cccaagggct cgaaccgacc tacgaaaacc 1283820
tgcgtatgcc caacgaaacg cgcaactatg tccccaagct gctcgccgtg cgcaacatta 1283880
ttgccactcc ccaatctttc ggcatgaata tcagcgacat agacaacaaa ccctattttc 1283940
aggcagtcga accggatcgt ccgctcgaca acgaagccat cgcccggctt gccggcatca 1284000
cgcaaagcga gctgctcgcc ctaaaccccg cattcaacgt ccccgcgttt atccccaaaa 1284060
gcaaacgcaa actgctgctt cctgtcgcgt ccgtacaaac cttccaaagc aactacctca 1284120
acgccgcacc cgacagcctg ttttcatggg aagtctatac gcctgccgcc aaaaccagcc 1284180
tgtccgacat ctcgacggca accggcatga gcattgccga catcaaacgc ctcaacaacc 1284240
tgaacggcaa ccttgtcaac gcaggacgca gcatccttgt cgccaagaac ggcaaaaccc 1284300
ttcagacggc atcggaatcc gtcgtttcca tcgacatcga caatacgccc gacacctacc 1284360
gttccaatat gccggcaggc acggtgaacg tcggcattgc ccgaatccga cccgccgccg 1284420
cacagacagc ggacattacc gtcgcacctt tgccgcagaa aaccgtccgt acggaacccg 1284480
atccccttgt ccgtattgcc gaacctgccc ttgcgacagc cgcagcgcaa cctcaaaccg 1284540
aaaaacagac cgccatgccg tctgaaaccc aaaccgcaac actcgcgcag atcatccccc 1284600
aaaacgacat gcaggcggca gacgaactca tgcagcttgt tgcccgaaac aacctgcgcc 1284660
gacaggctga agaaaccatc tccgccgtca tcggcacgcc tgacacagtt gccgaacaca 1284720
aaatttccgc atctccgcaa cataccgctg ccgccgacgg caaacgccgg gtacgtttgg 1284780
aaacgcgcgt agccaaagct gccgacggcg aagccgaaat ctccccgctc catgccagca 1284840
tccaccgcgt tgtagaaggc gacaccctgt tcaacattgc caaacgctac aacgtcagtg 1284900
tagccgacct gattgtcgcc aacaacatca aaggcaacac catccaaaaa ggacaggtac 1284960
tccgcctggc gcaggcagcc cctgcccaaa cccgtattga aaaagtatcc tacaccgcgc 1285020
gcaaaggcga caccttcaaa agtatcgccg cgcgcttcaa tatccatatc gacgacatcc 1285080
gccggctcaa tcccaacctg aacaccatca atccgggaca gagggtcaaa ctgattggaa 1285140
gctgattcgg atacggcaca tgacaggact ttctcagtcc tgtctttttc atcccacatt 1285200
tcccatacca tcatgaaact tatcaaatac ctgcaatatc aaggcatagg aagccgcaag 1285260

cagtgccaat ggctgattgc cggcggttat gttttcatca acggaacctg catggacgac 1285320
accgatgcag acatcgattc ctcatccgtc gaaacgttgg atattgacgg ggaagcagta 1285380
accgtcgttc ccgaacccta tttctacatc atgctcaaca agcctgaaga ttacgaaact 1285440
tcgcacaaac ccaagcacta ccgcagcgta ttcagcctgt tccccgacaa tatgcggaac 1285500
atcgatatgc aggcggtcgg caggctggat gcagatacga ccggcgtatt gctgattacc 1285560
aacgacggca aactgaacca cagcctgact tcgccgagca gaaaaattcc caagctgtac 1285620
gaagtaacgc tcaaacaccc cacaggagaa acgctctgcg aaaccttgaa aaacggcgtg 1285680
ctgctccacg acgaaaacga aaccgtttgt gccgccgatg ccgttttgaa aaacccgacc 1285740
accctgctgc tgaccattac cgaaggaaaa taccaccaag tcaaacgcat gatcgccgcc 1285800
gccggcaacc gcgtgcaaca ccttcatcgc cggcgattcg cacatctgga aacagaaaac 1285860
ctcaaacccg gggaatggaa atttatcgaa tgtccaaaat tctgaaataa catccaagaa 1285920
ttccatttat attcatccac atactcatta aatatatggt ttaacccaat ttaacgcaaa 1285980
aaatcaattt acgatataat ccatttgtct gagtaacgcc tgttcaagca ggcttatgag 1286040
taagacgttt tccccgtaat gtgttttgcc atctatactt ctccccttgt atagatggtt 1286100
tttttatagt ggattaaatt taaaccagta cagcgttgcc tcgccttgcc gtactatttg 1286160
tactgtctgc ggcttcgtcg ccttgtcctg atttttgtta atccactata tttcaaattc 1286220
cctctctttc atcattattt aaatgctatc attaatataa ttaacaaaat ttcatttcca 1286280
tacaccgatg tagaagaata acaaattaat ctatttataa actccgattc ttcctcatcg 1286340
ggttatactc atcagaaata ctcactgaaa tatatgcctt ttacatggaa aatcactaga 1286400
cagttgaaaa gcaatcagtc agttcctgtt aaaaaacgcg tgtagaattt ccgctttatc 1286460
tcaatcggac acggtttcaa tatgtcttcc ccttcaaata ccaatcgtca aacctggtcc 1286520
agccgattaa cctatatcct gaccgttgcc ggcgcgactg tcggtttcgg cgcgacgtgg 1286580
cgtttcccgt atttggtcgg tgaaaacggt ggtgcgcgta tgtgttttta ttctgtatcg 1286640
cgatgctggt tatcggcatc ccgatgattt tggtggaaaa tgtcatcgga cggcgcaaag 1286700
gcgtgaacgc gctggatgcg ttcggcggcc cgatgaacgg caaacccatt gccaaaattt 1286760
ggaaactggt cggctggatg ggctgctcgg cgcgttcggc atcatggctt attacatggt 1286820
actcggcggc tgggtaatca gctatatcgt taatattatt ggaggaaatt tgaatatttc 1286880
cagccccgtc gacggtgtgg ttacaaaagg cttctttgcc gaacacattg aaacagccct 1286940
tgggaaattg cgttttatac gctgcttttt gtcgccgtga accaatggat tttggtcaaa 1287000
ggcgttatcg gcggcattga aaaagcggca aaatagctga tgccgctgct gtttttgttc 1287060
ctaatcgcga tggtcgtccg caacgttacc cttccgggcg caatggaagg ggttgctttc 1287120
tatctgaaac ctgatttcag caagattacc gccgaactgt tcgtcttcgt tttggggcag 1287180
gtattttttg ccctgagctt gggtttcggc gtgatgatta ccttgtccag ctatttggat 1287240
aaaaacgaaa atctggttca gacggcagtt atcacggcaa ttaccaatac catcatcgcc 1287300
atacttgcgg gctttatgat tttcccgtcg ctcttcagct tcggcgttgc cccccgattcc 1287360
ggcccgactt tggtgttcca aagcttgccg attgtgttct cacatatgtg ggcgggatct 1287420
gtgttcgccg tgattttctt ctcgctgctc ctgattgccg cgttgacaac ttcgctgacc 1287480
atttatgaag tgttgattac gaccattcag gaaaaaacca aaatccgccg taccgccgcg 1287540
attacgattg tattggctgc catcttcatt ttcggcaaca tcccgtccat tctgagctat 1287600
ggtccgtgga aagacgttcc gtgttcggca aaaatatttt cgatgccttc gactacatca 1287660
gcggcaacat cttgtttatg ctgaccgcgc tcggttccgc gctgtttgcc ggttttgtga 1287720
tgaaggacga agcgaaggac gaattgcttt ataaaggcaa ccatacgacg gtcaatattt 1287780
ggtttgctta tgtgaaatat cttgtgccgc tggtgattct gctgattttc gtcagcaacc 1287840
tgttctaatc cgcagcaatc gatgccgtct gaaggtcata cctctttcag acagcattgc 1287900
cttgatgccc gccacaatac ccgcaacgcc gaaatccgaa ccggcttgcc ggcttctgtc 1287960
ggatgtttcc gggcaggcgg cgttccgtct gcttagacaa tcgtccttta aaacaggtag 1288020
aatccgcccc aacgggaaac acatccttca gacggcaaaa cccatacccc aaaccatcag 1288080
gaatccccct tatgaacaga caaaaagtca tcgccatcga cggcccgggc gcatcgggca 1288140
aaggcacggt tgccgcccgc gttgccgccg cattgggata cgattatctc gataccggcg 1288200
cactctaccg cctgactgcc ctatatgcac aaaaacaagg cgtgggatgg cacgatgaag 1288260
aaaacgtttc cgaactggca aaaaaactgc ccgccgtatt ttcaggcagc cgcatcctgc 1288320
tcggcggcga agacgtttca gacggcatcc ggacagaagc catcggcatg ggcgcatccg 1288380
cagtcgcaca gttgcctaaa gtccgcgccg ccctgctgca acgccaacgc gattttctga 1288440
ccgaaaaagg actggttgcc gacggacggg acaccggatc ggtcgtcttc cccccaagccg 1288500
aacttaaaat cttcctgacg gcagaatcca aaatccgtgc cgaacgccgc gccaaacaaa 1288560
tcggcatccc ctgcgaaggt ttggcattcg agcgcatcct gtccgacatc gaagccagag 1288620
acgaggcaga ccgaaaccgc aaagttgccc ccctgaaaca acagcccgat gccctgcttt 1288680
tggacacaag ccgcctgact atagaagaaa ctgtaaaaaa agtgcttgat tggtatcgtg 1288740
aagtttaaat tttcaggtat aatcgcacaa attacgtttc agacggcata aaaatccccc 1288800
atatgccgtc tgaaacctta tgtacccgtc tgccctgcca aggacggcag atatccacca 1288860
acccaccccg caccccttgg cggtgtaccg aaaagagtta tatatgtcta tggaaaattt 1288920

tgctcagctg ttggaagaaa gctttaccct gcaagaaatg aacccgggtg aggtgattac 1288980
cgctgaagta gtggcaatcg accaaaactt cgttaccgta aacgcaggtc tgaaatcaga 1289040
atccctgatt gatgtagctg aattcaaaaa cgctcaaggc gaaattgaag ttaaagtcgg 1289100
cgacttcgtt accgttacca tcgaatccgt cgaaaacggc ttcggcgaaa ccaaactgtc 1289160
ccgcgaaaaa gccaaacgtg cagccgattg gattgccctg gaagaagcca tggaaaacgg 1289220
cgacatcctg tccggcatca tcaacggaaa agtcaaaggc ggcctgaccg ttatgattag 1289280
cagcatccgc gcattcctgc cgggttcttt ggtcgacgta cgtcctgtaa aagacacttc 1289340
tcacttcgaa ggcaaagaga tcgaattcaa agtgatcaaa ctggacaaaa aacgcaacaa 1289400
cgtcgttgtt tcccgccgcg ccgttctgga agccactttg ggtgaagaac gcaaagccct 1289460
gctggaaaac ctgcaagaag gctccgtcat caaaggcatc gttaaaaaca ttaccgatta 1289520
cggtgcattc gttgacttgg gcggcatcga cggtctgttg cacatcaccg atttggcatg 1289580
gcggcgcgtg aaacacccga gtgaagtctt ggaagtcggt caggaagttg aagccaaagt 1289640
attgaaattc gaccaagaaa aacaacgcgt ttccttgggt atgaaacaac tgggcgaaga 1289700
tccttggagc ggtctgaccc gccgttatcc tcaaggcacc cgcctgttcg gcaaagtatc 1289760
caacctgacc gactacggcg cattcgtcga aatcgaacaa ggcatcgaag gtttggtaca 1289820
cgtctccgaa atggactgga ccaacaaaaa cgtacacccg agcaaagtcg tacaactggg 1289880
cgacgaagtc gaagtcatga ttttggaaat cgacgaaggc cgccgccgta tctctttggg 1289940
tatgaaacaa tgccaagcca atccttggga agaatttgcc gccaaccaca acaaaggcga 1290000
caaaatctcc ggcgcggtta aatccattac cgatttcggc gtattcgtcg gcctgcccgg 1290060
cggcatcgac ggtttggttc acctgtccga cctgtcctgg accgaatccg gcgaagaagc 1290120
cgtacgcaaa tacaaaaaag gcgaagaagt cgaagccgtc gtattggcaa tcgacgtgga 1290180
aaaagaacgc atctccttgg gtatcaaaca actggaaggc gatccgttcg gcaacttcat 1290240
cagcgtgaac gacaaaggtt ctttggttaa aggttccgtg aaatctgttg acgccaaagg 1290300
tgctgttatc gccctgtctg acgaagtaga aggctacctg cctgcttccg aatttgcagc 1290360
cgaccgcgtt gaagatttga ccaccaaact gaaagaaggc gacgaagttg aagccgtcat 1290420
cgttaccgtt gaccgcaaaa accgcagcat caaactttcc gttaaagcca aagatgccaa 1290480
agaaagccgc gaagcactga actccgtcaa tgccgccgcc aatgcgaatg ccggcaccac 1290540
cagcttgggc gacctgctga aagccaaact ctccggcgaa caagaataag gttgcagaca 1290600
tgacaaagtc tgagttaatg gttcgtttgg cagaagtgtt tgccgccaaa aacggcacgc 1290660
atcttctggc aaaagacgta gagtacagcg taaaagtctt ggttgacacc atgactagat 1290720
cgcttgcccg aggtcaacgc atcgaaatcc gcggtttcgg cagcttcgat ttgaaccatc 1290780
gtcctgcccg catcggtcgc aatcccaaaa ccggcgagcg tgtggaagta cctgaaaaac 1290840
atgtacccca cttcaagccc ggtaaagaat tgcgcgagcg ggtcgacttg gctttaaaag 1290900
aaaatgccaa ttaaacctta gcatcaaaac gccgctgtta cgcggcgttt tttctgtggt 1290960
ttaacttcat ccgttgcttc aataccttga gccaagcaag caacggatta gagcgtggat 1291020
ttttttatag tggattaaca aaaaccagta cggcgttgcc tcgccttagc tcaaagagaa 1291080
caattctcta aggtgctgaa gcaccaagtg aatcggttcc gtactattta cactgtctgc 1291140
ggcttcgtcg ccttgtcctg atttttgtta atccactata tcattgctta caatccgctt 1291200
tttaaacaac aaatttttga tttctattac gaacaggaca aaaatcctgc ttattgcact 1291260
aaaactaagc cgtttcagga atttgcggca aatttacagc ttttaccgag cctaatgctt 1291320
tcgctttttg gtaaaacgcc aatttgtatt caagcaaatc taaatagcgt tttaattcgg 1291380
caatttgaca cttcacattt tctatttgat tttcaaacaa ggaaaggcgt tcttcaatgg 1291440
tatcgtcgcc aatgacggta cattccgcaa agcgtttgat gtcttttaag ctcattcccg 1291500
tatttttcaa gcattgcaat aagcccaacc attgcaaatc gttatcggta aaacagcggt 1291560
taccgtattc atcacgtccg atattgggca acaaaccttc tttgtcgtaa aaacgtaggg 1291620
tgtgggcgga gatgcctatt ttttcggcgg ctttggcagt agtataagtc attttccctc 1291680
cttctaaaca aaaacagtaa aaaacacttg ctttagagtt aactctaaag tgtaaactgt 1291740
tgctatgttg ctcaggcaag gcaactttgt caatgaatta agaggaaaga caatggaaat 1291800
gaaacaagcc gattcaacca tcaaatctcg tgcggcggtg gcattcgccc ctaaccaacc 1291860
cttacaaatt gtggaaatcg acgtagaaat gccgcgtaaa ggcgaggtgt taatccgcaa 1291920
tacccacact ggcgtgtgcc atactgatgc gtttacgtta tcaggaagcg atcctgaagg 1291980
cgtattccct gtggtgcttg gacacgaagg tgcgggtgtg gtcgttgctg tgggcgaagg 1292040
tgtgtcaagc gtaaaaccgg gtgatcacgt gattccgctt tacaccgccg aatgtggcga 1292100
atgtgagttt tgttgttcag gtaaaaccaa cttgtgcgtc tcagtgcgtg atacacaagg 1292160
taaaggctta atgccggacg gcacgacgcg tttttcttat caaggtcagc caatctatca 1292220
ctatatgggc tgttcgactt tcagtgaata ctccgttgtt gccgaagttt cactggcgaa 1292280
aatcaaccct gaagccaacc atgaacaagt atgtttgctc ggctgcggcg ttaccacagg 1292340
tattggtgcg gtacataata cggcaaaagt gcaagaaggc gactctgttg ccgtgtttgg 1292400
tttgggggcg attggtttgg cggtggtgca aggtgcgcgt caagccaaag ccggccgcat 1292460
tatcgccatt gataccaatc catcaaaatt tgagttggca aaacagttcg gtgcaacgga 1292520
ttgtttgaac ccgaacgatt acgataaacc gatcaaagat gtgttgttag acattaataa 1292580

atggggcatt gaccatacct ttgaatgtat cggcaatgta aacgtaatgc gtcaggcatt 1292640
agaaagtgca catcgtggtt ggggtcaatc cattatcatc ggcgtagcag gtgcaggaca 1292700
agaaatttca acgcgtccgt tccagttggt aacaggtcgt gtttggaaag gttcagcatt 1292760
tggcggtgtg aaaggtcgct ctgaactgcc gaaaatggtg gaagattcaa tgaaaggcga 1292820
cattgagtta gaaccgtttg taacccacac aatgacactc gatcaaatca ataaagcctt 1292880
tgacttaatg cacgaaggta aatcgatccg cgccgttatt cactactaag gtatgcgatg 1292940
aaactgattg aacaacatca aatttttggt ggttcgcaac aagtttgggc gcatcatgcc 1293000
caaacgctgc aatgcgaaat gaaatttgcc gtctatttgc caaataatcc agaaaatcga 1293060
ccgcttggtg tgatttattg gctttccggc ttgacgtgta cggaacaaaa tttcattacc 1293120
aagtcaggct ttcagcgtta tgcggcagaa catcaagtaa ttgtggtggc ccccgatacc 1293180
agccctcgcg gagagcaagt gccgaacgat gatgcttacg atttaggaca gagtgcaggc 1293240
ttttatttga atgcgaccga acagccttgg gcggcgaatt atcaaatgta tgattacatt 1293300
ttgaacgagc tgccccgtct gattgagaaa cactttccta ccaacggcaa acgttccatt 1293360
atgggacatt caatgggcgg acacggcgca ttggtattgg cgctgcggaa tcaggaacgt 1293420
tatcaaagtg tttctgcctt ttcgcctatt ttatcgccaa gcctcgtgcc gtggggagaa 1293480
aaagccttta ctgcttattt agggaaagac cgtgaaaaat ggcagcaata tgatgctaac 1293540
tcactcattc aacaaggcta taaagtgcaa ggtatgcgca tcgatcaagg cttggaagat 1293600
gagtttttgc cgacacaatt gcgtaccgaa gattttatcg aaacctgccg tgcggcaaac 1293660
cagccggtcg atgtgcgttt ccataaaggc tacgatcaca gctattactt catcgccagt 1293720
tttattggcg agcatattgc ttatcacgcc gcgtttttga agtaaaccaa agagcgttca 1293780
gtgttcaaag cagttttggg atagccggca cgagggcggt aagaagtgcc ggcataaacg 1293840
tatgccgtct gagccgaaag gagccgactc tacggattat agtggattaa caaaaatcag 1293900
gacaaggcga cgaagccgca gacagtacag atagtacggc aaggcgaggc aacgctgtac 1293960
tggtttaaat ttaatctact ataaaaggca tttgagctca tatctgcacc atattgaaac 1294020
gccgcctttg cttataccccc cttgtgcgcg tcattattct tttccacgga aaatgccaag 1294080
tttgaaggaa atcatttata ataccgacgg taagcatttt ctttcttagc cgcaagaagt 1294140
ataacaaggt taaatatgag taatagagac caacttttta aagccccgcc gtttgaaaac 1294200
cacagcccgc tgacctggta tcaggctgcc tcacaactgc ccaacttcat ccgcgacgac 1294260
gcgcaggcag ccgccatcga acacctcgat cggctttgga ccgaattgat gatgttcaaa 1294320
cgcaaaagaa accgtttttt aggcaggagt ttgcgttccc cgcaagtccc caaagggctt 1294380
tatttctatg gcggggtcgg acgcggcaaa agctttctga tggacgcttt tttcggctgc 1294440
ctcccgtacc gccgcaaacg ccgcgtccac tttcatgcct ttatggcaga aatccaccag 1294500
cggctgaaaa ccctgaaaag cgaaagcaac ccgttgaaat ccgttgccgc cgagattgcc 1294560
aaagaaaccc gcgtattgtg ttttgacgaa tttcatgtca gcgatattgc ggatgcaatg 1294620
attttaggcc gtctgctgga aaacctgctt aacgagggcg ttgttttggt ggcgacttca 1294680
aactacgcgc cttccgaact ctacccgcaa ggtcaaaacc ggagcagttt tcttcccaca 1294740
atcgcgctca tcgagtccag cctgaccgtc ttaaacgttg acggcggtga agactaccga 1294800
ctgcgtaccc tccgccccgc cgagattttc tttacgcctg ccaatgaaga aaatgaggca 1294860
aaactggcaa aactgttcaa agaaatgaca ggcattaccg atttgaaccc cggcatcagc 1294920
accatccacg gtcgggagat tccccacaaa gccgagtccg gccgtgccat atggtttgat 1294980
ttccgcgcac tgtgcttcgg cccccgctca cagtccgact atctgtattt ggccgaacat 1295040
tatgaaatgg tttttatttc aggtttggaa caactctcac cgcaagaaaa ggcggaggcg 1295100
cggcggctga cttggctgat tgacgtactc tacgatttcc gggtcaaact gtgtgccacc 1295160
ggcgcggtag atgtcaacca tatctatacg gaaggcgatt ttgccgaaga atttacccgc 1295220
accgccagcc ggatggtcga aatgcagtcc gaagtttatt tggaacagcc gcacctgacc 1295280
ctatctccca aggcttcagg cggataagtt attttttttga tagaataccg atttgattct 1295340
tcttaagtaa aaataaggat atagcatggc gattgaacgt accatctcca tcatcaaacc 1295400
cgatgccgtc ggcaaaaatg ttatcggcaa aatatacagc cgctttgagg agaacggtct 1295460
gaaaatcgtt gccgccaaaa tgaagcagct tactctcaaa gaggcgcaag aattttatgc 1295520
ggttcataaa gaccgcccct tctacgccgg attggttgaa tttatgaccg gcggtccggt 1295580
tatgattcag gtattagagg gtgaaaacgc cgtcctgaaa aaccgcgaac tgatgggtgc 1295640
aactaatcct tccgaagccg ccgaaggcac gatacgcgcg gactttgcca cttcggtcag 1295700
cattaatgcc gtacacggtt ccgacagcgt ggaaaatgcc gctttggaaa ttgcctactt 1295760
tttcagccaa accgaaatct gcccccgttg atacaataca ccgcccaact cctcttcaga 1295820
cggcataaat atatccatgc cgtctgaaaa ctctgttgca aaaggcttca aatcaaactt 1295880
gcctgccctg caatttttta tttgaagcct tgatttaaga aaaacacaaa cacatgaaaa 1295940
ccaatctgct caactacgac cttcaagggc tgacccgaca ttttgccgat atgggcgaaa 1296000
aacctttccg tgccaaacag gttatgcgtt ggatgcacca atccggcgcg caaaattttg 1296060
acgaaatgac cgatttggca aaatcgttgc gccataaact gaacgaacag gcaggcatcg 1296120
aaattcccaa gctgatgatg tctcaaaaat cttcagacgg cactcgaaaa tggcttttgg 1296180
atgtcggtac gggcaacggc gtggaaaccg tcttcatccc cgaatcggat cgcggcacgc 1296240

tctgcatttc ctcacaagtc ggctgcgctt tggaatgtac attttgttcg accggccggc 1296300
agggcttcaa ccgcaatttg actgctgccg aaatcatcgg gcaattgtgg tgggcaaaca 1296360
aagcgatggg cgttacaccg aaaaacgagc gcgtgatttc caacgtcgtc atgatgggca 1296420
tgggcgagcc gatggcgaac ttcgacaatg tcgttaccgc cttaagcatc atgctggacg 1296480
accacggcta cggtttgagc cgccgccgcg taaccgtttc cacttcgggt atggttcccc 1296540
aaatggacag gttgcgcgat gtcatgccgg tggctttggc ggtttccctc cacgcttcca 1296600
atgacgaagt ccgcaaccaa atcgtaccgt tgaacaaaaa atatcccttg aaagaattga 1296660
tggccgcatg ccaacgctat ctggtcaaag cacccaggga tttcatcact ttcgaatacg 1296720
tcatgttgga cggaataaac gataaggcgc aacatgcgcg cgaactgatc gaactggtca 1296780
cagatgttcc ctgcaagttc aatctgattc cgttcaatcc cttcccaaac tccggatacg 1296840
aacgctccag caatgagaac atccgtgtgt tccgcgatat tttgcagcag gcaggatttg 1296900
tcgttaccgt acgaaaaacg cgcggcgacg acatcgatgc cgcctgcgga cagttggcgg 1296960
ggcaggttca ggataaaacg cgccgccaac aaaaatggca gcagatttta atcggacaac 1297020
aggggtaatt atgcctttta agccatccaa acgaatctct ttattactcg ttcttgcctt 1297080
gggcgcgtgc agcacttcct accgcccctc gcgggcagaa aaagccaatc aggtttccaa 1297140
tatcaaaacc cagttggcaa tggaatatat gcgcggtcag gactaccgtc aggcgacggc 1297200
aagtattgaa gacgccctga aatcggaccc taaaaacgag cttgcctggc tggtccgtgc 1297260
cgaaatctat caatacctga aagttaacga caaggcgcag gaaagtttcc ggcaagccct 1297320
ctccatcaaa cccgacagtg ccgaaatcaa caacaactac ggttggttcc tatgcggcag 1297380
gctcaaccgc cctgccgaat ctatggcata tttcgacaaa gctctggccg accccaccta 1297440
cccgacccct tatattgcca acctgaataa aggcatatgc agcgcaaaac aggggcaatt 1297500
cggattggcg gaagcctatt tgaaacgttc cctcgccgcc cagccgcagt tcccacccgc 1297560
atttaaagaa ctggcgcgca ccaaaatgct ggccgggcag ttgggcgatg ccgattacta 1297620
ctttaaaaaa taccaaagca gggtagaagt ccttcaggcc gatgatttgc tgctaggctg 1297680
gaaaattgcc aaagccctcg gcaacgcaca ggcggcatac gaatatgaag cacaattgca 1297740
ggcgaatttc ccctactcgg aagaattgca aaccgtcctc accggtcaat aaacagattc 1297800
aaaccatatg aacacactcc aacgccgcaa gacgcatcaa gtccgcatcg atcatattac 1297860
cgtcggttca gaagcacccg tcgttatcca atctatgacc aacaccgaca ctgccgatgc 1297920
aaaagccacc gcattgcaga ttaaggaatt gagcgatgcc ggatccgaaa tggtgcgtat 1297980
taccgtcaac agccccgaag ccgcgtccaa agttgccgaa atccgccgcc gcttggacga 1298040
tatgggctat gccacaccgc ttatcggcga tttccacttc aacggcgaac gcctgttggc 1298100
ggaatttcca gaatgcggca aagcattgtc caaataccgc atcaatcccg gcaatgtcgg 1298160
caaaggcgta aaaggcgatg aaaaatttgc ctttatgatt cggactgctg ctgaaaacga 1298220
taaagccgtc cgcatcggcg taaactgggg ttctttggat caaagcctcg ccaaacgtat 1298280
gatggatgcc aacctcgctt cttccgcgcc gaaaccgccc gaagaagtga cgaaggaagc 1298340
actgattgtc tccgctttgg aatctgccga aaaagccgtt ctattgggac tgcccgaaga 1298400
caaaatcatc ctgtcgtgca aagtcagcgc ggttcaggat ttgattcagg tttaccgcga 1298460
actgggcagc cgttgcgcct atccgctgca tttgggtttg accgaagccg gtatgggcag 1298520
caaaggcatt gtcgcatcaa cggcggcatt atccgtcttg cttcaagaag gaatcggcga 1298580
caccatccgc atttcactga ctccggaacc tggcagcccg cgtactcagg aggtcgtcgt 1298640
cgggcaagag attttacaga ctatgggatt gcgttcgttt acgccgatgg ttaccgcctg 1298700
ccccggctgc gggcgtacca ccagtaccgt atttcaagag ctggcacaag atgttcaaaa 1298760
ttacctgcgc caaaaaatgt ctatatggcg taccctttat cctggggttg aatccctgaa 1298820
cgttgccgta atgggctgcg ttgtcaatgg gcccggagaa agcaaattgg ccgacatcgg 1298880
catcagcctg cccggtacgg gagaaacacc cgttgcccct gtttatgtag atggtgaacg 1298940
taaagtaacg ctgaaaggcg acaacattgc aacggaattt ctggctattg ttgaagagta 1299000
tgtcaaaacc aattatgggg agaacggact caaacgccat caagggaagg ttatcccgat 1299060
acactcccta taaaatccaa ccgccagcct accttggcta tttttaaata aaaaccgttt 1299120
attttcattg atataaaacc catcccattg gaaaaggcat ttttttaaac cgataaggaa 1299180
tggaggcgca atatgaaaat acaatcggca aatacggaga tgaattgcag gtataaataa 1299240
ggaaggcggg ataccgcttc ctgcccttgt cttttctcaa taaccgtacc ggcagattca 1299300
ggaggatgcg agtgtcgcgc ccttgcaaaa actgctgccc catttggctt tcagacggca 1299360
tccgtccatc acaggcggga acggggatcc tttaaaaaac tccaaatcct tcttccgtcc 1299420
cgtggatgac ggtatcgata ccatatcaaa cgcagcttga aacaaatgcc gtctgaacgt 1299480
ttcagacggc atcggtttct ctcaggtttc ttttattaaa gccgcaaaga agcgcggttt 1299540
tccaaaatct ggtcaatcaa accatattct ttggcttctt cggcagacat gaaattatca 1299600
cggtcggtgt cgcgttccaa atctgccaaa tcgcggtcgc aatgtttcgc catcaggcgg 1299660
ttgagttttt ctttgatttt taaaagttcg cgtgcgtgaa tttcaatgtc ggatgcctga 1299720
ccgcccagac cgccgctgat taaaggctgg tgaatcataa tccggctgtt gggtagggca 1299780
aaacgtttgc ctttctcgcc tgccgacaat aagaacgcgc ccatacttgc cgcctgcccc 1299840
aagcacaaag tcgatacatc gggcttgatg aaattcatgg tgtcgtaaat cgacataccg 1299900

gccgttaccg aaccgcccgg cgagttaata tagaagaaaa tatccttatc cggattctca 1299960
ctttccaaaa acaacagttg ggcaaccacc agattggcgg actcgtcggt taccggtccg 1300020
accaagaata cgatgcgctc tttcaaaagc cgggaataga tatcgaatgc acgctcaccg 1300080
cgaccgctct gctcgataac ggtagggaca agatagttat caaaagacat ttcgtctcct 1300140
ttcatgatgg aaaagcacca aagcgagctt taaaagcggc ttcggtgctt tcaaaaactg 1300200
ccttcagacg gcattttcag gataatcagg cttgcgcgcc catcacttcg tcaaaagaca 1300260
aagctttttc atttactttg gctttgccca aaacgaaatc aacgacgttg ctttctaccg 1300320
ccaaagaagt cggggcttgc aggcgggaag gatctgcgta gtaccagtca atcacttctt 1300380
gaggatcttc gtagctttct gcaaagttgg caacaacggc tttgatttgc tcttcagtcg 1300440
gttccagttt gttttcgtca accagtttgg ctaaaatcag acctaaagat acgcggcgtt 1300500
cggcttgttc tttgaacata tccaaaggca gatccaagtt ggcagcatca gccatacctt 1300560
ggttaacaaa attttgtttc atttcgtttg ccaagcgtgc ggcttcttca ttgaccaaag 1300620
caacaggtgc tttcagctct acggctttga gcagcgcgtt cattacggat tctttggttt 1300680
gttcgtttac gcggcgttcc acttcgcggc ttacgttttt ctgcacttct tcgcgcattt 1300740
tggcaacgtc gccatccgca atacccaagg cttttgcaaa atctgcatcg acttcaggca 1300800
gagtcgcttc ggaaacgttg ttcagcgtaa tggtaaacac ggcagtttta ccggcaacgt 1300860
ctttaccgtg gtagtcttca gggaaattga cggtaacgtc tttactttcg ccagccttca 1300920
tgccgactac gccggcttca aattcaggca gcatttgact tgcgcccaat acgaaggcgt 1300980
agtttttgga tgcgccgccg gcaaaaggtt cgccgtcgat tttgccttca aagtcaatga 1301040
tgacgcggtc gccgtttcgg gcttcgcgtt cgacatggtt gaagcgggtg cgttgtttgc 1301100
gcaggatttc tacggtttgg tccacttcgg catcaccgac ggaagcggtt actttttcaa 1301160
cttcttgtgc agacaaatcg ccgataacga cttcggggaa cacttcaaaa atggcggcaa 1301220
ctttgaaaga ctctttatcg tcttgttctt caacgccttc aaaacggggg aagcctgcca 1301280
ctttcaactc ttgggcaacg gcaacatcgt agaagcggcg ttgcaccagc tcgttgatca 1301340
cgtcgttttg tgcgctcgca ccgtacattt gggcaatcat ttttaaaggt gctttacccg 1301400
gacggaaacc gtcgattttt gcacggcgtt gggtttgttt cagtttttta tcggtttctg 1301460
cgttgatttc ggaccaaggc agggacaaca ctactttgcg ttccagattt tctaaagttt 1301520
caacagttac gctcatcata agcccttaaa tttgttgtgt tgataaaatg ataaactttc 1301580
ttccctacat ggggaagcaa acagcgcaac ggtacgatat ttgaaccgca ttgccgcaaa 1301640
ggggaaattt tagctggcaa gtatatcaca atgtttcgcc tgaaacataa tatgccgtct 1301700
gaaacgccaa ttccgccgtt cagacggcat tttgcaatac gggctacaaa tggtccttgt 1301760
gcgccaaaat tttacggctg ccgttgaggt cggtggaaga aacgacaccc gcatttttcca 1301820
gtgcctccat caggtttgcc gcgcggttat agccgatgcg cagctgccgc tgcaaagacg 1301880
aaatggaggt ttttttgctt tccaaaacat aggcgactgc ctgatcgaac aattcgtcgc 1301940
tgtctgcatt cggattaacg atattggcag tttccagcgc ggcctcgccg ctgagcagac 1302000
cttcaatata gtcggctggg gcttgcgatt tgacatagtt gacgacttga tgtacttcgt 1302060
cgtctgaaac aaacgcgcct tgcaggcgag tcggttcggc actgccgggc tggaggaaca 1302120
gcgaatcgcc atatttgagc agttcgtccg cgcccatttg gtcgaggatg gtacggctgt 1302180
cgattttgct ttgcacggta aacgccatac gcgtcgggat gttggcttta atcaggccgg 1302240
taacgacatc gacactggga cgttgggtgg cgacaatcat atggataccg gcggcgcgcg 1302300
ctttttgggc gagacgggcg atttgctgct cgacggcttt gcgttcggtc atcatcaggt 1302360
cggcaagttc gtcgataacg accacaatca acggcagttt ttccagcggc tcgggctcgt 1302420
cggggttcag gctgaacgga ttgagcagcg gcttgcctgc cgcttttgcg gcttcgactt 1302480
tttggttgaa gccctccaaa ttacgcacac cggcatggga aagcaggcgg tagcgttttt 1302540
ccatttcggc gacgcaccag ttcaacgcct gccctgcttc gcgcatatcg gtcacgacgg 1302600
gacagagcag gtgcggaata ccgtcgtaaa tgctcaactc gagcattttc gggtctatca 1302660
taatgaagcg gacttcgtcg ggcgtagctt tgaaaagcat agacataatc atgccgttca 1302720
cgccgacgga cttgcccgaa ccagtcatac cggcgaccaa aaggtgcggc attttcgcca 1302780
agtcgccgac aacgggggta ccggcaatgt ctttgcccag cgcgacggtc agcttggatt 1302840
tggcttcggc aaacacgggc gaggacaaga tttcactcaa catcacgtct tggcgtttgt 1302900
cgttgggcaa ctcgatgccc atcgtgtttt tacctgcgat ggtttcgacg atacgcacgg 1302960
actgcagcga catagagcgt gccaaatctt tcgacaaggc aacaatttgg ctgcctttaa 1303020
caccttgcgc gggttcgatt tcgtagcgcg tgatgacggg gccggatgtg gcggatacga 1303080
cttgtacgcc gatgccgaat tctgccagtt tggattcgat cagttcggca gtgcgctcca 1303140
attcggcggg attgatgctg acgggttcgc tgtcaggaat ccgcaatagg ttcaatgtag 1303200
gcttgtggta ttcgcccgcc tgccgaggtt cgtcatcttc aaacagagaa acctgaattt 1303260
tgggcggcgg cgcgacggaa accgcgacgg atttgcggtt gctgctgctg ccttcgggca 1303320
aggcaacggg tttggccgta atattcttgg cttcttttac catgcgccgt gtattttggg 1303380
tatcgacacc gtctgttttg gtattcggcc ggcgttttcc taaagccatg accttgccgg 1303440
ataaggcact caggcggttt tgaaccgccc tgcccgcacc gttcaaaaat tccagccatg 1303500
aaatctgcac cagcagggac aacgacaaca gcagaacaac caagataatc agcaggctgc 1303560

ccgatttccc cagcagccac gcaaacactg cgccgacgcg tatgccgacc ataccgcctg 1303620
ctccgacagg cagggagtcg gcatattttc cgcccagcac aaaatactcc aagacggggc 1303680
tgaagaccgt caggacaaac agcgcggcgg cagcgatttt gtggttgtat gcctcgtttt 1303740
ccgtctgttt tgcgtgcagg cggaaatttt tatacagcac gacgcaggca gccgctatcc 1303800
accaccagaa cgaccagccg aaaagataat agccgacatc ggcaacatac gcgccgaaca 1303860
gtccgcccca attggcgaca tcttccacaa ccggcgaact gtgcgaccaa gacggatcgc 1303920
ccatatcgaa actgatcagg gaaatcgcca aatacagggt tgccgccaaa cccatcagcc 1303980
acagtgcgtc gccgataagg ttgacgacat gttcgggacg cgcctttttg gtttcggttt 1304040
tctgcaactc tttgaccgcc ttgagccgct cggaaacttt attgccccgt gcgccgttgt 1304100
cggctttctt gttgcgtgcg gacgttgggg acgggcttcc cgccctgcct tttgctgttt 1304160
ttttatggga tttttctgtc atgccgtatt accggaaaat gccgtctgaa ataaggaagc 1304220
cggacggctt gcggattgaa tatggaaagt gtcggattat actccatttc tcctgctttc 1304280
atcctgcaac agacagacat tgcctttcag acggcatatc tgttgcccga cgtatgtatt 1304340
tttacgcaga ccctcggcaa accagtataa tccgtgccgt ttgaaccgat tgaaagaaga 1304400
tggtatgaac caactgaaac ttgccgtttc cggtgcacag attttatttg tggcattcgg 1304460
cgcaatggtg ctggttcccc tgctgaccgg tctgaatccg gctcttgcgc ttttgggcgc 1304520
aggcttggga acgctgctgt tccaaatcac aaccaaacgc aaagtgccga tttttcttgg 1304580
ttcttcgttt gcctttatcg caccgattat ctactccgtc ggcgaatggg ggctgccttc 1304640
caccatgttc ggactgtttg ccgccggctt tatgtatttt gtgtttgccg cgctgatccg 1304700
ttggcgcgga ctggcagcgg tacacaaact gctgcctccg gtcgtcatcg gccccgtcat 1304760
catggtcatc ggtctgtctg ttgccgtggc ggcaagcagc atggcaatgg gtcaggcgga 1304820
cggcaaacag gtcatcgact ataccgattc gctgattctt tccggcttta cctttgccgt 1304880
taccgccatc gtatcggttt tcggcagcag gatgatgaag ctgattccca tcttgatcgg 1304940
tgtcgcttcg ggttatgttt tggcactgct gatgggactg gtggacacgg caagcattgc 1305000
acacgcgccc tggttcgccg ttccccattt tgaaacgcct cagatcaact ggcaggctgc 1305060
actgtttatg ctgcccgttg ccgtcgcccc cgccatcgaa cacatcggcg gcatcatggc 1305120
aatcggcaat gtgacgggga aagactatac gaaagacccg ggcttggaca aaacccttgc 1305180
aggcgacggt ttgggcgtat gcgttgcggg tctgatcggc ggcccgccgg ttacgaccta 1305240
cggcgaagta acgggtgcgg tgatgattac caaaaacagc aaccccgtca tcatgacttg 1305300
ggcggcggtt tttgccgtct gcatggcgtt tttcggcaaa ttcaatgcgt ttttggcttc 1305360
cattccgatg ccagtaatgg gcggcattat gctgctgctg ttcggcacga ttgcttcttt 1305420
gggcgtgaaa acgctgattg atgccaaagt cgatttgatg ctgccgaaaa acctggtcat 1305480
cgtcagctcg gtactgacca cgggcatcgg cggcatgacg ctcaaattgg gcagcttcag 1305540
ctttgccggc gtgggcttgt gcgccgtact tgccattatg ttgaacagcc tgctgcccga 1305600
tccgaaagaa tcctgaccgt cgatatagaa atgccgtctg aacatctttc agacggcatt 1305660
ttccgtttta tttgagattt tgaatcaaag agcgcacagt tccgccgtaa taagaagaag 1305720
atgtgcaata cactgtttcc aagccgcatt gtccctgtac gccgtatttt ttgatgcact 1305780
ggttgagtgc gacctgatga acgctcgtaa aacgcggaga agtaatcacg acggcgttgt 1305840
cgacacgcag cgcgcccaag gctttcgggt atgccagcgc gacacaggta ttgttcagcg 1305900
acacgaccga ccggcatccg gtcggctcgt cttcagcaat gcccgcaagc gtgtcctgac 1305960
ctttgcagaa ggcttccaac tcggaaaacg cttcgctttt cgtcgaatct tcttttgtgg 1306020
ttttaacctg caaaacatcg tccgcattct gcggattctg ccaaacggcg agatagccgt 1306080
aagtatcggc agcccgtgcc gccgcagtca tcaggcatag tgccgatacg gccagtatct 1306140
ttttcatcat gataaattcc cgacggttcg tccaaattct gttgcattat aaacaaaaaa 1306200
caggataagt cccgccttat cggcttatcc ctccccgcag attgcaccgc cgggtatggc 1306260
aaaccgattt cagcagcgca aatccgcata ccgccgcctt agcggcaagc cgttgttttc 1306320
agacggcatt gcggccaacc tttgcggcgg gcgaaaaacc ttgtcctata atttatcccg 1306380
tttcaaaatc agcatacggt cggaaatgca aaaaatatct ttcaatttgt tgaagcctgc 1306440
aaactccccg aaaataggga aacgccgccc cggtttgaac ggcgcgccgc atattccgat 1306500
gccctccccc cgataccttc cggcaagccc agaaatgccc ggcaacaaca tccatccggc 1306560
aaaaatccga aacaacacac ccggcggcag gcagagtcaa accgccccgc aaagcatccg 1306620
ccatcagaaa aacaaaccgc ctccgagggc ttcatcctaa agggcgtatt gttcgataat 1306680
ggtttgggtt ataatcccct atcgattctc cacgtccgtg agacacttca gctatggaaa 1306740
ccccgaccaa cacccccgcaa cgctccctgc gtcaaaacag tatctacctg ctgcccaatt 1306800
cctttactat cgccgcgctg ttttccgcgt tttacgcaat cacccaatcc atgcacggac 1306860
gttatgaaac cgccgccatc gcggtattca tctctatgtt gctggacggt atggacgggc 1306920
gcgtggcgcg gctgaccaac agccaaagcg cgttcgggga gcagctcgac agccttgccg 1306980
atatggtcag cttcggcgtt gctcccgctc tgattgccta caaatggcag ctttggcagt 1307040
tcggcaaaat cggttattcc gtcgccttca tctactgcgc ctgcgccgcc ctgcgcctcg 1307100
ccctgttcaa cacactcatc ggcaaggtgg acaaacgctg gtttatcggc gtgcccagtc 1307160
cgactgccgc cgcgctgatt gtcgggctga tttgggtcaa ccacagcgtc gaaaaattcc 1307220

ccgccgtcca ctggtgggca ttgggcatca cactgtttgc cggcctgtcg atgattgtcc 1307280
aaatcccttt ttggagtttt aaagaaatca acatccgcag acaagtcccc tttgtcggaa 1307340
tgctgcttgc cgtcttactg ctgcttctgg tcacttggga accgtcgctc gtcctcttcc 1307400
tgttctttct cggatacagc ctgtccggct acattatggc ggcacgccga ttttggaaaa 1307460
agtacagaaa ggcggattaa atgtggcatt gggacattat cttaatcctg cttgccgtag 1307520
gcagtgcggc aggttttatt gccggcctgt tcggcgtagg cggcggcacg ctgattgtcc 1307580
ctgtcgtttt atgggtgctt gatttgcagg gtttggcaca acatccttac gcgcaacacc 1307640
tcgccgtcgg cacatccttc gccgtcatgg tcttcaccgc cttttccagt atgctggggc 1307700
agcacaaaaa acaggcggtc gactggaaaa ccgtatttac gatgatgccg ggtatgatat 1307760
tcggcgtatt cacgggcgca ctctccgcaa aatatatccc cgcgttcggg cttcaaattt 1307820
tcttcatcct gtttttaacc gccgtcgcat tcaaaacact gcataccgac cctcagacgg 1307880
catcccgccc gctgcccgga ctgcccggac tgactgcggt ttccacactg ttcggcacaa 1307940
tgtcgagctg ggtcggcata ggcggcggtt cactttccgt ccccttctta atccactgcg 1308000
gcttccccgc ccataaagcc atcggcacat catccggcct tgcctggccg attgcactct 1308060
ccggcgcaat atcgtatctg ctcaacggcc tgaatattgc aggattgccc gaagggtcac 1308120
tgggcttcct ttacctgccc gccgtcgccg tcctcagcgc ggcaaccatt gcctttgccc 1308180
cgctcggtgt caaaaccgcc cacaaacttt cttctgccaa actcaaaaaa tcttcggcat 1308240
tatgttgctt ttgattgccg gaaaaatgct gtacaacctg ctttaaaaca cacgaaaaaa 1308300
ccttttttacc gtttgcacaa gcaattaatc aggacaaagc tgcccagtct cctgttccga 1308360
caaaaggaca gacaacctga ccgagacctt tgcagaatat acgaaaaaca aaacaaatac 1308420
cgtctgaaac cacattccga caatcggcag ggtttcagac ggcatctgat aatttcaatt 1308480
actcggttgc ggcaacgacg gcaacggtaa ttttagcaac ggcatcagtg tgcaaagcca 1308540
cttccacttc gtactcgcca acggctttca gaggaccgtt cggcagacgt acatttgctt 1308600
tcacggcttc gatgccggca gcaacgattg cagcagcaat gtcggcattg gtaacggaac 1308660
cgaacaggcg accgtccaca ccagcttttt gagcaacggt aacggtttga ccgtccaatt 1308720
tttcctgacg gactcgggca tctgccaaaa tttcagcctg tttggcttcc agttctgcgc 1308780
ggcgtgcttc aaactctttc atattcgctt cggtcgcacg ttttgcctta cctgcgggaa 1308840
ttagaaagtt gcgggcgtag ccgtttttaa cggttacgat gtcgcccaag ttgcccagac 1308900
cgccgatttt ttctaacaga ataatttgca tgattcaatc tccaaaatta tttgtgttgg 1308960
tcggtgtaag gcagcagagc caggaagcgt gcgcgtttta cggcaacagc caattggcgt 1309020
tggtagaatg ccttcgttcc tgtgatgcgt gcaggaatga ttttaccgtt ttcggagatg 1309080
aagtctttca gcaaatcaac ttgtttgtaa tcgacttctt ggattttttc agccgtgaaa 1309140
cggcagaatt ttctacgttt gaatgattga cgagccattg tcgtttaacc tttatattct 1309200
tgaatatttt gtatcctgag catcggcata agggaacgtc tgcttttttg agctaaaaaa 1309260
ccttcgacgt gaacatatac accttgccga tactgccact cttccgcctg cctgcctaaa 1309320
atccgtgccg gaatttccaa ttggacaagg cattgctgcc cgttttcctc ctgccacgat 1309380
tcgtgcttta aaataatatc taaaacaggg attccggcag gcgtatatcg aatagggaaa 1309440
accttttcaa ttaacgcggc aagcgaaaca agattattga atcccaatta ttgggcgacc 1309500
gcttcttcag acgcaccgct caacaggttc ttagcctttt caccacccaa catagggggat 1309560
gcttcggtaa cggcgtgttt ggttttgatg gtcagatgac gcaatattgc atcattgaag 1309620
cggaatgcgg tttccagctc ttcaaccact tcgggagtgg tttcgatgtt catcaaaacg 1309680
taatgggctt tatggatttt gttaatcggg taagccagct ggcggcgacc ccagtcttcc 1309740
agacggtgaa tcttaccgtt tgcttcggca atcatggttt tgtaacgttc aaccatagcg 1309800
ggtacttgct cgctttgatc gggatgaacg ataaacacga tctcgtaatg acgcatgtta 1309860
tctccttatg gatggtaaaa acagccttct gccatgcgaa agcagaaggc aaggtttaaa 1309920
gaagcggcat tatattgggg tttgccgacg gaatcaagga tttggtgcga aaaatttgca 1309980
ttccgccgaa aatttcggtt tcagacggca ttcaaatgtt ttggctgccc agccagcgtt 1310040
ccgcgtccaa agccgcctga cagccggaag ccgcgctggt aatcgcctga cggtaggtat 1310100
ggtcttttac gtcgcccgcc gcccatacgc cttcgatatt ggttgcgccg acattgtccg 1310160
ccgtgccgcc tttggttttc aggtaaccgg cttcgtccat ttccaactga cctttgaaaa 1310220
tatcggtatt cggcttgtgc ccgatggcga taaaaatgcc gctgacggca atttgttgct 1310280
cagaaccgtc gttgtttttt aataatgcgc cgtttacgcc ccgatcgtcg cccagtactt 1310340
cttgcaggtt gctttccagc ttgaggatga ttttgccctc ttccacgcgt ttcatcagtt 1310400
tgtcgatcat gattttttcg gcacggaact cgctgcggcg gtggatcagg gtaacggttt 1310460
tggcgatatt ggcaaggtag agtgcctctt caactgccgt attgccgccg ccaactacgg 1310520
caacatcttg gtttttatag aagaaaccgt cgcaggtggc acaagcggaa acgccttttc 1310580
ctgcaaacgc ttcctcactc ggcaaaccga ggtatttggc ggacgcgcct gttgcgacaa 1310640
tcagggcatc gcaagtgtac tcgcccatat cgcctttgag tgtaaacggg cgtttttgca 1310700
gatcgacggc gttgatttgg tcaaaaatga tttccgttcc gaaacgttcg gcgtgggcga 1310760
gaaaccgcgc catcaattcc ggcccttgca cgccgtcggc atcggcaggc cagttgtcca 1310820
cttcggtggt ggtcatcagt tgcccgcctt gcgcgatacc tgtaataatg acggggttta 1310880

aattggcgcg cgcggcatag acggcggcgg tgtatccggc ggggccggaa cccaaaataa 1310940
tcagtttgcg gtgttgggac attgtttttc ctttgctgtg tcaagttttc ggattctact 1311000
cgaattatcg gcgcgtttga gaaatttcga ccataccggc gctcagacgg catcccgcag 1311060
ccttaactgc cgtctgaata tcaaagcagg aatcacgctt atgcaacaaa aaatccgttt 1311120
ccaaatcgaa ggcatgacct gccaggcctg cgcttcgcgc attgaaaaag tgttgaacaa 1311180
aaaagatttt gtcgaatcgg cgggggtaaa cttcgccagc gaagaggcgc aggtagtgtt 1311240
tgacgacagc aaaacctcag tagccgacat tgccaaaatc attgagaaaa ccggttacgg 1311300
cgcgaaggaa aaaacggaag atacattgcc gcaacccgaa gcagaacacc atatcggctg 1311360
gcggctgtgg ctgctgttca ccatcaacgt cccgttcctt atcggcatgg cggggatgat 1311420
gatcggcaga cacgattgga tgattccgcc gttgtggcag ttcgcattgg caagcgtggt 1311480
gcagctttgg ctggcaatcc cgttttacaa aagcgcgtgg gcgagcatta agggcggact 1311540
ggcgaatatg gacgtgctgg ttaccatcgg cacggtctcg atttacctgt attccgtcta 1311600
tatgctgttt ttcagcccgc acgcggcgta cggtatggcg catgtgtatt ttgaagtggg 1311660
cgtgatggtg atcggttttg tgtcactggg taaatttttg gaacaccgta ccaaaaaatc 1311720
cagcctcaac agcttgggct tgctgctcaa acttacacca acccaagtca acgtgcaacg 1311780
caacggcgaa tggaaacagc ttcccatcga ccaagtgcaa atcggcgacc ttatccgcgc 1311840
caaccacggc gaacgcattg ccgcagacgg catcattgaa agcggcagcg gttgggcgga 1311900
cgagagccat cttaccggcg aatccaatcc tgaagaaaaa aaggcgggcg gcaaagtgtt 1311960
ggcgggcgcg ttaatgaccg aaggcagtgt ggtgtaccgc gccacgcagc tcggcagcca 1312020
aacccagctc ggcgacatga tgaacgcgct ctctgaagca caaggcagta aagcaccgat 1312080
tgcgcgcgta gccgataaag cggctgcggt attcgtgcct gccgtcgtgg gcattgcgtt 1312140
gttgactttt attgttactt ggctgattaa gggcgattgg acggttgcgc tgatgcacgc 1312200
cgtcgccgtt ttggtgattg cctgcccgtg cgcgctgggt ctggcaaccc ctgccgcgat 1312260
tatggtcggt atgggcaaag cggttaaaca cggtatttgg tttaaagacg cggcagcaat 1312320
ggaggaagcc gcccacgtcg atgccgtcgt gttggacaaa accggtacgc tgaccgaagg 1312380
cagcccgcag gttgccgccg tttattgcgt tcccgacagc ggctttgacg aagacgcttt 1312440
gtaccgcatc gccgccgccg tcgaacaaaa cgccgcccat ccgctcgccc gtgccatcgt 1312500
ctccgccgcc caagcgcgcg gtttggacat tcccgccgca caaaacgcac aaaccgttgt 1312560
cggcgcaggc attaccgccg aagtggaagg cgtgggtttg gtgaaagcag gcaaagccga 1312620
atttgccgaa ctggccttgc cgaagttttt agacggcgtt tgggatattg caagcattgt 1312680
tgcggtctca gtcgataaca aacccatcgg cgcattcgca cttgccgacg cgttgaaagc 1312740
cgataccgcc gaagccatag gccgtctgaa aaaacacaat atcgatgtct atattatgag 1312800
cggcgacaac caaggcacgg tcgaatacgt cgccaaacaa ctgggcatcg cacacgcctt 1312860
cggcaacatg agtccgcgcg ataaagctgc cgaagtgcaa aaactcaaag ccgccggcaa 1312920
aaccgtggcg atggtcggcg acggcatcaa cgacgcgccc gcgcttgccg ccgctaacgt 1312980
cagcttcgcc atgaaaggcg gagcggacgt tgccgaacat accgcatccg ccacgctgat 1313040
gcagcattcg gtcaaccaac tcgccgatgc tctgctggtg tcgcaagcca ctttgaaaaa 1313100
catcaagcaa aacctgtttt tcgccttctt ctacaatatt ttgggcattc ctctcgccgc 1313160
gcttggcttt ttaaatcccg tcatcgctgg cgcggcaatg gcggcaagct cggtttccgt 1313220
gttgagcaat gccttgcgcc tgaaacgggt aaaaatcgat tagcagcatg taaccgccct 1313280
gcagccttgt ccgaacggat aaggctgtct ccagcgatat ggtaatatgc cgtctgaaac 1313340
cgtttttcaa gtaattgata tgaataaaga aacccgtttt ccggaacact tcgacatccc 1313400
acttttcctc aaaaacctgc ccaacctgcc aggcgtatac cgttttttca acgaaagcgg 1313460
caacgtctta tacgtcggca aagccgtcaa cctcaagcgg cgcgtgtccg gctatttcca 1313520
gaaaaacgac cattccccgc gcatcgcatt gatggtgaaa caggttcacc acatcgaaac 1313580
caccatcacc cgctccgaat ccgaagccct gattctcgaa aacaacttca tcaaagccct 1313640
gtcgcccaaa tacaatattc ttttccgcga tgacaaaagc tatccttatt tgatgctcag 1313700
cggccatcaa tatccgcaaa tggcgtatta ccgcggcacg ctgaaaaagc ctaatcaata 1313760
tttcggccca tatcccaaca gcaacgccgt gcgcgacagc attcaagtgt tgcaaaaagt 1313820
ctttatgctg cgtacctgcg aagacagtgt attcgagcat cgcgaccgtc cttgtctgct 1313880
ttaccaaatc aaacgctgca ccgcgccttg tgtaggccac atcagtgaag aagattatcg 1313940
tgacagcgtg cgtgaagccg cgactttcct taatggcaaa actgacgaat tgacgcgtac 1314000
cctgcaacac aaaatgcaaa ccgccgccgc taatctacaa ttcgaagaag ccgcacgtta 1314060
ccgcgatcaa atccaagcgc tcggcatcat gcaaagtaat cagtttatcg acagtaaaaa 1314120
tccgaacaat cccaacgata tcgatttgct tgcactggcg gtttcagacg gcctggtttg 1314180
cgtacactgg gtcagcatcc gcggcggacg gcacgtcggc gacaaaagct ttttccccga 1314240
caccaaaaac gatcccgagc caaacggaca agattacgcc gaagccttcg tcgcccaaca 1314300
ctatctgggc aaaagcaaac ccgacatcat catcagcaac tttcccgttc ccgatgcgct 1314360
aaaagaggct ttggaaggcg aacacggcaa gcagatgcaa tttgtcacca agaccatagg 1314420
cgaacgcaaa gtccggttga aaatggcgga acaaaacgcg caaatggcga ttgcacaacg 1314480
ccgcctgcaa caaagcagcc agcaacaccg cattgatgaa ctggcaaaaa tcctcggcat 1314540

ggattcagac ggcctcaacc gccttgaatg tttcgacatc agccacacac aaggcgaagc 1314600
cactattgcg tcctgcgttg tgtacgatga gcaaaacatc cagccttcgc aataccgccg 1314660
ctacaacatc acgaccgcca aacccggcga cgactacgcc gccatgcgcg aagtgttgac 1314720
gcgccgttac ggcaaaatgc aggaggccga agccaacggc gagaccgtca aatggccgga 1314780
tgccgtgttg attgacggcg gcaaagggca aatcggcgta gccgtatcgg tatgggaaga 1314840
actcgggctg cacatccctt tggtcggcat tgccaaaggc ccggagcgca aagccggtat 1314900
ggaggagctc atactgcctt ttaccggcga agtcttccgc ctgccgccca acagcccggc 1314960
cttgcatcta ttgcaaaccg tacgcgatga atcgcaccgt ttcgccatta ccggtcaccg 1315020
caaaaaacgc gacaaagccc gcgttacctc ctccttaagc gacatccccg gcgtaggcag 1315080
caaacgccgc caagccctgc tcacccgctt cggcggtctg cgcggcgtga ttgccgccag 1315140
ccgcgaggac ttggaaaaag tggaaggcat cagcaaggca ttggcggaaa cgatttacaa 1315200
tcatctgcat tagcatgctg tcaaagacaa aatccgtctg taaaaaatat gatacagcag 1315260
gtcggtatac cgatatatag tggattaaat ttaaaccagt acggcgttgc ctcgccttgc 1315320
cgtactattt gtactgtctg cggcttcgtc gccttgtcct gatttttgtt aatccactat 1315380
aaacctaact tcataacgaa taacgatgat tcgacaaaac ggaaaacgat ctgacatgaa 1315440
caatcccgac ttaccctatc ggcaggcctt agaatgcctg tctcaaaaac aatataactt 1315500
taccgaagtc cgccgactgc tgacagaagc gttctcggca ggtcatcccg ccgccgcatt 1315560
cgagttggca aaacacctga tggacgcgga cagcccctac caagaccgcg aacaaggtat 1315620
ggaaatgctc cgcatcgccg ctgaacaggg acatccctac gcgcgttaca atctggcata 1315680
tatccaagaa ttggaaggcg cacccccgga aaccctgata ccgctttaca gaccgttggc 1315740
agaagaagga ctgcccgaag cgcaagtccg cctgatgtac cttctgtacg cgtcccgaca 1315800
ttttgaagaa gccttggaat gggcaaaaac aagcgcaaaa aacaacaacc cccacgggca 1315860
atacctgctt gcccaatact gccggtacgg cacaccgccg gattttgaaa cggcgcacct 1315920
gctctaccga aaatcggcgg cacaaggctt gccggaggca cattggcagc tcgggctgca 1315980
atatcgtttc gggcaaggga cgaaagtcga cacggcacag gccgtcaatc atttgcgcgc 1316040
cgccgcacaa caaggataca ttcctgccta caccccactt gccgagctca tcctacctac 1316100
ggctcctgat gaagccgttc actggtttca acaggccgca caggaaaatg accccgatgc 1316160
ccatgccgca cttgccgaca tctacctgca aggcaagcat ctggaaagaa accacaaact 1316220
tgccctgcat catgccgaag cagccgccgc cgaacgccat cccgaaggtt tgcggatact 1316280
gggcgacatc tgccgctacg gtttgggcat agcccccgat acggaaaaag cccggcatta 1316340
ttatcggcag gcagccgaag ccggcagcct ttccgcctat cagaaactca tatccgacag 1316400
cgcgttaaac catcctgacc aatacggcgg cattaaagat tccgccatca ggcggcaaag 1316460
ggcagaacgg ctttatcaaa aagcccaagc cctgcattac ggattacaat gcgcgcccga 1316520
atacgcagcc gcgctcaaac tctacacaga agccgcagaa ctcggacaca gcaaagccca 1316580
aaccaatctg ggcagcatgt attacttcgg acagggtatg accgccgact acaatgaagc 1316640
acgcaaatgg tttgaaaaag ccgccgcgaa aaaagacagt atggcgttct acaacctcgc 1316700
ctgcatccat tacagcggac acggcgtcga gccggacaaa gaaaaagcct gccgctacct 1316760
gcaagaagcc ataaacaacg gatacgggca aaaaagcgtc ctgcaagaac tgctgcaaca 1316820
atggcaaaat gccgtctgaa cagcgttaca cctaccctgc cgaaacgaaa caggtataat 1316880
cgcccctttc ccttcccgcc gtccgaacag gcatttcaca ttcagacggc atcctgattg 1316940
cacaagcgta cgaaagcatt atgacagaca ccgccgagaa ccaaacacaa aacaactggc 1317000
aagccggaca cccccgcagc atccgcagct tcgtcctccg ccaaagccat atgaccgccg 1317060
cgcagcaacg cgccatcgat accttatggg acagcttcgg catcgactac caagcaacac 1317120
cggccgatct tgatgcccgt ttcggaagca gccgacccaa aatcctcgaa ataggcttcg 1317180
gtatggggac ggcaaccgca gaaatcgccc gccgcctgcc cgaaaccgac ttcctcgcca 1317240
tcgacgtaca cggtcccggc gtaggcaacc tgctcaaact catagacgaa aaccatttag 1317300
aaaacatccg cgtgatgcgg cacgatgccg tagaagttgt cgaaaatatg ctgcaagacg 1317360
gctcgctcga cggcatccac atattcttcc ccgacccgtg gcacaaaaaa cgccaccaca 1317420
aacgccgtct gatacaagcc cccttcatcg ccaaactact gcccaaactc aaaaccggcg 1317480
gctatatcca cctggcgaca gactgggaag aatatgcaca gcagatgctt gaagtcctca 1317540
gtagcttcga cagcctgcaa aatacggcgg cagactacgc ccccaccccg gactaccgcc 1317600
ccgaaaccaa attcgaagcg cgcggcaaac gcctcggaca cggcgtttgg gacttggtat 1317660
tcaaacggat cggataacaa accactgttt gaaaatgccg tctgaaacat gtttgcttac 1317720
agacggcatt ttttcaagat aaagcagcaa gtgatgtttc gatataaagt ttaaaacaat 1317780
agtttgaacg gcaaaacgcg tgtgtaccgc acgcatcctt ataggtttta tgcacatcgg 1317840
ttttaaagtt tgtgccgccc gcaggtagta tgtgatagct acgcacgcgg ttggtgtgat 1317900
gtaggctacg gcttgctggt tacaaccgta aaaaagtaag tgccgccatt gcggtaaaaa 1317960
cgaagggatt tcatagtgtt atgctcgtaa tgattttgta gattggattc tcgaatccga 1318020
ccttttgggc attgctgcaa tggattgcaa cgacgggaat gttgaaggtt ttgtcggata 1318080
caagtatccg acctacgctt gttgctatat atcttttgat ttaatgacta aatatgacaa 1318140
agttaaagtg cagattacag caagcatgat atgcttcttt aggcttttat cattccatga 1318200

tatagatatt tcttcctttt cattttcttt ataaaatttt aaacctatat caccattttt 1318260
ccattcctgg tggtttacta tgattttatt tttaaaagaa tctcttaaac tttcatgtaa 1318320
agagttaaat tttcttgatt tacttccctt agtacatggt gagcaattgt atttctaatt 1318380
ttatttaatc tctcccctat atcatatact tcgctaaata agccaagatt acgcgcaatt 1318440
tttagttttg tgcgaaatcc aatttgtgta tcattgaaaa aatcttcttt attacatttt 1318500
gcatatatcc atgcctctaa aattctttca aaaaataaat gtgttcgtaa gattgaacct 1318560
atttcatcct gtgtttcaat agcttctttc acgatatcaa aaatcttata atcatcaaaa 1318620
tttgaatttt taataaataa ttctaaatta aaatctaatt gagacataat aagtgcccat 1318680
ttcaaaaata aatctatatt ctagttaata taatagttat tctaatatct aaattaaata 1318740
ataaactact atttttatat ccacgacaaa gtctaagtct cactccgccc caaacaacaa 1318800
attctcttta atatccctaa tcctatcccg caacacagcc gcctcttcaa actgcaaatc 1318860
cctagccgcc tgctgcatgg ctttttcgag tttggcgatt tctttaatcg catcttcttc 1318920
gttgtgaatc tcgcccactt taaccttgtt tttacctttc agacggcctt tactgccgtc 1318980
ttcttcgtgg tacacgccgt cgatgatgtc tttgacctgt tttttaatct gctgcggcac 1319040
gatgccctgt tcttcgttga atttaatctg tttttcacgg cggcgttcgg tttcgtcgat 1319100
agcggctttc atggagtcgg taattttgtc ggcgtacagg atggcgacgc cgttcacgtt 1319160
gcgcgcggcg cggcctatgg tttgaatcag gctgcggtgg gagcgcagga agccttcttt 1319220
atctgcgtcg aggatggcga cgagagagac ttcggggatg tcgaggcctt cgcgtaagag 1319280
gttgatgccg acgagtacgt caaacaggcc gagccgtaaa tctctaatga tttcaacgcg 1319340
ctcgacggtg tcgatgtcgc tgtgcaggta gcgcactttg ataccgagtt cgctgtaata 1319400
gtcggtgagt tgctccgcca tgcgtttggt gagggtagta acgagtacgc gttcgccttt 1319460
ttcaatgcgg tcgttgattt cgctcattaa gtcgtcgact tgggtggcaa cggggcggat 1319520
gatgatttgg ggatcaacca gccctgtggg gcggacgact tgttcgacca cttgtccggc 1319580
gtgttcttct tcgtatttgg cgggggtagc ggaaacgaag atggtttgcg gcatgacttt 1319640
ttcaaattcg tggaatttga gcgggcggtt gtcgcgggct gaaggcaggc ggaagccgta 1319700
gtcgacgagg ttttgcttgc gcgatgcgtc gcctttgtac atgccgccga tttgggttac 1319760
ggtaacgtgg ctttcgtcga tgaacatgat ggcgttgtcg ggcaggtagt ccatcagcgt 1319820
aggcggcggt tcgccttctt ttttgccgga aaagtggcgg gagtagtttt cgattccttt 1319880
gcagaagccc atttcgtaga gcatttcgag gtcgaaacgg gtgcgctgtt cgatgcgttg 1319940
ttgttcgacg gggcgttgtt cgcgggcgaa aaattcgatg cgttcgcgta attcttcttt 1320000
gatggactcg caggcgcgca agacggtgtc gcgcggggta acgtagtggc tggacgggaa 1320060
gacggtgtag cggccgacgc gctggataag gctgcctgaa agcgggtcga acatatcgag 1320120
gcggtcgatt tcgtcatcaa acaggctgat gcgtaaggcg ttttcggagc tttcggcggg 1320180
gtacacgtca atcacgtcgc cgcgcacgcg gaagctgccg cgtttgaagt ccaaatcgcc 1320240
gcgttcgtat tgcatggaaa cgagcgtggc gatgatgtcg cgctgctcga tggtatcgcc 1320300
ttctttgacg gacaacacca tttgttgata ctcggtcggg tcgccgatac cgtaaatggc 1320360
ggacacggtg gcgacgataa tcacgtcgtt gcgcgtcatt aggtttttgg tggcggaaag 1320420
gcgcatctgc tcgatgtgtt cgttgatcgc gctgtctttt tcgatgaaca aatcgcggct 1320480
gggcacatag gcttcgggct ggtaatagtc gtagtaggag acgaaatatt ccactgcgtt 1320540
ttcggggaaa aattcgcgca tttcggcgta aagctgggcg gcaagggttt tgttgtgcgc 1320600
catgatgatg gcggggcggc cgctttgggc gatgacgttc gccatggtgt aggttttgcc 1320660
cgaaccggtt acgccgagca gggtttgata ggcaaggccg tctgaaagcc cttcgagcag 1320720
gcctgcaatg gcggtgggct ggtcgcctgc gggcgggaag ggttggtgga gtttgaaggg 1320780
ggaatttggg tattggataa cttccataat cttgcctgtg atgcgtttgc ggacaaagcg 1320840
tgcagtaggg atgggtcgga aacgtctttc agacggcata aggcggtgaa atcctgaatg 1320900
tatgccgtct gaaacccaat cgctacccaa gtatagtgga ttaacaaaaa ccagtacggc 1320960
gttgcctcgc cttgccgtac tatttgtact gtctgcggct tcgtcgcctt gtcctgattt 1321020
ttgttaatcc actataaatg ccgcacggtt caaattccgg taaaaaatcg ctcataacct 1321080
gtcctttcaa acataatatg ccgtctgaaa tcctttcaga cggcatcgtc aaaacctact 1321140
tcttatcttt tttatctttc ttatctttat ttgaaaccgg ctttttcgcc gccagcccca 1321200
aagacttctg ccactgctcg ggcgacttga ctaaatccaa agctttccgc aactggtcgt 1321260
ctttggcagg gttgggaatc cgccttgaag acaaatcctc gtcctttttc tttttacctt 1321320
tttcttttac agcgggctta tccgcatctt tttcaagcgg cacggcaagg gtttcaccgt 1321380
tcacatcctc gccgcccaag ggattgccga tgtgtccgac caaatccgcc tcgcggcttt 1321440
caaaaatgcg ttccttatct tttacttcga catcgggaac aatcccctgc gcctgaatag 1321500
aacggtcgtt cggcgtataa tacagtgccg ttgtcagctt gaccgcgctg ccgttggaca 1321560
aaggaatcaa agtctgaacc gaacctttgc cgaagctctg cgtaccgacg atgaccgcgc 1321620
gtttatgatc ctgcaatgca cctgcgacaa tctccgacgc ggaagccgaa ccggaattga 1321680
ccaataccgt catcggtatg gttttcaact cggcaggaat gcccgccaac gaatcgccgc 1321740
ccatcccgta cacataatct tcaggaatgg ctttcagtac catgcggtct ttgccgtcgc 1321800
gtcccttggt gctgacgacg actgcttcag acggcagaaa tgccgccgac acgccgaccg 1321860

cgccagtcaa aagcccgccg gggtcgtcgc gcaaatccaa caccagcccc ttgagcggtt 1321920
ttcctttatt ttcctttacc agctcttttg cggcggtatt gacgctttcg accgtccgct 1321980
cttggaactg cgacacgcgg atatagccgt aatcgggttc gatcaggtga tggcggacgc 1322040
ttttcacttt aataatggca cgggtcaggt tgacgactat cggcttgtcg gcatttttgc 1322100
gcgacagcgt caaagtaatc ttcgtacccg gcttgccccg cattttcttc accgcttcgc 1322160
tgaccgtcat gccgcgtgtc gaaacattat cgattttcac aatgaaatcg ccgcttttca 1322220
cccccgcccg ttccgcaggc gtgtcctcaa tcggcgaaac cactttgaca aatccgtctt 1322280
cctgcccgat ttccatcccc aagccgccaa attcgccgct ggtggactcc tttatctcgg 1322340
cataaccttt tttatccata tattcggaat gcggatccaa accggccacc atacccttca 1322400
tcgcaccttc aaacaaatcg gcatcgggtt tgtcctgata gtagtttgcc ttgatttgac 1322460
cgtaaacctc cgccattgtg cggatggatt gcaccggcag gacttcgtta tcccgcctgt 1322520
ccttctcggc ggcaaaaccc tgcaccgcca gactgacggc cacgccgctg attgcaccca 1322580
aagtataaag tgcgattttc ttaaaaacag gtttcgacat tcttctttaa ctttctctct 1322640
tgatttccaa aaaccggaaa atacaggtac ggcaaacggc aaacttcacg gaacagcgca 1322700
ccatatcggc acgatttgca taaagcctac cgtttcggca atccgatcaa cgtatccagc 1322760
tcgaagggtt caatacctga ccttgataac gtatttgcag gtaaagcccc tcttccccgt 1322820
ccggcagcga cccgctcgag ccgattttgc ttcctgccgc gaccatataa cccttgccga 1322880
cggaaatttc gctcaaaccg gcatagatgc tgatgtagtt ctcgccgtga tcgaccacga 1322940
ccactttgcc gtagccgtcc aactcgtccg catagcttac cgttcccggc gcaatgcttt 1323000
caaccgttgc cggtgcagtg gaatagaaca cgcctttcca aatatcgccg ccgctccggt 1323060
tctgcccgaa aagtccggtc ggcacaccgt caaccggttt tttcaaacgt ccttgcatgc 1323120
ggctgaaacc gtcggcactg ccgataccca taaccgaagg cgcttggatg ttcctgtctt 1323180
cggcggtcag gttggacatt tccgcacgtc gtgcttcagc cttctgctgc gccgcttctt 1323240
ttctggcttt ttcggctgcc gccagtctgg cttcagccaa ttttcttttt gcttccgcat 1323300
cctgaatgcg gtgttcggcc tttttcttct ccaaattgct caagagcttg ttcagctgct 1323360
gctcgttccc tttctgttcc agcagttttc gggcatcttt ggcgattttg gcattctgtc 1323420
tgcggctttc cgtctgttcc gccgcatcgg ttacaccctg ttttttcagc agagattgca 1323480
cgtttgcctg aattttcttc aaacgggcaa gctcattgtt gattttctgc tcttgtaccg 1323540
ccaaagcctt ctgctgtttt tccaaatcct tgacaacttc ccgattggag gcgtttacat 1323600
aacgcgtata acgcaaaaag cggtttttct gacccggttc ggcgtttttc aggaacaggg 1323660
caaccgcatt cggctggctg tttttatagt tccccgatac gaaacgggaa atctgcgctt 1323720
tcgtagcggc gacttccgtt ttcaaacggt tcagctcggt attgagtttt tggaacttgt 1323780
cccaagcctc gcgctgtttg cggttgacgg aagcaaggtt gccgcgcgcc tgacggatac 1323840
gctcttggcg gatacgctct tcctgaagct gtttgaggga attgctgaca tgaagcagca 1323900
ttccttcgct ttgtttgagc agggcttttt tgttttcgac atcattggtg gcagcggcaa 1323960
cggcggcttt caattcgtcg gaatcggttt tggcattttt ctcttccctg tattttttgt 1324020
cctgtttcac tgctttgccg ttcttgtcgg aacggacttt tttatttgca gaaacagtgt 1324080
ctttttccgc cttgccgccc ttgcgcggat tgccctgtcc ttttgcctct tttttgcctt 1324140
gtttgttttc aggctgtgtt tttgcgtttt tttctttgga tccggacacg ggcttgccat 1324200
gtgccttttt gtgttccgcc ttcgatttct tatccccttc gcgtcctttg cgcgcagact 1324260
gcctggatgt cgcctccttc tctgcttctt tgcggttttt ggcggttttt ttggactctt 1324320
tgccctcttt tgccgcctct ttgccgcctg ttttttatc tttcactgcg ccatttttgc 1324380
ctttttcttt tttgccttcc gccgcttcgg gctgttcttt tttgttcttc gtctgttttt 1324440
tcacttcggc ggaacggttg tgtgccgcgt cgtgggcggc aacggcgggc gtggaaaaaa 1324500
cgagcatcag ggcaagcaga aggggtttgt agcgcatggt tcgaccttcg gaaaaagttg 1324560
gataatactg aaggctgcac gaaagcagcc ggacgtttgg attatactgt cagttatgcc 1324620
gtctgaaaat gccgtttgcc caatcttgcg ccttctttgc gcggatactt gcaatcggct 1324680
caaacagcct tatattgtgc gtcatatttt caatgccgca acggatattg tgttccgaca 1324740
cacagggtag cacattaagc cgcataccgt atgttgcccg attttgggaa cgtgcgcccc 1324800
tccaaacaaa gcaagccctg ccgctttcac ggaaaacggg gattcaaccg ataaggaaat 1324860
tttgatgaac agactgctac tgctgtctgc cgccgtcctg ctgactgcct gcggcagcgg 1324920
cgaaaccgat aaaatcggac gggcaagtac cgttttcaac atactgggca aaaacgaccg 1324980
tatcgaagtg gaaggattcg acgatcccga cgttcaaggg gttgcctgtt atatttcgta 1325040
tgcaaaaaaa ggcggcttga aggaaatggt caatttggaa gaggacgcgt ccgacgcatc 1325100
ggtttcgtgc gttcagacgg catcttcgat ttcttttgac gaaaccgccg tgcgcaaacc 1325160
gaaagaagtt ttcaaacacg gtgcgagctt cgcgttcaag agccggcaga ttgtccgtta 1325220
ttacgacccc aaacgcaaaa ccttcgccta tttggtgtac agcgataaaa tcatccaagg 1325280
ctcgccgaaa aattccttaa gcgcggtttc ctgtttcggc ggcggcatac cgcaaaccga 1325340
tggggtgcaa gccgatactt ccggcaacct gcttgccggc gcctgcatga tttccaaccc 1325400
gatagaaaat ctcgacaaac gctgatatga acctctccaa ccactttctc atcgccatgc 1325460
ccgatatgga agacgcgttt ttttcacaat cggtcgtcta tatctgcaaa cacgatgaag 1325520

acggcgcact cggcatcgcc atcaacaaac cctctccgat tacgatggac atgatttttt 1325580
ccgccaccgg caaaaacatc cccatgcgga tgcagcacga cagcgtgatg atgggcggtc 1325640
cggtgcaggt cgagcgcggt tatgtcgtgc ataccccgat cggcaactgg caaagcagta 1325700
tcggcgtttc agacaatatc gcgctaactt cttcccgaga cgtgattgaa aatatttcac 1325760
gcgaaggtgc ggttgacaaa gccttgatca gcataggcta ttcaagctgg agcaaagggc 1325820
agctcgaacg cgaacttgcc gacaatgcgt ggctgactgt tcccgccgac gaacacatcc 1325880
tgttcgacat cccctacgaa caccgttacg ccgccgcatt cgccaaactc ggcatcgacc 1325940
cgctcgccct gttttcagga gccggccatg cataaaattc caaaaggaac ggcactggca 1326000
ttcgacttcg gcgaagcgcg tatcggcgtg gcacaaggag acgcggaatt agggctatcc 1326060
catcctttga gcaccgttac cggcggcagc aacgatgaaa agttcgcggc aatcgccaag 1326120
ctggttcaag aatggcagcc gcgttatttt gtcgtcggac tgcccgtgca taccgacggc 1326180
acgaaacatg aaatgacgca cctgtcgcgc aagttcggac gcaggctgaa cggcaggttc 1326240
aatctccccg tctattgggt tgacgaacgg ctgtcgtccg tctatgccga aagcctgctt 1326300
tcggaagcac aggtcttcgg caaaaaaacgc aaatcggtgc tcgaccaagt ggcggcgcaa 1326360
gccatcttgc acggtttttt cgagggcggt ccggcggaat gtttcaacgg gcgtgagggt 1326420
taagcggcgc ggttaacacc ctaccgtgaa agaggcgcgc accaagccgt ccagctccaa 1326480
tgccaaattg tcccccgcac cgattgcgcc cacgccggag ggcgttccgg taaacaccaa 1326540
atcccctttc cccaaaccgt aatctgccgc cagtttgtgt aaaatttccc gaatcgggta 1326600
aatcatcaaa ccggtatccc cgcgctgttt caatacgccg ttttgtttta atgaaaacaa 1326660
caccttctcg ggattgccga ttctgcctgc cgccgcaaaa tccgacacgc acgcggaatg 1326720
cctgaaccct tttgccttca gccagggcag cccttttttcc ttcagacggc attggatatc 1326780
ccgtgccgta aggtccagcc ctacaccata tcctgcgaca catcccaaaa tatctttacc 1326840
ctcgcccgtg ccgtctgaat ccttaccgac cagcagcacg agttcgcact caaactgcac 1326900
atccctacta aactcgggca gcaagattgt accgccgctg ttcaaaatgc tgcctgacgg 1326960
cttcataaac accacaggtt cggaaggtat ttcgtttttt aactcttcga tatgtgcggc 1327020
atagttcctg ccgatacaga aaatattgcc gacctcgact gcctctcctt ctaaaaatac 1327080
tgaagccact tcactttccc cctaagtaaa aatgccgtct gaaattattt tcagacggca 1327140
ttcgaccaag cttacgcatt taatgaagct gttacacgtg caacaatttc tccgattgca 1327200
actgcctgcg cttcgttgtc gcggcgttcg gcgtattcga cattgccttc tttcaaggcg 1327260
cggtcgccga tgacgatgcg gtgcggaata cccaacagct cggaatcgtt cagcaacacg 1327320
cctgcgcgtt cgtcgcggtc gtcgaggagg acgtctgcgc ctgccgccag caattcggca 1327380
tagattttgt cggcggcttc gcgtacggtg tctgattttt tgtagttcat cggcacgata 1327440
acgacttcaa acggcgccat tgctttggtc cagatgatgc ctttttcgtc gttattctgc 1327500
tcgatggcgg cggcaacgac gcgggtgatg ccgatgccgt agcagcccat ttccataatt 1327560
tgcgatttgc cgttgttgtc aaggaagctt acgttcatgg cttgggtgta tttgtcgcgc 1327620
aattggaaaa cgtgtccgac ttcaatgccg cgcgccagtt tcagacggcc ttgcccgtcg 1327680
gggctttcgt cgccctcgac gacgttgcgc aaatcgacaa actcaggttc ggcagcgtcg 1327740
cggccgaaat tgaagccggt atagtggtag tcgtcttcgt ttgcgccgat gacccagtcc 1327800
gcgccttttt cggtagcgaa atcggcatag actttgcctg caaaaccgac agggccgaga 1327860
gagccgccgt ttgcgccgaa ctgttcgaca atcgcggcag ggcttgccat cgtcagcggc 1327920
gatttcacgc ccgcgagttt ctcggctttg atgtcgttaa attcatggtc gccgcgtaac 1327980
agcagcagga taagttcgcc ttcgttttcg ccttcaacca cgatggattt cagtgttttt 1328040
tcaatcggaa tactgaggaa atcaaccaat gaatcaatgg ttttgacgtt tggcgtgtgt 1328100
actttgacga gttctgcctg agcggctgca cgttcgcctt tgagcggcaa ggtcggcgct 1328160
aactcgatat tggcggcgta atcggaagtg tcgctgtatg caatcacatc ttcgccgctt 1328220
tccgccaaca cttgaaactc gtgcgaaccg gtaccgccga tgctgccggt atccgcagca 1328280
acgggtcgga acgccaagcc tagtcgggta aagatgcggc aataagcatc atacatatct 1328340
tgataggtcg tctggagcga ggcatagtcg gcgtggaagg aataagcgtc tttcatcaca 1328400
aactcgcgcg cgcgcatcac gccgaagcgc gggcgcactt cgtcgcggaa tttggtttgg 1328460
atgtggtaaa agtttttcgg cagctgtttg tagctgttga tttctttgcg cacgatgtcg 1328520
gcgatgactt cctcgcaggt cgggcccatg cagaaatcgc ggtcgtggcg gtctttcagg 1328580
cgcagcagtt ctttaccgta aaactcccag cggccggatt cctgccacag ctcggcaggc 1328640
tgcaccaccg gcatcagcaa ctccacgctg cccgcgcgcg ccatttcctc gcgcacgacg 1328700
ttttcgactt tgcgtaacac gcgcagcccc atcggcatcc aagtataaag acccgatgcg 1328760
ttggccttaa tcaggccggc gcgaatcatc agcttgtggc tggcaagcgc ggcttcggca 1328820
ggggcttctt ttaaagtaga gataaagaat tggctggctt tcataaaagt atttttccaa 1328880
acaggcaaat tcaaaagtaa atcgggtgca gattgtaacg cgaaaaaagc aggttttgca 1328940
ccaacctcca aaattcaccc cctgccccaa gcgcgggaca aatcccataa cagacggcaa 1329000
aaacatgacc agaaacatca tattgaacat aagcacatga tttttataga tttaaatgtg 1329060
cctatttttt aatcaaaata agcgtacatt tgttgcgtaa gactttttta acacaagccg 1329120
tggcttatca acacggttat ccacaaagct tgtgtataga ttttctacaa taggaaaatt 1329180

gccgacagag acataatgat tcgatatacc acaattccga aaaaatatcg ccaaaatcaa 1329240
acagaatatt tcgaaatcaa aaagacttga ccttaccaaa cgccaacttc agtataaaac 1329300
ctgcttttac aggcatggtt atttgccagc agacccgatt gctgatagga tttcgtgtgg 1329360
agcagatcga acattttttt caagttttcc cttgtttcca aaacttttat aattttttga 1329420
aaacattaaa cttaaattat ttttttcggt ttgatttaga aattttcgtt tttgcttatt 1329480
atttttcaca aacgaaaata aaggggttgg ctacaccctc cctgccgatt aaacactcaa 1329540
cataaaggat agatactatg tccacccaat tacacgatgt tgaccctatc gaaacccaag 1329600
agtggctgga cgcgttaagc tccgtcctcg aatatgaagg cggcgaacgc gcgcaatacc 1329660
tcttggaaaa cctggtcaaa tactgccgcg acaagggcgt acgtatgcca cacggcacga 1329720
ccaccccgta tttgaatacc gtttcggttg aaaacgaaaa aggcattccg ggcgaccaaa 1329780
acatcgaaca ccgcatccgc gcattcgtgc gctggaacgc cgccgccatc gtattgcgcg 1329840
ccggcaagaa agatttggaa ctgggtgggc acatcgcatc tttccaatct gccgccacca 1329900
tgtacgaagt cggtttcaac cacttttgga aagccaaagg cgaaggcgaa gaaggcgatt 1329960
tggtcttctt ccaaggtcac gtcgccccgg gcatctatgc acgcgcattc gtcgagggcc 1330020
gtctgaccga agaccagctg aacaacttcc gccaagaagt ggacggacac ggtctgcctt 1330080
cctatccgca cccccacctc ttgcccgact tttggcagtt cccgaccgta tccatgggct 1330140
tggggcccat catggcgatt tatcaggcgc gtttcctgaa atacttggaa tcgcgtggtt 1330200
tggcaaaaac caaaggccgt aaagtatggt gtttctgcgg cgacggcgaa atggacgaac 1330260
ccgaatctca aggtgcaatc gcactggctg cacgcgaagg cttggacaac ctgattttcg 1330320
tcatcaactg caatctgcaa cgcttggacg gtccggtacg cggcaacggc aaaatcatcc 1330380
aagaattgga aggcaacttt gccggcgccg gctggaatgt cgtcaaagtc atttggggcc 1330440
gccgctggga ccgcctcttg gcgaaagaca aagacggtat cctgcgccaa cgtatggaag 1330500
aatgtttgga cggcgactac caaacttaca aatccaaaga cggcgcgtat gtgcgcgaac 1330560
acttcttcaa tacgcccgaa ctgaaagcat tggttgccga tatgaccgat gagcaactct 1330620
gggcattgaa ccgcggcggc cacgacccgc aaaaagtgta caacgcctac gaccgcgcag 1330680
cgaaccatgc cgacggcaaa cctaccgtca tcttggcgaa aaccattaaa ggttacggta 1330740
tgggcgcatc cggcgaaggt cagaacgttg cccaccaagc caaaaaaatg gacaaagcgt 1330800
ccctgaaaca attccgcgac cgctttgaca ttccggttac cgacgaacaa atcgaaagcg 1330860
gcgatctgcc ttacctgact tttgcccccg atacggaaga atacaaatac ctgcacgcac 1330920
gccgcgatgc tttgggcggc tacctgccgc aacgcaaacc gacgcaggaa gtattggaag 1330980
tgcccgagct gtcagcattc gacgcacaac tcaaatccag cggtgaacgc gagttctcga 1331040
ccacgatggc attcgtccgc atcctgtcca ctttactgaa agacaaaaaa atcggcaaac 1331100
gcgtcgtacc tatcgttccc gacgaaagcc gtactttcgg catggaaggt atgttccgcc 1331160
aatacggtat ttggaatccg aaaggtcagc aatatacccc tcaagacaaa gaccaactga 1331220
tgttctataa agaatccgtt gacggtcaaa tcttgcaaga aggtattaac gaaccgggcg 1331280
cgatggccga ctggattgcg gctgcaacca gctacgccaa cagcaacttc gccatgattc 1331340
cgttctacat ttactattct atgttcggtt tccaacgtat cggcgacttg gcttgggcgg 1331400
cgggcgatat gcacgcgcgc ggcttcctgc tgggcggtac tgccggccgt acgacgctga 1331460
acggcgaagg cctgcaacac gaagacggcc acagccacat ccaggccgac ctgattccga 1331520
actgcgtatc ttatgacccg actttccaat acgaagtcgc cgtcatcgta caagacggtc 1331580
tgcgccgtat gtatgccaat aatgaagacg tgttctacta catcaccctg atgaacgaga 1331640
actacaccca tccggatatg cccgaaggtg cggaacaaga catcttgaaa ggtatgtacc 1331700
tgctgaaagc cggcggcaaa ggcgataaga aagttcaatt gatgggctcc ggtaccatcc 1331760
tgcaagaagt cattgccggt gccgagctgc tgaaagccga cttcggcgta gaagcagaca 1331820
tctggtcttg cccgtccttc aacctgctgc accgcgacgc tgtcgaggta gaacgcttca 1331880
accgcctgca tccgctggaa gccgaaaaag tacctttcgt tacttcccaa ctgcaaggtc 1331940
atgacggtcc ggttattgcc gctaccgact atatccgcag ctatgctgac cgtatccgcg 1332000
cctacatccc gaacgactac cacgtcttgg gcactgacgg tttcggccgt tccgacagtc 1332060
gcgccaacct gcgccgcttc tttgaagtgg atcgctacaa cgttgccgtg gccgcattgg 1332120
ccgcattggc ggaacaaggc aaagtcagca aagaaaccgt tcaacaagcc attgagaaat 1332180
acggcatcaa agccgattca gctcctagct ggaaacgctg attgatgttt cagacggcct 1332240
gtttgcccca ttccgacatc aggccgtctg aaaaccgaat gcccgaatgg tttgagcaga 1332300
caaaccgtac cgatgccgcc tgaagcagct ttcagacggc atccaatgaa aaagattaaa 1332360
ggaactcaaa tgagtatcgt agaaatcaaa gtccccgata tcggcggtca cgaaaacgtc 1332420
gacatcatcg ccgtagaagt taaagcgggc gacaccatcg ccgttgacga caccctgatt 1332480
acactggaaa ccgacaaagc cacgatggat gtgcctgccg atgcggccgg tgtcgtgaaa 1332540
gaagtaaaag tcaaagtcgg cgacaaaatc tccgaaggcg gcgtaattct gaccgttgaa 1332600
accggtgccg ccgccgccga agccgccccg gctgctgccg aagcacaacc tgcacctgct 1332660
gccgcacccg ctgccgcagg cggtgcaacc gttcaagtag ccgttcccga tatcggcggc 1332720
cataccgatg tggatgtaat cgccgttgaa atcaaagtgg gcgacaccgt tgccgaagac 1332780
gacacgctga ttactttgga aaccgataaa gcgacaatgg acgtaccttg taccgctgcc 1332840

ggtgtcgtta aagccgtatt cttaaaagtc ggcgacaaag tatccgaagg ctctgccatt 1332900
atcgaagtag aaaccgtcgg ctctgccgca gcagcccctg ctcaagccgc tcaagctgcc 1332960
gcaccggctg ccgctccgcc tccgactgct gccgccgcac ccgccgccgc gcctgcacct 1333020
tctgcacctg ccgctgccaa aatcgacgag gccgctttcg ccaaagcaca cgccggtcct 1333080
tccgcacgca aactggcgcg cgaattgggc gtggatttgg gccaagtcaa aggcaccggc 1333140
ttgaaaggcc gtatcatggg cgacgacatc aaagcctttg tgaaatccgt gatgcagggc 1333200
ggcgcggcaa aacctgccgc agccagcgca tctttgggcg gcggtctgga cttactgccg 1333260
tggcctaaag tggacttctc caaattcggc aatgtcgaag ttaaagaatt gtcccgcatt 1333320
aagaaaattt ccggtcaaaa cctgtcccgc aactgggttg tgattcccca cgttaccgta 1333380
cacgaagaag cggacatgac cgagctggaa gaattccgca aacagctgaa caaagaatgg 1333440
gaacgcgaag gcgtgaaact gtccccgttg gcgttcatca tcaaagcctc tgtttccgcg 1333500
ttgaaagcat tccccgaatt caacgcctca ctggacggcg acaacctggt gctgaaaaac 1333560
tacttcaaca tcggtttcgc agccgatacg ccgaacggct tggttgttcc cgtcatcaaa 1333620
gacgtggatc aaaaaggctt gaaacaaatc agccaagaat tgaccgaatt gtccaaaaaa 1333680
gcccgtgaag gcaagctcaa accgcaagaa atgcaaggcg cgtgctttac catttccagc 1333740
ttaggcggca tcggcggcac aggcttcacg ccaattgtga acgctcccga agtcgccatc 1333800
ttgggcgtgt gcaaatccca aatcaaacct gtttggaacg gcaaagagtt tgccccgcgc 1333860
ctgatgtgcc cgttgagcct gtccttcgac caccgtgtca tcgacggtgc ggccggtatg 1333920
cgcttcaccg tattcttggc gaagctgttg aaagacttcc gccgcattac cttataaaat 1333980
aaaacatccc tctcaagcag tctgataatg tttggattgc ttgagattga tgagtaatgg 1334040
tgttaaattc aacctttaaa ttaataactt atgggaaatt tcttatatag aggcattagt 1334100
tgccaacaag atgagcaaaa taatggacag ttaaaaccta aaggtaataa agctgaagtt 1334160
gcaattcgtt atgatggtaa gtttaaatat gatggtaaag ctacacatgg tccaagtgtg 1334220
aagaatgcag tttacgccca tcaaattgaa acaggtctat atgacggatg ttatatatct 1334280
acgacaacag acaaggaaat tgccaagaaa tttgcaacaa gttccggcat cgaaaatggc 1334340
tatatatatg ttttaaatag ggatttgttt ggtcaatatt ctatttttga atatgaggtt 1334400
gaacatccag aaaacccaaa tgagaaggaa gtaacaatca gagctgaaga ttgtggctgt 1334460
attcctgaag aagtgattat tgctaaagag ttgatagaaa ttaactaagt tgaaaggtca 1334520
atataatggc tttagttgaa ttgaaagtgc ccgacattgg cggacacgaa aatgtagata 1334580
ttatcgcggt tgaagtaaac gtgggcgaca ctattgctgt ggacgatacc ctgattactt 1334640
tggaaaccga taaagcgact atggacgtac ctgctgaagt tgcaggcgta gtcaaagaag 1334700
ttaaagttaa agtcggcgac aaaatctctg aaggtggttt gattgtcgtc gttgaagctg 1334760
aaggcacggc agccgctcct aaagccgaag cggctgccgc cccggcgcaa gaagccccta 1334820
aagctgccgc tcctgctccg caagccgcgc aattcggcgg ttctgccgat gccgagtacg 1334880
acgtggtcgt attgggtggc ggtcccggcg gttactccgc tgcatttgcc gctgccgatg 1334940
aaggcttgaa agtcgccatc gtcgaacgtt acaaaacttt gggcggcgtt tgcctgaacg 1335000
tcggctgtat cccttccaaa gccttgttgc acaatgccgc cgttatcgac gaagtgcgcc 1335060
acttggctgc caacggtatc aaataccccg agccggaact cgacatcgat atgcttcgcg 1335120
cctacaaaga cggcgtagtt tcccgcctca cgggcggttt ggcaggtatg gcgaaaagcc 1335180
gtaaagtgga cgttatccaa ggcgacgggc aattcttaga tccgcaccac ttggaagtgt 1335240
cgctgactgc cggcgacgcg tacgaacagg cagcccctac cggcgagaaa aaaatcgttg 1335300
ccttcaaaaa ctgtatcatt gcagcaggca gccgcgtaac caaactgcct ttcattcctg 1335360
aagatccgcg catcatcgat tccagcggcg cattggctct gaaagaagta ccgggcaaac 1335420
tgctgattat cggcggcggc attatcggcc tcgagatggg tacggtttac agcacgctgg 1335480
gttcgcgttt ggatgtggtt gaaatgatgg acggcctgat gcaaggcgca gaccgcgatt 1335540
tggtaaaagt atggcaaaaa caaaacgaat accgttttga caacattatg gtcaacacca 1335600
aaaccgttgc agttgagccg aaagaagacg gcgtttacgt tacctttgaa ggcgcgaacg 1335660
cgcctaaaga gccgcaacgc tacgatgccg tattggttgc cgccggccgc gcgcccaacg 1335720
gcaaactcat cagcgcggaa aaagcaggcg ttgccgtaac cgatcgcggc ttcatcgaag 1335780
tggacaaaca aatgcgtacc aatgtgccgc acatctacgc catcggcgac atcgtcggtc 1335840
agccgatgtt ggcgcacaaa gccgttcacg aaggccacgt tgccgccgaa aactgcgccg 1335900
gccacaaagc ctacttcgac gcgcgcgtga ttccgggcgt tgcctacact tcccccgaag 1335960
tggcgtgggt gggcgaaacc gaactgtccg ccaaagcctc cggccgcaaa atcaccaaag 1336020
ccaacttccc gtgggcggct tccggccgtg cgattgccaa cggttgcgac aacggcttta 1336080
ccaagctgat ttttgatgcc gaaaccggcc gcatcatcgg cggcggcatt gtcggtccga 1336140
acggtggcga tatgatcggc gaagtctgcc ttgccatcga aatgggctgc gacgcggcag 1336200
acatcggcaa aaccatccac ccgcacccga ccttgggcga atccatcggt atggcggcgg 1336260
aagtggcatt gggtacttgt accgacctgc ctccgcaaaa gaaaaaataa atccgactga 1336320
ataaacagcc gataaggttt atttgagcaa atgccgtctg aaatgttcag acggcatttt 1336380
ctattttaca gcggattaaa atatcttctc cgacctatag tggattaaca aaaatcagga 1336440
caaggagacg aagccgcaga cagtacaaat agtacggaac cgattcactt ggtgcttcag 1336500

caccttagag aatcgttctc tttgagctaa ggcgaggcaa cgccgtactg gtttaaattt 1336560
aatccactat aaaaacgaat ccgacacggc ttatctaaag gaatggttga aaacggcagt 1336620
ttccaataca acaaaatgcc gcctgaacat ttcagacggc atttgaccca ttactgctgc 1336680
ggctctgaaa ccataccgcc ttcatcaaaa tccggctccg gttcgttttg caacgtttta 1336740
ccgttcaatt tcaactgatt gtttttcaga gaaatggcag tatcaatctg gtcgccgttc 1336800
aaagtcagat atttttccct tgccatactc tgaaccgtac tgtccaccat caggcgcaag 1336860
gtctcgttga tgtcgtcaag acttgccctg ccttccgcct catcttcggc attgacgctg 1336920
aaaatattgc ctgcttgact gaccgccaag tcttccagca ttttttgggg aatactcatt 1336980
ctgatgtcgg cttcggtttt cttcagcatc aaacccaatt gattcaaatc ttccttcttc 1337040
atgtctttaa acatgatttt tccgcccaca tcgatttttc ccgatggcag cgtgaatcgg 1337100
aaagttttaa tgtccaatac gggattgttg gtgaacagtc cggaagcctc tcctttgacg 1337160
gcggcaatca aatcattgcg gatttgttcc tcggtcattt ttttggcgga aatttgtgca 1337220
aacttgcgtt tcaatacggt taaggcagaa gcatcgaggt gttcggcagc gatatggatg 1337280
tccagcgggc cgtatttttc atcgccgtac accagtgtat cgaaacggaa ctgcccttca 1337340
ctgttgataa acgcgcctga ttccccggtc ttggttgaaa aagccagttt gccgacttcg 1337400
attttggaag gtgcgatgct gccgttggga ttgataaacg cgccaatctg caaatcggta 1337460
acaagattga ccagttcgtt taacttgacg ttgtaatcga caccctcttt ccattctagg 1337520
gagaattttt ccaaggtcag attgctgctg cccaaagcaa gcggattgat gccgtctgaa 1337580
gtttccgaat cgaaatgcac tttttcaaac gcggcatcgc ctttgtctgc cagcttgatt 1337640
ttaaacaagg gggcatcata gccgttccgg tagcttttga aacctttttg ataaaccgtt 1337700
tctcccgtca ggccttccca gtgcagcctg atgcccgaca gctcttcata atcgaaggcg 1337760
ggaacactga cttccatttt accgctgccg ttaaaataaa cggtattggc aagggaagcc 1337820
gggacttgtt ttccaaaaaa gcgttccaga actttttccg tttcaggcgc gtatttgaac 1337880
tcggtttcaa tgtacgcctg cgtgccgaat ccgccggcga aagggccgtg cgtgatatgg 1337940
ttaaccagcg taaccggctg ttccaacact gttttcaggt tatccggcag gtattttcgg 1338000
gcattattca gcaactcggg tttcagacgg atgaccgtcg tttccataga ggtaaaccag 1338060
ccgcgctcat attggtgcga ttcgacggtc aagaagcccg tttcctgcaa tattttttgc 1338120
tgctgcgtca agctttcttc ggctttgaca cccaaataat aaggcgtgcc caaagcaacg 1338180
ccgagcaatg ctgccgcaac cgaaatcaaa ggttttttca tcacttcaaa caagcaggtt 1338240
tcaaagacgc tagaatagca ttatttaagc gtatcccgcc atatctcttt aaaagaaatg 1338300
ccgtctgaaa cctgttcgga cggcattttc cggatatagg gaaatcagaa atccaattcc 1338360
gccttcagcc agtaagtgcg cggcataccg acgacggcga agctgcggtc gtattggccg 1338420
cgctgtacct gccaatagtt tttgttgaac aggttttcca ccgagctgct gacggtcaga 1338480
gtgtttttgc caagcttggt tttgtagcgc gcgcctacgt caatcaaggt ataggacggg 1338540
aaggcgtatt gtttttgcgt gtcttggtca gacttgccga aatacgaaac attaccgttt 1338600
aaagtcaagc ctttggcaaa cggtgtatcc cattccaaac ctgctttggc aattacgcgc 1338660
ggattggcga cttgtacgcc gttaaccagc atatcgcgtg aatttggata ctctttcacg 1338720
gtcgattgca gatacatcag acccaaagtc ggacgcaaag tattgttgag caagttcgcg 1338780
taggtgttga actcaatacc gcgattgcgt tccatacctt gctcgtcgcc ggccgcgccg 1338840
ccttgcgcct tatagcgggc gaaatcagaa ttattgccat aggtcagcgt tgttgttacc 1338900
ccttttgttg tcttggtagt tgtatgaccg cgccagtagc ccgggcgttt gatttggaac 1338960
gcgtttaacg tggttacgaa attgccccag tttttacgca cgcccacttc aaactggcgg 1339020
ctgacacgcg gtttcgccat tgtcgtttcg ccggaatcat cggttttgat gtcggcaggc 1339080
tccaagtctt ccatatagtt gccgtacaca accaaatcag gttgcggcac ccacgccgcc 1339140
atcagcatcg ggctgaaacg tttggcatcg ccgctctgtg attttttgtc ggtatattcg 1339200
actgtttgga aacgtccgcc caaagtcagg cggtatttgt tatccacgaa gcccaaggtg 1339260
tcggacaaag ccaggctgtt gactttgata ttggcatcca agttggcaga gttctcccaa 1339320
gaattgggat agtcggctgt aaacgatgcc aattgatgct caatatttcc gtttgccttc 1339380
acttctacct tgctagctcc ggctgccgtt ccgcgtgatt ttttcttatt ggtgtattca 1339440
accgcttgga aacgtccgcc caaagtcagg acaagcgtcc gccggcgttt tgaatgtcct 1339500
gcatacgcgc gcgaccgccg ttggttttgc gttttgcgta gatggaatcg aacgctacgc 1339560
gcagtgtttc gccgcgatag tcggcattta ccgcaaattc tttgttgtct tcgctgtaac 1339620
cgtggcgcgg ggtgtcgccg tggcgcagtt tgccgttggc gcgcacgccg aatgctttgt 1339680
tttcgccgaa acgttggccc aagtcgaacg taccttgggc gcggttgttg ccgaaccggg 1339740
ccaaaccgat tttgcggttg ccttcatcag cggctttttt ggtttcgata ttgacggaac 1339800
cggataccgc gccatcaggg ttcatgccgt ttacggcggt ggacgcgcct tgaatcagtt 1339860
gtgcggagcc gacttgcacg ctggtcgtgc cttgcgtgcc gtacatacct gtcaaaccgt 1339920
tgacgctgaa ttggcgcgca tcaagctgat aacctctgaa atacaatccg gtcagcgtgt 1339980
tgctttcgcc gccgaactgc caaacggaag cgtctttttt cgctacggca tccaccaaag 1340040
tacgcgcctc ggtgttgttg agggcttgtt cgtcgtagtt gacgacggta atcggcgcgg 1340100
taaaggcgtt ggctttgccc attcacttgg tgcttcagca ccttagagaa tcgttctctt 1340160

tgagctaagg cgaggcaaca ccgtactggt ttttgttaat ccactataaa caaatcgtac 1340220
agggttctcc gtttaatcag atatgggttt ccatcttcgg cagtttcggg catttagccg 1340280
tttccacctt cctgcccccg ctgccagtaa aaatgccgtc tgaaatatcg gcgcgatact 1340340
tcagacggca tacccgcccg ttttcccgca ttcccgggag cgggctgaaa tttaaacgtg 1340400
tgcggaaatg attttcaaca tttgcgccag cactttggga ttggcagcca caatatcgcc 1340460
gctttccagc cagccgtctt cacccgacat atcggttacg ataccgcccg cttcctgaac 1340520
aatcaatgca ccggcggcaa tgtcccacgg tttgaggtta aactcgaaaa agccgtcaaa 1340580
acgtcctgtt gctacggcgc acaaatccaa agaagccgca ccttcacgac ggccgccggc 1340640
ggtttttgcc aagaaatctt tcaaaatcgc cagatacttg tccatcatgc tttgatcgac 1340700
aacagggaag ccggtaccaa tcaggcagcg gttcagttcg atgcggttgg aaacgcggat 1340760
gcggcggtcg ttgagcaacg cgcctttgcc acgcgaagcc atatatacgt cgttgcgttc 1340820
gggggcgtaa accaaagctt cttgcaacac gcctttgtgc agcagcgcca tagagatggc 1340880
gtattgggga tgaccgtgaa ggaaattggt cgtgccgtcg agcggatcga taatccattc 1340940
gtactcggct gcggctttgc cgtgggagcc gctttcttca caagtgattt tgtggtgcgg 1341000
ataggcttct ttcaaagcct caaccaggat gatttcggaa ttgcggtcaa catcggaaac 1341060
aaaatcgttg aaggctttgc tgtcggtttt gacggcatcg agattgcccg cggcgcgtat 1341120
catcatctga ccggcacggc gggcggcttt aaaggctgta ttcaaaaacg gattcatcag 1341180
atttccttaa gggtggcata ccgccggttc ggacgtacag tccttcggag cggcaaaatc 1341240
ggagtttatt tggttggggt aaatcctgcc aaatcgggta aaataccgcc tgacgcgtgt 1341300
ctgcttcagg cgcaacgtta aatttccgac gttgttaaag aacatttcag acggcatttg 1341360
accgtccgaa cgaaaaagac ggcgcattat accctattcc attccgaccg aaaaccgaac 1341420
atgactactc tcaaacccgc cctgcccgct tatctggaca acatccgcat catcctcacg 1341480
cgcaccagcc atcccgccaa catcggctct gccgcgcgcg cgatgaaaac aatgggtctg 1341540
cacaaactga ccatcgtcgc cccaaatctg atggcaacgc cgatgacgga aaacccgccc 1341600
gtgtttgacc cggagcatcc tcaatcgttt aaattaccgg aagaaagctt catcctcgct 1341660
tccggcgcgg cagacgtttt ggaaaatgcc accattgccg cttctttgga cgaagccctt 1341720
gccgacacca ccatcgcctg cgccctgacc agccgccgcc gcgaaattac tgcgccgctg 1341780
caaaccccgc gcgatttggt atccgaatta ctgcagaccg caaaccgagg cgagaaagtg 1341840
gcactggttt tcggcaacga gactttcggc ttgagcatcg aagaagtcca agcctgcaac 1341900
cgactgatga ccatcaacgg caatcccgac tatttctcgc tcaacctcgc ccaagccgtg 1341960
caggtcgtgt gctacgaaat cttcagccaa accggttcgc ccatgaccca tcttcaacaa 1342020
gaagaccacg ctgcgaccca cgagcaaatc aaaggcatgg tcgcccacat ggaaagcgtg 1342080
atgaacgaca tcggcttttt caaccgccgc aacggcgagc gtctgatgcg ccgtatgcag 1342140
agcctgttcg gccgcgccaa tacgcaaacc gaagacatcg atatcctgcg cggttttttc 1342200
aataccgtca gccaccgtat ccataaaaaa gactgattaa ggccgtctga aaacatttcc 1342260
agcttttcag acggcatgac tgatattcgg ataagcatga attacgccct agacgcatta 1342320
tggtggaaac ttaccagcca acccgtccgc gaccttgcct cgctgctgac tgcgccgcct 1342380
ttgtggcaaa gcggctgcga attgagcgtg cgagaactac tgggagaaca cggtttccgt 1342440
tacctttttgg cattggatgc cgatcccacg cggctgacgg attacctcgc ccaacgcgcc 1342500
ccgttcggcc accgtctcgg catttatgcc gaagagctgc tggctttttg gtttgccaat 1342560
gcaccgcacg ccgaactgct cgcgcacaac ctcacggttt ccggttcgga cggcaatacg 1342620
caaggcgcgg cggattttgt ggcaaggctt aacggcaaac cctaccatat cgagctgacc 1342680
tgcaaatatt acggcggcga cacggacagt cccgaaggga tgcgcggatt cgaccccaaa 1342740
gacacgctgt tgggaaaagc cgccaaactg accgcccaac tcggtctgcc gcacacttca 1342800
gacggcatcc ggaccttgcg gcagcacggt ttgccgctta acgtaaaacc cgtttccatc 1342860
gtgcgcggca tcggattttt tccacacggt ttccatgctt ttgagccacc gcttaatcca 1342920
tacggttggc gcggcatcta tattcaagat tgggcggaat acgggtttaa acgccaagaa 1342980
gtccgctacc atctgctcga ccgtatggcc tacctcgcgc ctgcgcgtgt cgccgaaacc 1343040
gaaacattga acgcaaccga aatccgccgt atcgaccaag gcttgattgc cgttttggaa 1343100
tgtcggccgg acggcttttg gcacgaaatc gaacgcatta tgaaggccgt ctgaaaccct 1343160
ttcccaacat taacgcgtat atctattgag aggcttagtg atggaaatct catttcccat 1343220
acaatttatg aaagagtcat ccgagttaat aaggatattg gatatgataa atataacaac 1343280
aacatgccaa ctaatattat gacgatccaa acaaataagt atggtaattt aataactacg 1343340
accccaggta gaatacaatg aagaataatg ttaaaaattg gacaactaaa gaagtcaagc 1343400
aatcattaga taaatttaat aatattttaa ttaaaaatac ttttcttctt cagtatctga 1343460
aaaaagagtt ttcagcttca agtgcttatt gtttgtctat gctccctgaa gaagaagata 1343520
tatatgaaat attggttaat gggaatatta ttattgattt agaatttaat aaacatacaa 1343580
atgaaacagt tgttattaat gttactgatg ttgatgaata cttgaaaact ttaaccaatg 1343640
agagtggtag agtatttttt acattagcaa aagaaatcgg caaacagaaa aacatttaac 1343700
aagagcgaaa tacaaattaa aaactcaatg gagtgatggc atatttagga tagatatact 1343760
gaacgaagaa aataaatttt gtttttttcc tcatgtttta gggagtgatt acaaaatgaa 1343820

atcgctgatg tgattatatc ggatgctgtt caagcgacct gaaaatagaa ctttttttcag 1343880
gctgcctttg tagttaacgg agaaatttag acaaatcccg attgcgcact tttaacacat 1343940
ctttcttatt gcggatagaa tactaagtaa tgataaagat gctattgtta ttttaaggac 1344000
gttagattga ttatgaataa cccacagtaa gagaacccat tacattatga acgccgcaca 1344060
actcgaccat accgccaaag ttttggctga aatgctgact ttcaaacagc ctgccgatgc 1344120
cgtcctctcc gcctatttcc gcgaacacaa aaagctcggc agtcaagatc gccacgaaat 1344180
cgccgaaacc gcctttgccg cgctgcgcca ctatcaaaaa atcagtaccg ccctacgccg 1344240
tccgcacgcg cagccgcgca aagccgctct cgccgcactg gttctcggca gaagcaccaa 1344300
catcagccaa atcaaagacc tgcttgatga agaagaaaca gcgttcctcg gcaatttgaa 1344360
agcccgtaaa accgagtttt cagacagcct gaataccgcc gcagaattgc cgcaatggct 1344420
ggtggaacaa ctgaaacagc attggcgcga agaagaaatc ctcgctttcg gccgcagcat 1344480
caaccagcct gccccgctcg acatccgcgt caacactttg aaaggcaaac gcgataaagt 1344540
gctgccgctg ttgcaagccg aaagtgccga tgcagaggca acgccttatt cgccttgggg 1344600
catccgcctg aaaaacaaaa tcgcgcttaa caaacacgaa ctgtttttag acggcacact 1344660
ggaagtccaa gacgaaggca gccagctgct tgccttattg gtgggcgcaa aacgaggcga 1344720
aatcattgtc gatttctgtg ccggtgccgg cggtaaaacc ttggctgtcg gtgcgcaaat 1344780
ggcgaacaaa ggcagaatct acgccttcga tatcgccgaa aaacgccttg ccaacctcaa 1344840
accgcgtatg acccgcgccg gactgaccaa tatccacccc gaacgcatcg gcagcgaaca 1344900
cgatgcccgt atcgcccgac tggcaggcaa agccgaccgt gtgttggtgg acgcgccctg 1344960
ctccggtttg ggcactttac gccgcaatcc cgacctcaaa taccgccaat ccgccgaaac 1345020
cgtcgccaac cttttggaac agcaacacag catcctcgat gccgcctcca aactggtaaa 1345080
accgcaagga cgtttggtgt acgccacttg cagcatcctg cccgaagaaa acgagctgca 1345140
agtcgaacgt ttcctgtccg aacatcccga atttgaaccc gtcaactgcg ccgaactgct 1345200
tgccggtttg aaaatcgatt tggataccgg caaatacctg cgcctcaact ccgcccgaca 1345260
ccaaaccgac ggcttcttcg ccgccgtatt gcaacgcaaa taaaccggtt tgaacaaaat 1345320
gccgtctgaa ccctttttcaa agcgttcaga cggcatttca tcaattatag tggattaaca 1345380
aaaatcagta cggcgttgcc tcgccttagc tcaaagagaa cgattctcta aggtgctgaa 1345440
gcaccaagtg aatcggttcc gtactgtttg tactgtctgc ggcttcgtcg ccttgtcctg 1345500
atttttgtta atccactata tttttgggaa tctgttttac cccaatatat aaagcaccat 1345560
attaaggcgg agtgtcttcc ccactttgac ccgaacccgg aaaagacacc gcccaagcca 1345620
atcctgatgc tgccccgaca gccaaccatt aaggaaatcc taatgaactt tgctttatcc 1345680
gtcattatgt tgaccctcgc ctctttcctg cccgtcccgc ctgccggagc cgccgtcttt 1345740
acttggaagg acggcggcgg caacagctat tcggatgtac cgaaacagct tcatcccgac 1345800
caaagccaaa tcttaaacct gcggacgcgc caaaccaaac cggcggtcaa acccgcccaa 1345860
gccgacgcag ggaagcgcac agacggcgcg gcacaggaaa acaatcccga cactgccgag 1345920
aaaaaccggc agcttgagga agaaaagaaa agaattgccg aaaccgaacg gcagaacaaa 1345980
gaagaaaact gccggatttc aaaaatgaac ctgaaggcgg tgggaaattc aaatgcaaaa 1346040
aacaaggatg atttgattcg gaaatacaat aacgccgtaa acaaatactg ccgttaatcg 1346100
gctctagcgc aaacccgatg ccgtctgaag cggcacgggg tttgtcattt ctgccagtag 1346160
gttttgacgt tgacgaactc gtacagcccg aattcggaca attcgcgccc gtaaccggaa 1346220
tctttgactc cgccgaaagg caggcgcaaa tcgctgctgg tatggcggtt gataaacacc 1346280
gatcccgcct gtatttttttc ggcaaaccgc caagcgcgtt cggtatcggc ggtataaatg 1346340
caggcaccga gcccgaacgg ggaatcattg gcaagggcga tggcatgttc ttcgttttcg 1346400
gcgcgcaaaa tcagggcggc cggcccgaat acttcttctc tccagacgcg gcaggcagga 1346460
tttaccctgt ctaaaaccgt cgcgggataa aaccagcctc gcccttgtgg gatttttccg 1346520
ccggtcaggc ataccgcgcc gtttgaaacg gcatcttcaa cctgcccgtg aaccctgtcc 1346580
cgcaaatctt cgcggtgcag cggtgcaagc gtagtatcgg gatgtttggg gtcgcccatt 1346640
ttcaatttag cgcattcggc aagaaacagc gtgataaaac gatcggctgc ggcttcggtt 1346700
acgatgatgc gcttggcggc gttacacgat tgccccgcat cgcggaaacg ggaataacag 1346760
gcttctgcgg cggcacgctc caaatcagca tcgggcatca cgataaaggc gttgctaccg 1346820
ccgagctcca acacggtttt cttaaggttt gcgcccgcgt gtgccgcaag gatgcgcccc 1346880
gtatgcgttg aaccggtaaa cgccattgca tcggtatctt caaccgcctt gagcgtgccc 1346940
gcctcatcca gccacacgcc tgccagagga atgccgtctg aagccaaatc gaacagtgcc 1347000
tgactgacgc gtgccacgct gggcgcgggt ttgacggcgc acgcgttgcc cgcgcacata 1347060
gcgggaacgg cgaaacgcaa tacctgccag acgggatagt tccaaggcat gacggcaaac 1347120
accacgccca aaggctcgaa gcgcacctga ctcaaactcg cctgcgtcgc gatggttttg 1347180
tgggcaagca gttcgggggc aaggcgggcg taatagcgta tcagttcgat agacttgccg 1347240
atttccgcac ggcattcgtg caagcagcgt ccgacttcct cacacaccat ttccgcaaaa 1347300
cgctctttct ccgcctccaa acggtcggca aatttttgca ggcgcgcggc acgttcggtt 1347360
acgcccagtt gcgcgaacgc cccgccgcgc attttcaaat ccgccagccg ccgttcaaac 1347420
tccgcataat cttgagcggg gcggcggtaa agcgtttcgc ccgtaaatac attgacactg 1347480

tgaaacatcg aatcaacctg ccagttgcgg gaatatcgtt ttcagtcccg acacaataat 1347540
ctccaccgat accgccgcca gcatcatacc cataatgcgg tttaaaatcg tcagccccgt 1347600
cgcgcccagc aggcggctga ccttcccggc aacgattaaa atggcataac aaatcgcact 1347660
gaccaccaaa ccggccgcga taatcaacgc gatgtcgccg tatgttttag ccgccgaagc 1347720
gtaaataatc acggtcgaaa taccgcccgg gccgatggtg atcggtatgg cgatgggcac 1347780
gacggcaatc gctccggcat tgcgggcggg gcgcgcctgc cccgtttccg gctgcgcgcc 1347840
gagattctgc ttggcgggat tgtcgttgcc gttcatcatc gaaatggcga tcagcagcac 1347900
caaaatcccg ccgccgacct gaaacgaacc gacgctgatg cccaaaacct tcagcagcgt 1347960
accgccgatc agcgcaaata ccgcaatcac ggcaaacacg gcaacggcgg ccgtccgcgc 1348020
gaccttcctg cgctccttcg tgctgtgccc gttggtcagg tcaaggtaaa gcgacaacgc 1348080
gctaaacgga ttaatcagca ccaaaaaagc cacaatcagc ttgccgattt ccatgcccaa 1348140
tcccattatt tccccctcct tcaaacccgt gcggcaggca tccgatgctg caaattgccg 1348200
ccgcaacgga tttttccgtt ataattaaaa attcaagcaa tacgccccat catacccgaa 1348260
cgacggtatc tttaccatca gacaaggatg cttttcatgg cactgacact tgccgacgta 1348320
gacaaaatcg cccgactctc ccgactgcac ctgactgcgg aagaaaaaga aaaatcgctt 1348380
caagaattaa acgacatttt cactatggtc gaacagatgc aaaccattaa cacagacggc 1348440
atcgaaccga tggcgcaccc gcacgaggcc gccctgcgcc tgcgcgaaga cgaagtaacc 1348500
gaaaccgacc gcgccgccga atatcaggcg ggtgctccgg aagtacgcaa ccgtctgtac 1348560
atcgtaccgc aagttatcga agaataatcc gaatatgctt cagacggcat cagcaatacc 1348620
gcccgaagcc ctttaaggat ggaagattta tgacccaata cacattgaaa caggcaagcg 1348680
tcctgttgca gtccaaacag atttccgccg tcgaactggc aagcgcatac cttgccgcca 1348740
tcgccgaaaa aaatcccgcc ctcaacggct atatcaccat cgaccaagat aaaacccttg 1348800
cagaagcccg tgccgccgac gaacgtatcg cgcagggcaa cgcctccgcg cttaccggcg 1348860
tacccgtcgc ctacaaggat attttctgcc aaaccggctg gcgcagcgcg tgcgcttcca 1348920
aaatgctcga caacttcatc tcccccctaca ccgccaccgt cgtccaaaac ctgctcgacg 1348980
aaggtatggt aacgctcggc cgcaccaata tggatgagtt cgctatgggt tcgaccaatg 1349040
aaaactcatt ctacggtgca gccaaaaacc catggaatct tgagcacgtc cccggcggtt 1349100
cgtcaggcgg ttccgccgcc gtcgttgccg cgcgcctcgc ccctgccgcg ctcggttcgg 1349160
acaccggcgg ctctatccgc caacccgcat cgcactgcgg cattaccggc atcaaaccca 1349220
catacggcac ggtttcccgc ttcggtatgg tcgcctacgc ctccagcttc gatcaaaccg 1349280
gcccgatggc gcaaactgcc gaagactgcg cgattctgtt aaacgcgatg gcaggtttcg 1349340
accccaaaga ctccaccagc ctcgagcgcg aaaaagaaga ctacacccgc gatttgaacc 1349400
aaccgctcaa aggtttgaaa atcggcctgc ccaaagaata tttcggcgaa ggcaacagcg 1349460
ccgatgttct gacggcattg caaaacacca ttgatttgct gaaagcccaa ggcgcggaat 1349520
tgattgaagt ttccctgccg caaaccaagc tgtccatccc cgcctactac gtcctcgcct 1349580
ccgcagaagc cagcaccaac ctttcacgtt acgacggcgt acgttacgga caccgtgccg 1349640
cccaattcgc cgatttggaa gaaatgtacg gcaaaacccg cgccgaaggt ttcggcagcg 1349700
aagtcaaacg ccgcatcatg atcggcactt atgtactgtc gcacggctac tacgatgcct 1349760
actatctcaa agcccaaaaa ctgcgccgcc tcgttgccga tgattttcag acggcatttg 1349820
cacggtgcga cctcatcctc gcgccgaccg cacccactgc agccccaaaa atcggagcgg 1349880
atgcttcgcc ggttgaaacc tacttgagcg atatctacac catcgccgtc aacctcgccg 1349940
gactgcccgc attgaccctg cccgcaggct tcagcggcgg cggactgccc gtcggcgttc 1350000
agcttgtcgg caactacttc gccgaagcca aaatcctcgg tgcggcgcat caaatccaac 1350060
tcaacagcga ttggcacggc aaacgacccg aatgaagcag aaccgcacct ttaccttccc 1350120
cgattttcgc accgtttaca gctatgcgcc tttatatcgg ctgcaacatt taaaatacac 1350180
attgcgaaaa tttttcggaa aaaaagaaat ttacgccttc gagcagtttg tcaacgcatc 1350240
ccctatccgt caggggctgt tcctccactg cccgcaaaat gcctatccgc tgctgcgcga 1350300
atttgttgac aggcgtttta actgcaaacg ccgtttagat gcgatgacgg cagattttct 1350360
catggcggaa aaactgttcg gcacagacat cctgcaccaa atggaagact accgcttcca 1350420
tttggtcttg gcgcaccttt cagacggcat cagcttgtgg ctcaaccgca acgacaactg 1350480
cgtcgaagaa ggcgcgtggt ctttatcttt gcgcgacgaa gcaggcaacc ggctgtatat 1350540
ggcgactttc gcctttgtcg gcacacacct gctgacagcc tccgtacaag ggccggcggg 1350600
tgaagaagcc aaagacaccg tccgccgcat aaccaaacaa ctccacggct tgcgtcccca 1350660
acaactgatg gtaaccgccc tgcaatattt cgccgccgta ctcggcttgg acggcgcaat 1350720
gggcattgca caaaaacatc aggtcaaact gcgctggaaa cttaaaaagc gcgtcaaaat 1350780
gaattacgac gcattctggc aggaatacgg cgcaagtttg gaacgggacg gctactggca 1350840
tctcccccaa acccccgccc gcaaagacct tgccgacatc gaaagcaaaa agcgttcgat 1350900
gtaccgcaag cgttatgaaa tgctggacaa tatggttgca gagatgaaag acagtctgaa 1350960
aacagaagca cgcggcattt cagacggcat ccaaacggaa aaaccgcccc gccggacagc 1351020
ctgacgcgaa gactatcgaa ttgatatttt agagaaagaa gctcttatga cctgggaaac 1351080
cgtaatcggc ttggaaatcc acgtccaatt gaacaccaaa tccaaaatct tcagcggcgc 1351140

atcgaccgca ttcggcgcag aacccaacgc gcacgccagc gtagtggaat gcgcgctgcc 1351200
gggcgttttg cctgtgatga accgtgaagt cgttgaaaaa gccatcaaat tgggtttggc 1351260
tttagatgcg aaaatcaatc agaaaaacgt gttcgaccgc aaaaactact tctatcccga 1351320
cttaccaaaa ggttatcaaa tcagccagtt ggacttaccg attgtcgaac acggcaaatt 1351380
ggaaatcgta gtcggcgacg atgtgaaaac catcaacgta acccgtgcgc acatggaaga 1351440
agacgcaggc aagtccgtgc atgaaggctt gaacggcgca accggtatcg acctgaaccg 1351500
cgccggcacg ccgctgttgg aagtggtatc cgaacctgaa atgcgttccg ccgccgaagc 1351560
cgttgcctac gccaaggcct tgcacagctt ggtaacctgg ctggacattt gcgacggcaa 1351620
tatggcggaa ggctcgttcc gcgtcgatgc caacgtatcc gtgcgcccga aaggtcaaga 1351680
agagttcggc acgcgccgcg agattaaaaa cctcaattcc ttccgtttct tggagcaggc 1351740
gattaattac gaagcggaag cgcaaatcga gattttggaa gacggcggca aagtacagca 1351800
ggcaaccatg ctgtttgatc ccgaaaaagg cgaaacccgc gtaatgcgcc tgaaagaaga 1351860
tgcgcacgac taccgctact tccccgaccc tgatttgctg cccgttatca tttcagacgc 1351920
ccaaatgcaa aaagccaaag cagaaatgcc cgagctgccg aaagaaatgg cagcgcgttt 1351980
cgtggcggat tacggcgtgt ccgaatacga cgcgcgcctg ctgaccgcaa gccgtgcgca 1352040
ggctgcctat tttgaagaag ccgccaaaga aagcggacaa ggcaagctga ctgccaactg 1352100
gatgaacggc gaacttgccg ccgcgctgaa caaagaaggc atggaacttg ccgacagccc 1352160
gattaccgcc ccgcgcctcg ccgcgctggt tggcaaaatc gccgacggca cattaagcag 1352220
caagttagcg aaaaaagcct ttgaagccat gtgggcagaa cccgaagcca ccattgccga 1352280
aatcattgaa aaacacggtt tgcaacagat gaccgacacc ggcgagattg aagccatggt 1352340
ggacgaagtg ctggcaaaca acgccaaagc cgtggaacag tttaaatccg gcaacgaaaa 1352400
agccctgaat gcgattgtgg gacaagtgat gaaggccagc aaaggcaaag ccaaccccgc 1352460
gcaggttcaa gagctgatta aagccaaact ggcttaatcc gttatcacac aggtcgtctg 1352520
aaagcaaagt tccaacgaag gtaaaacagg aaataagctt tcagacggcc ttttatagtg 1352580
gattaaattt aaaccagtac ggcgttgcct cgccttgccg tactatttgt actgtctgcg 1352640
gcttcgtcgc cttgtcctga tttaaattta atccactata acttaatctg ctcaaaccat 1352700
accaagacat gaaccacacc gttaccctgc ccgaccaaac cacctttgcc gccaacgacg 1352760
gcgaaaccgt tttgaccgct gccgcccgtc aaaacctcaa cctgccccat tcctgcaaaa 1352820
gcggtgtctg cggacaatgc aaagccgaac tggtcagcgg cgatattcaa atgggcggac 1352880
actcggaaca ggctttatcc gaagcagaaa aagcgcaagg caagattttg atgtgctgca 1352940
ccactgcgca aagcgatatc aacatcaaca tccccggcta caaagccgat gccctacccg 1353000
tccgcaccct gcccgcacgc atcgaaagta ttattttcaa acacgatgtc gccctcctga 1353060
aacttgccct gcccaaagcc ccgccgtttg ccttctacgc cgggcaatac attgatttac 1353120
tgctgccggg caacgtcagc cgcagctact ccatcgccaa tttacccgac caagaaggca 1353180
ttttggaact gcacatccgc aggcacgaaa acggtgtctg ctcggaaatg attttcggca 1353240
gcgaacccaa agtcaaagaa aaaggcatcg tccgcgttaa aggcccgctc ggttcgttta 1353300
ccttgcagga agacagcggc aaacccgtca tcctgctggc aaccggcaca ggctacgccc 1353360
ccatccgcag catcctgctc gaccttatcc gccaaggcag caaccgcgcc gtccatttct 1353420
actggggcgc gcgtcatcag gatgatttgt atgccctcga agaagcacaa gggttggcat 1353480
gccgtctgaa aaacgcctgc ttcacccccg tattgtcccg ccccggagag ggctggcagg 1353540
gaagaaatgg tcacgtacaa gacatcgcgg cacaagacca ccccgacctg tcggaatacg 1353600
aagtatttgc ctgcggttct ccggccatga ccgaacaaac aaagaatctg tttgtgcaac 1353660
agcataagct gccggaaaac ttgtttttct ccgacgcatt cacgccgtcc gcatcataat 1353720
tccccggtat aaagaggatt cgagctttcc gttcagaaca caaaaaactt cccgtccgtg 1353780
ttttccccgt gaaaaaatgc cgtctgaaac ccgattccgg ttttcagacg gcatatgttt 1353840
tttcctgttc aaggcgacag ccgctcgcgt atccagccac catccagcaa acggtattgg 1353900
atgcggtcgt gcagcctgct cggtctgccc tgccagaact caagcaaatc gggaatcaca 1353960
atatagccgc cccaatgcgg cggacgcggc acatgcagag gatgtttgag tccaaccgcc 1354020
gccgcctttg ccaccaatac cgccttgttc ggaataacct cgctctgcgc acttgcccac 1354080
gcacccaaac ggctctgata cgggcgactc tcaaaatatt cgtccgacaa cttctccgcc 1354140
agcctttcaa cacgcccttc cacgcgcacc tgacgctcca gctccggcca aaaaaacgtc 1354200
atcgccgcaa atggatgagc atccagcgaa cgccccttgc ggctgtgata attcgtaaag 1354260
aaaacaaaac cttcagaatt aacttccttc agcagcacca tacggctgtt gggcctgccg 1354320
cgtccgtcaa ccgccgccac attgaccgcc gtcggctcgt tgacctgtgc gcgtaccgcc 1354380
tcgtccaacc accgctcgaa ctgctcgatc ggattatcgg cgcaatcggc ttccgacaat 1354440
tcccgtttgc tgtaatcttc ccgaatattg tgcaaatcca tttactgctc ctcttatcat 1354500
ttgaaagatt ctactcccgc acgcaaaccg atttcaaccg tcgcacaaac tttgccccga 1354560
ccccaagccg cagcgacgat ttcatccgca aaaccgccgc atcaggtaca atatcgaacc 1354620
gtccgaccga ggacggcatt ttatcaaccc gtcctgccgc acacgccgca gaagaaccgc 1354680
cttatcaggc gagttaggaa aaatgatgtc caaacagccc accagcaaac gccaatggcg 1354740
cgacggcgca gccccgtctg ccaagaaaac cgccaaaccg ttcaaaagca aagcccgtcc 1354800

caaagatgaa acgggcaaaa ccgcttccca accttacgga caaaaagctt cagacggcat 1354860
caaacctcaa aacgtcccca aacagcgcgc cgccaaagcc aaaaaactcg tcgtccgcaa 1354920
tcccaaccaa aaaattatgg aacacgcgcg cgatttgaaa gaacgccgca gcgacctgtc 1354980
gcgcatggaa cccgaacgcc tgcaaaaagt gcttgccgcg tccggcgtcg gctcgcgccg 1355040
cgaaatggaa gaatggatta ccaacggctg gataacggtc aacggcaaaa ccgcgcaact 1355100
gggcgacaaa gttacccccg acgaccacgt taccgtcaaa ggcagcatca tcaagctcaa 1355160
atgggcggac cgcctgccgc gcatcatcct gtattacaaa caagaaggcg aaatcgtttc 1355220
ccgtgacgac ccgcaaggcc gcgtcagcat attcgaccgc ctgccgcagg ccgccagcag 1355280
ccgctgggtc gccatcggac gcttggacat caacaccagc ggacttctga ttcttaccac 1355340
ctccggcgaa ctcgtccaac gtttcgccca ccccagcttc gaagtcgaac gcgaatacgc 1355400
cgtgcgcgtc ttgggcgggc tgaccggcga acaaatgcgc gtcctcaccg aagaaggcgt 1355460
gatgctcgaa gacggcttgg caaaagtcga acgcatccgc gaacaaggcg gcgaaggcgc 1355520
gaacaaatgg tacaacgtcg tgattaaaga aggccgcaac cgcgaagtgc gccgcatttt 1355580
tgaaagccaa ggactcaccg tcagccgcct cgtgcgcatc ggcttcggtc ccatcggact 1355640
gcccaaccgc ctcaaacgcg ggcagttcta cgaactcaac cccgccgaag tcgccaacat 1355700
catcaaatgg gcggacatgc tgctgccggg cgaacgccgc cgcaaaaaag cctaaacccg 1355760
ccaaaacaca aaaatgccgt ctgaaacatc tgctgtttca gacggcattt tattcgggcg 1355820
ttttcaggag aaaaggtcga gtgctttgac aaagaccatc accacgccgt aggcgagcgg 1355880
tatgacggcc aatgcccaac gccaccagac cgatatgccg ccgccggaaa ctttttcgcc 1355940
cacaaggtaa gcgtcggata tggcggtttc gtcatcgggg ttgccgctgt gtgcggcggt 1356000
tttgatgtct ttttcgtggt gtttttcgtg tacggatttg acggcgaggt tgcacaacaa 1356060
accgataatc agcaggcacg ccatgatgta catggttacg ctgtatgcct gtgccgccgg 1356120
tatgccgctg tcgatttggc tttggcgtat gtaattgacc agtaccgggc cgatgacggc 1356180
ggcggttgac caggccagca ggatgcgtcc gtgaatcgcg ccgacctgat aggtgccgaa 1356240
caggtctttc aggtaggcgg gaatggcggc aaatccgccg ccgtacatgg aaataatcac 1356300
gcaaaagccg atgatgaaca gggctttgct gccgccctcg ccgatggagg gaacggcgaa 1356360
atacagcagc gaaccgagta cgaagaagat ggtgtaggtg tttttgcgtc cgattttgtc 1356420
ggaaacgctc gaccacaaaa agcgtccgcc catgttaaac aggctcagga ggctgacgaa 1356480
gcctgccgcc gcacctgcgc cgactgctgc ctgcctgcct atggaggttt cggaaaagag 1356540
ttcctgaatc atcacggatg cctgacccaa tacgccgatg ccggcagtta cgttcaggca 1356600
caatacccag aacaacagcc aaaactgcgg cgttttcatg gcttgggaca cgttgacatg 1356660
attgctgctg accagcttgt tttgcgtttt cggcgcggta tagccttcag gtttccagcc 1356720
gtcggcaggt acgcggatgg taaacgcgcc gaacatcatc agtgcgaggt aaagcagacc 1356780
caatacggcg aaggtttcgg caaccccgac cgaagcagcg tttgaaaagg tgttcatcag 1356840
tgatacggaa agcggcgagg ccagcattgc gccgccaccg aaacccataa tcgccaaacc 1356900
ggtcgccata cccggcttgt cgggaaacca tttcatcagt gtggaaaccg gcccgatgta 1356960
gcccaaaccc aagcctacgc cgccgatgac gccgttgccc aaatagagca ggaagaggtt 1357020
gtgcgtacgc acgccgaatg cggatacgaa gaagcccagg ctgaagcagc aggcggcggc 1357080
aaatatggct ttgcgcggcc ctacccgttc catccacgta ccgaacaggg cggccgacgc 1357140
gcccagcatc gcgagtgcga tactgaaaat ccaacctacg gtcgtcagct tccaatctcc 1357200
ggccgccgat tcggttatgc cgataagttt ggtcagcggc gcgttgaata cggaataggc 1357260
gtaaatctgc ccgatggcaa ggtgtaccgc caatgctgcg ggcggtacga gccaacggtt 1357320
gaaacccggc ttggcaatgc ttgcctcacg gtctaaaaac ttcataacat cctctttctg 1357380
tcagttgaaa aataaaattt catttgccca atggaaactt attgaaaatt ataaaaaaat 1357440
atcgggtcgg gtttttatcc gccccaagat gcgccgtctg aaacatttcg ggtgtacgga 1357500
aaggtttctg tttttccga caaattcctg cggcttttcg cttccggatt cccgcttttt 1357560
caggaatgac gaattaaaga ttatcttaag gtcaagggac tggattcccg ctttcgcggg 1357620
aatgacggcg gcgggggagc ggtttttccg attgggttta aatgcaatcg aacaaatcct 1357680
gctgcccttg ttctttgctt acgcgcacgt cggtttcgcc gtcggcgaag ataatgtgca 1357740
gcttctgccc ctgcttcaaa acatcggcgt tgcggatgac ttgtccgcgt gtgtttttga 1357800
cgacggaaaa gccgcgctcc agaatgtgct gcggcgaaac ggcttcgagc aatgcggctt 1357860
gggcagtcag gctttggcgg cggtgggtaa gcagttggtg gaaggcgtgc gacagggccg 1357920
tctgaaagcg gtcgatgttt tttttgcaaa cggaaacatc aggacggcaa tgtttcaggg 1357980
cttgggtttg gcgttcgaaa cgggcggtgt gggtacggac gttttgcgtc atcgagtaag 1358040
acagcgtttg cgccagcttg ccgattgaag cgcgctgttc gtcgagtttt tggcgcggat 1358100
gacggatttg ccgcgccagc cagtcgagtt tttggctggc atcgaaatag cgttgttcca 1358160
aaacggtttt cagacggcat tgggcttggg cgaggcggtg cagcgattct tggcggttgg 1358220
ggctgaccag ttccgccgca ccggtcggcg tgggcgcgcg catatcggcg acgaaatcgg 1358280
cgagcgtgaa atcggtttcg tggcctacgc cgctgacgac cggaaccgtg caggattcga 1358340
tggcgcgcac gaccggttct tcgttaaacg cccacaagtc ttcaatgctg ccgccgccgc 1358400
gacagacaat caacacatcg cattcggcgc gttgcgaggc ggttttaatc gcttgggcaa 1358460

tttgcaattc gctgcctgcg ccttgaacgg gtgtcggata aacgataacg gggatttcgg 1358520
gtgcgcggcg tttcaaggta gtaacgacat cgcgcaaagc cgccgccgcc agactggtta 1358580
cgatgccgat acattgcgga cggacgggca aaggtttctt gcgttccgcc gcaaacgcgc 1358640
cttccgcctg caactgcgcc ttcaaccgct cataggcttc gtaaagctgc cccaaacctt 1358700
tgagccgtac ttcgtttacg gtaatctgaa attcgccccg cgcttcataa atactgattt 1358760
ttcctgatac ctcgatatgg tcgccttctt tcaaaggctt cgccaaacgc accgccgcac 1358820
ccttgaacat cgcgcaacgc acctgtgcgc ggctgtcttt gagcgagaaa taataatgcc 1358880
cgctggcggc acgggtcagg ttggatactt cgccggcaat ccacaaaccg gcaaggtggt 1358940
tttccaaaag acttttggca aatgcgttca actcggaaac ggacaacacg tcagaatgaa 1359000
aaaaatcaga catcgaatca atcaaatagt aaaaaatatg aatatgtttt gaagcctaag 1359060
gcggcaccgg gccgcctaaa ttgtcaacaa tattataaca cgcgccatct tgccgcccgc 1359120
cttttcccgt atgacttttt taagcgggga atgggaaaaa tattcatcaa cctgcctgca 1359180
atctattcaa attgcaccgc cggcaggcta tgatgcggat attttcgaca ggaggaaaaa 1359240
tggatacgca ggcagttatc acacatatcg cccgatggtt ggacgaatac gccgcccggg 1359300
caaatgcaaa agggtttgtc gtgggcgttt ccggcggcat cgattccgcc gtcgtctccg 1359360
cactcgccgc ccgcaccggc cgccccacgc tgcttctgga tatgccgata cgccaacacc 1359420
ccggccagct tgagcgggca aggctgcaca tccgcaatct gcaacggcaa tatgccaatg 1359480
taagcgcgca aacggtcgat ctgaccgaca ccttccagac ctttgaacaa accgtcggtg 1359540
ctcatcagac ggcatttgac agtcagccgc tttccctcgc caacgccaga agccgcctac 1359600
gtatgctgac cctgtactac tacgggcaga tacacggact gctggttacg gggacaggta 1359660
ataagattga agatttcggc gtgggctttt ttactaaata cggcgacggc ggcgtggaca 1359720
tcagcccgat tgccgacctg accaaaacgc aggtttaccg gcttgccgaa gcattgggcg 1359780
tggacgaggc gattcaaaaa gccccgccga ccgacggcct gtgggatacg gaacgcaccg 1359840
acgaagaaca gatgggcgca agctatcccg aactggagtg ggcaatgggc gtgtacggca 1359900
cgcgcaaacc cgaagatttt gaagggcggc agcgcgaagt tctagaaatc tatacgcgac 1359960
ttcaccgcgc catgcagcac aaaatcaacc cgattcccgt atgccgcatt ccgcccgaat 1360020
tgctgggctg aaacacggaa atgccgtctg aaacggaaaa ccgtatttca gacggcatgg 1360080
aaatatccga ctcctatccc ttaagaatcg agtacgcggg caaacaaaat atcgttttcc 1360140
aaatgaatgt ggtcgttcaa atcctccacc atttctttcg ccagcgcgta aagccgcgtc 1360200
cagcttccgc aagccccttc tggcggttgg aaattgtcgg tcagctcttt gagccgtgcg 1360260
atggcgcggt cgtgttcttc gtgttcgtgc atcatcacgc cgatgggcat cgccgcaccg 1360320
cgtccgacac cctgattaat catcggaaac agcatccttt cctctttcat catatgcatc 1360380
agcagttcgt tctgcatata ggcaagcagc tcggcaattt ccgccggaaa ggtgtcggca 1360440
tgaacttggg ccactttttg cgccagcggc accaattctt caaattgtgc acggtggaca 1360500
ttgtggtagc gttgcaggat atgatcgacg gttgcaccaa aggggcggt ctcccaaacg 1360560
gaaaaatcag tcatcgcagt gttcctttta cagggtttcg ggtttggttt tgaacattca 1360620
tactttaaga atcaattcaa acggagcata caccgcccgc gcgcttctgt acagcctcaa 1360680
acgtattcct tacattttga taataaaagt aattttcaga aataaaatac tgtccgaacc 1360740
gtttttttaga atttgcaaag gcgattgggg cggtacagaa aaactattat cccgcccgcc 1360800
cacttgaaat ttttatgccc aagccctatc ctgcacgcta tcgtgccaat cccaaccgaa 1360860
aaggaaaaat aatgagcagc gaattgattg tacacaccag cgatgccgct tttgaaaaag 1360920
acgttttaaa tgcagatatc cccgtcctgc tggacttttg ggctccgtgg tgcggcccct 1360980
gcaaaatgat tgccccgatt ttggacgaca ttgccgccga atttgaaggc cgtctgaaag 1361040
tggtcaaaat caacatcgac gacaacgaag ccaccccgtc ccgtttcggc gtgcgcggca 1361100
ttccgaccct gatggtgttc aaaaacggcg aagtcgtcgc caccaaagtc ggcgcattgg 1361160
caaaaggtca gctgaccgcc tttgtcgaag cctctatcgc ctgataaagc gcaatcgaaa 1361220
aagccgccgg aagattccgg cggctttttc gcacccttaa gatttgtggc ggatttccca 1361280
gcacctatgg attttttgt tgcggaaatc ttcgggaacg gattgtttgg aaatgtcttt 1361340
gacggcgtat tgttccgata ccaagtcgtc taagacgaag ctgcgcaggt tgttggaaaa 1361400
gtacaaaatg ccgtctgaag cgagcagctt caccgcgccg tcaatcagct ttttgtggtc 1361460
gcgctggatg tcgaggatgt cggacatttt cttgctgttg gaaaaactgg gcgggtccat 1361520
cacaatgagg tcgaaccgcc tgccttcccc atatgccgtc tgaagatatt ggaacacgtc 1361580
ggcgcggacg attttgtgtc gttccgtatc gatgccgttc aattcaaaat tgcgtttcgc 1361640
ccaatcaaga tatgtgttgg acaaatcgac ggtttcgctg gatgccgcgc cgccggtggc 1361700
ggcatagacg gtgaagctgc cggtgtagga aaacaggttt aaaaaacgtt tgcccgccgc 1361760
cgtttcgccg acttttttgc gcgtgtttcg atgatccaaa aaaagccccg tatccaaata 1361820
cttatcaagg ttgacccaaa acttgcggcc gttttcggtg atgacgaaat cgtcgcccgc 1361880
cttgccggtt ttctcgtact gctgcaaacc tttttggcgt tcgcggcgtt tgaggcggat 1361940
ttgttcgggc gcaaaaccgg taacgaaagc gacggcttcc aagacttcgg caagccacgc 1362000
ttcgtattct tcgggccgca tcagccagcc ggtatcgtat tcctgaaggt ggattcgatc 1362060
gccgtaaaca tcggcggcaa aggggaattg ggggatgtcg cggtcgtaaa tgcgccaggc 1362120

ttcgatgccg ttgcgtttcg cccatttcat aaggtgtttg atgtttttgc ccaagcggtt 1362180
ggcaaacggt gtgatgtcgg tcattggttt caggcggaat aaagtggaaa acggcaattt 1362240
tactgtaatt aacgcccgat tgcttgaccg tttcgggcaa accctatacc atccgtcgct 1362300
tatcttgtca tacgaagcca tcgccttcca acctaaaccg cccttacggg cgcgtttctt 1362360
ctgttgcttt gattttgcaa agcatatctg tgcaggttgc cgtcgatgta aaccacaagc 1362420
aagccgcttg cgacaaccct gtaacttcac attccccgta tcgttaccct tccctgcttc 1362480
aggccgtctg aacctttcgg acgcgggcgt tgttgtcttc caaggatagc catgtctatt 1362540
aaatttgccg atttgaacct tgataaaaac attttgtccg ccgtcagcag cgagggttac 1362600
gaaagcccga cgccgattca ggcgcaagcc attccgtttg ctttggaagg ccgcgacatc 1362660
atggcttcgg cgcaaaccgg ctccggcaaa accgccgcct ttctgttacc gactttgcaa 1362720
aaactgacca aacgcagcga aaaaccgggc aaaggcccgc gtgctttggt gttgaccccg 1362780
acccgcgaac tggcggctca agtcgagaaa aacgcgctgg cgtatgccaa aaatatgcgt 1362840
tggttccgca ccgtcagcat cgtcggcggc gcgtctttcg gctaccaaac ccgtgccctg 1362900
agcaaaccgg tcgatctgat tgtcgccacg ccgggccgtc tgatggacct gatgcaaagc 1362960
ggcaaagttg attttgaacg tttggaagtg ctgattttgg acgaagccga ccgtatgttg 1363020
gatatgggct ttatcgacga catcgaaacc atcgtggaag caacgccgag cgaccgtcag 1363080
actttgttgt tctccgccac ttgggacggc gcggtcggca aactggcgcg caaactgacc 1363140
aaagaccctg aaatcatcga agtcgaacgc gtggacgatc aaggcaaaat cgaagaacaa 1363200
ctgctgtact gcgacgatat gcgccacaaa aaccgcctgc tcgatcatat cttgcgcgat 1363260
gccaatatcg atcaatgcgt gattttcacg tccaccaaag ccatgaccga agtcattgcg 1363320
gatgaactgt acgaaaaagg tttcgccgca aactgcctgc acggcgatat gccgcaaggc 1363380
tggcgcaacc gcacgctgat ggatttgcgt aaaggccgct gcaaaatttt ggttgccacc 1363440
gatgttgccg cacgcggtat cgacgtaccg accattaccc acgttatcaa ctacgacctg 1363500
ccgaaacagg cggaagacta cgtccaccgc atcgggcgca ccggccgcgc aggccgcacg 1363560
ggtattgcga ttacgtttgc cgaagtgaac gaatacgtca aagtccacaa aatcgaaaaa 1363620
tacattaacc gaaaactgcc cgaactgacc atcgaaggca tggaaccgac ccgcaaacgc 1363680
aaatccgcag gcggcaagcc gaaaggcaaa ggcggctggg gcgatcgtaa atccggcggt 1363740
tggcgcggcg atcataaacc gagcaaagaa ggcttcggcg gcaaaacgcg cggcgaaggt 1363800
ttcaagaaag aaggctttaa gagagacggt ttcaaaaaaa ccggcgaagg cttcaaaggc 1363860
aaacgcaaag ccggcgattc ttttgcaggc aaaggcgaac gccgttacaa agaccgctaa 1363920
gccccaacct gccgcataaa ccaatgccgt ctgaaaccga tttcgagttt cagacggcat 1363980
ttttgcaatg tttcagcacc gcccggcttt gatacccaaa ggattaggct gtaataaaaa 1364040
ccctttttccg ctttggcaac gattgaaaat ttccgtaaat tcaaatatct agattccttc 1364100
ctgcacggga atgacacgga agggtttcag atgcagggtg ggcattcctg cccacccaat 1364160
cccgcccttg caacggtggg caagaatgct cgccctacgg cttgactgtt cgatatgatg 1364220
ccgtctgaaa acccaacggc ggcatgacaa tgccaccctg ccaacgcacg taaatcagaa 1364280
ttgccatccc gacatcaaac gcttggaaac aaaatgccgt ctgaaaatca aacggcaaca 1364340
taacaatgtc cctaacaaat gcaaaaatgc cgtctgaaag ctcttcagac ggcattggcg 1364400
cgccgggttt accgcctcct gccgaaaccg cgcatagcgg ggcggcggta attggcgggc 1364460
gggcgcgttg tcgggcggta acgctgcgcc tgcgccgcct gttgttttgc acggaggctg 1364520
cgcgtgttca aatccctgct ggtgcgcgca ttggggcgtg cggactgatg gtaggctgca 1364580
cgcgcgcgcc gggcgacggg actgtcttgg ttgcctgccc gtgtgaattt gtttgccagc 1364640
gcgttgccga taaacgcgcc tgccgccgcg ccgaccaggc tttgcagcag ccagcttcct 1364700
gtcgattggt cgtaaatata ctgctgcccg tctttaccgg taacgggttg cccgttgttg 1364760
ccgtttgcct gtgcttcggc aggaatggtg tctttgactg cttcgggagt cagttggtaa 1364820
accgtatcgt ctgcctgctg tgcgagctgc tgttgcaggg cttcaatctg tttctgctgc 1364880
tgttcgagcc gcgcctgcgt gtcgtcttgg caggcggcga gtgcgaatgt tgcgataagc 1364940
gcggaggcga tgattttttt catgtgtgtc ctgtttgggt ggaaaatcgg ttttattgta 1365000
tcgccgtcgg gaattttggc aagcattctg ccggcaaatc gtgatgttta caggggcagg 1365060
gtgtgcaatt tgcggacaaa tgcgaggctg ttggcgactg ggttgccttt gttttcgact 1365120
tcgtgttcgg tttctacggt cagcaggcgg cggtttttgt gtttgttcag gctcaattcg 1365180
gcttgtgcgg gtgtgaaaaa caggcggtgt ttttcggcaa agcggcgggc gcggctgttg 1365240
gcggttttca aaatctgaag cagcgatacg tccagatgga agcgtcgcac gcccaatacg 1365300
agaatccgtg cggcaaaaaa atcggcggca tcggtaaact cggcggggct gctgcggctg 1365360
aagtcgtgcc gttcggcggc tatcagggcg gcaacggtgc ggatttgcgg aatcatcgat 1365420
tcgctgataa gtgtgtcgtc ccgccctgca tcgagaagca tgggggaaaa atcctgtgca 1365480
tcatcgacaa taatgctaca agtgtgcagg gtttcgtttt gtgcggcggt ttggggcatt 1365540
gcattcatgg tcattttcct gattctgtcg tgtgttgccg aatcgggcga cctgtgtgaa 1365600
ggtaacaaaa aagccgcccc gttttcgagc ggcctgtttt gcgtatggga tggatttcaa 1365660
gcaagcgcaa aaaagtaccg cacgtctgtg tggtaccaat agcaataagc ggttgtaaat 1365720
tttttgcctt gcatgatgaa atgccgtctg aagataaaaa tattggggag attctaaatc 1365780

aaaacgctgc cgcgcctcaa gcattttatc gaaatttttt tgatttttca tctatccgat 1365840
tgaaaatatt tcggtttatt tttaccgctg cccgatattg tcggcaattt ccctttatct 1365900
gctttgaaaa acggtgcata atcccgagca aaaccgcaat caggagcaat tatgcaaaac 1365960
tatctgaccc ccaatttcgc ctttgccccg atgattcccg aacgcgcttc aggcagccgc 1366020
gtttgggata cgaaagggcg tgaatatatt gatttttcag gcggtatcgc cgtcaatgcg 1366080
ctgggacact gccaccctgc ccttgtcgat gctttaaacg cgcagatgca caagctgtgg 1366140
cacatttcca atatctatac gacgcgtcca gcgcaggaat tggcgcaaaa attggttgca 1366200
aacagttttg ccgacaaggt ttttttctgc aactcgggct cggaagcgaa tgaggcggcg 1366260
ttgaagctgg cgaggaaata cgcccgcgac cgtttcggcg gaggaaaaag cgaaatcgtc 1366320
gcctgtatca acagttttca cggacgcacg ctgtttaccg tgtccgtcgg cggtcagccg 1366380
aaatacagca aggattatgc acccctgccg caaggcatta cgcacgttcc gttcaacgat 1366440
attgccgcgc tggaagctgc cgtcggcgaa cagacctgcg cggtcatcat cgagccgata 1366500
cagggcgaaa gcggcatcct gcccgccact gcggaatatt tgcaaaccgc gcgccgtctg 1366560
tgcgaccggc acaatgcgtt gttgattttg gacgaagttc aaaccgggat ggggcatacg 1366620
ggcaggctgt ttgcctatga acattacggc attgttcccg atattttgag ttcggcaaaa 1366680
gccttgggct gcggctttcc gatcggcgcg atgctggcga cagaaaagat tgccgccgcc 1366740
ttccaaccgg gcacgcacgg ctcgactttc ggcggcaacc cgatggcgtg tgcggtcggc 1366800
agccgcgcat tcgacatcat caatacgccc gaaactttaa accatgtccg tgaacagggg 1366860
cagaaacttc agacggcatt gctggatttg tgcaggaaaa cgggcttgtt ctcacaagtt 1366920
cgcgggatgg ggctgctact cggctgcgtg ttggacgaag cctatcgcgg acgcgcatcc 1366980
gaaatcaccg ccgccgcctt gaaacacggc gtgatgattt tggttgcggg tgcggacgta 1367040
ttgcgtttcg cgccttcgct actgttgaac gatgaggata tggcggaagg tttgcgacgt 1367100
ttggaacacg cgctgacgga atttgccgcg acatcagaca atccgtaaaa ctcaaatgcc 1367160
gtctgaaggc gggaaggctt cagacggcat cagaaacaaa aaaccgcttc agaaacgtgg 1367220
ttcaacgttc cgaagcggtt ttgtttgcca tcaggactcg aacaccaatt ccggttccct 1367280
gccctcttcg atgactgcct taccgaccac cactttttc aagcctttca aatcaggcag 1367340
gcggtacatc gtatcgagca ggcagcgttc gacgatggac ctcaggccgc gcgcgccggt 1367400
tttgcgttcc attgcctgac gcgcgatgga acgcaatgcg ccttcttcaa actccagttc 1367460
gacattttcc ataccgaaca acgcctgata ctgcttgacc aaagcatttt tcggctcggt 1367520
caaaatatta atcagcgcgt cttcgtccaa ttcttctaaa gttgcaatca caggcaaacg 1367580
tccgattaat tctggaatca gaccgaattt aatcaagtct tccggttcga cgatgccgaa 1367640
cagcttggta atgtcggcat tttcgtcctt gctgtgaacg gacgcaccga aaccgatacc 1367700
gccttttca gtacgctggc ggatgacttt ttccaagcct gcaaacgcgc cgccgcagat 1367760
aaacaggatg ttggtggtat cgacgttgat aaattcctga ttcggatgct tgcggccgcc 1367820
ttggggcgga acgctggcca ctgtaccttc aatcagtttc aacaaggctt gttgcacacc 1367880
ttcgccggat acgtcgcggg taatcgacgg gttgtcgctt ttgcgtgaaa ttttatcgat 1367940
ttcgtcgata tagacaatgc cgcgctgggc tttttcgaca tcgaaatcac atttgcccaa 1368000
aagcttggta atgatttgct cgacgtcttc gccgacataa cctgcttcag tcaaagttgt 1368060
ggcatccgcc atcacgaacg gcacatccag tttgcgcgcc aaagattgcg ccagcagggt 1368120
tttacccgat ccggtcgggc cgataagcag gatgttggat ttcgacaatt cgacattagc 1368180
tcctgcttta ggatggcgca ggcgtttgta atggttgtac accgacaccg ccaaggcttt 1368240
cttggcttgt tcctgaccga taacgtggtc gttgaggttg gcgacgattt cggcgggcgt 1368300
gggcagcttg ccggattctt ccggctcccc tccggcactt tccgaaggcg tgccgtcatt 1368360
tccgtcttca tgcaatattt caatacagtt tgagacgcat tcgtcacaga taaaggcgtt 1368420
ttcgccctca attaaatgtt tgacgtgtga tttggatttt ccgcaaaagg aacaagtacg 1368480
gttttcgttg gacatggctt tctttacaat gtatgcgtta cagaaaacgg cacgtgccgt 1368540
tcgggttgcc aagtataata actatatccg ttcttatcaa tgtattacct taaaatcccg 1368600
ccgattaggc tataatacgc cctttcgcaa ccgccccggc ggcaaaaatg ccgtctgaaa 1368660
ccaaatctga aatctgagga tattcatgag aaaaccccaa cgcggctatg cccgccaaga 1368720
ccgtgtcaaa gaacaaatta tgcgcgagct tgccgaactc gtccgtaccg gactgaaaga 1368780
cccgcgcgcc ggcttcatta ccgtcaacga agtcgaagtt acccgcgatt acagccacgc 1368840
caccgtgttc tacaccattt taaaccaaga cgcgcgcgaa attacggaag aagtgctgga 1368900
acacgcgcgc ggacacctcc gcagcgaatt ggccaaacgc atcaagctgt tcaaaacgcc 1368960
cgaactgcat ttcaaatacg acgaatcttt ggaacgcggt ttgaacctgt ccgcccttat 1369020
cgaccaagta gcggcggaaa aaccggttga agactgacgg atatgcccat gccgtccgaa 1369080
catcgaacca tgaatacagg caaaccccaa aaacgtgccg tcaacggtgt tttgctcttg 1369140
gacaaacccg aaggcctttc cagcaacacc gcgctgcaaa aagcgcggcg tttgtttcat 1369200
gccgaaaaag ccggacatac cggcgtgctc gaccctttgg caaccggact tttgcccgtc 1369260
tgcttcggtg aagcgaccaa gttcgcccaa tacctgctgg atgccgacaa agcctacacc 1369320
gccacgctga aactcggcga agccagcagc acgggtgatg ccgaaggcga aatcattgcc 1369380
accgcccgcg ccgatatttc cttagccgaa tttcagacgg cctgccaagc actgacaggc 1369440

aacatccgcc aagtgccgcc aatgttttcc gcgctcaagc acgaaggcaa accgctgtac 1369500
gaatacgccc gcaaaggcat cgtcatcgaa cgcaaagcgc gctacattac cgtttacgcc 1369560
atcgatattg ccgaatttga cgcgcccaaa gccgtcatcg acgtacgttg cagcaaaggc 1369620
acctacatcc gcaccctcag cgaagacatc gccaaacaca tcggcacgtt cgcccacctg 1369680
accgccctgc gccgcactga aaccgccggc tttaccatcg cccaaagcca cacgcttgag 1369740
gccttggcaa atttgaacga aacagaacgc gacagcttgc tgctaccctg cgacgtattg 1369800
gtttcacact ttccccaaac cgttttaaac gattatgccg tccatatgct ccactgcgga 1369860
caacgtccgc gtttcgaaga agacctgcct tccgacacgc cggtacgcgt ttacacggaa 1369920
aacggccgct ttgtcggtct ggcggaatat caaaaagaaa tatgccgtct gaaagccttg 1369980
cgcctgatga acacggcggc atccgccgcc tgaacggcgg ttaaaaatac aggctgtgct 1370040
tgaataatgt gttgatattt ccgcaaaatc ccgacacact cggacacccg ccccgcttat 1370100
cgcaactttg cgaacgcccc cggaaacagc aaagacatca aataattgat tttattagaa 1370160
tctatttgca aagccatttg ccgttacaca agaatggcac attaaaataa ctgatgagga 1370220
tttataacga tgaagacaga cattcaaacc gaattaaccc aagccctact accacacgaa 1370280
aaagtatggg cgaacgaaga aaaaaccatt ttagccaaaa acatcctgtt ggatttggtg 1370340
gaaaaaaccg acccgaccat tatcggtttg ttattgagta atgatgagtt aaaacgccat 1370400
ttctttgtgg aagtgaatgg tgtgctggtg tttaaattgc aggatttccg ttttttcttg 1370460
gacaaacaca gcgtcaataa ttcctacaca aaatacgcca accgcattgg tttgacggac 1370520
agcaaccgct ttttgaaaga cagcagtgat attgtgttgg attttccgtt taaagattgt 1370580
gtgttaaatg gcggacaaag caccgaggaa ggcgaagaaa tttattttaa acgcaataac 1370640
agccagccag ccagccagcc agccagccag ccagccagcc agccagccag ccagccagcc 1370700
agccagccag ccagccagcc aattatacac taaattaacc cgaaaaagac aagaaatctt 1370760
ttttaatcaa acccttgctt ttgatgaaat tgatcggctt tttgacgcaa aagcattctc 1370820
aaaattctct cgctataccg cagacggcaa acaagccgtt ggcgaaatca aacgacattc 1370880
agacggcaca cccgccgaaa atctcattat caaaggcaat aatctgattg ccctgcattc 1370940
gcttgccaag cagtttaaag gcaaagtgaa gctgatttat attgacccgc catataacac 1371000
gggtaatgac ggttttaaat acaacgacaa atttaatcat tccacttggc tgacttttat 1371060
gaaaaaccgt ctagaaatcg ccaaagagct gcttatgaaa gacggttcga tttttgtgtc 1371120
aattgacgac aacgaacagg catatttgaa aattttaatg gatgaagttt tcggaaatga 1371180
aaatttcatc tgcaatttta tttgggaaaa aaagacaggt gcgtccgatg ccaaacagat 1371240
agcgactatt acagagtttg tcttatgtta ctcaaagaac tttaaaaacag ttaaattaaa 1371300
taaaaacacg ttttcttatg atacagagag atacaaatta agtgataagt ttgaacagga 1371360
aagaggcaaa tattatatcg acaatttaga tagaggggga ttgcagtata gtgacagttt 1371420
gaattttgca atccaatgtc cagatggcac ttttacgtat ccgaatggca ggactgaatt 1371480
tgtcaatgat ggctggatat ggaaatggag taaaaataaa attgattggg caataacaaa 1371540
cggttttttg gagtttagaa aatcaaagtc taaaaaatct ggatggagtg tatgttataa 1371600
aaactatatg ttggttgata acgaaaacaa gccgatagaa cgttctgctc cctataagaa 1371660
cttaatacag gatatcttaa atacacatgc gacagatgaa ttgaaaaaac tgttcggcag 1371720
caaagttttt actactccaa aacctgagag cttattgcag tatcttattc aaattgccac 1371780
atccgaatcc gacatcgtct tagactacca tcttggtagt ggcacaaccg ccgccgttgc 1371840
ccacaaaatg aaccgccaat atatcggtat tgaacaaatg gattatattg aaacgcttgc 1371900
tgttgaacgc ttgaaaaaag tgattgatgg cgagcaaggc ggtatttcca aagccgtgaa 1371960
ttggcaaggt ggtggcgagt ttgtttatgc cgaacttgcc ccatttaacg aaaccgcaaa 1372020
acaacaaatt ttggcttgcg aagattcaga cggcatcaaa acgctgtttg aaggtttatg 1372080
cgaacgctat ttcttgaaat acaacgtcag cgtaaatgaa tttagtcaaa tcattcaaga 1372140
gcctgaattt caatctttgc cattagacga acaaaaacaa atggtgcttg aaatgttgga 1372200
tttaaatcaa atgtatgttt cattatccga aatggatgac gaacaatttg cagattgcct 1372260
gaacgatgat gataaagcct taagccgtgc attctatcaa tcagtaaaaa atcaagcgga 1372320
gaaaaaagat ggcgaataat aaaacgttgt ttgaagtgat tgaaaatgaa cgtaaagcgg 1372380
ttaaaaaata caagcctgaa ttacttgaaa tgccagaatt tacgtccaaa aacttaaaat 1372440
atgatttttt tgaatggcaa aaatctgccc ttgaaaactt tttgattttt gaccgcactt 1372500
caaagctaga cgatttccct gatttaaaaa ataagccaac gcatttgctg ttcaatatgg 1372560
caacaggtgc tggcaaaacg atgatgatgg cggcgttgat tttgtattat tttgaaaaag 1372620
gttatcggca ttttctgttt tttgtgaatc aaaacaatat cgtggataaa acggaaaata 1372680
attttaccga tccgacgcac gcaaaatttt tatttaccga gaagattttg cagggcgata 1372740
cggtaattcc tattcgcaaa gtggagacat ttagcccaca ttcagacggc attgaaatta 1372800
aatttaccag cattcaaaag ctgtataacg atattcgcac ccggcgggaa aatcaaacca 1372860
cattggcgga tttgcacaaa ttgaaccttg tgatgctggg tgatgaagcg caccatttaa 1372920
acgcgcaaac caaaggcaaa aaacaaggcg aattagattt agaaaaggaa atgaacgacc 1372980
gcaccagcaa tgccgaaatt gaacgtaaag gctgggagca tatggttttg gaattgttac 1373040
tcaataaaaa tggcaatcat agccaaaatg tgctgttgga atttaccgcc acgctgcctg 1373100

aaaatgccga tgtacaacaa aaatacgctg ataaaatcat cacaaaattt ggcttaaaag 1373160
aatttttgca aaaaggttat accaaagaaa tcaatttggt atccagtacg ctgggtaaga 1373220
aagagcgagt gttacacgct ttattgtttg cttggtatcg acatcgaatt gcgttgaaat 1373280
atggcattgc caatttcaag cctgtgatgt tgtttagaag taagacgatt gatgaatcaa 1373340
aagcggatta tctggcattt ttaaattggg cagaaaatgt gcaggcggtt gatttttcgt 1373400
ttttaactac attttcaaca agcttgaacg atagcgatag cgataacgcc aacgaacaag 1373460
gcaaaacccg cactgaacaa gccctaaaat ttatgcagga aaaaggcgtt gagtttgcac 1373520
atttggcaga ttgggtaaaa caaaattatc aaaaacacaa tgtgattatt accaactccg 1373580
aaaccaacaa aaccaaaacc gaaaaaaccg acagcgaaac agaaaaaattg ctgaataatt 1373640
tggaagcggc tgataatccg attcgtgcca tttttacggt ggacagatta accgaaggtt 1373700
gggacgttct gaatttgttt gatattgtgc gtttgtatga agggcaaaac ggcggcggtt 1373760
caaataaaaa atcaggcaaa acggctgccg ctactgtatc ggaaaagcag ttgattggtc 1373820
gcggcgtgcg ttattttcca tttgcgtttg aaggtaaaca gccgaataaa cgcaaatttg 1373880
acaacgatat gcaacacgaa ttgcgtattt tggaagaatt gttttattac acgcacgatg 1373940
agcaatctcg ctatattaca gaactgaaaa acgagttacg aaaagacggt tatttgcctg 1374000
aaaaagacga tgataaggta ttggcaacat ttaaactcaa atctgaattt gccgataatc 1374060
aggattttag agagttgtta atttgggcaa ataaaaaaat ccccaatccc aatgccagag 1374120
ccaataatgc agacagcctg aaagccaatc cgcaaacgct tccattccaa gttcacggca 1374180
atcaactgtt gcaggaaacg caatttacag ccgatgaaaa tgatgaaata gcccgacaaa 1374240
tcgacacaca aaataatttt actcaaatca taaaaatgag tgaaatggaa cggcacattt 1374300
tcaataaatc cctgcatatc aaaggaaaaa atggtcaatc tttattccat tttgaccgct 1374360
tgcaaagcaa actcaacatt tacaatcgca atgaattgca aaataacttg ttaaaagatt 1374420
gacaaattga atttttggga ttagggcaag acaaacagat cagcccagat gacaaacttg 1374480
caggctgcct aaaaatcttg gaaatggttg aaaaacattt gaatgaaagt gatatgccat 1374540
ttatcggtac aaaagaattt acgcctaaaa aattgtggga aatttttggc acaccaaaac 1374600
aaaaatgggt caaaaaagat gatataaaaa ctgccattgc cacgcaaaat gattggtatg 1374660
tgatggataa ttttgctgga acgagtttgg aagaagcgtt aattcaattt atttcagagc 1374720
atttgggcga tttgaagtct aaatatgatg ttcatttaat ccgtaatgaa gaagtgttta 1374780
aattgaataa cttttccgat ggtgaaggat ttatgccgga ctttatttta ttgctgaaaa 1374840
ataaacaaaa atcttcttcc aatggtgtgg atgacttttt gcattaccaa attttcattg 1374900
aaccaaaagg tgagcatttg gtggaaaatg attcgtggaa agacgctttt ttaaaggcaa 1374960
ttacagcgga atacgggacg gataagattc tgcaaaaaga tacaccgcat tatcgtttga 1375020
tcggtttgcc gtttttttact gacaatcagg aaaatgaaca atttacaaag tcattccctt 1375080
taggggcggc atcgcttgaa aaatagagtg gtgcattgca ggcaaccccg tttgacaaaa 1375140
cttcctttac aaaagggcgt tttgtcagat atttaatcaa cacattatta aaatacagcc 1375200
aaattttaat gccgtccgaa ccctgtgttc agacggcatc gtatttttca gtatctaaac 1375260
cgtttccctg ccccaatctt tgcctctcaa aatcgaagca tcgacatctt gaatatcgcg 1375320
gtgtcccgta aacgccatag atatatccat ttctttatac aggatttcca gcgcacgggt 1375380
tacgccttct tctccatacg cgcccaagcc atacaggaac gcccgaccta tcattgtacc 1375440
tttcgcgccc aaagcccacg ccttcaaaat atcctgaccg ctgcggatgc cgctgtccat 1375500
ccaaacttcg atgtcgctgc ccactgcgct gacgatgtcg ggcaaggctt tgatggcaga 1375560
cacggtatcg tcgagctgtc gaccgccgtg gttggaaaca atcaatgcgt ccgcgccgct 1375620
tttcgctgct ttttccgcgt cttcaggttc cataatgcct ttgataatca gcttgccgcc 1375680
ccacaaatct ttaatgcgcg ccacatcgtc ccagctcagg cgcgggtcga attgttcgga 1375740
agtccatgaa gacagcgaag acaaatcgcc gacgttcttc gcgtgtccga cgatattgcg 1375800
gaacgtgcgg cgttccgtgt tcagcatttt catacaccat tcgggcttgg tcgccagatt 1375860
gattaaattg gcgatggtcg gtttcggcgg cgcggacagg ccgtttttga tgtctttgtg 1375920
gcgttgcccc aaaacctgca aatcagcggt caataccaat gccgaacatt tggcatcctt 1375980
cgcgcgctta atcaggtttt ccataaactc gcggtcgcgc atcacataaa gctgaaacca 1376040
aaatggtgcg gaagtgttct cggcaacgtc ttcaatcgag cagatagaca tcgtggacag 1376100
cgtaaacgga atgccgaact tctccgccgc ccgcgccgcc aaaatttcac cgtcggcgtg 1376160
tgccataccc gtgaaacccg tcggcgcaat cgccaccggc attttcacat cctgcccgat 1376220
cattttggtt tccaggcttc ggccttccat attgaccaat actttttgac ggaagcggat 1376280
gtctttgaaa tccgaagtgt tttcacggta ggtagtttct gtccacgaac ccgaatcgat 1376340
gtaatcgtaa aacatacgcg gcattttgcg cttggcaacg cggcgcaagt cttcgatgca 1376400
ggtcattttg ctcaaatcac gtttcatttg tcgccccctg aatacctgaa taactttata 1376460
tgaaatcgat aatgtatatc aatattgatt ataaggcaaa tcatttcaac atttgccgca 1376520
tccgccgcag ctccctactt taagcgacat aaggtttaaa attcaaaaat aacaaattaa 1376580
aatcaaaata ttaaaaatca atcaatctat cgatttaaac agccaatcac acaatccgcc 1376640
ctcatacttg actgaaacac tcagatattg gacaattcca cccactaata aaaaaaccga 1376700
catgggcaac caccaccatg agactgacca ccaaagggcg tttcgccgtt accgctatgc 1376760

tggatttggc gatgaacgcg caaaccggcg ccgtcaaact cagtgccatc agcgaacgcc 1376820
aaaacatatc cctctcctat ctcgagcaat tgttcggcaa actccgccgc gccggacttg 1376880
ttgaaagcct gcgcgggccc ggcggcggct acatcctcgc cgcaccggcg gcacgcatca 1376940
acatcgccca aatcatcgcc gccgccgaag accggctgga cgcaacccaa tgcggcagca 1377000
aagccaactg ccaccacggc gcgccctgcc tgacgcacga tctttgggag aatttaaaca 1377060
aaaccatcaa cgactacctc ggcagcgtta ccctgcaaag catcatcgaa cagaaaaaca 1377120
acggcgacgg cagccgcgtc gtccaattta cacacatcca ttaaataaca cccgaaaaag 1377180
aaagagcaaa ccatgaccgt caaaaccccc gtttacctcg actacgccgc cacccccccc 1377240
gttgacaaac gcgttgccga aaaaatgatt ccctatctga ccgaaacctt cggcaaccca 1377300
gcctccaaca gccacagctt cggctgggaa gcagaagaag ctgtagaaaa agcacgtgca 1377360
gacattgccg ccctgattaa cgccgactct aaagaaatcg ttttcaccag cggcgcaacc 1377420
gagtccaaca acctcgctat caaaggcgcg gcgcacttct acaaatctaa aggtaatcac 1377480
ctcatcactg taaaaaccga acacaaagcc gtactcgaca ccatgcgcga actcgaacgc 1377540
caaggttacg aagtaactta tctggacgta caagaaaacg gtttggttga tttagacgta 1377600
ctgaaagccg ccatccgcga agacaccatc ctcgtttccg taatgtgggt aaacaacgaa 1377660
atcggcgtgg ttcaagatat tcctgccatc ggcgaaatct gccgcgaacg caaaatcatt 1377720
ttccacgttg acgcagcaca agcatgcggc aaagtgcctg ttgatgttga agccgcaaaa 1377780
gttgatttgc tgtctatgtc cggccacaaa gtatacggcc ctaaaggcat cggcgccctg 1377840
tatgtacgcc gtaaaccacg cgtccgcctc gaagcccaaa tgcacggcgg cggtcacgaa 1377900
cgcggtttcc gttccggcac attgccgacc catcaaatcg tcggcatggg tgaagccttc 1377960
cgcattgcca aagaagaatt ggcacaagac actgcacact acctgaaact gcgcgatatt 1378020
ttcctcaaag gtatcgaagg catcgaagaa gtctatatca acggcgacct cgaacatcgc 1378080
gtcccgaaca acctaaacgt cagcttcaac ttcgtcgaag gcgaaagcct gattatggca 1378140
gtgaaagaac tcgccgtatc cagcggctcc gcctgcacct ccgcctcgct cgaacccagc 1378200
tacgtcctgc gcgcgctcgg ccgcaacgat gaactggcgc actcatccct gcgcatcacc 1378260
ttcggtcgca tgaccaccga agaagaagtg caattcgccg ccgaactgat taaatccaaa 1378320
atcggcaaac tgcgcgaact gtcgccgctg tgggaaatgt tcaaagacgg gattgatttg 1378380
aactcgattg aatgggcagc gcattaaagc gtaccaacat gccgtccgaa cctttagacg 1378440
gcattccaaa aacaaagcaa tcaagagaaa atatgaacga acaagattta gatttggaca 1378500
atctcgacaa cctgcttgaa gattttgacg gcgttaccgt ggaaggcggc gtcgattcgg 1378560
aaaacgacga cggctgcgaa ggcggggcgt gcaaaatcta aacttcaggc cgtctgaaaa 1378620
tcggcaaaca aaccacttaa accatcaaaa aacattaagg aaaccacatc atggcataca 1378680
gcgataaagt aatcgaccac tatgaaaatc cgcgcaacgt cggcacattc gacaagggag 1378740
acgattccgt cggcaccggc atggtcggcg cgcccgcctg cggcgacgtc atgcgcctgc 1378800
aaatcaaagt gaacgacgag ggcatcatcg aagatgcgaa atttaaaact tacggctgcg 1378860
gctcggccat cgcttcgtcc agcctgatta ccgagtgggt taaaggcaaa agcctggatg 1378920
acgcgctggc aatcaaaaac agcgaaatcg ccgaggagtt ggaattgccg ccggtaaaaa 1378980
tccactgctc catcttggct gaagatgcgg taaaagcggc cgttgccgac taccgcaaac 1379040
gtcaggaaaa cagataaagc ccttcagacg gcatcatccc gcaatgccgt ccgaaccacc 1379100
cgccgcttca ggtccgtccg gggcggtaca acaaggaaga aatatgatta cccttaccga 1379160
gaatgccgca aaacacatca atgactatct cgccaaacgc ggcaaaggct tgggcgtacg 1379220
cttgggtgtg aaaaccagcg gctgctcggg gatggcgtac aaccttgaat ttgtcgacga 1379280
agccgatggc gacgacctga ttttcgaagg acacggcgcg cgcatttata tcgatccgaa 1379340
aagcctggtt tatctggatg gcacgcaagt cgattacacc aaagaaggtt tgcaggaagg 1379400
tttcaaattt gaaaacccca atgtcaaaga ctcctgcggc tgcggcgaaa gcttccacgt 1379460
ttaaggcata aaaacggcgg gaccgtatca aaaccgtccc gccatttttt cgcttcctgc 1379520
ctgttgtagc tgcctttgcc tttccttttc cgttccacct tgtgccggaa caaatcggat 1379580
ttcactaagg cttttaaagc attgtcgcgt attttgcctt tattgtgctg cactttgccg 1379640
cccatattca gtcctttcgt ttaagaagcg gcagattata aggcaaaaac agttttctgc 1379700
caaaatctta catttatcat cctactatgt cccaatattt caccctcttc cggattgaac 1379760
ccgctttcga tatcgacacc gaaaacttgg aacaaaccta ccgcgccttg gccgcccgtt 1379820
tccatcccga taaattcgct tcagcttccg cctttgagca aaagcaggca gtgatgatgt 1379880
cttccaccat caacgatgcc taccgcacct tgaaaaaccc catcgaccgc gccgcctacc 1379940
tgctgaaaac atcgggcatc gatgccgacg cgccggagca taccgctttc gcccccgaat 1380000
tccttatgca gcaaatggaa tggcgcgaaa cgctgatgga ggcacgggca ggcaacgacc 1380060
ttgaatcctt gaaaaatctc gacaacgaaa tccgcgacga acaagaaaaa ctgttctgcg 1380120
gtctgaaaca gtcgtttgcc cgacaagatt acgacacagc cgcacaacaa gtccgccaag 1380180
gcaggtttct cgacaaactc cgcaacgaaa tttcctcggc attataatcc gcaccgtgtt 1380240
tcagacggcg taaccgccgc accgttccgc gtcaaaatat gctaaaataa gcaacaattt 1380300
tttgccatac gaaacattga aaccatgacc gacgcaacca tccgccacga ccacaaattc 1380360
gccctcgaaa ccctgccggt aagccttgaa gacgaaatgc gcaaaagcta tctcgactac 1380420

gccatgagcg tcattgtcgg gcgcgcgctg ccggacgttc gcgacggtct caagccggta 1380480
caccgccgcg tactgtacgc gatgcacgag ctgaaaaaca actggaatgc cgcctacaaa 1380540
aaatcggcgc gcattgtcgg cgacgtcatc ggtaaatacc acccccacgg cgataccgcc 1380600
gtatacgaca ccatcgtccg tatggcgcaa aatttcgcta tgcgttatgt gctgatagac 1380660
ggacagggca acttcggatc ggtggacggg cttgccgccg cagccatgcg ctacaccgaa 1380720
atccgcatgg cgaaaatttc ccacgaaatg ctggcagaca ttgaggaaga aaccgtcaat 1380780
ttcggcccga actacgacgg tagcgaacac gagccgcttg tactgccgac ccgtttcccc 1380840
acactgctcg tcaacggctc gtccggcatc gccgtcggca tggcgaccaa tatcccgccg 1380900
cacaaccttt ctgataccgt caatgcctgc ctgcgcctgc tcgatgcacc cgacaccgaa 1380960
atcgacgaac tgatcgacat tatccaagcc cccgacttcc cgaccggggc aaccatctac 1381020
ggcttgagcg gcgtgcgcga aggctataaa acaggccgcg gccgcgtcgt tatgcgcggt 1381080
aagacccata tcgaacccat aggcagaaac ggcgaacgcg aagccatcgt tatcgacgaa 1381140
atcccctatc aggtcaacaa agccaagctg gtcgagaaaa tcggcgattt ggttcgggaa 1381200
aaaacactgg aaggcatttc cgagctccgc gacgaatccg acaaatccgg tatgcgcgtc 1381260
gttatcgagc tgaaacgcaa cgaaaatgcc gaagtcgtct taaaccaact ctacaaactg 1381320
actccgctgc aagacagttt cggcatcaat atggtggttt tggtcgacgg acaaccgcgc 1381380
ctgttgaacc tgaaacagat tctctccgaa ttcctgcgcc accgccgcga agtcgttacc 1381440
cgacgtacgc ttttccggct gaagaaggca cgccatgaag ggcatattgc cgaaggcaaa 1381500
gccgtcgcac tgtccaatat cgatgaaatc atcaagctca tcaaagaatc gcccaacgca 1381560
gccgaggcca aagacaaact gcttgcgcgc ccttggcgca gcagcctcgt tgaagaaatg 1381620
ctgacgcgtt ccggtctgga tttggaaatg atgcgtccgg aaggattggc tgcaaacatc 1381680
ggcttgaaag agcaaggtta ttacctgagc gagattcagg cagatgctat tttacgcatg 1381740
agcctgcgaa acctgaccgg cctcgatcaa gaagaaattg tcgaaagcta caaaaaacctg 1381800
atgggtaaaa tcatcgactt tgtggatatc ctctccaaac ccgaacgcat tacccaaatc 1381860
atccgcgacg aactggaaga aatcaaaacc aactatggcg acgaacgccg cagcgaaatc 1381920
aacccgttcg gcggcgacat tgccgatgaa gacctgattc cgcaacgcga aatggtcgtt 1381980
accctgacac atggcggcta tatcaaaacc cagccgacca ccgactatca ggcgcagcgt 1382040
cgcggcgggc gcggcaaaca ggcggctgcc accaaagacg aagactttat cgaaaccctg 1382100
tttgttgcca acacgcatga ttatttgatg tgctttacca atttgggcaa gtgtcattgg 1382160
attaaggttt acaaactgcc cgaaggcgga cgcaacagcc gcggccgtcc gattaacaac 1382220
gtcatccagt tggaagaagg cgaaaaagtc agcgcgattc tggcagtacg cgagttcccc 1382280
gaagaccaat acgtcttctt cgccaccgcg cagggaatgg tgaaaaaagt ccaactttcc 1382340
gcctttaaaa acgtccgcgc ccaaggcatt aaagccatcg cgctcaaaga aggcgactac 1382400
ctcgtcggcg ctgcgcaaac aggcggtgcg gacgacatca tgctgttctc caacttaggt 1382460
aaagccatcc gcttcaacga atactgggaa aaatccggca acgacgaagc ggaagatgcc 1382520
gacatcgaaa ccgaaatttc agacggcatc gaagatgaaa ccgccgacag cgaaaacgca 1382580
ctgccgagcg gcaaacacgg tgttcgcccg tccggtcgcg gcagcggcgg tttgcgcggt 1382640
atgcgcctgc ctgccgacgg caaaatcgtc agcctgatta ccttcgcccc tgaaaccgaa 1382700
gaaagcggtt tgcaagtttt aaccgccacc gccaacggat acggaaaacg caccccgatt 1382760
gccgattaca gccgcaaaaa caaaggcggg caaggcaata ttgccattaa cactggcgag 1382820
cgaaacggcg atttggtcgc cgcaaccttg gtcggcgaaa ccgacgattt gatgctgatt 1382880
accagcggcg gcgtacttat ccgcaccaaa gtcgaacaaa tccgcgaaac cggccgcgcc 1382940
gcagcaggcg tgaaactgat taacttggac gaaggcgaaa ccttggtatc gctggaacgt 1383000
gttgccgaag acgaatccga actctccgac gcttctgtaa tttccaatgt aaccgaaccg 1383060
gaagtcgaga actgaaaatc atctcccgat gccgtctgaa gattcagacg gcatttattt 1383120
tatccctcat ccgtcatcca gcttctcaca atatagcgga ttatagtcaa ttaaaaacaa 1383180
ggggctgtcc tagataacta gggaaattca aattaagtta gagttgcccc tatgagaaaa 1383240
agtcgtctaa gccggtataa acaaataaac tcattgaact gtttgtcgca ggtgtaactg 1383300
caagaacgac agcagagtta gtaggcgtta ataaaagtac cgcagcctat tattttcatc 1383360
gtttacgatt acttatttat caaaacagtc cgcatttgga aatgtttgat ggcgaagtag 1383420
aagcagatga aagttatttt gctgaacgac aaaaccatat caatggaatt gagaactttt 1383480
ggaaccgggc aaaacgtcat ttacgcaagt ttgacggcat tcccaaagcg cattttgagc 1383540
tgtatttaaa ggggtacgaa cggcgtttta acaacagtga gataaaagtt caaatttcca 1383600
ttttaaaaca attagtaaaa tcgagtttat cctagttatc taggacagcc ccaaaaacaa 1383660
aatagtacaa tattcaactt tgaaggtcta accatggcat actctgcgga cttaagaaac 1383720
aaagctttaa actatagtgg attaaattta aatcaggaca aggcgacgaa gccgcagaca 1383780
gtacaaatag tacggcaagg cgaggcaaca ccgtactggt ttaaatttaa tccactatat 1383840
tacgaacaat gcaaaaacat cagccaaacc gcagcaacgt ttaacttgtc aagaaacacg 1383900
ctttacctgt ggattcgcct taaaaaacaa acaggcagcc taaaacatca agttaccggt 1383960
ctaaatgccg tcaaatcgga taggcaaaaa ccggctcaat atgttgggca acacccggat 1384020
gcctatctgc atgaaatcgc caaacatttt gattgtacgg cagccaccat ttgctatgca 1384080

ctcaaacaga tggggataac gcgcaaaaaa agaccaccac ttacaaagaa caagacccgg 1384140
ccaaagtaac gcattatttg acacagccgg ccgaattttc cgactaccaa cgtgtttatt 1384200
tggatgaaac aggatttgac cgctacctgt tccgtcccta tgcccgcagc ctgaaagggc 1384260
aaatagtgaa agcgcagata agtggaaaaa gatatagtgg attaacaaaa atcaggacaa 1384320
ggcgacgaag ccgcagacag tacaaatagt acggaaccga ttcacttggt gcttcagcac 1384380
cttagagaat cgttctcttt gagctaaggc gagccaacgc tgtatcggtt taaatttaat 1384440
tcactataaa aacgacaaaa acgcaaaagc cgccgacatt cccgcatcca agtttcagtc 1384500
aatcagataa ccttggattt ctttggtttt cgcattgatt tctctggtac ggcagtcaga 1384560
ttgtgccacg ccgtattcgt cgccgtcggc gcatttggca ttcaaaccgt tttgtcagtt 1384620
gcggtactcg ggcatgacgg tttcggcaat atcggcgggc agtgcctgaa ccgcgctgtt 1384680
cagagccgct ttgacagttc tttcttgtcc gcttccctgg ccatttcgtc gataagggct 1384740
tttttacggt tgtgcagctc ttccaagcgt gcttcggttt tcgccgtttt gcatgccagt 1384800
tcgctgaatt ttatgccgtt tggcgtttta ccttcagctt tcgcattgtt ggcacagatt 1384860
ttatccatcc cgcttttcca tgttttctgt gaggcttgca gcttattctg tacggtttga 1384920
ggcaatcctt tccagaactg ctcgaactcg ctgcgcgaca gcttgtagcg ttcttcccat 1384980
tcggatacgc gggcggcttc ctgctcccga cccaatgcct cctgcgccgc cgcttcttct 1385040
gccgcttcaa gttcttcgtt cctttgtttc actttgtcca acggctcttt aatcagtgcc 1385100
atagctgcgg aacatatatg tttaaattta tgcaaaccat catatcggga ttgcacacgc 1385160
tctgcaagtt taccgacggt tttcctgttc gataaaaatg ccgtttgaaa cggtcggcgt 1385220
tcagacggca tttttccgca ggttttattt gcggttggtc tgcaggtaga ggttgatcag 1385280
gcgttcggtc gaactgtcgt gcttttgcgg cccggtttcg ccggtcagtt cgcccaaaat 1385340
ggttttagcc agttgtttgc cgagttccac gccccactgg tcgaagctgt tgatgcccca 1385400
aatgatgcct tgtacgaagg ttttgtgttc gtacatggca atcaggctgc ccatattgcg 1385460
cgggttgacc ttgtccatga gaatgaggtt ggtcgggcgg ttgccggaga aggttttgtg 1385520
cgggaccagc tcttcgatgc gcacctcatc cataccctgc gctttgagtt cggcgcggac 1385580
ttcgtcgggg gttttgccgc gcataaaggc ttctgcttgg gcgaagacgt tggcaagcag 1385640
gatttcgtgg tgtccgggca ggttgctgcg tttttcaagc gaggcaatca ggtcgatggg 1385700
ggtaatgtgc gtgccttggt gcagcagttg gaaaaaggcg tgctggccgt taatgcccgt 1385760
ttcgccccag ataatcggcg aggtttcgtg tccgactgct ttgccgtcca acgtaacctg 1385820
tttgccgtta ctttccatat cgagctgctg gatgaatttg ggcaggcggt gcaaatgttg 1385880
gtcgtaaggc gcgatgacgt ggctgccgcc gccgtagtag ttgatatacc agatgccgat 1385940
gagggcgaga atgacgggca ggttgcgctc gagcggtgtg ttgatgaagt gttggtccat 1386000
caggtgcgcg ccgttgagca tttcaatgaa gttttcttcg ccgagataca gcataatcgg 1386060
caatccgatg gcggaccaca ggctgtaccg accgccgacc caatcccaaa attcaaacat 1386120
attggcggtg tcgatgccga attcggcgac ggctttttga ttggtggaaa cggcggcgaa 1386180
gtgtttggca acggcttctt cgtcgcccgc atgattcaaa aaccattcgc gcgcggtcag 1386240
cgcgttggtc agcgtttcct gcgtggtaaa tgttttggag gcgatgatga acaacgtggt 1386300
ttcggggtgg actttggaca atacgtcgcg cagttgcgag ccgtccacgt tggagacgaa 1386360
gtgcatattg aggcgcggat gaccgaaagg tttgagcgcg gtacacatca tcagcggacc 1386420
caaatccgat ccgccgatgc cgatgttgac aacgtcggta atgacttggt tggtatagcc 1386480
cagccagctt ccgctgcgga cttcgtgtgc aaattcgccc atacgttgca aaacgcggtt 1386540

gactttgggc atcacatctt caccgtcaac cacaatcggc gaattggtgc ggttgcgaag 1386600
ggcgacatgc aggacggcgc ggttttcggt ggtattgatt ttttcgccgt ggaacatctg 1386660
ccgcatccgc tccggcacgc ctgcttctcg ggcaagctcg aacaaaagcg acatggtttc 1386720
gtcgttgatg cggtttttgg agtagtccag cgtcagtccg ccgacttgca gccagtagcg 1386780
ttccgcacgc tgcgggtctt gctcgaacat ttcgcgcata tgcaatgttt tgctgtcgtc 1386840
aaagtgattc cacaatttcg accatgcggg taagtcgtga aggtgtttca tctatatgct 1386900
cctgaatgag gttttttgtt gtgggatgaa aaggctgccg gaaactgccg caagccgccg 1386960
acgaccgttg ttcggcattt cagacggcat ttgtgggatg ccgtctgaag gtcaatcttt 1387020
gtcgtaatcg atgtgcttgt tgtgtatgct ttttttgctt ttctgcaatt gcaggctggc 1387080
agcatcgccc aagcgcaggg caagtccgat ggcgagaatg tcgatgacgg caagctgcaa 1387140
gaggcgggaa accatgggcg tgtagagttc ggcattttcc tgtgtggcaa cgctcaacac 1387200
gcagtcggca agttgcgcca gaggcgaatc gttgcgggtc agtgcgatga cagacgcgcc 1387260
gttttctttg gcgatgctga ccgcatccaa aagttcgata gacgaacccg tgttggaaat 1387320
ggcaaccaaa acatcctgat cgctcaaaac agatgccgcc atcagctgcg tgtgcgtatc 1387380
gacataggcg acggtggaca tgccgaaacg gaaaaattta tgctgcgcgt cctgtgccac 1387440
aatgccggaa ttgccgacac cgtaaaactc gacgcgacgg gcgtgcatca gcgtggcaat 1387500
ggcgttttcc agctccgact ctttcaggaa gcggcgttcg cccaacagcg aggcggcggc 1387560
attgcccaac actttctcga ccacgcttgc catatcgtcg tcggcgttga gttcttcgtg 1387620
gacatagggc ataccctcat gaccgatgct ggcggacaag gcgagcttga actcgggcag 1387680

```
ccctttataa cccaagctgc ggcagaatcg gatgacggtc ggctggctga cggacgcacg 1387740
ttcggcaatt tcggcaacgg cggcatggac gaaccatttg ggttccgcca atgcacattc 1387800
ggcgactttg cgttccgcac cggaaaggtt tgccagtgat tcgctgattt tgcttaacat 1387860
aatgatatgc ccttcgataa tgcagccccg ctgcaaggag ccgctgtggt taaacgtttc 1387920
tcaaatggtt gtcaagagcc gcagccgcac cggaaattcc gggaaactcg ctcaagacga 1387980
catacacggg aatcgcggca agatatgctt caaacctgcc cttgttctcg aaacggctgc 1388040
ggaacgggga agttttgaaa tattccaaca cgcggggaat aatgccgcca cacaggtaca 1388100
cgccgccgcg cgcgcccagc gtcagggcga ggttggaagc aaccgtgccg agcatggcgc 1388160
agaagatgtc caaagtctga cggcacaaag gcgacgcgcc gctcaaagcc ttttccgtga 1388220
tttcagacgg catcagtttg gcgggtttgg ctttctgttt tgcagccaaa gcctcgtaaa 1388280
ccaagctcaa gcccgcgccg ctcaaaaagc gttcggcgga aacatggccg tatttgtttt 1388340
tggcgtactg ccaaatcagc acttccatat cgtcaaacgg cgggaaactg gtatgcccgc 1388400
cctcgcccgc caaagccacc cagcctgcgt ggctgtgcac caatccgctc acgcccaggc 1388460
cggtaccggg gccgataacg gctttggggg caaattcgac aggcttttgc ccgcctacct 1388520
gcatcaggtc tttgcttgaa gtctgcgtta ccgccaatgc ctgcgcggta aagtcgttca 1388580
aaaggatgag ggtgtccagc cccaaagtct gacgggtggt ttcgatggaa aacgcccaat 1388640
ggtggttggt catctgcacc cagtcgccca aaatcgggtt ggcgatggca aatgccgcgt 1388700
gccgtacggc tgttgcaccg ctttgattca gataggcacg caccgcatcg gtaaccgtat 1388760
cgtagtcttt acacggaagc acggcggctt tttcaatgac gcgcggcgcg gtttccagcg 1388820
caaagcgtgc attcgtcccg ccgatatcgg cgaccagtcg gggatatccg gcttgtttat 1388880
tcggcgtaga agacatggca gttcactcct tgatggttca aaacgaggtt gatcggatat 1388940
tcgcggtttt cgccttgtgc ggcttggtcg aacacggctt ttttctcttc gccccgtatc 1389000
gccaaaaaca catgccccgt atgggcaatc gcatccaagg tcatactgac gcgctcgtgc 1389060
ggcgcggtaa cgggcgtggt atgcaccaac gcgacacctg ccgaaccgtc gattgccgtc 1389120
tgaaactgcg gagctttcgg gaaaatcgaa gccgtatgcc cgtcgtttcc catacccaaa 1389180
accaaaaacat cgggctgttt gtaatgtttc agtgcataat cgacaacagc atcgggatgt 1389240
aattcggttt cagtttttcc gtcttccacc ataggaatcc acattgccgc ttccgctttg 1389300
ttcttcaaca ggtattcgcg caccaaaccg gtattgctgt cggcgtggac ggtcggcacg 1389360
atgcgttcat ctgccaaggt gatgccgacg tttttccaat ccaaatcttt ttgcgacagg 1389420
gcgttgaaaa atgcaatcgg cgaacgtccg ccggaaactg ccaacaccgc accgcccttc 1389480
tcgtccagtg cgccctgcaa agcatccgcc actgcgtcag ccaaagactg cgccgcttct 1389540
gccgcatttt cgtattcgtg ccaaacaaac atatttgtgt cctttttttta tttcagacgg 1389600
catattccgt tatggaaacg ggttgagcaa tatgtcggcc gaacagttgt ttatgctttt 1389660
gataccaaat atcgggactg ctttttatag tggattaaat ttaaaccagt acagcgttgc 1389720
ctcgccttgc cgtactattt gtactgtctg cggcttcgtc gccttgtcct gatttttgtt 1389780
aatccactat actactttac ttatgttcag acggcatttc aaaccccatg ccgtctgaac 1389840
gcattattgt attactgctc ttcgtgccac ttgtgtccgt cgcgcgccaa tagttcgcgc 1389900
gcggcttcag gcccccacga gtgtgcgccg tagccgtgcg gcggcgtggt gttatttgtc 1389960
cagttttcca aaatcggcat cacatattcc cacgcggctt caagttcgtc gcggcggtta 1390020
aacaaagcga gtttgccgtt aatcacatcc agcagcaggc gctcgtaagc ttccgcgcgg 1390080
cggccttcca atgctttgcc caaatcggtt gccagcggca cggtttcgac cttatttcct 1390140
gcccccgggg ttttcatctg cgtatagagg cgcacggatt catatggttg caactcgata 1390200
acgagccggt tgggcgcggt gcggctgcct tcaaaaatat ggctgttcaa atctttgaag 1390260
ttcaaaacga tttccgccac tttgcccgcc atgcgtttgc cggtacgcag gtagaaggga 1390320
acgcccttcc agcgttcgtt ttcgatttcg gctttaatgg cgacgtaggt ttcggtaaag 1390380
ctgtcttgcg gaacgttgat ttcttcaaga tagccgttca tgcctctggc ggcggtatat 1390440
tgtccgcgca cgacgttttc attgacagac tcgacggtca gcggcttcaa tgacttgatg 1390500
actttgactt tttcatcgcg caccgcgtcg gcatccaagc tggcgggggc ttccatcgca 1390560
gtcatgcaca acatctgcat caaatggttt tgcaccatat cgcgcaacgc gccggtaatg 1390620
tcgtaaaact caccgcgctc ttccacaccg agctgttcgg cgatggtcaa ctgcacgctt 1390680
tcgatatatt tattgttcca cagcggctcg aacattacat tggcaaaacg cagcgcaagc 1390740
aggttttgca ggctttcttt gccaaggtag tggtcgatgc ggtaaatttg cccttctttg 1390800
aaataacgcg caacatcggt attgatttgc tgggaagaag ccaaatccgt acccaacggt 1390860
ttttccaaaa ctacgcgcac attgtcggca ttcaaaccga tcgcagcaag gttttcacag 1390920
gcttgcgcga agaatttggg cgcggtggac agatagatga cgacgttgtc ggtttctttg 1390980
cgcgctttga ccaaatcgcc caaagcggca aaatcgtccg gctgcgtaac atcgactttg 1391040
agatatgcga aacgttcgac aaacgatgcc caagcctcat cggaaaaatt ttctttcaca 1391100
tggattttgg aactggtttc caccttcgcc agaaaacctt cggtatccaa ctcgctgcgg 1391160
ctgaccccca aaatacgccc ttcgggatga agcagaccgg caacatgcgc ctggtacaga 1391220
cagggcaaca gcttgcgcat cgccaaatcg ccggtcgcac cgaacaacac caaatcaaaa 1391280
tttgtttgtg tactcatcgt attatctcgt caggaaagaa tttttcgatg ccgtctgaaa 1391340
```

cctgtttccc ccatcacgct gcatcgcaat atcggaaaca aaggcaggcg gcataatgag 1391400
tagtaatact acacaccgct acactttttg tctattccca tttttacaat ttatttgacc 1391460
tagtccaaaa atcgggcagg tttcccctat tccgttacaa caatcgaaag attctgcgat 1391520
ttaaatcaaa tttcttttca atgcctgatt tttttgtaac aaaattacaa attttgtact 1391580
ataataacac ccgcttccca ctttcagacg gcataccttt taaaatatag tggattaaca 1391640
aaaatcagga caaggcgacg aagccgcaga cagtacagat aatacggaac cgattcactt 1391700
ggtgcttcag caccttagag aatcgttctc tttgagctaa ggcgaggcaa cgccgtactg 1391760
gtttttgtta atccactata cttaccgtct gaatacccga tacaaaaatc agaaacgcac 1391820
aaacaaatcc ccaatacccc ccccgttccg acaggagacc gaccgtgaac actactccta 1391880
tccactccaa actcgccgaa atcaccgggc gcattattga acgcagccgt ccgacgcgtg 1391940
aaaaatatct ggcgaagatc cgcagtgcca aacaaatggg acgcttagag cgcaaccagc 1392000
tcggctgcag caacttggca cacggctatg ctgccatgcc taaaagtatc aaaatcgaaa 1392060
tgcttcagga aaccgtcccc aacttaggca tcatcaccgc ctacaacgac atggtttccg 1392120
cacaccagcc gtttaaagac ttccctgacc aaatcaaaga cgaagcgcag aaaaacggcg 1392180
cgaccgccca agtcgccggc ggcacgcccg ccatgtgcga cggcatcacg caaggctacg 1392240
ccggcatgga attgtcgctg ttctcccgcg acgtgattgc catgagtacc gccatcgggc 1392300
tgtcgcatca aatgtttgac ggcagcctgt ttatgggcgt atgcgacaaa atcgttccag 1392360
gtttgatgat aggcgcgctt tcgttcggtc atattccggg tatcttcgtc cccgcaggcc 1392420
cgatgtccag cggtatcggc aacaaagaaa aagcccgcac ccgccagctt ttcgccgaag 1392480
gcaaggtcgg acgcaacgaa cttttgaaaa gcgaaatggg ttcttaccac agcccgggca 1392540
cctgcacttt ctacggcacg gcaaactcca accaaatgat gatggaaatg atgggcgtgc 1392600
acctgcctgc cgccgccttc gtccacccctt acaccgacct gcgcgaagcg ctgacccgct 1392660
acgccgccgg acacctcgcg cgcggcatca aaaacggcac gattaaacct ttgggcgaaa 1392720
tgttgaccga aaaatccttt atcaacgcct tgattggcct gatggcaacc ggcggttcga 1392780
ccaaccacac catgcacctc gtcgctatgg cgcgtgcggc cggcgtgatt ttgaactggg 1392840
acgacttcga cgaaatttcc tccatcatcc cgctgctcat ccgcgtttat ccgaacggca 1392900
aggccgacgt gaatcacttt accgcagcgg gcggactgcc tttcgttatc cgcgaattgc 1392960
tgaatgcagg cctgttgcac gacgatgtcg ataccgtcgt cggacacggt atgcgccact 1393020
acaccaaaga gcctttcctt atcgacggca aactcgaatg gcgcgaagcc cccgaaacca 1393080
gcggcaacga cgacatcctg cgcaaagctg acaacccgtt ctcccccgac ggcggtctgc 1393140
gcctgatgaa aggcaacatc ggacgcggcg tgattaaagt gtccgccgtg cgcgaaggct 1393200
gccgcattat tgaagcgcct gccatcgtgt tcaacgacca acgcgaagtg ttggctgcgt 1393260
ttgaacgcgg cgagttggaa cgcgattttg tgtgcgtcgt ccgctaccaa ggcccgcgtg 1393320
ccaacggtat gcccgaattg cacaaactga ccccgccttt gggcatcctg caagaccgcg 1393380
gcttcaaagt ggcgctgctg accgacggcc gtatgtccgg cgcgtccggc aaagttccag 1393440
cctccatcca catgacaccc gaagccctga tgggcggcaa catcgccaaa atccgtaccg 1393500
gcgacctgat ccgcttcgac tccgttagcg gcgaactcaa cgtcctgatt aacgaaaccg 1393560
aatggaatgc ccgcgaagtc gaaagcatcg acttgggcgc gaaccaacaa ggctgcggcc 1393620
gcgaactctt cgccaacttc cgcagcatga ccagcagcgc ggaaaccggt gccatgagtt 1393680
tcggcggcga atttgcctga tgcgcgtttc agacggcctt ttcagaccga aggccgtctg 1393740
aaaaattatt caagcgtttt aagatagacg taggttggat tctcgaatcc gacacagccg 1393800
tccaagatgt cggtttcttg aatccgacct acaacctgtc ccatcttaat aaaatacccc 1393860
attccacccg gagaaccgaa atgtccaaac tgaccccccg cgaaattttg accgccggcg 1393920
cagttgtgcc ggtaatggcg attgacgact taagcaccgc catcgatttg tcccacgccc 1393980
ttgtcgaagg cggcatccct accctcgaaa tcaccctgcg cacccctgtc ggcctcgatg 1394040
ccatccgcct gattgccaaa gaagtgccca acgccatcgt cggcgcaggt acggtaacca 1394100
atcccgaaca gctcaaagcc gtcgaagacg caggcgcggt tttcgccatc agcccggggc 1394160
tgcatgaatc cctcgccaaa gccggccaca acagcggcat ccccctgatt cccggtgttg 1394220
ccaccccggg cgaaatccaa ctggctttgg aacacggcat cgacaccctc aaactcttcc 1394280
ccgccgaagt cgtcggcggc aaagccatgc tcaaagccct gtacggccct tacgccgatg 1394340
ttcgcttctg cccgacaggc ggcatcagcc tcgccaccgc gcccgagtac ttggcactgc 1394400
ccaacgtcct gtgcgtcggc ggctcttggc tgacaccgaa agaagccgtg aaaaacaaag 1394460
actgggacac catcacccgc ctcgccaaag aagcggcggc gttgaaaccc aaagcctgat 1394520
tcgcatcgta aaaatgccgt ctgaaaaacc tttcccgttt cagacggcat tttgccgatt 1394580
gagggcacag tcggcataca cggcagcact gatcagacat accgccccta aaatgcccat 1394640
ccgccttccg cataataaaa ataacgttca gttcattcga cagcagccgg acagcccata 1394700
ctacgcggct gaaaaaatgc cgtctgaaac gcattcagac ggcatccact taaaaaaaac 1394760
aactgattca acgccgatta atccgcttcc aaaaccactt tcatcacttg gttttcggcg 1394820
gcgtgtttga acacgtcgta ggctttttcc aattcactga atttgaaatg atgggtcagc 1394880
attttggtgt aatcgacgga gctgctggaa atcgccttca tcagcatttc ggtggtattg 1394940
gcgtttacca gaccggtagt gatggcaagc tttttaatcc agagtttttc cagtttgaaa 1395000

tcaacggatt gaccatgtac accaaccaca gcgatatggc cgccgggttt cacaatgtct 1395060
tggcacatat tccatgtagc agggataccg acggcttcga tggcgcaatc cacgccgtct 1395120
tcgccgacga tggcaaagac ttgtttggat acttcgccgg aagcagggtt aatggtatgg 1395180
gtcgcaccca attctttcgc cagtttcaaa cggttttcgt ccatatcgca aacgatgatg 1395240
gcggcgggac tgtacagttg ggcggtcaac agggcggaca taccgacagg gcctgcccca 1395300
gcgatgaata cggtgtcgcc gggtttgaca tcgccgtatt gcacgccgat ttcgtgggcg 1395360
gtcggcaaag cgtcgctcaa caacagggcg atttcttcgt tgacattatc gggcagcgga 1395420
acgaggctgt tgtcggcata aggcgtacgg acgtattcgg cctgagtacc gtcaatcatg 1395480
taacccaaaa tccaaccgcc gttacggcag tgtgaataga gttgggtttt gcagttgtcg 1395540
caagtgcaac atttgctgac gcatgaaata atgactttat cgccgacttt gatgtttttt 1395600
acagcctcgc cgacttcttc tacaataccg atgccctcat gaccgagaat acggccgtcg 1395660
gcaacttcgg ggtttttgcc tttccaaata cccaaatcgg taccgcaaat cgtggttttg 1395720
acgattttca ccaccgcatc ggtcggatcg ataatctgcg gacggggttt ttcttcaaaa 1395780
cggatgtcgt ttgcgccgtg ataaaccatt gctttcatgc tgatactcct tgcttgttga 1395840
taaataattt caataccgca ataaagtttc tttatatgag ttatatgccc ctacaaaaaa 1395900
taagtcaata agaattattt tcacaatgtt atacaataac ataccgtttt aaatataaat 1395960
aaaaccaccg attgatatta atgaacacac ccatcccctt ctccgarcgg ctcatccgct 1396020
ggcaaaaaca acacggtcgc caccacctcc cttggcaggt caaaaaccct tattgcgtct 1396080
ggctttccga aatcatgctc cagcaaacgc aagtcgccac cgtgttggac tactatccgc 1396140
gcttcttaga aaaattcccg accgttcaga cgcttgccgc cgcgccgcaa gacgaagtgt 1396200
tgtcgttgtg ggcgggcttg ggctattaca gccgcgcgcg caacctgcac aaagccgcgc 1396260
aacaagtcgt caggcaattc ggcggcacgt ttccgtcgga gcgcaaagac ttggaaaccc 1396320
tctgcggcgt aggcagaagc accgccgccg ccatttgcgc cttctccttc aaccgccgcg 1396380
aaaccatttt ggacggcaac gtcaaacgcg tactctgccg cgtgttcgcc cgcgacggca 1396440
atccgcagga caaaaaattt gaaaactcgc tctggacact tgccgaaagc ctgctgccgt 1396500
ctgaaaacgc cgatatgcct gcctatacac aaggtttgat ggatttgggc gcgaccgtgt 1396560
gcaaacggac gaaacccttg tgccaccaat gcccgatggc ggacatctgc gaagcgaaaa 1396620
agcaaaaccg caccgccgag ctgccgcgca aaaaaaccgc cgccgaagta ccgaccctgc 1396680
cgctttactg gctgattgtc cgcaaccggg acggcgcgat tttgctggaa aaacgccccg 1396740
ccaaaggcat ttggggcggg ctgtattgcg tgccgtgttt tgaaagtttg aacgggcttt 1396800
ccgactttgc cgccaaattc tccctgacca tggcagatat ggacgaacaa accgccctga 1396860
cccaccgcct gacgcaccgg ctgctattga ttacgccctt tgaagcacaa atgccgtctg 1396920
aaagcccttc agacggcatt tggataaagc cggcgcattt gaaagattac ggtttgccca 1396980
agcctttgga aatttattta aacggtaata ggttagaata aacaaaataa acccattgaa 1397040
ctgttgtttg caggtatcgc agcaagaaca accgatgaat ttgggtcgta ttttaggcgg 1397100
cgggataatg ttcaaatggg acatttggaa cggaagaagt cggcaattta aaaaggattt 1397160
aaaaagcaaa gaaggtcaaa aacatgaaca caaacttaaa tgacaaagac aaagccatgg 1397220
ataccgcaat caggtttcag aaaaggatga ggattccgaa atttttcttt ttaattctcg 1397280
gaatcacaat ggttttggca tttatccaag acgtgataac gggttctaat tttctgcaaa 1397340
taacaattaa tgtaaaattt tcgtaaaaat ttatcggctt ttaaaacaaa attgactaaa 1397400
atagtcgcga gtttttactg caataaagga gattgcaatg aatatgaaaa ccttattagc 1397460
actagcggtt agtgcagtat gttcagttgg tgttgcgcaa gcacacgagc ataatacgat 1397520
acctaaaggt gcttctattg aagtgaaagt gcaacaactt gatccagtaa acggtaacaa 1397580
agatgtgggt acagtgacta ttactgaatc taactatggt cttgtgttta cccctgattt 1397640
acaaggatta agcgaaggct tacatggttt ccacatccat gaaaacccaa gctgtgagcc 1397700
aaaagaaaaa gaaggtaaat tgacagctgg tttaggcgca ggcggtcact gggatcctaa 1397760
aggtgcaaaa caacatggtt acccatggca agatgatgca cacttaggtg atttacctgc 1397820
attaactgta ttgcatgatg gcacagcaac aaatcctgtt ttagcaccac gtcttaaaca 1397880
tttagatgat gttcgcggtc actctattat gatccacacg ggtggtgata atcactccga 1397940
tcatccagct ccacttggcg gtggcggccc acgtatggca tgtggcgtga ttaaataatt 1398000
cgattgttcg aaacgaaaag tgcggtgaat tttgaccgca ctttttttgct agatatttag 1398060
cattgagacc tttgcaataa cataggttac taaaatttta tgctcaatct cattttcaaa 1398120
atgcaaaact tttctgattt ttcctacttt ttgctcaata ttaggaaggt tttaggcaat 1398180
tgaaaatttt ttggcgcatt tttatgcgtc aaatttcgtt aacagactat ttttgcaaag 1398240
gtttcaattc ataagtttcc cgaaattcca acataaccga aacctgacaa taaccgtagc 1398300
aactgaaccg tcattcccgc gaaagcggga atctagacct tagaacaaca gcaatattca 1398360
aagattatct gaaagtccga gattctagat tcccgctttc gcgggaatga cgaaaagaga 1398420
cctttgcaaa attccttttc cccgacagcc gaaaccccaa cacaggtttt cggctgtttt 1398480
cgccccaaat accgcctaat tctacccaaa tatcccctta atcctccccg gatacccgat 1398540
aatcaggcat ccgtgctgcc ttttaggcgg cagcgggcgc acttagcctg ttggcggctt 1398600
tcaacaggtt caaacacatc gccttcaggt ggctttgcgc actcacttta accagtccga 1398660

aataggctgc ccgggcgtag cggaatttac ggtgcagcgt accgaagctc tgttcaacca 1398720
cataacgggt cttcgacaaa tatcggttgc gtttggtttg cacttccgtc agcggacggt 1398780
tgcggtgggc tttgcgcata atgccgtcca gcaactgatg ttcttccaga tgttgccggt 1398840
tttccgcact gtcgtagcct ttgtcggcat agacggtcgt acctttgggc agtccttcca 1398900
acaacggcga caggtgtttg cactcatggg cattggcggg ggtaatgtgc agtttctcga 1398960
tatagccttc tgcatcggta cgggtatgtt gtttgtaacc gagtttgtag aggccgtttt 1399020
tctttatcca acgggcatcg ctgtccttac tcggtgtggt ttgaccgctg atttgtcctt 1399080
cttcgtcaac ttctatggcc tgacgctgtt tgctgccggc ggtctgaata atggtggcgt 1399140
caacgacggc agcggatgct ttctctattt ttaaaccttt ttcggtcagt tggcggttaa 1399200
tcagttccaa cagttcagac agggtattgt cttgcgccag ccggttgcgg tagcggcata 1399260
aggtgctgta atcggggatg ctcagttcgt caaaacggca aaacaggttg aaatcgatgc 1399320
gggtaatgag gctgtgttcg agttcgggat cggagaggct gtgccattgt ccgagcagga 1399380
cggctttgaa catggacagc aggggatagg caggacggcc gcggtggtct ctaaggtaac 1399440
gggttttttg acggttcagg tattgttcga tcagctgcca atcaatcacc cggtccaact 1399500
tcaatagcgg gaagcggtcg atgtgtttgg caatcatggc ttgggcggtt tgctggaaga 1399560
aggtgctctt gagaaatccc ctaaatgtct tggtgggaat ttaggggatt ttggggaatt 1399620
ttgcaaaggt ctctagatga gtgaaaaaga agtgcaggct gcctaaaaag acagaaaaag 1399680
tctttccggc agcctgcact ttggtttcat ttcagtcagt aaacccagta aacgacggtc 1399740
tgaaaacgca gaacgttacg aaaaaagcag cctacacgcc catcccccgc cttctacccg 1399800
ttctgtaaat catacagata gcggtaatat ccgttcggct tcgccagcaa ttcctgctgt 1399860
gttcccgctt ccacaatcct gcctttatcc atggcaatga tccggtgtgc cgttttaaca 1399920
gtggacagac ggtgggcgat aatcagcacc gtccggttgg cgcaaatggc ctgcatgttc 1399980
tgcataatcg ctcgttcact ttcataatcc agcgcgctgg tggcttcatc aaaaatcaga 1400040
atgcgcggat tggtgattaa cgcgcgggca atcgcaatac gctgccgctg tccgcccgac 1400100
aagccggccc cttgttcgcc caccacggtg ccgtagcctt ccggcagctc cataataaac 1400160
tcgtgtgcgc ccgccagttt ggctgcttcg ataatgcgtt ccagcggcat acccgtatcc 1400220
gtcagcgcga tattgtcgcg tatgctgcgg ttgagcagca cattctcctg caagaccacg 1400280
ccgacctgcc gccgcagcca ggcaggagcg gccaaagcca aatcgttgcc gtccaccaac 1400340
acccgtccct gctccggtac atacagacgc tgcaccaatt tggtgagtgt ggatttgccc 1400400
gaccccgaac gtcccacaat ccccagcact tcccccgccc gaatccgcag gttcaaatcc 1400460
tgcaaaatca gcctgccgtc cgccttatag cggaaatcga catgttcgaa cgtaatctcc 1400520
ccccggatat cgggcaaagc caaatgcgaa gacgcattct cggtcggcgc attcagaata 1400580
tcccccaaac gcgccaccga aatccccacc tgctggaaat cctgccacaa ctgcgccaaa 1400640
cggataacag gcgccgccac ctgtcccgag agcatattaa acgcaatcag ctgccccacc 1400700
gtcagcttgc tctcaattac cagccgtgcg ccaatccaca acgtcgccac cgtcaccagc 1400760
ttctgaatca gctgcacccc ctgctggccg accaccgcca acttcgttac ccgaaatccc 1400820
gaagccacat aagccgccaa ctgattgtcc caacgctgcg tcatctgcgg ctccaccgcc 1400880
atcgccttta ccgtacccac cgcagtgatg ctttctacta aaaacgactg gttgtctgca 1400940
ttgcgcgcga acttatcgtt cagacgcgtc cgcagtatcg gactgataaa tgccgaccaa 1401000
aacgcatagg caggcaacga agccaatacc acccaagtca gagtggagct gtaataccac 1401060
atcaccgcca gaaagataaa cgaaaacgcc aaatccaaca ccgaagtcag cgcctgaccg 1401120
gtcaagaaat tgcgaatctg ctccaattcc cgcacccgag ccaccgtatc acccactcgt 1401180
ctgtgctcga aataggataa aggcagggaa agcagatgcc ggaacaaacg cgcgcccaat 1401240
tccacatcaa tacgtgaagt cgtatgtgca aacagatacg tccgcaaacc gcccaacaca 1401300
atctcaaaca gcgacaccac caacaaagcc accgacacca catccaaagt agagaatccc 1401360
cgatgtacca gcaccttgtc catcaccact tggaaaaaca gaggcgtaat cagcgcaaac 1401420
agctgcaaca ccaccgacac caccaatact tcaaaaaaca accggcggta tttgattacc 1401480
gccggaataa accaggtaaa gtcaaacttt gccaaactgc ccaataccga agcgcgggaa 1401540
gcaaccaata tcagtttgcc cgaatatctg ttagaaaatt cggcaaaaga caataccgca 1401600
gacttattcg taaccaaatc ctgtatcaaa aattgggcat gctcaccctc accgtctgtt 1401660
ttggccaaaa tgaaatggtt gccgtcatca caccatacca atgcgggtaa agtcgccata 1401720
gccaaacgtt taataggctg gcggactacc tttgccttca atcccaaaga tttggcggct 1401780
aacagccatt gcgtttcatt taaatcgctc tgtgcggaag tacaaaattc atgctgtata 1401840
tcggcaggat tggcggcaat gccgtggtaa tgggcgagga tgatgagggc ggaaagggcg 1401900
gggagcggtg cggatacgat agacataaat aaaatatagt tagattggat gtggataacg 1401960
gctggctgga aaaggaatat attaagtaga aagaaatata taaataaaac agcagaacgc 1402020
attgtaagga tatatatggg aattgtaaag agaaagtatg gaaaagttct cgtttcagga 1402080
aggtaaaacg gcttaggaat cgagttagat gaggatgcct cgcacctctc gtgcctcctg 1402140
cataccgtta aggcacaggg ttaaggtgca ggctgctccg aactctgttg cggtcgggta 1402200
atgttatttt ttgtgtttca ggcagcctga aatatctgta tatttttgtt ttaaatagat 1402260
tttaaagatt gataactgtt cttgacgatt tttcaagaaa ggagtaaatt tcaagaaagg 1402320

agtaaagtga cttattatca atgacaagca acgcgcgaag tgacaaggaa aactatctac 1402380
ttaaattcta aggaggcttc gaatatcata aaccaatcag aaacatagag ataaaaatta 1402440
tgtacaaata taatcctctt atacaattta ttgcacagtt gattatgtct tatggagcaa 1402500
gcgtagggtg ggcacttgct gccccacgcg tttcatattt caaggcagcc tgaaaccgtg 1402560
tgggcataaa tgcctaccct acatcccaaa aaacaagcgc agcctgcgtg tgtagggtgc 1402620
gaactttcgg caggtagaca cgcagtttta tattttcaag ctgagggatg cttaagaaaa 1402680
gtacaaaaca ttaaaaaata aggggctgta ctagattagc cctaaatcca caccaatccc 1402740
gcaagatttt tagctgtcgg gacggtgtgc cgaagttaaa tcgaaattcg cattctttca 1402800
agaacagcgg gaaagatttg cgatcaattc cgttctattt gcgcaagacg cgttttgcct 1402860
gattccaaaa gttctcaatg ccgtttatgt ggttctgacg gtcagcaaat tccttggaat 1402920
ggttgatgcg gtaatggata aaaccgctta cgtccaactt gtcgtaactg ctcaggctgt 1402980
cggtataaac aatgctgtcc ggcatgattt tctgtttgat aacaggcatt aaagtatcgg 1403040
acttggcatt atctacgaca acggtataga cccgtccgtt acgtttcaga atgccgaaga 1403100
caaccacttt tcctgccgca ccgcgaccac gtttgccttt acgccgtccg ccgaaatagc 1403160
tttcgtccaa ctcgacagag ccctcgaaaa cctcattggc agccaaggcc agataatggc 1403220
tgatgaccat acggattttg cggtagaaca ggactgccga attgggatgg atacccaaaa 1403280
tatcggcagc agaacgggcg gtaacttcga gtacaaaaaa acggagcagc tctttctgta 1403340
ctttttcttt taatttgcag tgtgttatct tcatatttcg ggggtaacat atctgctaat 1403400
ctagtacagc ctcaaacaaa aaagagaaat tttaatttcg ctaaatcgca taaagattaa 1403460
tcaagagtat cattaaatga tatgagtgag catctataat gccaagaaga gttgttaaga 1403520
cataacgatt attgaaatag attgtaaaat agatacttag atagtctgaa aaacggattt 1403580
gtgaaacttt ttattacgcg ccatcatttg aaaatgaaac ttaaaaaaca cttatcataa 1403640
taaatatttt ctttacgttg tttgctaata aactcagtgc aatatcagcg caatatttta 1403700
tggaaatttt atggataaca aaaaagaatt tattaataat ttaacaaata ggtatatgtg 1403760
gatctatcca ttggtcttaa atattctatt tctacctttt taccagtcct accaatcttt 1403820
ttttattgcg cttggttgtt tgtttgcact ggttagaaaa atgcaaagct tagattttaa 1403880
attacaaaat catattgtat tgttaaatat aaaaagtgct tgggcagata aaaaagtatt 1403940
tttgattagg atagtagtgt catggttggc agtaatggaa atatggatgt gttttatttc 1404000
ggaatcatca acgtgggtat gcggtgcttt ttgtttaaat agtgaaatat tggaaaaaat 1404060
ttttcgtggc tttggttatt ctggtagttt atattttttta tttatattga tgattgatct 1404120
caataagtta agagagagta tttgaatttc attttttgtt tgacttaaac tcaaggagag 1404180
taacaatgat tggtagtggt gatactaaac aatgcaaaaa attttctgcg tgtgatggaa 1404240
aataccacgt ctacgatccc ctcgccctag acttggacgg cgacggcata gaaacagtca 1404300
ccgccaaagg cttttcaggc agcctgaaga ctgagagagt gaatacgatg agtatacact 1404360
ctatgccact aaattgatat tcactaaatc ataccagcta tattttattt aatgagacat 1404420
atgaaaaata aaaattattt actagtattt atagttttac atatagcctt gatagtaatt 1404480
aatatagtgt ttggttattt tgtttttcta tttgattttt ttgcgttttt gtttttttgca 1404540
aacgtctttc ttgctgtaaa tttattattt ttagaaaaaa acataaaaaa caaattattg 1404600
tttttattgc cgatttctat tattatatgg atggtaattc atattagtat gataaatata 1404660
aaattttata aatttgagca tcaaataaag gaacaaaata tatcctcgat tactggggtg 1404720
ataaaaccac atgatagtta taattatgtt tatgactcaa atggatatgc taaattaaaa 1404780
gataatcata gatatggtag ggtaattaga gaaacacctt atattgatgt agttgcatct 1404840
gatgttaaaa ataaatccat aagattaagc ttggtttgtg gtattcattc atatgctcca 1404900
tgtgccaatt ttataaaatt tgcaaaaaaa cctgttaaaa tttattttta taatcaacct 1404960
caaggagatt ttatagataa tgtaatattt gaaattaatg atggaaacaa aagtttgtac 1405020
ttgttagata agtataaaac attttttctt attgaaaaca gtgtttgtat cgtattaatt 1405080
attttatatt taaaatttaa tttgctttta tataggactt acttcaatga gttggaatag 1405140
ttttggtaat tttatgagcg cacgctcatc cgcgttagca gaatttggaa atatggttgc 1405200
taatttagtt tctgcaaaaa atgagaaaga tatctcgaaa cgtaatgaat attacaaaca 1405260
agctggttat agtgcattat tagcatttgg taatttggct agtaatattg caccaggtag 1405320
tacgtcatcg catattgtaa acggaacaaa tgcctctgtg attgcaagcc gtctctctgg 1405380
aaatatatct tcagctattc aggagcataa agatggtaaa gttaatatca accgttttca 1405440
aaatatttta gcggatttat attcattggg agggttagga agtacattaa tagagaagaa 1405500
tggaaatatg cagagttggg ggattccatt agcaattgct ggagatataa ttgcagcaac 1405560
ggctattgcc acaggagata ctggtacgat atctacagag gaattttata attttgacaa 1405620
ctggaaaggt tttgggtatg agctatttga agactggtct cgttgggtat acgactggct 1405680
gcccgacggc tggaatctgt ggaaagaatt ggacagaaac cgttcaggcc aataccacat 1405740
ctacgacccc ctcgccctag acctagacgg cgacggcata gaaacagtcg ccgccaaagg 1405800
cttttcaggc agcctcttcg accataacgg caacggcatc cgcaccgcca ctggctgggt 1405860
ttctgccgat gacggtttac tcgtccgcga tttgaacggc aacggcatca tcgacaacgg 1405920
cgcggaactc ttcggcgaca acaccaaact ggcagacggt tcttttgcca aacacggcta 1405980

tgcagctttg gccgaattgg attcaaacgg cgacaacatc atcaacgcgg cagacgccgc 1406040
attccaatcc ctgcgtgtat ggcaggatct caaccaggac ggcatttccc aagctaatga 1406100
attgcgtacc cttgaagaat tgggtatcca atctttggat ctcgcctata aagatgtaaa 1406160
taaaaatctc ggtaacggta acactttggc tcagcaaggc agctatacca aaacagacgg 1406220
tacaaccgca aaaatggggg atttactttt agcagccgac aatctgcaca gccgcttcac 1406280
gaacaaaatg ctatccatta gccatgttcg ggaaaacacg atttccccgt ttgttttagg 1406340
ctgtctaaac aaataaccat aaatgtatat cattatttaa aataaataaa agtatttaac 1406400
tattattgac gaaattttag agaaagagta gactgtcgat taaatgacaa acaatagtga 1406460
gaaaggaaat atttactatc cgagcacaga gcatatttta ggtagcctgt aactgttcct 1406520
gctggcggaa gaggatgaag gttgacttac ccgagaataa atgtcctgtt gtgtgatatg 1406580
gatgccatgc cgcgaagcaa ttgatgcaat cacggcagtc ctacttgaat gaaacctgtc 1406640
gttgcagaat ttgaaaacgc tatttttaag aaaggataaa gggagaaaga atttttggtt 1406700
tttaagctgc atgaaaccgt gttggaataa atgcacacct acgataatta ataattttcg 1406760
tttttttattc tacaagctat ttatatatga ttgctaaaag tttatttttt agatgccaaa 1406820
aaatatattt tatatacttc atattgttta tatgtcttta tttgaatata tcttacgatg 1406880
gggaaatatt tatatatttt ataataaatt ttactcattt gctaatatgt catggaatat 1406940
tacttgtatt ttgtagaatt tttccatatg aaaatattcc atttactatt tttctgaact 1407000
ttattagttt atttttaata tttttacctc ttatatttac cataagagag ctaattgatt 1407060
catattatat tgagtcgata attaatttat tcttaatttt aattcctcac gttatttttt 1407120
taatttactt gaaaggaaag cagatatgac atctgcaaat tttaatatta acggttttgg 1407180
agatgtgaaa ttaacaccct attcaccact cttgggatat aaagcttggg attcatttat 1407240
tggttctatt caatccttat ctgatttaat ctataatgtg gataacaata gaaataaaat 1407300
ggaaattact gttaataatg ctattcaagc tgcagatagc tttttaagca gtaattggaa 1407360
gagataacaa aataacaaaa taacaaaata acaaatactg cttctttact tgcatccttc 1407420
gataacattt tttaaattta agaaatgtat ctcgagatat acgagaaaca ggaaaattta 1407480
aacctaatga tattcaacaa gcaattggtg atatattcat tgctgctggt gatggattac 1407540
aatatataaa acaacaaaca gaggcgatgg ctcaaagcaa attcttacca actaaattaa 1407600
aaactggttt aaatgatgtc cttaattcta gaatgctaaa atcctctact gttttacagc 1407660
atgaattgaa ttaaataagg attatggaaa cgagaggctt ggcgaatcta taatgaatat 1407720
agatgatttt acaccaagta agatagcaaa cttttttgcg gatcctgata catacagcaa 1407780
tgtattagaa gaagtatcta ggtttatata ttccttagtt cctgatgatg caaacccttg 1407840
gaaagggggc gaagattata ttggacgagg gataagtgaa tggggagagt tactggaaaa 1407900
atggtataaa caagattttc tcccttatct tgaaaagaat gggaccaatt tccgaaattt 1407960
gaagattggc tgcctgaatt ccctgaatgg gcaagagagt ggttgaaatt agctctcaaa 1408020
cgttcaggca aatataacgt ttacgatccc ctcgccctag atttggacgg cgacggtata 1408080
gaaaccgttg ccaccaaagg cttttcaggc agcttatttg atcacaccaa caacggcatc 1408140
cgcaccgcca cgggctggat tgctgcatat gacggttttc ctgtgcgcaa attaaacagt 1408200
aacgggggca ttattagcac gacagatacc atattccaat ctttgcatac atggcttgat 1408260
catcaaccaa gatgatattt cccaagcaca gcatgatgca tgccattgaa aaatatagaa 1408320
aattaattga aagcttaaat ggatattgaa atgaatgatc acatagtaca aattataaga 1408380
aggttcgggc taggtaggat attttttttat tcgtatagca aatcatctat aataattttt 1408440
tcttcgtatg ttgtttatta tataatttac aattatcaat ttaattacct ttcgctttta 1408500
atttatttat taccaatatt gtgcagtata tatatgttta tattttttttt agggaaaact 1408560
aaggatacat taacgacaga gcgaagaaaa aaattttttta attctatttt tccacttaga 1408620
attctaatga taataggttc tgagaaaaag aggttaggca tcggtagttt ttatttgcta 1408680
aacctactat ggattatttg gtgtcttatg attcatagag aacaagtccc attaaataac 1408740
ttaaccctcc tattatcctt catattttca ttatttttttt tattatgtga ttttgtttta 1408800
ttattgaatg tttatgttta ttttttttaaa ttaagagagg cttaatatgg ttaatcaaat 1408860
caaatctgat aataattcag tttctattga atttatataa gattttataa ctgcaagtac 1408920
ggatgtaatt aatctgagtt acgaaaattt tcgtaaaaat ttttatacac aaatgtcaac 1408980
tgattctacc aattatgcag ccaaacatga aagtttagga aaatcggtac aacgtgaatt 1409040
acaaaaaaca caaagtcagt tgagacaagt tgtaagaaaa atgcagagta aatataatat 1409100
aaataataaa gcacgagtag cagaaatatc tttgttaagg caaatgcaaa gccaattttc 1409160
tcgaaaatat gtaaacaaaa atcttggtaa cagcaacact ttggctcaac aaggcagcta 1409220
caccaaaaaa gacggcacaa ccgcgcaagc aggcgatttg ctgttggctg ctgacaacct 1409280
gcacagccgc ctcacggaca aaatgctatc cattagccat gttcgggaaa acacgatttc 1409340
cccgtttgtt ttaggctgtc taaaacaaat aaccataaat gcatatcatt atttaaaata 1409400
aataaaagta tttaactatt tttgacaaaa ttttagaaat agagctagag ttttagttaa 1409460
gtagaaattg atagtgcttc aagggaagta ttctctatgt ttgcattaaa gggggtctga 1409520
taaagctatt attcattact atggactttt atttcattat tttcaggcgg aaatctcata 1409580
gccgttttga atttttctct tccttattaa ttatacaaat aattagtata ttctgatatg 1409640

gattttttgg aaatttttat tatgtctgca tttagaaaaa tattattaat aatatcttgc 1409700
ctattgattg ctagctgcag ttttgttgaa actatttttt atatggctat tagcccagaa 1409760
cctgttgtgg tagactttcc tcttggtaaa aaaacaaaaa gatctattga actcaaacag 1409820
aaaattggta aaccttatgc aatatcgtta ggaactaatt ttatacatta tgatccaaaa 1409880
caggggggaga ggtggattga tgataagtta aactatccat ataatatatc ggttaaaata 1409940
tttaaagtgg aagaagatgg taaaaaactt attatagatg agttgcttac agagagaagt 1410000
agaaaattag gaggcggagt atttggagct gggggaaaat acagtatgca tatttatgat 1410060
ttttatttgc cggaagggga atatttattt gagatttctg ataatagtga atatattcca 1410120
ctttacgatg aaataaataa ttctataaga atagtagtta atgcacgaat tcagtaaatt 1410180
tttctagaaa tgtggggtta cttatggctg attattatgc gataactgta aaatttgcga 1410240
agcagggtac gccactgaaa caagaggggg tgtatccaag acgggtacgt ttgggttgaa 1410300
ctgtattcgg ctagagataa aaaaatcggg gctgtactag attagcccta aattccacac 1410360
caatcccgca ggattttaag ctgttgagac ggtgtgccga agttaaatcg aaattcgcat 1410420
tctttcaaga acagcgggaa agatttacga tcgattccgt tgtattttcg caagacgcgt 1410480
tttgcctgat tccaaaaatt ctcaatgccg ttaatgtggt tctgacggtc tgcaaattcc 1410540
ttggaatggt tgatgcggta atggataaaa ccgctcacgt ccaacttgtc gcagctgctc 1410600
agactatcgg tataaacaat actgtccggc atgattttct ttttgatgac agggagtaac 1410660
gtttcagact tggcattatc tacgacaacg gtatagcccc gtccgttgcg tttcagaatg 1410720
ccgaagacaa ccacttttcc tgccgcaccg cgaccacgtc tgcctttacg ccgtccgccg 1410780
aaatcgcttt cgtccggctc gacagggccc tcaaaaacct catcggcagc caaggccaaa 1410840
tgatggttga taaccgtgcg gattttacgg tagaacagta ctgccgaatt gggatggata 1410900
cccaaaatat cggcggcaga acgggcggta acttccagca caaaaaaacg gagcagttct 1410960
ttctgtactt ttttctttaa tttgcagtgc gttatcttca tatttcgagg gtaacatatc 1411020
tgctaatcta gtacagcccc aaaaatatac caaaaacagc aaaacaaatt gtaaggatag 1411080
gtataggctt tgtaaaggta aattgtgaaa aaagcagttt tttaaacgaa tgaaacggct 1411140
tcgggctgaa atatatgctg atgccctgtc cttcccgtat atcttgtgtg ttgtcaaagt 1411200
gcaggctgct ttgaaatcgg tattgccatc tatgaaccac cactttgttt tatttcagcg 1411260
ggcttgagat gtgtataaga atattgtttt gaataaattt aaaaaaatga taatcgttat 1411320
tgacgatttt taaaggaaag cgtagagtgc caattctatg aagcaatacg gtaagtaaca 1411380
atgaaaatat ctactgcttg ggtatagagc atatttcaca acccgtaact attcttgcgg 1411440
aaacagagaa aaaagtttct cttctatctt ggataaatat atttaccctc agtttagtta 1411500
agtattggaa tttataccta agtagtaaaa gttagtaaat tatttttaac taaagagtta 1411560
gtatctacca taatatattc tttaactaat ttctaggctt gaaattatga gaccatatgc 1411620
tactactatt tatcaacttt ttattttgtt tattgggagt gtttttacta tgacctcatg 1411680
tgaacctgtg aatgaaaaga cagatcaaaa agcagtaagt gcgcaacagg ctaaagaaca 1411740
aaccagtttc aacaatcccg agccaatgac aggatttgaa catacggtta catttgattt 1411800
tcagggcacc aaaatggtta tcccctatgg ctatcttgca cggtatacgc aagacaatgc 1411860
cacaaaatgg ctttccgaca cgccagggca ggatgcttac tccattaatt tgatagagat 1411920
tagcgtctat tacaaaaaaa ccgaccaagg ctgggtgctc gaaccataca accagcaaaa 1411980
caaagcgcac tttatccaat ttctacgcga cggtttggat agcgtggacg atattgttat 1412040
ccgaaaagat gcgtgtagtt taagcacgac tatgggagaa agattgctta cttacggggt 1412100
taaaaaaatg ccatctgcct atcctgaata cgaggcttat gaagataaaa gacatattcc 1412160
tgaaaatcca tattttcatg aattttacta tattaaaaaa ggagaaaatc cggcgattat 1412220
tactcatcgg aactatcata ggtatggaga gaacgattac agcactagcg taggttcctg 1412280
tattaacggt ttcacggtac ggtattaccc gtttattcgg gaaaagcagc agctcacaca 1412340
gcaggagttg gtaggttatc accaacaagt agagcaattg gtacagagtt ttgtaaacaa 1412400
tccaagtaaa aaataatggg gctgtcctag ataactagga taaactcgat tttactaatt 1412460
gttttaaaat ggaacaagaa cttttatctc actgttgtta aaacgccatt cgcactcctt 1412520
taaatacagc tcaaaatgcg ctttgggaat gccgttaaac ttgcgtaaat gacgttttgc 1412580
ctggttccaa aagttctcaa ttccattaat atggttttgt cgttcagcaa aatgtgtgct 1412640
gtgattgata cgaaaacgaa gtttcagcga agctaaaatg gctaaattcg cgcacatcta 1412700
atacatcata gctacgataa caatccgtat aaataatgct gtcaggtttc acttgttcac 1412760
ggataatagg aaataaagta gcggtttgag tattcggtac tgtaaccgta taaaccttac 1412820
catttcgctt caaaagaccg aatacggcga ctttaccggc agcaccgcga ccgcgtttgc 1412880
ctttgcgttg tccgccaaaa taactttcat ctgcttctac ttcgccatca aacatttcca 1412940
aatgcggact gttttgataa ataagtaatc gtaaacgatg aaaataatag gctgcggtat 1413000
ttttattaac gcctactaac tctgctgccg ttcttgcagt tacacctgtg acaaatagct 1413060
caatgagttt attttgttta tactggctta gacgactttt tctcataggg ataattctaa 1413120
cttaatttga atttccctag ttatctagga cagcccctat tctttaacta atttctaagc 1413180
ttgaaattat gagaccatat gctactacca tttatcaact ttttattttg tttattggga 1413240
gtgtttttac tatgacctca tgtgaacctg ttaatgaaca aaccagtttc aacaatcccg 1413300

agccaatgac aggatttgaa catacggtta catttgattt tcagggcacc aaaatggtta 1413360
tcccctatgg ctatcttgca cggtatacgc aagacaatgc cacaaaatgg ctttccgaca 1413420
cgccagggca ggatgcttac tccattaatt tgatagagat tagcgtctat tacaaaaaaa 1413480
ccgaccaagg ctgggtgctc gaaccataca accagcagaa caaagcacac tttattcaat 1413540
ttctacgcga tggtttggat agcgtggacg atattgttat ccgaaaagat gcgtgtagtt 1413600
taagcacgac tatgggagaa agattgctta cttacggggt taaaaaaatg ccatctgcct 1413660
atcctgaata cgaggcttat gaagataaaa gacatattcc tgaaaatcca tattttcatg 1413720
aattttacta tattaaaaaa ggagaaaatc cggcgattat tactcattgg aataatcgag 1413780
taaaccaggc tgaagaagat aattatagca ctagcgtagg ttcctgtatt aacggtttca 1413840
cggtacagta ttacccgttt attcgggaaa agcagcagct cacacagcag gagttggtag 1413900
gttatcacca acaagtagag caattggtac agagttttgt aaacaattca agtaaaaaat 1413960
aatttaaagg atcttattat gaatgagggt gaagttgttt taacaccaga acaaatccaa 1414020
accttgcgtg gttatgcttc ccgtggcgat acctatggcg gttggcgtta tttggctaat 1414080
ttgggtgacc gttatgcgga tgatgctgct gcaattgtcg gtaaggatgc aaacttaaat 1414140
ggtttgaatt tatggatgaa aaaaggtgtg gaaaacctat gggatgatac ggtcggtaaa 1414200
aagacccgtt tagagaaatt tgatcgggtt gcactgcaac atttcaggca atatgcgcgt 1414260
ctaattaatc aaaataatgg tagattaccc aatactagtg aaattgagag aagttactat 1414320
aaagccgtta ccgataatgg cgtttcttcc agtgcagcta ttgatttagt tattaatcgt 1414380
tcacttccgg atatggcgga tggttattgg gcattaggtt tggggataga agccgaacgt 1414440
atccacaatg agcaagcagt aaataatccg aacggtagcg aaagggataa tagaaagcag 1414500
ttaatatctg ctttagataa aggatttgat ggatctttta aagagaagca ttttactttt 1414560
ttacaatctg tgatgatgga tgtaacaaag ttaggtgttg aatatacaat agatggttgg 1414620
caaaaaattg gaggttgggg taatgggata atcaatgatt tatataaaag tgttgtaaaa 1414680
agagagtgga ctggaatatt tgagatcgtt aataataaca tcaagcaagg aaatgaagct 1414740
tttaaaaatg aaatcaatag cttggttcat gatatgaaag ctgctggcaa ggaatttgga 1414800
gatgacttaa atacacagtg gaataatctc actcaggctg ccgaaataat ctataatgac 1414860
atagtagaca atactagtca aggaatagaa aaaggtgtca aagccattaa agaattgtct 1414920
gaaaaaatga aaaatgctgc ttccgatttg gctgacggtt cagcagagaa agctaaacaa 1414980
gtagtggaag atttggctca agccgccaaa gaagcatacg aaaatgccaa atccacagcc 1415040
gagaaggctg ctcaagcagc tcgagaattt tttaagggct tgcccagttt taaagatctg 1415100
gccgaaaaat ttagagatct gttcccaaat ccggaaggct ggatcgatga tggtcaccaa 1415160
tgtttagctc cttgggttaa agaaactaaa aaacgcaatg gcaaatatca tgtctacgac 1415220
ccccttgccc tagacctaga tggcgacggt atagaaaccg ttgccaccaa aggctttgca 1415280
ggcagcttat ttgatcacac caacaacggt atccgcaccg ccaccggttg ggtttctgcc 1415340
gatgacggtt tactcgtccg cgatttgaac ggcaacggca tcatcgacaa cggtgcggaa 1415400
ctcttcggcg acaacaccaa actggcagac ggttcttttg ccaaacacgg ctacgcggct 1415460
ttggccgaat tggattcaaa cggcgacaac atcatcaacg cggcagacgc cgcattccaa 1415520
accctgcgtg tatggcagga tctcaatcag gacggcattt cccaagctaa tgaattgcgt 1415580
acccttgaag aattgggtat ccaatctttg gatctcgcct ataaagatgt aaataaaaat 1415640
ctcggtaacg gtaacacttt ggctcagcaa ggcagctata ccaaaacaga cggtacaacc 1415700
gcaaaaaatgg gggatttact tttagcagcc gacaatctgc acagccgctt caaagacaaa 1415760
gtggaactca ctgccgaaca ggcaaaagcc gccaatcttg cgggcattgg ccgtctgcgc 1415820
gatttgcgcg aagctgccgc attgtccggc gatttggcca atatgctgaa agcttattct 1415880
gccgccgaaa ctaaagaagc acagttggca ttgttagata atttgattca caaatgggcg 1415940
gaaaccgatt cgaactgggg caaaaaatcg ccaatgcgac tttcaaccga ttggacgcaa 1416000
acggctaatg aaggtattgc actgacacca tcccaagtag cacaactaaa aaagaacgct 1416060
ttagtttccc tttctgataa agctaaagca gctattgacg ccgcccgcga ccgcattgcc 1416120
gtgcttgatg cctacacggg gcaggattcc aacacactct attacatgag cgaggaagat 1416180
gcgcttaata tcgtcaaagt aaccaacgat acatacgacc atctcgccaa aaacatctac 1416240
caaaacctgt tgttccaaac ccgtttgcag ccatatttga atcaaatcag tttcaaaatg 1416300
gaaaatgata cgttcacttt ggattttagt ggtcttgttc aagcatttaa ccatgtcaaa 1416360
gaaactaatc cgcaaaaagc ttttgtggat ttggccgaga tgcttgcata tggcgaactt 1416420
cgttcttggt atgaaggccg aagactaatg accgattatg tggaggaggc aaaaaaagca 1416480
ggtaaatttg aagattacca gaaagtgttg ggtcaggaga ccgttgcatt attagctaaa 1416540
acatcgggta cgcaagcaga tgatatcctg caaaatgtag gctttggtca taataaaaat 1416600
gtttctttat atggtaatga cggcaacgac actctaatcg gcggcgccgg taatgactat 1416660
ttggagggcg gcagcggttc ggatacttat gtcttcggcg aaggcttcgg tcaggatacg 1416720
gtctataatt acgactacgc taccggacgc aaagacatca tccgctttac cgacggtatt 1416780
acagccgata tgctgacttt tacccgagag ggcaaccatc ttcttatcaa ggcaaaagac 1416840
ggcagtggac aagtgactgt tcagtcctat ttccagaacg atggctcagg tgcttaccgt 1416900
atcgatgaga ttcatttcga taacggcaaa gtactggatg ttgccactgt caaagaactg 1416960

gtacagcaat ccaccgacgg ttcggacaga ttgtatgcct accaatccgg aaatacctta 1417020
aatggcggat tgggcgatga ctatctgtac ggtgccgacg gggatgacct gctgaatggt 1417080
gatgcaggca acgacagtat ctacagtggc aatggcaatg atacgctcga tggaggagaa 1417140
ggcaacgacg ccctgtacgg ctataatggt aacgatgcac tgaatggtgg cgaaggcaat 1417200
gatcatttga acggcgaaga cggtaacgac actctgatcg gcggtgccgg taatgattac 1417260
ttggagggcg gcagcggttc ggatacttat gtcttcggca aggcttcgg tcaggatacg 1417320
gtctataatt acgactacgc taccggacgc aaagacatca tccgctttac cgacggtatt 1417380
acagccgata tgctgacttt tacccgagag ggcaaccatc ttcttatcaa ggcaaaagac 1417440
ggcagtggac aagtgactgt tcagtactat ttccagaacg atggctcagg agcttaccgt 1417500
atcgacgaga ttcatttcga taacggcaaa gtactggatg ttgccactgt caaagaactg 1417560
gtacagcaat ccaccgacgg ttcggacaga ttgtatgcct accaatccgg aaatacctta 1417620
aatggcggat tgggcgatga ctatctgtac ggtgccgacg gggatgacct gctgaatggt 1417680
gatgcaggca acgacagtat ctacagtggc aatggcaatg atacgctcga tggaggagaa 1417740
ggcaacgacg ccctgtacgg ctataatggt aacgatgcac tgaatggtgg cgaaggcaat 1417800
gatcatttga acggcgaaga cggtaacgac actctaatcg gcggtgcagg caatgattac 1417860
ttggagggcg gcagcggttc ggatacttat gtcttcggca aggcttcgg tcaggatgcg 1417920
gtctataatt acgactacgc taccggacgc aaagacatca tccgctttac cgacggtatt 1417980
acagccgata tgctgacttt tacccgagag ggcaaccatc ttcttatcaa ggcaaaagac 1418040
ggcagtggac aagtgactgt tcagtcctat ttccagaacg atggctcagg tgcttaccgt 1418100
atcgatgaga ttcatttcga taacggcaaa gtactggatg ttgccactgt caaagaactg 1418160
gtacagcaat ccaccgacgg ttcggacaga ttgtatgcct accaatccgg aaatacctta 1418220
aatggcggat tgggcgatga ctatctgtac ggtgccgacg gggatgacct gctgaatggt 1418280
gatgcaggca acgacagtat ctacagtggc aatggcaatg atacgctcaa tggaggagaa 1418340
ggcaacgacg ccctgtacgg ctataatggt aacgatgcac tgaatggtgg cgaaggcaat 1418400
gatcatttga acggcgaaga tggcaacgac actctaatcg gcggtgcagg caatgattac 1418460
ttggagggcg gcagcggttc ggatacttat gtcttcggca aggcttcgg tcaggatgcg 1418520
gtctataatt acgactacgc taccggacgc aaagacatca tccgctttac cgacggtatt 1418580
acagccgata tgctgacttt tacccgagag ggcaaccatc ttcttatcaa ggcaaaagac 1418640
ggcagtggac aagtgactgt tcagtcctat ttccagaacg atggctcagg tgcttaccgt 1418700
atcgatgaga ttcatttcga taacggcaaa gtactggatg ttgccactgt caaagaactg 1418760
gtacagcaat ccaccgacgg ttcggacaga ttgtatgcct accaatccgg aagtacctta 1418820
aatggcggat tgggcgatga ctatctgtac ggtgccgacg gggatgacct gctgaatggt 1418880
gatgcaggca acgacagtat ctacagtggc aatggcaatg atacgctcga tggaggagaa 1418940
ggcaacgacg ccctgtacgg ctataatggt aacgatgcac tgaatggtgg cgaaggcaat 1419000
gatcatttga acggcgaaga cggtaacgac actctgatcg gcggtgcagg caatgattac 1419060
ttggagggcg gcagcggttc ggatacttat gtcttcggcg aaggcttcgg tcaggatacg 1419120
gtctataatt accatgtgga taaaaactct gacactatgc actttaaagg atttaaagca 1419180
gcagatgttc attttatccg ttccggaagt gatttggtgc ttagcgcttc tgaacaagac 1419240
aacgtacgta tttccggatt tttctatggt gaaaaccatc gtgtagatac atttgtcttt 1419300
gatgatgcag ctatcagtaa tccagatttt gccaagtata ttaatgctgg caataatttg 1419360
gtacagtcta tgtctgtgtt cggttctaat actgctgcga caggaggaaa tgtggatgcc 1419420
aatatacaat ccgtacagca gccgttattg gtaacgccat ctgcataagg agcctaatca 1419480
cattcatggc ttaaactgaa aaacagcaat caagtttatt ttgattgctg tttttcttaa 1419540
tattgggata agggtcgtat tttaattaac cttaatcggt gcacttctag caatatagtg 1419600
gattcacaaa aaccagtaca gcgttgcctc gccttaccgt actatctgta ctgtctgcgg 1419660
ctttgtcgcc ttgtcctgat ttttgttaat ccactataat taatatgact ttgcggccgt 1419720
tttgccattg cgtaataaaa cgatggggaa gtgatgataa aacgtgtgtg taactatatc 1419780
agacggcatt gtttttctgt ttgacggcct caatccaaaa ttttgccgac gatttcgccc 1419840
acgtctttcg acaatccttc ctgcgcccga atgcgctgca atgcttgttt caccaagttt 1419900
ttgcggtgcg gctcgagctt gttgcagagg ttgaacgcct gcactaagcg ggcggcgacc 1419960
tgcgggttga agcggtcgat ttcgatgact ttgtcggcga tgaagcggta gccgctgccg 1420020
tcttctgcgt ggaaatgcgg gacgttgcgg ctgaagctgc cgatgagcga acgggctttg 1420080
ttggggtttt cgaggctgaa tttcggatgc tgcaaggcgg ttcgaacctg ttgcagggtg 1420140
tcgctgcggc ggcttgagcc gacgagggca aaatatttgt ccatcaccag cgcgtcgtct 1420200
gaaaacttgt cggcaaactg cgccagcagg cggttgcgcg tatcgctttc gttgccgttg 1420260
acggcggaca ggatgcccca ttcgtgggtc atgttttgcg ccatttcgcc gtatttttcg 1420320
gcaacggttt cgatgtgcgc ggggtcggcg cgcaggacaa aggcgcggca gacgttgcgc 1420380
agcgtgcgcc agccggcggc ttcggggctg tattcgtagc tttggttttc ctgcttcgcc 1420440
gcctgacggt tcaattcgtg ccatttcggc aggaagtgga cggcaagcgt atccaacaag 1420500
gcttcgcgcg cctgatggta gcgcagcggg tcgatgtttt ctgcgccgtc ccacagctcg 1420560
gcttcggatg gcacgcccaa aagcagggct ttgaaggcgt tgtctaagag gtcgtctgaa 1420620

atgacttttt cgacggcggc aagcagtttt tcgtgtttcg gcagctcaac gccgtctgaa 1420680
agcgtggcaa ggttggcggc gacggcgcgg cggtagagcg tttgggcggc ttcccagcgc 1420740
gtgaaggcgt cgctgtcatg ggcgagcagg agcagcaggt cgtcgtcgct gtacggatag 1420800
ttcagatgca ccggcgcgct gaacccgcgc agcagcgagg gaacgacggc ttcggttacg 1420860
ccttcgagca ggaaggtctg ttcggcttcg gtcagcagca acacggcttc ggtcgcgcgt 1420920
ttgccctgat agtcgaatgc caccgcttcg ccgttgcggt tcagcagccc gaccttgacg 1420980
ggaatcatca tcggctgttt atccgtcata tcgggcgtgg gcggcacggt ttgtttgacg 1421040
gtcaactcga aaatattgtt tttcagacga ccttccgctt ccaaaacggg cgtgcccgcc 1421100
tggctgtacc acaaggcgaa ctggtcgaga ttgatgccgt tcgcgtccgc catcgccgcg 1421160
cggaaatcgt cgcaggtaac ggcctgtccg tcgtggcgtt ggaaatagag cttcatgcct 1421220
ttctggaagc cctcttcgcc gagcagggtg tgatacatcc gcactacttc cgcgcctttt 1421280
tcataaacgg tcatggtgta gaaattgttc atctcctcat agctggcggg gcgcaccgga 1421340
tgggcggtcg ggcctgcgtc ttcggggaac tggtgctggc gcagcaggcg gatgttttcg 1421400
atgcggcgca cggcgcggct ggcgcggtcg ccggaaaatt cttggtcgcg gaacacggtc 1421460
agcccttcct tcagcgaaag ctggaaccag tcgcggcagg ttacgcggtt gcccgtccag 1421520
ttgtggaaat actcgtgtcc gaccacggat tcgatgcctt cgaaatcggt atcggtggcg 1421580
gtgcggctgt cggcaaggac gaacttggtg ttaaagatgt tcaaaccctt gttttccatc 1421640
gcgcccatat tgaaatcgcc cacggcgacg accatgaaaa tatccaagtc gtattccaaa 1421700
ccgaagcgcg tttcgtccca tttcatcgcg tttttcaacg attccacggc aaagccgacc 1421760
ttgggcttgt ccgcttcggt ggtgtaaaac tcgattttga cgtttctgcc gctcatggtg 1421820
gtgaaatagt cttccgttac cgccaaatcg cccgcgacca aagcaaacag atagctcggt 1421880
ttggaaaacg ggtcttccca tttcacccaa tggcggccgt ctgaaaactc gccgccgtcg 1421940
attttgttgc cgttggaaag caaaacggga tagcgttttt tgtcggcgac gatggtggtg 1422000
gtgaacttgg acatcacatc cggacggtcg atgtaaaatg tgattttgcg gaagccctcc 1422060
ggctcgcact gggtaaacaa attgccgccg gaagcataca gccccatcag cgatttgttt 1422120
tccgccggca ggatttcggt ttccacttcg acggtgaagc gttcggacgg cacgcccgca 1422180
atcgtcagcg tctctccttc caacacataa tccgccgccg ccccgttgat tttgacggac 1422240
aagagtttcg ccgaaccgtc caacaccagc ggctccccta ccctctgcgg ctcaaccgtc 1422300
aaacgcgact tcacgacggt ttgcggttca ttaatatcaa aatgtaaatc ggttttgaga 1422360
atatggtagg cgggcgtttg atagtctttg agataatgca cggttttgct catttttttc 1422420
tttcaatgtt attttgtttg actggaaaag gcttcagacg gcacgggcgc atcccgcgta 1422480
tgccgtctga agccgcagcg gcggcacggg cgcgccgccg gacaaccggt ttgaattcaa 1422540
tctttattcc cacgcgcgga caaactcttc ccaatgcggc ttttccccgg cttgtgcgga 1422600
caggtaattc cgcatccgtt tgatttccat ttcgtattcg tccgcatcca gcctgccgct 1422660
gaccagacag aaacgcaggt acatcagata agtgtttgcc gcgtcggttt cgcaataatt 1422720
gcggatttcc ttcagcctgc ccgtatggaa tgcctcccaa accttgctgc cgtccatacc 1422780
cagcttgccc ggaaaaccgc acagtttcgc catatcgtcc agcggcacgt ttgccctcgg 1422840
ctggtaaagc gcgagcaaat ccatcaaatc gcagtggcgt tggtgataac ggctgatgta 1422900
gttgttccac ttgaaatcgc ggctgtcgcc gaaatcgccg tcgcccatat cccaatagcg 1422960
cgcggcgttg atgccgtata tcagggagcg gtaatgcagt acgggcagat cgaaaccgcc 1423020
gccgttccaa ctgaccagtt gcggcgtatg tttttcaatc aattcgaaaa atttagcaat 1423080
gaccacttcc tcgccgtcat ccatctcgcc gatggtgccg acatgtactt tatcctgccc 1423140
ccaacgcatg cagcacgaaa tcgccacaac ctgatgaaga tgatgctgca taaaatcgcc 1423200
gcccgtctga gcacggcgtt tttgctgggc aaacagcacc acttcatcgt cgggcagcga 1423260
ggacggcagc tcgtacaatg ttcggatacc ctgcacatcg ggtacggttt caatatcgaa 1423320
agccaaaatc gtggtcatga cagcaccttg tatttaaaac ggatgcacct attgtgtcat 1423380
taaaaggcgg ataaaaaaag aggcaacccc ccacaggatt gccccaatac ctcaaatcag 1423440
agatttacgc ttcacaaaca atacaggctt tcgcctgcgg ctttacccgc gtagctcaac 1423500
tctacgccgg caaactttcg tttcaccgtt tccgatgaaa ccccgaccaa tcgcaagact 1423560
gaccggaaaa tcctttcaga cggcatttcc tgcctgtcgt gtaattccat gtagcgaaat 1423620
gtacgccatt ttctacgctt tgccaagcat tttttacaat ataaatgtca aaacattaat 1423680
tttataaaat tgctgaaaat attaaatata tggattttta ttttttatat ttcaataaat 1423740
ataaatttaa ttttgattta tatttaaatt taagcataaa atgtcaaata ttaaagtaaa 1423800
catgaaaggc atatattaaa tatttattta taacgctatg tttttaaaga aaattaattt 1423860
taatatatta actagattgt ctgcatatat tcataggttt gcggtatttc ttccaaaacc 1423920
tgcttcgaat ttcccgacca agtcttaaaa atattgtttt tgagatactt aaatagcagc 1423980
gattatcaaa tgaaatctgt tcatataatc tgccattttg catttaaaaa aacaatcagg 1424040
agtttcgact cgaaacgcct gatatgtttt gtaattttac gtagtcagta aaaatcgggg 1424100
ctgccttccg gacgggtttt aaaacgcttg tgcagccaaa aatattgttc cggatgttcg 1424160
cgcaccctgt cttcgataaa acggttcatg cgctgcgcgt cggctttcgc gtcttcaccc 1424220
ggaaaggatt tccaagcagg gtagaaatgc aatgtaaccg tattgtctgc ctcgcggacg 1424280

ggaatggcgg gtatcacttt tgcatttgca agcgcggcaa tgcggctcaa tccggtaatc 1424340
gttgccgtct gaataccgaa aaaatccaca aaaaccgaat cgttgcgtcc gaaatcctga 1424400
tcgggcagat acagaaacgg cgcgctgctt ttgcggaact gtttgacgag ggcgcgcagc 1424460
ccttcggtgc gcccgataag gaagacgttg tgatagcggt tgcggccttt caaaatctgt 1424520
tcgtccaata tcttgttttt ttgatgggaa tacatactga tcagcgggat atcctgatta 1424580
agcgcgtaca ccgccatctc gaacgcggtg aagtgcggat acaggatgat gactttttcc 1424640
cccgccgcca gcgcgtcgtc caaataatgc ttattgcggt agcgcaccag cgatttcaaa 1424700
cgtccggcag gcgcgtacca atataaaccg tattccaaca tcagtttcgc catgtgtttg 1424760
aaatgctgtt tcaacacggt tttacgcttt tcctcactcc attcggaaaa acattttgcc 1424820
aaattgattt cgccgatacg gcggcgcggt ttgaccagaa ggtaggcaag caaacccgtc 1424880
aggtcggcaa tcttgtgcag cagcgcaaac ggcagaaact gcaaaacata cagtacaaaa 1424940
aatataaatt tcatctcgat acacattttc ttttcagacg gcaaaataca aatgccgtct 1425000
gaaactattg aaacctgccg cgcttgacct gcatccccga aggattgagt ttggcggcaa 1425060
gcccgtggtt gcgtaaggcg tgggtcagcg cgacggcaag accgtccgcc gcatccggct 1425120
ggggcgttcc cgaaagtccc aacatctgca ccaccatatg ctgcacctgt tcttttgccg 1425180
ccttgccctt gccgactacc gcctgtttga cctgcaaggc cgtgtattcc gaaacgggca 1425240
gcttatggct gaccaatgcc gccaatgccg cgcccctagc ctgaccgagc atcagcgtcg 1425300
atgccggatt gacgttgacg aacacctgtt ccactgccgc ctgttgaggc ttgtaaacgg 1425360
taacgacttc gccgatgtgc cggacgatga cggcaatcct gtctgccaga ggcgcatcgg 1425420
caggcgtttt gatgcagccg gaggcgacgt aaaaatgatc ccgccccctg acatcgatga 1425480
caccgaaacc cgttacgcga ctgcccgggt cgatgcctaa gatacggacg cttgcagcca 1425540
tattcacaac aaaccgtgtt gaatcagctt cttacgcagg gtattgcggt tcagccccag 1425600
catcacggat gctttggact ggttgccgcc gcattgctcc atcacgcaca ccagcagcgg 1425660
tttttccacc tgatgcaata ccatatcgta cacgccgcaa ggttcggtac cgttcaggtc 1425720
tttgaaatat tgttctaaat tttgtctgat gcattgggaa atatcgggaa gggtatgggg 1425780
catgattgca ctttcaaagg ataatcaagt gttcagaagg catttgggcg gtaggcgcac 1425840
gcccaactgt cggttttttc ggcaagtctt tcaagataac ctgcaagcat gtcgtattgc 1425900
gccgccgcac tgtccaagcg gttgatttca cgacgtgtct gttcgccgtc gggcatttcg 1425960
tcgatgtacc agcctatgtg tttgcgtgcg atgcgcacac cggcggtgtc gccgtaaaac 1426020
gcgtgtatgg cgcggatgtg gttcaaaata gcggcggcgc attctgccaa actcaaggca 1426080
ggcggcaaaa caccgtgttc ggcataatgt ttcaaatcgc ggaagaacca cggcctgcct 1426140
tgcgcgccgc gccctatcat aatgccgtcg gcggcggttt gtttgaggac ggcttgggct 1426200
ttttgcggcg aagtaatgtc gccgttgacc cagaccggga tgttcagacg gcatttggtt 1426260
tcggcgatga gttcgtaacg cgcttcgcct ttgtacattt gcgtacgcgt gcgtccgtgg 1426320
acggcaaggg cggcgatgcc gcaatcttcg gcgattttgg cgatgacggg caggttttga 1426380
tggtcgtcgt gccaacccaa acgggttttg agggtaacgg gtacgcctgc cgcacggacg 1426440
acggcttcca aaatggcggc aaccagcggc tcgttctgca tcagcgcgct accggcttgg 1426500
acattgcaga cttttttagc gggacagccc atgttgatgt cgataagctg cgccccaagg 1426560
ctgacgttgt aacgcgcggc atccgccatc tgctgcggat cgcttccggc aatctgcacg 1426620
gcaacaatgc cgccttcatc ggcaaaatcg ctgcggtgca aggtttttct agtatttctg 1426680
agcgtcgggt cgctggtcag catttcgcac accgcccaac ctgcgccaaa atctcggcaa 1426740
agtcggcgga acggtttgtc ggtaatgccc gccatcggcg caagtgcgat ggggttgtcg 1426800
ataaaatagc cgccgatgtg cataatggat ccgcgtttca aaaaagtacg ccattgtaca 1426860
ttttttaagc aggatttcca atctccggac gcgcccgcga ttgggtcgga caccgtttta 1426920
tggcataatc cgcacacaga ttccctgccc cgccactcac aggcgggcag tttatagtgg 1426980
attaacaaaa accagtacgg cgttgcctcg ccttagctca aagagaacga ttctctaagg 1427040
tgctgaagca ccaagtgaat cggttccgta ctatctgtac tgtctgcggc tcgccgcctt 1427100
gtcctgattt ttgttaatcc actatatttc cccgtcctat cggtttcccg tttcagacga 1427160
cataaggtct gaaagaaaga ctacaattat gagtaatcca ttttcctctt taggtttggg 1427220
tacggaactc gtttccgcac tgaccgcgca aggttacgaa aacccgacgc ccatccaagc 1427280
cgccgccatt cccaaagcac tcgccggtca tgatttgcta gccgccgcgc aaaccggcac 1427340
aggcaaaacc gccgccttta tgctgcccag tctggaacgc ctcaaacgtt acgccaccgc 1427400
cagcacctcg cccgcgatgc accccgtgcg tatgctcgtc ctcaccccca cgcgcgaact 1427460
tgccgaccaa atcgaccaaa acgtgcaggg ctacatcaaa aacctgccgc tgcgccacac 1427520
cgtcttgttc ggcggtatga atatggacaa acagaccgcc gacctgcgtg ccggctgcga 1427580
aatcgtcgtc gccaccgtcg gacggctgct cgaccacgtg aaacagaaaa acatccattt 1427640
gaacaaagtc gaaatcgtcg ttttggacga agccgaccgt atgctggata tgggttttat 1427700
cgacgacatc cgcaaaatca tgcagatgct gccccgccaa cgccaaaccc tgctcttttc 1427760
cgccaccttc tccgccccga tacgcaaact ggcgcaagac ttcatgaacg cgcccgaaac 1427820
cgtcgaagtc gccgcgcaaa acaccaccaa cgccaacgtc gagcagcaca tcatcgccgt 1427880
cgataccatt cagaagcgca acctgctcga acggctgatt gtcgatttgc atatgaacca 1427940

ggtcatcgtg ttctgcaaaa ccaaacaaag cgtcgaccgc gtaacgcgcg aactggtgcg 1428000
ccgcaacctg tccgcacagg cgatacacgg cgaccgttcc caacaaagcc ggctcgaaac 1428060
actcaacgcc ttcaaagacg gcaacctgcg cgtcctcgtc gccaccgaca tcgccgcgcg 1428120
cgggctggac attgccgaac tgcccttcgt catcaattac gaaatgcccg cccagcccga 1428180
agactacgtc caccgcatcg ggcgcacggg gcgcgcgggc gcggacggcg tggcgatttc 1428240
cctgatggac gaatccgaac agaaaatgtt tgaatccatt aaagagctga ccggcaacaa 1428300
gctgctcatc gagcgcatcg agggcttcga gccgcaatgg tgggaacagg gcggcgcaaa 1428360
accggaaaaa cccgaaatgc gcgaaccgag acaacgcaac cgctacgaat ccgccaaagc 1428420
gcaacgcgaa aaaaacaccc ggccggaaaa tgcggcaaac gatgcgggcg cggcttgcgg 1428480
aaaaattgcc ggacgcagcc gccgaagccg ccgggaacac cggacgtgcg ccctgctcca 1428540
accgcgttac ggcgtaaaat agccctgaaa atcaaatgcc gtctgaacat ttcccgtttc 1428600
agacggcatt tttcaaaccg gactgacgca tcgggagcaa ccgcccgcac cggataaatt 1428660
tctgccgcaa acagtttcag acggcatttg ccgcctgtac aatatagtgg attaacaaaa 1428720
attaggacaa ggcggcgagc cgcagacagt acaaatagta cggaaccgat tcacttggtg 1428780
cttcagcacc ttagagaatc gttctctttg agctaaggcg aggcaacgcc gtactggttt 1428840
aaatttaatc cattaatagt gtatattaag tacgtctgat atacacgata ccctacgagg 1428900
gtgtaagctt tagttcacat ttaaaatgac ctctttaaac ctgtctttcg gcaggtttct 1428960
ttttaggttg tttggaaatc gtgtgcagac aaggtgtaaa ataggtaaca gcataaaata 1429020
atgcggtttt accgcccata tatttacaaa agccaaattt ttaaacatat atccttgata 1429080
tatacacggc gtaaacatat actggaaaca tctttaaatt ttccgaaatt ttaaatatga 1429140
gcaactggaa acccaatatt ccctataacg atttaccacc cctgccgcca aaacaggata 1429200
ttgaaagcaa aaccatcctg aaacgttgta tagccgcccg tgcatccctt gcccgtttaa 1429260
agcaggcggc agaattgata ccgaatcaag ccatgctgat taacaccctt cctgttatgg 1429320
aagcccgtgc aagttcggaa attgaaaaca tcgtaaccac cacggacaag ctgtttcaat 1429380
ccctgcaaat ggatacggaa cggcaagacc ctgccacgaa agaagccctg caataccgca 1429440
ccgccctgtt tgcaggctat gaatcactga cgagccgccc tttatgcaca caaaccgcca 1429500
tcatggtctg caacgccatc aagcacccct acgaaatggc catccgcaaa acaggcggca 1429560
cagccctaaa aggaggcaac agcggaaatg ttgtctatac cccgccccgaa ggagaagaaa 1429620
ccatacgcgg caagctggca aattgggagc ggtttattca cgaaagcggc gatttagacc 1429680
cgcttatcat catggcggcg gcacattacc aatttgaagc catccatccg tttacggacg 1429740
gcaacgggcg gacggggcgc atattgaaca gcctgctatt gattgaaaaa gggcttttgg 1429800
atttgcctat tttgtatttg agccgctaca tcatcgaaaa cagggcggac tattaccgcc 1429860
tgcttttagg cgtaaccgaa cggcaggact gggaaagctg gataatctac atcttagacg 1429920
gcgtagctga caccgccgat tggacggtat cgaaaataga tgcgatacgc cgcctgttcg 1429980
agcagacacg gcaacacata cggacacacg cacaaggaat ctacacgcac gaactggtaa 1430040
atcttctgtt tgagcagcca tatacacgca ttgccaacct agaagcggca gggatagcca 1430100
aacggcagac ggcctctaag tacctgaaag agctttcaga cataggtgtg ctgcaagaaa 1430160
tcgtcatcgg cagggacaaa ctattcattc atccgcgcct aatggaacta ttgcggggag 1430220
agggcaacag ctttacctca ttccaatccc tcgttaaagc atagccaaaa taatcaataa 1430280
tccggaggtc aatatggcaa gaaggtcaaa aacatttgaa gaagctgctg ctgaggttga 1430340
ggaacgtttc ggtcatcgtg gcattaagtt ggtcgagttt gagggtacag ccaagccgtg 1430400
tgtaatcaac tgccctaaac atggaaacca aacctgttcg aggtactcca atatgttcat 1430460
aggaagtagc tggggttgcc cctcttgtgg taatgagcaa gctgcaaaag ccggtatagc 1430520
gacccttagg aagaatcaca tagcgttaga aatgctgaaa caggctgtaa caggtatgac 1430580
caagcaagag cgcatcacga cgcaagccta caatgagatg accaaatccg tggcaggttc 1430640
aaacagcata gtccttaacg atgtccaagg cgatacgacc atcaacaacc atcatacgca 1430700
tacgcacaac cacagcgatg ccgatggcaa agcactgtcg atgaggctca caccccgtcc 1430760
tttgttgtca gaccgtcagg cggcggcttt cgcccgtaca ggcaaactca cgggcagttt 1430820
cgacctgttt gcttcggtgg tcgccccctc gcagtacacg tttgccgttg ccatgcccga 1430880
cacgtccatg tcgccggtta tcgaaaaggg agacttgctg gtggtcgagc cgcgtatgtg 1430940
ccctgcggac gaagacatcg cgctgattga actgtccgac aagcggctgg tcgtcgcgca 1431000
ccttgttatc gatattgcgg gcaggatgct gatttatcag acgggcaggc cgtctgaagc 1431060
ctttgacctg cccgaaggca gcacgatttt aggtgtggtg ctggagtcaa aaaacggttt 1431120
atgtccgccg cacaggcaag aaggcgtgtt gattcggatt accgcccctg atgtgtggac 1431180
ggttggtatg atttccgctt ccaaaacgtc gtgtacgcgc ccgaccgcag cccggaaatc 1431240
agccgtatgc tttcttcgat tttggcaggc tacgcgtggg ataccgaaaa cccgttcgtg 1431300
gcgaaatccg aacaacgcct gactgccttg tccgaatggg tcggtcagtt ggaaaccgaa 1431360
taaatccgta ccgccataca aaatgccgtc tgaatccaat cgggttcaga cggcattgcc 1431420
atttcaactg tttttatgat tactcggggc gcatctgcgg aaacagaatc acatcgcgga 1431480
tggtttgcga atcggtcagc agcattacca agcggtcgat accgatgccg caaccgccgg 1431540
tcggcggcaa accgaattcc atcgcgcgga tgtagtcggc atcgtagtgc atggcttcgt 1431600

cgtcgcccgc gtctttttgc accacttgcg ctttgaagcg ttcggcttgg tcttcggggt 1431660
cgttcaactc ggaatagccg tttgccagtt cgcggccgac aacgaacaat tcgaaacgtt 1431720
cggtcagacc ttgtttggta tccgaagcgc gcgccaacgg tgaaacttcg accgggtaat 1431780
cgacgatgaa ggtcggattc cacagcttgc cctcggcgca accttcaaac agcgcgagtt 1431840
gcaggctgcc gatgcccggg gacggcggca ggctttcgcc gtgtttgacg atttcttttt 1431900
tcagccattc cgcatcgttc aactgctcgt cggtgtagtg cggattgtat tttttgatgg 1431960
cttcgagaat ggtcaggcgt tcaaacgggc tttccaaatc gacttctttg ccgttgtaag 1432020
tgatgtttgc cgtgccgttt accgtgcgcg atgcgttgcg gatgatgtct tccgccatct 1432080
gcatcatgcg ttcgtagtcg gagaaggctt cgtagaattc gatcatggtg aattcggggt 1432140
tgtggcgcac ggacatgcct tcgttgcgga agctgcggtt gatttcaaac acgcgttcca 1432200
aaccaccgac aaccaggcgt ttcaaataca gctcaggcgc gatacgcagg taaagcggaa 1432260
tatctaaggc attgtgatgg gtaacgaagg gttttgccgt cgcgccgccg ggaatcgggt 1432320
gcatcatcgg ggtttcgact tcgagataat gctcgcccac cataaaatta cgcacggatt 1432380
ggatgatttg gctgcgtttg ataaaggtat tgcgcgattc ttcattggca atcaaatcaa 1432440
catagcgttg gcggtatttg gtttcctgat cgctcaaacc tttgtgtttg tcgggcagcg 1432500
ggcgtaggga tttggacagc aggcggatgc cggacacgcg tacggtcagt tcgccgtggt 1432560
tggttttgaa caaagtgcct tccgcgccga cgatgtcgcc caaatcccaa tggttgaagt 1432620
cgtccaaaac ttcttggctc acgcctttgt tgttcagata aagctggatt tgcccggaca 1432680
cgtcttgaat ggtggcaaaa ctcgccttgc ccatttgacg cttcagcatc atgcggccgg 1432740
ccactttgac gggaatgcct tgcggatcga gttcttcttt gccgatttcg ccgtattggg 1432800
cgtgcaaatc ggcggcgaag ctgtcgcgtt tgaagtcgtt gggataggcg ttgcgctgtt 1432860
ggcggatgtt gtgcagtttt tcgcggcgca gggcgatgat ttggttttcg tccaactgcg 1432920
gctcggtttg cggatggttt tgttcgctca taaggttttc cgaaaaaaata aatcaggcgc 1432980
aatctgtttc agacgacctg accgaatcac aaaatttgcg catattttac gcgatgtcgg 1433040
catttttttc cataaacgcg acaatgccgt ctgaaagcgg tttgcggttt cagacggcat 1433100
cgttatcatt tgaacattcc cgccaaattc aataagaaca aaacggtaaa accggtcaga 1433160
taaatcaagc ctgccaatgc aagggcattc atacctgatg tgagtttgtg tttttcatca 1433220
cctttaacca aacggtaatt cagccaggca aacacagggg cggacacaaa agcggcaatc 1433280
atcgcaaatt tgagcagatt cgccattacg ccgtcaaacc agaaaatcac cgccaaaccg 1433340
ctgcccgcca cccaaatatt ccaggcaaag aattcggcgt tgcccgtttt gtcttttccg 1433400
cgcagcaggc gcacgggttc ggcaatggca cgggcatagc cgtccacgac ggtaatcgtc 1433460
gtgccgtaca tacaggcaaa cgcgataaac gccaccagcg ggcgcgacca gccgccgatg 1433520
gtaacggcgt acatattgat caattgcccg atatatttgc cgcccgccat ctgcactgct 1433580
tcgccgttgc cgtattgcac aaacgcgccc agtgcaagga aaaccaaagc caaaaccgca 1433640
ctggcgatat aaccgacgtt gaaatcaaaa atcccgtcgc ggtattcgga aggattgatg 1433700
cgttgttttt cggttaccca caaagaattg atggcggaaa tttcaatcgg cgcgggcatc 1433760
cagcccatca gcgcgatcag gaagcccaaa ccggcaagcg tccacggtgt cggctcgata 1433820
aaatcggact gcatctgcat accgcgcgac atagcgatgc cggcggcggc aagcgtggcg 1433880
atactcaaag taacgatgat gattttggaa acgcgatcca aagcgcggta acgtccgctc 1433940
accaaaataa tcaggcagga tgccataatc aaggcggcaa ccgtgccggc atcaaacatc 1434000
agcgagggaa tcgccatttt gacgatggcg gcggttacaa tggcgaccgc gcccgcgtta 1434060
atcgtggcgg agaggatgca caaaatcagg aatacccaca aataaacgcg gcttttctcg 1434120
gcataacctt caatcaggct cttgcccgtg tccagcgtgt aatgcgcgct gaagcggaaa 1434180
aacgggtatt tgaagaggtt ggtcaggatg atgatgagcg cgatctgcca gccgtaaagc 1434240
gcgcccgcct gcgtcgaggc aatcaggtgc gaaccgccga ccgccgccga agccatcatg 1434300
atccccggac ccaatgcgtt gattttactt ttccaagtcg aaatatgttg ttcggacata 1434360
aagtcttccg tatttttaac tgtgtttcaa cacacagagc cgcatattcg gacacagccc 1434420
tatctattgc tccaatttgg gcgggattgc cccaaacaa acccaaatcc taccgtcttc 1434480
aaaaacagga taccgcccgg tagggaaatt ttgatgaaaa cacgtattgt aacgtaatcc 1434540
aaatacctgc caacacacac tattagaact tcatgctcaa acttgactat attttccata 1434600
ttacttccaa aaaaaggcat aaaacgacat tttatgccta aaattttaca acaaacaacc 1434660
ttacatcgct tttttcgcgc aaacacgcac catccgatca gcccgtccgt tttgcagcag 1434720
gctggcgatt tgataagatg gttatgtttt tcagacggca tttcagattt ccgtccatgc 1434780
catctgaagc cgcaaaaccc gattggagga actgttatga ataccgtatc gaattatctg 1434840
tccgcattac gcgaagccat gaaggcgcaa ggcttggatg cactcgtcat cccttccgcc 1434900
gaccccccacc tgtccgaata cctgcccgag cattggcagg cgcgccgcga attatcgggc 1434960
tttaccggct cggtcggcac gtttgtcctg accaccgatg aagcgggcgt gtgggtggac 1435020
agccgctatt gggaacaagc cgccaaacag cttgcgggca gcggcattgt gctgcaaaaa 1435080
agcgggcaag tgccgccgta caacgaatgg ctcgcggcaa gcctgcccga aaacgccgcc 1435140
gtcggcatcc cttccgatat ggtctcgctc accggcaaac gcactttggc gcaatcactc 1435200
gccgccaaaa acatccgcat cgaacacccg gataatttac tgaatcaagt gtggacaaac 1435260

cgccccgccc tccccgccga aacggtgttc atccacgacc ccgactatgt ttctgaaacc 1435320
gccgccgaaa aactcgcccg cgtgcgcgcc gtgatggcgg aaaaaggcgc ggattaccac 1435380
ttggtttcct cgcttgacga catcgcctgg ctgaccaacc tgcgcggcag cgacgtgcct 1435440
ttcaatcccg ttttcgtgtc cttcctgctg attggcaaag acaacgccgt cctgtttacc 1435500
gaccgatgcc gtctgaacgc cgaagccgcc gccgcgctgc aaaccgccgg catcgcggtc 1435560
gaaccttacg cccaagttgc cgacaaactc gcgcaaatcg gcggcgtgct gctcatcgag 1435620
ccgaacaaaa ccgccgtcag cacgcttgtg cgcctgcccg aaagcgtgcg ccttatcgag 1435680
ggaatcaacc catccacgct gttcaaatcc tgcaaatccg aagccgacat cgcccgcatc 1435740
cgcgaagcga tggaacacga cggcgcggcg ttgtgcggtt tcttcgccga gtttgaagac 1435800
atcatcggca acggcggcag cctgaccgaa atcgacgtgg acaccatgct ttatcgccac 1435860
cgcagcgtgc gcccaggctt catttcattg agtttcgaca ccatcgcagg cttcaacgcc 1435920
aacggcgcac tgccgcatta cagcgcgaca cccgaaagcc acagcaccat cagcggcaac 1435980
gggcttttgc tcatcgactc cggcgcgcaa tacaaaggcg gcacgaccga catcacccgc 1436040
gtcgtccccg tcggcacgcc gagtgccgaa caaaaaagcg acaacaccct cgttctcaaa 1436100
gcccatatcg cgcttgccga agccgtgttc cccgaaaaca tcccctcgcc gctgattgat 1436160
gcgatttgcc gcaaacccct gtggcaggcg caatgcgact acggccacgg caccggacac 1436220
ggcgtaggct atttcctcaa cgtccacgaa ggcccgcagc gcatcgcctt cgccgccccc 1436280
gccacgcccg aaaccgccat gaaaaaaggc atggttacct ccatcgaacc cggactctac 1436340
cgcccgggaa aatggggcat ccgcattgaa aaccttgccg ccaaccaagc cgtcgccgcc 1436400
cctcaagaaa ccgaattcgg cagcttcctc tgttttgaaa ccctgaccct ctgccccatc 1436460
gacacccgcc tgatggacac cgccctcatg accgacggcg aaatcgactg ggtcaaccgc 1436520
taccacgccg aagtccgccg ccgcctcgag ccgctgaccg aaggcgcggc aaaagcgtgg 1436580
ctgatcaaac gcaccgaacc gctggcgcgt taaacagcac ggcgcaaaaa atgccgtctg 1436640
aaagcccttc agacggcatt ggtttcccaa aacatcccgc accgttttca tcttgccgca 1436700
agcaaatata gtggattaac aaaaatcagg acaaggcgac gaagccgcag acagtacaaa 1436760
tagtacggaa ccgattcact tggtgcttca gcaccttaga gaatcgttct ctttgagcta 1436820
aggcgaggca acgccgtact ggtttttgtt aatccgctat attccgccat ctctaagatt 1436880
tacagcgata cacgggtgat ttaaggaatg cccgaaccgt cattcccgcc acttttcgtc 1436940
attcccacga aagtgggaat ctagaaataa aaagcagcag gaatttatcg gaaataactg 1437000
aaaccgaaca gactagattc ccgcctgcgt gggaatgaca attcgagacc tttgcaataa 1437060
cataggttac taaaatttta tgctcaatct cattttcaaa atgcaaaact tttctgattt 1437120
ttcctacttt ttgctcaata ttaggaaggt tttaggcaat tgaaaatttt ttggcgcatt 1437180
tttatgcgtc aaatttcgtt aacagactat ttttgcaaag gtctcactat atgtgcaaac 1437240
caagccaaaa atgcgaaata ccgtctgaaa atctttcaga cggtatttgc tgtctttatt 1437300
gccgtttttc ttccgtatcc ggatttttgt ttggggctga agcagattgg cagtcagatt 1437360
gcaatcaaag aatgaaggcg agccgtcaaa aacaaagcta tccgcttcac cgccccgata 1437420
tttagaattt gtggcgcaaa ccgacggagg cggcattaat ttgagtgtag ttgccgatgc 1437480
cggtattgcg tttcagccaa gcgccagaca cgatggcgga agtgcgtttg gaaaaatcat 1437540
aatcaacgcc ggcgatgatt tgatcgtagc tggtattttc gccttttttta ccgcgttcga 1437600
taaagtcgaa accatgggca tagctgatgc gtggaactgc attaccgaag cggtaggaag 1437660
cagtggcggc aatttcggtc gtactgtttt tggttttgtc gccattttca gacaaatcca 1437720
actgagccgc caaggcgaga ttcaagccgc cttcctcata gccgcccgtc agacggtgta 1437780
cctgatggtt tttcaaggga tcggtacctt tggcttgatc actcccgctg ccgatcaaga 1437840
acaactcaaa agcattacgt ccgacattgg cgtgtctcgc atatttaaag gcatagttcc 1437900
cggcaaaacc gccatttttg taattcagac cggcataata cacatccgat ccgggcttgc 1437960
cgacaacagc cggaacgaga gtaagattat tgtttgtatt cttagtataa taagccggcg 1438020
tataggcgga cttgctgttt tggatcggaa cgaattgaac gctgccgctg aaaccggaaa 1438080
attcggggga atcgtagcgt acggaaaccg gcatgtcgtc gtggcgtttg aaaataccca 1438140
attgcgaagc cacatcatta ttgctgtccc aaggatcaat ggcttggctg gcatcgtcaa 1438200
actgattcgc aacgcgaccg gcgcgcagcg taccgaattc gcctgccaag ccgataaagg 1438260
attccctgtt gccccactgg gtcgcgccgc cgccggcaac ggatacgtct tgctcaagct 1438320
gccaaacagc cttcagcccg tcgcccaaat cctcactccc cttaaagccg ataaacgagc 1438380
cgaaatcact gattttcgtc ctgatgcggc ttttggcctt agtaacttta gtaactttta 1438440
cctgaccgct cgctccaccg ttagcggctt gtgcttcagt caattgcagc tggtagttcc 1438500
tgccttccac gccggctttg atttcgccgt ataggctgac atcggcaacg gccgcaagcg 1438560
gcagtgcgga caatacgagg gcggtaagtt tttttcgcat atcggcttcc ttttgtaaat 1438620
ttgataaaaa cctaaaaaca tcgggcaaac acccgatacg tcttcaatta tacccccccc 1438680
cccgcaaaaa accatttttc agaacaaata tctgataaat gccgcaacct ttattttaaa 1438740
aatgattata ttttgatata aaacaatagc ttattttttc aaaaacgttg tgtttctaca 1438800
acacaattca agcgcagacc tcgtgcgagc cgatgcgctg ctgcccggat gcagtctcgg 1438860
ctttttaaaa cgccataaaa aaacacacgc ggcactttat agtggattaa caaaaacaag 1438920

tacggcgttg cctcgcctta gctcaaagag aacgattctc taaggtgctg aagcaccaag 1438980
tgaatcggtt ccgtactatc tgtactgtct gcggcttcgt cgccttgtcc tgatttttgt 1439040
taatccgcta taaagaccat cgggcatcta cagccgtcat tcccgcgcag gcgggaatct 1439100
agaatttcaa tgcctcaaga atttatcgga aaaaaccaaa acccttccgc cgtcattccc 1439160
acgaaagtgg gaatctagaa atgaaaagca gcaggaattt atcggaaatg accgaaactg 1439220
aacggactgg attcccgcct gcgcgggaat gacgggattt taggtttctg attttggttt 1439280
tctgtttttg agggaatgac gggatgtagg ttcttaggaa tgacgtggtg caggtttccg 1439340
tacggatgga ttcgtcattc ccgcgcaggc gggaatctag aatttcaatg cctcaagaat 1439400
ttatcggaaa aaaccaaaac ccttccgccg tcattcccac gaaagtggga atctagaaat 1439460
gaaaagcagc aggaatttat cggaaacgac cgaaactgaa cggactggat tcccgcctgc 1439520
gcgggaatga cgggatttta ggtttctgat tttggttttc tgtttttgag gaatgacggg 1439580
atgtaggttt tcttaaccct gcgtcctaga ttcccacttt cgtggtaatg acgggatgtg 1439640
ggttcgtggg aatgacgtgg tgcaggtttc cgtgcggatg gattcgtcat tcccgcgcag 1439700
gcgggaatct agaccttaga acaacagcaa tattcaaaga ttatctgaaa gtccgagatt 1439760
ctagattccc gctttcgcgg gaatgacgaa aagtggtggg aatgacggtt cagttgctac 1439820
ggttactgtc aggtttcggt tatgttggaa tttcgggaaa cttatgaatc gtcattcccg 1439880
cgcaggcggg aatctggaat ttcaatgcct caagaattta tcggaaaaaa ccaaaaccct 1439940
tccgccgtca ttcccacgaa agtgggaatc tagaaatgaa aagcaacagg aatttatcgg 1440000
aaatgaccga aactgaacgg actggattcc cgcttttgcg ggaatgacgg gattttaggt 1440060
ttctgatttt ggttttctgt ttttgaggga atgacgggat gtaggttttc ttaaccctgc 1440120
gtcctagatt cccgcttttg cgggaatgac gggatgtggg ttcgtgggaa tgacgtggtg 1440180
caggtttccg tgcggatgga ttcgtcattc ccgcgcaggc gggaatccag accttagaac 1440240
aacagcaata ttcaaagatt atctgaaagt ccgagattct ggattcccgc tttcgcggga 1440300
atgacgaaaa gtggtgggaa tgacggttca gttgctacgg ttactgtcag gtttcggtta 1440360
tgttggaatt tcgggaaact tatgaatcgt cattcccgcg cagacgggaa tctggaattt 1440420
caatgcctca agaatttatc ggaaaaaacc aaaacccttc cgccgtcatt cccacgaaag 1440480
tgggaatcta gaaatgaaaa gcagcaggaa tttatcggaa atgaccgaaa ttgaacggac 1440540
tggattcccg cctgcgcggg aatgacgaat tttaggtttc tgattttggt tttctgtttt 1440600
tgagggaatg acgggatgca ggttttctta accctgcgtc ctagattccc gcttttgcgg 1440660
gaatgacggc gacagggttg ctgttatagc ggatgaacaa aaaccagtac ggggttgtct 1440720
cgccttagct caaagagaac gattctctaa ggtgctgaag caccaagtga atcggtttcg 1440780
tactatctgt actgtcttcg gcttcgtcgc cttgtcctga tttttattaa tccactataa 1440840
tttcctgcgt gtgtcgggtg tatcgaaatc aagccgaatc aaatatatcg gacttcgata 1440900
atgtcgtatt cgcgcacgcc gcccggggct tggacttccg ccgtatcccc ctcttccttg 1440960
ccgattaagg cgcgggcgat gggtgagccg acatagattt tgccctgttt gatgtcggct 1441020
tcgtcttcgc cgacaatttg atagataacg tgttcttccg tttccaaatc ttccagcgta 1441080
accgtcgtac cgaacacgat tttgccttcg gcgtggattt cggtcggatt gatgatgtgg 1441140
gcaacggaaa gtttgtgttc cagctcggaa atgcggccct cgataaagcc ttggcgttct 1441200
ttggcggctt cgtattcggc gttttcggac aaatcgccgt gcgaacgggc ttcggcaatc 1441260
gcttcgatca cttcgggacg cgccacgctt ttgagctgct gcaattcctg tttcagcaat 1441320
tccgcaccgc gtacggtcag gggggatttttt tgcatcggtc tttttctcc atattccggc 1441380
acaccggttt gcggcagcaa gcataccgcg taccgtcttg ttttgtgcgt ccggatatta 1441440
aaataaaaat acaagccgcc cggaaaatcg gcggcttgtc tgtcgttgaa cagcggctat 1441500
tctaccaaat tctatgaaat tggcaatcgt gccgcgccgc cggcaaacgc gccatgtccg 1441560
caacaaaagc tgaaaatatg ccgacaaaga aattttagaa acaaaaaatt taaaaataat 1441620
caattttcgg cataaaaaac cacatttacg gactttaaaa ccgaaaatgc caagcctgag 1441680
atttttcata cagcatttgc accagtataa tgcaggctgt ttttatcttt aataatattg 1441740
acgttttgcc atgaccgaat ccgtccgcct ccccgtcgcc cgtctcaaac cttccaccgt 1441800
cgccctgccc ggctccaaaa gcatcagcaa ccgcaccctg ctgcttgccg ccttgtccga 1441860
caatgcttgc gaaatccatt ccctgctcaa atccgacgat accgaccgta tgctcgaagc 1441920
actcgataaa ctcggcgttc aaatcgaata tcttgccgaa gaccgtctga aagtgcacgg 1441980
cacaggcgga cgcttcccca accgcactgc cgatttgttt ttgggcaacg cgggcacggc 1442040
gttccgcccg ttaaccgccg ctctggccgt tttgggcggc gattatcatc tgcacggcgt 1442100
gcctcgtatg cacgaacgtc ctatcggcga tttggtcgat gcgttgcgga ttgccggggc 1442160
cgatgtcgaa tatctcggca aggaacacta tccgccgctt catatcggcg aacgccaaga 1442220
caacggcgag cgcgtgattc cgattaaagg caatgtgtcc agccagtttc tgaccgccct 1442280
tttaatggcg ttgccgctga ccgggcaggc gtttgaaatc cgtatggtcg gcgaattgat 1442340
ttccaagccc tatatcgaca ttactttaaa actgatggcg caattcggcg tacaggttat 1442400
caatgaaggc taccgcgtct tcaaaattcc cgccgatgcg cactaccacg cgcccgaaca 1442460
cttgcacgtc gaaggcgatg cctccagcgc gtcctacttc ctcgcagccg gtttgattgc 1442520
cgccacgccc gtccgcgtta ccggtatcgg cgcaaacagc atacagggcg atgtcgcctt 1442580

tgcccgcgag ctggaaaaaa tcggggcgga cgtggtttgg ggcgaaaact tcgtcgaagt 1442640
ttcacgcccg aaggaacgtg ccgtccaatc ctttgatttg gatgcgaacc atatccccga 1442700
tgccgccatg accctcgcca tcgtcgcgct tgctacaggg caaacctgca cgctgcgcaa 1442760
catcggttcg tggcgcgtca aagaaaccga ccgcatcgcc gcaatggcaa acgagttgcg 1442820
caaactcggg gcaaaagtcg tcgaagaagc cgaagcaatt cacatcaccc cgcccgaaac 1442880
gctgacaccc gacgccgtca tcgacacgta cgacgaccac cgcatggcga tgtgtttctc 1442940
gctggtttcg ctgttgggcg tacccgtcgt catcaacgat ccgaaatgca cccacaaaac 1443000
cttcccgact tatttcgacg tgttctcatc gctgaccgaa acagcggaat aaggcggcat 1443060
tttgccgcga ttccggcgcg gcgcggcggg cggctcattc tgtaaaaaaa gtatgtgcgc 1443120
cgaggtagtt tttggcgtaa aacggtgtgg agagtttttc ggttttgatg gttttgccgc 1443180
tgctgggggc atggatgaat tcgccgttgc cgatgtagag tccgacgtgt gagtagcggt 1443240
gtgcgccgcc ggtgttgaag aatacgaggt cgccggcctt gaggcggctg tcggggattt 1443300
tgcggcttgc cgccgccatg tcgcgggcgg tgcgcggcag cttgacgttg agggcgtttt 1443360
tgtaaacgaa ttgaatcatg ccgctgcaat cgaagccggt tgcggtgctg ctgccgcccc 1443420
atttgtaggg cgtgccgatg agtccgaggc tgtggagcat gagttcctgc gagccttgtg 1443480
tgcggtcgat gtggctgatg cggacggctt ggatttgccg gactgtctgt ttgggtttcg 1443540
gttggcggtg tttgccggag gtcgtgccgc atgaggcgag gagcagtgcg ctgagacaga 1443600
ggaaaagggt tttgtcgggg ggaaacatgg tttttccttt gcgggttcgg atatccgtct 1443660
gaaggtgttt cagacggtat agtggattaa caaaaatcag gacaaggcga cgaagccgca 1443720
gacagtacaa acagtacgaa accgattcac ttggtgcttc agcaccttag agaatcgttc 1443780
tctttgagct aaggcgaggc aacgtcgtac tggtttttgt taatccgcta tatttctata 1443840
ataaaccttc tatgggcagc agggatagga tttttgcggc gatgcgtttc caaagtttgg 1443900
cttcgggttc gttcgggtag gtttttcggg tggcgggatc gtgccattgc aggcggttgt 1443960
gcctgtcgag ggtaacgcgg taggcgtagg cgggtgtggt atcggcaagg gtgcgctcca 1444020
tctgttctgc gattttgggg ctttcgataa caacgcccat ttcggtgttg agacgcgcgg 1444080
aacgggggtc gaggttgaac gaaccgatga agatgcgttt gccgtccaca atgaaggttt 1444140
tggcgtgcag gctggttacg gagctgccgg tcaggccttt gtcttttgtg gcggggacgg 1444200
catggttggg ttgcagctcg tagagtttga tgccggcttt gagcagcggt tttcggtatt 1444260
tgacatagcc ggaatggacg gcggcaacgt cggtcgcctg cagcgagttg gtcagaacgg 1444320
taacgtctat gccgtcctgc accagttttg ccagtgcgtc tgtgccggat tttgtgggaa 1444380
cgaaataggg tgaaaccaga tagacgcttt tttcgggctg tttgagcgcg tcttgcagcc 1444440
gcccggcaat cggcggtttg cggcggtcgc ggtcgagtcc ttttgcaggg tcgtcgctga 1444500
tgaggcgggt tcggacgctc tgccagtcga tgcatcctgt ctgtattttt tggtagaggg 1444560
gcgactgttc gacggtttcg cggtagcgca ggagcgcgtg tctggacgtt tcgtcgttgt 1444620
atccgagtgc ttgaagaccc ttgccgatgt cgcccgctgcg gatgatgcgc gtggcgttgt 1444680
gggcggaatg gcttgcccag tagcggtcga agtcgtgcga tacttcgccg acgacgctgc 1444740
cggtggcgag gatgtccaaa tcggcgaaaa cggtgtcctc accgactttg aagtattcgt 1444800
cgccgatatt gcgtccgccg agtatggtgg cgcggttgtc ggcggtaaag gatttgttgt 1444860
gcatgcggcg gttgaggcgg gggaagtcgg tcaggtagcc gagtgcgcgc cattttcgta 1444920
agacgaaggg gttgaacagg cgcacttcga tattgggatg gctgtcgagg gcaagcagga 1444980
ggtcgtccaa tccgcgcgtg ttgttgtcgt ccaacagcag gcgtacgcgc acaccgcgtt 1445040
ctgcggcaag gtacacgagg ttgaacagca gcctgccgga aatgtcgttg cgccagatgt 1445100
agtattgcaa atcgaggctg tgttcggcag attcgataag ggcggcgcgg gcggcaaagg 1445160
cttcgtgggg gtcgttcaac agatagatat cggatagccc gttggtatga ggggtgtgcc 1445220
ggatttgcag gatgttgtcc aggcggacgg gtttggaagt attgaaatga cggctttccg 1445280
tccgttcttc cagtgggggc aaccatgaag aacatgaaca gagaaggagg cataaaaggg 1445340
aaattaggct gcgtgttttc atcagggata tggtttcaga cggcattgcc tgtgttttgg 1445400
ggttggcgcg catggaagtg cggtatcata atccaaacgt tgaaacgggt aaaagttttg 1445460
cgtgtggacc gcttcaggac ggtgtgttcc gtgtcaggtt ggtgccgtct gaaacgtgca 1445520
gccgtttgaa aaccagcgat gatgcaaggg tgatgccgcc gatgctgagc agggtcatac 1445580
ggaaggcgga atgcagacct gaagaagccg gtatcagaaa tgtccagttt ttaaggatta 1445640
atgcgccggc aacaatgccc atgctgatgg caagctgttg gttgaccgcc atcaggctgt 1445700
tgccgctgcc tgtttgttgc gggcgcaaat cggcgagggt cagtgtgttc atggcagaaa 1445760
actgtaggga gttgcacgcg ccgatcgcca gcgagaggaa aacccaaatc cacagcggcg 1445820
agtttccgtc aggcagggcg agcagcatga tgaaggcggc aagcagcttg gtgttccaaa 1445880
gcagtaccgt gcggtagccg aaacgtttca tgagcggtgc aatcagcggt ttgaccagca 1445940
gcgaagacag ggcgacgggt gcgaccagcc aacccgacag gcttgcgccg aagccgaaag 1446000
cgatttgaaa catcaggggc atcagaaaag gaatcgagct gatgccgaga cggctgaaca 1446060
gattgcccgc cagtcccaga cggaaagtgc gtatcagaaa caggtcggcg gaataaatcg 1446120
gtttggacgc ggttttcata tgtcggaaat aacggcgtgc aaacagcagt ccgccgcaca 1446180
gcggcaacag tgcaaaatac ggaggcagcg cgtgcgacag gctttctgcc gaaagtaaca 1446240

agaggcacgc ggcggcagaa aaaatcagat aacctttgaa gtctaaagag atattactgc 1446300
ctttaatatc gggcatgatg ttgcgtccca atatgaaacc cagcagaccg atgggcaggt 1446360
tgagcaggaa aatccagtgc cacgaagcgt attcgaccaa ataaccgccc gccaaaggcc 1446420
ctaaaaccgg cccgattaat gcgggcataa ccgcataatt gatggcattg agcagcttgg 1446480
acttgtcgta cacacgcaag atggtcagac gcggtatcgg aaccagcatc gaaccgccga 1446540
tgccctgaac gacacgggaa agcgtcaatt caaacagcga acccgatgcg gcgcacaatg 1446600
ccgatccgag cataaaaacg gcaatcgaac cgaaaaagac ttttttcgtt ccgaacctgt 1446660
ccgccaaata accgctcaaa ggaatcagca gggcaaccgt cagcgtgtag gaaataactg 1446720
ccagttgcat atccagaggc gactcattca ggtcggcggc aatttcaggc agtgcggtat 1446780
ttaaaatggt cgcatccaac atctgcataa aaatggcaat tgccagcaga agcggcagcc 1446840
aaggggatgg tgcgcgggcg gatagggtgt ttttttccat agggcgattg taccccatcc 1446900
ttgtgccgtt attgttttca gatgctgtct gaatgccgtc agagtcggca tcttgaatgt 1446960
tcacaagcaa acgaaccggc attgcattgt aatgataatt attatcgaaa accatcagat 1447020
taaggtacag taagcgttat gggggcagtt tgtaagaaaa accggattat tttttaaaat 1447080
tagacttgac ccgcaacagt caattactta aagtaaacgc ttacctttct acagagaaaa 1447140
acgggtttcc cgttatcaaa aaacatgagc gcaaccattc ccccaaaaat catccgatac 1447200
gacagcaatc cgaccgatgt ctattttttc ggcacttgcg tccttgatct ttttatgccc 1447260
gaagcaggca tggatgccat taccctaatc gagcagcagg gcatacgcgt ccatttcccg 1447320
atggcgcaaa gctgctgcgg ccagcctgcc tattcatccg gccatccgac cgaagccttc 1447380
gatgtcgcca aagcacaact cgaccttttc cctgaaaact ggccgatcgt cgtgccgtcc 1447440
ggctcgtgcg gcggcatgat gaaacaccac tggccgacgc tgtttaaagg cagcgagtac 1447500
gaggaaaggg ctgtggattg cgccggccgc atcatcgagt ttacccattt cctgcttgcc 1447560
atcggtttca aacccgaaga caagggcgaa ccgctcaaag tcgccgttca cacttcctgc 1447620
gccgcccgcc gcgaaatgaa tgtccatctt tcaggctggc aactgattga cggtatggaa 1447680
aacgtcgaac gcatcgtcca cgaccacgaa agcgaatgtt gcggcttcgg cggcacattc 1447740
tccgtcaaac aagccgatat ttccggcgca atggtaacag acaaagtcgc cgcgctgaaa 1447800
gaaaccggcg caaccgaaat catcagcgcg gactgcggct gtatgatgaa catcggcggc 1447860
aaaatcgcca aggacgagcc ggatatgccg cgtccgaaac atatcgcatc cttcttgttg 1447920
gaacgcaccg gaggcaaagc atgagcgcgc gtgaaaatat tttggcaaaa ctgaaaaaag 1447980
ccgacgcatt gccgatggaa gaacctgcgg tttttgatta ttaccgtgaa atgggtgttt 1448040
cttggggcag cgaagttgag cgtctgaaac attgggctgc cgctatgcgt gcggtcaaaa 1448100
ccgaaattta ttgggtgacg aaaagcaatt ggatgcaggt tttccgcgaa gcggcagaag 1448160
gcaagggttt gaaaaacatc ctgctgccct tggcgaccga acacggacaa attgcccgtg 1448220
ccgcattggc ggacagcaat atcgaaccga ttgccttcga gcgcgaaatc gatacttgga 1448280
aaaccgagtt tttcacgaac atcgatgcgg gcttcagcgg cgcgcaatgc ggcatcgccc 1448340
gcaccggcac gctgatgctg ttttccagcc ccgaagaacc gcgtacttta agcctcgttc 1448400
cgcccgtgca tttctgcctg ttcgatacgt ccaagatgta caacgagttt cataatgccg 1448460
tcgaaggcga aaaactggtg gaaaacggta tgccgaccaa tgtattcctg atttccggcc 1448520
cgtccaaaac cgcagacatc caactgacgc ttgcttacgg cgcgcacggc ccgcgcgatt 1448580
tggtcatcct cgccatcctg cccgaccaca tttcccctgc cgatttggag gaaaacgcat 1448640
gactacgcaa accatcaaat ttcacatgaa gccggaaact ttcaagcaaa acgccgcaat 1448700
ttcccttcaa gacaagcctt tgcgcaaaag cctgcgtacc gcgatggata tgctgatgac 1448760
caaacgcaaa gccgttttga ccgacgaaga agagctgcaa agcctgcgcg atttgtgcga 1448820
acacgtccgt cagcgctcat tgtctaaatt gccagccctg ctggagcagc tggaagaaaa 1448880
cctgactaag ttgggcgtga aagtgcactg ggcagaaacc ccgaccgaag cctgccaaat 1448940
tatccacgac atcatcacag ccaaaaacgg caagctgatg gtcaaaggca aatcgatggt 1449000
cagcgaggaa atcgagctga accattatct tgaagcaaaa ggcattaaag cggtagaaag 1449060
cgacttgggc gagttcatcg tccaaatggc aggcgaaaaa ccgacccata tcgtgatgcc 1449120
tgctatccac aaaaccaaag aacaggttag cgaactgttc caccaaaacc tcggtacgcc 1449180
gctgacagac gatgtagacc aactgaccgg cttcgcccgt aaagcactgc gcgatattta 1449240
cagcactgcc gatgtcggtt tgagtggcgt aaactttgcc gttgctgaaa caggtacgct 1449300
gtgtctggtg gaaaacgaag gcaacggtcg cttgagtacc accgtaccgc ccgtgcatat 1449360
cgctattacc ggcattgaaa aagtggtggc gaaattgtcc gacatcccac ccttgtacag 1449420
cctgctgccg cgttctgcca ttggtcagaa cattaccact tatttcaata tgattaccgg 1449480
cccgcgccgc agtgaagaat tagacggtcc gcaagaaatg cacttggttc tgctcgacaa 1449540
cggccgcagc caggcttatg ccgaagacca aatgcgccgc accctgcaat gtatccgttg 1449600
cggcgcgtgt atgaaccatt gcccggttta tacccgcatc ggcggcgcgg catacggcac 1449660
aacctatccc ggtccgattg gcgagattat ttccccgcac ctgttaggct tggatgccac 1449720
tcgcgacctg ccgaccgcct gcacgatgtg cggcgcgtgc gtggaagttt gtccggtacg 1449780
catcccgatt accgaacaaa tgcagcgttt gcgcgttgaa gcgcaacgtt cgccgaccga 1449840
aaccgtgccg caccccatcc gggggcaagg cgcatcgcat accttcggcg aacaaatggc 1449900

gtggcgcaca ttcaacggta ttttcagcgg cagcaaaacc taccgcgcct tcggttgggc 1449960
agccaccaag ttccgcaacc tgaccccgcg caaacagttg ggttggacgc aaaaccgcgt 1450020
gccgatgaaa ccggcgaaga aaaccctgca cgaactaatg gcagaaaaaa tgcgccaaaa 1450080
agaacaggca taaaaagttg ttcgcaaaaa tgccgtctga aacccgaaac agggcttcag 1450140
acggcatttg tatagtggat taacaaaaat caggacaagg cgacgaagcc gcagacagta 1450200
caaatagtac ggcaaggcga ggtaacgccg tactggttta aatttaatcc actatatatt 1450260
cgcagacggt gggttttaaa tttgttccaa ttccatattc aaaacagcct gttcctgttt 1450320
ggctcggaag tctgccagtt tttgcgccag ttcgggggtt tcgttggcga gcatggaaac 1450380
ggcgaacaat gcggcatttg ccgcgcctgc ctcgccgatg gcgaatgtgg cgacgggtac 1450440
gcctttgggc atttgtacaa tcgataaaag cgaatcttcg ccgcgcaggt atttgctggg 1450500
gacgggtacg cccaaaacgg ggacggtggt cttggcggca accataccgg gtaaatgcgc 1450560
cgcgccgccc gcacccgcga tgatggcttt gatgccgcgc gcccgtgcgg tttcggcgta 1450620
ttggaacatc aaatccgggg tgcggtgtgc ggaaacaacg cgcgcctcat attctacgcc 1450680
gaactcttca agaaactgcg ctgcctgccg cataacgggc caatcgctgt tgctgcccat 1450740
gatgatgccg atttgtatca taaatcctcc ttggtgcgga tggggtaaaa agcggaaaaa 1450800
tggaaaaact atcgtttgcg cacggctgcg gcggcgcgtt ttgccgccgg gctgccggga 1450860
taggtctgta tcaggctgcg ccaagtcgcc cttgcaatgt ctttttgctg aagcctgtat 1450920
tggcattcgc cgattttgaa catggcttca ggcgcggttg ggctgtcttt gaaacggttg 1450980
gcgtaacgcc ctccgatttc gatgacggat tcgcagttgc ccatacgcgc cctgctttgc 1451040
agcaacaggt acatactgcg ttgcgcgatg ctgccgccgt cgcctccgtc cgcgcctttc 1451100
aacagggagg cagcggcaga aaacttgccg cttttatagt gtttgagtgc ctgattgtag 1451160
aggttttgtg cggtttcgac agtatgtgcg gatgcgctgc cgccttcggt attgaggtaa 1451220
tgctctttca acttgcggtc gtcgagtttt tggacgtatg ccctgccgga agaatgtgtt 1451280
tttgcgtgtt ccagtgcttt gactttgccg tttaaggttt ccacttcgtt cgacagccgg 1451340
acgattttgc cttccagata gtccaaacgg tcttgcaagg tcggaacggg atagggaatg 1451400
ccgtctgaag cattttcccg tgtcgacatt tcggtttggc tgcctgccgg aacgggtgaa 1451460
acggaagcac aggaggcgga cacagacagc caaatgataa aaagcggtaa tttgatcttc 1451520
attatttttt cagaagcagg gtcaagccgt cgccgacggg cagggtgatg gggacgatgc 1451580
gcgggtcgtt cggcaggttt tgattgaaat ctttgaggat gccgacgctg ggcggcgcat 1451640
cggaagccgc ttcgcgcatc acccttccgt tcagcaaaat attgtcgatg gcgatgatgc 1451700
cgccttgacg gacgagtttg aggcaacgct cgaaatattg cggcgtgggc ggtttgtctg 1451760
cgtctatcag tgccaaatcg tagcttccgg cttcaccctg tgcaatcaaa tcatccaatg 1451820
tcagcaatgc gggttgcagg tgcaggctga ttttatgtgc cacaccggcc tcgttccaaa 1451880
cctgacgcgc cgtatcggta aaggttacat tgatgtcgca ggcggtaatc cgcccgtgtt 1451940
cgggcagtgc caatgcaagc gcggtgctgc tgtatccggt aaatacgccg atttccagat 1452000
atttttccgc acggatcagc tttgccagcc aaaccaaaac tgccgcctgt tcgcgcgcaa 1452060
tcgccatttt gcccatacgg tgatgcccgg tcttctcgcg cagccgcgtc aaaacgggat 1452120
gttcgggttc gccgatggcg ttcaaatagt tttgcaggtc cggtgcgaca ttggacagat 1452180
gggtcgtcat ttcggcggat tcagtcttgg taataggtat aaggtttttt cgccactttt 1452240
gccgcctcga agttttcctg ttcttcggga ttgagttcga catcccacaa aagccccctg 1452300
ttttccaaac gctgctgttc caactcaggt ttttcttcaa tcaggcggtt gaggaattgt 1452360
gtggcatcgg attggtagtg atacatcttt gtgctccaat tttacggaat atggcgtgat 1452420
tatactggta ttttccaaac gggataaacg gcttttatca agaatacggg cagaaagata 1452480
aggggtttta ttatagaata agacgttttt tgcaacggaa gcccgcctta tgtcccgaat 1452540
cgccgccctg cccgaccatc ttgtcaacca aatcgccgcc ggcgaagtgg tcgaacgccc 1452600
tgccaacgcc ttgaaagaaa tcgttgaaaa cagtatcgat gcaggcgcaa cggcgattga 1452660
agtcgagctg gcgggcggcg gcatccgcct gattcgcgtc agcgacaacg gcggcggcat 1452720
ccaccccgac gacatcgaac ttgcgctcca ccgccacgcc accagcaaaa tcaaaacctt 1452780
aaacgatttg gaacacgtcg ccagtatggg ctttcgcggc gaaggtttgg caagcatcgc 1452840
ctccgtcagc cgcctgaccc tgaccagccg tcagaacgac agttcgcacg cgacccaagt 1452900
caaagccgaa gacggcaaac tcagcagccc caccgccgcc gcccaccccg tcggcaccac 1452960
catcgaagcc gccgaactct tcttcaacac ccccgcacgg cgcaagttcc tcaaatccga 1453020
aaacaccgaa tacgcccact gcgccaccat gctcgaacgc ctcgcgctgg cgcatccgca 1453080
cattgccttc tcgctcaaac gcgacggcaa acaagtgttc aaactccctg cacaaagcct 1453140
gcatgaacgg attgccgcca ttgtcggcga agactttcag acggcatcat tgggaatcga 1453200
cagcggcaac ggcgcgctgc ggctctatgg tgcgattgcc aaaccgactt tcgccaaagg 1453260
taaaaccgac aaacaatact gcttcgtcaa ccatcgcttc gtgcgcgaca aagtgatgct 1453320
ccacgccgtc aagcaggcat accgcgacgt attgcacaac gcactcactc ccgccttcgt 1453380
cctctttctc gacctgccgc ccgaagccgt ggatgtcaac gtccacccga ccaaaaccga 1453440
aatccgcttc cgcgacagtc agcaggtgca ccaacttgtg ttccacacgc tcaacaaagc 1453500
ccttgccgac acacgcgcca acctgaccga aagcgtcggc aacgcaggcg aagtgttgca 1453560

tgacattacc ggcgttgtct ccacccccaat gccgtctgaa aacgacagcg aaaatctgtt 1453620
tgatagcgta tccaactacc cgacaggcaa caaatcagat acacacaatg cctttggttc 1453680
atcaggcaaa accgcgccca tgccctatca gtccgcatat gcgccgcaac aacgcagcct 1453740
gtccctgcgc gaaagccgcg cggcaatgaa tacttacgcc gaactttaca aaaaaaccga 1453800
cgacatcgac cttgagttaa gccgattcga gcaggcacgt ttcggcaata tgccgtctga 1453860
aacgcctgct ccccaaacag atacgccgct ttcagacggc atcccgtccc aatccgaact 1453920
gccgccgctc ggttttgcca ttgcccaatt acttggcatc tacattcttg cccaagccga 1453980
agacagcctg ttgctcatcg atatgcacgc cgccgccgaa cgcgtcaact acgaaaaaat 1454040
gaaacgccaa cgtcaggaaa acggcaacct gcaaagccaa cgcctgctta ttcccgtaac 1454100
ctttgccgcg tcccacgaag aatgcgccgc ccttgccgat tatgccgaaa cgctggcagg 1454160
cttcgggctg gaattatccg atatgggcgg caacaccctc gccgtccgtg cagttcccgc 1454220
catgctcggc aaagccgatg tcgtctcgct cgccaaagac gtattaaacg aactcgccca 1454280
agtcggcagc agccaaacca tcgaggaaca cgaaaaccgc atcctcgcca ccatgtcctg 1454340
ccacggctcg atccgcgccg gccgccggct caccctgccc gaaatgaacg cccttctgcg 1454400
cgatatggaa aatacgccgc gcagcaacca gtgcaaccac ggcaggccga cttgggtcaa 1454460
actgactttg aaagaattgg acgcactgtt cttgcgcgga cagtaagccg aaagtgctag 1454520
aatacgccgc ccgagaccgc cgttcagacg gcattccgac gcaccgacag aaacatcacg 1454580
accgaaacca agagaaaaac atggcctatc aagttctcgc ccgaaaatgg cggcccaaaa 1454640
cctttgccga cttagtcggt caggaacacg tcgtcaaagc cctgcaaaac gccctggacg 1454700
aaggcaggct gcaccacgcc tacctgctga ccggcacgcg cggcgtaggt aaaaccacca 1454760
tcgcccgcat ccttgccaaa agcctcaact gcgaaaacgc gcaacacggc gaaccttgcg 1454820
gcgtatgtga aagctgtacg cagatcgatg ccggacgcta cgtcgacctg ctggaaatcg 1454880
acgccgcctc caacacaggc atcgacaaca tccgcgaagt cttggaaaac gcccaatatg 1454940
caccgaccgc cggaaaatac aaagtctata tcatcgacga agtgcatatg ctttccaaaa 1455000
gcgcgttcaa cgctatgctc aaaacgctgg aagagccgcc cgaacacgtc aaattcatcc 1455060
tcgccaccac cgatccgcac aaagttcccg ttaccgtctt gagccgctgc ctgcaattcg 1455120
tcttacgcaa tatgaccgcg caacaggttg ccgaccacct cgcccacgtc ctcgacagcg 1455180
aaaaaatcgc ctacgaaccc gccgccctgc aacttttggg acgtgccgcc gccggatcga 1455240
tgcgcgatgc cttgagcctg ctcgaccaag ccatcgccct aggttcgggc aaagttgccg 1455300
aaaacgatgt ccgccaaatg atcggcgcgg ttgacaaaca atacctttac gaactgctga 1455360
caggcatcat caaccaagac ggcgcagccc tgaccgccaa agcgcaggaa atggcggcgt 1455420
gtgccgtcgg ctttgacaac gccttgggcg aacttgccat actgctgcaa cacctcgccc 1455480
tgatacaggc agtgccgaat gccttggcgc acgacgaccc cgattccgat attttgcacc 1455540
gcctcgccca aaccataagc ggcgaacaaa tccagcttta ctaccaaatc gccgtccacg 1455600
gcaaacgcga cctcagcctc gcccccgacg aatacgccgg ctttatgatg accctgctgc 1455660
gtatgctggc gtttgcgccc ttggcggcag catcgtgtga tgcaaatgcc gtgattgaaa 1455720
ataccgaact aaaatcccca tcggcacaaa ccgccgaaaa ggaaaccgcc gcaaaaaagc 1455780
cccaaccgcg ccctgaagcg gaaaccgccc aaacacccgt tcagacggca tccgcagcag 1455840
caatgccgtc tgaaggcaaa actgccgaac ccgttaccaa tcaagaaaac aacgatattc 1455900
cgccttggga agacgcgccg gacgaaaccg cagccggcac ggcgcaagca tcggcaaaaa 1455960
gcattcagac ggcatccgaa gccggaacgc cgcccaaaaa ccaagtttcc aagaacgaag 1456020
cagccgacaa cgaaaccgat gccccccttgt ccgaagtgcc gtctgaaaac cccattcagg 1456080
caacaccgaa taatgaagcc cttgaaacag aagcatttgc acacgaagct cctgcaaaac 1456140
ctttcaacgg ttacagcttt ccgaatgatg actacctcgt agaagacggc gcagaaatcc 1456200
caccgcccga ttgggaacac gccgcccctg ccgatgcgga agaagaaaac aacgccgacg 1456260
aaagcagcaa caacgaagac cacacgccat acgccccgcc gcccgaattt tccaccgaaa 1456320
actgggcagc catcgtccgg cacttcgccc gcaaactcgg cgcggcgcaa atgccggcgc 1456380
aacactccgc gtggacggaa taccatcccg acaccggtct gatggttttg gcaatgaccg 1456440
ccgaagcacg cgccaccgcc gacaaaaaac gcctcgacaa aatccgcgac acccttgccc 1456500
aagcctacgg gctgcaactc accctgcaaa cccaagactg gcgtgacgaa gccggccggg 1456560
aaacccccgc gatgcaggac aagcgcgtcc aagccgaaga caggcaaaaa gcacaagcat 1456620
tgctcgaagc cgaccccgcc gcacaaaaaa tcctccaagc attcggcgcg caatggcagc 1456680
ccgaatcact ggaattggcg gcaaaccggc cataaacaga tataatgccg cccgaaccct 1456740
tcggacggca ttgccgtttc ccttattcaa tcaaaacaga caggagtatt cagtatgttc 1456800
ggaaaagccg gattaggcgg cctgatgaaa caggcgcagc aaatgcagga aaatatgaaa 1456860
aaagcgcaag ccaaactcgc cgaaaccgaa atcgaaggcg aagcaggcaa cggcctggtc 1456920
aaaatcacaa tgacctgcgc gcacgaagta cgcaaaatcg acatcagccc cgatttgatt 1456980
caagaagccg ccgacgacaa agaaatgctt gaagacctca tcctcgccgc cctcaaatcc 1457040
gcccgaggca aagccgaaga aaccgcaaac aaaacaatgg gcgcattcac gcaaggtcta 1457100
ccccccggag tgggcgactt cttccgctga tccccgaccg tcattcccac gcaggcggga 1457160
atctagaacg tagaatctaa gaaaccgttt tactcgataa atttccgtgc cgaggggtct 1457220

ggattcccgc cttcgcggga atgacggcat cagtttgcag gattcggcgt gaacggtaaa 1457280
aacagtgaga atgataagaa cgcaaaaacg gcaagaatag cgggaatcgg caggctgaag 1457340
cccaccctac cattatttac acatccgtac cgcttaaatg ccgtctgaaa cttcgtcatt 1457400
cccgtgaaag cgggaatcca accccgtcgg agcagaaact tacaccccgt cattcccgcg 1457460
aacgcgggaa tccagtaacc gaaaaaccac aggaatctat cggaaaaaca gaaaccctcg 1457520
accgtcattc ccgcgaacgc gggaatccag taaccgaaaa accacaggaa tctatcggaa 1457580
aaaacagaac cccccgaccg tcattcccgc gaacgcggga atctagaacg tagaatctga 1457640
gaaaccgttt tactcgataa atttccgtgc cgacgggtct ggattcccgc cttcgcggga 1457700
atgacggcat caatttgcag gattcggcgt gaacggtaaa aacagtgaga atgataagaa 1457760
cgcaaaaacg gcaagaatag cgggaatcgg caggctgaag cccaccctac cattatttac 1457820
acatccgtac cgcttaaatg ccgtctgaaa tttcgtcatt cccatgaaaa cgggaatcca 1457880
gccccgtggg agcagaaact tacaccccgt cattcccgcg aacgcgggaa tccagtaacc 1457940
gaaaaaccac aggaatctat cggaaaaaac agaacccccc gccgccgtca ttcccgcgaa 1458000
cgcgggaatc tagtaaccga aaaaccacgg gaatctatcg gaaaaaacgg aaacccccga 1458060
ccgtcattcc cgcgaacgcg ggaatctaga acgtagaatc tgagaaaccg ttttactcga 1458120
taaatttccg tgccgacagg tctggattcc cgccttcgcg ggaatgacgg catcagtttg 1458180
caggattcgg cggaaacggt aaaaacggca gaatcgatgg gatgcggcag gctgaagccc 1458240
accaaaacac aaaaattccg atgccgtctg aaatttcgtc attcccgtga aaacgggaat 1458300
ccagccccgt gggagcagaa acttacaccc cgtcattccc gcaaaagcgg gaatccagta 1458360
accgaaaaac cacgggaatc tatcggaaaa aacagaaccc cccgccgccg tcattcccgc 1458420
gaacgcggga atctagaacg tagaatctga gaaaccgttt tactcgataa atttccatgc 1458480
cgaggggtct ggattcccgc gttcgcggga atgacggcat attttttgca tttgatataa 1458540
agggtcgttt gaattttgtt cagcaagtgc aaagtgttgc acataaaagg gcgcaggata 1458600
gaggcaaagc gggcgtaggt cgggctgtag caactgtatt tttcaccccg tcgggcaaaa 1458660
atatagtgga ttaacaaaaa ccagtacggc gttgcctcgc cttagctcaa agagaacgat 1458720
tctctaaggt actcaagcac caagtgaatc ggttccgtac tatttgtact gtctgcggct 1458780
tcgtcgcctt gtcctgattt ttgttaatcc actataccaa aactcaaatc aagccgttcg 1458840
gaggcggctc aaaaaaacgg tacttcgcag cagaagtacc gtttatcggg atttcaggtt 1458900
ttattcttcg gggcgttcgc cgtcggtttc gtcctgcgtc ccttcggtga tgtgcatttc 1458960
tacgccgttg agggcgcgga tttttgcgtc gatttcattg gcgacttcgg gattttcctt 1459020
cagccagacg cggacgttgt ctttgccctg accgattttc gcgccgttgt agctgtacca 1459080
cgcgccggat ttgttgatga tgtcgttttt cacgccgatg tcgatcaatt cgccttccca 1459140
actgatgcct tctccgtaga ggatgtcaaa ctctgcctga cggaacgggg gggcgacttt 1459200
gtttttgatg actttgacgc gggtttcgtt gcccaatacc tcttcgcctt ttttgatgga 1459260
tccggtgcgg cggatgtcga ggcggacgga agaatagaat ttcagcgcgt tgccgccggt 1459320
ggtggtttcg gggctgccga acattacgcc gatcttcatc cggatttggt tgatgaacac 1459380
aaccagcgtg ttggtttttt tgatgtgtcc ggtcagtttg cgcaaagcct ggctcatcag 1459440
gcgcgcctgc agtccgacat ggctgtcccc catatcgcct tcgatttcgg ctttggggac 1459500
gagtgcggct acggaatcga cgactaccat atctatgccg cccgaacgga cgagtgtgtc 1459560
gcagatttcc aaagcctgtt cgccggtatc gggctgggac aggtaaagct cttcgacttt 1459620
tacgccgagt ttgcgggcgt aaacgggatc aaaggcgtgt tcggcatcga caaaggcgca 1459680
cacgccgccg tttttctggc attgggcgac ggcttcgagg cagagggtgg ttttgccgga 1459740
ggattcgggg ccgaagattt cgacgatgcg cccgcgcggc agaccgccga ctccgagggc 1459800
gaggtctaat ccgagcgatc cggtggaaat gacttcgagg ttttcttcct gctggctgcc 1459860
gtccattttc atgatggcgc ctttgccgaa actttttttcg atttgcgcca gtgcggcggc 1459920
aagtgctttg cttttgtcgt ctgacattgg ggttactccg gaacaaatgc ggtatgtggg 1459980
atgcggcgca acacgggctg cggcgcggga tgtgtatcgt tttcccgatg tgcgggctat 1460040
cggtaatgct gcttcacgag gttgccatta tcgcatattt ccttgcttgc cgatatgcgc 1460100
caggacgcgg cggcttgtgc cggaatggaa tctggatgcc gtctaaaagg cggccggctt 1460160
tgttataatg gcggctgttt tttctgtgtg tgcctgtttt atgtgttcct gccttgttgt 1460220
caaaaatacc gttatcggaa gcggacgcac caaaatcgcc gtgccgcttg tcgcccgcga 1460280
tgccgccgaa ctttccgccg tacttgagca aatcaaaaat atgcccttcg atattgcgga 1460340
gttccgcgcc gactttttgg aatgcgcggg cagtatcggc gaaatattgc accacacgca 1460400
gaccgtccgc gacgcgctgc ccgacaagcc gctgctgttt acgttcagac ggcatggcga 1460460
aggcggctcg ttcccgtgtt cggacgatta ttattttgaa ctgctcgacg cgctgatcga 1460520
aagccgcctg cccgacatca tcgacatcga gctgttttcc ggcgaaaccg ccgtccggtg 1460580
cgccgtggca aatgctcaaa aaaacggcat cgccgccctg ctctgcaatc atgagtttca 1460640
ccgcacgccg ccgcaagaag aaatcgtatg ccgtctgaaa cagatggagg actgcggcgc 1460700
ggacatctgc aaaattgcgg tgatgccgca aagcgcggaa gatgtgctga ctttgctttc 1460760
cgccacgctc aaagcgaaag agcttgccgc caaaccgatt gttacgatgt cgatggggca 1460820
gacgggggcg gtcagccggc ttgccggaca ggtgttcggc tcaagcatca cgttcggttc 1460880

gggaacgcaa aactccgcgc cggggcaaat cggcgtatcc gccctccgtg cgacactcga 1460940
ctgcctcgaa aacggcgcag actgatttca gacagcatca aaacatgatg aaactcaatc 1461000
cccaacagct cgaagccgtc cgctacctcg gcggcccact gctcgtcctt gccggtgcag 1461060
gcagcggcaa aaccggcgtg attactcaaa aaattaagca tttgattgtc aatgtcggct 1461120
acctgccgca taccgttgcc gcaattacct ttaccaacaa agccgctgcg gaaatgcagg 1461180
agcgcgttgc caaaatgctg cccaaaccgc aaacgcgcgg gctgacgatt tgcacgttcc 1461240
actctttggg catgaagatt ctgcgcgaag aggcgaacca tattggttac aaaaaaaact 1461300
tctccattct cgattctacc gacagcgcga aaatcatcgg cgaactctta ggcggtacgg 1461360
gcaaagaagc cgtattcaag gcgcagcacc agatttcctt gtggaaaaac gatttaaaaa 1461420
cgcctgaaga tgtcgttcag acggcatcga acatttggga acaacaaacc gcacgcgtgt 1461480
atgcgagcta tcaggaaacc ttacaaagct atcaggcagt ggacttcgac gacttaatcc 1461540
gcctgcctgc cgtgctgttg cagcaaaaca gcgaagtgcg caacaaatgg cagcggcggc 1461600
tgcgttatct gttggttgac gaatgccaag atacgaatac ctgccaattt acgttgatga 1461660
agctgctgac cggcgcggaa ggtatgttta ccgccgtcgg cgacgacgac cagtccatct 1461720
acgcatggcg cggtgcgaac atggaaaacc tgcgtaaaat gcaggaaaac tatccgcaga 1461780
tgaaggtcat caaactggag caaaactacc gctccaccgc gcggattctc aaaatcgcca 1461840
acaaagtcat cgaaaacaac cccaagctgt ttaccaaaaa actttggtcg caattgggcg 1461900
aaggcgagcc ggtcaaagtc gttgcctgcc aaaacgagca acacgaagcc gactgggtcg 1461960
tcagccaaat cgtcaaacaa aaactcatcg gcggcgacaa aacccaatat gccgatttcg 1462020
ccgtgttata ccggggaaag catcaggcga ggattttcga ggaagcattg cgcggcgcgc 1462080
gcatccccta ccagctctcc ggcggacaaa gctttttcga caaagccgaa atcaaagacg 1462140
tgttgtctta tgtgcggctg cttgccaacc ccaacgacga tcccgccttt ctgcgtgccg 1462200
ttaccacgcc caaacgcggc atcggcgatg tcacgctggg caagctcaac acttacgcgc 1462260
acgaacacga atgcagcctg tatgaagccg cgcaaaacga agaagccctt gccacgctga 1462320
acaataccaa ccgccaacac ctgcaaacct ttatggatat gttcgtcagc tacctcgcca 1462380
aagccgaaac cagcgaagcg ggcgagttca tcaacagcct gctcgaagaa atcgactatg 1462440
aaaaccattt gatgcaaaac gaagaaggca aagccggcga aatcaaatgg cgcaacgtcg 1462500
gcgatttggt atcatggttt gcgcgaaaag gcggggaaga cggcaaaaac atcatcgaac 1462560
tcgcccaaac cgtcgccttg atgacgcttt tggaaggaaa agacgaagaa gaaaccgatg 1462620
ccgtctcgct atccacgcta cacgccgcca aaggtttgga gtatccgtat gttttccttg 1462680
tcggttgcga agaaggcgtt ttgccgcaca acgacagtat cgaagagggc aacgtcgaag 1462740
aagaacgccg cctgatgtac gtcggcatca cccgcgccaa acgccaactc acactgaccc 1462800
actgcgtcaa acgcaaaaaa caaggcacat ggcagttccc cgaacccagc cgattcatag 1462860
acgaaatgcc gcaggaagat ttgaaaatcc tggggcgcaa aggcggcgaa ccgattgtca 1462920
gcaaagaaga aggcagacgc aaccttgccg atataatcgg aaggctcgac aacctaaaaa 1462980
aaagcggcgc ggcggattaa accggagccg caatgccgtc tgaaggcttc agacggcata 1463040
ttttttggac ggcgcgcgta aagcggttta cgcccacaaa tcctgctgct ggtttttcgg 1463100
cacaagatgc cccacgccga taccgataag gcggaacgcg tcttccgtct gcggcgagac 1463160
gcgcgccatc aacatttgcg cagcctgcag cagagtgcgc agtcgggcaa tacggaggaa 1463220
taagtcagtg tgcgcgtgat gatgcggaaa tcgtaggtct tcagcttgag cgttacgctt 1463280
tgggcttcga cgtttttgcg cgtgatttgc cgccacaagt cttcggcaag atgggggagg 1463340
tgtccggcag cctgctcgag cggcaggtct tcgggcaggg taatttctgt ggagatttgg 1463400
aggcgttcgc gttcggcttt gacggggcgt tcgtccgtac cgcgcaccaa atcatagagg 1463460
cggtatccgt agcgtccgaa atggtttaag agttcgccgc gctcgaaacg gcgcaagtcg 1463520
cccgccgtcc gcatacccag cgactgcatt tttttcagcg ttaccttgcc cacgccgggg 1463580
attttgccca aaggcagggt ttccaaaaat gccatgactt tgtgcggcgg caacacaaac 1463640
tgcccgttcg gcttgcgcca gtccgacgcg attttcgcca gaaatttgtt cggcgcgatg 1463700
cctgcggatg cagtcaaacc tgtttccgca aaaatggcgg cacggatttc tttggcaacg 1463760
tcgccggcgt aagggatgtt tttgaaatta cgggtaacgt caagataggc ttcgtccagc 1463820
gacaagggtt cgattaaatc ggtataacgc ctgaatacgg cgtgaatctg cgcggaaacc 1463880
tgacggtaca aatcgaaatg cggcggcaca tacaccgctt gcggacacag cctttttcgcc 1463940
gttgccaccg acatcgcgga atgcagcccg aactgccgtg cctcatacga tgcggcgcaa 1464000
atcaccgaac gcgcgccctc ccacgcgacg accaccggcc gccctttcaa atgcggctgt 1464060
tcgcgcagct ctaccgatgc gtagaatgcg tccatgtcga tgtggataat tttgcgtgaa 1464120
gacatcggct cttctgagga taaaagggat attctactgc cggcatcggg caaattccaa 1464180
atatacgccc cgatagacct gcctccataa aaatgccgtc tgaaacatac cctgtttcag 1464240
acggcatccg caaaactacg gttttcaatt aaaactgcca atccagtttc atgctgacag 1464300
tgcgcggctc tccgtagaag ttgtttgcgc cgcgcgtacg gttgtagttg ttctcaaaat 1464360
aagtgcgtcc gtttaagttc gtaccgatga ggctcaattt ggcgtgtttg cccaattcgt 1464420
aacggacgaa accgtctatc agcccgtagc cgccctgcct gatgttatac agactgcttg 1464480
tgccgctttg tgcggacacg ccgccgccga cggtcagccc cgtattcggt atatggaagc 1464540

tcgttccgaa acggaatatg tgcacgggtg tgaaattgct gaagttgtac gggtctgcac 1464600
tggaattttt ggcaaggcgt tcggcgttga cttcggcggc gtttttgtag cggctcttgt 1464660
tgtaggtgta acccgcaaag actttccaat cttcgttcaa ctcacccgac aactcgaatt 1464720
ccgcacccct gctgaccact ttgcctatcg gtttggcaac ggtttggaac gacccctgct 1464780
tgccgcctgc tccgggaaca tagccgaaat cgacgaccgt gcggtttttc tgttcgaggt 1464840
aaaacaatgc gaacgaagca ttcagccgtc cttgcaagaa cgcgcctttc cagcctacct 1464900
catagtttgt gccgaccaaa ggcggtaaaa cggttttggc actgacatcg acattatcct 1464960
gctgtttgaa gattttggta taacttccgt aaatactctg ttgcggtgtc aagtcatagg 1465020
taatgcctgc atagggcgtc aatttatgac cttgcatctt ggccgtgtaa tggtcctgat 1465080
ccgccctaat gctcgatgcc gtctgaaaat cgcttgccgg ctgcccatag cggacaggca 1465140
tatctttggt ttgcgaagtc tcatagcgcg tgtagtgcag cccgcccaaa aggtgcagtc 1465200
ggccggttac gttgaaacgc gtgctggcag tcagcgaatg ggttttgttg gtgttgaggt 1465260
atttggcgta gttatacagc gcaggaacat ggtcgtctgc cactttgacg gttttccaaa 1465320
ccggcaccgt accggaaaaa ccggtaaagg caggcgtgcc gtcgggattg gtctcctgaa 1465380
tcttgttgcc tttttcgtcc agctcatata catcgacata taccggtgtc cggctgccgc 1465440
tgtattcgtc atagtaatac acctgcttgc cttcggcatc gagcttgggc tcggttttta 1465500
ttttcttggc gttcctgcat tcttcggcat aaacggtacg gttgcctttt tcatcgtacg 1465560
cctgccaatc gggttcttta tgccccctga ccaaaggaga cgacaaatcg ccgtccggct 1465620
cctcctgaca acttcccgca tacacgccgt gcgttgcccc cgtattcgga cgtactctgt 1465680
agcggcgttc gtagatttct agatattccg aacgtatctt ttcatcaccg taggcatagc 1465740
cgacaaagaa atcatgctcc cgcccgaaca gcccatatgt gccggtcagg tcaagtttaa 1465800
ttccccattg gcggtcgtct ttggtatgcc gcaacggcat ataactgtat cgtcggttgg 1465860
cggtagcctt ccgattaaag gaagagtcat acaggctgtt tggaaaacgt tgtgccgcat 1465920
tattaaagat gccctccttc gcaagggctt tatcgacaaa ttccgccttg tcggcatcaa 1465980
cgcccggatc cccccaagaa ccttgacaga taaagtccag cgcgaaaggg tcactcatac 1466040
acttgtcaaa accggctttg cgttctgcgg cacggcggct gcgatactgt tcgaaagcag 1466100
tattatcgaa acggttttta acaaaatcgt ctttgcgctc ccggtattcc ttggcggttt 1466160
catcacgata tgctttcagt ttctccaatg ccttatcttt cggctcgaac gggatgactt 1466220
cgttttttttc agtcaaaaag cctaccgcat cctcacccga caaacccgcc gcatattcgt 1466280
ttttcagaaa aaactgcccc accttcgcat cggattcatt cttggtataa gacacttcgg 1466340
cattgagctg ccaaccgttg tcaaacacat gtttgaatcc tgagaaaagg ttgtatttgt 1466400
cggcacttaa ccgcgaccaa tcctccccca aataagtgtt gcgcggcagt tgcaaaggcc 1466460
ggttgcaggc aggcgttgaa ctgaacgggg cagttttctg attttcacag ggcaaaataa 1466520
tgcccgaaaa atcaggaacc tccctactct tctgatacat gccgcccaaa gtaagcacac 1466580
tgctgtcgcc cgcatcggct tcggcaatgc cgtaaaccat atgtttcctg ccccaaactc 1466640
ggtctttaaa cgattttttta tactcttccg cacccaccaa ccttccgcgt aaggtattcg 1466700
ccttattcag gctgcctgaa acatccaaca ctgcacgccg gctgccgcga tggtcggcgg 1466760
tcagctctcc ggtatgtttg aaagaagcgg taggtcactt acggatcaaa ttgacggttc 1466820
ctcccggctc tgaattggat tgggtcaacc ccgttgcacc ccgtacaact tcaatatggt 1466880
cataaaccgc caaatcggta ctcggagaca cgtcgatttt cgccgtatat cccgaacggc 1466940
ctgcaacatt gacggtcata ccgtcttcac caatctgatc aatatagaaa ccgcgtgaca 1467000
aaaaccgcgt ctgcaagcct gaatcgcgca caacgttgac acccgtcgtg tttttcattg 1467060
cctcttcaag cgtatgcacc gccttatcgt caaggcggct gcgcgtgatg acgctgaccg 1467120
actgcggcgt atccttgccc gcaatcctca tacctgtggc ggtggacatc cgatctatcg 1467180
tataagaacg ggtcttttcg gtcttgccca acaaagcatg agagccgcgt acattgaccg 1467240
tatccagact gacggtattg ccgtctgaaa caggcacaac accgtctgca aaagaaccac 1467300
cgtaagccga taacagcata acggtcagaa ttttaagtga aaaatgattt tgattcatag 1467360
agacctctgt aatatgcaag tgtgcaaatc gtccaaaggc tctcacaact gttttgattt 1467420
tttatattaa ttgaaaaaaa gtaattctca attaaattta tagataggat tgtattccca 1467480
ttttgacaaa aaacaaacta ctccttaccg tttatttcaa aaaacgataa cattgtattg 1467540
aaaaatatcc gaatttaaat acagaccgcc aatgcagaaa aaaacaccca aattggctat 1467600
aatcccgaca aacacactca aggacaacaa catggcagcc tcgcccgaag caaaattcac 1467660
cgaagaaaag attttgtggg tcaaacacca cacgccgaaa ctcatcactt tcgccatcag 1467720
ccgtcccgaa tcctaccgct ttaaagccgg acagttctcc cgactcggtt tctacgaagg 1467780
ggaaggtttc atttggcgtg cctattccat tgtttccgca gaatatgccg acacgctcga 1467840
atattttgcc gtactcatcc aagacggccc catgtcggcc cgtttcgcca aaatgcaaca 1467900
gggcaacacc atcctgctcg ataaaaatgc caccggcttc ctcctgcccg aacgcttccc 1467960
cgacggcaag gatttggtga tgctctgcac cggctcgggc atcgccccct tcctttccat 1468020
tctcgaacaa cccgaaatcc gtcaacgttt cgataccgtc aacctgatac attccgtatc 1468080
ttttcccgaa gaattgattt tcaacgaccg actcgccgca ttgactgaac atcccctggt 1468140
aggcgaatac ggacactctt tccgtttcgt ccctgttacc acccgtgccg ccaacccctc 1468200

gggcttaagc ggaaaacgca ttccggaact cttaaaaaac aacagcatcg aacaggcgct 1468260
gcataccaag ttcaccccgg aatccacacg gtttatgatt tgcggcaacc cggaaatggt 1468320
caaagacact ttccaaacgc tgctcgacat gggttacgcc atgcaccgca accgcattcc 1468380
cggtcaaatc atgatggaaa acggcttcta aaaaccaccc tgcttgtccg atgccttcgg 1468440
atggacgggc aaaccgacac ggcacgaaaa ccgcgtcggc aaaaatgccg tctgaaaaaa 1468500
ttcagacggc atcttcggat acattacctg caaacggcaa cacaccggca caaaccgatt 1468560
aggcaatcaa cacggtgacg gctgtttaca tacttgccgg ctttcaccaa ccgatatcga 1468620
tttaaccgat ttccttaata tttttcctgt ccgttttaaa cttcgcctta aacgcatccg 1468680
gtaaatcttt atcgaaatac caaagcccgt catccatttc caatgcgccg cccattccgt 1468740
gcagaacgac tttttccccc gtcaaaggat cggtttcggt aatttccacc tcatgcattt 1468800
ttccccaatc caaaacaggc aacttgtgtg caaacgccaa aacctgttcg tcagaacggc 1468860
cgcacgcctt atcgcatcgg cctgaaacat atacgggaca aggctcatcc tctgcataac 1468920
cgtccggaag gataccggct gcggcaggac acaatccgta tgtttccgcc cacgacgaca 1468980
aagcccgtct cgctgccttt ttattggcaa ataatccggt aggcggatta tccgtcacac 1469040
cgtttttcaa agccgctgtt ttcgcattca acatgccgtc tgaacctttt tcaaacctga 1469100
cggtcgtaaa tgttttaagc agatttttgg cagacacata acaatccgaa tgataacgcc 1469160
cgaccaattc cgctttaatc ttatatgcat gcaggctgcc caatgcggga aaaaaacgga 1469220
cttcctccgt attgcaccaa tcaaacgggg cttttccgga gtccaataaa gccgaaatct 1469280
cgctatatac ccgttcaaac gtaccgatat aatttacttt ccctccgccg tcgaaacaag 1469340
ccagcacccc cataccgtca ggcaaaccgt acaactgttc cctcaaccgt tcgggcagcg 1469400
cggcaggcag cggtttcgga ttcatcaaac ggaaacactg cctgatccat gcctcaaccc 1469460
cgtgttccga cagactgtat tccaaataat cacacaatgc cgatacatcc gccatcgcac 1469520
gatgcctgtc ttccacaaca atccccaacc tttcgatgat actgtccagg ctgtgcttgt 1469580
aaaattgcgg atacagacac cgggacagct gcacactgca caaagcaggc gatgaaaatc 1469640
cgatacccgc acgatgaaac tcatgcttta aaaacgtata gtcgaaacgg ctgttatgtg 1469700
caaccagcac acaacccttc aataccgaaa acaactcgcc ggcaatctct gcaaaaacag 1469760
gcgcatcggc aaccatgccg tctgaaatcc ccgtcagccc cgccacaaac tgcggaatcg 1469820
gtttttgagg attaaccaac cactcatgcc tcaccaccct tccctgctca aacttgacca 1469880
aagccacttc ggttaccctg tcttcataca gattgccgcc cgtcgattcc aaatcaacca 1469940
cggcaacagg cattccaaac cgtaaaaata ccttttccag caagggccag cgagaagcaa 1470000
caatcatttt attctcttta aattcaaaca acaaaccaat attttacact tttaaggcat 1470060
ttcatccaac aaaacaattg acagaatccg atgattaccc taaaattcga atctttcttg 1470120
cagcgcaccc gtagctcagt tggatagagt atctggctac gaaccagagg gtcgggcgtt 1470180
cgaatcgctc cgggtgcgcc agtaagaaaa tacaatatgc gcccatcgtc tagcggttag 1470240
gacatcgccc tttcacggcg gtaaccgggg ttcgattccc cgtgggcgtg ccaaattcta 1470300
aatccccgag attatcgctc ggggattttt tattgtctca gcaactcgtt accatatctt 1470360
tacctacccc cttcatcaga atctcagacg taatcgaatc atattcaaac ctttgccgtg 1470420
caaaccgata tcccataacc ggatgcggtg tccgtccaac attttacccg attgaaacgc 1470480
ctgatatatt gcaccccatc aacgtggcat tacttttctt aacaatcccc tttgacagca 1470540
actgactagg gctttttttat gccatcatca aatttatagt ggattaactt taaaccagta 1470600
cggcgttgcc tcgccttgcc gtactatttg tactgtctgc ggcttcgtcg ccttgtcctg 1470660
atttttgtta attcactata atattttctc tcccgattga aacaggcgta acagaatgcc 1470720
cgaagctccg gctgctttct tgtttaccgc cgcgatattt agagtataat accaaatttg 1470780
agcaatagtt ctaaaacagt tagaaccatt tttcatgagc ctgactgatt cgtacactcg 1470840
gagaaactga tgcagaatat ttttgaccct ttggttattc gtggaaaatc ccttaccccc 1470900
atcgtgcaag gcggtatggg ggtcggtgtt tccgcatcgg gtttatccag cgcggtggcg 1470960
cgtgaaaacg gtatcggaac gattgccagt gtggatttgc gccaccttca cgaagaccta 1471020
ctcgccgaat cacaaatcaa tccgagtgaa gagaaatata catctttgaa ctgtaccgca 1471080
ttagacaggg aaatccaaaa agccaaaagc gcttcagagg gaaaaggact gattgcggtc 1471140
aacgtgatga aggcggtcaa agaccacgcc gcatatgtcc gccaggcttg cgaatcaggg 1471200
gcggatgcgg ttgtaatggg tgccggcctg cctttagacc tgccggaaat gaccgagggc 1471260
tatcataaag atgtcgcgct gctgccgatt ctgtccgaat cgcgcggtat taatatcgtc 1471320
ttgaaacgtt ggatgaaaaa aggcatattg cccgatgcga ttgtagtcga acatcctgcc 1471380
cacgcggccg gacatttggg tgcatcaacc gttgaaggcg taaacgatgc caagttcgac 1471440
ttcaaacgcg tgattgagga aacgtttgaa gttttcaaaa gtttagggct ggaaagcgaa 1471500
aaaatcccgc ttattcttgc gggaggcatg gcaaattttg aaaaagtcaa aaccgcccta 1471560
aagaactggg gagcatccgc cgttcaaatc ggtacggctt ttgccgttac cgaagaagga 1471620
gatgcacacc ttaacttcaa aaaaacgctc gccggtgcgg aaactgaaaa agtagtcgaa 1471680
tttatgtctg ttgccggttt gccggcgcgc ggtgtccgca ccaaattcct agacagctac 1471740
atcaagcgtg aaagcaaact tcagacaaac gccaaagccg acccgcgccg ctgtacccaa 1471800
ggtttaaact gcctaaccag ttgcggtctg cgcgacgggc tttccaaagc aggacagttc 1471860

tgtattgata tccagcttgc cgccgcattc cgtggagaag tagataaagg cctgttcttc 1471920
agaggtaaag accgctgccc ttcggcaatg ccatccgcac cgtccgcgag acgatacaat 1471980
atctgctgac ggggagcgaa cctgttgcaa cgctcggacg ctgacatcag gtaggatttg 1472040
atttgcaaac aaaatgccgt ctgaagggct ttcagacggc gtttttcagg ctgcgttccg 1472100
gaatagtgtt aaaaaataaa cgggatgaga tacatttatt ttcgtccgac aaatcaaacc 1472160
atcgcgccga atgatcaaat aatgcctgca cggcattaca tctggcaaag caatgcaatg 1472220
aaaacacggc ttttttattt gctttcagta ttattgaaaa gcttgtccat cggggtcaaa 1472280
tcgaccgcat tgccttggct ggtaatccat ttgttctcga cgcgccacac gcctgccgcg 1472340
tcctccacgc gcacatacca gtggttggtc ggcgaaaggg ttttgaacac cgcctcatat 1472400
tccgccctgc cgttctgcgc gctgccgacg ggcttgaggg cgacggtttg atcgtccgcc 1472460
ttgcgggtcg ggtgcatcag cagcaggttc aaaggctgtt tgccgtcaaa ctcgccgccg 1472520
acaaacactt ttgccgcatt catatcgggg gaaatgagaa cctgcacccc gatatgccgt 1472580
ctgacggctt cttcatcccg atgaagctgg atgtcgatat gtttgccgtc tttataataa 1472640
tcgtccgtaa ccaaatctgt cgcgtgctgc tgcgcgacaa aaaacatagc gacgctggcg 1472700
atgacgacaa aaatcggccc cgccatcaag atccacggcc agacgtgttt gtaccaaggt 1472760
ttgattggag tgttttgaga cacggttatt ctccgataaa ggttgcatct tcttccaaga 1472820
cgaccggctt gccgtcgggc gcgccgcttt cgcggtattg gaaggtaaat tcgatagggt 1472880
ggctgccttt gtccgcgtat tccggaatgg tggacacttg gacgggaagg gttaccgttt 1472940
cgcgcggggc aaccttgata ccgccttcgg gcagcccggt cagggcgatt tcgtcaaagc 1473000
ctttgacact tgcggtaatc agctgttctt tttcactttt gttgatgata cgcaggctgt 1473060
atgcgttttc cagccagcct ttggcgtttt cgcgcaccag tacgccacgg tctttcaaaa 1473120
tatcgacctc gaccattttg cgcgtggaca aaccggccag gaaggcaatg ataactaacg 1473180
ccaacaccgc gccgtaacct gccacgcgcg gtctgagcag ccgtttttta atgtcttttt 1473240
cagaatattc gtgttccagc gcgctttcgg tcgtataacg gattaatccg cgcggatagc 1473300
ccattttgtc cataatctca tcgcacgcgt cgatacaggc ggcgcagccg atacattggt 1473360
attgcagacc gttgcggatg tcgatgccga cggggcagac ttggacgcac atcgcacagt 1473420
tgatgcagtc gcccaaaccc gcctcttcct tattgaccgt tttcttgcgc gcgccgcgcg 1473480
gttcgccgcg ttccgcgtca taagaaacaa tcagcgtgtc cttgtcgaac atcgcgcttt 1473540
ggaaacgtgc atacggacac atatgcaggc atactttttc acgcataatg tgggcgaaga 1473600
agaaggtcat aaagccataa aacgctgcgg caaacatcgc gccgccacct gctgctccag 1473660
tgaataaatc gggaacgaac tggcggatag ggacaaacca gcctgcaaac gtgatgcccg 1473720
tccacgcgca gacaaggaaa atcagcaggt atttggtggc tttgatgcgg atttagtga 1473780
aattccacgg cgatttttcc agtttcagcc gtttgtttct atcgccttcg accaggttgt 1473840
caatccacag cataatttcg gtgtaaaccg tttgcgggca ggaatagccg caccacagtc 1473900
gccctgcaat cgtcgtccac caaaacagcc cgaaggcgca aatcatcagc agcaaggcaa 1473960
ggtaaatcaa atcgcccacc cccaacgaca atccgaaaat gaagaaatgc cgttcgggga 1474020
tattgaaaac gacggcctgc ctgccgctcc agttgaacca cggaatgacg taaaacacaa 1474080
actgcgtcgc caatacggcg gcgatacgca gtttggcgaa ccgtccttcc gcctttttgg 1474140
gatggatgcg ttcgccttcg ggatggattt gaatcacgct ggctcgcgga tcgaatgttt 1474200
tttttgcttt cggcgcggct tttgtttgtt cggacgtgcc gattccggat gccggactgc 1474260
cggcttggtt ttccgtggtc attctgcatt ccttagattt ttgattgatg gtttgcccgt 1474320
taccgccgcc gtttgctttt cagacgtcat ttttcttgtt ttttaaggcg ttgtgtttca 1474380
agttttgaga aaatccgttt ttcccaaaat atatttccgc tattgtacaa ctttatgcgc 1474440
cgtccggatg tatggggcgg atacatttcc catccgcatc aaaacgcctg gattttacct 1474500
taccgcccga acaaaatccg aatacggtta aaaaaaaaga ctaaaaaaac cgacaccccc 1474560
atatcggcag aaccgacggc gcaagctcat aaacaaacgc tatcgacaat ccggcacaca 1474620
atctataact ttttatttca aaaggaataa tggcaggctt cgcccgcaaa tcgaaaatcc 1474680
ttccccgcct gtcccctgcc gccgccttcc cacgcgtccg ccctttttctt gaaagcataa 1474740
gcgaatcggg cgataatcaa cgctttccga ttatccactt atctgaaaca ccagcaagga 1474800
aaatacaaaa tgtctcaact ggcaaacgca atccgcttcc tctcggccga tgccgttcaa 1474860
aaagccaatt ccggccaccc cggcgcgcct atgggtatgg cggaaatggc ggaaacattg 1474920
tggacgaaat tcctcaatca caaccccgcc aaccccaaat tctacaaccg cgaccgcttc 1474980
gtcctctcca acggccacgc gtctatgctg ttgtacagcc tgctgcacct gaccggctac 1475040
aacctaagca ttgaagactt gaaaaacttc cgccaactgc acagcaaaac ccccggccat 1475100
cccgaatacg gctacaccga cggcgtggaa accacgaccg gcccgttggg gcaagggatt 1475160
gccaacgcgg tgggtatggc attggcagaa aaaatccttg ccgccgaatt taataaagac 1475220
ggtttgaaca tcgtcgatca ttacacctac gtctttatgg gcgacggctg tctgatggaa 1475280
ggcgtatcgc acgaagcctg ttcgctcgcc ggcaccttgg gcttgggcaa actgattgtt 1475340
ttatatgatg acaacaatat ttccattgat ggtaaagtgg acggctggtt taccgaaaac 1475400
atcccgcaac gctttgaaag ctacggctgg cacgtcgttc ccaatgtaaa cggtcatgac 1475460
accgccgcca ttcaagccgc catcgaagcc gcacgtgccg aaaccggcaa accgtccatc 1475520

atctgctgca aaaccttaat cggcaaaggc agtgccaaca aagaaggcag ccacaaaacc 1475580
cacggcgcac ctttgggcgc ggacgaaatc gaagccacgc gcaaacattt gggctggact 1475640
taccccgcct ttgaaatccc gcaagaaatt tacgatgcgt ggaatgccaa agaacaaggc 1475700
gcgaaactgg aagccgactg gaacgaactg ttcgcgcaat atcaagccaa atatcctgcc 1475760
gaagccgcag aatttgtgcg ccgtatggat aaaaagctgc cggacaattt cgatgaatac 1475820
gttcaagccg cattgaaaga agtgtgcgcc aaagccgaaa ccatcgccac ccgcaaagcc 1475880
agccaaaaca gcatcgaaat cttggcaaaa gagttgcctg aattggtagg cggttctgcc 1475940
gacctgaccc cgtccaatct gaccgactgg tcaaacagcg tctccgttac ccgcgacaaa 1476000
ggcggcaact acatccacta cggcgtgcgc gagttcggca tgggtgcgat tatgaacggt 1476060
ttggtattgc acggcggcgt aaaacccttc ggcgcgactt tcctgatgtt cagcgaatac 1476120
gagcgcaatg ccctgcgtat ggctgcgttg atgaaaatca accctgtatt tgtgtttacc 1476180
cacgattcca tcggtttggg cgaagacggc ccgacccatc aaccgattga gcaaaccgcc 1476240
accctgcgcc tgattccgaa tatggacgta tggcggccgt gcgacaccgc cgaatccttg 1476300
gtggcttggg cagaagccgt caaagccgcc gatcacccgt cctgcctgat tttcagccgt 1476360
caaaacctga aattccaagc gcgcagcgag caacaactga acgacatcaa acgcggcggc 1476420
tacgtcatca gcgaagccca aggcaacgcc caagccgtca tcattgccac cggctcagaa 1476480
gtcgagctgg ctttggaagc gcaaaaagcc ctcgccgcgc aaaacatcgc cgtgcgcgtc 1476540
gtttccatgc cgtccaccaa cgtattcgac cgccaagacg ccgcctatca agccgccgtc 1476600
ctgcccgaag gcctgccgcg catcgccgta gaagccggac acgccgacgg ctggtacaaa 1476660
tatgtcggac tgaacggcgc agtcgtcggc atcaaccgct tcggcgaatc cgcccctgcc 1476720
gatttactct tcaaagcatt cggctttacc gtggacaatg tggttgatac ggtgaaatcc 1476780
gtgctgtaac cccacaccta aacaaatgcc gtctgaaacc aattagggct tcagacggca 1476840
tttttatatt ctcgcggcca tgatgctttc tcatcccacc aatctccatt ataatatttg 1476900
cgaatcactc ttattcacat ttcaaaagga gaaacgcatg agcacccgta ccgaacacga 1476960
cacgatgggc aatgtcgaag tcccatccga agcctattgg ggcgcgcaga cccagcgcag 1477020
ccgcaacaat ttcaaaatcg gtggcgaaac cctgccgcag ccgttgattt atgctttggc 1477080
attggtgaaa aaagccgccg ctgccaccaa tgtttccctc ggtaggatta agcctgaaca 1477140
ggcggatttg attacgcagg cggcggatga tgtgttgagc ggcaagctcg acgggcagtt 1477200
cccattggta gtgtggcaga ccggttccgg cacgcagtcc aatatgaaca tgaacgaagt 1477260
gctggcaaac cgcgccaacg aaatcgccgg tacgggtttg gcggcttatc agcccgtcca 1477320
tcccaacgac catgtgaacc acgcgcaatc gaccaacgac gcattcccga ccgctatcca 1477380
cgttgccgcc gcgattgaaa tcaaccgcca cctcatcccc gccgtaaaag ccctgcgcga 1477440
cacgttggac aaaaaagccc aagctttcgc ccctatcgtc aaaatcggcc gcacccactt 1477500
gcaagacgcg acgccgctga ctttgggaca ggaattttcc ggctacgttt cccagcttga 1477560
tcacggttta ggccgtctga acgatgcgct taaagacttg tatgaacttg ctttgggcgg 1477620
tacggcggtc ggcacgggtt tgaacagcca tcccgaatac gccgaaaaag ccgccgccaa 1477680
actcgccgaa ttgtccggct tgccgtttgt cagcgcgccg aacaaatttg aagccctggg 1477740
cggacgcgat gccgccgttg ccgcttcggg cgcattgaaa acgctggcgg caagcctgaa 1477800
caaaattgcc aacgacatcc gttggctggc aagcggcccg cgttgcggtt tgggcgaaat 1477860
caaaatcccc gaaaacgagc cgggttcgtc cattatgccg ggcaaagtca acccgaccca 1477920
atgcgaagca atgacgatgg tgtgctgcca agtgttcggc aacgacgtta ccatcggtat 1477980
ggcgggcgcg tcgggcaatt tcgagctgaa cgtctatatg cccgttatcg cctacaacct 1478040
cttgcaatcc atccacctgt tgggcgacgc gtgcaacagc ttcaacgaac actgcgccat 1478100
cggcatcgaa cccgtgccgg aaaaaatcga ctatttcctg caccattccc tgatgctggt 1478160
taccgcatta aaccgtaaaa tcggttacga aaacgccgcc aaagtcgcca aaaccgccta 1478220
caaaaacaac aaatcgttgc gcgaaaccgc cgttgagttg ggcttgctga cgggcgaaga 1478280
atttgacgaa ctggtcgttc ctgccgatat ggttcatccg cgctaatcct tccctcaaat 1478340
aaaatgccgt ctgaaacctc gttcggacgg cattttccgt tgcctgcaaa ctagcggcgt 1478400
ttgaacagcc tgtcccccac cgccgccgta accgcacccc cgaccacgat cagtgcgcct 1478460
gcataaccca aaccgttcat atccggcgcg gcaaaagtat caggcatcac ataatgcccg 1478520
agcaaagaaa atattacggt aaacacgggg agcaaggttg ttaccgcgct gactttggaa 1478580
gcctcccaat gtttcaacgc ctcgccgaac gagccgtaac cgattaacgt attcaagcag 1478640
caatacgcaa aacaaaccca cgccaacgta ccgtccaaac ttccgatgtg tgccggttcg 1478700
gcaaacggca ggaacacggc ggcacttgcc gcataaatca acagcagaat ctgttgcggc 1478760
ccgaattgcg ccgacagcag cttttgcgcc acggcataac acacccatgc catactgcct 1478820
gccgcacaca gcaacacgcc cttcgcatac gcgcccaaac ccgacaactc gccgaattta 1478880
tcgttaaaaa acataagcaa accggcaagc agcaaaacca agccgatttt ctgagcggca 1478940
gtcatccggt ctttaaacac caacacaccg acaacaatca tcgtaaacgg cgaaatctgc 1479000
cacaaaacct gcgtcgtggt cggcgaaata taatgcagcc cttgggcaat cagcacaaag 1479060
tttgccgaaa tgcccgccac gccgagcagc agcagcctga atgagcacca agaaaaatcc 1479120
cgccgcttcg gcagccgccc gcccagtgcc agcaaaacaa acaataccgc cgccgccacg 1479180

gtaaaacgca cccacaccag cgtcggcgca tcgacaaact tcaatacctg ccgcacggca 1479240
atcggcagcg ttccccacgt catcgccgcc aaaagtgcca acgcgaagcc taggagcggc 1479300
ctttggtttt ccatcctgat tttcctattt ttaaacaacc gtattgccgg acgatgccgg 1479360
tttgccgcat cgggcaatga tggttcaagc gtttggcgtt tgattccaac cctttgattt 1479420
caaacaaacc ggctgaagct cggctattgc ttcgcgctat ttgaaaacac cgcctgaatt 1479480
ttaaaatata gtggattaac aaaaaccagt acagcgttgc ctcgccttag ctcaaagaga 1479540
acgattctct aaggtgctca agcaccaagt gaatcggttc cgtactattt gtactgtctg 1479600
cggcttcgtc gccttgtcct gatttttgtt aatccactat accgtctgaa aacagcgggg 1479660
acgtgcggca ggcatcactg cctcaaaacg cccgaacggc gcaatcgcaa cgttccgccc 1479720
aacgcaaagg gcggcaacaa gccggcccaa atgcaaaaaa gagaaaccct gccccgtaag 1479780
gtttaaggtt tctccgtcct ttatgatttc cctccgcgag gatgtccggc cgtaaaattc 1479840
agaacgggat atcgtcgtca atgtcctcga ccggggcggc ggcaggcacg ggttggcggc 1479900
gcggcgcggc tggtgcttct tggggatggg acggcgcgtc ggaggcgggc tgccggcttt 1479960
gctgcgcggg gcgttggtaa gcctcctgac tctgaccgta accttcctcg taaggcgcac 1480020
cgccgctgtt ttcattgcgc ccgcccaaca ttttcatttc gttggcgaca atatcgtaag 1480080
cggtgcgttc gatgccgtct ttgccttggt atttgcggct ttggattctg ccttccaaat 1480140
aaaccagccc gcctttttg aggtattgcc cggcaatttc cgccagtttg cggtacatgg 1480200
tgatgttgtg ccactcagta cgctctacac gttggccgtt gcggtcgttc caagtttcgc 1480260
tggtggcgac gctgaaatta caaaccgcct cgccgttggg catatagcgc acttcgggat 1480320
cgcgtccgag gcggccgatg aggatgactt tgttcaatga cattttttaa actcctgtga 1480380
tgatttttc agcggcagcc tgatcgaaac ccttctgcaa cactttgaga tagacggtct 1480440
gcccgtcgaa actgaaaccg atgtcttcca caccctcaag ctccgacaag gcgcggtata 1480500
acccttcctg attgccctgc cacacgccgc cgacagggta actgaggttt ttgacgggct 1480560
tgggcgcagg cgataaaacg gcaattacca gccacagcag catcaatata ctgcaaaagg 1480620
caaacacgcc ggaaaagccg tatttttgaa acagcaaacc gcctgccgcg ccgccggcaa 1480680
acagtccgag cgactgcatc gtgttgtaca cgcccatcgc cgtacccttc aggtcggacg 1480740
gcgcgatttt ggaaaccata gacggcaggc tcgcttccaa cacattaaaa ccgataaagt 1480800
aaacaaccaa ataagcggta atcaagccta ccgagcgcat accggacagc aaaccgagct 1480860
gcgccgccgc aatacagacg atacccaaaa caaaaacctg cttaagcttg ttgcgcgtct 1480920
cgccgacgat aatcagcgga accatcacca ccaagcccgt aatggtcgaa ggcagataga 1480980
ctttccaatg ctgtattttt tccaaaccga gctgggtcat cgcgaaaggc agcgcggtaa 1481040
acaatgccat ttgtgcggcg tgcagggcga aaatgccgaa atcaagcgtc agcagcctac 1481100
ggtttttcaa aacttcgcct atgcgcgaag gctgcgcctg cgtatcttcg tgcagcttgg 1481160
aaacttcggg atcgggagtc atccacgcca ccacgccgat gctgatgacg gtcagaatgc 1481220
cggtcagcat aaacagtccg cgaacgccga ccgcgtcggc aatcacgggg gcaacgacga 1481280
ggctgaccga aaacgtcaaa ccgatactca aaccgatcat cgccattgcg cgggtacgta 1481340
cgccgtcgcg cgtcaaatcc gccagcagcg cggtaaccgc cgcactgacc gcccctgcac 1481400
cctgtatggc gcgtgcggcg accagcatgg gcagcgtatc ggcggcggcg gcaagaaagc 1481460
tgcccgccgc aaacacgacc agtcccgcat aaatggtttt cttgcgcccg aacttgtcgg 1481520
aagcgatgcc caaaggcagt tgcagcagag cctgtgtcag cccgtaaatg cccattgcca 1481580
gcccgaccag cgttttgttg ccttccgcgc cgggcagcga ggcggcatac accgccaata 1481640
cgggcagcac gaggaacata cccagcatac gcagcgcgta cacgccggaa agcgtcgtac 1481700
tggcgcgcca ttcgtgcgga aacatttgga tgcggttgtc tcttgccatc atattttttc 1481760
agacggcatc aacagttgca atgccgtctg aacttccagt gaacagattt tcggattata 1481820
caggattcgc cgtatttcgg ttgcggcgcg ggttcaaaat caacgccact gccagcggtt 1481880
gcgccacgcg cccaaaacgg cgttcggata tttattgctg cccaagctgc cgttaaagcg 1481940
ggcaagcgcg cggacgatgt tgccttttc aagattccgg taatggcgca ggatggtaca 1482000
gccgtaacgc aggttggtgc ggatgtcgaa caggttgtgc gccggtttgc cgatgtagtt 1482060
tttccaaaac ggcataacct gcatcaggcc gcgcgcgccg acaccgctga ttgcatactg 1482120
gcggaacgcg ctttccacct caatcagccc caacacaatc tgcgtatcca aaccggcccg 1482180
gctgctttcg tactggatat tgaccagcag cctgcgccgc tcctcctcct cggggacgaa 1482240
ccttgccaaa cgtgccgaca tggcagacaa ccaacgctcg ccctctttcg gattgtcaaa 1482300
caccagcctc ggcggattga cgctgccgac agaactcctc atcacggaag ccacatcgtc 1482360
ggcaagcgtt tcctcacgtt gcgcgccggc gtgcgccaga ggactgagca acaacgcacc 1482420
ggcggcacac aacaggcggc ggcgttgcag attaacgggt agggtatcgg tcggttttct 1482480
catagggaac gggggcgcgt ccggacgttt cagacggcat taaatattca aacagacata 1482540
attgctttca acgcgaaaaa ccgcgcgcaa aatccaagcg cggcatatcg ccctgcccct 1482600
ttcgggcaaa cctcaattct accgccctca agaacgcttg tccaaacagg cacaggcaac 1482660
accgcccggg catttccgtt ttcaccggtt atccgtcgtc cggattatgc agcagcacca 1482720
tcagcgcatc acgcttttcg ggcggcagca ggcggaaata tagtagatta aatttaaacc 1482780
agtacagcgt tgcctcgcct tgccgtactg atttaaattt aatccactat atcttgaggc 1482840

ctttgcaaaa ttcctttccc tcccgacagc cgaaacccaa acacaggttt tcggctgttt 1482900
tcgccccaga tacctcctaa ttttacccaa ataccccttt aatcctgccc ggacacctga 1482960
taatcaggca tccggggcac cttttaggcg gcagcgggcg cacttagcct gttggcggct 1483020
ttcaaaaggt tcaaacacat cgccttcaga tggctttgcg cactcacttt aatcagtccg 1483080
aaataggctg cccgggcgta gcggaattta cggtgcagcg taccgaagct ctgttcgacc 1483140
acatatagtg gattaacaaa aaccagtacg gcgttgcctc gccttagctc aaagagaacg 1483200
attctctaag gtgctgaagc accaagtgaa tcggttccgt actatttgta ctgtctgcgg 1483260
cttcgtcgcc ttgtcctgat ttaaatttaa tccactataa cgggttttcg acaaatatcg 1483320
gttgcgtttg gtttgcgcct ccgtcagcgg acggttgcgg caggctttgc gcataatgcc 1483380
gttctgcaac cgatgctctt tcagttttcc gtaggtcgga ttctcgaatc cgacattact 1483440
tcaatcgtat ccaatagaaa agtccgcatt gccgccaccc caattatgcg gataaatacc 1483500
ctgtttgaca taacggtgaa acgtagaaaa cccccaatcg gaaatttgtc ctacatagcc 1483560
atgtttgacc ggattgaaat gcagataatc aaaatgccag gcaaaatcgg cctcatcgcg 1483620
gatagtatat tcccaaaagc gtttttgcca aagcctgaga ttgccgccga ttaaatattg 1483680
gctgtgccgc ttgatttgcc gccagcgttc cgaataagca gaatcattgt ccggcagccg 1483740
ccatatggta tgcagatggt cgggcatcaa cacccatgcc aaaatttcaa acggataccg 1483800
ttcgcgcacc gccattaccg cctgccgtaa agccaaacgc accgcatcat cggtcaaaat 1483860
cttctgccgt ttattggtta caaccgtaaa aaagtaagtg ccgccattgc ggtaaaaacg 1483920
acggtatttc atagtattat gctcggaatg attttgtagg tcggattctt gaattcgaca 1483980
ttttgggcat tgctgcaatg gattgcaatg atgggaatgt taaaggtttt gtcggataca 1484040
agtatccgac ctacgcttgc tgaaccgtca ttcccacgaa agtgggaatc tagaatctcg 1484100
gggtttcagt catttccgat agattcccgc cgcgtcaggg ggtctggatt cccgcctgcg 1484160
cgggaatgac gggtttcaag attgcagtgt tgtcgggaat gacgggtttc aagattgcgg 1484220
tgttgtcggg aatgacgaat ccatccatac ggaaacctgc accacgtcat tcccacggaa 1484280
gtgggaatct agaatcccgg ggtttcagtc atttccgata gattcccgcc gcgtcggggg 1484340
tctagattcc cgcctgcgcg ggaatgacgg gtttcgagat tgcggtgttg tcggaacgca 1484400
actgaaccgt cattcccacg acagtgggaa tctagaatct cggggggttca gtcatttccg 1484460
atagattccc gccgcgtcag gggggtctaga ttcccgcctg cgcgggaatg atgggtttca 1484520
agattgcggt attgtcggga atgacgaatc catccatacg gaaacctgca ccacgtcatt 1484580
cccacgaaag tgggaatcta gaatcccggg gtttcagtca tttccgatag attcccgccg 1484640
cgtcagggag tctggattcc cgcctgcgcg ggaatgacga atttcgagat tgcggtatta 1484700
tcgggaatga cgaatttcga gattgcggta ttgtcgggaa tggcgggttt caagattacg 1484760
gtgttgtcgg gaatgacggt tcgggtattt ccacgcccgc cccgcgcctg taaacggcag 1484820
gtgaatcaaa aatgccgtct gaaggttcag acggcatcgg tgtcggggaa tcagaagtgg 1484880
tagcgcatgc ccaatgagac ttcgtgggtt ttgaagcggg tgttttccaa gcgtccccag 1484940
ttgtggtaac ggtatccggt gtctaaagtc agcttgggtg tgatgtcgaa accgacaccg 1485000
gcgatgacac caagacctaa gctgctgata ctgttgcttt cgtgataggc aggtttgttg 1485060
gtcggacctt gtacgatttt gcctggcact gtagcgcctt gcgctggtgg actgaaagta 1485120
gtcgtggttt cttttctcac cgaatgaacc tgatgtttaa cgtgtccgta ggcgacgcgc 1485180
gcaccgatat agggtttgaa tttatcgaat ttatcgttga gtttgaaatc gtaaatggcg 1485240
gataagccga gagaagaagc ggcgtggaat gtaccgtttt cctgattttc cgtcttcagt 1485300
tcttgccaga tgccactgct attgtttttt tgcaactctt ttgtgtttac ggaatattta 1485360
ttgttgttcc attttctgta actggcataa tctgccgcta tcctccagcc gccgaaatcg 1485420
tagccgaccg acacccgggg gtggatggaa tgcgcacgga tgtttctgaa ataatcgctt 1485480
actgtgcttg tgttgtttgc accggttgct ttcggataat cgtgggtaat gcgttcggcg 1485540
gcataagcta aatccgcctg cacataatac gggctgcggc tgccgtcttc acttgccgcc 1485600
tgcgctgcgg aagagaagag aagagaagag aagagaagag aagagaagag aagagaagag 1485660
aagagaagag aagagaaggt tttttggggg ctggattcat tttcggctcc gtattcggtt 1485720
ttaactgatt aaaaagaaag attttcaatg atgttgcagg agcggactat atcaggtttg 1485780
tggcgatgtt tcaacacaat atagcggatg aacaaaaaag agaacgattc tctaaggtgc 1485840
tgaagcacca agtgaatcgg ttccgtacta tttgtactgt ctgcggcttc gttgccttgt 1485900
cctgattttt gttaatccgc tataaacaac gcttcgtccg aaaaaacgat tgaatttgcg 1485960
ggcagaagct ggacgaaaac cgccgacagc ctgccgcaaa aggcacacgg tttgcgctag 1486020
ggcttaggcg tgtcgcgcga aatcaatgcg ggcaggcatc atttcctcta cggcggcatc 1486080
agcggcggcg gcgtgcatta ttgggataac aaagatttca gcgaacagag cctgcgcctg 1486140
tcgttcggct ataaaaaccg ttcggtaacg cgctcgttcg gcatcgtgcc gtttgtcgag 1486200
caaaacctct taggcggcag ccgatacaat ttcgtcggcg gcttcaatgc cgatttctcc 1486260
caacgcttga gcgaacgctg gcggttgaca ctaaacgcgg gcaatatgtg gaagcattat 1486320
caggaagacc gcaccgccgc ccgatacgac agccatatgc cgctggcggg cgcgacgctg 1486380
atgtattccg cgccgaaaga ctggctgctt tacggcggtg cggactggtc gcacaacata 1486440
acgaaagagg cggaacaggc ttccatccgc aagggtttgc gtgtcggcgc ggtcaaaacg 1486500

ttcgacggcg gcttgggtct gcgggcaaac ctgcgctata cccgcaggat gtttgacgca 1486560
cccgggacca ttgtgtaccg cttcccgcgc aaagaccacg aatatcaggc aaacctgtcg 1486620
ttgtggcatg acaaaatctc ttggaagggc tttacgccgc aactcaattt ccgctatctg 1486680
aaaatcgaca gcaatatgaa aagtttttac acacgcaaaa acatgcagat tttcatgagc 1486740
gtggaaaagg atttcaaata agcgcaaaaa atgccgtcgg caacatccgt gggcagaatc 1486800
aaaaaccgcc gcatcattta ttgtcaacgc ctgcgccgtc agagtaacat tgcgtttttc 1486860
ccccaccggt atccgccatg accaccaccc ccgcaaacgt cctcgcctcc gtcgatttgg 1486920
gttccaacag tttccgcctc cagatttgcg aaaacaacaa cggacaatta aaagtcatcg 1486980
attcgttcaa acagatggtg cgcttcgccg ccggactgga cgaacagaaa aatctgagtg 1487040
ccgcttccca agaacaggct ttggactgtc tggcaaaatt cggcgaacgc ctgcgcggct 1487100
tccgccctga acaggtacgc gccgtggcaa ccaacacatt ccgcgttgcc aaaaacatcg 1487160
cagatttcct tcccaaagcc gaagcggcat tgggtttccc catcgaaatc atcgccgggc 1487220
gcgaagaggc gcggctgatt tataccggcg tgatccacac cctccccccg ggcggcggca 1487280
aaatgctggt tatcgacatc ggcggcggtt cgacagaatt tgtcatcggc tcgacgctga 1487340
atcccgacat taccgaaagc ctgcccttgg gctgcgtaac ctacagcctg cgcttcttcc 1487400
aaaacaaaat caccgccaaa gacttccaat ctgccatttc cgccgcccgc aacgaaatcc 1487460
agcgtatcag caaaaatatg aggcgcgaag gttgggattt cgccgtcggc acatcgggtt 1487520
cggcaaaatc catccgcgac gtgcttgccg ccgaaatgcc ccaagaggcg gacattacct 1487580
acaaaggcat gcgcgccctc gccgaacgca tcatcgaagc cggttcggtc aaaaaagcca 1487640
aatttgaaaa cctgaaaccg gaacgcatcg aagtttttgc cggcggactt gccgtgatga 1487700
tggcggcgtt tgaggaaatg aaactcgaca ggatgaccgt aaccgaagcc gccctgcgcg 1487760
acggcgtgtt ttacgatttg atcgggcgcg gtttaaacga agatatgcgc ggacaaacgg 1487820
ttgccgagtt ccaacaccgc taccacgtca gcctcaatca ggcgaaacgc accgccgaga 1487880
ccgcgcaaac ctttatggac agcctctgcc acgctaaaaa cgttacagtt caagagcttg 1487940
ccttgtggca acagtatctc ggacgcgccg ccgcgctgca cgaaatcggt ttggacatcg 1488000
cccacaccgg ctatcacaag cattccgcct acatcctcga aaacgccgat atgccgggtt 1488060
tctcacgcaa agaacagacc atacttgccc aactggtcat cggtcatcgc ggcgatatga 1488120
aaaaaatgag cggcatcatc ggcaccaacg aaatgttgtg gtatgccgtt ttgtccctgc 1488180
gccttgccgc actgttctgc cgttcgcgcc aagacctgtc tttcccgaaa aatatgcagt 1488240
tgcgcacgga tacggaaagc tgcggcttca tcctgcgtat tgacagggaa tggctggaac 1488300
gccatcccct gattgccgac gcattggaat atgaaagcgt ccaatggcaa aaaatcaata 1488360
tgccgttcaa agtcgaggcc gtctgaacct tgcggaacaa atgccgtcca aaccctgtcc 1488420
agacggcatt tgcctgtccg caacatcccg atatgcgcgg cacatctgct cggaacggtc 1488480
atgcaggcgt aaaaaacaag ggcacataa cccaaaaacc gcctgaaaat cttcaggcgg 1488540
tttcgtttgg gttgccggca ggcggcatcc catcattttt gccaaggcaa caaattattt 1488600
ggcggcatct ttcattttgt ctgccgcttc ctgagtcgcg tcggcagctt tgttcaaagt 1488660
atctttagct gcttcagtta cagcttcttt ggcttcagtt acagcttcct cggcacttgc 1488720
ctttgcatca gccgcagcat ctttgacttg gtctttcgct tcttcgacgg cagaagcggc 1488780
agactcggcg gcagaagccg cagtgtcttt aacatcggac tcaacggctt gaaccgcttc 1488840
cttaacctcc tgtttggctt cttgcgaaca agctgccaag gcagccgcca tcattgcggc 1488900
aatcaataat tttttcatgt cttatccttc ttgagttgtt gattaaggtt ttgcttaaaa 1488960
atcggaccgt gttccatcaa tcggctgatt ttgcccatcg accggagaga aaacggtttc 1489020
ccgtttagtt aaaacccatt atatttaaat ataaaggttt ttttctcgaa caataaggcg 1489080
gcatcaatgc catattgaaa cacgtccgaa aactatttta tgaaaacagt tcggaaaatt 1489140
gtaacacata tccccctcct tttgagtttc ccgacggtgc ggactttttc ctgcagggtt 1489200
tgaaaaaccc aaatatattc cgggatgtcc gaatacctca ataatggcgg cggcggaaat 1489260
aaaacgcccc ttcgctgtcg atttccagca catagcgtcc gttctgcacg gcggcatagc 1489320
cgcttttgcc tgcctgatag ggttgcaggg cggcatgcga aactaggtaa tccgtcagtt 1489380
tgccgccgtc ttcggcgata ttgcccacca gtttggcaaa caaggtatgg cacacgccgt 1489440
tttctgccca acctgccgga ctgtccttat catcggtttc catacatttg ccgctgacgg 1489500
cttccaagtc gccgggatgc ttgccgatca gtcggataac attttgttcc ggcaagcctt 1489560
taatcggata actgatttgt tttttgccgt cgttggtttt gccttcgctg ctttgtccca 1489620
aatccaaacc ggcaatcgcc gtattgtcga tatatttgac tttgaaaacc ggtttcggcg 1489680
cgctttgtac cgcgttttgc ggctgttccg ccgtattttc ggatttgccg caggcggcaa 1489740
gcagcaggca gccgcccaat acggcaaaag atgttttcag cattccacac tcctgatggt 1489800
ttcaaaatgc cgtctgaaac gcggcaggcg gaggttcgga cggcatcggg ttcatttcaa 1489860
cgggcggatg ccgaccgcat cgcgtacttt gtccaataat tcgcgtgctt ctttacgcgc 1489920
tttcgccgcg cctgcctgca aaatctcttc gatttgcgaa gggtcggcgg tcagctcgtt 1489980
gtagcgttcg cgcggttcgg cgagttcggc gttgattttc gccgccaaaa gttttttggc 1490040
ttcaccccac gccaagccgt cggcaagcat tttcgtaaat tccaccgttt cagacggcgt 1490100
ggagaaggct ttgtagattt caaacaatgg gctttcgtcg ggctgtttcg gctcgcccgg 1490160

ctctttcata ttggtgatga ttttgttgac cgatttttgg gttttttgt cgttttccca 1490220
aagcggaatg gtgttgccgt aggatttgga cattttgcgt ccgtccaaac cgaccaagag 1490280
ttcgacgttt tcatcgattt tcacttcggg cagggtgaag agttcccgga agcggtggtt 1490340
gaagcggccg gcgatgtcgc gcgccatttc gacgtgttgg atttggtcgc gcccgacggg 1490400
cacttcgttg gcgttgaaca tcagaatatc ggcagtcatc agaatcggat aactgaacaa 1490460
acccatttcc acaccgaaat cagggtcttc ctgcccgttt tctgcatttg cctgcacggc 1490520
ggctttgtag gcatgggcgc ggttcatcaa acccttggca gtgatgcagg tcagaatcca 1490580
gttcaattcc atcacttcgg gagtgtcgct ttggcggtag aaggtggtgc gctcggggtc 1490640
gagtccgcag gcaagccaag tggcggcaac ggcttgggtg gattggtgaa tcatctccgg 1490700
ctcgtggcat ttgatgatac cgtggtaatc ggcgaggaag aggaaggatt cggtatcgag 1490760
gttttgcgcc gcgcggacgg cggggcggat ggcgccgacg tagttgccca gatgcgggat 1490820
gccggtggtg gttacgccgg tcagaactcg ttttttgctc ataaaaatgt ccttgcggca 1490880
tcaatgccgt ctgaaaggga aaaagatgtg ccgattatac ccgatttgcc acctacatcc 1490940
agccgacaac agacttttcc atattaagaa gatatagtta tacacattat tatacatttt 1491000
tatatacttt aaattcaatg atatatcgaa ttaaatatag aaaaacagaa aacagaactt 1491060
gagttatcca caattatgca catataggct tcgacagcgg acattttgaa aaggaaacaa 1491120
aaatgcgata cgacaaatta accgccaaat tccaacaagc ccttgcagaa gctcagagtt 1491180
tggcgttggc tgcggacggc agctatctgg aagcgggctt tgtgttaaaa gccctgcttg 1491240
acgaccaaaa cagcggagcc gccgcgctct tggctcatgc gggcgtgaac gtgccgcagg 1491300
tgaaacagcg tttgcagcag catttaaaca gcctgccgaa agtgtccggt cagggcggcg 1491360
atattctgcc cagccgagaa ttgcaggcgg tgttgaacct gatggacaaa gctgccacca 1491420
aacgcagcga tgcctatatt gccagcgaac ttttcctgct tgccttggta cagcagaacg 1491480
atgcgaccgg caaaattttg aaagaagccg gcgcgaccga acaaaacatc aatgccgcga 1491540
ttgacgcagt acgaggagga caaaacgtga acgatgccaa tgccgaagac caacgcgatg 1491600
ctttgaaaaa atatacgctt gacctgaccc agcgcgcccg cgacggcaaa cttgaccccg 1491660
ttatcggtcg tgacgacgaa atccgccgcg cgattcaggt attgcaacgc cgtaccaaaa 1491720
acaaccctgt gctgattggt gagccgggtg tgggtaaaac cgccattgtt gaaggcttgg 1491780
cgcaacgtat cgtcaacggc gaagtacctg aatccctgcg taacaaacgc ttgctggttt 1491840
tggatttggc ggctttgatt gccggcgcga aataccgcgg cgaatttgaa gaacgcttga 1491900
aaggcgtgtt gaacgatttg gcgaaagacg acggcaacac tctgattttc attgatgaaa 1491960
tccatacttt ggtcggcgcg ggcaaaaccg acggcgcgat ggacgcgggc aatatgctga 1492020
aaccggcttt ggcacgtggc gaattgcact gtatcggcgc gaccactttg gacgaatacc 1492080
gccaatacat cgaaaaagat gcggcactcg aacgccgctt ccaaaaagta ttggttggcg 1492140
agccaagcgt ggaagacacc atcgctattt tgcgcggttt acaggagcgt tatgaaatcc 1492200
accatggtat cgatattacc gaccctgcta tcgttgccgc agcggagttg agcgaccgct 1492260
acattaccga ccgcttcctg cccgataaag cgattgattt gattgacgaa gccgccagcc 1492320
gtgtcaagat ggaaaaagaa accaagccgg aagcaatgga caaaatcgac cgccgtctaa 1492380
ttcagcttcg gatggaaaag gcgcacgttg aaaaagaaaa agacgatgcc agcaaaaaac 1492440
gtttggaact gatagacgag gaaatcaacg gtctgcaaaa agaatacgcc gatttagacg 1492500
aaatctggaa agccgaaaaa gcaatttcag acggtgctgc taatattaag aaacaaattg 1492560
acgaagtcaa aattaaaatc gaacaggcaa aacggcaagg cgatttggca ctggcttcaa 1492620
aattgatgta tgaagatttg gagcatttgg aaaaacagcg tgcagccgcc gaacgggcag 1492680
atacggacag cacaaaaccg gcaaacaaac tcttgcgtaa taatgtcggc gcagaggaaa 1492740
tcgcagaggt ggtttcccgt atgaccggca ttcccgtatc caaaatgatg gaaggcgaac 1492800
gcgacaaact gctgaaaatg gaagaagtat tgcaccgccg cgtggtcgga caggacgaag 1492860
ccgtgcgtgc cgtgtccgac gctatccgcc gcagccgctc cggtcttgcc gatccgaaca 1492920
agccttacgg cagcttcctg ttcttgggcc cgaccggcgt gggtaaaacc gagttgtgta 1492980
aagccctggc aggctttctg ttcgacagcg aagatcatct gattcgcatc gatatgtccg 1493040
aatatatgga aaaacacgcc gttgcccgct taatcggcgc gcctccgggc tatgtcggct 1493100
acgaagaagg cggctacctg accgaacaag tgcgccgcaa accgtacagc gtgattctgc 1493160
tggacgaagt ggaaaaagcc catcccgatg tgttcaacat cctgctgcaa gtattggatg 1493220
acggccgctt gaccgacgga caaggtcgca ccgtggactt caaaaatacc gttatcgtga 1493280
tgacttccaa tattggtagc caacatatcc aacaaatggg cattcaggat tacgaagcgg 1493340
tgaaagaagt tgtgatggag gatgtgaaag aacatttccg ccccgaaatg atcaaccgca 1493400
tcgacgaagt ggtcgtgttc cacggactgg atcaggataa tatccgcaac attgcgaaaa 1493460
tccagctcaa aggcttggaa aaacgtttgg aaaaacaaaa cctgcgcctg gctgtttccg 1493520
atgccgcact ggacatcatc gccaaagccg gtttcgaccc gatttacggc gcacgtccgc 1493580
tcaaacgcgc catccagtcg gaaatcgaaa acccgctggc aaaagccctg cttgccggaa 1493640
actatgcgcc cgaaagcgaa atcagggtgg aagccgacgg cgacagactg aaatttgcct 1493700
gattcgttcc tgctgttgaa aatgccgtct gaaacgggaa tctccgtttc agacggcatt 1493760
ttttatcctc ggcagacaaa ccgtcccctt attggcggta ggtttgcagg aatcttgcca 1493820

gcctgcccat cgcctcttca atctgatgga cgtaaggcag cgtaacaatg cggaaatggt 1493880
cgggcttgat ccaattaaac cccgttccct gcaccagcaa gactttttcg cgcaccagca 1493940
aatcgtaaac gaatttcatg tcatcgcgga tacggtacat ttcggtatcg atttttggga 1494000
acatatacat cgcgcccatc ggtttgacgc aggatacgcc gggaatctgg ttgaccagtt 1494060
cccacgccct gttgcgctgt tccaaaagcc gtccgccggg caaaatgaat tcgttgatgc 1494120
tctgatagcc gcccaatgcc gtctgaatcg cgtgctgcat cggcgtattg gcacacaggc 1494180
gcatagacga gagcatatcc aaaccctcga tgtaaccttt tgcatgatgt ttcggcccgt 1494240
tgagcaccat ccagccttgg cggaatccgg ctacacggta ggctttggac aaaccgttga 1494300
acgttaccgt caaaaggtcg gggcaagcg cggcgatgtg gtggtgaacc gcgccgtcat 1494360
aaaggatttt gtcgtaaatc tcgtcggcga aaataatcaa accgtgcttg cgcgccagtt 1494420
cggcgatttc caacaggatt tccctgctgt acaccgcgcc tgtcggatta ttgggattga 1494480
tgacgacgat ggctttggtt ttgggcgtga ttttggcttc catatcggca aggttgggga 1494540
accagccgtt ttcttcgtcg cacagataat ggcgtaccgt accgcccgca agcgttgccg 1494600
ccgccgtcca caagggatag tcgggcgcgg gaatcaggat ttcgtcgccg tcgttgagca 1494660
atgcctgcat agacatcgta atcagctcgg acacgccgtt gccgatatag acatcatcaa 1494720
ccgtaatatc gcgcaaacct ttggtctgat agtagtgaac aatggctttg cgggcggaat 1494780
acagcccttt agaatcgcaa tagccttgcg aagtcggcag gttgcggatg acatcgacca 1494840
agatttcatc aggggcttca aagccgaacg gcgcagggtt gccgatattg agtttaagga 1494900
ttttattgcc ctcctcttcc aactgaaggg cttttttgtg aaccggcccg cgtatgtcgt 1494960
aacagacgtg atcgagcttt gcagacttgg gaaatttatc catgatgtgt tccgtaaatt 1495020
ttgggcaatg ggtgggaatg tactcttttt cacgcggaat ttaaagcatc aaaccgagat 1495080
tttcaggctt tttacctgcc ctctttgcgc cgttcgctga cgcttttgcc gcctattccc 1495140
cagttatcgg tatccacttc gtcaatcacg acaaccgttg tttcgggatt tttgcccagc 1495200
acgcgtgcca gcaattcggt tacgccgccg atcagttccg ctttttgcgc ggcagtcggt 1495260
gcttccttgc cgccggttac tttaatattg acataaggca tgatctttct ccgttttaaa 1495320
atattgctat cttatcaaac aagttgcctc cgcccaaacg tccgcttcat tttctgaaaa 1495380
attcaaatcg atatagtgga ttaacaaaaa tcaggacaag gcgacgaagc cgcagacagt 1495440
acagatagta cggaaccgat tcacttggtg cttcagcacc ttagagagtc gttctctttg 1495500
agctaaggcg aggcaacgcc gtactggttt ttgttaatcc actatacaaa aagacagttt 1495560
tcagacagca aatccgtctt cacacgatac ctattttgtt ataacataac aaaatcttta 1495620
acccacacga gacaaaggct gcaccatgaa gaaaacattg acactgctcg ccgtttccgc 1495680
cctatttgcc acatccgccc acgcccaccg cgtctgggtc gaaaccgccc acacgcacgg 1495740
cggcgaatac cttaaagccg acttgggcta cggcgaattt cccgaactcg aacccatcgc 1495800
caaagaccgc ctgcacatct tcagcaaacc gatgcagctg gttaccgaaa aggcaagga 1495860
aaacatgatt caacgcggca catacaacta ccagtaccga agcaaccgtc ccgttaagga 1495920
cggcagttac ctcgtcatcg ccgaatatca gcctactttc tggtcaaaaa acaaagcagg 1495980
ctggaaacag gcgggcatca aagaaatgcc tgacgcaagc tattgcgaac aaacccgaat 1496040
gttcggcaaa aacatcgtca acgtcggaca cgaaagcgcg gacaccgcca tcatcaccaa 1496100
accggtcgga caaaacttgg aaatcgtccc gctggacaat cccgccaaca ttcacgtagg 1496160
cgaacgcttc aaagtccgcg ttctgttccg tggcgaaccg ctgcccaatg ccaccgttac 1496220
cgccaccttt gacggcttcg acaccagcga ccgcagcaaa acgcacaaaa ccgaagcaca 1496280
ggctttctcc gacagcacag acgacaaagg cgaagtggac atcatcccct tgcgccaagg 1496340
cttctggaaa gccaatgtcg aacacaaaac cgacttcccc gatcaaagcg tgtgccaaaa 1496400
acaggcgaac tactcgactt taaccttcca aatcggtcat tcgcaccatt aatcccgccc 1496460
gcacaaaaat gccgtctgaa ggcttcagac ggcatttttt gttcaaacat caataccaac 1496520
cgcgcagttt catcgctttt tcaacacggc ggatactcat catgtaagac gcggttcgca 1496580
aatcgacatc atactcttgc gccaagttcc atatatcgcg gaacgcgcgt cgcaggacga 1496640
cggtttcttt ctcttgaact tcgtcaaact cccaataata gccttgcagg ttttgcaccc 1496700
actcgaaata ggaaacgacc acgccgccgc agttcgccag aatatcaggc acgaccaata 1496760
cgccgttttg acgcaggatc acgtcggctt cgggcgtagt cgggccgttc gcgccttcga 1496820
ctacgatttt cgcgcggact ttaccggcgt tttcggaagt cagttggttt tccagcgcgc 1496880
aaggggcgag tacgtccaca tccaaagcca aaagttcggc gttggtaatt tctttgccgt 1496940
aaccggcttc gttggtgatg aagccttttt cttggaactc tttaaacaaa gcttccatat 1497000
ccaaaccgtt ttcgttgtaa atggcaacgt caacagtaga aaccgcaaca actttcgcgc 1497060
cggattgatg cgcgtaataa cctgtgtggt aacccacatt accgaaacct tgaatggcgt 1497120
aagtggcacc cttcacgtcc ttgcccagtt tttccaaagc ttggacggcg gcgaggttca 1497180
cgccgtaacc ggtagcctcg gtacgcgcca aagagccgcc gaactcaacc ggttttccgg 1497240
taaatacgcc cggcgcggaa tgtttcacca cgttttcata agcatccacc atccacgaca 1497300
taattttgcc gttggtattc acatcggggg cgggaatatc gattttctcg ccaatcagcg 1497360
gggcaatcgc ttcagcataa gcgcgggcga tgcgttccag ttccgcctcg gaataatcgc 1497420
gcggatccaa ggtaatgccg cctttgccgc cgccgtaagg aatacccgca acgcagcatt 1497480

```
tgatggtcat ccaaattgac agggctttga cttcgtccaa attcacactg ggatggaagc 1497540
gcacgccgcc tttatagggg ccgacggcgt tgttgtgttg cgaacggtag cccgtgaagg 1497600
ttttgaccgt gtcgtcgtcg agtttgacgg gaaaattgac ttccaacacg cgggtcggac 1497660
tcttcaggat ttcataaacg gccggatcgg ttttcagccg gtcacaggcg gttttcacct 1497720
gtttgcgcgc gatttcaaac ggattgaggg tttcttttgc aagggcttca gacattttgc 1497780
ttccttttca caaagagagg ttcggaatgg aacaagccat caggttcgca actataacca 1497840
attttcaagc aaaatgtaat agcgtgtagt tggaatcggc ccgatttgat taatctatat 1497900
atgattttat ttcccaagcc gcacggaatc cgtctgaaaa aagcggaaca catatccaaa 1497960
aagcaaatgt ccaattaaat aaagatataa gaatcctttt attttttaaa aatttaattg 1498020
gaacggcgcc gggatttgca caccccttccc gactccgttc cgaaatccgg aaacaccgcc 1498080
ggcaaaacct gtttcgattg ttaacaatcc atacattaga agccctgtgc aaacgatgtt 1498140
aaaataaacc ttttcaaccc gacagaaaac cggattatga atgcagccat cgaacacgtc 1498200
caagccgtcg ccttcgattt ggacggcaca ctgtgcgatt ccgtccccga ccttgccgcc 1498260
gccgcagaag cgatgttgga acaactcggt atgaaaccgc tgcctgccaa agtggtcgaa 1498320
agctatgtgg gcgacggcat cggcaaactg gttcaccgcg tcctcaccaa cgaccgcgac 1498380
cgcgaagccg attccgaact gtgggaaaaa ggtttcgtat ctatatgaaa tactaccgcg 1498440
accatttgag cgtcttcacc cgcccctatc ccgaaaccga agccgggctg gcattgctta 1498500
aatctttggg catcccgctc gccgtcgtta ccaacaaaaa cgaaatcctt gcctccgagc 1498560
ttctaaaaca actgggactc gccgactatt ttagcctgat actcggcggc gacagcctgc 1498620
ccgagaaaaa acccagcccc ctgccgctgc ggcacgccgc cgaagttttg ggtatcgatg 1498680
ttgcaaacat ggttatggtc ggcgactcgc gcaacgacat catcgccgcc aaagccgccg 1498740
gctgcctgag cgtcggcgtt accttcggtt acggcgatat gacgctgctc tcgcaagacg 1498800
atgcgacccg ccccgactgg attatcggct cgctgcccga aatttacgaa aacctgcaac 1498860
ctcagaaaaa caaagaagag taggcattcg gacggctccg gtttgcgccg ctatgccgtc 1498920
tgaaacctgc cccacgccga aaccgccgcc atgaaaccgc aaaaatccct acgcgcccgc 1498980
gcgatggaca tcctctcgcg ccaagaactc agccgcatcg gtctgaaacg caaacttgca 1499040
ccgcacgccg aaagcgaaga ggagttggaa aacgtgttaa acgaatttgc cgaacgcaac 1499100
tggcagtcgg atttgcgcta tgccgaagcc tatatccgca gcaaaagccg caaacacggt 1499160
tcattgaggc tgaaacaggc tttggcgcaa cagggcatag atgaagaaac cagccgcaac 1499220
ctgcttcccg accgctcaag cgaaaaactg gccgccatag ccgtgttgcg taaaaaattc 1499280
aaacatccgg ccgccgacct taaagaaaaa caaaaacagg cacgcttcct cgcctatcgc 1499340
ggttttgatg ccgataccgt tcagacggca ttgaaacatg cctgggatga cggctgggag 1499400
gaagactgct gaactgaatc cttgaatctt tttgcatgac ggcgtaacct tacctccatt 1499460
tccaactttt ccgattgaga ataaaatgtc cgaacaatcc gagaaaaatc acaacccact 1499520
tcttgaagat gaacgcaaaa acccggttta ccgtatgggt caggcagttg ccggattcat 1499580
gctcgtcgtt tgggcaggcg tattggcact cgtgtttttc ctagtcttcc gtttttggct 1499640
ttcctaaaca aaatgccgtc tgaaaccttc agacggcatc ggcagcccat ttcggcaggc 1499700
tatcccatca tagctttttt tagcttgaat tccactttcc cattccctaa aatttttcca 1499760
cacccatttc aaaataccct ttcttaaaac aggtacacta tgacacaaca acgccaactg 1499820
ccttcgcacg aactcattat gtccgaactg atgatgccgg acaccgccaa tttcagcggc 1499880
aacgtacacg gcggcgaact cctgctcctg ctcgaccaag tcgcctattc ctgcgccagc 1499940
cgttacagcg gcaattattg cgttaccctg tcggttgaca aagtcctgtt taaagaaccc 1500000
atccatgtcg gcgacctggt tactttctac gccagcgtaa actacacggg gcgtacctct 1500060
atggaaatcg gcatccgtgt cgaagcacaa aacatccgta cgggagaaat ccgccatacc 1500120
aacagctgct acttcaccat ggttgcagtc aaagacggca aacccgtccc tgtccctccg 1500180
ctggaaatcc tgaccgaccg ccaacgctgc cgctacgaaa aagccaaaaa acgcagagac 1500240
atcagcctgc aagcctccgg agacgtgtcc tgcggctgct gacggcggac tatgccgtct 1500300
gaaagacagg cacatcgcgc catccgtttc cattgcaaac ggatgaaatc aagcaaatat 1500360
agtggattaa attcaaacca gtacggcgtt gcctcgcctt agctcaaaga gaacgattct 1500420
ctaaggtgct gaagcaccaa gtgaatcggt tccgtactat ctgtactgtc tgcggcttcg 1500480
tcgccttgtc ctgatttttg ttaatccact atacccaaac acagtcaaac aaatttatat 1500540
gccccatccc ttccgaataa tttgaaaaca cagccgccaa aaacaaaaat gccgtctgaa 1500600
aacctttcag acggcatttc caacttgatt tcaggcagaa agtcagaacg cgatatagct 1500660
gttcgggtta accggtttgc cgttttgacg cacctcgaaa tgaagctgcg ttctggaagc 1500720
atcggtattg cccatcaaag caacctgctg accgcgtttg acctgctgcc cctcgccgac 1500780
cagcaatttt tggttgtgcc cgtatgcggt caggaaagaa gaattatgct ggatgatgac 1500840
caagtttccg tatcccctca aacctgaacc ggcataaacc actttgccgt cagccgccgc 1500900
caaaacgggc tgtcccgcat taccggcaat atcgacaccc ttgttgttgc cgccgaaatc 1500960
ggcaaccact ttaccttgcg tcggacgctg ccaaacaatg ccgccgaccg aacgcgtgcc 1501020
ggaaggcgaa gcggcaggag attgcggggc gggcgcggga accgctttat tttccgcagc 1501080
gggcgcggca ggttgcggcg cggactgcac aggcggttgc gcggcgggtt tcacaggggt 1501140
```

ttgcacggca gccggtacgg cgggcctgct ttctacggct gcggttttcg gtgcggcata 1501200
tcctgccggt ttgactttaa caatctgacc gatgctcaac atattgtcgg tcatgccgtt 1501260
ccacgcacgg aaatcgtctt gagagatatg gtagcgtttg gaaatgttgt acaccgtgtc 1501320
gccgcgcaca atagtatgcg tcgccgcgtt aatgtcgacg ggtgcggact gtacgggcgg 1501380
ttgcgcggca gccggtacgg cgggcctgct tttacggct gcggctttcg gtgcggcata 1501440
tcctgccggt ttgactttaa caatctgacc gatgctcaac gtattgtcgg tcatgccgtt 1501500
ccacgcacgg aaatcgtctt gagagatatg gtagcgtttg gaaatgttgt acaccgtgtc 1501560
gccgcgcaca atagtatgcg tcgccgcgtt gatgtcgacg ggtgcgtaag aaggaacgta 1501620
tgtacccgaa acggcaggtg cagacggcgg aacataagca ggaggcgtat aaaccggcgc 1501680
gctttgcacc ggcggcacat aaggcgcatc gccggcagga gccgggctgt acggcgttgc 1501740
tccatagggg ttgttgtaaa ctgccgaaga cggcgcgtcc tgcatacctg aattgcctgc 1501800
aatgacagga gcaggctgtt gggtggcgca accgcccaac agagcggcaa cggcggtaca 1501860
agctgccaaa agtgtcgttt gtttcaacat aagataacct tcatgttccg atatatagcc 1501920
tgaatgcggt atatcataat aaaaatgcgc gttcttctca agcgcaaagc ccgacggtat 1501980
agtggattaa caaaaatcag gacaaggcga cgaagccgca gacagtacaa atagtacgga 1502040
accgattcac ttggtgcttc agcaccttag agaatcgttc tctttgagct aaggcgaggc 1502100
aacgccgtac tggtttttgt taatccacta tatttgatga aacggtcagt ccgcatgcca 1502160
gaacgccgct gtttccgcca tgtccggata ggcggtcagg tcgatttgca gcggcgttac 1502220
ggtaatgaaa cctgcgccgc attcaccgaa atccgttccc tcttcccgat cggaaacttc 1502280
gccgaccggt cctatccaat aaatctgttc gccgcgcgga ttgcgcgcgg gaatgacgtt 1502340
ctgaccgtga tgcctcctgc ccaaacgggc gattttaatg ccccgcacat cttccggcgc 1502400
aacggcgggg atattgatgt tccacaaaat aggggactgc gggggggtttt tgaaaaaatg 1502460
cgccaacaat gtccacagtg cctgttctgc ggtcgcccaa tagcgtccgg aagcgtcgtt 1502520
taaggaaaac gccacggcgg gtatgcccat aaggtaggct tcggttgccg ccgcaaccgt 1502580
ccccgaataa agcgtgtcgt cccccatatt cgcgccccgg ttgatgcccg aaaagacaaa 1502640
atcggcctga aaatccgaaa atacagactg cccgatgtgg atgcagtcgg tcggcgtgcc 1502700
gttgacatag tagaacccgt tttgcgcctg tttcaactgc aaagggcgtt ccagcgtcag 1502760
cgaattgctg accccgctcc tgtcgcgttc gggcgcgacc accctgacgt tggcaaattc 1502820
cgccgtaacg cgcgccaaaa cggcaatgcc ttcggagagg tagccgtcgt cgttggaaat 1502880
caaaacgttc attttctatc ctgaatgctt attcttcggg caatttggtg attttgaccc 1502940
gctcgatgcg ctgcccttct ttttcgacca cttcaaaccg ccagccgtgg aaatcggcaa 1503000
aatcgccgac atcgggggatg gtttgcaatt cttccataat cagcccggca accgtatgga 1503060
aatcggcatc ttcctcctgc tgcggcaggt tgagttgcgg tgcgagttcc acatattcca 1503120
acgcgccttc caccgtcagg ctttcatcgg gattcccctg aacggctggt tcttcttcgc 1503180
gctcaaattc ttcggggaac tcgcctgcga tggtttcgag caggtctttc atggttacca 1503240
tgcccaatac cgcgccgaac tcgtccacca ccaaagcata atccgcgctg ctttggcgga 1503300
agagttcgat tgcgcccagc gcggtggtgc tgtcgggcag gacgagcggc tggcgcaatg 1503360
ccgtctgaat gtcgagaccg cctgtttcca gcagttggga cagcaggtct tttttgttga 1503420
tgtagcccaa aggttcgtcc acgcccgcct taccgacaac gagcaggcgg ctgtaaggcg 1503480
tgttttgcag ttgggcacac tgttcttcgc ggctttggga aatgtccagc cgttcgatgt 1503540
cgcggcgtgg gatcatcacc ccataatcg ggcgttcggc aagcgtcagc acgctgcgta 1503600
tcatcgattt ttcgttttct tcaaaatgcg cgtcgtcccc ggattcgccg cccgcgtcgg 1503660
caagcacgct ttcgcgtata cccatcatac ccaagacgtt ttcggcggtg cgcttgcgcc 1503720
acgagctgcc gatgtagtcg tttttgcggc tgttgcgctg cgaaatctgg ttaaacaatt 1503780
cgattaaaat cgagaagccg atggcggcgt agaggtagcc tttgggaatg tggaaatgga 1503840
aggcttcggc aatcaggctg aaaccgatca tcaacaaaaa accaaggcag agcatcacga 1503900
cggtagggtg tctgtcgaca aattcggtca agagtttgct ggcagaaatc attacagcca 1503960
tcgcgacgac gaccgcaccc atcgccacga cgatatgatc gaccatcgcc accgcagtaa 1504020
tgaccgaatc gatggaaaac acggcatcca gtatcaggat ttgcgcgacc acgccccaaa 1504080
acggcgcgtg ttttttttgg ctgtcggcaa cggtaaaacg gttgtgccct tcgaggcgtt 1504140
catgcagttc ggtggtggct ttgtaaagca ggaaaatacc gcccgcgagc ataatcatgt 1504200
ccttgccgga aacggcgagg ccgccgattt ggaacagcgg ctcggtcagc gtgatgatgt 1504260
gcgccataaa agcaagcata atgatgcgga tgacgactgc cagccccagc ccgataatcc 1504320
gtgcgcggtc gcgccgtgcg ggctggacct tgtttgccaa aatcgccaca aagacaagat 1504380
tgtctatccc caatacgact tccaacacca aaagcgtggc aaaacctatc caggtatgcg 1504440
gttctgccaa ccaactgaaa tccatgattt tcgtattcct caagttcaaa cgcgaaaagg 1504500
cagcctgaag cgctcaagct gcctgaacag acggtacgca caaaaaacgg cgggcgggct 1504560
tgctgctctg ctcggggtct tgcatgtgcg tgtaccttcg gtcgaaataa tttaaatagt 1504620
ttaacagctt atcggggcaa tggcaaaacg ccataccgtc tgaaaggatg ttcggacggc 1504680
atgagcttat tttgaaatgt ttcaacacac ggacggcaca taaagccttc ccctatgtgt 1504740
tgccctgatt gaggggttgc gcccctctca aatacagtct gattctaccg ccgcgaagaa 1504800

cggatgttcg agtgcggacg gagtcccaac gcttaagggg tgatgatgaa gccgtctatc 1504860
ggcgcgtagc ctttggtgtt gccctcttta tcggtaatga ctatccactc tttctgcctg 1504920
ctgctggtaa acggcaggta atacagctcc tccccttcgg cgagacctgc ctgtttcaga 1504980
atgtccgcaa ccgtcgtttt tctcgcatcc gccaagactt tcagcggttt cagatgtttg 1505040
cggatttctt ctgcttcctt gtcggaatac ggcagccact ggtcgggacg catactcggc 1505100
tcgatacctt tcagggacaa atccagcgtc ttgttcttct catccgcatc ctcaggttct 1505160
ttcaatgcaa tgcggcggat gccgaaccac gacaggcttt gcagcccttc gggggctttg 1505220
tgcaaatctt cgaccacgac ttccgccgcc gtaacaatgg tcatacgatc ctgttcaaac 1505280
gcttccacca caggacgcgc cagcgaaacg ctgtgcagac cgtacaccaa agccgccagc 1505340
tggatgatgc cgaccatgga aaaatcgacc atgcgtgcct ttgtcttttt cttcgggctt 1505400
gccaaaatta aagtcagcag cggaccacat acaatatcga cagccaccac cagctgataa 1505460
agcgacagcc ctcccgtcag ctcggcataa ggataaggat accaaacctt aaaaaccagc 1505520
aatgccgcca gccctgcaac cgacaggctg attaagaggt gccagcccgc acttttcaag 1505580
gcaaaacgcc atctcgggac tgtttttccg ttttccatca tatcttgttc aaatcaaaaa 1505640
taaccgtaaa aacagggcgc attgtacaac agatagagac tgcttaaaat gcggcgccgt 1505700
ctgaaatcct gccgttcaga cggcatccgt cacccgacat ccatacacag atatttcaat 1505760
tctagatatt cgtccgcacc gtatttgctg ccttcacgtc ccaaaccgct acgtttcacg 1505820
ccgccgaacg gtgccgcttc attgctgatt aagcccgtat tgatgccgac cataccgtat 1505880
tccaaggctt cgccgacgcg ccattggcgg gcggtgtcgg cggtgaaaag gtaagctgcc 1505940
aaaccgtatt ccgtattgtt cgcagcctcg atgacctcgg cttcggtttc aaaacggaat 1506000
accggacaca acggcccgaa ggtttcttcg cgtgccaccg ccatttgcgc cgttacgccg 1506060
cttaaaacag tcggttcgaa aaacgttccg cccaacgcgc tgcgtttgcc gccggtcagg 1506120
cagcttgcac ctttagcaag cgcgtcggcg atgtgctgct cgactttctc caccgctttt 1506180
tcctcaatca gcggcccttg gttcacacca tcctccaagc cgttgcccaa tttgagcgcg 1506240
gctgcttttt cactcaattt gcggcaaaat tcgtcgtaaa tggcggattg agcgtaaacg 1506300
cggttggtgc agacgcaggt ctgaccgctg ttacggaact tgctggcgag cgcgccttcg 1506360
acggctttgt ccaaatcggc atcgtcaaac acgataaacg gcgcgttgcc gcccagctcc 1506420
aaactgagtt ttttaatgtc cgccgcgctg tcggcaaaaa tttttgcgcc gacttcggtc 1506480
gagccggtga agctgatttt gcggataatc gggttcgtag caaattcatg gccgatttcc 1506540
gaagcactgc cgctgacaac aggcaacaaa tcctgcggta tgcccgcttc gtaagccaac 1506600
gaagccaagg catacgcact caaaggcgtg agcgatgcgg gtttgacgat catcgcgcaa 1506660
cccaccgcca aagcaggcgc ggccttgcgc gcaatcatcg cggacggaaa gttccacggc 1506720
gtaatcgcag cggtaacgcc gacgggctgt ttcaacacga ccagtttttg cgacgctttc 1506780
acactcgtca gcacatcgcc gtcaatccgc cgcgcctctt cggcaaacca gcgcacaaac 1506840
gaagccgcat aatcgatttc gccacgcgcc tcggtcaggc ttttgccctg ctccatcgtc 1506900
atcaggcgcg ctaatgcttc tttgttttct ttaatctgaa aataccaacg ccacaacaca 1506960
tcggcgcgtt ccaacgcagt ttttgccgcc cataattttt gtgctgcagc tgctttttga 1507020
atcaggtttt tcagcttgtc cgaatccgtc ttgcggacaa acgccaaagt ctcgcccgtt 1507080
gccggattat cgactttgat gccgtctgaa accgggggaa gggaaatatc gggatgcttg 1507140
attaattggg aatattcgtt catttcgtat cctccggtat gcggaataac cgctttcaaa 1507200
tgccgtcaat ctcgcggaca ttatcatctt catattccaa aactgcaaac ccttccgatg 1507260
ccgtctgaag catccgatcg ggcagcgcaa catccgggcg gtgtctgaat atggcgcggg 1507320
cgcaatccct gtcgtttaag aaaaatattt tttatacgat agtaatcttt agaaagaaaa 1507380
gtaatgcagc cctttgatgg ggtgcaatat ataaggagca aagattgcag ttgcaacgtg 1507440
tggtagagta tggcaaaaat ccgaacatta tagtggatta acaaaaacca gtacagcgtt 1507500
gcctcgcctt agctcaaaga gaacgattct ctaaggtgct gaagcaccaa gtgaatcggt 1507560
tccgtactat ttgtactgtc tgcggcttcg tcgccttgtc ctgatttttg ttaaatccac 1507620
tatacagtca aaattacgga gatcaaataa tgatttttaa acagaatcaa aattattggg 1507680
cagtttttga tgctaataaa gaaactctga ttgttcaaac atgttcaggt ttggggttaa 1507740
cggcaataga ccacctatat cccccccata tcctgccatt ggataccgac aatgaaactt 1507800
taggcacgac agtcttgcaa gcgttggcaa acagcaggac tttcgtttat gacagtccag 1507860
aagaccaaga tttttttgat accgaaaaaa ttcggcaacg ctatgaggat tgggttgcca 1507920
agctatgcgg gaacttgggc tataaaacca gacgcgccct atttaaaaac atgatgagcg 1507980
tggatatttg gctgcacaac ggctgcctga aaatcagccc gagccgccat gtcaagctgg 1508040
aagcgtggaa tgccattgat gcagacgatg tcattttatc attggataac agccctgaag 1508100
aaatcggagc aggtttaaag ttggcattga gccactgccg ataatatttg acaaaaggcc 1508160
gtctgaaaaa cagctttgac aaagacgcgg ttgccaaaga gatcgaccta caaagggaag 1508220
taacgcaggc gttcggcaaa acgccgccca agccgcagcg gccgttgccg acgaactcgg 1508280
caatacccga agttacggac ggtatcaggc agcccgaacc ctgccggagg ccgaactgca 1508340
aaacaaatcc gacacaaaaa accgtccgtc cgacacaaaa aaccgcccga aaaatctgga 1508400
cggcggttca aacaggctgc cccgtttaac gggcgcggca ggaagtttcg accgaattgc 1508460

cgtaggcatc ggtaaagccg aaaaaggctt cgccgccttt ctggtgccac tcggttgcgt 1508520
ttccgaacaa accgtgttcg gcggtatagc gttcgccgga tgcggcaacg tcggaagaga 1508580
ggacggcacg cctgccgtcc agccgcaacg cgactttgcc gctgtccaaa tggcggacgc 1508640
gcacagacaa accgttctcg caggaaaacg cccgaaaatc gtccgtgccg gcttggtttt 1508700
gaacgggcgg catatgcccg cgtccgccgt catcatacgc ctccggcacg gcacaggccg 1508760
ccaaagacaa aaccggtacg gtcagcgcga aaaacctgat attcataaaa gctccccaat 1508820
aaaaataaga tatgaaacaa ccgccctgat tccaagctgc ggcaacgcca tactataaac 1508880
ggacgcgcaa acacacaagc ccgataaccg gaatttacct gcgatgaatc aataatccgg 1508940
attgcgcgcc ccttctttac ccctcttccg atgccgcctt ttgcctgacg atgccgtctg 1509000
aacctgcctg cccgcccgga ggaatgtaaa ttttttccaa attccaagta aaaaccgcta 1509060
tcggtgtgct aatttgcgtt aaaatcctat tcggcgttta acgttttgtg cgcccgcatc 1509120
cctgcactgt ttgatgcggg cataaggcac aaatcccgac aagcgcactg tttcatactt 1509180
cgtcaatcat tcagactccg gtttgtgccc gtgccggcag atggttcggc cgtttcccgc 1509240
cgttcaggca tattccgaca gtgtgagata aggatttatt cgatgaaatc actcaaaacc 1509300
ttcctcattt ggggcatagt ggtactggtc ggcttagcat cctttaccac tctggccctc 1509360
agccgaggcg aacaggtcag cgcggtatgg atggtcaccg ccgccatatc cgtttactgc 1509420

atcgcctacc gtttttacag cctctacatc gccaaccgcg taatgcggct cgatcctgac 1509480
cgcctgactc cggcagaacg ccacaacgac ggcttggact acgttccgac gcacaaaggc 1509540
gtattgttcg gacaccactt tgccgcaatt gccggcgcgg gccctttggt tggtccggtt 1509600
ttggcggcgc aaatgggtta tctgcccggt actttgtgga ttatcttcgg cgtggtattt 1509660
gccggcgcgg tacaggatat gatggtcttg ttcgtctcta tgcgccgcga cggtaagtct 1509720
ttgggcgata ttgtgaaaca ggaactcggc actgtccccg gcgtgattgc ctccatcggt 1509780
attttgatga ttatggtcat cattatggcg gtgttggcgt tgattgtcgt aaaagcattg 1509840
gttcacagcc cttggggtac gttcaccatt gcagcaacta tgccgattgc gctgtttatg 1509900
ggtatttaca cgcgttatat ccgtccgggc aaaatcggcg agatttccat cgtcggcttt 1509960
atttgctga tgctggcggt aatttacggc gaagatgtgg ctaaaagttc catcgggcat 1510020
tggttcgacc ttgacggcat ccagctcact tgggcgatta tgatttacgg ctttgtcgcc 1510080
tccgtattgc ccgtatggtt gctgctcact ccgcgcgact atctctccac cttcctgaaa 1510140
atcggtacga ttgcggcctt ggctttgggt atcgtcatcg tcaatcccgc tttgcaaatg 1510200
cctgccgtaa cccactttat cgacggttcg ggtccggtat tctcaggcgc attgttccca 1510260
ttcttgttca ttaccatcgc ctgcggtgcg gtttcgggct tccacgcgct gatttcttcc 1510320
ggcactacgc cgaaaatgct ggaaaacgaa acccacgtcc gcatgatcgg ttacggcggt 1510380
atgttgatgg aaagtttcgt agccattatg gcacttgccg ctgccgcatc gcttgatccc 1510440
ggcgtgtact tcgccatgaa cagcccagcc gccctgatcg gtacggatgc caataccgcc 1510500
gccgaagtga ttaccaccaa gctgcaattc cctgtcgatg ccgcaaccct gttgcacact 1510560
gctaaagaag tcggcgaaaa caccatcctt tcccgtgccg gcggtgcgcc caccctcgca 1510620
gtcggtatgg cgcacattat gagccgcctg attccgggcg aggcgatgat ggcgttctgg 1510680
tatcacttcg ccctgttgtt tgaagccttg ttcatcctga ccgccgtcga tgccggtacg 1510740
cgcgtcgcac gttttatgat tcaagacttg ggcagcatct tctacaaacc tttcggcaac 1510800
accgactcca tccccgccaa cctgattgcg accttcttcg ccgtggcatt gtggggctac 1510860
ttcctctaca cgggcgtgac cgacccgttg ggcggcatca actcgctctg gcctttgttc 1510920
ggcatcgcca accaaatgct ggcaggcgta gccttgatta tgtgcgccgt ggtgctgatt 1510980
aagatgaaac gcgaccgtta tgtctgggtg gtactcgttc ccgccgtcgg cgtactgttc 1511040
gtaacctgct acgccggcct gcaaaaactg ttccacagcg acccgcgcat cagcttcctt 1511100
gcccacgccg gcaaatacag cgacgcattg gctaaaaacg aaatccttgc gcctgccaaa 1511160
gacatcggcg aaatggcgca aatcatcttc aacgacaaga ttaatgccgg tctgaccatc 1511220
ctcttcttgt cggttgtcgt gattgtcgcc gcgtacggtt tgcgtaccgc cctcaaagca 1511280
cgcaaagtcg gctggccgac cgccaaagaa atcccggcgg tgtaccgcga cggcaaacag 1511340
ccggaggcac aaagtgaagc ataagctcgc gtcttggtgg aaaaccatca agctgacggc 1511400
aaacttgatg gcaggcgtgc ccgattatga aaactacgtt gcacagcagc gcaaacataa 1511460
tcccaacgcc cccgtgatga ccaagctgca gtttcaagac tattgccgca aacgccgctg 1511520
cggcgcaaac ggcggacgct gctgttaagc ctgcttgaaa caaattccgt ctgaacgccg 1511580
cttcagacgg aatttttata atatagtgga ttaacaaaaa tcaggacaag gcgacgaagc 1511640
cgcagacagt acaaatagta cgaaaccgac tcacttggtg cttcagcacc ttagagaatc 1511700
gttctctttg agctaaggcg agacaacgcc gtactggttt ttgttaatcc gctataccac 1511760
gatgaatcct tcgcaatatc tgtttatcga cctcaatttt gacaaaatac cggatacgcg 1511820
cctttgttgc ttttccatct tccaaccaac tgtaaatctc aaacagccgg tacacgccat 1511880
gcttcagttt cttttcctgt cggcggattg tttcgacaaa gaattgaaaa tccatttcat 1511940
gcaccttaaa atttaatctg cattcaaacc ttttcacttt ggaagcacca tttatcggat 1512000
gtcccttcgc aataaacaaa ttttcccgat accgccgccc atttcaaccc aaacccaaaa 1512060

gctatgaaaa acctcatcgc cttcaacaaa ccctatggcg ttatctgcca attttcaccg 1512120
cacgaaaaac acaaaagcct caaagacttt atcaatcttc ccggcttcta ccccgccgga 1512180
cggctcgaca ccgacagcga ggggctgctg ctgctgaccg acgacggcag gcttcaggca 1512240
caaattaccg accccaaatt caaacaccct aaaacctact gggcgcaact ggagggcgta 1512300
cccgacgaaa gccgattgga aagcctaaga aaagggatag acttaggcgg tttcgttacc 1512360
cgtccggcaa gcatccgcat cttgaaacac ggagaagcag attcgttatg ggagcgcatc 1512420
ccgccgatac gcgtccgcaa aaccgttccc gatttttgga ttgaaattac catttctgag 1512480
ggcaaaaacc gccaagtcag gcgaatgacc gccaaggcgg gctatccctg cctgcgtctg 1512540
atcagagtgg caagcggcag gctgaaactg tttgatttgg atttaaaacc cggggaatgg 1512600
gcatacgccc cgtttaaacc ataatcacgt ttatctcatc atttccacaa aagtgggaat 1512660
ccggaatttt atagtggatt aacaaaaatc aggacaaggc gacgaagccg cagacagtac 1512720
agatagtacg gaaccgattc acttggtgct tcagcacctt agagaatcgt tctctttgag 1512780
ctaaggcgag gcaacgccgt actggttttt gttaatccgc tatattccgc catctctaag 1512840
atttacagcg atacacgggt gatttaagga atgcccgaac cgtcattccc gccacttttc 1512900
gtcattcccg cgcaggcggg aatctagaat ctcggacttt cagataatct ttgaatattg 1512960
ctgttgttct aaggtctaga ttcccgcctg cgcgggaatg acgaatccat ccgcacggaa 1513020
acctgcacca cgtcattcct acgaacctac atcccgtcat tcccacgaaa gtgggaatcc 1513080
agaacgtaaa atctgaagaa accgttttat ccgataagtt tccgtaccga acagactaga 1513140
ttcccgcctg cgcgggaatg acgattcata agtttcccga aattccaaca taaccgaaac 1513200
ttgacagtaa ccgtagcaac tgaaccgtca ttcccacgaa agtgggaatc tagaaatgaa 1513260
aagcaacagg catttatcgg aaataactga aaccgaaccg actagattcc cgcctgcgcg 1513320
ggaatgacgg ctgcagatgc ccgacggtct ttatagcgga ttaataaaaa tcaggacaag 1513380
gcggcgagcc acagacagta caaacagtac ggaaccgatt cacttggtgc ttcagcacct 1513440
tagagaatcg ttctctttga gctaaagcga gacaacgctg tactggtttt tgttaatcca 1513500
ctataaatat ccaattgaaa tcttcagacg gtatatcaaa tttacacttt tttaatgttt 1513560
atgccgcctg aaaaaaatgc tagtatattt cctaattgtc tgactgttta ttgttgagga 1513620
aaatatgaga tcttctttcc ggttgaagcc gatttgtttt taccttatgg gtgttacgct 1513680
atatcattat agttatgccg aagatgcagg gcgcgcgggc agcgaggcgc agatacaggt 1513740
tttggaagat gtgcacgtca aggcgaagcg cgtaccgaaa gacaaaaaag tgtttaccga 1513800
tgcgcgtgcc gtatcgaccc gtcaggatat attcaaatcc agcgaaaacc tcgacaacat 1513860
cgtacgcagc atccccggtg cgtttacaca gcaagataaa agctcgggca ttgtgtcttt 1513920
gaatattcgc ggcgacagcg ggttcgggcg ggtcaatacg atggtggacg gcatcacgca 1513980
gacctttttat tcgacttcta ccgatgcggg cagggcaggc ggttcatctc aattcggtgc 1514040
atctgtcgac agcaatttta ttgccggact ggatgtcgtc aaaggcagct tcagcggctc 1514100
ggcaggcatc aacagccttg ccggttcggc gaatctgcgg actttaggcg tggatgacgt 1514160
cgttcagggc aataatacct acggcctgct gctaaaaggt ctgaccggca ccaattcaac 1514220
caaaggtaat gcgatggcgg cgataggtgc gcgcaaatgg ctggaaagcg gagcatctgt 1514280
cggtgtgctt tacgggcaca gcaggcgcag cgtggcgcaa aattaccgcg tgggcggcgg 1514340
cgggcagcac atcggaaatt ttggcgcgga atatttggaa cggcgcaagc agcgatattt 1514400
tgtacaagag ggtgctttga aattcaattc cgacagcgga aaatgggagc gggatttaca 1514460
aaggcaacag tggaaataca agccgtataa aaattacaac aaccaagaac tacaaaaata 1514520
catcgaagag catgacaaaa gctggcggga aaacctggca ccgcaatacg acattacccc 1514580
catcgatccg tccagcctga agcagcagtc ggcaggcaat ctgtttaaat tggaatacga 1514640
cggcgtattc aataaataca cggcgcaatt tcgcgattta aacaccaaaa tcggcagccg 1514700
caaaatcatc aaccgcaatt atcagttcaa ttacggtttg tctttgaacc cgtataccaa 1514760
cctcaatctg accgcagcct acaattcggg caggcagaaa tatccgaaag ggtcgaagtt 1514820
tacaggctgg gggcttttaa aggattttga aacctacaac aacgcgaaaa tcctcgacct 1514880
caacaacacc gccaccttcc ggctgccccg cgaaaccgag ttgcaaacca ctttgggctt 1514940
caattatttc cacaacgaat acggcaaaaa ccgctttcct gaagaattgg ggctgttttt 1515000
cgacggtcct gatcaggaca acgggcttta ttcctatttg gggcggttta agggcgataa 1515060
agggctgctg ccccaaaaat caaccattgt ccaaccggcc ggcagccaat atttcaacac 1515120
gttctacttc gatgccgcgc tcaaaaaaga catttaccgc ttaaactaca gcaccaatac 1515180
cgtcggctac cgtttcggcg gcgaatatac gggctattac ggctcggatg acgaatttaa 1515240
gcgggcattc ggagaaaact cgccgacata caagaaacat tgcaaccgga gctgcgggat 1515300
ttatgaaccc gtattgaaaa aatacggcaa aaagcgcgcc aacaaccatt cggtcagcat 1515360
tagtgcggac ttcggcgatt atttcatgcc gttcgccagc tattcgcgca cacaccgtat 1515420
gcccaacatc caagaaatgt atttttccca aatcggcgac tccggcgttc acaccgcctt 1515480
aaaaccagag cgcgcaaaca cttggcaatt tggcttcaat acctataaaa aaggattgtt 1515540
aaaacaagat gatacattag gattaaaact ggtcggctac cgcagccgca tcgacaacta 1515600
catccacaac gtttacggga aatggtggga tttgaacggg gatattccga gctgggtcag 1515660
cagcaccggg cttgcctaca ccatccaaca tcgcaatttc aaagacaaag tgcacaaaca 1515720

cggttttgag ttggagctga attacgatta tgggcgtttt ttcaccaacc tttcttacgc 1515780
ctatcaaaaa agcacgcaac cgaccaactt cagcgatgcg agcgaatcgc ccaacaatgc 1515840
gtccaaagaa gaccaactca aacaaggtta tgggttgagc agggtttccg ccctgccgcg 1515900
agattacgga cgtttggaag tcggtacgcg ctggttgggc aacaaactga ctttgggcgg 1515960
cgcgatgcgc tatttcggca agagcatccg cgcgacggct gaagaacgct atatcgacgg 1516020
caccaacggg ggaaatacca gcaatttccg gcaactgggc aagcgttcca tcaaacaaac 1516080
cgaaactctt gcccgccagc ctttgatttt tgatttttac gccgcttacg agccgaagaa 1516140
aaaccttatt ttccgcgccg aagtcaaaaa tctgttcgac aggcgttata tcgatccgct 1516200
cgatgcgggc aatgatgcgg caacgcagcg ttattacagc tcgttcgacc cgaaagacaa 1516260
ggacgaagac gtaacgtgta atgctgataa aacgttgtgc aacggcaaat acggcggcac 1516320
aagcaaaagc gtattgacca attttgcacg cggacgcacc tttttgatga cgatgagcta 1516380
caagttttaa aggcagcccg cattttgtag aaaaccgcaa tgccgtctga aagcccttca 1516440
gacggcattt gtttccccaa acgcatcatc ctgccgcaag cctatgccaa tccgttttat 1516500
cgcatcggca actcaaagaa aaatccattt cattcccacg cagggaagcc ggtttttgat 1516560
ttcggttatt tttggttgtt tcgggtaatt tatgagtcgt cattcccgca aaagcgggaa 1516620
tcagtttttt taagtttcag ccatttccga taaattcctg tggctttagc tttccggatt 1516680
cccactttcg tgagaatgac gtggtgcagg tttccgtacg gatggattcg tcattcccgc 1516740
gcaggcggga atctagaccg ttcggtttcg gtttttttgg ttagtgccgc aacattaaat 1516800
ttctagattc ccactttcgt gggaatgacg gcggagcggt ttctgctttt tccaataaat 1516860
gcccccaacc taaaatccgt cattcccgcg caggcgggaa tctagacatt caatgctaag 1516920
gcaatttatc ggaaatgact gaaactcaaa aaactagatt cccactttcg tgggaatgac 1516980
gtggtgcagg tttccgtatg gatggattcg tcattcccgc gcaggcggga atctagtccg 1517040
ttcggtttcg gtttttttgg ctaatgccgc aacattaaat ttctagattc ccactttcgt 1517100
gggaatgacg gcggagcggt tgctgttttt cccaataaat gcccccccaac ctaaaatccg 1517160
tcattcccgc gcaggcggga atctagtccg ttcggtttcg gtttttttgg ctagtgccgc 1517220
aacattaaat ttctagattc ccactttcgt gggaatgacg gcggagcggt ttctgctttt 1517280
cccaataaat gcccccaacc taaaatccgt cattcccgcg caggcgggaa tttagacatt 1517340
caacgctaag gcaatttatc ggaaatgact gaaactcaaa aaactggatt ccctctttcg 1517400
tgggaatgac gtagtgcagg tttccgtacg gatggattcg tcattcccgc gcaggcggga 1517460
atctagacat tcaatgctaa ggcaatttat cggaaatgac tgaaactcaa aaaactggat 1517520
tcccgctttc gtgggaatga cgcgattaga gtttcaaaat ttattctaaa tagctgaaac 1517580
tcaacgcact ggattcccgc ctgagcggga atgacgaagt ggaagttacc cgaaacttaa 1517640
aacaagcgaa accgaacgaa ctggattccc actttcgtgg gaatgacgga atgtaggttc 1517700
gtgggaatga cgggatgcag gtttccgatg gatggattcg tcattcccgc gcaggcggga 1517760
atctagacat tcaacgctaa ggcaatttat cggaaatgac tgaaactcaa aaaactggat 1517820
tcccactttt gtgggaatga cgcgattaga gtttcaaaat ttattctaaa tagctgaaac 1517880
tcaacgcact ggattcccgc ctgagcggga atgacgaatt tcaggttgct gtttttggtt 1517940
ttctgttttt gtgaaaataa tgggatttta gcttgtgggt atttaccgga aaaaacagaa 1518000
accgctccgc cgtcattccc gcgcaggcgg gaatctagtc cgttcggttt cggttttttt 1518060
ggctagtgcc gcaacattaa atttctagat tcccactttc gtgggaatga cgggatgtat 1518120
agtggattaa caaaaaccag tacggcgttg cctcgcctta gctcaaagag aacgattgtc 1518180
taaggtgctg aagcaccaag tgaatcggtt ccgtactatt tgtactgtct gcggcttcgt 1518240
cgccttgtcc tgatttttgt taatccacta taaatttaat ccactatatt ttttgttcca 1518300
aagtcaaaat atgccgtccg aacattcggg cggcagacaa aacggcactg cccgataaag 1518360
gcagtgccgt tgtccgtttc aaaccgtgaa acatcagccc aaattaaagg ctttatgcaa 1518420
taccctggtt gccagttcca tgtatttttc atcaatcaat acggaaactt tgatttcgga 1518480
ggtggaaatc atttggatgt tgataccctc ttcggcgagc gtgcggaaga ttttggcggc 1518540
tacaccgacg tgcgaacgca tacccaaacc gactgcggag actttgcata cggtgtcgtc 1518600
gccatcaata gaagccgcgc cgatactgtc ttggcgttcc gacaggattt ccaaagtctg 1518660
cttgtaatcg ccgcgcggta cggtaaagga aaaatcggtt gtgccttcgc tgccgacatt 1518720
ttggataatc atatcgactt cgatgttggc atcggcaacc gcgcctaaaa tctgataggc 1518780
gacgccaggt ttgtcgggta cgccgcgcac gttgatgcgg gcttggtttt tatcgaatgc 1518840
gataccggtt acggcagctc tttccatgtt gtcgtcctct tcaaaggtaa ttaaggtgcc 1518900
attgccgccg tcttgcaggc tgctcagtac gcgcaggcgc actttgtatt ttccggcgaa 1518960
ttctactgaa cggatttgca aaactttcga accgaggctt gccagttcga tcatttcttc 1519020
aaatgtaacc gtatccatgc ggcgcgcttc gggtacgacg cgggggtcgg ttgtgtaaac 1519080
gccgtctacg tcggtataga tttggcactc gtcggctttg agcgcggcgg caagcgcgac 1519140
ggcggaagtg tcggaaccgc cgcgtccgag cgtggaaata tcgccttcac tgctgatgcc 1519200
ttggaagccg gcaacgatga cgactttgcc ggcggtaagg tcggcacgca ttttttcgtc 1519260
atcaatgctt tcgatgcggg ctttggtgtg ggcggtatcg gttttgaggg cgacctgcca 1519320
gcctgtgtag cttttggcat ccacgccgat gtctttcaat gccatcgcca aaaggccgat 1519380

ggttacttgt tcgccggtag ctaagacgac gtccagctcg cgcggatcgg gatgctcttg 1519440
catttcgtgc gccagtgcga ccagtcggtt ggtttcgccg ctcatggcgg atacgacgac 1519500
tacgatgtcg tgtccttcgg cgcgggcttt ggcgacacgt ttggctacgt ttttgatgcg 1519560
ttcgggcgag cctactgatg tgccgccgta tttatgtacg attaacgcca tgtttcgtgc 1519620
tttcttgtgg gggttgtcgg gcagcttggt ttgctggaaa aagggttatt attactattt 1519680
tttacatgga attcaagaac ggactgcgct ttcccgcctg ccgtttgaca gcggtcagcg 1519740
aaaaacctgt tctttcagat tgttgacaaa atgccgtctg aacggttttc agacggcatc 1519800
cggacgacaa tcaggcggcg gacaacgcat tttgctggtg ttgcagcagt tcgcctatgc 1519860
ctttttgcgc cagtgcaacc agtttgccca attcgtccaa actgaacggc gcgtcttccg 1519920
ccgtcccctg tatttcgatg atttttcccg atgcggtcat gacgatattc acatcactgt 1519980
cgcaaccgga gtcttcggga taatccaaat ccaaaagcgg cacgccgttc actacgccta 1520040
ctgacacagc ggcaacggct tcgcggatgg ggttttcact caaaatgccg tctgaaacca 1520100
gtttgccgac ggcgatttgc agcgcgacaa acgcaccggt aatcgaagcc gtgcgcgtac 1520160
cgccgtctgc ctgaatcaca tcgcagtcaa tcaagatttg tcgttcaccg agtttttcca 1520220
tatccacgac cgcgcgcagg gaacgcccga tcaaacgttg gatttcttgt gtgcgcccgg 1520280
actgtttgcc cgccgaagct tcgcggagca tccgggaagc agttgaggca ggcagcatcc 1520340
cgtattccgc cgttacccag ccttggtttt taccgcgcag aaacggcggg acgttttcat 1520400
ctatggaagc ggtacaaatc actttggtat tgccgcattc aataaggcac gaaccgtccg 1520460
tatgcggcag gaaatgaggg gtgattttga tatcgcgcag gctgtcggcg gcgcgcgaga 1520520
tgcggatgta atcaggcata ctgccctccc gttaaaaaca gataaattaa aaagccttaa 1520580
atatgaaaaa tcacatttaa ggccttcaaa ctgaaaattt ctacgcctct tcggctttgc 1520640
tgcggataat caaaagcggc aggtggcttt ggcgcattac cgtttcggca aaactgccca 1520700
ttaaaaggtg catcagcccg gtacgtccgt gcgtacccaa caccagcagg tcggcaccgt 1520760
tttcatcggc ataatcaacc aaatcctgcg ccatttcacg cgcaccctta ttggcaacca 1520820
gcaggtgttt gacggtattt tccacaccca gttcctgggc ggtgcgctcg gcggcatcca 1520880
aaacttcgtt gccttgcgcg acggcggcgg cttcgtagct ttcgtgttgc aaaaattcgg 1520940
gggcgagtgc catatattcg gcaggattgg caacgtgcac caaagtcagg cgcgcaccgt 1521000
tgaccccggc aagctcggcg gcatgtttca gggcattgat ggacgtttca ctgccgtcaa 1521060
cggcaacaac caaatgtttg tacatatcgt attctccttt tgcaccgcct cgcggtgccc 1521120
tcttgtcgga tgggcgcagg gacagtttgc gctgtttcat tatagacccg ccgtcgggct 1521180
ttatacaaca gccgaacagc ccgaccgctt tccagtataa tatgccgctt ccgtgcagtc 1521240
aggcattttt tgccggcttt cgttcacttt ttgatttgac gcaatcttgc aggattcgac 1521300
catgtccgac aacgctttga cctcttcgcg acgcttcggc ggcatcgcca gactctacgg 1521360
agactctgcc ttggcgcact tttcacaggc acacgtctgc gtagtcggcg tgggcggtgt 1521420
cggctcgtgg gcggtcgagg ctttggcgcg gacgggcatc ggacgtttga ctttgattga 1521480
tttggacaac gttgccgaat cgaatgtcaa ccgccagctg cacgccctga ccggcgactt 1521540
cggcaaagca aaagttaccg ccttgcgcga acgcattaca caaattaatc cgcaatgcga 1521600
agtgtttgaa attgaagatt tcgttaccga agacaatttg ccggaatact tcggaaaagg 1521660
ttttgatttc gtcatcgacg cgatcgacca agtgcgcgtc aaagcagcaa tggcggctta 1521720
ttttgtggaa cgcaaacaac cgtttgtcct cagcggcggc gcgggcggac aaaaaaatcc 1521780
ggcgttaatc caaaccgccg atttgagccg cgtaacccac gacccgctgc ttgccaacct 1521840
gcgctacacc ttgcggaaac gctacggatt cagccgcgat acgaaagcaa atatgcgcgt 1521900
gccttgcgtg tattcgaccg aaaatatcgt gccgccgcag tctagggagg cttgttcggc 1521960
agatgccgct ccgcaaggct tgtcgtgcgc cggctacggt gcaagcatgc tcgttaccgc 1522020
ttcgttcggg ctatattgcg cacaggcggc ggtggaacac atcgcagaca aaaaataagc 1522080
aatgccgtct gaaacaggat tcagacggca tttgaacaaa ctatggttat gatttaagac 1522140
aacaaaggat acggataaaa aataacataa aatatatgat tcctaataat ataccaagta 1522200
tcggagagct atttaatgga attcgttaat aatttagtta ttttttcatt tttattacta 1522260
atgcttattc cgatattttt tgtagtatat ggtatatacc ataagatacg ttatcgcaaa 1522320
atatgtatcc taagaacaag ttttatatta ttagtggtaa tactttgcag tatgtattac 1522380
atatattgcc gttatcttga ccaacaaaaa gtagcttatt attgcataga tgaacaatgt 1522440
atttctattg ttcatctata caaagattat ggtataaact ctcccacata tgcgagaatt 1522500
tacgcaggaa aaatattgtt tagatttcaa gtaagagcta aaaattacgc tgaattactt 1522560
atggaagatg atatatcaat tagtaaaaaa attttgggga ataaatttat catttatggg 1522620
tcgctacctg taatatacgg taatgtagat aatattgaag taaaagaagc tactggttat 1522680
atagatagat ccagtactga ttatattgtc tcaagaaact taaaattcag acatttatat 1522740
taattaagag gttttagcaa gagtgccgtc aaaatatagg gcgcatcatc gaattcgcga 1522800
aagacaaacg ctacgatgaa cgtttcaagg atttgaaaaa agaatccata ggctatctga 1522860
accggcatcc cggtttggtg tccgactacc tgaaggcggc aatcaagctg tcggttcaga 1522920
aaaaccaaca tcagcacgcc taaaaccgta ttcacaacct gctccttttc aaaacatttg 1522980
catttaaaag ccgttataat gccgtctgaa catctgcccg accacattat acgtgaatgt 1523040

cggcagattg ttttcttttg taaacttata ttaaaatcca cttaccgatt cacgccatgc 1523100
cgcccatccc tgccccatct gcaccatccg agcacactgt cgcatgggta ttcggccaac 1523160
ccgttaccga tttgccccag gatttgttta ttccgcccga tgcattgaaa gtcgtattgg 1523220
gcagcttcca aggccctttg gatctactgc tgtatctgat ccgcaaacag aatatcgacg 1523280
tactggatat tccgatggtg aagattaccg agcagtatct gcactacatc gcccaaatag 1523340
aaacctatca gtttgatttg gcggcggaat atcttttgat ggcagcaatg ctgattgaaa 1523400
tcaaatcgcg cctgctgctg ccgcgtaccg aaaccgtcga agacgaagaa gccgacccgc 1523460
gtgccgagtt ggtgcgccgc ctgctggctt acgaacagat gaagctggcg gcgcagggtt 1523520
tggacgcgct gccccgagcc ggacgggatt tcgcgtgggc ttacctgccg ctggaaattg 1523580
ccgtcgaagc caagctgccc gaagtctata ttaccgactt gacgcaagcg tggctgggta 1523640
ttttgtctcg ggcaaaacac acgcgcagcc acgaagtaat caaagaaacc atctccgtgc 1523700
gcgcgcaaat gacggcaatc ctgcgccgtt tgaacggaca cggaatatgc aggtttcacg 1523760
acctgttcaa tcccaaacag ggcgcggctt acgtggtcgt caacttcatc gcactgttgg 1523820
agcttgccaa agaaggattg gtcagaatcg tgcaggaaga cggtttcgga gaaatccgaa 1523880
tcagcctcaa tcatgagggg gcgcattcag acggcatttc cggcacacga ggcgggcgcg 1523940
atgtgttcta atacgcccca agccgccacc aaaaatcggg agacacgcca tatgaccggc 1524000
atcatacatt cgctgcttga caccgacctc tacaaattca ctatgctgca agtggttctg 1524060
caccagtttc cgcagacgca cagcctttac gaattccgct gccgcaacgc ctcgaccgtc 1524120
tatccgcttg ccgacatcag ggaagacttg gaagccgaac tcgacgcgct ctgccaacta 1524180
cgcttcaccc acgacgaact cggctatctg cgctccctgc gtttcattaa aagcgacttt 1524240
gtcgattatc tcgaactctt ccagctccaa cgccgctttg tcgaaatcgg cacagacgat 1524300
aaagaccgtc tgaacatccg catcgaaggt ccgatgatac aggcgatgtt ttttgaaatc 1524360
ttcatcctcg ccattgtcaa cgaactttac ttccgccgcc tggaaacccc tgcagtcata 1524420
gaagaaggcg aacgccggct tcaagccaaa gccgcgcgcc tcaaagaaat cgccgccgca 1524480
caaaaccccg acgaaccgcc cttcctgatt tccgacttcg gcacgcgccg ccgctacaag 1524540
ctcgcgtggc aggaacacgt catccgcacc ctgcttgaag ccgcccccgg catcgtacgc 1524600
ggcaccagca atgtctttct cgccaaaaaa ctcggcatca cccccatcgg caccatggcg 1524660
cacgagttcc tgcaggcatt ccaggccctc gacgtacgcc tgcggaattt ccaaaaggcc 1524720
gcgctcgaaa gctgggtgca cgaataccgg ggcgatttgg gcgttgccct gaccgacgtg 1524780
gtcggtatgg atgccttcct gcgcgatttc gacctctatt tcgccaaact tttcgacggg 1524840
ctgcgccacg acagcggcga cccttacgtt tggggcgaca aagcctacgc ccactatcaa 1524900
aagctcaaaa tcgacagccg caccaaaatg ctgaccttct ccgacgggct ggacatcgaa 1524960
cgctcttggg cattgcacca atatttcaaa gaccgcttca aaaccggctt cggcatcggc 1525020
accaacctca ccaacgatat ggggcatacg cccttgaata tcgtcttgaa actggtcgaa 1525080
tgcaacgggc agtccgtcgc caagctgtcc gactctccgg gcaaaaccat gaccaacaac 1525140
agcaccttcc tcgcctacct gcgccaagtg ttcgacgtac ccgaacccga aacgccgtaa 1525200
accggcagaa aaagcgcaca attcctgttt ctgccgcata aaatctttta aaataccgcc 1525260
tgatttgaat ttaaccgaaa gaccgaactt catgaaccta catcaaaccg tcgaacacga 1525320
agccgccgcc gcctttgccg ccgcaggcat cgccgacagc cctattgttt tgcagccgac 1525380
caaaaacgcc gaacacggcg atttccaaat caacggcgtg atgggtgcgg cgaaaaaagc 1525440
caaacaaaac ccgcgcgagt tggcgcaaaa ggtcgccgaa gcattggcgg acaacgccgt 1525500
gattgaaagc gcggaagtcg ccggtccggg cttcatcaac ctgcgcctgc gccccgaatt 1525560
tctcgcgcaa aacattcaga cggccttgaa cgacgctcgt ttcggcgtgg caaaaaccga 1525620
caaaccgcaa accgtcgtta tcgactattc ttcgcccaat ctggcgaagg aaatgcacgt 1525680
cggccacctg cgttccagca tcatcggcga cagcatttcg cgcgtgttgg catttatggg 1525740
caataccgtt atccgtcaaa accacgtcgg cgactggggt acgcagttcg gtatgttggt 1525800
cgcttatttg gtcgagcagc aaaaagacaa tgccgcgttc gagctggcgg atttggagca 1525860
gttttaccgc gccgccaaag tgcgctttga cgaagaccct gcctttgccg acaccgcacg 1525920
cgaatacgtt gtgaagctgc aaggcggcga tgaaaccgtt ttggcattgt ggaaacagtt 1525980
tgtcgatatt tcgctctcgc acgcccaagc cgtttacgac acgctgggct tgaagctgcg 1526040
tcctgaagac gtggcaggcg aatcgaaata caacgacgat ttgcagcccg tggtcgatga 1526100
tttggttcaa aaaggtctgg cggttgagga cgacggcgcg aaagtcgtgt tcttggacga 1526160
atttaaaaac aaagaaggcg aacccgccgc atttatcgtg caaaaacaag gcggcggctt 1526220
cctctacgcc tccaccgatt tggcgtgcct gcgctaccgc ataggccgtc tgaaagccga 1526280
ccgcctgctg tacgtcgtcg accaccgcca agccctgcac ttcgaacaac ttttcaccac 1526340
ttcccgcaaa gcaggctatc tgccggaaaa cgtcggcgcg gcatttatcg gcttcggcac 1526400
catgatgggc aaagacggca agccgttcaa aacgcgcagc ggcgacaccg tgaaactggt 1526460
cgatctgctg accgaagccg tcgagcgcgc caccgctttg gtgaaagaaa aaaatcccga 1526520
attgggtgcg gacgaagccg ctaaaatcgg taaaaccgtc ggcatcggcg cagtcaaata 1526580
cgccgacttg agcaaaaacc gcaccagcga ctatgtgttc gactggggatg ccatgctctc 1526640
gtttgaaggc aacaccgccc cctatctgca atacgcctac acccgcgtgc aaagcgtgtt 1526700

ccgcaaagca ggcgaatggg atgcaaatgc gccaaccgtt ttgaccgaac cgctggaaaa 1526760
acagcttgcc gccgagctgc tgaaatttga agacgtactg caaagcgtgg cggacacggc 1526820
gtatccgcac tacctcgccg cctacctcta tcaaattgcg accctgttca gccgcttcta 1526880
cgaagcctgt ccgatactca aagccgaagg cgcaagccgc aacagccgcc tgcaactggc 1526940
aaaactcacc ggcgacacgc tgaaacaagg cttggatttg ctgggcatcg atgtgttgga 1527000
cgtaatgtaa aaccgcaccg cccgattgcg gacaacagcc tcgccatcct tatccgaatc 1527060
tgaaaaaagc ggcgcgatac accgtatccg ccgcccctcc caaaatgcga aacaaacaaa 1527120
cgccaagcaa gcaagcaagc aagcaagcaa gcaagcaagc aagcaagcaa aaaattataa 1527180
ccccccttcct gccgacgcac gcactttccg cgcggcgcat tccccttttc ccgcccctca 1527240
aatccgcctt ttcttcaggc agggtttcag cccgcctctt ttccctgttt tcctttcccc 1527300
gacacgcgtg cgctccccct gccgcactgt gctgcacttt cgcgcccgga cggcatcgtt 1527360
ccgccatccg gttctctgtt ttacataccc ctgtttcaga aagaaatgca gatgtttcaa 1527420
cacacaggac gacacataaa gcaccgccct atgtgttgcc ctgatttgga aggggttacg 1527480
cctcccaaat aaagtctgat cctgccgccc cgaaggacag atgtccgagt ggcgaagttt 1527540
caaccgaaaa ggaaatacga tgaatattca caccctgctc tccaaacaat ggacgctgcc 1527600
gccattcctg ccgaaacggc tgctgctgtc cctgctgata ctgcttgccc ccaatgcggt 1527660
gttttgggtt ttggcactgc tgaccgccac cgcccgcccg attgtcaatt tggactatct 1527720
tcccgccgcg ctgctgatcg ccctgccttg gcgtttcgtc aaaattgccg gcgtattggc 1527780
gttttggctg gcggttttgt ttgacgggct gatgatggtg atccaactct tccctttttat 1527840
ggatctcatc ggcgccatca acctcgtccc cttcatcctg accgcccccg ccccttatca 1527900
gataatgacc gggctgttgc tgctgtatat gctggcgatg ccgtttgtgt tgcagaaagc 1527960
cgccgccaaa accgacttcc ggcacattgc cgtctgcgcc gccgttgtgg cggcagccgg 1528020
ctatttcacc ggccatttga gttactacga ccggggtcgg atggccaata tcttcggcgc 1528080
aaacaacttc tactacgcca aaagtcaggc gatgctctac accgtcagcc agaatgccga 1528140
ctttattacc gccggcctgg tcgatcccgt cttcctcccc ttgggcaatc aacagcgtgc 1528200
cgccacgcat ctgaacgagc cgaaatctca aaaaatcctc tttatcgtcg ccgaatcttg 1528260
ggggctgccg gccaatcccg aacttcaaaa cgccactttt gccaaactgc tggcgcaaaa 1528320
agaccgtttt tcggtttggg aaagcggcag ttttcccttc atcggcgcga cggtcgaagg 1528380
cgaaatgcgc gaactgtgtg cctacggcgg tttgcgcggg ttcgcactgc gccgcgcgcc 1528440
cgacgaaaaa tttgcccgct gcctccccaa ccgtttgaaa caagaaggtt acgccacctt 1528500
tgcgatgcac ggcgcgggca gttcgcttta cgaccgcttc agctggtatc cgagggcggg 1528560
ctttcaagaa atcaaaaccg ccgaaaacct gatcggtaaa aaaacctgcg ccattttcgg 1528620
cggcgtgtgc gacagcgagc tgttcggcga agtgtcggca tttttcaaaa aacacgacaa 1528680
gggactgttt tactggatga cgctgaccag ccacgccgac tatcccgaat ccgacatttt 1528740
caaccacagg ctcaaatgca ccgaatatgg cctgcccgcc gaaaccgacc tctgccgcaa 1528800
tttcagcctg cacacccaat tcttcgacca actggcggat ttgatccaac gccccgaaat 1528860
gaaaggcacg gaagtcatca tcgtcggcga ccatccgccg cccgtcggca acctcaatga 1528920
aaccttccgc tacctcaaac aggggcacgt cgcctggctg aacttcaaaa tcaaataaca 1528980
acaatgccgt ctgaacgcac caacagcctt cagacggcat tttgcagaca gaccgaccct 1529040
tcaagcccac ttttttcatc atctccgata aattgctttg tatagtggat taacaaaaac 1529100
cagtacggcg ttgcctcgcc ttagctcaaa gagaacgatt ctctaaggta ctgaagcacc 1529160
aagtgaatcg gttccgtact atttgtactg tctgcggctt cgtcgccttg tcctgatttt 1529220
tgttaatccg ccataaagac cgtcgggcat ctgcagccgt cattcccgcg caggcgggaa 1529280
tccagaacgt ggaatctaaa gaaaccgttt tacccgataa gtttccgcac cgacagacct 1529340
agattcccgc ctgcgcggga atgacgggat tttaggtttc tgattttggt tttctgtcct 1529400
tgtgggaatg acgggatgta ggttcatagg aatgacgtgg tgcaggtttc cgtatggatg 1529460
gattcgtcgt tcccgcgaaa gcgggaatcc ggaaacccaa agccacggga atttatcgga 1529520
aaaaccgaaa ccgctccgcc gtcattcccg cgcaggcggg aatctaggtc tgtcggtgcg 1529580
gaaacttatc ggataaaacg gtttcttcag attttacgtt ctggattccc actttcgtgg 1529640
gaatgacggg atgtaggttc gtaggaatga cgtggtgcag gtttccgtat ggatgggatt 1529700
ccctcttgcg tgaggctgac agatgccgtc tgaaagactt tcagacggca tagctttttc 1529760
tctttgaatt tatagtggat taacaaaaat caggacaagg cggcgagccg cagacagtac 1529820
agatagtacg gaaccgattc actcggtgct tcagcacctt agagaatcgt tctctttgag 1529880
ctaaggcgag gcaacgccgt actggttttt gttaatccac gataaatttg ccacaaaaaa 1529940
gctgcctcaa atgaataccc gggcagcttt ttgttgatat gactccaatc agcggtgttg 1530000
cggattgtaa cgtttttcca aacgcaggaa tatccagcct aagaaagtcg tcatcaacag 1530060
ataaatcagg gcgacggtgt aaagcggttc ttcataaacc gaataccggc ccgtaatcgt 1530120
attctgaaca tacgccaact ccgccacagc aatgaccgac agcagcgagc tgtctttcaa 1530180
gagcgtgatg aactcgctcg ccaaaggcgg cagcatgcgg cgcaatgcct gcggcagaat 1530240
cacatagcgc atcgcctgcg gataggtcag ccccaaagaa cgcgccgcct ccatctgtcc 1530300
tttgtctata gactggatgc ccgcgcggaa aatctcacag atatacgccc ccgagttggc 1530360

gatcagtgcc aaagaaccgg caatcagcgg cccgtatccg cgacgcagcg cgattgccgc 1530420
ctcgccgctg accaaaatgc cgtctgaagg atggacgaaa aacggaaacc acacatacgc 1530480
ccaaatcaca atctgcacaa acagcggcgt accccggaac agcgtaacat acagcagcga 1530540
aactttacgc aacgcccacg ccagcacgcg catcggcgca ccggcttttt ccaagtgaat 1530600
caggcgcgcc aacgccaaca acagacccaa taccgaaccg cccgccgttg ccacgaccgt 1530660
cagccccaag gtcgtcagtg cgccgtaaag aaacatccag cggtattcgt aaataatgtc 1530720
aaaacgaaaa tccataaacc gtccgtatca aaaaccggcg gaactgccgc cgttgcaaaa 1530780
taatccgcca ttttaccgta aaaaccgccg cctgaacttt tttatcgcgg cagacggcgg 1530840
ttgcgcgtct ccgcaaaaat gcagggcgcg cggttttcag acggcatttg ccgttcaaag 1530900
ccgtgcggtg tctttaccaa atgcccaacc attcgcccac ggcatccatc caatccttat 1530960
tgccccgcc gctcctgcct gctcggcggt acgcccacgg cgcttgcgga tttttagctt 1531020
tccacaatcc tttgcgttcc ctttccgcct gaatttgagc gtcggcataa tcggcaaaat 1531080
ccgccttatc ctgctgttct ttagcataac ttttataatg ccacgccgcc ccgtcctgca 1531140
cctgcatcag gttcaaatcg gttttgccga cagaaacctg cgccacttcg cgctggtagc 1531200
ggtcggtatc gaacacgcgc acgctgactt tcctgccttc cgccgccgcg cgcaggttgt 1531260
cgcgcgaacg cgtgccgtaa gcctgtttca tctccggcgc gtcgatatac gccatccgga 1531320
ttttgtgttt cgcgccgtcg ccgtcgataa cgtgaagggt gtcgccgtca tagactttgg 1531380
acaccgtgcc tgtgtagcgg tggccggatt tcgccgatgc tcggcggcgg gcgggcgcgt 1531440
cggaacccgc gtcccctgcc gcgccgagta cgtcgagtac ggcaaccgcc gtccgcaccg 1531500
cctcgctgcc gtaccccgta taacccaacg cacccaaaag cgacagggcg acgggaagcc 1531560
atttcatgat ttttttaatc tgcatatttt tcaaatgccg atgccgtctg aacatatcgg 1531620
aatcggattt cagacggcat cttaacgtca ggattaccct tggcagggat agatgacttt 1531680
cgcaccctct tccgtcccca aaatcaacac atcggcggca tcgcgggcga atatgccgtt 1531740
ttcgagcacg ccggtgattt tgttgatttc gtcttccatc gtcagcggct gatcgatatt 1531800
caagccgtgg acatcgacga tttggttgcc gtaaaacgtg gtgtagccga tacgcagttc 1531860
gggctgtccg cccatagcga gcagtttgcg cgaaacaaga gagcgcgcgc tttcgacgac 1531920
ttccacaggc agagggaatt tgcccaaacg tgaaacatat ttgctttcat ccgcaatgca 1531980
gatgaatttt tcggacgcgc tggcgacgat tttttcgttg aggtgcgcgc cgccaccgcc 1532040
tttaatcatt tgcagggcgt ggttcacttc atccgcaccg tcgatataga ccgccaaccc 1532100
cgatacttcg ttcaaagaaa cgacgggaat atcgtactgg gcaagcagtt cgccggattt 1532160
tttggaagta gataccgcgc ctttgatttt tttgccgctc ttacccaagg cttcgatgaa 1532220
aaagttgatg gtcgagccgg taccgatgcc gatatattca ttttcgggta cgaattcgac 1532280
tgctttttcg gcggcgatgc gcttgagttc gtcttgtgtc gtcatatttt tgtcctttgg 1532340
gaaaccgtat caacaaacag ccgccatctt aacatttttt tgcacgtcct gcccgccgcg 1532400
ttcaaatgcg taccagcaat accgccgcct gcgcctctat gccttccatc cgcccgagat 1532460
agccgagttt ttcgttggtt ttgcctttga tgttgacgca cgaaatgtct atgcccaaat 1532520
cggcggcgat gttggcacgc atttgcggaa tgtgcggcgc gagtttgggt ttctgtgcaa 1532580
tcacggtcgt atcgacattg accgcctgcc aaccctgcgc ctgaacgctt tgatacgccg 1532640
cacgcaaaag gacgcggctg tccgcatctt tgaactctgc ggcggtgtcg gggaaatggc 1532700
tgccgatatc gcccaaacct gccgcaccga gcagcgcgtc ggtaacggcg tgcagcagcg 1532760
catcggcatc ggagtgtccg agcagccctt tttcaaatgg gatttcaact ccgccaagta 1532820
tcagctttct gccttcggtc agttggtgga catcgtagcc ctgtccgata cggatgttcg 1532880
tcatcgtttg tgttcctgat gttttgaatt gaagttcaga cggcatcgag cagcagcctg 1532940
acgatgtatg cgtcctgcgg ctgcgtcagt ttcaaattgc gcacgtcgcc ctgtatcagt 1533000
agcggacgca cacccaattt ttccacggcg gacgcttcat cggtaatgcc gtccaagttt 1533060
tccgcagcca atgcgcggtg cagcagcccg gcgcggaaaa gctgcggcgt ttgcgcctgc 1533120
caaaggctcg tccgctcgac ggttgcacta atgttcccac cgtccgcgca cttgagcgta 1533180
tcggcaatgg gaattgccaa aatcccgcct tcggcggcgt tgcccgcctg ttctatcaac 1533240
cgcgtcaaag cttcagacgg caggcagcaa cgcgcggcat cgtgtaccag aatattgtcg 1533300
gtttccgccg ccaaaccggt ttccaacagt tttgccacac cgttgcggac ggtttcggcg 1533360
cgggtctgtc cgccgttttt ccacacccga acctgtggaa atgccgtctg aaccttatcg 1533420
gcaaacgtgt cttcgggcga gacgacaacg acggtcaaat cgacggcctc atgccgttca 1533480
aaaatcccaa tcgtatgttc taaaacggtt ttgcttccga tttcgacata ttgcttgggt 1533540
ttgtccgcac cgaaacgcgc cccgatgccg gcggcgggaa tcagcgcgat atttttgcgc 1533600
ttcatgcgtc cgtcccgccg ttttcagacg gcacggcttc cttgcgccag atacaggctt 1533660
cgcccaagcc gtccaaatat tgcccgtgcg ccgccaactc gttttcgtcc gccctgatga 1533720
ctttcagttt gccgctgcgt ttggtttcgg tatgcaccac gggtttggtt tccatttttt 1533780
cctctgcggc cgcacccatc aggtcgaact gccgccgcgt catagcaaga tagacttcgc 1533840
ccaaaagttc gcagtcgatc aatgcgccgt gcaggacgcg cttgctgcgg tcgacggaaa 1533900
aacggttgca caaggcatcc aggctggctt tctgcccggg gaacatttcg cgcgccatcg 1533960
ccagggtatc ggtaacggta cagccgagtt cctcaacggt cggcaacccc atccggcgga 1534020

actccatatt gaggaagccc acgtcgaatt tggcattgtg gataatcagt tccgcaccgc 1534080
gcaggaaatc ggcaatctgc ctgccgacct ctgcaaacgg cggcgcgttt ttcccttcca 1534140
aaacctgtat cgtcaagccg tggacgcgtg ccgcctcttc gggcatatcg cgctcggggt 1534200
ggacatagag gtgcaggttt ttgtcggtca tttggcggtt gaccatttcc aaaccggcaa 1534260
actcgaccaa gcggtcgccg ccgtcggcat acagaccggt ggtttcggta tcgaggatga 1534320
tttggcgtgt cgtcatatcg gtgtctttct tctatcttcg taaattgctt attttttaag 1534380
caatgtattt ttctgttttc atttcaatgc acaaacccac ttattcacag tgtgttcaca 1534440
acattgggca ggcggattgt gtattttggg gacaattttt tcagacggca ttcaaggttt 1534500
tttcctgatt gccgccgcgc ctaaaaaccg cctttcgcgc ttaatcaaaa ataccgacaa 1534560
cggaatattg cccaaagcga caatcagata caacaaggaa atgctgtcaa acaaaaacag 1534620
caacaccgcg ctcaaaacgg cagcggaaac cataaaaata ccgttaacga tattgttggc 1534680
ggcaacggcg cgggcgcgga aagtctcgct actggcggtt tgcagccagg tatagagcgg 1534740
aacggagaaa aatccgccga aaaagccgat cagcgtcatc accgccatca cgggatatgc 1534800
ccatccttgc gataaaaacc aaaaaatgcc gttcagccct tcaaaacggt gtccgtgcgt 1534860
cagccacacc aaaaccaagc cgcaaaccgt caaacccaac gcaccaaccg ttacccaagc 1534920
caacatcagg cgttccctgc tgaacttggc acacagtacc gaaccggcgg caataccgat 1534980
ggaaaacaga gcaagcatca ggttgaaaac attgtcgttg ccgcccagat ggatttgggt 1535040
aaaggtcggc agttgcgtgg tataaaccgc gccgacaaac caaaaccacg aaataccgat 1535100
aatggcggta aaaacgggct tgtgccgcac cgtttcacgc agcagggatt ttgtgccacg 1535160
gacaatattc cactcaattt gtgtatcggc agccttggcg ggtacggacg gcataaacag 1535220
gctgccgacc gtgcctccga cggcgaccag caaaaccagt atcccgacaa tataaggcgg 1535280
tacacctgcc accgccgttc ccaaaatctg accgaacagg atggcgacaa acgtacccga 1535340
ttcaatcagg ctgttgccca tcatcaactc tttgtcgtcg agataatcgg gcaggatggc 1535400
gtatttcagc ggcccgaaca gcgtcgattg cgcgcccatg caaaacagac acgccaaaag 1535460
cagcggggca gaccggatat aaaacccgta tgccgccacc gccataatga tcatttccag 1535520
caccttgacc caacgcgcca aaacggcctt gtcgaatttg ttacccaact gccccgacag 1535580
cgaggaaaac aggaaatacg gcaaaataaa cagcaacgcg cccaagttca acatctgtcc 1535640
ggcaggcagg aagccgtttt gccccaaacc gtaaaaccca atcatcacaa acagcgcggt 1535700
tttgaacaca ttgtcgttga acgcgccgag aaactgcgta gcgaaaagag gtgcgaaacg 1535760
gcggctttta accagtccca aaccgccttt tttagcgtac atcgttttcc ctctcttttt 1535820
caatcagttt acttgtcgaa tcatcatcca tcaggatgcg gtgcgccggc ccttccaagt 1535880
cgtcaaactg cccgtttttg cccgaccacc aaaaaaacca gccgatgaca aacgccaaaa 1535940
taatgctgat gggcaccaat ataaacatgc tttccatcac atattccctg tcaaatcgtt 1536000
caaaacaaaa gtctgccccg acacggtcag atattcgtta cgcaaagttc cgacgggagc 1536060
ttcgtcaaaa aacagctcga tacggtcttt gaccacgcgc caatattggg ggatttccgt 1536120
ctgaccgaac ggcgacagga catgattttc cattccgcct tcaagtttga cggcaaaacg 1536180
cccgctttgc ggccgtgctt ccgattcgtc gtcggcaagc aggatgaaaa agcctatatg 1536240
ccgtcccgat tggtcatgaa tactgaaata atgcataaat ttcccacccg cctttttttca 1536300
gacgacacca actaaaaaca gggcgaatgt accagtttgg acgggaagaa tgcaaagaaa 1536360
ttctccctcc cccagccgaa aacaccggca aaccgcatat cccccttttt tccgtcaaaa 1536420
tgcctgactt ccgccatttt cacgcaaacg cccgattaag ccaagcaatt gcaaagattt 1536480
tttgctagaa tagcctgctt cttttatcaa ccttttcaga cggccccact actttcccgc 1536540
ccaggaaggc aaaacggatt cggcacgaat ccggttagta tccgtgtccg attccaatgc 1536600
cgtctgaaac tttccggagt aagaaaatgt cccaaaaatt gatcttggtt ttgaactgcg 1536660
gcagctcgtc cctcaaaggc gcggtcctgg ataacggcag cggcgaagtc ctgctcagct 1536720
gccttgccga aaaactcaac ctgcccgatg cctacatcac attcaaagta aacggcgaaa 1536780
aacacaaagt cgatctgtcc gcacatcccg accacaccgg cgcggtcgaa gccctgatgg 1536840
aagaactcaa agcccacggc ctcgacagcc gcatcggcgc catcggccac cgcgtcgtca 1536900
gcggcggcga actgtacagc gaatccatcc tcgttgacga cgaagtcatt gccggcatcg 1536960
aaaaatgcat cccgctcgcc cccctgcaca accccgccca cctcttgggc ctgcgtgccg 1537020
cgcaaagcat tttcaaaggc ctgcccaacg tcgtcgtatt cgatacctcc ttccaccaaa 1537080
ccatgcccga agtcgcctac aaatacgccg ttccgcagga gttgtatgaa aaatacggcc 1537140
tgcgccgtta cggcgcgcac ggtaccagct accgcttcgt cgccgacgaa accgcgcgct 1537200
tcctcggcaa agacaaaaaa gacctgcgta tggtcattgc ccacttgggc aacggcgcgt 1537260
ccattaccgc cgtcgccaac ggcgaatcgc gcgacaccag tatgggcctg accccgctgg 1537320
aagggctggt aatgggtacg cgcagcggcg acatcgatcc ttccgtattc ggcttcctcg 1537380
ccgaaaacgc caatatgacc atcgcccaaa tcactgaaat gctgaacaaa aaatccggtc 1537440
tgctcggcat ttccggcctg tccaacgact gccgcaccat tgaagaagaa gccgccaagg 1537500
ggcataaagg cgcgaaattg gccttggata tgtttatcta ccgccttgcc aaatacatcg 1537560
gcagtatggc ggttgccgca ggcggtttgg acgcactggt ctttaccggc ggcatcggcg 1537620
aaaactccga catcatccgc gaacgcgtga tcggctactt gggcttcctc ggtctgaaca 1537680

tcgaccaaga agccaacctg aaagcccgct tcggcaacgc cggcgtgatt accactgccg 1537740
acagcaaagc cgttgccgtg gtcattccga ccaacgaaga gctgatgatt gcccacgaca 1537800
ctgcccgttt gagcggtctg taaggtttta tccgcacacg aactgcctcc ggaaatggag 1537860
gcagtttttt tatccggctt tccatgctta aacagcactg cctcttttca gacattgacg 1537920
gttgcagccg cttacctgaa ccttatagtg gattaaattt aaatcagtac ggcgttgcct 1537980
cgccttgccg tactatctgt actgtctgcg gcttcgtcgc cttgtcctga tttaaattta 1538040
atccactata atgattaact atttttaat catgttatta ttttccataa aatacatgac 1538100
attaagatgt ttttccacaa aagatacaca caccggcaaa caccggctgt gtttatcttt 1538160
tcttatgcct attttttaat catcgtattt ttatctttta atttcaatac gcaaactaac 1538220
ttatacacac ggttttcaca tctttagact gcttccgtgt gtatagtgga tattgccgtt 1538280
ttcctttctg acaaaaatgc cgtctgagaa cttcagacgg catttgaaac atcggaatca 1538340
gcggttttgt tcataccact cgataaactt gtctgctttg acaaaaccca gcagcggctc 1538400
gctgcggctg ccgtcggagc ggacgacaaa cacgcccggc ggcccgaaca gaccgtattc 1538460
tttcaacaac gcctgatgtt cgggcgtgtt ggcggttacg tcgatttgga aaaagcgttc 1538520
catatcgact gcctgatgca cttccggctg attgagcgtg taagccgcca tttctttgca 1538580
ggaaatgcac cagtcggcat aaaaatccaa aacgacgggt ttgtcgggat gttctttcaa 1538640
cgccgtatcc atcgctgcct tcagcgcggc agtatcggca aacattttgc cgtgttccga 1538700
agatttgcct gcttcggctg gtggattgag ggtcaggaaa tggtgcagcg cggtcgtttt 1538760
gccgtttgcg ccctgccagc cgaaccacgc gccgcctatc agcaatatac cgcccaatgc 1538820
gaatgccaca gctttcggac ggcgtttctg cctgcgtccg ttgaccagca gcataaaggc 1538880
aggaaccagc atcagcagcg tgtacagcgc gacgacgaga taatagggca agtgcggcgt 1538940
ggcgaggtaa acggcgacgg ctagcaggat gaagccgaat gcgtatttga cggcattcat 1539000
ccaatcgcct gccttaggca ggatatgccc gccgaacgtg ccgatggcaa tcagcggaac 1539060
gccggtgccc aacgccaaag tgtaaagtgc caaaccgcct aaaaccgcat cgcccgtctg 1539120
accgatgtag cccaaagcaa atgccagcgg cggggcgacg cacggcccga caatcagcgc 1539180
ggacaatatg cccataataa agacggaaac gattttaccg cctgaaagcc tgctgctttg 1539240
attctgaaaa tacgactgca cggcgttggg aagctggatg ttgaacagcc cgaacataga 1539300
cagtgccaag acgaccatta aagccgatgc cgccaatacc acccaagcct gctgcaacca 1539360
tacggtcagc agtgcgcccg tcagtccggc aacaatgccg accagcgtat aagtcagagc 1539420
caaaccctga acataaacga cggacagcac aaacgcccgc gccttgcccg cctttttgtc 1539480
gccgaccaca atactggaaa caatcggcaa caggggatac atacaggcgg taaaactcag 1539540
gcccaaacca gcgagaaaaa acgccaaaag attggcgttg agcgtatccc aagacagctt 1539600
gaaacggctg tcgccgccct catccccctt cgggggcggc agcgccccgc tgccgttttg 1539660
agaggaaggc tgcaaaaagc ggtctttggc ggatgccggt tcgtcggttt gcggatggta 1539720
agtgccgttg ccgaaaatat caaactcggt atccacgggc ggatagcaca cgccggcttc 1539780
ggcacagccc tgataggtca aaaccaattt atacggttcg ccgacagcct ttgcataagg 1539840
aaaggcaacc tgcgcctcgt gatggtaaac cgtctgcctg ccgaaaaact cgtcttcctt 1539900
ctcttcgccc ttgctgaaag aaggctgtcc caacaaatcc gccggatcgg tcttgccgac 1539960
gattttcgcc tgatacatat agtatccgtc ggcaatcctg aaacggacgt tcacaccgtc 1540020
gtcggcaacg gcaagctccg gcacgaatgc cttttccggc ggcagcagat cgttcgcatc 1540080
cagcgcgaaa gctcgtccgc acaacatcaa aaatacggcg aacaggcaaa tcagtttttt 1540140
cataatcgaa tccgtttcag acaaataatt tgtctgcatt ataaatggta aggttgacgg 1540200
tgggatttaa tttatgtaaa acccgccatt atccgaacct atttccataa acatcttatc 1540260
gaacccgcca tgtacgatgt caatacccac gatgtccgcc gctttttcgc ccgcgtgtgg 1540320
cagcagcggc tcaatccgct gcaactgagc gcactggaac agaaagccct ccgcattgtc 1540380
gaagcccatc ccgaatacca ccgttatctc gaacgcatcg aagaccatct ggacaccgac 1540440
tggctgcccg aaaacggcga aagcaacccc ttcctgcata tgtcgctgca tctgtccgtc 1540500
caagaacagg cgggcataga ccagccgcac ggcatacgcg caatccacga caccctgtgc 1540560
gccaaacgcg gctggctgga agccgaacac gaaatgatgg aggcactggc ggaaacactg 1540620
tggacggcgc aacgctacgg caccggtttg gatgtcaatt tctacatgac ccgactgcgc 1540680
aaactcatcg gcttgggtgc agaagatcaa gccagattga acccgcatga aatcgcctga 1540740
ccataccaac cgcctgcaaa atgccgtctg aagcggaaca accccctttca gacggcattc 1540800
attttccccc aatcatttcc acaacgcctt tttcagcata atcaaccaat ccttcttatc 1540860
caaaacgggg cgttgtgcaa acacatcgta tcggcacgcg tccagtttct gcaaaatcaa 1540920
ctgcgccccc aacacaatca tacggagttc caaaccgata cgcccattca gttcccttgc 1540980
caaaggcgaa cccgccttca gcatacggaa cgcacgccga cactcatacg ccatcagccg 1541040
ctgaaacgcc gcatccgccc gtcctgccgc gatctgttcc tcagaaacac cgaatttcaa 1541100
caaatcgtcc tgcggaatat aaaccctgcc tttttgccaa tccacagcca catcctgcca 1541160
aaaattcacc agttgcaaag ccgtacagat gccgtcgctt tgcgccacgc acaccgcatc 1541220
cgttttcccg tacaaagcca gcataatgcg tccgacaggg ttggcggaac gccgacaata 1541280
atcggccagc tcgccgaaat ttccatacct tgttttaacc acatcctgag aaaatgcaga 1541340

aagcaaatca taaaacggct gcaaatccaa accgaacggc acaaccgcct cggcatccaa 1541400
tcgtgcaatc aaaggatgcg ccgaccggcc gcccgatgcc aacacgtcca actcgcgctg 1541460
caaaccctcc aaccccgcca acctggcttc agacggcata ctgccctcgt ccgccatatc 1541520
gtccgccgtc cgtgcaaacg cgtacaccgc gtgaaccggc ttcctcaacc tgcgcggcaa 1541580
aatcagcgaa ccgacgggaa aattctcata atgcccaacc gacatacctt ctccatccat 1541640
caaacaaaat gccgtctgaa acggaacaaa cccttttcag acggcatcag atacctccaa 1541700
gctgccggca atcagtggtg gtgatgaccg tgcgggccgt ggacatgacc gtgtgcgatt 1541760
tcctcatcgg atgcatcgcg cacgctttca actgtagcct taaagcggat tttcatgcct 1541820
gccaaaggat ggttgccgtc caccaccgcc ttgccgtcgg caacatcggt tacacgatag 1541880
acgacaacat cgccggtttc aggatcgtcg gcttcaaaca tcatgccgac ttcgacttca 1541940
acagggaaca cgcccgcatc ttcgatacgg accaactccg gatcctgctc gccgaacgca 1542000
tcgtcgggcg acagcgccac atcgaccgta tcgccggcat ccttaccgtg caacgcctct 1542060
tccaccaaag ggaaaatgcc gtcgtaaccg ccgtgcagat acgcaatcgg ttcttcggtt 1542120
ttgtccaaaa gctgattgtt ggcatcatac atctcataat gcagcgaaac cacggaattt 1542180
ttcacgatag ccatatttgt cctttcagga acagcagatt aattacaggc gcattctaac 1542240
acaaccgccg cgccggccga ttaccgttaa cctgttcata aactgtacag cacatatttc 1542300
aatgtaaatc tttgttattt tattgcggtg taactttttt acaacattct taaaaccatt 1542360
ccgacctgtc tgccgacttt cccaatccgc cttaataaat catacaagat actgaaatta 1542420
tattaatctc tataatattt atccctatcg aatttttaac agcaaaaccg ttttacagga 1542480
tttatcaatc cgcccgccag aaaacttttc attcaaacct ttttcccatc tgtacgacat 1542540
tgcaatccct tattccatag tgcataatta cgcaaattca gcgatgaatt tccaacccgg 1542600
tttgtagtat ggtcgataaa gacctatttg tttcaataat ttaaattggt tctaaaggtt 1542660
actaaaatga aaaaatccct gtttgccgct gctttgttgt ctttggttct ggcagcctgc 1542720
ggcggtgaaa aagccgctga agctcccgct gctgaagcac ctgccgccga agctcccgct 1542780
actgaagcac ctgccgccga agctcccgct gctgaagcac ctgccgccga agctcctgct 1542840
gctgaagctg ccgctaccga agcacctgcc gctgaagctg ccgctaccga agcacctgcc 1542900
gctgaagctg ccgctaccga agcacctgcc gctgaagctc ctgctgccga agctgcaaaa 1542960
taagcatttt ccgcttgcaa aaaagcagga tacgttcagt atcctgcttt tttgattttt 1543020
cagacggcat cagattccct tcctcaatct tctccctacc cttccgacaa acatgcttga 1543080
ccttcatacc gaattttccc gactcctacc ggcagatgaa attgccgaac cttctccgac 1543140
gcttttaaaa gaccagcgca accgctttac gtctgcacca gacatcattt tgcagccgct 1543200
cagcgttaaa agcgtgcaaa ccattatgcg tttctgccac caacaccgta ttccggttac 1543260
gccgcaaggc ggcaatactg gtttgtgcgg cgcggcagta tcggaaaacg gcgtattgct 1543320
gaacctttcc aaactcaacc gcatccgcag catcaatttg tcagacaact gcataaccgt 1543380
cgaagcaggt tccgtactcc aaaccgtcca acaggcagcc gaagcctcaa acaggctgtt 1543440
cccactcagt ctcgccagcg aaggctcgtg ccaaatcggc ggcaacatcg cctgcaatgc 1543500
cggaggtttg aacgtattgc gttacggcac gatgcgcgac ctggttatcg gtttggaagt 1543560
cgtcctcccc aacggcgaac tggtttccca tctccatccc ctgcataaaa acaccaccgg 1543620
ctacgacctg cgccatctgt ttatcggtag cgaaggtaca ttgggcatta tcactgccgc 1543680
cacgctcaag ctgtttgcca acccccttaga caaagcaacc gcatgggtcg gcatacccga 1543740
catcgaatcc gccgtccgcc tgctgaccga aacccaagca cactttgccg aacgcctatg 1543800
cagttttgag ctgatcggcc gttttgccgc cgaattgtct tccgaattca gcaaactccc 1543860
cctgccgaca cattcagaat ggcatatttt acttgagttg accgactcat tacccgacag 1543920
caatcttgat gatcggcttg tcgaatttct ttataaaaaa ggctttaccg acagcgtgtt 1543980
ggcgcaaagc gaacaagaac gtatccatat gtgggcgttg cgcgaaaaca tctccgcatc 1544040
gcaacgcaaa ctgggcacca gcatcaaaca cgatattgcc gttcctatcg ggcgcgttgc 1544100
cgactttgtc cgccggtgcg ccaaagattt ggaacagaat ttcaaaggca tacaaatcgt 1544160
ctgcttcgga catctgggcg acggcagcct gcactacaat actttcctgc ccgaaatcct 1544220
cagcaatgaa gtctatcgtt acgaaaacga catcaacagc acagtctatc gcaacgtcct 1544280
tgcctgcaac ggcacgattg ccgccgaaca cggcataggt atcatcaaaa aacagtggct 1544340
ggacaaagta cgcacgcctg ccgaaatcgc cctgatgaaa agcatcaaac aacaccttga 1544400
tccatataac attatgaatc cgggcaaact gcttccgtaa ccggcatttc tgatttgcat 1544460
acacaacaaa gaaagggaca atagatccga ttgtcggttt agcgcgagct cgtgagtgcg 1544520
gttaaaaatt ggtggaaatt acacgaaaaa tgaccgcact tttaaaataa aaaaatcggc 1544580
agtgaatttc cctgccgatt ttattttgtt acaacttaac ttaaaacgtc cactgtaaat 1544640
tcaacgcacc ttgtttagct tgatgatgtt tgcctgtttg gcggttgaat gtggcttgta 1544700
aggttaagtg agatttgatt ttcactgcta cacctaattg gctctcaatt gccgtcttat 1544760
tgtttatcac tcgacgctct ccgtccattt ccacaccgaa aggtttgttg tggtaaagcg 1544820
cgttcacagc ggcgaaaggt tcaatagcga tatttttata gagtgaaaat tgagctttag 1544880
cttgaacgcc aacccgagtt tgtaattggc gggagccaag taaattcacg tgggcatttt 1544940
cgctatcgct gaattttccg tttacccccca aataagtcaa ttgtgcctgt ggttgtaggt 1545000

aaacacgaag gctgttgccc tttttagtga agtgttccgc caataacgca ttgtaacctg 1545060
cttcaattga ggcagtaata ccttttgaag taaaacgttc tgtaccatct tcagtgttga 1545120
tacggtggcg gaagcgttga tattgcatcc agctatccgc atacgcacct gtctgtttgt 1545180
cctgaagttg gtgccaagtg gcgtaaacgc ctgcaccaaa gcctttcaca tttcccgttg 1545240
taagattgtc tgtatctggg ttgtggaaag tgctacgttg ttctgcttgt ccgcccatta 1545300
agccaataga aagttgatta ctttcgtttt gccatgtgaa tacttcgccg ccgagttgca 1545360
cacctttacg atagccttct acaggtgctg ttttgccttg cacccattgg ttggaatgtc 1545420
cgtcaatcac acgcaaccac aagcctttgc gtggtaaagt gcggtcgaaa atatcgctgt 1545480
ttttgttgtt caaacgcaag gcgaataagg tattggcggc ttgagcctgt tgtgcataaa 1545540
tcgccatatc atcgcgttct tgcactttgg taaaaaagcc ctctgggcgt tgttgtaaag 1545600
aaagcgtata aattcccttt tggtgtttgc cagaaagacg gaatgcgtgt ttatctgctg 1545660
tgccatttac tttgataatt tgatgcccat cgaggctttt taaatcgtct attggatttt 1545720
cgaagatgat gtcggaagtg ccagtaacat ttttctcaaa aattaatgca gtatttttcg 1545780
cttctttagg atcgtaagca aaacgaaaac gagctccgcc agcataatct tcttttacga 1545840
gtaaactttc actttagta ttaaaacgga tgtctgcatt cgttgttttt aatttcccaa 1545900
cattagaatc ccaacggggc tcccagagag aattttctaa gcggaattca tccaaactaa 1545960
tcgtttgccc gataacgtgc gagttgtctg taacctcaat atagtggaat ggatctaaac 1546020
cagaatatag atgtgctgca aaagaaacat aattttcaat atgatgaatt acttgattag 1546080
cccattctgt ataattcccg acagataaaa tttcgctgtt gatatgacta tttttattt 1546140
ttggacctaa ggagaatata tgacttttta ctataagagg atgggatcca aatttttcag 1546200
cttggcaagt actataatca cgtatcttag tgttagaatt aaaacattcc ttaaaatatt 1546260
tccgtatttg ttcttctgtg tccccatttc tttttgcaac ccctaaacct cgggcgaagc 1546320
caactaggta accttcggta tattcttgat cataaaaaga aatcttttt gagttattga 1546380
tgttttcgaa ttggtatgtt ctagggtata gtgcgggaaa gggtggaact tttggattat 1546440
cctcggttat aagataagtt tcttttttcc aatattcact cgttttatcg cggagttttt 1546500
ttaagcgggt aatttcatca ttagtgagct tggttttgtc gtaaacgtaa tcaacagcca 1546560
aaagcggaga ggtataaaga atagaaaaaa atagacttac aataaatgat tttttaaact 1546620
tctgcttgct tgcttgcttg cttcgagttt cataataaat tttcctttgt caagtaaaaa 1546680
taaatggggc gtggatttta gcataaaact gaacaaaaaa tgtcatttat ctcacatttt 1546740
tctctattta tttcttgttt attaaaagta aacgtttgct ttttgctatt ttgtcaagcc 1546800
agtttgaaaa tgtgtataat tgccctcgtt atttacaaaa atttcaggaa aaatgaccgc 1546860
actttaccct tggctaatgc caatttatca tcaaattgct caaacctttg acgaaagctt 1546920
ggggcatcat gccgtgctga ttaaagcgga tgctggttta ggtgtagaac gtttacacat 1546980
caggcggcag ccttgcccat accgtctgaa gcactgtttc cacaatcagc gcgtatgctt 1547040
aatcaaccgc tgtttctcgc gtttccaatc cgcctctttc atactctggc gtttgtcgtg 1547100
ctgtttctta ccttttgcca aaccgatttc catcttgatt tttccgcgtg aaaaatgcaa 1547160
atccagcggc acgatggtgt agccggcacg ttcggttttg ccgattaatt tgttgatttc 1547220
cgactggttc aacaagagct tgcgcggacg tacggcatct ggtttaatgt gtgtcgaggc 1547280
tgtgggcaaa gccgtaatat ggcagccgac cagataaaac gcgtcttttt tccaatagat 1547340
ataactctct ttaagctgta cgcgcgcggc gcggattgct ttgacttccc agccttccaa 1547400
gaccaaaccg gcttcaatcc ggtcttcaat gaaaaaatcg tgaaatgctt ttttattgtt 1547460
cgcaatagcc ataaacatcc tatcaatatc cgccgtcaga cggcataaac ccgaaaacag 1547520
aacccatcat accgcctctt caaccgcctg cacaatcttc tcgggataca gcctgttgag 1547580
gcagtcggta tgccccagcg gacattcccg cttaaaacac ggcgaacatt ccaagtgcag 1547640
gctgacgatt ttcgccctat cgctcaaagg cggcgtatgc gtcgggctgg aagaaccgta 1547700
aaccgccacc accttcctgc ccaaagctgc cgccaaatgc atcaatccgc tgtcgttaca 1547760
cacgaccgtg tccgccaacg acagcaaatc cattgcctgc gacaaatcgg ttttgccgca 1547820
caaattgaca cacataccgt ctgaaaggcg gttgatttcc tcggcaattt catcatcttt 1547880
ttgcgaaccg aacagccaaa cctgccaacc cgccgccaga taatgtttgc ccaactcggc 1547940
aaaatgcctt gtcggccaac gctttgccgg cccgaattcc gcacccggac aaaaagccag 1548000
aacaggcttt ccaatatcca agccaaaggt ttcgacagaa atttcccgcc gccgttcatc 1548060
aatggaaaac tcggggaatc ccgaatgccc gtcaaaatct tcctgactcg gatgcgcgag 1548120
agccgtatat cgatccacca tcaaaggcag acgttcctta tccagcctgc gtatatcgtt 1548180
caacagaaaa taacggcttt caccgacata acccgtcctt ttaccgatac ctgtcgccag 1548240
cgcgatgatt gccgatttca aagaaccggg caacacgata acctgatcgt atccgcgccg 1548300
ccccaaatcc ctaccgaccc gccaacggcg tttcaactcc aacgcaccat gtccgaacga 1548360
attctcaaga atttcattca cttccggcat acgctcgaac accgccatcg accacttcgg 1548420
tgcgaacaca tcaatcgtgc aaccggggtg aagttccttc aaacggcgga acaagggctg 1548480
ggtcatcacg cagtcgccta tccaactggg ggaaataatc aggattttga tggacataac 1548540
aagaaaccga aatcagacag gcagaatttt accgcgaaac cgttggaaaa cctatcttgc 1548600
cgcattccga acgccggacg tgcaaatatg aaaaagcccg aacattcaag ttcgggcttc 1548660

aaaattctgg ctccccgacc tgggctcgaa ccagggacct gcggattaac agtccgtcgc 1548720
tctaccgact gagctatcgg ggaatggggc gtattatagc gtccggaaaa aatgtgtcaa 1548780
tccttaattt tggaaaaatg ggcgacaaaa cgacaagcat atgaatcaga aagacattaa 1548840
gaccgatgcc ttaaaaggat tgccgttgta tgaatttcca cagccgtcat cacaccatat 1548900
ttaagcccga tgagccgttc tgccctcccc ccgcttaaaa caatgccgtc tgaacttcgc 1548960
cgtgttccaa agccagtaaa aactgtttgc ggttcaaccc gcccgcgtag ccggtcagtt 1549020
tgccgtcgct gccgatgacg cggtggcagg gaatcaggat agatactttg ttctgcccgt 1549080
tggcggcggc aacggcgcgg acggctttgg ggttgcccaa acgctgcgcc tgctccttgt 1549140
agctgcgcgt ttcgccgtaa ggaatcgcca agagcgcgtc ccatgcctgc ttttgaaact 1549200
cggtgccaat ctgctccaaa ggcgtggcaa aggttttcag acgacccttg aagtataagt 1549260
ccaattcctg ccgcaaaagt tgcgtccgct catcctcccg aaacacaaac cgtccgcgca 1549320
aggctttttg gacggcggca atttcctgtt ccaaatgctt ctgtccgaca aattccagca 1549380
aacacaaacc cctgctaccg aacaccgcca gcatctcgcc caaaggcgtg gcaatggcgg 1549440
cacacaccag ctcgttcaaa ctgtcgggat aacgcgcttc caacagacgg atggcgcggc 1549500
ggatgcggac atattcttca ggcgcgcagc cgatattgtc ccaaaaatcc cgctcgaact 1549560
gtttggcttc gcattccgtc agattgggat gcggcataac gccgcactca aaaacccgag 1549620
attcgagcca atggcggatt tcatcccatt ttgacggcag attgtttaag gaaggcaggg 1549680
taatcatttg tttgctccgt atccctatca tagatttgac ggcaaaatcc ccaatttttg 1549740
ccattcccgc acgccggagc aggaacgggc tatgacgtaa atcttgaggg ttaggttgcg 1549800
gcaataccta aatattcgat atttctaaag catcagagaa aggaatgttt caacacacag 1549860
gacgacacat aaagcgccgc cccatgaaaa atttcagacg acctgcaaag ggtcgtctga 1549920
aaccacgatt tttgcatttg cgcattctgg cacatcatcc aaccgtttcg gcacattcct 1549980
gccgccgttg acagcctata atgaatccac ttattcatca agcaaaggaa tcatctatgc 1550040
aaaccctcat cctctccgcc gtactgctgg ctttttcaac cgctgccttt gccgggggcg 1550100
cattcacgct gcaattcgac aacccgtccg aagacggcgg cttcacgcaa aaccagcttt 1550160
tgagcgcgcc ttacggcttt tgctgttcag gcgacaatgc ttcgcccgcg ctgtcgtgga 1550220
aaaatccgcc cgccgggaca aaagtttcgt cctgaccgtt tacgataaag acgcgccgac 1550280
cggactgggc tggatgcacc gggtggtcgc cgacattccc gccgatgtcc accgccgcaa 1550340
cgcgacctcg ctgcaattaa gccgctgcgc caacatcgcc gaccggactg ggctggatgc 1550400
actgggtggt cgccgacatt cccgccgatg tccgccgccg caacgcggcc tcgctgcaat 1550460
taagccgctg cgccaacatc gccgacgacc agtccgcagc catatcggcg gtaatcagtt 1550520
tgcggatttg ccgcatcagg ttgacgcctt cgtacacggc aaaaccgatg ccgtcatgct 1550580
gcaaccacgc caacacgccg caaagcgcgg cctccgcagc attgtgcggc acttcttcat 1550640
ccgccagtac cgcagcctca taatcaaacg ccgcgcccat acgcccggaa tacggcagct 1550700
ttaccgcatc gcacactgcc tgcgccgtcc cgtattgtgc ggcgaacctt tctacggttt 1550760
cctgttcgaa agcaatccat tgcgcctgat agaggccgtc tgaatcggga atattgatga 1550820
cgtcaaacgt ctgtccgcct gccaaggcga ccgccttacc cgccgcagct tcttacttcc 1550880
gcgccgcacg ataagcacag ccggttcata taccgccacg ctgcggtaca aggcggtatg 1550940
atgttgcacg atgccgccta aagcacccaa tcgttcgcgc gtatgaaagt atagtggatt 1551000
aaatttaaat caggacaagg cgacgaagcc gcagacagta caaatcgtac ggcaaggcaa 1551060
ggcaacgccg tactggttta aatttaatcc actatatctc aaacccacgt taggtctaag 1551120
caaatggtcg gacatcctta tccgacagcc catcttcttt tcagacggca ttgcaaattt 1551180
aagtttgacg tgcgttcaaa ataaggcagt taatgcgaag cgaaattccg tcggcgtacc 1551240
tgcaacttgg cccctcccct ataggggagg gtcggaggga gggtaaaacg gggcagatac 1551300
agacaatatt tccgttgccg ccccgatgcc ctctccctaa ccctctccca cgggagaggg 1551360
aatggattgc cgttgaaata aatcgctcta cataaaaaat caatgtgtta tctcaaaccc 1551420
acattaggtc taatcaaatg gtcggatatc catattcggc aagcaagctg ctttcagacg 1551480
gcatttccag ccaacaagcg cgccaatatc ccctcataca ccgcagacag cttcggaatg 1551540
tcgtttagcc gcacgttttc gttgatttgg tggatggtcg cattggacgg gcctaattcg 1551600
ataagttctt gcgcaatggc tttgatgaag cgtccgtccg aagtgccgcc ggtggtggac 1551660
aattcggcct caatgccgca ggtttcggca atggctgcgc gtgccacgtc ggtcagtttg 1551720
cccgcttggg tcagaaaggg ctgccccgaa cacgaccact gcaaatcgta ttgcacgccg 1551780
tgtttgtcca aaatggcgtg gacgcgttgt ttcagccctg cttcggtgga ctcggtggag 1551840
aagcggaaat tgaatttgac gttcagctcg cccggaatga cgttggtcgc gcctgtgccg 1551900
ccgttgatat tggaaatttg aaagctggtt ggcgggaaat attcgttgcc ttcatcccag 1551960
acttcctgcg tcagctctaa caaggccggg gcaaaagtat gcacgggatt gattgccaaa 1552020
tgcggatagg caatatggcc ttgcttgcct ttgacggtca ggttgcccga cagcgagccg 1552080
cgccgaccgt ttttaatcat atcgcccaat ttgtccacgg cggtcggttc gccgacgatg 1552140
cagtagtcga taagctcgtc gcgcgctttc aatacatcga cgactttggt cgtgccgtcc 1552200
aacgcgtcgc cctcttcgtc ggaagtaatc agaagcgcaa tgctgccttg gtggttggga 1552260
tgtttggcaa cgaagcgttc gcaggcggta acgaaacagg caatgctggt tttcatgtct 1552320

gccgcgccgc gcccgtataa tcttccgtcg cgctcggccg gttcgaacgg gggcgaatcc 1552380
catttttcga caggacctgt cggtacaacg tcggtatgcc ctgcaaaaca gacgacggga 1552440
gctttcgtgc cgcgtcgcaa ccagatgttt ttggtgtcgc cgaaatggag ttcttcagcc 1552500
gcaaaaccga ttttgtgcag gcgttcggca aggagttttt ggcaatccct gtcgtcaggg 1552560
gtaacggatg gtcgggaaat cagctctttg gcaagctcta gggattgagt ttcggtcata 1552620
tttgttcact tttgaaatta gaccgtctga aacgttctga atgtgatttt cagacggcat 1552680
ttaggttagg ttggcatacg gggtgggtat tttacccatc agtcttctga atcatttgcc 1552740
gtggcaggct tcgtaaagcg gcagcaaatc ttccaccgtt tccgctatcc atttcgcgac 1552800
atcctgcctg cccaaatcgt cgcgttcgat gtgtttgccg atgcagaaaa agtcttcgtc 1552860
gttttgcaac tttcggtcgg actcgttttg ttgcgcgacg gtgcggtagt cgtcgtattc 1552920
gctttccgca ccgtgccaca tatcgaaaga agcgtatttt tcggtatcaa aattatccaa 1552980
ccagcggttg taatcaggca gcgcgatggg ggaaacatcg gctttatagc agtgccaatc 1553040
caagctgacg ctcagacggc ggcggttgag cagtatcgac aaaatcgctg cggaattttt 1553100
atattgttcg tatttgaagt aggcaaagaa atgggcgcga acctgccagc cgttacacca 1553160
gcgttcgatg tgcggcggcg caaacggcgc acccaattcg gcggcaacct gctgaatcag 1553220
ctgctgccat atctgccagt tttctttata gtcagccttg atttgcggaa tgctttcagg 1553280
ctggtatttt ttaagctggg aaaattggaa aaacgggata ttgaacaaat cgcaactttt 1553340
cggggtcagc ataatatatc cttgagacga ttgtttcaga cggcattatt tgcgccggcg 1553400
cgccgccata atttcgccga tttcggtcag tttttctttt gggataaagg tgttgcccat 1553460
atcaaacagc ggctcttcaa tcgccaaatg aacatcatat cccgccacaa aacgtttgaa 1553520
cgcttcctca tcggggacat aagcgttgtc tgcttcgagt ttggcaaatt cggcggaaac 1553580
agccgcccag ttgtcgtgca gcccgatatg ttggcgcaaa agctcgtcca cgctttcttg 1553640
ggcttgcggc gcatattgca gcagcagcgg gaagaagttt tcttcttcgt cttcatggtg 1553700
cagcggcgcg gcaacgttga aatactgggc gatttggcgg atggtttgca aaacaatctg 1553760
attgcagccg ttttcggcga tatagtccga cagcatggcg acttgtccgc aaaaacggcg 1553820
cactttgccg tggcaggcat acagcatttc aatcggttcg gcaaaggtaa cgcttttggt 1553880
ttcaaacgga ttcatgtttt cgttctcaac gggcactttt caagcagtca ttttataata 1553940
aaacagcctg cacaaagcag gctgtccgtc ttttgagact ttaagcggat taatcgacca 1554000
aagtcacttt gccgttcatc aaagcaccgt gacctgggaa ggtacaagcg aatttatatt 1554060
cgccgtcggc caatttagca ggatccagag tcagggaagc ttcttcgccg ccgccgatca 1554120
gtttggtatg ggcaacaacg cgtgcatcat caggtttgac atagtcggta tcggcagcac 1554180
ctacgccgtc tttaaatacg ccgtccatgt cttcagcttt ggcaatcacg agattgtgac 1554240
ccatgctggc tttgggttgc gtaccggtat gtttcagagt gatggtgaac tctttacatg 1554300
ctttgctgac ttggatgtct ttggtgttga actgcatatt gtcgttggat tcgacagttg 1554360
ccgcacagtt gccggcagca ggggcttcgg cagcatctgc aggagcagct tcggcggcag 1554420
gcgcttcgga agcgggtgct tcagcagcag gagttgcctc ggcagcaggc gcggcaggtt 1554480
cttgagagca ggcagccaaa ccgataacgg cggcagaaat cagagccaga tacgctttca 1554540
taacaaatct ccaatcgata aaataatatt cggttttaca gaaatcaaag tgcaaccgcc 1554600
attaacaaaa ccttgaaaaa gattccgccg cgttgcacaa acagatgttt cggagcggca 1554660
ttttgctaca aatttcattt gaaatcaaag cctgtttgca agtttacaat cgtttaccca 1554720
aaaaagggca attttacccc gaacctattt ctttagtatt agacctatta tcctttactt 1554780
cttaatatta acggatgttt acacaaattc ccgtatacat tttatgcgcc atgccttcta 1554840
accaagtttg ccaatgcctc cgccaattcg ggatgccgtt tttccaactt tgccgccgcc 1554900
gaaccgaaac tctccagcgc agccttactc aaatgcaggg tattggtttt cggcggtttt 1554960
tccggtttcg ggaccagcct gaccgaaaca gagcgtatcg aagcatcaag ccctgccaac 1555020
tgcggcaata ccgacggtgc aatcattttc aagcgcgatg ccgccatatt gtttgccgcc 1555080
aaaaggacaa gcctgccgtc ttcgatacat gccgtctgaa aatgcgggtg caggttggca 1555140
ggcagcagtt ttttcacggc ggcatccaac cgccgccact gtcccgcctg tttcaaaagt 1555200
ccggaaagca gcgcgtcccg cctgcccaac tgttccaaat tcataaaaca tacacccaaa 1555260
aagattgaaa taccgcaaac gcgcctttat ttcagacggc attagcactt tgcacaaacg 1555320
cttgtgttaa aatcgcgttt tcgcccacta ttatatcagg cgcaggaatt attcatgctg 1555380
acaaacattg ccaagaaaat cttcggcagc cgcaacgacc gcttgctgaa acaataccgt 1555440
aaatccgttg ccagaatcaa cgcgctcgaa gaacagatgc aagccctaag cgatgctgat 1555500
ctgcaagcca aaactgccga attcaaacaa cgcctcgccg acggtcagac tttggacggc 1555560
attttgcccg aagccttcgc cgtctgccgc gaagcgtccc gccgcaccct cggtatgcgc 1555620
cacttcgacg tgcagcttat cggcggtatg gtgctgcacg acggcaaaat cgccgaaatg 1555680
cgtaccggcg aaggcaaaac cttggtcgcc accctcgccg tctatctcaa cgcgctggcc 1555740
ggcaaaggcg tacacgtcgt taccgtcaac gactacctcg cctcacgcga tgcgggcatt 1555800
atggagccgc tctacaattt cctcggcctt accgtgggcg tgattatttc agatatgcag 1555860
ccgttcgacc gtcaaaacgc ctatgccgcc gatatcacct acggcaccaa taatgaattc 1555920
ggcttcgact acctgcgcga caatatggtt accgaccaat acgacaaagt gcagcgcgaa 1555980

ttgaattttg ccgttgtcga tgaagtggat tccatcttga ttgacgaagc gcgcactccg 1556040
ctgattatct ccggtcaggc ggatgacaac atccagttgt accaaatcat gaacaccgtt 1556100
ccgccccacc tcgtccgtca agagacagaa gaaggcgaag gcgactattg ggtcgacgaa 1556160
aaggcacatc aggtcatcct gagcgaagca ggtcacgaac acgccgagca aatcctgacc 1556220
caaatgggat tgctggcaga aaacgactcc ctctattccg ccgccaatat cgccctgatg 1556280
caccacctta tggcggcatt gcgcgcgcat tccctcttcc acaaagacca acattacgtc 1556340
atccaagacg gcgaaatcgt catcgtggac gaattcaccg gccggctgat gtccggccgc 1556400
cgctggtcgg agggtctgca tcaagccgtc gaagccaaag aaggcgtgga aatcaaacgc 1556460
gaaaaccaaa cgcttgcatc tattaccttc caaaactatt tccgcctgta caccaagctc 1556520
tccggcatga ccggcacagc cgataccgaa gccttcgagt tccaaagcat ctacaacctc 1556580
gaaaccgtca tcattccgac caaccgcccc gtacagcgca aagacttcaa cgaccagatt 1556640
ttccgttccg ccgaagaaaa attcgaagcc gtcgttaaag acattgagga atgccacaaa 1556700
cgcgggcagc ccgtcctcgt cggcaccacc agcattgaaa actccgaact ggtatccaag 1556760
ctgctgaccc aagccggact gccgcacaac gtcctcaacg ccaaagaaca cgaacgcgaa 1556820
gccctgattg tcgcccaagc cggcaaagtc ggcgcgatta ccgttgccac caatatggcg 1556880
ggacgcggta cggacatcgt tttaggcggc aacctgaagc accaaaccga tgccatccgc 1556940
gccgacgaaa ccttgagcga cgaagagaaa caggcacaaa tcgccgcact cgaagacggc 1557000
tggcaggcgg aacacgacaa agtgatggaa gcaggcggtt tgcacatcat cggtacggaa 1557060
cgccacgaaa gccgccgcat cgacaaccaa ttgcgcggac gttccggccg tcagggcgac 1557120
cccggatcca gccgcttcta tctctccttt gaagacccat tgctgcgctt attcgcactc 1557180
gaccgcgccg ccgccatcct caaccgcctc gcccccgaac gcggcgtcgc catcgaacac 1557240
aacctgctga cgcgccaaat cgaagggggcg caacgcaaag tcgaaggcag aaacttcgat 1557300
atgcgcaaac aggttttgga atacgacgac gttgccaacg aacagcgcaa agtcatttac 1557360
agccagcgca acgaaattct gaccagcaaa gacatcagcg acctgatgca ggaaatccgt 1557420
tctgatgtcg tcagcgacct cgtggatacc tatatgccgc ccgacagcat ggaagaacaa 1557480
tgggacatcc cgactttgga gaaccgtctg gctgccgaat tcagactgca cgaagacatc 1557540
caatcctggc tgaaggcgga caatgcgatt gacggtcaag acatcaaaga acgcctgatc 1557600
gaacgcatcg aaaacgaata tgccgccaaa accgaactgg tcggcaagca ggcaatggcc 1557660
gatttcgagc gcaacgtgat gttgcaggtc atcgacaacc aatggcgcga acacctcgcc 1557720
gctatggact acctgcgaca aggcatacac ctgcgcagct atgcccaaaa aaatccgaag 1557780
caggaataca aacgtgaagc ctttaccatg ttccaagacc tgtggaacgg catcaaattc 1557840
catattgcct ccctgcttac ctcggttcaa atcgaacaaa accctgtcgc ggtggttgaa 1557900
gagcaaccca tcggcaacat ccagtccatc cattccgaat cgcccgatat ggaagaactt 1557960
ttgggtcagt cgcaaaccga tctggttacc gaagccttta atcccgatgg gacagatttc 1558020
agccccgaag ccttggaagc gcgggggcaa atcgtccacc gcaacgaccc ctgcccctgc 1558080
ggcagcggtt tgaaatacaa acaatgccac ggcaaactgg cttaagcgtt tgaacgcaaa 1558140
tgccgtctga acatcccgct cccgtttcag acggcatttt gcctgaaccg ccacatccga 1558200
ctgccattcc gaaaaatccc gatttcgtac cgtccgtacc aaaaacagac atcccgtccg 1558260
ccccacatca tgattccatc cgacttcatt gacgagcttt tagccaaaac cgatattgtc 1558320
gatattatcg acgagcaggt tccgctgaaa aaaggcgggg cgaactatat ggcgtgttgc 1558380
ccgttccaca aggaaaaaac gccgtcgttt tcggtcagtc caaccaagca gttttaccat 1558440
tgtttcagtt gcggggcaca cggctcagcg attggttttg tgatggaaca tcagggactg 1558500
tcgtttccgg aggcggttca gttccttgcc gaccgcgtgg gtatggtcgt gccgaaagtg 1558560
cacgggcaaa acgataatcc cgaagtccgt gccgaacgta agaaaaaaca gcagacactg 1558620
gaggaaacga cggctgcggc agctgatttt tacgcgcaac agctaaaatt caatccagcg 1558680
gcaaaagctt atttggacaa gcgcggcttg agtgcagaag ttatcgcgca ttatggtttg 1558740
ggctatgcgc ccgacggctg gcagcctttg acgcaagtgt tccaaccgta tcctaatacc 1558800
gcgttagtgg atacggggat ggtgattgac aatgagggac ggcattacga ccgcttccgc 1558860
catcggatta tgttccccat ccgcaatccg cgcgggcagg ttatcggttt cggcggcagg 1558920
gtgctggacg actcgaagcc gaaatattta aattctcccg atacgccttt gttcgataag 1558980
gggaaaaacc tttacggact gtatgaaggg cgtgccgctg tcaaggaagc ggggcggatt 1559040
ttggtggtcg aaggctatat ggacgtggtc gcgctggcac agttcggcgt gggctacggc 1559100
gtggcggctt tgggtacggc gacgacggcg gaacacgtca aaatcctgat gcgtcaggca 1559160
gacagtattt atttctgttt cgacggcgac agcgcggggc gaaaagcggc ttggcgcgcg 1559220
ctggaaaacg cgctgccgca gttgaaggac gacaaatcgc tgcatttttt gttcctgccg 1559280
gaagaacacg accccgacag ctacatccgc gcctacggca aagcgcaatt tgaagacgcg 1559340
cttctgaatc aaagcaagcc tttgtcggag tatttctggg aacacctttc agacggcatt 1559400
catctcaata cgcaggaagg caaggcggaa ttggtaaaaa ccagttcgcc gcttttggcg 1559460
cagattaccg cgccggcatt ggcttatttg ttaaaacaac ggcttagcga gctggtcggc 1559520
atcgaccccg acaacctcgc gcaactgcta ggacaggaag cgccgaagcg gcacgtcaaa 1559580
caaaaaaact acaaactgcc tccgatttcc gtcaaacagc ccgtcatgct gacgctggta 1559640

```
cagcggcaaa tccgcagcct cttgataaat ccggattggg ctgcatatat agacctgccc 1559700
gattatctgg cgttggacgg tgatttcgcc tgccttgcca atcttgccga atcgattaaa 1559760
aaccatgccg ccgtacccga aaccgctcag gttttagagt atatgcgcgg ctcgccttac 1559820
gaagaaacga taacccgaat cttccattca acgcaccaat cggaagaaat gaacagcagc 1559880
agtgaagaag attgcgagaa tttccaaatc ggcatgaaaa aactgctcaa tgagttaaaa 1559940
tacagccaaa tcgaaacatt aaaacaaaaa agcctgcaat ccggcttaaa tgaaagcgag 1560000
aaaaaacttt tgctgtcgct gctgaccgca aaacaaaatt gaccggcgga ttccgccatc 1560060
cgtaaaccgt tatgccgtct gaaaagcatt caccccggct gcaacaacga cacctgcaga 1560120
acacccatcc ccaaaagcct tcagacggca tcagagtacc ctactctgcc acgccttcag 1560180
gtgcgtccaa acgcaaaccg tcggcatctt accaacagaa agcagacaat gtccagaaac 1560240
caaaatcacg aagaatatca agacgacacc cgtccgttaa gcattgaaga gcaacgcgcg 1560300
cgcctgcgtc agctcatcat catgggtaaa gaacgcggct acatcaccta ctccgaaatc 1560360
aacgacgccc tgccagacga tatgtctgat gccgaccaaa tagacaatat cgtcagcatg 1560420
atttccggtt tgggcatcca agttaccgaa cacgcccccg atgcggaaga catattgtta 1560480
agcgacaatg ccgccgttac cgacgatgat gccgtcgaag aagccgaggc cgccctttcc 1560540
agtgcagatt ccgagttcgg cagaaccacc gaccccgtcc gtatgtatat gcgcgaaatg 1560600
ggacaggtcg acctgctgac ccgcgaagac gaaatcatca tcgcaaaaaa aattgaaaac 1560660
gccctgaaaa atatggttca ggccatctcc gcctgcccgg gatccattgc tgaaatctta 1560720
gaactcatcg aaaaaatccg caaagacgaa atccgcgtcg acgaagtcgt agaagccatt 1560780
atcgacccga atgaagtatt gctcaacgaa ttgggcttgg ggcacttgga aaccacagcg 1560840
cccgagaaac cttccaacga caattcggac gaaaacgaag acgacgaaga atcggaagaa 1560900
gatgcggatg aaatctcggc agccaatctc gccgaattga aacaaaaagt catcggccac 1560960
tttgcccaaa tcgaaaaaga ctacaaaaaa atgattggcc gtttggaaaa acaccacagc 1561020
cggcacaaag actatctcgc ctaccgcgac gcgattgcca acaaactgct ggaagtccgt 1561080
ttcgccaccc ggcaaatcga cagcctcagc agcagcctgc gcgggaaagt agaaaacatc 1561140
cgcaaactcg aacgcgaaat ccgcgacatc tgcctcgacc gcgtccatat ggaacgcgac 1561200
tacttcatcc aaaacttcct gcccgaaatc accaatctag aatggattga agaagaaatc 1561260
gccaaaggca gggtttggag cgacgcgctc gaccgcttcc gccacgccat cctcgaaaaa 1561320
caaaccgagt tggcggatat ggaaaaagaa acccgcattt ccatcgaaga gttgaaagaa 1561380
atcaacaaaa atatggtgtc gagcgaaaaa gaaaccgcag ccgccaaaca ggaaatgatt 1561440
caggcaaact tgcgcctcgt gatttccatc gccaaaaaat ataccaaccg ggcttacaa 1561500
ttccttgatc tgattcagga aggcaacatc ggtttgatga aagcggtcga taagttcgaa 1561560
taccgcagag gctataaatt ctccacctac gcaacctggt ggatccgcca ggcaattaca 1561620
cgctcgattg ccgatcaggc gcgtaccatc cgcattccgg tacatatgat tgaaaccatc 1561680
aacaagatga accgcatctc gcgccaacac cttcaagaaa ccggcgaaga acccgattcc 1561740
gccaaacttg ccgaactgat gcagatgccc gaagacaaaa tccgcaaaat catgaaaatc 1561800
gccaaagagc cgatttcgat ggaaaccccc atcggcgacg acgacgattc gcacttgggc 1561860
gacttcatcg aagatgccaa caatgttgcg ccggccgatg cggcaatgta caccagcctg 1561920
cacgaagtaa ccaaagaaat cctcgaaagc ctgacaccgc gtgaggcaaa agtcctgcgt 1561980
atgcgtttcg gcatcgatat gaacaccgac cacacgctgg aagaagtcgg cagacagttt 1562040
gacgtaacgc gcgaacgcat ccgacaaatc gaggcaaaag cactccgcaa gctgcggcat 1562100
ccgacaagaa gcgaccgttt gagaagtttc ttggacagcg aagacagcaa gctgtaaacc 1562160
aaaaaaccgc aggtttcaaa tacctgcggt tttttcttac acaataaaca acgcttccac 1562220
atatcccaca ctcctatccc gagacctttg caaaattccc caaaatcccc taaattccca 1562280
ccaagacatt taggggattt tccatgagca ccttctttca gcaaaccgca caagccatga 1562340
ttgccaaaca catcgaccgt ttcccactat tgaagttgga tcaggtaatt gattggcaac 1562400
cgatcgaaca gtacctgaac cgtcaaagaa cccgttacct tcgagaccac cgcggccgtc 1562460
ccgcctatcc cctgctgtcc atgttcaaag ccgtcctgct cggacaatgg cacagcctct 1562520
ccgatcccga actcgaacac agcctcatca cccgcatcga tttcaacctg ttttgccgtt 1562580
ttgacgaact gagcatcccc gattacagca ccttatgccg ctaccgcaac tggctggcgc 1562640
aagacgacac cctgtccgaa ctgttggaac tgattaactg ccaactgacc gaaaaaggct 1562700
taaaagtaga gaaagcatcc gccgccgtcg ttgatgccac cattattcag accgctggca 1562760
gcaaacagcg tcaggccata gaagtcgatg aagaaggaca agtcagcggc caaaccacac 1562820
cgagtaagga cagcgatgcc cgttggatca agaaaaacgg cctctacaaa ctcggttaca 1562880
aacaacatac ccgtaccgat gcggaaggct atatcgagaa actgcacatt acccccgcca 1562940
atgcccatga gtgcaaacac ctgtcgccgt tgttggaagg gttacccgaa ggtacgaccg 1563000
tctatgccga caaaggctat gacagtgcgg aaaaccggca acatctggaa gaacatcagt 1563060
tgcaggacgg cattatgcgc aaagcctgcc gcaaccgccc gctgtcggaa gtgcaaacca 1563120
agcgtaaccg atatttatcg aagacccgtt atgtggtcga acaaagcttc ggtacgctgc 1563180
accgtaaatt ccgctacgcc cgggcagcct atttcggact gattaaagtg agtgtgcaaa 1563240
gccatctgaa ggcgatgtgt ttgaacctgt tgaaagccgc caacaggcta agtgcgcctg 1563300
```

ttgccgccta aaaggcagca cggatgcctg attatcgggt atccggggag gattaagggg 1563360
gcgtttgggt agaattagga gatatttggg gcgaaaacag ccgaaaacct gtgtttgggt 1563420
ttcggctgtc gggagggaaa ggaattttgc aaaggtctca tcctgttatt ttcacaaaaa 1563480
cagaaaacca aaaacagcaa cctgaaattc gtcattccca cgaaagtggg aatccagtgc 1563540
gttgagtttc agctatttag aataaatttt gaaactctaa tcgcgtcatt cccacgaaag 1563600
tgggaatcca ggacgcaaaa tctcaagaaa ccgttttacc cgataagttt ccgcaccgac 1563660
aactctagat tctcgcctgc gcgggaatga cgaatccatc catacggaaa cctgcatccc 1563720
gtcattccca cgaacctgca tcccgtcatt cccacgaaag tgggaatcca gtttttttgag 1563780
tttcagtcat tcccgataaa ttgccttagc attgaatgtc tagattcccg cctgcgcggg 1563840
aatgacggga tttgagattg cggcatttat caggagcaac agaagccgct ctgccgtcat 1563900
tcccacgaaa gtgggaatcc agtttttttga gtttcagtca ttcccgataa attgccttag 1563960
cattgaatgt ctagattccc gcctgcgcgg gaatgacgaa tccatccata cggaaacctg 1564020
caccacgtca ttcccacgaa cctacattcc gtcattccca cgaaagtggg aatccagttt 1564080
tttgagtttc agtcattccc gataaattgc cttagcattg aatgtctaga ttcccgcctg 1564140
cgcgggaatg acgaatccat ccgtacggaa acctgcatcc cgtcattccc acgaacctac 1564200
attccgtcat tcccacgaaa gtgggaatcc agtttttttga gtttcagtca tttccgataa 1564260
attgccttag cattgaatgt ctagattccc gcctgcgcgg gaatgacgaa tccatccgta 1564320
cgaaaacctg caccacgtca ttcccacgaa agtgggaatc cagttgcttg agtttcagtc 1564380
atttccgata aattgcctta gcattgaatg tctagattcc cgcctgcgcg ggaatgacga 1564440
attcatccgt acggaaacct gcaccacgtc attcccacga acctacattc cgtcattccc 1564500
acgaaagtgg gaatccagtg cgttgagttt cagtcatttc caataaattg ccttagtatt 1564560
gaatgtctgg attcccgcct gcgcgggaat gacgaattca tccgtacgga aacctgcatc 1564620
ccgtcattcc cacgaaagtg ggaatccagt tttttgagtt tcagtcattc ccgataaatt 1564680
gccttagcat tgaatgtcta gattcccgcc tgcgcgggaa tgacggcgga aatcttgttt 1564740
atattgaatc aaaaaaaacc tgcaccttaa tcagttggcg gtttagtccg acttttgggg 1564800
tgcagatcaa gctttcagac ggtatttcct ttaaaacttc atttcgagcg cgagactgaa 1564860
gttcctgccc ggtgcggcat accttccata gttgctgtcg ccgccgtgcc ggtttgccgt 1564920
gctttccgca gtctggcgca aggattccca agtaacgtag cggtagttgc cgatattgta 1564980
gatagccgcc ctcaaggtca gccgtttttt cagattcaga taggcggaaa cgtctgccgt 1565040
cgaccaagaa gacgacgctc tttttgtcga atatcgtttt tgatcgcctg ccagataagc 1565100
aagctcgtca gggtttttcc ctttggaata ggtcagcata atgtttgcgc cccatttccc 1565160
ctcaggctgg tcgtatccga accccaaaac ataacgcgac ggctgtaccg catccaaagc 1565220
atagctgcgg agggacagtc ccggccggtt ggataccgat ttcggtttga tgcggttgta 1565280
cgccaatgtg gtgtacaaac cttcgggcag tttgccatac acgccgttcc agtcgatttt 1565340
tcccaatata ttaacgcctt gaagcgacat attttgggca ttgtaataat cgcgtatatc 1565400
aatctctgtc aattgtcctg cctgattcgg caatttggtt ttgtgatcgg caacggcaat 1565460
catatcggta taacggttgc ggaagctgct gatttccaaa aagccgaaat cgcccttcca 1565520
ctgcaaaccg atttcccggt tggctgcctt ttccgatttc agggcgggac gctgccagcc 1565580
tttcggataa tcgtgataaa tgtctatccc gaaaagttct tggaatgagg gcgttctgaa 1565640
gccgctggag gcacggtaag acacggaaaa atgccggttc ggtttgaaca agatgccgct 1565700
gttccacgaa cggtcaacat accgcccgct gcggacgagt tcttccgacg tggtgaagtt 1565760
tttccggtcg tacctgccgc ccaagctgaa atcgaaatat ttgccgattg aaaaacggtc 1565820
gttcaaagaa atatggatat tgctgccgtt gatttttctt ggcacgcatt tgcgggaacg 1565880
cagggtttcg atgtagccgc agaccgaccc ttcgacgact tcgggcttac ccaaaagata 1565940
cttatcttga ttgttttcat cgaatcccgt ggattccgaa atccttgccg cattgtggga 1566000
aagctgttcg gggcgggaaa tcgctttgga agcatcgtaa ccgaagccca aagtcagatg 1566060
gtgtttcgtc catttgtttt tcagcgattt ctcaaacgag gcattcaaaa cattgtgctg 1566120
ttcgcggtag tggaaacggt cgctgctgtc gtaggaatac ggtttgtccg ccgacgcgcg 1566180
gcaggatttg tccacagcag gatacacggc gcaattcagc ttcagcgtgt tgttatcggt 1566240
tgccacgccc tgtttgtcaa acgacaacac cgccttatcc gcccaattgt cagaatacgc 1566300
ttcgttttca taacgataca gcaaacccat acggcggcgg cggtgatgtt cgtcaataaa 1566360
tttggtgcgg gaatatttca aacctatgcc cctgaccaaa tttttatcgc ccttccactc 1566420
ttctatattc ggcacaaaat acaagccgtc gcggaaatcg tcgccgtcgt acaccccgct 1566480
cttgtctcta aacttttccg cctcgtccgt accgtaatac tgtttttccg tcatatcgcg 1566540
gatatcgtaa cgctgtttgg tatcctcaaa cacgccgccg acataatgcc tgccgccgaa 1566600
gcggtagccc agcttggcaa gccaagagcc gctgcggtaa tccatcggat cgggcaatat 1566660
cctgccgccg cccgtgtaag cttgggcgga cagattttcg tggcgcgcct gcgcctcccg 1566720
cacctgcgcc tcttcttcag cacttaaagg ctgattttgt tcaatacgtt cttttaccca 1566780
gcggttgagc tggttgttca aatatttccc gtagcccgcc aattttgcca cgggcttgga 1566840
ttcacgctcg ccctctactg agaaaaatgg ctctcttgtc ttgcgtttaa tatcgtatgt 1566900
ctgacggaac gcgtccaaac ggtctatgcc gtattccacc ccgtccgcaa tatcgccgtg 1566960

cgggcgcgtt tcccgccctt ggcgttcggt tcggattaac agcccttccc aaccgtcttt 1567020
gctgaacccc gcgccgagcg acttcataaa ttggcggttt ttactgccgt aggcggtttt 1567080
tgcctgtatc ccccaacttt tgccgtctga aatcaggtct gccgcctctt tggtgcggaa 1567140
ggcgaccgcg ccgccgagtg cgccgctgcc gtgatcggac gaaccggcac ctttgtcgat 1567200
ttccaccgtg ctgatgtttt catattcgat ttcgttgatt gcaccgctgc cgccgcgtcc 1567260
gccgtatccg ctcaacgatc cctgcacggt aaacgcctgt atttgggcaa caccgtcgac 1567320
cgaaaccgcc acacggtttt tatccacgcc gcgtatcgag tagccgccgc tcgcgccgtt 1567380
gccctgttcg acaaccgcca cgcccggatc gtagcgcgtc aggtcgcgga taccgagtac 1567440
ctgttctttg ttcaacgttt ccgacgtttt gacgattttg cccaaaccgg tcgcctcttt 1567500
cgatcgccgt cccactttgg cggcacggac ggtaatctct ttcagggatt gggtctgcgc 1567560
ggcatcaggt gtcgcccccc ccgcttgggc agcataagcc ggaaaagcgg ttgcaatggc 1567620
caaggcagtc agagtcagcg gaaaaccgtg tttcttattc atttttccac ctcctgcata 1567680
tctttcttcg caccgaatac cacgccgaat tggtgtttaa cttcagattc taactgtttg 1567740
ccaacatcaa cttcagcatc aacttcagct tcaacatcaa ctttattttc agtaccttca 1567800
gttataccaa gagatttccc atcattattg aaaataatac cgcccaattc ctccgcctgc 1567860
gggccgtaaa atccccccttc tacacgaaga ttactagctt ggaaggtttt ggggtcggtc 1567920
gaaccatttc ccgaaagatt gatgccgttc tcccgagtgc gtgctgtcgc gtagaaaccg 1567980
ttgccctcaa tcttgccgtt ttcaatatgg aaagcaggtt ctacaccgtt ttcctccgtc 1568040
agcgttccgg aaatcgattt cttgccgaaa tcaacggtaa atactgcttt tgccgcttct 1568100
ttatccgcct gattgtccca ttgaatgggt ttgccgatac gcgcttccca agtgccggta 1568160
tagtgtgctt ctccagtttt cggaatatcc gtttccgccg tgcggatacc tttcaggaaa 1568220
aggtcgatgt tcctgccttt aggggcttcc ggagcgggca ggatgccgtc tgaaccgctg 1568280
ccgccttctt ctgtcggcga ttcttcttcg ggttcttcag cttcatcttc accttctacg 1568340
gcttcgtctt cttcgctgcc ttcgtctttt acggctgcgt cttcggtgcc ttcttcatcg 1568400
tcgatttcgt cttcgccttc ttcgacgcta tcaacgcctg tatcctcttc gtccctctct 1568460
tcgtcctgcg ccttcggttt ggcggcggga cgttcggttt gcatccgtcc gattttcaca 1568520
taggtcagaa aatcgcagca ggttcggatt gtcgttttcc taccatcggc aagctcgatg 1568580
gtttgttctt tgtttaccaa aggaatttca cgcccttcga caagaagttt gtcgggatga 1568640
ccaaaatcgg gcatagagga aatggcaaac tcacggggat ttttatcact tgcctcgtca 1568700
acggaaattt tcagagaatc caagattttg gtgtgttttc cagacgacag ggcaggtttt 1568760
gtatctgctg cgttttctgt ctctgttttt tgtttgcctg cgaatacgcc gaatacgctg 1568820
ttgtcgttgc tgataaaccg tccggcaagc tcttctccgt tatcgccgaa aaaaccgccc 1568880
tcaagccgct gatcggcatc ggtatggaaa aacaaatatt ctttatcagc gtgttgcgtc 1568940
ttcacctcgg tgctaacttt ggcactgccg gtaaagcggt tgccgtccaa tgttgcggta 1569000
atgtcgtaaa tggtcagcgg ttttttgggc tcatttggat tacttttatt ttgcacatac 1569060
tgatttttaa tcagcttgcc attcagggtt ttgttatcaa aatcaaccgt atattcggca 1569120
ggatgctttt ccctgtcgtc ggcatcccta gcctcataag aagttgcccc aatttcatta 1569180
ccataatatg tggtataacc caaatccgta ctggaaaccg ccttacctgt ccgatgacgt 1569240
ttggcatcgg tcatatattg ccagttaccg gaatattgca ccgttcccgc gctcggtaaa 1569300
gattgggaag gacgttctcc ggaataatat acaaaaccgt cataactaaa tcggttaaca 1569360
aactccttac catcagaagt cttttctttt tcattatcct ttccttccgc cctggtaaac 1569420
acatagcccg cacggacaaa ttgatattga tatttttctt cttctttttt cgatgtgata 1569480
accctcacat cagaataccg ttcgttgatt ttctttttaa gtttgtcagc ctgttctttc 1569540
agcgtaccgt ctaaaaacag gatatccttc tctttaagcg gcagatgctc ctctgcctga 1569600
tgcttgtcgg gaatttccgt accgtcttgt ttataggaag caatattccg ccttggcagc 1569660
cgcattgccg caccgacggc gggccggttg accggcgtgg tttctaccga agacccggca 1569720
gggggcggag tgggaacgtc cttagatttg aaggtgacgg ggtacgcggt cggcgttgat 1569780
tcgacaacag gctgcacgcc gaaattgccg ccgatacaag atgctaaaag taagggcaac 1569840
aagacaatgc cgccataatt cggtttacac atccctactt ttcctctatt tgattaataa 1569900
taattatcat tatattaata tgtacagata atatcaagcc gtttttatag tgaattaaca 1569960
aaaatcagga caaggcgacg agccgcagac agtacagata cattccgtca ttcccacgaa 1570020
cctacatccc gtcattccca cgaacctgca ccacgtcatt cccacgaaag tgggaatcca 1570080
gttcgttcgg tttcgcttgt tttaagtttc gggtaacttc tacttcgtca ttcccacgaa 1570140
cctgcatccc gtcattccca cgaaagtggg aatccaggac gcaaaatctc aagaaaccgt 1570200
tttacctgat aagtttccgc actgacagac ctagattccc gcctgcgcgg gaatgacggg 1570260
atttgagatt gcggcattta tcgggagcaa cagaagccgc tctgccgtca ttcccacgaa 1570320
agtgggaatc cagttcgttc ggtttcgctt gttttaagtt tcgggtaact tccacttcgt 1570380
cattcccacg aaagtgggaa tccagttttt tgagtttcag tcatttccga taaattgcct 1570440
tagcattgaa tgtctagatt cccgcctgcg cgggaatgac ggattttagg ttgggggcat 1570500
ttattgggaa aagcagaaac cgctccgccg tcattcccac gaaagtggga atccagttcg 1570560
ttcggtttcg cttgttttaa gtttcgggta acttccactt cgtcattccc gcgaacctac 1570620

attccgtcat tcccacgaaa gtgggaatcc agttcgttcg gtttcgcttg ttttaagttt 1570680
cgggtaactt ccacttcgtc attcccacga acctgcatcc cgtcattccc actaaagtgg 1570740
gaatccagga cgcaaaatct caagaaaccg ttttacctga taagtttccg cactgacaga 1570800
cctagattcc cgccttatat gatgcgctct atcaaagggg cgcattaatt ttcttaacat 1570860
tcccctttga cagccaagtg aaaggggctt ttttatgtca gcagtaaatg taatattttc 1570920
ctgttcttat tggagaatat ttaaaaaatc agattcttgt gttttgtgtt tttatcagtt 1570980
cagacatggc gaaccgcata aactcattaa tcaagagaat ttttcaaagc tttatcaggc 1571040
gttcgattat atagattcgg ttggttcgaa ttttccagtg attatcacaa cggatggttg 1571100
tggtcttttt tgttgatctt taaaagtttg tcaggatttg gctttcggtc gttgaccgtc 1571160
gtacgcgctt tagcgcggaa gacgggaaac ggctgaaagc ccccccccttg actaacaggg 1571220
ggggagcgaa attaaaaacc aattccaaga gtagtgaacg aatgagtgaa gttgaatatt 1571280
tctcacactt tatatcggac ggaaaaggga agcttttaga aattccgcag cgaagaggta 1571340
agcaagacgg ggtttttgtt gattggattt cattcacatt ccatgaagat actttactga 1571400
aagtttccgg ttgcccttta ttttctgatg ctgaatacat gtatgtatta agcagaaagc 1571460
tggaagaaat tctaggtttt ggcataacgc gcaaatgcaa atcaaggggc aacaaattct 1571520
atgaatccat gtataggtta ggttcggatg atgttgatta tggagaggtg catttcggag 1571580
gtcagcgcaa tactgtttta gttgagttga aaggtactgg ttgcagcgtt gcaagtccgg 1571640
gttgggagtt gaggctaaag cagtttctcg atgattcgat aaggacaaga ataacgcgaa 1571700
ttgacctagc acttgatttt tttgatggag agtacacgcc ggatcaggcg ttgttagatc 1571760
acgataatgg tttttttgat aacagcaatc aaaggccgaa atctgaaacg atcggtacgg 1571820
cttggcggaa tgaggacggg agcggcaaga cattttatgt aggtcgcaag aaaaattctc 1571880
gttttgttcg tgtttatgag aaaggcaggc agcttggaga taaagaaagc aaatgggtaa 1571940
ggttcgagat ccagtttaat tatggagata tagaaatacc cttggatatt ttaataaatc 1572000
agggttcgta tttctgtgga gcttttccaa tttgtagaaa atttaaaaat atgccggttc 1572060
ccgaaaggtt tgatcagaga aagaaaaagc ttaatttaac tttcgagcat aaattgcatt 1572120
acgcgaaaaa cgcggttgga aaactggtca atttcatgat tgaaatgggt tttgataata 1572180
gcgaaattgt ggaatcttta aaggcagatt cgggatttcc caaaggatta gaacctgaaa 1572240
aatatgctct ggaaatgtta agggacggtt tgaaacacgg ttttattcat gaacagccgg 1572300
atattgattt ggaaattgaa cttgatgaat tgggggttat tgcttttaaa aattctgaca 1572360
aattcgatag ggaaaaaagg ctttttagtc ctgattatga tgtcgagaaa gaaaggaaat 1572420
atcaggaata tttaagtaaa gtttatcatc aaaatgtaga ttatgattat ttttaaagga 1572480
aatcaaaatg tttaatcaaa ctcaaactgt aacttatcct gcaacttttt tgggagccaa 1572540
aaaattcaaa ggcgaaattg atggctctaa tatcgacact tgttccgtat tggttgcaac 1572600
acctttgccg gcacagtcgg gaaatgctgt tggattcacg gcagcacaaa tgaagttcgg 1572660
ggacagtaag aatttctcaa aattagagaa tctcaaatac ccgtgcgaag ttatggtaac 1572720
ggttgaaatg acttcgacag gtaagggcat ggttccttca ttaattgatt ttcaggtggc 1572780
agaaaagccg aaaggttgat ttatgaaatt tgaagaacgt ttcatagttc aagacttgga 1572840
aacgcatgac tttatttatc ccgatccttt cggtgatgtg gggtttactc aaaatattaa 1572900
atcagcaggt caatttgaaa gctacgaaga tgcgttgaat tcaggcataa atgaaatagg 1572960
cggaggattc cagatatttc agttcttcgt aaaatcggaa taaaagaaaa acaggctcgg 1573020
cgggcggtct gtcaaccttt cacaaagccc gcaacaaagg aaaaatatca tgaaaatgaa 1573080
ccttgcaaca ctaattatcg gctgggtggt ctgtatgttt ctttttcttt tcgcaatcct 1573140
ctattttatc ggctaaaaac gagattcgga aaagacttcg tccggatgaa gcaagtcaag 1573200
aagtcgtctt attttaaata tcaaaaaagg aaaaaaacga tgaacatcgt taaaaaatac 1573260
gctgtaaaag cagccttggc agccggtatc ttcacaccgg ccattgttat ggcagatacc 1573320
tttgatccat ccgcgattgg tacgcaagta gcgaatgtaa tcatgggttt cgtgtcaatg 1573380
gtttccgccg tgggtatggc ggccattacc gtgattcttg caatccaagg cttcaaaatg 1573440
gcttggagca tgattaaatc tgtcaaataa acagagtgaa gaaaaagggg cgtataaatg 1573500
ggctatcgtg tcggcataaa ttgttttgat acaagattgc aggcagacga ctatttattg 1573560
tcgtcccttc ctcctactgt tacccaggac ggaaaaatca tcaggccgga aagggtgggc 1573620
gataaatgga ttttgaacgg aaagccggtt acgttgtctt atccggaatg ttccaatttt 1573680
gagcagataa agcaaggttc ttatgtcggt tcgacggttc taattctgtt tgtagtcatt 1573740
tacggtttca ggcttctgat taattttta aaagacatag gcaaggttgg gactgattga 1573800
tgattataga tttctggttt cttctcggtt tcttcttggc tttgtctgtt gcttggctgt 1573860
tttggtaacg gttggtagaa tcggcttttt agagtgtttt aaaaggtccg aattatgttt 1573920
atttctgaat atcatttagt taaatttcaa actgattcac atatttatag agatttacca 1573980
caagcgttaa tttattatag ggaattgatt agaaaagggg tttttaaaac ttcgttttca 1574040
tttgatattt ttaggaattt ctttcatcgt tatgatagag attttataga aattcaattc 1574100
cctgattctt ctacattatt aattaaatta gatgaagcaa aatgttatgt ttattatcct 1574160
agggcgaaat tttttaaaga ttatcctatg ctttagtttt tttgtatcta aatttgcatt 1574220
ggcatcagta aatgctccgg gtaaatttga tagggttgaa gtttatgatg atggcagata 1574280

tttaggtatt cgaggttcag atgacaaaag aagaagaatt tggaaaggtg tatttgatag 1574340
agaatcggga agatatttaa cttcagaagc tcaagattta aaagttaggc atgtatctac 1574400
tggagcatca agtacgggta aagttagttc ggttgtatct tcatcagttt cccgcgctgg 1574460
cgtattggcg gggtcggca aacttgcccg cttaggcgcg aaattaagca caagggcagt 1574520
tccttatgtc ggaacagccc ttttagccca tgacgtatac gaaactttca aagaagacat 1574580
acaggcacaa ggctaccaat acgaccccga aaccgacaaa tttgtaaaag gctacgaata 1574640
tagtaattgc ctttggtacg aagacaaaag acgtattaat agaacctatg gctgctacgg 1574700
cgttgacagt tcgattatgc gccttatgtc cgatgacagc agattccccg aagtcaaaga 1574760
attgatggaa agccaaatgt ataggctggc acgtccgttt tggaattggc ataaagaaga 1574820
actgaataaa ttaagttctt tggattggaa taattttgtt ttaaatagtt gcacatttga 1574880
ttggaacggc ggagattgtg tggtcaataa aggtgatgat ttcagaaatg ggctgattt 1574940
ttcccttatt cgcaattcaa aatacaaaga agaaatggat gccaaaaagc tggaagagat 1575000
tttatcgttg aaagtcgatg ccaatcccga caaatacata aaggcaaccg gttatcccgg 1575060
ttattccgaa aaagtagaag tcgcacccgg aacaaaagtg aatatgggtc ccgtcacgga 1575120
caggaacggg aatcccgttc aggttgtcgc aacattcggc agggattcgc aaggcaacac 1575180
cacggtggat gttcaagtaa tcccgcgtcc cgacttgacc cccggaagcg cggaagcacc 1575240
gaacgcacag ccgctgcccg aagtatcgcc cgccgaaaac cccgcaaaca acccgaaccc 1575300
caatgagaac cccggcacga gccccaatcc cgaacccgac cccgatttga atcccgatgc 1575360
aaatcccgat acggacggac agcccggcac aagacccgat tcccccgccg ttccgggacg 1575420
cacaaacggc agggacggca aagacggaaa ggacggcaaa gatggcggcc ttttgtgcaa 1575480
attcttcccc gacattctcg cttgcgacag gctgcccgag tccaatccgg cagaagattt 1575540
aaatctgccg tctgaaaccg tcaatgtaga gtttcagaaa tcaggaatct ttcaagattc 1575600
cgcacagtgt cccgcacctg tcactttcac agtgactgtg cttgattcaa gcaggcagtt 1575660
cgcgttcagc tttgagaacg catgtaccat agccgaacgg ctaaggtaca tgcttctcgc 1575720
ccttgcttgg gcggttgccg ccttttttttg tatccgcaca gtatctcgtg aagtctagca 1575780
ggcgcagcac cgccgggctt cagtaacttg taccaaggca gggggaggac gtccagaaag 1575840
atttgtaaag acggctttat cgtctttata aatctttttg gataccccctt gccgccccgc 1575900
caaaagaaca cattctgccg caagggcagg tggtaaggcg cgcgcctttt gcgccgttcc 1575960
ccctgccccc gcggcgtcgc aagtgagact gggggtgcgg gggctagtcc ccgcaaagcc 1576020
tttcagcttc ggaagccacg gccgaaaggc aggcgcagca ctgccggtct gagcggaagc 1576080
caggctacag gcaggcgcag caccgccgag ctaggcggaa gccaggctac aggcaggcga 1576140
agcaccgccg gttgggcgga agccacggcc gaaaggcagg gcgaagcacc gccaggctta 1576200
ggcggaagcc acggccgaaa ggcaggcgaa gtaccgccgg tctgggcgga agccatggta 1576260
aaaggcaggc gaagcaccgc cgggcttcag taacctttgt tcaggcaggg ggaggatgtc 1576320
cgtaaagaat cgtaaaggcg gggttttttc gcctttatga ttctttttgg ataccccttg 1576380
ccgccccgcc aaaagaacac attctgccgc aagggcaggt ggtaaggcgc gcgccttttg 1576440
cgccgtcccc atgcccccgc ggcgtcgcaa gtgagactag ggggtgtggg ggactagtcc 1576500
cccgcaaagc gttcagcttc ggaaactttg gccgaaaggc aggcgaagca gcgcactttg 1576560
cgacgaatgt cgcaaatagc cgagaagcgc ggggggattg gcgataagcg cgaggggggt 1576620
gtccccacag cgccgccgcg ccgcgaatgc ggcgcaaaat ctttcagatt aagaaacatt 1576680
tgtttaatga ggcaaccgtg ccttttaaga aagggatagc aaatgaaatt gttggccgca 1576740
ttgattccgc ttttgatgag cgtggcaggc cgtatattga ctgcattagg cttgatggcg 1576800
gtaacctatt caggggtgga tagattggta gcccattttc agcaggcgat aaccaatagc 1576860
ataacgggcg cgcctcaagc gatgttgcag ctttttttata taagcggcgg tggaaccgtt 1576920
cttaatatcc tgtttggcgc gatcgccttt attctgtcat tcaaacaaat gacaaaacta 1576980
gcaacctcaa tcgggaagaa aaaataaatg gcagagatct gtttgataac cggcacgccc 1577040
ggttcaggga aaacattaaa aatggtttcc atgatggcga atgatgaaat gtttaagcct 1577100
gatgaaaacg gcatacgccg taaagtattt acgaacataa aaggcttgaa aataccgcac 1577160
acctacatag aaacggacgc aaaaaagctg ccgaaatcga cagatgagca gctttcggcg 1577220
catgatatgt acgaatggat aaagaagccc gaaaatatcg ggtctattgt cattgtagat 1577280
gaagctcaag acgtatggcc ggcacgctcg gcaggttcaa aaatccctga aaatgtccaa 1577340
tggctgaata cgcacagaca tcagggcatt gatatatttg ttttgactca aggtcctaag 1577400
cttctagatc aaaatcttag aacgcttgta cggaaacatt accacatcgc ttcaaacaag 1577460
atgggtatgc gtacgctttt agaatggaaa atatgcgcgg acgatcccgt aaaaatggca 1577520
tcaagcgcat tctccagtat ctatacactg gataaaaaag tttatgactt gtacgaatca 1577580
gcggaagttc ataccgtaaa taaggtcaag cggtcaaagt ggttttacac tctgccagta 1577640
atagtattgc tgattcccgt gtttgtcggc ctgtcctata aaatgttgag cagttacgga 1577700
aaaaaacagg aagaacccgc agcacaagaa tcggcggcaa cagaacagca ggcagtactt 1577760
ccggataaaa cagaaggcga gccggtaaat aacggcaacc ttaccgcaga tatgtttgtt 1577820
ccgacattgt ccgaaaaacc cgaaagcaag ccgatttata acggtgtaag gcaggtaaga 1577880
acctttgaat atatagcagg ctgtatagaa ggcggaagaa ccggatgcgc ctgctattcg 1577940

catcaaggga cggcattgaa agaagtgacg gagttgatgt gcaaggacta tgtaaaaaac 1578000
ggcttgccgt ttaacccata caaagaagaa agccaagggc aggaagttca gcaaagcgcg 1578060
cagcaacatt cggacagggc gcaagttgcc acattgggcg gaaaaccgta gcagaaccta 1578120
atgtacgata attgggaaga acgcgggaaa ccgtttgaag gaatcggcgg gggcgtggtc 1578180
ggatcggcaa actgaagaaa acggcaagag agaaaaaaga cccgtaaacc gtttgaatat 1578240
agacggttta cgggtctttg tttcgcgcaa agcaagggct aaggcagtca ggcagcaaat 1578300
cccgcaatgt attaaaacag acgcgtagaa atgccggctg cctttatcca tcctcgaaat 1578360
tgaatatcat cctagccgta tcaaggctgt ataaataagg aaaataccaa tgaatataat 1578420
cgggctggac atctcaaagg acaccataga cgcaacattg cataaaacaa acggaagtat 1578480
ccattacatt aaatttaaga ataatgatga tggattaaaa cagtttagat tgtggataaa 1578540
gggaaacaga atcagaaaag tctatatcgg catggaggca acaggcatct attacgaaaa 1578600
ggcagcagat atgctttctt cctactatac tgtttacgtt attaatccct taaaaatcaa 1578660
ggactacgga aaaagcaggt ttaaccgtac caaaaccgac aaagcagatt caaacctgat 1578720
agcagactac ataaaaaggc atcaagatac attgataccg tatcagatac ccaaaaacaa 1578780
agcactgcaa aaactgatta accttaaaaa tcaattacat caacatcaga agcaaattaa 1578840
aaaccgtctt catagcactg aagaagactt cataaggaac atacatcaag acttgataga 1578900
taccatacag gacaagatgg aacaggtaaa aatagccata tccgaacaaa tcaaaaaaca 1578960
aacggacaat aaccattacc gcaatcttca aaccatcccg agcataggca aagacaccgc 1579020
atcagttctt tatgcgcaac tgacagaaaa acattttaaa accgcaaacc agtttgtatc 1579080
ctatgccgga ttaaatcccg ccatcataca atcagggaca agcgtaagag gtcggggcag 1579140
attgagccga tacggaaaca gacgattaaa aagtacgctg tatatgcccg ccctttgtgc 1579200
ttaccgtttt aacgcatttc cgaaattaat aaataatctg aaaaaagcgg gtaagccaaa 1579260
gatggtaatc atcgttgcca tcatgcgcaa actggcgaag ctcgcctatt acattgttaa 1579320
aaccggccag ccttacgatg cggaaagaca ccgattgaat caataaaatt caacaaaatt 1579380
aaacggttac gcgaatatat ttgtgtaacc gtgcatttgc atatcgtaaa taaacgtaaa 1579440
taaaaataac aatataaatc agtatattgc aactttgttt tttattttgt gttgacgggc 1579500
aacatatcat ctgcgcggga atgacgggat ttgagattgc ggcatttatc gggagcaaca 1579560
gaagccgctc cgccgtcatt cccacgaaag tgggaatcta gttcgttcgg tttcgcttgt 1579620
tttaagtttc gggtaacttc cacttcgtca ttcccacgaa agtgggaatc cagtttttg 1579680
agtttcagtc attcccgata aattgtctta gcattgaatg tctagattcc cgcctgcgcg 1579740
ggaatgacga atccatccat acggaaacct gcatcccgtc attcccacga acctacattc 1579800
cgtcattccc acgaaagtgg gaatccagtt ttttgagttt cagtcattcc cgataaattg 1579860
ccttagcatt gaatgtctag attcccgcct gcgcgggaat gacgggattt taagttgggg 1579920
tcatttattg gaaaaagcag aaaccgctcc gccgtcattc ccacgaaagt gggaatccag 1579980
tttttgagt ttcagtcatt tccgataaat tgccttagca ttgaatgtct agattcccgc 1580040
ctgagcggga atgacgaatc catccgtacg gaaacctgca ccacgtcatt cccacgaacc 1580100
tgcatcccgt cattcccacg aaagcgggaa tccagttcgt tcggtttcgc ttgttttaag 1580160
tttcgggtaa cttctacttc gtcattcccg cgcaggcggg aatccagtgc gttgagtttc 1580220
agctatttag aataaatttt gaaactctaa tcgcgtcatt cccacgaaag tgggaatcca 1580280
gtttttgag tttcagtcat ttccgataaa ttgccttagc attgaatgtc tagattcccg 1580340
cctgcgcggg aatgacgaat ccatccatac ggaaacctgc accacgtcat tcccacgaaa 1580400
gtgggaatct agttcgttcg gtttcgcttg ttttaagttt cgggtaactt ccacttcgtc 1580460
attcccacga aagtgggaat ccagtttttt gagtttcagt cattcccgat aaattgtctt 1580520
agcattgaat gtctagattc ccgcctgcgc gggaatgacg aatccatcca tacggaaacc 1580580
tgcatcccgt cattcccacg aaagtgggaa tccagctttt tgagtttcag tcatttccga 1580640
taaattgcct tagcattgaa tgtctagatt cccgcctgcg cgggaatgac ggattttagg 1580700
ttgggggcat ttattgggaa aagcagaaac cgctccgccg tcattcccac gaaagtggga 1580760
atccagttcg ttcggtttcg cttgttttaa gtttcgggta acttccactt cgtcattccc 1580820
gcgcaggcgg gaatccagtg cgttgagttt cagctattta gaataaattt tgaaactcta 1580880
atcgcgtcat tcccacgaaa gtgggaatcc agctttttga gtttcagtca ttcccgataa 1580940
attgccttag cattgaatgt ctagattccc gcctgcgcgg gaatgacgaa tccatccata 1581000
cggaaacctg caccacgtca ttcccacgaa cctgcatccc gtcattccca cgaaagtggg 1581060
aatctagttc gttcggtttc gcttgtttta agtttcgggt aacttccact tcgtcattcc 1581120
cgcgcaggcg ggaatccagt ttcttgagtt tcagtcattt ccgataaatt gccttagcat 1581180
tgaatgtcta gattcccgcc tgcgcgggaa tccagtgcgt tgagtttcag ctatttagaa 1581240
taaattttga aactctaatc gcgtcattcc cacgaaagtg ggaatccagt tttttgagtt 1581300
tcagtcattc ccgataaatt gccttagcat tgaatgtcta gattcccgcc tgcgcgggaa 1581360
tgacggcgga gcggtttctg ttttttccgg taaataccca caagctaaaa tcccgttatt 1581420
ttcacaaaaa cagaaaacca aaaacagaaa cctgaaattc gtcattccca cgaacctaca 1581480
tcccgtcatt cccacgaaag tgggaatcca gtttttgag tttcagtcat ttccgataaa 1581540
ttgcctcagc attgaatgtc tggattcccg cctgcgcggg aatgacggcg gagcggtttc 1581600

tattttttcc ggtaaatacc cacaagctaa aatcctgtta ttttcacaaa aacagaaaac 1581660
caaaaacaga aacctgaaat tcgtcattcc cgcgcaggcg ggaatctggt tcgttcggtt 1581720
tcgcttgttt taagtttcgg gtaacttcca cttcgtcatt cccgcgcagg cgggaatcca 1581780
gtgcgttgag tttcagctat ttagaataaa ttttgaaact ctaatcccgt cattcccacg 1581840
aaagtgggaa tccagttttt tgagtttcag tcattcccga taaattgcct tagcattgaa 1581900
tgtctagatt cccgcctgcg cgggaatgac ggctgcagat gcccgactgt ctttatagtg 1581960
gattaacaaa aatcaggaca aggcgacgaa gccgcagaca gtacaaatag tacggaaccg 1582020
attcacttgg tgcttcagca ccttagagaa tcgttctctt tgagctaagg cgaggcaacg 1582080
ccgtactggt ttttgttaat ccactatact gtaatcaggg atgctcagtt cgtcgaaacg 1582140
gcaaaacagg ttgaagtcga tgcgggtgat gaggctgtgt tcgagttcgg gatcggagag 1582200
gctgtgccat tgtccgagca ggacggcttt gaacatggac agcaggggat aggcaggacg 1582260
gccgcggtgg tctctaaggt aacgggtttt ttgacggttc aggtattgtt cgatcagctg 1582320
ccaatcaatc acccggtcca acttcaatag cgggaagcgg tcgatgtgtt tggcaatcat 1582380
ggcttgggcg gtttgctgga agaaggtgct catgagaaat ctcctaaatg tcttggtggg 1582440
aatttagggg attttgggga attttgcaaa ggtctcaact tgagtttcac gccccgctta 1582500
acaatattca gttggtaaat attagataaa accataaaaa ttaaattgat ggcttttata 1582560
atccccgatt tgcgaaaatg ccgtctgaaa gtcttcattc aggctttcag acggcatttt 1582620
gatcatcaag taacgcttta tcaggctttt ttattgttca acgcagcttt gacaaacgcg 1582680
gtgaacaaag gatgcccttt gcgcggattg gaggtaaact cggggtggaa ctggcaggcg 1582740
aagaaccaag gatggttcgg cagttcgatg gtttcgacca agcgttcgcg tccggcagat 1582800
acaccgccga tgaccaaacc tgcctgttcc agtgtaggaa cgtagttgtt gttgacttcg 1582860
tagcggtggc ggtggcgttc gcggatatgt ccgctgccgt agattttggc ggcgaggctg 1582920
cctgctttca attcgacttc ttgcgcgccc aaacgcatcg tgccgcccaa atcggtggat 1582980
tcgtcgcggg tttcgacgct gccgtcggca gtttgccatt cgtcaatcag ggcaacgact 1583040
ggcgcggcgc atttgaggtc gaactcggtg gaattcgcgc ctttcaagcc tgccacgtcg 1583100
cgggcgtatt cgatcagcgc aatctgcata ccgaggcaga tgcccaagta tggcacgttg 1583160
ttttcgcggg cgtagcgcac ggcggcgatt ttgccttcca caccgcgcga accgaaaccg 1583220
ccgggaacga ggatggcgtc catgtcttta agcatggaaa cgtcgccctt gtttttctcg 1583280
atgttttcgc tgtcgacaaa ggtaatctgc acgtcggttt cggtgtgaat gcctgcgtgt 1583340
ttcaaggctt cgatcagcga tttgtaggac tcggtcaaat cgacgtattt gccgaccatg 1583400
gcgattttga cggtgtgttt cgggttttgg atggcgtgga cgattttttt ccacgcggtc 1583460
aaatccgcct gctgcacatt aagctgcaac tgctcggtaa tgatgttgtc gatgccttgg 1583520
tcgtgcagca tttcggggca ttcgtagatg ctgtccacat cgtagctgcc gacaatcgcg 1583580
cgttcttcca cgttgcagaa caaggcgatt ttgcggcgtt cgtccgcagg cattgtcctg 1583640
tccatacggc aaatcaggat gtcgggttgc aaaccgatgc tcaacatttc tttaacggtg 1583700
tgctgggtcg gcttggtttt gatttcgcct gcggcggcga tgtaggggac gtagctcaag 1583760
tgggcaaaca aggtgttgtt gcgccccaac tggcttcgca tctggcggat ggcttccaaa 1583820
aacggcagcg attcgatgtc gccgaccgtg ccgccaattt cgacaatcgc cacatcgtaa 1583880
cctgccgcgc cttcgtggat gcgtcgtttg atttcgtcgg taatgtgcgg aatgacttga 1583940
accgtaccgc cgaggtagtc gccccgtcgt tctttggcga taacgttttc gtacacctgt 1584000
cccgtgctga agctgttgcg gcgggtcatc gtggaatcga taaagcgttc gtagtgtccc 1584060
aagtcgaggt cggtttccgc gccgtcgtcg gttacgaaca cttcgccgtg ttggaacggg 1584120
ctcatcgtgc cgggatcgac gttgatataa ggatcgagct tgagcatggt aacgttcaag 1584180
ccgcgcgatt cgaggatggc ggcaatagaa gcggcggcga tacctttacc cagtgaggag 1584240
acaacgccgc cggtgacgaa aatgaatttg gtcataatga aatacccgta ttggaatgcg 1584300
tgattttaac gtgaagcgcg cggttctggc aaacggacgg atgccgtctg aacgatggac 1584360
ggctgttttc agacggcatc ttttctttat ttcccggtac tttgccgcaa ctcgcggcgc 1584420
aggattttgc cgacgttgga cttgggcaac tcgtcgcgga attcgatatt tttcggtact 1584480
ttatatgcgg ttaattcggt gcggcaaaaa gcgataagtt cttctttggt caaagacggg 1584540
tctttttga cgacgaatac tttgagtgcc tcgccggttt tttcgtcggg aacgccgata 1584600
caggcgactt ccatgacttt gccgtgatgc gcgatgactt cctcgatttc gttcggataa 1584660
acattgaatc cggaaacaac gacgaggtct ttcttacgat cgaccagctt caaccagcct 1584720
ttttcgtcca tgacggcaat atcgccggtt tccaagaagc cgcgcgcgtc tatggctttg 1584780
gcggtttctt cggggcggtt ccagtagcct tgcatcactt gagggccttt tacccacaat 1584840
tcgcccggct gcccgacggg gacttctttg ccgtttgcgt cgcgcagttc gacttcggtg 1584900
gacgagacgg gcaaaccgat gctgccgctg tatgattcga tgtttaaggg gttgcagcac 1584960
acgccggggc tggcttcggt cagaccgtag gcttcgacga tgggcgtgcc ggtgattttt 1585020
ttccattttt cggcaacggc tttttgggtc gccataccgc cgcccaaagt cagccgcaat 1585080
tctgaaaaat cgacttcggc aaaatcagga cggttaacca tcgcgttaaa cagcgtgttc 1585140
acgccgataa atacattaac ccgctgtttt ttcagttctc cgataaagcc tttcatatcg 1585200
cgcgggttgg taatcaggat gattttcgag ccggcattgg caaaaatcat cagattcacg 1585260

gttaaggcaa aaatatggta cagcggcaag gcggcgataa cggtttcttt gccctcgcgc 1585320
aactggtttt taatccattc ttttgcctga agcatattgg cgcagatgtt gccgtgactc 1585380
agcaccgccc ctttggcaac acctgtcgtg ccgcccgtgt attgcaacag cgcggtatct 1585440
tcgcggttta atgcgacagg ttggaaaacg tgcttcgccc cttctttcaa tgccgtctga 1585500
aaggaaacgg tttcccgaat acggtattcg ggcaccattt tcttgatttt ccggatgacg 1585560
aaattgatca gcgaaccttt aagcagcccg aacatttcgc cgacggaggc tacgatgacg 1585620
tgtttgatct gcgtgcgcgg cagcaccagc tccagcgtgt tggcgaaatt ttccaaaacg 1585680
atgatggcgg tcgcgccgct gtctttcaac tgatgctcca gctcgcgcgg ggtatagagc 1585740
ggattggtgt tcaccgctac caaacctgcc tgcaaaatgc cgaaaagggc aaccggatat 1585800
tgcagtacat tgggcaacat tattgccacg cgctctcctc gaggcaattt aaggacgttt 1585860
tgcagataag aagcaaaatc tgttgccagt ttgccggttt cggcataagt cagcgtctta 1585920
cccatgtttt gaaaagcagg ttggtcggca aatttttcca cgctttggcg gaatacgtcg 1585980
ctgacggaat tgtattgcgt gatgtcgatt tcggcactga cgcccttctc gtagctgtct 1586040
aaccagattt tttccatagg tatcggtctt taaagtggaa ttgagcggaa caatgccgtc 1586100
tgaaaaccgt ttcagacggc attaccttta tcgtgtgatg atgacgggtt tgtcggtcgt 1586160
ttggatgata ccgccgccca aacagatatc gccgtcgtac agcacggcgg actgacccgg 1586220
cgtaaccgcc cattgcggtt cgtcaaacac cagctcggcg gtttcatcat ccaaatagcg 1586280
caactcacaa ggcgcgtccg ccatacggta acgcgttttg caggtatagc gtcctgcctt 1586340
cgggcgttcg ggcagcgtga aactcaaatc gttcatcaca aggctgcggg tataaagcag 1586400
cggatggtcg tgtccttgca cgacaatcag ttcgtttttc gtcaaatctt tagccgcaac 1586460
aaaccacggt tcgcccgcgc cgccaatgcc caaacctttg cgctgtccga gcgtgtagaa 1586520
catcagcccg acgtgttcgc cgacggtttt cccttcgggc gtaaccattt taccattgtc 1586580
ggtcggcagg tatttctgca gaaactcgcg aaacgggcgt tcgccgatga aacagatgcc 1586640
cgtgctgtct tttttagcgg cggtcggcag tttgaactcg gcggcaaggc ggcgcacttc 1586700
gggtttttcc aaaccgccca acggaaaaat cgcgcgctcg agttggaaag gcttgaggcg 1586760
gtagaggaaa tagctttggt ctttgtttcg atccaaacct ttgagcaggt aatgcacgcc 1586820
gttgcgaact tctttgcgcg catagtggcc ggtggcgatg gtatccgcgc cctgccctac 1586880
ggcgtagtcc aaaaagcatt tgaatttgat ttcggcgttg cacaacacat ccggattcgg 1586940
cgtgcgcccc gcactgtatt cctgaagaaa ataagcaaag actttgtctt tatattgcgc 1587000
ggcgaaatta acgatgtcga tatcgatgcc gataatatcg gcaacggcga tggcatcgaa 1587060
cgaatcctgt ttgatgctgc aatattcgtc gttgtcgtcg tcttcccagt tctgcatgaa 1587120
cacaccgcgc acttgataac cctgctgctt gagcagggcg gcggttacgg aagaatcgac 1587180
accgccggag agcccgacga tgatattgga agggtttgct gtcgtattca tgcgtagaat 1587240
atggttggaa acggcggttt ttaaaggcgg attttaacac attttaaagg cgggcataaa 1587300
aatgccgtct gaaagcccgg gctttttcag acggcatttc aaacattttc agcagattag 1587360
tgctgatgcg cttcgccgtg gtgatgaccg tggttcattg ccggcatcgg cgcgattttg 1587420
acttccagtt ggacggtttg cgctttggcg tttttaaatt tcagggtaac gggaatttta 1587480
tcgccctctt ttaattgttt tttcaaaccc ataaacatca catgatagct gccgggtttg 1587540
agttcggtaa cggatttcgc ttccaaaggc acgccgcctt cgacttcgcg catccgcatc 1587600
acgccgttgt cgttgatgtg ggtatgcact tcgacgcggt cggcaacggg gctgcttccg 1587660
ccgagcaaaa agtcttgttt ggcttcgtcg ttgtggattt tcatgaacgc gccgcctatt 1587720
ttcatacctt cgacggtggt gcgcgcccag ccgtcctcaa cgtggactcc ggcggcggaa 1587780
accgcgcctg ccaaacctgc catcatcacg gccgccaata attttttcat ctttctgctc 1587840
cttataatat cagacgggga atgtgcttaa tcttatagcg gattaacaaa aaccagtaca 1587900
gcgttgcctc gccttagctc aaagagaacg attctctaag gtgctgaagc accaagtgga 1587960
tcggttccgt actatttgta ctgtctgcgg cttcgtcgcc ttgtcctgat ttttgttaat 1588020
ccactataca tacaaatact gcctggaaat ttgatgtaga ttaagtgaat aataaatacc 1588080
acatactaat cctaaaggat tacaaatcct gctgcaagcg ttttacccga acagggcaga 1588140
cagccaaacc gccgccaaca tcagcatcgc gaacaattgt gcggcagaac ctgcgtcttt 1588200
ggcgagtttg gccagctcgt gtttttcggt cgaagtatga tcgacggcag cttcgacggc 1588260
ggtgttgaac agttcgacaa tgaccgacac aaaagacgcg ataatcaacg gcaggcggac 1588320
ggcggtttcg gaaacccaaa aaaatgccgc gcacaccagc agtacgttca gccacaaaac 1588380
ctgacggaat gccgcttcgt aacggtaggc ggcggcgatg ccgtctatcg aatagccgaa 1588440
tgcgttaatg acgcgcctga tgccgccttt gccttttttt tctgccgcgt aggaggaagg 1588500
ttccatcggt atcctttcaa aatgttctca atatagtgga ttaacaaaaa cctgtacggc 1588560
gttgccccgc cttagctcaa agagaacgat tctctaaggt gctgaagcac cgagtgaatc 1588620
ggttccgtac tatttgtact gtctgcagct tcgccgcctt gtcctgattt ttgttaatcc 1588680
actatatata ccgtctgaaa acggggcggc ggggtgtccg tacggtatta agcgtatccc 1588740
tgccggctga gagaaaaccc tgcctgccca atcaaaccag gcggttgtga agcaaaagcc 1588800
tttcagacgg catcggttta acgtaccgac cacgcggcaa cggcatcggc aaacattgcc 1588860
gccacatcga aacctttttg tttcataatt tcttggaatc cggtcgggct ggttacgttg 1588920

acttcggtca ggttgctgcc gataacgtcc aaaccggcca gcaggatgcc gcgccgtttg 1588980
agttcggggg cgagcgtttc ggcaatttcg cggtcgcgtc cgcccaattc ctgcgccacg 1589040
ccgcgcccgc ctgccgcaag gttgccgcgt gtttcgccgt tttgcgggat acgcgccaaa 1589100
gcatagggga cgacttcgcc gccgataatc aggatgcgtt tgtcaccgtg tacgatttcg 1589160
ggaatgtagc gttgcgccat aatggtgcgg gaatcaagct gcatcagggt ttcgaggatg 1589220
ctgccgatgt tggggtcttt ttcggtcagg cggaaaattc ccataccgcc catgccgtcg 1589280
agcggtttga tgatgatgtc gccgtgttct ttcaaaaatg tgcggacatc ggcggaacgg 1589340
gtcgttacca gcgtgggcgc gataaagcgg ctgaagttca aaatcgccag tttttcatta 1589400
aagtcgcgca tcgcctgtcc gctgttaaag accttcgcgc cctgctgttc cgccagcgtc 1589460
agtaattggg tggcgtagag gtattgcata tcgaacggcg gatcggtacg cataatcacg 1589520
gcatcaaatg cttccaatgc cgtctgaact ttgtcggcag atttgaacca cgcatgatca 1589580
tcatcgtttt ttgcacccaa aaattcaaat gccgatgcct gcgccgttac caaaccgccg 1589640
tttacagaca attccccgct caatgtgtga aacagccgcc agccgcgttt tgccatttcg 1589700
cgcatcatcg cgtaggtggt gtctttatag gttttgaaac ttgccatcgg gtcggcgata 1589760
aagaggactt tcatcatatt tcctttccgg tgtgccgaat gtgccgcatt tcgcgggtaa 1589820
aggagaaatt ccgcccgaac aatattcaga cggcagggat ggggttttac ttaggctgcc 1589880
aagagtcttt cagcgttacc gtgcggttaa acaccggcgt gtctttgccg tggtctttac 1589940
ggtcggttac gaagtagccg atacgctcga actgccaacg gctttctgcc ggcaaatctt 1590000
tggcggcagg ttcggcgtag gcggtgattt ccttgacgga ttccggattg aggaaatcgg 1590060
tgaacggcag gtattcgccg tcttcgccgc gcacggcatc gggacgctcg acggtaaaga 1590120
ggcggtcgta cagacggact ttgatttcgg cggcgtgttc ggcggaaacc caatgaatca 1590180
cgcctttaac tttacggcct tctggatttt tgcccaaggt gtcgtggtcg atgctgcatt 1590240
tgagttcaac cacattgcct gcttcgtctt tgacgacttc atcgcacttg atgacatagc 1590300
cgtggcgcaa gcgtacttcg ccgccgggaa tcaggcgttt gaagcctttg ggcggatttt 1590360
cggcaaagtc gtcggcttca atatagatgg tttgggaaat aggtacttcg cgctcgccca 1590420
tttcctcgtg gttcggatgg aacgcggcac ggcggctttg ggttctgccg gtttcaaagt 1590480
tggtcagggt cactttgagc gggttcaaca ccgccatcag gcgtggggcg gaattttcca 1590540
actcttcgcg aatcgcgcct tccaacacgc tcatatcgac gatgttttca gatttggaaa 1590600
taccggcgcg tttggcaaac aggcgcagcc cttcgggcgt gtagccgcgt cggcgcatac 1590660
cggaaatggt cggcatacgc ggatcgtccc agccggaaac gtgtttttcc acaaccaact 1590720
gattcaattt ccgtttggag gtaatggtgt acaaaagctc caaacgggaa aactcgtatt 1590780
ggcgcggacg ggtggcatgc ggcgcaggaa tgttgtccaa cacacagtcg tacagcggac 1590840
ggtgtgcttc gaattcgagc gtacacaagg aatgcgtgat gccttcgatg gcatcggaga 1590900
tgcaatgcgt gtagtcgtac atcgggtaga tacaccattt gtcgccggtg ttgtggtgat 1590960
gggcgcggcg gatgcggtag atgacggggt cgcgcatatt gatgttgccc gatgccatgt 1591020
cgattttcag gcgcagggtt ttgctgccgt cggggaactc gccgtttttc atgcgtgtga 1591080
acaggtcgag gttttcttcg acgctgcggt cgcggtaagg gctgttttta cccgcttcgg 1591140
tcagcgtacc gcggtattcg cgcatttctt cgggcgtcaa atcatcgaca tacgctttgc 1591200
cgtctttaat caaaccgacg gcgtagtcat aaagctggtc gaaatagttg gaagcgaaac 1591260
gcggctcgcc cgcccaatgg aaaccgagcc actcgacatc ttctttgatg gcgttgacgt 1591320
attcgtcgtt ttctttttcg ggttggtat cgtcaaaacg caggttgcac aagccgtcgt 1591380
aaatatacgc caaaccgaag ttcaggcaga tggatttggc gtgtccgatg tgcaggtagc 1591440
cgttgggttc gggcgggaaa cgggtttgga cagctgtatg tttgccgctt tcgaggtctt 1591500
cttcgatgat ggtgcggata aaatggttgt ccgcaaattg gtctttattg agcatagttt 1591560
tctttgaaca gatggcttca gacggcattg gaatgattcc gtatgccgtc tgaagcggtt 1591620
tgggaatgtg tttattgtac ccgacttgcg cgctttgaca tagcgttcag acggcatcgg 1591680
caatcaagca ttccaccccc gcctctttca gcatcttctg catcgcggta tcgggcagcc 1591740
ggtcggtaaa tactttgtca aacgccgtaa tgtcgccgag cctgaccagc gcgttgctgc 1591800
ggaatttact gtggtccacg ccgaggaagc ggacgcgcgc attggcaatc atcgcctgca 1591860
tcacgctgac ttctttgtag tcgtcgtcca aaagcgaacc gtcgctttcc acgccgtgcg 1591920
tactcatcac ggcataatcg actttgaact ggttgataaa atcgacggtt gccacgccgg 1591980
taataccgcc gtccaaaggg cggacgactc cggaagtgat gatgaccgta taatccgtcc 1592040
gcgccgaagc aatcgaggcg gcgtggatat tgttggtaat caccctcagg ctgccgcgcc 1592100
gcctgaccag ctccgacacc acggcctcca tcgtcgtgcc gatactgaca aacagcgacg 1592160
aaccgtcggg gatgtgttcc gcaatcagcc gggcaatggc gttttttttcg ttttgacacc 1592220
gggtttggcg gtcggcgggc aggccctccg gcaagtttcc gcccgaagat gcgccgccgt 1592280
gatggcgttt caggctgccg acctcctcca actcgcggat gtcgcggcgt atcgtctgcg 1592340
gggtaacgtc caatgcggcg gcaagctcgt ccaccgacat aaactgatgc cggcggacaa 1592400
ggcttaaaat ctctccgtgc ctttggattt tcggcttcat cgttttctgc ctccttgcat 1592460
cgggatgccg attttaccgc gttcaaccca aagcggaaaa caccaccatc agaaacgggg 1592520
cggcgatatt gaccaccacg ccgaagctga ccgctaccgg cacgacttcc aaaccgcccg 1592580

caccctgaat cacgggcaat gtaaaatcca tactggtcgc accgccaacc cccaccgccg 1592640
catctggaaa acgcttcatc agcagcggga taaatgccag tgcaaacagc tctcgtgcca 1592700
aatcgttcag cagcatgatg ctgccccata ccgcgccgta agcctcggtc atgaccaaac 1592760
ccgagaggga ataccaaccg aagccggaag ccatcgccaa acctttcgtc cacgacacac 1592820
cgtctgtcga tgcggcaaac agcagcccgc ccgaaagaga tgaaagcata aaccagaccg 1592880
acaaccgaat accccctgcgg ttgaccaaaa cctgccgcaa cgatacgccg ctgcttttga 1592940
gctgtacgcc gatgaggaac accagcagca tcagacaata catgcccgcg ctttcagacg 1593000
gcatccaaat atcgcgcatc agtttgccga atgcaaatcc gagcagcacg catccgagct 1593060
gccccacact gcccgacacg ccgaccgaaa cgcccttccc tttccccttt atccgccacg 1593120
ggaataactt tcccaacact gccaaagcaa gcaggttcgc cccgaccgta caaacaaaca 1593180
gccacagaac cgtcaacgcc atatcgtcca accgcgaacc caaatcctcc acgcgcgaca 1593240
acgagacgcc gatcagcagc agcacagcat acaccaagac cgatagcacc ttatccaaag 1593300
cgggcaggta aggcttgggc acacggataa aaaatccggc aaacatcggt atcaataccg 1593360
aaagcaacgt catcaggctg tccatctact gctctccttt attgccgcat gatatgtgcg 1593420
gtttaaaaat tgccgtctga aaattgcaga tacccgcatc catatttcag acggcatcag 1593480
gttcgccatt aaaaaaccgc ctgaaggttc aggcggctta tccgctccgg cattcaatct 1593540
tccaaagtct tttccaaacg ctccatacag ttgcccaaat ggcggcgcag gattttgacc 1593600
acgcggttgc gcctgcccgc cagcagcagg tcgaggattt cgcggtgttc ggaatgcgta 1593660
tgcgtattga tggcgtgttt ttcctcgcga tgcacgcccg ccacggcgac aatcagggaa 1593720
gaccgcgcgc acagcgtatt cataatgtcg aacagcacat cgttgcccac caggcgcgcc 1593780
agttcgacgt ggaaggcatt ggacaggcgg ttccagccga cgcggtcgcc cctgccggag 1593840
gcctcttctt cgcgccgtat catcgcataa agcggcttga ggcgcgtttc caaatccggc 1593900
aaatctgcga ggatattcaa aatcatcgtc tccatttcga tgcgcgcatt gaacacatcc 1593960
tgcatttctt tcaaatcggg aacgtggacg aacgcgcccc tgttgggttg caaatcgaca 1594020
atcttgtcgt gcgccaaaag cgacagcgcg ccgcggacgg tgttgcgcga acacaccatc 1594080
tgacggcaaa gttcggattc ggtcagcttt ttgccgggca gcagcacctg atcggtaatg 1594140
ccgtccaaaa tcagggcgta aacacggaac agctccgaat cgtgccgctc ttcgagaatc 1594200
agggaagacg tggtcggcgc atggataatg tcgtcgtttt caaagttcat gatgttttcc 1594260
gtatttttac gctttcaaat tttttaagat gttttaaggc ggctgtgttt caaatcgtgt 1594320
cagaggaatt aaagcattgc acaaatttat tttatagtgg attaacaaaa atcaggacaa 1594380
ggcgacgaag ccgcagacag tacaaatagt acggaaccga ttcacttggt gcttcagcac 1594440
cttagagaat cgttctcttt gagccaaggc gaggcaacgc cgtactggtt tttgttaatc 1594500
cactataatt caataaatta atatatggct taaaataacg ggattctcgc ctcccgcccg 1594560
cccgcagaag caggcggata tcattttaaa acgcggcatt taaaatttga ccgaaaattg 1594620
ttgacaatcc ggaatcaagt ctgcacaata ccccgacaag tccaagtatt ataaaggctg 1594680
aataaagagg aaacagcagg cagatatatt cgggaggtgc agtccgaata tatctgcttt 1594740
tttatgcgcc tccggattgc ctgccgcacc tttcccttca gacggtatca gccgtttccc 1594800
cataatgccg cccgatgcct atttatctgc cccggcaatt tcaaaactgt gggtaatctt 1594860
tgccgctttg cccaacataa tcgaagccga acagtatttt tcggcagaca tctgaacggc 1594920
gcgctcaatg gccgattctt tcaaatcatg cccgaatact ttgaaatgga tgtggatttc 1594980
ggtaaacacg cgcggcgcat cgtccgcccg tttcgccgta accgtcgcac ggcagtcagt 1595040
cactttctga cgctgttttt cggcaatcat caccacatcg atgctcgaac agcccgccac 1595100
gcccaacagc agcatttcca aagggctggg cccgcgctta gccttacctt ctgccgccga 1595160
cccctccata acgacgctgt gcccgccttc cgtcgtgccg acaaaacaca tcccgtctat 1595220
ccattttgat gtaacctgca tggtgtcatt cctgaaaata gcgttaaaac cgctttgcat 1595280
atggcgttat tgtaaacaat ttcaagcggc ttatgcagaa atatggacaa aacggcaaaa 1595340
aaacacttga aaaccgattt acggtttggc tgcctggccg ttgatctgca ccgatttgag 1595400
tttcagcgta taggttttgc cgtcgtcggt atagccgatt tgtgccggaa tattgttcag 1595460
ggacggtgcg aagaaataca ttaccgcatc gtcgccgcgc cgcacccgat atttgacgac 1595520
ttcggtttcc acgccgccta tgctgtattt tcctgtaccc gccttattca aaccgccgac 1595580
ggaataaagt tttttgccgt tggtgatttt cagccccggg gggagtttcg cgtcatttgc 1595640
cgccaactgc caggcaagcg tgaacaaatc catagccttg gggctttgct cggttttgct 1595700
ctcgcccgct ttgccgtaag ttacgctgcc gtcggcgaat ttggcttccg catacagttt 1595760
gcccctgcgt atgtctctat agtaggtagg gtgcagggta ttgccgacaa ccgtaccgcc 1595820
ggactcgaaa cggatattgt atagcggcac tttaatcgtc gaaacgattt tgtaagcatt 1595880
gccgctgcgt tcaaatgtca tcgtggcggg aatgccgtag ctgccggaat agtgcagcac 1595940
ggcggattgg ggcagccctg ccgcatacgc gcacggcagg gcggcggaca aaatggcggc 1596000
ggaaaatata tttttaaaag tcttcatcat ttgctcccgc ccggtttacg ccgtcagaaa 1596060
acgggcggca tcggcgtttt ccgaatttct gacgcggttt ccctcaataa tcaggcggcc 1596120
ggcggcaaaa tcggcaacgg ctttcggata aagtttatgc tcgacagcca aaacccgtgc 1596180
ggcaatatcg tctgccgtat cgccgtcgag tatcggcaca acccccttgcg atacaatcgg 1596240

gccgcaatcc agttcggcag taacgaaatg gatggtgcag ccggcaacgc ggcagcccgc 1596300
ctccaaagcg cgttcgtgcg tatgaagtcc ggtaaacgag ggaaggatgg acgggtgaat 1596360
gttcatcagc ctgccttcgt aacgggcgca aaactcgggg gtcagaatcc gcataaaacc 1596420
tgccaaaacc accaagtcgg gttgatatgc gtcgattttc tccatcatgg cggtatcgaa 1596480
ggcaagccgg gatgtaaagt ttttatgatt caggctatcg gtcgggatgc cgcgttcggc 1596540
cgcccattgc aaaccggcag ccgtttcgct gttgctcaac acggcggcaa tgcggacgtt 1596600
gtgaatggcg gcattgacga ttgcctgcat attgctgccg cgtccagaaa tcaggatgac 1596660
gatgtttttc ataatggtgc gcttttgaaa gggatgccgt ctgaaccgct gtttggtggt 1596720
ttcagacggc atttgccgta aaaatgcccg aaaacctgtt tcgggcatgg attcggactt 1596780
aatttacttt tttgatgtcg acttgagccg gctgcttggc gggcgcgttt tcgggtgcgc 1596840
cgattttgac cagtttcaca tcaaatacca aagtggcgtt cggaccgatt ttgtcgcccg 1596900
caccctgttc gcggtaggca aggttggacg ggatgtagaa cgtggcttcg ccgccttctt 1596960
tcagaagctg tacgccttcg gtccaacccg gaatcacttg gctcaaaggg aaggtgaccg 1597020
ggccgccgtt ggctttgctg ctgtcgaata ccgtaccgtc aatcaggcgg ccttcgtatt 1597080
ccacggtaac gatgtcgtct ttggtcggct gtttgccttc gccctgtttg gtgattttgt 1597140
attgcaggcc ggaagcagtg gtcttcacgc cgtctttggc ggcattttct ttcagaaagg 1597200
cttcgccttt ttctttattg gccttcgcgt ccgccttgtg tttttctacg gctttagcct 1597260
gttgttcctg aaggaatttc atcatgactt cctgagcctg ctcttcggtc attttgattt 1597320
ctttgccgtc atacactgcc tgcatggctt cggtaaagac tttcaaatcg atttccgcgc 1597380
cctgttcctt catttgcttc agggagcgtc cgatgtccac gcccatcgca tagcttgcct 1597440
gctgcatcgt gctgccgatc gaagaggtgt cgccctgcgc ggaagaagcg gcggcaggtt 1597500
cggatgcaga tgcgggggcg gcttcttttt tgccgcaggc ggaaagtgcc aaagcggcgg 1597560
aaagggtcag tgcgctgatt ttgaaaatgg tgttcatgat ggatcttcgc tgtcgataag 1597620
gtcggaaaaa cgggattata gccgagtttg aatgtttcaa cacacaggat gacacataaa 1597680
gcgtcaatcg tgtgttgccc tgttttggaa gggattgaac cttccaaaat aagttttgat 1597740
tctaccgccc cgagggacag atgtccaagt ggcggggttc aaccgataag gaaattttaa 1597800
tcaaatagaa tcaagcctgt ttaaattttg taaatgcggc atttcagacg gcattttatg 1597860
ccttgccctc catgccgtga tgttcgatgg caaaaccgct tcggcggtag gcggtaaagc 1597920
gttcgcgcgc gtcggcgagc tcttccaagc tgttgccgac gatttccaaa acgcgcgcgg 1597980
gcaagaccgg agcggtgttc caaaaaccgt cggacaggtt caaaacggtc atgccttcgg 1598040
gaattcgggg cagattgccg ccgcaggcaa gcaggacgga tggttcagac ggcatggctt 1598100
cttccgtttc ccaaatttcg tgcggaatga aactctcggc ctcgtattgc caaagcattt 1598160
tgtccaattc ctgaagctgc ccgaacgaat cggaccacac cagtatcctg ccgccgtccc 1598220
gaatcgcacg ggcaatcagg cggcaggtga aaatcggaac ttgggcaacg tgcgtgtaaa 1598280
aggtggcttt cggcatattg tttgaacatt tggcaggata atgccgtctg aaaggcttca 1598340
gacggcattg tgggaaaatt aaagattccg cagatagttc agcagcaagg gaacgggacg 1598400
gccggtcgca cctttttccg caccggattt ccacgccgta cccgcgatgt caaggtgtgc 1598460
ccatggatag tcttcggtaa agtaggatag gaatgttgcg gcggtaatcg tgcccgcgcc 1598520
gggcgtgccg atgtttggaa tgtcggcaaa gttggatttg agttggtctt tgtaggtctc 1598580
aaagagcggc agttgccatg ctttgtcgtc cacgttgtag aagcggcaag caggctgtcg 1598640
atcaaatcct gattgttgcc catcacgccg ctgacatcgt gccccaaggc aacaatacac 1598700
gcgccggtca gggtggcgac gtcgatgacg gctttgggtt tgaactgctc ggcgtaagtg 1598760
agcgcgtcgc acaaaatcag acggccttcg gcatcggtgt tcaacacttc gatggtcagc 1598820
cctttcatac ttttcacgac atcgcccggt ttgtttgccg cgccggaagg catattttca 1598880
caagtggcga cgacggcaat caggttaatc ggcagttgca gtttgacggc ggcgcagaag 1598940
gtgctgatga cggttgccgc tccgcacata tcaaacttca tttcgtccat gttcaggccg 1599000
ggcttgaggg agatgccgcc ggtgtcgaag gtaatgcctt tgccgaccaa taccacaggc 1599060
gcggcttctt tgtcggctgc accgaaatag ctcagttcga ccaaataggg ggcttccgcg 1599120
ctgcctttgg cgaccgacca aaacgaaccc atgttttctt tgatgtagtc tttttcgatg 1599180
attttggcgt gcgcgcccag tttttcggct tcggctttgg cggtgcgcgc taaaaattcg 1599240
ggcgtgcatt cgttgggcgc ggcgttgccc aagtcgcggc agaggctttg tccgtaaact 1599300
tgcgcttcgg cgacgcgcaa ggcttctttg acggcggctt cgtgcgcggt atggaacacg 1599360
gcagtttcaa atttggcggg cttggcttct tttttgtagc ggtcgaaacg gtaggcggca 1599420
ttgccgaacg caatcgcaaa cgcttcggca acggctgcag cctgcgcttc ttcaaagacg 1599480
tgaacgtcca cattgaccgt ttcctgattt tgcgcccatt tggcggcttc ggcggcggcc 1599540
ttgttcaatg cggcgcggcc ggtgcttttc agacagcata cggcaacagc ctgcaaaccg 1599600
ttgcctgtcg ggatttttgt gtcggcaaaa ttttgacctt cttcaagcga agacaaaagg 1599660
gcaaggacgg tcgggttgct cagttgcgat gcttcggtgc agacaaataa ctgtgcgcct 1599720
gcctgctgtt cctgcaagat ttcggttttt gtgctaaatt ccacgtttat tctcctgatt 1599780
gagacggttg tcggtagttt tcggacggcc tttcgctcaa aagaccgtct gaagacggct 1599840
ggcacgattg taccccattt gaagcaccgt ctgaaacctt gcgcggacaa tccgcctgcg 1599900

ccgaaccgct taccgccccc ctgaccgcga ttctatgatt tatcaaagaa acctcatcaa 1599960
agaactctct tttaccgccg tcggcatttt cgtcgtcctc ttggcggtat tggtctccac 1600020
gcaggcaatc aacctgctcg gccgtgccgc cgacgggcgt gtcgccatcg atgccgtgtt 1600080
ggcattggtc ggcttctggg tcatcggtat gacgccgctt ttgctggtgt tgaccgcatt 1600140
tatcagtacg ttgaccgtgt tgacccgcta ctggcgcgac agcgaaatgt cggtctggct 1600200
atcctgcgga ttggcattga aacaatggat acgcccggtg atgcagtttg ccgtgccgtt 1600260
tgccgttttg gttgccgtca tgcagctttg ggtgataccg tgggcagagc tacgcagccg 1600320
cgaatacgct gaaatcctga agcagaagca ggaattgtct ttggtggagg caggcgagtt 1600380
caacagtttg ggcaagcgca acggcagggt ttattttgtc gaaaccttcg ataccgaatc 1600440
cggcatcatg aaaaacctgt tcctgcgcga acaggacaaa aacggcggcg acaacatcat 1600500
cttcgccaaa gaaggtaact tctcgctgaa cgacaacaaa cgcacgctcg aattgcgcca 1600560
cggctaccgt tacagcggca cgcccggacg cgccgactac aatcaggttt ccttccaaaa 1600620
actcaacctg attatcagca ccacgcccaa actcatcgac cccgtttccc accgccgtac 1600680
cattccgacc gcccaactga ttggcagcag caacccgcaa catcaggcgg aattgatgtg 1600740
gcgcatctcg ctgaccgtca gcgtcctcct actctgcctg cttgccgtgc cgctttccta 1600800
tttcaacccg cgcagcggac atacctacaa tatcttgatt gccatcggtt tgtttttaat 1600860
ttaccaaaac gggctgaccc tgctttttga agccgtggaa gacggcaaaa tccatttttg 1600920
gctcggactg ctgcctatgc acattatcat gtttgccgtt gcactcatcc tgttgcgcgt 1600980
ccgcagtatg cccagccagc ccttctggca ggcggttggc aaaagtctga cattgaaagg 1601040
cggaaaatga acctgatttc acgttacatc atccgtcaaa tggcggttat ggcggtttac 1601100
gcgctccttg ccttcctcgc tttgtacagc ttttttgaaa tcctgtacga aaccggcaac 1601160
ctcggcaaag gcagttacgg catatgggaa atgctgggct acaccgccct caaaatgccc 1601220
gcccgcgcct acgaactgat tcccctcgcc gtccttatcg gcggactggt ctccctcagc 1601280
cagcttgccg ccggcagcga actgaccgtc atcaaagcca gcggcatgag caccaaaaag 1601340
ctgctgttga ttctgtcgca gttcggtttt atttttgcta ttgccaccgt cgcgctcggc 1601400
gaatgggttg cgcccacact gagccaaaaa gccgaaaaca tcaaagccgc cgccatcaac 1601460
ggcaaaatca gcaccggcaa taccggcctt tggctgaaag aaaaaaacag cattatcaat 1601520
gtgcgcgaaa tgttgcccga ccatacgctt ttgggcatca aaatttgggc gcgcaacgat 1601580
aaaaacgaat tggcagaggc agtggaagcc gattccgccg ttttgaacag cgacggcagt 1601640
tggcagttga aaaacatccg ccgcagcacg cttggcgaag acaaagtcga ggtctctatt 1601700
gcggctgaag aaaactggcc gatttccgtc aaacgcaacc tgatggacgt attgctcgtc 1601760
aaacccgacc aaatgtccgt cggcgaactg accacctaca tccgccacct ccaaaacaac 1601820
agccaaaaca cccgaatcta cgccatcgca tggtggcgca aattggttta ccccgccgca 1601880
gcctgggtga tggcgctcgt cgcctttgcc tttacccccgc aaaccacccg ccacggcaat 1601940
atgggcttaa aactcttcgg cggcatctgt ctcggattgc tgttccacct tgccggacgg 1602000
ctcttcgggt ttaccagcca actctacggc atcccgccct tcctcgccgg cgcactacct 1602060
accatagcct tcgccttgct cgccgtttgg ctgatacgca aacaggaaaa acgttgaacc 1602120
aatgccgtct gaacctctct tcagacggca tttgttttca ttgacacatt cccacagaca 1602180
gatagccgtt ccctattaca ttacctgtca taacagttcc atttttgtta aaactagtct 1602240
atgatagcgg tacaaatatt gtttacaata tttaacgcaa atcatttgca acccgacaaa 1602300
agaaaaacag aaaaaggaac aaagagatgt tagaagccta tcgtaaagcc gccgccgagc 1602360
gcgccgccct cggcattccc gccctccctt tgaacgcgca gcaaaccgcc gatttggttg 1602420
agctgctgaa aagcccgccc gcaggcgaag gcgagttctt ggtcgaactg cttgcccacc 1602480
gtgttccgcc cggtgtggac gatgccgcca aagtcaaagc ctcattcctg gctgccgttg 1602540
ccgaaggcag cgcgtccagc ccgctgatct cccccgaata tgcgaccgaa ctcttaggta 1602600
caatgctcgg cggttacaat attcacgcct taatcgaact cttggacgac gacaaactcg 1602660
cgtccattgc tgccaaaggc ttgaaacata cgcttctgat gttcgattcc ttccacgacg 1602720
ttcaagaaaa agccgaaaaa ggcaacaaat acgcgcaaga agttttgcaa tcttggcgcag 1602780
atgccgaatg gttcgcctca cgcgccaaag ttcccgaaaa aatcaccgtt accgttttca 1602840
aagttgacgg cgaaaccaat acagacgacc tctcccccgc gcccgacgcg tggagtcgtc 1602900
ccgatattcc gctgcacgcg ctggccatgc tgaaaaaccc gcgcgacggc atcacgcccg 1602960
acaaaccggg cgaagtcggt ccgattaaat tgttggaaga actcaaagcc aaaggccatc 1603020
cggttgctta cgtcggcgac gtggtcggta ctggttcttc acgcaaatcc gcgaccaact 1603080
ccgtcatttg gcataccggc gaagacattc cgttcgtgcc gaacaaacgc ttcggcggcg 1603140
tatgtttggg cggcaaaatc gcgccgattt tcttcaatac ccaagaagat tccggcgcgc 1603200
tgccgattga agtcgatgta tctgctctaa aaatgggcga tgtcgtcgat atcctgcctt 1603260
atgaaggcaa aatcgtgaaa aacggcgaga ctgttgccga gtttgaattg aaatcacaag 1603320
tattgctgga cgaagtgcaa gccggcggcc gtatcaacct gattatcggc cgaggtctga 1603380
ccgccaaagc gcgcgaagcc ctgaaactgc ctgcctctac tgcattccgc ctgccgcaag 1603440
cgcctgccga aagcaaagcc ggtttcacct tggcgcaaaa aatggtcggc cgcgcctgcg 1603500
gtctgcccga aggacaaggc gtgcgcccgg gtacttactg cgaaccgcgt atgacgacgg 1603560

tcggctcgca agacacgacc ggcccgatga cccgcgacga gttgaaagac ttggcttgtt 1603620
tgggcttctc cgccgatatg gtgatgcagt ctttctgcca caccgccgcc tatccgaaac 1603680
ctgtcgatgt aaaaacccat aaagaactgc ccgcctttat ttccacccgt ggcggcgtgt 1603740
cactgcgtcc gggcgacggc gtcatccact cgtggctcaa ccgcctgctg ctgcccgata 1603800
ccgtcggcac cggcggcgac agccataccc gtttccccat cggtatttcc ttccccgccg 1603860
gctccggctt ggttgccttt gccgccgcaa cgggcgtaat gccgctcgat atgcccgagt 1603920
ctgtattggt acgcttcagc ggcaagctgc aaccgggcgt aaccctgcgc gatttggtga 1603980
acgccatccc gctgtacgca atcaaacaag gtttgctgac cgttgccaaa gccggtaaga 1604040
aaaacatctt ctccggccgc atcctcgaaa tcgaaggcct gcctgatttg aaagtggaac 1604100
aagcctttga attgaccgac gcatccgccg aacgctccgc cgccggctgt accgtgaagc 1604160
tcaacaaaga gccgattatc gagtacatga aatccaacgt cgtgttgatg aaaaacatga 1604220
ttgccaacgg ctatcaagac ccgcgcactt tggaacgccg catcaaagct atggaaaaat 1604280
ggctggcaaa tcccgagttg ctcgaagcgg ataaagatgc cgaatacgcc gccgtgattg 1604340
aaatcaacat ggacgacatc aaagagccga ttatcgcctg cccgaacgac ccggacgacg 1604400
tgtgcttcat gtccgaacgc tccggcacca aaatcgacga agtattcatc ggttcgtgta 1604460
tgaccaacat cggccacttc cgcgccgcct ccaaactttt ggaaggcaag gcagacaccc 1604520
ccgtccgcct gtggattgcg ccgccgacca aaatggacgc gaaacaattg tccgacgaag 1604580
gacactacgg cgtactcgga cgtgccggcg cgcgtatgga aatgccgggt tgctccttat 1604640
gtatgggtaa tcaggcgcaa gtacgcgaag gtgcgaccgt tatgtccacc tccacccgca 1604700
acttcccgaa ccgtttgggt aaaaacacct ttgtttacct cggttcggcg gaattggcag 1604760
cgatttgctc caaactgggt aaaatcccga ccgttgaaga atatcaagcc aatatcggca 1604820
tcatcaacga acagggcgat aaaatctacc gctatatgaa cttcaacgaa atcgacagct 1604880
acaacgaagt agccgagacc gtgaacgttt aatccccgtc atccgtatga agtaagggat 1604940
tgaccgcaat gccgtctgaa caaccttcag acggcattgc aacattccgc taacccttct 1605000
ttccgcaaac gctgcaaata cggcgttcac gcccccacat aaaggaaacg acagtgaacc 1605060
tgaaaaaccg ccatttctg aaactttag acttcacgcc ggaagaaatc accgcctacc 1605120
tcgaccttgc cgccgaattg aaagccgcca aaaaagcagg gcgcgagatt cagcggatga 1605180
aagggaaaaa catcgccctg atttttgaaa aaacctctac tcggacgcgc tgcgcgtttg 1605240
aagtcgccgc gcgcgatcaa ggcgcgggag tgacttattt agagccgtcc gccagccaaa 1605300
tcgggcataa ggaaagcatc aaagacaccg cccgcgtgtt gggcaggatg tacgatgcca 1605360
tcgaatatcg cggtttcggt caggaagttg ttgaagaatt ggcgaaatac gcgggcgtac 1605420
ccgtgttcaa cgggctgacc aacgagttcc atcccacaca aatgcttgcc gacgcactga 1605480
ctatgcgcga acacagcggc aaacctttga accaaaccgc gtttgcctac gtcggcgacg 1605540
cgcgttacaa catgggcaat tccctgctga ttttaggggc aaaattgggg atggacgtgc 1605600
gtatcggcgc accgcaaagc ctgtggccgt ctgaaggcat tattgccgcc gcacacgccg 1605660
ccgccaaaga aaccggcgca aaaattaccc tgaccgaaaa cgcgcatgaa gccgtgaaga 1605720
atgttgattt tattcatacc gatgtgtggg tcagcatggg cgagccgaaa gaagtctggc 1605780
aggaacgcat cgatttgctg aaagattacc gcgttacgcc cgaactgatg gcggcatcgg 1605840
gcaatccgca agtcaaattc atgcactgcc tgcccgcctt ccacaaccgc gaaaccaaag 1605900
tcggcgaatg gatttacgaa accttcgggc tgaacggtgt ggaagttaca gaagaaatat 1605960
tcgaaagccc cgccagcatc gtgttcgatc aggcggaaaa ccgtatgcac acgattaaag 1606020
cggtaatggt cgcggctctg ggcgactgac agaactgtgc ctgtttaaat tcatccgcaa 1606080
cacagatacc gtctgaacac gatgttcaga cggtatccat atatagtgga ttaaatttaa 1606140
accagtacgg cgttgcctcg ccttgccgta ctatttgtac tgtctgcggc ttcgtcgcct 1606200
tgtcctgatt tttgttaatc cactataaaa aaactgccta cacgatgtgt aggtagtccc 1606260
gtttgaaaac aatcagtttt tgtcttggtc aaccaatttg ttggcagtaa tccaaggcat 1606320
catggcacgc agttgtgcgc cgactttttc aacttggtgg tcggcattca gacggcggcg 1606380
ggcagtcata gacgcatagt tgacattacc ctcttggata aacatttttg cgtattcgcc 1606440
ggtttgaatg cgtttcaggg cattgcgcat ggcttctttg ctggaagcat tgaccacttc 1606500
agggccggta acgtattcgc cgtactccgc attgttggaa atggagtagt tcatattggc 1606560
aataccgcct tcgaaaatca ggtcaacgat cagtttcatt tcgtgcagac attcgaagta 1606620
agccatttca ggcgcgtaac cggcttcggt cagggtttca aaacccgcct tgatcaactc 1606680
gaccacgccg ccgcacaata cggcttgttc gccgaacaga tcggtttcgg tttcttcgcg 1606740
gaaagtggtt tcaatcacac cgcctttggt gccgccgttg gcagccgcat aagacagggc 1606800
gatgtctttg gctttgccgg aattgtcttg gtaaacggca atcagagaag gcacgccgcc 1606860
gccgcgtttg tattcactgc gtacggtatg gcccggacct ttgggggcaa ccataatcac 1606920
gtccaagtcg gcacgcggaa cgatttggtt gtagtgcacg ttgaagccgt gtgcaaatgc 1606980
cagcgttgcg ccttctttca aattggctgt aacttcggcg tgatagacgg caggcatggt 1607040
ttcgtcaggc agcagcagca taacgacatc ggcttctttg gtcgcttcag caacggtttt 1607100
gacgacatga ccggctgctt cggctttttt ccaagaagaa ccttggcgca gaccaatcac 1607160
cacgtttaca cccgaatctt tcaggttggc ggcatgggca tgaccttgcg aaccgtaacc 1607220

gatgatggca acggttttgc ctttgattag ggacagatcg gcatctttat cgtaatagac 1607280
ttgcatttga tttcctttaa ggtaaatggt tgtcgaagcc ttaaaatgtt gagcggcttc 1607340
ggacgggtta aacagagtgt gccgcttaat cggcaacttc attcatcaat acgatttcca 1607400
acgcttcggt tttgccgtcg acggactgga cgaaggcttg gaaatgcgcg ctggcgttat 1607460
gttcgtcaat agctgcttga gatttccaat tttccacgaa aacaaaacgg ttcggtttgc 1607520
cgatttcctg atggagatcg tagctgatgt tgccctcttc tgcacggctg gctttgacca 1607580
gttctttaaa ctgtgctgcc agtgtttctg tgtattccgg tttgacggta accagtgcga 1607640
caattttaat gttcgacata aatctctcct gccgttcgtt tttcagacga cattcaaata 1607700
ccgtgccgtc tgaaaggtta cggcgttaaa ttttcaaaat acgctcaccg cgaccgatgc 1607760
cggccgcgcc tgtgcgtacg gtttccaaaa tttgggcgcg tccgaccgtt tccaaaaagg 1607820
aatccagctt gtctgtcgag ccggtaattt caatcgtata gctgcggtcg gttacgtcga 1607880
tgatgctgcc ccggtagatt tcggtcaagc gtaaaaattc gtcgcggtct ttgccggcgg 1607940
cacggacttt taccaacatc agttcgcgtt cgacaaaacg gctttcattc aaatcgacca 1608000
ctttaatcac ttcaatcaat ttattgagtt gcttggtaat ttgttcgatg acctgctcgt 1608060
cgccgtgggt aacgatggtc atccgtgaca gggttttgtc ttcggtcggc gcaaccgcca 1608120
aagaatcgat attgtaatcg cgtgcagaga acaaaccgac cacgcggctc atcgcacctg 1608180
attcgttttc aatcagaaca gataagatat gtcgcatttg tctctcctta cgcctttccg 1608240
tccgcacgca tatgcggcgg aagtaccatt tcgtccaaac ctttgccgtt gccgaccatg 1608300
ggcatcacat tctgtttctg gtcggtcagg aagtcgataa acaccagcct gtctttttgg 1608360
ttcaatgctt ccaacaacgc accttccaca tcagacttct tgtccacgcg gataccgata 1608420
tggccgtatg cctcggcaag tttgacgaaa tcgggcaaag aatcgaaata ggtttccgac 1608480
tctcgtccgc cgtaatatat ttcctgccac tggcgtacca taccgagata accgttgttc 1608540
agcgtaatga cgttaaccgg aatccgatat tggaaacagg tggacagctc ttggatgttc 1608600
atctggatcg agccgtcgcc ggtgatacag aatacgtctt gatccggggc ggcaagtttt 1608660
gcaccaatcg cataaggcag acccacgccc atcgtaccca aaccgccgga attgagccat 1608720
tggcgcggac gttcgaaggg ataatattga gccgcaaaca tttgatgctg ccctacatcc 1608780
gatgtgatga ttgccgaatt gccggtaatc tcggcaagct tctgaatcac atattgtggc 1608840
ttgataattt cgctgccgtt gtcaaaccac aagcaatctc gggaacgcca ttcctctatg 1608900
gttttccacc atttgcccaa agcatcttca gacggcacgg actcttgttt ttgccacagc 1608960
gcaaccatct cggacaaaac gtttttcacg tcgccgacaa tcggaatgtc caccttcacg 1609020
cgtttggcga tgctggaagg atcgacatcg atatggataa ccttcttcgc cttctcgaaa 1609080
aatttggacg gtacggaaac cacacggtcg tcaaaacgcg cacctacggc aagaacgaca 1609140
tccgcattct gcatggcaag gtttgcctcg taagtaccgt gcataccgag cataccgagg 1609200
aattggcggt cgccggaagg ataagcgccc aagcccatca gcgtacccgt gcacggagca 1609260
cccgtcattc ggacaaatcg ggtcagctct tcagaagcat tacccaacac cacgccgccg 1609320
ccaaaataga cgaccggacg tttggcagat gccaacatct gcacggcctt tttaatctga 1609380
ccgatatgtc cttgaacaac cggttgatac gaacggataa aaatgtcttc ctgaggatag 1609440
ctgaatttcg ccatcgcctg cgtaacatct ttcgggacat caaccaccac gggccccggt 1609500
cggccgcttg cggcaatttg gaacgccttt ttaatggttt ccgccaactc attgatgtcc 1609560
gtaaccagga aattgtgttt gacgcacgga cgggtaatac ccaccgtatc aacttcttgg 1609620
aacgcatccg taccaatcag ggaattgcct acctgcccgc tgatgaccac catcggaatc 1609680
gaatccgtat aggcagtagc aataccggtc agtgcattgg taacgcccgg gccggatgta 1609740
accaatgcca cgcccacctt accgctgacg cgcgcatacg catctgccgc gtgtactgcc 1609800
gcctgctcat ggcgggtaag aatgtgtttg aatttattga gttggaaaag ggcatcgtag 1609860
atttcgataa ccgcaccgcc gggataaccg aaaacgtact cgacaccttc ggctttgaga 1609920
ctctgcacta tgatttgcgc gcctgataac tgcataacga cctcttttat acggtttcaa 1609980
accaataggg acaaaccgct ttgccacagc acctgtaatg caattccacc aagcagcgat 1610040
ttagggtacg cgcattgggg gaacacggca acagacggat tatccaatca attggaaagg 1610100
aacacagagt ttgtgaaaaa gagtagaaac gataacgcaa accgacagtt caatcaagaa 1610160
aaatctttca tcttttaata ttttttgaaa gcagagaaat tattgattga ttttaaaaga 1610220
ataaaatcag gagtaccttt tttgaaagat ggaaattgtt gacagtttgt gtaggagggg 1610280
cagatgtgaa aaacccttct tcgatatcaa gaattgtaaa atttacaggg tttcatccca 1610340
ataaagactc gggatattga ttgaacttga ttttattttt gatatatcaa aaatattccc 1610400
aaccatactt cctgaaaatg gctcattgca ccggactgta ttggacggca ttgacagaac 1610460
caagagggct aacaacgact taatatattg attgtatagt ggattaacaa aaatcaggac 1610520
aaggcgacga agctgcagac agtacaaata gtacggaacc gattcacttg gtgcttcagc 1610580
accttagaga atcgttctct ttgagctaag gcgaggcaac gccgtactgg tttaaattta 1610640
atccactata tttagtttta tctatttcat taaacagcaa tagacaaaaa aaataaccgc 1610700
tctaaaagcg gttgtggtgc ccagggtcgg actcgaaccg acacaccttg cggcggggga 1610760
ttttgagtcc cctgcgtcta ccaatttcgc cacctgggct ggtgaagaag tcgtcattat 1610820
aatggctttt gaaattctgt aaaccttttt tttgaaatta ttttatctgt ttttatttta 1610880

```
tttttgattt taaatagaat ttttattatt ttaatcttac tgttctttcc gctccaaaga 1610940
ttctgtatga ttcggcaatt cctgccgtgc agacaacgta aaaaaatact acattaaatc 1611000
tgccaaacgc gttaagatgg aaatattcaa attccgtacg aatcaggttt tgctatttat 1611060
tcttgggaga ttgtcatgtt ttccgtaccg cgttcctttt tgccgggcgt tttcgtactt 1611120
gccgcgcttg ccgcctgcaa acctcaagac aacagtgcgg cgcaagtcgc ttcttcaagt 1611180
gcatccgcgt cggctgcgga aaatgcggca aagccgcaaa cgcgcggtac ggatatgcgt 1611240
aaggaagaca tcggcggcga tttcacgctg accgacggcg aaggcaagcc tttcaacctg 1611300
agcgatttga aaggcaaggt cgtgattctg tctttcggct ttacgcactg tcccgatgtc 1611360
tgcccgacag agcttttgac gtacagcgac acgttgaagc agttgggcgg gcaggctaag 1611420
gacgtgaaag tggtgttcgt cagcatcgat ccggaacgcg acacgcctga aatcatcggc 1611480
aagtatgcca aacagttcaa tccggacttt atcggtctga cggcaacggg cggccaaaac 1611540
ctgccggtca tcaagcagca ataccgcgtg gtttctgcca aagtcaatca aaaagacgac 1611600
agcgaaaact atttggtcga ccactcttcc ggtgcgtatc tcatcgacaa aaacggtgag 1611660
gttgccattt tctcgcctta cggaagcgag ccggaaacga ttgctgccga tgtaaggacc 1611720
ctgctctgat aaaaccgtat gccgtctgca ccgtcggcgc ctattcagac ggcattattg 1611780
tttcaaccga caaaggacat ccacaccatg caggataatg ctttgaccat cgccttatcc 1611840
aaggggcgca tttttgagga gacgctgccg ctgcttgccg ctgccggcat tgttccgact 1611900
gaagagcctg aaaaatcgcg caagctgatt atcgggacga accatgaaaa catccgcctt 1611960
gtcattgtcc gcgcaaccga tgtgccgact tatgtccgct acggcgcggc ggacttcggc 1612020
attgcgggca aagacgtgct gatcgaacac ggcggcacgg ggctttaccg gcctttggat 1612080
ttggagattg ccaagtgccg catgatggtt gctgtgcgta aagggtttga ttacgaagca 1612140
gcttcgcaac ccggatgccg tctgaagatt gccacaaagt atcctgaaat cgcggcatct 1612200
cattttgccg gcaagggtgt ccatgtggac attatcaaac tgtacggctc gatggaactt 1612260
gcgccgctgg tcggcttgag cgatgcgatt gtggacttgg tttcgacggg caacaccttg 1612320
aaggcaaacg gcttggaagc agtcgaacac atcgtcgaca tttccagccg cctggtggtc 1612380
aacaaggctg ctttgaaaac gaaatacgcg ctgctggagc cgattattca ggcgttcggc 1612440
ggcgcagtga aggcgaagta agcatccatt tgaataaaga tgcgttttca gacgacccta 1612500
tccgttcccg ccgacaggtc gtctgaaaat atcaccggca gtaaactgta taggagaagt 1612560
taaaatggtt gcaaaaataa aaaaattctc agattcaacc ctttccgttt tgaataacgg 1612620
cgagcgtcgg ttttatgtct attgtctgac cgacctgaaa aaagacaaaa tcctctacat 1612680
cggcaaaggc tgcggtaatc gtatcttcga gcatgaatgg gttgctagtc gttcacaaga 1612740
tccagtctcc ggcgagatta tcgatcggaa actcaaagcc atctccaaat gcaagaaact 1612800
cggtcgctat atcatcagct atcatctgac tgaagtcgaa gcactcgccg ccgaatctgc 1612860
cttaattcat tttgttaaat ctgtcttggg taaaaaactc aaaaataaaa ttgccgggca 1612920
tggtccgggt ggtattagcg tagaagaact agatcgccgc tttggattct cttctctccc 1612980
acttaacgag attaaccccg acgggctgat tctcgccatc aaaatccaca atgctttcga 1613040
tttagatact gacgaagaat tagactacct tttcgacaac caagacgatg ccaacctcaa 1613100
atcgcgtacg ttgggcaact gggttatcgg taaagatgtt gcttcaaaag tgaaatacgt 1613160
tatcggcgtt cacaccggtc tgcaaaacgc tgttgtcagt gcatacgaag tggacggttt 1613220
tgaaacaatg gttgaggaaa ccaaaaacgg tagaaaacaa tcccgttacc gtttccgcac 1613280
tacctctcgt agcgaagagg tattagccaa actcggtctg caacaaaaat gcctgcccga 1613340
attgaagttt ggtagcgggg gagaaaaagc gtatatcaga cccaaaacag agacagaaac 1613400
tgaacaagag aatattcaga cgacccccaa tccaaaaata aaaaaggaaa aaaccaaatc 1613460
atgaaaaaac tcaacaccca atcgcccgat ttccaagccg gactcaaagc cctgctggct 1613520
tttgaaaccg cgcaaaaccc cgaaaccgaa cgcatcgtcg ccgacatttg cgccgacgtg 1613580
caaaagcgcg gcgatgcggc tttgattgaa tacaccaaca aattcgatca gacaaacgct 1613640
aaaagcatcg atgatttaat actcacgcaa gccgatttga acgcggcgtt cgagcgcatt 1613700
ccgaacgacg ttcagacggc attgcagacc gccgcccgcc gtgtcgaaag ctaccaccaa 1613760
cgccaaaaaa tggaatcgtg gagctacacc gatgaagacg gcacgctgtt gggacaacaa 1613820
atcacaccgc ttgaccgcgt cggcatttac gtccccggcg gcaaggcggc gtatccgagt 1613880
tccgtcatca tgaacgccat gcccgcccac gtcgcaggtg tgaaagaaat catcatggtc 1613940
gtgccgacac caaaaggcga acgcaacgac atcgtacttg ccgccgcata cgtcgccggc 1614000
gtaaccaaag tcttcaccgt cggcggcgcg caggcggttg ccgccctcgc ctacggcacg 1614060
gaaaccatcc cccaagtcga taaaatcacc ggtccgggca acgccttcgt cgccgccgcc 1614120
aaacgccgcg tgttcggcgt ggtcggcatc gacatggtgg cggggccgtc tgaaatcctg 1614180
gtcatcgccg acggcacgac acctgccgat tgggtggcga tggatttgtt cagccaggcc 1614240
gaacacgacg aaattgccca agccatcctc atcggcacgt cgcaagcgta tctcgacgaa 1614300
gtagaagccg ctatggaccg cctgatcgaa actatgccgc gccgcgacat catcgaagcc 1614360
tcgctcggca acaggggcgc gatgatactc gccaaagact tggacgaagc ctgcgaaatc 1614420
gccaactaca tttcccccga acacttggaa ctgtcagtcg aaaacccgca ggaatgggcg 1614480
aaaaaaatcc gccacgccgg tgcgattttc atgggacgct acaccggcga aagcctcggc 1614540
```

gactactgcg ccggtccaaa ccatgtgttg cccaccagcc gaaccgcccg cttttcctcg 1614600
cctttgggga catatgattt ccaaaaacgc tccagcctga ttcaggtttc ggaacagggc 1614660
gcgcaaaaat taggcgaaac cgccagcgtg ctggcacacg gcgaaagcct gaccgcccac 1614720
gcccgcgcgg cagagttccg tatgaaataa tgccgaaacg gcgtacaggc atattccaac 1614780
cattaaggaa acacgatgaa atccgtccgc tccttcatcc gcgacgacat acaagctatg 1614840
tcggcatatc agattgccga cgttccgccc ggctttgcca aactcgattc gatggaaagt 1614900
cccgtccacc cttttgccgg acatgaaacg ctgttgcagg aatggcaggc acggcttgcc 1614960
gccgcgccca tccatcttta ccccaatccc tccggcagcg gtttacagga agcattacgt 1615020
tcggcgttcg acattcccga ctgcgccgac atcgcgctgg gcaacggttc ggacgaactg 1615080
atacagttca tcacgatgct gaccgccaaa ccgggcgcgg caatgttggc agccgaaccc 1615140
agtttcgtca tgtaccgcca caacgccgcg ctgtacggca tggattatgt cggcgttcca 1615200
ctgaacggag atttcaccct caacctgccc gccgtcctcg aagccgtcag gaaacaccgc 1615260
cctgccctga cctttatcgc ctaccccaac aaccccaccg gcgtatgctt cacgcgtgcc 1615320
gaaatcgaag ccgtcatcga agcttcagac ggcatcgtcg tcgtcgatga agcctacggc 1615380
gcattcaacg gcgacagctt cctgccgcag gcaggcagga ttcccaacct gatagtctta 1615440
cgcaccctca gcaaaatcgg ttttgccgga ctgcgtatcg gttatgcggc aggctgcccc 1615500
gaagtcatcg gcgaactgca aaaaatcctg ccgccctaca atatgaacca attgagcctg 1615560
accactgcca aactcgccct gcggcactac ggcattatct ctgccaacat cgacagcctg 1615620
aaaaacgaac gcgaacggat gttcgccgaa ttgggcaaaa tatgccgtct gaacaccttt 1615680
tcaagtcagg caaacttcat taccatacgc gtacccgatg ccgatttgtt gtttgacacg 1615740
ctcaaacaaa accgcatctt ggttaaaaaa ctgcatggcg cgcacccgct tttggaacac 1615800
tgcctgcgca ttaccgtagg cagccccgca caaaacgatg ccgttctcaa catcattcgc 1615860
caactttact gccaaccaac ggatttccta tgaatttgac taaaacacaa cgccaactgc 1615920
acaactttct gaccctcgcc caagaagcag gttcgctgtc caagctcgcc aaactctgcg 1615980
gctaccgtac ccccgtcgca ctctacaaac tcaaacaacg ccttgaaaag caggcagaag 1616040
acccagatgc acgcggcatc cgtcccagcc tgatggcaaa actcgaaaaa cacaccggca 1616100
aacccaaagg ctggctcgac agaaaacacc gcgaacgcac tgtccccgaa accgccgcag 1616160
aaagcaccgg aactgccgaa acccaaattg ccgaaaccgc atctgctgcc ggctgccgca 1616220
gcgttaccgt caaccgcaat acctgcgaaa cccaaatcac cgtctccatc aacctcgacg 1616280
gcagcggcaa aagcaggctg gataccggcg tacccttcct cgaacacatg atcgatcaaa 1616340
tcgcccgcca cggcatgatt gacatcgaca tcagctgcaa aggcgacctg cacatcgacg 1616400
accaccacac cgccgaagac atcggcatca cactcggaca agcaatccgg caggcactcg 1616460
gcgacaaaaa aggcatccgc cgttacggac attcctacgt cccgctcgac gaagccctca 1616520
gccgcgtcgt catcgacctt tccggccgcc ccggactcgt gtacaacatc gaatttaccc 1616580
gcgcactaat cggacgtttc gatgtcgatt tgtttgaaga atttttccac ggcatcgtca 1616640
accacagtat gatgaccctg cacatcgaca acctcagcgg caaaaacgcc caccatcagg 1616700
cggaaaccgt attcaaagcc ttcgggcgcg ccctgcgtat ggcagtcgaa cacgacccgc 1616760
gcatggcagg acagaccccc tcgaccaaag gcacgctgac cgcataaaaa accataccgt 1616820
ctgaaacacc cgcaggcttt tcagacggta tcggaacaga taagattaca ctacactaca 1616880
aacagaaaag gagtaaacat catgtccgca aacgaatacg cacaaatcgg ctggataggc 1616940
ttagggcaaa tgggtctgcc tatggtaacg cggctcttgg acggcggcat cgaagtcggc 1617000
gtatacaacc gctcgcccga caaaactgcc cccatctccg ccaaaggcgc aaaagtttac 1617060
ggcaacaccg ccgaactcgt ccgcgactat cccgtcattt tcctgatggt ttccgactat 1617120
gccgccgtgt gcgacatcct gaacggagtc cgcgacggat tggccggcaa aatcatcgtc 1617180
aacatgagca ccatctcccc gaccgaaaac ctcgccgtca aagcacttgt cgaagccgca 1617240
ggcggacagt ttgccgaagc acccgtttcc ggatcggtcg ggcccgccac caacggcacg 1617300
ctgctgattc tgttcggcgg cagcgaagcc gttttaaacc cgctgcaaaa aatattttcc 1617360
ctcgtcggca aaaaaacctt ccatttcggc gatgtcggca aaggttcggg cgcgaaactc 1617420
gtcttgaact cgctcttggg cattttcggc gaagcgtaca gcgaagcgat gctgatggcg 1617480
cggcagttcg gcatcgatac cgacaccatc gtcgaagcca tcggcgrctc ggcaatggac 1617540
tcgcccatgt tccaaaccaa aaaatccctg tgggcaaacc gcgaattccc gcccgccttc 1617600
gccctcaaac acgcctccaa agacctcaac ctcgccgtca aagagcttga acaggcaggc 1617660
aacaccctgc ccgccgtcga aaccgttgct gccagctacc gcaaagcagt cgaagccggc 1617720
tacggcgaac aggacgtttc cggcgtttac ctgaaactgg cagaacactg attgcctttt 1617780
ccaaacacaa tgccgtctga acatatttca gacggcattt ttatcacccc acgcttaaaa 1617840
tcagtcccga ttatgactat atagtggatt aacaaaaatc aggacaaggc gacgaagccg 1617900
cagacagtac aaatagtacg gaaccgattc acttggtgct tcagcacctt agagaatcgt 1617960
tctctttgag ctaaggcgag gcaacgccgt actggttttt gttaatccac tataatccgc 1618020
acaaatttag tcaatatcaa gaccaattat gaaccaactc gaccaacttg gcacccgtat 1618080
caacctgatt tgcaatgtct tcgacaaatg gatcgggcag caggatctga attacaacct 1618140
ctttgccgta ctttataccc tggcaaccga aggcagccgc acgcaaaagc atatcggcga 1618200

aaagtggagc ctgcccaaac agaccgtttc aggcgtatgc aaaacccttg ccggacaagg 1618260
gttgattgaa tggcaggaag gcgaacagga ccggcgcaaa cggttgctgt cgttgaccga 1618320
aacaggcaaa gcctatgccg cacctttaac agaaagcgcg caggaattca gcgacaaagt 1618380
atttgccaca ttcggcgaca agcgcacaac tcggctgttt gccgatttgg atgcactggc 1618440
tgaagtgatg gaaaaaacaa tctcggaaaa taaaaaatag gggggcaaat atgtggaaaa 1618500
tgttgaaaca catagcccaa acccaccgca agcgattgat tggcacattt tccctggtcg 1618560
gactggaaaa ccttttgatg ctggtgtatc cggtgtttgg cggccgggcg atcaatgccg 1618620
tgattgcggg ggaggtgtgg caggcgttgc tgtacgcttt ggttgtgctt ttgatgtggc 1618680
tggtcggtgc ggtgcggcgg attgccgata cgcgcacgtt tacgcggatt tataccgaaa 1618740
tcgccgtgcc ggtcgtgttg gaacagcggc agcgacaagt cccgcattcg gcggtaactg 1618800
cgcgggttgc cctgtcgcgt gagtttgtca gctttttga agaacacctg ccgattgccg 1618860
cgacatccgt cgtatccata ttcggcgcgt gcatcatgct gctggtgctg gaattttggg 1618920
tcggcgtgtc ggcggtgggc atacttgcgt tgtttttatg gcttttgcca cgttttgccg 1618980
ccatcagcga aaacctgtat ttccgcctga acaacagctt ggaacgcgac aaccacttta 1619040
tccgaaaagg cgaccggcgg cagctgtacc gccattacgg actgcttgcg cgcctgcgtg 1619100
tgctgatttc caaccgcgaa gccttcggct atctctgcgt cggcacggcg atgggtattt 1619160
tgttcggctt tgcttttgtg atgatgacgc tcaaaggcta cagcagcgcg gggcatgtct 1619220
attcggtcgg cacttatctg tggatgtttg ccatgagttt ggacgacgtg ccgcgattgg 1619280
tcgaacaata ttccaatttg aaagacatcg gacaacggat agagtggtcg gaacggaaca 1619340
tcaaagccgg aacttgaaaa atgccgtctg aacacgcttc agacggcatt tccatccgtt 1619400
cggcaaacta catcacatcc gcccgccggt tgacaagttt ggcaaacaac ttttcaacag 1619460
aagcttccgc ctgcaaacca atgcgctgga tcaggctttg cttctcctga tatttcactt 1619520
cgataacctg tttgttttca aacgctttca acaacaaatc atcactggtc gaaatctcgt 1619580
caatcaagtt caacgccaac gcctgccgac cgaaccaatg ctcgcccgtt gccacttcct 1619640
caatatccaa ttgagggcgg ttctcgctga caaactgctt gaacaactga tgcgtttcct 1619700
ccagttcctg tcggaatttc tgtttgccct tttccgtatt ttcacccata aaagtaaccg 1619760
tgcgcttaaa ttcgcccgcc gtcatcacat ccacatcaat atcatgtttt ttcaacaggc 1619820
ggtggatatt cggtacttcc gccaccacac ccaccgaacc gacaatcgca aacggagcgg 1619880
aagcaatttt atccgccaca cacgccatca tataaccgcc gctcgccgcc accttatcga 1619940
cggcgacggt cagcggaata ttgcgttcgc gcaaacgcyt aagctgcgaa gccgccaaac 1620000
cgtaaccgtg aaccacgccg cccggacttt ccaatctgag cagaacctca tcttcaggct 1620060
tggcaatcaa aagcaccgcc gtaatctcat gacgcaagga ttctacggcg tgtgcataca 1620120
aatcgccgtc aaaatccaac acaaaaaggc gggatttttg cgtttcggca gatttctccc 1620180
caccctcctt caaacgcttt ttctctgctt tggcttccgc cttttccttt ttcttttcct 1620240
ctttttcctg atgttttgcc tcttccccgc ttaaaaagaa tgcttcaaac gattgccgct 1620300
gtttttata attttccgaa aaatccgtca gtacgacact gccgctttcc gactgtttct 1620360
tactctgtac gatagccaac acaatcagcg caattgcgcc gaacacggta agcagttcga 1620420
gcaggaaaat accgtaattc agtaaaattt ctttccacat tgattggatt tcctcttgtt 1620480
caggcatgaa catgtcaata ttgtccatca ccgtccgaca gataaaaaaa taaccgcttg 1620540
gagcggcatt gtcattttca gcttggtgcc cggagccgga atcgaaccgg cacgggatgt 1620600
ttagtcccga cggattttaa gtccgttgtg tctacctatt tcaccacccg ggcatttgtg 1620660
aaaggtggag gcgggggcgc ggattttaac cggcctgtat gaagattgca ctcctcatag 1620720
cataaacact ctgccacccc gccatagtac gataatggag gcgagagtcg gaatcgaacc 1620780
ggcgtagacg gatttgcaat ccgctgcata accactttgc tatctcgccc taaaactggc 1620840
ttatctaaaa aacttggagc gggaaacgag tctcgaactc gcgacctcaa ccttggcaag 1620900
gttgcgctct accaactgag ctattcccgc gcgttcaaac atatcggttt ttggagcggg 1620960
aaacgagtct cgaactcgcg acctcaacct tggcaaggtt gcgctctacc aactgagcta 1621020
ttcccgcgtt gatatgtttg aaataaaact tggagcggga aacgagtctc gaactcgcga 1621080
cctcaacctt ggcaaggttg cgctctacca actgagctat tcccgcaatg attgcggaag 1621140
aatgaaattt ttggagcggg aaacgagtct cgaactcgcg acctcaacct tggcaaggtt 1621200
gcgctctacc aactgagcta ttcccgcccg atttcattct ccgatatcga agagacacaa 1621260
ttattatgga ttctgttttt gccgtcaagc tatttttatg tttttttcag gcgatttctt 1621320
tccacgccat tttcagataa tacagcatcg accagactgt cagcaaagat gcgataaaca 1621380
tcaatacatt gccgatgaat gcgaggttaa atccataaaa atcgggaaaa ttcagcagca 1621440
gcaggaagat tgccagcatt tgcgcggcgg ttttaaactt accgacggtg gcgacggcaa 1621500
cgctgttcct tttgcccatt tgcgccatcc attcgcgcaa tgcggaaatg gtaatttccc 1621560
tgccgatgat gatcatggca aacaaaacat aggtccggtc gagtttgacc agtaaaagca 1621620
aagagacggc gaccatcagc ttgtcggcaa cgggatcgag gaaggcgccg aaatccgagg 1621680
tctgtttcca caaccttgcc aaaaatccgt caaaccagtc ggtcaaggcg gcaacggcaa 1621740
aaatgacggc ggcggtgaga ttaatcgttt cctccgcgaa ccacggaaaa ggcaggtaaa 1621800
aaagggctgt caggacagga atgagcaaga ccctcaacca tgtgaggaag atggggagat 1621860

tccaaggcat cggttttctc tgtgcagact gtaaagttgt gattataacg gttatcctca 1621920
taacccaaaa cgtaaaattg ctgcatgggc attcccccgc cccgccaatc tgttttcaca 1621980
ttcttttcaa acgcaggaaa atggcgggca ataaaagcaa aatacccagt ttcaggctga 1622040
aaacggcagg ttgtgccaac acttcgacaa ggcggtcttc cgtgcgggca aaatctttat 1622100
tgcttataga cactgccact gttgcggtat tccaacagaa cgccgtttaa aaaacctttg 1622160
ccgacggttt cgcttaaaac ggctctaacc tgctccgccc tgatggttct gccgatattg 1622220
ccgcctgtgc acaaactgtc gaacccatag caggaaagcc ggtaatgctg cccgtctgca 1622280
tccagtttga ttgcccgtcc gctgcggttg agggcggtaa cggtcaattc cgcatattcg 1622340
aatgtttttt cttgttcgtg aaatgccgtc aggtaaggtg caataaaaac ggcggacaac 1622400
agcagacagc ttatggcggc aaaccatacc cagcgataat atagtggatt aaatttaaac 1622460
cagtacagcg ttgcctcgcc ttagctcaaa gagaacgatt ctctaaggtg ctgaagcacc 1622520
aagtgaatcg gttccgtact atttgtactg tctgcggctt cgtcgccttg tcctgattta 1622580
aatttaatcc actatatttc acgcttaccc cttgtttctc aaatgccgtc tgaaataagc 1622640
ggcttaatat attgtttaca gtattgggaa gcataacaga caaaatgccg tctgaaatat 1622700
tttcagacgg catttcttat ccgaaacgga ttatttttgc gtttcaaccg cttccaatgc 1622760
acgcagggca taagtgtaag cggcacccgc attcagggca atggcggttg ccaatgcacc 1622820
tgcgatttcg ctgtcggtcg caccggcttt ggtggcggcg gcggcgtgaa cactgatgca 1622880
gctctcacaa cgtgtagtaa tggcaacggc gatggcaatc agttcgcgtg ttttagcatc 1622940
aagtgcctct gcagctgccg cttgttccaa tgcgccgtag gcctgcagca ttttaggatg 1623000
cgccttaccc agctcgccga acgatttttt aaccaatgcg gtatgttctt tccaatcttt 1623060
aaacattttc ttttcctttc tcttgcgttt aaccctgata cgcgcttgcg tatctgtttt 1623120
cgatgtgcgt attattgcaa ttattcagtt gtgtttctcg tttaatcatc tcattttatg 1623180
gttcaaaaag atttatggac attctggaca aactggtcga tttcgcccaa ttgacgggca 1623240
gtgtggatgt gcagtgcctt ttgggcggac aatggtcggt acggcatgaa accttgcaac 1623300
gcgaaggatt ggtacacatt gttacatcgg gcagcggcta tctctgcatc gacggcgaaa 1623360
cttccccgcg tccggtcagt acaggggata ttgtattttt cccgcgcggc ttgggtcatg 1623420
tgttgagcca cgacggaaaa tgcggagaaa gtttacaacc ggatatgcgg cagcacggtg 1623480
cgtttacggt caagcagtgc ggcaacggac aggatatgag cctgttttgc gcccgtttcc 1623540
gctacgacac ccacgccgat ttgatgaacg ggctgcctga aaccgttttt ctgaacattg 1623600
cccatccgag tttacagtat gtggtttcaa tgctgcaact ggaaagcaaa aaacctttga 1623660
cggggacggt ttccatggtc aacgcattgt cgtccgtcct gctggtgctt atcctgcgcg 1623720
cctatctcga acaggataag gatgtcgaac tctcgggcgt attgaaaggt tggcaggaca 1623780
aacgtttggg acatttaatc caaaaggtga tagacaaacc ggaagacgaa tggaatgtcg 1623840
acaaaatggt ggcggctgcc aatatgtcgc gcgcgcaact gatgcgccgt ttcaaaagcc 1623900
gggtcggact cagcccgcac gcctttgtga accatatccg cctgcaaaaa ggcgcgttgc 1623960
tgctgaaaaa aaacccggat tcggttttgt cggtcgcact gtcggtaggc tttcagtcgg 1624020
aaacgcactt cggcaaggcg ttcaaacggc aatatcacgt ttcgccgggt caataccgga 1624080
aagaaggcgg gcaaaaataa atcggggctt caaacgcaaa tgccgtctga aaaggctttc 1624140
atacagcatt tgcgtaccgc gtcatttcaa gggctgcatc ttcatcactt ccatcaaaaa 1624200
gttggtaaat gcggggttgt tgggtttgac atccatattt ttccaacgct gctgccagcc 1624260
gcgcaaggca ttctggatat acagcttgga ctgttccgta ttgattgcgc cccgctggct 1624320
gtctatcgcc gaacgcaggt agatttcata catactgtca tcgacggcat tgcgtccgac 1624380
caggcgtttt ctgaagttgt tcagatattg cgccgcctga accttggtca ttttaccgat 1624440
acccacctga tagcccaagc gcgtcgcttc atcgctgatt ttggcaacat ccgtccaatg 1624500
cgaagaggca aggcggaaac cttttgcagg tgcttccgtt ttgacggtat tgataggatt 1624560
cacggggatt tccgtcaatg tgggcacata aatagactgg cagccggaaa gaactgccgc 1624620
aatggaaaga gggataaggt attttttcat gcccccatta taatcaagtt tgccttgaga 1624680
aaacaaattg ttcggcaaga aaaataaaat ttcggcatca gaagcaggca aaaacacatt 1624740
ccacaagcct tgccgcaagg tttacaatcc gaccgtcctt atcgcaacga ccgtttatgg 1624800
ataccgcaaa aaaagacatt ttaggatcgg gctggatgct ggtggcggcg gcctgcttta 1624860
ccattatgaa cgtattgatt aaagaggcat cggcaaaatt tgccctcggc agcggcgaat 1624920
tggtcttttg gcgcatgctg ttttcaaccg ttgcgctcgg ggctgccgcc gtattgcgtc 1624980
gggacacctt ccgcacgccc cattggaaaa accacttaaa ccgcagtatg gtcgggacgg 1625040
gggcgatgct gctgctgttt tacgcggtaa cgcatctgcc tttggccact ggcgttaccc 1625100
tgagttacac ctcgtcgatt tttttggcgg tattttcctt cctgattttg aaagaacgga 1625160
tttccgttta cacgcaggcg gtgctgctcc ttggttttgc cggcgtggta ttgctgctta 1625220
atccctcgtt ccgcagcggt caggaaacgg cggcactcgc cgggctggcg ggcggcgcga 1625280
tgtccggctg ggcgtatttg aaagtgcgcg aactgtcttt ggcgggcgaa cccggctggc 1625340
gcgtcgtgtt ttacctttcc gtgacaggtg tggcgatgtc gtcggtttgg gcgacgctga 1625400
ccggctggca caccctgtcc tttccatcgg cagtttatct gtcgtgcatc ggcgtgtccg 1625460
cgctgattgc ccaactgtcg atgacgcgcg cctacaaagt cggcgacaaa ttcacggttg 1625520

cctcgctttc ctatatgacc gtcgtttttt ccgctctgtc tgccgcattt tttctgggcg 1625580
aagagctttt ctggcaggaa atactcggta tgtgcatcat catcctcagc ggtattttga 1625640
gcagcatccg ccccactgcc ttcaaacagc ggctgcaatc cctgttccgc caaagataaa 1625700
aaatgccgtc cgaacatcct tcagacggca tatcgggctt tatttccccg ccttcacatc 1625760
ctgccactgg cgcaccataa acttcaatgc cgccggctgg ataggcacca tgataaagct 1625820
gtttttcaaa tcctcctcgg ttgggaaaat cgtattgtcg tttttaaatt cgtcttccat 1625880
cagctcacgc gcaggcttgc tcgaaggcgc gtaagtaacg aaattgccgt ttttcgccga 1625940
cacttccggg tcgaggaagt cgttgatgta tttgtgcgcg ttggcgacgt ttttcgcatc 1626000
tttcggaatc acgaaagaat ccacccaaat ccccacgccc tctttgggca tcatcacgcg 1626060
gatttttcc ttgccgcccg cttcttcggc acggcgtttg gcgatgttca aatcgccgcc 1626120
gaaaccgatt gttacgcagg tatcgccgcg cgccaaatca tcgataaagc cggacgaagt 1626180
aaagcgtttg atattggggc ggtttttctt gagtagggcg gttgcctccc tgatgtcttc 1626240
cgtattgctg ctgttcgggt ttttacccaa atagttcaac accataggat agatttccgc 1626300
cgcgctgtcc aaatagctga tgccgcattg cttgagtttg gacgtgtatt cggggtcgaa 1626360
caccaaatcc cactggttgt ccggcagctt gtccgtaccc aaagcctttt tcacgcgttc 1626420
ggtattgatg gcgaaggtat ttgtccccca ataaaacggc acggcgtatt cgtggccggg 1626480
atcgaccccg tccatcagcc tcatcatttc ggggttgagg tgtttataat tgggaatcag 1626540
cgacttatcg attttctgat acgcacctgc cttaatctgc ctgcccacaa acgcattgga 1626600
cggcgcgaca atgtcgtaac cggacttgcc tgtcagcacc ttgctttcca gcgtttcatc 1626660
gctgtcgtac acatcataag taaccttgat gccgtttttc ttttcaaaat cggcaacggt 1626720
ttccggatcg acatattccg accagttgta aattttcaat acgttttggt tttccgccgg 1626780
tgccggtttt tcggcaggcg gtttgtccga accgccgcac gctgcaagca gcaaagcagt 1626840
caggacggcc aggggcagat gtttggtcat tatcattcct tgcatatcgg gttggagaaa 1626900
gcggccatta tagccgatat tggcaacagg gcttcagacg gcattcaaaa tcccgccaca 1626960
ctcttccgaa aaccgccgct tccatagcta gaaacaggga tttgcggtaa gataccgccg 1627020
ttcgttttcc ctgcttttac catgacaaga catttgagag acattgaaaa aattatgaaa 1627080
acctccgaac tgcgccaaaa attcctaaaa ttttttgaaa ccaaaggcca caccgtcgtc 1627140
cgctcttcca gcctcgtgcc gcacgacgac ccgaccctgc tgtttaccaa cgcgggcatg 1627200
aaccagttta aagacgtatt cttaggtttc gacaaacgcc cgtacagccg cgccaccacc 1627260
gcgcaaaaat gcgtacgcgc aggcggcaaa cacaacgact tggaaaacgt cggctacacc 1627320
gcccgccacc acaccttctt tgaaatgatg ggcaacttct ccttcggcga ctacttcaaa 1627380
cgcgacgcca tccacttcgc ttgggaattt ctgacttccc ccgaatggct caacatccct 1627440
aaagacaaac tgttggcgac cgtttacgcg gaagacgacg aagcctacaa catctggttg 1627500
aacgaaatcg gtatgccgtc cgagcgcatc gtccgcatcg gcgacaacaa aggcgcgaaa 1627560
tacgcatccg acaacttctg gcaaatgggc gacaccggcc cttgcggccc ctgctccgaa 1627620
attttctacg accacggcga agaaatctgg ggcggcattc ccggcagtcc cgaagaagac 1627680
ggcgaccgct ggatcgaaat ttggaactgc gtatttatgc agttcaaccg cgacgaacaa 1627740
ggcaatatga acccgcttcc caaaccttcc gtcgataccg gtatgggctt ggaacgcata 1627800
gccgccgtca tgcagcatgt tcacagcaac tacgaaatcg acttgttcca agacctgctc 1627860
aaagccgttg cccgcgaaac cggcgcgccg ttcagaatgg aagaacccag cctgaaagtc 1627920
atcgccgacc acatccgctc ctgctcgttc ctgattgcag acggcgtctt gccttccaac 1627980
gaaggccgcg gctacgtatt gcgccgcatt atccgccgcg ccgtgcgcca cggttacaaa 1628040
ctgggtcaaa gcaaaccgtt cttccacaaa ctcgttgccg atttggtcaa agagatgggc 1628100
ggtgcctacc ctgaattgaa agaaaaacaa gcccaaatcg aagaagcatt gaaaaacgaa 1628160
gaaagccgtt ttgcccaaac gctggaaacc ggtatggctt tgttggaaaa cgcgctggtc 1628220
aaaggcggca aaacactcgg cggcgaaatc atcttcaaac tctacgatac ctacggtttc 1628280
ccatacgact tgactgccga catctgccgc gaacgcaata tcgaaccgga cgaagcaggc 1628340
ttcgagcgcg aaatggaagc ccaacgcgca cgcgcacgcg ccgcccaaag cttcaaagcc 1628400
aacgcccaac tgccttatga cggtcaagac accgagttta aaggttatag cgaacgccaa 1628460
accgaatcca aagtcctcgc cctctacaaa gacggcgagc aagtcaacga attgaacgaa 1628520
ggcgacagcg gcgcagtcgt catcgacttt accccgttct atgcagaatc cggcggccaa 1628580
gtcggcgatg tcggctatat cttctcaggc gaaaaccgct ttgaagtacg cgatacccaa 1628640
aaaatcaaag cggccgtatt cggtcaattc ggcgtacaaa cttcaggccg tctgaaagtc 1628700
ggcgacagcg ttaccgccaa agtggacgac gaaatccgca atgccaatat gcgcaaccac 1628760
agcgcaaccc acttgatgca caaagccctg cgcgatgtat tgggcagaca cgtcgaacaa 1628820
aaaggctctt tggttaccgc cgaatccacc cgtttcgaca tttcccatcc ccaagcggta 1628880
actgccgaag aaattgccga agtagaacgc cgcgtcaacg aagccatttt ggcgaacgtt 1628940
gccgtcaatg cagccattat gagcatggaa gacgcgcaaa aaaccggcgc gatgatgctc 1629000
ttcggcgaaa aatacggcga agaagtgcgc gtactgcaaa tgggcggttt ctctaccgaa 1629060
ttgtgcggcg gcacacacgt ttcacgcacc ggcgacatcg gcctcttcaa aatcatcagc 1629120
gaaggcggta ttgccgcagg cgtgcgccgt atcgaagcca tcaccggcct gaacgcactc 1629180

aaatgggcgc aagagcaaga gcgtttggtg aaagacatta ttgccgaaac caaagcccaa 1629240
accgaaaaag acgtactggc aaaaatccaa gcaggcgcgg cacacgccaa agcattggaa 1629300
aaagaattgg cacgcgccaa agccgaactc gccgtccacg caggcgccaa actcttggac 1629360
gatgcaaaag acttgggcgc agccaaactc gttgccgccc aaatcgaagc cgacgcagcc 1629420
gccctgcgcg aaatcgttac cgatttaacc ggtaaatccg acaacgccgt gattctttta 1629480
gcggcagtaa acgacggcaa agtctccctg tgcgccggcg tatccaaacc gttgaccggc 1629540
aaagtgaaag caggcgatct ggttaaattt gcagccgaac aagtcggcgg caaaggcggc 1629600
ggcagaccag atttggcgca agccggcggc acggatgccg acaaattgcc cgccgtgttg 1629660
gatagcgtga aagactgggt cggcgcgaag ctggtttgat gtgggaaagg cagcctgaaa 1629720
ggtttcaggc tgccttttgt gcaaagaggc cgtctgaaag gtctcgtttg ccgtaggttg 1629780
ggtcgcgacc caacaaattt tgtgaagtat aaaaatgttg gtcatgaccc aacctacctg 1629840
cctttttgta caaagaggct atctgaaagg ccttgtttgc cgtatggtgg gtcgcgaccc 1629900
agcagatttt tattagggta tgacccaagc tacttgctac gataaaaaag gatttttaaa 1629960
tgagcattag ccttattgga ctacacatta ccatagcaat cattttgttt tttactacaa 1630020
attttatggg aaaaaaatca tctatatttg gctattacca actgtctttt agcgaagaaa 1630080
atcactctcc ggcatttaat attttttaca gagcatttac ccctatatta tttatcgtta 1630140
ttttttcttg ggttgttact agtcttgaaa ttcccatttc tcttgaaaag ataaactatg 1630200
tagtaattta ttattttata attagattgt tatctgtatt tgtttttgag aaaacacaca 1630260
tagttaactg gtttaatcaa ctaacaatac ccatactatc cataacatta tcatttatag 1630320
tatataacaa aatgattttg cccaaaagtt ttctacttcc atcctcacaa gaagtagcta 1630380
ctactttttg aatagcgctt ggtggttaca tatataatat attaaataat gaatcagggc 1630440
atttaaaatc ttataaagaa agaagagtaa attatgtaaa acacatgcac aaaaaatttg 1630500
aaagttattt tggtaaaatt atagataaaa taatcaaaga ggatagttat aataatgatg 1630560
attttttaac cgataagaaa aaagcactaa tatattcagt tttaatttat gagaatttta 1630620
ataggggact agtttataga tattttgaaa aaaattattt tgtactggta gaataaaaac 1630680
atttggaata atgcaagtaa cctcagcaga gtacctttcc aatgaggaaa gtataaaaaa 1630740
aggcggaaat attcttatgg aaaaatacaa tgaaaaatat aatgaatcta ttgatggcaa 1630800
taaaactctc tataaatcat attatgaatc aagaagagag agtattaaaa actacaaccc 1630860
agatgcaaaa tacattaatg aaattgaatc aatttacatg atgcttggag aaatctatcc 1630920
aaatgcacca gacttcatgt caccacattt tgagggggac tgctctgagg gggaataaaa 1630980
tcattattta ttctttatta gttattagca ggatttgtcg ggcataaatg cccgacctac 1631040
aaattcaatt ttttcaaacc tctgccaaat attttcatct ttgcaaggct gtctgaaaac 1631100
ccaaacccca ttttcagacg gccttttttc gctaaaatcc ccataccgtt caatccgaaa 1631160
acacaggaga atcatcatgg aagttaccat ctccgccatc atcaatggcg aatttgccga 1631220
ccaatacggc aagcgcggta gtcagtttaa tgaaaacggg atgctgattt aattctattt 1631280
cctttgaaac taccaataac ctgcctccat cataaaacta aaagcaagcc gtagcctgca 1631340
ttcccacaaa ccgcgtgcgt tgccatgtca cacaccctac ctgcgggcga cgcaaacctt 1631400
aagagacctt tgcaaaattc cccaaaatcc cctaaattcc caccaagaca tttaggggat 1631460
ttctcatgag caccttcttt caacaaaccg cccaagccat gattgccaaa cacatcgacc 1631520
gcttcccgct attgaagttg gaccgggtga ttgattggca gctgatcgaa caatacctga 1631580
accgtcaaaa aacccgttac cttagagacc accgcggccg tcctgcctat cccctgctgt 1631640
ccatgttcaa agccgtcctg ctcggacaat ggcacagcct ctccgatccc gaactcgaac 1631700
acagcctcat tacccgcatc gatttcaacc tgttttgccg ttttgacgaa ctgagcatcc 1631760
ccgattacag caccttatgc cgctaccgca accggctggc gcaagacaat accctgtctg 1631820
aactgttgga actgattaac cgccaactga ccgaaaaagg tttaaaaata gagaaagcat 1631880
ccgctgccgt cgttgacgcc accattattc agaccgccgg cagcaaacag cgtcaggcca 1631940
tagaagttga cgaagaagga caaatcagcg gtcaaaccac accgagtaag gacagcgatg 1632000
cccgttggat aaagaaaaac ggcctctaca aactcggtta caaacaacat acccgtaccg 1632060
atgcagaagg ctatatcgag aaactgcaca ttacccccgc caatgcccat gagtgcaaac 1632120
acctgtcgcc gttgttggaa ggtctgccca aaggtacgac cgtctatgcc gacaaaggct 1632180
atgacagtgc ggaaaaccgg caacatctgg aagaacatca gttgcaggac ggcattatgc 1632240
gcaaagcctg ccgcaaccgc ccgctgtcgg aagtgcaaac caagcgtaac cgatatttgt 1632300

cgaagacccg ttatgtggtc gaacaaagct tcggtacgct gcaccgtaaa ttccgctatg 1632360
cccgggcagc ctatttcgga ctgattaaag tgagtgcgca aagccatctg aaggcgatgt 1632420
gtttgaacct gttgaaagcc gccaacaggc taagtgcgcc cgctgccgcc taaaaggcag 1632480
cccggatgcc tgattatcgg gtgtccgggg aggattaagg gggtgtttgg gtaaaattag 1632540
gcggtatttg gggcgaaaac agccgaaaac ctgtgttggg atttcggttg tcgtgaggga 1632600
aaggaatttt gcaaaggtct ccagcagttt gcgcatacat gccgtaacgg caaccttata 1632660
cggcttaccc tcggacagcg ggcgttggtg gaaatcccga ataagcggtt caaaacgtgt 1632720
cgctgccacg gtagccatat acagtgcctt aagcaccgca gaccttccgc caaagcagcg 1632780

gcttttgaat ttggcttccc cgctcttcct cgggtgcggg gcaatgccga ccaaactcgc 1632840
tatccgtttg tgcgacagcc gccccaattc aggtagcatc gccatcagcg tagccgtcgt 1632900
tatcgaaccg atgcctttga tttgctccgc cacttgggct ttgccgtcaa aatgcgtgtg 1632960
ggtgtggtcg tcgatttgtt tgtccgattc gtcaatcagc cggtcaaaat gggcaatcag 1633020
ttgtttgacg cttccgactt gcgtttcgtg aacctgatgc agacggtttt tctcggcagt 1633080
ccgcatatcc gccgattggt tgcggcggtt aaccaaggct tccaacactt cttccgcttc 1633140
tgtgggcggg tggtagggca tggtttgcca atcttctttc tgtgccttca tctgtgcgaa 1633200
gaaggcaggc attttggcat ctttggcgtc ggttttggtc agcgactgcg attgggcaaa 1633260
ctgatgcgtc tgacgcgggt tggcgataat cacggctatg cctgctcggt ggatggcttt 1633320
ggcggcgggg atttcgagac ctccggtact ttccgtcacg acgagggcga ccttgtgttt 1633380
tttaaggtat tcgatagtat gggcgatacc tttggggttg ttggtttcgg ttttggtttt 1633440
agacaaagac gaaacggcga tgacgaagtt tcgtttggcg atgtcgatat agtgaattaa 1633500
caaaaatcag gacaaggcgg cgagccgcag acagtacgga tagtacggaa ccgattcact 1633560
tggtgcttca gcaccttaga gaatcgttct cttcgagcta aggcgaggca acgtcgtact 1633620
ggtttttgtt aattcactat atctgtgcgt tacgacggca tgccgtctga agggtgttta 1633680
tgtctgcatc taagaaattt ccgattcctt tgagctattt cagcatcgcg ctgggcttgt 1633740
ttgccttggg gctgtcgtgg cgttacggcg cgtctgtcgg gctgctgccc gccttggccg 1633800
ccgaatcgct gcttgcggcg gcttcggtcg tctggctctt gctggtggcg gcatacctga 1633860
tcaaaatgtt tgcgtaccga aacgattttt tgtctgattt acgcgacttg gtgcaatgct 1633920
gcttcatcag cgcgattccg attaccgcta tgctggaggg actcgcgctg aagccctatc 1633980
aggcaggcgc ggcggcagtc ctgatttatg tcggcgttgc cggacagttg gctttttcga 1634040
tgtatcgggc ggccggtctg tggcgcggcc tgcattcctt ggaggcgacg acgccgatta 1634100
tttatctgcc tacggttgcg acaaactttg tcagcgcgtc atctctggcg gcgttggggc 1634160
atcatgatta tgcagctttg tttttcggcg cgggtatgtt ttcctggctg agcttggaag 1634220
cctccatctt gggcaggctg cgcacggcgg caccggtcgg cacggcggcg cgcggcgtgg 1634280
tcggcatcca gcttgcgccc gcctttgtcg gctgcggcgc gtattttgcc gtcggcggta 1634340
aagtcgacgg ttttgcgttg gcattaatcg gctacggctg cctgcagctt ttgttcttgc 1634400
tgcgcctgac ccgctggttt tgggaaggtg gttttacgat gagcttttgg ggattttcat 1634460
tcggtttcgc ggcaatggca ggatgcggtc tgcatctggc ggcttccggc gtattgtcgg 1634520
gcttggggct gacgcttgcc accgccggat cggcaggcgt ggcgctgctg cttgtcggta 1634580
cgctgcaccg gatagcgacg gggcgtttct tggtacgcag ctgatgcgtt ttgccgcctt 1634640
gtcaaaaatg ccgtctgaaa cgctgggatt cagacggcat tttttatttc acacccttac 1634700
aggtagaatt tttcgatgac tttcaaattg tcgtccaatt tgtacaccaa cggctgaccg 1634760
gtcgggattt ccaagcccat aatgtcttcg tcggaaatgc cctcgatgtg ttttgccagc 1634820
gcgcgcaggg agttgccgtg cgccgccacc aagacgcgtt tgccgctcaa aatcgcgggg 1634880
gcgatttggt cttcccaaaa cggcaatacg cgctccagcg ttactttcag gttttcgccg 1634940
tcgggtacga catcggcagg cagatgggca tagcggcggt ctttgtgtgc ggaaaactca 1635000
tcgtctttgt ccaaaagcgg cggcagggtg tcgtagctgc gccgccagat gcggacttgc 1635060
tcgtcgccgt attgttcggc ggtttgtttt ttgtccaggc cttgcagttg gccgtagtgg 1635120
cgttcgttca gccgccacgt tttgatttgc ggtacgaaca gttggtcgga ttcttccaaa 1635180
acgatgttgc aggtcttaat cgcgcgggtc aggacggatg tgaaggcgat gtcgaactca 1635240
tagccgtttt ctttcagttt cttgccggcg gcggcagcct cggcaagccc ctgctcgctc 1635300
agcttcacgt cgcgccagcc tgtaaacagg tttttcgcgt tccattcgct ttgtccgtgg 1635360
cggataaata ccagttccat atcgtctcca atgtgtgaaa gtgggaaagc cttatttata 1635420
acatattttc acatttcccg tatttgattc agattcagac acgcgcccac tatggtttgc 1635480
cgttttgatt tacaataatg tcctttgctt tacattccgc atacacaatg aatacgcaag 1635540
cgcacgcccc acataccgat tccaatacgc tgatgctcgg ccgatacgcc gaacgcgcct 1635600
atctcgaata cgccatgagc gtggtcaaag gccgcgcgct gcctgaagtt tcagacggcc 1635660
agaagcccgt gcagcggcgc attttgtttg ccatgcgcga tatgggtttg acggcggggg 1635720
cgaagccggt gaaatcggcg cgcgtggtcg gcgagatttt gggtaaatac cacccgcacg 1635780
gcgacagttc cgcctatgag gcgatggtgc ggatggcgca ggattttacc ttgcgctatc 1635840
ccttaatcga cggcatcggc aacttcggct cgcgcgacgg cgacggggcg gcggcgatgc 1635900
gttacaccga agcgcggctg acgccgattg cggaattgct gttgtccgaa atcaatcagg 1635960
ggacggtgga ttttgtgccg aactacgacg gcgcgtttga cgaaccgctg cacctgcccg 1636020
cccgcctgcc tatggtgttg ctcaacggcg cgtcaggcat tgcggtgggc atggcgaccg 1636080
agattccgcc gcacaatttg aacgaagtga cgcaggcggc gattgcgttg ttgaaaaagc 1636140
cgacgctgga aaccgccgac ctgatgcaat atattcctgc cccgatttt gccggcggcg 1636200
gtcaaatcat cacgccggcg gacgaattgc gccggattta tgaaaccggc aagggcagcg 1636260
tgcgcgtgcg tgcgcgttat gaaatcgaaa aattggcgcg cggacagtgg cgcgtcatcg 1636320
taaccgagct gccgccgaac gccaattccg ccaaaatcct tgccgaaatc gaagagcaaa 1636380
ccaacccgaa accgaaagcg ggtaagaaac agctcaacca agaccagctc aataccaaaa 1636440

agctgatgct ggatttaatc gaccgcgtgc gcgacgagtc cgacggcgaa catcccgtgc 1636500
gactggtatt cgagccgaaa tccagccgca tcgataccga taccttcatc aacacgctga 1636560
tggcgcaaac ttcgctggaa ggcaatgtgt cgatgaactt ggtgatgatg ggtttggaca 1636620
accgccccgc gcagaaaaac ctgaaaacga ttttgcagga atggctggat ttccgcaccg 1636680
taaccgtaac acgccgtctg aaattccgtt tgaaccaagt ggaaaaacgg ctgcacatcc 1636740
tcgaaggccg tctgaaagtc tttctgcaca tcgacgaagt gattaaagtc atccgcgaat 1636800
cagacgaccc gaaagccgat ttgatggcgg cgttcgggct gaccgaaatc caagccgaag 1636860
acattttgga aatccgcctg cgccagttgg cgcgtttgga gggtttcaaa ctcgaaaaag 1636920
aattgaacga gttgcgcgag gaacaaggcc gtctgaacat ccttttgagc gacgaaaacg 1636980
aaaaacgcaa gctgattgtc aaagagatgc aggcggatat gaaacaatac ggcgacgcgc 1637040
gacgcacgct ggtggaagag gccggacgcg ccgtgctgac gcagaccacc gccgacgaac 1637100
ccatcacgct gatcctgtcg gaaaaaggct ggatacgcag ccgcgccgga cacaatctcg 1637160
atttgagcca aaccgcgttc aaagaaggcg actgcctcaa acaaaccctc gaaggcagaa 1637220
cggttttacc cgtcgtcatc ctcgattcat cgggcagaac ctacacgctc gatgccgccg 1637280
aaatccccgg agggcgcggc gacggcgtac cggtttcctc cttaatcgag ctgcaaaacg 1637340
gcgcgaaacc cgttgcgatg ttgacaggat tgccggaaca acattattta ttatcaagca 1637400
gcagcggcta tggcttcatc accaagctgg gcgatatggt cgggcgcgtg aaagcgggca 1637460
aagtggtgat gaccgcagac agcggcgaaa ccgttttgcc gccggttgcc gtctatgcct 1637520
cctcgttcat caaccccgac tgcaaaatca ttgccgccac cagtcaaaac cgcgccctcg 1637580
ccttccccat cggcgaattg aaaattatgg cgaaaggcaa agggctgcaa atcatcggat 1637640
taaacgccgg cgaatcgatg acgcataccg ccgtttcttc cgagctggaa atcctgattg 1637700
aaagcgaagg caggcgcggc gcggcgcaca aagaccgcat ccccatctcc ctgcttgagg 1637760
caaaacgcgg caaaaaaggc agactattgc ccatatcggg cagcctgaaa cagctttctt 1637820
cccctaaata aacccggttc cgcacatatt atggtgattt ccaaccccccg cgaacttgaa 1637880
aaactcaaag accggattcc caatctgatc aacatcatcc gcgtcgccat cgtttttccg 1637940
ctgatgatta tgcacatcct cgggctggaa accggcagcc gtgcgaacct gcacgcttcg 1638000
tggacggcgt gggcgtttta tgtttggctc gccattgcct gctggctgat tttcttttcc 1638060
attatccatc cgcattggca atggcagtcg ctgaaaatgc cgcgtttcag cgcggtagcg 1638120
gacatcacga tgatcggcgt gctgacctac ctgttcggcg gcatcgattc cggcttcggc 1638180
atcctgatcc tgcccttcgt cgtctgctcc tgcctgctca gctacgggcg ctaccccctg 1638240
ctctattcca gctacgccgc catcctgctg atattcaacg ccattgccga cggcgatatc 1638300
ggcaaatacc cgctcatatc ggatgcccga accgcctcgg caaccttcat ccttgtcgcc 1638360
gcctcctatc tttccgccat cttcacctca ctgtcggtca aatacatcga ccgtgccgga 1638420
aaactcgcct acgacagcca tatcgcctac caccgcatca aaggcttgag ccaaaccgta 1638480
ctcgaacgcg ttcaggaagc tgtcgtcgtc atcaatgccg aagggctggc ggtgctgttc 1638540
aaccggaagg cgaaagacct tttccccgcg ctcgaaatcg gacggcgcgc cggtctgtcc 1638600
gattctgccg ccgaactgtg ggatcaagcc tctccgcaca ctttcgaata cgtcctcggc 1638660
acacccggcc tgaacgccgg catccgcgcc gttccggtca acaaagggtc ggacaagctg 1638720
ctcatcctct acatccgccc gcaaagcgaa attcaggcag aagccctgtc cgtcaaactt 1638780
gccgcgctcg gacaactgac cgccaacctc gcccacgaaa tccgcaaccc gatgtccgcc 1638840
atccgccacg ccaacgacct gctgcgcgaa aatatggaag cgggggcggc agatccgttc 1638900
aacgccaaat tgtgcaaaat catcgacggc aacatctgcc gcatcgacaa aatgctcgaa 1638960
gacatttcct cgctcaacaa gcgcaacaaa accgaacgcg aaaccatcgg cctgataccg 1639020
ttttgggaag aattcaaaca agagttcctg ctcggccatc ccgatgccgc cgactgcatc 1639080
cgtccggaca ttcaaggcgg cagcccgacc gcctatttcg atcccgccca cctgcggcaa 1639140
attatgtgga acctcgccaa caacgcgtgg cggcacagcc gcaaacagcc cggctcgatt 1639200
tccgtcacca tccgccccgc gcaaaaaaac accgtctgta tcctctttgc cgaccgcccg 1639260
aagtgcagga acacctgttc gaaccctttt acaccacggc ggaaaacggc accggcctcg 1639320
ggctgtatgt cgcccgcgaa ctggcgcacg ccaatttcgg cgatttgacc tacctaccgg 1639380
aagccaaatg tttcgaactc acattaccgg aaaaaaccaa tgactgaact gcaacacccc 1639440
gtcctcgtcg tcgatgacga aaccgacatt ctcgacctga tggaaatgac cctgatgaaa 1639500
atgggcttgc gcgtccatac cgcgtcaggc gttgccgaag ccaaaaaacaa gctcgacagc 1639560
caacgctatt cgctcgtcct gaccgatatg cgtatgccgg acggctcggg gctggaagtc 1639620
gtccaacaca tcaacagccg cctgctcgat acgccggttg ccgtcatcac cgccttcggc 1639680
aacgccgatc aggcacagga agcgttgcgt tgcggcgcgt tcgaccccga taccatgcag 1639740
atacaggact atctcgacca aatcgaacgc gacatcatcg aacaaaccct caaacaaacc 1639800
gaaggcaacc gcacgcaggc cgccaaacgc ttgggcatca gcttccgttc catgcgctac 1639860
cgtatggaac gcctcaacat cggctgacga caaaacggca tccgcaccat ctccgcccac 1639920
ccgaaaaaat gccgtctgaa acggcacggg aaagcgggtt cgccccacgc ccgaacggac 1639980
acaaaacacc atgaccgaca tccttattga caacaccgcc accgaaaccg tccgcaccct 1640040
gatacgggca ttccccccttg tgcccgtttc ccaaccgccc gaacaaggca gttacctcct 1640100

tgccgaacac gataccgtca gcctcaggct tgtcggggaa aaaagcagcg tcatcgtcga 1640160
ttttgcctcc ggcgcggcac aataccggcg cacaaaaggc gggggcgaac tcatcgccaa 1640220
agccgtcaac cacaccgcgc accccaccgt ttgggacgca accgcaggat tggggcgcga 1640280
cagcttcgtc ctcgcctcgc tcgggctggc cgttaccgcc ttcgagcaac atcccgccgt 1640340
cgcctgcctg ctttcagacg gcatccgccg cgccctcctc aatcccgaaa cgcaaaacac 1640400
cgccgcgcac atcaacctcc atttcggcaa cgccgccgaa caaatgcccg cacttgtcca 1640460
aacacaaggc aaacccgaca tcgtctatct cgaccccatg tatcccgaac gccgcaaaag 1640520
tgccgccgtt aaaaaagaaa tgacctactt ccaccggctc gtcggcgaag cgcaagatga 1640580
agcggcactc ctgcataccg cacgccaaac agcaaaaaaa cgcgtcgtcg tcaaacgccc 1640640
ccgcctcggc gaacaccttg ccggacaaga ccctgcctac caatacacag gcaaaagcac 1640700
ccgcttcgac gtttacctgc cctacgggac ggacaaggga taacgcccat aaaacaagac 1640760
accgaaaaat ttgccgttct tatgcaaacg agaaaccggt ttttgcgttt cgactgtttt 1640820
ggataagtca tcacacctta aagtttgtca ttcccacaga agtgggaatc cgattcattc 1640880
agttttatag tggtttaaat ttaaaccact atagttgttt tcgagtttca ggcaacttcc 1640940
aaaccgtcat tcccacggaa gtgggaatct agaaatgaaa ggcaacagga atttatcgta 1641000
aatgactgaa accgaacgga ctagattccc gcctacgcgg gaatgacggg gcgggcagat 1641060
gccgtctgaa attccgtcat tcccgtgaaa acgggaatct agaacttctg atttttcaga 1641120
cgacttttga acattgccgc cacccaatga tctggattcc cacctgcgcg ggaatgacga 1641180
ggtttcaggt tgctgttttt aagttgctgt ttcgggttgc tgttttttat ggaaatgaca 1641240
aggttttaga ttgcgagaat ttatccgctc ctccgtcatt cccacggaag tgggaatcca 1641300
gaaatgaaaa gcaacaggaa tttatcataa atgaccgaaa ccgaacggac tagatttccg 1641360
actgcgcggg aatgacgggg cgggaggatg ccgtctgaaa ttccgtcatt cccgtgaaaa 1641420
cgggaatcta gaacttctga tttttcagac gacttttgaa cattgccgct acccaatgat 1641480
ttggattccc gcctgcgcgg gaatgacgat gtaaaattat ccgggattca aaaagacagg 1641540
ctttcacatc cgtgggaatg actgcggaaa gatgattttt atagtggatt aacaaaaatc 1641600
aggacaaggc gacgaagccg cagacagtac aaatagtacg gcaaggcgag gcaacgccgt 1641660
actggttttt gttaatccac tatattttgt cataaaaatc cgcaccttaa tcagttggcg 1641720
gttaaatcaa acttttaggg tgcagattac tttttatgat ttcagacagc attttgacag 1641780
gcggcagcct atttcggcaa taccaaaaac ttaatcagca gttctttgaa tacaaaaccg 1641840
aacacgccca agcccaaaac caaaaacaaa atggcgatgc cgaatttgcc tgctttggac 1641900
tccttgccca aattccaaac gataaaaccc aaaaaaataa tcaagccggt caggcagatt 1641960
ttcaacgccc aatcggcaaa aaccgcttca tccatatttt tttcctattg ttgatgtgta 1642020
tgccatataa gataagggtt tcagacggca tctgctgtcc aatgccgtct gaaacacgca 1642080
atcagcgtgc gagtgcctgt ttcaaatcgt caatcaaatc gccaacatat tccaaaccga 1642140
ccgacaggcg caccaatccg gggcggatgt tggcggcgag tttttcttcg ggctgcatcc 1642200
tgccgtgcgt ggttgtccac gggtgggtaa tggtcgagcg cacgtcaccg aggttggcgg 1642260
tgcgggaaaa gagttccacg ccgtccacaa ctttccacgc cgcttcttga tcggcaactt 1642320
caaagccgat gacgatgccg ccgccgtttt gctgtttgcg gataagcgcc gcctgaggat 1642380
ggtcggacaa tccggtgtag tacacggctt gaacctgcgg ctgcgcttgc agccattgtg 1642440
cgattttcag ggcgttgtcg aactgttttt ccatacgcag cgacagggtt tccacgccgc 1642500
tcaacaactg ccacgcatta aacggcgaca tcgccagccc gcaagagttg caatacatgg 1642560
cgacctgcgc caacaactct tccgaacccg ccaacacgcc gcccatcaca cgcccgtgtc 1642620
cgtctatggc tttggtcgcg gaggaaacgg aaatatccgc accgtgtttc aaaggctgcg 1642680
agccgacggg cgacagcagg ctgttgtcca ccaccaagag cgcgccgatg ccgtgcgcca 1642740
attccgccaa ggcttccaag tcggccactt cgcctaaggg gttggacggc gtttccaaaa 1642800
acagcagttt ggtattggct ttgacggcgg ctttccattc gtttatatca gtcggcgaca 1642860
cgtggctcac ttcgatgccg aatttggcaa cgatgttatt gataaagccg acggtcgtgc 1642920
cgaacaggct gcggctggaa atcacatggt cgcccgcctg caaaaaggtg aaaaacgccg 1642980
cctgaatcgc agacataccc gccgaagtgg cgaccgcgcg ttccgcacct tccaaagcgg 1643040
cgatgcgttt ttcaaaggcg gctgtggtcg ggttggcggt acgggtataa gtgaaccctt 1643100
tgattttttt tgaaaacaaa tcggcagcgt gttgggcgtt gtcccacatg aagctgctgg 1643160
tcagaaacaa tgcctgattg tgttcgcggt attcggtttg ttctttgccg ccgcgtatgg 1643220
cgagcgtttg cggatggagt tttttgctca tcggtgattc ctcggttttg tccgttcggc 1643280
aacggagcgt gcgcccgttg tttaatttgt taatattttg cgcctgttct atgatgcttt 1643340
caagtcggat gagaatgcaa atgccgtctg aaacggcttt cagacggcat ggcaatcagc 1643400
gtttgtattt taactcgtac ttgatgtcgt tgaggatttt gcggacatcg tgttccaaca 1643460
cgtcttcgac taccgccccc gcctgctcgt gcagcatctg ctggagctga taggtgaaaa 1643520
ccgccatctg cttttgcacc gccgttcgga tgatgccgtt gacggtatcg gtcagatgcg 1643580
ggcgcaggcg tttgatcagc cgttcggtca gctcctgttc ggacaggcag aacacttcgc 1643640
gccggttgac ggctttcggg ttcaggatat tgatttggac gggcatcaac gtttcttccg 1643700
catcgttttc cccgttttcc gaaaccgccg gctcattcgt gccggattct gcctcgtcgg 1643760

cgttttcccc gctttcaatc tgtccggttt caaattcgac actgtctttt ttggtatcaa 1643820
accggattct ccgccgcgat tcgatgtgtt tttccgaaac cgacatttgc agggaagcct 1643880
gcgcgttgag ccagttttcc tgaaggacga tcatcgggtc ggtttcgact tcctcgccgc 1643940
aatcggcaac ggcggcattg tgttcctcct gccatttttt cagatacgcc ttcaacacac 1644000
gggctcggct ctcatcgtcc agtttcggca caggcgcgtc cgttccggtt tcagaggggc 1644060
gggacagcgg cgcgtaagtc ggcactgcct tcatacggcg cgtctgacgc aggttttcca 1644120
aacgtttttc ccaattcggc tctttattcg catccatttt cggcttccgg ttcttaatct 1644180
ttgcaagcag acaaacccgc gcccaaagcg cggtttgata taatggcgca ttttaacaga 1644240
ttcgcgagga tacatcatgg gcagcatcga acagcgtttg gaatatctgg aagaggcgaa 1644300
cgacgtgctg cgtatgcaga accacgtcct gtccaccgca ttcaaagcct taatccgcgc 1644360
ccttcccgcc gaaaccgccg aaatcgcggt cgagtcgatt cagcttgctt ttgaggacgc 1644420
cttggcagaa ttgagctatg aggacagccc gcatacggat ttgttccacg acgttactta 1644480
tgcgtttttc cgtgaaaaag aacgttaatt ttatgttaaa ctgatttttt aggctttttg 1644540
attaccgaaa ggaattttga tgaatatgaa aaaatggatt gccgccgccc ttgcctgttc 1644600
cgcgctcgcg ctgtctgcct gcggcggtca gggcaaagat accgccgcgc ctgccgccaa 1644660
ccccgacaaa gtgtaccgcg tggcttccaa cgccgagttt gccccctttg aatctttaga 1644720
ctcgaaaggc aatgtcgaag gtttcgatgt ggatttgatg aacgcgatgg cgaaggcggg 1644780
caattttaaa atcgaattca aacaccagcc gtgggacagc cttttccccg ccttaaacaa 1644840
cggcgatgcg gacgttgtga tgtcgggcgt aaccattacc gacgaccgca aacagtctat 1644900
ggacttcagc gacccgtatt ttgaaatcac ccaagtcgtc ctcgttccga aaggcaaaaa 1644960
agtatcttct tccgaagatt tgaaaaacat gaacaaagtc ggcgtggtaa ccggctacac 1645020
gggcgatttc tccgtatcca aactcttggg caacgacaat ccgaaaatcg cgcgctttga 1645080
aaacgttccc ctgattatca aagaactgga aaacggcggc ttggattccg tggtcagcga 1645140
cagcgcggtc atcgccaatt atgtgaaaaa caatccggcc aaagggatgg acttcgttac 1645200
cctgcccgac ttcaccaccg aacactacgg catcgcggta cgcaaaggcg acgaagcaac 1645260
cgtcaaaatg ctgaacgatg cgttggaaaa agtacgcgaa agcggcgaat acgacaagat 1645320
ttacgccaaa tattttgcaa aagaagacgg acaggccgca aaataagccc gcccgtccga 1645380
acacaatgcc gtctgaagcc ctttcagacg gcattgttca tcaatcggcc tacaatgaac 1645440
tgcctgctga tttctcccta ccgcaaagca acaggcaaag attacaaata tcaaaatccg 1645500
agtaaaacag tattttatta aaacaaattg ataatcaaga gattagaatt atgtattgtc 1645560
tttaccgtac aaacgctggc actatttcaa cctgataaaa aacagccttc aaaaaggttg 1645620
tttaaaacag cagcagacac ttaccgccac aaccttgaaa aggaacacaa tcatgaccgt 1645680
catcaaacag gaagacttta tccaaagcat ttgcgatgcc ttccaattca tcagctacta 1645740
tcatcccaaa gactacatcg acgcgcttta taaggcgtgg cagaaggaag aaaatcctgc 1645800
cgccaaagac gcgatgacgc agattttggt caacagccgt atgtgtgcgg aaaacaaccg 1645860
ccccatctgc caagacacag gtatcgcaac cgtcttcctc aaagtcggta tgaacgtcca 1645920
atgggatgcg gacatgagcg tggaagagat ggttaacgaa ggcgtacgcc gcgcctacac 1645980
ttgggaaggc aatacgctgc gcgcttccgt cctcgccgat ccggccggca aacgccaaaa 1646040
caccaaagac aacacccccg ccgtcatcca tatgagcatc gtgccgggcg gtaaagtcga 1646100
agtaacctgc gcggcaaaag gcggcggctc tgaaaacaaa tccaaactcg ccatgctcaa 1646160
tccttccgac aacatcgtcg attgggtatt gaaaaccatc ccgaccatgg gcgcgggctg 1646220
gtgtcctccc ggcatcttgg gtatcggcat cggcggcacg cccgaaaaag ccgtgctgat 1646280
ggcaaaagag tccctgatga gccacatcga cattcaagaa ttgcaggaaa aggccgcgtc 1646340
cggcgcggaa ttgtccacca ccgaagccct gcgcctcgaa ctctttgaaa aagtcaacgc 1646400
gctgggcatc ggcgcacaag gcttgggcgg actgaccacc gtgttggacg tgaaaatcct 1646460
cgattatccg acccacgccg cctccaaacc gattgccatg attccgaact gcgccgccac 1646520
ccgccacgtc gaatttgaat tggacggctc aggccctgtc gaactcacgc cgccgcgcgt 1646580
cgaagactgg cccgatttga cttacagccc cgacaacggc aaacgcgtcg atgtcgacaa 1646640
gctgaccaaa gaagaagtgg caagctggaa aaccggcgac gtattgctgt tgaacggcaa 1646700
aatcctcacc ggccgcgatg ccgcacacaa acgcctcgtc gatatgctca acaaaggcga 1646760
agaattgccc gtcgatttca ccaaccgcct gatttactac gtcggccccg tcgatccggt 1646820
cggcgatgaa gtcgtcggtc cggcaggtcc gaccacagcc acccgcatgg acaaattcac 1646880
ccgccaaatg ctcgaacaaa ccgacctctt gggcatgatc ggcaaatccg agcgcggcgt 1646940
ggccacctgc gaagccatcg ccgacaacaa agccgtgtac ctcatggcag tcggcggcgc 1647000
ggcgtatctc gtggcaaaag ccatcaaatc ttccaaagtc ttggcgttcc ccgaattggg 1647060
catggaagcc atttacgaat ttgaagtcaa agacatgccc gtaaccgtcg ccgtagatag 1647120
caaaggcgaa tccatccacg ccaccgcccc gcgcaaatgg caggcgaaaa tcggcatcat 1647180
ccccgtcgaa tcttgaggcg ccatgccgtc tgaacacaaa atctgccttc agacggcatt 1647240
tccgcccccg gttgcggtac aatccaccat ttcatcactc ggcgacccac accgtgaaaa 1647300
tcctcatttt aggcaacgga caggtaggtt ctaccgtcgc acaaaacctt gccgccatac 1647360
ccaacaacga cgtaaccgtt atcgacatcg acgaaaaagc attgcaggaa acaggcagcc 1647420

gcctcgatgt ccaaaccgtt ttcggcaacg gcgcatcccc cttcacatta gaacgcgccg 1647480
gcgcggaaga tgccgacttg ctgctcgcgc tctcccgcag cgacgaaacc aacatcgtcg 1647540
cctgcaaagt tgccgccgac ctgttcaaca tccccggccg catcgcgcgc gtccgttcca 1647600
gcgaatacct cgaatacctc agccccaagc tcgaaaacaa cgaaaacggc agcctttcca 1647660
tattcggcat aaccgaaacc atcagccccg aacagctcgt taccgaacag cttgccggcc 1647720
tgatagactg cccgggcgca ttgcaggttt tacgttttgc agacgaccgc gtgcggatgg 1647780
tcatcataca ggcgcggcgc ggcggactgc ttgtcggacg cagcattgcc gacatcgccc 1647840
aagatttgcc cgacggggcc gactgccaaa tctgcgccgt ttaccgcaac aaccgcctca 1647900
tcgtccccgc gccgcaaacc gtcatcatcg aaggcgacga aatcctattt gccgccgccg 1647960
ccgaaaacat cggcgcggtc atacccgaat tgcgccccaa agaaaccagc acccgccgca 1648020
tcatgattgc cggcggcggc aacatcggct accgtctcgc caagcagctc gaacacgcat 1648080
acaacgtcaa aatcatcgaa tgccggccgc gccgtgccga atggatagcc gaaaacctcg 1648140
acaacaccct cgtcctgcaa ggttcggcaa ccgacgaaac cctgctcgac aacgaataca 1648200
tcgacgaaat cgacgtattc tgcgccctga ccaacgacga cgaaagcaac attatgtccg 1648260
cccttttggc gaaaaacctc ggcgcgaagc gcgtcatcgg catcgtcaac cgctcaagct 1648320
acgtcgattt gctcgaaggc aacaaaatcg acatcgtcgt ctccccccac ctcatcacca 1648380
tcggctcgat actcgcccac atccggcgcg gcgacatcgt tgccgtccac cccatccggc 1648440
gcggcacggc ggaagccatc gaagtcgtcg cacacggcga caaaaaaact tccgccatca 1648500
tcggcaggcg catcagcggc atcaaatggc ccgaaggctg ccacattgcc gccgtcgtcc 1648560
gcgccggaac cggcgaaacc attatgggac accataccga aaccgtcatc caagacggcg 1648620
accacatcat ctttttcgtc tcgcgccggc gcatcctgaa cgaactggaa aaactcatcc 1648680
aggtcaaaat gggctttttc ggataaaccg ccccattccg gacatattgc cgccaagcgg 1648740
tatggaagcg gaaataatgg taggtgggct tcagacggca tccgccctcc ccgtcattcc 1648800
cgcgtaagcg ggcatccaga ccttgggata gcggcaatat tcaaaggtta taaaagaccc 1648860
gtcattcccg cgcaggcggg aatccagacc ttgggatagc ggcaatattc aaaggttatc 1648920
tgaaaattta gaggttctag attcccgctt tcgcgggaat gacgaaaagt tgcgggaatc 1648980
cagaacgtcg ggcaacggca atattcaaaa gccgtctgaa aatttaaaag ttctagattc 1649040
ccgctttcgc gggaatgacg aagtttcaga cggcatcgcc cgcctgtttt gatatagcgg 1649100
caccccccccg acaaaaaaac aatccggaac gcatctgacc gttccggctt gttttcaggc 1649160
gaatccgccg catcagaaca tactgcgcac gcccatattg acctgccaag tctagcgcat 1649220
cgtgtgcatc gaagaccttt gcgcctcaaa ataaagctgc cttccgttgt cggcattacc 1649280
acgcaaaaaa atgaattgct tgatattcca atgtttttta tatgttttta tattgtgatg 1649340
cgatcagaca aacgcccccc tgacatttgt ttagacggca tcgtattgct aaatttctat 1649400
aagtatgtat aatgtccgtt tccacgcgcc catcgtctag aggcctagga cactgccctt 1649460
tcacggcggc aaccggggtt cgaatccccg tgggcgtgcc aattcaaaaa cctgcttgtt 1649520
tcaagcaggt tttttattat gagtcgtcat tcccgcaatt tttcgtcatt cccgcaaaag 1649580
cgggaatcta gagcgtaggg ttgaagaaac cgttttatcc gataagtttc cgtgccgaca 1649640
ggtctggatt cccgcctgcg cgggaaggac ggcagagggt ggacgatgcc gtctgaagcc 1649700
tgacaaagca tttgatgccg tctgaaactt cgtcattccc gcaaaagcgg gaatctagag 1649760
cgtagggttg aagaaaccgt tttatccgat aagtttccgt gccgacaggt ctggattccc 1649820
gctttcgtag gaatgacgga attttaggtt tctgtttttg tggaaatgac gaataaagcg 1649880
tgccggttta tgctcgccgc aacacgcggt tcagacggca ttgctctctt ttttcattat 1649940
cagtgggtgt agcaactgta tttttcaccc cgtcgggcaa aaatacagtt gctacgatgc 1650000
accccgccgc cctgccctgt gccttgtcct gcaatacggc atataatgca ccacaaaccc 1650060
ccgcgctgcg gttttcagac ggcatcgccg tgcttttta caggcattag cccttttat 1650120
cggacgcaat attaaggagg aacaaatgaa aagctctttt gtgcaaacgc ttaccatcgc 1650180
cggttcggat tcgggcggcg gtgcgggcat tcaggcggat ttgaaaacat ttcagatgcg 1650240
cggcgtgttc ggaacgtgcg tcatcaccgc cgttaccgcg caaaatacct tgggcgtgtc 1650300
ggcggttcat ctcgtcccga ccgaaaccat caccgcacaa atccaagcaa tcagggaaga 1650360
cttcgacatc cgcgcctaca aaatcggtat gctcggcacg gcggaaatca tcgaatgcgt 1650420
tgccgacaag ctgaaacact gcagctttgg caggcgcgta ctcgaccctg tgatgattgc 1650480
caaaggcggt gcgccgctgt tgcaggattc cgccgttgcg gcactgacgc gcctgctgct 1650540
tcccgatacg gatgtattga cccccaacct gcccgaagcg gaagctctga ccggcgtgca 1650600
tattgaaaac cgtaaagatg cggaacgtgc ggcaaaaatc ctgcttgatt acggtgtcaa 1650660
aaatgtcgtt atcaaaggcg gacatttgaa cggcagcaca agcggacgct gcacggattg 1650720
gctgtttaca caaaatgaaa cgctggaatt cgacagcccg cgctttccga ccgcccacac 1650780
gcacggcacg ggctgcacgt tttccgcctg catcaccgcc gagttggcaa aaggctcgga 1650840
cgtttgcgaa gccgtacaga ctgccaaggc ctacatcacg gcggcaatct caaacccttt 1650900
ggaaatcggc gcaggacacg gcccggtcaa tcattgggcg tatcgggact aaccgtaaaa 1650960
atgccgtctg aaacaaaatg ttcagacggc atttttgagg attattcagg ctttttcgcc 1651020
agcatcgtta caaatttaaa ccgtatcgga ttgccgtttt cgtctttggc atgcatagaa 1651080

cccaattctt ctttatattc gaccagttcc caatcccgat aataatcctt cagctcgccc 1651140
tctttaaatt taaaagggaa cggcatcgga caggggaaat ccgccgtatc cattgccgat 1651200
acaatcaagt tgtacccgcc cgccgccgta tgcgcctgca tatcggcaat cacgtcgggt 1651260
acgcgctgcg gcatcaggaa catcagcacc actgttgcca caatataatc aaactcgccc 1651320
tgcaaggcgg cggcgttcaa atcatattcc agcgtgcgga cgttcaaacc ctccgcctct 1651380
gccagctccg ccacgtttgc caaggcggcg ggattgtgat cgactgcagt aacttcaaac 1651440
cccttcaaac cgagaaacag cgcgttgcgc ccctgtccgc agcccatatc caacgccctg 1651500
cccgccggta cggtatcccg tgccgccgcg accgcagaat gcgtggcact catcccgtat 1651560
tttttgtgaa aatagtctgc cgccgcgcaa tacagcgaca aacggatttc ggcatcgtcc 1651620
gttttcggtt tgacagaaaa cacctgctgc ggcgcaaaca cacaatcgcc gccgtctgcc 1651680
gaccaaactt ctgccgaccc gtccggtgca cgaacttcga catcgccctg caacacattc 1651740
aggcagaccc actccccttc ctcagacgaa tagcccgaca acaaaacttc cggcaggttt 1651800
tccactttcc atacaggcat ctgtccgaaa caaaacaact cgccactttg acccactatc 1651860
cgctccttca tattcaaaaa taaagttgca cattatatgc ctattttaat ccgccgcaat 1651920
ctttcagacg gcacggcgcg caaaccgctt ataatcacgc cggacaccac acaaaggcac 1651980
aataatgaac caaaccgttt acctttacac cgacggcgcg tgcaaaggca atcccggcgc 1652040
gggcggctgg ggcgtgttaa tgcgctacgg tagccacgaa aaagaacttt tcggcggcga 1652100
agcgcaaacc accaacaacc gcatggaact gactgccgtc atcgaaggac tgaaatcgct 1652160
caaacgccgc tgcaccgtca tcatctgcac cgactcgcaa tacgtcaaaa atggcatgga 1652220
aaactggata cacggttgga agcgcaacgg ctggaaaacc gcctccaaac agcccgtcaa 1652280
aaacgacgac ttgtggaaag aactcgacgc tctagtcgga cggcatcaag tcagttggac 1652340
ttgggtgaaa ggacacgcgg gacacgccga aaacgaacgc gccgacgatt tggcaaaccg 1652400
tggcgcagcg cagttttcct gactgccgct ccggcaaaaa tgccgtctga aaccgctaat 1652460
gggcttcaga cggcatcgtc ctccaccgtc attcccgcgc aagcgggaat ccaaaccgtc 1652520
gggcaacggc aatattcaaa gattatctga aagtttgaag ttctagattc ccgttttcac 1652580
gggaatgacg aaaagttgca agaatgacgg agtttcaggc ggcatccgac cgccccgtca 1652640
ttcccgcgaa agcgggaatc taaaaaccca acgctgcaag atttatcaga aacaactgaa 1652700
accgaacgga ctggattccc gcctgcgcgg gaatgacggg attttagtaa ccgtagcaac 1652760
cgcctgcgcg acggctaagg ggcttcagca accgtagcaa ctgcctgtgt gggaatgacg 1652820
gacaatgggc ttcagacggc atctcttgcc tgccgctaaa acagtttgcc gcacaactgt 1652880
tcaaacgcgt ccgatatgtt tcaacacaca ggacgacaca taaagcacct ccctatgtgt 1652940
cgtcctgatt tggaaggggt tacaccccct cccaaataaa gtctgatcct gccgccctaa 1653000
agggcggggt ttcaaccgaa aaggaaatac gatgaagtgg tacaattagc ggcaatgcgg 1653060
acagacaaat taaactatag tggattaaat ttaaaccagt acggcgttgc ctcgccttag 1653120
ctcaaagaga acgattctct aaggtgctga agcaccaagt gaatcggttc tgtactattt 1653180
gtactgtctg cggcttcgtt gccttgtcct gatttttgtt aatccgctat atcagaaatt 1653240
accctaccgt tttttaaaca ctttcaggaa taaggaaaaa tgaccgccca accctgcccc 1653300
atctgcacgg cgcaaaatga agacgttttg ctgcaaaccc ccaacctccg cgtcatcgcc 1653360
gtccataacg acagcggttc gcctgcattc tgccgcgtca tttggcgtaa gcatattgcc 1653420
gaaatgaccg acctttcggc agcggaacgc ggcgaattga tggaaatggt gtacaaagtc 1653480
gaagccgcta tgcgccaagt gttccggccg gcaaaaatca acctcgccag cttgggcaat 1653540
gtcgtgccgc acctgcattg gcatattatc gcccgctttg aaaacgatgc gtctttcccc 1653600
gcgccgattt gggcaaaccc cgtccggaaa cacggtatga ccctgccgca agattggacg 1653660
gaacagctta aaaagctgct ttaagcccgc cgatgccgtc tgaaaccgta tgaaagggaa 1653720
attatgaccg aaccgacctc ccgccgccgt tttctgaaaa cctgcaccgc cgctgccggc 1653780
gcggggctgc ttcaggcttg cggcacatcc gccacatccg ttccgcccct tccctcttcc 1653840
cattccgttg tgaaagcccg aaccgtgcct ctccaaacgc cacgccgtca aagttcggac 1653900
ggcaaccttc tgcgcgttgt cgcttcgtca ggatttgccg aagacaccaa ccgcgtcaac 1653960
acagccttaa cccgccttta caatgtcggt tttaccgtaa ccaaccaaca ggcgggcagc 1654020
cgccgtttcc aacggtttgc cggcacggac acgcaacgtg ccgccgattt ccaagaggtc 1654080
gcttccggcc gcgtcgccac gcctaaagtg ctgatgggtt tgcgcggcgg ttacggtgcg 1654140
gcgcggattc tgccgcatat cgattttgct tcgctcggcg caaggatgcg cgaacacggc 1654200
acgctctttt tcggattcag cgacgtatgc gccgtccagc tggcattgtt ggcaaaaggc 1654260
aatatgatga gttttgccgg cccgatggct tatagcgatt ttggcaaacc cgcccccggt 1654320
gcgtttacga tggatgcctt tatcaagggt gcaacccaaa accgcctgac cgttgatgtt 1654380
ccttatatcc aacgcgccga tgtcgaaacc gaaggcatat tgtggggcgg caacttaagc 1654440
gtcctcgcct cgctcgccgg cacgccttat atgcccgaca tcgacggcgg cattttgttc 1654500
ctcgaagatg tcggcgaaca gccctaccgc atcgaacgta tgctcaatac gctgtatctt 1654560
tcgggtattt tgaagaaaca gcgcgccatc gtgttcggca atttccgtat ggaaaaaatt 1654620
cgagatgtct atgatccgtc ttatgatttt tctgccgttg ccaaccatgt ttcgcgcacg 1654680
gcgaaaatcc ccgtgctgac gggcttcccg ttcggacaca ttgccgacaa aatcactttc 1654740

cctctaggcg cgcacgcccg aatccgtatg aacggaaaca gcggttattc ggtcgcgttt 1654800
gaaggctacc ccacactcga tgcgtccgcc ctgactttgg ataccctgct cccaccgccg 1654860
gatttgccca tcttccccga aagcggtgtt gccgatattt cggaataaac ccgcaaacgg 1654920
acaaatgccg tctgaagcct tcagacggca tttcccaaga cggcggcaga ttacagcaat 1654980
gcccgaatat cggcttcgat ttcttcgggc gtaacactag gcgcaaaacg ctcgaccact 1655040
tcgccgtcgc ggttgacgag gaatttggta aagttccatt tgatgtcgcc ttcgtcgcgc 1655100
ttctctccca aagctgcgag cttcaacacg aaatctttaa acagatgatt gcctttatct 1655160
tgcggtttga cggatttcag gtaggcatac aagggcgcgg tatttgctcc attgacttcg 1655220
attttgtcga aaatcttaaa cttcgtgcca aacttcatca tacacacttg ggcaatttct 1655280
ccgctgcttt cgggagcctg ttcgcggaac tggttgcacg gaaaatccaa aatctccaag 1655340
ccttctgcgg tatattgtgc atacagcttc tgcaaagcct cgtattgcgg ggtcagaccg 1655400
caacgcgttg ccgtgttgac aatcagcaga accttgccgc gatagcctga caaatcaacc 1655460
gcattgcctt ctgcatcttt catttgaaaa tcgtaaatac ccatttttat ccttatctga 1655520
tgtaaaccga tgccatctga aacgtgcttc agacggcatg aaagcagcaa ttgtatagcc 1655580
gattaaaata aaaaatccac atccttttcc attcccgtcc caatccgcaa taaaaaactg 1655640
cacccgaaaa cgggtgcagt tgctcatttc ataccgcaaa acttatttgt cgcggccgaa 1655700
tacgatttta gtggcttgga tggcgacaca gattgcaccg ccgataaaga ccaagtcagc 1655760
tgccgtacgt acccaacgca aggtatcgag gatttccatt tgcaggaact cttcgctgcg 1655820
ggcataccac agaccgtgcg tgatggaggc gtatgcctga atcgcgccga caggcagcag 1655880
gctgatggca atcataccgg ccaagccgcc gttgagcagc cagaagcccc aagtcatcag 1655940
tttgtcgtca aactgcgcgt tcggtttcaa ataacgggca accagcaata cgaagcccaa 1656000
tgccaagaaa ccgtacacac caaacaaggc ggcgtgcgcg tgaacggcag aagtgttcaa 1656060
accttggata tagaacaggg aaatcggcgg attgatcagg aagccgaata cgccggcacc 1656120
gatcatattc caaaaggcga ctgccacgaa gcacatcagc ggccaacgca ggcgtttcgc 1656180
ccagtcggac aggtgttggt aagaccagtg ttcgtatgct tcacggccca gcaacaccag 1656240
cggcacgact tccaaagcgg agaagcaggc accgattgcc atagaggcgg aggtagagcc 1656300
ggagaagtac aggtggtgca gcgtgcccgg aacgccgccc aacataaaga tggcggcagc 1656360
ggccaaagtg gaggcagtgg cggtactgcg gcggacaaag cccatattgt agaagacaaa 1656420
ggcaaaggcg gcagtggcaa atacttcgaa gaagccttct acccacaggt gaaccaccca 1656480
ccaacgccag tattccataa cggcaatcgg ggatttttcg ccatagaaca ggcctggtgc 1656540
gtagaatacg cccacaccga ccatagaagc tacgaagata gccaacaggt ttttgtccac 1656600
gcctttttct ttaaaggcgg aaaccgtgca acgcaacatc aggaacagcc ataacagcag 1656660
accgaccatc aaaaggagtt gccagaaacg tcccaaatcg aggtattcgt aaccttggtg 1656720
tccgaaccag aagttaaatt ccgggggaag gatgtgcgtc aacgcgaaga agttgcccgc 1656780
gtaagaaccg ccgaccacga tgaagagggc gatatagagg aagtttacgc cggcacgttg 1656840
gaacttggga tctttaccgc cgttgacaat cggcgcgagg aacaaacctg ccgtcaaaaa 1656900
gccggttgca atccagaaga tggcggattg gatgtgccaa gtacgggtca gggcgtaggg 1656960
gaaccagtcg gacatttcaa agcccaacgc ctcgtcaatg ccgtagaaac cctggccttc 1657020
gacggtgtag tgcgcggtca gtccgcccag caatacttgt accacaaaca gggcgaccgt 1657080
caggaagacg tatttgccca atgctttttg cgaaggggtc agttggattt tggaaatcgg 1657140
gtcttcagac ggcacttcca cttcctcgtg tttggtcagg aaggaataac cccacatcag 1657200
caaaccgatg cccatcagca gaagaacaac gctggtgaat gaccacatat agttttcagt 1657260
ggtcggtacg ttgttgatca aaggttcgtg cggccagttg ttggtgtaag taaaattctc 1657320
gtcaggacgg ttggtcgaag cagaccaaga agtccagaag aagaagttga acagtttttc 1657380
acgcgcttct tggcttggca atgtattgtt tttcattgca aagtgttcgc gagtggtttg 1657440
gaacttagga tcgtcgctgt acacaccgtg gtagtaaggc aggatgcttt cgatggcttt 1657500
cacgcgcgta tcgctgatga cgacgctgcc gtcttccttc acgcggcttt gattgcggta 1657560
ttcgtcggcc aggcgtgttt tcaagacggc ttgttcctcg gggaaacct cgtcgaattt 1657620
tttgccgtaa gtctgttgcg cggtcaaatc caaccaggca accaactcac gatgcagcca 1657680
gtccgccgtc cagtccggag cctgatatgc accgtgaccc aaaatcgaac cgacttccat 1657740
accgccggta gtctgccatg cagactgacc tgccaaaata tcgtctttcg tcatcaagac 1657800
cttgccggat gcggaaacga cctgttcggg gtaaggcggg gctttttgt aaacctcgct 1657860
gcccatatag ccaagaatgg taaagcatac cgccagaacg gcaaacagca agtaccaaag 1657920
cttcttgtac tgtcccattt tgagagctcc ttttaatata gtggattaaa attcacaaaa 1657980
tatgaatgtt aaagattgta gcacggttta ccgcgcaaat aaacatttgt tcaaagaaac 1658040
tcacatataa aacaaataca tatatgataa taactatcat tattctttag tcggcaacta 1658100
ccctgccttt gcctgatttg ccgaagccct taagcaaatc agcctattta ttgtaatttt 1658160
tagtagctat aaagtattag aagtatcatt ttaagttcat attttatgaa ttatttgact 1658220
taaatcaaaa tgcccccaat ggggcaaacg cataatcaca ccaagttctt aaccaatccc 1658280
tctacttttc ttacaaaagg aaaatattat gaaacgccaa gccttagctg caatgattgc 1658340
ttccttattc gcattagccg cctgcggcgg cgaacctgcc gcgcaagccc ctgccgaaac 1658400

ccctgccgct gccgccgaag ccgcaagctc cgccgcacaa accgccgccg aaacaccgtc 1658460
cggcgaactg cccgttatcg atgcggttac cacccacgct cccgaagtgc ctcctgcaat 1658520
cgaccgcgac taccccgcca aagtccgcgt aaaaatggaa accgtcgaaa aaaccatgac 1658580
catggaagac ggtgtggaat accgctactg gacatttgac ggcgacgttc cgggccgtat 1658640
gatccgcgta cgcgaaggcg atacggttga agtggaattt tccaacaatc cttcttctac 1658700
cgttccgcac aacgtcgact tccacgcggc taccggccag ggcggcggcg cggccgcaac 1658760
ctttaccgct ccgggccgta cttccacatt cagcttcaaa gccctgcaac cgggtctgta 1658820
catctaccac tgcgccgtcg caccggtcgg tatgcacatc gccaacggta tgtacggtct 1658880
gattttggtc gagcctaaag aaggcctgcc gaaagtggat aaagagttct acatcgtcca 1658940
aggcgacttc tacaccaaag gcaaaaaagg cgcgcaaggt ctgcaaccgt tcgatatgga 1659000
caaagccgtt gccgaacagc ctgaatacgt cgtattcaac ggtcacgtag gtgctatcgc 1659060
cggcgataac gcgctgaaag ccaaagcagg cgaaactgta cgtatgtacg ttggtaacgg 1659120
cggtccgaac ttggtatctt ccttccacgt catcggcgaa atcttcgaca aagtttatgt 1659180
tgaaggcggc aaactgatta acgaaaacgt acaaagcacc atcgttcctg ccggcggctc 1659240
tgccatcgtc gaattcaaag tcgacatccc gggcagctac actttggttg accactctat 1659300
cttccgcgca ttcaacaaag gcgcactggg tcaattgaaa gtagaaggtg cagaaaaccc 1659360
tgaaatcatg actcaaaaat tgagtgatac cgcttacgcc ggtaacggtg cagctcctgc 1659420
tgcttccgct cccgcagctt ctgccccggc agcctctgca tccgaaaaaa gcgtttatta 1659480
aattggatac ccgtcattag cgggacgaac cactgccgct gtacttcatt acgcacggcg 1659540
gtggtttttt aacaaccaat ctttcctttc ggaagattga ttttaaccgc ctgtcaggag 1659600
gctttatgaa gtatgtccgg ttatttttcc tcggcgcggc actcgccggc actcaagcgg 1659660
cggctgccga aatggttcaa atcgaaggcg gcagctaccg cccgctttat ctgaaaaaag 1659720
ataccggcct gattaaagtc aaaccgttca aactggataa atatcccgtt accaatgccg 1659780
agtttgccga atttgtcaac agccaccccc aatggcaaaa aggcaggatc ggttccaaac 1659840
aggcagaacc cgcttacctg aagcattgga tgaaaaacgg cagccgcagc tatgcgccga 1659900
aggcgggcga attaaaacaa ccggtaacca atgtttcctg gtttgccgcc aacgcctatt 1659960
gcgccgcaca aggcaaacgc ctgccgacca ttgacgaatg ggaatttgcc ggacttgctt 1660020
ccgccacgca gaaaaacggc tcaaacgaac ccggctacaa ccgcactatt ctcgattggt 1660080
atgccgacgg cggacggaaa ggcctgcacg atgtcggcaa aggccgcccg aactactggg 1660140
gcgtttatga tatgcacggg ctgatttggg aatggacgga agatttcaac agcagcctgc 1660200
tttcttccgg caatgccaac gcgcaaatgt tttgcagcgg cgcgtctatc gggtcgagcg 1660260
actcgtccaa ctatgccgcc ttcctccgct acggcatccg taccagcctg caatccaaat 1660320
atgtcttgca caacttgggc ttccgttgca caagccgata accccttcaa ttatagtgga 1660380
ttaacaaaaa ccagtacggc gttgcctcgc cttgccgtac tggtttttgt taatccacta 1660440
tattccgcca tctctaagat ttacagcgat acacgggtaa tttaaggaat gcccgaaccg 1660500
tcattcccgc cactttccgt cattcccgcc actttccgtc attcccgcca ctttccgtca 1660560
ttcccgcaac ttttcgtcat tcccacgaac ctacatcccg tcattcccac gaaagcggga 1660620
atccagtccg ttcagtttcg gtcatttccg ataaattcct gctgcttttc atttctagat 1660680
tcccactttc gtgggaatga cggcggaagg gttttggttt tttccgataa attcttgagg 1660740
cattgaaatt ctagattccc gcctgcgcgg gaatgacgat tcataagttt cccgaaattc 1660800
caacataacc gaaacctgac aataaccgca gcaactgaaa cgtcattccc accacttttc 1660860
gtcattccca ccacttttcg tcattcccac aaggacagaa aaccaaaatc agaaacctaa 1660920
aatcccgtca ttcccgcgca gatgatatgt tgcccgtcaa cacaaaataa aaaacaaagt 1660980
tgcaatatac tgatttatat tgttattttt atttacgttt atttacgata tgcaaatgca 1661040
cggttacaca aatatattcg cgtaaccgtt taattttgtt gaattttatt gattcaatcg 1661100
gtgtctttcc gcatcgtaag gctggccggt tttaacaatg taataggcga gcttcgccag 1661160
tttgcgcatg atggcaacga tgattaccat ctttggctta cccgcttttt tcagattatt 1661220
tattaatttc ggaaatgcgt taaaacggta agcacaaagg gcgggcatat acagcgtact 1661280
ttttaatcgt ctgtttccgt atcggctcaa tctgccccga cctcttacgc ttgtccctga 1661340
ttgtatgatg gcgggattta atccggcata ggatacaaac tggtttgcgg ttttaaaatg 1661400
tttttctgtc agttgcgcat aaagaactga tgcggtgtct ttgcctatgc tcgggatggt 1661460
ttgaagattg cggtaatggt tattgtccgt ttgttttttg atttgttcgg atatggctat 1661520
ttttacctgt tccatcttgt cctgtatggt atctatcaag tcttgatgta tgttccttat 1661580
gaagtcttct tcagtgctat gaagacggtt tttaatttgc ttctgatgtt gatgtaattg 1661640
atttttaagg ttaatcagtt tttgcagtgc tttgtttttg ggtatctgat acggtatcaa 1661700
tgtatcttga tgccttttta tgtagtctgc tatcaggttt gaatctgctt tgtcggtttt 1661760
ggtacggtta aacctgcttt ttccgtagtc cttgattttt aagggattaa taacgtaaac 1661820
agtatagtag gaagaaagca tatctgctgc cttttcgtaa tagatgcctg ttgcctccat 1661880
gccgatatag acttttctga ttctgtttcc ctttatccac aatctaaact gttttaatcc 1661940
atcatcatta ttcttaaatt taatgtaatg gatacttccg tttgttttat gcaatgttgc 1662000
gtctatggtg tcctttgaga tgtccagccc gattatattc attggtattt tccttattta 1662060

tacagccttg atacggctag gatgatattc aatttcgagg atggataaag gcagccggca 1662120
tttctacgcg tctgttttaa tacattgcgg gatttgctgc ctgactgcct tagcccttgc 1662180
tttgcgcgaa acaaagaccc gtaaaccgtc tatattcaaa cggtttacgg gtctttttc 1662240
tctcttgccg ttttcttcag tttgccgatc cgaccacgcc cccgccgatt ccttcaaacg 1662300
gtttcccgcg ttcttcccaa ttatcgtaca ttaggttctg ctacggtttt ccgcccaatg 1662360
tggcaacttg cgccctgtcc gaatgttgct gcgcgctttg ctgaacttcc tgcccttggc 1662420
tttcttcttt gtatgggtta aacggcaagc cgtttttttac atagtccttg cacatcaact 1662480
ccgtcacttc tttcaatgcc gtcccttgat gcgaatagca ggcgcatccg gttcttccgc 1662540
cttctataca gcctgctata tattcaaagg ttcttacctg ccttacaccg ttataaatcg 1662600
gcttgctttc gggtttttcg gacaatgtcg gaacaaacat atctgcggta aggttgccgt 1662660
tatttaccgg ctcgccttct gttttatccg gaagtactgc ctgctgttct gttgccgccg 1662720
attcttgtgc tgcgggttct tcctgttttt ttccgtaact gctcaacatt ttataggaca 1662780
ggccgacaaa cacgggaatc agcaatacta ttactggcag agtgtaaaac cactttgacc 1662840
gcttgacctt atttacggta tgaacttccg ctgattcgta caagtcataa acttttttat 1662900
ccagtgtata gatactggag aatgcgcttg atgccatttt tacgggatcg tccgcgcata 1662960
ttttccattc taaaagcgta cgcataccca tcttgtttga agcgatgtgg taatgtttcc 1663020
gtacaagcgt tctaagattt tgatctagaa gcttaggacc ttgagtcaaa acaaatatat 1663080
caatgccctg atgtctgtgc gtattcagcc attggacatt ttcagggatt tttgaacctg 1663140
ccgagcgtgc cggccatacg tcttgagctt catctacaat gacaatagac ccgatatttt 1663200
cgggcttctt tatccattcg tacatatcat gcgccgaaag ctgctcatct gtcgatttcg 1663260
gcagcttttt tgcgtccgtt tctatgtagg tgtgcggtat tttcaagcct tttatgttcg 1663320
taaatacttt acggcgtatg ccgttttcat caggcttaaa catttcatca ttcgccatca 1663380
tggaaaccat ttttaatgtt ttccctgaac cgggcgtgcc ggttatcaaa cagatctctg 1663440
ccatttattt tttcttcccg attgaggttg ctagttttgt catttgtttg aatgacagaa 1663500
taaaggcgat cgcgccaaac aggatattaa gaacggttcc accgccgctt atataaaaaa 1663560
gctgcaacat cgcttgaggc gcgcccgtta tgctattggt tatcgcctgc tgaaaatggg 1663620
ctaccaatct atccacccct gaataggtta ccgccatcaa gcctaatgca gtcaatatac 1663680
ggcctgccac gctcatcaaa agcggaatca atgcggccaa caatttcatt tgctatccct 1663740
ttcttaaaag gcacggttgc ctcattaaac aaatgtttct taatctgaaa gattttgcgc 1663800
cgcattcgcg gcgcggcggc gctgtgggga cacccccctc gcgcttatcg ccaatccccc 1663860
cgcgcttctc ggctatttgc gacattcgtc gcaaagtgcg ctgcttcgcc tgcctttcgg 1663920
ccaaagtttc cgaagctgaa cgctttgcgg gggactagtc ccccacaccc cctagtctca 1663980
cttgcgacgc cgcgggggca tggggacggc gcaaaaggcg cgcgccttac cacctgccct 1664040
tgcggcagaa tgtgttcttt tggcggggcg gcaaggggta tccaaaaaga tttataaaga 1664100
cgataaagcc gtctttacaa atctttctgg acgtcctccc cctgccttgg tacaagttac 1664160
tgaagcccgg cggtgctgcg cctgctagac ttcacgagat actgtgcgga tacaaaaaaa 1664220
ggcggcaacc gcccaagcaa gggcgagaag catgtacctt agccgttcgg ctatggtaca 1664280
tgcgttctca aagctgaacg cgaactgcct gcttgaatca agcacagtca ctgtgaaagt 1664340
gacaggtgcg ggacactgtg cggaatcttg aaagattcct gatttctgaa actctacatt 1664400
gacggtttca gacggcagat ttaaatcttc tgccggattg gactcgggca gcctgtcgca 1664460
agcgagaatg tcggggaaga atttgcacaa aaggccgcca tctttgccgt cctttccgtc 1664520
tttgccgtcc ctgccgtttg tgcgtcccgg aacggcgggg gaatcgggtc ttgtgccggg 1664580
ctgtccgtcc gtatcgggat ttgcatcggg attcaaatcg gggtcgggtt cgggattggg 1664640
gctcgtgccg gggttctcat tggggttcgg gttgtttgcg gggttttcgg cgggcgatac 1664700
ttcgggcagc ggctgtgcgt tcggtgcttc cgcgcttccg ggggtcaagt cgggacgcgg 1664760
gattacttga acatccaccg tggtgttgcc ttgcgaatcc ctgccgaatg ttgcgacaac 1664820
ctgaacggga ttcccgttcc tgtccgtgac gggacccata ttcacttttg ttccgggtgc 1664880
gacttctact ttttcggaat aaccgggata accggttgcc tttatgtatt tgtcgggatt 1664940
ggcatcgact ttcaacgata aaatctcttc cagcttttttg gcatccattt cttctttgta 1665000
ttttgaattg cgaataaggg aaaaatcagc ccccatttctg aaatcatcac ctttattgac 1665060
caaacaatct ccgccattcc aattaaatgt gcaacgattt aaaacaaaat tattccaatc 1665120
caaagaactt aatttattca gttcttcttt atgccaattc caaaacggac gtgccagcct 1665180
atacatttgg ctttccatca attctttgac ttcggggaat ctgctgtcat cggacataag 1665240
gcgcataatc gaactgtcaa cgccgtagca gccataggtt ctattaatac gtcttttgtc 1665300
ttcgtaccaa aggcaattac tatattcgta gccttttaca aatttgtcgg tttcggggtc 1665360
gtattggtag ccttgtgcct gtatgtcttc tttgaaagtt tcgtatacgt catgggctaa 1665420
aagggctgtt ccgacataag gaactgccct tgtgcttaat ttcgcgccta agcgggcaag 1665480
tttgccgact cctgacaaga cggcggcgcg ggaaactgat gcggtaaatt taacggggac 1665540
tttttcaaga gagcgtgcac ctgttggcac gtgttctatt attgaaggtt cgataattcg 1665600
acctgaaaaa cgctgaccat caacacgaaa atcggtaact acagattttt tatgatcaat 1665660
atccaatcta acatctgaat ttccaatagg atacttaaaa cgttcagcat aagaattaac 1665720

cgaaaacatc cccaacatta ggattaaaat caaacgtttt aatttcaatt ccacgactat 1665780
aatcatcctg taattctaaa atttttatat acgcaacaga ctcatcagaa aaataaattt 1665840
tccaaatatt attagaaatc cttctattta aaaaacattg gatttcttca tgaattataa 1665900
atttattaac ttttgaataa tccaaaagct cactagcgaa agtatatgcc attgttttac 1665960
cataatactt gcttgacggc tgatatttat aaagtgccaa ctgcgcctgc gtgataaacg 1666020
gtttgttcat tgttctgcct ttcaaaggtt gttttgaaag cctgatttta aaacacgtca 1666080
tttaaatatc aaagcgacag acaaagccaa gaagaaaccg agaagaaacc aaaaatcaat 1666140
aaccatcaat caatcccaac cttgcccatg tcttttaaaa aattaatcaa aagcctgaag 1666200
ccgtaaatga ctacgaacag aattaaaacc gtcgaaccga cataagaacc ctgtttgatc 1666260
tgctcaaaat tggaacattt cggataggac aacattaccg gctttccgtt caagacccat 1666320
ttttcgccca ccctttccgg cctgatgatt tttccgtcct gggtaacagt aggaggaagg 1666380
gacgacaata aatagtcgtc tgcatgcaat cttgtatcaa aacaatttat gccgacacga 1666440
tagcccattt atacgcccct ttttcttcac tctgtttatt tgacagattt aatcatgctc 1666500
caagccattt tgaagccttg gattgcaaga atcacggtaa tggccgccat acccacggcg 1666560
gaaaccattg acacgaaacc catgattaca ttcgctactt gcgtaccaat cgcggatgga 1666620
tcaaaggtat ctgccataac aatggccggt gtgaagatac cggctgccaa ggctgctttt 1666680
acagcgtatt ttttaacgat gttcatcgtt tttttccttt tttgatattt aaaataagac 1666740
gacttcttga cttgcttcat ccggacgaag tcttttccga atctcgtttt tagccgataa 1666800
aatagaggat tgcgaaaaga aaaagaaaca tacagaccac ccagccgata attagtgttg 1666860
caaggttcat tttcatgata tttttccttt gttgcgggct ttgtgaaagg ttgacagacc 1666920
gcccgccgag cctgttttttc ttttattccg attttacgaa gaactgaaat atctggaatc 1666980
ctccgcctat ttcatttatg cctgaattca acgcatcttc gtagctttca aattgacctg 1667040
ctgatttaat attttgagta aaccccacat caccgaaagg atcgggataa ataaagtcat 1667100
gcgtttccaa gtcttgaact atgaaacgtt cttcaaattt cataaatcaa cctttcggct 1667160
tttctgccac ctgaaaatca attaatgaag gaaccatgcc cttacctgtc gaagtcattt 1667220
caaccgttac cataacttcg cacgggtatt tgagattctc taattttgag aaattcttac 1667280
tgtccccgaa cttcatttgt gctgccgtga atccaacagc atttcccgac tgtgccggca 1667340
aaggtgttgc aaccaatacg gaacaagtgt cgatattaga gccatcaatt tcgcctttga 1667400
attttttagc tcctaaaaaa gttgcgggat aagttacagt ttgagtttga ttaaacatat 1667460
taatttttcc tttttaggtt aattttgatt tgcatgaaga tcatacattc tgtcgagata 1667520
aagctgatat tgcctctcac tttccacatc gtgatgtgga tcgaaaagcc ttttatccgg 1667580
atcgaattta tctgattgct taaatttgat aattccgagt tcttccaatt caacctctaa 1667640
atctacatcc ggttgttcgt ggataaagcc gaatttcaaa gattccttca atccggccaa 1667700
cgaatatttt tcaggttcta gccctttggg ataccccaaa tctgccttca gatatctgac 1667760
aatttcatca ctatcaaaac ccatatcaaa catgaaatta atcagtttgc cgaccgcgtt 1667820
ttttgcgtat ctcaatttat gctgaaaagt taaattagcc acttttttac ggtaatcgaa 1667880
cctttccgga ttcggcatat ttttaaattt ctgacaaatc gggaaagcgc ctgaaaagta 1667940
agaaccttga tttatcagaa tatccaaagg tatttccata tctccatgat taaactgaat 1668000
ttcgaacctt acccacttgc tttctttatc gcctagctgc ctgcctttct cataaacacg 1668060
cacaaaacga gaatttttct tgcgacctac ataaaatgtc ttgccgctcc cgtcctctct 1668120
ccgccaagcc gttccaacca tttcagattt cggcctcatg ttactgttat cgaaaaaacc 1668180
gttatcgtga tccaaaagtg cctgttccgg cgtgtactcc ccatcaaaaa aatcaagtgc 1668240
caaatctacc cgcgttatcc tcggcctcaa tgaatcttcc aaaaactgct taagcctcaa 1668300
ttcccaacct ggatttgcaa tgttgcaacc tacacctttc aattcgatta aaaccgtatt 1668360
tcgctgacct ccgtaatgga cttcgccgta gtcaacttct tccgatccca acctaaacat 1668420
cgaatcgtaa aatttattgc ccttcgattt gcatctgctc gtgatgccaa accctaatat 1668480
ttcctccaat tttttgctta aaacaaacat atattcggca tcggaaacta aggggcatcc 1668540
ggaaactttc agcaaggaat cttcgtgcag tgtgaatgac aaccaatcta taaaaacgcc 1668600
gtcctgcctg cccctacgtt gcggaatttc taataacttc ccattgccgt tagatatgaa 1668660
atgggaaaaa tattctgctt cactcatttt gttcagtacc tttagggatt tgttttattt 1668720
cgctcccccc ctgttagtca gggggggct ttcagccgtt tcccgtctgc cgcgctaaag 1668780
cgcgtccaac ggtcaacgac cgaaagccca atcctgacaa actgttaaag atcaagaaga 1668840
aagaccacaa ccgtctgttg tgataattac cggaaaattc gagccaaccg aatctatata 1668900
atcgaacgcc tgataaagct ttgaaaaatt ttcttgttca gcgagtttat gcggttcacc 1668960
atgcctgaac tgatagaaac ataaaacgca ataatctgat tttttaaata ttctccaata 1669020
ggaacaagaa aatattacat ttgctactga cataaaaaag cccctttcac ttggctgtca 1669080
aaggggaatg ttaagaaaag taatgcgccc ctttgataga gcgcatcata taaggcggga 1669140
atccagtccg ttcagtttcg gtcgtttccg ataaattcct gctgcttttc atttctagat 1669200
tcccactttc gtgggaatga cggcggaagg gttttggttt tttccgataa attcttgagg 1669260
cattgaaatt ccagattccc gcctgcgcgg gaatgatgaa ttcatccgca cggaaacctg 1669320
caccacgtca ttcccacgaa cctacatccc gtcattccca cgaaagtggg aatctagaat 1669380

ctcaaacttt cagataatct ttgaatattg ctgttgttct aaagtctaga ttcccgcctg 1669440
cgcgggaatg acgaatccat ccgcacggaa acctgcacca cgtcattcct acgaacctac 1669500
atcccgtcat tcccacgaaa gcgggaatcc agtccgttcg gtttcggtcg tttccgataa 1669560
attcctgctg cttttcattt ctagattccc actttcgtgg gaatgacggc ggaagggttt 1669620
tggtttttc cgataaattc ttgagacatt gaaattctag attcccgcct gagcgggaat 1669680
gacgattcat aagtttcccg aaattccaac ataaccgaaa cctgacagta accgtagcaa 1669740
ctgaaccgtc attcccacga aagtgggaat ctagaatctc agactttcag ataatctttg 1669800
aatattgctg ttgttctaag gtctagattc ccgcctgcgc gggaatgacg gctgcagatg 1669860
cccgacggtc tttatagcgg attaacaaaa atcaggacaa gacgacgaag ccgcaggcag 1669920
tacaaatagt acggaaccga ttcacttggt gcttcagcac cttagagaat cgttctcttt 1669980
gagctaaggc gaggcaacgc cgtactggtt tttgttaatc cgctataaca gcaaccttgt 1670040
cgccgtcatt cccgcaaaag cgggaatcca gtccgttcag tttcggtcat ttccgataaa 1670100
ttcctgttgc ttttcatttc tagattccca ctttcgtggg aatgacggcg gaagggtttt 1670160
ggttttttcc gataaattct tgaggcattg aaattctaga ttcccgcctg agcgggaatc 1670220
cagtccgttc agttccggtc atttccgata aattcctgct gcttttcatt tctagattcc 1670280
cactttcgtg ggaatgacgg cggaagggtt ttggtttttt ccgataaatt cttgaggcat 1670340
tgaaattcca gattcccgcc tgcgcgggaa tgacggctgc agatgcccga cggtccttat 1670400
agtggattaa caaaaatcag gacaaggcgg cgaagccgca gacagtacag atagtacgga 1670460
accgattcac ttggtgcttc agcaccttag agaatcgttc tcttttttgt tcatccgcta 1670520
tattgtgttg aaacatcgcc acaaacctga tatagtccgc tcctgcaaca tcattgaaaa 1670580
tctttctttt taatcagtta aaaccgaata cggagtcgaa aatgaatcca gcccccaaaa 1670640
aaccttctct tctcttctct tctcttctct tctcttctct tctcttctct tctcttctct 1670700
tccgcagcgc aggcggcaag tgaagacggc agccgcagcc cgtattatgt gcaggcggat 1670760
ttagcttatg ccgccgaacg tattacccac gattatccga aagcaaccgg tacagacaaa 1670820
gacaaaataa gcacagtaag cgattatttc agaaacatcc gtgcgcattc catccacccc 1670880
cgagtgtcag tcggctacga tttcggcggc tggaggatag cggcagatta tgccagttac 1670940
agaaaatgga gcaacaataa atattccgtc aacacaaaag tgttgaaaga aaaccagggc 1671000
aacaggataa aactgaagac ggaaaatcag ggaaacggta cgttccacgc ctcttcttct 1671060
ctcggcttat ccgccattta cgatttcaaa ctcaacgata aattcgataa attcaaaccc 1671120
tatatcggtg cgcgcgtcgc ctacggacac gttaaacatc aggttcattc ggtggaaacc 1671180
aaaaccacga tttataccac tgcaccaacg ggagacgcta cagtgggagg cactatccca 1671240
gagagaccga gtagcaaacc tgcctatcac gaaagcaaca gcatcagcag cttggggctt 1671300
ggtgtcatcg ctggtgtcgg tttcgacatc acgcccaagc tgaccttgga caccggatac 1671360
cgctaccaca actggggacg cttggaaaac acccgcttca aaacccacga agtctcattg 1671420
ggcatgcgct accacttctg attccccgat accgatgccg tctgaacctt cagacggcat 1671480
gagacctttg cctgcgtact tggtacgctg gtcgcctccg aacatggcgc gacacccgac 1671540
atttccgccg aacgcatcgg gcgtttcatg aatccggttt aaaacgcatg gaaaaatgcc 1671600
gtctgaaagc ctttcagacg gcattgtgct tgagattccg tttaccaatg gctgacaaac 1671660
gcttccaaat cggtattctt gggcttatgc acttcctctg tcggcgtgcc gaccatcatc 1671720
agcccgatga ttttatcctt atccgcacaa ccgaaagcct cccgcaacag gggctattg 1671780
acccacatcc ccgtaatcca gacattgtcg aatccctgag ccgttgccgc cagttgcagc 1671840
gcatacgccg cacaacccgc cgtcagcatc tgctcccatt ccggtttcgg cttaggcaca 1671900
tcgcggttcg gcgcaaacgt taccccgata accatcggcg ccatattgcc cactttttcc 1671960
gccttttca tcgcatcgtc gccgaaattc aattcggcaa ccgtttgctt caacacatcg 1672020
cgaaaacgtt gcaatcctac ctcgccttga atcacggtaa aacggaaggg gcgcatattg 1672080
ccgtgatcgg gaacttgggt tgccgcctga aatatttgtt ccaactccgc cgcatcgggg 1672140
gcggggtgct tcagcttttt ggaagatcgg cggttcgtca ataattttaa agcatccata 1672200
tcgttatact ccggtcatcg gtcgggtgtt cggacacata tgccgtccga aggcttcaga 1672260
cggcatatcc ggcatcagcg cggacggcgg caggctgcca atatatccat ttccttccga 1672320
taggtttggc tattggaaat gtccatcagc cccaataccg tgctgaaata gtggtcgtgc 1672380
gaatattcgt tttccgccgc tttttgtttg aggcattgga aatctatgcc cccgtgttgg 1672440
cggaaggctt tggaaaacca cataaccatc gggatatgcg tctgcccgga aggcgcgatg 1672500
gcgtaaggcg cggcgtgcag gtacatcccg ttttcgccca aactttcgcc gtggtcggaa 1672560
acataatgca ccacgctttc caaatcgtcg cggttttcaa gtttgcggat aaccttgtcg 1672620
ataaactggt ccacatacaa aaccgtattg tcgtaagtgt tgaccagcgt ggcgcgggtg 1672680
catttgttga tttcgttggt gtcgcaggtc ggcgtgaatt tgcgttcggc ttcggtatag 1672740
cgttcgtaat acgtcggccc gtggctgccg atggtatgca ggattaaaac cgcgtcttta 1672800
tcgtttttgt tgaggacttc gtcgaactta gtcagcagga tattgtcgag gcactcgccg 1672860
ttgcggcagt attcgggcag gttgagcgag gtaacgtcgg tattcggcac tttgccgcac 1672920
acgcccttgc agccggaatc gttttccaac caagtaactt ccacgccggc gcgctgcacg 1672980
atgtccagca ggttgtcttg gtgttcggct ttgatttcgt cataatccgt gcggtcgaag 1673040

gttgagaaca tacacggcag ggagtgcgcg gtcgatgtgc cgcagcttct gacctgcggg 1673100
aaattgacaa tttcatcgcc gcgcgcggca agcagcggcg tagtttggcg gctgtaaccg 1673160
ttcaaacccc agttggcggc acgcgtggtc tcgcccacga ccagcaccac gaaacggcgc 1673220
aggctgccgg ccgggcggtt ttgcacgacc gccatatcca attgcgtata aggaatattg 1673280
gaacgcttcc aatctttgta tttcgacacg cccgcgccga tgaaattaga cggcacaatc 1673340
agatgggtta ctgatttatt gttgcggaaa aacgaggcgt aatcctgata ttgcaacatt 1673400
gcgatgccca acgcgcacaa aaaggaaacg gcggcaagca caaggcgcgt caaaagctcc 1673460
ttataccaaa cgcggtattt aaccttgacg gcgatatacg ccagcgcggg caatacgccc 1673520
aaacatacaa tccacagcac atagcccggc gtaatcaggc gcgcgctttc ggcagccgta 1673580
gtttgcaaga cattattcaa catcgacttg ttgaaataga tattgaaaaa tatttcttgg 1673640
taagacaccg ccgcactgat aaccaatatc aacggaatca ataccttatg cacgaaaggc 1673700
agggcaatga cgtgaaaaac gaaattactt aaaaaaaaca gcaccaccgg catcgtatag 1673760
aggaagatat ccgcccccggt gccgttaaaa ggatgaagct cgacaacttt ggcaaaaaag 1673820
gcgtaattca ataccagcga ggaatacagg gaaaggaagg caatcagcgc ggaagagccg 1673880
agcttcggcc tcaggttcgg ttttatcatt tggaatgtgt cggataaggg ttggaaaagg 1673940
catccggcat ttggaatccg gattattgaa aaagattctt aattataagg caacggagca 1674000
aagcagggca agaaaacggc ggctgtgcgg ggggttccgc ccgccattca aacgtccggc 1674060
agacataaaa acatcgtaag caagattcga accggtctgc aaccgcccct gccagaaaaa 1674120
cgggcaaagc atttcatatt ggaaaaaccc agccgcgccg ccgacgggac agtccggcac 1674180
aaacagcatc acgctcggat tgaaaaggac ggatagccgc cggcagcaca atcttaccac 1674240
ctccaaaacg ccgccggaga acgccgaaac agccgatgcc gtctgaagcc gcttcagaca 1674300
acatcgggac atcaaccgta acgccgttgg aaatcgcgca taaaatctgc caaagcccgc 1674360
acgccttcaa gcggcatcgc attataaatg ctggcacgca taccgccgac ggttttatag 1674420
cccttaagca ggcacaagcc ctgcaattcg gcttccagca caaaacggcg gtcaagctcc 1674480
tcatcccccg tttggaacac gacattcatt ttagaacgcg cattcggacg gatacggttg 1674540
atataaaaac catcgctgcc gtctatcgtc tcatacaagg tttgcgcctt cagccgattg 1674600
accgcttcaa tttttttcac accgccctgc gcctgtagcc agcggaacac cagccccgac 1674660
atataaatcg cgtaagttga cggcgtgttg tacataccgt cgcggttgat gtgcgaacgg 1674720
tagttgaaca catcgggaat atcgttcgga caacgctcga gcaaatcctc acgcacaatc 1674780
accaccgtaa ctcctgccgg cccgatgttt ttctgtgcgc ctgcgtaaat cagtccgtag 1674840
tcggcaacat caaactcgcg cgacaaaatc tcgctggaca tatcgcacac cagcggcggc 1674900
atgccttctg aaaggcacgg cacttcacgg tattgcagcc cgttgaccgt ttcattgacg 1674960
gcaaaatgga caaacgccga atcgggtgca acatcccacg tttccacagg cggcaggtcg 1675020
agatagtcga actgctcgcc gccatgcgcc gccaaacgga tttccgtatc ggtcaaacgg 1675080
ctcatctgtt cataagcgat acggctccag ttgcccgtta ccaccgcgtc ggcagtgcgg 1675140
aaaccgtgtg ccagattcat ggctgccata ttaaattggg ttgttgctcc gccctgcaga 1675200
aacaatatct tatagttgtc aggcactttc aaaagctgcc tcaaatcctg ttccgcatga 1675260
tgcaggatgc tcaaaaacat ttccgaacgg tggctcattg ccatcacagg aaaacccgta 1675320
ccgttgtagt ccaacatttc ctgccgcgcc gtttccaaca cggcttcggg caatacggca 1675380
gggccggcgg aaaaattgta aatcggataa agagacatga tgcagccttg attctgaaca 1675440
ataacccgcc cgattttagg cttgcagcag gctttgtgca aggatggaaa atacctgtcc 1675500
tccgcccgat tccatgccgc ccgaacacgg aaaataatat caatatattg atttacaaac 1675560
ataaaaatca tgcacgcgac aaatagatac atttgttttg tcaacaatat tcacgatttc 1675620
ccattacaaa cctcccttac acccgctttt ttccgtccca aaaacacaaa ataaatcaac 1675680
actttcattt ctccgcaaaa gcggttataa tcacgccgat ttttcaactt tgacgaaaaa 1675740
tggccacggg gccatttttt tattatgtac atagggagca gcatggatat ccaaaccatc 1675800
ctcgaaaaaa ccctgcccgg cctgggctac gaactggtcg atttcgaact gaccgcgcaa 1675860
ggaacattgc gcgtgttcat cgacaaagaa agcggcatta ccgtcgaaga ctgcgcaacc 1675920
gtcagcaacc acttgagccg cgtcttcatg gttgaagaca tcgactacaa aaacctggaa 1675980
atttccagcc ccggactcga ccgcccettg aaaaaagccg ccgacttcgt gcgctttgcc 1676040
ggtcagaatg ccaaaatcaa aacccgcctg ccgatagacg gtcagaaaaa ctttatcggt 1676100
aaaatcgaag gctgcgaaaa cgataccgtt accgtatcct tcgacggcaa aaccgtacaa 1676160
atcgaattgg gcaacatcga caaagcccgt ctgcgccccg aattcaaatt ctaaaacaca 1676220
acaatattgg agatgttcaa aatgagtcgt gaaatgttac agctggcaga agcactggca 1676280
agcgaaaaaa acgttgatgc ggaagtcgtc ttccaagcac tggaattcgc cctgtctacc 1676340
gccgccaaga aaaaggcaga ccgcgaacac atggacgtgc gcgtccaaat caaccgcgac 1676400
accggcgaat accaaacctt ccgccgctgg ctgattgtcg ccgatgaaga ctatacctat 1676460
cccgatgtcg aaaaaaccat cgaggaaatc caagaggaaa ttcccggcac taccatccaa 1676520
atcggcgaat actacgaaga gcagctgccc aacgaaggct tcggccgcca agccgcgcaa 1676580
accgccaaac aaatcatcct gcaacgcatc cgcgatgccg agcgcgagca gaatctgaac 1676640
gagtttctcg ccgtcaaaga agacatcgtg tccggcacgg tcaaacgcgt cgaacgccac 1676700

ggcatcatcg tcgaagtcgt tgccggcaaa ctggacgcgc tgattccgcg cgaccaaatg 1676760
attccgcgcg aaaacttccg cagcggcgac cgcatccgcg ccctcttcct gcgcgtcgaa 1676820
gaaatcggca acaccggccg caaacaagtc attctgagcc gtacttccgg cgatttcctc 1676880
gtcaaactgt acgccaatga agtacctgaa attgcagacg gcatgcttga aatccgcgct 1676940
gtcgcccgcg acccgggaca acgtgccaaa gtcgccgtca aagccaacga ccagcgcatc 1677000
gatccgcaag gcacctgtat cggcgttcgc ggttcgcgtg tcaatgccgt cagcaacgaa 1677060
ttgtccggcg agcgcatcga tgtcgtcctc tggtcgcccg aacccgcgca attcgtgatg 1677120
agcgcgctct cacccgccga agtcagccgc atcgtcatcg acgaagacaa acacgccgtc 1677180
gatgtcatcg ttgccgaaga ccagctcgcg ctcgccatcg ggcgcggcgg tcaaaacgtg 1677240
cgccttgctt ccgacctgac cggctggcag ctcaacatca tgacttccgc cgaggcagac 1677300
gaacgcaatg cggcagaaga tgccgccatc cgccgcctgt ttatggatca cttgaacgtg 1677360
gacgaagaaa ccgccgacgt actggttcag gaaggttttg caaccttgga agaagtcgcc 1677420
tatgttcctg ccgccgaact gcttgccatt gaaggatttg acgaagaaat cgtcgatatg 1677480
ctccgcaacc gcgcccgcga tgccatcctg accatggcga ttgccgccga agaaaaactg 1677540
ggcgaagtgt ccgacgatat gcgcaacctc gaaggcatag atgccgatat gctccgcagc 1677600
cttgccgaag caggcattac cacccgcgac gacttggcag agcttgctgt ggacgaactg 1677660
attgaaatca ccggtgtaaa cgaagaaacc gcaaaagccg tcatcctgac cgcacgcgaa 1677720
cactggttta ccgaagacaa ataaaggggg tacagatgag taacacaacc gtagaacaat 1677780
ttgccgccga gctgaaacgc cccgtcgaag acctgttgaa acagttgaaa gaagccggcg 1677840
tcagcaaaaa cagcggcagc gattccctga cgctggacga caaacagctt ctgaacgcct 1677900
acctgaccaa gaaaaacggc agcaacagca gcaccatcag catccgccgc accaaaaccg 1677960
aagtcagcac cgttgacggc gtaaaagtcg aaacacgcaa acgcggacgc actgtcaaga 1678020
ttccttctgc cgaagaattg gcagcacagg taaaagccgc ccaaacccaa gccgcacctg 1678080
tccggccgga gcagacggca gaagacgcgg caaaagcccg agccgaagct gccgcacgcg 1678140
cagaagcccg tgccaaggca gaagcggaag cggcaaaact gaaagcggca aaagcaggca 1678200
acaaagccaa acctgccgcg cagaaaccca ccgaagcaaa agccgaaacc gcacccgttg 1678260
cggcggaaac caaacccgcc gaagaaagca aagcggaaaa agcccaagcc gacaaaatgc 1678320
cgtctgaaaa acccgccgag cccaaagaaa aagccgccaa gccgaaacac gagcgaaacg 1678380
gcaaaggcaa agatgccaaa aaaccggcga aacctgccgc acctgccgtg ccgcaacccg 1678440
tggtcagcgc ggaagaacag gcgcaacgcg acgaagaagc acgccgtgcc gccgcacttc 1678500
gcgcccacca ggaagccctg ttgaaagaga aacaggaacg ccaggcacgc cgcgaagcca 1678560
tgaaacaaca ggcagaacaa caggcaaaag ccgcacagga agccaaaacc ggcagacagc 1678620
gtcccgccaa acctgccgaa aaaccgcagg cagccgcgcc agccgtcgaa aataaacctg 1678680
tcaatccggc aaaagcgaaa aaagaagacc gccgcaaccg cgatgacgaa ggtcaaggcc 1678740
gaaacgccaa aggcaaaggc ggaaaaggcg gacgcgaccg caacaatgca cgcaatggcg 1678800
acgacgagcg cgtacgcggc ggcaaaaaag gcaaaaaact caaactcgag ccgaaccaac 1678860
acgccttcca agcaccgacc gaacccgtcg ttcatgaagt tttggttccc gaaaccatta 1678920
ccgttgccga tttggcgcac aaaatggcgg tcaaaggcgt ggaagtggtc aaagccctga 1678980
tgaagatggg catgatggtt accatcaacc aatccatcga ccaagacacc gccctgattg 1679040
tggtggaaga actcggccac atcggcaaac ctgccgcagc cgacgaccct gaagcattct 1679100
tggacgaggg cgcggaagca gtggaagccg aagcattgcc gcgtccgccc gtcgttaccg 1679160
tgatgggcca cgtcgaccac ggcaaaacct cgctgctgga ctacatccgc cgtaccaaag 1679220
tggtacaggg cgaagcgggc ggcattacgc agcacatcgg cgcgtaccac gttgaaaccc 1679280
ctcgcggcgt gattaccttc ttggacaccc cgggccacga agcctttacc gctatgcgcg 1679340
cacgcggtgc gaaagcaacc gacatcgtga ttctcgtggt cgccgccgac gacggcgtga 1679400
tgccgcaaac catcgaagcg attgcccacg ccaaagctgc gggtgtaccg atggtggttg 1679460
ccgtcaacaa aatcgataaa gaagccgcca acccagagcg tatccgccaa gagctgaccg 1679520
cacacgaagt tgtgcctgac gaatggggcg gcgatgtaca gtttatcgac gtttccgcta 1679580
aaaaaggcct gaacatcgat gcattgctcg aagccgtctt gctcgaagct gaagttttgg 1679640
aactgaccgc acctgtcgat gcgcccgcca aaggcatcat cgtcgaggcg cgcttggaca 1679700
aaggccgcgg cgcggttgcc acattgctgg ttcaaagcgg cacgctgaaa aaaggcgata 1679760
tgctgctggc cggtacggca ttcggcaaaa tccgcgcgat ggtcgatgaa aacggcaaat 1679820
ccattaccga agccggtccg tccatccccg tcgaaatcct cggcttgtcc gacgtaccga 1679880
atgcgggtga agacgcgatg gtattggcgg acgagaaaaa agcgcgcgaa atcgccctct 1679940
tccgccaagg caaataccgc gacgtgcgcc ttgccaaaca gcaggcggcg aagctggaaa 1680000
atatgttcaa caatatgggc gaaacccagg cccaatcttt gtcggtcatc atcaaggcag 1680060
acgtgcaggg ctcttacgag gctttggcgg gcagcctgaa aaaactgtcc acagacgaag 1680120
tgaaagtgaa cgtgttgcac agcggcgtgg gcggcattac cgaatcggat gtcaaccttg 1680180
ccatcgcttc gggcgcattc attatcggct ttaacgtgcg tgcagatgcc tcttcgcgca 1680240
aacttgccga aaatgaaaac gtggaaatcc gctactacaa catcatctac gatgccatca 1680300
acgacgtgaa ggcggcgatg agcggtatgc tttccccgga agagaaagaa caggttaccg 1680360

gtacggtcga aatccgtcag gtcatctccg tttccaaagt cggcaacatt gcaggctgta 1680420
tggttaccga cggcgtggtc aaacgcgatt cccatgtccg cctcatccgc aacaacgtgg 1680480
ttatccacac gggcgaactg gcttcgttga aacgctataa agacgatgta aaagaagtcc 1680540
gcatgggctt cgagtgcggt ctgatgctca aaggctacaa cgaaatcatg gaaggcgacc 1680600
aactggaatg cttcgacatc gtcgaagttg cccgcagcct gtaattcctt tgcaaataaa 1680660
atgccgtctg aagcgttcag acggcatacg aaacgggttc tgtatcatac agaacccgtt 1680720
ttttgtcgca aatcggcttc agacagccct cttgccttat cccgatttga atctgacttg 1680780
ccatacaaac aggcttcaga cggcattatt tgcccgctaa acgtatccca agcttctccg 1680840
catattccct gcgttcggcg cggctggttt ccgggcggtg cgtattgagc gacgaccatt 1680900
tccaatgact gcgggctttg ttgagttcgg gcgggagtct ggcggcatcc cacgggactt 1680960
tgcggctgtg cagctcgata tccgactgtg ccgcgtgtcc gcgcgtttgc aggacgtgga 1681020
gcaaatcgag ggcgcgggcg gcgagcaggg tcagggtttc agggtcggtg tgcagggttt 1681080
ggcggccagc gagtttgtcg gaaatggtgc gggtattggg caggatgccg cccaaaaagc 1681140
cgccgattgc cgtacccaat ccgagcgagc cgccgagtgt ggcgatgtcc agccccaagc 1681200
cgatgagcgc gccggttgcc gcgcccgtgc cggtgcggat gccgtattgt ttgagcaatt 1681260
cgctgtcgaa cgggtcttgg cggaaggctt gcggcatcca gtcgccgccg tcgatttcgc 1681320
tgtggtagaa acggtagagg gcaaacagcc gctgctgcat ctgccgttcg agttggcgta 1681380
tttccgcctg catggtttgc agcacggtgg cggtatcctc gttttcgtcc acttcctgcc 1681440
tgaaggcggc ggcatcaatt aaaaagtcgg cgatttcgcg gcgcgcttcg ccgtccagcc 1681500
gctgccattc gcgccggcgc atggctgtca ggcggtcaag tgtgctgcgt tcgggcaaca 1681560
tggtggcgag gttttcccac aggcgcagtt cgccttcaaa atcaaaggcg acggtgtcga 1681620
accctgcgaa aacgtgcagg tttctcctcg ccagcatggt tgtccacgat tcgggaagct 1681680
gtccgccggt aaagttgaac acgggcataa ccggtttggc acaccatgaa aggatggtca 1681740
actcgtccct gtatttgtcg aggacgggtt cgcgcgcgtc gatgacgtac attgccatat 1681800
cgctttgcaa gacttgccgt aagactttgg cttcctgatt gaaatcatgg tgcgcaccgt 1681860
ggctgccgag aaactgttgc agccgttcga tgccgtctga acgattgtcc gtatggtttt 1681920
ccagccattc cagcacgccg cccgcgtctt cgagtccggg cgtgtcgtac aggaaaacca 1681980
gcgtgtctgc gccgtcgctg atggcggctt cttcgacatg acgcgtggtc gatggggcgt 1682040
ttttgacttc gccgaaaccg ctgtcgcgca aaagggtacg caggagcgag gttttgccgg 1682100
tgttggtgtg tccgacgacg gcgagggaaa ggggttgttt gttcatgatg tttttgaaga 1682160
atggattttc agacggtctt ttttcagaat ggcggcttaa cagaacattt caagtgagtt 1682220
tattggtctt tcaaacgccc ttcctgcgcc gccctgtcag gctcaagcca cgccgcgccg 1682280
cattcggcca gcgcgttacg ccaatgttcc agcttttccg aaaggtcgtc tgaaagcccc 1682340
tgttccgcca aaagctgcac caccgcgccg ccctgcgccg cttccgagag tcggacaatc 1682400
tgccgcaaca cgccgcggtc cggcacagtt tgggcgcgca cgccgataag cagttgcgcc 1682460
ggtttctgct tcagctctgt ctccagcgcg gcaacctgtt cccgattggt ggcaacgccc 1682520
ttatccagcc attcctgcgc cagcctgccc tcgaaccatt cgccgtcctg ccactcggtc 1682580
tccagcatga ccgcccattt cggcgcatcg ttcaagatga ttttcggtga aacggcggac 1682640
acggtttccc gacgcgtatc cgcatcggtg attttgttct gccagcggcg gatgaccgcc 1682700
tgataatagg gcttttccaa atccaatccg ttttcgcttg ttttcaaaag gattttacac 1682760
actacccaag ccagcaggcg cggcaggatg ccgtagcagg cgatactgcc gaccagcagc 1682820
cccgaccaag cccgcgcatc ggcaatattg ccgttcagac ggccttcgat gaccgcccgc 1682880
gcatcgggga cagggaaacc gagtttcgac ggcagccatg ccaacatttc caccgcgcgt 1682940
accgaagcgg cattgctcaa cagcgtgctt tcccagttga acgtatattg ccgcaccaaa 1683000
agcagcaaca ataccgacac cagcattccg agcagcgtgc agagccacag gctgtgcgac 1683060
gttgcgccta ttttccaacg taccgaaggt tgccgccact cgtccgcata cagccgcaac 1683120
accgcctgat ttacagggtc tttgccccga aaccacgtcg ccggactgct gaaaaaacgc 1683180
cccactttca cacgcaggaa caacattgcc aaccatactg ccagcatcag cgtattcatg 1683240
cccaacacgc ccgccaaaac caaaaagaaa ttcagaccct gattgtccat tagaagataa 1683300
gtgactgaaa aaccggtaaa aaatgcaaac gtcgccgcca ccacccacaa ccagaacgac 1683360
cccgcacgca cacgttccaa cgtctcccgc agcatacggt tcctgtcaat catctccgcc 1683420
cgacggatga ttttttcctc cgtactgccg tccacgcggc gcaaagcctc cgtcgcctgt 1683480
acgggatcgc cgctgaaaat aaaaccgcct tcgtccaaaa tacggaccag ctcaaccagt 1683540
tttcgggatg gattcaacat aaaatgccgt ctgaaaataa aaaacagatt ttaacacacg 1683600
cattttcaag aatattcaca gtgtaggcaa agagtaaatc tcacacagaa gcaaaagtat 1683660
cggcgtaaac tgactgcctc tactttcccg aaagattgtg cgatgtatac aggcgaacgc 1683720
ttcaatactt acagccattt gagcggtttg attctggcgg cggcaggttt ggcgctgatg 1683780
ctgctgaaaa ccataggaca cggggacggc taccgtatct tcagcgtatc ggtttacggc 1683840
atcagccttc ttctgctcta tttgagttcc tcgctgtacc acggaattgc agccggaaaa 1683900
ctgaaaagca ttttgaaaaa aaccgaccac tgcatgattt atgtgctgat tgccggaagc 1683960
tacacaccgt ttgcactggt ttctttgaga aacgggccgg gctggacggt attttcactg 1684020

tcctggctgc tggcggctgc aggaatcgca caagaactca ccatcggacg gaaaagcgaa 1684080
aaacgtctgc tgtctattgt gatttatgtc gtcatgggtt ggatggtctt ggcggtaatg 1684140
aaatccctga cagcctcact cccgtcggca ggactggctt ggctggcggc aggcggtatg 1684200
ctgtacagtg tcggcattta ctggtttgta aacgatgaaa aaatccgaca cgggcacgga 1684260
atctggcatc tgttcgtatt gggcggcagc atcacccaat ttgtcagcgt gtacggttac 1684320
gtaatctgaa tgccgtctga aaagcaaaac ctcccgttcc tgaagattgg gaggttttct 1684380
gtttgccgga catcagccct tgtcgtggaa ctcgtggaat tcatactgat aggacaaatc 1684440
ccgacccgct tttttctgtg ccaaataatc atcataaatg gcgcggattt ccttacgcaa 1684500
caaaaacagg gctatcaggt tggggataac cataaaaccg ttgaacatat ccgacagact 1684560
ccaaaccaaa tcgactttgc cgagcgtacc caaaacaatg gcaagcagaa ccaatgcgcg 1684620
atagatgccc aagtgtcttc ccctgaaaag aaaacggata ttggactcgc cgaaataata 1684680
ccaaccgatg atggtggtga aggcaaagaa ggtcagacac acggcaagca attgcgaacc 1684740
gaagcccgga aatgccttgt taaaggcaaa ttgagtaacc gccgcgccct gttcgcccga 1684800
aaggttggca tcggtcagca ggataatcaa tgccgtagcc gtacatacca aaatcgtatc 1684860
gataaacaca ccgacaaatg ccgccatacc ttgctgcaca gggtgcttca catccgcagt 1684920
cgcgtgggcg tgcggagtcg aacccatacc tgcttcgttg gaaaacagac cgcgcgccac 1684980
gccgaaacgt atcgcttcgc gcataccgat acccgcagca ccgcccaaaa cggcttcggg 1685040
attgaaggcg gcggtaaaga tgtggttgaa catcggcaca atatggtcgg aaaattcaaa 1685100
caggataacg acggcgcaca aaatataaac aaccgccata aacggcacga caaattgggc 1685160
gatattggca atacggttca cgccgccaat cacaaccatg cccgcaagga cggcaagcac 1685220
aataccgact gccaaagaag gcacatcaaa tgcaatggta acggcagaag caatggagtt 1685280
tgcctgtgtc gcattaccga taaagcccaa tgcgataatc aacgcaatgg aaaagaaacc 1685340
ggacaaaaaa cgcgccgcgc ccctgccgat tttcggagtc agaccgtggg tgatgtagaa 1685400
cgccggcccg ccgatgtatt tgccgtggct gacgacgcgg tatttctgcg ccagcagtgc 1685460
ctccgcaaaa atcgtggaca tccccaaaac ggcagaaacc cacatccaaa aaatcgcgcc 1685520
cggcccgcct gcggtgatgg cggtcgccac gccggcaacg ttgcccgtac cgatttgcgc 1685580
agatatggca accgccaacg cctgaaactg cgataaagac ttgtcgtctt tatcgccttt 1685640
ggcaaacaag ccgccgaata cggatttgaa tcccgcgccc agcttggtaa tctgcggcgc 1685700
accaagatac agcgtaaaaa acaggccgat acccaaaagc gcgtaaatca gcaggtagtc 1685760
ccaaaggaac cgattgactg tacccaccag aacagacaat atattttcca taaaataaac 1685820
cttatcttac aattaaaatg actgccttcc aaaagacatt ccaataagga aacacggcga 1685880
gcagaccgta tttgccgcaa cagatgcctt aaattgtcaa caatcgggga gaagctgcgc 1685940
catcataccg taaaatatcg ttaattaaaa catttcttta tttttaagcg gaaagcggag 1686000
gaaatcgctt tcagacagca tagacaacgg cacggcataa aacaggatat tttgggtact 1686060
tgcaacttat gttaaaatgc cgaccgtaaa aaatctgaca aaaacagatt aattatttga 1686120
aataagaaag gaaatttatg gcaggccata gcaagtgggc aaatatccag cataaaaaag 1686180
cccgtcagga tgccaaacgc ggcaaaatct ttacccgttt aatcaaagaa atcaccgttg 1686240
cggcgcgtat gggcggcggc gaccccggtt caaatccgcg cctgcgcctg gctttggaaa 1686300
aagcagccga aaacaatatg cccaaagaca atgtgcaacg cgccatcgac aaaggcacgg 1686360
gcaacttgga aggcgtggaa tacatcgagt tgcgctacga aggctacggc atcggcggcg 1686420
cggctttgat ggtggactgc ctgaccgaca acaaaacccg caccgttgcg gacgtacgcc 1686480
acgcgtttac caaaaacggc ggcaacttgg gtaccgacgg ctgcgtggcg ttcaacttcg 1686540
tgcatcaggg ctatttggta ttcgaacccg gcgttgacga agacgcgctg atggaagcgg 1686600
ctttggaagc cggtgcggaa gacgtggtta ccaacgacga cggttccatc gaagtcatta 1686660
ccgcgccaaa cgattgggcg ggcgtaaaat ccgctttgga ggcggcaggt tacaaatccg 1686720
ttgacggcga cgttacgatg cgcgcccaaa acgaaaccga actctccggc gacgatgccg 1686780
tcaaaatgca aaaactgatt gacgcgctgg aagacttgga cgacgtgcaa gacgtttaca 1686840
cttccgccgt attgaatctg gactgatacg cagcacagca gacatacaat aaaatgccgt 1686900
ctgaaccttt cagacggcat tgatttattt agcccttgcc cacgcccacc aaaccgtcag 1686960
gacaaccgcc agaaaatacg ccacgctcca aacaggcaaa gcaattccca gcagataatc 1687020
cggttcagca caatttccga acccgcgcac gacaggctcg aaccaatcaa acaaaggcca 1687080
gcctttcaag cggaacgtcc acggcgcgcc gcacgaagga gcggttcccg gcggcagcga 1687140
ctgcaaccac aactgatatg ccgcaacaga aatacccgta acggccggaa tgctgataaa 1687200
gacagcaccg aacaaaccgc ctgcccttct tcttggtctg cacatcagga caattgccgt 1687260
acacaatgcg gttgccaaaa cgcataaccg ctgactgata cacaaaacgc aaggctccat 1687320
acccaaaaca tactgtgccg ccaaagaacc ggcaaatgca cagaccgaaa cggcaaacag 1687380
cagccaaacg gcttttctaa ataacggggt cattttctca acacaccaat caaaataccg 1687440
atatgccgat tttgctggat atatcccgag atggcaaggg acaaaacggc gatttgcccg 1687500
cacaatgccc acaaaaaaat acatccggag gatttgaatt tcagcaaatt tagcgaatca 1687560
aaagtttatt tcaatgaaat catatgattt ttttgaataa gcggattgat gggtttttga 1687620
aggaatttgt taccggatag ccatcgggca agtttttttgc aaaatttgaa tcggtcgggt 1687680

aaattttcaa aaaataattg acagcggata agaaacggcg gataattccc gccgtcgagt 1687740
tgcttgatgc agcttgattt ttctcctcta tttctccttt gtagacttgg cacacattca 1687800
actggatgtg tgcatttttt tatctccgct ttttttgaaa tttaattact tttattgttt 1687860
gaaattccat tctttaagaa atttaacaag aaaaacgctt gctttttgac cggaaaagct 1687920
gcataattca ttccgttagc tggttcgagt agtcagttaa tagtttctcc tctatttctc 1687980
ctttgtagac ttggcacaca ttcaactgga tgtgtgcatt tttttatctg aagcaacaag 1688040
cctctgtgcg tgatgttgtt atgtttcatt taggtgtcaa accgcatatc cggtctgaaa 1688100
tattcaatcc aaatccaaaa ccggattttc tttgacctcc tccatcacaa cataactcct 1688160
actctccgaa gcggcaggca gttgcaatag gatattgcct agcatatccc gataggcaga 1688220
catatcgggc aaacgtactt taatcagata gtcgtattcg cccgacacca agtggcattc 1688280
cataatttgc ggaattttca gcacttcttt tttgaaatct tcgaaaatat tgcccgattt 1688340
ggattgcagc ttcagctcga caaaaaccaa taaaggtttg cccaacagat ggggattgag 1688400
atgggcgtga taaccggaaa tataatgttc ccgctccaaa cggcgcaccc tctctgtaac 1688460
gggcgtggtg gacaagccta ccttctcggc aagctccgtc atcgggatgc gggcattctg 1688520
ttgaaggatc ttaaggatgc ggaaatcgat tttatctagt tctttcattt agattgcctt 1688580
gtatttatta ttgattttaa caaatagagt atatagtgga ttaacaaaaa ccagtacggc 1688640
gttgcctcgc cttgccgtac tatttgtact gtctgcggct tcgtcgcctt gtcctgattt 1688700
ttgttaatcc actatatatt tgagaaagcg attatatcag gaaaagcaaa ccgccttcct 1688760
acctgaaaac tgctgcttcg gcttgaagac acaaggttct ttaatatttt aaaagccttg 1688820
ccgttggatt ataatccccc aaccgatttc ttaattttgc taataaacac ttgcttggta 1688880
aggaatgaat ttatgcgccc tttgaacgtg cagatcaggt tgggcaacct taggcacaat 1688940
tatcggattt tgaaggaaat gcacggaggc aaactgttgg cggtagtgaa ggccgacgca 1689000
tacggacacg gtgcggtcag atgtgctttc gcgctggcag acttggcaga cggctttgcc 1689060
gtggcgacaa tcgatgaagg aatcaggctg cgggagagcg gcattaccca tccgattgtc 1689120
cttttggaag gcgtatttga agcatcggaa tacgaagcgg tcgaacaata ctcgctttgg 1689180
ccggcagtcg gaaaccaatg gcagcttgag gctttgctga tccgccattg gaaaaaaacc 1689240
gtcaaagtct ggttgaaaat ggattcgggg atgcaccgta ccggtttttt ccctcatgat 1689300
tacgcttcgg catatgcggc attgaagcag tcggaatatg tggacagtat tgtcaaattc 1689360
tcgcatttct cctgtgcgga cgaacccgaa agcggtatga cggaaataca gatggaagca 1689420
ttcgatttgg gtacggaagg gctggaaggc gaagaaagcc ttgccaactc cgccgctatt 1689480
ttgaatgttc ccgaagcacg cagggactgg gggcgcgccg gtctggcgtt atacggcatt 1689540
tccccgttcg gaggaggcga tgacaggctg aagcccgtga tgaggctttc aacccgtatt 1689600
ttcggcgaac gcgttttaca gccgcactcc cctatcggtt atggcgcaac attttatacc 1689660
agcaaatcta cgcgcgtcgg cctgattgcc tgcggttatg cggacggtta tccgcgccgc 1689720
gccccaagca attcccccgt cgctgtcgac ggcaaattga cccgggtcat cggcagggtc 1689780
tctatggata tgatgaccat cgagctggat gcttcgcaag aaggtttggg acacgaggtc 1689840
gaactgtggg gcgatacggt caacatcaat accgttgccg aagcggccgg aaccatccct 1689900
tacgaattga tgtgcaatat caaacgtgca aaattcactt atatcgagta atcaagtcca 1689960
aacgaaaatg ccgtctgaag cctttcagac ggcatttccc catcaaaacc gcaatcagtt 1690020
tttcatcgat tgaaccggag ccggaattct gccgcctcgg ttgacgaata cttcgcacga 1690080
accttctttg accggcatca caggcgcgta gcccaacaag ccgccgaact cgacgctgtc 1690140
gccgacggtt ttaccggtta ccggaataat gcgcacggca gtggttttgc tgttgatcat 1690200
gccgatggcg gcttcgtcgg caatgatgcc ggaaatggtg tgcgcgggcg tgtcgccggg 1690260
aacggcaatc atatccaagc cgaccgaaca aacggcggtc atggcttcga gtttgtccag 1690320
cgtcagcacg cctgcttcgg cggcggcaat catacccttcg tcttcggaaa cggggataaa 1690380
cgcgccactc aaacccccga ccgcgctgga agccatcatg ccgccttttt tcacggcatc 1690440
gttcagcaat gccaaagctg ctgttgtgcc gtgcgtaccg cagacgctca agcccatttc 1690500
ttcaagaatg cgtgccactg agtcgccgac ggcgggggtc ggcgccagcg acaagtcgag 1690560
aataccaaac gggatattca gcatttttga ggcttcgcgg ccgatgagtt cgcccacgcg 1690620
ggtaattttg aaagcagttt tcttcactac ttccgcaact tcggtcaatg tcgttgcatc 1690680
tgaattttcc aacgcggctt ttacgacacc tgggccggat acgccgacat tgataacggc 1690740
atccgcttcg cccgaaccat gaaacgcgcc cgccataaac gggttgtctt ccaccgcgtt 1690800
gcagaacacg acaatttag cgcagccgaa accttcgggc gtgatttccg ccgtgcgttt 1690860
gacggtttcg cccgccagct tgaccgcatc catattgata ccggcacgcg tactgccgat 1690920
attgatggag ctgcacacaa tatcggtagt cttcatcgct tcgggaatgg agcggattaa 1690980
cacctcatcc gaaggcgaca tcccttttg caccaacgcg gaaaaaccgc cgataaaaga 1691040
cacaccgatg gctttggcag ctttatccaa agtttgcgcc acgctgacgt aagaatcagc 1691100
atgggtggcc gccgcgattt gggcaatcgg cgtaacggaa atgcgctgat tcacaatcgg 1691160
tacgccgtat ttggcagaca gatattttgc cgtagtgacc aagtctttgc cgactgtggt 1691220
aattttattg taaatatttt ggttcaacac attgatatcg ctgctgatgc agtcgtgcaa 1691280
atcaatgccg atggtaatgg tgcggacatc aaaattctgg tcggcaacca ttttgacggt 1691340

ttctaaaatt tcgccggatt ggatactcat cacattcctc caactcaaat gcggtgcatc 1691400
gcttggaaga tttcttcgtt ttgcatacgg atatcaagcg cgagtttttt gctctcttcc 1691460
gcaaacaaat ccaaaacctc ttgacgcgat ttgctgcatt ttgaagtgtc caccaagata 1691520
atcatagtaa aaaaatcgtc catcagctgt tggctgatgt tgagaatatt gatttggttt 1691580
tccgccaaaa ttttggaaac atcgtacacg atgccgacgc ggtctttacc gatgacggtg 1691640
atgactgaat tgttcacagg cttactcctt gcagatatcc gttaaagtcc gaaattatac 1691700
caccgttgga ttttgaagaa atattgtcaa caatatatac atacaaaatg ccgtctgaaa 1691760
ctatttcaga cagcatcaag attcagggtt cgattaaata accatcctta tcccactggg 1691820
ttttcctgac caacttgtca tcctgataaa cagcttcgct ctttttagaa ccatcttcat 1691880
accactccaa aaccaccccg ttgcgttgat ggtggcggat agacagttcc gagagtaatc 1691940
ggccgctttc atcccaagtc agaattttgg caggctcatc gttgaccata accatttccg 1692000
tcttgatact gccgtcggca taccattgct tccatacgcc gtttgcctta ttttgcttaa 1692060
actggatttc gctttccttg ccgccgttac ggtaatagcg gtatcccgta ccctcactca 1692120
agccattttt ataaggcata acggcagatt ttttaccgtt cggataccag ttgacccact 1692180
ccccgtccgg cttacccttg ctgaagcccc ccgccatttt tttctgacca ttaaaatgcc 1692240
acaaaatcaa cataccgttt tgcagggtag gcacaaaaga tttgatttgc gttgaagcaa 1692300
cgatataagg ttcagaatat ttcttcatcg acggataata aaaatcctgc gcgtgcgcaa 1692360
tacccgccac cacactatat tgcctgatat aagcggcaga agacatcgtc gccgtcagct 1692420
ttccgttctg attaaaataa acagaatagg tctgcgccgg caaagcggcc gaaaaaccca 1692480
acaggacagt tgaaaataca atccgagata attttttcat tgcaatagcg atataaaaac 1692540
aaggctgtgt tttagtaatc tgttgatttc aattatttgc aagggaaaag acaattattt 1692600
tccggttagg aataaaccta ttctattgaa tatattgaag ccaagtacgc ctatcaacac 1692660
tatattaaaa cactgccaaa aacaattaac ttataaacaa tatggtaagg atttctctgc 1692720
caagcatcaa acccgagaca acgtatcgta aaaatgccgt ctgaaaacaa atcgtcttca 1692780
gacggcattt ccccttcaac tcactcttca cccaataact gctcgcgcgt caagaggaaa 1692840
acaaaaccgt cgcccccgct ggtttccaac caagtaaaag gcaactccgg atacgctgct 1692900
tccaatacat ccctgttatg cccgatttcc accagcaata cacctttggg attcagaaac 1692960
tttgccgcat tcagaagaat ctgcctggtg gcatccaacc cgtccgcccc gctgcccaat 1693020
gccaattccg gttcgtgcaa atactcttca ggcaataact caaccgattc cgcatccaca 1693080
taaggaggat tggaaacaat caaatcataa gtgccttcca atccttcaaa caaatccgta 1693140
tgaataagcc ggatgcgttc ttccaaacca taatcttcga cattaatccc tgccacttcc 1693200
aaagcatcca agctcacatc aaccgcatca atttgggcat caggataatg atgcgccatc 1693260
tgaatggcaa ggcaaccgct tccggtgcaa agatccaaag cattatgcac caactcatcg 1693320
tattctatcc aaggacgaag tccgtcaccc aacaattcat aaataaaaga acgaggtatg 1693380
attacgcgct catccacata gaaatcaaac tctccctgcc atgcctggtg tgtcaaataa 1693440
gcggctggaa tgtgttcgac agcacgacgc tcaataaccg ccagcacttc ctctttttca 1693500
gcttccaaga gttttgcatc aagatatggg gcaagcatat ccaaaggcaa attcaaagta 1693560
tgcagaatca aataagctgc ttcatcatgc gcattatctg ttccatgacc aaaaaagagc 1693620
cctgcctcat taaaacggct gactgcaaaa cgtaaaatat cgcggatagt cgtcaattct 1693680
tgtgctgcct gattaaacat aatatgaacc attctgcgta tagatacttt taattataac 1693740
agaaacaaca agcaaacctt ttcatatcgc caaataacca cccaatctac ccatacaact 1693800
acataaatgc ccgcgcgaaa accatcgccc gaacggaaac gacaatggcc gacggtatgg 1693860
gcaatctgat tggctgggaa aaaacggggc ttgttgtcgg taagcagtgg ataaccgcaa 1693920
aagacgacaa ggtgtccgat gtctgcaatg ccaacggcga gatgggcgta atcgggcttt 1693980
acgagccttt ctcacacggc gcattgacga tacccggtca tccgaactgc cgatgcgagg 1694040
ttgtttccgt atcgggtggc gaattggggg aatttgccga aaaaaaggag cttcgtaaag 1694100
cggctatgca gtatgcgcgg gataacttta tcggcaaaag ctatgtcaat aaaaacagcg 1694160
ggcatgaact gaaggtaact tggcaaggtg tgaaacacgc tgcgtcaaag gcaaatcagg 1694220
cggaattatc catcatgaca aaacttgatg acttattgcg ctacgcaaaa tatgagggtt 1694280
cttattcgga taggaaaggt catcctaata ttattgcagc acataagtat cgtgccgttg 1694340
ccaaggttgg gaatgagtct ttaaatatcg gtgtgattgt aagggaattt ccagacgacc 1694400
ataaacatta cgaccatttc atcttgaagg atgaataaag cccttttgca gtgtcgttct 1694460
ggagcggata gcgttaaggc aagtacactt ccagccttga aaaagggctt taaattcagc 1694520
atgccattta tacaggcagg agtaaaccca tgacaaagtt atacgcagaa atcgccaaga 1694580
tggagacgca ggacgacgac acggtcaagg tttggggtta cgcttcaagc gaggaaatcg 1694640
attcggacgg cgaagtcatc gcggcggcag ctatgaaggc ggcgattccc gattatatga 1694700
agtttggcgc ggggcgcgag atgcacggct caaacgctgc gggaacggca attgaaatca 1694760
acgtggaaga tgacggcaga accttttttcg tggcgcatat cgtcgatccc gttgccgtga 1694820
cgaaggtcaa aacaggcgtt tacaagggct tttccatcgg cggcagcgtt accgcccacg 1694880
atgagttgaa caagtcgcaa atcacgggtt tgaagctgac ggaaatcagc ttggttgacc 1694940
gacccgccaa tcccgatgcg gtgtctacct gctttaaggc ggacaaaggt gcggaagcgg 1695000

taaacaacga tacagaacat aatgctacat attttagcca tttcccttcc aaacaaaaaa 1695060
gcaccgacgg cggccgatgc cctttccttt acaggttccc ctattttttta tccgcgggca 1695120
gcaccggttt ggctggggct tttggtgcgg gcgcgccgac cgaagcctgg tccttcagct 1695180
tcgccagcac cgcagggccg atgcccttta ccttggtcaa atcgtctaca gacttgaacg 1695240
caccgttttg cgcacggtat tccgcaatgg ccttcgcctt cgccgggcct atgcccggca 1695300
gcgcctccaa ctcctgctgc gaagccgcat tgatgtttac cgccgcaagg gagaaggcgc 1695360
aggagaacag catacagaac agcacgaaca ttttcttcat ggtttttcct ttaagggttg 1695420
caaacaataa accgcatctt gcgacgataa aacgagtcat tctaaaatga atatcccaaa 1695480
gtttcaagcc gttcctccgc aaacccgacc ggacaccgta cggatgccgt cccgccatca 1695540
ccgacatttt ttccgggcaa agcaaacatt ttttccgggc aaagcaaaaa cccccgaata 1695600
atcggggggtt ttctgaatgg gtgtttggca gtgacctact ttcgcatgga agaaccacac 1695660
tatcatcggc gctgagtcgt ttcacggtcc tgttcgggat gggaaggcgt gggaccaact 1695720
cgctatggcc gccaaactta aactgttaca aatcggtaaa gccttaatca atatattcgg 1695780
taatgactga atcagtcagt aagcttttat ctcttgaagt tcttcaaatg atagagtcaa 1695840
gcctcacgag caattagtat gggttagctt cacgcgttac cgcgcttcca caccccacct 1695900
atcaacgtcc tggtctcgaa cgactcttta gtgcggttaa accgcaaggg aagtctcatc 1695960
ttcaggcgag tttcgcgctt agatgctttc agcgcttatc tcttccgaac ttagctaccc 1696020
ggctatgcaa ctggcgttac aaccggtaca ccagaggttc gtccactccg gtcctctcgt 1696080
actaggagca gcccccgtca aacttccaac gcccactgca gatagggacc aaactgtctc 1696140
acgacgtttt aaacccagct cacgtaccac tttaaatggc gaacagccat acccttggga 1696200
ccgactacag ccccaggatg tgatgagccg acatcgaggt gccaaactcc gccgtcgata 1696260
tgaactcttg ggcggaatca gcctgttatc cccggagtac cttttatccg ttgagcgatg 1696320
gcccttccat acagaaccac cggatcacta tgtcctgctt tcgcacctgc tcgacttgtc 1696380
ggtctcgcag ttaagctacc ttttgccatt gcactatcag tccgatttcc gaccggacct 1696440
aggtaacctt cgaactcctc cgttacgctt tgggaggaga ccgcccccagt caaactgcct 1696500
accatgcacg gtccccgacc cggatgacgg gtctgggtta gaacctcaaa gacaccaggg 1696560
tggtatttca aggacggctc cacagagact ggcgtctctg cttctaagcc tcccacctat 1696620
cctacacaag tgacttcaaa gtccaatgca aagctacagt aaaggttcac ggggtctttc 1696680
cgtctagcag cgggtagatt gcatcttcac aaccacttca acttcgctga gtctcaggag 1696740
gagacagtgt ggccatcgtt acgccattcg tgcgggtcgg aacttacccg acaaggaatt 1696800
tcgctacctt aggaccgtta tagttacggc cgccgtttac tggggcttcg atccgatgct 1696860
ctcacatctt caattaacct tccagcaccg ggcaggcgtc acaccctata cgtccacttt 1696920
cgtgttagca gagtgctgtg tttttaataa acagtcgcag ccacctattc tctgcgaccc 1696980
tccggggctt acggagcaag tccttaacct tagagggcat accttctccc gaagttacgg 1697040
tatcaatttg ccgagttcct tctcctgagt tctctcaagc gccttagaat tctcatcctg 1697100
cccacctgtg tcggtttgcg gtacggttcg attcaaactg aagcttagtg gcttttcctg 1697160
gaagcgtggt atcggttgct tcgtgtccgt agacactcgt cgtcacttct cggtgttaag 1697220
aagacccgga tttgcctaag tcttccacct accggcttaa acaagctatt ccaacagctt 1697280
gccaacctaa ccttctccgt ccccacatcg catttgaatc aagtacagga atattaacct 1697340
gtttcccatc gactacgcat ttctgcctcg ccttaggggc cgactcaccc tacgccgatg 1697400
aacgttgcgc aggaaacctt gggctttcgg cgagcgggct tttcacccgc tttatcgcta 1697460
ctcatgtcaa cattcgcact tctgatacct ccagcacact ttacaatgca ccttcatcag 1697520
cctacagaac gctcccctac catgccggta aaccggcatc cgcagcttcg gttatagatt 1697580
tgagccccgt tacatcttcc gcgcaggacg actcgaccag tgagctatta cgctttcttt 1697640
aaatgatggc tgcttctaag ccaacatcct ggctgtctgg gccttcccac ttcgtttacc 1697700
acttaatcta tcatttggga ccttagctgg cggtctgggt tgtttccctc ttgacaacgg 1697760
acgttagcac ccgctgtctg tctcccgagg aaccacttga tggtattctt agtttgccat 1697820
gggttggtaa gttgcaataa ccccctagcc ataacagtgc tttacccccca tcagtgtctt 1697880
gctcgaggca ctacctaaat agttttcggg gagaaccagc tatctccgag tttgtttagc 1697940
ctttcacccc tatccacagc tcatccccgc attttgcaac atgcgtgggt tcggtcctcc 1698000
agtacctgtt acggcacctt caacctggcc atggatagat cactcggttt cgggtctaca 1698060
cccagcaact catcgcccta ttaagactcg gtttccctac gcctccccta ttcggttaag 1698120
ctcgctactg aatgtaagtc gttgacccat tatacaaaag gtacgcagtc acaccactag 1698180
ggcgctccca ctgtttgtat gcatcaggtt tcaggttctg tttcactccc ctcccggggt 1698240
tcttttcgcc tttccctcac ggtactggtt cactatcggt cgatgatgag tatttagcct 1698300
tggaggatgg tccccccata ttcagacagg atttcacgtg ccccgcccta cttttcgtac 1698360
gcttagtacc gctgttgaga tttcgaatac gggactgtca cccactatgg tcaagcttcc 1698420
cagcttgttc ttctatctcg acagttatta cgtacaggct cctccgcgtt cgctcgccac 1698480
tacttgcgga atctcggttg atttcttttc ctccgggtac ttagatggtt cagttctccg 1698540
ggttcgcttc tctaagtcta tgtattcaac ttaggatact gcacagaatg cagtgggttt 1698600
ccccattcgg acatcgcggg atcattgctt tattgccagc tcccccgcgc ttttcgcagg 1698660

cttacacgtc cttcgtcgcc tatcatcgcc aaggcatcca cctgatgcac ttattcactt 1698720
gactctatca tttcaagaac ttctttgact ttgcctaaca ttccgttgac tagaacatca 1698780
gacttgaatt tcctactttg ataaagctta ctgctttgtt gtgtcttaat cctgcctttt 1698840
gtgtttcagg attaagtcga tacaatcatc acccaaatac tgtgtttgtt ttcttttctc 1698900
ttgcgagaga tttttatcct ttgcaaagaa taaaaaatca aaacaaacgc tttgtctttg 1698960
tttgttgatt tcggctttcc aatttgttaa agatcgatgc gttcgatatt gctatctact 1699020
gtgcaaatca aaacgagctg attattatat cagcattttg ttcttggtca agtgtgacgt 1699080
cgccctgaat ggattctgtt ccattcttcc gttttgattt gtacagtatt ggtggaggca 1699140
aacgggatcg aaccgatgac cccctgcttg caaagcaggt gctctaccaa ctgagctatg 1699200
cccccgttct tggtgggtct gggaggactt gaacctccga ccccacgctt atcaagcgtg 1699260
tgctctaacc agctgagcta caaacccgga ttctcttctt aagcgaatct tgccttcact 1699320
caagcttctt ccgcatcttt ttcagtttac cgataagtgt gaatgcctaa agcctcttct 1699380
ttctctagaa aggaggtgat ccagccgcag gttcccctac ggctaccttg ttacgacttc 1699440
accccagtca tgaagcatac cgtggtaagc ggactccttg tggttatcct acctacttct 1699500
ggtatccccc actcccatgg tgtgacgggc ggtgtgtaca agacccggga acgtattcac 1699560
cgcagtatgc tgacctgcga ttactagcga ttccgacttc atgcactcga gttgcagagt 1699620
gcaatccgga ctacgatcgg ttttgtgaga ttggctccgc ctcgcggctt ggctaccctc 1699680
tgtaccgacc attgtatgac gtgtgaagcc ctggtcataa gggccatgag gacttgacgt 1699740
catccccacc ttcctccggc ttgtcaccgg cagtctcatt agagtgccca actgaatgat 1699800
ggcaactaat gacaagggtt gcgctcgttg cgggacttaa cccaacatct cacgacacga 1699860
gctgacgaca gccatgcagc acctgtgtta cggctcccga aggcactcct ccgtctccgg 1699920
aggattccgt acatgtcaag accaggtaag gttcttcgcg ttgcatcgaa ttaatccaca 1699980
tcatccaccg cttgtgcggg tccccgtcaa ttcctttgag ttttaatctt gcgaccgtac 1700040
tccccaggcg gtcaatttca cgcgttagct acgctaccaa gcaatcaggt tgcccaacag 1700100
ctaattgaca tcgtttaggg cgtggactac cagggtatct aatcctgttt gctacccacg 1700160
ctttcgggca tgaacgtcag tgttgtccca ggaggctgcc ttcgccatcg gtattcctcc 1700220
acatctctac gcatttcact gctacacgtg gaattctacc tccctctgac acactcgagt 1700280
cacccagttc agaacgcagt tcccgggttg agcccgggga tttcacatcc tgcttaagta 1700340
accgtctgcg cccgctttac gcccagtaat tccgattaac gctcgcaccc tacgtattac 1700400
cgcggctgct ggcacgtagt tagccggtgc ttattcttca ggtaccgtca tcagccgctg 1700460
atattagcaa cagccttttc ttccctgaca aaagtccttt acaacccgaa ggccttcttc 1700520
agacacgcgg catggctgga tcaggcttgc gcccattgtc caaaattccc cactgctgcc 1700580
tcccgtagga gtctgggccg tgtctcagtc ccagtgtggc ggatcatcct ctcagacccg 1700640
ctactgatcg tcgccttggt aggcctttac cccaccaact agctaatcag atatcggccg 1700700
ctcgaatagc gcaaggcccg aaggtcccct gctttctctc tcaagacgta tgcggtatta 1700760
gctgatcttt cgatcagtta tcccccacta ctcggtacgt tccgatatgt tactcacccg 1700820
ttcgccactc gccacccgag aagcaagctt ctctgtgctg ccgtccgact tgcatgtgta 1700880
aagcatgccg ccagcgttca atctgagcca ggatcaaact cttatgttca atctctaact 1700940
ttttaacttc tggtctgctt caaagaaacc aacaggacaa tgttcaaaac attatcttgt 1701000
ctgtctttca aacagtgtga gactcaaggc actcacactt atcggtaatc tgttttgtta 1701060
aagagcgttg cgaattataa agtattcctt ccgcctgtca agatatctct cgatatcccc 1701120
aacattctgt gctatacttt tcagttcgtc cgccacttct gcagcagcga agaaccgaac 1701180
tatacgccca cagggaaaaa cggtcaatgc tttcagcggg attttttggg ggaaattcgt 1701240
catgtcgctg tcggataagg ttttttattt ctgctaaata ctgcgccgcc tccaacaatc 1701300
ctttcctctc cctcctccgg ctggtgcgcc tttgtgaata tgctgtctga aactcgggga 1701360
ctcagacggc attttgtatc caaacggtat ctaatgtatc cgtactttgt tatagaatgg 1701420
ctgctgtttt ttcttcgtaa ttagaaattg tcaaaatggg caaacatatt cttttaggtg 1701480
taacgggcag tattgcggcg tataagtctt gcgagttggt gcgactgctg aaaaaacagg 1701540
ggcattcggt tacggtggtt atgagccgct cggcaactga atttgtttct ccgctgactt 1701600
ttcaggcttt aagcggcaat cctgtcctga ccgacacgca cggcggcaac ggttcaaacg 1701660
gtatggaaca tatcaacctg acccggaatg cggatgtttt tctgattgcg ccggcaagta 1701720
tgaataccgt ggcaaaaatc tgtaacggcg tggcagataa cctactgacc agtctggcag 1701780
ccgcacggaa atgtccgctt gccatcgcgc ccgcgatgaa tgtggaaatg tggctcaacc 1701840
ctgccaacca acggaatatc gcacaactgg tttcagacgg cattactgtc tatatgccgg 1701900
gcttgggcga acaggcttgc ggagaaaatg gtatgggaag gatgccggaa cctgccgaat 1701960
tgctggatct gcttccggat ttatggacac cgaaaatttt aaagggcaaa aaagtcttga 1702020
ttaccgcagg tgcgacattt gaagccattg accctgtccg aggcatcaca aatatctcca 1702080
gcgggaaaat gggcgtggct ttggcgcggg cgtgccgtgc cgccggtgca gaagtcagcc 1702140
tgattcacgg acagcttcaa accgcgctgc ctttcggcat atccgatacg gttcaagccg 1702200
tcagtgccga aaatatgcat cgcgcagtgc atcgtttaat cgacaaacaa gatgctttta 1702260
tttctgttgc cgccgtctca gactataggg ttaagaatag gagtactcaa aaattcaaaa 1702320

aagataaaaa tgccaaaccg ttatccatcg aattggatga gaaccccgat attttggctt 1702380
ctattgcctc attaccgaac ccgccgttct gcatcggttt tgccgctgaa acggagaatg 1702440
taatgacata tgcgcgggaa aaacgtatta agaaaaagct accgatgatc gttgccaatg 1702500
atgtttcaat cgcaatgggc aaaccgacca accggattac cattatcggg gacgacgggg 1702560
aactgtcttt tcccgaaaca agtaaagatg aagcggcaat gcggattgtt gaaaggcttg 1702620
ccgtatattt gagcaaataa gcaattgaac ggataaacca taaaacgggt tgcctgttaa 1702680
tcaaaaggca acccgtttta cctgcttcaa cttctgatga ctttgcggat atatggaata 1702740
ctatgcagat tttgaataat ctgattcaat tgattcagat tcttgacttt caataagaat 1702800
ttgaattcga caaaaccttc cgttcccgac tgggatttag acggtgtttc gaccgactca 1702860
atgtctgcac cggaatcgga aatcgcttgc gccattaatg ccaacaggcc gtggctgtct 1702920
tccgattgga cttgaagccc gacacggtag ttctgcccgt tcatattttc ccagtctgca 1702980
tccagctgct gttcgggatc ggacttcaac aacgtcgggc aggtatccct atggataatc 1703040
atgccttttc ccttaaccaa cagcaaacgg atggaatcgc cgggaacagg gtggcagcac 1703100
tctgcaaaat gaatatgccc gctttcctgc ccatcgactt taatggaact gagcctgacc 1703160
tcgctgccga aatgctcccc tgccaactcg gcaatgtgca tggcgacata aacaggcagg 1703220
gtatgcccca tccctacgtt gtacagcact tcttcaaacg atgtctgctt gtcgttgaga 1703280
tcggcaagat atttttcctt gatgccgtct gaaagcagga catctttggg cagcaaactg 1703340
gacagggctt tttgtaagag gctctctccc aaaacgaccg catcgtgccg gttaaggttt 1703400
ttaatatatt ggcgtatggc gctgcgcgcc ctgcctgaca cggcgaaatt caaccacgcg 1703460
ggattgggtt tggcgtgttc ggatgtgata atttcaacag aatcaccggt tttgagcttc 1703520
gtacgcaacg gcatcatgat attgttgata cgtgcggcaa cggttttgtg cccgatatcg 1703580
gtatgcaccg cataagcaaa atcgacaggc gttgcccctt tgggcaaagt taggattttt 1703640
ccttttggcg taaggatgta gatttcgttc ggaaacaaat cgactttgac gtgttcgaga 1703700
aactcaatgg cattggcact gcttgcctgc aaatctaaga tatttttcag ccaccggttt 1703760
gtgtgaagca ccgcctgatc gaccgtctta gaatatgatt tatagctcca atgtccggcg 1703820
attccacctt cggcaacagc atccatttcc ttggtacgta tctgaacttc aatcggcaag 1703880
ccgtaagggc cgaccaaagt cgtatgcaga ctttgatacc cgttgctttt cggaatggcg 1703940
atatagtctt tgaaccgccc gggcttgggc tgatagaggg tgtgcaatgc gccgagtgcg 1704000
gcataacagg ctggaatgct gttgacaatg acgcggaaac cgtaaatatc cataacctcg 1704060
gcaaagcgca gctttttcgc catcattttc tgatggatgc cgtacaggtt tttttccctg 1704120
cctttgattt tggcctctat attcgcgcct accagccgct ggccgaatgc gcgcaagact 1704180
ttgccgacaa cgtcctgccg gttcttccgg ctcttgtcca tcgctttttt taaagtctcg 1704240
tagcggttgg gatgcaggtt ttggaacgat aaatcctgaa gctcttgata tgcgttattc 1704300
aaacctatac ggttggcaat ctgtgcatag atttcaaggg tttcccttgc aatccggcgg 1704360
cgtttgtccg ggcgcatcga accgagcgtc cgcatattgt gcaggcggtc ggcaagtttg 1704420
acgacaatca cgcgcacatc tttggtcatt gccaaaatca gtttgcggaa actctccgcc 1704480
tgatgctccg catgatcttc aaatttgagt ttttcaagct tggacagacc gtccaccatc 1704540
tcggcaatcg tattgccgaa caccgccgcc atttcccctt ttgtcacgcc cgtatcttcc 1704600
aatacgtcgt gcatcacgcc tgcacaaaga ccctgtatgt ccatatgcca aagggcgagc 1704660
tgcgtcgcaa cggcaatcgg atgcgtgatg tagggctccc cgcttttgcg ggtttgcccg 1704720
tcgtgggcgc gaaacgcata ggcgacagct tttcaagct ccgcctgttc ctcgggcttg 1704780
aggtaggagg cggtatggaa aagcagggca cgcgcttcgg cggtcagggg gtcgtaaggg 1704840
gcggaaggtt gggggcggg catttcagac ggctttcggt atgtatgtgt ttttcatttc 1704900
aaaccgtcgg actgcacggc ggcaaagtgt tccggcgtgc gtttccggca gaatttattt 1704960
attgcgcgtc aacagttctg taccgatatg tccggcggcg atttccctta aggcggtaac 1705020
ggtcggtttg ttattgcgga catcgtccac aagcggcgtg ttgccgttct caagctggcg 1705080
ggcgcggcga gccgctacca atgtcaggtc aaaatggttg gaaatttttc cggtacagtc 1705140
ttcggtggta atacgtgcca tattatttgc tttctttcaa aaatatttaa attgggaaac 1705200
cgggtatttt cgccgttttc taggaatttt ccaacaaatc tgcaataaac cccagttgcc 1705260
gcgacctttt cagacggcag gcattcacaa tatggcgcaa atcctcctcc gctcgcgcca 1705320
agtcgtcatt gaccacgaca aagtcaaaca atacggactg ctcgatttca tgccttgcct 1705380
tcgacagcct cctttggata acttcccgac tgtccgtccc gcgtccgttg aggcgcgcgg 1705440
caagtacgtc gaaagaaggc ggcaggataa agatgccgac ggcttcgggc agcgcgtcgc 1705500
gaacctgcgc cgcgccctga acgtcgattt ccaaaatcac gtcatagcct gccgccgcca 1705560
acgcattcac accctccgcg cctgtgccgt aatagttgcc aaatacgtcg gcgtattcca 1705620
aaaaagcttc ctgcgcgata agcgactcaa actcttcttt ggaaacaaag tgataatgta 1705680
cgccgtttgc ttcgccttca cgcggcgggc gcgtcgtgtg cgacacggaa acgcgcaaac 1705740
cgttatggtt tgccaacagc cgcgacacca gcgtggtttt gcccgtgccg gaagcggccg 1705800
aaatgataaa gatgttgcct tttcgataag cggacatatt ttttacctgt atattttcca 1705860
gccgattgta tcacaatgga cacccagttt cctatttgcc gatgcccata ttttgccgct 1705920
attgttttga ttgatttggc aagcgacagg ctgacggcta caatatggcg ttaaaaacat 1705980

caaacttgga acacgcaatg ctggttcatc ccgaagctat gagtgtcggc gcgcttgccg 1706040
acaaaatccg caaaatcgaa aactggccgc aaaaaggcat cttattccac gacatcacgc 1706100
ccgtccttca aagcgcggaa tacttccgcc ttttggttga tttattggtt taccgctata 1706160
tggatcagaa aatcgacatc gttgccggtt tggacgcgcg cggcttcatt atcggcgcgg 1706220
cactcgccta ccagctcaac gtcggtttcg tccccatccg caaaaaaggc aagctgcctt 1706280
ttgaaaccgt atcgcaaagc tacgcgctcg aatacgggga agctgcggtg gaaatccaca 1706340
ccgatgccgt caaactcggt tcgcgcgtgc tgctggtcga tgatttgatt gccacgggcg 1706400
gcacgatgct tgccggactg gaactgatcc gcaaactcgg cggagaaatt gtcgaagccg 1706460
ccgccatttt ggaatttacc gaccttcaag gcggcaagaa tatccgtgca agcggcgcgc 1706520
ccttatttac cctgcttcaa aacgaaggct gtatgaaggg ctgaaaaccg accctgccgt 1706580
ctgaaaccgg cagggttgtt atgatgcgtt caaatcacgc ccaaatcttg caagcccctc 1706640
aacacgccgt cttcatcaac gctggggcaa acatatttcg ccgcttcttt cgccgcctgt 1706700
tccccgttgc ccattgccac gccgaacccg acttctgaca gcatttccac atcgttcaaa 1706760
ccgtcgccga acgccatcac gtctgccatt tcccatccca atgcttcaac cacgcttctg 1706820
atgccgtccg ttttcgacgc acccgcaggc agcagatcga ccgcttcctc gtgccagcgc 1706880
accgttttca agccttcccg ttccacaata tccgaccaaa gcggcatttc gttttcctcc 1706940
gcaaacacca gcatctgata caccggtttg cttgaaaaat aatccttatc ggcaaaaaaa 1707000
tcgctggcga tatgcttcaa ggcgcggcac acgcattccg acagcgcgga cacagcgatc 1707060
ccctctccgc cgacaaacgc ataatccatg cccaagccat ccaaatgcgc gcaaaccctg 1707120
cccatcaaac cggcatccat cggtacttcg cgcacggttt ttccgtgcag cagcgcaaac 1707180
tgtccgttta tcgttaccac ggcatccatt cccgcttccg ccatcatatc cctgaccttt 1707240
tcgggaatcg tcgccaaaga ccgccccgtt gccaacgccg tcaatatacc tttgccgcgc 1707300
aaagccgcca ccgccgtttt cacggaaggg cgcaaagtat ccgtatattt tcggtacagc 1707360
gtatcgtcaa tgtcgaaaaa cacgatttta ggattcatca cattctctct cgcattcaaa 1707420
ctaccgcatt atatcccaag caggcaaata cttgataaat ccttataaat ttcccgtcaa 1707480
aattgaccga aaatacaaaa aggcggataa tccgcccatc ctcaaaccct tttcagacgg 1707540
catttgcagc aatgccgtct gaaacatttt tacaaagcat acaaatcatg tttcaacaca 1707600
caggacgaca cataaagcgt cgccctatat gttgccctga ttcggaaggg gttacgcccc 1707660
tcccaaataa agtctgattc tactgcccta aagggcgggg tttcaaccga aaaggaaaca 1707720
cgatgaaagc acccgaactc ttattgcccg ccggcggatt ggaaagaatg cgcgccgcct 1707780
acgactacgg cgcagacgcc gtttacgccg gcagcccgcg ttactcactg cgcgcccgca 1707840
acaacgaatt tgccaaactt gatgttttag aacaaggcat taaagaagcg cacgagcgca 1707900
acaaaaaatt ctttttaacc gtcaacaccc tgccgcacaa ttccaaactc aaaaccttcg 1707960
ttgccgacat ggagccgctg attgccatga aacccgacgc gctgattatg gcggatccgg 1708020
gtttgattat gaccgtgcgc gaaaaatggc cggaaatgcc gatccatctg tccgtacagg 1708080
cgaacaccac caactattgg ggcgtgaaat tctggcaaaa catcggcgtc gaacgcatta 1708140
ttctgtcgcg cgaattgagt atggaagaaa tcgccgaaat ccgccaagaa tgccccgaca 1708200
tcgaactcga agtcttcatc cacggcgcat tgtgcatcgc ttattcaggc cgttgcctat 1708260
tgtcgggcta tttcaaccac cgcgacccca accaaggcac ctgcaccaac tcctgccgtt 1708320
gggattacaa ggttcacaat gccacggaaa gcgatgcagg cgatgcccag cttctgcaag 1708380
gtttcaactt tgaaaaagcc caagaagaag ccaaccaaaa ctttgaaggc atcaacggtc 1708440
aaaaacgcca tccctacgcc gacaaagttt tcctgattga agaatccaac cgcccgggcg 1708500
aaatgatgcc gattatggaa gacgaacacg gcacctacat catgaattcc aaagaccttc 1708560
gcggtatcga agtcgtcgaa aaactcgcca aaatcggcgt ggacagcctc aaagtcgaag 1708620
gccgtaccaa atcgctctat tatgttgcac gcgtcgccca gtcctaccgc aaagcgattg 1708680
atgatgccgt cgcaggccgt ccgtttgatt acagcctgtt gagcgaactc gaaggcctcg 1708740
ccaaccgcgg ctacaccagc ggcttcctcg aacgccacca aactcaggat tatcaaaact 1708800
acctgaccgg ccattccacc gccaaacaaa gccaatacgt cggacacgtt accgaaatcg 1708860
atgaaaacgg ctgggcaaca gtggaagtca aaaaccgctt tgccgtcagc gattcactcg 1708920
aaatcatcca cccgagcggc aaccaaacca tcaaattgga acaaatgacc cgcaaaggcc 1708980
agcctgtcga tgttgccccg ggcaacggca ttcaggtcaa aatccccaat atgcagggta 1709040
aagaaaaagc cctcatcgca cgcgtgttga accccctaagc cattatgccg tctgaaacat 1709100
ttttcagacg gcatttttaa tccccttgcc ttattgtgcg gcagattcag atcgggacac 1709160
acctatagtc cacgacagaa gtctggcttt taagcgaaga caataaccga aaagaccgcc 1709220
tcggcggtct ttcttgtgta cacagttttt gtatttgtca gcttgatgcg ttgacaactc 1709280
taattccata ttgcggaata tattcatcga cagtcatcag ttcaaagcct tccgcttggg 1709340
tttgtgcaat caacatccta tcgaaagggt ctttgtgtat ctccggaagg cttccagcct 1709400
gttttgcatg aaacagacct ataggcaaca tttcaaaatc ctcttcttga agcacatcaa 1709460
aaaactcttc cggtaatttc aacaacccct tgttctgctt gatggaaatt tcccaaatac 1709520
ttgctgcact gacaaagatc gcatttctcg gattttctat cagtttgcgt gcagatatcc 1709580
ccagtttctt gtcatccaac aaccaccaca gcaacgcatg ggtatcaagc agaatctttc 1709640

tcacagagcc gactcctcaa aaaataaagc tgccgtttca ttgtcatcct caagaatacg 1709700
tgaaatatcc gtattttcca tatgactgaa ttttttcaac cttcctgcat ttcgtgccgg 1709760
tttttcaata ccgattagtt ggacgcaagg cttacctgcc ttcgcaataa taacgatttc 1709820
ccctgcttct gctctttgaa tcaattgact caaattggtt tttgcctgat gaatatttgc 1709880
ttgaaacata acacttctca tgattagcta acttgactaa tatacatcat taccaagatt 1709940
ttgggaatct cattacatat atttgattat atccgccgtt ttattcacac cttgctattt 1710000
atagtggatt aacaaaaacc agtacggcgt tgcctcgcct tagctcaaag agaacgattc 1710060
tctaaggtgc tgaagcacca agtgaatcgg ttccgtacta tctgtactgt ctgcggcttc 1710120
gtcgccttgt cctgattttt gttaatccac tataaaacgg ctttgcggta tcccagtttg 1710180
acaccggtta cttcctgatt ggtaagcatc attattttcc cataaatcaa acgtctgaca 1710240
cggcattata aacacaatgc ggcatctgcc gccacccttg cggacgcggc gttaccggct 1710300
tccacagcta cttcgacaag cagccgctgc aaggcggaca atacgactgt caggcaggct 1710360
cgttccacgt ccgcgtggtc atgcgcgcca acgtcgtccg ttaacatatc ggcaaccaaa 1710420
aaatgccgtc tgaaacattt ttcagacggc atttttaatc ctgcaacatt accccctgcc 1710480
tgagttcgga tactgtatca atataaaacc ccatcacaca gatttacggt aaaaagccgt 1710540
ccgaatgaat tcttgaacac aattcggacg gctttaattt tcaacaaggc gattaattca 1710600
ataataccag attaaaactt ccattccagc gatacggcgt aattgcggcc tggggcgcgg 1710660
tagcggtcta agcctttgcc atcgcggtcg accgcattgg tggtgctgta gctatataaa 1710720
ccgcgcaggg aatcccaagt ggtgtatttg cggttgaaca ggttgtacac gcctgcacgc 1710780
aaagtcaggt ttttagccgg tttgtagaag ccgtacatat caaacacata agccgacttg 1710840
ttcagccacg ggtaatcttt tacctttttc tgcaaaggcg taccccagcc cttgttttca 1710900
taaacggtgt attgcgcgtc tttgaccttt ttcgcgccta gataggtcag gcgggagaat 1710960
acgccccatt tttcgctcgg actttcatag tcgataccgg caatcacttt cagcggctgt 1711020
gtggacagca ggctgttgtc gcccgacagt ttgcttttcg cataacccag cgagccgaac 1711080
agtttccaac cctcaggaac aaaagacgct actttgtcca cattcagacg gcctgtcagc 1711140
tcgataccgc ggattctggc cttgtcgata tttttcatct gccaatccag tttttctttg 1711200
taggggtcgc tgcatatacc gtagtaagca ttttcctcag tacagccggg agtgccgctg 1711260
gtggtcagct tctgctcttc agacaggaaa ttgcggtaat tgctttgata caggttggca 1711320
tccagcatgc ctttttcgct gcggccttgc agagacaggg tgtgggtggt gctgcgctcg 1711380
gctttcaggt tgggattggg cagccaatta cccgaaccgt ggttgtaagt gaaatacact 1711440
tcggacgcat tggggacacg gtagccggaa gtaatgtcgt aaccgacacg ccaagcctga 1711500
ttcagttgcg ccgccaagcc gacaaaaccg ctccagcctt tataagtgtt ggctgcaggt 1711560
ggtgttttgt cacaagcatg acactcggca ttcaattcct gaggcgtcat tttggtgtgg 1711620
tcgtaacgga tacctgcgcg gctactgaac acgtcgttcc attgaatttg gtcagacagt 1711680
gagaaaccgt agttggtggt tttcaccgga tgctggatac tgctggtggt tcgaacaaca 1711740
cggccgctga agtaataatc gtcgcggttt aggttttcaa aatcacggcg gctgacgaaa 1711800
gttttaaacg acaggcggtg tcgccccccc ccgagttgca acggatggct gtccaaacgc 1711860
aaagtaaaac gtttgaatcg ggtgtccatg ctgcggttgt atatttcgtc caaatccttc 1711920
tgattatagt tgcgcgtcca ggtggaataa tccatcggga acgagccttt gttgttaacc 1711980
gccgccactt tggttttctg ataatcgaag tccgccttca aagacgacaa ccaatttgaa 1712040
tcaggcatcc attcgtaaaa gaggttggca ttgcgccgtc tgtttacgtc atcggcttcg 1712100
cgccaggaag aagcggtcag gttataagac tcttcaaccg tgtaattatg tccctgctgg 1712160
ccgttaagcg atgcgccgat gcggtggtta tcgttaattt ggtaagcaat cttacccaaa 1712220
aagctgtggt atttgtgttt ggacgaatca gggataccgc gtgccgaacc acggatattc 1712280
gcgccactgc cttccccttc cacagcatag cctcggtttc ccgcactttc ggtttcatga 1712340
ccgcgacgtt gcgaatacag caaagcagca tccacgcggt cgttactcac accgaaaccg 1712400
agagtatttg tccattcacg gttacgcgtg ctgtaaccgt ttttcatcat cacgccgaat 1712460
tgcctgtcgt ccaacagcaa atcacggcct tgcagcgttt ggtaattcac accgccgccc 1712520
aatgcaccac tgccggtatt gaaagagtct gcgcccttca cgatttcgat gttgcgcacg 1712580
agttcggggt cgatagacaa acgcgagctg ttgaagttgc cataacgggc gtacagcgag 1712640
ttttcttcag aatcaggcag gtttacaccg tctatgctca cgccgacacg gttgccttcc 1712700
acgccgcgaa cagcaaagcc tttttgatgg cggccgctgt cgctcaagcc gacatcggtg 1712760
gaatagcgca ccaagtcttt attgtcgcgt atcatttctt gtttgatacg gttaaggttg 1712820
acgcgttcca cagccgcagg cgcattgcgc tgacctttaa cgcgcactgc ttttatctct 1712880
gccttaacgg gtgtggtttc agttgcagct tcatctgctg ccaagaccgg attgccgaaa 1712940
atactgccga ccagcgcggc gatagggagc atttgtaatg gtttcatatt atcaactcaa 1713000
gatgtatgga ttgttcatcc atcggttagc gaataataga ttttatgata atcattaata 1713060
tttaataaga cagtaatcca tgtaaacaaa gccgcgccgt gtaattaaag gtccctgcaa 1713120
acagctatgc cgagaccttg tttatttggt tcgattcttg tttatttggt tcgattattt 1713180
tttagtgcct gtgcggcatc attccttccg gtgcttcggc atcggctgcc aagccgaagg 1713240
tttcgcgcaa cacgactttg tagaatgcaa aggcttcgcg cgcgccttgg atggcttccg 1713300

cttcggcttc gggagtcagg ttcaaagcgt tcagatgctc gacgaaagcg cgccagtgtt 1713360
tgccgcgccc gtcgggatgg ggtgcgaggt ggcgcgcgcc gtgttcgccg ttgtaatcga 1713420
gtttttgggc gtgtttgaac aaaaatgccg cgcccaaatt ggatccttcg gcgcaataaa 1713480
gccagccgat tgctttgttg ccggtttcat gcggcagcgg tttgccgtat tcgtaaggtt 1713540
tgtcacccaa atctgcaagg tcttgcgtta cggcatcgta tcgcgccatg tattccagct 1713600
cgggaatggc tttgtttaat tcggcatctt tatagatgtg gtcgacagcc ttgtggaaaa 1713660
cggattggag tttcaaaaat ttgatgtagt tttctttgct gacaaacggt tggacagaca 1713720
taacgaggtt atccacgctg tcgtgaaccg ccgtggtatc cgccttcaag cgtttggcaa 1713780
atgtcaatgc ttgattttcg gtttcactca tattttttcc tttgtcggta agggataagg 1713840
atgcggtgcg ggcaaaatcc gcacttgccc gcatatatag tggattaaca aaaaccagta 1713900
cggcgttgcc tcgccttagc tcaaagagaa cgattctcta aggtgctgaa gcaccaagtg 1713960
agtcggttcc gtactatttg tactgtctgc ggcttcgccg ccttgtcctg atttttgtta 1714020
atccactata aaaataataa agaatcataa acgaaattta ttatcacata tttttggaaa 1714080
aaatatcatt tgcgtgatgt ttttaagcag gtattttact attctttaca gaatcgggat 1714140
tttatcaaat gggttcggca gtcggcggac aaccgctcaa aaaatatttt tgccggacac 1714200
caagggtttg ttcatactgc cgaacctgcc ggttttgcat cctgattggg tgtatcgcct 1714260
tttttccttt ataatgccgc cacttatatt tgccactttc ccgatgaagc cgtttgccga 1714320
aaatatcccc cacagccttc gcggcaactg ctgcgacgaa gccctgccgc cgcatacggt 1714380
agattgtccg gaatgcggct gccgcgcgga tgtaccccgg ttggacagtg gagaagcggc 1714440
gttctgtccc cgttgcggac acaaactctt cagggtgggc aggcatcctt tttccgcccc 1714500
gcccgcctat gcggcggctt cgctgatttt aatggcgttt gcttacggta tgacgtatat 1714560
cgaggtcggg ataccgggtg cggcatccgt cctttcgctg cccgagatga tgcgcctgat 1714620
ggtgtttcag gattatggtt ttttggccga agtgatgttt gtgctgactt tcggcgcgcc 1714680
ggttctgttt ctgctgctgt gcctgtatgt ctatgccgcg ctgatacgga aacaggcgta 1714740
tcctgcgctg cgtttggcaa cgcgtgtgat ggtgcgcttg agacaggcga tgatggtgga 1714800
tgtgtttttt gtttccactt tggtggcgta tatcaagctc tcgtctgtgg cagaggttcg 1714860
cttcgggccg gcgttttatc tgatgttcgc gctgtcagtt atgctgattc ggacttcggt 1714920
atcggttccc cagcattggg tgtattttca aatcgggcgg ctgacggggg ataatgcggt 1714980
tcagacggca tcggaaggta aaacctgttg cagccgctgc ctgtatttcc gcgacagtgc 1715040
cgaatccccc tgcggcgtgt gcggtgcgga actgtaccgc cgacggccga aaagtctgag 1715100
tatttcgtcg gcgtttctga cggcggcggt tattttgtat ttccctgcca atatcctgcc 1715160
gattatgatt tcgtccaatc ctgccgccac ggaggtcaat accatcctta acggcatcgc 1715220
ttatatgtgg gacgagggcg acaggctgat tgcggcggtt attttcagcg cgagtatttt 1715280
ggtgccggta ctgaagattg cggcaatgtc ggttttgatt gcgtccgccc gcttcgcttt 1715340
gccaacgggt gcaaagaaat tgtcgcacct ctaccgcatc accgaagcgg tcggccgctg 1715400
gtcgatgatt gatatttttg tgattattat tttgatgtgt tcgttccaca cttatgccgc 1715460
gcgcgtcatt ccgggcagtg cggcagtcta tttctgcctg gtcgtgattc tgacgatgct 1715520
gtccgcctat tatttcgacc cgcgcctgct ttgggacaaa cgcgcttcag acggcattgc 1715580
tttcaatgaa acggaaaaac atgactgaca acagccctcc tccaaacgga cacgcccaag 1715640
cacgcgtccg caaaaacaac accttcctct ctgccgtctg gctggttccg ctgatcgcgc 1715700
tgattgccgg cggctggctt tgggttaagg aaatccgcaa caggggggcct gtggttacgc 1715760
tcttgatgga cagcgcggaa ggcattgagg tcaacaatac ggtcatcaaa gtattgagca 1715820
tcgatgtcgg acgcgttacc cgaatcaaac tgcgcgacga ccaaaaaggc gtggaagtaa 1715880
ccgcccaact caatgcggac gtatccggcc tcatccgcag cgatacccag ttttgggtgg 1715940
tcaagccgcg tatcgaccaa agcggcgtaa ccggtttggg tacgctgctt tcgggttcgt 1716000
acatcgcctt tacacccggc aaaagcgacg aggcaaaaga cgtgttccaa gtgcaggaca 1716060
ttccgcccgt taccgccatc gggcaaagcg ggctgcgctt gaatttgatt ggtaaaaacg 1716120
accgcatcct caacgtcaac agccctgttt tgtatgaaaa ttttatggtc gggcaagtcg 1716180
aaagcgcgca tttcgacccg tccgaccaaa gcgtgcatta caccatcttc atccaaagcc 1716240
ccaacgacaa actgattcat tccgccagcc gtttttggct ggaaagcggc atcaatatcg 1716300
aaaccacagg cagcggcatc aaactcaatt ccgcccctct gcctgccctg ctgtcgggcg 1716360
cgatttcatt tgattcgccg aaaaccaaaa acagtaaaaa cgtcaaaagc gaagacagct 1716420
tcacgcttta cgacagccgc agcgaagtcg ccaacctgcc tgacgaccgc tcgctgtact 1716480
acaccgcgtt tttcaaacaa tccgtgcgcg gcctgaccgt cggttcgccc gtcgagtaca 1716540
aagggctgaa tgtcggcgtg gtttccgacg ttccttattt cgaccgcaac gacagcctgc 1716600
acctgtttga aaacggctgg atacccgtac gcatccgcat tgaaccttcc cgtttggaaa 1716660
tcaatgccga cgaacaaagc aaagaacatt ggaaacaaca atttcagacg gccttaaaca 1716720
aaggcctgac cgccaccatc tccagcaaca acctgctgac cggaagcaaa atgattgagt 1716780
tgaacgatca gccttccgca tcacctaagc tgcgaccgca taccgtttat gcaggcgata 1716840
ccgttatcgc gacccagggc ggcggtttgg acgatttgca ggtcaaattg gcggatttgc 1716900
tggacaagtt cgacaaactg cctttagata agacggttgc cgaattgaac ggttcgcttg 1716960

ccgagctcaa atccacactc aaatctgcca atgccgccct aagctccatc gacaaactgg 1717020
tcggcaaacc gcagacacaa aacattccga acgaactgaa ccaaaccctg aaagagttgc 1717080
gcacaaccct gcaaggcgta tcgccgcaat cgcctatcta cggcgacgta caaaatacgc 1717140
tgcaaagttt ggacaaaact ttaaaagacg ttcaacccgt gattaatact ttgaaagaaa 1717200
aacccaacgc gctgatttc aacagcagca gcaaagaccc tatcccgaaa ggaagccgat 1717260
aatgcgcctt ttcccgattg ccgccgccct gtcgcttgcc gcctgcggta ctgtgcaaag 1717320
cacacaatat ttcgtgttgc ccgacagccg ctacatccgt cctgcaacgc aaggcggcga 1717380
aactgccgtc gaagtccgtc ttgccgaacc gctcaaacgc ggcggactgg tctatcaaac 1717440
cgacccctac cgcctcaaca ccgcacaaaa ccacgtctgg gcagacacct tggacgatat 1717500
gctcgaagcg gcgttgagca atgcattcaa ccgtttggac agcacacgca tctttgttcc 1717560
tgcctcacgc agcggcagta ccgaaaaatg gacggtctat atcgacgcat tccaaggcag 1717620
ctacacgggc aaaaccctca tcagcggcta cgccgtccta cccgacggta cgaacagacc 1717680
cttccatatc gaaaccgaac agcagggtga cggctacgcc gcgatgaccg ccgcactcga 1717740
acagggactg aaacaggcgg cgcaacagat ggtcgagtaa accgtgaact attgcgaatt 1717800
tgccgcctca cttcccgaaa acaccgataa cccgaacaaa cattaccacg acacgcaata 1717860
cggttttccg attgaggacg acaatgaatt gtttgagcgg ctggtgttgg aaatcaatca 1717920
ggcaggatta aactggacgc tgatgctgaa gaagcggcag gcgtttcaga cggcatttga 1717980
aggtttcgac atcgatacgg ttgccgcctt cgacgacacc gaccgcgaac gcctgcttgc 1718040
cgacgcgggc attgtccgca accgcctgaa aatcgatgcc gccattttca atgcacggca 1718100
aatccaagcg ttgcaacaag aatacggctc gttcaagaac tggctcgaca cgcaccatcc 1718160
gcgaagcaaa gacgaatggg ttaaactctt taaaaaacat ttcaaattcg tcggcggcga 1718220
aatcgtcggc gaatttctga tgagtaccgg ctacctcaaa ggcgcgcacg ccgaaagctg 1718280
tccggtttac cgtgaaaccc tgaaatacca cccgaaatgg ctcgatgcca tctgaaaaac 1718340
caatgaacag aagaaccttc ctcctcggcg caggcgcgtt gctgcttacc gcctgcggca 1718400
gaaaatccgc ccgaacccac gccaaaattc ccgaaggaag caccgtactt gccttgggcg 1718460
attcgcttac cttcggctac ggcgcaaacc ctggcgaatc ctaccccgcg caactgcaaa 1718520
aactgacggg ttggaatatt gtcaacggcg gcgtatcggg cgatacatct gcccaagccc 1718580
tgtcgcgcct gcccgcgctg ttggcacgca aacccaagct tgtgattgtc ggcataggcg 1718640
gcaacgactt tctgcgcaaa gttcccaagg agcagacccg cgccaatatc gcgaaaatca 1718700
tcgaaaccgt gcagaaggaa aacatccccg ccgtcctcgt cggcgtgccg cacatcacac 1718760
tgggtgcgtt gttcgggcat ttgagcgatc atccgctgta tgaggatttg tccgaggaat 1718820
acggcattcc gctgttcggc ggcgcgtggg cggaaatttt gggcgataat aatctgaaat 1718880
ccgaccaaat ccacgccaac ggcaaaggct atcggaaatt tgccgaagat ttgaatcaat 1718940
ttttgagaaa acaggggttt agataaacaa aggtttatcc gcacccaagt tgtttatata 1719000
atcatgaacc gactgggaca ccaaactgct tcgggacgca tatgccgtct gaagtgcaaa 1719060
gcctacgcca tacagccgca tgaagttgca gcggtatggc gttttttgaa aaagacggcc 1719120
tgccggttca gacggcatga ccgaccgtcc gagcctgctg tggataaagc ccggacaggc 1719180
tgaaatcatg gaatattgcg aacctgaaga agcatccgac ccgtacgcaa catacaggcg 1719240
tgccaacctg atggcgggtc tgccgctgtt tgtcgtgatt ttggttctgc tcaatattgt 1719300
ttttccgctt ccggcgcatc ccttagcttg gctggtgcct gcaggtttca tggttttggg 1719360
cggcggcttt cccttatcgc tgccgcttgt ggcgctgctt gtcctgacct gctgcattct 1719420
ggcgcattgt ccgccattat cccgtctttt gtgctaccct tgcccgaatc atccgatgtc 1719480
taaaaattct gcctgatggc agccctacaa acccgaagga gtagaaatga aactgtccga 1719540
actgttcaac cccgacgaat ttgccgcgcg gcatttgagt tttggcgacg aagcggcgtt 1719600
gcttgccgct gtcggcgaga aaagtatgga cgattttgtc ggcaacaccg tgccgcaaag 1719660
catccgtatg ccgtctgaac tcgatttgcc cgatgccctg accgaagcgg acgcgttggc 1719720
aaaattgaaa ggcattgcgt cgaaaaacat gatcaacaaa tcctatatcg gtttaggcta 1719780
ttacccgacc cgcgtgccga acgtgatttt gcgtaacgta ttggaaaatc cgggttggta 1719840
caccgcctac acgccgtatc aggcggaaat cgcgcaggtc gtttggaagc tttgttgaac 1719900
ttccaacaag tgtgtatcga tttgaccggt ttccctgtgg cgggcgcgtc tttgctggac 1719960
gaagcgaccg ccgccgccga agcgatggcg atggcgcacc gcgtgggcaa ggtaaaatcc 1720020
gagcgtttct ttgtggacga gcgcgtgtat ccgcaaactt tggacgtgat gaaaacccgt 1720080
gccaagtatt tcggcttcga gctggtggtc ggcgattttg cccaagccga cgaaggcgaa 1720140
tacttcggcg cgctgttcca atacgtcggc aaagacggcg acgtgcaaga cttgcaggac 1720200
gttatcggcc gtctgaaagc caaaggcacg attgtcgccg tttccgccga catcatgagc 1720260
ttggttttgc tgaaaccgcc tgccgaattg ggtgcggata ttgcgttggg caacacacaa 1720320
cgcttcggcg tgccgatggg cttcggcggg ccgcacgccg cttatttcgc gtttaaagac 1720380
gagttcaaac gttccgcccc gggccgcatc atcggcgtat ccaaagacgc atcgggcaaa 1720440
cctgccttgc gcatggcttt gtccacccgt gagcaacaca tccgccgcga aaaagctaca 1720500
tccaatattt gtaccgcgca ggcattgctg gcgaatttgg cgggtatgta tgccgtttac 1720560
cacggccctg aaggcgtgaa acgcattgcc aaccgcattc acgcgctggc ttctgccttt 1720620

gccgatgcgc tggtttcaga cggcctgaat gtggttcaca aagtcttttt cgatactgtt 1720680
accatcgatt ttggcagtaa agagaaagca gaccaagtgt ttgccgctgc tttggcgtcg 1720740
ggttacaacc tgcgccgcgt caacgatact caagttgcgg ctgcattcca tgaaacatcg 1720800
gcatacgaag atttggtcga tttgtaccgc gcgtttaccg gcaaggatac gtttacattt 1720860
gccgatgatg tcaaaggccg tctgaacgcc gaattgctgc gtcaggacga cattctgcaa 1720920
catcctgtgt tcaacagtta ccacaccgaa cacgaaatgt tgcgttatct gaaaaaactc 1720980
gaagaccgcg acttggcgat gaaccgcagt atgatttcat tgggcagctg tactatgaaa 1721040
ctcaacgcga ctgcggaaat gttgccgatt acttgggccg agttcaccga catccatcct 1721100
tacgctcccg aagcgcaaac cgccggctac cgcgaattgc tcgccgatat ggaaaacagc 1721160
ctgaaagcca tcaccggctt tgacgcgatt tccctgcaac caaattccgg cgcacaaggc 1721220
gaatacaccg gtatgctcgc catccgccgc tatcaggaat cccaaggcga agcgcaccgc 1721280
aacatctgtc tgattccaaa atcagcccac ggtaccaacc ccgccaccgc cgccatgctc 1721340
ggtttgaaag tcgtcgtcgt cgacaccgac gaacacggca acgtcaacat tgacgatttg 1721400
aaagccaaag ccgagcaaca ccgcgacgct ttgtctgcca tcatgattac ctatccgtcc 1721460
acccacggcg tgtacgaaga aggcatccgc gacatctgcc gcattattca cgaaaacggc 1721520
ggacaggttt acatggacgg tgcgaacctc aacgcccaaa tcggcatcat gcagcctgcc 1721580
gaagtcggtg cggatgtgtt gcacatgaac ctgcacaaaa ccttctgtat ccctcacggc 1721640
ggcggcggcc cgggcatggg tccgattggc ttgaaagccc atttggctcc gtttgccccg 1721700
ggccatacct tgaccgacac ccacaacgcg gctgccgatc aaaccgccgt ggctgccgca 1721760
gcatatggtt ctgcatccat cctgccgatt acttggatgt acctgaccat gatgggcaaa 1721820
caaggcatgg aacaggcaac gcgctgggca ttgctcaacg ccaactacgt cgccaaagcc 1721880
ttgggcgagg attatccgat tctctacacc ggcaaaaacg gccgcgtcgc gcacgaatgt 1721940
atcgtcgact tgcgtccgct caaagccgaa agcggcatta ccgaaaccga catcgccaaa 1722000
cgcctgatgg actacggctt ccacgccccg accgtctcct tccccgttgc cggcacgctg 1722060
atgatcgaac cgaccgaaag cgagagcaaa gccgaactcg accgcttcat cgccgccctg 1722120
aaacaaatca aacaggaagt gctgaaagtc gggcgcggcg aatggccgaa agacgacaac 1722180
ccactggtca acgcgccgca caccgccgca gatataaccg gcaactgggc gcatccgtat 1722240
tcccgcgaag aagccgtctt cccgttgccg ttcgtccgcg aacacaagtt ttggcccttc 1722300
gtcaaccgcg tggacgacgt gtacggcgac cgcaatctcg tgtgcagctg cccaccgatg 1722360
gaaaattatg aagactgact gttgatatct taaaaaatgc cgtctgaaac attttcagac 1722420
ggcattttca tcaacggcaa accagttgca ccaatacacg tatctcgact ataactttaa 1722480
aacaaatgag ttaaaccagt atccatacat cagctttttt atcatcctac tttttattca 1722540
tccgatcgtg caaacagatt tcaaagatga aaagcctatt cacacccttt gatgtcattt 1722600
ccacacggac aaacaaatat agtggattaa caaaaaccag tacagcgttg cctcgcctta 1722660
gctcaaagag aacgattctc taaggtgctc aagcaccaag tgaatcggtt ccgtactatt 1722720
tgtactgtct acggcttcgt cgccttgtcc tgatttttgt taattcacta taaattccca 1722780
taaaaaaacg gagcagatac ctgccccgtt tttatttaat ccgaaatttt aatctaaatt 1722840
tagaattttg caccggattg gtttgccata tagtcaacag ccgctttgac ttcgtcatcg 1722900
ctcaaacctg cattgccgcc tttggcaggc atcgcgttaa agccttcaag ggcgtgtttg 1722960
tgcaaggttt ctttgccttt tttgatacgc ggtgcccaat cgtctttttt gcctatgccg 1723020
ggaataccgg gaatcgaacc gccgtggcac acctgacagg ttgcttcgaa gactttttta 1723080
ccgtcaacgc cgaccgcagg ggctgccgca cccttgtctt ctgccttcgc ttctgccgga 1723140
gctgcactat cggcaggagc agaagctgtt cctgaagcgg cattgtcggc aggcgcagcc 1723200
tcatcaggat tcgggaaaga accgccgctt ttgttcgcca tgtaagtaat cgcccgttta 1723260
agttcctgat cggtcaggtc tgccgcaccg ccttttgcag gcatggcgtt aaagccgttc 1723320
agcgcgtgtt ggaacaaggt atcgaagcct tgcgcgatac gcggtgccca atcgccgttg 1723380
tgttccagtt tcggagcgtt cggcacattg ctgtccgccg cgtggcattg gatacagatt 1723440
ttgccgaaaa tctgttcgcc ttggcgttcg ccgacgggga tgccgtcgcc catcgtcaat 1723500
tgtccgacag gctggatacg ggtctgcgtt gctgcttccg tagtggcatc gacatcgccg 1723560
aacgagccgc tgcccgccag cttaatcagg aaataaagga ctgcaataac aataacgata 1723620
ccgctcacaa gggtaaacag tgcagagcct tgggctttgt tgtcgcggag ttgtttcatt 1723680
tggtaggcct cgccgtcagg ttaggttgtg ctgtaaatta tagtttggtg tgttaaacgc 1723740
agttaacaat attttgctgg attatactga attcacaggg tctttccaat cgctatcatt 1723800
gaaaatatga aaaaatttgc caacggtatc tgtataaaac aaataatcct ttgaaaataa 1723860
ttgtttatcc tcaagaaaac tctccttatg ccgccatacg ccgcctgccg gcgcaagata 1723920
acctttgcca atttgcagaa tttacgttaa ccttgcgttt tccgcaccca tagctcagtt 1723980
ggaagagtgt cagtttccga agctggaggt cacaggttcg atccctgttg ggtgcgccaa 1724040
ttataaagag accgtctgaa agataaatat ttttcagacg gtcttttgac ttacttcaaa 1724100
ctcttatttc aagacttccg caaatgcgcg ggcaacatag tcggtattcg acgtattcag 1724160
tccggcgacg cacatcctgc cggaatccag caggtaaacg gcaaattcgt cgcgcagcct 1724220
gcggacttgt tccacgctca atcctgtgta gccgaacatg ccgcgctgtt tgatgaaata 1724280

agtgaaatcg cgattgggga tttgcgcagt taagacatca taaagtttct gccgcatcgc 1724340
acggatgcgg tcgcgcatca tataaacctc gttttgccac aaggcgtaaa gttcggggct 1724400
gttcatcacg tcggcggcga tatacgcgcc gtgcgcgggc gggctggagt agatgcggcg 1724460
gacggtgaat ttgagctgtc cgaacaccaa atccgcttct tccttattcg ggcaaaccac 1724520
gcttaagccg ccgacgcgct cgccgtagag cgacaggttt tttgagaagg aattgctgac 1724580
gaacaagggc aattccattt ccaccgcttt gcggacggcg taggcatcgc tgtccaaatc 1724640
gccgccgaat ccttggtagg caatgtccat aaacggaatc agtttgcgcg ttttgatgat 1724700
gtgcaacact tcgtcccatt gccgttccga catatccacg ccggtcgggt tgtggcagca 1724760
gggatggagg atcaggacgc tgttttcggg cagggtgttg aaaaacgcgg tcatttcgtc 1724820
gaatttcacg ccgacagtgg cagggtcgta atatgggtaa gtgccgacct cgaaacctgc 1724880
gccttcaaaa atgccgcgat ggttgtccca agtcgggtcg ctgacgtagg cgcgcgcttc 1724940
gggaaaccag cggtgcagga agtccgcccc gactttgagc gcgcccgagc cgcccaaggt 1725000
ttgtacggta acgatgcgcc cttgcgcaag cgcgggattg tctttgccga acaataaatg 1725060
ctgcaccgcg ctgcggtaag tgtccaagcc ttccatcggc aggtagggcg acggcgcagg 1725120
cgcggcggca cgggcggttt cggctcggct cacggattcc aacacgggca ttttgccttc 1725180
gtcgtcgaaa taaatgccta tgctcaaatt gactttttcg gggcgcgggt cgtttttgaa 1725240
ggtttcgacc aaactcaaaa tcgggtcgcc aggatagtat tcgatgtgtc ggtacatagt 1725300
ccttacctct tgctttttca aaggattttc tttttcaaca atacaccact ttcgatatgg 1725360
tgcgtaaacg ggaattggtc gaacagggcg gcacgttcga ccgcatgggt ttccgccaag 1725420
gtgtccaaat tggcgcgcaa cgtttcggga ttgcaggaaa tgtagatgat gttgtcaaac 1725480
tgcgacacca gcttcaaagt ttcctcatcg ataccggcac gcggcggatc gacgaaaata 1725540
gtggaaaatg cgtaatccgt caaagcaata ccgccatcct taaggcgttt aaactcacgt 1725600
tttccggtat aggcttcggt aaattcttca gcagacagac gggcgatttt gatgttgccg 1725660
atgcggttgg cttcgatatt ccattgcgcc gcgctgacgg aggttttgga gatttcggtt 1725720
gccaaaacct gtcggaaata tcgggacagc ggcagggtga aattgccgtt tccgcaatac 1725780
agttcgagca ggtcgctgcc caagccttcc gccgtgcggc acgcccattc aagcattttc 1725840
tgacacacgg cggcattcgg ttgggtaaaa ctgccttcaa tttgccgata acggaaatcc 1725900
cggttgccga ccttcaaagt ttccgttaca tagtcctgtt ttaagactat tttctgtccc 1725960
ctgctccgcc caataacgga aatatccaac tgttgctgta acgcttgcgc cgcctgcatc 1726020
cactcagcat caagcctttt gtggtaaatc atggtaacca gcatttcccc gctgagcgtg 1726080
gacagaaatt cgacggcata ccagcgtttt ttgagttcgg gggattgcgc ggcggcggcg 1726140
atcagctcgg gcatgaggcg gttgacagcc tcggaagctg cttcaaaacg gtcgcagcgt 1726200
atcatgcttg cgccgctggc tttctgccct ttttcaaaca tggcataaaa catttccccg 1726260
ccttcgtgcc aaatacggaa ctcggcacgc atacggtaat gtttgtccgg agattcgtac 1726320
acttcccact caggaacatc caaacctgca aaaagggttt taaggtagtc ttttttacct 1726380
tgaagctgct gcgtgtagtc attcatatcg ctatcctgaa aaatcagacc ggattataag 1726440
ccgatttgcc tcgccgcacg caaactcttt gactgccgcc ccttcaatga cggacgggct 1726500
tttgtgctaa aatccgccat ctttccacac tataccgata aagggaaaaa tcatggcagg 1726560
caacactttc ggacaactct tcaccgttac caccttcggc gaaagccacg gcgcgggttt 1726620
gggctgtatc atcgacggct gcccgcccgg cttggaatta agcgaagcgg atatccaatt 1726680
tgacctcgac cgacgcaaac ccggcaccag ccgccacgtt acccaacgcc gcgaagccga 1726740
ccaagtcgaa atcctctccg gcgtattcga aggcaaaacc accggcacgc ccatcgccct 1726800
cttaatccgc aataccgacc agcgcagcaa agactacggc aacatcgcca ccagcttccg 1726860
ccccggccac gccgactata cctattggca caaatacggc acgcgcgact accgcggcgg 1726920
cggcaggagc tccgcccgtg aaaccgccgc ccgcgttgct gccggagccg ttgccaaaaa 1726980
atggttgaaa gaaaaattcg gcacggaaat caccgcctac gttacccaag tcggcgaaaa 1727040
agaaatccgg tttgaaggct gcgaacacat ttcccaaaat cctttttttg ccgccaacca 1727100
tagccaaatt gccgagctgg aaaactatat ggacagcgtg cgcaaatcct tggattccgt 1727160
cggcgcgaag ctgcatatcg aagcagccaa tgtccctgtc ggcttgggcg aacctgtttt 1727220
tgaccgcctc gatgccgaaa tcgcctacgc gatgatgggc atcaacgccg tcaaaggcgt 1727280
ggaaatcggc gcaggttttg acagcgtaac gcaacgcggc agcgaacacg gcgacgaact 1727340
gaccccgcaa ggcttcctgt ccaaccactc aggcggcatc ctcggcggca tcagcaccgg 1727400
gcaagacatc cgcgtcaata tcgccatcaa acccaccagc tccatcgcca ccccgcgccg 1727460
cagtatcgac atcaacggca accccatcga actcgccacg cacggcaggc acgacccctg 1727520
cgtcggactg cgctccgcgc cgatcgccga agccatgctc gcgttagtcc tcatcgacca 1727580
cgccctgcgc catcgcgcgc aaaatgccga cgttcaggtt aatacgcccg acattaccct 1727640
ttcaaacaaa taaaaattta gccaaaacac agactttata atagaatatc gagcattgcc 1727700
gtgcagcctt gacgcacggg tttgtttaga ggaatacaac cgaaatgaca caagaaaccg 1727760
ctttgggcgc ggcactaaaa tccgccgtcc aaactatgag caaaaagaaa cagaccgaaa 1727820
tgatcgccga ccacatctac ggcaaatacg atgtattcaa acgcttcaaa ccgttggcgc 1727880
tcggcatcga tcaggatttg attgccgctt tgccgcaata cgatgccgca ctgattgcac 1727940

gcgtcctcgc caaccactgc cgccgtccgc gctatctgaa agccttggcg cgcggaggca 1728000
aacgtttcga tttgaacaac cgtttcaaag gcgaagttac ccccgaagaa caggcgatcg 1728060
cgcaaaacca tccttttgtg cagcaggctt tgcaacagca gtccgcccaa gctgccgccg 1728120
aaacgctgtc tgttgaagcc gaagcagccg aatcttccgc agcagaataa atccccaaac 1728180
gaaatgccgt ctgaaaaccg atttggtttc agacggcatt ttttcgtatg cggcaatcac 1728240
ggttcaaata tccaattccg ccgtatcgcc ttcgcgttcc atccaagcgc ggcgggcggc 1728300
ggcttcgcct ttgcccatca gtttgacgaa gatgtcgcgc gtctcgtcat ctgcaccttc 1728360
tgggatttgt acctgcaaca ggcggcgggt gtcggggtgc atggtagtat ctttgagctg 1728420
gtcggggttc atctcgccca agcctttgaa acggctgatg gaataggcgg tttctttaac 1728480
gccttctttt tgcagccgct ccaaaatgct gtcgagttcg ttttggtcga gggcgtagaa 1728540
tttgcgggca ggtttgctct taccttgtgc gttgacatcg acgcggaaca gtggcggctg 1728600
ggcgacgtag atgtgtccgt cggcaaccag tttcgggaag tggcggtaga acagggtcag 1728660
cagcaaaact tgaatatgcg agccgtccac gtcggcatcg gacaggatgg cgattttgcc 1728720
gtagcgcagg ccgcttaaat cgggatggtc gttaataccg tgcggatcga cgccgatggc 1728780
gacggaaatg tcgtggattt cggcgttgcc gaagagttgg tcggggtgga cttcaaagct 1728840
gttgagcact ttgccgcgca ggggcaggat ggcttgggtg gctttgtcgc gggcgagttt 1728900
ggctgagccg ccggcggaat cgccttcgac gaggaagagt tcgttttcgc ggatgtcttc 1728960
gctttcgcag tcggtcagct taccgggcag gacggcgacg ccgctgcctt ttttcttttc 1729020
aatcttttta accgaacgca tccgcgcctg tgcttggcgg atggcgagtt cggcgatttt 1729080
tttgccgaag tccacgtttt ggttcagcca caattccaaa gggtcgcccg atacggtggc 1729140
gacgagtttc agcgcgtcgc ggttggtcag cttgtctttg gtttgacctt ggaactgcgg 1729200
gtcgaggacg cgggcagaga gaacgaaggc ggtttttccg aacacgtcgt cgctttgcac 1729260
tttaacgccg cgcggcaaga ggttgtgcag attgatgaag ttgttgactg cgttgaacac 1729320
ggcttgtttc aagcctgctt cgtgcgtgcc gcccagcggg gtggggatga ggttgacgta 1729380
gctttcgttg gcgcacgagc cttcttccag ccaagtcagg gcaaacgccg ctccttcgcc 1729440
gatgctgaaa tcgccgttgt gttcgtctga aatgtagttt tcgcaagaga acagcggtac 1729500
ggcttcctgc gcgtcggcaa tcaggtcggt cagatagctt ttcaggccgt cggggtaatg 1729560
ccaggtttgg gtgtgcgctt cgtcttcgcc tttgaccgga cgggtcaggg aaacgcgcac 1729620
acccggcagc agcacggctt tggcacgcag caggcgttcg agttcgggaa tgctgtaatt 1729680
cgggctttca aaatatttgc cgtccggcca gacgcgcact tctgtaccgc tgtctttgac 1729740
ggcgcatttg cccacttgtg ccaacggttc gaccacgtcg ccgccggcaa acacgatgcg 1729800
gtggattttg ccttcgcgtt tgaccgttac ttcaaggcgg gtggaaaggg cgttggtgac 1729860
ggatacgccc acgccgtgca ggccgcctga aaaggcatac gcgctgcctc cgtctttttt 1729920
gttgaacttg ccgcctgcgt gcagacgggt gaatacgagt tcgactacgg atacgccttc 1729980
ttcgggatgc aggccgacgg gaatgccgcg cccattgtcg tgcacggaaa gcgaaccgtc 1730040
ttcatgaatt tgcacgtcga ttgcagtcgc gaaaccgccc aacgcttcat ccgacgcgtt 1730100
gtcgatgact tcttggcaga tatgggtcgg gctgtcggtg cgggtgtaca taccgggacg 1730160
ttctttgacc ggctccaagc ctttgaggac ggtgatgctg gattcgctgt attggttgtt 1730220
tttagccatg ggaataatct gaaagtaaga aaaacaacgc tttcagacgg cctgaaagcg 1730280
ttgcgttccg ttgttttagc ggttgtcgga agattggcgg gcggcaaagt cttcataact 1730340
ttccataccg cgcaggaagc gggaagagag ttctttcaac gcccccaaat aaacgtcgcg 1730400
tttgaaatca atcacttcgt caacgggtgc ccaatattga tgccaacgcc agccgtcaaa 1730460
ttcggggtgg cgggtggcgc gcaggttgac atcgcaatct cggccggtca ggcgcaggag 1730520
ataccaaatc tgcttctgtc cgcgataaga gccgcgccat tcgcggcgta cccagttgtt 1730580
cggcacgtca taacgcagcc agtcgcgcgt gcggccgata attttgacgt gttgcggcaa 1730640
aagcccgact tcttcgtaca actcgcggta catggcggtt tcggggcttt cgcccggctt 1730700
gatgccgcct tgcggaaact gccaagaatg ttcgcgcacg cgcttacccc aaaagacttc 1730760
gttgcggttg ttgattaaga tgataccgac attggggcga tagccttccc tgtccaacac 1730820
ggtgtcgccc tccgttaaat tcaatcttgg gattttccca caaatcaggc ggttttgaca 1730880
aatcagacgg catggcggta cgcgtgccga aacacggggg gatttgggaa aatatcttaa 1730940
atttggttta caataatgta tttcaaatta ttcgggaatc agaccatgtt agatatccaa 1731000
ttgctccgca gcaacaccgc cgccgttgcc gaacggcttg cacggcgcgg ttatgacttt 1731060
gataccgcac gttttgacac actggaagaa cgacgcaagt ccgttcaggt gaaaaccgaa 1731120
gaattacagg cctcgcgcaa cagcatttcc aaacaaatcg gcgcactgaa aggtcagggc 1731180
aaacacgaag aagcgcaggc ggccatgaat caggttgccc aaatcaaaac cgatttggaa 1731240
caggctgccg ccgatttgga tgccgttcaa aaagaattgg acgcatggtt gttgagcata 1731300
cctaacctgc cgcacgaaag cgtacctgcc ggtaaagacg aaaccgaaaa cgtcgaagtc 1731360
cgcaaagtcg gcaccccgcg cgaatttgac tttgaaatca aagaccatgt cgatttgggc 1731420
gaacctttgg gtttggattt tgaaggcggt gcaaaactct ccggcgcacg atttaccgtg 1731480
atgcgcggac aaatcgcccg tctgcaccgc gccttggcac agttcatgct ggatacgcac 1731540
acgctgcaac acggctacac cgagcattac acgccttata tcgttgacga tacgacgctg 1731600

caaggtacgg gccaactacc aaaatttgcg gaagatctgt tccacgttac ccgtggcggc 1731660
gacgaaacca aaaccaccca atacctgatt ccgacagccg aagttaccct gaccaatacc 1731720
gttgccgaca gcattatccc gtccgaacaa ctgccgctga agctgaccgc gcattcgccc 1731780
tgtttccgca gcgaggcggg ttcgtacggc aaagacacgc gcggtctgat tcgccagcac 1731840
cagttcgaca aagtggaaat ggttcaaatc gttcatcccg aaaaatcata cgaaacgctg 1731900
gaagaaatgg tcggccatgc cgaaaacatc ctgaaggctt tggaactgcc ctaccgcgtg 1731960
attaccctgt gtaccggcga catgggcttc ggcgcggcaa aaacgtatga cttggaagtt 1732020
tgggtgccgg cgcaaaatac ctaccgcgaa atctcaagct gctccaactg cgaagatttc 1732080
caagcccgcc gcctgaaggc gcgtttcaaa gacgaaaacg gcaaaaaccg cttggtacat 1732140
actttgaacg gctccggctt ggctgtcggc agaacgctgg tcgccgtatt ggaaaaccat 1732200
caaaacgccg acggcagcat caacatccct gccgcactgc aaccgtatat gggcggtgtt 1732260
gccaagttgg aagtcaaata agtttgcagg ctgcctgaac gtcaaatgcc gtctgaaacc 1732320
tgtttcagac ggcatttcct ttaaactttt aaaacacgtc agccgtcggc acgaaccgca 1732380
ttgccgcaat cgccggtctg tccgacctcg cggatattgg acagcgtaac ttccgaaata 1732440
ttacccaacg cctcttccgt caaaaatgcc tgatggccgg taaacagcac attatgacaa 1732500
gacgacaggc ggcggaacac gtcgtcggta atcacatcgt tggatttgtc ttcaaaaaac 1732560
agctcgcgct cgttctcgta cacatccatg cccaatgcgc cgatttttccg gcgtttcaac 1732620
gcctcaatcg cggcggcact gtcaatcagc ccgccccggc tggtgttgat aatcatcacg 1732680
ccgtctttca ttttgtcgaa cgccgcttcg ttcagcatat agtggttttc cggcgtggcg 1732740
gggcaatgca gcgtgatgat gtccgaccgg gcatacagct cgtctaaatc cacatatttg 1732800
ccgccgattt tttccgcttc ggggttgcaa aacggatcgt aagccagcag gttcatgccg 1732860
aaacccttta aaatccgcat ggttgcaata ccgattttcc ccgtgccgat aacgcccgcc 1732920
gttttgccgt acatattgaa accggtcaga ccttccagcg aaaaattcgc atcgcgggta 1732980
cgctgatagg ctttgtggat acggcggttc aacgtcagca tcagaccgac cgtatgttcc 1733040
gcaaccgatt cgggcgaata ggcaggcacg cgcacgactt tcaagcccaa ctcttcagcc 1733100
gcctttaaat ccacattatt gaagccggca caacgcaacg ccacagtttt cacgccaatt 1733160
tgcgccaatt tttccaacac gggccggctg ccgtcgtcgt ttacaaaaat acagaccgct 1733220
tccgcgcctt ccgccatttt cgccgttttc gcatccagca taaaatcaaa aaactccagc 1733280
tcgaagccga aatgccggtt ggcgcgggta aaatgttcgc ggtcatagct tttcgtaccg 1733340
taaatcgcaa tcttcatcaa tatgtccagt tgtcgtctat ggttgagaaa cggcataata 1733400
cactgaattc aaacaaatca gtagaatatg gtggattaaa attgattgca tgcacggcat 1733460
ttccatttca aaacacaaaa ctcaatcgcc cattgccgcc agaagctcgg cctgatgctc 1733520
ggcaatcagg gcattggtga tttcttccaa gtcgccgtcc atcacaaaat ccagcttgtg 1733580
cagggtaagg ttgatgcggt ggtcggttac gcggccttgg ggatagttgt aggtgcggat 1733640
gcgttcgctg cggtcgccgc tgccgatgag ggatttgcgc tcggcggctt ctttggcttg 1733700
ggcttcgcgt ttttgcgcgt cgttcaggcg ggcggcgagg actttcattg cctgcgcttt 1733760
gttggcatgt tggctgcggc cgtcttggca ttcgaccacc atgccggtgg gcaggtgggt 1733820
gatgcggacg gcggagtcgg ttttgttgat gtgctgaccg cccgcgccgg atgcgcggaa 1733880
ggtgtcgatg cgcaggtcgg ctgggttcag ttcgatgtct tccagttcgt ccgcttcggg 1733940
catgacggca acggtgcagg cggaggtgtg gatgcggcct tggctttcgg tggcggggac 1734000
gcgctgcacg cggtgtccgc ccgattcaaa tttcaaacgg ctgtacgccc cgagtccgac 1734060
aatacgggcg atgacttctt tatagccgcc caattcgctt tcgttggcgg acacgatttc 1734120
aacctgccaa cggttgcgct cggcgtagcg gctgtacata cgcagcaaat cgccggcaaa 1734180
cagcgcggct tcgtcgccgc ccgttccggc gcgtatttcg atgaagatgt ttttgtcgtc 1734240
gtcggcatct ttgggcagca gcagtttttg cagttcggta tcgagttcgc cgattttggc 1734300
tttggccgct tcgatttctt cggcggcaaa gtctttcatt tcggggtcgg acaacatttc 1734360
ttcggcatcc gccaagtcgc tttgggcaag ccgatagttt tggaacactt cgacgacggg 1734420
ggtcagttcg gcgtgttcgc gcgtgagctt gcggtagttg tccatgtcgg acgtggcttc 1734480
gggctgtccg agaaggtggg taacttcttc cagtcggtcg ctgagttgtt gtagtttttc 1734540
taagatagac ggcttcataa ttcttccata acaaacgccg cctgaatgtt cagacggcat 1734600
caacactgga ttattataat aggttttccg gatattcaaa aagataatct tagatggata 1734660
acctaccgtc ccaacagggc atcggcattg cgctccgtta cctttgcaat ctcttctaca 1734720
caggttccgc ggatttccgc agcaatcttt gcaatacccg gaatattggc aggcgtatta 1734780
atctcttttt tcagcataaa cgggctatcc gtttccaata cgaaatcccc gtcgttcaag 1734840
gctttaagcg tatcgcgcac tttacgcgcg ttcggattga gcagcagcga accgatgccg 1734900
attttgaaac ccagtttcgt caacacacgc gcttcttccg cgctgccgga gaaggcgtga 1734960
acgatgccgc cttgggcaaa gcctgtctgt ttgacggcgg cggcgatgtc ggcggtggct 1735020
ttgagattat ggataatcac gcggcggcgc agggtttgcg caatttcaag ctggcggacg 1735080
aaaacttgaa tttgccgttc gcgctgctgc gacgtttggg ttttatcgta aaaatccaag 1735140
ccgatttcgc cgacccatgc ctgcggataa tgtgccaaca tcgtttccag gcggacgaaa 1735200
tcccgctcgg caatgccgtc tgaaaaccaa ggatgaatgc ccagtgcaat acggatttga 1735260

ccgtgttcgg acggcatttc cgccaaatcc gccacgtcct gccaatcctg cgggcgcgtc 1735320
gcgggaacga taaaccgctt caccccaact ttccgcgctg cggtcaggat gtgcggcagg 1735380
ttttcgcgca gggcgggatc agcgagatgg cagtgggtgt cggtgaagtt catttcgatt 1735440
tcacactaac tttagtctta ccaattcttt gtaaacatct tccttacccc agccttgcga 1735500
tacggcgagg gtcatcagcg cggtggcggt ttcgaggttg catttgccgc cgttgatgat 1735560
gcccgagttg cggaacgcgt tgccttgcgc gtaaacggcg gcggttttgc cttgtcggac 1735620
ttggctgatg ttgagcagca gtttgccctg ccgcgcgaag tctcggacgg cgcggataaa 1735680
accttcgtct gcgggcgtgt tgccgtgtcc gtagctttgc aggataagag cttgggcggg 1735740
aagctgtccg agtccgtccg caagttcttg gacggcaaag ccggggataa gcgtgcggac 1735800
agcgattttt gcctgcgggt cgggataacg gattttgagg ccgtctgaaa cggctgctgc 1735860
gtcttgggac gggaggcgaa gattgtgcca accccgggtt tcgtcccatt cggcaagcgt 1735920
gccgaaatgc ggattgtcga agcctgcagc agtttcggtg ctgactttgc tgctgccgac 1735980
ggcgggatac agtttgccgt caaacgcgat gacggtttgt ttgagcttga ggctgaaggc 1736040
ggcaacggcg gtggagaggt tgcgcggggc atcgctgttt tcggcggcgt aaggccattg 1736100
ggaaccagtc aggacaatcg gtttgcccaa accttgcaga gcgagcgcga ggagattggc 1736160
ggtgtacgcc atgctgtccg tgccgtgcag tatcaggatg ccgtcgcatg aagggagttt 1736220
gtcggcaatg atgtccagcc aatcgcgcca gtgttgcagc gtaacggagg aggaatcaat 1736280
caagggattg cagacgtgcc actcgaaatc gaggccgtct gaaaaggggg aaagggcttg 1736340
gctaaccagt gcggtatcgg ggcgcaggcc ttcgctgctt tgggtcatgc ctatggtgcc 1736400
gcctgtgtag aggacgaaga ttttttgttt catggacatc atcgggtcgt ctgaaaataa 1736460
taatacggct tatttaacta tatttcggac agactggcaa tttggcggcg cggacggttt 1736520
tcagacggcc ttcaaatgaa aaagcacccg agggctgtcg atatttgatt ttccaagtag 1736580
atttttattc acgaaatagg agagccgcaa caagcttaaa tcccttgtga ggttcccaac 1736640
acggaagata ccgctttgtg gattaaaaaa tacggaaact attgaatatc gacaacctat 1736700
ttaggtgctt gattttattg tttgctttgc gcggcttttt tggctgcctt ggcggctttg 1736760
cgttgcgccc gcttttcttt caatttgctg cggtaaaact ggatacgttg gcgtttttttc 1736820
caccaaatcc aagcgacaac ggtcgcacct atacccaaga taacaaaaat acccgattgc 1736880
aggctgtgca ttttcgccat cagccaatcg atgttgtgcg caccgtattc gcccagataa 1736940
atccaaatag ggacggaaat cagtgcggcc agtccatcca taatgataaa acgcaagtat 1737000
gaaaccttgc ggctgatacc ggctgtaaca aatacggccg ttctcaaacc gggcaggaaa 1737060
cgggcgacaa ataagaccca gttaccgtat ttgtcgaatt tttcctgaac ctgctcataa 1737120
cgtttcggcg tcatgatgcg cgcaataggt ttgaacctta ggattttctg cccccaaatt 1737180
cgtccggcgg cgaacatgat gccgtccccg accaatacgc cgagcatacc gactgcaaac 1737240
ataatatgcg gattggtata acccataccc gaaatcacgc cgcctgttac caaggtcaaa 1737300
tcctcgggaa tcggcacgcc gaaaccgcag atgaccaata caaaaaaaac agccgcataa 1737360
ccgtattcga caaaaaaggc ttctaaaaaa gcaaacatgg cggatattcc attgtcggag 1737420
ataaaaagtc agaacaaacc gaaacatttt ctacatgaag caggcattct atcaaagatt 1737480
atgccgtctg aaagcggaaa aaaggcagat tcgcgcatcc tccgcatgtt cagacggcat 1737540
ttaaacagaa aaccggccgg ttctaaaaac ggttttgcct gattttgcct aaatgccgcc 1737600
gatggcggcg gcaatgcgtt ccgccccttc gcgcgcccaa tccgcctgcc gcgcctccac 1737660
catcacgcgc acgacgggtt cggttcccga agcgcgcaac acgacacgcc ctttgccttc 1737720
gagttctttt tccacttccg ccaacacgtc tttcgaagct tcctgccatt gctgaccttt 1737780
ttggatgcgc acgttaatca tcgtttgcgg atacggctgc caatcggcgc aaacggtggc 1737840
gaggtcttgg ttcagcgttt gcagtgccgc caaaacttgc agcgcggaaa taatgccgtc 1737900
gccggtgttg tgtttgtcca tacacaaaat atggccgctg gcttcgccgc cgatgagcca 1737960
gccgcgttgg ttcagctgtt ccaacacata gcggtcgccg actttggcgc ggcagaaatc 1738020
cacgccctgc tctttcaggg cgatttccat cgccatattg gtcatgaccg tgccgaccac 1738080
gccgccgatg ttgatacctt ctcgggcgcg ggctttggca atgacgtaaa tcaggctgtc 1738140
gccgtcgtaa acctgccccgt ttttatcgac catcatcagg cggtcgccgt cgccgtctaa 1738200
ggcgatgccg tagtcggctt catgctgtaa aacggcggcc tggagtgtct tggtataagt 1738260
cgcaccgcat ttttcgttga tgttgtagcc gttgggttcg ttgccgatgc tgacgacctg 1738320
tgcgcccagt tcgtgaaaca ccttgggggc gacaccgtac cccgcgccgt tggcggtatc 1738380
gacaaccaac ttcaaacccc gaaggtcgga atggctggga aaggtggatt tgcaaaattc 1738440
gatatagcgg tcgtccgcac cgctgatgcg gcgtgcgcga ccgagacggg cggacggttg 1738500
ggttttcatt tcgccgtcga ttttggcttc gatttccaac tcgacttcat cggaaagttt 1738560
cacgccgcct tcggcgaaga atttgatgcc gttgtcggaa taggcgttgt gcgacgcgga 1738620
aatcatcacg ccggcggaca ggcgcaacgc gcgggtcaga taagccacgc cgggcgtggg 1738680
cagcggtccg gtctgtacca cattcacacc cgccgccgta aaaccggcca ccaaagcggc 1738740
ttccagcata tagccggaaa tgcgcgtgtc tttgccgatg aggacggtcg gtttctggtc 1738800
ggtgtcgtgc tgcaccaaaa cctgccccgc cgcatagccg agtttcaata cgaaatcggg 1738860
cgtaatcgga aattgcccca cttcgccgcg cacgccgtcc gtgccgaaat attttttttgc 1738920

catgtgttgc tccgagaatg tgaaccgttg tccgagatta tacagtcagt ttgtgccttg 1738980
ctgtctgcac cgttgatgcc gtctgaaacc gccccgtcct tttcagacgg catgaagtat 1739040
gtgaaccgct gtttacagat tgatgcccaa cgcttcccac accttcaacg catccgctgt 1739100
cgccttcaca tcatgcaccc gcacgatttg cgcgccgcgc gctacggaag ccaacgctgc 1739160
cgccacgctg ccgtgtacgc gttccgccgc atttgcctcg ccggtcagct cgcctatcgt 1739220
gcttttgcgc gatacgccga tgagcagcgg aaaacctgtt tccgccatca attcgggcaa 1739280
atgccgcatc agcgcgatat tgtgttgcaa gggtttgccg aagccggagc cgaagccggg 1739340
gtcgagtatg atgcgttgcg gtgcgatgcc tgccgcgata cattccgctg agcgcgcttt 1739400
caaataccgc gctacttcac cgacaacatc ttgatatttc ggattaatct gcatggtttt 1739460
gggcaaaccc tgcatgtgca tcaggcaaat gcccgtgtcc gcctgacgcg ccagcaattc 1739520
gaccgcgccc tcgtcattca acgccgccac atcattaata atatcgatgc cgccgagtgc 1739580
caacgctttt tccataatca ccgtgcggcg cgtgtccaaa ctgatgggaa cgccccaccc 1739640
cgccacttcc gccaaaacag gctcaacccg cgcccattct tcttcaggcg aaacataatc 1739700
cgcacccgac cgcgtcgatt cgccgccgat gtcgagaatg tctgcgcctt cttttagaag 1739760
ctgttcggca tgtgccaagg ctgtttgggc gttttgcgaa tacacgccgc cgtcggaaaa 1739820
agaatcgggt gtgagattca cgatgcccat gattttcggt ttgtccaaac cgatttcaaa 1739880
ccgtcctgcc tgccaaacgt gtcgtgccat ctgaactcct cccaaaataa aaaacagatt 1739940
atatgccgtc tgaaaccgtc ttgtgcgctt cagacggcac cgctattcgg gcggcagacg 1740000
gcatgttgtc cgaatgtctg ctccgccttt gaatctgccg gtatgcctgc tatccgcccg 1740060
acttttcaaa acaggttccg acgattccgc acgcgcctgc cgcctttgcc aagccgtaca 1740120
ggatttcctg cggcatatcg cggttccata atcccgtaat attcgcaatc acgggcagat 1740180
ggctgatttg gcggactttc acgatggatt cgacatccaa acggtaggga tggcctttgg 1740240
tatggttcaa tacgcccgac tcgcccagaa tcaggtggcg gttgccgcgc gagacgacat 1740300
attctgcggc attcaaccaa tcttcggcag aatggtgctt gtctttacac agcaccaagg 1740360
gaatgttcag gtttccggct tcattcaata cggcaagatc ggacatcagc ccgccgccca 1740420
aatacaggat gtccgccccc gcattcaaag ccgcttcgac atggcggacg ttgcggacgc 1740480
gcaccaatac gggtttccct gcatcatgcg ccgatgcggt ctgttccgcc aaccgtctgc 1740540
accgtccccg cccttcatcc gcacttgaag tgtcgtataa gtttgccgaa gtgaaaaacg 1740600
gatccagaaa cactgcatcc gcattgcgcc atactgacgg ttctgcggcg atacggacgg 1740660
tttccccgcc gccgaaagcc acgcctttgg cggcaacgcg gctgtcttcc gcccgatttt 1740720
cccgactgac ggttttccat gtatccaaaa tgcggacggc tttctcgacc tccggcagcg 1740780
tctgcacctc cctgacgctc aaaaccctat cgtcgccgat tgcgccgatg acagtacgct 1740840
cgtcgccgtg agaaatgtgt tctcgcagac ctctgctgcg gataaaggcg acaacgccgg 1740900
caatgtccgc ttcggcggca cgcctgctca tgacaataat catatttcct cctgacacaa 1740960
gaaacggcct acccaaaata ggatttttgc aagccgtgtt atactgtggc gtgttttaca 1741020
gattgttcgg gctatggatt tattatcggt tttccacaaa taccgtctga aatatgcggt 1741080
ggccgtgctg acgatactgc ttttggcggc agtcgggctg cacgcttccg tatatcgcac 1741140
cttcacgcct gaaaacatcc gcagccgcct acaacaaagc attgcacaca cacaccggaa 1741200
aatctcgttt gatgcggaca ttcagcgcag gctcctgccc cggccgaccg tcatcctgaa 1741260
aaacctgacc attaccgaac ccggcggcga ccagactgcc gtttccgtcc aagaaaccaa 1741320
aatcggattg agctggaaaa acctgtggtc ggatcagata cagattgaaa aatgggtggt 1741380
ttcgagtgcg gaacttgccc tgacgcgcga cgggaaaggt gtttggaaca tccaagacct 1741440
gatcgacagc caaaaacgcc aagcctcagt caaccgcatt atcgtcgaaa acagcaccgt 1741500
ccgcctcaat ttcctgcagg aacagcttat cctgaaggaa atcaacctca acctgcaatc 1741560
ccccgattcg tcggggcagc cgtttgaaag ttcgggcata ctggtttggg gaaagctgtc 1741620
cgtcccgtgg aaaagcaggg ggctgttcct ttcaaacggc atcggcccgc ccgaaatctc 1741680
accgttccat tttgaagctt ccacttcgct ggacggacac ggcattacca tttccaccac 1741740
cggcagccct tctgtccgct tcaacgccgg cggagcggat gccgccggcc tcggcctgcg 1741800
tgcagacact tccttccgca acctccacct gaccgcccaa atccccgcgc tggcactcag 1741860
gaacaacagc attaaaattg aaaccgtcaa cggcgcattt accgccggcg gcgaatatgc 1741920
ccgatgggac ggttcgttca aactcgacaa agccaacctg cactccggca tcgccaacat 1741980
cggcaacgcc gaaatctccg gcagcttcaa aacaccgcgc caccagacca acttctccct 1742040
caattcgccg ctcgtatgga cggaaaacaa agggctggac gcgccgcgcc tgtatgtatc 1742100
gacccttcag gataccgtca accgcctgcc gcaacccccgt ttcatcagcc ggctcgacgg 1742160
ttcgctgtcc gtaccgaatc tgcaaaattg gaatgccgaa ttaaacggca cattcgaccg 1742220
ccaaaccgtt gccgcgaaat tcagatacac acatgaagac gcaccgcatc tggaagccgc 1742280
cgtcgcactg caaaaattga acctgacccc ctatcttgac gacgtgcggc aacaaaacgg 1742340
caaaatattt cccgacaccc tcgccaagct gtccggcgac atcgaggcgc acctgaaaat 1742400
cggaaaagtc caacttcccg gcctgcaact ggacgatatg gaaacctacc tccacgccga 1742460
caaaggccat atcgcgctca gccgtttcaa gtcagggctt tacggcggcc ataccgaagg 1742520
cggcatcagc atcgccaaca cccgtcccgc cacttaccgc ctgcaacaga atgcaagcaa 1742580

catccaaatc caaccgctgc tgcaagacct gttcggcttc cacagcttca gcggcaacgg 1742640
cgacgcggtc atcgacctga ccgcgggcgg cgaaacccga aaagagctta tccgctcgct 1742700
tcagggcagc ctgtcgctaa atatttccaa cggtgcatgg cacggtatcg acatggacaa 1742760
tatcctgaaa aacggcattt cgggcaaaac tgccgacaat gccgcaccca gcacaccctt 1742820
ccaccgattc acgctcaaca gcgaaatttc agacggcatc agccgccaca tcgataccga 1742880
actcttctcc gacagcctct atgttaccag caacggctat accaatctgg atacgcagga 1742940
attgtctgaa gatgtcctta tccgcaacgc cgtccatccg aaaaacaaac cgattcccct 1743000
gaaaatcacc ggcacggtgg acaaaccgtc cattaccgtc gattacggca ggctgaccgg 1743060
cggcatcaat tcgcgcaaag agaaacagaa aatcctcgaa gacaccctgc tggaacaatg 1743120
gcagtggctc aaacctaaag aaccgtaaac atcctgcgta caaaaatgcc gtctgaaaca 1743180
cccccgcgct tcagacggca gaccgtaaaa cctacaaccc caattcctcc caaatcccat 1743240
caatcttagc cgtaaccgca gggtcttttt tgatgacgcg tccccattcg cggtcggttt 1743300
ctcccggcca tttgttggtc gcatccaaac ccatttgccc gccgagtccg ctgacggggc 1743360
tggcgaagtc gagataatcg atgggcgtgt tttctaccaa aacagtgtcg cgcacggggt 1743420
ccatgcgcgt ggtgaccgcc cagatgactt ctttccagtc gcgcacgttc acatcgtcat 1743480
ccaccacgat gatgaatttg gtatacataa actggcgcag gaacgaccag cagcccatca 1743540
tcacgcgctt ggcgtgtccg gcgtactgtt ttttcatgct caccaccgcc atgcggtagg 1743600
agcagccttc gggcggcagg tagaaatcgg tgatttcggg gaactgcttt tgcaaaagcg 1743660
gtacgaacac ttcgttcaac gccacgccca aaacggcggg ttcatcgggc ggtttgcccg 1743720
tgtaggtcga atggtaaatc gggttttcgc gcatggtgat gcgttcgacc gtaaacacag 1743780
ggaaataatc ctgctcgttg taatagccgg tgtggtcgcc gtacgggcct tccaacgcgg 1743840
tttcgttcgg atggatgacg ccttccaaca cgatttctgc gcgggcaggc acttgcaaat 1743900
cgttgccgat acatttcacc agctccgtcc gcgaaccgcg cagcagtccg gcaaactggt 1743960
attcgctcaa ggtatcggga acaggcgtta ccgcgcccaa aatggtggcg gggtcgcagc 1744020
cgagtacgac ggcgacggga tacggcgtat cgggattgag tttgcggaac tcctgataat 1744080
ccaacgcgcc gccgcgatgc gacagccaac gcataatcag cttgtttttg ccgatgagtt 1744140
gttggcggta aatgccgaga ttttggcgtt ttttgtgcgg cccgcgcgtg acggtcaagc 1744200
cccacgttac cagcggcgca acgtcttccg gccagcaatg ctgaatcgga agttgataca 1744260
aatcaacgtc ttcgccttcc cacacgattt cctgacacgg cgcgtttttc accacgttcg 1744320
gcgccatgct ccaaatgtct ttcagcagcg gcagtttgga aaacgcatct ttgatgcctt 1744380
tgggcggttc gggttctttc aaatacgcca gcgtctgccc aatttcacgc agcttggaca 1744440
cgctgtccgc gcccatgccc atcgccacac gttcgggcgt gccgaacagg tttgccaaca 1744500
cgggataacc gtagcgcgta ccgtcgggct taatcgggtt ttcaaacagc aacgccggcc 1744560
cttcggcacg cagcacgcgg tcggcgattt cggtcatttc caaatacggg gaaatggggt 1744620
gtgcgacgcg cttgagtttg ccctgctgct cgagcatggc gatgaagtcg cgcaggtctt 1744680
tgtatttcat attcatcctt tttgtccttt tatcctgaac aatccgattc ggataccgcc 1744740
cctatccttg cctgtgcctc ggcatattct atgccgtgat aaaagtcgcg taccagcgga 1744800
tgttcgccgc cttgatggag ttgcaacaaa ggacgttgac catcgggttg ggtaacgaca 1744860
ttgcagtgca gaccgaaggt gtcggtttca taagggggca gctggttgca gatcatgccg 1744920
aaataaacag cgttttcaag gttgtcgtaa aagcggcttt gatagtcgtt aaaactcttt 1744980
tcgctgacgg atacccacac gccatattcc agcgtttcct catgcccgat aatcggaatc 1745040
ggcagcaccg cgcggataaa gcggtcggtt tggtcgggat agcggatgat gcagaaatca 1745100
gaatcgcatt ctgcttgata agcaatgcgt tcttcttcac tgagttgatt atagggatcg 1745160
ggtgcggtaa agccgattgc gggcatttct tcgtggtttt cgccgcagga agtgcaagtg 1745220
tacataaggt ttcagacttt caaaacgagt ttgcggtaaa gccattcgcc ggcaaacagc 1745280
atccccatca atacataggc aatcacgccg gtataaaccg cccaccaatc atatcgcccc 1745340
aaccgtgcca acaaagcggc aagcgtcccg ttgattataa aaaatacgca ccaaacctgc 1745400
gttacccggc gggtatagcg cacggctttt tcaggcaggt cgggctgttg cagccgcgca 1745460
agtttttcaa tcaccgtctg cccggcaaac aggctgccgc cgaacaccgc caacatcatc 1745520
agattgacga ggacgggata ccaatacatc gaatcatgcc gcccgaacac caatactgcg 1745580
gcaaaaaaca gtgtaataaa caaagccgca taacgctgtt ggggcagttt ggcagtcagg 1745640
gcgcgcagca gccacaaccc gcacatcgcc gccgccagcc aaacaaacca gcccgcctcc 1745700
ctgccgtata tagtggatta acaaaaacca gtacagcgtt gcctcgcctt gccgtactgt 1745760
ctgcggcttc gtcgccttgt cctgattttt gttaatccac tataccacaa agcgggatag 1745820
gcaatgctta atacggtcag aaaaatatgt ccgaaaaaac cgggtttcat tttgaatccg 1745880
cacaaatgtt ttcagacggc atccgataaa aacatgccgt ctgaaaaata attagaaata 1745940
cccgattagc ccgcctgaat cttcaatacc gcctgtacca cgtcgttgac ggtgcggaca 1746000
ttgcggaaat cttcggcctg cagcttgcgg ccggtttcgc gcttgatgcg gtcaatcagg 1746060
tcgatggcat cgatgctgtc gatttccaaa tcttcgtaaa gattggtatc aggcgtaatc 1746120
cgttccggtt cgatttcaaa caactcggtc agggtatcgc gcaacaaccg gtagatttct 1746180
tgttcggtca tatgtttcat ccttatgctt ggcggctttt gacaaaggcg gcgagtgttt 1746240

tgacattggc aaaatgttcg cgcaagttct cctgctcgcc gtccaatcgg aaaccgaaat 1746300
gtttttgcac cgccaagccc aattccagcg catcgacgga atccagtccc agccctccgt 1746360
cgccgaacag cgcgtcttcg ctgccgatgt cggcggcggt tatatcctcc aaagccaaac 1746420
tgtcgataat cagttgtttg atttggtttt ccaagtcgtt catatggttt tccttgtaaa 1746480
atagtcctgc agatattcat tcagccgccg cgccgcaatc ggcaacggtt tctcggcgag 1746540
ccagtcttgg gggaggatgt cgtctccgac ggtaatttca taccgtatcc ttttcggggg 1746600
gatgcggtac cacggctgcc cctttttaaa attgggcgga ttcattttga tacatacggg 1746660
cgtaatcact tcggcatacc gcagtcccaa agaaaccgcg ccccggtgca tttttacccg 1746720
tccgtcccac cccgtcctcg ttccttcggg gaacaccagc aggctctgcc cgctgtcaaa 1746780
aaccgccttt accgtttcca gcattgcttc cgactcttcg ttcggaatat agcccgcacc 1746840
tttaatctgg ctgctcattg ccggattgtg ctgcaaatct tttttcacga tacagttcat 1746900
ttcgggcaca tggccgacaa gcagcaccac atccagcaaa gacggatggt ttgccaaaat 1746960
caactgtccc gggcggttga gtttttcaac acccttgaac gatacctcca acacgcccga 1747020
ccatttcaga taagcaacga acaaacgcca agatgttccg ataatccggc gcgccgccaa 1747080
ttggcgggcg acagaccctg aagtgccgtt caaagtataa ggcaacaaaa ccaatttcat 1747140
cataatgccg ccgacaccga aaatcacaaa tcctaaccaa gtcgcaaaaa aacggcggca 1747200
ataatccaat ttatccattc cgctgccaca gccattcacg attgcgatat acccggcggc 1747260
attctcgact gccgttcagc agaaagcgca cccattccaa accgctccaa tatgcctcgg 1747320
gcagcatacc ggcttcagac ggcatatcgt ccgaagcaga caaagtcagg ctgtaacgcg 1747380
tccctttggt cagaaccatc gccaaagcat aagcaaacgg cgcgcgagtc gccgatacgg 1747440
catatccttc cggcagcgga tcgtccgccg ccaaaaccaa aaccgacccg catccctctt 1747500
ccaacagtga tgccgcttcc gccaatgccg tttccacacc gtcggcacac acggccaatg 1747560
ccgtctgctc gctcatatcc tgacgcaata tcgaccattg ccccgccgtt gcattgtgca 1747620
ccgacaaacc gaacgaagtc ggcgacacgg tatgcgattt caacagttcc aaccacaaat 1747680
cgaaactgcg tgccatttcc ccgtcgtgcg aggcataaac taccggactg ccgggatggg 1747740
cggaggcaat gtcccaagcc gcgtcgcata ccaaacgcgc cgccttactc aaacggcggc 1747800
gctgcatagc gggcaggaac ggcaattccg gcctgacatc gggcaaaccg tcggcaaaat 1747860
ccggacattc cgcccatttt gcccactggg ccatatcgcg cattttgctg cccgaaaccc 1747920
gccaggcggc gatgtcgaag tggaaccggc aaataatcga cggcatagtt tctttcaaaa 1747980
atttacactg tgccgcattc taaccaaagc ctatccccct gacaatgccg aaattcaaac 1748040
gcatttctgc cccctttctc cgacaacgcc gcccctcgga aaaccgccag aattagcctg 1748100
aatttacatt tatcattata atgcccgtat ttgccagcct gccgccgcaa tatatggaca 1748160
cactgccaga atgcccgatt accaacaccg cctccctgct gccgcacagc gggcgtatgg 1748220
ttctgataga ccgcattacc cgatacggcg atgattttgt cgaagcaggg gcacaggtaa 1748280
gccccaatca catcctttta cttgacgaca aactgcccta cacggcattt atcgaactga 1748340
tggcacaggc tgtcggcgcg tatgccggta tccaagcccg aaaaaacgca cggtcggtcc 1748400
ggctcggctt cctgctcggc acgcgcaaac ttgaaatctt cgcccaatcc gtcccaatcg 1748460
gcacgcatct gctggcaacg gcgcatatgt ctattcagga tgccgggggt atgggcgtgt 1748520
ttgactgcga actgcgttgg acagacgcgc cggaaacttc gtccgaaaca ctcccttcag 1748580
acggcatttt ggcgcgcgcc tcactcaacg tgtacagccc cgaacaccct gccggaacaa 1748640
ccgatgccgt ctgaacaggc acacacatac ggagaacaac gatgaccgaa actgtcctga 1748700
ttaccggctc caacagggc ataggcaaag ccgtcgcatt cggtttggcg gaagacggct 1748760
ttgatatcgc tgtccactgc cgcagtcgcc gcgacgaagc cgaagccgtg gcggaagaaa 1748820
tccgcgcttt gggcagaaat gcgcgcgtgt tgcagtttga cgtgtccgac cgcgaagcct 1748880
gccgcgagat tctgaccgcc gacatcgaag caaacggcgc gtattacggc gtggtgttga 1748940
acgccggact gacgcgcgac aataccttcc ccgcgttttc agatgacgat tgggatgtgg 1749000
tgctgcggac taatttggac ggtttttaca atgtattgca tccgctggtt atgccgatga 1749060
tacgccgccg caaagccgga cggattgtgt gtatggcatc agtgtccggc ctgacgggca 1749120
accgcgggca ggtcaattac agcgcgtcaa aagcaggcat tatcggcgcg gcaaaagcct 1749180
tggcggtcga actggcgaaa cgcaaaatca ccgtcaactg tgtcgcgccg ggtctcatcg 1749240
ataccgatat tatcgatgag aacgtacctg tcgaagaaat cttaaaggct gtccccgcag 1749300
cgcttatggg gctgccggaa gaagtggcgc acgcggtgcg tttcctgatg gatgaaaaag 1749360
cggcgtacat cacgcgccag gtgattgcgg tgaacggagg tttgtgttga ataccagaag 1749420
ggtcgcagta acaggcatag gcggcattac cgccttcggc cgggattggc aaagcataca 1749480
ggcagcattc aaagccgaaa aaaacgccgt caaatatatg gattggcacg aacgtttccc 1749540
cgaattggaa gcgcaactgg gtgcgccgat tgaaaattac gcgccgccga aacattggac 1749600
gcgcaagcag ctcagaagta tggggcgcgt gtcgtacctg tgcgtcgatg cggcggagca 1749660
ggcgctggcg gatgccggtt tgctcgggga cgaaagcatt accgacggac ggatgggcgt 1749720
tgcctgcggc tcttccagcg gcagcaccaa agacatcggc gatgtgggcg aattgttgct 1749780
gaccggcacg tcgcgcaact tcagcgccaa cacctatgtg cgtatgatgc cgcacaccac 1749840
cgccgccaat atcggcatct tttcgggct gaaagggcgc atcatcccga catcgagcgc 1749900

gtgttcgtcc ggcagccaag gcataggtta tgcctacgaa gccatcaaat acggtctgac 1749960
cgatatgatg ctggcgggcg gaggcgaaga atttttcccg tccgaagtgt atgttttcga 1750020
ctcgctttat gccgccagcc gccgcaacgg cgaaccggaa aaaaccccgc gcccatacga 1750080
cgcgaaccgc gacgggctgg tcatcggcga aggcgcgggg attttcgtgc tggaagaatt 1750140
ggaacacgcc aaacggcgcg gtgcgataat ttacgccgaa ctcgtcggct acggagccaa 1750200
cagcgatgcc taccatattt ccacgccccg ccccgacgcg caaggcgcaa tccttgcctt 1750260
tcagacggca ttgcaacacg caaaccttgc acccgaagac atcggctgga ttaatctgca 1750320
cggcaccggg acgcaccaca acgacaatat ggaaagccgc gccgttgcag cggttttcgg 1750380
caacaatacg ccctgcacgt ccaccaagcc gcaaaccgga cacacgctgg gcgcggcgga 1750440
cgcaatcgaa gccgcgttcg cgtggggcat tgccgaccgg caaagcaatc ccgaaggaaa 1750500
acttccgccc cggctttggg acgggcagaa cgaccccaac ctgcccgcca tcaacctgac 1750560
cggcagcggc agccgctggg aaaccgaaaa acgcattacc gccagctcgt cgtttgcctt 1750620
cggaggaagc aactgcgtct taatcatcgg atgaaataag tttgtcaatc ccaccgctat 1750680
gctatacaat acgcgcctac tcttgacggg tctgtagctc aggggttaga gcaggggact 1750740
cataatccct tggtcgtggg ttcgaacccc accggaccca ccaattccca agcccggacg 1750800
tatgtttggg ctttttttgcc gccctgtgaa accaaaatgc tttgagaaac cttgataatg 1750860
aaaaaagtca gcgtattgat tgttgccaaa aacgaagcaa accacattca ggaatgtatt 1750920
gaaagttgcc gtttcgataa agaagttatc gttatcgacg actacagcac cgacaatact 1750980
gccgaaattg ccgagggttt gggcgcaaaa gtcttcagac ggcatttgaa tggggatttc 1751040
ggagcgcaaa aaacatttgc catcgaacag gcaggcggag aatgggtttt cctgattgat 1751100
gcagacgaac gctgcacgcc ggaactatct gatgaaatct caaaaattgt ccaaaccggc 1751160
gattatgccg cctattttgt cgaacgccgc aacctttcc ccaaccatcc cgccacacac 1751220
ggcgcgatgc gtcccgacag cgtatgccgt ctgatgccga aaaaagacag ttcggtgcaa 1751280
ggcaaagtac acgaaaccgt acaaaccccc taccccaaac gccgtctgaa gcattttatg 1751340
taccattaca cgtacgacaa ctgggaacaa tatttcaaca agttcaacaa atatacttcc 1751400
atttcagccg aaaaataccg agagcaggga aagcccgtgc gtttcgttag ggacattatc 1751460
ctccgcccga tttgggggtt tttcaaaatt tatatcctga acaaagggtt tcttgatgga 1751520
aaaatgggtt ggattatgtc cgtcaaccac agctattaca cgatgattaa atatgtcaaa 1751580
ctatattatc tgtacaaatc cggcggaaaa ttttaaatgg aaaaagaatt caggatatta 1751640
aatatcgtat cggccaagat ttggggtgga ggcgaacaat atgtctatga tgtttcaaaa 1751700
gcattggggc ttcggggctg cacaatgttt accgccgtca ataaaaataa tgaattgatg 1751760
cacaggcgat tttccgaagt ttcttccgtt ttcacaacgc gccttcacac gctcaacggg 1751820
ctgttttcgc tctacgcact tacccgcttt atccggaaaa accgcatttc ccacctgatg 1751880
atacacaccg gcaaaattgc cgccttatcc atacttttga aaaaactgac cggggtgcgc 1751940
ctgatatttg tcaaacataa tgtcgtcgcc aacaaaaccg atttttacca ccgcctgata 1752000
cagaaaaaca cagaccgctt tatttgcgtt tcccgtctgg tttacgatgt gcaaaccgcc 1752060
gacaatccct ttaaagaaaa ataccggatt gttcataacg gtatcgatac cggccgtttc 1752120
cctccctctc aagaaaaacc cgacagccgt ttttttaccg tcgcctacgc cggcaggatc 1752180
agtccagaaa aaggattgga aaacctgatt gaagcctgtg tgatactgca tcggaaatat 1752240
cctcaaatca ggctcaaatt ggcaggggac ggacatccgg attatatgtg ccgcctgaag 1752300
cgggacgtat ctgcttcagg agcagaacca tttgtttctt ttgaagggtt taccgaaaaa 1752360
cttgcttcgt tttaccgcca aagcgatgtc gtggttttgc ccagcctcgt cccggaggca 1752420
ttcggtttgt cattatgcga ggcgatgtac tgccgaacgg cggtgatttc caatactttg 1752480
ggggcgcaaa aggaaattgt cgaacatcat caatcgggga ttctgctgga caggctgaca 1752540
cctgaatctt tggcggacga aatcgaacgc ctcgtcttga accctgaaac gaaaaacgca 1752600
ctggcaacgg cagctcatca atgcgtcgcc gcccgtttta ccatcaacca taccgccgac 1752660
aaattattgg atgcaatata aactgctttc agacggcata tgccgtctga aagcctttga 1752720
tgcaacaaac cactaaatta tattcgttca ttggaaagaa acaccccgaa ttcatccttc 1752780
aaaataagaa aatcccaata tcccccgata ttacgcagcc tattggcaaa gttttgcagc 1752840
gtcttccccg gcttgtgctg ccgcgtcaag tgctttgtta caatgtatag tagactaaca 1752900
aaaaccagta cagcgttgcc tcgccttagc tcaaagagaa cgattctcta aggtgctcaa 1752960
gcaccaagtg aatcggttcc gtactatctg tactgtctgc ggcttcgtcg ccttgtcctg 1753020
atttttgtta atccactata ctaccttcac atttcttaat aaattttatg agtaaccata 1753080
cttcttggtc gtccaaaatc ggtttcgtcc ttgctgcggc aggttcggcc atcggtttgg 1753140
gcgcgatttg gaaatttcct tatacggcag gcaccaacgg cggcgcggtg tttttcctgc 1753200
tgtttttgat atttactatc ttggtcgccc tacccgttca gcttgccgaa ttttatatcg 1753260
ggcgcacggg cggtaaaaat gccgtcgatt ccttcagggt tctgcgtccg ggcacgcaat 1753320
ggctttgggt cggcgtatg ggcgttgccg cctgctttat tttgctgtcg ttttacagcg 1753380
tggtcggcgg atgggtatta aattatgtcg tccacagttt tacgggggcg gttcataccg 1753440
gcgcggactt tgaagccttg ttcggcgcga cgatttccaa tccggcaggt tcgctgtcct 1753500
atcaggcact gtttatgctg attacggttt gggtggtcaa aggcggcatt tcagacggca 1753560

ttgaaaaggc aaaccgttat ctgatgccgg ggctgtttat cctctttatt gcgctggcaa 1753620
tccgttcgct gacgctgccg ggtgcaatgg agggcgtgtc tttcctgctc aaaccgaatt 1753680
ggtcgtactt taaagccgat acgatgatta cggctttagg ccaggcgttt tttgccctga 1753740
gcatcggcgt ttccgccatg attacctacg cttcatattt gggaaaagat caggatatgt 1753800
tccgttccgg ccatacgatt atgtggatga acctcttggt ttcgctgctt gccggcctgg 1753860
tgatttttcc ggcggtgttc gccttcggtt ttgaaccgag ccaggggccg ggattgattt 1753920
ttatcgtatt gcccgcagtg tttatgaaga tgccgttcgg tacggttttg tttgcggtat 1753980
ttatgctgct ggtcgttttc gccacgctga cttcggcatt ttcgatgttg gaaacggtca 1754040
ttgcctcaac catccgccaa gacgagcgca aacgcaaaaa acacacttgg cttatcggca 1754100
cggccatttt cattatcggc atcccgtccg cgctgtcttt cggcgtatgg ggcgagttta 1754160
aggttttcgg caaaaccatt tttgatttgt gggactatgt tatttccgcc gtcattatgc 1754220
cgattggtgc tttgagtgtt tccatcttta ccgcctggat tcaggacaag cagtctgtgt 1754280
taaaagatgc cggcgcgggc agcaccgtac cacgggcagt gctgctgctg tggctgaata 1754340
ccttgcgcta ccttgccccg attgccatta ttattgtttt catcaattct ttggacatcc 1754400
tttaaaagcc atccaaacag caaaaatgcc gtctgaaagc ctttcagacg gcatttttgc 1754460
ttcgggttca gcctatttcg ttcaaagtat agtggattaa caaaaatcag gacaaggcga 1754520
cgaagccgca gacagtacaa atagtacgga accgattcac ttggtgcttc agcaccttag 1754580
agaatcgttc tctttgagct aaggcgaggc aacgccgtac tggtttttgt taatccacta 1754640
tagccttgcg cgatgccgtt caaggacaaa cccataccct tttcggcaaa acggatttca 1754700
cggtcgtcaa acgagacttt gccgaagccg acccgtttca gggcttcgtc cacgctgttt 1754760
tgaggaggcg gcgtttccgc atcgggacgg gcggcaaaat aatcggcata cagtttccac 1754820
aacgcctgca ctgtcggatc gaacgcgccg tccgtcagcg cgtgaatatc gcggcacagg 1754880
ctcaacagtt ccaaaaaatc cgccgacggc gaagtcagat aaccgtccct gttcaggcgg 1754940
ctgatcaggc tgtcttcacg gtaaaggctg aacaattttt tccaaacgcg ccacttccgc 1755000
caaaaccttg ttgaccaaat ccgccgcacg cctgtcgtcc acaccgaaca gacggagctc 1755060
cgcaccggaa cccagtgcga cacctttcca gaaaaacaca ttttcattgc gtttttcatc 1755120
cccgttgcgt ttttcatcat cggcggcaaa aggattcggc aggaaagaaa ccgccgcgcc 1755180

cgccgccgca acggcggcaa ccgtcagaaa acgcctgcgc ccgaaatgcc tgcccatacc 1755240
gcctctaaac cgacactgcc gccttgatat gcggatgagg gtcgtaacct tccaactcga 1755300
aatcttcaaa cttgaaggaa aacaaatctt tgacttcagg attgattttc atcactggca 1755360
aggcgcgcgg ttcgcgttcc aactgcaatg cggcctgctc gaaatggttg cggtacaaat 1755420
gcgcgtcgcc aaacgtatgg acaaactcgc ccgcctccaa tccgcacact tgcgccatca 1755480
tcatggtcaa caatgcgtag ctggcaatat taaacggcac accaaggaaa atatctgcac 1755540
tacgctggta aagctggcag gacagtttgc cgtcggcaac gtaaaactga aacagcgcgt 1755600
ggcagggcgg caaggccatt tcatcgacca aagccggatt ccacgccgat acaatcaggc 1755660
ggcgcgagtc gggattcttc ttgatttgtt ccagcacatt ggcgatttgg tcgatatgcc 1755720
tgccgtcggg cgcgggccag ttacgccact ggtagccgta aaccgggcct aagtcgccgt 1755780
tttcgtccgc ccactcgtcc caaatggaaa cattgttgtc ctttaggtat ttgatattgg 1755840
tatcgccttt gagaaaccaa agcagctcgt ggataatcga acgcagatgc agctttttgg 1755900
tcgtcagcag cggaaaacct ttgcccaagt caaaacgcat ctgataaccg aatacggagc 1755960
gcgtacccgt accggtgcgg tctgatttgt ccgtaccgtt gtcgaggacg tggcgcatca 1756020
agtccaaata ggctttcata gcagtctttc atcaaattaa acggcgcata ttgtaacatt 1756080
tccggataat gcccaaaaca cggatacagg caggcaggat tgttggcaat ttcagtcctt 1756140
ttccacagta aaacccggtg ggaaaacaaa attaccttga ttggaatcaa aaaatctagt 1756200
ttaattactt agaataaaat ttcaataata tttttattta cgaaattaat ttatgattat 1756260
tgattaatta tcggcaacaa acatcaaaca tcgaaaatat ggaaaaaata atgtcaacaa 1756320
tttttgccaa atcgggcttg gcatcagaaa aaaataggtt tatattccca cctacaaatt 1756380
tgttttccca ttagtacact atcaaccaaa aggagtatcc gaatgactga cctgaacacc 1756440
ctgtttgcca acctcaaaca acgcaatccc aatcaggagc cgttccatca ggcggttgaa 1756500
gaagtcttca tgagtctcga tccgtttttg gcaaaaaatc cgaaatacac ccagcaaagc 1756560
ctgctggaac gcatcgtcga acccgaacgc gtcgtgatgt tccgcgtaac ctggcaggac 1756620
gataaagggc aagtccaagt caaccggggc taccgcgtgc aaatgagttc cgccatcggt 1756680
ccttacaaag gcggcctgcg cttccatccg accgtcgatt tgggcgtatt gaaattcctc 1756740
gcttttgaac aagtgttcaa aaacgccttg accaccctgc ctatgggcgg cggcaaaggc 1756800
ggttccgact tcgaccccaa aggcaaatcc gatgccgaag taatgcgctt ctgccaagcc 1756860
tttatgaccg aactctaccg ccacatcggc gcggacaccg atgttccggc cggcgacatc 1756920
ggcgtaggcg ggcgcgaaat cggctacctg ttcggacaat acaaaaaaat ccgcaacgag 1756980
ttttcttccg tcctgaccgg caaaggtttg gaatggggcg gcagcctcat ccgtcccgaa 1757040
gcgaccggct acggctgcgt ctatttcgcc caagcgatgc tgcaaacccg caacgatagt 1757100
tttgaaggca aacgcgtcct gatttccggc tccggcaatg tggcgcaata cgccgccgaa 1757160

aaagccatcc aactgggtgc gaaagtactg accgtttccg actccaacgg cttcgtcctc 1757220
ttccccgaca gcggtatgac cgaagcgcaa ctcgccgcct tgatcgaatt gaaagaagtc 1757280
cgccgcgaac gcgttgccac ctacgccaaa gagcaaggtc tgcaatactt tgaaaaacaa 1757340
aaaccgtggg gcgtcgccgc cgaaatcgcc ctgccctgcg cgacccagaa cgaattggac 1757400
gaagaagccg ccaaaaccct gttggcaaac ggctgctacg tcgttgccga aggtgcgaat 1757460
atgccgtcga ctttgggcgc ggtcgagcaa tttatcaaag ccggcatcct ctacgccccg 1757520
ggaaaagcct ccaatgccgg cggcgtggca acttcaggtt tggaaatgag ccaaaacgcc 1757580
atccgcctgt cttggactcg tgaagaagtc gaccaacgcc tgttcggcat catgcaaagc 1757640
atccacgaat cctgtctgaa atacggcaaa gtcggcgaca cagtaaacta cgtcaatggt 1757700
gcgaacattg ccggtttcgt caaagttgcc gatgcgatgc tggcgcaagg cttctaagca 1757760
aacgccgccg ctccgcaaac aaaatgccgt ccgaaccgca aatgctgttc agacggcatt 1757820
tccttatccg cccgttcaaa tcgggtgaga ctaccgatac atctgaatat gctatgccgt 1757880
ctgaacggca ttcacaccgc ccaatcctgc acgcgcttca aatcattttg cgccaaagta 1757940
tctgcgtggc ggttacggct ctgatattcc ctgtctttca agatgctgct cgccacataa 1758000
ttcaaatgtg cctttgccgc ctccgaagcc tcgcccggcc ggcggtttga tattgcctca 1758060
tacaatacac ggtgctgcgc catcagcttc ggacgcggat cttcttcctg attcagataa 1758120
ataaggctgc tgcgcgtctg ccggtacagc attttcaaca aaccgcccga caaatggctg 1758180
aacaacaaat tgtgcgccgc atcggcaatc gtctgatgaa agctgacatc agcttcgctc 1758240
tgatgttcca aattgccgct ttcgcacgcc tcctcaaact tttcaagcca aaacccaatc 1758300
cgcttcaaat cggcatccgt gcggcgttct gccgccaatg ccgccataca gccctcgatg 1758360
tggcaactga aatcaaaaac atcctgttcc caattggaat gcttgcccaa aagctcctgc 1758420
caactttgca aaaaatcctg ctgcggcttg accgaaacat aataaccgtc tccctgcctc 1758480
gcttccaaaa cctgacgggc gaccaaaaca ttcaatgccg acctgaccga cgggcgcgaa 1758540
acgccgaact cttccgccaa aacgcgttcg ggcggaatct tgcccccttc cgcgtaaacc 1758600
ccttccgcaa tgcgctcctc caataccgac aatacctgat cgctgatttt ctgaggcctt 1758660
accagtttca tcactcctcc tttataaaga ttccctgcag aacccttccg aaatatagtg 1758720
gattaacaaa aatcaggaca aggtgacgaa gccgcagaca gtacaaatag tacagaaccg 1758780
attcacttgg tgcttcagca ccttagagaa tcgttctctt tgagctaagg cgaggcaacg 1758840
ccgtactggt ttttgttcat ccactataca tcaaacatca aattggactg accaatcagg 1758900
gcggattcta atgacacgcc gttcccgccg tcaacggcat ttacctcgca ccgcccccga 1758960
aacacaagaa aaaactacac caaactacaa tttttgttca tgcaaatatt tgttttgaca 1759020
ggatttaaac aaaagctccg attcaaatct gccgaaccgc ccaaaaatat attgacctaa 1759080
atattaaagt ttcgtaaagt aatgcaacgt tgctttaatt ggtttgacca ctattgccga 1759140
cgattagaaa aatattttcg gagatgttca attatggaaa cttgggttca aaactacacg 1759200
gcaatcggcg gcagcctgta tctgactgcc gccgccgcac tcttacccat cgtctttttc 1759260
tttgccgcgc tgaccgtcct gaagctgaaa ggctatcagg cggggcttta tacgctgctg 1759320
attgcgcttg ccgttgccgt attcggcttc gggatgccga cgggtatggc ggtttcttcc 1759380
ctgccgccgc agccgcattg acccaacgcc cctacgccac actgtatttg accgcgcatt 1759440
acaaaatcgg caaatccacc cgcatcggtt tggactttga aaacgtgttc aacaaacgct 1759500
accgccctat gcccgacatt cacgtttacg gcacgccgcg cagcctgacc gcaaccgtca 1759560
aacatatagt ggattaacaa aaatcaggac aaggcgacga agccgcagac agtacaaata 1759620
gtacggaacc gattcacttg gtgcttcagc accttagaga atcgttctct ttgagctaag 1759680
gcgagccaac gctgtactgg tttttgttaa tccactataa agaaagaaat gccgtctgaa 1759740
accttatcgt ttcagacggc cttggattcg gatttcaagt gcaacactag tgtattagtg 1759800
gttggaacag attcaagaat aaaacacttg gcgtttcgta gccaagtgtt tttcttggtc 1759860
ggtggttcaa ctcatcttga accctgcgta tctcccgatc actgatgtta cggaaatcgg 1759920
tttgtttggg gaagtattgc cggatgagtc cgttggtgtt ctcattcagc cctttctccc 1759980
aagaatggta agggcgacaa aaataagtct ccgctttcaa tgctttggtt attttggtgt 1760040
gttggtagaa ctctttgccg ttatccatgg tgatggtgtg caccctgtct ttatgtgcct 1760100
ttaatgccct aacagctgcc cgggcagtgt cttcggcttt gaggctatcc aatttgcaga 1760160
tgatggtgta gcgggtaacg cgttcgacca aggtcaataa tgcgcttttc tgtcctttgc 1760220
cgacaatggt gtcggcttcc caatcgccga tacgggattt ctggtcgacg atagcgggtc 1760280
ggttttctat gccgacacgg ttgggtactt tgcctctggt ccatgtgctg ccgtagcgtt 1760340
tgcggtaggg tttgctgcat attctgagat gttgccacaa cgtgctgccg ttgcttttgt 1760400
cttggcgaag gtagcggtaa atggtgctgt ggtggagcgt gatccggtgg tgtttgcaca 1760460
ggtaggcgca tacttgttcg ggactgagtt tgcggcggat aagggtgtcg atgtgctgaa 1760520
tcagctgcga atcgagctta tagggttgtc gcttacgctg tttgatagtc cggctttgcc 1760580
gctgggcttt ttcggcgctg tattgctgcc cttgggtgcg gtgccgtctg atttcgcggc 1760640
tgatggtgct tttgtggcgg ttcagctgtt tggcgatttc ggtgacggtg cagtggcggg 1760700
acaggtattg gatgtggtat cgttcgcctt gggtcagttg cgtgtagctc atggcaatct 1760760
ttcttgcagg aaaggccgta tgctaccgca tactggcctt tttctgttag ggaaagttgc 1760820

acttcaaatg cgaatccgcc ggcattttat tgcccgaccg gttatttgtc ggtttgggta 1760880
tcccgtttca atccgccgcc gagtgccttg tacaaatcgg caaggttttc ggcgcgggtc 1760940
agttgtgccg acaaagccgc accctccgcc gcatagctgc tgcgttccgc atcgagcaag 1761000
tcgagcgcgc cggatacgcc gtgcttgtaa cgcaggccga ccaagcgcaa cgcttcttta 1761060
gaggcgcggc tttgtttgct taaagcgtca taggctttat ccagctgctc gcgcgccgcc 1761120
aatgcgtttg ccacgtcttg aaatgcggat tggacggcgg attcataggc aacgatttgt 1761180
acctgttggc gcagcttggc tacatcaagg ttcgccttgt tcgtacccca ggtaaaaatc 1761240
ggcagggtaa tagacggcgc gaacgaccaa acgcccgtgc cgcttttgaa caacccaccc 1761300
aattcggcag aacccgtacc gacggttccg gtcaggcgga tggatgggaa aaaggcggcg 1761360
cgtgccgcac cgatattggc gtttgcctgt ttgagcgcgt gttcggcagc acggatatcg 1761420
ggacggtcga gcaatacttc ggaactcaaa ccggccggca gtttttcaac aaaaaactgc 1761480
ttgtccagcg gcaaaccggc aggcaggtct tcgggtatcg gttggttaat caaggttgcc 1761540
aaggcattgc gcgcctgttc gcggctgcgc gcggcatggg cataatcggc tttggcagat 1761600
tcgatcaggg cttcctgctg acgtagggcg acggcggaaa tcacgcctgc cttgtaacgt 1761660
aattcggaca gcttgtaggt ttcctcgcgc gttttcaaaa cacgttgcgc caaagacatc 1761720
gcttcttcgg cgtaacgttc gttgaaatag gctttggcaa cggtggcaat caggctcaaa 1761780
tgtgccgcat cgcggttggc ggtgctggcg aaatagcctt gcagtgccgc ctcgctgctg 1761840
ctgcgtacac gcccgaacag atcgagttcg taagatgccg cacccagtcc gactttgtag 1761900
ctgctgctta cattgccgcc gctcaagctg ccttggcgcg agtcgttcgc attggcggca 1761960
agcgtgggca ggaggttgtt gcgctcaatc atgtattgtt tgcggtagat ttcgctgttc 1762020
aatacggcgg tacgcaaact ggtattgcgc tcgagtgcga tgtcgatcag cttttgcagg 1762080
cgcgggtcgg caaaatagtc atgccaacct aaatcgacgg cgcggatgcc gctgtcggcg 1762140
gtatcgtttt tgaacgtttc ggcaacttcg actttgggct gctcgtattg gggaatcatg 1762200
gtgcaggcag acaatgcaaa ggctgctgca acagaagtca aggtggtttt caatgtagta 1762260
tccataaaaa agtcctgatg ccgtctgaaa acccgtgggc gttcagacgg catggttgct 1762320
taatgttggc tgtcgtccga accggtgatg cccgcttcgg cggcgtgttt tactgccatt 1762380
tcgtgttcgt gcgcggtttc tttgaagaat ttgcgcacca ccacatagaa aagcggaaca 1762440
aggaacacgg acaagagcgt gccgatgagc atccccccaga atacggttgt accgatggcg 1762500
cgctggctgg cagaacttgc accgccggca atatacaggg gaaccacgcc caaaataaag 1762560
gcgaacgagg tcatgataat cggacggaaa cgcaggcggg cggcttccaa agcggcttca 1762620
accgcgcttt tcccttgcgc ttgaaggtct ttggcaaatt cgataatcaa aatcgcattt 1762680
ttcgcactca aacccatcac ggtaacgaaa ccgacttgaa agtagatgtc gttggcgaac 1762740
gagggaacgc tgcccaacag tccttcaaac aggttgcgcc cggttacgcc cgcagccgca 1762800
ccgatcaaac ccaacggaat cacaaggatg accgccagcg gaatcgacca gctttcataa 1762860
agcgcggcaa gtaccaaaaa tacggctgca accgccaaac cgtacaaaat cagggtttgc 1762920
gagccgcctt ttgcctcttc gcgcgactgt ccgcccccact ccaggctgta accgccgccc 1762980
aattcgtcaa ccatttttttg aaccgccgcc atagcctgcc cggtggaaac gccggttgca 1763040
ggcgaagcgg acagcttcat cgaaggataa ccgttgaagc gtacgctctg ttccgtaccg 1763100
ttttcccaag aaacagtagc aatggtggaa agcggtacgg cgacgccgga tttgttcggc 1763160
acggtcaggt tcaaaatatc ggcaggctgc atacgggcat cctcgtcggc ctgcaccatc 1763220
acgcgttgca gacggccttg gttcgggaag tcgctgacat aagacgaact cagcgcgctt 1763280
gccaatgcgg tgcggatgtc ggcaaacgaa atgccttgcg ccgccgccgc ggcacggttg 1763340
atgtcgattt tcaactgcgg cgagtcttcc aaaccgccag cacggacggt gctggggtca 1763400
aacaaaccgc tggcacgcat tttctgaatc aactcgttgc gcttcgccag caatgcggta 1763460
tggccggtat tgttgcggtc ttgcaggttg atgctcagac ccgaaccgtt gcccaactcc 1763520
agaatcggag gcgggacgac ggcgatgcca aaaccgtctt taagcgtccc catcatcata 1763580
cccgtcagct tgccggcaat cgcaacggca tcgctgccgg gcgcggtacg ctcgttccaa 1763640
tctttcaata tggcaaaacc catcgccata ttctgaccgc tgcccgaaaa gctgaagccg 1763700
gaaacggtaa tgatgttttc tatttcagga atgcttttcg ccagttgggt aacttgcgcc 1763760
aaagtcgcat tggtgcgctc ttgggtcgct cctgcaggca gttgcacgct gaccatgacg 1763820
aagccttggt cttcggtcgg caggaatgaa gtcggcaggc gcataaacag gaacacgccc 1763880
acaaccgcca agccgatata gacaaccatc atgcggaaag tcttacgcag cactttggca 1763940
acccggcctt cgtaaccgtg cgtccaactg ttgaatttct tgttaaacca gccgaagaaa 1764000
cctttttttct cttcgtgatg ccctttcggg attgtcttca acattgtggc acacaaagca 1764060
ggggtaaggg tcagcgcaag gaaggcggag aatgcgattg atgacgccat cgtcagggca 1764120
aactgtttgt aaatattgcc cgtcgccccg ctgaacatcg ccaacggtac gaacacggaa 1764180
atcagaacgg cggtaatacc gatgaccgcg cccgaaatct gacccatcgc tttttttggtc 1764240
gcttctttgg gcggcaagcc ttcacccgcc ataatgcgct cgacgttttc aaccaccaca 1764300
atcgcgtcat cgaccacgat gccgatgacc aaaaccatcg caaacatggt cagtacgtta 1764360
atcgacatgc ccatataaga gatgaaggcg aaaccgccca acagcgaaat cggtacgacg 1764420
atggtcggaa tcagcgtata acggatgttt tgcaggaaga gatacattac gacaaacacc 1764480

agcaccatcg cttcgattaa agtgtgaatc actttttcaa tcgaaatttc gacgaatttg 1764540
gaagtatcgt aaggggtttt ccagctcata ccctgaggaa agtatttttc caacgtcgcc 1764600
atgcgttctt taaccgcctt tgccgtcgcc atcgcattgc cgctgttgga cagcatcacc 1764660
gccataccgg tggtatttac accgttcaga cgggttgagg aagaatagtc ttccataccc 1764720
agtccgaccc ttgccacatc cttcaggtaa acattagaac cgtcggtatt ggcgcggagg 1764780
atgacgttgc cgaattcttc tgccgtaccc aactgccctt gcgccgttac ggtagccgta 1764840
accgtctgtc cgcgaacggc gggaagcgaa ccgatagaac ccgctgaaat ctggacgttc 1764900
tgggcggaca gcgcgctgcc aacatcggca aacgacaaat tgtagttttg cagtttctta 1764960
ggatcaaccc aaatccgcat cgcgcgttgc gcgccgaaca ggcgtacctg ccccacgcct 1765020
tcgatacgct gcaactcggg aacgatatta cgctgcgcgt agtcgttcat ctcttcggtt 1765080
gactgcacat ccgacgaaag catcacaatc atcaggaaat tggaacgcgc cttggatacg 1765140
gttacgccgt attgctggac agttgccggc agcgtgctca atacttcgga aagcttgttc 1765200
tgcacttcca cctgcgccag attctcgtcg gtatcgggcg taaaggtcag gctcacgctg 1765260
ccgctgccgc tcgaatcggc ggaagtggac atataatcca aaccttccac gccgttcata 1765320
ttccgctcga tcacggaaag cacgctgtct tccattacct gcgcggacgc gcccggataa 1765380
gtggccctca gggtgatggt cggggcggcg acggacggat attgcgaaac cggcaggctt 1765440
ttgatgccga aaatacccgc cgcaataatg aaaatcgaaa taacccacgc aaaaatgggg 1765500
cggtcgataa aaaatttagc catcgatgcc ttccttattt cgcttcagaa gcaggtttgg 1765560
cttcagatgc cgtctgaacg ccggattgag gcgcggcggc ttggttttca gacgacgccc 1765620
attctttggg cgttaccttt ttcgcacccg ttataccggc gatactgatg ccttccacaa 1765680
ccaccttgtc cccgtccttc agacccgacg taacaatcca attcgtaccc tgctgttgcg 1765740
caaccgttac ctcgcggggt tccataccgc cttgggcatt cacaatcatc acggtatctt 1765800
tcgcaccgcg cgttaccgcc tgctgcggca caacaaatgc gttatccacc gccacttggt 1765860
ccatcagcac gcgcacatac agaccgggca tcaagatatt ctgatcgttc ggtacggcgg 1765920
cgcgcagggt aatctgaccg gtcgattcgt tgacggccgg atcggcaaac agcaggcggc 1765980
ctttttcagg gtaaactgtg ccgtcgtcaa atttgatgcc gaccgcaatc acaccatccg 1766040
ccgccagcag tttgccttcg gctatctgac ggcgcaattt catcacttcg gatgcagact 1766100
gggtaacgtt cacatacatc ggattggttt ggcggatggt cgccagtacg gtcgcatcgc 1766160
cagcgttcag caacgtacct tcggaaactt tggactgacc gataaagccg gaaatcggcg 1766220
cggtaatgcg cgaacggttc aggctgatgc cggcggattt gattgccgcc tgcgccgctt 1766280
taacgcctgc ctcggcagaa cgtttcgccg ttaccgcagc atcgtattcc tgccggctga 1766340
cggcttcggc ggcaaccaaa ggcttgtatc gcgccaaatc cgcatccgct ttggcaagcg 1766400
ttgcctgagc cgttgccagt tgcgcgcgcg cgctttccag acctgcttca taagtggaac 1766460
tgtcgatctg atacagcggc tgtccggcac ggacataact gccttcttgg aacaggcgtt 1766520
tttggatgat gccgccgact tgggcgcgga catcggcggt acgcagcgat tccaaacgcc 1766580
ccggcaactc gacggtcaat gcgacggttt gcggatggac ggttacgaca ccgacgacgg 1766640
gcgcaggggc ttcccgacca gcaggctgcc cgccctgcgc cgcgtctccg cctttaccgc 1766700
aagacgacag taccaatgca acggcggcag ccaacgcggc cgcacgcatc gccttaaaag 1766760
cataaaaagc cattatttat cctatctgtc tggtttgatg taaagggttt tgccaatcaa 1766820
caggcattct tatagtggat taacaaaaac cagtacggcg ttgcctcgcc ttagctcaaa 1766880
gagaacgatt ctctaaggtg ctgaagcacc aagtgaatcg gttccgtact atttgtactg 1766940
tctgcggctc gccgccttgt cctgattttt gttaatccac tatatttcag gatataaaaa 1767000
ccgcctgctt cgccaacccg atgttcaaac gggttgcgaa gcaggtttca tgggttttca 1767060
aagttgagat gtagtctcaa tttcatgggt ttcattatac atacacgatt gcatggttac 1767120
aaagtctttt ttataatccg ccctcatcaa accgacccga aacgaaaccg ccattatgag 1767180
aaaaaccaaa accgaagcct taaaaaccaa agaacacctg atgcttgccg ccttggaaac 1767240
cttttaccgc aaagggattg cgcgcacctc gctcaacgaa atcgcccaag ccgccggcgt 1767300
aacgcgcggc gcgctctatt ggcatttcaa aaataaggaa gacttgttcg acgcgctgtt 1767360
ccaacgtatc tgcgacgaca tcgaaaactg catcgcgcaa gatgccgaag atgccgaagg 1767420
agggtcttgg gcggtattcc gccacacgct gctgcacttt ttcgagcggc tgcaaagcaa 1767480
cgacatctac tacaaattcc acaacatcct gtttttaaaa tgcgaacaca cggagcaaaa 1767540
cgccgccgtt atcgccattg cccgcaagca tcaggcaatc tggcgcgaga aaattaccgc 1767600
cgttttgacc gaagcggtgg aaaatcagga tttggctgac gatttggaca aggaaacggc 1767660
agttatcttc atcaaatcaa ccttggacgg gctgatttgg cggtggttct cttcctgcga 1767720
acgtttcgat ttgggcaaaa ccgccccgcg catcatcggg ataatgatgg acaacttgga 1767780
aaaccatccc gacctgcgcc ggaaataatc aagccttggt agcaatgccg tctgaaacga 1767840
acaaaccctt tcagacggca tcaaaatgac acaaagcctt cttctaaaaa tacatattga 1767900
gacctttgca ataacatagg ttactaaaat tttatgctca atcttatttt caaaatgcaa 1767960
aacttttctg atttttccta ctttttgctc aatattagga aggttttagg caattgaaaa 1768020
ttttttggcg catttttatg cgtcaaattt cgttaacaga ctatttttgc aaaggtctca 1768080
tattcactaa attgcatttt taatttcttc tatcattgca tggacattct cttggtcaaa 1768140

atgtccgttt tcttctgaat aaacttctaa caaataatgt tcaatgaacg ttttatctgt 1768200
cgtcagcgat acatctctgg caatgtcttc atacgactca aaatcatctt catgccaggg 1768260
attatatttg tccatatttt tttgaatttc atttttcata tcatttacct tccaatattt 1768320
atttacaatt aataacaata ccattcaaaa tgtaaactgc atttttctcc agcatttttg 1768380
caaataaaaa ctgaaaatcc cgccatttcc gcgaaaacgg gaaaccgttt tttgagttcc 1768440
agtcattcct gataaggctt taacgtcaag ttttcggatt accgccttta tgagaataac 1768500
gatgtgggca ttttctgttt taatctattg cggttatata catatgcgat tattttagtt 1768560
tgcttacaaa acacttcatg ttacattcaa aaatttaatg cactcaatat atttttttaa 1768620
ggagaagcag atgagtcaaa ccgatacgca acgggacgga cgatttttac gcacagtcga 1768680
atggctgggc aatatgttgc cgcatccggt tacgcttttt attattttca ttgtgttatt 1768740
gctgattgcc tctgccgtcg gtgcgtattt cggactatcc gtccccgatc cgcgccctgt 1768800
tggtgcgaaa ggacgtgccg atgacggttt gatttacatt gtcagcctgc tcaatgccga 1768860
cggttttatc aaaatcctga cgcataccgt taaaaatttc accggtttcg cgccgttggg 1768920
aacggtgttg gtttctttat tgggcgtggg gattgcggaa aaatcgggct tgatttccgc 1768980
attaatgcgc ttattgctca caaaatcgcc acgcaaactc actactttta tggttgtttt 1769040
tacagggatt ttatctaata ccgcttctga attgggctat gtcgtcctaa tccctttgtc 1769100
cgccatcatc tttcattccc tcggccgcca tccgcttgcc ggtctggctg cggctttcgc 1769160
cggcgtttcg ggcggttatt cggccaatct gttcttaggc acaatcgatc cgctcttggc 1769220
aggcatcacc caacaggcgg cgcaaatcat ccatcccgac tacgtcgtag gccctgaagc 1769280
caactggttt tttatggtag ccagtacgtt tgtgattgct ttgattggtt attttgttac 1769340
tgaaaaaatc gtcgaaccgc aattgggccc ttatcaatca gatttgtcac aagaagaaaa 1769400
agacattcgg cattccaatg aaatcacgcc tttggaatat aaaggattaa tttgggctgg 1769460
cgtggtgttt gttgccttat ccgccctatt ggcttggagc atcgtccctg ccgacggtat 1769520
tttgcgtcat cctgaaacag gattggtttc cggttcgccg tttttaaaat cgattgttgt 1769580
ttttattttc ttgttgtttg cactgccggg cattgtttat ggccgggtaa cccgaagttt 1769640
gcgcggcgaa caggaagtcg ttaatgcgat ggccgaatcg atgagtactc tggggcttta 1769700
tttggtcatc atcttttttg ccgcacagtt tgtcgcattt tttaattgga cgaatattgg 1769760
gcaatatatt gccgttaaag ggcgacgtt cttaaaagaa gtcggcttgg gcggcagcgt 1769820
gttgtttatc ggttttattt taatttgtgc ttttatcaat ctgatgatag gctccgcctc 1769880
cgcgcaatgg gcggtaactg cgccgatttt cgtccctatg ctgatgttgg ccggctacgc 1769940
gcccgaagtc attcaagccg cttaccgcat cggtgattcc gttaccaata ttattacgcc 1770000
gatgatgagt tatttcggc tgattatggc gacggtgatc aaatacaaaa aagatgcggg 1770060
cgtgggtacg ctgatttcta tgatgttgcc gtattccgct ttcttcttga ttgcgtggat 1770120
tgccttattc tgcatttggg tatttgtttt gggcctgccc gtcggtcccg gcgcgcccac 1770180
attctatccc gcaccttaaa cacgataaac aaaatgccgt ctgaaatgct taaacgcttt 1770240
cagacggcat ttgcctttct atcccgtcag gcttctccgg cctcttcctt tttttccgct 1770300
gcggcaagcg tgtcggcaag cagacggacg aggttttcaa acagggggctg ttcgagcggg 1770360
ttttggcttg cgttgccgaa cacgagggcg acttcggtat agtctttctg ccctttgctg 1770420
actttgctgc cgtggtaggc ggtttgggct tttttcaggg cggcttccca atcttgtttt 1770480
tgggcaaacg cttcggcggt ggcaagcgag gttttggcaa aaaacggcag cgggcggttt 1770540
tgcccgatat tgaaaaacgc catccattcc gacaatagct gcaatgccct gtcttgcgcg 1770600
atttccgcca atatttcggt ttctccggat tggacgataa aggtttgccg catttcggct 1770660
tcagacggca taacggcgca aaatatcagg tgttccagca gaaaagcgat acgttgcggc 1770720
gcgttgggtt tgccgtaggc gtaaaacact tgtccgcagc ggtacagatt gcccaagctg 1770780
cctttcagga tttgcccgtc cgacggtatg gcatatgaaa gcggtggcag tttggggctg 1770840
tttaaaaccg ccgtgtcgat ttgtttggca gcagtttgga agtcctgctg ccaaagtctg 1770900
cccaactctc ccgacggcag gaggctttcc gccccgatgc gggcggcggt ttgggcaaaa 1770960
tcccgtcctt cgcaccgtgc ttcgatgtag atttcggcga tttgatcggc gtgttgcggc 1771020
tcgaagggtt cggcaggctc ccaggcttcg ccgatatggg gttcgctcca cgcaagctgc 1771080
tgctgaagcc atactttgac agggttgcgc cagaaacgga taaattcgtc ctgtccgatt 1771140
tcggcaacag gttcggcgtt ttctacgggt tgatcgaaaa agggttgcgg cggttcgggc 1771200
gtttgtccga gcgcggcggc gtagtcggta cgcgtgccga atatgccgtc tgaacgtccg 1771260
ccttcttgaa aatatcggcg cgagaaggct tgcagcggat gctgttctat caggttttgt 1771320
gcaagttggc ggctaccgat gcccgccata gcggcaacgg tatcgatgag ttcgcccaac 1771380
agggaagacg gggcaagctc ttcgtctttg cggatgtcgc gcccgatgta ggacaggtag 1771440
aggatttcac gcgcgctgat gagggcttcg aggaacaggt agcggtcgtc atcgcggcgg 1771500
gcgcggtctc ctttggcggg atgtttggca atcaggtcga atacggcggc tttggtatta 1771560
cggggaaaat ctccgtcgtt caaacccaac aggcagatga ctttgaacgg caggctccgc 1771620
atcggcacca tactgcaaaa ggtgatgccg ccgcgtaaaa agcctgcctc gctttcgctg 1771680
tcgagaaagc gtcggatatg gcggatgacg gtgtgcggcg gcaactgtcc ggaaaattgc 1771740
gccaattcgg tttccgcctg ccatttgacc cattcgtttt caaggttttg gactgacttt 1771800

tggtcatcgg cttcagcttg gaacaatgtt tcaagcaaat cccggcaacg cgccacccat 1771860
tcgccgaccg ttgcgggctg ccgccatatc cgtacaatat ccgtcagggt ttcgaggaag 1771920
gcggcaaaac gtccgaacat ggcggtttga ttcacgtcgg cataccacgc gctgacatcc 1771980
tgccacatcg gattgccgcc tttgggcagc atccagccca atatcatgcg ttctaccgcc 1772040
tgcttccagg tgaacagctg atccgtgccg ccgcgcattt ctccgtccaa accccagtgg 1772100
acgttcaaat cggcaaccat gtcgtgcaaa agcggtaaat cgtcctcagt cagtccgaaa 1772160
cggcgcaaca cgggcgcggt ttctaaaagc acaagcactt tatcgacttc aaatcggctt 1772220
tccaacaagt cgaacaggca tgacaaagca tgaaacagcg gttggcggcg gctgattttc 1772280
acgtctgaca cggaatacgg caatgcctgc gcaccgggct gcgcctgtcc gaacacggct 1772340
tcgataaaag gcgtatagga ttcgatattc ggggttaata cggcgatatc gtgcggctgc 1772400
caatcgggat gttcatgcag aattttcaac agcttgtctt tgagtatctg caattcgcgc 1772460
aaagggctgt gtgcggagac gatgcgtatc gagccgtcgc ccgtgttgac gcttcccgcc 1772520
atttcagacg gcattttcag gttttgaata tcggtttgca gggcgtgtaa aagcgtatcg 1772580
cgcccgcctt cctcaaatac cggcgtttcg ccttctattt ccatttcgtt caaaaagtcg 1772640
aaaaagtccc gcccctgctt gcccaatgag gcgagcagcg gatgccctgc ctgagttaaa 1772700
tcgggatcgc cgccaccttt gaggatttgc gccgcttcga tgacgttgcc ccagtacatc 1772760
ccgctcggat tgagtgcgaa cacgaacacg tcgcaatgtt cggacagctt gtgcaaaagt 1772820
tgcaaataca tcggcgccat cgtggaaatg ccgaacacga aataacgctc gggcagctta 1772880
tcactgctca aagattccaa cagcttttcc cacaacgcga cacggtgcgg cgcgctctgc 1772940
ctgccgtcgt cgaggtaacg ccacagtttg gactgccaga tttcgtcgtc gcccaaaccg 1773000
agccgcctgc cctgctgcca agcgtctatc cactgaggac ggtacacgag gtattggtcg 1773060
aatatgtccg caagctgtcc cgcaagctgg taatctgccg attcgccgct gcccagatag 1773120
tcttgcagca cattcctcac atcttcaaat tctgccgtat tccgaaatgc ctcgctgcgg 1773180
aacaaatcca gcagccgcca gcgcatgact tcgggcgcaa acgggctgag ttccggaata 1773240
ccgggaatca gtttttcat cagcttccac gtcaggccgg cgggcaggct gaacgacaaa 1773300
ttcgccgcca cgcccaaatc gcgggcgagg caggtattga ggtagcggcg catcccctga 1773360
ctctgcacaa taatctgttc gggctgtaaa gccgatttca gcggtttgac tttttgaatg 1773420
cgggcaaaca atgccgccag cgtttcaaga cggttggatt gatacagata aaacatgatt 1773480
tcaaacagaa gctgtggtca agtattcggg attatatagc ctttccccg tccgccttca 1773540
aacaaaatgc cgtctgaacc tttcagacgg catttggtca tttaaaccat ctcctcaaaa 1773600
caggaatccg cgacaacagc agcgtatcca acagccaaat cacggcaatg gcaagcagtg 1773660
aggtcgggaa gagcagtgcg attgccaata gcggcaatgc catcatccac caaaccggca 1773720
gcttgacttt ctgcgccggc ggaacgatgc ccaccgctcc ggtcggacgg cgtttccacc 1773780
acatcacgca gccgctgata ccgataaaaa tgacggcaag gcagaacaag acgttcgcca 1773840
acacgctcca ccagcccaga gtccccatat gcagcgcaat gcttgccgcc ataaatttgc 1773900
cgaacgggtt gtaatcgtca aaacggatgt cggcaaggat tttgccgctg tactggtcga 1773960
tatgtaccgt gcggtcggca aacgggctga tcatgtcgta actcatagaa tcctgcgaca 1774020
aagtccatac gccgtcctcg cctttgggca aattcaactg ataacgccct ttgaaaccga 1774080
tttcccgcgc aaagcggtcg acggtttcca atgtcatcgg ctcgtcaggg ttaatgccgt 1774140
ctttgcccac agtcgtccct gaaacaggca taggcgtaag ctccaaaacc cacggcactt 1774200
ccttaacctt gccgtcattc aatacctcgc cgtgggtcgg cacgactgaa acggggttcg 1774260
gttcgacacc ccatttaccg gcagggaact gactccaagc ctgtacgaac ttgccgcccc 1774320
aaatacccgc ccaagcaata cccgacaggc agaacaacag caaaatcaac gacacccaag 1774380
ttccaaacgt gccgtgcaga ttccgccacc aagaacgcgc cctgcctttt gacggcagca 1774440
gcatcgcctt gatgccgcgc cgtttcaccc accaaaggta caagccgctg acaaccataa 1774500
taatggtcag tgaagctgcc gtttccaaaa gataatcgcc tgccgcaccg agcatcatat 1774560
cgctgtggat ttcatccatc gtgtaatacc aaccctgatt gcgcggcatg gtactgacca 1774620
cttttgccgt ataaggatcg accgcgacca tcgttgcttt gccctcattg ttgacacgga 1774680
acacggcaac catatcatcg gcacgcggcg caatatactg aacgacggac gaagtttccg 1774740
gattaacggc actgcgtgcc gcttccgcct gaacagacag aggttgtacc gttgcctgcg 1774800
gcacaacatg aatccgctcg ccctccttac cggtaatatt ggcaaacagc agcataccca 1774860
aacccgtaac ggcaagcagg gtaagaaaag gcataaccag cagaccggca taaaaatgcc 1774920
accgccaaac ggtcagataa cgccggttgc tctgattgtc ggcttcagtt ttgatttgtg 1774980
tatccattaa tcgtcctttt gaaaataggg ctatcgtgat gatgcgcgat tataaacaat 1775040
aaagactaat tctttatgac taaagtcaaa attcattaca acaaataggc agtctgcgtt 1775100
taaaaccgga tgcccgttaa aacaaaaaat ccagattcaa tactgaatct ggattttcat 1775160
aaccgataat atcggaaact cagtcaagtt agaatttgcc gcctgactgg ttgaccatat 1775220
agtcaaccgc agctttaacc tcatcatcgc tcaaatcgcc gcgaccgcct tttgcgggca 1775280
tcgtattgaa accttcgatc gcgtgtttgt gcaacgtgtc cttgcctttt ttgatgcggt 1775340
cggcccaatc ggctttgatg cctacatggg gaatacccgg aatcgcattg ccatggcagg 1775400
cggcacaaac ggtttcataa acctatttgc cgtccgcttt ggcagcaggt gcagcttttt 1775460

cctcggcttt aggttctgct gcggcaggtt tggcttcgga cacggcttgt gctgcttctg 1775520
caggagcaga ggctgcgggt tctgccgccg gtgcgggagt cggtgcaggc tcggcttttt 1775580
tcaccggagc tttaccgtct ttatcggaaa gaccccatac ataagcagtc ataatatgca 1775640
gtttgtcttt atccaagaaa tgtccccaag cgggcatttg gctgctgcga ccgttggtaa 1775700
tggtttcgat aatggatttt tgcgtaccgc cccacaacca cacgtcatca gtcaggttcg 1775760
gacccaaacc ttggatacct tgtcccttat cgccgtggca agtgaaacag ttggcaggcg 1775820
gaccgctgaa caaggcttgt ccgcgcgcgg cacgttcctc atcatactga ccttcgggtt 1775880
ttgaaaggga catcacataa tgggcaacgt ctttcacgcc ttcttcgccc aaagcaggac 1775940
cccaggcagg catagtcgca acacggcctt tttcgatggt ctcgtggatt ttatcgggat 1776000
caccgcccca caaccaatcg ctatcggtca gattcggaaa acctttagag cctttagcat 1776060
cagagccgtg gcactggata caataagtgt taaacaggtt ttgggcgatt tgcttggctt 1776120
gagggtcttt tgccactttt tcaatcggca tatccgcaaa cttggcatac agtttgccgt 1776180
attgctcatc ggcttttttg acctcttttt catattggtt atggctggtc catttcagca 1776240
gacctttgta gtcgccgaca cccggataca taaccaaata accgataccg aacagccacg 1776300
tcaaaacaca cagccaaaac caccagcggg gcagcggatt gtcgtattcg gcaatgccgt 1776360
cccactcatg acccgtagtt tgtacttctt cgcccttctt cggacgtttg acaacatttt 1776420
gagacagcag cagccaagcc aaagcgataa agctcagtaa gacaataact gcaatatata 1776480
tattccagaa attactggta aattgggatg ttgtgttcat tgttttgctc cgttatcaca 1776540
atattaacgg ttttcgcttt tcttatcttg cgcatcttgg ttttcatcaa aaatgctgtt 1776600
tgcggcatta tcgtagtttt tcttattccg cctgttgaag acgatataga gtaccaacag 1776660
gaaacagata aagatccata ccgtgaagag agcacgaata ccgttaatat ccatgatgtt 1776720
accttacgtt tttcaaagcc agacccaatc cttgcagata ggcgactaca gcatccagct 1776780
cggatttgtt tgccaaagcc tcaggtgctt tcgcaatttc ctcatcactg taaggagtac 1776840
ctactttacg caaagccttc atgttggcaa cggttgcatc gacatcgact ttattgcgtg 1776900
caagccacgg gaatgccggc atattggact caggcacgac atcacgggga ttcagcaggt 1776960
ggatacggtg ccattcgtcg gaatagcgac cgcccacacg tgccaaatca ggaccggtac 1777020
gtttggaacc ccattggaac ggatggtcgt aaaccgactc tccggcaaca gagtaatgac 1777080
cgtaacgctc ggtttccgca cggaacggac gaatcatttg cgagtggcag ttgtaacagc 1777140
cctcacggat gtaaatatcg cgtccggcaa cctgcagggc attgtaaggc ttcacgcccg 1777200
gcgccggctg tgttgccgcc ttggtaaagg ccaagggcac aacttcaatc aacagaccga 1777260
cactgactac aagcagcgtg aacacaatca gaacgccgat tttttcttca gccaattgtt 1777320
gtaatttcat tttggtagcc tatctttctt agtattttag tggtgctgtg tttgggaaac 1777380
cgcagggatt tcggcatcga ctgctttacc accgatggct gtgcggtaca cgttgtacgc 1777440
cataatgcac ataccactca gatacaataa accacctgca aaacggatca cgtagtaagg 1777500
catggtgcgt tttacggatt cgacaaacga gtaggtcagc gtaccgtcat cgttcaaaga 1777560
actccacatc aaaccctgca tcacaccggc aatccacatg gcagcgatat acagaaccac 1777620
gccgatggtc gcaatccaaa aatgtgcttc taccagcttg gtgctgtgca tctgttcttt 1777680
gccgaacaga cggggaatca tgtaatagac ggaaccgatg gttacaaagc ctacccagcc 1777740
caacgcaccc gcatgaacgt gcgcgacggt ccagtccgta tagtggctca atgcattgac 1777800
cgttttaatc gacatcatcg ggccttcaaa ggtagacata ccgtagaagg acaaggatac 1777860
aatcaggaat ttaagaatcg ggtctgtacg cagtttgtcc cacgcgccgg acaaggtcat 1777920
gatgccgtta atcataccgc cccaagaggg tgcgaacaga atcaaagaca gaaccatacc 1777980
caaagattgc gtccagtcag gcagcgcagt gtagtgaaga tggtgcggac ccgcccacat 1778040
ataggtaaaa atcaacgccc agaagtgaac gacggacagg cggtaggagt aaacggggcg 1778100
ggctgcttgt ttgggtacga aatagtacat catacccaag aagccggcag tcaggaagaa 1778160
gcccacggca ttatgcccgt accaccattg aaccatagca tcaatcgcac cggaatagac 1778220
ggggtatgac ttcatcaaac cggccgggat gctgatattg ttgacgatgt gtaaaagtgc 1778280
gaccgccaaa ataaagccgc cgtagaacca gttggcaacg taaatatgtt taatcttacg 1778340
tttggcaatc gtaccgaaga atacgatggc gtaagccacc caaaccaaag taatcagaat 1778400
atcgatcggc cattccagtt cggcatattc cttaccttgg gtccaaccca tagggaagct 1778460
gacgacggcg gcaacgatta ccgcctgcca gccccaaaag gtaaatgccg gcagccaacc 1778520
gccgaaaaga cgggtattac aagtacgttg gacaacgtag tatgatgtgc cgatcaggcc 1778580
gcaaccgcca aatgcgaaaa taaccgcatt ggtgtgcagc ggacgcaggc ggccgaagtg 1778640
gaaccaaggt ccgatattag acaagtcgag ggcaggagca aaaagctggg cggcgacgat 1778700
aacgccgacc aacataccca caatccccca aactacagtc atgatggcga actggcgcac 1778760
cactttgtag ttgtaagttt gtgtgtccat gagagtctcc atgaatttat gggaataaag 1778820
atttttatcc tgccgcttcc gcagcctgtt taaggtgcaa tccgggcaag cgtaattttt 1778880
tctaaattta acatatctgc cttattacgc caagcggaat tacattcgca ccgccgacga 1778940
gccctttgct taatctgttt tttattacat ataaatcata ttgttataat aaattacaac 1779000
ccgaccgcca ttgcttttgt ttccaatttt cccttttttgt ggcactttat tgatgtaggt 1779060
taagctgcat tttaaaggta tttaatccat cccgtttaac gatatatttg atagttatga 1779120

ttcattataa aataaccccg tcccctctcg accacgagtg gcacatcctg ctgacattca 1779180
cacaagatga tgatcttcct atagaaataa gcctgccaaa ctgggttccg ggcagctatc 1779240
tgattcggga tttttcccgc cacatcactt ctatccatgc atcctgtaac ggcacgtcca 1779300
tgccgctcga acaaattgcc aaaaaccgct ggcatgccgc cgccgtacgc ggcgagtggc 1779360
aaatccgcta caccgtatat gcattcgatt tgtcggttcg aggttctttc ctgacgacag 1779420
aacgcggttt ttttgacgga tcgtgcctgt ttttgaaagt cgaaggaacg gaaacgctgc 1779480
cgcaccgctt ggaattgacg ggtattccgt ccgaatggcg tattgccaca acgctgccgg 1779540
aaacagggag gtttgtcttt caggcggcat cttatgccga attgattgac cgacctgtcg 1779600
agatgggctt gattgaattt ttagattttg aggcggcagg cattccgcac acaattgcct 1779660
taagcggcat atatcccgat ttcgaccgca acaggctggt ttcggatatc aaaaaaatct 1779720
gcgaaacaga actggcggtg ttttcctccc ctgccccgtt tcaaaaatat ttgttcctgc 1779780
tccacgtcgg cgaccatatt tacggcggtt tggaacacac cgacagcacc gccctgctcg 1779840
ccgaccgcca cagccttccg ccgtacggta tgaccgatgc cgacgatacc tacaccacat 1779900
tgctcggact tttctcccac gaatattttc acgcgtggaa cgtcaaatcc atcaaacctg 1779960
ccgcgttcgt cccttatgac ctcgacaaag aaaactatac cgaacaacta tgggcattcg 1780020
aaggtattac atcctattac gacgatttgt ttttggcacg cagccgcacc atctcgcccg 1780080
aatcttattt aaacctgctg gcacaaggca ttacgcgcgt acaacaaacc cgcggccgtt 1780140
tgaggcagac cttggcggaa tcgagtttta ccgcgtggaa caaattttac aaaccggatg 1780200
aaaacagccc caacgccatc gtcagctact accagaaagg cgcgcttgcc gcattgtgcc 1780260
ttgatctgat aatacgcaac cgaagcaacg gcagacattc tctcgatacg ttaatggaca 1780320
aactctatcg ggagtggagg gacacacact cgggtattcc ggaaaaacac tggcaaatcc 1780380
gctgtcagga aattaccggc ttggatttgg aagatttttt ccaaaaagcg ttatacagta 1780440
ccgaagattt gccgcttgcc gaatgcctgg caaccgcagg cgtgggactg accttcctgc 1780500
cgcttccccg acaacacggc ggcggatacg cagaacacat ctgccccgtc ccgtcggcag 1780560
gcgattttgg cgcacgtttc aaacaaaaca ccgaccacat cgtcctgacc catgtcttca 1780620
acggcggcag cgcggaatct gcggcactgt gcccgcaaga caaaatcatt gctttagacg 1780680
gttatgcctg caccgacttt accgcacaat gggcccgata ccacgtcaat gcaaaaatca 1780740
atatccactt cttccgtgcc ggcatattgc gtcaaaccgt cttgacggtt caggcagcgg 1780800
cagcggatac tgccatccta catatcacag accggaacct gttggacaac tggttgttcg 1780860
gttaaacttt cagacggcat tgcacacaaa atgccgtctg aaaaacaacc gcaaagtaaa 1780920
ggaaacaaaa tggccattct gaaacttgac gaacacctct atatttctcc gcaactgacc 1780980
aaagccgatg cggaacaaat cgcgcaactg ggcatcaaaa ccgtcatctg caaccgcccc 1781040
gaccgcgaag aagaatcgca acccgacttc gcccaaatca aacagtggct ggaacaagca 1781100
ggcgttactg gattccatca ccaacccgtt accgcacgcg acatccaaaa acacgatgtc 1781160
gaaaccttcc gccaactcat cggacaagcc gaatatcccg tccttgccta ttgccggacc 1781220
ggtacgcgct gctccctcct gtggggcttc cgccgggcgg cagaaggtat gccggttgac 1781280
gaaatcatcc gccgcgccca agcggcaggc gtaaatttgg aaaacttcag agagcggctg 1781340
gacaacgccc gcgtctgatt acaagccgaa acgtttaaac cacaccttca agcggcattc 1781400
caccgcaact tgaaaaagag gacggcaaac cttactgccg tcctctgtcc ttctccgttt 1781460
ttacagtggg agacctttgc aaaaatagtc tgttaacgaa atttgacgca taaaaatgcg 1781520
ccaaaaaatt ttcaattgcc taaaaccttc ctaatattga gcaaaaagta ggaaaaatca 1781580
gaaaagtttt gcattttgaa aatgagattg agcataaaat tttagtaacc tatgttattg 1781640
caaaggtctc agtgggtata gcggattaac aaaaaccagt acggcgttgc ctcgccttaa 1781700
ctcaaagaga acgattctct aaggtgctga agcaccaagt gaatcggttc cgtactattt 1781760
gtactgtcta cggcttcgtt gccttgtcct gatttttgtt aatccactat aaaaattaga 1781820
aatgcacatt ttcattattc tcgcgcaggc aggactccag acttacccat ttcagtaatg 1781880
tttgaaaata aaagaaaaat cagatgtttg tattcccgcc tgcgcagaaa tggagacggt 1781940
gctctgtcgt ctcatttttg ttttaatcaa ctatatatag ctgattaaac ataagaaatg 1782000
ccgtctgaaa gactttcaga cggcattcgt tcaagcgtcg aactttattg cgccttggtt 1782060
tcggttacaa aaccgatttt ggtgattcct gcctgacggg cggcttctaa agctttgttt 1782120
acataatcgt attccaccgc cttgtctgcc gcaatcgcca caatcacgtt ttcattctgc 1782180
tccttggcgg ctttcagacg gctttccact tccccgattt ccactttgct tgcagaatcc 1782240
ccgccgacat aatagccgcc gttcgcatca atcgtcaggc gcagggggtc tttaggctgt 1782300
ttgtcctgct tgtttgtctg ctcggacgcg gtcggcagtt ccaaagggat ggaatgcgtc 1782360
agcaccggca tagtaatcat aaacacaatc agcaacacca gcatcacgtc caccaacggc 1782420
gtaacgttga tgtcggacat cggagaatcg tcgccggaat tcatcgaacc aaatgccata 1782480
atcagctatc cttttgatta agcaggcgga cgtgcaaatc gtgcgccatc gcatccaaat 1782540
cctgggtcag tatttttgtg ccgcgattga ggaagttgta tgccaacacc gccggaatcg 1782600
ccacgaacaa acccgccgcc gtcgccacca gtgcctcgcc aatcgggccg gcaaccgccg 1782660
caatactcat ctgcccgctt tgcccgatat tgatcagggc gtggtaaatc ccccaaaccg 1782720
tgccgaacag cccgataaac ggcgcggtcg cgccgatgga ggcaagcgcg gtcatcccgt 1782780

aatcaaaccg gcgcataatc tgcgccatac tgttgcggat ttgaatgacc aaatactcgt 1782840
tcaacggcaa agcctgcgcc agttcggacg cttcgtttcg gcggtagttg cggtaagact 1782900
gcaatgcctc ttgcgccagt ttggacaaag gcgcatcgac ggcgcgcact ttttcgaccg 1782960
cgtcgttcag cgacaaagta tcgcgcatat gccgtttgac ggcggcattc cctttgcgcg 1783020
cccgatacag cttgatgcag cgcaagacaa ccaaacacca cgttacgata ctcatcaaca 1783080
gcatcaacac aaacacacca atcaggacgg gatcgcccga ttcaaacact aatttcaaat 1783140
tcataatgat tccaacactg aaaaaaccaa tcaaacatcc aagctgccgc aaaccgctgc 1783200
ggcaaccgcc taattcaatt caaacttgac ggggacttta aactccgtcc aggcattggc 1783260
ttgaaaatgc ccgttttgcg ccgccttgcg tgccgcattg tccaaccggg aaaaaccact 1783320
gcttttcacg attttaacgg actcaacatg accgcccgga gaaaccaaaa cgctcaaaac 1783380
aaccgtaccc tgctcgtcat tctccataga aagcgtggga taagccgggc gcggaatgct 1783440
gccgttggcg cgtaaaggat tgcctttgct gctgccggct ccttccccgt gttcgccttt 1783500
gacaccgccg ctacctttac cgctgccttc tccgcgcccc gttccgtctc ctttggtacc 1783560
agttccctta tcttccccat tgccctgctc gctgtctgct ttggcagaag cattgccggg 1783620
atgttcggca ggtttttcag acggcttctc gaccggtttt tccgccggtt tcgggacagg 1783680
cttcgcttcc ggcttaggct ctggtttcgg tttttcttcg ggtttcggct tttcttcagg 1783740
tttcggctct tccttaggct gctgaatatc cgcatccgcc tttttcgtaa ccaccggctt 1783800
caaaaccggc ttgggcggct cgacaggttt gggcggctcg ggcacgggtt gcggttcggg 1783860
cgcagcaggc gcgcctgcac cttcgggggc gccgtcccct ccgccaaaat cgcccaaatc 1783920
gacaaattca ataacattgc ctgactctat cacgggcagc ttgtgcgcct gccagagcaa 1783980
tgccaccatt gccaaatgca gcagtgcgac ggaaaacacg actgcggggg ttaaaattcg 1784040
ttctttatcc ataattcggg cataataata gcaacaattc ctatttgcaa cctattttta 1784100
caatttttgg tcatatgaat gtctgttccg ttcacaggca aaccgtgttt aaacgctgta 1784160
ttacagcaaa tcatcagata acgggccggc agaaaaaatg attccgtctg atttcttatt 1784220
ccaataaaat caggttagat gatatattgc cgcttctgtc tgtcagccgt ttcgggctgc 1784280
acaccacatc tgttcaaagg aaaaccatgt ttcaaaattt tgatttgggc gtgtttctgc 1784340
ttgccgtcct gcccgtgctg ctctccatta ccgtcaggga ggtggcgcgc ggctatacgg 1784400
cgcgctactg ggagagacaac actgccgaac aatacggcag gctgacactg aaccccctgc 1784460
cccatatcga tttggtcggc acaatcatcg taccgctgct tactttgatg ttcacgccct 1784520
tcctgttcgg ctgggcgcgt ccgattccta tcgattcgcg caacttccgc aacccgcgcc 1784580
ttgcctggcg ttgcgttgcc gcgtccggcc cgctgtcgaa tctagcgatg gctgttctgt 1784640
ggggcgtggt tttggtgctg actccgtatg tcggcggggc gtatcagatg ccgttggctc 1784700
aaatggcaaa ctacggtatt ctgatcaatg cgattctgtt cgcgctcaac atcatcccca 1784760
tcctgccttg ggacggcggc attttcatcg acaccttcct gtcggcgaaa tattcgcaag 1784820
cgttccgcaa aatcgaacct tatgggacgt ggattatcct actgctgatg ctgaccgggg 1784880
ttttgggtgc gtttattgca ccgattgtgc ggctggtgat tgcgtttgtg cagatgttcg 1784940
tctgactggc tttcagacgg cataaacgct ccagaaaacg cggcaggaca tattgccctg 1785000
ccgcgttttc ctgtagtgta atcttatttt tttcatcatt attagaacca ggttgcatga 1785060
taataccttt cattaactga aacactgatt aagaaactcc agtctgtcta atgatgaggt 1785120
tttcacatcg ccaaaacttg ccaatcaaat gctggattta ttgccgtctg agatttggtc 1785180
aaatccaaag gcgacattct tagaccctgt gtgtaaatca ggggtatttt tgcgtgaaat 1785240
cgtcaaacgc ttggatgaag gcttgaccaa tcaaatacca gataaacaaa ctcgcattaa 1785300
ccacatttta aaaaatcaag tttttggaag tactgccacg tatgtaggta gctttgaccg 1785360
atatttgcat aaaaactcct ttgctggtga aaggaattat tttgccaatt ttaaaatatt 1785420
tctggcacca aatagtacaa tgacaaagac aatcatgcca atgattaaat caggatagct 1785480
agaatgagtc aataacgtca atgctcccgc cgctatcaca ccgatattga tgataatgtc 1785540
attggatgta aaaatcatgc tggctttgat atggatttct ttattttgat ttttgctcag 1785600
tagatataag cacagccagt ttgcaatcaa tgccaaaaat gccgtgccaa tcatcagttg 1785660
ataattgggc agctgctcag caccgataaa acgcctaatc acttctatca ccccaaataa 1785720
cgccaatatt atctgcgtta tccccgccaa aaatgccaca cgttttttat acgccagcgt 1785780
cataccaatg gctgatagcg ccaatatata gacaaagctg tccgccagca tatctagact 1785840
atcagcaatc agccccatag aattagcaaa aataccaacc gaacactcta tgataaaaaa 1785900
cacaaagtta atcatgagca cttgatataa taatcttttt tctaagtgct catcaggctt 1785960
gttaaacact atcttatcaa caatcacttc ggtggaaatg atatgactat caaaattaag 1786020
cggttcaagt acttgtaaaa tcgttgtatc ttgattatcg tgatagacgg ttaagcaccg 1786080
cccagcaata tcaaactgta attcataaat atcagacaca tcttttaaac gcatgcgaat 1786140
gagctgttct tcggacgggc agtccatttt ggtaatgtta aaaatggtct ttttcatcta 1786200
tttagttcct tgttttgatc aggttggctc aaataaatct gtgtttatat tgctgcttgg 1786260
taatttttgg atggtttgag taaattgatt aggttaaaat ttacctttgg aagtaccgcc 1786320
acgcataata gtttagatat gtttataatc tctggataaa aaaacgtaat aagtgcttac 1786380
tggataacaa agtccaaacc aatagcaggc aaaataaggc atccaccccc cttcttcatt 1786440

aaggatatat attgagaaac aaatcgcaac taaacagaaa aaacttggga gataaagcca 1786500
tttcattccc ctattcaaga atctagccaa gataggtatt ttgtattcta caaaaaagaa 1786560
aggcatttcc aagggaaaca tgtcagataa aaacttttgt ttattttta ctatagatag 1786620
aaccttgctt ctcaagagaa agccattaat aataccgatg acagctatta atatatagag 1786680
aatagtataa gtatgaataa tcttcattag acaaaaagaa gaaatggcag ataaattaca 1786740
tacgatatat tggaatataa aatatttacg gtctaaacct tgttcagttg caattttttt 1786800
aaaattgcct tgcataaaaa aatcaaaggc gtccattaaa ctatctttca cattagaaat 1786860
ttaaaagcta aataatacga caaacaatgt gaagtactat tcatggttta tttaaaaaat 1786920
aatactattc tgaacattat ttagatacag aaattaacaa attagaacta aacaagcttt 1786980
taaatacttt aattttattg gaaagctata aaaggaacta taactttaca cactagtcac 1787040
ttctttttaa gaggcaaaag ggattgggaa ggtcgtcttg gagataagca ctggtatttc 1787100
ggccaatggt aaatagagtt tacctcaaat agggtagaac ctccttcatc tgtcagttaa 1787160
taacagccac ttttacaatg ccctgtcaaa ataaagcggc acgcccgatt tttcactcat 1787220
cgtcatcaaa taacccatca ccttttgggg ccattcgatg ccgcgcacca cggtcagatt 1787280
cctcaaaacg gggaaaacca aaatatcctc cataccgatt ccgccgttga tgccgtctga 1787340
agcaccgtcc atcaaatttt ccaactcttg caaatctgcg tttatccgtt cgaggtattg 1787400
ggcggtttta ttcaaattgg cggaaaagct gccgatgctt ttctcttttt tgtctgtaaa 1787460
atatttcacc gcttccggcg ttgcaaattc aggcagcccg attttgatca cgcgcggctg 1787520
caccagtttg tcgttgtatc cgcccacctt gtccagccac gcccgtatct cggggcggac 1787580
ttcgtctttc agacggtctt cgcggtcgaa atgccgcaca atgtccaaac tctcgcccat 1787640
aaacgaaccg tcttcttttt gcaggacggg cacttgtttc gcaccgatca taccgatcgg 1787700
cgttgcctcg tcgtcgtttg ccagcacggc ttcttcaacg tccgcgccaa acagcccggc 1787760
agccatccgc gcacgcacgc aaaacgggca atggtcgtaa atatacagtt tcatcaaaat 1787820
attcctcgtc aacctgtcgg taccgactac cttaacaccc cgcgccgccc gaaacaagtt 1787880
tatcttcccg cctatgcacc gtaaataaat aagctgttac aataaactcg tttttatcgg 1787940
aacggaagac cccatcatga ccgccatcag cccgattcaa gacacgcaaa gcgcgactct 1788000
gcaagaattg cgcgaatggt tcgacagcta ctgcgccgct ctgccggaca acgataaaaa 1788060
cctcatcggt accgcatggt tgctggcgca ggaacattac cccgccgatg ccgccacgcc 1788120
gtatggcgag ccgctgcccg accacttcct cggcgcggcg caaatggttc atgaactcga 1788180
cctgctcccc gatgccgtcg ccgccaccct gcttgccgac atcggacgct acgtccccga 1788240
ctggaaccta ttggtttccg aacgctgcaa cagtaccgtc gccgagctgg tcaaaggtgt 1788300
ggacgaagtg cagaaactca cccacttcgc ccgggtggac agcctcgcca cgccggaaga 1788360
acgcgcccag caggcagaaa ctatgcggaa aatgctgctg gcgatggtta ccgacatccg 1788420
cgtcgtgtta atcaaactgg cgatgcgtac gcgcaccctg caatttttaa gcaacgcccc 1788480
cgacagcccc gaaaaacgcg ccgtcgccaa agaaaccctc gacatcttcg ccccgctcgc 1788540
caaccgtttg ggcgtgtggc agctcaaatg gcagctcgaa gatttgggct tccgccatca 1788600
aaagcccgaa aaataccgcg aaatcgcgct gcttttggac gaaaaacgca ccgaacgcct 1788660
cgaatacatc gaaaacttcc tcaacatcct gcgcggtgaa ctcaagaaat acaatgtcca 1788720
tttcgaagtc gccggccgcc cgaaacacat ctactccatt tacaaaaaaa tggtgaagaa 1788780
aaaactcagc ttcgacggcc tctttgacat ccgcgccgtg cgaattctgg ttgataccgt 1788840
ccccgagtgt tacaccacgc tgggtatcgt ccacagcctc tggcagccca ttcccggcga 1788900
gttcgacgac tacatcgcca atcccaaagg caacggctat aaaagtttgc acaccgtcat 1788960
cgtcggcccg gaagacaaag gcgtggaagt acaaatccgc accttcgata tgcaccaatt 1789020
caacgaattc ggtgtcgccg cccactggcg ttacaaagag ggcggcaagg gcgattccgc 1789080
ctacgaacag aaaatcgcct ggttgcgcca actcttggac tggcgcgaaa acatggcgga 1789140
aagcggcaag gaagacctcg ccgccgcctt caaaaccgag cttttcaacg acacgattta 1789200
tgttttgacc ccgcacggca aagtcctctc cctgcccacg ggcgcgaccc ccatcgactt 1789260
cgcctacgcc ctgcacagca gcatcggcga ccgttgccgc ggtgcgaaag tcgaagggca 1789320
gattgtgccg ctgtccaccc cgctcgaaaa cggacagcgc gtcgaaatca ttaccgccaa 1789380
agaagggcat ccttccgtca actggcttta cgaaggctgg gtcaaatcca acaaggcaat 1789440
cggcaaaatc cgcgcctaca tccgccagca aaacgccgac accgtgcgcg aagaaggccg 1789500
cgtccaactc gacaaacagc ttgccaaact cacgcccaaa cccaacctgc aagagcttgc 1789560
cgaaaatctc ggctacaaaa agccagaaga cctctacacc gccgtcggac aaggcgaaat 1789620
ttccaaccgc gccatccaaa aagcctgcgg cacgctgaac gaaccgccgc ccgtacccgt 1789680
cagcgaaacc accatcgtca aacagtccaa aatcaaaaaa ggcggcaaaa acggcgtgct 1789740
catcgacggc gaagacggtc tgatgaccac gcttgccaaa tgctgcaaac ccgcgccgcc 1789800
cgacgatatt atcggcttcg ttacccgcga gcgcggcatt tcagtgcacc gcaaaacctg 1789860
cccgtctttc caacacctcg ccgaacacgc gcccgaaaaa gtgctggacg caagctgggc 1789920
ggcattgcag gaaggacaag tattcgccgt cgatatcgaa atccgcgccc aagaccgctc 1789980
cgggcttttg cgcgacgtat ccgacgcgct cgcccgccac aaactcaacg ttaccgccgt 1790040
gcaaacccag tcccgcgact tggaagccag catgaggttc acgctcgaag tcaaacaagt 1790100

caacgacctc ccgcgcgtcc tcgccagcct cggcgacgtc aaaggcgtat tgagcgttac 1790160
ccggctttaa atacaaaaat gccgtctgaa agccgaataa cgcttcagac ggcattttga 1790220
ttgccggggt ttgctatttt ttgttgcata gtcaattaaa aacaaaatag tacaatactc 1790280
aactttgaag gtctaaccat ggcatactct gcggacttaa gaaacaaagc tttaaactat 1790340
agtggattaa caaaaatcag gacaaggcga cgaagccgca gacagtacaa atagtacggc 1790400
aaggcgaggc aacaccgtac tggtttaaat ttaatccact atattacgaa caatgcaaaa 1790460
acatcagcca aaccgcagca acgtttaact tgtcaagaaa cacactttac ctgtggattc 1790520
gccttaaaaa acaaacaggc agcctaaaac atcaagttac cggtctaaat gccgtcaaat 1790580
cggataggca aaaaccggct caatatgttg ggcaacacca ggatgcctat ctgcatgaaa 1790640
tcgccaaaca ttttgattgt acggcagcca ccgtttgcta tgcactcaaa cagatgggga 1790700
taacgcgcaa aaaaagacca ccacttacaa agaacaagac ccggccaaag taacgcatta 1790760
tttgacacag ccggccgaat ttctgacta ccaacgcgtt tatttggatg aaacaggatt 1790820
tgaccgccac ctgttccgtc cctatgcccg cagcctgaaa gggcaaatag tgaaagcgca 1790880
gataagtgga aaaagatacc gacgcttatc tctggtgtcc gcacaagtcg gcaaccggct 1790940
gattgctccg atggtttatc aaaatacgat gaccggagtc ttttttgaag cgtggtttca 1791000
gcaatgccta ctgcccgcat tgactcaaaa atcggtgatt attttagata atgcacgatt 1791060
tcaccgtatg ggtgtcttac gggaaatggc ggaaaaattg ggacataagg tattgcctct 1791120
tgcaccttat tcacctgagc tcaacccgat tgagaaggtt tgggcgaata ttaagcggta 1791180
tctgcgaacc gtattgtctg attacgcccg atttaacgat gcactactgt cctattttga 1791240
ttttaattga atatacattt gaattaattc gcacttaatt taaatgtgtt tttaactgtg 1791300
ctttatttaa aggcaatgag aatgtgaaaa tatcggatca atcccaaagc agcctgcact 1791360
ttcgaaacgg ggtgcaggct gctttgggaa tttcataacc gtttcagcct gctttattcc 1791420
gcaaataccg tttccaaccc taacccgctc tctttcacca agcgcaaata agccagcatg 1791480
aatttatacc gtgcttgagc cagtttctgt tctgcttggg cgacttcctg ccgcgcccgt 1791540
attacttcca gccggttgcg gatgccgtat tgttggccgg tttcggtcga tttcagtttc 1791600
aaacggctgc tttccaaaac ccgttcttgc gccatgattt ggtaacgcgc cgcaccgctt 1791660
tcggtataag cctggcgtac ggcgagtttg atgtgccgct cggttgcggt cagctgtgct 1791720
tcggcggccc cgtattgcgc ttcggcttca tggattttgc ccgacaattc tccgccggta 1791780
taaagcggca aattcaactg tacgccgacg ctcatccctt tgccccgata gtggtagtca 1791840
ttattctgcg cagatgaagt gtagaggtta ttctgatagc cgacatgggc agaaacggtg 1791900
ggatagcggc tgttctgtgc tgcccgaagc gcctgtccgc tgctttgcag ggcaagctgc 1791960
tgcatccggt attcatgatt gttggataag gcaatgcgct gccattcatc cagactgtaa 1792020
cgttccagct tgggcagata gcgtgccaac aggttggcgg tatctatggc ctcgatttgt 1792080
ttgctatcca ggtcggtgta gtcgttcaac tggttttcat aggtttgttt ctcagccaat 1792140
acggcgattt cttgggccag ggcattgtcg taaccggctt tggcttcgtg aatatccagc 1792200
gcggtggcag cacctttatt gaataaagcc tgcgcctgcc ttacctgctg ggcataagcc 1792260
tctttttccg ccgcatgggc ggcaacggtg tctcggctga gtaaaacgtt gaaataactt 1792320
tcggcaactt tcaacagcaa ttcttcgcgt gccgcatcga aacgctgttc tgcagcctgc 1792380
gtatcgaacc tgctttggcg gtattgtgca aatttggcag cgtcaaataa ggtttgtccc 1792440
acctgcacgc tccatccctg tgtttcgcgg gtggaagaaa tcgatggcgg ctggcgctgg 1792500
tagctggcat tggcggatac atggggaagg aatgcggcct tggcttgttg ttgccgtgcg 1792560
cgcactgcat cacgctggta atgggacgct tgaaaatcag ccgaatgttg ctgcgccgcc 1792620
cgccatgctt caggcagcgt aaaagccgaa acggatgggg aaagggatag tggcaaggta 1792680
aaaagtgaaa cgggtaggat atatttggaa aaataggatt tcatagccga aaatagttca 1792740
tgttgcaaat agggcgtcag tgtcaggcaa acggaaatac cgtaatcttg cattatcatt 1792800
agattgagca atgtcatccg ggcaatggtt tcaggcagtc tgcatgtccg aaccggcgga 1792860
taacaaatgc ccagtacgga tccgcctatc gctccctaaa gctttcgtcc aatttggttt 1792920
gcagcgggct taacagataa tccagcaccc gccgtttacc cgttttaatc tccgccgtga 1792980
cattcatgcc cgccgtcaga ttcactgctt tgccgtcaat attcaaggta tgtttgtcca 1793040
gcgacaccac cgccgtataa accaagccca actgttcgtg gcttaccgca tcatggctga 1793100
cacttttcac cttgcccgtc agataaccgt agcgcgtata gggaaagctc tcaatcttca 1793160
ccaccgcatc ctgtccctgt tccacaaaac cgatgtcttt gttcaatacc aaaacttcca 1793220
cgtccatttt gtcgtcatcg ggcgcaatca ccatcatttt ttgggcagcc tgcaccacac 1793280
cgcccaccgt ataggtagcc aattcctgca ccgtgccgtc cgcaggcgac tgtattgtca 1793340
tcagctgctg ccgctgcttt gccttatccg tttggccgcg gtattggtca atctgttcgt 1793400
ttgcctggcg cagcgcatcc agcgtatcgc gtttcaggtt ctgcgtattc agcacccgat 1793460
tctgctccgc ctgtgcaatg gccgcctgaa tctgcctcat ctgaccgcgc gtactttcca 1793520
aatcgttcca attgctgacc gatttgctct gctgctccaa aaacgcatgt tccgaaataa 1793580
aattgtcggc ccgcaaacgg cggtagtctg ctgttttctg ctgctcgatc gcccccaccg 1793640
aaaccagctt ctgctcctgc gccttggccg actgcaattc cgcctgatgg ccgcgcaaag 1793700
ccgactgcaa ttgcgcatcc tgcgccgccc atgcctgata ctggtgctgc gccaacacct 1793760

gcgccgattg cacatcggca tcggagagac ctaaagaccg tgcttgcgcc atatcgatat 1793820
gcggcacggt acggctttcc aatgccgcca ataccgcttc ataacgcagt ttggacaatt 1793880
gggcagcctg caaagcctgc tccgactgca ccacatcgct gtctgttccc acagcctcca 1793940
gttccgccag cgtttctccc tgtttcacat gctgcccgtc gcgcacatgt accgccttaa 1794000
ccaccgccgt ttccagcggc tggatggttt tgctgcgccc gcccgacacc gttttgcccg 1794060
aagccgccgc cacaatatcg attttgccga accaggacca caacaaagcc aaaagcgcaa 1794120
acgccataat aaaacgcgcc gcccatttcg gagcggcaga gaccggcgta tcggtcagtt 1794180
ccaaatgcgc gggcaaaaac gcctgttctt ccgccgtgcg tttgggcggt ttcaactggt 1794240
cgcgcaccgc ccaaacattg cgccatacag taatgtatcg agaaagaaag gatttcaggg 1794300
cggagaaaaa cataacgggt ataaccttgg caatatagaa acaggaaaca atataaatat 1794360
gtaaaggaat tttaacggaa agcgcggcag ctgttaaggg aaaggcggga atattgacaa 1794420
aaaataccca agtcgttaca aatattcatt attttactgc gtaacgcaac gctgaagcgc 1794480
aggctgcttt tgagatgcgg caaggttcgg caaaaagcag cctgcacatt taaccacagg 1794540
aacaacccat gtttaccaca aacgatttac gccatttcct agaaggtttg gccatcctat 1794600
tctcaatcgg ctattggggc accatgctgc tgttgctttg gtttctcgtc cgctttgcct 1794660
ataaaaagcc caaacggaac cccggcaaaa tcctttcagg cagcctgacc gccgccgcta 1794720
tcctgatgct gtttgtttgg attattccca aacaattcgg cccgatcaaa gaagaaatac 1794780
aggcacaaga agagtgggac agaaaataca aagaagccga agccgtgttt aacgaacaat 1794840
gcaaaacggc gggggaaaga tttaccagac ggcggacaat gtggaaggga ttatgctgtt 1794900
gaaggtagta cctgagcgta ccgtttcggc agatgcaaaa accagagacc cgatgtggga 1794960
caatgcggct ttacagacca gcgaaggcgt aaattttatt gctcgtttcc taggattttt 1795020
tagcgatggg gaataccgct atgtggatgt cctgcaaccc aaccattccg atattattcg 1795080
gtattcaggt aaagattttt ccgctaaatc aaatatttaa tcatatacac cccgcccgtt 1795140
atgcggtaac gttcgaaaac aatgtcgatt ccaagctgcg caggcactgg gtggcaggtg 1795200
cgaccatacg gattatcgac cgccaaactg acgaagtgat tgccaagaaa accatctatg 1795260
tctttgaaaa aggcttggac ggcacgggtg gggcgagaat gccgtggaag tttgctatct 1795320
tgtgcaataa agaaagactt acttcttcag agccgttatc ggattttgtt cttagcgttt 1795380
taaaacctta tatattgcgt cccttatata ttgcgtccct aagaagggac gattaacaaa 1795440
aattaacgtc ctttactttc tacaagtaac agggcttttt tttgcccgtt tttgaggatt 1795500
cgcaccatgg aagataagca agggatgaca aaggcggttg ccggcgtgat gacggacgcg 1795560
ctagcggacg gcaggaagcc gacaaccgct tcaaatcttc ccccttatc taacaggggg 1795620
ggacagaaac cgaaacggca ggcagggttc aggaagtctt cgaatgttac gaaacgtaca 1795680
taacggacgg taaaggaaac ctgttaggcg ttcctcttcg gcgcggtgta tcagattcgg 1795740
ctttcattga tcaaattagc ttttcatttc atgaaaaaac ctttttcgat aaatacggcg 1795800
ttcgtgtaag tcttttggaa gacgaagatt ttattcgcgc cgcgtccatg ctcgccgaag 1795860
aagttttcgg tttcggtatc tacaaagaat ccaaaggttc gggcggtcgt ttctatgagc 1795920
gctgttggtt gatgggttcg gaagacgccc tatacggtcg cgtccatttt ggcggccaac 1795980
aaaataccat tcttttcgaa ctgaccggca ccggttgcgg cgtcgcaaaa gaaggctggg 1796040
aatcacgact tttcgcattc ctgactaatg caatccgccc aaaaatcaca cgcgttgaca 1796100
tcgcaaaaga ctttttcaac ggcgaataca gcccgaacca agcccgtgaa gaccgaaata 1796160
aaggtatgtt tacctgtcat cacgtcaaac caaaaggcga atgtttgggg tcagattggg 1796220
aagaagacga tgaagccaaa atgaccaaag gcaagaccta tggtatcggc tcccgtgaat 1796280
cgtccaaata tgtccgcgtc tatgaaaaag gcaagcagtt gggcgataaa acaagcacat 1796340
ggacgcgatt tgaaattgaa ttcaaagcaa aagacatcgt tatccctttc gaagttttgc 1796400
agaatccggg cgaatatttc ggcggcgcat atccgatttg cgaacgattc gcccaaaagg 1796460
caacgcgcat acacgcggtt aaggaagata aggtcatttc agccgaccgc taccttgaat 1796520
gggtaaaaaa acagttcgga cgtgcggcaa acggtctgaa attcattttt cccgaattgg 1796580
acaaagccaa actgtttgaa ctgattgagc cgagtcatca caagctgccc aagtctttgg 1796640
ctcccgaagc ctacgactgc gcctttttga aagctcaagc cattcatgaa cagcccgcat 1796700
tcaaaccgta caaagaccct tactatatgt acgaatatta cgagaatctt gaaaaacagc 1796760
ttgaacagca aaaacacgtc aacaatgaag aaagctataa caacttcatt tacgacaaat 1796820
tcgcaagact accgatttca tgggcttaaa gtgtctgccc gaaagacgtt taatcacaca 1796880
aggaaaccaa aaaatgaaca tccaacttca aggccacatc gtcggcgtta aaaaaatcaa 1796940
cggacaaatc gaaggcaaga gcttcgacta ttgctgcctg attgtcgcca caccccttaga 1797000
cagctcccaa ggcaacgcat tgggcagctc tactactgaa tacgatttcg gcggctctgc 1797060
caatttcgag cagttccgaa acgcccaatt tccgatcgaa gcaaacctga acgtagaaat 1797120
cgtcactacg ggcaaaaccc aaaaactgaa agtcatcggt tttcaactcg tgaagaaagg 1797180
ctgattgaat gcagaaagtc tatgttgtcc agtccgtatc aacaggggac tttctgtatc 1797240
tctctcctga aacgggcgac atcggacata ccaaattaat caccaatgcc gattatttct 1797300
acgacttcga agaagcgatt aacgcaggtt tggaagaaat cggcaaccaa tacgaatttg 1797360
tcgtattcgg atttttgaaa gactgatttt cggatgttcg gcggtcgtct gaaaaacgct 1797420

```
ccatccatta ccgccaaaca ctttttgaag gaaaatatca tgaaatttat taacacctgc 1797480
cgtaaatacg gcgcaaaact ggctgttgta acagctgctc ccctggcttt ggccgcacat 1797540
gcaaatgcaa cgttgcccga tacggcaaaa aacgctttgg aagccgcaaa agcggacggt 1797600
atggaagccg gttggattgt agtgggcatt ttcgccgcgc tttttgtatt ttccatcgtt 1797660
aagagagtga tgaagtaaga cggcatgtac taccaagtcg gaaataaatg tcttgagaag 1797720
caccaggctg aaaaccttta tttcagcttg gtagtaccaa gaatcaaaga aaacggacag 1797780
attgtcaggc cggaatataa cggcagcctg tggaagatgt cggacggtca gccgctaagg 1797840
cttttattgg cggaatgcag tccgaaagac aacctgcaaa gcggtcttga aacaggctgg 1797900
atagtattcg gcatcctcgc gtccgtttac tttgtttccc tgctgaaaaa ggttttgaaa 1797960
tgatggattt ttatttttat ctcggcgttt ccgtacccgt attaatcggg gcggttctgt 1798020
ttaagaattg agcgcatgaa gttatggtgt caaaatcagg cttttaatta gacatttgag 1798080
gcttgaaacc atgaataaaa atgaacgtga ctttttctat atatcaaatt ctgatttaga 1798140
taaattgtca gaatcttatc ctgataggcc tctttcttat gtgttttatt gttatttgaa 1798200
agaaactggt ctattgaaaa atttctcaat ggataaatgt cataattttt ttaatagaat 1798260
taattttaat gaatcttgct ttgaaattaa attcaaggat gattcatttt tcattattgg 1798320
caatggaaaa attgatgttt cggattctaa taatttcttt tctgtttctt ttgagtgcta 1798380
aatctttttc agcagattta gaaattaaaa atgggaaatt gatgtatgca ctttcggaaa 1798440
aatataacga taatggattt aaggcataca aagttttagg tgagggagga ggaattcata 1798500
cagaatataa ttacaaattt gataaaagtt tgaatttgaa tgtattagaa agttcaacag 1798560
gcgcacgctc tcttgaaaaa gtccccgtta aagtaactgc atcagtttcc cgcgccgccg 1798620
tcttgtcagg agtcggcaaa cttgcccgct taggcgcgaa attaagcaca agggcagttc 1798680
cttatgtcgg aacagccctt ttagcccatg acgtatacga aactttcaaa gaagacatac 1798740
aggcacaagg ctaccaatac gaccccgaaa ccgacaaatt tgtaaaaggc tacgaatata 1798800
gtaattgcct ttggtacgaa gacaaaagac gtattaatag aacctatggc tgctacggcg 1798860
ttgacagttc gattatgcgc cttatgtccg atgacagcag attccccgaa gtcaaagaat 1798920
tgatggaaag ccaaatgtat aggctggcac gtccgttttg gaattggcat aaagaagaac 1798980
tgaataaatt aagttctttg gattggaata attttgtttt aaatcgttgc acatttaatt 1799040
ggaatggcgg agattgtttg gtcaataaag gtgatgattt cagaaatggg gctgattttt 1799100
cccttattcg caattcaaaa tacaaagaag aaatggatgc caaaaagctg gaagagattt 1799160
tatcgttgaa agtcgatgcc aatcccgaca aatacataaa ggcaaccggt tatcccggtt 1799220
attccgaaaa agtagaagtc gcacccggaa caaaagtgaa tatgggtccc gtcacggaca 1799280
ggaacgggaa tcccgttcag gttgtcgcaa cattcggcag ggattcgcaa ggcaacacca 1799340
cggtggatgt tcaagtaatc ccgcgtcccg acttgacccc cggaagcgcg gaagcaccga 1799400
acgcacagcc gctgcccgaa gtatcgcccg ccgaaaaccc cgcaaacaac ccgaacccca 1799460
atgagaaccc cggcacgagc cccaatcccg aacccgaccc cgatttgaat cccgatgcaa 1799520
atcccgatac ggacggacag cccggcacaa gacccgattc cccgccgtt ccggaccgcc 1799580
caaacggtag gcatcgcaaa gaaaggaaag aaggcgaaga cggcggctt ttgtgcgatt 1799640
attttccgga aatcctagcc tgtcaggaga tgggcaaacc ttcagacggc atgtttcacg 1799700
atataagcat accgcaggtt atagacgata aaacatggtc ttcacataac tttttaccgt 1799760
ctaacggcgt atgtccgcag ccgaaaacct ttcatgtttt cggtaggcaa tatcaggcaa 1799820
gctatgagcc gttatgcgtg tttgccgaaa aaatccgttt tgccgtactg ctcgccttta 1799880
tcattatgtc ggcttttgtc gttttcggtt cgttgaaggg gaaataaatg ccattacttg 1799940
ccggtctgat tccactttta ggcatacttc tgaaaatgct gattgtcaga ataatccttg 1800000
caacaggtct gacatttgta acctatgccg ggtatctcat cgcgctggaa aagttcaaag 1800060
actacacgtc aaatgcgatc aattccatgc cttccgacat actgaacctt cttttaattt 1800120
cgggattcgg tcaggggttg ggctacctgt tcggcgcatt ctcgttcttc attggtatgc 1800180
acgcattcaa aaaactgacg tttgtctttc caggatgagg tagaagcatg atttatctgt 1800240
ttacaggaaa catggggaca ggcaaaacct cccgcgtcgt ctctatgatt ttgaacaacg 1800300
aagacggatt gttcaaaatg aaattggaag acggcacaga ggtagacaga ccgctttatt 1800360
tctgccatat cgacggattg gataaacggc agtttaaagc ccacgaactg acggaagagc 1800420
aaatcatgtc cgccccgctt cgtgatgtca taccggaagg cgcagtgctg attgttgacg 1800480
aagcgcacta cacttatccg gtacgcgcgg caggccgtcc cgttccgcct tatattcagg 1800540
aactgacaga actccgccat cacgggcata ccgttatttt gatgacgcag cacccgagcc 1800600
aacttgatat attcgtccgc aaccttgttt caaagcatgt acaccttgaa cgcaaggcaa 1800660
tcggaatgaa acagtattat tggtataaat gcgtaacctc gttggacaat cccgcaggcg 1800720
taagcggcgt agaagtcgca agttggaaac cgccgaaaga agcctttaaa tactataaat 1800780
cagcaagcca gcaccaaaag ttcaagaaaa aagtaccttg ggcggtttgg gcgttgattg 1800840
cgattgtagg gtttgtaggc tggaaaagtt acggcatttt taaagtttac agcaaagcca 1800900
cagacagccg gattgagcag gaagcgcaaa aagaaagcgt tgtgcagacg atgacggagc 1800960
agcctgcatc atcagaggaa atgcctttaa aaaattcaga caatttgaaa cctgaagact 1801020
ttgtgccgac tttacccgaa aagcccgaaa gcaagcctat ttataacaca gtccgacaag 1801080
```

taaaaacctt tgagcaaatc gccggatgta tagacggcgg aaaatcagat tgcacatgct 1801140
attcaaatca aggaacaccc ttgaaagaaa taacaaagat aatgtgtaaa gaatatgtga 1801200
aaaacgggtt gcctttcaat ccttataagg acgaacagca aaggacggaa caggtggaac 1801260
agtccgcgaa agcggacaag ccgcaagttc tcgtaatggg cggaaagccg tagcaaaatc 1801320
tcatgtacga caactgaaga gcgcggaaaa ccgtttgaag gaattggcgg cggagtcgta 1801380
aagcagaaag ttcaatccct acccctcagg atggcttgag ctgagtgaag gggttaatt 1801440
gctagaatgg ctgttttttt taaagtgtct cagtctggaa tcgcttcgtt cgggggttgt 1801500
aggtgcagga aaatagggca gaaaaaagga aaagggggaa gctttgtaaa gattgggcgc 1801560
gctttttacc caatctttat gaataccccc ttttcctttt ttatgaactg tttttcaata 1801620
ccggaaaccc ccgaacggag tgattccaga ctgagatacg cccaaaaaaa atcagacatt 1801680
cgggtcgcaa cagaaacctt taccaaaacc tgcgaccccca ataaaatcag atacggcaaa 1801740
ggcgataagc ttcaagccct gaatgagtaa atcagcccat tgagggcttg gcgtttgacg 1801800
aaacaccaag taaagcccac gacttcgaaa gtacggccaa agcgtacagc ttgtaagaaa 1801860
gatagaagcg tgggctttcg tacatcttaa gtttgaacac tatctagggc aaaaagcccg 1801920
aattaataag gttaaaccat gtacttagga atagacgttt caaagctcac aatagattgc 1801980
tgtttgattg tagacggtca aaattatcaa aagaagtttc agaacaacaa aggaggattt 1802040
gaacaattaa taaattggct acaaagtcat aaagtaaacg ataagctcca ttgcgtgtgc 1802100
gaagcaacag gcacatatta cgaagcatta gccgaatatc tttattcaag atatacaatt 1802160
accgtagaga atccacgaaa gataaaagga tatgcgatag cagaactaca acgatcaaaa 1802220
acagatacac aagacgcaaa gttgatagcc caatattgcc aagaccgaaa gcacaaatta 1802280
aaagcatgga aaccgccgac aaaagaacag aagcaattac aggaaatcgc ccgatattta 1802340
gactatctga aacagcaacg cgcaacagaa aaagctaaac aacacgaagc acccgactat 1802400
atcaaatccc atattcaaac aactatttca aacctgacag cacaaataca gatagtcaaa 1802460
aagcaattac tccagttcta caaagacaat ccaagttata acaatctacg caaaaggctg 1802520
aaaacaataa caggcatagg cgagcaagcg acagcagtat tgctatcaac ctataaaaga 1802580
catgaattta aaaatgcaag acagttcacg gcttatctag gcctagaccc tagaaaattt 1802640
caatcaggaa caagcgtgaa cggaaaaagc agaatatcaa aaataggaag ttcggaaata 1802700
aggaaaagcc tttatatgcc tgcacttgtt gcatatcgtt gtaatgcctt ccctgaattt 1802760
gtagggcgtc tgaaaaataa agggaagcat ataaaattga tattaattgc catcatgcgg 1802820
aaactggcgg taatagcgtt tacgattttg caaaacggcc aagatttcca agtggaaaga 1802880
tataaataaa aaattaaact gggctttcgc cggtgatttt caatttattg aaaataaagt 1802940
ataaattaaa acatcaaatc cattcaaaac gaaacaaaca tcccgaaaaa gtcggggtgc 1803000
gcattcttgc aacttcaaga aatgtaaagt tatttgaccg tgaaatacac tatctttttt 1803060
tcaacaagcc accacagcaa tcagacaaaa gcaacccacc gccacaccca tgtcggcagt 1803120
acggccggac aaaccaccat ccgaagcggc ggggatacca ccctcaaagg tgctcagctt 1803180
atcggcatag gcatacaggc agaccccaa ctacaaccct gacgactatt ggtggaaccg 1803240
atattaactg acccccaaaa gttggacagt ttaatcaagc ggctttcagg gactgaattc 1803300
tgtactgaac agggctcagt ccttttaatt tcaacttgat tctatcgttg ttgtagtaac 1803360
ggatatattc gtgcagtaca gcttccaatt cggtaacgga atcatatttg cacgtatgga 1803420
aacattccga tttcaacgtt ccgaagaaac tttccattgc cgcattgtcc aagcagtttc 1803480
ccttgcggga catactctga accagaccgt tgtctttcaa ctgcttttga taaatatcat 1803540
ggatatgccg tttcaaatcg gcatatttgt cttctgccga ttggacaacc aattggtaat 1803600
agaaggtgcc gcgtggcagt ccgacaatca ccaacagcag ttcaacggat ggcattgcct 1803660
taaccctgcg acgagttgcg ttctttctac cgcacttctt tcccatagat taaggcatcg 1803720
agctttttta gggcagccat ttccgcttta aggcaagcca attccgcaag cagttcttcc 1803780
ttggttttca gatagtcggc tttttcgttt ccggcggatg ctgttttttc acgggctttc 1803840
ttcctttggg tttagggttt gggctttaaa ccgttaatac cattcaaatg gtagaggcgc 1803900
aaccattgca gcaagatgga gcagtcgggc aaattcagtt ggtctgcggc agctttttgg 1803960
gacattccct accccgccac caggcggatt gcctcaagtt tgtattcgac cgaatatttt 1804020
gtcgtatgct ttctacgttt gatgccactc tctccgtgta atctgtattt tgtcacccat 1804080
ctgcgtacca atgaatcgga aatagaaaga tggtctgctg ttccctgcca aatagtattg 1804140
aacgacggca agtcggaatt catctgaata ttttgccata aaaaactgca ccccctaaag 1804200
tcggtaaggt gtccaacttt tgggatgcag ttcagaagcg gtcttttttt gcctgccggt 1804260
tttgaatcat cctccgtgta tattcccttg acgaaaaaaa tgatgatatt acggatacca 1804320
aaactaaggt cgtatccgcc cccctactct ccctaagcaa agagatgaaa cagcgtatcg 1804380
gctccctgcc ggttgaattt tccgaaaaaa cgcgacgtaa ccagcatcaa catatataag 1804440
aacagcacaa atagcatcaa tacatcaggc aacgaaaatg cagaataatg cacttaatgg 1804500
tgtttggata tctgttgttt tgtgctgtta gtaattcttc tttctgtgtt tacagtttag 1804560
cagttgtaca gttttacagt aatgtttaaa caatgactga tttattttaa atgcagatat 1804620
tgtagaggat aaaaatggcc aaagtccttt cagtaacatt tttgattttt tagcgagcct 1804680
tctcatttcc ccggcgagat cggcaatggc agcggtactt tggccgccga tatgcttaag 1804740

ttcagtaacc ttacgccacc aaaacccttg ctagctaagg gttaaacagc tcacttgaaa 1804800
tctacttaag tctaatctaa actatccaat atggatagat ttttaaacat agggcaagca 1804860
gcaaaattat tgtagctgaa agcacaatca ctcgctggtg gtctcaaaca cgtgccgact 1804920
acctcgccga aaacactatc agccgcgata aaccgtggga aaagctcgtt atcagccgcc 1804980
gcacttggta ctatcgcggg aaaccgatgc tgtctgaaac gcaacaggag aaaaataatg 1805040
agccgttacc tgattacctt tgatatggat accaactgcc tgaaagacaa ttaccacgga 1805100
aataactata ccaatgccta ctccgatatt aaaaccatct tggctagaca tggatttgag 1805160
aacattcagg gcagtgttta tctaggccgt gaaggcatca gtgaagcaca cggaacaata 1805220
gccattcagg aactgaccgc tcggtttgat tggttttact cctgtatttc aaacattaag 1805280
ttttaccgcc ttgaaagtga tttgaacgca caatttatcg ctgatggtgt gtatcaagcc 1805340
aaacaggctt tccttcaacg tgttgaacaa cttcgtatat ccctaacaga agctggattg 1805400
tctgatgagc aaatcaatca ggttctggaa aaacagaaat ttgaattgga aagtcctaac 1805460
ctgaaattaa attaacctcc tttactcacc aacatccgcc gcagctctgt cagtttttgg 1805520
cgcgctgcgg cgatttctgt gcgttttaga gcttcgggta gggtgtgaaa caactcactc 1805580
gaaatttact taagtctaat ctaaactatc caagcagtaa ttagtacaaa aaaggcaaac 1805640
ttattttagg agtttaaaat tgcagctgcg ataaaccgtg ggagagtctc ggcatttccc 1805700
gcgccacttg gtacaaacgt ggcaaaccga tgccgtctga aaccgtacaa caggagacaa 1805760
aataatgctt gctcaaatcg aactcacacc ctgccagcta atggtttacg taacttaagg 1805820
ttactggatt tacgcactaa ggttactgaa cttaagcata tcggcggcca aagtaccgct 1805880
gccattgccg atctcgctgg ggaaatgaga aggctcgcta aaaaatcaaa aatgttactg 1805940
aaaggacttt ggccattttt atcctctaca atatctgcat ttaaaataaa tcagtcattg 1806000
tttaaacatt actataaaac tgtacaactg ctaaactgta aacacagaaa gaagaattac 1806060
taacagcaca aaacaacaga tatccaaaca ccattaagtg cattattctg cattttcgtt 1806120
gcctgatgta ttgatgctat ttgtgctgtt cttatatatg ttgatgctgg ttacgtcgcg 1806180
ttttttcgga aaattcaacc ggcagggagt cgatacgctg tttcatctct ttgcttaggg 1806240
agagtagggg ggggcggata cgaccttagt tttggtatcc gtaatatcat cattttttttc 1806300
gtcaagggag tatatcgact ctagaagata ggtattagat actgcctttt cttacaagag 1806360
tgatggtagg atggttctct tcaagtcaat caaacaggaa agtatttctt ttctgtctga 1806420
agatttgaaa aaggactgga tgtttcacaa ggttaaaact ggggaaaaag atggatatgg 1806480
ttcagatgaa atgctgagcg taccccgtgt ctatttggaa atgatgtcgc ggaaaacggg 1806540
agtcccctac tccagtattc tttaaattct aagcagaaga cttcttcgtc ggtctttttt 1806600
tgttgtttgg tttgcatgga gtaaaactgt acaactgttg aagcataaaa ccagcaagaa 1806660
ggtaaaaaag aaagaagcag ttctttggat tttagatatt accgcaatta gtttcctttc 1806720
ctaaaatttg tttaaattat ttgcaatatt aatataaacg agatattaat gatgagaaat 1806780
caaaaaggca taatgaatat attttgtaca aaatatttgc agtatttaaa aatgttggtt 1806840
cgtatatgaa aagttaaaaa tgccaaaatg tacagttgct aaactgtaaa actgctaaag 1806900
caacaaaaca taaaaaggaa tgcaaggatg cgatcactac atctttttat tccgaagcgt 1806960
ttatgatttt acggtcaact gctactctat gtgcctagct tttcagctcc ctattttcga 1807020
atattggagg aggcattttc atcagtgtcg taatgccgac caaaactctc acaaaccata 1807080
ttggttcttg tggcagcaac acctatccgt ttgttcaagc gaccacaaga gtaacatgat 1807140
tggctggtag cattggcttt aatctcttcg atatgaactc catttttagc tgcaccttct 1807200
ttcagcatat gcagcagtag ggatgaggca actatcttct ggtggagttt gttgacttta 1807260
atctgtatgc actgtaggtt gagcagattc aattttttga tacagattat ccggctttgt 1807320
tcggcaattc tgtttgcgaa cgtatagtag agctgtcgtc ttgcagcttt caatttgcgg 1807380
taggtatttt accattcaat gcgtagccgc tcggtacgtt tgagccaaat gttatcttcg 1807440
ccatttgtac caatttgttt ttacattagg ctgtgtttta gtaatctatt gatttcaatt 1807500
atttgcaagg gaaaagacaa ttattttccg gttaggaata aacctatcct gttgaatacc 1807560
ttaaagccaa atacgcctat caacaccata ttaaaacaca gcctttttta atatagtaga 1807620
cacaatcttt ccctatttat gaaggtgatc gtttctttca gattcgtatt ttaatgcttt 1807680
ctatttctat aaaaattgac tagaatagct caattataaa aaattgcgcg attttggtat 1807740
ttatcatgaa aatttccaga cctccggaat ttaccctgtt gcaacaggaa tatatgcagc 1807800
atctcactga aagaatgacg caaattgcca agctgctgaa ttcttccgca aacaatcctg 1807860
atatagacat tcccgatttt cttactgaaa tcaaagatta ttcagaattt tccgtgacag 1807920
atgaaaatgg aacctacctg cattgggaca aattccgccg gattcacacg gaagatacgc 1807980
ggatgaaatg gcgcgccgtt aaggaaagcc gcaaaaaaat ccaaaaacca attgatttcc 1808040
cgtttgaaca tcagttttgg ttctgcattc ccgactcttt gcaggcacgg cttcatttga 1808100
ttgacaaaag ctgcggcagt tctatcggca cgtctagctt gggtggcttc ggcagaagcg 1808160
agcaaaacag attcttgctc aagtctctga ttatggaaga agcgattaca tccgcccaac 1808220
tggaaggtgc ggctaccacg cgtaaagtgg ccaaggatat gctcaaatcg cagcgtaaac 1808280
ccaaaacaaa agacgaaatc atgatagtga acaactatca cttgatgaaa aaagcggtag 1808340
aattgaaaaa tacgccgtta agtgttgaaa tgattttgga tttgcaccgc attgctacca 1808400

gtaacgctat tgaaaacaag gccgagcccg gacaattcag gcaggatgac gaaatcttta 1808460
tcgccgatat caatggtaac agcctgtatc aaccaccgcc gcacggacag gttcatacgc 1808520
tgatggaaga ggtgtgtgcg tttgccaata atacctatga cggcgtggaa aatccgttta 1808580
tccatccggt tgtccaagct attatcttgc atttcctcat cggctacatc cacccatttg 1808640
gtgatggcaa cgggcggaca gcgcgggctt tgttctattg gtttatgctc aaaaacggct 1808700
actggctatt tgaatacata tccatcagcc gtcttctgaa aaacgctcct gcccaatacg 1808760
ccaaatccta tttgtatgcg gaaactgacg atttagattt aacctatttc atctattacc 1808820
aatgcgatat tatcaagcgg gcggttgccg atttggagca ctacatttcc gacaaacaaa 1808880
aacaccaaca ggaattcaaa gcagcgattg cccaatatac tgaaaagata ggaaagttga 1808940
accaacggca aattggtatc ctgcaaaaag cagtggaaga aagcggaaaa atctttactg 1809000
cacaagaaat tgccaaccaa tacggcatct ccctgaatac tgcccgtagc gatttgagta 1809060
aactgggaga atatagattc ctagtgccgt tcaaatcagg aaatgcttta gagtatgttg 1809120
ctcctcagga tttattggaa aggttagaaa aaaaatagtt tgctagccca gaatgcagct 1809180
ttaaccgagt caaaatcaat acagtccgca ccttcaaaaa gaagctgcgg actgcttgct 1809240
ttttgctcta caaatgatct ttgtagctga tttaaccaag attgtagcaa ttttgctttc 1809300
caagcaagca gggttagaaa attcgatact tttaattatt ggctgtgttt taatatgatg 1809360
ttgataggcg tgtttggctt taaggtattc aacaggatag gtttattcct aaccggaaaa 1809420
taattgtctt ttcccttgca agtaattgaa atcaacagat tactaaaaca cagccttaca 1809480
ttattggggt gactatcctg taaaatatgt cctaaaacgt ggaaaccact tttgctctgc 1809540
taaattttaa ggaatcttta tgttacatat accccccaac ggaacctgtt cgatatgtta 1809600
gcagtattgt agccttaaac gtgcatagtc ctaacggtac aggcgactgg catagtgcaa 1809660
aggcattgag tgatcgggct taccctgaaa aattttatat ttacggtgaa aatcaagagc 1809720
gaaataccaa tcatttattt ggtgataatg gcattattga tgggacggat cgactgaaca 1809780
aaatgggtta tttccctgaa aacatcccag tttggctcgc agatcacccc cgtgcttgcg 1809840
tggattatct ttacacagca gtgttacaaa ctggctcaat cggtcgggtg attttagatg 1809900
attggtttcc aagcgatgaa gacaagcaat cagtttatga cttacttaac caaatcgaac 1809960
cgcacttgaa tactcaagaa tgggagaatt tacagctatg gaaacgcaaa aacccaataa 1810020
tgtaatgcta accgaacgag ataaaaggca cgagaagcgg tattactgaa tttcatccgc 1810080
aatacgccat gtacaattaa atcagcagat gtgtctaaac gtaccgctgt tcccgtttga 1810140
aagaattttt catgatgaag tctatcctca ccgtatccgg aaatcgtatg cgtaaaccca 1810200
gaatcaccta tttggatgtt tgggcaaacg atgaaagaat cggtactttg gaaaaggggg 1810260
ccatgtatcg gttcgcatac gacaatccca attcttcgtt gctgggcctg cattatcaag 1810320
acagaagcaa ggtatatatc agcaacaata tgccgcatat ctttgcacag tattttccgg 1810380
aaggcttttt ggatgcacac atcacaagca aatatgcttt tcatgatgcg ccttttgaag 1810440
acaatgagat gctgcgcttg gcaattctgt gcagagagac tttgggtcgg atacatgtgc 1810500
gctgtaatga cccgcttttt aatgaatgga ttgacgggtt ggagatgaaa aatccaagaa 1810560
tattgactga acgggatttg ctgggcataa atgcccgaca ggtttttcag caatatatgg 1810620
cagaaatctt ccatcacggc cgtttcgtca gtgtatccgg gatacagcag aagatgtcct 1810680
tagatgccat ccgcagaaat accaagcaaa ctgcctcata tattgccaaa ggttttgatg 1810740
catccgaata tccttgcttg gctgccaatg aatttttatg catgcagacc atcaaacaag 1810800
ccggcattgc cgttgcacag accagcctgt cggaagattc atcagtctta ttggtacgtc 1810860
ggtttgatgt cagtgaacag ggttattttt tagggatgga agactttacc agtctgcgcc 1810920
agtattcggt agaagataaa tataaaggca gttatgcggc tattgcacag attatccgac 1810980
agatatccgg cagaccagat gaagatttaa tccatttctt taatcagctt gctgccagtt 1811040
gcatattgaa aaacggcgat gcacacctca aaaatttttc agtactctat catgacgaat 1811100
acgatgttcg tcttgcacct gtctatgatg tattggatac atcaatatac agggttggaa 1811160
cacaaggaat tttgatgct tatgacgata cgctggcatt aaacctgact aaccacggta 1811220
agaaaacata tccttccaag aatacattgt tggattttgc tgagaaatat tgcgatttgg 1811280
gaagagaaga tgcatccttt atgatagata caatcgttca agctaaagaa caggttcttg 1811340
ttaaatactc ggatgtattg cgtgagaatg aatggttggc gcagaagtgg cattttatcc 1811400
cggatgaaaa tgaagaaggt ctaccgttta cattccggta gctgccgctg tcagagatgg 1811460
ccggtctact ttcaccctga aaatcacttc atcttatggt gtttgaaacc gagaaattag 1811520
aagaatcgta ttcggtagga gatatactgg gaagattgga aaactggtaa atcactctat 1811580
tgattgagtt ggcggcctat atgtttaatg taggcaaacg agaaggaatc atgtatttca 1811640
tgattccttc ttaaattcct gtgtcaatct aatatcaaaa cacagccatc tcttaccata 1811700
atcatgatag gtgttttatt atgaaaagct atatctatag ttaccgttga tttgactatg 1811760
ccgttttaaa acgtatagcc tacctgaaaa ccgcttgccc atttccttga ttggaaaaat 1811820
tcgggctttt tcaatgcgcg gccggtaaat atatcgtaat gcaggttgcc gccaagcttt 1811880
atctgcccgc gtatcccaat tgctgtgccg actagagttt ggcccgataa ccatttggcg 1811940
gattgtcctg aaacatgtcc tacatcagcc ccaagataaa gctgatggcc tggtttaaat 1812000
tgccagctca aatcgttgcg ccaataccat ccccgctcgg cagacaaact catttcaccg 1812060

```
tcgaagccac gtacggtgtg gtgtccgccg atagccagtt tgtcttgcga tgttagcggg 1812120
gttttgttcc attgtgcatg aacggatgtg tcataggcaa atagctgttt accgatttga 1812180
aaaggagtat ttacatcagc cgatgccgtc caaattttca tacgtgacgt gccttcgcca 1812240
aaggcttctt caggcgcgcg cagagcatct ttcatgccgg tgccgcgttt atatttcaac 1812300
ttaaaatctg ccgtactgcg accgatatat tctttgtggg aaagttctgc caaccaaccc 1812360
gcagttttac gccgttgtac agtcagttcg gcatcatcaa tgtaactttt tgtttccctc 1812420
atccacagtt ttacaccgag ataggttttg cgtttggcat cacgatacaa caggcggttg 1812480
aagccgaaat cagtattgta actttttcca ttatagtcat agacttccga taatccggaa 1812540
actgcctgat ggtaacggta gccattgtga ttgaatgccc atgtccattt accgaaaggg 1812600
gctgaataat gtacggcgta attgtttgat ccgccttctt tgcgatggcc gtcaaaactt 1812660
tcctcatcgg gcgtaccgcc aatcgaacgt ccataattta catagaacat atcactcagt 1812720
cccaaaggat tgtcggcaga gaaagtgata tttccttggt attttcctgt cgcctcacta 1812780
cccgaattat ccatccccac actcacacgg tagggcagca gacgttgccg ccattgcacc 1812840
acgacatcac tttggtttgg ttctccctct acgggaacga tttggagatc ggcttccgca 1812900
gtcgggagac gtttgagatt ttccagtcct tgttccaaat cacgcagatt caacagatcg 1812960
ttcgagcggg tgggaaattt gttctggaat gctgcaatac gtcctgcatg ggtttgatca 1813020
tcgttagacc gatcgattcg tatggagcgc agatagctcg gtatcagggt taattgaagc 1813080
ttgccactat tcaaatcctg tggcgcagcc aagatacggg tcgtggtata tcccctgccg 1813140
atcaaagcat tttgtgctaa ggacatgatt tgattaatgt tgcccgcatg cagacacttg 1813200
ccagcctgaa aacccgtttc gcgcaaggca cgttttaggg caaactgaaa ccgagcatgg 1813260
tgttcgcctt ccaacaccac ttcgttaatg gcaaaacacg gttggctgct gtcatcgccc 1813320
atcaactgat taaccgtttc ccccgtgttt ttttgatgca aacgcacatc gctttcaggc 1813380
tgcatggttt ggcgcaactg ctcttcgcgt tggcgttgct gaatatcttg ctgcatacgg 1813440
atttcggcag ggttgggggga ggccaacaaa gtagcaggag caatgatacc tgccaataag 1813500
cagcaccaag acaaaaagcg aatattaggc aaataggata aaggaagttt catggcatgg 1813560
tcgcaaaatc aataaaatca tcaatgggca ttcaatacac gattgcatca agttttaaaa 1813620
gttaaaattc tcaaaccctt atcgctccct ttatgataca aggcgcgtac tgtcgtacta 1813680
ggaaataact ggcgcacagc tgagcgcatt tgatgactgt gtcccaatgg cgcaaaccat 1813740
tgaatcagcc aaatattgtc gccacagttc caatcgctgt tgtcacgcaa atggcggtca 1813800
gattctaaat aatgcgcctg cgccacttca tcaaaataag cccatgagat ataaccgatt 1813860
ggttgggtac ccttgcaaaa caaagcgaac tgcccgtttt ttaacacagg caatatatac 1813920
gtcatcatct ccacaatagg tacttggcga tgcgtaggcg actgatacca tagccaagtg 1813980
atggcaccga gtgcttcgct ttcgttccat tgttcattgg ggtagagttt aggagagatg 1814040
atgtttaagg gtggagtgat gggcatattt taagttaggg ttcgtgttgg ttaaaacaaa 1814100
aaatggtttc agcctgaaat gaaatatttc acgttaaaac atagttttag cacatgagac 1814160
tgaactgcgt gggcaacgag ttgcccaccc taccatgtaa cagttcgcat taattgcaca 1814220
ggctacgctg gttattgcac aaacaaatga ccatactgct tatctttacg aatattgtat 1814280
ccaccgccat ggtgaataca ctgataagaa atatgttgcc caatttcact aaatgagact 1814340
tcggtaatag atttatatcc taattgattg cctaattcat gcaaaacaaa ggtgagattt 1814400
tcagtttcac atgtgtcaac ggctttctcc cagcgtggat taagtttgga gcaattccag 1814460
tctgcacaac taccccaaaa agggcaagct gttagcattg ttgagcagaa tgatattagc 1814520
actaaattag aaagggtttt cttcatatct ctctccttta taagttattg tgtgttacaa 1814580
cataattagg atttttaatg cactgatagt aaatatccat tactgttttg tttttgtact 1814640
tttcttcatc attctcaaaa tagatattca gttttactaa tgccttatca aggcaattca 1814700
taatttcttg gtgttcctta tcagtagttg tccagttact actagtaccg tacaaggctg 1814760
taccgactac gacagtacct aataaggtag tagcgcaagc tgttagacaa aaaacagatg 1814820
aaagaaacag agaatattta gaaagcttct ttttcatatt tttcattatc ttttgttttg 1814880
tttggttcta ccagtatggg tttagatttg ttaattggct taccttgagc gtcatattca 1814940
acttcacccc atatttttac atagtcatca aattcttttg tacctgcttt tggcacctcc 1815000
gcaaaataac tgctatgcga atagcacaac cccttacatg taccttgtgt ggtgtcattg 1815060
gttcctagca agaaaggtat ccatttgttc cccacaaaat ctttatcatg cacgattgag 1815120
taggatccgc tgttataagt tttgccgtct gcacccgtat aggtatagcc gtttttctgt 1815180
aaaacatcgg cgtaatcatt ctgcacattt gtggctgtac catagaaacg tgcttttctg 1815240
attggggcaa tgccattttg tttttgattg tttacccaat cttttaagga aacgcttgct 1815300
gtaattcccc cacgactgtg attactggta tcaaccgacc agccgttacc catttttga 1815360
acctctcgat aaatatcttg attcagtttt tctgaattgg ttttgggtaa atagccttgg 1815420
aacactttat tatttaattg gtcgtaaccg acatacatca gttcagaaac aagacttgaa 1815480
ccgagccata aaaaatcttt tattttgtta ttagaatcag atttatattt ccctgttgga 1815540
ggattcatga ctgcaataac accactaccg tttgtactat tacgattttg ttttgctgcg 1815600
ttgcttaatg aatcttctcg attattgaaa ataccaggat tagacacagt aataactttg 1815660
ccatttgtgt cttgaatgct tgccagttct tccttgctta aatcattcaa tgaccaaatt 1815720
```

tgtctggtat caaaggatac tttacctgtt ttttcatcta ttatcttctt atggaaccac 1815780
atttctttag gtgcagttgc agaccttacc gctctatcgg cttgggttgt ggcaaacata 1815840
ctccaagcca tatcagaact ccctgctccc caaacgagtc ctaatcctga atcctaggcg 1815900
gtaatgacga aggatagttt tacaaaagtt tcggcctaca atttataggt ttataataat 1815960
aaaaatatcc atcaaaaaaa tgtgattttt cttttttaa aagttgcatc ttgccattct 1816020
tgtaatcaca ttcgtaatat tcaacatttt tcaaatctga atcaaataga tattgaggta 1816080
aaacattccc ttccttatct agttctatta attcctcaaa aactatctta cttttatgat 1816140
aaaaaacaat tttttggaaa tctgaattga ttatatacag ttttacataa tcccaattaa 1816200
aattcgttat atcactaatt aagaattctt ttttattata attaaaactt atttttctta 1816260
ttacattctt ttcaaatata aagaaaataa caatagtgat taataaagaa aatatataaa 1816320
gtatattttt attcatcttt tatcttctcc attgtctggc tgaaatttgt gctctgattt 1816380
gttttctcct ctgctgaatt ttaaatccgg caccagtatt atctaagtga atcaatatat 1816440
tgcttgtatt ctggtaatct ttttcagata atctttttct tacaacatca taatttccat 1816500
tactgtttct ttctgcttga taaaaaccat tatttttata agtttccaga attaatccaa 1816560
ctaactcttt tgtgtttaac ctattggtat ttaatgctat ttctcttcct atttcgttat 1816620
ttagttgatc aatcatttca tctgctttat ctaatgaaag attacgtctt atagaataat 1816680
taattttttc cccactttca tgactatttc ctactttaac agcaatatct ttgccaaatt 1816740
ctcgtgtgat ggttgcttgc cataaaacgt gcctgaatgc attgccaact ccaccttcac 1816800
caccaaattc actattagga aataaattta attgaaaagt tgaagcaatt gttgaaatat 1816860
taggctctaa tgtagagcca ggatctttat gtacagatcc aattgcaata gcaattctag 1816920
ggtgcctaag agcaaatttc acttgctcta cagtatcatt attctccacc gcactttgcg 1816980
cattcaggct gccttgcgct gcatctgttg cgctgttgcc gactgccgca cccgtagccg 1817040
tacccaatac atttgtaatc gctgttacag tctctttctc ttccgccgtt aagtcgcttc 1817100
ctttttcttt gccgtataac catttgctga tgtaaggcgc agccgcttcc gacccgcccg 1817160
cactcaatgc tcctgctaga gcattgttgt ctcctactgc ggcaaccgct gctcctaata 1817220
ccgcgtgggc aagaacgtgt gcggtttctt gactggcggt tagtttacca ttcgcgtttt 1817280
gaccggctaa atctttaaag tgctgtccaa tcgcatacga tacggctggc gatgcggtag 1817340
ccgcagcgat gcccgctccg ctttgggtcg gcgcagctaa acctgaggct aacatgttga 1817400
gaatgacttt gccttgttgc caattatctg cttttgctgc cgcatcttga gcttcatggg 1817460
ctttgcgttt ggcagtttcc atatcgccat tggctaatgc ctcggctgct gctgtttcgg 1817520
ctgcttcttt gtctgctttg agtttgtcta aatgttggtt aatctcggta ttggcttgtt 1817580
gaacattttt actaaaatct tggctgacgg ttctttgtaa atccagttca ctttgcaccg 1817640
cttctttgtt gaaggtgttc ttcaagctgc ccgaatgtcg ttcggcggtg tctgtggtta 1817700
cgtttgtatc aatatcggct ttggtttgtg ccgctgtttt gcctgtcagc cggatttgtg 1817760
cggcttcgtc ggtgatttga atgttgcggg tgttgatgcc gctttttgtg atgctgcttt 1817820
gactgtcgct gtcgctgcca taacccactg atgaacttgc gctgttttta tcggctacgc 1817880
ttgtcaggtg tttgttttga ggtttatttt gtgcgccctg tcccagtgtt ttgccgctta 1817940
tggacgcact tgcgcccaat ccaaaacttt cgcctttgta ttggctgtgg tttttgatgt 1818000
cgctatgggt gagggtggcc gtctgaaagc ggtttttacc cttgtcttct gcgctttggg 1818060
tactggtgat gatgccgcct ttgaggtctg tatggtttcc gaccttgatt tgatagccgt 1818120
cttctccggc ataaataccg ctttgctcgg ttactgaaac atggtcggct cggattttgc 1818180
tttggctgta atcgccaccg gcactgaagc cataacctac ggtaacttgt gcactggcgt 1818240
tttgttgttt gctttgatag gtttctctat cttgtacgct ttgaatactt aggtttttgg 1818300
cattgacttg tacgcctttg ccgcgtactt gcgcgccttt gatggtagtg tcgccaccgc 1818360
tttggataag ggtttggctg cctttgtcgc cgatatggct atggcggtgg gtgatgctgt 1818420
cgccattgcc gtagcctttg ccgacattgc cgcctgcggt aacgcctaat gaccagcctc 1818480
cttgtccgaa tgatacggca gcacctgcgt tccagcctgc cgatttgttt tggccgcgtt 1818540
cggtattgct ttgctcggct gattggagtg tgatgtcgtt atcggcaatc aggattgtgc 1818600
ctgctttgcc ggcaacatct gagcctgcga tgttgatatt ggattgttct gctgcgcctg 1818660
tggcgattaa tgtggtttta ccacctgctt gaatttgact cgcttgggct tgattggctt 1818720
gaacttgggt ggtttgtcgg ttttgctgtt cgccgtaggt tatggagatg ctgacttgtt 1818780
tggcattggt tgtaccattg gctaagtttt gtgcactctt acctgtttga taggcttgcc 1818840
agcctgcatt ggcagccgcc atggcattaa cgcggtcgtt tttgctttgt ccgacttgtt 1818900
tgctgctttg tgctacggca atcgcttgtt gtgccaaatc ggtaacgggc gaactgaatg 1818960
ccaccgttag gcctttttgt tcataggttt gggtggtatt actgtttaat ttgttgtgtg 1819020
ccgcttgaat gtctatgctt tgggcataga tggtattgtt gccttccggg ctggaaacgg 1819080
tactgccgat ttgttcgtag tgtttgcctg caacaatggt ggtatcgcct ttcaagctgc 1819140
ctacggtact gcctgtatgt tcgttgcttt gggattggtt ttcttgtgtg tttgtcttgc 1819200
tgccaatagt gaagccgata cctgcactca tcaatcctga tttctgggtt tgatgatagg 1819260
tttcgctttg gctttgagtt tgggttgtac caatgcgaac atgattgcct gcttgaatct 1819320
gggtgccatt atcggaaata acattgctgc caaggatgtt ggcatcgttt cctgcctgca 1819380

atacaacttg cttgccttca aaggtgctgc tttgggcggt ttcgtgatga ctttgggctt 1819440
tatcggtaat gactaattta ttgccaccac cgcttctgcc tgtgtgtttg gacgcatcat 1819500
caacatgggt cgtgttgatg cctgcgctga tgttgatgtc atttttggca gacacagcga 1819560
gtgtaccgtt tgcgctgctg acttcggcag ctttggcatt gaggttattc cctgacaata 1819620
gggtaacatc gccttttgtt tgaatgctgc tgccgacttc gttcgttgaa ccgcgaataa 1819680
catggttatc ggcatcaaaa tgggttgctt gatgtttgct ggtttgtacc gtatctaggt 1819740
taatgtcgcg ccctgcttgc agccgggttt gcccttgctc tgattgattg ctgatttgac 1819800
cggcaatgat gttgatgtct tttcctgcct gcgctgctaa aacacctttt tctttgcctg 1819860
tgatataaat acctgccatt cggtctaggt aggtgctgct gccttgtgta ttttgactgc 1819920
tggcggtggt gctttggctg ttgatgttgt tgcctgcgtt gagcaataat gtctgttcgg 1819980
cagaaagcat gccgccaata ttattgatgt cttgtgtggc cgtaaccgct gatttttgcg 1820040
catgaatacg cccaccgata ttgtctagcg tatcggtatt gataataagc gcattgcgcc 1820100
ctgcaatcgt gcctgagttt ttcaggctgc ctgaaacatt gatttgtgta ttgctgcctg 1820160
acaacaatgc acctttaccg tctatgtcgc catttttaac gcgtacataa acctgtggca 1820220
ccaatacggt ttgtgtgccg ccatcaggaa gcttaacttc tttttgtacc aaccaaacaa 1820280
tatcgctggt cagttgcgct acttgctcgg cacttaatgc aatgccaacg ctgagattca 1820340
tcgaacgtgc cgcagtcgcg ccattatcca ttaaggcttt aaattgttct tcgtcgtttt 1820400
gataaccgtc taaacgacga tgccctgtca gctctgcgat ttgttcattg attaaacgtt 1820460
gctcgtaata accatcaccc aaacgtttat gtaaattgtt tgggtctagt ttgaggctgt 1820520
ccagcatata gtcactaccc aaccattgac ggtagttggc aaagcgtgga tcggtttcaa 1820580
caagatagcc tttattgaca ggattgataa tgtataagct gctgctgggt aatggggtaa 1820640
aagaattgga cgtatagggt agcgaaatac cgttgctttg cggcaactca gtgccttggc 1820700
tgggcgcatg atggcttaat gctttgcgat gcgattcata ggcaaatgaa cccagtgaaa 1820760
tgttgcgtgt gatttcctcc ggcaaagtgt aattttgttc gctatgtccc gttgagtctc 1820820
gtcctttatg tttctcacgc caatagctgt gtaatttgcc attttcactg aatactttct 1820880
tttcgccaaa ggtttgctcg ttatgcaaac cgtctttttc tgtttgtaca atgagattgc 1820940
caccagcaat gatttggcta tcggtattaa atacttcttt accatcaatg gttaaatcat 1821000
tacctgaaat gattttggct ggcgcagttt gggtaacttg ggtttttttgg gtgacttttt 1821060
cataatcgta tttatgccaa ttttcatgcg ccgctccatc aggggtgcgt aagtggtctg 1821120
attcatcgtt atagacagac cagcctaatt catgttgcgt gccttctcgc aataattcgt 1821180
gtcgtccaaa tgcttcgtaa tcaacaatat gctcgcgccc tgtttctacc aactgcgttt 1821240
tcaaatgctc attggtattg tgcagctttt ctacacctaa acgcattttg cctgcagctt 1821300
caatggttgc gccggcattg tgtatccttt gggctttgcc tgtggcttgg ccattggtat 1821360
ctaatgcgcc gccaaccgcc atatcgttac cgctgtaaat cagactgttt tcacggttgt 1821420
ttaattgtcc gatgcctaaa ttcaggtttt cacgtgccgc aatggcggca cctgtaccgt 1821480
tttcatcttg attgtctaag cgggtagccg caatagcgat attgtcgcca taaatccgac 1821540
ctgtaccgat attattcatt tgcccggctt ggattttggt ttgttgtccg tcaatcaagc 1821600
ctctattggt taaattgtgc tgcgtgccaa tgtctgtcgt accgccggat tgaatgttgc 1821660
cttgtgctgc attatcaagg ttatttgctt taatccgaat gcgttttcct gcttgcaaag 1821720
tatgtgaatt tttcaggctg cctcgtgtac tgagcgacaa ttcattgccc gccacgatat 1821780
tgcgttctac ataaaaatca tcttgtaacg caatatccag tttattatca gcggcaagtg 1821840
tgccgttgtt ggataacgat tttgcctgaa tagcaacatc acggcctgat tgtatcgtgc 1821900
cattcgtatt atcaatgaca gcggtagatt gctgaccatc gtgaataatc agttgttgat 1821960
tggtcgctat ttcgccattt tgattgttca ggctgcctga aacggctaaa tccgctattt 1822020
ctgctgataa taacttgcca tgagcgttat ccagttgatc ggtttcaatc tctaactgtt 1822080
ggcgtgttgt gatgttgcca ttttgattat tcaggctgcc ggcttgaatg tggaccgcat 1822140
cactgataat tgttccattg tgattgtcaa acgccgaacc ttttgcattt aactgatgaa 1822200
tgtctatttg tcctgcatta tttaaacctt gttgcgcact aacatctgtt tgaccattgg 1822260
caataatact gcctgaatta tccagtgcac catgagtgcg aattgtccca tcagcaaagg 1822320
taggcgcagt tatgtttgat atagaaacgg ttgcagtacc cgtacctgtt gccgttgttg 1822380
gtgtggtagt ggatgaatgg aaagatgcat tgtaactatt gccggtttga ttgcttgaac 1822440
catttgacgc ggttggtgcg gtatcttgta aacccatgcg gccacggtta tccattttgc 1822500
cttgtgcatc aatatggagt ttttgtgaac ctgtttgaga gagtttgcct tgattattaa 1822560
gtgtgtcggt atcaatagcc aaacgagcgg cttcaatggt gcctgatgtt tcatttttca 1822620
ggctgcccga attgtgaatc aatatttcgc ctgaggacaa taatgtgcca gtgttttgaa 1822680
tcgactgact gtgaatttga gtgccttgtt gcgataccgc cgtaccgctg ttttcaacgc 1822740
cctgactgcg gatattgact ttgtgttccg ctgtattatc cgtatctttc gcattggcgg 1822800
cagccatcgt gccactattg actaaacggc catttgcatc aatcgccaca ttaccggaag 1822860
aagcaaacaa ctgcccttga ttacgaatgc ctgcttgctc ggccgtactg atcaaggtga 1822920
ttttgttggc atacatacct cctaatttgc ctgtatcaat cgcaaataaa gggatatgtg 1822980
tgccgttgtt ggctgtattg tttgacgtat tggcagcagc attattgaga ataggcgaat 1823040

gtgcattacc tgttgcgacc acatcgtttt gtcccgcgac gacacgaaca tcttgtcccc 1823100
atacgggtgc atcaattttg gaatgataac tgagaatacg tgtgaaatcg gtatcacggg 1823160
catccaaacc gtgtccggcg attacaacat tgccttgcct tatcttaaag ccgctaaggt 1823220
ctcctgcttg atattgcggt tggcctgtcg tcaaagtggc acgggaagca ttgataaaac 1823280
caccaccatt gactgcaatc cctgccggat tggcaataac gacttctgca cgtcgtccgc 1823340
ccacttcaat atagccattc atttgtgaag aatggctgct gttgatttgg tttacaacca 1823400
cacgtgcttc gccccttgcc aaccaaggat taccttgaat ccaaccgcct agctgtgttt 1823460
gggtgttgct gcggctgttg tttaaaatcg ccccgcgatt acccacatca aactgggcgt 1823520
attgattaac agaaacccct gccgaagtag gggtttgaat attgacttgc ggtatgccgt 1823580
tacctgtttg cagaatcgtg gcttgttgag ttttaggagc agctttatca gcaataatgc 1823640
catcagcaaa agcaatattg gccgtaccta cagccaaaca taaagaaaag cccaataaag 1823700
aaaaagaaaa gatatttgaa cgacaaacag gtgcatgagt agtaccaaaa ggaacagatt 1823760
tcacatgagc gctgcctgaa tcactatcgg cacagctttt accttcgcgc ttggtagttt 1823820
cagcaacggc taccacagcc ccacgtttgc ggttgaaaat tacacgatag agagttttat 1823880
tcatgatttc agttatttga tttttataga gttattagaa aaaattggat agtctgacca 1823940
ttctagatca aggattttgg cgagtcaatt accgccattt tactgccatt tgtttattaa 1824000
attagggact ttactagata acggttaaaa atcccattcg aacgaaatgg caaggtttat 1824060
accgtcgttg tccctaatgc gcaatcagca acattattgc cgattatccg agagaaagtt 1824120
aagtctgatg gcattgtgta tacggatacc tttcgtagtt atgatgtact tgatgtcagt 1824180
gaatttagcc atttacgcaa gttttccagt atttgactgg caatttaaaa cagtcggatt 1824240
ttgtcccatt tgttggccaa gtctttactt gcttggccgt ttgaatttaa aaagcagtct 1824300
ttctactttc cgaccttttt ttctgttgta aggtctataa tccaatagca ttcccaaaga 1824360
gcattttgga cggtggcgga ttcgcatttg aagtgcaact ttccctaaca gaaaaaggcc 1824420
agtatgcggt agcatacggc ctttcctgca agaaagattg ccatgagcta cacgcaactg 1824480
acccaaggcg aacgatacca catccaatac ctgtcccgcc actgcaccgt caccgaaatc 1824540
gccaaacagc tgaaccgcca caaaagcacc atcagccgcg aaatcagacg gcaccgcacc 1824600
caagggcagc aatacagcgc cgaaaaagcc cagcggcaaa gccggactat caaacagcgt 1824660
aagcgacaac cctataagct cgattcgcag ctgattcagc acatcgacac ccttatccgc 1824720
cgcaaactca gtcccgaaca agtatgcgcc tacctgtgca aacaccaccg gatcacgctc 1824780
caccacagca ccatttaccg ctaccttcgc caagacaaaa gcaacggcag cacgttgtgg 1824840
caacatctca gaatatgcag caaaccctac cgcaaacgct acggcagcac atggaccaga 1824900
ggcaaagtac ccaaccgtgt cggcatagaa aaccgacccg ctatcgtcga ccagaaatcc 1824960
cgtatcggcg attgggaagc cgacaccatt gtcggcaaag gacagaaaag cgcattattg 1825020
accttggtcg aacgcgttac ccgctacacc atcatctgca aattggatag cctcaaagcc 1825080
gaagacactg cccgggcagc tgttagggca ttaaaggcac ataaagacag ggtgcacacc 1825140
attaccatgg ataacggcaa agagttctac caacacacca aaataaccaa agcattgaaa 1825200
gcggagactt atttttgtcg cccttaccat tcttgggaga aagggctgaa tgagaacacc 1825260
aacggactca tccggcaata cttccccaaa caaaccgatt tccgtaacat cagtgatcgg 1825320
gagatacgca gggttcaaga tgagttgaac caccgaccaa gaaaaacact tggctacgaa 1825380
acgccaagtg ttttattctt gaatctgttc caaccactaa tacactagtg ttgcacttga 1825440
aatccgaatc taaggtcatc tgaaattaaa tttagttttc agacaatctt tttcttcaat 1825500
tggaacgtgg agttacattt tcacctaaac tatgcacgct agatttatag ataaaccatt 1825560
cagacagtcc aataaacatt atggttggga ttaccaaata ccaaaaacta ccgactcttt 1825620
cagatgaatt aaataaagaa aagtacccat atgtataaaa aacaatgaag gttaatatga 1825680
cagatacagc aagttgattt tgaaataccc atctgaataa agttgataat atcactggaa 1825740
ttagaaacca taacataact ataaacccac cccctaccat cccaaatata aaatctatta 1825800
cccctaaaaa agatagttta aaatcattac cccataaacc tgaaaaaatt aaaaggccaa 1825860
aaaatactcc acctattaag aaaatagatt taatattttg aataagataa aataaataat 1825920
ttttattcat ggcttgcatc cctgtttctc agaatatccc ataagcttac aaccatattt 1825980
aattctagat ttgtaatcta acatattaat tccatcctct attttttttcc tctcatctaa 1826040
aggatttgga attattgaaa atatcttatt tgatgttacc cctttaatca atgacaaata 1826100
atccttctca ttactcaaat atttttggtg aataggctgt aataactttt gctcgctttt 1826160
caactgttga atatcccact ccctaggatt agttttttta ttgaacaagt ctctaaaatt 1826220
ataaaatcca tttactatca gtgcattgcc tgacgcagcc ctccattcat aaattggagg 1826280
attaccaaca actttacctg ccgcaaaagt tatataagaa gcttctccgt ctgcccctaa 1826340
cccagttata gcagcacgcg atacaaaatc agctccacca aaccatcgtg cttttgaacc 1826400
caaattttgc tcataaagat tgctggcagc aaagaaatct gcacgattgt caatggtgtt 1826460
gaaatacttg tcaaatcttg tagatgcccc ttgtgagtta tataaatagc caaaaacttc 1826520
tttggcaacc cgtgatacat ccgtaggcat atacttccac gcagcaggca tggcaatata 1826580
tttaatagac atattaatgc cgtttctgca accatcgccc aatgggttcc tagaacagac 1826640
tttttgaatt tccgtatcaa ttactttgct aaattgttca tcctttttga tggcatttac 1826700

tttttccatc gtagtagaac aagttttacc ggaaaggcat tttgtcaatt tatccattct 1826760
ttccttgcta agcgcattac tctctaccgc cacagcagcc gcattcgccg cagcattcac 1826820
atccccctta ctcaacgccg caaccgtccc tgctgccagc ttcgccttag caatgatttt 1826880
tgcccggtct ttcacattca ggctgcccgg gtctctgccg tccagcaggg cttcgccgag 1826940
gatttcaccg accgccgctc ctatcgcgcc atcctgacac ttgcccttat tcgccgccgc 1827000
agccgcacag cccgctatgg catgggcaat cttgtgggta atgtagtgct gatccaactg 1827060
tttgatttta ctggctgctt ctccatgcgc agtattcacc aaagccgcaa ggatattcgc 1827120
ttccagattg tctttcaggc tgccgccgtt gacagcggta ttaatcagtg cggcactgcc 1827180
cgcattggcc aggttgacgg tcaggttgtt gatccactgc ttatcgctga cattgttcag 1827240
tgccgaagca ccgattttgt cggctacgcc tgcggtagcg acggcaacca tcagattttt 1827300
caccgtgctg cttctgccca gctctttcag ggtgttaccg atattgcctt tgttgttgat 1827360
gagcgatacg gaagcctggc tggccagcga ggcgaatgcg gcatcggttg ccgctgcggc 1827420
cgcgccgttt aagcccagtg cggctccggc tcccgcgccc gcagtaacca cggtaacagc 1827480
cagcgcaata atcgctgctc cggctccggt taagccttcc tgtttatagt cccatttgtc 1827540
gtacgccagt tgtacctggt tccagttgac gtctttggtg acttggagct gtttcaaata 1827600
gcaagcgtag gggtgtctt cagcacgcac gcatctgggc agtatatagg gaatctgaat 1827660
atttacttgc ataacaaatg ccgtctgaaa aattgtgagc ttttcagacg gcattgagcc 1827720
gtaaatcatg gaacgcgtgc gtgctgaagc acacacctta cgcatggatt ttaggtttca 1827780
tgcaggctac agcttgctgc tattcatcaa attgcggcca ttgaaagtct gttgttttac 1827840
tttcacctct caacagtcta atcatatcgc ttttgagaaa ctcaaaaaaa tttttaatat 1827900
taccaacata gagcatagct tcacatagtg aactacatgc agatttaatg tcttcattgt 1827960
caatagcata ttgatattcc ttcatatgct gaaaaaaaga atcaaagtct tcttctaatt 1828020
catcattcca atcagatgaa tagttagaaa gccattgtaa gtcaagagga tcttcactat 1828080
tcaatttttc agttgtggct ttctcataaa gatcaaatcc ttgtttaatt cctaactctc 1828140
ttaaactttc ttttactaca ttaaaatttt tcatctgaat caccttattt aagattcaat 1828200
tttcgccctt gccctgctaa tgtcttagct taattttgag cgagttttag gtttcatgca 1828260
gactacagct tactcagcac acacgagtct aaacagtata cagggaatct aaatatttac 1828320
tttcataaca aatgccgtct gaaaaaattg agcttttcag acggcatatg gccgtaaatc 1828380
atggaacgcg tatactgaag cccacacctt atgcatgggt tttagatttc atgcaggcta 1828440
caacttgctt tctattcatc aagagatggc catgaaaaac tattcttttt atactcagca 1828500
ctcaataatg ttgatatatc agtttttatt gaatcaaata taagagatag attacctgcg 1828560
taaatcatag ccataaataa agaattactg gcaattttga aatttttatc gtttagggct 1828620
aattggcata cttccatata atctaaaaag ttttttaaat cctccttaaa ttcattatcc 1828680
caattcccgt ctgaagtata atctttaatc catttcatat tagctgtttc atgattaact 1828740
tcttctgata cttttggatc tgtcaaatca aacccttgat aaagccccac attaatcaag 1828800
attttttta tatcattcaa tgtttccata aaatttccta ttttaagttt aatttacgac 1828860
cttttgctgc cccagttttc atttggttaa gcgaaccatc catatttaga acaaacttaa 1828920
agttcccatt tttatcaaaa acctctaaat gatttttatg ttggccatct aaataaaacc 1828980
tatcaccggt ttttaataac ccttggtttc tttttaccaa gaagacagac tgcccttgct 1829040
gcgtcggaag cgttgtcttt tctgaaattt gagccagctg ttttccaaaa ggattatttt 1829100
tcatgtatgt actcatattc ggtacagcac ctttattagg gatataagga cgattttttt 1829160
ctaaaacttc cttgaccttt tgtgccgctt cccctttatt agcgcgattc agctctgttc 1829220
cgacgacaat atcaataacg gctttggcat cgttccaatc caatgtttcg tcgaataagg 1829280
tggtcaggtt gtcggctaaa ttataacctt cgtctttcaa cgtctgtttt aaatctctaa 1829340
cgttgatttt cccgtttttt aatccttttc tggctacctt ataaaccact tttgcagcag 1829400
ttacaacagc tttaaccgca ttattttcta ccgcgttttg tgcggtttgt gcagcagtat 1829460
tgacatctcc tcccgttacg cctgcaactg tacctgccgc aagtttggca taggcggtaa 1829520
ttttcttaac ttccagatct aattgttccg ggtcatatc gctaaaatcg gtatttttaa 1829580
ccaaagcctc cccgacaatc tcacccacag ccgcaccgat cgcgccgtcc tgacatttgc 1829640
ccttattcgc cgctgcagcc gcacagcccg ctacggcatg agcgattttg tgggcgacat 1829700
agtgctgatc cagtcctttg atcttactcg ccgcctcccc atgcgcggta ttcaccaatg 1829760
ccgccaggat atttgcctcc agattgtctt tcaggctgcc gccgttaaca gcggtgttga 1829820
tcagcgcggc actgcccgca ttggccaggt taacgttgag gttgtttacc caaggggttt 1829880
cgctccaagt ggcaagggaa gaggcaccga gtttgttgga tacgcctgcc gttgccgccg 1829940
ctacaaccag attttttacc gtgcggcttc tgcccagttc cttcagggtt ttgccgacat 1830000
cgcctttatt gttgatgagc gatacggaag cctgagaagc gagtgaggca aaggcggcat 1830060
cggccgctgc tgcggctgcg ccgtttaagc ctagtgcggc tccgactccc gcgcccgcag 1830120
taaccacggt aacagccagc gcgataatcg ctgcaccggc tctggttaag ccttcctgct 1830180
tatagtccca tttatcgtaa gccagttgca cctggttcca gttgacgttt ttcgctactt 1830240
ggagctgttt cagataggca tactcgggct gtttggccag cttttcgatt tcggttttca 1830300
gattgccttt ggggatgtcg acaatgtagc cgccgggagc agagagtacg ggcgcaacgg 1830360

agcctgtgaa acttggcagt tgcaaggttt cgatattgct gccgcgtccg gcctgtttct 1830420
gccagagggc agatttgcta ctgcttactg tttcagtgcg cacactactt ttgatgcctt 1830480
caagaataat cttggcatct gctcgtgcct gatcgcctac acctgcacgg atggctgcgc 1830540
cgcccagcgt ggtttcaaac tgggtgcctt gcagtttggc gtcccagcct gattgcaggt 1830600
tggccgattc tgcaactacc cttgagggca gggcggtttt catggtgtgg gtggtggtgt 1830660
cgtgcacctt gtcgtaggta atgccgataa atttgcgctt ggtacgggtg tcaagtttgt 1830720
cgtagttgag atcttccacg gcatagagaa ccagcccacg tccggcttcg attttaacgg 1830780
agccgcgggg tgcatcaaac aaggtggcgt gggcaccgat attgccgccg gatttgattt 1830840
caatatccttg tgacgcactg aggctcaccg ggtcggcttt ggcgttttta tgctctttga 1830900
tttcagtaac atgttttagt ttgtaccact taccggtttt atagctgcgt ttatcgaagg 1830960
tgtagagctc accctgtccg gcgtagtaga actggtcgcc gtaggattgc agtttgattt 1831020
tgccgttttc cgaactgata tccgtggtgc tcagcaggat gcggctgttt tcattggcat 1831080
acggcgcgct aatgctcaca ccggttttac ccgaaagttc tgcagcaagc gtaggaggtg 1831140
tcttcagcac gcacgcgtct gaacagtata cagagaatct gaatatttac ttgcataaca 1831200
aatgccgtct gaaaaattgt gagcttttca gacggcattg agccgtaaat catggaacgc 1831260
gtgcgcgctg aagcacacac cttacgcatg gattttaggt ttcatgcagg ctacagcttg 1831320
cttccataaa tcatttttat cagagctcgt aggtacggtt agccgccttt agcggcgtaa 1831380
ccgtacgaat gaaatgccaa gttgcaaggc cgtctgaaaa agttgaaaaa cagatttcag 1831440
acggccttgt tattttataa agtttgctga tatgcgtacg gttacgccgc taaaggcggc 1831500
taaccatacc tacgcttgct cataaatatc aatattcggc aaatcggcca aatctattgg 1831560
acacgcaata tcccaccaaa gccattctaa gtaataccaa gggtcttcag gccatattgc 1831620
ttgggcatct tccaaagtag gccatatgtc tttcaatttc tgcacttgtt cttttgaagt 1831680
tccagttaga ggaattccga taccgtcggt ataatcatgt aaacggattg aaccgtcatt 1831740
taacagttct tccataaaag aacagaaatt gttttttaag ttttcatctt gaatattaat 1831800
acccatttga tttttataag agttaaaaat tgaggataaa tcgatttgaa aatcaagaat 1831860
cttaattttt tgtttctcat ccacggtaaa atcctttgta aattattgga atttaagctc 1831920
caatttagta cctttaataa atgatggagg attctgcaaa tctaatgtcc atcgtgcttg 1831980
agttgaattc tctgtttttg aaaaatttct taatgcaatt tttgttcctg cccattctcc 1832040
agtactgata atgccatttg ctaatcttcc gtcaggcaaa actctaaaat tcggattctg 1832100
gccagtcatc tgccgataaa gcgcaaaaat ctcctgttcg cttacgccgc tgtaaacggg 1832160
tgctcccttt aagcttatgg cttgtacagg tttgatggtt tttccgttaa tggttatcgg 1832220
aatattactt gcatcaagca agcctgctcc tttccttact ctattgttaa atcccgtctt 1832280
ccatgcatcc agttgcgtat cgcgttgggc aatacggaat aaaatctgct gttctgcttt 1832340
ggctaaattt gccaaattgt ttattgtttc tttaggagca gcttgcttag ccgccttcgc 1832400
tttcgacaac gcccccacag gcgcttccca agcgttgcct accgttaccg cacccgtggc 1832460
gattcccgcg cccgcttcgg cagcctgagt gaccatgaca gtacaaccag aaggattagc 1832520
catgcaggtg ctgatagcta atttacccgc tgtaccgatc agcggagctg tccaacctgc 1832580
agcataaacc ccatagctgg taatcacaat cgggcctgtg atgccattac ggatattgct 1832640
tatccaaatg gcagcatcct tatcctgcgg attagtcatc gcacctgctg catgtgcagg 1832700
cataatacct tggataattt tttccagtgc ggttttgtcg ggcttctgcg gttgatgctt 1832760
tttcgcattg gtaggggtac tgtcaaaatt caaagcatta ttcactaccg ccacctcagc 1832820
cgcattcgcc gcagtattca catcgccgcc gttgagtgcc gccacgctgc cggcaataat 1832880
cttcgagtaa ctgataacct tatgcttttc cgcatcgctg agtgtagcag ggtttctgcc 1832940
gccaagcatg gagtcggcta cgatttcccc aactgctgcg ccaattgccc cgtctttaca 1833000
ttttccttgt accaatccgc taacacaccc agccaaagcg tgggcgaact gtttggcaac 1833060
ataatcgtcg ctgaaggttg ttttgatttt gctggcggct tctccttgga agctattaac 1833120
caatgctcct aatgcggcat tgcctaagtt gtctttcagg ctgccgccgt tgacggcggt 1833180
attgatacca gctgagatac ctgcattact gagattggta gccagtctgc ctccaaggtt 1833240
ggcaatagtt tgattgcccg tactgctgaa cagttcggtt cttaccttgc tgttcaattg 1833300
ggcaatatct gcgcccatct gatttaatgc acccgccgtc agggcagaag tgacaatctg 1833360
cttgaccgta tcactggtgc cgagatcttt caacgctttg ccgacatcac ctttattatt 1833420
gatgatggat acagctgctt ggctatacaa ggaggctaaa gcagcggttt gcatggcagt 1833480
cgctgtagaa acggtagtag ctgctgctgt cgttgtggcg gctgttccgg cagctgcggc 1833540
tgtactactt cctgaagcgg ctacaccgcc cgctgcggtt gcgccgtatc cataagtcag 1833600
tgcggttacg attatggtaa caatcgctgc accggctctg gttaagcctt cctgcttata 1833660
gtcccattta tcgtaagcca gttgcacctg gttccagttg acgtttttcg ctacttggag 1833720
ctgtttcaga taggcatact cgggctgttt ggccagcttt tcgatttcgg ttttcaaatt 1833780
gcctttcgga atgtcgacga tatagccacc gggggcggtc agtttgggcg gagtagggct 1833840
ttcgaagctg ggcagtttca gcgtttcgat agtgctgccg cgtccggcct gtttctgcca 1833900
tacggttgag ttggtttcta atttttcttc cgactggata cggttcacaa tgcctttgag 1833960
gataattttc gcatcggcac gggctttttc gcctacacct gcctgaatgt ccgcaccggc 1834020

cagcgtggtt ttgaattcgg taccttcgag cacggtatcc cagcctgaac gggtggctgc 1834080
agtttgggcg acgacgcgga caggcaattt ggtttcgttc agttcgtttt tactgtaatt 1834140
gctcttgcct accttgatgc cgataaagcg gcggcttttt tggacatcca actcgtgctt 1834200
gtggatgcct tcttctgcca gcagttgcag ctcttcaccc gcaaccaggg taactttacc 1834260
tgcaggggca ttgaagcggg tggtattagc ttcgatgttg ccgcctgcct gaagcgttat 1834320
gccgttggcg gtcagctcga cgggggctgg cataatcagg tggtcgcggg tgctggtaaa 1834380
cttggttttt ctgatgattt tgccgctttt acctttggtt tttaagaagg tataggcatc 1834440
gttttgtcca gcctccagta caatatcact atgggctttg atgtctatgc tgcctgaggg 1834500
agctttgatt tcggatgcac cgataataat acgtgcatca tcgagtgccg cagctgcatg 1834560
aatacttacc cctgtacgtc cggtcaaacg tgaaggcttg ttcagagcag ctttgtcgta 1834620
gtgactcttg taggtgggct tgccaatttc atattggtcg gttatgccgt caatcagaat 1834680
agcagccgcc tctgaatctg ctgcctttgg caatacgcct gcggcgtgaa ggttcagttt 1834740
tttggaagcg gtaatatcgg aaccgctgat ttcgatgcct tgtgcggaaa tcaagtcaat 1834800
attttgtgca gaaagcttgg cttgcaggta ttctttgcct ttgggttttt tacctttaac 1834860
ttccttgttg atggcttgaa tatagaaagc gagacggtcg cgttcttctt gcagggttgg 1834920
aatcagcttg cttttaggcg agcttttttt caactgcgca atctgctgtt ccaattcttt 1834980
ggatttttgg ttgagttcag ccgctttttg tgtaggaaaa taattgctga atgagttgtt 1835040
tacggcttcg atattcaact tgcctttggt ggtggcgaca accaagtttt taccggctgt 1835100
aattttagaa cctcttaaat ctgtttctcc tgtaaccaga cggatattgc cttttgcttc 1835160
caatgaggaa acttgagcac taggcgcacc tgcatttcct ccttttgcag acaacagcaa 1835220
ttttccgcct gttttgatgc tcaggtcggt atgcgcactg atgcggttgg caggttcgat 1835280
ggttaatgtg ccgctacctg cttcaatatt cagccgtccg gccaatggtt ttaattcggc 1835340
attatcttcc aaagttttgg tcgaaacggt actccaattg atgttgccgc gcttgactag 1835400
ggcggtaccg gcagtaaggt tgattgcacc cgctctcagc gtggtgttgt cggcaatttg 1835460
ggaatagcgc gcattgagtg ccaatacacc gttagccacc agcttgttgg cagaaggcag 1835520
tttgtcgttt tgccaaatct ggctgccggt aatgcttaga tgacggtgtg cgtaggcatc 1835580
tacttggttg agcgttaccc gctcgtgttg tgcattaaga tgcgtattat gggtagactc 1835640
cagcttggta ttttctatgc tcaatgcccg gtcggaatga atgttcaatg ccccgctttt 1835700
ggcatggacg ttaaggtttt ttaagtcggc attaccaccg ttgtttttga tgctgatgtg 1835760
ttttccgttg attgaattgc gttgttttcc gtcaccaagc tgaataccgt tgccggcaac 1835820
caacgtaata tctcctgaag atgaagtgat attggtattg tctgctttca gacggccttt 1835880
accaaccgat cctgcattga catcggcctt ggctgtcagg gtattgtgac cggtaaagtc 1835940
ggcattaccg ttggccaata aggatacatg accgtctgca gaaacagcat gaaggccgtc 1836000
tgaaacgata ttgccctgca atgcggtggt ttcgagagcc ttggctgcgt tcagcttggt 1836060
attgcgaagc tgaatattgc ctttggctgc ctgaatgtgc agattacccg agttggtacg 1836120
cagattggta ttggtaacat tcagcgagcc tgcctccaca cccatgtctt ttgaggcagt 1836180
gagggtttta ctggtgccgg taatatgggc agcgttatcc gatttcaaat ggatgctggc 1836240
ggcagacaaa tctttatcaa cattcaaatt cagatcttta cctgtatgaa catacagatt 1836300
gccgggagta ttcagattgg tagttttggc agtaatgtta tcgtctgcca gtaaagcaag 1836360
ctgcttgccg cccttgatac tgcctccgtt taagcggata tcggaggtaa ctgttgaagc 1836420
ttccaaagaa agcggtttgc ctgcttcgat gtgtgcggtg tctttggcat ctattacggc 1836480
ggaactgctg atggtgccgt tggataatac ggtaacatct gccccggtaa tgcgtgtgtt 1836540
attgcctaat tcggcgttgc ctttgctgga actgtatacg gtagtgccag tctgaatact 1836600
ggcctccttg atgacggtac ggccgtcggc cgacagagta gccgggcctt tggcattgtt 1836660
cacattagtt ttgctctcaa tcaccaaatt atgaccagca tttaataccg tggtagctgg 1836720
gcgactgccg ttattctgca ccacggctcc gttacgcaag ctgatatctt ctcccgtctc 1836780
aataaccaat aagcctttgc tctcgatccg accaccattg gagataaatg tgcctgccgc 1836840
tcctttttcg gtggtttcga tggagagata agtcggtgaa gcttcggtgc cgtcggcagt 1836900
ggtggcgatg cggccgctgt tttcaatgcg gcctgacgaa gtcacaatca attgcttggc 1836960
cgcttcgagt gtgccggcat ttttgacgcc tacgcctttt tcattggcaa tcagtgtgat 1837020
gctgtcggcg tacataccgc ccagtgcggc agtatcaagg gcaatagtcg gtttcgtacc 1837080
cgctgccgta cctgcactga tttcgccgct ggcgtaatct actttctgag gaccggtaga 1837140
aaccgccagg tttttaccct gtaatttccc ctgcaaagca actgcacgag caagtacccc 1837200
ggtgtagtcg gctccgcctt tatcattcca acctgctgct cctacggtca atgtgccttg 1837260
acgcacatca aatcctgtca gtgcaccgtc tttgccgatt tggggcgcac cggtagttaa 1837320
gatgccccga ccgacatttt taaagccgcc gccattaacg gtaatgccgt tggggttggc 1837380
aataatcacg tcggcctttt gaccgcctac ggtaacgatg ccgttgagtt tgctagccgt 1837440
accgcgtacc tcgttcaaaa tcaattgcgc actgcctttg accacaaacg gattattgtt 1837500
acggtcgttg tttaacactg cccctttgtt gtcaacatca aactgcgtat agcggttgtg 1837560
gctcaatccg cgtccattcg gagtttggat attcaccaag ggggcaccag tgttggtttt 1837620
aaggataacg acctgctggt tttaggtgc tgatttgtcg gtggtaattt gggcatgggc 1837680

aggcaatacc atactcaggg aaaccaaaga gcagaccaaa gttttaaggg tggttttgag 1837740
tttgccgcaa aggtcgcctg aagttttcag tgaaacagaa accgaactgc ctgcctgttt 1837800
acctttgccc tggctgttgg cagtttcggc tactgcaacc atggtgctgt gctttttact 1837860
aaagataatg cgatgtaaac ctttattcat gtctattcca ttttgaagat gaacgtactg 1837920
cgcgccaagt acgtaggtaa agtttgacgg tctgaggata aggaaagacc gtctaaatat 1837980
cagtaaaaaa ttcagaggtt agaaactgta attcaagttg aagccgtaaa cggtgttggt 1838040
cgtctgaaag cctttgggtt tatgaagcgg cttgccggca aacagatcat aagcaaacat 1838100
accgcctact ttatgccctc ctctgaagcc gaccactgca cccatcagct gcttgcccga 1838160
tacatattgt gcactttcgc cagatacgcg gccatagtcc gcaccgagat agaactgatg 1838220
gttcggatga aaataccaag ttaaagtatt ctgccagtag aaacctcgct ctccgaaaag 1838280
actctgctcc ccatcaaatc cgcgaacggt gtagcggctg ccgattgaca atttatcttg 1838340
ggcaaccaac ggcgttttgt tccattgagc ttgaatggcg gttgcgtaga aaaactgctg 1838400
tttgcctaaa ataaatgggg cggctgcgtc caaactggca gtaatgattt tcatacgaga 1838460
tgtacctgga agaatatcgc cgccgttttc ttccggtgca ggcatacttt ggcgcatgcc 1838520
ggtcccgcgt ttgtaagaca acttgccgtc aagctgccaa cggttgaggt aagcacggtg 1838580
gcgcaattcg gcttcccagc ctgcagagcg gcggcgttgt acttcgattt cggcatcgtc 1838640
gatgtattta taggtttggc gtgtccataa tttcattccg actgaagttt tatgaagtct 1838700
gttacgccaa agcatgcgct cggcggccag gctgctctga tattgtttgc cgttgtaatc 1838760
gtaattgacg gaatagcctt cggttgcttc gtggtaacga tgtccattgt gattaaaaga 1838820
aaacagccat ttttttacgg gcaccgaata atgcacgctg taacttctgg atccgctttc 1838880
agtttccgta ccggtggcat cagtcaagtc cgttttgtgc gccaaaccgc gtccatatga 1838940
aacataaaac aaatcgctta agcccaaagg gttatcgaac gataaagcga catttccttg 1839000
atatttgccg gtcgttttgc cgcccgcatc atctataccg atactgaacc gtatgggttt 1839060
attctgctgc catttgatct gtaaatcgct tttgccttct tcttcggacg gtataatctg 1839120
aatatctgtt ttaacactcg gcaaacgacg caggttttcc aagccctgct ctacatcgcg 1839180
aagattgaga attttgttcc tatataaggg aaatttgtta ttgaatgcac taatactgcc 1839240
ctcggcagac ttcccatccc gtttttcttc atagcggata tcccctattt cgcctgctga 1839300
tacccgtaat ttcagaattc ccgaatccat attctgtggt tggataatag cttgggaagt 1839360
gaggtagcca cgcacgatca gtatctgttg cgcggctttt tgtagcctgc tcaaattatt 1839420
ggaacctaaa cacatcccag ttttaaaagc tgtttctttc atgagcacag aaggaagaaa 1839480
agaaaatttg cgcaccgtct tatcatctaa actaatgtaa tttacccgag tacacggtgt 1839540
ttcatcttca ctcaggacat aattgttctt ctccaatggt tgctcgaaac ggacatttgc 1839600
atcagttaac aattcagcat ctatgtgctg ctgacgctgc atggaacgga taagttctgc 1839660
atcgttttca tcggcagcta aggttttaag ggtatgaca gccaggataa ccaacagaca 1839720
tggagcagga aaaaatttca tgacatcaat attattttag caatatttac tattttgtca 1839780
taaatttaaa agtatttaca gttatagaat gagacctttg caaaattccc caaaattccc 1839840
accaagacat ttaggggatt ttggggaatt ttgcaaaggt ctcggacagt attttgaacg 1839900
cagtgcgcgt aaattcgtat ggaaaccatg aaatcccgcc acagccgcca gacatgccaa 1839960
gccgcattct gatatttctg tttgcaggat aacaggcagc tttttcttta agcccaaaga 1840020
caggttttgc agatggggca tagatttcct ttttgaaaaa tagggattag gaagttggat 1840080
gtattttaga aaggccgcct gaaaaggttt cagacgacct tttgcgacta gctgctattt 1840140
tatttaaagc ttttctctaa caaacgagct aatatttttc ctgtaataaa acagataaaa 1840200
aacagcatcc aatacgtcag attggaaaaa tcggtcgtat agagaatcaa catataaaga 1840260
agcagcatga tgccgagtgc gatgaattga taatgtttgg caaacatcat gacctcctca 1840320
actattaagg caaaccgcct gaatattctc gttcaatcgt ttcggcaatt tccctataac 1840380
gtcgatacca tgaccagtcg aaattttcaa tggcatggct cgcaaacgta ccaaattcag 1840440
gcatccctat gcggctacct gctaaagctc cgattgtagc tccccaacca ggcggattac 1840500
tgaacgtatt gtttggcaat cctaaagatt tatcaggatt tcccgtatcg ttaagccctg 1840560
cagattcgca aatttcgcaa taatatgagc tttgttgcgc attacctgag cctccgaccc 1840620
aagtcatttc atgaacacat atagtggatt aaatttaaat caggacaagg cgacgaagcc 1840680
gcagacagta caaatggtac ggcaaggcga ggcaacgctg tactggttta aatttaatcc 1840740
actataaaac tctcattttg aaactccttg tatcgttaat caaacaatca aaagggcaga 1840800
tgccctatcc ttgcttttac aaacggagtg cctgtaaaag gggatggttt caggcagttt 1840860
tgaagtttgt gtttttatat attgtcttct ggtcgtctga aaaggtttca gacaacttct 1840920
ttatctttac agcctcaagt cttacagttt gcccgacata ctataaatca gctccaatac 1840980
ccattcgtac aatcaccgtt tctcgtgtag gatgtctgct tccaacgtca tgccgatttg 1841040
cagcggtttt tcctcaccgt atgcagtgat ggttgatttg tcgggtttta ttttcacaag 1841100
ataaacaggt tcgttgctct tcgccaaatc ggaggatacc atgcccaatc ccgacaattc 1841160
ctgtctgccc agtgccgttt ttgctactga tacgacactg ccggaagcaa gcccgaattt 1841220
ttgataggga tatgcctgat aacgtaggac aaccttgtct ttcggcttga taaagcctgc 1841280
tgcactgctg ggatatatata gatgggcata tagctcggta cgttcgggaa caatgctcaa 1841340

gagcagtttg gaaggatcaa cctgctgtcc gacttcgacg ttcggtattg ctatataacc 1841400
cgaccgtcct gcacggatga tttgttcaga gcgcatttca aaatccaaaa cttcttgaga 1841460
aatatcggca atggtgcgtt caagccagct ttgttctgtc tcatgccgct tgggggaggc 1841520
tggccaatgt cagattctgc gtgcggattt cctgaagcag cccgacttct tctcggcggt 1841580
aggcatcaag tttggctttc tgctctaaaa gctctgcctt gacattcatc atttcttgtt 1841640
ttggcactgc atcattggcg gataggaaac gatatttctg caacatttct tccgcaagtc 1841700
taatgcgcct tttctgaccg tctatctgtt gcgaaatatg gagttcctgg ttttccaaac 1841760
gttcgacagt tgctttaagg ctgcgcgttt cattcccgtg tatcagcttc agacgaccca 1841820
gttcctgttc tgccaacgtt ttcttcaaaa ctgcctccgt tttcaactgc tgctgcacgc 1841880
tacctcctgc gccgaaacgt gaggtcgaaa gcgcaaatag cttgtcgcca gccttaacct 1841940
tttctccatc ttccacgaat ttcgctgtaa ttgtccccgt atccggtgca tacaccctga 1842000
ttacgcccga tgcaggtaaa atttgtccct ccactgttgt ctttcgcgta tagttaccaa 1842060
atatcaaaaa caggataatc aataacgcag atatcgatgc aaatgtcgtc catagggaaa 1842120
atgacaacgg tcgtgtcaga atcactttac ccgtcaggct ggtttggcgg gcaacggcga 1842180
cttcgggacg gaagaagggt tgcttgggtc tattcataaa attgaagtta agaaagtttc 1842240
agacgacccc tagagattgt ctggacgatg agaaatatca gcagtaatct gtaccgtcag 1842300
tgtagccgtt tcctgattta tctgcttttg ttgcgggagc agttaatcca tgttcaatct 1842360
caaagattgg tcttccgtta taaggaggtg cattaacggc atcatttacc caattacgag 1842420
tcacattgta tacaccattt gcaccagcag caccgtaagc atttttcggc agataataaa 1842480
ctgccgctgc ggcagcaggt attgcaacca aatcccccca tgtgggacct cctttggttg 1842540
tggcagcatt agctacattt ccagctatat tgtctgttac aggacctccc cctgaaacca 1842600
gcttcaattc atgaagttga agttcaatca tttttatact ccttttcttg gttggtattc 1842660
ctaaaaattc ggctaacaaa aacatatggc agatatattg aaaaaaaatt caaagtaccc 1842720
tgaataaaat tcaaattcca actatatttg ttaatgtagt cgagaagaaa catatctgat 1842780
aaaaaatata gcacttgata acaagctatt actaatatta cgaaaaatgt aaattgcttc 1842840
cagtttttca tagaatccct cacaaaattt ccagaaaatc taactctatc aactgataaa 1842900
tcaacttcct aacttcttca tattttccct gattgaagtt aaccagtaga tttttcaaca 1842960
ataacggttc attcttaccg atgtgttcta acactttttt ccccaactca tctacgctta 1843020
tcttcatccc attcccaatc aaatatccct tttccaacgt atccaaatta ttggcattta 1843080
atctcaacct gacgtcgtct gaaagcggag tagcgttggg attcgcgaac tgttcgagat 1843140
gaaaagcggt atcggtacgt tctttgccga gaaagtcttc actgaaggct tcataattga 1843200
cggggtcggc aatcatggca gcaatttgtg cggcagtatc gttgatacgc gtcctatctt 1843260
gctcccagtc tgagaaactg tggcgcagac tttctatggt gggaaatttc ttcattagcc 1843320
actcgaggta attatagccg ttgggtggaa aggtaccgac agcgaagtgg aaggtttcac 1843380
agccgagcgg gataggtctg tgccaccaac cgcgtgggat gtagaggaca tcacctgctt 1843440
caaggataat atccatatcg atatgttcag gaatggaaat atcagtatct ttagtctgtt 1843500
gcatatacaa tggcataggg aaatcagggg cagtaagttg ccaacgtttc ttgccgaaaa 1843560
gctggatggc atacacatcg cgggggtccc aatggttttt ataagattcg tcgctgccaa 1843620
aagcaagata tccactaaca atagtatgtg cgccggcaaa gcgggcgact tgacgggcga 1843680
tatggtctga aaacggctcg ttgttaatat ggttatagac taacgacgca ccattcttca 1843740
tatgttcgta gataacggat ttaataaaac ggtagcgagt tttgcccaaa tcgtcgaaac 1843800
tttcgacgta ttcttcttta ggaacgattg cgcctttttt acgcagatga aacagcggtg 1843860
cggttgggtc tgctcgttgg tatatctcgt tgatatcttt ccaagatgcg gattcgagat 1843920
tccgaaccgc tcctttaaag agcttgggct tttgatacag ataagtctgt cggaacgttt 1843980
taggactaat gccgaagtcg agatggatgc tcattacttc cccttactca gaaaatattt 1844040
aaaatttata atgttacata tatttacaaa tattaaagtt tttttttgtg tgtgcgtcaa 1844100
ggaattgttg acaattttag ttaaaaattt gtctccaaac ggaaaaaagc ggtttgtttt 1844160
ttgttgttaa catttttatt gtaattaaat aaaaggtcgt ctgaaaacgg ttttcagacg 1844220
accttttgct ataatcgggc ttcatcgccc cgttcggttt ggaaccttat gaaaaccctc 1844280
gtcctcctcc tgctttcctt ctccacgacc accgctttcg ccgcatacgg tttgggtttg 1844340
gggcaggcac cgaaatatcc tgccggcttt cgcgcctacg gttatgttta ttccggacgg 1844400
cagggctagg ttttaaaaac agaggcggat gccattaaat tagacacgct tttcaaacgc 1844460
tttgtgtacc gtccttccgc cgccaatcaa aaccccgtcg gacagcgttc ggacggcata 1844520
cccgccaacc acacaaagga aaaaccatga gtaaaaaaat caaagtcggc attgtcggcg 1844580
cgacgggcta caccggcgtg gaactgctgc gcctgcttgc cgcccatccc gatgtcgaag 1844640
tcgccgccgt aaccagccgc agcgaagcgg gaaccgcagt tgccgattac tttccgagtt 1844700
tgcgcggcgt gtacggcctc gccttccaaa cgcccgacga ggcaggtttg gaacaatgcg 1844760
acatcgtctt cttcgccacg cccaacggca tcgccatgaa agacgcgccg cgcctgattg 1844820
aacagggcgt gcgcgtcatc gacctttccg ccgacttccg catacgggac attccgacct 1844880
gggaacactg gtacggcatg acccacgccg cccccgacct cgtttcccaa gccgtgtacg 1844940
gattgagcga actcaaccgc gaagccgtcg cacaggcgcg cctcgtcgcc aaccccggct 1845000

gctacccgac ctgcgtatcc ctaccgctcg tgccgctgtt gcggcaatgc cgtctgaagc 1845060
ccggtatgcc gctgattgcc gactgcaaat ccggtgtgtc cggcgcgggc aggaaaggca 1845120
atgtcggttc gctgttgtgc gaagccggcg acaacttcaa agcctacggc atagccggac 1845180
accgccacct gcccgaaatc aggcagacca tcgccgggct tcaggacggc atcgccgaag 1845240
gattcgtgtt cacgccgcac ctcgcgccaa tgatacgcgg tatgcacgcc accgtttacc 1845300
tccacctttc agacggcagc gaccccgaaa ccgtcctgcg cgactactac cgcgacagcc 1845360
cgttcgtgga catcctgccg accggttccg cccccgaaac ccgcagcgtg cgcggcgcaa 1845420
acctctgccg catcagcatc caacaggcgg cgcaatccga tgtgtgggtc gtcctttccg 1845480
tcatcgacaa cctcgtcaaa ggcgcggcgg gtcaggcagt ccaaaatatg aacattatgt 1845540
tcggactgga ggaaacacac ggcttggacg caatccccct gctcccctga agcgcaaaca 1845600
gcaaaccgca ggcatcgtgc ctgcggtttt tgatgccgtc tgaaagcgac gttttttttgg 1845660
gttcggacgg cttttgaccc atccattcac acgaaaacaa aaatctaaaa taccgtcatt 1845720
cccgcaaaag cgggaatcta gtttatccag cttcagcaat ttccgacaca tttccacacg 1845780
cttcgattcc gtcatttctc cggtttcagt cattgccgat aacaccgtgg tttttcattt 1845840
ctagattccc gcctgcgcgg gaatgacggc ggagggcttg ccgtttttcc cggtaaatac 1845900
ctgcaattta aaatcccatc attgccgtga aaacaaacca aaaacctaaa atcccatcat 1845960
tgccgcgaaa acaaaccaaa aacctaaaat cccgtcattc ccgcaaaagc gggaatctag 1846020
tttatccggc ttcagcgatt tccgacacat ttccgtacgc ttcaatttcg tcatttctcc 1846080
ggtttcagtc attgccgata acaccgtggt tttcatttc tagattcccg cctgcgcggg 1846140
aatgacggcg gagggcttgc cgtttttccc ggtaaatacc tgcaatttaa aatcccatca 1846200
ttgccgtgaa aacaaaccaa aaacctaaaa tcccgtcatt cccgcaaaag cgggaatcta 1846260
gtttatccgg cttcagcgat ttccgacaca tttccgcacg cttcaatttc gtcatttctc 1846320
cggtttcagt cattgccgat aacaccgtgg tttttattt ctagattccc gcctgcgcgg 1846380
gaatgacggc ggagggcttg ccgtttttcc tggtaagtct ctgcggcttc tcattgccgg 1846440
tttccgccta cttgggaatg acgtgattta aaatcatgaa aatgtgtcaa aaataatata 1846500
gtggattaac aaaaaccagt acggcgttgc ctcgccttgt cgtactatct gtactgtctg 1846560
cggcttcgtc gccttgtcct gatttttgtt aatccactat aaaaatcaga tttccgttac 1846620
actttttttcc aatatttcag acggcatttt gctcacacgc ccaaataccc ttccctgccg 1846680
gaaagccacc ttgccaaatg cgcttcgacg atttcggggt tttgttcaat cagcatcggg 1846740
gcggtttcgc gcgcttgttc caagaggtgc aggtcttctt cgagcttggc gaaacgcagc 1846800
ataggcacgc cgctttggcg cgcgccgaga aattcgccgg ggccgcggat gttgaggtct 1846860
tggcgggcga tttcaaagcc gtcggtgtgt tcgtagatga ctttcagccg cgctttggcg 1846920
agttcgccca agggttcggc aaacaggagg acgcacacgc tttctgccgc gccgcgcccg 1846980
acccgaccgc gtaattggtg cagctgcgcc aagcccatgc gctcggcgtg ttcgatgacc 1847040
atcagggcgg cattgggcac atctacgccg acttcgatga cggtggtggc gaccaagacg 1847100
ttcagccccc ccgaagaaaa ccgcgccatc acttcggcct tttcggcggc cttcatgcgc 1847160
ccgtgtacca gtccgatatt gagttcgggc aatgccgtct gaagccgggc gagggtttcg 1847220
gcggcggttt gcagttgcag ggtttcgctt tcttcaatca atgggcagac ccaatacgcc 1847280
tgccgccctt ttcggcaagt gccgaggacg aagccttcga cttcggcgcg gcggacgttg 1847340
ttgacgaggc gcgttttaat cggtgtgcgc ccgggcggca attcgtcgat gacggacacg 1847400
tccaaatcgg cgaaaaaact catcgcaagc gtgcgcggga tgggcgtggc ggacatcatc 1847460
agctgatgga cttcgcgccc tttgtttttg agggcgaggc gttgggcaac gccgaaacgg 1847520
tgctgttcgt ccacaatggt caagcccaaa ttgtgaaacg ccacgccgtc tgaaaacagg 1847580
gcgtgcgtgc cgacggcgat tttgacgctg ccgtcggcga gtttggcttt ggcttcgtct 1847640
ttggcttttt tacgcaaact gccaaaaagg cggacaactt caatgcccaa aggttcgagc 1847700
cattgtttaa atttaataaa atgttgttcg gcaaggattt cagtgggcgc cattacagcc 1847760
acctgcgcac cggattcgat agccgtcaaa gcagacaaag cagccacaat ggttttgccg 1847820
ctgccgacat cgccctgcag caggcggtgc atcgggtagg tttgcgccat atcgcggcag 1847880
atttcggaaa caactttttc ttgcgcatcg gtcagggcaa acggcagggc ttggcgcagg 1847940
gcttgggtca atgtgccgtc gccgcccaat gccgccgccg tgccgccgat acgcttctgt 1848000
cgcgccaagc gcatcgaaag ctgttgcgcc aaaagttcat cgaatttgag ccgttgccat 1848060
gcaggcagcg tgccgtctga aagctgatga atcgtgaaac tcggcggcgg cgaatgcaaa 1848120
agacgcaggc tttcggcgag gtgtggcagc ttcagacggc acagcagggc atcgggcagc 1848180
gtgtcgtgca gcggcgtaac gtccaacgcc gtctgaataa tacggcgcaa agtgggctgg 1848240
ttcaaaccgt ttacggtcgg gtaaaccggc gtgaggcttt ccgccaaacc gccgccctcg 1848300
gcatcgcgga ttttgggatg aatcatctcg tcgccgtaaa agccgtgttt gatttcgccc 1848360
acggcgcgga tgcgtttgcc gaccgccgtc tgtttctgat ggctggcgta aaagtggatg 1848420
aagcgcagaa aaaggacgct gccggagccg tcggcgattt ggacaatcag ctgcttgcgc 1848480
ggtttgaacg ttacttcctg atggataacc tccccctcga cctgacacgg cacgccaatc 1848540
ggcgcgtcct taatcggcat aatgtgcgtc tcgtcctcgt aacgcagcgg caggtgcaac 1848600
accaaatccc acgcggtatg gaggttgagt ttgtcgagct tcttggcgga aacatcggtg 1848660

attttgagct gttttcgggt ttcgggcgac atcataggca gattcctttg gacgcgccta 1848720
ttttatccga aaacaaaaat gccgtctgaa acggattcag acggcatcga caggcaggaa 1848780
tcaagccccg gcggcttcgg cttcctgctg ttgttggtaa atcgcctcaa aattaatcgg 1848840
cgcgagcagg acgggcggga agccggcgcg cgttaccgta ccggaaacgg cttcgcgcgc 1848900
gtaagggaag aggatgttcg gacacgccac gccgagcagc aggtcggcat cttcttcggg 1848960
gatgttttcc aaacggaaaa taccgctttg ggttacttcg ttcaaaaaca tcgtgcgctc 1849020
gttatccaat ttggcggtta cggtaacggt tacatccacg ttgtagtagc cgtcttccag 1849080
cttttggctg ccggtggaaa cgcgcatctc cacttcgggc tcgccctgtt ccaaaaagat 1849140
ttgcggcgcg tgcggcactt ccaaagacaa gtctttgaca tacagtcgct cgatgctgaa 1849200
tacgggttgc agttcttcgc tcattttgtt ttcctagttg gggttaagg gttcagcagt 1849260
ccgtccagcc cgccttcctg ctggaggcgg tagaggtcgg taaatccgcc gacgtgcgtt 1849320
tcgccgatga aaatctgcgg cacgctgcgc tgtcccgaaa gctgctgcat ttcggcaaag 1849380
gcttcggggc ttgcatcgac acggatttcg tcgatatgtc cgacacctgc cgcgtgcagc 1849440
agccttttcg ccatcgcgca gtaggggcaa aacggacctg tgtacatggt aacggtctgc 1849500
atattgggtt tccgaaagtt ttgcaatgat aatcaatata ggggcatttc ccctgtttgg 1849560
caagtgcgga acagatgcac gttcaaacgg catgtgcgga atgtgtcaaa gtttcttttt 1849620
taaagtatga tagacattgt gaaaaatatt tttgcacccg cgctgcgcgg cggaacggat 1849680
gcaaaatatt tttattacat tttcaggaaa aaccatgttg tcaggactcc ccatccccaa 1849740
agacatcgcg cgcccgcccg aaacgatatt ggtcaacatc acgccgcaga aacgcgcgta 1849800
gcggtgttgg aggaaaacaa tatctgcgag ctgcacatcg agcgcaacag cgaacacagc 1849860
ctagtcggca atatctattt gggcgtggtg cgccgcgtgc tgcctgggat gcagagcgcg 1849920
tttatcgaca tcggcttgga acgcgcggcg tttttacaca tcgtcgatgt cctcgaacaa 1849980
cgccgcaacc ccgaagaaac ccagcgcatc gaacatatgc tgtttgaagg gcagtctgtt 1850040
ttggtgcagg tcatcaaaga cccgatcaac accaaaggcg cgcggctttc cacccaaatc 1850100
tcgctggcgg ggcgtttcct cgtccatctt ccgcaagaag accacatcgg cgtgtcccaa 1850160
cgcatcgaag acgatgccga acgcagcagc ctgcgcgaac gcctcgacaa gctcctgccg 1850220
gaaaatgcct gccggggcta catcatccgc accaacgccg aaaacgccac cgacgaacag 1850280
ctccagtccg acatcgacta cctgaccaaa gtgtgggaac acatccaaga acaggcgaaa 1850340
atccggccgc ccgaaaccct gctttatcag gatttgcctt taagcctgcg cgtgttgcgc 1850400
gatatggtcg gctgcgacac gcaaaaaatc ctcgtcgatt ccaccgtaaa ccacgggcgc 1850460
atgacgcgtt ttgccgaaca atacgtccac ggcgcattgg gcaggataga gctgttcaaa 1850520
ggcgaacgcc cgctgtttga aacccacaac gtcgaacagg aaatcagccg cgccctgcaa 1850580
ccgcgcgtca acctcaactt cggcagctac ctgattatcg aatccaccga agccatgacc 1850640
acgatagacg tgaacaccgg cggcttcgtc ggcgcacgca acttcgacga aaccatcttc 1850700
cgcaccaacc tcgaagcctg ccacaccatc gcccgcgaat tgaggctacg caacctcggc 1850760
ggcatcatca tcatcgactt catcgatatg gcacaggaaa gccaccgcga agccgtgttg 1850820
caggagcttg ccaaagccct cgccttcgac cgtacccgcg ttaccctgca cggttttacc 1850880
agcctagggc tggtcgagct gacgcgcaaa cgctcgcgcg aaaacttaaa ccaagtcctc 1850940
tgcgaaccct gcccttcctg ccaaggcaga ggccgtctga aaacgccgca aaccgtatgc 1851000
tacgaaatcc agcgcgaaat cgtccgcgaa gcgcgccgtt acgatgccga aagtttccgc 1851060
atcctcgccg cccccaacgt catcgatttg tttttggacg aagaatcgca atccttggca 1851120
atgctgatag atttcatcgg caaaccgatt tctctggcgg tcgaaaccgc ttacacgcag 1851180
gaacaatacg acatcgtttt gatgtaaaaa atgccgtctg aagccttcag acggcatctg 1851240
tctatttcag ggtttccttg tccaacaacg cgcgtatcag cagaccgcgt ccgaaacgtc 1851300
ggctgtcgga caattccaaa tatccgccgt atttttggc aagcgtgtcg gcgatggaca 1851360
gacccagccc cgtcccctgc tgctccgttc ccaaaatacg gtaaaacgga tcgaggacac 1851420
gggcgcgttc ggattcggga atgcctttcc cgttatcttc cacccacacg gcaagatatt 1851480
tcccttcgtc cgtgaaaccc aaatctatcc tgccttcggg cggcgtataa cgtaccgcgt 1851540
tgtcggcaaa ggttttaatc agcgtataga tttccgtttc gtcggcagac acttcgacat 1851600
cgcctccgac cgccacgccg atgtcctgac atttttccaa agccagcggc atcagttcct 1851660
gcaacacttg gcggaaacgg ctttgcagac cgaatgtcgt tttcgtcaga gggatttcat 1851720
ccgactgcga acgcgccaat gccaaaagct gttcgagcag gtgtttgtta cgccgtatgc 1851780
tttgctgcaa aacggcaggc tgccgcgccg catcgggtgg gagcggcata ttgttgagcc 1851840
gttccgcctg aaggggaagg gcggtcatcg gcgtacgcaa ttcgtgtgcc gcgtcggcga 1851900
caaaccgctg acggtggcgg atgtcttcat ccgcacgttt caaaagcagg ttgatggcgg 1851960
ttacgaaacc tctgatttca ctgggaatat tgtccacact caaagcagac aggtcattga 1852020
ttcggcgttg ttcgagactt tgcgacaatt tgcggacggg gcgcatggct ttgtgcgtaa 1852080
tccacacggt cagcaaaatc atcagcggca gtgccgccaa caggggcaac acgctttgcc 1852140
gtgccgcatc cgccgccaaa tcttcacggt attcgttttc ctgcataacg gcaatccgtc 1852200
cctgctcggt cgtgcggata tagacgcggt aataatcgtc gtcatcgtcc gcctgaagcg 1852260
tgtgcagacc gtccgccaga tgcgcaggca ggctgacaac agggtcttcc tgctgcggca 1852320

tctgtaccaa aatacgcgta tcgccgtcgc cctcgggcaa agtttcgggt ttggaatcgg 1852380
gggcgacgta caatgccgcc tgacggagca ggtcgtcctg caacgcttcc gtttcgtgga 1852440
aggtttcgta gtaggaaaac atacctgcaa gcattgccag cggaacaaac atccaaaccg 1852500
tcccgccccg gcaaacccaa atccagcagc atcaagtcat aaggctgggc agcggcagcc 1852560
gcgcgccgtt tttgacccaa tccaccgcat agccgccgtc tttcaaactt gccgacaccg 1852620
cctccgcaat catcgcatcg tcttccacca gcaaaacacg catcaacttt cccttcaaaa 1852680
taaaccgtgc ctattctaac accccaaaat tagccgcaat ttagcggtct ttacgcttgc 1852740
cggtattttt caaaactgca gcacaaaaaa accgcgccgg caactgcctt cagacggcat 1852800
tggggcgcga ttgcaacaca cgggcaggca ggcagagcct gcgacagacc acaggaacga 1852860
ttcaggcttc agacggcttc gccgtttacg gcagaggcac gattcctgcc gctatcgaac 1852920
tggccaatat cgccagcgac aaaccccacg cccagaaaaa cgaataacgg atgtgtttgc 1852980
ccatcgacaa tttcgccaaa cccaagccca tccacaaagc cggcgaaagc ggcgtaacaa 1853040
aagtgccgac gatactgccg atcaacatcg cataacctgc tgcttcgggc gccacgcccg 1853100
cctgcgaggt aatctgctcc acaatcggaa acagtccgaa ataataagcg tccgtactca 1853160
aaaccaactc aagcggaatg cccaacacac cgatggcaat atgcagataa ggcagcagcg 1853220
cgtccggcag gatatgcaca atgtctttgg aaatcgcgtc caacatcccc gcacccttca 1853280
aaatccccaa aaacgtacct gccgccaaaa taatggacgc catcatcacc gcgccgccgg 1853340
cgtgggcata aatccgctcc atctgttcct gcgggctgcg gtaattcaaa agcaacgccg 1853400
ccgttgcagc cagcataaat acataacccg gtgggaagat gcccgaaaaa agcaggctca 1853460
tcgccgccaa aaacagcagg acattccacc aaaacagttt cggacgcgcc aatttttgtt 1853520
cttcttccga caaaggcacc ggctttatca aatccgccac ggcgggcaac gcgcccaact 1853580
cccggacaat ccgccttttt tcacgcacac ccaaaagcag ggacagcgca aggataaaca 1853640
ccacaccgat aatttgcacc gtcaacaaag gtttatacaa ttcgcccaca tctgcgccca 1853700
acacgcttgc aacccgcccg gtcggcccgc cccacggcag aaggttaatc aatcccgcac 1853760
tggaagtcag cagcaaaaac agcaggtaag gattcatatg cagacgcttg taaagcggca 1853820
aaagggcggg gacgaccaat aaaaacgtcg tcgcacccgc cccgtccaac tgcgccacca 1853880
ccgacaccaa gaccgtcccc acactcactg ccacgatatt accccgagtc agcttaatca 1853940
aaccgcctat catcggacgg aacagcccca catcgttcat gattccaaaa aacaaaatgg 1854000
aaaacataaa cataatcaca atctgcatca ccgatttggt gccgcccgaa taaaattctt 1854060
ttaattggga tacatcaaac cccgccagca acgccccaaa cagcggcacc aagattaatg 1854120
cgatgatggg cgacactttt tccgtcagca gcagccatac gatgaccccg ataatcagca 1854180
gtccgataaa cgtcagcatc atttctcctt tattttattt taaacagaaa accgaccgtg 1854240
caggcaaaac cgcccacaga cgcgggataa gccctgcatt ctactttttt attttgaaac 1854300
aagtcaatcg gtcatttcct cccatttacg cctgccgcca ttcctgcatc cgtccgtcat 1854360
ttcacagcgg caaccgatac ggaacaaccg gtaaatcggt atcgggacgg cgcgggggca 1854420
ttcatcccgg tgcgccgatt caaacgaaac cgcccctatc attgcggagc gcggggcgtg 1854480
ccgtacacgc gggattttat agtggatgaa caaaaatcag gacaaggcgg cgagccgcag 1854540
acagtacaaa tagtacggaa ccgattcact tggtgcttca gcaccttaga gaatcgttct 1854600
ctttgagcta aggcgaggca acgccgtact ggtttttgtt aatccgctat aaacacgccg 1854660
gtcatttgcc gcgcattatc cggcaaacgg caaaccttga cgctgcccag cccatataaa 1854720
aaagccgcaa aacccgaacc ggttttgcgg cctttcgact tggtttgttt atttggcatt 1854780
tctttcaatc aaaccgacaa actgattaat atatgactga acaaaatccc ttgccgagtc 1854840
aatcaatttg ccgttttcat caaacagcgt cggcgaattg cccaaaaaca cttccggctg 1854900
tccggttacg ggcatatcga aataagacag cgcaaggcgc aggttttttt gggaactgta 1854960
accgcccatc ttgccgacgg aatggctgat gatgcctgcc ggtttgtttt tccacgccac 1855020
gtcggcattc ggtttcgagc cgatgtccac cgcatttttc aaacaggcgg gaatggtgcg 1855080
gttattttcg gacgtaacga acaaaatgcc gtccgaagcc ttaatcgttt cgcggaaagc 1855140
cgtgtagctt tcgggtagcg gcacatcttc caccgcaggg tcgtcataat cgaaattgta 1855200
aagcggcaga tgtccgattt caacgatttc cgcctgccag ccttcgggga acatctccgc 1855260
cgcattcaat gccactttgc gcgcaaaaga agcacggcgc aggctgccca ccaaaatact 1855320
gattttctta gccataatca ttcctcctga atattaagtt tgtgcgtctc aatcatttca 1855380
taatgatagc gattattata tatgtgattt cccctgcaaa caagccggcc cgccgccaca 1855440
gcgttcccac ttatccggct ttgccttata attgcttttt ttatgtaaca gatttaccta 1855500
tgaatttccc caaaacagcg gcctccctgc tgctgcttct cgcctccctc gccgcacacg 1855560
cgctcgatac cggccgcatt ccgcaaaacg aaatcgccgt atatgtccaa gagcttgaca 1855620
gcggaaaagt catcattgac caccgctcgg atgtccccgt caaccccgcc tccacaatga 1855680
aactcgttac cgcgtttgcc gccttcaaaa ccttcggcag caattaccgc tgggcgaccg 1855740
agtttaaaag caacggtacg gtaaacgacg gcacgcttga cggaaaccta tattgggcgg 1855800
gcagcggcga ccccgttttc aatcaggaaa acctgcttga tgctcaaaaa cagttgcgcg 1855860
aacaaggcat actcaatatc acgggacacc tgatgctcga ccacagcctg tggggcgaag 1855920
tcggcagccc cgacgatttc gaagccgaca gcggttcgcc gtttatgacg ccccccaatc 1855980

caactatgct gtctgccggt atggttatgg tgcgcgccga acgcaatgcc gccggcagta 1856040
ccgacatcct caccgatccg cctttgccgc atattttcgc ccaaaacaac ttgaaaatta 1856100
ccgcctccca agctgcctgc ccttcgatca aaaaactgat gcgtgcatct tttcggaca 1856160
atacgctgaa attgcgcggc aatattcccg agagctgttt gggcaagcct gtcggtgtcc 1856220
ggatgttcgc gcttgacgaa ctgatccggc aaagttttac caaccactgg ctgctcggcg 1856280
gcggacggat ttcagacggt atcggcatag ccgacacgcc ggaaggcgcg cagacacttg 1856340
ccgttgcaca cgccaaaccg atgaaagaaa ttttgacgga catgaacaag cgttcggaca 1856400
atctaattgc gcgttccgtc ttcctcaaac tcggcggcga cggcaaactg cccgccgttt 1856460
ccgaacaggc ggcgtctgcc gtccggcgcg aacttgccgt atcgggcatc gatgttgcgg 1856520
atttggtttt ggaaaacggt tcgggcctgt ccagaaaaga aagggtaacg gcgagaatga 1856580
tggcgcaaat gttggaaacg gcttatttca gcccgtttgc acaagatttc atcgacacgc 1856640
tacccatcgc cggcacagac ggaactttac gcaaccgctt caaacaaagc ggcgggctgt 1856700
tgcgcttaaa aaccggcacg ctcaacaatg tccgcgccct tgcaggttat tggctgggcg 1856760
acaaaccgat ggcggtggtc gtcatcatca acagcggccg cgccgtttcc ctgctgccag 1856820
acttggacaa cttcgttgcc aacaacatca tctccggcgg cgatggctgg ctggatgcga 1856880
aactgatgtg caaagaacgc cgagcctgaa acaggaaaat atagtggatt aaatttaagg 1856940
ggctgtccta gataactagg acaaactcga ttttactaat tgttttaaaa tggaacaaga 1857000
acttttatct cactgttgtt aaaacgccat tcgcactcct ttaaatacag ctcaaaatgc 1857060
gctttgggaa tgccgttaaa cttgcgtaaa tgacgttttg cctgattcca aaagttctca 1857120
attccattaa tatggttttg tcgttcggca aaatgtgtgc tgtgattgat acgaaaacga 1857180
agtttcagcg aagctaaaat ggctaaattc gcgcacatct aatacatcat agctacgata 1857240
acaatccgta taaacaatac tgtcaggttt cacttgttca cggataatag gaaataaagt 1857300
agcggtttga gtattcggta ctgtaaccgt ataaacctta ccatttcgct tcaaaagacc 1857360
gaatacggcg actttaccgg cagcaccgcg accgcgtttg cctttgcgtt gtccgccaaa 1857420
ataactttca tctgcttcta cttcgccatc aaacatttcc aaatgcggac tgttttgata 1857480
aataagtaat cgtaaacgat gaaaataata ggctgcggta tttttattaa cgcctactaa 1857540
ctctgctgcc gttcttgcag ttacacctgc gacaaacagt tcaatgagtt tattttgttt 1857600
ataccggctt agacgacttt ttctcatagg ggcaactcta acttaatttg aatttcccta 1857660
gttatctagg acagccccaa atttaaacca gtacggcgtt gcctcacctt agctcaaaga 1857720
gaacgattct ctaaggtgct gaagcaccaa gtgaatcggt tccgtactat ctgtactgtc 1857780
tgcggcttcg tcgccttgtc ctgatttttg ttaatccact atataaaaat gccgtctgaa 1857840
ctgttcagac ggcatttttg attttcaaac cggaattaca gccccgctgc cgccctcaat 1857900
gcagcagctt tgtcggtgcg ctcccaagtg aactcaggtt cttcgcggcc gaaatgtccg 1857960
taagcggcgg atttactgta aatcgggcgc aagagatcga gcatttggac gatgcctttg 1858020
gggcgcaggt cgaaatgttc gcgaactaag gcaatcagtt tttcttcgct gattttgccg 1858080
gtgccgaaag tatcgatgga aatcgaagtc ggttcggcaa cgccgatggc gtaggaaact 1858140
tggatttggc attgggttgc caaacctgcg gcgacgatgt tttttgcgac atagcggcag 1858200
gcgtaagcgg cggaacggtc cactttggac gggtctttgc cggagaatgc gccgccgccg 1858260
tgcggagccg cgccgccgta ggtatcgacg atgattttac ggccggtcaa accgcagtcg 1858320
ccttgcgggc cgccgataac gaagcggccg gtcgggttga tcaggtattt ggtttcgtcg 1858380
gtcagcagtt cagacggcag aaccggtttg atgatgtgtt cgattacggc gtttttcagc 1858440
tcttcgtaag cgatggacgg atcgtgctgg gtagacagga cgacggtgtc gatgcgtttt 1858500
actttgccgg tttcgctgtc gtaaaccacg gtcagttggg ctttggcatc aggacgcagc 1858560
caaggcaggc ggccgtcttt gcgcaattcg ctttgacgct gcatcaggcg gtggctgtaa 1858620
tagatggcaa acggcatcag ggtaggggtt tcgtcacagg catagccgaa catcaaacct 1858680
tggtcgcccg cgccctggtt caagtcgatg ccttcgcctt cgttcacgcc ttgggcgatg 1858740
tcgggggatt gctggtcgta gtacacgccg actgcgcagc cgttggcatc aaagcccagc 1858800
tcggaggagt tgtagccgat gcgtttgatg gtttcgcgtg cgactttgat gtagtctact 1858860
tgggcggtgg tggtaatttc gcctgccaat acgcacaagc ctgtgttgac caaggtttct 1858920
gcggcgacac gtgcttttgg gtcttgcgcc aagatggcat ccaaaatcgc atcggatact 1858980
tggtcggcaa ctttatccgg atggccttcg gataccgatt cggaagtaaa cagatattcg 1859040
ctcattgaga ttttccttga aaaacaaacc atcttcttca gacggcatgt tgtatgaaca 1859100
taatgtcgac agcgggaaat atagcaaaat ttccctattc ataccattca gttgagaaat 1859160
attcccattt gaatagcact ttggaatctc tgcccgtacg tttcttacag gcaaaaaaat 1859220
tcccgcatca agcgggtttg gattgctctg gtgagccaca tcggcttttc aaccgtccac 1859280
cttactttc cttttgaaaa gcaggttggc atggaattcc caactcttaa tgcagcacgc 1859340
atcgtagcag aaaaggcata ttgccgcaat acttcccttt ttcagacggc atgtttcgtt 1859400
tacaattcag gctgtttccc cctttgcgaa ccgccatgca catcctgttg accgccctgc 1859460
tcaaatgcct ctccctgctg ccgctttcct gtctgcacac gctgggaaac cggctcggac 1859520
atctggcgtt ttacctttta aaggaagacc gcgcgcgcat cgtcgccaat atgcggcagg 1859580
cgggtttgaa ccccgacccc aaaacggtca aagccgtttt tgcggaaacg gcaaaaggcg 1859640

gtttggaact tgccccccgcg tttttcagaa aaccggaaga catagaaaca atgttcaaag 1859700
cggtacacgg ctgggaacat gtgcagcagg ctttggacaa acacgaaggg ctgctattca 1859760
tcacgccgca catcggcagc tacgatttgg gcggacgcta catcagccag cagcttccgt 1859820
tcccgctgac cgccatgtac aaaccgccga aaatcaaagc gatagacaaa atcatgcagg 1859880
cgggcagggt tcgcggcaaa ggaaaaaccg cgcctaccag catacaaggg gtcaaacaaa 1859940
tcatcaaagc cctgcgttcg ggcgaagcaa ccatcgtcct gcccgaccac gtcccctccc 1860000
ctcaagaagg cggggaaggc gtatgggtgg atttcttcgg caaacctgcc tataccatga 1860060
cgctggcggc aaaattggca cacgtcaaag gcgtgaaaac cctgtttttc tgctgcgaac 1860120
gcctgcctgg cggacaaggt ttcgatttgc acatccgccc cgtccaaggg gaattgaacg 1860180
gcgacaaagc ccatgatgcc gccgtgttca accgcaatgc cgaatattgg atacgccgtt 1860240
ttccgacgca gtatctgttt atgtacaacc gctacaaaat gccgtaacga aaataaaaat 1860300
gccgtctgaa caatttcaga cggcattttg tcatctgacg atttccgaca gcggccagcg 1860360
cggacggaca ttgaacgcac cgacctccct gcccttgccc aggtgcatcg caccggcaaa 1860420
ggcaatcatg gcaccgttgt ccgtgcagta tgccgtcggc gggaaaaaca cgctgacttt 1860480
ttcggacgga tgtttcggct tgcctttggg ggtcgggatt tgcaccgtca tgttgccgaa 1860540
agtttcacgg agcttgcggt ttgcaccgac cccgccggcg accactacgg ttctgaaccc 1860600
tgtctgcaac agggcttttt tcactttcgc cgccaacaca tcgactaccg catcttgaaa 1860660
cgcacggcag atgtcgttgc gtgtctgctc aggaatgtca tccgccccgt tttccgcgcg 1860720
cactttctcg acggcggtca atacggcggt tttcaaacct gaaaaactca tctgcaaatc 1860780
gtcggaatga atcatcgggc gcggaaaaac aaacgcttcg aacctgcccg attccgcaag 1860840
ttccgacagt ttcgcaccgc ccggatacag caagcccagc agtttcgccg ttttgtcgaa 1860900
tgcctcgccc gccgcatcat cgacgctctc gcccaaaagc gcgtagtcgc ctatgcccct 1860960
gaccgccata atctgcgtat gcccgcccga aaccaacagc gcgacaaaag gaaagtcggg 1861020
tttttcctcc gccaacagcg gcgacagcag atgtccttcc aaatgatgga cgggaataac 1861080
aggcttgtcc aacgctaaag ccagcgcgtt ggcgtagctc gaacccgcca gcagcgcgcc 1861140
gcccaaaccg ggcccctgcg taaaggcaac cgcgtcaatg tcgccatacg atgcgcctgc 1861200
ctgcgccaga cagccttccg tcaacggaac aaggcggcgg atatggtcgc ggcttgccaa 1861260
ttccggcaca accccgccgt attcggcgtg cattgccatt tgagtgtgca ggcagtgcgc 1861320
ccgcaatcca cgttccgtat cgtaaagcgc aacacctgtt tcgtcgcaag aagactcgat 1861380
tcctaatacc aacatggtct gatgccgtta aaaactgaaa aacgtatttt agcggatttc 1861440
ggcacgactg ccgtatccca aaaacggaac atgccgtctg aagaccgttc agacggcatc 1861500
gtcgcaccgt atcaaagcgt tccgtaagaa tgcagcccgc tcaaaaacat attcacgccg 1861560
ataaaggcaa atgcggttac gaacaaaccg ataatcgccc accacgccag cactttgccg 1861620
cgccaaccgg caaccagccg caagtgcagc caaacggcgt aattgagcca gacgatgaac 1861680
gcccacgtct ctttcggatc ccaactccaa tagcgtcccc aagcatctgc cgcccacagc 1861740
gcacccaaaa tggtggcaat ggtaaagaac agaaagccga cggcaatcgc cttatacatc 1861800
acctcgtcga tcaatgccga cggcggcagc cacagttttc cgccttttcc ttccgcacgc 1861860
agggaaacca gttcggcaat accgagcatc gcggaaatgc aaaacgcgcc gtaaccgata 1861920
aagtttgccg gaacgtggat tttcatccac caggactgga gcgcgggaat cagcggctgg 1861980
atggtatgcg cctcgcggga cacgctgtac cacaagacaa atccaaccac gaccgccata 1862040
aagccgaaca cgaagccgcc caatttctgt atggcgaact taccttcata ataaagatac 1862100
atcagcgcgg taatcaccaa aaacaggatg aacacttcat acaggttgga aaccggaata 1862160
tgccccgcat cgggacggag cagatagctt tcgtgccaac gtaccagcag accggtaaag 1862220
cctgctacgg cagacaccca tgcaaacacg gttcccatac ccaacagcgt gttggtcggc 1862280
acattttttta cgcttgccaa aaccgcgccc gaaatatagg cgaacagggc gaaaaagaca 1862340
aaggcgcact gccacatgat cgccgactgg ctgctgagga aataccgcaa caggaaaatc 1862400
tctgccgatt tgatgtcgcc tccgtacaaa ccgacggcgg cataggcaag caatacgctt 1862460
aaaggaacaa accagcgcat cggtttgaaa aaccaaccca aaaacacggc aataccggca 1862520
cttgcccaca acatgaccgt ttcgtaaatg tccatatgca taccggaacg ggtctgtacg 1862580
aaaaccgtag ccgcaaaaac cagcacggca aatacccaat cccaaagatt cagattgctg 1862640
atcaaagact tctgaatcag cagctcgtgt tccggaaggg ttttatagtg ttcagtcatg 1862700
attcaagtcc ttgccgagcc gttgcagact ctcgacgtgt tttggaaatt ccttctgcaa 1862760
atcccgttcg ctgcgggccg aagacatggc aaaacggatt ttgccgtctg aaaacaatac 1862820
ccacgcccgt ttttcgcgca cataaaacat caataccgta cccaatacca acagcaccga 1862880
gccgagatag accaaaagcg cacccgggga acgggtcatc tgcaaacccg acgaacgcac 1862940
ctcggaaaac ccatcaagtt gcagcagcat aggcgcggga tattcggtca aacccgtgta 1863000
cgcatccata ctgtgcagca ggaaacgatt ccgcgcttca tcctgctgcc attcgggcaa 1863060
gccgtaccgg cgtatggttt catccaaagc agcgttcatc acgccgtaaa gcatttcgta 1863120
gaaatagccc tgcatcttat cctgctgctc tttcgggata ttggacgtaa taaattcgtc 1863180
caatcccaaa tagccttttt gtgcaaagat gttcagcgtg ttttccgcag ccagcatgaa 1863240
ttgttcgcgg atttcggcag gtgcgccttt ggttgcgtcg gcaaccagac gtttgcgccc 1863300

ttccccatct ttcaaaaact cacgcaatgc cataaaggtg tccgctttca actgcttgtc 1863360
caaggggata cgcagccagc ggtattgctg ctgcaagccg ctgcgcgtgc cggtaatcca 1863420
aaaataatcc tgttcctgca aaaccggcag catatagttt ttatattcga ccgcctgccc 1863480
tgccgcatca cggatacggt aaacaatgga agggccgata ttggtgtatt ttttaccttc 1863540
ctgagtaacg gcgcggacat cgttcagcgt ggatttcagg ctttttttccc gttccgcgcc 1863600
ctcgctcatg tcctccacat tcatagaagt gaactgatcg aactcaagac gatatttgtg 1863660
tttgccaatt tccaacggaa actggtgtat ggatgttgcc ttcaacacga caggctcgcg 1863720
cgaagcatca cccaaattcc acgccttgaa tgtcaaatcc gaaccgccgt cggcaaaact 1863780
cgcctgataa atcgtgatgc cgtgcaaggt caaaggatgg ttcacgcgga tggtgcgctc 1863840
gagtttctca ccggttgcct tgtccgtcac ttcaatatcg ctggcgaaat cacgcggcat 1863900
acccgtattg taaaaatcga tatggaattt tttcagtttg acttcaaaag gcaagtcctg 1863960
aaccaatatc ccgttgtcgg cattcaggaa aaccacatcc gcactctgcc cctcggaaat 1864020
attgacgttg cccctaaatg agagattgga cgcacccaaa atactttcgg gcttgaaatc 1864080
cttggcataa accgcctgat tgtccggaac aatccgaccg gtcagcatac ccagtttcaa 1864140
cagcaggtta ctgtctatca acccgcccag gcaaatgaca atcaaagcaa catgggcaaa 1864200
gatatagccc catttgttca ttgtgccttt tttggcggca atcagaaccg acccgtcttc 1864260
acggttaatg gtttttccct gaaaaccttg tacttccaga taacgtttgg caacctcggg 1864320
cgcaattttt acatccaaca gcgaagaatg gcgcatcgcc gccagagatt tttctttaac 1864380
cttttcccga aaagacttca tttcgcgcca gaacggcggc acattgcgaa tcaggcacaa 1864440
actggtagaa accaccaaaa acatcatgat aacgacaaac catgccgaag catagacgtc 1864500
atacagtccc agaaaaccaa aaatctgcgc ccaaaacgat ccgaatttga ccaaataatc 1864560
cgtctgcggc tggttttgct gcaacaccgt accgataacc gatgcaatac ccagcagact 1864620
gagcaaagcg actgcaaagc gcatggagct gaaaaaagcg aaccacggac gggaaagaag 1864680
tgggggagat ctacgggatt tactcattgt gtgtttattc cgccatcagg aatatgggaa 1864740
agcagaattg ggcaaacaga aaacaacgtc ccgattctac tgtcttgatg ctttttattt 1864800
caagacaatg aagacagcct gcatcgattc caacggttgc gattgaaaaa acttatcgca 1864860
gaattgcctg aagccgtctg aaaacttttc agacggcctc taaaacagac tattgcggaa 1864920
ttaacgcaaa ccttggataa agttggcgac cgctttcaaa tcttcttcag acatacggtt 1864980
tgcaatatct tccatgatgg tatttttacg ctgaccggac ttgtaggcat tcatctgttc 1865040
aacaatatat gcctgatgct gaccgcccaa acgcggataa gcctgaattt cgcttccgcc 1865100
tcccggcata cccgcaccgc tcggaccgtg gcaggacata cacgccggca ctttttttatc 1865160
gctcaaaccg ccgcgataga ttttcgcacc caattcggga ttttccttag gattggcttc 1865220
accggatttg ggctgctgtt tggcatagaa tgcggatacg ttcaaaatat cctgatcgct 1865280
caaattcatt accaccggtt tcatcacagc tgccgaaccg tgggtgcgtt taccgtcgcg 1865340
gatgccgata gtttgatgat agatgtaagc agtatgctgt gccgccaaac gcggatacat 1865400
cgcaatgccg ctgttaccgt ctgctgcatg gcaagccgca caaaccgttg cggcaacctg 1865460
tttgcctttt tccacgtctg ctttgggaga ggcggaaacc gcaccggcag ccaaaacaaa 1865520
ggccaataaa gtcaatcgtt tcatggagtg ctcctgatta cagcattgga taacgcaaca 1865580
atgctctttt tatattcaaa tacgggattt ttgacccgat taaaaccgat gattctgtaa 1865640
acgtgttatt ctatactaaa tttacattaa attaccactg tgtttcacat aaaaccaacc 1865700
gcatattttt gctgtcggac aaacggcggc ggaaaacaag gatatgccca tgaacctttt 1865760
tcaaaacgcc aaattcttca cgacgatcaa ccaccttaaa gacctgcccg acacccctct 1865820
cgaaattgcc tttgtcggca ggagcaatgc cggaaaatcc agtgccatca ataccctgac 1865880
caaccatgtc cgtcttgcct acgtttcaaa aacacccgga cggacgcagc atatcaactt 1865940
cttcgagctg cagaacggca attttatggt cgatttgccc ggctacggtt atgcccaagt 1866000
ccccgaagca gtacgcgcac attgggtcaa tctgctcggc gactatctgc aacagcgcaa 1866060
acagcttatc gggctggttt tgattatgga tgcccgccat cctttaaaag aactcgacat 1866120
ccgtatgctg gatttttttcc acacgaccgg cagaccggtt cacatcctgc tgtcaaaagc 1866180
cgacaaatta tccaaaaaacg aacagataaa aaccctgtcc caagtcaaaa aactgctcaa 1866240
accttattcc gacaggcaaa acatcagcgt acagctgttt tccagcctga aaaaacaagg 1866300
tattgacgag gccaaccgaa ctgtcggaag ctggttggac gcagcagatg ccgccgcttc 1866360
ctctccagag gaaaactgac cccaattata cggaaaccgt attcccccca cttgaccgac 1866420
cgcaaacatt taaaaaattg ccactgccaa atctaaaatg ccgtctgaaa agtctttcag 1866480
acggcatttt gcggagtctt taaaacagag aatccaactg ctgctgtttg gaaccagtat 1866540
tactcggaag caccggcgtt tcctgcatat cttggcggac ttcgtcatcc gccgcctgcc 1866600
gtccgccttc tgccgcgccc ccgtcatctt cttttgcccg tcgggaaggt tgcggcgcaa 1866660
taccgctgtt gtccagcgtc aagcccggat cggttaccat acgttccttc atatagtatt 1866720
cgccattgct gctgaccaca ccttcaggca ttttcatccc cttgccctgc tttcctttca 1866780
acgcaaaacg catatagtcc acccaaaccg gcaccgcaat cgtaccgccg tagccgacac 1866840
gccccatact cttaggtttg tcgaagccga tatatacggc agtaaccaca tcagggttaa 1866900
aaccgacaaa ccacgcatcc ttattgtcat tggtcgtacc cgttttaccg gcaatatccg 1866960

ttcttcccaa cgcagctgcc ccccttgccg taccaacacg gaccacatcc tgcataatct 1867020
tatacataat ataggcattg cgcggatcga ttgcctgagg cgcattttgc ccagccacca 1867080
aaggttgcat ttgggcgcgc aacctgccgt ctctgtcata aatcttatcg attacgtgcg 1867140
aagaaaccct atatccgccg ttcgcaaata cgctatatgc ctccgccact ttcaacggcg 1867200
ttgtctcgcc cgtacctaaa gccatagaca ggcttgccgg cagctcggac gacctgaagc 1867260
cgaaacgccg gatatactgt tgcgcgtaac cgacaccgat agacatcaaa atacggatgg 1867320
aaaccatatt cttggaagcc gtcagagcct gtctcaaagt aatgtagccg gaatatctgc 1867380
cgtctgaatt tttaggtgtc caaaccgaac cgttcggccc tttccccggc agggaaatcg 1867440
gcgcatcgtt aaccactgtg gacgcggtca tccccttaga taatgccgcc gaatagacaa 1867500
acggcttaaa ggtcgaaccc ggctgccgca ttgcctgaac ggcacgattg aatgttttgc 1867560
tgtgaaaatc ataaccgccg accagcgcgc gcacagctcc ggtttttgca tccagcgaaa 1867620
ccaaagcccc ctgcagcaac ggctcttgaa ccaccgccca acgcccgccg ttgtttttga 1867680
cacggatgac cgcgcccctg cggatacggt cctcccccat tttttcatta ttgaccgcgc 1867740
gggccgcaaa acccaaggcg cgcctgtcaa gcgtaacccg cctgccgccg ggcagctgta 1867800
tgacgacatt tttcttttta gtcacatcca acacaacggc gggaaccatt ttatcgacgg 1867860
tatagagtcc cgacagatac tggctgacag tctcctcgac atcttcactc ttactcaaat 1867920
cgatatagtt ttccgcaccg cggtagctgc tgccgcgatc gaaattccgt agagccttgc 1867980
gcaatgcctc ggttgccacc ttctgatgat cggcgcggac cgtggtataa accttaaaac 1868040
cctgcgtata ggcatcttca ccgtatttct catacagttc ctgacgcacc atttccgcca 1868100
catataacgc actctgatcg attttccgaa caaaccgctc gtaatgcagt tcctcattca 1868160
acgcctgatc gcgctgttgc acggtaatca tcttctcctc gagcatattg ttcaaaatat 1868220
acttctggcg caacttggca cgttctggat taacaatcgg attataggca gacggagcct 1868280
tgggcagtcc cgcaagcatg gcggcttccg ccaaagtcaa atctcggaca ttcttattga 1868340
aatagatttg cgcggcagat gcaaaaccat aggcgcgctg accgaggtaa atctgattga 1868400
aatacaactc gaggattttg tctttgctta aagactgctc gattttatag gcaagcaaca 1868460
cctcattgaa tttgcgtgtg aacgtttttt cactgctcaa ataaaaattt ttcgccacct 1868520
gctgcgtaat cgtactcgca cccgactgca cgctgccgga cacgacattg ccgacggcag 1868580
cgcgggcaac accccaaaca tccacccccc aatgccggta aaagcgttta tcctcggcgg 1868640
cgataaccgc attccgcaac acctctggga aatcgccgat ttttgtaaat tcgcgccgct 1868700
gctccccata cataccgatg acttccccat ccgccgaata aatagtcaac ggcattttag 1868760
gctggtaatg ctgcaaagaa tccaaagacg gcagtttcgg atacgttacc aaaatagcaa 1868820
tggcaaccaa acccactcca aatacacaaa acccaaaaac caaaccaaaa caagtcgtta 1868880
aaatcttttt aatcatagct gaataataat ttaccattat tggtattaaa taaagtaaaa 1868940
tagcaaccga tttctacaaa gcacggtttc aatgtgcaaa gaacaaggaa tccattacgg 1869000
ataccgaaac ggttactcac tgtacaaata aagcaggaac tttcatcatg cgcttgttta 1869060
aaagcttgaa aaaccctaaa aaaacagatg ccaagctccc taaaaaatct tcgggactca 1869120
ataaccgcgc ggcaatcggc atcgatatcg accagcattc catcaaaatg gtccaattgt 1869180
caggacgtag tttaaaccaa attcaattgg aaaaatacgt cattgccaaa ttaccaaaga 1869240
atatcattca aggcaataaa gtccaaaatt acgatcaact tgttacatat ttgcaacaag 1869300
cctatgccaa actgggtact tcgtgcaaaa acatcatcgc gtccgtcccg caaaatttgg 1869360
caaccatcga acaattgacc tacacagaca aagatgcaga attagacctg caggggttcg 1869420
tggagtcctc catctccgaa gtcagctcga tatcgctcga agaagccaat tacgactatc 1869480
aggtcttgtc ccaatcggcc gccggcgaag ctgtgttggc cgtcgcatcg agaaaggatg 1869540
aaatcgaacc cctgattgac gcattcaacg ccgccggtat gaaattatcc gcgcttgatg 1869600
tggacatttt cggacaatac aacgcctacg cgctatggat aaaccatttc gcccccgagc 1869660
ttgcagccga aaaagtcgcc attttcggcg tatatgccgc acagacctac gccttggtca 1869720
tccaagacgg aaaaatccta tacaaacagg aaacctccgt cagcgaagaa cagctcaacc 1869780
aactcatcca gcgcacctat caggtaacag aagaaaaagc ggaagaaatc atcaactccc 1869840
cgcaaaaacc ttccgattac caagaaagcg tggcaaacta tttcaaccag cagattaccc 1869900
aagaaataca aagggtcttg cagttttatt acaccacgca gaccgcagac gatatgaccg 1869960
acatcaagca tatcctgctg accggggaag cggcgcgcca ggaaggcatc gcccaaaccg 1870020
tcgcctcaca aaccaatgca gatgtacaat gcgtccatcc cgcgcgttat tttgcggaca 1870080
acctcaaaac agacaaacaa caattcgaac ttgatgcgcc gacactgacc agggcgttcg 1870140
gtttggcggt acggggatta taattatgaa caatttaatc aaaatcaacc tcctccccta 1870200
cagggaagag atgaacaagc gcaaacagca gcagtttaaa acgctgatgt acggtgccgt 1870260
gctgacgggc gttgccgccg ttgcggcaac ctacctgttt atcgacaata tgatcaataa 1870320
ccagtcggaa agaaacacgc tgctggaaac ctccatcgca cacttggata ccgagctgtc 1870380
ggaaatacaa aagctcaaac aggaaaaaga tgccttcctg attaagaaaa acaaaatcga 1870440
ggagctccag ctcaaacgcc tccaagccgc aaaaatcctc gacagcctga atgaggccgt 1870500
ccccggaagc acctacctga cctcgctgga tgccgttacc gccgactctt atcggctcag 1870560
cggcaggaca tccagcgaca accgcgttgc cgccatgatg agggcgatgc ccaataccgg 1870620

catattcaag caacccgaat tgttaagcat caagaaaaac aattcgcatc aagaatttac 1870680
ccttcaggca acattacaac ccatcgtaaa ggcggccgaa tccaaagaga atccggcttc 1870740
gggaaacgca caggaggcaa actgaatggc ttctaaatca tctaaaacca acttggatct 1870800
caacaacctt cacctgctca accttcctgc caggcttttt atcgccctgc tggccgttgc 1870860
cgccgtgctg gggctcggtt atgccggatt gttcaaaagc cagatggaat cccttgagga 1870920
atacgaagca aaagaaaccg aactgaaaaa cacctacaaa cagaaaagta tcgacgcggc 1870980
cagcctgaac aacctgaggg acgaacttgc ctcaatccgc tctgccttcg atatcatgtt 1871040
gaaacagctg ccgacagatg cagaaattcc caatctggtt caagagcttc atcaggcagg 1871100
ttcgagcaac ggtctgcgct tggacagcgt tatgccccaa cctcccgtag atgacggccc 1871160
catcaaaaga ttaccctatt ccatttccat taccggaaat tacgaacaga tcagccaatt 1871220
tacccgcgat gtcggcagcc tctcccgaat cattaccctt gagtcgctga aatcgcccca 1871280
atctccggaa aacggcggca atcctgacgg caagagcagc atcctgaacc tcagcgccat 1871340
tgccaccacc taccaagcaa aatccgtaga agagcttgcc gcagaagcgg cacaaaatgc 1871400
cgagcaaaaa taacttacgt tagggaaacc atgaaacact atgccttact catcagcttt 1871460
ctggctctct ccgcgtgttc ccaaggttct gaggacctaa acgaatggat ggcacaaacg 1871520
cgacgcgaag ccaaagcaga aatcatacct ttccaagcac ctaccctgcc ggttgcgccg 1871580
gtatacagcc cgccgcagct tacagggccg aacgcattcg acttccgccg catggaaacc 1871640
gacaaaaaag gggaaaatgc ccccgacacc aagcgtatta aagaaacgct ggaaaaattc 1871700
agtttggaaa atatgcgtta tgtcggcatt ttgaagtccg gacagaaagt ctccggcttc 1871760
atcgaggctg aaggttatgt ctacactgtc ggtgtcggca actatttggg acaaaactac 1871820
ggtagaatcg aaagcattac cgacgacagc atcgtcctga acgagctaat agaagacagc 1871880
acgggcaact gggtttcccg taaagcagaa ctgctgttga attcttccga caaaaacacc 1871940
gaacaagcgg cagcacctgc cgcagaacaa aattaagaag aggattactc cattatgaat 1872000
accaaactga caaaaatcat ttccggtctc tttgtcgcaa ccgccgcctt tcagacagca 1872060
tcggcaggaa acattacaga catcaaagtt tcctccctgc ccaacaaaca gaaaatcgtc 1872120
aaagtcagct ttgacaaaga gattgtcaac ccgaccggct tcgtaacctc ctcaccggcc 1872180
cgcatcgcct tggactttga acaaaccggc atttccatgg atcaacaggt actcgaatat 1872240
gccgatcctc tgttgagcaa aatcagtgcc gcacaaaaca gcagccgtgc gcgtctggtt 1872300
ctgaatctga acaaaccggg ccaatacaat accgaagtac gcgggaacaa agtttggata 1872360
ttcattaacg aatcggacga taccgtgtcc gcccccgcac gccccgccgt aaaagccgcg 1872420
cctgccgcac cggcaaaaca acaggctgcc gcaccgtcta ccaagtccgc agtatccgta 1872480
tccgaaccct ttaccccggc aaaacaacag gctgccgcac cgtttaccga gtccgtagta 1872540
tccgtatccg caccgttcag cccggcaaaa caacaggcgg cggcatcagc aaaacaacag 1872600
gcggcagcac cagcaaaaca acaggcggca gcaccagcaa aacaacaggc ggcagcacca 1872660
gcaaaacaaa ccaatatcga tttccgcaaa gacggcaaaa atgccggcat tatcgaattg 1872720
gctgcattgg gctttgccgg gcagcccgac atcagccaac agcacgacca catcatcgtt 1872780
acgctgaaaa accataccct gccgaccacg ctccaacgca gtttggatgt ggcagacttt 1872840
aaaacaccgg ttcaaaaggt tacgctgaaa cgcctcaata acgacaccca gctgattatc 1872900
acaacagccg gcaactggga actcgtcaac aaatccgccg cgcccggata ctttaccttc 1872960
caagtcctgc cgaaaaaaca aaacctcgag tcaggcggcg tgaacaatgc gcccaaaacc 1873020
ttcacaggcc ggaaatctcc cttgacttcc aagatgtcga aatccgcacc atcctgcaat 1873080
tttggcaaaa gaatccggaa tgaacattgt tgccagcgac tccgtcaacg gcaaaatgac 1873140
cctctccctc aaggatgtgc cttgggatca ggctttggat ttggttatgc aggcgcgcaa 1873200
cctcgatatg cgccagcaag ggaatatcgt caacatcgcg ccccgcgacg agctgcttgc 1873260
caaagacaaa gccctcttac aggcagaaaa agacattgcc gatttgggtg cgctgtattc 1873320
ccaaaacttc cagttgaaat acaaaaatgt ggaagaattc cgcagcatcc tgcgtttgga 1873380
caatgccgac acgaccggaa accgcaacac gcttatcagc ggcaggggca gcgtgctgat 1873440
cgatcccgcc accaacaccc tgattgttac cgacacccgc agcgtcatcg aaaaattccg 1873500
caaactgatt gacgaattgg acgtacccgc gcaacaagtg atgattgagg cgcgtatcgt 1873560
cgaagcggca gacggcttct cgcgcgattt gggcgttaaa ttcggcgcga caggcaagaa 1873620
aaagctgaaa aatgatacaa gcgcattcgg ctgggggcta aactccggct tcggcggcga 1873680
cgataaatgg ggggccgaaa ccaaaatcaa cctgccgatt accgctgccg caaacagcat 1873740
ttcgctggtg cgcgcgattt cctccggtgc cttgaatttg gaattgtccg catccgaatc 1873800
gctttcaaaa accaaaacgc ttgccaatcc gcgcgtgctg acccaaaacc gcaaagaggc 1873860
caaaatcgaa tccggttacg aaattccttt caccgtaacc tcaatcgcga acggcggcag 1873920
cagcacgaac acggaactca aaaaagccgt cttggggctg accgttacgc cgaacatcac 1873980
gcccgacggc caaatcatta tgaccgtcaa aatcaacaag gactcgcctg cgcaatgtgc 1874040
ctccggtaat cagacgatcc tgtgtatttc gaccaaaaac ctgaatacgc aggctatggt 1874100
tgaaaacggc ggcacattga ttgtcggcgg tatttatgaa gaagacaacg gcaatacgct 1874160
gaccaaagtc cccctgttgg gcgacatccc cgttatcggc aacctcttta aaacacgcgg 1874220
gaaaaaaacc gaccgccgcg aactgctgat tttcattacc ccgaggatta tgggtacggc 1874280

```
cggcaacagc ctgcgctatt gatgcgtcaa aataagggca tatgttttac ggcatatgcc 1874340
ctttctttat gctttttgcc gcgaccgaaa tgccgtcatt cccgcgcagg cgggaatcca 1874400
gtccgttcag tttcggtcag tttcggtcat ttccgataaa ttcctgttgc ttttcatttc 1874460
tggattccca cttttgtggg aatgacggcg gaaggggtaa atcctcacaa cccaaagcct 1874520
cgtcatttcc acaaaaaaca gcaacccgaa acagcaactt aaaaccccgt cattcccgcg 1874580
caggcgggaa tctaggtctg tcggttcagg aacttatcgg ataaaacggt ttctccaacc 1874640
ctgcgttcta gattcccact ttcgtgggaa tgacgggata tgggtttccg tgcggacgtg 1874700
ttcggatttc cgcctgcgcg ggaatgacgg cgacagatgc ccaacggtct ttatagtgga 1874760
ttaacaaaaa tcaggacaag gcgacgaagc cgcagacagt acaaatagta cggaaccgat 1874820
tcacttggtg tttcagcacc ttagagaatc gttctctttg agccaaggcg aggcaacgcc 1874880
gtactggttt ttgttaatcc actatagtat tgataaacat attatcttca atatattcaa 1874940
ttggataatt gtttacctaa gcaaagataa ttgccttttc ctgacaaata agtgaaatca 1875000
acggattgtc aaaacacagc ctgaaataaa aaacctccct gatttctttt atttgtcctt 1875060
aaaatcagaa aggttcggga tggtcgggtt atttttccaa acgtaccgcc gccctgccga 1875120
tttcgtataa aattccgccg taacccgaca agcccgaacc ctgtcgcccc gaaaggcggg 1875180
gtgtcaaaca ttaaggaatt gtgatgaaaa actttaacgg caaactcatc ctcatcggac 1875240
tgatgggcgc gggcaaaacc acgctgggcc ggcaaatggc gcagcggctg gattaccgtt 1875300
tttacgacag cgatcacgaa atcgccgcag ccgccggcgt tcccatcccc accatatttg 1875360
aaatggaagg cgaacaggga ttccgttcgc gcgaaaccgc catactcaaa aagctggtta 1875420
tcctgcccca tatcgtcctg tccaccggcg gcggcgcggt gttaaaagaa gaaaaccgcg 1875480
cccttatccg caaaagcggc acggtcgtct atctgcacgc cccgcccgaa accctgctcg 1875540
aacgcacgcg ctgcgacaac agccgtcctt tgctgcaagt tgccgatcct ttggcgaaat 1875600
tacgtgaact ctacgccgca cgcgaccccg tttaccgcca aaccgccgac tttaccgtag 1875660
aatcggcaaa ctgccgggaa accgtgcaaa ccctgctcaa acgcttatcc cgataaaccg 1875720
gcatatgcgc cgcgcccaga aaaccaaacc gcgccccgcc cggcgggccg gcggttcaaa 1875780
ctttaaggaa caacaatgaa aacactgacc gtacacaccc cgtcccacag ctaccccatc 1875840
tttatcggca acggactgct gccgcaggca ggaagcctgc tcaaaccgca tttgggcaaa 1875900
cgcgccgcca tcatcgccaa cgaaaccgtc gccccgctct acctcggcac gcttcagacg 1875960
gcattggatg cggcaggcgt atcccatttc agcatcatcc tgcccgacgg cgaggcgcac 1876020
aaaaactggc agacgctcaa cctcatcttt gacgggctga tgcaaaaccg cgccgaacgc 1876080
aaaaccacat taatcgcact gggcggcggc gtgatcggcg acatggtcgg ctttgccgct 1876140
gccacctacc agcgcggcgc accgttcgtt caaataccga ccacgctgtt gagtcaggtc 1876200
gactcatcgg tgggcggcaa aaccgccatc aaccacccgc tcggcaaaaa tatgattggc 1876260
gcgttttacc agccgcaggc ggtgcttgcc gacttggaca cgctgcacac cctgcccgcc 1876320
cgcgaattgt ccgccggtat ggcggaagtc atcaaatacg gcgcgctcgg cgacatcggc 1876380
ttttttgaat ggctggaaca gcatatgccc gaactgatga cgctcgatcg ggaaaaactc 1876440
gcccaagccg tgtaccgctg ctgccaaatg aaggcagaca tcgtcgccca agacgaaacc 1876500
gaacagggca tacgcgcatg gctcaacctc ggacacacct tcggacacgc cattgaaacc 1876560
gagatgggtt acggcacttg gctgcatgga gaagccatcg ccgccggctg cgtgttggcg 1876620
gcgcgtttgt ccgaacaact gggcaaaacc tccgccgcag ataccgcgcg gctcgccgcc 1876680
ctgctcgaag ccgccggact gccgtccgcg ccacccgtgt ttgcctttga aaaatggctg 1876740
gaacacatga gccacgataa aaaagtcagc ggcggcatca tgcgctttat cggtctgaac 1876800
cggctgggcg aagccaacat caccgaaatt accgacacgg acatcctccg ccgcaccctg 1876860
caaccgtatc tctgatttcc tctgccgatg tgctgccgcg cgggtttgac gcacgatgat 1876920
gattttccat catctttctc cgcaaaagcg ggaatccagt ccgttcggtt tcggtcgttt 1876980
ccgataagtt cccgttgctt ttcatttcta gattcccact ttcgtgggaa tgacggcgga 1877040
gaggtttttg ttgtttcgga gaagtttctg caaccctaga atctcgttat ttccacaaaa 1877100
aacagaaaac caaaacagca acttaaaacc tcgtcattcc cgcaaaagcg ggaatccggt 1877160
ccgttcggtt tcggtcgttt ccgataaatt cctgctgctt ttcatttcta gattcccact 1877220
tttgtgggaa tgacggcgga agggttttgg ttttttccga taaattcttg aggcattgaa 1877280
attctatagt ggattcacaa aaatcaggac aaggcgacga agccgcagac agtacagatg 1877340
gtacggaacc gattcactcg tgcttcagca ccttagagaa tcgttctctt tgagctaagg 1877400
cgagacaacg ccgtaccggt ttttgttcat ccactataac agcaaccctg tcgccgtcat 1877460
tcccgcaaaa gcgggaatcc agtccgttcg gtttcggtcg tttccgataa gttcccgttg 1877520
cttttcattt ctagattccc actttcgtgg gaatgacggc ggaagggttt tggtttttttc 1877580
cgataaattc ttgaggcatt gaaattccag attcccgcct gcgcgggaat gacggctcaa 1877640
aagttacgga acgaaaaaca accaaaaccg gacaagtcgg attcccgcct gcgcgggaat 1877700
gacggaatct taagtttccg tctttgtttt ctgttttctg ttttcgaggg aataatgggg 1877760
aacaagccgt atttcagacg gcattttcag ttcggggtat aatccgaata cttgcgacca 1877820
tctgaatcat tgggacaaac catgtgtcaa ctgctgggca tgaactgcaa tacgccgacc 1877880
gatattatgt tttcctttga aggcttccgc cgcaggggcg gcattaccga ccaccatgcc 1877940
```

gacggtttcg gtatcggctt tttcgaaggc aaaggcgtgc gcctgttcca cgacgacaag 1878000
ccgagcgtaa attcccccgt cgccgacctc gtgcgtgcct accaaatcaa atcggaaaac 1878060

gtcatcgcac atatccgcaa agcatcgcaa ggacaaacct cgctggcgaa cacccatccc 1878120
tttatgcgtg aaatgtgggg cggctactgg ctgtttgccc acaacggaca tttgattgat 1878180
tttttccccg aacagggcga atttttccac cccgtcggca caaccgattc cgaacgcgcg 1878240
ttctgccaca tcctcaaccg cctgcgcacc cgctttgccg cccgtcccga cgacgacacg 1878300
ctgtttgacg cgattgcggg gctgacgcac gaaatccgca agttcgggct gtttaacttt 1878360
atgctttcag acggcattgc cctgtttgcc cacgccagca cgctgctgca ctacatcgtc 1878420
cgccaagccc cgttcggcaa ggcgcgcctg ctcgacgacg acgtgatggt cgattttgcc 1878480
gaagtaacca cgccctccga ccgcgtcgcc gttatcgcca ccctgccact gacccgcgac 1878540
gaatcatggt cccaacttgc cgtggacgaa ctggtcatgt tccgcgaagg caacatcgtc 1878600
cgacacgacc gtcccgaaaa ccccgtctat atgagtgccg aagaaggtct ggaaatcgcc 1878660
cgcgccgccg gcgtcgccgt ctgaacttca gacgacatag gaggacgaac ccgatgaaat 1878720
gcccgttttg cgcccacccc gacacccgcg ttgccgattc gcgtctgatg gaagaacgca 1878780
acgccgtgcg ccgccgccgc cactgcccca actgcggcaa acgcttcggc acgctcgaaa 1878840
ccgccgaact caaaatgccc gccgtcatcg gtccggacaa aaaacgctcg ccctttaatg 1878900
cacaacgcct ccgcaacgac ctgaccgccg ccgcccgaaa atccgccctg acacccgaac 1878960
agatcgacga aaccgtccgc ctgacggaac acaggctcta cacttcgggt cagcgcgaca 1879020
tcccctctgc cgcacttgcc gacatggtgc taaaagaact gctccgacaa gatacggaag 1879080
ccgccgtccg tttcgccgcc ctgcacaaac gcttcgacaa tccggcagac tttgcctcgt 1879140
ggctggcgca agccgtcaaa acaggcggca aagcctgatt cccccaaccc atactgatac 1879200
ggtatcccta tgttttcgga cacagatata tccatgatgg aaaacgccct ccgacttgcc 1879260
gctttggggc gtttttccac ttcgcccaat ccgcgcgtcg gctgcgttat cgcacacggc 1879320
agccaaattg tcgggcaagg cttccacgtc aaagcgggcg aaccccatgc cgaagtccac 1879380
gccctgcgtc aggcgggcga aatggcacaa ggcgcgaccg cctttgttac cctcgaaccg 1879440
tgcagccatt acgggcgcac accgccctgt gccgaagcac tggtgcgggc gggcgtgtcc 1879500
cgcgtcgttg ccgccatgcg cgaccccaac ccgctggttg caggcaaagg gcttgccctg 1879560
ctcgaagcag caggcatcaa gacggaatgc ggtttactcg aacatcaggc aagggaactc 1879620
aaccgaggct tcctgtcgcg catcgaacgc cgccgcccct ttgtccgcct caaatgcgcc 1879680
gtttcgctgg acggcaaaac cgccctttca gacggcagca gcttttggat taccggcgaa 1879740
gacgcgcgtg ccgacgtaca ggttttgcgt gccgaaagct gcgcggtgct gaccggcatc 1879800
ggcacggtgt tggcggacaa tccccggctc aacgtccgcg cttttccaac tttgcgccaa 1879860
cccgcacgca tcgttttaga cagccgcctg cgcctgcccc cgaacagcca tttggttacc 1879920
gacggacaat ctccgaccta catcgccaca ctcgaacgca acgaagacag actgcacccc 1879980
tatcgggaac acgcacacgt ccgcatcctg atgccgtctg aaacggcaga cagcaaaatc 1880040
gacctgcacc acctgatgcg cctccttgct gacgaaggtt tcggcgaaat catggtcgaa 1880100
gcaggctccg aactcacatc cgcatttttg gcagaaaatc tggcagacga aatcgtcctg 1880160
taccgttcgc ccaaaatcct cggcagcggc aaagacctgt tttccctgct cgaaaaccgc 1880220
gccgcccttt ccgcaccgcc cttgtggaca cccgtttcaa gcgaaatcct cggacacaac 1880280
atcaaaaccg tgttccgaaa aaacggcaac gccttttaaa gggtttgcgc cgtttcacta 1880340
tataataacg ccgataaaaa acggccccgt tcagaccgcc gcccgcccga aaaaacgcaa 1880400
cccggactgc cgcaccccgc cggcagccgc gacggtctga aagccgtcaa attccgatca 1880460
agaaaggctt cagacggcac aggcagcatc ccgccgccgc ccggacatca aaaatggaca 1880520
caaaagaaat cctcggctac gcggcaggct cgatcggcag cgcggtttta gccgtcatca 1880580
tcctgccgct gctgtcgtgg tatttccccg ccgacgacat cgggcgcatc gtgctgatgc 1880640
agacggcggc ggggctgacg gtgtcggtgt tgtgcctcgg gctggatcag gcatacgtcc 1880700
gcgaatacta tgccaccgcc gacaaagaca ccttgttcaa aaccctgttc ctgccgccgc 1880760
tgctgtctgc cgccgcgata gccgccctgc tgctttcccg cccgtccctg ccgtctgaaa 1880820
tcctgttttc actcgacgat gccgccgccg gcatcgggct ggtgctgttt gaactgagct 1880880
tcctgcccat ccgctttctc ttactggttt tgcgtatgga aggacgcgcc cttgcctttt 1880940
cgtccgcgca actcgtgccc aagctcgcca tcctgctgct gctgccgctg acggtcgggc 1881000
tgctgcactt tccagcgaac accgccgtcc tgaccgccgt ttacgcgctg gcaaaccttg 1881060
ccgccgccgc ctttttgctg tttcaaaacc gatgccgtct gaaggccgtc cggcacgcac 1881120
cgttttcgcc cgccgtcctg caccgggggc tgcgctacgg cataccgatc gcactgagca 1881180
gcatcgccta ttgggggctg gcatccgccg accgtttgtt cctgaaaaaa tatgccggcc 1881240
tggaacagct cggcgtttat tcgatgggta tttcgttcgg cggggcggca ttattgttcc 1881300
aaagcatctt ttcaacggtc tggacaccgt atattttccg cgcaatcgaa gaaaacgccc 1881360
cgcccgcccg cctctcggca acggcagaat ccgccgccgc cctgcttgcc tccgccctct 1881420
gcctgaccgg cattttctcg ccccttgcct ccctcctgct gccggaaaac tacgccgccg 1881480
tccggtttat cgtcgtatcg tgtatgctgc cgccgctgtt ttgcacgctg gcggaaatca 1881540

gcggcatcgg tttgaacgtc gtccgcaaaa cgcgcccgat cgcgctcgcc accttgggcg 1881600
cgctggcggc aaacctgctg ctgctggggc ttgccgtgcc gtccggcggc gcgcgcggcg 1881660
cggcggttgc ctgtgccgcc tcattctggc tgtttttgc cttcaagacc gaaagctcct 1881720
gccgcctgtg gcagccgctc aaacgcctgc cgctttatct gcacacattg ttctgcctga 1881780
cctcctcggc ggcctacacc tgcttcggca cgccggcaaa ctatcccctg tttgccggcg 1881840
tatgggcggc atatctggca ggctgcatcc tgcgccaccg gaaagatttg cacaaactgt 1881900
ttcattattt gaaaaaacaa ggtttcccat tatgaaaatc gttttgacca catctatggc 1881960
aggcttgggc ggcacgggca cgatatcatc gattgccaaa tggcgtgcaa aaacgacccc 1882020
gttcagtccg acgaaatcgt ccgccgcttc aggcgcgaca tttcctatcg gaaaatcgtc 1882080
aacctgattg aaagattggc aaatgagtaa attcttcaaa cgcctgtttg acattgttgc 1882140
ctccgcctcg ggactgattt tcctctcgcc agtatttttg attttgatat acctcatccg 1882200
caagaatcta ggttcgcccg tcttcttctt tcaggaacgc cccggaaagg acggaaaacc 1882260
ttttaaaatg gtcaaattcc gttccatgcg cgacgcgctt gattcagacg gcattccgct 1882320
gcccgacgga gaacgcctga caccgttcgg caaaaaactg cgtgccgcca gtttggacga 1882380
actgcctgaa ttatggaata tcttaaaagg cgagatgagc ctggtcggcc cccgcccgct 1882440
gctgatgcaa tatctgccgc tgtacgacaa cttccaaaac cgccgccacg aaatgaaacc 1882500
cggcattacc ggctgggcgc aggtcaacgg gcgcaacgcg ctttcgtggg acgaaaaatt 1882560
cgcctgcgat gtttggtata tcgaccactt cagcctgtgc ctcgacatca aaatcctact 1882620
gctgacggtt aaaaaagtat taatcaagga agggatttcc gcacagggcg aagccaccat 1882680
gccccctttc acaggaaaac gcaaactcgc cgtcgtcggt gcgggcggac acggaaaagt 1882740
cgttgccgac cttgccgccg cactcggccg gtacagggaa atcgtttttc tggacgaccg 1882800
cgcacaaggc agcgtcaacg gcttttccgt catcggcacg acgctgctgc ttgaaaacag 1882860
tttatcgccc gaacaatacg acgtcgccgt cgccgtcggc aacaaccgca tccgccgcca 1882920
aatcgccgaa aaagccgccg cgctcggctt cgccctgccc gttctggttc atccggacgc 1882980
gaccgtctcg ccttctgcaa cagtcggaca aggcagcgtc gttatggcga aagccgtcgt 1883040
acaggcaggc agcgtattga aagacggcgt gattgtgaac actgccgcca ccgtcgatca 1883100
cgactgcctg cttaacgctt tcgtccacat cagcccaggc gcgcacctgt cgggcaacac 1883160
gcatatcggc gaagaaagct ggataggcac gggcgcgtgc agccgccagc agatccgtat 1883220
cggcagccgc gcaaccattg gagcgggcgc agtcgtcgta cgcgacgttt cagacggcat 1883280
gaccgtcgcg ggcaatccgg caaagccgct gccgcgcaaa aaccccgaga cctcgacagc 1883340
ataagcgatt aaaatacacc cccgtacaga ccgatttga caacacctgc ggcgcgcgcc 1883400
cgattcttcg gaacacgccc cccttcagac ggcatagggt cggaaatgcc gtctgaaaac 1883460
cgacggacaa accatcatgc tgaacacttt cctttccccg tggccctgct tcacccaaga 1883520
agaagccgat gccgtttcca aagtcctgct gtccaacaaa gtcaactact ggacgggcaa 1883580
cgaatgccgc gaatttgaaa aagaatttgc cgcctttgcc ggcacgcggt acgccgtcgc 1883640
ccttgccaac ggcacgctgg cactcgatgt cgcgctcaaa gcaatgggca taggcgcggg 1883700
cgacgatgtg attgttacct cgcgcacctt cctcgcttcc gcgtcctgca ttgtgaacgc 1883760
gggcgcaaac cccgtgtttg ccgatgtgga tttgaacagc caaaacatca gcgcggaaac 1883820
cgtcaaagcc gcgctgacac cgactaccaa agccgtcatc gtcgtccacc tcgccggtat 1883880
gcccgccgaa atggacggca ttatggcttt ggcaaaagaa cataatcttt gggtaatcga 1883940
agactgcgcc caagcgcacg gcgcaaaata caaaggcaaa tccgtcggct ctatcggaca 1884000
cgtcggcgcg tggtcgttct gccaagacaa aatcatgacc accggcggcg aaggcggtat 1884060
ggttacgacc aacgacaaaa ccctgtggga aaaaatgtgg tcgtacaaag accacggcaa 1884120
aagctacgat gccgtgtaca accacgaaca cgcgcccggt ttccgctggc tgcacgaaag 1884180
tttcggcaca aactggcgta tgatggaaat gcaggcggtc atcggacgca tccagctcaa 1884240
acgcctgccc gaatggacgg cgcgccgccg agaaaacgcc gccaagctgg cggaaagttt 1884300
gggcaaattc agcagcatcc gcttggttga agtcgccgac tacatcggac acgcgcaata 1884360
taagttctac gccttcgtca aacccgaaca cctcaaagac ggctggacgc gcgaccgcat 1884420
cgtcggcgaa ctgaacgcgc gcaaagtccc ctgctatcaa ggcagctgct ccgaagtcta 1884480
tttggaaaaa gccttcgaca acacgccgtg gcgaccgaaa gagcgtttga caaatgctgt 1884540
cgagttgggc gacaccagcc tgatgttctt ggtgcacccg acgctgaccg acgacgaaat 1884600
tgcgttttgc aaaaaacaca tcgaagccgt cttgaccgaa gccgcacgat aacccttcag 1884660
acggcatatg ccgcctgaaa acacataccg ccccacgata tgaatctgga aactctgatc 1884720
gccctgccgc gcaacatcaa gaaaatctgt ttcctcatac acgattttct gatgattttc 1884780
attgcctttt ggttcaccca aagcctaaag gccgactact cggacgaatg gttcgatttt 1884840
gccaactggc agtctttttt gctgactgcc ttgctgacca tcacattatt tatccgaatg 1884900
gggctttacc acgccgttac acgcttcgtc agcttccgca tcctcaccac cgcactggcg 1884960
ggcagcctcg cctccgccgt gttgtttttc ctcaatacgc tgatatttga agaaaggctg 1885020
cgcctcgccc tgccgattgt ctatttctta ctgctgtttg tttccgtgac cggctcgcgt 1885080
atggttttgc gcggactgtt gtccgaacac cccaaaaaac agatgatccc tgtcatcatt 1885140
tacggcgcgg gacggtcggg cagacaactg cttgaggccg tcaaacaaat gcgcgaatat 1885200

tccgccgccg cctttgtaga cgacgacccc aaactgtggc acaccgtcat ctacgacctt 1885260
gccgtttacc agcccgatgc catcgccttc ctcatcgaac gctacggcgt ggaaaaaatc 1885320
ctgctcgcca tccccggcgc gacccaggaa caacgccgcc gaatcatcaa caaactggaa 1885380
gcctatccgt gcgaagtgtt gaccattccc ggaatgaaag acctgatgga cggaaaaatc 1885440
agcatcggca cgctcaaaaa aatctctgtg tccgacctgc tcgggcgtga ttccgtcgcg 1885500
cccgacgacc gcctgatgag tgccgacatc gaaggcaaaa ccgtcatggt aaccggcgcg 1885560
ggcggctcca tcggttcgga actctgccgc cagattatcc gccgccgccc cgaaaagctg 1885620
ctgctgttcg agttatccga attcgccctg tacgccatcg aaaaagaatt gcgcgaaacc 1885680
tgcatccaaa aacgcctcga caccgaaatc ctgccctttc tcggttcggt gcaaaaccgc 1885740
acgctgctcg aacacgtcat gaccgccttt tccgttgcga ccgtctatca cgccgctgcc 1885800
tacaaacacg tccccatggt cgagttcaac accgtcgaag gcatacgcaa caacatcttc 1885860
ggcacactcg agtgcgcgct tgccgccacg acatcgggcg taagaacttt cgtcctcatc 1885920
tccaccgaca aagccgtccg ccccaccaac accatgggtg ccagcaaacg catggcggaa 1885980
ctctgccttc aggcactcgc cgccgaaccc ggacaaaaaa cccgcttcag catggtacgt 1886040
ttcggcaatg ttttaggttc gtccggctcc gttgtcccgc tgtttgaaaa acagattgca 1886100
gaaggcggcc cgcttaccct gacccacccc gaaatcacac gttatttcat gaccataccc 1886160
gaagccgccc aactcgtcat acaggcaggc gcgatgggta cgggcggcga cgtattcgtc 1886220
ctcgacatgg gtgaatccgt caaaatcatc gaccttgccc gccaaatgat taccctaagc 1886280
ggcctcaaac ccaacacacc cgaacaaccc gacggcgaca tcgaaatcct cattaccgga 1886340
ctgcgtcccg gagaaaaact ctacgaagag ctgctcatcg gcgacaacgt ccgcaaaacc 1886400
ggccatccgc gcatcatgac cgccaacgag accatgctgc cgtggcacga gctctccgcc 1886460
ctgctcgacc gcatccgtgc ggcctgcgac cgttacgacc agcaggcaat ccgcaccctg 1886520
ctcatcaacg ccccgaccgg ctttgccccg agcgacggca tctgcgacct gctttgggta 1886580
cgagaaacac acagaaaaaa tgccgtctga accttcagac ggcataacgt acaaaccaac 1886640
ctaccttaca cacacggagt ttgacatgca gttctcagca ttcggcgaaa aattcacgca 1886700
acacagcggc atcctccaac tgatggacga cctcggcgac gcgctcaaaa gcgacaagcc 1886760
cgtcaacatg ctcggcggcg gcaacccggc gcgcattccg gaaatcgatc aggcgttcgc 1886820
cgacatattc tccaaactgg cggcagaaca cgccgtcgaa aacatcggca actactccaa 1886880
tccccaaggc gatgccgtgc tgattgacgc gctgaccgcc ttcctcaacc gcgaatacag 1886940
ctggaatctg accgccgaca atatcgcgct gaccaacggt tcgcaaaacg cgtttttcta 1887000
tcttttcaac ctcttcggcg gcaaattcaa cctttcagac ggcacatccg cagaaaaagc 1887060
cattttgttg ccgctcgcgc ccgaatacat cggctatgcc gacgtgcatg tcgaagggca 1887120
gcacttcgtt tccgtcaagc ccaaaatcga aaacgtcgaa cacgaaggcg aagccggctt 1887180
cttcaaatac cgcgtggact ttgacgcact ggaaaacctg cccgaactca aagcgggcaa 1887240
aatcggcgcg atttgctgtt cgcgcccgac caacccgacc ggcaatgtgt tgaccgacgg 1887300
cgaaatggcg cgtttggacg ctttggcgcg tgaacacggc attccgctga ttatcgacaa 1887360
cgcctacgga atgccgttcc ccaacatcat ttacagcgac gtaacgctga attggcacga 1887420
aaacatcatc ctctgcttca gcctgtccaa agtcggcctg ccgggcgtgc gcaccggcat 1887480
catcgtcgcc gcgcccgaag tcgtcaaagc cgtcagcagc ctgaacgcga ttgtgaacct 1887540
tgcccccacg cgcttcggcg cggccatcgc aacgccgctg ctggaaagcg gcgagatgaa 1887600
acggcttgcc gaccaagtca tccggccgtt ttaccgcaat caggcgcaaa ccgccgtctc 1887660
gctgctcaag cgggagctgg gcgcgtaccc gatgaaaatc cacaaacccg aaggcgcgat 1887720
tttcctgtgg ctctggtttg aaaacctgcc cgtttcttcg caaaccctgt acgaaatgct 1887780
caaagccgaa ggcacactga ttattccggg cgaacatttc ttcgtcggca tcgacacgca 1887840
ggattacccg catgcgggcg agtgcatccg catgagcatc gcgcaggacg ctcaaacgct 1887900
ggaaaaaggc attgccgcca tcggtaaaac cgtccgaaaa ctgtacgaca acgtttaaaa 1887960
cgcaaaaaat gccgtctgaa aagttttcag acggcatttt tatctgcatt caatatcggg 1888020
aaaaatgttc ccaaaccggt ttgcagtttt ccggcagctc gggacacgcg ccgaggatgc 1888080
cgccgctgaa gtcgtttaag cggtggaagt cgctgcccgc gctggcgagc ataccgaagc 1888140
gttctgccaa aagcgcgtag ttgaggcggt cgtttttgca gcagtttccg ctgtggactt 1888200
cgatgcctgc gccgccgagg tttttaaatt cttcaaacaa attgcgcttg gcggtggcgg 1888260
acaaatcgta gcgcatgggg tgggcgatga ctgccatgcc gcccgctccg ttgacggcgg 1888320
agacgcagtc ttccagcgtc gcccattcgt ggcggacggc gcaggatttg ccgtcgccca 1888380
agtatttggt gaacgcctgc tgcttgtttt tgacgtgtcc cgcttggatg aggaactcgg 1888440
cgacgtgggt gcggctgacc atttctttgt ttgccgccag tgccagcgcg ccgtcgtatg 1888500
cgccgccgat gcctttcttt tcgagcttgg cggcgatggc ttcaagacgt ttcagacggc 1888560
ctttccgcac ttgcgccaac aggttttgca ggttttcgtc ctgctcgtcg aaatccaaac 1888620
cgacaacgtg tatggtgcgc ccgcgccacg ttacggagat ttccacaccg ttaatcaggc 1888680
gcaaaccgag cttgtcggct tcggcgcgcg cttcggcgat gccgccggtg tggtcgtggt 1888740
cggtcaacgc cagcagcgtg cagccgtttt gatgcgcgag gcgcacgact tcggcggggg 1888800
agagcatacc gtcggaaacg gtggaatggc agtgcaggtc tatcatgggg tgttatgtgt 1888860

gttgtgaatg aaggtcgggg gttaatatta tcggttggtg tggatacagc ggtgatttca 1888920
acaaacaggt gtatggcaaa tgcaaaggaa aagtccctat gccgtcattc ccgcgcaggc 1888980
gggaatccag accttgattt gtcaaaaata tttaaggtta accgctattt cgaacttccg 1889040
gattcccgcc tgcgcgggaa tgacgatatg gacgttttca gttttaatct actataaaag 1889100
actgtctgaa aacgtggttt tatagtgaat taaatttaaa ccggtacagc gttggctcgc 1889160
cttggctcaa agagaacgat tctctaaggt gctgaagcac caagtgaatc ggttccgtac 1889220
tatttgtact gtctgcggct cgccgccttg tcctgatttt tgttaattca ctatatcaag 1889280
ccgaaccgtt tcagacggca tcgtccgacc aacccgcttc tttcaatttc tgccgttgca 1889340
cgtcgtattt ggctttatcc gcccagtaaa tcgtctgaat gcacgcctcg ccgcagtcgc 1889400
tgccgcagca ttcccacgac tcgggtcgga cgggttcgtc taaaagcggc tcgcccaaaa 1889460
gggcttcggc tttatacttc agggtcgtat ccatcggcga tttccaagcg agcgccgtca 1889520
aactcgatga cttcgccgcc gcgtattttg gcggttttac gggtttcggt ttcgccgttg 1889580
cgcaacacca gcccttcggc gataaacgct ttcgcctgtc cgccgctttc ggcaagtccg 1889640
accaatttca agaggtcgca caaggcgatg tattcgttgt cttcgagata gacagtggct 1889700
tccataatgt tcccttgcag aaagaggccg ttattgtagc acctgccgcc gccgtaccca 1889760
aaattaccga aaaaccggcg atgtatccgc accgcctgtt ccgtaaaagt aaaaatgccg 1889820
tctgaaaccc catatgccgc catccgttca aagaaatcct gcccaacggc agactgcaaa 1889880
tcctgttccc cgacgaatcc gcattgacgc tgatgcacat cctcaaacgc gaactgcccg 1889940
atacaccggc aatcggcatc aaaacgaaat caaaaacctg tttgaacgta aaattaattt 1890000
aactgcttgc catccgtccg aataaggcat atagttcttt ataactagtt tgatagtcct 1890060
ttatatctat caatactcct tgggaagcct ccgccatacg gcaggaggca tttttttgcc 1890120
gtagtaaaag ctcaaaaaca tttgcaggtc atgccgtctg aacccgaaac ggcattacct 1890180
acaccgccat ctaaagacaa ccctgctaca atacgccttt tattgtccac gccgattttg 1890240
ccatgaccga gccgacctac attcccctgc gcctgcatac cgaattttcg attaccgacg 1890300
gtatggtgcg gattaaaaaa ctgattgcca aagcgcagga atacggtttg cctgctttgg 1890360
gcatcagcga tttgatgaac gaattcggtt tggtgaaatt ttataaagcc tgccgcagcg 1890420
cggggattaa gcctatcggc gcggcggatg tgcggatagg caatccggat gcgcccgaca 1890480
agccgttccg cgctatgctg attatccgta acgatgcggg ctatctgcgc ttgagcgagc 1890540
ttctgacggc ggcttatgtc ggcaaagacc gcaatgtcca tcatgcggaa ctcaatcccg 1890600
aatggctgga aaacggcgac aacagcggct tgatttgttt gagcggcgca cattacggcg 1890660
aagtgggcgt gaatctgttg aacggcaatg aagacgcggc gcgtacggcg gcgttgaagt 1890720
atgcggcgtg gttccccgat gcgttctata tggagctgca acgcctaccc gaacgccccg 1890780
aatgggaggc ttgcgtttcg ggcagcgtga agctggcgga ggaattgggt ttgccggtgg 1890840
tggcgacgca tccgacacag tttatgagcc gcgacgattt caacgcgcac gaggcgcgag 1890900
tgtgtatcgc aggcggctgg gtattgacgg acaagaaacg tccgcgcgat ttcacgccgg 1890960
gccagttttt cattccgccg gaaaccatgg ccgaacgttt cgccgatttg cctgaagcct 1891020
tggaaaacac ggtagaaatt gccaaacgct gcaacctgca catcacattg ggcaaaaact 1891080
tcctgcccct tttccccacg cccgacggtt tatcactcga tgactatctc atcaaactct 1891140
ccaacgaggg tttgcaggaa cgtatggttc agctttatcc cgacgaggcg gagcgggcgg 1891200
caaaaatgcc ggaatatcag gaacgtttgg attttgagct gaacatcatc atccaaatga 1891260
aattccccgg ctatttcctt atcgtacaag actttatcaa ctgggcgaaa acacacggct 1891320
gtccggtcgg gccgggccgt ggttcgggcg cgggttcgct ggtggcgtat tcattgaaga 1891380
ttaccgacct tgatccgctc aaatacgcgc tgctgttcga gcgtttccta aaccccgaac 1891440
gcgtttctat gcccgacttc gacgtggact tttgccaaag caaccgcggc cgcgtgattg 1891500
aatatgtgcg cgagaaatac ggcgcggagg cggtcagcca gattgttacc ttcggcacga 1891560
tgtcgtccaa agcggtcatc cgcgacgtcg ggcgcgtgtt agagctgccg tttatgctgt 1891620
gcgacaaact gtccaagctg attccgttgg aagccaacaa acccctgagt ttggaaaaag 1891680
ccatggagac cgagccacag attcaggaat taatcgaagc ggaagaagcg gacgaactga 1891740
ttacgctggc gaaaaagctg gaagatttaa cgcgcggttt gggtatgcac gcaggcggcg 1891800
tgttgattgc gccgggcaag atttccgatt acagccccgt gtatcaggcg gacgaatccg 1891860
cctcgcccgt atccatgtac gacaagggcg acgtggaaga tgtgggtttg gtgaaattcg 1891920
actttttggg tctgcgcaac ctgaccatta tcgaaatggc gcagaacaac atcaaaaaca 1891980
ctaccggcga catcatcgat gtcggcaaaa tcccgcttga cgaccaggtc gcctaccaaa 1892040
tcttccgcga tgcgaacacc accgccgtct tccagtttga gtcgaccggc atgaaaaaaa 1892100
tgctgaaaac ggcgcacacg accaagtttg aagaactcat cgccttcgta tcgctctacc 1892160
gccccggccc gatggacaac attcccgact tcgtcgcacg tatgaagggg caagaattcc 1892220
aatacatcca tccgctactc gaaggcatcc tcgcgccgac ctacgggatt atggtgtatc 1892280
aggaacaagt gatgcaggcg gcgcaaatta tcggcggcta ctcgctcggc ggcgcggacc 1892340
tgctgcgtcg cgccatgggt aagaaaaaac ccgaagaaat ggtgaaacac cgcgaaatct 1892400
tcgccgaagg cgcggcaaaa caaggcattt cgcgcgaaaa atccgacgaa atcttcaact 1892460
acatggaaaa attcgccggc tacggtttca acaaatccca cgccgccgcc tacgccctga 1892520

tttcctacca gaccgcatgg cttaaagcgc actaccccgc cgaatttatg gcggcgacca 1892580
tgtcgtccga attggacaac accgaccagc tcaagcattt ctacgacgac tgccgcgcca 1892640
acggcattga gttcctgccg cccgacatca acgaatccga ctaccgcttc acgccgtatc 1892700
cggacatgaa aatccgctac gcgctcggcg cgattaaagg cacgggcgaa gccgccgtcg 1892760
aatccatcac cgccgcgcgg caaagcggcg gcaagtttac cggtctgttg gacttctgcg 1892820
agcgcgtcgg caaagaacac atgaaccgcc gcaccctcga ggccctgata cgcggcggcg 1892880
cgttcgacag catcgaaccc aaccgcgcca tgctcttggc gaacatcgac ctcgctatgg 1892940
acaacgccga ccaaaaagcc gccaacgcca atcagggcgg gcttttcgac atgatggaag 1893000
acgccatcga accggtgcgg ctcatcgacg cgccgatgtg gagcgaatcg gaaaaactcg 1893060
ccgaagaaaa aaccgtcatc ggcttttacc tgtccggcca cccgttcggc ccgtatgccc 1893120
aagaagtccg ccaaatcgca ccgaccaaat tagaccgtct gaagccgcaa gacagcgtgc 1893180
gcctcgccgg attcgttacc gccgtgcgta cgatgatggg caaacgcggc aaaatcgcct 1893240
tcgtcagcct cgaagatttg agcggacagg ttgaaatcat ggtcggcggt cagacgttgg 1893300
aaaactgcgc cgactgcctc aaagccgacc aagtgctgat tatcgaatcc aaagtcagcc 1893360
gcgacgacta cggcggcggc gacgggctgc gtattctggc aaaccaagtc atgaccctgc 1893420
aaacggcgcg cgaacgctac gcccgcagcc tcagcctcgc cctcgccccg catcacgaca 1893480
tcggcggact ggtacggctg ctcgccgccc accaactgcc cgacacgccg cgcatcccgc 1893540
tgcaactgtc gtatgccaac gaaaaagcgt cgggcaggct tcaagtgccg ccgaaatgga 1893600
cggttacacc gagctccgca ttgttcggcg aactggaaac attgctcggc agccggtcgg 1893660
tgcgcgtcaa ctggtaaccc aaaatataaa tgccgtctga agcccaaaaa ccggtttcat 1893720
tcgtacttta ttcgaatgat tgaataaaag taactgccaa gaaaaacgta ttttttggtt 1893780
atttcgccag tctaaataga gcaaccggga cgattgatat ccgtgtgcat gacacagaca 1893840
gcaccaaagg gaaaaacggc attttccaaa gtatcggtat caaaaccgcc ctttcactcc 1893900
aaaaatacca aatcgacaaa ccgggcaaag aaatcagacc gtgccgcctg aaaaaacgcc 1893960
cacccgtccg ttaaattaac ctatccctgt ttcagacggc ctgaagcagg gatttttata 1894020
tcaaaataaa atgagaaagg gagcaataac ccttaggtag ctcttgttat tttccgatgc 1894080
aaaacaaagc agtcatatat ttaattcccc ctacctctgc caagccttcc tcaaatattc 1894140
gacgcaatcg gtcagcgagt agaacgggac attgccgtgg tcggcattcg gatattcccg 1894200
gaaaaagacg gcggccccgt gcctgtctaa ctctgccgcc atttgttcgg cctgccctgc 1894260
catatcgcgt tcttccctgc gtttacaatc gctaccccgt tctagcgcgc cgatgttgag 1894320
gcagacatcg atgccgttta gccggttttc agacggcata aagtcgagta tccgcctgtt 1894380
gtgccaccaa atcgatgggg atacgagcca atgccgtctg aaacggcggt gggaaagcag 1894440
ggaatacagt ccgaacagtg cgccgaacga gtgtccgaat acggcggttt cattgcggtt 1894500
gagggtgtag cggctttcta aaaaggcggt cagctcgctg tcgataaagg cggcgaagcg 1894560
gtctgcctgt ccgaactgct gccgttcgtc tgctgtggcg ttgtctccaa gcggcggcgt 1894620
gtagtcggcg gcacgttgtg ccaaatcgcg cacactgcct gtcgtgtagc cgataccgac 1894680
aatcaggcag gggcgttgc ttcgggtaac ggggttgttc atcagcgact gcatgatgtt 1894740
gaaaagtgcg gggaaaaagg cttcgccgtc gaggacaaag aggacgggat agccttcaga 1894800
cggtatttcg ccgagtgttg ccgtctgaat ccgatagatt cgccccgtgc aggtggattt 1894860
gatttcggtt tcaaaggctt ggggcagtat ggcaggttgg aatgtgtcgg tcggtatggg 1894920
tttcatgatg ttcggcttgt gggtcagact ggcggcaagc ggtaaaaccg aaccggcaaa 1894980
gcggtctgcc tgccggttcg gtctattttc cttcgcaatg ccatactcca gttgtgagag 1895040
catagggtat gccgcgcagc ttgttgtagt ttgatgatgc tgcggctgcc gtttcagacg 1895100
gcatattggt ctttaaaaac tgtaacgcag gtttgccgtc aggctgcgct ccgaaccggg 1895160
aatgttaaag gtgctctcgc tgccgacgcg ggcgtagtaa tggcggttga agatgttgtc 1895220
ggcgttgatt tgcagcttca gtttgggcgt gaagcggtat gccgccatcg catcgaacgt 1895280
ggcataaccg cctgcatgta tccctgcaga tgaagtaatg ccgctcatcg cgttcacgcc 1895340
gccgccgatg gtcagcccgg acgtaacttg gtaagtcgtc cacaggtttg cgctgtgttt 1895400
gggcatcagc aggaagatgc cttcgtcgcg cgaattggag gcggttttga tttggctgtg 1895460
caggtagctg taacctgcat ggatttgcca tttcggtgtc atcgcgccgc tgatttcggt 1895520
ctcaacacct tccatcacgc gtttgcccaa tgcggcgtaa cgggtttttt tgttgtttga 1895580
gtccagcggt gcggcggcgt ttttatcctt catgcggtag aacgaaaccc gggtattgag 1895640
gcggtcgtcc atgtagctgc ctttgtagcc gatttcaaac tggttgcctt cgcgcggttt 1895700
gagcagcttg ccgtcggtgc cgatgctggt ttgcggtgtg tagagttggg aggcggaagc 1895760
gtacaggctg ttgctgccgt ctatatcgta aaccgcgccg gcgtagcttg taaatttggt 1895820
tttcgaagct ttatgcaggg ttttgccgtc gcccgactcg attttgtgat gtcctacacg 1895880
tccgcctgca atcaacgaca aaccttccag aggacggaac accgtcttgg catacaaacc 1895940
ggtttcgtcg aggttttctt cggtaacgga gtgattgaaa cctttgtttc cggcgcgggc 1896000
gttctgaagt atgccgttat aaggcaaagc gcggaaacca tctaaagcga cgctttttga 1896060
caaagtcgaa cgcccttgtt cattagtact gcgcaagcgg ttgtagtctg caccaatcac 1896120
aaattcgttg gcggtgttgc ccaaggcaaa cggacggctg taacttgcgt caaccgcaaa 1896180

ggctttttgt ttaatgtccg tacccaaacc cgctacgtcg gcttgtccgg tattgttgag 1896240
tttgctgccc gcaaacgtat aattggaatc ggctttccga tcggaatagc gcataccgac 1896300
tttgccgtag ccgccgttgc cgaagtaatg tttcaaatcg gcgaacacgt cgtggctgtg 1896360
cattttaaat ttgttccaat ccgcgccgac aaatacgtgt tgcggcaggg acggtaattt 1896420
gttattggca tcggcaggca ggccgttgta cggcgcgagg cggcgttgct ggtaaagata 1896480
gcccgcgccc aaaaccgtat cggggttgat gtcccaatcc gccgccgcgt agaaggtttc 1896540
gcgccggttg tttttctcgg cgggacgcgg agacgcgccg acggtctgcg ccatcacgcg 1896600
gccgcgcacg ctgccgtctg aattgaggct gcccgatacg tccgcctcgg ctttatattg 1896660
tttgtgcgta ccgaaccctg ccgccgcatg accttggaac gctttggtcg ggcgtttgcg 1896720
caccagattc acgatgccgc ccatctcgcc gctgctgtcg aacagtccgc tcggcccgcg 1896780
catcacttcc acgcggtcga aggcgaacag gttgggcagc gtgccgttga tactctgcat 1896840
ctgcgcgggc aggccgtcga tgttgtattc gctgtattcg taaccgcgcg cgtaaaccga 1896900
agagcgtccg tcgtcgttgc tcaacacgcg caggccgggc gttttgcgtg ccaactggtc 1896960
aaacgtatca acattgcggt ctttgacctg ctggttggta atgatgctga cggattgcgg 1897020
aatttcgcgc aaagaagcgg ggattttttgt accgacggtg gcggcaaacg agctgtaatc 1897080
gccgtttttc tcggtggcaa tcgcgttgta agaacgctga cccttaatat ggacggtttc 1897140
caaaccttcc gtttgtgcgg caaaaaccga agacgagagt gctgccaaaa ccgtggcggc 1897200
ggtcatattg atgcggaaaa ctgacataaa ctgtcccatt cacataaatg ataatggttc 1897260
tattttaata aagcgcaacg cggcttgttc ggaaaaacat atcgcgcagc cgacaaattt 1897320
tgtcgaaaat gcgacacgtc tgcgttttcc gcataaaatt tgctttttta ctgcaaccaa 1897380
cctgctatga ccacgcccaa actcatcatc ttcgactggg acggcacgct tgccgatacg 1897440
acccaaccca tcatcgacac catgcgccgc agcttcgccg aatgcggttt tccgccgccc 1897500
gaagcggaac gcgtccgcag cctgattggc tacagcctgc ccgaaatcat ccgcaccctg 1897560
ctcgaaatgc cgtctgaaac cgccgttgcc gacatcacac gcacttattc cgcacattac 1897620
ctcaatccca acaaccgcaa tatgtcctta tttcccgatg ccctgccctg tctggacaag 1897680
ctcaaagcac aaggatactg gcttgccgtc gccacgggca aagggcgggc gggtttggac 1897740
aacgccatca gtcaaaccgc caccggcggc tattggctcg ccaccgcctg cgcgggggaa 1897800
tatccctcca aaccctcgcc cgaaatggta ttcggaatct gcggcgaact gggactcgac 1897860
ccgaaagagg cattggtcgt cggcgatacg gcgcacgacc tgcatatggc ggcaaacgca 1897920
ggcgcggcgg cagtcggcgt ggccaccggc gcacattcgc gcgaacagct ccttagcgca 1897980
ccgcatctcg ccgtattgga cggtttgtcc gaactgcccg gttttcttgc acaacattac 1898040
gcctgattgg tttccgcatc cggcacacgg caaaaatgcc gtctgaagcc tgttcagacg 1898100
gcatttgtgt tgcccaaaca ttcaacgcct gcgtcaacgt ttgcacaaat cgggtttggt 1898160
ttcgccctcg cggcgcaact ctttgggcag gacgaacaca atgctttctt ccgcaccctc 1898220
gccttcgcgt acggtttcgt gcccccatcc gcgtatggtt gccagtactt cccgcaccaa 1898280
cacttcgggc gcggacgcgc ctgccgttac gccgactttg tttttgccct caaaccatgc 1898340
gcgttgcagg tagcctgcat tatccaccat atacgcatcg attccgcgcg atgccgccac 1898400
ttcgcgcaag cggttgctgt tggacgaatt gggcgaaccg accacaatca cgatgtcgca 1898460
ctgttctgcc aactctttga cggcggtttg ccggttggtc gtcgcatagc agatatcttc 1898520
cttgtgcgga ttgcggatat tggggaaacg cgcgttcagc gcggcgatga tgtctttggt 1898580
ttcatcgacc gagagcgtgg tttggctgac ataggcgagt ttgtcggggt ttctgacttc 1898640
gagttttgcc acatctccga ccgtttcgac caaaagcatt ttgcccggcg caagctgccc 1898700
catcgttcct tcgacctcga cgtgcccctt atgcccgatc atgatgattt cacagtcttg 1898760
ggcatccagt cgggcgactt ccttatgcac tttcgtcacc agcgggcaag tcgcatcaaa 1898820
cacgcggaaa ccgcgctccg ccgcttcttg ccgcaccgcc ttcgatacgc cgtgtgccga 1898880
ataaaccagt gtcgcgcccg gcggcacttc cgccaagtct tcaataaaca ccgcaccttt 1898940
ttcacgcagg ttgtccacga cgaatttgtt gtgaacgact tcgtggcgca cataaatcgg 1899000
cgcgccgaac tcttccaaag cacgttcgac aatactgatt gcccgatcca caccagcgca 1899060
gaagccgcgc ggattggcaa ggatgatggt tttctcgttc ataagcccgg tatttcgttt 1899120
tcagacggca tcaatatttt tcttcttggg ttttacggtg gacgatgttg tccaacaccg 1899180
ccaacaccgc accgacgcag ataaagctgt cggcaatatt aaaggcggga taaaaccaat 1899240
tttgccaata aaacaataag aaatcgacga catgaccgtg tatcaggcgg tcgatgacat 1899300
tgcctaacgc accgccgata atcattgccg cacccgtttt gccgagggtt gcaaactcat 1899360
cgcgcaagat ggcgcgtacc aaatacgcgc tcaccgccac cgccagcacc aaaaaaaagt 1899420
atttttgcca gccgccctga tcggcaagga agctgaacgc cgcacccggg ttgtacacca 1899480
gcgtcagatc gaaaaaggaa ggaatgacat tgacgcgttc ccgatactga aacgacgaca 1899540
gcaccgccca cttcgaccac tggtccagca cgatggcggc aagtgccaat acccaatagc 1899600
gcgttttact tgaaacagat gaagacatat ttttcaacag ccggtaaaag agtaccattt 1899660
tacccgaaaa cccccttcc tgtacccgaa acggcaaatg ccgtaatctt aaaacccgtc 1899720
attcccgaca acaccgtaat ctcgaaaccc gtcattcccg cgtaggcggg aatccagacc 1899780
tgtccgcaca gaaacttatc ggataaaaac agttgcccaa accccgcgtt ctatagtgga 1899840

ttaaattcaa accagtacgg cattgcctcg ccttgccgta ctatttgtac tgtctgcggc 1899900
ttcgttgcct tgtcctgatt taaatttaat ccactataga ttcccacttc cgtgggaatg 1899960
acggttcagt tgcattccga caacaccgta atcttgaaat ccgtcattcc cgcgcaggcg 1900020
ggaatctatc ggaaatgact gaaacctcga gattctagat tcccactttc gtgggaatga 1900080
cggttcagtt gcgttccaac aacaccgcaa tctcgaaatc cgtcattccc gcgcaggcgg 1900140
gaatccagac ctccgacgcg gcgggaatct atcggaaatg actgaaacct cgagattcta 1900200
gattcccact ttcgtgggaa tgacggttca gttgcgttcc aacaacaccg caatctcgaa 1900260
atccgtcatt cccacacagg cgggaatcca gacccctgac gcggcgggaa tctatcggaa 1900320
atgactgaaa ccccgagatt ctagattccc actttcgtgg gaatgacggt tcagttgcgt 1900380
tccgacaaca ccgcaatctc gaaatccgtc attcccgcac aggcgggaat ccagacccct 1900440
gacgcggcgg gaatctatcg gaaatgactg aaaccccgag attctagatt cccactttcg 1900500
tgggaatggc ggttcagttg cattccgaca acaccgtaat cttgaaatcc gtcattcccg 1900560
ataacagcgc aatcttgaaa cccgtcattc ccgcgcaggc gggaatccag acctccgacg 1900620
cggcgggaat ctatcggaaa tgactgaaac cccgagattc tagattccca ctttcgtggg 1900680
aatgacggtt cagttgcgtt ccgacaacac cgtaatctcg aaatccgtca ttcccgcaca 1900740
ggcgggaatc tatcggaaat gactgaaacc tcgagattct agattcccac tttcgtggga 1900800
atgacggttc agttgcattc cgacaacacc gcaatcttga aacccctccg ccgttataaa 1900860
gacaaatcgc ggcacaaaaa atgccgtctg aaatgctgtt cggcggtttc agacggcatt 1900920
tgctcaaact ttatcaggcg taatggcgcg tttcgccttc tccgccgaca ttctctgcac 1900980
agcgtttgca gacggtttca tagcctgcaa ccgcgcccac atcgcgggtg tagtgccagc 1901040
agcgttcgca tttttcacca tcactggctt tagcggcaac ggcaagttcg ctgcctactt 1901100
tcacttctgc tttagacacc agcaaagcaa agcgcaattc ttcgcccaaa gcattcagat 1901160
agccggccat ttcttccggc gcggtaattt cggcttcggc ttgcaaggac gaaccgacgg 1901220
ttttgtcggc gcgcaaaggc tcgatggcgg cggttaccgc ttcgcgggct tcgcggattg 1901280
ccgtccattt tttcaccagt tcggcttcgg ttttttcgtt gatggtcggg aactcgtgcc 1901340
aagtatggaa gaggacgctg tcttcttcgc cgccgccgat gatgtcccac gcttcttcgc 1901400
cggtgaagca caaaatcggt gcaatcaaga gaaccaaact gcgtgtgatg tgatacaggg 1901460
cagtttgtgc gctgcggcgt gcatggctgt ctgctttggt ggtgtagagg cggtctttca 1901520
ggatgtcgag gtagaacgca cccaagtctt ccgagcagaa agaaacaatg tcttttacgg 1901580
caaagtggaa ggcataacgc ggatagtaat cgcctgccag acactcttgc agctgacgtg 1901640
ccaataccac ggcgtagcgg tcgatttcca ccatatccgc ctgttgcacg gcatcttcaa 1901700
tcggattaaa gtcgctcaag ttggcaaaca aaaagctcaa ggtattgcgg atacggcggt 1901760
agctttcggt tacgcgtttg aggatttctt tggaaatcgc caattcgccg ctgtaatcgg 1901820
tagatgccgc ccacaggcgc aggatgtctg cgccgaattc gttataaacc tcttgcggtg 1901880
caacgacgtt gccgatggat ttcgacattt ttttgccttc gccgtcgaca acgaaaccat 1901940
gggtcagcag ctgtttatac ggcgcgcgac ccattgatga ggcgcagccg gtcagcatgg 1902000
acgattgaaa ccagccgcgg tgttggtcgc tgccttcgag atacaaatca gccggccatt 1902060
ccaattcttc gcgttgtttc acaacggaat aatgggtcga gccggagtcg aaccatacgt 1902120
ccattgtgtc agaaagttta tcgtaatttt cgcaatcttc cgcgctcaag agttcgctct 1902180
tatcgaggga gaaccacgct tcgatgcctt tttcttcgat tttcagggca actttttcca 1902240
aaagttcggc agagttcgga tgcagctcgc ccgtttcttt gtgaacaaag aaagtcatcg 1902300
gcgtgcccca atagcgttgg cgtgaaacca cccagtcagg acgaccttca atcatggctt 1902360
ccaaacgcgc gcgaccccaa gacgggaaga attcggtgtc gtccacggcc ttgatggctt 1902420
tgtcgcgcag ggttttgccg tcggcaccgg ctttgtccat accgacaaac cattgacctg 1902480
tcgcgcggta aatcagcggc gttttgtgcc gccagcagtg ggcgtagctg tgttcgattt 1902540
tactgcttgc caaaaggttg ccggtttctt ccaaccattg caggatgacg gggttcgcct 1902600
cccaaacgcg cataccggcg acacgcggcg tttcgccgat gtatcggcct tcggcgttga 1902660
cggggttgta aagctcgatg ccgtatttat tgcagacggc gtagtcttcc aaaccgtgcg 1902720
cgggggcggt gtgtaccaag ccggtaccgg catcggtggt aacgtgttcg ccgttgagca 1902780
tgggaatatc gcgttcgagg aacggatggt tcatgtgcag attttccagc ttgtcgccgg 1902840
tggtttcggc gagaatagca atgccgtctg aaaaaccgta acgtttgagc gcgtcttctg 1902900
ccaaatcttt cgccaatacc aatttgcctt tcggcgtatc aatcagttga tacaccacgt 1902960
ctgcacccgc agacacggct tggctcgccg gtagcgtcca aggcgtagtc gtccaaatga 1903020
cggcaaacgc tttgccttcg aaaccagcca aaccgaatgc ggcggcaagc gcggcagtgt 1903080
ctttaaacag ataggcaacg tcaatcgcgg gcgagatttt gtctttgtat tccacttccg 1903140
cttcggccag cgaagaaccg cagtccaagc agaattgaac cggtttcgca ccccggtaga 1903200
gatagccgga tttgtagatt tcgccgagca tacgcacggt atcggcttcg gttttgaaat 1903260
ccatagtcag gtaaggatgg tcccagtcgc ccaacacgcc caagcggata aagtcttttt 1903320
tctgacgggc aatctgttcg gcggcgtatt cgcggcacaa ttcgcggaaa cgtgctttgg 1903380
gcatatcttt gccgtgcagt tttctaccca tcacttcgat gggcaggccg tggcagtccc 1903440
aacccggcac ataaggcgcg tcaaaaccgg cttgggtttt gctgcggata atgatgtctt 1903500

tgagaatttt attgacggca tgaccgatgt ggatgtcgcc gttggcatac ggcgggccgt 1903560
cgtgcagaat aaatttcgga cggcctttgg cgatttcgcg cagtttttgg tagcgttttt 1903620
gctcgtacca gcttttcagc catgcaggct cgcgcttggc aagattgccg cgcatcggaa 1903680
acgggctctc gagcaggttt acggttttac tgtaatcggt catttttttaa tctctattgt 1903740
tacaatattt cggtctcaga cggcattgcg ccccaaacag catttcacaa cgggaaaacc 1903800
ctgtgccgtc tgaacagtta aaagattgat tgtagcccaa tcggatggtt tgtataaggt 1903860
ttttctacca acgccttgcg gcttccatat cggcttcaat ctgccttttc agttcttcca 1903920
taccgtcaaa cttttcctca tcgcgcagtt tgtgcaggaa gcggacgttc agcccttgtc 1903980
cgtacaggtc gccttgaaag tcgaacaggt ggacttcaag cttttgagaa cagccgctat 1904040
caacggtggg attgaagccg aaactcgcca cgccgcgccg cgtgccgaat gcgccgtctg 1904100
cttcgacgac aaacacgccg ccgagtgcat aacggtggcg gggcaggcgg atgttggcag 1904160
tcggggcgtt taaggtgcgt ccgagttttc tgccgtgcac caccctgccg ctcaagacgt 1904220
agtcgtgtcc caaaagtttt ttcgcatagg caaggttgcc gtctgaaagg gcttgtcgca 1904280
cggcggtact gctggtgcgg atgtcttcga cgatgacgga aggcgtacgc tcggtctgca 1904340
tatcgggctg ttgtgccaaa agttcaaaac agccttcccg ccccgcaccg aaacggaaat 1904400
catcgccgac gagcaaataa cgcgtattca aggtttgacg cagcaggcgg tcgataaacc 1904460
cttgcgcgga tatttcggaa aaattttgat cgaaacgcaa aacccagacg gcatcgacac 1904520
agcctgtgcc ttccaataat tcgagcttgg tgcgcagggg gctgatccga cacggtggca 1904580
tcctgccggt gcggagtgcg aaaaattctt tgggttgggg ttcgaaaacg acggtcacga 1904640
cgggcagtcc gcgcgcgtcg gcttcgaggc ggagtttttg gaggatgtgt ttgtgtccga 1904700
ggtgtacgcc gtcgaaattg cctatggtta cggcggcacc ctgtggaaag tcgggcgcgt 1904760
tgtgccgccc cagcctgatt ctcattgttg cattcgggta tgtggtgaaa caggcggtca 1904820
ttgtaaacgg tattgcggtt tatagacagt gtgccgcccg ttacgcccgc ccgacgcggg 1904880
aaaagtaggc aaatttcccg ccgccgaacg cgccaaacgc acaaaaacgc gcagcaggcg 1904940
cggtgctatg tgttgaaaca tcgccccaaa cctgatgcaa catcagtgaa aatcgttctt 1905000
ttttaaccag gtaaaccgag acaaacaacc ctccgccgtc attcccgcgc aggcgggaat 1905060
ccggacctgt ccgcacagaa acttatcgga taaaaacagt tgctcaaacc ccgagattct 1905120
agattcccac tttcgtggga atgacggttc agttgcgttc cgacaacacc gtaatcttga 1905180
aactcgtcat tcccgctcag gcgggaatct agaacgtgga atctaagaaa ccgttttgcc 1905240
cgataagttt ccgtgcggac aggtccggat tcccgcctgc gcgggaatga cggcatttct 1905300
gcggcaatcg gattatttcc aaaccaaaag cgcgtggttg cgtttgccgc gccgaaggat 1905360
agtgtatttg ccgaaacgtt tgtgttcgcc gttcagcagg caggcatcgt cggggcgttc 1905420
ggcggcgtgg ttggggttgt tggcttcggc aggtttgccg ttgagcaaaa ccgctttgct 1905480
gttcacaaag ccgcgcgctt ctttattgga ggatgccaaa ccggttttta ccaaggcttc 1905540
gacgacattg atgccgtctg aaacttcaaa tgcaggcagg ccgtcgaggg cgagctgctc 1905600
gaagtcgctt tcggtcaggc tgctttggtc ttcggcaaac aggctttcgg aaatgcgttg 1905660
cgcggcggca agggcttctt cgccgtgaat caggcgggtc atttcttcgg cgaggatgcg 1905720
ttgcgcttcg ggcttgctgc cgcttgcctt gtctttggct tcgatggcat cgatttcttc 1905780
gatggacagg aaggtaaagt atttcaggaa tttatacaca tcggcatcgg cgactttcag 1905840
ccagaattgg tagaactgat agggcgaggt ttttttcgcg ttcagccata ccgcgccgcc 1905900
ttcggttttg ccgaatttgg taccgtctga tttggttacc aaaggcaggg tcagaccgaa 1905960
tacttgtttt tggtgcaggc ggcgggtcag gtcgataccg gcggtgatat tgccccattg 1906020
gtcggagccg ccgatttcca aaaccgcgcc gtggcgtttg ttcaactcgg cgaagtcgta 1906080
accttgcagc agggaatagg cgaactcggt gaaggaaatg cctgcgccgt cgcggtcgat 1906140
gcgctgtttg acggattctt tgttcagcat ggcgttgacg gagaaatgct tgccgatgtc 1906200
gcgcaggaag tcaaggcagt tcatgctgcc gaaccagtcg gcattgttcg ccataatggc 1906260
ggcatttccg ccttcaaagc tcaagaaagg ggttaattgg ttgcggatac tttccaccca 1906320
gccggcaaca gtttcggcgg aattcaagct gcgttcggcg gctttgaagc tggggtcgcc 1906380
gatcataccg gtcgcgccgc ccaccaaagc aatcggcgta tgccccgcct gttggaagcg 1906440
gcgcaatgcc aatacgggca gcaggtgtcc gatgtgcagg ctgtcggcgg tcgggtcgaa 1906500
gccgcaataa agggcaattt tttgttcgtt caacaaagcg tctaaggctt cgatgtcggt 1906560
ggtttgcgcg ataaggccgc gcgattgcag gtcttggatg acgctcatcg gtctctttca 1906620
aaaaaaatta gcgtttttgc aaaccgccga ttgtaacaaa tttaagcgaa tcaatggtta 1906680
tggcgcgtat cgagaaaccg ttgtttttcg gaaaaacgct ttgccaattc cgtgccgccg 1906740
taagggttga tgtggtcttt gtccgagtaa accggcaatc cgccgatttg aaaatctgcg 1906800
gggatatagg cggcggcatc aataatatag acgttggggt atttggctgc caattccctg 1906860
atgcgtgcat tggctttcag ggtgctttcg tcgtccgggc gcagggcttg gcggtaaccc 1906920
ggtatgcgtg aagacaagat ataggcgcgc tggacgttgt aagacgaggc aaggttgtcc 1906980
gccatcaggt aaacggcttg tttttcggac gagagtttat gcagcatacg gtcgaatttt 1907040
tggaaaaaac cggcatcata ggcaagggag cggctgtttt cgggcatttg gctgccccag 1907100
cgcatcgcca aaaccacttt tgaataccgg ggcaggtgtt cttcggcata gcgataaacg 1907160

gcgcggcagg ctgcccagtt ttggaacaca cgggacgcgt agccttccac ataggcgcaa 1907220
gcgtcggcgg aaaccatagt ggcggaccat ttttcttttt tgcccacggc atcgaagaat 1907280
gttttgtaat ggtcggcgtg ggagtcgccc aaaaccagca gttccggctg tttttccgta 1907340
tccccccata ggcattgttt gccggtattg ttgtggcagg aggtgttgga acgcgtcagc 1907400
cccaagcggt cgtattgcgc cataaacggc agtctcatcg caaaaaacga gcccgccccc 1907460
aaaatgagca taggcaaggc ataaatccat aaaacggatt gtgcgaacga accttgccat 1907520
tttttaaacg gtttttcgat gcagtggtaa gaaaacaggg aaagcagcaa tatcaggacg 1907580
accgccgccg ccggcgaata aggcggcagg ttgtccgggc cgatatagcg cataaaggcc 1907640
aatatcggcc aatgccacag ataaagcgaa taggaaatca aaccggcggc aacagtgatt 1907700
ttcgattgga aaaatttttt aagcgggtgt tcgtaatgat tgaaataaat cagcgcggca 1907760
acagccagac agggaatcaa agcggcgggg cccgggaaat aggcggtttg ttccgaatag 1907820
gaaaacaggc aggttgacaa tatgcacacg gcaaacaatg cgccgacggc ggcacagcgt 1907880
ctgccgacgg caggttgccg gcagcgcatc cacacggcgg tcagcgatcc tatcagtaat 1907940
tcgcaggcgc gcaggtgggg caggtaatat ttatcgagcg cggaaggtat aaaggaggcg 1908000
gcaaggctta aggcacacag tgcggcaagg aagccgaact gtacgcgcag gcttttgcgg 1908060
gcgacaagca gcagcagtat cggaaagaca aagtaaaatt gttcttcgac cgacaaagac 1908120
cagatgtgca gcaggggctt ttcttcctgc gcgggatcga aataatcctt ccccccttgca 1908180
aaatacaggt tagaggcgaa acccaaggcg gtcagcgcgg atttccacaa aagaaagaaa 1908240
tcatctttgg tgaataaaaa gaagccgcct gccagcgttg ccgccaatac ggcgaaaaat 1908300
gcgggcagaa tccgcttgat gcggcggata taaaatgcct tcagggaaaa cctccccccc 1908360
ccccccgaca tttcgcggtg aagaatcgtc gtcatcaaaa agcctgaaat cacaaagaat 1908420
atatcgacac cgagaaaccc gcccggcagc caatcctttt cgatatggaa cacgatgacg 1908480
gacaagacgg cggcggcgcg caatgtgtcg atgtccgggc ggtagggtaa ggcttggctc 1908540
ataatgtttt tatagtggat taacaaaaac cagtacggcg ttgcctcgcc ttgccgtact 1908600
atctatactg tctgcggttt cgtcgccttg tcctgattta aagttaatcc actatactcg 1908660
aaacgcggcg gcgcaaatgc cgtctgaaag gtcatttcgt atcggggatc gggatattcg 1908720
gaatgccgga cggcttcccg taacggcggg gcaggcggtt tgttttgcag gaatcgggga 1908780
gggcaaatcg gaaatgcggg tgggagttta ttttgatgcg gctgcattcc ggcggtacgg 1908840
gaaacgccga aaatcatcaa aatcggcttc agacggcatt tccggcaagc cgcctgaaac 1908900
ctgccgcatt tgggttacac gttaaacaaa aagtgcatca catcgccgtc ttgcacgaca 1908960
tattccttgc cttccacacg cattttgccg gcttctttgg ctttggcttc gccgccgagc 1909020
gagacaaagt cgtcgtaaga aatgacttgg gcgcggatga agccgcgttc aaaatccgta 1909080
tgaatcacgc cggcggcttg cggcgcggtg tcgcctttgt gtatcgtcca cgcgcggact 1909140
tctttcacac cggcggtgaa ataggtttgc agccccaaga ggtcgtaacc ggcacgaatc 1909200
aggcggttca ggcccggttc ttccaagccc atttcggcga ggaactcggc tttttcgtcg 1909260
tcttccaatt cggcaatttc gctctccatc gcggcgcaaa cggcgacgac gggggcgttt 1909320
tcttttgccg ccaattcttt caggcggtcg aggtgcggat tgttttcaaa accgtcttcg 1909380
gcgacgttgc ccacatacat cgccggtttg gcggtcagca ggaacagcgg tttgagcatc 1909440
gcgcgttctt ccgcgtccaa accgaaggaa cgcacgggtt tgccttcgtc cagatgcggc 1909500
agcagttttt tgcacaaatc gaccagcttt tgcgcgtctt tgtcgcctga gcgggcgcgt 1909560
ttttcttcgc ggacgatggc tttttcgaca cttgccaggt cggcaagtgc caactctgtg 1909620
ccgatggttt caatgtcggc aatcggatcg acgcggcctg caacgtggac gatgttgtcg 1909680
tcgtcaaagc agcgcacgac attcacaatc gcatcggttt cgcggatgtt ggcaaggaac 1909740
tggttgccca agccctcgcc tttgctcgcg cctgcaacca aaccggcaat atcgacaaat 1909800
tcgacgatgg caggctgcat tttttgcgga ttgacgattt ttgccaattc ggccatacgc 1909860
ggatcgggga cttcgacgat gccgacgttg ggttcgatgg tacagaaagg atagtttgcc 1909920
gcttcgatac ccgattgggt cagcgcgtta aaaagggtgg atttgccgac gttgggcaaa 1909980
ccgacgatgc cgcatttcaa actcatgttt tttcctgaaa atagagaaat ttaacggcgg 1910040
attatagcat accgccgccc gcgttccgaa aaaatgccgt ctgaaacggc ttcagacggc 1910100
atccggtttc agaaaaccgt tcagaacaag ccgtgaatca cgccttctgc gtccacatcg 1910160
attttctcgg cagccggaac tttgggcagg ccgggcattt tcatcatgtt gccgcacagg 1910220
gcgacgatga aacctgcgcc tgcggaaacg gtgatgccgc gcacggcgat gcggaagtct 1910280
tcggggcagc ccaacagttt ggcgttgtcg ctcaaagagt attgggtttt cgccatgcag 1910340
atcggcattt tgtccaagcc cagtttttcc agtgaagcga tttcggcaga cgcttccgcg 1910400
ctgaaatcaa catcttccgc gccgtacact ttttgggcaa tcgcacggat tttgtctttg 1910460
atgcccaact cgacatcgta ggcgaaaccg aagttattgg tttgactttc aatggcgttg 1910520
acgactttgc gcgccaaatc cgcgccgccc gcaccacctt tgccccacac ttcggtcagg 1910580
gaaacttcaa cgccgtgttc ggcacaggct ttttcaatca tcgccaactc ggcatcggcg 1910640
tcggacacga agcggttgag cgcaacgacg acgggcagtc cgaatacgtt tttcaggttg 1910700
gaaatgtgtt tcagcaggtt gggcaaacct ttttccaaag cgtctaaatt ttcttcgccg 1910760
aggttggcgc gttccacgcc gccgttatat ttcaacgcgc ggacagtcgc cacgacaaca 1910820

```
gccgcatcag gtttcaaacc ggcaaggcgg catttgatgt cgcagaattt ttccgcgccc 1910880
aagtccgcgc cgaagcctgc ttcggttacg gcgtaatcgg caaggtgttt cgccagacgg 1910940
gttgcggtta cggagttgca gccgtgggcg atgttggcga acgggccgcc gtgtacgaag 1911000
gcgggcgtgc cttcgatggt ttgcaccaag ttgggcttaa tcgcatcttt aagcaatgcc 1911060
gccatcgcgc cattcgcttt caaatctttg gcgtaaacgg ggctgccgtc tttggcgtag 1911120
gcgacaagga tgttgcccaa acgctctttc aaatcgctga tgtctttggc aagacagaat 1911180
accgccatca cttcggaagc aacggtaata tcgaaaccgt caggacgcat cacgccgtca 1911240
acgggtttac ccatgccgtc gatgatgttg cgcaactggc ggtcgttcat atcgaccacg 1911300
cgccgccaca gcacgcgttt ggggtcgatg ttcaactcgt tgccttggta gatatggttg 1911360
tcgagcatcg cggcaagcag attatttgcc gcaccgatgg cgtgaaaatc tccggtgaag 1911420
tgcaggttga tgtcttccat cggcaaaact tgggcatagc cgccgcctgc cgcgccgcct 1911480
ttcacgccga acaccggccc cagagaaggt tcgcgcaggg caatcacggc atctttgccg 1911540
atgtggcgca acgcgtccgc caaaccgatg gttacggtgg ttttgccttc gcccgccgga 1911600
gtcgggttga tggcggtaac caaaatcagc ctgccctgtt tttgcggcag tttgaacgct 1911660
tcggcaggat tgattttcgc cttgtaatga ccgtaaggct caatgttgtc ggcattcaga 1911720
ccaagcttgg cggcaatttc gccaatcggg cgcatggtgg aggattgggc gatttcggca 1911780
tcggttttga agctcatgat tttcctttag aaatgaggag ggacatgccg tctgaaagca 1911840
tcaggcgaca aacaggtgga ttgaaaataa tatcaggcat attataacgt tatccgcacc 1911900
aaacccgcag tgaaattttt gacgcagcaa caaaaatacc gttcatattg ttcacaatcc 1911960
aaggagaaaa catgggcagc aacgcatggc tgttttgggc attggcatcg gcaggcttcg 1912020
cctcattgac cgctattttc gccaaaatgg gtttacaggg tatagattcc gatttcgcca 1912080
cctttatccg caccttggtc atccttgccg ctttgttatt gtttttaacc tacaccggca 1912140
aatggcaggg tgtgaacggc tttacggggc gcaactggac attcctcatc ctatccggtc 1912200
ttgctaccgg cgcatcttgg ctcgcctatt ttaaagccct gcaactgggc aacgcctcgc 1912260
aagtcgcccc catcgacaaa ttcagcctgg tcttggtcgc gctgatggcg gtggttttct 1912320
tggacgaacg cccgaacacg caggaatgga taggcttggg gctggtaacg gcgggcgtgt 1912380
tggtgctggc gttgaaacgt taaaccgaat ccgccatacc gtctgaaacc gggtttttac 1912440
ttccaagccc ctgcaagggc ttgagcctct ttcagacggc ataccgtgcc gacatccagc 1912500
cacaagcccg tatgcttctg accgctcacg cggttttgcc gcatttcgcc acgcaatacg 1912560
ggcgcgagtt tcgccacact gcccgcttcg attccgtcaa acatttcagg acggtaaata 1912620
cccacgccgc tgaatgtcaa tccgttgccg ccatttactt ccggccgcac gctgctgtcg 1912680
ggcagcaggg aaaaatcgcc gtcggggttg tgcggcggat tttccaccag ccacagatgg 1912740
gcggaaatat gttccggcag ggacgatgcc gtctgaaacg cggcggtaaa atcgatgtcg 1912800
gtcagcacgt cgccgttgac caccaaaaac ggctgcccac ccaacagcgg caatgcctgc 1912860
gcgatgccgc ctgccgtttc caaaccgcct gcgggttcgg gcgaataggc gatgttcacg 1912920
ccataagccg agccgtcgcc caaagcatct tctatctgcc gacccagcca agcgtggttg 1912980
atgacgattt cggtaaaccc cgcctgcttc agacggcata ggtgccaacc gattagaggc 1913040
ttacccgcca catcgagcag cggcttcgga gtggtatcgg tcaaagggcg catacgctcg 1913100
ccgcgtcctg ccgccagtat catcgctttc atatatctgt ccgaatatca gtctaaaaat 1913160
ctaaactgcc gtctgaaata cagcagcgcg gggcgtttgc acccgcagtt tttgatttcg 1913220
tcgagcctga cgtaaaacac aaaatgcgtg ccgatttcat gtttgccgac aatatgcccg 1913280
tgcaggtgcg ccaacgcgcc ctctatttca agttgtcccg ttttgccgcg atgccagatg 1913340
tggtaggcaa accgctcttc gggcgacagg ccggtcagcc cggcaaaatg ttcggcaaca 1913400
tcctgatgtt cgtccgccag cgtattgatg cagaggctgc cgttttccga caggatcgga 1913460
atgattcgcg cactccggtt gatgcacagc atcacggtcg gcggctcgtc ggtaaccggc 1913520
gcgaccgccg tcattgtaat gccgtaacgc cctgccgcac cgtctgtcgt gatgacatga 1913580
acgcctgccg cgcaagatgc catcgcatca cggaacgaag tttgaaaatt tttctgcaaa 1913640
tccgccattt ttcccccttta aactgtcccc tatataagaa tgctgcacac aaggcatccc 1913700
ccatgtgcag cagttttgat tcaaaaagcc gtcggtcgga cgtttccgcg cgttacggcg 1913760
tattacgagt tcaacgcatc ctcgattttg gcaagttctg ccaacaggtc tttaagcagc 1913820
agcattttct cgcggcccag cacttcctcg atagcgtcgt agcgttcgtc cacttcttcg 1913880
ccgatttcct catacagctt ctcgccctcg gcagtcagct tcagaaaaac acgtcgttgg 1913940
tcgttggaag gtttcaggcg gacaaccaaa cccgcttttt caaggcgggt caggataccg 1914000
gtcaggctgg ggcgcaaaat gcacgcctga ttcgccaaat cttgaaagtc cagcgtgccg 1914060
ttttccgcca aaagacggat aatccgccat tgctgatcgg taatattcgc ctgattcaga 1914120
ataggcctga attgggtcat cagggcttcc cttgcctgta tcagaccgat attgatagac 1914180
gcatgttttg attgggtagg cattgtttaa gtctccaagt tatcgaaaat caaactttca 1914240
aaccgtcggg aaagcctgtg ggcgtaaatt ttgatgcaac cgttatataa caaaacgaac 1914300
atatagcaac aatacgctat aaaccgcatc ggacgactgg gtataaaaga ctttaattcc 1914360
gataatccta tctaaaaata ttttaatagt tatatcttaa tctatttttc ccacaatcac 1914420
aacaagggat tacatcggca ggcgcgtcgg ctctttccca aaaaacaaaa gccgccgcat 1914480
```

ccgccgcgca aggcatatgc cgcttgattc tctacatagc ggaaaattta ataaaaacaa 1914540
aagttaaccg aaaacatccg cctgaaaaat tcgtgcgcgc aagccccaat aactgctgat 1914600
tcccgtcgta tagtgaacca ttttcccatt tttgaccaaa acgacggcag gcgttgcgac 1914660
aatccgccaa gaccttgcca aacccccgtc ctcatcgttg acagtcggaa agcccaagcc 1914720
gcgttttgcc atatacgccg ccacttccgc cgaactgccg gaacgtaccg ccacgccgac 1914780
gaccggcacg ccgtccgccg ccaaatcatc gattatcggc gactgataac ggcacacgcc 1914840
gcaccagctc ccccaaaaat acaccaaaac cgccttatct cggctaaact gtcccaaagt 1914900
cagccgctgc cccgacagca gggtcaaagg ccgccctgcc gcaccggccg gctcttcggg 1914960
cttgcgtatc caatccaaaa acagcgacac caataaaaac accaatgccg tctgaacggc 1915020
aaatttgatg cccgaaagca gtttcttttt catacgctct ctcaaacggt acgcccgcgc 1915080
aaccggcagg caaacaaaaa gccaagtctc aaaacttggc ttccggttat ctggtgggtc 1915140
gtgagcgatt cgaacgctcg accaacggat taaaagtccg ctgctctacc gactgagcta 1915200
acgacccgat aagccgtgca ttatacagca ccatcctacc tcgtcaagca aattttacag 1915260
gcttaattgc agaccactgt ttgcacggga tattttgaca acggattttc acaatccgcc 1915320
gcataccgtg taaaagttcg cacaaggaaa agcaaaccgc ccgaaatcaa tgtacacttt 1915380
ccgcccgttt cccttcccaa cctgcacaca gaaacacaca ttatgaacat acaaaacatc 1915440
cgcaccctcc tcgacaccgt cgccgttccg aatacggcac gcacgctcgg cggcgaaaag 1915500
gccgtccgtt cggtcgaaca gcgttcagac ggcatccata tcgccctgca tttcggcttc 1915560
cccgtcgcgc acattgcctc agaaacagcc gaccgcatac aggaaatcct gatgcccgaa 1915620
acaggcgaca cacacatcca tctgtccatg gacactgaaa tcggcacaca caaagtccag 1915680
cccggcgtta ccaccatcaa aggcgtgaaa aacatcatcg ccgtcgcatc gggaaaaggc 1915740
ggcgtgggca aatcgacaac caccgccaac cttgccgccg caatggcgcg catgggcgcg 1915800
cgcgtcggcg tgctcgatgc cgacctttac ggcccgagcc aaccgaccat gttgggtgtg 1915860
gacgaccgca aacccgatca gaaaaaccaa aaactcattc ccgtcgaatc ttcagacggc 1915920
atacaggtca tgtctatcgg ctttctcgtc gataccgacc aagccgtcgt ctggcgcggg 1915980
ccgatggtca gccaagcctt gcagcagctg atgttccaaa gcgagtggga cgaagtggac 1916040
tacctgttta tcgacctgcc ccccggcacg ggcgacatcc agctcacgct gtcccagcgc 1916100
atccccgtaa ccggttccgt catcgtaacc acgccgcagg acatcgccct gatagacgcg 1916160
cgcaaagccg tggatatgtt ccgcaaagtc aacattccca ttttgggcgt attggaaaat 1916220
atgtccgtcc acatctgcac caactgcgga cacagcgaag cactgttcgg cacggacggc 1916280
ggcaaagatt tcgccgcacg cctcaacgtc cccctgctcg gacagcttcc cctaagcctg 1916340
cccgtgcgcg aagccatgga cggcggcaca ccggcgcaac tgttcgacga acaccccgcc 1916400
atcgcccgaa tctacaccga tgccgcattc caaatcgccc tgagcattgc cgacaaaggc 1916460
aaagacttca gcagccgctt ccccaaaatc gtcgtcgaat aaagccgcgt ccgaaaccgc 1916520
aacagcaatg ccgtcccaag ccccgcgcct gccggcgggc aaacttgccg gataaaacgg 1916580
tttttttgag attttacgtt ccggattccc gcctgcgcgg gaatgacgaa ttttaggttt 1916640
ctgattttgg ttttctgttt tgtaggaatg atgaaatttt gagttttagg aatttattgg 1916700
aaaaaacaga aaccgctccg ccgtcattcc cgcgcaggcg ggaatctaga ccttagaaca 1916760
acagcaatat tcaaaggtta gctgaagctt tagagattct agattcccac tttcgtggga 1916820
atgacgggat gtaggttcgt gggaatgacg cggtgcaggt ttccgtgcgg atggattcgt 1916880
cattcccgcg taggcgggaa tctagaccat tggacagcgg caatattcaa agattatctg 1916940
aaagtccgag attctagatt cccactttcg tgggaatgac gggatgtagg ttcgtgggaa 1917000
tgacgcggtg caggtttccg tgcggatgga ttcgtcattc ccgcgcaggc gggaatctag 1917060
accttagaac aacagcaata ttcaaaggtt agctgaagct ttagagattc tggattccca 1917120
ctttcgtggg aatgacggga tttgagattg cggcatttat cggaaaaaac agcaaccgct 1917180
ccgccgtcat tcccgcgcag gcgggaatcc agaccttggg ataacagtaa tattcaaaga 1917240
ttataaaaga cccgtcattc ccgcgcaggc gggaatccag accttagaac aacagtaata 1917300
ttcaaagatt ataaaagact cgtcattccc gcgcaggcgg gaatccagac tgtcgggcat 1917360
ctgcagcggt ttgctaaaaa acgctttacc gtgatcagtg tgcaaagtta aaatggggag 1917420
gtaagctttt caatcagcaa tccggcgggc gcgggatcgg gcggtttacc gaaccccggt 1917480
gttcgcggcg cgcctgccgc cgacggtatc ccgcgaagca agatttaagg gataaaatat 1917540
gttccaacac gcagggcggc acataaggcg ccgccctgat tcggaagggc ttgcacccct 1917600
cccggacaaa gcctgatcct gccgccccga aggacggatg cccgaagggc gggggtttg 1917660
accgaaaagg aaatacgatg aataaaactt taaaaaggcg ggttttccgc cataccgcgc 1917720
tttatgccgc catcttgatg ttttcccata ccggcggggg ggggggggc gatggcgcaa 1917780
acccataaat acgctattat catgaacgag caaaaccagc ccaaggtaaa ggggaatggg 1917840
caatattcaa caataaagga caaagacagg gaacgcaaat ttatctataa taaaagcggc 1917900
cggggtggag gctctgtctt tttcgacaat accgataccc ttgtttcccg acaaagcggt 1917960
actgccgttt ttggcacagc cacctacctg ccgccctacg gcaaggtttc cggttttgat 1918020
gccgacgggc tgaaagagcg cggcaatgcc gttaattgga ttcatacgac ccacccaggg 1918080
ttgataggct acagctacac cagtgtcgta tgcagagaca gcacaggctg tcccaaactt 1918140

gtctataaaa cccgattttc cttcgacaac accggtttgg caaaaaatgc gggcagcctg 1918200
gataggcacc cggacccaag ccgcgaaaat tcgcccattt acaaattgaa ggatcatcca 1918260
tggttgggcg tgtctttcaa tttgggcagc gagaataccg tcaaaaatgg caactcattc 1918320
aacaaattga tatcttcttt tagtgaagac aataataatc aaaccatcgt ctctacgaca 1918380
gaaggctccc ctatttccct tggcgaccag cagcgcgaac ataccgccgt ggtctattat 1918440
ctgaacgcca aactgcacct gctggacaaa aaagggatta aagatatcac cggcaaaaca 1918500
gtgcggttgg gtgtcttgaa gccgagcatc gatgtgaaga cacaaaatac ggggcttggc 1918560
ggcattctag cttattgggc taggtgggac attaaagata ccgggcagat tccagtcaag 1918620
ctcggcctgc agcaagtcaa agcaggccgc tgcatcaata aaccgaaccc caatcccaac 1918680
aaaaaagacc tttcgccggc cctgactgcc cccgcgctgt ggttcggacc tgtgaaagat 1918740
ggtaaggcgg agatgtattc cgcttcggtt tctacctacc ccgacagttc gagcagccaa 1918800
attttcctgc aaaacctttc ccgcaaggat gacacaagca aaccgggccg ctattccctc 1918860
aaacccttga gtacgtcgga gattaaaagt aaagagccga gtttcacggg gcggcaaacc 1918920
gtcatccgat tggatggcgg cgtacggcat atccaactgg atagaaacaa tgaggccacc 1918980
ggtttaaatg gaaatgacgg caaaaacgac actttcggca ttattagaga agggagcttc 1919040
atgcctgatg ccagcgagtg gaaaaaagta ttgctgcctt ggacggttcg gggttttgct 1919100
gatgacagta aatttaaagc attcaacaaa gaagaaaaca acgacaacaa gccaaaatac 1919160
agccaaagat accgcatccg cgaaaacggc aagcgcgatt tgggcgacat cgtcaacagc 1919220
ccgattgtcg cggtcggcga gtatttggct acttccgcca acgacgggat ggtgcatatc 1919280
ttcaaaaaag gcaacgggga cgcgcgcgac tatagtctga agctcagtta tatcccgggc 1919340
acgatgccgc gcaaggatat tcaaaacacc gaatccaccc ttgccaaaga gctgcgcacc 1919400
tttgccgaaa aaggctatgt gggcgaccgc tatggcgtgg acggcggctt tgtcttgcgc 1919460
cgcattacag atgaccaaga caagcaaaaa cacttcttta tgttcggcgc aatgggcttt 1919520
ggcggcagag gcgcatacgc cttggattta agcaaaatcg acaacagcaa cccggccggc 1919580
gtttccatgt ttgatgtcaa aaacgacaat ggcgtgaaat taggctacac cgtcggtacg 1919640
ccgcaaatcg gcaaaaccca caacggcaaa tacgccgcct tcctcgcctc cggttatgcg 1919700
actaaagaca ttaacaacgg cgagaataaa accgcgctgt atgtgtatga tttggaaaac 1919760
aacaacggta cgccgattgc aacaatcaac gtacccgacg gcaagggcgg gctttcgtcc 1919820
cccacgttgg tggataaaga tttggacggc acggtcgata tcgcctatgc cggcgaccgc 1919880
ggcgggaata tgtaccgctt tgatttgagc aacaacgatc cgaccaaatg gtctgtacgt 1919940
actattttta aaggcacgct ggataagccg attacctccg cgcccgccgt ttccaaactg 1920000
aaagacaaac gcgtggttat cttcggtacg ggcagtgatt tgagtgagga tgatgttgat 1920060
aaaaaggata tacaatctat ttacggtatt tttgacaatg acacaggcac ggatgtggca 1920120
gaagaaggac agggcaaagg gttgctcgag caacacctta ctcaggaaga taaaacctta 1920180
ttcctgaccg attacaagcg atccgacggc tcgggcgaca agggctgggt agtgaaattg 1920240
gaagccggac agcgcgttac cgtcaaaccg accgtggtat tgcgtaccgc ctttgtaacc 1920300
atccgcaaat ataacgacgg cggctgcggc gcggaaaccg ccattttggg catcaatact 1920360
gccgacggcg gcaagctgac caagaaaagc gcgcgcccga ttgtgccgga agccaatacg 1920420
gctgtcgcgc aatattccgg tcataagcaa accgccaaag gcaaatccat ccctataggt 1920480
tgtatgtgga aaaacaatga aaccgtctgc ccgaacggat atgtttacga caaaccggtt 1920540
aatgtgcgtt atctggatga aaagaaaaca gacggatttt caacaacggc agacggcgat 1920600
gcgggcggca gcggaacatt caaagagggt aaaaaacccg cccgcaataa ccggtgcttc 1920660
tccggaaaag gtgtgcgcac cctgctgatg aacgatttgg acagcttgga tattaccggc 1920720
ccgatgtgcg gtatgaaacg aatcagctgg cgtgaagtct tcttctgatt tgcacgcgaa 1920780
aatgccgtcc gaaaggtttt cggacggcat tttttgcgtt tttcgggagg ggcgggttcg 1920840
taaaaggcgg gctatagggt aggcttcatc tcgccaatct cactgaatcc atcaatttcc 1920900
acaattcaat taaataccgt caaaccgatg ccgtcattcc cgcgcaggcg ggaatctaga 1920960
cattcaatgc taaggcaatt tatcgggaat gactgaaact caagaaactg gattcccact 1921020
ttcgtgggaa tgacgggatg caggttcgtg ggaatgacgt ggtgcaggtt cgtaggaatg 1921080
acgtggtgca ggtttccgtg cggatggatt cgtcattccc gcgcaggcgg gaatccagac 1921140
attcaatgct aaggcaattt atcgggaatg actgaaactc aaaaaactgg attcccactt 1921200
tcgtgggaat gacgggatta gagtttcaaa atttattcta aatagctgaa actcaacgca 1921260
ctggattccc gcctgcgcgg gaatgacgaa gtggaagtta cccgaaactt aaaacaagtg 1921320
aaaccgaacg aaccggattc ccactttcgt gggaatgatg ggattagagt ttcaaaattt 1921380
attctaaata gctgaaaccc aacgcactgg attcccgcct gcgcgggaat gacgaatttt 1921440
aggtttctga ttttggtttt ctgtttttgt aggaatgatg aaattttgag ttttaggaat 1921500
ttatcggaaa aaacagaaac cgctccgccg tcattcccgc gcaggcggga atctaggacg 1921560
taaaatctca agaaaccgtt gtacccgata agtttctgcg ccgacaaacc tagattcccg 1921620
cctgcgcggg aatgacggtt cagttgcgta ggactggatt gtgaaaaggg gcggattcgg 1921680
tgaaaacggc ggaaatgtgg gattgatgga atcggtgggc tgaagccctc cctacagagc 1921740
tttcagacgg tattgtttgc gttttcggga tgggggcaaa tgaaacaccg acaaaccgat 1921800

accgtcattc ccgcgcaggc gggaatctag acattcaatg ctaaggcaat ttatcggaaa 1921860
tgactgaaac tcaaaaaact ggattcccac tttcgtggga atgacgattc ggacattcct 1921920
taaactaccc gtgtatcgct gtaaatctta gagatggagg aataaagacc gttgggcatc 1921980
tgcagccgtc attcccgcgc aggcgggaat ctaggatgcg gaatctcaag aaaccgttat 1922040
acccgataag tttctgcacc gacaggtctg gattcccgcc tgcgcgggaa tgacgattcg 1922100
ggtatttctg acggttcggg cattcccgac aaggtggatt ttcaaggtgt tgtatagggt 1922160
gtaggaggat tcgtaaaagg tgagttatag ggtgggcttc agcccaccga ttccaacgat 1922220
tccaccaatc ctacaccgtt cccatagact caaatcaaca cagaaactta tgcgccgtca 1922280
ttcccgcgca ggcgggaatc taggatgcgg aatctcaaga aaccgttata cccgataagt 1922340
ttctgcaccg acaggtctgg attcccgcct gcgcgggaat gatggttcgg gtattcctga 1922400
cgattcgggt attcctgacg attcgggtat tcctgacgat tcgggtattc ctgacgattc 1922460
aggtattcct gacgattcag gtattcctga cgattcaggt attcctgacg attcaggtat 1922520
tcctgacgat tcaggtattc ctgacgattc aggtattcct gacgattcag gtattcctga 1922580
cgattcaggt attcctgacg attcaggtat tcctgacgat tcaggtattc ctgacgattc 1922640
aggtattcct gacgattcgg gtattcccat agtttcgccg ggcggacgtg gggaaatgcg 1922700
taacgggcat agtgggcgcg gagcgggcgg ttttatgccc cggatttccg ttttcgcgcg 1922760
aacatatcag cccgcctgcc gcgtttgcgc ttgaaatcgg gtatgtttcg tcttaaaata 1922820
tgctgctttc agggtatagg cacttgcccg aaaagcacgt tacgcgtcta tcttgcgcgg 1922880
cgtgtttttt tttgaccgga tttttccgac cggatgcccc ctgccgaagt cccttcagac 1922940
ggcattgtca agaattttat taaaaacagg attcccatca tgagcacccc cgccctcctc 1923000
gtcctcgctg acggcagcgt atttcacggc acatcaatcg gttacgaagg ttcgacttcc 1923060
ggcgaagtcg tgttcaatac ttcgatgacc ggctatcagg aaatcctgac cgacccgtcc 1923120
tactgcaaac aaatcgttac cctcacctac ccacacatcg gcaacaccgg caccaacgcc 1923180
gaagatgaag aaagccgcag cgtttatgcc gccggcctga ttatccgcga cctgccgctc 1923240
ttgcacagca acttccgcgc ctccgaaagc ctgcacgact atctggtacg caacaaaacc 1923300
gtcgccatcg ccgacatcga cacccgccgc ctgaccacgc tgttgcgcga aaaaggcgcg 1923360
caaggcggtg cgattctgac cggtgcggat gccacaatcg aaaaagcgca agaactcatc 1923420
gccgcgttcg gcagcatggt cggaaaagat ttggcaaaag aagtttcctg cacggaaact 1923480
tacgaatgga cggaaggcga atgggcattg ggcaagggtt tcgttacccc tgacgaacag 1923540
ccttaccacg tcgtcgccta cgatttcggc gtgaaaacca acatcctgcg tatgctcgcc 1923600
tcgcgcggct gccgcctgac cgtcgtcccc gcccaaacga gcgcggaaga cgtgttggca 1923660
ctcaaccctg acggcgtatt cctatccaac ggccccggcg accccgagcc ttgcacctac 1923720
gccatcaaag ccgtacaaaa actgatagaa agcggcaaac cgatttttgg catttgcttg 1923780
ggacaccagc tcatcagcct cgccatcggc gcgaaaaccc tgaaaatgcg cttcagccac 1923840
cacggtgcga accaccctgt gcaagatttg gacagcggca aagtcgtcat caccagccaa 1923900
aaccacggtt ttgccgttga tgccgacacc ctgcccgcta acgcacgcat tacccacaaa 1923960
tccttgtttg acaacacttt gcaaggcatc gagctgaccg acaaacctgt gttctgcttc 1924020
caaggccacc ccgaagccag ccccggtccg caagatgtcg gctatttgtt tgacaaattc 1924080
attggcaata tgaaagcggc aaaacgggca taatggtttt cagacggcaa cagtatgctg 1924140
ctgccgtctg aaaaacaaag ctggaaatga agattagcgc actcgaccat ctagtactaa 1924200
ctgttgccga cattgaccga accatcgcgt tttatagtga attaaattta aaccggtaca 1924260
gcgttggctc gccttgccgt actatttgta ctgtctgcgg ctcgccgcct tgtcctgatt 1924320
tttgttaatt cactatacac acaagttttg ggcatggaag aagtttcatt tggcagcgac 1924380
cgtaaagctt tgttgtttgg cagtcagaaa atcaacctac acgggcgcgg tgcggaaatt 1924440
cagcctaacg cgcaacacgc cgcctgcggc acagcggatt tatgcctgct gaccgatacg 1924500
ccactggaaa cggttttaca ggaattatcc gcacacggca tcaaaccttt aagcggcatc 1924560
gtagcgcgca caggcgcaat gggcaaaatc caatcggttt acctgcgcga tcccgatggc 1924620
aacctgctgg aaatcagcag ttattgattt tcagacggct tatgcaaaat aaaaaacagc 1924680
ctgcacaagc tgttttcctt gcagcctctt taaccccaac agccgccccg tcctctctcc 1924740
ctgtgggaaa gcgttagaga gagggcaaca agccgcaagg cttgtgtttg ggcggttagg 1924800
gtgttgggga aggttgccga aattcgggga atgccctctc cccggccctc ccccacgggg 1924860
gagggagaag gttgcagcag attttgcggt tgcaggcggt ttgaaaggca acttagattt 1924920
gcagctgttg tttcaggtca tctgaaaaat aaaaagcagc ctgcacaacc tgttttcctt 1924980
gcaaaaccct taatcccaac cgccaccacg tcctctctcc catgggagag agtcagagag 1925040
agggcaacaa actgtaaggc ttacacaaac agtaacccga caacagaatg agcacgcacg 1925100
agaaactttt aaccgccgac aaccccgtcc tgcatcaacg cgccaaagcc atgcgccaag 1925160
aaatgagcga ggcggaagca aaattgtggc agcacctgcg ggcaggccgt ctgaacggct 1925220
ataaattccg ccgccagcag ccgatgggga attatattgt tgattttatg tgcgtaacgc 1925280
ccaagctgat tgtcgaagca gacggcgggc agcacgcgga acaagccgta tacgaccacg 1925340
cgcggacggc atatctcaac agcctgggct ttaccgtgct gcgtttttgg aatcacgaaa 1925400
ttttgcagca gacaaacgat gtactggcgg aaatcctgcg cgtattgcag gaattggaaa 1925460

agcagtatgc gcaataacaa acggttaatt ttgattagag ttttgaaaat tataggatac 1925520
aggtagggta caggctgctt gaattgagcg tttagaagac cgtctgaaaa acaaaaaaca 1925580
gcccgcacaa cctgtttttc ctgcagaacc cttaattcca acagccgccc cgtcctctct 1925640
ccctgtggga aagcgttaga gagagggcaa caagccgcaa ggcttgtatt taggcggtga 1925700
aggcattggg gaaggttgcc gaaattcgga gaattccatc tccccagccc tcccccacgg 1925760
gggaggggggc aggttgcagc ggattttgcg gttgcaggcg gtttgagaaa gaatgcccga 1925820
aatatcaaca gcgggaattt ttcaggcagc ctttatcgca aggcaggtgg aacaaacgcc 1925880
gcgaacgttt tttcagacga cctttgaact catcggcaga gagtgtgccg caaggcacgc 1925940
acgcggtggg ttggggttgc agggaaaatg gagaacgcgt gcatacgtac cgcacatacc 1926000
ctacatacgg gctacggctt gctacgatac gggggtttcg atatacaagt taggttttag 1926060
caaacccaac attttagaca attaagcggt ttgtgttggg ttttcaaccc aacctacgct 1926120
tgctacgttt attgcaacat attcgcagga gtttaaatat gtcaatacct attaatttca 1926180
ataatttaaa gtatttgctt aatgatatga gaaacaaaaa tagaataatt gaagcatttc 1926240
cttttaatta taatcaaagg caatacgccg ttattttgac taggtataaa cctgatgaac 1926300
ctagaccaga tgattatgca caagcaaaat tagagttttt taatttgaat agtgaaaatt 1926360
caatatttgc gtatgctgat ttttatgaag ttcattttaa aagtgctact gattttatta 1926420
attttttttaa aattaatgtt caggctggtg ctgcgaaaat cagagaaatt tttcagagtt 1926480
ttagtaatct ttttgcagat ttcattccaa cacaaactaa aaaagattta gacataattt 1926540
ataaaaagat tgtagctact cgtttagaac ctaattctcc taacactatt tattgctatg 1926600
atgtccgtag aaatgggaaa gataaggctg gcaagcctaa tcgcaggagc gtggaaaata 1926660
gtgaaaaagc aaaaattttg cgcccagagc tatacgaaaa atttaaagcc gatagtaatt 1926720
acagtttttt cttttcagat aatccaagcg atgaaaaaac agatgcagaa ataattagag 1926780
aagttaccaa tcgtcaataa tccaaattct tcaaaagaaa gacccaccat gcccaaacgt 1926840
accgacctaa aatccatcct tatcatcggc gccggcccta tcgttatcgg tcaggcctgc 1926900
gaatttgact attcgggcgc acaggcctgc aaagccttgc gtgaagaagg ctataaagtc 1926960
attttggtga attccaaccc cgccacgatt atgaccgacc ccgaaatggc ggatgttacc 1927020
tacatcgagc cgattatgtg gcagacggtg gaaaaaatta ttgccaaaga gcgtcctgac 1927080
gcgattctgc ctaccatggg tggtcagact gcgctgaact gtgcgctgga tttggcgcgc 1927140
aacggcgtgc tggcgaaata caatgtcgag ctgatcggcg cgaccgaaga cgccatcgac 1927200
aaagcagaag accgtggccg ctttaaggag gcgatggaga aaatcggcct ctcctgcccg 1927260
aaatcttttg tctgccacac gatgaacgaa gctttggcgg cgcaagaaca ggtcggcttc 1927320
cctaccctga ttcgtccttc tttcaccatg ggcggttcgg gcggcggcat tgcctacaat 1927380
aaagacgagt tttggcgat ttgcgaacgc ggtttcgatg cgtcgcccac gcacgagctg 1927440
ttgattgagc agtccgttct cggctggaaa gagtacgaga tggaagtggt gcgcgataag 1927500
aacgacaact gcatcatcat ctgctcgatt gaaaacttcg acccgatggg cgtgcataca 1927560
ggcgactcga ttacggttgc gccggcgcaa acgctgacgg acaaggaata tcaaattatg 1927620
cgtaatgctt cgctggcggt attgcgcgaa atcggcgtgg acacgggcgg ctcgaacgtg 1927680
cagtttgcgg tgaaccctgc aaacggcgag atgattgtga ttgagatgaa cccgcgcgtg 1927740
agccgttctt ccgcgttggc ttccaaagca acgggtttcc cgattgcgaa ggtggcggcg 1927800
aagctggcgg tcggctttac gctggacgag ttgcgcaacg acatcaccgg cggcaaaacc 1927860
cccgcgtcgt tcgagccttc catcgactat gtggttacca aaatcccgcg tttcgcgttt 1927920
gaaaaattcc ctgccgcaga cgaccgcctg accacgcaga tgaaatcggt gggcgaagtg 1927980
atggcgatgg gccgcacgat tcaagaaagt ttccaaaaag ccctgcgcgg cttggaaaca 1928040
ggcttgtgcg gcttcaatcc gcgcagtgaa gacaaagcgg aaatccgccg cgaactggcg 1928100
aaccccggcc ccgaacgtat gctgtttgtg gcagacgcgt tccgcgcggg cttcacgctg 1928160
gaagaaatcc acgaaatctg cgccatcgac ccttggttct tggcgcaaat cgaagacttg 1928220
atgaaggaag aaaaagcggt ttcagacggc attttgagtg atttggattt cgccgcccta 1928280
cgtcgtctga aacgcaaagg cttctccgac aaacgtttgg cacaattgtt gaacgtaagc 1928340
gaaaaagaag ttcgcgaaca ccgctacgcg ctgaagctgc atccggttta caaacgcgtc 1928400
gatacctgcg ccgccgagtt cgccaccgaa accgcctatc tttactccac ttacgaagaa 1928460
gaatgcgaat ctcgtccttc cgaccgcaaa aaagtgatga ttctcggtgg cggcccgaac 1928520
cgcatcggtc agggcatcga gtttgactac tgctgcgttc acgccgcgct cgccctgcgc 1928580
gaatcgggct ttgaaaccat catggtcaac tgcaaccccg aaactgtgtc caccgacttc 1928640
gacaccagcg accgcctgta tttcgagccg ctgacgctgg aagacgtgtt ggaaatcgtc 1928700
cgcaccgaaa acccgtgggg cgtgattgtg cattacggcg gccaaacccc gctcaaactc 1928760
gccaacgcgc tggttgaaaa cggcgtgaac atcatcggca cgtccgccga cagcatcgac 1928820
gccgccgaag accgcgaacg cttccaaaaa gtgttgaacg acttaggcct gcgccaaccg 1928880
cccaaccgca tcgcccacaa cgaagaagaa gcgctcgtca aagccgaaga aatcggctat 1928940
ccgctggtcg tgcgcccgtc ttacgtcctc ggcggccgcg ccatgcaggt cgtccattcc 1929000
gccgaagagc tgcaaaaata catgcgcgaa gccgtgcagg tttccgaaga cagccccgtg 1929060
ttgctcgact tcttcctgaa caacgcgatt gaagtggatg tggactgcgt ttcagacggc 1929120

aaagacgtgg ttatcggcgg catcatgcag cacgtcgaac aggcgggcat ccactccggc 1929180
gactccggct gctcgctgcc gccctactcc ttaagcgaag aaatccaaga cgaaatccgc 1929240
cgccaaacca aagcgatggc gtacgcgctg ggcgtggtcg gactgatgaa cgtgcagttt 1929300
gccgtacaag acggcgtagt gttcgtattg gaagtgaacc cgcgcgccag ccgcaccgtg 1929360
cccttcgtct ccaaagccac cggcgtgccg ctcgccaaag tcggcgcgcg ctgcatggca 1929420
ggcatttccc tgaaagaaca aggcgtggaa aaagaagttg tccccgattt ctatgccgtt 1929480
aaagaagccg tgttcccatt catcaaattc ccgggcgtgg atacgatttt gggaccggaa 1929540
atgcgctcca ccggcgaagt catgggcgtg ggcgcaagct ttggcgaagc ctactacaaa 1929600
gcccaactcg gcgcgggcga acgcctcaac ccgaccggca aaatcttcct ctccgtgcgc 1929660
gaagaagaca aagaacgcgt cattaaaacc gctaaaaact tccaagtttt aggctacggc 1929720
atctgcgcca cgcgcggcac ggcgcaatac ctgaccgaac acgggctgat tgtgcagacc 1929780
atcaacaaag tacccgaagg ccgcccgcac atcggcgacg cgctgaaaaa cggcgaaatc 1929840
gcactggtcg tgaacaccgt ttccagcgat ccgcaatccg tgtccgacag ccacatcatc 1929900
cgccaaagcg cattgcagca acgtgtgccg caatacacca ccaccgccgg cggcgaagcg 1929960
atgagcgaag gcgcgaaaag ccgagaccat ctgggcgtgt acagcgttca agaactgcac 1930020
gggcgtttga aaaaccgcaa ctgatgcctg aatcaggttg aaaatgccgt ctgaagccgt 1930080
tttgcggttt cagacggcat tttgtcattt ggaaagccga tgttgccaca cacaagccgt 1930140
acataaggaa cagccctatc acgctcccca tatagattgc cattgccgcc gactatacat 1930200
tatcttattt attttttctc aaagttatta agtgagtaaa aacagtttta tgacaggttt 1930260
ttatagaatt atccacagag attgtttccc agttcctcca ctaaaaaatc caaaaatacg 1930320
cgtaagcgga gattgacggc tttatcgctg taataaacag cattaaaggg gtgtgtttta 1930380
tcggaggttt gttcggcgag caggggaatt aactttcctt cagcgatgtc gttgtcaacc 1930440
aaaaaatctg ataagcaaac aataccgcaa cctgaaaggc acaacgagcg taagatttca 1930500
ccgctgctgg cggtaaagtg cggtgaaatc ttatagggat ttccctgcgc atctaaaacc 1930560
gcccatgtat ttagagaacc gggttcggtg aagcctaaac attggtggcc ggcaagctct 1930620
tctgtagatt gcggcgtgcc gtgttttgcc aggtattcag gactggcgat tacgcggaag 1930680
cggctgtcaa acagatggcg tgcacgcagc ccggaatcgt ccaattctcc ggcccgtaag 1930740
gcaatatcga ctttgcgttc aatcagattg atatagcctt cggaagaaac gagcgaaagt 1930800
cggatatgcg gatagcgttc gttgaatttt gctgccagcg gcgccagcag atgcagcacc 1930860
atcggcatcg cggaatccac gctcaacacg ccttgcggta tttcgtgcac tgccagcatt 1930920
tcggtttccg ccgctgccat ttcttgcagg attctctgcg cgcggcggaa atattgcgcg 1930980
ccttcttccg tcagactgag ttgccgcgtg gtgcggttga gcaggttcac acccaacttt 1931040
tcctccagcc gtttgacgat gcggcttacg gcagaatttg ccatcgccaa ctgctccgcc 1931100
gcacggctga agctgccgct ttccaccact tgaacaaata cggtcagttc ttctgaattg 1931160
gttttcatcg tgtttccttt tcggttggaa ccccgccctt tagggcggca ggatcagact 1931220
ttatttggga ggggtgtaac cccttccgaa tcaggacggc acacagggcg gtgctttatg 1931280
tgccatcccg tgtgttggaa catctgatta tttcatttga cgcaaaagtg ttttcttatt 1931340
tttgcacttt taaattataa agtaaaacgg cacaatacat tcatcaattc acaaacgagg 1931400
taacaaatga atattttatt attagacggc ggcaaggcgt tcggacattc tcacggcggg 1931460
ttaaaccgta cgcttcacaa aaaagcgaaa gaagttttga ccgcgctcgg acacaatgtt 1931520
caagaaaccg tgattgatgc cggctatgat gttgaggcag aaatcgaaaa gttcgtttgg 1931580
atggatgctg tgatttggca gatgccgggc tggtggatgc acgagccttg gacagtgaaa 1931640
aaatacatag acgaagtatt aaccgctgga cacggcaaac tctaccaaag cgacggcaga 1931700
cacagcgtca atccgactga gggctacggc acaggcggct tgttgcaagg caaaaaacat 1931760
atgatttcac tgacttggaa tgcgccgatt gaagccttta cccgcgaagg cgatttcttt 1931820
gaaggcaaag gcgttgatgt tttgtatatg cacttccaca aagccaacga gtttttgggt 1931880
atgacccgcc tgccgacatt cttatgtaac gatgtggtta aaaatccgca agtggaaaaa 1931940
tacttggcag attatcaggc acacttggaa aaagtgttcg gctaaaaatt tatcttataa 1932000
acaaacaaag gcagcctgaa agattgaatg gtctgcaccc ctaaggttgg actaaccaac 1932060
cgactaaggt gcagattatt ttttgttgct ttttcagctt ttcgttgggt tagatattct 1932120
tgcccactgt tttcaggcag ccttgaatac aaaaaaatgg cgtatgtaat atgtttatac 1932180
gaccaaaacg gaatgaattt taacgtattg cgcgtcatca acaatgactg agtttctcgc 1932240
ctctcgcgcc tgaatctata gtggattaac aaaaaccagt acagcgttgc ctcgccttgc 1932300
cgtactattt gtactgtctg cggcttcgtt gccttgtcct gatttaaatt taatccacta 1932360
tatgtcatgc agttcctttc attcaaatca acaaaagaat gccgtccgaa cgtccgttca 1932420
gacggcactt gtcttcccac aatagacttg aggctgttct aacgtaccac cccttcgttc 1932480
cgccccaaaa ccatcgcatc gccgtagctg aagaaacggt attcgcgttc gaccgcatga 1932540
cgatacgcgg cgcggatatg acccataccc gaaaacgcgc tgaccaacat cagcagcgtc 1932600
gatttcggca aatgaaaatt ggtaaccagt ctgtcgacaa cattaaaacg gtagcccggc 1932660
gtgatgaaaa tatcggtgtc gccctgcccc gctttcagac gacccgtcgc acgcgcggca 1932720
gattcgaggg cgcgcatgga agtcgtgccg accgcccaga ctttgttccc ccgggctttt 1932780

gccgcctcaa cggcggcggc ggtttcagac ggcacttcaa accattcgct gtgcattttg 1932840
tgctcttcga ttttgtccac acgcacgggt tggaacgttc cggcaccgac gtgcagggtt 1932900
acttctgcgg ttaccgcgcc tttgtctttc agacggtgca aaagttcttc cgtaaaatgc 1932960
aggcccgccg tcggcgcggc gaccgcgccc tgatatttgg cataaacggt ttgataacgg 1933020
ctgtcgtcat ccgcatcggc ggcgcgttcg atataaggcg gcaggggcag gtgtccgttc 1933080
tgttccaaaa gttcgtaaac ggtctctccg ccttcaaaac gcaggcagaa cagttcgccc 1933140
tcacgcccga ccgtcacggc gcggatgccg ccttcaaaca ccagccccat accgggcttg 1933200
ggcgatttgg acgaacggat gtgcgccagt gcggtatggt tgtccaacac gcgctcaatc 1933260
agggcttcga tcctgccgcc gctgtctttc tgcccaaaca gccgcgcctt catgactttg 1933320
gtgttgttga acaccaaaac gtcgcccgcc tcgacataat ccggcaaatc gccgaacacc 1933380
cggtcttgca gcggcatatc gggcaacgca accaaaaggc ggctgctgcc gcgcacttcg 1933440
ggcggatgct gggcaatcag cttttcgggc agggtaaaat caaaatctga aatatccatt 1933500
tttacactct cgttcgggca agccgccatt atacgcactt tagccctttt tcagacggca 1933560
tctttgtccg aaaaaccaac agattagaat aaacactctt aacctggaac atcttgtgcg 1933620
caaaatcaaa cttcctgcac atttccccca aaaaccgccg tttttttgata ttttactgga 1933680
catttaccga caacttcggg aaaataaaca cattctcacg gtcgttttcc accacaggaa 1933740
aaccgtatcc gaacaccatt ccgcccggtt tgcgccgttg ccgcaagccg gctgttttct 1933800
gaaaaaccaa cgcaacaacc cgccggaaca ccggcagcct ttaaaggaac agaaatggat 1933860
ttgcgcaaat taaaaaaact gattgatttg gttgaagaat cgggtatcgc cgaaatcgaa 1933920
gtaaccgaag gcgaggaaaa agtccgcatc acccgaacca tcgccgccgc acccgtttac 1933980
gccgcgcccg tacctgccgc cgcgccggcc gtaacgcctg ccgccgcacc cgttgcggca 1934040
tccgcgcccg ccgccgcacc tgccgcccgc gatttgtccg acgcgcaaaa atcgcctatg 1934100
gtcggcacgt tctaccgcgc acccggcccg aatgccgcgc cttttgtcga agtcggccaa 1934160
caagttaaag ccggcgacac gctgtgcatc atcgaagcga tgaagctgat gaacgaaatc 1934220
gaagccgaaa aatccggcac ggtcaaagaa attttggtcg aaaacggtac gcccgtcgaa 1934280
ttcggcgaac cgctcttcat tatcggataa tcctgttttc agacggcata aacttccgat 1934340
gccgtctgaa atgctttccc ccttcagcgt tcccgcaccc ttttttacgg acgggttgcc 1934400
ggaaccgcag gaaaggtcat catgctgaaa aaagttttaa tcgccaaccg aggcgaaatc 1934460
gcattacgcg tactccgtgc ctgccgcgaa atgggcattg ccaccgtcgc cgtgcattcc 1934520
gaggccgaca aagacagcct gcacgtcaaa ctcgccgacg aatccgtgtg catcggccct 1934580
gccgcttccg cgcaaagcta ccttaacgtc cccgccatta tcgccgccgc cgaagtaagc 1934640
tgcgcggacg ctgtccatcc gggttacggt ttccttgccg aaaacgccga tttcgccgaa 1934700
caggtcgagc agtccggctt tacctttatc ggcccgaaac ccgacaccat ccgcctgatg 1934760
ggcgacaaag tctccgccaa acacgcgatg atagcggcag gcgtaccctg cgtccccggt 1934820
tctgacggcg cattgcccga cgacggcgaa gaaatcctca aaatcgccga taaagtcggt 1934880
tatcccgtca ttatcaaagc ctctggcggc ggcggcggcc gcggtatgcg cgtggtcgag 1934940
aaaaaagaag acctcctcca atctgtcgaa atgaccaaag ccgaagcagg cgcggcattc 1935000
ggcaacccga tggtttacat ggaacgctat ttgcaacgtc cgcgccacgt cgaaatccaa 1935060
gtgattgccg acgaacacgg caacgccatc taccttgccg agcgcgactg ttcgctgcaa 1935120
cgccgccacc aaaaagtcat cgaggaagca ccggctccgt tcatcactga aaaagaacgc 1935180
gccaaaatcg gcaacgcctg tgccgatgcc tgcaaacgca tcggctaccg gggcgcgggt 1935240
acgtttgagt tttatacga agacggcgaa tttttcttta tcgagatgaa cacgcgcgtt 1935300
caggtcgagc atccggttac cgagctcatc accggcgtgg acatcgtgca ggagcaactc 1935360
cgcatcgccg ccggcctgcc tttgcaatac aaacaaaagg atattcaagt cgaaggccac 1935420
gcgtttgagt gccgtatcaa cgcccgaagac ccgtacaact tcattccaag cccgggcctg 1935480
attgaaagct gccacctgcc cggcggcttc ggtatccgcg tggacagcca catttaccaa 1935540
ggctaccgca tcccaccgta ctacgacagc ctgatcggca aaatctgcgt acacggcaaa 1935600
acgcgtgaac aggcaatggc gaaaatgcgc gtcgcactcg ccgagctggc ggtaaccggc 1935660
atcaaaacca atacgccgct tcaccgcgac ctgttcgccg atgcgggttt ccaaaaaggc 1935720
ggcgtcagca tccactattt ggaacactgg ctggaagatc gcaaagccaa acaggacaag 1935780
taaaccgccg ccgatatgcc gtctgaagcc gcccgtccgc gttcagacgg catttccctt 1935840
gccccgcgcc gtctgaaacc gatttcgata tagtggatta actttaaacc agtacggcgt 1935900
tgcctcgcct tagctcaaag agaaagattc tctaaggtgc tgaagcacca agtgaatcgg 1935960
ttccgtacta tttgtactgt ctgcggcttc gtcgccttgt cctgatttaa attcaatcca 1936020
ctatatttcc aagaaagccc gttatgccct accaacaaat caccgtcaac gtcaacgatg 1936080
ccgtcgccga acgcctcgcc gacgcgctga tggaacacgg cgcactctcc gccgccatcg 1936140
aagatgccta cgccggcacg caaaacgaac aggcgatttt cggcgaaccc ggtatgcccg 1936200
ccgaacaaat ctggcagcag agcaaagtca tcgccctgtt cggcgaacac gacgaagccg 1936260
ccgccatcat ccaaaccgcc acacaagaat gcgggttaaa agacttggca tacaccggcg 1936320
aaaccatcga agaccaagac tgggtgcgtc tcacgcaatc gcaattcgac cccatccgga 1936380
tttccgaccg cctgtggatt accccctctt ggcacgaagt ccccgaaggc agtgccgtca 1936440

acctccgcct cgaccccgga ctcgccttcg gcaccggcag ccacccgacc acgcgcctct 1936500
gcctcaaatg gttggatacg caactcaaaa acggcgaaag cgtcctcgac tacggctgcg 1936560
gttcgggcat cctgaccatc gccgccctca aactcggtgc aggtttcgcc gtcggcgtgg 1936620
atattgacga acaggccgtc cgcgccggca aggacaacgc cgcgcaaaac aacgtcgatg 1936680
cacaattctt cctgcccgac ggtctgcctc aagggcaatt cgacgtagtt gtcgccaaca 1936740
tcctcgccaa ccctttgcgt atgcttggcg aaatgctcgc cgcccgcacc aaacagggcg 1936800
gacgcatcgt gttgtccggt ttgttggacg aacaggccga agaactcggc ggcatttaca 1936860
gccaatggtt cgacctcgac ccggcggaaa ccgaggaagg atgggcgcga ttgagcggcg 1936920
taaaacgctg aaacggaaag gaaacaccgt gcaggataaa aacaacctct gctggctcga 1936980
tatggaaatg acggggctga atcccgaaac cgaccgcatt atcgaagtcg cgatgattat 1937040
taccgactcg gatttgaatg tgttggcgca atccgaagtt tacgccgtcc accaaagcga 1937100
cgacgtgctg aacaaaatgg acgaatggaa caccgccaca cacggcagga cggggctgac 1937160
acagcgcgta cgcgaatcgt cgcataccga agccgaagtc gaacagaaac tgctggactt 1937220
tatgtcggaa tgggtacccg gacgcgccac gccgatgtgc ggcaactcca tccaccaaga 1937280
ccggcgtttt atggtcaaat atatgccgaa actggaaaac tacttccact accgcaacct 1937340
cgacgtttcc acgctgaaag aactcgccaa acgctggaat ccgcccgttg ccaaaagcgt 1937400
cgtcaaacgc ggttcgcaca aggcattgga cgacattttg gagagcatcg aagaaatgcg 1937460
ccactaccgc gaacactttc tgatttccgc cccgagagcc gaagcgcaat aagaaacaaa 1937520
caatgccgtc tgaaacgcag tttgcatttc agacggcatt tttacagcag attgaaatca 1937580
aaaatataca cgcccgtcat tcccgcacag gcgggaatcc ggaaggtcgg gcctgccgtt 1937640
attttcaatc attacagaaa ctgaaaggtc tggattcccg cctgcgcggg aatgacgggc 1937700
gtgtgcattc ttatagtgga ttaacaaaaa tcaggacaag gcgacgaagc cgcaaacagt 1937760
acaaatagta cgaaaccgat tcacttggtg cttcagcacc ttagagaatc gttctctttg 1937820
agctaaggcg aggcaacgcc gtactggttt ttgttaatcc actatacttc aatctgccaa 1937880
acagatcgaa cagagaaacc ctgtccgtca aaacatcatt cagccatcgc cttgaacact 1937940
tcaaccgcaa ccgcaaccgt ttcgtcaatc agctcgggcg tatgcgcggc ggaaacgaaa 1938000
cctgcttcat aagcggacgg gccgaaggcg acattgcggt cgagcatccc gtggaaaaac 1938060
tgtttgaagc cttcaatatt ggaacgcgcc atatcggcat agtttcgcgg cgcgtgtgcg 1938120
gcgaaataca gaccgaacat accgcccacg ctgtcggcgg tgaactcgat gcccgccgca 1938180
tccgctgccg tccgaaaacc ttgaaccaac tgttcggtac gcgccgtcag gttttcatag 1938240
aagccttcgc gctggatgat ttccagcgtt ttcaagcctg cggcgacagc aatcgggttg 1938300
cccgacaagg tgcctgcctg atacacgccg cccagcgggg aaatacattc cataatgtct 1938360
ttgcgcccgc caaacgcggc aagcggcata ccgccgccga tgactttgcc catcgtggtc 1938420
aggtcgggcg tgatgccgtg caaagattgc gcgccgccga gcgcgacgcg gaagccggtc 1938480
atcacttcgt cgtaaatcaa caccgcgccg tatttttcgg tcaatccgcg caaggctttg 1938540
acaaaggctt cggtcgggcg gacgaggttc atattgccga cgaagggttc gacaatcacg 1938600
caggcgattt cattgccgct ttgagcaaag gcttcttcga gttgggcgat attgttgtac 1938660
tcgagtacca aagtgtgttt ggtaaagtcg gcaggcacac cggcggaaga cgggttgcca 1938720
aacgtcagca gaccgctgcc ggctttcacc agcaggctgt cggaatgccc gtggtagcag 1938780
ccttcaaact tgatgatttt gtcacgcccg gtaaaaccgc gtgccagacg gatggcggtc 1938840
atggtcgctt cggtaccgga gctgacgagg cgcagccgtt cgacggacgg catgattttg 1938900
gcgatttctt cggcaatgac gatttcgcct tcggtaggcg cgccgaacga caaaccgccc 1938960
aatgcggctt cgcatacggt ttcgacgact tcggggtgcg cgtgtccgac aatcgcaggt 1939020
ccccacgagc cgacgtaatc ggtatagcgc gtgccgtttt cgtcccaaac atacgcgcct 1939080
tcggcttttt tgataaagcg cggtacgccg ccgacgctgc cgaatgcgcg gacgggggaa 1939140
ttcacgccgc cggggatgat ggctttggcg cggtcgaata aaatttcgtt acggttcata 1939200
tatatcctca aatgccgtct gaacggcagg tttcgggctt ggaagcagaa agccccattt 1939260
tatcattttt caggttgcga caaggatttg cccgcttctt tgcggatcac gccaaccgca 1939320
tcccggatga cggaacgctc gtctttttcc actttatgtg taaagcggta gtctcggacg 1939380
actccctccc cgtcgtaatc cacacaccac tcccaatgtc ggcgttctga tttcatataa 1939440
atgaaattgg tcggcaaaaa attataaatc ggcaggctga cttcatgata ggcataacaa 1939500
ccgaaagggt tgcgcttccc gaaacgtgcc tctacacctc cgcccgggtc gttttgcctt 1939560
taacaaccgt ttgtgcgatt ccctcttccg tctgatatag tggattaaca aaaatcagga 1939620
caaggcgacg aagccgcaga cagtacagat agtacggcaa ggcgaggcaa cgctgtactg 1939680
gtttttgtta atccactata acgcaggaac tgatgttccc tgtcgccgaa attgctggta 1939740
cacgcacaca gcagcaatgc cgcccataca gccggtttca tacacatctc ccattaaagc 1939800
caaacattat acagccgtcc cgaccgatta aattcatatt ttaaaacaat atcctgcctc 1939860
caaaacccac atcgtgctat aatccgcacc gattttcaga cggcatcgtc gtgccgtctg 1939920
aaatttttc attccaacaa caatcagccc cgcgattacg gctgcctgag aaagacacaa 1939980
accatgaaaa aagtatttat ccgcaccttc ggctgccaga tgaacgaata cgacagcgac 1940040
aaaatgctcg ccgtcctcgc cgaagaacac ggcggcatcg aacaggttac ccaagccgac 1940100

gaagccgaca tcatcttgtt caacacctgc tccgtgcgcg aaaaagcgca agaaaaagtc 1940160
ttctccgatt tggggcgcgt gcgtccgctc aaagaaaaaa accccggcct catcatcggc 1940220
gttgccggct gcgtcgcctc gcaagaaggc gaaaacatca tcaaacgcgc gccttatgtg 1940280
gacgtggttt tcggcccgca aacgctgcac cgcctgccaa aaatgattgt ggacaaagaa 1940340
accagcgggc tgtcgcaagt cgatatttcc ttccccgaaa tcgaaaaatt cgaccacctg 1940400
ccgcccgccc gcgtcgaagg cggcgcggca tttgtatcga ttatggaagg ctgttccaaa 1940460
tactgctcct tctgcgtcgt cccctacacg cgcggcgaag aattctcccg cccgctcaac 1940520
gacgtattga ccgaaatcgc caaccttgcc cagcaaggcg tgaaagaaat caacctcttg 1940580
ggacaaaacg tcaacgccta tcgcggcgaa atggacgacg gcgaaatctg cgacttcgcc 1940640
accctgctgc gcatcgtcca cgaaatcccc ggcatcgaac gtatgcgctt caccaccagc 1940700
cacccgcgcg agtttaccga ctcgattatc gagtgctacc gcgacctgcc caaactggtt 1940760
tcccacctgc acctgccgat tcaaagcggt tccgaccgcg tattgagcgc aatgaaacgc 1940820
ggctacaccg ctttggaata caaatccatc atccgcaaac tgcgcgccat ccgtcctgat 1940880
ttgtgcctga gcagcgattt catcgtcggc ttccccggcg agaccgaacg cgagttcgag 1940940
caaaccttga aactggtgaa agacatcgcc ttcgacttga gcttcgtgtt tatttacagt 1941000
ccgcgccccg gcacgcctgc cgccaacctg ccggacgaca cgccgcacga agaaaaagtg 1941060
cgccgcctcg aagccttgaa cgaagtcatc gaagccgaaa ccgcgcgcat caaccaaacc 1941120
atggtcggca cggtacaacg ctgcctggtc gaaggcatct ccaaaaaaga ccccgaccaa 1941180
ctgcaagccc gtaccgccaa caaccgcgtc gtcaacttca ccggcacgcc cgacatgatt 1941240
aaccaaatga tcgatttgga aatcaccgag gcctacacct tctccctgcg cggcaaagtt 1941300
gtcgaagcct aaaccctcac gccgaaaaaa tgccgtctga agcgtttcag acggcatttt 1941360
gccttgtatc ggcagacgac ggcgcggccg ggcggcttaa tttgccgcat cccgatccga 1941420
cagccacgcg cgcacacgcc gttccaccgc ttcggcactc aagcccaaat cgtctaaaag 1941480
ttttttcgga tcgccgtgtc cggttacggt atcggcaacg cccaaaagca aaacgggttt 1941540
gcagatgccg tgtttcgcca atacttccag caccgcgccg cctgcgccgc cctgttcggc 1941600
gttttcttca agggtaacga tgcggtcgtg gcttcgggca aggcggacaa tcaactcttc 1941660
gtctatcggt ttgacgaagc gcatatcggc gacggtggcg ttcagttttt cggcaaccgc 1941720
caatgcgggg gcgaccatac tgccgaaggc aatgaatgcg gttttctcac cttcgcggcg 1941780
gataatgccc ttgccgattt ccacggtttc catgccgtct gaaaccggcg cgcccgtacc 1941840
cgtgccgcgc ggatagcgga cggcggcggg cgcgtctgcc tgatagcagg tcgaaagcaa 1941900
caggcggcat tcgttttcat cgctcggcgc ggcgacaatc atgttcggca cgcagcgcaa 1941960
aaagctcaaa tcgtacagac cggcatgggt cgggccgtcc gcgccgacga tgcccgcgcg 1942020
gtcgacggca aacaaaacgg gtaggttttg cagggcgatg tcgtgcacca gttggtcgta 1942080
ggcgcgttgt aaaaaggtgg aataaatcgc cacgacgggc ttcatccctt cgcaagccaa 1942140
accgccggca aaggtaacgg cgtgctgctc ggcgatgccg acatcgaaat agcggtcggg 1942200
gaatcgttgt tcaaactcaa ccaagccgct gccctcgcgc atggcggggg taatcgcaac 1942260
cagtcgggaa tctgccgccg cccggtcgca cagccatttg ccgaacactt gggtataggt 1942320
cggtttggcg gcgggcttgg gttctttttc agacggcatt tgcgccgcgc tttctttagg 1942380
caggttggcg acggcgtggt atttgacggg gtcgttttcg gcgagtttgt agccgttgcc 1942440
ctttttggtg atgacgtgca gcaactgagg gcctttgcgg ctgcgcaagt ctttcaatac 1942500
gtccaccaga ttttcgacgt tgtgtccgtc cacggggccg gtgtagcgga agccgaagtt 1942560
ttcaaacaaa gacagcgact gtttggcgtg ttcggcttct tcggcaaggg ttttgatttt 1942620
gtgttcgact ttttgggcaa actccatcgc gccgggtatt ttgtctaata ccttgcccgt 1942680
ttgcgctttg acggtactca acaggccgtg catatcgcgc acgacgttgc tggcaaggta 1942740
tttcggcagc gcgccgacgt tgggggaaat cgacatttcg ttgtcgttga ggacgaccag 1942800
caaatccaca tccatatcgc ctgcgcaatt caaggcttca aacgcctgcc ccgccgtcat 1942860
cgcgccgtcg ccgatgatgg cgacgctgcg gcggtcgctg cccaagagtt tgtctgccgc 1942920
cgccatgccc aacgccgcgc cgatggaggt ggaggaatgc cccacgccga acgcgtcgta 1942980
ctcggactcg caacgtttcg gaaaacccgc caaaccgcca tattggcgca tggtgtgcat 1943040
ctggtttttc ctgcctgtca ggattttgtg cggatagctt tggtgtccga catcccacac 1943100
cagcttgtct tcgggcgtgt cgtacacata gtgcagggcg atggtcagtt cgaccgcgcc 1943160
caaattgctg gcgaaatgcc cgccggtctg cccgacagat tccagcagaa aggtgcgcaa 1943220
ctcgccggca aggcgcggca gctgtttttt gtccagacgg cgcaaatctt gcgggctgtc 1943280
aatcaggtcg agtagggggc ttgggttcat ggtgtgtctt ttttatgtgt cgtccgggtg 1943340
caacggtcaa ttatatatca agagcgtgcg gctgacggct gattttgccg tatgtcattc 1943400
gtcctgccgc ttggcgcgcg ggtgggcttc gtcatacagg cgggcgatgt ggtcgaaatc 1943460
gagcttggta taaatctgcg tggtcgaaag gctgctgtgc ccgagcagct cctgcaccgc 1943520
cctgatgtcg cgcgaagcct gcaataggtg tccggcgtag ctgtggcgca tcatatgcgg 1943580
cgaaacgtgc ctgccgtcgc cgttttgcgc cgcccattgc gccaaacgtt tttggatttg 1943640
gcgttggctc aggcgcgtgc cgttcctgcc ggtaaacagg gctttgccgt ccgatgccgt 1943700
ctgacgcagc ggcagatagt tttcagggc ttccacgctt ttgccgacca gcggcacctg 1943760

ccgctgcttg cgccctttgc cgataacgtg tacccacgcc tcgtccaaat agacatcatc 1943820
tgcattcaag ccgtgtatct cgctcacgcg caaaccgctg ccgtacatca gttcgaacag 1943880
ggcgtggtcg cgcaccgcca gcgggtcgcc gccgtccacg ggcaaatcca gcatccggtt 1943940
cagccattcc tgcggcaggg ctttgggtac gcgctcgggc tgcttcggcg gtttgatgtc 1944000
ggcggtcggg tcggcgtgca tcaggccgcg ctttaccagc caaacgcaat actgccgcca 1944060
agacgaaagc ttgcgagcca gcgtccgttc cccaaaccg cggccggaca gccggcgtaa 1944120
tgcctgtacg aagtcgccgc gagtgcaatt tgaagggttt gcagacggca tttcttccag 1944180
aagggcaagc agttcctgca agtcgcgccg gtatgcggca accgtgtgct ccgatttacc 1944240
ctcgcgcacg atattttcca aataagcgtc caagtatgcc gcaagtccgt ccaaacccat 1944300
tcccacacct aaaataacat tagaaacatt atcataaatc ggaatatccg aatcccgaaa 1944360
cgtcaaaacc cgacaaacct gcatactggc atcgttaata taaaatcaat gagctgttta 1944420
tggtttttttg ctgtaaaaaa cattataatc cgccttattt acctattgcc caaggagaca 1944480
caaatggcac tcgtatccat gcgccaactg cttgatcatg ctgccgaaaa cagctacggc 1944540
ctgccggcgt tcaacgtcaa caacctcgaa cagatgcgcg ccatcatgga ggctgcagac 1944600
caagtcgacg cccccgtcat cgtacaggcg agtgccggtg cgcgcaaata tgcgggtgcg 1944660
ccgtttttac gccacctgat tttggcggct gtcgaagaat ttccacacat ccccgtcgtc 1944720
atgcaccaag accacggcgc atcacccgac gtgtgccaac gctccatcca actgggcttc 1944780
tcctctgtaa tgatggacgg ctcgctgatg gaagacggca aaacccccttc ttcttacgaa 1944840
tacaacgtca acgccacacg taccgtggtt aacttctccc acgcttgcgg cgtatccgtt 1944900
gaaggcgaaa tcggcgtatt gggcaacctc gaaaccggcg aagcaggcga agaagacggt 1944960
gtaggcgcag tgggcaaact ttcccacgac caaatgctga ccagcgtcga agatgccgta 1945020
tgtttcgtta aagataccgg cgttgacgca ttggctattg ccgtcggcac cagccacggc 1945080
gcatacaaat tcacccgtcc gcccacaggc gatgtattac gtatcgaccg catcaaagaa 1945140
atccaccaag ccctgcccaa tacacacatc gtgatgcacg gctccagctc cgttccgcaa 1945200
gaatggctga aagtcatcaa cgaatacggc ggcaatatcg gcgaaaccta cggcgtgccg 1945260
gttgaagaaa tcgtcgaagg catcaaacac ggcgtgcgca aagtcaacat cgataccgac 1945320
ttgcgccttg cttctaccgg cgcggtacgc cgctaccttg ccgaaaatcc gtccgacttt 1945380
gacccgcgca aatacctgag caaaaccatt gaggccatga agcaaatctg cctcgaccgt 1945440
tatcttgcgt ttggctgcga aggtcaggca ggcaaaatca aacctgtttc gttggaaaaa 1945500
atggcaagcc gttatgccaa gggcgaattg aaccaaatcg tcaaataaca ggttgcctgt 1945560
aaacaaaatg ccgtctgaac cgccgttcgg acgacatttg atttttgctt ctttgacctg 1945620
cctcattgat gcggtatgca aaaaaagata ccataaccaa aatgtttata tattatctat 1945680
tctgcgtatg actaggagta aacctgtgaa tcgaactgcc ttctgctgcc tttctctgac 1945740
cactgccctg attctgaccg cctgcagcag cggagggggt ggtgtcgccg ccgacatcgg 1945800
tgcggggctt gccgatgcac taaccgcacc gctcgaccat aaagacaaag gtttgcagtc 1945860
tttgacgctg gatcagtccg tcaggaaaaa cgagaaactg aagctggcgg cacaaggtgc 1945920
ggaaaaaact tatggaaacg gtgacagcct caatacgggc aaattgaaga acgacaaggt 1945980
cagccgtttc gactttatcc gccaaatcga agtggacggg cagctcatta ccttggagag 1946040
tggagagttc caagtataca aacaaagcca ttccgcctta accgcctttc agaccgagca 1946100
aatacaagat tcggagcatt ccgggaagat ggttgcgaaa cgccagttca gaatcggcga 1946160
catagcgggc gaacatacat cttttgacaa gcttcccgaa ggcggcaggg cgacatatcg 1946220
cgggacggcg ttcggttcag acgatgccgg cggaaaactg acctacacca tagatttcgc 1946280
cgccaagcag ggaaacggca aaatcgaaca tttgaaatcg ccagaactca atgtcgacct 1946340
ggccgccgcc gatatcaagc cggatggaaa acgccatgcc gtcatcagcg gttccgtcct 1946400
ttacaaccaa gccgagaaag gcagttactc cctcggtatc tttggcggaa aagcccagga 1946460
agttgccggc agcgcggaag tgaaaaccgt aaacggcata cgccatatcg gccttgccgc 1946520
caagcaataa ccattgtgaa aatgccgtcc gaacacgata atttaccgtt cggacggcat 1946580
tttgtattgc accgtccgac ggcatgccca agggggggaaa tccctatttt caggccaacc 1946640
gctatataat gccgtctgaa ccaacgagag aatgccatgc aagctgattt taaccgtccc 1946700
gtcctggccg tcgataccgg tacttcccgt ttgtcgctcg cgctgcgtgc cgacggcgaa 1946760
acccgtctgt tccatcagga agtcggcagc cgccagtccg aactgattct gccggaaatc 1946820
cgcaccctat tccgcgatgc aggcattacc gccgccgatt tgggtgcggt cgtgtacgca 1946880
cagggtcccg gcgcgtttac cggactgcgt atcggcatcg gtgtagctca gggtttggca 1946940
acgccgtttg atacccccctt aatcggcgta ccctcgctcg atgccgccgc ctcgctgccg 1947000
ccgccgcaaa gctgcatcct tgccgctacg gacgctcgta tgggcgaagt gttttatgca 1947060
tggttcgata cgctgaactg ccaccgtttg agcgattatc aggtcgggcg ggcggcagac 1947120
atccggctgc cggagggatg cgccttttca gacggcatag gcagcgcgtt cgcgctggaa 1947180
gaagctccgc cgttctcagg cagaccggat atgccgactg ccgccgactt tctcgcattg 1947240
gcagccaagg gcggttatcc tgccgtccat gccgcacacg ccggtttgct ctacgtccgc 1947300
aacaaaatcg ccctgactgc caaagaacag gccgaacgga gagcgcgccc gtgaacatcc 1947360
gccgtgccgt ttgtgccgat tgtgaggagc tggccgcact cgatgccgtc tgcaacccgt 1947420

ccgcatggac gcaacgccaa tttgagtccg cactggtttc gccgtccgaa caggttttcc 1947480
ttgcggaaaa agacggcggg attgccgcct ttatcgtttg gcagaacctg cccgacgaat 1947540
ccgaactgca cctgattgcc accgcgcccg aatgccgccg ccaaggaatt gcgtccgccc 1947600
tgctcgaata ttggttcaca catctgcccg aagacacgca acgcctgctg ctcgaagtcc 1947660
gtgcaggcaa caccgccgca caggcactgt acacggcgca cggcttcagc attacgggca 1947720
ggcggaaaaa ctattaccgt acagccgacg gtaaaaccga agatgccgtc ttaatggaga 1947780
aaatatgtta agcgcgcgct acctccacct gcacgaagct ttgggtttgg gtccgatgtg 1947840
gctgaaacag gccgccgccg tcctgccgcc caaaaacaca cccgcaccct cggcacaggc 1947900
acgtccccaa accgtccgcg ccgccccgat ccgcccttcc caaccccata acggtcaggc 1947960
gcggctcgaa acgatgaaag cgttggaaac cgccgccgta cctacgcgca aacccgcgcc 1948020
tgaaaccgaa acgcctctgc ccggcctttc agacggcatc gcccccgttc ccgccgcttc 1948080
gggcatcacc aagcttgccg tcgtcagcct ttgcccaccg atcgaggatg cggtttacgg 1948140
gcaactgttc cacggcaaag caggcatcct gctcgacaac atactcaaag ccgtaggact 1948200
ggatgccgcc tatgtccaca aaacctgttg ggtgaaaacc gccgccgtcg gcaacccgat 1948260
gccgtctgaa caggccgtcg cgaatgcgct gggtcaaatc gcccgcgaac tcgacggctg 1948320
ccgcgccccg gctgtccttt tcctcgggca ggcttttgtc cagcctgaac ggcaaacgat 1948380
gattgaaact ttgtgcggca gccgtccctt cttcatcatc gaccatcccg cccggctttt 1948440
acgccaaccc gaactcaaag cccgcgcctg gcaggtgttg aaacagttga aacgcgcctt 1948500
gcggcaaggc ggcggcagtt gaagcgcgcc gcacggggcg gtagaatcgc aactgcgtcc 1948560
caatatctga cagaaagcac aaaatgaccg atttccgcca agatttcctc aaattctccc 1948620
tcgcccaaaa tgttttgaaa ttcggcgaat ttaccaccaa ggcaggacgg cggtcgccct 1948680
atttcttcaa tgccggcctc tttaacgacg gcttgtccac gctgcaactg gcaaaatttt 1948740
acgcacaatc catcattgaa agcggcatcc gattcgatat gctgttcggt cccgcctaca 1948800
aaggcattat tttggcggcg gcaaccgcga tgatgctggc ggaaaaaggc gtgaacgtcc 1948860
cgtttgccta caaccgcaaa gaagccaaag accacggcga aggcggcgtg ttggtcggcg 1948920
cgccgcttaa agggcgcgtg ctgattatcg acgacgtgat ttccgccggc acatccgtac 1948980
gcgaatcgat caaactgatt gaagcggagg gtgcaacccc cgccggtgtc gccatcgcgc 1949040
tcgatcgcat ggaaaaaggc acgggtgaat tgagcgcggt tcaggaagtg gaaaaacaat 1949100
acggtctgcc cgtcgccccc atcgccagcc tgaacgattt gtttattctg ttgcaaaaca 1949160
accccgaatt cggacagttc ctcgaacccg tccgagccta ccgtcggcag tacggcgtag 1949220
aataaaaaca aagcatatgc cgtccgaacc gccttacgcc tcagacggca tcaaacctga 1949280
cacacacgag gaaataccat gcccgcctgt ttctgccccc actgcaaaac ccgtctctgg 1949340
gtcaaagaaa cccaactcaa tgtcgcccaa ggcttcgtcg tctgccaaaa atgcgaagga 1949400
ctgtttaaag ccaaagacca tctggcaagc acgaaagaac ccatattcaa cgatttgccc 1949460
gaggctgttt cggatgtcaa actcgttcac cgtatcggca cgcgcgccat cggcaagaaa 1949520
cagatttccc gtgacgaaat cgccggcatc ctcaacggcg gtacaaccca gcccgatatt 1949580
ccgcccgcaa ccgccgccac ccctgctgcc gcaccgcagg ttaccgtacc gcccgccgcg 1949640
cccgcccgtc aggatgggtt caactggacg attgcaaccc tgtttgccct tatcgtcctc 1949700
attatgcagc tttcctacct cgtcatccta tgagcgcgcc cgacctcttt gtcgcccact 1949760
tccgcgaagc cgtcccctac atccgccaaa tgcgcggcaa aacgctggtc gccggcatag 1949820
acgaccgcct gctcgaaggt gataccttaa acaagctcgc cgccgacatc gggctgttgt 1949880
cgcaactggg catcaggctc gtcctcatcc acggcgcgcg ccacttcctc gaccgccacg 1949940
ccgccgctca aggccgcacg ccgcattatt gccggggctt gcgcgttacc gacgaaacct 1950000
cgctcgaaca ggcgcagcag tttgccggca ccgtccgcag ccgttttgaa gccgcattgt 1950060
gcggcagcgt ttccgggttc gcgcgcgcgc cttccgtccc gctcgtatcg ggcaacttcc 1950120
tgaccgcccg tccgataggt gtgattgacg gaaccgatat ggaatacgcg ggcgttatcc 1950180
gcaaaaccga caccgccgcc ctccgtttcc aactcgacgc gggcaatatc gtctggctgc 1950240
cgccgctcgg acattcctac agcggcaaga ccttctatct cgatatgctt caaaccgccg 1950300
cctccgccgc cgtctcgctt caggccgaaa aactcgttta cctgaccctt tcagacggca 1950360
tttcccgccc cgacggcacg ctcgccgaaa ccctctcggc acaggaagcg caatcgctgg 1950420
cggaacacgc cggcggcgaa acgcgacggc tgatttcgtc cgccgttgcc gcgctcgaag 1950480
gcggcgtgca tcgcgtccaa atcctcaacg gagccgccga cggcagcctg ctgcaagaac 1950540
tcttcacccg caacggcatc ggcacgtcca ttgccaaaga agccttcgtc tccatccggc 1950600
aggcgcacag cggcgacatc ccgcacatcg ccgccctcat ccgcccgctg gaagaacagg 1950660
gcatcctgct gcaccgcagc cgcgaatacc tcgaaaacca catttccgaa ttttccatcc 1950720
tcgaacacga cggcaacctg tacggttgcg ccgccctgaa aacctttgcc gaagccgatt 1950780
gcggcgaaat cgcctgcctt gccgtctcgc cgcaggcaca ggacggcggc tacggcgaac 1950840
gcctgcttgc ccacattatc gataaggcgc gcggcatagg cataagcagg ctgttcgcac 1950900
tgtccacaaa taccggcgaa tggtttgccg aacgcggctt tcagacggca tcggaagacg 1950960
agttgcccga aacgcggcgc aaagactacc gcagcaacgg acggaactcg catattctgg 1951020
tacgtcgcct gcaccgctga ccgcaacgga aagccgccgc agaaatgccg tctgaacccc 1951080

gtttcagacg gcatttcccc gattatatag tggattaaat ttaaatcagg acaaggcgac 1951140
gaagccgcag acagtacaaa tagtacggca aggcgaggca acgctgtact ggtttaaatt 1951200
taatccacta taaagacctg cccaaccctc aaggaccccg atgaaatcct accccgaccc 1951260
ctaccgccat tttgaaaacc tcgattccgc cgaaacgcaa aacttcgctg ctgaagcgaa 1951320
tgccgaaacg cgcgcgcgtt ttttagaaaa cgacaaggcg cgcgcgcttt cagacggcat 1951380
tttggcgcag ttgcaggaca cgcggcagat tccgttttgt caggaacacc gcgcgcggat 1951440
gtaccatttc catcaggacg cggagtatcc gaagggcgtg taccgcgtgt gtaccgcggc 1951500
gacgtatcgt tccggctatc ccgagtggaa aatcctgttt tcggtggcgg atttcgacga 1951560
attgcttggc gacgatgtgt atttgggcgg cgtgtcgcac ttggtggaac agcccaaccg 1951620
cgcgttgtta acactgagca aattgggcag cgatacggcg tacacgctgg aagtggattt 1951680
ggaagcaggg gagttggtcg aaggcggttt tcactttccg gcaggcaaaa accatgtgtc 1951740
gtggcgcgat gaaaacagcg tgtgggtgtg tccggcttgg aacgaacgcc agttgaccca 1951800
atcgggctat ccgcgcgaag tatggctggt ggaacgcggc aagagtttcg aggaaagcct 1951860
gcctgtgtat caaatcggcg aagacggcat gatggtgaac gcgtggcgtt atctcgatcc 1951920
gcagggttcg ccgattgatt tgattgaagc gtcggacggt ttttacacca aaacctattt 1951980
gcgggtctca gccgaaggcg aggcgaaacc gttaaacctg cccaacgatt gcgacgtggt 1952040
cggctatctg gcggggcatc ttttgctgac gctgcgcaag gactggaacc gcgcgaacca 1952100
aagctatccg agcggcgcgc tggtggcggt gaagctgaat cggggcgaac tcggggcggc 1952160
gcagcttttg tttgcgcccg atgaaacgca ggcattggaa agcgtggaaa cgaccaagcg 1952220
ttttgtggtg gcgagcctgt tggagaacgt acaaggccgt ctgaaagcat ggcggtttgc 1952280
cgacggcaaa tggcaggaag tcgaattgcc gcgcctgcct tcgggcgcgt tggaaatgac 1952340
cgaccaacct tggggcggcg acgtggttta ccttgccgcc agcgatttca ccacgccgct 1952400
gacgctgttt gcgctggatt tgaacgtgat ggaactgacc gtcatgcgcc gccagccgca 1952460
gcagtttgat tcagacggca ttaacgtgca gcagttttgg acgacttcgg ctgacggcga 1952520
gcgcattcct tatttccacg tcggcaaaaa cgccgcgccc gacatgccga cgctggtcta 1952580
tgcctacggc ggtttcggca ttcccgaatt gccgcattat ctgggcagca ttggcaaata 1952640
ttggctggaa gagggcaatg cctttgtatt ggcgaacatc cgcggcggcg gcgagttcgg 1952700
cccgcgctgg catcaggcgg cgcagggaat cagcaaacat aaaagcgttg atgatttatt 1952760
ggcagtcgtg cgcgatttgt ccgaacgcgg tatcagttcg cccgaacaca tcggcttgca 1952820
gggcggcagc aacggcggac tgattactgc cgccgccttc gtgcgcgaac cgcaaagcat 1952880
cggcgcgctg gtgtgcgaag tgccgctgac cgacatgatc cgttatccgc tgctctccgc 1952940
cggttcaagc tggacagacg aatacggcaa tccgcaaaaa tacgaagtct gcaaacgccg 1953000
gttgggcgaa ttgtcgccgt atcacaatct ttcagacggc atcgattatc cgcccgcgct 1953060
cattaccacc agcctgtccg acgatcgcgt ccatcccgcc cacgcgctca agttctacgc 1953120
caaactgcgc gaaacctccg cgcaatcttg gctctactcg cctgacggcg gcggccatac 1953180
cggcaacggc acccaacgcg aatccgccga cgaactcgcc tgcgtcttgc tgtttttgaa 1953240
agagtttttg ggctaagggc gggggagcgg cactgccgcc gcgaatgaaa aaaggtcgtc 1953300
tgaaactgct ttttcagacg accttttttta atggttgttt caaatcaaaa tatctatgcc 1953360
gccggcccca tcagcacttc ttcacatccg aaggcaaaaa tccgtaatgc cgtctgaacg 1953420
cttcgttgaa ccgtcccgcg tggcggtagc cgcaaaagtg catggcggct tggacggtgc 1953480
tgccggattc gatgagggcg agcgcgtgtt ccagccgcag gcggcgcagg catccggcga 1953540
cggtttcgcc ggtttgcgct ttgaaatagc gtttcaggta gcattcgttc agtccgacgc 1953600
ggcgggcgat ttcggcgatg gtcagcggac gggcgaattc gtgttgcagg atgtcggcgg 1953660
cttcgtctat gcgccgacgg cggtaaccgt tgtcgtggcg gcggaaggtg aagcgcaata 1953720
atcgggcgga gagttccagc gcggcggctt cgtcggcaag caggccgaag ccgtccgatt 1953780
cgaacgggcg ttgcagcagt gggcaggccg ccgccgtcag tgccgctgcg ttttgcgcca 1953840
gccgttgcag ggcgaatcgg cctattgttt gcggcgaaaa caggcgttcg tccagcaagc 1953900
cttcgtcgtg ccagcggcgc agtttttcca gcgaaaaatc caaatgcagc gcgcacatgc 1953960
cgctgttgtc gggcagcagg gtttcggata cgtccgccaa atcgccgcgt accagtcaga 1954020
tttcgccggc agatgggcgg tattccctgc cgcccatttg taaccggttc tgccccgaca 1954080
ccatgacgaa caaggcgcag ttgtggctga aattgtggat ttcggtggga aacgcgcccg 1954140
ttccgccgcc gcgcatccgc gacaaggtga tgcccgaatc gaagcggttg atgcacattt 1954200
ccagatgcaa accgggctgt tttgcctgcg caatgagcgc gctgtcggaa cagccgtcca 1954260
acgcccagcc ggatttatcg gagcggacat aggtttggta ctggcggtag atggcggcgg 1954320
tgttcatgat tggataggaa cgagttgtct aacaaatgaa ttaaatagga attattacca 1954380
ataatcaagc gcagggattg gttgaaacgg aaaaggtcgt ctgaaagggt gtttcagacg 1954440
accttttccg tatcgggaat ttgttttgcc gtatcgggaa ttttgcgttt tgcggcgtgg 1954500
tttctgcagg ttgtttgctt aataataaac attcttattc gtatgcaaag gaaccgcaca 1954560
ccgtgaaacc gcgtttttat tgggcagcct gcgccgtcct gctgaccgcc tgttcgcccg 1954620
aacctgccgc cgaaaaaact gtatccgccg catccgcatc tgccgccacg ctgaccgtgc 1954680
cgaccgcgcg gggcgatgcc gttgtgccga agaatcccga acgcgtcgcc gtgtacgact 1954740

gggcggcgtt ggatacgctg accgaattgg gcgtgaatgt gggcgcaacc accgcgccgg 1954800
tgcgcgtgga ttatttgcag cctgcatttg acaaggcggc aacggtgggg acgctgttcg 1954860
agcccgatta cgaagccctg caccgctaca atcctcagct tgtcattacc ggcgggccgg 1954920
gcgcggaagc gtatgaacag ttagcgaaaa acgcgaccac catagatctg acggtggaca 1954980
acggcaatat ccgcaccagc ggcgaaaagc agatggagac cttggcgcgg attttcggca 1955040
aggaagcgcg cgcggcggaa ttgaaggcgc agattgacgc gctgttcgcc caaacgcgcg 1955100
aagccgccaa aggcaaagga cgcgggctgg tgctgtcggt tacgggcaac aaggtgtccg 1955160
ccttcggcac gcagtcgcgg ttggcaagtt ggatacacgg cgacatcggc ctaccgcctg 1955220
tagacgaatc tttacgcaac gaggggcacg ggcagcctgt ttccttcgaa tacatcaaag 1955280
agaaaaaccc cgattggatt ttcatcatcg accgtaccgc cgccatcggg caggaagggc 1955340
cggcggctgt cgaagtattg gataacgcgc tggtacgcgg cacgaacgct tggaagcgca 1955400
agcaaatcat cgtcatgcct gccgcgaact acattgtcgc gggcggcgcg cggcagttga 1955460
ttcaggcggc ggagcagttg aaggcggcgt ttaaaaaggc agaacccgtt gcggcgggga 1955520
aaaagtaggg agtcgtctga aaacggagct tccgaaggaa gcggggggtt tctgcgaagc 1955580
taaagtgcgg tttcaacgaa ttgaaaagca gcctgtatgt tgaaaatacc gctcaagcaa 1955640
acctacggtt tgccgccctc tccctagccc tctcccacag ggagagggga ttgggttgca 1955700
ggctgccttt aaggtttagg caaattttta acttcgttga agctgcgatt tcagaagctc 1955760
cgttttagct tcgcagaaac tccgcttcct tcgaaagctc cgttttcaga cgaccttttg 1955820
gagtaccgca ggcacacgca tcgaacggct gaatcaaaga ttcagaccga tggcagtccg 1955880
cacccgagtt tatgcggcaa acagcgaggc tacggcaacc cgccccctct ccctgtggga 1955940
gagggttagg gagagggcgg taagccgcag gcttacatca aagccgataa cgcttccgtt 1956000
acaactccgc ccactgaaag cagcctgcaa cgaagccaaa acgacaaacc gcatcgtaaa 1956060
ccacccaacc cataggagaa ccccatgcaa aacgaaacca tcaacctgaa acagcacctt 1956120
gccgccatca aagaatactg gcagcccgaa atcatcaacc gccacgggtt ccaattccac 1956180
ttggtcaaac ttttgggcga ttacggctgg catacgcacg gatacagcga caaagtgctg 1956240
tttgccgtgg agggcgacat ggcggtggac ttcgccgacg gcggcagcat gacgatacgc 1956300
gagggcgaga tggcggtcgt gccgaagtcg gtgtcgcacc gcccgcgttc ggaaaacggc 1956360
tgctcgttgg tgctgattga gttgtccgac ccgtccgagg ccgtctgaaa acgaagtttc 1956420
cgaaggaagc tgagtttctg cgaagctaaa agcagcctgc accttcaatc aatatgccga 1956480
aaatacaacc ccaccgcaca ccaacacaca aaggaaatcc catgacacgc ttcaaatatt 1956540
ccctgctgtt tgccgccctg ttgcccgtgt acgcgcaggc cgatgtttct gtttcagacg 1956600
accccaaacc gcaggaaagc actgaattgc cgaccatcac cgttaccgcc gaccgcaccg 1956660
cgagttccaa cgacggctac actgtttccg gcacgcacac cccgctcggg ctgcccatga 1956720
ccctgcgcga aatcccgcag agcgtcagcg tcatcacatc gcaacaaatg cgcgaccaaa 1956780
acatcaaaac gctcgaccgc gccctgttgc aggcgaccgg caccagccgc cagatttacg 1956840
gctccgaccg cgcgggctac aactacctgt tcgcgcgcgg cagccgcatc gccaactacc 1956900
aaatcaacgg catccccgtt gccgacgcgc tggccgatac gggcaatgcc aacaccgccg 1956960
cctatgagcg cgtagaagtc gtgcgcggcg tggcggggct gctggacggc acgggcgagc 1957020
cttccgccac cgtcaatctg gtgcgcaaac gcctgacccg caagccattg tttgaagtcc 1957080
gcgccgaagc gggcaaccgc aaacatttcg ggctggacgc ggacgtatcg ggcagcctga 1957140
acaccgaagg cacgctgcgc ggccgcctgg tttccacctt cggacgcggc gactcgtggc 1957200
ggcggcgcga acgcagccgc gatgccgaac tctacggcat tttggaatac gacatcgcac 1957260
cgcaaacccg cgtccacgca ggcatggact accagcaggc gaaagaaacc gccgacgcgc 1957320
cgctcagcta cgccgtgtac gacagccaag gttatgccac cgccttcggc ccgaaagaca 1957380
accccgccac aaattgggcg aacagccgcc accgtgcgct caacctgttc gccggcatcg 1957440
aacaccgctt caaccaagac tggaaactca aagccgaata cgactacacc cgcagccgct 1957500
tccgccagcc ctacggcgta gcaggcgtgc tttccatcga ccacaacacc gccgccaccg 1957560
acctgattcc cggttattgg cacgccgacc cgcgcaccca cagcgccagc gtgtcattga 1957620
tcggcaaata ccgcctgttc ggccgcgaac acgatttaat cgcgggtatc aacggttaca 1957680
aatacgccag caacaaatac ggcgaacgca gcatcatccc caacgccatt cccaacgcct 1957740
acgaattttc ccgcacgggt gcctacccgc agcctgcatc gtttgcccaa accatcccgc 1957800
aatacggcac caggcggcaa atcggcggct atctcgccac ccgtttccgc gccgccgaca 1957860
acctttcgct gattttgggc ggacgataca cccgttaccg caccggcagc tacgacagcc 1957920
gcacacaagg catgacctat gtgtccgcca accgtttcac cccctacaca ggcatcgtgt 1957980
tcgacctgac cggcaacctg tctctttacg gctcgtacag cagcctgttc gtcccgcaat 1958040
cgcaaaaaga cgaacacggc agctacctga aacccgtaac cggcaacaat ctggaagccg 1958100
gcatcaaagg cgaatggctt gaaggccgtc tgaacgcatc cgccgccgtg taccgcgccc 1958160
gtaaaaacaa cctcgccacc gcagcaggac gcgacccgag cggcaacacc tactaccgcg 1958220
ccgccaacca agccaaaacc cacggctggg aaatcgaagt cggcggccgc atcacgcccg 1958280
aatggcagat acaggcaggt tacagccaaa gcaaaacccg cgaccaagac ggcagccgcc 1958340
tgaaccccga cagcgtaccc gaacgcagct tcaaactctt cactgcctac cactttgccc 1958400

ccgaagcccc cagcggctgg accatcggcg caggcgtgcg ctggcagagc gaaacccaca 1958460
ccgaccctgc cacgctccgc atccccaacc ccgccgccaa agcccgcgcc gccgacaaca 1958520
gccgccaaaa agcctacgcc gtcgccgaca tcatggcgcg ttaccgcttc aatccgcgcg 1958580
ccgaactgtc gctgaacgtg gacaatctgt tcaacaaaca ctaccgcacc cagcccgacc 1958640
gccacagcta cggcgcactg cggacagtga acgcggcgtt tacctatcgg tttaaataag 1958700
gtcgtctgaa aacggagttt ctgcgaagct atagtggatt aacaaaaacc agtacggtgt 1958760
tgcctcgcct tagctcaaag agaacgattc tctaaggtgc tcaagcacca agtgaatcgg 1958820
ttccgtacta tttgtactgt ctgcggcttc gtcgccttgt cctgattttt gttaatccac 1958880
tataaaagca gcctgcacat tgaaaatgcc gcccaagcaa actttcagtt tgcccgcctc 1958940
gtcctagccc tctcccacgg gagaggggat tggggtgcag gctgccttta aggttcaggc 1959000
aaattttaac ttcgttgata ccgcgcttta gcttcgcaga agctgcactt tcagacgacc 1959060
ttttggaaca ccacaggtac acgcatttaa ggaatgccgt ctgaaatgcc tgcctcaata 1959120
acgcatcatg ttgccgtcaa tctcggccgc ccatgcatcg atgccgccct gaaggttgta 1959180
caggttttca aaacccgcgt ccgccaaata catcgccgta tgcaggctgc ggataccgtg 1959240
gtggcaatac accacaagcg gcacatcatc cggcagctcg ttctgccgca gcggaatcag 1959300
attcatcggg atatgcagcg catttggcag cgaacaaacc gccgtttctt catccgtacg 1959360
cacgtccaac aaacaaaaca tccgcccttc gtccatccac gctttcaatt ccgcgggccc 1959420
aagttgcaca atatccatcg cacccccaa aaaaaaccaa gcaaaatgcc gtctgaagcc 1959480
ccaaacccgc tttcagacgg catgacctgt caacatctta aaaatcgaaa ccgccaaacg 1959540
gatcggcatc cttatcatcc aaatgcgcca ccaaggtatc gaacagcacc ttctcttcaa 1959600
acacatcgcc cctgcgcgta atcaaaagcg cgcgttgaac aggcttgcga cctacgataa 1959660
ccaccatgcg tccgccatct ttcaactgtt ctttcaacac ttcaggcaca aggtttaccg 1959720
caccgccgac ataaaccgca tcaaacggcg cacctgcgga aagttcggtc aacccgttgt 1959780
tttgcacata atcgatattg tccaaaccca agccgtccaa caccgctttg gcgcggtttt 1959840
gctgttcgac atcgatgtcg tccgacacca cacgaccagc caattttgcc aacagcgcgg 1959900
tcgcatagcc cgaacccgtg ccgatttcca aaaccgtatc gtttttcgtc agcttcaagc 1959960
cctgcgccag ccgcgccacg actttcggct cgagcatctt atgaccgttg gcaagcggca 1960020
gcgccatatc cgcatacgcc aaaccctgca agtcctcatc gacaaaaagc tcgcgcggaa 1960080
tctccgccaa agcgtccaac acatcaaaat ccaatacatc ccacggacgg atttgctgtt 1960140
cgaccatatt gaaccgcgct ttttcaaaat ccatttagtg ctccgtcaaa taattgttcc 1960200
aacaatggca ggcattataa aaccacactc ccgccgcgcc aacttcggca cgcgccgaca 1960260
gcccgttttg tcagtctcaa accgcctgac gcgaagcctc aaaccgcttc tccaaaatct 1960320
tcgccagttc gcccaaatac aaagcatccg tctcatcaaa ctgcgccaaa tgttcgctgt 1960380
ccgcgtccaa cacgccgata cagcggccgt ctgaaaacag cggcacgaca atctccgaac 1960440
gtgacaaaga cgaacaggca atatggtcgg gatgcgcgtt gacatcctta acaaccaccg 1960500
tttcaccctt cgcccaagcc tgaccgcaca ccccgcgacc gaacggaatc cgcgtacacg 1960560
ccaaaggccc ctgaaacggt gccaaaacca attcgtccga acgcgtatcg accaaataaa 1960620
aacccaccca aaaccaaccg aacgcctcct tcaaaaccgc cgccgtgttc gccaaattcg 1960680
ccacccaatc cgcctcgtca gccaccacag actcaatctg cggcaacacc tcccgataaa 1960740
gcgcggcctt gtccgaagcc gaaaaatgaa gcgcgtgcat cacatctcct atagttgcat 1960800
acatatcagg cggccattat aaaacagcct gcccgaaaca acattccaaa ccgcccggcc 1960860
ggccgcttca agttgcgaac ccgccgcata tccactaaac ttcacgttgc accgcgccac 1960920
acgcggcaga caaaaaaaca cgacacggag caaaaaagat gtatcgccaa atcggaatgt 1960980
gggatcaaaa atgggtcatc ggcaactgga aaatgaacgg ccggctccaa aacaacaacg 1961040
cactgatgca ccgcttccgc atccacccca ccgccgaacg cgtcctcatc ggactcgccg 1961100
ccccgaccgt ttacctgctg caactgcaca acgccatgca aatcgtttta aacaaccgca 1961160
tcctcacctg cgcccaagac gtgagccgct tccccaataa cggcgcgtac accggcgaag 1961220
tgtccgccga aatgctcgcc gacaccggca cagacatcgt cctcatcgga cactccgaac 1961280
gcagccttta tttcggcgaa aaaaacgaaa tccaacgccg caaaatggaa aacgtcctca 1961340
acgtcggact catcccgtta ttgtgcgtcg gcgaaagcct cgaagagcgc gaagccggca 1961400
aagaacacga agtcatcgcc catcagcttt ccatcctgca agggctggat accaaaaaca 1961460
tcgccgtcgc ctacgaaccc gtctgggcga tcggcaccgg caaagtcgcc accgtcgaac 1961520
agattgccga tatgcacgca ttcatctaca aagaaatctt gtctttgtgc ggaagcgatg 1961580
ttaaaatccg cgtcctttac ggcggaagtg tgaaagcgga caacgcggcc gacatcttcg 1961640
cagtacctta tgtggacggc gcactcgtcg gcggcgcgtc attgtcgtac gactccttta 1961700
ccgccatcat cagtgccgca caaaatgcgt agaaaaatat ggaagccttc aaaaccctaa 1961760
tttggattgt taatataatt tccgctttgg ccgtcatcgt gttagtattg ctccaacacg 1961820
gcaaaggcgc ggatgccggc gcgactttcg gatcgggaag cggcagcgcg caaggcgtat 1961880
tcggctctgc cggcaacgct aacttcctca gccgctcgac cgccgttgca gcaacatttt 1961940
tctttgcaac ctgcatggct atggtgtata ttcacaccca cacgacaaaa cacggtttgg 1962000
acttcagcaa cgtacaacaa actcagcaag cacccaaacc cgtaagcaat accgaacctt 1962060

ctgcccctgt tcctcagcag cagaaataac agtttttcaa atgccgacat ggtgaaattg 1962120
gtagacacgc tatcttgagg gggtagtggc cgtaggctgt gcgagttcaa atctcgctgt 1962180
cggcaccaac acacaaaaac gcctgaaaat ttcaggcgtt ttttgttcaa tcccatccaa 1962240
cgctgtcaac caattccaat caatacaagg atttcaggct gattgttatc ctgccgtccc 1962300
ccttcctgac agtgcaatcc cgtccaatcc gccctaattg aagtaaccta aaatttacgg 1962360
tatcttttgc ggtatctgaa aaatacctcg aaaaaatacc gcaaaaataa agctgaacga 1962420
ccgccaaatc aggaatgcca agcggaaaag agcttgcggg gaatactgcc aagacgtagg 1962480
gaacaagggg gaaaccgtcc aagatgcagg gcggtttttt ttgggttttt ggaaaaaacc 1962540
tatactagga agcgataccc ttagttgtta ccttgttacc ggggaaaagt tagataaata 1962600
agcatatgaa atatagtgaa ttaaatttaa atcaggacaa ggcggcgagc cgcagacagt 1962660
acaaatagta cggcaaggcg agccaacgct gtaccggttt aaatttaatt cactataaaa 1962720
taagaaaaag ataaaaaatt ggtaacaaat gcggtaacaa atggtaacga atcggtaaca 1962780
acttttgggg ttttccggtt tttcaccgtc ttggcagtgg gagcgtagcg gaatgaaaag 1962840
ccaaaacgca cggaaccgcg cctattttga gcaggaatgg cggttaaacc gcttggttat 1962900
atacggggaa taggaagaca gcgaaacgcg ctaaaaacgc aatttgagcc attaaaagcg 1962960
attgattaaa aaaataatca ggttagccgc cagtgtttca ggcggcataa acggagaaat 1963020
tgcggggcat aaaaaaggca gcttgccgtg ttgtctgtct ctggtataat tccaagtatc 1963080
actaatcaac ggctacacaa tgcggatatt caaaaaccaa tggatagtga aatttgccaa 1963140
gaagcacaaa atcaacgatt ccgagctgct ggaagcggta gagcgggcgg ataacgggct 1963200
gatagacgca gatttaggcg gcggtgtgat taagcagcgc atagcaaggc aaggacaagg 1963260
cagaagcggc ggttatcgca gtctgatact gttcaaacag gcagacaagg catttttgt 1963320
ttacgccttt gccaagaacg acagggaaaa catttcggat aaagaacttg acgtttaccg 1963380
aaaagccgcc gcatattatc tgaaatacac gcgggcagag ctggcggctt tgaaagaaga 1963440
cggcattatc acggagatag aatcatgaaa tacaaaaacg aggcattagc cgccattcat 1963500
gaaatgatgg aaggggctta caacatcggc gcaatcgaca aaaagaccat gcgcgacttt 1963560
gacaagtcct gcctgaccga aatcaaaccg ttgagcggcg gagacatcaa ggcaatcagg 1963620
gagaaggagg cactatcgca agccgctttc gccatctatc tcaacgtggg aaaaaatcac 1963680
gtttcggctt gggagcgggg cgttaaaaag ccgagcggcg cggcgttgaa gctgctgacc 1963740
atcgtcaaaa acaagggcat cgaagccatt gcgtagccga cttggcaaac ggcaaaatca 1963800
gcaagttcac aatagacgcg ctgctgaata tgcctgccaa gacaggcaag accgccgaac 1963860
tgaatatcag ggcgtagccg cataaatgcc cgaccgcatc aaaccaagcc gaaacggcgg 1963920
cggtgcagac gacatagccc gacagcaagg cacggcgcag acgggcgcga aacccgaaac 1963980
atcaccgacc gcgaggtacg gggatttttt gcgcccgttg caggggggga ttggatttaa 1964040
gcggcgcggg cttgaaggca aaacgggtgg ggcacagaac tgtttaaatg cagtctgaat 1964100
ctcaaacgat ttcagacggc attttgaaac aatggctcaa attctcgatc cccttccctt 1964160
aacgccgacg tttttatta acgcgcccct tatttctgac actttgctca taaaccggca 1964220
taacggtcgg caacaaccgt tttagatttt ctatacgggc attgtttgtc ggatgagtag 1964280
aggtaatagc ataaataaag ccgttttggt cgttttcctg attcattttt tcccaaaccc 1964340
tgacagcggc cgccggatga tagcctgcct gcgccatcaa catcattccc ccctcatcgg 1964400
cttcttcttc caagctgcgg ctataaggca aggtaagacc gtacgtcccc aaaatatcca 1964460
tacccaatcc gaccaattcc ggattagtat ccggtttttt gtctaatata atctgcgtgc 1964520
ctatctgcgc cgccgtattg gtcaagattt gctgcccgac cttattttta ccgtgttcat 1964580
gcagggcgtg cgtcatttca tgccccataa tggcggcaat ttcgtcatcg gtcagcttga 1964640
gtttgtcgac tatccccgta taaaacgcca tttttccacc gggcattgcc cacgcgttca 1964700
gctcatcgtt tttgaaaacc gtcattttcc agtcaaactt atggctggta ttatttgccg 1964760
catcggcata aggcagcata cgtcgaaata ctgcctgcac cctgcgggct gttctggatg 1964820
tggtatcgac attgccggca gacttgttta actcaaccgt tttcatataa tctttggcag 1964880
ccgcagcgtt cattgtggcg gaatcatgac cgtaaacatc agcaacgacc gcacaagccc 1964940
ccaataccga gattactgcc gacaggcaga gtatccgttt aaaggaagga aggaaggaaa 1965000
tttcatattt aggtttactc cttaaaaaat taaatttcaa aaaaatgccg tctgaatcca 1965060
aaacggattt cggacggcat cttaacattg tttaatgttt ttaaaaagat ttacaccacg 1965120
atgttctcca gtctgcccgg tacggcgatg attttcttgg caggcttgcc ttctatgaat 1965180
ttcaccgcgc cttcagcggc gtattcggca gcttcttcag ccggtttgtc gaaatcacgc 1965240
ataaattgcc aataattctc caacttttt acggctgctg ctgccttttg cggcaatatt 1965300
gcgctgaact tcaactgttt tcaaaatggc agaagaataa atatcccttg tgaattcagt 1965360
atcatgattt gaaatcaaaa taccttggga gttggcgcca atttattgat tttttgtaaa 1965420
gtccgcgacc aatgaattcg atcgtatttt ggtcgcgcag aatttgcaac tgttggcgga 1965480
ttttgtctct gatatggttg ttttggggaa attggatgga tagtttgttt tcaaattcat 1965540
acatttgcga caatgtgaat tcttcgggga gttggtcgat acatttcata acagccagaa 1965600
gccagccttt gcgctccgca ttttggttgc gtaaaaacaa attggattgc catttttca 1965660
gaacggtttc gggttcgata atgcgggaat tgtctattaa gaatattttg ccgctttcag 1965720

gcaaaggggc gagattgata gaacacataa tgtggttcgg ccggttttta atgcctttat 1965780
ttctgggaat aatcatatcc ggcgtgatga aatgtttggg tacaagcacc aattgccgta 1965840
tggagtaatc cgcttttttta tatgcaagaa agaaaaagtt ggggttggta tctgaccgga 1965900
tgcgctccaa catggtgtga tatgcaccgt caggcacgct gttgcctatg gttttttgat 1965960
ttttactctt taattcatat tgctcgtggc aatttgggca aaagaggtct gcaacaggtt 1966020
tgttattggc aaatctctgc atcggcttgc ttccgcaaca ggggcagtag ccgttttttt 1966080
ccaaccaagc ctcgctcatt acacggattt tatgggttgc tttattttgt tgctttccca 1966140
attcggtatc gaaaaataaa ttcatgtttt ggattttgag atttcagtta ttcggggttc 1966200
gtcatgcaga caacacaatc caccttaaaa aggccgtctg aaaccctgtt tccaagtttc 1966260
agacggcctt tatccgtgtg gctaaacctt aaaagcggtt agacgacgat gttcaccagt 1966320
ctgcccggta cgacgatgat tttcttcgcc ggtttgcctt ccatgaattt caccgcgcct 1966380
tcggtggcga gtgcggcggc ttcgaggtcg gctttggatg cgtcggcggc aacagtgatt 1966440
ttgccgcgca gtttgccgtt gacttgaacc atcacttcga tttcggattt gaccaaggcg 1966500
gcttcgtcga ctgtcggcca gcctgcttcc cacagtttcg cgccgttcaa ttcgctccac 1966560
agggtttcgc agatgtgcgg cacgatgggc cacaacaggc gtacggcggt ttccaatact 1966620
tcttgggcga cggcgcgtcc ttgttcgccg ccggtgtcgg ttttgtcgta ttggttgagc 1966680
aattccatca cggcggcgat ggcggtgttg aactgctggc ggcggccgta gtcgtcgctg 1966740
actttggcag tggtcgcgtg cagtttgtgg cgcaggtctt tgagttcttt agacaaaccg 1966800
tcttggctgc ctgcgaacgc tttgaccgct tcgccttgct tcaagtattc gtaaacggta 1966860
cgccacaggc ggcgcaggaa gcggtgtgcg ccttcgacgc cgctgtcgct ccattcgagg 1966920
gactgttcgg gcggtgcggc gaacatcata aacaggcggg cggtgtccgc gccgtaggcg 1966980
ttaatcagtt cttgcggatc gacgccgttg tttttggact tggacatttt ttccgtgccg 1967040
ctgatgacga cgggcagccc gtcggctttg aggacggcgg aaatggggcg gcctttgtcg 1967100
tcgaacgtca gctcgacatc ggcggggttg atccaatctt tgccgccttt gtcgttttcg 1967160
cggtagtagg tttcgcaaac gaccatgcct tgcgtcagca ggcgttcaaa cggttcgtca 1967220
acattgacta gaccttcgtc gcgcatcagt ttggtgaaga aacgcgcgta caagaggtgc 1967280
aaaatcgcgt gttcgatgcc gccgatgtat tggtcgaccg cgccccagta tttcgcggcg 1967340
gcaggatcga ccatgccgtc tgaaaatttt ggcgacatgt agcggaagaa ataccagctc 1967400
gattccatga aggtgtccat ggtgtcggtt tcgcgtttcg ccgcgccgcc gcagcatggg 1967460
caggcagttt cgtaaaactc gggcattttt gccagcggcg aacccatgcc gtcgggtacg 1967520
acgttttcag gcaaaacgac cggcaattgg tcggcaggga cgggtacgtc gccgcattgt 1967580
tcgcaatgga cgatgggaat cgggcagccc cagtagcgtt ggcgcgaaat gccccagtcg 1967640
cgcaggcggt attgggtttt cggttcgccc gcgccttggc tttgcagctt ggcggcgacg 1967700
gcgtcgaatg ccgtctgaaa atccaagccg tccaagtcgc cgctgttgac caatacgccg 1967760
ttttctttgt cgccgtacca ttcttgccat tggttttcgt caaatgcgtt gtcgccgacg 1967820
gcaatgactt gttttttcgg cagattgtat ttggtggcga actcaaaatc gcgttcgtcg 1967880
tgcgccggaa ccgccatcac cgcgccgtcg ccgtagcccc acaatacata gttggcaatc 1967940
cacacttcca gcttgtcgcc gttgagcggg ttgacgacgt agcggccggt cggcacgcct 1968000
tttttctcca tcgtcgccat atcggcttcg gcaaccgaac cggctttgca ttcggcaata 1968060
aatgcctgca attcgggttt gtcggcggct gcggcggctg ccagcggatg ctcggcggca 1968120
acggcaacat aagtcgcacc catcagcgtg tcggggcggg tggtataaac ttgcaggaat 1968180
ttcgcgtaat cgccttccaa gccttgtttg ctgtcgtctg aaacggcgaa gcgcacggtc 1968240
ataccgcgcg atttgccgat ccagttgcgc tgcatggttt tgacttgttc cggccagtgt 1968300
tccagcttgt ccaagtcgtt gagcagctct tcggcgtaat ccgtgatttt gaagtaatac 1968360
atcgggattt cgcgtttttc gatcaatgcg ccggaacgcc agccgcgtcc gtcgatgact 1968420
tgctcgttgg caaggacggt ttggtcgaca gggtcccagt ttaccgtgcc gtttttgcga 1968480
taaacgatgc ctttttcaaa cagcttggta aacagccatt gttcccagcg gtagtattcg 1968540
ggtttgcagg ttgcggtttc gcgcgcccag tcaatcgcaa aacctaggct tttgagctgg 1968600
gttttcatgt attcgatgtt atcgtacgtc caagcggcag gggcgacgtt gtttttcatc 1968660
gccgcgtttt ccgccggcat gccgaacgcg tcccaaccca taggctgcat gacgttgaag 1968720
ccgtttaaaa gtttgaagcg gctcaataca tcgccgatgg tgtagttgcg cacatgcccc 1968780
atgtgcagct tgccgctggg atagggggaac atggagaggc aataatattt gggtttggaa 1968840
gcgtcttcgg agacgttgaa aatacgggcg tcgtcccatt ttttctgcgc cgcaggctca 1968900
atggcggcgg gccggtattg ttcttgcata gtcattctgt tttcgcttaa aaacgttgga 1968960
aaaataaagt cggcatcaat tataacaggt tgccggaagc ggcgaatcgg cagattgccg 1969020
gcaggatgcg taaattcgca cgcgcattat tccgtatgcc gtacaaatac accgcgttta 1969080
ttgatacgca cgttttttat gctaatatta caaaccaaaa tcaaatgttt aaaactctcc 1969140
tgatgcggct cttccgaaca aaaggcagac gggcatcggg taaaagagga ttctgcatat 1969200
gaaaatcaaa caaatcgtca aaccgggctt ggcagtattg gcggcgggcg ttctgtctgc 1969260
ctgcgcaacc aaaagcaacg tcaaagccga cggcacgacc gacaatccgg ttttcccgaa 1969320
accctattcc gtaacgctcg acaacaagcg cggcacattc ccgacttatg acgaactgga 1969380

tcagatgcgc cccggcctga ccaaagacga catctacaaa atcctgggcc gcccgcatta 1969440
cgacgaaagt atgtacggcg tgcgcgaatg ggattacctg ttccacttcc ataccccggg 1969500
cgtaggtatc gaccctgaaa acacttccgg cgtagaagat gttactacct gccaatacaa 1969560
agtgattttc gataaagaca aatttgcccg cagcttctac tggaaccccg tcttcccgaa 1969620
agatgccgcc tgtccgccgc ccgcacccaa agccgagccg caagtcatca tccgcgaaat 1969680
cgtgccggca aaaccgaaac gtatccgcca ataatccgac atgccgttcc gcctgttttt 1969740
agggatatta tgcggcctgt caatggttgc cccgtatat gcacaggggc agccggatac 1969800
ggtcggcgac tttatccaaa agaaaaaagt catcgtcgat acatccaaag cggaactctg 1969860
tttcgctgac gaccgtcagt gccaccccgt cctcatcggt gttgccacgc ccaaggggac 1969920
gttcgggctg acgctgaaca gtaccgacaa gcccggatac ggcggcgaag tcatcggttt 1969980
caagcaggag ggtgattttc ttttcgccct gcaccgcgtt tggaatcaga taccgtcgga 1970040
aaggcggaac gaacgcatcg cctccccgtc cgtgtccgac aggattatga ccaacggctg 1970100
catcaacgtc agcgatgcgg tgtacgaaaa actgcgtcat tattttgtgt tggaagtgat 1970160
ttgaaacaga cggataccgc acgcgccggt atctgttttc acattgcccc gatgcctgaa 1970220
acagactgtc cgccacgtca tgccgtctga aaccggcgca gatgccgcca agccttcaga 1970280
cggcattgcc tgcccgctcc gaccgaacaa caaccatctt tgggagaacc ttatgcccga 1970340
acaaaaaccgc atcctctgcc gcgaactgag cttgctggca ttcaaccgcc gcgtgttggc 1970400
gcaggcggaa gaccaaaacg tccccctttt ggaacgcctg cgcttcctgt gcatcgtttc 1970460
atccaacctc gacgagtttt tcgaagtccg tatggcgtgg ctgaagcgcg aacacaaacg 1970520
ctgcccgcag cgcaggctgg acaacggcaa aatgccgtct gaaaccatcg ccgacgttac 1970580
cgaagcggcg cgctccctga tacggcacca gtacgacctg ttcaacaacg tccttcagcc 1970640
cgagctggca caagaaggca tccattttta ccgccgccga aattggacag acacacagaa 1970700
aaaatggatt gaagactatt tcgaccgcga attgctgccg atcctgaccc ccatcggact 1970760
cgacccttcc cacccccttcc cgcgcccgct gaacaaatcg ctcaacttcg ccgtcgaact 1970820
cgacggcaca gacgcgttcg gcaggccttc ggggatggcg attgtgcagg caccacgcat 1970880
cctgccgcgc gttgttcccc tgccgtccga actgtgtggc ggcggacacg gcttcgtctt 1970940
cctctcctcc atcctgcacg cccacgtcgg aaaactcttc ccgggcatga acgtcaaagg 1971000
ctgccaccag ttccgcctga cgcgcgacag cgacttgacc gttgacgaag aagacctgca 1971060
aaacctccgc gccgccattc aaaacgagtt gcacgaccgc gaatacggcg acggcgtgcg 1971120
gctcgaagtc gccgacacct gtcccgccta catccgcgac tttctgctcg cgcaattcaa 1971180
actgaccgcc gccgaactct atcaggtcaa aggcccggtc aacctcgtgc gcctcaacgc 1971240
cgtccccgac ctagtcaacc gccccgattt gaaatttccc acacacacgc cgggcagact 1971300
gaaagccttg ggcaaaaccg cgtccatatt cgatttggtg cgccaatcgc ccatcctgct 1971360
gcaccacccc taccaatcgt tcgaccccgt tgtcgaaatg atgcgcgaag ccgccgccga 1971420
ccccgccgtg cttgccgtca aaatgacgat ttaccgcacc ggcacgcgtt ccgaactcgt 1971480
ccgcgccctg atgaaggcgg cactcgccgg caaacaagta accgtcgtcg tcgaactgat 1971540
ggcgcgtttt gacgaagcca acaacgtcaa ctgggcgaag cagctcgaag aggcgggcgc 1971600
gcacgtcgtg tacggcgtgt tcggctacaa agtccacgcc aaaatggcac tggtcatccg 1971660
ccgcgaagac ggcgtgctca aacgttacgc ccatctcggc acgggcaact accaccaagg 1971720
cacatcgcgc atctacaccg acttcggcct cattaccgcc gacgaacaaa tcaccgccga 1971780
tgtgaacata ttgtttatgg aaatcacagg tttgggcaaa cccgggcggc tgaacaaact 1971840
ctaccaaagt ccgtttaccc tgcacaaaat ggttatcgac cgcatcgcac gcgaaaccga 1971900
acacgcaaaa gccggcaaac cggcgcggat taccgccaag atgaattcgc tcatcgaacc 1971960
gaccgtcatc gaagccctgt atcgggcaag cgcggcaggc gtacaaatcg atttgattgt 1972020
gcgcggtatg tgcaccttgc gcccgggtgt aaaaggcttg tccgaaaaca tccgcgtccg 1972080
ctccatcgtc ggcaggcagc tcgaacacgc gcgcgtgtat tacttccata acaacggcac 1972140
ggacgatacc tttatctcca gcgcggattg gatggggcgc aacttcttcc gccgcatcga 1972200
aaccgccacg ccgattaccg cgcccgaact caaaaagcgc gttatacatg aaggactgac 1972260
catggcactg gacgacaaca cccacgcgtg gctgatgcag cccgacggcg gctatatccg 1972320
cgccgcacct gccgagggcg aatccgaagc cgacctgcaa aacgatttgt ggacactgct 1972380
cggaggctga cccgcaccgc cccaatcaaa aaccatgccg tctgaaacct ttccgtttca 1972440
gacggcatgg ttttacagca atctaaacag ggcggaccgg agtcaaaaac acaccttcgc 1972500
cattcctgca caagcacttc ccctatacgc tcccaacccc aagccgccgc attccagacg 1972560
gcattatagt ggattaaatt ttaggggctg tactagatta gcagatatgt taccctcgaa 1972620
atatgaagat aacgcactgc aaattaaaga aaaaagtaca gaaagaactg ctccgttttt 1972680
tgtgctggaa gttaccgccc gttctgccgc cgatattttg ggtatccatc ccaattcggc 1972740
agcactgttc taccgtaaaa tccgcacggt tatcaaccat catttagcct tggctgccga 1972800
tgaggttttt gagggccctg tcgagccgga cgaaagcgat ttcggcggac ggcgtaaagg 1972860
cagacgtggt cgcggtgcgg caggaaaagt ggttgtcttc ggcattctga aacgcaacgg 1972920
acggggctat accgttgtcg tagataatgc caagtctgaa acgttactcc ctgtcatcaa 1972980
gaagaaaatc atgccggaca gcattgttta taccgatagt ctgagcagct gcgacaagtt 1973040

ggacgtgagc ggttttatcc attaccgcat caaccattcc aaggagtttg cagaccgtca 1973100
gaaccacatt aacggcattg agaatttttg gaatcaggca aaacgcgtct tgcgaaaatt 1973160
atagtggatt aacaaaaatc aggacaaggc gacgaagccg cagacagtac aaatagtacg 1973220
aaaccgattc acttggtgct tcagcacctt agagaatcgt tctctttgag ctaaggcgag 1973280
gcaacgccgt actggttttt gttcatccac tataccttc cgacagccga acaaaacccc 1973340
gaatccgtct gcacggttcg gggtatatct ccaatacggg catcgtgttc cggaaaaccg 1973400
tcaaatccgc atcggcatca caatatattt gaaattcgga ttgttcggca cggtaaacag 1973460
cgtcgagcgg ttggcatcgc cgaaggcaag ctgcatatcg tcggaatgga tgttgcgcaa 1973520
cacgtccatc agatagccga tattgaaacc gacttcgagt tcgccgccct gataggcgat 1973580
ttcgatttct tcgcgcgctt cttcctgctc gttgttgctg cacacaacgc tcaacaggcc 1973640
gggttgcaaa aacaatcgcg caccgcggaa tttttcattg gcaagaatcg atgcacgttc 1973700
caacgcgccc aacaattctg ccctcgacaa cacgaaaatc ttgtcgttgt ccaaaggaat 1973760
cacgcggttg aaatcgggga atttgccgtc gatgaccttg ctgacgatgg tcgtgccgtt 1973820
gcattggaaa cgcacctgtt tgtccagcag ctcgatttga atcggatcgt cggggttgtt 1973880
caacagtttg aacagttcca gcaccgtttt gcgcggcaaa atcacttcgg cgcgcggcaa 1973940
atccgcatca atcgcgcagg ctgcataggc aaggcggtgt ccgtcggtcg ccacaaggcg 1974000
caactggctg ccctcaacct gcatcagcag accgttgaga taatagcgga tgtcctgcac 1974060
cgccatgctg tactgcactt gcgacagcat ggttttgaaa cgctcctgct ccagcgagaa 1974120
agtcgcgctg atgtcctcgc cgacattcat catcggaaaa tcggcggcag gcagggtctg 1974180
cagggcaaaa cgcgatttgc ccgccttcag cgtcagacgg ctgtcgtccc aatccagcga 1974240
caccagcgca ccggcaggca gcgcgcgcaa aatatcctga aatttcttgg cattggtggt 1974300
gatgcggaag tcgcccgcgc cgccctcggg acccgcagtg tcgatttgga tttccaaatc 1974360
ggttgccaag agtttggtct gaccgccttt tccctcaatc aggacgttgg acaggatggg 1974420
cagggtgtgg cggcgttcga cgatgccggt aacggcttgc aacggcttga gcaggctgtc 1974480
gcgctcggct tgtaaaatca acatgttcgc tcctttaaat cggtttgtat agtggattaa 1974540
atttaaatca ggacaaggcg acgaagccgc agacggtaca aatagtacgg aaccgattca 1974600
cttggtgctt cagcacctta gagaatcgtt ctctttgagc taaggcgagg caacgccgta 1974660
ctggtttaaa gttaatccgc tatatcttta cccttcggac ggcatgggca atatcatgtc 1974720
gtctgaaaac gttttccatc agttttgaat cagaatcagc agcttttcat aatcctgagc 1974780
caattccgga tcttcttcgc gcagtttcgc cactgccctg atgccgtgca taacggtcgt 1974840
atggtcgcgc ccaccaaacg aatcgccgat agacggcagg ctcaaagtag tcagttcttt 1974900
ggtcaggctc atcgccacct ggcgcggacg ggcaatgttt cgtgtccgtt tcttaccgag 1974960
cacatcgctg attttgatgc ggtaatattt cgccaccgca tcgatgatga tgtcggcggt 1975020
gatgactttg tgcttctcgg caataatgtc ctgcaaagcg gtacgcgcca aatcgatgtc 1975080
gatgacggga cggttcataa agcggctgct cgctccgaca cgattaaacg cgccttcaag 1975140
ctcgcgcacg ttggaacgga tcagattggc aatgaacagc gcggcttcgt cttcgatact 1975200
gatgcccgcc gcttccgcct ttttctgcaa aatggcgatg cgcatttcca attcgggcgg 1975260
ctcgagttcc aaagtcagtc cccatgaaaa acgggatttg aggcggtcgt ccatgccttc 1975320
gattttcgca ggcaacacat cgcaagtgag gatgagctgt tttttctcgt tgtggaaatg 1975380
gttgtacaga tagaaaaact cttccatcgt acggtctttg cctttgatga actggatgtc 1975440
gtcgataatc agcaggtcgt attgcttgta ttgctgcttg aacacgtcgt aagtgttgtt 1975500
gcgaaccgcc ttcataaagc tgcggatata gtcatccgaa tgcatatagc gcactttggc 1975560
atcgggacgg tttttcagca gctcgttgcc gaccgcctgc acaaggtggg ttttgcccaa 1975620
acccgtgctg ccatagagga agaacgggtt gtaactctgc cccgggcttt ccgcaatcgc 1975680
ctgcgccgca gccgccgcaa ggcggttgcc cttaccttct accaacgtat caaacgtgta 1975740
atccggagac aggttggtct gctcgtaacg cgcctcttcc gcatcgcgct gcacgtccgt 1975800
ccgtgctttg gcaactgcca ccgattccgg ccgggaagca gacccggcag cctgacgcgg 1975860
ctcgtgcggc aggtttttca tacgttccgc caaaatatcc gccgccgttt tcgacgcagc 1975920
gggtttgaca ggctcttcag acggcagctc gtccaacaga acctcctgca cgggcattcc 1975980
ctccgacacc gcatgcaagg acggctcggc aggttcgaca gcaccttcaa ccgccgccat 1976040
ctcataacgc acgccttctc ccggtttgaa tacgaaggcg gaacggccgg cagccaactc 1976100
ttccctcacc gcttctattt ttccggcaaa ctggctcttg agcatattgc aggcaaactg 1976160
gttcttgccg tacaccaccc atacgccacc ctcctcacca acggtaaggg gcgcaatcca 1976220
ttgcgcaaac tgcccttgag gcaacatatc gtgaagacgg cggaggcaca gcggccaaaa 1976280
ctctgctaat gtcatggata ggctcgaatc ggtaaaaatg aaatcgaaaa caaagaaaat 1976340
ataatatttt caaaaagaaa acaaatctgt tgaacgcaca tcggttcaaa acgcgactgc 1976400
ccgattatac cgactcacga atattttatc cacaacccgt gcaaaaattt atccacagaa 1976460
aggcggcgga aatccgcagg caatcgggca atcttcctgc aaagtttcta tattgattga 1976520
caaaagcggc aaattggagt gtaattcacg gtttaattat ctacccattc tattttagga 1976580
aacatcatga aacgcactta tcaaccttcc gttaccaaac gcaaacgcac ccacggcttc 1976640
ctggtgcgct ccaaaacgcg cggcggccgc gcagtattgg ccgcacgccg tgccaaaggc 1976700

cgcaaacgcc tggcggtata attttggact accgcttcgg aaggcagtac cgcttgttga 1976760
aaacggatga tttttcatcc gtttttgcat tcagaaaccg ccgcagccgc gacctgctgc 1976820
aagtttcgcg ctcaaacggc aacgggctgg gccatccccg catcggtctg gtggtcggca 1976880
aaaaaaccgc caaacgcgcc aacgaacgaa attatatgaa gcgcgttatc cgcgactggt 1976940
ttagattgaa caaaaaccgg ctgccgccgc aggatttcgt cgtgcgcgtc caccgtaaat 1977000
tcgacagggc taccgcaaaa caggcaaggg cggaactggc acaactcatg ttcggcaacc 1977060
ccgcaaccgg atgcaggaaa caggcatgat cagaacggta ctctgcaggc aaggttcaga 1977120
cggcaacggg tttcccatac aaggaacatc ccgatgaact tcctattgtc caaactcctg 1977180
ctgggactga tacggttcta ccaatattgc atcagcccgc tgattccgcc gcgctgccgt 1977240
tatacgccga cctgttcgca atacgcggtc gaagcggtca aaaaatacgg cgcattcaaa 1977300
ggcggccggc tcgccatcaa gcgcattgca cgctgccacc ctttcggcgg acacggacac 1977360
gaccccgttc cctgacccga cgcaatattc aaattgcacg ctttcctttt atttcccatc 1977420
ggtttctata taatgccgtc tgaagcttcg ggcaggcggc acgaccgccg ggtatgaagc 1977480
ccgcccttat tccccgtcta tcggaacacg caacctgcgg catttccgac cattcaggaa 1977540
actcttatgg attttaaaag actcacggcg tttttcgcca tcgcgctggt gattatgatc 1977600
ggctgggaaa agatgttccc cactccgaag cccgtccccg cgccccaaca ggcagcacaa 1977660
caacaggccg taaccgcttc cgccgaagcc gcgctcgcgc ccgcaacgcc gattaccgta 1977720
acgaccgaca cggttcaagc cgtcattgat gaaaaaagcg gcgacctgcg ccggctgacc 1977780
ctgctcaaat acaaagcaac cggcgacgaa aataaaccgt tcatcctgtt tggcgacggc 1977840
aaagaataca cctacgtcgc ccaatccgaa cttttggacg cgcagggcaa caacattcta 1977900
aaaggcatcg gctttagcgc accgaaaaaa cagtacagct tggaaggcga caaagttgaa 1977960
gtccgcctga gcgcgcctga aacacgcggt ctgaaaatcg acaaagttta tactttcacc 1978020
aaaggcagct atctggtcaa cgtccgcttc gacatcgcca acggcagcgg tcaaaccgcc 1978080
aacctgagcg cggactaccg catcgtccgc gaccacagcg aacccgaggg tcaaggttac 1978140
tttacccact cttacgtcgg ccctgttgtt tatacccctg aaggcaactt ccaaaaagtc 1978200
agcttttccg acttggacga cgatgccaaa tccggcaaat ccgaggccga atacatccgc 1978260
aaaaccccga ccggctggct cggcatgatt gaacaccact tcatgtccac ctggattctc 1978320
caacctaaag gcagacaaag cgtttgcgcc gcaggcgagt gcaacatcga catcaaacgc 1978380
cgcaacgaca agctgtacag caccagcgtc agcgtgcctt tagccgccat ccaaaacggc 1978440
gcgaaagccg aagcctccat caacctctac gccggcccgc agaccacatc cgtcatcgca 1978500
aacatcgccg acaacctgca actggccaaa gactacggca aagtacactg gttcgcctcc 1978560
ccgctcttct ggctcctgaa ccaactgcac aacatcatcg gcaactgggg ctgggcgatt 1978620
atcgttttaa ccatcatcgt caaagccgta ctgtatccat tgaccaacgc ctcttaccgc 1978680
tctatggcga aaatgcgtgc cgccgcaccc aaactgcaag ccatcaaaga gaaatacggc 1978740
gacgaccgta tggcgcaaca acaggcgatg atgcagcttt acacagacga gaaaatcaac 1978800
ccgctgggcg gctgcctgcc tatgctgttg caaatccccg tcttcatcgg attgtattgg 1978860
gcattgttcg cctccgtaga attgcgccag gcaccttggc tgggttggat taccgacctc 1978920
agccgcgccg acccctacta catcctgccc atcattatgg cggcaacgat gttcgcccaa 1978980
acttatctga acccgccgcc gaccgacccg atgcaggcga aaatgatgaa aatcatgccg 1979040
ttggttttct ccgtcatgtt cttcttcttc cctgccggtc tggtattgta ctgggtagtc 1979100
aacaacctcc tgaccatcgc ccagcaatgg cacatcaacc gcagcatcga aaaacaacgc 1979160
gcccaaggcg aagtcgtttc ctaaatgccg cagcatgaaa aatgccgtct gaaacctgtt 1979220
cagacggcat ttttattgcc caccccctat cggggcggaa atcttcaacc cgcatacatc 1979280
acaaaaatcg tcgggcgttt tttcagattg ggcatttctt ttctttttcg ccactgcacg 1979340
attgtttgac tgatgatttc ctgtgtcggc aaggtcaaat ccgtagccgt gcataaacgc 1979400
gtttcaggat gcaggttttc caccgcatcg gcaagcagcg catcattgcg gtaaggcgtt 1979460
tcaataaaaa tctgcgtctc gccgcactgg cgcgaacgct gttccaaagc ccgaaaagcc 1979520
tgaatccgct cgtttttttc agacggcaga tagcctttaa acgcaaaact ctgcccgttc 1979580
gcacccgaag ccatcaaagc cagcagcagg ctggaaggcc cgaccagcgg acgcacttca 1979640
aaaccgtgtt tatgcgccaa tgccaccaaa ttcgcacccg gatcggccac agccgggcaa 1979700
cccgcctcac tgacaatgcc catactgcgc ccttcttgca aaggtttcag caattccggc 1979760
aaagtcttca aatccgtatg ttcattcaac gtttgcagat tcagctcgcg gataggcgta 1979820
gtcacgccca aatgtttcaa atgcgcacgc gccgtttttt ccgcctccac gacaaaatcc 1979880
gtcagcccga caatcgcctg ttgttcatgc ggcaacaggc acggcgtgtc aggcgtaccc 1979940
aaaggcgtag gaatcaaata caaaacagga gacatcattc cctcactcat cggttaaaaa 1980000
tgccgtctga gcctttcaga cggcataaac gggcagttac agaacctcca cgccctcatt 1980060
tttcaagaaa tcgaccagac ggaaaaccgg caaaccgatt aaagcattcg gatcggtact 1980120
ctcaatcctt tcaatcagca atgcacccaa atcctcactc ttcagcgcac acgaacaata 1980180
aaccgcatca ggctcgcgct ccaaatagcg gaggatatgc aactcgtcca actgcctcat 1980240
cacgaccacc gtcttatcga tatgccgccg catcctgccc gtaaccgtat tcaacagcac 1980300
gatcgcgctg taaaactcaa tctccctgcc gctcaagtgc atcagcatct tttgcgcatt 1980360

ggcaaggttc atcggcttgc cccactgcct gccgtcgcac cacgccacct ggtccgcacc 1980420
gacaatcaac gcctctggga aacgcccggt caacgaccgc gccttaccct cggcaaggcg 1980480
caatgccgtc tgaggggcgg attcccccaa catcggcgtt tcgtcaaaat cgggggacgc 1980540
cgcctgaaag gcaatgccga gcctttccat ctgttcgcgg cggaaaaccg aactcgtacc 1980600
caaaatcaaa ggcagttcca aacccatccc atcctcctta ccgttgaaaa cacgcccgaa 1980660
ggggcagtaa aatccagcca tgcgccgaaa cacggatacc cgccttcggc gtaccgcaac 1980720
atttttctta aaaatattga cgttagaaca tctaaattat atcatatccc gtttatgtca 1980780
gaccctaatt tgattgactt ggaaattttt gccgccgaag ggcagaacct gcaaggcagt 1980840
tttctgctgg aagaattgga tgaacgcgtc agttcgcacg attatcccgc cgacaggcag 1980900
accaaaatat cgtttacact gaccggcggt cgcgaccggc tgcaacgcct gttcctcgac 1980960
ctgaacgtca aagccgatat gcccctgatt tgccagagat gtatcaaacc catgccgttc 1981020
atgcttgatg aaagcagccg tatcgtcctg ttttccaacg aagagtcctt ggacgaatcc 1981080
atgcttgccg acgaagaact cgaaggcata ctgattgaaa aagaactcga cgtgcgcaca 1981140
ttggtagaag accaaatcct gatgtccctg cccttttcgc cgcgacacga agactgcggc 1981200
gacaatggga cactggaaga agtcaatcgg gacaaaccca accccttgc tgttttggca 1981260
ggtttgaaaa gcaattgatt aggacacagt ttatttatct aggagcttga aatggccgtt 1981320
caacaaaaca aaaaatcccc ttccaaacgc ggtatgcacc gttcgcacga cgcgctgacc 1981380
gcgcctgcac tgtctgtcga cagcacaacc ggcgaagtac accgcccgca ccacatctcc 1981440
cccaacggta tgtaccgcgg ccgcaaagtg gtcaaagcca aaggcgaata atccctattc 1981500
gactgactga aaaagccaga acattgccat gcaattactg gcttttttg cattggacgc 1981560
accatccgtc caaactttcg ccatacgtca acacacaggg gcaaagcgtt ccgtataata 1981620
ccccgtgaaa atattccaaa agccccaacc accaaggaaa ttccgatgaa acagaaaatc 1981680
tggtacacct acgatgacat ccaccgcgtc atcaaagcat tggcagaaaa aatccggaac 1981740
gccgacatca aatacgatgc catgattgcc atcggcggcg gcggctttat tccggcacgt 1981800
atgctgcgct gttttctgga aattccgatt tatgccgtaa ccaccgccta ttacgacagc 1981860
gacaacgaag gacaggttac cgaagaagtc aaaaaagtcc aatggctcga ccccgttccc 1981920
gaagccctgc ggggcaaaaa cgtactcgtc gtcgatgaag tggacgacag ccgcgtaacc 1981980
atggagttct gcctgaaaga actgctcaag gaagacttcg gtacgatcgg agtcgccgta 1982040
ctgcacgaaa aaatcaaagc caaagcaggc aaaatccccg aaggcattcc ctatttcagc 1982100
ggcatcaccg tagaagactg gtggatcaac tatccgtggg acgcactcga catcgacgaa 1982160
cacaaccgcc ttgccgaggc cggccgaggc tgaccctttc agacggcata ttttccgaac 1982220
cgatgccgtc tgaagcccgc acgaccccctg ccgcagaccg aaaacctacc ggagaaaccc 1982280
tatgattaca ttggccgtag atgccatggg cggcgaccaa ggacttgccg ttaccgtacc 1982340
cggcgcaacc gcattcctcc aagcacaccc cgatgtccgc ctgattatga ccggcgacga 1982400
aacgcaactg cgccaagccc tgaccgcggc aggcgcaccg atggaacgca tcgacatctg 1982460
ccataccacc caagtcgtcg gcatggacga agccccgcaa tccgccctga aaaacaaaaa 1982520
agactcctcc atgcgcgtcg ccatcaacca ggttaaagaa ggcaaagccc aagccgccgt 1982580
atccgcaggc aacacgggtg cgctcatggc aaccgcacgt ttcgtcctca aaaccattcc 1982640
cggcatcgaa cgcccccgcca tcgccaaatt ccttccttcc gacaccgacc acgttaccct 1982700
tgcactcgac cttggcgcga acgtcgactg cacgtccgaa cagctcgccc aatttgccgt 1982760
tatcggcagc gaactcgtcc acgcactcca tcctcaaaaa ggacagccgc gcgtcgggct 1982820
ggtcaacgtc ggcacggaag acatcaaagg tacggacacc gtcaaacaaa cctacaaact 1982880
gctgcaaaac agcaaactca actttatcgg caacatcgaa agcaacggca tcctctacgg 1982940
cgaagcagat gtcgtcgtcg ccgacggctt tgtcggcaac gtcatgctca aaaccatcga 1983000
aggcgcggtc aaattcatga gcggagccat ccgccgcgaa ttccaaagca acctgttcaa 1983060
caaacttgcc gccgttgccg ccctacccgc cctcaaaggg ctgaaaaaca aactcgaccc 1983120
gcgcaaattc aacggggcca tcctgctcgg gctgcgcggc atcgtgatta aaagccacgg 1983180
cggcacagac gaaaccggtt tccgctatgc cctcgaagaa gcctaccacg aagccaagtc 1983240
cgccggcctt tccaaaatcg aacagggcgt agccgaacaa ctcgccgcac tcgaaactgc 1983300
caaagccgtc caaaacgaaa atgtcggcgg tctgtaacac acacgatgcc gtctgaacgc 1983360
ccccgcccct ttcagacggc atccgcccgc accaaacctg cgggcgcgga cggcgatgcg 1983420
cctgtccggc acttcccaaa tatcgccttg taaaataagg agtatttgaa aaatgaagac 1983480
attagaaaaa cggatgaaag ctctagacaa acggattatg aagttcggaa aatcccttga 1983540
aggcaggctt gatgcccgtc tgattgaatc cgcattggat tatattcatt attcggaacg 1983600
ttttttggct tttgaaatcc tgtgtactta tatcgaagat ttcgatgtcc ggctgacgga 1983660
acaagaatcc cgggaaattt cttttatcaa caaggaattt gagatagaaa gcacgtccga 1983720
ttaaccaata aagccaatgg gttgataaac atgaaaacat cgacggtcgt tttggcgga 1983780
ttttttatgg cagacaacgg agagcgaatc caaatccccg ttttggaaaa tcctgacatt 1983840
agggaaatca atcacttttt ttccgtatca aattttgaga aaaaaaccgg cgtccttgtt 1983900
ttcagaatca tccccgagcc ggaatttggc aataccgaat taactgtcta ttttaaaaaa 1983960
ggatattata gtggattaac aaaaaccagt acggcgttgc ctcgccttgc cgtactggtt 1984020

tttgttaatc cactatatca gacgaaaaca aacacccgcg ccaatagcct gacggcaacc 1984080
cggcaatcaa aatgccgtct gaagcagctt gggctttcag acggcatttc cttcgcttaa 1984140
aacagcgtat cggcaacccc gccctgcctg tccacggcaa tctgcatctg aaaaccatct 1984200
gtatcccaaa ccacaccccc atccctgttt ccatcatgtg caccctgtcc gtattgggca 1984260
atcatctgtt tttcgcttac aatagccgaa tctgaaccaa ctctctaaaa aggccgttcc 1984320
catgcagtat gcaaaaattt ccggcacagg cagctatctt cccgccaacc gcgtcagcaa 1984380
tgacgacctt gcccaaaagg tagatacctc tgacgagtgg attaccgcgc gcacgggcat 1984440
caaattccgc catattgcag ccgaaaacga aaaaaccagc gatcttgccg ccgaagcggc 1984500
gcaccgcgcg ctggatgcag ccggattaga cagcggcgaa atcgatttga ttatcgtggc 1984560
aacggcaacg ccggatatgc agtttccgtc tactgcgacc atcgtgcaac aaaaattggg 1984620
catcaccaac ggctgccccg cgtttgacgt acaggcggtg tgcgccggct ttatgtacgc 1984680
gctgaccacg gcaaacgcct acattaaaag cggtatggcg aaaaacgcgc tggtcatcgg 1984740
cgcggaaacc ttcagccgca ttgtagactg gaacgaccgc acaacctgcg tattgttcgg 1984800
cgacggcgcg ggcgcggtgg ttttaagcgc gtcggacacg ccgggcatca tccacagcaa 1984860
actcaaggcc gacggcaatt atctgaaact cttaaacgtc cccgggcaaa tcgcctgcgg 1984920
caaagtttcc ggttcgccgt acatttcgat ggacggtccc ggcgtgttca agtttgccgt 1984980
caaaatgctg tccaaaatcg ccgatgacgt tatcgaagaa gcaggttaca ccgccgctca 1985040
aatcgactgg attgttcccc atcaggcaaa ccgccgcatt atcgaatcga ccgcgaaaca 1985100
tttaggtttg agtatggaca aagtcgtcct gaccgtccaa gaccacggca acacatccgc 1985160
cgcatcgatt ccgctggctt tggatacggg catccgcagc ggacaaatca aacgcggtca 1985220
aaacctgctg ctcgaaggca tcggcggcgg tttcgcgtgg ggcgcggtgc tgttgcaata 1985280
ttgaacccga tgccgtctga aacaggcttt cagacggcat ttcccatatc atgaagcggc 1985340
aggctttctt caaactgatg gcgtgtgcgg catttctgtc tgccgtttcg ctgcgcctcc 1985400
ccgtattggg cgcgtgttac gcaatattgt ccctctatgc gtttgcactt tacggcatcg 1985460
acaaacggtg cgccatacgg gggcaacgcc gcattcccga acaccgcctg ctgctgcctg 1985520
cattgctcgg cggctgggtg ggcgcgtatt tcggcagcat gacattcaaa cataagacag 1985580
cgaaaaagcg ttttgttgtg ctgttccgtc tgactgtttc aggtaatgtc ttggcgaccc 1985640
tcatcctgat ttatagtgga ttaaatttaa accagtacgg cgttgcctcg ccttgccgta 1985700
ctatttgtac tgtctgcggc ttcgtcgcct tgtcctgatt tttgttaatc cactatatta 1985760
ttttgtcccg cctgaatttt tcgtaaaact cgggcagaat acctgattat ccaaccaaac 1985820
aaaggaatac tatgtctttt gccttctttt ttcccggaca aggttcccaa agcctcggta 1985880
tgatgaacgg ctttgccgaa cacgccatcg tcaaaaacac ctttgccgaa gcctccgcca 1985940
tattggggca ggacttgtgg gcgatgataa acggcagcga tgccgaaatc atcggtcaaa 1986000
ccgtcaacac ccagcccatt atgctcgccg ccggcgttgc cgtttaccgc gcctatttag 1986060
aagcgggcgg caaaacgcct gccgccgttg ccggacacag cctcggcgaa tacaccgcac 1986120
tcgttgccgc cggcgcattg aattttgccg acgcggtcaa actcgtgcgc ctgcgcgccg 1986180
aactgatgca gtccgccgta ccgcaaggcg tgggcgcaat ggcggcgatt ctcggcttgg 1986240
aagatgagca ggttaaagcc atttgtgccg aagccgccca aagcgaagtg gtcgaagccg 1986300
tcaacttcaa ctcacccgga caaatcgtga ttgcaggcaa cgccgccgcc gtcggacgcg 1986360
ccatggctgc cgccaaagaa gccggtgcca aacgcgccct gccgctgccc gtgtccgtac 1986420
cttcccattg cagcctgatg aaacccgccg ccgacaaact tgccgaagcc ctgaaaaccg 1986480
ttgaaatcaa gcagccgcaa atccgcgtta tccacaacgc cgacgttgcc gcctacgatg 1986540
atgccgacaa aatcaaagac gcgctcgtcc gccagcttta cagccccgta cgctggacgg 1986600
aaaccgtcaa cgccctcgtt tcagacggca ttgccgaatc cgccgaatgc ggcccgggca 1986660
aagtgttggc gggcttggca aaacgcatca acaaagccgc cgcgtgcagc gcactgaccg 1986720
atgccggaca ggttgccgcc tttatcgaag cgcactgact tcgttctgca aaaagcagcc 1986780
tgccctcttc aggctgcttt tcatgtccga acgacggcag ccccatattt acgctataat 1986840
ccatcccgac caaaccaccg acagcggctg ccgttgcagt tcccgcccta ccgatatgat 1986900
agaaaaactg actttcggac tgtttaaaaa agaagacgcg cgcagcttta tgcgcctgat 1986960
ggcgtacgtc cgcccctaca aaatccgcat cgttgccgcc ctgattgcca ttttcggcgt 1987020
tgccgccacc gaaagctacc ttgccgcctt catcgccccc ctgattaacc acggcttttc 1987080
cgcacctgcc gcgccgcccg agctgtctgc cgccgccggc atcatttcca ccctgcaaaa 1987140
ctggcgcgaa cagtttacct atatggtttg ggggacggaa aacaaaatct ggaccgtccc 1987200
gctcttcctc atcatcctcg tcgtcatccg tggcatctgc cgctttacca gcacctatct 1987260
gatgacttgg gtctccgtga tgaccatcag caaaatccgc aaagatatgt ttgccaaaat 1987320
gctgaccctt tcctcccgct accatcagga aacgccgtcc ggcaccgtac tgatgaatat 1987380
gctcaacctg accgaacagt cggtcagcaa cgccagcgac atcttcaccg tcctcacgcg 1987440
cgacacgatg atcgttaccg gcctgaccat cgtcctgctt tacctcaact ggcagctcag 1987500
cctcatcgtc gtcctgatgt tcccccctgct ctccctgctc tcgcgctact accgcgaccg 1987560
tctgaaacac gtcatttccg actcgcaaaa aagcataggc acgatgaaca acgtgattgc 1987620
cgaaacccat cagggacacc gcgtcgtcaa gctgttcaac gggcaggcgc aggcggcaaa 1987680

ccggttcgac gcggtcaacc gcaccatcgt ccgcctcagc aaaaaaatca cgcaggcaac 1987740
ggcggcacat tccccgttca gcgaactgat cgcctcgatc gccctcgccg tcgtcatctt 1987800
catcgccctg tggcaaagcc aaaacggcta caccaccatc ggcgaattta tggcattcat 1987860
cgtcgcgatg ctgcaaatgt acgcccccat caaaagcctt gccaacatca gcatccctat 1987920
gcagacgatg ttcctcgccg ccgacggtgt atgtgcattt ctcgacaccc cgcccgaaca 1987980
ggacaagggc acgctcgcac cgcagcgtgt cgaagggcgc atcagcttcc gcaacgtcga 1988040
tgtcgaatac cgttcagacg gcatcaaagc cctcgacaac ttcaacctcg acatcagaca 1988100
aggcgaacgc gtcgccctgg tcggacgttc cggcagcggc aaatccaccg tcgtcaacct 1988160
gctgccccgc tttgtcgaac cgtctgccgg caacatctgc atagacggta tcgacatcgc 1988220
cgacatcaaa ctcgactgcc tgcgcgccca attcgccctc gtctcccaag acgtattcct 1988280
gtttgacgac accctgtttg aaaacgtccg atacagccgt cccgacgcgg gcgaagccga 1988340
agtcctgttc gccctccaaa ccgccaacct gcaaagcctg attgacagct ccccgctcgg 1988400
actgcaccag cccatcggat cgaacggcag caacttatcc ggcggacagc ggcaacgcgt 1988460
cgccattgcc cgcgccattt tgaaagacgc gccgatatta ttattggacg aagccaccag 1988520
cgcattagac aacgaatccg aacgcctcgt ccaacaggcg ctcgaacgcc tgatggaaaa 1988580
ccgcaccggc atcatcgtcg cccaccgcct gaccaccatc gaagggggccg accgcatcat 1988640
cgtgatggac gacggcaaaa tcatcgaaca aggcacacac gaacaactga tgtcccaaaa 1988700
cggttactac acgatgttac gcaatatctc aaacaaagat gccgccgtcc ggacggcata 1988760
aacaaaatgc cgtccgaaat ggtacaatcg ccccgaccct ttcagacggc atcatatccg 1988820
ccgacccatc cgattatctt caatcactgt aaaacccatt atgacccaag acaaaatcct 1988880
catccttgac ttcggttcgc aagttaccca gctcatcgcc cgccgcgtgc gcgaagccca 1988940
cgtttactgc gagctgcatt ctttcgatat gcctttggac gaaatcaaag ccttcaaccc 1989000
caaaggcatc atcctctccg gcggccccaa ttccgtttac gaatccgact atcaagccga 1989060
taccggtatt tttgatttgg gcattccggt tttgggcatc tgttacggca tgcagtttat 1989120
ggcgcaccac ttgggcggcg aagtgcagcc cggcaaccag cgcgaattcg gttatgcgca 1989180
agttaaaacc atagacagcg agctgacacg cggcattcaa gatggtgagc caaacacact 1989240
cgacgtatgg atgagccacg gcgacaaagt gtccaaactg cccgacggtt tcgccgtcat 1989300
cggcaacacc ccgtcctgcc cgattgccat gatggaaaac gccgaaaaac aattctacgg 1989360
catccagttc caccccgaag ttacccacac caaacaaggc cgcgccctgt tgaaccgctt 1989420
tgtcttggat atttgcggcg cacaaccggg ctggacgatg ccgaactaca tcgaagaagc 1989480
cgttgccaaa atccgcgaac aggtcggcag cgacgaagtg attttaggtc tgtccggcgg 1989540
cgtggactct tccgtagccg ccgcgctgat tcaccgcgcc atcggcgacc aactgacctg 1989600
cgtgttcgtc gatcacggtt tgttgcgcct gaacgaaagc aaaatggtga tggatatgtt 1989660
cgcccgcaac ttgggtgtga aagtgataca cgtcgatgcc gaagggcagt ttatggcgaa 1989720
actcgccggc gtaaccgacc ccgagaaaaa acgcaaaatc atcggtgcgg aatttatcga 1989780
agtatttgat gccgaagaaa aaaaacttac caacgccaaa tggttggcac aaggcacgat 1989840
ttaccctgac gtaatcgaat ccgcaggtgc aaaaaccaaa aaagcccacg ccatcaaatc 1989900
gcaccacaac gtcggcggcc tgcctgaaaa catgaagctc aaattgcttg agcctttgcg 1989960
cgatttgttc aaagacgaag tacgcgaatt gggtgtggct ttgggcctgc cgcgcgaaat 1990020
ggtgtaccgt catccgttcc cgggtccggg tttgggcgtg cgtattttgg gcgaagtgaa 1990080
aaaagaatat gccgacctgc ttcgtcaggc agacgatatt ttcattcaag aattgcgcaa 1990140
tactaccgat gaaaacggta catcttggta cgacctgacc agccaggcat tcgccgtgtt 1990200
cctgcccgtc aaatctgtcg gcgtaatggg cgacggccgc acatacgatt acgtcattgc 1990260
cttgcgtgcc gtgattacca gcgactttat gaccgcgcat tgggcggaac tgccgtattc 1990320
cttgttgggc aaagtgtcca accgcatcat caacgaagtc aaaggcatca accgcgtggt 1990380
ttatgatgtg agcggcaaac cgcctgccac catcgagtgg gaataaacag caaacatggc 1990440
tgccccgtcc ggcgcagtcc ttcgattatc ggaaaaaagg aaaaaatatg agcacacaag 1990500
atttaaacgg caaaatcgct ttggtaacag gcgcatcgcg cggtatcggt gcagcaattg 1990560
ccgacacgct ggcggcagcc ggtgccaaag tcatcggtac ggcgaccagt gagagcggtg 1990620
cggcggcgat tagcgagcgg ttggcgcaat ggggcggcga aggccgcgta ttaaattccg 1990680
ccgaacctga aaccatcgaa agcctgattg ccgacatcga aaaagcgttc ggcaaactcg 1990740
atattctggt caacaacgcc ggcatcaccc gcgacaacct cctgatgcgc atgaaagaag 1990800
aagagtggga cgacatcatg caggtcaacc tcaaatccgt gttccgcgct tctaaagccg 1990860
ttttgcgcgg tatgatgaaa caacgttccg gccgcatcat caacatcaca tccgtcgtcg 1990920
gcgtgatggg caatgccggt caaaccaact atgccgcggc aaaagcaggc ttaatcggtt 1990980
tctccaaatc catggcgcgc gaagtcggca gccggggcat taccgtcaac tgcgtcgccc 1991040
ctggctttat cgataccgac atgacacgcg ccctgccgga agaaacccgc caaaccttta 1991100
ccgcccaaac cgccttgggc agattcggcg acgcgcaaga catcgccgat gcggttctgt 1991160
tcctcgcttc cgaccaagca aaatacatca ccggccaaac gctgcacgtc aacggcggta 1991220
tgctgatgcc ttaacagaca actttttcaa ccatgccgtc tgaagccctt tcagacggca 1991280
tttgcattct caggcaaaat gaacacacac cacaccccgc cctgcccatg cggctcaggc 1991340

acaagctgag acctttgcaa aattcctttc cctcccgaca gccgaaaccc caacacaggt 1991400
tttcagctgt tttcagctgt tttcgcccca aataccgcct aattctaccc aaataccccc 1991460
ttaatcctcc ccggacacct gataatcagg catccgggtc accttttagg cggcagcggg 1991520
cgcacttagc ctgttggcgg ctttcaaaag gttcaaacac atcgccttca gatggctttg 1991580
cgcactcact ttaatcagtc cgaaataggc tgcccgggcg tagcggaatt tatggtgcag 1991640
cgtaccgaag ctctgttcga ccacatatag tggattaaca aaaaccagta cggcgttgcc 1991700
tcgccttagc tcaaagagaa cgattctcta aggtgctgaa gcaccgagtg aatcggttcc 1991760
gtactatttg tactgtcttc ggcttcgtcg ccttgtcctg atttttgtta atccactata 1991820
catcatcgct actaccgttc cggcgcaaca ggcattcctc gatgccgccg aactgatgca 1991880
atggagtata gaaaccgaag ggctgggctt gaacgtcatc tcgcacaaga tactcggcaa 1991940
agaccacgcc caagtcgaat ttgaagccta cttccgagac ggacaacacc gatccgcgca 1992000
tcacgaactg tccggcttcg tcaacatcgg cggacaatgg tattttatcg atcccaccgt 1992060
tccgcatcct gcgatgaaac aaccctgcat ttgcggatca ggcaaaaaat tcaaagcctg 1992120
ctgcggcaaa tatctgaaac ctgtcgcata aaaatgccgt ctgaacgttc agacggcatt 1992180
ttcaacgtgc aaaaaaaacc attcatacca agggtaagta tgaatggtca atacattgcg 1992240
ggaaaacgtc ttacttgctg cactgccgaa aagggagaaa cggcagcggt aatcagcgga 1992300
aaggattgta cccgaattaa tattaagaaa cgttaatcgc gaaaatatat taacaaacct 1992360
gttgaaacct attggttttc ccgtatccac ccgacccagc gttcaaacag cttcggttcg 1992420
agcgcggcaa cgaccgagcg tttgaacacg tgttcaccac tccaaaaccc gtcgccttcc 1992480
aaagtcgtca gcctgccgcc cgcctcctcg aaaatcaacg cgccggcggc ataatcccac 1992540
agcttctgcc cgccgtgaac ataaacatca taacgcccgc acgccagata acaccaatcc 1992600
aacgtactgc tgcccatact ccgtatcgtt ccaaaaggcg cgagcgtact catacggctg 1992660
gaaagtttgc ccgaacgcag atatttgatt tccacgcccg caatcgcctc attgagtttt 1992720
ttatccacga ggcgcagggg cagacgcgtc ccgtttaaaa acgccccctg cccgcgttcg 1992780
gcataaaaac attcgccgct gactgggttg tagattacgc ccaactcggc gcgcccgttg 1992840
cggacaaacg ccaccgatac cgcaaaatgc ggcagcccgt tgacaaaatt gttcgtcccg 1992900
tctatcggat cgacaatcca cagccccttt tcccccgaat attgttccca caaagccgac 1992960
tgttcctgcc gcgacatttc ctcacccaac atcggactgt cgattaaaag cggcaacgcg 1993020
gcggcaaaag ccgtctgcgc ggcaatgtcc gcctcgctca acatcgaacc gtcttccttg 1993080
cggtgagacg gcgtattcaa aaaacgcggc ataatttcgg tttgcgcgat atggcgcacg 1993140
actttctgca aacggtgtaa cacttcctac tgtcctcata ttttgaactt gcggcgcgcg 1993200
aacgtataat gtccgcttcc atcacgccgc tgcgacggat tataaccgtc cgaaccgcca 1993260
aaaactatgc cccgattcca cctgcccgaa aacctttccg tcggacaaac cgtcgccctg 1993320
cccgacaaca tcgtccgcca cctcaacgtc ctgcgcgtcc gccccaacga aaacatcacc 1993380
ctcttcgacg gcaaaggcaa ggcacacgcc gcacggctga ccgttttgga aaaacgccgc 1993440
gccgaagccg aaatcctgca cgaagacaca accgacaacg agtccccgct caacatcaca 1993500
ctgatacaat ccatctcctc cggcgatcgc atggatttca ccctgcaaaa aagcgtcgaa 1993560
ctcggcgtaa ccgccataca gcccgtcatc agcgaacgct gcatcgtccg cctcgatggg 1993620
gaacgcgccg ccaaacgcct cgcacgctgg caggaaatcg tcatctccgc gtgcgaacaa 1993680
agcggcagga acaccgttcc ccccgtactg cccatcatcg gctaccgtga agcactcgac 1993740
aaaatgccgt ctgaaagcac caagctgatt atgagcatca accgcgcccg caaactcggc 1993800
gacatacgcc aaccgtccgg cgcaatcgtc tttatggtcg ggcccgaagg cggctggaca 1993860
gaacaggaag aacaacaggc atttgaagct ggctttcagg cggttacact cggcaaacgg 1993920
attttacgca cagaaaccgc cccactcgcc gccctcgccg ccatgcagac gctttggggc 1993980
gatttcgcat aaacagaaat gccgtctgaa acccgttcag acggcatttt gcagccgatt 1994040
aagatagtag gttcaaataa gatttcccgt gtcgtcattc ccgcgaaagc gggaatctag 1994100
aaacgaaaaa ctacagagat ttatccgaaa caacaaccct ctccgccgtc attcccgcaa 1994160
aagcgggaat ctagaaacga aaaactacag ggatttatcc gaaacaacaa accctctccg 1994220
ccgtcattcc cgcgcaggcg ggaatctaga aacgaaaaac tacagggatt tatccgaaac 1994280
aacaaaccct ctccgccgtc attcccgcgc aggcgggaat ctagaaattt aacgttgcgg 1994340
tgatttatcg gaaatgactg aaactcaacg gactggattc ccgcctgcgc gggaatgacg 1994400
agattttagg tttctgtttt tggttttctg ttctcgcggg aataacggaa ttttaagttt 1994460
taggaatttg tcggaaaaac agaaatcccc ccgccgtcat tcccgcaaaa gcgggaatct 1994520
agaaacgaaa aactacaggg atttatccga aacaacaaac cctctccgcc gtcattcccg 1994580
cgaaagcggg aatctagaaa tttaacgttg cggtgattta tcggaaatga ctgaaactca 1994640
acggactgga ttcccgcctg cgcgggaatg acgaatttta ggtttctgtt tttggttttc 1994700
tgttctcgcg ggaataacgg aattttaagt tttaggaatt tatcggaaaa acagaaatcc 1994760
ccccgccgtc attcccgcga aagcgggaat ctagaaattt aacgttgcgg tgatttatcg 1994820
gaaatgactg aaactcaacg gactggattc ccgcctgcgc gggaatgacg aattttaggt 1994880
tgctgttttt tggttttctg tttttgcggg aatgacgaat tttaggtttc tgtttttggt 1994940
tttctgttct cgcgggaata acggaatttt aagttttagg aatttgtcgg aaaaacagaa 1995000

atccccccac cgtcattccc gcaaaagcgg gaatctagaa atttaacgtt gcggtgattt 1995060
atcggaaatg actgaaactc aacggactgg attcccgcct gcgcgggaat gacgaagtgg 1995120
aagttacccg aaacttaaaa caagcgaaac cgaacggact agattcccgc ctgcgcggga 1995180
atgacagtgt atccatttct aattttaatc cgctatattt tacacaaact atttgaacga 1995240
tatgacccgc ctgccgtaag ctttctcaag ctccgcctgc ctttgacgct ccattctttt 1995300
cttcttttcc ctaccgaatt tacccaaagc acgcttcaag tcaaacatca ccttcaacga 1995360
acggcggtgt cttctttctt gttccctatc tttttccaaa tcgctaccca acatactgtt 1995420
tttactgagg aacttggcat aatgcaattc ttgggtacat aaggcgggat taacctgata 1995480
aacaggcatc ccctccttat caaagaaata agtaaacatc atccaatcta ccgctttaat 1995540
ccactctgcc ggcaaaacgg caaacctttc caagaaaaac cgcatcgcct cacgcgaaat 1995600
gatatagcca gccgtccccc aatgttcgct ctccagcaaa ggaaatgacc gattctcata 1995660
attcaggact ttatccggtc tgacaataac tttcgcaaac atcgtttcca aacgaacgat 1995720
aaaggcagaa tccttatcaa aacgctcttc caaccaagta tcttcggcaa ggaacttttc 1995780
tgcgtctttg ccaagcagga catcatcctc aaatacggca acatagggca gaccttcatc 1995840
caatgcctgt ttccacaata cggcgtggct cataaagcag gctttttcca cttcgctcaa 1995900
caggtgctgt tttgccaatc ccggcaccaa ttccgccatc atccgattca gttcttcaga 1995960
cggcatcagt gcgtcgaaaa actgaaacgg gatgccgcgc acgccgaagg ttgcggcaat 1996020
gtgcgccctg cgttctgcgg cggaagctaa gctgataaca tggttttgca taatttatcc 1996080
tgttttttgt ctgttggata aagcggcgtt tttcaacggt ttttcagcaa tcggcgcaaa 1996140
atgccgaagt attgcctcaa ggtaaacagc cgccgcatcc tgccgtctgc tgcaaatacg 1996200
atgtccatct ctcctccttt tattggaaag gcacaatgaa ctgttcgcgc ctttgccggc 1996260
gtttttccct ttccctgctg attttggtca aggcgcggat caggcggtgt ttgaatgtgt 1996320
tggcggggga atcgcgcctt tgctgtttgc ggttcaggag gcggtcgtgt tcgatcaggc 1996380
tgcccaatgc gctgttttgg tcgtgaaact tggcataatg cagctcttgg gcgcacaagg 1996440
cgggattgag ctggcaaacc ggcattcctt ccctgtcgaa aaaatcgctg aacatcatca 1996500
gatcgacggg gtgcagccct tcgggcggca gggcggcaaa cctgtccagg aaaaaccgca 1996560
tcgcttttcg ggaaatgata tagcccgccg tcccccagtg ttcgctttcc aacagcggaa 1996620
aggcgcgccc gcagtaatcc gccacgccgg agggcgaggt caggacgtgc ataaacatcg 1996680
tttccaagcg gacgataaag gcggtatccg ggtcaaagcg ttcttgcagc caagcgtctt 1996740
cggcaaggaa tttttccgca ccttcgccga gtaaaacgtc gtcctcaaat acggtgatat 1996800
acggcagacc ttcgtccaat gcctgcttcc acaatacggc gtggctcata aagcaggctt 1996860
tttccactcc gctcaaatag ggtgcgccg acaagccggg gacgagttcc gccattgcct 1996920
gttccagcct ttcagacggc atcagtgcgt cgaaaaactg aaacgggatg ccgtgcctgc 1996980
cgaaggtatc ggcaatgtgc gccctgcgtt ctgcggcgga agctaagctg ataacgtggt 1997040
tttgcataat ttatcctgtt ttttgtctgt tggataaagc ggcgtttttc aacggttttt 1997100
cagcaatcgg tgcaaaatgc cgaagtattg cctcaaggta aacagccgcc gcatcctgcc 1997160
gtctgccgca aaatccagcc acgcgccggc gggcagcgtg tccgtccgtt tgaagcattg 1997220
gtacaaaaac cggcgggcgc gttcaaaatc ttcttccggc aaatgtttct ccagcaattc 1997280
atacgctact gcttttattt ggcggtattc aaggctgtcg aaccgggttt taaaacccat 1997340
agactgcaaa aaatcgtttc tggcggtttt ttggatgcct tgcgcgattt cgtgttggcg 1997400
gatgctgtat ttggatgaaa cctgattggc gtgaaggcgg tatttgacca aggcttcggg 1997460
ataataagcc agcctgccca atttgctgac atcgtaccaa aattggtaat cttccgccca 1997520
atcccgctcg gtgttgtaac gcaaaccgcc gtcaatgacg ctgcgcctca taatcatcgt 1997580
gttgttgtgt atggggttgc cgaaagggaa aaagtcggca atgtcttcgt gtcgggtcgg 1997640
ttttttccaa attttgccgt gttcgtggtg ccgcgccagc cggttgccgt ccttttcttc 1997700
cgacaaaact tccagccacg cacccatcgc gatgatgctg cggtcttttt ccatctcacc 1997760
cacgattttc tcaatccagt cgggggcggc aatatcgtct gcatcggtgc gcgcaatata 1997820
ttcccccccc ccccccgact ttgccaattc atccagcccg atgtttaaag agggaatcag 1997880
accggaattg cgcggctgcg cgaggatgcg gatgcggccg tcctgttctt ggaaacgctg 1997940
ggcaatggca agcgtaccgt ccgtcgagcc gtcatcgaca atcaaaatat ccaagttgcg 1998000
ccaagtttga ttcacgacgg cggctaatga ttgggcgaaa tatttttcta cgttgtaggc 1998060
gcaaatcaat acgctgacta aaggctgcaa tttattctcc cgataggcac gatgccgtct 1998120
gaaggcttca gacggcattt ggactgtaca acggttactc gcccaaaagc gcgatatccg 1998180
ctaccgcgtt catttgttct gccaagcggt tcagcaggtt caggcggttt tgtttcacgg 1998240
cggcatcttc cgccatcacc atcacgccgt cgaagaaggc atcgacttgc ggtttgacgg 1998300
aagccagttc ggacaaggcg gtctggaaat tgccttcggc aacggcggcg gcaattttcg 1998360
gctgcaagcc ttgtgcggcg gcaaagaggg ctttttcttc gtcctgttgc agcaagcttt 1998420
cgttaaccgc gcccaactcg gcatcggctt ttttcagcag gttttgcacg cgtttgttgg 1998480
cagcggcgag cgcggcggct tcgggcagtt gtttgaacgc ggcgacagcc tgcagtttgg 1998540
cggtcaaatc gtccaaacgg cgcggctgct tggcaagtac ggcggcaacg atgtcttgcg 1998600
gataatcgtt ttgcagcaat acggcaaggc gcgcctgcat gaagtcggcg gtttcagacg 1998660

gcgtttttc gttgagcaaa ccttgcggga agctgttgaa ggccgtctga atcagttcgt 1998720
ttacgtccaa accgtactgc atcagcatac gcaaaatacc caatgcggcg cggcgcaggg 1998780
cgtatgggtc tttgtcgccg gtcggaatca ggccgatacc ccaaatgccg accaaggttt 1998840
ccagtttgtc ggcaagcgca acggcggcgg caattttgcc ctcaggcagg ttgtcgccgg 1998900
caaaacgcgg ttggtagtgt tgctcgacgg cttcggtaat ttcttcggtt tcgccgtcca 1998960
agcgggcgta gtatttgccc atcgtgcctt gcagttcggg gaactcgccg accatttcgg 1999020
ttactaagtc ggcttttgcc aaacgcgcgg cgcgttcggc tgcggcggca tccgcgccca 1999080
aagccttggc gatatgggcg gcgatgcttt gcaggcgttc gatgcgttcg gcttgcgaac 1999140
cgattttgtt gtgataaacc acgttcgtca gtttgggcag gcggctttcc aaagtcgctt 1999200
tttggtcttg tttgtagaag aactcggcat cagacaggcg cgcgcgcaag acacgttcat 1999260
tgccttggat gatgtgtgac ggatcttcgg tttgcagatt ggacaccagc aggaagcggt 1999320
tcatcagctt gccgttttgg tcgagcagcg ggaagtattt ttggttttgc tgcatcgtca 1999380
gaatcaggca ttcttgcggt acggcgagga agtgttcttc aaaaccggct tccaatacca 1999440
caggccattc gaccagcgcg gttacttcgt ccaacaaggc ttcatcggcg gcggcggtcg 1999500
cgttcagacg gcgtgcctgc ccttccaata ccgtctgaat cgcggctttg cgctcggcaa 1999560
acgaagcgac gactttgcct tgctcgcgca tttgtgcggc gtagctgtcg gcgttttcaa 1999620
tggtaatttc gccgtcggag aggaagcggt gtcccaaggt tttgttgccg ctttgcagac 1999680
ccaaaacgct gacgttcaca atgtcgccgc cgtgcagtac aactagcccg tgaacggggc 1999740
gcacaaaggt aaacgtgctg ctgccccaac gcattacttt gggaatcggc agcttcttaa 1999800
ccgcttgatt gataatgtct tccaaaagtc cgcccaacgg tttgccgatt tggacgtatt 1999860
cgtaggcgta cacgtcctgc ttgccgtcgt ggacgatggt caagtcttcg attttcgcgc 1999920
ccgcaccgcg tgcgaaacct tccaaagcct tggttggcgc accgtctttc atggcattcg 1999980
ctacggcagg gcctttttttc acaatttttt gatcagcctg aacggctttg acgtttttga 2000040
cttgaaccgc caaacggcgc ggcgaggcat aagccgtaaa ttcggctgcg ccgtcaacca 2000100
gttgcgcttt ttccaagcct tcggcaacgg aagcggcgaa atggttgccc agattattca 2000160
gggctttggg cgggagttct tcggtaagga gttcgattaa aagggtttgg gtcatcattc 2000220
ggctttcttt gaatttggtt aatctgcctg tttataggtt tcgctgtaat tttcccagcc 2000280
gtcatcccca taaaaaccgt caaccagcgg ggtggcgtac aaagtggcaa catcttcgcg 2000340
gtctgccagc caagagataa tggctttttt ctcggtttct cccaagcttc gggcaccgga 2000400
tttttgaaac aggcacgaaa aatcgccgca atcgcccccc cgccatttca aagccgtttg 2000460
ccgcaagata cgcaatcagc tcgtccataa agcggtcgaa cgcttcggca tcgtcctcag 2000520
cttggtgcaa actgccttga acgccgaaaa tcaatgtttg aaactcgccc aaatgcagct 2000580
ttttatgctg gcggcggttc attttgtgca ggcgtttcct gcttggggtg cggaaataga 2000640
caggcatgat tttcctaaaa aatataatgg cttccggacg gctgccttat cgtgccgccc 2000700
gaacgtaaaa aatcgtcgcc cccttaggcg gcgtttgcct tcattaaagg gaagcccagt 2000760
ttttcgcggc tttcaacata tttttgcgcc acggcgcggc tcaatgcacg aatacgtcca 2000820
atataagttg cccgctcagt tacggaaatc gcgccgcgtg cgtctaaaag gttgaacgta 2000880
tgccccgctt tgaggacaag ctcgtaggca ggcagggcga gggcggcgtt ttcttcggca 2000940

agcaggcgtt tggcttgcgc ttcgtagtcg ttgaactggc gcagcagcca gtcggcatcg 2001000
ctgtattcga agttgtaggt ggattgctcg acttcgtttt ggtggtacac gtcgccgtag 2001060
gtgacggtgt tgccgtcgag cgtttttgcc caaacgaggt cgtagacgtt ttctacacct 2001120
tgcaagtaca tcgccaagcg ttcgatgccg taggtgattt cgccgagtac gggcgtgcag 2001180
tcgatgccgc cgacttgttg gaaataggta aactgggtta cttccatgcc gttgagccag 2001240
acttcccagc ccaaacccca cgcgccgagg gtggggtttt cccagtcgtc ttcgacaaag 2001300
cggatgtcgt ggactttggg atcgatgccc aattcgcgca gagagtcgag atagaggtct 2001360
tggatattgg cgggagcggg cttgagggcg acttggaatt ggtaatagtg ttgcaggcgg 2001420
ttggggttgt cgccgtagcg gccgtctttg gggcggcggc tgggttggac gtaggcggca 2001480
aaccaaggct cggggccgag tgcgcgcagg caggtggcgg gatgggatgt gccggcaccg 2001540
acttccatgt cgaagggttg gatgacggtg cagcctttgt ctgcccagaa tgtttgcagt 2001600
ttgaagatga tttgttggaa ggtaagcatg gcttatgatt cgataaaata aagggtttat 2001660
tttactgttt ccattgctgt ttggataggt ttatctcaaa gacagactga tttgaaaaca 2001720
cggcatacat gatatagtgg attaaattta aaccagtaca gcgttgcctc gccttagctc 2001780
aaagagaacg attctctaag gtgctcaagc accaagtgaa tcggttccgt actatttgta 2001840
ctgtctgcgg cttcgtcgcc ttgtcctgat ttttgttaat ccgctatatg tttcggttag 2001900
gcggcaggct gccctattga ataccttaaa gcaggctatg cctgccaacg ccatatccaa 2001960
acacagtctt taatttaaat ccggaaaata aaaagcacga ccaaacggtc gtgcttttcc 2002020
aaaccaaaca agtttatttc ttgtgcgaac ggatatagtc caaagttttg agctgtgcaa 2002080
tcgcagcagc caatacttta tgcgcttccg ccaaagcctt atcgtcttta gcttgggaaa 2002140
tgcccgcttc tgcggctttt ttcgcctctt ccgcacgtgc ccgatccatc tccgcactgc 2002200
ggacggcaac atccgccaag acagttactt tatcaggctg tacttccaaa acaccgccgg 2002260

aaacagcaac caaaacctct ttatcctcgc ccggaacggt caaacgcaaa gcccccggcc 2002320
gcaccaaact cataatcggc tcgtgtcgcg gataaatacc gagttcgccc tgtacagtcg 2002380
gaacaacgat aaatgttgcc tcgcctgaat agattttctg ctcgctactt accacctcaa 2002440
cttgcatgat gctcatgccg acctccttag tttaaggttt tcgctttctc tactgcttct 2002500
tcaatgctgc cgaccatata gaatgcctgc tcgggcagat gatcgtattc gccgttcaag 2002560
atggctttga agccggcaat ggtatcgcgc agggcgacat atttacccgg agaacctgta 2002620
aacacttcgg caacgtggaa cggttgggac aggaagcgtt ggattttacg cgcacgcatt 2002680
acggtcagtt tgtcttcatc agacaattcg tccataccca agatggcgat gatgtcgcgc 2002740
aattctttgt atttttgcag ggtggactgc acaccgcgcg ccacgtcgta gtgctcttga 2002800
cccaatacca tcggatccag ttggcgcgaa gtagaatcaa gcggatcgac tgccgggtaa 2002860
atacccaaag aggcaatatc gcggctcaat acgacggttg cgtccaagtg ggcgaacgtt 2002920
gttgccggag acgggtcggt caagtcgtcc gcaggtacat atacggcttg gatggaagta 2002980
atagaaccgg tttgggtaga ggtaatacgc tcctgcaaac gacccatttc ttctgccaat 2003040
gtcggttggt agcccactgc agacggcata cgacccaaca atgcggatac ttcggtacca 2003100
gccagggtgt aacggtagat gttgtccacg aagaacaata cgtcgcggcc tttgccgttt 2003160
tcgtcttttt cgtcacggaa gtattccgcc atggtcaaac cggtcaatgc gacgcgcaaa 2003220
cggttgcccg gaggttcgtt catctgaccg taaaccattg ccactttatc caatacgttg 2003280
gaatctttca tctcgtggta gaagtcgtta ccttcgcggg tacgctcacc cacgcctgcg 2003340
aacacggaca agccgctgtg cgctttggcg atgttgttga tcaattccat catgttcacg 2003400
gttttaccca caccggcacc gccgaacaga cctactttac cgcctttggc aaacggacac 2003460
agcaagtcaa tcactttaat gcccgtttcg agcaattcgg ttgtggaaga cagttcgtca 2003520
aacttagggg cagcttggtg gatggcacgg ctcttgtcgg tatcgatcgg acctgcttcg 2003580
tcaacaggcg ttcccaatac atcgacaatg cgtcccaacg taccttttacc taccggcaca 2003640
gtaatgggcg caccggtatt gctcacagtc atgccgcgtt tcaaaccgtc cgagctgccc 2003700
atcgcaatgg cacggactac gccgtcgccc aaaagctgtt ggacttccaa agtcagaccg 2003760
ttttcgtcta atttcaaagc gtcgtaaacg cgcggaatca tgtcgcgtgg aaattccacg 2003820
tcaacaaccg caccgataat ttgtacgatt ttgccttggc tcattatcgt atcctaattt 2003880
ccgtacagga ttcagacggc atcagacagc cgccgcacct gctacaattt ctgacaattc 2003940
cgtggtaatc gcagcttgac gcgatttgtt atataccaaa cgcaactctt tgatggcatt 2004000
gcctgcattg tctgttgcag ctttcatggc aaccatgcgg gctgcctgtt cggatgccat 2004060
attgtcgctc aacgcctgat aaaccacaga ctctaaatag cggcgaacca gatattccaa 2004120
cactgcaagt gcagtcggtt cgtagcggta ttcccagctg aacggtgatt tgggagctga 2004180
atcgccaatc acgttctcac cgataggcag caatacttcc attctcggtt cttgacgcat 2004240
ggtattgaca aaacccgaat acaccagatg gattctgtca atttcatgtt tctcataccg 2004300
ttggaagagt tctgtcaaag gtccgagcag catttccatt tttggggtat cgcccaaatt 2004360
tacggcactg gcaaccacat tcagaccaat gctctgacac gccatcagac ctttactgcc 2004420
aaagcatacg atttcctctt caataccttg attccgatac tcttgaactt gtgccaaaaa 2004480
ctttttcagc acgttggcgt tcaaaccgcc acacaaaccc ttatcagacg taatcaaaat 2004540
aaaaccgaca cgtctgattt cccgatgaga ttccagtaac ggaataccat gatcggtatt 2004600
ggtttgcgca agatggctca tcaccatacg cactttttcg gcatacggac gcgccaaacg 2004660
catccgttcc tgagtcttcc gcattttaga ggttgacacc atctgcatcg ctttagtgat 2004720
cttttgggta ttctgaacac tgcggatttt ggtgagaatc tcttttccta ctgccatttc 2004780
agactccttt cacttcaagc cttatgcctg ataggcgtaa gaagatttga aggatttcat 2004840
ggctgcttca agcgttttct cgctctcgtc ggacattgca cctgaagcat tgacggcttc 2004900
caaaacttcc ggatgttggg tacggacaaa gctcaaaaat tcagattcaa aagccagagc 2004960
tttggcaacc ggaacatcag aatacgaacc gttgttgatt gcccaaaggg tcaaagccat 2005020
ttcagccgta ttcaacgtac tgaactgttt ctgtttcatc agttcggtta cgacttcgcc 2005080
atgctccaat tgtttgcgcg tagcttcatc caaatcggat gcaaattgcg agaacgccgc 2005140
caattcacga tattgtgcca acgccaaacg gataccgcca cccagctttt taatcacttt 2005200
ggtttgtgca gcaccgccta cgcgggatac ggaaataccg gcattgattg caggacggat 2005260
accggcgttg aagaggtcgg tttccaagaa aatctgaccg tcggtaatcg aaatgacgtt 2005320
agtcggaacg aaagcagata cgtcgcccgc ttgggtttcg ataatcggca acgcggtcag 2005380
agaaccggtt ttgcctttta cttcgccgtt ggtcaatttc tccacttcgt gttcattgac 2005440
acgtgccgca cgttccaaca gacgggagtg caggtagaac acatcgccgg gataggcttc 2005500
gcggccgggc ggacggcgca aaagcaggga aatttgacgg taagccacag cctgtttgga 2005560
caaatcgtca taaacaatca aggcatcttc gccacgatcg cggaagaatt cacccatcgt 2005620
acaaccggag taaggtgcga tatattgcaa tgccgccgct tcagatgcag ttgcagcaac 2005680
cacgatggta tgctccatcg cgccatgctc ttccaatttg cggaccacgt tggcaataga 2005740
agatgctttt tgaccgatag cgacatagat acagataaca cccgtacctt tttggttgac 2005800
gatggcatcc aatgctacgg ccgttttacc tgtctgacgg tcgccaataa tcaactcacg 2005860
ctgaccgcga ccgacaggaa ccatagagtc aatcgccttc agaccggttt gcatcggctg 2005920

gtcaaccgat ttgcgcgcaa tcacgcccgg tgcgattttt tcgatagggg cggtcaaagt 2005980
tgtattaatc gggcctttgc cgtcgatagg ccgacccaat gcatcaacga cgcgtccgac 2006040
cagttcgcgt ccgaccggca cttccaagat acgaccggta caggtaaccg tgtcgccttc 2006100
tttaatgtgt tcgtactcgc ccaacactac ggcgccgacg gagtcgcgct ccaggttcat 2006160
cgccaagccg aaagtgttac ccgggaattc gagcatctca ccttgcattg catctgacaa 2006220
accatggatg cgaacgatac cgtcagttac cgaaattacc gtaccacagg tacgcacttc 2006280
ggcatttaca gacagatttt cgatcttggc tttaatcaaa tcgctaattt cagcaggatt 2006340
aagctgcatg aaaactctcc taattcgtca tagtcgtgta caaggcactc aatttgcctt 2006400
gtacagacaa atccaaaacc tgatcaccca cttcaacttt tatgccgcca atcagctccg 2006460
gttcgatttc gacagagatt ttcagctcgc tgtcgaaacg cttattcagc atttgcacca 2006520
actcgccgac ctgtttgtcg gtcaacggat aggcactgta aatgacggca gatttgatat 2006580
ggttgaatga taaggtcaag tcttgatatt gagcatatac ttccggcaat atcgacaaac 2006640
gtttctgccc ggccaagacg ataacaaagt ttttcaactc cttgtctttc aaaccgacca 2006700
aatcgatgag gatatctgct ttttctgaag cattcgtttc aggacggtca atcaatgaag 2006760
ccaccttccc ttcctgaaca accgccgcaa gtttttccag tccgcccaac caagactcaa 2006820
tttggttttt ttcctgagcc agaccgaaca atgcctttgc ataaggtctg gcaatcgttg 2006880
cgaactctgc cataagatta cagctcctgt ttcagggtat cgagcagttt tgcgtgtttg 2006940
gaagcatcga cttcgctgcg caaaatagat tcggcacctt tgacagccaa cacggcaacc 2007000
tgctcgcgca gggattcgcg tgcgcggaac aattcctgct ccacatcggc ctttgcctga 2007060
gctgcaatgc gcgccgcctc ggaagaagcc tgttctttgg cttcttcgac aattttggcg 2007120
gcacgttttt cggcgttggc aaccatttcg gaaacctgat tacgcccttc tgccaagagt 2007180
tctgcaacct ttttttcagc ctgctcaaaa tcgcttttac cacgctcggc ggcagccaag 2007240
ccttcggcga cttttgcggc acgctcatcc aaagcttttg caatcggcgg ccacacgaat 2007300
ttcatggtaa accataccaa accgaaaaag acgatgattt gagcgaataa tgttgcattg 2007360
atattcacgt tacttaacct tcgtactggg gttaatcaaa caggctgcgc ctgtacggaa 2007420
cggacgaatc cgtcctgatt atgcacctgc aaacgggtta acgaaggcga acagcagtgc 2007480
aatggcgaca ccaatcaaga atgcggcatc aatcaaaccg gcaatcagga acagtttggt 2007540
ttgcagcgga ccgatcagtt cgggctgacg ggcagaagac tccaaatatt tagaaccgac 2007600
cattgcgata ccgatagagg cacccaatgc acccaatgca acgatcaaac cacatgcgat 2007660
agcaatcaaa cccattttaa actccttaaa gaaacaaagg ttaaactaca aaaacaaact 2007720
acttaggaaa atcagtgcgc atcatgtgcc tgtccgatat agacgaacgc caacgccatg 2007780
aaaataaacg cctgcagggt aatcaccaaa atatggaaaa tcgcccatgc caaaccggca 2007840
ataatgtgga atacaaacag aatcggatcc atgacttcga cgctgccgga agccgcccaa 2007900
gcaccgccaa gcaaggctat caacaagaat accaattcgc ccgcatacat attgccgaac 2007960
aaccgcatac cgtgggatac ggttttagaa agaaactcga ccaaattcaa cagaaagttc 2008020
gcaggtgcga gttttgcacc gaacggcgcg ctgaacaact cgtgaaacca gccacccaat 2008080
cctttgattt tgatgttgta atagatacaa atcagcaaca cgccgacagc gagtgccaaa 2008140
gtggtgttca aatcggcagt cggtacgacg cgcagcaggg cgtgatggtt gccggtaatg 2008200
ccctgccata ccatcggcag caaatcgacc ggcagcatat ccatcgcgtt catcagaaaa 2008260
atccagacaa acagcgtcag acccaacggc gcgacggctt ttctagactt ttcgttgtga 2008320
atgatgctct tacacatatc gtccacaaac tcaaacaaga tttccactgc ggcctggaaa 2008380
cgtccgggaa cgcctgccgt cgcttttttt gcaccgcgcc acaacagaaa gctgccgatt 2008440
acgcccaaca ggacggcaaa aaagacggca tcaaggttaa taaacgaaaa atcagcaatg 2008500
tttttcagtc cctgaccctg agtaacatcc gacaaactgg tcaagctctg caagtggtgc 2008560
ttgatgtagt cggcagcggt aatggtttca cctgccataa tctttcactc tcaacaatac 2008620
taaaaaaacc aaatggctga caccgagcag ccccatcaga aacggggcga acaccagcga 2008680
ttgatgccat attgcaaata cggcaagcat ggacaacagc gacagcacta cttttaaaat 2008740
ctctccgaag acgaacatcc tgctttgcag gaaggggttt cccctgaaaa gttttaaaag 2008800
taaaactgca acaaacgtgg gaagcaggta ggacaaaccg ccaccgaccg ccgaaaggaa 2008860
tccggcaaaa ccccatacag caaaggcaac tgcggcgcat atggacaata cggcggattg 2008920
taggatgata atctgcttca taaagggaat gtttccgcct cggatttggg gcgcggctaa 2008980
tataatttag aagccttatt acgtcaagcg acagttaatc tttgtgaaac aacgtatccc 2009040
aatccgccgc gctcgccgcc tgaataacgg cgacaggtgt cattctaaca cacattacat 2009100
ataattacag gatattaagg agtttgtccg caatttcttt acatttttaa tgttcttacg 2009160
tgatttgttt gctttacgtg gaaataataa aaaatcaacg cgaaattgta gcagtttatc 2009220
ggtcggattg tcggcagttt ggggaatttg ctcaataaat aaaaggtcgt ctgaaaatat 2009280
tttcagacga ccttttccga ataaaggatt agcaactgcc tgccgcttta agcaaagcat 2009340
tgcattgact tttgcctttg tgcgttccgc ctcccaaaca aattgcatcg gaagtggtaa 2009400
cgccgattgt gctgattaca ctggtaacat agcattggct cacgcgctta ccccacagttg 2009460
cggtaaagtt gatgcgtatg ccttcattgt tgcggttgct gatttttacg gcatttgggc 2009520
tgacgcccaa ggcaaacgcg gcacgttcct gaagtttcta gtcggaaacg gttacattat 2009580

```
tgattgagcc gcaacctgct aatgccaacg caacgaacgc agccgaaacg atgatgcgtg 2009640
tgttcataat ttcctcgaaa attaaaaatg aaaacaggaa aacgattctt acgtgaagca 2009700
gaaaaaatgt caatagaatt atatttccca cttaaaatct ggaaagctat tctctatatt 2009760
tcagacggta tatcccgcaa aattaaggcc ggtaatctat gcccaactgc tccagcaggt 2009820
ggccgaacgt ttcaggcgta tcgaaataca ggacaatcct gcctttttttg tggttggcgg 2009880
ttttgacttc agcgttgaca cccagttttt cagtcagcaa atcattcagg cggccgatgt 2009940
cggcggcggc agtctttttg ggctcgggac gtttgttttg aagggcggcc tggctgcggc 2010000
gttcgacttc gcgcaccgac cagccgtttt tgacggcctt ttgcgccaat tcgagctgtt 2010060
cgacgacggg cagggtcagc aatgcgcggg cgtgccccat ttcgaggcgg cgttggtaaa 2010120
gcatttcctg cacgggttcg ggcaggctta aaaggcgcag gctgttggaa atcgcgcttc 2010180
ggcttttacc gacggcttgg gcgatggttt cgtgggtcag cccgaactcg tcggcaaggc 2010240
gtttcaagcc ttgtgcttct tcgatggggt tgaggttttc gcgctggagg ttttcgatca 2010300
aacccattgc caatgcggtt tcgtcgctga tggttttgat aacggcgggg atttcggtca 2010360
ggccggcaat ctgtgcggcg cgccaacggc gttcgcctgc aatcagttcg tatcgggaca 2010420
gtccgtgttc gcgcacgatg acgggctgta tcacgccttg cgccttaatc gaatctgcca 2010480
gttcctgcaa ggcttcgtca tcgatttgaa cacgcgcctg atagcggccg ggccggatat 2010540
ctttaaccgc aaccgtggtc aatcggtcgc cgctgctgtt gtccgcgccg ttggcgagca 2010600
gcgaatccaa gccgcgcccc aatccgcctt ttacttttgc cataccgccc tcccgtgcct 2010660
attcagatag gatgttaaat cgggtatttt atcggatatt gggtgttgcc gacaatttgt 2010720
atccgcgttt atcggatttc tgttttttca ctataatagc cggtttgccg ttgcaggcgg 2010780
ttttatggga aaggcggatg atggtacggc gtttgataat cggcatcagc ggggcgagcg 2010840
gtttccaata cggcgtgaag gctttggaac ttttgcgcgc gcaagatgtc gaaacgcacc 2010900
ttgtggtatc gaaaggtgcg gagatggcgc gcgcttcgga aacggcttat gcgagagacg 2010960
aggtatatgc cttggcggac ttcgtgcatc cgatcggcaa tatcggggcg tgcattgcca 2011020
gcggtacgtt taaaacggat gggatgctgg tcgccccctg ttcgatgcgg acgcttgcct 2011080
ctgtcgcgca cggcttcggc gacaatctgc tgacgcgtgc ggcggatgtg gttttgaagg 2011140
aaaggcggcg gctggtgctg atggtgcgcg aaacgccgct gaaccttgcc catttggaca 2011200
atatgaagcg ggtaacggaa atgggcggcg tggtgtttcc ccctgttcct gcgatgtacc 2011260
gcaaaccgca gacggcggac gacatagtgg cgcacagtgt tgcacacgct ttgtcgctgt 2011320
tcggaatcga tacgccggat tcggcggaat ggcagggaat ggcggattaa aggacaaaaa 2011380
tgccgtctga acacggatac agttcagacg gcatcatttt atacgactgc cttatttggc 2011440
tgcgccttca ttccatgcgg caggggattt gtagccctcg aagcgtttgt gcgcgtaggc 2011500
tttgaacgcg tcggagttat aggcctcggt tacgtcttta agccattggc tgtctttgtc 2011560
ggcggttttg acggcagacc agttgacata ggcaaagctc ggttcttgga acagggcttc 2011620
ggtcagcttc atgccgctgc ttatggcgta gttgccgttg acgacggcaa aatccacgtc 2011680
ggcgcggcta cgcggcagtt gcgcggcttc aagctcgacg attttgatgt ttttcaggtt 2011740
ctcggcgatg tccgctttgg atgcggtcaa cggattgatg ccgtctttga gtttgatcca 2011800
acccagttcg tcgagcatca ccaagacgcg ggcgaagttg gacgggtcgt tgggcgcgga 2011860
tacggtgctg ccgtctttga cttcttccag cgatttcagc ttgcccgggt acagtcccaa 2011920
aggcgcggtc ggcacttgga agacttcggt gatgtccaga ttgtgttctt ttttgaagtc 2011980
gtcaagatag ggtttgtgtt ggaagacgtt gatgtccaac tcgccctcag ccaatgccag 2012040
attcgggcgt acatagtcgg taaactcgac cagtttgacg gtgtagcctt ttttctccag 2012100
ctcggcttgg atttgttctt tgaccatatc gccgaagtcg ccgacggtcg tgccgaagac 2012160
gatttctttt ttcgccgcgc cgttgtcggc ggcggcagaa gcggatgcgg cgggcgcgct 2012220
gtctttttga ccgccgcagg cggcgaggat gagcgcgagt gcggcggcgg aaagggtttt 2012280
gaagaaggtt ttcatatttt ctcctgatgt tgtggcagtt tcaaacaaaa atgacgggca 2012340
gggagtcctg ccgtccggat tcggcgttca gacggcattt gccgcgaaca ggggggatttt 2012400
atagcatttt tcggatagcg gtggggggttt tggcgttcag acggcattcg ggttcaacgt 2012460
ttgtcgagtt tccgcgccaa cgcgttgccg gtgctttgaa tcaggatgac cagcagcacg 2012520
aggagggcga cgatgaagat gatgacttcg gtttggtagc ggtagtagcc gtagcggatg 2012580
gcgaggtcgc ccaagccgcc gccgcctatc atccctgccg ccgcgctgta tgacaaaagc 2012640
ccgatggcaa gcacggtaat gctggaaacc atgcccgcgc gcgcttcgtt caagaggact 2012700
ttgcagacga tggcaatcgg cggcgcaccc atcgcggcgg cggcttcaat tacgcctttg 2012760
gggacttcgc gcaggttttg ttccaccagt cggcaaaat aaaacaatcc cgacacgctc 2012820
aacaccagcg aggcggcaac cggaccgatg gtgctgccga cgatggcgcg tgtggcgggt 2012880
atcatcgcaa tcatcaggat gacgaagggg aaggcgcgca tgaggttgac gaggttgtcg 2012940
agcaggaagt tcaccagctt gttgtaatgc agttggcggc tggaggttac gaagagcagc 2013000
acgcccagca gcgtgccgaa gatgacggcg aatgtggtgg acaagccgac catcacgaag 2013060
gtttcgccca aggcgcggaa gatttcgtct ttcatgccga cgatggtgga aacggcttgt 2013120
tggaatgtta agtctgccat atcagtcctc ccgaatcagt tcgcgcccga tgtcggattg 2013180
ggcgtggatt tggttgccgc gtacttcgac gatttcgacg actttgcctt tatccaagag 2013240
```

ggcggcgcgg tcgcacaggc ggcggatgac gctcatttcg tgggttacga tgacgatggt 2013300
tacgttgaag cgtttgttga tgtcttccaa acattccaag acgctgcgcg tggtggcggg 2013360
gtcgagggcg gaagtgggtt cgtctgcgag gatgacttgg ggtttgggcg cgagtgcgcg 2013420
ggcgatgccg acacgttgtt tctgcccgcc ggaaagctgg gcgggatagt ggccggcgcg 2013480
ttcggtcaag ccgacgattt caaggcattc tttaacgcgc gctttgattt tttcagacgg 2013540
ccatccggcg atttccaaag gaaaggcaac attgtcggca acggtgcggt tgctcaaaag 2013600
attaaactgc tgaaacacca tgccgatatt ctgccgagcc tgacgcaatg cggcggcatc 2013660
gagcgcggtc agctcttgtc cgcagacgtt gaccttgccg ctgtcggggc gttccaacag 2013720
gttaatcagg cgcaacaggg tggatttgcc tgcacccgaa taacccatca gcccgaagat 2013780
ttcgccgtcg cggatttcga ggctggtcgg ctcgacggcg gcaaaacggg tcttgtcgcg 2013840
cgtttggtaa tgcttggaaa ccttgtccaa aataatcatt gtctttccca tacaacaaag 2013900
cccgatgtcg gacacaacgg gcgcggaaga taaagctgaa attgtcggaa cgctttagct 2013960
gttatgcccg caagctgtgt caaatcggca ggttaatttt cgtaggatat tatcgggaaa 2014020
gcattttttt gtcaataaag caggaagcgg gcaaccattt cggacaatgc cgtctgaaac 2014080
gggcaaaggc agcggttcgc accaaaacgg caaataattg aaaaacatat agtggattaa 2014140
caaaaatcag gacaaggcga cgaagccgca gacagtacaa atagtacgga accgattcac 2014200
ttggtgcttc agcaccttag agaatcgttc tctttgagct aaggcgaggc aacgccgtac 2014260
tggtttttgt taatccacta taaattatgt cggaaacatt ccaaaggcgg tgcagtttcg 2014320
gcatataatt cgggcaaacg cctgttcaga cggcattttg tcttttccaa ccctgaccgt 2014380
tcagggttcc gattcttaag gaaatccgat gtacctaccc tctatgaagc attccctgcc 2014440
gctgctggcg gccctggtgc ttgccgcgtg ttcttcgaca aacacactgc cagccggcaa 2014500
gaccccggca gacaatatag aaactgccga cctttcggca agcgttccca cccgccctgc 2014560
cgaacccgaa agaaaaacgc tggcagatta cggcggctac ccgtccgcac tggatgcagt 2014620
gaaacagaaa aacgatgccg ccgtcgccgc ctatttggaa aacgccggcg acagcgcgat 2014680
ggcggaaaat gtccgcaacg agtggctgaa gtctttgggc gcacgcagac agtggacgct 2014740
gtttgcacag gaatacgcca aactcgaacc ggcagggcgc gcccaagaag tcgaatgcta 2014800
cgcccgattcg agccgcaacg actatacgcg tgccgctgaa ctggtcaaaa atacgggcaa 2014860
actgccttcg ggctgcacca aactgttgga acaggcagcc gcatccggct tgttggacgg 2014920
caacgacgcc tggaggcgcg tgcgcggact gctggccggc cgccaaacca cagacgcacg 2014980
caaccttgcc gccgcattgg gcagcccgtt tgacggcggt acacaaggtt cgcgcgaata 2015040
tgccctgttg aacgtcatcg gcaaagaagc acgcaaatcg ccgaatgccg ccgccctgct 2015100
gtccgaaatg gaaagcggtt taagcctcga acaacgcagt ttcgcgtggg gcgtattggg 2015160
gcattatcag tcgcaaaacc tcaatgtgcc tgccgccttg gactattacg gcaaggttgc 2015220
cgaccgccgc caactgaccg acgaccaaat cgagtggtac gcccgcgccg ccttgcgcgc 2015280
ccgacgttgg gacgagctgg cctccgttat ctcgcatatg cccgaaaaac tgcaaaaaag 2015340
cccgacctgg ctctactggc tggcacgcag ccgcgccgca acgggcaaca cgcaagaggc 2015400
ggaaaaactt tacaaacagg cggcagcgac gggcaggaat ttttatgcgg tgctggcagg 2015460
ggaagaattg ggtcggaaaa tcgatacgcg caacaatgtg cccgatgccg gcaaaaacag 2015520
cgtccgccgc atggcggaag acggtgcagt caaacgcgca ctggtactgt tccaaaacag 2015580
ccaatctgcc ggtgatgcaa aaatgcgccg tcaggctcag gcggaatggc gttttgccac 2015640
acgcggcttt gacgaagaca agctgctgac cgccgcgcaa accgcgttcg accacggttt 2015700
ttacgatatg gcggtcaaca gcgcggaacg caccgaccgc aaactcaact acaccttgcg 2015760
ctatatttcg ccgtttaaag acacggtaat ccgccacgcg caaaatgtta atgtcgatcc 2015820
ggcttgggtt tatgggctga ttcgtcagga aagccgcttc gttataggcg cgcaatcccg 2015880
cgtaggcgcg caggggctga tgcaggttat gcctgccacc gcgcgcgaaa tcgccggcaa 2015940
aatcggtatg gatgccgcac aactttacac cgccgacggc aatatccgta tggggacgtg 2016000
gtatatggcg gacaccaaac gccgcctgca aaacaacgaa gtcctcgcca ccgcaggcta 2016060
taacgccggt cccggcaggg cgcgccgatg gcaggcggac acgcccctcg aaggcgcggt 2016120
atatgccgaa accatcccgt tttccgaaac gcgcgactat gtcaaaaaag tgatggccaa 2016180
tgccgcctac tacgccgccc tcttcggcgc gccgcacatc ccgctcaaac agcgtatggg 2016240
cattgttcct gcacgctgac gtaccgatgc cgtctgaaac ccgcccggtc tttcagacgg 2016300
catttttatc ccgaacggca ttgacggcga accataaata taagacaatc cgaaaattgt 2016360
ttttcctgct ttttcaagca gcttgacacg gcacaagccg acccgttagg aggtgatgtt 2016420
tccgtcacgg cgcgtatccc gccgccgcaa ggcacagcga tacggtaaac tttcaacacc 2016480
gtctgcccta ccctttccac cgatatgatg ggcagatgaa acaaccgaat ttattaaagg 2016540
aaataaaatg cctgcaatcc gcgtaaaaga gaatgaacca tttgaagtcg ctatgcgccg 2016600
tttcaaacgc gccgtagaaa aaaccggcct gctgaccgag ctgcgcgccc gcgaagccta 2016660
cgaaaaaccg actaccgaac gcaaacgcaa aaaagcggca gccgtaaaac gcctgcaaaa 2016720
acgcctgcgc agccaacaac tgccgcccaa aatgtactaa acgttcaagt acagattaca 2016780
ggtcagccct gtgatatgag gacacaccgc aagacctgct ctgcggtgtg ttttgctttt 2016840
cagacggcat cgaaacccgc cgtttccatc cgacatccca gcgaggacat catgagcctg 2016900

aaaatccgcc ttaccgaaga catgaaaacc gcgatgcgcg ccaaagacca agtttccctc 2016960
ggcaccatcc gcctcatcaa cgccgccgtc aaacagtttg aagtggacga acgcaccgaa 2017020
gccgacgatg ccaaaatcac cgccatcctg accaaaatgg tcaaacagcg aaaagacagc 2017080
gcgaaaatct acactgaagc cggccgtcag gatttggcag acaaagaaaa cgccgaaatc 2017140
gaggtactgc accgctacct tccccaaatg ctttccgccg gcgaaatccg taccgaggtc 2017200
gaagctgccg ttgccgaaac cggcgcggca ggtatggcgg atatgggtaa agtcatgggg 2017260
ctgctgaaaa cccgcctcgc aggtaaagcc gacatgggcg aagtcaacaa aatcctgaaa 2017320
gccgtgctga ccgcctgatt gcccgaatat cggacaaaat gccgtctgaa gcccgtatcg 2017380
caggttcaga cggcattttc aatatcccaa tatcgaatcg gcaggggcaa cacggttttg 2017440
atacgccgaa acgggttttg ccgataaaca gattccgttt gcgcccccatc ggacaaaatg 2017500
ccgtctgaaa cacgattccg ttcagacggc atagatttat ttgaccaatt tcaagccttt 2017560
tttggcgggt cggggcgcgg tttcggcaga ggtgttttca ggcggcgtat cggggcggta 2017620
cgcttccaac tcaaacccca taccttctcc ggtctcccgt gcgaaaaggc tgaggacgtg 2017680
tccgacaggt atccatatat cgtgcgcctg tccgccgaag cgggcggaaa agctgatcca 2017740
atcgttgtcg atttgaaggt tttgcgtggc ggtcgcgccg atgttgagca taatttcgtt 2017800
gtcgcggacg tactgcatgg ggacgcgcgt gtgttcgttg acccagacaa ggatgtgcgg 2017860
tgtgaggctg ttgtcgctgc accattcgca gagggcgcgg aggatgtagg gtttggtgga 2017920
agtgggcata atgggttccg tgttgtacgc caaaatagga aaatgcctgc aaaacggtgg 2017980
gttttgcaag catttcggac ttatttgcgc atggcttttt cggcgggtgt cagtgcttcg 2018040
ataaaggctt cgcgctggaa gatgcgctcg gcgtatttga gcagcggcgc ggcacttttg 2018100
cccagtttga catcgtagtg gtcgagccgc cacagcagcg gagcaagggc gacatcaatc 2018160
atagaaaaat cttcgccgag gatgtatttg cttttgctga acgaaggggc aagcatggtc 2018220
agaccgttgc cgatggcttc gcgcgctttt gcctgttcct tgttggtggc ggcggggttt 2018280
tctaacactt ggacgtggtt gaacaattct ttttccatac ggtacagcac cagccggccc 2018340
cgaccgcgca taacgggatc gccgggcatc agctgcggat gggggaagcg ttcgtcaatg 2018400
tattcgttga tgatattgga ctcgtgcagc accaaatcgc gctcgaccag cacgggaact 2018460
tggttatacg gattcatgac ggcgaggtct tcgggtttgt tgtaaatatc gacgtctttg 2018520
atttcaaaat ccataccttt ttcgtacaaa acgaagcggc agcggtggct gaaggggcag 2018580
gtaatgccgg aatagagggt catcataata attgtcgctc ctgtgtgatg cctgcaaaac 2018640
ggctgattta tagtggatta acaaaaacca gtacggcgtt gcctcgcctt agctcaaaga 2018700
gaacgattct ctaaggtgct gaagcaccga gtgaatcggt tccgtactat ttgtactgtc 2018760
tgcggcttcg tcgccttgtc ctgatttttg ttaatccact atataaaggt ttaatcgcgc 2018820
aattatacgc gatttccggc acttaatcca gaaattcggc tcaatctgtt gtttttttata 2018880
tattttcccc gattttccgt atcagtgcga acttactgtc tttgttgcgc ggacgcgcac 2018940
cctgccaaac cgtctgccag ccttgcggcg catccgcatt ttggggcagg aggacgatgc 2019000
ggtagcggca ttgtacatcg ccgacgcggt gcggcaatgt gccgtactgc gtccaaacaa 2019060
tccgcgtgtg caggtcgccg ccgcctatgc cgatacactc gatgccgtct gaaagctccc 2019120
gtttcaattc cggggaaagc gatgcctcca tactccggac gaccggcgcg tggcttttcg 2019180
ccgcgtccag ccacggcagg aacagcgtca tcagcaaagc ccaggtcagg gtaacgcctg 2019240
ccgcccagtt ggtaaccgcc tgcctgccgc gtatgttttt ccgggtaatc gcccacagcc 2019300
acaagggtgt gaacagtacg gcaaccgcca tcggaatggg atcgatatca ggaacataat 2019360
acgggctgaa ataggcggcg cgttcggcaa gcttggcggg ccagccgtaa ttcatggcga 2019420
aaaagcccgt ccacaggaac acggcaaaca gtccgaacgc cataatgccg aaccagttga 2019480
caaacgccgc cgcgccgcgc ctcaggctgt ccagttgcgc cgcgccgaac agggcaagcg 2019540
gcggaagcag ccagacgagg ttatcctgaa aacgctgcgg attgacggca agcagcacca 2019600
aaacggcaag catccagacg acgcccaaaa tcccccagtc ggtcgaaaac aggcgcgtgc 2019660
ggcaaaccgt ccaaaccgcc agcggcagcg cgggcaatgc aaaccaaagc aggtttttca 2019720
gatagtaaaa caaactgaat gccgtctgaa cgtgccgcac gccgccgaac gtaccgaaaa 2019780
cgtgatagtc gagccattgc gcgaacagcg cgggctgcgt ttttgccaag agcagcgggt 2019840
aaacggtcat aagcggcagg gcaaaggcaa gtgaggcgac tgccgtcaac atcaaacgcc 2019900
tgctttgcca cggacggaaa aacatcagta cgggcaaggg cagcatcagg gcaaatgctg 2019960
ccggataagc tgctgccaac gacatcagcg tccagcccgt accgagcaga aaagaggcgg 2020020
caatcacgcg ccggcgagcc aaagaataac cgtgcagcac cagtccggcg gcggcaaagg 2020080
cggcggcagc ggggttgagg aaatgggcaa ctggaatcag cccgatacag ccgatgagaa 2020140
tcaggacgac gctgcgcccg tggtgtctgc ccaaaaagtt gaaaccggca aagccgcagg 2020200
aagtcagtcc gataacggca aaaaatacgc ctgcaaagcg tgcggcatcg tatgagtcgg 2020260
cagcccacgg cgacagcaaa tgtttgaacg cggcggcaac ccaaagatac acgggcggta 2020320
tgccgaaatc ggtttgaccg aacagatggg caaccaaggg ggtggggctg cctgccagtg 2020380
cttcgacggc ggtatagacg gcaggttcgt caggattcca caaatcgtgg gaaaacacgc 2020440
cgggccacaa ccaggcaaac gccatcaaca gcagcagcca cggcttttcg tgggttttgg 2020500
cgggcgggcg ggcatcgggc ggggtatagg tcagcataag cgtgagaaaa aatggacgga 2020560

ttgccaagtg tagcaaatat tcgcacaaag gtcgtgcaga gactgcttca gacggcatca 2020620
gacacaaaaa gaccggcaac aaaaaagact gcacatggca gtctttgcag atactatctt 2020680
tttcataata ttttttccta gcccaacaca gtccatagca tatcaaaact gttgttttca 2020740
accaggatat atatccccaa tcctaaataa acaacagcaa caaaccatct gctatatttt 2020800
tccaaagttt ctccaacaga agggacttgt gctaattttt gggcagaaaa aaccaagaga 2020860
taaatcatga ctagaaaggt aagtaaagcc actatcaaat tcgctaaatt taaggtagta 2020920
aaatatggga caaagacacc aatattgtca gcaccacaac ttgcaaaagt aatcatagcg 2020980
actagaaaaa tcaggttttt attatctttg cgcaaaccct ctttggcaat agcctctcca 2021040
tcagaatctc ctaaaagcaa aactttgatg cctaggagaa ttggaatcaa gccgagcaaa 2021100
cctaaaatct ctttactagg aatataatct aagacaaatg caaaaagtaa acttagcaat 2021160
atcagactaa cagagcctag aaattgtcct aaatagatgt taatgatgtc ttttctactt 2021220
tttcttttgg caaaaaataa cattaggata ataagtaagt ctacggctgt cccagaatac 2021280
aggattattg aagtaacgac attttgaatc ataaaacatc tcattcaaat atatttttaa 2021340
atgtattcaa acattaaacc ttgtagatgt caacttcaac cccgtcaaaa tatagtggat 2021400
taacaaaaac cagtacggcg ttgcctcgcc ttagctcaaa gagaacgatt ctctaaggtg 2021460
ctgaagcacc aagtgaatcg gttccgtact atttgtactg tctgcggctt cgtcgccttg 2021520
tcctgatttt tgttaatcca ctatatagat aagaagtcag tgtgccaaat attaaaaagc 2021580
cctgccatcg aaatgatggc agggcttaat tcttgcaaag cggcaatcag cgtttgaaca 2021640
ggttgccgaa tttgttgttg aatttgtcca cgcggccggt ggtatcaacg atttttttggg 2021700
tgccggtata gaacgggtgg cacagggagc aaacctcgat attgaagttt tctttttcca 2021760
tcgcggattt ggttgcgaat ttgttgccgc aagagcaggt aacgttgact tcgtggtagt 2021820
tcgggtgaat accttgtttc atttgatttc ctttcaaaaa agcgggcata ggggatgtac 2021880
ctatgctaca gacaagtccg acattctcgc tattttctgt tgttacgtca agagtatatt 2021940
cgataaaatg tatagtggat taacaaaaac cagtacagcg ttgcctcgcc ttgccgtact 2022000
atctgtactg tctgcggctt cgttgccttg tcctaatttt tgttaatcca ctataaaaag 2022060
ttcttttgag ggaggtttga tgggatcaaa attctttttc ctgctgctgc gttttgccgg 2022120
ttcggggttg ccgccgtcac atatgcgcgg catcggcatc gtcggcagac gggtgcgcgg 2022180
ttttttggcg cggcgggttt ctccgcatat cggacgcggg gtcaatatcg aacgcggggc 2022240
gtatgtgttt ccggatacgg ttttgggcga cggctcgggc atcggggcaa actgtgaaat 2022300
ctgccgtggg ctggtggtcg gcaaaaatgt gatgatggag ccggaatgtc tgttttattc 2022360
aaataaccac aagtttgacc gttcaaaaaa cgctttgagg gctacacgga aatccgtccg 2022420
attacgttgg aggacgatgt ctggccgggg cacagggtga ttgtaatggc gggcgtaacc 2022480
gtcggacgcg gttcggtcgt gggcgcagcg cggtggttac aaaagacatt ccgccctact 2022540
ctttggcggc aggcaatccg gcagtggtga aaaagaatct gccggaaggt tgaatgccgt 2022600
ctgaacgtgt cggggcggat gatctgaaaa aacaggaaca tcgtttctgt tttttgcgct 2022660
tcagacggca tcgctattgc gccacgcggt atcgatttct tggtagagtt tgccgaaatc 2022720
gggttcgccg acgtaggttt tgaggatttc gcctttttttg ccgataagga cggaagtcgg 2022780
ataaacctgt gtgccgaacg cctgtccgac agctttgtcc gcatcataca tgacggtaaa 2022840
cggcaaaccg tagtctttga catattggcg gacgctttct atcggatcga tgggctgggc 2022900
gacggcaagt acttggaagt ttttgttttt atagtcattt gccgttttaa tgattttggg 2022960
catttcgctc acacaacccg gacaggaggg aaaccaaaaa ttaatcaggg ttactttgcc 2023020
ttgcaggtcg gcgttggaaa cggtttttcc gtgcaggtcg ggcagggaga aggcgggcgc 2023080
ggttttgctg tcggggatga ggacgatggc aaggaggatg ccgatcagtg cgacgacggc 2023140
ggcggtgagt atttttttca ttcggacaag gcttccaatg cgcgggcaag ggtggcgggc 2023200
aggctgacgg tgcgttgtgt ggcggcgtgg acgggcatca gggtgatgtc ggcttctgcg 2023260
gcggttttgc cgtttggcag tgtaatcgtc tgggtcagca caatacggcg cgtgccgggg 2023320
gttttcaggc ggcatgaaaa ctgcaatacg tcgccttcga cggcggggcg gctgtatcgg 2023380
atgtcgatgc gggcgacaat cagtatgagg cctgccaact cgtgcagcag tccgcgttct 2023440
tcaaaaaacg cccagcgcgc ttcttcgaaa aattcgaggt agcgcgcatt gttgacatgg 2023500
ccgtagccgt cgagatggta gttgcggacg gtcagcttca tcagttcagg ttgatgggtt 2023560
ggaaggcttc gcgggcaagc ggttcgtgtt cgaggtcggt gatgacggta gaaagctgga 2023620
tgtcgaacca ttcgttgaaa atgtcggcat cgagcgcagg ccactcgcgt tcgtcttcgc 2023680
accagtcggc aagttcggcg gcgaaaatgt cttcaaaacg ggcttcgatt tcgtcccata 2023740
cttcgtcggc ggtttcgcac gggcggacaa ggtaggaatt ggcgtcggct tggatgtctt 2023800
caagagtcag tccgtcgagg tggttgcccg gcagggtttg cagccagttc caaaaaggtt 2023860
ctaaagggat gaggacgaat acgctgcggt tgacttcgta catggttttt cctttgctgt 2023920
cgcgcggtat gcgcaaaaaa gagattatag cccaatctgt ggtttcggac tgtccgttcc 2023980
gacagaaggg aatgccgtcc gaacacggat tttcagacgg catggcttta aggttgtgtt 2024040
ccaggttgcg tttcggcttc ccctgctgct tctgcctgtg tttcggatac ggaatcttct 2024100
tgaacggcag tttccgccgc gccggtttcg gcactttcga ccaattcgtc gatgtcgatg 2024160
ttatcttccg taccttcggc aggtgttgca ccggtctgcc gcgcacggac tttcatatag 2024220

aggtcgcgcg tgtagctgta tttgtcgatg gcggcttcgt ccagactgtc ggtcaaatcg 2024280
agcaggcctt cgcgcgtact gacggcggat acggcagtcg tgccccagcg tccgacaggg 2024340
gtgcggaaga cgatattctt gggcgaataa acggaggtaa tacccgtgcc gagcgcgtcg 2024400
cggacggtgg acggccctaa gacgggcaac acgaaataat tgctgttttt ccatccccac 2024460
gaggcaaacg tgtcgcccaa ggtgttttta ttgtcgggaa tgccgcccgc gccggcgatg 2024520
tcgataagcc cgcccaaacc gaaagtggtg ttgatgccga cgcggacaag gtcttcgctt 2024580
gcgcgtttga tgtccaagcg caagatattg ctgccgaagc tgaccacgtc gcacaggttg 2024640
ttaaaaaaat tggacacgcc ggcgcggacg ggtttcggcg caactttgcg gtagccgcgc 2024700
gcggcagggg cgaaaatgta gcggtcggct tggtcgttga atttgaaaac ggcgcggttg 2024760
tagccttcat aagggtcggc ggggcgggtt tcggcaaatg caggggcgga agcgaacccg 2024820
atcagcagga ggaaggcata ggcggttttt ttcatgattt cagccagtct ttgatttcgt 2024880
acagttcgga cagcgcgcgc acggattcgg gaatgccggt cagcctgacg ctgcctttgc 2024940
aaccgcgcag cacttcgagc agcagcgaca cgcaggcgga atcggcgcgt ccgacgccgc 2025000
tcaaatcaac cgcgcaggtg tctttcagac gacattgctg tctgaagcgg gtaaaagcgg 2025060
cggcggtcag ggttttgacg gtgatgtcgc cgccgatgtg caatattccg tttttgagtt 2025120
ctgtatgcat agcgtttgct cggaaaaccc ataccgccct cggacggtat ggtttgtcgg 2025180
ttatttgccg ccgtttttgg ctttcaactc ggcaatcagt ccgtccacgc ctttcgcttt 2025240
gataatttcg ccgaattggt tgcggtacac ggtaaccagg ctcgcgcctt cgatggcgac 2025300
gttgtaggta cggtatttac cgccgctttg gtaggtggtg aagtccatgt tgacgggttt 2025360
ttgcccgggt acgccgactt cggcgcggac gatgatttct ttgccgcctt tattgacgat 2025420
gggattgtct ttgacgttga cgttggcgtt ttttaatttc agcatcgtgc cggaataggt 2025480
gcggatcagc agggtttgaa attctttggc caacgcttgt ttttgcgcgt cggacgcggt 2025540
gcgccaaggg ttgccgaccg ccaatgcggt catacgttgg aaatcgaaat agggaatcgc 2025600
ataggcttcg gctttttggc gagcggtgtt ggcatcgccg ttttttaaga tgctcaatac 2025660
ttgagtggcg ttttgacgga tttggcttac cgcgtcggca ggggcggcaa atgccatgcc 2025720
gatgctcaaa ataccgatgc ccaatgcgct gatgagggag gatttttttca tgattaagtg 2025780
tcctagtttg aatatgatgg catacgttta ttcggcggct ttttccgcat tgccgccgtc 2025840
ggcatttttc tcggcaaaac tcgtcatgaa tttgccgata aggttttcca gaaccattgc 2025900
agaactggtt acggagatgg tgtcgccggc agcaaggttt tccgtgtcgc cgccctgctg 2025960
cagcccgatg tactgctcgc ccaaaagtcc cgaagtcagg atttgcgcgg aaacgtcgct 2026020
gctgaactga tacttgccgt ccaaatcgag gcgcaccctc gcctgatagg atttcgggtc 2026080
aagtccgata gcgccgacgc gcccgaccaa tacgcctgcg gatttgacgg gggcattgac 2026140
cttcaaaccg ccgatgtcgc cgaaatcggc ataaacggcg taagttttgt ccgaaccgcc 2026200
gaacgccgca ccgccggcca cgcggaaagc gagaaaggca accgccgccg cgccaatcag 2026260
gacgaacagt ccgacccaaa attccaatat gttctttttc attaaagttc cttgaatatc 2026320
cgatgttccg cgtttcgtct tcagacggcc tgtcaatctg taaacatcca cgcggtcaat 2026380
ataaaatcga ccgccaaaat cgtcagggcg gacgaaacca ccgtgcgcgt gctggcgcgc 2026440
aaaatgcctt ccgaagtcgg gacgcaatgg aagccctgat gcacggcaat cagcgttacc 2026500
gccacgccga acgcggcgga tttgatcaga ccgttgatta catcgtaatg tatcgtgatg 2026560
ttgttctgca tttgcgacca gaaaataccg ctgtccaagc ccagccaggt tacaccaacc 2026620
aaatacgcac cgaaaatgcc cgccacgttg aaaatcgaag ccaaaagcgg catggaaaac 2026680
acgcccgccc aaaagcgcgg cgcaaccacg cgggcgacag ggtttaccgc catcacattc 2026740
atcgcttcga gctgttcggt cgttttcatc agaccgattt cgctggtcat cgcaccgccc 2026800
gcgctgctgg caaacaaaat cgctgccaat accggaccca gctcgcgcaa tagcgaagcc 2026860
gcgaccatat agcccaaaat atcggcggat ttgaatttcg acaactgcgt atagccctgt 2026920
aaacccaaga ccatgccgac aaacagcccc gaaacggcaa caatcaacac cgacagcaca 2026980
ccggcgaaat acacttggcg cacgctcagg cgcggacgga cgaaagccgt accggacttc 2027040
gccagaatgt tcagcagaaa cagcgtgata ctgccgaggg attgaataag gccgagggtt 2027100
ttcgccccga cggaacggat aaagttcata aatttctatg tgtaaagttc aacggtttca 2027160
gacggcatca actcatttat cccaacaggt cctgctgcaa cgacgtttgc gccggataac 2027220
ggtatgctac ggggccgtct gccagcccgc cgacaaactg gcgcacccaa ggcgaatcca 2027280
gttcgcgcat ttcctgcggc gagccggaga acataatttc gccgtgcgcc aagaaaatca 2027340
cctgatcgac gatttccaaa gatttttcaa tgtcgtgcgt taccataata ctggtcgaac 2027400
gcaaagcctt gttgacgcgg ctgatcaagt gggcaatcac gcccaaggaa atcggatcga 2027460
ggccggtaaa cggctcgtcg tacaacataa tttcagggtc gagcgcaatc gtgcgggcaa 2027520
gcgcgacgcg gcgcgacatc ccgccggaca actcggacgg catcaggttt tccacaccgc 2027580
gcagaccgac cgcgttcaat ttcaacaaaa ccaaatcccg aatcaccgct tccggcaggc 2027640
gcgtcagttc gcgcatcgga aaagcgatat tgtcgaatac cgacaaatca gtaaacagcg 2027700
cgccgtgttg gaacaatacg cccatacggc ggcggtgttc atacaactcg tcagccgaaa 2027760
agcccgccaa atcccgtcct tcaatcaaaa cctgcccgga ctgcggacga atctgtcctg 2027820
taatcagtcg catcagcgtg gttttgccgc tgcccgaacc gcccattacg gcagcaaaat 2027880

tgccttgcgg aatgctgaaa ttgatgttct tcagaatcgg gcggtcgcca tacgcgaagg 2027940
cgacgtcttt catttcgata aagggggatg ggctcatgta cggacggacg gtaggtttga 2028000
cggcgtgtat tttaaggctt atcgggaaga cgggcaattt tcagacggca tacggacggt 2028060
aaatgttgtg aaaatgccgt tgtcggcggc ggattgtttg ctgtggcgaa aaatgttatc 2028120
tttcaaatga taacctttat cagaaaacta tggaaaaagc agaacatttg aacagcagcc 2028180
ggttcgtcaa tctagtcaaa agcggcggcg gcagctatgt ggagggcagc taccgtttcg 2028240
atactttgtc caacggcatt tccatccacg gcggcacagt aacggcacgg tgtgattttt 2028300
gcagcagccg cctcgccgaa ccttatgtgt cgttcgtgct cttgctggaa ggcagtttgg 2028360
acttcggcat caaccgctgc cgcttccaaa tcgatgcgga cggcggcaag attgtcctaa 2028420
ttgctgtcgg ggaagaagtc ctgttcagcc gctatcttta ccgaggcggc aaaacggtca 2028480
aaatgaccat taaaggtatg gaacaatggc tgctgcgtcc ggaatacgcg cgtttcgcac 2028540
ccctgcttta ccgcgaaccg gtcaggatat gggatttgcc cccgaacctg cgcggcttgg 2028600
cggcatcctg cctgaaagcc gtcccaaagg ggcatttggg cgaaacattg cgccgcgagg 2028660
cggacgtgtt gcggctgctg tcggacttgt gggacacggt ttcagacggc atcgggccgg 2028720
cggcggggca aacggcggaa gcagacgcta tgccgtctga agacttcagc cgcaccctaa 2028780
atgccgcgtt tgccgacggc gcacaccaag tcaaccggct gacagacgcg ctgaacatca 2028840
gtgaaaggac gctgcaacgc cgtatgcgcg accatttcgg cattacggca agcgaatggc 2028900
tgcaccacaa acaaatgcag cacgcgctct atctgttgca aaacggggga aaaagcatag 2028960
gcgaaaccgc atatttatgc ggctaccgcc acgtttccag ctttactcag gcattcaggc 2029020
aatatttcgg cagcacgcct gcggaaacca aaaaagaaaa ccggtaagcc gcatttgatt 2029080
tcaaacccga aatccgcgtg tatagtggat taacaaaaac cagtacggcg ttgcctcgcc 2029140
ttagctcaaa gagaacgatt ctctaaagtg ctcaagcacc aagtgaatcg gttccgtact 2029200
atttgtactg tctgcggctt cgtcgccttg tcctgatttt tgttaatcca ctataaaaaa 2029260
cttgtacggc aatttatttg ggaataaact aaaactacat ctaactacaa aactggagaa 2029320
cccgaaatga aacaattggc catgtacatc aacggacgct ttgaaaacga tttcaacggc 2029380
gaatggcgcg acgtattgaa cccgtccacc gaagaggcca tcgcccgcga acccaaaggc 2029440
ggcaaggcgg acgttgaccg cgccgtcgcg gcggcgcgtg cggcgcaacc ggcttgggag 2029500
cgtctgcctg cggtcgaacg cggcgcgtat ttgcgtaaaa tcgcccaagg catacgcgaa 2029560
cgtgccgacg agctgaccga caccatcgtt gccgaaggcg gcaaaaccaa agacttggca 2029620
cgcgtggaag tcatgttcac cgccgactat ctcgattatc aggccgaatg ggcgcgccgc 2029680
tacgaaggcg aaatcatcca aagcgaccgc ccgcgcgaaa atattttatt gttcaaacgt 2029740
ccgctgggcg taattgccgg cattttgccg tggaacttcc ccttcttcct gattgcccgc 2029800
aaaatgggcc ccgctttggt aacgggcaac accatcgtcg tcaaacccag cagcgtaacc 2029860
ccgatcaact gccacatctt cgccgaaatc gtcgatgcgg tcggactgcc cgcaggcgtg 2029920
ttcaacgtgg tgaacggtcc cggcgcggaa atcggcaatg ccttgtccgc ccatccgcaa 2029980
gtcgatatgg tcagcctgac cggctccgtc gaagcaggcc gccaagtgat ggaagccgcc 2030040
tccgccaaca tcaccaaagt ttcgctggaa ctcggcggca aagcgcctgc catcgttttg 2030100
aaagatgcgg atttggactt ggcggtgaaa tccatcttgg cttcgcgcgt cggcaacacc 2030160
ggtcaaatct gcaactgcgc cgagcgcgtc tatgtccaca gcagtctgaa agacgcattc 2030220
attgaaaaaa tgaccgccgc gatgaaaggc gtgcgctacg gcaaccctgc cgaagccgaa 2030280
gcaggcgcgc tggaaatggg cccgctgatt gaagaacgcg ccgtcaaagc cgttgccgaa 2030340
aaagtggaac gggcagtcaa acaaggtgcg aaattggttt gcggcggcaa acgcgccgaa 2030400
ggacgcggtt atttcttcga gccgaccctg ctgaccgaca ccgacaacag tatggacatt 2030460
atgaaagaag aaaccttcgg ccccgtgctg cccgtttccg ctttcgacac gctcgaccaa 2030520
gtcatcgcct tggcaaacga ttgcgagttt ggtctgacca gttctgttta tacgactaat 2030580
ttaaacgaag ccttctacgt tacccgccgc ctgcaattcg gcgaaaccta catcaaccgc 2030640
gaaaactttg aagcgatgca gggtttccac gccggttgga aaaaatccgg tatcggcggc 2030700
gcggacggca aacacggttt ggaagaatat ctgcaaaccc aagtcgttta tttggaaacc 2030760
gacatttaat gccgctttaa aaccccgata gaaaatgccg tctgaacccg ttttcaggtt 2030820
cagacggcat ttttattgct tcaccggcaa tcagtcatga ccgaggtcga tgtttttgtc 2030880
tttgtatagt ggattaacaa aaatcaggac aaggcggcga agccgcagac agtacaaata 2030940
gtacggaacc gattcactcg gtgcttcagc accttagaga atcgttctct tcgagctaag 2031000
gcgaggcaac gctgtactgg tttttgttaa tccgctatat tccgccatct ctaagattta 2031060
cagcgataca cgggtaattt aaggaatgcc caaaccgtca ttcccgccac ttttcgtcat 2031120
tcccgcgaaa gcgggaatct agaatctcgg actttcagat aatctttgaa tattgctgtt 2031180
gttctaaggt ctagattccc gcctgcgcgg gaatgacaaa tccatccgca cggaaacctg 2031240
caccacgtca ttcccacgaa cccacatccc gtcattccca cggaagtggg aatctagaaa 2031300
taaaaagcaa caggcattta tcggaaataa ctgaaaccga acagacctag attcccgcct 2031360
gcgcgggaat gacggctgca gatgcccgac ggtctttata gcggattaac aaaaatcagg 2031420
acaaggcgac gaagccgcag acagtacaga tagtacggaa ccgattcact tgttaaagaa 2031480
tcgttctctt tgagctaagg cgaggcaacg ccgtactggt tttgttcat ccactataac 2031540

tagggaaatt caaattaagt tagaattatc cctatgagaa aaagccgtct aagccggtat 2031600
aaacaaaata aactcattga gctatttgtc gaaagttcaa atttccattt taaaacaatt 2031660
agtaaaatcg agtttatcct agttgtccaa gacaacccct ataataatat aattcaaaat 2031720
ataaaaatgg gttacatcta aacattacgg aatttttatt ccctcgcctg aattctattg 2031780
tcagattcaa ggagacctca tcatgcgaac gaccccaacc ttccctacaa aaactttcaa 2031840
accgactgcc atggcgttag ctgttgcaac aacactttct gcctgcttag gcggcggcgg 2031900
aggcggcact tctgcgcccg acttcaatgc aggcggtacc ggtatcggca gcaacagcag 2031960
agcaacaaca gcgaaatcag cagcagtatc ttacgccggt atcaagaacg aaatgtgcaa 2032020
agacagaagc atgctctgtg ccggtcggga tgacgttgcg gttacagaca gggatgccaa 2032080
aatcaatgcc cccccccga atctgcatac cggagacttt ccaaacccaa atgacgcata 2032140
caagaatttg atcaacctca aacctgcaat tgaagcaggc tatacaggac gcggggtaga 2032200
ggtaggtatc gtcgacacag gcgaatccgt cggcagcata tcctttcccg aactgtatgg 2032260
cagaaaagaa cacggctata acgaaaatta caaaaactat acggcgtata tgcggaagga 2032320
agcgcctgaa gacggaggcg gtaaagacat tgaagcttct ttcgacgatg aggccgttat 2032380
agagactgaa gcaaagccga cggatatccg ccacgtaaaa gaaatcggac acatcgattt 2032440
ggtctcccat attattggcg ggcgttccgt ggacggcaga cctgcaggcg gtattgcgcc 2032500
cgatgcgacg ctacacataa tgaatacgaa tgatgaaacc aagaacgaaa tgatggttgc 2032560
agccatccgc aatgcatggg tcaagctggg cgaacgtggc gtgcgcatcg tcaataacag 2032620
ttttggaaca acatcgaggg caggcactgc cgaccttttc caaatagcca attcggagga 2032680
gcagtaccgc caagcgttgc tcgactattc cggcggtgat aaaacagacg agggtatccg 2032740
cctgatgcaa cagagcgatt acggcaacct gtcctaccac atccgtaata aaaacatgct 2032800
tttcatcttt tcgacaggca atgacgcaca agctcagccc aacacatatg ccctattgcc 2032860
attttatgaa aaagacgctc aaaaaggcat tatcacagtc gcaggcgtag accgcagtgg 2032920
agaaaagttc aaacgggaaa tgtatggaga accgggtaca gaaccgcttg agtatggctc 2032980
caaccattgc ggaattactg ccatgtggtg cctgtcggca ccctatgaag caagcgtccg 2033040
tttcacccgt acaaacccga ttcaaattgc cggaacatcc ttttccgcac ccatcgtaac 2033100
cggcacggcg gctctgctgc tgcagaaata cccgtggatg agcaacgaca acctgcgtac 2033160
cacgttgctg acgacggctc aggacatcgg tgcagtcggc gtggacagca agttcggctg 2033220
gggactgctg gatgcgggta aggccatgaa cggacccgcg tcctttccgt tcggcgactt 2033280
taccgccgat acgaaaggta catccgatat tgcctactcc ttccgtaacg acatttcagg 2033340
cacgggcggc ctgatcaaaa aaggcggcag ccaactgcaa ctgcacggca acaacaccta 2033400
tacgggcaaa accattatcg aaggcggttc gctggtgttg tacggcaaca acaaatcgga 2033460
tatgcgcgtc gaaaccaaag gtgcgctgat ttataacggg gcggcatccg gcggcagcct 2033520
gaacagcgac ggcattgtct atctggcaga taccgaccaa tccggcgcaa acgaaaccgt 2033580
acacatcaaa ggcagtctgc agctggacgg caaaggtacg ctgtacacac gtttgggcaa 2033640
actgctgaaa gtggacggta cggcgattat cggcggcaag ctgtacatgt cggcacgcgg 2033700
caaggggggca ggctatctca acagtaccgg acgacgtgtt cccttcctga gtgccgccaa 2033760
aatcgggcag gattattctt tcttcacaaa catcgaaacc gacggcggcc tgctggcttc 2033820
cctcgacagc gtcgaaaaaa cagcgggcag tgaaggcgac acgctgtcct attatgtccg 2033880
tcgcggcaat gcggcacgga ctgcttcggc agcggcacat tccgcgcccg ccggtctgaa 2033940
acacgccgta gaacagggcg gcagcaatct ggaaaacctg atggtcgaac tggatgcctc 2034000
cgaatcatcc gcaacacccg agacggttga aactgcggca gccgaccgca cagatatgcc 2034060
gggcatccgc ccctacggcg caactttccg cgcagcggca gccgtacagc atgcgaatgc 2034120
cgccgacggt gtacgcatct tcaacagtct cgccgctacc gtctatgccg acagtaccgc 2034180
cgcccatgcc gatatgcagg gacgccgcct gaaagccgta tcggacgggt tggaccacaa 2034240
cggcacgggt ctgcgcgtca tcgcgcaaac ccaacaggac ggtggaacgt gggaacaggg 2034300
cggtgttgaa ggcaaaatgc gcggcagtac ccaaaccgtc ggcattgccg cgaaaaccgg 2034360
cgaaaatacg acagcagccg ccacactggg catgggacgc agcacatgga gcgaaaacag 2034420
tgcaaatgca aaaaccgaca gcattagtct gtttgcaggc atacggcacg atgcgggcga 2034480
tatcggctat ctcaaaggcc tgttctccta cggacgctac aaaaacagca tcagccgcag 2034540
caccggtgcg gacgaacatg cggaaggcag cgtcaacggc acgctgatgc agctgggcgc 2034600
actgggcggt gtcaacgttc cgtttgccgc aacgggagat ttgacggtcg aaggcggtct 2034660
gcgctacgac ctgctcaaac aggatgcatt cgccgaaaaa ggcagtgctt tgggctggag 2034720
cggcaacagc ctcactgaag gcacgctggt cggactcgcg ggtctgaagc tgtcgcaacc 2034780
cttgagcgat aaagccgtcc tgtttgcaac ggcgggcgtg gaacgcgacc tgaacggacg 2034840
cgactacacg gtaacgggcg gctttaccgg cgcgactgca gcaaccggca agacgggggc 2034900
acgcaatatg ccgcacaccc gtctggttgc cggcctgggc gcggatgtcg aattcggcaa 2034960
cggctggaac ggcttggcac gttacagcta cgccggttcc aaacagtacg gcaaccacag 2035020
cggacgagtc ggcgtaggct accggttctg acggacagga agcagacagc cgcaaagatc 2035080
acggtctttg cggctgtttc ttatgaaaag aaaaccctat tccaattgcc tgcttctatt 2035140
gtttcaagac ttcttccaaa gattcggcat taatcagatg tatagcggat taacaaaaat 2035200

caggacaagg cggcgaagcc gcggacagta caaatagtac ggaaccgatt cactcggtgc 2035260
ttcagcacct tagagaatcg ttctctttga gctaaggcga ggcaacgccg tactggtttt 2035320
tgttaatccg ctatattcca ccatctctaa gatttacagc gatacacggg tgatttaagg 2035380
aatgcccgaa ccgtcattcc cgccactttc cgtcattccc gcgaaagcgg gaatctagaa 2035440
tctcggactt tcagataatc tttgaatatt gctgttgttc taaggtctag attcccgcct 2035500
gcgcgggaat gacgaatcca tccgcacgga aacctgcacc acgtcattcc cacgaaccca 2035560
catcccgtca ttcccacgaa agtgggaatc tagaaatgaa aagcaacagg catttatcgg 2035620
aaataactga aaccgaacag actagattcc cgcctgcgcg ggaatgacgg ctgcagatgc 2035680
ccgacggtct ttatagcgga ttaacaaaaa tcaggacaag gcggcgaaga cgcagacagt 2035740
acagatagta cgaaaccgat tcactcggtg cttcagcacc ttagagaatc gttctcttcg 2035800
agctaaggcg aggcaccgct gtactggttt ttgttaatcc actatacttg gagctggtct 2035860
tgcttttcgc ctaattctac gttttcaaac ggttgcagct ggtggtctgc cataaaggtc 2035920
tccttattgt atttcaggtt ggaaatcgga atttgttttc acaattttac accttcgccc 2035980
ccgctttctc tacataaaat tacattttgc cgatatttgc cgaattgtct gaaaatatgt 2036040
gtaataaggg gcgtataatc aaaacatttg ccccggattg ccatgcctta tttcgccctg 2036100
tttgacgatg ccgtaagcgg ccgcgcaaaa cgctatcaaa atcatgtgga aagccgtttt 2036160
ttccgtcccg aagaactcga tgctttggac ggcgcgctgc aatcgggctg gcaaaaaggg 2036220
ctgcattcgg tgttgtttgc agactacgga ttcggtttgc cgctgacggg ggttgagtcc 2036280
gaacgcggcg gcaatcttgc cctgcactgg tttgccaact gcgccgacat cgatgccgaa 2036340
agctggcttg cccgacactc agacggcctc cccgccggca tttccacgcc gcaaccctcc 2036400
gtatccgaaa ccgattacct cgaccgcatc cgccaaatcc acgaagccat ccggcgcggc 2036460
gacacctatc aaatcaacta caccacccgc ctgcacctgc aagcctacgg caatcccgtc 2036520
agcctctacc gccgcctgcg ccagcccgtc ccctatgccg tcttgtccca cctgcccgat 2036580
gcggaggggc aatccgcgtg gacgctgtgt ttctcgcccg aactcttcct caaaatcggt 2036640
tcggacggca ccatcagcac cgaaccgatg aaaggcaccg cgccgatttt gggcgacgga 2036700
caagacgaac gccgcgccgc cgagttgcaa gcagacccga aaaaccgcgc cgaaaacgtg 2036760
atgattgtcg atttgctgcg taacgatctc ggcaaaatcg cccaaaccgg cacagtatgc 2036820
gtacccgaac cgtttaaagt atcgcgtttc ggcagcgttt ggcagatgac cagcaccatc 2036880
caagcccaag ccttgccgca cacctcgttc gccgacatcc tccgcgccgc cttcccctgc 2036940
ggcagcatca ccggcgcgcc caaaaaaatg agtatgcaga ttatcgaatc gctcgaagcc 2037000
gaagcgcgcg gactttatac gggcagcatc ggctatttga acccgtgttc cggcggcttg 2037060
gggtttgaag gcacgttcaa cgtcgttatc cgcaccttgt cgctcacgcc gctttcagac 2037120
ggcatttatc aaggcgtgta cggtgtcggt tccggcatcg tcatcgacag cgaccccgcc 2037180
gccgaatatc gcgaatgcgg ctggaaagcc cgtttcctca acgaattgcg ccccgacttc 2037240
ggcatttttg aaaccctgcg cgcggaaaac ggacgctgca ccctgctcga ccgccaccta 2037300
tgccgtctga aaacctccgc ccaagccctc aacctgcccc tgcccgacgg ctgcgaaaat 2037360
caaatcaaac aatacattgc cgacttgccc gatggcgcgt tccgcgtcaa agccctgctc 2037420
gcttcagacg gcatcagcct gtcccgcgcc gttttaaacc gtctgaccga caaacaacgc 2037480
gtcatcattt cgcccgccgt cctgcccgca caaaactacc tgcgccgctt caaaaccacc 2037540
tgccgcgccc tcttcgacca agcgtggcaa accgccgaaa cacaaggcgc gttcgacagc 2037600
ctgtttttca attcagacgg catcctgctc gaaggcggca gaagcaacgt cttcatcaaa 2037660
catcgcggac aatggctcac accctcttta gatttagaca ttttaaacgg cataatgcgc 2037720
caagccgtat tggacgaacc gcaaaaatat ttgcaaacaa atcaagtaat cgaaacacac 2037780
atcacacaaa aaacactgca agaagccgaa gaaatccgcc tctccaacgc cttgcgcggc 2037840
gtatttgccg ccgcccttgc ctgaacgcgc aaaaatgccg tccgaacctg tttccaaagt 2037900
tcggacggca ttatcccacc attcaaaacc gccaatccgc cgacacaaac acctcgctgt 2037960
tgcggcgttt cgcatacggc acattacttt ccgtcctgcc gaaacgataa ttcaacgccg 2038020
gcacgatacc tttgtacgac aacttgtcgt ggctcaaagc cagcgagaca ttccattcgc 2038080
ggttgcgttg cgcctctgtc gagaaagccg caatgccctt atagttgcgg cgggcataag 2038140
acgcggaaac ccgactgttc aaaccgccca actgccgcca ctcctgcgcc caaccggcat 2038200
aaacaccgtt gcgccggtag gcggcattat tgaccgcgcc gcccaccgtt tcgcgtttcg 2038260
gcacaaaccg cacaaactgc cagccgccga acacagttgc cgattcgccc aaacgttttg 2038320
ccgacgaaac ataaaacccg tcctgcctgc cgttattgta ttccgcccta tcctgttcgc 2038380
ggtagcgttg gcggtaatgt tccagcgcga ccgaaaattg ccatcccggg tttgggcggt 2038440
aagtatggga cagctgcacg ccgactccgt gcgccagcat atacggcggc aggcggcggt 2038500
tgtttacccg ttttgttttc gcatcaaagc cgtcgctgcc cgacaactgc acctgataaa 2038560
acggcaaaat ccccgccgtc tgccgtgcat ttttatactg ccaacccaaa tacgccctgc 2038620
cgaacccgtc atcataagct gattttttac tgaaataata gctcgtgccg ccgatattgg 2038680
aacggaacaa caaataatga ttatctgcca acggcgtcag cttttccgcc tcgatttcat 2038740
aattcaaccc tgccgcccgc tccgcccggc tgacactgca tatctgccgg cctccgtttt 2038800
gccggcaata ttgcggcgcg gcattattgg catttctatt gaccgccgga ctgatgccgc 2038860

ccgaaaaacg ccagcccgtc agcccctccg tttttttccg aaaacgcccc acattttcca 2038920
aaaccggtgc cggcaaatcc aattttgccg cctccgcaaa atgcctttct gccgacttca 2038980
gccggaaatc gtcaaactcc gccgccgcca aatccagcaa aatccgctcg tctgccgcat 2039040
tttccccgtg cagttcccga taccgcgcca ccgcctccgc cggccttccc gccaatttcg 2039100
ccagcaaagc ccgcgccctg ccgtacaaaa ccgcgtcata atccggcagc ttggcataca 2039160
aatccgccaa cgaagcgatt aaatccgcct gattgccgtt gagcgcgtcg cgcaaactat 2039220
gttccaacat tttcggatgc gccaacaaaa aatccccgtc aaccacgcgc ggggcatcat 2039280
tttcaacttt ccaatctgat tccgcccact tatccgacac cgaccgctgc acctgcaaca 2039340
atgccttgtc atccaaaatc gcgggcgcat ccgccccata ggcggcagaa acacctgccg 2039400
cacaccaaac aaccaaaaag ccgtatctga aatacaacat accctgtcat ttacctttct 2039460
ggcaaacacg ccgccgaagc acgtcaaacc atccgaaaaa caggcagaaa cccgtgaaaa 2039520
ccggctttgc cgcctgaaag caggcaaaca aaaaccgccg cccgatttc aaagggcgga 2039580
tttcacattt atagtggatt aacaaaaatc aggacaaggc gacgaagccg cagacagtac 2039640
aaatagtacg gaaccgattc actcgtgctt aagcacctta gagaatcgtt ctctttgagc 2039700
tgaggcgagg caacgccgta ctggtttttg ttaatccact ataacagcaa ccctgtcgcc 2039760
gtcattcccg caaaagcggg aatgacgaag ctatccgcac agaaacctgc accacgtcat 2039820
tcccacgaaa gtgggaatcc agaacgtaaa atctgaagaa accgttttat ccgatacgtt 2039880
tccgcaccga cagacctaga ttcccgcttt cgcgggaatg acggcggaaa ggttgctgtt 2039940
tttccgataa attcctgccg ctcttcgttt ttgggatggc gggaaataaa acaaaagcgc 2040000
gcgtatcaaa aaacaaaaat gcaaagaacg gcgttggcaa ttttaaggtt tgcgccttag 2040060
ccgcacttat tcgcaaaaaa aaccgcacgg cgttgaccgt gcggtttttt atctgaaagc 2040120
ttcagacggc attgcttaca tcatgccgcc cataccaccc atgccgccca tatcaggcac 2040180
agccggtttg tcttcgggga tttcagcgat catgcaatca gtggtcagca tcaagccggc 2040240
gatagatgcg gcgtgttgca gcgcagaacg ggttactttg gcggggtcga gtacgcccat 2040300
ttcgatcata tcgccgtatt cgccgctgcc agcgttgtaa ccgtagttgc ctttgccttc 2040360
caatactttg ttcacaacca cgctgggttc gccgcctgcg ttggcaacga tttggcgcag 2040420
cggagactca acggcgcgca agacgatttg tacgcctgcg tcttggtcgg cattgccggt 2040480
gtgcaggttt tccaaagcag cacgggcacg caacagggct acgccgccgc ctgcaaccac 2040540
gccttcttca acggctgcgc gggtagcgtg cagcgcgtct tccacgcggt ctttttttctc 2040600
tttcatttcg acttcggtcg cggcaccgac tttgatgact gccacgccgc ctgccaattt 2040660
agccacgcgc tcttgcagtt tttctttgtc gtaatcgctg gttgcggttt cgatttgttg 2040720
gcggatttcg gcaacacgcg cttcgatttg ggctgcgtcg ccaaagccgt cgatgatggt 2040780
ggtgttttct ttaccgattt cgatgcgttt ggcttgaccc aagtcgtcca aagtcgcttt 2040840
ttccaaagac agaccgactt cttcggaaat caccacgccg ccggtcagga tggcgatgtc 2040900
ttgcaacatc gctttgcggc ggtcgccgaa gccaggggct ttgacggcaa cggttttcag 2040960
gatgcctcgg atgttgttca cgaccaaagt cgccaaggct tcgccttcta cgtcttcagc 2041020
gataatcaac agcggacggc tggcttttgc cacttgttcc aaaacaggca gcaggtcgcg 2041080
gatgttgctg atttttttgt cgaacaacaa tacaaacgga ttgtccaaag cagcgatttg 2041140
tttttccgca tcgttgatga agtaaggaga caggtagccg cggtcgaact gcataccttc 2041200
aactacgtcc agctcgtttt ccaaagactt gccgtcttca acggtaatca cgccttcttt 2041260
gccgactttt tccatcgctt cggcgataat cgcgccgact tgttcgtcgg agttggcgga 2041320
aatagagccg acttgggcga tttctttaga agtgtcgcaa ggtttggcga tgtttttcag 2041380
ttcgtcaacc aaagcggcga cggctttatc gataccgcgt ttcaggtcgg tcggattcat 2041440
acctgcggta acatatttca taccttcggc aacgatggat tgcgccagta cggtggcggt 2041500
agtcgtaccg tcgcctgcca cgtcgttggt tttggacgca acttctttca ccatttgcgc 2041560
gcccatattt tcaaacttgt ctttcagttc gatttctttg gcgacggtta cgccgtcttt 2041620
ggtgatgtgc gggccgccga atgcgcggtc aacgactacg ttgcgacctt tggggcccaa 2041680
ggttacgcgg acggcgtttg ccagaatgtt cacgccgttt accatttttt gacggacttc 2041740
attgccgaac tgtacgtctt ttgctgccat ttcaattctc caaaaatcat taaaactgtc 2041800
tgataaaacc gtttatgccg tctgaaggcg gtttgccgtt tcagacggca tcgtgtccgt 2041860
atttattttt caacgatgcc gaaaatatct tcttcgcgca ttaccaacag ctcttcgccg 2041920
tcggctttta cggtttggcc gctgtatttg ccgaagatga ttttgtcgcc gactttgaca 2041980
tccagcggac ggcggctgcc gtctttaccg attttgcccg cgcccacggc gatgacttcg 2042040
cccatatcgg gtttttcggc ggccgcaccc ggcaaaacga tgcccgatgc ggtttttttct 2042100
tcagcttcca agcgtttgac gacaacgcgg tcgtgtaaag gacggatggt catatttatg 2042160
ctccgataaa atagtttgaa aacaatcatc tgcccgaacg gttcaggcag attgaagtgg 2042220
aaaccggaca gccgtcaagc agctgcccgt ataagtcggc aaaattaggg tgtgtgcggg 2042280
taaattcaag tgaggcggaa aaaatttatt tccgccgttt tttatagtgg actaaattta 2042340
aggggctgta ctagattagc agatatgtta ccctcgaaat atgaagataa cgcactgcaa 2042400
attaaagaaa aaagtacaga aagaactgct ccgtttttttg tactggaagt taccgcccgt 2042460
tctgccgccg atattttggg tatccatccc aattcggcag cactgttcta ccgtaaaatc 2042520

```
cgcacggtta tcaaccatca tttagccttg gctgccgatg aggtttttga gggccctgtc 2042580
gagccggacg aaagcgattt cggcggacgg cgtaaaggta gacgtggtcg cggtgcagca 2042640
ggaaaagtgg ttgtcttcgg cattctgaaa cgcaacggac gggtctatac cgttgtggtg 2042700
gataatgcca agtctgaaac gttactccct gtcatcaaga agaaaatcat gccggacagt 2042760
attgtttata ccgatagtct gagcagctgc gacaagttgg acgtgagcgg tttcatttat 2042820
taccgcatca accattccaa ggaatttgca gaccgtcaga accacattaa cggcattgag 2042880
aatttttgga atcaggcaaa acgtgtcttg cgaaaataca atggaatcga tcgtaaatct 2042940
ttcccgctgt tcttgaaaga atgcgaattt cgatttaact tcggcacacc gtctcaacag 2043000
cttaaaatcc tgcgggattg gtgtgggatt tagggctaat ctagtacagc ccctaaaatt 2043060
tttcgttttc aagccttcac cgcttgccat cagcgttaaa ttttttttacg ataagcacat 2043120
agattgtaaa caatcggcca caagccggtt tgttttttca gaagacatta tccctgtcag 2043180
acgctatttc tatatatttc gcctataatg gcttgttttt aataaataat tcaagaggta 2043240
tcaacgtgtc tgattccaag acgaaagaac gcgccacatt cggcacgcgc cgcgcgttta 2043300
tgattgccgc catcgggtcc gccgtcggct tgggcaatat ttggcgtttc ccctatattg 2043360
cttttgaaaa cggcggcggc gcgttcatcc tgccctatct ggtcgcgctt ctgacggcgg 2043420
gcatcccgct gctgctgctc gattatgcca tcggccaccg ttaccgtggt tctgcgccct 2043480
tggctttccg ccgcctcgga cgatggtttg agccggtcgg ctggtggaac gtgatgacca 2043540
atatcgtcat ctgcatctat tacgcggtaa ttatcggttg ggcggcaagc tatacctatt 2043600
attcggtcaa cgccgcctgg ggtgcggatc cgcagggttt tttctttaag gacttcctgc 2043660
aaatggcggg cccggaagcc ttgggtttgg attttgtcgg caaagtcgcc ggtcctttgg 2043720
cgggcgtgtg ggtttttacc gccgccatta tggcgttggg cgtgcaaaag ggcgtggcgc 2043780
gcgcctcgtc gttctttatg ccgctgcttt tggtgatgtt tttgattatg gtcggcattt 2043840
cactaaccct gccgggtgcg gcaaagggct tggacgcatt gtttacgccc gactggtcga 2043900
aactcgccga ttccaaggtc tgggtggcgg catacgggca gattttcttt tcgctttcca 2043960
tctgcttcgg cattatggtt acctattctt cttatttgaa gaaaaaaacc gacttgggcg 2044020
gaacggggct ggtggtcggt tttgccaaca gcagctttga actgctcgcg ggcatcggcg 2044080
tgtttgccgc attgggcttt atggcgcagg cgggcggtaa ggcggtcaac gaggttgcct 2044140
caggcggcat cggtttggcg tttatcgcct ttccgaccat tatcaaccag gcaccgatgg 2044200
gctggctgat cggcatattg tttttcggtt cgctggtgtt cgccggcgtt acgtcgatga 2044260
tttccatcct tgaagtgatt gtggcggcga ttcaggacaa gctgaacatc gggcgcgtca 2044320
acgccacgct gctggtctgc attccgatgg gcattgtttc cacgctgctg ttcggtacgg 2044380
cgacggggct gccggttttg gacgtgatgg acaaattcgt caacacctac ggcattgttg 2044440
ccgccggctt tgtttatgtt gccgccatca tcatcagcgg caggctgccg gaattacgca 2044500
agcacctgaa cgctttgtcc tccatccgca tcggcggctt gtggacggtc tgcgtcgtgg 2044560
ttaccgtcgt gatgctcggc tatatgctgt ttaaagatac cagcggcctg atggagaaaa 2044620
attacgaagg ttatccggat ggtttcctca gtattttcgg ctgggggatg tcggcggcgt 2044680
tggtcgtgtt cgggctgctg ctgtcgttgc tgccttggaa acacggtcag gatttcaacg 2044740
tcaaagacga acacgaacat gaacaaggag aagaaaaatg agtacttccg ccattgtgat 2044800
gatgattgtc tcaatcgtga taatctgggg agggctgctg ctttccctgt taaggctgcc 2044860
gaacgagtaa gcctttagag cgttaaaaat gccgtctgaa ccgcttcaga cggcattttg 2044920
atgttcggct tgcaggcagg cgagttcgtt tgccatttgc tgttccaagg tttcgcgccg 2044980
gcggatgagt cggtattcgt tgccgtccac caacacctct gccgcacggt tgcgcgcgtt 2045040
gtaattgctc gccatactgg ccccgtatgc gcccgcgctg cggataagca gcaaatcccc 2045100
ttcttcgcag gcgatggtgc ggtctttgcc gaggaagtcg ccggtttcgc aaatcggacc 2045160
gacgatgttg gcggtcagcg tcgcgatgtc tttggtttcg accgcctcga tgtgatgata 2045220
ggcatcataa agcgccgggc gcatcaaatc gttcatcgcc gcatcgacca tcacaaagtt 2045280
tttctcttcg ccgtatttga caaactcgac gcgtgtcagc agcgaacctg cgttgccgac 2045340
caggctgcgg ccgggctcaa gaatgagttt cagacggcgt gtgccgatca gtttttgaac 2045400
ggcttgggca tacgcgccca aatcaggcac attttcgtct tggtaaacaa tgccgacgcc 2045460
gccgcctaag tctaaatgtt ccaaaacaat gccttcggcg gcaagcgcgt caaccaaaat 2045520
caaaatgcgc tcgcaggctt cgaccagcgg gcttaagtcg gtcagttgcg aaccgatgtg 2045580
gcagtcgatg ccgatgattt tcaaattggg ctgttgtgcg gcatagtggt aggcttcgag 2045640
cgcgtcggcg taggcgatgc cgaatttgtt ggctttcaga cctgtggaga tgtagggatg 2045700
ggtttttgca tcgacatcgg ggttgatgcg cagggagacg ggcgcggttt tacccaaacg 2045760
tgcggcaact ttctgaatac ggtcgatttc ggggatgctt tccatattga agcatttcac 2045820
gcctgcattc agcgcgaact cgatttccgc ctcgcttttg cctacgcctg aaaatatggt 2045880
ttttgccgcg tcgccgcctg ccgccaaaac gcgtgccaat tcgccgccgg acacaatgtc 2045940
aaaaccgctg cccagcgagg cgaagtgttt gataatgctc agattgccgt ttgccttgac 2046000
ggcgtaacag acgagcgggt tcaaagcggc aaacgcggtt tggtagtgtt caaatgcttc 2046060
ggtcagcgcg gattggctgt acacataaag cggtgtgccg aatgcttcag caaggcgggg 2046120
gtaggggact tgttcgcaaa atagggtcat gttttcgttt tcatttttgg gtttgtggag 2046180
```

cggattgcgg tttgctttga agttgcaaac cggtttggat tacgccgaaa cgcgccttgt 2046240
cgccttcttt gggcaggtag aggtcgcctt tgtaaccgca ggccgagagc aggagggcgg 2046300
ttgccgccgc aaaaaatacg ccgtatttca tcggtaaact tccttcataa gcgcgaatgt 2046360
ggcaagattc ggcatcttaa acaaaaaaca cgcaaaaagc tatgatgacc gaaagcgagt 2046420
ttatccgcgc gagcgaagca ttatttgaac acatcgaaga ccaaatcgac gaaaacggct 2046480
gggatttcga ctgccggttt gccggaaacg tcctgaccat cgaagccgga gacggcgccc 2046540
aaatcatcgt caaccgccac acgcccaatc aggaattgtg gattgccgca aaaagcggcg 2046600
gctaccattt cgccgagcaa aacggcaaat ggctggcaac gcgcgacgga cgcgattttt 2046660
atgacgtttt aaacgaagcc ctgagcgcgg cttcgggcga agcggtggaa attgcggaat 2046720
tgtgatttgg gtgttatcac ggaaagaaaa aatgaacaca cgtccctttt atttcggact 2046780
gatatttatc gcgattatcg ctatacttgc taactattta ggaaacactg atttttccca 2046840
tcattatcat atcagtgctt taattattgc tatcttgctg ggaatggcaa tcggcaatac 2046900
catttatccg caattttcga cacaagtgga aaaaggcgtt ttgtttgcca aaggcgcgct 2046960
tcttcgcact ggcattgtgt tgtatggttt tcgcctcact tttggcgata ttgccgatgt 2047020
aggattaaat gcggttgtca ctgatgcaat catgctaatt tcaaccttct tttttaccgc 2047080
acttttaggc attcgttatc taaaaatgga taaacaattg gtttatctca ctggggcagg 2047140
ttgcagcatt tgcggtgcgg cagcagtgat ggcggcagag cctgttacta aagcagaatc 2047200
ccataaagtt tcagtggcga ttgccgtagt ggtcattttc gggacgcttg ctattttttac 2047260
ttacccccttg ttctacacgt ggtcacaaca tttaattaac gcccatcaat tcggtattta 2047320
tgttggttct agtgtacacg aagtggctca agtgtatgcg attggggaaa atattgatcc 2047380
tatcgtggcg aatactgccg tcatttccaa aatgatccga gtgatgatgc tcgccccctt 2047440
tttattaatg ctttcttggt tattaacacg tagtaatgga gtatcagaaa atacatcaca 2047500
caaaattaca attccttggt ttgctgtact ttttattggt gttgccattt ttaattcttt 2047560
tgatttatta ccaaaagaac tcgtgaaatt attcgttgaa atcgattctt tcttattaat 2047620
ttcatcaatg gctgcgcttg gcttaacgac gcaagcaagc gcaatcaaaa aggcaggatt 2047680
aaaaccgttt gttttaggaa tactaactta tttatggcta gtggttggtg gatttttagt 2047740
gaactatgga atatcaaaat taatataaaa ttcactaaag agagcgttac ccaatggcac 2047800
aattaccgct atatctgact tctgaaatca aagactttac tgtcggcacg cctaaagttt 2047860
tagaatcatt ttccaaacat atcccttatg gtgtcgtctt tgaagacgac ggcgacacag 2047920
gctacttcta tgccgcttcg caagacggga ttttagatgc cttgcacatc tataatgtcg 2047980
aagatgtatc cgacaaacat atccccaatc atgtcttgat tttatgggat gatgcctgca 2048040
ccatagccgc attgtgtatc aacgactaca ttcatgccgt ctatgatttt gtcgaacagg 2048100
caggatattg ccgcaacggc ttccctgaag caggcggcga atgggtgaaa gtcgaaaacc 2048160
gcgtcttgga tgatgaattg ctggacaaaa tcctatcccg aaaatctaca taaccctcac 2048220
aaaaggatac ccaaatgccc ctactagaca gtttcaaagt cgatcacacc cgtatgcatg 2048280
cccccgccgt acgcgtggcg aaaaccatga ctacgcccaa aggcgacacc attaccgtgt 2048340
ttgacctgcg cttttgcgtt cccaacaaag aaatcctgcc tggaaaaggc atacacacgc 2048400
tggagcattt gttcgcaggt tttatgcgcg accacttgaa cggcaacggc gtggaaatca 2048460
tcgacatttc cccgatgggc tgccgcaccg gtttttatat gagtcttatc ggcacgcctt 2048520
ccgaacagca ggtcgccgat gcgtggctgg cttcgatgca ggatgttttg aatgtcaaag 2048580
accaaagcaa aatccccgag ttgaacgaat accaatgcgg cacttatcaa atgcactcgc 2048640
tcgccgaagc gcagcaaatc gcgcaaaacg tgttggcgcg caaagtggcg gtgaacaaaa 2048700
atgaagagct gacgctggat gaagggctgc tgaacgccta atccgccaaa aatgccgtct 2048760
gaacaagggt ttcagacggc atttgccttt tccgttataa tccggggttg tccgggggcg 2048820
ggttttaagc cggcatcgtc cttccctatt tttttctgtc ccttatcggt tttaagcggg 2048880
ttttttatgt ccaacagacc tacactcctc ctcgttgacg gatcgtccta cctctaccgt 2048940
gcgtatcacg cgatggggca aaacctgacc gcccccgacg gcgcgccgac gggtgcgctg 2049000
tatggtgtat tgaatatgtt gcgccgtttg cggtcggaat atccgcacga ttattgcgcg 2049060
gtggtttttg atgcgaaagg caaaaatttc cgccatcaaa tgtttgaaga atacaaggcg 2049120
acgcgcccgc cgatgcccga cgatttgcgc ccgcaggcgg aagcactgcc ggatttagtg 2049180
cgcctgacag gctggccggt attggtgatt gggcaggtgg aggcggacga tgtgatcggc 2049240
acgctggcga aacagggggc ggaacatggt ttgcgagtca ttgtttcgac cggcgataag 2049300
gacatggcgc agttggtgga tgagcgcgtt acgctggtga acacgatgag cagcgaaacg 2049360
ctggacattg aaggcgtgaa ggcaaaattc ggcgtgcgcc ccgaccaaat ccgcgattat 2049420
ctcgcgctga tgggcgacaa ggtggacaac gtgccgggcg tggaaaaatg cggcccgaaa 2049480
acggcggtga aatggctgga agcctacggt tcgctggctg gtgtgatgga acacgcttcg 2049540
gaaatcaagg gcaaagtggg cgaaaacctg caagccgcgc tgccccaact gccgctgtcg 2049600
tatgatttgg tcacgattaa aaccgatgtg gacttgcacg ccgagctttc agacggcatc 2049660
gaaagcctgc gccgtactac gccgaaatgg gcgcagctgg ttgtcgattt caaacgctgg 2049720
ggcttccgca cctggctgaa agaagcggaa tcaaacatga ataccggctc gaccgatgat 2049780
ttgttcggca gcgacagcat cggcgagcag gcggctttga atgcggaaat gccgtttgaa 2049840

aaacaagccg aaaaagccac cgcccccgaa aaactggatt atcaagccgt taccaccgaa 2049900
gcgcagtttg ccgctttgtt ggacaaactg tcgcgggcgg acacaatcgg catcgatacg 2049960
gaaaccacgt cattagacgc gatgaacgcc tcgctggtcg gcatcagcat cgctttccaa 2050020
gcaggcgaag cggtttacat ccccgtagga cacagcctga ccgccgcgcc tgaacagctt 2050080
gatttacaag acgtattagg ccgtctgaaa ccgcatttgg gaaaccccgc cctaaaaaaa 2050140
atcgggcaaa acctcaaata cgaccaacac gttttcgcca actacggcat cgccctgaac 2050200
ggcattgccg gcgacgccat gctcgcttcc tacatcatcg agagccatct cggacacggc 2050260
ttggacgaat tgtccgaacg ctggctcggc ttggaaacca ttacctacga atcgctgtgc 2050320
ggcaaaggcg cgaagcaaat cggttttgcc gatgtcgcca tcgggcaggc gaccgaatac 2050380
gccgcccaag acgccgattt cgccctgcgc ctcgaagcgc acctgcgcgc gcaaatggac 2050440
gaaaaacagc ttgaaatgta tgaaaaaatg gagctgcccg tcgcgcaggt attgtttgaa 2050500
atggaacgca acggcgtgca aatcgaccgc gccgaactcg cccgccaaag cgcggaactc 2050560
ggcgccgagc tgatgaagct cgaacaggaa gcctatgccg ccgcaggcca gccgttcaac 2050620
ctcaattcgc ccaaacagct gcaagaaatc ctgttcgaca aaatgggcat ccccaccaaa 2050680
ggcctgaaaa aaaccgccaa aggcggcatt tccaccaacg aagccgtgct cgaacagctc 2050740
gcgcccgact accccctgcc taaaatcatc ctgcaaaacc gcagcctggc gaagctcaaa 2050800
tccacctaca ccgacaaact acccgaaatg atttccccca aggacggccg cgtgcatacc 2050860
acctacgccc aagccgtcgc cattaccggc cgcctcgcca gcaacaaccc caacctgcaa 2050920
aatatcccca tccgtaccga agaagggcgt aaagtccgcc gcgcctttac cgcaccgcaa 2050980
ggcagcgtca tcgtttccgc cgactattcc caaatcgagc tgcgcattat ggcgcacctc 2051040
tccggcgaca aaaccctgat tgccgcgttc caaaacggcg aagacgtaca ccgccgcacc 2051100
gccgccgaag tgttcggcac tgcgcccgaa aacgtctcgt ccgagcaacg ccgctatgcc 2051160
aaaagcatca acttcggctt aatttacggt atggggcaat acggtttggc aaaatcattg 2051220
ggcatcgaca acctttccgc caaaaacttt atcgaccgct acttcgcccg ctaccccggc 2051280
gtcgccgaat acatgcagcg caccaaagaa caagccgccg cccaaggcta cgtcgaaacc 2051340
ctgttcggca gaaggctcta cctgcccgac atccgcaaca aaaacgccaa cgcccgcgcc 2051400
ggagccgaac gcgctgccat caacgccccc atgcagggca ccgcctccga cctcatcaaa 2051460
cgcgccatga tagacgtgtc ccgctggctt tcagagtgcg aagcctcccc gtgggacgaa 2051520
ctcttacaaa gcaaactgat tatgcaggtg catgacgaac tggtgctgga agtcgttgaa 2051580
accgaactgg attttgtcaa agaaaaactg ccgcagatta tggcgaaagt ggacggcgga 2051640
ttattggatg taccgctggt ggctgaggtt ggcgtagggg agaattggga agaggcacat 2051700
tgattgaaag gtgttatatg ctatctttat ttaaataaaa tttaattttt ggtatatttt 2051760
ttctaaatgt tcctatagta tagtggatta acaaaaatca ggacaaggcg acgaagccgc 2051820
agacagtaca aatagtacgg aaccgattca cttggtgctt cagcacctta gagaatcgtt 2051880
ctctttgagc taaggcgaga caacgctgta ctggtttttg ttaatccgct atattccgcc 2051940
atctctaaga tttacagcga tacacgggtg atttaaggaa tgcccgaacc gtcattcccg 2052000
caacttttcg tcattcccac gaaagtggga atctagaaat aaaaagcagc aggaatttat 2052060
cggaaataac tgaaaccgaa cagactagat tcccacctgc gtgggaatga caattcgaga 2052120
cctttgcaat aacataggtt actaaaattt tatgctcaat ctcattttca aaatgcaaaa 2052180
cttttctgat ttttcctact ttttgctcaa tattaggaag gttttaggca attgaaaatt 2052240
ttttggcgca tttttatgcg tcaaatttcg ttaacagact atttttgcaa aggtctcaat 2052300
tcataagttt cccgaaattc caacataacc gaaacctgac aataaccgta gcaactgaac 2052360
cgtcattccc gcgcaggcgg gaatctagac cttagaacaa cagcaatatt caaagattat 2052420
ctgaaagtcc gagattctag attcccgctt tcgcgggaat gacgaaaagc aagccgtagg 2052480
tcggatactt gtatccgaca aaagcctgcc atctcaaata gccgtcggat tcgagaatcc 2052540
gacctgccaa accgggcgcg gacgctccgg ccggcagtta gtacgcaaat cgaacagaac 2052600
atcacaaaaa agcccgattc ggattttcca atcgggcttt tttgcgcccg ttttgtcatc 2052660
ccgtgaaata tccgcatgac aaaaatatag tgaattaaca aaaatcagga caaggcgacg 2052720
aagccgcaga cagtacagat agtacggtaa ggcgaggcaa cgctgtactg gtttaaattt 2052780
aattcactat aatgcaaaat catgacaaaa ccggcgcgag gttacacaaa cggatgaaat 2052840
caaccgatat tcaaacacag tcatttttag cgcattttca gcgtatcgtt aatgcggaaa 2052900
atttcgtgaa caggtttttt gcacaggcct ccgcctcgtt tcgcgggatg ggaaaccgta 2052960
ttagaaaacg gacgcacgca acataaaccc cgaaagtgat gataagatga tgatttaacg 2053020
tactgcttta attatttaag gaattatcgt gtttcccgac aaatacaagt tgagtttgaa 2053080
agaaaatatt tttttggcaa agaaagtatt ggttgcccaa attcacaacc tcagtcgttt 2053140
tgagaattgt cagacgacct tgttgcagac cgaacaaatt atccatggca aaaatgtagc 2053200
ctccgcgtca ctggaagaca tccaaaccat cttgaacctg aaacgtgcct atcaatatgt 2053260
gatttcgcat atttcaaacg gcgaaccggt cgatatttca ctccttaaaa aaatcaacaa 2053320
cattgttgcc aaggacgatt ctttggcacc cggtgatttc cgtaccggtt cggtcggcgt 2053380
aacgctattg gacggttccc gtcatgcccc gaatccagtg aaggaaattg aagtggcccg 2053440
cgtgttacaa aatatcggac tgcaaagcgg ttcgacgacg gaggcagccg tccgttttat 2053500

gctttattgt atgcggcagc aggtttttttg ggacggcaac aaacgaacgg caaccttatt 2053560
tgccaacggt ctgatgatgg cggggggctg cggcatcttg gaaatctccg aaatgcagat 2053620
gccgcaattc aatgaaaaac tgtccgcatt ctatcgctcc ggcgacgata ccgatatttc 2053680
caagtttgtg tatcaaaatt gtatatcggg catagactga gacctttgca aaattcccca 2053740
aaacccctta aattcccacc aagacattta ggggattttc catgagcacc ttcttccagc 2053800
aaaccgccca agccatgatt gccagacaca tcgaccgttt cccgctattg aagttggacc 2053860
aggtgattga ttggcagccg atcgagcagt acctgaaccg tcaaaaaacc cgttacctta 2053920
gagaccaccg cggccgtccc gcctatcccc tgctgtccat gttcaaagcc gtcctgctcg 2053980
gacaatggca cagcctctcc gatcccgaac tcgaacacag cctcattacc cgcatcgatt 2054040
tcaacctgtt ttgccgtttt gacgaactga gcatccccga ttacagcacc ttatgccgct 2054100
accgcaaccg gctggcgcaa gacgacaccc tgtccgaact gttggaactg attaaccgcc 2054160
aactgaccga aaaaggctta aaagtagaga aagcatccgc cgccgtcgtt gacgccacca 2054220
ttattcagac cgtcggcagc aaacagcgcc aggctataga agtcgatgag gaaggacaaa 2054280
tcaacggcca aaccacaccg agtaaggaca gcgatgcccg ttggataaag aaaaacggcc 2054340
tctacaaact cggttacaaa caacataccc gtaccgatgc ggaaggctat atcgagaaac 2054400
tgcacattac cccgccaat gcccatgagt gcaaacacct gccgcctttg ttggaaggac 2054460
tgcccaaagg tcgaccgtct atgccgacaa aggctacgac agtgcggaaa accggcaaca 2054520
tctggaagaa catcagttgc aggacggcat tatgcgcaaa gcctgccgca accgtccgct 2054580
gacggaaacg caaaccaaac gcaaccggta tttgtcgaag acccgttata gtggattaaa 2054640
tttaaatcag gacaaggcga cgaagccgca gacagtacaa atagtacggc aaggcgaggc 2054700
aacgccgtac tggtttaaat ttaatccact atatgtggtc gaacagagct tcggtacgct 2054760
gcaccgtaaa ttccgctacg ctcgggcagc ctatttcgga ctgattaaag tgagtgcgca 2054820
aagccatctg aaggcgatgt gtttgaacct tttgaaagcg gccaacaggc taagtgcgcc 2054880
cgctgccgcc taaaaagcag cccggatgcc tgattatcgg gtgtccgtgg aggattaagg 2054940
gggtatttgg gtagaattag gaggtatttg gggcgaaaat agacgaaaac ctgtgtttgg 2055000
gtttcggctg tcgggaggga aaggaatttt gcaaaggtct cagactattt cggcacggac 2055060
gaagatatag atttccccga cccaccaaac atgggctaaa aatcaatttg acggttatca 2055120
gacaatggag caggcacaag gcggcggcag aaaaagggtt tgacagcgca cggtggcatc 2055180
gtcagacccc tttcggcata tccggcggtt accagcggta gcctaatttg atgcccgcgc 2055240
tgtgttgcgc ttccagttgc gggcctttgg cggcggcagc gtggagggac agcgtgaaac 2055300
ctttgatttc ggcgtttacg ccccattccg cactgcgggt tttgccgaaa tcctgagcca 2055360
atacggcggt attgacgcgt gttcggactt tgcccgaagc ggcatcggta taggacaggc 2055420
tcaaataagg cgtgatggaa atgtgttgcg ccggtttgaa tgaataatct gccttaatgc 2055480
ccgcgcggta gcggttgaat gcaaggccgg gggtggcgat attgacgttt tcgtagcggt 2055540
aatccgcttt ttggacgaaa tagcgcgttg cgccgatgtg cggttcgatg ccgaatccgc 2055600
cgaaaccggc gcggtatcgt gcctgaatgc cgtaatgcag cacgcggcgg cggattttgc 2055660
ctccgatgcc gtctgaaagg ctgccgctgc taaaacccgc gcccgcgctg atgccgatgt 2055720
agaacctgtc gatgccgtat tgcccgaaaa cggcgccgtg ggcaagccgt gccgagttgc 2055780
cgatgccgtc gtcgaaggtg ttttcggtcc ggttgtgcga aaacaggatg ccgacgcgcc 2055840
cgctgccgag gtttttctgc ataccgattt ggcgcaggtc ggtttgttgg cggtaggcgc 2055900
ggaaatcttg cgaacggtag tgtttggtgt cccggatgcc gcttgtccaa acggcgttgc 2055960
ggcggtcttc ggcaaatacg cggtctaatt cgtcctgtac ggcgaaaacg ctgttgagcg 2056020
tggcggaaaa ttcactcaaa ccgctattgg cataacggct gatcaggtcg cgctgcggtt 2056080
ggggctgcgg ttggggttgc agttgcggca aatcccggcg ggcgcggcgg gcgcggggga 2056140
aggcggtggt agccggccgg gtttccgctt cgcgctgttt cgccaaggcg gtgtctttat 2056200
ccgcctgcac ccgtttttttc tcttcctccg cctgcataat gccgacattt tccccgcctg 2056260
cctgccgggc cggttcggca acgctttctg tcttttcgac ggcatcgcgc ccggccgcaa 2056320
tcagcgcgtc aaggctttgc gcgttgtctt tttccgcctg ttttttggct tctgccttgc 2056380
cgagtttgtc ggaaagctct tgttctttga ccggattatg caggcggaac tcgccgtctt 2056440
tgcggatgag ttggtaacgc cacgcgccgg catcgacgtg ttcgttttgc agggtgaaat 2056500
taaggttttc ggacagcggt ttgttgtctt ttccttccac taccgtcaat tgttcgaggc 2056560
ttgcaggttc gttgccggta ttgttgaccg ccaaggtgta agtgccttcg gaactttccg 2056620
ccagcttcaa tttgtcgctg cggtagccga agagttccga cataaagcgg aatgttccct 2056680
gaccgttcaa tttgccgttt accgtcagcg tgttgaaacg ggattctacc gaagttggcg 2056740
gtgtaacgga taatagggaa cggcgcgaac ggcgcgaacg gcggcgcggc gcatctgtcg 2056800
cactgccggt ttgcgcccct gccgcatcgt ggcgataggc ggaattgagt gtaatggtgg 2056860
cgttgtcaag gtttaaattg cctaattccg tgcctgacgg cagcgtccat tcgctgtctt 2056920
ttaagtgtaa tgccgtatcc ttgccgccgc tgatttgtcc ggtaaagcgg ctgctttcaa 2056980
aatggaatac tgccttatcg gctagggaga cattaccgtt gagtgcggaa tggcttacgt 2057040
ttgccttagc gttgccggaa agcgtcagac tgccgttttg tacggcgtgg tcgcttagat 2057100
taaatgaagc attgcccgaa gccgatgtgt tgccgtttaa tgtggcttga ttaaatgttg 2057160

cttgggcatt gcccacgagg ctaaggttgc cgttttgggt ggcgttgtgg ctgactgtat 2057220
aacgtgtatc gccatttgca ctaagattgc cgttgagtgt ggcaagccct gtgagattta 2057280
aatgagcgtg atcggcaaga tcgacattgc cgctgatgtc ggtcttagtc aatgaagcaa 2057340
tcactttatc gtcggtaatg gtttttcga cacaatttgt cagacccgtc cagtccgaac 2057400
gtgtacagat tgtgtggctt tgatgcggtg cgacaccaaa aactgcttgg gcgtgattgc 2057460
tcaaatgcca atcgcctttc actttggcaa cattgcggga aaccaccgcc tgtccgcctt 2057520
taatttggaa gttttccgct ttaaatgtgc ggttgatcca gtcgttgtcc cacacgattt 2057580
ccccgcgagg aatgccctct ttttgcgacc aatggtcgtt taaatgattg taggcgtgcg 2057640
gtgttggtct gccgctgaaa aacagtttgc cgtttgtttg cgtgatgttg ccgtttaaat 2057700
ttgttccgcc ggaaagcagc agggtgcggt cttctgcggc gggctggtaa acaaggttga 2057760
gccgcccgtt cgttttggtc gtatctttct cgccaaacca accgttgtag gcaatttctt 2057820
ttttgctatc caagctgttg ttattgccgg ttgtagcaat atctttattg cctgtaatgg 2057880
taacggtgga ttctttgtct tgattgtggt tgacaatcat cgccccttca tcggtatttt 2057940
gaatacggtg gaacgaaagc gaatgcccgt ttaaatccaa acgtccgccg cgaaagccga 2058000
aatagagttt gtcggggttg aactgattat cggcattcag ttgcaccgta cccctgccgc 2058060
tgaccaagcc gatttcacta aaggcttgtt ttttgccttt atcgtctgcc tgctgatcca 2058120
aaatgactgt accgtcgccc acgctgatcg agccttggtt ttccccttttg gcttgaacgt 2058180
gcagcgtgcc tttgccgatt ttggacaggc ggtcgtttgc cacgccgttt actttccaag 2058240
taacggtact gtcttcactg atatgaacgc ccgcgccttg ccaagtttcg ttattttcag 2058300
gcgagaccgt aaaatctcct tggaaatata atcctccagc accttgattg atgttgctgg 2058360
taagtatcaa ttcgcctttt ccttcgtcaa taaaggaaat attttctcca ttattcagtc 2058420
tgggtcgata actgttgaca ccacctgcag catgataaac aggttctctt gctgtctcgg 2058480
ataaagaaac attaaacaat tgaacggttc gtgtttttaa tctattaggc agagaattgt 2058540
gttcatgttt ggcattgatt tttcctgtgc cattattatc gtcgttaaaa gagtatttcc 2058600
cattttgacg tggttcgtag aatactgaat gggtatctcc agcaaagatt tcatcataga 2058660
accaatcttt acgaaccagc tggaagccat tgctttttcc tatatagggg ttgcccgttt 2058720
gcaatacccc attaattaac cacttttgct tttgggcatc atagataaac attggtgagc 2058780
cactgtcgcc aaatgagcct cctgttggta aaaaaccata tgggctatgt ttaatttttt 2058840
cactacctaa gttgactgtg ccaccacctg atccattttg tgcaaaggta ttgccaccaa 2058900
cgagccaaga atacgcactt gcaatatgat atgaactttc gcggttattg ggctcatctt 2058960
catcagatcg ccaatattgc ctgcctgccc caatacgaac acggtcaggg taattatttt 2059020
gatcgatata tttccgccca tccatataac tggtcatttc aacaggttct gcatctgtga 2059080
caaatttatg caaacgcggc atatgataat cgccgccata aggatggcct ttagtccctg 2059140
ctttataatt attccgtttc acaattttat aagtaaaacg atgttgatcg ggatttcttc 2059200
cttccgcacc aaaatcaacg ttgttatagc cgccgttatg tgccacgctc acaatatatt 2059260
gatcgcccac caatgccgcc acgccgttac gcgacaccac agaaaaatca atcatcgggg 2059320
cttttgtcat tgatttgccg accaactccc ctttttttgtt gtaaacctca atatctttcg 2059380
ccccgactgc aaacttgcct ttattttcgg caaagtcgcg atagtattgg tagttgatgc 2059440
cgaaataagt gtgtcccgcc caggcttggg gaagaatgcc gaacgacagg catatggcta 2059500
agtaagcagg cgagaagcgg atgcggccgg ttttcggggc tttgcggtgt gtttcggttg 2059560
tccgtttgtc ggttgttttc attatttttc cttatctgac gggattcggg tttgtttggg 2059620
agggcgcggc ttccgcttcc gggcggcgcg cgggatgtgc ctatatgtgc ggttcggcgt 2059680
tcgggcggat atgaagcacg ccctaggatt tgtcattaat ttttgccttg gtctcggctt 2059740
cttccaatca cgaaagcacc cgccaaggca aacactgtgc cgccggcaag ggaggcggcg 2059800
gttgcggggt agccgctcca tacgaggaag acggcaaaaa gcagtatcag gatggcgctg 2059860
atgaagccgt acagttgccc gcgcctgttg aaggtttggt cttgccgtat ggtttcgtgc 2059920
cggacggctt gttctttttc cgccattgcc ataatgcggt ctgccccgtt gctgataatg 2059980
tcgttgtatt gcgccaagtc ggacggcggc ggcaacggtc ccgaatggaa acaccgggct 2060040
atcattattt gcacgtactc gtcggacagg atttgctcga caagctccgg ggatttgacg 2060100
acggtttcga cagcctgccg cgccttgtcc tgtgcgtttt cggtcatttt cgcgctttct 2060160
ctatggcgcg ttgaaaatcg ccgccgatgt ttttgagatc gtcggcggga ttggggcgga 2060220
tggcggtttt tgcgggatgg aacagaccca gcagcgagcc tataccgagc aggagggcgt 2060280
atgtgtttcg ttttttcata tggttatata ttaggtcagg cggacggatt tatcaagcat 2060340
ttttgcggtt ttataccgtc tgaaagccaa accgtcggac ttcagacggc atttgctata 2060400
atcgcggctg ttttgaattt tcgggggttt tatgtcggat aacgttccaa cgattgcggc 2060460
agtcgctacc gcaccagggc gcggcggcgt gggcgtgata cgcatatcgg ggaaaaacct 2060520
gctgccgatg gcgcaggctt tgtgcgggaa aacgcccaag ccgcgtaccg caacctatgc 2060580
tgattttacg gacacggacg gacaggcaat cgacagcggg cttttgctgt tttttgccgc 2060640
accggcaagt tttacgggtg aagatgtcat cgagcttcag ggacacggcg ggccggtggt 2060700
gatggatatg ctgctgaacc gctgtttgga attgggcgcg cgccttgccg aaccgggcga 2060760
gtttacgaag cgtgcgtttt tgaacgacaa actggacttg gcacaggcgg aaggcgtggc 2060820

ggatttgatt gacgcatcca gccgttcggc ggcgcgtctg gctttgcgct cgctcaaggg 2060880
cgatttttcg cggcggatac acggtctggt cgaagacttg attaccttgc ggatgctggt 2060940
cgaagcgacg ttagattttc ccgaggaaga cattgatttt ctcgaagcgg cagacgcacg 2061000
cggcaaactg gacggcttgc gccgcgccgt ggatgatgtg cttgccaacg cgcagcaggg 2061060
cgcgattttg cgcgaaggtc tgaatgtcgt attggtcggc gcgccgaatg tgggcaagtc 2061120
cagcctgctg aacgcgttgg cgggcgacga agtggcgatt gttaccgata ttgccggaac 2061180
gacgcgcgac gcggtcaggg aacgtatcct gattgacggc gtgccggtgc atattgtcga 2061240
tacggcaggt ttgcgcgaga cggacgacgt ggtcgagcgt atcggcatcg aacgcagccg 2061300
caaagccgta tccgaagccg atgtcgcgct ggtgttggtc gatccgcgcg agggtttgaa 2061360
tgaaaagaca cgggcgattt tggacgcgtt gccgccggag ttgaaacgca tcgaaatcca 2061420
cagcaaatcc gatttgcacg cacacgcggc aggcgggttc ggtacgggcg cggaaaccgt 2061480
catcgcgttg tcggcgaaaa ccggcgacgg cttggacgcg ctgaaacgga cgttgttgcg 2061540
cgaggccggt tggcagggcg aaagcgaagg gttgtttttg gcgcggacgc ggcacgtcaa 2061600
cgcactcaaa gcagcgcagg aagaattgtc gctggcggca ttgtgcggca accatcaaat 2061660
cgagctgttt gccgaacact tgcgcttggc gcaggtcgca tgcggcgaaa tcacgggcga 2061720
gtttacggcg gacgacctgc tcggcgtgat tttttcgagg ttttgtatcg gaaaataaac 2061780
ggatcgaaag catcgtggtg gtgtccggct gaacattccg ttatcccata aaaacgggaa 2061840
tccgatccgt ttggttttat agtggattaa caaaaatcag gacaaggcga cgaagccgca 2061900
gacagtacaa atagtacgga accgattcac ttggtgcttg agcaccttag agaatcgttc 2061960
tctttgagct aaggcgaggc aacgccgtac tggtttttgt taatccacta tagttttttt 2062020
gaatttcggg caacgcttga atcttcattc cgcgcaggcg gaaattatcg gtgcggtacg 2062080
gcaacttttt tcgatatgaa aagaccgtca ttcctgtaaa aacaaaaaat caaaaacaga 2062140
aaattgaaat tcgtcattcc cgcgcaggcg ggaatccagg acgtaaaatc tatagtggat 2062200
taacaaaaac cagtacagcg ttgccttgac ttagctcgaa gagaacgatt ctctaaggtg 2062260
ctgaagcacc gagtgagtcg gttccgtact atccgtactg tctgcggctt cgtcgccttg 2062320
tcctgatttt tgttaatcca ctataaagaa accgtttttc tcgataagtt tccgtgccga 2062380
cagacctgga ttcccacttt cgtgggaatg acggtggaaa agttgccgtg atttcggata 2062440
aattttcgta acgcataatt tccgttttac ccgataaatg cccgcaatct caaatcccgt 2062500
cattccccaa aaacaaaaaa tcaaaaacag aaatatcgtc attcccgcgc aggcgggaat 2062560
ctagacctta gaacaacagc aatattcaaa gattatctga aagtccggga ttctagattc 2062620
ccactttcgt gggaatgacg aattttaggt ttctgttttt ggttttctgt ccttgcggga 2062680
atgatgaaat tttaagtttt aggaatttat cggaaaaaac agaaaccgct ccgccgtcat 2062740
tcccgcacag gcttcgtcat tcccgcgcag gcttcgtcat tcccgcattt gttaatccac 2062800
tatattcccg ccgttttttt acatttccga caaaacctgt caacaaaaaa caacacttcg 2062860
caaataaaaa cgataatcag ctttgcaaaa atcccccccc ccctgttaat ataaataaaa 2062920
ataattatta attatttttc ttatcctgcc aaatcttaac ggtttggatt tacttccctt 2062980
catactcaag aggacgattg aatgaatacc ccattgttcc gtctcagcct gctctcgctt 2063040
accctggcgg caggttttgc ccatgcggca gaaaataatg ccaaggtcgt actggatacc 2063100
gttaccgtaa aaggcgaccg ccaaggcagc aaaatccgta ccaacatcgt tacgctgcaa 2063160
caaaaagacg aaagcaccgc aaccgatatg cgcgaactct taaaagaaga gccctccatc 2063220
gatttcggcg gcggcaacgg cacgtcccaa ttcctgacgc tgcgcggcat gggtcaaaac 2063280
tctgtcgaca tcaaggtgga caacgcctat tccgacagcc aaatccttta ccaccaaggc 2063340
agatttattg tcgatcccgc tttggttaaa gtcgtttccg tacaaaaagg cgcgggttcc 2063400
gcctctgccg gtatcggcgc gaccaacggc gcgatcatca ccaaaaccgt cgatgcccaa 2063460
gacctgctca aaggcttgga taaaaactgg ggcgtgcgcc tcaacagcgg ctttgccagc 2063520
aacgaaggcg taagctacgg cgcaagcgta ttcgggaaag agggcaactt cgacggcttg 2063580
ttctcttaca accgcaacaa tgaaaaagat tacgaagcag gtaaaggctt ccgtaataat 2063640
ttcaacggcg gcaaaaccgt accgtacagc gcgctggaca aacgcagcta cctcgccaaa 2063700
atcggaacaa gcttcggcga cggcgaccac cgcatcgtat tgagccatat gaaagaccag 2063760
caccggggca tccgtaccgt ccgtgaagaa tttaccgtcg gcggcgataa agagcgaata 2063820
agtatggaac gccaagcccc tgcttaccgc gaaaccacac aatccaacac caatttggcg 2063880
tacacgggta aaaacctggg ctttgtcgaa aaactggatg ccaacgccta tgtgttggaa 2063940
aaagaacgct attccgccga tgacagcggc accggttacg caggcaatgt aaaaggcccc 2064000
aaccataccc aaatcaccac tcggggtatg aacttcaact tcgacagccg ccttgccgaa 2064060
caaaccctgc tgaaatacgg tatcaactac cgccatcagg aaatcaaacc gcaagcgttt 2064120
ttgaattcac aatttaaaat tgaagataaa gaaaaagcaa ctgatgaaga gaaaaataag 2064180
aaccgtgaaa atgaaaaaat tgccaaagcc taccgtctga ccaacccgac caaaaccgat 2064240
accggcgcgt atatcgaagc cattcacgag attgacggct ttaccctgac cggcgggctg 2064300
cgttacgacc gcttcaaggt gaaaacccac gacggcaaaa ccgtttcaag caacaacctt 2064360
aacccgagtt tcggcgtgat ttggcagccg cacgaacact ggagcttcag cgcgagccac 2064420
aactacgcca gccgcagccc gcgcctgtat gacgcgctgc aaacccacgg caaacgcggc 2064480

atcatctcga ttgccgacgg cacgaaagcc gaacgcgcgc gcaataccga aatcggcttc 2064540
aactacaacg acggcacgtt tgccgcaaac ggcagctact tctggcagac catcaaagac 2064600
gcgcttgcca atccgcaaaa ccgccacgac tctgtcgccg tccgtgaagc cgtcaatgcc 2064660
ggttacatca aaaaccacgg ttacgaattg ggcgcgtcct accgcaccgg cggcctgact 2064720
gccaaagtcg gcgtaagcca cagcaaaccg cgcttttacg atacgcacaa agacaagctg 2064780
ttgagcgcga atcctgaatt tggcgcacaa gtcggccgca cttggacggc ttcccttgcc 2064840
taccgcttcc aaaacccgaa tctggaaatc ggctggcgcg gccgttatgt tcaaaaagcc 2064900
gtgggttcga tattggtggc aggtcaaaaa gaccgcaacg gcaaattgga aaacgttgta 2064960
cgcaaaggtt tcggtgtgaa cgatgtcttc gccaactgga aaccgctggg caaagacacg 2065020
ctcaatgtta atctttcggt taacaacgtg ttcaacacgt tctactatcc gcacagccaa 2065080
cgatggacca ataccctgcc gggcgtggga cgtgatgtac gcttgggcgt gaactacaag 2065140
ttctaaaacg cacatcccga aaaaatgccg tctgaaagcc tttcagacgg catctgttct 2065200
gataatttga tatatagtgg attaacaaaa accagtacgg cgttgcctcg ccttagctca 2065260
aagagaacga ttctctaagg tgctgaagca ccaagtgaat cggttccgta ctatttgtac 2065320
tgtctgcggc ttcgtcgcct tgtcctgatt tttgttaatc cactataaag accgtcgggc 2065380
atctgcagcc gtcattcccg cgcaggcggg aatctagacc ttagaacaac agcaatattc 2065440
aaagattatc tgaaagtccg agattctaga ttcccgcttt cgcgggaatg acgaaaggtt 2065500
gcgggaatga cgaaaagtgg tgggaatgac gaaaagtgat gggaatgacg aaaagtgatg 2065560
ggaatgacgg ttcgggcatt ccttaaatta cccgtgtatc gctgtaaatc ttagagatgg 2065620
cggaatatag cggattaaca aaaaccagta cggcgttgcc tcgccttagc tcaaagagaa 2065680
cgattctcta aggtgctgaa gcaccaagtg aatcagttcc gtactatttg tactgtctgc 2065740
ggcttcgtcg ccttgtcctg atttttgtta atccactata gattatcatt tatcctttct 2065800
aaagccgttc cggtttgtcc gaccggcggc tttgccccaa tatccccatt ttggagacac 2065860
ctatgttacg tttgactgct ttagccgtat gcaccgccct cgctttgggc gcgtgttcgc 2065920
cgcaaaattc cgactctgcc ccacaagcca aagaacaggc ggtttccgcc gcacaaaccg 2065980
aaggcgcgtc cgttaccgtc aaaaccgcgc gcggcgacgt tcaaataccg caaaaccccg 2066040
aacgcatcgc cgtttacgat ttgggtatgc tcgacacctt gagcaaactg ggcgtgaaaa 2066100
ccggtttgtc cgtcgataaa aaccgcctgc cgtatttaga ggaatatttc aaaacgacaa 2066160
aacctgccgg cactttgttc gagccggatt acgaaacgct caacgcttac aaaccgcagc 2066220
tcatcatcat cggcagccgc gccgccaagg cgtttgacaa attgaacgaa atcgcgccga 2066280
ccatcgaaat gaccgccgat accgccaacc tcaaagaaag tgccaaagag cgcatcgacg 2066340
cgctggcgca aatcttcggc aaacaggcgg aagccgacaa gctgaaggcg gaaatcgacg 2066400
cgtcttttga agccgcgaaa actgccgcac aaggtaaggg caaaggtttg gtgattttgg 2066460
tcaacggcgg caagatgtcg gctttcggcc cgtcttcacg cttgggcggc tggctgcaca 2066520
aagacatcgg cgttcccgct gtcgatgaat caattaaaga aggcagccac ggtcagccta 2066580
tcagctttga atacctgaaa gagaaaaatc ccgactggct gtttgtcctt gaccgaagcg 2066640
cggccatcgg cgaagagggt caggcggcga aagacgtgtt ggataatccg ctggttgccg 2066700
aaacaaccgc ttggaaaaaa ggacaggtcg tgtacctcgt tcctgaaact tatttggcag 2066760
ccggtggcgc gcaagagctg ctgaatgcaa gcaaacaggt tgccgacgct tttaacgcgg 2066820
caaaataatg aaacggcggc attcgatgcc gtctgaaaca cggatgcaaa ccgcctcctg 2066880
tgtttcagac ggcattgccc gatacggagg cttcaaacaa ggctttccgc tccgacggtt 2066940
cggactgcct tgtttgaatc ttctacgcct taacgctttt cccttctgtt tatgactgcc 2067000
aaaccttttt ccctcaacct gaccaacctg ctgctgctgg cggtgttgtt tgccgtcagc 2067060
ctgtcggtgg gcgttgccga tttccgctgg tctgatgtgt tttcactgtc cgacagccag 2067120
caggtcatgt tcatcagccg cctgccgcgc acgtttgcga ttgtgctgac gggcgcgtcg 2067180
atggcggtgg ccggcatgat tatgcagatt ttgatgcgca accgttttgt cgaaccgtcg 2067240
atggtgggcg caagccaaag cgcggcttta ggtttgctgc tgatgaccct gctgctgccg 2067300
gccgcgccgc tgccggcgaa aatgtcggtt gccgccgttg ccgcgctgat cgggatgttg 2067360
gtctttatgc tgctgatccg ccgcctgccg ccgaccgcgc aactgatggt gcctttggtc 2067420
gggattattt tcggcggtgt gattgaggcg gtagccacct ttatcgcgta tgaaaacgaa 2067480
atgctgcaaa tgctcggcgt gtggcagcag ggcgattttt cgagcgtgct gctggggcgg 2067540
tacgagctgc tttggattac gggcggtttg gcggtgtttg cctatctgat tgccgaccgg 2067600
ctgacgattt tggggctggg cgaaacggta agcgtgaatt tgggtttgaa ccggacggcg 2067660
gtgttgtggt cgggtttgat tattgtggct ttgattacgt cgctggttat cgttacggtc 2067720
ggcaatattc cgtttatcgg gctggtcgtg ccgaacatca tcagccgcct gatgggcgac 2067780
aggttgcgcc aaagcctgcc tgcggtggcc ttgctgggcg catctttggt gttgctgtgc 2067840
gacattatcg gacgcgtgat tgtgtttccg tttgaaattc cggtctctac ggtttttggt 2067900
gtattgggta cggctttgtt tttgtggctt ttgttgagga aacccgccta tgccgtctga 2067960
aaaaaatatc ggttttatgg caggaagcag ccgcccgttg tgggtcgcct ttgcgctgtt 2068020
gctggtttcc tgcgtcctgt ttatgacgct caacgtcaaa ggcgattggg attttgtttt 2068080
gcaactgcgg ctgaccaaac ttgccgcgct gctgatggtc gcctatgcgg tcggcgtgtc 2068140

cacgcaactc ttccaaacgc tgaccaataa tccgattctg acccccttcaa ttttgggttt 2068200
cgattcgctg tatgtgtttt tgcagacctt gctggtgttt acgttcggcg gcgtgggcta 2068260
tgcttccctg ccgttgacgg gcaaattcgg ctttgaactg gtcgtcatga tgggcggctc 2068320
gctgctgctg ttctacacgc tcatcaaaca gggcggacgc gatttgtcgc gcatgatttt 2068380
aatcggcgtg attttcggga ttttgttccg cagcctgtcg tcgctgcttt cgcgcatgat 2068440
cgatcccgaa gaatttaccg ccgcgcaggc gaatatgttt gccggattca ataccgtcca 2068500
cagcgagctt ttgggcatag gcgcgctgat tctgctcgtc agcgcggcgg tcgtttggcg 2068560
cgaacgctac cgcttggacg tttacctttt ggggcgtgac caagccgtca atttgggcat 2068620
cagctacacg cgcaacacct tatggatact gctttggatt gccgcattgg tggcgacggc 2068680
gaccgccgtg gtcggccccg taagcttttt cgggcttctc gccgcctcgc ttgccaacca 2068740
cttttccccg tcggtcaaac attccgtccg cctgccgatg acggtttgta tcggcggcat 2068800
cctcttggtc ggcggacaga ccgtgttcga acacctgctc ggtatgcagg cagtgttgag 2068860
cgtagtagta gaatttgccg gcggactcgt tttcctctat ctcgttttaa aacacaaaaa 2068920
atgacggatg ccgtctgaac ggccgccgcc ccgaaaggac aaaccatatg acacaagaac 2068980
atttcccatc attcttcaac caagccccga ccattaccgt ccaagacgca ttggccgaat 2069040
tcctcggcgc ggccgaaaac ggcatcctca cttaccgcta cgccgatgcc gtgcgcctgt 2069100
gcggacattc ctgcccgacc gtcgcgggcg cgtacctgat ggttatcaaa ggtctgaaag 2069160
cactttacgg cgaagagctg cccgaacgcg gcggcatcga agcctttatg cagggcgcgc 2069220
gcgacgaagg cacggtcggc gtaaccgcgt ccgtcgtcca actcctcacc ggcgcagccc 2069280
ccgaaaccgg ctttggcggc atcggaatgc agggacgctt tgcccgccgc cacctcttat 2069340
cctttggtgt aggcgaaatc aacggcacac tgaccctgcg ccgcaaagac aacggcaaaa 2069400
ccgtcgccgt cggcctcaac gccgccctgc aacccttcgc acccgaaatg cgcgacatca 2069460
tgcccaaagc cgtcagcggc agcgcaagcg cagaagaact cgaacgcttc ggacaactct 2069520
ggcaggcacg cgttaaagca tttttaaccg aatcggcgga cgacccgcag ttcgtcatcg 2069580
tccgcgaagt gtgagcgttc agacggcatt ccgaatttca aatgccgtct gaaccccgcc 2069640
aaacaacaac aaacctacgc ccgacaagca tccgccatga ttaccatccg caacgtcagc 2069700
taccgcatcg gcacacgccc catcctcgac aacgtcagcc tcgacatccc cgaaggcggc 2069760
attaccgccc tcgtcggccc caacggtgcg ggcaaatcca ccctgttttc ctttatggcg 2069820
cggctgcgac cgcttgaaag cggcagcatc gcctaccgag gcaaaaatct tgccgatacc 2069880
cccaccgccg aactcgccaa aaccctgtcc atcctcaccc aagaaaacag catcatgagc 2069940
cgcatcaccg tgcgcgacct gctgatgttc ggccgttacc cctaccatca aggcagaccg 2070000
actgccgaat gccgccgtat cgttaacggt gcaatcgaag aattccacct gcaagacctc 2070060
tccgaccgct acctgaccga gctttccggc ggccaacgcc aacgcgccat gattgcgatg 2070120
gtgttctgcc aaagcaccga ctacgtcctt ttggacgaac cgctgaacaa cctcgatatg 2070180
tatcacgccc gctcgctcat gcaaatcctg cgccggctga ccgacgaaca caagcgcacc 2070240
accgtcgtcg tattgcacga catcaaccag gcagcagcct acgccgacca cgtcgtcgcc 2070300
atgaaaaacg gccaagtcgc catgcagggc aaacccaacg atattttcac cgccgcaaac 2070360
atcaaaaccc tattcgatat ggacgtcgac gtcctcgatt acgaaggcaa aaaattggtt 2070420
atccaccata tctaaatccg acaaaaaggc cgtctgaaca ttcagacggc aacccatatc 2070480
ctgacaaaat taagacacga caccggcaga attgacatca gcataatatg cacatattaa 2070540
cagatattaa tgccgaacta cctaactgca agaattaaat aaataaataa ataaataaat 2070600
aaataaataa attgcgacaa tgtattgtat atatgcctcc tttcatatat actttaatat 2070660
gtaaacaaac ttggtgggga taaaatactt acaaaagatt tccgccccat tttttatcca 2070720
ctcacaaagg taatgagcat gaaacacttt ccatccaaag tactgaccac agccatcctt 2070780
gccactttct gtagcggcgc actggcagcc acaagcgacg acgatgttaa aaaagctgcc 2070840
actgtggcca ttgttgctgc ctacaacaat ggccaagaaa tcaacggttt caaagctgga 2070900
gagaccatct acgacattgg tgaagacggc acaattaccc aaaaagacgc aactgcagcc 2070960
gatgttgaag ccgacgactt taaaggtctg ggtctgaaaa aagtcgtgac taacctgacc 2071020
aaaaccgtca atgaaaacaa acaaaacgtc gatgccaaag taaaagctgc agaatctgaa 2071080
atagaaaagt taacaaccaa gttagcagac actgatgccg ctttagcaga tactgatgcc 2071140
gctctggatg aaaccaccaa cgccttgaat aaattgggag aaaatataac gacatttgct 2071200
gaagagacta agacaaatat cgtaaaaatt gatgaaaaat tagaagccgt ggctgatacc 2071260
gtcgacaagc atgccgaagc attcaacgat atcgccgatt cattggatga aaccaacact 2071320
aaggcagacg aagccgtcaa aaccgccaat gaagccaaac agacggccga agaaaccaaa 2071380
caaaacgtcg atgccaaagt aaaagctgca gaaactgcag caggcaaagc cgaagctgcc 2071440
gctggcacag ctaatactgc agccgacaag gccgaagctg tcgctgcaaa agttaccgac 2071500
atcaaagctg atatcgctac gaacaaagct gatattgcta aaaactcagc acgcatcgac 2071560
agcttggaca aaaacgtagc taatctgcgc aaagaaaccc gccaaggcct tgcagaacaa 2071620
gccgcgctct ccggcctgtt ccaaccttac aacgtgggtc ggttcaatgt aacggctgca 2071680
gtcggcggct acaaatccga atcggcagtc gccatcggta ccggcttccg ctttaccgaa 2071740
aactttgccg ccaaagcagg cgtggcagtc ggcacttcgt ccggttcttc cgcagcctac 2071800

catgtcggcg tcaattacga gtggtaagca gcatctcccg ataaagaaac cgcagccctg 2071860
caaggctgcg gtttttattt tctatccggc cgtcagactg ccgcgtccga acgttcgccc 2071920
gtgcggatac ggattgcctc ctcaaccggc agcacaaaaa tcttgccgtc gccgattttt 2071980
cccgaacgcg ccacctcgaa atcacgtcaa tcgcgcgttc cacagcatca tccgccaaca 2072040
ccagctcgat tttgattttg ggcaggaaat cgacggcgta ttccgcgccg cgatagattt 2072100
ccgtatgccc cttctgcctg ccgaaccctt tgacctcgct gacggtcatg cccgtaatgc 2072160
cgatttccgt caacgcctcg cgcacgtcgt cgagtttgaa cggtttgaca atcgcctcga 2072220
ttttttttcat aaaatttcct ttgaacaaac aatacaaaca catccgaaaa acgggaacct 2072280
cccgtcagat tgtcaacatt ttaagccaaa atacccaagc aatacagccc cgttgcgcgt 2072340
ataatgacag attttccaac cgcatttgag agccgaatcc atgtctgtcg ttttgccctt 2072400
gcgcggcgtt accgcccttt ccgatttccg tgttgaaaaa ctcttgcaaa aagccgccgc 2072460
actcggtctg cccgaagtca aattaagcag cgaattttgg tatttcgtcg gcagcgagaa 2072520
agcacttgat gccgcgactg tcgaaaaact gcaagccttg ttggcggcgc aaagcgttga 2072580
acaaacgcca aaagcgcgcg agggcttgca tttgtttttg gtcacgcccc gtttgggtac 2072640
gatttcgccg tgggcttcca aggcgaccaa tatcgcggaa aactgcggtt tggcaggcat 2072700
cgaacgcatc gagcgcggta tggcggtgtg gctggaaggt cgtctgaacg atgaacagaa 2072760
acagcaatgg gcggctttgc tgcacgaccg catgaccgaa agcgtgctgc ccgattttca 2072820
gacggcctcc aaattattcc accatctcga atccgaaact ttctccggcg tcgatgtttt 2072880
gggcggcggt aaagaagctt tggtcaaagc caataccgaa atgggcttgg cactttccgc 2072940
cgacgaaatc gattatctgg tcgaaaacta tcaggctttg cagcgcaatc cgtccgatgt 2073000
tgaattgatg atgttcgcgc aggcaaacag cgaacactgc cgccacaaaa tcttcaacgc 2073060
cgatttcatc ctcaacggcg aaaagcagcc caaatccctc ttcggtatga tacgcgacac 2073120
acacaacgcg catcccgaag gcacggtcgt tgcctataaa gacaattcgt ccgtaatcga 2073180
aggcgcgaaa atcgagcgtt tctatccgaa tgcggcggaa aaccaaggct accgtttcca 2073240
cgaggaagac acgcatatca tcatgaaagt ggaaacgcac aaccacccga ccgccatcgc 2073300
gccgtttgcg ggtgcggcga cgggcgcggg cggcgaaatc cgcgacgaag gcgcgacggg 2073360
caaaggttcg cgtccgaaag cgggcctgac cggctttacc gtgtccaacc tcaatattcc 2073420
cgacctcaaa cagccgtggg aacaagacta cggcaagccg gaacatattt cctcgccgct 2073480
ggacatcatg attgaaggcc cgatcggcgg cgcggcgttc aacaacgaat tcggccgccc 2073540
caacctcttg ggctacttcc gcacttttga agaaaaattt gacggtcagg ttcgcggcta 2073600
tcacaaaccg attatgattg ccggcggctt gggcagcatt caggcgcagc agacgcataa 2073660
agacgaaatc cccgaaggcg cattgctgat ccaactgggc ggcccgggta tgcttatcgg 2073720
cttgggcggc ggcgcggctt cttcgatgga taccggcaca aacgacgcgt ctttggactt 2073780
caactccgtg caacgcggca accccgaaat cgaacgccgc gcgcaggaag tcatcgaccg 2073840
ctgctggcag ctcggcggca aaaacccgat tatctccatc cacgacgtag gcgcgggcgg 2073900
cctgtccaac gccttccccg aactggtcaa cgatgccaga cgcggcgcag tattcaagct 2073960
gcgcgaagtg ccgcttgaag aacacggcct caacccgctg caaatctggt gcaacgaatc 2074020
gcaagagcgt tatgtgttgt cgattttgga aaaagatttg gatgctttcc gcgccatctg 2074080
cgaacgcgaa cgctgcccgt ttgccgtagt cggcacggcg actgacgacg gtcatttgaa 2074140
agtacgcgac gatttgttcg ccaacaatcc cgtcgatttg ccgttgaacg tcttgctcgg 2074200
caaactgccc aaaaccacgc gcaccgacaa aacggttgca ccgtccaaaa aaccgtttca 2074260
cgcgggcgat atcgacatta ccgaagccgc ctaccgcgtt ttgcgcctgc ctgccgtagc 2074320
cgccaaaaac ttcctgatta ccatcggcga ccgcagcgtc ggcggtttga cgcaccgcga 2074380
ccaaatggtc ggcaaatatc aaactccagt agccgactgc gccgttacca tgatgggctt 2074440
caacacctat cgcggtgaag cgatgtctat gggcgaaaaa ccgaccgtcg ccctgtttga 2074500
tgcgcctgct tcgggcagaa tgtgcgtcgg cgaagccatc accaacatcg cggcggtcaa 2074560
catcggagac atcggcaaca tcaaactctc cgccaactgg atggcggcgt gcggcaacga 2074620
aggcgaagac gaaaaactct accgcactgt cgaagccgtt tccaaagcct gtcaggcatt 2074680
ggatttgagc atccccgtgg gcaaagacag cctgtcgatg aaaaccgttt ggcaggacgg 2074740
cgaggagaaa aaatccgtgg tttcaccgtt gagcctgatt atctcagcgt tcgcgcctgt 2074800
gaaagacgta cgcaagactg ttacgcctga gttgaaaaac gtcgaagaca gcgtattgtt 2074860
gtttgtcgat ttgggcttcg gcaaagcgcg tatgggcggt tcggcgtttg gtcaggtgta 2074920
caacaatatg agcggcgacg cgcccgattt ggacgataca ggtcgtctga aagcctttta 2074980
cagtgtgatt cagcagcttg ttgccgaaaa caaactcttg gcgtatcacg accgcagcga 2075040
cggcggcttg tttgccgttt tggtagaaat ggcgtttgcg gggcggtgcg gcttggatat 2075100
agatttaaat ttattgcttg cacaaacatt tattaccaac cataccgctc tgtctcaatc 2075160
attgcggact gaagaggtaa aagcgttggc tgaatggcaa gaaaccattg cccgcacatt 2075220
atttaatgaa gagttgggtg ctgttatcca agttagaaaa caagatgttg ccgatattat 2075280
caatttattc tatcaacaac agctgcatca taatgtcttt gaaatcggta cgttaactga 2075340
tgagaacacg ttaatcatcc gcgacgggca aacgcacctt atttctgaca acctaatcaa 2075400
actgcaacaa acctggcaag aaaccagcca tcaaatccaa cgcctgcgcg acaaccctgc 2075460

ctgcgccgac agcgagttcg cactgattgg cgacaacgaa cgcagcgcat tgtttgccga 2075520
cgtgaagttc gacgtgaacg aagacatcgc cgcgccgttt atcaacagcg gcgcgaaacc 2075580
caaaatcgcc atcctgcgcg aacagggcgt aaacgggcaa atcgaaatgg ccgccgcctt 2075640
tacccgcgcc ggattcgatg cttacgacgt gcatatgtcc gacctgatgg caggccgcat 2075700
ccacctcgcc gacttcaaaa tgctggcggc gtgcggcggc ttcagctacg gcgacgtact 2075760
cggcgcgggc gaaggctggg cgaaatcgat tctgttccac cctgctctgc gcgaccagtt 2075820
tgccgccttc ttcgccgacc cggacacgct gacattgggc gtgtgcaacg gctgccaaat 2075880
ggtcagcaac cttgccgaaa tcatccccgg cacggcaggc tggccgaagt tcaaacgcaa 2075940
cctgagcgaa cagtttgaag cacgcctgag catggttcac gttccgaaat cagcgtcgct 2076000
gattctgaac gaaatgcaag gctccagcct gcctgtcgtg gtcagccacg gcgaaggccg 2076060
cgccgacttc gcgcttcacg gcggcaatat ttccgccgat ttgggcattg cgctgcaata 2076120
catcgacgga caaaaccaag tgacccaaac ttatccgctc aaccccaacg gctcgcctca 2076180
aggcatcgcc ggcgttacta acgccgacgg ccgcatcacc atcatgatgc cccaccccga 2076240
acgcgtgtac cgtgccgcgc aaatgagctg gaaaccggaa ggctggacgg aactgtccgg 2076300
ctggtaccgc ctctttgccg gcgcacgtaa agccttgggc taaccgccct actcaaacca 2076360
atgccgtctg aagaatattt cagacggcgt tccggcatac catcctttaa acggtatccg 2076420
tccaccgagg aacactcatg aaaatcaccc ccgtcaaagc cctaaccgac aactacatct 2076480
ggatgataca gcacggcaac catgccgtct gcgtcgaccc ttccgaaccc tcgcccgtct 2076540
tggaattcct cgtccgcaac cgcctcatgc ttgcccaaac atgggtaact cacccccatc 2076600
ccgaccacga gggcggtgcg gcggcactct ggcgcggcta catggaatcg cccgtttacg 2076660
gcgaatccga catcgaagca gcaacccaca ccgtaaccgc cggcacccaa ttcaccttcg 2076720
gcgacggaca ggttaccgtt tgggcaacac ccggccacac agaccgccac accagctacc 2076780
ttctcgaaac ttcagacggc atacacgtct tttgcggcga cacccttttt tccgccggct 2076840
gcggacgcgt gtttaccggc acaatcgaac agctttacga cagcttccaa cgcttcaacc 2076900
gcctgcctga aaacaccctg ttctatccgg cgcacgaata caccgccgcc aacctgcgtt 2076960
tcgccgccca tatcgagccg gacaacgccg acattcagac ggcactgaaa gcggcggcgc 2077020
atacgcctac cctgcccgtt accctcgcgc acgaacgccg cgtcaatccg tttttgcgcg 2077080
tcgacctgcc gcacgtcaga gaccgcgccg aggcattgag cgggaaaacg ttaaacagca 2077140
gcctcgatac ctttgtcgcg ctgcgtgaac ttaaaaacca ataccggacg aaataaaaca 2077200
acgggaaaac gcagccattc ctaggatttt tattaaaatc ttaaataaaa tcatacaatc 2077260
atcgccaata gacgaaagga caccgttgcc ttataatcaa acaaaaacaa aatatataat 2077320
atagtggatt gaatttaaat caggacaagg cgacgaagcc gcagatagta cggcaaggcg 2077380
aggcaacgct gtactggttt ttgttaatcc actatattgt taatccacta tataaatcca 2077440
gcacaaaacg ggatcggtga ttcttgtccg caagaatcgt tgattttctc tattacacgg 2077500
ataatcatca tgcgcttcac acacaccacc ccattttgtt ccgtattgtc caccctcggt 2077560
ctttttgccg tttcccctgc ttactcatcc attgtccgca acgatgtcga ttaccaatat 2077620
tttcgcgact ttgccgaaaa taaaggcgcg ttcaccgtag gtgcaagcaa tatttccatc 2077680
caagacaagc aaggcaaaat attaggcagg gttctcaacg gcatccccat gcccgacttc 2077740
cgcgtcagca accgccaaac cgccatcgcc accctggttc acccccaata cgtcaacagt 2077800
gtcaaacaca acgtcggcta cggttccata caattcggca acgacaccca aaatccagaa 2077860
gaacaagcct atacctaccg cctcgtatca cgcaacccgc acccggacta cgactaccac 2077920
cttccccgcc tcaacaaact ggttaccgaa atctcaccta ccgcactcag cagcgtaccc 2077980
ttgcttggaa acggccagcc aaaggccaat gcctacctcg ataccgaccg cttcccctac 2078040
tttgtacgac tcggctcagg cacgcaacaa gtccgcaaag cagacggcac gcgtacacga 2078100
accgccccgg cataccaata cctgaccggc ggcacgccgc tgaaagtatt ggggttccaa 2078160
aaccacggct tactcgtcgg cggcagcctg accgaccaac ccccttaacac ctacgcaatc 2078220
gccggagaca gcggttcccc cctgtttgcc ttcgacaagc atgaaaaccg ctgggtgctt 2078280
gcgggcgtac tcagcaccta cgccggcttc gataatttct tcaacaaata catcgtcacg 2078340
caacccgaat tcatccgttc caccatccgc caatacgaaa cccggctgga tgtcgggctg 2078400
accaccaacg aactcatatg gcgcgacaac ggtaatggca acagcaccct gcaagggctc 2078460
aacgaacgca tcaccctgcc cattgcaaac ccttcgcttg ccccacaaaa cgacagcagg 2078520
cacatgccgt ctgaagatgc cggcaaaacg ctcatcctat ccagcaggtt cgacaacaaa 2078580
acactgatgc tggcagacaa tatcaaccaa ggcgcaggcg cattgcagtt cgacagcaac 2078640
ttcaccgtcg tcggtaaaaa ccacacatgg caaggtgcag gcgttatcgt agccgacggc 2078700
aaacgcgtct tctggcaagt cagcaacccc aaaggcgacc ggctctccaa actgggcgca 2078760
ggcacgctta tcgccaacgg acaaggcatc aaccagggcg acatcagcat cggggaaggc 2078820
actgtcgtac tcgcccaaaa agctgcttca gacggcagca aacaagcatt caaccaagtc 2078880
ggcatcacca gcggcagggg cacggccgtc ctcgccgaca gccagcaaat caaacccgaa 2078940
aacctctatt tcggcttcag gggcggacgg ctcgacctca acggcaacaa ccttgccttt 2079000
acccatatcc gccatgcgga cggcggcgcg caaatcgtca atcacaaccc tgaccaagcc 2079060
gcgacactga cgctgaccgg caaccccgtc ctcagtcccg agcatgtcga gtgggtgcaa 2079120

tggggcaacc gtccgcaagg caacgcggcg gtttacgaat acatcaaccc gcaccgcaac 2079180
cgtcggaccg actacttcat actcaaaccc ggcggcaacc cgcgcgaatt tttcccgtta 2079240
aatatgaaaa actcaacaag ctggcaattt atcggcaaca acaggcaaca ggccgccgaa 2079300
caagtcgccc aagccgaaaa tgcccgcccc gacctgatta ccttcggcgg atacttgggt 2079360
gaaaacgcgc aaacgggcaa agccgcgccg agttacagca aaaccaatga agcagccata 2079420
gaaaaaaccc gccatatcgc aaatgccgcc gtatacggcc ggcccgaata ccgttacaac 2079480
ggcgcactca acctgcacta tcgtcccaaa cgcaccgaca gcacgctgtt gctcaacggc 2079540
ggcatgaacc ttaacgggga agtcttgatt gagggcggca atatgattgt gtcaggcagg 2079600
cccgtacccc atgcctacga ccaccaggcc aaacgcgaac ccgttcttga aaacgaatgg 2079660
accgacggca gcttcaaggc tgcacggttc accctgcgaa accatgcccg actgacggca 2079720
gggcgcaata ccgcgcatct ggacggcgac ataaccgcat acgatctgtc cggcatcgac 2079780
ctcggcttta cccaaggcaa aacaccggaa tgctaccgct cctaccatag cggcagcacc 2079840
cactgcacac ccaacgccgt tttaaaagcc gaaaactatc gtgcactacc tgcaacgcaa 2079900
gtacgcggcg acattaccct taacgaccgt tcagagctcc gcctgggcaa agcacacctg 2079960
tacggcagca tccgtgccgg caaagacacc gcagtccgca tggaagcaga cagcaactgg 2080020
acactttccc agtccagcca caccggcgca ctgacgcttg acggcgcaca aattaccctg 2080080
aaccccgatt tcgccaataa tacacacaac aaccgcttca acacactgac cgtcaacggc 2080140
acacttgacg ggttcggcac attccgattc ctgaccggca tcgtccgaaa acaaaatgcc 2080200
cccccccctca aactggaagg ggacagccgc ggcgcattcc aaatccacgt caaaaacacc 2080260
ggacaagaac ctcaaacaac cgaatcgctt gcacttgtga gcctcaatcc gaaacacagc 2080320
caccaagccc gattcaccct ccaaaacggc tatgccgatt tgggtgccta ccgctacatc 2080380
ctccgcaaaa acaacaacgg atacagcctg tacaacccgc tcaaagaggc cgaacttcaa 2080440
attgaagcca cgcgtgcgga acatgagcgc aaccaacagg catacaacca attacaggca 2080500
accgacatca gcagacaggt tcaacatgac tctgacgcga ccaggcaggc actacaggcc 2080560
tggcagaaca gtcaaaccga acttgcccgc atcgacagcc aagtccaata tctgtccgcc 2080620
caattgaaac agacagaccc gctgaccggc attctgacgc gtgcccaaaa cctgtgtgcc 2080680
gcacaaggat acagtgccga tatctgccgt caggttgcca aagccgccga cacgaacgac 2080740
ctgacactct tcgaaaccga actggatacg tatatagaac gtgtagaaat ggccgaatcc 2080800
gaacttgaca aagcacggca aggcggcgat gcgcaagccg tcgaaacagc ccggcacgcc 2080860
tacctgaacg cactcaaccg tctgtcccga caaatccaca gtttgaaaac cggcgttgcc 2080920
ggcatccgta tgccgaacct ggccgaactg atcagccggt cggccaacac cgccgtttcc 2080980
gaacaggccg cctacaatac cggccggcaa caggcgggac gccgcatcga ccgccacctt 2081040
accgatccgc agcagcaaaa catctggctg gaaaccggta cgcaacaaac cgactaccat 2081100
agcggcacac accgtcccta ccaacaaact accaactatg cacatatcgg catccaaacc 2081160
ggcatcaccg accgtctcag tgtcggtacg attttaaccg atgagcgcac aaacaaccgt 2081220
tttgatgaag gcgtatccgc ccgaaaccgc agcaacggcg cacatctgtt cgtcaaaggg 2081280
gaaaacggcg cactctttgc cgcggcagat ttaggctaca gcaacagccg tacccgattt 2081340
accgattatg acggggctgc cgtccgccgc cacgcatggg atgcaggcat caacaccggc 2081400
atcaaaatcg ataccggcat caacctcaga ccctatgccg gcatccgtat aaaccgcagc 2081460
aacggcaacc ggtacgtact cgacggcgca gagataaaca gcccggcgca aatccaaacc 2081520
acatggcatg ccggcatccg tctcgataaa accgtcgaac tgggtcaagc caagctgacc 2081580
cccgccttca gcagcgatta ctaccatacc cgccaaaaca gcggttccgc cctcagcgtc 2081640
aacgaccgta ccttactgca gcaagccgcc cacggcacac tgcataccct gcaaatcgac 2081700
gccggataca aaggctggaa cgccaaactt catgccgctt acggcaaaga cagcaacacc 2081760
gcccgccaca aacaggcagg aatcaaaata ggctacaact ggtaacaagc cgataaaaat 2081820
gccgtctgga acccgcgttt cagacggcat ttgcgttaaa aatagtaaac cgttccaaaa 2081880
gggagtagaa tagtgccgtt tccaaccctg cgcctgtacc gtcaggcttt tattatggac 2081940
cttcccagtt cgtttttact gaacacccca tccgattcaa acccgcaata accatcccgc 2082000
cggaatgcct ccccgcacac ggcgggcgga gcatttatga gcatcgaacc aacccctccg 2082060
aaccttgaaa acgacggtat cgaaaacgat gtagaacgcg tttccgccga tttcgaccgt 2082120
gtccactccc tctgcgaaat cctcgaacct gcttttgaac aaatcgaaaa cggtacaccg 2082180
ctcgaagacg cgccgctgcg cgacaagctg accgagctga ccgtcctctt gagcgagctg 2082240
caccctgccg acgtggcggc ggtattggaa tcgctaccgc cgcgcgaacg caatatcgtc 2082300
tggattctgg tcaaaccgga agacgacggc gaagtattgc tggaagtatc cgacgcggtg 2082360
cgcgaaacgc tgatcgagtc gatggacaaa gacgaattgt tggcagcggt cgatgatttg 2082420
gacgcggacg aattggcgga actggcagac gatttgccgc accaagtggt ttacgaagcg 2082480
ctacaaaccc gcgatgagga agaacgcgcc caagtcaaag cggcaatgtc ctacgaagac 2082540
aaccaagtcg gtgcgattat ggacttcgag ttggtcagca tccgcgccga tgtcgcctgt 2082600
gaagtggtgc tgcgctatct gcgccgcttc gacagcctgc ccgaccatac cgacaagatt 2082660
tttgtggtcg atgaaaacga cgtactgcag ggcgtgctgc ccatccgcaa acttttggtc 2082720
gccgatcccg aagacttggt ggaaaacgtg atggcgaaag atgtcgtgcg tttccgcgcc 2082780

gaagatgacg tggaagaagc ggcgcaggcg tttgaacgct acgacttggt taccgcgccc 2082840
gtcgtcgatg aaaacaaaaa gctcatcggc aggattacca tcgacgagat ggtggacgtg 2082900
atccgcgaag aatcggaagc ggatatgctg aatatggcgg gtttgcagga agaggaagac 2082960
ctgttcgccc ccgtgtggga ttcggtgaaa aaccgctgga tgtggctcgc cgtcaacctc 2083020
tgcaccgcct tcctcgccag ccgtgttatc ggcgcgtttg aaggcagcat cgaaaaaatc 2083080
gtcgcactcg ccgcgctgat gcccatcgtc gccggcatag gcggtaactc gggcaaccag 2083140
acgattacca tgattgtccg cgcgatggcg atggggcagc tgacggatat gcaggcgggg 2083200
cgtttgctga aaaaagaagt cggtgtcgcc ttggtcaacg gcatcatttg ggaacggtc 2083260
atgggcgcag tatcttggct gctttacggc agcctcggca tcgggctggt tatgattgcc 2083320
gcgatgacgc tcaacctcct gctggcggca accgtcggcg tattaattcc cgtggtaatg 2083380
gaaaagttcg gacgcgatcc cgcactgggc agctcggtgc tgattaccgc cgttaccgac 2083440
tccggcggct tcctgatttt cttggggctc gccaccctat tcctgcttta aatgccgtct 2083500
gaacccgcgc aaaaatgccg tctgaagcgg aagctgcttc agacggcatt tgactattta 2083560
tccttgttgc acaagattat tggacggtat gccggggcag ccctttggca acgccgacca 2083620
catcctcccc gaacagcgcg ttgacatcgg tttcgtcaaa cacatatttg ctgtggcaga 2083680
aatcgcaatc gacttcgatg ctgccttgtt ccaccaccac gccgccgact tcttccccgc 2083740
ccagcatcaa cagcatatcg ctgactttgc cgcgcgaaca ggtgcatgaa aattcaaacg 2083800
tttccggctc gaacacgcgc ggcggcgttt cgtggaacag gcggtataaa acgtgttgcg 2083860
cgtccagtcc tgccagctcc tccgccgtca gcgtgcgcgc cagcgtactg acgtgttccc 2083920
atgcctcttc atccaatacc tcttcaggca gacgctgcac cagcagaccg cccgccgctt 2083980
cgtcgcttgc agacaggacg atgtgcgtat caagctgttc ggaacgtttc atatagttca 2084040
ccaacatttg cgcgataccg ccgccttcca aaggcactac gccctgccag ggttcgccgt 2084100
ctttgggctg cagcgtcagc acgaatacgc cgccctcgcc caaaaggtcg ccgaggcttt 2084160
cgtcatcggc tatttctgcg gtttcgtccc aacgcgcggt tgcacggacg gtacggtcgg 2084220
aagccgcttc cgcaaccagc attttcagcc gcccccgccc ctgaacctgc acaatcagcg 2084280
tgccttcgtt tttgaggttg cccgacagca acacacccgc cgccaacaac tcacccaaag 2084340
cgcggcggat ggcggcggga tagtttttct gttttacaat gtgctgccac acgttttcca 2084400
gacggacgtg cagcccgcgc acgggcatat cgtcgaagat aaagcgggta cgcacatcgg 2084460
cgcggttgat ggcggtttga ttcatgattt tctctgactg attgttcgga tggcggctat 2084520
atggttgcgg tcggcgcgaa aacaagacgg acggcggatg cgcttcccaa attatcaata 2084580
aattatataa aaatcaacat attaactcaa tctaacaagc cgtttttttgc caaacagccg 2084640
ttttttttata tacaatcaac aagatatttt cgactgatac agcataacat cgcacggcgg 2084700
cacgatgcct cctgcgcgga aacaccgata tggattcttt tttcaaaccg gcagtttggg 2084760
cggttttgtg gctgatgttt gccgtccgcc ccgcccttgc cgacgagttg accaacctgc 2084820
tcagcagccg cgagcagatt ctcagacagt ttgccgaaga cgaacagccc gttttaccca 2084880
tcaaccgagc ccccgcccgg cgggcgggca atgccgacga actcatcggc agcgcgatgg 2084940
ggcttaacga acagcccgtt ttacccgtca accgagtccc cgcccggcgg gcgggcaatg 2085000
ccgacgaact catcggcaac gcgatggggc ttaacgaaca gcccgtttta cccgtcaacc 2085060
gagccccccgc ccggcgggcg ggcaatgccg acgaactcat cggcaacgcg atgggacttt 2085120
tgggtattgc ctaccgctac ggcggcacat cggtttctac cggttttgac tgcagcggct 2085180
tcatgcagca catcttcaaa cgcgccatgg gcatcaacct gccgcgcacg tcggcagaac 2085240
aggcacggat gggtacgccg gttgcccgaa gcgaattgca gcccggagat atggtgtttt 2085300
tccgcacgct cggcggcagc cgcatttccc atgtcggact ttatatcggc aacaaccgct 2085360
tcatccacgc gccgcgcacg gggaaaaata tcgaaatcac cagcctgagc cacaaatatt 2085420
ggagcggcaa atacgcgttc gcccgccggg tcaagaaaaa cgacccgtcc cgctttctga 2085480
actgatttcc caaggaatac gcaatgagta tgcccgaaat gcccaaatgg tacgacgatg 2085540
acggacagat cgtgtcctgt accgaaaagg tcaaagtgat gtccgaaaat atggccgagc 2085600
tgtatcagac ggcacaagac gcgtttgaag acgcgctgct gatgggttgc ggcgaacgtc 2085660
agttgcgcga ttacctgctc gcgctgattg aaggtttgga aaatccctac cgcaaagtct 2085720
gaacacgccc cggttgctgc ggcacggttt atccgtgccg tttttgcgtt tgtgcgcggc 2085780
ttcggctttt cagacggcat atttgacgtt atgattaaac agttaacaag atttatcaca 2085840
acgccgtcaa aagacagaca cacaacatga acatcatccg cgcgctcctc atcatcctcg 2085900
gctgcctcgc caccggcgaa accgccgttt tcctagcagg catcaaactg cccggcagca 2085960
tcgtcggcat gggcgtgctg tttgcgcttt tgcaggcggg ttgggtcaaa acgtcttggc 2086020
tgcaacagct taccgacgcg ctgatgtcga acctgacgct gttcctcgtg ccgccctgcg 2086080
tggcggtcat cagctatttg gatttgattg ccgacgattg gttttcgata ctggtttccg 2086140
cctccgccag cactttgtgc gtactgctgg ttacgggcaa agtccaccgg tggatacggg 2086200
gtattatccg atgaacgaaa tcctcaggca gcccagcgtt ctgcttttcc tcacgcttgc 2086260
cgtgtacgcg cttgcgatta tcgtgcgcac gcgcacgggc aatatcttct gcaaccccgt 2086320
actcgtcagc actatcgtgc tgattgccta cctgaaaatc ctcggtatcg attatgcggt 2086380
gtaccacaac gccgcgcaat tcattgattt ttggctgaaa cccgccgtcg tcgtgcttgc 2086440

cgtgccgctc taccaaaacc gccgtaaaat cttcaaccag tggctgcccg tcatcgtttc 2086500
acagcttgcg ggcagcgtta cgggcattgt tacagggatg tattttgcca aatggctggg 2086560
cgcggaacgc gaagtcgtcc tctcgctcgc gtccaaatct gttaccaacc ccatcgctat 2086620
tgaaatcacc cgctccatcg gcggcattcc cgccattacc gccgccaccg tcatcattgc 2086680
cggtctggtc ggacagattg ccggttacaa aatgctgaag aacacggtcg tcatgccctc 2086740
gtccgtgggt atgtcgctcg gcacggcttc gcacgcgatg gggattgccg cctcgctcga 2086800
acgcagccgc cgtatggcgg catacgcggg gctggggctg acgttcaacg gcgtactgac 2086860
cgcgctgatt gcgccgctgc tcatccccgt tttgggattt tgaacccgtt tcagacggca 2086920
tttcagccca tgctgtctga acgccgacac actcgcaagg agaaccgtta tggctgtcaa 2086980
cctgaccgaa aaaaccgccg aacaactgcc cgacatcgac ggcattgccc tctacaccgc 2087040
ccaagcaggc gtgaagaagc ccgggcatac cgacctgaca ctgattgccg tagccgccgg 2087100
cagcaccgtc ggtgcagtct tcacgaccaa ccgtttctgt gccgcgcccg tccacatcgc 2087160
caaatcgcac cttttcgacg aagacggcgt gcgcgccctc gtcatcaaca cgggcaacgc 2087220
caacgcgggt acgggcgcac agggcagaat cgatgctttg gcagtgtgtg ccgccgccgc 2087280
ccggcaaatc ggctgcaaac cgaaccaggt gctgcccttc tccaccggcg tgattctcga 2087340
accgctgccc gcagacaaaa tcatcgccgc cctgcccaaa atgcagcctg ccttctggaa 2087400
cgaagcggca cgcgccatca tgaccaccga caccgtgccc aaagccgcct cgcgcgaagg 2087460
caaggtcggc gacaaacaca ccgtccgcgc cacgggcatc gccaaaggct cgggcatgat 2087520
tcatcccaat atggcgacca tgctcggttt catcgccacc gatgccaaag tttcccaacc 2087580
cgtcctccaa ctgatgacgc aggaaatcgc cgacgaaacc ttcaacacca tcaccgttga 2087640
cggcgacacc agcaccaacg acagcttcgt catcatcgcc accggcaaaa acagccaaag 2087700
cgaaatcgac aacatcgccg acccgcgtta cgcccaactc aaagaattgt tgtgcagcct 2087760
cgcgctcgaa ctcgcccaag ccatcgtccg cgacggcgaa ggtgcgacca agttcatcac 2087820
cgtccgcgtc gaaaacgcca aaacccgcga cgaagcccgc caagccgcct acgccgtggc 2087880
acgttcgccg ctggtcaaaa ccgccttttt cgcctccgac cccaacctcg gcaggctgct 2087940
cgccgccatc ggttatgccg gcgttgccga cctcgatacc gacctcgtgg aaatgtatct 2088000
cgacgatatt ttggttgccg aacacggcgg acgcgccgca agctacaccg aagcacaagg 2088060
gcaggcggtg atgtcgaagg ccgaaatcac cgtccgcatc aagctgcatc gcggacaagc 2088120
cgccgccacc gtctatacct gcgacctgtc gcacggatac gtttccatca acgccgatta 2088180
ccgttcctga cccgacacgg cttcagacgg catacataaa atgccgtctg aaccgccgga 2088240
caacatacca tgacctccac attcccccgc cgcctcgccc gcaaaatccg ccaaacccgc 2088300
cgcctgtcgc gcaaaagcat cgcctttctg ttccttttgg caggttcggc actcgtcgcc 2088360
ctgaccgcgc tgtttttgc ccatcttgcc gattttgcgc tggaactgaa cgccaaactg 2088420
gttcaacaat acccgtggtt cgcgtgggtc gcgcttcctt tgggtttacc gcttattgcg 2088480
tggctcacac gcaaattcgc cccctcacc gccggcagcg gcatcccgca ggtcatcgcc 2088540
tcactgtcgc tgccctacgg cgcacagaaa acgcggctga tccgcctcgg gcagacgctg 2088600
ctgaagattc cgctaacctt tttgggtatg ctgttcggcg cgtccatcgg acgcgaaggt 2088660
ccgtccgtgc aggtcggcgc ggcagtgatg ggcgcgtggg gcgcgtggtg caagaaacac 2088720
ggcttggcat tcaaagggat gcaggaaaac gatttgatgg cggcgggcgc ggcgggcggt 2088780
ttggcagccg cgttcaacgc gccgctggcg ggcgtgattt tcgccattga ggaactcggg 2088840
cgcggcatca tgttgcgctg ggagaggcaa attcttttgg gcgtgctcgc ctccggtttc 2088900
atacaggtcg ccattcaggg caacaacccg tatttttccg gcttcaacgg cggcgtattg 2088960
gaacatatct ttctgtgggt cgcactgtcc ggcctggttt gcggcgcggc gggcgggctg 2089020
ttcggacgtt tgctctatcg cggtgcggcg gcgtttgcac cgcgcaagat acgcggcttc 2089080
atccgcaacc gtccgctgct gctggcggca ctgatggggc tgctgctcgc cctgctcggc 2089140
acgttctacc aaggcaaaac ctacggcacc ggctaccacg aagccgccca agccctgcac 2089200
ggcatctacg aagccccctt cggactcgcc gccgccaaat ggctcgccac cgtattcagc 2089260
tattgggcag gcgttccggg cggcattttc actccctcgc tgaccatagg cgcggttttg 2089320
ggcgagcata tcgccgccat cgccgacata tcgcagggtg caaacatcat cgtcctcatc 2089380
tgcatggcgg catttctggc gggcgcgaca caatccccga ttacttccgc cgtcgtcgtc 2089440
atggaaatga cgggcggaca aagcctgctg ttttggatgc taattgcctg cattttcgcc 2089500
tcgcaggttt cgcgccagtt ttcgccgcgt ccgttctacc acgcatcggg aatgcgcttc 2089560
cgccagcgcg tgcttcaaga aaccgccgcc caaaccggca atgcgcccgc aagaccgcaa 2089620
acagcaaaca gcaaaacggg aatgccgtct gaaaattaaa acgcccccga tcaaacgccg 2089680
gcagccgcct tgatttgaat accgttccgc cgccgcttga aatttcagca acaatgccgt 2089740
ctgaacgaca gaatgcggtt ttcagacggc atttccccat cccgatattg cctaaacaaa 2089800
accgaagcgt ttgctataat tctatttttt accgcatacg caccaatcat gtttcccgat 2089860
ttctcccaaa ccctctccaa agaccgccac ttcctgcgtt ccgccttcaa aaatcccaac 2089920
aaatacggcg gtttgtccaa aatcgaagaa aaataccgaa aatcgcacga aatctttttg 2089980
aagcgtttgg cagccttgcc aaaacccgaa ttcgacaaca ccctgcccgt tcacgagaag 2090040
ctcgaagaaa tcaaaaaagc cattgccaag aatcaggtaa cgattatttg cggcgaaacc 2090100

ggttcgggca aaaccacgca gttgcccaag atttgcttgg aactcgggcg tggggcggca 2090160
ggattgatcg ggcataccca gccgcgccgt ttggccgcgc gctccgtagc agagcggatt 2090220
gccgaagagc tgaaatccga aatcggcagc gcggtcggct ataaagtacg cttcaccgac 2090280
cacacctcgc gcgatgcctg cgtcaagctg atgaccgacg gcatcctgct ggcggaaacg 2090340
cagaccgacc gttatctcgc cgcctacgac acgattatca tcgacgaagc gcacgagcgc 2090400
agcctgaaca tcgacttcct tttgggctat ttgaaacaac tcctgccgcg ccgccccgat 2090460
ttgaaagtca tcatcacctc ggcaacgata gacgcagaac gcttctcccg acacttcaac 2090520
ggcgcgcccg ttttagaagt gagcggacgg acgtatcccg tcgaaatcct ctaccgaccg 2090580
ctgaccggca aagacgaaga cgacgcagaa gtggagttga ccgacgcgat tgtcgatgcg 2090640
gcggacgaat tagcgcgaca cggcgaaggc gatattttgg tattcctgcc gggcgagcgc 2090700
gaaatccgcg aaactgccga agccctgcgc aaatccacgc tgcgccgcaa cgacgaaatc 2090760
ctgcccctgt tcgcacgcct gtcgcacgcc gagcagcaca aaatcttcca cccctcaggc 2090820
gcgaaacgcc gcatcgtatt ggcaaccaac gtcgccgaaa cctcgcttac cgtgccgggc 2090880
atcaaatacg tcatcgacac cggcctcgcg cgtgttaaac gctattccgc acgggcgaaa 2090940
gtggagcagc ttcatatcga aaaaatctcc caagccgccg cccgccaacg atccggccgc 2091000
tgcggacgcg tctccgcagg cgtgtgtatc cgactgtttt cagaagaaga ttttaacagc 2091060
cgccccgaat ttaccgaccc cgaaatcgtc cgcagcaacc tcgccgccgt catcctgcgc 2091120
atggcagcat tgaaactcgg cgatgtggcg gcattcccgt ttttagaaat gcccgattca 2091180
cggtatatca atgacggttt tcaggtgttg ttggagttgg gggcggtgga ggccgtctga 2091240
aaacaggcag acataaaaga aaatccgcgt agagtgatgt aaacttaccc ttgctttaat 2091300
aagtagaaaa tggtgggttt acgtcccccc ctgcggctac taaaaaaata taagagtaaa 2091360
caaccttttt gaaagaaaaa tgtatggacg aaattcaaat acccaaaaaa gtggaattac 2091420
aaaccaaact agaaaatgaa aagattgttt tatcgaaagg ttctaccacg attattgttg 2091480
gtgctaatgg cacagggaaa acaagattag ctgtttatat tgaagaacaa ttaaaggaaa 2091540
aagcacacag aatttcggct catagagcat taaaattaaa ccctaatgtc aataaaatac 2091600
cagaaaagag tgccaaaaca tatctatctt atggtcagaa ctgggatgga atcgatgtat 2091660
caaatagaaa aaattataga tgggataata actcatatac tcatttactc aacgattttg 2091720
attggttatt acaatattta ttcgctcaac aaaataatat tgcggtagca aataatcaaa 2091780
agctcaaccg taatgaaaaa gtaaccaatt caaaaacaaa gctagatatt ttgcaagaag 2091840
catgggaaac attattacca cacagaaaat tacatattac agcagatgat attcaagtct 2091900
ctgctgtaga taatgaggaa ttgtattctg cctcaaatat gagtgatgga gagcgagcac 2091960
ttttctatat tcttggacaa gttttgtcag tagatgacgg ttctgtctta atttttgatg 2092020
agcctgaatt acatattcat aaatcaatta tttcaaatct atgggataaa attgaagaat 2092080
tacgacctga ttgttcattt ctaatcatta cacacgatat tgaatttgct gcaactcgag 2092140
tagctaaaaa atatgttatc agaaattatt atccgacccc tgcttgggat atttctgaag 2092200
ttcctgaaag taattttgat gaagaaacaa taacgatgat tttaggtagc cgtaagccaa 2092260
tattatttgt tgagggcaac aataatagtt tagatattgc tacttaccgc tattgttatc 2092320
ctgattggac catcataccc aaaggggcat gcaaagatgt cattcaatca gtatcatcgc 2092380
tgaaaaaatt aagtaatgaa atgccattac taaacttaaa atgttcaggt attgtcgatt 2092440
tagatagtag ggatgaaaga gaaattgaac aattaaataa tttgggtatt tacattttac 2092500
ctgtatccga aattgaaaat ctttttagct taactgatgt agcaaaagag atattgaaac 2092560
taaatcaata ttcagatgaa gaattactca ataaacttaa tggatttaaa tccgaactaa 2092620
ttaaatatat agataatgaa ttaaaagacg ataaattaga cgaatttgtt gtaaaacagg 2092680
ttcgacgtaa aattgataat tatttaaaaa atattgattt atcctccaaa ataacaagta 2092740
ctgatatgaa aaaatcatta cttaatgaaa tttctacttt aacagaacag aaaattgaaa 2092800
catggatttc agaaattaaa aatgaaattc aaagatgtat tgaacagcaa gatttggata 2092860
aattacttac tatatatgat aataaaggac tcttggctaa atcagcttgt gttttaaaag 2092920
gaatgcgtaa caaacatgaa tttgaaagct ggataatgag aacattaaaa ggaaggaata 2092980
aagattttat tgatgcaatc agacagaaac ttccaattct ggattaaata aaaccatctg 2093040
aaaatttacc ttcagataca gatatatttc atgaaaaatc atcaaactac actctctttc 2093100
cctacttcga gtagcctgaa accttgcgca gacaaacaag gcctgtctga agaccgcagc 2093160
caataccgcc tgaccaaact cggcgaacaa atggcgcacc tgcctatcga cccgaaaatt 2093220
gcgcgtattt tgttagtatt attccgtttt taaaaatgcc cgattcgcgg tatatcaatg 2093280
acggttttca ggtattgctg gaattggggg cggtggaggc cgtctgaaaa taaaatcttt 2093340
ctttataaaa aggcaggcca tgtttcattt tcagacggcc taaatcattg agaaactaaa 2093400
aactattaaa aagggaatat tgggttttaa aactcaatcg gtaaattttt attgtgaaat 2093460
attaatgatg aaaaaatctt tccttacgct tgttctgtat tcgtctttac ttaccgccag 2093520
cgaaattgcc tatcgctttg tatttgggat tgaaacctta ccggcggcaa aaattgcgga 2093580
aacgtttgcg ctgacatttg tgattgctgc gctgtatctg tttgcgcgtt ataaggtgac 2093640
gcgtttgttg attgcggtgt tttttgcgtt cagcattatt gccaacaatg tgcattacgc 2093700
ggtttatcaa agctggatga cgggcatcaa ttattggctg atgctgaaag aggttaccga 2093760

agtcggcagc gcgggtgcgt cgatgttgga taagttgtgg ctgcctgtgt tgtggggcgt 2093820
gttggaagtc atgttgtttt gcagccttgc caagttccgc cgtaagacgc atttttctgc 2093880
cgatatactg tttgccttcc taatgctgat gattttcgtg cgttcgttcg acacgaaaca 2093940
agagcacggt atttcgccca aaccgacata cagccgcatc aaagccaatt atttcagctt 2094000
cggttatttt gtcggacgcg tgttgccgta tcagttgttt gatttaagca ggattcccgc 2094060
ctttaagcag cctgctccaa gcaaaatcgg gcagggcagt gttcaaaata tcgtcctgat 2094120
tatgggcgaa agcgaaagcg cggcgcattt gaagctgttt ggctacggac gcgaaacttc 2094180
gccgttttta acccggctgt cgcaagccga ttttaagccg attgtgaaac aaagttattc 2094240
cgcaggcttt atgactgcag tgtccctgcc cagttttttc aatgcgatac cgcacgccaa 2094300
cggcttggaa caaatcagcg gcggcgatac caatatgttc cgcctcgcca aagagcaggg 2094360
ctatgaaacg tatttttaca gcgcgcaggc ggaaaacgag atggcgattt tgaacttaat 2094420
cggtaagaaa tggatagacc atctgattca gccgacgcaa cttggctacg gcaacggcga 2094480
caatatgccc gatgagaagc tgctgccgtt gttcgacaaa atcaatttgc agcagggcaa 2094540
gcattttatc gtgttgcacc aacgcggttc gcacgcccca tacggcgcat tgttgcagcc 2094600
tcaagataaa gtattcggcg aagccgatat tgtggataag tacgacaaca ccatccacaa 2094660
aaccgaccaa atgattcaaa ccgtattcga gcagctgcaa aagcagcctg acggcaactg 2094720
gctgtttgcc tatacctccg atcatggcca gtatgttcgc caagatatct acaatcaagg 2094780
cacggtgcag cccgacagct atctcgtgcc gctagtgttg tacagcccgg ataaggccgt 2094840
gcaacaggct gccaaccagg cttttgcgcc ttgcgagatt gccttccatc agcagctttc 2094900
aacgttcctg attcacacgt tgggctacga tatgccggtt tcaggttgtc gcgaaggctc 2094960
ggtaacgggc aacctgatta cgggtgatgc aggcagcttg aacattcgcg acggcaaggc 2095020
ggaatatgtt tatccgcaat gagtggcgta aaaaccaata aagacaaatt tagatgatgt 2095080
cggggaagat gcccgaccga caagactatg caaaatatga aaaaccaagt acgcggatca 2095140
ggcatggatg cccgatccaa tccggccaat gtttcagacg gcctgcaaaa cagttcgggt 2095200
catatcggta ccaacacgcg ttaccgcctg accaaactcg gcgaacagat agcgcgccta 2095260
cccatcgacc cgaaaatcgc gcgcattttg ctggcggcga agaaacacga ctgcatggcg 2095320
gaaatattgg tgattgcgtc cgcgctgtcg attcaagacc cgcgcgagcg gccgctagaa 2095380
gcgcgcgatg cctcagccaa ggcgcacgag cgttttaccg acaagcagtc cgatttcctt 2095440
gcctatctga acatttggga cagcttccag cgcgaacgcg ataaaggctt gtccaacaag 2095500
cagctggtgc agtggtgccg ccaatatttc ctgtcgcacc tgcggatgcg cgagtggcgc 2095560
gagctgcacc accagcttgc ccaaaccgcg attgaaatgg gtttaaccac caaggaagcc 2095620
gctttcagac gacctcccga agtcaggcag ctcacgtcgt ctgaaaatgc gggtgaccaa 2095680
gacctatctg ctaaactcaa acaaaaacaa ctggataaaa agcaacaccg cgcccaaatc 2095740
cgcgccgcca aagaagcggg ctacgaacaa atccaccgcg ccctgctcac tggccttatc 2095800
gccaacgtcg gcatgaaatc gcccgacggt aacgactaca ccggcgcgcg cggcagccgc 2095860
ttccaccttt tccccgcctc cgccctgttc aaagccaaac ccaaatgggt gatggcggca 2095920
gaattggttg aaaccacgcg cctttacgcg cgcgacgtcg ccgttatcca gcccgaatgg 2095980
atagagcagg aagcgccgca cctcgtccgc tatcattatt tcgagccgca ttgggaacaa 2096040
aaacgcggcg aagtcgtcgc cagcgaacgc gtgacgcttt acggtctgac cgtattgccg 2096100
cgccgccccg tgtcttacgg caaagttgcc cccgaagaag cgcgcgaaat ctttatccgc 2096160
agcgcgttgg tggcgcagga atgcgatttg aaagcggatt tttttgtcca caacaaaaag 2096220
ctgattaaag aaattaccga actcgaacac aaatcgcgca agcaagacgt gctggtcgat 2096280
gacgaagccc tgtttgcgtt ttataacgaa cgactgcccg aaatggcttg gaaagacgcg 2096340
caaggcagcg tttggggaag tgaagattcc gtacggatta ttgaatctga caaagccgag 2096400
aggtcgtctg aaaatgagcg caacgagttt cgtaaaaaca agcgtaatgg gtctcgccaa 2096460
aatgaaaatc acggcaacac cgtaggttgg gttgaaaacc caacatcagc cgcaactgca 2096520
aaaactgttg ggtttgacaa tccaacctac gctgcccaac aaaccacccc ctcccccgtg 2096580
ggggagggtc ggggagaggg caaaacagtt gccgcacaaa ccaacttttc cgcaaccgca 2096640
gcaaaccctc tccctaaccc tctcccgcag gagagggaac agagtgccgc agcttcaacg 2096700
atttcagacg acctgcgtcc tgcaaatctg cagcaaaccg ccccctcccc cgtgggggag 2096760
ggctggggag agggcaaaac agttgccaca caaaccaact tttccgcaac ctcaacaaac 2096820
cctctcccgc aggagaggga acagagtgcc tcagcttcaa cgttttcaga cgacctgcgt 2096880
cctgcaaatc tgcagcaacc ctctccctcc cccgtggggg agggctgggg agagggcaaa 2096940
acagttgcca cacaaaccaa cttttccgca acctcaacac tttcagacga ctccaaaccc 2097000
aaaaagcagc ctgcacccca aaaaaaccgt ctgaaacccc taccccctcgc cgacatccgc 2097060
accttccaag cctggctcaa aaccgccgag cgcgacaatc cgcgcctgct gttcctcagc 2097120
cgcgacgatc tgatgcaaca cgccgccgca cacattaccg aagaacagtt ccccaaattc 2097180
tggcaaaccg cagacggcaa attcaaactt tcctaccgct tcgagccgca ccatccgcta 2097240
gacggcgtga ccatgaccgt gccgctgacc gtcctcaacc gcctgcacgc gccgtcgctc 2097300
gaatggctgg tgcccggcat gatacgcgaa aaaatccagt tgcaaatcaa agcactgccc 2097360
aagcaaatcc gccgcatctg cgtgcccgtg cccgaattca tcacccaatt tttaagccaa 2097420

aaccccgacc gcaacgcccc catcctgccc caactcgccc aagccatcgc caaaaccgca 2097480
ggcgacatcc gcatattcga gcaaatcaac caagacgaat gggccgcgtt caggctgccc 2097540
gaacactgct atttcaacct ccgcattatc gacgacggcg gacaagagct tgccggcggc 2097600
cgcaaactgc acgaattgca acaacaactc ggtcaagctg ccgccgttac cttccgtgac 2097660
aacacccaag aatttgagcg cgacaacgtc accgcatggg acatcggcac cctgcccgaa 2097720
tccatcaaat tcgcacgcgg caaacaacag ctcaccggct atctcggcct acaaaaagaa 2097780
aaagacggcc gcatcgccct gcgcctgttt gataccacag aagccgcaga gcaggcacac 2097840
cgtcaaggtg tcatcgaatt gatgaagctg caattaaaag agcaggtaaa ggatttgaac 2097900
aaaggcatcc aaggcttcac ccaagctgcc atgctgctca aacacatcaa cgccgacact 2097960
ctgcgcgacg acctcaccca agccgtctgc gaccgcgcct ttatcggcga agacgagctg 2098020
ccgcgcaacg aaaaagcctt caaagaacaa atcaaacgcg cccgcagccg cctgcccgcc 2098080
gtcaaagaag ccctcagccg ctacctgcag gaaaccgccg ccgtctacgc cgaactcaac 2098140
agcaaactcg gcaaacaccc attgacccac cttctaagac tacgcctgca aaccctgctc 2098200
gccgccggct tcgccacccg aaccccgtgg gcacaatggc cgcgcctccc catctacctc 2098260
aaagccatga ccctgcgcct cgaaaaatac agcagcaacc ccgcccgcga cgcagcccgc 2098320
gaagccgata tccaagagct ggaacaaatg tggcaggaaa aaacagacag cctgattaaa 2098380
caaggtctcc ccatttcaga cggcctcgcc gcgtttaaat ggatgattga agaattgagg 2098440
gtgtcgctgt tcgcgcagga attgaagaca ccgtatccgg tgtcggtgaa gcggctgttg 2098500
aaagagtggg aaaaaattga aaaataaaaa aacagcctga aaagtttcag gctgtttttt 2098560
tatttgacta atcgaagttt cctatatcta tttaagtccc tctcaactaa tccaaaagtt 2098620
aaatcagcaa catctttggg ggatacgttt aaattttcag caatctgttc aataccaatg 2098680
ccatcatttt ttaaaatagt aagcatttta cgtaatgcgc ttgatatttc cctttccatt 2098740
ggctctggtt cgatagttcg atattttttc tttgcaaaca aaggacaaag attgtgtata 2098800
tacatcctat cagtaatcat tcctaattta tgcatccgat atgctaaggc aacaagtgat 2098860
acaccaaatc gtcttttgat ttttaataaa ttttcaatag tgataggaac atgacgatat 2098920
aagcgtagtg cagcctccgg cattaaaaaa gctgaagcaa aggcattagc ctctttttcg 2098980
ataatatcac gaggttcatc ttctgtaatt tcactatttt tactatgttc catactgtat 2099040
ttatcacgga ttaagtgccc taattcatgg gcagcatcaa atcgactacg ttctgcagat 2099100
ttttgtgtat ttaaaaatac aaatggatga ttttcatacc aagtacaaaa ggcatcaatg 2099160
tcctttgtat ctaaagataa tgaaaataca cgaacaccct taacttcaag tagggtgatc 2099220
atattcggaa taggttcatt gccaagcccc cattctaatc ttagttcctg agcagcctct 2099280
tcaggagaaa tatcagaaaa atcaggcaat acggcttgac ttagtgtaaa ttctgtctcg 2099340
agccagtcat ttaacaaaaa agccgtaatg ctatgattta atgcttgttt ttcaagcctc 2099400
ttcgaggtgc gtgaacgagc acgaaaactt actgcctgag atttcaactc aggcagtctt 2099460
tcgtcattag taaagaaatg aactggaaac tctaataaat tggctaattc atttaaatca 2099520
ggtatttgct catcttttac atagtttcta acctgtcgag cggtaatacc taataactca 2099580
gctaattttg tttgcgtaca accacgttta tccagcgcaa attccagtct ctcacgatta 2099640
aatgtctgca tgatttatca ttcaaattat cctgcttttt gttttctttc aaccaaaggc 2099700
tcatattctt caacaggttg cttacgttca agctcatcaa atttagttaa atcaacatca 2099760
gctaatataa ttcgctgctt gtacccagtt atttgatgac taacaaaacc actcggtaaa 2099820
gataattcaa gttgcacttt attatacttc cagtgaaaca gcagaaccca aaactgcaca 2099880
gtatcaggca aatctagttt tgaattacga atagcctcct caaatccctt acctttcctt 2099940
gcggttgtca ttggcatccc gtgatgccta ccaacatctg aagtagcagt agccacaata 2100000
atacttttag ttcgacatgg cgagagacat agaaatgcac cacccgacca aggctcaagc 2100060
gtccagccat ctttacttaa atataccctt agagcaaatg taatttctgc ttgccgatac 2100120
atccccaatg tatttctgtc agataatgct gctttgtcct gaatattatt atgcgcagta 2100180
agtacaatct ctttaagcat ctcctgagat aagtacttgc tgatttcact taaagcaata 2100240
tcactatttt gttgctcgac tatttctcct acttcaaatg ggaaaggttc tgataatgca 2100300
aattccacca taaaaatttc ctaattttat acgtaatgtt tacacaatat atcaggaaat 2100360
atgaaaacgt acaactatat ctataaagca attaataagt agcctgccca accgtgtcct 2100420
tatctttcgg cacacccgac ctgcaaatca cgcaaaactt ggaatccgtg tgtagggtgt 2100480
gtgcggtaca tacgcacgca gtctttttaa accacagccc ttcccaacta aaccaaaagg 2100540
tcgtctgaac cctattttca gacgaccttt tgccactttg taaaacaaat cttcccacca 2100600
tcctctcccc aaacatcgcc cgaaccagta aacttctcat atttcaacaa ctccttggaa 2100660
gcaaaccatg tctggtatct acctaccccg cctattcccg ccccatatcg ccgaacgcgg 2100720
cctgttgtat tttcagcagg gcaaggttct cgatgtccga aaaacttccg ccgggcatta 2100780
tcgggcggag gtgtgcggtt cggaaaacta ttgggtatag ttgaagctgg atagtgattt 2100840
gtatattaaa gacgaaggct gcaattgtcc ttatatctaa gagtgcaaac ataccttaaa 2100900
ttactatatt gcataggcaa aatacaagcc tataacgaat tggaaacaaa atgccgtctg 2100960
aaaacatctt cagacggcat tataaaatct gttcaccttt tcagatgagt aatgtacacc 2101020
cttatacaat ttttgctact atgccccata aatccacggc taaagatatc cttattatgt 2101080

cctatgattt atcgaaacga cttgtaatcg gcttagcatc aagtgcccta ttcgacttat 2101140
ccgaatcgga taatatattt agaatggaag gggcagaaac ctataggcaa tatcagagag 2101200
aaaaacaaaa ccatccccta aaaaaggcgt tgtctttcca tttattaaaa aacttctgtc 2101260
aatcaatgaa ataaacccaa acgacccaac gattgggttt attcttttat ccagaaacaa 2101320
tccagataca gattacgagt cataactata ggcttaatat tacacgattc tcattccatc 2101380
aaggcggaaa accgcacaaa tactgaaaca ctatcgatcg atttgtaaac aagcctactt 2101440
aagtaacttg cagtccttat catttccttt aaaataatcc agcccgtcac tacacgaact 2101500
ggcggacttc ttgcaaataa aggttactag attttcattc atcttaataa taaaaggatt 2101560
tttatcttta tctatggcta ccgccttcaa catgaattta ctgtctaaag ccccgcgcgc 2101620
gattccattc aaacggatac aaaagccttc tgcctcttta atcggcaaac ttggccactt 2101680
ggtagatgtt tgtttaaacc tcccattctg cagataaaac ttttccataa aatgtgcatt 2101740
ttctaacaag gctgcccgca ctgcatttat ctttgctttc tcaacataat tgcgatagct 2101800
cggataaaca attaaagcaa gtacagacaa tatcaagacc actgatatta attcaaccag 2101860
cgtaaacccc cgattatcag tcattacttt acttccaata agaacagatt attcaacata 2101920
tttctttgaa cagacttact atcccattca acagtatgca tatttcccac tctatttttt 2101980
agcggccggt atagccggtt tggctgggcc ttttggtgcg ggcgcgccga ccgaagcctg 2102040
gtccttcagc ttcgccagca ccgcagggcc gatgcccttt accttggtca aatcgtctac 2102100
agacttgaac gcaccgtttt gcgcacggta ttccgcaatg gccttcgcct tcgccgggcc 2102160
tatgcccggc agcgcctcca actcctgctg cgaagccgca ttgatgttta ccgccgcaag 2102220
ggagaaggcg caggagaaca gcatacagaa cagcacgaac attttcttca tggttttttcc 2102280
tttaagggtt gcaaacaata aaccgcatct tgcgacgata aaacgagtca ttctaaaatg 2102340
aatatcccaa agtttcaagc cgttcctccg caaacccgac cggacaccgt acggatgccg 2102400
tcccgccatc accgacattt tttccgggca aagcaaacat tttttccggg caaagcaaaa 2102460
acccccgaat aatcgggggt tttctgaatg ggtgtttggc agtgacctac tttcgcatgg 2102520
aagaaccaca ctatcatcgg cgctgagtcg tttcacggtc ctgttcggga tgggaaggcg 2102580
tgggaccaac tcgctatggc cgccaaactt aaactgttac aaatcggtaa agccttaatc 2102640
aatatattcg gtaatgactg aatcagtcag taagctttta tctcttgaag ttcttcaaat 2102700
gatagagtca agcctcacga gcaattagta tgggttagct tcacgcgtta ccgcgcttcc 2102760
acaccccacc tatcaacgtc ctggtctcga acgactcttt agtgcggtta aaccgcaagg 2102820
gaagtctcat cttcaggcga gtttcgcgct tagatgcttt cagcgcttat ctcttccgaa 2102880
cttagctacc cggctatgca actggcgtta caaccggtac accagaggtt cgtccactcc 2102940
ggtcctctcg tactaggagc agcccccgtc aaacttccaa cgcccactgc agatagggac 2103000
caaactgtct cacgacgttt taaacccagc tcacgtacca ctttaaatgg cgaacagcca 2103060
tacccttggg accgactaca gccccaggat gtgatgagcc gacatcgagg tgccaaactc 2103120
cgccgtcgat atgaactctt gggcggaatc agcctgttat ccccggagta ccttttatcc 2103180
gttgagcgat ggcccttcca tacagaacca ccggatcact atgtcctgct ttcgcacctg 2103240
ctcgacttgt cggtctcgca gttaagctac cttttgccat tgcactatca gtccgatttc 2103300
cgaccggacc taggtaacct tcgaactcct ccgttacgct ttgggaggag accgccccag 2103360
tcaaactgcc taccatgcac ggtccccgac ccggatgacg ggtctgggtt agaacctcaa 2103420
agacaccagg gtggtatttc aaggacggct ccacagagac tggcgtctct gcttctaagc 2103480
ctcccaccta tcctacacaa gtgacttcaa agtccaatgc aaagctacag taaaggttca 2103540
cggggtcttt ccgtctagca gcgggtagat tgcatcttca caaccacttc aacttcgctg 2103600
agtctcagga ggagacagtg tggccatcgt tacgccattc gtgcgggtcg gaacttaccc 2103660
gacaaggaat ttcgctacct taggaccgtt atagttacgg ccgccgttta ctggggcttc 2103720
gatccgatgc tctcacatct tcaattaacc ttccagcacc gggcaggcgt cacaccctat 2103780
acgtccactt tcgtgttagc agagtgctgt gtttttaata aacagtcgca gccacctatt 2103840
ctctgcgacc ctccggggct tacggagcaa gtccttaacc ttagagggca taccttctcc 2103900
cgaagttacg gtatcaattt gccgagttcc ttctcctgag ttctctcaag cgccttagaa 2103960
ttctcatcct gcccacctgt gtcggtttgc ggtacggttc gattcaaact gaagcttagt 2104020
ggcttttcct ggaagcgtgg tatcggttgc ttcgtgtccg tagacactcg tcgtcacttc 2104080
tcggtgttaa gaagacccgg atttgcctaa gtcttccacc taccggctta aacaagctat 2104140
tccaacagct tgccaaccta accttctccg tccccacatc gcatttgaat caagtacagg 2104200
aatattaacc tgtttcccat cgactacgca tttctgcctc gccttagggg ccgactcacc 2104260
ctacgccgat gaacgttgcg caggaaacct tgggctttcg gcgagcgggc ttttcacccg 2104320
ctttatcgct actcatgtca acattcgcac ttctgatacc tccagcacac tttacaatgc 2104380
accttcatca gcctacagaa cgctccccta ccatgccggt aaaccggcat ccgcagcttc 2104440
ggttatagat ttgagccccg ttacatcttc cgcgcaggac gactcgacca gtgagctatt 2104500
acgctttctt taaatgatgg ctgcttctaa gccaacatcc tggctgtctg ggccttccca 2104560
cttcgtttac cacttaatct atcatttggg accttagctg gcggtctggg ttgtttccct 2104620
cttgacaacg gacgttagca cccgctgtct gtctcccgag gaaccacttg atggtattct 2104680
tagtttgcca tgggttggta agttgcaata accccctagc cataacagtg ctttaccccc 2104740

atcagtgtct tgctcgaggc actacctaaa tagttttcgg ggagaaccag ctatctccga 2104800
gtttgtttag cctttcaccc ctatccacag ctcatccccg cattttgcaa catgcgtggg 2104860
ttcggtcctc cagtacctgt tacggcacct tcaacctggc catggataga tcactcggtt 2104920
tcgggtctac acccagcaac tcatcgccct attaagactc ggtttcccta cgcctcccct 2104980
attcggttaa gctcgctact gaatgtaagt cgttgaccca ttatacaaaa ggtacgcagt 2105040
cacaccacta gggcgctccc actgtttgta tgcatcaggt ttcaggttct gtttcactcc 2105100
cctcccgggg ttcttttcgc ctttccctca cggtactggt tcactatcgg tcgatgatga 2105160
gtatttagcc ttggaggatg gtccccccat attcagacag gatttcacgt gccccgccct 2105220
acttttcgta cgcttagtac cgctgttgag atttcgaata cgggactgtc acccactatg 2105280
gtcaagcttc ccagcttgtt cttctatctc gacagttatt acgtacaggc tcctccgcgt 2105340
tcgctcgcca ctacttgcgg aatctcggtt gatttctttt cctccgggta cttagatggt 2105400
tcagttctcc gggttcgctt ctctaagtct atgtattcaa cttaggatac tgcacagaat 2105460
gcagtgggtt tccccattcg gacatcgcgg gatcattgct ttattgccag ctcccccgcg 2105520
cttttcgcag gcttacacgt ccttcgtcgc ctatcatcgc caaggcatcc acctgatgca 2105580
cttattcact tgactctatc atttcaagaa cttctttgac tttgcctaac attccgttga 2105640
ctagaacatc agacttgaat ttcctacttt gataaagctt actgctttgt tgtgtcttaa 2105700
tcctgccttt tgtgtttcag gattaagtcg atacaatcat cacccaaata ctgtgtttgt 2105760
tttcttttct cttgcgagag atttttatcc tttgcaaaga ataaaaaatc aaaacaaacg 2105820
ctttgtcttt gtttgttgat ttcggctttc caatttgtta aagatcgatg cgttcgatat 2105880
tgctatctac tgtgcaaatc aaaacgagct gattattata tcagcatttt gttcttggtc 2105940
aagtgtgacg tcgccctgaa tggattctgt tccattcttc cgttttgatt tgtacagtat 2106000
tggtggaggc aaacgggatc gaaccgatga ccccctgctt gcaaagcagg tgctctacca 2106060
actgagctat gcccccgttc ttggtgggtc tgggaggact tgaacctccg accccacgct 2106120
tatcaagcgt gtgctctaac cagctgagct acaaacccgg attctcttct taagcgaatc 2106180
ttgccttcac tcaagcttct tccgcatctt tttcagttta ccgataagtg tgaatgccta 2106240
aagcctcttc tttctctaga aaggaggtga tccagccgca ggttccccta cggctacctt 2106300
gttacgactt caccccagtc atgaagcata ccgtggtaag cggactcctt gtggttatcc 2106360
tacctacttc tggtatcccc cactcccatg gtgtgacggg cggtgtgtac aagacccggg 2106420
aacgtattca ccgcagtatg ctgacctgcg attactagcg attccgactt catgcactcg 2106480
agttgcagag tgcaatccgg actacgatcg gttttgtgag attggctccg cctcgcggct 2106540
tggctaccct ctgtaccgac cattgtatga cgtgtgaagc cctggtcata agggccatga 2106600
ggacttgacg tcatccccac cttcctccgg cttgtcaccg gcagtctcat tagagtgccc 2106660
aactgaatga tggcaactaa tgacaagggt tgcgctcgtt gcgggactta acccaacatc 2106720
tcacgacacg agctgacgac agccatgcag cacctgtgtt acggctcccg aaggcactcc 2106780
tccgtctccg gaggattccg tacatgtcaa gaccaggtaa ggttcttcgc gttgcatcga 2106840
attaatccac atcatccacc gcttgtgcgg gtccccgtca attcctttga gttttaatct 2106900
tgcgaccgta ctccccaggc ggtcaatttc acgcgttagc tacgctacca agcaatcagg 2106960
ttgcccaaca gctaattgac atcgtttagg gcgtggacta ccagggtatc taatcctgtt 2107020
tgctacccac gctttcgggc atgaacgtca gtgttgtccc aggaggctgc cttcgccatc 2107080
ggtattcctc cacatctcta cgcatttcac tgctacacgt ggaattctac ctccctctga 2107140
cacactcgag tcacccagtt cagaacgcag ttcccgggtt gagcccgggg atttcacatc 2107200
ctgcttaagt aaccgtctgc gcccgcttta cgcccagtaa ttccgattaa cgctcgcacc 2107260
ctacgtatta ccgcggctgc tggcacgtag ttagccggtg cttattcttc aggtaccgtc 2107320
atcagccgct gatattagca acagcctttt cttccctgac aaaagtcctt tacaacccga 2107380
aggccttctt cagacacgcg gcatggctgg atcaggcttg cgcccattgt ccaaaattcc 2107440
ccactgctgc ctcccgtagg agtctgggcc gtgtctcagt cccagtgtgg cggatcatcc 2107500
tctcagaccc gctactgatc gtcgccttgg taggccttta ccccaccaac tagctaatca 2107560
gatatcggcc gctcgaatag cgcaaggccc gaaggtcccc tgctttctct ctcaagacgt 2107620
atgcggtatt agctgatctt tcgatcagtt atcccccact actcggtacg ttccgatatg 2107680
ttactcaccc gttcgccact cgccacccga gaagcaagct tctctgtgct gccgtccgac 2107740
ttgcatgtgt aaagcatgcc gccagcgttc aatctgagcc aggatcaaac tcttatgttc 2107800
aatctctaac tttttaactt ctggtctgct tcaaagaaac caacaggaca atgttcaaaa 2107860
cattatcttg tctgtctttc aaacagtgtg agactcaagg cactcacact tatcggtaat 2107920
ctgttttgtt aaagagcgtt gcgaattata aagtattcct tccgcctgtc aagatatctc 2107980
tcgatatccc caacattctg tgctatactt ttcagttcgt ccgccacttc tgcagcagcg 2108040
aagaaccgaa ctatacgccc acagggaaaa acggtcaatg cttttctgaa gaaatttttt 2108100
taaaaatatt tatctatttg tttataaatt taatttatat cagtcaattt tattttccat 2108160
acagaattct tccagtgccc gatggatatt ttcagtctgc cattcgtttt ttaagggtgc 2108220
aacaatttcg atttgtcggt tttggtagtc aaattgtatt ttccatgcat acagaaacat 2108280
ggtttcggat tctgttccgc cgtataagct gtcgcccaga atcggactgc ccaaactttt 2108340
catcgccact ctcaattggt gcgttttgcc cgtatgcggt tctaggatga acagccgcag 2108400

ttttcggcg atactgatgc tgtggaatcg ggtaacggcg atattttctg tattgcgcgt 2108460
caacttccac attccacatc tggatttttc cattccgcct ttaatccaac cctgcttttt 2108520
ggacggcttg cggtcggaca gtgccaaata ggtttttttg atgcttttgc cggcaaactg 2108580
tccgctaagg gcggacgcgc tttctttgtt gagggcaaac agtaaaatgc cgctggtctg 2108640
tttgtccaat cggtgcagca gccacacacg ctctacgccc aactgtatgg cgagtgttcg 2108700
ggccagtccg gtctcgccgc tgtcttggtg gacggatatg ccgcccggtt tgttgatggc 2108760
gacgaagtct tgatggcgga acaaaatttc caacatatcc atatatgcct tgcaaaaata 2108820
gaagggttca attttcgtgt tgatgttcgg caaggatttt ttcgtacaca gcttgcggca 2108880
cgtagcggtg gatcgtttcc gtccagcctt ccggcccgac cagtcctttg accatagtgg 2108940
acgacacttc ggcgatttcg cgcggcggca tgaggaatac ggtggatatt tcgggggcga 2109000
ggtcgctgtt gatatggcgc atggaacgtt cgtattcgta atccgaagca gaacggatgc 2109060
cgcgcacgat gaatcctgca tctacctcac gggcgtaatg caccagaaat cggttttcaa 2109120
atacatcggt tctgacgttg ggaaacattt tagtaatatc gcacaacata tcctgccttt 2109180
cagcgacggt ataggtgctg cgtttgtcgg ggttaatgcc gatggcgacg atgagttcgt 2109240
caaacataga ttgcgcctgc cgtatcatcc acagatgccc caatgtgggc ggatcgaaac 2109300
tgccggcata aacggcgcgg cgcggtgtat tcggtaacat ttgattcctc ccggcttcat 2109360
agtcggctgt gtgttggtgc gtgtgcatcc gtattgtatg cccaaagtaa aatgccgtct 2109420
gaagcatttt cagacggcat agtcggacgg cgttttaccg gcatcaatcc tcgccgttta 2109480
aagacaacag gatgttcagc aggctgctga agatgttgta aagcgagata aacagtgtca 2109540
gtgccgcgct gatgtggctg tcttcgccgc cgtcgatgac ggtgcgtacc tgccacataa 2109600
tcattaagga actgaacaag acaaaaccgg cggaaatggt cagggcgagt gcgggaatac 2109660
ccaaaaacag attggcaacc acggcgacca tcagaatgac cgcacctacg gtcaggaagc 2109720
gtccgagcgc gttcatatcg agccgggttc ggcgcgccaa ggcggacatc gttaaaaaga 2109780
cggcggcggt catcgcggcg gcaatgccga cgattttcgc accgtcggca atatggagcg 2109840
cgtattgcag cacggggccg atcaatacgc ccataccgaa tgtgaatacc atcagcaggg 2109900
taacgccggt attgctgtaa cggtttttct cgatgaagtg gatcataccg tagaaaaacg 2109960
ccaacacgac ggcaaaccct atccagcgcg aaccgaaggc ggcgtaaaaa ttgaaaccgg 2110020
cattggcggc aagtgccgcg cctgcggaag ccggaataaa tgaaaatccg agcaggcggt 2110080
aggttttctg caggacggtg tttttagaaa ccgtatgcgc ggtgtagtcg taaacgtcgt 2110140
gttgcatatc atctgctcct gaaagcgcgg ttgggaataa tgggggattt taacattgcc 2110200
caatgtcaaa atttgtccgg ttgcgtgaag ataaagttgt ccggcgtatt ttaaaggccg 2110260
tctgaagcag tttcggacag cctgtgttca aaacggaaaa ccgttattgc ggaacgtatc 2110320
cctgaacggc atccgcgccg tcgccgaaga aatactgctc catctgctga gccaggtatt 2110380
cgcgcgcgcg cggatcggcg aggcttaaac ggttttcgtt aatcagcatc gtttggtggc 2110440
gcgtccacgc cgcccacgct tcttgcgata cgttttcaaa aatgcgcttg cccaattcgt 2110500
tgggaagcgg cggaaatttc atgccttcgg cttctttgtt gagcttgacg cagaatacca 2110560
tgcgtgccat agcggattcc tttgctgtgt tcagaaataa cggggtgatt ttaaccgatt 2110620
agggatacgg acaaaagcct tcttattccc gatgataggg atggttgtgc aggatggaaa 2110680
cggcgcggta gagctgctcg gtcagaaaga cgcgcaccat gccgtgcggc agggtcaggc 2110740
tggacaggcg catcatcatg cgtgcctgct gtttgaggcg gtcggtcatg ccgtccgcgc 2110800
cgccgatgac gaagcagacg tgttcgccgt tttgccgcca gcttttgagg tgttccgcca 2110860
gctcgacgga ggtcggtgct ttgccgcgtt cgtcaagaac gacgaggaac gcgccttgcg 2110920
gaatggcttc aaggatgcgt ttttcttccg ccgccatacc ttgggcggca ttcacgcccg 2110980
cgccgcgttt ttcgggtttg atttctttga gtgcgtaggc gacgtcgcgt ccgaagcgtt 2111040
tggcgtattc ggcgacggcc tcatcaaccc agcgcggcat tttggtgccg actgccaaaa 2111100
cggtgatgtt caatgctttc tcccttacag gaaaatgccg tctgaaggtt cagacggcat 2111160
cgggaatcag tctgccgcgt gccacggctt ctgcattccg gcgtggaaac tcggtttctc 2111220
gccgccccag agggtgtcga tgtcgtagaa gtcgcgcacg gcagggagca tgacgtggac 2111280
gacgaggtct cctgcatcaa ccagcgtcca ttcgccgctg tcgccttcgg tactgaggat 2111340
ttcaaaaccg gcttctttca aatcgacggc aacgttgttg gccagtgctt tgacttggcg 2111400
cgtactgtcg ccgctggcga taatcattct ggcaaacagc gaagttttgt cttgggtttc 2111460
gagaacggaa atgtctttgg ctttgatgtc tccgagggcg ttgacggcga ccccgaccat 2111520
tttttgcagg tcttgcagtt cttgttcgtt cattattttc ctaacgggat gttttcagac 2111580
ggcattatag ccgtttctta ctgatttgac tttatttttc atacaaaccg tgttcgcgga 2111640
tgtagcgtgc ggcggcaggc gggatgccgt ctgaaacgcc ttggccggca aggttgcggc 2111700
ggatttccgt tgacgacaca ttatgcatcg gggcggacaa gatgcggacg ctgccgtcct 2111760
gaagggactt gcccagccac gcgtgcagtt cgcgcggggt ttggtgcagg ctgtcgccct 2111820
gcctcatggc gacggcgata ttggtttcgc gcacgagcat ctgccatttt ttccatgtgt 2111880
gcagcttcat caggctgtcg ctgcccatca gccaccagag ttgcgcggat gggaactgct 2111940
ggcggaagat ttggacggta tcaaaagtat aggttgcacc ttctcggacg atgtcgcaat 2112000
cgctgacggc aaaacgcgcg tcttctgccg tcgccaattc gaccatggca aggcggtcgg 2112060

cggcggaagc ggaggctgcg tctttgtgat acgggccgcc tgtcggcagg aaaacaaccg 2112120
cgtccaagcc gatttcgtcg gcaaaggcac gggcgatatg aagatgtccg ttgtgtatcg 2112180
ggtcgaaagt accgccgaac aatccgattt tcttcatgat gtttccattc cttccgataa 2112240
gtccatgccg tctgaaacac tgccgtccac aaccagcgtc agtttgccgg taacgggatg 2112300
gatgaccagt ccgtgaacgg cgatatggcg cggcatcagc ggatggttac ggataaggtc 2112360
caccgtgtgg cgcacgctgt cttcgacgtt gtcgaaaccg gtcagccagc cgtcgaggtc 2112420
gataccggca taacgcaggg tttcgatacg gtcttcggga atccggcttt cccggacgcg 2112480
cccgaggaat tcttcggcat tcagcccctg cataccgcaa tcgtgatggg cgatgaccat 2112540
aatctctctg accttcagtt caaacacggc aaccaaaagg ctccgcatca ccgaacccca 2112600
cgggtgcgta accagcgcgc cggcattttt aatcagcttg gcatcgccgt tttcaaacc 2112660
caacgcgtcg ggcagcagcc cgataatccg cgcatccata caggacaaaa ctgccagccc 2112720
gcgttcgggg tatttgtcgg taaagtattt ttcatattcg cccgactcga caaactgccg 2112780
gttatgggca aggatgttat ccaactcgct cattttgccg tcctctgaaa aagggttcac 2112840
attataacgt ttccgtctgt tttccgcctt cgccgccgtc caacagcagg aaaataccca 2112900
gcgcgaacgc cgccaacagc aatgtcagca ccatcgcccg cgcgtaatta tcctcacccg 2112960
cgcgtcccaa ataggcataa atcaaagtcg tcagcgtctg ccattccgga cgcgacagaa 2113020
acaatgtcgc cgcaaattcg cccacgcagg ttgccgccgc caaagtcaga ccgcgccgca 2113080
acgccggttt caagaggggg aacgtgatgc ggcatgccgt ctgaaagccg tttgcaccca 2113140
aacccgccgc cgccctgccg taatccggcg gcagtgcatc ccaggctgat aaaacatctt 2113200
ttgccacaaa cggatacgcc agcagcgcat acatcgccag cagcaacggc aacgaagccg 2113260
tccactgcgg ataaagcagc agcacgcccg ccgaaacaca aaccggcgac accataaacg 2113320
gcaaaaacat cagcccgcgc atccacgccg accgccgcgc cgccgccgca tacaccacac 2113380
ccaaaaccgc cgccgcatac accgccgccg ccgagaagcg caaagtattc cacaccgcct 2113440
gccacgtttc actttccatt aacacacgcc acgattcgcc ggccgaccac gctttcacaa 2113500
caattgccaa caaaggaaac aggcagcaca cagacaacac cgccgccgca aacgccagca 2113560
gcacatattc cccgaccgac tgcggcggcg acggcatcac aggggaaacc gccttatccg 2113620
aaaccgcgcg cctgccgaac cacgcataca gcaaccctgc cgccgccgtt acccccaaca 2113680
ccagccacac cagcaccgaa gcaaccgcca tatcgagttc gaacatgacc aactggtaaa 2113740
tttccacttc gaccgtggca taacggctgc cgcccagcag cagcgccagc ccgaacccgg 2113800
aaaaacaata cagaaagaca aggcacacgc cgccggcaag ccacgggcgc aaaacgggca 2113860
tttcaatgtc ccaaaaccgc cgccacgccc ccgcgcccaa cgtccgtgcc gtctgaagcc 2113920
gtgccgcagg cacttgcaca aacccctgat acgccgccct gaccaacaca ggaaggttga 2113980
aaaacacatt gccgtacaac aacagatacg gcgtatcctg cctgccgcgc cacaacagcc 2114040
cgtccgcccc gaacagggcc agcacgccca cgcccgccac caacgtgggc atcacaaaag 2114100
gcagcatcag caggcgcagc accaaagccc gccccggaaa cgccagccgc gccagcaccc 2114160
acgcgacagg cacgcccaaa ggcagcacca gcacacaggt tgccgctgcc tgaaataccg 2114220
tccacgccaa acgtttgagc atataggcat ccgacagcac cgcgcgccac gccaaaccgt 2114280
catacgccgc caccgcccac aaaggcgcaa cgaccattac cgccaaaaaa gccgaaggca 2114340
gcagggcaaa agcaccccat accacccaac gccgtccatc catcgccttc cccacttgaa 2114400
acactgatgt tgcgattgta cccaaaagcc cccacatacc gtatatttca atccgactac 2114460
ataccgtatc cgccttcctc ccgccgtctg aaatatagtg gattaacaaa aatcaggaca 2114520
aggcgacgaa gctgcagaca gtacaaatag tacggaaccg attcactcgg tgcttcagca 2114580
ccttagagaa tcgttctctt tgagctaagg cgaggcaacg ccgtactggt ttttgttaat 2114640
ccactataaa tcgttcaaat aaacaggaat ataacttcag acaaacaact taccgcccga 2114700
tttgtgctat cgttttcgca caacttaaaa aaacctgaca attttgtact tttattacag 2114760
agaaaggctt tacaaatgga cggctggaca cagacgctgt ccgcgcaaac cctgttgggc 2114820
atttcggcgg cggcaatcat cctcattctg attttaatcg tcaaattccg catccacgcg 2114880
ctgctgacac tggtcatcgt cagcctgctg acggctttgg caaccggttt gcccacaggc 2114940
agcattgtca acgacatact ggtcaaaaac ttcggcggca cgctcggcgg cgtggcgctt 2115000
ctggtcggcc tgggcgcgat gctcggacgt ttggtcgaaa catccggcgg cgcacagtcg 2115060
ctggcggacg cgctgatccg gatgttcggc gaaaaacgcg caccgttcgc gctgggcgtt 2115120
gcctcgctga ttttcggctt cccgattttc ttcgatgccg gactaatcgt catgctgccc 2115180
atcgtgttcg ccaccgcacg gcgcatgaaa caggacgtac tgcccttcgc gcttgcctcc 2115240
atcggcgcat tttccgtcat gcacgtcttc ctgccgcccc atccgggccc gattgccgct 2115300
tccgaatttt acggcgcgaa catcggccaa gttttgattt tgggtctgcc gaccgccttc 2115360
atcacatggt atttcagcgg ctatatgctc ggcaaagtgt tggggcgcac catccatgtt 2115420
cccgttcccg aactgctcag cggcggcacg caagacaacg acctgccgaa agaacctgcc 2115480
aaagcaggaa cggtcgtcgc catcatgctg attcccatgc tgctgatttt cctgaatacc 2115540
ggcgtatcgg ccctcatcag cgaaaaactc gtaagtgcgg acgaaacctg ggttcagacg 2115600
gcaaaaataa tcggttcgac accgatcgcc cttctgattt ccgtattggt cgcactgttt 2115660
gtcttgggac gcaaacgcgg cgaaagcggc agcgcgttgg aaaaaaccgt ggacggcgca 2115720

ctcgcccccg tctgttccgt gattctgatt accggcgcgg gcggtatgtt cggcggcgtt 2115780
ttgcgcgctt ccggcatcgg caaggcactc gccgacagca tggcggattt gggcattccc 2115840
gtccttttgg gctgtttcct tgtcgccttg gcactgcgta tcgcgcaagg ttcggcaacc 2115900
gtcgccctga ccaccgccgc cgcgctgatg gctcctgccg ttgccgccgc cggctttacc 2115960
gactggcagc tcgcctgtat cgtattggca acggcggcag gttcggtcgg ttgcagccac 2116020
ttcaacgact ccggcttctg gctggtcggc cgtctcttgg acatggacgt accgaccacg 2116080
ctgaaaacct ggacggtcaa ccaaaccctc atcgcactca tcggctttgc cttgtccgca 2116140
ctgctgttcg ccatcgtctg acagacggaa aggatagtaa atgactacgc attttgtcgt 2116200
tatgggcgta tgcggctgcg gcaagaccac cgccgcgctg tccctgcaga aacacctcgg 2116260
tcaatgtccc tatgccgaag gcgacgagtt ccacacccaa gccaaccgcg acaagatggg 2116320
cgcgggtatt ccgctgaccg atgaagaccg ctatccgtgg ttgggcaatc tgcgcgactg 2116380
gatgacgcaa caggcgcaaa acggtgcgaa ccacaccatc gtaacctgtt ccgccctcaa 2116440
acgcggctac cgcgacattc tgcgcggagc cgaaggcaaa gctgccttca tccacctcag 2116500
tccgccgcaa gacatcaacc tcgagcgcat gatgtcgcgc aaaggacatt acatgaaagc 2116560
agggatgctc gattcgcaac tggaaatcct cgaggaactg ggcgaaggcg aatacggggt 2116620
caaaatcgcc aaccccggca cgcccgaagc ggtcgaagcc gatattctga actgggttgc 2116680
ctcggaaaac ctgctttgaa gcaatatgcc gtctgaagcc cgacacagga tgggtttcag 2116740
acggcataaa catcgggaac agaatggatt acattgattt atagtggatt aacaaaaacc 2116800
agtacagcgt tgcctcgcct tagctcaaag agaacgattc tctaaggtgc tgaagcacca 2116860
agtgaatcgg ttccgtacta tctgtactgt ctgcggcttc gtcgccttgt cctgattttt 2116920
gttaatccac tataaaatgg aaaatacccg gctatcgtct cattttcgtt ttaatcagcc 2116980
ataaaaatgc cgtctgaaac cctttcagac ggcatttctg tcaaacgccg gacgcactca 2117040
acccaaactc aacagcaggt tgcggaacgc gttcgggtct ttgataaacg tcatctcgcc 2117100
cgcctgcgga aaatgaaaat ccaacaggcg cgacacccaa aaacggatgc agccggcacg 2117160
ttgggcggtc gggaaatacg ccttttcttc ggcactcaag gggcgcacgc cctcataacc 2117220
gccgataaac gcctttttca acgcctcatc caacttattg tccgccgtcc ttgcccaatc 2117280
gttgaccgca atcgccaagt catacataaa attgccccgg caggcgtaat agaaatcgat 2117340
gaagcccgat acctgaccgc cgtcaagcaa cacattgtct ttaaacagat cggcatggat 2117400
gatgcccgaa ggcagatgat tgccgagatt gtccttcaac gcatcgattt cggaacacag 2117460
cagtgcggca tcgtcttgcg acaggacggg cagcagccgg gcgcacgcct ccgtccacca 2117520
cgcattgtaa cgcgggtttt ccatttccaa agggaaatcg gcggcggcaa ggtgcatttt 2117580
cgccaacatc gcaccggtat gaaaacactg ctcagccgtc ggcagcgcgg tatccgaacc 2117640
tttcaggcag gcaaccaggc aggcaggctt acccgccaaa acggaatcaa gccggccgtc 2117700
tttgcgcgca accggcgcgg caaccgccac gcccttcata ctcaaatgcc ggttaagctc 2117760
cagaaaaaac ggcagctctt cctgtttcaa cacttcaaac acggtcagca cataacgtcc 2117820
cgaagtcgtc gtcagaaaat aattgctgtt ggtaatcccc tgcgcgatgc cctgcaggga 2117880
aacaaattcc cccaaatcgt aaccgctcag gaagccgcgc atttcatcat cggaaacact 2117940
ggtatagaca gacatatcaa ctcacttgct caaaaacggg caaacccgcc gtcagaacgc 2118000
cggacggcag gcaaacatat aattcacatc ggtcgaatcg cacagggcat aagtttgcga 2118060
caacacatgg taagtcatac ccttcgtatc cgccacatcc aagcctgcct gacggcacat 2118120
tcgcgccagc tcggcaggtg cgatgaattt tttccagtcg tgcgtgcctt tggggacaaa 2118180
cttcaacaga tattccgccg ccacaatcag atgcaggtac gatttcgggt ttttattgat 2118240
ggtggaaaaa aacaccatgc cgtccggttt gaccagattg gcacaagcac gcacgatggc 2118300
ggcgggatcg gggacgtgtt ccatcatttc catgcacgtt accacatcga acgagtgcgg 2118360
ttccgcctcg gcaaggtctt ccacgcggat acattcgtat tcgatatcgg cgacattgtt 2118420
caaagccgcg tgcaggcggg cggtttccaa cgactgctcc gccatgtcga tgccctttac 2118480
aaacgccgcg ccgcgccgcg ccatactttc cgccaagatg ccgccgccgc agcccacgtc 2118540
caaaacccgt ttgccgcaca aatccgcgtg tccgtcgata taatccagcc gcagcggatt 2118600
gatgtcgtgc aaggttttga actcgcccga cttgtcccac catttgtcgg caatccggct 2118660
gaatttggcg atttcccccct catcgacatt atattttttg tcggacattt tccctcccat 2118720
ctgacgaacc gcccactcca aaacccaaga ttatactatg gaacacaatg ccgtctgaaa 2118780
gccttttcgg gcggcgcggc gcaaatacct tacaaatcct tacactttac ggcataatgg 2118840
cggctcgctt tttctggcag aaagacaaaa tatgcccaac aaaacccctt cactgttcgg 2118900
cggcgcgatg attatcgccg gcacggtcat cggcgcaggc atgctcgcca acccgaccgc 2118960
cacatccggc gtatggttta ccggctcgct ggccgtgttg ctgtacacct ggttttctat 2119020
gctttccagc ggcctgatga ttttggaagt caacacccat tatccgcacg gcgcaagttt 2119080
cgacacgatg gtcaaagacc tgctcggacg cggctggaac atcatcaacg gcatcgccgt 2119140
cgccttcgtt ttatacctgc ttacttacgc ttatatcttc gtcggcggcg acctgaccgc 2119200
caaaggctta ggcagcgcgg caggcggcga cgtttcactc accgtcggac aactcgtctt 2119260
cttcggcatc ctcgcctttt gcgtatgggc atccgcacgc ttggtcgacc gcttcaccgg 2119320
cgtccttatc ggcggcatgg tattgacctt tatttgggcg gccggcgggc tgattgccga 2119380

tgccaagccg tccgtcctct tcgataccca agcccccgcc ggcacaaact actggattta 2119440
cgccgccacc gccctgcccg tctgcctcgc ttccttcggc ttccacggca acgtctccag 2119500
cctgctcaaa tactttaaag gcgacgcgcc caaagtggct aaatccatct ggacgggcac 2119560
actgattgcg ctggtaattt acgtcctctg gcaaaccgcc atccaaggca acctgccgcg 2119620
caacgagttc gcccccgtca tcgccgccga agggcaagtc tccgtcctca tcgaaaccct 2119680
gtccaaattc gcccaaaccg gcaatatgga caaaatattg tccctgtttt cctatatggc 2119740
gatcgccacc tcgtttttag gcgtaacgct cggactcttc gactacatcg ccgacatctt 2119800
caaatggaac gacagcatct ccggccgcac caaaaccgcc gcgctgacct tcctgccgcc 2119860
cctgatttcc tgcctgctct tccccaccgg cttcgttacc gccatcggct acgtcggcct 2119920
ggcggcaacc gtctggacag gcatcatccc cgccatgctg ctctaccgtt cgcgcaaaaa 2119980
attcggcgca ggcaaaacct ataaagttta cggcggcttg tggctgatgg tttgggtctt 2120040
ccttttcggc atcgtcaaca tcgccgcaca ggtattgagc caaatggaac tcgtccccgt 2120100
atttaaagga taaaggcaaa atgccgtctg aagcccgccg gcggcttcag acggcattgc 2120160
cgcaacaaac ggcaaccgta ttccggcaca cagcgcatta ccctgcccct cacgcacaaa 2120220
tcccgccccg acaaaccggg acgcaaccat aaaggaacaa tgatgaagct caaaatagac 2120280
attgcaacca acaacttcaa acacggcggc ggcacggaac gctacacatt ggatttggta 2120340
aagggtctga acagacaaaa catcacaccg gccgtttatg cgacgaaatt tgatcacagc 2120400
attcctgaat acgccctaat cgaaccccat cttgtcgatc aacaccggac gctgaaaaaa 2120460
ctacgctcat tcctcttttc aagccggctc gctcaaacca gaaaaaacag tgccgccaaa 2120520
ctgattgcct gccaccacgc cgattacgcc gacctcctca tctgcggcgg cacacacttg 2120580
ggctacctgc accatatggc gcaaaaaccg aacctgctcg accgcctcgc catacgccgc 2120640
aaccgcagca actacgccac cgccaaactg attatggcgc attcccatat gatgcggcgc 2120700
gaactggtcg gactgtacgg cgttccccct gaaagaatcc aagtcgcccc cccccccgc 2120760
agatacggaa cgcttctttc cacaacccgg agaaactgcc gacctgcgcg ccaaatacgg 2120820
ctttgccgac catgaaaccg ttttcctgtt cccatcgacc ggccacacgc gcaaaggtct 2120880
ggaactgctt gccgactttt tcgaacatac cagcctgccc gtcaagctcg ccgttgtcgg 2120940
ctccccgctt ccccgcccta tgaaaaacgt cgtcggactg ggcttctgca ccgatatgcc 2121000
cgaactctac cgcgccgccg actttaccat tatggcttcc ctgtacgaac ccttcgggct 2121060
ggtcggcgtc gaatccgtcc tatgcggcac acgcgtcgtc ctctccgaaa acatggcatg 2121120
tacagaggtc atgaacgaag aagccggctt ctttttctca cgccaaaacc cggaaaccct 2121180
ggcgcaagcc gttgcccaag ccgtcagcct taaaaaacag ggcggacacc gcctgtccga 2121240
cccgatgcgg gcactgaact acaacccggc tttagacaaa cacatcgggc tgattcttga 2121300
aatgcttgcc gcctgaccgc gtccccaaac ggcattgccc cgcaacttcc gcgccgagac 2121360
ttttgcagcg gaaaatacgt ccggcagaaa atccgccgtt gcaggagcag gcaggaaaac 2121420
atcggcaacc gcccccgaaa cgccgtaccc gcgcattgca agcggttgcc ggaacaggcg 2121480
cgttatcgcg cggcacaggc gcatttccac cgatatttca gtataatgcc acccccgacc 2121540
tgccccaatc caaaggaaac gcgatgaaac tcatcattct cgaccgcgac ggcgtcatca 2121600
atcaggaccg cgacgacttc gtcaaatccg ttgacgagtg gatacctgtc gaaggcagca 2121660
tggatgcggt ggcatttctg acgcaggcag gctacaccgt cgccgttgcc accaaccaat 2121720
ccggcatcgg gcgcaaatat tttaccgttc aaaaccttac cgaaatgcac gccaaaatgc 2121780
accgcctcgt ccgtcaggca ggcggcgaaa tcaacggcat ctggttctgc ccgcacaccg 2121840
atgccgacaa ctgcaactgc cgcaagccca aaccgggtat gattgaagac atcatcggac 2121900
gcttcaacgc ccaagcctcg gaaacctggc tggtcggcga cagcctgcgc gatttgcagg 2121960
caatcgatgc cgtcggcggc aaacccgcgc tggttctgac cggaaaaggc aaaaaaacgc 2122020
tctcccaaca cggacacgaa ttgcccgaac acacacaggt tttcgatacc ctgctcgatt 2122080
tctcacaata catcatgcag gaaaacaccg caccgcaagc cgactgaaca taccgcattc 2122140
cgacaaggca aaaccatgct catcatccgc aacctgattt actggctgat actctgttcc 2122200
accctgattt tcctctttcc ctttatgctg ctcgcctcgc ctttccggga cggggcgcac 2122260
aagatggcgc gggtctgggt cggcattctc aactggtcgc tcaaacacat cgtcgggctc 2122320
aaataccgca tcatcggcgc ggaaaacatc cccgaccgcc ccgccgtcat ctgcgccaaa 2122380
caccaaagcg gctgggaaac gctcgccctt caggacattt ttccgccgca ggtttacgtt 2122440
gccaaacgcg agttgttcaa aatccccttt ttcggctggg gcttgaaact ggtcaaaacc 2122500
ataggcatag accgcaacaa ccgccgcgaa gccaacgagc agctcataaa acaggggttg 2122560
gtgcgcaaaa acgaaggcta ttggattacc attttccccg aaggcacgcg ccttgcgccc 2122620
ggaaaacgcg gcaaatacaa actcggcggc gcgcgcatgg cgaaaatgtt tgagatggac 2122680
atcgtccccg tcgccctcaa cagcggcgaa ttttggccga aaaactcctt tctgaaatat 2122740
ccgggggaaa tcaccgtcgt catctgtccg accatcccgc acgcaagcgg cagcgaagcc 2122800
gaattgatgg aaaaatgcga acatctcatc gaaacgcaac aaccgcttat ttccggcgca 2122860
ggcccgtttg ccgccaaaat gccgtctgaa accgcatgac cgcctttgtc cacacccttt 2122920
cagacggcat ggaactgacc gtcgaaatca agcgccgtgc caagaaaaac ctgattatcc 2122980
gccccgccgg cacacatacc gtccgcatca gcgtcccacc ctgcttctcc gtctccgctc 2123040

taaaccgctg gctgtatgaa aacgaagccg tcctgcggca aacactggcg aaaacaccgc 2123100
cgccgcaaac tgccgaaaac cggctgcccg aatccatcct cttccacggc agacagcttg 2123160
ccctcaccgc ccatcaagac acgcaaatcc tgctgatgcc gtctgaaatc cgtgttcccg 2123220
aaggcgcacc cgaaaaacag cttgcgctgc tgcgggactt tttggaacgg caggcgcaca 2123280
gttacctgat tccccgcctc gaacgccacg cccgcaccac acaactgttc cccgcctcct 2123340
cctcgctgac ctctgccaaa accttctggg gcgtgtgccg caaaaccaca ggcatacgct 2123400
tcaactggcg gctggtcggc gcaccggaat acgttgccga ctatgtctgc atacacgaac 2123460
tctgccacct cgcccatccc gaccacagcc ccgccttttg ggaactgacc cgccgcttcg 2123520
ccccctacac gcccaaagcg aaacagtggc tcaaaatcca cggcagggaa cttttcgcct 2123580
taggctgacg cggaccggac cgacccgccg ctttcagacg gcatccgtgc cggaacaggc 2123640
acgcgcccgc ccgattcaaa ccgcgatgac gctttgccgc cggttcgggg caggatggcg 2123700
gcacacacgc cgtctgccgc gtttcatttc acaccgctct tccgaaaccc gaaacccgcc 2123760
cggtccgacg tgcggtatga aacgcttaag ctgacgcgaa gtcttttact gatttgcccg 2123820

cgaaaatgcc gtctgaaagg ttttcggacg gcattttttt tgcgtttccc aggatggcgg 2123880
cggattcgta aaaggcggtc agggtggatt gtaggatggg ttgagacctg ccgaatccgc 2123940
cgcatctgcc aaatctaccg ccgtcattcc tacgaaagtg ggaatctaga acgcggggtt 2124000
aagaaaacct gcatcccgtc attcccacga aagtgggaat ccagtttttt gagtttcagt 2124060
catttccgat aaattgcctt agcattgaat gtctagattc ccgactgcgc gggaatgacg 2124120
aatccatcca tacggaaacc tgcaccgcgt cattcccacg aaagtgggaa tccaggacga 2124180
aaaatctcca gaaaccgttt tatccgataa gtttccgcac tgacagacct agattcccgc 2124240
ctgcgcggga atgacgaatc catccatacg gaaacctgca tcccgtcatt cctacgaacc 2124300
tacattccgt cattcccacg aaagtgggaa tccagaatcc cagactttca gataatcttt 2124360
gaatattgct gttgttctaa ggtctagatt cccgcctgcg cgggaatgac gggatttgag 2124420
gtttctgttc gcgtcattcc cacgaacctg catcccgtca ttcccacgaa agtgggaatc 2124480
tagttttgtc ggtgcggaaa cttatcggat atagtggatt aacaaaaatc aggacaaggc 2124540
gacgaagccg cagacagtac aaatagtacg gaaccgattc acttggtgct tcagcacctg 2124600
agagaatcgt tctctttgag ctaaagcgag gcaacgctgt actggttttt gttaatccac 2124660
tataaaatgg tttctttaga ttttacgtcc tagattcccg cctgcgcggg aatgacgatt 2124720
cgggcactcc tgacagggta aattcacagg atagcgattc gtagcaactg catccccccc 2124780
ccccaacaac tccccaaaca acgccgctcg ccctgggcgt ttgccgtttc cctgcaaaat 2124840
ctgcgataca atgcagtctg aacatttatc cgaatcccaa atccgatgga taccgcacaa 2124900
aaacaacgct gggcaataac cctatcctat gacggcagcc gcttttacgg ctggcagaaa 2124960
caggctgacg gcgtaccgac cgttcaggcg gcattggaaa ccgcgctcgc ccaaatagca 2125020
ggggaagcgg tttccaccac cgttgccggc aggaccgaca ccggcgtgca tgccaccgcc 2125080
caagtcgtcc acttcgacac aactgccgcc cgtccccaac aggcatgggt gcgcggcgta 2125140
aatgcccacc tgcccgaagg cattgccgtt ttgcacgccc gacaggtcgc ccccgaattt 2125200
catgcacgat ttgacgcata cggacggcac taccgctacc tgctcgaatc cgcccccgtc 2125260
cgttcccccc tgctcaaaaa cagggcaggc tggacacacc tcaaactcga catcgggcag 2125320
atgcggcagg ctgccgcctt attggtcggc gaacaagact tctccagctt ccgcgccgcc 2125380
gaatgccaag caaaatcccc cgtcaaaacc atctaccgcg ccgaccttac ccaaagctca 2125440
ggactcgtcc gcctcgattt gcacggcaac gcctttttgc accacatggt acgcaacatc 2125500
atgggcgcgc tcgtttatgt cggcagcggc agactcagcg tcgaaggctt cgccgcactg 2125560
attcaagaac gcagccgcct caaagccccg ccgaccttca tgcccgacgg actttacctg 2125620
accggcgtcg actatcccga ggcatacggc atcatccgcc cccaaatccc cgaatggctt 2125680
taaaacatgc ttgtcgcgga gattttgaaa tcggacaaac tgtcaggcaa tctttttcca 2125740
tgttgacact acctcatcaa ggtactaaca ttgttattac ataaacaggt gaatatggta 2125800
cgtatatgat tctcaacata cgcaaaatgg gaaactcgca aggcgtgatt ctgcccaaat 2125860
cattattggg tcaaataggg gcagtagaca gcttggctgt tacagttgaa aagggcaata 2125920
ttattttaag ctgtcctacc gttcgcaggg gatgggcaga agctgccgca atgcttgtcg 2125980
aaaccgagca ggagcatttt ttttccgaaa ttgaaaacga agcggataaa gaatggatat 2126040
ggtagtacgc ggcggaatct atctggtctc cttagacccg accgtaggaa gcgaaatcaa 2126100
aaagacacgt ccttgtgtcg tagtctctcc tcctgaaata cacaactatc tcaagactgt 2126160
gctgatcgtt cccatgacga gcggaagccg tcctgccccg ttccgcgtca atgtccgctt 2126220
tcaggataaa gacggtttgc ttttgcccga acagattagg gctgtggata aagccggatt 2126280
ggtcaaacat cttggcaatt tagacaacag tacggctgaa aaactgtttg cagtattgca 2126340
ggagatgttt gcctgattga atagtctgaa tggattgtgt tcattatagt ggattaactt 2126400
taaaccagta cggtgttgcc tcgccttagc tcaaagagaa cgattctcta aggtgttgaa 2126460
gcaccaagtg aatcggttcc gtactatttg tactgtctgc ggcttcgtcg ccttatcctg 2126520
atttttgtta atccactata aagaccgtcg ggcatctgca gccgtcattc ccgcgcaggc 2126580
gggaatctag aacgtggaat ctaaagaaac cgttttaccc gataagtttc cgcaccgaca 2126640

gacctagatt cccgcctgcg cgggaatgac gggattttag gtttctaatt ttggttttct 2126700
gtttttgagg gaatgacggg atgtaggttc gtaagaatga cgggatatag gtttccgtgc 2126760
ggatggattc gtcattcccg cgcaggcggg aatctagaac gtggaatcta agaaaccgtt 2126820
ttatccgata agtttccgtg cggacaagtt tggattcccg cctgcgcggg aatgacggga 2126880
ttttaggttt ctaattttgg ttttctgttt ttgagggaat gacgggatgt aggttcgtag 2126940
gaatgacggg atataggttt ccgtgcggat ggattcgtca ttcccgcgca ggcgggaatc 2127000
tagaccttag aacaacagca atattcaaag attatctgaa agtccgagat tctagattcc 2127060
cgcctgagcg ggaatgacga aaagtggcgg gaatgacggt tagcgttgcc tcgccttagc 2127120
tcaaagagaa cgattctcta aggtgttgaa gcaccaagtg aatcggttcc gtactatttg 2127180
tactgtctgc ggcttcgtcg ccttgtcctg atttttgtta atccactcta aagaccgtcg 2127240
ggcatctgca gccgtcattc ccgcgcaggc gggaatccag accttaaggc agcggcaata 2127300
ttcaaagatt atctgaaagt ccgagattct agattcccgc ctgagcggga atgacgaaaa 2127360
gtggcgggaa tgacggttag cgttgcctcg ccttagctca aagagaacga ttctctaagg 2127420
tgctgaagca ccaagtgaat cggttccgta ctatttgtac tgtctgcggc ttcgtcgcct 2127480
tgtcctgatt tttgttaatc cactatctcc tgccgcaggg gcgggttttg catccgcccg 2127540
ttccgaaaga aaccacgtgc gcgttttttg ccgtctttat aacccccggt ttgcaatgcc 2127600
ctccaatacc ctcccgagta agtgttgtaa aaatgcaaat cttaaaaaat ttaaataacc 2127660
atatgttata aaacaaaaaa tacccataat atctctatcc gcccttcaaa atacacatcg 2127720
aattccacac aaaaacaggc agaagtttgt tttttcagac aggaacatct atagtttcag 2127780
acatggaatc gccgaaaacg tcggcggtaa atgcaaagct aagcggcttg gaaagcccgg 2127840
ccggcttaaa tttcttaacc aaaaaaggaa tacagcaatg aaaaaatccc tgattgccct 2127900
gactttggca gcccttcctg ttgcagcaat ggctgacgtt accctgtacg gcaccatcaa 2127960
agccggcgta gaaacttccc gctctgtatt tcaccagaac ggccaagtta ctgaagttac 2128020
aaccgctacc ggcatcgttg atttgggttc gaaaatcggc ttcaaaggcc aagaagacct 2128080
cggtaacggc ctgaaagcca tttggcaggt tgagcaaaaa gcatctatcg ccggtactga 2128140
ctccggttgg ggcaaccgcc aatccttcat cggcttgaaa ggcggcttcg gtaaattgcg 2128200
cgtcggtcgt ttgaacagcg tcctgaaaga caccggcgac atcaatcctt gggatagcaa 2128260
aagcgactat ttgggtgtaa acaaaattgc cgaacccgag gcacgcctca tttccgtacg 2128320
ctacgattct cccgaatttg ccggcctcag cggcagcgta caatacgcgc ttaacgacaa 2128380
tgcaggcaga cataacagcg aatcttacca cgccggcttc aactacaaaa acggtggctt 2128440
cttcgtgcaa tatggcggtg cctataaaag acatcatcaa gtgcaagagg gcttgaatat 2128500
tgagaaatac cagattcacc gtttggtcag cggttacgac aatgatgccc tgtacgcttc 2128560
cgtagccgta cagcaacaag acgcgaaact gactgatgct tccaattcgc acaactctca 2128620
aaccgaagtt gccgctacct tggcataccg cttcggcaac gtaacgcccc gagtttctta 2128680
cgcccacggc ttcaaaggtt tggttgatga tgcagacata ggcaacgaat acgaccaagt 2128740
ggttgtcggt gcggaatacg acttctccaa acgcacttct gccttggttt ctgccggttg 2128800
gttgcaagaa ggcaaaggcg aaaacaaatt cgtagcgact gccggcggtg tcggtctgcg 2128860
ccacaaattc taatctgcaa agattggtat caacaaaaag cctgtcgcca gacaggcttt 2128920
tttctgtttg gctttttcct gttttctgtt tggctttttc ctgttttctg tttcgctgtt 2128980
ttctgtttcg ctgttttctg tttcgctgtt ttctgtttcg ctgttttctg tttcgctgtt 2129040
ttctgtttcg ctgttttctg tttggctttt ttctgtttgg ctttttctg tttggctttt 2129100
tcctgttttt agtctttttt attcaatgtc aaaatatgcc gtcattcccg cgcaggcggg 2129160
aatctagtgc gttgagtttc agctatttag aataaatttt gaaactttaa tcccgtcatt 2129220
cccacgaaag tgggaatcca ggacgcaaaa tctcaagaaa ccgttttacc cgataagttt 2129280
ccgcaccgac agacctagat tcccgcctgc gcgggaatga cgggatttga ggttgcggca 2129340
tttatcggga gcaacagaat ccgctctgcc gtcattccca cgaaagtggg aatctagttc 2129400
gttcggtttc gcttgtttta agtttcgggt aacttccact tcgtcattcc cgcgcaggcg 2129460
ggaatccagt gcgttgagtt tcagctattt agaataaatt ttgaaacttt aatcccgtca 2129520
ttcccacgaa agtgggaatc tagttttttg agtttcagtc attcccgata aattgcctta 2129580
gcattgaatg tctagattcc cgcctgcgcg ggaatgacgg cggaaagatt ctatttttcc 2129640
cgataatcgc ccacaatctc aaattccttc attctctcaa aaacaaaatc agaatcctaa 2129700
atcccatcat ccccatctat gtgaatataa aaattttaaa aattatagtg gattaacaaa 2129760
aaccagtacg gcgttgcctc gccttagctc aaagagaacg attctctaag gtgctgaagc 2129820
accaagtgaa tcggttccgt actatctgta ctgtctgcgg cttcgtcgcc ttgtcctgat 2129880
ttttgttaat ccactatatt ttcacaagcg aaagaatgcc gtctgaagcc ttttttccgg 2129940
ttttcagacg gcattttttg cttgacgttt aactgtaaat cttcgcgcct tttttgacga 2130000
actcgaccgc tttttcctcc atgccctgcc gttgggcttt ttgcttgtcg gcgtagtcgc 2130060
gcacttcctg cgtgattttc atcgagcaga atttggggcc gcacatcgag cagaagtggg 2130120
cgattttcgc gccttcggca ggcagggttt cgtcgtggaa gctctcggca cgttcagggt 2130180
cgaggcttaa gcgaaattgg tcgcgccagc ggaactcgaa acgcgctttg ctcagggcgt 2130240
tgtcacgtaa ttgtgcgccc ggccagcctt tggcgagatc ggcggcgtgg gcggcgagtt 2130300

tgtaggtgat gatgccggtg cgcacgtctt ctttgtcggg cagccccaaa tgctctttcg 2130360
gggtaacgta acaaagcatc gccgtgccgt accagccgat attggccgcg cctatgcccg 2130420
aggtgatgtg gtcgtagccg ggtgcgatgt cggtaacgag cgggccgagc gtgtaaaaag 2130480
gtgcttcaaa gcagtgttgc agctcttcgg tcatgttttc tttgacgcgt tgcagcggca 2130540
catggccggg gccttcgatc atgacttgta cgtcatgttt ccacgcttta tcggtcaatt 2130600
cgcccaaggt gtgcagttcg gcgaattggg attcgtcgtt ggcatcggca atgcagccgg 2130660
ggcgcaggcc gtcgccgagg ctgaacgata cgtcatacgc tttcataatt tcgcagattt 2130720
cgtcgaaatg cgtgtagagg aaattttccc gatgatgtgc caaacaccat ttcgccataa 2130780
tcgaaccgcc gcgcgagacg atgccggtga ggcggttggc ggtcatcggc acataacgca 2130840
gcaacacgcc cgcgtgtatg gtgaaatagt ccacgccttg ctccgcctgt tcgatgaggg 2130900
tgtcgcggaa caaatcccaa gtcaaatctt cggcgatgcc gccggttttt tccaacgctt 2130960
ggtaaatcgg cacggtgccg atggggacgg gcgcgttgcg gataatccat tcgcgcgttt 2131020
catggatgtg cgcgccggtg gacaaatcca taatcgtgtc cgcgccccaa cgcagcgacc 2131080
acaccatttt ttcgacttct tcggtcaggc tggaggtgac ggcggagttg cccaagttgc 2131140
cgttgatttt gacacgaaag ttgcggccga taatcatcgg ttcgagttcg gggtggttga 2131200
tgttggcggg aataatcgcg cgtccggcgg cgatttcttg gcgcacgaat tcggcgtga 2131260
tttggtcggg atgggtcggg atgttcgcac cgaaactttg ccccgcgtgc tgttccaaga 2131320
gtttggcgta ttccggtttt tgggacaatt cgtctaattt taaacgttcg cgtatggcga 2131380
caaactccat ttcgggcgtg ataatgcctt ggcgcgcgta gtgaagctgg gttacgttgc 2131440
tgccgctttt cgcgcggcgc gggcgggtga tttggttgaa acgcagatgg gcggttttcg 2131500
gatcgtgtgc gcgttcgatg ccgtattcgc tggagagctt gggcaggatt tcggtatcgc 2131560
cgcgttcgtc cagccacgcg gtgcggatgt gcggcagacc ttgtttcagg tcgatatgcg 2131620
ccgccgggtc gccgtacacg ccgctggtgt cgtagacggg aatcggcgga ttggcttccg 2131680
taccttgcgc cgtgtaggtg tcgtcctgac ggatttcgcg caaaggcacg cggatgtcgt 2131740
cgcggctgcc ttgcagatac acgcgttccg agttcggata tttaaagcag atgccgatgt 2131800
cttcgctcaa gtcggcaagc tcgcgcgctt cgttgccgga agttttggcg gttttttttg 2131860
gcgtagtcat aaaaaaatgc tcctgttttc tcgtttagaa ttaaagaaac aggagcgttt 2131920
tgcgttttca gacggcattt gaaaaccaat gccgtctgaa aagcagaatc cgtgaaaact 2131980
ccccacgcag gtattatccc gatcgggtgt aaagggtatt tctcagccgc ctaaacatca 2132040
ggcagcaccc ctgtttcaat gttaaccaaa attaaatcac gaacatgaac ttttgtaaag 2132100
aaaataatat ttcaaatcag gcataaaccg ccggacggca aaattttatg atttttcgcg 2132160
gaagtaatgt ttgacaacat aaaaaatctg ccgtatagtt tcatcttctg acgcgggatg 2132220
gagcagcatg gtagctcgtc gggctcataa cccgaaggtc gtaggttcga atcctgctcc 2132280
cgcaaccaaa tatcaaaccc ctcggttcaa tgccgagggg ttttgttttg cctgtttcct 2132340
gtttcctgtt tcctgccgcc tccgtttttt gccggatttt ccttccggcc gcaatatcgg 2132400
aacggcagac cgccgtctgt ttgcggttgc aaattcaggc agtttggcta caatcttccg 2132460
cattgtcttc aagaaagcca accatgccga ccgtccgttt taccgaatcc gtcagcaaac 2132520
aagaccttga tgctctgttc gagtgggcaa aagcaagtta cggtgcagaa agttgctgga 2132580
aaacgctgta tctgaacggt ctgcctttgg gcaacctgtc gccggaatgg gtggaacgcg 2132640
tcaaaaaaga ctgggaggca ggctgctcgg agtcttcaga cggcattttt ctgaatgcgg 2132700
acggctggcc tgatatgggc ggacgcttac agcacctcgc cctcggttgg cactgtgcgg 2132760
ggctgttgga cggctggcgc aacgagtgtt tcgacctgac cgacggcggc ggcaacccct 2132820
tgttcacgct cgaacgcgcc gctttccgtc ctttcggact gctcagccgc gccgtccatc 2132880
tcaacggtct gaccgaatcg gacggccgat ggcatttctg gataggcagg cgcagtccgc 2132940
acaaagcagt cgatcccaac aaactcgaca atactgccgc cggcggtgtt tccggcggcg 2133000
aaatgccgtc tgaagccgtg tgtcgcgaaa gcagcgaaga agccggtttg gataaaacgc 2133060
tgcttccgct catccgcccg gtatcgcagc tgcacagcct gcgctccgtc agccggggtg 2133120
tacacaatga aatcctgtat gtattcgatg ccgtcctgcc cgaaaccttc ctgcctgaaa 2133180
atcaggatgg cgaagtggcg ggttttgaga aaatggacat cggcggtctg ttggatgcca 2133240
tgttgtcggg aaacatgatg cacgacgcgc aactggttac gctggacgcg ttttgccgtt 2133300
acggtctgat tgatgccgcc catccgctgt ccgagtggct ggacggcata cgtttatagg 2133360
atgcgccatg cttgaactga acggactctg caaacgcttc ggcaataaaa ccgtcgccga 2133420
caacatctgc ctgactgtcg ggcgcggcaa aatactcgcc gttttggggc ggtcgggctg 2133480
cggaaaatcc accctgctga atataattgc ggggattgtc cggccggacg gcggggaaat 2133540
atggctgaac ggagaaaaca ttacccgtat gccgcccgaa aaacgccgta tctcgctgat 2133600
gtttcaagat tacgcgctgt ttccccatat gagtgcgctg gaaaatgcgg cattcggttt 2133660
gaaaatgcaa aaaatgccga aagccgaagc cgaacgcctc gccatggcgg cacttgccga 2133720
agtcggactg gaaaacgagg cgcaccgcaa gcctgaaaaa ctttccggag gcgagaagca 2133780
acggctggcg ttggcgcgcg ctttggttgt ccgcccttcc ctgctgctgt tggacgaatc 2133840
gttttccagt ttggacacgc atttgcgcgg cacgctgcgc cgtatgactg ccgaacgtat 2133900
ccgaaacggc ggcatccctg ccgttttggt aacgcattcg cccgaagaag cctgtacgac 2133960

ggcagacgaa atcgccgtga tgcataaagg gaggattcta caatacggta cgcccgaaac 2134020
attggtcaaa acaccatcct gcgtgcaggt cgcccgactg atgggtttgc ccaataccga 2134080
cgataaccgc catattccgc aacatgcggt gcgtttcgac caagacggca tggagtgccg 2134140
cgtattatcc cgtacctgtt tgcccgaatc gttcagcctg tccgtcctcc atccggaaca 2134200
cggcatcctg tggctgaacc tcgatatgcg gcacgccggg gcggtatcgg gcaaggatac 2134260
ggtacgcatc catatcgaag aacgggaaat cgtccgcttc cgctgatgct tcttaaaaac 2134320
aaaatgccgt ctgaaaacct ttcagacggc attttttac caaagcagcc atattttttt 2134380
atcagggctg caaaatttta tccgaaacaa caacaatctt ttcatcgtca ttcccgcgca 2134440
ggcgggaatc tagaacgtaa aatctaaaga aaccgttttt cccgataagt ttccgtgccg 2134500
acagacctag attcccgcct gcgcgggaat gacggatttt aggtttctga ttttggtttt 2134560
ctgtttttga gggaatgacg agacttgaga tggcggcatt tatcgggagc aactgaaacc 2134620
accctgccgt cattcccgca aaagcgggaa tctagaaccc aacacggcaa aaatttatcc 2134680
gaagcgacaa caatcttttc atcgtcattc ccgcgcaggc gggaatccag aacgtaaaat 2134740
ctaaagaaac cgtttttccc gacaagtttc tgtgccgaca gacctagatt cccgcctgcg 2134800
cgggaatgac gggattttag gtttctgatt ttggttttct gtttttgagg gaatgacgag 2134860
acttgagatg gcggcattta tcgggagcaa ctgaaaccac cctgccgtca ttcccgcaaa 2134920
agcgggaatc tagaacccaa cgcggcaaaa atttatccga agcgacaaca atctgagacc 2134980
tttgcaaaat tcctttccct cacaacagcc gaaacccaaa cacaggtttt cgtctatttt 2135040
cgccccaaat acctcctaat tctacccaaa taccccctta atcctccccg gatacccgat 2135100
aatcaggcat ccggtcgcct tttaggcggc agcgggcgca cttagcctgt tggcggcttt 2135160
caacaggttc aaacacatcg ccttcagatg gctttgcgca ctcactttaa tcagtccgaa 2135220
ataggctgcc cggcgtagc ggaatttacg gtgcagcgta ccgaagctct gttcgaccac 2135280
ataacgggtc ttcgacaaat atcggttgcg tttggtttgc gcctccgtca gcggacggtt 2135340
gcggcaggct ttgcgcataa tatagtggat taaatttaaa ccagtacggc gttgcctcgc 2135400
cttgccgtac tatctgtact gtctgcggct tcgtcgcctt gtcctgattt aaatttaatc 2135460
tactataatg tgcagtttct cgatatagcc ttccgcatcg gtgcgggtat gttgtttgta 2135520
accgagtttg tagaggccgt ttttcttgat ccaacgcgca tcgctgtcct tactccgtgt 2135580
ggtttggccg ctgacttgtc cttcttcatc gacttctatg gcctgacgct gtttgccgtc 2135640
ggcggtctga ataatggtgg cgtcaatgac ggcggcggat gctttctcta cttttaaacc 2135700
tttttcggtc agttggcggt tgatcagttt gagcaattcg gacagggtgt cgtcttgcgc 2135760
cagccagttg cggtagcggc ataaggtgct gtaatcgggg atgctcagtt cgtcgaaacg 2135820
gcaaaacagg ttgaagtcga tgcgggtaat gaggctgtgt tcgagttcgg gatcggagag 2135880
gctgtgccat tgtccgagca ggacggcttt gaacatggac agcagcggat aggcgggacg 2135940
gccgcggtgg tctcgaaggt aacgggtttt ttgacggttc aggtattgtt cgatcggctg 2136000
ccaatcaatc acttgatcca acttcaatag cgggaagcgg ttgatgtgtt tggcaatcat 2136060
ggcttgtgcg gtttgctgga agaaggtgct catggaaaat ctcctaaatg tcttggtggg 2136120
aatttagggg attttgcaaa gttttcaaca agtttccgca ccgacaaacc tagattcccg 2136180
cctgcgcggg aatgacggga ttttaggttt ctgatttcgg ttttctgttt taagggaatg 2136240
acgagacttg agatggcggc atttatcggg agcaacagaa accactctgc cgtcattccc 2136300
gcgaaagcgg gaatctagaa cccaacgcga caaaaattta tccgaagcga caacaatctt 2136360
ttcatcgtca ttcccgcgca ggcgggaatc tagaacgtaa aatctaaaga aaccgttttt 2136420
cccgacaagt ttctgtgccg acagacctag attcccgcct gcgcgggaat gacgggattt 2136480
taggtttctg atttcggttt tctgttttaa gggaatgacg agacttgaga tggcggcatt 2136540
tatcgagagc aactgaaacc actctgccgt cattcccgcg aaagcgggaa tctagaaccc 2136600
aacacggcaa aaatttatcc gaagcgacaa caatcttttc atcgtcattc ccgcgcaggc 2136660
gggaatctag aacgtaaaat ctaaagaaac cgtttttccc gataagtttc cgtgccgaca 2136720
aacctagatt cccgcctgcg cgggaatgac ggattttagg tttctgattt tggttttctg 2136780
tttttgaggg aatggccgat tttgggtttc tgtttcggtt ttctattttg caagaatggc 2136840
aaaatttcag attgcgggca ttgttaagta tttctatttt ttacctgccg tatttatttc 2136900
cgccccttga agtcggcttc ttcctcgaca gacacgctgt tcatctgttt gatcagcttt 2136960
tccgacttct cttcgtcttc gcagcggatg actttcacaa tatcactttc gagctgtccg 2137020
acattgctgt gcagaatgat gtttttgacg ggcaggatgt tgttggggtt catggaaaaa 2137080
cggcgcagcc ccatacccaa taaaacgcgg gtaaacgcgg tatcgcccgc catctcgccg 2137140
catacggata cgtctttgtc catgcggttg gcggtacgga tgacgtgttg cagcattttc 2137200
agcacggcgg gatggccggg ctggtagagg tggctgacgc tgtcgtcgcc gcgatcgacg 2137260
gacaagatgt attgaatcag gtcgttggta ccgacggaga tgaaatcgac cagtttcaaa 2137320
atactgccga cggtcagcgc ggcagacgga atttcaatca tacagccgat gccgacttta 2137380
ccgaaggcat cgccgcgttc ggcaagctgg cgttgcgcgg tgtcgaggtg gatgaggcac 2137440
tggcgcactt cggatacgga ggtaatcatc ggccacatca tccgcacggg gccgtgtacc 2137500
gccgcacgga ggatggcgcg catctgggtg cggaacatga ccggttcggc aaggcacagg 2137560
cggatgccgg tcatgcccag cgcggggttg aggctgccgt tgggcgtgct gtttttcccg 2137620

aaccagcgcg ggtttttgtc cacacctaaa tcgactgtcc gtatcgttac gcttttgcct 2137680
ttcatttttt tgacaatcgc gctgtacact tcgtactgct cgtcttcaga cggcatcgta 2137740
tcgcggttca ggtaaagaaa ctcgctgcgg aacagcccga tgccgtctgc gccgaggttg 2137800
tgcagcggtt tcacgtcttc ggcggattct atattgccca caagctcgat gcagaccccg 2137860
tcggcggtgg cggcggcggt ttttttgagc ttgttcaaat cgcgtttgtg gctgcggtat 2137920
tcgcgggcac ggcggcggta ttcgttcaac accgactcat ccggcgcgat aatcaacacg 2137980
ccgttgatac cgtccacaat gaccgtttcg ccttcggtaa tcagtttgcg cgcgttgtgc 2138040
agcccgacga cggacgggat gtccaagctc ctgcccaaaa tcgccgtatg cccggtgggg 2138100
ccgccggcat cggtaacgaa ggcggcaatg cgctgctctt taaacaaaac cgtgtcggcg 2138160
ggcgaaaggt cgtttgcaat cagaacggtt tcgtcaaaca ggttgtcggc aacttccaac 2138220
tcgttgccct gcccgatcag gttgttgtgg atgcggcgga cgacttgcag catatcctgc 2138280
ttgcgttcgc gcaaataggc atcgtccata ttgtcgaatt gggcggcgag tttgtcgctc 2138340
tgctgcttca atgcccactc ggcgttgatt ttttgttccc ttaaaatatc gacgggttcg 2138400
cgcgacaagg taacatcggt caagagcatc aggtgtagcg agatgaacgc gcccaactcg 2138460
gtcggggcgt tttcgggaat cgcgctgcgg agctgttcca actctttgcg cgtggctttg 2138520
acggcggcat cgaaacgttc ggcttcggca tcggtgtccg cctccgcaac atcatactgc 2138580
ggcacttcct ccgtaccgcg cgcaatcagg tgggcgcaac cgacggcaat gcctttgccc 2138640
gccgccacgc cgtgcagcac gatactcatt attcgccctc gccgaagtag ccgttgatta 2138700
agtcggtcag ggcgcgcatc gcttccgcct cgtccgcgcc gtccgtctcc agttcgatga 2138760
ccgtaccctt ggcggcggcg agcatcatca gccccataat gcttttgccg ttgacgcggc 2138820
tgtcgttttt cgtaacccag acttcgcttt tgaattggga cgcggtttgg gtgaacttgt 2138880
tggacgcgcg ggcgtggagt ccgagtttgt tgatgatttc gatggattgt ttgagcattt 2138940
cgattcccgt gttatgtata tcggcagcag acgccgttta aaatgttttc ctgccctgcc 2139000
gcttcttcag acggcatcgc cgctgcgccg gcacaccaaa tcttcgggcg cggacgtgat 2139060
ggcgaaaatg ccttttaccg ccgcctccct gacgcattcg gtaaaggcgg caaggtcttc 2139120
cgccgccggc gaatattgga cggccttaac catcatcggc gcgttcagcc cggtcaaaat 2139180
cgccgatttg ttttcgcgca cgaggcggcg ggcggcattg caggggggtcg caccgaaaat 2139240
atcggtcata atcagcacgc cgtcgttgtc gggaaattcc tgaagcgcgg caatggcgtt 2139300
gttgttgatg tcgtcttggt cttccgtcgg ctgcacgccg agtatgcgga cgttttcagg 2139360
cagtccgccc ggaaaaaaat gatgcgccag cttgcggtag gcttcgccta tggtttcgtg 2139420
tgtgatgatt aaaagcccta tcatattatg cgtcctgttc ctcattatcc tgccggcgta 2139480
tgggcgcgat gccgtctgaa cagccttcag acggcatcgc gcccttattt tccgcccaat 2139540
gcgtaaatct cgcccagatt gcgccagcag cccgccgcat ccatgccgta accgaaaaca 2139600
taacggttcg gcacatccag tccgacataa tcggctcgga taggcttggg tttgtcaatc 2139660
agcttgttgg cgaacaccgc cgcacggcag cttgccgcac ccatttccaa aagtttggct 2139720
tgaatggcgg acatcgtatg cccttcgtcc aaaatatcgt ccagcacgac gacgtgcctg 2139780
ccccggattt gttccgcatc gggcatacgc ttccagttga acgcgccgcc ctccagcttg 2139840
tcgccgtaac gggaaacgtg aacataatca aaatctaagg gaaaacgcaa cagcggcagc 2139900
aactgccccg taaacaccac cgcgccgccc atcacgggca gcagcagcgg atatttgccg 2139960
cccaaatcac gcgtaatctc gtccgccact ttttgcagtg cggcacggca ttggccttgg 2140020
tcgaacaaaa gatcggcgtt ttcaagcatc gcctgtgttt caaggcgttt ggtttctaaa 2140080
tcggtcatat gtcggaatcg gtcggtaaaa ggaaaattat aaaccaaagt atcggatgcc 2140140
gtctgaactg tcctgctcag cggtcggtac gcacgcgcac aaatgtggca aatttcggcg 2140200
tgcctttccg cgtaaagcca cggtaacggt aggtaatcag tgtgccgatt ttgggcgggt 2140260
tgtcgcggtc tttatctttg aaaccgctgc cgatgcggaa ttcgccgtgc cggtttttgc 2140320
agccgaccgc gcccagccgt ccggcgtttc gccctttgcc ctcatagtgc cgcgttaccg 2140380
tgcattcgtc gtcgtattgg cttttcagct tcaataattg gctgctcctg ccgccgctgt 2140440
aacgggattc gggctgacgc agcatcacgc cttcgccgcc ctgcgcttcg atttgtttta 2140500
aaaagtccat cgcgtgctgc cggtcgcgca ctttgatttg cgggatgatg gtaatcggcg 2140560
cgttcggatg cgttttcagc cactgcgttg cgactgccaa acgttggtag aggttgccct 2140620
gcgccttggg tacatcgaaa acgtgcaggc ggatgccgcg ccagtctgaa gaaacagaac 2140680
gcacggtagc ggaaatctgc tcgaactgac cacgtccgct atacaattcg ccgtccaaag 2140740
gataaggcgg aaactgagcg gtaaaacctt tgggcggagc aaacgcgtag ccctgacggc 2140800
tcatcaggtg ctttccgtcc caataggcgc gcacgccgtc gagtttctcg ctcatcgccc 2140860
agccggcaat atcctgccct ttgtattcct gcgccagcat caaatccgcc gcgcctgctg 2140920
atgcagggat gaaaaccgcc gtaaaaatcg gtatgatgcc gccgattgtc ttcttaatca 2140980
tctgattccc ccaatatcaa aacgggcggc aaaccgccat aaaacaaacg gcaaacccga 2141040
tgccgtctga aaaaccgttt aggaacacgc cgatgaccct acgttacgaa atcttccccg 2141100
ttacccccctt ccgccaaaac tgcaccctga tttgggacga cgaaagcggc gaagccgtcc 2141160
tgaccgatgt cggcggcgac gtgccgttcc tgctgcaagc gttggcaaac cgcaaactta 2141220
cgctcacggc aatctggctg acgcacggcc atctcgatca cgcgggcggc gtggtcgaaa 2141280

tgttgaaaac gcataaagtc cctgtcctcg ggccgcatcc ggacgatgaa ttcctgctcc 2141340
aatcgctgcc gcaaaccacc gcgcaatacg gatttcccgt ctcgcccgcc tttgcgccga 2141400
accgttggct cgaagaaggc gaaacgctca cggtcggacg ctatgccttt caagtgctgc 2141460
atattccggg ccatacgccg ggacatatcg tcttttattg tgccgaggcg gaattgctga 2141520
ttgcgggcga cgtgctgttt tacgaaacca taggcagaac cgattttccg cgcggcaacc 2141580
acgccgactt aatcaataat atccgcaaca aattattcac ccttcccgaa accgtgcaag 2141640
ttgtcgccgg acacgggcgt atgacttcca tcggacacga aaagcggcac aatccgtttt 2141700
tctaaccgcc ttccctacgg tcttcagacg gcatcatctg cactgatgcc gtctgaaaca 2141760
caaaaggctc agacaaccgc cgccttgccg gtgtacctcg ccggacaagg ctttggtaag 2141820
tacttcaaac aaacccaaaa tcagaaccag tacagttacg ccgcgctttc ggcattcccg 2141880
ccccggctga aacaatattt ttccgcacaa gtcagactgc ttcatcttct gccgcgtatt 2141940
ccaaagattc cgacaacgcc gttgtttcat tggaacgctc gaccaaatcc cgttgaagtt 2142000
gtttgcctgc cttcacgccc aacagacgca gtttctcggc gcgtccgacc agattcccgc 2142060
gcccttcggc aagttgcttg aatgccgtct gaaaactgct ttgcgcctga tcgatgcctt 2142120
tgccgacgct ttcgagcgtc tgtacgaagc cgacaaactt gtcgtacagc ttgccgcctt 2142180
cgtccgcaat cgccagtgcg ttctgatttt gctgttcgtt gcgccaaata ttcgccaccg 2142240
tcctcaaagt cgccagcagc gtactggggc cgaccagcat aatccgtttg tcgaaacact 2142300
cttggaacaa gcccgcgtca ttctgcaacg ccaacaggta ggccggttcg acagggataa 2142360
acataaagac gaaatccaat gtgttcacac cttccaaatc ggtgtaatcc ttcagcgaca 2142420
agcctttcat gtgtgcacgg atgctggcaa cgtgtgccgc cagttcgcgt gccgccgtat 2142480
ccgcatccgc cgcctgcgtg tagcgcacat aagctgtcag cgagaccttg gaatcaatca 2142540
caatctgctt gttgtcgggc aggttgacca aaacgtcggg ctggaggcgg cgcgtgccgc 2142600
cgtcttcctc ttttcggacg gatgccgcct gaaccacata ttcccgccct ttctgaaggc 2142660
cggaattttc caaaaccgtt tccagaatca tctcgcccca attgccctga accttattct 2142720
gcgtaccggt cagcgcgttg gtcagggcct ttgcctcgct gtgcagctgc gcgttcaacc 2142780
cctgaagccg tttcaattcg ttttccaacg tcagccgctc gcgcgattct ttatcatagg 2142840
tttgcttgac caactcgccg aaaccgtgga tgcgttcgtt tagcgggttc aaaacctgat 2142900
ggagctgctc gcggttctgc tcggtaaaac ggcggctttt ttcttccaaa atcgtgttgg 2142960
caagattttg aaactgatcg ctcaaacttt tgcgcgcctc gcccagcaag gacagcttct 2143020
cttcagaagc aaggcgttcc tgttcgattt gcgttgccaa acgttcgttt tcaaccgcca 2143080
aaccctgtgc cttttcctgc aactcggtat gcgactgcct caaccgctcc gcttccgcct 2143140
ctttttcctg caaatgggca atctgttttt cggctgcggc aaaacggttg ccgacatcgg 2143200
aaaggtcgtt ttgcacgtcg cggacagttt ggcggctttc ttccaaatcg gtttcgattc 2143260
tttggcggat ttggcgttcc aaggcatatt ggtcggcaaa cgccttccgt tcctgtccga 2143320
gctgcgttgc caaacgttcg ttttcaaccg ccaaaccctg tgccttttcc tgcaactcga 2143380
tatacgactg cttcagccgc gccgactccg cctctttttc ctgcaaatgg gcaatctgct 2143440
tttcggctgc ggaaaaacgg ttgcccaaag cataattttc gtcctgcaaa tgccggtatt 2143500
tcccgtccaa caccgccaat tccgacacgg ttttgccgtg tgcctgttcg acaaaatcac 2143560
atcttgccgc cttttccgcc aggtgcgcgt tcaaaccggc aaactcgccc tgaaaccggc 2143620
ccttcatcag caaccatgta aacaacacgc ccgacaccaa cgccgccaaa ggcagcaaaa 2143680
cagtcatcag ttccatcaat tatcctaata ttcaaacatt ttcacaccgg acaaaaccgc 2143740
cgcttattcc gattctacct gtttgttcga catagctcca aaaaatatag cggattggct 2143800
ttaaacctgt tcgacatcgc cttaccatgc tgcttgcggt ttcagacctt ttcctaattc 2143860
aatatcaatc tgccacaaac cctgattaag ttcccgatgt ctgacatttt tagaatgatg 2143920
ccgtctgaaa tgttgcagct atgttcagac ggcatacgga ttcaggcttt tcaaacggca 2143980
ggcaaaatga aaaaagggca aaccctaaag gatttgccct tttgttccaa acgcttagtg 2144040
tacgtctttc caatattctt tatttaggaa gtaagccaaa ggcagcataa ccgcaaatag 2144100
gaaaatcatc acgacatagc ctatacgttt gcgttgcagt tgtgcaggtt cgcccatgta 2144160
cacaaggtaa ttgaccaaat cgcgtacata tgcgtcgtac tctttttgga tcactttgcc 2144220
gttaggcagg cggcggctgt gcaaaccggt agattcccaa tacagcttag gcttcatctc 2144280
gccgtgttcg tcttttacca taaccggctg acctttggca tccaactcaa cggcttgaac 2144340
accttgttgc tcccacaacg ggtggggcat accgacttta tcgaatacag tattgttcca 2144400
gccgctcgga cgggtcggat ctttatagaa gccgcgcata taggcgtaaa ggtagtctgc 2144460
acctttggaa cgcgcaatca acgtcaaatc gggcggagca gcaccaaacc attttgccgc 2144520
atctttcggg ttcatcgccg aatgcatgac atcgccgaca ttatcggtgg taaacatcag 2144580
gtttttcttg atttcttcgt cagtcaaacc gatgtctttc agacggttga agcgcatacc 2144640
gcttgcagag tggcaagaca aacagtagtt tgtaaagatt tgcgcaccgt gctgcaggct 2144700
gacttggtca cgcaggtcga tatcgacttt ttcgtagtgt ccgccgccgc tggcgacggc 2144760
tgcactcata ggcactgcca gcaataaggc agcaaaccag tttttcagag tttgtttcat 2144820
tttcgctgcc ctcatcagat attggttgca aacaagtaag caccaacaac ggtaataccg 2144880
acgtaaacaa agaacataat tttttgttta gtagtgctca tggttacgcg ttcaggaact 2144940

ggtttgttgg tatccagttt ggtatagaac ggcatacccca ggaagaatgc aaagtagacg 2145000
aaagacagga tacgtgcaac caaagtacgc gtatcagttg ctaccattgc acccaaaata 2145060
cccaaaccga tgaaggcaat gatgaacaga accaatgcgg ttttgaagat tgggccgcga 2145120
tagcggacag atttaacctc gcctttatcc aaccaaggca gcaaggcgat cagtacaact 2145180
gctgcaccca taccgattac accccatacc tgagtaccgg caaaggaagg aatcgcacgc 2145240
agaattgcgt agaacggagt gaagtaccat accggcgcaa tgtgcggagg tgttttcagc 2145300
gcattcgctg catcgaagtt tggcgcttcc aagaagtagc cgccgccttc aggtgcaaag 2145360
aacatcacgg cacagaagac aatcaagaat atcgttactg ccaatatatc atgcacaaca 2145420
taatacggaa aaaaaggtat gccatctaga gggacaccgt tttcatcttt cagctttttg 2145480
atttctacac cgtcagggtt gttggaaccc acttcatgca aggcaatgat atgagccaca 2145540
accaagccga gcaataccaa aggtacagcg ataacgtgca gggcgaagaa tcggttcaaa 2145600
gtaacatcgg aaacgttgaa gtcaccgcgg atccaagtgg acaaatcagg accgataaca 2145660
gggatggcgg agaacaggtt aataattacc tgcgcacccc agaaggacat ttgaccccaa 2145720
ggcagcaggt agcccataaa ggcttctgcc atcaatgcca agaaaatcag ggaaccgaaa 2145780
atccacacca attcgcgcgg ttttttgtac gaaccgtaaa tcagaccacg gaacatgtgc 2145840
agataaacga cgatgaagaa gaaagatgcg ccggtagagt gcatatagcg gataatccag 2145900
ccgccggaca cgtcgcgcat gatgtactct actgcggtaa aggcagcagg cagatggtag 2145960
gcgttaaggt tgccgtccgg tttgtagttc atggtcagga aaataccgct gacgatttga 2146020
atcaccagca ccagcataga caatgagccg aagaaatacc agaagttgaa gtttttaggc 2146080
gcatagtatt cagacagatg ctctttccac attttactta atggaaaacg ggcatctacc 2146140
cagcctaaca atgcttttgc tttgctattg gtttggtttg ccataattat cgttccttat 2146200
tcttagtctt cgcccaccaa gatagttgtg tcgctcaagt atttatatgg cgggacaacc 2146260
aggttggtcg gggcaggaac acctttatat acgcggccgg ccaagtcgaa tttcgaaccg 2146320
tggcaggggc agaagaagcc gcctttccag tctgcaccca aatcggcggg ggcaatgtcg 2146380
ggacggaagg tgggcgagca gcccaaatgg gtgcagatac cgatggcgac aaggatgttc 2146440
ggcttaatcg aacgggtctc gtttttagca tactccggct gctgttccgc atcggaattg 2146500
ggatcggtaa gttcgccgtt caggcctttc aggtctttaa gctgctgatc tgtacggttg 2146560
agcacccaaa tcggtttgcc ttgccactcg gcggtcagca gctgacccgc ttcgatttta 2146620
ctgacatcca cctcgacggc agcaccggcg gccttggctt tttccgaagg gaaaaaactg 2146680
gccacaaacg gcgttgccac acccaatgct gccactccgc ccgcgccgca ggtcgcgagt 2146740
gtcaggaaac ggcggcggcc gttgttgatt tcttgattat ccattattca gtcgtcctaa 2146800
tattttggga ataccgagcc attaaacgtt gcaattttac ccagtttgca gtgatactca 2146860
aagcattatt taaaataagg taaagtttta tgatatttct caagactcaa gccggattgt 2146920
tttcgtcaaa atggcacact tccaacccga aacctctgc cgccgattct gccagcgcgc 2146980
gtacgccgta acgttccgtc gcgtgatgcc ctgccgaaat gaaagccgta cccgtttcat 2147040
tggcaaggtg gtattgggct tcagagattt cccccgtcaa atacagatcg acaccttcgt 2147100
ctattgccgt ctgaaaaaac ccctgcgccc cgccgctgca ccatgcaacc cgtcggattt 2147160
cgcgttcggg attgccgata acgacaggct tacgttgcaa aactgtttca atatgcgccg 2147220
ccaatgcgcc gagtgtcttg gcttgtttca ggctgcccga gttgagcagg ttttgttcgc 2147280
cgaaccgttt ttctgtcgca aaacccaatc tgtcggcgag ttgggcattg ttgcccagtg 2147340
tgggatgtgc atccaggggc agatggtagc ctgccatatt gatgtcgtgc cgtaacagtg 2147400
cggcaatccg ttcttttttc caaccagtaa cggtcggcaa ctcgtttttc cagaacatac 2147460
cgtgatgtac caaaagcaaa tctgccttct gctccacagc aaaatcaatc gctgccctgc 2147520
ttgccgttac cgacgtaacg attttcccga tatattccct cccttcaacc tgcaaaccgt 2147580
taggggcgta atctttaaac aacgctgtct gcaatgtttc attacaccaa gtcagaaaat 2147640
ccctgcacaa taccatcttt tttcctaatc gctttaaaca agcgggcatt ctaatcgcaa 2147700
aatgtccgga attcacattt ttccgatttg cacccgcata tgaattattt taatatgcgc 2147760
cggttcaata tgccgtctga agccccatgg attccattat cgaattgcgc cacctcaaaa 2147820
ccctgctggc acttgaagaa accggcagcg tctcccttgc cgccaaacgg gttttcctta 2147880
cccaatccgc cctttcccac cagatccgta tgctcgaaaa ccactacggc acgccgctgt 2147940
tcgaacgcaa atccacgccc ttgcgcttta ccccggtggg cgaaaggctg ctgcgcctcg 2148000
cccacgaact tatacctcaa gttgctgttg cagaatggga tttggcgcga atcacggaag 2148060
gagaggcggg agagctgcgg attgccgtcg aatgccatac ctgtttcgac tggctgatgc 2148120
ccgccatggg cgaattccgc ccgatgtggc cccaagtcga attggatatc gtatcgggat 2148180
tccaagcgga tcccgtcgga ctgctgctgc aacaccgtgc cgaccttgcc attgtttccg 2148240
aagcggaaaa acaaaacggt atttctttcc aaccgctgtt tgcctacgaa atggtcggca 2148300
tttgcgcacc agaccatccg cttgccgcca aaaacgtttg gacggcggaa gactttatcg 2148360
gggaaaccct gattacttat cccgttcccg acgagatgct ggatttgccc aaaaaaatcc 2148420
tgattccgaa aacatcaac ccgccgcgcc gacacagcga gctgaccatc gccattatcc 2148480
aactggttgc cagcagacgt ggcattgccg cccttcccta ttggacagtc atgccctacc 2148540
ttgaaaaagg ctatgtcgtc caccgccaaa ttactgccga cggactgcaa agcaaactgt 2148600

atgccgccat ccgtaccgaa gatacggaca agagctatct gaacaatttt tgccaaatca 2148660
tacgcgaacg cggttttgca gatttgcccg gactgagcga actggaaccg gtctgacccc 2148720
ttattcaacc atacccggca gtttttctat tttttcatgt atagtggatt aacaaaaacc 2148780
agtacggcgt tgcctcgcct tgccatacta tttgtactgt ctgcggcttc gtcgccttgt 2148840
cctgattttt gttaatccac tatactgttt ttgatttttg cccaatctgt aatctttaga 2148900
ttgccaatgg gaaaccgtct actacaaata aaaaaccctg cgataagcag ggtttttttga 2148960
atttccaaca ttaacgtttg gagaattgtt ttgcacggcg tgctttgcgc agacccggtt 2149020
ttttacgttc gacttcgcgg gcatcgcggg taacaaaacc agcttgagac aaggcgggtt 2149080
tcaacgcggc atcgaagtcg atcagggcac gggtaatgcc gtggcggatt gcgccggact 2149140
ggccggtttc gccgccgcca acaacattga ctttgatgtc gaaagattcg gcgttttcag 2149200
tcagaaccaa gggttggcga acaaccattc ggctggtttc ccgtgcgaag aattcgtcaa 2149260
cgggacgacc gtttacgatg atttgacctg tacctttaat caggaataca cgagccactg 2149320
aacttttgcg gcggcctgtg ccgtagtagt atttaccgtt catgtcgcgt ccttatttca 2149380
gttccaaaac tttgggttgt tgcgcagcat gggcgtgttc cgcacccgca tacactttca 2149440
gttttttaat catggcgtaa cccagaggac ctttgggcag catacctttt acagcttgtt 2149500
ccaaagcgcg gcccgggaat tgctcttgca tttcgcggaa ggtgcgttcg tagataccgc 2149560
ctgggaaacc ggaatggcgg aagtattttt tatcttcgaa tttggcaccg gttacacgca 2149620
gtttgtccgc attgataaca atgatgtaat cgccggtatc gacgtggggg gtgtattcag 2149680
gtttgtgttt gccacgcaga cggctggcga cttcggccgc aacgcgaccc aagactttgt 2149740
cttgggcatc gatgacgaac cattcgcgct tcacctcgtg gggtttcgct gaaaaggttt 2149800
tcatagtgga aatccagata gatatagaaa gttgtaaatt ttaaagacag gattcgattt 2149860
tgtcaatcgc attaccgcgt tacggaagga ttttccggat ttcggcagac tgcatactgc 2149920
tttttcgggc ggggcggcgg ccaatgtgaa aaaccgcatc gttgcgatgc ggttttgaat 2149980
gggaatcccc gcgagagccg tttcggccga atccgcttga accttgctga caaggcggct 2150040
gcctcgggta gtttcgggtg cgtccgcaaa aggacgctcg cgcccactac tgctcccggc 2150100
aaccttaagc gaacttattg gttcaaagga atatatgcct tcgcggacac cgcagggaaa 2150160
aaggggttat tcctgcgcca agcgggatag tgctttttgg caggcgttgt ccatatcggc 2150220
tattttacgc gcaaaatcgc cgattgccaa atcgccgccg ttcagggagg ttttcaacag 2150280
gtcgtggacg acgttgagcg cggccataat gacgattttt tcgctgtccg cgacgcgtcc 2150340
gccttcgcgg atggcttcgg ctttgccgtt gagcattccg actgcctgca acagtgtgtc 2150400
tttttcttct gccggcgtgt tgacggtcag ccgggcgtgc atgacttcga tgtggacttg 2150460
ttcgatgttc atcctttaat ccttattgct gcgtttcctg ccattggggg aggcgcgctg 2150520
ccagtgcgct gatttttttcc ctgctctgtt cgagcaggct gcggtatcgt gtattttctt 2150580
ctgtcaggct gtcaattttg ttttgcaggt cttctttgag tttgccgact tggacgagca 2150640
gggcttcgct gagttcgtcg acggcggttt cgtgttcgag tttttgccgc tcgtgcgccc 2150700
gtttgagttc ggcgacggtt tctttgaggc ggcggttttc gctgacgagg gtttcgaatt 2150760
tttgtaccaa cgtataaacg ctgctttcga gtttttcgat attttgtttc ataaccttac 2150820
ctgtccgtat gccgtctgaa ggcttcagac ggcatctgtc tgttgtttat tcaaaacgcg 2150880
cgctgcgttc catcagtctt tcgacaacct gttgcggggt catttctttg cggatgagtt 2150940
gcagcagagt ttgggtaatc ggcatgtcga tttggtactt acaggcagta ttgaagactt 2151000
cttctatcgt gctgacccct tcggaaacgt gtccgatttc gaccagcacc tgatgcagtt 2151060
ccttgccttc tgccaaaccc aagccgacgc ggcggttgcg cgaaagtgcg ccggtgcagg 2151120
tgaggatgag gtcgccgatg cctgccagcc ccatcatggt tttgggctgt gcgcccattg 2151180
cggaggcaag gcgggtgatt tcagctaatc cgcgcgtaac cagtgcggca cgggcgttaa 2151240
gcccgtactc taggccgtcg gacaatccgg tggcaatcgc cataacattt tttaccgcgc 2151300
cgccaaccgc cacgccgata acatcggtac tgccgtaaag cctcatgacg gtcgtgttga 2151360
gctgcggtac gagttcttca atccactctt ggttttcgga ggcaaggacg acggcgcagg 2151420
gcagttgttt ggcgagttcc tgtgcaaaac tcgggccgga aagtacgccg attttcttat 2151480
tgtcgggcaa tacttctttc aagacttgaa aggtcagcag cccggtatcc tgctcgaatc 2151540
ctttgcaggc ggcgaggacg gggaggtgtc ccgcgccgta ctgtttgagc agctctgcgc 2151600
tgcttctcaa tccggcaacg gaggttacga taaggacaag tccgctgtct ttgagcgcgt 2151660
ctgccaaatc cgcacacact tccaaggttt cgggaaagga aaagccgggc agtccgcgtt 2151720
tgttttcacg cgcttcctgc atttgacgga cttggtctgc gttgcgcgtc cacagggata 2151780
cgcggttgcc gtgttgggaa aaatgcaggg cgagcgccgt accccacgaa cctgcgccga 2151840
taacggtaat tttcattggt cgtctttcaa catatcactg ccgttcactt taaaacaatc 2151900
ggtgtttctc tgcaagtgcg gtcagggaaa tgccgtctga aaggcgttca gacggcattt 2151960
tgccccgatg cggcactatc agcctgtatt gcgcaaacct tgcgccacgc cgttgatggt 2152020
caggtgcacc atcagaaggg cgtgcggatt gtcgggttct ttacgcaggc gtttgagcat 2152080
ggcgacttgc aaaccgttga gcgcgttcag gtagggaatc ctcaaagcga gcgaacgggc 2152140
gaggctgcgg ttgtcgcgca aaagctcttc ggtttgcagt aggtcgagca gtgctttgcg 2152200
gctgcggcgg tattcttcct taatcatccc gaagatgatt tttgccttat cgggcgattc 2152260

gctcaagccg gcatagtttt ccgcgagggt gatgtcggtt ttcgccatca cttgttccat 2152320
attggagagc atggcttgga agaacgggtt gctttgggcg tgttcgcgca gggcggcgag 2152380
cgtttcgggt ttgtcttcgc acaaggtttc caccgcgctg ccgaaaccgt accaagccgg 2152440
cagcatgagg cggttctgca tccaggaaaa tacccacgga atcgcgcgca agtcctgaat 2152500
ccgcgccaag gttttgcggc tggcgggacg gctgcctagg ttgagggtgg cgatttcctg 2152560
aatcgggctg gtttgcagaa agtagtcgat gaagtcggga tgggtaatca gttcgcggta 2152620
gtatttgaac gatacgtccg acaatgcctg catcagtttg gcatcagggt ctttttttatc 2152680
cggcaggatg ctggcttcca aagtcgcggc aaccaaggtt tccaagttgc gttgggcatt 2152740
gccggggtcg gcgtatttgg cggtaatgac ttcgccttgt tcggtgatgc ggatttgtcc 2152800
cgccacgctg cccgccggtt gggcgagaat ggcttggtaa gaagggccgc cgccgcgacc 2152860
tacgctgccg ccgcgtccgt ggaacaggcg catacggaca tcgtattttt tgaagagttc 2152920
gaccaagccc aattccgcct gatagaggca ccatgagctg gtaacgtagc cgccgtcctt 2152980
gttggagtcg gaatagccga gcatgatttc ttggatgttt ccacggcttt cgagcagtgc 2153040
atcgtaccag tcgaggcgga acatggtttc catgaccgga caggcgtttt ccaacgcttc 2153100
aatggtttca aacagcggca cgatattgat gcggctgtgc ggtttgccgt tttccaccgc 2153160
caacaggccg gtttctttca gcagcaatgc caaggcgagc aggtcgctgg gttgttcgca 2153220
gttggaaata atgctttgtg ttacggcatc ttcgccaaat tcgtctttga ttttgcgcgc 2153280
ttcgttgaaa attgccagtt cgtggcgggt atggtcgctg tatgtgataa acgggctgta 2153340
cagaggacgt tgatggctca attcgcgcaa cagggcggtt tgtttttgct cttcgttcag 2153400
gcggttgtag tcttccaagc ctgcgtgttg gaaaagctcg gcaaccacat cggcgtgttt 2153460
gcctgcgtgt tggcgcaagt cgagcggcat catgtgaaag ccgaacacgg atacggaacg 2153520
gatgaggtct gccaaacggc cttcggcaag cagacggctg ccgttgtcga taagggaacg 2153580
ttgcaatttt ttcaaatcat ccagaaactc ttgtgccgaa gcataaggct cgagaaagcc 2153640
gaatttgcag cccataccca aaccgagcgc gcgcgctttg cccatagcgc gcgccataat 2153700
gtaggcgatg gcgcggcggt agggttcttc ggcgcgggcg atttcttcgt cgggcgattt 2153760
gtcggacaac gccgttacat cgccgttgac tttgacgcgg cggatggaga gcggcagttc 2153820
gcggtagagt ttgtcgagtt cgccgcgata gaagcggaac acggcatcgg cgtggcggcg 2153880
gaaggcaaag cgcagggttt cggcagaaac aaacggattg ccgtcgcggt cgccgccgat 2153940
ccagccgccg attttgagga tgtccggaac gcggacgccg ggataggccg tctgaaagtc 2154000
gtgttccatc ttgcggtaga gcttgggcag ggcttcgaaa aagctcatcg ggaagatgga 2154060
cacgccgttg ttgatttcgt cgttgacgct gagtttgtgg cggcgcgttt cgctggtctg 2154120
ccacaagccc agcaggatag tgtcgatttc gcggcgcagc cgtgccagcg cgtcggcatt 2154180
ggtgcagcgt tcgcgttgcg gcaacagtgc gcggatgcgg cggttgaagc ttaagacggt 2154240
ttggcgttgc acttcggtcg ggtgcgcggt caaaacggcg gtaacggacg tattgtccaa 2154300
ctgccgctgc accgatttgc cgtcggcttt ccccgctttg agcctgcgga cggtttccgt 2154360
caggctgcct tccgcgccgc cgcgtccggc ttcttcgtgg atttggcggc ggcgttcgtg 2154420
gtgcacgtct tcggcgatgt tcaaaatctg ggcgaacagg ccgcaggcca aggttaaatc 2154480
gtgggtttgt tgttcgtcca attgcggcaa tactttttca atcaatgccg cgctgtcgtc 2154540
ggaagtggac aagagtttga ctgtttcgac aaccaacggc gaggcttctt cgtgcaggag 2154600
gttgaacagg gattgtttca gaaattccgc gtccgccgcc aaagccgcgt cctttggatt 2154660
gttcagaata tgcagttgca tgatttttct ctctcgtctg ccgtaaatat tgtaaatgta 2154720
ccccaaatgc cgcatccgtg ccaaaccgtt cacactttaa ccgcccgtgt cccgaaatgc 2154780
cgtctgaagt tgaacgccgc ccgacggcag cgttacaatc gcccgcaact gtttttttcc 2154840
gaacatcatc atgaccacga ccgaacacga caacgacgat gcattcctgc tgcggtacag 2154900
ccgccacatc ctcttggacg aaatcggcat cgaagggcag cagaaacttt ccgccgcgca 2154960
tattttggtc gtcggctgcg gcggtttggg tgccgccgca ctgccctacc ttgccgcttc 2155020
gggtgtcggc acgctgacca tagccgattc cgacacggtc gaactgcaca acctgcaacg 2155080
ccaagtcgca tttgacgagg gcgatgtcgg caaactcaaa accgaagcct tggcaggccg 2155140
cctgaaacgc atcaaccata ccgtcaacgt ccgcgccgtc aacgaaaaac tcgacggctg 2155200
ccgcctgacc ggtttggttc aagccgccga catcgtttta gactgttgcg acaactacgc 2155260
cacgcggcaa gccgtcaacc gtgcctgcgt gcaaacgaaa acaccgctgg tttcaggggc 2155320
ggcggtacgc tttgaagggc aacttgccgt gtaccgtccc gacttgcccg actcgccgtg 2155380
ttacgcctgc ctgtttgacg gcggatcggc ttcagacggc atctgttctc tcttcggcgt 2155440
gttctcgccg ctggtcggca tcatcggcag tacccaagcg gcggaggctc tgaaaatcct 2155500
gctggatgcg ggcgaaccgt cgcacggcag gctggcggtt taccgtgcct tggaaggggg 2155560
ctggcaatat ttcgacctgc cgcgcaaccc tgaatgcccg gtttgcggca cagcgcgata 2155620
aaccctgccg ccgtttcaga cggcatccaa acggatgcgg cggaacggtt ttaaaaattt 2155680
aaaaatttac atttctttgc aaaaaaaaaa aaaaaaaaat aaacttacct tataattgca 2155740
attgttttag caatgtctgt ttcgcagact cattgagtaa aacgttttcc ccgtaatgtg 2155800
tttggccgtc tgtccccttt gggttcggac ggcttttttt tggctgtgtt tgaatacccg 2155860
gttggtttta tctgtttgca gcgggggaag ccgcttattt ccgttcgggc ggaaaacggt 2155920

tccatcggat aaaaggcatt ttgtccgact gattaaagtt atagtggatt aacaaaaacc 2155980
agtacagcgt tggctcgcct tagctcaaag agaacgattc tctaaggtgc tgaagcacca 2156040
agtgaatcgg ttccgtacta tttgtactgt ctgcggcttc gtcgccttgt cctgattttt 2156100
gttaatccac tatatatctt aggtttgcat cggcggaata ttcaaacaca gccttttttta 2156160
aggaaatccg gatacggcgg cgcatcaata atgcggcgga atctcgtcgc gcagggaata 2156220
cggctcttgc gcgtcgggat tcctgtcctg catttttga tacagcagcc tcaactgagc 2156280
ctgctgcaaa tccagcgtct gccgcaattc cgccaccatc gcgttcaggc cggcgattac 2156340
gtcctcctga agcgcggatt ggatttccag ttcgacaata cggcgttcca actcttgaac 2156400
cgcgtccatt tacagcacca tcgcggcaat ccagccggca atcagcagcg ggatgttgta 2156460
gtggatgaag gtcgggataa cggaatcgcg gatgtggtcg tgctgcccgt cggcgttcag 2156520
ccccatcgtc gggcccagcg tggaatcgga cgcaggcgaa ccggcatcgc ccaacgcccc 2156580
cgccgtgccg acaatggcga cggtggcaag cggcgaaaaa cccaaaccga cacacaaagg 2156640
cacataaatc gcggcaataa tcggcaaagt ggaaaaggac gaaccgatgc ccatcgttac 2156700
caaaagcccc accaccagca tcgccaatgc cgccatacct ttgctgttgc cgaatatcgc 2156760
catactgctt tccaccagcg gctgaatatg cccggtcgca ttcatcacgg cggcaaaacc 2156820
ctgcgcggca atcataatga agccgaccat cgccatcatc ttgatacctt cgccgaatac 2156880
gtcgtttgcc ttgtcgcggt taatgacccc caacatcata aatacggcga aaccgagcat 2156940
cgcgcccaac accagcgagt cttcatacat caactggatg gcaaagcata cggcaatggc 2157000
gacggcggcg gccaggctgc ggtaggcgga cggctgcgga cggtttgccg catcggcgtt 2157060
gcccgccgta tcggcattgt tgctttggta caggcgcggt ttgcggtaat ggacaaacgc 2157120
cagcaggagt ccggccagca ttcccaacgc gggaatcgcc attgccgcca tcacgttaat 2157180
gtttttcaca tcaagctgcg gcgcggcgga atggatgttg cccaacagga tttcgttcaa 2157240
aaaaatcgcg ccgaagccgt aaggcaggaa catataagtc gtaaccagcc cgaaagtgat 2157300
gacgcacgca atcaggcggc ggtcgatttt caggcggttg aacaccaaaa gcagcggcgg 2157360
aacaatcatc gggataaagg caatgtggat ggggatgatg ttctgactca tcatgcccat 2157420
cacaaggatg atggaaagca gcagccattt gaccgcgccc tcgcccgaac gcacgctgtc 2157480
gggcataccg ccccggttca gcttgcggac gaccgcgccg gcaagctgct gcggcaggcc 2157540
ggaatgggta atcgccattg caaacgcgcc gagcatcgca taagaaagcg caatcttcgc 2157600
accgccttcc aaacctttgt tgaacacggg gataatcccc gcctgactga cctgtcccgc 2157660
cgcatcggca atgttttgca gcggcatacc cgccaccgcg ccgccgacaa acgcgccgac 2157720
cgtcaggctc aataccacgt gcacgcgcga cagcgacagc accagcataa cgattacggc 2157780
aactacgact gcattcattg tttatcgctc caaacctata aatgtttaca tatcgaaaca 2157840
catcataacc caataacggg aaacccgcca attttgcaaa caattatttc aaatgcttca 2157900
tatacttccc cagcgtaacc ctgtccaaac ccgccaaatc cggcagggtt tccactcctg 2157960
aaaaaccatt ctccgccaac acgccgcgca ccgccgcgcc ctgatcgaaa ccgtgttcca 2158020
gcaataaaaa accgccttcc gccaaacggt cgggcgcgcc ttgcgccaag gtgcggatgc 2158080
agcttaggcc gtctgaaaag tcggtcagcg cgatttgcgg ctcaaaccgc aaatcgcctt 2158140
gcaacaaatg tttatcgccg ttttcgatat agggcgggtt ggacacgatg atgtcccatt 2158200
tcccttcaga cggcatatcg gtgtcgaacc acgaaccgtg tgcaaattcg acccgcgcgc 2158260
ccaaatccgc cgcatttttc cgcgccgttt caagggcggg cgggctgatg tcggatgcgc 2158320
gcacaaacgc atcggggcgt tcgagcgcga cggttacggc aaccgcgccg ctgcccgtcc 2158380
ccaaatccca cacgcgcccg ttttcgggca ggcgcgccaa tacggcttcg accaaatgtt 2158440
cggtttcggg gcgcggaatc agcacgctcg gattgactgt aaagcgtctg ccataaaatt 2158500
cgcgcacacc taaaatatag gcaaccggct cgccgttcag acggcgttgc gccagcctgt 2158560
ccgcccgctg tcggacttcg tccggcattt cttccccgcc ccgcgtcaac aactgcacgc 2158620
gcgtatattc cgaaacatat tgtagcagca ttcttgcttc atttttaggc agttttgaca 2158680
agcccaacca tttatcaaac gtcattttta tcccgtctgc cgctgatgcg gcttttcttt 2158740
ccttattctt tccggcaaac gtaccgatgg tggcaaccgc aaatgcggca taccacaaat 2158800
aaaatcctgc accgtagcgc acaatatccg atgtattccc tgcttcatcg acgtatacgg 2158860
ctttcacact gaaagccacc aacgccaagc cccaaagtgc cgcatggaca ggcacgacct 2158920
tcttccgcaa cgccagcaaa acaatggccg ccaaccaaac ataattcgca tagaccgcac 2158980
aatacctgat atccaaagaa gcaaatatcg accccaaaat caaaacggtc aaaccctcca 2159040
tgcttccatg attgcccaaa taaaatgcaa cattggataa agacgctatc cacagggcaa 2159100
ccgacaccag caacatcact atgggaaaac ttggtttccg attctgttcc tgcatggttt 2159160
tatcctaatg taaaaggccg cctgaaaacc tttcagacgg catcgtgccg gattccgcgt 2159220
cagattgcgc tgccgccgac ggtcagtccg gcatcaatcc gcaaagtcgg ttgccccacg 2159280
ccgacgggga cgctctgccc ttctttgccg cacacgccga caccgctgtc gagcgcagta 2159340
tcgttgccta tcatggaaac gtgtttcagc acttcggggc cgttgccgat gatggtcgcg 2159400
cctttgacgg gatattgcag cctgccgcct tccacccacc acgcttcgga cgcactgaac 2159460
acgaacttgc cgctggtaat gtccacttgt ccgccgccaa agttgacggc gtaaatgccc 2159520
ttgtcgatgg acgcgatgat ttcttccggc tcatagctgc cgttttccat aaaggtattg 2159580

gtcatgcgcg gcataggggc ggaagcgtaa ctttcgcggc ggccgttgcc ggtggactgc 2159640
gtacccgtca ggcgggcatt ggtttcgtcc tgcatatagc cgactaaaat gccgtcttca 2159700
atcaatacgg tgcggcgggt ttcgttgcct tcgtcgtcga tgttgagcga accgcgccgg 2159760
ccggcaatat caccctgatc gacgacggta acgcctttgg cggcgacgcg ctcgcctatt 2159820
ctgccggaaa agacgctggt tcccttgcgg ttgaaatcgc cttccaaacc gtgtccgacc 2159880
gcttcgtgca gcaacacgcc cggccagccg ttgcccaaaa cgacggtcat ttcgccggcg 2159940
ggcgcggggc gggattcgag gttggtgagt gcctgtttga cggcggcatc gacaaaccga 2160000
tgaaccaagt tttcatcgaa ataagccaag tcgtagcgtc cgccgccgcc cgcgctgccc 2160060
tgttcgcggc gttcgccctg tttggcgata acggtaacgt tcaggcgcac catcgggcgg 2160120
atgtcggcgg cgtgtttgcc gtccagacgg gcgaggtaaa ccatatcgta ttcgcaggtc 2160180
aaaccggcca tcacttgcac gatgcgcgga tcggcggctt tggcgattgc ttccactttg 2160240
ttcaacagcg cgactttggc ggcggaatcg aggccggcaa tggggtcgga cgcggaacaa 2160300
accggcttgc cgcgcgtttc agacggcatt ttggcggaca ccttgccgcc tgccgcccca 2160360
atcgcgcgga cggcgcgggc ggaacggttt atcgaatcga tgcacaggct gtcggcgtag 2160420
gcaaaggcgg ttttgtcgcc cgaaacggcg cgcacgccca cgccctgatt gatttggaag 2160480
ctgcccgatt tgacgatgcc ctcttccaaa tgccagcttt cataagcggt gcgctggcag 2160540
tagatgtcgg cgtaatcgac gtggtgcgcg ccgatgatgc acaggctttt ggcgagcagt 2160600
tcgggggaaa ggcggttggc ttcgagcagc cgcgcctgta cggcggaata ggtcggatgc 2160660
atagtgtcgg cgcataaaaa atcaggggct tgattatacg gcatttgtta tatagtggat 2160720
taacaaaaaa cagtacggtg ttgcctcgcc ttgccgtact atttgtactg tctgcggctt 2160780
cgtcgccttg tcctgatttt tgttaatcca ctatagaaat gcgccgtgcc gcctgaaatg 2160840
taagattttt gccaacgccc cctgcttttg tgtacactta aagctccttg tcggagtgcc 2160900
gccgccgggc ggctgagatt gcgaaagcag aatccgtaga acctgtcggg gtaatgcctg 2160960
cgtaggaaac aaaccgtcaa atgccttatc aggcttccgt tccctttcc gcacttcccc 2161020
gccccatttt catgtttttt aaggacttga tatgtcgggc aatgcctcct ctccttcatc 2161080
ttcctccgcc atcgggctga tttggttcgg cgcggcggta tcgattgccg aaatcagcac 2161140
gggtacgctg cttgcgcctt tgggctggca gcgcggtctg gcggctctac ttttgggtca 2161200
tgccgtcggc ggcgcgctgt tttttgcggc ggcgtatatc ggcgcactga ccggacgcag 2161260
ctcgatggaa agcgtgcgcc tgtcgttcgg caaacgcggt tcagtgctgt tttccgtggc 2161320
gaatatgctg caactggccg gctggacggc ggtgatgatt tacgccggcg caacggtcag 2161380
ctccgctttg ggcaaagtgt tgtgggacgg cgaatctttt gtctggtggg cattggcaaa 2161440
cggcgcgctg attgtgctgt ggctggtttt cggcgcacgc aaaacaggcg ggctgaaaac 2161500
cgtttcgatg ctgctgatgc tgttggcggt tctgtggctg agtgccgaag tcttttccac 2161560
ggcaggcagc accgccgcac aggtttcaga cggcatgagt ttcggaacgg cagtcgagct 2161620
gtccgccgtg atgccgcttt cctggctgcc gcttgccgcc gactacacgc gccacgcgcg 2161680
ccgcccgttt gcggcaaccc tgacggcaac gctcgcctac acgctgaccg gctgctggat 2161740
gtatgccttg ggtttggcag cggcgttgtt caccggagaa accgacgtgg caaaaatcct 2161800
gctgggcgca ggtttgggtg cggcaggcat tttggcggtc gtcctctcca ccgttaccac 2161860
aacgtttctc gatgcctatt ccgccggcgc gagtgcgaac aacatttccg cgcgttttgc 2161920
ggaaacaccc gtcgctgtcg gcgttaccct gatcggcacg gtacttgccg tcatgctgcc 2161980
cgttaccgaa tatgaaaact tcctgctgct tatcggctcg gtatttgcgc cgatggcggc 2162040
ggttttgatt gccgactttt tcgtcttgaa acggcgtgag gagattgaag gctttgactt 2162100
tgccggactg gttctgtggc ttgcgggctt catcctctac cgcttcctgc tctcgtccgg 2162160
ctgggaaagc agcatcggtc tgaccgcccc cgtaatgtct gccgttgcca ttgccaccgt 2162220
atcggtacgc cttttcttta aaaaaaccca atctttacaa aggaacccgt catgacccgt 2162280
atcgccatcc tcggcggcgg cctctcggga aggctgaccg cgttgcagct tgcagaacaa 2162340
ggttatcaga ttgcactttt cgataaaggc tgccgccggg gcgaacacgc cgccgcctat 2162400
gttgccgccg ccatgctcgc gcctgcggcg gaagcggtcg aagccacgcc cgaagtggtc 2162460
aggctgggca ggcagagcat cccgctttgg cgcggcatcc gatgccgtct gaacacgcac 2162520
acgatgatgc aggaaaacgg cagcctgatt gtgtggcacg ggcaggacaa gccattatcc 2162580
agcgagttcg tccgccatct caaacgcggc ggcgtagcgg atgacgaaat cgtccgttgg 2162640
cgcgccgacg acatcgccga acgcgaaccg caactcggcg gacgtttttc agacggcatc 2162700
tacctgccga ccgaaggcca gctcgacggg cggcaaatat tgtctgcact tgccgacgct 2162760
ttggacgaac tgaacgtccc ctgccattgg gaacacgaat gcgtccccga aggcctgcaa 2162820
gcccaatacg actggctgat cgactgccgc ggctacggcg caaaaaccgc gtggaaccaa 2162880
tcccccgagc acaccagcac cctgcgcggc atacgcggcg aagtggcgcg ggtttacaca 2162940
cccgaaatca cgctcaaccg ccccgtgcgt ctgctccatc cgcgttatcc gctctacatc 2163000
gccccgaaag aaaaccacgt cttcgtcatc ggcgcgaccc aaatcgaaag cgaaagccaa 2163060
gcccccgcca gcgtgcgttc agggttggaa ctcttgtccg cactctatgc catccacccc 2163120
gccttcggcg aagccgacat cctcgaaatc gccaccggcc tgcgccccac gctcaaccac 2163180
cacaaccccg aaatccgtta caaccgcgcc cgacgcctga ttgaaatcaa cggccttttc 2163240

cgccacggtt tcatgatctc ccccgccgta accgccgccg ccgccagatt ggcagtggca 2163300
ctgtttgacg gaaaagacgc gcccgaacgc gataaagaaa gcggtttggc gtatatccga 2163360
agacaagatt aaagccgccc gaaaggacac cttatgacct tcccgcccct aaaatccccg 2163420
ctcaaattct acgccgtcgt ccccaccgcc gattgggtgg ggcgcatggt caaagcaggt 2163480
gccgacacgg tgcaactgcg ctgcaaggcc ctgcacggcg atgaattgaa acgcgaaatc 2163540
gcccgctgcg ccgcagcctg tcagggcagc cgtacgcagc ttttcatcaa cgaccactgg 2163600
cgcgaagcaa tcgaagcggg cgcgtacggc gtgcatctcg gacaagaaga catggacacc 2163660
gccgaccttg ccgccatcgc cgccgccggt ttgcgcttgg gtttgagtac gcactccgtt 2163720
gccgaactcg accgcgccct gtccgtacac cctagctaca tcgccagcgg cgcgattttc 2163780
ccgaccacga ccaaacaaat gcccaccgcc ccgcaaggct tggacaaact gcgcgaatac 2163840
gtcaaacaag caggcggcac gcccgtcgtc gccatcggcg gtatcgactt gaacaacgcc 2163900
cgagccgtac tcgccaccgg cgtttcctca ctcgccgccg tccgcgccgt aaccgaagcg 2163960
gcaaatcccg aagcggtggt taaagcgttt caggctttgt gggatggata aaaccgaaag 2164020
aagaaaattc aattgccgtg taggcaaaac ttagcccgtt atcgcaaaca tacttaactt 2164080
taaatgtggc atatcatcaa attccgtcat tcccgcgtaa gcgggaatcc gccttaaaac 2164140
ttgagaaacc atcatttgaa aaacagtttc cgaatttcaa aaatggattc ccgcccgtgc 2164200
gggaatgacg gcaaccggtc agttgcgtat caaaaaataa agtaattcgg ctagatatag 2164260
tggattaaca aaaatcagga caaggcgacg aagccgcaga cagtacaaat agtacggaac 2164320
cgattcactt ggtgcttcag caccttagag aatcgttctc tttgagctaa ggcgaggcaa 2164380
caccgtactg gtttttgtta atccactata aatacagaaa catcgagaaa ccatgaacat 2164440
catcttaaac ggcggacccg ccgaacttca cggcacgacc gttgccgacc tcatcgccca 2164500
aaccgcgccg caaaagccct ttgccgtggc ggtcaacacc gttttcgtcc ccaaaggcgc 2164560
gtatgcggaa acggttttaa acgaaaacga caaaatcgat atcgtgcggc cggtggtcgg 2164620
cggctaggcg gttttgcctt ttcagacgac ccctgtcccc aaaacaacgt tatggtggat 2164680
taactttaaa tcaggacaag gcgacgaagc cgcagacagt acggatagta cggaaccgat 2164740
tcacttagtg cttcagcacc ttagagaatc gttttctttg agctaaggcg aggcaacgcc 2164800
gtactggttt ttgttaatcc actatacaaa ggaacccatt atgctcaccc tatacggcga 2164860
aactttcccc tcgcggctgc tgctcggcac ggctgcctac ccgacccccg aaatcctcaa 2164920
acaatccatc caaaccgccc agcctgcgat gattaccgtc tcgctgcgcc gcgcgggaag 2164980
cggcggcgag gcgcacggtc aggggttttg gtcgctgctt caagaaaccg gcgttcccgt 2165040
cctgccgaac acggcaggct gccaaagcgt gcaggaagcg gtaacgacgg cgcaaatggc 2165100
gcgcgaagtg tttgaaaccg attggataaa attggaactc atcggagatg acgacacctt 2165160
gcagccggat gtgttccagc ttgtcgaagc ggcggaaatc ctgattaaag acggcttcaa 2165220
agtgctgcct tattgcaccg aagacctgat tgcctgccgc cgcctgctcg acgcgggctg 2165280
tcaggcgttg atgccgtggg cggccccgat cggcacgggt ttgggcgcgg ttcacgccta 2165340
cgcgttgaac gtcctgcgcg aacgcctgcc cgacacgccg ctgattatcg acgcgggctt 2165400
gggtttgccc tcacaggcgg cacaagtgat ggaatggggc tttgacggcg tgcttttgaa 2165460
tactgccgtt tcccgcagcg gcgatccggt caatatggca cgcgccttcg cactcgccgt 2165520
cgaatccgga cggctggcat ttgaagccgg accggtcgaa gcacgcgaca aagcgcaagc 2165580
cagcacgccg acagtcggac aaccgttttg gcattcggcg gaatattgaa aaaggcagca 2165640
aaaatgccgt ctgaaggctt cagacggcat cgcggtccaa aacggcggcg gcctgaaacg 2165700
gacaaaccgc cattccccgg catcacggct ttgtcggaaa aaatggaaaa accggccgga 2165760
aaaccttgcc gcccgtcccg atgccgcaac caacgaaaca ctcggcctcc acggtgtgca 2165820
ggctgccgcg caagccctaa atacggcaat agaagaagcg ttttgttgtt gtttgaatac 2165880
acttaaaaat acctaaagcc gtccgtaagc cttcatccgc aacggtttta ccgctttcgc 2165940
atccccgaat ccacgctcaa acacccccga atgacaaccc tgtccgcgcc aaatcggacg 2166000
gatgttcaaa cacgggcaac cttatttccg tcaggcacga agccctcagc tatgcctgcc 2166060
gaccccgatt tgtccgacac aatgaaagtt tgccgacccg aatcacaaac atcggcggac 2166120
aggttaattt gtttattttt catcgtatta caaaaaatct gcatttattt ttaaattttt 2166180
attgataatt attattatta gcgtataatc aaaaccactc ggaagccgtc cgttccgaac 2166240
cattaaacac catatttccc catcatcact ttcacacttg gagtcggcat atacgagaca 2166300
tacattccct ttttatatat cagatactca aaaccgaaac gccaaaccca ccttcgcggt 2166360
gggtttggcg tttatcgtcc ggctttcgcg cctatttgca agacttgagg ttcagtttgc 2166420
cgtataggga cgtgatttta cgaatttcgt ccgcatcggc ggcattcacg ccggtaaaca 2166480
aaaccgtcat acgcgacacg ctcaaagaat cgtcctgcct gtcggcttcg gcaaagtgtt 2166540
cgacaatatg cgccccgccg aatccggcgc cggcaagccg gttttgcagg gcttgggcgt 2166600
tttcccggct gttgccgaca cccaaactca atgcgccgtc aaacggtatg gggttgaaac 2166660
ctttggcaga cagctccgcc gcctgatttt cggcatcggc ggaaacgggc aggacgacgc 2166720
ggtaggtttt gtcggcaggt ttggcttggg cggtgcgttt ttcgacgctc ctgctggcaa 2166780
cgtgcgacca tttgcccaaa agtcctttga tgcggtggta gtcatcttcg tccatcgtga 2166840
ggcttgcctg cgcggcgcat aaggcatccg ccgcgccgtt ttcgcgttcc gcctgcgcct 2166900

tttcggcggc gagtttttcg cggcgggctt tttcttcacg ctgtttttc tctttcagtt 2166960
ttttctgttc cgcttctttt ttcaagcgca actgctccgc ctgttcttcg ctcagaatgt 2167020
cgccctgttt gagcagtgcg cctgtatccg attcagatgc cgcctgaacg acaggaccgg 2167080
atgctggaat attccgaaca accggcatag ttggggcaac tggttgaacc tgcaaattgt 2167140
ttgcggcatt ctgtgcctcc ggtattctgc cggcctgttt cagtgtcagt ttgtaaccta 2167200
ccgtaccgcc gaatacggca atattaatcg caaccaaaag gataaatagc catttcatct 2167260
ctgtattcct taaatatgtt catattccct gccttcggcg gcaatcatgt tcaacaaccc 2167320
gtaaatgacg aggttgtccg ccacgcgcac ggtattttcc gccaaaaatg caggcggcag 2167380
ggcttcggca acttttgccg cgccgccgcc ggtaatgatg acatcgacag gcttgcccgc 2167440
cccggttttt tctttcaaac gcccgtgcat catcataacc gagccgcaaa ccgcatccat 2167500
catgccgctg gcgacggcat tgcccgttgt ggtcgggaaa ggataacgct taccggcgtg 2167560
ccggttgagg ttggcggttc ggacggcgag cgattctttc atcaggtgga aaccgggcat 2167620
gatggttccc ccgagataat gtccgtcatc ggtgagcgcg tcaaccgtta ccgccgtgcc 2167680
gcaactgacg acgacgcagg cgttgcggct gaagcggcgg ctgcccaagg cgttgaacca 2167740
gcggtcggaa ccgtgttctt cggggtggcg gtagtggttg cgtatgccca aagcctgtgc 2167800
ggaagacggc agccactcga tttttcgggc gagctgttcc tgcacttgtg cctttttgaa 2167860
ttctccgcac acagcgcaac cgacgatgcg gacatttcca tccgcctttt ccgcccactc 2167920
cgcgcccaaa ggcgacaaat cgcggtacgg cgcgctaccg acggttgcga acgtgccgtt 2167980
ttccacccac gcccacttga gccggctgtt gccgccgtcc aacagcagaa aacgttccga 2168040
atcccgccgc ttcggcacgg aaaccggcct gtcgtcggac cgcaggctga tttcgccgct 2168100
gacgaccgtc tgtttgccct ctgccgtttc caagtgcaaa acgccttgtc cgtccacgcc 2168160
tttaaccgtg ccttcgaaca cggtttcgcc gtcgcgcaac agcaataccg ccttgccgtg 2168220
gtcgcggttg gcagcctgat attccgccac aaaaggcgca aatccgtccc gcgcatattg 2168280
caacaacacc gcgtccagtt ccaccaacag cgtttccagc agcacggcgg catcggcatt 2168340
gccccgccgc gatgccgtct gaaacagcga ttgcacggaa gcggcatttt ctacttcctt 2168400
gggcaggaca aaattgatgc cgataccgac cacggcaacc gttttgccgc ccgtcctgac 2168460
cgtttcaatc agaatgccgc ccaatttgtc gcgtccgaca accaaatcat tgggccactt 2168520
aatctgcaca tccaaaccta aacgcgacaa ggcgcgccga cacgccactg ccgcaacagg 2168580
cgacagcgaa cccaactcat actgcggccg gtcaaacacc cagccaaaac tgaacatcag 2168640
acactcgccc aaacggtgcg accacttccg cccctgccgc cccctgccct tactttgcag 2168700
gtgggtcacg catatggttt tgtgcgcctt gtccggcgca atccgcgcca attccagtat 2168760
ctcgtcgttg ctggacgcgc actcgtgctt caatgccgtc tgaaaacccg acctttcccc 2168820
cagctcgcgc aaaccttcgg catcgaaaac cgccaatggg cgcaccagcc gccaatagcc 2168880
gtcgtgttgg cgcaacagcc cgcgtatgtg cgccggcatc tgctgccaaa aaccgttgag 2168940
ctgctgcggc ttcatatccg ccatacgcgc cagttgcgag acgtgttgcg gcaaaccgtc 2169000
ggcaagctcc gccaacaccc gccagtgcga aagcttcaaa accgtcattt tccgccctct 2169060
gccgcacgga tttttgccaa agtcttcgtt gtcgaagtct ggtgcagaaa cggaattgaa 2169120
aacacctgac cgccgcgcgc caacgtttct gccgcaccga caatcttatc cgcagcccaa 2169180
tcgccgccct tgaccaaaat ctcaggtttg accgcctcaa tcaacgccgc cggcgtatcc 2169240
ccgtcaaacc acgttaccaa atccacactt tccaaagcgg cggcaacggc ggcacggttc 2169300
tccaaaggat taaccgggcg gtcaccgccc ttgcccagac gccgcaccga agcatcggta 2169360
ttcaacgcca gcaccaacgc gtccccccatc gaacgcgcct gcgccagata agtaacgtgc 2169420
cccctgtgga ggatgtcgaa acagccgttg gtaaacacca gcgggcgcgg caacaacgcc 2169480
aaacgcgccg ccaacgcctc gggcggacag attttcgatt caaaatcagg gacagaccaa 2169540
gcgtcaacca tcaaagcctc cgacaaaaac cataaaagac agaaaaaccc acatgataca 2169600
gaagcatatg cgaaaggcaa agccggcggc gcggacagta cgcgcaaacg ggaaaagacc 2169660
cgtaccgaaa agtacgggcc tttatctggg gtggctgatg gggctcgaac ccacgacaac 2169720
cggaatcaca atccggggct ctaccaactg agctacagcc accataaaaa cggttttcaa 2169780
tcaaattctt ggcacgcccg acaggaatcg aacctgtaac ccccgactta gaaggtcggt 2169840
gctctatccg gttgagctac gggcgctcat gccgattcgt gctgattgat tggtcggggc 2169900
ggtgggattc gaactcacga ccctctgctc ccaaagcaga tgcgctaacc gggctgcgct 2169960
acgccccgac ttgaagaagc gaactataca actcagggaa agatgcgtca acatttattt 2170020
tcaagacacc aagatgaaaa atatagtttt ttgatttgaa aaaatattta atccgtccaa 2170080
acagccgtat tttatttcag ggcaaattta ttttcggcat cctgctgtaa aaacaaacgg 2170140
aaaatgcgat aattttcagc attttctacc tgtttaacaa aaggacggat atgtcggcac 2170200
aactgatcaa tggtaaagaa gtttcgcaaa aacgcctgca ggcggttgcc gaagcggtgg 2170260
cgcaacgcca acagaacaat ctgcacaccc ttgcctggcc gtggttttgg tcggaggcga 2170320
ccctgccagc gcggtttatg tccgcaacaa gaaaactgcc tgccaaaaat gcggcatcaa 2170380
atcactgtct tacgagctgc ccgaatcaac atcgcaggaa gaactgctgg cactggtcga 2170440
ccgcctgaat gccgattccg aagtggacgg tattctggtt cagctaccgc tgccgaagca 2170500
cctcgacagc caggcggttt tggaacgtat ttcgccggat aaggacgtgg acggcttcca 2170560

tccttacaat gtcggcaggc tggcggtcaa aatgccgctg atgcgcccgt gtacgcccaa 2170620
gggcgtgatg acgcttttgg aagcttacgg cattgatccg aaggggaaaa aagcggtcgt 2170680
ggtcggcgcg tcgaatatcg tcggccgccc gcaggctttg gaactgctgc tggcgcgcgc 2170740
aacggtaacg gtctgccaca gcgcaaccga aaatctgaca gacgaggttg ccggagccga 2170800
tattttggtg gtcggcgtag gcattccgaa ctttgtcaaa ggcgaatgga tcaaacctgg 2170860
cgcggtcgtt attgatgtgg gcatcaaccg tttggacgat ggcagcctgt gcggcgacgt 2170920
ggaatttgaa acggcaaaag aacgggcggc gatgattacg cccgttcccg gcggcgtggg 2170980
tccgatgacg attgccacat tgatggaaaa caccctgcac gcggcttcac tgcacgatgc 2171040
ttgagcggtt ctgaagataa aaatgccgtc tgaaaggctt tcagacggca ttttgccgtg 2171100
tccgtttatt tgggcagctt gacgacaacc gtatccgcca gtatgtcgta aagcgtgcgg 2171160
cggtcgcgtt tgaccataaa gagcaggaca aagttggcaa ggaatgccag caggttgatg 2171220
gcgttttctc cgttgtcacc tactgcaaga ccgataacgg cggcaataat ggcaaccaaa 2171280
accgaccatg cgatttcgcg taccaaaacc gtgccgacaa aaccgggatt gcggccgtcg 2171340
gttttcaaca cacggattct catgattttc ttacccaatg actgcccgtc ccggctcata 2171400
tagtagattt ggatgacggt gtacgccaaa atgcctgcca gtcctaccca aaaggaagtc 2171460
atgcccaaaa gcagcccgaa tatttcttcg ccgctgccaa tcctgccttc attcttgatg 2171520
gcgaaagcaa tcagtccggc aaacggcacc aacaaaacca aaaaggtaaa caattggttc 2171580
agcagcgcgg caagtatccg gtcgcctgca ccggcaattc cgacttcaat ttcctgcccg 2171640
ttgcggttgt cggatgccgc gtcggtgtag tcgttttttt cttccatatc cgttcctgat 2171700
aattgttctt aactgacccc gattctaccg ccacgacacc gaaaacgcca atacttaaag 2171760
aaatcccgat aaagaacttt acattttccc aatacggcgt taaaacgctt cctttacgcc 2171820
atacataatt ttattaacga tttttcctca aggagcaaca caatgaaagt aggtttcgtc 2171880
ggctggcgcg gtatggtcgg ttcggttttg atgcagcgta tgaaagaaga aaacgacttc 2171940
gcccacattc ctgaagcgtt tttctttacc acttccaacg tcggcggcgc agcccctgat 2172000
ttcggtcagg cggctaaaac attattagat gccaacaatg ttgccgaact cgccaaaatg 2172060
gacatcatcg ttacctgcca aggcggcgat tacaccaaat ccgtcttcca agccctgcgc 2172120
gacagcggct ggaacggcta ctggattgac gcggcgtcct cactgcgcat gaaagacgac 2172180
gcgattatcg tcctcgaccc tgtcaaccgc gatgtcctcg acaacggtct caaaaacggc 2172240
gtgaaaaact acattggcgg caactgcacc gtttccctga tgctgatggc tttgggcggc 2172300
ctgttccaaa acgatttggt cgaatgggca accagcatga cctaccaagc cgcttccggc 2172360
gcgggcgcga aaaacatgcg cgaactcatc agcggtatgg gcgcggttca cgcccaagtg 2172420
gcggacgcgc ttgccgatcc tgccggctcg attctcgaca tcgaccgcaa agtatccgat 2172480
ttcctgcgca gcgaagacta tccgaaagcc aacttcggcg taccgctcgc cggcagcctg 2172540
attccgtgga ttgacgtgga tttgggcaac ggccagtcca aagaagaatg gaaaggcggc 2172600
gtggaaacca acaaaatcct cggccgcagc gacaatccaa ccgtgattga cggcctgtgc 2172660
gtccgcgtcg gcgcgatgcg ctgccacagc caagccatca ctctgaagtt gaaaaaagac 2172720
ctgcctgttt ccgaaatcga aacgattttg gcaggcgcga atgactgggt gaaagtcatc 2172780
cccaatgaaa aagaagccag catccacgag ctgactcctg ccaaagttac cggcacgctg 2172840
tccgtccctg tcggacgcat ccgcaaactg ggcatgggcg gcgaatacat cagcgcgttc 2172900
accgtcggcg accaactttt gtggggcgct gccgaaccgc tgcgccgcgt attgcgtatc 2172960
gtgttgggca gcctgtgagc cctgtttgaa tggaaatgcc gtctgaagcc tgtttcagac 2173020
ggcattttcc ttgcaaccct gccggataac gccctgccgg cactgccgac gtaaaaaata 2173080
aaggattcca tttccggcgg tatgcggcag cccgacttta tccgaacctg atgcgcctgc 2173140
acgtcaatga aaacagcccg attgcggact tcctgctaca gccgaaattc cgataaggca 2173200
agcgttcacg ccagcaacat ttcctgcatc agcttcatac ccactgcca gccgccgagc 2173260
atgccgttca aactgcccga atgcggggaa accaacaggc gggcgttcca caaatccgcc 2173320
tgtttttgcg cccaaccgtg cggcacgccg ccgtgttcgg gtacaaccaa tgcggcacgg 2173380
cagggacagc ggacgcgttg gaaagcgtgt tccgcatcgt cgggaaaaat atcgggacgc 2173440
tgcggtacaa ggatgatgtt ggcaattttc ttccgtgtca ggatgtctgc ctgatacagc 2173500
cacgccaaaa atgcggccgc gcccgcaccg tgtgcgacaa cggcgacgta tttgccgcgt 2173560
atgcgttcaa atgccgtctg aagccctgcc tgccattccc ctatgctttg accggccgac 2173620
gcttcggaca tctgcacgac gggataactg atcgcccaac ggtctatcca catctgatcc 2173680
tctccggcat cgcgtatcag ccaaagcgtc aaatcttcga gttcaaaacc ctgcataccg 2173740
ccccgcctat ttcagcaggt cccggagggt aaaggcgatg agcagcgaag cgggtacgct 2173800
caatatggcg cagacggtca ggcaggcaaa aatattcacc acccgccgcc agcctttcca 2173860
acccaaacat ttggacgcaa tcagcaggca gggcaggcaa atcagcagcc acaccaacgc 2173920
ccatatcggg tttgccttgg tcggcgcaag ccagccttgc atccgcgaca acataaatat 2173980
cgcccacacc aacatgggca ggataaacgc agcgacgacc catgccgcgc ctattcctgt 2174040
ttttccgtcc acattccaat catatttacc caaaacctta ttcggcagca tagtcatact 2174100
ccacgaccag cggcgcatgg tcagaaaatt tttcatcttt ataaacgtgt gcggacacgg 2174160
ctttggcagc aagttcgggc gtaaccatct gataatcgat gcgccacccg acatctttcg 2174220

catacgcctg ccctcggttg ctccaccaag tgtagcccgg cacatcggga taaagcgtgc 2174280
gccacatatc cgtccaaccg agcttgtgga taaccttgcc tatccactcg cgctcttcag 2174340
gcaggaaacc tgaatttttc tggttgcctt tccagttttt caggtcgatg ttttggtggg 2174400
cgatgttcca gtcgccgcag acgacaatgt cgcgcccttc gtttttcatc gcttcgagca 2174460
tagggtaaaa cgcatcaagg aaacggtatt tcacctgctg gcgttcttcc gcgctgctgc 2174520
cgctgggcaa ataaagcgag ataacgctca acctgccgaa atcgcaacgc acaaaccgcc 2174580
cttccctgtc gaattcttca atgcccatac cgatttgcac attgtcgggt ttgcgtttgc 2174640
tgtacaccgc cacgccgctg taaccgcgct tctcggcgca atgccaatga ccgtgcatcc 2174700
cgtgcggatt tttcatatcg gcagacaaat cagcctcctg cgctttgagt tcctgcacgc 2174760
agacaatgtc cgcgcccgat gcggcgatgt attcgtaaaa accttttttg taggcggagc 2174820
ggatgccgtt gacgttggcg gaaatgattt taagcataat aaaaataagt tctcacaata 2174880
aaaatgccgt ctgaacaaaa aagggcaaaa tgcggcacat ttaccctttt cgatggattt 2174940
taaccgcgcc gccaagtcgt gccgccggcg ttgtcttcca aaatgatttt gtgttcgttc 2175000
agaaggtcgc ggatgcggtc ggattccgcc cagtttttat cggcgcgcgc ctgtttccgc 2175060
cgggcgatca agtcttcgat ttcttcgttg gagagaccgt ctgaagccgc gccgccttgc 2175120
aggaactcgg tcggatcgcg ttgcagcagt ccgatgatgc cgcccaaggc tttcagacgg 2175180
cctgccagtt gcgcgtcatt ggttttgttc acttcgccgg caagttcgaa caacaccgcc 2175240
accgctttca ccgtatcaaa atcatcattc atcgcaacat aaaagcggcg cgtgtagtca 2175300
tcgccggctt cagacggcat cggatcggcg ggcggcgtat ttttcaaagt cgtatacaaa 2175360
cgcgtcaacg cgccttttgc atcatccaaa tgcgcgtcgg aatagttcaa cgggctgcgg 2175420
tagtgggcgc gcaggatgaa gaagcgcacg acttccggat cgtattgttt caacacttcg 2175480
cggatggtga agaagttgcc cagcgatttg gacatctttt cgccgtccac gcggataaag 2175540
ccgttgtgca gccagtattt gacgtggctg gcgatgcttt gcccgtggtg ggtttgcgcg 2175600
tgatgatgac cgcaggtatg ccccgtcgcg ccgacgcttt gggcaatttc gttttcgtgg 2175660
tgcggaaact gcaaatccgc gccgccgccg tggatgtcga aggtatcgcc gaacaggttt 2175720
tcactcatgg cagagcattc aatgtgccaa cccggacggc cgttgcccca cgggctttcc 2175780
cacgccggtt cgcctgcttt ggcggctttc cacaacacaa aatcaagcgg atcgcgtttg 2175840
aaaccgtcca cttccacgcg ttcgcccgca cgcaggtcgt ccaacgattt gcccgacaat 2175900
tgtccgtaag cggcaaactc gcgcacggcg tagtaaacgt cgccatttgc ggcaggatat 2175960
gccttgccgt tttgaatcag ggtttcaatc atggcaatca tttgcggaat gttttccgtt 2176020
gccttcggct caatatccgg acgcaacacg cccaaagcat cggcatcttc gtgcatggct 2176080
tggatgaaac gcgcagtcag tttgccgatg gtctcgccgt tttcagccgc gcgggcaatg 2176140
attttatcgt cgatgtcggt gatgttgcgt acataagtga gcggatagcc gcactcgcgc 2176200
aaccaacggg caatcatgtc gaacaccacc atcacgcggg cgtgtcccaa atggcagtaa 2176260
tcgtaaacgg tcataccgca gacgtacata cgcacgtttt cagggtcgat gggggaaaag 2176320
ggttcttttt gacgggttag ggtgttgtag atggtggtca tgggattatg gattaatctt 2176380
tgttgctcgg atgataattt ctgttctgtt cctgtagata cggaccaagg aacattacgt 2176440
agttgcggat tattaatatg gctgatattt gtgaaaattg gttctgcata acagtttgca 2176500
aaattttttg taaattctga taatttaaac ttatctttta ataagtttgc taaatctgat 2176560
gacgagggat aaagtttact tcttatacta ggcatttcaa tatgaaggac tatttttatt 2176620
tcgttacaat ctaaagccaa gcgagaaaaa tctttttctt cctgtttttc tgctttaaat 2176680
ttagcagaaa ccaatcctgc caatgaatct cgaatttttc ttgcgattaa atatggtagg 2176740
tcagaaagtt tttcatctat tgattgggca tttggctcaa gcccaagtcg gtaataatct 2176800
ttaatttcga ttagccaaag tgtcgattca tgaagggcta ttatatctac acctgagctg 2176860
ccattatcgt catctacact ttgatttatc ccgttctttc ccttttcatt tgtatcaatt 2176920
ttattacgta aattacaact gttctgaaaa attttataat gttcccattc gtcatacttg 2176980
gtaacgtaat aatcttcagg aaaagcaaag gttaatctct tttctgtgat tgtagtcata 2177040
gcttaacctc aaatattcag atacctgtct gcctgcataa tgttttcatc taacaatatc 2177100
aatgtgttca aatcattaat actgttccct tgctccactt ttgttccatc atcggaagca 2177160
atcaacgaga aaaaacgtac aggtaaatcc gtgttatttt caagcttcaa aagttccaat 2177220
tctctcaata agaataaaga gtgtgttgca ataaaaacct gaataccctg ttgagataaa 2177280
gaccaaataa tacgggcagc cactttgatc aatttaggat tcagattagc ttccggttca 2177340
tcccaaaata gatagccttt atccagcaat gcccctgttg cgattaaccg ggcaatcatg 2177400
acaaatttcc gcaaaccctc tgctaccaaa ggtgcttcaa tcttaccgcc cgtatttgtc 2177460
agcgatagat aaaaccttcc ttgttcttca gatacttttc cgcccatcgc gttctcaata 2177520
ggttcgagca attctcgaat ttttgtttct ctggggcctt tggcaagcgg gtgatttaat 2177580
tgcatacagg tatcaaacca agtttcttcg aaagggatgc tttggttttg atacaaagaa 2177640
gtgaaccaag ggcaaagtgt aattaattcg cggctgggta agaagatagg tgtcggagta 2177700
tattcatttt ctttcaatcc gatgctttga acattgactt gcgatgatga gttactggaa 2177760
aaattcagac tactatgcgt agtgccgttt tgcagtttta aaacgatttc cgtacgcccg 2177820
cgcccctgca aacgtttgct caacctaccc aaggaatcgg gacggaaaac attcagtaat 2177880

ttatcggcaa aactttttttg caattctgtt ttcagtaatc tgtttttggt gttagatgtt 2177940
acttctagca ggctgtataa aatttttaac aaatgtgttt tgccacaacc gttttcggca 2178000
acaataacat tgagattttc agaaaattca aaagtatcgt ttggaagaac ggtaaagttt 2178060
gtcaactcaa gcgactggat atattggtta gatgacattt ttaatccatt tcaatcttgc 2178120
tttaaaattg tttcaaacaa ccttttgtag aacaaatatc gtctgaaacc ctttcttttt 2178180
tcactccggc ttaaacacgc ctgtatccgt tttaggctgc tgttcgataa tttcaacatt 2178240
tgccgctgct ttctccgctt ctgcttttttc agcttcgata cgtttttttct cggtcaggta 2178300
ttggttgatt tggtgtacca attcctgcgt gccttggtgg gtcagcgcac tgatttggaa 2178360
gaggcgcggg gtttccatgt caaattggaa acggtcgtcg ggtttggggt agtcccagcc 2178420
gacggcttcg aggaaggcgg cagtgcgcgt ttgggcttct tcttcgtcaa gcatatcgag 2178480
tttgttcagt accagccagc gcggtttgcc gtagagttct tcgtcgtatt tgcgtaattc 2178540
gttgatgatg gcgagtgctt cttcggcggg gttgacggtt tcgtcgaagg gcgccaaatc 2178600
gacgacgtgc agcagcaggc cggtacgtga taagtgtttg aggaaacgat ggccgaggcc 2178660
tgcgccttct gccgcgcctt caatcaggcc ggggatgtcg gccatcacga agctgtggtt 2178720
ttcgtcgatg cgtaccacgc ctaagtttgg atgcagggtg gtgaaggggt agttggcgat 2178780
tttggggcgt gcggcggata cggcggtaat cagggtggat ttgccggcgt tgggcatacc 2178840
caataagccg acatcggcga ggactttaag ttcgagttgc agggaacggg cttcgccttc 2178900
ttcgccgggg gtggattgtt tcggggcgcg gttgacggac gatttgaagt ggatgttgcc 2178960
caagccgcct ttgccgcctt tggcgaggca gacgcgctgt ccgtgataag tgaggtcggc 2179020
aacggtttcg ccggtgtcga ggtcgcggat aagggtgccg acgggcattt tgaggacgat 2179080
gtcgtccgca cctgcgccgt aacggtcgga accgtggcct ttttcgccgt ttttggcttg 2179140
gtagcgttta acgaagcggt attcgacgag ggtgttggtg ttttcgtcgg cttctgccca 2179200
gacgctgccg cctttgccgc cgtcgccgcc gtccgggccg ccgcgcggta cgaatttttc 2179260
gcggcggaaa ctggttgcgc cattaccgcc tttgcctgcg gcgacttcga tttttgcttc 2179320
gtcgatgaat ttcattcaat gctcttgttt gttggtttca aatggggggt tcagacggat 2179380
taccgtgtgt tttgatgccg tccgaacaga atttcggacg ctattataag ggataagcgg 2179440
tatttcaaca cgccgtaccc aaactatttg ttccgcccat cttaatgaat ttttaagcaa 2179500
atcttcagcc tgcaaacaaa attatgtcca acttctttgg tacaatcgcg cctttttgac 2179560
attccgaccc gacggaatgt ccgttcaaac cgttacatat aataagtttt ttatgaacac 2179620
aaaccaacct gccgtttacg acccgttgac acgcgcgctg cactggctga ccgttgccgg 2179680
cttcatcggc attctgacca ccattgtcct gtggacgatt tatagtggat taacaaaaat 2179740
caggacaagg cgacgaagcc gcagacagta caaatagtac ggcaaggcga ggcaacgccg 2179800
tactggtttt tgttaatcca ctatacgaag aggcggaatg ggtgggcagc ctgttcggcc 2179860
tgcacaaatc tttcggtttc cttacgctga cggtgattac attgcgcatc gtgtgggcgg 2179920
ttgccaaccg cgccaagcgt ccgcaaagcg actgcaaggc tgcggcggca ggccacggca 2179980
ttctgtatct gctcatgctt gctgttcccg ttatcggcat gatccgccaa tacggcagcg 2180040
gccgcggccc gttgaaagtg ttcggcgttg aagtgatgca agggttcgcc ggaaaaaatc 2180100
gagtggatgg caaacttggg caacacgttc cacggcaatt tgggcttgct gctgtttgcc 2180160
gccgtcgccg gacacgtcgc catggtcgtc gcccaccgtg ttcagggtag agatgttctg 2180220
tgccgcatga cgggtcgtgt ccgctgattc cgttcacact atggtgccgg ctcgtccggc 2180280
actatttgtt tttccaagac agagccagat cgtacaaagc tttctttccc tcgcccgtga 2180340
ttttggcagc aagctccgcc gcctgtttgg tcggcagctc ggctgtgagg attttcatga 2180400
tgttttgcgc ggactcggac aagccttcgt gtttttcatc ctgcgccgga taaagcacca 2180460
acaccatctc gccgcgcgat tggttgccgt cggcagacaa tgccgtctga atttccccaa 2180520
ccgtgccgct taagaacgtt tcaaacgttt tcgtaatttc gcgcgccagc attaatcggc 2180580
gttcggggaa cagttccgcc atatcggcaa gcgtcgcacc gatgcggtgc ggcgtttcaa 2180640
acatgacgat aggaaacgcc gcccgcaccc atttggcaaa cagtttcctg cgttctcccg 2180700
atttcggcgg tacaaaaccg ttgaaataaa aatcggatcc ttccacaccg gccacgctca 2180760
aagccgccat caccgcgctt gcgcccacga cgggaacgac tttaaacccg gcctcacgca 2180820
cgcggcgggc gagtttcgcg cccgggtcgc acacggccgg cgtacccgca tcggaaacct 2180880
gtgccacaac catgccgtct gaaagatagc cgacaatctt gtccgccatc tgccgttcgt 2180940
tgtgttcgcg cacactgacg agtttgccct gaatgccgta cgcgctcaaa agctgtgcgg 2181000
taacgcgcgt gtcttcggca cagatgatgt ccgccttttg caataccgcc aaagcgcgca 2181060
gggtaatgtc cgccaaattg ccgatgggcg tggcaaccac gtataatgtc cctccgacga 2181120
cgctgtcgga ggctttctgc aaatgtttct gaaacataag aatgccgtct gaaaaacaaa 2181180
cattataaag gttaaaccga ttatgcgcct aaaccacaaa cagggcgagg caggggaaga 2181240
tgccgcgctt gccttcctcc aatcccaagg ctgcacgctg cttgcccgca actggcactg 2181300
cgcctacggc gaaatcgacc tgattgtcaa aaacggcggc atgattctgt ttgttgaagt 2181360
aaaataccgc aaaaatcggc aattcggcgg tgtcgcatac agcatttccc catccaaatt 2181420
attgaaactg caacgaagtg tagagtatta tctgcaacag aacaggttga caaacgtacc 2181480
gtgccgcctc gatgcggtac ttatcgaagg cagccgcccg cccgagtgga tacagaatat 2181540

tacaggttga cgatatgacg acattacaag aacgcgttgc cgcccatttt gccgaaagca 2181600
tccgtgccaa gcaggaagcc ggaaaagtat tggtcgagcc gaccgtacag gctgccgagc 2181660
tgatgctgca atgcctgatg aatgacggca aaatcctggc ctgcggcaac ggcggttcgg 2181720
ctgccgacgc gcaacacttc gccgccgaaa tgaccggccg ttttgaaaaa gaacgcatgg 2181780
aactcgccgc tgtcgcgctg acaacagaca cttccgcgct gacagccatc ggcaacgact 2181840
acggtttcga ccacgtattc agcaaacagg tgcgcgcgct cggacgtgca ggcgatgtat 2181900
tggtcggcat ttccacctcc ggcaattccg ccaacgtcat cgaagccgtc aaagccgcac 2181960
acgaacgcga tatgcacgtc atcgccttga ccggccgcga cggcggcaaa atcgccgcca 2182020
tactcaaaga caccgacgtt ttgctcaacg ttccccatcc gcgcaccgcc cgtattcaag 2182080
aaaaccacat cctgctgata cacgccatgt gcgactgtat cgactccgta ctgctggaag 2182140
gaatgtaacc cttttcagac ggcatggcgc aaagcaatgc cgtctgaaac gcccaagaaa 2182200
ggaagcaccc gatgaaaccc aaaccgcaca ccgtccgcac cctgattgcc gccattttca 2182260
gccttgccct tagcggctgc gtcagcgcag taatcggaag cgccgccgtc ggcgcgaaat 2182320
ccgccgtcga ccgccgaacc accggcgcgc aaaccgacga caacgttatg gcgttgcgta 2182380
tcgaaaccac cgcccgttcc tatctgcgcc aaaacaacca aaccaaaggc tacacgcccc 2182440
aaatctccgt cgtcggctac aaccgccacc tgctgctgct cggacaagtc gccaccgaag 2182500
gcgaaaaaca gttcgtcggt cagattgcac gttccgaaca ggccgccgaa ggcgtgtaca 2182560
actatattac cgtcgcctcc ctgccgcgca ctgccggcga catcgccggc gacacttgga 2182620
acacatccaa agtccgcgcc acgctgttgg gcatcagccc cgccacacag gcgcgcgtca 2182680
aaatcgttac ctacggcaac gtaacctacg ttatgggcat cctcaccccc gaagaacagg 2182740
cgcagattac ccaaaaagtc agcaccaccg tcggcgtaca aaaagtcatc accctctacc 2182800
aaaactacgt ccaacgctga ctcggcaatg ccgtctgaac cgccttcaga cggcattgcc 2182860
cgacacccca aaagcacaat caaaatggca aaaaaaccga acaaaccctt caggctgacc 2182920
cccaaactcc tgatacgcgc cgtattgctc atctgtatcg ccgccatcgg cgcattggca 2182980
ataggcatcg tcagcacatt caacccgaac ggcgacaaaa cccttcaagc cgaaccgcaa 2183040
cacaccgaca gcccccgcga aaccgaattc tggctgccaa acggcgtagt cggacaagat 2183100
gccgcccaac ccgaacacca ccacgccgcc tcatccgaac ccgcacagcc ggacggcaca 2183160
gacgaaagcg gcagcggact gccgtcccct gccgcaccca agaaaaaccg ggtcaaaccg 2183220
caacctgccg acacagctca aaccgacagg cagccggacg acgccggaac acaagctgaa 2183280
aacacactca aagaaacccc cgtactgccc acaaacgtcc cccgtcccga accccgaaaa 2183340
gaaacacccg aaaaacaggc gcagcccaaa gaaacgccca aagaaaacca taccaaaccg 2183400
gacaccccga aaaacacgcc gcccaaaccc cataaagaaa ttctcgacaa cctcttctga 2183460
cccggcacgg caggcacacc cgcaatccaa ggaagcatta tgaacggcat catcatcaaa 2183520
acccccgaag aaatcgaaaa aatgcgcgag ctgggcaaac tcgtcgccga agccctcgac 2183580
tacatcggac aattcgtcaa acccggcgta accaccgacg aaatcgacaa actcgtttac 2183640
gactaccacg tcaacgtcca aggcggctat cccgcccccc tgcactacgg caacccgccc 2183700
taccccaaat cctgctgcac ctccgtcaac cacgtcatct gccacggcat tcccgacgac 2183760
aagccgctca aagaaggcga cattatcaac atcgacctca ccatcaaaaa agacggcttc 2183820
cacggcgact ccagccgtat gtttaccgtc ggcaaagtct cccccatcgc ccaacgcctg 2183880
atcgacgtaa cccacgcctc catgatggcg ggcatagaag ccgtcaaacc cggcgcgaca 2183940
ctgggcgacg taggttacgc ctgccaacag gttgccgaaa acgccggcta ttccgtcgta 2184000
caggaattct gcggacacgg catcgggcgc ggtttccacg aagccccgca agtgttgcac 2184060
tacggaaaaa aaggacaggg ccccgttcta aaaccgggta tgatttttac cgtcgaaccg 2184120
atgatcaacc aaggcaaacg ccacctgcgt atcctcaacg acggctggac ggtggttacc 2184180
aaagaccgct ccctctccgc ccaatgggaa cacgaagtct tggtgaccga aaccggctac 2184240
gaaatcctca ccgtcagccc cgcctccggc aaaccctgaa accggacgta tccgccccat 2184300
aaaaacaaac aatgccgtct gaaagaaacg gcagatatga tatataatat aaaaacaggc 2184360
ttgacccggc acattacgaa aacaaagcaa atcggaattt gccccgcaac cagacaaact 2184420
taaaggaagt tttatgaaaa tatttgaaaa tatagaagat gttaaagcca tccgtaaaaa 2184480
gaccgggctg aaccagatag acttctgggg caaggtcggc gttacccagt ccggaggatc 2184540
gcgctacgaa accggccgca aaatgcccaa acccgtacgc gaactgctcc gcctcgtcca 2184600
tatcgaatgc atcgatttgg cgaaagtcaa caaaaaagat atggaaatcg ccgccctgtt 2184660
gaaaaaacac catcccgacc tgtatgccga gttgtccaaa cagaccaagt ccgaaagaaa 2184720
aaaacaaagt taaaccgcaa cctccggatg cccgacagtt tttcatttcc gaaaaacgca 2184780
aacaatgccg tctgaaacac cggacaggtc gccgtatccc gcctgccgcc cctgcctcaa 2184840
accgccgaac cgcccgaacc cgccttttta caaactttat ccaatttcct gtttatttcg 2184900
ggatacgccg acattagaat gtcaaacagc tcgaaacggg caaactccac atccatccaa 2184960
aggaataaaa atgaaacttc tgaccaccgc aatcctgtct tccgcaatcg cgctcagcag 2185020
tatggctgcc gccgctggca cggacaaccc cactgttgca aaaaaaaccg tcagctacgt 2185080
ctgccagcaa ggtaaaaaag tcaaagtaac ctacggcttc aacaaacagg gtctgaccac 2185140
atacgcttcc gccgtcatca acggcaaacg cgtgcaaatg cctgtcaatt tggacaaatc 2185200

cgacaatgtg gaaacattct acggcaaaga aggcggttat gttttgggta ccggcgtgat 2185260
ggatggcaaa tcctaccgca aacagcccat tatgattacc gcacctgaca accaaatcgt 2185320
cttcaaagac tgttccccac gttaatcagg caacaaaaaa cagcgttttc agaaatgaaa 2185380
acgctgtttt tttgaccgtt ccattattca caaaagggaa aaaacgatta cctgccccgt 2185440
gtatcaaaac ctgccctgcc ggatgaaggg cataaccggc agggacggcg tcaacaccat 2185500
atgggggtac ggcttttctt gaaagattcg gcttaaatat ccaatacttt cgcggtatag 2185560
gcgataattt catccgccct ttcagggttt tcgttcaact tgatgccgta acccggtacc 2185620
agctctttca gacggtcttc ccaagacggg gcgcgctcgg ggaagcattg gtgcatcagc 2185680
cggatcatca gcggcacagc ggtcgatgcg cccggcgacg cgcccagcaa tgcggcgagt 2185740
gagccgtcgg cgtgggcgac aatctccgta ccaaactgga gcacgccgcc tttttcggag 2185800
tcttttttaa tgatttggac gcgttgccct gcggtgatga gttcccagtc gtcggggttt 2185860
gcctcggggt agtattccag cagggaggcg aagcgttctt ctttggtttt acgcaattcg 2185920
cccagcaggt atttggtcag cggcatattc gcccagccgg cgcacagcat aggatagagg 2185980
ttgtccatat ggatggacag cggcaaatcc ataagcgagc cttgcttgag gaagttggaa 2186040
cggaagcctg cgtaagggcc gaacataagg tggcgtttgc cgtccacgtt gcgtgtgtcg 2186100
aggtgcggga cggacatcgg cggcgcgccg acggaagcct gcccgtacac tttggcgttg 2186160
tgttgttcgg cggtttcggg gttgctgttg cggaagaaca ggccggacac ggggaagccg 2186220
ccgtagcctt tgccttcggg gatgccggat ttttgcagca gggtcagcgc gccgccgccc 2186280
gcgccgagga agaggaagcg ggtacggagg gtgagctgcc cgtcggggtt gcgggtatcg 2186340
gcggttttga gcacccacgc gccgtcggat tcgcgtttga tgtcttcgac gtggcggttg 2186400
aactcggttt ttacgccctt gccctgcaaa tatttcacca tttggcgcgt cagccgtccg 2186460
aaatcgacat ccgtaccttc ggcggagtag ttggcggcga cgggttggtt ttcgtcccgg 2186520
ccgcgcatca tcagcggagc ccaatcggaa attttgttcc gatcggtgga aaattccata 2186580
ttttcaaaaa gtttttgggt tttaaacgcg tcataacgtt tttgaagata agaacaatgg 2186640
tcttcattca tcaccaaaga catatgcggc acggcattga tgaaggaatt gtcttccaac 2186700
ttgccttccg cgaccagcgt cgcccaaaac tggcggctga catgaaactg ttcggcaata 2186760
ttgagggcgc gcgccggatc gataatccca tttgcaccca acggcgcata gttcaattcg 2186820
cacagcgcgg aatgccccgt gccggcgttg ttccacgcgt ttgacgattc caacgccaca 2186880
tcttccaagc gttcaatcag ggtgatttcc caagacggtt cgagttcttt gagcaaaacg 2186940
cccaaagtcg cgctcataat gccgccgccc accaagacaa cgtctgtcgc ttcagccatg 2187000
gtttactcct aaaaaacagg catcttctgc ccttatggtt atttgccgta ctacaaacgc 2187060
ctgaatcgca aaagcaggga aaaccggcaa tggtgtgtgt ccgagtatgc tgtttcgggg 2187120
ttggaatgcg ttgcaagcat ggcttccgac accgcttcag ggcttgtaa tatgttatcg 2187180
tgaatgtagt ggattttact gggaaatgca aagtttttct gtcgcccgcc aagtcgggaa 2187240
actgcgaaat gaaaaataaa aatagttatt tatctatata tatcaaattt ttaatagata 2187300
aaaaatcaaa attgtttata tattaatttt taaaagattg tcagcatatt gcgttaagtt 2187360
ttttatagtg gattaacaaa aatcaggaca aggcgacgaa gccgcagaca gtacaaatag 2187420
tacggaaccg attcacttgg tgcttcagca ccttagagaa tcgttctctt tgagctaagg 2187480
cgaggcaacg ctgtactggt ttttgttaat ccactataaa tttgaaaata ctgcctcaca 2187540
cctgcacgcc ataccctgcc aacctgccgg tcaggatttc cctgtttttg caccaatctt 2187600
ccctcagcat actgtacacg accgtatcgc gcacactgcc gtctttacgg agcatatgca 2187660
tacgcagcac gccgtctttt tccgcaccca gccgttcgat ggcacgttgc gaggcaaggt 2187720
tcagaatatc cgtgcgccat cccacgcaac ggcaagccaa aacatcaaat gcggaatcca 2187780
acagcatgat tttgcaacag gtgtttatcc gtgtccgccg tgccgatgcc gcataccatg 2187840
tgaatccgat atccaaacgc ggaatctgcg gttcaaaatg ataatacgcc gttgtcccga 2187900
ccaccctgcc cgcctcttca tcgacaaccg caaacgccaa acgcgttgcc aatgctgtcc 2187960
cgatatagtc tgccacccta tccggatggg gcgcggacgt tacccccagc ttccaaacct 2188020
ccccatcgca aaccgcctcg cgcaaacccg tttcatgatg cacatccaac ggttcgagac 2188080
gaacgccgcc caacgacaag accggcagta ttatcttttc cgacatcctt ttctcccaat 2188140
attccgcctt cagacggcat ttccgcccgg aatgccgtct gaacggctaa aaacacaata 2188200
tccccgcctc cgacacaaaa ccgtccaaag accggtcgtg cgcctcgacc ggcagcctgt 2188260
ccaccaactg gcaggcaaag cccacgccca cggtttttgc ctgcaaacgg tatttcatcg 2188320
ctgaaagcgt cgcatcgtaa tagccgcctg cctgtcccaa gcggtagccc agcctgtcca 2188380
taccgaccac tggcacaagc aggaggttca aatcatgcac acgctttttc cgacctgcaa 2188440
actgagggac atgcagcttc gccctaccgc gcttgcgttc ttgttttact ccatcggcag 2188500
gatacggcgt aaaccacatc cgccgcgaac gcggttcgat ataaggcagg tagagttccg 2188560
caccgcgttt ttgcgccgcg cggacaaagc cgtccaaacg caattccttg cccatcggcc 2188620
aatacacgcc gattttccgc cctttttaa tataacgttt gagcaggtgg ttgattttta 2188680
ccgttgccgc cgcccgcacg tcccgcccca tttgcgaacg ccgcccgcgc aattcgcggc 2188740
gcagggcgcg tttttcctcg ttcctcattt cagacggcct ttcaggattg cggtagaatg 2188800
ttgcgattat aacgattttg ttaacattca aacaggacgc acacaatgtg gcacatcgtc 2188860

gccatcggct atctttttgt tgccgttatg tattccgccg cgcagccgag tattgcgcgc 2188920
gccttgattt atttggtttt ttgggcggtg ctgcccaccg tgttcacggt tttcaccatt 2188980
accgtccgcc gccgcaacca cctgatgagg cagcaggaac aggcggaatc cgaacagcag 2189040
cgcgcacaac ggcaaaaaga cagcggcaca aaaccctgaa tccctttca gacggcatct 2189100
tatccgctat aatccgtcag ttttccattt cggaaacaca ctatttttta aaacttatgc 2189160
ccactttcgc cgaagggtgc ttgacaatag gcgtgaccta tcaagttcta tgcgattgaa 2189220
tgtgtgctct taaccctttc aaggaaataa aatgtctcaa attactatgc gtcagatgat 2189280
tgaagccggt gttcacttcg gccaccaaac ccgtttctgg aacccgaaaa tggcacaata 2189340
catttcggt gcgcgcaaca aaatccatat cgtcaacctg gaaaaaaccc tgccgatgtt 2189400
ccaagacgcg caagaagccg tacgtcgtct ggttgccaac aaaggtacag tattgttcgt 2189460
aggtaccaaa cgccaagccc gcgacatcat ccgcgaagaa gcgacccgcg ccggtatgcc 2189520
tttcgtcgat taccgctggt tgggcggtat gctgaccaac tacaaaaccg ttaagcaatc 2189580
catcaaacgc ctggaagaaa aaaccgcagc cttggaaaat gctgccgaaa gcggtttcag 2189640
caaaaaagaa attctggaaa tgcaacgcga tgttgaaaaa ctggaacgtt ctttgggcgg 2189700
tatcaaaaac atgaaaggcc tgcctgacgc gattttcgtt atcgataccg gctaccaaaa 2189760
aggtactctg gttgaagctg aaaaattggg catccctgtt atcgccgtag tcgataccaa 2189820
caacagcccc gacggcgtga aatacgttat ccccggcaac gacgactccg ccaaagccat 2189880
ccgcctgtac tgccgcggca tcgctgacgc agttttggaa ggcaaaaacc aagcgctgca 2189940
agaaaccgta gccgctgccc aagaagccgc tgccgagtaa tccggcaaac cgaagagggg 2190000
cgttatgccc cttttctcaa atatgccgtc tgaacgtccg ttcgcggcac acgattcccg 2190060
aatgcggaaa atcctttccg tatttcccaa aaatctagga gattcaaaat ggcagaaatt 2190120
actgcaaaaa tggttgccga cctgcgcgcc gctaccggcc tgggcatgat ggaatgcaaa 2190180
aaagccttgg ttgaagccga aggcaacttc gacaaagccg aagaaatcct gcgtatcaaa 2190240
tccggtgcga aagccggtaa actggccggc cgtaccgctg ccgaaggcgt attggcttac 2190300
gcgatcaacg gcaatgtcgg cgcattggtc gaagtaaact gcgaaaccga cttcgttgct 2190360
aaagacgcgg gcttcgtaga atttgccaac ttcgttgcga aaactgctgc cgagaaaaaa 2190420
ccggcttctg ttgaagaact gagcgaactg gttgaagcag aacgcaaagc catcatcgcc 2190480
aaattgggcg agaatatgtc tgtccgtcgc ttccaagtga tcgacactgc caaccaactg 2190540
gttgcctaca tccacggcgc attggcgacc gaaggcgtat tggttgagta caaaggctct 2190600
gaagacgtag cacgcaaaat cggtatgcat attgttgccg ctaaaccaca atgcgtaagc 2190660
gaagccgaag tagatgccga aaccgttgaa aaagaacgcc acatctacac cgagcaagcc 2190720
atcgcttccg gcaaacctgc cgacatcgcc gctaaaatgg ttgaaggccg catccgtaaa 2190780
ttcttggctg aaatcactct gaacggccaa gcattcgtga tgaaccccga tcaaactgtt 2190840
gcccaattct ctaaagaaaa cggcactgaa gtgatcagct tcgtacgcta caaagtaggc 2190900
gatggtattg agaaaaaagc cgtcgattac gcagccgaag ttgctgccgc tgctaaagtg 2190960
taaggcactt atgaaaaaga aagcacctgg attccaaacg aatcagggtg cttttttttg 2191020
agaaaaccgt ttacggtacc tattttaaga cgaccgaata ttcagaccgt cttaaaacaa 2191080
aacaataata aaccgacaca ccctatcatt aatattccga ccgttggaaa ttcagacggc 2191140
ccaactccga ccgacgacat tcaagaaagc aaggtatcca tgacacagca aatcaaatac 2191200
aaacgcgtat tactgaaact ctccggcgaa tccctgatgg gttccgatcc gttcggcatc 2191260
aatcacgata ccatcgttca aactgtcggc gaaattgccg aagtcgttaa aatgggcgtg 2191320
caagtcggta ttgttgtcgg cggcggcaat attttccggg gcgtatccgc ccaagcaggc 2191380
agcatggatc gcgccaccgc cgactacatg ggcatgatgg cgaccgtgat gaacgcgttg 2191440
gcactcaaag acgcatttga aactttaggc atcaaagcgc gcgtacaatc cgcactgtct 2191500
atgcagcaaa tcgctgaaac ctacgcccgc cccaaagcca tccaatattt ggaagaaggc 2191560
aaagtcgtga tttttgccgc cggtaccggt aacccgttct tcacgaccga cactgccgcc 2191620
gcattgcgcg gtgcggaaat gaactgcgac gtgatgctca aagccaccaa cgtcgacggt 2191680
gtgtacaccg cagacccgaa aaaagacccg tccgccacgc gctacgaaac cattactttt 2191740
gacgaagcct tgttgaaaaa cctcaaagtc atggacgcga ccgctttcgc cctctgccgc 2191800
gaacgcaagc tcaatattgt cgtcttcggc atcgccaaag aaggctcgct caaacgcgtc 2191860
attaccggcg aagacgaggg aacgctggtt cactgctgat tgaccatagt gtcggcagat 2191920
atagtcgcat atgggcttca gacagccatt tattatatgg agattatagt ggattaaatt 2191980
taaaccagta cggcgttgcc tcgccttgcc gtactggttt aaatttaatc cactatattt 2192040
acaattttga tacaatttgt ttttcatcaa aggagaaaat ctatgcaagc acggctgctg 2192100
atacctattc ttttttcagt ttttatttta tccgcctgcg ggacactgac aggtattcca 2192160
tcgcatggcg gaggtaaacg ctttgcggtc gaacaagaac ttgtggccgc ttctgccaga 2192220
gctgccgtta aagacatgga tttacaggca ttacacggac gaaaagttgc attgtacatt 2192280
gccactatgg gcgaccaagg ttcaggcagt ttgacagggg ggtcgctact ccattgatgc 2192340
actgattcgt ggcgaataca taaacagccc tgccgtccgt accgattaca cctatccacg 2192400
ttacgaaacc accgctgaaa caacatcagg cggtttgaca ggtttaacca cttctttatc 2192460
tacacttaat gcccctgcac tctctcgcac ccaatcagac ggtagcggaa gtaaaagcag 2192520

```
tctgggctta aatattggcg ggatggggga ttatcgaaat gaaaccttga cgactaaccc 2192580
gcgcgacact gcctttcttt cccacttggt acagaccgta tttttcctgc gcggcataga 2192640
cgttgtttct cctgccaatg ccgatacaga tgtgtttatt aacatcgacg tattcggaac 2192700
gatacgcaac agaaccgaaa tgcacctata caatgccgaa acactgaaag cccaaacaaa 2192760
actggaatat ttcgcagtag acagaaccaa taaaaaattg ctcatcaaac caaaaaccaa 2192820
tgcgtttgaa gctgcctata aagaaaatta cgcattgtgg atggggccgt ataaagtaag 2192880
caaaggaatt aaaccgacgg aaggattaat ggtcgatttc tccgatatcc gaccatacgg 2192940
caatcatacg ggtaactccg ccccatccgt agaggctgat aacagtcatg aggggtatgg 2193000
atacagcgat gaagtagtgc gacaacatag acaaggacaa ccttgattca cactaccata 2193060
accgcttgct accaaggaaa acaaaatgaa tttgcctatt caaaaattca tgatgctgtt 2193120
tgcagcagca atatcgttgc tgcaaatccc cattagtcat gcgaacggtt tggatgcccg 2193180
tttgcgcgat gatatgcagg caaaacacta cgaaccgggt ggtaaatacc atctgtttgg 2193240
taatgctcgc ggcagtgtta aaaagcgggt ttacgccgtc cagacatttg atgcaactgc 2193300
ggtcagtcct gtactgccta ttacacacga acggacaggg tttgaaggtg ttatcggtta 2193360
tgaaacccat ttttcagggc acggacatga agtacacagt ccgttcgatc atcatgattc 2193420
aaaaagcact tctgatttca gcggcggtgt agacggcggt tttactgttt accaacttca 2193480
tcgaacaggg tcggaaatcc atccggagga tggatatgac gggccgcaag gcagcgatta 2193540
tccgcccccc ggaggagcaa gggatatata cagctattat gtcaaaggaa cttcaacaaa 2193600
aacaaagact aatattgtcc ctcaagcccc attttcagac cgttggctaa aagaaaatgc 2193660
cggtgccgcc tctggttttt tcagccgtgc ggatgaagca ggaaaactga tatgggaaag 2193720
cgaccccaat aaaaattggt gggctaaccg tatggatgat gttcgcggca tcgtccaagg 2193780
tgcggttaat cctttttttaa tgggttttca aggagtaggg attggggcaa ttacagacag 2193840
tgcagtaagc ccggtcacag atacagccgc gcagcagact ctacaaggta ttaatgattt 2193900
aggaaaatta agtccggaag cacaacttgc tgccgcgagc ctattacagg acagtgcttt 2193960
tgcggtaaaa gacggtatca actctgccaa acaatgggct gatgcccatc caaatataac 2194020
agctactgcc caaactgccc tttccgcagc agaggccgca ggtacggttt ggagaggtaa 2194080
aaaagtagaa cttaacccga ctaaatggga ttgggttaaa aataccggtt ataaaaaacc 2194140
tgctgcccgc catatgcaga ctttagatgg ggagatggca ggtgggaata aacctattaa 2194200
atctttacca aacagtgccg ctgaaaaaag aaaacaaaat tttgagaagt ttaatagtaa 2194260
ctggagttca gcaagttttg attcagtgca caaaacacta actcccaatg cacctggtat 2194320
tttaagtcct gataaagtta aaactcgata cactagttta gatggaaaaa ttacaattat 2194380
aaaagataac gaaaacaact attttagaat ccatgataat tcacgaaaac agtatcttga 2194440
ttcaaatggt aatgctgtga aaaccggtaa tttacaaggt aagcaagcaa aagattattt 2194500
acaacaacaa actcatatca ggaacttaga caaatgaatg aacacaacct gttaattttc 2194560
tgtttaaaag acaatgtttc aattagtgaa tatactgaaa tggttgattg ggcttatgaa 2194620
aacattcaat ctgaaacagt tgtagaaatt acggaaaatc aaattattga atatcaaaat 2194680
cgtggattat gggggcttgt ttctgaaatt accgataatt ggttatttgg accaagtgag 2194740
ggggattggc taatagataa ggaaagtatt ttggctgtaa aagaaaaatt acaaaattca 2194800
gatttttcta cagagccctt agtgaaaaat attattcatg tacttgaata tgctataaag 2194860
aatgaaaaaa cagtaatttt tcatttttga aactaatcta atttttagca gccgtaggtc 2194920
ggattctcga atccgatatt ttccaacagc ggcatttcgg aaacgataga tgcgtcaaat 2194980
atttttgtcg gatacaaata tccgacctac atctctgcgc agcaaacttt acaagatatt 2195040
aatgaattag gaaatttaag tccggaagca caacttgctg ccgcgagcct attacaggac 2195100
agtgcttttg cggtaaaaga cggcatcaat tccgccagac aatgggctga tgcccatccg 2195160
aatataacag caacagccca aactgccctt gccgtagcag aggccgcaac tacggtttgg 2195220
ggcggtaaaa aagtagaact taacccgacc aaatgggatt gggttaaaaa taccggctat 2195280
aaaacacctg ctgttcgcac catgcatact ttggatgggg aaatggccgg tgggaataga 2195340
ccgcctaaat ctataacgtc caacagcaaa gcagatgctt ccacacaacc gtctttacaa 2195400
gcgcaactaa ttggagaaca aattagtagt gggcatgctt ataacaagca tgtcataaga 2195460
caacaagaat ttacggattt aaatatcaat tcaccagcag attttgctcg gcatattgaa 2195520
aatattgtta gccatccaac aaatatgaaa gagttacctc gcggtagaac tgcgtattgg 2195580
gatgataaaa cagggacaat agttatccga gataaaaatt ctgacgatgg aggtacagca 2195640
tttagaccaa catcaggtaa aaaatattat gatgatttat aggaaaaagc catgaatata 2195700
ctatccataa ataatcaaaa ctcaactatt tcactaactc aagatgaagt ttttgtttta 2195760
cgagctatct tgaatgagat atatgcgggc gtatgtgtag attcaagaga atttgaaaat 2195820
gtatctggtg ttagaaaaca tgaagtagat aatttacaac aacagtttgc tggaatttat 2195880
aaaaaaatga caacttaaca acccaaattt ttgtcagagc ctagtgcaaa ttacaactat 2195940
gattctattg tagccgaaat gaaagaaaaa aatcatgggt tggcgacagg gttgatgttg 2196000
ttaatatgcc tgatggagca cctactagta tggataacac gcgtattatg gcagcacgtg 2196060
aagcaggagt aaaagtggaa gcgaatgttc ataattttaa tgaccgatta tcatcaaaag 2196120
agagaatcag gtttaagcat gatggtattg agcctcaaac ttggggagaa gctatccagc 2196180
```

tacgaattag aaagcaagaa acacaaaaag gagttccaga agggtggagc aaaagatttc 2196240
ctaacggaag tatttatgat gtaaaggtac ttaggaaatg ataaaacaaa atagttttgt 2196300
tccgtatcct gaagcaatgc ttcctaaagg atttaaatat ccgcaaagtt atttaaaatt 2196360
agctcaatcc actcatgcca ttaactacga tgaacaatat tcttttcctt ggtggtttga 2196420
aaatgcagaa agcaatatat cagaagtaat tgacatttat tttgaaataa ctggcattcc 2196480
aaacctatta cctttgcta gaaaccaaga gtgggctgcc tgttttgata tttcagataa 2196540
atcaggtaat cctaaaatta tagtagttaa tttagataat acaaaatatt acgagacttt 2196600
tgaaaatttt gatacttggc taaaagaagc tgaaaatgat ggttggtagc aaccgtaggt 2196660
cggattctcg aatccgacat ttttcaacag cggcatttcg gaaacgatag acgcgtcaaa 2196720
tatttttgtc ggatacaaat atccgaccta catctctgcg cagcaaactt tacaagatat 2196780
taatgaatta ggaaatttaa gtccggaagc acaacttgct gccgcgagcc tattacagga 2196840
cagtgctttt gcggtaaaag acggcattaa ttccgccaga caatgggctg atgcccatcc 2196900
gaatataact gcaacagccc aaactgccct ttccgtagca gaagccgcaa ctacggtttg 2196960
gggcggtaaa aaagtaaacc ttaacccgac caaatgggat tgggttaaaa ataccggcta 2197020
taaaacacct gctgcccgcc ctatgcagac tttagatggg gagatggcag gtgggaataa 2197080
gccaccaaaa ccaagtacgc agcaacaccc tacacactct gataacaata tcggcttacc 2197140
tgcctcatat gttaaacctg atacatctat ttctccgaca ggaacaattc aagaccgcat 2197200
cagatggaca aagtccaagt ttcctactga gaaatcttta aatggacatt tcaaagctca 2197260
tggaaaagaa tttggcgata taaccattga agactaccaa aaaatggcgt ctgatttgtt 2197320
atcaaaacag acatcggaca agatattagg ttatcagacg gaacatagac gagtgcgcta 2197380
tgatatcaat aacaatatct atgttttggc caatccaaaa acattcaaaa tcaaaacaat 2197440
gtttaaacca aacttaggaa agaagtatta tgatggagaa ttcaaaaaag acatgggaaa 2197500
ttgacggaga aatatggcta cattgtcctg tttgcggaac tgaagttatg gactatgata 2197560
tctgtgacgt ttgtcagtgg caaaatacag gagaaactaa tatagatggt ggccctaatg 2197620
aaatgacact tgcggaggcg aaagaagctt acgcaaaagg cttaccaatc agataaataa 2197680
gcacctagag aaatcaatga tgacggaatc ccatggttac ctatcaaata atttacctgt 2197740
taaaatcata aatgatatta tttatgcaac ccagttagtc gaagatttgg ttttaggaaa 2197800
aataaaaatt gttgattttt taaaatcata taacaatttt tattgttggc ttggttttga 2197860
tgagttgcct caatctgaga aaataaaatt cctaagctat cttaatatat taagtattca 2197920
taaagaaata caagatgaaa ctgtgaatag ggtttatacc gattgaaaaa tagtagatag 2197980
agattaacat gttaaatgaa atttttgaaa tttattcgag acaaggggaa tctttgatag 2198040
gaattggaat tagagaagcc gcattacccg tccctattgc aatagatata ttaaatttat 2198100
ttatcaatga gagaatactt gtattggggg gagatattta tatcaagaaa gataattatt 2198160
tttatcaaac atatgataat tggtattacg agggaagtaa tttatttaac agtatcgaca 2198220
aagcaatgca ttatttatct caaataaaat tagagaatgc atacgtatct tttgtgttga 2198280
aatttatcta acaaaggaag cacaagaata gatttatagt aaaacatcaa gatgttgaaa 2198340
atgctgggtt ttaatccaac ctacactgac cggctcagat acagccgctc agcagactct 2198400
acaaggtatt aatgatttag gaaatttaag tccggaagca caacttgctg ccgcgagcct 2198460
attacaggac agtgcttttg cggtaaaaaa cggcattaat tccgccagac aatgggctga 2198520
tgcccatccg aatataactg caacagccca aactgccctt tccgtagcag aggccgcagg 2198580
tacggtttgg cgcggtaaaa aagtagaact taacccgact aaatgggatt gggttaaaaa 2198640
taccggctat aaaaaacctg ctgcccgccc tatgcagact gtagacgggg aaatggctgg 2198700
gggaaacaaa tcattaaaaa tagggacaca atctgttgaa aaatcaaccg gtcgtacaat 2198760
acctaataat ttaaaggaac aattagcaat ggaagaagtt aaggcaaacc cacagggcaa 2198820
aactcctgcg agaatacctc ctatgtccga tactaaaaat ggttggttag caaaagacgg 2198880
ttgggttaag cgtgttcaaa acgtaaacaa aattgaaata cattacattg aaaactcaag 2198940
aaccggtgag aaaacagatt ttaagtttaa ggattagtca tgtttttaga tgatgtaaat 2199000
gttttttag atgatttaaa tgttttttta gatgatttaa atactaatcc aatcactgac 2199060
gaatggtata tgtccaattt tgccgataaa catattaaaa ttttggaaag ttacgaagcc 2199120
tttgatattc taaaacaatt tgttgattac atgattgaag aatatgatga aaaatcagaa 2199180
tatgaaatca tggaaatatt gagacaatta aaatatcaag cagataccaa cgaaaaattt 2199240
tatacaaata cacagaaaca gaaaattgta gaattatata aacaagaaat tagtcaggat 2199300
attttaaatg aaatctttag ataaactatc aatatagaag gaaatccttg gaaaaaataa 2199360
aatgataatc gaacacaatg gaaatataca taaaatagcc agaatgactg gaaataaaaa 2199420
taattttta gaaataatcc tatcagatat tcatgaaaac ataaaaatca aaccattaac 2199480
tataaaagta aaaggagaga atgttataaa tatccttcct gaggaagtta gtttttatgt 2199540
aaaacaaggt gttgatttaa tttatgaaaa atataaacgg aaattcttta tctccgaaat 2199600
ttctttttgc caatcagata gccggccttc aagtatctac gcttttctta catttcactt 2199660
gcttgaagat attattaaaa atgaatcccc atccaactac acctgactgg ctaatagcag 2199720
gtatgaaccg tgtattcata tcaatataag attaattacg gcagaatttg atgaaattag 2199780
acaaaaactt atcgtaagat tttatttaga cagagaagta actgatgatg gcaaagaaga 2199840

tatggaaaca gcacgaacag aattacttcc aggaggatat gcttcatctc tggtagtttg 2199900
acagatttga ccgcttcata aacttagaac attaattaat gatgataatg tttatatgat 2199960
tggttctaag gatagcaaaa gcaaattcag aaggaacatg aatggctatt tatgacttaa 2200020
acgaaatagc cgtaggtcgg attctcgaat ccgacatttt ccaacagcgg catttcggaa 2200080
acgatagatg cgtcaaatat ttttgtcgga tacaaatatc cgacctacat ctctgcgcag 2200140
caaactttac aaggtattaa tgatttagga aatttaagtc cgaaagcaca acttgctgcc 2200200
gcaagcgcat tataggacag tacttttgcg gtaaaagacg gtatcaattc cgccagacaa 2200260
tgggctgatg cccatccgaa tataactgca acagcccaaa ctgcccttgc cgtagcagag 2200320
gccgcaggta cggtttggag aggtaaaaaa gtagaactta acccgaccaa ataggattgg 2200380
gttaaaaata acggctataa aacacctgct gcccgcccta tgcagacgtt ggacggtgag 2200440
atggcaggag gaaacaagcc agttgttaaa tctatcagac caactacgcg agatgaatta 2200500
cgtcaagcat tgcaagaaca aggttttaga cgtactggtt cagatgcggc tcaatatgaa 2200560
acatggaaag gtcctgatgg cgtgaaaata gatattcgtc caaatggaga ggttataaga 2200620
acccaaagag tgccgcgaac cgatggtgta cagggaaaat atccgcaacg acaagattat 2200680
gaaggcaatc cattgccaaa taatcatcat cattctggat attttgtcaa atgaaaaaaa 2200740
atatttttca caatgtaagc ctttatgaaa taatcttttc cgataatgga aataccctta 2200800
cattatcttt tacagataca attgaaggta attatttcgg atatatcaaa tgcagtaata 2200860
ttttgaattt taaattagat acaaataatt tcgtagatta tgaggataag gaagatagct 2200920
tgtttccctt gtttataccc gaaatagagc tatataaata ccaattttat agtgaaatta 2200980
ttattgatgt agggattatt ataaaaatat ctgctgaaac aattaatttt gagccactgg 2201040
gaaaatagta actgctttcc cagcagccgt agcaactgta tttttacccg acggggtaaa 2201100
aatacagttg ctacatctct gcgcagcaga ctctacaagg tattaataat tcaggaaaat 2201160
taagcccgga agcacaactt gctgccgcga gcatattaca ggacagtgct tttgcggtaa 2201220
aagacggcat caattccgcc agacaatggg ctgatgccca tccgaatata acagcaacag 2201280
cccaaactgc ccttgccgta gcagaggccg caggtacggt ttggagaggt aaaaaagtag 2201340
aacttaaccc gaccaaatgg gattgggtta aaaataccgg ctataaaaaa cctgctgttc 2201400
gccatatgca gactaaggcg ttaggtacgg tagatgaaat tggcgataca gtacagcagg 2201460
ttgggaaaca ggctagcgga caaaaaacca gcggtggtaa tcctgcgatt gatagcgacc 2201520
cctatagccc gagtagtgtg gcagctcgca tagaagccgg taaggcgcgc agtgatttac 2201580
aaatcaaaga cattttgagc aatactactc aaaggagtaa aacaaaaggt cccgctgttc 2201640
agtatgataa agtgggggat tacaatgacg cactaaatga ttttaatagt ctgaatgttc 2201700
gaaatgtaca aacacgtcct aatggaacga taacgggcaa tttacctgat gggcgtgcgg 2201760
ttaatgctcg taatgatagt agtggtggag aaccaacact tgaaataaca attagtaata 2201820
accgaaaaat aaaaatcaga tatggaaata cacgataaat tatgaaatta aaaagcttag 2201880
atttcccaac tggctatttc tattttgata atgcagcaat aaactctgat aaagtagaag 2201940
ttatagcagt tggttataga aatacggata aaaccataaa aatttttatt gaagatgtta 2202000
ttcattttag ggttgttgat gaatcgtatt ttatagatac ttttatggat ttaatttcgg 2202060
aagatgcaga tagagctttg cttcatgaaa atggtggtca atcttttttt gaacttcttg 2202120
atgagtgtta tgcggaatgg atattgaaag aaagttattt tcctttgaat agagaattct 2202180
ttaaatacta tatttttatg tttgagcaaa cattcataga aataattggt tctagtgcaa 2202240
cgtattcaat tattgagggc tagcgtaaga tgagtaataa gttgcctatc tttctttcag 2202300
gcagcctgaa aataaaacta cccaagttga tggtgtacct gtatcagtga agggaaattt 2202360
tgttgatggt aaatttcgca ttggtacggc aacaatgaaa tcattttaaa ttgagctaga 2202420
aatgaaccta gaaaattatg aaaacatttt aataaaatta ctttttttatc ataacaactt 2202480
agtaaatgaa tattcttatt ttattgaaaa taaaaattta tttaaaatag tatctcgaac 2202540
gaaaacgagt aagggctttt ttttcactat agaaaaacca ttaaattttc taagcaaaaa 2202600
aacttatttt gagtttaatt ttaaatattt acactcaggg aaagaacgct ttggttcgtt 2202660
tatgtgctgg ataaatacta atttaatgga atttgagggg gtttttttta acgacctgct 2202720
ccctgataat atgataataa ataacttttt tgaaataaat gattaacgat acaccaataa 2202780
aaattggtgg ggtaaccgta tggatgatat tcgcggcatc atccaaggtg cggttaatcc 2202840
tttaatttac aaggtaagca agcaaaagat tatttacaac aacaaactca tatcaggaac 2202900
ttagacaaat gaatgaacac aacctgttaa ttttctgttt aaaagacaat gtttcaatta 2202960
gtgaatatac tgaaatgatt gattgggctt ataaaaacat tcaatctgaa acagttgtag 2203020
aaattacgga aaatcaaatt attgaatatc aaaatcgtgg attatggaga cttgtttctg 2203080
aaattaccga taattggtta tttggaccaa gtgaggggga ttggctaata gataaggaaa 2203140
gtattttggc tgtaaaagaa aaattacaaa attcagattt ttctacagag cccttagtga 2203200
aaaatattat tcatgtactt gaatatgcta taaaaaatga aaaaacagta atttttcatt 2203260
tttgagacta atccaatttt tagtaatatt gatgcagagc aagcagcatt agatgccgca 2203320
aacatgggga gaagctattc aatttagaat taaaaaacaa attgaaaatg aactagcacc 2203380
accaaattgg tctacccagt ttcctaatgg tagtatttat gatcctaagg taacgaaatg 2203440
attattcaaa atgaatttaa tttatatcct agtaatatgc ttcctgaaag gttttgttat 2203500

cctgaaaagt atgttcgtat ctctaacgat acatctttaa taccttatat tcagccacat 2203560
aattttcact ggtggtttga gaattatgga acagaagggg cagaagtagc ttatatattt 2203620
agaaattcta tcctgcctga tttaaatctt atcccattcg ctagtaatgg agaatgggaa 2203680
gcttattttg atggtaatga tgtaacagga aattctaggg ttattgtcat taatttagat 2203740
aatatagaaa accatgaatt ttttaatagt tttgaagatt ggcttgaatt agcaattaag 2203800
gatacttggt aagcagctat ctataaagag atgaggctgc cctggacaac taggataaac 2203860
tcgattttac taattgtttt aaaatggaac aagaactttt atttcactgt tgttaaaacg 2203920
ccattcgcac tcctttaaat acagctcaaa atgcgctttg ggaatgccgt taaacttgcg 2203980
taaatgacgt tttgcttgat tccaaaagtt ctcagttcca ttaatatggt tttgtcgttc 2204040
ggcaaaatgt gtgctgtgat tgatacggaa atggctaaat tcgcccgcat ccaatacatc 2204100
atagccacga taacaaaatg agtttatttt gtttataccg tcttagacga ctttctctca 2204160
tagggataat tctaacttaa tttgaatttc cctagtgatc tagggcagcc cctaaattaa 2204220
taaagcagca caactccttt tgccgatgtt ccggactgtc aaacgactgt tcctcatgcc 2204280
acatctccat caaggtacgg ataacccgct ccgccttacc gttggtctgc ggacaagcaa 2204340
atcgggcaag cctccaacca atcccattat cataacaagc tgcaccgaaa gcatgttgga 2204400
cggctcttta tattacctat cattgtcaga gtaaacgtac tcaatcaggt acaagcaggg 2204460
gtcggacaga tgttcggtca gaaacttggc agcactgtct gcggttttgt ccggcaaaat 2204520
ggcagagtat aaaaatcgtc aatagcgaca aacaggtaat ctcgtttatc agcggccttc 2204580
tgtcctttga gcaacaacaa ccgatcggta tcaggatgca caaaacctcc cggggacaac 2204640
ctgcctttta cggctttaag tgcacggtaa atagtgacgc ggctgactta gtggcagcat 2204700
actggggagg tgagtgtttt tgtgtatatt tttattttgg tattccctta gaaatactgt 2204760
aaacaacgct accggacggc ctgcagggct tcgcgcacgc ttgctttgag ttctgcgccg 2204820
aagcgtctgc ccaagattct gccgaaatcg tccttcggag tgtaatccac cacatcgggg 2204880
gctttgacca cgtctcgcgc cacgctgtaa atattgccga gtccgtccac cagcccgact 2204940
ttcagcgcat ccgcgcctgt gtacacgcga ccgctgaaca cgtcgggata ttgtcggaat 2205000
ttgaggcggc cgccgcgtcc ggttttgacg gctttgatga actcgccgtg tatgccggtc 2205060
agcatttctt cccagatttt tgactgttcg ggcgtttcgg gcgaaaacgg atcgcccatg 2205120
cctttgttgc tgcctgcaat tttaaccctg cgtttcacgc cgattttttc catcaggccg 2205180
gtcgcgtcga aactgctgcc gataacgccg atgctgccga cgatgctgga cgggtcggca 2205240
tagattttgt ccgccgccgc cgcgatgtag tagcagccgg acgcgcacat atcttccgcc 2205300
acgagataaa cgggaatgcc ggggtgctgc gccttcagac ggcgtatttc ttcaaaagcg 2205360
gtgttggaca cgacgggcga accgccgggg ctgttggcgc ggatgacgat ggcttttgcc 2205420
tgcgggtttt tgtaggcggc ctccataccg tctttgagtt ttttgacctg gtcttctaca 2205480
ccgttgccga tttcgccgta cagattgacg actgcggtat gcggcgtgtt gcccgccaac 2205540
tgcaatgcgg cttcgtcttt tcggaaaatg cctgcaatca gggcaaccag aatcagggtg 2205600
ctgacggcgc gccagatgtt tttccacatc cgctccctgc gcctgtcctg ataggcggac 2205660
aacagcactt cgcgcatgat gtcgcgctcc cataaggttt cccccgcatt ttttgcttcg 2205720
ggtgcttcgt tttctcttct gattcggtat tgcatggttt tccttaaata ttgtccgatt 2205780
tgggcaaacg gttttcagtt tacccgattt ttcagctctg ctcccaatcc gtccaagctg 2205840
tgcaacactt ccgcccacgc cgcgtccaaa aggttgacgg cttctccttc ggctttgatg 2205900
ccgaactcaa tgtgcggttt gacctgcgtg ccgtctgaat gcgtccaacc gacgctgggc 2205960
aggctgtacg aacgcacgcc gggataagtt tgctcgatat gctccataag cggcgtaatg 2206020
cgcgattcgg gctgctcaaa cacatacacg ctgcggctgc cgcgttcggt ttggttgaag 2206080
cggtcggcgt aataagtttc caatacccat tccgccatcg ggtgcgccat cacaggaaag 2206140
ccggggaaga aataatgctc gcggatagaa aatccggcga tgttgttaaa cggattgggc 2206200
accaattccg cgccttcggg aaaatctgcc attttcaggc gttgggcgtg ttccggcgaa 2206260
tcaagcggct cgccgcgttt ctgggttatg ccttcgataa acttggcggc ttcagaatgg 2206320
cggacgacgg gcaaatccaa agcagcggct gcggcttggc gggtgtggtc gtcgggcgtg 2206380
gcgccgatac cgccggtaac gaaagttggc atgccgtctg aaaagctgcg gcgcagttgc 2206440
ctgaccagca aatcgggttc gtcgggcagg tattgcacct gattgagctt cagccctttg 2206500
gattcgagca gggatttgaa aaaggcgaaa tgcttgtctt ggctgctgcc gtgtaagatt 2206560
tcgtcgccga tgatgatgag gttgaacgcg ttcatagatg gtttctttac cgatgccgtc 2206620
tgaaaatgtc gatggtgctg tgatttgttc cctctcccgt gggagagggt tagggagagg 2206680
gtcgagcttg cgtttttcag gcagcgtttg cttaaggcct gctgtctgta ccctctcccc 2206740
aaccctcccc cgcaggggag ggagtcaggt tgaggatggc gtaaagaccg tctgaaaaga 2206800
ttttcagcga aacgggcaaa gcttcttttc agacagcctt aacggctgac aatgggttat 2206860
atttataaga taatgaactc ctttttttcaa gtccgaagga tacccttatg agccaaaacc 2206920
ataccattct gcaatccctc cccgtcggtc agaaagtcgg catcgccttc tccggcggtc 2206980
ttgatacctc tgccgcgctg ttgtggatga aactcaaagg cgcgctgcct tatgcctaca 2207040
ctgccaacct cggccagccc gacgaagacg actacaacgc cattcccaaa aaagcgatgg 2207100
aatacggtgc ggaaaacgcc cgcttaatcg actgccgcgc gcagttggca cacgaaggca 2207160

tcgccgccat ccaatgcggc gcgtttcacg tttccaccgg cggcatcgcc tatttcaaca 2207220
ccacgcctct gggccgcgcc gtaaccggca ctatgcttgt ttccgcaatg aaagaagacg 2207280
atgtgaatat ttggggcgac ggcagcacct acaaaggcaa cgacatcgag cgtttctacc 2207340
gctacggttt gctcaccaat cccgcgctga aaatctacaa accctggctc gatcagcaat 2207400
ttatcgacga actcggcggc cgtcacgaaa tgagcgaatt tctgattgcc aacggcttca 2207460
actacaaaat gtcggtggaa aaagcctact ccaccgattc caatatgttg ggtgccaccc 2207520
acgaagccaa agacttggaa tttttgaact cgggcatcaa aatcgtcaaa cccattatgg 2207580
gcgttgcctt ttgggacgaa aacgtcgaag tcagcccgga agaagtcagc gtacgctttg 2207640
aagaaggcgt gccggttgca ctaaacggca aagaatacgc cgatcccgtc gaactcttcc 2207700
tcgaagccaa ccgcatcggc ggccgccacg gcttgggtat gagcgaccaa atcgaaaacc 2207760
gcatcatcga agccaaatcg cgcggcatct acgaagcccc gggtatggcg ttgttccaca 2207820
tcgcctacga gcgtttggtc accggcatcc acaacgaaga caccatcgaa caataccgca 2207880
tcaacggcct gcgcctcggc cgcctgctct accaaggccg ctggttcgac agccaagccc 2207940
tgatgttgcg cgaaaccgca caacgctggg ttgccaaagc cgttaccggc gaagttaccc 2208000
tcgaactgcg gcgcggcaac gactactcaa ttctgaacac cgaatcgccc aacctgacct 2208060
accaacctga acgcctgagt atggaaaaag tcgaagacgc tgcgttcact ccgctcgacc 2208120
gcatcggaca gctcacgatg cgcaacctcg acatcaccga cacccgcgtc aaactgggta 2208180
tctactcgca aagcggtttg ctctcgctgg gcgaaggttc ggtattgccg cagttgggca 2208240
ataagcaata aggtttgctg ttttacatca ttagcaactt aaggggtcgt ctgaaaagat 2208300
gatcccttat gttaaaagga atcctatgaa agaatacaaa gtcatcattt atcaggaaag 2208360
cctgttgtcc agcctgtttt tcggcgcggc aaaggtcaac cccatcaaat tcagcgagtt 2208420
cctcaataaa caaacccccg aaggctggcg ggttgtaacg atggaaaaag atttgcgccg 2208480
tatgctgctg tttttcaaac gcgaggccta cgtcgtcatt ttggagcggg atcgtgttta 2208540
agctcggcgt ttatacctgt ctcggactgt ttgccggctg ggtgctgctg ctgatcgtgc 2208600
aactctggtt ttcttttctc gaagcggaat tgttcttcaa aatcacactg actatggcgg 2208660
ggctgtttgt catcatcctc gccgccttac tggtatgcgg tcagtatttt tccgaaaaga 2208720
aaatgaaaga cgacgggttt atcaactgat gcggacttga accggacccg cgacccaaac 2208780
atcacaatgc cgtctgaacg ccctcgcttc agacggcatc aacatcaatc ctgctctttt 2208840
ttgccggcaa acacgccgaa tccgcccttt tccgcatctg tcgggcgata gctgtatttt 2208900
cccgccactt cctcgccggc cgggccgtaa aactttccgg aaacatcccc gctgccattt 2208960
tccgtccaag tcccccttaaa gccgtttcca tcgatggcgg ctttgaattt ttgcgtaccc 2209020
atatgcaaat catcgccgct gtcgataatg ccgtccacag atttgctgcc gaaatcgact 2209080
tttgcggcaa acctgcccct ggtcgggtac ggacggccgt tttccgtatg gaaatgcagt 2209140
acttcgccgt tgtacacggc cgcgcccgca agcatttcgc cttttgccgg ttcgccttga 2209200
acacgaaggg catacgatcc gccgggcaat ttttccgccc cgtaagtcag ataccggtaa 2209260
ttcccttcgg gcgcgaagat attgccggaa tgccccgtca ggctgaccgc ttccccatcg 2209320
acaatcagcg tatccgcctg attgacggga atcagcggca tctcggccgg aagcgaccgc 2209380
ctcgaccgtg cagaacgcct aaatcgcgca aatgaagtgg gtttaggttt ataaaagata 2209440
atatattgat tgattccctt catctgcaca ctatcggcaa ccaaaccgac aaatttatca 2209500
ttcttcccat ctttcttgta attacttatt ttgtctgcat cacttaattt ttcaaattct 2209560
gattttagct gtacttcttc atccaagaaa ttattgccac tacaagaatc gcctttacag 2209620
tgggtcaacg ttatattttg cgacggcccg tcaatcaaaa cgccattagc caaatcaacc 2209680
cttccaaaat tgctaccgcc attcgcaggt gcagggtttg acgcggggat gggatctgaa 2209740
gaaccggcgg cttgattgtt tccggcttga tttgcacctt gggcagccgt attgccggca 2209800
ttttgcccgc ctgccgacgg atcgtccccc tgcattccgt ccgccgcatt tgccatatcc 2209860
ggttggtttg ccggctgaga cgattccccg gcatccgttg cttgattttc catatttccg 2209920
gcaagcatat tcggatccgg ggtgtgattc ggtgtcgaac tatctgtacc ggcggcattt 2209980
tgcggcatat catttgtgc cacctcgtct tcatttttgg gattatccgc tgttaccgca 2210040
ccgccattgc ctgtattttc ttccgaaacc gccgccatat cttgactgcc ttgtgcggat 2210100
ggcgcgccct gtccttgaga acctgcctgt ggcgcatctt cctttgcctc tgtctctttt 2210160
tcagaaacaa caggggcggc aggttttgac agcgtgtccg ccgacttgac atcgggcgat 2210220
ccgccaccgc cgcccccgca ggctgaaagg gcaaaaatac aagccattgc gattacgctg 2210280
cgtttaaaca tcatcatctc cttcatcgta tttccttttt ggtttaaacc ccgccacttg 2210340
gacatccgtc cttcggggcg gtggaatcag ctttatttgg gaagagcgca acctttccaa 2210400
atcagggcga cacatagggc tgtgctttat gtgccgccct gtgtgttgaa acatattcaa 2210460
taaatattgt ttccgccgta tgcctataaa attgtaaaaa tatgccgtct gaacgccaaa 2210520
cgggcttcag acggcatagc ttggtttatt ccgcccggtt cctctgtcgg cccaaatcgg 2210580
cggcagcggt aaacaaaacg tcggtcgaag agttcagcgc agtttccgcc gaatcctgaa 2210640
tcacgccgat aatgaagccg acggcaacca cctgcatggc cacatcgtta tcgataccga 2210700
acaggctgca cgccaaagga atcagcagca acgagccacc ggccacaccg gatgcaccgc 2210760
acgcgctaac ggtagccacc aggctcagca gcagggcagt ggcgaagtca accgtaatgc 2210820

cttgcgtgtg cgccgcagcc atcgccaaaa cggtaatggt gattgccgca ccggccatat 2210880
tgatggttgc acccaatgga atggagatgg agtaagtgtc ttcgtgcaaa cccagctttt 2210940
tcgccaatgc catgttcaca gggatattgg cggcggaaga acgggtaaag aaggcataaa 2211000
cgccactttc acgcaggcag gtaaacacca gcgggaaagg gttgcggcgg attttccacc 2211060
acacgatggc gggattgacc gccagcgcga taaacgccat acagcccaac agcactgcaa 2211120
gcagcttcgc gtaccccgcc agcgcgccga aacccgtctc cgcgattgtg gacgacacca 2211180
gcccgaaaat gcccaaaggg gcaaaacgga taatccattt cacgacggtg gaaaccgctt 2211240
ccgccaaatc ggcaacgacc tgccgcgtaa cgtccgaacc gtgattccgc aacgccgcgc 2211300
ccaaaaccaa agcccaagcc aaaatgccga tatagttggc attggcaatc gcgttaatcg 2211360
ggttggcgac caggttcatc agcagcgatt tcaatacttc cacaatgccg gaaggcggcg 2211420
cggcggacac atcgcccgcg cccgccaaaa caatgtgcgt cgggaaaacc ataccggcga 2211480
tgacggcggt cagggctgcg gaaaacgtac cgatgaggta aaggacgata atcggcctga 2211540
tatgcgcctt gttgcctttt tggtgctgcg cgattgtggc cgccaccaaa ataaatacca 2211600
aaaccggcgc gaccgctttg agcgcaccga caaacaggct gccgaacaag cctgccgcca 2211660
agcccagttg cggggaaacc gaaccgatta cgatgcccaa cgccaaaccg gcggcaatct 2211720
gcctgaccag gctgacgcgg ccgatcgcat gaaataagga tttgccgaac gccataattc 2211780
ttccttatgt tgtgatatgt taaaaaatgt tgtattttaa aagaaaactc attctctgtg 2211840
ttttttttta ttttccggct gtattttata gtggattaac aaaaatcagg acaaggcgac 2211900
gaagccgcag acagtacaaa cagtacggaa ccgattcact tggtgcttca gcaccttaga 2211960
gaatcgttct ctttgagcta aggcgaggca acgccgtact ggtttttgtt aatccactat 2212020
aaggttgcgt tgatttgccc tatgcagtag tgccggacag gctttgcttt atcattcggc 2212080
gcgacggttt aatttattga acgaaaataa atttatttaa tcctgcctat tttccgacac 2212140
tattccgaaa cgcagcctgt tttccatatg cggattagaa acaaaatacc ttaaaacaag 2212200
cagatacatt tccggcgggc cgcaacctcc gaaataccgg cggcagtatg ccgtctgaag 2212260
cgtcccgccc cgtccgaaca gtgttaaaat cgaaagccgc cacaccgatg cacgacaccc 2212320
gtaccatgat gatcaaaccg accgccctgc tcctgccggc tttatttttc tttccgcacg 2212380
catacgcgcc tgccgccgac ctttccgaaa acaaggcggc gggtttcgca ttgttcaaaa 2212440
acaaaagccc cgacaccgaa tcagtcaaat taaaacccaa attccccgtc ctcatcgaca 2212500
cgcaggacag tgaaatcaaa gatatggtcg aagaacacct gccgctcatc acgcagcagc 2212560
aggaagaagt attggacaag gaacagacgg gcttcctcgc cgaagaagcg ccggacaacg 2212620
ttaaaacgat gctccgcagc aaaggctatt tcagcagcaa agtcagcctg acggaaaaag 2212680
acggagctta tacggtacac atcacaccgg gcccgcgcac caaaatcgcc aacgtcggcg 2212740
tcgccatcct cggcgacatc ctttcagacg gcaacctcgc cgaatactac cgcaacgcgc 2212800
tggaaaactg gcagcagccg gtaggcagcg atttcgatca ggacagttgg gaaaacagca 2212860
aaacttccgt cctcggcgcg gtaacgcgca aagcctaccc gcttgccaag ctcggcaata 2212920
cgcaggcggc cgtcaacccc gataccgcca ccgccgattt gaacgtcgtc gtggacagcg 2212980
gccgccccat cgccttcggc gactttgaaa tcaccggcac acagcgttac cccgaacaaa 2213040
tcgtctccgg ccttgcgcgt ttccagcccg gtatgccgta cgacctcgac ctgctgctcg 2213100
acttccaaca ggcgctcgaa caaaacgggc attattccgg cgcgtccgta caagccgact 2213160
tcgaccgcct ccaaggcgac cgcgtccccg tcaaagtcag cgtaaccgag gtcaaacgcc 2213220
acaaactcga aaccggcatc cgcctcgatt cggaatacgg tttgggcggc aaaatcgcct 2213280
acgactatta caacctcttc aacaaaggct atatcggttc ggtcgtctgg gatatggaca 2213340
aatacgaaac cacgcttgcc gccggcatca gccagccgcg caactatcgg ggcaactact 2213400
ggacaagcaa cgtttcctac aaccgttcga ccacccaaaa cctcgaaaaa cgcgccttct 2213460
ccggcggcgt ctggtatgtg cgcgaccgcg cgggcatcga tgccaggctg ggggcggaat 2213520
ttctcgcaga aggccggaaa atccccggct cggctgtcga tttgggcaac agccacgcca 2213580
cgatgctgac cgcctcttgg aaacgccagc tgctcaacaa cgtgctgcat cccgaaaacg 2213640
gccattacct cgacggcaaa atcggtacga ctttgggcac attcctgtcc tccaccgcgc 2213700
tgatccgcac ctctgcccgt gcaggttatt tcttcacgcc cgaaaacaaa aaactcggca 2213760
cgttcatcat acgcggacaa gcgggttaca ccgttgcccg cgacaatgcc gacgttcctt 2213820
cagggctgat gttccgcagc ggcggcgcgt cttccgtgcg cggttacgaa ctcgacagca 2213880
tcggacttgc cggcccgaac ggatcggtcc tgcccgaacg cgccctcctg gtgggcagcc 2213940
tggaatacca actgccgttt acgcgcaccc tttccggcgc ggtgttccac gatatgggcg 2214000
atgccgccgc caatttcaaa cgtatgaagc tgaaacacgg ttcgggactg ggcgtgcgct 2214060
ggttcagccc gcttgcgccg ttttccttcg acatcgccta cgggcacagc gataagaaaa 2214120
tccgctggca catcagcttg ggaacgcgct tctaaaccga tatggccact tcagacggca 2214180
ttgcagcaaa ccattttgaa acagacatta tgaccgatac cgcaccgaca gataccgatc 2214240
cgaccgaaaa cggcacgcgc aaaatgccgt ctgaacaccg ccctaccccg ccggcaaaaa 2214300
aacgccgccc gttgctgaag ctgtcggcgg cactgctgtc tgtcctgatt ttggcagtat 2214360
gtttcctcgg ctggctcgcc ggtacggaag caggtttgcg cttcgggctg taccaaatcc 2214420
cgtcttggtt cggcgtaaac atttcctccc aaaacctcaa aggcacgctg ctcgacggct 2214480

tcgacggcga caactggtcg atagaaaccg agggggcaga ccttaaaatc agccgcttcc 2214540
gcttcgcgtg gaaaccgtcc gaactgatgc gccgcagcct gcacattacc gaaatttccg 2214600
ccggcgacat cgccatcgtt accaaaccga ctccgcctaa agaagaacgc ccgccgctca 2214660
gccttcccga cagcatagac ctgcctgccg ccgtctatct cgaccgcttc gagacgggca 2214720
aaatcagcat gggcaaagcc tttgacaaac aaaccgtcta tctcgaacgg ctggatgctt 2214780
cataccgtta cgaccgcaaa ggacaccgcc ttgacctgaa ggccgccgac acgccgtgga 2214840
gcagttcgtc gggggcggcc tcggtcggct tgaaaaaacc gtttgccctc gataccgcca 2214900
tttacaccaa aggcggactc gaaggcaaaa ccatacacag tacggctcgg ctgagcggca 2214960
gcctgaagga tgtgcgcgcc gaactggcga tcgacggcgg caatatccgc ctctcgggaa 2215020
aatccgtcat ccacccgttt gccgaatcat tggataaaac attggaagaa gtactggtca 2215080
aagggttcaa catcaatccg gccgccttcg tgccttccct gcccgatgcc ggactgaatt 2215140
tcgacctgac cgccatcccg tcgttttcag acggcatcgc gctggaaggt tcgctcgatt 2215200
tggaaaacac caaagccggc tttgccgacc gcaacggcat ccccgtccgt caggttttag 2215260
gcggctttgt catccggcag gacggcacgg tgcatatcgg caatacgtcc gccgccctgc 2215320
tcggacgggg cggcatcagg ctgtcgggca aaatcgacac cgaaaaagac atcctcgatt 2215380
taaatatagg catcaactcc gtcggcgcgg aagacgtact gcaaaccgcg ttcaaaggca 2215440
ggttggacgg cagcatcggc atcggtggca cgaccgcctc gcccaaaatc tcttggcaac 2215500
tcggcatcgg cacggcgcgc acggacggca gcctcgccat tgcaagcgac ccagcaaacg 2215560
gacagcggaa actggtgctc gacaccgtca acatcgccgc cgggcaaggc agcctgaccg 2215620
cgcaaggcta tctcgagctg tttaaagacc gcctgctcaa gctggacatc cgttcccgcg 2215680
cattcgaccc ttcgcgcatc gatccgcaac ttccggcagg caatatcaac ggctcaataa 2215740
accttgccgg cgaactggca aaagagaaat tcacaggcaa aatgcggttt ttacccggca 2215800
cgttcaacgg cgtaccgatt gccggcagtg ccgacattgt ttacgagtcc cgccaccttc 2215860
cgcgtgccgc cgtcgatttg cggctggggc ggaacattat taaaacagac ggcggcttcg 2215920
gcaaaaaagg cgaccggctt aacctcaata tcaccgcacc cgatttatcc cgtttcggtt 2215980
tcggactcgc ggggtcttta aatgtacgcg gacacctttc cggtgatttg gacggcggca 2216040
tccgaacctt tgaaaccgac ctttccggcg cggcgcgcaa cctgcacatc ggcaaggcgg 2216100
cagacatccg ttcgctcgat ttcacgctca aggttcgccc cgacacaagc cgcccgatac 2216160
gcgccgacat caaaggcagc cgcctttcgc tgtcgggcgg agcggcggtt gtcgataccg 2216220
ccgacctgat gctggacggc acgggcgtgc agcaccgcat ccgcacacac gccgccatga 2216280
cgctggatgg caaaccgttc aaattcgatt tggacgcttc aggcggcatc aacagggaac 2216340
ttacccgatg gaaaggcagc atcggcatcc tcgacatcgg cggcgcattc aacctcaagc 2216400
tgcaaaaccg tatgacgctc gaagccggtg cggaacgcgt ggcggcaagt gcggcaaatt 2216460
ggcaggcaat gggcggcagc ctcaacctgc aacacttttc ttgggataaa aaaaccggca 2216520
tatcggcaaa aggcggcgca cacggtctgc atatcgccga gttgcacaat ttcttcaaac 2216580
cgcccttcga acacaatctg gttttaaacg gcgactggga tgtcgcctac gggcgcaacg 2216640
cgcgcggcta cctcaatatc agccggcaaa gcggcgatgc cgtattgccc ggcgggcagg 2216700
ctttgggttt gaacgcattt tccctgaaaa cgcgctttca aaacgaccgc atcggaatcc 2216760
tgcttgacgg cggcgcgcgt ttcgggcgga ttaacgccga tttgggcatc gccaacgcct 2216820
tcggcggcaa tatggcaaat gcaccgctcg gcggcaggat taccgcctcc cttcccgact 2216880
tgggcgcatt gaagcccttt ctgcccgccg ccgcgcaaaa cattaccggc agcctgaatg 2216940
ccgccgcgca aatcggcgga cgggtaggct ctccgtccgt caatgccgcc gtcaacggca 2217000
gcagcaacta cgggaaaatc aacggcaaca tcaccgtcgg gcaaagccgc tctttcgata 2217060
ccgcgccttt gggcggcagg ctcaacctga ccgttgccga tgccgaagta ttccgcaact 2217120
tcctaccggt cggacaaacc gtcaaaggca gcctgaatgc cgccgtaacc ctcggcggca 2217180
gcatcgccga tccgcacttg ggcggcagca tcaacggcga caaactctat taccgcaacc 2217240
aaacccaagg catcatcttg gacaacggct cgctgcgttc gcatatcgcg ggcaggaaat 2217300
gggtaatcga cagcctgaaa ttccggcacg aagggacggc ggaactctcc ggtacggtcg 2217360
gtatggaaaa cagcggaccc gatgtcgata tcggcgcggt gttcgacaaa taccgcatcc 2217420
tgtcccgccc caaccgccgc ctgacggttt ccggcaacac ccgcctgcgc tattcgccgc 2217480
aaaaaggcat atccgttacc gggatgatta aaacggatca ggggctgttc ggttcgcaaa 2217540
aatcctcgat gccgtccgtc ggcgacgatg tcgtcgtatt aggcgaagtc aaaaaagagg 2217600
cggcggcacc gctccccgtc aatatgaacc tgactttaga cctcaatgac ggcatccgct 2217660
tcgccggcta cggcgcggac gttaccatag gcggcaaact gaccctgacc gcccaatcgg 2217720
gcggaagcgt acggggcgtg ggcacggtcc gcgtcatcaa agggcgttat aaggcatacg 2217780
ggcaggattt ggacattacc aaaggcacgg tctcctttgt cggcccgctc aacgatccca 2217840
acctcaacat ccgcgccgaa cgccgccttt cccccgtcgg tgcgggcgtg gaaatattgg 2217900
gcagcctcaa cagcccgcgc attacgctga cggcaaacga accgatgagt gaaaaagaca 2217960
agctctcttg gctcatcctc aaccgcgccg gcagcggcag cagcggcgac aatgccgccc 2218020
tgtctgcagc cgcaggtgcg ctgcttgccg ggcaaatcaa cgaccgcatc gggctggtgg 2218080
atgatttggg ctttaccagc aagcgcagcc gcaacgcgca aaccggcgaa ctcaaccccg 2218140

ccgaacaggt gctgaccgtc ggcaaacaac tgaccggcaa actctacatc ggctacgaat 2218200
acagcatctc cagcgcggaa cagtccgtca aactgattta ccggctgacc cgcgccatac 2218260
aggcggttgc ccgtatcggc agccgttcgt cgggcggcga gctgacatac accatacgtt 2218320
tcgaccgctt ctccggttcg gacaaaaaag actccgccgg aaacggcaaa ggaaaataag 2218380
cggttttcag acggcgcgcc gccaaaccgg acatttgaaa acctgctttt ccaccgtccg 2218440
ccgccgccgt ccgcctgcaa gggaacagaa tcgatatagt gaattaacaa aaatcaggat 2218500
aaggcgacga agccgcagac agtacaaata gtacggaacc gattcactcg gtgcttgagc 2218560
accttagaga atcgttctct ttgagccaag gcgaggcaac gccgtaccgg tttttgttaa 2218620
tccgctatat tccgccatct ctaagattta cagcgataca caggtaattt aaggaatgcc 2218680
cgaaccgtca ttcccgccac tttccgtcat tcccgcgaaa gcgggaatct aggacgcagg 2218740
gttaagaaaa cctacatccc gtcattcccg cgaaagtggg aatctagaaa tgaaaagcaa 2218800
caggcattta tcggaaataa ctgaaaccga acagactaga ttcccgcctg cgcgggaatg 2218860
acggctgcag atgcccgacg gtctttatag cggattaaca aaaatcagga taaggcgacg 2218920
aagccgcaga cagtacaaat agtacggaac cgattcactc ggtgcttgag caccttagag 2218980
aatcgttctc tttgagccaa ggcgaggcaa cgccgtaccg gtttttgtta atccgctata 2219040
ttccgccatc tctaagattt acagcgatac acaggtaatt taaggaatgc ccgaaccgtc 2219100
attcccgcca ctttccgtca ttcccgcaaa agcgggaatc tagaatctcg gactttcaga 2219160
taatctttga atattgctgt tgttctaagg tctagattcc cgcctgcgcg ggaatgacga 2219220
ttcataagtt tcccgaaatt ccaacataac cgaaacctga cagtaaccgt agcaactgaa 2219280
ccgtcattcc cacgaaagtg ggaatctaga aatgaaaagc aacaggcatt tatcggaaat 2219340
aactgaaacc gaacagacta gattcccgcc tgcgcgggaa tgacggctgc agatgcccga 2219400
cggtctttat agcggattaa caaaaatcag gacaaggcgg cgaagccgca gacagtacaa 2219460
atagtacgga accgattcac tcggtgcttc agcaccttag agaatcgttc tctttgagct 2219520
aaggcgaggc aacgccgtac tggtttttgt taatcctcta taatgcgccc ttcggcgtgg 2219580
cggatatata aggaagtgat tttccatcta agtaaaaacc gccctatcgg ataagccctt 2219640
aacagaaaag gctttacccg cgccgtatcg gaacacatcc tctaaaatac aatccgttga 2219700
attgaaaaaa atataaaaac atccgcccgc gaaaaacggc agcgcgtcgt ttgacaaaga 2219760
atgaaaatat cggttaaaaa ccgattttca tacaaaaaac accgctgccg tccgcatccg 2219820
tttcagacgg tattgagaga aaatctttta ggagaacctt tatgtcccgg catcccgccc 2219880
ccaccggaga aaaaacattc ttcggccacc ccttccagct ttccaccctc ttccatatcg 2219940
aattgtggga acgtttttca ttttacggaa tgcagggcat cctgctgatt tacctctact 2220000
acaccgccga caaaggcggc ttgggcatag acaaaaccct cgccggcggc attgtcggcg 2220060
catacagcgg cagcgtgtac ctgtccacca ttttgggggc gtggtttgcc gaccgagtat 2220120
ggggtgcgga aaaaaccctc ttcctctcgg gcatcgtcgt gatgctcgga cacatcgtcc 2220180
ttgccgccgc cccgggcctg tacggccttt taatcgggct gatattcatc gcattgggca 2220240
gcggcggcgt gaaatctacg gccagttcta tggtgggcgc attatacgaa caggacgaaa 2220300
tgcgcccgct gcgcgatgcg ggatttttcca ttttctacat cgccatcaac atcggcggct 2220360
tcctaggccc gctgctgacc ggcctactgc aggaaaacat cggtttccat tatggtttcg 2220420
gcgcggcggc ggtcggtatg gcattcggct tgtggcgtta ttccctggga cgtaaaaacc 2220480
tgccccaccc caccgtcccc catccgcttt caaaaggaca gggcaaaact gcggccgccg 2220540
tcggcatcgc cctcatcgcc gcacttgcaa ccgccatcaa aaccgggctt gtcaacctcg 2220600
acaatttctc cggcatccta ttatctaccg tcatccttgc cgtcatcgcc tatttcgccc 2220660
gcctgctgac caaccccccgc gtcagttccg acaacaaacg gcacatcatc gcctacatcc 2220720
cgcttttcct gaccatctgt atgttttggg ccgtctggtt tcagatttac accgtggcaa 2220780
ccgtctattt cgacgaaacc gtcaaccgca ccatcggttc gtttaccgtg cccgtcgctt 2220840
ggaaagattc tatgcaaagc ctgtgggtca tcctgttttc cggactgatg gcggcaatgt 2220900
ggacaaaaat ggggcgcaaa cagcccaaaa ccccgctgaa attcgctatg gcggtatttg 2220960
ttaccggcgc gtcgtttttg ggattcgtcc cctttatttc ctccggtacg ccgatgccta 2221020
ttgcggtttt cgcactgatc gtcctcgcca tcacgatagg cgaactgatg atttccccga 2221080
ttgcgctgtc catctccacc aaaatcgcac cgcctttatt caaaacccaa atggtcgccc 2221140
ttaatttcct tgccttttca ttaggcttca ctttgggcgg cgtattgttt gaaaaaggct 2221200
atcaggcggg cgacgaaatc ggcttctatc ggctgctgtt ctacatcggc gcagccacag 2221260
gcttcctgct gctcctgctc gtccccaaat tgaacaaaat gctcgaaggc acagactaag 2221320
tcccgccccg atgccgtctg aacccttcag acggcatttt tccgcataat gaaaccaaac 2221380
cgtttccacc cgacaggaca ggctcccgcc caaccggaag gcagcctgcc gattgtcatt 2221440
tgaataacgc aagggaaagc cgttgatttc cgtttgtatg gaaacagttt ggtttcattg 2221500
gaaaaaggca ttttgtccga ctaaattagt gctgcatcaa cgaaatatat agtggattaa 2221560
caaaaatcag gacaaggcga cgaagccgca gacagtacaa atagtacgga accgattcac 2221620
ttggtgcttg agcaccttag agaatcgttc tctttgagct aaggcgaggc aacgccgtac 2221680
cggtttttgt taatccacta taaaaacaca acctaaataa aaatgccgtc tgaaccatat 2221740
ttcaggtttc agacgacatt tgcgtgtcgg atgcacaccg gacaggcggt aagccgggtt 2221800

```
ctgtctcgga cagtcattcc tctaggcata ccgttaccgg tatgctcaag caacctaccc 2221860
gaacgctcgg cgggcagcgt cattgcgttc tgtttggtct tgctccgaat ggggtttggc 2221920
ctgccgcata ttgttaccaa atgcgcggtg cgcccttacc gcaccttttc acccttacct 2221980
gtgctgccaa agcagccatc ggcggttttg ctttctgttc cactttccgt cgcgttaccg 2222040
cgcccggccg ttaaccggca ttctaccctg cggagcccgg actttcctcc ccgtatgcct 2222100
tacgcgatac gcggcgactg tctgcccgtc ccgtgtgcgg cgcggattat aacacgaaac 2222160
acaaaaatgc cgtctgaaac ggtacaggtt tcagacggca tacagcctaa actacacgcc 2222220
ctgtttcagg ctggcttcga tgaagccgtc caagtcgcca tccaatacgg ctttggtgtt 2222280
gccgacttcg tagcctgtac gcaagtcttt gatacgtgag gaatccaaaa catacgaacg 2222340
gatttggctg ccccaaccta catcggattt accttcttcc aacgcctgtt tctcttcatt 2222400
gcgtttgcgc atttccaatt catacagttt ggacttcaac atttccatcg cagcggcttt 2222460
gttggcgtgt tgcgaacggt cgttttgaca ttgcaccaca atccccgtcg gctcgtgggt 2222520
aatgcgcacg gcggagtcgg ttttattgat gtgctgaccg cccgcacccg atgcgcgata 2222580
ggtgtcgatg cgcaaatcgg cggggttgat ttcgatttcg atggaatcgt cgatttcagg 2222640
gtaaacgaac acggaggcaa acgaggtatg gcgtttgttg ttcgagtcaa acggcgagta 2222700
acgcaccaag cggtgaacgc cggtttcggt acgcagcaaa ccataagcgt attcgccttc 2222760
cacacggatg gtggcgcggt tgatgcctgc gatttcgccg tcgtcttctt caaggatttc 2222820
gattctgaag cctttgcgct cggcgtagcg gctgtacata cggaacagca tacccgccca 2222880
gtcttccgct tccgtaccgc ccgcgcctgc ggtgatgtcg ataaagcagt tgttcgggtc 2222940
ggcgggctgg ttgaacatcc gtttgaactc caaatccgcc atctgttttt ccagccccgc 2223000
tacgtcttcc tgcacggcgg caaaaccttc ttcgtcgttt tcttcgacgg tcatttcaat 2223060
cagcatgcgg ttgtcttcga tgcccgaagc gatgttgtcg agcgtcaaca cgatgccttc 2223120
gaggattttg cgctctttgc cgatttcttg ggcgcgtttc gggtcgttcc aaagttcggg 2223180
gtcttcggaa agaccgataa cttcttccaa tcggtctttc ttaccctgat aatccatata 2223240
aactcggatg tcttcgctgc gcttttccaa atcgttcagg gtattgttga gctggttgat 2223300
tacttcggct tccatgattc ttttgttctt tcaaaatttt aggggcgtat tgtacgggat 2223360
tcgggtattt ttttctatgg ataaagcctt ctggaaacac gttcagacgg catagcgtca 2223420
ataacggtat gccgccagtt tgcgtttgat ttcaggcaat gcggcacgtg ctgcctcctc 2223480
acccaaccgg atggcgcgtt ttttctgatc gaatccgccg actgcaccca aatccaaaac 2223540
ctgcggtttg ataaccacat ccgcctgccc caactcattt tgcaacgcag aaacgctcat 2223600
tacgttcagc gtctgatcga gataagagaa gaaaccttgg ctgatgtttt tgcccggacg 2223660
ggcggaaata tcgacggcaa tcacgaaatt cgccccctgc cgccgggcgg cactgacggg 2223720
cacgggctgc gacagaccgc cgtcaacata tgtatgcctg ccgatgataa cgggttggaa 2223780
cacattggga atggcggcgg aagcgcgcac agcctgcccg gcattcccct gattgaaagc 2223840
gacggccttg ccggtttcaa aatcagtagc aacggcggca aatttgatgg gaaactgctg 2223900
aatctgcctg ccgccgactt ttcggttgat gtaattttgc agcttttcgc ctttgataaa 2223960
accactggtg gacaaggtta aatcgaccaa atcggttttg cctaaaattt cggcttccaa 2224020
ttcgaggcgg tcgggcgaca tacccgatgc aaaaaggctg ccgacaatcg aacctgccga 2224080
tgtgccggta accaccttca caggaatacc gttttctttc aaaaccttaa taatacctac 2224140
atgggcaaat cctttagatg cgccgccacc gagtgccaaa ccgaccactg cggcgggttt 2224200
ggcggtttgc accggcttgc ggacagcatt atttcccgcc gtgccgcagg cggcaagcaa 2224260
cgcggcggcg gcgattgcca aaagcggtct gatttttgaa aacgttacca tattttccat 2224320
tcctttatat atcgcacccc gtcaaaaaga gggattgctt ttcttaacac ccccctttga 2224380
cagccaagca aatgggggct ttgttaagtc atcatcaaaa ttaatatttc tttttttttt 2224440
cctttacgga aattatattt gaaggcatac tatccaaggc gggaattatc tcacaacacc 2224500
gccgttatcc aaatatcccg ccttttttccc tttctttcca tcaaaatact ttctttttat 2224560
attcattaac ttgttaaatc attggctgcc gggtgtcagt ttttccgaca aaatccgtct 2224620
aatgggggtat caacagaacc aaaacaggaa cacttatgaa aatcggaaca acttggcaga 2224680
cggcatccgc tatgctggtt ttgcgtctgt ttgccgcata tgaatttttg gaatcgggtt 2224740
tgcaaaaatg gaacggggag aattggtttt ccgaaatcaa cgatcagttt ccattcccgt 2224800
tcaacttgct gccggacgcg ttaaactgga atctcgccat gtatgcggag cttttgctgc 2224860
ccgtattgtt gcttttgggt ttggcaacgc gtctgtcggc attggggctg atggtcgtta 2224920
ccgccgtcgc ttgggctgcg gttcacgccg gttcgggtta caatgtctgc gacaacggtt 2224980
ataaaatggc tttaatttat atcgtggtat taatcccgct gcttttccag ggtgcgggcg 2225040
gatggtcgct ggatacgctg ctgaaaaaac ggttttgccc ccgatgccgt ctgaaacaag 2225100
attgattcag tcgtggaatc tgactttaaa cattccaacc ttatctcgtt aacttgatat 2225160
tttgaaaagg aaatgacatg aacaaaaaca ttgctgccgc tctcgccggt gctttatccc 2225220
tgtctttggc cgccggtgca gttgctgcca acaaaccggc aagcaacgca acaggcgttc 2225280
ataaatccgc ccatggctct tgcggcgcgt ccaaatctgc cgaaggttcg tgcggcgcgg 2225340
ctggttctaa agcaggcgaa ggcaaatgcg gcgagggcaa atgcggtgcg accgtaaaaa 2225400
aaacccacaa acacaccaaa gcatctaaag ccaaggccaa atctgccgaa ggcaaatgcg 2225460
```

gcgaaggcaa atgcggttct aaataatccc accccttcaa accaagccgc gtttttcagt 2225520
aaaatgcggc ttttttaacg gcaaacaaag attttttaac aagcacatca ttcttttgtg 2225580
ccatccgaac cgggtaaaaa tatgattcaa cacgcaggct tgggctaccg ccgcgacttg 2225640
gcggaagact ttctctcgct ttccgaaaac agcccgatat gctttatcga agccgcaccg 2225700
gaaaactggc tgaaaatggg cggctgggcg cgcaaacagt ttgaccgtgt ggcggaacgg 2225760
ctgccgctgg cgttgcacgg attgtctatg tcgctgggcg ggcaagcacc gctggatact 2225820
gatttgatag acggcatcaa agaaatgatg cgccgttacg attgcacgtt tttctccgac 2225880
catttgagct actgccacga cggcggtcat ctttacgatt tgttgccgct gccctttacc 2225940
gaggaaatgg tgcatcatac ggcgcggcgt atccgcgaag tgcaagaccg tttgggctgc 2226000
cgcatcgccg tggaaaacac gtcctactat ctgcattccc cgcttgccga gatgaacgag 2226060
gtcgagttcc tcaacgccgt cgcacgtgag gccgattgcg gcattcatct ggatgtgaac 2226120
aatatctacg tcaacgccgt caatcacggt ctgctgtcgc cggaggcttt tttggaaaat 2226180
gtggatgcag agcgcgtgtg ctatatccat attgccggac atgacgtgga aacgccggaa 2226240
ttgttgattg atacacatgg cgcggcagtt ttgccgactg tttgggactt gctcgaactt 2226300
gcctatgcca agctgccgac gattccgccc accctgttgg aacgcgattt taatttcccg 2226360
cctttttccg aactcgaagc cgaagtcgcc aaaatcgccg attatcaaac gcgtgccgga 2226420
aaggaatgcc gccgtgcagc ctgaaacctc cgcccaatac cagcaccgtt tcgcccaagc 2226480
catacgcggg ggcgaagccg cagacggtct gccgcaagac cgactgaacg tctatatccg 2226540
cctgatacgc aacaatatct acagctttat cgaccgttgt tataccgaaa cgctgcaata 2226600
ctttgaccgc gaagaatggg gccgtctgaa agaaggtttc gtccgcgacg cgtgcgccca 2226660
aacgccctat tttcaagaaa tccccggcga gttcctccaa tattgccaaa gcctgccgct 2226720
tttagacggc attttggcac tgatggattt tgaatatacc caattgctgg cagaagttgc 2226780
tcaaattccg gatattcccg acattcatta ttcaaatgac agcaaataca caccttcccc 2226840
tgcggccttt atccggcaat atcgatatga tgttaccgat gatttgcatg aagcggaaac 2226900
agccttgtta atatggcgaa acgccgaaga tgatgtgatg taccaaacat tggacggctt 2226960
cgatatgatg ctgctagaaa taatggggtt ctccgcgctt tcgtttgaca ccctcgccca 2227020
aacccttgtc gaatttatgc ctgaggacga taattggaaa aatattttgc ttgggaaatg 2227080
gtcaggctgg actgaacaaa ggattatcat cccctccttg tccgccatat ccgaaaatat 2227140
ggaagacaat tccccgggcc aaaaccatct atccgcataa aattaccttg ttcccgatac 2227200
tatgccgcta cccgacctga ccgatgccga attaatagag tcgcgtaaac tgcttctgca 2227260
ttttgcgcgg cttcagttgc ccgaccaccc tgatttggct gaagatttag tgcaggaaac 2227320
attgctgtcc gcatacagcg caggcgacag ttttcaaggc agggcacttg tcaacagctg 2227380
gctttttgcc atattgaaaa acaaaattat tgacgcatta cgtcaaatcg gaaggcagag 2227440
gaaagtcttt accacactgg atgacgagct actggatgaa gcatttgaaa gccatttttc 2227500
ccaaaacggg cattggacgc aggaagggca gccgcaacat tggaacactc cggaaaaatc 2227560
attaaacaac aacgaattcc aaaaaattct gcaaagctgc ctatacaacc tgcctgaaaa 2227620
caccgcacgg gtatttaccc tgaaggaaat actcggtttt tcatccgacg aaatacaaca 2227680
aatgtgcggt atcagcacgt ccaactacca caccattatg caccgcgccc gagaatcatt 2227740
gcgccaatgc ctgcaaatca aatggttcaa ccaagaaaac ccgaagtaaa cgttatgaaa 2227800
aaatgccgcg atatcgccct gcttctttcc aaacatcagg accgggaaac caccccgggc 2227860
gagaagattt ccatatacac acacctgctg ttctgtccgt attgccgtga atataaaaga 2227920
caacttcaaa ccatcaaaag atcactggca aaaacaacca gaacttcaaa ataaatgccg 2227980
tctgaaaagg cttcagacgg cataagctga cggaaacaaa tcaaaccgat ttactgttat 2228040
ctgcagttca tccataatac acacttcaaa agcagcatat ttccccatac ggaatgtata 2228100
aatacgcaaa atacgaaggc tgcatcaatt tgccatattt gctttatttg ccttatttca 2228160
cagacggcgc tacccctccc gcccaacccg ttctttctga atgagcagat ttcaatgatt 2228220
aaggaaaccc taatgcgccc aatcttccta tctttcgttt tattccctat tttgataacc 2228280
gcctgcagca caccggacaa gtctgcccga tgggaaaata tcggcacaat ctcaaacggc 2228340
aatattcata catatatcaa taaagacagc gtgagaaaaa acggaaatct gatgattttc 2228400
caagataaaa aagttgttac caatctaaaa caagaacgtt ttgccaacac ccccgcatac 2228460
aagactgcca ttgccgagtg ggaaatccac tgcaacaaca aaacataccg cttaagttcg 2228520
ctacagttgt ttgatacaaa aaacacggaa atttccacac aaaactacac agcctcttcc 2228580
ctccgcccga tgagcatcct gtccgggaca ttaaccgaaa aacaatatga aaccgtatgc 2228640
ggaaaaaaac tctgattgca acttatacac aaacttaccc acaaacctta tcataaaaat 2228700
gccgtctgaa atactgaaat atcagcattt cagacggcat tttgccattc cctgaaaatt 2228760
atccacaaag ttatccacat tattttttaa aaccggcttc catccgaaat atagtggatt 2228820
aacaaaaatc aggacaaggc gacgaagccg cagacagtac aaatagtacg gcaaggcgag 2228880
gcaacgccgt actggtttaa atttaatcca ctatataaac tcgctataca atttcactat 2228940
ccaaacgtaa attgttccat tgatacacaa aactgcttac cccataattt ttgataaagc 2229000
atttcttaca ttcccggctc cgtcccgtaa ccaacacagc ggcggattcg catttgaagt 2229060
gcaactttcc ctaacagaaa aaggccagta tgcggtagca tacgaccttt cctgcaagaa 2229120

agattgccat gagctacacg caactgaccc agggcgaacg ataccacatc caatacctgt 2229180
cccgccactg caccgtcacc gaaatcgcca aacagctgaa ccgccacaaa agcaccatca 2229240
gccgcgaaat cagacggcac cgcacccaag ggcagcaata cagcgccgaa aaagcccagc 2229300
ggcaaagcca gactatcaaa cagcgtaagc gacaacccta taagctcgat tcgcagctga 2229360
ttcagcacat cgacaccctt atccgccgca aactcagtcc cgaacaagta tgcgcctacc 2229420
tgtgcaaaca ccaccagatc acgctccacc acagcaccat ttaccgctac cttcgccaag 2229480
acaaaagcaa cggcagcacg ttgtggcaac atctcagaat atgcagcaaa ccctaccgca 2229540
aacgctacgg cagcacatgg accagaggca aagtacccaa ccgtgtcggc atagaaaacc 2229600
gacccgctat cgtcgaccag aaatcccgta tcggcgattg ggaagccgac accattgtcg 2229660
gcaaaggaca gaaaagcgca ttattgacct tggtcgaacg cgttacccgc tacaccatca 2229720
tctgcaaatt ggatagcctc aaagccgaag acactgcccg ggcagctgtt agggcattaa 2229780
aggcacataa agacagggtg cacaccatca ccatggataa cggcaaagag ttctaccaac 2229840
acaccaaaat aaccaaagca ttgaaagcgg agacttattt ttgtcgccct taccattctt 2229900
gggagaaagg gctgaatgag aacaccaacg gactcatccg gcaatacttc cccaaacaaa 2229960
ccgatttccg taacatcagt gatcgggaga tacgcagggt tcaagatgag ttgaaccacc 2230020
gaccaagaaa aacacttggc tacgaaacgc caagtgtttt attcttgaat ctgttccaac 2230080
cactaataca ctagtgttgc acttgaaatc cgaatccaag agcctctaaa aaataatcgc 2230140
ttgttttgac accgatacac tcatatagtg gattaacaaa aatcaggaca aggcgacgaa 2230200
gccgcagaca gtacaaatag tacggcaagg cgaggcaacg ccgtactggt ttaaatttaa 2230260
tccactatac aaatacagaa actcaagaaa ataaccttgt gtattgacca tctcaagcaa 2230320
ttcagaaaaa tcaagaaatt ttctgaccgt aaacaaacgt ttccctaaaa aaacgatgtc 2230380
ttcaaaaata tcgaacaaat agagaccttt gcaaaaatag tctgttaacg aaatttgacg 2230440
cataaaaatg cgccaaaaaa ttttcaattg cctaaaacct tcctaatatt gagcaaaaag 2230500
taggaaaaat cagaaaagtt ttgcattttg aaaatgagat tgagcataaa attttagtaa 2230560
cctatgttat tgcaaaggtc tcaaataatc atcttcggcg ttttcatttt tatggattaa 2230620
aacaacacgg gaaaaatctg ttttcagatg cttgcccgct tgattgttcg gattattgtc 2230680
cggaacgaca aaaccgtcct caaaattaaa gcagacgttg cgtccttcta cctttatctc 2230740
tgtgcaataa caatcatgta gagaaatgct atccgaaaaa ttttttcttt gtgtatgcaa 2230800
aaaaagtttt cattcaagta cccatatcta acgcaaacgt ttacctgttt ccccgtcaat 2230860
aatctgactc ggcgatttct gcctgccgat tctcccacca acaatccaca catcgcgtcc 2230920
gaattgcctt ctgacttccc tctccgtccg acacgcgcgt ttgcctgcgc ggttgcacga 2230980
agtcgagacc aaaggcgttt gcaaagcctg acacaagcgg cgcgcaccta catgggcggg 2231040
aaccctgacc gccaacttgc tgcgctgttt cccgcgtaac tcgggtaaga cacaggattt 2231100
ggcggataat aggaacgttt taggggcggg ccattctttt ctaagcatat cctgaagatt 2231160
ttcagacggc atttgaagta aaggctgcaa ttgttcaaat tgattcccga tgacaatcat 2231220
acccttgtgt tgcggtcttt ttttcaaatg cgccaactta ccgagtgctt tggctaatgt 2231280
cggaagacac cccaagccat aacaagattc ggtcggataa gcgaccaaac caccttttt 2231340
caaataaacg cttaacttac gttgcgctga tgctgcgata attctcggaa ataacataat 2231400
ataaaatacc gtctgaagca cattagtcat acttggcttc agacggcatc atcctctttc 2231460
taattaacgg ttaatcgctt tatcggcaat gtctttacgg tattgcatcc cgtcgaaact 2231520
gattttttcc aacgcgccat atgccttagc tttcgcttgc gccacattat cgcccaatcc 2231580
cacaacacac aatacgcgtc cgccgttggt caatacgtca cctttctcgt ttgccgttgt 2231640
acctgcatgg aaaactttgc cgatttggtt ggcagcatcc agaccggaaa taatatcgcc 2231700
ttttttgggc gtttcggggt aattttgcgc cgccagtacc acgcccacgg cagtttgcgg 2231760
gctccattcc gcggttacgc tatcgagttt gccgtctatt gccgcttcaa ccaaatccga 2231820
taagtcgctg ttcagtcggc tcataatcgg ctgggtttca ggatcgccga aacggcagtt 2231880
aaactcaatc gtatagggtg caccgctttg atcaatcatc aaacctgcgt acaggaaacc 2231940
ggtgaactca tgcccctccg ctttcatccc tgctacggtc ggcaaaataa tttcattcat 2232000
cgcgcgttcg tacacaacag gcgttaccac aggcgcaggg ctgtacgcac ccataccgcc 2232060
cgtattcaga cctttgtcgc cgtctaaaag acgcttgtgg tcttggctgg ttgccatagg 2232120
cagtacatta ttgccatcaa ccatgacgat aaaactcgct tcttcgcctt gcaggaaatc 2232180
ttcaattaca acacgcgcgc cggcattgcc cattttgttg tccagcagca tatcatcaat 2232240
cgcagcatgc gcttcatcca aagtcatcgc cacaatcacg cctttacctg ccgccaaacc 2232300
atcggctttg ataacgatag gcgcaccttt ctgattgacg taatcatgtg cggcatcggc 2232360
gttttcaaag gtttgatatt gcgcggtcgg aatattgtat ttcgccataa atgctttggc 2232420
gaaatctttg gaactttcca actgcgccgc atattgtgtc ggaccgaata tttttagtcc 2232480
tgcagcacgg aaatcatcca caatacctgc cgccaaaggc gcttcagggc cgacgacggt 2232540
aaaaacaata ttttctttac gacagaattc aatcaaatcc tgatgcgcag tcaagtcgat 2232600
gttttgcaac ttgggttcaa tcgctgtacc ggcattacca ggcgcaacaa atactgtttc 2232660
cactttaggc gactgcgcca atttccaagc cagcgcgtgt tcgcgaccgc cattaccgat 2232720
aaccagcagt ttcataccat ctccttgaca aatatgtact tttaacgaaa actcgataca 2232780

aagggacttt tatcccatct gaagaaattt tagtagaatc aaacaaaaga ccgcttcatt 2232840
ccactctgca acctattcaa cttatccata aattaaaaaa ggacaagcaa ccatgcaaaa 2232900
acgtattgat gaaatccaaa gcaaataccg cgaatggtgt catttactac cgcaactgga 2232960
agaagacatc cgccgttgga aacatgtcgt cactttaatt cgcgacatgg acaatttcta 2233020
tacccacgag tatcaggcgt gtcatcaggc tattgaagac ggggtagaac tggatttgag 2233080
tacggaaggc gaatacagca ttatgagtga agatgcgcta tggaacgcgc tgggcgaatt 2233140
ccatcaattg gcttggttat atttgcgctc cagcgtcgat gccttagaca aatatacaca 2233200
agaagattag tcagcgaaga ggtcgtctga aataccatca caaagcattt cagacgacct 2233260
ttcattcaaa aggcttttcc gtatttactt caatctgccg agtattcttc caagccgcaa 2233320
cacaggcctc ataatttacc aacgacaaac tgaccgtcaa tcggcaatcc aactgcaaat 2233380
cccgctccaa tatatccgcc tgatattgtt tggcaatgcg tatcgcttca ttcaaaaacg 2233440
gatattcaca tttcagccaa acagtttttt caatattctt ttcaactact tctgcaactg 2233500
ccaacgcttg agccgtcgcc tctttgtacg catgtatcag acctggaaca cctaacaaag 2233560
taccaccgaa atagcggacg accacaacca aaacgtcggt aatacccacc gaatcaatct 2233620
gtcccaaaat tggtcgtcca gcacttcctg atggctctcc atcatcgttg gcacgaaatt 2233680
gcacaccatc cacacccaaa cgataggcat agcaccagtg tcgtgcttta tgatgctctt 2233740
cctttaacgg atcgaggtat tttttcacat cagccaatgt ccgaatcgga taggcaaatg 2233800
caataaaacg gctgccttta tctttaaact cagcctgcgt caaggaagta atggttttat 2233860
aagtcgtaat catgctgaaa tgttttcaga cgacctcatt aataacaagg tcgtctgaaa 2233920
gtttcacgtg aaacatcaat ttttcaatac ttctgttaat tgtggaacga tttcaaataa 2233980
atcgccaacc aatccgtaat cggctacatt gaaaatcggc gcatcagcat ctttattgat 2234040
tgcaacaatc accttactgt cttgcatacc ggcaacgtgt tgaattgcac ctgaaatacc 2234100
gattgcaaaa tagagttgcg gcgcaaccac tttaccggtt tgtccgactt gagcatcgtt 2234160
tggcgcatat tcggcatcaa ctgctgcacg ggatgcaccg attgccgcac ctaaaacatc 2234220
cgccaacggt gtcagcactt cattgaattt ttccgcacta cccaacgcac gaccaccgga 2234280
aacaatcact tttgcctgag tcagttcagg acgatcggaa tgggaaagct gacggttaac 2234340
aaaacgactc aggttttggg caggggttgc ttcaacatta attacctcag cattaccacc 2234400
ttgcgccgcc actgcgtcaa aaaccgtcgc acggaaggtc agcaccaatt tttctgaatc 2234460
agcttgcacg gtttcaaatg cattacccgc ataaatgggg cgcacaaaag tcgtgttatc 2234520
cacaatttcg gtcaaatcag aaatttgcgg tacgtctaat aaggctgcta cgcggggcaa 2234580
aaggttttta ccgaatgtgg ttgccgttgc tgcaacatag cggtaatcgg ccgccaattt 2234640
aacaaccagc ggagccaact cttcagccaa accttcggca taatgagcag catctgcaac 2234700
caaaactttt ttcacccccg ctacttgctt cgcgaattcc actacagcag atgcgccgtt 2234760
tccggcaacc aataaatcga ctttgcccag tttggcggca gcggtaacag catgcaaagt 2234820
ggtaggattc aactgtttgt tgtcgtgttc gacaataatc aatacactca tttcagcctc 2234880
ctcaaatcac tttggcttcg tttttcaatt tttcaaccaa ttcggcaacg cttgctactt 2234940
ttacgcctgc ctgacgcgcc ttaggttcgg caaatttcac cgttttcaaa cgaggtgaaa 2235000
tgtcggcaac caaatcgtca ggagtcagtt tttccaaagg ttttttcttt gccgccataa 2235060
tattggggag tttgacaaag cgcggctcgt tcaaacgcaa atccgcgctg ataacagcag 2235120
gcagtttcaa tgcgatggtt tcttcgccgc catcgatttc ccgcacaatc tgcacttcgt 2235180
cgccttcaat ttgtactttg gacgcgaacg taccttgcgc cgcattcagc aaagctgcca 2235240
gcatttgcgc cacttgattg gcatcatcat caatcgcttg tttgcccaaa aagaaaattt 2235300
gcggattttc tttgtccgca acggctttca gcaacttagc aacggccaga gactccagtt 2235360
tagtatcggt ttcaacatga atggcacggt cggcacccat cgccaaagct gtacgcaagg 2235420
tttcttcgca ttttttctca cccaaagaaa ccgctacgat ttcgcttact tttccggctt 2235480
ctttcaaacg gacagcttct tccacagcga tttcgtcaaa cggattcatc gacattttga 2235540
cattgccgat atccacatcc gaaccatcgg cttttacacg aactttgacg ttgtagtcca 2235600
ctacgcgctt tactgcgacc agtgctttca ttgaaccctc ctaaaaagaa cgctgctttc 2235660
accatccagc gaaaccaaac cttcttccct ataaaaccaa atccgttttc cttaaaaacg 2235720
aattcattca aaaatctttc ggataatgct tgccgattat accattttta aagcatttac 2235780
tcagactagc ggatatacat tcctgtatct aataaattgg aaaatatcat gccgccatat 2235840
cagttttaga cgacccttta gcctttatct gctgcaacac aatccatcag cgcttgataa 2235900
accaaatctg cggtcggaat ctgcccgata ttgcccaaat tttttgcaat tggcgaaacc 2235960
tgaacgcctg ttttaatcgg atcggtatcg gtataaatgc cgaccacagg tttttccaag 2236020
gcatttgcca aatgcagcaa accggtatcc acgccgacaa ttccgaccgc gtatttcagc 2236080
agatacgctg cctgcaataa atttatttg tcgcacacaa tagcaaacgg cagcccatct 2236140
gcaatttgtt tggcacgcgt tttttcatct tcatttcccc aaggcaggta aatattgcat 2236200
tgctgttctt cattcaactt ttgcagcaac gaccgccagt tttccacagg ccataactta 2236260
ctgtcccgac tggtcgcatg caaagccgca taatacggct gcgctaaatt tttcagacgg 2236320
cctgcttcag gaacagtcaa gccaaatacc tgcgtttccg gcattacata cccaaatact 2236380
tgggcaaaca gttcacggtt gcgccaaacg gcattttttc ccttcggtac agcgtatgtt 2236440

tttacatacg ccaaagcagc ccatccctcg cgcgcactgt ttttatccaa accacaaatc 2236500
ggggattttg ccattttagc gaaacacgcg cttttaatca gaccttgact gtccaatacg 2236560
aaatcaaata cttcctgccg caaagtctgt ttcagatgac ccatttcccg ccaagtttca 2236620
gcccgaaaga gatgtttgcg ccattgccgc catttcatca catggatttt ttttacaaac 2236680
ggatgcaggc gcgcaatatc tgcaaatcca gcctcacata gccaatgcag ttctacatca 2236740
ggacattgtc gcgccaaatc ttcgattgcg ggcaaagtgt gaattaaatc gcccatacta 2236800
gacaagcgga caagcaaaat tttcatattt aggaaggggg tttcacgtga aacaatttta 2236860
acttattgat tattaatata tttatttatt tcatcagcgt tttttaagat gattgcccca 2236920
gcagaatgca tttcctgcca tgctgtttcg atggtttccg gcgcaatacc ccgacaagcc 2236980
gcttcattga cgacaacctg ccaacgaccg cctttgagta actgcaaaac cgttgtttta 2237040
acacaataat ccgtagctaa cccaccgata ataaccgtat ccgtattttg acaacgcagc 2237100
cattcaatca gccctgtgct tagtttttcc tcaatatcgt gaaaacacgc gccgtaagga 2237160
tgcaattcag gatcaacacc tttccaaacg caataatcgt attctttagc agaaggcagc 2237220
ccgtccaata attcatagcc gcgcgtaccg accatcgcat gagccaccca agtcaaatcc 2237280
gcatcaggca aacctgtcgg cttcaacata tcaacagggt tatccacaag ccatttcgct 2237340
accatatgat gcgcatcttt cgtcatcacg cgcaaatccg ccaaagcggc ttgcgcattc 2237400
aactcctcga caatcaaatg cccctcgttc acgggcagtt cgtcaggaca cagtggcgta 2237460
aacgtttttt gtgcatcaac atcaatggaa acaatcatct cattatttca acgcgattaa 2237520
aatgccctgt attataacaa attactgccc aaaagcggta aaaccgattg tgataagata 2237580
aggtttttcc aaaaaactta tccacaacct tatgacttat accattaccc ccatcggcac 2237640
cgcccgctcg ccctacaaac agaaattcgg catcgcccgc cagcccggtt tggtctccgc 2237700
cgcaaaagcc tgcatcgagc tgaatcccaa attcaccgca gacagcgtgc gcgggctgga 2237760
agatttcgat tatgtgtgga taagttttat ttttcacggc gtattggatg aaggctgggc 2237820
gcaaatggtg cgcccgccac ggctcggcgg caaacaaaaa atgggcgtgt tcgccacgcg 2237880
cagcccccac cgccccaacc atctcggact ctcgctcctg aaactcgaac gcatcgaaac 2237940
cggcaaaccc gtccgcctct attgcagcgg cgcagacctg ctggacggca caccgattgt 2238000
ggacatcaaa ccttatatcc cctttgtcga atccaaaccc gatgccgcat ccggtttcgt 2238060
cagcggcaaa cccgtagagt tggaagtcgt ttggcaggaa aacatcggcg cggaaaattt 2238120
atctgcaaac accaaaaacc ttatcagcca aagcattgcc caagatccgc gccccgccta 2238180
tcagaatatt cccgaacgga tttatgtgat gaatattgca gattacgaag tcagatttca 2238240
aatcgaggaa aaccgtgcaa ccgttattga tctttcccca accccgcttt aaatcgggca 2238300
aaaatccggt tttgccgcat agcagttgaa caaacggctg ttgtttgttc gccataagcc 2238360
gcaatatcaa gttatagcgg attaaattta aatcaggaca aggcaacgaa gccgcagaca 2238420
gtacaaatag tacggcaagg cgagataacg ccgtactggt ttaaatttaa tccactatac 2238480
agataaacaa tgccgtctga acgcaatgtg ttcagacggc atttacttat ccacaggttt 2238540
gttcaagcct tagattttgc ctgcgaagta ttccaaagtg cggacgagtt ggcaggtgta 2238600
ggacatttcg ttgtcgtacc aggcaacggt tttcaccaat tgtttgccgc ccacggtcat 2238660
cacgcgggtt tgggtcgcat cgaagagcga gccgtattcg atgccgacaa cgtcggaaga 2238720
aacgatttga tcttcgttgt agccgtaaga ttcgctggcg gcggctttca tcgcggcgtt 2238780
gatttcttct ttggttacag ggcgttcgag gatggaaacc aattcggtca gcgagccgct 2238840
ggcaacaggg acgcgttggg cggagccgtc gagtttgccg ttcaattcgg ggataaccag 2238900
accgatggcc ttggcggcac cggtgctgtt gggcacgatg ttgagcgcgg cggctcgggc 2238960
gcggcgcaaa tcgcctttgc ggtgcggcgc gtcaagggtg ttttggtcgc cggtgtaggc 2239020
gtggatggtg gtcatcagac cttcgactac gccgaactct ttttgcagga ctgccgccat 2239080
cggggcaagg cagttggtgg tgcaggaagc ggcggagata acggtttcgc tgccgtccaa 2239140
aatgtcttgg tttacgccat atacgacggt tttcacatca ttgccgccgg gtgcggaaat 2239200
cacgactttg cgcgcgccgg ccctgatgtg tgcttcggct ttggttttat tggtaaagaa 2239260
gccggtacat tcgaggatga catccacacc caactcgccc caaggcaatt cttcgggatt 2239320
cggattggca aaaactttga tctctttgcc gtttaccacg atggcatcgt cttttaattc 2239380
ggcagtacct tggaaacggc cttgtgtgct gtcgtatttg aaaaggtgca gcagcatttc 2239440
ggcaggggtc aggtcgttga cggcgacgac ttcgatgtcg tgggcttttt caatttgacg 2239500
caatgcgagg cggccgatgc ggccgaaacc gttaatcgct actttaatgc tcatgtatat 2239560
actccaagct gtgaaacgaa atttcaatac ctgtattgta ttctgaaata aagttacatt 2239620
ccactattac atctaactac ttgccgctta tttgatatag atgaatttta ctgtttgcac 2239680
agatttccaa aacttttacc atcaatattt gaatttaaaa ttttaatgat gattttgatg 2239740
attgccaacc tgcttgtgcg taagtagcaa atatccaata ttttcattac ctttttgtca 2239800
aataagtttg agtttaagac ttgctgtata agacagataa gcgtggatgt tttttgactt 2239860
aataatattt ctgtggataa ctttgctgtt ttcctagttg tctccacaac cttattgaca 2239920
ggcttacggt cagtctcatt ccgtcgaaga caaaaccttt tgctacaata ccgttttcct 2239980
aatgataagg cagccccatg tccaaatccg ccgtttcccc aatgatgcag caatacctcg 2240040
gcatcaaagc gcaacatacc gacaaactgg tgttttaccg tatgggcgat ttttacgaga 2240100

tgtttttcga cgatgcggta gaagcggcaa aacttttgga tattaccctg accacgcgcg 2240160
gacaggtgga tggcgagccg gtcaaaatgg caggcgtgcc gtttcacgcc gccgaacaat 2240220
atctggcgcg cctggtcaag ttgggcaaaa gcgtggcgat ttgcgaacag gtcggcgaag 2240280
tcggcgcggg caaagggcct gtggagcgca aagtcgtgcg catcgtaacg cccggcacgc 2240340
tgaccgattc cgcattgctg gaagacaagg aaaccaaccg catcgttgcc gtgtcccccg 2240400
acaaaaaata catcggtttg gcgtgggcat cgctgcaaag cggcgaattc aaaaccaagc 2240460
tgacaactgt ggataaattg gacgacgaac tggcgcgcct gcaggcggcg gaaattctgt 2240520
tgcctgacag taaaaacgca ccgcaacttc agacggcatc gggtgttacg cgcctgaacg 2240580
cgtggcagtt tgccgccgac gcgggggaaa aactgctgac ggaatatttc ggctgccagg 2240640
atttgcgcgg cttcggtttg gacggcaaag aacacgccgt tgcgattggc gcggcaggtg 2240700
cactgttgaa ctatatccgt ctgacgcaaa acctgatgcc gcaacatttg gacggcctgt 2240760
cgctcgaaac cgacagccaa tatatcggta tggatgccgc cacgcgccgc aatctcgaaa 2240820
tcacgcaaac cctctccggc aaaaaatcgc cgaccctgat gtccacgctc gacctttgcg 2240880
ctacccatat gggcagccgc ctcttggctc tctggctgca ccacccttta cgcaaccgcg 2240940
cccacatccg agcgcgccaa gaagccgttg ccgcgctgga aagccaatac aaacccctcc 2241000
agtgccgtct gaaaagcatt gccgacatcg aacgcatcgc cgcccgtatt gccgtgggta 2241060
acgcccgccc gcgcgacctc gccgccctgc gcgacagcct gtttgccctg tccgaaatcg 2241120
aattgtccgc cgagtgcagc agtctcttag gaaccctcaa agccgttttc ccggaaaacc 2241180
tatccacagc cgaacagctc cgccaagcca ttttgcccga accttccgtc tggctgaaag 2241240
acggcaatgt catcaaccac ggttttcatc ccgaactgga cgaattgcgc cgcattcaaa 2241300
accatggcga cgaatttttg ctggatttgg aagccaagga acgcgaacgt accggtttgt 2241360
ccacacttaa agtcgagttc aaccgcgttc acggctttta cattgaattg tccaaaaccc 2241420
aagccgaaca agcacctgcc gactaccaac gccggcaaac ccttaaaaac gccgaacgct 2241480
tcatcacgcc ggaactgaaa gcctttgaag acaaagtgct gactgctcaa gagcaagccc 2241540
tcgccttaga aaaacaactc tttgacggcg tattgaaaaa ccttcagacg gcattgccgc 2241600
agcttcaaaa agccgccaaa gccgccgccg cgctggacgt gttgtccaca ttttcagcct 2241660
tggcaaaaga gcggaacttc gtccgccccg agtttgccga ctatccggtt atccacatcg 2241720
aaaacggccg ccatcccgtt gtcgaacagc aggtacgcca cttcaccgcc aaccacaccg 2241780
accttgacca caaacaccgc ctcatgctgc tcaccggccc caatatgggc ggcaaatcca 2241840
cctacatgcg ccaagtcgcg ctgattgttt tattggcaca caccggctgt tttgtgcctg 2241900
ccgatgccgc cacaatcggg cccatcgatc aaatcttcac ccgcatcggc gcatcggacg 2241960
acctcgcctc caaccgctcc actttcatgg tcgaaatgag cgaaaccgcc tacatcctgc 2242020
atcacgccac cgaacaaagc cttgttttaa tggacgaagt cggacgtggt acttccactt 2242080
tcgacggcct cgccctcgcg cacgccgttg ccgaacacct gctgcaaaaa aacaaatcct 2242140
tcagcctgtt tgctacccac tatttcgagc tgacctacct gcccgaagcc cacaccgccg 2242200
ccgtcaatat gcacctttcc gcgctcgaac agggacagga catcgttttc ctgcaccaaa 2242260
tccaaccggg tcccgccggt aaaagctacg gcattgccgt cgccaaactc gccggcctgc 2242320
ctgtacgcgc attgaaatcc gcccaaaagc atttgaacgg actggaaaac caagccgccg 2242380
cgaaccgtcc ccaactggat attttcagta ccatgccgtc tgaaaaagga gatgaaccga 2242440
atgtgggcaa ctttgtggat aaagcagagg aaaaacattt tgaaggtata ttggcagcag 2242500
ccttggaaaa actcgatccc gacagcctga ccccgcgcga agcattgtca gaactgtacc 2242560
gtctgaaaga tttgtgcaaa tccgtatctt aatttccgtt gtcggaacag catcaaacca 2242620
tatggaaaaa tctgtggata aacattatct gacaggaaat ttccaaacat aaaaaatgcc 2242680
gtccgaacag ctcagacggc atccgtccat tcggct 2242716

<210> 2
<211> 459
<212> DNA
<213> Neisseria meningitidis

<400> 2
ataacgcgga tttgcggctg cttgatttca acggttttca gggcttcggc aagtttgtcg 60
gcggcgggtt tcatcaggct gcaatgggaa ggtacggaca cgggcagcgg cagggcgcgt 120
ttggcaccgg cttctttggc ggcagccatg gcgcgtccga cggcggcggc gttgcctgca 180
atcacgattt gtccgggtga gttgaagttg acggcttcga ccacttcgct ttgggcggct 240
tcggcacaaa tggctttaac ctgctcatct tccaagccga gaatcgccgc cattgcgccc 300
acgccttgcg gtacggcgga ctgcatcagt tcggcgcgca ggcgcacgag tttgaccgcg 360
tcggcaaaat tcaatgcgcc ggcggcaacg agtgcggtgt attcgccgag gctgtgtccg 420
gcaacggcgg caggcgtttt gccgcccgct tctaaatag 459

<210> 3

<211> 152
<212> PRT
<213> Neisseria meningitidis

<400> 3
Ile Thr Arg Ile Cys Gly Cys Leu Ile Ser Thr Val Phe Arg Ala Ser
1 5 10 15

Ala Ser Leu Ser Ala Ala Gly Phe Ile Arg Leu Gln Trp Glu Gly Thr
20 25 30

Asp Thr Gly Ser Gly Arg Ala Arg Leu Ala Pro Ala Ser Leu Ala Ala
35 40 45

Ala Met Ala Arg Pro Thr Ala Ala Ala Leu Pro Ala Ile Thr Ile Cys
50 55 60

Pro Gly Glu Leu Lys Leu Thr Ala Ser Thr Thr Ser Leu Trp Ala Ala
65 70 75 80

Ser Ala Gln Met Ala Leu Thr Cys Ser Ser Ser Lys Pro Arg Ile Ala
85 90 95

Ala Ile Ala Pro Thr Pro Cys Gly Thr Ala Asp Cys Ile Ser Ser Ala
100 105 110

Arg Arg Arg Thr Ser Leu Thr Ala Ser Ala Lys Phe Asn Ala Pro Ala
115 120 125

Ala Thr Ser Ala Val Tyr Ser Pro Arg Leu Cys Pro Ala Thr Ala Ala
130 135 140

Gly Val Leu Pro Pro Ala Ser Lys
145 150

<210> 4
<211> 459
<212> DNA
<213> Neisseria gonorrhoeae

<400> 4
atgacgcgga tttgcggctg cttgatttca acggttttga gtgtttcggc aagtttgtcg 60
gcggcgggtt tcatcaggct gcaatgggaa ggaacggata ccggcagcgg cagggcgcgt 120
ttggctccgg cttctttggc ggcagccatg gtgcgtccga cggcggcggc gttgcctgca 180
atcacgactt gtccgggcga gttgaagttg acggcttcga ccacttcgcc ctgtgcggat 240
tcggcacaaa tctgcctgac ctgttcatct tccaaaccca aaatggccgc cattgcgcct 300
acgccttgcg gtacggcgga ctgcatcagt tcggcgcgca ggcggacgag tttgacggca 360
tcggcaaaat ccaatgcttc ggcggcgaca agcgcggtgt attcgccgag gctgtgtccg 420
gcaacggcgg caggcgtttt gccgcccact tccaaatag 459

<210> 5
<211> 152
<212> PRT
<213> Neisseria gonorrhoeae

<400> 5
Met Thr Arg Ile Cys Gly Cys Leu Ile Ser Thr Val Leu Ser Val Ser
1 5 10 15

Ala Ser Leu Ser Ala Ala Gly Phe Ile Arg Leu Gln Trp Glu Gly Thr
20 25 30

Asp Thr Gly Ser Gly Arg Ala Arg Leu Ala Pro Ala Ser Leu Ala Ala
35 40 45

Ala Met Val Arg Pro Thr Ala Ala Ala Leu Pro Ala Ile Thr Thr Cys
50 55 60

Pro Gly Glu Leu Lys Leu Thr Ala Ser Thr Thr Ser Pro Cys Ala Asp
65 70 75 80

Ser Ala Gln Ile Cys Leu Thr Cys Ser Ser Ser Lys Pro Lys Met Ala
85 90 95

Ala Ile Ala Pro Thr Pro Cys Gly Thr Ala Asp Cys Ile Ser Ser Ala
100 105 110

Arg Arg Arg Thr Ser Leu Thr Ala Ser Ala Lys Ser Asn Ala Ser Ala
115 120 125

Ala Thr Ser Ala Val Tyr Ser Pro Arg Leu Cys Pro Ala Thr Ala Ala
130 135 140

Gly Val Leu Pro Pro Thr Ser Lys
145 150

<210> 6
<211> 459
<212> DNA
<213> Neisseria meningitidis

<400> 6
atgacncnga tttgcggctg cttgatttca acggtttnna gggcttcggc gagtttgtcg 60
gcggcgggtt tcatgaggct gcaatgggaa ggtacngaca cnggcagcgg cagggcgcgt 120
ttggcgccgg cttctttggc ggcaagcata gcgcgctcga cggcggcggc attgcctgca 180
atcacgactt gtccgggcga gttgaagttg acggcttcaa ccacttcatc ctgtgcggat 240
tcggcgcaaa tttgttttac ctgttcatct tccaagccga gaatcgccgc cattgcgccc 300
acgccttgcg gtacggcgga ctgcatcagt tcggcgcgca ngcgcacgag tttgaccgcg 360
tcggcaaaat ccaatgcgcc ggcggcaacn agtgcggtgt attcgccgan gctgtgtccg 420
gcaacggcgg caggcgtttt gccgcccgct tccgaatag 459

<210> 7
<211> 152
<212> PRT
<213> Neisseria meningitidis

<400> 7
Met Thr Xaa Ile Cys Gly Cys Leu Ile Ser Thr Val Xaa Arg Ala Ser
1 5 10 15

Ala Ser Leu Ser Ala Ala Gly Phe Met Arg Leu Gln Trp Glu Gly Thr
20 25 30

Asp Thr Gly Ser Gly Arg Ala Arg Leu Ala Pro Ala Ser Leu Ala Ala
35 40 45

Ser Ile Ala Arg Ser Thr Ala Ala Ala Leu Pro Ala Ile Thr Thr Cys
50 55 60

Pro Gly Glu Leu Lys Leu Thr Ala Ser Thr Thr Ser Ser Cys Ala Asp
65 70 75 80

```
Ser Ala Gln Ile Cys Phe Thr Cys Ser Ser Ser Lys Pro Arg Ile Ala
                85                  90                  95

Ala Ile Ala Pro Thr Pro Cys Gly Thr Ala Asp Cys Ile Ser Ser Ala
            100                 105                 110

Arg Xaa Arg Thr Ser Leu Thr Ala Ser Ala Lys Ser Asn Ala Pro Ala
        115                 120                 125

Ala Thr Ser Ala Val Tyr Ser Pro Xaa Leu Cys Pro Ala Thr Ala Ala
    130                 135                 140

Gly Val Leu Pro Pro Ala Ser Glu
145                 150

<210>   8
<211>   600
<212>   DNA
<213>   Neisseria meningitidis

<400>   8
tccgttatcg ggcgtatgga gttggacaaa acgtttgaag aacgcgacga aatcaacagt   60
actgttgttg cggctttgga cgaggcggcc ggggcttggg gtgtgaaggt tttgcgttat   120
gagattaaag acttggttcc gccgcaagaa atccttcgct caatgcaggc gcaaattact   180
gccgaacgcg aaaaacgcgc ccgtatcgcc gaatccgaag gtcgtaaaat cgaacaaatc   240
aaccttgcca gtggtcagcg cgaagccgaa atccaacaat ccgaaggcga ggctcaggct   300
gcggtcaatg cgtcaaatgc cgagaaaatc gcccgcatca accgcgccaa aggtgaagcg   360
gaatccttgc gccttgttgc cgaagccaat gccgaagcca tccgtcaaat tgccgccgcc   420
cttcaaaccc aaggcggtgc ggatgcggtc aatctgaaga ttgcggaaca atacgtcgct   480
gcgttcaaca atcttgccaa agaaagcaat acgctgatta tgcccgccaa tgttgccgac   540
atcggcagcc tgatttctgc cggtatgaaa attatcgaca gcagcaaaac cgccaaataa   600

<210>   9
<211>   199
<212>   PRT
<213>   Neisseria meningitidis

<400>   9
Ser Val Ile Gly Arg Met Glu Leu Asp Lys Thr Phe Glu Glu Arg Asp
1               5                   10                  15

Glu Ile Asn Ser Thr Val Val Ala Ala Leu Asp Glu Ala Ala Gly Ala
            20                  25                  30

Trp Gly Val Lys Val Leu Arg Tyr Glu Ile Lys Asp Leu Val Pro Pro
        35                  40                  45

Gln Glu Ile Leu Arg Ser Met Gln Ala Gln Ile Thr Ala Glu Arg Glu
    50                  55                  60

Lys Arg Ala Arg Ile Ala Glu Ser Glu Gly Arg Lys Ile Glu Gln Ile
65                  70                  75                  80

Asn Leu Ala Ser Gly Gln Arg Glu Ala Glu Ile Gln Gln Ser Glu Gly
                85                  90                  95

Glu Ala Gln Ala Ala Val Asn Ala Ser Asn Ala Glu Lys Ile Ala Arg
            100                 105                 110

Ile Asn Arg Ala Lys Gly Glu Ala Glu Ser Leu Arg Leu Val Ala Glu
        115                 120                 125
```

Ala Asn Ala Glu Ala Ile Arg Gln Ile Ala Ala Ala Leu Gln Thr Gln
    130                 135                 140

Gly Gly Ala Asp Ala Val Asn Leu Lys Ile Ala Glu Gln Tyr Val Ala
145                 150                 155                 160

Ala Phe Asn Asn Leu Ala Lys Glu Ser Asn Thr Leu Ile Met Pro Ala
                165                 170                 175

Asn Val Ala Asp Ile Gly Ser Leu Ile Ser Ala Gly Met Lys Ile Ile
            180                 185                 190

Asp Ser Ser Lys Thr Ala Lys
        195

<210> 10
<211> 951
<212> DNA
<213> Neisseria gonorrhoeae

<400> 10
atggaatttt tcattatctt gttggcagcc gtcgccgttt tcggcttcaa atcctttgtc 60
gtcatccccc agcaggaagt ccacgttgtc gaaaggctcg ggcgtttcca tcgcgccctg 120
acggccggtt tgaatatttt gattcccttt atcgaccgcg tcgcctaccg ccattcgctg 180
aaagaaatcc ctttagacgt acccagccag gtctgcatca cgcgcgataa tacgcaattg 240
actgttgacg gcatcatcta tttccaagta accgatccca aactcgcctc atacggttcg 300
agcaactaca ttatggcaat tacccagctt gcccaaacga cgctgcgttc cgttatcggg 360
cgtatggagt tggacaaaac gtttgaagaa cgcgacgaaa tcaacagtac cgtcgtctcc 420
gccctcgatg aagccgccgg ggcttggggt gtgaaagtcc tccgttacga aatcaaggat 480
ttggttccgc cgcaagaaat ccttcgcgca atgcaggcac aaattaccgc cgaacgcgaa 540
aaacgcgccc gtattgccga atccgaaggc cgtaaaatcg aacaaatcaa ccttgccagt 600
ggtcagcgtg aagccgaaat ccaacaatcc gaaggcgagg ctcaggctgc ggtcaatgcg 660
tccaatgccg agaaaatcgc ccgcatcaac cgcgccaaag gcgaagcgga atccctgcgc 720
cttgttgccg aagccaatgc cgaagccaac cgtcaaattg ccgccgccct tcaaacccaa 780
agcggggcgg atgcggtcaa tctgaagatt gcgggacaat acgttaccgc gttcaaaaat 840
cttgccaaag aagacaatac gcggattaag cccgccaagg ttgccgaaat cgggaaccct 900
aattttcggc ggcatgaaaa attttcgcca gaagcaaaaa cggccaaata a 951

<210> 11
<211> 316
<212> PRT
<213> Neisseria gonorrhoeae

<400> 11
Met Glu Phe Phe Ile Ile Leu Leu Ala Ala Val Ala Val Phe Gly Phe
1               5                   10                  15

Lys Ser Phe Val Val Ile Pro Gln Gln Glu Val His Val Val Glu Arg
            20                  25                  30

Leu Gly Arg Phe His Arg Ala Leu Thr Ala Gly Leu Asn Ile Leu Ile
        35                  40                  45

Pro Phe Ile Asp Arg Val Ala Tyr Arg His Ser Leu Lys Glu Ile Pro
    50                  55                  60

Leu Asp Val Pro Ser Gln Val Cys Ile Thr Arg Asp Asn Thr Gln Leu
65                  70                  75                  80

Thr Val Asp Gly Ile Ile Tyr Phe Gln Val Thr Asp Pro Lys Leu Ala

```
            85                  90                  95

Ser Tyr Gly Ser Ser Asn Tyr Ile Met Ala Ile Thr Gln Leu Ala Gln
            100                 105                 110

Thr Thr Leu Arg Ser Val Ile Gly Arg Met Glu Leu Asp Lys Thr Phe
        115                 120                 125

Glu Glu Arg Asp Glu Ile Asn Ser Thr Val Val Ser Ala Leu Asp Glu
    130                 135                 140

Ala Ala Gly Ala Trp Gly Val Lys Val Leu Arg Tyr Glu Ile Lys Asp
145                 150                 155                 160

Leu Val Pro Pro Gln Glu Ile Leu Arg Ala Met Gln Ala Gln Ile Thr
                165                 170                 175

Ala Glu Arg Glu Lys Arg Ala Arg Ile Ala Glu Ser Glu Gly Arg Lys
            180                 185                 190

Ile Glu Gln Ile Asn Leu Ala Ser Gly Gln Arg Glu Ala Glu Ile Gln
        195                 200                 205

Gln Ser Glu Gly Glu Ala Gln Ala Ala Val Asn Ala Ser Asn Ala Glu
    210                 215                 220

Lys Ile Ala Arg Ile Asn Arg Ala Lys Gly Glu Ala Glu Ser Leu Arg
225                 230                 235                 240

Leu Val Ala Glu Ala Asn Ala Glu Ala Asn Arg Gln Ile Ala Ala Ala
                245                 250                 255

Leu Gln Thr Gln Ser Gly Ala Asp Ala Val Asn Leu Lys Ile Ala Gly
            260                 265                 270

Gln Tyr Val Thr Ala Phe Lys Asn Leu Ala Lys Glu Asp Asn Thr Arg
        275                 280                 285

Ile Lys Pro Ala Lys Val Ala Glu Ile Gly Asn Pro Asn Phe Arg Arg
    290                 295                 300

His Glu Lys Phe Ser Pro Glu Ala Lys Thr Ala Lys
305                 310                 315
```

<210> 12
<211> 948
<212> DNA
<213> Neisseria meningitidis

<400> 12

```
atggaatttt tcattatctt gctggcagcc gtcgttgttt tcggcttcaa atcctttgtt    60
gtcatcccac agcaggaagt ccacgttgtc gaaaggctcg ggcgtttcca tcgcgccctg   120
acggccggtt tgaatatttt gattcccttt atcgaccgcg tcgcctaccg ccattcgctg   180
aaagaaatcc ctttagacgt acccagccag gtctgcatca cgcgcgacaa tacgcagctg   240
actgttgacg gtatcatcta tttccaagta accgacccca aactcgcctc atacggttcg   300
agcaactaca ttatggcgat tacccagctt gcccaaacga cgctgcgttc cgttatcggg   360
cgtatggaat tggacaaaac gtttgaagaa cgcgacgaaa tcaacagcac cgtcgtctcc   420
gccctcgatg aagccgccgg agcttggggt gtgaaggttt tgcgttatga gattaaagac   480
ttggttccgc cgcaagaaat ccttcgctca atgcaggcgc aaattactgc tgaacgcgaa   540
aaacgcgccc gtatcgccga atccgaaggt cgtaaaatcg aacaaatcaa ccttgccagt   600
ggtcagcgcg aagccgaaat ccaacaatcc gaaggcgagg ctcaggctgc ggtcaatgcg   660
```

```
tcaaatgccg agaaaatcgc ccgcatcaac cgcgccaaag gtgaagcgga atccttgcgc   720
cttgttgccg aagccaatgc cgaagccatc cgtcaaattg ccgccgccct tcaaacccaa   780
ggcggtgcgg atgcggtcaa tctgaagatt gcggaacaat acgtcgccgc gttcaacaat   840
cttgccaaag aaagcaatac gctgattatg cccgccaatg ttgccgacat cggcagcctg   900
atttctgccg gtatgaaaat tatcgacagc agcaaaaccg ccaaataa                948
```

<210> 13
<211> 315
<212> PRT
<213> Neisseria meningitidis

<400> 13

```
Met Glu Phe Phe Ile Ile Leu Leu Ala Ala Val Val Val Phe Gly Phe
1               5                   10                  15

Lys Ser Phe Val Val Ile Pro Gln Gln Glu Val His Val Val Glu Arg
            20                  25                  30

Leu Gly Arg Phe His Arg Ala Leu Thr Ala Gly Leu Asn Ile Leu Ile
        35                  40                  45

Pro Phe Ile Asp Arg Val Ala Tyr Arg His Ser Leu Lys Glu Ile Pro
    50                  55                  60

Leu Asp Val Pro Ser Gln Val Cys Ile Thr Arg Asp Asn Thr Gln Leu
65                  70                  75                  80

Thr Val Asp Gly Ile Ile Tyr Phe Gln Val Thr Asp Pro Lys Leu Ala
                85                  90                  95

Ser Tyr Gly Ser Ser Asn Tyr Ile Met Ala Ile Thr Gln Leu Ala Gln
            100                 105                 110

Thr Thr Leu Arg Ser Val Ile Gly Arg Met Glu Leu Asp Lys Thr Phe
        115                 120                 125

Glu Glu Arg Asp Glu Ile Asn Ser Thr Val Val Ser Ala Leu Asp Glu
    130                 135                 140

Ala Ala Gly Ala Trp Gly Val Lys Val Leu Arg Tyr Glu Ile Lys Asp
145                 150                 155                 160

Leu Val Pro Pro Gln Glu Ile Leu Arg Ser Met Gln Ala Gln Ile Thr
                165                 170                 175

Ala Glu Arg Glu Lys Arg Ala Arg Ile Ala Glu Ser Glu Gly Arg Lys
            180                 185                 190

Ile Glu Gln Ile Asn Leu Ala Ser Gly Gln Arg Glu Ala Glu Ile Gln
        195                 200                 205

Gln Ser Glu Gly Glu Ala Gln Ala Ala Val Asn Ala Ser Asn Ala Glu
    210                 215                 220

Lys Ile Ala Arg Ile Asn Arg Ala Lys Gly Glu Ala Glu Ser Leu Arg
225                 230                 235                 240

Leu Val Ala Glu Ala Asn Ala Glu Ala Ile Arg Gln Ile Ala Ala Ala
                245                 250                 255

Leu Gln Thr Gln Gly Gly Ala Asp Ala Val Asn Leu Lys Ile Ala Glu
```

260 265 270

Gln Tyr Val Ala Ala Phe Asn Asn Leu Ala Lys Glu Ser Asn Thr Leu
275 280 285

Ile Met Pro Ala Asn Val Ala Asp Ile Gly Ser Leu Ile Ser Ala Gly
290 295 300

Met Lys Ile Ile Asp Ser Ser Lys Thr Ala Lys
305 310 315

<210> 14
<211> 948
<212> DNA
<213> Neisseria meningitidis

<400> 14
atggaatttt tcattatctt gttggtagcc gtcgccgttt tcggtttcaa atcctttgtt 60
gtcatcccac aacaggaagt ccacgttgtc gaaaggctgg ggcgtttcca tcgcgccctg 120
acggccggtt tgaatatttt gattcccttt atcgaccgcg tcgcctaccg ccattcgctg 180
aaagaaatcc ctttagacgt acccagccag gtctgcatca cgcgcgacaa tacgcagctg 240
actgttgacg gcatcatcta tttccaagta accgacccca aactcgcctc atacggttcg 300
agcaactaca ttatggcgat tacccagctt gcccaaacga cgctgcgttc cgttatcggg 360
cgtatggagt tggacaaaac gtttgaagaa cgcgacgaaa tcaacagtac tgttgttgcg 420
gctttggacg aggcggccgg ggcttggggt gtgaaggttt tgcgttatga gattaaagac 480
ttggttccgc cgcaagaaat ccttcgctca atgcaggcgc aaattactgc cgaacgcgaa 540
aaacgcgccc gtatcgccga atccgaaggt cgtaaaatcg aacaaatcaa ccttgccagt 600
ggtcagcgcg aagccgaaat ccaacaatcc gaaggcgagg ctcaggctgc ggtcaatgcg 660
tcaaatgccg agaaaatcgc ccgcatcaac cgcgccaaag gtgaagcgga atccttgcgc 720
cttgttgccg aagccaatgc cgaagccatc cgtcaaattg ccgccgccct tcaaacccaa 780
ggcggtgcgg atgcggtcaa tctgaagatt gcggaacaat acgtcgctgc gttcaacaat 840
cttgccaaag aaagcaatac gctgattatg cccgccaatg ttgccgacat cggcagcctg 900
atttctgccg gtatgaaaat tatcgacagc agcaaaaccg ccaaataa 948

<210> 15
<211> 315
<212> PRT
<213> Neisseria meningitidis

<400> 15
Met Glu Phe Phe Ile Ile Leu Leu Val Ala Val Ala Val Phe Gly Phe
1 5 10 15

Lys Ser Phe Val Val Ile Pro Gln Gln Glu Val His Val Val Glu Arg
20 25 30

Leu Gly Arg Phe His Arg Ala Leu Thr Ala Gly Leu Asn Ile Leu Ile
35 40 45

Pro Phe Ile Asp Arg Val Ala Tyr Arg His Ser Leu Lys Glu Ile Pro
50 55 60

Leu Asp Val Pro Ser Gln Val Cys Ile Thr Arg Asp Asn Thr Gln Leu
65 70 75 80

Thr Val Asp Gly Ile Ile Tyr Phe Gln Val Thr Asp Pro Lys Leu Ala
85 90 95

Ser Tyr Gly Ser Ser Asn Tyr Ile Met Ala Ile Thr Gln Leu Ala Gln
100 105 110

```
Thr Thr Leu Arg Ser Val Ile Gly Arg Met Glu Leu Asp Lys Thr Phe
        115                 120                 125

Glu Glu Arg Asp Glu Ile Asn Ser Thr Val Val Ala Ala Leu Asp Glu
    130                 135                 140

Ala Ala Gly Ala Trp Gly Val Lys Val Leu Arg Tyr Glu Ile Lys Asp
145                 150                 155                 160

Leu Val Pro Pro Gln Glu Ile Leu Arg Ser Met Gln Ala Gln Ile Thr
                165                 170                 175

Ala Glu Arg Glu Lys Arg Ala Arg Ile Ala Glu Ser Glu Gly Arg Lys
            180                 185                 190

Ile Glu Gln Ile Asn Leu Ala Ser Gly Gln Arg Glu Ala Glu Ile Gln
        195                 200                 205

Gln Ser Glu Gly Glu Ala Gln Ala Ala Val Asn Ala Ser Asn Ala Glu
    210                 215                 220

Lys Ile Ala Arg Ile Asn Arg Ala Lys Gly Glu Ala Glu Ser Leu Arg
225                 230                 235                 240

Leu Val Ala Glu Ala Asn Ala Glu Ala Ile Arg Gln Ile Ala Ala Ala
                245                 250                 255

Leu Gln Thr Gln Gly Gly Ala Asp Ala Val Asn Leu Lys Ile Ala Glu
            260                 265                 270

Gln Tyr Val Ala Ala Phe Asn Asn Leu Ala Lys Glu Ser Asn Thr Leu
        275                 280                 285

Ile Met Pro Ala Asn Val Ala Asp Ile Gly Ser Leu Ile Ser Ala Gly
    290                 295                 300

Met Lys Ile Ile Asp Ser Ser Lys Thr Ala Lys
305                 310                 315

<210>   16
<211>   948
<212>   DNA
<213>   Neisseria gonorrhoeae

<400>   16
atggaatttt tcattatctt gttggcagcc gtcgccgttt tcggcttcaa atcctttgtc    60
gtcatccccc agcaggaagt ccacgttgtc gaaaggctcg ggcgtttcca tcgcgccctg   120
acggccggtt tgaatatttt gattcccttt atcgaccgcg tcgcctaccg ccattcgctg   180
aaagaaatcc ctttagacgt acccagccag gtctgcatca cgcgcgataa tacgcaattg   240
actgttgacg gcatcatcta tttccaagta accgatccca aactcgcctc atacggttcg   300
agcaactaca ttatggcaat tacccagctt gcccaaacga cgctgcgttc cgttatcggg   360
cgtatggagt tggacaaaac gtttgaagaa cgcgacgaaa tcaacagtac cgtcgtctcc   420
gccctcgatg aagccgccgg ggcttggggt gtgaaagtcc tccgttacga aatcaaggat   480
ttggttccgc cgcaagaaat ccttcgcgca atgcaggcac aaattaccgc cgaacgcgaa   540
aaacgcgccc gtattgccga atccgaaggc cgtaaaatcg aacaaatcaa ccttgccagt   600
ggtcagcgtg aagccgaaat ccaacaatcc gaaggcgagg ctcaggctgc ggtcaatgcg   660
tccaatgccg agaaaatcgc ccgcatcaac cgcgccaaag gcgaagcgga atccctgcgc   720
cttgttgccg aagccaatgc cgaagccatc cgtcaaattg ccgccgccct tcaaacccaa   780
ggcggggcgg atgcggtcaa tctgaagatt gcggaacaat acgtagccgc gttcaacaat   840
cttgccaaag aaagcaatac gctgattatg cccgccaatg ttgccgacat cggcagcctg   900
atttctgccg gcatgaaaat tatcgacagc agcaaaaccg ccaaataa                948
```

```
<210>   17
<211>   315
<212>   PRT
<213>   Neisseria gonorrhoeae

<400>   17
Met Glu Phe Phe Ile Ile Leu Leu Ala Ala Val Ala Val Phe Gly Phe
1               5                   10                  15

Lys Ser Phe Val Val Ile Pro Gln Gln Glu Val His Val Val Glu Arg
            20                  25                  30

Leu Gly Arg Phe His Arg Ala Leu Thr Ala Gly Leu Asn Ile Leu Ile
        35                  40                  45

Pro Phe Ile Asp Arg Val Ala Tyr Arg His Ser Leu Lys Glu Ile Pro
    50                  55                  60

Leu Asp Val Pro Ser Gln Val Cys Ile Thr Arg Asp Asn Thr Gln Leu
65                  70                  75                  80

Thr Val Asp Gly Ile Ile Tyr Phe Gln Val Thr Asp Pro Lys Leu Ala
                85                  90                  95

Ser Tyr Gly Ser Ser Asn Tyr Ile Met Ala Ile Thr Gln Leu Ala Gln
            100                 105                 110

Thr Thr Leu Arg Ser Val Ile Gly Arg Met Glu Leu Asp Lys Thr Phe
        115                 120                 125

Glu Glu Arg Asp Glu Ile Asn Ser Thr Val Val Ser Ala Leu Asp Glu
    130                 135                 140

Ala Ala Gly Ala Trp Gly Val Lys Val Leu Arg Tyr Glu Ile Lys Asp
145                 150                 155                 160

Leu Val Pro Pro Gln Glu Ile Leu Arg Ala Met Gln Ala Gln Ile Thr
                165                 170                 175

Ala Glu Arg Glu Lys Arg Ala Arg Ile Ala Glu Ser Glu Gly Arg Lys
            180                 185                 190

Ile Glu Gln Ile Asn Leu Ala Ser Gly Gln Arg Glu Ala Glu Ile Gln
        195                 200                 205

Gln Ser Glu Gly Glu Ala Gln Ala Ala Val Asn Ala Ser Asn Ala Glu
    210                 215                 220

Lys Ile Ala Arg Ile Asn Arg Ala Lys Gly Glu Ala Glu Ser Leu Arg
225                 230                 235                 240

Leu Val Ala Glu Ala Asn Ala Glu Ala Ile Arg Gln Ile Ala Ala Ala
                245                 250                 255

Leu Gln Thr Gln Gly Gly Ala Asp Ala Val Asn Leu Lys Ile Ala Glu
            260                 265                 270

Gln Tyr Val Ala Ala Phe Asn Asn Leu Ala Lys Glu Ser Asn Thr Leu
        275                 280                 285
```

Ile Met Pro Ala Asn Val Ala Asp Ile Gly Ser Leu Ile Ser Ala Gly
290 295 300

Met Lys Ile Ile Asp Ser Ser Lys Thr Ala Lys
305 310 315

<210> 18
<211> 948
<212> DNA
<213> Neisseria meningitidis

<400> 18
atggaatttt tcattatctt gctggcagcc gtcgttgttt tcggcttcaa atcctttgtt 60
gtcatcccac agcaggaagt ccacgttgtc gaaaggctcg ggcgtttcca tcgcgccctg 120
acggccggtt tgaatatttt gattcccttt atcgaccgcg tcgcctaccg ccattcgctg 180
aaagaaatcc ctttagacgt acccagccag gtctgcatca cgcgcgacaa tacgcagctg 240
actgttgacg gtatcatcta tttccaagta accgacccca aactcgcctc atacggttcg 300
agcaactaca ttatggcgat tacccagctt gcccaaacga cgctgcgttc cgttatcggg 360
cgtatggaat tggacaaaac gtttgaagaa cgcgacgaaa tcaacagcac cgtcgtctcc 420
gccctcgatg aagccgccgg agcttggggt gtgaaggttt tgcgttatga gattaaagac 480
ttggttccgc cgcaagaaat ccttcgctca atgcaggcgc aaattactgc tgaacgcgaa 540
aaacgcgccc gtatcgccga atccgaaggt cgtaaaatcg aacaaatcaa ccttgccagt 600
ggtcagcgcg aagccgaaat ccaacaatcc gaaggcgagg ctcaggctgc ggtcaatgcg 660
tcaaatgccg agaaaatcgc ccgcatcaac cgcgccaaag gtgaagcgga atccttgcgc 720
cttgttgccg aagccaatgc cgaagccatc cgtcaaattg ccgccgccct tcaaacccaa 780
ggcggtgcgg atgcggtcaa tctgaagatt gcggaacaat acgtcgccgc gttcaacaat 840
cttgccaaag aaagcaatac gctgattatg cccgccaatg ttgccgacat cggcagcctg 900
atttctgccg gtatgaaaat tatcgacagc agcaaaaccg ccaaataa 948

<210> 19
<211> 315
<212> PRT
<213> Neisseria meningitidis

<400> 19
Met Glu Phe Phe Ile Ile Leu Leu Ala Ala Val Val Val Phe Gly Phe
1 5 10 15

Lys Ser Phe Val Val Ile Pro Gln Gln Glu Val His Val Val Glu Arg
20 25 30

Leu Gly Arg Phe His Arg Ala Leu Thr Ala Gly Leu Asn Ile Leu Ile
35 40 45

Pro Phe Ile Asp Arg Val Ala Tyr Arg His Ser Leu Lys Glu Ile Pro
50 55 60

Leu Asp Val Pro Ser Gln Val Cys Ile Thr Arg Asp Asn Thr Gln Leu
65 70 75 80

Thr Val Asp Gly Ile Ile Tyr Phe Gln Val Thr Asp Pro Lys Leu Ala
85 90 95

Ser Tyr Gly Ser Ser Asn Tyr Ile Met Ala Ile Thr Gln Leu Ala Gln
100 105 110

Thr Thr Leu Arg Ser Val Ile Gly Arg Met Glu Leu Asp Lys Thr Phe
115 120 125

Glu Glu Arg Asp Glu Ile Asn Ser Thr Val Val Ser Ala Leu Asp Glu
130 135 140

```
Ala Ala Gly Ala Trp Gly Val Lys Val Leu Arg Tyr Glu Ile Lys Asp
145                 150                 155                 160

Leu Val Pro Pro Gln Glu Ile Leu Arg Ser Met Gln Ala Gln Ile Thr
                165                 170                 175

Ala Glu Arg Glu Lys Arg Ala Arg Ile Ala Glu Ser Glu Gly Arg Lys
            180                 185                 190

Ile Glu Gln Ile Asn Leu Ala Ser Gly Gln Arg Glu Ala Glu Ile Gln
        195                 200                 205

Gln Ser Glu Gly Glu Ala Gln Ala Ala Val Asn Ala Ser Asn Ala Glu
    210                 215                 220

Lys Ile Ala Arg Ile Asn Arg Ala Lys Gly Glu Ala Glu Ser Leu Arg
225                 230                 235                 240

Leu Val Ala Glu Ala Asn Ala Glu Ala Ile Arg Gln Ile Ala Ala Ala
                245                 250                 255

Leu Gln Thr Gln Gly Gly Ala Asp Ala Val Asn Leu Lys Ile Ala Glu
            260                 265                 270

Gln Tyr Val Ala Ala Phe Asn Asn Leu Ala Lys Glu Ser Asn Thr Leu
        275                 280                 285

Ile Met Pro Ala Asn Val Ala Asp Ile Gly Ser Leu Ile Ser Ala Gly
    290                 295                 300

Met Lys Ile Ile Asp Ser Ser Lys Thr Ala Lys
305                 310                 315

<210>   20
<211>   669
<212>   DNA
<213>   Neisseria meningitidis

<400>   20
atgcagcagg caagctatgc gatgggcgtg gacatcggac gctccctgaa gcaaatgaag   60
gaacagggcg cggaaatcga tttgaaagtc tttaccgaag ccatgcaggc agtgtatgac   120
ggcaaagaaa tcaaaatgac cgaagagcag gctcaggaag tcatgatgaa attccttcag   180
gaacaacagg ctaaagccgt agaaaaacac aaggcggacg cgaaggccaa taaagaaaaa   240
ggcgaagcct ttctgaaaga aaatgccgcc aaagacggcg tgaagaccac tgcttccggc   300
ctgcaataca aaatcaccaa acagggcgaa ggcaaacagc cgaccaaaga cgacatcgtt   360
accgtggaat acgaaggccg cctgattgac ggtacggtat tcgacagcag caaagccaac   420
ggcggcccgg tcaccttccc tttgagccaa gtgattccgg gttggaccga aggcgtacag   480
cttctgaaag aaggcggcga agccacgttc tacatcccgt ccaaccttgc ctaccgcgaa   540
cagggtgcgg gcgacaaaat cggtccgaac gccactttgg tatttgatgt gaaactggtc   600
aaaatcggcg cacccgaaaa cgcgcccgcc aagcagccgg ctcaagtcga catcaaaaaa   660
gtaaattaa                                                           669

<210>   21
<211>   222
<212>   PRT
<213>   Neisseria meningitidis

<400>   21
Met Gln Gln Ala Ser Tyr Ala Met Gly Val Asp Ile Gly Arg Ser Leu
1               5                   10                  15
```

Lys Gln Met Lys Glu Gln Gly Ala Glu Ile Asp Leu Lys Val Phe Thr
20 25 30

Glu Ala Met Gln Ala Val Tyr Asp Gly Lys Glu Ile Lys Met Thr Glu
35 40 45

Glu Gln Ala Gln Glu Val Met Met Lys Phe Leu Gln Glu Gln Gln Ala
50 55 60

Lys Ala Val Glu Lys His Lys Ala Asp Ala Lys Ala Asn Lys Glu Lys
65 70 75 80

Gly Glu Ala Phe Leu Lys Glu Asn Ala Ala Lys Asp Gly Val Lys Thr
85 90 95

Thr Ala Ser Gly Leu Gln Tyr Lys Ile Thr Lys Gln Gly Glu Gly Lys
100 105 110

Gln Pro Thr Lys Asp Asp Ile Val Thr Val Glu Tyr Glu Gly Arg Leu
115 120 125

Ile Asp Gly Thr Val Phe Asp Ser Ser Lys Ala Asn Gly Gly Pro Val
130 135 140

Thr Phe Pro Leu Ser Gln Val Ile Pro Gly Trp Thr Glu Gly Val Gln
145 150 155 160

Leu Leu Lys Glu Gly Gly Glu Ala Thr Phe Tyr Ile Pro Ser Asn Leu
165 170 175

Ala Tyr Arg Glu Gln Gly Ala Gly Asp Lys Ile Gly Pro Asn Ala Thr
180 185 190

Leu Val Phe Asp Val Lys Leu Val Lys Ile Gly Ala Pro Glu Asn Ala
195 200 205

Pro Ala Lys Gln Pro Ala Gln Val Asp Ile Lys Lys Val Asn
210 215 220

<210> 22
<211> 648
<212> DNA
<213> Neisseria gonorrhoeae

<400> 22
atgggcgtgg acatcggacg ctccctgaaa caaatgaagg aacagggcgc ggaaatcgat 60
ttgaaagtct ttaccgatgc catgcaggca gtgtatgacg gcaaagaaat caaaatgacc 120
gaagagcagg cccaggaagt gatgatgaaa ttcctgcagg agcagcaggc taaagccgta 180
gaaaaacaca aggcggatgc gaaggccaac aaagaaaaag gcgaagcctt cctgaaggaa 240
aatgccgccg aagacggcgt gaagaccact gcttccggtc tgcagtacaa aatcaccaaa 300
cagggtgaag gcaaacagcc gacaaaagac gacatcgtta ccgtggaata cgaaggccgc 360
ctgattgacg gtaccgtatt cgacagcagc aaagccaacg gcggcccggc caccttccct 420
ttgagccaag tgattccggg ttggaccgaa ggcgtacggc ttctgaaaga aggcggcgaa 480
gccacgttct acatcccgtc caaccttgcc taccgcgaac agggtgcggg cgaaaaaatc 540
ggtccgaacg ccactttggt atttgacgtg aaactggtca aaatcggcgc acccgaaaac 600
gcgcccgcca agcagccgga tcaagtcgac atcaaaaaag taaattaa 648

<210> 23
<211> 215
<212> PRT

<213> Neisseria gonorrhoeae

<400> 23

```
Met Gly Val Asp Ile Gly Arg Ser Leu Lys Gln Met Lys Glu Gln Gly
1               5                   10                  15

Ala Glu Ile Asp Leu Lys Val Phe Thr Asp Ala Met Gln Ala Val Tyr
            20                  25                  30

Asp Gly Lys Glu Ile Lys Met Thr Glu Glu Gln Ala Gln Glu Val Met
        35                  40                  45

Met Lys Phe Leu Gln Glu Gln Gln Ala Lys Ala Val Glu Lys His Lys
    50                  55                  60

Ala Asp Ala Lys Ala Asn Lys Glu Lys Gly Glu Ala Phe Leu Lys Glu
65                  70                  75                  80

Asn Ala Ala Glu Asp Gly Val Lys Thr Thr Ala Ser Gly Leu Gln Tyr
                85                  90                  95

Lys Ile Thr Lys Gln Gly Glu Gly Lys Gln Pro Thr Lys Asp Asp Ile
            100                 105                 110

Val Thr Val Glu Tyr Glu Gly Arg Leu Ile Asp Gly Thr Val Phe Asp
        115                 120                 125

Ser Ser Lys Ala Asn Gly Gly Pro Ala Thr Phe Pro Leu Ser Gln Val
    130                 135                 140

Ile Pro Gly Trp Thr Glu Gly Val Arg Leu Leu Lys Glu Gly Gly Glu
145                 150                 155                 160

Ala Thr Phe Tyr Ile Pro Ser Asn Leu Ala Tyr Arg Glu Gln Gly Ala
                165                 170                 175

Gly Glu Lys Ile Gly Pro Asn Ala Thr Leu Val Phe Asp Val Lys Leu
            180                 185                 190

Val Lys Ile Gly Ala Pro Glu Asn Ala Pro Ala Lys Gln Pro Asp Gln
        195                 200                 205

Val Asp Ile Lys Lys Val Asn
    210                 215
```

<210> 24
<211> 819
<212> DNA
<213> Neisseria meningitidis

<400> 24

```
atgaacacca ttttcaaaat cagcgcactg accctttccg ccgctttggc actttccgcc    60
tgcggcaaaa aagaagccgc ccccgcatct gcatccgaac ctgccgccgc ttcttccgcg   120
cagggcgaca cctcttcgat cggcagcacg atgcagcagg caagctatgc gatgggcgtg   180
gacatcggac gctccctgaa gcaaatgaag gaacagggcg cggaaatcga tttgaaagtc   240
tttaccgaag ccatgcaggc agtgtatgac ggcaaagaaa tcaaaatgac cgaagagcag   300
gctcaggaag tcatgatgaa attccttcag gaacaacagg ctaaagccgt agaaaaacac   360
aaggcggacg cgaaggccaa taaagaaaaa ggcgaagcct ttctgaaaga aaatgccgcc   420
aaagacggcg tgaagaccac tgcttccggc ctgcaataca aaatcaccaa acagggcgaa   480
ggcaaacagc cgaccaaaga cgacatcgtt accgtggaat acgaaggccg cctgattgac   540
ggtacggtat tcgacagcag caaagccaac ggcggcccgg tcaccttccc tttgagccaa   600
```

```
gtgattctgg gttggaccga aggcgtacag cttctgaaag aaggcggcga agccacgttc   660
tacatcccgt ccaaccttgc ctaccgcgaa cagggtgcgg gcgacaaaat cggcccgaac   720
gccactttgg tatttgatgt gaaactggtc aaaatcggcg cacccgaaaa cgcgcccgcc   780
aagcagccgg ctcaagtcga catcaaaaaa gtaaattaa                          819

<210>   25
<211>   272
<212>   PRT
<213>   Neisseria meningitidis

<400>   25
Met Asn Thr Ile Phe Lys Ile Ser Ala Leu Thr Leu Ser Ala Ala Leu
1               5                   10                  15

Ala Leu Ser Ala Cys Gly Lys Lys Glu Ala Ala Pro Ala Ser Ala Ser
            20                  25                  30

Glu Pro Ala Ala Ala Ser Ser Ala Gln Gly Asp Thr Ser Ser Ile Gly
        35                  40                  45

Ser Thr Met Gln Gln Ala Ser Tyr Ala Met Gly Val Asp Ile Gly Arg
    50                  55                  60

Ser Leu Lys Gln Met Lys Glu Gln Gly Ala Glu Ile Asp Leu Lys Val
65                  70                  75                  80

Phe Thr Glu Ala Met Gln Ala Val Tyr Asp Gly Lys Glu Ile Lys Met
                85                  90                  95

Thr Glu Glu Gln Ala Gln Glu Val Met Met Lys Phe Leu Gln Glu Gln
            100                 105                 110

Gln Ala Lys Ala Val Glu Lys His Lys Ala Asp Ala Lys Ala Asn Lys
        115                 120                 125

Glu Lys Gly Glu Ala Phe Leu Lys Glu Asn Ala Ala Lys Asp Gly Val
    130                 135                 140

Lys Thr Thr Ala Ser Gly Leu Gln Tyr Lys Ile Thr Lys Gln Gly Glu
145                 150                 155                 160

Gly Lys Gln Pro Thr Lys Asp Asp Ile Val Thr Val Glu Tyr Glu Gly
                165                 170                 175

Arg Leu Ile Asp Gly Thr Val Phe Asp Ser Ser Lys Ala Asn Gly Gly
            180                 185                 190

Pro Val Thr Phe Pro Leu Ser Gln Val Ile Leu Gly Trp Thr Glu Gly
        195                 200                 205

Val Gln Leu Leu Lys Glu Gly Gly Glu Ala Thr Phe Tyr Ile Pro Ser
    210                 215                 220

Asn Leu Ala Tyr Arg Glu Gln Gly Ala Gly Asp Lys Ile Gly Pro Asn
225                 230                 235                 240

Ala Thr Leu Val Phe Asp Val Lys Leu Val Lys Ile Gly Ala Pro Glu
                245                 250                 255

Asn Ala Pro Ala Lys Gln Pro Ala Gln Val Asp Ile Lys Lys Val Asn
            260                 265                 270
```

<210> 26
<211> 819
<212> DNA
<213> Neisseria meningitidis

<400> 26
atgaacacca ttttcaaaat cagcgcactg accctttccg ccgctttggc actttccgcc 60
tgcggcaaaa aagaagccgc cccgcatct gcatccgaac ctgccgccgc ttcttccgcg 120
cagggcgaca cctcttcgat cggcagcacg atgcagcagg caagctatgc gatgggcgtg 180
gacatcggac gctccctgaa gcaaatgaag gaacagggcg cggaaatcga tttgaaagtc 240
tttaccgaag ccatgcaggc agtgtatgac ggcaaagaaa tcaaaatgac cgaagagcag 300
gctcaggaag tcatgatgaa attccttcag gaacaacagg ctaaagccgt agaaaaacac 360
aaggcggacg cgaaggccaa taaagaaaaa ggcgaagcct ttctgaaaga aaatgccgcc 420
aaagacggcg tgaagaccac tgcttccggc ctgcaataca aaatcaccaa acagggcgaa 480
ggcaaacagc cgaccaaaga cgacatcgtt accgtggaat acgaaggccg cctgattgac 540
ggtacggtat tcgacagcag caaagccaac ggcggcccgg tcaccttccc tttgagccaa 600
gtgattccgg gttggaccga aggcgtacag cttctgaaag aaggcggcga agccacgttc 660
tacatcccgt ccaaccttgc ctaccgcgaa cagggtgcgg gcgacaaaat cggtccgaac 720
gccactttgg tatttgatgt gaaactggtc aaaatcggcg cacccgaaaa cgcgcccgcc 780
aagcagccgg ctcaagtcga catcaaaaaa gtaaattaa 819

<210> 27
<211> 272
<212> PRT
<213> Neisseria meningitidis

<400> 27
Met Asn Thr Ile Phe Lys Ile Ser Ala Leu Thr Leu Ser Ala Ala Leu
1 5 10 15

Ala Leu Ser Ala Cys Gly Lys Lys Glu Ala Ala Pro Ala Ser Ala Ser
20 25 30

Glu Pro Ala Ala Ala Ser Ser Ala Gln Gly Asp Thr Ser Ser Ile Gly
35 40 45

Ser Thr Met Gln Gln Ala Ser Tyr Ala Met Gly Val Asp Ile Gly Arg
50 55 60

Ser Leu Lys Gln Met Lys Glu Gln Gly Ala Glu Ile Asp Leu Lys Val
65 70 75 80

Phe Thr Glu Ala Met Gln Ala Val Tyr Asp Gly Lys Glu Ile Lys Met
85 90 95

Thr Glu Glu Gln Ala Gln Glu Val Met Met Lys Phe Leu Gln Glu Gln
100 105 110

Gln Ala Lys Ala Val Glu Lys His Lys Ala Asp Ala Lys Ala Asn Lys
115 120 125

Glu Lys Gly Glu Ala Phe Leu Lys Glu Asn Ala Ala Lys Asp Gly Val
130 135 140

Lys Thr Thr Ala Ser Gly Leu Gln Tyr Lys Ile Thr Lys Gln Gly Glu
145 150 155 160

Gly Lys Gln Pro Thr Lys Asp Asp Ile Val Thr Val Glu Tyr Glu Gly
165 170 175

```
Arg Leu Ile Asp Gly Thr Val Phe Asp Ser Ser Lys Ala Asn Gly Gly
            180                 185                 190

Pro Val Thr Phe Pro Leu Ser Gln Val Ile Pro Gly Trp Thr Glu Gly
        195                 200                 205

Val Gln Leu Leu Lys Glu Gly Gly Glu Ala Thr Phe Tyr Ile Pro Ser
    210                 215                 220

Asn Leu Ala Tyr Arg Glu Gln Gly Ala Gly Asp Lys Ile Gly Pro Asn
225                 230                 235                 240

Ala Thr Leu Val Phe Asp Val Lys Leu Val Lys Ile Gly Ala Pro Glu
                245                 250                 255

Asn Ala Pro Ala Lys Gln Pro Ala Gln Val Asp Ile Lys Lys Val Asn
            260                 265                 270


<210>   28
<211>   819
<212>   DNA
<213>   Neisseria gonorrhoeae

<400>   28
atgaacacca ttttcaaaat cagcgcactg accctttccg ccgctttggc actttccgcc    60
tgcggcaaaa aagaagccgc ccccgcatct gcatccgaac ctgccgccgc ttctgccgcg   120
cagggcgaca cctcttcaat cggcagcacg atgcagcagg caagctatgc aatgggcgtg   180
gacatcggac gctccctgaa acaaatgaag gaacagggcg cggaaatcga tttgaaagtc   240
tttaccgatg ccatgcaggc agtgtatgac ggcaaagaaa tcaaaatgac cgaagagcag   300
gcccaggaag tgatgatgaa attcctgcag gagcagcagg ctaaagccgt agaaaaacac   360
aaggcggatg cgaaggccaa caaagaaaaa ggcgaagcct tcctgaagga aaatgccgcc   420
aaagacggcg tgaagaccac tgcttccggt ctgcagtaca aaatcaccaa acagggtgaa   480
ggcaaacagc cgacaaaaga cgacatcgtt accgtggaat acgaaggccg cctgattgac   540
ggtaccgtat tcgacagcag caaagccaac ggcggcccgg ccaccttccc tttgagccaa   600
gtgattccgg gttggaccga aggcgtacgg cttctgaaag aaggcggcga agccacgttc   660
tacatcccgt ccaaccttgc ctaccgcgaa cagggtgcgg gcgaaaaaat cggtccgaac   720
gccactttgg tatttgacgt gaaactggtc aaaatcggcg cacccgaaaa cgcgcccgcc   780
aagcagccgg atcaagtcga catcaaaaaa gtaaattaa                          819

<210>   29
<211>   272
<212>   PRT
<213>   Neisseria gonorrhoeae

<400>   29
Met Asn Thr Ile Phe Lys Ile Ser Ala Leu Thr Leu Ser Ala Ala Leu
1               5                   10                  15

Ala Leu Ser Ala Cys Gly Lys Lys Glu Ala Ala Pro Ala Ser Ala Ser
            20                  25                  30

Glu Pro Ala Ala Ala Ser Ala Ala Gln Gly Asp Thr Ser Ser Ile Gly
        35                  40                  45

Ser Thr Met Gln Gln Ala Ser Tyr Ala Met Gly Val Asp Ile Gly Arg
    50                  55                  60

Ser Leu Lys Gln Met Lys Glu Gln Gly Ala Glu Ile Asp Leu Lys Val
65                  70                  75                  80
```

```
Phe Thr Asp Ala Met Gln Ala Val Tyr Asp Gly Lys Glu Ile Lys Met
                85                  90                  95

Thr Glu Glu Gln Ala Gln Glu Val Met Met Lys Phe Leu Gln Glu Gln
            100                 105                 110

Gln Ala Lys Ala Val Glu Lys His Lys Ala Asp Ala Lys Ala Asn Lys
        115                 120                 125

Glu Lys Gly Glu Ala Phe Leu Lys Glu Asn Ala Ala Lys Asp Gly Val
    130                 135                 140

Lys Thr Thr Ala Ser Gly Leu Gln Tyr Lys Ile Thr Lys Gln Gly Glu
145                 150                 155                 160

Gly Lys Gln Pro Thr Lys Asp Asp Ile Val Thr Val Glu Tyr Glu Gly
                165                 170                 175

Arg Leu Ile Asp Gly Thr Val Phe Asp Ser Ser Lys Ala Asn Gly Gly
            180                 185                 190

Pro Ala Thr Phe Pro Leu Ser Gln Val Ile Pro Gly Trp Thr Glu Gly
        195                 200                 205

Val Arg Leu Leu Lys Glu Gly Gly Glu Ala Thr Phe Tyr Ile Pro Ser
    210                 215                 220

Asn Leu Ala Tyr Arg Glu Gln Gly Ala Gly Glu Lys Ile Gly Pro Asn
225                 230                 235                 240

Ala Thr Leu Val Phe Asp Val Lys Leu Val Lys Ile Gly Ala Pro Glu
                245                 250                 255

Asn Ala Pro Ala Lys Gln Pro Asp Gln Val Asp Ile Lys Lys Val Asn
            260                 265                 270


<210>   30
<211>   819
<212>   DNA
<213>   Neisseria meningitidis

<400>   30
atgaacacca ttttcaaaat cagcgcactg accctttccg ccgctttggc actttccgcc   60
tgcggcaaaa aagaagccgc ccccgcatct gcatccgaac ctgccgccgc ttcttccgcg   120
cagggcgaca cctcttcgat cggcagcacg atgcagcagg caagctatgc gatgggcgtg   180
gacatcggac gctccctgaa gcaaatgaag gaacagggcg cggaaatcga tttgaaagtc   240
tttaccgaag ccatgcaggc agtgtatgac ggcaaagaaa tcaaaatgac cgaagagcag   300
gctcaggaag tcatgatgaa attccttcag gaacaacagg ctaaagccgt agaaaaacac   360
aaggcggacg cgaaggccaa taaagaaaaa ggcgaagcct ttctgaaaga aaatgccgcc   420
aaagacggcg tgaagaccac tgcttccggc ctgcaataca aaatcaccaa acagggcgaa   480
ggcaaacagc cgaccaaaga cgacatcgtt accgtggaat acgaaggccg cctgattgac   540
ggtacggtat tcgacagcag caaagccaac ggcggcccgg tcaccttccc tttgagccaa   600
gtgattctgg gttggaccga aggcgtacag cttctgaaag aaggcggcga agccacgttc   660
tacatcccgt ccaaccttgc ctaccgcgaa cagggtgcgg gcgacaaaat cggcccgaac   720
gccactttgg tatttgatgt gaaactggtc aaaatcggcg cacccgaaaa cgcgcccgcc   780
aagcagccgg ctcaagtcga catcaaaaaa gtaaattaa                          819

<210>   31
<211>   272
```

<212> PRT
<213> Neisseria meningitidis

<400> 31
Met Asn Thr Ile Phe Lys Ile Ser Ala Leu Thr Leu Ser Ala Ala Leu
1 5 10 15

Ala Leu Ser Ala Cys Gly Lys Lys Glu Ala Ala Pro Ala Ser Ala Ser
20 25 30

Glu Pro Ala Ala Ala Ser Ser Ala Gln Gly Asp Thr Ser Ser Ile Gly
35 40 45

Ser Thr Met Gln Gln Ala Ser Tyr Ala Met Gly Val Asp Ile Gly Arg
50 55 60

Ser Leu Lys Gln Met Lys Glu Gln Gly Ala Glu Ile Asp Leu Lys Val
65 70 75 80

Phe Thr Glu Ala Met Gln Ala Val Tyr Asp Gly Lys Glu Ile Lys Met
85 90 95

Thr Glu Glu Gln Ala Gln Glu Val Met Met Lys Phe Leu Gln Glu Gln
100 105 110

Gln Ala Lys Ala Val Glu Lys His Lys Ala Asp Ala Lys Ala Asn Lys
115 120 125

Glu Lys Gly Glu Ala Phe Leu Lys Glu Asn Ala Ala Lys Asp Gly Val
130 135 140

Lys Thr Thr Ala Ser Gly Leu Gln Tyr Lys Ile Thr Lys Gln Gly Glu
145 150 155 160

Gly Lys Gln Pro Thr Lys Asp Asp Ile Val Thr Val Glu Tyr Glu Gly
165 170 175

Arg Leu Ile Asp Gly Thr Val Phe Asp Ser Ser Lys Ala Asn Gly Gly
180 185 190

Pro Val Thr Phe Pro Leu Ser Gln Val Ile Leu Gly Trp Thr Glu Gly
195 200 205

Val Gln Leu Leu Lys Glu Gly Gly Glu Ala Thr Phe Tyr Ile Pro Ser
210 215 220

Asn Leu Ala Tyr Arg Glu Gln Gly Ala Gly Asp Lys Ile Gly Pro Asn
225 230 235 240

Ala Thr Leu Val Phe Asp Val Lys Leu Val Lys Ile Gly Ala Pro Glu
245 250 255

Asn Ala Pro Ala Lys Gln Pro Ala Gln Val Asp Ile Lys Lys Val Asn
260 265 270

<210> 32
<211> 1326
<212> DNA
<213> Neisseria meningitidis

<400> 32
atgaaaaaat acctattccg cgccgccctg tacggcatcg ccgccgccat cctcgccgcc 60
tgccaaagca agagcatcca aacctttccg caacccgaca catccgtcat caacggcccg 120
gaccggccgg tcggcatccc cgacccgcc ggaacgacgg tcggcggcgg cggggccgtc 180
tataccgttg taccgcacct gtccctgccc cactgggcgg cgcaggattt cgccaaaagc 240
ctgcaatcct tccgcctcgg ctgcgccaat ttgaaaaacc gccaaggctg gcaggatgtg 300
tgcgcccaag cctttcaaac ccccgtccat tcctttcagg caaaacagtt ttttgaacgc 360
tatttcacgc cgtggcaggt tgcaggcaac ggaagccttg ccggtacggt taccggctat 420
tacgaaccgg tgctgaaggg cgacgacagg cggacggcac aagcccgctt cccgatttac 480
ggtattcccg acgattttat ctccgtcccc ctgcctgccg gtttgcggag cggaaaagcc 540
cttgtccgca tcaggcagac gggaaaaaac agcggcacaa tcgacaatac cggcggcaca 600
cataccgccg acctctcccg attccccatc accgcgcgca caacagcaat caaaggcagg 660
tttgaaggaa gccgcttcct cccctaccac acgcgcaacc aaatcaacgg cggcgcgctt 720
gacggcaaag ccccgatact cggttacgcc gaagaccctg tcgaactttt ttttatgcac 780
atccaaggct cgggccgtct gaaaaccccg tccggcaaat acatccgcat cggctatgcc 840
gacaaaaacg aacatccyta cgtttccatc ggacgctata tggcggataa gggctacctc 900
aaactcggac aaacctccat gcagggcatt aagtcttata tgcggcaaaa tccgcaacgc 960
ctcgccgaag ttttgggtca aaaccccagc tatatctttt tccgcgagct tgccggaagc 1020
agcaatgacg gccctgtcgg cgcactgggc acgccgctga tggggggaata tgccggcgca 1080
gtcgaccggc actacattac cttgggtgcg cccttatttg tcgccaccgc ccatccggtt 1140
acccgcaaag ccctcaaccg cctgattatg gcgcaggata ccggcagcgc gattaaaggc 1200
gcggtgcgcg tggattattt ttggggatac ggcgacgaag ccggcgaact tgccggcaaa 1260
cagaaaacca cgggatatgt ctggcagctc ctacccaacg gtatgaagcc cgaataccgc 1320
ccgtaa 1326

<210> 33
<211> 441
<212> PRT
<213> Neisseria meningitidis

<400> 33
Met Lys Lys Tyr Leu Phe Arg Ala Ala Leu Tyr Gly Ile Ala Ala Ala
1 5 10 15

Ile Leu Ala Ala Cys Gln Ser Lys Ser Ile Gln Thr Phe Pro Gln Pro
20 25 30

Asp Thr Ser Val Ile Asn Gly Pro Asp Arg Pro Val Gly Ile Pro Asp
35 40 45

Pro Ala Gly Thr Thr Val Gly Gly Gly Gly Ala Val Tyr Thr Val Val
50 55 60

Pro His Leu Ser Leu Pro His Trp Ala Ala Gln Asp Phe Ala Lys Ser
65 70 75 80

Leu Gln Ser Phe Arg Leu Gly Cys Ala Asn Leu Lys Asn Arg Gln Gly
85 90 95

Trp Gln Asp Val Cys Ala Gln Ala Phe Gln Thr Pro Val His Ser Phe
100 105 110

Gln Ala Lys Gln Phe Phe Glu Arg Tyr Phe Thr Pro Trp Gln Val Ala
115 120 125

Gly Asn Gly Ser Leu Ala Gly Thr Val Thr Gly Tyr Tyr Glu Pro Val
130 135 140

Leu Lys Gly Asp Asp Arg Arg Thr Ala Gln Ala Arg Phe Pro Ile Tyr
145 150 155 160

```
Gly Ile Pro Asp Asp Phe Ile Ser Val Pro Leu Pro Ala Gly Leu Arg
                165                 170                 175

Ser Gly Lys Ala Leu Val Arg Ile Arg Gln Thr Gly Lys Asn Ser Gly
            180                 185                 190

Thr Ile Asp Asn Thr Gly Gly Thr His Thr Ala Asp Leu Ser Arg Phe
        195                 200                 205

Pro Ile Thr Ala Arg Thr Thr Ala Ile Lys Gly Arg Phe Glu Gly Ser
    210                 215                 220

Arg Phe Leu Pro Tyr His Thr Arg Asn Gln Ile Asn Gly Gly Ala Leu
225                 230                 235                 240

Asp Gly Lys Ala Pro Ile Leu Gly Tyr Ala Glu Asp Pro Val Glu Leu
                245                 250                 255

Phe Phe Met His Ile Gln Gly Ser Gly Arg Leu Lys Thr Pro Ser Gly
            260                 265                 270

Lys Tyr Ile Arg Ile Gly Tyr Ala Asp Lys Asn Glu His Pro Tyr Val
        275                 280                 285

Ser Ile Gly Arg Tyr Met Ala Asp Lys Gly Tyr Leu Lys Leu Gly Gln
    290                 295                 300

Thr Ser Met Gln Gly Ile Lys Ser Tyr Met Arg Gln Asn Pro Gln Arg
305                 310                 315                 320

Leu Ala Glu Val Leu Gly Gln Asn Pro Ser Tyr Ile Phe Phe Arg Glu
                325                 330                 335

Leu Ala Gly Ser Ser Asn Asp Gly Pro Val Gly Ala Leu Gly Thr Pro
            340                 345                 350

Leu Met Gly Glu Tyr Ala Gly Ala Val Asp Arg His Tyr Ile Thr Leu
        355                 360                 365

Gly Ala Pro Leu Phe Val Ala Thr Ala His Pro Val Thr Arg Lys Ala
    370                 375                 380

Leu Asn Arg Leu Ile Met Ala Gln Asp Thr Gly Ser Ala Ile Lys Gly
385                 390                 395                 400

Ala Val Arg Val Asp Tyr Phe Trp Gly Tyr Gly Asp Glu Ala Gly Glu
                405                 410                 415

Leu Ala Gly Lys Gln Lys Thr Thr Gly Tyr Val Trp Gln Leu Leu Pro
            420                 425                 430

Asn Gly Met Lys Pro Glu Tyr Arg Pro
        435                 440

<210>   34
<211>   1326
<212>   DNA
<213>   Neisseria meningitidis

<400>   34
atgaaaaaat acctattccg cgccgccctg tacggcatcg ccgccgccat cctcgccgcc   60
```

```
tgccaaagca agagcatcca aacctttccg caacccgaca catccgtcat caacggcccg   120
gaccggccgg tcggcatccc cgaccccgcc ggaacgacgg tcggcggcgg cggggccgtc   180
tataccgttg taccgcacct gtccctgccc cactgggcgg cgcaggattt cgccaaaagc   240
ctgcaatcct tccgcctcgg ctgcgccaat ttgaaaaacc gccaaggctg gcaggatgtg   300
tgcgcccaag cctttcaaac cccgtccat tcctttcagg caaaacagtt ttttgaacgc   360
tatttcacgc cgtggcaggt tgcaggcaac ggaagccttg ccggtacggt taccggctat   420
tacgaaccgg tgctgaaggg cgacgacagg cggacggcac aagcccgctt cccgatttac   480
ggtattcccg acgattttat ctccgtcccc ctgcctgccg gtttgcggag cggaaaagcc   540
cttgtccgca tcaggcagac gggaaaaaac agcggcacaa tcgacaatac cggcggcaca   600
cataccgccg acctctcccg attccccatc accgcgcgca caacagcaat caaaggcagg   660
tttgaaggaa gccgcttcct cccctaccac acgcgcaacc aaatcaacgg cggcgcgctt   720
gacggcaaag ccccgatact cggttacgcc gaagaccctg tcgaactttt ttttatgcac   780
atccaaggct cgggccgtct gaaaaccccg tccggcaaat acatccgcat cggctatgcc   840
gacaaaaacg aacatcccta cgtttccatc ggacgctata tggcggataa gggctacctc   900
aaactcggac aaacctccat gcagggcatt aagtcttata tgcggcaaaa tccgcaacgc   960
ctcgccgaag ttttgggtca aaaccccagc tatatctttt tccgcgagct tgccggaagc   1020
agcaatgacg gccctgtcgg cgcactgggc acgccgctga tgggggaata tgccggcgca   1080
gtcgaccggc actacattac cttgggtgcg cccttatttg tcgccaccgc ccatccggtt   1140
acccgcaaag ccctcaaccg cctgattatg gcgcaggata ccggcagcgc gattaaaggc   1200
gcggtgcgcg tggattattt ttggggatac ggcgacgaag ccggcgaact tgccggcaaa   1260
cagaaaacca cgggatatgt ctggcagctc ctacccaacg gtatgaagcc cgaataccgc   1320
ccgtaa                                                              1326
```

<210> 35
<211> 441
<212> PRT
<213> Neisseria meningitidis

<400> 35

```
Met Lys Lys Tyr Leu Phe Arg Ala Ala Leu Tyr Gly Ile Ala Ala Ala
1               5                   10                  15

Ile Leu Ala Ala Cys Gln Ser Lys Ser Ile Gln Thr Phe Pro Gln Pro
            20                  25                  30

Asp Thr Ser Val Ile Asn Gly Pro Asp Arg Pro Val Gly Ile Pro Asp
        35                  40                  45

Pro Ala Gly Thr Thr Val Gly Gly Gly Gly Ala Val Tyr Thr Val Val
    50                  55                  60

Pro His Leu Ser Leu Pro His Trp Ala Ala Gln Asp Phe Ala Lys Ser
65                  70                  75                  80

Leu Gln Ser Phe Arg Leu Gly Cys Ala Asn Leu Lys Asn Arg Gln Gly
                85                  90                  95

Trp Gln Asp Val Cys Ala Gln Ala Phe Gln Thr Pro Val His Ser Phe
            100                 105                 110

Gln Ala Lys Gln Phe Phe Glu Arg Tyr Phe Thr Pro Trp Gln Val Ala
        115                 120                 125

Gly Asn Gly Ser Leu Ala Gly Thr Val Thr Gly Tyr Tyr Glu Pro Val
    130                 135                 140

Leu Lys Gly Asp Asp Arg Arg Thr Ala Gln Ala Arg Phe Pro Ile Tyr
145                 150                 155                 160

Gly Ile Pro Asp Asp Phe Ile Ser Val Pro Leu Pro Ala Gly Leu Arg
                165                 170                 175
```

```
Ser Gly Lys Ala Leu Val Arg Ile Arg Gln Thr Gly Lys Asn Ser Gly
            180                 185                 190

Thr Ile Asp Asn Thr Gly Gly Thr His Thr Ala Asp Leu Ser Arg Phe
        195                 200                 205

Pro Ile Thr Ala Arg Thr Thr Ala Ile Lys Gly Arg Phe Glu Gly Ser
    210                 215                 220

Arg Phe Leu Pro Tyr His Thr Arg Asn Gln Ile Asn Gly Gly Ala Leu
225                 230                 235                 240

Asp Gly Lys Ala Pro Ile Leu Gly Tyr Ala Glu Asp Pro Val Glu Leu
                245                 250                 255

Phe Phe Met His Ile Gln Gly Ser Gly Arg Leu Lys Thr Pro Ser Gly
            260                 265                 270

Lys Tyr Ile Arg Ile Gly Tyr Ala Asp Lys Asn Glu His Pro Tyr Val
        275                 280                 285

Ser Ile Gly Arg Tyr Met Ala Asp Lys Gly Tyr Leu Lys Leu Gly Gln
    290                 295                 300

Thr Ser Met Gln Gly Ile Lys Ser Tyr Met Arg Gln Asn Pro Gln Arg
305                 310                 315                 320

Leu Ala Glu Val Leu Gly Gln Asn Pro Ser Tyr Ile Phe Phe Arg Glu
                325                 330                 335

Leu Ala Gly Ser Ser Asn Asp Gly Pro Val Gly Ala Leu Gly Thr Pro
            340                 345                 350

Leu Met Gly Glu Tyr Ala Gly Ala Val Asp Arg His Tyr Ile Thr Leu
        355                 360                 365

Gly Ala Pro Leu Phe Val Ala Thr Ala His Pro Val Thr Arg Lys Ala
    370                 375                 380

Leu Asn Arg Leu Ile Met Ala Gln Asp Thr Gly Ser Ala Ile Lys Gly
385                 390                 395                 400

Ala Val Arg Val Asp Tyr Phe Trp Gly Tyr Gly Asp Glu Ala Gly Glu
                405                 410                 415

Leu Ala Gly Lys Gln Lys Thr Thr Gly Tyr Val Trp Gln Leu Leu Pro
            420                 425                 430

Asn Gly Met Lys Pro Glu Tyr Arg Pro
        435                 440
```

<210> 36
<211> 1326
<212> DNA
<213> Neisseria gonorrhoeae

<400> 36
```
atgaaaaaac acctgctccg ctccgccctg tacggcatcg ccgccgccat cctcgccgcc    60
tgccaaagca ggagcatcca aacctttccg caacccgaca catccgtcat caacggcccg   120
gaccggccgg ccggcatccc cgaccccgcc ggaacgacgg ttgccggcgg cggggccgtc   180
```

```
tataccgttg tgccgcacct gtccatgccc cactgggcgg cgcaggattt tgccaaaagc    240
ctgcaatcct tccgcctcgg ctgcgccaat ttgaaaaacc gccaaggctg gcaggatgtg    300
tgcgcccaag cctttcaaac ccccgtgcat tcctttcagg caaagcggtt ttttgaacgc    360
tatttcacgc cgtggcaggt tgcaggcaac ggaagccttg caggtacggt taccggctat    420
tacgaaccgg tgctgaaggg cgacggcagg cggacggaac gggcccgctt cccgatttac    480
ggtattcccg acgattttat ctccgtcccg ctgcctgccg gtttgcgggg cggaaaaaac    540
cttgtccgca tcaggcagac ggggaaaaac agcggcacga tcgacaatgc cggcggcacg    600
cataccgccg acctctcccg attccccatc accgcgcgca caacggcaat caaaggcagg    660
tttgaaggaa gccgcttcct cccttaccac acgcgcaacc aaatcaacgg cggcgcgctt    720
gacggcaaag cccccatcct cggttacgcc gaagaccccg tcgaactttt tttcatgcac    780
atccaaggct cgggccgcct gaaaaccccg tccggcaaat acatccgcat cggatacgcc    840
gacaaaaacg aacatccgta tgtttccatc ggacgctata tggcggacaa aggctacctc    900
aagctcgggc agacctcgat gcagggcatc aaagcctata tgcggcaaaa tccgcaacgc    960
ctcgccgaag ttttgggtca aaaccccagc tatatctttt tccgcgagct tgccggaagc   1020
ggcaatgagg gccccgtcgg cgcactgggc acgccactga tgggggaata cgccggcgca   1080
atcgaccggc actacattac cttgggcgcg cccttatttg tcgccaccgc ccatccggtt   1140
acccgcaaag ccctcaaccg cctgattatg gcgcaggata caggcagcgc gatcaaaggc   1200
gcggtgcgcg tggattattt ttggggttac ggcgacgaag ccggcgaact tgccggcaaa   1260
cagaaaacca cgggatacgt ctggcagctc ctgcccaacg gcatgaagcc cgaataccgc   1320
ccgtga                                                              1326
```

<210> 37
<211> 441
<212> PRT
<213> Neisseria gonorrhoeae

<400> 37

```
Met Lys Lys His Leu Leu Arg Ser Ala Leu Tyr Gly Ile Ala Ala Ala
1               5                   10                  15

Ile Leu Ala Ala Cys Gln Ser Arg Ser Ile Gln Thr Phe Pro Gln Pro
            20                  25                  30

Asp Thr Ser Val Ile Asn Gly Pro Asp Arg Pro Ala Gly Ile Pro Asp
        35                  40                  45

Pro Ala Gly Thr Thr Val Ala Gly Gly Gly Ala Val Tyr Thr Val Val
    50                  55                  60

Pro His Leu Ser Met Pro His Trp Ala Ala Gln Asp Phe Ala Lys Ser
65                  70                  75                  80

Leu Gln Ser Phe Arg Leu Gly Cys Ala Asn Leu Lys Asn Arg Gln Gly
                85                  90                  95

Trp Gln Asp Val Cys Ala Gln Ala Phe Gln Thr Pro Val His Ser Phe
            100                 105                 110

Gln Ala Lys Arg Phe Phe Glu Arg Tyr Phe Thr Pro Trp Gln Val Ala
        115                 120                 125

Gly Asn Gly Ser Leu Ala Gly Thr Val Thr Gly Tyr Tyr Glu Pro Val
    130                 135                 140

Leu Lys Gly Asp Gly Arg Arg Thr Glu Arg Ala Arg Phe Pro Ile Tyr
145                 150                 155                 160

Gly Ile Pro Asp Asp Phe Ile Ser Val Pro Leu Pro Ala Gly Leu Arg
                165                 170                 175

Gly Gly Lys Asn Leu Val Arg Ile Arg Gln Thr Gly Lys Asn Ser Gly
```

```
            180                 185                 190

Thr Ile Asp Asn Ala Gly Gly Thr His Thr Ala Asp Leu Ser Arg Phe
        195                 200                 205

Pro Ile Thr Ala Arg Thr Thr Ala Ile Lys Gly Arg Phe Glu Gly Ser
    210                 215                 220

Arg Phe Leu Pro Tyr His Thr Arg Asn Gln Ile Asn Gly Gly Ala Leu
225                 230                 235                 240

Asp Gly Lys Ala Pro Ile Leu Gly Tyr Ala Glu Asp Pro Val Glu Leu
                245                 250                 255

Phe Phe Met His Ile Gln Gly Ser Gly Arg Leu Lys Thr Pro Ser Gly
            260                 265                 270

Lys Tyr Ile Arg Ile Gly Tyr Ala Asp Lys Asn Glu His Pro Tyr Val
        275                 280                 285

Ser Ile Gly Arg Tyr Met Ala Asp Lys Gly Tyr Leu Lys Leu Gly Gln
    290                 295                 300

Thr Ser Met Gln Gly Ile Lys Ala Tyr Met Arg Gln Asn Pro Gln Arg
305                 310                 315                 320

Leu Ala Glu Val Leu Gly Gln Asn Pro Ser Tyr Ile Phe Phe Arg Glu
                325                 330                 335

Leu Ala Gly Ser Gly Asn Glu Gly Pro Val Gly Ala Leu Gly Thr Pro
            340                 345                 350

Leu Met Gly Glu Tyr Ala Gly Ala Ile Asp Arg His Tyr Ile Thr Leu
        355                 360                 365

Gly Ala Pro Leu Phe Val Ala Thr Ala His Pro Val Thr Arg Lys Ala
    370                 375                 380

Leu Asn Arg Leu Ile Met Ala Gln Asp Thr Gly Ser Ala Ile Lys Gly
385                 390                 395                 400

Ala Val Arg Val Asp Tyr Phe Trp Gly Tyr Gly Asp Glu Ala Gly Glu
                405                 410                 415

Leu Ala Gly Lys Gln Lys Thr Thr Gly Tyr Val Trp Gln Leu Leu Pro
            420                 425                 430

Asn Gly Met Lys Pro Glu Tyr Arg Pro
        435                 440
```

<210> 38
<211> 1326
<212> DNA
<213> Neisseria meningitidis

<400> 38

```
atgaaaaaat acctattccg cgccgccctg tgcggcatcg ccgccgccat cctcgccgcc    60
tgccaaagca agagcatcca aacctttccg caacccgaca catccgtcat caacggcccg   120
gaccggccgg tcggcatccc cgaccccgcc ggaacgacgg tcggcggcgg cggggccgtt   180
tataccgttg tgccgcacct gtccctgccc cactgggcgg cgcaggattt cgccaaaagc   240
ctgcaatcct tccgcctcgg ctgcgccaat ttgaaaaacc gccaaggctg gcaggatgtg   300
```

```
tgcgcccaag cctttcaaac ccccgtccat tccgttcagg caaaacagtt ttttgaacgc   360
tatttcacgc cgtggcaggt tgcaggcaac ggaagccttg ccggtacggt taccggctat   420
tacgagccgg tgctgaaggg cgacgacagg cggacggcac aagcccgctt cccgatttac   480
ggtattcccg acgattttat ctccgtcccc ctgcctgccg gtttgcggag cggaaaagcc   540
cttgtccgca tcaggcagac gggaaaaaac agcggcacaa tcgacaatac cggcggcaca   600
cataccgccg acctctccca attccccatc actgcgcgca caacggcaat caaaggcagg   660
tttgaaggaa gccgcttcct cccctaccac acgcgcaacc aaatcaacgg cggcgcgctt   720
gacggcaaag ccccgatact cggttacgcc gaagaccccg tcgaactttt ttttatgcac   780
atccaaggct cgggccgtct gaaaaccccg tccggcaaat acatccgcat cggctatgcc   840
gacaaaaacg aacatcccta cgtttccatc ggacgctata tggcggacaa aggctacctc   900
aagctcgggc agacctcgat gcagggcatc aaagcctata tgcagcaaaa cccgcaacgc   960
ctcgccgaag ttttggggca aaaccccagc tatatctttt tccgagagct taccggaagc   1020
agcaatgacg gccctgtcgg cgcactgggc acgccgctga tgggcgagta cgccggcgca   1080
gtcgaccggc actacattac cttgggcgcg cccttatttg tcgccaccgc ccatccggtt   1140
acccgcaaag ccctcaaccg cctgattatg gcgcaggata ccggcagcgc gattaaaggc   1200
gcggtgcgcg tggattattt ttggggatac ggcgacgaag ccggcgaact tgccggcaaa   1260
cagaaaacca cgggatatgt ctggcagctt ctgcccaacg gtatgaagcc cgaataccgc   1320
ccgtaa                                                              1326
```

<210> 39
<211> 441
<212> PRT
<213> Neisseria meningitidis

<400> 39

```
Met Lys Lys Tyr Leu Phe Arg Ala Ala Leu Cys Gly Ile Ala Ala Ala
1               5                   10                  15

Ile Leu Ala Ala Cys Gln Ser Lys Ser Ile Gln Thr Phe Pro Gln Pro
            20                  25                  30

Asp Thr Ser Val Ile Asn Gly Pro Asp Arg Pro Val Gly Ile Pro Asp
        35                  40                  45

Pro Ala Gly Thr Thr Val Gly Gly Gly Gly Ala Val Tyr Thr Val Val
    50                  55                  60

Pro His Leu Ser Leu Pro His Trp Ala Ala Gln Asp Phe Ala Lys Ser
65                  70                  75                  80

Leu Gln Ser Phe Arg Leu Gly Cys Ala Asn Leu Lys Asn Arg Gln Gly
                85                  90                  95

Trp Gln Asp Val Cys Ala Gln Ala Phe Gln Thr Pro Val His Ser Val
            100                 105                 110

Gln Ala Lys Gln Phe Phe Glu Arg Tyr Phe Thr Pro Trp Gln Val Ala
        115                 120                 125

Gly Asn Gly Ser Leu Ala Gly Thr Val Thr Gly Tyr Tyr Glu Pro Val
    130                 135                 140

Leu Lys Gly Asp Asp Arg Arg Thr Ala Gln Ala Arg Phe Pro Ile Tyr
145                 150                 155                 160

Gly Ile Pro Asp Asp Phe Ile Ser Val Pro Leu Pro Ala Gly Leu Arg
                165                 170                 175

Ser Gly Lys Ala Leu Val Arg Ile Arg Gln Thr Gly Lys Asn Ser Gly
            180                 185                 190
```

```
Thr Ile Asp Asn Thr Gly Gly Thr His Thr Ala Asp Leu Ser Gln Phe
        195                 200                 205

Pro Ile Thr Ala Arg Thr Thr Ala Ile Lys Gly Arg Phe Glu Gly Ser
    210                 215                 220

Arg Phe Leu Pro Tyr His Thr Arg Asn Gln Ile Asn Gly Gly Ala Leu
225                 230                 235                 240

Asp Gly Lys Ala Pro Ile Leu Gly Tyr Ala Glu Asp Pro Val Glu Leu
                245                 250                 255

Phe Phe Met His Ile Gln Gly Ser Gly Arg Leu Lys Thr Pro Ser Gly
            260                 265                 270

Lys Tyr Ile Arg Ile Gly Tyr Ala Asp Lys Asn Glu His Pro Tyr Val
        275                 280                 285

Ser Ile Gly Arg Tyr Met Ala Asp Lys Gly Tyr Leu Lys Leu Gly Gln
    290                 295                 300

Thr Ser Met Gln Gly Ile Lys Ala Tyr Met Gln Gln Asn Pro Gln Arg
305                 310                 315                 320

Leu Ala Glu Val Leu Gly Gln Asn Pro Ser Tyr Ile Phe Phe Arg Glu
                325                 330                 335

Leu Thr Gly Ser Ser Asn Asp Gly Pro Val Gly Ala Leu Gly Thr Pro
            340                 345                 350

Leu Met Gly Glu Tyr Ala Gly Ala Val Asp Arg His Tyr Ile Thr Leu
        355                 360                 365

Gly Ala Pro Leu Phe Val Ala Thr Ala His Pro Val Thr Arg Lys Ala
    370                 375                 380

Leu Asn Arg Leu Ile Met Ala Gln Asp Thr Gly Ser Ala Ile Lys Gly
385                 390                 395                 400

Ala Val Arg Val Asp Tyr Phe Trp Gly Tyr Gly Asp Glu Ala Gly Glu
                405                 410                 415

Leu Ala Gly Lys Gln Lys Thr Thr Gly Tyr Val Trp Gln Leu Leu Pro
            420                 425                 430

Asn Gly Met Lys Pro Glu Tyr Arg Pro
        435                 440

<210>  40
<211>  1101
<212>  DNA
<213>  Neisseria meningitidis

<220>
<221>  n
<222>  364 .. 606
<223>  n is any nucleotide

<400>  40
atggaaacac agctttacat cggcatcatg tcgggaacca gcatggacgg ggcggatgcc    60
gtactgatac ggatggacgg cggcaaatgg ctgggcgcgg aagggcacgc ctttaccccc   120
```

```
taccccggca ggttacgccg ccaattgctg gatttgcagg acacaggcgc agacgaactg   180
caccgcagca ggattttgtc gcaagaactc agccgcctat atgcgcaaac cgccgccgaa   240
ctgctgtgca gtcaaaacct cgcaccgtcc gacattaccg ccctcggctg ccacgggcaa   300
accgtccgac acgcgccgga acacggttac agcatacagc ttgccgattt gccgctgctg   360
gcgnnnnnnn nnnnnnnnnn nnnnnnnnnn nnnnnnnnnn nnnnnnnnnn nnnnnnnnnn   420
nnnnnnnnnn nnnnnnnnnn nnnnnnnnnn nnnnnnnnnn nnnnnnnnnn nnnnnnnnnn   480
nnnnnnnnnn nnnnnnnnnn nnnnnnnnnn nnnnnnnnnn nnnnnnnnnn nnnnnnnnnn   540
nnnnnnnnnn nnnnnnnnnn nnnnnnnnnn nnnnnnnnnn nnnnnnnnnn nnnnnnnnnn   600
nnnnnncagc ttccttacga caaaaacggt gcaaagtcgg cacaaggcaa catattgccg   660
caactgctcg acaggctgct cgcccacccg tatttcgcac aacgccaccc taaaagcacg   720
gggcgcgaac tgtttgccat aaattggctc gaaacctacc ttgacggcgg cgaaaaccga   780
tacgacgtat tgcggacgct ttcccgtttt accgcgcaaa ccgtttgcga cgccgtctca   840
cacgcagcgg cagatgcccg tcaaatgtac atttgcgacg gcggcatccg caatcctgtt   900
ttaatggcgg atttggcaga atgtttcggc acacgcgttt ccctgcacag caccgccgac   960
ctgaacctcg atccgcaatg ggtggaagcc gccgnatttg cgtggttggc ggcgtgttgg   1020
attaatcgca ttcccggtag tccgcacaaa gcaaccggcg catccaaacc gtgtattctg   1080
ancgcgggat attattattg a                                             1101
```

<210> 41
<211> 366
<212> PRT
<213> Neisseria meningitidis

<400> 41

```
Met Glu Thr Gln Leu Tyr Ile Gly Ile Met Ser Gly Thr Ser Met Asp
1               5                   10                  15

Gly Ala Asp Ala Val Leu Ile Arg Met Asp Gly Gly Lys Trp Leu Gly
            20                  25                  30

Ala Glu Gly His Ala Phe Thr Pro Tyr Pro Gly Arg Leu Arg Arg Gln
        35                  40                  45

Leu Leu Asp Leu Gln Asp Thr Gly Ala Asp Glu Leu His Arg Ser Arg
    50                  55                  60

Ile Leu Ser Gln Glu Leu Ser Arg Leu Tyr Ala Gln Thr Ala Ala Glu
65                  70                  75                  80

Leu Leu Cys Ser Gln Asn Leu Ala Pro Ser Asp Ile Thr Ala Leu Gly
                85                  90                  95

Cys His Gly Gln Thr Val Arg His Ala Pro Glu His Gly Tyr Ser Ile
            100                 105                 110

Gln Leu Ala Asp Leu Pro Leu Leu Ala Xaa Xaa Xaa Xaa Xaa Xaa Xaa
        115                 120                 125

Xaa Xaa Xaa Xaa Xaa Xaa Xaa Xaa Xaa Xaa Xaa Xaa Xaa Xaa Xaa Xaa
    130                 135                 140

Xaa Xaa Xaa Xaa Xaa Xaa Xaa Xaa Xaa Xaa Xaa Xaa Xaa Xaa Xaa Xaa
145                 150                 155                 160

Xaa Xaa Xaa Xaa Xaa Xaa Xaa Xaa Xaa Xaa Xaa Xaa Xaa Xaa Xaa Xaa
                165                 170                 175

Xaa Xaa Xaa Xaa Xaa Xaa Xaa Xaa Xaa Xaa Xaa Xaa Xaa Xaa Xaa Xaa
            180                 185                 190

Xaa Xaa Xaa Xaa Xaa Xaa Xaa Xaa Xaa Xaa Gln Leu Pro Tyr Asp Lys
```

```
        195                 200                 205

Asn Gly Ala Lys Ser Ala Gln Gly Asn Ile Leu Pro Gln Leu Leu Asp
    210                 215                 220

Arg Leu Leu Ala His Pro Tyr Phe Ala Gln Arg His Pro Lys Ser Thr
225                 230                 235                 240

Gly Arg Glu Leu Phe Ala Ile Asn Trp Leu Glu Thr Tyr Leu Asp Gly
                245                 250                 255

Gly Glu Asn Arg Tyr Asp Val Leu Arg Thr Leu Ser Arg Phe Thr Ala
            260                 265                 270

Gln Thr Val Cys Asp Ala Val Ser His Ala Ala Ala Asp Ala Arg Gln
        275                 280                 285

Met Tyr Ile Cys Asp Gly Gly Ile Arg Asn Pro Val Leu Met Ala Asp
    290                 295                 300

Leu Ala Glu Cys Phe Gly Thr Arg Val Ser Leu His Ser Thr Ala Asp
305                 310                 315                 320

Leu Asn Leu Asp Pro Gln Trp Val Glu Ala Ala Xaa Phe Ala Trp Leu
                325                 330                 335

Ala Ala Cys Trp Ile Asn Arg Ile Pro Gly Ser Pro His Lys Ala Thr
            340                 345                 350

Gly Ala Ser Lys Pro Cys Ile Leu Xaa Ala Gly Tyr Tyr Tyr
        355                 360                 365
```

<210> 42
<211> 1101
<212> DNA
<213> Neisseria gonorrhoeae

<400> 42

```
atggaaacac agctttacat cggcattatg tcgggaacca gtatggacgg ggcggatgcc   60
gtgctggtac ggatggacgg cggcaaatgg ctgggcgcgg aagggcacgc ctttaccccc   120
taccctgacc ggttgcgccg caaattgctg gatttgcagg acacaggcac agacgaactg   180
caccgcagca ggatgttgtc gcaagaactc agccgcctgt acgcgcaaac cgccgccgaa   240
ctgctgtgca gtcaaaacct cgctccgtgc gacattaccg ccctcggctg ccacgggcaa   300
accgtccgac acgcgccgga acacggttac agcatacagc ttgccgattt gccgctgctg   360
gcggaactga cgcggatttt taccgtcggc gacttccgca gccgcgacct tgctgccggc   420
ggacaaggtg cgccgctcgt ccccgccttt cacgaagccc tgttccgcga tgacagggaa   480
acacgcgtgg tactgaacat cggcgggatt gccaacatca gcgtactccc ccccggcgca   540
cccgccttcg gcttcgacac agggccgggc aatatgctga tggacgcgtg gacgcaggca   600
cactggcagc tgccttacga caaaaacggt gcaaaggcgg cacaaggcaa catattgccg   660
caactgctcg gcaggctgct cgcccacccg tatttctcac aaccccaccc aaaaagcacg   720
gggcgcgaac tgtttgccct aaattggctc gaaacctacc ttgacggcgg cgaaaaccga   780
tacgacgtat tgcggacgct ttcccgattc accgcgcaaa ccgtttggga cgccgtctca   840
cacgcagcgg cagatgcccg tcaaatgtac atttgcggcg gcggcatccg caatcctgtt   900
ttaatggcgg atttggcaga atgtttcggc acacgcgttt ccctgcacag caccgccgaa   960
ctgaacctcg atcctcaatg ggtggaggcg gccgcatttg cgtggttggc ggcgtgttgg   1020
attaaccgca ttcccggtag tccgcacaaa gcgaccggcg catccaaacc gtgtattctg   1080
ggcgcgggat attattattg a                                             1101
```

<210> 43
<211> 366
<212> PRT

<213> Neisseria gonorrhoeae

<400> 43

```
Met Glu Thr Gln Leu Tyr Ile Gly Ile Met Ser Gly Thr Ser Met Asp
1               5                   10                  15

Gly Ala Asp Ala Val Leu Val Arg Met Asp Gly Gly Lys Trp Leu Gly
            20                  25                  30

Ala Glu Gly His Ala Phe Thr Pro Tyr Pro Asp Arg Leu Arg Arg Lys
        35                  40                  45

Leu Leu Asp Leu Gln Asp Thr Gly Thr Asp Glu Leu His Arg Ser Arg
    50                  55                  60

Met Leu Ser Gln Glu Leu Ser Arg Leu Tyr Ala Gln Thr Ala Ala Glu
65                  70                  75                  80

Leu Leu Cys Ser Gln Asn Leu Ala Pro Cys Asp Ile Thr Ala Leu Gly
                85                  90                  95

Cys His Gly Gln Thr Val Arg His Ala Pro Glu His Gly Tyr Ser Ile
            100                 105                 110

Gln Leu Ala Asp Leu Pro Leu Leu Ala Glu Leu Thr Arg Ile Phe Thr
        115                 120                 125

Val Gly Asp Phe Arg Ser Arg Asp Leu Ala Ala Gly Gly Gln Gly Ala
    130                 135                 140

Pro Leu Val Pro Ala Phe His Glu Ala Leu Phe Arg Asp Asp Arg Glu
145                 150                 155                 160

Thr Arg Val Val Leu Asn Ile Gly Gly Ile Ala Asn Ile Ser Val Leu
                165                 170                 175

Pro Pro Gly Ala Pro Ala Phe Gly Phe Asp Thr Gly Pro Gly Asn Met
            180                 185                 190

Leu Met Asp Ala Trp Thr Gln Ala His Trp Gln Leu Pro Tyr Asp Lys
        195                 200                 205

Asn Gly Ala Lys Ala Ala Gln Gly Asn Ile Leu Pro Gln Leu Leu Gly
    210                 215                 220

Arg Leu Leu Ala His Pro Tyr Phe Ser Gln Pro His Pro Lys Ser Thr
225                 230                 235                 240

Gly Arg Glu Leu Phe Ala Leu Asn Trp Leu Glu Thr Tyr Leu Asp Gly
                245                 250                 255

Gly Glu Asn Arg Tyr Asp Val Leu Arg Thr Leu Ser Arg Phe Thr Ala
            260                 265                 270

Gln Thr Val Trp Asp Ala Val Ser His Ala Ala Ala Asp Ala Arg Gln
        275                 280                 285

Met Tyr Ile Cys Gly Gly Gly Ile Arg Asn Pro Val Leu Met Ala Asp
    290                 295                 300

Leu Ala Glu Cys Phe Gly Thr Arg Val Ser Leu His Ser Thr Ala Glu
```

305 310 315 320

Leu Asn Leu Asp Pro Gln Trp Val Glu Ala Ala Ala Phe Ala Trp Leu
325 330 335

Ala Ala Cys Trp Ile Asn Arg Ile Pro Gly Ser Pro His Lys Ala Thr
340 345 350

Gly Ala Ser Lys Pro Cys Ile Leu Gly Ala Gly Tyr Tyr Tyr
355 360 365

<210> 44
<211> 1101
<212> DNA
<213> Neisseria meningitidis

<400> 44
atggaaacac agctttacat cggcatcatg tcgggaacca gcatggacgg ggcggatgcc 60
gtactgatac ggatggacgg cggcaaatgg ctgggcgcgg aagggcacgc ctttaccccc 120
taccccggca ggttacgccg caaattgctg gatttgcagg acacaggcgc ggacgaactg 180
caccgcagca ggatgttgtc gcaagaactc agccgcctgt acgcgcaaac cgccgccgaa 240
ctgctgtgca gtcaaaacct cgcgccgtcc gacattaccg ccctcggctg ccacgggcaa 300
accgtcagac acgcgccgga acacagttac agcgtacagc ttgccgattt gccgctgctg 360
gcggaacgga ctcagatttt taccgtcggc gacttccgca gccgcgacct tgcggccggc 420
ggacaaggcg cgccgctcgt ccccgccttt cacgaagccc tgttccgcga cgacagggaa 480
acacgcgcgg tactgaacat cggcgggatt gccaacatca gcgtactccc ccccgacgca 540
cccgccttcg gcttcgacac aggaccgggc aatatgctga tggacgcgtg gatgcaggca 600
cactggcagc ttccttacga caaaaacggt gcaaaggcgg cacaaggcaa catattgccg 660
caactgctcg acaggctgct cgcccacccg tatttcgcac aaccccaccc taaaagcacg 720
gggcgcgaac tgtttgccct aaattggctc gaaacctacc ttgacggcgg cgaaaaccga 780
tacgacgtat tgcggacgct ttcccgattc accgcgcaaa ccgttttcga cgccgtctca 840
cacgcagcgg cagatgcccg tcaaatgtac atttgcggcg gcggcatccg caatcctgtt 900
ttaatggcgg atttggcaga atgtttcggc acacgcgttt ccctgcacag caccgccgaa 960
ctgaacctcg atccgcaatg ggtagaagcc gccgcgttcg catggatggc ggcgtgttgg 1020
gtcaaccgca ttcccggtag tccgcacaaa gcaaccggcg catccaaacc gtgtattctg 1080
ggcgcgggat attattattg a 1101

<210> 45
<211> 366
<212> PRT
<213> Neisseria meningitidis

<400> 45
Met Glu Thr Gln Leu Tyr Ile Gly Ile Met Ser Gly Thr Ser Met Asp
1 5 10 15

Gly Ala Asp Ala Val Leu Ile Arg Met Asp Gly Gly Lys Trp Leu Gly
20 25 30

Ala Glu Gly His Ala Phe Thr Pro Tyr Pro Gly Arg Leu Arg Arg Lys
35 40 45

Leu Leu Asp Leu Gln Asp Thr Gly Ala Asp Glu Leu His Arg Ser Arg
50 55 60

Met Leu Ser Gln Glu Leu Ser Arg Leu Tyr Ala Gln Thr Ala Ala Glu
65 70 75 80

Leu Leu Cys Ser Gln Asn Leu Ala Pro Ser Asp Ile Thr Ala Leu Gly
85 90 95

```
Cys His Gly Gln Thr Val Arg His Ala Pro Glu His Ser Tyr Ser Val
            100                 105                 110

Gln Leu Ala Asp Leu Pro Leu Leu Ala Glu Arg Thr Gln Ile Phe Thr
        115                 120                 125

Val Gly Asp Phe Arg Ser Arg Asp Leu Ala Ala Gly Gly Gln Gly Ala
    130                 135                 140

Pro Leu Val Pro Ala Phe His Glu Ala Leu Phe Arg Asp Asp Arg Glu
145                 150                 155                 160

Thr Arg Ala Val Leu Asn Ile Gly Gly Ile Ala Asn Ile Ser Val Leu
                165                 170                 175

Pro Pro Asp Ala Pro Ala Phe Gly Phe Asp Thr Gly Pro Gly Asn Met
            180                 185                 190

Leu Met Asp Ala Trp Met Gln Ala His Trp Gln Leu Pro Tyr Asp Lys
        195                 200                 205

Asn Gly Ala Lys Ala Ala Gln Gly Asn Ile Leu Pro Gln Leu Leu Asp
    210                 215                 220

Arg Leu Leu Ala His Pro Tyr Phe Ala Gln Pro His Pro Lys Ser Thr
225                 230                 235                 240

Gly Arg Glu Leu Phe Ala Leu Asn Trp Leu Glu Thr Tyr Leu Asp Gly
                245                 250                 255

Gly Glu Asn Arg Tyr Asp Val Leu Arg Thr Leu Ser Arg Phe Thr Ala
            260                 265                 270

Gln Thr Val Phe Asp Ala Val Ser His Ala Ala Ala Asp Ala Arg Gln
        275                 280                 285

Met Tyr Ile Cys Gly Gly Gly Ile Arg Asn Pro Val Leu Met Ala Asp
    290                 295                 300

Leu Ala Glu Cys Phe Gly Thr Arg Val Ser Leu His Ser Thr Ala Glu
305                 310                 315                 320

Leu Asn Leu Asp Pro Gln Trp Val Glu Ala Ala Ala Phe Ala Trp Met
                325                 330                 335

Ala Ala Cys Trp Val Asn Arg Ile Pro Gly Ser Pro His Lys Ala Thr
            340                 345                 350

Gly Ala Ser Lys Pro Cys Ile Leu Gly Ala Gly Tyr Tyr Tyr
        355                 360                 365

<210>   46
<211>   1101
<212>   DNA
<213>   Neisseria meningitidis

<400>   46
atggaaacac agctttacat cggcatcatg tcgggaacca gcatggacgg ggcggatgcc     60
gtactgatac ggatggacgg cggcaaatgg ctgggcgcgg aagggcacgc ctttaccccc    120
taccccggca ggttacgccg ccaattgctg gatttgcagg acacaggcgc agacgaactg    180
caccgcagca ggattttgtc gcaagaactc agccgcctat atgcgcaaac cgccgccgaa    240
```

```
ctgctgtgca gtcaaaacct cgcaccgtcc gacattaccg ccctcggctg ccacgggcaa   300
accgtccgac acgcgccgga acacggttac agcatacagc ttgccgattt gccgctgctg   360
gcggaacgga cgcggatttt taccgtcggc gacttccgca gccgcgacct tgcggccggc   420
ggacaaggcg cgccactcgt ccccgccttt cacgaagccc tgttccgcga caacagggaa   480
acacgcgcgg tactgaacat cggcgggatt gccaacatca gcgtactccc ccccgacgca   540
cccgccttcg gcttcgacac agggccgggc aatatgctga tggacgcgtg gacgcaggca   600
cactggcagc ttccttacga caaaaacggt gcaaaggcgg cacaaggcaa catattgccg   660
caactgctcg acaggctgct cgcccacccg tatttcgcac aaccccaccc taaaagcacg   720
gggcgcgaac tgtttgccct aaattggctc gaaacctacc ttgacggcgg cgaaaaccga   780
tacgacgtat tgcggacgct ttcccgtttt accgcgcaaa ccgtttgcga cgccgtctca   840
cacgcagcgg cagatgcccg tcaaatgtac atttgcggcg gcggcatccg caatcctgtt   900
ttaatggcgg atttggcaga atgtttcggc acacgcgttt ccctgcacag caccgccgac   960
ctgaacctcg atccgcaatg ggtggaagcc gccgnatttg cgtggttggc ggcgtgttgg   1020
attaatcgca ttcccggtag tccgcacaaa gcaaccggcg catccaaacc gtgtattctg   1080
ancgcgggat attattattg a                                             1101
```

<210> 47
<211> 366
<212> PRT
<213> Neisseria meningitidis

<400> 47

```
Met Glu Thr Gln Leu Tyr Ile Gly Ile Met Ser Gly Thr Ser Met Asp
1               5                   10                  15

Gly Ala Asp Ala Val Leu Ile Arg Met Asp Gly Gly Lys Trp Leu Gly
            20                  25                  30

Ala Glu Gly His Ala Phe Thr Pro Tyr Pro Gly Arg Leu Arg Arg Gln
        35                  40                  45

Leu Leu Asp Leu Gln Asp Thr Gly Ala Asp Glu Leu His Arg Ser Arg
    50                  55                  60

Ile Leu Ser Gln Glu Leu Ser Arg Leu Tyr Ala Gln Thr Ala Ala Glu
65                  70                  75                  80

Leu Leu Cys Ser Gln Asn Leu Ala Pro Ser Asp Ile Thr Ala Leu Gly
                85                  90                  95

Cys His Gly Gln Thr Val Arg His Ala Pro Glu His Gly Tyr Ser Ile
            100                 105                 110

Gln Leu Ala Asp Leu Pro Leu Leu Ala Glu Arg Thr Arg Ile Phe Thr
        115                 120                 125

Val Gly Asp Phe Arg Ser Arg Asp Leu Ala Ala Gly Gly Gln Gly Ala
    130                 135                 140

Pro Leu Val Pro Ala Phe His Glu Ala Leu Phe Arg Asp Asn Arg Glu
145                 150                 155                 160

Thr Arg Ala Val Leu Asn Ile Gly Gly Ile Ala Asn Ile Ser Val Leu
                165                 170                 175

Pro Pro Asp Ala Pro Ala Phe Gly Phe Asp Thr Gly Pro Gly Asn Met
            180                 185                 190

Leu Met Asp Ala Trp Thr Gln Ala His Trp Gln Leu Pro Tyr Asp Lys
        195                 200                 205
```

```
Asn Gly Ala Lys Ala Ala Gln Gly Asn Ile Leu Pro Gln Leu Leu Asp
    210                 215                 220

Arg Leu Leu Ala His Pro Tyr Phe Ala Gln Pro His Pro Lys Ser Thr
225                 230                 235                 240

Gly Arg Glu Leu Phe Ala Leu Asn Trp Leu Glu Thr Tyr Leu Asp Gly
                245                 250                 255

Gly Glu Asn Arg Tyr Asp Val Leu Arg Thr Leu Ser Arg Phe Thr Ala
            260                 265                 270

Gln Thr Val Cys Asp Ala Val Ser His Ala Ala Ala Asp Ala Arg Gln
        275                 280                 285

Met Tyr Ile Cys Gly Gly Gly Ile Arg Asn Pro Val Leu Met Ala Asp
    290                 295                 300

Leu Ala Glu Cys Phe Gly Thr Arg Val Ser Leu His Ser Thr Ala Asp
305                 310                 315                 320

Leu Asn Leu Asp Pro Gln Trp Val Glu Ala Ala Xaa Phe Ala Trp Leu
                325                 330                 335

Ala Ala Cys Trp Ile Asn Arg Ile Pro Gly Ser Pro His Lys Ala Thr
            340                 345                 350

Gly Ala Ser Lys Pro Cys Ile Leu Xaa Ala Gly Tyr Tyr Tyr
        355                 360                 365

<210>   48
<211>   1101
<212>   DNA
<213>   Neisseria meningitidis

<400>   48
atggaaacac agctttacat cggcatcatg tcgggaacca gcatggacgg ggcggatgcc     60
gtactgatac ggatggacgg cggcaaatgg ctgggcgcgg aagggcacgc ctttaccccc    120
taccccggca ggttacgccg caaattgctg gatttgcagg acacaggcgc ggacgaactg    180
caccgcagca ggatgttgtc gcaagaactc agccgcctgt acgcgcaaac cgccgccgaa    240
ctgctgtgca gtcaaaacct cgcgccgtcc gacattaccg ccctcggctg ccacgggcaa    300
accgtcagac acgcgccgga acacagttac agcgtacagc ttgccgattt gccgctgctg    360
gcggaacgga ctcagatttt taccgtcggc gacttccgca gccgcgacct tgcggccggc    420
ggacaaggcg cgccgctcgt ccccgccttt cacgaagccc tgttccgcga cgacagggaa    480
acacgcgcgg tactgaacat cggcgggatt gccaacatca gcgtactccc ccccgacgca    540
cccgccttcg gcttcgacac aggaccgggc aatatgctga tggacgcgtg gatgcaggca    600
cactggcagc ttccttacga caaaaacggt gcaaaggcgg cacaaggcaa catattgccg    660
caactgctcg acaggctgct cgcccacccg tatttcgcac aaccccaccc taaaagcacg    720
gggcgcgaac tgtttgccct aaattggctc gaaacctacc ttgacggcgg cgaaaaccga    780
tacgacgtat tgcggacgct ttcccgattc accgcgcaaa ccgttttcga cgccgtctca    840
cacgcagcgg cagatgcccg tcaaatgtac atttgcggcg gcggcatccg caatcctgtt    900
ttaatggcgg atttggcaga atgtttcggc acacgcgttt ccctgcacag caccgccgaa    960
ctgaacctcg atccgcaatg ggtagaagcc gccgcgttcg catggatggc ggcgtgttgg   1020
gtcaaccgca ttcccggtag tccgcacaaa gcaaccggcg catccaaacc gtgtattctg   1080
ggcgcgggat attattattg a                                             1101

<210>   49
<211>   366
<212>   PRT
<213>   Neisseria meningitidis
```

<400> 49

```
Met Glu Thr Gln Leu Tyr Ile Gly Ile Met Ser Gly Thr Ser Met Asp
1               5                   10                  15

Gly Ala Asp Ala Val Leu Ile Arg Met Asp Gly Gly Lys Trp Leu Gly
            20                  25                  30

Ala Glu Gly His Ala Phe Thr Pro Tyr Pro Gly Arg Leu Arg Arg Lys
        35                  40                  45

Leu Leu Asp Leu Gln Asp Thr Gly Ala Asp Glu Leu His Arg Ser Arg
    50                  55                  60

Met Leu Ser Gln Glu Leu Ser Arg Leu Tyr Ala Gln Thr Ala Ala Glu
65                  70                  75                  80

Leu Leu Cys Ser Gln Asn Leu Ala Pro Ser Asp Ile Thr Ala Leu Gly
                85                  90                  95

Cys His Gly Gln Thr Val Arg His Ala Pro Glu His Ser Tyr Ser Val
            100                 105                 110

Gln Leu Ala Asp Leu Pro Leu Leu Ala Glu Arg Thr Gln Ile Phe Thr
        115                 120                 125

Val Gly Asp Phe Arg Ser Arg Asp Leu Ala Ala Gly Gly Gln Gly Ala
    130                 135                 140

Pro Leu Val Pro Ala Phe His Glu Ala Leu Phe Arg Asp Asp Arg Glu
145                 150                 155                 160

Thr Arg Ala Val Leu Asn Ile Gly Gly Ile Ala Asn Ile Ser Val Leu
                165                 170                 175

Pro Pro Asp Ala Pro Ala Phe Gly Phe Asp Thr Gly Pro Gly Asn Met
            180                 185                 190

Leu Met Asp Ala Trp Met Gln Ala His Trp Gln Leu Pro Tyr Asp Lys
        195                 200                 205

Asn Gly Ala Lys Ala Ala Gln Gly Asn Ile Leu Pro Gln Leu Leu Asp
    210                 215                 220

Arg Leu Leu Ala His Pro Tyr Phe Ala Gln Pro His Pro Lys Ser Thr
225                 230                 235                 240

Gly Arg Glu Leu Phe Ala Leu Asn Trp Leu Glu Thr Tyr Leu Asp Gly
                245                 250                 255

Gly Glu Asn Arg Tyr Asp Val Leu Arg Thr Leu Ser Arg Phe Thr Ala
            260                 265                 270

Gln Thr Val Phe Asp Ala Val Ser His Ala Ala Ala Asp Ala Arg Gln
        275                 280                 285

Met Tyr Ile Cys Gly Gly Gly Ile Arg Asn Pro Val Leu Met Ala Asp
    290                 295                 300

Leu Ala Glu Cys Phe Gly Thr Arg Val Ser Leu His Ser Thr Ala Glu
305                 310                 315                 320
```

Leu Asn Leu Asp Pro Gln Trp Val Glu Ala Ala Ala Phe Ala Trp Met
325 330 335

Ala Ala Cys Trp Val Asn Arg Ile Pro Gly Ser Pro His Lys Ala Thr
340 345 350

Gly Ala Ser Lys Pro Cys Ile Leu Gly Ala Gly Tyr Tyr Tyr
355 360 365

<210> 50
<211> 396
<212> DNA
<213> Neisseria meningitidis

<400> 50
atgactgaca acgcactgct ccatttgggc gaagaacccc gttttgatca aatcaaaacc 60
gaagacatca aacccgccct gcaaaccgcc atcgccgaag cgcgcgaaca aatcgccgcc 120
atcaaagccc aaacgcacac cggctgggca aacactgtcg aacccctgac cggcatcacc 180
gaacgcgtcg gcaggatttg ggcgtggtg tcgcacctca actgcgtcgc cgacacgccc 240
gaactgcgcg ccgtctataa cgaactgatg cccgaaatca ccgtcttctt caccgaaatc 300
ggacaagaca tcgagctgta caaccgcttc aaaaccatca aaaattcccc cgaattcgac 360
accctctccc ccgcacaaaa aaccaaactc aaccac 396

<210> 51
<211> 132
<212> PRT
<213> Neisseria meningitidis

<400> 51
Met Thr Asp Asn Ala Leu Leu His Leu Gly Glu Glu Pro Arg Phe Asp
1 5 10 15

Gln Ile Lys Thr Glu Asp Ile Lys Pro Ala Leu Gln Thr Ala Ile Ala
20 25 30

Glu Ala Arg Glu Gln Ile Ala Ala Ile Lys Ala Gln Thr His Thr Gly
35 40 45

Trp Ala Asn Thr Val Glu Pro Leu Thr Gly Ile Thr Glu Arg Val Gly
50 55 60

Arg Ile Trp Gly Val Val Ser His Leu Asn Cys Val Ala Asp Thr Pro
65 70 75 80

Glu Leu Arg Ala Val Tyr Asn Glu Leu Met Pro Glu Ile Thr Val Phe
85 90 95

Phe Thr Glu Ile Gly Gln Asp Ile Glu Leu Tyr Asn Arg Phe Lys Thr
100 105 110

Ile Lys Asn Ser Pro Glu Phe Asp Thr Leu Ser Pro Ala Gln Lys Thr
115 120 125

Lys Leu Asn His
130

<210> 52
<211> 2037
<212> DNA
<213> Neisseria gonorrhoeae

<400> 52
atgattgaca acgcactgct ccacttgggc gaagaacccc gttttaatca aatccaaacc 60
gaagacatca aacccgccgt ccaaaccgcc atcgccgaag cgcgcggaca aatcgccgcc 120
gtcaaagcgc aaacgcacac cggctgggcg aacaccgtcg agcgtctgac cggcatcacc 180
gaacgcgtcg gcaggatttg gggcgtcgtg tcccatctca actccgtcgt cgacacgccc 240
gaactgcgcg ccgtctataa cgaactgatg cctgaaatca ccgtcttctt caccgaaatc 300
ggacaagaca tcgaactgta caaccgcttc aaaaccatca aaaattcccc cgaatttgca 360
acgctttccc ccgcacaaaa aaccaagctc gatcacgacc tgcgcgattt cgtattgagc 420
ggcgcggaac tgccgcccga acggcaggca gaactggcaa aactgcaaac cgaaggcgcg 480
caactttccg ccaaattctc ccaaaacgtc ctagacgcga ccgacgcgtt cggcatttac 540
tttgacgatg ccgcaccgct tgccggcatt cccgaagacg cgctcgccat gtttgccgcc 600
gccgcgcaaa gcgaaggcaa aacaggttac aaaatcggct tgcagattcc gcactacctt 660
gccgttatcc aatacgccgg caaccgcgaa ctgcgcgaac aaatctaccg cgcctacgtt 720
acccgtgcca gcgaactttc aaacgacggc aaattcgaca acaccgccaa catcgaccgc 780
acgctcgaaa acgcattgaa aaccgccaaa ctgctcggct ttaaaaatta cgccgaattg 840
tcgctggcaa ccaaaatggc ggacacgccc gaacaggttt taaacttcct gcacgacctc 900
gcccgccgcg ccaaacccta cgccgaaaaa gacctcgccg aagtcaaagc cttcgcccgc 960
gaacacctcg gtctcgccga cccgcagccg tgggacttga gctacgccgg cgaaaaactg 1020
cgcgaagcca aatacgcatt cagcgaaacc gaagtcaaaa aatacttccc cgtcggcaaa 1080
gttctggcag gcctgttcgc ccaaatcaaa aaactctacg gcatcggatt cgccgaaaaa 1140
accgttcccg tctggcacaa agacgtgcgc tattttgaat tgcaacaaaa cggcaaaacc 1200
atcggcggcg tttatatgga tttgtacgca cgcgaaggca aacgcggcgg cgcgtggatg 1260
aacgactaca aaggccgccg ccgctttgcc gacggcacgc tgcaactgcc caccgcctac 1320
ctcgtctgca acttcgcccc gcccgtcggc ggcaaagaag cgcgtttaag ccacgacgaa 1380
atcctcaccc tcttccacga aaccggccac ggactgcacc acctgcttac ccaagtggac 1440
gaactgggcg tgtccggcat caacggcgta gaatgggacg cggtcgaact gcccagccag 1500
tttatggaaa acttcgtttg ggaatacaat gtattggcac aaatgtccgc ccacgaagaa 1560
accggcgagc ccctgccgaa agaactcttc gacaaaatgc tcgccgccaa aaacttccag 1620
cgcggtatgt tcctcgtccg gcaaatggag ttcgccctct tcgatatgat gatttacagt 1680
gaaagcgacg aatgccgtct gaaaaactgg cagcaggttt tagacagcgt gcgcaaagaa 1740
gtcgccgtca tccaaccgcc cgaatacaac cgcttcgcca acagcttcgg ccacatcttc 1800
gccggcggct attccgcagg ctattacagc tacgcatggg ccgaagtcct cagcaccgat 1860
gcctacgccg cctttgaaga aagcgacgac gtcgccgcca caggcaaacg cttctggcaa 1920
gaaatccttg ccgtcggcgg ctcccgcagc gcggcggaat ccttcaaagc cttccgcgga 1980
cgcgaaccga gcatagacgc actgctgcgc caaagcggtt tcgacaacgc ggcttga 2037

<210> 53
<211> 678
<212> PRT
<213> Neisseria gonorrhoeae

<400> 53
Met Ile Asp Asn Ala Leu Leu His Leu Gly Glu Glu Pro Arg Phe Asn
1 5 10 15

Gln Ile Gln Thr Glu Asp Ile Lys Pro Ala Val Gln Thr Ala Ile Ala
20 25 30

Glu Ala Arg Gly Gln Ile Ala Ala Val Lys Ala Gln Thr His Thr Gly
35 40 45

Trp Ala Asn Thr Val Glu Arg Leu Thr Gly Ile Thr Glu Arg Val Gly
50 55 60

Arg Ile Trp Gly Val Val Ser His Leu Asn Ser Val Val Asp Thr Pro
65 70 75 80

Glu Leu Arg Ala Val Tyr Asn Glu Leu Met Pro Glu Ile Thr Val Phe
85 90 95

Phe Thr Glu Ile Gly Gln Asp Ile Glu Leu Tyr Asn Arg Phe Lys Thr

```
            100                 105                 110

Ile Lys Asn Ser Pro Glu Phe Ala Thr Leu Ser Pro Ala Gln Lys Thr
        115                 120                 125

Lys Leu Asp His Asp Leu Arg Asp Phe Val Leu Ser Gly Ala Glu Leu
    130                 135                 140

Pro Pro Glu Arg Gln Ala Glu Leu Ala Lys Leu Gln Thr Glu Gly Ala
145                 150                 155                 160

Gln Leu Ser Ala Lys Phe Ser Gln Asn Val Leu Asp Ala Thr Asp Ala
                165                 170                 175

Phe Gly Ile Tyr Phe Asp Asp Ala Ala Pro Leu Ala Gly Ile Pro Glu
            180                 185                 190

Asp Ala Leu Ala Met Phe Ala Ala Ala Ala Gln Ser Glu Gly Lys Thr
        195                 200                 205

Gly Tyr Lys Ile Gly Leu Gln Ile Pro His Tyr Leu Ala Val Ile Gln
    210                 215                 220

Tyr Ala Gly Asn Arg Glu Leu Arg Glu Gln Ile Tyr Arg Ala Tyr Val
225                 230                 235                 240

Thr Arg Ala Ser Glu Leu Ser Asn Asp Gly Lys Phe Asp Asn Thr Ala
                245                 250                 255

Asn Ile Asp Arg Thr Leu Glu Asn Ala Leu Lys Thr Ala Lys Leu Leu
            260                 265                 270

Gly Phe Lys Asn Tyr Ala Glu Leu Ser Leu Ala Thr Lys Met Ala Asp
        275                 280                 285

Thr Pro Glu Gln Val Leu Asn Phe Leu His Asp Leu Ala Arg Arg Ala
    290                 295                 300

Lys Pro Tyr Ala Glu Lys Asp Leu Ala Glu Val Lys Ala Phe Ala Arg
305                 310                 315                 320

Glu His Leu Gly Leu Ala Asp Pro Gln Pro Trp Asp Leu Ser Tyr Ala
                325                 330                 335

Gly Glu Lys Leu Arg Glu Ala Lys Tyr Ala Phe Ser Glu Thr Glu Val
            340                 345                 350

Lys Lys Tyr Phe Pro Val Gly Lys Val Leu Ala Gly Leu Phe Ala Gln
        355                 360                 365

Ile Lys Lys Leu Tyr Gly Ile Gly Phe Ala Glu Lys Thr Val Pro Val
    370                 375                 380

Trp His Lys Asp Val Arg Tyr Phe Glu Leu Gln Gln Asn Gly Lys Thr
385                 390                 395                 400

Ile Gly Gly Val Tyr Met Asp Leu Tyr Ala Arg Glu Gly Lys Arg Gly
                405                 410                 415

Gly Ala Trp Met Asn Asp Tyr Lys Gly Arg Arg Arg Phe Ala Asp Gly
            420                 425                 430
```

Thr Leu Gln Leu Pro Thr Ala Tyr Leu Val Cys Asn Phe Ala Pro Pro
435 440 445

Val Gly Gly Lys Glu Ala Arg Leu Ser His Asp Glu Ile Leu Thr Leu
450 455 460

Phe His Glu Thr Gly His Gly Leu His His Leu Leu Thr Gln Val Asp
465 470 475 480

Glu Leu Gly Val Ser Gly Ile Asn Gly Val Glu Trp Asp Ala Val Glu
485 490 495

Leu Pro Ser Gln Phe Met Glu Asn Phe Val Trp Glu Tyr Asn Val Leu
500 505 510

Ala Gln Met Ser Ala His Glu Glu Thr Gly Glu Pro Leu Pro Lys Glu
515 520 525

Leu Phe Asp Lys Met Leu Ala Ala Lys Asn Phe Gln Arg Gly Met Phe
530 535 540

Leu Val Arg Gln Met Glu Phe Ala Leu Phe Asp Met Met Ile Tyr Ser
545 550 555 560

Glu Ser Asp Glu Cys Arg Leu Lys Asn Trp Gln Gln Val Leu Asp Ser
565 570 575

Val Arg Lys Glu Val Ala Val Ile Gln Pro Pro Glu Tyr Asn Arg Phe
580 585 590

Ala Asn Ser Phe Gly His Ile Phe Ala Gly Gly Tyr Ser Ala Gly Tyr
595 600 605

Tyr Ser Tyr Ala Trp Ala Glu Val Leu Ser Thr Asp Ala Tyr Ala Ala
610 615 620

Phe Glu Glu Ser Asp Asp Val Ala Ala Thr Gly Lys Arg Phe Trp Gln
625 630 635 640

Glu Ile Leu Ala Val Gly Gly Ser Arg Ser Ala Ala Glu Ser Phe Lys
645 650 655

Ala Phe Arg Gly Arg Glu Pro Ser Ile Asp Ala Leu Leu Arg Gln Ser
660 665 670

Gly Phe Asp Asn Ala Ala
675

<210> 54
<211> 2037
<212> DNA
<213> Neisseria meningitidis

<400> 54
atgactgaca acgcactgct ccatttgggc gaagaacccc gttttgatca aatcaaaacc 60
gaagacatca aacccgccct gcaaaccgcc attgccgaag cgcgcgaaca aatcgccgcc 120
atcaaagccc aaacgcacac cggctgggca aacactgtcg aacccctgac cggcatcacc 180
gaacgcgtcg gcaggatttg ggcgtggtg tcgcacctca actccgtcac cgacacgccc 240
gaactgcgcg ccgcctacaa tgaattaatg cccgaaatta ccgtcttctt caccgaaatc 300
ggacaagaca tcgagctgta caaccgcttc aaaaccatca aaaactcccc cgagttcgac 360

```
accctctccc acgcgcaaaa aaccaaactc aaccacgatc tgcgcgattt cgtcctcagc   420
ggcgcggaac tgccgcccga acagcaggca gaattggcaa aactgcaaac cgaaggcgcg   480
caactttccg ccaaattctc ccaaaacgtc ctagacgcga ccgacgcgtt cggcatttac   540
tttgacgatg ccgcaccgct tgccggcatt cccgaagacg cgctcgccat gtttgccgct   600
gccgcgcaaa gcgaaggcaa aacaggctac aaaatcggtt tgcagattcc gcactacctc   660
gccgtcatcc aatacgccga caaccgcaaa ctgcgcgaac aaatctaccg cgcctacgtt   720
acccgcgcca gcgagctttc agacgacggc aaattcgaca acaccgccaa catcgaccgc   780
acgctcgaaa acgccctgca aaccgccaaa ctgctcggct tcaaaaacta cgccgaattg   840
tcgctggcaa ccaaaatggc ggacaccccc gaacaagttt taaacttcct gcacgacctc   900
gcccgccgcg ccaaacccta cgccgaaaaa gacctcgccg aagtcaaagc cttcgcccgc   960
gaaagcctcg gcctcgccga tttgcaaccg tgggacttgg gctacgccgg cgaaaaactg   1020
cgcgaagcca aatacgcatt cagcgaaacc gaagtcaaaa aatacttccc cgtcggcaaa   1080
gtattaaacg gactgttcgc ccaaatcaaa aaactctacg gcatcggatt taccgaaaaa   1140
accgtccccg tctggcacaa agacgtgcgc tattttgaat tgcaacaaaa cggcgaaacc   1200
ataggcggcg tttatatgga tttgtacgca cgcgaaggca aacgcggcgg cgcgtggatg   1260
aacgactaca aaggccgccg ccgtttttca gacggcacgc tgcaactgcc caccgcctac   1320
ctcgtctgca acttcacccc gcccgtcggc ggcaaagaag cccgcttgag ccatgacgaa   1380
atcctcaccc tcttccacga aaccggacac ggcctgcacc acctgcttac ccaagtcgac   1440
gaactgggcg tatccggcat caacggcgta gaatgggacg cagtcgaact gcccagtcag   1500
tttatggaaa atttcgtttg ggaatacaat gtcttggcgc aaatgtccgc ccacgaagaa   1560
accggcgttc ccctgccgaa agaactcttc gacaaaatgc tcgccgccaa aaacttccaa   1620
cgcggaatgt tcctcgtccg ccaaatggag ttcgccctct ttgatatgat gatttacagc   1680
gaagacgacg aaggccgtct gaaaaactgg caacaggttt tagacagcgt gcgcaaagaa   1740
gtcgccgtcg tccgaccgcc cgaatacaac cgcttcgcca acagcttcgg ccacatcttc   1800
gcaggcggct attccgcagg ctattacagc tacgcgtggg cggaagtatt gagcgcggac   1860
gcatacgccg cctttgaaga aagcgacgat gtcgccgcca caggcaaacg cttttggcag   1920
gaaatcctcg ccgtcggcgg atcgcgcagc gcggcagaat ccttcaaagc cttccgcgga   1980
cgcgaaccga gcatagacgc actcttgcgc cacagcggct tcgacaacgc ggcttga      2037
```

<210> 55
<211> 678
<212> PRT
<213> Neisseria meningitidis

<400> 55

```
Met Thr Asp Asn Ala Leu Leu His Leu Gly Glu Glu Pro Arg Phe Asp
1               5                   10                  15

Gln Ile Lys Thr Glu Asp Ile Lys Pro Ala Leu Gln Thr Ala Ile Ala
            20                  25                  30

Glu Ala Arg Glu Gln Ile Ala Ala Ile Lys Ala Gln Thr His Thr Gly
        35                  40                  45

Trp Ala Asn Thr Val Glu Pro Leu Thr Gly Ile Thr Glu Arg Val Gly
    50                  55                  60

Arg Ile Trp Gly Val Val Ser His Leu Asn Ser Val Thr Asp Thr Pro
65                  70                  75                  80

Glu Leu Arg Ala Ala Tyr Asn Glu Leu Met Pro Glu Ile Thr Val Phe
                85                  90                  95

Phe Thr Glu Ile Gly Gln Asp Ile Glu Leu Tyr Asn Arg Phe Lys Thr
            100                 105                 110

Ile Lys Asn Ser Pro Glu Phe Asp Thr Leu Ser His Ala Gln Lys Thr
        115                 120                 125

Lys Leu Asn His Asp Leu Arg Asp Phe Val Leu Ser Gly Ala Glu Leu
    130                 135                 140
```

```
Pro Pro Glu Gln Gln Ala Glu Leu Ala Lys Leu Gln Thr Glu Gly Ala
145                 150                 155                 160

Gln Leu Ser Ala Lys Phe Ser Gln Asn Val Leu Asp Ala Thr Asp Ala
                165                 170                 175

Phe Gly Ile Tyr Phe Asp Asp Ala Ala Pro Leu Ala Gly Ile Pro Glu
            180                 185                 190

Asp Ala Leu Ala Met Phe Ala Ala Ala Ala Gln Ser Glu Gly Lys Thr
        195                 200                 205

Gly Tyr Lys Ile Gly Leu Gln Ile Pro His Tyr Leu Ala Val Ile Gln
    210                 215                 220

Tyr Ala Asp Asn Arg Lys Leu Arg Glu Gln Ile Tyr Arg Ala Tyr Val
225                 230                 235                 240

Thr Arg Ala Ser Glu Leu Ser Asp Asp Gly Lys Phe Asp Asn Thr Ala
                245                 250                 255

Asn Ile Asp Arg Thr Leu Glu Asn Ala Leu Gln Thr Ala Lys Leu Leu
            260                 265                 270

Gly Phe Lys Asn Tyr Ala Glu Leu Ser Leu Ala Thr Lys Met Ala Asp
        275                 280                 285

Thr Pro Glu Gln Val Leu Asn Phe Leu His Asp Leu Ala Arg Arg Ala
    290                 295                 300

Lys Pro Tyr Ala Glu Lys Asp Leu Ala Glu Val Lys Ala Phe Ala Arg
305                 310                 315                 320

Glu Ser Leu Gly Leu Ala Asp Leu Gln Pro Trp Asp Leu Gly Tyr Ala
                325                 330                 335

Gly Glu Lys Leu Arg Glu Ala Lys Tyr Ala Phe Ser Glu Thr Glu Val
            340                 345                 350

Lys Lys Tyr Phe Pro Val Gly Lys Val Leu Asn Gly Leu Phe Ala Gln
        355                 360                 365

Ile Lys Lys Leu Tyr Gly Ile Gly Phe Thr Glu Lys Thr Val Pro Val
    370                 375                 380

Trp His Lys Asp Val Arg Tyr Phe Glu Leu Gln Gln Asn Gly Glu Thr
385                 390                 395                 400

Ile Gly Gly Val Tyr Met Asp Leu Tyr Ala Arg Glu Gly Lys Arg Gly
                405                 410                 415

Gly Ala Trp Met Asn Asp Tyr Lys Gly Arg Arg Arg Phe Ser Asp Gly
            420                 425                 430

Thr Leu Gln Leu Pro Thr Ala Tyr Leu Val Cys Asn Phe Thr Pro Pro
        435                 440                 445

Val Gly Gly Lys Glu Ala Arg Leu Ser His Asp Glu Ile Leu Thr Leu
    450                 455                 460
```

```
Phe His Glu Thr Gly His Gly Leu His His Leu Leu Thr Gln Val Asp
465                 470                 475                 480

Glu Leu Gly Val Ser Gly Ile Asn Gly Val Glu Trp Asp Ala Val Glu
                485                 490                 495

Leu Pro Ser Gln Phe Met Glu Asn Phe Val Trp Glu Tyr Asn Val Leu
            500                 505                 510

Ala Gln Met Ser Ala His Glu Glu Thr Gly Val Pro Leu Pro Lys Glu
        515                 520                 525

Leu Phe Asp Lys Met Leu Ala Ala Lys Asn Phe Gln Arg Gly Met Phe
    530                 535                 540

Leu Val Arg Gln Met Glu Phe Ala Leu Phe Asp Met Met Ile Tyr Ser
545                 550                 555                 560

Glu Asp Asp Glu Gly Arg Leu Lys Asn Trp Gln Gln Val Leu Asp Ser
                565                 570                 575

Val Arg Lys Glu Val Ala Val Val Arg Pro Pro Glu Tyr Asn Arg Phe
            580                 585                 590

Ala Asn Ser Phe Gly His Ile Phe Ala Gly Gly Tyr Ser Ala Gly Tyr
        595                 600                 605

Tyr Ser Tyr Ala Trp Ala Glu Val Leu Ser Ala Asp Ala Tyr Ala Ala
    610                 615                 620

Phe Glu Glu Ser Asp Asp Val Ala Ala Thr Gly Lys Arg Phe Trp Gln
625                 630                 635                 640

Glu Ile Leu Ala Val Gly Gly Ser Arg Ser Ala Ala Glu Ser Phe Lys
                645                 650                 655

Ala Phe Arg Gly Arg Glu Pro Ser Ile Asp Ala Leu Leu Arg His Ser
            660                 665                 670

Gly Phe Asp Asn Ala Ala
        675

<210>   56
<211>   2037
<212>   DNA
<213>   Neisseria meningitidis

<400>   56
atgactgaca acgcactgct ccatttgggc gaagaacccc gttttgatca aatcaaaacc    60
gaagacatca aacccgccct gcaaaccgcc atcgccgaag cgcgcgaaca aatcgccgcc   120
atcaaagccc aaacgcacac cggctgggca aacactgtcg aacccctgac cggcatcacc   180
gaacgcgtcg gcaggatttg ggcgtggtg tcgcacctca actccgtcgc cgacacgccc   240
gaactgcgcg ccgtctataa cgaactgatg cccgaaatca ccgtcttctt caccgaaatc   300
ggacaagaca tcgagctgta caaccgcttc aaaaccatca aaaattcccc cgaattcgac   360
accctctccc ccgcacaaaa aaccaaactc aaccacgatc tgcgcgattt cgtcctcagc   420
ggcgcggaac tgccgcccga acagcaggca gaactggcaa aactgcaaac cgaaggcgcg   480
caactttccg ccaaattctc ccaaaacgtc ctagacgcga ccgacgcgtt cggcatttac   540
tttgacgatg ccgcaccgct tgccggcatt cccgaagacg cgctcgccat gtttgccgcc   600
gccgcgcaaa gcgaaagcaa aacaggctac aaaatcggct tgcagattcc acactacctc   660
gccgtcatcc aatacgccga caaccgcgaa ctgcgcgaac aaatctaccg cgcctacgtt   720
acccgcgcca gcgaactttc agacgacggc aaattcgaca acaccgccaa catcgaccgc   780
```

```
acgctcgcaa acgccctgca aaccgccaaa ctgctcggct tcaaaaacta cgccgaattg    840
tcgctggcaa ccaaaatggc ggacacgccc gaacaagttt taaacttcct gcacgacctc    900
gcccgccgcg ccaaacccta cgccgaaaaa gacctcgccg aagtcaaagc cttcgcccgc    960
gaaagcctga acctcgccga tttgcaaccg tgggacttgg gctacgccag cgaaaaactg   1020
cgcgaagcca aatacgcgtt cagcgaaacc gaagtcaaaa aatacttccc cgtcggcaaa   1080
gtattaaacg gactgttcgc ccaaatcaaa aaactctacg gcatcggatt taccgaaaaa   1140
accgtccccg tctggcacaa agacgtgcgc tattttgaat tgcaacaaaa cggcgaaacc   1200
ataggcggcg tttatatgga tttgtacgca cgcgaaggca aacgcggcgg cgcgtggatg   1260
aacgactaca aggccgccg ccgtttttca gacggcacgc tgcaactgcc caccgcctac   1320
ctcgtctgca acttcgcccc acccgtcggc ggcagggaag cccgcctgag ccacgacgaa   1380
atcctcatcc tcttccacga aaccggacac gggctgcacc acctgcttac ccaagtggac   1440
gaactgggcg tatccggcat caacggcgta gaatgggacg cggtcgaact gcccagccag   1500
tttatggaaa atttcgtttg ggaatacaat gtcttggcac aaatgtcagc ccacgaagaa   1560
accggcgttc ccctgccgaa agaactcttc gacaaaatgc tcgccgccaa aaacttccaa   1620
cgcggcatgt tcctcgtccg gcaaatggag ttcgccctct ttgatatgat gatttacagc   1680
gaagacgacg aaggccgtct gaaaaactgg caacaggttt tagacagcgt gcgcaaaaaa   1740
gtcgccgtca tccagccgcc cgaatacaac cgcttcgcct tgagcttcgg ccacatcttc   1800
gcaggcggct attccgcagg ctattacagc tacgcgtggg cggaagtatt gagcgcggac   1860
gcatacgccg cctttgaaga aagcgacgat gtcgccgcca caggcaaacg cttttggcag   1920
gaaatcctcg ccgtcggcgg atcgcgcagc gcggcagaat ccttcaaagc cttccgcggc   1980
cgcgaaccga gcatagacgc actcttgcgc cacagcggtt tcgacaacgc ggtctga      2037
```

```
<210>   57
<211>   678
<212>   PRT
<213>   Neisseria meningitidis

<400>   57
Met Thr Asp Asn Ala Leu Leu His Leu Gly Glu Glu Pro Arg Phe Asp
1               5                   10                  15

Gln Ile Lys Thr Glu Asp Ile Lys Pro Ala Leu Gln Thr Ala Ile Ala
            20                  25                  30

Glu Ala Arg Glu Gln Ile Ala Ala Ile Lys Ala Gln Thr His Thr Gly
        35                  40                  45

Trp Ala Asn Thr Val Glu Pro Leu Thr Gly Ile Thr Glu Arg Val Gly
    50                  55                  60

Arg Ile Trp Gly Val Val Ser His Leu Asn Ser Val Ala Asp Thr Pro
65                  70                  75                  80

Glu Leu Arg Ala Val Tyr Asn Glu Leu Met Pro Glu Ile Thr Val Phe
                85                  90                  95

Phe Thr Glu Ile Gly Gln Asp Ile Glu Leu Tyr Asn Arg Phe Lys Thr
            100                 105                 110

Ile Lys Asn Ser Pro Glu Phe Asp Thr Leu Ser Pro Ala Gln Lys Thr
        115                 120                 125

Lys Leu Asn His Asp Leu Arg Asp Phe Val Leu Ser Gly Ala Glu Leu
    130                 135                 140

Pro Pro Glu Gln Gln Ala Glu Leu Ala Lys Leu Gln Thr Glu Gly Ala
145                 150                 155                 160

Gln Leu Ser Ala Lys Phe Ser Gln Asn Val Leu Asp Ala Thr Asp Ala
                165                 170                 175
```

```
Phe Gly Ile Tyr Phe Asp Asp Ala Ala Pro Leu Ala Gly Ile Pro Glu
            180                 185                 190

Asp Ala Leu Ala Met Phe Ala Ala Ala Ala Gln Ser Glu Ser Lys Thr
        195                 200                 205

Gly Tyr Lys Ile Gly Leu Gln Ile Pro His Tyr Leu Ala Val Ile Gln
    210                 215                 220

Tyr Ala Asp Asn Arg Glu Leu Arg Glu Gln Ile Tyr Arg Ala Tyr Val
225                 230                 235                 240

Thr Arg Ala Ser Glu Leu Ser Asp Asp Gly Lys Phe Asp Asn Thr Ala
                245                 250                 255

Asn Ile Asp Arg Thr Leu Ala Asn Ala Leu Gln Thr Ala Lys Leu Leu
            260                 265                 270

Gly Phe Lys Asn Tyr Ala Glu Leu Ser Leu Ala Thr Lys Met Ala Asp
        275                 280                 285

Thr Pro Glu Gln Val Leu Asn Phe Leu His Asp Leu Ala Arg Arg Ala
    290                 295                 300

Lys Pro Tyr Ala Glu Lys Asp Leu Ala Glu Val Lys Ala Phe Ala Arg
305                 310                 315                 320

Glu Ser Leu Asn Leu Ala Asp Leu Gln Pro Trp Asp Leu Gly Tyr Ala
                325                 330                 335

Ser Glu Lys Leu Arg Glu Ala Lys Tyr Ala Phe Ser Glu Thr Glu Val
            340                 345                 350

Lys Lys Tyr Phe Pro Val Gly Lys Val Leu Asn Gly Leu Phe Ala Gln
        355                 360                 365

Ile Lys Lys Leu Tyr Gly Ile Gly Phe Thr Glu Lys Thr Val Pro Val
    370                 375                 380

Trp His Lys Asp Val Arg Tyr Phe Glu Leu Gln Gln Asn Gly Glu Thr
385                 390                 395                 400

Ile Gly Gly Val Tyr Met Asp Leu Tyr Ala Arg Glu Gly Lys Arg Gly
                405                 410                 415

Gly Ala Trp Met Asn Asp Tyr Lys Gly Arg Arg Arg Phe Ser Asp Gly
            420                 425                 430

Thr Leu Gln Leu Pro Thr Ala Tyr Leu Val Cys Asn Phe Ala Pro Pro
        435                 440                 445

Val Gly Gly Arg Glu Ala Arg Leu Ser His Asp Glu Ile Leu Ile Leu
    450                 455                 460

Phe His Glu Thr Gly His Gly Leu His His Leu Leu Thr Gln Val Asp
465                 470                 475                 480

Glu Leu Gly Val Ser Gly Ile Asn Gly Val Glu Trp Asp Ala Val Glu
                485                 490                 495

Leu Pro Ser Gln Phe Met Glu Asn Phe Val Trp Glu Tyr Asn Val Leu
```

```
            500                 505                 510

Ala Gln Met Ser Ala His Glu Glu Thr Gly Val Pro Leu Pro Lys Glu
        515                 520                 525

Leu Phe Asp Lys Met Leu Ala Ala Lys Asn Phe Gln Arg Gly Met Phe
    530                 535                 540

Leu Val Arg Gln Met Glu Phe Ala Leu Phe Asp Met Met Ile Tyr Ser
545                 550                 555                 560

Glu Asp Asp Glu Gly Arg Leu Lys Asn Trp Gln Gln Val Leu Asp Ser
                565                 570                 575

Val Arg Lys Lys Val Ala Val Ile Gln Pro Pro Glu Tyr Asn Arg Phe
            580                 585                 590

Ala Leu Ser Phe Gly His Ile Phe Ala Gly Gly Tyr Ser Ala Gly Tyr
        595                 600                 605

Tyr Ser Tyr Ala Trp Ala Glu Val Leu Ser Ala Asp Ala Tyr Ala Ala
    610                 615                 620

Phe Glu Glu Ser Asp Asp Val Ala Ala Thr Gly Lys Arg Phe Trp Gln
625                 630                 635                 640

Glu Ile Leu Ala Val Gly Gly Ser Arg Ser Ala Ala Glu Ser Phe Lys
                645                 650                 655

Ala Phe Arg Gly Arg Glu Pro Ser Ile Asp Ala Leu Leu Arg His Ser
            660                 665                 670

Gly Phe Asp Asn Ala Val
        675

<210>   58
<211>   1473
<212>   DNA
<213>   Neisseria gonorrhoeae

<400>   58
atgattgaca acgcactgct ccacttgggc gaagaacccc gttttaatca aatcaaaacc   60
gaagacatca aacccgccgt ccaaaccgcc atcgccgaag cgcgcggaca aatcgccgcc   120
gtcaaagcgc aaacgcacac cggctgggcg aacaccgtcg agcgtctgac cggcatcacc   180
gaacgcgtcg gcaggatttg gggcgtcgtg tcccatctca actccgtcgt cgacacgccc   240
gaactgcgcg ccgtctataa cgaactgatg cctgaaatca ccgtcttctt caccgaaatc   300
ggacaagaca tcgaactgta caaccgcttc aaaaccatca aaaattcccc cgaatttgca   360
acgctttccc ccgcacaaaa aaccaagctc gatcacgacc tgcgcgattt cgtattgagc   420
ggcgcggaac tgccgcccga acggcaggca gaactggcaa aactgcaaac cgaaggcgcg   480
caactttccg ccaaattctc ccaaaacgtc ctagacgcga ccgacgcgtt cggcatttac   540
tttgacgatg ccgcaccgct tgccggcatt cccgaagacg cgctcgccat gtttgccgcc   600
gccgcgcaaa gcgaaggcaa aacaggttac aaaatcggct tgcagattcc gcactacctt   660
gccgttatcc aatacgccgg caaccgcgaa ctgcgcgaac aaatctaccg cgcctacgtt   720
acccgtgcca gcgaactttc aaacgacggc aaattcgaca acaccgccaa catcgaccgc   780
acgctcgaaa acgcattgaa aaccgccaaa ctgctcggct ttaaaaatta cgccgaattg   840
tcgctggcaa ccaaaatggc ggacacgccc gaacaggttt taaacttcct gcacgacctc   900
gcccgccgcg ccaaacccta cgccgaaaaa gacctcgccg aagtcaaagc cttcgcccgc   960
gaacacctcg gtctcgccga cccgcagccg tgggacttga gctacgccgg cgaaaaactg   1020
cgcgaagcca aatacgcatt cagcgaaacc gaagtcaaaa aatacttccc cgtcggcaaa   1080
gttctggcag gcctgttcgc ccaaatcaaa aaactctacg gcatcggatt cgccgaaaaa   1140
accgttcccg tctggcacaa agacgtgcgc tattttgaat tgcaacaaaa cggcaaaacc   1200
```

```
atcggcggcg tttatatgga tttgtacgca cgcgaaggca aacgcggcgg cgcgtggatg   1260
aacgactaca aaggccgccg ccgctttgcc gacggcacgc tgcaactgcc caccgcctac   1320
ctcgtctgca acttcgcccc gcccgtcggc ggcaaagaag cgcgtttaag ccacgacgaa   1380
atcctcaccc tcttccacga aaccggccac ggactgcacc acctgcttac ccaagtggac   1440
gaactgggcg tgtccggcat caacggcgta aaa                                1473
```

<210> 59
<211> 491
<212> PRT
<213> Neisseria gonorrhoeae

<400> 59

```
Met Ile Asp Asn Ala Leu Leu His Leu Gly Glu Glu Pro Arg Phe Asn
1               5                   10                  15

Gln Ile Lys Thr Glu Asp Ile Lys Pro Ala Val Gln Thr Ala Ile Ala
            20                  25                  30

Glu Ala Arg Gly Gln Ile Ala Ala Val Lys Ala Gln Thr His Thr Gly
        35                  40                  45

Trp Ala Asn Thr Val Glu Arg Leu Thr Gly Ile Thr Glu Arg Val Gly
    50                  55                  60

Arg Ile Trp Gly Val Val Ser His Leu Asn Ser Val Val Asp Thr Pro
65                  70                  75                  80

Glu Leu Arg Ala Val Tyr Asn Glu Leu Met Pro Glu Ile Thr Val Phe
                85                  90                  95

Phe Thr Glu Ile Gly Gln Asp Ile Glu Leu Tyr Asn Arg Phe Lys Thr
            100                 105                 110

Ile Lys Asn Ser Pro Glu Phe Ala Thr Leu Ser Pro Ala Gln Lys Thr
        115                 120                 125

Lys Leu Asp His Asp Leu Arg Asp Phe Val Leu Ser Gly Ala Glu Leu
    130                 135                 140

Pro Pro Glu Arg Gln Ala Glu Leu Ala Lys Leu Gln Thr Glu Gly Ala
145                 150                 155                 160

Gln Leu Ser Ala Lys Phe Ser Gln Asn Val Leu Asp Ala Thr Asp Ala
                165                 170                 175

Phe Gly Ile Tyr Phe Asp Asp Ala Ala Pro Leu Ala Gly Ile Pro Glu
            180                 185                 190

Asp Ala Leu Ala Met Phe Ala Ala Ala Ala Gln Ser Glu Gly Lys Thr
        195                 200                 205

Gly Tyr Lys Ile Gly Leu Gln Ile Pro His Tyr Leu Ala Val Ile Gln
    210                 215                 220

Tyr Ala Gly Asn Arg Glu Leu Arg Glu Gln Ile Tyr Arg Ala Tyr Val
225                 230                 235                 240

Thr Arg Ala Ser Glu Leu Ser Asn Asp Gly Lys Phe Asp Asn Thr Ala
                245                 250                 255

Asn Ile Asp Arg Thr Leu Glu Asn Ala Leu Lys Thr Ala Lys Leu Leu
```

```
            260                 265                 270

Gly Phe Lys Asn Tyr Ala Glu Leu Ser Leu Ala Thr Lys Met Ala Asp
        275                 280                 285

Thr Pro Glu Gln Val Leu Asn Phe Leu His Asp Leu Ala Arg Arg Ala
    290                 295                 300

Lys Pro Tyr Ala Glu Lys Asp Leu Ala Glu Val Lys Ala Phe Ala Arg
305                 310                 315                 320

Glu His Leu Gly Leu Ala Asp Pro Gln Pro Trp Asp Leu Ser Tyr Ala
                325                 330                 335

Gly Glu Lys Leu Arg Glu Ala Lys Tyr Ala Phe Ser Glu Thr Glu Val
            340                 345                 350

Lys Lys Tyr Phe Pro Val Gly Lys Val Leu Ala Gly Leu Phe Ala Gln
        355                 360                 365

Ile Lys Lys Leu Tyr Gly Ile Gly Phe Ala Glu Lys Thr Val Pro Val
    370                 375                 380

Trp His Lys Asp Val Arg Tyr Phe Glu Leu Gln Gln Asn Gly Lys Thr
385                 390                 395                 400

Ile Gly Gly Val Tyr Met Asp Leu Tyr Ala Arg Glu Gly Lys Arg Gly
                405                 410                 415

Gly Ala Trp Met Asn Asp Tyr Lys Gly Arg Arg Arg Phe Ala Asp Gly
            420                 425                 430

Thr Leu Gln Leu Pro Thr Ala Tyr Leu Val Cys Asn Phe Ala Pro Pro
        435                 440                 445

Val Gly Gly Lys Glu Ala Arg Leu Ser His Asp Glu Ile Leu Thr Leu
    450                 455                 460

Phe His Glu Thr Gly His Gly Leu His His Leu Leu Thr Gln Val Asp
465                 470                 475                 480

Glu Leu Gly Val Ser Gly Ile Asn Gly Val Lys
                485                 490

<210>   60
<211>   2037
<212>   DNA
<213>   Neisseria meningitidis

<400>   60
atgactgaca acgcactgct ccatttgggc gaagaacccc gttttgatca aatcaaaacc   60
gaagacatca aacccgccct gcaaaccgcc attgccgaag cgcgcgaaca aatcgccgcc   120
atcaaagccc aaacgcacac cggctgggca aacactgtcg aacccctgac cggcatcacc   180
gaacgcgtcg gcaggatttg gggcgtggtg tcgcacctca actccgtcac cgacacgccc   240
gaactgcgcg ccgcctacaa tgaattaatg cccgaaatta ccgtcttctt caccgaaatc   300
ggacaagaca tcgagctgta caaccgcttc aaaaccatca aaaactcccc cgagttcgac   360
accctctccc acgcgcaaaa aaccaaactc aaccacgatc tgcgcgattt cgtcctcagc   420
ggcgcggaac tgccgcccga acagcaggca gaattggcaa aactgcaaac cgaaggcgcg   480
caactttccg ccaaattctc ccaaaacgtc ctagacgcga ccgacgcgtt cggcatttac   540
tttgacgatg ccgcaccgct tgccggcatt cccgaagacg cgctcgccat gtttgccgct   600
gccgcgcaaa gcgaaggcaa aacaggctac aaaatcggtt tgcagattcc gcactacctc   660
```

```
gccgtcatcc aatacgccga caaccgcaaa ctgcgcgaac aaatctaccg cgcctacgtt   720
acccgcgcca gcgagctttc agacgacggc aaattcgaca acaccgccaa catcgaccgc   780
acgctcgaaa acgccctgca aaccgccaaa ctgctcggct tcaaaaacta cgccgaattg   840
tcgctggcaa ccaaaatggc ggacaccccc gaacaagttt taaacttcct gcacgacctc   900
gcccgccgcg ccaaacccta cgccgaaaaa gacctcgccg aagtcaaagc cttcgcccgc   960
gaaagcctcg gcctcgccga tttgcaaccg tgggacttgg gctacgccgg cgaaaaactg   1020
cgcgaagcca aatacgcatt cagcgaaacc gaagtcaaaa aatacttccc cgtcggcaaa   1080
gtattaaacg gactgttcgc ccaaatcaaa aaactctacg gcatcggatt taccgaaaaa   1140
accgtccccg tctggcacaa agacgtgcgc tattttgaat tgcaacaaaa cggcgaaacc   1200
ataggcggcg tttatatgga tttgtacgca cgcgaaggca aacgcggcgg cgcgtggatg   1260
aacgactaca aaggccgccg ccgtttttca gacggcacgc tgcaactgcc caccgcctac   1320
ctcgtctgca acttcacccc gcccgtcggc ggcaaagaag cccgcttgag ccatgacgaa   1380
atcctcaccc tcttccacga aaccggacac ggcctgcacc acctgcttac ccaagtcgac   1440
gaactgggcg tatccggcat caacggcgta gaatgggacg cagtcgaact gcccagtcag   1500
tttatggaaa atttcgtttg ggaatacaat gtcttggcgc aaatgtccgc ccacgaagaa   1560
accggcgttc ccctgccgaa agaactcttc gacaaaatgc tcgccgccaa aaacttccaa   1620
cgcggaatgt tcctcgtccg ccaaatggag ttcgccctct ttgatatgat gatttacagc   1680
gaagacgacg aaggccgtct gaaaaactgg caacaggttt tagacagcgt gcgcaaagaa   1740
gtcgccgtcg tccgaccgcc cgaatacaac cgcttcgcca acagcttcgg ccacatcttc   1800
gcaggcggct attccgcagg ctattacagc tacgcgtggg cggaagtatt gagcgcggac   1860
gcatacgccg cctttgaaga aagcgacgat gtcgccgcca caggcaaacg cttttggcag   1920
gaaatcctcg ccgtcggcgg atcgcgcagc gcggcagaat ccttcaaagc cttccgcgga   1980
cgcgaaccga gcatagacgc actcttgcgc cacagcggct tcgacaacgc ggcttga      2037
```

```
<210>   61
<211>   678
<212>   PRT
<213>   Neisseria meningitidis

<400>   61
Met Thr Asp Asn Ala Leu Leu His Leu Gly Glu Glu Pro Arg Phe Asp
1               5                   10                  15

Gln Ile Lys Thr Glu Asp Ile Lys Pro Ala Leu Gln Thr Ala Ile Ala
            20                  25                  30

Glu Ala Arg Glu Gln Ile Ala Ala Ile Lys Ala Gln Thr His Thr Gly
        35                  40                  45

Trp Ala Asn Thr Val Glu Pro Leu Thr Gly Ile Thr Glu Arg Val Gly
    50                  55                  60

Arg Ile Trp Gly Val Val Ser His Leu Asn Ser Val Thr Asp Thr Pro
65                  70                  75                  80

Glu Leu Arg Ala Ala Tyr Asn Glu Leu Met Pro Glu Ile Thr Val Phe
                85                  90                  95

Phe Thr Glu Ile Gly Gln Asp Ile Glu Leu Tyr Asn Arg Phe Lys Thr
            100                 105                 110

Ile Lys Asn Ser Pro Glu Phe Asp Thr Leu Ser His Ala Gln Lys Thr
        115                 120                 125

Lys Leu Asn His Asp Leu Arg Asp Phe Val Leu Ser Gly Ala Glu Leu
    130                 135                 140

Pro Pro Glu Gln Gln Ala Glu Leu Ala Lys Leu Gln Thr Glu Gly Ala
145                 150                 155                 160

Gln Leu Ser Ala Lys Phe Ser Gln Asn Val Leu Asp Ala Thr Asp Ala
```

```
                165                 170                 175

Phe Gly Ile Tyr Phe Asp Asp Ala Ala Pro Leu Ala Gly Ile Pro Glu
            180                 185                 190

Asp Ala Leu Ala Met Phe Ala Ala Ala Ala Gln Ser Glu Gly Lys Thr
        195                 200                 205

Gly Tyr Lys Ile Gly Leu Gln Ile Pro His Tyr Leu Ala Val Ile Gln
    210                 215                 220

Tyr Ala Asp Asn Arg Lys Leu Arg Glu Gln Ile Tyr Arg Ala Tyr Val
225                 230                 235                 240

Thr Arg Ala Ser Glu Leu Ser Asp Asp Gly Lys Phe Asp Asn Thr Ala
                245                 250                 255

Asn Ile Asp Arg Thr Leu Glu Asn Ala Leu Gln Thr Ala Lys Leu Leu
            260                 265                 270

Gly Phe Lys Asn Tyr Ala Glu Leu Ser Leu Ala Thr Lys Met Ala Asp
        275                 280                 285

Thr Pro Glu Gln Val Leu Asn Phe Leu His Asp Leu Ala Arg Arg Ala
    290                 295                 300

Lys Pro Tyr Ala Glu Lys Asp Leu Ala Glu Val Lys Ala Phe Ala Arg
305                 310                 315                 320

Glu Ser Leu Gly Leu Ala Asp Leu Gln Pro Trp Asp Leu Gly Tyr Ala
                325                 330                 335

Gly Glu Lys Leu Arg Glu Ala Lys Tyr Ala Phe Ser Glu Thr Glu Val
            340                 345                 350

Lys Lys Tyr Phe Pro Val Gly Lys Val Leu Asn Gly Leu Phe Ala Gln
        355                 360                 365

Ile Lys Lys Leu Tyr Gly Ile Gly Phe Thr Glu Lys Thr Val Pro Val
    370                 375                 380

Trp His Lys Asp Val Arg Tyr Phe Glu Leu Gln Gln Asn Gly Glu Thr
385                 390                 395                 400

Ile Gly Gly Val Tyr Met Asp Leu Tyr Ala Arg Glu Gly Lys Arg Gly
                405                 410                 415

Gly Ala Trp Met Asn Asp Tyr Lys Gly Arg Arg Arg Phe Ser Asp Gly
            420                 425                 430

Thr Leu Gln Leu Pro Thr Ala Tyr Leu Val Cys Asn Phe Thr Pro Pro
        435                 440                 445

Val Gly Gly Lys Glu Ala Arg Leu Ser His Asp Glu Ile Leu Thr Leu
    450                 455                 460

Phe His Glu Thr Gly His Gly Leu His His Leu Leu Thr Gln Val Asp
465                 470                 475                 480

Glu Leu Gly Val Ser Gly Ile Asn Gly Val Glu Trp Asp Ala Val Glu
                485                 490                 495
```

Leu Pro Ser Gln Phe Met Glu Asn Phe Val Trp Glu Tyr Asn Val Leu
500 505 510

Ala Gln Met Ser Ala His Glu Glu Thr Gly Val Pro Leu Pro Lys Glu
515 520 525

Leu Phe Asp Lys Met Leu Ala Ala Lys Asn Phe Gln Arg Gly Met Phe
530 535 540

Leu Val Arg Gln Met Glu Phe Ala Leu Phe Asp Met Met Ile Tyr Ser
545 550 555 560

Glu Asp Asp Glu Gly Arg Leu Lys Asn Trp Gln Gln Val Leu Asp Ser
565 570 575

Val Arg Lys Glu Val Ala Val Val Arg Pro Pro Glu Tyr Asn Arg Phe
580 585 590

Ala Asn Ser Phe Gly His Ile Phe Ala Gly Gly Tyr Ser Ala Gly Tyr
595 600 605

Tyr Ser Tyr Ala Trp Ala Glu Val Leu Ser Ala Asp Ala Tyr Ala Ala
610 615 620

Phe Glu Glu Ser Asp Asp Val Ala Ala Thr Gly Lys Arg Phe Trp Gln
625 630 635 640

Glu Ile Leu Ala Val Gly Gly Ser Arg Ser Ala Ala Glu Ser Phe Lys
645 650 655

Ala Phe Arg Gly Arg Glu Pro Ser Ile Asp Ala Leu Leu Arg His Ser
660 665 670

Gly Phe Asp Asn Ala Ala
675

<210> 62
<211> 534
<212> DNA
<213> Neisseria meningitidis

<400> 62
atgtttcccc ccgacaaaac ccttttcctc tgtctcagcg cactgctcct cgcctcatgc 60
ggcacgacct ccggcaaaca ccgccaaccg aaacccaaac agacagtccg gcaaatccaa 120
gccgtccgca tcagccacat cgaccgcaca caaggctcgc aggaactcat gctccacagc 180
ctcggactca tcggcacgcc ctacaaatgg ggcggcagca gcaccgcaac cggcttcgat 240
tgcagcggca tgattcaatt cgtttacaar aacgccctca acgtcaagct gccgcgcacc 300
gcccgcgaca tggcggcggc aagccgsaaa atccccgaca gccgcytcaa ggccggcgac 360
ctcgtattct tcaacaccgg cggcgcacac cgctactcac acgtcggact ctacatcggc 420
aacggcgaat tcatccatgc ccccagcagc ggcaaaacca tcaaaaccga aaaactctcc 480
acaccgtttt acgccaaaaa ctacctcggc gcacatactt tttttacaga atga 534

<210> 63
<211> 177
<212> PRT
<213> Neisseria meningitidis

<400> 63
Met Phe Pro Pro Asp Lys Thr Leu Phe Leu Cys Leu Ser Ala Leu Leu
1 5 10 15

```
Leu Ala Ser Cys Gly Thr Thr Ser Gly Lys His Arg Gln Pro Lys Pro
            20                  25                  30

Lys Gln Thr Val Arg Gln Ile Gln Ala Val Arg Ile Ser His Ile Asp
        35                  40                  45

Arg Thr Gln Gly Ser Gln Glu Leu Met Leu His Ser Leu Gly Leu Ile
    50                  55                  60

Gly Thr Pro Tyr Lys Trp Gly Gly Ser Ser Thr Ala Thr Gly Phe Asp
65                  70                  75                  80

Cys Ser Gly Met Ile Gln Phe Val Tyr Lys Asn Ala Leu Asn Val Lys
                85                  90                  95

Leu Pro Arg Thr Ala Arg Asp Met Ala Ala Ala Ser Arg Lys Ile Pro
            100                 105                 110

Asp Ser Arg Xaa Lys Ala Gly Asp Leu Val Phe Phe Asn Thr Gly Gly
        115                 120                 125

Ala His Arg Tyr Ser His Val Gly Leu Tyr Ile Gly Asn Gly Glu Phe
    130                 135                 140

Ile His Ala Pro Ser Ser Gly Lys Thr Ile Lys Thr Glu Lys Leu Ser
145                 150                 155                 160

Thr Pro Phe Tyr Ala Lys Asn Tyr Leu Gly Ala His Thr Phe Phe Thr
                165                 170                 175

Glu


<210>   64
<211>   534
<212>   DNA
<213>   Neisseria gonorrhoeae

<400>   64
atgttttccc ccgacaaaac ccttttcctc tgtctcggcg cactgctcct cgcctcatgc   60
ggcacgacct ccggcaaaca ccgccaaccg aaacccaaac agacagtccg gcaaatccaa   120
gccgtccgca tcagccacat cggccgcaca caaggctcgc aggaactcat gctccacagc   180
ctcggactca tcggcacgcc ctacaaatgg ggcggcagca gcaccgcaac cggcttcgac   240
tgcagcggca tgattcaatt ggtttacaaa aacgccctca acgtcaagct gccgcgcacc   300
gcccgcgaca tggcggcggc aagccgcaaa atccccgaca gccgcctcaa ggccggcgac   360
atcgtattct tcaacaccgg cggcgcacac cgctactcac acgtcggact ctacatcggc   420
aacggcgaat tcatccatgc ccccggcagc ggcaaaacca tcaaaaccga aaaactctcc   480
acaccgtttt acgccaaaaa ctaccttgga gcgcatacgt tttttacaga atga         534

<210>   65
<211>   177
<212>   PRT
<213>   Neisseria gonorrhoeae

<400>   65
Met Phe Ser Pro Asp Lys Thr Leu Phe Leu Cys Leu Gly Ala Leu Leu
1               5                   10                  15

Leu Ala Ser Cys Gly Thr Thr Ser Gly Lys His Arg Gln Pro Lys Pro
            20                  25                  30
```

```
Lys Gln Thr Val Arg Gln Ile Gln Ala Val Arg Ile Ser His Ile Gly
        35                  40                  45

Arg Thr Gln Gly Ser Gln Glu Leu Met Leu His Ser Leu Gly Leu Ile
    50                  55                  60

Gly Thr Pro Tyr Lys Trp Gly Gly Ser Ser Thr Ala Thr Gly Phe Asp
65                  70                  75                  80

Cys Ser Gly Met Ile Gln Leu Val Tyr Lys Asn Ala Leu Asn Val Lys
                85                  90                  95

Leu Pro Arg Thr Ala Arg Asp Met Ala Ala Ala Ser Arg Lys Ile Pro
            100                 105                 110

Asp Ser Arg Leu Lys Ala Gly Asp Ile Val Phe Phe Asn Thr Gly Gly
        115                 120                 125

Ala His Arg Tyr Ser His Val Gly Leu Tyr Ile Gly Asn Gly Glu Phe
    130                 135                 140

Ile His Ala Pro Gly Ser Gly Lys Thr Ile Lys Thr Glu Lys Leu Ser
145                 150                 155                 160

Thr Pro Phe Tyr Ala Lys Asn Tyr Leu Gly Ala His Thr Phe Phe Thr
                165                 170                 175

Glu


<210>   66
<211>   534
<212>   DNA
<213>   Neisseria meningitidis

<400>   66
atgtttcccc ccgacaaaac ccttttcctc tgtctcagcg cactgctcct cgcctcatgc    60
ggcacgacct ccggcaaaca ccgccaaccg aaacccaaac agacagtccg gcaaatccaa   120
gccgtccgca tcagccacat cgaccgcaca caaggctcgc aggaactcat gctccacagc   180
ctcggactca tcggcacgcc ctacaaatgg ggcggcagca gcaccgcaac cggcttcgat   240
tgcagcggca tgattcaatt cgtttacaaa aacgccctca acgtcaagct gccgcgcacc   300
gcccgcgaca tggcggcggc aagccgcaaa atccccgaca gccgccttaa ggccggcgac   360
ctcgtattct tcaacaccgg cggcgcacac cgctactcac acgtcggact ctatatcggc   420
aacggcgaat tcatccatgc ccccagcagc ggcaaaacca tcaaaaccga aaaactctcc   480
acaccgtttt acgccaaaaa ctacctcggc gcacatactt tctttacaga atga         534

<210>   67
<211>   177
<212>   PRT
<213>   Neisseria meningitidis

<400>   67
Met Phe Pro Pro Asp Lys Thr Leu Phe Leu Cys Leu Ser Ala Leu Leu
1               5                   10                  15

Leu Ala Ser Cys Gly Thr Thr Ser Gly Lys His Arg Gln Pro Lys Pro
            20                  25                  30

Lys Gln Thr Val Arg Gln Ile Gln Ala Val Arg Ile Ser His Ile Asp
        35                  40                  45
```

```
Arg Thr Gln Gly Ser Gln Glu Leu Met Leu His Ser Leu Gly Leu Ile
    50                  55                  60

Gly Thr Pro Tyr Lys Trp Gly Gly Ser Ser Thr Ala Thr Gly Phe Asp
65                  70                  75                  80

Cys Ser Gly Met Ile Gln Phe Val Tyr Lys Asn Ala Leu Asn Val Lys
                85                  90                  95

Leu Pro Arg Thr Ala Arg Asp Met Ala Ala Ala Ser Arg Lys Ile Pro
            100                 105                 110

Asp Ser Arg Leu Lys Ala Gly Asp Leu Val Phe Phe Asn Thr Gly Gly
        115                 120                 125

Ala His Arg Tyr Ser His Val Gly Leu Tyr Ile Gly Asn Gly Glu Phe
    130                 135                 140

Ile His Ala Pro Ser Ser Gly Lys Thr Ile Lys Thr Glu Lys Leu Ser
145                 150                 155                 160

Thr Pro Phe Tyr Ala Lys Asn Tyr Leu Gly Ala His Thr Phe Phe Thr
                165                 170                 175

Glu


<210>   68
<211>   1467
<212>   DNA
<213>   Neisseria meningitidis

<400>   68
atgtttaaac gcagcgtaat cgcaatggct tgtatttttg ccctttcagc ctgcgggggc   60
ggcggtggcg gatcgcccga tgtcaagtcg gcggacacgc tgtcaaaacc tgccgcccct   120
gttgtttctg aaaaagagac agaggcaaag gaagatgcgc cacaggcagg ttctcaagga   180
cagggcgcgc catccgcaca aggcagtcaa gatatggcgg cggtttcgga agaaaataca   240
ggcaatggcg gtgcggtaac agcggataat cccaaaaatg aagacgaggt ggcacaaaat   300
gatatgccgc aaaatgccgc cggtacagat agttcgacac cgaatcacac cccggatccg   360
aatatgcttg ccggaaatat ggaaaatcaa gcaacggatg ccggggaatc gtctcagccg   420
gcaaaccaac cggatatggc aaatgcggcg gacggaatgc aggggggacga tccgtcggca   480
ggcgggcaaa atgccggcaa tacggctgcc caaggtgcaa atcaagccgg aaacaatcaa   540
gccgccggtt cttcagatcc catccccgcg tcaaaccctg cacctgcgaa tggcggtagc   600
aattttggaa gggttgattt ggctaatggc gttttgattg acgggccgtc gcaaaatata   660
acgttgaccc actgtaaagg cgattcttgt agtggcaata atttcttgga tgaagaagta   720
cagctaaaat cagaatttga aaaattaagt gatgcagaca aaataagtaa ttacaagaaa   780
gatgggaaga atgataaatt tgtcggtttg gttgccgata gtgtgcagat gaagggaatc   840
aatcaatata ttatctttta taaacctaaa cccacttcat ttgcgcgatt taggcgttct   900
gcacggtcga ggcggtcgct tccggccgag atgccgctga ttcccgtcaa tcaggcggat   960
acgctgattg tcgatgggga agcggtcagc ctgacggggc attccggcaa tatcttcgcg   1020
cccgaaggga attaccggta tctgacttac ggggcggaaa aattgcccgg cggatcgtat   1080
gcccttcgtg ttcaaggcga accggcaaaa ggcgaaatgc ttgcgggcgc ggccgtgtac   1140
aacggcgaag tactgcattt ccatacggaa aacggccgtc cgtacccgac caggggcagg   1200
tttgccgcaa aagtcgattt cggcagcaaa tctgtggacg gcattatcga cagcggcgat   1260
gatttgcata tgggtacgca aaaattcaaa gccgccatcg atggaaacgg ctttaagggg   1320
acttggacgg aaaatggcag cggggatgtt tccggaaagt tttacggccc ggccggcgag   1380
gaagtggcgg gaaaatacag ctatcgcccg acagatgcgg aaaagggcgg attcggcgtg   1440
tttgccggca aaaaagagca ggattga                                       1467

<210>   69
```

<211> 488
<212> PRT
<213> Neisseria meningitidis

<400> 69

```
Met Phe Lys Arg Ser Val Ile Ala Met Ala Cys Ile Phe Ala Leu Ser
1               5                   10                  15

Ala Cys Gly Gly Gly Gly Gly Gly Ser Pro Asp Val Lys Ser Ala Asp
            20                  25                  30

Thr Leu Ser Lys Pro Ala Ala Pro Val Val Ser Glu Lys Glu Thr Glu
        35                  40                  45

Ala Lys Glu Asp Ala Pro Gln Ala Gly Ser Gln Gly Gln Gly Ala Pro
    50                  55                  60

Ser Ala Gln Gly Ser Gln Asp Met Ala Ala Val Ser Glu Glu Asn Thr
65                  70                  75                  80

Gly Asn Gly Gly Ala Val Thr Ala Asp Asn Pro Lys Asn Glu Asp Glu
                85                  90                  95

Val Ala Gln Asn Asp Met Pro Gln Asn Ala Ala Gly Thr Asp Ser Ser
            100                 105                 110

Thr Pro Asn His Thr Pro Asp Pro Asn Met Leu Ala Gly Asn Met Glu
        115                 120                 125

Asn Gln Ala Thr Asp Ala Gly Glu Ser Ser Gln Pro Ala Asn Gln Pro
    130                 135                 140

Asp Met Ala Asn Ala Ala Asp Gly Met Gln Gly Asp Asp Pro Ser Ala
145                 150                 155                 160

Gly Gly Gln Asn Ala Gly Asn Thr Ala Ala Gln Gly Ala Asn Gln Ala
                165                 170                 175

Gly Asn Asn Gln Ala Ala Gly Ser Ser Asp Pro Ile Pro Ala Ser Asn
            180                 185                 190

Pro Ala Pro Ala Asn Gly Gly Ser Asn Phe Gly Arg Val Asp Leu Ala
        195                 200                 205

Asn Gly Val Leu Ile Asp Gly Pro Ser Gln Asn Ile Thr Leu Thr His
    210                 215                 220

Cys Lys Gly Asp Ser Cys Ser Gly Asn Asn Phe Leu Asp Glu Glu Val
225                 230                 235                 240

Gln Leu Lys Ser Glu Phe Glu Lys Leu Ser Asp Ala Asp Lys Ile Ser
                245                 250                 255

Asn Tyr Lys Lys Asp Gly Lys Asn Asp Lys Phe Val Gly Leu Val Ala
            260                 265                 270

Asp Ser Val Gln Met Lys Gly Ile Asn Gln Tyr Ile Ile Phe Tyr Lys
        275                 280                 285

Pro Lys Pro Thr Ser Phe Ala Arg Phe Arg Arg Ser Ala Arg Ser Arg
    290                 295                 300
```

Arg Ser Leu Pro Ala Glu Met Pro Leu Ile Pro Val Asn Gln Ala Asp
305 310 315 320

Thr Leu Ile Val Asp Gly Glu Ala Val Ser Leu Thr Gly His Ser Gly
325 330 335

Asn Ile Phe Ala Pro Glu Gly Asn Tyr Arg Tyr Leu Thr Tyr Gly Ala
340 345 350

Glu Lys Leu Pro Gly Gly Ser Tyr Ala Leu Arg Val Gln Gly Glu Pro
355 360 365

Ala Lys Gly Glu Met Leu Ala Gly Ala Ala Val Tyr Asn Gly Glu Val
370 375 380

Leu His Phe His Thr Glu Asn Gly Arg Pro Tyr Pro Thr Arg Gly Arg
385 390 395 400

Phe Ala Ala Lys Val Asp Phe Gly Ser Lys Ser Val Asp Gly Ile Ile
405 410 415

Asp Ser Gly Asp Asp Leu His Met Gly Thr Gln Lys Phe Lys Ala Ala
420 425 430

Ile Asp Gly Asn Gly Phe Lys Gly Thr Trp Thr Glu Asn Gly Ser Gly
435 440 445

Asp Val Ser Gly Lys Phe Tyr Gly Pro Ala Gly Glu Glu Val Ala Gly
450 455 460

Lys Tyr Ser Tyr Arg Pro Thr Asp Ala Glu Lys Gly Gly Phe Gly Val
465 470 475 480

Phe Ala Gly Lys Lys Glu Gln Asp
485

<210> 70
<211> 1290
<212> DNA
<213> Neisseria gonorrhoeae

<400> 70
atgtttaaac gcagtgtgat tgcaatggct tgtatttttc ccctttcagc ctgtgggggc 60
ggcggtggcg gatcgcccga tgtcaagtcg gcggacacgc cgtcaaaacc ggccgccccc 120
gttgttgctg aaaatgccgg ggaaggggtg ctgccgaaag aaaagaaaga tgaggaggca 180
gcgggcggtg cgccgcaagc cgatacgcag gacgcaaccg ccggagaagg cagccaagat 240
atggcggcag tttcggcaga aaatacaggc aatggcggtg cggcaacaac ggacaacccc 300
aaaaatgaag acgcgggggc gcaaaatgat atgccgcaaa atgccgccga atccgcaaat 360
caaacaggga acaaccaacc cgccggttct tcagattccg cccccgcgtc aaaccctgcc 420
cctgcgaatg gcggtagcga ttttggaagg acgaacgtgg gcaattctgt tgtgattgac 480
ggaccgtcgc aaaatataac gttgacccac tgtaaaggcg attcttgtaa tggtgataat 540
ttattggatg aagaagcacc gtcaaaatca gaatttgaaa aattaagtga tgaagaaaaa 600
attaagcgat ataaaaaaga cgagcaacgg gagaattttg tcggtttggt tgctgacagg 660
gtaaaaaagg atggaactaa caaatatatc atcttctata cggacaaacc acctactcgt 720
tctgcacggt cgaggaggtc gcttccggcc gagattccgc tgattcccgt caatcaggcc 780
gatacgctga ttgtggatgg ggaagcggtc agcctgacgg ggcattccgg caatatcttc 840
gcgcccgaag ggaattaccg gtatctgact tacggggcgg aaaaattgcc cggcggatcg 900
tatgccctcc gtgtgcaagg cgaaccggca aaaggcgaaa tgcttgttgg cacggccgtg 960
tacaacggcg aagtgctgca tttccatatg gaaaacggcc gtccgtaccc gtccggaggc 1020
aggtttgccg caaaagtcga tttcggcagc aaatctgtgg acggcattat cgacagcggc 1080

gatgatttgc atatgggtac gcaaaaattc aaagccgcca tcgatggaaa cggctttaag 1140
gggacttgga cggaaaatgg cggcggggat gtttccggaa ggttttacgg cccggccggc 1200
gaggaagtgg cgggaaaata cagctatcgc ccgacagatg ctgaaaaggg cggattcggc 1260
gtgtttgccg gcaaaaaaga tcgggattga 1290

<210> 71
<211> 429
<212> PRT
<213> Neisseria gonorrhoeae

<400> 71
Met Phe Lys Arg Ser Val Ile Ala Met Ala Cys Ile Phe Pro Leu Ser
1 5 10 15

Ala Cys Gly Gly Gly Gly Gly Gly Ser Pro Asp Val Lys Ser Ala Asp
20 25 30

Thr Pro Ser Lys Pro Ala Ala Pro Val Val Ala Glu Asn Ala Gly Glu
35 40 45

Gly Val Leu Pro Lys Glu Lys Lys Asp Glu Glu Ala Ala Gly Gly Ala
50 55 60

Pro Gln Ala Asp Thr Gln Asp Ala Thr Ala Gly Glu Gly Ser Gln Asp
65 70 75 80

Met Ala Ala Val Ser Ala Glu Asn Thr Gly Asn Gly Gly Ala Ala Thr
85 90 95

Thr Asp Asn Pro Lys Asn Glu Asp Ala Gly Ala Gln Asn Asp Met Pro
100 105 110

Gln Asn Ala Ala Glu Ser Ala Asn Gln Thr Gly Asn Asn Gln Pro Ala
115 120 125

Gly Ser Ser Asp Ser Ala Pro Ala Ser Asn Pro Ala Pro Ala Asn Gly
130 135 140

Gly Ser Asp Phe Gly Arg Thr Asn Val Gly Asn Ser Val Val Ile Asp
145 150 155 160

Gly Pro Ser Gln Asn Ile Thr Leu Thr His Cys Lys Gly Asp Ser Cys
165 170 175

Asn Gly Asp Asn Leu Leu Asp Glu Glu Ala Pro Ser Lys Ser Glu Phe
180 185 190

Glu Lys Leu Ser Asp Glu Glu Lys Ile Lys Arg Tyr Lys Lys Asp Glu
195 200 205

Gln Arg Glu Asn Phe Val Gly Leu Val Ala Asp Arg Val Lys Lys Asp
210 215 220

Gly Thr Asn Lys Tyr Ile Ile Phe Tyr Thr Asp Lys Pro Pro Thr Arg
225 230 235 240

Ser Ala Arg Ser Arg Arg Ser Leu Pro Ala Glu Ile Pro Leu Ile Pro
245 250 255

Val Asn Gln Ala Asp Thr Leu Ile Val Asp Gly Glu Ala Val Ser Leu
260 265 270

Thr Gly His Ser Gly Asn Ile Phe Ala Pro Glu Gly Asn Tyr Arg Tyr
275 280 285

Leu Thr Tyr Gly Ala Glu Lys Leu Pro Gly Gly Ser Tyr Ala Leu Arg
290 295 300

Val Gln Gly Glu Pro Ala Lys Gly Glu Met Leu Val Gly Thr Ala Val
305 310 315 320

Tyr Asn Gly Glu Val Leu His Phe His Met Glu Asn Gly Arg Pro Tyr
325 330 335

Pro Ser Gly Gly Arg Phe Ala Ala Lys Val Asp Phe Gly Ser Lys Ser
340 345 350

Val Asp Gly Ile Ile Asp Ser Gly Asp Asp Leu His Met Gly Thr Gln
355 360 365

Lys Phe Lys Ala Ala Ile Asp Gly Asn Gly Phe Lys Gly Thr Trp Thr
370 375 380

Glu Asn Gly Gly Gly Asp Val Ser Gly Arg Phe Tyr Gly Pro Ala Gly
385 390 395 400

Glu Glu Val Ala Gly Lys Tyr Ser Tyr Arg Pro Thr Asp Ala Glu Lys
405 410 415

Gly Gly Phe Gly Val Phe Ala Gly Lys Lys Asp Arg Asp
420 425

<210> 72
<211> 1494
<212> DNA
<213> Neisseria meningitidis

<400> 72
atgtttaaac gcagtgtgat tgcaatggct tgtattgttg ccctttcagc ctgtgggggc 60
ggcggtggcg gatcgcccga tgttaagtcg gcggacacgc tgtcaaaacc tgccgcccct 120
gttgttactg aagatgtcgg ggaagaggtg ctgccgaaag aaaagaaaga tgaggaggcg 180
gtgagtggtg cgccgcaagc cgatacgcag gacgcaaccg ccggaaaagg cggtcaagat 240
atggcggcag tttcggcaga aaatacaggc aatggcggtg cggcaacaac ggataatccc 300
gaaaataaag acgagggacc gcaaaatgat atgccgcaaa atgccgccga tacagatagt 360
tcgacaccga atcacacccc tgcaccgaat atgccaacca gagatatggg aaaccaagca 420
ccggatgccg gggaatcggc acaaccggca aaccaaccgg atatggcaaa tgcggcggac 480
ggaatgcagg gggacgatcc gtcggcaggg gaaaatgccg gcaatacggc agatcaagct 540
gcaaatcaag ctgaaaacaa tcaagtcggc ggctctcaaa atcctgcctc ttcaaccaat 600
cctaacgcca cgaatggcgg cagcgatttt ggaaggataa atgtagctaa tggcatcaag 660
cttgacagcg gttcggaaaa tgtaacgttg acacattgta aagacaaagt atgcgataga 720
gatttcttag atgaagaagc accaccaaaa tcagaatttg aaaaattaag tgatgaagaa 780
aaaattaata aatataaaaa agacgagcaa cgagagaatt ttgtcggttt ggttgctgac 840
agggtagaaa agaatggaac taacaaatat gtcatcattt ataaagacaa gtccgcttca 900
tcttcatctg cgcgattcag gcgttctgca cggtcgaggc ggtcgcttcc ggccgagatg 960
ccgctgattc ccgtcaatca ggcggatacg ctgattgtcg atggggaagc ggtcagcctg 1020
acggggcatt ccggcaatat cttcgcgccc gaagggaatt accggtatct gacttacggg 1080
gcggaaaaat tgtccggcgg atcgtatgcc ctcagtgtgc aaggcgaacc ggcaaaaggc 1140
gaaatgcttg cgggcacggc cgtgtacaac ggcgaagtgc tgcatttcca tatggaaaac 1200
ggccgtccgt ccccgtccgg aggcaggttt gccgcaaaag tcgatttcgg cagcaaatct 1260
gtggacggca ttatcgacag cggcgatgat ttgcatatgg gtacgcaaaa attcaaagcc 1320
gttatcgatg gaaacggctt taaggggact tggacggaaa atggcggcgg ggatgtttcc 1380
ggaaggtttt acggcccggc cggcgaagaa gtggcgggaa aatacagcta tcgcccgaca 1440

gatgcggaaa agggcggatt cggcgtgttt gccggcaaaa aagagcagga ttga 1494

<210> 73
<211> 497
<212> PRT
<213> Neisseria meningitidis

<400> 73
Met Phe Lys Arg Ser Val Ile Ala Met Ala Cys Ile Val Ala Leu Ser
1 5 10 15

Ala Cys Gly Gly Gly Gly Gly Gly Ser Pro Asp Val Lys Ser Ala Asp
20 25 30

Thr Leu Ser Lys Pro Ala Ala Pro Val Val Thr Glu Asp Val Gly Glu
35 40 45

Glu Val Leu Pro Lys Glu Lys Lys Asp Glu Glu Ala Val Ser Gly Ala
50 55 60

Pro Gln Ala Asp Thr Gln Asp Ala Thr Ala Gly Lys Gly Gly Gln Asp
65 70 75 80

Met Ala Ala Val Ser Ala Glu Asn Thr Gly Asn Gly Gly Ala Ala Thr
85 90 95

Thr Asp Asn Pro Glu Asn Lys Asp Glu Gly Pro Gln Asn Asp Met Pro
100 105 110

Gln Asn Ala Ala Asp Thr Asp Ser Ser Thr Pro Asn His Thr Pro Ala
115 120 125

Pro Asn Met Pro Thr Arg Asp Met Gly Asn Gln Ala Pro Asp Ala Gly
130 135 140

Glu Ser Ala Gln Pro Ala Asn Gln Pro Asp Met Ala Asn Ala Ala Asp
145 150 155 160

Gly Met Gln Gly Asp Asp Pro Ser Ala Gly Glu Asn Ala Gly Asn Thr
165 170 175

Ala Asp Gln Ala Ala Asn Gln Ala Glu Asn Asn Gln Val Gly Gly Ser
180 185 190

Gln Asn Pro Ala Ser Ser Thr Asn Pro Asn Ala Thr Asn Gly Gly Ser
195 200 205

Asp Phe Gly Arg Ile Asn Val Ala Asn Gly Ile Lys Leu Asp Ser Gly
210 215 220

Ser Glu Asn Val Thr Leu Thr His Cys Lys Asp Lys Val Cys Asp Arg
225 230 235 240

Asp Phe Leu Asp Glu Glu Ala Pro Pro Lys Ser Glu Phe Glu Lys Leu
245 250 255

Ser Asp Glu Glu Lys Ile Asn Lys Tyr Lys Lys Asp Glu Gln Arg Glu
260 265 270

Asn Phe Val Gly Leu Val Ala Asp Arg Val Glu Lys Asn Gly Thr Asn
275 280 285

Lys Tyr Val Ile Ile Tyr Lys Asp Lys Ser Ala Ser Ser Ser Ser Ala
    290                 295                 300

Arg Phe Arg Arg Ser Ala Arg Ser Arg Arg Ser Leu Pro Ala Glu Met
305                 310                 315                 320

Pro Leu Ile Pro Val Asn Gln Ala Asp Thr Leu Ile Val Asp Gly Glu
                325                 330                 335

Ala Val Ser Leu Thr Gly His Ser Gly Asn Ile Phe Ala Pro Glu Gly
            340                 345                 350

Asn Tyr Arg Tyr Leu Thr Tyr Gly Ala Glu Lys Leu Ser Gly Gly Ser
        355                 360                 365

Tyr Ala Leu Ser Val Gln Gly Glu Pro Ala Lys Gly Glu Met Leu Ala
    370                 375                 380

Gly Thr Ala Val Tyr Asn Gly Glu Val Leu His Phe His Met Glu Asn
385                 390                 395                 400

Gly Arg Pro Ser Pro Ser Gly Gly Arg Phe Ala Ala Lys Val Asp Phe
                405                 410                 415

Gly Ser Lys Ser Val Asp Gly Ile Ile Asp Ser Gly Asp Asp Leu His
            420                 425                 430

Met Gly Thr Gln Lys Phe Lys Ala Val Ile Asp Gly Asn Gly Phe Lys
        435                 440                 445

Gly Thr Trp Thr Glu Asn Gly Gly Gly Asp Val Ser Gly Arg Phe Tyr
    450                 455                 460

Gly Pro Ala Gly Glu Glu Val Ala Gly Lys Tyr Ser Tyr Arg Pro Thr
465                 470                 475                 480

Asp Ala Glu Lys Gly Gly Phe Gly Val Phe Ala Gly Lys Lys Glu Gln
                485                 490                 495

Asp

<210> 74
<211> 963
<212> DNA
<213> Neisseria meningitidis

<400> 74
atgcaagcac ggctgctgat acctattctt ttttcagttt ttattttatc cgcctgcggg 60
acactgacag gtattccatc gcatggcgga ggtaaacgct ttgcggtcga acaagaactt 120
gtggccgctt ctgccagagc tgccgttaaa gacatggatt tacaggcatt acacggacga 180
aaagttgcat tgtacattgc cactatgggc gaccaaggtt caggcagttt gacagggggt 240
cgctactcca ttgatgcact gattcgtggc gaatacataa acagccctgc cgtccgtacc 300
gattacacct atccacgtta cgaaaccacc gctgaaacaa catcaggcgg tttgacaggt 360
ttaaccactt ctttatctac acttaatgcc cctgcactct ctcgcaccca atcagacggt 420
agcggaagta aaagcagtct gggcttaaat attggcggga tgggggatta tcgaaatgaa 480
accttgacga ctaacccgcg cgacactgcc tttctttccc acttggtaca gaccgtattt 540
ttcctgcgcg gcatagacgt tgtttctcct gccaatgccg atacagatgt gtttattaac 600
atcgacgtat tcggaacgat acgcaacaga accgaaatgc acctatacaa tgccgaaaca 660
ctgaaagccc aaacaaaact ggaatatttc gcagtagaca gaaccaataa aaaattgctc 720

```
atcaaaccaa aaaccaatgc gtttgaagct gcctataaag aaaattacgc attgtggatg   780
gggccgtata aagtaagcaa aggaattaaa ccgacggaag gattaatggt cgatttctcc   840
gatatccgac catacggcaa tcatacgggt aactccgccc catccgtaga ggctgataac   900
agtcatgagg ggtatggata cagcgatgaa gtagtgcgac aacatagaca aggacaacct   960
tga                                                                 963

<210>   75
<211>   320
<212>   PRT
<213>   Neisseria meningitidis

<400>   75
Met Gln Ala Arg Leu Leu Ile Pro Ile Leu Phe Ser Val Phe Ile Leu
1               5                   10                  15

Ser Ala Cys Gly Thr Leu Thr Gly Ile Pro Ser His Gly Gly Gly Lys
            20                  25                  30

Arg Phe Ala Val Glu Gln Glu Leu Val Ala Ala Ser Ala Arg Ala Ala
        35                  40                  45

Val Lys Asp Met Asp Leu Gln Ala Leu His Gly Arg Lys Val Ala Leu
    50                  55                  60

Tyr Ile Ala Thr Met Gly Asp Gln Gly Ser Gly Ser Leu Thr Gly Gly
65                  70                  75                  80

Arg Tyr Ser Ile Asp Ala Leu Ile Arg Gly Glu Tyr Ile Asn Ser Pro
                85                  90                  95

Ala Val Arg Thr Asp Tyr Thr Tyr Pro Arg Tyr Glu Thr Thr Ala Glu
            100                 105                 110

Thr Thr Ser Gly Gly Leu Thr Gly Leu Thr Thr Ser Leu Ser Thr Leu
        115                 120                 125

Asn Ala Pro Ala Leu Ser Arg Thr Gln Ser Asp Gly Ser Gly Ser Lys
    130                 135                 140

Ser Ser Leu Gly Leu Asn Ile Gly Gly Met Gly Asp Tyr Arg Asn Glu
145                 150                 155                 160

Thr Leu Thr Thr Asn Pro Arg Asp Thr Ala Phe Leu Ser His Leu Val
                165                 170                 175

Gln Thr Val Phe Phe Leu Arg Gly Ile Asp Val Val Ser Pro Ala Asn
            180                 185                 190

Ala Asp Thr Asp Val Phe Ile Asn Ile Asp Val Phe Gly Thr Ile Arg
        195                 200                 205

Asn Arg Thr Glu Met His Leu Tyr Asn Ala Glu Thr Leu Lys Ala Gln
    210                 215                 220

Thr Lys Leu Glu Tyr Phe Ala Val Asp Arg Thr Asn Lys Lys Leu Leu
225                 230                 235                 240

Ile Lys Pro Lys Thr Asn Ala Phe Glu Ala Ala Tyr Lys Glu Asn Tyr
                245                 250                 255

Ala Leu Trp Met Gly Pro Tyr Lys Val Ser Lys Gly Ile Lys Pro Thr
```

260 265 270

Glu Gly Leu Met Val Asp Phe Ser Asp Ile Arg Pro Tyr Gly Asn His
275 280 285

Thr Gly Asn Ser Ala Pro Ser Val Glu Ala Asp Asn Ser His Glu Gly
290 295 300

Tyr Gly Tyr Ser Asp Glu Val Val Arg Gln His Arg Gln Gly Gln Pro
305 310 315 320

<210> 76
<211> 963
<212> DNA
<213> Neisseria gonorrhoeae

<400> 76
```
atgcgggcac ggctgctgat acctattctt ttttcagttt ttattttatc cgcctgcggg    60
acactgacag gtattccatc gcatggcgga ggcaaacgct tcgcggtcga acaagaactt   120
gtggccgctt ctgccagagc tgccgttaaa gacatggatt tacaggcatt acacggacga   180
aaagttgcat tgtacattgc aactatgggc gaccaaggtt caggcagttt gacagggggt   240
cgctactcca ttgatgcact gattcgcggc gaatacataa acagccctgc cgtccgcacc   300
gattacacct atccgcgtta cgaaaccacc gctgaaacaa catcaggcgg tttgacgggt   360
ttaaccactt ctttatctac acttaatgcc cctgcactct cgcgcaccca atcagacggt   420
agcggaagta ggagcagtct gggcttaaat attggcggga tgggggatta tcgaaatgaa   480
accttgacga ccaacccgcg cgacactgcc tttctttccc acttggtgca gaccgtattt   540
ttcctgcgcg gcatagacgt tgtttctcct gccaatgccg atacagatgt gtttattaac   600
atcgacgtat tcggaacgat acgcaacaga accgaaatgc acctatacaa tgccgaaaca   660
ctgaaagccc aaacaaaact ggaatatttc gcagtagaca gaaccaataa aaaattgctc   720
atcaaaccca aaaccaatgc gtttgaagct gcctataaag aaaattacgc attgtggatg   780
gggccgtata aagtaagcaa aggaatcaaa ccgacggaag gattgatggt cgatttctcc   840
gatatccaac catacggcaa tcatacgggt aactccgccc catccgtaga ggctgataac   900
agtcatgagg ggtatggata cagcgatgaa gcagtgcgac aacatagaca agggcaacct   960
tga                                                                 963
```

<210> 77
<211> 320
<212> PRT
<213> Neisseria gonorrhoeae

<400> 77
Met Arg Ala Arg Leu Leu Ile Pro Ile Leu Phe Ser Val Phe Ile Leu
1 5 10 15

Ser Ala Cys Gly Thr Leu Thr Gly Ile Pro Ser His Gly Gly Gly Lys
20 25 30

Arg Phe Ala Val Glu Gln Glu Leu Val Ala Ala Ser Ala Arg Ala Ala
35 40 45

Val Lys Asp Met Asp Leu Gln Ala Leu His Gly Arg Lys Val Ala Leu
50 55 60

Tyr Ile Ala Thr Met Gly Asp Gln Gly Ser Gly Ser Leu Thr Gly Gly
65 70 75 80

Arg Tyr Ser Ile Asp Ala Leu Ile Arg Gly Glu Tyr Ile Asn Ser Pro
85 90 95

Ala Val Arg Thr Asp Tyr Thr Tyr Pro Arg Tyr Glu Thr Thr Ala Glu

```
            100                 105                 110

Thr Thr Ser Gly Gly Leu Thr Gly Leu Thr Thr Ser Leu Ser Thr Leu
        115                 120                 125

Asn Ala Pro Ala Leu Ser Arg Thr Gln Ser Asp Gly Ser Gly Ser Arg
    130                 135                 140

Ser Ser Leu Gly Leu Asn Ile Gly Gly Met Gly Asp Tyr Arg Asn Glu
145                 150                 155                 160

Thr Leu Thr Thr Asn Pro Arg Asp Thr Ala Phe Leu Ser His Leu Val
                165                 170                 175

Gln Thr Val Phe Phe Leu Arg Gly Ile Asp Val Val Ser Pro Ala Asn
            180                 185                 190

Ala Asp Thr Asp Val Phe Ile Asn Ile Asp Val Phe Gly Thr Ile Arg
        195                 200                 205

Asn Arg Thr Glu Met His Leu Tyr Asn Ala Glu Thr Leu Lys Ala Gln
    210                 215                 220

Thr Lys Leu Glu Tyr Phe Ala Val Asp Arg Thr Asn Lys Lys Leu Leu
225                 230                 235                 240

Ile Lys Pro Lys Thr Asn Ala Phe Glu Ala Ala Tyr Lys Glu Asn Tyr
                245                 250                 255

Ala Leu Trp Met Gly Pro Tyr Lys Val Ser Lys Gly Ile Lys Pro Thr
            260                 265                 270

Glu Gly Leu Met Val Asp Phe Ser Asp Ile Gln Pro Tyr Gly Asn His
        275                 280                 285

Thr Gly Asn Ser Ala Pro Ser Val Glu Ala Asp Asn Ser His Glu Gly
    290                 295                 300

Tyr Gly Tyr Ser Asp Glu Ala Val Arg Gln His Arg Gln Gly Gln Pro
305                 310                 315                 320


<210>   78
<211>   963
<212>   DNA
<213>   Neisseria meningitidis

<400>   78
atgcaagcac ggctgctgat acctattctt ttttcagttt ttattttatc cgcctgcggg   60
acactgacag gtattccatc gcatggcgga ggtaaacgct tcgcggtcga acaagaactt   120
gtggccgctt ctgccagagc tgccgttaaa gacatggatt tacaggcatt acacggacga   180
aaagttgcat tgtacattgc aactatgggc gaccaaggtt caggcagttt gacagggggt   240
cgctactcca ttgatgcact gattcgtggc gaatacataa acagccctgc cgtccgtacc   300
gattacacct atccacgtta cgaaaccacc gctgaaacaa catcaggcgg tttgacaggt   360
ttaaccactt ctttatctac acttaatgcc cctgcactct cgcgcaccca atcagacggt   420
agcggaagta aaagcagtct gggcttaaat attggcggga tgggggatta tcgaaatgaa   480
accttgacga ctaacccgcg cgacactgcc tttctttccc acttggtaca gaccgtattt   540
ttcctgcgcg gcatagacgt tgtttctcct gccaatgccg atacggatgt gtttattaac   600
atcgacgtat tcggaacgat acgcaacaga accgaaatgc acctatacaa tgccgaaaca   660
ctgaaagccc aaacaaaact ggaatatttc gcagtagaca gaaccaataa aaaattgctc   720
atcaaaccaa aaaccaatgc gtttgaagct gcctataaag aaaattacgc attgtggatg   780
```

```
ggaccgtata aagtaagcaa aggaattaaa ccgacagaag gattaatggt cgatttctcc   840
gatatccaac catacggcaa tcatatgggt aactctgccc catccgtaga ggctgataac   900
agtcatgagg ggtatggata cagcgatgaa gcagtgcgac gacatagaca agggcaacct   960
tga                                                                  963

<210>   79
<211>   320
<212>   PRT
<213>   Neisseria meningitidis

<400>   79
Met Gln Ala Arg Leu Leu Ile Pro Ile Leu Phe Ser Val Phe Ile Leu
1               5                   10                  15

Ser Ala Cys Gly Thr Leu Thr Gly Ile Pro Ser His Gly Gly Gly Lys
            20                  25                  30

Arg Phe Ala Val Glu Gln Glu Leu Val Ala Ala Ser Ala Arg Ala Ala
        35                  40                  45

Val Lys Asp Met Asp Leu Gln Ala Leu His Gly Arg Lys Val Ala Leu
    50                  55                  60

Tyr Ile Ala Thr Met Gly Asp Gln Gly Ser Gly Ser Leu Thr Gly Gly
65                  70                  75                  80

Arg Tyr Ser Ile Asp Ala Leu Ile Arg Gly Glu Tyr Ile Asn Ser Pro
                85                  90                  95

Ala Val Arg Thr Asp Tyr Thr Tyr Pro Arg Tyr Glu Thr Thr Ala Glu
            100                 105                 110

Thr Thr Ser Gly Gly Leu Thr Gly Leu Thr Thr Ser Leu Ser Thr Leu
        115                 120                 125

Asn Ala Pro Ala Leu Ser Arg Thr Gln Ser Asp Gly Ser Gly Ser Lys
    130                 135                 140

Ser Ser Leu Gly Leu Asn Ile Gly Gly Met Gly Asp Tyr Arg Asn Glu
145                 150                 155                 160

Thr Leu Thr Thr Asn Pro Arg Asp Thr Ala Phe Leu Ser His Leu Val
                165                 170                 175

Gln Thr Val Phe Phe Leu Arg Gly Ile Asp Val Val Ser Pro Ala Asn
            180                 185                 190

Ala Asp Thr Asp Val Phe Ile Asn Ile Asp Val Phe Gly Thr Ile Arg
        195                 200                 205

Asn Arg Thr Glu Met His Leu Tyr Asn Ala Glu Thr Leu Lys Ala Gln
    210                 215                 220

Thr Lys Leu Glu Tyr Phe Ala Val Asp Arg Thr Asn Lys Lys Leu Leu
225                 230                 235                 240

Ile Lys Pro Lys Thr Asn Ala Phe Glu Ala Ala Tyr Lys Glu Asn Tyr
                245                 250                 255

Ala Leu Trp Met Gly Pro Tyr Lys Val Ser Lys Gly Ile Lys Pro Thr
            260                 265                 270
```

Glu Gly Leu Met Val Asp Phe Ser Asp Ile Gln Pro Tyr Gly Asn His
275 280 285

Met Gly Asn Ser Ala Pro Ser Val Glu Ala Asp Asn Ser His Glu Gly
290 295 300

Tyr Gly Tyr Ser Asp Glu Ala Val Arg Arg His Arg Gln Gly Gln Pro
305 310 315 320

<210> 80
<211> 606
<212> DNA
<213> Neisseria meningitidis

<400> 80
atgaccatct atttcaaaaa cggcttttac gacgacacat tgggcggcat ccccgaaggc 60
gcggttgccg tccgcgccga agaatacgcc gcccttttgg caggacaggc gcagggcggg 120
cagattgccg cagattccga cggccgcccc gttttaaccc cgccgcgccc gtccgattac 180
cacgaatggg acggcaaaaa atggaaaatc agcaaagccg ccgccgccgc ccgtttcgcc 240
aaacaaaaaa ccgccttggc attccgcctc gcggaaaagg cggacgaact caaaaacagc 300
ctcttggcgg gctatcccca agtggaaatc gacagctttt acaggcagga aaaagaagcc 360
ctcgcgcggc aggcggacaa caacgccccg accccgatgc tggcgcaaat cgccgccgca 420
aggggcgtgg aattggacgt tttgattgaa aaagttatcg aaaaatccgc ccgcctggct 480
gttgccgccg gcgcgattat cggaaagcgt cagcagctcg aagacaaatt gaacaccatc 540
gaaaccgcgc ccggattgga cgcgctggaa aaggaaatcg aagaatggac gctaaacatc 600
ggctga 606

<210> 81
<211> 201
<212> PRT
<213> Neisseria meningitidis

<400> 81
Met Thr Ile Tyr Phe Lys Asn Gly Phe Tyr Asp Asp Thr Leu Gly Gly
1 5 10 15

Ile Pro Glu Gly Ala Val Ala Val Arg Ala Glu Glu Tyr Ala Ala Leu
20 25 30

Leu Ala Gly Gln Ala Gln Gly Gly Gln Ile Ala Ala Asp Ser Asp Gly
35 40 45

Arg Pro Val Leu Thr Pro Pro Arg Pro Ser Asp Tyr His Glu Trp Asp
50 55 60

Gly Lys Lys Trp Lys Ile Ser Lys Ala Ala Ala Ala Ala Arg Phe Ala
65 70 75 80

Lys Gln Lys Thr Ala Leu Ala Phe Arg Leu Ala Glu Lys Ala Asp Glu
85 90 95

Leu Lys Asn Ser Leu Leu Ala Gly Tyr Pro Gln Val Glu Ile Asp Ser
100 105 110

Phe Tyr Arg Gln Glu Lys Glu Ala Leu Ala Arg Gln Ala Asp Asn Asn
115 120 125

Ala Pro Thr Pro Met Leu Ala Gln Ile Ala Ala Ala Arg Gly Val Glu
130 135 140

```
Leu Asp Val Leu Ile Glu Lys Val Ile Glu Lys Ser Ala Arg Leu Ala
145                 150                 155                 160

Val Ala Ala Gly Ala Ile Ile Gly Lys Arg Gln Gln Leu Glu Asp Lys
                165                 170                 175

Leu Asn Thr Ile Glu Thr Ala Pro Gly Leu Asp Ala Leu Glu Lys Glu
            180                 185                 190

Ile Glu Glu Trp Thr Leu Asn Ile Gly
        195                 200

<210>   82
<211>   624
<212>   DNA
<213>   Neisseria meningitidis

<400>   82
atgagaaaac cgaccgatac cctacccgtt aatctgcaac gccgccgcct gttgtgtgcc    60
gccggtgcgt tgttgctcag tcctctggcg cacgccggcg cgcaacgtga ggaaacgctt   120
gccgacgatg tggcttccgt gatgaggagt tctgtcggca gcgtcaatcc gccgaggctg   180
gtgtttgaca atccgaaaga gggcgagcgt tggttgtctg ccatgtcggc acgtttggca   240
aggttcgtcc ccgaggagga ggagcggcgc aggctgctgg tcaatatcca gtacgaaagc   300
agccgggccg gtttggatac gcagattgtg ttggggctga ttgaggtgga aagcgcgttc   360
cgccagtatg caatcagcgg tgtcggcgcg cgcggcctga tgcaggttat gccgttttgg   420
aaaaactaca tcggcaaacc ggcgcacaac ctgttcgaca tccgcaccaa cctgcgttac   480
ggctgtacca tcctgcgcca ttaccggaat cttgaaaaag gcaacatcgt ccgcgcgctt   540
gcccgcttta acggcagctt gggcagcaat aaatatccga acgccgtttt gggcgcgtgg   600
cgcaaccgct ggcagtggcg ttga                                          624

<210>   83
<211>   207
<212>   PRT
<213>   Neisseria meningitidis

<400>   83
Met Arg Lys Pro Thr Asp Thr Leu Pro Val Asn Leu Gln Arg Arg Arg
1               5                   10                  15

Leu Leu Cys Ala Ala Gly Ala Leu Leu Leu Ser Pro Leu Ala His Ala
            20                  25                  30

Gly Ala Gln Arg Glu Glu Thr Leu Ala Asp Asp Val Ala Ser Val Met
        35                  40                  45

Arg Ser Ser Val Gly Ser Val Asn Pro Pro Arg Leu Val Phe Asp Asn
    50                  55                  60

Pro Lys Glu Gly Glu Arg Trp Leu Ser Ala Met Ser Ala Arg Leu Ala
65                  70                  75                  80

Arg Phe Val Pro Glu Glu Glu Glu Arg Arg Arg Leu Leu Val Asn Ile
                85                  90                  95

Gln Tyr Glu Ser Ser Arg Ala Gly Leu Asp Thr Gln Ile Val Leu Gly
            100                 105                 110

Leu Ile Glu Val Glu Ser Ala Phe Arg Gln Tyr Ala Ile Ser Gly Val
        115                 120                 125
```

Gly Ala Arg Gly Leu Met Gln Val Met Pro Phe Trp Lys Asn Tyr Ile
    130                 135                 140

Gly Lys Pro Ala His Asn Leu Phe Asp Ile Arg Thr Asn Leu Arg Tyr
145                 150                 155                 160

Gly Cys Thr Ile Leu Arg His Tyr Arg Asn Leu Glu Lys Gly Asn Ile
                165                 170                 175

Val Arg Ala Leu Ala Arg Phe Asn Gly Ser Leu Gly Ser Asn Lys Tyr
            180                 185                 190

Pro Asn Ala Val Leu Gly Ala Trp Arg Asn Arg Trp Gln Trp Arg
        195                 200                 205

<210> 84
<211> 564
<212> DNA
<213> Neisseria meningitidis

<400> 84
atgaaaaaaa tcatcttcgc cgcactcgca gccgccgcca tcagtactgc ctccgccgcc 60
acctacaaag tggacgaata tcacgccaac gcccgtttcg ccatcgacca tttcaacacc 120
agcaccaacg tcggcggttt ttacggtctg accggttccg tcgagttcga ccaagcaaaa 180
cgcgacggta aaatcgacat caccatcccc attgccaacc tgcaaagcgg ttcgcaacac 240
tttaccgacc acctgaaatc agccgacatc ttcgatgccg cccaatatcc ggacatccgc 300
tttgtttcca ccaaattcaa cttcaacggc aaaaaactgg tttccgttga cggcaacctg 360
accatgcacg gcaaaaccgc ccccgtcaaa ctcaaagccg aaaaattcaa ctgctaccaa 420
agcccgatgg agaaaaccga agtttgtggc ggcgacttca gcaccaccat cgaccgcacc 480
aaatggggca tggactacct cgttaacgtt ggtatgacca aaagcgtccg catcgacatc 540
caaatcgagg cagccaaaca ataa 564

<210> 85
<211> 187
<212> PRT
<213> Neisseria meningitidis

<400> 85
Met Lys Lys Ile Ile Phe Ala Ala Leu Ala Ala Ala Ala Ile Ser Thr
1               5                   10                  15

Ala Ser Ala Ala Thr Tyr Lys Val Asp Glu Tyr His Ala Asn Ala Arg
            20                  25                  30

Phe Ala Ile Asp His Phe Asn Thr Ser Thr Asn Val Gly Gly Phe Tyr
        35                  40                  45

Gly Leu Thr Gly Ser Val Glu Phe Asp Gln Ala Lys Arg Asp Gly Lys
    50                  55                  60

Ile Asp Ile Thr Ile Pro Ile Ala Asn Leu Gln Ser Gly Ser Gln His
65                  70                  75                  80

Phe Thr Asp His Leu Lys Ser Ala Asp Ile Phe Asp Ala Ala Gln Tyr
                85                  90                  95

Pro Asp Ile Arg Phe Val Ser Thr Lys Phe Asn Phe Asn Gly Lys Lys
            100                 105                 110

Leu Val Ser Val Asp Gly Asn Leu Thr Met His Gly Lys Thr Ala Pro
        115                 120                 125

```
Val Lys Leu Lys Ala Glu Lys Phe Asn Cys Tyr Gln Ser Pro Met Glu
    130                 135                 140

Lys Thr Glu Val Cys Gly Gly Asp Phe Ser Thr Thr Ile Asp Arg Thr
145                 150                 155                 160

Lys Trp Gly Met Asp Tyr Leu Val Asn Val Gly Met Thr Lys Ser Val
                165                 170                 175

Arg Ile Asp Ile Gln Ile Glu Ala Ala Lys Gln
            180                 185
```

<210> 86
<211> 4374
<212> DNA
<213> Neisseria meningitidis

<400> 86

```
atgaaaacaa ccgacaaacg gacaaccgaa acacaccgca aagccccgaa aaccggccgc   60
atccgcttct cgcctgctta cttagccata tgcctgtcgt tcggcattct tccccaagcc  120
tgggcgggac acacttattt cggcatcaac taccaatact atcgcgactt tgccgaaaat  180
aaaggcaagt ttgcagtcgg ggcgaaagat attgaggttt acaacaaaaa aggggagttg  240
gtcggcaaat caatgacaaa agccccgatg attgattttt ctgtggtgtc gcgtaacggc  300
gtggcggcat tggtgggcga tcaatatatt gtgagcgtgg cacataacgg cggctataac  360
aacgttgatt ttggtgcgga aggaagaaat cccgatcaac atcgttttac ttataaaatt  420
gtgaaacgga ataattataa agcagggact aaaggccatc cttatggcgg cgattatcat  480
atgccgcgtt tgcataaatt tgtcacagat gcagaacctg ttgaaatgac cagttatatg  540
gatgggcgga aatatatcga tcaaaataat taccctgacc gtgttcgtat tggggcaggc  600
aggcaatatt ggcgatctga tgaagatgag cccaataacc gcgaaagttc atatcatatt  660
gcaagtgcgt attcttggct cgttggtggc aatacctttg cacaaaatgg atcaggtggt  720
ggcacagtca acttaggtag tgaaaaaatt aaacatagcc catatggttt tttaccaaca  780
ggaggctcat ttggcgacag tggctcacca atgtttatct atgatgccca aaagcaaaag  840
tggttaatta atggggtatt gcaaacgggc aacccctata taggaaaaag caatggcttc  900
cagctggttc gtaaagattg gttctatgat gaaatctttg ctggagatac ccattcagta  960
ttctacgaac cacgtcaaaa tgggaaatac tcttttaacg acgataataa tggcacagga 1020
aaaatcaatg ccaaacatga acacaattct ctgcctaata gattaaaaac acgaaccgtt 1080
caattgttta atgtttcttt atccgagaca gcaagagaac ctgtttatca tgctgcaggt 1140
ggtgtcaaca gttatcgacc cagactgaat aatggagaaa atatttcctt tattgacgaa 1200
ggaaaaggcg aattgatact taccagcaac atcaatcaag gtgctggagg attatatttc 1260
caaggagatt ttacggtctc gcctgaaaat aacgaaactt ggcaaggcgc gggcgttcat 1320
atcagtgaag acagtaccgt tacttggaaa gtaaacggcg tggcaaacga ccgcctgtcc 1380
aaaatcggca aaggcacgct gcacgttcaa gccaaagggg aaaaccaagg ctcgatcagc 1440
gtgggcgacg gtacagtcat tttggatcag caggcagacg ataaaggcaa aaaacaagcc 1500
tttagtgaaa tcggcttggt cagcggcagg ggtacggtgc aactgaatgc cgataatcag 1560
ttcaaccccg acaaactcta tttcggcttt cgcggcggac gtttggattt aaacgggcat 1620
tcgctttcgt tccaccgtat tcaaaatacc gatgaagggg cgatgattgt caaccacaat 1680
caagacaaag aatccaccgt taccattaca ggcaataaag atattgctac aaccggcaat 1740
aacaacagct tggatagcaa aaaagaaatt gcctacaacg gttggtttgg cgagaaagat 1800
acgaccaaaa cgaacgggcg gctcaacctt gtttaccagc ccgccgcaga agaccgcacc 1860
ctgctgcttt ccggcggaac aaatttaaac ggcaacatca cgcaaacaaa cggcaaactg 1920
tttttcagcg gcagaccaac accgcacgcc tacaatcatt taaacgacca ttggtcgcaa 1980
aaagagggca ttcctcgcgg ggaaatcgtg tgggacaacg actggatcaa ccgcacattt 2040
aaagcggaaa acttccaaat taaaggcgga caggcggtgg tttcccgcaa tgttgccaaa 2100
gtgaaaggcg attggcattt gagcaatcac gcccaagcag tttttggtgt cgcaccgcat 2160
caaagccaca caatctgtac acgttcggac tggacgggtc tgacaaattg tgtcgaaaaa 2220
accattaccg acgataaagt gattgcttca ttgactaaga ccgacatcag cggcaatgtc 2280
gatcttgccg atcacgctca tttaaatctc acagggcttg ccacactcaa cggcaatctt 2340
agtgcaaatg gcgatacacg ttatacagtc agccacaacg ccacccaaaa cggcaacctt 2400
agcctcgtgg gcaatgccca agcaacattt aatcaagcca cattaaacgg caacacatcg 2460
gcttcgggca atgcttcatt taatctaagc gaccacgccg tacaaaacgg cagtctgacg 2520
```

```
ctttccggca acgctaaggc aaacgtaagc cattccgcac tcaacggtaa tgtctcccta   2580
gccgataagg cagtattcca ttttgaaagc agccgcttta ccggacaaat cagcggcggc   2640
aaggatacgg cattacactt aaaagacagc gaatggacgc tgccgtcagg cacggaatta   2700
ggcaatttaa accttgacaa cgccaccatt acactcaatt ccgcctatcg ccacgatgcg   2760
gcaggggcgc aaaccggcag tgcgacagat gcgccgcgcc gccgttcgcg ccgttcgcgc   2820
cgttccctat tatccgttac accgccaact tcggtagaat cccgtttcaa cacgctgacg   2880
gtaaacggca aattgaacgg tcagggaaca ttccgcttta tgtcggaact cttcggctac   2940
cgcagcgaca aattgaagct ggcggaaagt tccgaaggca cttacacctt ggcggtcaac   3000
aataccggca acgaacctgc aagcctcgaa caattgacgg tagtggaagg aaaagacaac   3060
aaaccgctgt ccgaaaacct taatttcacc ctgcaaaacg aacacgtcga tgccggcgcg   3120
tggcgttacc aactcatccg caaagacggc gagttccgcc tgcataatcc ggtcaaagaa   3180
caagagcttt ccgacaaact cggcaaggca gaagccaaaa aacaggcgga aaaagacaac   3240
gcgcaaagcc ttgacgcgct gattgcggcc gggcgcgatg ccgtcgaaaa gacagaaagc   3300
gttgccgaac cggcccggca ggcaggcggg gaaaatgtcg gcattatgca ggcggaggaa   3360
gagaaaaaac gggtgcaggc ggataaagac accgccttgg cgaaacagcg cgaagcggaa   3420
acccggccgg ctaccaccgc cttcccccgc gcccgccgcg cccgccggga tttgccgcaa   3480
ctgcaacccc aaccgcagcc ccaaccgcag cgcgacctga tcagccgtta tgccaatagc   3540
ggtttgagtg aattttccgc cacgctcaac agcgttttcg ccgtacagga cgaattagac   3600
cgcgtatttg ccgaagaccg ccgcaacgcc gtttggacaa gcggcatccg ggacaccaaa   3660
cactaccgtt cgcaagattt ccgcgcctac cgccaacaaa ccgacctgcg ccaaatcggt   3720
atgcagaaaa acctcggcag cgggcgcgtc ggcatcctgt tttcgcacaa ccggaccgaa   3780
aacaccttcg acgacggcat cggcaactcg gcacggcttg cccacggcgc cgttttcggg   3840
caatacggca tcgacaggtt ctacatcggc atcagcgcgg gcgcgggttt tagcagcggc   3900
agcctttcag acggcatcgg aggcaaaatc cgccgccgcg tgctgcatta cggcattcag   3960
gcacgatacc gcgccggttt cggcggattc ggcatcgaac cgcacatcgg cgcaacgcgc   4020
tatttcgtcc aaaaagcgga ttaccgctac gaaaacgtca atatcgccac cccggccttt   4080
gcattcaacc gctaccgcgc gggcattaag gcagattatt cattcaaacc ggcgcaacac   4140
atttccatca cgccttattt gagcctgtcc tataccgatg ccgcttcggg caaagtccga   4200
acacgcgtca ataccgccgt attggctcag gatttcggca aaacccgcag tgcggaatgg   4260
ggcgtaaacg ccgaaatcaa aggtttcacg ctgtccctcc acgctgccgc cgccaaaggc   4320
ccgcaactgg aagcgcaaca cagcgcgggc atcaaattag gctaccgctg gtaa         4374
```

<210> 87
<211> 1457
<212> PRT
<213> Neisseria meningitidis

<400> 87

```
Met Lys Thr Thr Asp Lys Arg Thr Thr Glu Thr His Arg Lys Ala Pro
1               5                   10                  15

Lys Thr Gly Arg Ile Arg Phe Ser Pro Ala Tyr Leu Ala Ile Cys Leu
            20                  25                  30

Ser Phe Gly Ile Leu Pro Gln Ala Trp Ala Gly His Thr Tyr Phe Gly
        35                  40                  45

Ile Asn Tyr Gln Tyr Tyr Arg Asp Phe Ala Glu Asn Lys Gly Lys Phe
    50                  55                  60

Ala Val Gly Ala Lys Asp Ile Glu Val Tyr Asn Lys Lys Gly Glu Leu
65                  70                  75                  80

Val Gly Lys Ser Met Thr Lys Ala Pro Met Ile Asp Phe Ser Val Val
                85                  90                  95

Ser Arg Asn Gly Val Ala Ala Leu Val Gly Asp Gln Tyr Ile Val Ser
            100                 105                 110

Val Ala His Asn Gly Gly Tyr Asn Asn Val Asp Phe Gly Ala Glu Gly
        115                 120                 125
```

```
Arg Asn Pro Asp Gln His Arg Phe Thr Tyr Lys Ile Val Lys Arg Asn
    130                 135                 140

Asn Tyr Lys Ala Gly Thr Lys Gly His Pro Tyr Gly Gly Asp Tyr His
145                 150                 155                 160

Met Pro Arg Leu His Lys Phe Val Thr Asp Ala Glu Pro Val Glu Met
                165                 170                 175

Thr Ser Tyr Met Asp Gly Arg Lys Tyr Ile Asp Gln Asn Asn Tyr Pro
            180                 185                 190

Asp Arg Val Arg Ile Gly Ala Gly Arg Gln Tyr Trp Arg Ser Asp Glu
        195                 200                 205

Asp Glu Pro Asn Asn Arg Glu Ser Ser Tyr His Ile Ala Ser Ala Tyr
    210                 215                 220

Ser Trp Leu Val Gly Gly Asn Thr Phe Ala Gln Asn Gly Ser Gly Gly
225                 230                 235                 240

Gly Thr Val Asn Leu Gly Ser Glu Lys Ile Lys His Ser Pro Tyr Gly
                245                 250                 255

Phe Leu Pro Thr Gly Gly Ser Phe Gly Asp Ser Gly Ser Pro Met Phe
            260                 265                 270

Ile Tyr Asp Ala Gln Lys Gln Lys Trp Leu Ile Asn Gly Val Leu Gln
        275                 280                 285

Thr Gly Asn Pro Tyr Ile Gly Lys Ser Asn Gly Phe Gln Leu Val Arg
    290                 295                 300

Lys Asp Trp Phe Tyr Asp Glu Ile Phe Ala Gly Asp Thr His Ser Val
305                 310                 315                 320

Phe Tyr Glu Pro Arg Gln Asn Gly Lys Tyr Ser Phe Asn Asp Asp Asn
                325                 330                 335

Asn Gly Thr Gly Lys Ile Asn Ala Lys His Glu His Asn Ser Leu Pro
            340                 345                 350

Asn Arg Leu Lys Thr Arg Thr Val Gln Leu Phe Asn Val Ser Leu Ser
        355                 360                 365

Glu Thr Ala Arg Glu Pro Val Tyr His Ala Ala Gly Gly Val Asn Ser
    370                 375                 380

Tyr Arg Pro Arg Leu Asn Asn Gly Glu Asn Ile Ser Phe Ile Asp Glu
385                 390                 395                 400

Gly Lys Gly Glu Leu Ile Leu Thr Ser Asn Ile Asn Gln Gly Ala Gly
                405                 410                 415

Gly Leu Tyr Phe Gln Gly Asp Phe Thr Val Ser Pro Glu Asn Asn Glu
            420                 425                 430

Thr Trp Gln Gly Ala Gly Val His Ile Ser Glu Asp Ser Thr Val Thr
        435                 440                 445
```

```
Trp Lys Val Asn Gly Val Ala Asn Asp Arg Leu Ser Lys Ile Gly Lys
    450                 455                 460

Gly Thr Leu His Val Gln Ala Lys Gly Glu Asn Gln Gly Ser Ile Ser
465                 470                 475                 480

Val Gly Asp Gly Thr Val Ile Leu Asp Gln Gln Ala Asp Asp Lys Gly
                485                 490                 495

Lys Lys Gln Ala Phe Ser Glu Ile Gly Leu Val Ser Gly Arg Gly Thr
            500                 505                 510

Val Gln Leu Asn Ala Asp Asn Gln Phe Asn Pro Asp Lys Leu Tyr Phe
        515                 520                 525

Gly Phe Arg Gly Gly Arg Leu Asp Leu Asn Gly His Ser Leu Ser Phe
    530                 535                 540

His Arg Ile Gln Asn Thr Asp Glu Gly Ala Met Ile Val Asn His Asn
545                 550                 555                 560

Gln Asp Lys Glu Ser Thr Val Thr Ile Thr Gly Asn Lys Asp Ile Ala
                565                 570                 575

Thr Thr Gly Asn Asn Asn Ser Leu Asp Ser Lys Lys Glu Ile Ala Tyr
            580                 585                 590

Asn Gly Trp Phe Gly Glu Lys Asp Thr Thr Lys Thr Asn Gly Arg Leu
        595                 600                 605

Asn Leu Val Tyr Gln Pro Ala Ala Glu Asp Arg Thr Leu Leu Leu Ser
    610                 615                 620

Gly Gly Thr Asn Leu Asn Gly Asn Ile Thr Gln Thr Asn Gly Lys Leu
625                 630                 635                 640

Phe Phe Ser Gly Arg Pro Thr Pro His Ala Tyr Asn His Leu Asn Asp
                645                 650                 655

His Trp Ser Gln Lys Glu Gly Ile Pro Arg Gly Glu Ile Val Trp Asp
            660                 665                 670

Asn Asp Trp Ile Asn Arg Thr Phe Lys Ala Glu Asn Phe Gln Ile Lys
        675                 680                 685

Gly Gly Gln Ala Val Val Ser Arg Asn Val Ala Lys Val Lys Gly Asp
    690                 695                 700

Trp His Leu Ser Asn His Ala Gln Ala Val Phe Gly Val Ala Pro His
705                 710                 715                 720

Gln Ser His Thr Ile Cys Thr Arg Ser Asp Trp Thr Gly Leu Thr Asn
                725                 730                 735

Cys Val Glu Lys Thr Ile Thr Asp Asp Lys Val Ile Ala Ser Leu Thr
            740                 745                 750

Lys Thr Asp Ile Ser Gly Asn Val Asp Leu Ala Asp His Ala His Leu
        755                 760                 765

Asn Leu Thr Gly Leu Ala Thr Leu Asn Gly Asn Leu Ser Ala Asn Gly
```

770 775 780

Asp Thr Arg Tyr Thr Val Ser His Asn Ala Thr Gln Asn Gly Asn Leu
785 790 795 800

Ser Leu Val Gly Asn Ala Gln Ala Thr Phe Asn Gln Ala Thr Leu Asn
805 810 815

Gly Asn Thr Ser Ala Ser Gly Asn Ala Ser Phe Asn Leu Ser Asp His
820 825 830

Ala Val Gln Asn Gly Ser Leu Thr Leu Ser Gly Asn Ala Lys Ala Asn
835 840 845

Val Ser His Ser Ala Leu Asn Gly Asn Val Ser Leu Ala Asp Lys Ala
850 855 860

Val Phe His Phe Glu Ser Ser Arg Phe Thr Gly Gln Ile Ser Gly Gly
865 870 875 880

Lys Asp Thr Ala Leu His Leu Lys Asp Ser Glu Trp Thr Leu Pro Ser
885 890 895

Gly Thr Glu Leu Gly Asn Leu Asn Leu Asp Asn Ala Thr Ile Thr Leu
900 905 910

Asn Ser Ala Tyr Arg His Asp Ala Ala Gly Ala Gln Thr Gly Ser Ala
915 920 925

Thr Asp Ala Pro Arg Arg Arg Ser Arg Arg Ser Arg Arg Ser Leu Leu
930 935 940

Ser Val Thr Pro Pro Thr Ser Val Glu Ser Arg Phe Asn Thr Leu Thr
945 950 955 960

Val Asn Gly Lys Leu Asn Gly Gln Gly Thr Phe Arg Phe Met Ser Glu
965 970 975

Leu Phe Gly Tyr Arg Ser Asp Lys Leu Lys Leu Ala Glu Ser Ser Glu
980 985 990

Gly Thr Tyr Thr Leu Ala Val Asn Asn Thr Gly Asn Glu Pro Ala Ser
995 1000 1005

Leu Glu Gln Leu Thr Val Val Glu Gly Lys Asp Asn Lys Pro Leu Ser
1010 1015 1020

Glu Asn Leu Asn Phe Thr Leu Gln Asn Glu His Val Asp Ala Gly Ala
1025 1030 1035 1040

Trp Arg Tyr Gln Leu Ile Arg Lys Asp Gly Glu Phe Arg Leu His Asn
1045 1050 1055

Pro Val Lys Glu Gln Glu Leu Ser Asp Lys Leu Gly Lys Ala Glu Ala
1060 1065 1070

Lys Lys Gln Ala Glu Lys Asp Asn Ala Gln Ser Leu Asp Ala Leu Ile
1075 1080 1085

Ala Ala Gly Arg Asp Ala Val Glu Lys Thr Glu Ser Val Ala Glu Pro
1090 1095 1100

```
Ala Arg Gln Ala Gly Gly Glu Asn Val Gly Ile Met Gln Ala Glu Glu
1105                1110                1115                1120

Glu Lys Lys Arg Val Gln Ala Asp Lys Asp Thr Ala Leu Ala Lys Gln
                1125                1130                1135

Arg Glu Ala Glu Thr Arg Pro Ala Thr Thr Ala Phe Pro Arg Ala Arg
            1140                1145                1150

Arg Ala Arg Arg Asp Leu Pro Gln Leu Gln Pro Gln Pro Gln Pro Gln
        1155                1160                1165

Pro Gln Arg Asp Leu Ile Ser Arg Tyr Ala Asn Ser Gly Leu Ser Glu
    1170                1175                1180

Phe Ser Ala Thr Leu Asn Ser Val Phe Ala Val Gln Asp Glu Leu Asp
1185                1190                1195                1200

Arg Val Phe Ala Glu Asp Arg Arg Asn Ala Val Trp Thr Ser Gly Ile
                1205                1210                1215

Arg Asp Thr Lys His Tyr Arg Ser Gln Asp Phe Arg Ala Tyr Arg Gln
            1220                1225                1230

Gln Thr Asp Leu Arg Gln Ile Gly Met Gln Lys Asn Leu Gly Ser Gly
        1235                1240                1245

Arg Val Gly Ile Leu Phe Ser His Asn Arg Thr Glu Asn Thr Phe Asp
    1250                1255                1260

Asp Gly Ile Gly Asn Ser Ala Arg Leu Ala His Gly Ala Val Phe Gly
1265                1270                1275                1280

Gln Tyr Gly Ile Asp Arg Phe Tyr Ile Gly Ile Ser Ala Gly Ala Gly
                1285                1290                1295

Phe Ser Ser Gly Ser Leu Ser Asp Gly Ile Gly Gly Lys Ile Arg Arg
            1300                1305                1310

Arg Val Leu His Tyr Gly Ile Gln Ala Arg Tyr Arg Ala Gly Phe Gly
        1315                1320                1325

Gly Phe Gly Ile Glu Pro His Ile Gly Ala Thr Arg Tyr Phe Val Gln
    1330                1335                1340

Lys Ala Asp Tyr Arg Tyr Glu Asn Val Asn Ile Ala Thr Pro Gly Leu
1345                1350                1355                1360

Ala Phe Asn Arg Tyr Arg Ala Gly Ile Lys Ala Asp Tyr Ser Phe Lys
                1365                1370                1375

Pro Ala Gln His Ile Ser Ile Thr Pro Tyr Leu Ser Leu Ser Tyr Thr
            1380                1385                1390

Asp Ala Ala Ser Gly Lys Val Arg Thr Arg Val Asn Thr Ala Val Leu
        1395                1400                1405

Ala Gln Asp Phe Gly Lys Thr Arg Ser Ala Glu Trp Gly Val Asn Ala
    1410                1415                1420
```

Glu Ile Lys Gly Phe Thr Leu Ser Leu His Ala Ala Ala Ala Lys Gly
1425 1430 1435 1440

Pro Gln Leu Glu Ala Gln His Ser Ala Gly Ile Lys Leu Gly Tyr Arg
1445 1450 1455

Trp

<210> 88
<211> 1827
<212> DNA
<213> Neisseria meningitidis

<400> 88
ttgggcattt cccgcaaaat atcccttatt ctgtccatac tggcagtgtg cctgccgatg 60
catgcacacg cctcagattt ggcaaacgat tcttttatcc ggcaggttct cgaccgtcag 120
catttcgaac ccgacgggaa ataccaccta ttcggcagca ggggggaact tgccgagcgc 180
agcggccata tcggattggg aaaaatacaa agccatcagt tgggcaacct gatgattcaa 240
caggcggcca ttaaaggaaa tatcggctac attgtccgct tttccgatca cgggcacgaa 300
gtccattccc ccttcgacaa ccatgcctca cattccgatt ctgatgaagc cggtagtccc 360
gttgacggat ttagccttta ccgcatccat tgggacggat acgaacacca tcccgccgac 420
ggctatgacg ggccacaggg cggcggctat cccgctccca aaggcgcgag ggatatatac 480
agctacgaca taaaaggcgt tgcccaaaat atccgcctca acctgaccga caaccgcagc 540
accggacaac ggcttgccga ccgtttccac aatgccggta gtatgctgac gcaaggagta 600
ggcgacggat tcaaacgcgc cacccgatac agccccgagc tggacagatc gggcaatgcc 660
gccgaagcct tcaacggcac tgcagatatc gttaaaaaca tcatcggcgc ggcaggagaa 720
attgtcggcg caggcgatgc cgtgcagggc ataagcgaag gctcaaacat tgctgtcatg 780
cacggcttgg gtctgctttc caccgaaaac aagatggcgc gcatcaacga tttggcagat 840
atggcgcaac tcaaagacta tgccgcagca gccatccgcg attgggcagt ccaaaacccc 900
aatgccgcac aaggcataga agccgtcagc aatatcttta tggcagccat ccccatcaaa 960
gggattggag ctgttcgggg aaaatacggc ttgggcggca tcacggcaca tcctatcaag 1020
cggtcgcaga tgggcgcgat cgcattgccg aaagggaaat ccgccgtcag cgacaatttt 1080
gccgatgcgg catacgccaa atacccgtcc ccttaccatt cccgaaatat ccgttcaaac 1140
ttggagcagc gttacggcaa agaaaacatc acctcctcaa ccgtgccgcc gtcaaacggc 1200
aaaaatgtca aactggcaga ccaacgccac ccgaagacag gcgtaccgtt tgacggtaaa 1260
gggtttccga attttgagaa gcacgtgaaa tatgatacga agctcgatat tcaagaatta 1320
tcgggggggcg gtatacctaa ggctaagcct gtgtttgatg cgaaaccgag atgggaggtt 1380
gataggaagc ttaataaatt gacaactcgt gagcaggtgg agaaaaatgt tcaggaaata 1440
aggaacggta atataaacag taactttagc caacatgctc aactagagag ggaaattaat 1500
aaactaaaat ctgccgatga aattaatttt gcagatggaa tgggaaaatt taccgatagc 1560
atgaatgaca aggcttttag taggcttgtg aaatcagtta aagagaatgg cttcacaaat 1620
ccagttgtgg agtacgttga aataaatgga aaagcatata tcgtaagagg aaataatrgg 1680
gtttttgctg cagaatacct tggcaggata catgaattaa aatttaaaaa agttgacttt 1740
cctgttccta atactagttg gaaaaatcct actgatgtct tgaatgaatc aggtaatgtt 1800
aagagacctc gttataggag taaataa 1827

<210> 89
<211> 608
<212> PRT
<213> Neisseria meningitidis

<400> 89
Leu Gly Ile Ser Arg Lys Ile Ser Leu Ile Leu Ser Ile Leu Ala Val
1 5 10 15

Cys Leu Pro Met His Ala His Ala Ser Asp Leu Ala Asn Asp Ser Phe
20 25 30

Ile Arg Gln Val Leu Asp Arg Gln His Phe Glu Pro Asp Gly Lys Tyr
35 40 45

```
His Leu Phe Gly Ser Arg Gly Glu Leu Ala Glu Arg Ser Gly His Ile
    50                  55                  60

Gly Leu Gly Lys Ile Gln Ser His Gln Leu Gly Asn Leu Met Ile Gln
65                  70                  75                  80

Gln Ala Ala Ile Lys Gly Asn Ile Gly Tyr Ile Val Arg Phe Ser Asp
                85                  90                  95

His Gly His Glu Val His Ser Pro Phe Asp Asn His Ala Ser His Ser
            100                 105                 110

Asp Ser Asp Glu Ala Gly Ser Pro Val Asp Gly Phe Ser Leu Tyr Arg
        115                 120                 125

Ile His Trp Asp Gly Tyr Glu His His Pro Ala Asp Gly Tyr Asp Gly
    130                 135                 140

Pro Gln Gly Gly Gly Tyr Pro Ala Pro Lys Gly Ala Arg Asp Ile Tyr
145                 150                 155                 160

Ser Tyr Asp Ile Lys Gly Val Ala Gln Asn Ile Arg Leu Asn Leu Thr
                165                 170                 175

Asp Asn Arg Ser Thr Gly Gln Arg Leu Ala Asp Arg Phe His Asn Ala
            180                 185                 190

Gly Ser Met Leu Thr Gln Gly Val Gly Asp Gly Phe Lys Arg Ala Thr
        195                 200                 205

Arg Tyr Ser Pro Glu Leu Asp Arg Ser Gly Asn Ala Ala Glu Ala Phe
    210                 215                 220

Asn Gly Thr Ala Asp Ile Val Lys Asn Ile Ile Gly Ala Ala Gly Glu
225                 230                 235                 240

Ile Val Gly Ala Gly Asp Ala Val Gln Gly Ile Ser Glu Gly Ser Asn
                245                 250                 255

Ile Ala Val Met His Gly Leu Gly Leu Leu Ser Thr Glu Asn Lys Met
            260                 265                 270

Ala Arg Ile Asn Asp Leu Ala Asp Met Ala Gln Leu Lys Asp Tyr Ala
        275                 280                 285

Ala Ala Ala Ile Arg Asp Trp Ala Val Gln Asn Pro Asn Ala Ala Gln
    290                 295                 300

Gly Ile Glu Ala Val Ser Asn Ile Phe Met Ala Ala Ile Pro Ile Lys
305                 310                 315                 320

Gly Ile Gly Ala Val Arg Gly Lys Tyr Gly Leu Gly Gly Ile Thr Ala
                325                 330                 335

His Pro Ile Lys Arg Ser Gln Met Gly Ala Ile Ala Leu Pro Lys Gly
            340                 345                 350

Lys Ser Ala Val Ser Asp Asn Phe Ala Asp Ala Ala Tyr Ala Lys Tyr
        355                 360                 365
```

```
Pro Ser Pro Tyr His Ser Arg Asn Ile Arg Ser Asn Leu Glu Gln Arg
    370                 375                 380

Tyr Gly Lys Glu Asn Ile Thr Ser Ser Thr Val Pro Pro Ser Asn Gly
385                 390                 395                 400

Lys Asn Val Lys Leu Ala Asp Gln Arg His Pro Lys Thr Gly Val Pro
                405                 410                 415

Phe Asp Gly Lys Gly Phe Pro Asn Phe Glu Lys His Val Lys Tyr Asp
            420                 425                 430

Thr Lys Leu Asp Ile Gln Glu Leu Ser Gly Gly Gly Ile Pro Lys Ala
        435                 440                 445

Lys Pro Val Phe Asp Ala Lys Pro Arg Trp Glu Val Asp Arg Lys Leu
    450                 455                 460

Asn Lys Leu Thr Thr Arg Glu Gln Val Glu Lys Asn Val Gln Glu Ile
465                 470                 475                 480

Arg Asn Gly Asn Ile Asn Ser Asn Phe Ser Gln His Ala Gln Leu Glu
                485                 490                 495

Arg Glu Ile Asn Lys Leu Lys Ser Ala Asp Glu Ile Asn Phe Ala Asp
            500                 505                 510

Gly Met Gly Lys Phe Thr Asp Ser Met Asn Asp Lys Ala Phe Ser Arg
        515                 520                 525

Leu Val Lys Ser Val Lys Glu Asn Gly Phe Thr Asn Pro Val Val Glu
    530                 535                 540

Tyr Val Glu Ile Asn Gly Lys Ala Tyr Ile Val Arg Gly Asn Asn Arg
545                 550                 555                 560

Val Phe Ala Ala Glu Tyr Leu Gly Arg Ile His Glu Leu Lys Phe Lys
                565                 570                 575

Lys Val Asp Phe Pro Val Pro Asn Thr Ser Trp Lys Asn Pro Thr Asp
            580                 585                 590

Val Leu Asn Glu Ser Gly Asn Val Lys Arg Pro Arg Tyr Arg Ser Lys
        595                 600                 605


<210>   90
<211>   33
<212>   DNA
<213>   Artificial Sequence

<220>
<223>   PCR primer

<400>   90
cgcggatccc atatgttgcc tgcaatcacg att                                33

<210>   91
<211>   28
<212>   DNA
<213>   Artificial Sequence
```

<220>
<223>   PCR primer

<400>   91
cccgctcgag tttagaagcg ggcggcaa                                     28

<210>   92
<211>   34
<212>   DNA
<213>   Artificial Sequence

<220>
<223>   PCR primer

<400>   92
cgcggatccc atatgttcaa atcctttgtc gtca                              34

<210>   93
<211>   27
<212>   DNA
<213>   Artificial Sequence

<220>
<223>   PCR primer

<400>   93
cccgctcgag tttggcggtt ttgctgc                                      27

<210>   94
<211>   30
<212>   DNA
<213>   Artificial Sequence

<220>
<223>   PCR primer

<400>   94
cgcggatccc atatggccgc ccccgcatct                                   30

<210>   95
<211>   31
<212>   DNA
<213>   Artificial Sequence

<220>
<223>   PCR primer

<400>   95
cccgctcgag atttactttt ttgatgtcga c                                 31

<210>   96
<211>   33
<212>   DNA
<213>   Artificial Sequence

<220>
<223>   PCR primer

<400>   96
cgcggatccc atatgtgcca aagcaagagc atc                               33

<210> 97
<211> 25
<212> DNA
<213> Artificial Sequence

<220>
<223> PCR primer

<400> 97
cccgctcgag cgggcggtat tcggg 25

<210> 98
<211> 32
<212> DNA
<213> Artificial Sequence

<220>
<223> PCR primer

<400> 98
cgcggatccc atatggaaac acagctttac at 32

<210> 99
<211> 29
<212> DNA
<213> Artificial Sequence

<220>
<223> PCR primer

<400> 99
cccgctcgag ataataatat cccgcgccc 29

<210> 100
<211> 29
<212> DNA
<213> Artificial Sequence

<220>
<223> PCR primer

<400> 100
cgcggatccc atatgactga caacgcact 29

<210> 101
<211> 26
<212> DNA
<213> Artificial Sequence

<220>
<223> PCR primer

<400> 101
cccgctcgag gaccgcgttg tcgaaa 26

<210> 102
<211> 31
<212> DNA
<213> Artificial Sequence

<220>
<223> PCR primer

<400> 102
cgcggatccc atatgaaaca ccgccaaccg a 31

<210> 103
<211> 31
<212> DNA
<213> Artificial Sequence

<220>
<223> PCR primer

<400> 103
cccgctcgag ttctgtaaaa aaagtatgtg c 31

<210> 104
<211> 32
<212> DNA
<213> Artificial Sequence

<220>
<223> PCR primer

<400> 104
ccggaattct agctagcctt tcagcctgcg gg 32

<210> 105
<211> 28
<212> DNA
<213> Artificial Sequence

<220>
<223> PCR primer

<400> 105
cccgctcgag atcctgctct tttttgcc 28

<210> 106
<211> 31
<212> DNA
<213> Artificial Sequence

<220>
<223> PCR primer

<400> 106
cgcggatccc atatgtgcgg gacactgaca g 31

<210> 107
<211> 28
<212> DNA
<213> Artificial Sequence

<220>
<223> PCR primer

<400> 107
cccgctcgag aggttgtcct tgtctatg 28

<210> 108
<211> 1035
<212> DNA
<213> Neisseria meningitidis

<400> 108
tacgccagcg aaaaactgcg cgaagccaaa tacgcgttca gcgaaaccga wgtcaaaaaa 60
tayttcccyg tcggcaawgt attaaacgga ctgttcgccc aamtcaaaaa actmtacggc 120
atcggattta ccgaaaaaac ygtccccgtc tggcacaaag acgtgcgcta ttktgaattg 180
caacaaaacg gcgaamccat aggcggcgtt tatatggatt tgtacgcacg cgaaggcaaa 240
cgcggcggcg cgtggatgaa cgactacaaa ggccgccgcc gtttttcaga cggcacgctg 300
caaytgccca ccgcctacct cgtctgcaac ttcgccccac ccgtcggcgg cagggaagcc 360
cgcytgagcc acgacgaaat cctcatcctc ttccacgaaa ccggacacgg gctgcaccac 420
ctgcttaccc aagtggacga actgggcgta tccggcatca acggcgtaka atgggacgcg 480
gtcgaactgc ccagccagtt tatggaaaat ttcgtttggg aatacaatgt cttggcacaa 540
mtgtcagccc acgaagaaac cggcgttccc ytgccgaaag aactcttsga caaawtgctc 600
gccgccaaaa acttccaasg cggcatgttc ytsgtccggc aawtggagtt cgccctcttt 660
gatatgatga tttacagcga agacgacgaa ggccgtctga aaaactggca acaggtttta 720
gacagcgtgc gcaaaaaagt cgccgtcatc cagccgcccg aatacaaccg cttcgccttg 780
agcttcggcc acatcttcgc aggcggctat tccgcagctn attacagcta cgcgtgggcg 840
gaagtattga gcgcggacgc atacgccgcc tttgaagaaa gcgacgatgt cgccgccaca 900
ggcaaacgct tttggcagga aatcctcgcc gtcggggnat cgcgcagcgg ngcagaatcc 960
ttcaaagcct tccgcggccg cgaaccgagc atagacgcac tcttgcgcca cagcggtttc 1020
gacaacgcgg tctga 1035

<210> 109
<211> 344
<212> PRT
<213> Neisseria meningitidis

<400> 109
Tyr Ala Ser Glu Lys Leu Arg Glu Ala Lys Tyr Ala Phe Ser Glu Thr
1 5 10 15

Xaa Val Lys Lys Tyr Phe Pro Val Gly Xaa Val Leu Asn Gly Leu Phe
20 25 30

Ala Gln Xaa Lys Lys Leu Tyr Gly Ile Gly Phe Thr Glu Lys Thr Val
35 40 45

Pro Val Trp His Lys Asp Val Arg Tyr Xaa Glu Leu Gln Gln Asn Gly
50 55 60

Glu Xaa Ile Gly Gly Val Tyr Met Asp Leu Tyr Ala Arg Glu Gly Lys
65 70 75 80

Arg Gly Gly Ala Trp Met Asn Asp Tyr Lys Gly Arg Arg Arg Phe Ser
85 90 95

Asp Gly Thr Leu Gln Leu Pro Thr Ala Tyr Leu Val Cys Asn Phe Ala
100 105 110

Pro Pro Val Gly Gly Arg Glu Ala Arg Leu Ser His Asp Glu Ile Leu
115 120 125

Ile Leu Phe His Glu Thr Gly His Gly Leu His His Leu Leu Thr Gln
130 135 140

Val Asp Glu Leu Gly Val Ser Gly Ile Asn Gly Val Xaa Trp Asp Ala
145 150 155 160

```
Val Glu Leu Pro Ser Gln Phe Met Glu Asn Phe Val Trp Glu Tyr Asn
                165                 170                 175

Val Leu Ala Gln Xaa Ser Ala His Glu Glu Thr Gly Val Pro Leu Pro
            180                 185                 190

Lys Glu Leu Xaa Asp Lys Xaa Leu Ala Ala Lys Asn Phe Gln Xaa Gly
        195                 200                 205

Met Phe Xaa Val Arg Gln Xaa Glu Phe Ala Leu Phe Asp Met Met Ile
    210                 215                 220

Tyr Ser Glu Asp Asp Glu Gly Arg Leu Lys Asn Trp Gln Gln Val Leu
225                 230                 235                 240

Asp Ser Val Arg Lys Lys Val Ala Val Ile Gln Pro Pro Glu Tyr Asn
                245                 250                 255

Arg Phe Ala Leu Ser Phe Gly His Ile Phe Ala Gly Gly Tyr Ser Ala
            260                 265                 270

Ala Xaa Tyr Ser Tyr Ala Trp Ala Glu Val Leu Ser Ala Asp Ala Tyr
        275                 280                 285

Ala Ala Phe Glu Glu Ser Asp Asp Val Ala Ala Thr Gly Lys Arg Phe
    290                 295                 300

Trp Gln Glu Ile Leu Ala Val Gly Xaa Ser Arg Ser Gly Ala Glu Ser
305                 310                 315                 320

Phe Lys Ala Phe Arg Gly Arg Glu Pro Ser Ile Asp Ala Leu Leu Arg
                325                 330                 335

His Ser Gly Phe Asp Asn Ala Val
            340
```

## Description

This application claims priority to provisional U.S. application serial no. 60/132,068, filed 30 April 1999; PCT/US99/23573, filed 8 October 1999 (to be published April 2000); and Great Britain application serial no. GB-0004695.3, filed 28 February 2000.

This invention relates to methods of obtaining antigens and immunogens, the antigens and immunogens so obtained, and nucleic acids from the bacterial species: *Neisseria meningitidis.* In particular, it relates to genomic sequences from the bacterium; more particularly its "B" serogroup.

BACKGROUND

*Neisseria meningitidis* is a non-motile, gram negative diplococcus human pathogen. It colonizes the pharynx, causing meningitis and, occasionally, septicaemia in the absence of meningitis. It is closely related to *N. gonorrhoea*, although one feature that clearly differentiates meningococcus from gonococcus is the presence of a polysaccharide capsule that is present in all pathogenic meningococci.

*N meningitidis* causes both endemic and epidemic disease. In the United States the attack rate is 0.6-1 per 100,000 persons per year, and it can be much greater during outbreaks. (see Lieberman *et al*. (1996) Safety and Immunogenicity of a Serogroups A/C *Neisseria meningitidis* Oligosaccharide-Protein Conjugate Vaccine in Young Children. *JAMA* 275(19):1499-1503; *Schuchat et al* (1997) Bacterial Meningitis in the United States in 1995. *N Engl J Med* 337(14):970-976). In developing countries, endemic disease rates are much higher and during epidemics incidence rates can reach 500 cases per 100,000 persons per year. Mortality is extremely high, at 10-20% in the United States, and much higher in developing countries. Following the introduction of the conjugate vaccine against *Haemophilus influenzae, N. meningitidis* is the major cause of bacterial meningitis at all ages in the United States (Schuchat *et al* (1997) *supra*).

Based on the organism's capsular polysaccharide, 12 serogroups of *N. meningitidis* have been identified. Group A is the pathogen most often implicated in epidemic disease in sub-Saharan Africa. Serogroups B and C are responsible for the vast majority of cases in the United States and in most developed countries. Serogroups W135 and Y are responsible for the rest of the cases in the United States and developed countries. The meningococcal vaccine currently in use is a tetravalent polysaccharide vaccine composed of serogroups A, C, Y and W135. Although efficacious in adolescents and adults, it induces a poor immune response and short duration of protection, and cannot be used in infants (e.g., Morbidity and Mortality weekly report, Vol. 46, No. RR-5 (1997)). This is because polysaccharides are T-cell independent antigens that induce a weak immune response that cannot be boosted by repeated immunization. Following the success of the vaccination against *H. influenzae*, conjugate vaccines against serogroups A and C have been developed and are at the final stage of clinical testing (Zollinger WD "New and Improved Vaccines Against Meningococcal Disease". In: *New Generation Vaccines, supra*, pp. 469-488; Lieberman *et al* (1996) *supra*; Costantino *et al* (1992) Development and phase I clinical testing of a conjugate vaccine against meningococcus A (menA) and C (menC) *(Vaccine* 10:691-698)).

Meningococcus B (MenB) remains a problem, however. This serotype currently is responsible for approximately 50% of total meningitis in the United States, Europe, and South America. The polysaccharide approach cannot be used because the MenB capsular polysaccharide is a polymer of $\alpha$(2-8)-linked N-acetyl neuraminic acid that is also present in mammalian tissue. This results in tolerance to the antigen; indeed, if an immune response were elicited, it would be anti-self, and therefore undesirable. In order to avoid induction of autoimmunity and to induce a protective immune response, the capsular polysaccharide has, for instance, been chemically modified substituting the *N*-acetyl groups with *N*-propionyl groups, leaving the specific antigenicity unaltered (Romero & Outschoom (1994) Current status of Meningococcal group B vaccine candidates: capsular or non-capsular? *Clin Microbiol Rev* 7(4):559-575).

Alternative approaches to MenB vaccines have used complex mixtures of outer membrane proteins (OMPs), containing either the OMPs alone, or OMPs enriched in porins, or deleted of the class 4 OMPs that are believed to induce antibodies that block bactericidal activity. This approach produces vaccines that are not well characterized. They are able to protect against the homologous strain, but are not effective at large where there are many antigenic variants of the outer membrane proteins. To overcome the antigenic variability, multivalent vaccines containing up to nine different porins have been constructed (e.g., Poolman JT (1992) Development of a meningococcal vaccine. *Infect. Agents Dis.* 4:13-28). Additional proteins to be used in outer membrane vaccines have been the opa and opc proteins, but none of these approaches have been able to overcome the antigenic variability (e.g., Ala'Aldeen & Borriello (1996) The meningococcal transferrin-binding proteins 1 and 2 are both surface exposed and generate bactericidal antibodies capable of killing homologous and heterologous strains. *Vaccine* 14(1):49-53).

A certain amount of sequence data is available for meningococcal and gonococcal genes and proteins (e.g., EP-A-0467714, W096/29412), but this is by no means complete. The provision of further sequences could provide an opportunity to identify secreted or surface-exposed proteins that are presumed targets for the immune system and which are not antigenically variable or at least are more antigenically conserved than other and more variable regions. Thus, those antigenic sequences that are more highly conserved are preferred sequences. Those sequences specific to *Neisseria meningitidis* or *Neisseria gonorrhoeae* that are more highly conserved are further preferred sequences. For instance, some of the identified proteins could be components of efficacious vaccines against meningococcus B, some could be components of vaccines against all meningococcal serotypes, and others could be components of vaccines against all pathogenic *Neisseriae.* The identification of sequences from the bacterium will also facilitate the production of biological probes, particularly organism-specific probes.

It is thus an object of the invention is to provide Neisserial DNA sequences which (1) encode proteins predicted and/or shown to be antigenic or immunogenic, (2) can be used as probes or amplification primers, and (3) can be analyzed by bioinformatics.

BRIEF DESCRIPTION OF THE DRAWINGS

Fig. 1 illustrates the products of protein expression and purification of the predicted ORF 919 as cloned and expressed in *E. coli.*

Fig. 2 illustrates the products of protein expression and purification of the predicted ORF 279 as cloned and expressed in *E. coli*.

Fig. 3 illustrates the products of protein expression and purification of the predicted ORF 576-1 as cloned and expressed in *E. coli*.

Fig. 4 illustrates the products of protein expression and purification of the predicted ORF 519-1 as cloned and expressed in *E. coli*.

Fig. 5 illustrates the products of protein expression and purification of the predicted ORF 121-1 as cloned and expressed in *E. coli*.

Fig. 6 illustrates the products of protein expression and purification of the predicted ORF 128-1 as cloned and expressed in *E*. *coli*.

Fig. 7 illustrates the products of protein expression and purification of the predicted ORF 206 as cloned and expressed in *E. coli*.

Fig. 8 illustrates the products of protein expression and purification of the predicted ORF 287 as cloned and expressed in *E. coli*.

Fig. 9 illustrates the products of protein expression and purification of the predicted ORF 406 as cloned and expressed in *E. coli*.

Fig. 10 illustrates the hydrophilicity plot, antigenic index and AMPHI regions of the products of protein expression the predicted ORF 919 as cloned and expressed in *E. coli*.

Fig. 11 illustrates the hydrophilicity plot, antigenic index and AMPHI regions of the products of protein expression the predicted ORF 279 as cloned and expressed in *E. coli*.

Fig. 12 illustrates the hydrophilicity plot, antigenic index and AMPHI regions of the products of protein expression the predicted ORF 576-1 as cloned and expressed in E. *coli.*

Fig. 13 illustrates the hydrophilicity plot, antigenic index and AMPHI regions of the products of protein expression the predicted ORF 519-1 as cloned and expressed in *E. coli*.

Fig. 14 illustrates the hydrophilicity plot, antigenic index and AMPHI regions of the products of protein expression the predicted ORF 121-1 as cloned and expressed in *E. coli*.

Fig. 15 illustrates the hydrophilicity plot, antigenic index and AMPHI regions of the products of protein expression the predicted ORF 128-1 as cloned and expressed in *E. coli*.

Fig. 16 illustrates the hydrophilicity plot, antigenic index and AMPHI regions of the products of protein expression the predicted ORF 206 as cloned and expressed in *E. coli*.

Fig. 17 illustrates the hydrophilicity plot, antigenic index and AMPHI regions of the products of protein expression the predicted ORF 287 as cloned and expressed in E. *coli.*

Fig. 18 illustrates the hydrophilicity plot, antigenic index and AMPHI regions of the products of protein expression the predicted ORF 406 as cloned and expressed in *E. coli*.

THE INVENTION

The first complete sequence of the genome of *N. meningitidis* was disclosed as 961 partial contiguous nucleotide sequences, shown as SEQ ID NOs:1-961 of co-owned PCT/US99/23573 (the '573 application), filed 8 October 1999 (to be published April 2000). A single sequence full length genome of *N. meningitidis* was also disclosed as SEQ ID NO. 1068 of the '573 application. The invention is based on a full length genome of *N. meningitidis* which appears as SEQ ID NO. 1 in the present application as Appendix A hereto. The 961 sequences of the '573 application represent substantially the whole genome of serotype B of *N. meningitidis* (>99.98%). There is partial overlap between some of the 961 contiguous sequences ("contigs") shown in the 961 sequences, which overlap was used to construct the single full length sequence shown in SEQ ID NO. 1 in Appendix A hereto, using the TIGR Assembler [G.S. Sutton et al., *TIGR Assembler: A New Tool for Assembling Large Shotgun Sequencing Projects,* Genome Science and Technology, 1:9-19 (1995)]. Some of the nucleotides in the contigs had been previously released. (See ftp:11ftp.tigr.org/pub/data/ n_meningitidis on the world-wide web or "WWW"). The coordinates of the 2508 released sequences in the present contigs are presented in Appendix A of the '573 application. These data include the contig number (or i.d.) as presented in the first column; the name of the sequence as found on WWW is in the second column; with the coordinates of the contigs in the third and fourth columns, respectively. The sequences of certain MenB ORFs presented in Appendix B of the '573 application feature in International Patent Application filed by Chiron SpA on October 9, 1998 (PCT/ IB98/01665) and January 14, 1999 (PCT/IB99/00103) respectively. Appendix B hereto provides a listing of 2158 open reading frames contained within the full length sequence found in SEQ ID NO. 1 in Appendix A hereto. The information set forth in Appendix B hereto includes the "NMB" name of the sequence, the putative translation product, and the beginning and ending nucleotide positions within SEQ ID NO. 1 which comprise the open reading frames. These open reading frames are referred to herein as the "NMB open reading frames".

In a first aspect, the invention provides nucleic acid including the *N. meningitidis* nucleotide sequence shown in SEQ ID NO. 1 in Appendix A hereto. It also provides nucleic acid comprising sequences having sequence identity to the nucleotide sequence disclosed herein. Depending on the particular sequence, the degree of sequence identity is preferably greater than 50% (e.g., 60%, 70%, 80%, 90%, 95%, 99% or more). These sequences include, for instance, mutants and allelic variants. The degree of sequence identity cited herein is determined across the length of the sequence determined by the Smith-Waterman homology search algorithm as implemented in MPSRCH program (Oxford Molecular) using an affine gap search with the following parameters: gap open penalty 12, gap extension penalty 1.

The invention also provides nucleic acid including a fragment of one or more of the nucleotide sequences set out herein, including the NMB open reading frames shown in Appendix B hereto. The fragment should comprise at least n consecutive nucleotides from the sequences and, depending on the particular sequence, n is 10 or more (e.g., 11, 12, 13, 14, 15, 16, 17, 18, 19, 20, 21, 22, 23, 24, 25, 30, 35, 40, 45, 50, 60, 75, 100 or more). Preferably, the fragment is unique to the genome of *N. meningitidis,* that is to say it is not present in the genome of another organism. More preferably, the fragment is unique to the genome of strain B of *N. meningitidis.* The invention also provides nucleic acid that hybridizes to those provided herein. Conditions for hybridizing are disclosed herein.

The invention also provides nucleic acid including sequences complementary to those described above (e. g., for antisense, for probes, or for amplification primers).

Nucleic acid according to the invention can, of course, be prepared in many ways (e.g., by chemical synthesis, from DNA libraries, from the organism itself, etc.) and can take various forms (e.g., single-stranded, double-stranded, vectors, probes, primers, etc.). The term "nucleic acid" includes DNA and RNA, and also their analogs, such as those containing modified backbones, and also peptide nucleic acid (PNA) etc.

It will be appreciated that, as SEQ ID NOs:1-961 of the '573 application represent the substantially complete genome of the organism, with partial overlap, references to SEQ ID NOs:1-961 of the '573 application include within their scope references to the complete genomic sequence, that is, SEQ ID NO. 1 hereof. For example, where two SEQ ID NOs overlap, the invention encompasses the single sequence which is formed by assembling the two overlapping sequences, which full sequence will be found in SEQ ID NO. 1 hereof. Thus, for instance, a nucleotide sequence which bridges two SEQ ID NOs but is not present in its entirety in either SEQ ID NO is still within the scope of the invention. Such a sequence will be present in its entirety in the single full length sequence of SEQ ID NO. 1 of the present application.

The invention also provides vectors including nucleotide sequences of the invention (e.g., expression vectors, sequencing vectors, cloning vectors, etc.) and host cells transformed with such vectors.

According to a further aspect, the invention provides a protein including an amino acid sequence encoded within a *N. meningitidis* nucleotide sequence set out herein. It also provides proteins comprising sequences having sequence identity to those proteins. Depending on the particular sequence, the degree of sequence identity is preferably greater than 50% (e.g., 60%, 70%, 80%, 90%, 95%, 99% or more). Sequence identity is determined as above disclosed. These homologous proteins include mutants and allelic variants, encoded within the *N. meningitidis* nucleotide sequence set out herein.

The invention further provides proteins including fragments of an amino acid sequence encoded within a *N. meningitidis* nucleotide sequence set out in the sequence listing. The fragments should comprise at least *n* consecutive amino acids from the sequences and, depending on the particular sequence, *n* is 7 or more (e.g., 8, 10, 12, 14, 16, 18, 20 or more). Preferably the fragments comprise an epitope from the sequence.

The proteins of the invention can, of course, be prepared by various means (e.g., recombinant expression, purification from cell culture, chemical synthesis, *etc.)* and in various forms (e.g. native, fusions *etc.).* They are preferably prepared in substantially isolated form (*i.e*., substantially free from other *N. meningitidis* host cell proteins).

Various tests can be used to assess the *in vivo* immunogenicity of the proteins of the invention. For example, the proteins can be expressed recombinantly or chemically synthesized and used to screen patient sera by immunoblot. A positive reaction between the protein and patient serum indicates that the patient has previously mounted an immune response to the protein in question; i.e., the protein is an immunogen. This method can also be used to identify immunodominant proteins.

The invention also provides nucleic acid encoding a protein of the invention.

In a further aspect, the invention provides a computer, a computer memory, a computer storage medium (e.g., floppy disk, fixed disk, CD-ROM, etc.), and/or a computer database containing the nucleotide sequence of nucleic acid according to the invention. Preferably, it contains one or more of the *N. meningitidis* nucleotide sequences set out herein.

This may be used in the analysis of the *N. meningitidis* nucleotide sequences set out herein. For instance, it may be used in a search to identify open reading frames (ORFs) or coding sequences within the sequences.

In a further aspect, the invention provides a method for identifying an amino acid sequence, comprising the step of searching for putative open reading frames or protein-coding sequences within a *N. meningitidis* nucleotide sequence set out herein. Similarly, the invention provides the use of a *N. meningitidis* nucleotide sequence set out herein in a search for putative open reading frames or protein-coding sequences.

Open-reading frame or protein-coding sequence analysis is generally performed on a computer using standard bioinformatic techniques. Typical algorithms or program used in the analysis include ORFFINDER (NCBI), GENMARK [Borodovsky & McIninch (1993) *Computers Chem* 17:122-133], and GLIMMER [Salzberg et al. (1998) *Nucl Acids Res* 26:544-548].

A search for an open reading frame or protein-coding sequence may comprise the steps of searching a *N. meningitidis* nucleotide sequence set out herein for an initiation codon and searching the upstream sequence for an in-frame termination codon. The intervening codons represent a putative protein-coding sequence. Typically, all six possible reading frames of a sequence will be searched.

An amino acid sequence identified in this way can be expressed using any suitable system to give a protein. This protein can be used to raise antibodies which recognize epitopes within the identified amino acid sequence. These antibodies can be used to screen *N. meningitidis* to detect the presence of a protein comprising the identified amino acid sequence.

Furthermore, once an ORF or protein-coding sequence is identified, the sequence can be compared with sequence databases. Sequence analysis tools can be found at NCBI (http://www.ncbi.nlm.nih.gov) e.g., the algorithms BLAST, BLAST2, BLASTn, BLASTp, tBLASTn, BLASTx, & tBLASTx [see also Altschul *et al*. (1997) Gapped BLAST and PSI-BLAST: new generation of protein database search programs. *Nucleic Acids Research* 25:2289-3402]. Suitable databases for comparison include the nonredundant GenBank, EMBL, DDBJ and PDB sequences, and the nonredundant GenBank CDS translations, PDB, SwissProt, Spupdate and PIR sequences. This comparison may give an indication of the function of a protein.

Hydrophobic domains in an amino acid sequence can be predicted using algorithms such as those based on the statistical studies of Esposti *et al*. [Critical evaluation of the hydropathy of membrane proteins (1990) *Eur JBiochem* 190:207-219]. Hydrophobic domains represent potential transmembrane regions or hydrophobic leader sequences, which suggest that the proteins may be secreted or be surface-located. These properties are typically representative of good immunogens.

Similarly, transmembrane domains or leader sequences can be predicted using the PSORT algorithm (http://www.psort.nibb.ac.jp), and functional domains can be predicted using the MOTIFS program (GCG Wisconsin & PROSITE).

The invention also provides nucleic acid including an open reading frame or protein-coding sequence present in a N. *meningitidis* nucleotide sequence set out herein. Furthermore, the invention provides a protein including the amino acid sequence encoded by this open reading frame or protein-coding sequence.

According to a further aspect, the invention provides antibodies which bind to these proteins. These may be polyclonal or monoclonal and may be produced by any suitable means known to those skilled in the art.

The antibodies of the invention can be used in a variety of ways, e.g., for confirmation that a protein is expressed, or to confirm where a protein is expressed. Labeled antibody (e.g., fluorescent labeling for FACS) can be incubated with intact bacteria and the presence of label on the bacterial surface confirms the location of the protein, for instance.

According to a further aspect, the invention provides compositions including protein, antibody, and/or nucleic acid according to the invention. These compositions may be suitable as vaccines, as immunogenic compositions, or as diagnostic reagents.

The invention also provides nucleic acid, protein, or antibody according to the invention for use as medicaments (e.g., as vaccines) or as diagnostic reagents. It also provides the use of nucleic acid, protein, or antibody according to the invention in the manufacture of (I) a medicament for treating or preventing infection due to Neisserial bacteria (ii) a diagnostic reagent for detecting the presence of Neisserial bacteria or of antibodies raised against Neisserial bacteria. Said Neisserial bacteria may be any species or strain (such as *N. gonorrhoeae*) but are preferably *N. meningitidis*, especially strain A, strain B or strain C.

In still yet another aspect, the present invention provides for compositions including proteins, nucleic acid molecules, or antibodies. More preferable aspects of the present invention are drawn to immunogenic compositions of proteins. Further preferable aspects of the present invention contemplate pharmaceutical immunogenic compositions of proteins or vaccines and the use thereof in the manufacture of a medicament for the treatment or prevention of infection due to Neisserial bacteria, preferably infection of MenB.

The invention also provides a method of treating a patient, comprising administering to the patient a therapeutically effective amount of nucleic acid, protein, and/or antibody according to the invention.

According to further aspects, the invention provides various processes.

A process for producing proteins of the invention is provided, comprising the step of culturing a host cell according to the invention under conditions which induce protein expression. A process which may further include chemical synthesis of proteins and/or chemical synthesis (at least in part) of nucleotides.

A process for detecting polynucleotides of the invention is provided, comprising the steps of: (a) contacting a nucleic probe according to the invention with a biological sample under hybridizing conditions to form duplexes; and (b) detecting said duplexes.

A process for detecting proteins of the invention is provided, comprising the steps of: (a) contacting an antibody according to the invention with a biological sample under conditions suitable for the formation of an antibody-antigen complexes; and (b) detecting said complexes.

Another aspect of the present invention provides for a process for detecting antibodies that selectably bind to antigens or polypeptides or proteins specific to any species or strain of Neisserial bacteria and preferably to strains of *N. gonorrhoeae* but more preferably to strains *of N. meningitidis,* especially strain A, strain B or strain C, more preferably MenB, where the process comprises the steps of: (a) contacting antigen or polypeptide or protein according to the invention with a biological sample under conditions suitable for the formation of an antibody-antigen complexes; and (b) detecting said complexes.

Having now generally described the invention, the same will be more readily understood through reference to the following examples which are provided by way of illustration, and are not intended to be limiting of the present invention, unless specified.

<u>Methodology - Summary of standard procedures and techniques.</u>

General

This invention provides *Neisseria meningitidis* MenB nucleotide sequences, amino acid sequences encoded therein. With these disclosed sequences, nucleic acid probe assays and expression cassettes and vectors can be produced. The proteins can also be chemically synthesized. The expression vectors can be transformed into host cells to produce proteins. The purified or isolated polypeptides can be used to produce antibodies to detect MenB proteins. Also, the host cells or extracts can be utilized for biological assays to isolate agonists or antagonists. In addition, with these sequences one can search to identify open reading frames and identify amino acid sequences. The proteins may also be used in immunogenic compositions and as vaccine components.

The practice of the present invention will employ, unless otherwise indicated, conventional techniques of molecular biology, microbiology, recombinant DNA, and immunology, which are within the skill of the art. Such techniques are explained fully in the literature e.g., Sambrook *Molecular Cloning; A Laboratory Manual, Second Edition* (1989); *DNA Cloning, Volumes I and ii* (D.N Glover ed. 1985); *Oligonucleotide Synthesis* (M.J. Gait ed, 1984); *Nucleic Acid Hybridization* (B.D. Hames & S.J. Higgins eds. 1984); *Transcription and Translation* (B.D. Hames & S.J. Higgins eds. 1984); *Animal Cell Culture* (R.I. Freshney ed. 1986); *Immobilized Cells and Enzymes* (IRL Press, 1986); B. Perbal, *A Practical Guide to Molecular Cloning* (1984); the *Methods in Enzymology* series (Academic Press, Inc.), especially volumes 154 & 155; *Gene Transfer Vectors for Mammalian Cells* (J.H. Miller and M.P. Calos eds. 1987, Cold Spring Harbor Laboratory); Mayer and Walker, eds. (1987), *Immunochemical Methods in Cell and Molecular Biology* (Academic Press, London); Scopes, (1987) *Protein Purification: Principles and Practice,* Second Edition (Springer-Verlag, N.Y.), and *Handbook of Experimental Immunology, Volumes I-IV* (D.M. Weir and C.C. Blackwell eds 1986).

Standard abbreviations for nucleotides and amino acids are used in this specification.

All publications, patents, and patent applications cited herein are incorporated <u>in</u> <u>full</u> by reference.

Expression systems

The *Neisseria* MenB nucleotide sequences can be expressed in a variety of different expression systems; for example those used with mammalian cells, plant cells, baculoviruses, bacteria, and yeast.

i. Mammalian Systems

Mammalian expression systems are known in the art. A mammalian promoter is any DNA sequence capable of binding mammalian RNA polymerase and initiating the downstream (3') transcription of a coding sequence (e.g., structural gene) into mRNA. A promoter will have a transcription initiating region, which is usually placed proximal to the 5' end of the coding sequence, and a TATA box, usually located 25-30 base pairs (bp) upstream of the transcription initiation site. The TATA box is thought to direct RNA polymerase II to begin RNA synthesis at the correct site. A mammalian promoter will also contain an upstream promoter element, usually located within 100 to 200 bp upstream of the TATA box. An upstream promoter element determines the rate at which transcription is initiated and can act in either orientation (Sambrook et al. (1989) "Expression of Cloned Genes in Mammalian Cells." In *Molecular Cloning: A Laboratory Manual, 2nd ed.).*

Mammalian viral genes are often highly expressed and have a broad host range; therefore sequences encoding mammalian viral genes provide particularly useful promoter sequences. Examples include the SV40 early promoter, mouse mammary tumor virus LTR promoter, adenovirus major late promoter (Ad MLP), and herpes simplex virus promoter. In addition, sequences derived from non-viral genes, such as the murine metallothionein gene, also provide useful promoter sequences. Expression may be either constitutive or regulated (inducible). Depending on the promoter selected, many promotes may be inducible using known substrates, such as the use of the mouse mammary tumor virus (MMTV) promoter with the glucocorticoid responsive element (GRE) that is induced by glucocorticoid in hormone-responsive transformed cells (see for example, U.S. Patent 5,783,681).

The presence of an enhancer element (enhancer), combined with the promoter elements described above, will usually increase expression levels. An enhancer is a regulatory DNA sequence that can stimulate transcription up to 1000-fold when linked to homologous or heterologous promoters, with synthesis beginning at the normal RNA start site. Enhancers are also active when they are placed upstream or downstream from the transcription initiation site, in either normal or flipped orientation, or at a distance of more than 1000 nucleotides from the promoter (Maniatis et al. (1987) *Science* 236:1237; Alberts et al. (1989) *Molecular Biology of the Cell,* 2nd ed.). Enhancer elements derived from viruses may be particularly useful, because they usually have a broader host range. Examples include the SV40 early gene enhancer (Dijkema et al (1985) *EMBO J. 4*:761) and the enhancer/promoters derived from the long terminal repeat (LTR) of the Rous Sarcoma Virus (Gorman et al. (1982b) *Proc. Natl. Acad. Sci. 79*:6777) and from human cytomegalovirus (Boshart et al. (1985) *Cell 41*:521). Additionally, some enhancers are regulatable and become active only in the presence of an inducer, such as a hormone or metal ion (Sassone-Corsi and Borelli (1986) *Trends Genet. 2*:215; Maniatis et al. (1987) Science 236:1237).

A DNA molecule may be expressed intracellularly in mammalian cells. A promoter sequence may be directly linked with the DNA molecule, in which case the first amino acid at the N-terminus of the recombinant protein will always be a methionine, which is encoded by the ATG start codon. If desired, the N-terminus may be cleaved from the protein by in *vitro* incubation with cyanogen bromide.

Alternatively, foreign proteins can also be secreted from the cell into the growth media by creating chimeric DNA molecules that encode a fusion protein comprised of a leader sequence fragment that provides for secretion of the foreign protein in mammalian cells. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell. The adenovirus tripartite leader is an example of a leader sequence that provides for secretion of a foreign protein in mammalian cells.

Usually, transcription termination and polyadenylation sequences recognized by mammalian cells are regulatory regions located 3' to the translation stop codon and thus, together with the promoter elements, flank the coding sequence. The 3' terminus of the mature mRNA is formed by site-specific post-transcriptional cleavage and polyadenylation (Birnstiel et al. (1985) *Cell* 41:349; Proudfoot and Whitelaw (1988) "Termination and 3' end processing of eukaryotic RNA. In *Transcription and splicing* (ed. B.D. Hames and D.M. Glover); Proudfoot (1989) *Trends Biochem. Sci.* 14:105). These sequences direct the transcription of an mRNA which can be translated into the polypeptide encoded by the DNA. Examples of transcription terminator/polyadenylation signals include those derived from SV40 (Sambrook et al (1989) "Expression of cloned genes in cultured mammalian cells." In *Molecular Cloning: A Laboratory Manual*).

Usually, the above-described components, comprising a promoter, polyadenylation signal, and transcription termination sequence are put together into expression constructs. Enhancers, introns with functional splice donor and acceptor sites, and leader sequences may also be included in an expression construct, if desired. Expression constructs are often maintained in a replicon, such as an extrachromosomal element (e.g., plasmids) capable of stable maintenance in a host, such as mammalian cells or bacteria. Mammalian replication systems include those derived from animal viruses, which require trans-acting factors to replicate. For example, plasmids containing the replication systems of papovaviruses, such as SV40 (Gluzman (1981) *Cell 23*:175) or polyomavirus, replicate to extremely high copy number in the presence of the appropriate viral T antigen. Additional examples of mammalian replicons include those derived from bovine papillomavirus and Epstein-Barr virus. Additionally, the replicon may have two replication systems, thus allowing it to be maintained, for example, in mammalian cells for expression and in a prokaryotic host for cloning and amplification. Examples of such mammalian-bacteria shuttle vectors include pMT2 (Kaufman et al. (1989) *Mol. Cell. Biol. 9*:946) and pHEBO (Shimizu et al. (1986) *Mol. Cell. Biol. 6*:1074).

The transformation procedure used depends upon the host to be transformed. Methods for introduction of heterologous polynucleotides into mammalian cells are known in the art and include dextran-mediated transfection, calcium phosphate precipitation, polybrene mediated transfection, protoplast fusion, electroporation, encapsulation of the polynucleotide(s) in liposomes, and direct microinjection of the DNA into nuclei.

Mammalian cell lines available as hosts for expression are known in the art and include many immortalized cell lines available from the American Type Culture Collection (ATCC), including but not limited to, Chinese hamster ovary (CHO) cells, HeLa cells, baby hamster kidney (BHK) cells, monkey kidney cells (COS), human hepatocellular carcinoma cells (e.g., Hep G2), and a number of other cell lines.

ii. Plant Cellular Expression Systems

There are many plant cell culture and whole plant genetic expression systems known in the art. Exemplary plant cellular genetic expression systems include those described in patents, such as: U.S. 5,693,506; US 5,659,122; and US 5,608,143. Additional examples of genetic expression in plant cell culture has been described by Zenk, *Phytochemistry* 30:3861-3863 (1991). Descriptions of plant protein signal peptides may be found in addition to the references described above in Vaulcombe et al., *Mol. Gen. Genet.* 209:33-40 (1987); Chandler et al., *Plant Molecular Biology* 3:407-418 (1984); Rogers, *J. Biol. Chem.* 260:3731-3738 (1985); Rothstein et al., *Gene* 55:353-356 (1987); Whittier et al., Nucleic Acids Research 15:2515-2535 (1987); Wirsel et al., *Molecular Microbiology* 3:3-14 (1989); Yu et al., *Gene* 122:247-253 (1992). A description of the regulation of plant gene expression by the phytohormone, gibberellic acid and secreted enzymes induced by gibberellic acid can be found in R.L. Jones and J. MacMillin, Gibberellins: in: *Advanced Plant Physiology,.* Malcolm B. Wilkins, ed., 1984 Pitman Publishing Limited, London, pp. 21-52. References that describe other metabolically-regulated genes: Sheen, *Plant Cell,* 2:1027-1038(1990); Maas et al., *EMBO J.* 9:3447-3452 (1990); Benkel and Hickey, *Proc. Natl. Acad. Sci.* 84:1337-1339 (1987)

Typically, using techniques known in the art, a desired polynucleotide sequence is inserted into an expression cassette comprising genetic regulatory elements designed for operation in plants. The expression cassette is inserted into a desired expression vector with companion sequences upstream and downstream from the expression cassette suitable for expression in a plant host. The companion sequences will be of plasmid or viral origin and provide necessary characteristics to the vector to permit the vectors to move DNA from an original cloning host, such as bacteria, to the desired plant host. The basic bacterial/plant vector construct will preferably provide a broad host range prokaryote replication origin; a prokaryote selectable marker; and, for Agrobacterium transformations, T DNA sequences for Agrobacterium-mediated transfer to plant chromosomes. Where the heterologous gene is not readily amenable to detection, the construct will preferably also have a selectable marker gene suitable for determining if a plant cell has been transformed. A general review of suitable markers, for example for the members of the grass family, is found in Wilmink and Dons, 1993, *Plant Mol. Biol. Reptr,* 11(2):165-185.

Sequences suitable for permitting integration of the heterologous sequence into the plant genome are also recommended. These might include transposon sequences and the like for homologous recombination as well as Ti sequences which permit random insertion of a heterologous expression cassette into a plant genome. Suitable prokaryote selectable markers include resistance toward antibiotics such as ampicillin or tetracycline. Other DNA sequences encoding additional functions may also be present in the vector, as is known in the art.

The nucleic acid molecules of the subject invention may be included into an expression cassette for expression of the protein(s) of interest. Usually, there will be only one expression cassette, although two or more are feasible. The recombinant expression cassette will contain in addition to the heterologous protein encoding sequence the following elements, a promoter region, plant 5' untranslated sequences, initiation codon depending upon whether or not the structural gene comes equipped with one, and a transcription and translation termination sequence. Unique restriction enzyme sites at the 5' and 3' ends of the cassette allow for easy insertion into a pre-existing vector.

A heterologous coding sequence may be for any protein relating to the present invention. The sequence encoding the protein of interest will encode a signal peptide which allows processing and translocation of the protein, as appropriate, and will usually lack any sequence which might result in the binding of the desired protein of the invention to a membrane. Since, for the most part, the transcriptional initiation region will be for a gene which is expressed and translocated during germination, by employing the signal peptide which provides for translocation, one may also provide for translocation of the protein of interest. In this way, the protein(s) of interest will be translocated from the cells in which they are expressed and may be efficiently harvested. Typically secretion in seeds are across the aleurone or scutellar epithelium layer into the endosperm of the seed. While it is not required that the protein be secreted from the cells in which the protein is produced, this facilitates the isolation and purification of the recombinant protein.

Since the ultimate expression of the desired gene product will be in a eucaryotic cell it is desirable to determine whether any portion of the cloned gene contains sequences which will be processed out as introns by the host's spliceosome machinery. If so, site-directed mutagenesis of the "intron" region may be conducted to prevent losing a portion of the genetic message as a false intron code, Reed and Maniatis, *Cell* 41:95-105, 1985.

The vector can be microinjected directly into plant cells by use of micropipettes to mechanically transfer the recombinant DNA. Crossway, *Mol. Gen. Genet,* 202:179-185, 1985. The genetic material may also be transferred into the plant cell by using polyethylene glycol, Krens, et al., *Nature,* 296, 72-74, 1982. Another method of introduction of nucleic acid segments is high velocity ballistic penetration by small particles with the nucleic acid either within the matrix of small beads or particles, or on the surface, Klein, et al., *Nature,* 327, 70-73, 1987 and Knudsen and Muller, 1991, *Planta,* 185:330-336 teaching particle bombardment of barley endosperm to create transgenic barley. Yet another method of introduction would be fusion of protoplasts with other entities, either minicells, cells, lysosomes or other fusible lipid-surfaced bodies, Fraley, et al., *Proc. Natl. Acad. Sci. USA,* 79, 1859-1863, 1982.

The vector may also be introduced into the plant cells by electroporation. (Fromm et al., *Proc. Natl Acad. Sci. USA* 82:5824, 1985). In this technique, plant protoplasts are electroporated in the presence of plasmids containing the gene construct. Electrical impulses of high field strength reversibly permeabilize biomembranes allowing the introduction of the plasmids. Electroporated plant protoplasts reform the cell wall, divide, and form plant callus.

All plants from which protoplasts can be isolated and cultured to give whole regenerated plants can be transformed by the present invention so that whole plants are recovered which contain the transferred gene. It is known that practically all plants can be regenerated from cultured cells or tissues, including but not limited to all major species of sugarcane, sugar beet, cotton, fruit and other trees, legumes and vegetables. Some suitable plants include, for example, species from the genera *Fragaria, Lotus, Medicago, Onobrychis, Trifolium, Trigonella, Vigna, Citrus, Linum, Geranium, Manihot, Daucus, Arabidopsis, Brassica, Raphanus, Sinapis, Atropa, Capsicum, Datura, Hyoscyamus, Lycopersion, Nicotiana, Solanum, Petunia, Digitalis, Majorana, Cichorium, Helianthus, Lactuca, Bromus, Asparagus, Antirrhinum, Hererocallis, Nemesia, Pelargonium, Panicum, Pennisetum, Ranunculus, Senecio, Salpiglossis, Cucumis, Browaalia, Glycine, Lolium, Zea, Triticum, Sorghum,* and *Datura*.

Means for regeneration vary from species to species of plants, but generally a suspension of transformed protoplasts containing copies of the heterologous gene is first provided. Callus tissue is formed and shoots may be induced from callus and subsequently rooted. Alternatively, embryo formation can be induced from the protoplast suspension. These embryos germinate as natural embryos to form plants. The culture media will generally contain various amino acids and hormones, such as auxin and cytokinins. It is also advantageous to add glutamic acid and proline to the medium, especially for such species as corn and alfalfa. Shoots and roots normally develop simultaneously. Efficient regeneration will depend on the medium, on the genotype, and on the history of the culture. If these three variables are controlled, then regeneration is fully reproducible and repeatable.

In some plant cell culture systems, the desired protein of the invention may be excreted or alternatively, the protein may be extracted from the whole plant. Where the desired protein of the invention is secreted into the medium, it may be collected. Alternatively, the embryos and embryoless-half seeds or other plant tissue may be mechanically disrupted to release any secreted protein between cells and tissues. The mixture may be suspended in a buffer solution to retrieve soluble proteins. Conventional protein isolation and purification methods will be then used to purify the recombinant protein. Parameters of time, temperature pH, oxygen, and volumes will be adjusted through routine methods to optimize expression and recovery of heterologous protein.

iii. Baculovirus Systems

The polynucleotide encoding the protein can also be inserted into a suitable insect expression vector, and is operably linked to the control elements within that vector. Vector construction employs techniques which are known in the art. Generally, the components of the expression system include a transfer vector, usually a bacterial plasmid, which contains both a fragment of the baculovirus genome, and a convenient restriction site for insertion of the heterologous gene or genes to be expressed; a wild type baculovirus with a sequence homologous to the baculovirus-specific fragment in the transfer vector (this allows for the homologous recombination of the heterologous gene in to the baculovirus genome); and appropriate insect host cells and growth media.

After inserting the DNA sequence encoding the protein into the transfer vector, the vector and the wild type viral genome are transfected into an insect host cell where the vector and viral genome are allowed to recombine. The packaged recombinant virus is expressed and recombinant plaques are identified and purified. Materials and methods for baculovirus/insect cell expression systems are commercially available in kit form from, *inter alia*, Invitrogen, San Diego CA ("MaxBac" kit). These techniques are generally known to those skilled in the art and fully described in Summers and Smith, *Texas Agricultural Experiment Station Bulletin No. 1555* (1987) (hereinafter "Summers and Smith").

Prior to inserting the DNA sequence encoding the protein into the baculovirus genome, the above described components, comprising a promoter, leader (if desired), coding sequence of interest, and transcription termination sequence, are usually assembled into an intermediate transplacement construct (transfer vector). This construct may contain a single gene and operably linked regulatory elements; multiple genes, each with its owned set of operably linked regulatory elements; or multiple genes, regulated by the same set of regulatory elements. Intermediate transplacement constructs are often maintained in a replicon, such as an extrachromosomal element (e.g., plasmids) capable of stable maintenance in a host, such as a bacterium. The replicon will have a replication system, thus allowing it to be maintained in a suitable host for cloning and amplification.

Currently, the most commonly used transfer vector for introducing foreign genes into AcNPV is pAc373. Many other vectors, known to those of skill in the art, have also been designed. These include, for example, pVL985 (which alters the polyhedrin start codon from ATG to ATT, and which introduces a BamHI cloning site 32 basepairs downstream from the ATT; see Luckow and Summers, *Virology* (1989) *17*:31.

The plasmid usually also contains the polyhedrin polyadenylation signal (Miller et al. (1988) *Ann. Rev. Microbiol.*, *42*:177) and a prokaryotic ampicillin-resistance (*amp*) gene and origin of replication for selection and propagation in *E. coli*.

Baculovirus transfer vectors usually contain a baculovirus promoter. A baculovirus promoter is any DNA sequence capable of binding a baculovirus RNA polymerase and initiating the downstream (5' to 3') transcription of a coding sequence (e.g., structural gene) into mRNA. A promoter will have a transcription initiation region which is usually placed proximal to the 5' end of the coding sequence. This transcription initiation region usually includes an RNA polymerase binding site and a transcription initiation site. A baculovirus transfer vector may also have a second domain called an enhancer, which, if present, is usually distal to the structural gene. Expression may be either regulated or constitutive.

Structural genes, abundantly transcribed at late times in a viral infection cycle, provide particularly useful promoter sequences. Examples include sequences derived from the gene encoding the viral polyhedron protein, Friesen et al., (1986) "The Regulation of Baculovirus Gene Expression," in: *The Molecular Biology of Baculoviruses* (ed. Walter Doerfler); EPO Publ. Nos. 127 839 and 155 476; and the gene encoding the p10 protein, Vlak et al., (1988), *J. Gen. Virol. 69*:765.

DNA encoding suitable signal sequences can be derived from genes for secreted insect or baculovirus proteins, such as the baculovirus polyhedrin gene (Carbonell et al. (1988) *Gene, 73:*409). Alternatively, since the signals for mammalian cell posttranslational modifications (such as signal peptide cleavage, proteolytic cleavage, and phosphorylation) appear to be recognized by insect cells, and the signals required for secretion and nuclear accumulation also appear to be conserved between the invertebrate cells and vertebrate cells, leaders of non-insect origin, such as those derived from genes encoding human (alpha) $\alpha$-interferon, Maeda et al., (1985), *Nature 315*:592; human gastrin-releasing peptide, Lebacq-Verheyden et al., (1988), *Molec. Cell. Biol. 8*:3129; human IL-2, Smith et al., (1985) *Proc. Nat'l Acad. Sci. USA, 82*:8404; mouse IL-3, (Miyajima et al., (1987) *Gene 58*:273; and human glucocerebrosidase, Martin et al. (1988) *DNA,* 7:99, can also be used to provide for secretion in insects.

A recombinant polypeptide or polyprotein may be expressed intracellularly or, if it is expressed with the proper regulatory sequences, it can be secreted. Good intracellular expression of nonfused foreign proteins usually requires heterologous genes that ideally have a short leader sequence containing suitable translation initiation signals preceding an ATG start signal. If desired, methionine at the N-terminus may be cleaved from the mature protein by *in vitro* incubation with cyanogen bromide.

Alternatively, recombinant polyproteins or proteins which are not naturally secreted can be secreted from the insect cell by creating chimeric DNA molecules that encode a fusion protein comprised of a leader sequence fragment that provides for secretion of the foreign protein in insects. The leader sequence fragment usually encodes a signal peptide comprised of hydrophobic amino acids which direct the translocation of the protein into the endoplasmic reticulum.

After insertion of the DNA sequence and/or the gene encoding the expression product precursor of the protein, an insect cell host is co-transformed with the heterologous DNA of the transfer vector and the genomic DNA of wild type baculovirus -- usually by cotransfection. The promoter and transcription termination sequence of the construct will usually comprise a 2-5kb section of the baculovirus genome. Methods for introducing heterologous DNA into the desired site in the baculovirus virus are known in the art. (See Summers and Smith *supra*; Ju et al. (1987); Smith et al., *Mol. Cell. Biol.* (1983) 3:2156; and Luckow and Summers (1989)). For example, the insertion can be into a gene such as the polyhedrin gene, by homologous double crossover recombination; insertion can also be into a restriction enzyme site engineered into the desired baculovirus gene. Miller et al., (1989), *Bioessays* 4:91. The DNA sequence, when cloned in place of the polyhedrin gene in the expression vector, is flanked both 5' and 3' by polyhedrin-specific sequences and is positioned downstream of the polyhedrin promoter.

The newly formed baculovirus expression vector is subsequently packaged into an infectious recombinant baculovirus. Homologous recombination occurs at low frequency (between about 1% and about 5%); thus, the majority of the virus produced after cotransfection is still wild-type virus. Therefore, a method is necessary to identify recombinant viruses. An advantage of the expression system is a visual screen allowing recombinant viruses to be distinguished. The polyhedrin protein, which is produced by the native virus, is produced at very high levels in the nuclei of infected cells at late times after viral infection. Accumulated polyhedrin protein forms occlusion bodies that also contain embedded particles. These occlusion bodies, up to 15 μm in size, are highly refractile, giving them a bright shiny appearance that is readily visualized under the light microscope. Cells infected with recombinant viruses lack occlusion bodies. To distinguish recombinant virus from wild-type virus, the transfection supernatant is plaqued onto a monolayer of insect cells by techniques known to those skilled in the art. Namely, the plaques are screened under the light microscope for the presence (indicative of wild-type virus) or absence (indicative of recombinant virus) of occlusion bodies. *Current Protocols in Microbiology* Vol. 2 (Ausubel et al. eds) at 16.8 (Supp. 10, 1990); Summers and Smith, *supra*; Miller et al. (1989).

Recombinant baculovirus expression vectors have been developed for infection into several insect cells. For example, recombinant baculoviruses have been developed for, *inter alia*: *Aedes aegypti* , *Autographa californica, Bombyx mori, Drosophila melanogaster, Spodoptera frugiperda*, and *Trichoplusia ni* (PCT Pub. No. WO 89/046699; Carbonell et al., (1985) *J. Virol. 56*:153; Wright (1986) *Nature 321*:718; Smith et al., (1983) *Mol. Cell. Biol. 3*:2156; and see generally, *Fraser, et al*. (1989) *In Vitro Cell. Dev. Biol. 25*:225).

Cells and cell culture media are commercially available for both direct and fusion expression of heterologous polypeptides in a baculovirus/expression system; cell culture technology is generally known to those skilled in the art. *See,* e.g., Summers and Smith *supra*.

The modified insect cells may then be grown in an appropriate nutrient medium, which allows for stable maintenance of the plasmid(s) present in the modified insect host. Where the expression product gene is under inducible control, the host may be grown to high density, and expression induced. Alternatively, where expression is constitutive, the product will be continuously expressed into the medium and the nutrient medium must be continuously circulated, while removing the product of interest and augmenting depleted nutrients. The product may be purified by such techniques as chromatography, e.g., HPLC, affinity chromatography, ion exchange chromatography, etc.; electrophoresis; density gradient centrifugation; solvent extraction, or the like. As appropriate, the product may be further purified, as required, so as to remove substantially any insect proteins which are also secreted in the medium or result from lysis of insect cells, so as to provide a product which is at least substantially free of host debris, e.g., proteins, lipids and polysaccharides.

In order to obtain protein expression, recombinant host cells derived from the transformants are incubated under conditions which allow expression of the recombinant protein encoding sequence. These conditions will vary, dependent upon the host cell selected. However, the conditions are readily ascertainable to those of ordinary skill in the art, based upon what is known in the art.

iv. Bacterial Systems

Bacterial expression techniques are known in the art. A bacterial promoter is any DNA sequence capable of binding bacterial RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site and a transcription initiation site. A bacterial promoter may also have a second domain called an operator, that may overlap an adjacent RNA polymerase binding site at which RNA synthesis begins. The operator permits negative regulated (inducible) transcription, as a gene repressor protein may bind the operator and thereby inhibit transcription of a specific gene. Constitutive expression may occur in the absence of negative regulatory elements, such as the operator. In addition, positive regulation may be achieved by a gene activator protein binding sequence, which, if present is usually proximal (5') to the RNA polymerase binding sequence. An example of a gene activator protein is the catabolite activator protein (CAP), which helps initiate transcription of the lac operon in Escherichia coli *(E. coli*) (Raibaud *et al*. (1984) *Annu. Rev. Genet. 18*:173). Regulated expression may therefore be either positive or negative, thereby either enhancing or reducing transcription.

Sequences encoding metabolic pathway enzymes provide particularly useful promoter sequences. Examples include promoter sequences derived from sugar metabolizing enzymes, such as galactose, lactose (*lac*) (Chang *et al*. (1977) *Nature 198*:1056), and maltose. Additional examples include promoter sequences derived from biosynthetic enzymes such as tryptophan (*trp*) (Goeddel *et al*. (1980) *Nuc. Acids Res. 8*:4057; Yelverton *et al*. (1981) *Nucl. Acids Res.* 9:731; U.S. Patent 4,738,921; EPO Publ. Nos. 036 776 and 121 775). The beta-lactamase (*bla*) promoter system (Weissmann (1981) "The cloning of interferon and other mistakes." In *Interferon 3* (ed. I. Grosser)), bacteriophage lambda PL (Shimatake *et al*. (1981) *Nature 292*:128) and T5 (U.S. Patent 4,689,406) promoter systems also provide useful promoter sequences.

In addition, synthetic promoters which do not occur in nature also function as bacterial promoters. For example, transcription activation sequences of one bacterial or bacteriophage promoter may be joined with the operon sequences of another bacterial or bacteriophage promoter, creating a synthetic hybrid promoter (U.S. Patent 4,551,433). For example, the *tac* promoter is a hybrid *trp-lac* promoter comprised of both *trp* promoter and *lac* operon sequences that is regulated by the *lac* repressor (Amann *et al*. (1983) *Gene 25*:167; de Boer *et al*. (1983) *Proc. Natl. Acad. Sci. 80*: 21). Furthermore, a bacterial promoter can include naturally occurring promoters of non-bacterial origin that have the ability to bind bacterial RNA polymerase and initiate transcription. A naturally occurring promoter of non-bacterial origin can also be coupled with a compatible RNA polymerase to produce high levels of expression of some genes in prokaryotes. The bacteriophage T7 RNA polymerase/promoter system is an example of a coupled promoter system *(Studier et al*. (1986) *J. Mol. Biol. 189*:113; Tabor *et al*. (1985) *Proc Natl. Acad. Sci. 82*:1074). In addition, a hybrid promoter can also be comprised of a bacteriophage promoter and an *E. coli* operator region (EPO Publ. No. 267 851).

In addition to a functioning promoter sequence, an efficient ribosome binding site is also useful for the expression of foreign genes in prokaryotes. In *E. coli*, the ribosome binding site is called the Shine-Dalgarno (SD) sequence and includes an initiation codon (ATG) and a sequence 3-9 nucleotides in length located 3-11 nucleotides upstream of the initiation codon (Shine *et al*. (1975) *Nature 254*:34). The SD sequence is thought to promote binding of mRNA to the ribosome by the pairing of bases between the SD sequence and the 3' end of *E. coli* 16S rRNA (Steitz *et al*. (1979) "Genetic signals and nucleotide sequences in messenger RNA." In *Biological Regulation and Development: Gene Expression* (ed. R.F. Goldberger)). To express eukaryotic genes and prokaryotic genes with weak ribosome-binding site, it is often necessary to optimize the distance between the SD sequence and the ATG of the eukaryotic gene (Sambrook *et al*. (1989) "Expression of cloned genes in Escherichia coli." In *Molecular Cloning: A Laboratory Manual*).

A DNA molecule may be expressed intracellularly. A promoter sequence may be directly linked with the DNA molecule, in which case the first amino acid at the N-terminus will always be a methionine, which is encoded by the ATG start codon. If desired, methionine at the N-terminus may be cleaved from the protein by *in vitro* incubation with cyanogen bromide or by either *in vivo* or *in vitro* incubation with a bacterial methionine N-terminal peptidase (EPO Publ. No. 219 237).

Fusion proteins provide an alternative to direct expression. Usually, a DNA sequence encoding the N-terminal portion of an endogenous bacterial protein, or other stable protein, is fused to the 5' end of heterologous coding sequences. Upon expression, this construct will provide a fusion of the two amino acid sequences. For example, the bacteriophage lambda cell gene can be linked at the 5' terminus of a foreign gene and expressed in bacteria. The resulting fusion protein preferably retains a site for a processing enzyme (factor Xa) to cleave the bacteriophage protein from the foreign gene (Nagai *et al*. (1984) *Nature 309:810)*. Fusion proteins can also be made with sequences from the *lacZ* (Jia *et al*. (1987) *Gene 60*:197), *trpE* (Allen et al. (1987) J. Biotechnol. 5:93; Makoff *et al*. (1989) *J. Gen. Microbiol. 135*:11), and *Chey* (EPO Publ. No. 324 647) genes. The DNA sequence at the junction of the two amino acid sequences may or may not encode a cleavable site. Another example is a ubiquitin fusion protein. Such a fusion protein is made with the ubiquitin region that preferably retains a site for a processing enzyme (e.g. ubiquitin specific processing-protease) to cleave the ubiquitin from the foreign protein. Through this method, native foreign protein can be isolated (Miller *et al*. (1989) *Bio*/*Technology* 7:698).

Alternatively, foreign proteins can also be secreted from the cell by creating chimeric DNA molecules that encode a fusion protein comprised of a signal peptide sequence fragment that provides for secretion of the foreign protein in bacteria (U.S. Patent 4,336,336). The signal sequence fragment usually encodes a signal peptide comprised of hydrophobic amino acids which direct the secretion of the protein from the cell. The protein is either secreted into the growth media (gram-positive bacteria) or into the periplasmic space, located between the inner and outer membrane of the cell (gram-negative bacteria). Preferably there are processing sites, which can be cleaved either *in vivo* or *in vitro* encoded between the signal peptide fragment and the foreign gene.

DNA encoding suitable signal sequences can be derived from genes for secreted bacterial proteins, such as the *E. coli* outer membrane protein gene (*ompA*) (Masui *et al*. (1983), in: *Experimental Manipulation of Gene Expression;* Ghrayeb *et al*. (1984) *EMBO J.* 3:2437) and the *E. coli* alkaline phosphatase signal sequence (*phoA*) (Oka *et al*. (1985) *Proc. Natl. Acad. Sci. 82*:7212). As an additional example, the signal sequence of the alpha-amylase gene from various Bacillus strains can be used to secrete heterologous proteins from *B. subtilis* (Palva *et al*. (1982) *Proc. Natl. Acad. Sci. USA* 79:5582; EPO Publ. No. 244 042).

Usually, transcription termination sequences recognized by bacteria are regulatory regions located 3' to the translation stop codon, and thus together with the promoter flank the coding sequence. These sequences direct the transcription of an mRNA which can be translated into the polypeptide encoded by the DNA. Transcription termination sequences frequently include DNA sequences of about 50 nucleotides capable of forming stem loop structures that aid in terminating transcription. Examples include transcription termination sequences derived from genes with strong promoters, such as the *trp* gene in *E. coli* as well as other biosynthetic genes.

Usually, the above described components, comprising a promoter, signal sequence (if desired), coding sequence of interest, and transcription termination sequence, are put together into expression constructs. Expression constructs are often maintained in a replicon, such as an extrachromosomal element (e.g., plasmids) capable of stable maintenance in a host, such as bacteria. The replicon will have a replication system, thus allowing it to be maintained in a prokaryotic host either for expression or for cloning and amplification. In addition, a replicon may be either a high or low copy number plasmid. A high copy number plasmid will generally have a copy number ranging from about 5 to about 200, and usually about 10 to about 150. A host containing a high copy number plasmid will preferably contain at least about 10, and more preferably at least about 20 plasmids. Either a high or low copy number vector may be selected, depending upon the effect of the vector and the foreign protein on the host.

Alternatively, the expression constructs can be integrated into the bacterial genome with an integrating vector. Integrating vectors usually contain at least one sequence homologous to the bacterial chromosome that allows the vector to integrate. Integrations appear to result from recombinations between homologous DNA in the vector and the bacterial chromosome. For example, integrating vectors constructed with DNA from various Bacillus strains integrate into the Bacillus chromosome (EPO Publ. No. 127 328). Integrating vectors may also be comprised of bacteriophage or transposon sequences.

Usually, extrachromosomal and integrating expression constructs may contain selectable markers to allow for the selection of bacterial strains that have been transformed. Selectable markers can be expressed in the bacterial host and may include genes which render bacteria resistant to drugs such as ampicillin, chloramphenicol, erythromycin, kanamycin (neomycin), and tetracycline (Davies *et al*. (1978) *Annu. Rev. Microbiol. 32*:469). Selectable markers may also include biosynthetic genes, such as those in the histidine, tryptophan, and leucine biosynthetic pathways.

Alternatively, some of the above described components can be put together in transformation vectors. Transformation vectors are usually comprised of a selectable market that is either maintained in a replicon or developed into an integrating vector, as described above.

Expression and transformation vectors, either extra-chromosomal replicons or integrating vectors, have been developed for transformation into many bacteria. For example, expression vectors have been developed for, *inter alia*, the following bacteria: Bacillus subtilis (Palva *et al*. (1982) *Proc. Natl. Acad. Sci. USA* 79:5582; EPO Publ. Nos. 036 259 and 063 953; PCT Publ. No. WO 84/04541), Escherichia coli (Shimatake *et al*. (1981) *Nature* 292:128; Amann *et al*. (1985) *Gene 40*:183; Studier *et al*. (1986) *J. Mol. Biol*. 189:113; EPO Publ. Nos. 036 776, 136 829 and 136 907), Streptococcus cremoris (Powell *et al*. (1988) *Appl. Environ. Microbiol*. *54*:655); Streptococcus lividans (Powell *et al*. (1988) *Appl. Environ. Microbiol*. *54*:655), Streptomyces lividans (U.S. Patent 4,745,056).

Methods of introducing exogenous DNA into bacterial hosts are well-known in the art, and usually include either the transformation of bacteria treated with $CaCl_2$ or other agents, such as divalent cations and DMSO. DNA can also be introduced into bacterial cells by electroporation. Transformation procedures usually vary with the bacterial species to be transformed. (See e.g., use of *Bacillus:* Masson *et al*. (1989) *FEMS Microbiol. Lett.* 60:273; Palva *et al*. (1982) *Proc. Natl. Acad. Sci. USA 79*:5582; EPO Publ. Nos. 036 259 and 063 953; PCT Publ. No. WO 84/04541; use of *Campylobacter*: Miller *et al*. (1988) *Proc. Natl. Acad. Sci. 85*:856; and Wang *et al*. (1990) *J. Bacteriol. 172*:949; use of *Escherichia coli: Cohen et al*. (1973) *Proc. Natl. Acad. Sci. 69:*2110; Dower *et al*. (1988) *Nucleic Acids Res. 16*:6127; Kushner (1978) "An improved method for transformation of *Escherichia coli* with ColE1-derived plasmids. In *Genetic Engineering: Proceedings of the International Symposium on Genetic Engineering* (eds. H.W. Boyer and S. Nicosia); Mandel *et al*. (1970) *J. Mol. Biol. 53*:159; Taketo (1988) *Biochim. Biophys. Acta 949*:318; use of *Lactobacillus: Chassy et al*. (1987) *FEMS Microbiol. Lett. 44*:173; use of *Pseudomonas:* Fiedler *et al*. (1988) *Anal. Biochem 170:38;* use of *Staphylococcus:* Augustin *et al*. (1990) *FEMS Microbiol. Lett. 66*:203; use of *Streptococcus:* Barany *et al*. (1980) *J. Bacteriol. 144*:698; Harlander (1987) "Transformation of Streptococcus lactis by electroporation, in: *Streptococcal Genetics* (ed. J. Ferretti and R. Curtiss III); Perry *et al*. (1981) *Infect. Immun. 32*:1295; *Powell et al*. (1988) *Appl. Environ. Microbiol*. *54*:655; Somkuti *et al*. (1987) *Proc. 4th Evr. Cong. Biotechnology 1*:412.

v. Yeast Expression

Yeast expression systems are also known to one of ordinary skill in the art. A yeast promoter is any DNA sequence capable of binding yeast RNA polymerase and initiating the downstream (3') transcription of a coding sequence (e.g. structural gene) into mRNA. A promoter will have a transcription initiation region which is usually placed proximal to the 5' end of the coding sequence. This transcription initiation region usually includes an RNA polymerase binding site (the "TATA Box") and a transcription initiation site. A yeast promoter may also have a second domain called an upstream activator sequence (UAS), which, if present, is usually distal to the structural gene. The UAS permits regulated (inducible) expression. Constitutive expression occurs in the absence of a UAS. Regulated expression may be either positive or negative, thereby either enhancing or reducing transcription.

Yeast is a fermenting organism with an active metabolic pathway, therefore sequences encoding enzymes in the metabolic pathway provide particularly useful promoter sequences. Examples include alcohol dehydrogenase (ADH) (EPO Publ. No. 284 044), enolase, glucokinase, glucose-6-phosphate isomerase, glyceraldehyde-3-phosphate-dehydrogenase (GAP or GAPDH), hexokinase, phosphofructokinase, 3-phosphoglycerate mutase, and pyruvate kinase (PyK) (EPO Publ. No. 329 203). The yeast *PHO5* gene, encoding acid phosphatase, also provides useful promoter sequences (Myanohara *et al*. (1983) *Proc. Natl. Acad. Sci. USA 80*:1).

In addition, synthetic promoters which do not occur in nature also function as yeast promoters. For example, UAS sequences of one yeast promoter may be joined with the transcription activation region of another yeast promoter, creating a synthetic hybrid promoter. Examples of such hybrid promoters include the ADH regulatory sequence linked to the GAP transcription activation region (U.S. Patent Nos. 4,876,197 and 4,880,734). Other examples of hybrid promoters include promoters which consist of the regulatory sequences of either the *ADH2, GAL4, GAL10*, OR *PHO5* genes, combined with the transcriptional activation region of a glycolytic enzyme gene such as GAP or PyK (EPO Publ. No. 164 556). Furthermore, a yeast promoter can include naturally occurring promoters of non-yeast origin that have the ability to bind yeast RNA polymerase and initiate transcription. Examples of such promoters include, *inter alia*, (Cohen *et al*. (1980) *Proc. Natl. Acad. Sci. USA 77:1078;* Henikoff *et al*. (1981) *Nature 283*:835; Hollenberg *et al*. (1981) *Curr. Topics Microbiol. Immunol*. *96*:119; Hollenberg *et al*. (1979) "The Expression of Bacterial Antibiotic Resistance Genes in the Yeast Saccharomyces cerevisiae," in: *Plasmids of Medical, Environmental and Commercial Importance* (eds. K.N. Timmis and A. Puhler); Mercerau-*Puigalon et al*. (1980) *Gene 11*:163; *Panthier et al*. (1980) *Curr. Genet. 2*:109;).

A DNA molecule may be expressed intracellularly in yeast. A promoter sequence may be directly linked with the DNA molecule, in which case the first amino acid at the N-terminus of the recombinant protein will always be a methionine, which is encoded by the ATG start codon. If desired, methionine at the N-terminus may be cleaved from the protein by *in vitro* incubation with cyanogen bromide.

Fusion proteins provide an alternative for yeast expression systems, as well as in mammalian, plant, baculovirus, and bacterial expression systems. Usually, a DNA sequence encoding the N-terminal portion of an endogenous yeast protein, or other stable protein, is fused to the 5' end of heterologous coding sequences. Upon expression, this construct will provide a fusion of the two amino acid sequences. For example, the yeast or human superoxide dismutase (SOD) gene, can be linked at the 5' terminus of a foreign gene and expressed in yeast. The DNA sequence at the junction of the two amino acid sequences may or may not encode a cleavable site. See e.g., EPO Publ. No. 196056. Another example is a ubiquitin fusion protein. Such a fusion protein is made with the ubiquitin region that preferably retains a site for a processing enzyme (e.g. ubiquitin-specific processing protease) to cleave the ubiquitin from the foreign protein. Through this method, therefore, native foreign protein can be isolated (e.g., W088/024066).

Alternatively, foreign proteins can also be secreted from the cell into the growth media by creating chimeric DNA molecules that encode a fusion protein comprised of a leader sequence fragment that provide for secretion in yeast of the foreign protein. Preferably, there are processing sites encoded between the leader fragment and the foreign gene that can be cleaved either *in vivo* or *in vitro.* The leader sequence fragment usually encodes a signal peptide comprised of hydrophobic amino acids which direct the secretion of the protein from the cell.

DNA encoding suitable signal sequences can be derived from genes for secreted yeast proteins, such as the yeast invertase gene (EPO Publ. No. 012 873; JPO Publ. No. 62:096,086) and the A-factor gene (U.S. Patent 4,588,684). Alternatively, leaders of non-yeast origin, such as an interferon leader, exist that also provide for secretion in yeast (EPO Publ. No. 060 057).

A preferred class of secretion leaders are those that employ a fragment of the yeast alpha-factor gene, which contains both a "pre" signal sequence, and a "pro" region. The types of alpha-factor fragments that can be employed include the full-length pre-pro alpha factor leader (about 83 amino acid residues) as well as truncated alpha-factor leaders (usually about 25 to about 50 amino acid residues) (U.S. Patent Nos. 4,546,083 and 4,870,008; EPO Publ. No. 324 274). Additional leaders employing an alpha-factor leader fragment that provides for secretion include hybrid alpha-factor leaders made with a presequence of a first yeast, but a pro-region from a second yeast alpha factor. (See e.g., PCT Publ. No. WO 89/02463.)

Usually, transcription termination sequences recognized by yeast are regulatory regions located 3' to the translation stop codon, and thus together with the promoter flank the coding sequence. These sequences direct the transcription of an mRNA which can be translated into the polypeptide encoded by the DNA. Examples of transcription terminator sequence and other yeast-recognized termination sequences, such as those coding for glycolytic enzymes.

Usually, the above described components, comprising a promoter, leader (if desired), coding sequence of interest, and transcription termination sequence, are put together into expression constructs. Expression constructs are often maintained in a replicon, such as an extrachromosomal element (e.g., plasmids) capable of stable maintenance in a host, such as yeast or bacteria. The replicon may have two replication systems, thus allowing it to be maintained, for example, in yeast for expression and in a prokaryotic host for cloning and amplification. Examples of such yeast-bacteria shuttle vectors include YEp24 (Botstein *et al*. (1979) *Gene 8*:17-24), pCl/1 (Brake *et al*. (1984) *Proc. Natl. Acad. Sci USA 81*:4642-4646), and YRp17 (Stinchcomb *et al*. (1982) *J. Mol. Biol. 158*:157). In addition, a replicon may be either a high or low copy number plasmid. A high copy number plasmid will generally have a copy number ranging from about 5 to about 200, and usually about 10 to about 150. A host containing a high copy number plasmid will preferably have at least about 10, and more preferably at least about 20. Enter a high or low copy number vector may be selected, depending upon the effect of the vector and the foreign protein on the host. See e.g., Brake *et al*., *supra*.

Alternatively, the expression constructs can be integrated into the yeast genome with an integrating vector. Integrating vectors usually contain at least one sequence homologous to a yeast chromosome that allows the vector to integrate, and preferably contain two homologous sequences flanking the expression construct. Integrations appear to result from recombinations between homologous DNA in the vector and the yeast chromosome (Orr-*Weaver et al*. (1983) *Methods in Enzymol. 101*:228-245). An integrating vector may be directed to a specific locus in yeast by selecting the appropriate homologous sequence for inclusion in the vector. See Orr-Weaver *et al*., *supra*. One or more expression construct may integrate, possibly affecting levels of recombinant protein produced (Rine *et al*. (1983) *Proc. Natl. Acad. Sci. USA 80*:6750). The chromosomal sequences included in the vector can occur either as a single segment in the vector, which results in the integration of the entire vector, or two segments homologous to adjacent segments in the chromosome and flanking the expression construct in the vector, which can result in the stable integration of only the expression construct.

Usually, extrachromosomal and integrating expression constructs may contain selectable markers to allow for the selection of yeast strains that have been transformed. Selectable markers may include biosynthetic genes that can be expressed in the yeast host, such as *ADE2*, *HIS4, LEU2, TRP1*, and *ALG7,* and the G418 resistance gene, which confer resistance in yeast cells to tunicamycin and G418, respectively. In addition, a suitable selectable marker may also provide yeast with the ability to grow in the presence of toxic compounds, such as metal. For example, the presence of *CUP1* allows yeast to grow in the presence of copper ions (Butt *et al*. (1987) *Microbiol, Rev. 51*:351).

Alternatively, some of the above described components can be put together into transformation vectors. Transformation vectors are usually comprised of a selectable marker that is either maintained in a replicon or developed into an integrating vector, as described above.

Expression and transformation vectors, either extrachromosomal replicons or integrating vectors, have been developed for transformation into many yeasts. For example, expression vectors and methods of introducing exogenous DNA into yeast hosts have been developed for, *inter alia*, the following yeasts: *Candida albicans* (Kurtz, *et al.* (1986) *Mol. Cell. Biol*. *6*:142); *Candida maltosa* (Kunze, *et al*. (1985) *J. Basic Microbiol. 25*:141); *Hansenula polymorpha* (Gleeson, *et al*. (1986) *J. Gen. Microbiol. 132*:3459; Roggenkamp *et al*. (1986) *Mol. Gen. Genet.* 202:302); *Kluyveromyces fragilis* (Das, *et al*. (1984) *J. Bacteriol. 158*:1165); *Kluyveromyces lactis* (De Louvencourt *et al*. (1983) *J. Bacteriol. 154:737;* Van den *Berg et al*. (1990) *Bio/Technology 8*:135); *Pichia guillerimondii* (Kunze *et al.* (1985) *J. Basic Microbiol. 25*:141); *Pichia pastoris* (Cregg, *et al.* (1985) *Mol. Cell. Biol*. *5*:3376; U.S. Patent Nos. 4,837,148 and 4,929,555); *Saccharomyces cerevisiae* (Hinnen *et al*. (1978) *Proc. Natl. Acad. Sci. USA* 75:1929; Ito *et al*. (1983) *J. Bacteriol. 153*:163); *Schizosaccharomyces pombe* (Beach and Nurse (1981) *Nature 300*:706); and *Yarrowia lipolytica* (Davidow, *et al*. (1985) *Curr. Genet. 10*:380471 Gaillardin, *et al*. (1985) *Curr. Genet. 10*:49).

Methods of introducing exogenous DNA into yeast hosts are well-known in the art, and usually include either the transformation of spheroplasts or of intact yeast cells treated with alkali cations. Transformation procedures usually vary with the yeast species to be transformed. See e.g., [Kurtz *et al*. (1986) *Mol. Cell. Biol. 6*:142; Kunze *et al*. (1985) *J. Basic Microbiol. 25*:141; Candida]; [Gleeson *et al*. (1986) *J. Gen. Microbiol. 132*:3459; Roggenkamp *et al*. (1986) *Mol. Gen. Genet. 202*:302; Hansenula]; [Das *et al*. (1984) *J. Bacteriol. 158*:1165; De Louvencourt *et al*. (1983) *J. Bacteriol. 154*:1165; Van den Berg *et al*. (1990) *Bio/Technology 8:135;* Kluyveromyces]; [Cregg *et al*. (1985) *Mol. Cell. Biol. 5*:3376; Kunze *et al*. (1985) *J. Basic Microbiol. 25*:141; U.S. Patent Nos. 4,837,148 and 4,929,555; Pichia]; *[Hinnen et al*. (1978) *Proc. Natl. Acad. Sci. USA 75*;1929; Ito *et al*. (1983) *J. Bacteriol. 153*:163 Saccharomyces]; [Beach and Nurse (1981) *Nature* 300:706; Schizosaccharomyces]; [Davidow *et al*. (1985) *Curr. Genet. 10*:39; Gaillardin *et al*. (1985) *Curr. Genet. 10*:49; Yarrowia].

Definitions

A composition containing X is "substantially free of" Y when at least 85% by weight of the total X+Y in the composition is X. Preferably, X comprises at least about 90% by weight of the total of X+Y in the composition, more preferably at least about 95% or even 99% by weight.

The term "heterologous" refers to two biological components that are not found together in nature. The components may be host cells, genes, or regulatory regions, such as promoters. Although the heterologous components are not found together in nature, they can function together, as when a promoter heterologous to a gene is operably linked to the gene. Another example is where a Neisserial sequence is heterologous to a mouse host cell.

An "origin of replication" is a polynucleotide sequence that initiates and regulates replication of polynucleotides, such as an expression vector. The origin of replication behaves as an autonomous unit of polynucleotide replication within a cell, capable of replication under its own control. An origin of replication may be needed for a vector to replicate in a particular host cell. With certain origins of replication, an expression vector can be reproduced at a high copy number in the presence of the appropriate proteins within the cell. Examples of origins are the autonomously replicating sequences, which are effective in yeast; and the viral T-antigen, effective in COS-7 cells.

A "mutant" sequence is defined as a DNA, RNA or amino acid sequence differing from but having homology with the native or disclosed sequence. Depending on the particular sequence, the degree of homology between the native or disclosed sequence and the mutant sequence is preferably greater than 50% (e.g., 60%, 70%, 80%, 90%, 95%, 99% or more) which is calculated as described above. As used herein, an "allelic variant" of a nucleic acid molecule, or region, for which nucleic acid sequence is provided herein is a nucleic acid molecule, or region, that occurs at essentially the same locus in the genome of another or second isolate, and that, due to natural variation caused by, for example, mutation or recombination, has a similar but not identical nucleic acid sequence. A coding region allelic variant typically encodes a protein having similar activity to that of the protein encoded by the gene to which it is being compared. An allelic variant can also comprise an alteration in the 5' or 3' untranslated regions of the gene, such as in regulatory control regions. (see, for example, U.S. Patent 5,753,235). Antibodies

As used herein, the term "antibody" refers to a polypeptide or group of polypeptides composed of at least one antibody combining site. An "antibody combining site" is the three-dimensional binding space with an internal surface shape and charge distribution complementary to the features of an epitope of an antigen, which allows a binding of the antibody with the antigen. "Antibody" includes, for example, vertebrate antibodies, hybrid antibodies, chimeric antibodies, humanized antibodies, altered antibodies, univalent antibodies, Fab proteins, and single domain antibodies.

Antibodies against the proteins of the invention are useful for affinity chromatography, immunoassays, and distinguishing/identifying *Neisseria* MenB proteins. Antibodies elicited against the proteins of the present invention bind to antigenic polypeptides or proteins or protein fragments that are present and specifically associated with strains of *Neisseria meningitidis* MenB. In some instances, these antigens may be associated with specific strains, such as those antigens specific for the MenB strains. The antibodies of the invention may be immobilized to a matrix and utilized in an immunoassay or on an affinity chromatography column, to enable the detection and/or separation of polypeptides, proteins or protein fragments or cells comprising such polypeptides, proteins or protein fragments. Alternatively, such polypeptides, proteins or protein fragments may be immobilized so as to detect antibodies bindably specific thereto.

Antibodies to the proteins of the invention, both polyclonal and monoclonal, may be prepared by conventional methods. In general, the protein is first used to immunize a suitable animal, preferably a mouse, rat, rabbit or goat. Rabbits and goats are preferred for the preparation of polyclonal sera due to the volume of serum obtainable, and the availability of labeled anti-rabbit and anti-goat antibodies. Immunization is generally performed by mixing or emulsifying the protein in saline, preferably in an adjuvant such as Freund's complete adjuvant, and injecting the mixture or emulsion parenterally (generally subcutaneously or intramuscularly). A dose of 50-200 μg/injection is typically sufficient. Immunization is generally boosted 2-6 weeks later with one or more injections of the protein in saline, preferably using Freund's incomplete adjuvant. One may alternatively generate antibodies by in vitro immunization using methods known in the art, which for the purposes of this invention is considered equivalent to *in vivo* immunization. Polyclonal antisera is obtained by bleeding the immunized animal into a glass or plastic container, incubating the blood at 25°C for one hour, followed by incubating at 4°C for 2-18 hours. The serum is recovered by centrifugation (e.g., 1,000g for 10 minutes). About 20-50 ml per bleed may be obtained from rabbits.

Monoclonal antibodies are prepared using the standard method of Kohler & Milstein (*Nature* (1975) 256: 495-96), or a modification thereof. Typically, a mouse or rat is immunized as described above. However, rather than bleeding the animal to extract serum, the spleen (and optionally several large lymph nodes) is removed and dissociated into single cells. If desired, the spleen cells may be screened (after removal of nonspecifically adherent cells) by applying a cell suspension to a plate or well coated with the protein antigen. B-cells that express membrane-bound immunoglobulin specific for the antigen bind to the plate, and are not rinsed away with the rest of the suspension. Resulting B-cells, or all dissociated spleen cells, are then induced to fuse with myeloma cells to form hybridomas, and are cultured in a selective medium (e.g., hypoxanthine, aminopterin, thymidine medium, "HAT"). The resulting hybridomas are plated by limiting dilution, and are assayed for the production of antibodies which bind specifically to the immunizing antigen (and which do not bind to unrelated antigens). The selected MAb-secreting hybridomas are then cultured either *in vitro* (e.g., in tissue culture bottles or hollow fiber reactors), or *in vivo* (as ascites in mice).

If desired, the antibodies (whether polyclonal or monoclonal) may be labeled using conventional techniques. Suitable labels include fluorophores, chromophores, radioactive atoms (particularly $^{32}$P and $^{125}$I), electron-dense reagents, enzymes, and ligands having specific binding partners. Enzymes are typically detected by their activity. For example, horseradish peroxidase is usually detected by its ability to convert 3,3',5,5'-tetramethylbenzidine (TMB) to a blue pigment, quantifiable with a spectrophotometer. "Specific binding partner" refers to a protein capable of binding a ligand molecule with high specificity, as for example in the case of an antigen and a monoclonal antibody specific therefor. Other specific binding partners include biotin and avidin or streptavidin, IgG and protein A, and the numerous receptor-ligand couples known in the art. It should be understood that the above description is not meant to categorize the various labels into distinct classes, as the same label may serve in several different modes. For example, $^{125}I$ may serve as a radioactive label or as an electron-dense reagent. HRP may serve as enzyme or as antigen for a MAb. Further, one may combine various labels for desired effect. For example, MAbs and avidin also require labels in the practice of this invention: thus, one might label a MAb with biotin, and detect its presence with avidin labeled with $^{125}I$, or with an anti-biotin MAb labeled with HRP. Other permutations and possibilities will be readily apparent to those of ordinary skill in the art, and are considered as equivalents within the scope of the instant invention.

Antigens, immunogens, polypeptides, proteins or protein fragments of the present invention elicit formation of specific binding partner antibodies. These antigens, immunogens, polypeptides, proteins or protein fragments of the present invention comprise immunogenic compositions of the present invention. Such immunogenic compositions may further comprise or include adjuvants, carriers, or other compositions that promote or enhance or stabilize the antigens, polypeptides, proteins or protein fragments of the present invention. Such adjuvants and carriers will be readily apparent to those of ordinary skill in the art.

Pharmaceutical Compositions

Pharmaceutical compositions can include either polypeptides, antibodies, or nucleic acid of the invention. The pharmaceutical compositions will comprise a therapeutically effective amount of either polypeptides, antibodies, or polynucleotides of the claimed invention.

The term "therapeutically effective amount" as used herein refers to an amount of a therapeutic agent to treat, ameliorate, or prevent a desired disease or condition, or to exhibit a detectable therapeutic or preventative effect. The effect can be detected by, for example, chemical markers or antigen levels. Therapeutic effects also include reduction in physical symptoms, such as decreased body temperature, when given to a patient that is febrile. The precise effective amount for a subject will depend upon the subject's size and health, the nature and extent of the condition, and the therapeutics or combination of therapeutics selected for administration. Thus, it is not useful to specify an exact effective amount in advance. However, the effective amount for a given situation can be determined by routine experimentation and is within the judgment of the clinician.

For purposes of the present invention, an effective dose will be from about 0.01 mg/kg to 50 mg/kg or 0.05 mg/kg to about 10 mg/kg of the DNA constructs in the individual to which it is administered.

A pharmaceutical composition can also contain a pharmaceutically acceptable carrier. The term "pharmaceutically acceptable carrier" refers to a carrier for administration of a therapeutic agent, such as antibodies or a polypeptide, genes, and other therapeutic agents. The term refers to any pharmaceutical carrier that does not itself induce the production of antibodies harmful to the individual receiving the composition, and which may be administered without undue toxicity. Suitable carriers may be large, slowly metabolized macromolecules such as proteins, polysaccharides, polylactic acids, polyglycolic acids, polymeric amino acids, amino acid copolymers, and inactive virus particles. Such carriers are well known to those of ordinary skill in the art.

Pharmaceutically acceptable salts can be used therein, for example, mineral acid salts such as hydrochlorides, hydrobromides, phosphates, sulfates, and the like; and the salts of organic acids such as acetates, propionates, malonates, benzoates, and the like. A thorough discussion of pharmaceutically acceptable excipients is available in Remington's Pharmaceutical Sciences (Mack Pub. Co., N.J. 1991).

Pharmaceutically acceptable carriers in therapeutic compositions may contain liquids such as water, saline, glycerol and ethanol. Additionally, auxiliary substances, such as wetting or emulsifying agents, pH buffering substances, and the like, may be present in such vehicles. Typically, the therapeutic compositions are prepared as injectables, either as liquid solutions or suspensions; solid forms suitable for solution in, or suspension in, liquid vehicles prior to injection may also be prepared. Liposomes are included within the definition of a pharmaceutically acceptable carrier.

Delivery Methods

Once formulated, the compositions of the invention can be administered directly to the subject. The subjects to be treated can be animals; in particular, human subjects can be treated.

Direct delivery of the compositions will generally be accomplished by injection, either subcutaneously, intraperitoneally, intravenously or intramuscularly or delivered to the interstitial space of a tissue. The compositions can also be administered into a lesion. Other modes of administration include oral and pulmonary administration, suppositories, and transdermal and transcutaneous applications, needles, and gene guns or hyposprays. Dosage treatment may be a single dose schedule or a multiple dose schedule.

Vaccines

Vaccines according to the invention may either be prophylactic (i.e., to prevent infection) or therapeutic (i.e., to treat disease after infection).

Such vaccines comprise immunizing antigen(s) or immunogen(s), immunogenic polypeptide, protein(s) or protein fragments, or nucleic acids (e.g., ribonucleic acid or deoxyribonucleic acid), usually in combination with "pharmaceutically acceptable carriers," which include any carrier that does not itself induce the production of antibodies harmful to the individual receiving the composition. Suitable carriers are typically large, slowly metabolized macromolecules such as proteins, polysaccharides, polylactic acids, polyglycolic acids, polymeric amino acids, amino acid copolymers, lipid aggregates (such as oil droplets or liposomes), and inactive virus particles. Such carriers are well known to those of ordinary skill in the art. Additionally, these carriers may function as immunostimulating agents ("adjuvants"). Furthermore, the immunogen or antigen may be conjugated to a bacterial toxoid, such as a toxoid from diphtheria, tetanus, cholera, *H. pylori*, etc, pathogens.

Preferred adjuvants to enhance effectiveness of the composition include, but are not limited to: (1) aluminum salts (alum), such as aluminum hydroxide, aluminum phosphate, aluminum sulfate, etc; (2) oil-in-water emulsion formulations (with or without other specific immunostimulating agents such as muramyl peptides (see below) or bacterial cell wall components), such as for example (a) MF59 (PCT Publ. No. WO 90/14837), containing 5% Squalene, 0.5% Tween 80, and 0.5% Span 85 (optionally containing various amounts of MTP-PE (see below), although not required) formulated into submicron particles using a microfluidizer such as Model 110Y microfluidizer (Microfluidics, Newton, MA), (b) SAF, containing 10% Squalane, 0.4% Tween 80, 5% pluronic-blocked polymer L121, and thr-MDP (see below) either microfluidized into a submicron emulsion or vortexed to generate a larger particle size emulsion, and (c) Ribi™ adjuvant system (RAS), (Ribi Immunochem, Hamilton, MT) containing 2% Squalene, 0.2% Tween 80, and one or more bacterial cell wall components from the group consisting of monophosphorylipid A (MPL), trehalose dimycolate (TDM), and cell wall skeleton (CWS), preferably MPL + CWS (Detox™); (3) saponin adjuvants, such as Stimulon™ (Cambridge Bioscience, Worcester, MA) may be used or particles generated therefrom such as ISCOMs (immunostimulating complexes); (4) Complete Freund's Adjuvant (CFA) and Incomplete Freund's Adjuvant (IFA); (5) cytokines, such as interleukins (e.g., IL-1, IL-2, IL-4, IL-5, IL-6, IL-7, IL-12, *etc.*), interferons (e.g., gamma interferon), macrophage colony stimulating factor (M-CSF), tumor necrosis factor (TNF), etc; (6) detoxified mutants of a bacterial ADP-ribosylating toxin such as a cholera toxin (CT), a pertussis toxin (PT), or an E. coli heat-labile toxin (LT), particularly LT-K63, LT-R72, CT-S109, PT-K9/G129; see, e.g., WO 93/13302 and WO 92/19265; and (7) other substances that act as immunostimulating agents to enhance the effectiveness of the composition. Alum and MF59 are preferred.

As mentioned above, muramyl peptides include, but are not limited to, N-acetyl-muramyl-L-threonyl-D-isoglutamine (thr-MDP), N-acetyl-normuramyl-L-alanyl-D-isoglutamine (nor-MDP), N-acetylmuramyl-L-alanyl-D-isoglutaminyl-L-alanine-2-(1'-2'-dipalmitoyl-*sn*-glycero-3-huydroxyphosphoryloxy)-ethylamine (MTP-PE), *etc.*

The vaccine compositions comprising immunogenic compositions (e.g., which may include the antigen, pharmaceutically acceptable carrier, and adjuvant) typically will contain diluents, such as water, saline, glycerol, ethanol, etc. Additionally, auxiliary substances, such as wetting or emulsifying agents, pH buffering substances, and the like, may be present in such vehicles. Alternatively, vaccine compositions comprising immunogenic compositions may comprise an antigen, polypeptide, protein, protein fragment or nucleic acid in a pharmaceutically acceptable carrier.

More specifically, vaccines comprising immunogenic compositions comprise an immunologically effective amount of the immunogenic polypeptides, as well as any other of the above-mentioned components, as needed. By "immunologically effective amount", it is meant that the administration of that amount to an individual, either in a single dose or as part of a series, is effective for treatment or prevention. This amount varies depending upon the health and physical condition of the individual to be treated, the taxonomic group of individual to be treated (e.g., nonhuman primate, primate, *etc.),* the capacity of the individual's immune system to synthesize antibodies, the degree of protection desired, the formulation of the vaccine, the treating doctor's assessment of the medical situation, and other relevant factors. It is expected that the amount will fall in a relatively broad range that can be determined through routine trials.

Typically, the vaccine compositions or immunogenic compositions are prepared as injectables, either as liquid solutions or suspensions; solid forms suitable for solution in, or suspension in, liquid vehicles prior to injection may also be prepared. The preparation also may be emulsified or encapsulated in liposomes for enhanced adjuvant effect, as discussed above under pharmaceutically acceptable carriers.

The immunogenic compositions are conventionally administered parenterally, e.g., by injection, either subcutaneously or intramuscularly. Additional formulations suitable for other modes of administration include oral and pulmonary formulations, suppositories, and transdermal and transcutaneous applications. Dosage treatment may be a single dose schedule or a multiple dose schedule. The vaccine may be administered in conjunction with other immunoregulatory agents.

As an alternative to protein-based vaccines, DNA vaccination may be employed (e.g., Robinson & Torres (1997) *Seminars in Immunology* 9:271-283; Donnelly *et al*. (1997) *Annu Rev Immunol* 15:617-648).

Gene Delivery Vehicles

Gene therapy vehicles for delivery of constructs, including a coding sequence of a therapeutic of the invention, to be delivered to the mammal for expression in the mammal, can be administered either locally or systemically. These constructs can utilize viral or non-viral vector approaches in *in vivo* or *ex vivo* modality. Expression of such coding sequence can be induced using endogenous mammalian or heterologous promoters. Expression of the coding sequence in vivo can be either constitutive or regulated.

The invention includes gene delivery vehicles capable of expressing the contemplated nucleic acid sequences. The gene delivery vehicle is preferably a viral vector and, more preferably, a retroviral, adenoviral, adeno-associated viral (AAV), herpes viral, or alphavirus vector. The viral vector can also be an astrovirus, coronavirus, orthomyxovirus, papovavirus, paramyxovirus, parvovirus, picomavirus, poxvirus, or togavirus viral vector. See generally, Jolly (1994) *Cancer Gene Therapy* 1:51-64; Kimura (1994) *Human Gene Therapy* 5:845-852; Connelly (1995) *Human Gene Therapy* 6:185-193; and Kaplitt (1994) *Nature Genetics* 6:148-153.

Retroviral vectors are well known in the art, including B, C and D type retroviruses, xenotropic retroviruses (for example, NZB-X1, NZB-X2 and NZB9-1 (see O'Neill (1985) *J. Virol*. 53:160) polytropic retroviruses e.g., MCF and MCF-MLV (see Kelly (1983) *J. Virol*. 45:291), spumaviruses and lentiviruses. See RNA Tumor Viruses, Second Edition, Cold Spring Harbor Laboratory, 1985.

Portions of the retroviral gene therapy vector may be derived from different retroviruses. For example, retrovector LTRs may be derived from a Murine Sarcoma Virus, a tRNA binding site from a Rous Sarcoma Virus, a packaging signal from a Murine Leukemia Virus, and an origin of second strand synthesis from an Avian Leukosis Virus.

These recombinant retroviral vectors may be used to generate transduction competent retroviral vector particles by introducing them into appropriate packaging cell lines (see US patent 5,591,624). Retrovirus vectors can be constructed for site-specific integration into host cell DNA by incorporation of a chimeric integrase enzyme into the retroviral particle (see W096/37626). It is preferable that the recombinant viral vector is a replication defective recombinant virus.

Packaging cell lines suitable for use with the above-described retrovirus vectors are well known in the art, are readily prepared (see WO95/30763 and W092/05266), and can be used to create producer cell lines (also termed vector cell lines or "VCLs") for the production of recombinant vector particles. Preferably, the packaging cell lines are made from human parent cells (e.g., HT1080 cells) or mink parent cell lines, which eliminates inactivation in human serum.

Preferred retroviruses for the construction of retroviral gene therapy vectors include Avian Leukosis Virus, Bovine Leukemia, Virus, Murine Leukemia Virus, Mink-Cell Focus-Inducing Virus, Murine Sarcoma Virus, Reticuloendotheliosis Virus and Rous Sarcoma Virus. Particularly preferred Murine Leukemia Viruses include 4070A and 1504A (Hartley and Rowe (1976) *J Virol* 19:19-25), Abelson (ATCC No. VR-999), Friend (ATCC No. VR-245), Graffi, Gross (ATCC Nol VR-590), Kirsten, Harvey Sarcoma Virus and Rauscher (ATCC No. VR-998) and Moloney Murine Leukemia Virus (ATCC No. VR-190). Such retroviruses may be obtained from depositories or collections such as the American Type Culture Collection ("ATCC") in Rockville, Maryland or isolated from known sources using commonly available techniques.

Exemplary known retroviral gene therapy vectors employable in this invention include those described in patent applications GB2200651, EP0415731, EP0345242, EP0334301, WO89/02468; WO89/05349, WO89/09271, WO90/02806, WO90/07936, WO94/03622, WO93/25698, WO93/25234, WO93/11230, WO93/10218, WO91/02805, WO91/02825, WO95/07994, US 5,219,740, US 4,405,712, US 4,861,719, US 4,980,289, US 4,777,127, US 5,591,624. See also Vile (1993) *Cancer Res* 53:3860-3864; Vile (1993) *Cancer Res* 53:962-967; Ram (1993) *Cancer Res* 53 (1993) 83-88; Takamiya (1992) *J Neurosci Res* 33:493-503; Baba (1993) *JNeurosurg* 79:729-735; Mann (1983) *Cell* 33:153; Cane (1984) *Proc Natl Acad Sci* 81:6349; and Miller (1990) *Human Gene Therapy 1.*

Human adenoviral gene therapy vectors are also known in the art and employable in this invention. See, for example, Berkner (1988) *Biotechniques* 6:616 and Rosenfeld (1991) *Science* 252:431, and WO93/07283, WO93/06223, and WO93/07282. Exemplary known adenoviral gene therapy vectors employable in this invention include those described in the above referenced documents and in WO94/12649, WO93/03769, WO93/19191, WO94/28938, WO95/11984, WO95/00655, WO95/27071, WO95/29993, WO95/34671, WO96/05320, WO94/08026, WO94/11506, WO93/06223, WO94/24299, WO95/14102, WO95/24297, WO95/02697, WO94/28152, WO94/24299, WO95/09241, WO95/25807, WO95/05835, WO94/18922 and WO95/09654. Alternatively, administration of DNA linked to killed adenovirus as described in Curiel (1992) *Hum. Gene Ther*. 3:147-154 may be employed. The gene delivery vehicles of the invention also include adenovirus associated virus (AAV) vectors. Leading and preferred examples of such vectors for use in this invention are the AAV-2 based vectors disclosed in Srivastava, WO93/09239. Most preferred AAV vectors comprise the two AAV inverted terminal repeats in which the native D-sequences are modified by substitution of nucleotides, such that at least 5 native nucleotides and up to 18 native nucleotides, preferably at least 10 native nucleotides up to 18 native nucleotides, most preferably 10 native nucleotides are retained and the remaining nucleotides of the D-sequence are deleted or replaced with non-native nucleotides. The native D-sequences of the AAV inverted terminal repeats are sequences of 20 consecutive nucleotides in each AAV inverted terminal repeat (i. e., there is one sequence at each end) which are not involved in HP formation. The non-native replacement nucleotide may be any nucleotide other than the nucleotide found in the native D-sequence in the same position. Other employable exemplary AAV vectors are pWP-19, pWN-1, both of which are disclosed in Nahreini (1993) *Gene* 124:257-262. Another example of such an AAV vector is psub201 (see Samulski (1987) *J. Virol*. 61:3096). Another exemplary AAV vector is the Double-D ITR vector. Construction of the Double-D ITR vector is disclosed in US Patent 5,478,745. Still other vectors are those disclosed in Carter US Patent 4,797,368 and Muzyczka US Patent 5,139,941, Chartejee US Patent 5,474,935, and Kotin WO94/288157. Yet a further example of an AAV vector employable in this invention is SSV9AFABTKneo, which contains the AFP enhancer and albumin promoter and directs expression predominantly in the liver. Its structure and construction are disclosed in Su (1996) *Human Gene Therapy* 7:463-470. Additional AAV gene therapy vectors are described in US 5,354,678, US 5,173,414, US 5,139,941, and US 5,252,479.

The gene therapy vectors comprising sequences of the invention also include herpes vectors. Leading and preferred examples are herpes simplex virus vectors containing a sequence encoding a thymidine kinase polypeptide such as those disclosed in US 5,288,641 and EP0176170 (Roizman). Additional exemplary herpes simplex virus vectors include HFEM/ICP6-LacZ disclosed in WO95/04139 (Wistar Institute), pHSVlac described in Geller (1988) *Science* 241:1667-1669 and in WO90/09441 and WO92/07945, HSV Us3::pgC-lacZ described in Fink (1992) *Human Gene Therapy* 3:11-19 and HSV 7134, 2 RH 105 and GAL4 described in EP 0453242 (Breakefield), and those deposited with the ATCC as accession numbers ATCC VR-977 and ATCC VR-260.

Also contemplated are alpha virus gene therapy vectors that can be employed in this invention. Preferred alpha virus vectors are Sindbis viruses vectors. Togaviruses, Semliki Forest virus (ATCC VR-67; ATCC VR-1247), Middleberg virus (ATCC VR-370), Ross River virus (ATCC VR-373; ATCC VR-1246), Venezuelan equine encephalitis virus (ATCC VR923; ATCC VR-1250; ATCC VR-1249; ATCC VR-532), and those described in US patents 5,091,309, 5,217,879, and WO92/10578. More particularly, those alpha virus vectors described in U.S. Serial No. 08/405,627, filed March 15, 1995,WO94/21792, WO92/10578, WO95/07994, US 5,091,309 and US 5,217,879 are employable. Such alpha viruses may be obtained from depositories or collections such as the ATCC in Rockville, Maryland or isolated from known sources using commonly available techniques. Preferably, alphavirus vectors with reduced cytotoxicity are used (see USSN 08/679640).

DNA vector systems such as eukarytic layered expression systems are also useful for expressing the nucleic acids of the invention. SeeWO95/07994 for a detailed description of eukaryotic layered expression systems. Preferably, the eukaryotic layered expression systems of the invention are derived from alphavirus vectors and most preferably from Sindbis viral vectors.

Other viral vectors suitable for use in the present invention include those derived from poliovirus, for example ATCC VR-58 and those described in Evans, Nature 339 (1989) 385 and Sabin (1973) *J. Biol. Standardization* 1:115; rhinovirus, for example ATCC VR-1110 and those described in Arnold (1990) *J Cell Biochem* L401; pox viruses such as canary pox virus or vaccinia virus, for example ATCC VR-111 and ATCC VR-201 0 and those described in Fisher-Hoch (1989) *Proc Natl Acad Sci* 86:317; Flexner (1989) *Ann NY Acad Sci* 569:86, Flexner (1990) *Vaccine* 8:17; in US 4,603,112 and US 4,769,330 and WO89/01973; SV40 virus, for example ATCC VR-305 and those described in Mulligan (1979) *Nature* 277:108 and Madzak (1992) *J Gen Virol* 73:1533; influenza virus, for example ATCC VR-797 and recombinant influenza viruses made employing reverse genetics techniques as described in US 5,166,057 and in Enami (1990) *Proc Natl Acad Sci* 87:3802-3805; Enami & Palese (1991) *J Virol* 65:2711-2713 and Luytjes (1989) *Cell 59: 110,* (see also McMichael (1983) *NEJMed* 309:13, and Yap (1978) *Nature* 273:238 and Nature (1979) 277:108); human immunodeficiency virus as described in EP-0386882 and in Buchschacher (1992) *J. Virol*. 66:2731; measles virus, for example ATCC VR-67 and VR-1247 and those described in EP-0440219; Aura virus, for example ATCC VR-368; Bebaru virus, for example ATCC VR-600 and ATCC VR-1240; Cabassou virus, for example ATCC VR-922; Chikungunya virus, for example ATCC VR-64 and ATCC VR-1241; Fort Morgan Virus, for example ATCC VR-924; Getah virus, for example ATCC VR-369 and ATCC VR-1243; Kyzylagach virus, for example ATCC VR-927; Mayaro virus, for example ATCC VR-66; Mucambo virus, for example ATCC VR-580 and ATCC VR-1244; Ndumu virus, for example ATCC VR-371; Pixuna virus, for example ATCC VR-372 and ATCC VR-1245; Tonate virus, for example ATCC VR-925; Triniti virus, for example ATCC VR-469; Una virus, for example ATCC VR-374; Whataroa virus, for example ATCC VR-926; Y-62-33 virus, for example ATCC VR-375; O'Nyong virus, Eastern encephalitis virus, for example ATCC VR-65 and ATCC VR-1242; Western encephalitis virus, for example ATCC VR-70, ATCC VR-1251, ATCC VR-622 and ATCC VR-1252; and coronavirus, for example ATCC VR-740 and those described in Hamre (1966) *Proc Soc Exp Biol Med* 121:190.

Delivery of the compositions of this invention into cells is not limited to the above mentioned viral vectors. Other delivery methods and media may be employed such as, for example, nucleic acid expression vectors, polycationic condensed DNA linked or unlinked to killed adenovirus alone, for example see US Serial No. 08/366,787, filed December 30, 1994 and Curiel (1992) *Hum Gene Ther* 3:147-154 ligand linked DNA, for example see Wu (1989) *J Biol*

*Chem* 264:16985-16987, eucaryotic cell delivery vehicles cells, for example see US Serial No.08/240,030, filed May 9, 1994, and US Serial No. 08/404,796, deposition of photopolymerized hydrogel materials, hand-held gene transfer particle gun, as described in US Patent 5,149,655, ionizing radiation as described in US5,206,152 and in WO92/11033, nucleic charge neutralization or fusion with cell membranes. Additional approaches are described in Philip (1994) *Mol Cell Biol* 14:2411-2418 and in Woffendin (1994) *Proc Natl Acad Sci* 91:1581-1585.

Particle mediated gene transfer may be employed, for example see US Serial No. 60/023,867. Briefly, the sequence can be inserted into conventional vectors that contain conventional control sequences for high level expression, and then incubated with synthetic gene transfer molecules such as polymeric DNA-binding cations like polylysine, protamine, and albumin, linked to cell targeting ligands such as asialoorosomucoid, as described in Wu & Wu (1987) *J. Biol. Chem.* 262:4429-4432, insulin as described in Hucked (1990) *Biochem Pharmacol* 40:253-263, galactose as described in Plank (1992) *Bioconjugate Chem* 3:533-539, lactose or transferrin.

Naked DNA may also be employed to transform a host cell. Exemplary naked DNA introduction methods are described in WO 90/11092 and US 5,580,859. Uptake efficiency may be improved using biodegradable latex beads. DNA coated latex beads are efficiently transported into cells after endocytosis initiation by the beads. The method may be improved further by treatment of the beads to increase hydrophobicity and thereby facilitate disruption of the endosome and release of the DNA into the cytoplasm.

Liposomes that can act as gene delivery vehicles are described in U.S. 5,422,120, WO95/13796, WO94/23697, WO91/14445 and EP-524,968. As described in USSN. 60/023,867, on non-viral delivery, the nucleic acid sequences encoding a polypeptide can be inserted into conventional vectors that contain conventional control sequences for high level expression, and then be incubated with synthetic gene transfer molecules such as polymeric DNA-binding cations like polylysine, protamine, and albumin, linked to cell targeting ligands such as asialoorosomucoid, insulin, galactose, lactose, or transferrin. Other delivery systems include the use of liposomes to encapsulate DNA comprising the gene under the control of a variety of tissue-specific or ubiquitously-active promoters. Further non-viral delivery suitable for use includes mechanical delivery systems such as the approach described in Woffendin *et al* (1994) *Proc. Natl. Acad. Sci. USA* 91(24):11581-11585. Moreover, the coding sequence and the product of expression of such can be delivered through deposition of photopolymerized hydrogel materials. Other conventional methods for gene delivery that can be used for delivery of the coding sequence include, for example, use of hand-held gene transfer particle gun, as described in U.S. 5,149,655; use of ionizing radiation for activating transferred gene, as described in U.S. 5,206,152 and WO92/11033

Exemplary liposome and polycationic gene delivery vehicles are those described in US 5,422,120 and 4,762,915; inWO 95/13796; WO94/23697; and WO91/14445; in EP-0524968; and in Stryer, Biochemistry, pages 236-240 (1975) W.H. Freeman, San Francisco; Szoka (1980) *Biochem Biophys Acta* 600:1; Bayer (1979) *Biochem Biophys Acta* 550:464; Rivnay (1987) *Meth Enrymol* 149:119; Wang (1987) *Proc Natl Acad Sci* 84:7851; Plant (1989) *Anal Biochem* 176:420.

A polynucleotide composition can comprise a therapeutically effective amount of a gene therapy vehicle, as the term is defined above. For purposes of the present invention, an effective dose will be from about 0.01 mg/ kg to 50 mg/kg or 0.05 mg/kg to about 10 mg/kg of the DNA constructs in the individual to which it is administered.

Delivery Methods

Once formulated, the polynucleotide compositions of the invention can be administered (1) directly to the subject; (2) delivered ex vivo, to cells derived from the subject; or (3) in vitro for expression of recombinant proteins. The subjects to be treated can be mammals or birds. Also, human subjects can be treated.

Direct delivery of the compositions will generally be accomplished by injection, either subcutaneously, intraperitoneally, transdermally or transcutaneously, intravenously or intramuscularly or delivered to the interstitial space of a tissue. The compositions can also be administered into a tumor or lesion. Other modes of administration include oral and pulmonary administration, suppositories, and transdermal applications, needles, and gene guns or hyposprays. Dosage treatment may be a single dose schedule or a multiple dose schedule. See WO98/20734.

Methods for the *ex vivo* delivery and reimplantation of transformed cells into a subject are known in the art and described in e.g., WO93/14778. Examples of cells useful in ex vivo applications include, for example, stem cells, particularly hematopoetic, lymph cells, macrophages, dendritic cells, or tumor cells.

Generally, delivery of nucleic acids for both *ex vivo* and *in vitro* applications can be accomplished by the following procedures, for example, dextran-mediated transfection, calcium phosphate precipitation, polybrene mediated transfection, protoplast fusion, electroporation, encapsulation of the polynucleotide(s) in liposomes, and direct microinjection of the DNA into nuclei, all well known in the art.

Polynucleotide and Polypeptide pharmaceutical compositions

In addition to the pharmaceutically acceptable carriers and salts described above, the following additional agents can be used with polynucleotide and/or polypeptide compositions.

A. Polypeptides

One example are polypeptides which include, without limitation: asialoorosomucoid (ASOR); transferrin; asialoglycoproteins; antibodies; antibody fragments; ferritin; interleukins; interferons, granulocyte, macrophage colony stimulating factor (GM-CSF), granulocyte colony stimulating factor (G-CSF), macrophage colony stimulating factor (M-CSF), stem cell factor and erythropoietin. Viral antigens, such as envelope proteins, can also be used. Also, proteins from other invasive organisms, such as the 17 amino acid peptide from the circumsporozoite protein of plasmodium falciparum known as RII.

B. Hormones, Vitamins, Etc.

Other groups that can be included in a pharmaceutical composition include, for example: hormones, steroids, androgens, estrogens, thyroid hormone, or vitamins, folic acid.

C. Polyalkylenes, Polysaccharides, *etc.*

Also, polyalkylene glycol can be included in a pharmaceutical compositions with the desired polynucleotides and/or polypeptides. In a preferred embodiment, the polyalkylene glycol is polyethlylene glycol. In addition, mono-, di-, or polysaccarides can be included. In a preferred embodiment of this aspect, the polysaccharide is dextran or DEAE-dextran. Also, chitosan and poly(lactide-co-glycolide) may be included in a pharmaceutical composition.

D. Lipids, and Liposomes

The desired polynucleotide or polypeptide can also be encapsulated in lipids or packaged in liposomes prior to delivery to the subject or to cells derived therefrom.

Lipid encapsulation is generally accomplished using liposomes which are able to stably bind or entrap and retain nucleic acid or polypeptide. The ratio of condensed polynucleotide to lipid preparation can vary but will generally be around 1:1 (mg DNA:micromoles lipid), or more of lipid. For a review of the use of liposomes as carriers for delivery of nucleic acids, see, Hug and Sleight (1991) *Biochim. Biophys. Acta.* 1097:1-17; Straubinger (1983) *Meth. Enzymol.* 101:512-527.

Liposomal preparations for use in the present invention include cationic (positively charged), anionic (negatively charged) and neutral preparations. Cationic liposomes have been shown to mediate intracellular delivery of plasmid DNA (Felgner (1987) *Proc. Natl. Acad. Sci. USA* 84:7413-7416); mRNA (Malone (1989) *Proc. Natl. Acad. Sci. USA* 86:6077-6081); and purified transcription factors (Debs (1990) *J. Biol. Chem*. 265:10189-10192), in functional form.

Cationic liposomes are readily available. For example, N(1-2,3-dioleyloxy)propyl)-N,N,N-triethylammonium (DOTMA) liposomes are available under the trademark Lipofectin, from GIBCO BRL, Grand Island, NY. (See, also, Felgner *supra*). Other commercially available liposomes include transfectace (DDAB/DOPE) and DOTAP/DOPE (Boerhinger). Other cationic liposomes can be prepared from readily available materials using techniques well known in the art. See, e.g., Szoka (1978) *Proc. Natl. Acad. Sci. USA* 75:4194-4198; WO90/11092 for a description of the synthesis of DOTAP (1,2-bis(oleoyloxy)-3-(trimethylammonio)propane) liposomes.

Similarly, anionic and neutral liposomes are readily available, such as from Avanti Polar Lipids (Birmingham, AL), or can be easily prepared using readily available materials. Such materials include phosphatidyl choline, cholesterol, phosphatidyl ethanolamine, dioleoylphosphatidyl choline (DOPC), dioleoylphosphatidyl glycerol (DOPG), dioleoylphoshatidyl ethanolamine (DOPE), among others. These materials can also be mixed with the DOTMA and DOTAP starting materials in appropriate ratios. Methods for making liposomes using these materials are well known in the art.

The liposomes can comprise multilammelar vesicles (MLVs), small unilamellar vesicles (SUVs), or large unilamellar vesicles (LUVs). The various liposome-nucleic acid complexes are prepared using methods known in the art. See e.g., Straubinger (1983) *Meth. Immunol*. 101:512-527; Szoka (1978) *Proc. Natl. Acad. Sci. USA* 75:4194-4198; Papahadjopoulos (1975) *Biochim. Biophys. Acta* 394:483; Wilson (1979) *Cell* 17:77); Deamer & Bangham (1976) *Biochim. Biophys. Acta* 443:629; Ostro (1977) *Biochem. Biophys. Res. Commun.* 76:836; Fraley (1979) *Proc. Natl. Acad. Sci. USA* 76:3348); Enoch & Strittmatter (1979) *Proc. Natl. Acad. Sci. USA* 76:145; Fraley (1980) *J. Biol. Chem.* (1980) 255:10431; Szoka & Papahadjopoulos (1978) *Proc. Natl. Acad. Sci. USA* 75:145; and Schaefer-Ridder (1982) *Science*

*215:166.*

E. Lipoproteins

In addition, lipoproteins can be included with the polynucleotide or polypeptide to be delivered. Examples of lipoproteins to be utilized include: chylomicrons, HDL, IDL, LDL, and VLDL. Mutants, fragments, or fusions of these proteins can also be used. Also, modifications of naturally occurring lipoproteins can be used, such as acetylated LDL. These lipoproteins can target the delivery of polynucleotides to cells expressing lipoprotein receptors. Preferably, if lipoproteins are including with the polynucleotide to be delivered, no other targeting ligand is included in the composition.
Naturally occurring lipoproteins comprise a lipid and a protein portion. The protein portion are known as apoproteins. At the present, apoproteins A, B, C, D, and E have been isolated and identified. At least two of these contain several proteins, designated by Roman numerals, AI, AII, AIV; CI, CII, CIII.
A lipoprotein can comprise more than one apoprotein. For example, naturally occurring chylomicrons comprises of A, B, C, and E; over time these lipoproteins lose A and acquire C and E apoproteins. VLDL comprises A, B, C, and E apoproteins, LDL comprises apoprotein B; and HDL comprises apoproteins A, C, and E.
The amino acid sequences of these apoproteins are known and are described in, for example, Breslow (1985) *Annu Rev. Biochem* 54:699; Law (1986) *Adv. Exp Med. Biol*. 151:162; Chen (1986) *J Biol Chem* 261:12918; Kane (1980) *Proc Natl Acad Sci USA* 77:2465; and Utermann (1984) *Hum Genet* 65:232.
Lipoproteins contain a variety of lipids including, triglycerides, cholesterol (free and esters), and phopholipids. The composition of the lipids varies in naturally occurring lipoproteins. For example, chylomicrons comprise mainly triglycerides. A more detailed description of the lipid content of naturally occurring lipoproteins can be found, for example, in *Meth. Enzymol*. 128 (1986). The composition of the lipids are chosen to aid in conformation of the apoprotein for receptor binding activity. The composition of lipids can also be chosen to facilitate hydrophobic interaction and association with the polynucleotide binding molecule.
Naturally occurring lipoproteins can be isolated from serum by ultracentrifugation, for instance. Such methods are described in *Meth. Enzymol*. (*supra*)*;* Pitas (1980) *J. Biochem.* 255:5454-5460 and Mahey (1979) *J Clin. Invest* 64: 743-750.
Lipoproteins can also be produced by *in vitro* or recombinant methods by expression of the apoprotein genes in a desired host cell. See, for example, Atkinson (1986) *Annu Rev Biophys Chem* 15:403 and Radding (1958) *Biochim Biophys Acta* 30: 443.
Lipoproteins can also be purchased from commercial suppliers, such as Biomedical Techniologies, Inc., Stoughton, Massachusetts, USA.
Further description of lipoproteins can be found in Zuckermann et al., PCT. Appln. No. US97/14465.

F. Polycationic Agents

Polycationic agents can be included, with or without lipoprotein, in a composition with the desired polynucleotide and/or polypeptide to be delivered.
Polycationic agents, typically, exhibit a net positive charge at physiological relevant pH and are capable of neutralizing the electrical charge of nucleic acids to facilitate delivery to a desired location. These agents have both in vitro, ex vivo, and in vivo applications. Polycationic agents can be used to deliver nucleic acids to a living subject either intramuscularly, subcutaneously, etc.
The following are examples of useful polypeptides as polycationic agents: polylysine, polyarginine, polyornithine, and protamine. Other examples of useful polypeptides include histones, protamines, human serum albumin, DNA binding proteins, non-histone chromosomal proteins, coat proteins from DNA viruses, such as ΦX174, transcriptional factors also contain domains that bind DNA and therefore may be useful as nucleic aid condensing agents. Briefly, transcriptional factors such as C/CEBP, c-jun, c-fos, AP-1, AP-2, AP-3, CPF, Prot-1, Sp-1, Oct-1, Oct-2, CREP, and TFIID contain basic domains that bind DNA sequences.
Organic polycationic agents include: spermine, spermidine, and purtrescine.
The dimensions and of the physical properties of a polycationic agent can be extrapolated from the list above, to construct other polypeptide polycationic agents or to produce synthetic polycationic agents.

G. Synthetic Polycationic Agents

Synthetic polycationic agents which are useful in pharmaceutical compositions include, for example, DEAE-dextran, polybrene. Lipofectin™, and lipofectAMINE™ are monomers that form polycationic complexes when combined with polynucleotides or polypeptides.

Immunodiagnostic Assays

*Neisseria* MenB antigens, or antigenic fragments thereof, of the invention can be used in immunoassays to detect antibody levels (or, conversely, anti-*Neisseria* MenB antibodies can be used to detect antigen levels). Immunoassays based on well defined, recombinant antigens can be developed to replace invasive diagnostics methods. Antibodies to *Neisseria* MenB proteins or fragments thereof within biological samples, including for example, blood or serum samples, can be detected. Design of the immunoassays is subject to a great deal of variation, and a variety of these are known in the art. Protocols for the immunoassay may be based, for example, upon competition, or direct reaction, or sandwich type assays. Protocols may also, for example, use solid supports, or may be by immunoprecipitation. Most assays involve the use of labeled antibody or polypeptide; the labels may be, for example, fluorescent, chemiluminescent, radioactive, or dye molecules. Assays which amplify the signals from the probe are also known; examples of which are assays which utilize biotin and avidin, and enzyme-labeled and mediated immunoassays, such as ELISA assays.

Kits suitable for immunodiagnosis and containing the appropriate labeled reagents are constructed by packaging the appropriate materials, including the compositions of the invention, in suitable containers, along with the remaining reagents and materials (for example, suitable buffers, salt solutions, *etc.)* required for the conduct of the assay, as well as suitable set of assay instructions.

Nucleic Acid Hybridization

"Hybridization" refers to the association of two nucleic acid sequences to one another by hydrogen bonding. Typically, one sequence will be fixed to a solid support and the other will be free in solution. Then, the two sequences will be placed in contact with one another under conditions that favor hydrogen bonding. Factors that affect this bonding include: the type and volume of solvent; reaction temperature; time of hybridization; agitation; agents to block the non-specific attachment of the liquid phase sequence to the solid support (Denhardt's reagent or BLOTTO); concentration of the sequences; use of compounds to increase the rate of association of sequences (dextran sulfate or polyethylene glycol); and the stringency of the washing conditions following hybridization. See Sambrook *et al*. (*supra*) Volume 2, chapter 9, pages 9.47 to 9.57.

"Stringency" refers to conditions in a hybridization reaction that favor association of very similar sequences over sequences that differ. For example, the combination of temperature and salt concentration should be chosen that is approximately 120 to 200°C below the calculated Tm of the hybrid under study. The temperature and salt conditions can often be determined empirically in preliminary experiments in which samples of genomic DNA immobilized on filters are hybridized to the sequence of interest and then washed under conditions of different stringencies. See Sambrook *et al*. at page 9.50.

Variables to consider when performing, for example, a Southern blot are (1) the complexity of the DNA being blotted and (2) the homology between the probe and the sequences being detected. The total amount of the fragment (s) to be studied can vary a magnitude of 10, from 0.1 to 1μg for a plasmid or phage digest to $10^{-9}$ to $10^{-8}$ g for a single copy gene in a highly complex eukaryotic genome. For lower complexity polynucleotides, substantially shorter blotting, hybridization, and exposure times, a smaller amount of starting polynucleotides, and lower specific activity of probes can be used. For example, a single-copy yeast gene can be detected with an exposure time of only 1 hour starting with 1 μg of yeast DNA, blotting for two hours, and hybridizing for 4-8 hours with a probe of $10^8$ cpm/μg. For a single-copy mammalian gene a conservative approach would start with 10 μg of DNA, blot overnight, and hybridize overnight in the presence of 10% dextran sulfate using a probe of greater than $10^8$ cpm/μg, resulting in an exposure time of ~24 hours.

Several factors can affect the melting temperature (Tm) of a DNA-DNA hybrid between the probe and the fragment of interest, and consequently, the appropriate conditions for hybridization and washing. In many cases the probe is not 100% homologous to the fragment. Other commonly encountered variables include the length and total G+C content of the hybridizing sequences and the ionic strength and formamide content of the hybridization buffer. The effects of all of these factors can be approximated by a single equation: $Tm= 81 + 16.6(\log_{10}Ci) + 0.4(\%(G + C)) - 0.6(\%formamide) - 600/n - 1.5(\%mismatch)$ where Ci is the salt concentration (monovalent ions) and *n* is the length of the hybrid in base pairs (slightly modified from Meinkoth & Wahl (1984) *Anal. Biochem.* 138:267-284).

In designing a hybridization experiment, some factors affecting nucleic acid hybridization can be conveniently altered. The temperature of the hybridization and washes and the salt concentration during the washes are the simplest to adjust. As the temperature of the hybridization increases (i.e., stringency), it becomes less likely for hybridization to occur between strands that are nonhomologous, and as a result, background decreases. If the radiolabeled probe is not completely homologous with the immobilized fragment (as is frequently the case in gene family and interspecies hybridization experiments), the hybridization temperature must be reduced, and background will increase. The temperature of the washes affects the intensity of the hybridizing band and the degree of background in a similar manner.

The stringency of the washes is also increased with decreasing salt concentrations.

In general, convenient hybridization temperatures in the presence of 50% formamide are 42°C for a probe with is 95% to 100% homologous to the target fragment, 37°C for 90% to 95% homology, and 32°C for 85% to 90% homology. For lower homologies, formamide content should be lowered and temperature adjusted accordingly, using the equation above. If the homology between the probe and the target fragment are not known, the simplest approach is to start with both hybridization and wash conditions which are nonstringent. If non-specific bands or high background are observed after autoradiography, the filter can be washed at high stringency and reexposed. If the time required for exposure makes this approach impractical, several hybridization and/or washing stringencies should be tested in parallel.

Nucleic Acid Probe Assays

Methods such as PCR, branched DNA probe assays, or blotting techniques utilizing nucleic acid probes according to the invention can determine the presence of cDNA or mRNA. A probe is said to "hybridize" with a sequence of the invention if it can form a duplex or double stranded complex, which is stable enough to be detected.

The nucleic acid probes will hybridize to the Neisserial nucleotide sequences of the invention (including both sense and antisense strands). Though many different nucleotide sequences will encode the amino acid sequence, the native Neisserial sequence is preferred because it is the actual sequence present in cells. mRNA represents a coding sequence and so a probe should be complementary to the coding sequence; single-stranded cDNA is complementary to mRNA, and so a cDNA probe should be complementary to the non-coding sequence.

The probe sequence need not be identical to the Neisserial sequence (or its complement) -- some variation in the sequence and length can lead to increased assay sensitivity if the nucleic acid probe can form a duplex with target nucleotides, which can be detected. Also, the nucleic acid probe can include additional nucleotides to stabilize the formed duplex. Additional Neisserial sequence may also be helpful as a label to detect the formed duplex. For example, a non-complementary nucleotide sequence may be attached to the 5' end of the probe, with the remainder of the probe sequence being complementary to a Neisserial sequence. Alternatively, non-complementary bases or longer sequences can be interspersed into the probe, provided that the probe sequence has sufficient complementarity with the a Neisserial sequence in order to hybridize therewith and thereby form a duplex which can be detected.

The exact length and sequence of the probe will depend on the hybridization conditions, such as temperature, salt condition and the like. For example, for diagnostic applications, depending on the complexity of the analyte sequence, the nucleic acid probe typically contains at least 10-20 nucleotides, preferably 15-25, and more preferably at least 30 nucleotides, although it may be shorter than this. Short primers generally require cooler temperatures to form sufficiently stable hybrid complexes with the template.

Probes may be produced by synthetic procedures, such as the triester method of Matteucci *et al*. (*J. Am. Chem. Soc.* (1981) 103:3185), or according to Urdea *et al.* (*Proc. Natl. Acad. Sci. USA* (1983) 80: 7461), or using commercially available automated oligonucleotide synthesizers.

The chemical nature of the probe can be selected according to preference. For certain applications, DNA or RNA are appropriate. For other applications, modifications may be incorporated e.g., backbone modifications, such as phosphorothioates or methylphosphonates, can be used to increase *in vivo* half-life, alter RNA affinity, increase nuclease resistance *etc.* (e.g., see Agrawal & Iyer (1995) *Curr Opin Biotechnol* 6:12-19; Agrawal (1996) *TIBTECH* 14: 376-387); analogues such as peptide nucleic acids may also be used (e.g., see Corey (1997) *TIBTECH* 15:224-229; Buchardt *et al*. (1993) *TIBTECH* 11:384-386).

One example of a nucleotide hybridization assay is described by *Urdea et al*. in international patent application WO92/02526 (see also U.S. Patent 5,124,246).

Alternatively, the polymerase chain reaction (PCR) is another well-known means for detecting small amounts of target nucleic acids. The assay is described in: Mullis *et al*. (*Meth*. *Enzymol*. (1987) 155: 335-350); US patent 4,683,195; and US patent 4,683,202. Two "primer" nucleotides hybridize with the target nucleic acids and are used to prime the reaction. The primers can comprise sequence that does not hybridize to the sequence of the amplification target (or its complement) to aid with duplex stability or, for example, to incorporate a convenient restriction site. Typically, such sequence will flank the desired Neisserial sequence.

A thermostable polymerase creates copies of target nucleic acids from the primers using the original target nucleic acids as a template. After a threshold amount of target nucleic acids are generated by the polymerase, they can be detected by more traditional methods, such as Southern blots. When using the Southern blot method, the labeled probe will hybridize to the Neisserial sequence (or its complement).

Also, mRNA or cDNA can be detected by traditional blotting techniques described in Sambrook *et al* (*supra*). mRNA, or cDNA generated from mRNA using a polymerase enzyme, can be purified and separated using gel electrophoresis. The nucleic acids on the gel are then blotted onto a solid support, such as nitrocellulose. The solid support is exposed to a labeled probe and then washed to remove any unhybridized probe. Next, the duplexes containing the labeled probe are detected. Typically, the probe is labeled with a radioactive moiety.

## EXAMPLES

The invention is based on the 961 nucleotide sequences from the genome of *N. meningitidis* set out in Appendix C, SEQ ID NOs:1-961 of the '573 application, which together represent substantially the complete genome of serotype B of *N. meningitidis*, as well as the full length genome sequence shown in Appendix D, SEQ ID NO 1068 of the '573 application, and the full length genome sequence shown in Appendix A hereto, SEQ ID NO. 1.

It will be self-evident to the skilled person how this sequence information can be utilized according to the invention, as above described.

The standard techniques and procedures which may be employed in order to perform the invention (e.g. to utilize the disclosed sequences to predict polypeptides useful for vaccination or diagnostic purposes) were summarized above. This summary is not a limitation on the invention but, rather, gives examples that may be used, but are not required.

These sequences are derived from contigs shown in Appendix C (SEQ ID NOs 1-961) and from the full length genome sequence shown in Appendix D (SEQ ID NO 1068), which were prepared during the sequencing of the genome of *N. meningitidis* (strain B). The full length sequence was assembled using the TIGR Assembler as described by G. S. Sutton et al., *TIGR Assembler: A New Tool for Assembling Large Shotgun Sequencing Projects,* Genome Science and Technology, 1:9-19 (1995) *[see also* R. D. Fleischmann, et al., Science 269, 496-512 (1995); C. M. Fraser, et al., Science 270, 397-403 (1995); C. J. Bult, et al., Science 273, 1058-73 (1996); C. M. Fraser, et. al, Nature 390, 580-586 (1997); J.-F. Tomb, et. al., Nature 388, 539-547 (1997); H. P. Klenk, et al., Nature 390, 364-70 (1997); C. M. Fraser, et al., Science 281, 375-88 (1998); M. J. Gardner, et al., Science 282, 1126-1132 (1998); K. E. Nelson, et al., Nature 399, 323-9 (1999)]. Then, using the above-described methods, putative translation products of the sequences were determined. Computer analysis of the translation products were determined based on database comparisons. Corresponding gene and protein sequences, if any, were identified in Neisseria meningitidis (Strain A) and Neisseria gonorrhoeae. Then the proteins were expressed, purified, and characterized to assess their antigenicity and immunogenicity.

In particular, the following methods were used to express, purify, and biochemically characterize the proteins of the invention.

### Chromosomal DNA Preparation

*N. meningitidis* strain 2996 was grown to exponential phase in 100 ml of GC medium, harvested by centrifugation, and resuspended in 5 ml buffer (20% Sucrose, 50 mM Tris-HCl, 50 mM EDTA, adjusted to pH 8.0). After 10 minutes incubation on ice, the bacteria were lysed by adding 10 ml lysis solution (50 mM NaCl, 1% Na-Sarkosyl, 50 µg/ml Proteinase K), and the suspension was incubated at 37°C for 2 hours. Two phenol extractions (equilibrated to pH 8) and one $ChCl_3$/isoamylalcohol (24:1) extraction were performed. DNA was precipitated by addition of 0.3M sodium acetate and 2 volumes ethanol, and was collected by centrifugation. The pellet was washed once with 70% ethanol and redissolved in 4 ml buffer (10 mM Tris-HCl, 1mM EDTA, pH 8). The DNA concentration was measured by reading the OD at 260 nm.

### Oligonucleotide design

Synthetic oligonucleotide primers were designed on the basis of the coding sequence of each ORF, using (a) the meningococcus B sequence when available, or (b) the gonococcus/meningococcus A sequence, adapted to the codon preference usage of meningococcus. Any predicted signal peptides were omitted, by deducing the 5'-end amplification primer sequence immediately downstream from the predicted leader sequence.

For most ORFs, the 5' primers included two restriction enzyme recognition sites (*BamH*I-*Nde*I, *BamH*I-*Nhe*I, or *EcoR*I-*Nhe*I, depending on the gene's restriction pattern); the 3' primers included a *Xho*I restriction site. This procedure was established in order to direct the cloning of each amplification product (corresponding to each ORF) into two different expression systems: pGEX-KG (using either *BamH*I-*Xho*I or *EcoR*I-*Xho*I), and pET21b+ (using either *Nde*I-*Xho*I or *Nhe*I-*Xho*I).

| | | |
|---|---|---|
| **5'-end primer tail:** | CGCGGATCCCATATG | **(*BamH*I-*Nde*I )** |
| | CGCGGATCCGCTAGC | **(*BamH*I-*Nhe*I)** |
| | CCGGAATTCTAGCTAGC | **(*EcoR*I-*Nhe*I)** |

**3'-end primer tail:** CCCGCTCGAG (*Xho*I)

For some ORFs, two different amplifications were performed to clone each ORF in the two expression systems. Two different 5' primers were used for each ORF; the same 3' *Xho*I primer was used as before:

**5'-end primer tail:** GGAATTCCATATGGCCATGG (*Nde*I)

**5'-end primer tail:** CGGGATCC (*BamH*I)

Other ORFs were cloned in the pTRC expression vector and expressed as an amino-terminus His-tag fusion. The predicted signal peptide may be included in the final product. *Nhe*I-*BamH*I restriction sites were incorporated using primers:

**5'-end primer tail:** GATCAGCTAGCCATATG (*Nhe*I)

**3'-end primer tail:** CGGGATCC (*BamH*I)

As well as containing the restriction enzyme recognition sequences, the primers included nucleotides which hybridizeed to the sequence to be amplified. The number of hybridizing nucleotides depended on the melting temperature of the whole primer, and was determined for each primer using the formulae:

$$T_m = 4\ (G+C) + 2\ (A+T) \qquad \text{(tail excluded )}$$

$$T_m = 64.9 + 0.41\ (\%GC) - 600/N \qquad \text{(whole primer)}$$

The average melting temperature of the selected oligos were 65-70°C for the whole oligo and 50-55°C for the hybridising region alone.

Oligos were synthesized by a Perkin Elmer 394 DNA/RNA Synthesizer, eluted from the columns in 2 ml $NH_4$-OH, and deprotected by 5 hours incubation at 56 °C. The oligos were precipitated by addition of 0.3M Na-Acetate and 2 volumes ethanol. The samples were then centrifuged and the pellets resuspended in either 100μl or 1ml of water. $OD_{260}$ was determined using a Perkin Elmer Lambda Bio spectophotometer and the concentration was determined and adjusted to 2-10 pmol/μl.

Table 1 shows the forward and reverse primers used for each amplification. In certain cases, it might be noted that the sequence of the primer does not exactly match the sequence in the ORF. When initial amplifications are performed, the complete 5' and/or 3' sequence may not be known for some meningococcal ORFs, although the corresponding sequences may have been identified in gonoccus. For amplification, the gonococcal sequences could thus be used as the basis for primer design, altered to take account of codon preference. In particular, the following codons may be changed: ATA→ATT; TCG→TCT; CAG→CAA; AAG→AAA; GAG→GAA; CGA and CGG→CGC; GGG→GGC.

**Amplification**

The standard PCR protocol was as follows: 50-200 ng of genomic DNA were used as a template in the presence of 20-40 μM of each oligo, 400-800 μM dNTPs solution, 1x PCR buffer (including 1.5 mM $MgCl_2$), 2.5 units *TaqI* DNA polymerase (using Perkin-Elmer AmpliTaQ, GIBCO Platinum, Pwo DNA polymerase, or Tahara Shuzo Taq polymerase).

In some cases, PCR was optimsed by the addition of 10μl DMSO or 50 μl 2M betaine.

After a hot start (adding the polymerase during a preliminary 3 minute incubation of the whole mix at 95°C), each sample underwent a double-step amplification: the first 5 cycles were performed using as the hybridization temperature the one of the oligos excluding the restriction enzymes tail, followed by 30 cycles performed according to the hybridization temperature of the whole length oligos. The cycles were followed by a final 10 minute extension step at 72°C.

The standard cycles were as follows:

| | Denaturation | Hybridisation | Elongation |
|---|---|---|---|
| First 5 cycles | 30 seconds 95°C | 30 seconds 50-55°C | 30-60 seconds 72°C |
| Last 30 cycles | 30 seconds 95°C | 30 seconds 65-70°C | 30-60 seconds 72°C |

The elongation time varied according to the length of the ORF to be amplified.

The amplifications were performed using either a 9600 or a 2400 Perkin Elmer GeneAmp PCR System. To check the results, 1/10 of the amplification volume was loaded onto a 1-1.5% agarose gel and the size of each amplified fragment compared with a DNA molecular weight marker.

The amplified DNA was either loaded directly on a 1% agarose gel or first precipitated with ethanol and resuspended in a suitable volume to be loaded on a 1% agarose gel. The DNA fragment corresponding to the right size band was then eluted and purified from gel, using the Qiagen Gel Extraction Kit, following the instructions of the manufacturer. The final volume of the DNA fragment was 30µl or 50µl of either water or 10mM Tris, pH 8.5.

**Digestion of PCR fragments**

The purified DNA corresponding to the amplified fragment was split into 2 aliquots and double-digested with:

NdeI/*Xho*I or *Nhe*I/*Xho*I for cloning into pET-21b+ and further expression of the protein as a C-terminus His-tag fusion

BamHI/*Xho*I or *EcoR*I/*Xho*I for cloning into pGEX-KG and further expression of the protein as a GST N-terminus fusion.

For ORF 76, *Nhe*I/*BamH*I for cloning into pTRC-HisA vector and further expression of the protein as N-terminus His-tag fusion.

Each purified DNA fragment was incubated (37°C for 3 hours to overnight) with 20 units of each restriction enzyme (New England Biolabs ) in a either 30 or 40 µl final volume in the presence of the appropriate buffer. The digestion product was then purified using the QIAquick PCR purification kit, following the manufacturer's instructions, and eluted in a final volume of 30 (or 50) µl of either water or 10mM Tris-HCl, pH 8.5. The final DNA concentration was determined by 1% agarose gel electrophoresis in the presence of titrated molecular weight marker.

**Digestion of the cloning vectors (pET22B, pGEX-KG and pTRC-His A)**

10 µg plasmid was double-digested with 50 units of each restriction enzyme in 200 µl reaction volume in the presence of appropriate buffer by overnight incubation at 37°C. After loading the whole digestion on a 1% agarose gel, the band corresponding to the digested vector was purified from the gel using the Qiagen QIAquick Gel Extraction Kit and the DNA was eluted in 50 µl of 10 mM Tris-HCl, pH 8.5. The DNA concentration was evaluated by measuring $OD_{260}$ of the sample, and adjusted to 50 µg/µl. 1 µl of plasmid was used for each cloning procedure.

**Cloning**

The fragments corresponding to each ORF, previously digested and purified, were ligated in both pET22b and pGEX-KG. In a final volume of 20 µl, a molar ratio of 3:1 fragment/vector was ligated using 0.5 µl of NEB T4 DNA ligase (400 units/µl), in the presence of the buffer supplied by the manufacturer. The reaction was incubated at room temperature for 3 hours. In some experiments, ligation was performed using the Boheringer "Rapid Ligation Kit", following the manufacturer's instructions.

In order to introduce the recombinant plasmid in a suitable strain, 100 µl *E. coli* DH5 competent cells were incubated with the ligase reaction solution for 40 minutes on ice, then at 37°C for 3 minutes, then, after adding 800 µl LB broth, again at 37°C for 20 minutes. The cells were then centrifuged at maximum speed in an Eppendorf microfuge and resuspended in approximately 200 µl of the supernatant. The suspension was then plated on LB ampicillin (100 mg/ml ).

The screening of the recombinant clones was performed by growing 5 randomly-chosen colonies overnight at 37 °C in either 2 ml (pGEX or pTC clones) or 5ml (pET clones) LB broth + 100 µg/ml ampicillin. The cells were then pelletted and the DNA extracted using the Qiagen QIAprep Spin Miniprep Kit, following the manufacturer's instructions, to a final volume of 30 µl. 5 µl of each individual miniprep (approximately 1g ) were digested with either *Nde*I/*Xho*I or *BamH*I/*Xho*I and the whole digestion loaded onto a 1-1.5% agarose gel (depending on the expected insert size), in parallel with the molecular weight marker (1Kb DNA Ladder, GIBCO). The screening of the positive clones was made on the base of the correct insert size.

**Cloning**

Certain ORFs may be cloned into the pGEX-HIS vector using *EcoR*I-*Pst*I, *EcoR*I-*Sal*I, or *Sal*I-*Pst*I cloning sites. After cloning, the recombinant plasmids may be introduced in the E.coli host W3110.

**Expression**

Each ORF cloned into the expression vector may then be transformed into the strain suitable for expression of the recombinant protein product. 1 µl of each construct was used to transform 30 µl of *E.coli* BL21 (pGEX vector), *E.coli* TOP 10 (pTRC vector) or *E.coli* BL21-DE3 (pET vector), as described above. In the case of the pGEX-His vector, the same *E.coli* strain (W3110) was used for initial cloning and expression. Single recombinant colonies were inoculated into 2ml LB+Amp (100 µg/ml), incubated at 37°C overnight, then diluted 1:30 in 20 ml of LB+Amp (100 µg/ml) in 100 ml flasks, making sure that the $OD_{600}$ ranged between 0.1 and 0.15. The flasks were incubated at 30°C into gyratory water bath shakers until OD indicated exponential growth suitable for induction of expression (0.4-0.8 OD for pET and pTRC vectors; 0.8-1 OD for pGEX and pGEX-His vectors). For the pET, pTRC and pGEX-His vectors, the protein expression was induced by addiction of 1mM IPTG, whereas in the case of pGEX system the final concentration of IPTG was 0.2 mM. After 3 hours incubation at 30°C, the final concentration of the sample was checked by OD. In order to check expression, 1ml of each sample was removed, centrifuged in a microfuge, the pellet resuspended in PBS, and analysed by 12% SDS-PAGE with Coomassie Blue staining. The whole sample was centrifuged at 6000g and the pellet resuspended in PBS for further use.

**GST-fusion proteins large-scale purification.**

A single colony was grown overnight at 37°C on LB+Amp agar plate. The bacteria were inoculated into 20 ml of LB+Amp liquid colture in a water bath shaker and grown overnight. Bacteria were diluted 1:30 into 600 ml of fresh medium and allowed to grow at the optimal temperature (20-37°C) to $OD_{550}$ 0.8-1. Protein expression was induced with 0.2mM IPTG followed by three hours incubation. The culture was centrifuged at 8000 rpm at 4°C. The supernatant was discarded and the bacterial pellet was resuspended in 7.5 ml cold PBS. The cells were disrupted by sonication on ice for 30 sec at 40W using a Branson sonifier B-15, frozen and thawed two times and centrifuged again. The supernatant was collected and mixed with 150µl Glutatione-Sepharose 4B resin (Pharmacia) (previously washed with PBS) and incubated at room temperature for 30 minutes. The sample was centrifuged at 700g for 5 minutes at 4C. The resin was washed twice with 10 ml cold PBS for 10 minutes, resuspended in 1ml cold PBS, and loaded on a disposable column. The resin was washed twice with 2ml cold PBS until the flow-through reached $OD_{280}$ of 0.02-0.06. The GST-fusion protein was eluted by addition of 700µl cold Glutathione elution buffer 10mM reduced glutathione, 50mM Tris-HCl) and fractions collected until the $OD_{280}$ was 0.1. 21µl of each fraction were loaded on a 12% SDS gel using either Biorad SDS-PAGE Molecular weight standard broad range (M1) (200, 116.25, 97.4, 66.2, 45, 31, 21.5, 14.4, 6.5 kDa) or Amersham Rainbow Marker (M") (220, 66, 46, 30, 21.5, 14.3 kDa) as standards. As the MW of GST is 26kDa, this value must be added to the MW of each GST-fusion protein.

**His-fusion soluble proteins large-scale purification.**

A single colony was grown overnight at 37°C on a LB + Amp agar plate. The bacteria were inoculated into 20ml of LB+Amp liquid culture and incubated overnight in a water bath shaker. Bacteria were diluted 1:30 into 600ml fresh medium and allowed to grow at the optimal temperature (20-37°C) to $OD_{550}$ 0.6-0.8. Protein expression was induced by addition of 1 mM IPTG and the culture further incubated for three hours. The culture was centrifuged at 8000 rpm at 4°C, the supernatant was discarded and the bacterial pellet was resuspended in 7.5ml cold 10mM imidazole buffer (300 mM NaCl, 50 mM phosphate buffer, 10 mM imidazole, pH 8). The cells were disrupted by sonication on ice for 30 sec at 40W using a Branson sonifier B-15, frozen and thawed two times and centrifuged again. The supernatant was collected and mixed with 150µl $Ni^{2+}$-resin (Pharmacia) (previously washed with 10mM imidazole buffer) and incubated at room temperature with gentle agitation for 30 minutes. The sample was centrifuged at 700g for 5 minutes at 4°C. The resin was washed twice with 10 ml cold 10mM imidazole buffer for 10 minutes, resuspended in 1ml cold 10mM imidazole buffer and loaded on a disposable column. The resin was washed at 4°C with 2ml cold 10mM imidazole buffer until the flow-through reached the $O.D_{280}$ of 0.02-0.06. The resin was washed with 2ml cold 20mM imidazole buffer (300 mM NaCl, 50 mM phosphate buffer, 20 mM imidazole, pH 8) until the flow-through reached the $O.D_{280}$ of 0.02-0.06. The His-fusion protein was eluted by addition of 700µl cold 250mM imidazole buffer (300 mM NaCl, 50 mM phosphate buffer, 250 mM imidazole, pH 8) and fractions collected until the $O.D_{280}$ was 0.1, 21µl of each fraction were loaded on a 12% SDS gel.

**His-fusion insoluble proteins large-scale purification.**

A single colony was grown overnight at 37 °C on a LB + Amp agar plate. The bacteria were inoculated into 20 ml of LB+Amp liquid culture in a water bath shaker and grown overnight. Bacteria were diluted 1:30 into 600ml fresh medium and let to grow at the optimal temperature (37°C) to $O.D_{550}$ 0.6-0.8. Protein expression was induced by addition of 1 mM IPTG and the culture further incubated for three hours. The culture was centrifuged at 8000rpm at 4°C. The supernatant was discarded and the bacterial pellet was resuspended in 7.5 ml buffer B (urea 8M, 10mM Tris-HCl, 100mM phosphate buffer, pH 8.8). The cells were disrupted by sonication on ice for 30 sec at 40W using a Branson sonifier B-15, frozen and thawed twice and centrifuged again. The supernatant was stored at -20°C, while the pellets were resuspended in 2 ml guanidine buffer (6M guanidine hydrochloride, 100mM phosphate buffer, 10 mM Tris-HCl, pH 7.5) and treated in a homogenizer for 10 cycles. The product was centrifuged at 13000 rpm for 40 minutes. The supernatant was mixed with 150µl $Ni^{2+}$-resin (Pharmacia) (previously washed with buffer B) and incubated at room temperature with gentle agitation for 30 minutes. The sample was centrifuged at 700 g for 5 minutes at 4°C. The resin was washed twice with 10 ml buffer B for 10 minutes, resuspended in 1ml buffer B, and loaded on a disposable column. The resin was washed at room temperature with 2ml buffer B until the flow-through reached the $OD_{280}$ of 0.02-0.06. The resin was washed with 2ml buffer C (urea 8M, 10mM Tris-HCl, 100mM phosphate buffer, pH 6.3) until the flow-through reached the $O.D_{280}$ of 0.02-0.06. The His-fusion protein was eluted by addition of 700µl elution buffer (urea 8M, 10mM Tris-HCl, 100mM phosphate buffer, pH 4.5) and fractions collected until the $OD_{280}$ was 0.1. 21µl of each fraction were loaded on a 12% SDS gel.

**His-fusion proteins renaturation**

10% glycerol was added to the denatured proteins. The proteins were then diluted to 20µg/ml using dialysis buffer I (10% glycerol, 0.5M arginine, 50mM phosphate buffer, 5mM reduced glutathione, 0.5mM oxidised glutathione, 2M urea, pH 8.8) and dialysed against the same buffer at 4°C for 12-14 hours. The protein was further dialysed against dialysis buffer II (10% glycerol, 0.5M arginine, 50mM phosphate buffer, 5mM reduced glutathione, 0.5mM oxidised glutathione, pH 8.8) for 12-14 hours at 4°C. Protein concentration was evaluated using the formula:

$$\text{Protein (mg/ml)} = (1.55 \times OD_{280}) - (0.76 \times OD_{260})$$

**Mice immunisations**

20µg of each purified protein were used to immunise mice intraperitoneally. In the case of some ORFs, Balb-C mice were immunised with $Al(OH)_3$ as adjuvant on days 1, 21 and 42, and immune response was monitored in samples taken on day 56. For other ORFs, CD1 mice could be immunised using the same protocol. For other ORFs, CD1 mice could be immunised using Freund's adjuvant, and the same immunisation protocol was used, except that the immune response was measured on day 42, rather than 56. Similarly, for still other ORFs, CD1 mice could be immunised with Freund's adjuvant, but the immune response was measured on day 49.

**ELISA assay (sera analysis)**

The acapsulated MenB M7 strain was plated on chocolate agar plates and incubated overnight at 37°C. Bacterial colonies were collected from the agar plates using a sterile dracon swab and inoculated into 7ml of Mueller-Hinton Broth (Difco) containing 0.25% Glucose. Bacterial growth was monitored every 30 minutes by following $OD_{620}$. The bacteria were let to grow until the OD reached the value of 0.3-0.4. The culture was centrifuged for 10 minutes at 10000 rpm. The supernatant was discarded and bacteria were washed once with PBS, resuspended in PBS containing 0.025% formaldehyde, and incubated for 2 hours at room temperature and then overnight at 4°C with stirring. 100μl bacterial cells were added to each well of a 96 well Greiner plate and incubated overnight at 4°C. The wells were then washed three times with PBT washing buffer (0.1% Tween-20 in PBS). 200 μl of saturation buffer (2.7% Polyvinylpyrrolidone 10 in water) was added to each well and the plates incubated for 2 hours at 37°C. Wells were washed three times with PBT. 200 μl of diluted sera (Dilution buffer: 1% BSA, 0.1% Tween-20, 0.1% $NaN_3$ in PBS) were added to each well and the plates incubated for 90 minutes at 37°C. Wells were washed three times with PBT. 100 μl of HRP-conjugated rabbit anti-mouse (Dako) serum diluted 1:2000 in dilution buffer were added to each well and the plates were incubated for 90 minutes at 37°C. Wells were washed three times with PBT buffer. 100 μl of substrate buffer for HRP (25 ml of citrate buffer pH5, 10 mg of O-phenildiamine and 10 μl of $H_2O$) were added to each well and the plates were left at room temperature for 20 minutes. 100 μl $H_2SO_4$ was added to each well and $OD_{490}$ was followed. The ELISA was considered positive when OD490 was 2.5 times the respective pre-immune sera.

**FACScan bacteria Binding Assay procedure.**

The acapsulated MenB M7 strain was plated on chocolate agar plates and incubated overnight at 37°C. Bacterial colonies were collected from the agar plates using a sterile dracon swab and inoculated into 4 tubes containing 8ml each Mueller-Hinton Broth (Difco) containing 0.25% glucose. Bacterial growth was monitored every 30 minutes by following $OD_{620}$. The bacteria were let to grow until the OD reached the value of 0.35-0.5. The culture was centrifuged for 10 minutes at 4000 rpm. The supernatant was discarded and the pellet was resuspended in blocking buffer (1% BSA, 0.4% $NaN_3$) and centrifuged for 5 minutes at 4000 rpm. Cells were resuspended in blocking buffer to reach $OD_{620}$ of 0.07. 100μl bacterial cells were added to each well of a Costar 96 well plate. 100μl of diluted (1:200) sera (in blocking buffer) were added to each well and plates incubated for 2 hours at 4°C. Cells were centrifuged for 5 minutes at 4000 rpm, the supernatant aspirated and cells washed by addition of 200μl/well of blocking buffer in each well. 100μl of R-Phicoerytrin conjugated $F(ab)_2$ goat anti-mouse, diluted 1:100, was added to each well and plates incubated for 1 hour at 4°C. Cells were spun down by centrifugation at 4000rpm for 5 minutes and washed by addition of 200μl/well of blocking buffer. The supernatant was aspirated and cells resuspended in 200μl/well of PBS, 0.25% formaldehyde. Samples were transferred to FACScan tubes and read. The condition for FACScan setting were: FL1 on, FL2 and FL3 off; FSC-H Treshold:92; FSC PMT Voltage: E 02; SSC PMT: 474; Amp. Gains 7.1; FL-2 PMT: 539. Compensation values: 0.

**OMV preparations**

Bacteria were grown overnight on 5 GC plates, harvested with a loop and resuspended in 10 ml 20mM Tris-HCl. Heat inactivation was performed at 56°C for 30 minutes and the bacteria disrupted by sonication for 10' on ice ( 50% duty cycle, 50% output ). Unbroken cells were removed by centrifugation at 5000g for 10 minutes and the total cell envelope fraction recovered by centrifugation at 50000g at 4°C for 75 minutes. To extract cytoplasmic membrane proteins from the crude outer membranes, the whole fraction was resuspended in 2% sarkosyl (Sigma) and incubated at room temperature for 20 minutes. The suspension was centrifuged at 10000g for 10 minutes to remove aggregates, and the supernatant further ultracentrifuged at 50000g for 75 minutes to pellet the outer membranes. The outer membranes were resuspended in 10mM Tris-HCl, pH8 and the protein concentration measured by the Bio-Rad Protein assay, using BSA as a standard.

**Whole Extracts preparation**

Bacteria were grown overnight on a GC plate, harvested with a loop and resuspended in 1ml of 20mM Tris-HCl. Heat inactivation was performed at 56°C for 30' minutes.

**Western blotting**

Purified proteins (500ng/lane), outer membrane vesicles (5 μg) and total cell extracts (25μg) derived from MenB strain 2996 were loaded on 15% SDS-PAGE and transferred to a nitrocellulose membrane. The transfer was performed for 2 hours at 150mA at 4°C, in transferring buffer (0.3 % Tris base, 1.44 % glycine, 20% methanol). The membrane was saturated by overnight incubation at 4°C in saturation buffer (10% skimmed milk, 0.1% Triton X100 in PBS). The membrane was washed twice with washing buffer (3% skimmed milk, 0.1% Triton X100 in PBS) and incubated for 2 hours at 37°C with 1:200 mice sera diluted in washing buffer. The membrane was washed twice and incubated for 90 minutes with a 1:2000 dilution of horseradish peroxidase labeled anti-mouse Ig. The membrane was washed twice with 0.1 % Triton X100 in PBS and developed with the Opti-4CN Substrate Kit (Bio-Rad). The reaction was stopped by adding water.

**Bactericidal assay**

MC58 strain was grown overnight at 37°C on chocolate agar plates. 5-7 colonies were collected and used to inoculate 7ml Mueller-Hinton broth. The suspension was incubated at 37°C on a nutator and let to grow until $OD_{620}$ was in between 0.5-0.8. The culture was aliquoted into sterile 1.5ml Eppendorf tubes and centrifuged for 20 minutes at maximum speed in a microfuge. The pellet was washed once in Gey's buffer (Gibco) and resuspended in the same buffer to an $OD_{620}$ of 0.5, diluted 1:20000 in Gey's buffer and stored at 25°C.

50μl of Gey's buffer/1% BSA was added to each well of a 96-well tissue culture plate. 25μl of diluted (1:100) mice sera (dilution buffer: Gey's buffer/0.2% BSA) were added to each well and the plate incubated at 4°C. 25μl of the previously described bacterial suspension were added to each well. 25μl of either heat-inactivated (56°C waterbath for 30 minutes) or normal baby rabbit complement were added to each well. Immediately after the addition of the baby rabbit complement, 22μl of each sample/well were plated on Mueller-Hinton agar plates (time 0). The 96-well plate was incubated for 1 hour at 37°C with rotation and then 22μl of each sample/well were plated on Mueller-Hinton agar plates (time 1). After overnight incubation the colonies corresponding to time 0 and time 1h were counted.

The following DNA and amino acid sequences are identified by titles of the following form: [g, m, or a] [#].[seq or pep], where "g" means a sequence from *N. gonorrhoeae,* "m" means a sequence from *N. meningitidis B,* and "a" means a sequence from *N. meningitidis A;* "#" means the number of the sequence; "seq" means a DNA sequence, and "pep" means an amino acid sequence. For example, "g001.seq" refers to an *N. gonorrohoeae* DNA sequence, number 1. The presence of the suffix "-1" or "-2" to these sequences indicates an additional sequence found for the same ORF. Further, open reading frames are identified as ORF #, where "#" means the number of the ORF, corresponding to the number of the sequence which encodes the ORF, and the ORF designations may be suffixed with ". ng" or ".a", indicating that the ORF corresponds to a *N. gonorrhoeae* sequence or a *N. meningitidis A* sequence, respectively. Computer analysis was performed for the comparisons that follow between "g", "m", and "a" peptide sequences; and therein the "pep" suffix is implied where not expressly stated.

EXAMPLE 1

The following ORFs were predicted from the contig sequences and/or the full length sequences using the methods herein described.

**Localization of the ORFs**

| ORF: | contig: |
|---|---|
| 279 | gnm4.seq |

The following partial DNA sequence was identified in *N. meningitidis* <SEQ ID 2>: m279.seq

```
  1  ATAACGCGGA TTTGCGGCTG CTTGATTTCA ACGGTTTTCA GGGCTTCGGC
 51  AAGTTTGTCG GCGGCGGGTT TCATCAGGCT GCAATGGGAA GGTACGGACA
101  CGGGCAGCGG CAGGGCGCGT TTGGCACCGG CTTCTTTGGC GGCAGCCATG
151  GCGCGTCCGA CGGCGGCGGC GTTGCCTGCA ATCACGATTT GTCCGGGTGA
201  GTTGAAGTTG ACGGCTTCGA CCACTTCGCT TTGGGCGGCT TCGGCACAAA
251  TGGCTTTAAC CTGCTCATCT TCCAAGCCGA GAATCGCCGC CATTGCGCCC
301  ACGCCTTGCG GTACGGCGGA CTGCATCAGT TCGGCGCGCA GGCGCACGAG
351  TTTGACCGCG TCGGCAAAAT TCAATGCGCC GGCGGCAACG AGTGCGGTGT
401  ATTCGCCGAG GCTGTGTCCG GCAACGGCGG CAGGCGTTTT GCCGCCCGCT
451  TCTAAATAG
```

This corresponds to the amino acid sequence <SEQ ID 3; ORF 279>:

m279.pep

```
  1  ITRICGCLIS TVFRASASLS AAGFIRLQWE GTDTGSGRAR LAPASLAAAM
 51  ARPTAAALPA ITICPGELKL TASTTSLWAA SAQMALTCSS SKPRIAAIAP
101  TPCGTADCIS SARRRTSLTA SAKFNAPAAT SAVYSPRLCP ATAAGVLPPA
151  SK*
```

The following partial DNA sequence was identified in *N.gonorrhoeae* <SEQ ID 4>:

```
g279.seq
  1  atgacgcgga tttgcggctg cttgatttca acggttttga gtgtttcggc
 51  aagtttgtcg gcggcgggtt tcatcaggct gcaatgggaa ggaacggata
101  ccggcagcgg cagggcgcgt ttggctccgg cttctttggc ggcagccatg
151  gtgcgtccga cggcggcggc gttgcctgca atcacgactt gtccgggcga
201  gttgaagttg acggcttcga ccacttcgcc ctgtgcggat tcggcacaaa
251  tctgcctgac ctgttcatct tccaaaccca aaatggccgc cattgcgcct
301  acgccttgcg gtacggcgga ctgcatcagt tcggcgcgca ggcggacgag
351  tttgacggca tcggcaaaat ccaatgcttc ggcggcgaca agcgcggtgt
401  attcgccgag gctgtgtccg gcaacggcgg caggcgtttt gccgcccact
451  tccaaatag
```

This corresponds to the amino acid sequence <SEQ ID 5; ORF 279.ng>:

```
g279.pep
  1  MTRICGCLIS TVLSVSASLS AAGFIRLQWE GTDTGSGRAR LAPASLAAAM
 51  VRPTAAALPA ITTCPGELKL TASTTSPCAD SAQICLTCSS SKPKMAAIAP
101  TPCGTADCIS SARRRTSLTA SAKSNASAAT SAVYSPRLCP ATAAGVLPPT
151  SK*
```

ORF 279 shows 89.5% identity over a 152 aa overlap with a predicted ORF (ORF 279.ng) from *N. gonorrhoeae:*

```
                    10        20        30        40        50        60
m279.pep    ITRICGCLISTVFRASASLSAAGFIRLQWEGTDTGSGRARLAPASLAAAMARPTAAALPA
            :||||||||||: :||||||||||||||||||||||||||||||||||||:|||||||||
g279        MTRICGCLISTVLSVSASLSAAGFIRLQWEGTDTGSGRARLAPASLAAAMVRPTAAALPA
                    10        20        30        40        50        60

                    70        80        90       100       110       120
m279.pep    ITICPGELKLTASTTSLWAASAQMALTCSSSKPRIAAIAPTPCGTADCISSARRRTSLTA
            || ||||||||||||| | |||: |||||||::|||||||||||||||||||||||||||
g279        ITTCPGELKLTASTTSPCADSAQICLTCSSSKPKMAAIAPTPCGTADCISSARRRTSLTA
                    70        80        90       100       110       120

                   130       140       150
m279.pep    SAKFNAPAATSAVYSPRLCPATAAGVLPPASKX
            ||| || ||||||||||||||||||||||:|||
g279        SAKSNASAATSAVYSPRLCPATAAGVLPPTSKX
                   130       140       150
```

The following partial DNA sequence was identified in *N. meningitidis* <SEQ ID 6>:

```
a279.seq
  1  ATGACNCNGA TTTGCGGCTG CTTGATTTCA ACGGTTTNNA GGGCTTCGGC
 51  GAGTTTGTCG GCGGCGGGTT TCATGAGGCT GCAATGGGAA GGTACNGACA
101  CNGGCAGCGG CAGGGCGCGT TTGGCGCCGG CTTCTTTGGC GGCAAGCATA
151  GCGCGCTCGA CGGCGGCGGC ATTGCCTGCA ATCACGACTT GTCCGGGCGA
201  GTTGAAGTTG ACGGCTTCAA CCACTTCATC CTGTGCGGAT TCGGCGCAAA
251  TTTGTTTTAC CTGTTCATCT TCCAAGCCGA GAATCGCCGC CATTGCGCCC
301  ACGCCTTGCG GTACGGCGGA CTGCATCAGT TCGGCGCGCA NGCGCACGAG
351  TTTGACCGCG TCGGCAAAAT CCAATGCGCC GGCGGCAACN AGTGCGGTGT
```

```
401  ATTCGCCGAN GCTGTGTCCG GCAACGGCGG CAGGCGTTTT GCCGCCCGCT
451  TCCGAATAG
```

This corresponds to the amino acid sequence <SEQ ID 7; ORF 279.a>:

```
a279.pep
  1  MTXICGCLIS TVXRASASLS AAGFMRLQWE GTDTGSGRAR LAPASLAASI
 51  ARSTAAALPA ITTCPGELKL TASTTSSCAD SAQICFTCSS SKPRIAAIAP
101  TPCGTADCIS SARXRTSLTA SAKSNAPAAT SAVYSPXLCP ATAAGVLPPA
151  SE*
```

m279/a279 ORFs 279 and 279.a showed a 88.2% identity in 152 aa overlap

```
                     10        20        30        40        50        60
m279.pep    ITRICGCLISTVFRASASLSAAGFIRLQWEGTDTGSGRARLAPASLAAAMARPTAAALPA
            :| ||||||||| ||||||||||:||||||||||||||||||||||::|| |||||||
a279        MTXICGCLISTVXRASASLSAAGFMRLQWEGTDTGSGRARLAPASLAASIARSTAAALPA
                     10        20        30        40        50        60

                     70        80        90       100       110       120
m279.pep    ITICPGELKLTASTTSLWAASAQMALTCSSSKPRIAAIAPTPCGTADCISSARRRTSLTA
            || ||||||||||||| | |||: :|||||||||||||||||||||||||||| ||||||
a279        ITTCPGELKLTASTTSSCADSAQICFTCSSSKPRIAAIAPTPCGTADCISSARXRTSLTA
                     70        80        90       100       110       120

                    130       140       150
m279.pep    SAKFNAPAATSAVYSPRLCPATAAGVLPPASKX
            ||| |||||||||||| ||||||||||||||:|
a279        SAKSNAPAATSAVYSPXLCPATAAGVLPPASEX
                    130       140       150
```

519 and 519-1 gnm7.seq

The following partial DNA sequence was identified in *N. meningitidis* <SEQ ID 8>:

```
m519.seq  (partial)
  1  ..TCCGTTATCG GGCGTATGGA GTTGGACAAA ACGTTTGAAG AACGCGACGA
 51    AATCAACAGT ACTGTTGTTG CGGCTTTGGA CGAGGCGGCC GGGgCTTgGG
101    GTGTGAAGGT TTTGCGTTAT GAGATTAAAG ACTTGGTTCC GCCGCAAGAA
151    ATCCTTCGCT CAATGCAGGC GCAAATTACT GCCGAACGCG AAAAACGCGC
201    CCGTATCGCC GAATCCGAAG GTCGTAAAAT CGAACAAATC AACCTTGCCA
251    GTGGTCAGCG CGAAGCCGAA ATCCAACAAT CCGAAGGCGA GGCTCAGGCT
301    GCGGTCAATG CGTCAAATGC CGAGAAAATC GCCCGCATCA ACCGCGCCAA
351    AGGTGAAGCG GAATCCTTGC GCCTTGTTGC CGAAGCCAAT GCCGAAGCCA
401    TCCGTCAAAT TGCCGCCGCC CTTCAAACCC AAGGCGGTGC GGATGCGGTC
451    AATCTGAAGA TTGCGGAACA ATACGTCGCT GCGTTCAACA ATCTTGCCAA
501    AGAAAGCAAT ACGCTGATTA TGCCCGCCAA TGTTGCCGAC ATCGGCAGCC
551    TGATTTCTGC CGGTATGAAA ATTATCGACA GCAGCAAAAC CGCCAAaTAA
```

This corresponds to the amino acid sequence <SEQ ID 9; ORF 519>:

```
m519.pep  (partial)
  1  ..SVIGRMELDK TFEERDEINS TVVAALDEAA GAWGVKVLRY EIKDLVPPQE
 51    ILRSMQAQIT AEREKRARIA ESEGRKIEQI NLASGQREAE IQQSEGEAQA
101    AVNASNAEKI ARINRAKGEA ESLRLVAEAN AEAIRQIAAA LQTQGGADAV
151    NLKIAEQYVA AFNNLAKESN TLIMPANVAD IGSLISAGMK IIDSSKTAK*
```

The following partial DNA sequence was identified in *N. gonorrhoeae* <SEQ ID 10>:

```
g519.seq
      1  atggaatttt tcattatctt gttggcagcc gtcgccgttt tcggcttcaa
     51  atcctttgtc gtcatccccc agcaggaagt ccacgttgtc gaaaggctcg
    101  ggcgtttcca tcgcgccctg acggccggtt tgaatatttt gattcccttt
    151  atcgaccgcg tcgcctaccg ccattcgctg aaagaaatcc ctttagacgt
    201  acccagccag gtctgcatca cgcgcgataa tacgcaattg actgttgacg
    251  gcatcatcta tttccaagta accgatccca aactcgcctc atacggttcg
    301  agcaactaca ttatggcaat tacccagctt gcccaaacga cgctgcgttc
    351  cgttatcggg cgtatggagt tggacaaaac gtttgaagaa cgcgacgaaa
    401  tcaacagtac cgtcgtctcc gccctcgatg aagccgccgg ggcttggggt
    451  gtgaaagtcc tccgttacga aatcaaggat ttggttccgc cgcaagaaat
    501  ccttcgcgca atgcaggcac aaattaccgc cgaacgcgaa aaacgcgccc
    551  gtattgccga atccgaaggc cgtaaaatcg aacaaatcaa ccttgccagt
    601  ggtcagcgtg aagccgaaat ccaacaatcc gaaggcgagg ctcaggctgc
    651  ggtcaatgcg tccaatgccg agaaaatcgc ccgcatcaac cgcgccaaag
    701  gcgaagcgga atccctgcgc cttgttgccg aagccaatgc cgaagccaac
    751  cgtcaaattg ccgccgccct tcaaacccaa agcggggcgg atgcggtcaa
    801  tctgaagatt gcgggacaat acgttaccgc gttcaaaaat cttgccaaag
    851  aagacaatac gcggattaag cccgccaagg ttgccgaaat cgggaaccct
    901  aattttcggc ggcatgaaaa attttcgcca gaagcaaaaa cggccaaata
    951  a
```

This corresponds to the amino acid sequence <SEQ ID 11; ORF 519.ng>:

```
g519.pep
      1  MEFFIILLAA VAVFGFKSFV VIPQQEVHVV ERLGRFHRAL TAGLNILIPF
     51  IDRVAYRHSL KEIPLDVPSQ VCITRDNTQL TVDGIIYFQV TDPKLASYGS
    101  SNYIMAITQL AQTTLRSVIG RMELDKTFEE RDEINSTVVS ALDEAAGAWG
    151  VKVLRYEIKD LVPPQEILRA MQAQITAERE KRARIAESEG RKIEQINLAS
    201  GQREAEIQQS EGEAQAAVNA SNAEKIARIN RAKGEAESLR LVAEANAEAN
    251  RQIAAALQTQ SGADAVNLKI AGQYVTAFKN LAKEDNTRIK PAKVAEIGNP
    301  NFRRHEKFSP EAKTAK*
```

ORF 519 shows 87.5% identity over a 200 aa overlap with a predicted ORF (ORF 519.ng) from *N. gonorrhoeae:*

```
m519/g519

                                                  10        20        30
m519.pep                                 SVIGRMELDKTFEERDEINSTVVAALDEAA
                                         ||||||||||||||||||||||:||||||
g519          YFQVTDPKLASYGSSNYIMAITQLAQTTLRSVIGRMELDKTFEERDEINSTVVSALDEAA
               90       100       110       120       130       140

                 40        50        60        70        80        90
m519.pep      GAWGVKVLRYEIKDLVPPQEILRSMQAQITAEREKRARIAESEGRKIEQINLASGQREAE
              ||||||||||||||||||||||:|||||||||||||||||||||||||||||||||||||
g519          GAWGVKVLRYEIKDLVPPQEILRAMQAQITAEREKRARIAESEGRKIEQINLASGQREAE
               150       160       170       180       190       200

                100       110       120       130       140       150
m519.pep      IQQSEGEAQAAVNASNAEKIARINRAKGEAESLRLVAEANAEAIRQIAAALQTQGGADAV
              |||||||||||||||||||||||||||||||||||||||| ||||||||||:|||||
g519          IQQSEGEAQAAVNASNAEKIARINRAKGEAESLRLVAEANAEANRQIAAALQTQSGADAV
               210       220       230       240       250       260

                160       170       180       190       200
m519.pep      NLKIAEQYVAAFNNLAKESNTLIMPANVADIGSL-ISAGMKIIDSSKTAK
              ||||| |||:||:|||||:|| | ||:||:||:  :    |:   :||||
g519          NLKIAGQYVTAFKNLAKEDNTRIKPAKVAEIGNPNFRRHEKFSPEAKTAK
               270       280       290       300       310
```

The following partial DNA sequence was identified in *N. meningitidis* <SEQ ID 12>:

```
                              a519.seq

  1   ATGGAATTTT TCATTATCTT GCTGGCAGCC GTCGTTGTTT TCGGCTTCAA
 51   ATCCTTTGTT GTCATCCCAC AGCAGGAAGT CCACGTTGTC GAAAGGCTCG
101   GGCGTTTCCA TCGCGCCCTG ACGGCCGGTT TGAATATTTT GATTCCCTTT
151   ATCGACCGCG TCGCCTACCG CCATTCGCTG AAAGAAATCC CTTTAGACGT
201   ACCCAGCCAG GTCTGCATCA CGCGCGACAA TACGCAGCTG ACTGTTGACG
251   GTATCATCTA TTTCCAAGTA ACCGACCCCA AACTCGCCTC ATACGGTTCG
301   AGCAACTACA TTATGGCGAT TACCCAGCTT GCCCAAACGA CGCTGCGTTC
351   CGTTATCGGG CGTATGGAAT TGGACAAAAC GTTTGAAGAA CGCGACGAAA
401   TCAACAGCAC CGTCGTCTCC GCCCTCGATG AAGCCGCCGG AGCTTGGGGT
451   GTGAAGGTTT TGCGTTATGA GATTAAAGAC TTGGTTCCGC CGCAAGAAAT
501   CCTTCGCTCA ATGCAGGCGC AAATTACTGC TGAACGCGAA AAACGCGCCC
551   GTATCGCCGA ATCCGAAGGT CGTAAAATCG AACAAATCAA CCTTGCCAGT
601   GGTCAGCGCG AAGCCGAAAT CCAACAATCC GAAGGCGAGG CTCAGGCTGC
651   GGTCAATGCG TCAAATGCCG AGAAAATCGC CCGCATCAAC CGCGCCAAAG
701   GTGAAGCGGA ATCCTTGCGC CTTGTTGCCG AAGCCAATGC CGAAGCCATC
751   CGTCAAATTG CCGCCGCCCT TCAAACCCAA GGCGGTGCGG ATGCGGTCAA
801   TCTGAAGATT GCGGAACAAT ACGTCGCCGC GTTCAACAAT CTTGCCAAAG
851   AAAGCAATAC GCTGATTATG CCCGCCAATG TTGCCGACAT CGGCAGCCTG
901   ATTTCTGCCG GTATGAAAAT TATCGACAGC AGCAAAACCG CCAAATAA
```

This corresponds to the amino acid sequence <SEQ ID 13; ORF 519.a>:

```
a519.pep
      1  MEFFIILLAA VVVFGFKSFV VIPQQEVHVV ERLGRFHRAL TAGLNILIPF
     51  IDRVAYRHSL KEIPLDVPSQ VCITRDNTQL TVDGIIYFQV TDPKLASYGS
    101  SNYIMAITQL AQTTLRSVIG RMELDKTFEE RDEINSTVVS ALDEAAGAWG
    151  VKVLRYEIKD LVPPQEILRS MQAQITAERE KRARIAESEG RKIEQINLAS
    201  GQREAEIQQS EGEAQAAVNA SNAEKIARIN RAKGEAESLR LVAEANAEAI
    251  RQIAAALQTQ GGADAVNLKI AEQYVAAFNN LAKESNTLIM PANVADIGSL
    301  ISAGMKIIDS SKTAK*
```

**m519/a519** **ORFs 519 and 519.a showed a** 99.5% identity in 199 aa overlap

```
                                                            10        20        30
m519.pep                                         SVIGRMELDKTFEERDEINSTVVAALDEAA
                                                 ||||||||||||||||||||||:||||||
a519            YFQVTDPKLASYGSSNYIMAITQLAQTTLRSVIGRMELDKTFEERDEINSTVVSALDEAA
                  90       100       110       120       130       140

                          40        50        60        70        80        90
m519.pep        GAWGVKVLRYEIKDLVPPQEILRSMQAQITAEREKRARIAESEGRKIEQINLASGQREAE
                ||||||||||||||||||||||||||||||||||||||||||||||||||||||||||||
a519            GAWGVKVLRYEIKDLVPPQEILRSMQAQITAEREKRARIAESEGRKIEQINLASGQREAE
                 150       160       170       180       190       200

                         100       110       120       130       140       150
m519.pep        IQQSEGEAQAAVNASNAEKIARINRAKGEAESLRLVAEANAEAIRQIAAALQTQGGADAV
                ||||||||||||||||||||||||||||||||||||||||||||||||||||||||||||
a519            IQQSEGEAQAAVNASNAEKIARINRAKGEAESLRLVAEANAEAIRQIAAALQTQGGADAV
                 210       220       230       240       250       260

                         160       170       180       190       200
m519.pep        NLKIAEQYVAAFNNLAKESNTLIMPANVADIGSLISAGMKIIDSSKTAKX
                ||||||||||||||||||||||||||||||||||||||||||||||||||
a519            NLKIAEQYVAAFNNLAKESNTLIMPANVADIGSLISAGMKIIDSSKTAKX
                 270       280       290       300       310
```

Further work revealed the following DNA sequence identified in *N. meningitidis* <SEQ ID 14>:

m519-1.seq

```
  1 ATGGAATTTT TCATTATCTT GTTGGTAGCC GTCGCCGTTT TCGGTTTCAA
 51 ATCCTTTGTT GTCATCCCAC AACAGGAAGT CCACGTTGTC GAAAGGCTGG
101 GGCGTTTCCA TCGCGCCCTG ACGGcCGGTT TGAATATTTT GATTCCCTTT
151 ATCGACCGCG TCGCCTACCG CCATTCGCTG AAAGAAATCC CTTTAGACGT
201 ACCCAGCCAG GTCTGCATCA CGCGCGACAA TACGCAGCTG ACTGTTGACG
251 GCATCATCTA TTTCCAAGTA ACCGACCCCA AACTCGCCTC ATACGGTTCG
301 AGCAACTACA TTATGGCGAT TACCCAGCTT GCCCAAACGA CGCTGCGTTC
351 CGTTATCGGG CGTATGGAGT TGGACAAAAC GTTTGAAGAA CGCGACGAAA
401 TCAACAGTAC TGTTGTTGCG GCTTTGGACG AGGCGGCCGG GGCTTGGGGT
451 GTGAAGGTTT TGCGTTATGA GATTAAAGAC TTGGTTCCGC CGCAAGAAAT
501 CCTTCGCTCA ATGCAGGCGC AAATTACTGC CGAACGCGAA AAACGCGCCC
551 GTATCGCCGA ATCCGAAGGT CGTAAAATCG AACAAATCAA CCTTGCCAGT
601 GGTCAGCGCG AAGCCGAAAT CCAACAATCC GAAGGCGAGG CTCAGGCTGC
651 GGTCAATGCG TCAAATGCCG AGAAAATCGC CCGCATCAAC CGCGCCAAAG
701 GTGAAGCGGA ATCCTTGCGC CTTGTTGCCG AAGCCAATGC CGAAGCCATC
751 CGTCAAATTG CCGCCGCCCT TCAAACCCAA GGCGGTGCGG ATGCGGTCAA
801 TCTGAAGATT GCGGAACAAT ACGTCGCTGC GTTCAACAAT CTTGCCAAAG
851 AAAGCAATAC GCTGATTATG CCCGCCAATG TTGCCGACAT CGGCAGCCTG
901 ATTTCTGCCG GTATGAAAAT TATCGACAGC AGCAAAACCG CCAAATAA
```

This corresponds to the amino acid sequence <SEQ ID 15; ORF 519-1>:

```
m519-1.
  1  MEFFIILLVA VAVFGFKSFV VIPQQEVHVV ERLGRFHRAL TAGLNILIPF
 51  IDRVAYRHSL KEIPLDVPSQ VCITRDNTQL TVDGIIYFQV TDPKLASYGS
101  SNYIMAITQL AQTTLRSVIG RMELDKTFEE RDEINSTVVA ALDEAAGAWG
151  VKVLRYEIKD LVPPQEILRS MQAQITAERE KRARIAESEG RKIEQINLAS
201  GQREAEIQQS EGEAQAAVNA SNAEKIARIN RAKGEAESLR LVAEANAEAI
251  RQIAAALQTQ GGADAVNLKI AEQYVAAFNN LAKESNTLIM PANVADIGSL
301  ISAGMKIIDS SKTAK*
```

The following DNA sequence was identified in *N. gonorrhoeae* <SEO ID 16>:

```
g519-1.seq
  1  ATGGAATTTT TCATTATCTT GTTGGCAGCC GTCGCCGTTT TCGGCTTCAA
 51  ATCCTTTGTC GTCATCCCCC AGCAGGAAGT CCACGTTGTC GAAAGGCTCG
101  GGCGTTTCCA TCGCGCCCTG ACGGCCGGTT TGAATATTTT GATTCCCTTT
151  ATCGACCGCG TCGCCTACCG CCATTCGCTG AAAGAAATCC CTTTAGACGT
201  ACCCAGCCAG GTCTGCATCA CGCGCGATAA TACGCAATTG ACTGTTGACG
251  GCATCATCTA TTTCCAAGTA ACCGATCCCA AACTCGCCTC ATACGGTTCG
301  AGCAACTACA TTATGGCAAT TACCCAGCTT GCCCAAACGA CGCTGCGTTC
351  CGTTATCGGG CGTATGGAGT TGGACAAAAC GTTTGAAGAA CGCGACGAAA
401  TCAACAGTAC CGTCGTCTCC GCCCTCGATG AAGCCGCCGG GGCTTGGGGT
451  GTGAAAGTCC TCCGTTACGA AATCAAGGAT TTGGTTCCGC CGCAAGAAAT
501  CCTTCGCGCA ATGCAGGCAC AAATTACCGC CGAACGCGAA AAACGCGCCC
551  GTATTGCCGA ATCCGAAGGC CGTAAAATCG AACAAATCAA CCTTGCCAGT
601  GGTCAGCGTG AAGCCGAAAT CCAACAATCC GAAGGCGAGG CTCAGGCTGC
651  GGTCAATGCG TCCAATGCCG AGAAAATCGC CCGCATCAAC CGCGCCAAAG
701  GCGAAGCGGA ATCCCTGCGC CTTGTTGCCG AAGCCAATGC CGAAGCCATC
751  CGTCAAATTG CCGCCGCCCT TCAAACCCAA GGCGGGGCGG ATGCGGTCAA
801  TCTGAAGATT GCGGAACAAT ACGTAGCCGC GTTCAACAAT CTTGCCAAAG
851  AAAGCAATAC GCTGATTATG CCCGCCAATG TTGCCGACAT CGGCAGCCTG
901  ATTTCTGCCG GCATGAAAAT TATCGACAGC AGCAAAACCG CCAAATAA
```

This corresponds to the amino acid sequence <SEQ ID 17; ORF 519-1.ng>:

```
g519-1.pep
  1  MEFFIILLAA VAVFGFKSFV VIPQQEVHVV ERLGRFHRAL TAGLNILIPF
 51  IDRVAYRHSL KEIPLDVPSQ VCITRDNTQL TVDGIIYFQV TDPKLASYGS
101  SNYIMAITQL AQTTLRSVIG RMELDKTFEE RDEINSTVVS ALDEAAGAWG
151  VKVLRYEIKD LVPPQEILRA MQAQITAERE KRARIAESEG RKIEQINLAS
201  GQREAEIQQS EGEAQAAVNA SNAEKIARIN RAKGEAESLR LVAEANAEAI
251  RQIAAALQTQ GGADAVNLKI AEQYVAAFNN LAKESNTLIM PANVADIGSL
301  ISAGMKIIDS SKTAK*
```

**m519-1/g519-1 ORFs 519-1 and 519-1.ng showed a** 99.0% identity in 315 aa overlap

```
                   10        20        30        40        50        60
g519-1.pep  MEFFIILLAAVAVFGFKSFVVIPQQEVHVVERLGRFHRALTAGLNILIPFIDRVAYRHSL
            ||||||||:|||||||||||||||||||||||||||||||||||||||||||||||||||
m519-1      MEFFIILLVAVAVFGFKSFVVIPQQEVHVVERLGRFHRALTAGLNILIPFIDRVAYRHSL
                   10        20        30        40        50        60

                   70        80        90       100       110       120
g519-1.pep  KEIPLDVPSQVCITRDNTQLTVDGIIYFQVTDPKLASYGSSNYIMAITQLAQTTLRSVIG
            ||||||||||||||||||||||||||||||||||||||||||||||||||||||||||||
m519-1      KEIPLDVPSQVCITRDNTQLTVDGIIYFQVTDPKLASYGSSNYIMAITQLAQTTLRSVIG
                   70        80        90       100       110       120

                  130       140       150       160       170       180
g519-1.pep  RMELDKTFEERDEINSTVVSALDEAAGAWGVKVLRYEIKDLVPPQEILRAMQAQITAERE
            |||||||||||||||||||:||||||||||||||||||||||||||||||:|||||||||
m519-1      RMELDKTFEERDEINSTVVAALDEAAGAWGVKVLRYEIKDLVPPQEILRSMQAQITAERE
                  130       140       150       160       170       180

                  190       200       210       220       230       240
g519-1.pep  KRARIAESEGRKIEQINLASGQREAEIQQSEGEAQAAVNASNAEKIARINRAKGEAESLR
            ||||||||||||||||||||||||||||||||||||||||||||||||||||||||||||
m519-1      KRARIAESEGRKIEQINLASGQREAEIQQSEGEAQAAVNASNAEKIARINRAKGEAESLR
                  190       200       210       220       230       240

                  250       260       270       280       290       300
g519-1.pep  LVAEANAEAIRQIAAALQTQGGADAVNLKIAEQYVAAFNNLAKESNTLIMPANVADIGSL
            ||||||||||||||||||||||||||||||||||||||||||||||||||||||||||||
m519-1      LVAEANAEAIRQIAAALQTQGGADAVNLKIAEQYVAAFNNLAKESNTLIMPANVADIGSL
                  250       260       270       280       290       300

                  310
g519-1.pep  ISAGMKIIDSSKTAKX
            ||||||||||||||||
m519-1      ISAGMKIIDSSKTAKX
                  310
```

The following DNA sequence was identified in *N. meningitidis* <SEQ ID 18>:

```
a519-1.seq
      1  ATGGAATTTT TCATTATCTT GCTGGCAGCC GTCGTTGTTT TCGGCTTCAA
     51  ATCCTTTGTT GTCATCCCAC AGCAGGAAGT CCACGTTGTC GAAAGGCTCG
    101  GGCGTTTCCA TCGCGCCCTG ACGGCCGGTT TGAATATTTT GATTCCCTTT
    151  ATCGACCGCG TCGCCTACCG CCATTCGCTG AAAGAAATCC CTTTAGACGT
    201  ACCCAGCCAG GTCTGCATCA CGCGCGACAA TACGCAGCTG ACTGTTGACG
    251  GTATCATCTA TTTCCAAGTA ACCGACCCCA AACTCGCCTC ATACGGTTCG
    301  AGCAACTACA TTATGGCGAT TACCCAGCTT GCCCAAACGA CGCTGCGTTC
    351  CGTTATCGGG CGTATGGAAT TGGACAAAAC GTTTGAAGAA CGCGACGAAA
    401  TCAACAGCAC CGTCGTCTCC GCCCTCGATG AAGCCGCCGG AGCTTGGGGT
    451  GTGAAGGTTT TGCGTTATGA GATTAAAGAC TTGGTTCCGC CGCAAGAAAT
    501  CCTTCGCTCA ATGCAGGCGC AAATTACTGC TGAACGCGAA AAACGCGCCC
    551  GTATCGCCGA ATCCGAAGGT CGTAAAATCG AACAAATCAA CCTTGCCAGT
    601  GGTCAGCGCG AAGCCGAAAT CCAACAATCC GAAGGCGAGG CTCAGGCTGC
    651  GGTCAATGCG TCAAATGCCG AGAAAATCGC CCGCATCAAC CGCGCCAAAG
    701  GTGAAGCGGA ATCCTTGCGC CTTGTTGCCG AAGCCAATGC CGAAGCCATC
    751  CGTCAAATTG CCGCCGCCCT TCAAACCCAA GGCGGTGCGG ATGCGGTCAA
    801  TCTGAAGATT GCGGAACAAT ACGTCGCCGC GTTCAACAAT CTTGCCAAAG
    851  AAAGCAATAC GCTGATTATG CCCGCCAATG TTGCCGACAT CGGCAGCCTG
    901  ATTTCTGCCG GTATGAAAAT TATCGACAGC AGCAAAACCG CCAAATAA
```

This corresponds to the amino acid sequence <SEQ ID 19; ORF 519-1.a>:

```
a519-1.pep.
      1  MEFFIILLAA VVVFGFKSFV VIPQQEVHVV ERLGRFHRAL TAGLNILIPF
     51  IDRVAYRHSL KEIPLDVPSQ VCITRDNTQL TVDGIIYFQV TDPKLASYGS
    101  SNYIMAITQL AQTTLRSVIG RMELDKTFEE RDEINSTVVS ALDEAAGAWG
    151  VKVLRYEIKD LVPPQEILRS MQAQITAERE KRARIAESEG RKIEQINLAS
    201  GQREAEIQQS EGEAQAAVNA SNAEKIARIN RAKGEAESLR LVAEANAEAI
    251  RQIAAALQTQ GGADAVNLKI AEQYVAAFNN LAKESNTLIM PANVADIGSL
    301  ISAGMKIIDS SKTAK*
```

**m519-1/a519-1** ORFs 519-1 and 519-1.a showed a 99.0% identity in 315 aa overlap

```
                       10        20        30        40        50        60
a519-1.pep     MEFFIILLAAVVVFGFKSFVVIPQQEVHVVERLGRFHRALTAGLNILIPFIDRVAYRHSL
               ||||||||:||:||||||||||||||||||||||||||||||||||||||||||||||||
m519-1         MEFFIILLVAVAVFGFKSFVVIPQQEVHVVERLGRFHRALTAGLNILIPFIDRVAYRHSL
                       10        20        30        40        50        60

                       70        80        90       100       110       120
a519-1.pep     KEIPLDVPSQVCITRDNTQLTVDGIIYFQVTDPKLASYGSSNYIMAITQLAQTTLRSVIG
               ||||||||||||||||||||||||||||||||||||||||||||||||||||||||||||
m519-1         KEIPLDVPSQVCITRDNTQLTVDGIIYFQVTDPKLASYGSSNYIMAITQLAQTTLRSVIG
                       70        80        90       100       110       120

                      130       140       150       160       170       180
a519-1.pep     RMELDKTFEERDEINSTVVSALDEAAGAWGVKVLRYEIKDLVPPQEILRSMQAQITAERE
               |||||||||||||||||||:||||||||||||||||||||||||||||||||||||||||
m519-1         RMELDKTFEERDEINSTVVAALDEAAGAWGVKVLRYEIKDLVPPQEILRSMQAQITAERE
                      130       140       150       160       170       180

                      190       200       210       220       230       240
a519-1.pep     KRARIAESEGRKIEQINLASGQREAEIQQSEGEAQAAVNASNAEKIARINRAKGEAESLR
               ||||||||||||||||||||||||||||||||||||||||||||||||||||||||||||
m519-1         KRARIAESEGRKIEQINLASGQREAEIQQSEGEAQAAVNASNAEKIARINRAKGEAESLR
                      190       200       210       220       230       240

                      250       260       270       280       290       300
a519-1.pep     LVAEANAEAIRQIAAALQTQGGADAVNLKIAEQYVAAFNNLAKESNTLIMPANVADIGSL
               ||||||||||||||||||||||||||||||||||||||||||||||||||||||||||||
m519-1         LVAEANAEAIRQIAAALQTQGGADAVNLKIAEQYVAAFNNLAKESNTLIMPANVADIGSL
                      250       260       270       280       290       300

                      310
a519-1.pep     ISAGMKIIDSSKTAKX
               ||||||||||||||||
m519-1         ISAGMKIIDSSKTAKX
                      310
```

## 576 and 576-1 gnm22.seq

The following partial DNA sequence was identified in *N. meningitidis* <SEQ ID 20>:

```
m576.seq.. (partial)
      1  ..ATGCAGCAGG CAAGCTATGC GATGGGCGTG GACATCGGAC GCTCCCTGAA
     51    GCAAATGAAG GAACAGGGCG CGGAAATCGA TTTGAAAGTC TTTACCGAAG
    101    CCATGCAGGC AGTGTATGAC GGCAAAGAAA TCAAAATGAC CGAAGAGCAG
    151    GCTCAGGAAG TCATGATGAA ATTCCTTCAG GAACAACAGG CTAAAGCCGT
    201    AGAAAAACAC AAGGCGGACG CGAAGGCCAA TAAAGAAAAA GGCGAAGCCT
```

```
251     TTCTGAAAGA AAATGCCGCC AAAGACGGCG TGAAGACCAC TGCTTCCGGC
301     CTGCAATACA AAATCACCAA ACAGGGCGAA GGCAAACAGC CGACCAAAGA
351     CGACATCGTT ACCGTGGAAT ACGAAGGCCG CCTGATTGAC GGTACGGTAT
401     TCGACAGCAG CAAAGCCAAC GGCGGCCCGG TCACCTTCCC TTTGAGCCAA
451     GTGATTCCGG GTTGGACCGA AGgCGTACAG CTTCTGAAAG AAGGCGGCGA
501     AGCCACGTTC TACATCCCGT CCAACCTTGC CTACCGCGAA CAGGGTGCGG
551     GCGACAAAAT CGGTCCGAAC GCCACTTTGG TATTTGATGT GAAACTGGTC
601     AAAATCGGCG CACCCGAAAA CGCGCCCGCC AAGCAGCCGG CTCAAGTCGA
651     CATCAAAAAA GTAAATTAA
```

This corresponds to the amino acid sequence <SEQ ID 21; ORF 576>:

```
m576.pep.. (partial)
  1  ..MQQASYAMGV DIGRSLKQMK EQGAEIDLKV FTEAMQAVYD GKEIKMTEEQ
 51    AQEVMMKFLQ EQQAKAVEKH KADAKANKEK GEAFLKENAA KDGVKTTASG
101    LQYKITKQGE GKQPTKDDIV TVEYEGRLID GTVFDSSKAN GGPVTFPLSQ
151    VIPGWTEGVQ LLKEGGEATF YIPSNLAYRE QGAGDKIGPN ATLVFDVKLV
201    KIGAPENAPA KQPAQVDIKK VN*
```

The following partial DNA sequence was identified in *N. gonorrhoeae* <SEQ ID 22>:

```
g576.seq..(partial)
  1  ..atgggcgtgg acatcggacg ctccctgaaa caaatgaagg aacagggcgc
 51    ggaaatcgat ttgaaagtct ttaccgatgc catgcaggca gtgtatgacg
101    gcaaagaaat caaaatgacc gaagagcagg cccaggaagt gatgatgaaa
151    ttcctgcagg agcagcaggc taaagccgta gaaaaacaca aggcggatgc
201    gaaggccaac aaagaaaaag gcgaagcctt cctgaaggaa aatgccgccg
251    aagacggcgt gaagaccact gcttccggtc tgcagtacaa aatcaccaaa
301    cagggtgaag gcaaacagcc gacaaaagac gacatcgtta ccgtggaata
351    cgaaggccgc ctgattgacg gtaccgtatt cgacagcagc aaagccaacg
401    gcggcccggc caccttccct ttgagccaag tgattccggg ttggaccgaa
451    ggcgtacggc ttctgaaaga aggcggcgaa gccacgttct acatcccgtc
501    caaccttgcc taccgcgaac agggtgcggg cgaaaaaatc ggtccgaacg
551    ccactttggt atttgacgtg aaactggtca aaatcggcgc acccgaaaac
601    gcgcccgcca agcagccgga tcaagtcgac atcaaaaaag taaattaa
```

This corresponds to the amino acid sequence <SEQ ID 23; ORF 576.ng>:

```
g576.pep..(partial)
  1  ..MGVDIGRSLK QMKEQGAEID LKVFTDAMQA VYDGKEIKMT EEQAQEVMMK
 51    FLQEQQAKAV EKHKADAKAN KEKGEAFLKE NAAEDGVKTT ASGLQYKITK
101    QGEGKQPTKD DIVTVEYEGR LIDGTVFDSS KANGGPATFP LSQVIPGWTE
151    GVRLLKEGGE ATFYIPSNLA YREQGAGEKI GPNATLVFDV KLVKIGAPEN
201    APAKQPDQVD IKKVN*
```

Computer analysis of this amino acid sequence gave the following results:
Homology with a predicted ORF from *N. gonorrhoeae*

```
m576/g576  97.2% identity in 215 aa overlap

                   10        20        30        40        50        60
m576.pep   MQQASYAMGVDIGRSLKQMKEQGAEIDLKVFTEAMQAVYDGKEIKMTEEQAQEVMMKFLQ
                  |||||||||||||||||||||||||:||||||||||||||||||||||||||
g576              MGVDIGRSLKQMKEQGAEIDLKVFTDAMQAVYDGKEIKMTEEQAQEVMMKFLQ
                          10        20        30        40        50

                   70        80        90       100       110       120
m576.pep   EQQAKAVEKHKADAKANKEKGEAFLKENAAKDGVKTTASGLQYKITKQGEGKQPTKDDIV
           ||||||||||||||||||||||||||||||:|||||||||||||||||||||||||||||
g576       EQQAKAVEKHKADAKANKEKGEAFLKENAAEDGVKTTASGLQYKITKQGEGKQPTKDDIV
                 60        70        80        90       100       110

                  130       140       150       160       170       180
m576.pep   TVEYEGRLIDGTVFDSSKANGGPVTFPLSQVIPGWTEGVQLLKEGGEATFYIPSNLAYRE
           ||||||||||||||||||||||:||||||||||||||||:||||||||||||||||||||
g576       TVEYEGRLIDGTVFDSSKANGGPATFPLSQVIPGWTEGVRLLKEGGEATFYIPSNLAYRE
              120       130       140       150       160       170

                  190       200       210       220
m576.pep   QGAGDKIGPNATLVFDVKLVKIGAPENAPAKQPAQVDIKKVNX
           ||||:||||||||||||||||||||||||||||| |||||||||
g576       QGAGEKIGPNATLVFDVKLVKIGAPENAPAKQPDQVDIKKVNX
              180       190       200       210
```

The following partial DNA sequence was identified in *N. meningitidis* <SEQ ID 24>:

```
a576.seq
      1  ATGAACACCA TTTTCAAAAT CAGCGCACTG ACCCTTTCCG CCGCTTTGGC
     51  ACTTTCCGCC TGCGGCAAAA AAGAAGCCGC CCCCGCATCT GCATCCGAAC
    101  CTGCCGCCGC TTCTTCCGCG CAGGGCGACA CCTCTTCGAT CGGCAGCACG
    151  ATGCAGCAGG CAAGCTATGC GATGGGCGTG GACATCGGAC GCTCCCTGAA
    201  GCAAATGAAG GAACAGGGCG CGGAAATCGA TTTGAAAGTC TTTACCGAAG
    251  CCATGCAGGC AGTGTATGAC GGCAAAGAAA TCAAAATGAC CGAAGAGCAG
    301  GCTCAGGAAG TCATGATGAA ATTCCTTCAG GAACAACAGG CTAAAGCCGT
    351  AGAAAAACAC AAGGCGGACG CGAAGGCCAA TAAAGAAAAA GGCGAAGCCT
    401  TTCTGAAAGA AAATGCCGCC AAAGACGGCG TGAAGACCAC TGCTTCCGGC
    451  CTGCAATACA AAATCACCAA ACAGGGCGAA GGCAAACAGC CGACCAAAGA
    501  CGACATCGTT ACCGTGGAAT ACGAAGGCCG CCTGATTGAC GGTACGGTAT
    551  TCGACAGCAG CAAAGCCAAC GGCGGCCCGG TCACCTTCCC TTTGAGCCAA
    601  GTGATTCTGG GTTGGACCGA AGGCGTACAG CTTCTGAAAG AAGGCGGCGA
    651  AGCCACGTTC TACATCCCGT CCAACCTTGC CTACCGCGAA CAGGGTGCGG
    701  GCGACAAAAT CGGCCCGAAC GCCACTTTGG TATTTGATGT GAAACTGGTC
    751  AAAATCGGCG CACCCGAAAA CGCGCCCGCC AAGCAGCCGG CTCAAGTCGA
    801  CATCAAAAAA GTAAATTAA
```

This corresponds to the amino acid sequence <SEQ ID 25; ORF 576.a>:

```
a576.pep
      1  MNTIFKISAL TLSAALALSA CGKKEAAPAS ASEPAAASSA QGDTSSIGST
     51  MQQASYAMGV DIGRSLKQMK EQGAEIDLKV FTEAMQAVYD GKEIKMTEEQ
    101  AQEVMMKFLQ EQQAKAVEKH KADAKANKEK GEAFLKENAA KDGVKTTASG
    151  LQYKITKQGE GKQPTKDDIV TVEYEGRLID GTVFDSSKAN GGPVTFPLSQ
    201  VILGWTEGVQ LLKEGGEATF YIPSNLAYRE QGAGDKIGPN ATLVFDVKLV
    251  KIGAPENAPA KQPAQVDIKK VN*

m576/a576    ORFs 576 and 576.a showed a 99.5% identity in 222 aa overlap

                                                    10        20        30
m576.pep                                     MQQASYAMGVDIGRSLKQMKEQGAEIDLKV
                                             ||||||||||||||||||||||||||||||
a576         CGKKEAAPASASEPAAASSAQGDTSSIGSTMQQASYAMGVDIGRSLKQMKEQGAEIDLKV
                     30        40        50        60        70        80

                     40        50        60        70        80        90
m576.pep     FTEAMQAVYDGKEIKMTEEQAQEVMMKFLQEQQAKAVEKHKADAKANKEKGEAFLKENAA
             ||||||||||||||||||||||||||||||||||||||||||||||||||||||||||||
a576         FTEAMQAVYDGKEIKMTEEQAQEVMMKFLQEQQAKAVEKHKADAKANKEKGEAFLKENAA
                     90       100       110       120       130       140

                    100       110       120       130       140       150
m576.pep     KDGVKTTASGLQYKITKQGEGKQPTKDDIVTVEYEGRLIDGTVFDSSKANGGPVTFPLSQ
             ||||||||||||||||||||||||||||||||||||||||||||||||||||||||||||
a576         KDGVKTTASGLQYKITKQGEGKQPTKDDIVTVEYEGRLIDGTVFDSSKANGGPVTFPLSQ
                    150       160       170       180       190       200

                    160       170       180       190       200       210
m576.pep     VIPGWTEGVQLLKEGGEATFYIPSNLAYREQGAGDKIGPNATLVFDVKLVKIGAPENAPA
             || |||||||||||||||||||||||||||||||||||||||||||||||||||||||||
a576         VILGWTEGVQLLKEGGEATFYIPSNLAYREQGAGDKIGPNATLVFDVKLVKIGAPENAPA
                    210       220       230       240       250       260

                    220
m576.pep     KQPAQVDIKKVNX
             |||||||||||||
a576         KQPAQVDIKKVNX
                    270
```

Further work revealed the following DNA sequence identified in *N. meningitidis* <SEQ ID 26>:

```
m576-1.seq
      1  ATGAACACCA TTTTCAAAAT CAGCGCACTG ACCCTTTCCG CCGCTTTGGC
     51  ACTTTCCGCC TGCGGCAAAA AAGAAGCCGC CCCCGCATCT GCATCCGAAC
    101  CTGCCGCCGC TTCTTCCGCG CAGGGCGACA CCTCTTCGAT CGGCAGCACG
    151  ATGCAGCAGG CAAGCTATGC GATGGGCGTG GACATCGGAC GCTCCCTGAA
    201  GCAAATGAAG GAACAGGGCG CGGAAATCGA TTTGAAAGTC TTTACCGAAG
    251  CCATGCAGGC AGTGTATGAC GGCAAAGAAA TCAAAATGAC CGAAGAGCAG
    301  GCTCAGGAAG TCATGATGAA ATTCCTTCAG GAACAACAGG CTAAAGCCGT
    351  AGAAAAACAC AAGGCGGACG CGAAGGCCAA TAAAGAAAAA GGCGAAGCCT
    401  TTCTGAAAGA AAATGCCGCC AAAGACGGCG TGAAGACCAC TGCTTCCGGC
    451  CTGCAATACA AAATCACCAA ACAGGGCGAA GGCAAACAGC CGACCAAAGA
    501  CGACATCGTT ACCGTGGAAT ACGAAGGCCG CCTGATTGAC GGTACGGTAT
    551  TCGACAGCAG CAAAGCCAAC GGCGGCCCGG TCACCTTCCC TTTGAGCCAA
    601  GTGATTCCGG GTTGGACCGA AGGCGTACAG CTTCTGAAAG AAGGCGGCGA
    651  AGCCACGTTC TACATCCCGT CCAACCTTGC CTACCGCGAA CAGGGTGCGG
    701  GCGACAAAAT CGGTCCGAAC GCCACTTTGG TATTTGATGT GAAACTGGTC
    751  AAAATCGGCG CACCCGAAAA CGCGCCCGCC AAGCAGCCGG CTCAAGTCGA
    801  CATCAAAAAA GTAAATTAA
```

This corresponds to the amino acid sequence <SEQ ID 27; ORF 576-1>:

```
m576-1.pep
     1  MNTIFKISAL TLSAALALSA CGKKEAAPAS ASEPAAASSA QGDTSSIGST
    51  MQQASYAMGV DIGRSLKQMK EQGAEIDLKV FTEAMQAVYD GKEIKMTEEQ
   101  AQEVMMKFLQ EQQAKAVEKH KADAKANKEK GEAFLKENAA KDGVKTTASG
   151  LQYKITKQGE GKQPTKDDIV TVEYEGRLID GTVFDSSKAN GGPVTFPLSQ
   201  VIPGWTEGVQ LLKEGGEATF YIPSNLAYRE QGAGDKIGPN ATLVFDVKLV
   251  KIGAPENAPA KQPAQVDIKK VN*
```

The following DNA sequence was identified in *N. gonorrhoeae* <SEQ ID 28>:

```
g576-1.seq
     1  ATGAACACCA TTTTCAAAAT CAGCGCACTG ACCCTTTCCG CCGCTTTGGC
    51  ACTTTCCGCC TGCGGCAAAA AAGAAGCCGC CCCCGCATCT GCATCCGAAC
   101  CTGCCGCCGC TTCTGCCGCG CAGGGCGACA CCTCTTCAAT CGGCAGCACG
   151  ATGCAGCAGG CAAGCTATGC AATGGGCGTG GACATCGGAC GCTCCCTGAA
   201  ACAAATGAAG GAACAGGGCG CGGAAATCGA TTTGAAAGTC TTTACCGATG
   251  CCATGCAGGC AGTGTATGAC GGCAAAGAAA TCAAAATGAC CGAAGAGCAG
   301  GCCCAGGAAG TGATGATGAA ATTCCTGCAG GAGCAGCAGG CTAAAGCCGT
   351  AGAAAAACAC AAGGCGGATG CGAAGGCCAA CAAAGAAAAA GGCGAAGCCT
   401  TCCTGAAGGA AAATGCCGCC AAAGACGGCG TGAAGACCAC TGCTTCCGGT
   451  CTGCAGTACA AAATCACCAA ACAGGGTGAA GGCAAACAGC CGACAAAAGA
   501  CGACATCGTT ACCGTGGAAT ACGAAGGCCG CCTGATTGAC GGTACCGTAT
   551  TCGACAGCAG CAAAGCCAAC GGCGGCCCGG CCACCTTCCC TTTGAGCCAA
   601  GTGATTCCGG GTTGGACCGA AGGCGTACGG CTTCTGAAAG AAGGCGGCGA
   651  AGCCACGTTC TACATCCCGT CCAACCTTGC CTACCGCGAA CAGGGTGCGG
   701  GCGAAAAAAT CGGTCCGAAC GCCACTTTGG TATTTGACGT GAAACTGGTC
   751  AAAATCGGCG CACCCGAAAA CGCGCCCGCC AAGCAGCCGG ATCAAGTCGA

   801  CATCAAAAAA GTAAATTAA
```

This corresponds to the amino acid sequence <SEO ID 29; ORF 576-1.ng>:

```
g576-1.pep
      1  MNTIFKISAL TLSAALALSA CGKKEAAPAS ASEPAAASAA QGDTSSIGST
     51  MQQASYAMGV DIGRSLKQMK EQGAEIDLKV FTDAMQAVYD GKEIKMTEEQ
    101  AQEVMMKFLQ EQQAKAVEKH KADAKANKEK GEAFLKENAA KDGVKTTASG
    151  LQYKITKQGE GKQPTKDDIV TVEYEGRLID GTVFDSSKAN GGPATFPLSQ
    201  VIPGWTEGVR LLKEGGEATF YIPSNLAYRE QGAGEKIGPN ATLVFDVKLV
    251  KIGAPENAPA KQPDQVDIKK VN*
```

**g576-1/m576-1** ORFs 576-1 and 576-1.ng showed a 97.8% identity in 272 aa overlap

```
                    10        20        30        40        50        60
g576-1.pep   MNTIFKISALTLSAALALSACGKKEAAPASASEPAAASAAQGDTSSIGSTMQQASYAMGV
             ||||||||||||||||||||||||||||||||||||||:|||||||||||||||||||||
m576-1       MNTIFKISALTLSAALALSACGKKEAAPASASEPAAASSAQGDTSSIGSTMQQASYAMGV
                    10        20        30        40        50        60

                    70        80        90       100       110       120
g576-1.pep   DIGRSLKQMKEQGAEIDLKVFTDAMQAVYDGKEIKMTEEQAQEVMMKFLQEQQAKAVEKH
             ||||||||||||||||||||||:|||||||||||||||||||||||||||||||||||||
m576-1       DIGRSLKQMKEQGAEIDLKVFTEAMQAVYDGKEIKMTEEQAQEVMMKFLQEQQAKAVEKH
                    70        80        90       100       110       120

                   130       140       150       160       170       180
g576-1.pep   KADAKANKEKGEAFLKENAAKDGVKTTASGLQYKITKQGEGKQPTKDDIVTVEYEGRLID
             ||||||||||||||||||||||||||||||||||||||||||||||||||||||||||||
m576-1       KADAKANKEKGEAFLKENAAKDGVKTTASGLQYKITKQGEGKQPTKDDIVTVEYEGRLID
                   130       140       150       160       170       180

                   190       200       210       220       230       240
g576-1.pep   GTVFDSSKANGGPATFPLSQVIPGWTEGVRLLKEGGEATFYIPSNLAYREQGAGEKIGPN
             |||||||||||||:|||||||||||||||:|||||||||||||||||||||||||:||||
m576-1       GTVFDSSKANGGPVTFPLSQVIPGWTEGVQLLKEGGEATFYIPSNLAYREQGAGDKIGPN
                   190       200       210       220       230       240

                   250       260       270
g576-1.pep   ATLVFDVKLVKIGAPENAPAKQPDQVDIKKVNX
             |||||||||||||||||||||||| ||||||||
m576-1       ATLVFDVKLVKIGAPENAPAKQPAQVDIKKVNX
                   250       260       270
```

The following DNA sequence was identified in *N. meningitidis* <SEQ ID 30>:

```
a576-1.seq
      1  ATGAACACCA TTTTCAAAAT CAGCGCACTG ACCCTTTCCG CCGCTTTGGC
     51  ACTTTCCGCC TGCGGCAAAA AAGAAGCCGC CCCCGCATCT GCATCCGAAC
    101  CTGCCGCCGC TTCTTCCGCG CAGGGCGACA CCTCTTCGAT CGGCAGCACG
    151  ATGCAGCAGG CAAGCTATGC GATGGGCGTG GACATCGGAC GCTCCCTGAA
    201  GCAAATGAAG GAACAGGGCG CGGAAATCGA TTTGAAAGTC TTTACCGAAG
    251  CCATGCAGGC AGTGTATGAC GGCAAAGAAA TCAAAATGAC CGAAGAGCAG
    301  GCTCAGGAAG TCATGATGAA ATTCCTTCAG GAACAACAGG CTAAAGCCGT
    351  AGAAAAACAC AAGGCGGACG CGAAGGCCAA TAAAGAAAAA GGCGAAGCCT
    401  TTCTGAAAGA AAATGCCGCC AAAGACGGCG TGAAGACCAC TGCTTCCGGC
    451  CTGCAATACA AAATCACCAA ACAGGGCGAA GGCAAACAGC CGACCAAAGA
    501  CGACATCGTT ACCGTGGAAT ACGAAGGCCG CCTGATTGAC GGTACGGTAT
    551  TCGACAGCAG CAAAGCCAAC GGCGGCCCGG TCACCTTCCC TTTGAGCCAA
    601  GTGATTCTGG GTTGGACCGA AGGCGTACAG CTTCTGAAAG AAGGCGGCGA
    651  AGCCACGTTC TACATCCCGT CCAACCTTGC CTACCGCGAA CAGGGTGCGG
    701  GCGACAAAAT CGGCCCGAAC GCCACTTTGG TATTTGATGT GAAACTGGTC

    751  AAAATCGGCG CACCCGAAAA CGCGCCCGCC AAGCAGCCGG CTCAAGTCGA
    801  CATCAAAAAA GTAAATTAA
```

This corresponds to the amino acid sequence <SEQ ID 31; ORF 576-1.a>:

```
a576-1.pep
     1  MNTIFKISAL TLSAALALSA CGKKEAAPAS ASEPAAASSA QGDTSSIGST
    51  MQQASYAMGV DIGRSLKQMK EQGAEIDLKV FTEAMQAVYD GKEIKMTEEQ
   101  AQEVMMKFLQ EQQAKAVEKH KADAKANKEK GEAFLKENAA KDGVKTTASG
   151  LQYKITKQGE GKQPTKDDIV TVEYEGRLID GTVFDSSKAN GGPVTFPLSQ
   201  VILGWTEGVQ LLKEGGEATF YIPSNLAYRE QGAGDKIGPN ATLVFDVKLV
   251  KIGAPENAPA KQPAQVDIKK VN*
```

**a576-1/m576-1** ORFs 576-1 and 576-1.a 99.6% identity in 272 aa overlap

```
                     10        20        30        40        50        60
a576-1.pep   MNTIFKISALTLSAALALSACGKKEAAPASASEPAAASSAQGDTSSIGSTMQQASYAMGV
             ||||||||||||||||||||||||||||||||||||||||||||||||||||||||||||
m576-1       MNTIFKISALTLSAALALSACGKKEAAPASASEPAAASSAQGDTSSIGSTMQQASYAMGV
                     10        20        30        40        50        60

                     70        80        90       100       110       120
a576-1.pep   DIGRSLKQMKEQGAEIDLKVFTEAMQAVYDGKEIKMTEEQAQEVMMKFLQEQQAKAVEKH
             ||||||||||||||||||||||||||||||||||||||||||||||||||||||||||||
m576-1       DIGRSLKQMKEQGAEIDLKVFTEAMQAVYDGKEIKMTEEQAQEVMMKFLQEQQAKAVEKH
                     70        80        90       100       110       120

                    130       140       150       160       170       180
a576-1.pep   KADAKANKEKGEAFLKENAAKDGVKTTASGLQYKITKQGEGKQPTKDDIVTVEYEGRLID
             ||||||||||||||||||||||||||||||||||||||||||||||||||||||||||||
m576-1       KADAKANKEKGEAFLKENAAKDGVKTTASGLQYKITKQGEGKQPTKDDIVTVEYEGRLID
                    130       140       150       160       170       180

                    190       200       210       220       230       240
a576-1.pep   GTVFDSSKANGGPVTFPLSQVILGWTEGVQLLKEGGEATFYIPSNLAYREQGAGDKIGPN
             |||||||||||||||||||||| |||||||||||||||||||||||||||||||||||||
m576-1       GTVFDSSKANGGPVTFPLSQVIPGWTEGVQLLKEGGEATFYIPSNLAYREQGAGDKIGPN
                    190       200       210       220       230       240

                    250       260       270
a576-1.pep   ATLVFDVKLVKIGAPENAPAKQPAQVDIKKVNX
             |||||||||||||||||||||||||||||||||
m576-1       ATLVFDVKLVKIGAPENAPAKQPAQVDIKKVNX
                    250       260       270
```

919 and 919-2 gnm43.seq

The following partial DNA sequence was identified in *N.meningitidis* <SEQ ID 32>:

```
m919.seq
     1  ATGAAAAAAT ACCTATTCCG CGCCGCCCTG TACGGCATCG CCGCCGCCAT
    51  CCTCGCCGCC TGCCAAAGCA AGAGCATCCA AACCTTTCCG CAACCCGACA
   101  CATCCGTCAT CAACGGCCCG GACCGGCCGG TCGGCATCCC CGACCCCGCC
   151  GGAACGACGG TCGGCGGCGG CGGGGCCGTC TATACCGTTG TACCGCACCT
   201  GTCCCTGCCC CACTGGGCGG CGCAGGATTT CGCCAAAAGC CTGCAATCCT
   251  TCCGCCTCGG CTGCGCCAAT TTGAAAAACC GCCAAGGCTG GCAGGATGTG
   301  TGCGCCCAAG CCTTTCAAAC CCCCGTCCAT TCCTTTCAGG CAAAACAGTT
   351  TTTTGAACGC TATTTCACGC CGTGGCAGGT TGCAGGCAAC GGAAGCCTTG
```

```
 401  CCGGTACGGT TACCGGCTAT TACGAACCGG TGCTGAAGGG CGACGACAGG
 451  CGGACGGCAC AAGCCCGCTT CCCGATTTAC GGTATTCCCG ACGATTTTAT
 501  CTCCGTCCCC CTGCCTGCCG GTTTGCGGAG CGGAAAAGCC CTTGTCCGCA
 551  TCAGGCAGAC GGGAAAAAAC AGCGGCACAA TCGACAATAC CGGCGGCACA
 601  CATACCGCCG ACCTCTCCcG ATTCCCCATC ACCGCGCGCA CAACAGCAAT
 651  CAAAGGCAGG TTTGAAGGAA GCCGCTTCCT CCCCTACCAC ACGCGCAACC
 701  AAATCAACGG CGGCGCGCTT GACGGCAAAG CCCCGATACT CGGTTACGCC
 751  GAAGACCCTG TCGAACTTTT TTTTATGCAC ATCCAAGGCT CGGGCCGTCT
 801  GAAAACCCCG TCCGGCAAAT ACATCCGCAT CGGCTATGCC GACAAAAACG
 851  AACATCCyTA CGTTTCCATC GGACGCTATA TGGCGGATAA GGGCTACCTC
 901  AAACTCGGAC AAACCTCCAT GCAGGGCATT AAGTCTTATA TGCGGCAAAA
 951  TCCGCAACGC CTCGCCGAAG TTTTGGGTCA AAACCCCAGC TATATCTTTT
1001  TCCGCGAGCT TGCCGGAAGC AGCAATGACG GCCCTGTCGG CGCACTGGGC
1051  ACGCCGCTGA TGGGGGAATA TGCCGGCGCA GTCGACCGGC ACTACATTAC
1101  CTTGGGTGCG CCCTTATTTG TCGCCACCGC CCATCCGGTT ACCCGCAAAG
1151  CCCTCAACCG CCTGATTATG GCGCAGGATA CCGGCAGCGC GATTAAAGGC
1201  GCGGTGCGCG TGGATTATTT TTGGGGATAC GGCGACGAAG CCGGCGAACT
1251  TGCCGGCAAA CAGAAAACCA CGGGATATGT CTGGCAGCTC CTACCCAACG
1301  GTATGAAGCC CGAATACCGc CCGTAA
```

This corresponds to the amino acid sequence <SEQ ID 33; ORF 919>:

**m919.pep**

```
  1  MKKYLFRAAL YGIAAAILAA CQSKSIQTFP QPDTSVINGP DRPVGIPDPA
 51  GTTVGGGGAV YTVVPHLSLP HWAAQDFAKS LQSFRLGCAN LKNRQGWQDV
101  CAQAFQTPVH SFQAKQFFER YFTPWQVAGN GSLAGTVTGY YEPVLKGDDR
151  RTAQARFPIY GIPDDFISVP LPAGLRSGKA LVRIRQTGKN SGTIDNTGGT
201  HTADLSRFPI TARTTAIKGR FEGSRFLPYH TRNQINGGAL DGKAPILGYA
251  EDPVELFFMH IQGSGRLKTP SGKYIRIGYA DKNEHPYVSI GRYMADKGYL
301  KLGQTSMQGI KSYMRQNPQR LAEVLGQNPS YIFFRELAGS SNDGPVGALG
351  TPLMGEYAGA VDRHYITLGA PLFVATAHPV TRKALNRLIM AQDTGSAIKG
401  AVRVDYFWGY GDEAGELAGK QKTTGYVWQL LPNGMKPEYR P*
```

The following partial DNA sequence was identified in *N.meningitidis* <SEQ ID 34>:

**m919-2.seq**

```
   1  ATGAAAAAAT ACCTATTCCG CGCCGCCCTG TACGGCATCG CCGCCGCCAT
  51  CCTCGCCGCC TGCCAAAGCA AGAGCATCCA AACCTTTCCG CAACCCGACA
 101  CATCCGTCAT CAACGGCCCG GACCGGCCGG TCGGCATCCC CGACCCCGCC
 151  GGAACGACGG TCGGCGGCGG CGGGGCCGTC TATACCGTTG TACCGCACCT
 201  GTCCCTGCCC CACTGGGCGG CGCAGGATTT CGCCAAAAGC CTGCAATCCT
 251  TCCGCCTCGG CTGCGCCAAT TTGAAAAACC GCCAAGGCTG GCAGGATGTG
 301  TGCGCCCAAG CCTTTCAAAC CCCCGTCCAT TCCTTTCAGG CAAAACAGTT
 351  TTTTGAACGC TATTTCACGC CGTGGCAGGT TGCAGGCAAC GGAAGCCTTG
 401  CCGGTACGGT TACCGGCTAT TACGAACCGG TGCTGAAGGG CGACGACAGG
 451  CGGACGGCAC AAGCCCGCTT CCCGATTTAC GGTATTCCCG ACGATTTTAT
 501  CTCCGTCCCC CTGCCTGCCG GTTTGCGGAG CGGAAAAGCC CTTGTCCGCA
 551  TCAGGCAGAC GGGAAAAAAC AGCGGCACAA TCGACAATAC CGGCGGCACA
 601  CATACCGCCG ACCTCTCCCG ATTCCCCATC ACCGCGCGCA CAACAGCAAT
 651  CAAAGGCAGG TTTGAAGGAA GCCGCTTCCT CCCCTACCAC ACGCGCAACC
 701  AAATCAACGG CGGCGCGCTT GACGGCAAAG CCCCGATACT CGGTTACGCC
 751  GAAGACCCTG TCGAACTTTT TTTTATGCAC ATCCAAGGCT CGGGCCGTCT
 801  GAAAACCCCG TCCGGCAAAT ACATCCGCAT CGGCTATGCC GACAAAAACG
 851  AACATCCCTA CGTTTCCATC GGACGCTATA TGGCGGATAA GGGCTACCTC
 901  AAACTCGGAC AAACCTCCAT GCAGGGCATT AAGTCTTATA TGCGGCAAAA
 951  TCCGCAACGC CTCGCCGAAG TTTTGGGTCA AAACCCCAGC TATATCTTTT
1001  TCCGCGAGCT TGCCGGAAGC AGCAATGACG GCCCTGTCGG CGCACTGGGC
1051  ACGCCGCTGA TGGGGGAATA TGCCGGCGCA GTCGACCGGC ACTACATTAC
1101  CTTGGGTGCG CCCTTATTTG TCGCCACCGC CCATCCGGTT ACCCGCAAAG
1151  CCCTCAACCG CCTGATTATG GCGCAGGATA CCGGCAGCGC GATTAAAGGC
```

```
1201  GCGGTGCGCG TGGATTATTT TTGGGGATAC GGCGACGAAG CCGGCGAACT
1251  TGCCGGCAAA CAGAAAACCA CGGGATATGT CTGGCAGCTC CTACCCAACG
1301  GTATGAAGCC CGAATACCGC CCGTAA
```

This corresponds to the amino acid sequence <SEQ ID 35; ORF 919-2>:

```
m919-2.pep
  1  MKKYLFRAAL YGIAAAILAA CQSKSIQTFP QPDTSVINGP DRPVGIPDPA
 51  GTTVGGGGAV YTVVPHLSLP HWAAQDFAKS LQSFRLGCAN LKNRQGWQDV
101  CAQAFQTPVH SFQAKQFFER YFTPWQVAGN GSLAGTVTGY YEPVLKGDDR
151  RTAQARFPIY GIPDDFISVP LPAGLRSGKA LVRIRQTGKN SGTIDNTGGT
201  HTADLSRFPI TARTTAIKGR FEGSRFLPYH TRNQINGGAL DGKAPILGYA
251  EDPVELFFMH IQGSGRLKTP SGKYIRIGYA DKNEHPYVSI GRYMADKGYL
301  KLGQTSMQGI KSYMRQNPQR LAEVLGQNPS YIFFRELAGS SNDGPVGALG
351  TPLMGEYAGA VDRHYITLGA PLFVATAHPV TRKALNRLIM AQDTGSAIKG
401  AVRVDYFWGY GDEAGELAGK QKTTGYVWQL LPNGMKPEYR P*
```

The following partial DNA sequence was identified in *N.gonorrhoeae* <SEQ ID 36>:

```
g919.seq
   1  ATGAAAAAAC ACCTGCTCCG CTCCGCCCTG TACGGcatCG CCGCCgccAT
  51  CctcgCCGCC TGCCAAAgca gGAGCATCCA AACCTTTCCG CAACCCGACA
 101  CATCCGTCAT CAACGGCCCG GACCGGCCGG CCGGCATCCC CGACCCCGCC
 151  GGAACGACGG TTGCCGGCGG CGGGGCCGTC TATACCGTTG TGCCGCACCT
 201  GTCCATGCCC CACTGGGCGG CGCaggATTT TGCCAAAAGC CTGCAATCCT
 251  TCCGCCTCGG CTGCGCCAAT TTGAAAAACC GCCAAGGCTG GCAGGATGTG
 301  TGCGCCCAAG CCTTTCAAAC CCCCGTGCAT TCCTTTCAGG CAAAGcGgTT
 351  TTTTGAACGC TATTTCACGC cgtGGCaggt tgcaggcaAC GGAAGcCTTG
 401  Caggtacggt TACCGGCTAT TACGAACCGG TGCTGAAGGG CGACGGCAGG
 451  CGGACGGAAC GGGCCCGCTT CCCGATTTAC GGTATTCCCG ACGATTTTAT
 501  CTCCGTCCCG CTGCCTGCCG GTTTGCGGGG CGGAAAAAAC CTTGTCCGCA
 551  TCAGGCAGac ggGGAAAAAC AGCGGCACGA TCGACAATGC CGGCGGCACG
 601  CATACCGCCG ACCTCTCCCG ATTCCCCATC ACCGCGCGCA CAACGGcaat
 651  caaaGGCAGG TTTGAaggAA GCCGCTTCCT CCCTTACCAC ACGCGCAACC
 701  AAAtcaacGG CGGCgcgcTT GACGGCAAag cccCCATCCT CggttacgcC
 751  GAagaccCcG tcgaacttTT TTTCATGCAC AtccaaggCT CGGGCCGCCT
 801  GAAAACCCcg tccggcaaat acatCCGCAt cggaTacgcc gacAAAAACG
 851  AACAtccgTa tgtttccatc ggACGctaTA TGGCGGACAA AGGCTACCTC
 901  AAGctcgggc agACCTCGAT GCAGGgcatc aaagcCTATA TGCGGCAAAA
 951  TGCGCAACGC CTCGCCGAAG TTTTGGGTCA AAACCCCAGC TATATCTTTT
1001  TCCGCGAGCT TGCCGGAAGC GGCAATGAGG GCCCCGTCGG CGCACTGGGC
1051  ACGCCACTGA TGGGGGAATA CGCCGGCGCA ATCGACCGGC ACTACATTAC
1101  CTTGGGCGCG CCCTTATTTG TCGCCACCGC CCATCCGGTT ACCCGCAAAG
1151  CCCTCAACCG CCTGATTATG GCGCAGGATA CAGGCAGCGC GATCAAAGGC
1201  GCGGTGCGCG TGGATTATTT TTGGGGTTAC GGCGACGAAG CCGGCGAACT
1251  TGCCGGCAAA CAGAAAACCA CGGGATACGT CTGGCAGCTC CTGCCCAACG
1301  GCATGAAGCC CGAATACCGC CCGTGA
```

This corresponds to the amino acid sequence <SEQ ID 37; ORF 919.ng>:

```
g919.pep
      1  MKKHLLRSAL YGIAAAILAA CQSRSIQTFP QPDTSVINGP DRPAGIPDPA
     51  GTTVAGGGAV YTVVPHLSMP HWAAQDFAKS LQSFRLGCAN LKNRQGWQDV
    101  CAQAFQTPVH SFQAKRFFER YFTPWQVAGN GSLAGTVTGY YEPVLKGDGR
    151  RTERARFPIY GIPDDFISVP LPAGLRGGKN LVRIRQTGKN SGTIDNAGGT
    201  HTADLSRFPI TARTTAIKGR FEGSRFLPYH TRNQINGGAL DGKAPILGYA
    251  EDPVELFFMH IQGSGRLKTP SGKYIRIGYA DKNEHPYVSI GRYMADKGYL
    301  KLGQTSMQGI KAYMRQNPQR LAEVLGQNPS YIFFRELAGS GNEGPVGALG
    351  TPLMGEYAGA IDRHYITLGA PLFVATAHPV TRKALNRLIM AQDTGSAIKG
    401  AVRVDYFWGY GDEAGELAGK QKTTGYVWQL LPNGMKPEYR P*
```

ORF 919 shows 95.9 % identity over a 441 aa overlap with a predicted ORF (ORF 919.ng) from *N. gonorrhoeae:*

```
m919/g919

                       10        20        30        40        50        60
m919.pep     MKKYLFRAALYGIAAAILAACQSKSIQTFPQPDTSVINGPDRPVGIPDPAGTTVGGGGAV
             |||:|:|:|||||||||||||||:|||||||||||||||||||:||||||||||:|||||
g919         MKKHLLRSALYGIAAAILAACQSRSIQTFPQPDTSVINGPDRPAGIPDPAGTTVAGGGAV
                       10        20        30        40        50        60

                       70        80        90       100       110       120
m919.pep     YTVVPHLSLPHWAAQDFAKSLQSFRLGCANLKNRQGWQDVCAQAFQTPVHSFQAKQFFER
             ||||||||:||||||||||||||||||||||||||||||||||||||||||||||:||||
g919         YTVVPHLSMPHWAAQDFAKSLQSFRLGCANLKNRQGWQDVCAQAFQTPVHSFQAKRFFER
                       70        80        90       100       110       120

                      130       140       150       160       170       180
m919.pep     YFTPWQVAGNGSLAGTVTGYYEPVLKGDDRRTAQARFPIYGIPDDFISVPLPAGLRSGKA
             |||||||||||||||||||||||||||| ||| :||||||||||||||||||||||:|| 
g919         YFTPWQVAGNGSLAGTVTGYYEPVLKGDGRRTERARFPIYGIPDDFISVPLPAGLRGGKN
                      130       140       150       160       170       180

                      190       200       210       220       230       240
m919.pep     LVRIRQTGKNSGTIDNTGGTHTADLSRFPITARTTAIKGRFEGSRFLPYHTRNQINGGAL
             ||||||||||||||||:|||||||||||||||||||||||||||||||||||||||||||
g919         LVRIRQTGKNSGTIDNAGGTHTADLSRFPITARTTAIKGRFEGSRFLPYHTRNQINGGAL
                      190       200       210       220       230       240

                      250       260       270       280       290       300
m919.pep     DGKAPILGYAEDPVELFFMHIQGSGRLKTPSGKYIRIGYADKNEHPYVSIGRYMADKGYL
             ||||||||||||||||||||||||||||||||||||||||||||||||||||||||||||
g919         DGKAPILGYAEDPVELFFMHIQGSGRLKTPSGKYIRIGYADKNEHPYVSIGRYMADKGYL
                      250       260       270       280       290       300

                      310       320       330       340       350       360
m919.pep     KLGQTSMQGIKSYMRQNPQRLAEVLGQNPSYIFFRELAGSSNDGPVGALGTPLMGEYAGA
             |||||||||||:||||||||||||||||||||||||||||:|:|||||||||||||||||
g919         KLGQTSMQGIKAYMRQNPQRLAEVLGQNPSYIFFRELAGSGNEGPVGALGTPLMGEYAGA
                      310       320       330       340       350       360

                      370       380       390       400       410       420
m919.pep     VDRHYITLGAPLFVATAHPVTRKALNRLIMAQDTGSAIKGAVRVDYFWGYGDEAGELAGK
             :|||||||||||||||||||||||||||||||||||||||||||||||||||||||||||
g919         IDRHYITLGAPLFVATAHPVTRKALNRLIMAQDTGSAIKGAVRVDYFWGYGDEAGELAGK
                      370       380       390       400       410       420

                      430       440
m919.pep     QKTTGYVWQLLPNGMKPEYRPX
             ||||||||||||||||||||||
g919         QKTTGYVWQLLPNGMKPEYRPX
                      430       440
```

The following partial DNA sequence was identified in *N.meningitidis* <SEQ ID 38>:

a919.seq

```
   1 ATGAAAAAAT ACCTATTCCG CGCCGCCCTG TGCGGCATCG CCGCCGCCAT
  51 CCTCGCCGCC TGCCAAAGCA AGAGCATCCA AACCTTTCCG CAACCCGACA
 101 CATCCGTCAT CAACGGCCCG GACCGGCCGG TCGGCATCCC CGACCCCGCC
 151 GGAACGACGG TCGGCGGCGG CGGGGCCGTT TATACCGTTG TGCCGCACCT
 201 GTCCCTGCCC CACTGGGCGG CGCAGGATTT CGCCAAAAGC CTGCAATCCT
 251 TCCGCCTCGG CTGCGCCAAT TTGAAAAACC GCCAAGGCTG GCAGGATGTG
 301 TGCGCCCAAG CCTTTCAAAC CCCCGTCCAT TCCGTTCAGG CAAAACAGTT
 351 TTTTGAACGC TATTTCACGC CGTGGCAGGT TGCAGGCAAC GGAAGCCTTG
 401 CCGGTACGGT TACCGGCTAT TACGAGCCGG TGCTGAAGGG CGACGACAGG
 451 CGGACGGCAC AAGCCCGCTT CCCGATTTAC GGTATTCCCG ACGATTTTAT
 501 CTCCGTCCCC CTGCCTGCCG GTTTGCGGAG CGGAAAAGCC CTTGTCCGCA
 551 TCAGGCAGAC GGGAAAAAAC AGCGGCACAA TCGACAATAC CGGCGGCACA
 601 CATACCGCCG ACCTCTCCCA ATTCCCCATC ACTGCGCGCA CAACGGCAAT
 651 CAAAGGCAGG TTTGAAGGAA GCCGCTTCCT CCCCTACCAC ACGCGCAACC
 701 AAATCAACGG CGGCGCGCTT GACGGCAAAG CCCCGATACT CGGTTACGCC
 751 GAAGACCCCG TCGAACTTTT TTTTATGCAC ATCCAAGGCT CGGGCCGTCT
 801 GAAAACCCCG TCCGGCAAAT ACATCCGCAT CGGCTATGCC GACAAAAACG
 851 AACATCCCTA CGTTTCCATC GGACGCTATA TGGCGGACAA AGGCTACCTC
 901 AAGCTCGGGC AGACCTCGAT GCAGGGCATC AAAGCCTATA TGCAGCAAAA
 951 CCCGCAACGC CTCGCCGAAG TTTTGGGGCA AAACCCCAGC TATATCTTTT
1001 TCCGAGAGCT TACCGGAAGC AGCAATGACG GCCCTGTCGG CGCACTGGGC
1051 ACGCCGCTGA TGGGCGAGTA CGCCGGCGCA GTCGACCGGC ACTACATTAC
1101 CTTGGGCGCG CCCTTATTTG TCGCCACCGC CCATCCGGTT ACCCGCAAAG
1151 CCCTCAACCG CCTGATTATG GCGCAGGATA CCGGCAGCGC GATTAAAGGC
1201 GCGGTGCGCG TGGATTATTT TTGGGGATAC GGCGACGAAG CCGGCGAACT
1251 TGCCGGCAAA CAGAAAACCA CGGGATATGT CTGGCAGCTT CTGCCCAACG
1301 GTATGAAGCC CGAATACCGC CCGTAA
```

This corresponds to the amino acid sequence <SEQ ID 39; ORF 919.a>:

```
a919.pep
   1 MKKYLFRAAL CGIAAAILAA CQSKSIQTFP QPDTSVINGP DRPVGIPDPA
  51 GTTVGGGGAV YTVVPHLSLP HWAAQDFAKS LQSFRLGCAN LKNRQGWQDV
 101 CAQAFQTPVH SVQAKQFFER YFTPWQVAGN GSLAGTVTGY YEPVLKGDDR
 151 RTAQARFPIY GIPDDFISVP LPAGLRSGKA LVRIRQTGKN SGTIDNTGGT
 201 HTADLSQFPI TARTTAIKGR FEGSRFLPYH TRNQINGGAL DGKAPILGYA
 251 EDPVELFFMH IQGSGRLKTP SGKYIRIGYA DKNEHPYVSI GRYMADKGYL
 301 KLGQTSMQGI KAYMQQNPQR LAEVLGQNPS YIFFRELTGS SNDGPVGALG
 351 TPLMGEYAGA VDRHYITLGA PLFVATAHPV TRKALNRLIM AQDTGSAIKG
 401 AVRVDYFWGY GDEAGELAGK QKTTGYVWQL LPNGMKPEYR P*
```

m919/a919 ORFs 919 and 919.a showed a 98.6% identity in 441 aa overlap

```
                   10        20        30        40        50        60
m919.pep   MKKYLFRAALYGIAAAILAACQSKSIQTFPQPDTSVINGPDRPVGIPDPAGTTVGGGGAV
           |||||||||| |||||||||||||||||||||||||||||||||||||||||||||||||
a919       MKKYLFRAALCGIAAAILAACQSKSIQTFPQPDTSVINGPDRPVGIPDPAGTTVGGGGAV
                   10        20        30        40        50        60

                   70        80        90       100       110       120
m919.pep   YTVVPHLSLPHWAAQDFAKSLQSFRLGCANLKNRQGWQDVCAQAFQTPVHSFQAKQFFER
           ||||||||||||||||||||||||||||||||||||||||||||||||||| ||||||||
a919       YTVVPHLSLPHWAAQDFAKSLQSFRLGCANLKNRQGWQDVCAQAFQTPVHSVQAKQFFER
                   70        80        90       100       110       120

                  130       140       150       160       170       180
m919.pep   YFTPWQVAGNGSLAGTVTGYYEPVLKGDDRRTAQARFPIYGIPDDFISVPLPAGLRSGKA
           ||||||||||||||||||||||||||||||||||||||||||||||||||||||||||||
a919       YFTPWQVAGNGSLAGTVTGYYEPVLKGDDRRTAQARFPIYGIPDDFISVPLPAGLRSGKA
                  130       140       150       160       170       180

                  190       200       210       220       230       240
m919.pep   LVRIRQTGKNSGTIDNTGGTHTADLSRFPITARTTAIKGRFEGSRFLPYHTRNQINGGAL
           ||||||||||||||||||||||||||:|||||||||||||||||||||||||||||||||
```

```
a919       LVRIRQTGKNSGTIDNTGGTHTADLSQFPITARTTAIKGRFEGSRFLPYHTRNQINGGAL
                   190       200       210       220       230       240

                   250       260       270       280       290       300
m919.pep   DGKAPILGYAEDPVELFFMHIQGSGRLKTPSGKYIRIGYADKNEHPYVSIGRYMADKGYL
           ||||||||||||||||||||||||||||||||||||||||||||||||||||||||||||
a919       DGKAPILGYAEDPVELFFMHIQGSGRLKTPSGKYIRIGYADKNEHPYVSIGRYMADKGYL
                   250       260       270       280       290       300

                   310       320       330       340       350       360
m919.pep   KLGQTSMQGIKSYMRQNPQRLAEVLGQNPSYIFFRELAGSSNDGPVGALGTPLMGEYAGA
           |||||||||||:||:|||||||||||||||||||||||:|||||||||||||||||||||
a919       KLGQTSMQGIKAYMQQNPQRLAEVLGQNPSYIFFRELTGSSNDGPVGALGTPLMGEYAGA
                   310       320       330       340       350       360

                   370       380       390       400       410       420
m919.pep   VDRHYITLGAPLFVATAHPVTRKALNRLIMAQDTGSAIKGAVRVDYFWGYGDEAGELAGK
           ||||||||||||||||||||||||||||||||||||||||||||||||||||||||||||
a919       VDRHYITLGAPLFVATAHPVTRKALNRLIMAQDTGSAIKGAVRVDYFWGYGDEAGELAGK
                   370       380       390       400       410       420

                   430       440
m919.pep   QKTTGYVWQLLPNGMKPEYRPX
           ||||||||||||||||||||||
a919       QKTTGYVWQLLPNGMKPEYRPX
           430       440
```

## 121 and 121-1

The following partial DNA sequence was identified in *N. meningitidis* <SEQ ID 40>:

**m121.seq**

```
   1  ATGGAAACAC AGCTTTACAT CGGCATCATG TCGGGAACCA GCATGGACGG
  51  GGCGGATGCC GTACTGATAC GGATGGACGG CGGCAAATGG CTGGGCGCGG
 101  AAGGGCACGC CTTTACCCCC TACCCCGGCA GGTTACGCCG CCAATTGCTG
 151  GATTTGCAGG ACACAGGCGC AGACGAACTG CACCGCAGCA GGATTTTGTC
 201  GCAAGAACTC AGCCGCCTAT ATGCGCAAAC CGCCGCCGAA CTGCTGTGCA
 251  GTCAAAACCT CGCACCGTCC GACATTACCG CCCTCGGCTG CCACGGGCAA
 301  ACCGTCCGAC ACGCGCCGGA ACACGGTTAC AGCATACAGC TTGCCGATTT
 351  GCCGCTGCTG GCGxxxxxxx xxxxxxxxxx xxxxxxxxxx xxxxxxxxxx
 401  xxxxxxxxxx xxxxxxxxxx xxxxxxxxxx xxxxxxxxxx xxxxxxxxxx
 451  xxxxxxxxxx xxxxxxxxxx xxxxxxxxxx xxxxxxxxxx xxxxxxxxxx
 501  xxxxxxxxxx xxxxxxxxxx xxxxxxxxxx xxxxxxxxxx xxxxxxxxxx
 551  xxxxxxxxxx xxxxxxxxxx xxxxxxxxxx xxxxxxxxxx xxxxxxxxxx
 601  xxxxxxCAGC TTCCTTACGA CAAAAACGGT GCAAAGTCGG CACAAGGCAA
 651  CATATTGCCG CAACTGCTCG ACAGGCTGCT CGCCCACCCG TATTTCGCAC
 701  AACGCCACCC TAAAAGCACG GGGCGCGAAC TGTTTGCCAT AAATTGGCTC
 751  GAAACCTACC TTGACGGCGG CGAAAACCGA TACGACGTAT TGCGGACGCT
 801  TTCCCGTTTT ACCGCGCAAA CCGTTTGCGA CGCCGTCTCA CACGCAGCGG
 851  CAGATGCCCG TCAAATGTAC ATTTGCGACG GCGGCATCCG CAATCCTGTT
 901  TTAATGGCGG ATTTGGCAGA ATGTTTCGGC ACACGCGTTT CCCTGCACAG
 951  CACCGCCGAC CTGAACCTCG ATCCGCAATG GGTGGAAGCC GCCGnATTTG
1001  CGTGGTTGGC GGCGTGTTGG ATTAATCGCA TTCCCGGTAG TCCGCACAAA
1051  GCAACCGGCG CATCCAAACC GTGTATTCTG AnCGCGGGAT ATTATTATTG
1101  A
```

This corresponds to the amino acid sequence <SEQ ID 41; ORF 121>:

**m121.pep**

```
   1  METQLYIGIM SGTSMDGADA VLIRMDGGKW LGAEGHAFTP YPGRLRRQLL
  51  DLQDTGADEL HRSRILSQEL SRLYAQTAAE LLCSQNLAPS DITALGCHGQ
```

```
101  TVRHAPEHGY SIQLADLPLL Axxxxxxxxx xxxxxxxxxx xxxxxxxxxx
151  xxxxxxxxxx xxxxxxxxxx xxxxxxxxxx xxxxxxxxxx xxxxxxxxxx
201  xxQLPYDKNG AKSAQGNILP QLLDRLLAHP YFAQRHPKST GRELFAINWL
251  ETYLDGGENR YDVLRTLSRF TAQTVCDAVS HAAADARQMY ICDGGIRNPV
301  LMADLAECFG TRVSLHSTAD LNLDPQWVEA AXFAWLAACW INRIPGSPHK
351  ATGASKPCIL XAGYYY*
```

The following partial DNA sequence was identified in *N. gonorrhoeae* <SEQ ID 42>:

**g121.seq**

```
   1  ATGGAAACAC AGCTTTACAT CGGCATTATG TCGGGAACCA GTATGGACGG
  51  GGCGGATGCC GTGCTGGTAC GGATGGACGG CGGCAAATGG CTGGGCGCGG
 101  AAGGGCACGC CTTTACCCCC TACCCTGACC GGTTGCGCCG CAAATTGCTG
 151  GATTTGCAGG ACACAGGCAC AGACGAACTG CACCGCAGCA GGATGTTGTC
 201  GCAAGAACTC AGCCGCCTGT ACGCGCAAAC CGCCGCCGAA CTGCTGTGCA
 251  GTCAAAACCT CGCTCCGTGC GACATTACCG CCCTCGGCTG CCACGGGCAA
 301  ACCGTCCGAC ACGCGCCGGA ACACGGTtac AGCATACAGC TTGCCGATTT
 351  GCCGCTGCTG GCGGAACTGa cgcggatttT TACCGTCggc gacttcCGCA
 401  GCCGCGACCT TGCTGCCGGC GGacaAGGTG CGCCGCTCGT CCCCGCCTTT
 451  CACGAAGCCC TGTTCCGCGA TGACAGGGAA ACACGCGTGG TACTGAACAT
 501  CGGCGGGATT GCCAACATCA GCGTACTCCC CCCCGGCGCA CCCGCCTTCG
 551  GCTTCGACAC AGGGCCGGGC AATATGCTGA TGGAcgcgtg gacgcaggca
 601  cacTGGcagc TGCCTTACGA CAAAAacggt gcAAAGgcgg cacAAGGCAA
 651  catatTGCcg cAACTGCTCG gcaggctGCT CGCCcaccCG TATTTCTCAC
 701  AACCCcaccc aaAAAGCACG GGgcGCGaac TgtttgcccT AAattggctc
 751  gaaacctAcc ttgacggcgg cgaaaaccga tacgacgtat tgcggacgct
 801  ttcccgattc accgcgcaaA ccgTttggga cgccgtctca CACGCAGCGG
 851  CAGATGCCCG TCAAATGTAC ATTTGCGGCG GCGGCATCCG CAATCCTGTT
 901  TTAATGGCGG ATTTGGCAGA ATGTTTCGGC ACACGCGTTT CCCTGCACAG
 951  CACCGCCGAA CTGAACCTCG ATCCTCAATG GGTGGAGGCG gccgCATTtg
1001  cgtggttggC GGCGTGTTGG ATTAACCGCA TTCCCGGTAG TCCGCACAAA
1051  GCGACCGGCG CATCCAAACC GTGTATTCTG GGCGCGGGAT ATTATTATTG
1101  A
```

This corresponds to the amino acid sequence <SEQ ID 43; ORF 121.ng>:

**g121.pep**

```
  1  METQLYIGIM SGTSMDGADA VLVRMDGGKW LGAEGHAFTP YPDRLRRKLL
 51  DLQDTGTDEL HRSRMLSQEL SRLYAQTAAE LLCSQNLAPC DITALGCHGQ
101  TVRHAPEHGY SIQLADLPLL AELTRIFTVG DFRSRDLAAG GQGAPLVPAF
151  HEALFRDDRE TRVVLNIGGI ANISVLPPGA PAFGFDTGPG NMLMDAWTQA
201  HWQLPYDKNG AKAAQGNILP QLLGRLLAHP YFSQPHPKST GRELFALNWL
251  ETYLDGGENR YDVLRTLSRF TAQTVWDAVS HAAADARQMY ICGGGIRNPV
301  LMADLAECFG TRVSLHSTAE LNLDPQWVEA AAFAWLAACW INRIPGSPHK
351  ATGASKPCIL GAGYYY*
```

ORF 121 shows 73.5% identity over a 366 aa overlap with a predicted ORF (ORF121.ng) from *N. gonorrhoeae:*

**m121/g121**

```
                     10        20        30        40        50        60
m121.pep     METQLYIGIMSGTSMDGADAVLIRMDGGKWLGAEGHAFTPYPGRLRRQLLDLQDTGADEL
             ||||||||||||||||||||||:||||||||||||||||||| ||||:||||||||:|||
g121         METQLYIGIMSGTSMDGADAVLVRMDGGKWLGAEGHAFTPYPDRLRRKLLDLQDTGTDEL
                     10        20        30        40        50        60
                     70        80        90       100       110       120
m121.pep     HRSRILSQELSRLYAQTAAELLCSQNLAPSDITALGCHGQTVRHAPEHGYSIQLADLPLL
             ||||:||||||||||||||||||||||||| |||||||||||||||||||||||||||||
g121         HRSRMLSQELSRLYAQTAAELLCSQNLAPCDITALGCHGQTVRHAPEHGYSIQLADLPLL
                     70        80        90       100       110       120
                    130       140       150       160       170       180
```

```
m121.pep     AXXXXXXXXXXXXXXXXXXXXXXXXXXXXXXXXXXXXXXXXXXXXXXXXXXXXXXXXXXX
             | :   :                                   :
g121         AELTRIFTVGDFRSRDLAAGGQGAPLVPAFHEALFRDDRETRVVLNIGGIANISVLPPGA
                    130       140       150       160       170       180
                    190       200       210       220       230       240
m121.pep     XXXXXXXXXXXXXXXXXXXXXQLPYDKNGAKSAQGNILPQLLDRLLAHPYFAQRHPKST
                  :         :     ||||||||||:|||||||||| ||||||||:| |||||
g121         PAFGFDTGPGNMLMDAWTQAHWQLPYDKNGAKAAQGNILPQLLGRLLAHPYFSQPHPKST
                    190       200       210       220       230       240
                    250       260       270       280       290       300
m121.pep     GRELFAINWLETYLDGGENRYDVLRTLSRFTAQTVCDAVSHAAADARQMYICDGGIRNPV
             ||||||:||||||||||||||||||||||||||||| ||||||||||||||||| |||||||
g121         GRELFALNWLETYLDGGENRYDVLRTLSRFTAQTVWDAVSHAAADARQMYICGGGIRNPV
                    250       260       270       280       290       300
                    310       320       330       340       350       360
m121.pep     LMADLAECFGTRVSLHSTADLNLDPQWVEAAXFAWLAACWINRIPGSPHKATGASKPCIL
             |||||||||||||||||||:|||||||||||| |||||||||||||||||||||||||||
g121         LMADLAECFGTRVSLHSTAELNLDPQWVEAAAFAWLAACWINRIPGSPHKATGASKPCIL
                    310       320       330       340       350       360

m121.pep     XAGYYYX
              ||||||
g121         GAGYYYX
```

The following partial DNA sequence was identified in *N. meningitidis* <SEQ ID 44>:

```
a121.seq
   1  ATGGAAACAC AGCTTTACAT CGGCATCATG TCGGGAACCA GCATGGACGG
  51  GGCGGATGCC GTACTGATAC GGATGGACGG CGGCAAATGG CTGGGCGCGG
 101  AAGGGCACGC CTTTACCCCC TACCCCGGCA GGTTACGCCG CAAATTGCTG
 151  GATTTGCAGG ACACAGGCGC GGACGAACTG CACCGCAGCA GGATGTTGTC
 201  GCAAGAACTC AGCCGCCTGT ACGCGCAAAC CGCCGCCGAA CTGCTGTGCA
 251  GTCAAAACCT CGCGCCGTCC GACATTACCG CCCTCGGCTG CCACGGGCAA
 301  ACCGTCAGAC ACGCGCCGGA ACACAGTTAC AGCGTACAGC TTGCCGATTT
 351  GCCGCTGCTG GCGGAACGGA CTCAGATTTT TACCGTCGGC GACTTCCGCA
 401  GCCGCGACCT TGCGGCCGGC GGACAAGGCG CGCCGCTCGT CCCCGCCTTT
 451  CACGAAGCCC TGTTCCGCGA CGACAGGGAA ACACGCGCGG TACTGAACAT
 501  CGGCGGGATT GCCAACATCA GCGTACTCCC CCCCGACGCA CCCGCCTTCG
 551  GCTTCGACAC AGGACCGGGC AATATGCTGA TGGACGCGTG GATGCAGGCA
 601  CACTGGCAGC TTGCTTACGA CAAAAACGGT GCAAAGGCGG CACAAGGCAA
 651  CATATTGCCG CAACTGCTCG ACAGGCTGCT CGCCCACCCG TATTTCGCAC
 701  AACCCCACCC TAAAAGCACG GGGCGCGAAC TGTTTGCCCT AAATTGGCTC
 751  GAAACCTACC TTGACGGCGG CGAAAACCGA TACGACGTAT TGCGGACGCT
 801  TTCCCGATTC ACCGCGCAAA CCGTTTTCGA CGCCGTCTCA CACGCAGCGG
 851  CAGATGCCCG TCAAATGTAC ATTTGCGGCG GCGGCATCCG CAATCCTGTT
 901  TTAATGGCGG ATTTGGCAGA ATGTTTCGGC ACACGCGTTT CCCTGCACAG
 951  CACCGCCGAA CTGAACCTCG ATCCGCAATG GGTAGAAGCC GCCGCGTTCG
1001  CATGGATGGC GGCGTGTTGG GTCAACCGCA TTCCCGGTAG TCCGCACAAA
1051  GCAACCGGCG CATCCAAACC GTGTATTCTG GGCGCGGGAT ATTATTATTG
1101  A
```

This corresponds to the amino acid sequence <SEQ ID 45; ORF 121.a>:

```
a121.pep
   1  METQLYIGIM SGTSMDGADA VLIRMDGGKW LGAEGHAFTP YPGRLRRKLL
  51  DLQDTGADEL HRSRMLSQEL SRLYAQTAAE LLCSQNLAPS DITALGCHGQ
 101  TVRHAPEHSY SVQLADLPLL AERTQIFTVG DFRSRDLAAG GQGAPLVPAF
 151  HEALFRDDRE TRAVLNIGGI ANISVLPPDA PAFGFDTGPG NMLMDAWMQA
 201  HWQLPYDKNG AKAAQGNILP QLLDRLLAHP YFAQPHPKST GRELFALNWL
 251  ETYLDGGENR YDVLRTLSRF TAQTVFDAVS HAAADARQMY ICGGGIRNPV
 301  LMADLAECFG TRVSLHSTAE LNLDPQWVEA AAFAWMAACW VNRIPGSPHK
```

```
351  ATGASKPCIL GAGYYY*
```

**m121/a121** **ORFs 121 and 121.a** 74.0% identity in 366 aa overlap

```
                    10        20        30        40        50        60
m121.pep    METQLYIGIMSGTSMDGADAVLIRMDGGKWLGAEGHAFTPYPGRLRRQLLDLQDTGADEL
            ||||||||||||||||||||||||||||||||||||||||||||||:|||||||||||||
a121        METQLYIGIMSGTSMDGADAVLIRMDGGKWLGAEGHAFTPYPGRLRRKLLDLQDTGADEL
                    10        20        30        40        50        60

                    70        80        90       100       110       120
m121.pep    HRSRILSQELSRLYAQTAAELLCSQNLAPSDITALGCHGQTVRHAPEHGYSIQLADLPLL
            ||||:|||||||||||||||||||||||||||||||||||||||||||:||:||||||||
a121        HRSRMLSQELSRLYAQTAAELLCSQNLAPSDITALGCHGQTVRHAPEHSYSVQLADLPLL
                    70        80        90       100       110       120

                   130       140       150       160       170       180
m121.pep    AXXXXXXXXXXXXXXXXXXXXXXXXXXXXXXXXXXXXXXXXXXXXXXXXXXXXXXXXXXX
            | :   :                                     :
a121        AERTQIFTVGDFRSRDLAAGGQGAPLVPAFHEALFRDDRETRAVLNIGGIANISVLPPDA
                   130       140       150       160       170       180

                   190       200       210       220       230       240
m121.pep    XXXXXXXXXXXXXXXXXXXXXQLPYDKNGAKSAQGNILPQLLDRLLAHPYFAQRHPKST
                  :                ||||||||||:||||||||||||||||||||| |||||
a121        PAFGFDTGPGNMLMDAWMQAHWQLPYDKNGAKAAQGNILPQLLDRLLAHPYFAQPHPKST
                   190       200       210       220       230       240

                   250       260       270       280       290       300
m121.pep    GRELFAINWLETYLDGGENRYDVLRTLSRFTAQTVCDAVSHAAADARQMYICDGGIRNPV
            ||||||:|||||||||||||||||||||||||||||| ||||||||||||||||| |||||||
a121        GRELFALNWLETYLDGGENRYDVLRTLSRFTAQTVFDAVSHAAADARQMYICGGGIRNPV
                   250       260       270       280       290       300

                   310       320       330       340       350       360
m121.pep    LMADLAECFGTRVSLHSTADLNLDPQWVEAAXFAWLAACWINRIPGSPHKATGASKPCIL
            ||||||||||||||||||:|||||||||||| |||:||||:||||||||||||||||||||
a121        LMADLAECFGTRVSLHSTAELNLDPQWVEAAAFAWMAACWVNRIPGSPHKATGASKPCIL
                   310       320       330       340       350       360

m121.pep    XAGYYYX
             ||||||
a121        GAGYYYX
```

Further work revealed the DNA sequence identified in *N. meningitidis* <SEQ ID 46>:

```
m121-1.seq
      1  ATGGAAACAC AGCTTTACAT CGGCATCATG TCGGGAACCA GCATGGACGG
     51  GGCGGATGCC GTACTGATAC GGATGGACGG CGGCAAATGG CTGGGCGCGG
    101  AAGGGCACGC CTTTACCCCC TACCCCGGCA GGTTACGCCG CCAATTGCTG
    151  GATTTGCAGG ACACAGGCGC AGACGAACTG CACCGCAGCA GGATTTTGTC
    201  GCAAGAACTC AGCCGCCTAT ATGCGCAAAC CGCCGCCGAA CTGCTGTGCA
    251  GTCAAAACCT CGCACCGTCC GACATTACCG CCCTCGGCTG CCACGGGCAA
    301  ACCGTCCGAC ACGCGCCGGA ACACGGTTAC AGCATACAGC TTGCCGATTT
    351  GCCGCTGCTG GCGGAACGGA CGCGGATTTT TACCGTCGGC GACTTCCGCA
    401  GCCGCGACCT TGCGGCCGGC GGACAAGGCG CGCCACTCGT CCCCGCCTTT
    451  CACGAAGCCC TGTTCCGCGA CAACAGGGAA ACACGCGCGG TACTGAACAT
    501  CGGCGGGATT GCCAACATCA GCGTACTCCC CCCCGACGCA CCCGCCTTCG
    551  GCTTCGACAC AGGGCCGGGC AATATGCTGA TGGACGCGTG GACGCAGGCA
    601  CACTGGCAGC TTCCTTACGA CAAAAACGGT GCAAAGGCGG CACAAGGCAA
    651  CATATTGCCG CAACTGCTCG ACAGGCTGCT CGCCCACCCG TATTTCGCAC
    701  AACCCCACCC TAAAAGCACG GGGCGCGAAC TGTTTGCCCT AAATTGGCTC
    751  GAAACCTACC TTGACGGCGG CGAAAACCGA TACGACGTAT TGCGGACGCT
```

```
 801  TTCCCGTTTT ACCGCGCAAA CCGTTTGCGA CGCCGTCTCA CACGCAGCGG
 851  CAGATGCCCG TCAAATGTAC ATTTGCGGCG GCGGCATCCG CAATCCTGTT
 901  TTAATGGCGG ATTTGGCAGA ATGTTTCGGC ACACGCGTTT CCCTGCACAG
 951  CACCGCCGAC CTGAACCTCG ATCCGCAATG GGTGGAAGCC GCCGNATTTG
1001  CGTGGTTGGC GGCGTGTTGG ATTAATCGCA TTCCCGGTAG TCCGCACAAA
1051  GCAACCGGCG CATCCAAACC GTGTATTCTG ANCGCGGGAT ATTATTATTG
1101  A
```

This corresponds to the amino acid sequence <SEQ ID 47: ORF 121-1>:

```
m121-1.pep
      1  METQLYIGIM SGTSMDGADA VLIRMDGGKW LGAEGHAFTP YPGRLRRQLL
     51  DLQDTGADEL HRSRILSQEL SRLYAQTAAE LLCSQNLAPS DITALGCHGQ
    101  TVRHAPEHGY SIQLADLPLL AERTRIFTVG DFRSRDLAAG GQGAPLVPAF
    151  HEALFRDNRE TRAVLNIGGI ANISVLPPDA PAFGFDTGPG NMLMDAWTQA
    201  HWQLPYDKNG AKAAQGNILP QLLDRLLAHP YFAQPHPKST GRELFALNWL
    251  ETYLDGGENR YDVLRTLSRF TAQTVCDAVS HAAADARQMY ICGGGIRNPV
    301  LMADLAECFG TRVSLHSTAD LNLDPQWVEA AXFAWLAACW INRIPGSPHK
    351  ATGASKPCIL XAGYYY*
```

**m121-1/g121 ORFs 121-1 and 121-1.ng showed a** 95.6% identity in 366 aa overlap

```
                       10        20        30        40        50        60
m121-1.pep  METQLYIGIMSGTSMDGADAVLIRMDGGKWLGAEGHAFTPYPGRLRRQLLDLQDTGADEL
            ||||||||||||||||||||||:||||||||||||||||||| ||||:||||||||:|||
g121        METQLYIGIMSGTSMDGADAVLVRMDGGKWLGAEGHAFTPYPDRLRRKLLDLQDTGTDEL
                       10        20        30        40        50        60

                       70        80        90       100       110       120
m121-1.pep  HRSRILSQELSRLYAQTAAELLCSQNLAPSDITALGCHGQTVRHAPEHGYSIQLADLPLL
            ||||:|||||||||||||||||||||||| ||||||||||||||||||||||||||||||
g121        HRSRMLSQELSRLYAQTAAELLCSQNLAPCDITALGCHGQTVRHAPEHGYSIQLADLPLL
                       70        80        90       100       110       120

                      130       140       150       160       170       180
m121-1.pep  AERTRIFTVGDFRSRDLAAGGQGAPLVPAFHEALFRDNRETRAVLNIGGIANISVLPPDA
            || ||||||||||||||||||||||||||||||||||||| |||:||||||||||||||| |
g121        AELTRIFTVGDFRSRDLAAGGQGAPLVPAFHEALFRDDRETRVVLNIGGIANISVLPPGA
                      130       140       150       160       170       180

                      190       200       210       220       230       240
m121-1.pep  PAFGFDTGPGNMLMDAWTQAHWQLPYDKNGAKAAQGNILPQLLDRLLAHPYFAQPHPKST
            ||||||||||||||||||||||||||||||||||||||||| ||||||||:|||||||
g121        PAFGFDTGPGNMLMDAWTQAHWQLPYDKNGAKAAQGNILPQLLGRLLAHPYFSQPHPKST
                      190       200       210       220       230       240

                      250       260       270       280       290       300
m121-1.pep  GRELFALNWLETYLDGGENRYDVLRTLSRFTAQTVCDAVSHAAADARQMYICGGGIRNPV
            |||||||||||||||||||||||||||||||||| |||||||||||||||||||||||||
g121        GRELFALNWLETYLDGGENRYDVLRTLSRFTAQTVWDAVSHAAADARQMYICGGGIRNPV
                      250       260       270       280       290       300

                      310       320       330       340       350       360
m121-1.pep  LMADLAECFGTRVSLHSTADLNLDPQWVEAAXFAWLAACWINRIPGSPHKATGASKPCIL
            ||||||||||||||||||:||||||||||| ||||||||||||||||||||||||||||||
g121        LMADLAECFGTRVSLHSTAELNLDPQWVEAAAFAWLAACWINRIPGSPHKATGASKPCIL
                      310       320       330       340       350       360

m121-1.pep  XAGYYYX
             ||||||
g121        GAGYYYX
```

The following partial DNA sequence was identified in *N. meningitidis* <SEQ ID 48>:

```
a121-1.seq
      1  ATGGAAACAC AGCTTTACAT CGGCATCATG TCGGGAACCA GCATGGACGG
     51  GGCGGATGCC GTACTGATAC GGATGGACGG CGGCAAATGG CTGGGCGCGG
    101  AAGGGCACGC CTTTACCCCC TACCCCGGCA GGTTACGCCG CAAATTGCTG
    151  GATTTGCAGG ACACAGGCGC GGACGAACTG CACCGCAGCA GGATGTTGTC
    201  GCAAGAACTC AGCCGCCTGT ACGCGCAAAC CGCCGCCGAA CTGCTGTGCA
    251  GTCAAAACCT CGCGCCGTCC GACATTACCG CCCTCGGCTG CCACGGGCAA
    301  ACCGTCAGAC ACGCGCCGGA ACACAGTTAC AGCGTACAGC TTGCCGATTT
    351  GCCGCTGCTG GCGGAACGGA CTCAGATTTT TACCGTCGGC GACTTCCGCA
    401  GCCGCGACCT TGCGGCCGGC GGACAAGGCG CGCCGCTCGT CCCCGCCTTT
    451  CACGAAGCCC TGTTCCGCGA CGACAGGGAA ACACGCGCGG TACTGAACAT
    501  CGGCGGGATT GCCAACATCA GCGTACTCCC CCCCGACGCA CCCGCCTTCG
    551  GCTTCGACAC AGGACCGGGC AATATGCTGA TGGACGCGTG GATGCAGGCA
    601  CACTGGCAGC TTCCTTACGA CAAAAACGGT GCAAAGGCGG CACAAGGCAA
    651  CATATTGCCG CAACTGCTCG ACAGGCTGCT CGCCCACCCG TATTTCGCAC
    701  AACCCCACCC TAAAAGCACG GGGCGCGAAC TGTTTGCCCT AAATTGGCTC
    751  GAAACCTACC TTGACGGCGG CGAAAACCGA TACGACGTAT TGCGGACGCT
    801  TTCCCGATTC ACCGCGCAAA CCGTTTTCGA CGCCGTCTCA CACGCAGCGG
    851  CAGATGCCCG TCAAATGTAC ATTTGCGGCG GCGGCATCCG CAATCCTGTT
    901  TTAATGGCGG ATTTGGCAGA ATGTTTCGGC ACACGCGTTT CCCTGCACAG
    951  CACCGCCGAA CTGAACCTCG ATCCGCAATG GGTAGAAGCC GCCGCGTTCG
   1001  CATGGATGGC GGCGTGTTGG GTCAACCGCA TTCCCGGTAG TCCGCACAAA
   1051  GCAACCGGCG CATCCAAACC GTGTATTCTG GGCGCGGGAT ATTATTATTG
   1101  A
```

This corresponds to the amino acid sequence <SEQ ID 49; ORF 121-1.a>:

```
a121-1.pep
      1  METQLYIGIM SGTSMDGADA VLIRMDGGKW LGAEGHAFTP YPGRLRRKLL
     51  DLQDTGADEL HRSRMLSQEL SRLYAQTAAE LLCSQNLAPS DITALGCHGQ
    101  TVRHAPEHSY SVQLADLPLL AERTQIFTVG DFRSRDLAAG GQGAPLVPAF
    151  HEALFRDDRE TRAVLNIGGI ANISVLPPDA PAFGFDTGPG NMLMDAWMQA
    201  HWQLPYDKNG AKAAQGNILP QLLDRLLAHP YFAQPHPKST GRELFALNWL
    251  ETYLDGGENR YDVLRTLSRF TAQTVFDAVS HAAADARQMY ICGGGIRNPV
    301  LMADLAECFG TRVSLHSTAE LNLDPQWVEA AAFAWMAACW VNRIPGSPHK
    351  ATGASKPCIL GAGYYY*
```

**m121-1/a121-1 ORFs 121-1 and 121-1.a showed a 96.4% identity in 366 aa overlap**

```
                     10        20        30        40        50        60
m121-1.pep   METQLYIGIMSGTSMDGADAVLIRMDGGKWLGAEGHAFTPYPGRLRRQLLDLQDTGADEL
             |||||||||||||||||||||||||||||||||||||||||||||||:||||||||||||
a121-1       METQLYIGIMSGTSMDGADAVLIRMDGGKWLGAEGHAFTPYPGRLRRKLLDLQDTGADEL
                     10        20        30        40        50        60

                     70        80        90       100       110       120
m121-1.pep   HRSRILSQELSRLYAQTAAELLCSQNLAPSDITALGCHGQTVRHAPEHGYSIQLADLPLL
             ||||:|||||||||||||||||||||||||||||||||||||||||||:||:||||||||
a121-1       HRSRMLSQELSRLYAQTAAELLCSQNLAPSDITALGCHGQTVRHAPEHSYSVQLADLPLL
                     70        80        90       100       110       120

                    130       140       150       160       170       180
m121-1.pep   AERTRIFTVGDFRSRDLAAGGQGAPLVPAFHEALFRDNRETRAVLNIGGIANISVLPPDA
             ||||:||||||||||||||||||||||||||||||||:||||||||||||||||||||||
a121-1       AERTQIFTVGDFRSRDLAAGGQGAPLVPAFHEALFRDDRETRAVLNIGGIANISVLPPDA
                    130       140       150       160       170       180

                    190       200       210       220       230       240
m121-1.pep   PAFGFDTGPGNMLMDAWTQAHWQLPYDKNGAKAAQGNILPQLLDRLLAHPYFAQPHPKST
             ||||||||||||||||| ||||||||||||||||||||||||||||||||||||||||||
a121-1       PAFGFDTGPGNMLMDAWMQAHWQLPYDKNGAKAAQGNILPQLLDRLLAHPYFAQPHPKST
                    190       200       210       220       230       240
```

```
                  250        260        270        280        290        300
m121-1.pep  GRELFALNWLETYLDGGENRYDVLRTLSRFTAQTVCDAVSHAAADARQMYICGGGIRNPV
            ||||||||||||||||||||||||||||||||||| ||||||||||||||||||||||||
a121-1      GRELFALNWLETYLDGGENRYDVLRTLSRFTAQTVFDAVSHAAADARQMYICGGGIRNPV
                  250        260        270        280        290        300

                  310        320        330        340        350        360
m121-1.pep  LMADLAECFGTRVSLHSTADLNLDPQWVEAAXFAWLAACWINRIPGSPHKATGASKPCIL
            |||||||||||||||||||:||||||||||| |||:||||:|||||||||||||||||||
a121        LMADLAECFGTRVSLHSTAELNLDPQWVEAAAFAWMAACWVNRIPGSPHKATGASKPCIL
                  310        320        330        340        350        360

m121-1.pep  XAGYYYX
             ||||||
a121        GAGYYYX
```

## 128 and 128-1

The following partial DNA sequence was identified in *N. meningitidis* <SEQ ID 50>:

```
m128.seq  (partial)
      1  ATGACTGACA ACGCACTGCT CCATTTGGGC GAAGAACCCC GTTTTGATCA
     51  AATCAAAACC GAAGACATCA AACCCGCCCT GCAAACCGCC ATCGCCGAAG
    101  CGCGCGAACA AATCGCCGCC ATCAAAGCCC AAACGCACAC CGGCTGGGCA
    151  AACACTGTCG AACCCCTGAC CGGCATCACC GAACGCGTCG GCAGGATTTG
    201  GGGCGTGGTG TCGCACCTCA ACTGCGTCGC CGACACGCCC GAACTGCGCG
    251  CCGTCTATAA CGAACTGATG CCCGAAATCA CCGTCTTCTT CACCGAAATC
    301  GGACAAGACA TCGAGCTGTA CAACCGCTTC AAAACCATCA AAAATTCCCC
    351  CGAATTCGAC ACCCTCTCCC CCGCACAAAA AACCAAACTC AACCAC
      1  TACGCCAGCG AAAAACTGCG CGAAGCCAAA TACGCGTTCA GCGAAACCGA
     51  wGTCAAAAAA TAyTTCCCyG TCGGCAAwGT ATTAAACGGA CTGTTCGCCC
    101  AAmTCAAAAA ACTmTACGGC ATCGGATTTA CCGAAAAAAC yGTCCCCGTC
    151  TGGCACAAAG ACGTGCGCTA TTkTGAATTG CAACAAAACG GCGAAmCCAT
    201  AGGCGGCGTT TATATGGATT TGTACGCACG CGAAGGCAAA CGCGGCGGCG
    251  CGTGGATGAA CGACTACAAA GGCCGCCGCC GTTTTTCAGA CGGCACGCTG
    301  CAAyTGCCCA CCGCCTACCT CGTCTGCAAC TTCGCCCCAC CCGTCGGCGG
    351  CAGGGAAGCC CGCyTGAGCC ACGACGAAAT CCTCATCCTC TTCCACGAAA
    401  CCGGACACGG GCTGCACCAC CTGCTTACCC AAGTGGACGA ACTGGGCGTA
    451  TCCGGCATCA ACGGCGTAkA ATGGGACGCG GTCGAACTGC CCAGCCAGTT
    501  TATGGAAAAT TTCGTTTGGG AATACAATGT CTTGGCACAA mTGTCAGCCC
    551  ACGAAGAAAC CGGcgTTCCC yTGCCGAAAG AACTCTTsGA CAAAwTGCTC
    601  GCCGCCAAAA ACTTCCAAsG CGGCATGTTC yTsGTCCGGC AAwTGGAGTT
    651  CGCCCTCTTT GATATGATGA TTTACAGCGA AGACGACGAA GGCCGTCTGA
    701  AAAACTGGCA ACAGGTTTTA GACAGCGTGC GCAAAAAAGT CGCCGTCATC
    751  CAGCCGCCCG AATACAACCG CTTCGCCTTG AGCTTCGGCC ACATCTTCGC
    801  AGGCGGCTAT TCCGCAGCTn ATTACAGCTA CGCGTGGGCG GAAGTATTGA
    851  GCGCGGACGC ATACGCCGCC TTTGAAGAAA GCGACGATGT CGCCGCCACA
    901  GGCAAACGCT TTTGGCAGGA AATCCTCGCC GTCGGGGnAT CGCGCAGCGG
    951  nGCAGAATCC TTCAAAGCCT TCCGCGGCCG CGAACCGAGC ATAGACGCAC
   1001  TCTTGCGCCA CAGCGGTTTC GACAACGCGG TCTGA
```

This corresponds to the amino acid sequence <SEQ ID 51; ORF 128>:

```
m128.pep   (partial)
      1  MTDNALLHLG EEPRFDQIKT EDIKPALQTA IAEAREQIAA IKAQTHTGWA
     51  NTVEPLTGIT ERVGRIWGVV SHLNCVADTP ELRAVYNELM PEITVFFTEI
    101  GQDIELYNRF KTIKNSPEFD TLSPAQKTKL NH
//
      1  YASEKLREAK YAFSETXVKK YFPVGXVLNG LFAQXKKLYG IGFTEKTVPV
```

```
 51  WHKDVRYXEL QQNGEXIGGV YMDLYAREGK RGGAWMNDYK GRRRFSDGTL
101  QLPTAYLVCN FAPPVGGREA RLSHDEILIL FHETGHGLHH LLTQVDELGV
151  SGINGVXWDA VELPSQFMEN FVWEYNVLAQ XSAHEETGVP LPKELXDKXL
201  AAKNFQXGMF XVRQXEFALF DMMIYSEDDE GRLKNWQQVL DSVRKKVAVI
251  QPPEYNRFAL SFGHIFAGGY SAAXYSYAWA EVLSADAYAA FEESDDVAAT
301  GKRFWQEILA VGXSRSGAES FKAFRGREPS IDALLRHSGF DNAV*
```

The following partial DNA sequence was identified in *N. gonorrhoeae* <SEQ ID 52>:

```
g128.seq
   1  atgattgaca acgCActgct ccacttgggc gaagaaccCC GTTTTaatca
  51  aatccaaacc gaagACAtca AACCCGCCGT CCAAACCGCC ATCGCCGAAG
 101  CGCGCGGACA AATCGCCGCC GTCAAAGCGC AAACGCACAC CGGCTGGGCG
 151  AACACCGTCG AGCGTCTGAC CGGCATCACC GAACGCGTCG GCAGGATTTG
 201  GGGCGTCGTG TCCCATCTCA ACTCCGTCGT CGACACGCCC GAACTGCGCG
 251  CCGTCTATAA CGAACTGATG CCTGAAATCA CCGTCTTCTT CACCGAAATC
 301  GGACAAGACA TCGAACTGTA CAACCGCTTC AAAACCATCA AAAATTCCCC
 351  CGAATTTGCA ACGCTTTCCC CCGCACAAAA AACCAAGCTC GATCACGACC
 401  TGCGCGATTT CGTATTGAGC GGCGCGGAAC TGCCGCCCGA ACGGCAGGCA
 451  GAACTGGCAA AACTGCAAAC CGAAGGCGCG CAACTTTCCG CCAAATTCTC
 501  CCAAAACGTC CTAGACGCGA CCGACGCGTT CGGCATTTAC TTTGACGATG
 551  CCGCACCGCT TGCCGGCATT CCCGAAGACG CGCTCGCCAT GTTTGCCGCC
 601  GCCGCGCAAA GCGAAGGCAA AACAGGTTAC AAAATCGGCT TGCAGATTCC
 651  GCACTACCTT GCCGTTATCC AATACGCCGG CAACCGCGAA CTGCGCGAAC
 701  AAATCTACCG CGCCTACGTT ACCCGTGCCA GCGAACTTTC AAACGACGGC
 751  AAATTCGACA ACACCGCCAA CATCGACCGC ACGCTCGAAA ACGCATTGAA
 801  AACCGccaaa cTGCTCGGCT TTAAAAATTA CGCCGAATTG TCGCTGGCAA
 851  CCAAAATGGC GGACACGCCC GAACAGGTTT TAAACTTCCT GCACGACCTC
 901  GCCCGCCGCG CCAAACCCTA CGCCGAAAAA GACCTCGCCG AAGTCAAAGC
 951  CTTCGCCCGC GAACACCTCG GTCTCGCCGA CCCGCAGCCG TGGGACTTGA
1001  GCTACGCCGG CGAAAAACTG CGCGAAGCCA AATACGCATT CAGCGAAACC
1051  GAAGTCAAAA AATACTTCCC CGTCGGCAAA GTTCTGGCAG GCCTGTTCGC
1101  CCAAATCAAA AAACTCTACG GCATCGGATT CGCCGAAAAA ACCGTTCCCG
1151  TCTGGCACAA AGACGTGCGC TATTTTGAAT TGCAACAAAA CGGCAAAACC
1201  ATCGGCGGCG TTTATATGGA TTTGTACGCA CGCGAAGGCA AACGCGGCGG
1251  CGCGTGGATG AACGACtaca AAGGCCGCCG CCGCTTTGCC GACGgcacGC
1301  TGCAACTGCC CACCGCCTAC CTCGTCTGCA ACTTCGCCCC GCCCGTCGGC
1351  GGCAAAGAAG CGCGTTTAAG CCACGACGAA ATCCTCACCC TCTTCCACGA
1401  AacCGGCCAC GGACTGCACC ACCTGCTTAC CCAAGTGGAC GAACTGGGCG
1451  TGTCCGGCAT CAAcggcgtA GAATGGGACG CGGTCGAACT GCCCAGCCAG
1501  TTTATGGAAA ACTTCGTTTG GGAATACAAT GTATTGGCAC AAATGTCCGC
1551  CCACGAAGAA AccgGCGAGC CCCTGCCGAA AGAACTCTTC GACAAAATGC
1601  TcgcCGCCAA AAACTTCCAG CGCGGTATGT TCCTCGTCCG GCAAATGGAG
1651  TTCGCCCTCT TCGATATGAT GATTTACAGT GAAAGCGACG AATGCCGTCT
1701  GAAAAACTGG CAGCAGGTTT TAGACAGCGT GCGCAAAGAA GTcGCCGTCA
1751  TCCAACCGCC CGAATACAAC CGCTTCGCCA ACAGCTTCGG CCacatctTC
1801  GCcggcGGCT ATTCCGCAGG CTATTACAGC TACGCATGGG CCGAAGTCCt
1851  cAGCACCGAT GCCTACGCCG CCTTTGAAGA AAGcGACGac gtcGCCGCCA
1901  CAGGCAAACG CTTCTGGCAA GAAAtccttg ccgtcggcgg ctCCCGCAGC
1951  gcgGCGGAAT CCTTCAAAGC CTTCCGCGGA CGCGAACCGA GCATAGACGC
2001  ACTGCTGCGC CAaagcggtT TCGACAACGC gGCttgA
```

This corresponds to the amino acid sequence <SEQ ID 53; ORF 128.ng>:

```
g128.pep
      1  MIDNALLHLG EEPRFNQIQT EDIKPAVQTA IAEARGQIAA VKAQTHTGWA
     51  NTVERLTGIT ERVGRIWGVV SHLNSVVDTP ELRAVYNELM PEITVFFTEI
    101  GQDIELYNRF KTIKNSPEFA TLSPAQKTKL DHDLRDFVLS GAELPPERQA
    151  ELAKLQTEGA QLSAKFSQNV LDATDAFGIY FDDAAPLAGI PEDALAMFAA
    201  AAQSEGKTGY KIGLQIPHYL AVIQYAGNRE LREQIYRAYV TRASELSNDG

    251  KFDNTANIDR TLENALKTAK LLGFKNYAEL SLATKMADTP EQVLNFLHDL
    301  ARRAKPYAEK DLAEVKAFAR EHLGLADPQP WDLSYAGEKL REAKYAFSET
    351  EVKKYFPVGK VLAGLFAQIK KLYGIGFAEK TVPVWHKDVR YFELQQNGKT
    401  IGGVYMDLYA REGKRGGAWM NDYKGRRRFA DGTLQLPTAY LVCNFAPPVG
    451  GKEARLSHDE ILTLFHETGH GLHHLLTQVD ELGVSGINGV EWDAVELPSQ
    501  FMENFVWEYN VLAQMSAHEE TGEPLPKELF DKMLAAKNFQ RGMFLVRQME
    551  FALFDMMIYS ESDECRLKNW QQVLDSVRKE VAVIQPPEYN RFANSFGHIF
    601  AGGYSAGYYS YAWAEVLSTD AYAAFEESDD VAATGKRFWQ EILAVGGSRS
    651  AAESFKAFRG REPSIDALLR QSGFDNAA*
```

ORF 128 shows 91.7% identity over a 475 aa overlap with a predicted ORF (ORF 128.ng) from *N. gonorrhoeae:*

m128/g128

```
                   10        20        30        40        50        60
g128.pep   MIDNALLHLGEEPRFNQIQTEDIKPAVQTAIAEARGQIAAVKAQTHTGWANTVERLTGIT
           | |||||||||||||:||:||||||:||||||| ||||:|||||||||||| |||||
m128       MTDNALLHLGEEPRFDQIKTEDIKPALQTAIAEAREQIAAIKAQTHTGWANTVEPLTGIT
                   10        20        30        40        50        60

                   70        80        90       100       110       120
g128.pep   ERVGRIWGVVSHLNSVVDTPELRAVYNELMPEITVFFTEIGQDIELYNRFKTIKNSPEFA
           ||||||||||||| |:||||||||||||||||||||||||||||||||||||||||||
m128       ERVGRIWGVVSHLNCVADTPELRAVYNELMPEITVFFTEIGQDIELYNRFKTIKNSPEFD
                   70        80        90       100       110       120

                  130       140       150       160       170       180
g128.pep   TLSPAQKTKLDHDLRDFVLSGAELPPERQAELAKLQTEGAQLSAKFSQNVLDATDAFGIY
           ||||||||||:|
m128       TLSPAQKTKLNH
                  130
                  //
                                        340       350       360
g128.pep                               YAGEKLREAKYAFSETEVKKYFPVGKVLAG
                                       ||:||||||||||||| |||||||| || |
m128                                   YASEKLREAKYAFSETXVKKYFPVGXVLNG
                                               10        20        30

                  370       380       390       400       410       420
g128.pep   LFAQIKKLYGIGFAEKTVPVWHKDVRYFELQQNGKTIGGVYMDLYAREGKRGGAWMNDYK
           |||| |||||||:||||||||||||| |||||::||||||||||||||||||||||||
m128       LFAQXKKLYGIGFTEKTVPVWHKDVRYXELQQNGEXIGGVYMDLYAREGKRGGAWMNDYK
                   40        50        60        70        80        90

                  430       440       450       460       470       480
g128.pep   GRRRFADGTLQLPTAYLVCNFAPPVGGKEARLSHDEILTLFHETGHGLHHLLTQVDELGV
           ||||:|||||||||||||||||||||:|||||||||| ||||||||||||||||||||
m128       GRRRFSDGTLQLPTAYLVCNFAPPVGGREARLSHDEILILFHETGHGLHHLLTQVDELGV
                  100       110       120       130       140       150

                  490       500       510       520       530       540
g128.pep   SGINGVEWDAVELPSQFMENFVWEYNVLAQMSAHEETGEPLPKELFDKMLAAKNFQRGMF
           |||||| ||||||||||||||||||||| ||||||| |||||| || ||||||| |||
m128       SGINGVXWDAVELPSQFMENFVWEYNVLAQXSAHEETGVPLPKELXDKXLAAKNFQXGMF
                  160       170       180       190       200       210

                  550       560       570       580       590       600

g128.pep   LVRQMEFALFDMMIYSESDECRLKNWQQVLDSVRKEVAVIQPPEYNRFANSFGHIFAGGY
            ||| ||||||||||||:|| ||||||||||||||:||||||||||||| |||||||||
m128       XVRQXEFALFDMMIYSEDDEGRLKNWQQVLDSVRKKVAVIQPPEYNRFALSFGHIFAGGY
                  220       230       240       250       260       270

                  610       620       630       640       650       660
g128.pep   SAGYYSYAWAEVLSTDAYAAFEESDDVAATGKRFWQEILAVGGSRSAAESFKAFRGREPS
           ||: ||||||||||:||||||||||||||||||||||||||| |||:|||||||||||||
m128       SAAXYSYAWAEVLSADAYAAFEESDDVAATGKRFWQEILAVGXSRSGAESFKAFRGREPS
                  280       290       300       310       320       330

                  670      679
g128.pep   IDALLRQSGFDNAAX
           ||||||:||||||:
m128       IDALLRHSGFDNAVX
                  340
```

The following partial DNA sequence was identified in *N. meningitidis* <SEQ ID 54>:

```
a128.seq
   1  ATGACTGACA ACGCACTGCT CCATTTGGGC GAAGAACCCC GTTTTGATCA
  51  AATCAAAACC GAAGACATCA AACCCGCCCT GCAAACCGCC ATTGCCGAAG
 101  CGCGCGAACA AATCGCCGCC ATCAAAGCCC AAACGCACAC CGGCTGGGCA
 151  AACACTGTCG AACCCCTGAC CGGCATCACC GAACGCGTCG GCAGGATTTG
 201  GGGCGTGGTG TCGCACCTCA ACTCCGTCAC CGACACGCCC GAACTGCGCG
 251  CCGCCTACAA TGAATTAATG CCCGAAATTA CCGTCTTCTT CACCGAAATC
 301  GGACAAGACA TCGAGCTGTA CAACCGCTTC AAAACCATCA AAAACTCCCC
 351  CGAGTTCGAC ACCCTCTCCC ACGCGCAAAA AACCAAACTC AACCACGATC
 401  TGCGCGATTT CGTCCTCAGC GGCGCGGAAC TGCCGCCCGA ACAGCAGGCA
 451  GAATTGGCAA AACTGCAAAC CGAAGGCGCG CAACTTTCCG CCAAATTCTC
 501  CCAAAACGTC CTAGACGCGA CCGACGCGTT CGGCATTTAC TTTGACGATG
 551  CCGCACCGCT TGCCGGCATT CCCGAAGACG CGCTCGCCAT GTTTGCCGCT
 601  GCCGCGCAAA GCGAAGGCAA AACAGGCTAC AAAATCGGTT TGCAGATTCC
 651  GCACTACCTC GCCGTCATCC AATACGCCGA CAACCGCAAA CTGCGCGAAC
 701  AAATCTACCG CGCCTACGTT ACCCGCGCCA GCGAGCTTTC AGACGACGGC
 751  AAATTCGACA ACACCGCCAA CATCGACCGC ACGCTCGAAA ACGCCCTGCA
 801  AACCGCCAAA CTGCTCGGCT TCAAAAACTA CGCCGAATTG TCGCTGGCAA
 851  CCAAAATGGC GGACACCCCC GAACAAGTTT TAAACTTCCT GCACGACCTC
 901  GCCCGCCGCG CCAAACCCTA CGCCGAAAAA GACCTCGCCG AAGTCAAAGC
 951  CTTCGCCCGC GAAAGCCTCG GCCTCGCCGA TTTGCAACCG TGGGACTTGG
1001  GCTACGCCGG CGAAAAACTG CGCGAAGCCA AATACGCATT CAGCGAAACC
1051  GAAGTCAAAA AATACTTCCC CGTCGGCAAA GTATTAAACG GACTGTTCGC
1101  CCAAATCAAA AAACTCTACG GCATCGGATT TACCGAAAAA ACCGTCCCCG
1151  TCTGGCACAA AGACGTGCGC TATTTTGAAT TGCAACAAAA CGGCGAAACC
1201  ATAGGCGGCG TTTATATGGA TTTGTACGCA CGCGAAGGCA AACGCGGCGG
1251  CGCGTGGATG AACGACTACA AAGGCCGCCG CCGTTTTTCA GACGGCACGC
1301  TGCAACTGCC CACCGCCTAC CTCGTCTGCA ACTTCACCCC GCCCGTCGGC
1351  GGCAAAGAAG CCCGCTTGAG CCATGACGAA ATCCTCACCC TCTTCCACGA
1401  AACCGGACAC GGCCTGCACC ACCTGCTTAC CCAAGTCGAC GAACTGGGCG
1451  TATCCGGCAT CAACGGCGTA GAATGGGACG CAGTCGAACT GCCCAGTCAG
1501  TTTATGGAAA ATTTCGTTTG GGAATACAAT GTCTTGGCGC AAATGTCCGC
1551  CCACGAAGAA ACCGGCGTTC CCCTGCCGAA AGAACTCTTC GACAAAATGC
1601  TCGCCGCCAA AAACTTCCAA CGCGGAATGT TCCTCGTCCG CCAAATGGAG
1651  TTCGCCCTCT TTGATATGAT GATTTACAGC GAAGACGACG AAGGCCGTCT
1701  GAAAAACTGG CAACAGGTTT TAGACAGCGT GCGCAAAGAA GTCGCCGTCG
1751  TCCGACCGCC CGAATACAAC CGCTTCGCCA ACAGCTTCGG CCACATCTTC
1801  GCAGGCGGCT ATTCCGCAGG CTATTACAGC TACGCGTGGG CGGAAGTATT
1851  GAGCGCGGAC GCATACGCCG CCTTTGAAGA AAGCGACGAT GTCGCCCGCCA
1901  CAGGCAAACG CTTTTGGCAG GAAATCCTCG CCGTCGGCGG ATCGCGCAGC
1951  GCGGCAGAAT CCTTCAAAGC CTTCCGCGGA CGCGAACCGA GCATAGACGC
2001  ACTCTTGCGC CACAGCGGCT TCGACAACGC GGCTTGA
```

This corresponds to the amino acid sequence <SEQ ID 55; ORF 128.a>:

```
a128.pep
      1  MTDNALLHLG EEPRFDQIKT EDIKPALQTA IAEAREQIAA IKAQTHTGWA
     51  NTVEPLTGIT ERVGRIWGVV SHLNSVTDTP ELRAAYNELM PEITVFFTEI
    101  GQDIELYNRF KTIKNSPEFD TLSHAQKTKL NHDLRDFVLS GAELPPEQQA
    151  ELAKLQTEGA QLSAKFSQNV LDATDAFGIY FDDAAPLAGI PEDALAMFAA
    201  AAQSEGKTGY KIGLQIPHYL AVIQYADNRK LREQIYRAYV TRASELSDDG
    251  KFDNTANIDR TLENALQTAK LLGFKNYAEL SLATKMADTP EQVLNFLHDL
    301  ARRAKPYAEK DLAEVKAFAR ESLGLADLQP WDLGYAGEKL REAKYAFSET
    351  EVKKYFPVGK VLNGLFAQIK KLYGIGFTEK TVPVWHKDVR YFELQQNGET
    401  IGGVYMDLYA REGKRGGAWM NDYKGRRRFS DGTLQLPTAY LVCNFTPPVG
    451  GKEARLSHDE ILTLFHETGH GLHHLLTQVD ELGVSGINGV EWDAVELPSQ
    501  FMENFVWEYN VLAQMSAHEE TGVPLPKELF DKMLAAKNFQ RGMFLVRQME
    551  FALFDMMIYS EDDEGRLKNW QQVLDSVRKE VAVVRPPEYN RFANSFGHIF
    601  AGGYSAGYYS YAWAEVLSAD AYAAFEESDD VAATGKRFWQ EILAVGGSRS
    651  AESFKAFRG REPSIDALLR HSGFDNAA*
```

m128/a128 ORFs 128 and 128.a showed a 66.0% identity in 677 aa overlap

```
                    10        20        30        40        50        60
m128.pep    MTDNALLHLGEEPRFDQIKTEDIKPALQTAIAEAREQIAAIKAQTHTGWANTVEPLTGIT
            ||||||||||||||||||||||||||||||||||||||||||||||||||||||||||||
a128        MTDNALLHLGEEPRFDQIKTEDIKPALQTAIAEAREQIAAIKAQTHTGWANTVEPLTGIT
                    10        20        30        40        50        60

                    70        80        90       100       110       120
m128.pep    ERVGRIWGVVSHLNCVADTPELRAVYNELMPEITVFFTEIGQDIELYNRFKTIKNSPEFD
            ||||||||||||||  |:||||||:|||||||||||||||||||||||||||||||||||
a128        ERVGRIWGVVSHLNSVTDTPELRAAYNELMPEITVFFTEIGQDIELYNRFKTIKNSPEFD
                    70        80        90       100       110       120

                   130
m128.pep    TLSPAQKTKLNH------------------------------------------------
            ||| ||||||||
a128        TLSHAQKTKLNHDLRDFVLSGAELPPEQQAELAKLQTEGAQLSAKFSQNVLDATDAFGIY
                   130       140       150       160       170       180

m128.pep    ------------------------------------------------------------

a128        FDDAAPLAGIPEDALAMFAAAAQSEGKTGYKIGLQIPHYLAVIQYADNRKLREQIYRAYV
                   190       200       210       220       230       240

m128.pep    ------------------------------------------------------------

a128        TRASELSDDGKFDNTANIDRTLENALQTAKLLGFKNYAELSLATKMADTPEQVLNFLHDL
                   250       260       270       280       290       300

                                                   140       150
m128.pep    ---------------------------------YASEKLREAKYAFSETXVKKYFPVGX
                                             ||:||||||||||||| |||||||
a128        ARRAKPYAEKDLAEVKAFARESLGLADLQPWDLGYAGEKLREAKYAFSETEVKKYFPVGK
                   310       320       330       340       350       360

                  160       170       180       190       200       210
m128.pep    VLNGLFAQXKKLYGIGFTEKTVPVWHKDVRYXELQQNGEXIGGVYMDLYAREGKRGGAWM
            |||||||| |||||||||||||||||||||| ||||||:|||||||||||||||||||||
a128        VLNGLFAQIKKLYGIGFTEKTVPVWHKDVRYFELQQNGETIGGVYMDLYAREGKRGGAWM
                   370       380       390       400       410       420

                  220       230       240       250       260       270
m128.pep    NDYKGRRRFSDGTLQLPTAYLVCNFAPPVGGREARLSHDEILILFHETGHGLHHLLTQVD
```

```
             |||||||||||||||||||||||||:|||||:||||||||| ||||||||||||||||
a128         NDYKGRRRFSDGTLQLPTAYLVCNFTPPVGGKEARLSHDEILTLFHETGHGLHHLLTQVD
                    430       440       450       460       470       480

            280       290       300       310       320       330
m128.pep     ELGVSGINGVXWDAVELPSQFMENFVWEYNVLAQXSAHEETGVPLPKELXDKXLAAKNFQ
             |||||||||| |||||||||||||||||||||||| ||||||||||||| || |||||||
a128         ELGVSGINGVEWDAVELPSQFMENFVWEYNVLAQMSAHEETGVPLPKELFDKMLAAKNFQ
                    490       500       510       520       530       540

            340       350       360       370       380       390
m128.pep     XGMFXVRQXEFALFDMMIYSEDDEGRLKNWQQVLDSVRKKVAVIQPPEYNRFALSFGHIF
              ||| ||| ||||||||||||||||||||||||||||||:|||::|||||||| ||||||
a128         RGMFLVRQMEFALFDMMIYSEDDEGRLKNWQQVLDSVRKEVAVVRPPEYNRFANSFGHIF
                    550       560       570       580       590       600

            400       410       420       430       440       450
m128.pep     AGGYSAAXYSYAWAEVLSADAYAAFEESDDVAATGKRFWQEILAVGXSRSGAESFKAFRG
             ||||||: |||||||||||||||||||||||||||||||||||||| |||:|||||||||
a128         AGGYSAGYYSYAWAEVLSADAYAAFEESDDVAATGKRFWQEILAVGGSRSAAESFKAFRG
                    610       620       630       640       650       660

            460       470
m128.pep     REPSIDALLRHSGFDNAVX
             ||||||||||||||||:
a128         REPSIDALLRHSGFDNAAX
                    670
```

Further work revealed the DNA sequence identified in *N. meningitidis* <SEQ ID 56>:

```
m128-1.seq
       1  ATGACTGACA ACGCACTGCT CCATTTGGGC GAAGAACCCC GTTTTGATCA
      51  AATCAAAACC GAAGACATCA AACCCGCCCT GCAAACCGCC ATCGCCGAAG
     101  CGCGCGAACA AATCGCCGCC ATCAAAGCCC AAACGCACAC CGGCTGGGCA
     151  AACACTGTCG AACCCCTGAC CGGCATCACC GAACGCGTCG GCAGGATTTG
     201  GGGCGTGGTG TCGCACCTCA ACTCCGTCGC CGACACGCCC GAACTGCGCG
     251  CCGTCTATAA CGAACTGATG CCCGAAATCA CCGTCTTCTT CACCGAAATC
     301  GGACAAGACA TCGAGCTGTA CAACCGCTTC AAAACCATCA AAAATTCCCC
     351  CGAATTCGAC ACCCTCTCCC CCGCACAAAA AACCAAACTC AACCACGATC
     401  TGCGCGATTT CGTCCTCAGC GGCGCGGAAC TGCCGCCCGA ACAGCAGGCA
     451  GAACTGGCAA AACTGCAAAC CGAAGGCGCG CAACTTTCCG CCAAATTCTC
     501  CCAAAACGTC CTAGACGCGA CCGACGCGTT CGGCATTTAC TTTGACGATG
     551  CCGCACCGCT TGCCGGCATT CCCGAAGACG CGCTCGCCAT GTTTGCCGCC
     601  GCCGCGCAAA GCGAAAGCAA AACAGGCTAC AAAATCGGCT TGCAGATTCC
     651  ACACTACCTC GCCGTCATCC AATACGCCGA CAACCGCGAA CTGCGCGAAC
     701  AAATCTACCG CGCCTACGTT ACCCGCGCCA GCGAACTTTC AGACGACGGC
     751  AAATTCGACA ACACCGCCAA CATCGACCGC ACGCTCGCAA ACGCCCTGCA
     801  AACCGCCAAA CTGCTCGGCT TCAAAAACTA CGCCGAATTG TCGCTGGCAA
     851  CCAAAATGGC GGACACGCCC GAACAAGTTT TAAACTTCCT GCACGACCTC
     901  GCCCGCCGCG CCAAACCCTA CGCCGAAAAA GACCTCGCCG AAGTCAAAGC
     951  CTTCGCCCGC GAAAGCCTGA ACCTCGCCGA TTTGCAACCG TGGGACTTGG
    1001  GCTACGCCAG CGAAAAACTG CGCGAAGCCA AATACGCGTT CAGCGAAACC
    1051  GAAGTCAAAA AATACTTCCC CGTCGGCAAA GTATTAAACG GACTGTTCGC
    1101  CCAAATCAAA AAACTCTACG GCATCGGATT TACCGAAAAA ACCGTCCCCG
    1151  TCTGGCACAA AGACGTGCGC TATTTTGAAT TGCAACAAAA CGGCGAAACC
    1201  ATAGGCGGCG TTTATATGGA TTTGTACGCA CGCGAAGGCA AACGCGGCGG
    1251  CGCGTGGATG AACGACTACA AAGGCCGCCG CCGTTTTTCA GACGGCACGC
    1301  TGCAACTGCC CACCGCCTAC CTCGTCTGCA ACTTCGCCCC ACCCGTCGGC
    1351  GGCAGGGAAG CCCGCCTGAG CCACGACGAA ATCCTCATCC TCTTCCACGA
    1401  AACCGGACAC GGGCTGCACC ACCTGCTTAC CCAAGTGGAC GAACTGGGCG
```

```
1451  TATCCGGCAT CAACGGCGTA GAATGGGACG CGGTCGAACT GCCCAGCCAG
1501  TTTATGGAAA ATTTCGTTTG GGAATACAAT GTCTTGGCAC AAATGTCAGC
1551  CCACGAAGAA ACCGGCGTTC CCCTGCCGAA AGAACTCTTC GACAAAATGC
1601  TCGCCGCCAA AAACTTCCAA CGCGGCATGT TCCTCGTCCG GCAAATGGAG
1651  TTCGCCCTCT TTGATATGAT GATTTACAGC GAAGACGACG AAGGCCGTCT
1701  GAAAAACTGG CAACAGGTTT TAGACAGCGT GCGCAAAAAA GTCGCCGTCA
1751  TCCAGCCGCC CGAATACAAC CGCTTCGCCT TGAGCTTCGG CCACATCTTC
1801  GCAGGCGGCT ATTCCGCAGG CTATTACAGC TACGCGTGGG CGGAAGTATT
1851  GAGCGCGGAC GCATACGCCG CCTTTGAAGA AAGCGACGAT GTCGCCGCCA
1901  CAGGCAAACG CTTTTGGCAG GAAATCCTCG CCGTCGGCGG ATCGCGCAGC
1951  GCGGCAGAAT CCTTCAAAGC CTTCCGCGGC CGCGAACCGA GCATAGACGC
2001  ACTCTTGCGC CACAGCGGTT TCGACAACGC GGTCTGA
```

This corresponds to the amino acid sequence <SEQ ID 57; ORF 128-1>:

```
m128-1.pep.
  1  MTDNALLHLG EEPRFDQIKT EDIKPALQTA IAEAREQIAA IKAQTHTGWA
 51  NTVEPLTGIT ERVGRIWGVV SHLNSVADTP ELRAVYNELM PEITVFFTEI
101  GQDIELYNRF KTIKNSPEFD TLSPAQKTKL NHDLRDFVLS GAELPPEQQA
151  ELAKLQTEGA QLSAKFSQNV LDATDAFGIY FDDAAPLAGI PEDALAMFAA
201  AAQSESKTGY KIGLQIPHYL AVIQYADNRE LREQIYRAYV TRASELSDDG
251  KFDNTANIDR TLANALQTAK LLGFKNYAEL SLATKMADTP EQVLNFLHDL
301  ARRAKPYAEK DLAEVKAFAR ESLNLADLQP WDLGYASEKL REAKYAFSET
351  EVKKYFPVGK VLNGLFAQIK KLYGIGFTEK TVPVWHKDVR YFELQQNGET
401  IGGVYMDLYA REGKRGGAWM NDYKGRRRFS DGTLQLPTAY LVCNFAPPVG
451  GREARLSHDE ILILFHETGH GLHHLLTQVD ELGVSGINGV EWDAVELPSQ
501  FMENFVWEYN VLAQMSAHEE TGVPLPKELF DKMLAAKNFQ RGMFLVRQME
551  FALFDMMIYS EDDEGRLKNW QQVLDSVRKK VAVIQPPEYN RFALSFGHIF
601  AGGYSAGYYS YAWAEVLSAD AYAAFEESDD VAATGKRFWQ EILAVGGSRS
651  AAESFKAFRG REPSIDALLR HSGFDNAV*
```

The following partial DNA sequence was identified in *N. gonorrhoeae* <SEQ ID 58>:

```
g128-1.seq  (partial)
   1  ATGATTGACA ACGCACTGCT CCACTTGGGC GAAGAACCCC GTTTTAATCA
  51  AATCAAAACC GAAGACATCA AACCCGCCGT CCAAACCGCC ATCGCCGAAG
 101  CGCGCGGACA AATCGCCGCC GTCAAAGCGC AAACGCACAC CGGCTGGGCG
 151  AACACCGTCG AGCGTCTGAC CGGCATCACC GAACGCGTCG GCAGGATTTG
 201  GGGCGTCGTG TCCCATCTCA ACTCCGTCGT CGACACGCCC GAACTGCGCG
 251  CCGTCTATAA CGAACTGATG CCTGAAATCA CCGTCTTCTT CACCGAAATC
 301  GGACAAGACA TCGAACTGTA CAACCGCTTC AAAACCATCA AAAATTCCCC
 351  CGAATTTGCA ACGCTTTCCC CCGCACAAAA AACCAAGCTC GATCACGACC
 401  TGCGCGATTT CGTATTGAGC GGCGCGGAAC TGCCGCCCGA ACGGCAGGCA
 451  GAACTGGCAA AACTGCAAAC CGAAGGCGCG CAACTTTCCG CCAAATTCTC
 501  CCAAAACGTC CTAGACGCGA CCGACGCGTT CGGCATTTAC TTTGACGATG
 551  CCGCACCGCT TGCCGGCATT CCCGAAGACG CGCTCGCCAT GTTTGCCGCC
 601  GCCGCGCAAA GCGAAGGCAA AACAGGTTAC AAAATCGGCT TGCAGATTCC
 651  GCACTACCTT GCCGTTATCC AATACGCCGG CAACCGCGAA CTGCGCGAAC
 701  AAATCTACCG CGCCTACGTT ACCCGTGCCA GCGAACTTTC AAACGACGGC
 751  AAATTCGACA ACACCGCCAA CATCGACCGC ACGCTCGAAA ACGCATTGAA
 801  AACCGCCAAA CTGCTCGGCT TTAAAAATTA CGCCGAATTG TCGCTGGCAA
 851  CCAAAATGGC GGACACGCCC GAACAGGTTT TAAACTTCCT GCACGACCTC
 901  GCCCGCCGCG CCAAACCCTA CGCCGAAAAA GACCTCGCCG AAGTCAAAGC
 951  CTTCGCCCGC GAACACCTCG GTCTCGCCGA CCCGCAGCCG TGGGACTTGA
1001  GCTACGCCGG CGAAAAACTG CGCGAAGCCA AATACGCATT CAGCGAAACC
1051  GAAGTCAAAA AATACTTCCC CGTCGGCAAA GTTCTGGCAG GCCTGTTCGC
1101  CCAAATCAAA AAACTCTACG GCATCGGATT CGCCGAAAAA ACCGTTCCCG
1151  TCTGGCACAA AGACGTGCGC TATTTTGAAT TGCAACAAAA CGGCAAAACC
1201  ATCGGCGGCG TTTATATGGA TTTGTACGCA CGCGAAGGCA AACGCGGCGG
1251  CGCGTGGATG AACGACTACA AAGGCCGCCG CCGCTTTGCC GACGGCACGC
1301  TGCAACTGCC CACCGCCTAC CTCGTCTGCA ACTTCGCCCC GCCCGTCGGC
1351  GGCAAAGAAG CGCGTTTAAG CCACGACGAA ATCCTCACCC TCTTCCACGA
1401  AACCGGCCAC GGACTGCACC ACCTGCTTAC CCAAGTGGAC GAACTGGGCG
1451  TGTCCGGCAT CAACGGCGTA AAA
```

This corresponds to the amino acid sequence <SEO ID 59; ORF 128-1.ng>:

```
g128-1.pep  (partial)
      1  MIDNALLHLG EEPRFNQIKT EDIKPAVQTA IAEARGQIAA VKAQTHTGWA
     51  NTVERLTGIT ERVGRIWGVV SHLNSVVDTP ELRAVYNELM PEITVFFTEI
    101  GQDIELYNRF KTIKNSPEFA TLSPAQKTKL DHDLRDFVLS GAELPPERQA
    151  ELAKLQTEGA QLSAKFSQNV LDATDAFGIY FDDAAPLAGI PEDALAMFAA
    201  AAQSEGKTGY KIGLQIPHYL AVIQYAGNRE LREQIYRAYV TRASELSNDG
    251  KFDNTANIDR TLENALKTAK LLGFKNYAEL SLATKMADTP EQVLNFLHDL
    301  ARRAKPYAEK DLAEVKAFAR EHLGLADPQP WDLSYAGEKL REAKYAFSET
    351  EVKKYFPVGK VLAGLFAQIK KLYGIGFAEK TVPVWHKDVR YFELQQNGKT
    401  IGGVYMDLYA REGKRGGAWM NDYKGRRRFA DGTLQLPTAY LVCNFAPPVG
    451  GKEARLSHDE ILTLFHETGH GLHHLLTQVD ELGVSGINGV K
```

**m128-1/g128-1** ORFs 128-1 and 128-1.ng showed a 94.5% identity in 491 aa overlap

```
                    10        20        30        40        50        60
g128-1.pep   MIDNALLHLGEEPRFNQIKTEDIKPAVQTAIAEARGQIAAVKAQTHTGWANTVERLTGIT
             | ||||||||||||:|||||||||:|||||||| ||||:||||||||||||| |||||
m128-1       MTDNALLHLGEEPRFDQIKTEDIKPALQTAIAEAREQIAAIKAQTHTGWANTVEPLTGIT
                    10        20        30        40        50        60

                    70        80        90       100       110       120
g128-1.pep   ERVGRIWGVVSHLNSVVDTPELRAVYNELMPEITVFFTEIGQDIELYNRFKTIKNSPEFA
             |||||||||||||||:|||||||||||||||||||||||||||||||||||||||||||
m128-1       ERVGRIWGVVSHLNSVADTPELRAVYNELMPEITVFFTEIGQDIELYNRFKTIKNSPEFD
                    70        80        90       100       110       120

                   130       140       150       160       170       180
g128-1.pep   TLSPAQKTKLDHDLRDFVLSGAELPPERQAELAKLQTEGAQLSAKFSQNVLDATDAFGIY
             ||||||||||:||||||||||||||||:||||||||||||||||||||||||||||||||
m128-1       TLSPAQKTKLNHDLRDFVLSGAELPPEQQAELAKLQTEGAQLSAKFSQNVLDATDAFGIY
                   130       140       150       160       170       180

                   190       200       210       220       230       240
g128-1.pep   FDDAAPLAGIPEDALAMFAAAAQSEGKTGYKIGLQIPHYLAVIQYAGNRELREQIYRAYV
             ||||||||||||||||||||||||:|||||||||||||||||||| ||||||||||||||
m128-1       FDDAAPLAGIPEDALAMFAAAAQSESKTGYKIGLQIPHYLAVIQYADNRELREQIYRAYV
                   190       200       210       220       230       240

                   250       260       270       280       290       300
g128-1.pep   TRASELSNDGKFDNTANIDRTLENALKTAKLLGFKNYAELSLATKMADTPEQVLNFLHDL
             |||||||:||||||||||||||| |||:||||||||||||||||||||||||||||||||
m128-1       TRASELSDDGKFDNTANIDRTLANALQTAKLLGFKNYAELSLATKMADTPEQVLNFLHDL
                   250       260       270       280       290       300

                   310       320       330       340       350       360
g128-1.pep   ARRAKPYAEKDLAEVKAFAREHLGLADPQPWDLSYAGEKLREAKYAFSETEVKKYFPVGK
             |||||||||||||||||||| |:||| |||||:||:||||||||||||||||||||||||
m128-1       ARRAKPYAEKDLAEVKAFARESLNLADLQPWDLGYASEKLREAKYAFSETEVKKYFPVGK
                   310       320       330       340       350       360

                   370       380       390       400       410       420
g128-1.pep   VLAGLFAQIKKLYGIGFAEKTVPVWHKDVRYFELQQNGKTIGGVYMDLYAREGKRGGAWM
             || |||||||||||||:|||||||||||||||||||||:|||||||||||||||||||||
m128-1       VLNGLFAQIKKLYGIGFTEKTVPVWHKDVRYFELQQNGETIGGVYMDLYAREGKRGGAWM
                   370       380       390       400       410       420

                   430       440       450       460       470       480
g128-1.pep   NDYKGRRRFADGTLQLPTAYLVCNFAPPVGGKEARLSHDEILTLFHETGHGLHHLLTQVD
```

```
                ||||||||:||||||||||||||||||||:|||||||||| |||||||||||||||
m128-1          NDYKGRRRFSDGTLQLPTAYLVCNFAPPVGGREARLSHDEILILFHETGHGLHHLLTQVD
                        430       440       450       460       470       480

                        490
g128-1.pep      ELGVSGINGVK
                ||||||||||:
m128-1          ELGVSGINGVEWDAVELPSQFMENFVWEYNVLAQMSAHEETGVPLPKELFDKMLAAKNFQ
                        490       500       510       520       530       540
```

The following DNA sequence was identified in *N. meningitidis* <SEQ ID 60>:

```
a128-1.seq
   1  ATGACTGACA ACGCACTGCT CCATTTGGGC GAAGAACCCC GTTTTGATCA
  51  AATCAAAACC GAAGACATCA AACCCGCCCT GCAAACCGCC ATTGCCGAAG
 101  CGCGCGAACA AATCGCCGCC ATCAAAGCCC AAACGCACAC CGGCTGGGCA
 151  AACACTGTCG AACCCCTGAC CGGCATCACC GAACGCGTCG GCAGGATTTG
 201  GGGCGTGGTG TCGCACCTCA ACTCCGTCAC CGACACGCCC GAACTGCGCG
 251  CCGCCTACAA TGAATTAATG CCCGAAATTA CCGTCTTCTT CACCGAAATC
 301  GGACAAGACA TCGAGCTGTA CAACCGCTTC AAAACCATCA AAAACTCCCC
 351  CGAGTTCGAC ACCCTCTCCC ACGCGCAAAA AACCAAACTC AACCACGATC
 401  TGCGCGATTT CGTCCTCAGC GGCGCGGAAC TGCCGCCCGA ACAGCAGGCA
 451  GAATTGGCAA AACTGCAAAC CGAAGGCGCG CAACTTTCCG CCAAATTCTC
 501  CCAAAACGTC CTAGACGCGA CCGACGCGTT CGGCATTTAC TTTGACGATG
 551  CCGCACCGCT TGCCGGCATT CCCGAAGACG CGCTCGCCAT GTTTGCCGCT
 601  GCCGCGCAAA GCGAAGGCAA AACAGGCTAC AAAATCGGTT TGCAGATTCC
 651  GCACTACCTC GCCGTCATCC AATACGCCGA CAACCGCAAA CTGCGCGAAC
 701  AAATCTACCG CGCCTACGTT ACCCGCGCCA GCGAGCTTTC AGACGACGGC
 751  AAATTCGACA ACACCGCCAA CATCGACCGC ACGCTCGAAA ACGCCCTGCA
 801  AACCGCCAAA CTGCTCGGCT TCAAAAACTA CGCCGAATTG TCGCTGGCAA
 851  CCAAAATGGC GGACACCCCC GAACAAGTTT TAAACTTCCT GCACGACCTC
 901  GCCCGCCGCG CCAAACCCTA CGCCGAAAAA GACCTCGCCG AAGTCAAAGC
 951  CTTCGCCCGC GAAAGCCTCG GCCTCGCCGA TTTGCAACCG TGGGACTTGG
1001  GCTACGCCGG CGAAAAACTG CGCGAAGCCA AATACGCATT CAGCGAAACC
1051  GAAGTCAAAA AATACTTCCC CGTCGGCAAA GTATTAAACG GACTGTTCGC
1101  CCAAATCAAA AAACTCTACG GCATCGGATT TACCGAAAAA ACCGTCCCCG
1151  TCTGGCACAA AGACGTGCGC TATTTTGAAT TGCAACAAAA CGGCGAAACC
1201  ATAGGCGGCG TTTATATGGA TTTGTACGCA CGCGAAGGCA AACGCGGCGG
1251  CGCGTGGATG AACGACTACA AAGGCCGCCG CCGTTTTTCA GACGGCACGC
1301  TGCAACTGCC CACCGCCTAC CTCGTCTGCA ACTTCACCCC GCCCGTCGGC
1351  GGCAAAGAAG CCCGCTTGAG CCATGACGAA ATCCTCACCC TCTTCCACGA
1401  AACCGGACAC GGCCTGCACC ACCTGCTTAC CCAAGTCGAC GAACTGGGCG
1451  TATCCGGCAT CAACGGCGTA GAATGGGACG CAGTCGAACT GCCCAGTCAG
1501  TTTATGGAAA ATTTCGTTTG GGAATACAAT GTCTTGGCGC AAATGTCCGC
1551  CCACGAAGAA ACCGGCGTTC CCCTGCCGAA AGAACTCTTC GACAAAATGC
1601  TCGCCGCCAA AAACTTCCAA CGCGGAATGT TCCTCGTCCG CCAAATGGAG
1651  TTCGCCCTCT TTGATATGAT GATTTACAGC GAAGACGACG AAGGCCGTCT
1701  GAAAAACTGG CAACAGGTTT TAGACAGCGT GCGCAAAGAA GTCGCCGTCG
1751  TCCGACCGCC CGAATACAAC CGCTTCGCCA ACAGCTTCGG CCACATCTTC
1801  GCAGGCGGCT ATTCCGCAGG CTATTACAGC TACGCGTGGG CGGAAGTATT
1851  GAGCGCGGAC GCATACGCCG CCTTTGAAGA AAGCGACGAT GTCGCCGCCA
1901  CAGGCAAACG CTTTTGGCAG GAAATCCTCG CCGTCGGCGG ATCGCGCAGC
1951  GCGGCAGAAT CCTTCAAAGC CTTCCGCGGA CGCGAACCGA GCATAGACGC
2001  ACTCTTGCGC CACAGCGGCT TCGACAACGC GGCTTGA
```

This corresponds to the amino acid sequence <SEQ ID 61; ORF 128-1.a>:

```
a128-1.pep
   1  MTDNALLHLG EEPRFDQIKT EDIKPALQTA IAEAREQIAA IKAQTHTGWA
  51  NTVEPLTGIT ERVGRIWGVV SHLNSVTDTP ELRAAYNELM PEITVFFTEI
 101  GQDIELYNRF KTIKNSPEFD TLSHAQKTKL NHDLRDFVLS GAELPPEQQA
 151  ELAKLQTEGA QLSAKFSQNV LDATDAFGIY FDDAAPLAGI PEDALAMFAA
 201  AAQSEGKTGY KIGLQIPHYL AVIQYADNRK LREQIYRAYV TRASELSDDG
 251  KFDNTANIDR TLENALQTAK LLGFKNYAEL SLATKMADTP EQVLNFLHDL
```

```
301  ARRAKPYAEK DLAEVKAFAR ESLGLADLQP WDLGYAGEKL REAKYAFSET
351  EVKKYFPVGK VLNGLFAQIK KLYGIGFTEK TVPVWHKDVR YFELQQNGET
401  IGGVYMDLYA REGKRGGAWM NDYKGRRRFS DGTLQLPTAY LVCNFTPPVG
451  GKEARLSHDE ILTLFHETGH GLHHLLTQVD ELGVSGINGV EWDAVELPSQ
501  FMENFVWEYN VLAQMSAHEE TGVPLPKELF DKMLAAKNFQ RGMFLVRQME
551  FALFDMMIYS EDDEGRLKNW QQVLDSVRKE VAVVRPPEYN RFANSFGHIF
601  AGGYSAGYYS YAWAEVLSAD AYAAFEESDD VAATGKRFWQ EILAVGGSRS
651  AAESFKAFRG REPSIDALLR HSGFDNAA*
```

**m128-1/a128-1** ORFs 128-1 and 128-1.a showed a 97.8% identity in 677 aa overlap

```
                       10        20        30        40        50        60
a128-1.pep      MTDNALLHLGEEPRFDQIKTEDIKPALQTAIAEAREQIAAIKAQTHTGWANTVEPLTGIT
                ||||||||||||||||||||||||||||||||||||||||||||||||||||||||||||
m128-1          MTDNALLHLGEEPRFDQIKTEDIKPALQTAIAEAREQIAAIKAQTHTGWANTVEPLTGIT
                       10        20        30        40        50        60

                       70        80        90       100       110       120
a128-1.pep      ERVGRIWGVVSHLNSVTDTPELRAAYNELMPEITVFFTEIGQDIELYNRFKTIKNSPEFD
                ||||||||||||||||:||||||:||||||||||||||||||||||||||||||||||||
m128-1          ERVGRIWGVVSHLNSVADTPELRAVYNELMPEITVFFTEIGQDIELYNRFKTIKNSPEFD
                       70        80        90       100       110       120

                      130       140       150       160       170       180
a128-1.pep      TLSHAQKTKLNHDLRDFVLSGAELPPEQQAELAKLQTEGAQLSAKFSQNVLDATDAFGIY
                ||| ||||||||||||||||||||||||||||||||||||||||||||||||||||||||
m128-1          TLSPAQKTKLNHDLRDFVLSGAELPPEQQAELAKLQTEGAQLSAKFSQNVLDATDAFGIY
                      130       140       150       160       170       180

                      190       200       210       220       230       240
a128-1.pep      FDDAAPLAGIPEDALAMFAAAAQSEGKTGYKIGLQIPHYLAVIQYADNRKLREQIYRAYV
                ||||||||||||||||||||||||:|||||||||||||||||||||||:|||||||||||
m128-1          FDDAAPLAGIPEDALAMFAAAAQSESKTGYKIGLQIPHYLAVIQYADNRELREQIYRAYV
                      190       200       210       220       230       240

                      250       260       270       280       290       300
a128-1.pep      TRASELSDDGKFDNTANIDRTLENALQTAKLLGFKNYAELSLATKMADTPEQVLNFLHDL
                ||||||||||||||||||||| ||||||||||||||||||||||||||||||||||||||
m128-1          TRASELSDDGKFDNTANIDRTLANALQTAKLLGFKNYAELSLATKMADTPEQVLNFLHDL
                      250       260       270       280       290       300

                      310       320       330       340       350       360
a128-1.pep      ARRAKPYAEKDLAEVKAFARESLGLADLQPWDLGYAGEKLREAKYAFSETEVKKYFPVGK
                ||||||||||||||||||||||:||||||||||||:||||||||||||||||||||||||
m128-1          ARRAKPYAEKDLAEVKAFARESLNLADLQPWDLGYASEKLREAKYAFSETEVKKYFPVGK
                      310       320       330       340       350       360

                      370       380       390       400       410       420
a128-1.pep      VLNGLFAQIKKLYGIGFTEKTVPVWHKDVRYFELQQNGETIGGVYMDLYAREGKRGGAWM
                ||||||||||||||||||||||||||||||||||||||||||||||||||||||||||||
m128-1          VLNGLFAQIKKLYGIGFTEKTVPVWHKDVRYFELQQNGETIGGVYMDLYAREGKRGGAWM
                      370       380       390       400       410       420

                      430       440       450       460       470       480
a128-1.pep      NDYKGRRRFSDGTLQLPTAYLVCNFTPPVGGKEARLSHDEILTLFHETGHGLHHLLTQVD
                ||||||||||||||||||||||||:|||||:|||||||||| ||||||||||||||||||
m128-1          NDYKGRRRFSDGTLQLPTAYLVCNFAPPVGGREARLSHDEILILFHETGHGLHHLLTQVD
                      430       440       450       460       470       480

                      490       500       510       520       530       540
a128-1.pep      ELGVSGINGVEWDAVELPSQFMENFVWEYNVLAQMSAHEETGVPLPKELFDKMLAAKNFQ
                ||||||||||||||||||||||||||||||||||||||||||||||||||||||||||||
```

```
m128-1      ELGVSGINGVEWDAVELPSQFMENFVWEYNVLAQMSAHEETGVPLPKELFDKMLAAKNFQ
                    490       500       510       520       530       540

                    550       560       570       580       590       600
a128-1.pep  RGMFLVRQMEFALFDMMIYSEDDEGRLKNWQQVLDSVRKEVAVVRPPEYNRFANSFGHIF
            |||||||||||||||||||||||||||||||||||||:|||::|||||||| ||||||
m128-1      RGMFLVRQMEFALFDMMIYSEDDEGRLKNWQQVLDSVRKKVAVIQPPEYNRFALSFGHIF
                    550       560       570       580       590       600

                    610       620       630       640       650       660
a128-1.pep  AGGYSAGYYSYAWAEVLSADAYAAFEESDDVAATGKRFWQEILAVGGSRSAAESFKAFRG
            ||||||||||||||||||||||||||||||||||||||||||||||||||||||||||||
m128-1      AGGYSAGYYSYAWAEVLSADAYAAFEESDDVAATGKRFWQEILAVGGSRSAAESFKAFRG
                    610       620       630       640       650       660

                    670      679
a128-1.pep  REPSIDALLRHSGFDNAAX
            ||||||||||||||||:
m128-1      REPSIDALLRHSGFDNAVX
                    670
```

## 206

The following partial DNA sequence was identified in *N. meningitidis* <SEQ ID 62>:

```
m206.seq
  1   ATGTTTCCCC CCGACAAAAC CCTTTTCCTC TGTCTCAGCG CACTGCTCCT
 51   CGCCTCATGC GGCACGACCT CCGGCAAACA CCGCCAACCG AAACCCAAAC
101   AGACAGTCCG GCAAATCCAA GCCGTCCGCA TCAGCCACAT CGACCGCACA
151   CAAGGCTCGC AGGAACTCAT GCTCCACAGC CTCGGACTCA TCGGCACGCC
201   CTACAAATGG GGCGGCAGCA GCACCGCAAC CGGCTTCGAT TGCAGCGGCA
251   TGATTCAATT CGTTTACAAr AACGCCCTCA ACGTCAAGCT GCCGCGCACC
301   GCCCGCGACA TGGCGGCGGC AAGCCGsAAA ATCCCCGAcA GCCGCyTCAA
351   GGCCGGCGAC CTCGTATTCT TCAACACCGG CGGCGCACAC CGCTACTCAC
401   ACGTCGGACT CTACATCGGC AACGGCGAAT TCATCCATGC CCCCAGCAGC
451   GGCAAAACCA TCAAAACCGA AAAACTCTCC ACACCGTTTT ACGCCAAAAA
501   CTACCTCGGC GCACATACTT TTTTTACAGA ATGA
```

This corresponds to the amino acid sequence <SEQ ID 63; ORF 206>:

```
m206.pep..
  1   MFPPDKTLFL CLSALLLASC GTTSGKHRQP KPKQTVRQIQ AVRISHIDRT
 51   QGSQELMLHS LGLIGTPYKW GGSSTATGFD CSGMIQFVYK NALNVKLPRT
101   ARDMAAASRK IPDSRXKAGD LVFFNTGGAH RYSHVGLYIG NGEFIHAPSS
151   GKTIKTEKLS TPFYAKNYLG AHTFFTE*
```

The following partial DNA sequence was identified in *N. gonorrhoeae* <SEQ ID 64>:

```
g206.seq
      1  atgttttccc ccgacaaaac ccttttcctc tgtctcggcg cactgctcct
     51  cgcctcatgc ggcacgacct ccggcaaaca ccgccaaccg aaacccaaac
    101  agacagtccg gcaaatccaa gccgtccgca tcagccacat cggccgcaca
    151  caaggctcgc aggaactcat gctccacagc ctcggactca tcggcacgcc
    201  ctacaaatgg ggcggcagca gcaccgcaac cggcttcgac tgcagcggca
    251  tgattcaatt ggtttacaaa aacgccctca acgtcaagct gccgcgcacc
    301  gcccgcgaca tggcggcggc aagccgcaaa atccccgaca gccgcctcaa
    351  ggccggcgac atcgtattct tcaacaccgg cggcgcacac cgctactcac
    401  acgtcggact ctacatcggc aacggcgaat tcatccatgc cccggcagc
    451  ggcaaaacca tcaaaaccga aaaactctcc acaccgtttt acgccaaaaa
    501  ctaccttgga gcgcatacgt tttttacaga atga
```

This corresponds to the amino acid sequence <SEQ ID 65; ORF 206.ng>:

```
g206.pep
      1  MFSPDKTLFL CLGALLLASC GTTSGKHRQP KPKQTVRQIQ AVRISHIGRT
     51  QGSQELMLHS LGLIGTPYKW GGSSTATGFD CSGMIQLVYK NALNVKLPRT
    101  ARDMAAASRK IPDSRLKAGD IVFFNTGGAH RYSHVGLYIG NGEFIHAPGS
    151  GKTIKTEKLS TPFYAKNYLG AHTFFTE*
```

ORF 206 shows 96.0% identity over a 177 aa overlap with a predicted ORF (ORF 206.ng) from *N. gonorrhoeae:*

```
m206/g206

                      10        20        30        40        50        60
m206.pep     MFPPDKTLFLCLSALLLASCGTTSGKHRQPKPKQTVRQIQAVRISHIDRTQGSQELMLHS
             || |||||||||:||||||||||||||||||||||||||||||||| |||||||||||||
g206         MFSPDKTLFLCLGALLLASCGTTSGKHRQPKPKQTVRQIQAVRISHIGRTQGSQELMLHS
                      10        20        30        40        50        60

                      70        80        90       100       110       120
m206.pep     LGLIGTPYKWGGSSTATGFDCSGMIQFVYKNALNVKLPRTARDMAAASRKIPDSRXKAGD
             ||||||||||||||||||||||||||:|||||||||||||||||||||||||||| ||||
g206         LGLIGTPYKWGGSSTATGFDCSGMIQLVYKNALNVKLPRTARDMAAASRKIPDSRLKAGD
                      70        80        90       100       110       120

                     130       140       150       160       170
m206.pep     LVFFNTGGAHRYSHVGLYIGNGEFIHAPSSGKTIKTEKLSTPFYAKNYLGAHTFFTEX
             :||||||||||||||||||||||||||||:|||||||||||||||||||||||||||
g206         IVFFNTGGAHRYSHVGLYIGNGEFIHAPGSGKTIKTEKLSTPFYAKNYLGAHTFFTE
                     130       140       150       160       170
```

The following partial DNA sequence was identified in *N. meningitidis* <SEQ ID 66>:

```
a206.seq
      1  ATGTTTCCCC CCGACAAAAC CCTTTTCCTC TGTCTCAGCG CACTGCTCCT
     51  CGCCTCATGC GGCACGACCT CCGGCAAACA CCGCCAACCG AAACCCAAAC
    101  AGACAGTCCG GCAAATCCAA GCCGTCCGCA TCAGCCACAT CGACCGCACA
    151  CAAGGCTCGC AGGAACTCAT GCTCCACAGC CTCGGACTCA TCGGCACGCC
    201  CTACAAATGG GGCGGCAGCA GCACCGCAAC CGGCTTCGAT TGCAGCGGCA
    251  TGATTCAATT CGTTTACAAA AACGCCCTCA ACGTCAAGCT GCCGCGCACC
    301  GCCCGCGACA TGGCGGCGGC AAGCCGCAAA ATCCCCGACA GCCGCCTTAA
    351  GGCCGGCGAC CTCGTATTCT TCAACACCGG CGGCGCACAC CGCTACTCAC
    401  ACGTCGGACT CTATATCGGC AACGGCGAAT TCATCCATGC CCCCAGCAGC
    451  GGCAAAACCA TCAAAACCGA AAAACTCTCC ACACCGTTTT ACGCCAAAAA
    501  CTACCTCGGC GCACATACTT TCTTTACAGA ATGA
```

This corresponds to the amino acid sequence <SEQ ID 67; ORF 206.a>:

```
a206.pep
      1  MFPPDKTLFL CLSALLLASC GTTSGKHRQP KPKQTVRQIQ AVRISHIDRT
     51  QGSQELMLHS LGLIGTPYKW GGSSTATGFD CSGMIQFVYK NALNVKLPRT
    101  ARDMAAASRK IPDSRLKAGD LVFFNTGGAH RYSHVGLYIG NGEFIHAPSS
    151  GKTIKTEKLS TPFYAKNYLG AHTFFTE*
```

m206/a206 ORFs 206 and 206.a showed a 99.4% identity in 177 aa overlap

```
                        10        20        30        40        50        60
m206.pep    MFPPDKTLFLCLSALLLASCGTTSGKHRQPKPKQTVRQIQAVRISHIDRTQGSQELMLHS
            ||||||||||||||||||||||||||||||||||||||||||||||||||||||||||||
a206        MFPPDKTLFLCLSALLLASCGTTSGKHRQPKPKQTVRQIQAVRISHIDRTQGSQELMLHS
                        10        20        30        40        50        60

                        70        80        90       100       110       120
m206.pep    LGLIGTPYKWGGSSTATGFDCSGMIQFVYKNALNVKLPRTARDMAAASRKIPDSRXKAGD
            ||||||||||||||||||||||||||||||||||||||||||||||||||||||| ||||
a206        LGLIGTPYKWGGSSTATGFDCSGMIQFVYKNALNVKLPRTARDMAAASRKIPDSRLKAGD
                        70        80        90       100       110       120

                       130       140       150       160       170
m206.pep    LVFFNTGGAHRYSHVGLYIGNGEFIHAPSSGKTIKTEKLSTPFYAKNYLGAHTFFTEX
            ||||||||||||||||||||||||||||||||||||||||||||||||||||||||||
a206        LVFFNTGGAHRYSHVGLYIGNGEFIHAPSSGKTIKTEKLSTPFYAKNYLGAHTFFTEX
                       130       140       150       160       170
```

287

The following partial DNA sequence was identified in *N. meningitidis* <SEQ ID 68>:

m287.seq

1 ATGTTTAAAC GCAGCGTAAT CGCAATGGCT TGTATTTTTG CCCTTTCAGC
51 CTGCGGGGGC GGCGGTGGCG GATCGCCCGA TGTCAAGTCG GCGGACACGC
101 TGTCAAAACC TGCCGCCCCT GTTGTTTCTG AAAAAGAGAC AGAGGCAAAG
151 GAAGATGCGC CACAGGCAGG TTCTCAAGGA CAGGGCGCGC CATCCGCACA
201 AGGCAGTCAA GATATGGCGG CGGTTTCGGA AGAAAATACA GGCAATGGCG
251 GTGCGGTAAC AGCGGATAAT CCCAAAAATG AAGACGAGGT GGCACAAAAT
301 GATATGCCGC AAAATGCCGC CGGTACAGAT AGTTCGACAC CGAATCACAC
351 CCCGGATCCG AATATGCTTG CCGGAAATAT GGAAAATCAA GCAACGGATG
401 CCGGGGAATC GTCTCAGCCG GCAAACCAAC CGGATATGGC AAATGCGGCG
451 GACGGAATGC AGGGGGACGA TCCGTCGGCA GGCGGGCAAA ATGCCGGCAA
501 TACGGCTGCC CAAGGTGCAA ATCAAGCCGG AAACAATCAA GCCGCCGGTT
551 CTTCAGATCC CATCCCCGCG TCAAACCCTG CACCTGCGAA TGGCGGTAGC
601 AATTTTGGAA GGGTTGATTT GGCTAATGGC GTTTTGATTG ACGGGCCGTC
651 GCAAAATATA ACGTTGACCC ACTGTAAAGG CGATTCTTGT AGTGGCAATA
701 ATTTCTTGGA TGAAGAAGTA CAGCTAAAAT CAGAATTTGA AAAATTAAGT
751 GATGCAGACA AAATAAGTAA TTACAAGAAA GATGGGAAGA ATGATAAATT
801 TGTCGGTTTG GTTGCCGATA GTGTGCAGAT GAAGGGAATC AATCAATATA
851 TTATCTTTTA TAAACCTAAA CCCACTTCAT TTGCGCGATT TAGGCGTTCT
901 GCACGGTCGA GGCGGTCGCT TCCGGCCGAG ATGCCGCTGA TTCCCGTCAA
~~951 TCAGGCGGAT ACGCTGATTG TCGATGGGGA AGCGGTCAGC CTGACGGGGC~~
1001 ATTCCGGCAA TATCTTCGCG CCCGAAGGGA ATTACCGGTA TCTGACTTAC
1051 GGGGCGGAAA AATTGCCCGG CGGATCGTAT GCCCTTCGTG TTCAAGGCGA
1101 ACCGGCAAAA GGCGAAATGC TTGCGGGCGC GGCCGTGTAC AACGGCGAAG
1151 TACTGCATTT CCATACGGAA AACGGCCGTC CGTACCCGAC CAGGGGCAGG
1201 TTTGCCGCAA AAGTCGATTT CGGCAGCAAA TCTGTGGACG GCATTATCGA
1251 CAGCGGCGAT GATTTGCATA TGGGTACGCA AAAATTCAAA GCCGCCATCG
1301 ATGGAAACGG CTTTAAGGGG ACTTGGACGG AAAATGGCAG CGGGGATGTT
1351 TCCGGAAAGT TTTACGGCCC GGCCGGCGAG GAAGTGGCGG GAAAATACAG
1401 CTATCGCCCG ACAGATGCGG AAAAGGGCGG ATTCGGCGTG TTTGCCGGCA
1451 AAAAAGAGCA GGATTGA

This corresponds to the amino acid sequence <SEQ ID 69; ORF 287>:

m287.pep

1 MFKRSVIAMA CIFALSACGG GGGGSPDVKS ADTLSKPAAP VVSEKETEAK
51 EDAPQAGSQG QGAPSAQGSQ DMAAVSEENT GNGGAVTADN PKNEDEVAQN
101 DMPQNAAGTD SSTPNHTPDP NMLAGNMENQ ATDAGESSQP ANQPDMANAA
151 DGMQGDDPSA GGQNAGNTAA QGANQAGNNQ AAGSSDPIPA SNPAPANGGS
201 NFGRVDLANG VLIDGPSQNI TLTHCKGDSC SGNNFLDEEV QLKSEFEKLS
251 DADKISNYKK DGKNDKFVGL VADSVQMKGI NQYIIFYKPK PTSFARFRRS
301 ARSRRSLPAE MPLIPVNQAD TLIVDGEAVS LTGHSGNIFA PEGNYRYLTY
351 GAEKLPGGSY ALRVQGEPAK GEMLAGAAVY NGEVLHFHTE NGRPYPTRGR
401 FAAKVDFGSK SVDGIIDSGD DLHMGTQKFK AAIDGNGFKG TWTENGSGDV
451 SGKFYGPAGE EVAGKYSYRP TDAEKGGFGV FAGKKEQD*

The following partial DNA sequence was identified in *N. gonorrhoeae* <SEQ ID 70>:

```
g287.seq
   1  atgtttaaac gcagtgtgat tgcaatggct tgtatttttc ccctttcagc
  51  ctgtgggggc ggcggtggcg gatcgcccga tgtcaagtcg gcggacacgc
 101  cgtcaaaacc ggccgccccc gttgttgctg aaaatgccgg ggaaggggtg
 151  ctgccgaaag aaaagaaaga tgaggaggca gcgggcggtg cgccgcaagc
 201  cgatacgcag gacgcaaccg ccggagaagg cagccaagat atggcggcag
 251  tttcggcaga aaatacaggc aatggcggtg cggcaacaac ggacaacccc
 301  aaaaatgaag acgcgggggc gcaaaatgat atgccgcaaa atgccgccga
 351  atccgcaaat caaacaggga acaaccaacc cgccggttct tcagattccg
 401  cccccgcgtc aaaccctgcc cctgcgaatg gcggtagcga ttttggaagg
 451  acgaacgtgg gcaattctgt tgtgattgac ggaccgtcgc aaaatataac
 501  gttgacccac tgtaaaggcg attcttgtaa tggtgataat ttattggatg
 551  aagaagcacc gtcaaaatca gaatttgaaa aattaagtga tgaagaaaaa
 601  attaagcgat ataaaaaaga cgagcaacgg gagaattttg tcggtttggt
 651  tgctgacagg gtaaaaaagg atggaactaa caaatatatc atcttctata
 701  cggacaaacc acctactcgt tctgcacggt cgaggaggtc gcttccggcc
 751  gagattccgc tgattcccgt caatcaggcc gatacgctga ttgtggatgg
 801  ggaagcggtc agcctgacgg ggcattccgg caatatcttc gcgcccgaag
 851  ggaattaccg gtatctgact tacggggcgg aaaaattgcc cggcggatcg
 901  tatgccctcc gtgtgcaagg cgaaccggca aaaggcgaaa tgcttgttgg
 951  cacggccgtg tacaacggcg aagtgctgca tttccatatg gaaaacggcc
1001  gtccgtaccc gtccggaggc aggtttgccg caaaagtcga tttcggcagc
1051  aaatctgtgg acggcattat cgacagcggc gatgatttgc atatgggtac
1101  gcaaaaattc aaagccgcca tcgatggaaa cggctttaag gggacttgga
1151  cggaaaatgg cggcggggat gtttccggaa ggttttacgg cccggccggc
1201  gaggaagtgg cgggaaaata cagctatcgc ccgacagatg ctgaaaaggg
1251  cggattcggc gtgtttgccg gcaaaaaaga tcgggattga
```

This corresponds to the amino acid sequence <SEQ ID 71; ORF 287.ng>:

```
g287.pep
  1  MFKRSVIAMA CIFPLSACGG GGGGSPDVKS ADTPSKPAAP VVAENAGEGV
 51  LPKEKKDEEA AGGAPQADTQ DATAGEGSQD MAAVSAENTG NGGAATTDNP
101  KNEDAGAQND MPQNAAESAN QTGNNQPAGS SDSAPASNPA PANGGSDFGR
151  TNVGNSVVID GPSQNITLTH CKGDSCNGDN LLDEEAPSKS EFEKLSDEEK
201  IKRYKKDEQR ENFVGLVADR VKKDGTNKYI IFYTDKPPTR SARSRRSLPA
251  EIPLIPVNQA DTLIVDGEAV SLTGHSGNIF APEGNYRYLT YGAEKLPGGS
301  YALRVQGEPA KGEMLVGTAV YNGEVLHFHM ENGRPYPSGG RFAAKVDFGS
351  KSVDGIIDSG DDLHMGTQKF KAAIDGNGFK GTWTENGGGD VSGRFYGPAG
401  EEVAGKYSYR PTDAEKGGFG VFAGKKDRD*
```

**m287/g287** ORFs 287 and 287.ng showed a 70.1% identity in 499 aa overlap

```
                    10        20        30        40                   49
m287.pep    MFKRSVIAMACIFALSACGGGGGGSPDVKSADTLSKPAAPVVSE-----------KETEA
            ||||||||||||| ||||||||||||||||||| ||||||||:|           |: ||
g287        MFKRSVIAMACIFPLSACGGGGGGSPDVKSADTPSKPAAPVVAENAGEGVLPKEKKDEEA
                    10        20        30        40        50        60

           50        60        70        80        90       100       109
m287.pep    KEDAPQAGSQGQGAPSAQGSQDMAAVSEENTGNGGAVTADNPKNEDEVAQNDMPQNAAGT
              |||| :|      | :::|||||||||| ||||||||:|:|||||||  ||||||||||
g287        AGGAPQADTQD--ATAGEGSQDMAAVSAENTGNGGAATTDNPKNEDAGAQNDMPQNAA--
                    70        80        90       100       110
```

```
            110       120       130       140       150       160      169
m287.pep    DSSTPNHTPDPNMLAGNMENQATDAGESSQPANQPDMANAADGMQGDDPSAGGQNAGNTA

g287        ------------------------------------------------------------


            170       180       190       200       210       220      229
m287.pep    AQGANQAGNNQAAGSSDPIPASNPAPANGGSNFGRVDLANGVLIDGPSQNITLTHCKGDS
            ::|||:|||| |||||  |||||||||||:|||::::|:|:|||||||||||||||||||
g287        -ESANQTGNNQPAGSSDSAPASNPAPANGGSDFGRTNVGNSVVIDGPSQNITLTHCKGDS
              120       130       140       150       160       170

            230       240       250       260       270       280      289
m287.pep    CSGNNFLDEEVQLKSEFEKLSDADKISNYKKDGKNDKFVGLVADSVQMKGINQYIIFYKP
            |:|:|:||||:  |||||||| :||: |||| : ::||||||| |:  | |:|||||
g287        CNGDNLLDEEAPSKSEFEKLSDEEKIKRYKKDEQRENFVGLVADRVKKDGTNKYIIFYTD
              180       190       200       210       220       230

            290       300       310       320       330       340      349
m287.pep    KPTSFARFRRSARSRRSLPAEMPLIPVNQADTLIVDGEAVSLTGHSGNIFAPEGNYRYLT
            || :       |||||||||||:|||||||||||||||||||||||||||||||||||||
g287        KPPT-----RSARSRRSLPAEIPLIPVNQADTLIVDGEAVSLTGHSGNIFAPEGNYRYLT
                  240       250       260       270       280       290

            350       360       370       380       390       400      409
m287.pep    YGAEKLPGGSYALRVQGEPAKGEMLAGAAVYNGEVLHFHTENGRPYPTRGRFAAKVDFGS
            ||||||||||||||||||||||||:|:||||||||||| |||||||: ||||||||||
g287        YGAEKLPGGSYALRVQGEPAKGEMLVGTAVYNGEVLHFHMENGRPYPSGGRFAAKVDFGS
                  300       310       320       330       340       350

            410       420       430       440       450       460      469
m287.pep    KSVDGIIDSGDDLHMGTQKFKAAIDGNGFKGTWTENGSGDVSGKFYGPAGEEVAGKYSYR
            ||||||||||||||||||||||||||||||||||||:|||||:|||||||||||||||||
g287        KSVDGIIDSGDDLHMGTQKFKAAIDGNGFKGTWTENGGGDVSGRFYGPAGEEVAGKYSYR
                  360       370       380       390       400       410

            470       480      489
m287.pep    PTDAEKGGFGVFAGKKEQDX
            ||||||||||||||||::||
g287        PTDAEKGGFGVFAGKKDRDX
                  420       430
```

The following partial DNA sequence was identified in *N. meningitidis* <SEQ ID 72>:

```
a287.seq
  1  ATGTTTAAAC GCAGTGTGAT TGCAATGGCT TGTATTGTTG CCCTTTCAGC
 51  CTGTGGGGGC GGCGGTGGCG GATCGCCCGA TGTTAAGTCG GCGGACACGC
101  TGTCAAAACC TGCCGCCCCT GTTGTTACTG AAGATGTCGG GGAAGAGGTG
151  CTGCCGAAAG AAAAGAAAGA TGAGGAGGCG GTGAGTGGTG CGCCGCAAGC
201  CGATACGCAG GACGCAACCG CCGGAAAAGG CGGTCAAGAT ATGGCGGCAG
251  TTTCGGCAGA AAATACAGGC AATGGCGGTG CGGCAACAAC GGATAATCCC
301  GAAAATAAAG ACGAGGGACC GCAAAATGAT ATGCCGCAAA ATGCCGCCGA
351  TACAGATAGT TCGACACCGA ATCACACCCC TGCACCGAAT ATGCCAACCA
401  GAGATATGGG AAACCAAGCA CCGGATGCCG GGGAATCGGC ACAACCGGCA
451  AACCAACCGG ATATGGCAAA TGCGGCGGAC GGAATGCAGG GGGACGATCC
501  GTCGGCAGGG GAAAATGCCG GCAATACGGC AGATCAAGCT GCAAATCAAG
551  CTGAAAACAA TCAAGTCGGC GGCTCTCAAA ATCCTGCCTC TTCAACCAAT
601  CCTAACGCCA CGAATGGCGG CAGCGATTTT GGAAGGATAA ATGTAGCTAA
651  TGGCATCAAG CTTGACAGCG GTTCGGAAAA TGTAACGTTG ACACATTGTA
701  AAGACAAAGT ATGCGATAGA GATTTCTTAG ATGAAGAAGC ACCACCAAAA
751  TCAGAATTTG AAAAATTAAG TGATGAAGAA AAAATTAATA AATATAAAAA
801  AGACGAGCAA CGAGAGAATT TTGTCGGTTT GGTTGCTGAC AGGGTAGAAA
```

```
 851  AGAATGGAAC TAACAAATAT GTCATCATTT ATAAAGACAA GTCCGCTTCA
 901  TCTTCATCTG CGCGATTCAG GCGTTCTGCA CGGTCGAGGC GGTCGCTTCC
 951  GGCCGAGATG CCGCTGATTC CCGTCAATCA GGCGGATACG CTGATTGTCG
1001  ATGGGGAAGC GGTCAGCCTG ACGGGGCATT CCGGCAATAT CTTCGCGCCC
1051  GAAGGGAATT ACCGGTATCT GACTTACGGG GCGGAAAAAT TGTCCGGCGG
1101  ATCGTATGCC CTCAGTGTGC AAGGCGAACC GGCAAAAGGC GAAATGCTTG
1151  CGGGCACGGC CGTGTACAAC GGCGAAGTGC TGCATTTCCA TATGGAAAAC
1201  GGCCGTCCGT CCCCGTCCGG AGGCAGGTTT GCCGCAAAAG TCGATTTCGG
1251  CAGCAAATCT GTGGACGGCA TTATCGACAG CGGCGATGAT TTGCATATGG
1301  GTACGCAAAA ATTCAAAGCC GTTATCGATG GAAACGGCTT TAAGGGGACT
1351  TGGACGGAAA ATGGCGGCGG GGATGTTTCC GGAAGGTTTT ACGGCCCGGC
1401  CGGCGAAGAA GTGGCGGGAA AATACAGCTA TCGCCCGACA GATGCGGAAA
1451  AGGGCGGATT CGGCGTGTTT GCCGGCAAAA AAGAGCAGGA TTGA
```

This corresponds to the amino acid sequence <SEQ ID 73; ORF 287.a>:

```
a287.pep
   1  MFKRSVIAMA CIVALSACGG GGGGSPDVKS ADTLSKPAAP VVTEDVGEEV
  51  LPKEKKDEEA VSGAPQADTQ DATAGKGGQD MAAVSAENTG NGGAATTDNP
 101  ENKDEGPQND MPQNAADTDS STPNHTPAPN MPTRDMGNQA PDAGESAQPA
 151  NQPDMANAAD GMQGDDPSAG ENAGNTADQA ANQAENNQVG GSQNPASSTN
 201  PNATNGGSDF GRINVANGIK LDSGSENVTL THCKDKVCDR DFLDEEAPPK
 251  SEFEKLSDEE KINKYKKDEQ RENFVGLVAD RVEKNGTNKY VIIYKDKSAS
 301  SSSARFRRSA RSRRSLPAEM PLIPVNQADT LIVDGEAVSL TGHSGNIFAP
 351  EGNYRYLTYG AEKLSGGSYA LSVQGEPAKG EMLAGTAVYN GEVLHFHMEN
 401  GRPSPSGGRF AAKVDFGSKS VDGIIDSGDD LHMGTQKFKA VIDGNGFKGT
 451  WTENGGGDVS GRFYGPAGEE VAGKYSYRPT DAEKGGFGVF AGKKEQD*
```

```
m287/a287     ORFs 287 and 287.a showed a 77.2% identity in 501 aa overlap

                        10        20        30        40                   49
m287.pep      MFKRSVIAMACIFALSACGGGGGGSPDVKSADTLSKPAAPVVSE-----------KETEA
              |||||||||||| |||||||||||||||||||||||||||||:|           |: ||
a287          MFKRSVIAMACIVALSACGGGGGGSPDVKSADTLSKPAAPVVTEDVGEEVLPKEKKDEEA
                        10        20        30        40        50        60

                 50        60        70        80        90       100      109
m287.pep      KEDAPQAGSQGQGAPSAQGSQDMAAVSEENTGNGGAVTADNPKNEDEVAQNDMPQNAAGT
                 |||| :|   | :::|:|||||||| ||||||||:|:|||:|:||  ||||||||| |
a287          VSGAPQADTQ--DATAGKGGQDMAAVSAENTGNGGAATTDNPENKDEGPQNDMPQNAADT
                        70        80        90       100       110

              110       120       130       140       150       160      169
m287.pep      DSSTPNHTPDPNMLAGNMENQATDAGESSQPANQPDMANAADGMQGDDPSAGGQNAGNTA
              ||||||||| ||| : :| ||| |||||:|||||||||||||||||||||||| :|||||
a287          DSSTPNHTPAPNMPTRDMGNQAPDAGESAQPANQPDMANAADGMQGDDPSAG-ENAGNTA
               120       130       140       150       160       170

              170       180       190       200       210       220      229
m287.pep      AQGANQAGNNQAAGSSDPIPASNPAPANGGSNFGRVDLANGVLIDGPSQNITLTHCKGDS
              |:|||| |||::||::|    ::||  :||||:|||::::|||: :|: |:|:||||||
a287          DQAANQAENNQVGGSQNPASSTNPNATNGGSDFGRINVANGIKLDSGSENVTLTHCKDKV
                180       190       200       210       220       230

              230       240       250       260       270       280      289
m287.pep      CSGNNFLDEEVQLKSEFEKLSDADKISNYKKDGKNDKFVGLVADSVQMKGINQYIIFYKP
              |:  :|||||:  ||||||||| :||::|||| : ::||||||| |: :| |:|:|:||
a287          CD-RDFLDEEAPPKSEFEKLSDEEKINKYKKDEQRENFVGLVADRVEKNGTNKYVIIYKD
                240       250       260       270       280       290

              290       300       310       320       330       340
m287.pep      KP--TSFARFRRSARSRRSLPAEMPLIPVNQADTLIVDGEAVSLTGHSGNIFAPEGNYRY
              |   :|||||||||||||||||||||||||||||||||||||||||||||||||||||||
```

```
a287          KSASSSSARFRRSARSRRSLPAEMPLIPVNQADTLIVDGEAVSLTGHSGNIFAPEGNYRY
               300       310       320       330       340       350

              350       360       370       380       390       400
m287.pep      LTYGAEKLPGGSYALRVQGEPAKGEMLAGAAVYNGEVLHFHTENGRPYPTRGRFAAKVDF
              |||||||| |||||| |||||||||||||:||||||||||| ||||| |: |||||||||
a287          LTYGAEKLSGGSYALSVQGEPAKGEMLAGTAVYNGEVLHFHMENGRPSPSGGRFAAKVDF
               360       370       380       390       400       410

              410       420       430       440       450       460
m287.pep      GSKSVDGIIDSGDDLHMGTQKFKAAIDGNGFKGTWTENGSGDVSGKFYGPAGEEVAGKYS
              ||||||||||||||||||||||||:||||||||||||||:|||||:||||||||||||||
a287          GSKSVDGIIDSGDDLHMGTQKFKAVIDGNGFKGTWTENGGGDVSGRFYGPAGEEVAGKYS
               420       430       440       450       460       470

              470       480      489
m287.pep      YRPTDAEKGGFGVFAGKKEQDX
              ||||||||||||||||||||||
a287          YRPTDAEKGGFGVFAGKKEQDX
               480       490
```

## 406

The following partial DNA sequence was identified in *N. meningitidis* <SEQ ID 74>:

```
m406.seq
      1  ATGCAAGCAC GGCTGCTGAT ACCTATTCTT TTTTCAGTTT TTATTTTATC
     51  CGCCTGCGGG ACACTGACAG GTATTCCATC GCATGGCGGA GGTAAACGCT
    101  TTGCGGTCGA ACAAGAACTT GTGGCCGCTT CTGCCAGAGC TGCCGTTAAA
    151  GACATGGATT TACAGGCATT ACACGGACGA AAAGTTGCAT TGTACATTGC
    201  CACTATGGGC GACCAAGGTT CAGGCAGTTT GACAGGGGGT CGCTACTCCA
    251  TTGATGCACT GATTCGTGGC GAATACATAA ACAGCCCTGC CGTCCGTACC
    301  GATTACACCT ATCCACGTTA CGAAACCACC GCTGAAACAA CATCAGGCGG
    351  TTTGACAGGT TTAACCACTT CTTTATCTAC ACTTAATGCC CCTGCACTCT
    401  CTCGCACCCA ATCAGACGGT AGCGGAAGTA AAAGCAGTCT GGGCTTAAAT
    451  ATTGGCGGGA TGGGGGATTA TCGAAATGAA ACCTTGACGA CTAACCCGCG
    501  CGACACTGCC TTTCTTTCCC ACTTGGTACA GACCGTATTT TTCCTGCGCG
    551  GCATAGACGT TGTTTCTCCT GCCAATGCCG ATACAGATGT GTTTATTAAC
    601  ATCGACGTAT TCGGAACGAT ACGCAACAGA ACCGAAATGC ACCTATACAA
    651  TGCCGAAACA CTGAAAGCCC AAACAAAACT GGAATATTTC GCAGTAGACA
    701  GAACCAATAA AAAATTGCTC ATCAAACCAA AAACCAATGC GTTTGAAGCT
    751  GCCTATAAAG AAAATTACGC ATTGTGGATG GGGCCGTATA AAGTAAGCAA
    801  AGGAATTAAA CCGACGGAAG GATTAATGGT CGATTTCTCC GATATCCGAC
    851  CATACGGCAA TCATACGGGT AACTCCGCCC CATCCGTAGA GGCTGATAAC
    901  AGTCATGAGG GGTATGGATA CAGCGATGAA GTAGTGCGAC AACATAGACA
    951  AGGACAACCT TGA
```

This corresponds to the amino acid sequence <SEQ ID 75; ORF 406>:

```
m406.pep
      1  MQARLLIPIL FSVFILSACG TLTGIPSHGG GKRFAVEQEL VAASARAAVK
     51  DMDLQALHGR KVALYIATMG DQGSGSLTGG RYSIDALIRG EYINSPAVRT
    101  DYTYPRYETT AETTSGGLTG LTTSLSTLNA PALSRTQSDG SGSKSSLGLN
    151  IGGMGDYRNE TLTTNPRDTA FLSHLVQTVF FLRGIDVVSP ANADTDVFIN
    201  IDVFGTIRNR TEMHLYNAET LKAQTKLEYF AVDRTNKKLL IKPKTNAFEA
    251  AYKENYALWM GPYKVSKGIK PTEGLMVDFS DIRPYGNHTG NSAPSVEADN
    301  SHEGYGYSDE VVRQHRQGQP *
```

The following partial DNA sequence was identified in *N. gonorrhoeae* <SEQ ID 76>:

**g406.seq**

```
  1  ATGCGGGCAC GGCTGCTGAT ACCTATTCTT TTTTCAGTTT TTATTTTATC
 51  CGCCTGCGGG ACACTGACAG GTATTCCATC GCATGGCGGA GGCAAACGCT
101  TCGCGGTCGA ACAAGAACTT GTGGCCGCTT CTGCCAGAGC TGCCGTTAAA
151  GACATGGATT TACAGGCATT ACACGGACGA AAAGTTGCAT TGTACATTGC
201  AACTATGGGC GACCAAGGTT CAGGCAGTTT GACAGGGGGT CGCTACTCCA
251  TTGATGCACT GATTCGCGGC GAATACATAA ACAGCCCTGC CGTCCGCACC
301  GATTACACCT ATCCGCGTTA CGAAACCACC GCTGAAACAA CATCAGGCGG
351  TTTGACGGGT TTAACCACTT CTTTATCTAC ACTTAATGCC CCTGCACTCT
401  CGCGCACCCA ATCAGACGGT AGCGGAAGTA GGAGCAGTCT GGGCTTAAAT
451  ATTGGCGGGA TGGGGGATTA TCGAAATGAA ACCTTGACGA CCAACCCGCG
501  CGACACTGCC TTTCTTTCCC ACTTGGTGCA GACCGTATTT TTCCTGCGCG
551  GCATAGACGT TGTTTCTCCT GCCAATGCCG ATACAGATGT GTTTATTAAC
601  ATCGACGTAT TCGGAACGAT ACGCAACAGA ACCGAAATGC ACCTATACAA
651  TGCCGAAACA CTGAAAGCCC AAACAAAACT GGAATATTTC GCAGTAGACA
701  GAACCAATAA AAAATTGCTC ATCAAACCCA AAACCAATGC GTTTGAAGCT
751  GCCTATAAAG AAAATTACGC ATTGTGGATG GGGCCGTATA AAGTAAGCAA
801  AGGAATCAAA CCGACGGAAG GATTGATGGT CGATTTCTCC GATATCCAAC
851  CATACGGCAA TCATACGGGT AACTCCGCCC CATCCGTAGA GGCTGATAAC
901  AGTCATGAGG GGTATGGATA CAGCGATGAA GCAGTGCGAC AACATAGACA
951  AGGGCAACCT TGA
```

This corresponds to the amino acid sequence <SEQ ID 77; ORF 406.ng>:

**g406.pep**

```
  1  MRARLLIPIL FSVFILSACG TLTGIPSHGG GKRFAVEQEL VAASARAAVK
 51  DMDLQALHGR KVALYIATMG DQGSGSLTGG RYSIDALIRG EYINSPAVRT
101  DYTYPRYETT AETTSGGLTG LTTSLSTLNA PALSRTQSDG SGSRSSLGLN
151  IGGMGDYRNE TLTTNPRDTA FLSHLVQTVF FLRGIDVVSP ANADTDVFIN
201  IDVFGTIRNR TEMHLYNAET LKAQTKLEYF AVDRTNKKLL IKPKTNAFEA
251  AYKENYALWM GPYKVSKGIK PTEGLMVDFS DIQPYGNHTG NSAPSVEADN
301  SHEGYGYSDE AVRQHRQGQP *
```

ORF 406.ng shows 98.8% identity over a 320 aa overlap with a predicted ORF (ORF406.a) from *N. gonorrhoeae:*

**g406/m406**

```
                    10        20        30        40        50        60
g406.pep    MRARLLIPILFSVFILSACGTLTGIPSHGGGKRFAVEQELVAASARAAVKDMDLQALHGR
            |:||||||||||||||||||||||||||||||||||||||||||||||||||||||||||
m406        MQARLLIPILFSVFILSACGTLTGIPSHGGGKRFAVEQELVAASARAAVKDMDLQALHGR
                    10        20        30        40        50        60

                    70        80        90       100       110       120
g406.pep    KVALYIATMGDQGSGSLTGGRYSIDALIRGEYINSPAVRTDYTYPRYETTAETTSGGLTG
            ||||||||||||||||||||||||||||||||||||||||||||||||||||||||||||
m406        KVALYIATMGDQGSGSLTGGRYSIDALIRGEYINSPAVRTDYTYPRYETTAETTSGGLTG
                    70        80        90       100       110       120

                   130       140       150       160       170       180
g406.pep    LTTSLSTLNAPALSRTQSDGSGSRSSLGLNIGGMGDYRNETLTTNPRDTAFLSHLVQTVF
            |||||||||||||||||||||||:||||||||||||||||||||||||||||||||||||
m406        LTTSLSTLNAPALSRTQSDGSGSKSSLGLNIGGMGDYRNETLTTNPRDTAFLSHLVQTVF
                   130       140       150       160       170       180

                   190       200       210       220       230       240
```

```
g406.pep   FLRGIDVVSPANADTDVFINIDVFGTIRNRTEMHLYNAETLKAQTKLEYFAVDRTNKKLL
           ||||||||||||||||||||||||||||||||||||||||||||||||||||||||||||
m406       FLRGIDVVSPANADTDVFINIDVFGTIRNRTEMHLYNAETLKAQTKLEYFAVDRTNKKLL
                   190       200       210       220       230       240

                   250       260       270       280       290       300
g406.pep   IKPKTNAFEAAYKENYALWMGPYKVSKGIKPTEGLMVDFSDIQPYGNHTGNSAPSVEADN
           ||||||||||||||||||||||||||||||||||||||||||:|||||||||||||||||
m406       IKPKTNAFEAAYKENYALWMGPYKVSKGIKPTEGLMVDFSDIRPYGNHTGNSAPSVEADN
                   250       260       270       280       290       300

                   310       320
g406.pep   SHEGYGYSDEAVRQHRQGQPX
           ||||||||||:||||||||||
m406       SHEGYGYSDEVVRQHRQGQPX
                   310       320
```

The following partial DNA sequence was identified in *N. meningitidis* <SEQ ID 78>:

```
a406.seq
      1  ATGCAAGCAC GGCTGCTGAT ACCTATTCTT TTTTCAGTTT TTATTTTATC
     51  CGCCTGCGGG ACACTGACAG GTATTCCATC GCATGGCGGA GGTAAACGCT
    101  TCGCGGTCGA ACAAGAACTT GTGGCCGCTT CTGCCAGAGC TGCCGTTAAA
    151  GACATGGATT TACAGGCATT ACACGGACGA AAAGTTGCAT TGTACATTGC
    201  AACTATGGGC GACCAAGGTT CAGGCAGTTT GACAGGGGGT CGCTACTCCA
    251  TTGATGCACT GATTCGTGGC GAATACATAA ACAGCCCTGC CGTCCGTACC
    301  GATTACACCT ATCCACGTTA CGAAACCACC GCTGAAACAA CATCAGGCGG
    351  TTTGACAGGT TTAACCACTT CTTTATCTAC ACTTAATGCC CCTGCACTCT
    401  CGCGCACCCA ATCAGACGGT AGCGGAAGTA AAAGCAGTCT GGGCTTAAAT
    451  ATTGGCGGGA TGGGGGATTA TCGAAATGAA ACCTTGACGA CTAACCCGCG
    501  CGACACTGCC TTTCTTTCCC ACTTGGTACA GACCGTATTT TTCCTGCGCG
    551  GCATAGACGT TGTTTCTCCT GCCAATGCCG ATACGGATGT GTTTATTAAC
    601  ATCGACGTAT TCGGAACGAT ACGCAACAGA ACCGAAATGC ACCTATACAA
    651  TGCCGAAACA CTGAAAGCCC AAACAAAACT GGAATATTTC GCAGTAGACA
    701  GAACCAATAA AAAATTGCTC ATCAAACCAA AAACCAATGC GTTTGAAGCT
    751  GCCTATAAAG AAAATTACGC ATTGTGGATG GGACCGTATA AAGTAAGCAA
    801  AGGAATTAAA CCGACAGAAG GATTAATGGT CGATTTCTCC GATATCCAAC
    851  CATACGGCAA TCATATGGGT AACTCTGCCC CATCCGTAGA GGCTGATAAC
    901  AGTCATGAGG GGTATGGATA CAGCGATGAA GCAGTGCGAC GACATAGACA
    951  AGGGCAACCT TGA
```

This corresponds to the amino acid sequence <SEO ID 79: ORF 406.a>:

```
a406.pep
      1  MQARLLIPIL FSVFILSACG TLTGIPSHGG GKRFAVEQEL VAASARAAVK
     51  DMDLQALHGR KVALYIATMG DQGSGSLTGG RYSIDALIRG EYINSPAVRT
    101  DYTYPRYETT AETTSGGLTG LTTSLSTLNA PALSRTQSDG SGSKSSLGLN
    151  IGGMGDYRNE TLTTNPRDTA FLSHLVQTVF FLRGIDVVSP ANADTDVFIN
    201  IDVFGTIRNR TEMHLYNAET LKAQTKLEYF AVDRTNKKLL IKPKTNAFEA
    251  AYKENYALWM GPYKVSKGIK PTEGLMVDFS DIQPYGNHMG NSAPSVEADN
    301  SHEGYGYSDE AVRRHRQGQP *
```

```
m406/a406    ORFs 406 and 406.a showed a 98.8% identity in 320 aa overlap

                     10        20        30        40        50        60
m406.pep     MQARLLIPILFSVFILSACGTLTGIPSHGGGKRFAVEQELVAASARAAVKDMDLQALHGR
             ||||||||||||||||||||||||||||||||||||||||||||||||||||||||||||
a406         MQARLLIPILFSVFILSACGTLTGIPSHGGGKRFAVEQELVAASARAAVKDMDLQALHGR
                     10        20        30        40        50        60

                     70        80        90       100       110       120
m406.pep     KVALYIATMGDQGSGSLTGGRYSIDALIRGEYINSPAVRTDYTYPRYETTAETTSGGLTG
             ||||||||||||||||||||||||||||||||||||||||||||||||||||||||||||
```

```
a406        KVALYIATMGDQGSGSLTGGRYSIDALIRGEYINSPAVRTDYTYPRYETTAETTSGGLTG
                        70        80        90       100       110       120

                       130       140       150       160       170       180
m406.pep    LTTSLSTLNAPALSRTQSDGSGSKSSLGLNIGGMGDYRNETLTTNPRDTAFLSHLVQTVF
            ||||||||||||||||||||||||||||||||||||||||||||||||||||||||||||
a406        LTTSLSTLNAPALSRTQSDGSGSKSSLGLNIGGMGDYRNETLTTNPRDTAFLSHLVQTVF
                       130       140       150       160       170       180

                       190       200       210       220       230       240
m406.pep    FLRGIDVVSPANADTDVFINIDVFGTIRNRTEMHLYNAETLKAQTKLEYFAVDRTNKKLL
            ||||||||||||||||||||||||||||||||||||||||||||||||||||||||||||
a406        FLRGIDVVSPANADTDVFINIDVFGTIRNRTEMHLYNAETLKAQTKLEYFAVDRTNKKLL
                       190       200       210       220       230       240

                       250       260       270       280       290       300
m406.pep    IKPKTNAFEAAYKENYALWMGPYKVSKGIKPTEGLMVDFSDIRPYGNHTGNSAPSVEADN
            ||||||||||||||||||||||||||||||||||||||||||:||||| ||||||||||||
a406        IKPKTNAFEAAYKENYALWMGPYKVSKGIKPTEGLMVDFSDIQPYGNHMGNSAPSVEADN
                       250       260       270       280       290       300

                       310       320
m406.pep    SHEGYGYSDEVVRQHRQGQPX
            ||||||||||:||:|||||||
a406        SHEGYGYSDEAVRRHRQGQPX
                       310       320
```

The following partial DNA sequence was identified in *N. meningitidis* <SEQ ID 80>:

```
m726.seq
      1  ATGACCATCT ATTTCAAAAA CGGCTTTTAC GACGACACAT TGGGCGGCAT
     51  CCCCGAAGGC GCGGTTGCCG TCCGCGCCGA AGAATACGCC GCCCTTTTGG
    101  CAGGACAGGC GCAGGGCGGG CAGATTGCCG CAGATTCCGA CGGCCGCCCC
    151  GTTTTAACCC CGCCGCGCCC GTCCGATTAC CACGAATGGG ACGGCAAAAA
    201  ATGGAAAATC AGCAAAGCCG CCGCCGCCGC CCGTTTCGCC AAACAAAAAA
    251  CCGCCTTGGC ATTCCGCCTC GCGGAAAAGG CGGACGAACT CAAAAACAGC
    301  CTCTTGGCGG GCTATCCCCA AGTGGAAATC GACAGCTTTT ACAGGCAGGA
    351  AAAAGAAGCC CTCGCGCGGC AGGCGGACAA CAACGCCCCG ACCCCGATGC
    401  TGGCGCAAAT CGCCGCCGCA AGGGGCGTGG AATTGGACGT TTTGATTGAA
    451  AAAGTTATCG AAAAATCCGC CCGCCTGGCT GTTGCGGCCG GCGCGATTAT
    501  CGGAAAGCGT CAGCAGCTCG AAGACAAATT GAACACCATC GAAACCGCGC
    551  CCGGATTGGA CGCGCTGGAA AAGGAAATCG AAGAATGGAC GCTAAACATC
    601  GGCTGA
```

This corresponds to the amino acid sequence <SEQ ID 81; ORF 726>:

```
m726.pep
      1  MTIYFKNGFY DDTLGGIPEG AVAVRAEEYA ALLAGQAQGG QIAADSDGRP
     51  VLTPPRPSDY HEWDGKKWKI SKAAAAARFA KQKTALAFRL AEKADELKNS
    101  LLAGYPQVEI DSFYRQEKEA LARQADNNAP TPMLAQIAAA RGVELDVLIE
    151  KVIEKSARLA VAAGAIIGKR QQLEDKLNTI ETAPGLDALE KEIEEWTLNI
    201  G*
```

The following partial DNA sequence was identified in *N. meningitidis* <SEQ ID 82>:

```
m907-2.seq
      1  ATGAGAAAAC CGACCGATAC CCTACCCGTT AATCTGCAAC GCCGCCGCCT
     51  GTTGTGTGCC GCCGGTGCGT TGTTGCTCAG TCCTCTGGCG CACGCCGGCG
    101  CGCAACGTGA GGAAACGCTT GCCGACGATG TGGCTTCCGT GATGAGGAGT
```

```
151  TCTGTCGGCA GCGTCAATCC GCCGAGGCTG GTGTTTGACA ATCCGAAAGA
201  GGGCGAGCGT TGGTTGTCTG CCATGTCGGC ACGTTTGGCA AGGTTCGTCC
251  CCGAGGAGGA GGAGCGGCGC AGGCTGCTGG TCAATATCCA GTACGAAAGC
301  AGCCGGGCCG GTTTGGATAC GCAGATTGTG TTGGGGCTGA TTGAGGTGGA
351  AAGCGCGTTC CGCCAGTATG CAATCAGCGG TGTCGGCGCG CGCGGCCTGA
401  TGCAGGTTAT GCCGTTTTGG AAAAACTACA TCGGCAAACC GGCGCACAAC
451  CTGTTCGACA TCCGCACCAA CCTGCGTTAC GGCTGTACCA TCCTGCGCCA
501  TTACCGGAAT CTTGAAAAAG GCAACATCGT CCGCGCGCTT GCCCGCTTTA
551  ACGGCAGCTT GGGCAGCAAT AAATATCCGA ACGCCGTTTT GGGCGCGTGG
601  CGCAACCGCT GGCAGTGGCG TTGA
```

This corresponds to the amino acid sequence <SEQ ID 83; ORF 907-2>:

**m907-2.pep**

```
  1  MRKPTDTLPV NLQRRRLLCA AGALLLSPLA HAGAQREETL ADDVASVMRS
 51  SVGSVNPPRL VFDNPKEGER WLSAMSARLA RFVPEEEERR RLLVNIQYES
101  SRAGLDTQIV LGLIEVESAF RQYAISGVGA RGLMQVMPFW KNYIGKPAHN
151  LFDIRTNLRY GCTILRHYRN LEKGNIVRAL ARFNGSLGSN KYPNAVLGAW
201  RNRWQWR*
```

The following partial DNA sequence was identified in *N. meningitidis* <SEQ ID 84>:

**m953.seq**

```
  1  ATGAAAAAAA TCATCTTCGC CGCACTCGCA GCCGCCGCCA TCAGTACTGC
 51  CTCCGCCGCC ACCTACAAAG TGGACGAATA TCACGCCAAC GCCCGTTTCG
101  CCATCGACCA TTTCAACACC AGCACCAACG TCGGCGGTTT TTACGGTCTG
151  ACCGGTTCCG TCGAGTTCGA CCAAGCAAAA CGCGACGGTA AAATCGACAT
201  CACCATCCCC ATTGCCAACC TGCAAAGCGG TTCGCAACAC TTTACCGACC
251  ACCTGAAATC AGCCGACATC TTCGATGCCG CCCAATATCC GGACATCCGC
301  TTTGTTTCCA CCAAATTCAA CTTCAACGGC AAAAAACTGG TTTCCGTTGA
351  CGGCAACCTG ACCATGCACG GCAAAACCGC CCCCGTCAAA CTCAAAGCCG
401  AAAAATTCAA CTGCTACCAA AGCCCGATGG AGAAAACCGA AGTTTGTGGC
451  GGCGACTTCA GCACCACCAT CGACCGCACC AAATGGGGCA TGGACTACCT
501  CGTTAACGTT GGTATGACCA AAAGCGTCCG CATCGACATC CAAATCGAGG
551  CAGCCAAACA ATAA
```

This corresponds to the amino acid sequence <SEQ ID 85; ORF 953>:

**m953.pep**

```
  1  MKKIIFAALA AAAISTASAA TYKVDEYHAN ARFAIDHFNT STNVGGFYGL
 51  TGSVEFDQAK RDGKIDITIP IANLQSGSQH FTDHLKSADI FDAAQYPDIR
101  FVSTKFNFNG KKLVSVDGNL TMHGKTAPVK LKAEKFNCYQ SPMEKTEVCG
151  GDFSTTIDRT KWGMDYLVNV GMTKSVRIDI QIEAAKQ*
```

The following partial DNA sequence was identified in *N. meningitidis* <SEQ ID 86>:

**orf1-1.seq**

```
  1  ATGAAAACAA CCGACAAACG GACAACCGAA ACACACCGCA AAGCCCCGAA
 51  AACCGGCCGC ATCCGCTTCT CGCCTGCTTA CTTAGCCATA TGCCTGTCGT
101  TCGGCATTCT TCCCCAAGCC TGGGCGGGAC ACACTTATTT CGGCATCAAC
151  TACCAATACT ATCGCGACTT TGCCGAAAAT AAAGGCAAGT TTGCAGTCGG
201  GGCGAAAGAT ATTGAGGTTT ACAACAAAAA AGGGGAGTTG GTCGGCAAAT
251  CAATGACAAA AGCCCCGATG ATTGATTTTT CTGTGGTGTC GCGTAACGGC
301  GTGGCGGCAT TGGTGGGCGA TCAATATATT GTGAGCGTGG CACATAACGG
351  CGGCTATAAC AACGTTGATT TTGGTGCGGA AGGAAGAAAT CCCGATCAAC
401  ATCGTTTTAC TTATAAAATT GTGAAACGGA ATAATTATAA AGCAGGGACT
451  AAAGGCCATC CTTATGGCGG CGATTATCAT ATGCCGCGTT TGCATAAATT
501  TGTCACAGAT GCAGAACCTG TTGAAATGAC CAGTTATATG GATGGGCGGA
```

```
551   AATATATCGA TCAAAATAAT TACCCTGACC GTGTTCGTAT TGGGGCAGGC
601   AGGCAATATT GGCGATCTGA TGAAGATGAG CCCAATAACC GCGAAAGTTC
651   ATATCATATT GCAAGTGCGT ATTCTTGGCT CGTTGGTGGC AATACCTTTG
701   CACAAAATGG ATCAGGTGGT GGCACAGTCA ACTTAGGTAG TGAAAAAATT
751   AAACATAGCC CATATGGTTT TTTACCAACA GGAGGCTCAT TTGGCGACAG
801   TGGCTCACCA ATGTTTATCT ATGATGCCCA AAAGCAAAAG TGGTTAATTA
851   ATGGGGTATT GCAAACGGGC AACCCCTATA TAGGAAAAAG CAATGGCTTC
901   CAGCTGGTTC GTAAAGATTG GTTCTATGAT GAAATCTTTG CTGGAGATAC
951   CCATTCAGTA TTCTACGAAC CACGTCAAAA TGGGAAATAC TCTTTTAACG
1001  ACGATAATAA TGGCACAGGA AAAATCAATG CCAAACATGA ACACAATTCT
1051  CTGCCTAATA GATTAAAAAC ACGAACCGTT CAATTGTTTA ATGTTTCTTT
1101  ATCCGAGACA GCAAGAGAAC CTGTTTATCA TGCTGCAGGT GGTGTCAACA
1151  GTTATCGACC CAGACTGAAT AATGGAGAAA ATATTTCCTT TATTGACGAA
1201  GGAAAAGGCG AATTGATACT TACCAGCAAC ATCAATCAAG GTGCTGGAGG
1251  ATTATATTTC CAAGGAGATT TTACGGTCTC GCCTGAAAAT AACGAAACTT
1301  GGCAAGGCGC GGGCGTTCAT ATCAGTGAAG ACAGTACCGT TACTTGGAAA
1351  GTAAACGGCG TGGCAAACGA CCGCCTGTCC AAAATCGGCA AAGGCACGCT
1401  GCACGTTCAA GCCAAAGGGG AAAACCAAGG CTCGATCAGC GTGGGCGACG
1451  GTACAGTCAT TTTGGATCAG CAGGCAGACG ATAAAGGCAA AAAACAAGCC
1501  TTTAGTGAAA TCGGCTTGGT CAGCGGCAGG GGTACGGTGC AACTGAATGC
1551  CGATAATCAG TTCAACCCCG ACAAACTCTA TTTCGGCTTT CGCGGCGGAC
1601  GTTTGGATTT AAACGGGCAT TCGCTTTCGT TCCACCGTAT TCAAAATACC
1651  GATGAAGGGG CGATGATTGT CAACCACAAT CAAGACAAAG AATCCACCGT
1701  TACCATTACA GGCAATAAAG ATATTGCTAC AACCGGCAAT AACAACAGCT
1751  TGGATAGCAA AAAAGAAATT GCCTACAACG GTTGGTTTGG CGAGAAAGAT
1801  ACGACCAAAA CGAACGGGCG GCTCAACCTT GTTTACCAGC CCGCCGCAGA
1851  AGACCGCACC CTGCTGCTTT CCGGCGGAAC AAATTTAAAC GGCAACATCA
1901  CGCAAACAAA CGGCAAACTG TTTTTCAGCG GCAGACCAAC ACCGCACGCC
1951  TACAATCATT TAAACGACCA TTGGTCGCAA AAAGAGGGCA TTCCTCGCGG
2001  GGAAATCGTG TGGGACAACG ACTGGATCAA CCGCACATTT AAAGCGGAAA
2051  ACTTCCAAAT TAAAGGCGGA CAGGCGGTGG TTTCCCGCAA TGTTGCCAAA
2101  GTGAAAGGCG ATTGGCATTT GAGCAATCAC GCCCAAGCAG TTTTTGGTGT
2151  CGCACCGCAT CAAAGCCACA CAATCTGTAC ACGTTCGGAC TGGACGGGTC
2201  TGACAAATTG TGTCGAAAAA ACCATTACCG ACGATAAAGT GATTGCTTCA
2251  TTGACTAAGA CCGACATCAG CGGCAATGTC GATCTTGCCG ATCACGCTCA
2301  TTTAAATCTC ACAGGGCTTG CCACACTCAA CGGCAATCTT AGTGCAAATG
2351  GCGATACACG TTATACAGTC AGCCACAACG CCACCCAAAA CGGCAACCTT
2401  AGCCTCGTGG GCAATGCCCA AGCAACATTT AATCAAGCCA CATTAAACGG
2451  CAACACATCG GCTTCGGGCA ATGCTTCATT TAATCTAAGC GACCACGCCG
2501  TACAAAACGG CAGTCTGACG CTTTCCGGCA ACGCTAAGGC AAACGTAAGC
2551  CATTCCGCAC TCAACGGTAA TGTCTCCCTA GCCGATAAGG CAGTATTCCA
2601  TTTTGAAAGC AGCCGCTTTA CCGGACAAAT CAGCGGCGGC AAGGATACGG
2651  CATTACACTT AAAAGACAGC GAATGGACGC TGCCGTCAGG CACGGAATTA
2701  GGCAATTTAA ACCTTGACAA CGCCACCATT ACACTCAATT CCGCCTATCG
2751  CCACGATGCG GCAGGGGCGC AAACCGGCAG TGCGACAGAT GCGCCGCGCC
2801  GCCGTTCGCG CCGTTCGCGC CGTTCCCTAT TATCCGTTAC ACCGCCAACT
2851  TCGGTAGAAT CCCGTTTCAA CACGCTGACG GTAAACGGCA AATTGAACGG
2901  TCAGGGAACA TTCCGCTTTA TGTCGGAACT CTTCGGCTAC CGCAGCGACA
2951  AATTGAAGCT GGCGGAAAGT TCCGAAGGCA CTTACACCTT GGCGGTCAAC
3001  AATACCGGCA ACGAACCTGC AAGCCTCGAA CAATTGACGG TAGTGGAAGG
3051  AAAAGACAAC AAACCGCTGT CCGAAAACCT TAATTTCACC CTGCAAAACG
3101  AACACGTCGA TGCCGGCGCG TGGCGTTACC AACTCATCCG CAAAGACGGC
3151  GAGTTCCGCC TGCATAATCC GGTCAAAGAA CAAGAGCTTT CCGACAAACT
3201  CGGCAAGGCA GAAGCCAAAA AACAGGCGGA AAAAGACAAC GCGCAAAGCC
3251  TTGACGCGCT GATTGCGGCC GGGCGCGATG CCGTCGAAAA GACAGAAAGC
3301  GTTGCCGAAC CGGCCCGGCA GGCAGGCGGG GAAAATGTCG GCATTATGCA
3351  GGCGGAGGAA GAGAAAAAAC GGGTGCAGGC GGATAAAGAC ACCGCCTTGG
3401  CGAAACAGCG CGAAGCGGAA ACCCGGCCGG CTACCACCGC CTTCCCCCGC
3451  GCCCGCCGCG CCCGCCGGGA TTTGCCGCAA CTGCAACCCC AACCGCAGCC
3501  CCAACCGCAG CGCGACCTGA TCAGCCGTTA TGCCAATAGC GGTTTGAGTG
3551  AATTTTCCGC CACGCTCAAC AGCGTTTTCG CCGTACAGGA CGAATTAGAC
3601  CGCGTATTTG CCGAAGACCG CCGCAACGCC GTTTGGACAA GCGGCATCCG
3651  GGACACCAAA CACTACCGTT CGCAAGATTT CCGCGCCTAC CGCCAACAAA
```

```
3701  CCGACCTGCG CCAAATCGGT ATGCAGAAAA ACCTCGGCAG CGGGCGCGTC
3751  GGCATCCTGT TTTCGCACAA CCGGACCGAA AACACCTTCG ACGACGGCAT
3801  CGGCAACTCG GCACGGCTTG CCCACGGCGC CGTTTTCGGG CAATACGGCA
3851  TCGACAGGTT CTACATCGGC ATCAGCGCGG GCGCGGGTTT TAGCAGCGGC
3901  AGCCTTTCAG ACGGCATCGG AGGCAAAATC CGCCGCCGCG TGCTGCATTA
3951  CGGCATTCAG GCACGATACC GCGCCGGTTT CGGCGGATTC GGCATCGAAC
4001  CGCACATCGG CGCAACGCGC TATTTCGTCC AAAAAGCGGA TTACCGCTAC
4051  GAAAACGTCA ATATCGCCAC CCCCGGCCTT GCATTCAACC GCTACCGCGC
4101  GGGCATTAAG GCAGATTATT CATTCAAACC GGCGCAACAC ATTTCCATCA
4151  CGCCTTATTT GAGCCTGTCC TATACCGATG CCGCTTCGGG CAAAGTCCGA
4201  ACACGCGTCA ATACCGCCGT ATTGGCTCAG GATTTCGGCA AAACCCGCAG
4251  TGCGGAATGG GGCGTAAACG CCGAAATCAA AGGTTTCACG CTGTCCCTCC
4301  ACGCTGCCGC CGCCAAAGGC CCGCAACTGG AAGCGCAACA CAGCGCGGGC
4351  ATCAAATTAG GCTACCGCTG GTAA
```

This corresponds to the amino acid sequence <SEQ ID 87; ORF orf1-1>:

**orf1-1.pep**

```
   1  MKTTDKRTTE THRKAPKTGR IRFSPAYLAI CLSFGILPQA WAGHTYFGIN
  51  YQYYRDFAEN KGKFAVGAKD IEVYNKKGEL VGKSMTKAPM IDFSVVSRNG
 101  VAALVGDQYI VSVAHNGGYN NVDFGAEGRN PDQHRFTYKI VKRNNYKAGT
 151  KGHPYGGDYH MPRLHKFVTD AEPVEMTSYM DGRKYIDQNN YPDRVRIGAG
 201  RQYWRSDEDE PNNRESSYHI ASAYSWLVGG NTFAQNGSGG GTVNLGSEKI
 251  KHSPYGFLPT GGSFGDSGSP MFIYDAQKQK WLINGVLQTG NPYIGKSNGF
 301  QLVRKDWFYD EIFAGDTHSV FYEPRQNGKY SFNDDNNGTG KINAKHEHNS
 351  LPNRLKTRTV QLFNVSLSET AREPVYHAAG GVNSYRPRLN NGENISFIDE
 401  GKGELILTSN INQGAGGLYF QGDFTVSPEN NETWQGAGVH ISEDSTVTWK
 451  VNGVANDRLS KIGKGTLHVQ AKGENQGSIS VGDGTVILDQ QADDKGKKQA
 501  FSEIGLVSGR GTVQLNADNQ FNPDKLYFGF RGGRLDLNGH SLSFHRIQNT
 551  DEGAMIVNHN QDKESTVTIT GNKDIATTGN NNSLDSKKEI AYNGWFGEKD
 601  TTKTNGRLNL VYQPAAEDRT LLLSGGTNLN GNITQTNGKL FFSGRPTPHA
 651  YNHLNDHWSQ KEGIPRGEIV WDNDWINRTF KAENFQIKGG QAVVSRNVAK
 701  VKGDWHLSNH AQAVFGVAPH QSHTICTRSD WTGLTNCVEK TITDDKVIAS
 751  LTKTDISGNV DLADHAHLNL TGLATLNGNL SANGDTRYTV SHNATQNGNL
 801  SLVGNAQATF NQATLNGNTS ASGNASFNLS DHAVQNGSLT LSGNAKANVS
 851  HSALNGNVSL ADKAVFHFES SRFTGQISGG KDTALHLKDS EWTLPSGTEL
 901  GNLNLDNATI TLNSAYRHDA AGAQTGSATD APRRRSRRSR RSLLSVTPPT
 951  SVESRFNTLT VNGKLNGQGT FRFMSELFGY RSDKLKLAES SEGTYTLAVN
1001  NTGNEPASLE QLTVVEGKDN KPLSENLNFT LQNEHVDAGA WRYQLIRKDG
1051  EFRLHNPVKE QELSDKLGKA EAKKQAEKDN AQSLDALIAA GRDAVEKTES
1101  VAEPARQAGG ENVGIMQAEE EKKRVQADKD TALAKQREAE TRPATTAFPR
1151  ARRARRDLPQ LQPQPQPQPQ RDLISRYANS GLSEFSATLN SVFAVQDELD
1201  RVFAEDRRNA VWTSGIRDTK HYRSQDFRAY RQQTDLRQIG MQKNLGSGRV
1251  GILFSHNRTE NTFDDGIGNS ARLAHGAVFG QYGIDRFYIG ISAGAGFSSG
1301  SLSDGIGGKI RRRVLHYGIQ ARYRAGFGGF GIEPHIGATR YFVQKADYRY
1351  ENVNIATPGL AFNRYRAGIK ADYSFKPAQH ISITPYLSLS YTDAASGKVR
1401  TRVNTAVLAQ DFGKTRSAEW GVNAEIKGFT LSLHAAAAKG PQLEAQHSAG
1451  IKLGYRW*
```

The following partial DNA sequence was identified in *N. meningitidis* <SEQ ID 88>:

**orf46-2.seq**

```
   1  TTGGGCATTT CCCGCAAAAT ATCCCTTATT CTGTCCATAC TGGCAGTGTG
  51  CCTGCCGATG CATGCACACG CCTCAGATTT GGCAAACGAT TCTTTTATCC
 101  GGCAGGTTCT CGACCGTCAG CATTTCGAAC CCGACGGGAA ATACCACCTA
 151  TTCGGCAGCA GGGGGGAACT TGCCGAGCGC AGCGGCCATA TCGGATTGGG
 201  AAAAATACAA AGCCATCAGT TGGGCAACCT GATGATTCAA CAGGCGGCCA
 251  TTAAAGGAAA TATCGGCTAC ATTGTCCGCT TTTCCGATCA CGGGCACGAA
 301  GTCCATTCCC CCTTCGACAA CCATGCCTCA CATTCCGATT CTGATGAAGC
 351  CGGTAGTCCC GTTGACGGAT TTAGCCTTTA CCGCATCCAT TGGGACGGAT
```

```
 401  ACGAACACCA TCCCGCCGAC GGCTATGACG GGCCACAGGG CGGCGGCTAT
 451  CCCGCTCCCA AAGGCGCGAG GGATATATAC AGCTACGACA TAAAAGGCGT
 501  TGCCCAAAAT ATCCGCCTCA ACCTGACCGA CAACCGCAGC ACCGGACAAC
 551  GGCTTGCCGA CCGTTTCCAC AATGCCGGTA GTATGCTGAC GCAAGGAGTA
 601  GGCGACGGAT TCAAACGCGC CACCCGATAC AGCCCCGAGC TGGACAGATC
 651  GGGCAATGCC GCCGAAGCCT TCAACGGCAC TGCAGATATC GTTAAAAACA
 701  TCATCGGCGC GGCAGGAGAA ATTGTCGGCG CAGGCGATGC CGTGCAGGGC
 751  ATAAGCGAAG GCTCAAACAT TGCTGTCATG CACGGCTTGG GTCTGCTTTC
 801  CACCGAAAAC AAGATGGCGC GCATCAACGA TTTGGCAGAT ATGGCGCAAC
 851  TCAAAGACTA TGCCGCAGCA GCCATCCGCG ATTGGGCAGT CCAAAACCCC
 901  AATGCCGCAC AAGGCATAGA AGCCGTCAGC AATATCTTTA TGGCAGCCAT
 951  CCCCATCAAA GGGATTGGAG CTGTTCGGGG AAAATACGGC TTGGGCGGCA
1001  TCACGGCACA TCCTATCAAG CGGTCGCAGA TGGGCGCGAT CGCATTGCCG
1051  AAAGGGAAAT CCGCCGTCAG CGACAATTTT GCCGATGCGG CATACGCCAA
1101  ATACCCGTCC CCTTACCATT CCCGAAATAT CCGTTCAAAC TTGGAGCAGC
1151  GTTACGGCAA AGAAAACATC ACCTCCTCAA CCGTGCCGCC GTCAAACGGC
1201  AAAAATGTCA AACTGGCAGA CCAACGCCAC CCGAAGACAG GCGTACCGTT
1251  TGACGGTAAA GGGTTTCCGA ATTTTGAGAA GCACGTGAAA TATGATACGA
1301  AGCTCGATAT TCAAGAATTA TCGGGGGGCG GTATACCTAA GGCTAAGCCT
1351  GTGTTTGATG CGAAACCGAG ATGGGAGGTT GATAGGAAGC TTAATAAATT
1401  GACAACTCGT GAGCAGGTGG AGAAAAATGT TCAGGAAATA AGGAACGGTA
1451  ATATAAACAG TAACTTTAGC CAACATGCTC AACTAGAGAG GGAAATTAAT
1501  AAACTAAAAT CTGCCGATGA AATTAATTTT GCAGATGGAA TGGGAAAATT
1551  TACCGATAGC ATGAATGACA AGGCTTTTAG TAGGCTTGTG AAATCAGTTA
1601  AAGAGAATGG CTTCACAAAT CCAGTTGTGG AGTACGTTGA AATAAATGGA
1651  AAAGCATATA TCGTAAGAGG AAATAATRGG GTTTTTGCTG CAGAATACCT
1701  TGGCAGGATA CATGAATTAA AATTTAAAAA AGTTGACTTT CCTGTTCCTA
1751  ATACTAGTTG GAAAAATCCT ACTGATGTCT TGAATGAATC AGGTAATGTT
1801  AAGAGACCTC GTTATAGGAG TAAATAA
```

This corresponds to the amino acid sequence <SEQ ID 89; ORF orf46-2>:

**orf46-2.pep**

```
  1  LGISRKISLI LSILAVCLPM HAHASDLAND SFIRQVLDRQ HFEPDGKYHL
 51  FGSRGELAER SGHIGLGKIQ SHQLGNLMIQ QAAIKGNIGY IVRFSDHGHE
101  VHSPFDNHAS HSDSDEAGSP VDGFSLYRIH WDGYEHHPAD GYDGPQGGGY
151  PAPKGARDIY SYDIKGVAQN IRLNLTDNRS TGQRLADRFH NAGSMLTQGV
201  GDGFKRATRY SPELDRSGNA AEAFNGTADI VKNIIGAAGE IVGAGDAVQG
251  ISEGSNIAVM HGLGLLSTEN KMARINDLAD MAQLKDYAAA AIRDWAVQNP
301  NAAQGIEAVS NIFMAAIPIK GIGAVRGKYG LGGITAHPIK RSQMGAIALP
351  KGKSAVSDNF ADAAYAKYPS PYHSRNIRSN LEQRYGKENI TSSTVPPSNG
401  KNVKLADQRH PKTGVPFDGK GFPNFEKHVK YDTKLDIQEL SGGGIPKAKP
451  VFDAKPRWEV DRKLNKLTTR EQVEKNVQEI RNGNINSNFS QHAQLEREIN
501  KLKSADEINF ADGMGKFTDS MNDKAFSRLV KSVKENGFTN PVVEYVEING
551  KAYIVRGNNR VFAAEYLGRI HELKFKKVDF PVPNTSWKNP TDVLNESGNV
601  KRPRYRSK*
```

Using the above-described procedures, the following oligonucleotide primers were employed in the polymerase chain reaction (PCR) assay in order to clone the ORFs as indicated:

**Oligonucleotides used for PCR**

Table 1

| ORF | Primer | Sequence | Restriction sites |
|---|---|---|---|
| 279 | Forward | CGCGGATCCCATATG-TTGCCTGCAATCACGATT <SEQ ID 90> | BamHI-NdeI |
| | Reverse | CCCGCTCGAG-TTTAGAAGCGGGCGGCAA <SEQ ID 91> | XhoI |
| 519 | Forward | CGCGGATCCCATATG-TTCAAATCCTTTGTCGTCA <SEQ ID 92> | BamHI-NdeI |
| | Reverse | CCCGCTCGAG-TTTGGCGGTTTTGCTGC <SEQ ID 93> | XhoI |
| 576 | Forward | CGCGGATCCCATATG-GCCGCCCCCGCATCT <SEQ ID 94> | BamHI-NdeI |
| | Reverse | CCCGCTCGAG-ATTTACTTTTTTGATGTCGAC <SEQ ID 95> | XhoI |
| 919 | Forward | CGCGGATCCCATATG-TGCCAAAGCAAGAGCATC <SEQ ID 96> | BamHI-NdeI |
| | Reverse | CCCGCTCGAG-CGGGCGGTATTCGGG <SEQ ID 97> | XhoI |
| 121 | Forward | CGCGGATCCCATATG-GAAACACAGCTTTACAT <SEQ ID 98> | BamHI-NdeI |
| | Reverse | CCCGCTCGAG-ATAATAATATCCCGCGCCC <SEQ ID 99> | XhoI |
| 128 | Forward | CGCGGATCCCATATG-ACTGACAACGCACT <SEQ ID 100> | BamHI-NdeI |
| | Reverse | CCCGCTCGAG-GACCGCGTTGTCGAAA <SEQ ID 101> | XhoI |
| 206 | Forward | CGCGGATCCCATATG-AAACACCGCCAACCGA <SEQ ID 102> | BamHI-NdeI |
| | Reverse | CCCGCTCGAG-TTCTGTAAAAAAAGTATGTGC <SEQ ID 103> | XhoI |
| 287 | Forward | CCGGAATTCTAGCTAGC-CTTTCAGCCTGCGGG <SEQ ID 104> | EcoRI-NheI |
| | Reverse | CCCGCTCGAG-ATCCTGCTCTTTTTTGCC <SEQ ID 105> | XhoI |
| 406 | Forward | CGCGGATCCCATATG-TGCGGGACACTGACAG <SEQ ID 106> | BamHI-NdeI |
| | Reverse | CCCGCTCGAG-AGGTTGTCCTTGTCTATG <SEQ ID 107> | XhoI |

EXAMPLE 2

Expression of ORF 919

The primer described in Table 1 for ORF 919 was used to locate and clone ORF 919. The predicted gene 919 was cloned in pET vector and expressed in *E. coli*. The product of protein expression and purification was analyzed by SDS-PAGE. In panel A) is shown the analysis of 919-His fusion protein purification. Mice were immunized with the purified 919-His and sera were used for Western blot (panel B), FACS analysis (panel C), bactericidal assay (panel D), and ELISA assay (panel E). Symbols: M1, molecular weight marker; PP, purified protein, TP, *N. meningitidis* total protein extract; OMV, *N. meningitidis* outer membrane vesicle preparation. Arrows indicate the position of the main recombinant protein product (A) and the *N. meningitidis* immunoreactive band (B). These experiments confirm that 919 is a surface-exposed protein and that it is a useful immunogen. The hydrophilicity plots, antigenic index, and amphipatic regions of ORF 919 are provided in Figure 10. The AMPHI program is used to predict putative T-cell epitopes (Gao et al 1989, *J. Immunol* 143:3007; Roberts et al. 1996, *AIDS Res Human Retroviruses* 12:593; Quakyi et al. 1992, *Scand J Immunol Suppl* 11:9). The nucleic acid sequence of ORF 919 and the amino acid sequence encoded thereby is provided in Example 1.

EXAMPLE 3

Expression of ORF 279

The primer described in Table 1 for ORF 279 was used to locate and clone ORF 279. The predicted gene *279* was cloned in pGex vector and expressed in *E. coli*. The product of protein expression and purification was analyzed by SDS-PAGE. In panel A) is shown the analysis of 279-GST purification. Mice were immunized with the purified 279-GST and sera were used for Western blot analysis (panel B), FACS analysis (panel C), bactericidal essay (panel D), and ELISA assay (panel E). Symbols: M1, molecular weight marker; TP, *N. meningitidis* total protein extract; OMV, *N. meningitidis* outer membrane vescicle preparation. Arrows indicate the position of the main recombinant protein product (A) and the *N. meningitidis* immunoreactive band (B). These experiments confirm that 279 is a surface-exposed protein and that it is a useful immunogen. The hydrophilicity plots, antigenic index, and amphipatic regions of ORF 279 are provided in Figure 11. The AMPHI program is used to predict putative T-cell epitopes (Gao et al 1989, *J. Immunol* 143:3007; Roberts et al. 1996, *AIDS Res Human Retroviruses* 12:593; Quakyi et al. 1992, *Scand J Immunol Suppl* 11: 9). The nucleic acid sequence of ORF 279 and the amino acid sequence encoded thereby is provided in Example 1.

EXAMPLE 4

Expression of ORF 576

The primer described in Table 1 for ORF 576 was used to locate and clone ORF 576. The predicted gene *576* was cloned in pGex vector and expressed in *E. coli*. The product of protein purification was analyzed by SDS-PAGE. In panel A) is shown the analysis of 576-GST fusion protein purification. Mice were immunized with the purified 576-GST and sera were used for Western blot (panel B), FACS analysis (panel C), bactericidal assay (panel D), and ELISA assay (panel E). Symbols: M1, molecular weight marker; TP, *N. meningitidis* total protein extract; OMV, *N. meningitidis* outer membrane vescicle preparation. Arrows indicate the position of the main recombinant protein product (A) and the *N. meningitidis* immunoreactive band (B).. These experiments confirm that ORF 576 is a surface-exposed protein and that it is a useful immunogen. The hydrophilicity plots, antigenic index, and amphipatic regions of ORF 576 are provided in Figure 12. The AMPHI program is used to predict putative T-cell epitopes (Gao et al 1989, *J. Immunol 143: 3007;* Roberts et al. 1996, *AIDS Res Human Retroviruses* 12:593; Quakyi et al. 1992, *Scand J Immunol Suppl* 11:9). The nucleic acid sequence of ORF 576 and the amino acid sequence encoded thereby is provided in Example 1.

EXAMPLE 5

Expression of ORF 519

The primer described in Table 1 for ORF 519 was used to locate and clone ORF 519. The predicted gene *519* was cloned in pET vector and expressed in *E. coli*. The product of protein purification was analyzed by SDS-PAGE. In panel A) is shown the analysis of 519-His fusion protein purification. Mice were immunized with the purified 519-His and sera were used for Western blot (panel B), FACS analysis (panel C), bactericidal assay (panel D), and ELISA assay (panel E). Symbols: M1, molecular weight marker; TP, *N. meningitidis* total protein extract; OMV, *N. meningitidis* outer membrane vesicle preparation. Arrows indicate the position of the main recombinant protein product (A) and the *N. meningitidis* immunoreactive band (B). These experiments confirm that 519 is a surface-exposed protein and that it is a useful immunogen. The hydrophilicity plots, antigenic index, and amphipatic regions of ORF 519 are provided in Figure 13. The AMPHI program is used to predict putative T-cell epitopes (Gao et al 1989, *J. Immunol* 143:3007; Roberts et al. 1996, *AIDS Res Human Retroviruses* 12:593; Quakyi et al. 1992, *Scand J Immunol Suppl* 11:9). The nucleic acid sequence of ORF 519 and the amino acid sequence encoded thereby is provided in Example 1.

EXAMPLE 6

Expression of ORF 121

The primer described in Table 1 for ORF 121 was used to locate and clone ORF 121. The predicted gene 121 was cloned in pET vector and expressed in *E. coli*. The product of protein purification was analyzed by SDS-PAGE. In panel A) is shown the analysis of 121-His fusion protein purification. Mice were immunized with the purified 121-His and sera were used for Western blot analysis (panel B), FACS analysis (panel C), bactericidal assay (panel D), and ELISA assay (panel E). Results show that 121 is a surface-exposed protein. Symbols: M1, molecular weight marker; TP, *N. meningitidis* total protein extract; OMV, *N. meningitidis* outer membrane vescicle preparation. Arrows indicate the position of the main recombinant protein product (A) and the *N. meningitidis* immunoreactive band (B). These experiments confirm that 121 is a surface-exposed protein and that it is a useful immunogen. The hydrophilicity plots, antigenic index, and amphipatic regions of ORF 121 are provided in Figure 14. The AMPHI program is used to predict putative T-cell epitopes (Gao et al 1989, *J. Immunol 143:3007;* Roberts et al. 1996, *AIDS Res Human Retroviruses* 12:593; Quakyi et al. *1992, Scand J Immunol Suppl 11:9).* The nucleic acid sequence of ORF 121 and the amino acid sequence encoded thereby is provided in Example 1.

EXAMPLE 7

Expression of ORF 128

The primer described in Table 1 for ORF 128 was used to locate and clone ORF 128. The predicted gene *128* was cloned in pET vector and expressed in *E. coli*. The product of protein purification was analyzed by SDS-PAGE. In panel A) is shown the analysis of 128-His purification. Mice were immunized with the purified 128-His and sera were used for Western blot analysis (panel B), FACS analysis (panel C), bactericidal assay (panel D) and ELISA assay (panel E). Results show that 128 is a surface-exposed protein. Symbols: M1, molecular weight marker; TP, *N. meningitidis* total protein extract; OMV, *N. meningitidis* outer membrane vesicle preparation. Arrows indicate the position of the main recombinant protein product (A) and the *N. meningitidis* immunoreactive band (B). These experiments confirm that 128 is a surface-exposed protein and that it is a useful immunogen. The hydrophilicity plots, antigenic index, and amphipatic regions of ORF 128 are provided in Figure 15. The AMPHI program is used to predict putative T-cell epitopes (Gao et al 1989, *J. Immunol* 143:3007; Roberts et al. 1996, *AIDS Res Human Retroviruses* 12:593; Quakyi et al. 1992, *Scand J Immunol Suppl* 11:9). The nucleic acid sequence of ORF 128 and the amino acid sequence encoded thereby is provided in Example 1.

EXAMPLE 8

Expression of ORF 206

The primer described in Table 1 for ORF 206 was used to locate and clone ORF 206. The predicted gene *206* was cloned in pET vector and expressed in *E. coli*. The product of protein purification was analyzed by SDS-PAGE. In panel A) is shown the analysis of 206-His purification. Mice were immunized with the purified 206-His and sera were used for Western blot analysis (panel B). It is worthnoting that the immunoreactive band in protein extracts from meningococcus is 38 kDa instead of 17 kDa (panel A). To gain information on the nature of this antibody staining we expressed ORF 206 in *E. coli* without the His-tag and including the predicted leader peptide. Western blot analysis on total protein extracts from *E. coli* expressing this native form of the 206 protein showed a recative band at a position of 38 kDa, as observed in meningococcus. We conclude that the 38 kDa band in panel B) is specific and that anti-206 antibodies, likely recognize a multimeric protein complex. In panel C is shown the FACS analysis, in panel D the bactericidal assay, and in panel E) the ELISA assay. Results show that 206 is a surface-exposed protein. Symbols: M1, molecular weight marker; TP, *N. meningitidis* total protein extract; OMV, *N. meningitidis* outer membrane vesicle preparation. Arrows indicate the position of the main recombinant protein product (A) and the *N. meningitidis* immunoreactive band (B). These experiments confirm that 206 is a surface-exposed protein and that it is a useful immunogen. The hydrophilicity plots, antigenic index, and amphipatic regions of ORF 519 are provided in Figure 16. The AMPHI program is used to predict putative T-cell epitopes (Gao et al 1989, *J. Immunol* 143:3007; Roberts et al. 1996, *AIDS Res Human Retroviruses* 12:593; Quakyi et al. 1992, *Scand J Immunol Suppl* 11:9). The nucleic acid sequence of ORF 206 and the amino acid sequence encoded thereby is provided in Example 1.

EXAMPLE 9

Expression of ORF 287

The primer described in Table 1 for ORF 287 was used to locate and clone ORF 287. The predicted gene 287 was cloned in pGex vector and expressed in *E. coli.* The product of protein purification was analyzed by SDS-PAGE. In panel A) is shown the analysis of 287-GST fusion protein purification. Mice were immunized with the purified 287-GST and sera were used for FACS analysis (panel B), bactericidal assay (panel C), and ELISA assay (panel D). Results show that 287 is a surface-exposed protein. Symbols: M1, molecular weight marker. Arrow indicates the position of the main recombinant protein product (A). These experiments confirm that 287 is a surface-exposed protein and that it is a useful immunogen. The hydrophilicity plots, antigenic index, and amphipatic regions of ORF 287 are provided in Figure 17. The AMPHI program is used to predict putative T-cell epitopes (Gao et al 1989, *J. Immunol* 143:3007; Roberts et al. 1996, *AIDS Res Human Retroviruses* 12:593; Quakyi et al. 1992, *Scand J Immunol Suppl* 11:9). The nucleic acid sequence of ORF 287 and the amino acid sequence encoded thereby is provided in Example 1.

EXAMPLE 10

Expression of ORF 406

The primer described in Table 1 for ORF 406 was used to locate and clone ORF 406. The predicted gene 406 was cloned in pET vector and expressed in *E. coli*. The product of protein purification was analyzed by SDS-PAGE. In panel A) is shown the analysis of 406-His fusion protein purification. Mice were immunized with the purified 406-His and sera were used for Western blot analysis (panel B), FACS analysis (panel C), bactericidal assay (panel D), and ELISA assay (panel E). Results show that 406 is a surface-exposed protein. Symbols: M1, molecular weight marker; TP, *N. meningitidis* total protein extract; OMV, *N. meningitidis* outer membrane vescicle preparation. Arrows indicate the position of the main recombinant protein product (A) and the *N. meningitidis* immunoreactive band (B). These experiments confirm that 406 is a surface-exposed protein and that it is a useful immunogen. The hydrophilicity plots, antigenic index, and amphipatic regions of ORF 406 are provided in Figure 18. The AMPHI program is used to predict putative T-cell epitopes (Gao et al 1989, *J. Immunol 143:3007;* Roberts et al. 1996, *AIDS Res Human Retroviruses* 12:593; Quakyi et al. 1992, *Scand J Immunol Suppl* 11:9). The nucleic acid sequence of ORF 406 and the amino acid sequence encoded thereby is provided in Example 1.

The foregoing examples are intended to illustrate but not to limit the invention.

Appendix A

# APPENDIX A

The following DNA sequence was identified in *N. meningitidis B* <SEQ ID NO. 1>:

TAAACCTTATCCACATCCAAACGCATAACCGTAACCCATTCACCGTTATGGAAATGTCGC
CCGACAACCACCCAGCCGAATGATTCATAAAATATTTGCACATCAGGCGTATAAAGATAC
AAGAACTTTATCCCCAGCGAACGCGCTGCGCCTATGCAGTGGGCGACCAGCCTCCTGCCA
ATGCCTTTTCCGCGATATTCAGGTAAAACAAAGACATCCCCCAACCAATATTCATACCGT
GGAAAACTTTCCATATCATGCCGCTTGACCGCAGCCGAACCCAACAGGATTCCGGAATCA
TCCACAGCCGCAAATGCCAGCGGCAGTTCGTCATCCTTCAAACACCTGCCGTAATAGGCA
TGAATCTTATCCACAGAAGACCACGGTTCAAATCCGTGCCACTCCTCAAACAACGCCTGA
ACCAACCTGCCGATATGCCCGGCTTTCAGCCGTGTAATGAAAACAGTATTGTCCACAAAG
AGGGAATTCATCGGTCAATTCCCCGACGCCTTCGTTCCCCCTGCGCCGTAAACCGCATTC
CAAGCATGGTCCAAACGCACTCCGATTTGCCTCAAATCTTCAGCCTGCCGGGCTTTTTGC
GCCATTGCTGCAGGAATTTCCGCTTCCAAACGGGCGATGTCTGCCTGAGCCGTCTGCAAA
CGCCGGCGCGCATCTTCCAAATCCGACTGCATCCCGATGATTTTTCCGTCCAGATTGTTT
TGCTTTTGCAATAAGGCGCGGTAACCGGATTGGATGCTGAGCAGATTGTCTTCAGCATCC
CCTGCCCATACGCTTGTAGAAAAAACAACCATCAGAAAATAAAATATTTTTTTCATTTTT
AACTTCCATTTAAATGCTGTCTGAAGCCGTATTCCGACATCAGACGGCATCGCCCACGCC
TGTGGATAACTTAAGCGCGGATGCGTTTCAACACTTCTTCTTTGCCGATTAATGCCAACA
CAGCATCGACGCTGGGGGTTTTCGCCGTACCGCAGACGGCAAGGCGCAGGGGCATGCCGA
GTTTGCCCATTTTAATGCCTTCTTCGTCGCAGAAGGGTTTGAAGAGGTCGTGGATGGCTT
CGGCATTCCAGTCTTCCAGCCCTTCGAGGCGTTCGGCAAAGCGCAGCATACGGGCGGCGG
CTTCATCGTCCCAGTGTTTCTGCACGTCTGCTTCGGCAGGCGTTTGTTTGACGTAGAAGT
AGAAGCACTCGTCGGCAAGCGTGTTCAAGTCTTGGGGGCGGTCTTTGACCAGTTCCAACA
CATCTTCCAAAGCAGGTTTTTCGGTTTCATGAATATCGCGCAACGCAAGGCGGGGTTTGA
CGAGTTCGGCGAGTTTGCCGTTGGGTGTGATTTTGATGTGTTCGCCGTTGATCCAGTAGA
GTTTTTTCAAGTCCATACGGCTTGGAGACGGGGAAACGTCTTTCAAATCAAACCATTCGA
TGAATTGTTCCATTGTGAAGAATTCATCGTCGCCGTGCGCCCAGCCCAAGCGTGCCAGAT
AGTTGAGCATCGCTTCGGGCAGGATGCCCATTGCGCCGAAATCGGTAATGGCAACGGTAT
CGCCGCTGCGTTTGGAGATTTTTTTGCCTTGTTCGTTAAGAATCATCGGCAGGTGGCCGT
ATTCGGGCAGGTTCGCGTCGATGGCTTTTAAGATGTTGATTTGTTTCGGCGTGTTGTTCA
CATGGTCGTCGCCGCGGATAACGTGGGTAACGCCCATGTCGTAGTCGTCTACGACAACGC
AGAAGTTGTAGGTCGGCGTACCGTCGGCGCGGGCGATAATCAGGTCATCGAGTGCTTCGT
TGGGGATGGAGATTTCGCCTTTGACCAAGTCTGTCCATTTGGTCACACCGTCCAAAGGCG
TTTTGAAACGGACAACGGGTTGTACGTCGGACGGGATTTCGGGCAGGGTTTTACCTACTT
CCGGACGCCAGCGGCGGTCGTAAGTCGCCGAGCCTTCTTTTTCGGCTTTCTCACGCATGG
CTTCCAGCTCTTCTTTGCTGCAATAGCAGTAGTAGGCATGGCCTTTTTCTAAAAGTTCGG
CAATGACCTCTTTGTAGCGGTCGAAACGGCGAGTTTGGTAAACGACGTTGTCGGCGTTGT
CGTAATTGAGACCGACCCATTTCATGCCGTCGAGGATGATGTTGACGGATTCGGCGGTAG
AACGCGCCAAGTCGGTGTCTTCAATACGTAATAGGAACTCGCCTTTATGATGGCGGGCAA
ACGCCCATGAAAACAAGGCGGTGCGCACGCCGCCGATGTGCAGGTAGCCGGTGGGGCTGG
GGGCGAAACGGGTTTTGACGGTCATGATGGCTCCGAAATCTTTGAAAGCGTTTATTTTAC
TGGTTTTACCGTGCTTGGGCATCAAAAATGCCGTCTGAACCCTGCCTGCGGATAAAGTTT
CAGACGGCATTTTCCTTGTTTTCAATGCTTCGGCACGCGGAACAGTGTATCACGCGCCGC
CGACCGAATTCCTTCGGGATTGCGTCCAAAAAAAAGTTCAATGAAACAGCTAATTGAAAA
AATCCCGCCCCCATTTTTCCAAACGGTAGAGGGATAACGCATATCCCTCTTGCAGCATAA
AGATTTTTTTCTTATTTCCCGCATCAAACCGCGTGGTCGGCGTGGCAGACATATAAACGC
GGACACCCAAATCCTCCGCCATTTCCGCCGCCCGCGCCAAATGGTAGGGATCGCTGACAA
TCACCACGCTGGCAATACCGTTGGCACGCAAAACCGGACGGATGTTGTTCAGGTTTTCAT
AAGTGTTGCGCGAAGTGTTTTCAAACAGGATGTTGCGCGCCGGAACCCCCTGTTTGAGTG
CGTACCGCCGCCCGACCTCGGCTTCGGTCATATAGCCTTTTTTGGTCCGGCCTCCCGTAA
ACACGATTTTGCCTACCCTGCGGCTCTGATAAAGTGCGATGGCATGGTTGATGCGTTCGC
GGAAAACAGGAGAAGGGCGTTTGTCCCACGCGGCGGCGCCCAACACCAGCGCGGCATCCG
CCCGGACATACGGCGGCAAAACCTGCCCACCCGTCCGATAAACCGCCCAAACGGATGAGG
CAAACACCAGCAAAAGCGGAAAAACACTCAAACAGAAACCGCCCAACAGGTAATAGCGCA
AGCCGTTGCGGCTGCAAAACAGCCGTTTGTTCACAATACCGCTTCGATATTTTCCAGCGG
TCTGCCGACAGCCGCCTTACCGTTTGCCAAAACAATCGGACGCTCCAACAGGGCGGGATG
ATCGGCGATGGCACGCAGCAGCGCGTCATTGTCCAAATTGGGGTTGTCCAAACCCAATTC
CTTATACAAATCATCTTTCACGCGCATCATCCCGCGCGCCGATGCCAAGCCCAATTTGTT
GAAAATATCCTTCAATTCGGACAAGTCGGGCGGCGTATCCAAATATTTGACCACTTCGGC
AGCAATGCCGCGTTCTTCCAATAGGGACAAGGCGGCACGCGATTTGCTGCAACGCGGATT
GTGGAAAATTTTGATTTCAGGCATGACATTTCCTTGCTTCTCGACAATCCCCTTATTATC
GGCTTACACAGGGTTTTACTCAATATCCCGCCTACAACCGTACCAAACGGTTTACAATAC
CCGAATCGACATACAAAGGACAAAACGATGAAATACTTGAATCTTGCCGCAATCACCCTT
GCCGCCACATTTGCCGCACATACCGCCTCGGCAGACGAACTGGCCGGATGGAAAGACAAC
ACCCCGCAAAGCCTGCAATCGCTCAAAGCCCCCGTACGCATCGTCAACCTTTGGGCGACT
TGGTGCGGCCCGTGCCGAAAAGAGATGCCTGCCATGTCCAAATGGTACAAAGCGCAGAAA
AAAGGCAGCGTCGATATGGTCGGCATCGCGCTCGACACATCCGACAATATCGGCAACTTC

## Appendix A

CTCAAACAAACTCCTGTTTCCTACCCGATTTGGCGTTACACCGGGGCGAACAGCCGAAAC
TTTATGAAAACCTACGGAAACACTGTCGGCGTACTGCCCTTTACCGTCGTCGAAGCACCG
AAATGCGGATACAGGCAGACCATTACCGGGGAGGTAAACGAAAAAAGCCTGACCGACGCC
GTCAAACTCGCCCATTCAAAATGCCGTTAAACGCCGGATGCCGTCTGAAGCCGCTTCAGA
TGGCATTTTTCTTTTCCACCCGCCTGCCGGTGCAAACTTATCCACTATCTAAAAACAGGC
GGAATCTTTATAATCGGCACTGTCTTACCTATTGTTCAGACGGCATATCCCTGCGGACGC
AACCGCCCGAAACGATATGCCGCCCTTCCTTACAGGACCTCCTATGATCCGTTTCGAACA
AGTTTCCAAAACCTATCCCGGCGGTTTTGAAGCCCTGAAAAACGTCAGCTTCCAAATCAA
CAAAGGCGAAATGATATTTATCGCGGACACTCCGGTTCGGGCAAATCCACCATCCTCAA
ACTGATTTCGGGCATTACCAAGCCGAGCAGGGGCAAAATCCTGTTTAACGGGCAGGACCT
CGGCACATTGTCCGACAACCAAATCGGCTTTATGCGCCAACACATCGGCATCGTGTTCCA
AGACCACAAAATCCTCTACGACCGCAACGTCCTGCAAAACGTCATCCTGCCGCTTCGGAT
TATCGGCTATCCGCCGCGCAAAGCCGAAGAGCGTGCCCGCATCGCCATCGAAAAAGTCGG
CCTGAAAGGACGAGAATTGGACGATCCCGTAACCCTCTCCGGCGGTGAACAACAACGCCT
GTGCATCGCCCGCGCCGTCGTTCACCAGCCCGGCCTGCTGATTGCCGACGAACCCTCCGC
CAACCTCGACCGCGCCTACGCGCTCGATATTATGGAATTGTTCAAAACCTTCCACGAAGC
GGGAACTACCGTCATCGTTGCCGCACATGACGAAACCCTGATGGCGGACTACGGACACCG
CATCCTGCGCCTCTCGAAAGGACGACTCGCATGAGCATCATCCACTACCTCTCGCTGCAC
GTCGAATCCGCGCGCACCGCGCTCAAGCAGCTCCTGCGCCAACCCTTCGGCACACTGCTT
ACCCTCATGATGCTCGCCGTCGCGATGACCCTGCCGCTGTTTATGCATCTGGGCATCCAA
AGCGGGCAAAGCGTGTTGGGCAAACTCAACGAGTCGCCGCAAATCACAATCTATATGGAA
ACCTCCGCCGCACAAAGCGACAGCGATACCGTCCGCAGCCTGCTGGCGCGCGACAAACGG
CTCGACAACATCCGCTTCATCGGCAAAGAAGACGGTCTGGAAGAATTACAGTCCAATCTT
GACCAAAATCTGATTTCCATGCTTGACGGCAACCCCCTGCCGGATGTCTTTATCGTTACC
CCCGACCCGGCAACCACGCCCGCCCAAATGCAGGCAATCTACCGAGACATTACCAAACTG
CCTATGGTCGAATCCGCGTCTATGGATACCGAATGGGTGCAAACGCTGTACCAAATCAAC
GAGTTCATCCGCAAAATTTTGTGGTTTCTTTCCCTGACGCTGGGGATGGCGTTCGTCCTT
GTCGCACACAACACCATCCGCCTGCAAATCCTCAGCCGCAAAGAAGAAATCGAAATCACC
AAACTCTTGGGCGCGCCCGCGTCGTTTATCCGCCGCCCATTCCTTTATCAAGCCATGTGG
CAGAGCATCCTTTCCGCCGCCGTCAGCTTGGGGCTTTGCGGTTGGCTGCTCTCTGCCGTG
CGCCCATTGGTCGATGCCATTTTCAAACCCTACGGACTTAATATCGGCTGGCGGTTCTTC
TACGCTGGCGAACTCGGGCTGGTGTTCGGCTTCGTCATCGCGTTGGGCGTATTCGGCGCG
TGGCTTGCCACCACCCAGCACCTGCTCGGCTTCAAAGCCAAAAAATAAAACACCGTCAAA
AATGCCGTCCGAACCCGTTTTCAGACGGCATTTCAATTTGCCAGTATAATGGCGCATTTT
TCCAACAAGGAACCTACCATGCTGACCTCGGAACAAGTAAAAGCCATGATTGAAGGCGTG
GCAAAATGCGAACATATCGAAGTAGAAGGCGACGGACACCATTTTTTCGCCGTCATCGTT
TCATCAGAATTTGAAGGCAAGGCACGCCTCGCGCGCCACCGCCTGATTAAAGACGGACTC
AAAGCCCAACTGGAAAGTAACGAACTGCACGCACTTTCCATTTCGGTTGCCGCCACTCCG
GCGGAATGGGCAGCCAAAGCACAATAATCGCCACACAAAAATGCCGTCTGAAACCATTTC
GTTTCAGACGGCATTTTTTTATATCAAACCGCTTACGCGCCGCGTTTTTCCAAAGCGGC
TACGGCAGGCAGCTCTTTGCCTTCCAAGAACTCAAGGAACGCGCCGCCGCCGGTGGAGAT
GTAGCCGATTTGTTCGGTAACGCCGAATTTGGCAATCGCCGCCAGCGTGTCGCCGCCGCC
CGCAATCGAGAACGCTTTGCTTTGGGCAATGGCTTCGGCAAGGGCTTTCGTACCGCCTGC
GAATTGGTCAAACTCGAACACGCCGACCGGCCCGTTCCAAACGACCGTACCGGCGGCTTT
AAGCAAATCGGCAAGCGCGGCAGCGGATTTCGGACCGATGTCCAAAATCATCTCGTCTTC
GGCAACGTCGGCAATGTCTTTCACCACAGCTTCCGCATCGGCGGCAATGTCTTTCACCAC
AGCTTCCGCATCGGCGGCAAAGGCTTTGGCAACGACGACATCGGTCGGCAGCGGCACAGA
ACCGCCTTTTGCCGCCATTTTCGCCATAATTTTTTTGGATTCTTCCACCAAATCGTGTTC
CGCCAAAGATTTGCCGATGGCTTTGCCTTCCGCCAACAGGAAGGTGTTTGCGATACCGCC
GCCGACGATGAGTTGGTCGACTTTGTCCGCCAGCGATTCGAGGATGGTCAGCTTGGTGGA
CACTTTGCTGCCGGCAACGATGGCAACCATCGGGCGCGCGGGCTGTTTCAAGGCTTTGCC
CAAAGCGTCGAGTTCGCCCGCCATCAATACGCCGGCGCAGGCAACGGGCGCGGCTTGGGC
GACGGCTTCGGTCGAGGCTTGGGCGCGGTGGGCGGTTCCGAACGCGTCATTGACGAACAC
GTCGCACAAAGAAGCGTAGGCTTTACCCAGTTCCAAATCGTTTTTCTTCTCGCCTTTGTT
GATGCGCACGTTTTGCAGCATGACGACATCGCCCGCGTTCAGGGCGGGTTTGTTTTCACG
CCAGTCGTTCAATACTTTCACGTCTTTGCCCAACAGGCTGCCCAAGTGCGCGGCAACGGG
GGCGACATCGTCTTCGGGGTGGAACTCGCCTTCGGTCGGGCGGCCGAGATGGGTCATCAC
GATAACGGACGCACCGTTGTCCACGCAGTATTTAATGGACGCGAGCGAGGCGCGGATACG
GGTGTCGTCGCTGATTTTGCCGTCTTTGAACGGTACGTTCATATCGGCGCGGATGAGGAC
GGTTTTGCCCTGCACGTTTTGTTCGGTCAGTTTTAAAAATGCCATAATCAGTCCTTTTCA
ATCAGTGTTTGCGATACGGAAACAATTGATGCCGTCTGAAGGCTTCAGACGGCATCGCAA
CCCGATCAGCCGGATACGCGCTCGATTTTCGCGCCGACGCTGCCGAGTTTTTTTCAATA
TTTTCATAACCGCGATCCAAGTGGTAAATCTGTTCGACCACGGTTTCGCCTCGCGCCGCC
AAACCGGCGATAACGAGGCTGGCGGACGCACGCAAATCCGTCGCCTTGACGACTGCGCCG
GAAAGCTGTTCCACACCCTGCACAAATGCCGTATTGCCCTCGGTTGTGATGTTCGCCCCC
ATCCGGTTCAACTCGGGGACGTGCATAAAGCGGTTTTCAAAAATCGTTTCCACCACGCGG
CAGCTTCCCTCCGCCACGGCATTCAATGCCATAAACTGCGCCTGCATATCCGTGGGGAAG
CCGGGGTGGACGACCGTGCGGATGTCCACCGCCTTCGGACGCTGCCGCATATCGATGGCG
ATCCAATCGTCGCCCGCCTCAATCACCGCACCTGCCTCAACCAGTTTGTCCAACACCACT
TCCATCGTTTTCGGCGCGGCATTCCGCAAAACCACCCTGCCACCGGTTATCGCCACCGCG
CACAGGAACGTCCCCGCCTCGATCCGGTCGGGGACGACGCTGTGTTCGCAGCCTTGCAGC
TCGTCCACCCCTTCCACAATCATTGTGGACGTACCGATGCCGCTGATTTTCGCGCCCATT
TTGACCAGGCATTCCGCCAAATCGACCACTTCAGGCTCAATGGCGCAGTTTTCCAAAACC
GTCGTACCTTCCGCCAGCGTCGCCGCCATCAGCAGGTTTTCCGTGCCGCCGACGGTAACG
ACATCCATCGCCACGCGCGTACCTTTGAGTTTGCCTTTGGCTTTGACGTAACCGTGTTCG

# Appendix A

ATAACAATCTCAGCACCCATCGCTTCCAAGCCTTTCAAATGCTGATCGACGGGGCGCGAA
CCGATGGCGCAGCCGCCCGGCAGGCTGACTTGCGCCTCGCCGAAACGCGCCAGCGTCGGG
CCCAGCACCAAAATCGAAGCGCGCATCGTTCGGACCAACTCGTAAGGGGCGCAGGTATTG
TTTACCGTACCGCCGTTGATTTCAAATTCGCTGATATTGTCGGTCAGGACGCGCGCGCCC
ATCCCCTGAAGCAGCTTTTGCGTGGTTTTCACATCTGCCAGCATAGGGACGTTTTTCAGG
CGCAACGTACCCGATGTCAGCAAACCCGCGCACATCAGCGGCAATGCCGCGTTTTTCGCG
CCCGAGACCGTTATTTCCCCGTTGAGCGGGCCGTTTGCGGAGATTTTCAGTTTGTCCACG
TTTGTTCTTTCCTGGTGGGTACTTGTATAGTGAATTAACAAAAATCGGGACAAGGCGGCG
AAGCCGCAGACAGTACAGATAGTACAGAACCGATTCACTTGGTGCTTCAGCACCTTAGAG
AATCGTTCTCTTTGAGCTAAGGCGAGGCAATACCGTACTGGTTTTTGTTAATCCACTATA
ATATTTCAATTCTCGGGACAACGCATAAAGCATCACCCGATGAAGGTTGCAGAGGCGGAA
TTATAAGGGATTTTCGGGAAAAATACGGAAGCCGCACCAAAGAATTTGACGAAATGCCGC
GCTTTCCGAACAAGGATTGTCGGAAGACAAAAAAGCCGAGTTTTGAAAACTCAGCTTTTT
TGCTTTATCTGGTGGGTCGTGAGCGATTCGAACGCTCGACCAACGGATTAAAAGTCCGCT
GCTCTACCGGCTGAGCTAACGACCCGATAAGTTTGGAATTTTACAGACCGGCCGAAACCC
TGTCAAGCCCCTTGCGGGCGGACGGGCGTTATATCCGCTTATCGGCCTGTTTTTTTCGTA
TAAACCAAAGAAGTCAACACCGATGCACCCAATGCGCCGAACACGACCGACAGCGAAACG
GAAATCGGGATATGCACCCAATGCATTACCAGCATTTTCACACCGATAAAACCCAACACG
AATGCCAATCCATATTTCAGGAAGATAAAGCGTTCCGCCACATCCGCCAGCAGGAAATAC
ATCGCCCGCAAGCCCAGAATTGCGAAAATATTGGAAGTCAGCACGATAAACGGATCGGTG
GTAACGGCAAAGACGGCGGGGATGCTGTCCACGGCAAACACGACATCGCTCAATTCAATC
ATGACCAGCACCAAAAACAGCGGCGTGGCGATTTTTTTGCCGTTTTCGACGGTAAAAAAT
TTCTCGCCGTGAAATTCCGTGCCGACCGGAACGACTTTCTTGACGGTATTCAGCAGCCTG
CTGTTTGCCAAATCCTCTTTCTCATCGCCTTCGGGCTTCATCATGTGTATACCAGTATAG
AGCAGGAACGCGCCAAACAGATACAGAATCCACTCAAACTGCTGAACCAGTGCCGCGCCG
ACGAAAATCATGACGGTGCGCAATACCAATGCGCCCAATACGCCGTACAGCAGCACGCGG
TGCTGAAACTGTGGTGCGACTTTGAAGTAGCCGAATATCATCAGGAACACGAAAATATTG
TCGACTGCCAACGATTTTTCCAAAATGTAGCCGGTAAAGAATTCCAATACTTTTTCTTTT
GCGACTGCCGCGCCGTAGCCGGGATTGCCGGCGAGTTCAAAATACAGCCAGCCCGCGAAC
AGGCAGGATACGGCAACCCACAAGCCGCTCCATGCCAAGGCTTCTTTGACGCCGACTTTA
TGGCTGCCGTTTTTCTTCAGCGAAAACATATCCAAGGCAATCATGACCAGCACTGCCGCA
AAAAAAACGCCGTAAAACAACGGCGACCCGATGCCGGGATATTCTGTCATGGTTCAATCT
CCTGATTTGAAATGTAATTGTGTTACCAGCTGATATAAAACATCGCTTTTGCCAAAAAGA
CAATCAGCAGCATATGGGTAAAGACGACGGCGTGTATGTATTTCGACCAACCGACCGTCA
GTGTGGAACGCGCCATTTTGACGACGGCGATGGCGAAGTGCGCCAATACGCTGAACGCCA
ACAGGATTTTCAGCGTCAGCATCGTACCGAAGGAAGTGGCAAACGGTTCGCCCAATATAG
AAAGATAGCGGTTTGCCGCCATCACGATGCCGCTGGCGAACAGCAGTCCGACCACAAACG
GCATCACCCTGACGGCGCGGTAAGACATTGCCTTTTCCACTTCGCGCCGCGCCTCGCGCG
ACACCCGTCCCGTATGCAGGACGGACAAAACCAGCACTTCAAAAAACACGCCGCCGACAA
AGGCAATAGCGCAATACAGATGAACGATGTGCGCGACGGCATAAATACTCATACGATGCT
CCAAACGGAAAACTCGGATACGGATTGTATCACTATCGCCCCCGATATCCGCATACCGCT
TCCCGCACCGCCTCGGCGATTCTCGCGCCCGCTCCGCGATGTTGTGCGATAAAGCCGTCC
ACGCGCGCCTGCATCTGCATCCCCCCCCCCTCGGACGATAAGGTTTTTTCAACGGCTTCC
CGCCACGCATCCGCCGATTCGACTTGAACCGCCGCACCCGATGCCAAGGCGTGTCGGCAG
GCTTCGGAAAAATTGTAGGTTGAAAAGCCGAATATCGTCGGAACGCCGCAGGAAAGCGGT
TCGATGATGTTCTGACAACCCGAATCGACCAGACTGCCGCCGACAAAAGCGACATCGGCG
CACAGGTAATACGCATACAGCTCGCCCATACTGTCGCCTATCCACACCTGCGTATCAGGT
TCGACCGGCAAACCGTCGCTGCGCCGCTGAACCTTAAACCCGAAGCGTTTTGCCGTTTCA
AATACCGTCTGAAAATGCTCGGGATGGCGCGGCACGACGACCAGCAGCGCATCGCCGCGA
TATTGTTGCCACGCCGCCAGCAGTTTTTCCGCCTCGTCTTCACCCCGATAAACGCGCGTG
CTGCCGCACACGGCAACCGGCCGGCCTCCGATGCGTTTTTCAAACTGCCCCGCCAGCGTT
TTCATCTGTTCCGACGGTATGATGTCGTATTTGGTATTGCCGCACACCTGCACGGATGCC
GCGCCCAATTTCGCCAACCGCGCCGCATCCGCCTCTGTCTGCGCCAGACACCCCGTCAGC
GAAGCGGCGGCAGGACGGATCAGGCGGCGGACTTTCAGATAACCGTTCAACGATTTTTCC
GACAGCCGCGCATTCGCCAAAAACAGCGGCACACCCGCGCGCCGGCATTCCCTCATCAGG
TTGGGCCAGATTTCGGTTTCCATCAAAATGCCGAACATCGGGCGGTGTTCGCGCAAAAAC
TGCCGTACCCACGTTTTTTTGTCATACGGAAGATAGCGGCATTGCGCATCGGGAAACAGA
ACTTGCGCGGTTTCCCGCCCCGTCGGGGTCATCTGCGTCATCAGCAGCGGCGCATCGGGA
AAACGCCGCCGCAACTCGCGTATCAAGGACTGGGCGGCACGCGTTTCTCCGACCGAAACG
GCGTGTATCCAAACCGCGCCGGTAACGGGATTCGGATACGGCTTGCCGAAACGCTCGTCC
CGATGCGCCCGATATGCCGGGGCACTTCCGGAGCGTTTGTCCAAATAACGCCGTATCCAT
ATCGGCGCAAGCAGCCACAATACATCATAAAGCCATTGGAACATCTTTCTATTTCCTGCA
AAACAAATGCCGTCTGAACGGTTCAGACGGCATTTCGGCAACGGAATCAAATATCGTAGG
TTGTCGAAGCGGTATCTCCGCCCTTGCCCGTCCAGTTGGTATGGAAAAACTCACCGCGCG
GTTTGTCGGTGCGCTCGTAAGTGTGCGCGCCGAAGTAGTCGCGCTGTGCCTGCAAGAGGT
TGGCAGGCAGACGTTCGGTCGTGTAGCCGTCCAAGAACGTAATCGCCGAAGCCATGCAGG
GCATAGGGATGCCGCATTCGACCGCCTTGGCAACCACCTTGCGCCACGCCGGCAGGCAGT
TTTCCAAAATATTTTTGAAATACGGATCCGCACCCAAGAACACCAAATCGGGATTGTTTT
CATACGCGTCGCGGATATTGCTTAAGAATGCGCTGCGAATGATGCACCCCTCGCGCCACA
GCAGCGCAGTGTTGCCGTAGTCCAAATCCCAGCCGTAGCTTTCGCCCGCTTCGCGGATCA
GCATAAAGCCTTGTGCGTAGGAAATGATTTTAGATGCAAGCAGGGCCTGTCTCAACGCCT
CGACCCATTCTTGTTTGCCGCCTTCGACGGGCGTAACGGTTCGGGCGAACAGTTTGCCGG
TCTGCACGCGCTGTTCTTTGAACGACGAAACGCAGCGGGCGAATACGGCTTCGGAAATCA
GCGTCAGCGGAATACCCAAATCCAAAGCATTGATGCCCGTCCATTTGCCTGTACCTTTTT
GCCCTGCCGTATCGAGGATTTTCTCGACCAGCGGTTCGCCGCCTTCGTCCTTATAGCCCA

## Appendix A

AAATTGCCGCTGTGATTTCAATCAGATAAGAATCCAGCTCGGTTTTGTTCCACTCGGCAA
ACACGCGGTACATTTCGTCGTAAGACAGCCCCAAGCCGTCTTTCATGAACTGGTACGCTT
CGCAAATCAACTGCATATCGCCATATTCGATGCCGTTATGCACCATTTTGACAAAATGCC
CCGCACCGTCTTTGCCGACCCAGTCGCAACACGGTTCGCCCTGCGACGTTTTGGCGGCAA
TCGCCTGAAAAATCGGCTTGACCGCATCCCAAGCGCGCTTATCCCCGCCCGGCATAATGG
ACGGCCCGCGCCGCGCCCCTTCTTCCCCGCCGGACACGCCCGCGCCGACAAACAAAATCC
CTTTTTCAGCAAGGTAATGTGTCCGCCGTGTCGTGTCGGGGTAATTGGCATTGCCGCCGT
CGATAAGGATGTCGCCTTCTTCCAACAGCGGAAGCAGTTGTTCGATAAATTCGTCAACCA
CCGAACCGGCACGAACCATCATCATAATTTTTCGCGGTTTTTCCAGCTTATCGACCAAAT
CTTGCAAAGAATACGCGCCGATAATATTAGTTCCTTTTGCCGCGCCGTTTAAAAATTCGT
CCACCTTGGCAGTCGTGCGGTTGTAGGCAACCACCTTAAATCCGCAATCGTTCATATTCA
AAATCAGGTTTTGCCCCATAACCGCCAAACCGATTACACCAATATCGCCGTTCATTGCAG
GAAGCTCCGTTATAGATTTAATTTATCGACCGCAACTCTACCCGATTTACACTTGTTTAA
CAATCCTTAACTTTTTAATTTTTTGAAAAGATGCCTTTACGCTTTGCTGTACCGTTTTGC
TGAAGGGTTATAAATAAAATATAAAATTTAAATAATAAAACGATGATTATATTGATAGGA
GAAATTTTCTGTGGGTAACTTTTTTTTATTTTAAAAATCATCAGGATTTCTTTTTTTTAG
GGTGTCGGTAAGGCGGATTCCCTTTTGTGCATACCTGTGGATTGTTTTTCATGAAGAATA
GTTTTTGTGGACAGTTTGCTTGTTGTGCAAATGGCATCCTACTTTTCTTTACCGAATGGC
TGCCGATGTCTTTAAGAACCGGAATACTGTGGAGGTTTGAGAGGAAAGTGTGTTTGGAAC
TTGTGGAAATGGTCAGGTGTCGGCACGAATGTCTTATTTCTGCATATCGGCAGAGTGCGC
ATCCGAATTTGTGTATAAGTGGTGGAAAAAATGAGATTTGCGGGTAAATCTCACAATATT
TCAGTCAGATAACTTTGGATTGCTTGTGTATAAGTAAACTTTCGGATGGGGATACGTAAC
GGAAACCTGTACCGCGTCATTCCCACGAACCTACATTCCGTCATTCCCACGAAAGTGGGA
ATGATGAAATTTTGAGTTTTAGGAATTTATCGGGAGCAACAGAAACCGCTCCGCCGTCAT
TCCCGCGCAGGCGGGAATCTAGAACGTAAAATCTAAAGAAACCGTGTTGTAACGGCAGAC
CGATGCCGTCATTCCCGCGCAGGCGGGAATCTAGACCATTGGACAGCGGCAATATTCAAA
GATTATCTGAAAGTCCGAGATTCTGGATTCCCACTTTCGTGGGAATGACGGGATTTGAGA
TTGCGGCATTTATCGGAAAAAACAGAAACCGCTCCGCCGTCATTCCCGCGCAGGCGGGAA
TCCAGACCTTAGAACAACAGCAATATTCAAAGGTTATCTGAAAGTCCGAGATTCTGGATT
CCCACTTTCGTGGGAATGACGGGATTTTAGGTTTCTGATTTTGGTTTTCTGTTTTTGTGG
GAATGATGAAATTTTGAGTTTTAGGAATTTACCGGAAAAAACAGAAACCGCTCCGCCGTC
ATTCCCGCGCAGGCGGGAATCCAGACCTTAGAATAACAGCAATATTCAAAGATTATCTGA
AAGTCCGGGATTCTAGATTCCCACTTTCGTGGGAATGACGGCATCAGTCTGCCGTTTACA
GCACGGTTTCTTTAGATTTTACGTTCTAGATTCCCGCCTGCGCGGGAATGACGAATCCAT
CCATACGAAAACCTGCACCACGTCATTCCCACGAACCTACATCCCGTCATTCCCACAAAA
ACAGAAACCTCAAATCCCGTCATTCCCGCGCAGGCGGGAATCTAGACTTGTCGGTGCGGA
CGCTTATCGGATAAAACGGTTTCTTGAGATTCCGCGTCCTGGATTCCCACTTTCGCGGGA
ATGACGAATTTTAGGTTTCTGTTTTGGTTTTTTGTCCTTGTAGGAATGATGAAAATTTAA
GTTTTAGGAATTTACCGGAAAAAATAGAAAGCGTTATCCACAAGTTCTGATGTTCAGCTC
GTGAAATGCGTCGGGCAAATCATCGCTGTCGGCAAATTCCACCCGGTCGTAAGCCGTTTC
GTCTGCCAAAACCGCGCGCAAGAGTGCGTTGTTGATGGCGTGTCCCGATTTGTAGCCTTC
AAATGCGCCGACAATCGGATGTCCGACGATATACAAATCACCGATGGCATCAAGGATTTT
GTGGCGCACAAACTCATCGGGATAGCGCAAGCCTTCAGGATTCAGGACATCCGTGTCGTC
AATCACGATGGCGTTGTTCAAATTGCCGCCCAAACCCAGATTGTGGGCGCGCATCATTTC
CACTTCGTGCATAAAGCCGAAAGTGCGCGCGCGCGATTTCGTCGATGTAGGATTTGCC
GGCGAAATCGATTTCAAAAGTGGGCGAGCTGCGGTTGAAAACCGGATGGTCGAATTCGAT
GGTCAGCGTTACCTTAAAGCCGTCATACGGCGTAAAGCGCACCCATTTGCCCGCTTCTTT
GATTTCGACAGGCTTGAGGATTTTCAAAAAACGCTTTTGCGCCTTTTGATCGACCACGCC
CGCATCTTGCAAAAGGTAAATAAACGGCAGGCTGGAGCCGTCCATAATCGGGATTTCGGG
CGCGTTCAGCTCAATCAGCGCATTGTCGATGCCGTAGGCGGACAGCGCGGACATAATGTG
TTCGATCGTGCCGACGCGCACGCCTTTGTCGGTAACGATGGTGGAGGAAAGGCGGGTATC
GTTGATCAAATAAGGGGTCAGCTTGATTTGTTCGCCCATCTCGCCGTCCAAATCGGTACG
GCGGAAGGAAATCCCGCTGTTTTCAGGCGCGGGGTGCAGGGTCAGCGCGACGCGTTCGCC
CGAATGCAGCCCGACGCCGGTAACGCTGATGGATTTCGCCAAAGTTCTTTGCAGCATAAA
CCGCTTCCTTATCAAGGGGGTAAGTTTTGGAATAATACGATAAAACCGGAAAAACAGGCT
ATGTTTTTCCATAGTATTTGCCAATGTATCCGTTTTCAATACGTAAGCCGCATAAAAATG
TATAGTGGATTAACAAAAATCAAGACAAGGCGACGAACCCACCCCCCTCCTGAAAAACGC
AAAAAATGCCGTCCGAAAACCTTTCGGACGGCATTTTCGCGTAAACCGTCATTCCCACAA
GGACAAAAAACCAAAACAGAAAACCAAAAACAGCAACCTAAAATTCGTCATTCCCGCGCA
GGCGGGAATTTGGAATTTCAATGCCTCAAGAATTTATCGGAAAAAACCAAAACCCTTCCG
CCGTCATTCCCACGAAAGTGGGAATCTAGAAATGAAAAGCAGCAGGCATTTATCGGAAAT
GACCGAAACTGAACGGACTGGATTCCCGCTTTTGCGGGAATGACGGCGACAGGGTTGCTG
TTATAGTGGATGAACAAAAACCAGTACGGCGTTGCCTCGGCTTAGCTCAAAGAGAACGAT
TCTCTAAGGTGCTGAAGCACCAAGTGAATCGGTTCCGTACTATTTGTACTGTCTGCGGCT
TCGTCGCCTTGTCCTGATTTTTGTTAATCCACTATATCTAGCCGAATTACTTTATTTTTT
GATACGTAACCGGCCGGTTGCCGTCATTCCCGCGCAGGCGGGAATCTAGACATTCAATGC
TAAGGCAATTTATCGGGAATGACTGAAACTCAAAAAGCTGGATTCCCACTTTCGTGGGAA
TGACGCGGTGCAGGTTTCCGTACGGATAGCTTCGTCATTCCCGAGTAGGCGGGAATCTAG
TCCGCTTGTTCGGTAAATGAGAGGGCGGATTGCGCGCCTGTCAGATAAACCACGTGTTTA
AACGGGCGGCAATGAGGTACGCGCAGAGCCTTGAAGCGCAATCGATATATTATTTTCAGC
CAAAACGGACGCCCCCGCTTGCCTTGCAAACCTTTAAAAAGGAAGCCACCCGGATTAATC
CGAGTGGCCGTGGAAAATCACTTACCGCTTGATTTATTTAAAATTTATGGTATAATTTAC
CTTAGCTGGCATCACTTGCGTCGCGGCAGGTTGACGGCAGGTGCTTGGTGTCAATCTTCT
TAGCGTTGGCGGCGGCGGCGGCGGTAACGTCGTCGTTGGCGGCTTTGGCTTTGTCGCGCG
TAACCGGCTGTCCGCAGAACCATTTTACCGAACCGTTTTGACGCTTGGCCCACAGGGAGA

## Appendix A

GTTTTTGCCTTTGATTTCGTTGTTTACGTTGCTTGAAGCCATTTGGGCGGTAACGACGC
CGTTTTTGACTTCAACGCTTTTAACATATTTGCCTTTGATTTCAGAGGAGGTTGCCACGC
CGGCAGAAGTGTTGTTGCCGGGCCATTCGCCGTGATTCAGGTAATACTCGGTAACGGCTG
ATTTTTGACCTTCGGCCAAAAGAATGGCTTCGGAAACTTGTGCGCGGGCTGTGTAGTCTT
GATAAGCAGGAAGGGCGACTGCCGCCAAAATGCCGACGATGGCAATCACAATCATCAGCT
CGATAAGGGTAAAACCTTTTTGAAGGGTGTTCATAAAATTACTCCTAATTGGAAAGGAAA
TGCCTCAAGCTTACGCCATCGGCATTATGCAATGTATTTGACCATCGGTATTTTGTTGCG
ATACCTGTGTATTATAAAGCAAGATTGGTACCAAGTTTGTATTTTGAGGTGAAAATTTAT
GCGTTTATCTCTATGTAATTGTTTTTATTTTACATTTTCTTTCGTTTGGCGTGGTTTGAG
TAATTAGGGGGTTGCCGTTTTTTGTCAGCAGTGTTGAAAATTGTCAGTTTTAGTGCCGAT
TTTCGGCACTTTTTTATTGGCGTGGGGTATCTCTATTGGCATGGGGCATCGGGTGTGTTG
ATTGGGTCGGAATTTGAGATTTTTGAATTTGCGCGGTAGCATAGGGTGGGTTGGGTGGGA
AATTTTAAATTTAATTTTTAAAAATTTCCGTTTTCTTGGAAAGTGATTGAAATCGGCGCG
TGGTGTTCCTGTGCAACCGGCAGTTGAATCATCGCGGCAGGTTTCCGTGCGGATGGCTTC
GTCATTCCCGCGCAGGCGGGAATCCAGCCTTGTTGGTACGGAAACTTATCGGGAAAACGG
TTTCTTGAGATTTTACGTTCTGGATTCCCACTTTCGCGGGAATGACGCGGTGCAGGTTTC
CGTATGGATAGCTTCGTCATTCCCGCGCAGGCGGGAATCCAGGTCTGTCGGCACGGAAAC
TTATCGGGTAAAAAGGTTTCTTGAGATTTTTCGTCCTGGATTCCCACTTTCGTGGGAATG
ACGGGATGTAGGTTCGTGGGAATGACGGTTTAGGTATTTTTATAGAAAGCCGTAGGTGGT
GTTTCTATGCAAACGACAGATGAATCATCGCGGCAGGTTGACGGCAGGTGCTTGGTGTCG
ATTTTGTCGGTGCCGGTGGCGGCGGCGGTAACGGCGTCGTCTTTGGCGTTGTCGGCGCGC
GTAACCGGCAGTCCGCAGAACCATTTTACCGAACCGGCTTGACGCTTGGCCCACAGGGAG
AGTTTTTTGCCTTTGATTTCGTTGTTTACGTTGCTTGAAGCCATTTGGGCGGTAACGACG
CCGTTTTTGACTTCAACGCTTTTAACATATTTGCCTTTGATGTCGGCGGAGGTTGCCACG
CCGGCAGAACTGTTGTTGCCGGGCCATTCGCCGTGATTCAGGTAATACTCTGTGACGGCT
GATTTTTGACCTTCAGCCAAAAGAATGGCTTCGTCATTCCCGCGCAGGCGGGAATCTAGG
TCTGTCGGCACGGAAACTTATCGGGAAAACAGTTTCTTGAGATTTTGCGTTCTGGATTCC
CGCTTTCGCGGGAATGACGGGATTAAAGTTTCAAAATTTATTCTAAATAACTGAAATTCA
ACGAACTAGATTCCCACTTTCGTGGGAATGACGAATTTTAGGTTGCTGTTTTTGTGGGAA
TGATGAAATTTTAAGTTTTAGGAATTTATCGAAAAAACAGAAACCGCTCCGCCGTCATTC
CCGCGCAGGCGGGAATCCAGCCTCGTCGGTACGGAAACTTATCGGGTAAAAAGGTTTCTC
TAGTTTGGTGTCGATTTTCTTGTCGATGCTGTTGACGGCAGGTGCTTGGTGTCGATCTGC
TTGCCGTTGGCGGCGGTGTCGGCTTTGACGGCGTCGGCGCTGGCGTTGTCGCGCTTAACC
GGCTGTCCGTAGAACCATTTTACCGAACCGTCTTGACGCTTGGCCCACAGGGAGAGTTTT
TTGCCTTGGATTTCTTTGTTTACGCCGCTTGAAAGCATTGTGGCGGTAACGACGCCGTTT
TTGACTTCAACTTTCTCAACATATTTGCCTTTGATGTTGGCGGAGGTTGCCACGCCGGCA
GAACTGTTGTTGCCGGGCCATTCGCCGTGATTCAGGTAATACTCGGTGACGGCTGATTTT
TGACCTTCAGCCAAAAGAATGGCTTCGTCATTCCCGCGCAGGCGGGAATCTAGACCTTAG
AACAACAGCAATATTCAAAGATTATCTGAAAGTCCGGGATTCTAGATTCCCACTTTCGTG
GGAATGACGAATTTTAGGTTGCTGTTTTTGGTTTTCTGTTTTTGAGGGAATGATGAAATT
TTAAGTTTTAGGAATTTATCAGAAAAAACAGAAACCGCTCCGCCGTCATTCCCGCGCAGG
CGGGAATCCAGGTCTGTCGGTACGGAAACTTATCGGGTAAAACGGTTTCTCTAGTTTGGT
GTCGATTTTCTTGTCGGTGCTGTTGACGGCAGGTGCTTGGTGTTGATGTTGGCGGTGCCC
TTGCCGGTGGCGGCGGTGACGGCGTCGTCTTTGGCTTTGTCGCGCGTAACCGGCTGTCCG
CAGAACCATTTTACCGAACCGTTTTGACGCTTGGCCCACAGGGAGAGTTTTTTGCCTTTG
ATTTCGTTGTTTACGTTGCTTGAAGCCATTTGGGCGGTAACGACGCCGTTTTTGACTTCA
ACGCTTTTAACATATTTGCCTTTGATTTCAGAGGAGGTTGCCACGCCGGCAGAACTGTTG
TCGCCGGGCCATTCGCCGTGATTCAGGTAATACTCGGTAACGGCTGATTTTTGACCTTCG
ACCAAAAGGATAGCTTCGTCATTCCCGCGCAGGCGGGAATCCAGCCTTGTCGGTACGGAA
ACTTATCGGGTAAAACGGTTTCTTTAGATTTTGCGTTCTGGATTCCCACTTTCGTGGGAA
TGACGGGATTAAAGTTTCAAAATTTATTCTAAATAACTGAAACTCAACGAACTAGATTCC
CGCTTTTGCGGGAATGACGAATTTTAGGTTTCTGTTTTGGGTTTTCTGTTTTTGAGGGAA
TGATGAAATTTTAGGTTTCTGTTTTTGGTTTTCTGTCCTTGTGGGAATGATGAAATTTTA
AGTTTTAGGAATTTATCGGAAAAAACAGAAACCGCTCCGCCGTCATTCCCGCGCAGGCGG
GAATCCAGCCTCGTCGGTGCGGAAACTTATCGGGAAAACGGTTTCTTTAGATTTTACGTT
CTGGATTCCTACTTTCGTGGGAAAGACGAATTTTAGGTTTCTGTTTTTGGTTTTCTGTCC
TTGTGGGAATGATGAAAATTTAAGTTTTAGGAATTTATCGGAAAAAACAGAAACCGCTCT
GCCGTCATTCCCGCAAAAGCGGGAATCCAGCCTCGTCGGTGCGGAAACTTATCGGGTAAA
AAGGTTTCTTTAGTTTGGTGTCGATTTTGTCGGTGCCGGTGGCGGCGGCAACGTCGTCTT
TGGCGTTGTCGGCGCGCGTAACCGGCTGTCCGCAGAACCATTTTACCGAACCGGCTTGAC
GCTTGGCCCACAGGGAGAGTTTTTTGCCTTTGATTTCGTTGTTTACGCCGGTTGAAAGCA
TTGTGGCGGTAACGACGCCGTTTTTGACTTCAACTTCCTTAACATATTTGCCTTTGATTG
TTGAAGAAGATGCCACGCCGGCGGCATCATTAAATCCCGTCATTCCCACTTTCGTGGGAA
TGACGGGATTAAAGTTTCAAAATTTATTCTAAATAACTGAAACTCAACGAACTAGATTCC
CGCTTTTGCGGGAATGACGAATTTTAGGTTGCTGTTTTTGGTTTTCTGTCCTTGCGGGAA
TGATGAAATTTTAAGTTTTAGGAATTTATCGAAAAAACAGAAACCGCTCCGCCGTCATTC
CCGCGCAGGCGGGAATCCAGCCTCGTCGGTGCGGAAACTTATCGGGAAAACGGTTTCTTG
AGATTTTGCGTTCTGGATTCCCGCTTTCGTGGGAATGACGGTTTAGGTATTTTTATAGAA
AGCCGTAGGTGGTGTTTCTATGCAAACGACAGATGAAGCGTCGCGGCAGGTTGACGGCAG
GTGCTTGGTGTTGATGTTGTCGGCGGTCTTGGCGGCGGCGGCGACGGTGTCGGCTTTGGC
GTCGGTGCGCGTAACCGGCTGTCCGCAGAACCATTTTACCGAACCGTCTTGACGCTTGGC
CCACAGGGAGAGTTTTTTGCCTTGGATTTCTTTGTTTACGCCGCTTGAAAGCATTGTGGC
GGTAATGACGCCGTTTGCGACTGTAACTTCCTTAACATATTTGCCTTTGATTGTTGAAGA
AGATGCCACGCCGGCAGAAGTGTTGTTGCCGGGCCATTCGCCGTGATTCAGGTAATACTC
TGTGACGGCTGATTTTTGACCTTCGGCCAAAAGGATAGCTTCGTCATTCCCGCGCAGGCG

## Appendix A

GGAATCCAGGTCTGTCGGTACGGAAACTTATCGGGTAAAACGGTTTCTTTAGATTTTGCG
TTCTGGATTCCCACTTTCGCGGGAATGACGGGATTAAAGTTTCAAAATTTATTCTAAATA
ACTGAAACCAACGAACTAGATTCCCACTTTTGCGGGAATGACGAAGTTTTTCTGCCATTT
GCCGTGATTCGGGCAATACTCGGTAACGGCTGATTTTTTGAAAGTGTTTGAAATCGGCGC
GTGGTGTTTCTATGCAACCGGTAGATGAATCATCGCGGCAGGTTGACGGCAGGTGCTTGG
TGTTGATTTTGTCGTCGGTCTTGCCGTTGGCGGCGGCGACGTCGGTGGCGGTGGCGGTGG
CGGTGTCGTTGCGCGTAACCGGCTGTCCGCAGAACCATTTGACCGAACCGTTTTGACGCT
TGGCCCACAGGGAGAGTTTTTTGCCTTTGATTTCTTTGTTTACGCCGCTTGAAAGCATTG
TGGCGGTAACGACGCCGTTTTTGACTTCAACTTTCTCAACATATTTGCCTTTGATGTCGG
AGGAGGATGCCACGCCGGCGGCATCATTAAATCCCGTCATTCCCGCAAAAGCGGGAATCT
AGAACTCAGGACCGGAGAAACCTTTTTACCCGATAAGTTTCCGTGCCGACAGACCTAGAT
TCCCGCCTGCGTGGGAATGATGGGATTAAAGTTTCAAAATTTATTCTAAATAACTGAAAC
TCAACGAACTAGATTCCCGCTTTTGCGGGAATGACGAATTTTAGGTTTCTGTTTGTGGGT
TTCTGTTCTTGTGGGAATGATGAAATTTTAAGTTTTAGGAATTTATCGGAAAAAACAGAA
ACCGCTCCGCCGTCATTCCCGCGCAGGCGGGAATCCAGCCTTGTCGGTACGGAAACTTAT
CGGGTAAAAAGGTTTCTCTAGTTTGGTGTCGATTTTCTTGTCGGTGCTGTTGACGGCAGG
TGCTTGGTGTTGATTTTGTCGGTGTCGGGTGTGGCGGCGGTGACTTCGTCGGTGCCGGCT
TTGGCGTTGGCGGCGTTGCGCGTAACCGGCTGTCCGCAGAACCATTTTACCGAACCGTCT
TGACGCTTGGCCCACAGGGAGAGTTTTTTGCCTTGGATTTCTTTGTTTACGCCGCTTGAA
AGCATTGTGGCGGTAATGACGCCGTTTGCGACTGTAACTTCCTTAACATATTTGCCTTTG
ATTGTTGAAGAAGATGCCACGCCGGCAGAAGTGTTGTTTTTCGGCCATTCGCCGTGATTC
GGGTAATACTCGGGTGTTTTTGTGCAAACGGCAGATGCTGCGTCGCGGCAGGTTGACGGC
AGGTGCTTGGTGTTGGTTTTCTTGTTGCCGGTGTTGTCGGCGGCGACGGTGTCGTCGGTG
CCGGCGCGCGTAACCGGCTGTCCGCAGAACCATTTTACCGAACCGTTTTGACGCTTGGCC
CACAGGGAGAGTTTTTTGCCTTGGATTTCTTTGTTTACGCCGCTTGAAAGCATTGTGGCG
GTAACGACGCCGTTTGCGACTGTAACTTCCTTAACATATTTTCCTTTGATTTTAGAGGAG
GATGCCACGCCGGCGGCATCATTAAATCCCGTCATTCCCACGAAAGTGGGAATCTAGAAC
TCAGGACCGGAGAAACCTTTTTACCCGATAAGTTTCCGTGCCGACAGACCTGGATTCCCG
CCTGCGCGGGAATGACGAAGTTTTTCGGCCATTCGCCGTGATTCGGGCAATACTCGGGTG
TTTTGTGCAAACGGCAGATGCTGCGTCGCGGCAGGTTGACGGCAGGTGCTTGGTGTCAAT
CTTCTTACCGTTGGCGGCGGCGGCGGCGGTAACGTCGTCGTTGGCGGCTTTGGCGTTGTC
GCGCTCAACCGGCTGTCCGCAGAACCATTTTACCGAACCGGCTTGACGCTTGGCCCACAG
GGAGAGTTTTCTGCCTTTGATTTCTTTGTTTACGCCGCTTGAAGCCATTATGTCAGACGG
TATTGCCCGGGCAGCTTTATTCGTACACTTTCAGCAGCTCGACTTCAAATATCAAAGTGG
CGTGCGGGGGAATCACGCCGCCCGCGCCGTGTGCGCCGTAGCCCATTTCCGAAGGGATGG
TCAGCTTGCGTTTGCCGCCTTCCTTCATGCCGCCGAAGCCTTCGTCCCAGCCTTTGATGA
CTTGTCCGACACCGAGCGTGATGGTCAGCGGCTGGCGGCGGTCGAGGCTGGAGTCGAATT
TGGTTCCGTTTTCCAGCCAACCTGTGTAATGCACGGTAATCTCTTTGCCTTTAACTGCTT
CTTTTCCGAAGCCTTCTTGCAAGTCTTCAATAATCAGGCCGCCCATATTTGTCCTTTCGT
TGCTTGTTGGTCAAAACGGCAAGGGTAACATACCGTCCGTCGAAGTCAAATGCCGCTCAA
ACGTCAGCTGCATCGGTGCAGCTGAAACGGCTGTGTTTGTTTGACTGTTTTATTTTTTTC
GTAAAGGTTCCATGCTTTTTCATGGAAATAGAAAACGACGGTGTTGATTAGGGGTTCGAC
CAGCGCAACTGCTCCCGATACGCCTATACTGCCCGTCAGTACATAGGTTACACTGAAGGC
GACGCTGAAATGCAGTGCGGCAAAAGTCAGGGTTTTAAGCATCATCCTCTCCCGGATTGG
ACATTGACGGAGAGATGATAAAGATTATCATAAGGCTGCGCGGTTTAAATTTGCTATTTG
TTGTTAGTGTAGATAAATCGTTTTTTAAATAAGGATAGGAATTATGAATCATAAAAAGAT
CGTTGTTTTGGATGCGGATACTTTGCCCGGCCGGGTTTTTCATTTTGATTTTCCGCACGA
GCTTGCGGTTTACGGTACGACAGGTGCGGATGAAACGGCAGAACGGGTGCGCGATGCACA
TATTGTCATTAGTAACAAAGTGATGATTTCTGCCGATATTATTGCGGCTAATCCGCAGTT
GGAGCTGATTGCCGTCAGTGCGACCGGCGTGAACAATGTCGATATTGGGGCGGCGAAGGC
GGCCGGTGTTGCGGTATGCAATGTCCGCGCATACGGAAACGAATCGGTTGCGGAACACGC
CTTTATGCTGATGATTGCGTTAATGCGGAATTTGCCTGCCTATCAGCGTGATGTTGCGGC
AGGATTGTGGGAAAAGTCGCCGTTTTTCTGCCATTACGGCGCGCCGATTCGGGATTTGAA
CGGCAAAACGCTGGCGGTTTTCGGACGCGGCAATATCGGACGGACGCTTGCCGGATACGC
GCAGGCATTCGGTATGGGGGTGGTGTTTGCCGAACACAAACACGCGTCCGCTGTGCGTGA
AGGCTATGTTTCCTTTGAAGATGCGGTACGGGCTGCTGATGTGTTGTCGCTGCACTGTCC
GCTAAACGCCCAAACTGAAAATATGATAGGCGAAAACGAATTGCGGCAGATGAAGCCTGG
CGCGGTTTTAATCAATTGTGGGCGCGGCGGGCTGGTGGATGAAAACGCGCTGCTTGCCGC
ACTCAAATACGGGCAGATCGGTGGGGCAGGTGTCGATGTTTTGACGAATGAGCCGCCCAA
AAACGGCAATCCCTTGCTGAATGCACGATTACCCAATCTGATTGTTACGCCGCATACCGC
GTGGGCAAGTCGTGAGGCTTTGGACAGGCTGTTTGATATATTGTTGGCGAACATTCACGC
CTTTGTGAAAGGAGAGGCGCAAAACCGCGTGGTTTGAACCTGTCGGGATTGCGGAAAAAA
ATGCCGTCTGAACGCCTCAAGGGTTCAGACGGCATTTCTTGAGATTCCCGTTTAACCGAC
TTTGTCGCCCGGCTGCGCGCCTGTATCCACATCCAAGAGCTTCAGTTTCCCGTCTGCCGT
GGCGGCACTCAAAATCATGCCTTCAGATACACCGAATTTTGCCATTTTGCGCGGGGCGAA
GTTGGCGACGGCGATGACCATGCGGCCGTTCAATTCGGCAGGGTTCGGGTAAGACGCGGC
GATGCCGGAGAAGATGATGCGTTTTTCAAAACCGAAATCGAGGTCGAATTTCAAAAGTTT
GGTGCTGCCTTCGACAGCTTCGCAGTTCAATACTTTGGCAACGCGCATGTCGATTTTCAT
AAAGTCGTCGAAACTCGCCTGTTCGGCGACTTTTTCGTATTTGCCCTCTTCGGCGGCAGG
TGCGGCTGCGGCGGCGATGCTTTGTTTGTTGGCTTCGATTAAATCGTCCACTTGTTTTTG
CTCCACTCGTTGCATTAAATGTTCGTATTTGTTGATGGCGTGTTTGCCCAAGGTATCGCG
TGTATTTGCCCAAGTGATGGCTTCCAAATTCAGGAATTTGGCGGCGTTTGCGGCGGTTTG
CGGCAAGACGGGGGCGAGGTAGGCGGTCAACATGGTGAAGGCGTTGATGAGTTCGCTGCA
TACTTCGTGCAGGCGTTCGTCTTGGCCTTCTTGTTTGGCGAGTTCCCACGGCTTGTTGGC
ATCAACGTATTCGTTGACAATGTCTGCCAAGGCCATGATGTCGCGCAGGGCTTTGGCGTA

## Appendix A

TTCGCGGCTTTCGTAGCATTCGGCAATGGCTTCGCTTTGCGCAGTCAGTTTTGCCAGCAA
TTCGCTGTCGGCAACATCTTTCAGACGGCCTTCAAAGCGTTTGGCGATGAAACCTGAGGC
GCGGGCGGCGATGTTGACGTATTTGCCGACGAGGTCGCTGTTTACGCGGCTGATAAAGTC
TTGCAGGTTCAAATCGATGTCTTCGATTTTGCTGTTGAGTTTGGCGGCGATGTAGTAGCG
CATCCACTCGGGGTTCAGGCCTTGTTCCAGATAGGATTTGGCGGTAATAAACGTGCCGCG
CGATTTGGACATTTTTTGTCCGTCGACGGTCAAAAAGCCGTGTGCGTACACGCCGGTCGG
GGCGCGGTGGCCGGAGAAATGCAGCATAGCGGGCCAGAACAGGGCGTGGAAATAGAGAAT
ATCTTTGCCGATGAAGTGGTACATCTCGGTTTGGCTGTCGGCTTTGAAGTATTCGTCAAA
ATCGACGCCGATGCGGTCGCACAGGTTTTTAAACGACGCCATGTAGCCGACGGGCGCGTC
CAGCCAGACGTAGAAGTATTTGCCCGGCGCGTCGGGGATTTCAAAACCGAAATACGGCGC
GTCGCGGGAAATATCCCAGTCGGACAGGGTGGTTTCTTCACCTTCGCCCAGCCATTCTTT
CATTTTGTTGAGGGCTTCGGCTTGCAGATGGGGCTTGCCGTCGTGCGGGTTGTTGCCGGA
AGTCCATGCTTTGAGGAAGTCGGCGCATTCGCCCAGTTTGAAGAAGAAGTGTTCGGATTC
GCGCAATTCGGGTTTCGTACCGGAAACGGCGGAATACGGGTTAATCAGTTCGGTCGGGGA
ATAGGTCGTGCCGCAGACTTCGCAGTTGTCGCCGTATTGGTCTTGGGCGTGGCATTTCGG
GCATTCGCCTTTGACGAAGCGGTCGGGCAGGAACATTTGTTTTTCGGGGTCGAAAAGCTG
CTCGATGACGCGGCTCTCAATCTTGCCGTTGGCTTTCAGCGCGCGGTAAATGTCTTGGGA
AAACTGTTTGTTTTCAGGGGAATGGGTGCTGTAATAATTGTCGTAACCGATGAAAAAGCC
AGTAAAGTCGGCGAGGTGCTCTTCGCGCACTTTGGCAATCATGTCTTCGGGCGCGATACC
TTGTTTTTGCGCGGCAAGCATTACGGGCGTGCCGTGGGTGTCGTCGGCGCAGCAGTAGTG
GCACGCGTGGCCGCGCAGTTTTTGAAAGCGCACCCAAACGTCGGTTTGGATGTGTTCGAC
CATGTGGCCGAGGTGGATGCTGCCGTTGGCATAGGGCAGGGCGGAGGTAACTAAGATTTT
GCGTGTCATATTGTGCTTTGCAAACAATGGGTAAAGGCGGATTATACCGCAAATCAAACG
GGGAAATGCCGTCTGAAGCCTGAAAAATCGGGCTTCAGACGGCATTTTTGCCAACCGGCG
GGAGTTATTCGACGGTTACGGATTTCGCCAGGTTGCGCGGCTTGTCCACATCGGTACCGC
GTGCGAGGGCGGTGTGGTAGGCGAGGAGCTGCACGGGGATAGTATGCACGACGGGGGACA
GTTTGCCGACGTGGCGCGGTGCGCGGATAACGTGCACACCTTCGGTGGCATTAAAATTGC
TGTCGAGGTCGGCAAAGACGAAAAGTTCGCCGCCGCGCGCGCCGACTTCCTGCATATTGG
CTTTGACTTTGTCCAACAGGCTGTCGTTGGGTGCGATGACGACGACGGGCATATTTTCGT
CCACCAGGGCAAGCGGCCCGTGCTTCAGTTCGCCGGCAGGATAGGCTTCGGCGTGGATGT
AGGTGATTTCCTTCAGCTTCAACGCACCTTCGAGGGCAATCGGGTAATGGATGCCGCGCC
CTAAAAACAGCGCGCTGGTTTTCTTGGCAAACTGTTGCGCCCATGCGGCAATTTGAGGTT
CGAGGTTCAGAGCGTGCTGCACGCTGCCGGGAAGCTGGCGGAGTTCTTCGGTGTAACGCG
CTTCGTCTTCTTCGGAAACCAAACCGCGCACTTTCGCCAGCGTTACCGCCAAACCGAACA
GCGCAACCAGTTGCGTGGTAAACGCTTTGGTCGAGGCGACGCCGATTTCCGCACCGGCAC
GGGTATAAAGCACGAGGCTGCTTTCGCGCGGCAGGGCGGATTCCATCACGTTGCAAATGG
AGAGGCTGTGGCGGTGTCCCAAGGATTTGGCGTATTTCAACGCCTCCATCGTGTCCAGCG
TTTCGCCGGATTGGGAAATGGTAATGACCAGTTGGTCGGAATCAGCAATCACGCTGCGGT
ATCGGTATTCGCTGGCGATTTCGACGTCGGACGGGATTTTTGCGATGGATTCCAACCAAT
ATTTGGCGGTCAGCGCGGCGTAATAGGACGTGCCGCAGGCAAGGATTTTGACGCTGCGGA
TGCTTTCAAACACGCTTTTGGCATCTTTGCCGAAGTTTTCGGGGATGAAGCCGCCGTCGA
GGAAAACCTCCGCCGTGTCTGCAATCGCGCGGGGCTGCTCGTGGATTTCTTTTTGCATAA
AGTGGCTGTACAGTCCCAGTTCCAAAGAGGCGAGCGAGAGTTCGGATACCTTGACTTTGC
GTTCGGCAGGCAGGCCGTTTTTATCGGTCAGCCTTTTGATGCCGTCTGAAGCCAGCAGCG
CGATGTCGCCGTCTTCGAGGTACGCCACGCGGCGCGTAAAGGCGATGACGGCGGATACGT
CCGAAGCGATAAAGGTTTCATCGTCGCCCAAAGCGACCAAAAGCGGGCAGCCCATACGCG
CCACAACTAATTCATCAGGCTTGTCTTGGGCAATAACCGCGATGGCGTATGCGCCGTGGA
AACGTTTGACCGCTTCTTGTACCGCTTCAAACAGCCTGCCGCCGTTTTGCGCGTATTCGT
GATTGATGCTGTGTGCGATGACTTCGGTATCCGTTTGCGATTCAAAACGGTATCCCAAAC
CTTCCAAACGTTTGCGTTCGCTTTCAAAGTTTTCGATGATGCCGTTGTGTACGACCGCAA
TCATACCGCCGCTGATGTGCGGGTGGGCGTTCGGCTCAGTAACGCCGCCGTGTGTCGCCC
AACGCGTATGTCCGATGCCGATGCCGCCGCTGATGCCTTTTTCGCGTGCCGCGTCCTCCA
TAAGCTGCACGCGTCCGACGCGGCGCACACGTTTGATTTTGCCGTCGGTGTTGACGGCAA
TGCCTGATGAGTCATAACCCCGGTATTCGAGGCGTTTGAGACCGTCGGTCAGAAAATCGA
CGACGTTGTGATGGGCGCGGATGGCGCCGACGATACCGCACATAACTGTTCCTTAGTATC
CGGTTGAAAAAAAACAGGCGCGGACGGCTTCCGTGCCGCACCTTCCTCTTCGGATTATAA
ACCGCCTCCCGCGCCGGAAAACAGCAAAATGCCGTCTGAAGGCTTGGGCTTGCTCAAAAA
AAGGAGGGATTTCCCTGTTTATCCAGGATGGGCGTTCAGACGGCATTACCTGCTGCTGGT
TTATAGTTTTTGCAAATCAACATTGACAAGCTGAAAAAAAAAAACAATATACTCGCTCGG
TCTTAATGTTAACGGAGTATGGAAATGAAACAAATGCTTTTAGCCGTCGGCGTGGTGGCG
GTGTTGGCGGGCTGCGGCAAGGATGCCGGCGGTTACGAGGGTTATTGGCGCGAAAAGTCG
GACAAAAAAGAGGGTATGATTGCCGTCAAAAAAGAAAAAGGCAATTACTTCCTTAATAAA
ATCCACGTGGTTACAGGCAAGGAAGAGTCCTTGCTTTTGTCTGAAAAAGACGGCGCGCTT
TCGATAAACACAGGGATAGGGGAAATCCCGATCAAACTTTCCGACGACGGGAAAGAGCTG
TATGTCGAACGTAGGCAGTATGTCAAAACCGATGCGGCGATGAAGGACAAAATCATCGCC
CATCAGAAAAAGTGCGGACAAACAGCACAGGCATACCGCGACGCGCGAAATGCGTTGCCG
TCAAACCAGACGTATCAGCAGCATCTGGCGGCGATCGAGCAATTGAAACGGCGGTTTGAA
GCCGAGTTTGACGAATTGGAAAAAGAAATCAAATGCAACGGCAGAAGCCCGGCATTGTTG
CTTTAGTAGGGGACAACCGGGAGGATGCCGCCGTCCGAATCGGATGTGCGGTTTCTGTAC
CGGTACGGGCGGGCAGGAATGTCCGCCTTTTTTGTTCGGATGCGTTTGAATACCCGTTTG
ATTCCGACCGTTTGCAAGGGGTATTTCCGTTCGGGCGGAAATTATAGTGGATTAACAAAA
ACCAGTACGGCGTTGCCTCGCCTTAGCTCAAAGAGAACGATTCTCTAAGGTGCTCAAGCA
CCAAGTGAATCGGTTCCGTACTATTTGTACTGTCTGCGGCTTCGTCGCCTTGTCCTGATT
TAAATTTGATCCACTATAATTCCGTCAAATAAGAAAGGAATTTTGTGCCTGCGGTATCGC
AAAACTTCGCCTTAATGCGCCCGATTGCCTAGGGATGGGCTTCAGATGGCATTGTTTTCC

## Appendix A

GGTTTACGGGCGGTATTCGGGCTTCATACCGTTGGGTAGGAGCTGCCAGACATATCCCGT
GGTTTTCTGTTTGCCGGCAAGTTCGCCGGCTTCGTCGCCGTATCCCCAAAAATAATCCAC
GCGCACCGCGCCTTTAATCGCGCTGCCGGTATCCTGCGCCATAATCAGGCGGTTGAGGGC
TTTGCGGGTAACCGGATGGGCGGTGGCGACAAATAAGGGCGCACCCAAGGTAATGTAGTG
CCGGTCGACTGCGCCGGCATATTCCCCCATCAGCGGCGTGCCCAGTGCGCCGACAGGGCC
GTCATTGCTGCTTCCGGCAAGCTCGCGGAAAAAGATATAGCTGGGGTTTTGACCCAAAAC
TTCGGCGAGGCGTTGCGGATTTTGCCGCATATAAGACTTAATGCCCTGCATGGAGGTTTG
TCCGAGTTTGAGGTAGCCCTTATCCGCCATATAGCGTCCGATGGAAACGTAGGGATGTTC
GTTTTTGTCGGCATAGCCGATGCGGATGTATTTGCCGGACGGGGTTTTCAGACGGCCCGA
GCCTTGGATGTGCATAAAAAAAAGTTCGACAGGGTCTTCGGCGTAACCGAGTATCGGGGC
TTTGCCGTCAAGCGCGCCGCCGTTGATTTGGTTGCGCGTGTGGTAGGGGAGGAAGCGGCT
TCCTTCAAACCTGCCTTTGATTGCTGTTGTGCGCGCGGTGATGGGGAATCGGGAGAGGTC
GGCGGTATGTGTGCCGCCGGTATTGTCGATTGTGCCGCTGTTTTTTCCCGTCTGCCTGAT
GCGGACAAGGGCTTTTCCGCTCCGCAAACCGGCAGGCAGGGGGACGGAGATAAAATCGTC
GGGAATACCGTAAATCGGGAAGCGGGCTTGTGCCGTCCGCCTGTCGTCGCCCTTCAGCAC
CGGTTCGTAATAGCCGGTAACCGTACCGGCAAGGCTTCCGTTGCCTGCAACCTGCCACGG
CGTGAAATAGCGTTCAAAAAACTGTTTTGCCTGAAAGGAATGGACGGGGGTTTGAAAGGC
TTGGGCGCACACATCCTGCCAGCCTTGGCGGTTTTTCAAATTGGCGCAGCCGAGGCGGAA
GGATTGCAGGCTTTTGGCGAAATCCTGCGCCGCCCAGTGGGGCAGGGACAGGTGCGGTAC
AACGGTATAGACGGCCCCGCCGCCGCCGACCGTCGTTCCGGCGGGGTCGGGGATGCCGAC
CGGCCGGTCCGGGCCGTTGATGACGGATGTGTCGGGTTGCGGAAAGGTTTGGATGCTCTT
GCTTTGGCAGGCGGCGAGGATGGCGGCGGCGATGCCGTACAGGGCGGCGCGGAATAGGTA
TTTTTTCATAATGGACAATGTTGCCGGCAGTAATAAGAAAGATGGTTTCGGGCGGCGTTG
CGGCAGCCGTGGAGAGGGGATTTTAACACAGGGCGCAGCTGCAGCCTGCGGAACTTTCCG
CCGCGCGGTACTGCAGATAAAAATAACTTGCATTTGTATTTACAAGCAATGAAAATATTC
CGATAATATATTATTCATCATCCTTGTTCGTTCGCGTTTATGCTGGTCGCTTTTTTAATT
ATGTTGCGCGAGGGTATTGAAGCCGCGCTCATTGTCGGCATCGTTGCCGGTTTTCTGAAA
CAGTCCGGACATTCCAAACTGATGCCTAAGGTCTGGTTCGGGGTCGTCCTTGCTTCTTTG
ATGTGTTTGGGGCTGGGGTACGGCATCCATTCGGCAACGGGCGAGATTCCCCAGAAGCAG
CAGGAGTTCGTCGTCGGCATTATCGGTTTGGTTGCCGTTGCCATGCTGACTTATATGATT
TTATGGATGAAAAAGGCGGCGCGTTCGATGAAGCGGCAGCTTCAGGATTCTGTGCAGGCG
GCTTTGAACCGTGGCAGCGGTCAAGGATGGGCCTTGGTCGGTATGGCGTTTCTTGCCGTG
GCGCGCGAAGGTCTGGAGAGTGTTTTTTTCCTGCTTGCCGTATTCAAACAGAGCCCGACG
TGGCAGATGCCGGCCGGCGCGGTAGCGGGGGTTTTGGCTGCCGCCGTGATTGGCGCGTTG
ATTTATCAGGGCGGGATGCGCCTGAATCTGGCGAAGTTTTTCCGTTGGACGGGGGCGTTT
CTGATTGTCGTTGCCGCCGGCCTGCTTGCCGGCTCGCTGCGCGCGCTGCATGAGGCAGGT
ATTTGGAACGCGCTTCAGGACATTGTGTTCGACTCATCAAAATATTTGCACGAAGACAGT
CCGTTGGGCGTGCTGCTCGGCGGATTTTTCGGCTATACCGACCATCCGACGCAGGGCGAG
ACCTTGGTTTGGCTGCTGTACCTTATTCCCGTCATAACTTGGTTTTTGTGCGGCAGCAGG
CCGTCTGAAACTTTAACCCGTAAAGAGGAGCTGAAATGAGAAAATTCAATTTGACCGCAT
TGTCCGTGATGCTTGCCTTAGGTTTGACCGCGTGCCAGCCGCCGGAGGCGGAGAAAGCTG
CGCCGGCAGCGTCCGGTGAGGCGCAAACCGCCAACGAGGGCGGTTCGGTCAGTATCGCCG
TCAACGACAATGCCTGCGAACCGATGGAACTGACCGTGCCGAGCGGACAGGTTGTGTTCA
ATATTAAAAACAACAGCGGCCGCAAGCTCGAATGGGAAATCCTGAAAGGCGTGATGGTGG
TGGACGAGCGCGAAAACATCGCCCCCGGACTTTCCGATAAAATGACCGTCACCCTGTTGC
CGGGCGAATACGAAATGACTTGCGGTCTTTTGACCAATCCGCGCGGCAAGCTGGTGGTAA
CCGACAGCGGCTTTAAAGACACCGCCAACGAAGCGGATTTGGAAAAACTGTCCCAACCGC
TCGCCGACTATAAAGCCTACGTTCAAGGCGAGGTTAAAGAGCTGGTGGCGAAAACCAAAA
CTTTTACCGAAGCCGTCAAAGCAGGCGACATTGAAAAGGCGAAATCCCTGTTTGCCGACA
CCCGCGTCCATTACGAACGCATCGAACCGATTGCCGAGCTTTTCAGCGAACTCGACCCCG
TCATCGATGCGCGTGAAGACGACTTCAAAGACGGCGCGAAAGATGCCGGATTTACCGGCT
TTCACCGTATCGAATACGCCCTTTGGGTGGAAAAAGACGTGTCCGGCGTGAAGGAAATTG
CAGCGAAACTGATGACCGATGTCGAAGCCCTGCAAAAAGAAATCGACGCATTGGCGTTTC
CTCCGGGCAAGGTGGTCGGCGGCGCGTCCGAACTGATTGAAGAAGTGGCGGGCAGTAAAA
TCAGCGGCGAAGAAGACCGGTACAGCCACACCGATTTGAGCGACTTCCAAGCCAATGTGG
ACGGATCTAAAAAAATCGTCGATTTGTTCCGTCCGCTGATCGAGGCCAAAAACAAAGCCT
TGTTGGAAAAAACCGATACCAACTTCAAACAGGTCAACGAAATTCTGGCGAAATACCGGA
CTAAAGACGGTTTTGAAACCTACGACAAGCTGGGCGAAGCCGACCGCAAAGCGTTACAGG
CCTCTATTAACGCGCTTGCCGAAGACCTTGCCCAACTTCGCGGCATACTCGGCTTGAAAT
AAGCCGCAAGCGTTCAGACGGTATTTGGCGGCAGATACCGTCTGAAGTTTTATAGTGGAT
TAACAAAAACCAGTACGGCATTGCCTCGCCTTGCCTTGCCGTACTATTTATACTGTCTGC
GGCTTCGTCGCCTTGTCCTGATTTTTGTTAATCCACTATATCCGCCATATATTGCAGGGC
GGGATTTCAACCTGCCGCTATCGGTTAATGGAAAAACGGCGTGCAGGGATACCCATCCTG
CTGCACGGATATTGAAGGAAACACCATGAGCAAAAAACAACCCGCACAACCGACCAGGCG
CACTCTTTTTAAAACCGCGATCGCAGCCGGAGCAGTCGGCGCAATCGGAGGTTATCTCGG
CGGCAAAAAACAGGGCGAAACCGCCGAACGCACCGCCGAAAGCCAACACTCGCCCCAAGC
CTATCCCTGCTACGGCGAACATCAGGCAGGCATCGTTACGCCGCAGCAGGCGTTTTCGAT
TATGTGCGCCTTCGACGTAACCGCGCAAAGTGCCAAGCAGCTGGAAAACCTGTTCCGCAC
GCTGACCGCCCGCATCGAGTTTCTCACCCAAGGCGGCGAATACCAAGACGGCGACGACAA
ACTTCCGCCAGCCGGCAGCGGCATTTTGGGCAAAGCCTTCAACCCCGACGGGTTGACCGT
TACCGTGGGGGTGGGCAGCAGCCTGTTTGACGGCCGGTTCGGACTCAAAGACAAAAAACC
GATTCATTTGCAGGAAATGCGCGACTTCTCCAACGATAAGCTGCAAAAAAGCTGGTGCGA
CGGCGATTTGAGCCTGCAAATCTGTGCCTTCACCCCCGAAACCTGCCAAGCCGCCCTGCG
CGACATCATCAAACACACCGTCCAAACCGCCGTTATCCGTTGGAGTATCGACGGGTGGCA
GCCCAAATCCGAACCCGGCGCGATGGCGGCGCGCAACCTGTTGGGCTTCAGGACGGCAC

## Appendix A

GGGCAACCCCAAAGTTTCCGATCCCAAAACTGCCGACGAGGTTTTGTGGACGGGGGTGGC
CGCCAACAGCCTCGACGAACCGGAGTGGGCGAAAAACGGCAGCTATCAGGCAGTCCGCCT
TATCCGCCACTTTGTCGAGTTTTGGGACAGGACGCCGCTTCAAGAGCAAACCGACATTTT
CGGGCGGCGCAAATACAGCGGTGCGCCGATGGACGGCAAAAAAGAAGCCGACCAACCGGA
TTTTGCCAAAGACCCCGAGGGTGATATCACGCCCAAAGACAGCCATATACGCCTGGCGAA
TCCGCGCGATCCCGAATTCCTCAAAAAACACCGCCTCTTCCGCCGCGCCTACAGCTATTC
GCGCGGACTCGCCTCAAGCGGACAGCTTGATGTCGGGCTGGTGTTCGTCTGCTATCAGGC
AAACCTTGCCGACGGATTCATCTTCGTGCAAAACCTCCTCAACGGCGAACCGCTGGAAGA
ATACATCAGCCCCTTCGGCGGCGGCTATTTCTTCGTCTTGCCCGGCGTGGAAAAAGGCGG
CTTTTTGGGGCAAGGGCTGCTGGGCGTATAAATCCGCCATATAAAAAACGCCGTCCGAAC
CTTGCCAAACGGGTTCGGACGGCGTTTCTTGTTTTGGGCGGTCAGGCTTTTTTGACGAA
TTCGGATTTTAAATTCATCGCGCTGCCGTCGATTTTGCAGCCGATGTTGTGATCGCCTTC
TTGCAGGCGTATGCCTTTGACTTTTGTGCCTTGTTTGATCACCATCGAGCTGCCTTTTAC
CTTGAGGTCTTTGATGAGGATGACGGTATCGCCGTTTTGCAGCACTGCGCCGTTGGCATC
GCGCACTTGAGCCGCAAGGTCGGCGGCGGATTCGGTTTCATTCCATTCATGGGCGCATTC
GGGGCAGATGTATTGTCCGCCGTCTTCATAGGTGTATTCGGAGGCGCATTGCGGGCATGG
GGGTAATGACATGGTTTGCCGTCCTTATCGGATGTTTGTTTTGGGGTGCCGTCTGAAACC
TGAAACCGGCTTCAGACGGCATAGCTTTATTGTTTGTCTTTTTCAGGACGCACCCAGCCT
TCGATGACGGTTTGGCGGGCGCGGGCGAGGGCGAGTTTGTTGTCTTCGACATTGCGGGTA
ATCGTGCTGCCCGCGCCTGTGGTTACTTTGTTGCCGAGGGTAACGGGGGCGACTAGGACG
CAGTTTGAACCGATGCGCACTTCGTCGCCGATGACGGTTTTGTGTTTGTGCACGCCGTCG
TAGTTGGCAATAATCGTACCGGCGCCGAAGTTGGTTTTGCAGCCGACTTCGGCGTCGCCG
ATGTAGGTGAGGTGGTTGGCTTTGGTGCCTTTGCCGATGGCGGCGTTTTTGATTTCGACG
AAGTTGCCGACGTGTACGTCGTCTGCAAGGCGGGCTTGCGGACGCAGGCGGGCGTACGGG
CCGATTCGGTTGTTTTCGCCGACTTCGCAGCTTTCGAGGTGGGAGAAGGGGGCGATTTTG
CTGTTTGCGCCGATTTTGGCGTTTTTGATGACGCAGTTTGCGCCGATTTCGACGTTGTCG
CCGAGCTCGATGTCGCCTTCAAAGATACAGTTCACATCAATCACGACGTCTTGCCCGTGT
TTCAGACGGCCTCGTAAATCGAAACGTGCCGGATCGCGCAGGGTTACGCCTGCTTTGAGC
AATTCTTGCGCCTGTTCGGTTTGGAAGATGCGTTCGAGTTCGGTGAGCTGGAGTTTGTTG
TTCACGCCGGCGGCGAGGTGGGAGGCGCGCACTTGGACGGGATGAACTTTAATACCGTCG
GCAACGGCTTTGGCGATGAGGTCGGTCAGGTAGTATTCGCCTTGTGCATTGTTGCTGGAA
AGGCTGTTCAGCCAGTTTTCGAGTTTGGCGTTGGGCAGGACGAGGATGCCGGTATTGATT
TCTTTCACGGCTTTTTGGACGGCGTCGGCGTCTTTTTCTTCGACGATGGCGGTTACGCTG
CCGTTGCTGTCGCGGATGATACGCCCCAAGCCTGTCGGGTCGTTGGGAACGTCGGTCAAC
AGCCCGACTTCGTTGCCTGCGGCTTCGAGCAGGGTTTCGAGGGTTTCAACGTCAATTAAA
GGAACGTCGCCGTACAACACCAGCGTGCGGCCTTCGGCGGAAAGGTGGGGCAGGGCGGTT
TTGACGGCGTGGCCGGTACCGAGCTGTTCGGTTTGTTCAACCCAAACGACATCGCGTTTG
ACGGTGTCCAAGACTTGCTCTTTGCCGTGGCCGATGACGACGCAGATGTTTTGCGGATTC
AGTGCGGCTGCGGTGTCGATAACGCGCCCGACCATGGGCTTGCCGCCGATGCGGTGCAGC
ACTTTTGGCATTTTGGAATACATGCGCGTGCCTTTGCCGGCGGCGAGGATGACGATGTTT
AAAGTGTTTTGCGGCATGACGGTTTCCTGTGCAATGCCGTCTGAAGCGGCTTCAGACGGC
ATAGGGTAGGTTTATCGGTTTTGAAACTTTGGTTTTTGCCAGTGTTGGCGATGCTCTTCG
TCGGCGTTGTTGCCGGTTTGATTGGGTAACACGGCATGGCGTTCGGGACGGTATTGGTTG
TAGTTCATATTTTTCGAGTAGCTGCCGTCTTGGTAATAAACGGGCGTGCCGGCGGGATAT
TTTTGACGGACGGCGGTCTTGCCGTTGCCGTCTTGATAAGTTTCCCACGCGCAGCCCGAC
AAAAGGGCGGCGGCAGCGGTCAGGAAGAGGAAGGTTTTACGCATGGCTTTTCTTTCGTAT
TTTCGGGGGGTAGGGGGTATTGTAATGATTTTGGCGGTGTTCTGACAAAGTTTCTGCATA
CCGAGCCAGTTGCGCCATATCGCTTACGGAGGCATCGATAAAGGGCAGCGCGTGGGATTT
TGCACCGAAGGGGAGGGTTTTCATACCCAGCGCCTTTGCCTGATGCAGGTTGTCCGCGCT
GTCGTCCACCATAATGCAGCATTCGGGCGGTACGTCCAACAGGCGGCAGACATTGAGATA
CGCTTGCGGATTGGGTTTGTACAGCAGCCCGAAATCATCCGTGCCGAAAAGCGCGTCGAA
ACGGTTTTCCAAACCGAGTGCGTTGACAACGGCACGGACGTAAAACGACGGGCCGTTGGA
AAAAACCGCCTTGCGCCCTTTTAGGCGGCTCAGGGTGTTTTGTGTTTCAGGCATGCCGTG
CAGCCTGGTCAGGATTGCATCGATCGGATGGCTTTCGCGCAAAAATTCGGCGATGTCGAT
TTCGGGATGGTGGATTTGCAGTCCGGCGAGCGTTGCGCCGTAGCGGTGCCAATAGTCTTG
ACGCAGGTCGGACGCGGCAGATTCGGAGAGTTTGAGGCGGCGTGCCATATAGCGTGTCAT
AGCGCGGTTGATGAGTGTGAAGATGCCTGCGTCGGCATCGTGCAGCGTGTTGTCGAGGTC
GAACAGCCACACGGTCGGGTTTTCTTGCATGTTGAACCGTGAAAATTTGTTAGAATGTTA
TTTTACAGCGAATAGAGGAGGACTCGGAATGAAACGGAAAATTTGGCTGCTGCCGCTGCT
GGCGGTTTCGGCATACCTGCAGGCGCAGACGGAAGTCAGGCTGGCGGTGCATAAGTCGTT
CAGCCTGCCCAAAGGGTTGATTGCGCGCTTCGAGCGGGCAAACGATGCGAAGGTGTCGAT
TATTCAGGCGGGCGGCGCGAACGAAATGCTCAACAAACTGATTTTGAGCCGCGCCAACCC
GATTGCCGACGCGGTGTATGGTTTGGACAACGCCAATATCGGCAAGGCGCGGGAAATGGG
CATTTTGGCGGCGGCGCAACCCGAATCCGCCCCCGTCGCGGTCGGGCTGCCTTCGGCTTT
GGCGGTCGATTACGGCTATGTGTCCATCAATTACGACAAAAAATGGTTTGAAGGCAAAAA
GCTGCCCCTGCCGCAAACCCTGCAGGATTTGACCCGCCCCGAATATAAAAACCTATTGGT
CGTGCCGTCCCCCGCCACGTCGTCCCCGGGGCTGGGCTTCCTGATGGCGAACATCAGCGG
TCTGGGCGAAGAAAGCGCGTTCAAATGGTGGGCACAGATGCGGCAGAACGGCGTGAAGGT
CGCCAAAGGCTGGAGCGAGGCGTATTACACCGACTTTTCGCACAACGGCGGCGCGTATCC
GCTGGTGGTCGGTTATGCCGCCAGCCCGGCGGCGGAAGTGTATTTTTCCAAAGGCAAATA
CAGCGAGCCGCCGACGGGCAACCTGTTTTTAAAAGGCGGCGTATTCCGCCAGGTCGAAGG
CGCGGCGGTCTTGAAGGGCGCGAAACAGCCGGAATTGGCGGCAAAACTGGTGCAATGGCT
GCAAAGTCGGGAAGTGCAGCAGGCGGTTCCGTCCGAAATGTGGGTTTACCCCGCCGTCAA
AAACACGCGCCTGCCCGACGTGTTCCGCTTCGCCCAAGCCCCGACGCACACCACCGCCCC
CGCGCAGCGCGATATTGATGCGAACCAGCGCGGATGGGTTTCCCGTTGGATTAGAACGGT

# Appendix A

TTTGAAATAAAACAAACATACCTCCCCGCAGGGCTTCATACGGCATTTTTACACCTGTGC
CGATTACGCCGCACGGGGCGGATGTTCGATCAAGAGGAAAACAATGGACTTCAAACAATT
TGATTTTTTACACCTGATCAGTGTTTCCGGTTGGGAGCATCTGGCTGAAAAGGCGTGGGC
GTTCGGGCTGAACCTTGCCGCCGCGCTGCTTATTTTTTTGGTCGGAAAATGGGCGGCGAA
ACGCATTGTCGCTGTGATGAGGGCGGCGATGACGCGCGCGCAGGTCGATGCCACGCTGAT
TAGTTTTTTGTGTAATGTTGCCAATATCGGCTTATTGATTTTGGTGATTATTGCCGCATT
GGGCAGATTGGGCGTTTCCACAACATCCGTAACCGCCTTAATCGGCGGCGCGGGTTTGGC
GGTGGCGTTGTCCCTGAAAGACCAGCTGTCCAATTTTGCCGCCGGCGCACTGATTATCCT
GTTCCGCCCGTTCAAAGTCGGCGATTTTATCCGCGTCGGCGGTTTTGAAGGATATGTCCG
AGAGATTAAAATGGTGCAGACTTCTTTGCGGACGACCGACAACGAAGAAGTCGTGCTGCC
CAACAGCGTGGTGATGGGCAACAGCATCGTCAACCGTTCCACACTGCCGCTGTGCCGCGC
CCAAGTGATAGTCGGCGTCGATTACAACTGCGATTTGAAAGTGGCGAAAGAGGCGGTGTT
GAAAGCCGCCGTCGAACACCCCTTGAGCGTTCAAAACGAAGAGCGGCAGGCTGCCGCCTA
CATCACCGCCTTGGGCGACAATGCCATCGAAATCACATTATGGCTTGGGCAAACGAAGC
AGACCGCTGGACGCTGCAATGCGACTTGAACGAACAAGTGGTCGAAAACCTCCGCAAAGT
CAATATCAACATCCCGTTCCCGCAACGCGACATACACATCATCAATTCTTAAACGCCGTC
TGAAAGAGGAGTGGGAAATGGACGCGCTGCACACCATCGCCCGAAACCTGACGAAAAAAC
GTCAAACCGTAAGCTGTGCCGAATCCTGTACGGGCGGAATGCTTGCCGCCGCATTCACAA
GCGTTGCAGGCAGTTCGCAATGGTTCGACCAGAGTTTTGTAACATACAGCAACAAAGCCA
AAGAAGACCGCTTGGGCGTGTTGCCCGAAACCCTGCTCGAACACGGCGCGGTCAGCCGCC
AAACCGTCTATGAGATGGCGCGCGGCGCGAAAGCCGTGGCGCAGGCGGATTACGCCGTCG
GTATTTCCGGCATCGCCGGTCCGGGCGGCGGCAGCGAAAGCAAACCCGTCGGCACGGTTT
GGTTCGGGTTTGCCTTTCCGGGCGGAAGTTGCGAAGCAATGCGCCGTTTTGACGGCAACC
GCGAATCCGTCCGCGCGCAGGCGGTCGCCTTCGCGTTGGAACGGTTGGCGGGGCTGATTG
AAAACGGCGGCGATGCTGTCTAAACAAAATCTCCGTCTGAACAAAATCCCCATCGGATAA
AAAATGCCGTCTGAAACGTTTCGGGTTTCAGACGGCATTTTGTCGGGGTAGGCGGCGGTG
CGGCTTATTTCACTTTACCTTTCAACGCGCCATAGCCTGCCGCGTCCATTTGTTCCAGCG
GGATGAATTTCAAGCTCGCGCCGTTGATGCAGTAGCGCAGTCCGCCTTTGTCGCGCGGGC
CGTCGGGGAAGACGTGTCCCAAATGCGAGTCGGCGGCGTGGCTGCGCACTTCGGTGCGGC
GCATGTTGTAGCTGAAATCATCGTGTTCGGTAACGGATTTTGCATCAATCGGGCGCGTGA
AGCTCGGCCAGCCGCAGCCGGAATCATATTTGTCGGCGGAGCTGAACAAAGGTTCGCCGC
TGACAACGTCCACATAAATGCCGGGTTTGAACAAATGGTCGTATTCGTGGCTGAAGGCAT
ATTCGGTCGCGCTGTTTTGGGTAACTTGGTATTGCTCTTCGGTCAGGGTGCGTTTGAGTT
CGGCGTCACTCGGTTTTTTATACGTTGCCGCGTCGAAGCCTTTGCCTTGCGGGGCGGTCT
TGGTTTTGCCCGGCAGCGGTTCGTCAGCTTTGCGGATGTCGATGTGGCAGTAGCCGTTGG
GGTTTTTAATCAAGTAGTCCTGATGGTATTCCTCGGCATCGTAGAAGTTTTTCAGCGGCT
CGTTTTCAACAACGAGGGGCAGTTGGTATTTTTGCTGCTCGCGTTTGAGGGCGGCGGCGA
TGACGGCTTTTTCGGCGGGGTCGGTGTAGTACACGCCGCTGCGGTATTGCGTACCGGTGT
CGTTGCCCTGTTTGTTGAGGCTGGTCGGATCAACGACGCGGAAGAAATATTGCAGGATGT
CGTCTAGGCTGAGTTTGTCGGCATCGTAGGTCACTTTGACGGTTTCGGCGTGGCCCGTAT
GGCGGTAGGACACGTCTTCATAGCTCGGATTTTTCGTGTTGCCGTTGGCGTAGCCGGATA
CCGCGTCAACCACGCCGTCGATGCGTTGGAAATAGGCTTCCAAGCCCCAGAAGCAGCCGC
CGGCGAGGTAAATGGTGCGCGTGTTCATGATTTTTGAATCCTTTTTCTGAGTGTCGGGTT
TGTAGAACGAATGTTTCAAGCTGCCCAAATCGGCATTCGGGTCGCGGATTAACGCCAACG
CCTGCGCTTCGTTGATGCTGCCTTTGACGATGCGCTGCACGTCGCTGTCTTTACCGATTA
ACGCCCACGAGGGGTAAACGCTGATATTCAGGCTTTGGGCGATCGTGCCGCCGTTGTCGG
TTACGACGGGCAGCTTGGGATAATTCAAACCGGCATACCATTTTTGGAAGTCGCCGTCTT
TTTTCTCGTGCAAAAAGCCCGGGGAGGCGACGGTAATCAGGTTGGCGGAGCTGAATTTTG
CATCTTGCGCCCATTTTTGGTCTGTCCCAATTCGGACAGACACAAAGGACACCAGCTCG
CCCAAAATTTAATCAGCGTCGGTTTGTCTTTTTTCAAGTAAACACTGGCGGGGCGGTTGT
CCGCAGTTTTCAAAGTGGATAAAGTGTGCGGCACGGTCGCGGCTCCGGCATCGACGATTT
TGGGCGAACAAGCACCCAGCGCAAGCAGGCAGCCGAACTTGGCGCAAAGGGAAAAGAAAG
TACGGTGTTTCATTTTGATGTTTCCTGTGTGGACGGTTTGCATGATTAGACGTTTGAGAT
GCCGAAACCTTACAGCCCGGATTTTCAGACAACCTTACCGCGTAAAATACGCTACAATAC
GCCCTGTTTCAAGTTTCTAAAATTAAAAGGAAAATTCAATGTTCAGCTTCTTCCGTCGCA
AGAAAAAACAGGAAACGCCGGCTCTCGAGGAGGCTCAAATTCAGGAAACCGCAGCAAAAG
CAGAATCTGAACTTGCTCAAATAGTTGAAAATATTAAAGAAGATGCTGAATCTTTAGCAG
AAAGCGTCAAAGGGCAGGTCGAATCTGCCGTTGAAACCGTCAGCGGTGCGGTTGAACAGG
TAAAGGAAACCGTTGCCGAGATGCTGTCTGAAGCAGAGGAAGCGGCGGAAAAAGCAGCGG
AACAAGTCGAAGCGGCAAAAGAAGCCGTTGCCGAAACCGTCGGCGAGGCTGTCGGGCAAG
TTCAAGAAGCCGTTGCGACAACTGAAGAACACAAGCTCGGTTGGGCGGCGCGTTTGAAAC
AAGGCCTGACCAAATCGCGCGACAAAATGGCGAAATCGCTGGCGGGCGTGTTCGGCGGCG
GACAAATCGACGAAGATTTATACGAAGAGCTGGAAACCGTGCTGATTACCAGCGATATGG
GCATGGAAGCCACCGAATACCTGATGAAAGACGTGCGCGACCGCGTCAGCCTCAAAGGGC
TGAAAGACGGCAACGAATTGCGCGGCGCGTTGAAAGAAGCCTTGTACGACCTGATTAAGC
CTCTGGAGAAACCTTTGGTTTTGCCCGAAACCAAAGAGCCGTTTGTCATCATGCTTGCCG
GCATCAACGGCGCGGGCAAAACCACGTCTATCGGTAAACTCGCCAAATATTTCCAAGCGC
AGGGCAAATCCGTATTGCTGGCGGCAGGCGATACTTTCCGTGCCGCCGCGCGTGAGCAGC
TTCAAGCTTGGGGCGAGCGCAACAACGTAACCGTGATTTCGCAAACCACGGGCGATTCCG
CCGCCGTGTGCTTCGATGCCGTCCAAGCCGCCAAAGCGCGCGGCATCGACATTGTGCTGG
CCGACACCGCCGGCCGCCTGCCCACGCAGCTTCATTTGATGGAAGAAATCAAAAAAGTGA
AACGCGTGCTGCAAAAAGCCATGCCCGACGCGCCGCACGAAATCATCGTCGTGCTTGATG
CCAATATCGGGCAAAACGCCGTCAACCAAGTCAAAGCCTTTGACGACGCATTGGGGCTGA
CCGGTTTAATCGTTACCAAACTCGACGGCACGGCAAAAGGCGGCATCCTCGCCGCGCTTG
CCTCCGACCGCCCCGTTCCCGTCCGCTATATCGGCGTGGGCGAAGGCATAGACGACCTGC

## Appendix A

GCCCGTTTGACGCGCGCGCGTTTGTGGACGCACTGCTGGATTGAGCCGAAATGCCGTCCG
AAAACAGCAGACCGATGCCGTCATTCCCGCGCAGGCGGGAATCCAGACCTTGGGATAACG
GCAATATTCAAAGGTTATCTGAAAGTCCGAGATTCTGGATTCCCACTTTCGTGGGAATGA
CGGGATGTAGGTTCGTGGGAATGACGTGGTGCAGGTTTCCGTATGGATGGATTCGTCATT
CCCGCGCAGGCGGGAATCTAGAACGTAAAATCTAAAGAAACCGTGTTGTAACGGCAGACC
GATGCCGTCATTCCCGCGCAGGCGGGAATCTAGACCATTGGACAGCGGCAATATTCAAAG
ATTATCTGAAAGTCCGAGATTCTGGATTCCCACTTTCGTGGGAATGACGGGATTTGAGAT
TGCGGCATTTATCGGAAAAAACAGAAACCGCTCCGCCGTCATTCCCGCGCAGGCGGGAAT
CTAGGTTTGTCGGTGCGGAAACTTATCGGGTAAAACGGTTTCTTTAGATTTTGCGTTCTA
GATTCCCACTTTCGCGGGAATGACGAAGAGTTGCGGGAATGATGGAAAGCTATGGGAATA
ACGAAGGGTTAAAGTAATCACGGGATGGTGTTCGCGGGAATATAAATTGAAATAATTCAA
AAGGGTATTATATGCAGCCTGCGGTTTATATTTTAGCAAGCCAACGTAATGGCACGTTAT
ACATTGGCGTTACATCTGATTTGGTGCAACGTATTTACCAACATAGGGAGCATTTGATTG
AGGGATTTACATCACGGTACAACGTTACTATGCTGGTTTGGTATGAACTGCATCCTACGA
TGGAGAGTGCAATTACTCGGGAAAAACAGTTGAAGAAATGGAACAGGGCTTGGAAATTGC
AACTGATTGAAGAAAATAATGTTTCTTGGCAGGATTTATGGTTTGATATTATTTAGCCCG
TCATTCCCGCGCAGGCGGGAATCCGGCTTGTTCGGTTTCGGTTTTTTTTTTGAGGTTTCG
GGCAACTTCTAAACCGTCATTCCCGCGTAGGCGGGAATCTAGACCTTGGGATAACGGCAA
TATTCAAAGTTTATAAAAGACCCGTTATTCCCGCGCAGGCGGGAATCTAGACCTTAGAAC
AACAGTAATATTCAAAGGTTAGCTGAAGCTTTAGAGATTCTAGATTCCCACTTTCGTGGG
AATGACGGGATGTAGGTTCGCGGGAATGACGGGATTTGAGATTGCGGCATTTATCGGAAA
AAACAGAAACCGTTCTGCCGTCATTCCCGCGCAGGCGGGAATCCGGCTTGTTCGGTTTCG
GTTTTTTTGAGGTTTCGGGCAACTTCTAAACCGTCATTCCCGCGCAGGCGGGAATCTAGA
CCATTGGACAGCGGCAATATTCAAAGATTATCTGAAAGTCCGAGATTCTAGATTCCCACT
TTCGTGGGAATGACGGGATGTAGGTTCGTGGGAATGACGGGATTTGAGATTGCGGCATTT
ATCGGAAAAAACAGAAACCGCTCTGCCGTCATTCCCGCGCAGGCGGGAATCCGGCTTGTT
CGGTTTCGGTTTTTTTTTTTTTTGAGGTTTCGGGCAACTTCTAAACCGTCATTCCCGCGC
AGGCGGGAATCCAGACCATTGGACAGCAGCAATATTCAAAGATTATCTGAAAGTCCGGGA
TTCTAGATTCCCACTTTCGTGGGAATGACGGGATGTAGGTTCGTGGGAATGACGGGATTT
GAGATTGCGGCATTTATCGGAAAAACAGCAACCGCTCCGCCGTCATTCCCGCGCAGGCGG
GAATCTAGACCTTGGGATAACAGCAATATTCAAAGGTTAGCTGAAGCTTTAGAGATTCTG
GATTCCCACTTTCGTGGGAATGACGGAATGTAGGTTCGTGGGAATGACGGGATTTGAGAT
TGCGGCATTTATCGGAAAAACAGCAACCGCTCCGCCGTCATTCCCGCGCAGGCGGGAATC
TAGACCTTGGGATAACAGCAATATTCAAAGGTTAGCTGAAGCTTTAGAGATTCTGGATTC
CCACTTTCGTGGGAATGACGGAATGTAGGTTCGTGGGAATGACGGGATTAGAGTTTCAAA
ATTTATTCTAAATAGCTGAAACTCAACGCACTGGATTCCCGCCTGCGCGGGAATGACGAA
TTTTAGGTTTCTGATTTTGGTTTTCTGTTTTTGAGGGAATGACGGGATTTGAGATTGCGG
CATTTATCGGGAGCAACAGAAACCGCTCCGCCGTCATTCCCGCGCAGGCGGGAATCTAGA
CCTTAGAACAACAGCAATATTCAAAGGTTAGCTGAAGCTTTAGAGATTCTAGATTCCCAC
TTTCGTGGGAATGACGGAATGTAGGTTCGTGGGAATGACGCGGTGCAGGTTTCCGTATGG
ATGGGTTCGTCATTCCCGCGCAGGCGGGAATCCGGCTTGTTCGGTTTCGGTTTTTTTTTT
TTGAGGTTTCGGGCAACTTCTAAACCGTCATTCCCGCGCAGGCGGGAATCTAGACCTTAG
AACAACAGCAATATTCAAAGATTATAAAAGACCTGTCATTCCCGCGCAGGCGGGAATCTA
GGTCTGTCGGCACGGAAACTTATCGGGTAAACGGTTTCTTGAGATTCCGCGTCCTGGATT
CCCACTTTCGTGGGAATGACGGGATGTAGGTTCGTGGGAATGACGCGGTGCAGGTTTCCG
TATGGATGGGTTCGTCATTCCCGCGCAGGCGGGAATCTAGACCTTAGAATAACAGCAATA
TTCAAAGATTATCTGAAAGTCCGAGATTCTGGATTCCCACTTTCGTGGGAATGACGGAAT
GTAGGTTCGCGGGAATGACGCGGTGCAGGTTTCCGTGAGGATGGATTCGTCATTCCCGCG
CAGGCGGGAATCTAGACCTTAGAACAACAGCAATATTCAAAGATTATAAAAGACCTGTCA
TTCCCGCGCAGGCGGGAATCCAGACCTTAGAACAACAGCAATATTCAAAGGTTAGCTGAA
GCTTTAGAGATTCTGGATTCCCACTTTCGTGGGAATGACGGGATGTAGGTTCGTGGGAAT
GACGCGGTGCAGGTTTCCGTGCGGATGGATTCGTCATTCCCGCGCAGGCGGGAATCCAGA
CCTTGGGATAACAGCAATATTCAAAGGTTATAAAAGACCCGTCATTCCCGCGCAGGCGGG
AATCTAGACCTTAGAACAACAGTAATATTCAAAGGTTAGCTGAAGCTTTAGAGATTCTGG
ATTCCCACTTTCGTGGGAATGACGGGATTAGAGTTTCAAAATTTATTCTAAATAGCTGAA
ACTCAACGCACTGGATTCCCGCCTGCGCGGGAATGACGAATTTTAGGTTTCTGATTTTGG
TTTTCTGTTTTTGTAGGAATGATGAAATTTTGAGTTTTAGGAATTTATCGGAAAAAACAG
AAACCGCTCCGCCGTCATTCCCGCGTAGGCGGGAATCCAGACCGTTGGGCATCTGCAGCG
GTTTGCTAAAAACCGCTTTACTGTGATAAGTGCGCAGGGTTAGAATGGCGCGGTAACCTT
ATATATTGTACCCCGTCAAAGGGGCGCATTGCTTTTCTTAACATTCCCCTTTGGCAGCCA
AGTGAAAGGGCTTTTCAATCAGCAATTCGGCGGGCGCGGAATCGGGCGGTTTACCGAACC
CCGGCGTTCGCGGCGCGCCGCCCCGTCCCGTGAAGGCAAACTCAAGGAATAAAAGATGAA
TAAAACTTGGAAACGGCAGGTTTTCCGCCATACCGCGCTTTATACCGCCATATTGATGTT
TTCCCATACCGGCGGGGGGGGGGGCAGGCGCAAGCGCAAACGCAAACGCAAACGCATAAA
TACGCTATTGTAATGAACGCGCAAAATCTGCCCGAGGTAAAGTGGGGGGATCAATATCAG
TCATTGACGCACAAAAGCAATGAACGCGAAGTTATCCATACGAGTGGTTTTGGTTTGGCA
AAAAAGAGCATTAGTTTCTCATTCAATAATACCGATGAAGTTGTTGCTGAAAAAAAAGAT
ACTGTCGTTTTCGGCGCGGCGACCTACCTGCCGCCCTACGGAAAGGTTTCCGGTTTTGAT
ACCGCTAAGCTGACCGAGCGCAAAAATGCCCTTGATCAGATTGGTACGACCAAAACGGGG
CTGGTAGGCTACAGCTACGAAGGTAGCACATGCTCCAGCGGAGGTTGTCCTACAGTTGCC
TATAGAACCCAATTTACCTTCGGCAATTCCAGTTTGGCAAAAAAGGCAAACGGCGGCGGG
CTGGATATATACGAAGACAAAAGCCGCGACAATTCGCCCATTTACAAATTGAAGGATCAT
CCTTGGTTGGGCGTGTCTTTCAATTTGGGCGGAGAGAGCTCCTTCAAACCAAAGAGACAA
GGTTCTTTGGTATCTTCTTTTAGCGAGGACGTGACGCAGCAAAATGGTGCGGGCAGCCAA
CACAAAGACAAAAACCTCGTTTATACGACAGACGATTACAAGAGTCAGAATAATAAAAAC

## Appendix A

CATCAGGACAAACACCACGCCGTCGCCTTTTATCTGAACGCCAAGCTGCACCTGCTGGAT
AAAAAACACATTAAAAATATCGTGCAAGGTAAAACAGTTAATTTGGGTATCTTGAAAACA
CGCATCGAGCCGACGGAAGCATGGAAAAGACGGAATAGTAACTTTTTTAACGGTAGTTGG
ACGTATGAAGAGAAAGGAACAGTCAGCGTCAAACTCAAATTGCCGGAAGTCAAAGCAGGC
CGCTGCATCAACGCAAATAACCCCAATAAGAGTACCAAAGCCCCTTCCCCCGCACTGACT
GCCCCCGCGCTGTGGTTCGGACCTGTGCAAAATGGTAAGGTGCAGATGTATTCCGCTTCG
GTTTCCACCTACCCCGATAGTTCGAGCAGCCGCATCTTCCTTCAAAATCTGAAAAGAAAA
ACCGACCCCAACAAACCCGGCCGCCATTCCCTCGCAGACTTGGCTAAGTCGGATATTGAA
AATCGACAGCCGAATTTCACAGGGCGGCAAACCATCATCCGATTGGATGGCGGCGTACAG
CAGATCAAACTGGGTAGAAACAATGATGAGGTCGCCAATTTTAATGGAAATGACGGCAAA
AACGACACTTTCGGCATTGTTAGTGAAGGGAGCTTCATGCCTGATGCCAGCGAGTGGAAA
AAAGTATTGCTGCCTTGGACGGTTCGTGCTTCCAATGATGACGGTCAATTTAACACATTC
AACAAAGAAGAAAAAGACGGCAAGCCAAAATACAGCCAAAAATACCGCAGCCGCGACAAC
GGCAAGCACGAGCGCAATTTGGGCGACATCGTCAACAGCCCCATCGTGGCGGTCGGCGAG
TATTTGGCTACTTCCGCCAACGACGGGATGGTGCATATCTTCAAACAAAGCGGCGGGGAC
AAGCGCAGCTACAATCTGAAGCTCAGTTATATCCCGGGTACGATGCCGCGCAAGGATATT
CAAAACACCGAATCCACCCTTGCCAAAGAGCTGCGCGCCTTTGCCGAAAAAAGCTATGTG
GGCGACCGCTACGGCGTGGACGGCGGCTTTGTCTTGCGCAAAGTCGAACGGAACGGGAAA
GACCATGTGTTTATGTTCGGCGCGATGGGCTTTGGCGGCAGAGGCGCGTATGCCTTGGAT
TTAAGCAAAATCGACAGCGGCAACGGCAACCTGGCAGACGTTTCCCTGTTTGATGTCAAA
CATGACAAGAATGGCAATAACGGCGTGAAATTAGGCTACACCGTCGGCACGCCGCAAATC
GGCAAAACCCACGACGGCAAATACGCCGCTTTCCTCGCCTCCGGTTATGCGACTAAAGAC
ATTACCAGCGGCGACAATAAAACCGCGCTGTATGTGTATGATTTGGAAAGCAGCGGCACG
CTGATTAAAAAAATCGAAGTACCCGGTGGCAAGGGCGGGCTTTCGTCCCCCACGCTGGTG
GATAAAGATTTGGACGGCACGGTCGATATCGCCTATGCCGGCGATCGCGGCGGCAGTATG
TACCGCTTTGATTTGAGCAATCAAGATCCTAATCAATGGTCTGTACGCGCCATTTTTGAA
GGCACAAAACCGATTACTTCCGCGCCCGCTATTTCCCAACTGAAAGACAAACGCGTGGTT
ATCTTCGGCACGGGCAGTGATTTGAGTGAGGATGATGTACTCAGTACGAGCGAACAATAT
ATTTACGGTATCTTCGACGACGATACGGTGGCGAATAACGTAAATGTAAAACTCAGCGGT
TTGGGAGGCGGGCTGCTCGAGCAAGAGCTTAAGCAGGAGGATAAAACCTTATTCCTGACC
GATTACAAGCGATCCGACGGATCGGGCAGCAAAGGGTGGGTAGTGAAATTGAAGGGCGGA
CAGCGCGTTACCGTCAAACCGACCGTGGTATTGCGTACCGCCTTTGTAACCATCCATAAA
TATACGGGTACGGACAAATGCGGCGCGGAAACCGCCATTTTGGGTATCAATACCGCCGAC
GGCGGCAAGCTGACCAAGAAAAGCGCGCGCCCGATTGTGCCGGCCGAGAATCAGGCTGTC
GCGCAATATTCCGGCCATAAGAAAGGCATCAACGGCAAATCCATCCCTATAGGTTGTATG
CAAAAAGGCAATGAAATCGTCTGCCCGAACGGATATGTTTACGACAAACCGGTTAATGTG
CGTTATCTGGATGAAAAGAAAACAGACGGATTTTCAACAACGGCAGACGGCGATGCGGGC
GGCAGCGGTATAGACCCCGCCGGCAAGCGTTCCGGCAAAAACAACCGCTGCTTCTCCCAA
AAAGGGGTGCGCACCCTGCTGATGAACGATTTGGACAGCTTGGACATTACCGGCCCGACG
TGCGGTATGAAACGAATCAGCTGGCGTGAAGTCTTCTACTGATTTGCACGCGAAAATGCC
GTCCGAAAGGTTTTCGGACGGCATTTTTTGCGTTTTTCGGGAGGGGCGGGTTCGTAAAAG
GCGGGCTATAGGGTAGGCTTCATCTCGCCAATCTCACTGAATCCATCAATTTCCACAATT
CAATTAAATACCGTCAAACCGATGCCGTCATTCCCGCGCAGGCGGGAATCTAGACCTTAG
AACAACAGCAATATTCAAAGGTTAGCTGAAGCTTTAGAGATTCTGGATTCCCACTTTCGT
GGGAATGACGGGATGCAGGTTTCCGTATGAATGGATTCGTCATTCCCGCGCAGGCGGGAA
TCCAGACCTTAGAACAACAGTAATATTCAAAGATTATCTGAAAGTCCGAGATTCTGGATT
CCCACTTTCGTGGGAATGACGGGATTTTAGGTTTCTGATTTTGGTTTTCTGTTTTTGTAG
GAATGATGAAATTTTGAGTTTTAGGAATTTACCGGAAAAAACAGAAACCGTTCTGTCGTC
ATTCCCGCGCAGGCGGGAATCTAGACATTCAATGCTAAGGCAATTTATCGGGAATGACTG
~~AAACTCAAAAAACTGGATTCCCACTTTCGTGGGAATGACGGGATTTGAGATTGCGGCATT~~
TATCGGGAGCAACAGAAACCGCTCTGCCGTCATTCCCGCGCAGGCGGGAATCCAGACCTT
AGAACAACAGTAATATTCAAAGATTATCTGAAAGTCCGAGATTCTGGATTCCCGCCTGCG
CGGGAATGACGAATTTTAGGTTTCTGATTTTGTTTTTCTGTTTTTGTGGGAATGATGAAA
TTTTGAGTTTTAGGAATTTATCGGAAAAAACAGAAACCGCTCTGCCGTCATTCCCGCGCA
GGCGGGAATCTAGACCTTAGAACAACAGCAATATTCAAAGATTATCTGAAAGTCTGAGAT
TCTAGATTCCCACTTTCGTGGGAATGACGGGATGTAGGTTCGTGGGAATGACGTGGTGCA
GGTTCGTGGGAATGACGTGGTGCAGGTTCGTAGGAATGACGTGGTGCAGGTTTCCGTGCG
GATGGATTCGTCATTCCCGCGCAGGCGGGAATCTAGACCTTAGAACAACAGCAATATTCA
AAGGTTATCTGAAAGTCCGAGATTCTGGATTCCCACTTTCGTGGGAATGGCGCGATTAGA
GTTTCAAAATTTATTCTAAATAGCTGAAACTCAACGCACTGGATTCCCGCCTGCGCGGGA
ATGACGAAGTGGAAGTTACCCGAAACTTAAAACAAGTGAAACCGAACGAACCGGATTCCC
ACTTTCGTGGGAATGACGGGATGCAGGTTTCCGTACGGATGGATTCGTCATTCCCGCGCA
GGCGGGAATCTAGACATTCAATGCTAAGGCAATTTATCGGGAATGACTGAAACTCAAAAA
ACTGGATTCCCACTTTCGTGGGAATGACGGGATTAGAGTTTCAAAATTTATTCTAAATAG
CTGAAGCTCAACGCACTGGATTCCCGCCTGCGCGGGAATGACGAAGTGGAAGTTACCCGA
AACTTAAAACAAGCGAAACCGAACGAACTGGATTCCCATTGTCGTGGAAATGACGGGATT
TTAGGTTTCTGTTTTTGGTTTTCTGTTTTCGTGGGAATGACGGGATGTAGGTTCGTGGGA
ATGACGGTTCAGTTGCTACGCATTTACCCTGCGCAAAGCTTTATCCACTATCTTGTAACC
TGTCTGACAATCTGTCCTCTCTTACAAAATGCCGAAACTTTTTCAGGCTGCATTTTGGGG
CTGCCTGTGCGGAATTTGGCGGTAGGCGCGGTAGTAGGGTTCGAGCTGTCGGGCGATGAG
TTGGAGCTGTTGGAGGAGGATGTGGCTTTGTGTTCCGCTGCTGTGGGTGCGGAGGGTGTC
GAGTTCGCCGCGCAGTGTATCCAGTGCTGTCTGAAAGTCGTCGGGTTCGGTTTCGGGCAG
GTGTTGGAAGATGTGGGCGGTGTGTTCGGCGGCGAGGTGGAACTGTGCGGTAAAGTCGGG
GCTGCATTCTTCGTGCATTTCGCTGCGGTATGCGCCGAGGGCGGAGATGTAGCCGGTCAG
GGCGTAGCCGGTTTTGAGCAGGGTAAAGCCGGGTTGCAGGCTGTCGGCGAATTTTGCGGG

## Appendix A

TTCGCTGCTCATGTCGGAAAGGGTGCTGCTGAGGGCGGCGGTGTGTTCGTGGGCGCGGCG
GCGGGTGGCGCGGTATTCGACGTCGTCGCCGGTTTCGCCGCTTTTGAGGCGTTCGGTGAT
TTTTTCGAGATAGGCACCGTTGCTGCATACGGCAAGGGCGGCGGTGCGTTCGAGCGTGAG
GTATTTCCAGTCTGGCCACAGGTAGCTGACTGCCGCCCAGGCAAGGGATGCGCCGATAAT
GGTGTCGATGATGCGTACGGGCATGGCGGCGTATACGTCCAAACCTGCGAGGGAGAGGCT
GGTCAGGGCTTGAATGGTAATGAAGAAGGTGGAGAAACTGTATTTGTAGGTGCGGGTCAT
GAAAAAGAGGGTGGTACTGGCGATGACAATCCAGAGTTTGGTTTCGACAGACGGGGTGAA
GTAGGGGACGAGCGAGCCGACGATTACGCCGAGTACGGTGCCGGCGATGCGCTGGCGGAC
GCGGCTTTTGGTGGCGGTGTAGTTGGGTTGGCAGACGAAAAGGGCGGTCAGTAGTATCCA
GTAGCCGAGGTTGAGGTTGAGGGCTTCGACGATGGTGCAGGCGGCGGCAACGACGAGGGA
CAGGCGGACGGCATGGCGGAATACGCCTGATTCGAGGTTTAGCTGCGGACGGATTGCCTG
CCAGGTGTTTTTGAGGCTGCTGGTTTCGAGGGCGGCGATGCGGGTGTCGCCCATGCGGTC
GTTTTCTGCCTGCAGGCCGTTGTGCTGGAGTTGGCGGAACTGCTGGTCGACGCTGCCGAG
GTTGTCGAGAAGGCGGCGCAGGTGGCGGATGTCGGGACTGTCGTTGCTGTCTGAAAGGAG
GCGCAGCGATTGGCGGCAGCCTTCGATGGCGCGGCCGAGGCGTTTGCTGTAAACGTAGTC
TTTGCTTGCGCGCAGGGCTTGGGCGGTGTTGCGGCAGGCTTGTCCCTGCATTTCGAGCAG
GCGGTGGATGCGGAAGATGATGTCGGTGTTTTTGAATTTTTCGGACATTTCCTGATAATC
GACGTGGGCGGAGCTGATGCGTTCGTGTATGTCTTGGGCGGCAAAGTAGTAACGCAGCAT
TTTGGCGGTGCGCGGGTGGCGGTGTTTGCCGCGAAGGCGGTAAAACAGGGCGGAACGGCA
TTGGTTGAAGGCGGTGATGACGCCGGTGTTGCTCATGGCGAGGTCGATGTGGCGGTTGCC
TATCCAGGCTGCCTCATCGGGGTCGAAGAAGTCGGCTTTGGCTTCGAGGTAGCCGCCGAG
TGCGTCGTAGGCGTTGGCGACGCTTTCTTGGACGGGGCGGTGGGGCAGGACGATTTGGAA
CAGGAGGATGGCGGTGCTGTACAGTACGGTGCCGCATAAAATCATGAAGGGGTTGGTCAG
CCAGTAGGTTTCGGGGGTGTAGGTAAGTGTGGTGTAGGTGGCGACGGCGAGTGCACCGAA
GGCGAAGGTGCGGTATTTGAGCCCGACCGCGCCTAAAATGGTGAAGCCGAAGGTCATCAG
GGTCATGGCGAGGATGAAGGGCAGCCCTGTGCCGAGGGTGCTTTGTGCCGTGAGCGAGGA
GAGGGTGAACAGGGCGACGGTGGTGATGATGTTTTTCAGCCGTCCGGTCAGGCGGTTGTC
CAAATCGACAAGGCCGCCGGCGATGATGCCGAGTACGAAGGGCATGGCGAGCTTGGGTTC
GCCTAGCTGCCAGACGATGGAGGCGGCGGTAAAAACACTGGCGAAAACGGGAAGCGAGGT
AATGAGCAGAGGCTTGAGGAGTGGGGTTTTCATGGTTTTACCGGTTTATTGTTATGAAGT
GAATATAGTGGATTAACAAAAACCAGTACGGCGTTGCCTCGCCTTAGCTGAAAGAGAACG
ATTCTCTAAGGTGCTCAAGCACCAAGTGAATCGGTTCCGTACTATTTGTGCTGTCTGCGG
CTTCGTCGCCTTGTCCTGATTTTTGTTAATCCACTATAAATTTAATCCACTATAAAGTGT
AGCACATGAATGGGGCGGATAAAATCATGCCGTCTGAAAACGGGGATGCGGTTTTCAGAC
GGCATTGGGTTTTGCGGATCAGGAAATGAGGTTGAGACCGTTGACCCTGTCGTAAAGGAG
TTCGGGCGTTTTGCCTTCTTTGTGCAGTTGGATGTGCAATCGCAGGTTGTTGGCGGAAAC
GGACTGGCGCAGGGCTTCTTCGTAACTGATGATGCCGTGACGGTACAGTTCGAAAAGGTT
TTGATCCATCGTCTGCATTCCGTCGGTTTTGGCGGTTTCCATGATTTTACTGATGTTCAT
CAGGTCGCCCTTCAGGATGAAGTCTTGGATGGCGGGCGTGTTGATGAGCAAGTCGACAAC
CGCCGTCCTGCCCGTTTTGTCTTGTTTGAGGGCGAGGCGTTGGCAGATGATGCCGGTCAG
GTTGAGGGCGATGTCGATCAGTATTTGGTTGTGCTGTTCTTTGGGGTAGAAGTTGAGTAT
GCGTTCGAGCGACTGCGGCGCGGTGTTGGCGTGGAGCGTAAAAATGCACAGGTGGCCGGT
TTGGGCGAGCTGCATCGCGTATTCCATACTTTCCCTGCTGCGGACTTCGCCGATGCAGAC
CACGTCGGGGGATTGGCGCATAGCGTTTTGTACCGCCGTCTGCCAGTTTATGGTGTCGAC
GCCGATTTCGCGCTGGGTAAAGATGCAGCGGCGCGGTTTGTAGATAAATTCAATCGGGTC
TTCGATGGTAACGATATGGCTGGGCAGGGTTTTGTTGCGGTGTTCGAGCATAGTCGCCAT
CGTGGTGGATTTGCCCGAACCGGTAGGCCCGACGATAATCAGCAGCCCGCGCGGTGCGAC
GGCGAGGTCTTTGAGTTTTTCGGGCAGGCCCAATTCCTGCATTTGCGGGATGACGTGGTT
GATGCGCCGCAAAACCAAACCTGCGCTGCCTTGGCTGTGGTAGGCGTTGGCGCGGTAGCG
CGTGCCGCTGCGCGACTGGACGGAGTAGTTGATTTCGCCGTCGCGCCGGAATATTTCCGA
TTGTTCGGCGTTCATCGTCGATGCGGCGATGGCGGCGGTTTCCTCGCCCGTCAGCGCCTT
TTGCGGCTGCGGGGTTAATGCGCTGTTGATTTTCAACGAGGGCGGGAATCCTTTGCTGAT
AAGGATGTCGGACGCGTTTTGTGCTTCTGCGGTTTCGCACAGGCGGTCGAGCAGCGGGTG
GAAGTGTGCGCCGATTTCGGCCGGGGTTTCGGATCGGCTTTGTTTTTTTTGAGAATACAC
TTGAACCATTTCGTCCAAGATGTCGTGCAGGTTATCGGTATTCATCGTTAGCTTCTTTTC
GGTTTAAGCCTTGCAGTTTGCGGCGGCAGGTTTCAACAGGAAGGCGGACGCTTCTTGTTC
GGAAAGGTAGCCGGGCGGGATGCTGCGTCCCGCCCCGCGTGTTTGCGCCTTGTTTTCCCG
CCGGTATGGCCGGAAAGCGGTTGTGTGTCAGAAACTCATACTTTCGCTGTTTTGCGCGCG
TCTGCGTGCGACTTCCGGTGCGATCAGCCCTTGGCGCACCAGCGATTGCAGCGATTGGTC
CATTGTCTGCATACCGCTCGCCTGCCCGGTTTGCAGGACGGAGTTAATCTGCGTGATTTT
GTTTTCGCGGATGAGGTTGCGGACGGCGGGGTTGGCAATCAGGATTTCGTGCGAGGCGAC
ACGGCCGTTGCCGTCGTGCGTTTTCAGCAGGTTTTGGGAGATGACGGCGGTCAGCGATTC
GGACAGCATAGAGCGCACCATTTCTTTTTCTCCCGCCGGGAATACGTCCACAATACGGTC
GACGGTTTTTGCTGCGCCGGTCGTGTGCAGCGTGCCGAAAACCAAGTGTCCGGTTTCGGC
GGCGGTCAGTGCCAAGCCGATGGTTTCTGGGTCGCGCATCTCGCCGACAAGGATAACGTC
GGGGTCTTCGCGCAATGCGGAACGCAGCGCGTTGGCGAAGCTGAGGGTGTGCTGGTGCAG
CTCGCGCTGGTTAATCAGGGATTTTTTGCTTTGGTGGACGAATTCAATCGGGTCTTCGAT
GGTCAGGATGTGTGCCGGCTGGGTTTCGTTGATGTAGTTGATCATCGCGGCAAGCGTGGT
CGATTTGCCCGAACCGGTAGGGCCGGTAACCAAAACCATGCCGCGCGGCGATTCTGCGAT
TTTTTGGAAAATGCTCGGGGCTTTCAATTCTTCCAGCGATAAGACGGTGCTGGGAATGGT
GCGGAATACGGCGGCGGGACCGCGGCCGATGTTGAAGGCGTTGACGCGGAATCGGGCGAC
GTTGGGCAGTTCGAACGAGAAGTCGACTTCCAAGTTTTGCTGGTAGATTTTCCGCTGGTG
GTCGTTCATCACCGAAGTTACCATATTACCGACCTCTTCCGCGCTCATTTCGGGAAGGTT
GATGCGCCGCATATCGCCGTGAACCCGAATCATAGGGGATATGCCCGAACTCAGGTGAAG
GTCGGATGCTTTGTTTTTAGCGCCGAAGGCGAGTAAGTCGGTAATCTGCATAATGCGGCT

## Appendix A

CTGTTTAGTATAATGTTTCGATTGGTTGGAATGGTTCTAACAACCTTGATTGTACCGCCC
TGACTGGAGGGGTTTCAACTGTTTAATCATTTTTAATTAGGGGATAATCTATGACGGTGT
TGCAAGAACGTTATTGTGAGGTGTCCGACCGTATCGGAAAATTGGTTCTGCAGGCGGGCA
GGGAGCCGCATTCCGTCAGCCTGATTGCCGTCGGTAAGACTTTCCCTTCAGACGGCATCC
GCGAAGTTTACGCCGCCGGACAGCGTGATTTCGGCGAGAACTATATTCAGGAGTGGTACG
GCAAAACGGAAGAGTTGGCGGATTTGACCGACATCGTGTGGCACGTCATCGGCGATGTGC
AGTCCAACAAAACCAAGTTTGTCGCCGAACGCGCGCATTGGGTGCATACCGTATGCCGTC
TGAAAACCGCCGTCCGGCTGAGCGGGCAACGTCCTTCCTCAATGCCGCCTTTGCAGGTGT
GTATCGAGGTGAACATTGCGGGCGAGGCGGTGAAGCACGGTGTCGCGCCCGAAGAAGCAG
TCGCGCTTGCTGTGGAAGTGGCGAAGCTGCCGAATATCGTCGTACGTGGACTGATGTGTG
TTGCCAAAGCCAACAGCAGTGAAACGGAGTTGAAGGTGCAATTTCAAACGATGCGGAAAC
TGCTTGCCGACCTCAATGCGGCTGGCGTTAAGGCAGACGTGCTGTCTATGGGGATGTCGG
ACGATATGCCTGCCGCCATTGAGTGCGGTGCGACACACGTCCGTATCGGCAGCGCGATTT
TCGGGAAAAGGGGCTGATGGAAATTCGGGCAATAAAATATACGGCAATGGCTGCGTTGCT
TGCATTTACGGTTGCAGGCTGCCGGCTGGCGGGGTGGTATGAGTGTTCGTCCCTCACCGG
CTGGTGTAAGCCGAGAAAACCGGCTGCCATCGATTTTTGGGATATTGGCGGCGAGAGTCC
GCCGTCTTTAGGGGACTACGAGATACCGCTTTCAGACGGCAATCGTTCCGTCAGGGCAAA
CGAATATGAATCCGCACAACAATCTTACTTTTACAGGAAAATAGGGAAGTTTGAAGCCTG
CGGGCTGGATTGGCGTACGCGTGACGGCAAACCTTTGATTGAGACGTTCAAACAGGGAGG
ATTTGACTGCTTGGAAAAGCAGGGGTTGCGGCGCAACGGTCTGTCCGAGCGCGTCCGATG
GTAAAAAATTGGGAATGAATTTAGTAAGGTAATTTTGAATAGGGTAGAAATAATGAATGT
TTATTTTCTCGGCGGCGGCAATATGGCGGCTGCCGTTGCGGGCGGATTGGTCAAACAAGG
CGGTTACCGCATCTATATAGCCAATCGGGGTGCGGAAAAACGCGAACGTTTGGAAAAAGA
GTTGGGGGTCGAAACTTCGGCAACCCTGCCGGAGCTTCATTCCGACGATGTTTTAATCCT
TGCCGTCAAACCGCAGGATATGGAAGCTGCGTGCAAAAAATATCCGCACCAACGGCGCATT
GGTGCTTTCTGTCGCAGCCGGATTGTCGGTCGGTACGCTCAGCCGTTACCTCGGGGGAAC
ACGCCGCATTGTCCGGGTTATGCCGAATACACCCGGAAAAATCGGGCTGGGCGTATCTGG
TATGTATGCCGAAGCGGAAGTATCGGAAACAGACCGCAGGATTGCCGATCGAATCATGAA
ATCAGTCGGTTTGACTGTTTGGTTGGATGATGAGGAAAAAATGCACGGCATTACCGGCAT
CAGCGGCAGCGGACCGGCTTATGTGTTTTATCTGCTGGACGCATTGCAAAATGCCGCCAT
CCGACAAGGGTTTGATATGGCAGAAGCACGCGCGCTCAGTCTGGCAACGTTTAAAGGAGC
GGTTGCCCTTGCCGAGCAGACGGGTGAAGATTTCGAGAAGCTTCAAAAAAATGTAACGTC
AAAAGGCGGGACAACCCACGAAGCCGTGGAAGCTTTCAGGCGGCATCGTGTCGCCGAAGC
CATAAGCGAGGGCGTTTGTGCCTGTGTGCGCCGTTCGCAGGAAATGGAACGGCAATATCA
ATAATGTAAAGAAAATAAAAAAACCAATCCAAAACGTGTTATGATGCGCGTTTTCAAAAA
CGCCTTAGGCAATAAGCCTTATAAAAATCAAAGGAATAAAGCCACTTTGTGGTGCTTTGT
TTTTTGCGGTGAACCGAGAGGATATACATTATGGCAAAGCTGACAGAACAAGATATTTTG
AATTGGAGCGGGCCGGAAGACGATTATATGAATGACGACCATTTGGCTTTTTTCCGCGAA
TTGCTGGTAAAAATGCAAGACGAACTCATCGAAAATGCTTCCGCTACGACAGGGCATCTC
CAAGAACACGAATCAGCCCCCGATCCTGCCGACCGTGCCACACAGGAAGAAGAGTACGCA
TTGGAACTCCGTACCCGCGATCGGGAACGAAAACTTCTCAGTAAAATACAGGCGACCATC
CGCAATATTGATGAAGGGGATTATGGATTCTGTGCCGATACGGGAGAGCCTATCGGTTTG
AAGCGGCTGCTGGCACGCCCGACAGCCACTTTATCTGTTGAGTCCCAAGAACGCCGAGAG
AGGATGAAAAAACAGTTTGCCGACTGATGGCGGCAAACAAAATGCCGTCTGAGTCCCCGA
GTTTCAGACAGCATATTCACAAAGGCGCACCAGCCGGAGGAGGGAGAGGAAAGGATTGTT
GGAGGCGGCGCAGTATTTAGCAGAAATAAAAAACCTTATCCGACAGCGACATGACGAATT
TCCCCAAAAAAATCCCGCTGAAAGCATTGACCGTTTTTCCCTGTGGGCGTATAGTTCGGT
TCTTCGCTGCTGCAGAAGTGGCGGACGAACTGAAAAGTATAGCACAGAATGTTGGGGATA
TCGAGAGATATGTTGACAGGCGGAAGGAATACTTTATAATTCGCAACGCTCTTTAACAAA
ACAGATTACCGATAAGTGTGAGTGCCTTGAGTCTCACACTGTTTGAAAGACAGACAAGAT
AATGTTTTGAACATTGTCCTGTTGGTTTCTTTGAAGCAGACCAGAAGTTAAAAAGTTAGA
GATTGAACATAAGAGTTTGATCCTGGCTCAGATTGAACGCTGGCGGCATGCTTTACACAT
GCAAGTCGGACGGCAGCACAGAGAAGCTTGCTTCTCGGGTGGCGAGTGGCGAACGGGTGA
GTAACATATCGGAACGTACCGAGTAGTGGGGGATAACTGATCGAAAGATCAGCTAATACC
GCATACGTCTTGAGAGAGAAAGCAGGGGACCTTCGGGCCTTGCGCTATTCGAGCGGCCGA
TATCTGATTAGCTAGTTGGTGGGGTAAAGGCCTACCAAGGCGACGATCAGTAGCGGGTCT
GAGAGGATGATCCGCCACACTGGGACTGAGACACGGCCCAGACTCCTACGGGAGGCAGCA
GTGGGGAATTTTGGACAATGGGCGCAAGCCTGATCCAGCCATGCCGCGTGTCTGAAGAAG
GCCTTCGGGTTGTAAAGGACTTTTGTCAGGGAAGAAAAGGCTGTTGCTAATATCAGCGGC
TGATGACGGTACCTGAAGAATAAGCACCGGCTAACTACGTGCCAGCAGCCGCGGTAATAC
GTAGGGTGCGAGCGTTAATCGGAATTACTGGGCGTAAAGCGGGCGCAGACGGTTACTTAA
GCAGGATGTGAAATCCCCGGGCTCAACCCGGGAACTGCGTTCTGAACTGGGTGACTCGAG
TGTGTCAGAGGGAGGTAGAATTCCACGTGTAGCAGTGAAATGCGTAGAGATGTGGAGGAA
TACCGATGGCGAAGGCAGCCTCCTGGGACAACACTGACGTTCATGCCCGAAAGCGTGGGT
AGCAAACAGGATTAGATACCCTGGTAGTCCACGCCCTAAACGATGTCAATTAGCTGTTGG
GCAACCTGATTGCTTGGTAGCGTAGCTAACGCGTGAAATTGACCGCCTGGGGAGTACGGT
CGCAAGATTAAAACTCAAAGGAATTGACGGGGACCCGCACAAGCGGTGGATGATGTGGAT
TAATTCGATGCAACGCGAAGAACCTTACCTGGTCTTGACATGTACGGAATCCTCCGGAGA
CGGAGGAGTGCCTTCGGGAGCCGTAACACAGGTGCTGCATGGCTGTCGTCAGCTCGTGTC
GTGAGATGTTGGGTTAAGTCCCGCAACGAGCGCAACCCTTGTCATTAGTTGCCATCATTC
AGTTGGGCACTCTAATGAGACTGCCGGTGACAAGCCGGAGGAAGGTGGGGATGACGTCAA
GTCCTCATGGCCCTTATGACCAGGGCTTCACACGTCATACAATGGTCGGTACAGAGGGTA
GCCAAGCCGCGAGGCGGAGCCAATCTCACAAAACCGATCGTAGTCCGGATTGCACTCTGC
AACTCGAGTGCATGAAGTCGGAATCGCTAGTAATCGCAGGTCAGCATACTGCGGTGAATA
CGTTCCCGGGTCTTGTACACACCGCCCGTCACACCATGGGAGTGGGGATACCAGAAGTA

## Appendix A

GGTAGGATAACCACAAGGAGTCCGCTTACCACGGTATGCTTCATGACTGGGGTGAAGTCG
TAACAAGGTAGCCGTAGGGGAACCTGCGGCTGGATCACCTCCTTTCTAGAGAAAGAAGAG
GCTTTAGGCATTCACACTTATCGGTAAACTGAAAAAGATGCGGAAGAAGCTTGAGTGAAG
GCAAGATTCGCTTAAGAAGAGAATCCGGGTTTGTAGCTCAGCTGGTTAGAGCACACGCTT
GATAAGCGTGGGGTCGGAGGTTCAAGTCCTCCCAGACCCACCAAGAACGGGGGCATAGCT
CAGTTGGTAGAGCACCTGCTTTGCAAGCAGGGGGTCATCGGTTCGATCCCGTTTGCCTCC
ACCAATACTGTACAAATCAAAACGGAAGAATGGAACAGAATCCATTCAGGGCGACGTCAC
ACTTGACCAAGAACAAAATGCTGATATAATAATCAGCTCGTTTTGATTTGCACAGTAGAT
AGCAATATCGAACGCATCGATCTTTAACAAATTGGAAAGCCGAAATCAACAAACAAAGAC
AAAGCGTTTGTTTTGATTTTTTATTCTTTGCAAAGGATAAAAATCTCTCGCAAGAGAAAA
GAAAACAAACACAGTATTTGGGTGATGATTGTATCGACTTAATCCTGAAACACAAAAGGC
AGGATTAAGACACAACAAAGCAGTAAGCTTTATCAAAGTAGGAAATTCAAGTCTGATGTT
CTAGTCAACGGAATGTTAGGCAAAGTCAAAGAAGTTCTTGAAATGATAGAGTCAAGTGAA
TAAGTGCATCAGGTGGATGCCTTGGCGATGATAGGCGACGAAGGACGTGTAAGCCTGCGA
AAAGCGCGGGGGAGCTGGCAATAAAGCAATGATCCCGCGATGTCCGAATGGGGAAACCCA
CTGCATTCTGTGCAGTATCCTAAGTTGAATACATAGACTTAGAGAAGCGAACCCGGAGAA
CTGAACCATCTAAGTACCCGGAGGAAAAGAAATCAACCGAGATTCCGCAAGTAGTGGCGA
GCGAACGCGGAGGAGCCTGTACGTAATAACTGTCGAGATAGAAGAACAAGCTGGGAAGCT
TGACCATAGTGGGTGACAGTCCCGTATTCGAAATCTCAACAGCGGTACTAAGCGTACGAA
AAGTAGGGCGGGGCACGTGAAATCCTGTCTGAATATGGGGGGACCATCCTCCAAGGCTAA
ATACTCATCATCGACCGATAGTGAACCAGTACCGTGAGGGAAAGGCGAAAAGAACCCCGG
GAGGGGAGTGAAACAGAACCTGAAACCTGATGCATACAAACAGTGGGAGCGCCCTAGTGG
TGTGACTGCGTACCTTTTGTATAATGGGTCAACGACTTACATTCAGTAGCGAGCTTAACC
GAATAGGGGAGGCGTAGGGAAACCGAGTCTTAATAGGGCGATGAGTTGCTGGGTGTAGAC
CCGAAACCGAGTGATCTATCCATGGCCAGGTTGAAGGTGCCGTAACAGGTACTGGAGGAC
CGAACCCACGCATGTTGCAAAATGCGGGGATGAGCTGTGGATAGGGGTGAAAGGCTAAAC
AAACTCGGAGATAGCTGGTTCTCCCCGAAAACTATTTAGGTAGTGCCTCGAGCAAGACAC
TGATGGGGGTAAAGCACTGTTATGGCTAGGGGGTTATTGCAACTTACCAACCCATGGCAA
ACTAAGAATACCATCAAGTGGTTCCTCGGGAGACAGACAGCGGGTGCTAACGTCCGTTGT
CAAGAGGGAAACAACCCAGACCGCCAGCTAAGGTCCCAAATGATAGATTAAGTGGTAAAC
GAAGTGGGAAGGCCCAGACAGCCAGGATGTTGGCTTAGAAGCAGCCATCATTTAAAGAAA
GCGTAATAGCTCACTGGTCGAGTCGTCCTGCGCGGAAGATGTAACGGGGCTCAAATCTAT
AACCGAAGCTGCGGATGCCGGTTTACCGGCATGGTAGGGGAGCGTTCTGTAGGCTGATGA
AGGTGCATTGTAAAGTGTGCTGGAGGTATCAGAAGTGCGAATGTTGACATGAGTAGCGAT
AAAGCGGGTGAAAAGCCCGCTCGCCGAAAGCCCAAGGTTTCCTGCGCAACGTTCATCGGC
GTAGGGTGAGTCGGCCCCTAAGGCGAGGCAGAAATGCGTAGTCGATGGGAAACAGGTTAA
TATTCCTGTACTTGATTCAAATGCGATGTGGGGACGGAGAAGGTTAGGTTGGCAAGCTGT
TGGAATAGCTTGTTTAAGCCGGTAGGTGGAAGACTTAGGCAAATCCGGGTCTTCTTAACA
CCGAGAAGTGACGACGAGTGTCTACGGACACGAAGCAACCGATACCACGCTTCCAGGAAA
AGCCACTAAGCTTCAGTTTGAATCGAACCGTACCGCAAACCGACACAGGTGGGCAGGATG
AGAATTCTAAGGCGCTTGAGAGAACTCAGGAGAAGGAACTCGGCAAATTGATACCGTAAC
TTCGGGAGAAGGTATGCCCTCTAAGGTTAAGGACTTGCTCCGTAAGCCCCGGAGGGTCGC
AGAGAATAGGTGGCTGCGACTGTTTATTAAAAACACAGCACTCTGCTAACACGAAAGTGG
ACGTATAGGGTGTGACGCCTGCCCGGTGCTGGAAGGTTAATTGAAGATGTGAGAGCATCG
GATCGAAGCCCCAGTAAACGGCGGCCGTAACTATAACGGTCCTAAGGTAGCGAAATTCCT
TGTCGGGTAAGTTCCGACCCGCACGAATGGCGTAACGATGGCCACACTGTCTCCTCCTGA
GACTCAGCGAAGTTGAAGTGGTTGTGAAGATGCAATCTACCCGCTGCTAGACGGAAAGAC
CCCGTGAACCTTTACTGTAGCTTTGCATTGGACTTTGAAGTCACTTGTGTAGGATAGGTG
GGAGGCTTAGAAGCAGAGACGCCAGTCTCTGTGGAGCCGTCCTTGAAATACCACCCTGGT
GTCTTTGAGGTTCTAACCCAGACCCGTCATCCGGGTCGGGGACCGTGCATGGTAGGCAGT
TTGACTGGGGCGGTCTCCTCCCAAAGCGTAACGGAGGAGTTCGAAGGTTACCTAGGTCCG
GTCGGAAATCGGACTGATAGTGCAATGGCAAAAGGTAGCTTAACTGCGAGACCGACAAGT
CGAGCAGGTGCGAAAGCAGGACATAGTGATCCGGTGGTTCTGTATGGAAGGGCCATCGCT
CAACGGATAAAAGGTACTCCGGGGATAACAGGCTGATTCCGCCCAAGAGTTCATATCGAC
GGCGGAGTTTGGCACCTCGATGTCGGCTCATCACATCCTGGGGCTGTAGTCGGTCCCAAG
GGTATGGCTGTTCGCCATTTAAAGTGGTACGTGAGCTGGGTTTAAAACGTCGTGAGACAG
TTTGGTCCCTATCTGCAGTGGGCGTTGGAAGTTTGACGGGGGCTGCTCCTAGTACGAGAG
GACCGGAGTGGACGAACCTCTGGTGTACCGGTTGTAACGCCAGTTGCATAGCCGGGTAGC
TAAGTTCGGAAGAGATAAGCGCTGAAAGCATCTAAGCGCGAAACTCGCCTGAAGATGAGA
CTTCCCTTGCGGTTTAACCGCACTAAAGAGTCGTTCGAGACCAGGACGTTGATAGGTGGG
GTGTGGAAGCGCGGTAACGCGTGAAGCTAACCCATACTAATTGCTCGTGAGGCTTGACTC
TATCATTTGAAGAACTTCAAGAGATAAAAGCTTACTGACTGATTCAGTCATTACCGAATA
TATTGATTAAGGCTTTACCGATTTGTAACAGTTTAAGTTTGGCGGCCATAGCGAGTTGGT
CCCACGCCTTCCCATCCCGAACAGGACCGTGAAACGACTCAGCGCCGATGATAGTGTGGT
TCTTCCATGCGAAAGTAGGTCACTGCCAAACACCCATTCAGAAAACCCCCGATTATTCGG
GGGTTTTTGCTTTGCCCGGAAAAAATGTTTGCTTTGCCCGGAAAAAATGTCGGTGATGGC
GGGACGGCATCCGTACGGTGTCCGGTCGGGTTTGCGGAGGAACGGCTTGAAACTTTGGGA
TATTCATTTTAGAATGACTCGTTTTATCGTCGCAAGATGCGGTTTATTGTTTGCAACCCT
TAAAGGAAAAACCATGAAGAAAATGTTCGTGCTGTTCTGTATGCTGTTCTCCTGCGCCTT
CTCCCTTGCGGCGGTAAACATCAATGCGGCTTCGCAGCAGGAGTTGGAGGCGCTGCCAGG
CATAGGCCCTGCGGTGCTGGCGAAGCTGAAGGATCAGGCTTCCGTCGGCGCGCCCGCACC
AAAAGGCCCAGCCAAACCAGTGCTGCCCGCGGATAAAAAATAAAATAGGGGGAAGTCTGC
AGCCGCATCAAATGCCGTCTGAACATGCGTTCGGGCGGCGTTTTTATAACAAAAACACTT
CATGGCGGTTGGTTTTATGCCTATCTAAGTTTTTGTGTCGTGCATACCTGAAGATTTCAG
ACGGCATCGGTTTATGCTGTCTGAAAAGTGTATTCCGTTTCAGTTTGTAAGCTATGGCAG

## Appendix A

TCTGTTTGTCTTGTGTTTTGCGCAATTGCCCTTATTTTGAGCCGTGATTTTATTTTGAAT
TAGATGAAAAAATGAGTAATCAAGATTTTTATGCGACGCTGGGTGTGGCAAGAACAGCTA
CCGATGATGAGATTAAAAAAGCCTACCGGAAATTGGCGATGAAATACCATCCCGACCGCA
ATCCTGACAATAAAGAGGCGGAAGAGAAGTTTAAAGAAGTACAAAAGGCGTATGAAACTT
TGTCCGACAAGGAAAAGCGCGCTATGTACGACCAGTATGGTCATGCGGCGTTTGAAGGCG
GCGGACAGGGGGGCTTCGGAGGGTTTGGCGGATTTGGCGGTGCGCAGGGTTTTGACTTTG
GGGATATTTTCAGCCAAATGTTTGGAGGCGGTTCGGGGCGCGCCCAGCCTGATTATCAGG
GTGAGGACGTTCAAGTCGGTATCGAAATCACGCTTGAAGAAGCCGCAAAAGGTGTGAAGA
AACGCATCAATATTCCGACTTATGAAGCGTGTGATGTCTGTAACGGCAGTGGCGCGAAAC
CGGGGACATCCCCGGAAACCTGCCCGACTTGCAAAGGTTCGGGTACGGTGCACATCCAGC
AGGCGATTTTCCGTATGCAGCAGACTTGTCCGACCTGCCACGGTGCGGGCAAACACATTA
AAGAACCTTGCGTCAAATGCCGTGGCGCGGGGCGGAATAAGGCGGTCAAGACGGTGGAAG
TCAATATTCCCGCCGGTATCGATGACGGGCAGCGTATCCGTTTGAGCGGCGAAGGCGGGC
CGGGTATGCACGGTGCGCCTGCCGGCGACTTGTATGTAACCGTCCGCATTCGGGCGCATA
AGATTTTCCAACGCGACGGTCTGGACTTGCATTGCGAACTGCCGATCAGTTTTGCCACGG
CTGCTTTGGGCGGGGAGTTGGAAGTGCCGACCTTGGACGGAAAGGTCAAGCTCACCGTCC
CCAAAGAAACCCAAACCGGCAGGAGGATGCGCGTGAAGGGTAAGGGTGTCAAATCTTTAC
GCAGCAGCGCGACCGGCGATTTGTACTGCCATATTGTTGTCGAAACGCCTGTCAATTTGA
CCGACCGTCAAAAAGAGCTTTTGGAAGAATTTGAGCGGATTTCTACCGGCTTGGAAAACC
AAACACCGCGCAAGAAATCGTTTTTAGACAAGCTGCGCGATTTGTTTGATTGATTTTAAG
GTTCGGAAACAAGCAGCCGTATCGGGGAATCTCCTTGATACGGCTGTTTTTATTTGTTTA
AAAATAGTTTTTATTTTCAATGGGGTATGAGGCAGGGTGGGATAACTGTTTTTAACTGTT
CTTTTTAAAACTTGACATCATGGCGTGATGCCAACAATATGTGAACGTCTGTTGTCAAAG
GAAGAATAATGAATAAATCTTTATCCAGTTCGGTAGAAGAATACCGCGAGCTGACGCTCC
GAGGCATGATACTCGGTGCATTGATCACTGTAATTTTTACTGCGTCCAATGTTTACCTCG
GTTTGAAAGTCGGGCTGACCTTTGCCTCGTCGATTCCGGCGGCGGTGATTTCGATGGCGG
TTTTAAAGTTTTTCAAAGGCAGCAATATTTTGGAAAACAACATGGTGCAGACCCAAGCCT
CGGCTGCGGGTACGCTTTCGACCATCATCTTCGTCCTGCCCGGTTTGCTGATGGCGGGCT
ACTGGAGCGGTTTCCCGTTCTGGCAGACGACGCTTTTATGTATTGCCGGCGGGATTTTGG
GGGTGATTTTCACCATTCCTCTGCGTTACGCAATGGTGGTGAAAAGCGATTTGCCTTATC
CGGAAGGTGTGGCGGCTGCTGAAATTTTGAAAGTGGGCGGTCATGAAGAAGGGGATAACC
GTCAGGGCGGCAGCGGCATCAAAGAGCTGGCGGCCGGCGGTGCGTTGGCGGGATTGATGA
GCTTTTGCGCCGGAGGTCTGCGCGTGATTGCCGACAGCGCGAGTTATTGGTTTAAAAGCG
GTACGGCGATTTTCCAGCTGCCGATGGGCTTTTCACTGGCATTGTTGGGCGCGGGCTATT
TGGTCGGACTGACGGGCGGTATCGCCATCCTGTTGGGCATTTCGATTGCTTGGGGCATTG
CCGTGCCGTATTTCTCCTCACACATTCCGCAACCTTCCGATATGGAAATGGCGGCGTTTG
CGATGAAGCTGTGGAAGGAGAAAGTGCGTTTTATCGGTGCGGGGACTATTGGCATTGCGG
CGGTTTGGACGCTGTTGATGCTGCTCAAGCCGATGGTGGAAGGCATGAAGATGTCGTTCA
AGAGTTTTGGCGGCGGTGCGCCCGCTGCGGAACGCGCCGAACAGGATTTGTCGCCTAAGG
CTATGATTTTTTGGGTGCTGGCGATGATGTTTGTTTTAGGCGTGTCGTTTTACCACTTTA
TCGGCGATTCGCACATTACGGGCGGCATGGCTTGGCTTTTGGTGGTCGTTTGCACGCTTT
TGGCTTCCGTCATCGGCTTTTTGGTCGCCGCCGCCTGCGGTTATATGGCAGGTTTGGTCG
GCTCGTCTTCCAGCCCGATTTCCGGCGTGGGCATCGTGTCCGTCGTCGTTATTTCACTGG
TTTTGCTGCTGGTAGGCGAATCCGGAGGTTTGTTGGCGGATGAGGCTAACCGCAAATTTT
TGCTGGCACTGACTTTGTTTTGCGGCTCGGCAGTAATCTGCGTGGCTTCGATTTCCAATG
ACAACCTGCAAGACTTGAAAACCGGCTACCTGCTCAAAGCCACGCCTTGGCGGCAGCAAG
TCGCCCTGATTATCGGCTGTATCGTTGGTGCGCTGGTTATTTCGCCCGTGTTGGAACTGC
TTTACGAAGCCTACGGCTTTACCGGCGCAATGCCGCGCGAAGGCATGGACGCGGCGCAGG
CTTTGGCAGCCCCTCAAGCGACTTTGATGACGACCATCGCGTCGGGCATTTTCGCCCACA
~~ACCTTGAATGGGTCTATATCTTTACCGGTATCGTGATTGGAGCAGTATTAATCGTCGTCG~~
ATTTGGTGTTGAAAAAATCATCAGGCGGCAAACTTGCCCTGCCCGTCCTTGCGGTCGGTA
TGGGTATTTATCTGCCGCCGTCCGTCAATATGCCCATCGTGGCAGGCGCGGTGTTGGCGG
CGGTGTTGAAACACATCATCGGTAAAAAAGCGGAAAACCGCGAAGGCCGTCTGAAAAACG
CCGAGCGCATCGGAACCTTGTTCTCCGCCGGCCTGATTGTCGGTGAAAGCCTGATCGGTG
TGATTATGGCGTTTATTATTGCCTTCTCCGTGACCAACGGCGGCTCGGATGCGCCGCTCG
CGTTGAATCTGCAAAACTGGGATGCCGCCGCTTCTTGGCTGGGTTTGGCGTTCTTCGTTA
CCGGGATGTTTTTCTTTGCACAGCGCGTACTGAAGGCGGGCAAGTAGGCTGTCGGAAAAA
ATGCCGTCTGAAACGTTCAGACGGCATTTTTTATCGGTAAAGCGGAAGGCGGAGCTTTTC
GGCTTGCGCCCACGTTTTGCCGGCAAGGTCTTTGGGCGACAGCAGCGGCGCGGTTTGAAG
CGGCCAGCCTATGCCGACTGTCGGGTCGTTCCATATTAAAACCTGTTCGGCTTCAGGCTT
GTAATAGTCCGTGCATTTATAGACGAACTCGGCTTCATCGCTCAGTACATAGAAGCCGTG
TGCGAAACCTTCGGGTACCCACAGTTGGCGTTTGTTTTCTGCGGACAGAATTTCGCCTAC
CCATTTGCCGAAAGTGGGGGAGTCTTTACGCATATCGACGGCCACGTCGAATACTTCGCC
GACAACCACGCGTACGAGTTTGCCTTGTGTGTTTTCAGTTTGATAGTGCAGGCCGCGCAA
TACGCCTTTGCCGGATTTGGAGTGGTTTTCCTGCACGAAGGTGCGTTCGCAGACTTGGGT
TTTAAACCACTCGTCGCGGAAGGTTTCCATAAAAAAGCCGCGCGCGTCGCCGAAGACTTG
GGGCTCAAGCAGTTTTACGTCAGGAATGGCGGTATCAATGATGTTCATCTTTTTATCTTT
CATCTAAAGGCCGTCTGAAAAGTTTCAGACGGCCTCAAACATTATTTTTTCAACAGGCGC
AGCAAATATTGGCCGTATTGGTTTTTCGCCATCGGGCGCGCCAATTCTTCCAGTTTTTCA
TCGGAAAGCCAACCGTTGCGCCAAGCGATTTCTTCGAGGCAGGCGATGTGCAGGTTTTGG
ATATTTTGCACGGTTTGGACGAATGAAGCGGCTTCGTGCAGGCTCTCGTGGGTGCCGGTG
TCCAGCCACGCGAAACCGCGTCCCAATATTTGAACGGAGAGCGAGCCGTCTTCCAAATAC
ATCCGGTTGAGGTCGGTAATTTCCAATTCGCCGCGTGCGGACGGTTTGAGCTGTTTGGCG
AACTCGACGGCGCGGTTGTCGTAGAAATACAAGCCGGTTACCGCCCAATCGGATTTGGGC
CGTTGCGGTTTTTCTTCGATGGAAACGGCGCGGAAGTTTTCGTTAAATTCAACCACGCCG

## Appendix A

AAACGTTCGGGGTTTTTGACCTGATAAGCAAACACGGTTGCGCCGTGCGTTTGCGCTGCC
GCCTGTTTCAATGTTTGCGTAAACGACTGACCGTAAAAAATATTGTCGCCCAAAACCAAG
CAAACATTGTCGTTGCCGATAAATTCTTCGCCGATGATAAATGCCTGTGCCAAGCCGTCC
GGACTGGGTTGCACGGCATAACTGATGGAAATGCCGAAATCGCTGCCGTCGCCAAGCAGG
CGTTTGAAAGAGGCGTTGTCTTCAGGCGCGGTAATCACCAAAATATCGCGGATTCCCGCC
AGCATCAAAACCGACAAGGGGTAATAAATCATCGGTTTGTCGTACACGGGCAGGAGCTGT
TTGGATACGCCGCGCGTGATGGGGTAGAGGCGCGTGCCGCTGCCGCCTGCCAGTATGATG
CCTTTCATCTTTTCTTTCTTCCTTTGCGATGGGTTTTCAGACGGCATTGCGTCGGGATGC
CGTCTGAAAACTATTTTCCAGTACCTAAACGTTCCAAACGATAGCTGCCGTTCAATACAT
TTTGCCACCAGGTTTTGTTGTCCAGATACCATTGCACGGTTTTGCGGAGGCCGGACTCGA
AGGTTTCCAAAGGCAGCCAGCCCAAATCCCGCCTGATTTTGGCTGCGTCGACGGCGTAGC
GTACGTCATGGCCGGGGCGGTCTTGTACGAAAGTAATCAAATCTTCATAACGCGCCACAC
CGGCCGGTTTTTCGGGAGCGAGTTCTTCCAGCAGGGCGCAGATGGTTTTGACGACTTCAA
TATTGGCTTTTTCATTGTGGCCGCCGATATTGTAGGTTTCGCCGACAACACCTTCGGTAA
CAACCTGATACAGTGCGCGCGCGTGGTCTTCGACAAACAGCCAGTCGCGGATTTGCATAC
CGTCGCCGTACACAGGCAGCGGTTTGCCGTCAAGCGCGTTCAGAATCATCAAAGGAATGA
GTTTTTCCGGAAAATGGTAAGGACCGTAGTTGTTGGAGCAGTTGGTTACAATGGTCGGCA
AGCCGTAAGTACGCAACCACGCGCGGACGAGGTGGTCGCTGGACGCTTTAGAGGCAGAGT
AGGGGCTGGACGGCGCGTAGGGCGCGGTTTCGGTAAACAAATCGTCCGTGCCGCCTAAAT
CGCCATAGACTTCATCGGTGGAAATATGGTGGAAACGGAAGGCTTCGTGCTGTTCAGACG
GCATTTGTTGCCAGTAGGCGCGGGCTGCTTCAAGCAGATTGAATGTGCCGACGATATTGG
TTTGGATAAACTCGCCTGCCGAACCGATAGAGCGGTCGACATGGCTTTCCGCCGCCAAGT
GCATCACGGCATCAGGCCGGTATTGCGCGAATACGCGGTCGAGTTCGGCGCGGTCGCAAA
TATCCACTTGTTCAAAAGCATAGCGAGGATTATCGGCTACCTCAGTCAAAGATTCCAAAT
TGCCGGCATAAGTCAGCTTATCGACATTGACGACAGCGTCCCGGGTGTTTCGGATAATAT
GACGGACAACGGCAGAACCGATAAAGCCCGCGCCGCCGGTAACAAGGATTTTTCTCATAA
ATTTCAGAGGATAGCCAAAAAATATAAACAGATTATAGCAGACAGAATGTGTGTTTTTCA
GATAAAGAGGCCGTCTGAAAACATCTCTTTCAGACGGCCTGTATCAGGTCAACTTAATCG
TCGTAGCCATTCGGATTATTACTCACCCAGCGCCATGAGTCTTCCATCATTTGGGTTAAA
TCACGCTGGGTTTGCCAGCCGATTTGCGCCTTTGTATAGGAAGGGTCGGCATAGAAGCAC
GCCAAATCACCGGCACGGCGCGGTTTGACTTCATACGGAATCGTCAAACCCGAAGCTGCT
TCAAATGCGCGGATGATTTCCAACACCGAAGAAGCGCGGCCGGAGCCTAAGTTCAGCAAA
TGCGTGCCTGCTACATTACTTTTTGCCTGCATAGCCGCGACATGGCCTTCTGCCAAATCC
ATCACATGAATATAGTCACGCATCCCCGTGCCGTCGGGGGTAGGGTAGTCATCGCCAAAT
ACCGCCAATTGCGGCAGTTTGCCTGCCGCCACTTGGCAGATATAAGGCAACAAATTATTC
GGGATGCCGTTTGGCTGCTCGCCAATCAAGCCGCTTTCATGCGCGCCAATCGGATTGAAA
TAACGCAACAAAATCATGCTCCAGCGCGGATCGGCTTTTTGAATGTCAGTGAGAATGCGC
TCAACCATCGATTTCGATGCGCCGTAAGGGCTGGTGGTGTCGCCCGGTGGCATATCCTCG
GTATAAGGCACTTTGCCCGGATCGCCATAAACCGTCGCCGAAGAACTGAACACAATGCTA
AACACGCCCGCACGCGCCATTTCTTCCGCCAACACCAAGCTGCCGGAAACATTATTATCA
TAATATTTCATCGGCTCGGCCACACTTTCACCCACCGCTTTCAAGCCGGCAAAATGAATC
ACCGAATCAATGCGGTTTTCCGCAAAAATACGGCGCAAAATCTCACGATCGCGGATATCG
CCTTGATAAAACGGAATCTCTTGGCCGGTAATCGTTTTCAAGCGTGGCAGGATATTGATG
CTGGAATTGCATAGGTTATCCAAAATCACGACTTGATGGCCGCTTTTCAGCAAAGAAACA
ACGGTATGCGAGCCGATAAAACCGGTGCCGCCGGTAACGAGAATTTTTTTCATAGAATAA
AATACTAAAAATACTTTGATAGATTGATAATAATGGTTGTAAAATCTTAATGAAATAATT
ATCCCTGAAGTAGCAGTAGATTTCTTCAGATTTTTTTGGTTAAGTATATTTGATATCTAA
GGTAAAATACTATAATTTTATTCATATGGTGTAGAATTAAGGGAAAATAGTGAAAAAAGT
ATTACTAATTGCCAGTTATGACTCGTTCCTTAACTCGGGCTATGCTGTTGCAAAAGAGAT
~~AAAAGATGCTCAAATTGATATTTATATCCACAAAAGTCGAGAAAACATTCTTTCAAATCG~~
ACAGTTATTAGAATCAGGGATAGATAAAGACCAAGCAATTTTTTTTTTCATATTGATGAT
TACTTTATTAAGAATATGCATCAATATTATGACGCAGTAATTTTATCGGTTGGAAATGGG
TTGTTAAAAAGGTTCTTTAAGCAGAATGCGCAATTAAATATTGCTTCAAGGCCATTGATT
ATTACCTTGTTTCCAGGTGTAGTATTCGGTGATCAGGCAAGTATTCTATCTCGTATGGGG
GCTGATATTGTTTTATATAATAATAAGCATGATTTTAGAATTGCAGAGGAATATAAGAAA
CAATATAAATTAAGTTGTCAAAATATACTTTATGGTTATCCAATTTTTCGCCATGCTTCG
AAAGGTTGTCATGGAGAGAAAATTTACTTTATTGACCAAGTTAAAATCCCATTTAAAAAA
GAAGAAAGAATTTATACATTAAAAAAATTGATTGCCTTGGCTGAAAAATACCCTGAGAAA
GAATTTACTATTTTGCTAAGGGTTGCAGATAAAGATATTACTGTGCATCAGGATAAACAT
TCGTATATAGAGCTGGCAAAGCAGTTTCAGTTGCCGAGTAATTTGACAATAGAGCGAAAA
AGTACCGCGCAAGCCTTCCAAGAAATGGGGTATTGTTTATCTTATTCATCTACTATGCTT
TTTGAAGCTGAATGTAAGGGTATCCCTGTTGGTGTTGTTGCAGACTTAGGCTTTTCTAAA
TCCTATGCAAATCAGCATTTTTTAGGTAGTGGGGTTTTAGTTTATTTTGATCAAATAGAT
TTCACTTCCCCAAAAATAGCAGATCCGGATTGGCTTGATTGCTATGCTACTAAAAAGGTG
ATTACAACTGATGAGTTTAATAAGCTATTAAAGCAGGTTGTGCCATTGCAACATGATTAC
CAAGAATATTTATCTGCAGGAATTCGATATCAAGCTTTGGCTAACACACACGCCATTCCA
ACCAATAGTTTTCTCGGCATAAAGCCATGCTCTGACGCTTAAATGCACTAATGCCTTAAA
AAAACATTAAAGTCTAACACACTAGACTTATTTACTTCGTAATTAAGTCGTTAAACCGTG
TGCTCTACGACCAAAAGTATAAAACCTTTAAGAACTTTCTTTTTTCTTGTAAAAAAAGAA
ACTAGATAAATCTCTCATATCTTTTATTCAATAATCGCATCAGATTGCAGTATAAATTTA
ACGATCACTCATCATGTTCATATTTATCAGAGCTCGTGCTATAATTATACTAATTTTATA
AGGAGGAAAAAATAAAGAGGGTTATAATGAACGAGAAAAATATAAAACACAGTCAAAACT
TTATTACTTCAAAACATAATATAGATAAAATAATGACAAATATAAGATTAAATGAACATG
ATAATATCTTTGAAATCGGCTCAGGAAAAGGGCATTTTACCCTTGAATTAGTACAGAGGT
GTAATTTCGTAACTGCCATTGAAATAGACCATAAATTATGCAAAACTACAGAAAATAAAC

## Appendix A

TTGTTGATCACGATAATTTCCAAGTTTTAAACAAGGATATATTGCAGTTTAAATTTCCTA
AAAACCAATCCTATAAAATATTTGGTAATATACCTTATAACATAAGTACGGATATAATAC
GCAAAATTGTTTTTGATAGTATAGCTGATGAGATTTATTTAATCGTGGAATACGGGTTTG
CTAAAAGATTATTAAATACAAAACGCTCATTGGCATTATTTTTAATGGCAGAAGTTGATA
TTTCTATATTAAGTATGGTTCCAAGAGAATATTTTCATCCTAAACCTAAAGTGAATAGCT
CACTTATCAGATTAAATAGAAAAAAATCAAGAATATCACACAAAGATAAACAGAAGTATA
ATTATTTCGTTATGAAATGGGTTAACAAAGAATACAAGAAAATATTTACAAAAAATCAAT
TTAACAATTCCTTAAAACATGCAGGAATTGACGATTTAAACAATATTAGCTTTGAACAAT
TCTTATCTCTTTTCAATAGCTATAAATTATTTAATAAGTAAGTTAAGGGATGCATAAACT
GCATCCCTTAACTTGTTTTTCGTGTACCTATTTTTTGTGAATCGATACCGTCGACCTCGA
GGGGGGGCCCGGTACCCAATTCGCCCTATAGTGAGTCGTATTACGCGCGCTCACTGGCCG
TCGTTTTACAACGTCGTGACTGGGAAAACCCTGGCGTTACCCAACTTAATCGCCTTGCAG
CACATCCCCCTTTCGCCAGGCAAAAAACCGGTTATATTTTTTTGCATTAAATATTTTTTT
AGCATATTCAGGAAAGGGGACATGCAATATGTCAAAATGATCTATATATCCTTTAATATT
AAGATTATTTCCAATCAAATAACGTTCTAATTTTGTTGGATGATATGAAAATGATTCTAA
TAAAGGAGCATATGTTCCAGTCCCTTCATCAATTAAATGAGTCGTAATATTCTTTTTTTT
TGCAATACTAATCAGATAGGAGTAGTGGCCTGTAAAAGACAGCATATAGAGATGAGCAGG
CTGTATAATATTAAGGATTTTTTTGTAACTTCTATAAATATAAAGTAATTTTTTAGGAGT
TATATTATTAGGGCTTCTAGGAAGCTCAAATAGATAAATAGATTCAAATAGATTCTTGTT
AGCTGATTGATGAACTAACTTAGGCATTTTTAAGTTTTTAGAAGTATATAAAATTACTAG
TAAATTATTGGTTAATTTTTGTATTTTAATTAGGCTTTGGACTTGGTTAAGCTGACCTAA
ATTAGATATGACAAATAAATTGTTACGTGGGGGGGTAAGATAAAATGGAGATGTTGTCAA
CCACATTGAATCTTGAAAAAACTTTTTAGGCTGAAAAAGAGCTTTTTTTATTTTCTTTAG
CATTATTGTATCTCTTAAAAATTAATGAGAATTAGCTATATGTAATAGCCAATCCTCTGT
TAATAAAGTAACTAAGTTAATAAGCATTATTCAATATCAGTTTTTTTGATTTGAGCACCT
TTGCGAATATTGCAAGCAGCGACCTTACCAAATAATGTTTCATATTCGTTGACGCTGAAG
TCTCCATTGCCTGGGCGTTTAACCCATAGGTTATCTCCGGACAACAGTTCTCCTTTTTTA
ATGTCTTTATCTGCTACGACAGATGCAAAGGCGAAATCTTTAGTTGGCTTTTCTCCCGCG
ATAATCGTGTCTTTTTTGCCGCCGCGTGCCAATTTTAAAGCATGAGCGCCTTGCTTGAGC
TCTTTAAAAGTATCCGGATTCATAGAGCATACAATATCCGGACCTGGGCGATCCATGCGG
TCAGTAAAGTGACGCTCTAAAATCGAACCGCCTAAAGCTACTGCTCCTAAGCAAGCATAG
TTATCTAAGGTATGGTCAGACAGGCCAATGATTGCGTCTGGAAAGGCTTCAGATAAATCG
TTCATACCACCCAATCGAACATCTTCGTAAGGGGTTGGGTAGATGTTGGTACAGTGAAGC
AAAGCATAAGGTACCCCTGCTTCTCGAATAATTTCTACCGACTTTTTGATGCTTTCAATA
GAATTCATGCCGGTAGAGAGAATAATAGGCTTACCAAAAGAGGCCACCAGTTTAATTAAT
GGGTAGTTATTACATTCGCCAGAGCCGATTTTATATGCTGGAATATCCATACGTTGTAAT
CGTAAAGCAGCTGCACGAGAGAAAGGAGTACTGATAAAAATCATACCCTTACTCTCTACG
TATTCTTTTAATTTAATCTCATCTTCTTCATTCAGGGCGCAACGTTCCATAATTTCATAA
ATAGAGACATCTGCATTGCCTGGAATGACTTGTTTGGCCTCATCAGACATTTCGTCTTCA
ACGATGTGTGTTTGATGTTTAACAACTTCAGCGCCTGCATTATAGGCAGCATCAACCATT
TCAAAAGCTGTTTTTAAAGAGCCTTCATGATTGATGCCGATTTCACAGATAATCAATGGT
TCGTGGTTGTAACCTACTGAACGATTACCAATTTTAAATTCGTTGTTGTTTTGCATTTAG
CTTTCCTTGTGATTAAGAATGTTTTCTGCCTGTTGTAAATCAAGCTCAGTATCAATATCG
ATAGAGTCTTGATGAGACATAATATAAAGTTTGGTTGGGGCGATAAAAAAACAATTATTT
GCAATTAGTGAAGCAGTATCATTAATGTAAATTGCACCATTAGGCCTAAATGCCTGAGGT
AATTGTTGGCGAGGCTGCTCCAAATCGCTTAGATGGCGCATGGGGGCATATTCGCCATTA
TTGATTTGAAGCAGGGTTTTTAGTGGATGATGCTCCATTGGGCATGCAGAGACAACGGAT
CCTTTTATTTTCTCATCAAATAGAGAAAAAGCTTCACGAATATGAGCCCCTGTGCGTAAT
GGACTGGTTGGTTGTAATAGGGTTACTGTGCCGGAATTACTGCCAATTGTTTCTAAAGCA
~~TGTATTACACCTGAAATAGAGCTGGCTGTATCGGAGGCCAGCTCTGCAGGGCGTAGGACG~~
ACTTCGACACCGAAATTTTTAGCTTCTTCTGCAATTAACCCGCCATCAGTCGAAACAATT
ATGCGGTCAAAACACTTTGATGATATAGCAGCATTAATTGTATGACCAAGTAATGATATG
CCATTCATTTTCCGGAGATTTTTTAATGGCAATCCTTTGGAGTTTTGGCGCGCAAGTATA
ACCGCAATATTTTGTTTTTCCATAATTTAAAGATTCAAATCGATAAAACGTTTTTGAGCA
GAAACATTCCACGTTTCAGGATTGTTGATTACTTCAGCAAATCTTTCTGTGCTGGTGCGA
GTATCTCCGCCATTAAAGGTATCATCTGCTTCAAATTTGCCTAAACTGCATGCTTGTTGA
ATCGCATCAAAGATATTTTTAGTTTCATAATCTGTATGAATAATAGATTTTCCCATATGG
CGGTTACTTTGGCGTGTACCAACATCAATTGAAGGGACACCGTAGAGAGGAGCTTCTCTA
ATACCTGCACTTGAGTTGCCGACCATAAATTTAGCATGTTTCAATAAGACTAAAAAATAT
TCAAATCGAATGGAAGGAAATGCAATAAATTTATCAGATTGATATTTTAATAATTCTTGC
AGAATACTTTCAGTGCCAGTGTCATTATTAGGGTAGATGCTAATGATATTTTGGCCACTT
AATTCTAATGCTTTGAAATATTGGGCCGCATATTGTGGCATTAAATGTGCTTCTGTAGTC
ACGGGGTGAAACATAGAAATACCATAATTTTCGTATGGTAAACCGTAATATTCTTTGACT
TCTTCTAAGGATGGGAGGGTGGAAGAGGCCATAACATCTAAATCGGGGGAGCCGATGATG
TGAATATGCTTTCTTTTTTCTCCCATTTGCACTAGGCGAGTGACAGCTTGTTCATTTGCT
ACCAAGTGGATATGAGAAAGTTTACTAATAGAATGACGAATGGAGTCATCTACTGTACCA
GATAGTTCACCACCTTCGATATGGCAAACTAAACGGCTGCTTAATGCACCTACAGCTGCG
CCTGCTAGTGCTTCTAAACGGTCGCCGTGAATCATGACCATATCAGGTTCAATTTCATCA
GATAGACGAGAGATAAACGTAATGGTATTGCCTAAAACGGCACCCATTGGTTCACCTTGG
ATTTGATTTGAAAACAGATATGTATGTTGATAGTTTTCTCGAGTTACTTCCTTGTAGGTT
CTGCCATATGTTTTCATCATATGCATACCAGTTACAATCAAATGCAATTCAAGGTCTGGG
TGATTTTCAATATAGGCTAATAAAGGTTTTAGCTTGCCGAAGTCGGCTCTGGTACCTGTA
ATGCAAAGAATTCTTTTCATGATTTTAGAATCTATAAGTATATAAGTATAAGGAAGTTGG
AAAGAAGAATACTAATTATACTCTACGTACTCATAAATTTATTTCGATTAAGTGCTATAA
TTAGGCCATTTATAATTATATTAGGATTTGGCTTGTGTTTAAAGTGAAATTTTATATTCG

## Appendix A

TCACGCAGTATTATTATTGTGTGGAAGTTTAATTGTAGGATGCTCTGCGATTCCTTCATC
AGGCCCCAGCGCAAAAAAAATTGTCTCTTTAGGGCAACAATCTGAAGTTCAAATTCCTGA
AGTGGAGCTGATTGATGTGAATCATACGGTTGCTCAGTTATTATATAAGGCTCAGATAAA
TCAGTCATTCACTCAGTTTGGCGATGGTTATGCTTCGGCTGGTACGCTAAATATTGGTGA
TGTATTGGATATTATGATTTGGGAAGCGCCGCCGGCAGTATTGTTTGGTGGTGGCCTTTC
TTCGATGGGCTCGGGTAGTGCGCATCAAACTAAGTTGCCAGAGCAGTTGGTCACGGCACG
TGGTACGGTTTCTGTGCCGTTTGTTGGCGATATTTCGGTGGTCGGTAAAACGCCTGGTCA
GGTTCAGGAAATTATTAAAGGCCGCCTGAAAAAAATGGCCAATCAGCCACAAGTGATGGT
GCGTTTGGTGCAGAATAATGCGGCGAATGTGTCGGTGATTCGTGCTGGGAATAGTGTGCG
TATGCCGCTGACGGCAGCCGGTGAGCGTGTGTTGGATGCGGTGGCTGCGGTAGGTGGTTC
AACGGCAAATGTGCAGGATACGAATGTGCAGCTGACACGTGGCAATGTAGTACGAACTGT
TGCCTTGGAAGATTTAGTTGCAAATCCGCGACAAAATATTTTGCTGCGTCGCGGTGATGT
GGTTACCATGATTACCAATCCCTATACCTTTACGTCTATGGGTGCGGTGGGGAGAACACA
AGAAATCGGTTTTTCAGCCAGAGGCTTATCGCTTTCTGAAGCCATTGGCCGTATGGGCGG
TTTGCAAGATCGCCGTTCTGATGCGCGTGGTGTGTTTGTGTTCCGCTATACGCCATTGGT
GGAATTGCCGGCAGAACGTCAGGATAAATGGATTGCTCAAGGTTATGGCAGTGAGGCAGA
GATTCCAACGGTATATCGTGTGAATATGGCTGATGCGCATTCGCTATTTTCTATGCAGCG
CTTTCCTGTGAAGAATAAAGATGTATTGTATGTGTCGAATGCGCCGTTGGCTGAAGTGCA
GAAATTTTTGTCGTTTGTGTTCTCGCCGGTTACCAGTGGCGCGAACAGTATTAATAATTT
AACTAATTAATGTGAGTAATTAAGATGTCTGAGCAACTTCCTGTGGCAGTTGCCACTGAA
ACCAAAGCCGAGCGTAAAAAGCCGAAAAAGAAAAGTTGGATTAAAAAGCTAAGCCCTTTA
TTTTGGGTAACGGTGATTATCCCTACGGTAATTTCGTTGGTGTATTTCGGCTTCTTCGCT
TCCGATCGTTTTACGTCGCAATCGAGCTTTGTGGTGCGCTCGCCTAAAAGCCAATCTTCT
CTCAATGGCCTGGGTGCCATTTTGCAGGGCACAGGTTTTGCCCGTGCGCAAGATGATATT
TACACGGTTGGGGAGTATATGCGTTCGCGCTCGTCTTTGGATGAACTGCGTAAAATCTTG
CCGGTGCGTGAGTTTTATGAAACCAAAGGTGATGCGTTCAGCCGCTTTAATGGGTTTGGG
TTCCGTGGCGAGGAAGAGGCTTTTTATCAATACTATAAAAATCAGGTGATGATCAATTTT
GATACGGTTTCGGGTATTTCCACGTTGAATGTAACTTCCTTTGATGCGCTGGAATCTAAG
AAAATCAATGAGGCTTTGTTAAAACAAGGTGAAGCATTGATTAACCAGTTGAACGATCGT
GCACGTGCTGATACGGTGCGCTATGCGGAAGAAGTAGTGAAAACGGCGGCAGAGCGGGTA
AAGGAAGCCTCTCAGAATCTGACGGATTACCGGATTGCCAATGGCGTTTTTGATTTGAAA
GCGCAATCGGAAGTGCAAATGGGGTTGGTTTCCAAGCTGCAAGATGAATTGATTGTGATT
CAAACCCAGCTGGATCAGGTGAAAGCAGTCACTCCGGAGAATCCGCAGATTCCGGGTTTG
CAGGCGCGTGAGCAGAGCTTGCGTAAAGAAATTGACCAACAGTTACGTGCCATTTCGGGC
GGTGGGCATTCTTCGTTGTCTAATCAGGCTGCCGAATATCAGCGTGTGTATTTGGAAAAC
CAGTTGGCAGAGCAGCAGTTGGCAGCCGCCATGACTTCTTTGGAAAGTGCCAAGGTTGAA
GCAGACCGTCAGCAGCTTTATTTGGAAGTGATCTCGCAACCGAGCCTGCCGGATTTGGCA
CATGAGCCTAAACGGTTATACAACATTGTTGCCACTCTGATTATCGGCTTGATGGTTTAT
GGTATTTTGAGCCTGTTGACTGCCAGCATTCGTGAGCATAAAAACTGATGAAAGCCTTGC
ATAAAACATCATTTTGGGAATCTTTAGCCATTCAAAGGCGCGTAATCGGTGCGCTGTTGA
TGCGGGAAATTATCACCCGTTACGGTCGCAATAATATTGGCTTTTTATGGCTGTTTGTTG
AGCCGTTGCTGATGACATTCGTTATCGTCTTGATGTGGAAATTTTTAAGGGCAGACCGAT
ATTCAACTTTGAATATTGTCGCATTTGCGATTACTGGCTATCCGATGTTGATGATGTGGC
GTAATGCCTCAAAACGGGCAGTTGGGTCGATTTCTTCAAATGCCAGCTTGCTTTATCACC
GCAATGTAAGAGTTTTGGATACCATCTTGGCGCGCATGATTTTGGAAATTGCTGGTGCAA
CCATTGCGCAGATTGTGATTATGGCGGTATTGATTGCGATTGGCTGGATTGAAATGCCGG
CAGATATGTTTTATATGCTGATGGCTTGGCTTTTGATGGCTTTTTTTGCGATTGGTTTGG
GTTTGGTGATTTGTTCGATTGCCTTTAATTTCGAGCCGTTTGGCAAGATTTGGGGCACAT
TGACTTTTGTGATGATGCCGTTATCCGGTGCGTTCTTTTTTGTGCATAATTTGCCGCCCA
~~AGGTACAAGAATATGCATTAATGATTCCGATGGTGCATGGCACAGAAATGTTCCGTGCCG~~
GATATTTTGGCAGCGATGTAATTACCTATGAAAATCCTTGGTATATCGTATTGTGCAATC
TGGTGTTGTTGTTGTTTGGCTTGGCGATGGTCAGTAAATTCAGTAAAGGAGTCGAGCCGC
AATGATTTCAGTTGAACACGTTTCCAAACGCTATCTGACCCGCCAAGGTTGGCGGACAGT
CTTGCACGATATTAGCTTCAAAATGGAGAAGGGCGAGAAAATCGGTATTCTCGGCCGCAA
CGGTGCAGGTAAATCGACGCTCATCCGTTTGATCAGTGGCGTTGAGCCGCCGACCACGGG
TGAAATCAAGCGGACAATGAGTATTTCTTGGCCTTTGGCATTCTCCGGTGCGTTTCAAGG
CAGTCTGACCGGTATGGACAATTTGCGTTTCATCTGCCGGATTTACAATGTCGATATCGA
TTATGTGAAAGCGTTTACGGAAGAATTTTCGGAGCTGGGGCAATATTTGTATGAGCCGGT
GAAACGCTATTCTTCAGGTATGAAAGCGCGTTTGGCTTTTGCGCTGTCGTTGGCGGTGGA
GTTTGACTGTTACCTGATTGACGAAGTGATTGCAGTTGGTGACTCGCGTTTTGCCGATAA
ATGTAAGTACGAGTTGTTTGAAAAGCGCAAAGACCGTTCCATCATCTTGGTGTCGCACAG
CCACAGCGCCATGAAGCAATATTGCGATAATGCGATGGTGCTGGAAAAAGGGCATATGTA
CCAGTTTGAAGATATGGACAAAGCCTACGAATATTATAATTCGCTGCCTTAAAGCGATTG
TTTTTAAATCAGGCCGTCTGAAATTTCAGACGGCCTGTCCGTTGGAATTCTATTGATGAA
CATTACTCAAATTCTTTCCCAAGAACTCTCCGCGACTGCCGCGCAAATCACCGCCGCCGT
CGAGCTTTTGGACGACGGCGCGACCGTGCCGTTTATCGCCCGCTACCGCAAGGAAGCGAC
GGGCGGGTTGGACGATACGCAGTTGCGCCGGCTTGCCGAGCGGCTGCAATATCTGCGCGA
GTTGGAAGAGCGCAAAGCCGTTGTTTTAAAAAGCATTGAAGAGCAAGGCAAGCTTTCAGA
CGACCTCAGGGCGCAAATCGAAGCCGCCGATAACAAAACCGCGCTGGAAGACCTGTATCT
GCCCTACAAACCCAAACGCCGCACCAAAGCGCAAATCGCGCGCGAACACGGTTTGCAGCC
GCTGGCGGACGTGTTGCTTGCCGAGCAGTCGCAGGACGTGGAAGCCGCCGCACAAGGCTA
CCTGAACGAAAACGTCCCCGATGCCAAAGCCGCGTTGGACGGCGCGCGTGCGATTCTGAT
GGAGCAGTTTGCCGAAGACGCGGAACTTATCGGCACGCTGCGCGACAAGCTGTGGAACGA
AGCCGAAATCCACGCGCAAGTCGTTGAAGGCAAAGAAACCGAAGGCGAAAAATTCAGCGA
TTATTTCGACCACCGCGAACCCGTCCGCACTATGCCCAGCCACCGCGCGCTGGCGGTTTT

## Appendix A

GCGCGGCCGCAACGAAGGCGTGTTGAACATCGCGCTCAAATACCAGCCCGACGACACGCC
GATTACCCGGCAAAGCGAATACGAGCAAATCATCGCCTGCCGCTTCAAGGTTTCAGACGG
CCACAAATGGCTGCGCGATACCGTGCGTCTGACTTGGCGCGCGAAAATCTTTTTGTCGTT
GGAACTTGAAGCCCTAGGCCGTCTGAAAGAAGCCGCCGACACCGACGCGATTACCGTGTT
CGCCCGCAATCTCAAAGACTTGCTGCTCGTCGCGCCCGCCGGACGGCTGACCACGCTGGG
TCTCGACCCCGGCTACCGCAACGGCGTGAAATGCGCCGTGGTGGACGACACCGGCAAGCT
GCTGGATACCGTCATCGTCTATTTGCATCAAGAAAACAATATGTTGGCAACGCTGTCGCG
CCTGATTAAGCAACACGGCGTGAAGCTCATCGCCATCGGCAACGGCACCGCCAGCCGCGA
AACCGACAAAATCGCGGGCGAACTGGTGCGCGGAATGCCGGAAATGGGGCTGCACAAAAT
CGTCGTGTCCGAAGCCGGCGCGTCGATTTATTCCGCGTCCGAACTGGCGGCGCGCGAGTT
CCCCGACTTGGACGTTTCCCTGCGCGGCGCGGTGTCCATCGCCCGCAGGCTGCAAGACCC
GCTTGCCGAGTTGGTCAAAATCGACCCTAAATCCATCGGCGTGGGCCAGTATCAGCACGA
TGTGAACCAAAACCAGCTCGCCAAATCGCTGGACGCAGTGGTCGAAGACTGCGTGAACGC
CGTCGGCGTGGACGTGAATACCGCCTCCGCCCCGCTCTTGGCGCGGATTTCCGGCTTGAA
TCAAACCCTTGCCCAAAACATCGTTGCCTACCGCGATGAAAACGGCGCGTTCGACAGCCG
CAAAAAATTGCTGAAAGTACCGCGTTTGGGCGAAAAAACCTTCGAGCAGGCGGCAGGCTT
TTTGCGGATTAACGGCGGTAAAGAGCCGTTGGACGCGAGCGCCGTCCACCCCGAAGCCTA
TCCCGTCGTCGCCAAAATGCTGGCGCAACAAGGCATTAGCGCCGCCGAACTCATCGGCAA
CCGCGAGCGCGTGAAGCAAATCAAAGCGTCCGACTTCACCGACGAACGCTTCGGCCTGCC
GACCATTTTGGACATCCTGTCCGAACTGGAAAAACCCGGCCGTGATCCGCGCGGCGAGTT
TCAGACGGCATCGTTTGCCGAAGGTATCCACGAAATCAGCGACTTGCAAGTCGGTATGAT
ACTCGAAGGCGTGGTTTCCAACGTCGCCAACTTCGGCGCGTTCGTGGACATCGGCGTCCA
TCAGGACGGCTTGGTGCACATCTCCGCCCTGTCCAACAAGTTCGTCCAAGACCCGCGCGA
AGTGGTGAAAGCTGGCGACGTGGTGAAAGTGAAAGTGCTGGAAGTCGATGCTGCACGCAA
ACGCATCGCGCTGACCATGCGCTTGGATGACGAACCGGGCGGCGCAAAACATAAAATGCC
GTCTGAAAACCGCAGCCGCGAACGGACAGCCGGCCGCAAACCCCAACGCAACGACCGCGC
CCCAGCCAATTCGGCGATGGCGGATGCGTTTGCGAAGCTGAAGCGGTAAAATAATCGAAG
AGTTTATGGATTTTGACTTATGCACACACCACTTACCTATATTGACCTTTTCTCAGGAGC
AGGAGGCCTATCCTTGGGTTTTGAACAAGCCGGATTCCAACAATTGCTTTCTGTTGAAAT
GGAGTCTGATTATTGTCAGACTTACCGTACCAACTTCCCCCATCATCAATTACTGCAAAA
AGATTTAACCACACTAACCGAACAAGATTTAATCAATTGTCTTAACGGACAAGCAGTTGA
TTTGATTATTGGAGGACCACCTTGTCAAGGTTTTAGTATGGCAGGAAAGATTGGACGGAC
ATTTACAGATGACCCACGCAACCATTTATTTAAAGAGTTTGTCCGAATAGTTAAAATTGT
CCAACCATATTTTTTTGTTATGGAAAATGTAGCGCGACTCTATACACACAATTCAGGTAA
AACACGTATTGAGATTATTCAAGCATTTCAGAATATCGGTTATTCGGTGGAATGTAAGAT
ACTGAGTGCAGCCGATTTCGGTGTTCCTCAGATACGTAGCCGAGTGATATTTATCGGGAG
GAGGGATAAAGGCAAAATTTCCTTTCCCGAACCTTTGCAGATTTCCCATCAGACTGTTGG
ATCAGCAATAGGACATTTTCCAAAACTGGCTGCTGGCGAAAGCAATCCACACGTTGCAAA
TCATGAAGCTATGAATCATTCGGCACAAATGTTAGAAAAAATGGCATTTGTTAAAAATGG
AGGTAACCGTAACGATATTCCTGAACCATTACGTCCGAAAACAGGTGATATCCGTAAATA
CATCCGTTACAACAGCAACAAAACCAGCCGTTTGTATTACAGGAGATATGCGCAAAGTTT
TTCACTATGAACAGAATCGGGCGTTAACCGTTCGTGAATTAGCTGCCTTACAATCTTTCC
CTGATAATTTTATTTTTTGCGGCAGCAAAATTGCCCAGCAGCAGCAGGTTGGTAACGCCG
TACCGCCTTTATTGGCAAAAGCTATTGCTGAAAGTATTTTAAAAATGAGTGAAAATGAAT
AAGCAATATCCGAAAATTAACTATATCGGTAATAAAGAGAAAATAGCTTCCTGGATTTGT
GACCAGCTTCCGTCTGATGTAGATACAGTTGCAGATGTATTTAGTGGAGGCTGTTCCTTT
GCCTACGAAGCCAAAAAACGCGGCTATCGTGTGATTACTAACGATATTTTGGCAATTAAT
TACCAAATTGCTTTAGCATTAATAGAAAACAACCATGAAACATTAAATGACGATGATGTC
GCAATGATTTTTTCAGGCAGCCCGCATGCCGGTTTTATGAGTCAGCGTTATGCCGAAAAA
TTCTATTTTCACGATGAATACCAACAACTTGATTTGTAACGTAAAAATATAGGGAAACTG
GATAACCAGTATAAACGCGCTTTGGCGTTTACTTTAATGCGTCGCGCCATGATACGTAAA
ATGCCCTATACGGAAGATATGCGCCCAGGCGATACCGCTAATCCTTATGGTGCGTCCAAA
GCGATGGTGGAACGGATGTTAACCGACATCCAAAAAGCCGATCCGCGCTGGAGCATGATT
TTGTTGCGTTATTTCAATCCGATTGGCGCGCATGAAAGCGGCTTGATTGGCGAGCAGCCA
AACGGCATCCCGAATAATTTGTTGCCTTATATCTGCCAAGTGGCGGCAGGCAAACTGCCG
CAATTGGCGGTATTTGGCGATGACTACCCTACCCCCGACGGCACGGGGATGCGTGACTAT
ATTCATGTGATGGATTTGGCAGAAGGCCATGTCGCGGCTATGCAGGCAAAAAGTAATGTA
GCAGGCACGCATTTGCTGAACTTAGGCTCCGGCCGCGCTTCTTCGGTGTTGGAAATCATC
CGCGCATTTGAAGCAGCTTCGGGTTTGACGATTCCGTATGAAGTCAAACCGCGCCGTGCC
GGTGATTTGGCGTGCTTCTATGCCGACCCTTCCTATACAAAGGCGCAAATCGGCTGGCAA
ACCCAGCGTGATTTAACCCAAATGATGGAAGACTCATGGCGCTGGGTGAGTAATAATCCG
AATGGCTACGACGATTAAGTTGACCTGATACAGGCCGTCTGAAAGAGATGTTTTCAGACG
GCCTCTTTATCTGAAAAACACACATTCTGTCTGCTATAATCTGTTTATATTTTTTGGCTA
TCCTCTGAAATTTATGAGAAAAATCCTTGTTACCGGCGGCGCGGGCTTTATCGGTTCTGC
CGTTGTCCGTCATATTATCCGAAACACCCGGGACGCTGTCGTCAATGTCGATAAGCTGAC
TTATGCCGGCAATTTGGAATCTTTGACTGAGGTAGCCGATAATCCTCGCTATGCTTTTGA
ACAAGTGGATATTTGCGACCGCGCCGAACTCGACCGCGTATTCGCGCAATACCGGCCTGA
TGCCGTGATGCACTTGGCGGCGGAAAGCCATGTCGACCGCTCTATCGGTTCGGCAGGCGA
GTTTATCCAAACCAATATCGTCGGCACATTCAATCTGCTTGAAGCAGCCCGCGCCTACTG
GCAACAAATGCCGTCTGAACAGCACGAAGCCTTCCGTTTCCACCATATTTCCACCGATGA
AGTCTATGGCGATTTAGGCGGCACGGACGATTTGTTTACCGAAACCGCGCCCTACGCGCC
GTCCAGCCCCTACTCTGCCTCTAAAGCGTCCAGCGACCACCTCGTCCGCGCGTGGTTGCG
TACTTACGGCTTGCCGACCATTGTAACCAACTGCTCCAACAACTACGGTCCTTACCATTT
TCCGGAAAAACTCATTCCTTTGATGATTCTGAACGCGCTTGACGGCAAACCGCTGCCTGT
GTACGGCGACGGTATGCAAATCCGCGACTGGCTGTTTGTCGAAGACCACGCGCGCGCACT

## Appendix A

GTATCAGGTTGTTACCGAAGGTGTTGTCGGCGAAACCTACAATATCGGCGGCCACAATGA
AAAAGCCAATATTGAAGTCGTCAAAACCATCTGCGCCCTGCTGGAAGAACTCGCTCCCGA
AAAACCGGCCGGTGTGGCGCGTTATGAAGATTTGATTACTTTCGTACAAGACCGCCCCGG
CCATGACGTACGCTACGCCGTCGACGCAGCCAAAATCAGGCGGGATTTGGGCTGGCTGCC
TTTGGAAACCTTCGAGTCCGGCCTCCGCAAAACCGTGCAATGGTATCTGGACAACAAAAC
CTGGTGGCAAAATGTATTGAACGGCAGCTATCGTTTGGAACGTTTAGGTACTGGAAAATA
GTTTTCAGACGGCATCCCGACGCAATGCCGTCTGAAAACCCATCGCAAAGGAAGAAAGAA
AAGATGAAAGGCATCATACTGGCAGGCGGCAGCGGCACGCGCCTCTACCCCATCACGCGC
GGCGTATCCAAACAGCTCCTGCCCGTGTACGACAAACCGATGATTTATTACCCCTTGTCG
GTTTTGATGCTGGCGGGAATCCGCGATATTTTGGTGATTACCGCGCCTGAAGACAACGCC
TCTTTCAAACGCCTGCTTGGCGACGGCAGCGATTTCGGCATTTCCATCAGTTATGCCGTG
CAACCCAGTCCGGACGGCTTGGCACAGGCATTTATCATCGGCGAAGAATTTATCGGCAAC
GACAATGTTTGCTTGGTTTTGGGCGACAATATTTTTTACGGTCAGTCGTTTACGCAAACA
TTGAAACAGGCGGCAGCGCAAACGCACGGCGCAACCGTGTTTGCTTATCAGGTCAAAAAC
CCCGAACGTTTCGGCGTGGTTGAATTTAACGAAAACTTCCGCGCCGTTTCCATCGAAGAA
AAACCGCAACGGCCCAAATCCGATTGGGCGGTAACCGGCTTGTATTTCTACGACAACCGC
GCCGTCGAGTTCGCCAAACAGCTCAAACCGTCCGCACGCGGCGAATTGGAAATTACCGAC
CTCAACCGGATGTATTTGGAAGACGGCTCGCTCTCCGTTCAAATATTGGACGCGGTTTC
GCGTGGCTGGACACCGGCACCCACGAGAGCCTGCACGAAGCCGCTTCATTCGTCCAAACC
GTGCAAAATATCCAAAACCTGCACATCGCCTGCCTCGAAGAAATCGCTTGGCGCAACGGT
TGGCTTTCCGATGAAAAACTGGAAGAATTGGCGCGCCCGATGGCGAAAAACCAATACGGC
CAATATTTGCTGCGCCTGTTGAAAAAATAATGTTTGAGGCCGTCTGAAACTTTTCAGACG
GCCTTTAGATGAAAGATAAAAAGATGAACATCATTGATACCGCCATTCCTGACGTAAAAC
TGCTTGAGCCCCAAGTCTTCGGCGACGCGCGCGGCTTTTTTATGGAAACCTTCCGCGACG
AGTGGTTTAAAACCCAAGTCTGCGAACGCACCTTCGTGCAGGAAAACCACTCCAAATCCG
GCAAAGGCGTATTGCGCGGCCTGCACTATCAAACTGAAAACACACAAGGCAAACTCGTAC
GCGTGGTTGTCGGCGAAGTATTCGACGTGGCCGTCGATATGCGTAAAGACTCCCCCACTT
TCGGCAAATGGGTAGGCGAAATTCTGTCCGCAGAAAACAAACGCCAACTGTGGGTACCCG
AAGGTTTCGCACACGGCTTCTATGTACTGAGCGATGAAGCCGAGTTCGTCTATAAATGCA
CAGACTATTACAACCCCAAAGCCGAACACTCGCTGATTTGGAATGATCCGACCGTCGGCA
TCGACTGGCCGTTGCAAGGCGAGCCGAACCTGTCGCCTAAAGACTTGGCAGGCAAAGTAT
TGTCTGAAGCGGTAACGTTTTAAAAATAATTCAGGCCGTCTGAAAGAATGTTCCTCTTTT
CAGACGGCCTACAATCCATTAATAACAATAATCGACGAAAACGCATTGTGAAAAACGCCT
ACATCCCCTCTCGCGGCATCCGCAAAATCCCCCATCTCTCCACCCTATTGCCTGAATTTC
ATATCTGCAAAGACGGGAAAGAAGCAGAGGCTGTTGTCGGCTGGGGTTTGCGCCCGACGA
CACACAAAGCGCGTGCTTTTGCCGCTGAACACCAGCTTCCCTTTATTGCTTTGGAAGACG
GCTTTTTACGATCGCTCGGACTGGGTGTCGCCGGTTATCCGCCCTACTCTATCGTCTATG
ACGACATCGGCATCTACTACGACACCACACGTCCTTCGCGTTTGGAACAACTGATTCTTG
CCGCCGATACCATGCCGTCTGAAACCTTGGCTCAGGCGCAGCAGGCGATGGATTTCATCC
TGCAACACCACCTGTCCAAATACAACCACGCGCCCGAACTTTCAGACGACCATCCTTTAC
GTTCCCCATCCAAACCCGAAACCGTCCTCATCATCGACCAAACCTTCGGCGATATGGCCA
TCCAATATGGCGGCGCAGACGCCTCTACGTTTGAACTGATGTTTCAGACGGCCTTAAATG
AAAACCCGCAAGCCGATATCTGGGTAAAAACCCATCCCGATGTTTTGTGCGGCAAAAAAC
AAGGCTATCTGACCCAACTGGCGCAGCAACACCGCGTCCATCTTTTGGCAGAAGACATCA
ATCCGATTTCTTTGTTGCAAAACGTTGATAAAGTTTATTGCGTTACCTCGCAAATGGGTT
TTGAGGCGCTTTTGTGCGGCAAACCGCTGACCACTTTCGGCCTGCCGTGGTATGCCGGAT
GGGGTGTAAGCGACGACCGCCATCCTGAAATCAACCGCCTTGTTCAAACCCAACGCCGCG
CCACCCGCAACTTGCTGCAGCTCTTCGCCGCAGCCTATCTGCAATACAGCCGCTACCTCA
ACCCCAATACCGGCGAAGCAGGCAGCCTCTTTGATGTCATCGACTATCTGGCGACGGTCA
~~AACGTAAAAACGACAAATTGCGTGGCGAGTTATATTGCGTCGGTATGTCTTTGTGGAAAC~~
GCGCGGTTGCCAAACCGTTCTTTAACGTACCCTCTTGCCGTCTGAAATTTATCTCTTCCA
CCCAAAAACTGGCAAGGGTCAAACTGTCCGACGATGCACGCATCCTGGCTTGGGGCAACG
GCAAAGAGGCCATCGTCCGCTTTGCCGAACAACACCACATCCCCCTGCTGCGCATGGAAG
ACGGCTTTATCCGCTCGGTCGGACTCGGCTCCAACTTAGTGCCGCCGCTGTCGCTCGTTA
CCGACGATATGAGCATTTATTTCAATGCCGAAACCCCGTCCCGTCTTGAATACATCCTAC
AAAACCAAAACTTCGACGATCAAGACTTTCAGACGGCCTTGAAGCTGCAAAAAATGCTGA
CCGAAAACCACATCAGTAAATACAACGTCGGCAGCTCAGACTTCACCGCCCCGTCAACCG
ACAAAACCGTGATCCTCGTTCCCGGCCAGGTTGAAGATGATGCGTCTATCCGCTACGGTT
CGCCCCAAATCTACCGCAATCTGGATTTGCTCCGTACCGTACGCGAACGAAACCCCAATG
CCTATATCATCTACAAACCGCATCCCGATGTAGTCAGCGGTAACCGCATCGGCCATATTT
CCCCTGAAGATGCTGCACGATATGCCGACCAAACCGCCGAACAAGCCGACATCCTGACCT
GTCTCCAATACGCAGACGAAATACATACCATGACTTCGCTGACCGGTTTTGAAGCCTTGT
TGCGCGGCAAAAAAGTCAGCTGCTACGGCCTGCCTTTTTACGCAGGCTGGGGGCTTACCC
AAGATCTGCTCCCCATCCCGCGCCGTAGCCGCAGACTTGAGCTTTGGCAGCTGATTGCCG
GCACGCTCATCCACTATCCCGACTACATCCACCCCGAAACCCATCAGGCCATAAATGCAG
AAACCGCAGCCCAAATCCTGATACGACAAAAAAATATGCAAAAAAACAACAACGGATTAC
ATCGCGGGTGCTTTGCCAAAAAATTAGGTAAAATCAAACAACTATATCGATCTTTCAAAT
AAATACCATCAAAGTTAACGATGCGTCATAAACTTGCCTCTATTGCGGCATCATTGCCTT
TGCATCGTTAATTCTCTTGGCGTATGCTTGAAAGTTCAACCTAAAACTATTACATAAAAA
ACAAAACCACATTGCAACATGAAACAGACCGTCCTCAAAAATAACCTGCAAAACCTGCTT
GAAAGCGCAGAAAATATCCTGCTGCTTCAAGGCCCTGTCGGCGATTTTTTTCTGCGCCTT
GCCGACTGGCTGACTGCAAACGGCAAAACCGTACATAAATTCAACTTTAATGCAGGCGAC
GACTATTTTTATCCGCCCACTCAAGCGCATACCGTTGTTTTTAACGACAACTACGATGCC
TTTCCTGAGTTTTTGCAAGAATACATCACTCAACATCACATCCAGGCCGTTGTCTGCTTT
GGCGACACACGCCCTTATCACGTCATTGCAAAACGCATTGCAAACGAAAACCAAGCCAGT

## Appendix A

TTCTGGGCGTTTGAAGAAGGCTATTTCCGCCCCTACTACATCACCTTAGAAAAAGACGGC
GTCAACGCATTTTCCCCGTTGCCGCGCCGTGCCGACTTTTTCTTGAACAATTCCCTAAG
CTTGCCCAGCAAGAATATAAAGCGCCAACGCCGGTACACGGCGGTTTTACGCCCATGGCA
AAAAACGCTATCCGTTACTATATCGAGTTGTTCCGCAATCCACGCAAATACCCCGACTAC
ATCCACCACCGCGCACCCAATGCCGGCCATTACCTCAAACCGTGGTCGCTCTCCATCCTC
AAGCGTTTGAACTACTATATTGAAGACATCCAAATCGCCAAACGTGTGGAAGCAGGCAAA
TACGGCAAGTTTTTTATTGTTCCCTTACAGGTATTCAACGACAGCCAAGTCCGTATCCAT
TGCGACTTTCCCAGCGTCCGCAGCTTCCTGCTCCATGTTTTGAGTTCATTTGCCGAGCAC
GCGCCTGCCGATACCAACATCATCATCAAGCATCATCCGATGGACCGCGGTTTTATCGAC
TACTGGCGCGACATTAAACGCTTTATCAAAGAACACCCCGAACTCAAAGGCCGTGTGATT
TATGTCCATGATGTCCCCCTGCCCGTTTTCCTGCGCCACGGTCTCGGCATGGTCACCATC
AACAGCACCAGCGGCCTGTCCGGACTGATTCACAATATGCCAGTTAAGGTTCTCGGCCGT
GCCTATTATGATATTCCCGGCATTACTGACCAAAATACCTTGGCAGAATTTTGGAATCAT
CCGACACCGCCTGACAAAGAGCTGTTCCATGCCTACCGAATGTACCACCTCAACGTGACC
CAAATTAACGGCAACTTCTACAGTCAGGTGTTTTTCCCCAACAAAAAAACCTCCAACTCT
TCCACACCAGTAATCTGACTTAGCGAAGGAAGTTCAGGCCGTCTGAAAACATTTCAGACG
GCCTGAAACAATCAATACCTTAGCTACTGCCATGTAAATAAAACACAAAAATCTGCATTT
ATCATTAACAATAAATTACAAAAACAGTATAATGACCGAGCTGCCATGAGCGCATACCGA
CTCAACCTGAGCCCTTTGTAACACACAAAATATGGATATATCCCTAGGCAAAACAATATA
ACAAGCCAAACATCCTAAAGATAAGCCGGCAAGGCAATACACTCTATAAAACTATGCCGA
GCAAAATTTTTACAAAGCCCTCAACCGGTATCGCCGCCCATATGCCGCAGCATCCGTCTT
CCACTTTATATCCGCCCGCAAACCATGACCGCCGCTCCTGATATCCTCTACCGGCAAGCC
GCCGCCCTTTTGGAACAATCCAATACCGCCCAAGCCCTGCCCCTGTTGCAACAGGCGGCA
GAGCAAGGTTATGCGGAAGCTGCTTTCGTATTGGGCAACCATCTGCTGCAAAACGGCCAA
CCGGAGCAGGCACTTTCATGGTTGGAAGCCGCCGCGGCCCAACGCCATCCCAAAGCACTC
TTCTCCCTGCTGCAACAACGCGAACACAACGGCACCCCGACCGGACAGCTTCTCAACGAC
TATGCCTGGCTGGGTGAGCAGGGGCACTCAGAAGCCCAATTAATCCTCATGCGTTACCAC
GCGCAACGCAACGATCCACAATCGCTCTACTGGGCGGAACTTGCTGCCGCCCGATATGCC
GCACCTGCGTATTACCATCTGGCACGCCATCATCAACGCCAAGGCGACGTTGAAACAGCC
ATCGAACAATACGAAAAAGCGGCAGCACTCGGCGTAACTGCCGCCTGCTGGCAACTTGGT
CAAATCTACTTCTACGGTACAGGTGTCAGCCCCAACCACGCACAAGCCGAACACTATCTC
GAACCAGCCGCACAAGCCGGCCACATCGCCGCACAAACGCTGCTGGCTGACCTTCTTGCC
GCCCAACGCAAACCTGAAGCCTTGGAATGGTATCGTCGTGCCGCCGATAAGGAACAAGCG
GAAGCACAGTCTAAGCTGGCCCAATACGCCCTGACCGGCGAACTTTCCGAACGCGATCCG
TTCCAAGCGGCACGATATGCCAAAGCCGCTGCCGAGAAAAACCATCCTGAAGCCCTGAAA
ATCATGGGCGACCTCTACCGCTACGGTCTCGGTATCAAAGCCGACAACCATATCGCGCAA
GATTACTACCACCGTGCCGCCGCGCTGGGTTCTGCCGCCGCAGCACAAAAACTCATCAGC
GACGCCGCGCTGTACCATCCGCAACAATACGAACAAATCAAAACTGCCGCCTGCAACAAC
AACAAACCGAAACCATCTACCGTTTGGCGGAAGCACAAGCCTGCGCCATCGGCCGTCCCG
CCGACTACAATGCCGCGCGAAAAAATTACATGGAAGCTGCCGGGTTCCACCATAAAAACG
CAGCGGCAGCCTTAGGCCGCATCTACCATTACGGCCTCGGTACGGCGCAAGATCCTCGGG
CGGCTGCACACTGGTACGCCATTGCTGCCGAACAAAACCACCCTTCCGCCCAATACCACC
TCGCCTGTTTTTACTATCACGGGCAAGGTGTCGGCTGTCATGTTCCGACCGCCTGCTACT
GGCTGCAGGCCGCCATCGGCAACGGCCACACTTCGGCCGAATCATTAATATCCCTATTAG
AACAATGGCGACGCGAAGCACACCATGCCATCGGACAAAAGGCCGTCTGAAAAGATTTAC
ACTCGCATTTTTTGACAATCTTTAACTATTCCCCTAATATTTGCCAGTTATTTTTCACGG
ACACGCCATTGTTTTCATTTCTTTCTGAAAACACCTTGTCCGCGCATCAATACCATGACA
CTCGGCGGATAACGCCAAGCGTTGAAACACACTACATCCGGAACAAAAACGGATGCTCGG
AAAAATATTTCTAGGAGGTGAAACAACATGGAATGGGAATTCAACAGTTATTACACACTG
ATTGCCGCCACGCTCGTGTTGCTGGTTGGTAAATTTCTGGTTCAAAAAATCAAATTCTTA
CGAGACTTCAATATTCCCGAGCCGGTAGCCGGCGGTTTGATTGCCGCTATCGTCCTGTTC
GCCCTGCACGAGGCGTACGGCGTGAGCTTCAAATTTGAGAAACCGCTGCAAAATGCGTTT
ATGCTGATTTTTTTCACGTCCATCGGCTTGAGCGCGGATTTTTCCCGTTTGAAGGCGGGC
GGTTTGCCGCTGGTGGTTTTTACCGCGATTGTGGGCGGATTTATCTTGGTGCAAAACTTT
GTCGGGGTCGGACTGGCTACGGCTTTGGGTTTGGATCCGCTCATCGGTCTGATTACCGGT
TCGGTGTCGCTGACGGGCGGACACGGTACGTCAGGTGCGTGGGGACCTAATTTTGAAACG
CAATACGGCTTGGTCGGCGCAACCGGTTTGGGTATTGCATCGGCTACTTTCGGGCTGGTG
TTCGGCGGCCTGATCGGCGGGCCGGTTGCGCGCCGCCTGATCAACAAAATGGGCCGCAAA
CCGGTTGAAAACAAAAAACAGGATCAGGACGACAACGCGGACGACGTGTTCGAGCAGGCA
AAACGCACCCGCCTGATTACGGCGGAATCTGCCGTTGAAACGCTTGCCATGTTTGCCGCG
TGTTTGGCGTTTGCCGAGATTATGGACGGCTTCGACAAAGAATATCTGTTCGACCTGCCC
AAATTCGTGTGGTGTCTGTTTGGCGGCGTGGTCATCCGCAACATCCTCACTGCCGCATTC
AAGGTCAATATGTTCGACCGCGCCATCGATGTGTTCGGCAATGCTTCGCTTTCGCTTTTC
TTGGCAATGGCGTTGCTGAATTTGAAACTGTGGGAGCTGACCGGTTTGGCGGGGCCTGTA
ACCGTGATTCTTGCCGTACAAACCGTGGTGATGGTTTTGTACGCGACTTTTGTTACCTAT
GTCTTTATGGGGCGCGACTATGATGCGGCAGTATTGGCTGCCGGCCATTGCGGTTTCGGC
TTGGGTGCAACGCCGACGGCGGTGGCAAATATGCAGTCCGTCACGCATACTTTCGGCGCG
TCGCATAAGGCGTTTTTGATTGTGCCTATGGTCGGCGCGTTCTTCGTCGATTTGATTAAT
GCCGCGATTCTCACCGGTTTTGTGAATTTCTTTAAAGGCTGATTTTCCGCCTTTCCGACA
AAGCACCTGCAAGGTTTACCGCCTGCAGGTGCTTTTGCTATGATAGCCGCTATCGGTCTG
CACCGTTTGGAAGGAACATCATGTATCGGAAACTCATTGCGCTGCCGTTTGCCCTGCTGC
TTGCCGCTTGCGGCAGGGAAGAACCGCCCAAGGCATTGGAATGCGCCAACCCCGCCGTGT
TGCAAGGCATACGCGGCAATATTCAGGAAACGCTCACGCAGGAAGCGCGTTCTTTCGCGC
GCGAAGACGGCAGGCAGTTTGTCGATGCCGACAAAATTATCGCCGCCGCCTACGGTTTGG
CGTTTTCTTTGGAACACGCTTCGGAAACGCAGGAAGGCGGGCGCACGTTCTGTATCGCCG

## Appendix A

ATTTGAACATTACCGTGCCGTCTGAAACGCTTGCCGATGCCAAGGCAAACAGCCCCCTGT
TGTACGGGGAAACTGCTTTGTCGGATATTGTGCGGCAGAAGACGGGCGGCAATGTCGAGT
TTAAAGACGGCGTATTGACGGCAGCCGTCCGCTTCCTGCCCGTCAAAGACGGTCAGACGG
CATTTGTCGACAACACGGTCGGTATGGCGGCGCAAACGCTGTCTGCCGCGCTGCTGCCTT
ACGGCGTGAAGAGCATCGTGATGATAGACGGCAAGGCGGTGAAAAAAGAAGACGCGGTCA
GGATTTTGAGCGGAAAAGCCCGTGAAGAAGAACCGTCCAAACCCACGCCCGAAGACATTT
TGGAACACAATGCCGCCGGCGGCGATGCGGGCGTACCCCAAGCCGCAGAAGGCGCGCCCG
AACCGGAAATCCTGCATCCTGACGACGGCGAGCGTGCCGATACCGTTACCGTATCACGGG
GCGAAGTGGAAGAGGCGCGCGTACAAAACCAGCGTGCGGAATCCGAAATTACCAAACTTT
GGGGAGGACTCGATACCGACGTGCAAAAAGAGTTGGTCGGCGAACAACGCAAGTGGGCGC
AGGAAAAAATCAGCAACTGCCGACAAGCCGCCGCGCAGGCAGACCGGCAGGAATACGCCG
AATACCTCAAGCTGCAATGCGACACGCGGATGACGCGCGAACGGATACAGTATCTTCGCG
GCTATTCCATCGATTAGGGGCAAACCGATGAATACCGTCCCAAAAAGCAGGATTCCCGTC
AAACCGCTGCCCGAAAAAACCACAGACGAAGCCAAAGTCGAAAAATGGCGGCAGCTCGGT
GCGGAACACGGTTTGTCGGGCGAATGGGCAGTTGCCGTCAGATTGGGCGAAAACGGTTTT
ACCGAAGAACAGATGGAAAATATCGCCAACCTGTTCGGCAGATAAAGAGAAAATTGACGG
AAATGCCGTCTGAAACCCTGTTATCGGTTTCAGACGGCATTTTGACCAATACGGTACGCA
GGCGCAAAACAGCCGGCTTTTCCTGTGTTGCCTATGCTGATGTTTCAACACACAGGACGA
TACAAAAAACGTCGCCCTATGTGCCGTCCTGATTCGGAAGGGTTACGCTCCTTCCAAATA
TAGTGGATTAACAAAAACCGGTACGGCGTTGTCTCGCCTTAGCTCAAAGAGAACGATTCT
CTAAGGTGCTGAAGCACCAAGTGAATCGGTTCCGTACTATCTGTACTGTCTGCGGCTTCG
TTGCCTTGTCCTGATTTTTGTTAATCCACTATAAATCGAGCCTAAAACAATGCCGTCTGA
AACGGAAATCTGTTTCAGACGGCATTGTTACATTCAAACGGCGGGCCGTTTATTTGAATT
TGTAGGTGTATTGCAGACCGATGATGTCGGCGTGGTTTTTGAAACGTGCGGAAGACGCGC
CTTTGCTGTCCACATCGTTGCCGCTTGCCTTCGCCGTGCGGTAGCTGGTGTCGTTGATGT
GGATGTGGGTGTAGGCGGCATCGACGACGTGGTTTTTACCGATATGGTATTTCATACCGG
CGGAGAACCAGATGCGGTTGCCGTCGGGTAGGCTGTTCATGCGGTAGTCGGCGTTGCGGA
CGGGCGATTTGTCAAAAGCGATGCCGGCGCGCAGTTGCAGCGGTTCGCTGATTTGATAAG
AACCGCCGAAGCCGACTTTGTAGGTGTTGCGCCAGTTGGGGGTGATGGTGGTGCGGTCGG
ATTTGCCTTTGACGACGGTTTTTTCTTTTTCAAAAACCAGTTCCGCCTTATCGAAGCGGC
TGTGGCGCGTCCAAGTTACGTCGCCGAACAGGTCGGCTTTATCGGACACTTTGTACATAC
CGTGTACGGACAAAGACTCAGGCGTAACGATTTTAACGCGGGCTTTTTCATTCGCCGTGT
AGCCGTTTGCTGCAAGCATCGTACTCCACATTGCTTTCGCCGCCGCGCCGTCTGCCGCCC
ATTCGGCATCGCCTTTGAGCGTGTGCGAGACTTTGGAACGGTAGTTCACGCCCACGCGCG
CACGGTCGTTGATGTCCCACATCCACGCCAGTTGGTAGCCGAAGCCCCAATCGCTGCCTT
TGACATCGGCGTGTCCGTCGGCCTGAATTTTTGCAGCTTCGGCTACACCGTTAGGTTTGG
GCGGTTTTGCCGTCAATATCTCTGCTTTACTCTTAATCCCCCAGTCGGCATATTTGCGCA
GTTCGGCGGAAGTATGTTGGGCGATGATGCCTGCGCCGAAGGAATGGCGGTCGTTGAGTT
TCCACGCGGCGACAGGTTCGACGGCGATGCTGGTCAGACCGAGTTTGTTGATGTTGTGGC
GCAACACGGAATCTTTTTCGTATTCGGTGGCAGAGCCGAAGGGGACGTACACGCCCAAGC
CCACGGTCAGATTGTCGTTGACTTTGTATGCGCCGTAGATGTGGGGCGCGACCGTGGTTT
TGGTGATTTTGCCGCTTTTCGAACCTTGGACGGGAAGCCCGGTAAAGTCGGTGGCGGAAT
CCGCCTCATAATGAATGCTGGGCAGCACGATGTTGGCGTTGACGGAAATCTGGCTGCTGT
CGAGTTTGGTCAGGCCGGCAGGGTTGTAGAAGATGGTCGATGCGTCGGCGGCTTCTGCGG
CGGCGGCATTTGCCGTGCTTTGCGCGTTGACCGACTGTGTGCCGAAGTGGTAGCCGGATG
CGTGGACGGATGCGGCGGCAAAGGCAGTGCCGAGCAGCAGGACGGTTTTTTTCAGTGCGG
AAGGGGTCATTTCGGTTTCCGTAAAAAGGCGGACGGTGGATAAATATAGTGGATTAACAA
AAATCAGGACAAGGCGACGAAGCCGCAGACAGTACAGATAGTACGGCAAGGCGAGGCAAC
GCTGTACTGGTTTAAATTTAATCCACTATAAAAAAGGCAGTCGGAAATGCCTTGTTTCGC
TTTAGTATAGGTACTCGATTTTATCCGATGTTGCCGGATTTGCACAATTTTTTCAGAGTT
TGCCCGAACCGCCGCGCCGCCGCAAAAAATGCCGTCTGAAGCCTCGGGCATCGGCTTCAG
ACGGCATTTTCCACTCAGGGCGGATTATTTGACGCGCAGCACTTCCAGTGTGTTGGTCGA
ACCGGATTCGCGCATTTGCGAACCGCTGGTAATGATGTATTGGTCGCCGGAATGCAGGAT
GTTGTGTTCCACCAGCATCGTTTCGACTTCGTTTAACGCCGTGTCGTGGTCGGTACTGGT
TGCCAAAATCAGCGGGCGCACGCCCCGGTACATCGCCATACGGCGTTGGGCGGAAACGCT
CGGGGTCAGCGCGAAAATCGGCAGGGTGATGTTGTGGCGGCTGATTTCAAAGGCGGTCGA
ACCGCTTTCGGTCAGGGCGACGATGGCTTTGGCGTGAACCGCGCGCGCCACGCTGACCGC
ACCGCCGGCAACCGCCAGGTTGGTGCTGACCGCTTCGGGATACTCGACCTGTTCGGCAAC
GCCGTTGAGCGAATCCTGCTCTTTTTCCGCAGCCGCGCAGATAATCGCCATTTGGCTGAC
GGTTTCAAACGGATACGCGCCGACGGCGGTTTCGGCGGAACACATCACCGCATCGGTACC
GTCCAATACCGCGTTTGCCACATCGCTGACTTCCGCGCGGGTCGGTACGGGGTTGGTAAT
CATCGATTCCATCATTTGCGTCGCCGTAATGCTGAAGCGGCGCAACTCGCGGGCGCGGCG
GATCATCCGTTTTTGCAGGGCGGGGACGGCGGCGTGTCCGACTTCGACCGCCAAGTCGCC
GCGCGCAACCATAATGCCGTCGCCGGCGAGGATGATTTCGTCCAAGTTTTCAATCGCTTC
CACGCGTTCGATTTTGGAAACCAAACCGGGGCGCACGGCCGTGCTGCCCTTCATTTCTTC
TTCGACTTTGGCGCGCGCGATATGCAAATCTTCGGCGGATTTCACAAAGCTGATGGCGAG
GTAGTCGCAACCGATGGCAATCGCGGTTTTCAGGTCGCGGAAGTCTTTTTCGGTCAACGC
GCCTGCGGACAGACCGCCACCGCGTTTGTTGATGCCCTTGTTGCTTTTCAGGACGTGGCT
GTTTTCCACCCTTGTGATAATCCTGCTGCCTTCGACGGATTCCACGGTCAGGGTCAGCAG
GCCGTCGTCCAGCCACAAGACATCGCCTGCGGCAACGTCGTCGGGCAGGTCGCGGTAGTC
CAAACCGACCGCCTCGCGCGTGCCTTCGCCTTCGAGCGCGGCATCCAGTACCAGCGTTTC
GCCTTTGTTCAATTCGATGCCGCCGCCGGCGATTTTGCCCACGCGGATTTTCGGGCCCTG
CAGGTCGGCAATGATGGCGATTTCCTGTCCGGCGCGTTTTGCCGCCTCGCGCACGATGAG
GGCGTTTTCCTGATGGAATTCGGGCGTGCCGTGGCTGAAGTTGAAGCGGACGACGTTCAG
ACCGCCGACGCGGATCATGTCTTCCAACAGTTCGACGTTGTTGCTGCCCGGCCCAAGGGT

# Appendix A

GGCGACGATTTTAGTGTTGTGGCTGATGCGGGTCAGATCGCGGCTTGTCTGGTTCATATG
AAAGTCCTTTTGGTCTCAATCGGGTGTTTTGCGGTATTTTGTTACAAAATTACAGAAATT
TGGAACCGGTTTGATGTCCATTTGATGAACGCGGCGGAATATTCTGTAAAAATATGATTT
AAATTAATAGTTTGATATTTTACCTGCAAACCGCCTTTTTTGGCGCAAAATTACACGGTT
TTATGACTTAGGCTAAATTTATTTTGGGGCTGTCCTAGATAACTAGGGAAATTCAAATTA
AGTTAGAATTATCCCTATGAGAAAAAGTCGTCTAAGCCGGTATAAACAAAATAAACTCAT
TGAGCTATTTGTCGCAGGTGTAACTGCAAGAACAGCAGCAGAGTTAGTAGGCGTTAATAA
AAATACCGCAGCCTATTATTTTCATCGTTTACGATGACTTAATTTATCAAAACAGCCCAC
ATTTAGAAATGTTTGATGGCGAAGTAGAAGCAGATGAAAGTTATTTTGGCGGACAACGCA
AAGGCAAACGCGGTCGCGGTGCTGCCGGTAAAGTCGCCGTATTCGGTCTTTTGAAGCGAA
ATGGTAAGGTTTATACGGTTACAGTACCGAATACTCAAACCGCTACTTTATTTCCTATTA
TCCGTGAACAAGTGAAACCTGACAGCATTTTTTATACGGATTGTTATCGTAGCTATGATG
TATTAGATGTGCGCGAATTTAGCCATTTTAGCTTCGCTGAAACTTCGTTTTCGTATCAAT
CACAGCACACATTTTGCCGAACGACAAAACCATATTAATGGAATTGAGAACTTTTGGAAC
CAGGCAAAACGTCATTTACGCAAGTCTAACGGCATTCCCAAAGCGCATTTTGAGCTGTAT
TTAAAGGAGTGCGAACGACGTTTTAACAACAGTGAGATAAAAGTTCTTGTTCCATTTTAA
AACAATTAGTAAAATCGAGTTTATCTTAGTTATCTAGGACAGCCCCGTTTGTGTACTGAA
ATGCTTCAAAACACCAAACCAAGTTTCGTTTTCTAAAATACGAAACCATTACTGCTGCCT
AAATTTTTTTGGATTGCTAAATTATGGCAGTATGATTTTGGATTTTAAATTGAAAGGCAA
GAAAAATGTCAAAAAATGATGTAGTTAAAGTAATTGGTATATTCCCCCTATTGTCCGAAC
AATAGAGCAGACTTCCCGGCAGGCTGCCCACATCAGAACGCCCGTTCGCTGGTTTGTACG
TCCTGAAAAAGCTCTTGCATTAAGTTAATCATAATGGGAAATTTAAATTTTTTTAATGCT
TACTTAAACAAAAGCCCCACTCCACCATTAGGAGTTTCTTTTTCAGTATACAAGTAAATA
TTTTTAAAATATTGATTTAATTTAAAATAAAATACTTGCAAAAAAAGTATTAAATTAAAC
TTAAGAAAGGTTAATTCTGATTTACATTTCCAACCATACTTCTTTACAGGAGAAAATCAT
GAAAGAGTTACACACCTCTGAATTAGTTGAAGTGTCAGGTGGCAAATTCCATATCTTTGC
ACAGGGTGGCGGCAACCTAGGTAAAAAAGATATGGTTGCTGTTGGTAAAATTGGTGCTTC
CTATTCCCCTAACAATAGTGGAGTAGAGTTTTCTGTTAGCAAGCAATTTGGATATGTACA
AGGTCTTGGTGTACAGTTTTCGAAACCTACTTTTGGTATTAGTAAAAAATGGTAAGATTT
TTTGTTTTATCCTTTCTGACATTAATAAATCTATGCTCATTAAGCGCATGCAATAGCCAC
TTTACAGGAAATATCAATCCATTAGGTACTCACAATAAAGTTGCTAATCCCAATTGTGCC
AATAGTGCCAATAGTCATATCAGACAACCCAGTAGGAAAAACTATGATCCAACTGAATAT
AGTGCTTGGTTACAGTATATGCATGATTGCAAATAATGAGTAACGATGAAAATTTACTTT
TTTCTCAACCACACTTAACAAAGGTGAATATTATGCAAGTTTTGACTTTGAATGAAATTT
AACAAGTTTCTGGTGCTGCTTGTAACTGGCGTGATTTCTCAAAAAATACCATTGGTAGTG
CATTAGGTGGAGCAGCTGGTGGGGCAATTGTTGGTTCATTTGCAGGTGGTATTGGTGCTA
TTCCAGGTGCGAAATTCGGAGCTATTGGTGGTGCAATCACTGGTGCTGTACAATATGGAA
GCACTTGTTGGTGGTAATATTCCTTAATAAAACTAGGGTATTTTGATATTTTCTATTCAA
AATACCCTAGTTTTTCATAAGAACTTAAATACAAAAAGGAACAAATAATGAAAAAATATA
GTGATTATTTTAAATATTTAATCTTTTTTTTGATTTTACTCCCAACAAATTATCTCGTAT
CTCATTATGTGGTACAAACCTCAATGAGTATGTTAAGCATTTTAAGTTCTTCTATAATAA
CAACTTGCCTTTTTTTTGTTTTTACAAATTTATCCCAATCAAAGAAACATAAATAAGTAC
ACATGTCTAACAATCACTCATTTTTCAGACCAGAAGTCTTTGTAGCTCAACGGAACAAGT
GGACAGGACCAGTAGGCTGGGTTGACGCAATGGGAGCTGGTATTTTCTCTGTTGCTGGCG
GATACAATATCGGTCGTGGCATGATGAAGCCATAAGATAATTACATCATTAAGGAAAAGG
TAATTTCAGTTACAGCAATATGTATTGAAGTTACCTTTTTCTATTTAGATTGAACAATTT
TGAAAGAGAAAAATTATGAATACTGAAACCATTTACGCCACTGTCTTTTGCATTTTAGCT
GCAACCATTTCTGGATTATTGGTTAAATTTAATGTAATTAAAATAGAAACATCAATCAAT
AGCAAATTTATGTTATTAGGCATAAGTATTTTAATTATTGGTATTTTTCTATCCATTTTT
TTTTAAGAAATAATAATAAATGTCCCACTTATTCCGAAAAGAAGTCTTTGTAGCCCAACA
AAATAAGTGGACAGGTCAGGTTATCTTGACCCGTCCATTCTCTTTTTTATTTCTGACTTT
TTGCGCTTTTCTCATTGCTCTGTGTATCATTATCTTTTTGATTTTTGGTAGCTATACCAA
TAAAACAACCGTTGAAGGTCAATTACTTCCAACTATGGGGGTGGTTCGTGTTTACTCTTC
CGATATCGGCACGATTACGCATAAATTTGTTGAAGATGGTAACTTTGTCAAAGCTGGCGA
ACCATTGTTCAAACTTTCCACATCGCGTTTTGGCGAAAAAGGAAACGTACAAGCCAAATT
GGCAGCAGAAGCCAACCTTAAAAAAACTTTGGCATTACAAGAATTGGAACGTTTAAAGCG
CATTCATCAAAATGAGCAAAAAAATGTTCATAACAACATTCATCGTTTAAACAATCAATT
AGAGAATATTAAACAGCAAATTACAGGGCAAAATCGTCAAATTCGTTTAGCGGAAAAAAC
CCTTAACAAGAACAAGTTTTTAGCCAGTCAAGGCGCAGTATCCCAACAAGATAAGATGAC
CGCCGAAAGCCATTTATTGGAACAACGCTCACGTTTGGAGAGCCTAAAACGTGAACAAAA
TAATGCAATCAGGGAACTTGATGAACAGAAAATCACATTAAGCAGCCTGCCTGAACGCCA
TAAAACCGAATTGAGCCAACTCAACCGTGCGATTACGGAAATGAACCAAGAAATTTTGGA
TTTTGATTTGAAATCCGAACAAACCATACGAGCTAGTAAATCAGGTTGAGACCTTTGCAA
AAATAATCTGTTAACGAAATTTGACGCATAAAAATGCGCCAAAAAATTTTCAATTGCCTA
AAACCTTCCTAATATTGAGCAAAAAGTAGGAAAAATCAGAAAAGTTTTGCATTTTGAAAA
TGAGATTGAGCATAAAATTTTAGTAACCTATGTTATTGCAAAGGTCTCAGGTTATATATC
AACAATTAATGTTGATATAGGGCAACAAGTTGAACCGTCTAAATTGCTGTTAAGCATTGT
CCCTGAACAAACTGAATTGGTCGCCAATCTTTACATACCCAGTAAAGCTGTTGGTTTTAT
TAAACCGAAAGATAAAGTTGTTTTACGTTACCAAGCGTACCCTTACCAAAAATTTGGACA
TGCCACAGGAGAAATTATTTCAGTTGCCAGAACTGCTCTCGGTAAACAAAAGCTATCAGG
TTTAGGTATCATTTTCACTAACCCAACCTTATTAAATGAACCTGCCTATCTTGTGAAAGT
TAAATTGGAAAAACAAACGATTAAAGCATACGGAGAAAACAAGCCGCTTCAAATTGGCAT
GATTTTAGAAGCAGATATTCTCCATGAACGAAAAAATTGTACGAATGGGTACTTGACCCA
CTTTACAGCATTTCAGGAAAAATCAATTAAAAATGGATTATTTATCAAGACTGTCCTTTG
GATTTAACAAAAAGCTACCTGTCATTCTGCAAACAGAAGTTGCTGAATGTGGTTTAGCAT

## Appendix A

GCCTGACATCCATCTTGTCCTATTATGGCTTTCACACTGATTTAAGAACGTTACGCCAAA
AATACACCCTGTCATTAAAGGGCGCAAATCTTGCAGACATCATGAGATTTGGCAATGAAA
TGAATTTAACGCCACGAGCTTTGCGTTTAGAGTTAGATGAGCTGTCAAATTTACAACTAC
CCTGCATTCTCCATTGGAACTTAAACCATTTTGTTGTACTTTGTTCCATTTCCAAAGACA
GTATCGTCATTATGGACCCTGCTGTCGGTATGCGAAAAATCAAAATGGACGAAGTTTCAC
AAAAATTCACAGGGATTGCCCTAGAATTATTCCCCAATACCCATTTTGAAGAGAAAAAAG
AAACAAAGAAAATCAAAATATTATCTCTATTAAGGGGGGGTCAGGCTTAAAACGCTCTTT
AATTCAAATGCTTATATTAGCTATTTCTTTGGAAGTCTTTGCATTGGTTAGTCCATTCTT
TATGCAATGGGTAATAGACCATGTCATTGTAACTGCTGATAAAAATTTATTATTGACCCT
TACTTTGGGATTTGGTTTACTGACTATCCTGCAACAGTTAATTAGCCTGTTACAAGCATG
GGTAGGTATGCACCTATCTACAACTCTTAATTTACAATGGAAAGCCAATATATTTAAAAG
GTTACTTGACTTACCTAATGACTATTTCAGTAAACGACATTTAGGAGATGTGATTTCAAG
ATTTGGTTCAATAGATCATATCCAAGAAACACTAACTTCTACTTTTTTTGTTTTAGTTTT
AAATAGCTTAATGGCTGTTTTTACTTTCGTGTTAATGACAATTTACAGCACTCAATTATC
GCTGATTGTTCTTTTAACACTTGTTTTGTACATACTAATTCGTTGGCTTGCATATTACCC
ATTAAGAAATGCAACAGAAGAAAATATTGTTCATGAAGCCAAACAAAACTCATATTTCAT
GGAAACCATTCGTGGTATCCAATCAGTTAAATTATTTGATAAACATTATCAAAGACATGG
CACTTGGATGAGCCTATTTGTGAATACAGTCAATACCAAGCTGACAACAGATAAACTCTC
TGCTTTATTTGAATTTTCAAATAAACTGTTGTTTAGCATGGAAAATGTTATCATAATTTA
TCTTGGTGCAAGCGCAATTTTAGATGGTTCATTTACAGTCGGTGTTCTGATGGCTTTTTT
GGCTTATAAAGGGCAATTTGAAAGCAGAACAGCTTCTCTCGTTGACCAATACATCCAAAT
CAAAATGTTAGGGCTTCATGCTGAACGTTTGGCTGACATTACTTTAAATGAAACAGAAAC
TGAAATTATTAAGTATAATCATATACCTAAATTAGATAATGAACAACTGGTTCTTAAAGT
TGAAAACGTCTCATTCAGATATGCTGATAATGAGCCATATCTTTTTGAAAACATTAATTT
GGAATTTAAAGATAATGAAGCAGTTGTTTTAACAGGACAATCTGGTCGGGGGAAGTCCAC
TTTGTTAAACATTTTAACAGGTAGCCTAAAACCTGAAACTGGTACAGTTAGTATTAATGG
GCATGATATATATCAAGTTTCTCCATCCTTTATTAGGGGATTGAGCGGGATTGTTCGCCA
AGATGATGTCCTTTTTGCAGGTTCTATTGGGGAAAATATTTCATTTTTTGATGAAAGCCC
AAATATGGAGCTCATTGAACAATGTGCAAAAATGGCACAAATACATGACGATATACTTAA
AATGCCAATGGGCTATGAGACCTTGATTGGCGATATGGGAAATATCTTATCAGGTGGACA
AAAGCAGAGAGTTATCTTGGCTCGTGCATTGTATAAACGACCCAAAATTCTATTTTTAGA
CGAAGCAAGTAGCCATTTAGATGTAGAAAATGAACAAAAAATTAACCATAACCTAAAAAG
TCTTGGTATTATGAAAATAATGGTTGCACACCGCCAAGAAACAATTCAATCGGCAGATAA
AATTCTGAATTTAGGTTGAACAGAACAAGACTTCATTTTTCTTTAACAAAAAGTGAAGTC
TTTTTTCAAATAATTTAATAGAATACATGAAAATAGCGGTTTAACGTTCCATTTCCCAAT
CATCACGACTGGCTTTGTGTTTTGGCGATTTTTCAGTTTCCTTTTTCTGTTGAATTTGTT
GTTTTTTCTGCTCTTGTTCCCATTTTTGGGCTAATTTCACGGTCTCATTTTCAGCCCATT
CCATCACGGCACAACGATGTAGCTTTTCTCCGATATCGCCATTAAAGCCAGCTCCACGAA
CTTCACCATAAATTCTTGAATATTTTTGATTATATTCAATTTCTTTTCCATTTTCTTTAA
AGGATTTCTCCCACTTTTCACAAACTTCATCAAAATCTTTCAAAGGGATATTTTTTAAGG
GGCTGTCCTAGATAACTAGGGAAATTCAAATTAAGTTAGAATTATCCCTATGAGAAAAAG
TCGTCTAAGCCAGTATAAACAAAATAAACTCATTGAACTGTTTGTCACAGGTGTAACTGC
AAGAACGGCAGCAGAGTTAGTAGGCGTTAATAAAAATACCGCAGCCTATTATTTTCATCG
TTTACGATTACTTATTTATCAAAACAGTCCGCATTTGGAAATGTTTGATGGCGAAGTAGA
AGCAGATGAAAGTTATTTTGGCGGACAACGCAAAGGCAAACGCGGTCGCGGTGCTGCCGG
TAAAGTCGCCGTATTCGGTCTTTTGAAGCGAAATGGTAAGGTTTATACGGTTACAGTACC
GAATACTCAAACCGCTACTTTATTTCCTATTATCCGTGAACAAGTGAAACCTGACAGCAT
TTTTTATACGGATTGTTATCGTAGCTATGATGTATTAGATGTGCGCGAATTTAGCCATTT
TAGCTTCGCTGAAACTTCGTTTTCGTATCAATCACAGCACACATTTTGCCGAACGACAAA
ACCATATTAATGGAATTGAGAACTTTTGGAATCAGGCAAAACGTCATTTACGCAAGTTTA
ACGGCATTCCCAAAGCGCATTTTGAGCTGTATTTAAAGGAGTGCGAATGGCGTTTTAACA
ACAGTGAGATAAAAGTTCTTGTTCCATTTTAAAACAATTAGTAAAATCAAGTTTGTCCTA
GTTATCTAGGACAGCCCCTTGTTTTTTGTTCGGCGGCTTGCGTGGTCGGGTAAAATGAAA
GTTTTGAACGGTTGGTCGGACAGGAAGATGTGGCGGGTTTTGAGTGCTTTGCCGATAGGC
GTGGTGTTTTTTGATTTGATCTACGGTTTTGTGTTGAATGTGTTGCAGGGTTTGGATTTG
CAGCGTGCCGTGCCGGATTCGGAAGGCGTGTTGGCGGTTACGCCCGATATTGCATTCAAC
AGTTTGCAGATTGTCGCCAACGGCGGTATGGCGGCGGTGGTCTGTTTCGGGTTGGCGGTT
GTGTTTTTGCTCAACCGTTCGGTGCGGCGGCGGCAGGTGTTGGAAATCGGGGTGTTCCGG
ATGTTGGGGCTGGTGGCGGTATTGGCGTTCAGCGCGCCGTCGGTGTGGGAGTGGGCGAAC
GCGCTGCCGCTGCTGCTGAAGGGCGCGGACGTGGTCAATACGGGGAATGCGCGTTATGTG
CTGACGGCTTTGTGTATGCCCTTTCCGGCGGTGTCGTGCGTCATCGGGCTGGTGGGGCGG
TTCAGGCTTCAGACGGCATCGGGCAGGGCGGCAAAGTCAGGGGGTGCGGGCAAGGCGGAC
GGATAGGACGCATTTTTCAGCGGGTGCGTCGAGAAGCAGCCGATGTGTTTGGCAGCCGCA
GCTTGGGGGGTGTAGTGCTAATGGCGGTTTCTTTGCTTTTATAGTGGATTAACAAAAACC
AGTACGGCGTTGCCTCGCCTTAGCTCAAAGAGAACGATTCTCTAAGGTGCTGAAGCACCA
AGTGAATCGGTTCCGTACTATTTGTACTGTCTGCGGCTTCGTCGCCTTGTCCTGATTTTT
GTTAATCCACTATATAAAATAAATGGGCAAAAATCGGTTTATTATCGTTTTTGCCGCATT
TGGATTTGTTCTACCGTAAAACGTGTTTGACGAACGGGATTCTTATTAAAAAACATCTGA
TTTCTAACAAAATCAGTATTTTTTGGCACGATGGCTAAAATTTTTCCTTCCATTTCGCCA
TCACGTGTTTTCCATGCGCTCAAGAATTGTGATTTGCTCATTGAGACGTGCCCCAGCGAT
GGATCAGCCAGCAAAACAGTTTCTCCGTTAATACCGTTCAATACCGAAAAATGGTTGTTT
TTACGGTATTTTAAATACACAATTACAGGAATTTTTAGTTGTACCAACTGTTCAAATGGC
AAAGCATAACCTTGTGCTTCAAAACCCAGTTCGGGCATTATGCGTTGCATATCGTCAAAA
GAAGCACGCATTTGGGTTTTATCCATTTTGTCTAAGATTTCCGCTTCAGAATAATGTCTG
CCATAAAAATTATTCAGTAACGTGGCAATCGAAGCCGCGCCGCAAGAAAAATCCAAATCT

## Appendix A

TGTTTTACTATGCCGGAATCTCGCCGTGCTTTCCAACTCCGTACATGGATGTTTTGGTAA
GAAGCGGGGGGTCAACAAACATAGGCCAAGCAAAAACTATATTTGGGGCGAAACCAATCA
AAGCCGCATAATTTATCAATTTATAAAGATTTTTTATCATAATATGTATACGCGGAATAA
ACGCATGAAACATAAAAAAATAAAATCATATGCAATTTTATTGCATGAATAAATATGAAT
AAAATAGATAATGATGGGAGTAAATACGCCATGTATTTTGGAAGTTTAAATTTATTAATA
ATAAAATAATTTATCGTAGCGCAAATAAATCCCAAAATTGGAACGATTAGAAAAAAAATT
AATACACCCATCATCATGCAAACTCTATATTAATAAATAGCTAACTAATTCTATTGTGGA
AATTAGTTAGCTAACTAAAAGTTATTAATGATTATTTTCGAGAATTGACTGCATTGTTGG
CAGCATTGGCACCAAAACCTAGTGCATGAATACCCGGTCTCCATGCCAAATTCCCAGCCA
ATCCGCCTCCGGCAGCAGCAGCCAGCCCTGTTTTGCCGCTACTCCTGTTGCCGCACCGAT
TCCTGTCGCAGTAGCCGCGCCTTGCGCAGTTCCTAATTTACCATGATTATACAAATTAGC
ACCATGATACCCCCATGCACCTAATGCACCGCCAAAAGCAGCGGCTGCAATAATGGGAAC
AAATTCACCTTGTGTTTCTTTCATTTCAGCCTGTGATAATTGAATTGCTTTCACATTTTG
GCTGTCAAAAACTTGGCTGTCTAAATTTTGCGCCATTACAGGTGTAATCATCATAGCCAT
TACAGTTGCAATTTTCGTTGCGCTGGTTTGCACATAAATAGGATTAGCAAATTCGCTTTG
ATTGCGTTCAGTGTTGATGTAGCTAATACTGCTTTCTAGTTTGAATTTACCCTTGTCAGT
CAATAATTCTTCCAAACTTAAAGGTAAGTCAGCATAAGCAATTTGGCTGGCAACAGTCAA
AATAAAATCTATTAGACATTTGTGTTTTTGCATCATTTCGTTTGATTTTCTAGGTTTTGA
GAATGATACAAAGTTTTTTACAAAGTAAAGAGTCACTCTGAAAAAACTTTTTTCATTATA
AATCAAAATATTGATAGAATAAATAGCGAGCATCGATTCACGGTGCGCTTTAGTGCAAAG
CGTACAACGCGAGCCTGAACCACCAGCCGCAACAGGAAAAGAAAGCCGATAGAGTGCAAT
GCTTGCCAACGTGCAAGCGAGCTTGCAAGAACGCTTGGCTCAACGAGAGCAGGCAAGACA
GAAAGCAGAAAGCAGGATAGGAGCGGTAACGCAAAGGTCTCGGGCTTTGATTTCGCCGTA
AACCCTGCTGCCGCCTTGTCCGGAAAGGGTGCAGGCGGCGAGTGCCGACAGGGTGCAGAT
GGGGAGGGGGGTTTTCATTTGGGGTCGCAACGGAAGTGGTATGCGCAGATTTCAAAACCG
TTTTTGAAATACAGGCGGTGCGCGTCGGCACGGTCGTGGTTGACGTGGACGTTGAGGTGG
ATTTTGGTTACCCCTGTTTCCGCGCCGATTTTGCGGACTTCTTCCAAAAGGCGCGAGGCG
TAGCCTTTGCGGCGGCTTTGCGGCAGGGTAACGATGTCATCGATGTGGATGTGGCGGCCG
CTGGCGAGGGTGCAGGCTTCGCGGAAGCCGCAGACGGCGACGGCATTGTGTTTGCCTTCT
TCAAAAATACCCAGCAGGCGGTAGCCTTGGGGGCGTTGGACTTTGTTGATCTGTTCGGTA
AAGCGGTTGATGTCGGTCAGGGCGGAACGCAAAACGCTCAAGGCTGCAAAGGCGGTGGCG
GTGTCGTCCGCGCCGATTTCGCGCAAAACGTAGGATGCGCCCGAGGCGGTCTGTTCCTGT
GCTTTCTCGGCGGCGTGTTTTTCTTCGATTGCCTGTGCCAGCATGACGTGTTCGTCGGCA
GGGTTGTTTTGTCCGCCCTGTTCGCGTTCTTCGAGCAGGGCTTTGCAGTCGATGACGCGC
AGGTCGTTGTCGGCGGCAAAGTCCATCAGGAAGCGGAACATTTGGGGATTGTCGGTTTCC
AGTTTTTTATCGACGGCGACGCAGCGGATGTTGTCCACCAGTATGGGGCGCACCCATTTC
GAATAGGAAAGCCTGTGGTCTTTGGTGAACGAGGACAGAATGCCGCACAGGCGTTCCGCC
CAGTCGCTGGGACGGAAAATCTTGCCGGAACTCGTTGTGCCGTGGATGACGACTTCGTAG
GGGTTGCAGACTAACATGGCGGCTTCCTGAAAAGAAATGTCTAGCGCGATTATACCTTAT
GCTTATGCGGGCGTGTTTGGATATGCCGTCTGAAAAGTACGGGATTCGTGCGGTAAAACT
TTGCGGCGGCAAATGTGCGATAATACGCGCCGTATTGCCGCTTTTGCGAAGCTGTTCCGC
AAACATACGGGCGGCGTGGACGACGTATAACCGGATACCCGCCTGACGCGGGTTTTTTAC
GGAAGGGGGGCAAAAATGCCTAATCCGCTTTACAGACAGCATATCATCTCCATTTCGGAT
TTGTCGCGCGAACAGTTGGAATGCCTGCTTCAGACGGCATTGAAGCTGAAGGCGCATCCG
CGCGGCGACCTGTTGGAAGGCAAACTTATCGGTTCGTGCTTTTTCGAGCCGTCCACGCGC
ACGAGGCTGTCGTTTGAAACGGCGGTGCAGCGTTTGGGCGGCAAGGTCATCGGTTTCTCG
GACGGCGCGAATACCAGTGCCAAAAAAGGCGAGACGCTTGCCGATACCGCCCGCATCATT
TCCGGATATACTGATGCTATCATCCAACGCCACCCCAAAGACGGCGCGGCGCGCGTGGCA
GCGGAGTTTTCGCGCGTGCGGCTTATCAACGCCGGCGACGGCACGAACCAGCACCCCAGT
CAGACGCTGCTCGACCTGGTTACCATTTATGAAACACAGGGACGTTTGGACAAGCTCAAA
ATCGCCATGGCGGGCGACTTGAAATACGGACGTACCGTGCATTCGCTTTGTCAGGCGTTG
AAACGCTGGAATTGTGAATTTGCCTTTGTTTCGCCGCCCAGCCTAGCCATGCCCGACTAT
ATTACCGAAGAGTTGGACGAAGCCGGCTGCCGATACCGTATCCTCGGTAGTTTGGAAGAA
GCGGCGGAATGGGCGGATATCCTGTATATGACCCGCGTCCAGCGCGAACGTTTCGACGAA
CAGGAATTTGCCAAAATCCAAGGCAAATTCAACCTCGAAGCGTCTATGCTCGCCCGCGCC
AAACCGAACCTGCGCGTGCTGCACCCCCTGCCGCGCGTGGACGAAATCCATCCCGATGTC
GATGCCACGCCGCACGCCTATTATTTCGAGCAGGCGACCAACGGCGTTTATGCGCGTATG
GCGATATTGTCGCTGGTGTTGAACGAAGAAGTGTGAGGAACCGATATGGAAACCCCGAAA
CTCAGTGTCGAAGCCATTGAAAAAGGTACGGTTATCGACCATATTCCCGCCGGCAGGGGG
CTGACCATCCTGCGCCAGTTCAAACTTTTGCACTACGGCAACGCGGTAACCGTGGGCTTC
AACCTGCCCAGCAAAACCCAAGGCAGCAAAGACATCATCAAAATCAAAGGCGTGTGCTTG
GACGACAAAGCCGCCGACCGCCTCGCCCTGTTCGCCCCCGAAGCGGTGGTCAACACCATC
GACAATTTCAAGGTCGTGCAGAAGCGGCATTTGAACCTGCCCGACGAAATCGCCGAAGTG
TTCCGCTGTCCGAACACGAATTGCGCCGGCCACGGCGAGCCGGTCAAAAGCCGGTTTTAT
GTTAAAAAGCACAACGGGCAGACGCGGCTGAAATGCCACTACTGCGAAAAAACCTACAGC
CGGGATTCGGTGGCGGAAGCCTGACGGATTCCCTTAAACCGAGTGGGCGGCATTTCGTCT
GCCGCCTGTTTTGCCAATCTGAAATGGAATGATGATGCACGCTTCTGTCCAAAGCCGTTT
CGCACCGATACTTTATGTTTTGATTTTCTTTGCCGGTTTTTTGACCGCGCAAATCTGGTT
CAATCAGAAAGCCTATACTGAAGAGCTGCCTCCGCTTCTGTCCGCATTGTCCGCCGTCGC
GCTGGTGTGGCTGGCGTGGGCGTTCGTGTCGGCGCGTTCAAAGGCCAAGGCGGAAAAGTT
CTACCGCGAAAAAATGATACAGAACGAAAGCATACACCCCGTCCTGCACGCCTCTTTGCA
ACACTTGGAACACAAGCCGCAAATACTCGCCCTGCTGGTCAAAAACCACGGCAAAGGGAT
GGCGGAACAGGTCAGGTTCAAGGCGGAAGTGCTGCCCGACGACGAAGACGCGCGCACGAT
TGCCGCCGAGTTGGCAAAAATGGATATGTTCGCATTGGGGACGGACGCGGTCGCCTCGGG
CGAAACCTATGGACGCGTGTTCGCCGATATTTTCGAGTTGTCGGCGGCTTTGGAAGGGCG

## Appendix A

CGCGTTCAAAGGAATGTTGAAACTGACGGCGGAATATAAAAACATCTTCGGCGATGCCTG
CCGTTCGGAAACGGCGTTGGAGTTGGGCGCACTCAATCAGGCGTTGCAGGAGATTTCAAA
AACATCGGAAAAGTCCAAACGGATATTTTATTGAAGATGGAAAAATGCCGTCTGAAACGG
AAGGTGTTTCAGACGGCATTTTTGTCGGATGATTAATTATTCGGAGCGGTTGAAGCCAAA
CTTCACGCGGCTGCGGCCCTGATCCGGTATATTGTCCAAATCGCGTCCCGGATTGGCGGC
GGTGTCGCCTACGGAAATATCGGAGATGTTTTCCAAAATGATGGCGGACGACAGGTGTTC
GGAGGTGCGGTAAACCATTGCCAAGCCCACTTCTTCGGCAGGAGTGGAAATCAGCTCGAC
GGTATCCCTGCTTTTGAAATTGTTGGAGAGGTCGACCTGCATCGTTTTCTTGCGTTTGTA
GAGGCTCAAAACCGTGCCTTTGTCCAAACCGTCCGCCTCGCCTTTGTCGATGGTGATGGT
TTGAAACTGGCCGGCAATCCTTGTGCCTTCAAACACGGAAACGATTTTAGCCTGAACCGG
GCGGGACGGTTCGTGCGGCATCATGTTGAAGCGGTCGGTGTCTTCCGGCATTTTCATCAG
GTAGTCGCCCTGCTGTATTTCGGAAATGGCGGTTTCGACCACCAGCGGCTGTATCGAAGG
GGTGCGCAGCGGGGTAATCAAAGGATGGGTGCGGGTATGGTATTCGTTGTCTTTCGGCCG
TTCTCCAGCCTGTTTCGAGCGTTGTTCGAGGACAGAGTCGGTATAGTCGAGGGAGCGCAC
GATGCCGCTGAATGCGACTTCCTGCCCGAGGAATTTACCCGTATCCGGATCGGTGATGTT
TTTATTGATTCGGTAGGTCAGGTAGCGGCCCGGCTCTTTCAGGCCTTTGGTGTAAACCCT
GGTGCCTTTGGTGTACAGCAGCCTGCCTTCCGGGCCCGAGAGCAGGCGCGGCGCGGCAGC
GGTTTCTTTGCGGGAAACGATTTGCGGATGCCGCATAAAGATGCGGTAGAAGTTGACATC
GATGGCGGGAATACCGTATCCGGACACTTCCTTATCCGGACTCATTTTGACGACGGGGAT
GCCGTCTGTCTGTTCCAAGCCGAGGCGCGGTTCGCCGTCAACGTGGCGCAACACCAATAC
CTGGTCCGGATAAATCAGGTCGGGATTGTGGATTTGATCCCGGTTCGCGTCCCACAGGCG
GCCCCATTGCCACGGGCTGTACAGGTATTTGCCCGAAATGCCCCACAGGGTGTCGCCCTG
TTTGACCGTGTAGCGTTCCGGCGCGTTCGGGCGCACCTCCAAATTTGCCGCCAAAGTTTG
TGTTGAGAATGCCATACCTGCCGCGCAGAGCAGGGTTATAATACGACGTTGCATAACCGT
TCCCCTTATCTGATAAATTTCGGTTTGTCTTGCTTGATTGGGTTGGAAAAAGCGGCGGCA
GCCCCTCGGGATGTGCCGCGTGATAAAAAATGTTCCGCATTTTAACATCGAATTATCCGC
ACCATCACGGTAATTATGAAAAACAGGCGGCGTATCCGCCGAAGGAAAGAGAAAATTATG
GCTTTATTGAATATCTTGCAATATCCCGACGAGCGTCTGCACACGGTGGCAAAGCCTGTC
GAACAAGTCGACGAGCGCATCCGGAAGCTGATTGCCGATATGTTTGAAACGATGTACGAA
TCGCGCGGCATCGGGCTGGCGGCGACGCAGGTCGATGTGCACGAGCGCGTGGTCGTGATG
GATTTGACCGAAGACCGCAGCGAACCGCGCGTGTTCATCAACCCCGTCATCGTTGAAAAA
GACGGCGAAACCACTTACGAAGAGGGCTGCCTGTCCGTGCCGGGCATTTACGACACCGTA
ACCCGCGCCGAACGCGTCAAGGTCGAGGCTTTGAACGAAAAAGGCGAAAAGTTCACGCTG
GAGGCGGACGGCTTGTTGGCGATTTGCGTGCAGCACGAGTTGGACCACCTGATGGGCATC
GTGTTTGTCGAACGCCTTTCCCAACTCAAGCAGGGGCGGATTAAGACCAAGCTGAAAAAA
CGTCAGAAACATACGATTTGACCCTTTTGCCGTGCCGTCTGAACGCTGCAAAGTTTTCAG
ACGGCACGGTCTTGTCCGACAATTTTACGCACGCGCAGGAACACGCTATGAAAGTCATCT
TCGCCGGCACGCCCGATTTTGCCGCCGCCGCCTTAAGAGCCGTTGCCGCCGCCGGTTTTG
AAATTCCGCTGGTGCTGACCCAGCCCGACCGTCCGAAAGGGCGCGGTATGCAACTGACTG
CCCCGCCCGTCAAACAAGCCGCGCTGGAACTCGGTTTGCGCGTCGAACAGCCCGAAAAGC
TGCGCAACAACGCCGAAGCCCTGCAAATGCTCAAAGAGGTCGAGGCAGACGTAATGGTGG
TTGCCGCCTACGGTTTGATTCTGCCGCAGGAAGTGTTGGATACGCCGAAACACGGCTGCC
TCAACATCCACGCTTCGCTGTTACCCCGTTGGCGTGGCGCGGCGCCGATTCAACGCGCGA
TTGAAGCCGGCGATGCCGAGACAGGCGTGTGTATTATGCAGATGGACATCGGTTTGGACA
CCGGCGATGTGGTCAGCGAACACCGCTACGCCATCCAACCGACCGATACCGCCAACGAAG
TCCACGACGCGCTGATGGAAATCGGTGCGGCGGCGGTTGTTGCCGATTTGCAACAGCTTC
AAAGCAAAGGCCGTCTGAACGCGGTCAAACAGCCCGAAGAAGGTGTTACTTACGCGCAAA
AATTGAGCAAAGAAGAGGCGCGTATCGATTGGAGCAAAAGCGCGGCGGTTATCGAACGCA
AAATCCGCGCCTTCAACCCCGTGCCTGCCGCGTGGGTTGAGTATCAGGGCAAGCCGATGA
AAATCCGGCGGGCGGAAGTGGTGGCGCAACAAGGCGCGGCAGGCGAAGTGTTGTCCTGTT
CGGCGGACGGTTTGGTCGTTGCCTGCGGCGAAAACGCGCTGAAGATTACCGAATTGCAGC
CTGCCGGCGGCAGGCGGATGAATATCGCGGCGTTTGCAGCAGGACGGCATATCGAAGCAG
GGGCGAAGCTGTAAATCCCTTCAGACGGCATTCCGATCCGCAAACGGGAATGCCGTCTGA
AACCATCAGTCGAAGAAAGCGAATCACATAATATGAGTATGGCACTTGCCCAAAAACTTG
CCGCCGACAGCATTGCGGCGGTTGCCGAAGGACGTAACCTTCAGGACGTGTTGGCGCAAA
TCCGCACCGCGCATCCCGACCTTATGGCGCAGGAAAACGGCGCGTTGCAGGACATCGCCT
ACGGCTGCCAGCGTTATTTGGGCAGTTTGAAACATATGCTCGCGCAGATGCTGAAAAAGC
CGATTGGCAATCCGCAGCTCGAAAGCCTGCTTTTGGCGGCGTTGTACCAGCTGCATTACA
CGCGCAACGCGCCCCACGCCGTGGTCAATGAGGCGGTGGAAAGCATCGCGAAAATCGGAC
GCGGGCAGTACCGTTCGTTTGCCAACGCGGTTTTGCGCCGCTTTTTGCGCGAACGCGACA
AGCTTGTGGCTTCCTGTAAAAAAGACGATGTAGCGAAACACAACCTGCCGCTGTGGTGGG
TGGCTTACTTGAAAAACCATTATCCGAAACACTGGCACAACATCGCCGCCGCGCTGCAAT
CCCATCCGCCGATGACTTTGCGCGTCAACCGCCGACACGGCAATGCCGAAAGCTATTTGG
AAAAACTGGTGGCGGAAGGTATCGCGGCTAAGGCGTTGGACGAATATGCGGTTACGTTGG
AAGAAGCCGTGCCGGTGAACCGCCTGCCTGGTTTTTCAGACGGCATTGTTTCGGTACAGG
ACTTCGGCGCGCAGCAGGCGGCGTATTTGTTAAACCCGAAAGACGGCGAACGGATTTTGG
ACGCGTGCGCCGCGCCGGGCGGCAAGACGGGGCATATCTTGGAACTGGCGGATTGCCGTG
TTACCGCCTTGGACATTGATGCAGGCCGTCTGAAACGGGTGGAAGACAATATCGCGCGTC
TGGGCTTTCAGACGGCATCGACGGCGTGTGCCGATGCACAGGACCTGTCGGCATGGTATG
ATGGGAAACCGTTTGATGCCGTCCTTGCCGACGTGCCGTGTACCGCCTCGGGCGTGGCGC
GGCGCAATCCCGACGTGAAATGGCTACGCCGTCCGACCGACGCGCTCAAAACCGCCCGCC
AGCAGGAAGCCCTGCTAGATGCATTGTGGCAGGTGCTGAAAAGCGGGGGAAGGATGTTGA
TCGCTACCTGTTCCGTGTTCGTCGAGGAAAACGACGGACAATTGCAAAAATTCCTCAACC
GCCATGCCGATGCAGAACTGATCGAATCGCGGGTACTCTTACCGAACAAACACCAAGATG
GCTTTTATTACGCGCTTATTCAAAAGCAGTAAATGGCTGATTGTGCCGCTGATGCTCCCC

## Appendix A

GCCTTTCAGAATGTGGCGGCGGAGGGGATAGATGTGAGCCGTGCCGAAGCGAGGATAACC
GACGGCGGGCAGCTTTCCATCAGCAGCCGCTTCCAAACCGAGCTGCCCGACCAGCTCCAA
CAGGCGTTGCGCCGGGGCGTGCCGCTCAACTTTACCTTAAGCTGGCAGCTTTCCGCCCCG
ATAATCGCTTCTTATCGGTTTAAATTGGGGCAACTGATTGGCGATGACGACAATATTGAC
TACAAACTGAGTTTCCATCCGCTGACCAACCGCTACCGCGTTACCGTCGGCGCGTTTTCG
ACAGACTACGACACCTTGGATGCGGCATTGCGCGACCGGCGCGGTTGCCAACTGGAAA
GTCCTGAACAAAGGCGCGCTGTCCGGTGCGGAAGCAGGGGAAACCAAGGCGGAAATCCGC
CTGACGCTGTCCACTTCAAAACTGCCCAAGCCTTTTCAAATCAATGCATTGACTTCTCAA
AACTGGCATTTGGATTCGGGTTGGAAACCTCTAAACATCATCGGGAACAAATAATGCGCC
GTTTTCTACCGATCGCAGCCATATGCGCCGTCGTCCTGTTGTACGGACTGACGGCGGCAA
CCGGCAGCACCAGTTCGCTGGCGGATTATTTCTGGTGGATTGTTGCGTTCAGCGCAATGC
TGCTGCTGGTGTTGTCCGCCGTTTTGGCACGTTATGTCATATTGCTGTTGAAAGACAGGC
GCGACGGCGTATTCGGTTCGCAGATTGCCAAACGCCTTTCTGGGATGTTTACGCTGGTTG
CCGTACTGCCCGGCGTGTTTCTGTTCGGCGTTTCCGCACAGTTCATCAACGGCACGATTA
ATTCGTGGTTCGGCAACGATACCCACGAGGCGCTTGAACGCAGCCTCAATTTGAGCAAGT
CCGCATTGAATTTGGCGGCAGACAACGCCCTCGGCAACGCCGTCCCCGTGCAGATAGACC
TCATCGGCGCGGCTTCCCTGCCCGGGGATATGGGCAGGGTGCTGGAACATTACGCCGGCA
GCGGTTTTGCCCAGCTTGCCCTGTACAATGCCGCAAGCGGCAAAATCGAAAAAAGCATCA
ACCCGCACAAGCTCGATCAGCCGTTTCCAGGTAAGGCGCGTTGGGAAAAAATCCAACGGG
CGGGTTCGGTCAGGGATTTGGAAAGCATAGGCGGCGTATTGTACGCGCAGGGCTGGCTGT
CGGCGGGTACGCACAACGGGCGCGATTACGCCTTGTTTTTCCGTCAGCCGGTTCCCAAAG
GCGTGGCAGAGGATGCCGTCTTAATCGAAAAGGCAAGGGCGAAATATGCTGAGTTGAGTT
ACAGCAAAAAAGGTTTGCAGACCTTTTTCCTGGCAACCCTGCTGATTGCCTCGCTGCTGT
CGATTTTTCTTGCACTGGTCATGGCACTGTATTTCGCCCGCCGTTTCGTCGAACCCGTCC
TATCGCTTGCCGAGGGGGCGAAGGCGGTGGCGCAAGGCGATTTCAGCCAGACGCGCCCCG
TGTTGCGCAACGACGAGTTCGGACGCTTGACCAAGTTGTTCAACCACATGACCGAGCAGC
TTTCCATCGCCAAAGAAGCAGACGAGCGCAACCGCCGGCGCGAGGAAGCCGCCAGGCATT
ATCTTGAATGCGTGTTGGAGGGGCTGACCACGGGCGTGGTGGTGTTTGACGAACAAGGCT
GTCTGAAAACCTTCAACAAAGCGGCGGAACAGATTTTGGGGATGCCGCTTACCCCCCTGT
GGGGCAGCAGCCGGCACGGTTGGCACGGCGTTTCGGCGCAGCAGTCCCTGCTTGCCGAAG
TGTTTGCCGCCATCGGCGCGGCGGCAGGTACGGACAAACCGGTCCATGTGAAATATGCCG
CGCCGGACGATGCCAAAATCCTGCTGGGCAAGGCAACCGTCCTGCCCGAAGACAACGGCA
ACGGCGTGGTAATGGTGATTGACGACATCACCGTTTTGATACACGCGCAAAAAGAAGCCG
CGTGGGGCGAAGTGGCGAAGCGGCTGGCACACGAAATCCGCAATCCGCTCACGCCCATCC
AGCTTTCCGCCGAACGGCTGGCGTGGAAATTGGGCGGGAAGCTGGATGAGCAGGATGCGC
AAATCCTGACGCGTTCGACCGACACCATCGTCAAACAGGTGGCGGCATTGAAGGAAATGG
TCGAAGCATTCCGCAATTATGCGCGTTCCCCTTCGCTCAAATTGGAAAATCAGGATTTGA
ACGCCTTAATCGGCGATGTGTTGGCATTGTATGAAGCCGGTCCGTGCCGGTTTGCGGCGG
AGCTTGCCGGCGAACCGCTGACGGTGGCGGCGGATACGACCGCCATGCGGCAGGTGCTGC
ACAATATTTTCAAAAATGCCGCCGAAGCGGCGGAAGAAGCCGATGTGCCCGAAGTCAGGG
TAAAATCGGAAACAGGGCAGGACGGTCGGATTGTCCTGACGGTTTGCGACAACGGCAAAG
GGTTCGGCAGGGAAATGCTGCACAACGCCTTCGAGCCGTATGTAACGGACAAACCGGCGG
GAACGGGATTGGGTCTGCCTGTGGTGAAAAAAATCATTGAAGAACACGGCGGCCGCATCA
GCCTGAGCAATCAGGATGCGGGTGGCGCGTGTGTCAGAATCATCTTGCCAAAAACGGTAA
AAACTTATGCGTAGCAGCGATATTTTAATTGTAGACGACGAAATCGGCATCCGCGACCTG
CTGTCGGAAATCCTGCAGGACGAAGGTTATTCGGTCGCATTGGCGGAAAACGCCGAAGAG
GCGCGCAAGCTGCGCCATCAGGCGCGCCCCGCGATGGTGCTGCTGGATATTTGGATGCCT
GATTGCGACGGCATCACCCTTTTGAAGGAGTGGGCGAAAAACGGGCAGCTCAATATGCCG
GTGGTGATGATGAGCGGGCATGCCAGCATCGATACCGCCGTGGAAGCCACCAAAATCGGC
GCGATCGATTTTTTGGAAAAACCGATTTCCCTGCAAAAGCTGCTGTCTGCCGTCGAAAAC
GCGTTGAAGTACGGTGCGGCGCAAACCGAAACGGGGCCTGTATTCGACAAGCTGGGCAAC
AGTGCGGCGATTCAGGAAATGAACCGTGAGGTAGGGGCTGCGGTGAAATGTGCCTCTCCC
GTACTTTTGACGGGCGAGGCGGGTTCGCCGTTTGAAACGGTGGCACGCTATTTCCATAAA
AACGGTACGCCGTGGGTCAGCCCGGCAAGGGTCGAATATCTGATCGATATGCCGATGGAA
CTGTTGCAGAAGGCGGAGGGCGGCGTTTTGTATGTCGGCGACATCGCCCAGTACAGCCGC
AACATCCAAGCCGGTATTGCCTTTATTGTCGGAAAGGCGGAACACCGCCGCGTCAGGGTG
GTCGCATCGGGCAGCAGGGCGGCAGGTTCAGACGGCATTGCCTGCGAGGAAAAGCTGGCG
GAACTGCTGTCGGAATCGGTCGTCCGTATTCCGCCGCTGCGTATGCAGCATGAAGACATT
CCCTTCCTGATACAGGGGATTGCCTGCAATGTGGCGGAAAGCCAAAAGATTGCGCCTGCC
TCATTCAGTGAAGAGGCACTTGCCGCATTGACCCGTTACGACTGGCCGGGAAATTTCGAC
CAACTGCAAAGCGTCGTTGCAACGCTGTTGTTGGAGGCGGACGGACAGGAAATCGGCGCA
GGGGCGGTTTCTTCCCTTTTGGGGCAGAATGTGCCTGCCGAGGGGGCGGAAGATATGGTG
GGCGGGTTTAATTTCAACCTGCCCCTGCGCGAATTGAGGGAGGAGGTGGAGCGGCGTTAT
TTCGAGTACCACATCGCCCAAGAAGGTCAGAATATGAGCCAAGTGGCGCAGAAAGTTGGT
TTGGAACGCACGCACCTTTACCGCAAACTCAAACAGCTCGGCATCGGCGTTTCGCGCCGG
GCGGGGGAAAAAACCGAAGAATAGGCCCGGACGGCCGGTTTACCGGCTGCGGGCTTTTGT
TTTCAGACGGCATTTGGTGCAAATGCCGTCTGAAATCGTAAGGGGACGGATTTTATGACA
GAGGACGAACGTTTCGCGTGGCTGCAATTGGCGTTTACGCCCTATATCGGCGCGGAAAGT
TTCCTGCTGCTGATGCGCCGTTTCGGCAGCGCGCAAAATGCCCTGTCCGCACCGGCGGAA
CAGGTGGCGGCACTGATACGGCACAAACAGGCGCTTGAGGCTTGGCGCAATGCGGAAAAA
CGCGCTCTGGCGCGGCAGGCGGCAGAAGCGGCATTGGAATGGGAAATGCGGGACGGATGC
CGCCTGATGCTGCTTCAGGATGAAGATTTTCCCGAAATGCTGACGCAGGGGCTGACCGCG
CCACCGGTTTTGTTTTTGCGCGGCAACGTGCAACTGCTGCACAAACCTTCCGCCGCCATC
GTCGGCAGCCGTCATGCCACGCCGCAGGCGATGCGGATTGCCAAAGATTTCGGCAAGTCG
TTGGGTGGGAAAGGCATTCCCGTTGTGTCGGGTATGGCTTCGGGCATCGATACCGCCGCC

## Appendix A

CATCAGGGTGCGTTGCAGGCAGAAGGCGGCACCATCGCCGTGTGGGGACGGGCATAGAC
CGCATTTATCCGCCGGTCAACAAAAACCTTGCCTATGAAATCGCCGAAAAAGGATTGATT
GTCAGCGAGTTCCCCATCGGCACGCGGCCGTATGCCGGCAATTTTCCGCGCCGCAACCGC
CTGATTGCCGCCCTGTCGCAAGTAACGCTGGTGGTTGAAGCCGCGTTGGAATCCGGTTCG
CTGATTACTGCCAGATTGGCGGCGGAGATGGGGCGCGAAGTGATGGCGGTACCCGGCTCG
ATAGACAATCCACACAGTAAAGGCTGCCACAAACTGATTAAAGACGGCGCAAAATTGGTG
GAATGCCTGGACGACATCCTGAACGAATGCCCGGGGCTATTGCAAAATACGGGTGCTTCA
TCATATTCTATAAATAAGGGAATACCTGAAAAGCGCATCACTGCCGTTCAGACGGCATCC
GACCAGCTGTCTCTGCCTGAAGGCAAAATGCCGTCTGAAAAGACGGAGAACCGACCCGTC
GGCGGCAGTATCTTGGACAGGATGGGTTTCGACCCAGTTCATCCCGACGTGCTTGCCGGA
CAGTTGGCTATGCCTGCCGCAGATTTGTATGCCGCACTGTTGGAATTGGAATTGGACGGC
AGCGTTGCCGCAATGCCCGGCGGCAGATACCAGCGTATCCGAACTTGAACGCACTTTATA
TTAAGGAACACGAATGACCGAAGTCATCGCCTACCTCATCGAACATTTCCAAGATTTCGA
TACCTGCCCGCCGCCCGAAGACTTGGGTATGCTGCTTGAAGAAGCGGGTTTCGATACGAT
GGAAATCGGCAACACCCTGATGATGATGGAAGTATTGCTCAACAGCTCCGAATTTTCCGC
CGAACCCGCCGACAGCGGCGCATTGCGCGTGTACAGCAAAGAAGAAACCGACAACCTGCC
GCAGGAAGTGATGGGGCTGATGCAGTATCTGATTGAAGAAAAAGCCGTCAGCTGCGAACA
GCGGGAAATCATCATCCACGCGCTCATGCACATTCCGGGCGACGAAATTACCGTAGATAC
CGCCAAAGTGCTGACCCTGCTGCTTTTATGGGCAAACAAGAGCGAGCTGCCCGTGTTGGT
CGGCGACGAGCTGATGAGCGCGCTTTTACTCGACAACAAACCCACGATGAACTGAAGCGG
CTTCAGACGGCCCGCCCGAGTCCGTCTGAAACGTCGGCATCAAAACCACCATCCAGAGAA
CGACAAATGGCGAAAAACCTATTAATCGTCGAATCCCCGTCCAAAGCCAAAACCCTGAAA
AAATATTTGGGCGGCGATTTTGAAATCCTTGCATCCTACGGACACGTCCGCGACCTCGTC
CCCAAAAGCGGCGCGGTCGATCCCGACAACGGCTTTGCGATGAAATACCAACTCATCAGC
CGCAACGGCAAACACGTCGATGCCATCGTCGCCGGTGCCAAAGAAGCTGAAAACATCTAC
CTCGCCACCGACCCGGATAGGGAAGGCGAAGCCATTTCCTGGCATCTTTTGGAAATCCTC
AAATCCAAACGCGGCTTGAAAAACATCAAGCCGCAGCGTGTCGTGTTCCACGAAATCACC
AAAAACGCCGTGCTCGATGCCGTTGCCCATCCGCGCGAAATCGAAATGGACTTGGTCGAT
GCGCAACAAGCCCGTCGCGCTTTGGACTATTTGGTCGGTTTCAACCTTTCGCCATTGTTG
TGGAAAAAAATCCGTCGCGGTTTGAGCGCGGGCCGTGTACAAAGCCCCGCACTGCGTTTG
ATTTGCGAACGCGAAAACGAAATCCGCGCGTTTGAAGCGCAGGAATATTGGACGGTACAT
CTAGACAGCCACAAAGGCCGCAGCAAGTTCACCGCCAAACTCGCCCAATACAACGGCGCG
AAACTCGAACAATTCGACCTGCCGAACGAAGCCGCTCAAGCCGATGTGTTGAAAGAACTC
GAAGGCAAAGAGGCCGTCGTTACCGCCATCGAAAAGAAAAAGCGCAGCCGCAACCCCGCC
GCGCCGTTTACCACATCCACCATGCAGCAGGATGCTGTGCGCAAACTCGGCTTCACCACC
GACCGCACCATGCGTACCGCCCAGCAGCTTTACGAAGGTATTGACGTAGGGCAGGGTGCC
ATCGGTCTGATTACCTATATGCGTACCGACAGCGTGAACTTGGCGGATGAAGCCTTAACC
GAAATCCGCCATTACATTGAAAACAAAATCGGCAAAGAATATCTGCCGAGTGCCGCCAAA
CAATACAAAACCAAATCCAAAAACGCCCAAGAAGCGCACGAAGCCATCCGCCCGACTTCC
GTGTACCGCACGCCCGAAAGCGTCAAACCCTTCCTGAGCGCCGACCAGTTCAAACTCTAT
CAAATGATTTGGCAGCGTACCGTCGCCTGTCAGATGACGCCCGCCAAATTCGACCAAACC
ACCGTCGATATTACCGTCGGCAAAGGCGTATTCCGCGTAACCGGACAAGTGCAAACCTTC
GCAGGCTTCCTCAGCGTTTACGAAGAAAGCAGCGACGATGAAGAAGGCGAAGACAGCAAA
AAACTGCCCGAAATGAGCGAAGGCGACAAATTGCCCGTGGACAAACTCTACGGCGAACAA
CACTTTACCACTCCGCCGCCACGCTACAACGAAGCCACGCTGGTTAAAGCCCTCGAAGAA
TACGGCATCGGCCGCCCCTCGACCTACGCCAGCATCATCTCCACGCTCAAAGACCGCGAA
TACGTTACCCTTGAGCAAAAACGCTTTATGCCCACCGACACAGGCGACATCGTCAATAAA
TTCCTGACCGAACACTTCGCCCAATACGTCGATTACCACTTCACTGCCAAACTCGAAGAC
CAGCTTGACGAAATTGCCGACGGCAAACGCCAATGGATTCCCTTGATGGACAAATTCTGG
AAACCGTTCATCAAACAAGTGGAAGAAAAAGAAGGCATCGAACGCGCCAAATTTACCACG
CAGGAACTTGATGAAACCTGCCCGAAATGCGGCGAACACAAACTGCAAATCAAATTCGGC
AAAATGGGTCGTTTTGTTGCGTGTGCCGGTTATCCCGAGTGCAGCTACACGCGCAATGTC
AACGAAACCGCCGAAGAAGCTGCCGAACGCATCGCCAAAGCCGAAGCCGAACAGGCCGAA
CTCGACGGACGCGAGTGCCCGAAATGTGGCGGTCGCCTAGTGTACAAATACAGCCGCACC
GGCAGCAAATTCATCGGCTGCGTCAACTATCCGAAATGCAAACACGTCGAGCCGCTGGAA
AAACCGAAAGATACCGGCGTCCAGTGTCCGCAATGCAAAAAAGGCAACCTCGTCGAGCGC
AAATCCCGCTACGGCAAACTGTTTTACAGTTGCAGCACCTATCCCGACTGCAACTACGCC
ACTTGGAACCCGCCCGTTGCCGAAGAATGCCTGAACTGCCATTGGCCGGTCTTGACCATC
AAAACCACTAAACGCTGGGGTGTAGAAAAAGTCTGCCCACAAAAAGAATGCGGCTGGAAA
GAACAGATTGAACCGCCCGCGCCGAAGGAGTAAGATTAGGTTGGTTTGAAAGAGAAAAGG
TCGTCTGAAAAATTTTCAGACGACCTTTGCTTTTCTGTGATTGGTTTATTTGAATCCGCG
TGTTGTTTTAAAGTCCGATAAAATCCGGTTCATTTCAGGCGCAAACAAGGCGATGTAATC
GTAAGATAGACCGCGACTGGCACTGGGATGGGGAAAGCAGACGACTTCGCAATCTTCAAA
CGATTGGAATTTGACATTGAAACGTGTACCGTCAAATTCTTTTTGCACCGTCTCCAGCGG
TTTGGTCTGCTTACCGACCAACTGCTCGAAGCGTGGCAGTACATTTTGGTTGTTCAGAAA
ATCCGCCAACCTGCTGCCCATGAAGAGGATGACTTTCGGACGCAGTTTTTCGATGTGGTA
GAGAAAATTATCGATGTGCTCGGGTTGTGTGAACTTGTCGGGATTGTCGATAGTGTTGCC
CTGTGTAGCAGCCCAGTTGGTTTGAACCAGGGATTTTTCAAATGCACCGCCCAATCCATT
TTCGTCTAAGGGGTGTCCCCACATTTCAAACCAATTTTTTATCGTATTGTCGTAACGCCA
CTTTTTTGCCTGCTCTCCGAAATAGAGGGATTTGTTTGCAAATGTATGGTCGATTTTGTT
TTCAGGGAGTTTGTATTCACCTGCTACATAAGCAGCCTCATCGGCTTTACTCCAACCCCA
TTCATAGCCACAAATCATTAAGCCATGTTTGTCGTTGTAGCCTTTGAACAGGCTGTTGCT
CAAATTCAAATCCTTCATCATGAACTCTTCCTTTTAAAATTTAAGAGCGATTGACTTCAA
TGTTTTTAGATGGGGTGGAAAAATCCTTGTGTAGGCAACATAAATTCAATAAATTTCTTG
ATAATTCGAAACCTACTAATAGCGCACCTATAAAAGCTTTTTCATTACGTTCAGCATGAC

# Appendix A

GGTCACGTCGTTCATATTTTTTACGCTTGCTGTTCCCTGTTATTACAGCTAAGCCAAGTG
ATATGGCGAGAATTGCCCAAACAATAGTACTTAATAACAATTTTCCCCATACTATCAATA
AGGAAAGAAAAAACCTTTTAGTATTAGATCGATAGGTTATAATCCATGCCCATGAAAATG
CAATAAGAGTTATACATAAGATACAAGCTGACAGGATTTCTTTATTTTTAATTAAATAAC
TTAGAGCGATGAGGATGACAGGTGTCAAGAAAATAATAGTTACATCCCGATAGCTATAAA
AGAAAACTGCCCTATTTTGAATGTGGAGATGTGCACAGAATCCAATATAGCTAAGGATAA
TAGTTAATATAATAAAAAAAGACCACCAAGGGTGAAGAGATAGGAATTCCATGTTTTCCC
TTTTTTTGTAAAAAGGAAAAAATCTTATCTAATAGTTAATTGCCTAACCAGCCAGAAGAA
GTTTAAAATCTATCCCAATAATTCAACCATCTATACAGAAAGTTCAGCTTATGGAAACCC
ACGAAAAAATCCGCCTGATGCGCGAATTGAATAAATGGTCCCAGGAGGATATGGCGGAAA
AGCTGGCGATGTCGGCAGGCGGGTATGCCAAAATCGAACGGGGCGAAACGCAGTTAAATA
TCCCGCGTTTGGAGCAGTTGGCTCAGATTTTCAAAATCGATATGTGGGACTTGCTCAAAT
CGGGCGGTGGTGGGATGGTGTTTCAGATTAATGAAGGTGATAGTGGTGGCGATATTGCGT
TGTATGCGTCGGGTGATGTTTCGATGAAAATAGAATTTTTAAAAATGGAGTTGAAACACT
GCAAAGAAATGTTGGAACAAAAAGACAAAGAAATCGAGCTGCTCCGCAAGCTGACCGAAA
CCGTTTAAACAGATATGCCGTCTGAAAAAAGTTTTCAGACGGCATATTCTTTGACAGGTC
TTGTATAATACCGTTTGAACTTACAGGTTTTTGATTATGGCGGCAGGCAAACATACCAAA
CACAGCAACCGGGTACGCATTATCGGCGGGCAATGCCGGGGCAGGAAATTGAGTTTCACA
TCCGCCgACGGACTGCGTCCGACACCCGACAGCGTGCGTGAAAAGCTGTTTAACTGGCTG
GGACAGGATTTGACGGGTAAAACGGTTTTGGATCTCTTCGGAGGCAGCGGCGCACTCGGT
ATAGAAGCCGCTTCGCGCAACGCCAAACGCGTGCTGATTTCGGATAACAACCGCCAAACC
GTGCAGACCTTGCAGAAAAACAGTCGCGAACTGGGTTTGGGGCAGGTGCAAATCGTCTTT
TCAGACGGCATCGCATATTTGAAGACCGTATCCGAACAGTTTGATGTTGTCTTTCTCGAC
CCGCCGTTTGCATGGCAGGACTGGCAAATCCTGTTCGATGCCTTGAAGCCGTGCCTGAAC
CCCCGGGCATTCGTCTATCTCGAGGCGGGTACGCTGCCGAATATTCCCGATTGGCTGACG
GAATATAGAGAAGGGAAATCGGGGCAGAGTACATTTGAATTAAGGGTTTTCCAAGTGGCT
GAATAATATGCGCTTTGATAATCATTTCCGAGTTGTAAACATTCGTTTGCAACCGTCCGG
TTCAAAAAAACCTTGTGCTATAATCCGCGCCCGCCCGGTTTTGATAATTTAGTGGAAAAG
GAAAAGAAATGTCGCTTTTTATTACCGACGAGTGCATCAACTGCGACGTATGCGAACCCG
AATGCCCCAATGATGCCATTTCCCAAGGCGAGGAAATTTACGAAATCAACCCCAACCTCT
GCACGCAGTGCGTCGGACACTACGATGAGCCGCAGTGCCAGCAGGTTTGCCCGGTGGACT
GCATCCTGATTGACGAAGAACATCCCGAAACCCATGACGAGTTGATGGCGAAATACGAAA
AGATTATCCAGTTTAAATAAATTCTTTTTAAAACATCAAATTATGTCTGTTTTGAAATAA
AATCAAAAAAAAACTTGACGGAAAAGCAAGCCGCTAATAAACTAACGTTCTCTTTTGGAG
GGATTCCCGAGCGGTCAAAGGGGGCAGACTGTAAATCTGTTGCGAAAGCTTCGAAGGTTC
GAATCCTTCTCCCTCCACCAAAATTCTTACTTGGGGCAGTAGCGAGTAATGCGGGTGTAG
CTCAATGGTAGAGCAGAAGCCTTCCAAGCTTACGGTGAGGGTTCGATTCCCTTCACCCGC
TCCAAACAATTAGGCCCATGTAGCTCAGGGGTAGAGCACTCCCTTGGTAAGGGAGAGGTC
GGCAGTTCAAATCTGCCCATGGGCACCATCTCTCGATTATTCATTTCTTTAAGGCTTAGA
TATATAGGATATTGCCATGGCTAAGGAAAAATTCGAACGTAGCAAACCGCACGTAAACGT
TGGCACCATCGGTCACGTTGACCATGGTAAAACCACCCTGACTGCCGCTTTGACTACTAT
TTTGGCTAAAAAATTCGGCGGTGCTGCAAAAGCTTACGACCAAATCGACAACGCACCCGA
AGAAAAAGCACGCGGTATTACCATTAACACCTCGCACGTGGAATACGAAACCGAAACCCG
CCACTACGCACACGTAGACTGCCCGGGGCACGCCGACTACGTTAAAAACATGATTACCGG
CGCCGCACAAATGGACGGTGCAATCCTGGTATGTTCCGCAGCCGACGGCCCTATGCCGCA
AACCCGCGAACACATCCTGCTGGCCCGCCAAGTAGGCGTACCTTACATCATCGTGTTCAT
GAACAAATGCGACATGGTCGACGATGCCGAGCTGTTGGAACTGGTTGAAATGGAAATCCG
CGACCTGCTGTCCAGCTACGACTTCCCCGGCGATGACTGCCCGATTGTACAAGGTTCCGC
ACTGAAAGCCTTGGAAGGCGATGCCGCTTACGAAGAAAAAATCTTCGAACTGGCTGCCGC
~~ATTGGACAGCTACATCCCGACTCCCGAGCGAGCCGTGGACAAACCGTTCCTGCTGCCTAT~~
CGAAGACGTGTTCTCCATTTCCGGCCGCGGTACAGTAGTAACCGGCCGTGTAGAGCGCGG
TATCATCCACGTTGGTGACGAGATTGAAATCGTCGGTCTGAAAGAAACCCAAAAAACCAC
TTGTACCGGTGTTGAAATGTTCCGCAAACTGCTGGACGAAGGTCAGGCGGGCGACAACGT
AGGCGTATTGCTGCGCGGTACCAAACGTGAAGACGTGGAACGCGGTCAGGTATTGGCTAA
ACCGGGTACTATCACTCCTCACACCAAATTCAAAGCAGAAGTATACGTACTGAGCAAAGA
AGAGGGTGGTCGTCACACTCCGTTCTTCGCCAACTACCGTCCGCAATTCTACTTCCGTAC
CACCGACGTAACCGGCGCGGTTACTTTGGAAGAAGGTGTAGAAATGGTAATGCCGGGTGA
AAACGTAACCATCACCGTAGAACTGATTGCGCCTATCGCTATGGAAGAAGGCCTGCGCTT
TGCGATTCGCGAAGGCGGCCGTACCGTGGGTGCCGGCGTGGTTTCTTCTGTTATCGCTTA
AGTTTAGAGGCCAATAGCTCAATTGGTAGAGTATCGGTCTCCAAAACCGAGGGTTGGGGG
TTCGAGACCCTCTTGGCCTGCCAAATAAAAAATTAACCGGCCTTGTGTCGGTTAATTTTT
TTGTATTTGTTATTTAGTAAACTCTCTTGCCATTTACATGGATTGAGAATAGACAGATGC
TATGATGGATAAATAATATGACAGAACATACGCCTGAAAAAAAGAACGTTAAAGTGGATC
AACTGGTTGTTCAAGATAAAGAATCTGCATCTAATTCCGGTAAGGAAGGGTTTTTTGCAT
ATTTCTCAAATTCTTGGTCCGAATTCAAAAAGGTGGTTTGGCCTAAGCGTGAAGATGCTG
TCAGAATGACTGTATTTGTTATAGTGTTTGTTGCTGTGCTTTCTATATTTATCTATGCGG
CAGATACAGCAATTTCGTGGTTATTTTTTGATGTATTGCTGAGAAGGGAAGGTTGAGATG
TCGAAAAAATGGTATGTTGTACAGGCGTATTCGGGGTTTGAGAAGAATGTCCAACGAATA
TTGGAAGAGCGCATTGCCCGTGAGGAGATGGGAGATTATTTCGGACAAATTCTGGTGCCT
GTAGAGAAAGTTGTTGATATCCGCAATGGTCGTAAGACTATTAGTGAAAGAAAGTCATAT
CCTGGTTATGTGCTAGTTGAGATGGAAATGACAGATGACTCTTGGCATCTTGTAAAAAGC
ACCCCCCGTGTTTCCGGTTTTATTGGAGGGAGGGCTAATAGACCTACGCCGATTAGTCAG
AGAGAGGCTGAAATTATTTTACAGCAGGTTCAGACCGGCATAGAGAAGCCGAAACCAAAA
GTTGAATTTGAGGTCGGTCAACAGGTTCGTGTAAATGAAGGGCCGTTTGCGGATTTTAAC
GGGGTGGTTGAGGAGGTCAATTATGAACGGAATAAGTTACGCGTGTCTGTTCAGATATTT

## Appendix A

GGTAGAGAAACACCCGTTGAGCTGGAGTTCAGCCAGGTTGAAAAGATTAACTGATTTTTA
TACTTGAAAAAAAAGCAATAAGAGGATAGAATCAAAAATTAACTTGGGGAGCGGAAATGG
TTCCGCGTCTTACCCGTTTTTAGGAGTTCGTTAAGTGGCAAAGAAAATTATCGGCTATAT
TAAACTGCAAATTCCTGCAGGTAAAGCCAATCCATCTCCTCCGGTTGGTCCTGCTTTGGG
TCAGCGCGGTTTGAATATTATGGAATTTTGTAAGGCATTTAATGCTGCAACCCAAGGTAT
GGAGCCTGGCTTACCGATTCCGGTTGTGATTACTGCATTTGCAGATAAATCATTCACATT
TGTGATGAAAACCCCGCCAGCTTCTATCTTGTTGAAAAAGGCTGCCGGTTTGCAAAAAGG
TAGTTCTAATCCTCTGACCAACAAAGTGGGTAAATTGACCCGTGCCCAGTTGGAAGAAAT
TGCTAAAACTAAAGATCCTGATTTGACTGCTGCTGACTTGGATGCGGCTGTCCGTACTAT
AGCAGGTTCTGCTCGCTCAATGGGCTTGGATGTGGAGGGTGTTGTATAATGGCTAAAGTA
TCTAAACGCTTGAAAGCTCTTCGCTCTTCTGTGGAAGCCAATAAATTATATGCAATTGAT
GAAGCAATTGCTTTGGTAAAAAAAGCAGCGACTGCTAAATTTGACGAGTCTGTTGACGTA
TCTTTCAACTTGGGCGTTGATCCGCGTAAATCTGACCAAGTTATCCGTGGTTCGGTCGTT
CTGCCTAAAGGCACCGGTAAGATAACCCGTGTGGCTGTATTTACTCAAGGTGCAAATGCA
GAAGCTGCTAAAGAAGCTGGTGCAGATATCGTCGGTTTCGAAGATTTGGCTGCTGAAATC
AAAGCAGGCAATCTGAACTTTGATGTCGTTATTGCTTCTCCCGATGCAATGCGTATTGTT
GGTCAGTTGGGTACTATTTTGGGTCCTCGAGGCTTGATGCCAAACCCTAAAGTAGGTACG
GTTACTCCTAACGTTGCTGAAGCAGTTAAGAATGCAAAAGCAGGTCAAGTACAATACCGT
ACAGATAAAGCAGGTATCGTTCATGCAACGATTGGTCGTGCTTCTTTCGCTGAAGCTGAT
TTGAAAGAGAACTTTGATGCGTTGCTGGATGCTATCGTTAAAGCCAAGCCTGCTGCCGCT
AAAGGTCAGTATCTGAAAAAAGTTGCTGTGTCTAGCACCATGGGTTTGGGTATTCGCGTT
GATACATCAAGCGTAAATAACTAATCTTAAGGAATTTTCAAGCAGTTTGGTTTTCTGGGC
TGCTTGAATTTGGGCTACTTAAAATTAAGTAGATGTCCAAGACCGTAGGGATCGTAAGAT
TTAATCGTAACTGCCCTACGCAGACGGTAGTCCTGAAACACATTGCAAGATTGCTTGTAA
GATGTCTTTTTAGGTTACCGCGCTGGTGGGATATCGTTTTGGTATCCTGTTTATAAACAG
TGGGAGGTAGACCTTGAGTCTCAATATTGAAACCAAGAAAGTGGCGGTCGAGGAAATTAG
CGCGGCAATTGCTAATGCTCAAACCCTCGTAGTCGCTGAATATCGCGGTATCAGTGTTTC
CAGTATGACTGAGCTTCGTGCGAATGCACGTAAAGAAGGCGTTTATTTGCGCGTTCTGAA
AAATACTTTGGCTCGTCGTGCAGTGCAAGGTACTTCATTTGCAGAATTGGCCGATCAAAT
GGTTGGTCCGTTGGTTTACGCTGCTTCTGAAGATGCTGTTGCTGCTGCTAAAGTGTTGCA
CCAATTCGCGAAAAAAGATGACAAAATTGTCGTTAAAGCCGGTTCTTACAATGGCGAAGT
AATGAATGCTGCTCAGGTTGCTGAGTTGGCTTCTATTCCGAGCCGCGAAGAGCTGTTGTC
CAAACTGTTGTTCGTTATGCAAGCTCCTGTATCGGGCTTTGCGCGCGGTTTGGCTGCTTT
GGCAGAGAAAAAAGCCGGCGAAGAAGCCGCTTAATCGATTTTGTTTCTGTTAATCAATTA
TTTTTTAATACAATATTTGGAGTAAAATAGCATGGCTATTACTAAAGAAGACATTTTGGA
AGCAGTTGGTTCTTTGACCGTAATGGAATTGAACGACTTGGTTAAAGCTTTTGAAGAAAA
ATTCGGTGTTTCTGCTGCTGCTGTTGCAGTTGCAGGTCCTGCTGGTGCCGGTGCTGCCGA
TGCTGAAGAAAAAACCGAATTTGATGTCGTTTTGGCTTCTGCCGGCGATCAAAAAGTCGG
CGTGATTAAAGTTGTCCGTGCAATTACCGGTTTGGGTCTGAAAGAAGCTAAAGACATCGT
TGACGGCGCACCTAAAACCATTAAAGAGGGTGTTTCTAAAGCTGAAGCCGAAGACATCCA
AAAACAACTGGAAGAAGCAGGCGCTAAAGTCGAAATCAAATAATTTGATGCTTCTTATGA
AGGCTGGCAGTTTTCTGCCAGCCTTATTTTGCTTCTTAAAATAAACATCAAGTATTGTTT
ACATTTATTTGCATAGTTTTTATCAAGTCATTGCAAATAAATGTAAATATCAGATTGATG
CGTACCGTTGTTTCAGACGGCCTATTATTGAAAATTACTTTTCGGAGTGTGTATGAACTA
TTCGTTTACCGAGAAAAAACGTATCCGTAAGAGTTTTGCAAAGCGGGAAAATGTTTTGGA
AGTTCCTTTCTTGCTAGCAACCCAAATTGATTCTTATGCGAAGTTTTTGCAGCTGGAAAA
TGCTTTTGACAAACGTACCGATGACGGTCTGCAGGCGGCATTTAATTCTATTTTCCCGAT
TGTGAGCCATAACGGTTATGCGCGATTGGAGTTTGTGCATTACACATTGGGCGAGCCTTT
GTTCGATATTCCCGAATGTCAGTTGCGCGGAATCACTTATGCAGCCCCCTTGCGCGCGCG
TATCCGTTTGGTGATTTTGGATAAGGAAGCATCTAAACCGACGGTAAAAGAAGTTCGTGA
AAACGAAGTGTATATGGGCGAAATTCCGTTGATGACCCCGAGCGGTTCTTTTGTGATTAA
CGGCACAGAGCGTGTGATTGTCTCCCAGTTGCACCGTTCGCCCGGCGTATTCTTCGAGCA
TGACAAAGGTAAGACGCACTCTTCCGGCAAATTGTTATTCTCCGCCCGCATCATTCCCTA
CCGTGGTTCATGGTTGGATTTTGAATTTGATCCGAAAGATTTGCTGTATTTCCGTATCGA
CCGCCGCCGTAAAATGCCGGTAACGATTTTGTTGAAGGCTTTAGGCTACAACAATGAGCA
AATCTTGGATATTTTCTACGACAAAGAAACGTTCTATTTGTCTTCAAACGGTGTTCAAAC
CGATTTGGTTGCAGACCGTCTGAAAGGCGAAACTGCCAAGGTCGATATCTTGGATAAAGA
AGGCAATGTATTGGTTGCCAAAGGTAAGCGCATTACTGCGAAAAATATCCGTGATATTAC
CAATGCAGGCCTGACCCGTTTGGATGTAGAACCGGAAAGCCTGCTGGGCAAAGCATTGGC
TGCCGATCTGATTGATTCGGAAACCGGCGAGGTATTGGCTTCTGCCAATGATGAAATTAC
AGAAGAGTTGTTGGCCAAATTTGATATCAACGGCGTAAAAGAAATTACGACCCTTTATAT
CAATGAGCTGGATCAGGGTGCTTATATCTCCAATACCTTGCGTACGGATGAGACTGCCGG
CCGGCAGGCGGCTCGTGTTGCGATTTACCGTATGATGCGTCCGGGCGAACCGCCCACCGA
AGAGGCGGTCGAGCAATTGTTTAACCGCTTGTTCTTCAGTGAAGACAGCTACGATCTGTC
CCGCGTAGGCCGTATGAAATTTAATACGCGCACATACGAACAAAAACTGTCCGAAGCCCA
ACAAAACTCTTGGTACGGCCGCCTGCTGAACGAAACGTTTGCCGGTGCTGCCGACAAAGG
CGGTTATGTCCTGAGCGTCGAAGATATTGTCGCCTCGATTGCGACTTTGGTCGAGTTGCG
TAACGGCCATGGCGAAGTGGACGATATCGATCACTTGGGCAACCGCCGAGTACGTTCGGT
AGGCGAGCTGACTGAAAACCAATTCCGTAGCGGTTTGGCCCGTGTGGAACGTGCCGTAAA
AGAACGTTTGAATCAGGCGGAATCAGAAAACTTGATGCCGCACGATTTGATTAATGCAAA
ACCTGTTTCTGCCGCTATTAAAGAATTCTTCGGCTCCAGCCAATTGAGTCAGTTTATGGA
TCAGACCAACCCCTTGTCTGAAGTAACCCATAAACGCCGTGTATCTGCATTGGGTCCGGG
CGGTTTGACCCGCGAACGTGCAGGATTTGAGGTGCGGGACGTGCATCCGACCCACTACGG
TCGCGTATGTCCGATTGAAACGCCTGAAGGTCCGAACATCGGTTTGATCAACTCATTGTC
CGTTTATGCGCGCACCAATGATTACGGTTTCTTGGAAACGCCTTACCGCCGCGTTATCGA

## Appendix A

CGGCAAAGTAACCGAGGAAATCGATTACTTGTCTGCCATCGAAGAAGGCCGCTATGTGAT
TGCACAGGCGAATGCCGATTTGGATTCAGATGGCAATCTGATTGGCGATTTGGTTACCTG
TCGTGAAAAAGGCGAAACCATTATGGCAACGCCCGACCGCGTCCAATATATGGACGTGGC
AACTGGTCAAGTGGTATCCGTTGCAGCATCCCTGATTCCATTCTTGGAACATGATGACGC
GAACCGCGCATTGATGGGTGCCAACATGCAACGTCAGGCAGTGCCTTGCTTGCGTCCTGA
AAAACCGATGGTCGGTACCGGTATCGAGCGTTCCGTTGCCGTTGACTCTGCTACTGCAAT
CGTTGCCCGCCGAGGCGGCGTGGTCGAGTATGTCGATGCCAACCGCGTTGTGATCCGTGT
CCATGACGACGAAGCGACTGCCGGTGAAGTGGGTGTCGATATTTACAACTTGGTTAAATT
CACCCGTTCCAACCAGTCTACCAATATCAATCAGCGTCCTGCCGTCAAAGCCGGCGATGT
TTTGCAACGCGGCGATTTGGTGGCCGACGGCGCGTCCACCGATTTTGGCGAATTGGCTTT
GGGTCAAAATATGACCATCGCCTTCATGCCGTGGAACGGTTACAACTACGAAGACTCGAT
TCTGATTTCCGAAAAAGTGGCTGCGGACGACCGCTATACTTCGATTCACATTGAGGAATT
GAATGTCGTTGCCCGCGATACTAAGCTGGGTGCGGAAGACATTACCCGCGATATTCCGAA
CTTGTCCGAGCGTATGCAAAACCGTTTGGACGAATCCGGTATCGTTTACATCGGTGCGGA
AGTAGAAGCCGGCGATGTGTTGGTAGGCAAGGTAACGCCTAAAGGCGAAACCCAACTGAC
GCCGGAAGAAAAACTGCTGCGCGCCATCTTCGGTGAAAAAGCATCTGACGTAAAAGATAC
TTCATTGCGTATGCCTACCGGCATGAGCGGTACCGTTATCGACGTTCAAGTCTTCACTCG
TGAAGGTATTCAACGCGACAAACGTGCTCAATCCATTATCGATTCCGAATTGAAACGCTA
CCGTTTGGATTTGAACGACCAATTGCGTATTTTCGACAACGACGCATTCGACCGTATCGA
GCGTATGATTGTCGGTCAGAAAGCCAACGGTGGTCCGATGAAGCTGGCCAAAGGCAGCGA
AATCACGACCGAATATCTGGCGGGTCTGCCGAGCAGGCACGATTGGTTCGATATCCGTCT
GACCGATGAAGATTTGGCCAAGCAGTTGGAACTGATTAAAGTGAGCCTGCAACAAAAACG
CGAAGAAGCGGACGAGTTATACGAAATCAAGAAGAAAAAACTGACCCAAGGCGACGAATT
GCAACCCGGCGTACAAAAAATGGTGAAAGTTTTTATCGCCATCAAACGCCGTCTGCAAGC
CGGCGACAAAATGGCGGGCCGCCACGGTAACAAAGGCGTGGTATCGCGCATTCTGCCAGT
GGAAGACATGCCTTACATGGCGGACGGCCGTCCGGTAGACATCGTACTGAACCCATTGGG
CGTACCTTCCCGTATGAACATCGGTCAGATTTTGGAAGTTCACTTGGGTTGGGCAGCAAA
AGGTATCGGCGAGCGTATCGACCGTATGCTGAAAGAGCAACGCAAAGCAGGCGAGTTGCG
CGAGTTCTTGAACAGACTCTACAACGGCAGCGGTAAGAAAGAAGATTTGGATGCCCTGAC
TGATGAAGAAATCATCGAACTGGCCTCCAACCTGCGCAAAGGTGCATCTTTCGCCTCTCC
TGTATTCGACGGTGCGAAAGAGTCTGAAATCCGCGAAATGCTGAACTTGGCTTATCCGAG
CGACGATCCTGAGGTTGAAAAACTGGGCTTCAACGACAGTAAAACCCAAATCACGCTGTA
TGACGGCCGTTCAGGCGAAGCATTTGACCGCAAGGTTACAGTAGGTGTGATGCACTATCT
GAAACTGCACCACTTGGTTGACGAAAAAATGCACGCGCGTTCTACCGGTCCGTACAGTCT
GGTTACCCAGCAGCCTTTGGGCGGTAAAGCCCAGTTCGGCGGCCAACGTTTCGGCGAGAT
GGAGGTTTGGGCATTGGAAGCATACGGCGCGGCATACACGCTGCAAGAGATGCTGACTGT
GAAGTCTGACGACGTGAACGGCCGTACCAAAATGTACGAAAACATCGTCAAAGGCGAACA
CAAAATCGATGCCGGTATGCCCGAGTCCTTCAACGTATTGGTCAAAGAGATTCGCTCACT
GGGCTTGGATATCGATTTGGAACGTTACTAAACAAAAGTTTTCAGACGGCCTTTCAGGGT
CGTCTGAAAAAGTGGTTTCAGAATAAGAATGAAGCAATCGGCATTTAGGCCGTCTGAAAT
CAAAAGTACCGTTTCCCAATATCGAAAATCCGCCATGCGGTAAAAATACTTCCTTCAAGG
AGCAAAAATGAATTTGTTGAACTTATTTAATCCGTTGCAAACTGCCGGCATGGAAGAAGA
GTTTGATGCCATTAAAATCGGTATTGCCTCTCCCGAAACCATCCGCTCATGGTCTTATGG
CGAAGTCAAAAAACCTGAAACCATCAACTACCGTACGTTCAAACCTGAGCGTGACGGTTT
GTTCTGTGCCAAAATCTTTGGCCCGGTCAAAGACTACGAATGCTTGTGCGGAAAATACAA
ACGCTTGAAATTTAAAGGCGTAACGTGTGAAAAATGCGGCGTGGAAGTAACCCTGTCCAA
AGTGCGCCGCGAACGCATGGGTCATATCGAATTGGCTGCGCCCGTCGCACATATTTGGTT
CTTAAAATCCCTGCCTTCCCGCTTGGGTATGGTGTTAGACATGACTTTGCGCGACATCGA
GCGCGTATTGTACTTTGAAGCATTTGTGGTAACCGATCCCGGTATGACTCCGCTGCAACG
CCGCCAATTGCTGACTGAAGACGATTACTACAACAAGCTGGACGAATACGGCGACGATTT
CGATGCCAAAATGGGTGCGGAAGGTATCCGCGAATTGCTGCGTACCCTGAATGTAGCGGG
CGAAATCGAAATCCTGCGCCAAGAGTTGGAATCGACCGGTTCCGACACCAAAATCAAAAA
AATCGCCAAACGCTTGAAAGTATTGGAAGCCTTCCATCGTTCCGGTATGAAACTGGAATG
GATGATTATGGATGTGCTGCCGGTATTGCCGCCTGATTTGCGTCCGTTGGTTCCATTGGA
TGGTGGTCGTTTTGCCACTTCCGATTTGAACGATTTGTACCGCCGCGTTATTAACCGTAA
CAACCGTCTGAAACGTCTGTTGGAACTGCATGCGCCTGACATCATCGTCCGCAACGAAAA
ACGTATGTTGCAAGAAGCAGTTGACTCGCTGTTGGATAACGGCCGTCGCGGTAAAGCCAT
GACCGGCGCCAACAAACGCCCGCTGAAATCATTGGCAGACATGATTAAAGGTAAAGGCGG
TCGCTTCCGTCAAAACCTGTTGGGCAAACGTGTGGACTACTCCGGCCGTTCCGTGATTAC
CGTAGGCCCGTACCTGCGTCTGCACCAATGCGGTTTGCCGAAAAAAATGGCTTTGGAACT
GTTCAAACCGTTCATTTTCCACAAATTGGAAAAACAAGGTTTGGCCTCTACCGTTAAAGC
AGCGAAAAAATTGGTAGAGCAAGAAGTACCGGAAGTATGGGACATCTTGGAAGAAGTCAT
CCGCGAACATCCGATTATGCTGAACCGTGCGCCGACCCTGCACCGTTTGGGTATTCAAGC
GTTCGAACCTATCTTGATTGAAGGTAAAGCGATTCAGTTGCACCCATTGGTGTGTGCTGC
GTTCAACGCCGACTTTGACGGCGACCAAATGGCGGTACACGTTCCATTGAGCTTGGAAGC
ACAAATGGAAGCACGCACGCTGATGCTGGCTTCAAACAACGTATTGTCTCCGGCCAACGG
CGAACCGATTATCGTACCTTCCCAAGACATCGTATTGGGCCTGTACTATATGACTCGCGA
TCGTATCAATGCCAAAGGCGAAGGCAGCCTGTTTGCCGATGTGAAAGAAGTGCATCGCGC
ATACCATACCAAACAGGTCGAGCTGGGTACGAAAATCACCGTACGTCTGCGCGAATGGGT
GAAAAACGAAGCAGGTGAGTTTGAGCCTGTCGTTAACCGTTACGAAACAACCGTCGGCCG
TGCATTGTTGAGCGAAATCCTGCCGAAAGGCCTGCCGTTTGAATATGTCAACAAAGCGTT
GAAGAAAAAAGAAATTTCTAAACTGATTAACGCATCGTTCCGCCTGTGCGGCTTGCGCGA
TACGGTTATCTTTGCTGACCACCTGATGTACACCGGTTTCGGATTTGCGGCAAAAGGCGG
TATTTCCATTGCCGTTGACGATATGGAAATTCCAAAAGAAAAAGCGGCCTTGCTGGCTGA
AGCCAATGCCGAGGTTAAAGAAATCGAAGACCAATACCGTCAAGGTTTGGTTACCAACGG

# Appendix A

CGAACGCTACAACAAGGTGGTCGATATTTGGGGTCGTGCCGGCGATAAGATTGCTAAAGC
GATGATGGACAACTTGTCCAAACAAAAAGTTATCGACCGTGCCGGCAACGAAGTCGATCA
AGAGTCATTCAACTCCATTTATATGATGGCGGACTCCGGTGCCCGTGGTTCTGCAGCTCA
GATTAAACAGTTGTCCGGTATGCGTGGCTTGATGGCAAAACCTGACGGCTCGATTATTGA
AACGCCGATTACCTCAAACTTCCGTGAAGGTCTGACCGTATTGCAATACTTTATTGCGAC
CCACGGTGCGCGTAAGGGTTTGGCGGATACCGCATTGAAAACCGCGAACTCCGGTTACCT
GACTCGTCGTCTGGTAGACGTAACTCAAGATTTGGTCGTTGTTGAAGACGATTGCGGTAC
TTCAGACGGCTTTGTCATGAAGGCAGTGGTACAAGGCGGTGATGTGATTGAAGCATTGCG
CGATCGTATTTTGGGTCGTGTTACCGCGTCTGACGTTGTCGATCCGTCAAGTGGCGAAAC
CTTGGTTGAAGCCGGTACGTTGCTGACTGAAAAACTGGTGGATATGATCGACCAATCCGG
TGTCGATGAAGTCAAAGTCCGTACGCCGATTACTTGTAAAACCCGTCACGGCCTGTGTGC
ACACTGTTACGGTCGTGACTTGGCACGCGGCAAACTGGTTAACGCCGGTGAGGCAGTCGG
TGTGATTGCTGCACAATCCATTGGCGAACCGGGTACCCAGTTGACCATGCGTACGTTCCA
CATCGGTGGTGCGGCATCCCGTGCGGCAGCAGCCAGCCAAGTGGAAGCCAAATCCAACGG
TACGGCACGATTCAGCAGCCAGATGCGCTACGTTGCCAACAACAAAGGCGAGTTGGTTGT
CATCGGCCGTTCTTGTGAAGTCGTGATTCACGACGATATCGGCCGTGAACGCGAACGCCA
CAAAGTACCTTACGGTGCCATCCTGCTGGTACAAGACGGTATGGCCATTAAAGCCGGTCA
AACCTTGGCAACCTGGGATCCGCATACCCGTCCGATGATTACCGAACACGCAGGTATGGT
GAAATTCGAAAACGTGGAAGAGGGCGTTACCGTTGCCAAACAAACCGATGATGTAACCGG
TTTGTCCACTTTGGTGGTGATTGACGGTAAACGTCGTTCCTCTAGTGCTTCCAAACTGCT
GCGTCCGACTGTGAAACTCTTGGACGAAAACGGCGTGGAAATCTGTATTCCCGGTACTTC
TACTCCGGTATCCATGGCATTCCCCGTTGGTGCGGTGATTACCGTACGCGAAGGTCAGGA
AATCGGTAAAGGCGACGTATTGGCGCGTATTCCGCAAGCCTCTTCCAAAACCCGCGACAT
TACCGGCGGCCTGCCGCGCGTTGCCGAATTGTTTGAAGCACGCGTGCCGAAAGATGCCGG
TATGTTGGCGGAAATTACCGGTACCGTTTCCTTCGGCAAAGAGACCAAAGGCAAGCAACG
TCTGATTGTTACTGACGTGGACGGTGTAGCATACGAGACCTTGATTTCCAAAGAGAAACA
AATTCTGGTACACGACGGTCAAGTGGTAAACCGCGGTGAAACCATCGTGGACGGCGCGGT
CGATCCGCACGATATTCTGCGTTTGCAAGGTATCGAAGCACTGGCACGCTACATTGTCCA
AGAGGTGCAAGAGGTTTACCGTCTGCAAGGTGTGAAGATTTCTGATAAACACATCGAAGT
CATCATCCGTCAAATGTTGCGCCGTGTGAACATTGCGGATGCCGGCGAAACCGGGTTCAT
TACCGGAGAGCAGGTCGAACGCGGCGATGTGATGGCGGCCAATGAAAAAGCTTTGGAAGA
AGGCAAAGAACCGGCGCGTTACGAAAACGTATTGCTGGGTATTACCAAAGCTTCCCTGTC
CACCGACAGCTTCATTTCTGCCGCATCGTTCCAAGAAACGACCCGCGTTCTGACCGAAGC
CGCGATTATGGGCAAACAAGACGAGTTGCGTGGTTTGAAAGAAAACGTCATCGTCGGTCG
CTTGATTCCTGCCGGTACCGGTTTGACTTACCACCGCAGCCGTCATCAACAATGGCAAGA
GGTGGAACAGGAGACTGCCGAAACCCAAGTAACGGATGAATAATCTTTGGTGCATCCATT
CAATAAAAAACCGCAAGCCTTGAGCTTGCGGTTTTTCTTTGTCCGATTAAGGCAAAAACA
AGCGTTTTCGTCATTTTGAGGCGTGTGGATTATTCCTTAGGTATTTTCGGGCCGGAGACC
AACGAGGTGGCGGGTGTCGTCGGTACGTCCGGAGACCAAAATAACTTTGCCAGGGATGTT
GGTTTCGGCGGTCAAAAAAAGTAGCGTCTTAATGTTTTCCATTTAAACAAATGTCGTCTG
AAACTTCAGACGGCATTTCCTTTAAGAAATAAATATGAAACCCAGAAATCTCTTTTTTGC
AGGCTGCCTGCTGACTTCGGCGACGTTTGCCGAGGATATCGGCGTACCTGTCGAACTGAT
TAACGTCGGTAATCGGATTGCGATGCCGTCTGAAGGGGAAAGCCTCGCCCTCCTGCCGTT
TGCCGAGGATGTACCGCCGGTTCGCGATGCAATGCCGTCTGAAGTTCCTAAAAGCGCGGC
AGGCGGCGATGTTCGGGGTGACCGGATGAGAATGCCGATTAACATCGGATGAGCGCGGCT
TTATGGCATAAAAAACTGTCGTGGAAAGGATTTACACCCCAAATAAATTTCCGTTACAAC
AAGATCAACAGCAATATGCCCGCCTTTTATTCGCGCAGCGGCAAGGAACGGTTTGTCAGT
ATAGAAAAAACGTATTGACAGTATTTTCTTCAGTCGTCCGACTGATTGTGAGGGATGTCG
GTAAATATTTATCGGCAAACAAGAAAATCATCTTTCTTCTTGTCGTTATGCTTGACTGTC
TGCTTGCAATAAAAATATAATTCCACTCTTGCCGACATGGTGTCGGCAAGTATTTAACTC
AACAGGACGAGAAAATATGCCAACTATCAACCAATTAGTACGCAAAGGCCGTCAAAAGCC
CGTGTACGTAAACAAAGTGCCCGCACTGGAAGCTTGCCCGCAAAAACGTGGCGTGTGCAC
CCGTGTATACACAACTACCCCTAAAAAACCTAACTCTGCATTGCGTAAAGTATGTAAAGT
CCGCCTGACCAACGGTTTTGAAGTCATTTCATACATCGGCGGCGAAGGTCACAACCTGCA
AGAGCACAGTGTCGTATTGATTCGCGGCGGTCGTGTAAAAGACTTGCCAGGTGTGCGTTA
CCACACTGTACGCGGTTCTTTGGATACTGCAGGTGTTAAAGACCGTAAACAAGCCCGTTC
CAAATACGGTGCTAAGCGTCCTAAATAATTACTGGGACTTAAATAGGCACGTCGGCCGCC
TAAGCTGAACAACGGCCGAGTAAGTGAATACTCAATTGGGTATTCATGGGAATAGACCCG
ACTGAATAGATTAAAGGAAATTAAAATGCCAAGACGTAGAGAAGTCCCCAAGCGCGACGT
ACTGCCAGATCCTAAATTCGGCAGCGTCGAGTTGACCAAATTCATGAACGTATTGATGAT
TGACGGTAAAAAATCCGTTGCCGAGCGTATCGTTTACGGTGCGTTGGAACAGATTGAGAA
AAAAACCGGCAAAGTAGCAATCGAAGTATTTAACGAAGCCATTGCAAACGCCAAACCTAT
CGTGGAAGTGAAAAGCCGCCGTGTAGGTGGTGCAAACTACCAAGTTCCTGTTGAAGTTCG
TCCTTCACGCCGTTTGGCTTTGGCAATGCGCTGGGTTCGCGATGCGGCCCGCAAACGTGG
TGAGAAATCCATGGACCTGCGTTTGGCAGGCGAATTGATTGATGCGTCCGAAGGCCGTGG
CGGTGCGTTGAAAAAACGTGAAGAAGTACACCGTATGGCTGAAGCCAACAAAGCATTCTC
TCACTTCCGTTTCTAATTTTGAAAGGCTAATAAAATGGCTCGTAAGACCCCGATCAGCCT
GTACCGTAACATCGGTATTTCCGCCCATATTGACGCGGGTAAAACCACGACGACAGAACG
TATTTTGTTCTATACCGGTTTGACCCACAAGCTGGGCGAAGTGCATGACGGTGCGGCTAC
TACCGACTACATGGAACAAGAGCAAGAGCGCGGTATTACCATTACCTCCGCTGCCGTTAC
TTCCTACTGGTCCGGTATGGCGAAACAATTCCCCGAGCACCGCTTCAACATCATCGACAC
CCCGGGACACGTTGACTTTACCGTAGAGGTAGAGCGTTCTATGCGTGTATTGGACGGCGC
GGTAATGGTTTACTGCGCGGTGGGCGGTGTTCAACCCCAATCTGAAACCGTATGGCGGCA
AGCCAACAAATACCAAGTGCCGCGCTTGGCGTTTGTCAATAAAATGGACCGTCAGGGTGC
CAACTTCTTCCGTGTTGTCGAGCAAATGAAAACCCGTTTGCGCGCAAACCCTGTACCTAT

## Appendix A

CGTCATTCCGGTTGGTGCGGAAGACAACTTCAGCGGTGTGGTTGATTTGTTGAAAATGAA
ATCCATCATTTGGAATGAAGTCGATAAAGGTACAACCTTTACCTATGGCGATATTCCTGC
CGAATTGGTCGAAACTGCCGAAGAATGGCGTCAAAATATGATTGAAGCCGCAGCCGAAGC
CAGCGAAGAACTGATGGACAAATACTTAGGCGGCGACGAGCTGACCGAAGAAGAAATCGT
AGGCGCGTTGCGTCAACGTACTTTGGCAGGCGAAATTCAGCCTATGCTGTGTGGTTCTGC
ATTTAAAAACAAAGGTGTTCAACGTATGTTGGACGCAGTTGTAGAATTGCTGCCAGCTCC
TACCGATATTCCTCCGGTTCAAGGTGTCAACCCGAATACCGAGGAAGCCGACAGCCGTCA
AGCCAGCGATGAAGAGAAATTCTCTGCATTGGCGTTCAAAATGTTGAACGACAAATACGT
CGGTCAGCTGACCTTTATCCGCGTTTACTCAGGCGTAGTAAAATCCGGCGATACCGTATT
GAACTCCGTAAAAGGCACTCGCGAACGTATCGGTCGTTTGGTACAAATGACTGCCGCAGA
CCGTACTGAAATCGAAGAAGTACGCGCCGGCGACATCGCAGCCGCTATTGGTCTGAAAGA
CGTTACTACCGGTGAAACCTTGTGTGCGGAAAGCGCGCCGATTATCTTGGAACGTATGGA
ATTCCCCGAGCCGGTAATCCATATTGCCGTTGAGCCGAAAACCAAAGCCGACCAAGAGAA
AATGGGTATCGCCCTGAACCGCTTGGCTAAAGAAGACCCTTCTTTCCGTGTCCGTACAGA
CGAAGAATCCGGTCAAACCATTATTTCCGGTATGGGTGAGCTGCACTTGGAAATTATTGT
TGACCGTATGAAACGCGAATTCGGTGTGGAAGCAAATATCGGTGCGCCTCAAGTGGCTTA
CCGTGAAACTATCCGCAAAGCCGTTAAAGCCGAATACAAACATGCAAAACAATCCGGTGG
TAAAGGTCAATACGGTCACGTTGTGATTGAAATGGAACCTATGGAACCGGGTGGTGAAGG
TTACGAGTTTATCGATGAAATTAAAGGTGGTGTGATTCCTCGCGAATTTATTCCGTCTGT
CGATAAAGGTATCCGCGATACGTTGCCTAACGGTATCGTTGCCGGCTATCCTGTAGTTGA
CGTACGTATCCGTCTGGTATTCGGTTCTTACCATGATGTCGACTCTTCCCAATTGGCATT
TGAATTGGCTGCTTCTCAAGCGTTTAAAGAAGGTATGCGTCAAGCATCTCCTGCCCTGCT
TGAGCCAATCATGGCAGTTGAAGTGGAAACCCCGGAAGAATACATGGGCGACGTAATGGG
CGACTTGAACCGCCGTCGCGGTGTTGTATTGGGTATGGATGATGACGGTATCGGCGGTAA
AAAAGTCCGTGCCGAAGTACCTTTGGCAGAAATGTTCGGTTACTCGACCGACCTGCGTTC
TGCAACCCAAGGCCGCGCTACTTACTCTATGGAGTTCAAGAAATATTCTGAAGCTCCTGC
CCACATAGCTGCTGCTGTAACTGAAGCCCGTAAAGGCTAATCAGAAAAGGCCGTCTGAAA
CTGAAAATAAATTTTCAGACGGCCATTGTTCTTTAATCGATCTTTATATGTAAAGGAATT
AGCTCATGGCTAAGGAAAAATTTGAACGTAGCAAACCGCACGTAAACGTTGGCACCATCG
GTCACGTTGACCATGGTAAAACCACTCTGACTGCTGCTTTGACTACTATTTTGTCTAAAA
AATTCGGTGGCGCTGCAAAAGCTTATGACCAAATCGACAACGCTCCTGAAGAAAAAGCTC
GTGGTATTACCATTAATACCTCACACGTAGAATACGAAACTGAAACCCGTCACTACGCAC
ACGTAGACTGCCCGGGGCACGCCGACTACGTTAAAAACATGATTACCGGCGCCGCACAAA
TGGACGGTGCAATCCTGGTATGTTCCGCAGCCGACGGCCCTATGCCGCAAACCCGCGAAC
ACATCCTGCTGGCCCGCCAAGTAGGCGTACCTTACATCATCGTGTTCATGAACAAATGCG
ACATGGTCGACGATGCCGAGCTGTTGGAACTGGTTGAAATGGAAATCCGCGACCTGCTGT
CCAGCTACGACTTCCCCGGCGATGACTGCCCGATTGTACAAGGTTCCGCACTGAAAGCCT
TGGAAGGCGATGCCGCTTACGAAGAAAAAATCTTCGAACTGGCTGCCGCATTGGACAGCT
ACATCCCGACTCCCGAGCGAGCCGTGGACAAACCGTTCCTGCTGCCTATCGAAGACGTGT
TCTCCATTTCCGGCCGCGGTACAGTAGTAACCGGCCGTGTAGAGCGCGGTATCATCCACG
TTGGTGACGAGATTGAAATCGTCGGTCTGAAAGAAACCCAAAAAACCACTTGTACCGGTG
TTGAAATGTTCCGCAAACTGCTGGACGAAGGTCAGGCGGGCGACAACGTAGGCGTATTGC
TGCGCGGTACCAAACGTGAAGACGTGGAACGCGGTCAGGTATTGGCTAAACCGGGTACTA
TCACTCCTCACACCAAATTCAAAGCAGAAGTATACGTACTGAGCAAAGAAGAGGGTGGTC
GTCACACTCCGTTCTTCGCCAACTACCGTCCGCAATTCTACTTCCGTACCACCGACGTAA
CCGGCGCGGTTACTTTGGAAGAAGGTGTGGAAATGGTAATGCCGGGTGAAAACGTAACCA
TCACCGTAGAACTGATTGCGCCTATCGCTATGGAAGAAGGCCTGCGCTTTGCGATTCGCG
AAGGCGGCCGTACCGTGGGTGCCGGCGTGGTTTCTTCTGTTATCGCTTAATTGAAGGATA
TTGATAAATGGCAAACCAAAAAATCCGTATCCGCCTGAAAGCTTATGATTACGCCCTGAT
TGACCGTTCTGCACAAGAAATCGTTGAAACTGCAAAACGTACCGGTGCAGTTGTAAAAGG
CCCGATTCCTTTGCCGACCAAAATCGAGCGTTTCAACATTTTGCGTTCTCCGCACGTGAA
CAAAACTTCCCGTGAGCAATTGGAAATCCGCACCCACTTGCGCCTGATGGACATCGTGGA
TTGGACCGATAAAACTACCGATGCGCTGATGAAGCTGGATTTGCCGGCCGGTGTTGATGT
AGAAATCAAAGTCCAATAATTCGGACTATAAAAAATCCCCAAGCAATCAATGCTTGGGGA
TTTTTTATGTTATGCCGAGACCTTTGCAAAATTCCCCAAAATCCCCTAAATTCCCACCAA
GACATTTAGGAGCACCTTCTTCCAGCAAACCGCCCAAGCCATGATTGCCAAACACATCGA
CCGGTTCCCACTATTGAAGTTGGACCGGGTAATTGATTGGCAGCCGATCGAACAGTACCT
GAATCGTCAAAGAACCCGTTACCTTAGAGACCACCGCGGCCGTCCCGCCTATCCCCTGTT
GTCCATGTTCAAAGCCGTCCTGCTCGGACAATGGCACAGCCTCTCCGATCCCGAACTCGA
GCACAGCCTCATCACCCGCATCGATTTCAACCTGTTTTGCCGCTTTGACGAACTGAGCAT
CCCCGATTACAGTCATCAACCATATTCCGGTTTGTCGGAGAAAGATGCATACGCTGTGAT
GACCGGATACCGACCCGTTAAAAGAGTCCGACCCTATGCCGTCTGAAAATTCAAAACGCT
TCAGACGGCATATTGAAGATATTTCTGATATTTCTGTTGATATTTCTTTGACTTGTCAGA
TATAATGCCGAGCTTGGTACATTTGTGCCAAGTTTAACTTTGTCTGAAAGACAGGCCAAT
CGTAGCCTGTCCCTTTACTTTAAAAGGAAAATAATCATGACTTTAGGTCTGGTTGGACGC
AAAGTTGGTATGACCCGCGTGTTCGACGAACAGGGTGTTTCTGTTCCGGTAACCGTTTTG
GATATGTCTGCCAACCGCGTTACACAAGTAAAATCCAAAGATACTGACGGCTATACTGCC
GTTCAAGTTACCTTTGGTCAGAAAAAAGCCAATCGTGTCAACAAAGCCGAAGCCGGGCAC
TTTGCAAAAGCAGGTGTTGAAGCCGGTCGCGGTTTGATTGAGTTTGCTTTGACTGAAGAA
AAACTGGCTGAATTGAAAGCTGGTGACGAAATCACCGTTTCTATGTTTGAAGTCGGTCAA
CTGGTCGATGTAACCGGTACCTCTAAAGGTAAAGGTTTCTCCGGCACGATTAAACGTCAT
AACTTCGGTGCCCAACGTACTTCCCACGGTAACTCCCGTTCTCACCGTGTTCCAGGCTCT
ATCGGTATGGCGCAAGACCCGGGTCGCGTGTTCCCCGGTAAACGCATGGCCGGCCAATAC
GGCAACACCAAAGCAACTGTTCAAAAATTGGAAGTTGTCCGTGTTGACGCAGAACGCCAA
CTGCTGTTGGTTAAGGGTGCTGTTCCGGGTGCGGTCAACAGCGATGTTGTAGTTCGTCCC

## Appendix A

AGCGTGAAAGTAGGTGCGTAATGGAATTGAAAGTAATTGACGCTAAAGGACAAGTTTCAG
GCAGTCTGTCTGTTTCTGATGCTTTGTTCGCCCGCGAATACAATGAAGCGTTGGTTCATC
AGCTGGTAAATGCCTACTTGGCAAACGCCCGCTCCGGTAACCGCGCTCAAAAAACCCGTG
CCGAAGTAAAACACTCAACCAAAAAACCATGGCGTCAAAAAGGTACCGGCCGTGCCCGTT
CCGGTATGACTTCTTCTCCGCTGTGGCGTAAAGGTGGTCGCGCGTTCCCGAACAAACCCG
ACGAAAACTTCACTCAAAAAGTAAACCGCAAAATGTACCGTGCCGGTATGGCGACTATTC
TGTCCCAATTGACTCGTGACGAGCGTTTGTTTGCGATTGAGGCGTTGACTGCCGAAACTC
CTAAAACCAAAGTTTTTGCCGAACAAGTGAAAAATCTGGGTCTGGAGCAAGTGTTGTTTG
TAACCAAACAGCTCGACGAGAATGTTTACTTGGCTTCACGCAACTTGCCAAACGTGTTGG
TTTTGGAAGCTCAACAAGTTGATCCTTACAGCTTGCTGCGTTACAAAAAAGTAATCATCA
CTAAAGATGCAGTTGCACAATTAGAGGAGCAATGGGTATGAATCAACAACGTTTGACTCA
AGTGATTTTGGCACCTATCGTTTCTGAAAAAAGCAACGTATTGGCTGAAAAACGTAACCA
AATGACGTTTAAAGTTTTGGCAAATGCAACCAAACCTGAAATTAAAGCGGCTGTTGAGCT
GCTGTTCGGCGTTCAAGTTGCAGACGTTACTACTGTTACCATTAAAGGTAAAGTTAAACG
TTTTGGTCGCACTTTAGGTCGTCGCAGCGATGTTAAAAAGGCTTATGTAAGCTTGGCTGC
CGGTCAAGAGTTGGATTTGGAAGCCGCTGCTGCAGCTGCAGATAAGGAATAAACAAAATG
GCAATCGTTAAAATGAAGCCGACCTCTGCAGGCCGTCGCGGCATGGTTCGCGTGGTAACA
GAAGGTTTGTACAAAGGTGCACCTTATGCACCTCTGCTGGAAAAGAAAAATTCTACTGCC
GGTCGTAACAACAATGGTCATATTACTACCCGTCATAAAGGTGGTGGTCATAAACATCAT
TACCGCGTCGTAGATTTTAAACGTAACAAAGACGGTATCCCTGCAAAAGTAGAGCGTATC
GAATATGACCCTAACCGTACTGCATTTATCGCACTGTTGTGCTATGCAGATGGTGAGCGT
CGCTACATTATTGCTCCTCGTGGTATTCAAGCCGGTGCAGTATTGGTTTCCGGTGCTGAA
GCTGCGATCAAAGTAGGTAACACTCTGCCGATCCGCAATATTCCTGTTGGTACAACTATT
CACTGTATCGAAATGAAACCAGGTAAAGGTGCGCAAATTGCACGTTCTGCCGGTGCTTCT
GCGGTATTGCTGGCTAAAGAAGGCGCGTACGCTCAAGTCCGCCTGCGCTCTGGCGAAGTC
CGTAAAATCAACGTAGATTGCCGTGCAACCATCGGTGAAGTCGGTAACGAAGAGCAAAGC
CTGAAAAAAATCGGTAAAGCCGGTGCCAATCGTTGGCGCGGTATTCGTCCGACTGTACGT
GGTGTTGTCATGAACCCTGTCGATCACCCGCATGGTGGTGGTGAAGGCCGTACGGGCGAG
GCCCGCGAACCGGTCAGCCCATGGGGTACTCCTGCTAAAGGCTACCGCACTCGTAATAAC
AAACGCACGGATAACATGATTGTTCGTCGCCGTTACTCAAATAAAGGTTAATTTAGTATG
GCTCGTTCATTGAAAAAAGGCCCATATGTAGACCTGCATTTGCTGAAAAAAGTAGATGCT
GCTCGCGCAAGCAACGACAAACGCCCGATTAAAACCTGGTCTCGTCGTTCTACCATTCTG
CCTGATTTTATCGGTCTGACCATTGCTGTGCACAACGGCCGCACCCATGTGCCTGTGTTT
ATCAGCGACAATATGGTTGGTCATAAATTAGGCGAATTCTCATTGACCCGTACCTTTAAA
GGCCACTTGGCCGATAAAAAGGCTAAAAAGAAATAAGGTGAATCATGAGAGTAAATGCAC
AACATAAAAATGCCCGTATCTCTGCTCAAAAGGCTCGTTTGGTAGCTGATTTGATTCGTG
GTAAAGACGTTGCCCAAGCTTTGAATATTTTGGCTTTCAGTCCTAAAAAAGGTGCCGAGC
TGATTAAAAAAGTATTGGAGTCAGCTATTGCTAATGCCGAGCACAATAACGGTGCGGACA
TTGATGAACTGAAAGTGGTAACTATCTTTGTTGACAAAGGCCCAAGCTTGAAACGTTTTC
AAGCTCGCGCCAAAGGTCGCGGTAACCGCATCGAAAAACAAACTTGTCATATCAATGTGA
CAGTGGGTAACTAAGGAAAAGCTATGGGACAAAAGATTAACCCTACAGGCTTTCGCCTGG
CGGTAACTAAAGACTGGGCTTCAAAATGGTTTGCTAAAAGCACCGACTTTTCTACTGTTT
TGAAGCAGGATATCGATGTTCGCAATTATTTGCGTCAAAAATTGGCCAATGCTTCGGTTG
GTCGAGTGGTTATTGAACGCCCTGCAAAATCTGCACGCATTACCATTCACTCCGCTCGTC
CGGGTGTGGTTATCGGTAAAAAAGGTGAGGATATCGAGGTTTTGAAACGTGACTTGCAAG
TCTTGATGGGTGTACCTGTTCATGTAAATATTGAAGAGATTCGCCGTCCTGAGTTGGATG
CTCAAATTATTGCTGACGGTATTGCCCAGCAGTTGGAAAAGCGCGTTCAATTCCGTCGTG
CTATGAAACGAGCAATGCAAAATGCAATGCGTTCTGGTGCTAAAGGCATTAAGATTATGA
CTTCAGGCCGTCTGAATGGTGCGGATATTGCCCGTAGCGAATGGTATCGTGAAGGTCGCG
~~TGCCACTGCATACTTTACGTGCAAATGTAGATTATGCAACCAGCGAAGCGCACACCACAT~~
ATGGTGTATTGGGTCTGAAAGTTTGGGTTTATACGGAAGGCAATATTAAATCTTCCAAAC
CTGAACATGAGAGTAAACAAAGAAAGGCAGGTAGACGTAATGCTGCAGCCAACTAGACTG
AAATACCGTAAGCAACAAAAGGGTCGCAATACCGGCATCGCTACTCGCGGTAATAAGGTA
AGTTTCGGTGAGTTCGGCTTGAAAGCCGTAGGTCGTGGTCGTTTGACTGCCCGTCAAATC
GAAGCTGCTCGTCGTGCAATGACCCGTCATATCAAACGTGGTGGTCGTATTTGGATTCGT
GTATTCCCTGATAAACCGATTACTGAAAAGCCTATTCAAGTTCGTATGGGTGGCGGTAAA
GGTAACGTGGAATATTACATTGCCGAAATTAAACCAGGTAAAGTGTTGTATGAAATGGAT
GGCGTTCCAGAGGAACTGGCTCGTGAAGCATTCGAGTTGGCTGCTGCCAAATTGCCTATT
CCTACAACCTTTGTAGTAAGACAGGTGGGTCAATAATGAAAGCAAATGAATTGAAAGACA
AATCCGTTGAGCAGTTGAATGCAGATTTGTTGGACTTGTTGAAAGCTCAGTTTGGCTTAC
GTATGCAAAACGCTACCGGTCAATTAGGCAAACCAAGTGAATTGAAACGTGTACGTCGCG
ATATTGCTCGTATTAAAACCGTTTTAACTGAAAAAGGTGCTAAGTAATGAGCGAAACTAA
AAATGTTCGTACTTTGCAAGGCAAAGTAGTAAGCGACAAAATGGATAAAACCGTAACAGT
ATTGGTTGAGCGTAAAGTAAAACATCCGCTGTATGGTAAGATTATTCGATTATCTACTAA
AATCCATGCCCATGATGAAAATAATCAATATGGAATTGGTGATGTGGTTGTTATATCGGA
ATCCCGTCCATTGTCAAAAACTAAATCTTGGGTTGTCAGTGAGCTGGTTGAGAAAGCACG
TTCTATTTAAGAATTAAAGCAACGTGCTTGGAATGGGAAACGAAGTATTGCAGCAAATTT
AATTTGCGTGTAAACTTCGTTTCCTGTCTTTCAGTTTCTTCTGGAAGTTTCTTCCCTTTC
GGGGTCCAAGACTGGTTTACTTGAACCGCAAGGTTTCATTTAATAAGCAGCGGCTTTGCT
GTAAGTTATCTGAAAGTGGTAAATTAAGTTGGTTAATTTAAAGGTAATAACATGATTCAA
ATGCAGACCATCTTAGATGTGGCTGATAACTCTGGTGCGCGTCGCGTAATGTGTATCAAG
GTATTGGGCGGATCTAAGCGTCGCTACGCTTCTGTTGGCGATATTATTAAAGTGGCAGTT
AAAGATGCGGCTCCGCGTGGCCGTGTCAAAAAAGGCGATGTATATAATGCGGTAGTTGTT
CGTACTGCTAAGGGTGTACGTCGTCCTGATGGTGCGTTAATTAAATTCGATAACAATGCC
GCCGTGTTACTGAATAATAAACTTGAACCTTTGGGTACTCGTATCTTTGGTCCGGTAACC

## Appendix A

CGTGAATTGCGTACTGAGCGATTTATGAAAATCGTTTCATTGGCACCTGAAGTATTATAA
GGAATGGCACGATGAATAAAATCATTAAAGGCGATAGGGTTGTAGTAATTGCTGGTAAGG
ATAAAGGTAAGCAGGGTCAAGTAGTTCGAGTGTTGGGTGATAAAGTTGTTGTTGAGGGCG
TTAATGTTGTAAAACGCCATCAAAAACCTAATCCAATGCGTGGCATTGAGGGCGGTATTA
TTACTAAAGAAATGCCTTTGGATATTTCTAATATCGCAATCCTGAATCCGGAAACTAATA
AAGCGGACCGTGTTGGTATTAAGCTGATTGAAAATGAAGGCAAAGTTAAACGCGTTCGTT
TCTTCAAATCAAATGGCTCTATCATTGGGGCATAAGGAGATAACATGGCTCGGTTGAGAG
AGTTTTATAAAGAGACAGTTGTTCCTGAATTGGTTAAACAATTTGGTTACAAATCAGTAA
TGGAAGTCCCGCGTATTGAAAAAATTACCTTGAATATGGGTGTGGGTGAGGCTGTTGCTG
ATAAAAAAGTTATGGAACATGCTGTTTCCGATTTAGAGAAAATTGCCGGTCAAAAACCGG
TTGTTACTGTTGCCCGTAAATCTATCGCAGGTTTTAAAATCCGTGATAACTATCCGGTTG
GTTGCAAAGTAACATTGCGTCGTGATCAAATGTTTGAATTCTTGGATCGTTTGATTACTA
TTGCATTACCTCGCGTACGTGACTTCCGTGGTGTGAGCGGTAAATCATTTGATGGCCGTG
GCAATTACAATATGGGTGTTCGTGAGCAAATTATTTTTCCGGAAATTGAATACGATAAAA
TTGATGCTTTGCGTGGTTTGAATATTACTATTACTACTACAGCAAAAACCGATGAGGAAG
CGAAAGCTTTATTGTCATTGTTTAAATTTCCGTTCAAAGGATAATCATGGCTAAGAAAGC
ACTTATTAATCGTGATCTGAAACGTCAAGCTTTGGCTAAAAAATATGCGGCTAAACGCGC
GGCAATTAAAGCGGTAATCAATGATTCGAATGCAACTGAGGAAGAGCGTTTTGAGGCTCG
TTTGAGGTTTCAATCCATTCCTCGTAATGCGGCACCTGTGCGTCAACGTCGTCGTTGTGC
TTTGACAGGTCGCCCTCGTGGTACTTTCCGTAAATTTGGTTTGGGTCGTATTAAAATCCG
TGAAATCGCCATGCGTGGCGAAATTCCGGGTGTTGTTAAAGCCAGCTGGTAATAGGAGTA
ATTAAGAATGAGTATGCATGATCCTATTTCCGATATGTTGACTCGTATCCGCAATGCGCA
ACGTGCTAATAAAGCAGCGGTTGCAATGCCTTCTTCAAAATTAAAGTGTGCTATTGCAAA
GGTATTGAAAGAAGAAGGATATATTGAGGACTTCGCAGTTTCATCTGACGTAAAGTCTAT
ATTGGAAATTCAATTAAAATACTATGCAGGTCGTCCTGTAATTGAACAAATCAAGCGTGT
ATCTCGCCCCGGTTTGCGTATTTATAAAGCGTCTAGTGAGATTCCAAGTGTTATGAATGG
CTTGGGTATTGCTATTGTTAGTACTTCTAAAGGTGTAATGACTGATCGTAAAGCACGTTC
TCAAGGTGTTGGTGGTGAGTTGTTATGCATTGTAGCCTAGTGGAGGAAAAGAAATGTCAC
GTGTCGCAAAAAACCCAGTGACTGTTCCCGCTGGTGTAGAAGTAAAATTTGGAGCAGAGG
CATTAGTTATTAAGGGTAAGAACGGTGAATTGTCTTTTCCTTTGCATTCTGATGTAGCCA
TTGAATTTAATGATGGCAAATTGACTTTTGTTGCGAATAACAGCAGTAAACAAGCAAATG
CAATGTCTGGTACTGCTCGCGCATTAGTCAGCAATATGGTTAAAGGTGTTTCAGAAGGTT
TTGAGAAAAGATTGCAATTGATAGGTGTGGGTTATCGTGCTCAAGCACAAGGTAAAATCT
TGAATCTGTCTTTGGGTTTTTCTCATCCGATCGTATATGAAATGCCTGAAGGTGTCTCCG
TTCAAACTCCTAGCCAAACAGAGATTGTTTTAACCGGCTCGGATAAACAAGTTGTTGGTC
AAGTTGCTGCTGAGATTCGTGCGTTCCGTGCTCCTGAGCCTTATAAAGGTAAAGGTGTTC
GCTATGTAGGAGAAGTAGTGGTAATGAAAGAAGCCAAGAAAAAATAATTGAGGTTCACTA
ATGGATAAACATACAACCCGACTCCGTCGTGCACGCAAAACCCGTGCTCGTATTGCGGAC
TTGAAAATGGTAAGATTATGTGTGTTCCGAAGCAATAATCATATTTATGCTCAAGTAATT
AGTGCTGAAGGTGATAAAGTATTGGCTCAAGCCTCTACATTGGAAGCTGAGGTGCGCGGT
AGTCTGAAATCTGGAAGCAATGTTGAAGCAGCTGCAATAGTTGGTAAACGTATCGCTGAA
AAAGCTAAAGCAGCAGGTGTAGAAAAGGTTGCTTTTGATCGTTCAGGTTTCCAATATCAC
GGTCGTGTGAAGGCTTTGGCTGAAGCTGCTCGTGAAAATGGTTTAAGCTTCTAAATATTT
GGAGACTTTCAGATGGCAAAACATGAAATTGAAGAACGCGGTGACGGTCTGATTGAAAAG
ATGGTCGCTGTTAATCGCGTAACTAAAGTAGTTAAAGGTGGCCGTATCATGGCTTTCTCA
GCACTGACTGTTGTTGGTGATGGTGATGGTCGCATTGGTATGGGCAAAGGTAAATCAAAA
GAAGTACCAGTTGCTGTTCAAAAAGCAATGGATCAAGCTCGACGCTCTATGATTAAAGTA
CCTTTGAAAAACGGTACTATTCATCATGAGGTTATTGGCCGTCATGGTGCTACTAAAGTA
TTTATGCAGCCTGCTAAAGAGGGTAGTGGCGTAAAAGCCGGTGGACCTATGCGTTTGGTT
TTTGATGCTATGGGCATTCATAATATCTCCGCCAAAGTGCACGGATCTACTAACCCATAT
AATATCGTACGTGCAACATTAGATGGTTTGTCTAAGTTGCATACTCCTGCTGATATCGCA
GCCAAACGTGGCTTGACAGTGGAAGACATTTTGGGAGTTAACCATGGCTGAACAAAAAAA
GATTAGGGTTACATTGGTTAAAAGCCTGATTGGTACAATTGAATCTCATCGTGCATGTGC
ACGCGGTTTAGGTTTGCGTCGTCGCGAGCATACGGTAGAGGTTTTAGATACCCCTGAAAA
CCGTGGTATGATTAATAAAATCAGCTACTTGTTGAAAGTGGAGTCTTGATATGTTTTTGA
ATACAATTCAACCTGCTGTTGGTGCTACGCATGCTGGTCGTCGTGTTGGACGCGGTATTG
GTAGTGGTCTTGGCAAAACGGGTGGTCGTGGTCATAAAGGTCAAAAGAGCCGGTCTGGTG
GGTTTCATAAGGTGGGTTTCGAGGGTGGTCAAATGCCCTTGCAACGACGCCTCCCTAAAA
GAGGTTTTAAATCTTTAACAGCATCAGCTAATGCACAGCTTCGTTTAAGTGAACTGGAAT
CAATTGCTGTTAATGAGATTGATATTTTGGTCTTAAAGCAAGCGGGTCTGATTGCATCTA
CAGTCTCTAATGTTAAAGTTATTGCTTCTGGTGAAATTTCTAAGGCAGTTGCTTTGAAGG
GTATTAAAGTTACCAAAGGTGCGAGAGCTGCTATCGAGGCTGTTGGTGGTAAGATTGAAA
TGTAAGGTTTAATATTGTGGCTAATCAACAAACGTCATCAGGTTCATCCAAATTTGGAGA
TCTTAAGAAACGTCTTTTGTTTCTATTTGGAGCATTGATTGTTTTTCGAATTGGTGCCCA
TATACCCGTACCTGGAGTTGATGCTGTTGCTTTAGCTAAATTATACGAAAGCGCTGGAAA
CGGCATCCTGGGAATATTGAATATGTTTTCCGGTGGGTCGTTAGAGCGCTTTAGTATATT
TGCAATAGGAATTATGCCATATATTTCAGCTTCTATTATTGTACAGCTCGCTTCTGAAAT
TTTGCCATCATTGAAGGCTTTAAAAAAAGAAGGGGAGGCTGGTAGAAAGGTAATTACGAA
ATATACTAGGTATGGTACTGTTTTGTTAGCAATTCTTCAAAGTCTAGGTGTTGCATCTTT
CGTATTTCAGCAAGGAATTGTTGTAACAAGTTCATTTGAGTTTCATGTTTCCACGGTAGT
TTCTTTGGTAACGGGAACCATGTTTCTTATGTGGCTTGGGGAGCAAATTACTGAAAGGGG
TATCGGGAACGGTATTTCTTTAATCATTACGGCAGGTATTGCTTCAGGTATTCCTTCGGG
TATTGCAAAGCTGGTTACACTGACGAACCAAGGTTCTATGAGCATGCTTACGGCGTTGTT
TATTGTATTTGGTGCCTTATTATTAATTTATTTGGTTGTATACTTTGAAAGTGCACAGCG
GAAGATTCCTATTCATTATGCAAAACGCCAGTTTAATGGTAGGGCGGGTAGTCAAAATAC

## Appendix A

GCATATGCCTTTCAAGTTGAATATGGCTGGTGTTATTCCCCCAATTTTTGCTTCCAGTAT
TATTCTATTTCCATCTACTCTTTTAGGTTGGTTTGGTTCGGCTGATACAAATAGTGTTTT
GCACAAAATAGCTGGATTGTTACAACACGGTCAATTGCTGTATATGGCTTTATTTGCAGC
GACAGTTATTTTCTTTTGTTATTTTTATACGGCTTTGGTTTTTAGCCCTAAAGAAATGGC
AGAGAATTTAAAAAAGAGTGGTGCTTTTGTTCCTGGGATTAGACCTGGTGAGCAGACCTC
TAGGTATTTAGAAAAAGTTGTATTACGTTTGACATTGTTTGGAGCTCTTTATATTACAAC
TATTTGTTTAATTCCAGAGTTCTTAACTACGGTTTTAAATGTACCTTTTTATTTGGGTGG
CACGTCTTTGTTGATTCTAGTTGTTGTAACGATGGATTTTAGTACACAAATAAATTCGTA
TAGGCTTACTCAACAGTATGATAAGTTAATGACTCGTTCAGAAATGAAATCATTTTCTCG
GAAATAGAATTATGGCGAAAGAAGATACTATCCAAATGCAAGGTGAAATTCTTGAAACTT
TACCTAATGCAACATTTAAAGTAAAACTTGAGAATGACCATATTGTATTGGGTCATATTT
CTGGGAAGATGCGGATGCATTACATTCGTATTTCTCCGGGAGATAAGGTCACAGTAGAGC
TGACACCTTATGATCTAACTAGGGCTCGAATCGTTTTCAGAGCAAGATAAACCAATAAAA
GGAAAATAAAATGCGTGTACAACCATCTGTTAAGAAAATTTGCCGAAATTGCAAGATTAT
TCGTCGAAATCGTGTAGTTCGTGTAATTTGTACTGATCTCCGTCACAAACAGCGTCAAGG
TTAATGGAATATTTCTTTTAATGTGATTCTGTGATATAGTGACACACTTTGCCCTAAAAA
GGAAAAAATATGGCTCGTATTGCAGGGGTAAATATCCCTAATAACGCACACATCGTAATT
GGTCTTCAGGCTATTTACGGTATTGGTGCTACTCGTGCTAAATTGATTTGTGAGGCTGCA
AATATTGCGCCTGATACTAAAGCAAAAGATTTGGACGAGACTCAATTAGATGCTTTGCGT
GACCAAGTTGCCAAGTATGAAGTAGAAGGTGATTTGCGTCGTGAGGTAACTATGAGTATC
AAGCGATTGATGGACATGGGCTGCTATCGTGGCTTCCGTCATCGTCGCGGCTTACCATGC
CGCGGTCAACGCACTCGTACAAATGCGCGTACCCGCAAAGGTCCGCGTAAAGCGATTGCT
GGTAAGAAATAAATTTTAAGGAATTTTATTAATGGCTAAAGCAAACACAGCTTCACGTGT
ACGTAAAAAAGTACGTAAAACCGTGAGTGAGGGTATTGTGCACGTTCATGCATCTTTCAA
CAATACCATCATTACAATCACTGACCGTCAAGGCAATGCGTTGTCTTGGGCTACCTCTGG
CGGCGCTGGTTTTAAAGGTTCTCGTAAAAGTACACCATTTGCAGCACAAGTTGCAGCAGA
AGCAGCTGGTAAAGTTGCCCAAGAGTATGGCGTTAAAAATTTAGAGGTTCGTATTAAAGG
TCCAGGTCCAGGTCGTGAATCCTCTGTACGTGCTTTGAATGCTCTTGGTTTCAAGATTAC
CAGCATTACTGACGTTACCCCGTTGCCTCATAACGGTTGCCGTCCGCCTAAAAAACGTCG
TATTTAATATTGGAGTGATTTGAAACATGGCACGTTATATTGGCCCTAAATGTAAGTTGG
CACGTCGCGAAGGTACGGATTTGTTTTTGAAGAGTGCGCGCCGCTCTTTGGATTCTAAAT
GTAAAATTGATTCCGCTCCTGGTCAGCATGGTGCAAAAAAACCGCGTTTGTCAGACTATG
GTTTGCAGTTGCGTGAAAAACAAAAAATCCGCCGTATTTATGGCGTATTAGAACGTCAGT
TCCGTCGTTATTTCGCAGAAGCTGATCGTCGTAAAGGTTCTACCGGCGAGTTGCTGTTGC
AGTTGCTGGAATCTCGTTTGGATAATGTCGTTTATCGTATGGGTTTCGGTTCTACCCGAG
CTGAAGCAAGACAGCTTGTTTCTCATAAGGCGATAGTTGTGAATGGACAAGTTGTCAATA
TTCCTTCTTTCCAAGTGAAAGCTGGTGATGTTGTCTCAGTTCGTGAAAAAGCCAAAAAAC
AGGTACGTATTCAAGAAGCATTGGGTTTGGCAACTCAAATCGGCTTGCCGGGTTGGGTTT
CTGTAGATGCGGATAAACTTGAGGGTGTGTTCAAAAACATGCCGGATCGCTCGGAATTGA
CCGGTGATATTAATGAACAGCTGGTGGTAGAGTTCTACTCTAAATAATGCTAGCTCAGTG
AGGGACAGTTAAATGCAGAATAGCACAACCGAATTTTTGAAACCTCGTCAAATTGATGTA
AATACTTTTTCTGCAACTCGTGCAAAAGTATCTATGCAGCCATTTGAACGTGGTTTCGGT
CATACCTTAGGTAATGCTTTGCGCCGTATCTTACTGTCATCCATGAATGGTTTTGCTCCT
ACTGAAGTAGCTATTGCCGGTGTATTACACGAATATTCTACTGTTGATGGTATTCAGGAA
GATGTTGTTGACATTTTGCTGAATATTAAAGGTATTGTGTTTAAACTCCATGGTCGTAGC
CAAGTTCAACTTGTGTTGAAGAAATCAGGTTCAGGTGTCGTATCTGCCGGTGATATTGAG
TTGCCGCATGATGTAGAAATTCTGAATCCTGGTCATGTCATTTGTCATTTGGCTGATAAC
GGTCAAATTGAGATGGAAATTAAAGTAGAGCAAGGTCGTGGTTATCAATCTGTTTCAGGT
CGTCAGGTAGTTCGTGATGAGAACCGTCAGATTGGTGCAATCCAGTTGGATGCGAGCTTT
TCGCCCATCAGCCGTGTTAGCTTTGAGGTTGAACCTGCACGTGTAGAGCAGCGGACGGAT
CTTGATAAGTTGGTTTTGGATATCGAAACCGACGGTTCTATTGATCCTGAGGAAGCTGTA
CGCAGTGCGGCACGTATTTTGATTGATCAGATGTCTATTTTTGCTGATTTGCAGGGTACG
CCTGTGGAGGAGGTTGAAGAAAAAGCACCTCCTATCGACCCTGTTCTTTTGCGTCCGGTG
GATGATCTGGAATTGACAGTACGTTCAGCTAATTGTTTGAAAGCTGAGGATATTTATTAT
ATTGGCGATTTGATTCAACGCACTGAAACCGAGCTTCTTAAAACGCCGAATTTGGGACGT
AAATCTTTGAATGAGATTAAGGAAGTATTGGCATCTAAAGGTTTGACACTGGGTTCTAAG
TTGGAAGCATGGCCACCTGTAGGCTTGGAAAAGCCTTAATGAAGAATTAAAGGATAATTG
ATATGCGTCATCGTAATGGCAATCGCAAATTAAACCGTACCAGCAGTCATCGTGCTGCAA
TGCTGCGTAATATGGCGAATTCATTATTGACTCACGAAGCTATTGTAACAACTCTGCCTA
AGGCCAAGGAATTGCGCCGTGTAGTAGAGCCGTTGATTACATTGGGTAAAAAGCCGTCAT
TGGCAAACCGCCGTTTGGCATTTGACCGTACTCGCGACCGTGATGTTGTAGTAAAACTGT
TTGGCGATTTGGGTCCTCGTTTTACTGCTCGTAACGGTGGTTATGTTCGGGTGTTGAAAT
ACGGATTCCGTAAAGGTGATAATGCACCTCTGGCACTGGTTGAATTGGTTGACAAACCGG
CTGCTGAGTAATTTTAGTCATATAACGCCATCTGCCGAAAAGCAGGTGGCGTTATTTTTG
CAATATCTGATAGGTAATAGGGTATTGGCTATCATGTTTAAAATATTAATTGAATAGCTA
AGGTTTGCGCGGTAAACTTACATCATTAAAAAATTCTATGATGGTTTATATAATGAATGC
TTTCGATATAAAGTCGACAAAGATGGACGTATTGTCTATATCTTTGCATACGTCAGACTT
GTTTGATTTGGAAGATGTGCTGGTCAAATTGGGCAAGAAGTTTCAAGAGTCTGGTGTTGT
TCCATTTGTGCTGGATGTTCAAGAGTTTGATTATCCCGAGTCTTTGGATCTTGCTGCATT
GGTTTCGTTGTTTTCAAGGCATGGTATGCAAATTTTGGGTCTGAAGCATTCTAATGAACG
TTGGGCTGCTGCGGCTATGAAGTATCATTTGCTGTTTTGTCTGTCTCATTCGGAAAATGT
TAAAGAACTGGGTCAGGTTGAGGTGCAGAAAACGGAGGATGGTCAGAAAGCAAGGAAAAC
AGTATTGATTACATCCCCTGTCCGTACCGGTCAGCAGGTTTATGCCGAAGATGGCGATTT
GATTGTTACGGGGGCGGTCAGCCAGGGGGCGGAATTGATTGCGGATGGCAATATACATAT
TTATGCGCCGATGAGGGGGCGTGCTTTGGCCGGTGCCAAGGGTGATACTTCTGCCCGCAT

## Appendix A

ATTTATCCACTCCATGCAGGCAGAACTGGTTTCTGTGGCGGGTATTTACCGTAATTTTGA
ACAGGATTTGCCGAACCATCTGCACAAGCAGCCGGTACAGATATTGTTGCAGGATAACCG
ATTGGTTATCAGTGCAATTGGCTCAGAGTAATTGTTTGATATTTAAAAAGGAAATATTGT
GGCAAAAATTATTGTAGTAACTTCAGGTAAGGGCGGTGTCGGTAAAACGACTACCAGTGC
CAGTATTGCGACAGGTTTGGCATTACGCGGATATAAAACTGCGGTAATTGATTTTGATGT
GGGTTTGCGTAACCTCGACCTCATTATGGGTTGCGAGCGTCGTGTCGTTTATGACCTGAT
CAATGTCATTCAGGGGGAGGCGACGCTCAACCAAGCTTTGATTAAAGATAAAAATTGTGA
AAACCTGTTTATTTTGCCGGCTTCCCAGACTCGGGATAAAGACGCTTTGACACGCGAGGG
CGTAGAAAAAGTGATGCAGGAGCTGTCCGGCAAGAAAATGGGCTTTGAGTATATTATTTG
CGACTCTCCTGCCGGTATTGAGCAGGGTGCATTGATGGCGTTGTATTTTGCTGATGAAGC
CATTGTAACGACCAATCCTGAGGTTTCCAGTGTGCGTGACTCCGACAGGATTTTGGGAAT
TTTGCAAAGCAAATCCCATAAGGCAGAGCAAGGCGGTTCGGTTAAAGAACATCTGTTGAT
TACGCGTTATTCTCCCGAACGTGTGGCAAAAGGCGAAATGCTGTCTGTACAGGATATTTG
CGATATTCTGCATATTCCTTTGCTGGGTGTGATTCCTGAATCCCAAAACGTCTTGCAGGC
ATCCAATTCCGGAGAACCGGTCATCCATCAGGACAGCGTGGCGGCTTCCGAGGCATATAA
GGACGTTATTGCCCGTCTTTTGGGCGAGAACCGTGAAATGCGTTTCTTGGAAGCTGAGAA
AAAAAGCTTCTTCAAACGTCTGTTTGGAGGATAAGGTATGTCATTAATCGAATTTTTATT
CGGCAGAAAGCAGAAAACGGCAACCGTTGCCCGCGACCGCCTTCAAATCATCATTGCCCA
AGAGCGCGCCCAAGAAGGTCAGGCTCCGGATTACCTGCCGACTTTACGTAAAGAGTTGAT
GGAAGTCCTGTCCAAATATGTGAATGTTTCATTAGACAATATCCGTATTTCCCAAGAAAA
GCAGGATGGTATGGATGTGCTTGAGTTGAACATTACTTTGCCGGAACAGAAAAAGGTATA
GGACATGACCTTAACCGAATTGCGGTACATCGTCGCAGTCGCCCAAGAACGTCATTTCGG
CAGGGCGGCGCGGCGTTGTTTTGTCAGCCAGCCCACTTTGTCTATTGCCATTAAGAAATT
GGAAGAAGAGCTTGCCGTCTCTTTGTTTGACCGGAGCAGTAACGATATTATTACGACCGA
GGCGGGGGAACGTATCGTTGCACAGGCGCGTAAGGTATTGGAAGAGGCGGAGCTTATCAG
GCATTTGGCAAATGAAGAACAAAACGAGCTGGAGGGTGCGTTCAAACTCGGGCTGATTTT
TACGGTTGCGCCGTACCTGCTGCCGAAACTGATTGTTTCGTTGCGCCGTACTGCACCGAA
AATGCCTTTGATGTTGGAAGAGAATTACACGCATACTTTGACCGAGTCGCTCAAACGCGG
GGACGTTGATGCGATTATCGTTGCCGAACCGTTTCAAGAGCCGGGCATTGTTACCGAACC
CTTGTATGACGAACCGTTTTTCGTGATTGTCCCGAAAGGGCATTCATTTGAGGAACTGGA
TGCCGTTTCGCCCCGGATGCTGGGTGAGGAGCAGGTTTTGCTGCTGACGGAAGGCAACTG
TATGCGGGATCAGGTACTCTCAAGCTGTTCCGAATTGGCGGCGAAACAACGTATACAGGG
GTTGACCAATACATTGCAGGGCAGCTCGATTAATACAATCCGCCATATGGTTGCCAGCGG
TTTGGCAATCAGCGTGTTGCCGGCAACCGCACTGACCGAAAACGATCATATGCTGTTCAG
CATTATTCCGTTTGAGGGTACGCCGCCAAGCCGGCGGGTCGTATTGGCGTACCGCCGCAA
TTTTGTCCGTCCGAAGGCGTTGTCGGCGATGAAGGCGGCGATTATGCAGTCGCAGCTTCA
CGGGGTAAGTTTTATCTGCGACTAGGCGCAGGCATTGTTTTCAAAACGCCATTTCCCTGA
GCCGACAACACGGTATGCCAAGATATTGCCGTCATCATCGATTTTGAGTATAGCATCGCC
ACGGAAACTGCCGTCCTGAAGATATTCGACTTTTGCATCACTGTGAATGTTTTCATCAGT
GCCGATGCAATGCCATGTATAGTGGATTAACAAAAACCAGTACGGCGTTGCCTCGCCTTG
CCGTACTATTTGTACTGTCTGCGGCTTCGTCGCCTTGTCCTGATTTTTGTTAATCCACTA
TAAAAGAGGCCGTCTGAAAAACATTTTTCAGACGGCCTTGTTTATTCAATCAAATCAGTC
TTTCAACTTCGCCAACTGATTTTGAACTTTTGCCATTTTGTCTTCCAATTCCGCCAAATC
GGCTTTGTCTTTTTCCACCAGATGCGCAGGGGCTTTTTCGGTGTAGCCGGGTTTGGAGAG
TTTGGCGTTGAGTTTGTCCAAGGCTTTTTGCAGCTTCTCGGCTTCTTTGCTCAAACGGGC
GGTTTCGGCGGCTTTGTCGATTTCGACTTTCAACATCAGGCGCGCGCCGTTGCAGACGGC
GACGGGCGCGTCTTCGCTTTCGGGTAGGGCGGCGACTTGCTGTGCTTCGGTCAGGCGGGT
CATCATCGGCAGGTATTTGAGGTAGTCCGCCAAGTCGTCCGTGCTTTCGACAAACAGCGG
GGCTTTTACGTTGGGCTGGATGCCCATTTCGCCGCGCAGGTTGCGGACTGCGCCAATCAA
~~ATCCTGCAACACGGTCATTTGCTCGAATGCCGTCTGAACAATCTCGGCGCTGTCGGCTTC~~
GGGGAAGCGGGCGAGCATGATGCTGTCGGCGGTTTTCGCGTCGCACATAGGAGCGACGGT
TTGCCACAGTTCTTCGGTGATGAACGGGATAATCGGGTGCAGCAGGCGCAGGGCGGCTTC
GAGTACGCGCAATAAGGTATGGCGTGTGGCGCGTTGGCGGCTGGCGCAGCCGGTTTGAAG
CTGCACTTTGGCGAGTTCCAAATACCAGTCGCAATAGTCGTTCCATACGAAGCTGTACAG
GGTTTCCGCCGCCAAATCAAAGCGGTAGGTTTCGTAGGCTTGCGTAACCTGTTCGATGGT
CTGATTCAGACGGCCTACAATCCACATATCGGGGAAGGAGTAGCCGCGCGGTTCGGCAGC
GGTTGCGCCGTAACCGCAGTCTTGGTTTTCGGTGTTCATCAAGACGAAGTTGGTGGCGTT
CCAGATTTTGTTGCAGAAGTTGCGGTAGCCTTCGGCGCGTTTGAAGTCGAAGTTGACCGA
ACGCCCCAAGCTGGCGTAGCTCGCCATAGTGAAGCGCAAAGCGTCCGCGCCCATACTCGG
AATGCCTTCGGGGAAGAGTTTTTTCGTGGCTTCTTCCACTTTCGGCGCGGTTTCGGGTTT
GCGCAGGCCGGTGGTGCGTTTTACCAGCAGTTTTTCCAAGCCGATGCCGTCGATCAAATC
CACAGGGTCAATGACGTTGCCTTCGGATTTGGACATTTTTTTGCCTTCGTGGTCGCGCAC
GATGCCGTGGATGTACACGTCTTTAAACGGTACTTTGCCGGTGAAGTGGGTGGTCATCAT
AATCATACGCGCCACCCAGAAGAAGATGATTTCGTAGCCGGTTACTAAGACATTGGACGG
CAGGAAGGCTTTGAGTTCGTCGGTTTCAGACGGCCAGCCGAGTGTGGAGAACGGCACAAG
CGCGGAGGAGAACCATGTATCCAATACGTCTTCTTCGCGAGTCAAGCCTGTTTTGCCGGC
TTGTTTTTCGGCTTCTTCCTGATTGCGGGCAACATACACATTGCCTTCGTTGTCGTACCA
TGCAGGGATTTGATGGCCCCACCACAGTTGGCGTGAGATACACCAGTCTTGGATGTTGTT
CATCCATTGGTTGTAAGTGTTGACCCAGTTTTCAGGGATAAAGCGTACCGCGCCGCTATC
AACGGCTTTTTTGGCTTTATCGGCGAGGCTCAAGCCTTTGAACTCGCTGTCCGGCTCGCC
GCCGTTTGGGGTGGCGGACATGGCGACAAACCATTGGCTGGTCAGCATAGGTTCAATCAC
CGAACCTGTACGGTCGCCTTTCGGCGTCATCAGCGTGTGTGGTTTGATTTCGACCAAGAA
ACCTTGTTCCTGCAAATCGGCAACCATTTGTTTGCGCGCGGCAAAGCGGTCTAAGCCTGC
GTATTTTTCAGGCAGGGCAAAGCCTAGTTGCGCTTCGCCTTTGAAGTTGAACACTTCGGC
GTTTGCCAGCACTTTGGCTTCCAAGTTGAACACATTAATCAGGCGCGTGTCGTGGCGTTT

# Appendix A

GCCGACTTCGTAGTCGTTGAAGTCGTGTGCAGGCGTGATTTTCACGCAGCCTGTGCCGAA
GTCTTTTTCAACGTATTCGTCGGCAATCACGGGGATAGTACGGCCGGTCAGCGGCAGGAT
TAATTCCTTGCCGATTAAGTGGGTATAACGTTCGTCTTCAGGATTGACGGCAACGGCAAC
GTCGCCCAGCAGCGTTTCAGGACGGGTGGTCGCCACGATAACGGCTTCGGCGGGATTGTC
CGCCAGCGGATAGCGGATGTGCCACATAGAGCCTTGTTCTTCCACGCTTTCCACTTCCAA
ATCCGATACCGCCGTGCCAAGCACGGGATCCCAGTTCACCAAGCGTTTGCCGCGGTAAAT
CAAGCCTTGCTCATACAGGCGCACGAACACTTCGGTTACGGTTTCGGCGCGCACGTCGTC
CATCGTGAAATACTCGCGCGTCCAGTCGGCAGAGCAGCCCACGCGGCGCATTTGTTGGGT
AATCGTGCCGCCGGAAACTTCTTTCCATTCCCACACTTTCTCCAAAAATTTTTCGCGACC
CAAGTCATGGCGGGACACGTTTTGCGCAGCAAGCTGACGCTCAACCACAATCTGCGTGGC
GATGCCCGCGTGGTCTGTGCCGGGAATCCAGGCGGTGTTGCAGCCTTTCATGCGGTAGTA
GCGGGTCAGACCGTCCATAATGGTTTGGTTGAAGGCATGACCCATGTGCAGCGTGCCGGT
TACGTTGGGCGGCGGCAGTTGGATGGAGAAAGACGGTTTCGTCAAATCCATATCAGGTTG
GAAATAGCCCTGCTCTTCCCAGTTTTGATAATGTTTGGATTCGATTTCGGCTGGATTGTA
TTTGTCTAACATGATGGAACTTTGTGAAATTAAGGTTATTTTTGATGTGCGGATTATAAC
GCAAAAAGGCCGTCTGAATCATTTCAGACGGCCTTTGGCATACAGGTTTTAAAAATGGAA
CAATACCAGGCTGACGGCAATCACCGCCATACCCGTTGTCAGGCCGTAAACGGTTTCATG
GCCGTCTGAATAGCGTTTGGCAGCCGGCAGCAGCTCGTCCAACGCCAAAAACACCATCAC
ACCGGCTATCACGCCGAATACCGAACCAAACACGGCAGGCGACAAAAACGGCTGCAAAAC
CAAATAGCCCAAAGCCGCCCCCAACGGCTCGGCCAAGCCGGATAGCAGACACGCCCACAC
CGTTTTCTTACGGCTGCGGGTGGCAAAATAAACCGGCGCGGCGATGGAAATGCCCTCCGG
AATATTATGGATGGCAATCGCCAAGGCCAAAGGCATCCCGACTGCTGGATTTTCCAATGT
GGCAAAAAACGTCGCCAAGCCTTCGGGGAAATTGTGCGCAGTAATCGCAAACGCCGCCAT
CATGCCGACTCGCGCGATATGGCGGCGTTTGCTTTCTTGAAACGACGGGTCTTGCGCGTC
TAAAGTTTCATGCGGGTTCGGCACCAGACGGTCAATCAGCGCAATGCCGCCCATCCCGGC
CAAAAATGCCATGGTCGCCGCCGCAAACGCGTGGTCTTTATCATAAATTTCAGCGAACGC
CTCGCTGGACTTACTGAAAATCTCCGTCAGGGAAACATATACCATCGCACCGCCGGCAAA
CGCCAAACCAAACGACAACACACGCGGATTGGGCGTTTTGGAAAACATCACCAAGCCACT
GCCTAATACGGTAAACAAACCGGCAGCCAATGTGATGGAAAAGGCAACGGCCAAATTGGA
CATCGAAAAATCGGGCATGAGAAAACCTGCGCTAAAAGCTGGGACAGGTTCAGACTAACA
CTTTTTAATGTATATGATAATAGTTATTATTTATTTTATTGATTGGATACACGGATTTTG
AAACAAAAGGCCGTCTGAAAAATGATTTTCAGACGGCCTTTAAATTTGAAATGCCGCTAA
ACCTTAGTGCTTTCCAGCTTAAGCCTGATAACGCGACAGGCTCAAATCGTCGCTGCGGAT
TTCGGTGTCTTTGCCGCTCACGATATCGGCGGTTAATTTTGCCGAACCCAGCGACATGGT
CCAGCCTAAAGTACCGTGGCCGGTATTCAGAAACAGGTTGTCAAAGCGGGTGCGACCGAT
TAACGGCGTGCTGTCGGGCGTCATCGGTCTGAGGCCGCTCCAGAACGATGCTTGGCTCAA
ATCGCCGCCTTCCGGGAACAAGTCGTTGACGACCAAAGCCAAGGTTTCGCGGCGTTTTTC
GGGCAGTTTGATTTCGTAGCCCGACAATTCCGCCATACCGCCGACGCGGATTCTGTTGTC
AAAGCGCGTGATGGCGACTTTGTAGCTTTCATCTAAAACGGTGGACACCGGTGCGCCGTC
TGAATTGGTGACCGGCAGGGTCAAGGAATAGCCTTTGACGGGATAAATGGGCAGATTGAG
ATCCAACTGCGCCAAAACCGTCCTGCTGAAGCAACCGAGCGCGCAGACAACGGCATCTGC
TTCAAACCGCCCTGTTTCGGTTTCAACGGTTTTGATGCGCAGCCCGTTGTGGTCGATGCG
GCTGATGTTTTGGTTGAAATGAAACCGTACGCCCTTTTCCTGACACAATTTGTATAGGTT
TTCAGTGAAGAGGCGGCAGTCGCCGGTCGCATCTGCAGGCAGGTGCAGGCCGCCGGCAAT
TTTGGCGGTAACGCGTGCCAGCGCAGGCTCAAATTCTGCACATTCTTCGGGTTTCAGACG
GCGGTACGGCACGCCGTAGCGTTCCAAAACGGCAATGTCTTGTTTTGCCGCTTCGACTTC
TTTGGTTTGGCGGAAAATCTGCAACGTCCCTTTTTTGCGTCCCTCAAAATTCATGCCGGT
TTGCGCTTCAAAACGGCGGAACATTTCACGGCTGTATTCGGAAATCCTGACCATGCGCTC
TTTATTGGTTTGATAGTGCGCTGCCGTGCAGTTTTGCAGCATTTGCCACAGCCATTCGAT
~~TTGATACAGGCTGCCGTCGGGGCGAAACAGCAAAGGCGGATGGCTTTTAAACAGCCATTT~~
CAGCGCTTTGGTCGGGATACCGGGTGCAGCCCAAGGCGTGGTATAGCCGTAAGAAAGCTG
GCCTGCGTTGGCAAAACTGGTTTCCATCGCCACACCCTCGGCGCGGTCGATGACCGTTAC
TTCATGTCCGGCCTCTGCCAGATACCACGCGGAAGACACGCCGGCAACACCCGCACCTAA
AACAAGCACTTTCATGTTTCTCCCTCCGGCTTTTTCAAAACAGACTTAATATGCCGTGCC
GTCTGAATATTCGGATTCAGACGGCCTCGGATATTAATGCGGCAATTCGCCGTTTGTGAT
TTTTTGTTTGAAGTCGCGCGTTTCATTGACGATGACTTTCGCCATCAATAAAAGTGCAAT
CAGGTTGGGCAATGCCATCAAGCCGTTGAATGTGTCCGAAGCCAGCCACACCAAATCAAG
GCTCAACACGGTACCCAGCATAACGGAAGAAACATAACCCACGCGGTACAAACCGGCAAA
TTTCTCGCCGAAAACATACACCGCGCATTTTTCGCCGTAATAGCACCAGCCCAAAATGGT
TGAGTAGGCAAAGAAAATCAGGCCGATGGTAACAATCCAGCCGCCGATGCCGGGCAGCAT
TTTTTGGAATGTGACGGTTGTCAGTGCCGCGCCGCTCACTTCAGGTTTGACAAACTCGCC
GCCCGCGCCGAGCAGTCCCATTACCAACACGATGCCGGTAATCGAGCAAACGACGATGGT
ATCCAAAAACGTACCGGTCATAGAAACCAAGGCCTGACGGACGGGATGGTCGGTTTTCGC
GGCTGCGGCGGCAATAGGCGCAGAACCCATACCCGCCTCATTGGAGAACACGCCGCGCGC
CACGCCGTAGCGGATGACCGTACCGATAGCACCGCCCGCCACTGCCTGCGCGCTGAACGC
ATCGGAGAAAATCAGCTTGACGGCAGGCATCAGTGCATCGGAATTAATCGCGATAATGGA
AAGACCGCCCAACACATAAAACACCGCCATAGCAGGCACGATGAAAGAAGCGGCTTTGGC
GATGCCTTTAATACCACCTAAAACGACAACGGCAGTCAGAACGGTCAACGTAATGCCGGT
ATAGGCAGGTTCGATACCGAAGCTGGTTTGCACCGCCTGTGCAACCGAGTTGGACTGCAC
CGAGCTGCCGATACCGAAGGAAGCGAATGTGCCGAACAGCGCAAACGCGACGGCCATCCA
TTTCCAGTTTTTGCCCAAGCCTTTTTCGATGTAATACATCGGGCCGCCGGACATTTCGCC
TTTGGAATTGTTGACGCGGTATTTCACCGCCAACACGCCTTCGCCGTATTTGGTGGCCAT
GCCGAAAATGGCGGTCATCCACATCCAAAATACCGCGCCCGGGCCGCCGGTTACCACCGC
AGTCGCCACGCCGGCGATGTTACCCGTGCCGATGGTGGCGGACAGCGCGGTCATCAACGC
CGCAAAATGGGAAATATCGCCTTCGTGGCCTTCGCCGCTTTTATGCTTCTTTGGCGGCAT

## Appendix A

AAACGCCTGTTTCAGCGCATAACCCAACATCGTGAACTGCAAACCTTTTAATAAAACAGT
CAGCAAAATACCCGTGCCGACCAGCAGCATCAGCATCAAAGGTCCCCAAACCCAGCCGCT
GACGGTTTCAAAAAAGGCTTTGGGATTGTCTAAAAACACTTGCATGGCTTTCTCCTTTGT
CTGTTTTATTTTTAAAACACCACTTTTGTAGTGTCCAGTAATTTCAGCACAGAATATCCA
ATAAGACAATATGTTCTTTTGAAAAATACTTTTGGTTTTTTCGCCGAAAACAGGACGGTT
CAAGTTGCGGAAATTGTTTGCAATTCTTTAAAAGCAGCGGCGGAGGTCACAATGAAATGT
CCGAATGGGGATGTGGCGGGCGGCAGAAATCATCAATGCTGCCGACTGCCATACTTCTGA
AATCTACAAAATGATGCATCGATCAAACAATATACCGCTTTAAAAAAACCGATGCCGTCT
GAAACGCTTTCGGGGTTTCAGACGGCATCAAAAGGGTACGGTCAGCGGATGATGCCGCGC
GCCGATTGTGCGAAAAAGTCTCGGAATACGGCAAGCTCGGCTTGGGTTTCGGCGCGGCGG
AGAATGTCTGCCTTGGCTTCTTCAAACGGAATGCCGCGATGGTAGAGGGTTTTGTACACG
TCTTTGACGGCGGAAATCTGCTCTGCGGTAAAACCGTTGCGGCGCATGCCTTCGCTGTTG
AGCCCCGCCGGTTCGGCGCGGTAGCCCGATGCCATAAAGTAGGGCGGCACGTCTTTGTGT
ACGCCTGCGGCAAACGCGGTCATGGCGTAGTCGCCGATGCGGCAGAATTGGAAAACCAGC
GTGTAGCCGCCCAAAACGACGTAGTCGCCGATGGTAACGTGTCCGGCAAGCGAGGCGTTG
TTGGCGAAAATGGTGTGGTTGCCGATGACGCAGTCGTGCGCGAGGTGGCAGTACGCCATA
ATCCAGTTGTCGTCGCCGATACGGGTTTCGCCGATGCCGGTTACCGTACCTAAATTAAAG
GTGGTGAATTCGCGGATGGTGTTGCCGTTGCCGATAATCAGCTTGGTCGGCTCGTCGCGG
TATTTTTTGTCCTGCGGGATTTCGCCGAGGCTGGCAAATTGGAAAATGCGGTTGTTTTCG
CCGATGCTGGTGTGGCCGTTGATGACGGCGTGCGGACCGATTTCGGTATTCGCGCCGATT
TGGACGTTGGGGCCGATAACGGTGTACGCGCCGACTTTGACGCCGGAGTCGAGTTCGGCT
TTGGGGTCGATGACGGCGGTCGGGTGGATGAGGGTCATGTTTTTCCTTTCCTGTCGTGTT
GCCGCGAAGATGCGCGACGGCAACAGGTTGTCTGAAAACTTTCAGACGACCTTTTTCTGA
ACACTCAAACCACGCGTTTGGCACACATGATGATGGCTTCGACGGCAACTTGCCCGTCCA
CTTTGGCAACGGCGTTGAATTTGCCGATGCCGCGCCGGCTGGTCAGCAGCTCGACTTCAA
AGACGAGTTGGTCGCCGGGGATGACTTGGCGTTTGAAACGGGCTTCGTCTATGCCGGCGA
AGAAGAAGAATTCGTTTTCTTTGCGCCCGCCTTCGCTCAAAATCGCCAACGTGCCGCACG
CCTGCGCCATCGCTTCGATGATGAGTACGCCGGGCATCACGGGCAGGTCGGGGAAATGGC
CTTGGAACTGGGGTTCGTTTATGGTGACGTTTTTAATCGCGGTCAGGGTTTTCATCGGCT
CGAAGGCGGTGATGCGGTCGAGCTGGAGAAACGGATAGCGGTGGGGGATGAGTTTTTGGA
TGTCTTTGGCTTCGATGGGGAGTTGTACGTCCATGTCTGTCGTATTCCTTGAATAAAGTC
GGTTTGGTTATTTGCTGTCTTGACCGGCATCTGAAAGCTGCTGCTCCAGTGTTTTGAGCC
GTTTGTTCATTTCGCTTAAGCGGTGGATGTAAACAGCGTTGCGCGCCCATTCTTTATGGG
TGGACATCGGGAAGATGCCGGCGAGGTGTTTGCCGCTTTCGGTAATGCTGTGGGTGACGG
ACGTGCCGCCGCCGATGGTGGTTTTGTCGGCGATTTCGATGTGTCCGACCGTACCGACGC
CGCCGCCGATGATGCAGTAGCTGCCTATGGTTACGCTACCTGAGATGCCGGTTTTGGCGG
CGATGACGGTGTGCGAACCGATTTTGCAGTTGTGTCCGATTTGGACTTGGTTGTCGATTT
TGGTGCCGTTGCCGACGGTGGTGTCGCTCATCGCGCCGCGGTCGATGTTGGTGTTCGAGC
CGATTTCTACGTCGTCGCCCAGCGTTACCGCGCCGGTTTGCGGGATTTTGAACCACGAAT
CGTCGGCGAAGGCGAGTCCGAAACCGTCCGCGCCGATGACCGCGCCGCTGTGGATTTCGA
CGCGTCTGCCCAGTGTGCAGCCGTAATAAACGACGGCGTTGGGATGCAGGACGACTTCGT
CGCCCAGTTTGCAATCGTGTTGGACGACGGCGTTTGCCAAGATGCGGCAGCCTTCGCCGA
GCACGGTGTTTGCGCCGATGTAGACGTTCGCGCCGATTTCGCAGCTGGTGGGAACGGTCG
CGCCCGGTTCGACGACGGCGGTCGGATGGATGCCGCCGCGCGCTTTGACGACGGGTGAAA
ACAGGCGGGCGACTTTGGCGAAATAGAGATAGGGGTCGTCGGCGACAATCAGGTTGCGCC
CTTCAAATCCGTCTGCCGCTTTGGCGGAAACGATGACCGCGCCCGCGCTGCTGTCGTGGA
CTTCGGCTTTGTATTTCGGATTGGCAAGGAAGCTGATGTGTTCCGCCTGCGCGTCTGCGA
GCGGGCGCACGGCGGTAACGGAAATGTCCTCGCCGCGCCATTCGCCGCCGAGCCGCGCGG
TGATTTGGGACAGGGTGTAGGTGGCCGGAATCATGGTTTTCCTGTTCGGTATGCCGTCTG
AAAGGGTCAGCGGGCGTTCATTTCTTTAATGACGCTGTCGGTAACGTCGTATTGGGTGTT
GACGTAAATCACGTTCTGCAAAATGACATCGTAACCTTCCTGTTTGGCGATTTTGACGAT
GACGCGGTTGGCGTTTTGCTGGAGGGAGGCAAACTCTTCGTTGCGGCGGAGGTTGTAGTC
TTCTTCAAACTGCGCCTGTTTTTTGCGGAACGCTGCGACCAGCCCGCGCCATTTTTCTTC
GGCTTGCGCCTTTTTTGCGTTTCTGAGTTTGCCTTCGGCAAGCTGCCTTTCCAAATCCAG
ACCTTCGCGTTGCAGTTTTTGCAATTCGTCCTGACGAGCGGAAAATTCGCTGTCCAGCGT
TTTTTGAATCTTGCGCGCCTGCTTGGATTCGAGGTAGATGCGCTCGGTGTTGATAAAGCC
GATTTTTTGGAAGGTGTCGGCGTGCGCGCCTGCGGTGCAGCACAAACCGATCAGAGCCGC
GGCAAACGCGCGGGTCAAACGGGTCATGGTAAAACTCCTTCGAATGTTGCCGCGAAATGC
CGTCTGAAGGGCTTCAGACGGCATTTGCGGGATTAGAACGTCGTGCCGAGTTGGAATTGG
AAGCGTTGGATTTCGTCTTCCGGTTTTTTCTTCAGCGGGTAGGCGTAGCTGAATTTCATC
GGGCCTAAAGGCGAGAGCCAGGTAACCGCGCCGCCGGCGGAATAGCGCAATTCGTTGGTA
AAGGTGGATTTATGGGTATTGCCGGCGCCGTAAATGTTTTGAACCCTGCCGCCGGTCGCG
GAACTGCTGTTGTCGTCGTAGGTTTTGCCGTCCCACACGCTGCCTGCGTCGGCAAACAGG
CTCAGGCGGACGGTGCGCGCGTCTTTCGCGCCGGGCATCGGGAAGAGCAGCTCGGCGGAG
ACGTTGGCTTTTTTGTTGCCGCCGTAGCTGATTTTTTCGCCGTATTCGTCATAGACTTTC
GGACCGAGCGTGCCGCTTTCGTATCCGCGCACCGAACCCAGGCCGCCGCCGTAGAAGTTT
TCAAAGAAGGGGATTTCTTTGGTTCTGCCGTAGCCGCCCGCAATGCCGACTTCGCCGCCG
AGCATCAGCGTGAAGGTTTTGCTCAGGGGGAAGAACCAGGTTTGGTTGTGGGTGGCGGAG
TAGTATTGCAGTTTGCTGCCAGGCAGGGCGATTTCGGCGTTCACGCCCGTCAGGTAGCCG
CGCGTCGGCCATAACGCGCTGTCGGTTTTGTTGCGCCCCCAGCCGACGGTACCTTTGTAC
AGCCAGCCTTTGAAGCTGCCGTCTGTGCCGTCGGTTTTGCCGTATTTCTTGATAAAGTCG
GCATAGTGTTTGGGCGCTTTGTTGTAGGTGTTGACGGTCAGGTGTTCTGCCACCAAACCG
AAATTCACGCGGTCGTATTCGGTAACAGGCACGCTCATGCGGATGCCTGCGCCTGCCGTG
GTGGTTTTATATTGTTTGATGCTGGTCGATGCTTTGCGCGGGTCGAAGGCTTTTCCGTAA
ACATCGTAGCCCAGGCTGACCCCGTCTGCCGTGAAGTACGGGTCAGTAAACGACAGCGAG

## Appendix A

CCGTTAAGCGTGGTTTTGCTCCTGGAGGCGCGCAGTGCGGCCGACTTGCCCGTACCGAAC
AGGTTGTCTTGGGAAACGCCTGCGGACATGACCAACCCGGTATCTTGAACCCAACCCGCG
CTCAAATCCAGGGAACCGGTGGAACGTTCGGTCAGACTCATGTTCAAATCGACTTTGTCG
GGCGTGCCGGCAAGCGGGACAGCATCAAACTGGACATTGTCGAAGTAGCCCAAAAGCTCG
ACGCGCTCTTTGGAACGTTGCAGCTTGGAGGTGTCGTAAGGTGCGGATTCCATTTGGCGT
AATTCACGGCGGACGACTTCGTCGCGGGTTTTGTTGTTGCCGGTGATGTGTATTTCGTTG
ACGTAGATTTTCCGGCCCGGTTCGATGTGCAGGACGAAATCGACGGTTTTGGTTTCAGCG
TTCGGCAGCGGCTGTACGCTGATTTCGCTGTATGCGTAGCCTGCCGAGCCCATGCGGTTC
TGAATCTCACCCAAAACGGCGGTCATCTGCTGGCGTTCGTACCATTTGCCGGGCTTCATG
GTCAGCAGTTTTTCCAGTTCGGCTTTGGGGACTTCGTTGGTGTCGCCTTCGATGGAGACT
TTGCCCCAACGGAAACGTCCGCCTTCGTGGACGGTGATTTTGATGGTCTGCTTGGTTTTG
TCTTCGTTGGTTTGGATGTCGGTATCGAGGATACGGAAATCGAAGTAGCCGTTATTTTGG
TAGAAGTCGGTTACTTTTTCCATATCTTGGGCAAATTTCTGCTCGTTGAATTGGTTGCTT
CGTGTCAGCCATGTCCAAATGCCGCCTTCGGTCAGGGACATTTGCCGCATCAGTTTGCGG
TCGGAATAGACTTGGTTGCCTTCAAATTCGATGTCGGTGATTTTGGCGGATTTGCCCTCG
TCAATCGTGATGTCGATGTCGACGCGGTTGCGGGCGAGTTTGGTTACTTTGGGCGTGATT
TGGATATTGAGTTTGCCGCGCCCGAGGTATTCTTCTTTCAGGCCGGCGACTGCCTGATTG
AGTGTCGCCTGATTAAAGTATTGCGACTGCGCCAGCCCGAACGATTCGAGGTTTTTCTTA
ATGGCGTCGTTTTGCAGCATTTTTGCGCCGGTGATGTTGAGCGAGCCGATGGTGGGGCGT
TCGATAACGGTCAGCAGGAGCTGCCCGTCCGCAGTTTCGACGCGTACGTCGTCAAAGAAA
CCGGTGGCGTACAGGCTTTTGATGATGGCACTGCCGTGTGTGTCGTTGTAGGTGTCGCCG
ACTTTGACGGGCAGGTAGTTGAATACGGTACTCGGCTCGGTACGCTGCAAGCCTTCGACG
CGGATGTCTTGGATGGTGAAGTCGGCAAGTGCCAAAGGCGATATGCCCAACATCATCAGT
GCGGAAGCAATCTGTTTCAGTTTCATTGTCAGTTCCTTGTGGTGCGGAATGCGGTTTCAG
ACGGCATTCCGAAACGTAAAATCTAACCGAGCAGCCGGGTAACGTCGTTGAAGAAGGCGA
CCGCCATCATCAGCATCATGAGGGCGAGCCCGAAGCGCAAACCGATGTTTTGGACGCGTT
CGCCCAAAGGTTTGCCGCGTATCCATTCGGCAGTATAAAACACGAGGTGCCCGCCGTCCA
AAACAGGGACGGGCAGTAGGTTCAGCACGCCGAGGCTGATGCTGACCAGTGCTAAAAATT
CCAAATAACTTTGCAAGCCGAGTTCGGCGGACTGTCCGGCAATGTCGGCAATGGTCAGCG
GCCCGGAAATATGGCTGACGGAGGCGTTGCCGCTGATTAGTTTGCCGAAAAATTTGAGGG
TTGTCCACGAGTGGGAAACGGTTTTTCCCAGCCCATGCCGAATGCGCGGACAACAGACG
GACGGTAGCTGCGGCGGATTTGCGCGTCCCACGCCCTGTCCGGCTGCGGACGGAGGCCGA
CGCGCCCGATCAGGGTGTGGTCGGACTGTTCGACAGTATCGGGGCGGATGTCGGCGGTAT
GGGTTTGTCCGGCGCGTTCGTAGTTCAGGGTGATTTTTTTGCCGGGGCTTTGGCGGGTCA
GGTTTGCCCATTCTTGCCATGAGGCGATGGGTTTGCCGTCGGCGGCAGTCAGCCTGTCGC
CCGGTTTCAGGCCTGCTTTTTCGGCGGGGCTGCCTTTTTCCACGCCGCCGGCAACGGTTG
TGATTTTAAAGGGCATCAGTCCGATGTAGCCTTGGTTTTTTGCGATTTTACCGGCTTCCG
GCGTGCCTGCGGCATCGATGGTGCGGACGGTTTGCGCGCCCGATGCCGTCTGAACGCCGA
CGGCGACTTTGCCGGCTTCGAGGTTGAGGACGATTTCGGTTTGCGCGCTGCCCCAATCTG
CAACGGGTGTGCCGTTGACGGATTGTATTTTGTCGCCGCTTTGGAAGCCGGCGCGGGCGG
CAATGGTGTCGGGTTCGACTGTGCCGACGTAGGGGCGCAGTTCGGTTACGCCGAAGGAAA
AGCTCAGTCCGTACAGCAAAACCGCCAGTGCGAGGTTGGTCAGTGGGCCGGCGGCGACGA
TGGCGATGCGCTTGGCGGGGTGTTGTTTGTCAAAAGCGTAGGGTAAATCGGCTTCTGATA
CTTCGCCTTCGCGCGTATCGACCATTTTGACGTAACCGCCCAACGGAATCGGGGCGAGGC
ACCATTCGGTGTCGCCGCGCTTTCGGGTGAAAAACGGTTTGCCGAAGCCGACGGAAAAGC
GTACGACTTTGACGCCGCACAATCTGGCAACGATGTAGTGTCCGAACTCGTGCAGGCTGA
CCAAAATCAGGATGGCGAAGATAAAAGCTAGAAGGGTGTGCAAATGGTTTTCCTTTGATA
ACGGTGTTCAGATGGCATCAGCGCAGTGTGCCGATAAATGCTCGCGCTTGTGCGCGTGTC
CGGGGATGTTGGCGCAAGAGCCCCCCTATATCGCCTATGCCGTCTGAAAAGTCTTGTGCA
AGACAGTGGGCGACGGTTTTGGCAATGTCGGTAAACTTAATCTGTCCGTCCAAAAAGGCG
GCGACGGCGGCTTCGTTGGCGGCGTTCAATACGCAGGGCGCGGCTCCGCCTGCGTTCATG
GCTTCATAGGCGAGCCTCAGGCAGGGGAAGCGGTCAAAGTCGGGCTTTTGGAAGGTCAGC
GCGGACAATGCGTCGAAATCCAGGTCGCCGACACCCGAATCGATGCGCTCGGGCAAACCC
AAACAATAAGCGATGGGCGTTCGCATATCGGGATTGCCCAGTTGCGCCAGCACGGAGCCG
TCGCGGTAGCGCACCATGCTGTGTATCACGGATTGCGGATGGATGACGACTTCGAGTTTG
TCGGGCGGACAGTTGAACAGCCAATGCGCTTCAATCAGCTCCAAACCTTTGTTCATCATG
GTGGCGGAATCGACGGAGATTTTGCGTCCCATACGCCAATTGGGGTGTTTGACCGCTTGG
GCGGGCGTAATGCGGTCGAACGTGTTTAAATCGGCGGTCAGAAACGGGCCGCCGGAAGCG
GTCAGGATAATCGAAGCGATGCCGTGTTCGTTCAGACGGCCGGCGTAATCGCGCGGCAAA
ACTTGGAAAACGGCGTTGTGTTCGCTGTCGACGGGCAGCACTGCCGCGCCGTTTGCACGG
GCGGTTTCCATAAACAACGCGCCGGAAACCACCAGCGTTTCTTTGTTTGCCAGATAAATG
GTTTTGCCTTTTTGCGCCGCTGCGAGCGCGGAAGGCAGCCCCACCGCCCCGACGATGGCG
CACATGACACCGCTGACTTCGTCGGCAGAGGCAACGTCAACCAATGCCTGCGCGCCGTGT
AAAACCTGAGTCGCCGTGCCGTCGCGTTTCAACAGGGCTTCAAGCCGGGCGGCGTGTTCC
GCATCGGCAACGACGGCATATTCGGGGTGGAACGTTTGACATTGAGCCGCCAATTTCTCG
ACCTGCTTATGCCCTGCCAGCGCGAATACGCGGAATTTTTCGGGGTGGCGGGAGACAACG
TCCAGCGTGCTTTCGCCTATGCTGCCGGTACTGCCTAATATGGTCAGGACTTGTGGTGTC
ATAATGGGGATAACTTTATACCGGATGCCGTCTGAAGCGTTTTCAGACGGCATAGAATCA
ATTTAAAACCGACATCATCGCTGCATAGACGCTGATAACGGCAATCAGGCTGTCGGTACG
GTCGAACACGCCGCCGTGTCCGGGCAGCAGCTTGCTGCTGTCTTTGATGCCTGCCGCGCG
CTTGAGCCAGCTTTCCAAAAGGTCGCCGCATACGCTGACAACGGTCAGCACCAAACCGAT
TAACACGGTATCGAACCAGCCTGTATCGAATGCCAGCCAGCCGGCACTTCGTACGGCGGT
CATGTACACTGCCACGCAAACCGCGCCGCCGATTGCACCTTCCCAGCTTTTGCCGGGGCT
GATTGCCGGCGCGATTTTGTGTTTGCCGAACGCCTTGCCGCTGAAATACGCGCAAATATC
GGCAACCCACACCAAACCCATCACGGCGAGCAGCGGCAGGGCATCATCGGGATGCGGGCG

# Appendix A

CAGGGATACGAGCGCGAACCAAAACGGCATGACCAGAAGCCAGCCGACGGCATAAACCTG
CCAACCGCCGTTGAGCCTCCATTTGAATCTCAACCATAAAGGCATAACGGCGAGCCAAAA
TGCCAAAACAACATACCAAACCAAATTAGGCAGCATCCAGCCGCCCGCATAGGCAACCAC
GCCGAAAACCAAGGTTGCGGCGAGGTAATGGTTGGTTTTAATTTTGCACAAACCGCCCAT
ACGGGCATATTCCCACAAGGCAATCAGGGCAATCAGTCCGCAAAATGCAGCCCACAACCA
TTGCGGCGCGTAAAACAGCATGCCCAGCATCAGCGGCAGCAGCCACATGGCGGTTATTAC
CCGTTGTTTCAGCATATTCAGTTCCTTTGCTGTTCGATAGGCAGTTGCTCGGAGGTGCGT
CCGAACCGCCGTTCGCGTTTTTGGAACGAAGCGACGGCATCGTCCAAAGCCTTGCCGTCA
AAATCGGGCCACAAAATATCGGTGAAATACAGTTCTGCATATGCCATCTGCCAGAGCAGG
AAATTGCTGATGCGCGTTTCGCCGCCGGTGCGGATGAACAAATCCGGTTCCGGTGCATCG
CCCAGCATCAAGTGTTTCGCCAGCGTGTCTTCCGTAATCTCGGATACGCCTTCGGCAATC
AGTTTGTTTGCCGCCTGCAAAATATCCCAGCGGCCGCCGTAATCGGCGGCAATGCTCAGG
GTCAGGCCGGTATTGTTTGCCGTCAACGCTTCCGCCTCTTCGATGCCTTGCAGAATCTGC
CGGTTGAAGCGTTCGCGGCTGCCCAATATCTTCAGGCGCATATTGTTTTCGTGCAGGCGG
CGTACCTGTTTTTGCAAAGCCTGTAAAAACAGCCCCATCAGGAACGAAACTTCGTCTTCG
GGGCGGCGCCAGTTTTCGGTTGAAAAGGCAAACACGGTCAGATATTGCACACCCAGTTTG
GCGCAATGCTTCACCATATTTTCCAATGCGTCCAAACCGCGTTTGTGTCCCATTATGCGC
GGGAGGAAACGTTTTTTCGCCCAACGGCCGTTGCCGTCCATAATCACGGCGATATGCTTG
GGAATGGCGGTGTGTTCCAAAACGGCCTGCGTGCTGCTTTTCATGTCTGCCTTTCGCGGT
TCGGCATTCAAATGCCGTCTGAACGCCGAACCGTGCAGGTTAAATTGCCATCAAATCTTC
TTCTTTGGCAGTCAGGAGTTTGTCGGCTTCGGTAATGTATTTGTCGGTCAGTTTTTGAAC
CGCTTCTTCGCCGCGACGTGCCTCGTCTTCGGAAATTTCTTTGTCTTTGAGGAGTTTTTT
GATGTGGTCGTTGGCATCGCGGCGCACGTTGCGGATAGAGACGCGGCCTTCTTCCGCTTC
GCCGCGTACGACTTTAATCAGGTCTTTGCGGCGTTCCTCGGTCAGCATGGGCATCGGCAC
GCGGATCAGGTCGCCGACAGCTGCCGGGTTCAGTCCCAAGTTTGAATCGCGGATGGCTTT
CTCGACTTTGGCCGCCATATTGCCCTCAAACGGTTTCACGCCGATGGTGCGCGCGTCCAG
AAGCGTTACGTTGGCAACTTGGCTGACGGGGACCATGCTGCCCCAGTATTCGACTTCCAC
TTGGTCGAGCAGGCCGGTATGCGCGCGGCCGGTACGCACTTTCGCCAGATTTTCTTTCAG
TACTTCGACCGAACGCTGCATCTTGCCTTCGGCTGTTTTTTGAATATCGTTGATCATATT
GTTCTTTCGGTGGGATAAGGTGGGCGGGAGACCGTCTGAACGCGTTTCAAGCCGTTCAGA
CGGCATAAAGACCGTTAACCGCGAATAGTACCGTTATTCGGGCATAACGACAAGGTAGGC
GGATTGGGGATGCCGTCTGAAGCGACAGGCGTTTCAGACGGCATCGTGTCCGACCGTCAG
CCGTGTTCCCGTGTTTCAAGCAGGCTTTGGCGCAGGTGTTGGCGTTCGTGGGCATCCAGC
CATTTGCGGCGGGTGCGTTGCAGCAGGATGACGAGGGCGGAAATTTCCTGACGCATATTG
GTGCTGAGCCAGAGGAAGCCCTGCCATTGGTAGTGGAGGTGTTCGGCGAGGGCTTCCAGT
TCGGGGTTGATGGCGGTGTCGATGCGGATGCGGCGGGCGTGTCTGCCGTTGATAAGGGCG
ACGGTTTGTTGCAGGTCGGTTTGGAGCAGTGTGAAGTGGCGGTCAAGCAGCCGGATTTCG
CTGCCGTTGAGTTTGGGAGATTGCAGCTTGGCGGCGGTGGTCAGGAGCAGCTCGGTGGTG
TTGACGATTTTACGGTGGGCGTGCTGCATGGCTTCCATCATGGCGGGGCTGATGCGGCTT
TCGCCCGATGTGGCGGCGAGATGGCTGCGGCTTTTGACCATGCGTGCGTTGATTTGGCGC
ATTTTCGCCATGTTCTCCTCGAGGCGTTCGCGGGTCATGCGCCTGCCGTTGCTGATTTCG
GCAATCATTTTGCTGCAGTCGGCCAGGTTGTCGGCAAGCATGAAACGCCACATCAGTGTG
GATTTCAGCGGCAGCAGTTTGGCGGCGGCGATGGCGATGGCCGCGCCGATGAGGACGTTC
ATGGCGCGCATGAGTCCGCTGTCGAGCCATTCGCTGCCGTTGTCGCCGATGAGCATACAC
ATCGTCAGCCCTGCCAGCATAGGGACGTAGCCGTTTTTGCCGACCGCCGCCCAGCCGGCC
AGTGCGCTTGCCGTGCCGACGGTGAGGTAGAAGAGGAGGTTGCCGTGGAAATAATGCTGG
TTCAGCCATAAAACGCCCAAACCCGCGCCCAGCCCGATGACCGTGCCGAGCATACGTTCC
ACCGCCTTGGAGTAAATCGCCCCTTGAAACTGGAGCATGCCGAGGACGACGAAGACGGTC
ATCCGTATGCAGTCGCCGTGTTGGAGGTGGAGCAGCCGGGCGGAGGCGGTGGCGAACAGG
ACGGCCCCGCCGAGCCGGACGGCGTGGATGAGGCGGCGGTAGCGGTAGCGTTCGTAGGAG
TTGAGCCAGCGGCTGACGAGGCGGTTGCGTTGCGAGGTGTTCATATCGGTTGTGCCGTCT
GAAGCGGAAATGTGAAAAAGCACAGGCTTCCCGAGGAAGGGAGGGTCTGTGCTTGGTATT
GGTGCCGGAGAAGGGAATCGAACCCCCGACCTTCGCGTTACGAATGCGCTGCTCTACCGA
CTGAGCTACACCGGCGTTTTTTCGTCATGATATATATGAACGGTTGTTTGTGCAACTTTT
CGGGCGGGCGGCAAGGCAGTGCGCGGTATAGTGGATTAACAAAAACCAGTACGGCGTTGC
CTCGCCTTAGCTCAAAGAGAACGATTCTCTAAGGTGCTCAAGCACCAAGTGAATCGGTTC
CGTACTATTTGTACTGTCTGCGGCTTCGTCGCCTTGTCCTGATTTTTGTTAATCCGCTAT
ATAATGCGGTCTGCTTCGGAAGAGGGGGACGGCGATGTTTGTGAACGAGAAATATCCTTA
TGCGGCTCTGTTTGCGGGACTGGTGTTTTTGACGCTGCCGTTTGCGTTGGCGGTGCATGA
TGCCTTTGCGCTTGCGTTCGGACGGACGGGGTTGCTGGTGTCGGTGTCGGACGGCGGATT
CGGCTGGCGTGGCGGTTGGGACGGCACTGTTTGGTTTGTGTTCGGTGTGTTTGCGTTTTT
GAATGTGGTTGTGTCGGCGGGTCTGACGAAACTGGCGTACAAAAAGATGATGCGGCGGCA
TTCGCGTTACACACTGTTTCTGTCGGGCGTGGCGGCTTGCGCGGCGGCAGCGGTGGCTTG
GATTTTCGAGCTGCTGCTTGGCAGTGGGGCTTTGGGCGGGCTGCGGGGGAGGCGGTGTTG
GAATATGCGTTTGCCGTGTGGCTGGTGGCGATGCTGACGCTGCCCAAACGCCTGACGCGC
GCGCCGGTGCAGCCGGTGGTGTTTCACAGGAAAAAATAGGTTGGAACGGGAAATGCCGTC
TGAAACCCGACACGCGGTTTCAGACGGCATGTTTTTCCGCTAACATTACGCCTGAATATG
GACAGGAAGCAGATATGGAACGCAAAGAACGCCTGCGTGCAGGCATTGCCGCGATGGGGC
TGGATATTTCGGAAACGGCGCAGGACAGGCTTTTGGTCTATGTGGATTTGTTGAAAAAGT
GGAACAAAACCTACAATCTGACCGCCCTGCGCGACGAGGAAAAAATGATTGTCCATCATC
TTTTGGACAGCCTGACGCTGCTGCCCCATATCGAGGGTGTGCAAACGATGCTGGATGTCG
GTTCGGGCGGCGGTCAGCCCGGCATTCCGGCGGCGGTGTGCCGTCCGGATGTGCAAATAA
CCCTTTTGGATGCGAATACGAAGAAAACGGCTTTTTTACAGCAGGCGGTTATCGAGTTGG
GGTTGGACAATGTGCGCGTGGTATCCGGACGCGTGGAGGCGGTTTCGGACGTGCGTGCCG
ATGTGGTTACCAGCCGTGCGTTTGCAGAACTGGCGGATTTTGTGTCGTGGACGGTGCATC

## Appendix A

TGTTGAAAGACGGCGGCTACTGGGCGGCGATGAAGGGCGTGTATCCGCAGGAAGAAATCG
GCCGCCTGCCGCAGGATGTGTGCGTTGAAAAAGTCCAAAGGCTCGACGTGCCGGGCTTGG
ATGCGGAACGCCATATCGTCATCCTGAGCAAGCGTTGAGCGCACTTCAGACGGCATGAAT
ACCTTTTTTGTGCGGATAAAGGTAAAATTCCGCACTGTTTTTCTTTTTTCAACATCAGAC
GGGACACGGGCGGGACATGAGTGCGAACATCCTTGCCATCGCCAATCAGAAGGGCGGTGT
GGGCAAAACGACGACGACGGTAAATTTGGCGGCTTCGCTGGCATCGCGCGGCAAACGCGT
GCTGGTGGTCGATTTGGATCCGCAGGGCAATGCGACGACGGGCAGCGGCATCGACAAGGC
GGGTTTGCAGTCCGGCGTTTATCAGGTCTTATTGGGCGATGCGGACGTGCAGTCGGCGGC
GGTACGCAGCAAAGAGGGCGGATACGCTGTGTTGGGTGCGAACCGCGCGCTGGCCGGCGC
GGAAATCGAACTGGTGCAGGAAATCGCCCGGGAAGTGCGTTTGAAAAACGCGCTCAAGGC
AGTGGAAGAAGATTACGACTTTATCCTGATCGACTGCCCGCCTTCGCTGACGCTGTTGAC
GCTTAACGGGCTGGTGGCGGCGGGCGGCGTGATTGTGCCGATGTTGTGCGAATATTACGC
GCTGGAAGGGATTTCCGATTTGATTGCGACCGTGCGCAAAATCCGTCAGGCGGTCAATCC
CGATTTGGACATCACGGGCATCGTGCGCACGATGTACGACAGCCGCAGCAGGCTGGTTGC
CGAAGTCAGCGAACAGTTGCGCAGCCATTTCGGGGATTTGCTTTTTGAAACCGTCATCCC
GCGCAATATCCGCCTTGCGGAAGCGCCGAGCCACGGTATGCCGGTGATGGCTTACGACGC
GCAGGCAAAGGGTACCAAGGCGTATCTTGCCTTGGCGGACGAGCTGGCGGCGAGGGTGTC
GGGGAAATAGGTCAATCCAAATCGGGCTGCCCGTGCCTTTATGCTGTTTGGCCGGGTGCG
TTATAGTGGATTAACAAAAATCAGGACAAGGCGACGAAGCCGCAGACAGTGCAAATAGTA
CGGAACCGATTCACTTGGTGCTTCAGCACCTTAGAGAATCGTTCTCTTTGAGCTAAGGCG
AGGCAACGCCGTACTGGTTTTTGTTAATCCACTATAATATGGCGGATTAAAATAAAAATA
CTTATATCGTCATTTATCGTCATTCCCGCAAAAACAAAAAAATCAAAAACACAAAACTGA
AATATCGTCATTCCCGCGCAGGCGGGAATCTAGGTCTGTCGGTACGGAAACTTATCGGGA
AAAACGGTTTTTCCAACCCTGAGACTCCGGATTCCTGTTTTCGCGGGAATCCGGTTTTTT
GAGTTTCAGTCATTTTTGATAAATTCTTGCAGCTTTGAGTTTCTAGATTCCCGCTTTTGC
GGGAATGACGCGGAAAAGTTGCTGTGATTTCGGATAAATTTTCGTCACGCTTAATTTCTG
TTTTATCCGATAAATGCCTGCAATCTAAAATTTCGTCATTCCCGCAAAAACAAAAAATCA
AAACAGAAGCCTAAAATTTCGTCATTCCCGCGAAGGCGGGAATCTAGGTCTGTCGGTACG
GAAACTTATCGGGAAAAACGGTTTTTCCAAACCTGAGACTCCGGATTCCTGTTTTCGCGG
GAATCCGGTTTTTTGAGTTTCAGTCATTTTTGATAAATTCTTGCAGCTTTGAGTTTCTAG
ATTCCCGCTTTTGCGGGAATGACGCGGAAAAGTTGCTGTGATTTCGGATAAATTTTCGTC
ACGCTTAATTTCTGTTTTATCCGATAAATGCCTGCAATCTAAAATTTCGTCATTCCCGCG
AAGGCGGGAATCTAGGTCTGTCGGTACGGAAACTTATCGGGTAAAACGGTTTTGCCAGCC
CTGAGACTCCGGATTCCTGTTTTCGTAGGAATCCGGTTTTTTGAGCTTCAGTCATTTTTG
ATAAATTCTTGCAGCTTTGAGTTTCTAGATTCCCGCTTTCGCGGGAATGACGGTTTGGAA
GTTACCTGAAATTCAAAAAAAAAACGGAAACCGGACGGATTGGATTCCCGCCTGCGCGGG
AATGACGGATTTTAGGTTTTTTTTTTGATTTTCTATTTTTCGCGGGAATGACGGTTTGGG
TTCTTTCTCTTTGGAGTTGCGATGCCGGAAATGCCGTCTGAAGGCTTCAGACGGCATTTT
TGTGCCGGTTTAAAACAAGGCCTGCTGCGCGAGCAGGTTTCTGACGGGGGCGAAGTCGCG
GCGGTGTTCGGGCAGCACGCCGTATTTTTCGAGGGCTTCCAAATGCTGCTTCGTGCCGTA
ACCTTTGTGTTTGTCGAAACCGTATTGGGGATGGCGTTGCGCCAGTGCGTACATTTCCGC
ATCGCGTGCGGTCTTTGCCAAAACGGATGCGGCGGAGATTTCGATGATTTTGCTGTCGCC
TTTGACGACGGCTTCGGCAGGGATGTTCAAATGTTCAGGAATGCGGTTGCCGTCGATGAA
TATTTTTTCGGGACGCACAGCCAAGCCGTCAACGGCGCGTTTCATCGCGAGCATGGTGGC
GTGCAGGATGTTGAGGCTGGCGATTTCTTCGGGCGAGGCGGCGGCAACGTGCCACTCAAC
CGCCTGATTTTTTATCATTTCGGCAAGCGCGTCGCGTTTTTTCTCGCTGAGTTTTTTGGA
GTCGGTCAGTCCGGGCAGGTCGAATGTTTCCGGAAGGATGACGGCGGCGGCAAACACGCT
GCCGACTAAAGGTCCGCGTCCTGCCTCGTCCACGCCGGCGGTCAGTATGTGCATGATGTT
TGGTGTCGGGATGGTGGGAATGCCGTCTGAAAAGGGTTTCAGACGGCATCGCGCCGATGT
GTTTATTTCGCGTCTTTAAACCCGCGCTTCAAATGCACCATCAGCAATGCCACTGCCGCA
GGGGTTACGCCGGAAATGCGGCTGGCTTGTCCGACGGTTTCGGGTTTGTGCTGGTTGAGC
TTTTGCTGCACTTCTGCCGACAAGCCTTTGACTTTGCCGTAATCGATGCCGTCGGGCAGT
TTTAAGGTTTCGATGTCGCGGCGGCTGTCGATTTCTTCGTTTTGGCGGTCGATATAGCCT
TGGTATTTGACTTGGATTTCGACTTGTTCGATGACTTCGGCGGAGAGGTTTTCAGACGGC
ATCGCGCCTTCGAGCGTCATCAGCGCGGCGTAGTCGAGGTTTGGGCGGCGCAGGAGGTCG
TGCAGGTTGGCTTCGCGGCTGAGTTTTTGTCCGAACACACGGATTTGTTCGCCTTCGGCG
AGTTTTTGCGGCGTGTACCACGTTGTTTTCAAACGTTGGATTTCGCGTTCGACGGCTTCG
CGTTTTTCGTTGAACATGCGCCATTGCGCTTCGGACACCAAGCCGATTTTGTAGCCGTCT
TCGGTCAGGCGCATGTCGGCGTTGTCTTCCCTGAGTTGCAGGCGGTATTCGGCGCGGCTG
GTGAACATTCGGTAGGGTTCGTTCACGCCTTTGGTGATGAGGTCGTCCACCAATACGCCG
AGGTAGGCTTGTTCGCGGCGCAGCAGGAGCGGGTCTTGTCCGCGCACATATTGCACGGCG
TTCGCGCCTGCCAATAAACCTTGCGCGGCGGCTTCTTCGTAGCCGGTCGTACCGTTGATT
TGCCCGGCGAAAAACAATCCGGCAATGGTTTTGGTTTCGAGGCTTGCTTTGAGGTTGCGC
GGATCGAAGTAGTCGTATTCGATGGCGTAGCCGGGGCGCAGGATATGGGCGTTTTCCAAA
CCTTTCATACTGCGGACGAGCGCGATTTGGATGTCGAACGGCAGGCTGGTGGAGATACCG
TTAGGATAGTATTCGTGCGTGGTCAGACCTTCGGGTTCGAGGAAAATCTGGTGGCTGTCT
TTGTCGGCGAAGCGGTTGATTTTGTCTTCGATAGACGGACAATAACGCGGACCCACGCCT
TCGATTTTGCCGGTAAACATCGGGCTGCGGTCGAAGCCTGAGCGGATGATGTCGTGGGTT
TGCGTGTTGGTATGCGTAATCCAGCAGGACACTTGGCGCGGGTGCATATCGGCGTTGCCG
CGCACGGACATGACGGGAACGGGCGTGTCGCCGGGCTGTTCGGTCAGTTGGGAGAAGTCA
ATCGTGCGTCCGTCAATACGCGGCGGCGTGCCGGTTTTCAGACGGCCTTGCGGCAGCTTC
AATTCGCGCAAACGTCCGCCCAACGATTTGGCGGCGGGGTCGCCGGCGCGTCCGCCTTCG
TAGTTTTCCAAACCGATGTGGATTTTGCCGGACAAAAACGTGCCTGCGGTCAACACGACG
GCGCGTGCTTTAAACTCCACGCCCATCGCGGTAATTACGCCGCTGATGCGTTCGCCGTCG
AGCGTTACGTCTTCGACGGCTTGTTGGAAAAGGTCGAGGTTTTCTTGGTTTTCCAACATT

## Appendix A

TCGCGGATGGCGGCTTTGTACAGGATGCGGTCCGCCTGCGCGCGCGTGGCACGCACTGCC
GCGCCTTTGCTGGCGTTCAGGCGGCGGAACTGGATACCGGATTTGTCGGTTGCCAACGCC
ATCGCGCCGCCGAGCGCGTCGAGTTCGCGCACCAAATGCCCTTTGCCGATGCCGCCGATA
GAGGGGTTGCACGACATTTGTCCGAGCGTTTCGATATTGTGTGAGAGCAAAAGCGTCTGC
GCGCCCATACGGGCGGCGGCGAGTGCGGCTTCCGTGCCGGCGTGTCCGCCGCCGACGACG
ATAACGTCGTAGGTTTTGGGGTAAATCATGTGGGTCATAGTGTGTATTGCCTGACGGTGT
TTCAGACGGCATTTATAGTGGATTAACAAAAACCAGTACAGCGTTGCCTCGCCTTAGCTC
AAAGAGAACGATTCTCTAAGGTGCTGAAGCACCAAGTGAATCGGTTTCGTACTGCTTGTA
CTGTCTGCGGCTTCGTCGCCTTGTCCTGATTTTTGTTAAACCACTATATTCAATATGCCG
TCTGAAAAACGAAATGGATTCAAAAGTAAAGGGTTGGGATTGTACGCTTGTTCGCCCTGT
TTTTACAGTGTGCGGAAAGGGAAAAGCCGCTTCGCGGGGAAGCGGCTCCGGTAAGGGCGG
GATTTACCAAACGTCGGATTTGATACGGCGTTTCAGGCCCGGATGTTCGGAAAGTTTGAA
CTCGGGGTCTTTGCCCATTTTCAGCTTGGCGGTGTAATCGCGCAGCAGCATAAACGCCAA
GGGCGAGAGCAGCAGGATGGCGACAAGGTTGATCCACGCCATAATGCCCATCGCCATATC
CGCCATATCCCAGACCAAAGGCACATTGGCAACCGCGCCGAAATAGACCCACGCCAAAAC
CAGCATACGGAAAACGGCGGTAATCAGCCAATGGCTTTTGATGAATTGGACGTTGGACTC
GGCATAGGCATAGTTGCCGATAACGGTGGAAAAGGCAAACATAAACAGGATGACGGCGAG
GAAGCCCGCGCCCCATTGCCCCACTTGGCTGACAATCGCCGCCTGCGTCAGCGCCGCACC
GCTCAAATCGCCGTAAGGCTGTTGGTAAATCAAGATGATGAAGGCGGTGCAAGAACAAAC
GATGATGGTATCGACAAACACGCCCAGCATTTGAATCATACCTTGCGAAACAGGGTGTTT
CACTTCGGCGGCGGCGGCGGCGTTCGGCGCGGAACCCATACCCGCCTCGTTGGAATACAG
GCCGCGTTTGATGCCCATCATCATCGTTTGCGAAATCAGACCGCCGAGTAAGCCGCCTGC
TGCCGCGTCGAATTTGAACGCGCCCGAAAAAATCTGACCGAACACGTCCGGAATCATCGG
AATATTGGTCAAAATGATGAAAAGCGCGATAAAGAGGTACAAAACCGCCATCAGGGGGAC
GACGATTTCCGCCGCTTTAGATATGCGCCTGATGCCGCCGAAGATAATCGGCGCGGTTAA
AATCACCAGGGCGACGCCGACATAATGAGGCTCCCAACCCCATGCCGCTTTGACGGTATC
GGCGATGGTATTGGTCTGAACCGCTTCAAACACAAAGCCGAAACAGAAAATCAGGCTCAG
GGCGAACAACACGCCCAGCCATTTCTGCCCCAGCCCTTGAGTGATGTAGTAGGCAGGGCC
GCCCCGGAAATGGTGGTTGTCGTAGTCGCGGACTTTAAAGAGCTGCGCCAGCGAAGATTC
GACAAACGCCGAACTCATACCGATTAAGGCGGTTACCCACATCCAAAACACCGCGCCCGG
TCCGCCGACTTTGATGGCGATGGCCACGCCCGCGATATTGCCCACGCCCACGCGGCTGGC
AAGGCCGGTTACAAATGCCTGAAACGGCGTGATGCCGTGAGGGTCGTCCCCCTGTTTGCG
GCCGCCGAGCATTTCTTTGATGCTGCGCCCGAACAGGCGGAATTGGACAAAGCCCGTGGT
TACGGTGAAGAAAAGCCCCGTACCCAAAAGCATATAAACCAAGTATGACCACATCGGATC
GTTGATGGCGCCGACCCAGCCGTGCAGCCATTCGGTAAAGTTCTCGTTCATATCGCTTCC
TTAAAGTTGAAACTCGCACATATTGGCGGTATGCAAGCAGGGTTTAAATTTTGTAAACGC
CCATTCTAGCAGATTGTCAACAAAATCAGAAAAATTTACATCGCCGCGCGGCTGCGGCGT
TAGAATCGCATTTTGTTTGGAGCAAACACGATGAAACAGCCTGTTTTTGCCGTTACTTCC
GGCGAGCCTGCCGGCATCGGCCCCGATATTTGTTTGGACTTGGCGTTTGCACGCCTGCCC
TGCCGCTGCGCGGTATTGGGCGACAAAAACCTATTGCGCGCGCGCGCCGAAGCCTTGGGC
AAAAGCGTCGTCCTGCGCGACTTCGATCCAGAATCAGGCGGCGCGGCATACGGCGAGCTG
GAAGTGCTGCACATCCCTGCCGTCGAAGCGGTTGAGGCGGGCAAACTCAATCCCGCCAAC
GCCGCCTATGTGCTGCAACTTTTGGACACCGCGCTCGCAGGCATTTCAGACGGCATTTTC
GACGGCATCGTTACCGCGCCGCTGCACAAAGGCATCATCAACGACGCGCGCGCAAGCACA
GGTTTTTTCAGCGGACACACCGAATATCTGGCGGAAAAAAGCGGCACGGGGCAGGTCGTG
ATGATGCTTGCCGGCAAAGGCCTGCGCGTCGCCCTCGTAACGACCCACCTGCCGCTGAAA
GACGTTGCCGCCGCCATCACGCAACCGCTGATTGAAAGCGTCGCACGCATTTTGCATCAC
GACTTAAAACACAAATTCGGCATCAAAAATCCCAAAATCCTTGTCGCCGGACTTAATCCC
CACGCCGGCGAAGGCGGACACCTCGGACACGAAGAAACCGACACCATTATCCCTGCATTG
GAAAACCTGCGCCGCGAAGGGATAAACCTTGCCGGCCCGTATCCGGCGGACACATTGTTC
CAGCCGTTTATGCTCGAAGGTGCGGATGCCGTATTGGCGATGTACCACGACCAAGGGCTG
CCCGTGTTGAAATACCACAGCTTCGGACAGGGCGTGAACATCACGCTCGGCCTGCCCTTT
ATCCGCACCTCCGTCGATCACGGCACCGCGCTTGATTTGGCGGCAACCGGCAGGGCGGAT
TCCGGCAGCCTGATAACTGCCGTGGAGACCGCCGTCGAGATGGCGCGCGGCAGCCTTTAA
AGATGATAAAAGACCCGTCATTTCCGCGCAGGCGGGAATCCGGTCTGTTCGGTTTCAGTT
GTTTTTGGGTTTCGGGTAATTTCCAAATCGTCATTCCCGCGCAGGCGGGAATCCAGACCA
TTGGACAGCGGCAATATTCAAAGATTATCCGAAAGTTTGAGGTTCTAGATTCCCGTTTTC
ACGGGAATGACGAAAGGTGGCGGGAATCCGGTCTGTTCGGTTTCGGTTTTTTTTTTTGAG
GTTTCGGGCAACTTCTAAACCGTCATTCCCGCGCAGGCGGGAATCCAGACCATTGGACAG
CGGCAATATTCAAAGATTATCTGAAAGTTTGAGGTTCTAGATTCCCGTTTTCACGGGAAT
GACGGAATGTTGCGGGAATCCGGCTTGTTCGGTTTCGGTTTTTTTGAGGTTTCGGGCAAC
TTCTAAACCGTCATTCCCGCGCAGGCGGGAATCCAGACCATTGGACAGCGGCAATATTCA
AAGATTATCTGAAAGTTTAGAGGTTCTAGATTCCCGTTTTCACGGGAATGACGGAATGTT
GCGGGAATCCGGCTTGTTCGGTTTCGGTTTTTTTTGAGGTTTCGGGCAACTTCTAAACCG
TCATTCCCGCGCAGGCGGGAATCCAGGCCTTTGGGCGACGGCAATATTCAAAGATTATCT
GAAAGTTTAGAGGTTCTAGATTCCCGTTTTCACGGAAATGACGAAATGTTGTGGGAATCC
AGACCTTCGGGCAGCGGCAATATTCAAAGGTTATCTGAAAGTTTGAGGTTCTAGATTCCC
GTTTTCACGGGAATGACGAAAGGTTGTGGGAATCCAGACCTTCGGGCAGCGGCAATATTC
AAAGATTATCCGAAAGTTTGAGGTTCTAGATTCCCGTTTTCACGGGAATGACGAAAGGTG
GCGGGAATGACGAAAGGTTGCGGTAATCATGGGAATGGCGAAGTTTCAGACGGCATCGTC
CACCCTCCGCCGTCATTCCCGCGCAGGCGGGAATCCAGGCCTTTGGGCGACGGCAATATT
CAAAGATTATCCGAAAGTTTGAGGTTCTAGATTCCCGTTTTCACGGGAATGACGGAATGT
TGCGGGAATCATGGGAATGACGGAATGTTGCGGGAATCATGGGAATGACGGAATGTTGCG
GGAATCATGGGAATGACGGAATGTTGCGGGAATCATGGGAATGGCGGAATGTTTCGGTAA
TCACGGGAATGGCGAAGTTTCAGACGGCATTGCAGGTATCCGAACCCATGTAAAAAAGAG

## Appendix A

GTTCTGCGGAACAGAACCTCTTTTTGCCGCCGTCGGTTCAGCCTTGCCGGGTTTCGACTT
GGATCATTTCTTCGGCAGGGACGGTTGCGACTTCAGACGGCTTGGGCTGTTCGGAACGGC
GCAAACCGCGTCCGGCTTGGACTTCGGGTTGTGCCGCCCATGCCTTCAATGCGGCAGGGT
CGGTTTCGATCAGGACGAGTCCGCCGGTTTGTGCGGTTTCCCGTGCCTGTTCCGCCGCAG
CCGTAAAGGTTGCGGTTTCAGACGGCATTTCCTGTGCTTCGGCTTTCGGTGTCGCGCCTT
CGGGCAGGATGGCGGCGGTGGCACGGCGGATTTTTTCCGCCGCATCATAAACCGGTGCGT
CGCCGTTTGAAACGGCGGGAGATGCTGTCGGAAGATCCCTTTCTGCAACCGGATCGGCAA
TGCTGACAGTAATCGGCGCGTTTGCGTCGGTTTCGCCGAAAACGTGCGCGGCGGCGGAAC
GGACTTTGTCGGCGGTGTCGTGAATATTCAGGTACTGCTCGATTTTGGCGGCAGACGGAA
TATTGCGTTTTTTGCCGTTTTGACGGCGGTCGCGCTGATTGTTGCGCTCGCGGCGTTCTT
TGGCATCTCGGCTGTCGCGTTCGCGGCGGTTGCGTTCGGATTTGGGCTTGCTGCCTTTGT
CTTCTGCGGTATGCGGTTCGGACGGCGTGTTTTCCGCTGTCTGAACGGTTGTTTCGGCAA
CGGTGGCGGTTTCCGGCGCGGTTTGGCCGCGTTCGCTGCGGCTGCCGTTGCGGCGGCGTT
TTCCGGTTTGCACTTCGGTTTCGGACGGTGCGGCATCTGCAACGGTTGCGGCAGGCTGTA
CGTTGCGGCTTTGGATTTCCGCTTCGTTGGCGCGTTCGGCGGCACGGTCGCCGCGTTCAT
TGCGGCGGCGGTTGCCGTTGTTGCGCGTTTCGGCTTTGTCGGCACGCGCTTCCTGTCCGG
CAGTTTTGCCTGCCACTTCGCGGACTTCTACTTTGCTGCCTTCGCGTTTGCTGCGGCGCG
GGTTTTGGCGGCGGTTGTTGGCGCGGCTGCCGCTGCGGTTTGCCGTGCTGCGTTTTTCGG
AGGTTTCGGCAGCGGGCGCGGCTTGGGTTTCGCTGCCGCCGAAAATGCGTTTGAGCCATG
CTTTGAAGCTGTCCCACCAAGAGGTTTTTTTCTCGGGGGCGGCAGTCGGGGCGGGGCTGG
TGTGGCGCACGCCTTTGACGGCGGGTTCGGGACGGGCGGCTTTGGCTTTTTCGCCGCCGA
ACGGTTTGGCGGATTCGTCTTCTTCCGGCTCGGCGACGCGTTTGTAGCTCGGTTCGCCGT
CTTCTTCTACGTCGTCGGTGCGGATGCGGTTGATTTCGTAGTGCGGATTTTCGAGGTGGA
TGTTCGGAATCAGGACGACGTTGACATCCAAACGCTCTTCCATCGCAAACAGCTCGGCGC
GTTTTTCGTTCAGCAGGAAGGTGGCGACATCGACGGGCACTTGTGCGCGCACTTCTCCGG
TGTTGTCCTTCATCGCTTCTTCTTGAATGATGCGTAAAACGTGCAGGGCGGTGGATTCGA
TGCCCCGAATCACGCCGGTGCCGGCGCAGCGCGGACAGGCGACGTGGCTGCTTTCGCCCA
AAGCCGGTTTCAAACGTTGGCGGCTCAATTCTAAAAGTCCGAAACGGGAGAGTTTGCCCA
TCTGCACGCGGGCGCGGTCTTTTTTGAGCGCGTCGCGCAGGACGTTTTCCACATCGCGCT
GGTGTTTGGGGTTTTCCATGTCGATGAAGTCGATGACGACCAAGCCGCCCAAGTCGCGCA
GGCGCATTTGTCGGGCGACTTCTTCGGCGGCTTCCATATTGGTTTTGAACGCGGTGTCTT
CAATGTCTGCGCCGCGAGTGGCGCGTGCGGAGTTCACGTCGATGGAGACGAGGGCTTCGG
TATGGTCGATGACGATCGCGCCGCCGGAGGGCAGGCTGACGCTGCGCGAAAACGCGCTTT
CGATTTGGTGTTCGATTTGGAAGCGGGAAAACAGCGGCGTGTGGTCTTCGTAGAGTTTCA
GACGGCCTATATTGCCCGGCATGACGTAGCTCATGAACTCGGCAACTTGGTCGTAAACTT
CTTGATTGTCCACCAAAATCTCGCCGATGTCGGGGCGGAAATAGTCGCGGATGGCTCGGA
TCAGCAGCGAGCTTTCCATAAAGAGCAGGTAGGGGTCGTGATGCGCTTTTCCTGCTTCTT
CAATCGCCTGCCAGAGTTGTTTGAGGTAGTTCAAGTCCCATTCCAACTCTTCCGCGCTGC
GGCCGATGCCGGCGGTACGGGCGATGATGCTCATGCCGTTCGGAATGTCGAGTTCCGCCA
TGGCGGCTTTCAACTCTTGACGCTCTTCACCTTCGATACGGCGGGATACGCCGCCGCCGC
GCGGGTTGTTCGGCATCAATACCAGATAGCGTCCGGCGAGGCTGATGAAGGTGGTCAGCG
CGGCGCCTTTGTTGCCGCGCTCGTCTTTTTCGACTTGGACGATGACTTCCATGCCTTCTT
TGAGCACGTCTTGGATGCGCGCGCGTCCGCCTTCGTAGTCTTGGAAGTATGAGCGGGAGA
CTTCTTTAAACGGCAAGAAGCCGTGGCGGTCGGTTCCGTAATCCACGAAACACGCTTCCA
GCGACGGCTCGATGCGGGTAATGATGCCTTTGTAGATATTGCCTTTGCGCTGTTCTTTGC
CCAGCGTTTCGATGTCCAAATCCAGCAGGTTTTGTCCGTCGACGATGGCAACGCGCAGCT
CTTCGGCCTGCGTTGCGTTAAATAACATTCTTTTCATGATCACCTCGTGGGCAGGCGGCG
TTCAGACGGCACATGCCCGGTTCGGCATTCCGTAAGGCTGGGTTTTCCGATGTTTTCGGA
TAAAACCGGTAATCAGTTTTTGAGTTGAAAATCCGCAGGGATGCACGTTCCGGAGAACCG
TGTGCGGAAGGGTCGGATACAGAAGGCTATAAAGATCGATGCGGCGGTTTGTCTGCCGCG
TTCCGAACGCTGCGGTCGGAAAAATGGGGGCCGGCTTCTTCTTGTTATCGTGATGCCTGT
GTTTTGGGCGGTTTGCGTTTGGGACTTGGGCCCGGCTGCCGTCTTACTTCCGCGCCGAAA
CGGCAAAATCAATTCAAACTTGATTACGTTCTGCGCCTGCCGGCTGGGAACAGGCGCAGG
GAAAATGCTTTGCGGAGTGCGTTTTTAATATAAAATTCCGTTTTAAAGTAAACCGTTTCA
GGAGGCGCGGCGGGCGCGCTTTTTGCTGAAACGGATGTTCGGATTATAGATGAAAACGCA
CGAAATAAGCAAAGATTCGGTCAGCTTGATAGGGGTTGCCGAACATGAGGCGGGTCAACG
CCTTGATAACTATCTGATAAAAATCCTCAAGGGTGTTCCCAAGAGCCATATCCACCGCAT
TATCCGCGCCGGCGAGGTGCGGTTGAACAAGAAACGCTGCAAACCCGACAGCCGTATTGC
GGAGGGGGATACGGTGCGGATTCCGCCTGTGCGCGTGGCGGAGAAGGAAATGCCGTCTGA
AAGGCGTGCCGCCGTACCGGCGCGTGCGTTTGACGTTGTTTACGAAGACGATGCGCTTTT
GGTCATCGACAAACCGTCCGGCGTTGCCGTCCACGGCGGCAGCGGCGTGAGTTTCGGCGT
TATCGAACAGTTGCGCCGCGCCCGTCCGGAGGCGAAGTATTTGGAGTTGGTTCATCGTTT
GGACAAGGATACGAGCGGCTTGTTGATGGTGGCGAAGAAACGCAGCGCGCTCGTCAAACT
TCACGAAGCCATCCGTAACGACCACCCCAAAAAAATCTACCTTGCGCTGGGGGTGGGCAA
ACTGCCGGACGACAATTTCCATGTCAAACTGCCCCTGTTCAAATATACCGGCGCACAAGG
CGAAAAGATGGTGCGCGTCAGTGCGGACGGGCAGTCGGCGCATACGGTGTTCCGTGTGTT
AAGCCGTTTTTCAGACGGCATTTTGCACGGTGTCGGGCTGTCGCACCTGACTTTGGTGCG
GGCGACGTTGAAAACGGGGCGCACGCACCAAATCCGCGTCCACCTGCAATCTCAAGGCTG
TCCGATTGCGGGCGACGAACGCTACGGCGATTATCAGGCGAACCGTCGTTTGCAGAAGTT
GGGTTTGAAGCGGATGTTTTTGCACGCGTCCGAGCTGCACTTGAACCATCCGCTCACGGG
CGAGCCGCTGGTGTTGAAGGCGGAGCTGCCGCCGGACTTGGCGCAGTTTGCGGTGATGTT
GGAAAACGGACGAAAATGTGAACCCCGATGCCGTCTGAAGCCTTCAGACGGCATCGGGA
CGTGAAAGTATGTGGGGACAGACGAATATGGCTGATAAAAAAAGCCCTTTGATTGCCGTC
AGTGTCGGCGAAGCGTCGGGCGACCTATTGGGGGCGCACCTGATACGCGCCATCCGCAAG
CGTTGTCCGCAGGCGCGGTTTACCGGTATCGGCGGCGAACTGATGAAGGCGGAAGGTTTC

## Appendix A

GAGAGCCTTTATGATCAGGAGCGGCTGGCGGTGCGCGGCTTTGTCGAAGTGGTCAGGCGG
CTGCCGGAAATTTTACGGATACGCAGGGGGCTGGTACGGGATTTGCTGTCGTTGAAACCT
GATGTCTTTGTCGGTATCGATGCGCCCGATTTTAATTTGGGTGTGGCGGAAAAGCTGAAA
CGGTCGGGGATTCCGACCGTGCATTATGTCAGCCCGTCGGTGTGGGCGTGGCGGCGGGAA
CGTGTGGGCAAAATCGTGCATCAGGTCAACCGCGTGTTGTGCCTGTTCCCGATGGAGCCG
CAGCTTTATCTCGATGCGGGCGGACGTGCGGAGTTTGTCGGTCATCCGATGGCGCAGCTT
ATGCCCTTGGAAGACGACCGTGAAACGGCGCGGCAAACTTTGGGCGTGGATGCCGGCATC
CCCGTATTCGCCCTGCTGCCCGGCAGCCGCGTCAGCGAAATCGACTATATGGCGCCGGTG
TTTTTTCAGACGGCATTATTGTTGTTGGAACGCTATCCCGCCGCACGCTTCCTGCTGCCT
GCCGCAACGGAGGCGACGAAGCGGCGTTTGGCGGAAGTTTTGCAGCGGCCGGAGTTTGCC
GGATTGCCGCTGACGGTAATCGACAGACAGTCTGAAACAGTGTGCAGGGCGGCGGATGCG
GTGCTGGTAACGAGCGGTACGGCAACTTTGGAGGTGGCGTTGTGTAAGCGTCCGATGGTC
ATCAGCTACAAGATTTCGCCGCTGACCTATGCTTATGTGAAACGCAAAATCAAAGTGCCG
CATGTCGGCCTGCCGAATATCCTGTTGGGTAAGGAGGCTGTGCCGGAATTATTGCAATCT
GAAGCAAAACCGGAAAAACTGGCGGCGGCGTTGGCGGACTGGTACGAACACCCCGATAAG
GTTGCCGCGCTGCAACAGGATTTCAGGGCGTTGCACCTGCTGTTGAAAAAAGATACGGCG
GATTTGGCCGCGCGCGCGGTTTTGGAAGAGGCGGGATGTTGAGCGGTTAATGGATTATTT
TCCCGAAGCAGCACGTATTACAAAAAAAGGGGGAGAAATTGTGATTAATGGCACATCAAA
CAATAAGTATTTAAGAGGAATTCCAAATGAAACAGAACTGGCCCGAATGGGATTAAGGTT
AAAATATAATGGTCAGTTAACTGATTAATTTTGTTATATATGATTTATGATTATAGCTTA
TACTAATACGCTTACTTACCTTGTTTCATTTGTTCTTCGTAAATTTCTATTTTAGGCAAT
TGTGTCAGTTCAATAGGGCAAGTTGCTCCCCACCAAAAATGTTCTACATAAAACCAAGGA
TTATCTGGAAAATATAGCAACATCTCTTCCATATCCGGCCAAATTCTTCTTAATTCATCT
ACCTGTGTTTTTGGCGAACCAGTTAATATTTTTGGAGGATTTTCACGATAATCGCATAAT
TCAATAACACCATCTGATAAAAGTTCTTCCAAAAAATCAAAAAATCTAATTTTTAAATTT
GGATCTTTGATATCCATATTTAAATAATTTTTATAAACACCAAAAATACCACCTAAATAT
TCACAATATTCTAAAAGATTATATTTTATCTTCACATTCATAACGTAACCTTTATCTAAA
TTTTAATTCTAATCTTTGCCCATGTACTGAATCAGGTTGATTCCTAAACTCAATCGTCCA
TTTTGCTCCAGTTTGTTCTCGGCTAGTTGAAAAATTCCTTAAAATAAAGGAAGAGTTTAA
ACAACTGAAATTTCATAAGAGTAGTAGAACCAACTTGGACTCAAAAAATCTTAAACTCAT
TGTTTTTGAAAAGGTAAAATAATATGACAACTTATACCATTCCAAAAAAAGATTATCAAT
TTCTGTATATATATATGAGGGCACTCTATTAAACTATACTTTGAAAAACGATGAATTCCATA
TCATCGTCCAGAATGTGGATTATCCGGACTTTCCTCAAGAGATTCCTACACCAAATTATA
CAGACTGGGTAAAAATTAAATTCAAGCAGTTCAGCTATCTGAAATTTATCTATGGATACG
CCACGAAGAACCAAGATAAAAATATCAAAAATGTATTGGAACTTGGAGAATTAAAGCAGG
ATGATGAAATCTTGGATTATGGAGGTGCGCTGGAAGTGATAGGCAGTAGGTATGATCTTC
CGACCGGTTTTAGTATAGATATAGTTTGCCGGGAAATAGAGTTAGAATTTTTAGATCAGG
AGAGTTTCAATTAAACGAGCCGTAGCTTGTTATGCTGAGCAGGCAACTTTATCGTATTTC
CTTTTCGGTTGAAACCCCGCCACTCGGACATCTGTCCTTCGGGGCGGTAGAATCAGATTT
TATTTGGGAGGGGCGTAACCCCTTCCGAATCAGGGCAACACATAGGGCGACGCTTTATGT
GTCGTCCTGTGTGTTGAAACATTGATATGCCGATACGGAGCCTGTCGGCAAAATGCCGTC
TGAACAATATCTTTTCAGACGGCATTTTGTATGGGGGTTAACGGTTGTTCAGCCCGAGTA
CGTCCTGCATATCGTACAAACCCGTTTTGCCGTTGACCCAAACTGCGGCGCGGACGGCAC
CGGCGGCAAAGGTCATGCGGCTGCTGGCCTTGTGGGTGATTTCCACGCGCTCGCCGTCGG
TGGCGAAGAGGGCGGTGTGGTCGCCGACGATGTCGCCTGCGCGGACGGTGGCAAAGCCGA
TGGTCGACGGATCGCGCGGACCGGTGTGGCCTTCGCGGCCGTAAACGGCGCATTGTTTGA
GGTCTCTGCCGAGCGCGCCGGCGATGACTTCGCCCATGCGTAACGCGGTGCCGCTGGGGG
CATCGACTTTGTGGCGGTGGTGGCCTTCAATGATTTCGATGTCGTAGCCTTCGTTTAATA
CGCGTGCGACGGTGTCGAGGATGTGGAAGGTGAGGTTGACGCCGACGCTGAAGTTGGCGG
CGAAAACGATGCCTGTTTTTTCGGCGGCAGTGTGGATAGCGGCTTTGCCCGTATCGTCGA
AGCCTGTTGTGCCGATGATGATGTTGACTTGTTTTTCAACGCATTTTTGCAGGTGTTTGA
GGGTGGGCTCGGGGCGGGTGAAGTCGATGAGTACGTCGCTTTGTGCGAGAACGGCGTCAA
CGTCGTCTGAAATGGCGATGCCGGTTTTGAGTCCGACGGCGTAGCCTGCGTCCAGCCCGA
GGGCTTCTGAGCCTGAGTGTTCAAGCGCACCGGAAAGGACGGTGTCGGGATGGTTGTTGA
CGGCTTCAACCAATACGCGTCCCATACGGCCGTTTGCGCCGGCGATGGCGATTTTGAGCG
GTGTCATGTGTGTTCCTTATGGTTTGTCTGTGTTTTGGCGGTCTTTGAGGGCTTCGGCAG
CGTTTTGCAGGACGTCGCCTTCGGTGCGGACGAGTACGCCGTTTTCAAAATAGACGGTCA
GATTGCTGCGTTCTTTGATGATGCCGTTGCGGGAGGTGTTGAAGGTATAGTCCCAGCGGT
CGGTATGGAATGCGTCGCGCAGTATGGGGCTGCCGAGCAGGAGCAGGACTTGGTCTTTGG
TCATGCCGGGGCGGAGGGCGGCAACGGCGCGCGGTTCGAGTTCGTTGCCCTGTATGATTT
TGAGTTTGTACGAGGGGAACAGTGAAACGCGTTCGGCACTGCACGCGGCAAGGCCGAGGA
GGGCGGAAAGGGCGAGGATGAGGGTTTTGTTCACGGAAATGCCTTTCTGTGCAAATCGGG
ATGGGTAGTGTAACACTGCTTGAATATTTTATAAAAGCGAACGATAATCATACGATTAAG
CGGTATCCGCCCTGTCCGCGCATCGGCCGCCGGTGCGGTTTTACTATTGCAAACTGCTAT
GGTGCGATAGTGGGCAAACAGGCCGAAATTGCGTATTATAACGTCTATTGTTTTACAGGG
GTATTGAATATTATGGAAAAATTCAACAATATTGCACAACTGAAAGACAGCGGTCTGAAG
GTTACCGGCCCGCGTTTGAAGATTTTGGATTTGTTCGAGACGCATGCGGAAGAGCATTTG
AGTGCGGAAGATGTGTACCGCATTTTGTTGGAAGAGGGTGTGGAAATCGGTGTGGCGACG
ATTTACCGTGTGCTGACCCAGTTTGAGCAGGCGGGCATTTTGCAACGCCATCATTTTGAA
ACGGGCAAGGCGGTTTATGAGTTGGACAAAGGCGACCACCATGACCACATCGTCTGCGTG
AAGTGCGGCGAGGTAACGGAATTCCACAATCCCGAAATCGAAGCCCTGCAAGACAAAATC
GCGGAAGAAAACGGCTACCGCATCGTCGATCACGCGCTTTATATGTACGGCGTGTGCAGC
GACTGTCAGGCCAAGGGCAAACGTTAAATCCGGACGGTTTGTTGTTCAGACGGCATTCAT
GATTTTGGATGCCGCCTGTGTTTTTGGAGAACTGTCATGCGTATTCCGCTGCTTGCCCCT
GACAATTATGCCTTTCCCGATCCTGCCTATGCTTTGGCCCGGTGCGACGGGCTGGTCGGC

## Appendix A

GTGAGCGGCGATTTGGATGCGGGGCGGCTGCTTGAGGCGTATCGGAACGGCGTGTTTCCG
TGGTTTTCCCGGGACGGGTGGTTTTTTTGGTATGCGGTCGGGCCCCGTGCGGTGGTGTTT
CCCGACAGGCTGCATATTCCGCGCTCGCTGGCGAAAACGCTGCGCAACGGCAGCTATCGG
GTTGCGGTCAACGGCTGTTTTGCGGAAGTGGTCGCGCATTGTGCGGCAGCGGCGCGCCCG
AATCAGGACGGAACTTGGATTGCGCCCGAGTTTCAGACGGCATATTTGAAGCTGCACGAA
ATGGGGTACGCGCATTCTTTCGAGTGCCATTATCCCGATGAAAGCGGTGAAACGAGGTTG
GCGGGCGGCTTTTACGGCGTTCAGATCGGCAGGGTGTTTTATGGCGAATCGATGTTCGCA
TTACAACCGGATGCGTCGAAAATCGCGTTTGCCTGCGCCGTGCCGTTTTTGGCGGATTTG
GGCGTGGAACTGATAGACTGCCAGCAGGATACGGAACATATGCGCCGTTTCGGTTCGGAG
CTGCTGCCGTTTGCGGATTTTGCCGAACGTCTGCGGATGTTGAACGCCGTGCCGTTGAAA
GAGGAAATCGGGCGGCGCGAAGTGGCGTGCAAGGGGCTTTGATGGCGGCTTATGCTCCGG
TCAGGTTCAAATATGGTGGATTATAGTGGATTAACAAAAATCAGGACAAGGCGACGAAGC
CGCAGACAGTACAAATAGTACGGCAAGGCGAGGCAACGCCGTACTGGTTTTTGTTAATCC
ACTATAAAATTAGAAATGACGACAGCCGGATAAAATCACGGTGAAAATGAAAAATGCCGT
CTGAAACTTGAAAACATCGGGTTTCAGATGGCATTTTGTTTGACGGTTTGTTGCTTATTT
GAGCGGGCGCACTTCAAGTCCGAACATACGGCGTGCGGTGTTCAGCATTTGGCAGCTGAA
GCCCCATTCGTTGTCATACCAAGCGAACACTTTGACCATGTTGCCGTCAACGACTTTGGT
CAGTGTTGCGTCGAAGTGGCTGGCTTCGGTAGTGTGGTTGAAGTCCATGGAAACCAAGGG
CAGGGTGTTGTAGCCCAAAACGCCTTTGAGCGGGCCTGCTTCCGAGGCGGCTTTCATCAG
TGCGTTGATTTCTTCGACTGTGGTGTCGCGCGCGGCTTGGAAGCTCAAATCTACCAATGA
TACGTTGACGGTCGGCACGCGGATGGCAAGCCCGTCGAGCCTGCCTTTCAATTCGGGCAG
TACCAAACCGACGGCTTTTGCCGCGCCGGTTTTGGTCGGAATCATGTTTTCCACGCCGCT
GCGGGCGCGGCGCAGGTCTTTGTGGCGCACGTCGGTAACGGTTTGGTCGTTGGTCAGCGC
GTGGATGGTGGTCATCGCGCCTTTGACGATGCCGACGCTTTCGCTCAACACTTTGGCAAC
CGGCGAGAGGCAGTTGGTGGTGCAGGAAGCGTTGGAAACGACGGTCATGTCGGCGGTCAG
GACGCTGTCGTTCACGCCGTACACGACGGTTGCATCGACATCGTCGCCGCCCGGTGCGGA
AATGAGGACTTTTTTCGCGCCGCTTTCGAGGTGGATTTTGGCTTTTTCTTTGCTGGTGAA
CGCGCCGGTGCATTCCATGACCAAATCGACACCGAGTTCTTTCCACGGCAGTTCGGCAGG
GTTGCGGGTCGAGAAGAAGGGGATTTTGTCGCCGTTGACGATGAGGTTGCCGCCGTCGTG
GGATACGTCGGCTTCAAAGCGTCCGTGCACGGTGTCGAATTTGGTCAGATGGGCGTTGGT
TTCAAGGCTGCCGCTGGCGTTGACGGCGACGATTTGGAGTTGGTCTTGAATCTGATAATC
GTAGATGGCGCGCAAAACCTGGCGGCCGATGCGTCCGTAGCCGTTGATGGCGACTTTGAT
GCCCATGGTTTGTTCCTTTGTTGAGGGTTGGGTAGATTTTCGGGGCGGATTATAGCAAAT
TTGTAGTGGCGTGTAATTAATATTTTATTGAAAACGGCGCGGCCGGAAGGGTGGGCGGTA
AGATGCGGACGGCACGGGTGCGGCGGACGGAGAGCTTGATAAAATGCCGTCTGAAGCGGC
TTCAGACGGCATATCAGGGAAGGGTCAGGAGGCGGTATTCTGTGCGGCTTCCTGTTTGGC
TTTGTATTGTTTGAGATATTCGAGGGCGGCGGCTTTTTCGCTGTCGCTGCCGTATTTCAT
ATCGCGTTGGGCGCGGCGCAACTCGGCGCGTTCGCGGGCTTCGGCTATCTGTTTCGCCTG
ATAGTCTTTGCGGTTGTCGGCGGCGGCGAGGCGGTCTTGTTGGGTTTGCGCTTTTGCCAT
GGCTTTGGCGATGAGGTCGGCAGGGTTAAACGTCGGTTTTTTCGGTGTGTCGGGCGTTTG
CGGACGCGCGTTGCGGACGGCGGCTTCGCGTTCGGCAAGCATGGCCTTGCGTTCGTCGGC
TTCGCGCTGTTTGCGTTCGTTGCGTTTGAGGTAGCGCGTGCGCGCGTGTTCGGCGGCGGC
AAAACGGCTGTCGGCGGACAGGCTGAAGCGGCGCGCGCGGGGCAGGACGGTGTCGGCAAC
GGGCTGCATATGGATGCAGTCGACGGGGCAGGGGGCGACGCAGAGTCCGCAGCCGGTGCA
TTCGTCGGCGATGACGGTGTGCATAAGTTTGCCCGCGCCCATAATGGCATCGGCAGGGCA
GGCGCGGATGCAGGCGGTGCAGCCGATACAGGCGGTTTCGTCTATCCGGGCGAGTGCTTT
GGCTTGGGTTTTGGCAGGTGCGACAAAGGGTTTGCCGAGCAGGGCGGAAATGTCCCGAAT
GACGGTTTCTCCGCCCGGGGCGCAGAGGTTGTACGCTTCGCCTGTTGCGACTGCCTGTGC
GTAGGGCAGGCAGCCGTCGTAGCCGCATTCGCGGCATTGGGTTTGGGGAAGCAGGCGGTC
TATGGCGGCGGCTGTGGCGGTCATGTCGGTGTGCGGCTCAAAATCGAAAGGGCGTATTTT
AGCAGAATTGTATGCCGCGCCCGTTTCGGATGGTGCGCGGTGTTTTGTTATAATGCGGCG
GCGTATGCCGTTTCAGACGGCATTTTTCTGTATTTTCCTGTTCGGACGGTCTATGAACGA
ATTTTCGCTTGCCCCTATTGTGATTGTTTTGCTGGTGTCGGTCATTACGGTGATCCTGTG
CCGCAAGTTCAACATTCCCTCCATGCTGGGCTACCTGCTGGTGGGCTTTTTGGCGGGGCC
CGGTATGCTCAGCCTGATTCCGAAAAGCCATGCGACGGATTATTTGGGCGAAATCGGGAT
TGTGTTCCTGATGTTCAGCATCGGTTTGGAGTTCTCGCTGCCCAAGTTGAGGGCGATGAG
GCGGCTGGTGTTCGGTCTGGGCGGTTTGCAGGTCGGCATTACGATGCTGTCGGTAATGGG
CATACTGATGCTGACGGGCGTGCCGTTCAATTGGGCGTTTGCCGTGTCGGGCGCGTTGGC
GATGTCGTCCACGGCGATTGTGAGCCGGATTTTGTCGGAAAAGACGGAATTGGGGCAGCC
GCACGGTCAGATGGCGATGGGCGTGCTGCTGATGCAGGACATCGCCGTCGTGCCGCTGAT
GATTCTGATTCCCGCGCTGGCGGGCGGAGGGGACGGAAATATTTGGGCGGCCTTGGGTTT
GGCGTTTGCAAAAATGCTGCTGACGCTGGGGCTGCTGTTTTTCGTCGGCAGCAAAATTAT
GTCGCGATGGTTCAGGATGGTGGCAAAACGCAAATCGTCCGAACTCTTTATGATCAATGT
GCTGCTGGTAACCTTGGGTGTGGCTTATCTGACTGAGCTGGAAGGTTTGTCTATGGCGTT
GGGCGCATTCGTTGCCGGCATGCTGCTTTCGGAAACGGAATACCGTTTCCAAGTCGAAGA
CGACATCCGCCCGTTCCGCGATATTTTGCTCGGCTTTTTCTTTATCACGGTCGGCATGAA
GCTGGACATTCAGGCATTGATCGGCGGCTGGCGGCAGGTATTGATGCTGTTGGCAATGCT
GCTGGTGTTGAAGGCACTGGTTGTGTTTGCCATTGCCTTCAAAATGAAACATTCGGTCGG
CGACAGCCTCAAAACGGCTTTGTATCTCGCGCAGGGCGGCGAGTTCGGCTTCGTGATGCT
GGCCATTGCCGGGCAGCTTGATATGGTTTCGCCAGAATGGGAACAGGCGGCGACGGCGGC
GGTTCTGCTGTCGATGATTATCGCGCCCTTCCTCTTGGGCGGCAGCGATGCGCTGGTCGG
GCGTTTGGTCAAGTCAAGCTGGGACATGAAGTCGCTCGATCTGCACAGTATGCTGGTAGA
AACCATGAGCAAGTCCGACCATGTGCTGATTGTCGGCTTCGGCAGGGGCGGGCAGACGGT
CGGACGCGTCCTTGCCCAAGAGGATATTCCGTATTTCGCGCTCGACTTGGACATTGCGCG
GGTGCAGGTTGCCAGAAGTGCGGGCGAACCGGTGTCGTTCGGCGATGCGAAACGCAGGGA

## Appendix A

AGTATTGGAAGCCGCCGGTCTGGGACGGGCGAAAATGGTGGTGGTTACGCTCAACAATAT
GCACGAAACGCAACACGTTTTAGACAATGTGCTGTCCATGTATCCCAATATGCCCGTATA
TGTGCGCGCCACCAACGACGATTATGTGAAAACGTTTACCGATATAGGTGCGGAAGAAGC
CGTGTCGGACACCAAAGAAACCGGACTCGTGCTGGCAGGCTATGCAATGTTAGGCAACGG
CGCGTCGTATCGGCACGTCTATCAGACGATGGCAAATATCCGCCACAGCCGTTATGCCGC
GTTGGAGGGACTGTTTGTCGGTAGTGATGATGAGGCAGGATTCGGCGAAAACGGCGAAAC
CGTCCGTCACGCCTTTCCTTTGGCTGCAGAAGCATACGCCGTCGGCAAAACAGTCGGCAC
GCTTCCGATGGCGGCTTACGGCATCAAACTCTTGTTCGTCCGCCGCCGCACCGGCCGGAT
TGAAAACCCGGATGCCTCGTTTACATTGGAAGGCGGTGACGTGTTGGTGGTCGCAGGCAA
AAAAGAAGAAATTATCTCTTTTGAAAACTGGAGTTTGCAGGGAATATAAATGAAATGCCG
AAATAAGGCTTGCGCCATTTCCGGTTATTTGGTTTAATAACGCTTTCGCAAATCGCAAGG
GTGATTAGCTCAGTTGGTAGAGTGTCTGCCTTACAAGCAGAATGTCGGCGGTTCGACTCC
GTCATCACCCACCAAGTTTTCTTTCATTGTTGCAAACAATGGATGCGCGGTGGTAGCTCA
GTTGGTTAGAGTACCGGCCTGTCACGCCGGGGGTCGCGGGTTCGAGCCCCGTCCGCCGCG
CCAAGTTTCAAAATACTGACTCTGTCGGTATTTTTTATACACGGGTGATTAGCTCAGTTG
GTAGAGCGTCTGCCTTACAAGCAGAATGTCGGCGGTTCGACTCCGTCATCACCCACCAAG
TTTTCTTTCATTGTTGCAAACAATGGATGCGCGGTGGTAGCTCAGTTGGTTAGAGTACCG
GCCTGTCACGCCGGGGGTCGCGGGTTCGAGCCCCGTCCGCCGCGCCAAAAGTTAAGGAAT
ACCAACCTCCGGTTGGTATTTTTTTGTTTGTATGCTTTGAAAAATGTTTGTTTCCGGATT
TTGCCATTCCCATCCGGTTTTGCGCTGTACGATGTGTTTTAGCGCGGACTTGCTCAAAAT
CGCATGTGATTCCGGTATTTGAGGCTTTGATTAGGGATGCGGACTTTCAATATATTTTCT
CAGCTACAACAACGAAGGCTTGATGTCTGTCGGGCAGGTAAGGGAGATTTTTGAGCGTTT
CGGCAAATATAATTTGGTTCAAACGGAATACCGGCGTTTTAAGGCAGATAAGACAGAAAA
CCGTAATCATAAGGCAAATTCGATATTCGAATTTCTGCATATTTTAGAAAAGACCTTTTA
TAGTGGATTAACAAAAACCAGTACAGCGTTGCCTCGCCTTAGCTCAAAGAGAACGATTCT
CTAAGGTGCTGAAGCACCAAGTGAATCGGTTCCGTACTATTTGTACTGTCTGCGGCTTCG
TCGCCTTGTCCTGATTTTTGTTAATCCACTATAAAAATTCTTGCCGGATGCTGCAAACAA
CGCCGGTTTGCATTCCTGATGGCGGTGGTTTTCTTAGACGAACGCCCGAACACGCAGGAA
TGGATAGGCTTGGGGCTGGTTACGGCGGGCGTGTTGACGCTGGCACTGAAACGGTAAAGC
CGCAAGAAATAAATGAAATGCCGTCTAAAAAACTGTTTTCAGACGGCATTTTCGTTTCTG
TCCATCCTCAGCACTCGACCACGCGCACGGATACGGGGACGGCTTTTTTCCGGAGCGTGG
CGGCGAGTCCGGCAAGCGAGGTTTCTTTGTAGGTCGCCTTCATATCCCGGCCGGTTTGCA
GCATGGTTCGGATGACTTCGTCGAGCGAGACTTTTTTGTCCGTGCCGTCTTCCAAAAGCG
CGAGCGTGCCGAGTTTGAGGGCTTTTTCGGCGGCGATGCCGTTGCGCTCGATGCAGGGGA
TTTGCACCAGTCCGCCGACGGGGTCGCAAGTCAGCCCCAAATGGTGTTCCATCGCCATTT
CGGCGGCGTTTTCCACTTGTTTGGGCGTGCCGCCGATGACTTCGGCGTATGCGCCCGCCG
CCATCGAACACGCTACGCCGACTTCGCCCTGACAGCCGACATCCGCACCGGAAATGGAGG
CGTTGGTCTTGTAGAGGATGCCGATTGCGCCTGCGGTGAGCAGGAAGTTTTCGACGCGTT
CCTGTGTGGCGTGCGGATTGAACTTGCGGAAATAGTGCAATACGGCGGGAATGATGCCTG
CCGCGCCGTTGGTCGGTGCGGTAACGACGCGTCCGCCGGCGGCGTTTTCTTCGTTGACCG
CCATGGCGTACACCATCGGCCAGAGCTGGGTGTTGACGATTTCGGTTTCGCGCAGGACTT
TGAGCTTGGCGGCAAGCTGCGGGGCGCGGCGGCGGACGTTCAATCCGCTGGGCAGTTCGC
CGTCCGCACCCAAGCCGCGTTTGATGCAGCCTTCCATAACCTCGGCAACGGCAGCGGCGC
GGCGGCGGATTTCGGCTTCGCCGCATCCGGCAAGCGCGGCTTCGTTTGCCAACACGACTT
CGGAGATGTCGAGCCGGTTCAGACGGCATCGGGCAAGCAGTTCGGCGCAACTGGTATAGG
GATAGGGAACGGCTTTTTCCGTTTCCGCCTGCCGGTCAAAATCTTCTTCGGTAACGACAA
AGCCGCCGCCGACCGAATAATAAACCTGTTCATTCAATACCGTGCCGTCTGAAGCATAGG
CGGTAAAACGCAGGCTGTTGGGGTGTTTGGGCAGCACTTGATTGCCGAGTATGTTCAGGT
CGCGGTCGGGGATGAAGCGGATTTCTTGCCCGTTGAGCCGGAGGATGTGCTGCGTGCGGA
~~TGCGTTCGAGGCGTTCGGGAATGCCGGCAAGCGGGATGTCGTGCGGCAGGCTGCCTTCCA~~
AACCGAGCATCAGCGCGTCAAATGTACCGTGTCCGTATCCGGTCAGTGCGAGCGAGCCGT
AAATGTCGATGACGATGCGAACAGCCTGTGCATCCAAACCTGCCGCAAAGGCGGCGGCTG
CCTTCATCGGGCCGACCGTATGCGAACTGGAAGGCCCGATACCGATTTTGAAAATATCGA
AAATGCTGATCATATTTTGCTCCGACGGTTTTTCAGACGGCACAGGTTCCGTTTGACCAA
CCAAAAAGGAGACGCGGCACGATGCCCGTCTCCTTTTTTAAAACGGCACTTATGCGTCGA
TATTTTGGGCAATCAGCGCGTTGTTTTCGATAAAGGCACGGCGCGGCTCGACCTCGTCGC
CCATCAGCGTAACGAACACTTCGTCGGCGGCAATGGCATCTTCGATGCGCACTTTCAACA
GGCGGCGCACGGCGGGATCCATCGTGGTTTCCCACAGCTGCTCGGGGTTCATCTCGCCCA
AGCCTTTGTATCGTTGGATGGACATACCTTTTTGGGCAACGCTCATCAAGATGTCCAAAG
CGGTTTCAAAGCTGTCCGCGTCGTACCCGTTTTCGCCTTTGTAAAGCTTGGCACCCTCGC
CGACCATGCCTTTGAGCGCGGCGGCGGTTTGGGTGAGGGTTTGGTAGGCTTTGCTGTTGA
GGAACTTGGGTTCGATGTAGCTGACCATGACGTTGCCGTGCAGCTTGCGCGTGATTTTGA
TGAACCGGTGTCCTTCATGACCTTCGATGCGTTCGAGGGCGACTTCTTTTTCGTCAAGCA
GACCGGAAAGTTCGGCAACGGCTTTATCGGCGTTTTCAGACGACGTCAAATCAATGGGCG
ACGCGTGTAGCATGGCGCGCAGGACGAGTTCGTCTACGAAGCGGCTTTCCTGTTCGATGA
CGGTTTTTGCCAACAGGAATTGTTTGGCGGTGTCGGCAAGTTCTGCGCCTTCGATGGTGC
GGCCGTCTGAAATGATTTTGGCTTTTTCCAAGGCAAGACCGAGCAGCCATTGGTCTTTTT
CCAACTCGTCCTTGAGGTAACGTTCCTGTTTGCCGTATTTCGCTTTATACAAAGGCGGCT
GGGCGATATAGATGTAGCCGCGCTCGACCAGCTCGGGCATTTGGCGGTAGAAGAAGGTCA
GGAGCAGGGTGCGGATGTGCGCGCCGTCCACGTCGGCATCGGTCATGATGATGATGCGGT
GGTAACGCAGTTTTTCGGCATTGAATTCTTCTTTGCCGATGCCCGCGCCCAAAGCGGTAA
TCAGCGTGGCGACTTCTTGGCTGGCCAGCATTTTTTCAAAACGTGCTTTTTCGACGTTCA
AAATTTTACCTTTGAGCGGCAAAATCGCTTGGAATTTGCGGTCGCGGCCTTGCATGGCGG
AACCGCCTGCGGAGTCGCCCTCGACGAGGTAGAGTTCGGACAGGGCAGGGTCTTTTTCTT
GGCAGTCGGCGAGTTTGCCGGGCAGTCCCAAGCCGTCCATCACGCCTTTGCGGCGGGTGA

## Appendix A

TTTCGCGTGCTTTGCGGGCGGCTTCGCGCGCGCGGGCGGCATCGACGATTTTGCCGGTGA
TGATTTTGGCTTCGTTCGGATTTTCTTCGAGGAAGTCGGTCAGGGCTTGGCTGATGACTT
CGTTGACAACGGGGCCGATTTCGCCGGAAACCAGTTTGTCTTTGGTTTGGGACGAGAATT
TGGGGTCGGGCAGTTTGACGGACAACACGCAGGTCAAACCCTCGCGCATATCGTCGCCTG
CGGTTTCCACTTTGGCTTTTTTGGCGACTTCGTTGGCTTCGATATAGTTGTTGATGGTGC
GGGTCATCACTTGGCGCAGTGCGGTCAGGTGAGTACCGCCATCACGTTGCGGGATGTTGT
TGGTGAAACACTGCACGCTTTCTTGATAGCTGTCATTCCATTGCATCGCGCATTCGACGC
TCATGCCGTCTTTTTCGCCGAACGCGTAGAAGATTTTTTCGTGCAACGGCGTTTTTTTGC
GGTTCATGTATTGCACGAAACCCGCCACGCCGCCGGAAAGGGCGAAGCTTTCGTGTTTGC
CGTCGCGCTCGTCGGTCAATTCGATGTCCACGCCGTTGTTCAGGAAGGAAAGTTCGCGGA
TGCGTTTGGCAAGGATGTCGAAGCTGTATTCGACGTTGCCGAAGGTTTCCGTACTGGCGA
GGAAGCGCACGGTCGTGCCTTTTTTATCGGAATCGCCGACAATTTTCAGCGGCTCTTCGG
TTTCGCCGCGCACGAAGCGGACGAAGTGTTCTTTGCCGTCGCGGTAGATGGTCAGCGTTA
CCCAGTCGGACAGCGCGTTGACGACGGACACGCCCACGCCGTGCAGGCCGCCGGAGATTT
TGTAGCTGTTGTTGTCGAATTTACCGCCCGCGTGCAATACGGTCATGATGACTTCGGCGG
CGGAGCGTCCTTCTTTCGGGTGGATGCCGGTGGGCATACCGCGCCCGTTGTCGGCGACGC
TGACGGAATGGTCGGCGTGTATCGTTACCGTGATTTTGTCGCAATGTCCGGCGAGTGCTT
CGTCAATGGCGTTGTCCAATACTTCGAACACCATGTGGTGCAGACCGCTGCCGTCCTGCG
TGTCGCCGATGTACATGCCGGGGCGTTTGCGTACCGCTTCCAAGCCTTCGAGCACCTGAA
TGCTGTCGGCGCCGTATTCTTCGTGTTTTTGTTCAGTCATATTTTTTGCCGGATTTTGAA
AAGATTTTGCGATGCCGCCAAAACAAGTCCGCACCTTGTAGAAAAAGCGGGCGGACGAC
ATATATAATTGTGTATTATAGCCGATTTTGCCGCCTAATTCAGCGTTATCCGCATCAGTG
TGCCGCCGGGAAAAGATGAAACGGTACGTTTGCCTCCGGCATCAGGTCGGGGATTGTCCC
GTAAAGTGGCAAAAGCGTTTTTTTGCCACTAAAATCTACACCCTATACTTTTCGGACAGG
GGCGCGGAAATGGAAATATGGAATATGTTGGACACTTGGCTCGGTGCCGTCCCGATACGT
GCGGAGGCGGTCGAATCCGTGGCGGCGGTTGCGGCTTTGCTGCTGGCGCGCGCCCTTCTG
TTGAATATCCACTTCAAACGGCATCCGGATTTCGGCATCGAAAGCAAGCGGCGGTTTTTG
GTTGCCAGCCGCAATATAACGCTGCTTTTGGTGCTGTTTTCGCTGGCATTTATCTGGTCG
GCGCAAATCCAAACGCTGGCTTTGTCGATGTTTGCGGTGGCGGCGGCGGTCGTCGTGGCG
ACGAAGGAACTGATTATGTGTCTGTCGGGCAGTATTTTAAGGTCTGCCACCCAGCAATAC
TCGGTCGGCGACTATATCGAAATCAACGGCCTGCGCGGGCGCGTGGTCGACATCAACCTG
TTGAACACGCTGATGATGCAGGTCGGTCCGAACCCCTTGGTCGGACAGCTTGCGGGAACC
ACCGTTTCTTTCCCCAACAGCCTGTTGTTGAGCCACCCCGTGCGCCGCGACAATATTTTG
GGCGACTATGTCATCCATACGGTCGAAATCCCCGTTCCCATCCATTTGGATTCGGATGAA
GCCGTATGCCGTCTGAAAGCCGTACTCGAGCCCTTGTGCGCGCCCTACATCCCCGCCATC
CAACGGCATTTGGAAAACGTGCAGGCGGAAAAACTGTTTATCACGCCCGCCGCCAGACCG
CGCGTTACCCGCGTGCCGTACGATGACAAGGCATACCGCATCATCGTCCGCTTCGCTTCC
CCCGTTTCAAAGCGGCTGGAAATCCAACAGGCGGTTATGGACGAATTTTTGCGCGTACAA
TACCGCCTGTTAAATCACCCCGCCGGCTCCGAAACACTTTAACTTTCCCCGACCGACCCC
ATTTCCGGCTTCAGACGGCATATTGCCGATATGCTGTCTGAAACACAACACGCAAAGGAA
ACCCATCTTATGACTGACAACGCACTGCTCCATTTGGGCGAAGAACCCCGTTTTGATCAA
ATCAAAACCGAAGACATCAAACCCGCCCTGCAAACCGCCATCGCCGAAGCGCGCGAACAA
ATCGCCGCCATCAAAGCCCAAACGCACACCGGCTGGGCAAACACTGTCGAACCCCTGACC
GGCATCACCGAACGCGTCGGCAGGATTTGGGGCGTGGTGTCGCACCTCAACTCCGTCGCC
GACACGCCCGAACTGCGCGCCGTCTATAACGAACTGATGCCCGAAATCACCGTCTTCTTC
ACCGAAATCGGACAAGACATCGAGCTGTACAACCGCTTCAAAACCATCAAAAATTCCCCC
GAATTCGACACCCTCTCCCCCGCACAAAAAACCAAACTCAACCACGATCTGCGCGATTTC
GTCCTCAGCGGCGCGGAACTGCCGCCCGAACAGCAGGCAGAACTGGCAAAACTGCAAACC
GAAGGCGCGCAACTTTCCGCCAAATTCTCCCAAAACGTCCTAGACGCGACCGACGCGTTC
GGCATTTACTTTGACGATGCCGCACCGCTTGCCGGCATTCCCGAAGACGCGCTCGCCATG
TTTGCCGCCGCCGCGCAAAGCGAAAGCAAAACAGGCTACAAAATCGGCTTGCAGATTCCA
CACTACCTCGCCGTCATCCAATACGCCGACAACCGCGAACTGCGCGAACAAATCTACCGC
GCCTACGTTACCCGCGCCAGCGAACTTTCAGACGACGGCAAATTCGACAACACCGCCAAC
ATCGACCGCACGCTCGCAAACGCCCTGCAAACCGCCAAACTGCTCGGCTTCAAAAACTAC
GCCGAATTGTCGCTGGCAACCAAAATGGCGGACACGCCCGAACAAGTTTTAAACTTCCTG
CACGACCTCGCCCGCCGCGCCAAACCCTACGCCGAAAAAGACCTCGCCGAAGTCAAAGCC
TTCGCCCGCGAAAGCCTGAACCTCGCCGATTTGCAACCGTGGGACTTGGGCTACGCCAGC
GAAAAACTGCGCGAAGCCAAATACGCGTTCAGCGAAACCGAAGTCAAAAAATACTTCCCC
GTCGGCAAAGTATTAAACGGACTGTTCGCCCAAATCAAAAAACTCTACGGCATCGGATTT
ACCGAAAAAACCGTCCCCGTCTGGCACAAAGACGTGCGCTATTTTGAATTGCAACAAAAC
GGCGAAACCATAGGCGGCGTTTATATGGATTTGTACGCACGCGAAGGCAAACGCGGCGGC
GCGTGGATGAACGACTACAAAGGCCGCCGCCGTTTTTCAGACGGCACGCTGCAACTGCCC
ACCGCCTACCTCGTCTGCAACTTCGCCCCACCCGTCGGCGGCAGGGAAGCCCGCCTGAGC
CACGACGAAATCCTCATCCTCTTCCACGAAACCGGACACGGGCTGCACCACCTGCTTACC
CAAGTGGACGAACTGGGCGTATCCGGCATCAACGGCGTAGAATGGGACGCGGTCGAACTG
CCCAGCCAGTTTATGGAAAATTTCGTTTGGGAATACAATGTCTTGGCACAAATGTCAGCC
CACGAAGAAACCGGCGTTCCCCTGCCGAAAGAACTCTTCGACAAAATGCTCGCCGCCAAA
AACTTCCAACGCGGCATGTTCCTCGTCCGGCAAATGGAGTTCGCCCTCTTTGATATGATG
ATTTACAGCGAAGACGACGAAGGCCGTCTGAAAAACTGGCAACAGGTTTTAGACAGCGTG
CGCAAAAAAGTCGCCGTCATCCAGCCGCCCGAATACAACCGCTTCGCCTTGAGCTTCGGC
CACATCTTCGCAGGCGGCTATTCCGCAGGCTATTACAGCTACGCGTGGGCGGAAGTATTG
AGCGCGGACGCATACGCCGCCTTTGAAGAAAGCGACGATGTCGCCGCCACAGGCAAACGC
TTTTGGCAGGAAATCCTCGCCGTCGGCGGATCGCGCAGCGCGGCAGAATCCTTCAAAGCC
TTCCGCGGCCGCGAACCGAGCATAGACGCACTCTTGCGCCACAGCGGTTTCGACAACGCG
GTCTGACGGCAGGGTTGAAGTAAAAAATATGGCGGATTCGATAGAAAAACATCCGCACCG

## Appendix A

TCATTCCCGCGCAGGCGGGAATCCAGACCGGTCGGTGCAGAAACTTATCGGGAAAAACGG
TTTCTTTAGATTTTACGTTCTAGATTCCCACTTTCGTGGGAATGACGCGGAAAAGTTGCT
GTGATTCCGGATAAATTTTCGCAACGTTTAATTTCCGTTTTACCCGATAAATGCCCGCAA
TCTCAAATCCCGTCATTCCCCAAAAACAAAAAAATCAAAAACAGAAATCCCATCATTCCC
GCGCAGGCGGGAATCCAGGTCTGTCGGTGCGGAAACTTATCGGATAAAACGGTTTCTTTA
GATTTTACGTTCTAGATTCCCGCTTTCGCGGGAATGACGGAATATTTTTGAATTTGATAA
AAATGCCGTCTGAAACGGTCAAACAACGCTTCAGACGGCATTTTATAGTGGATTAACAAA
AATCAGGACAAGGCGACGAAGCCGCAGACAGTACAAATAGTACGGAACCGATTCACTTGG
TGCTTCAGCACCTTAGAGAATCGTTCTCTTTGAGCCAAGGCGAGGCAACGACGTACTGGT
TTTTGTTAATCCACTATATTTTCCGACATCATTGAATCAAACCCAAATGCGACAAGAGCG
TCCATGTGCCGATGGCAATCAACACCAAACCTCCGGCAAATTCCGCACACCTGCCGAACA
ATACGCCCAAAGCCCTTCCCGCCGTCAGCCCGACCGCCACCATCACCGTCGTCGCCATAC
CGATGATTGCGGCGGCAAAGGCGATGTTTACCTCCATAAACGCCAAGCCCACCCCGACTA
TCATGGAATCAATACTGGTTCCAAAAGCAGTCAAAACCGTCATCCATAGGCTTTCCCGTT
TGCTTTCGCGCACATCTTCCGCCTCGCCGGACAGCCCTTCGCGCATCATTTTCAGACCCA
GCCCGCCCAGCAGGACGAAAGCCACCCAATGGTCCCATTCGCTGATAAACGGCTTGGCAT
AAAAACCGCCTACCCAGCCTGCCAGCGGCGTGAGCGCTTCAACCGTGCCGAACACCAAAG
CCGTTGCCGCAATTTTGCGCGGAGGCATTCTGACCGCCGCACCCTTTGCCAATGCGACGG
CAAACGCATCCATCGACATCCCCAGAGCAATCAAGAGCAAAGCATAAAAACCCATACCGC
ACCCGTCCTCAAAAAGGGCGGATTATAGCAAAAGCAAAAAAATGCAAAAATGCCGCACGA
AAACCCGCATCCCGTCATTCCCGCAAAAACAAAAAATCAAAAACAGAAATCCCGTCATTC
CCGCGCAGGCGGGAATCCAGAGTTGTCGGTGCGGAAACTTATCGGATAAAACGGTTTCTC
CAACCCCGAGTCCTTGATTCCCACTTTCGTGGGAATGACGGGATATTTTGCGTTTAATAA
AAAACGCCCGCTGAAACGGCGGGGCGGGATGGGGGAATGCCGTCTGAAACGGTCGGACAA
TGTTTCAGACGGCATTTTTATGCCCGGTTATTTCCGATAGCGGACGGCGCGGGACAGGAT
TTCTTCAATTTCCATCCACATAATGCCCCCTTACAGCAAACCAGCCTGACCCAGTGCGGG
ATCGGTCGCGCGGGCGGCTTGGGCATCTTCGACAGTCAGTCCAAGGGCTTTGGCCACGCC
TTCGCCGTATGCCGGGTCGCAACGGTAGCAGTTGCGGATATGGCGGTATTTGATGAAGTC
GGGCGCGTCGCCCATTGCGGCGGCGGTGTTGCCGAACAATGCCTGTTTCTGCGCGTCGTT
CATCAGGTTGAACAGGGCGCGCGGTTGGCTGAAATAGTCGTCATCGTCTTGGCGGTAGTC
CCAGTGTGCCGCGTCGCCGTTGATTTTCAAAGGCGGTTCGGCGAAGTCGGGTTGTTGCTG
CCATTGGCCGAAGCTGTTGGGTTCGTAGTGCGGCAGGCTGCCGTAGTTGCCGTCGGCGCG
GCCTTGCCCGTCGCGCTGGTTGCTGTGAACAGGGCAACGCGGACGATTGACGGGAATTTG
GCGGAAGTTTACGCCCAAACGGTAGCGTTGTGCGTCGGCGTAATTGAACAAACGCGCTTG
CAGCATTTTATCTGGGCTGGCGCCGACACCGGGAACGAGGTTGCTCGGTGCGAAGGCGGA
TTGTTCCACATCGGCGAAGAAGTTTTCGGGATTGCGGTTCAACTCGAATTCGCCCACTTC
AATCAGCGGATAGTCTTTTTTCGGCCAAACTTTGGTCAAGTCAAACGGATGATAAGGTAC
TTTTTCCGCGTCTGCTTCAGGCATGACTTGGATGTACATCGTCCATTTCGGAAACTCGCC
GCGTTCGATGGCTTCGTATAAGTCGCGCTGATGGCTTTCGCGGTCGTCGGCGATGATTTT
GGCGGCTTCTTCGTTGGTCAGGTTTTTAATGCCTTGTTGGGTGCGGAAATGGAATTTCAC
CCAAAAACGCTCGCCTGCTTCGTTCCAGAAGCTGTAGGTATGCGAACCGAAGCCGTGCAT
ATGGCGGTAGCCGGCGGGGATGCCGCGGTCGCTCATCACGATGGTAACTTGGTGCAGTGC
TTCGGGCAGCAGCGTCCAGAAGTCCCAGTTGTTTGTGGCAGAGCGCATATTGGTGCGCGG
GTCGCGTTTGACGGCTTTGTTCAGGTCGGGGAACTTACGCGGGTCGCGCAGGAAGAACAC
GGGCGTGTTGTTGCCGACCACATCCCAGTTGCCTTCTTCGGTATAAAATTTCAAGGCAAA
ACCGCGGATGTCGCGTTCTGCATCGGCTGCGCCGCGTTCGCCTGCCACGGTGGTGAAACG
GGCGAACATCTCGGTTTTTTTGCCGACTTCGCTGAAGATTTTGGCGCGGGTGTATTTGGT
GATGTCGTGCGTTACGGTAAACGTACCGAACGCGCCCGAACCTTTGGCGTGCATACGGCG
TTCGGGGATGACTTCGCGCACGAAGTCGGCGAGTTTTTCATTCAGCCACAAATCCTGCGC
~~CAGCAGAGGGCCGCGAGGACCGGCGGTCAGGCTGTTTTGATTGTCGGCAACAGGCGCGCC~~
GTTGTTCATGGTCAGATGGGTTACAGGGCATTTGGAGGTAGTCATCGCTCTTGTTCCTTT
TCTCAGGTTGGTCAAATGGGGGTAAACGGCTTACAGTACGATTTGGCGGAAAGCGTATTC
GTAACCGGTTTCTTGATTGCAATAAATTTCTTGAATCGACATTTTATTTCCCTTTTGTAA
AAACTATGGATGCGACTATACGCCAAGATTTTCGCTATTAAAACTATGAAATCGATTTAA
TATTATTATAAGCAATCGGTTCTTGATTTTCGTTTGTTTTTTGTTATCGAACGGAATCCG
AACCCGCTCATTAAAACCATTTATAATGCAATGACGCTTTGCGGCATTTTTTGCGCCGAC
AGGCTGAAAATAACAATTTTCCCCACATTATCATGACCTTACTCGGAATAAAGCTCAAAC
AGACCCAGCAGCTCAACCAGCGGCTGCAACAATCTTTGCGCGTATTGCAGATGTCGGGTA
TCGAACTTGAACGCGAGGTCGAAAACTGGCTGTCGGACAACCCCCTGCTCGAACGCAAAG
ACACGGATGAATTTTCCGATGCCGAGTTCAGCCATTACACTGCGCCTGCCCGTCAAATCG
GCGGAGACGAAGGCGAAGATATGCTGTCCAACATCGCCGGCGAGCAGGATTTCAAGCAAT
ACCTGCACGCGCAAGTATGCGAACACCCGCTTTCCGACCAAGAAGCCGCCTGTGTCCACA
TCCTTATCGATTTCCTTGACGAGCAGGGTTATCTGACCGACAGCATCGAAGACATCCTCG
ACCATACGCCCTTAGAGTGGATGTTGGATGAAGCAATGCTGCAACACGCGCTGACCGCAT
TGAAAAAATTCGACCCGGCAGGCGTGGCCGCCGCCGATTTGAACGAATCGCTGATACTGC
AGATAGAAAGATTGGGCGAATGTGCTGCCAAACCCGCCGCCCTGCATATCGTCCGAAACG
CCCTCGACAGCATTGACGGCAACCGCAGCCAAACCCTCGCACGAATAAAAAAACACCTGC
CCCAAACCGACAGCGGCACACTCGAAGCCGCACTCGACCTCATTGCTTCGCTCAATCCCT
TTCCCGCCGCCGGTTTTGCCTCGTCCACGCCCACGCCGTATTCTGACGAGGCGCTCGCCA
ACCTGCTGGCTTTCCGCGGCATGGAGGTTTCTCGCCGCACCATTGCCAAATACAGAGAAT
CCTTTGAGATTCCGGCAGCACACAAACGCAAAACCGCAGAATAATTGCCGAATAATCTTA
TAAAGACAACAAACCAAAAGCCGGCATTTCTGCGAAAGCGGGAATGCCGAATCCGTCCGC
GCGGAAACCTGCATCCCGTCATTCCCGCGAAAGAGGGAATCTAGAAACGCAAAGCTGCAA
GAGTTTATCGGAAATGACCGAAACTCAACGAACCTGGATTCCCGCTTTCGCGGGAATGAC
GGGGGTTTGGCGGGAATGACGAGGGTTTGGGATTTCTGTTTTTGAATTTCTGTTTTTGTG

## Appendix A

AGAATGGCAAGATTTTCGGTTCTTGTATGGATAACGAGATTTTAGATGGCGGGAATTTGT
CGGGAAAACAGCAATCTGAGACCTTTGCAAAAATAATCTGTTAACGAAATTTGACGCATA
AAAATGCGCCAAAAAATTTTCAATTGCCTAAAACCTTCCTAATATTGAGCAAAAAGTAGG
AGAAATCAGAAAAGTTTTGCATTTTGAAAATGAGATTGAGCATAAAATTTTAGTAACCTA
TGTTATTGCAAAGGTCTCAATCTTTACCGTCATTCCCACGAAAGTGGGAATCTAGAAACG
CAAAGTTGCAAGAATTTATCGGAAATGACCGAAACTCAACGAACCTGGATTCCCGCTTTC
GCGGGAATGACGAGGGTTTGGGATTTCTGTTTTTGAATTTCTGTTTTTGTGAGAATGGCA
AGATTTTCGGTTCTTGTATGGATAACGAGATTTTAGATGGCGGGAATTTGTCAGGAAAAC
AGCAACCCTCCGCCGTCATTCCCACGAAAGTGGGAATCTAGAAACGCAAAGTTGCAAGAA
TTTATCGGAAATGACCGAAACTAAACGAACCTGAATTCCCGCTTTCGAGGGAATGACGGG
GGTGTGGCGGGAATGACGGGGGTTTATCAGAAATGACCGAAACTCAAAAGCGGGCAGCCT
TGTTTACGCCTTCAAAATATCGAGCAATTTCAAATCGACTTTTTCGGCATCGAATTTATC
TTTGGCAATCGCATAACTTGCATTCCCCATCAGGCGGACGGCTTCCCTGTTTTCGATAAA
ATAAATCATTTTTTCGGCCAAGATGCGGGGATTCCAAGGCTCGATCAGGAAGCCGTTGAC
CTTGTCGGCGACCGTTTCCCTGCATCCGGGGACATCCGTCGTAATCACTGCCCTGCCGAC
GGCCATTGCCTCCTGAGTGCTTCGGGGAACGCCTTCCCTATAATAAGACGGCAATACGAA
TATATGATGTTCTTTTATCACTTCGGAAACATTGTTCACAAAACCGGGGAAACGGATAAT
ATCGCGGGCGGCAAGCCGTTCCAAATCGCCCCCCCCCCCGCGTGATTTGTCGATTGCGCC
CAAAGCGGTAAAAACCGTATCGGGGTATTTGTCCTTAACCTGTTCCGCCGCCCGAATAAA
ATCATCAATCCCCTTTTCTTTCAGAAATCTGCCGATAAAGAGGAATTTTACGGTTCTTT
TTCATCGGGAATATCCGCCTCGGAATAAGGATATTGCCGCAAATCCAGACCGATTCCGCC
CAAAATATGGATGTTTTTTATTTTGATGCCGTATTTGTCCGTCAGTTCGTCTTTGTCGTC
GGGGTTTAATACAATCAGGCTTTCCAACATCGGCAGGGCAATGCGGTATAAGGCAATCAA
AATCCCCTTTATGATTTTTGTTTTTAACGGTATGCCTTCCGGCTGCGGGGTAAATGCGAA
TCCCAAACCTTCCAGCATCCCGACGATTCTGGGCACGCCTGCCAGTTTTGCGGCAAAAGT
GCCGAAAATCACGGGTTTTGCGAAATAAGGGAAAACCAAATCCGGCGATATTTTTTTGAG
TTCTTTAAAGATGAGGAAGGTGGATTTTATATCCGAAAACGGGTTCAGCCCGCTGCGGTT
TGAACGGTAGGTAACGGGTGTAACCCCCATTTCCCTGATAATATCCAATTCATTGTCGGA
AAACTCCGATACAAAGGCATACACCTGATGGTTTTTGCCGATTAATTTTTTAATGACGGG
GGCGCGGAAACCGTAAATGCTGGATGCGACTGTTGTGATAAAAACGATTTTCATAAGGCG
GACACCTTGAATATGGATTGGAAATGCGGTCTGCTACGGCAGGGTTTCATCCTGTAACCC
AGCAAGGCTTGGGTTTGCCTGCGTATTATAGTGGATTAACAAAAACCGGTACGGCGTTGC
CCCGCCTTAGCTCAAAGAGAACGATTCTCTAAGGTGCTGAAGCACCAAGTGAATCGGTTC
CGTACTATTTGTACTGTCTGCGGCTCGCCGCCTTGTCCTGATTTTTGTTAATCACTATAA
AAATGCCGTCTGAAACGGTTTCAGACGGCATTTCGATGTCGGCGGCGGCTTTGCGGAATC
AGCCTTTGAAGCGTTTGAAGACCAGCGTGCCGTTGGTGCCGCCGAAGCCGAAGGAGTTGG
AAATGGCAACGTCGATTTCCGCGTCGCGCGCTTCGTTGGCGCAGTAGTCCAAATCGCAGC
CGGCTTCAACGTCTTGTTCAAAAATGTTGATGGTCGGCGGGATTTTGCCGTCGTGTATCG
CCAAAATGCTGTACACGGCCTCCACGCCGCCCGCCGCGCCGAGCAGGTGGCCGGTCATGG
ATTTGGTCGAGCTGACGACGGTTTTGTAGGCGTGTTCGCCGAACGCGCGTTTGAGGGCTT
TGGTTTCGTTGGCATCGCCCAAGGGGGTGGACGTGCCGTGCGCGTTGACGTAATCCACGT
CTTCGGGATTGATGCCGGCATCTTTCAGCGCGCGGGTAACGGCAAGGGCGGGGCCTTCTT
CGTTCGGCGCGGTGATATGGTAAGCATCGGAACTCATGCCGAAGCCGACGATTTCGGCGT
AGATTTTCGCGCCGCGTTTTTTGGCGTGTTCCAATTCTTCCAACACCAATATGCCCGCGC
CTTCGCCGATAACGAAGCCGTCGCGGCCTTTGTCCCACGGACGGGAAGCGGTGGCGGGGT
CGTCGTTGCGGGTGGAGAGGGCTTTCATCGCGGCAAAACCGCCCACGCCCAAAGTGCTGA
TTGCGCCTTCCGCGCCGCCGGCAACCATTATGTCCGCGTCGCCGTATTTAATCATACGGA
GGGAATCGCCGATGGCGTGCGCGCCGGTGGTGCAGGCGGAAACCATCCCGTAGCTCGGGC
CGCGGTAGCCTTTGAGGATGGTAACGTGTCCGGAAATCAGATTAATCAGAGAACCGGGGA
TAAAGAAAGGGTTGATTTTGCGCGCGCCGCCTTCGATTACGGCTTTGCCGGTGACCTCGA
TGCCGGGCAGTCCGCCGATGCCGGAACCGATGTTCACGCCGATGCGGTCTTTGTCGAGGT
TTTCCACATCGTCCAAACCCGAATCGGCGATTGCCTGCAATGCGGCGGCAATGCCGTAGT
GGATGAATACGTCCATCCGGCGCGCTTCTTTCGCGCTGATGTATTGTCCGATGTCGAAAC
CGCGCACCTCGCCGGCGACACGGCTGTTGATGTCGGATGTGTCAAAGCGGGTAATCGCGC
CGATGCCGCTTTTGCCGGTGAGCAGGGTGTCCCAAGCCTCTGCGACAGTGTTGCCGACAG
GGGAAACCTGACCTAAGCCTGTAATGACTACTCTTCTCTGACTCATGATAACCTCGCTGT
TGGTTGTCGGAATGGGGGCATATGCGGCTGTCGTGCAGATGCCGTCTGTAATTTGCGGCA
GGGGTTCAAACAGTTTGCCATATAAGGGAAAAGCCTCTATTGCGCGGTGCAGCAGAGGCT
GTTGTGTCGGGCGACGACCGGTTAGCCGTTGTGGGCATTGATGTAGTCGATAGCCAGTTG
GACGGTGGTGATTTTTTCGGCATCTTCGTCGGGGATTTCGCAGCCGAATGCTTCTTCCAA
AGCCATAACCAGCTCCACGGTGTCCAAAGAATCCGCGCCCAAGTCGTCTTGGAAGGAAGA
TTCGTTTTTCACGTCGGCTTCGTTTACGCCCAGTTGTTCAGCAACAATTTTTTTAACTTG
TTGTTCGATGTTTGACATATCAGTCGTTCCTTTATGCCTTGCGGCAGGTTGTTTAAGGGA
AATAAATCGGTGGTATTGTACCGACTTTTAATAGAGTTTTCTATCTAATGACTATTATAT
CAATATTTGCCGATTTGTACATTTTTGGGTGCGGCGGGTTTTGTCGTTCAAGTTTGACCT
GTGTGCCGTATGTTTGGCGGGATTTCGGTTAAAATGGCGGCATTTCCATCTGAAGCAGAA
AGCCCTGTCATGTATCCACTTGCCCGTCGCATCCTGTTTGCACTCGATGCCGAAAAAGCC
CACCACTTCACGCTCGACGCGCTCTACACGGTTTATAAATTGGGTTTGATTCCTGTAACC
GACAACCGTACCAAACCTGTAAAATTGATGGGTATGGATTTGCCCAACCCTGTCGGACTT
GCCGCCGGACTCGACAAAAACGGCGAATACATCGACGCATTGGGCGCGCTCGGCTTTGGT
TTCATCGAAATCGGCACGGTAACGCCCAACCCGCAGCCCGGCAACCCGCAGCCGCGCCTC
TTTCGCGTTCCCGAACACCAAGGCATCATCAACCGCATGGGTTTCAACAACCACGGTATC
GACACCATGATACGCAACATCGAAAAAAGTAAATTCAGTGGCGTATTGGGCATCAACATC
GGTAAAAACGCGGTTACACCCATCGAAAACGCTGCCGATGATTATTTAATCTGCCTTGAA
AAAGCCTACGCACACGCAAGTTACATTACCGTCAATATTTCCTCGCCCAACACTAAAAAC

## Appendix A

CTCCGCGCGCTGCAAGGTGGCGACGAGTTGAGCGCATTGCTTGAGGCTTTGAAAAACAAA
CAGGCACAGCTTGCCTCTGTACACGGGAAATACGTCCCGCTCGCCGTCAAAATCGCCCCC
GATTTGGATGAAGCACAAATCGAAGACATCGCCCACGTTGTCAAATCCGTCGAAATGGAC
GGCATCATCGCTACCAATACCACCATCGACAAATCAAGTCTCGGCAGCCATCCGCTCGCA
GGCGAGCAGGGCGGTTTGAGCGGGCTGCCCGTTCATGAAAAAAGTAATCGGGTGTTGAAG
CTGTTGGCAGACCACATAGACGGCAAGCTGCCGATTATCGGCGTAGGCGGCATTATGGAA
GGCGAGGACTCGGCAGATAAAATCCGCTTGGGCGCGACCGCCGTCCAAGTGTACAGCGGA
TTGATATACAAAGGTCCGGCATTGGTCAAAGAATGTTTGAAGGCTTTGGCGCGATGACGC
GATCCGCCCAAAATGCCGTCTGAACGCACGTTTTGCCGTTCAGACGGCATTTTCATTTCC
TTTTTCCGCCTGACGCCCCTTGAAAATCCCTTACGCGCCGCCCTGTTTGAAATAAGGCAA
ACCGATGCGTGAACACGGAGCAGGCAATCGGAGTAAAAAATGAACCTTGATTTAACCGCG
CAAAAAGTCCGTCTTTCTTGGAAGGATATTCTGTGGGGGTATGGGAATAAATACTTGGGT
TGGGCTGATGTGGCAGCTTATGCCCGAAAAATGACGCTTTCAGATCATGATGAACGTGTG
TTCAAACTATCTTTAATCAACAAATCCAATATTCTTGAATTAAAGCCTGTTCTGGAAGAT
TTGGCTTCGGAAATGAGGGATTATTCCCCTAAAAATTGGCTGTACGTCCTCTTAAGCGAT
GTATTCCATAGAAAAGAAGAATTTGAGGATCCTTTGGGGGAAGTTGAAAAAATTTATGCA
GATTTTGATTATCCGGAAGAAATAGAATCATTTGTCAGGTATATGCCGCCCAAAGACGGT
TATATTCCTTCTGCCCACACCTATGAAGAAAATATTGCCCGGTTATATTCTCACTGGGAA
CACTATTTGAACAACGGCGGAGGGCAGGGTTAAAACCGGCAATCCGATGCCGTCTGAAGC
ATTATCCGGCCTTCAGACGGCATTTTGTTTTCCGACAGTTTATAAACTGTCGTTGTTTCT
TGACAGAAACAACGACCTTATTTGAAACGATTGGAGGACATGATTATGGGTTTTTGGAAT
GGTGTGGCAAAAGCAGCAAAAGCAGTGGGAGAGGGAATGATTGAAGCCGGCAATGAGCAT
AAGGCGTTGAAAATGGAATATGCGGAGAAATCAAGTGAGGAGCTGCATGAAATCGTCAAG
AGTGATGGTTTTTTTAAAAATTCCACACGGGAGAAAAGTGCGGCTTATGCTATTTTAAAA
GAGCGTGGCGAGGTGTGAACAGGAAACGGCGGCATTTGCCGCTGTTTTTTATTGGTAGGC
ATCCGTCCGAATATCGGGGCAAGGTTTCAGACGACATCGAAGGTTGCTATGATATAGTGG
CTTGACTTTAAACCGGTACGGCATCCCCTCGCCTTGTCCTGATTTAAAGTTAATCCACTA
TCTCATTCCCGTCATCCTTCCAAACGGAATCCGAAATGTCCGACAACCGCCTCGACACCG
CCCGCCGCCATTCCCTCTTCCTCGCCCGCCAGCTCGACAACGGCAAACTCAAGCCCGAAA
TATTCCTGCCTATGCTCGACAAGGTTTTGACCGAAGCGGATTTCCAAGCCTTTGCCGACT
GGGGCGAAATCCGCGCGGAAGAAAACGAGGAAGAATTGGCGCGGCAGTTGCGCGAGTTGC
GCCGTTATGTGGTGTCGCAGATTATCGTGCGCGATATCAACCGTATCAGCGATTTGAACG
AAGTAACCCGCACGATTACGCTGTTTGCCGATTTTGCCGTCAATACCGCGCTGGATTTTG
CCTACGCCTATTATCGGGACATGTACGGCACGCCGATCGGGCGTTATACCAAATCGCCGC
AGCATTTGAGCGTGGTGGCGATGGGCAAGGCGGGCGGCTATGAGTTGAACGTGTCTTCCG
ACATCGATTTGATTTTCGTCTATCCCGAATCAGGCGACACCGACGGCAGGCGCGAACGGG
GCAATCAGGAATTTTTCACCAAAGTCGGGCAGAAACTGATTGCGCTGCTGAACGACATTA
CCGCCGATGGGCAGGTGTTCCGCGTCGATATGCGGCTGCGGCCGGACGGCGATTCGGGCG
CGTTGGTATTGAGCGAAACCGCGCTGGAGCAATATTTGATTACACAGGGGCGAGAATGGG
AACGCTACGCGTGGTGCAAAGGTCGCGTGGTTACGCCGTATCCGAACGACATCAAAGCAC
TGGTGCGCCCCTTTGTGTTCCGCAAATATCTGGATTACGGCGCGTATGAGGCGATGCGTA
AGCTGCACCGCCAAATCAGCAGCGAAGTCAGCAAAAAAGGCATGGCGGACAACATCAAAC
TCGGCGCGGGCGGCATCCGCGAAGTCGAATTTATCGCCCAGATTTTCCAGATGATACGCG
GCGGACAAATGCGCGCGCTGCAACTGAAAGGCACGCAGGAAACGCTGAAGAAGCTTGCCG
AGCTGGGCATCATGCTGTCTGAACACGTCGAAACCCTGCTTGCCGCCTACCGCTTCCTGC
GCGATGTTGAACACCGCCTGCAATACTGGGATGACCAGCAAACCCAAACCCTGCCGACCT
CGCCCGAACAGCGGCAACTGCTCGCCGAAAGCATGGGTTTCGACAGTTATTCCGCTTTTT
CAGACGGTCTCAATGTTCATCGGAACAAAGTCAATCAGTTGTTCAACGAAATTTTGAGCG
AACCCGAAGAGCAAACGCAAGACAACAGCGAATGGCAATGGGCATGGCAGGACAAACCCG
~~ACGAAGAAGGGCGGCGATGCCGTCTGAAGGCGCACGGGTTCGATGCCGAAACCGTCGCCG~~
CAAGGCTCGACCAAATCCGCCACGGCCATAAATACCGCCATCTTTCCGCACACGCCCAGC
CGCGTTTCGATGCGGTTGTGCCGCTGTTCGTACAGGCGGCGGCAGCGCAAAGCAACCCGA
CCGATACATTGATGCGGCTGTTGGATTTTCTCGAAAACATCAGCCGCCGATCCGCCTATC
TCGCCTTCCTCAACGAACATCCGCAAACCTTGGCGCAACTGGCGCAGATTATGGGCCAAA
GTTCTTGGGTGGCGGCGTATCTGAACAAATATCCGATTTTGTTGGACGAACTCATCAGCG
CGCAGCTTTTGGATACCGCGTTTGATTGGCAGGCGCTCGCCGCCGCCCTTTCAGACGACC
TCAAAGCCTGCGGCGGCGATACTGAAGCGCAAATGGACACCCTGCGCCGCTTCCAGCACG
CCCAAGTCTTCCGTCTCGCCGTCCAAGACCTCGCCGGACTGTGGACGGTAGAATCCCTCT
CCGACCAACTCTCCGCCCTCGCCGACACCATCCTCGCCGCCGCCCTGCTGTGCGCATGGG
CGGACATGCCCAAAAAACACCGCGACACACCGCAATTCGCCGTCGTCGGCTACGGCAAAC
TCGGCGGTAAAGAACTCGGCTACGCCTCCGACCTCGACCTCGTCTATCTCTACGACGACC
CCCACCCCGACGCAGGCGACGTGTACAGCCGCCTCGCCCGCCGCCTGACCAACTGGCTTT
CCGCCGCCACTGGCGCAGGCAGCCTCTACGAAACCGACCTGCGCCTGCGCCCTAATGGCG
ACGCCGGTTTCCTCGCCCACAGCATCGCCGCCTTTGAAAAATACCAGCGCGAAAACGCCT
GGACGTGGGAACACCAATCCCTTACCCGCGCCCGCTTCATCTGCGGCACGTCCGAAATTC
AGACGGCCTTCGACCGCATCCGCACCGAAATCCTCACCGCCGAACGCGACCAAACCGCCT
TGGCAGGCGAAATCATCGAAATGCGCGAAAAAATGTTCCCCACCCACCCGCCTGCCGACA
GCAACGTCAAATACGCGCGCGGTGGCGTGGTCGATGTCGAATTTATCGTCCAATATCTGA
TACTTGCCCATGCCCGCCAGTATCCGCAACTCTTGGACAACTACGGCAACATCGCCCTCT
TAAACATCTCCGCCGACTGCGGTTTGATTGACAAAACCCTCGCCGGACAAAGCCGCACCG
CCTATCGCTTCTACCGCCGGCAGCAGCACAACACCAAACTGCGCGACGCGGCAAAAACCG
AAGTAACCGGCGAACTGTTGGCACATTACGGCAATGTCAGGAAATTGTGGCGGGAAGTGT
TCGGCGAAGAAGCGGCAACCGTCTGAACAAAAAATGCCGTCTGAAGCCTGACAATCTGGG
TTTCAGACGGTATTTTCGTACCGTGCCGTTTTAAGGTTGCGGCAGAGCTAAAGCGATTTA
TCGGGAATGGCTGAAACCCAAAAACCGGATTCCTCTTTCGCGGGAATGACGGGATTTCAG

## Appendix A

GCTTCTGTTTTTGTGGGAATGATGGGATTTTTTATCCAAGCAAAAATCAAAACAAACAAA
TAAGAACCGTTTAAAACCCCGCCGTTTCCATTAAAATAGCGCATTCTACTTTTTAGACGG
CCTTGGATTCGGATTTCAAGTGCAACACTAGTGTATTAGTGGTTGGAACAGATTCAAGAA
TAAAACACTTGGCGTTTCGTAGCCAAGTGTTTTTCTTGGTCGGTGGTTCAACTCATCTTG
AACCCTGCGTATCTCCCGATCACTGATGTTACGGAAATCGGTTTGTTTGGGGAAGTATTG
CCGGATGAGTCCGTTGGTGTTCTCATTCAGCCCTTTCTCCCAAGAATGGTAAGGGCGACA
AAAATAAGTCTCCGCTTTCAATGCTTTGGTTATTTTGGTGTGTTGGTAGAACTCTTTGCC
GTTATCCATGGTAATGGTGTGCACCCTGTCTTTATGTGCCTTTAATGCCCTAACAGCTGC
CCGGGCAGTGTCTTCGGCTTTGAGGCTATCCAATTTGCAGATGATGGTGTAGCGGGTAAC
GCGTTCGACCAAGGTCAATAATGCGCTTTTCTGTCCTTTGCCGACAATGGTGTCGGCTTC
CCAATCGCCGATACGGGATTTCTGGTCGACGATAGCGGGTCGGTTTTCTATGCCGACACG
GTTGGGTACTTTGCCTCTGGTCCATGTGCTGCCGTAGCGTTTGCGGTAGGGTTTGCTGCA
TATTCTGAGATGTTGCCACAACGTGCTGCCGTTGCTTTTGTCTTGGCGAAGGTAGCGGTA
AATGGTGCTGTGGTGGAGCGTGATCTGGTGGTGTTTGCACAGGTAGGCGCATACTTGTTC
GGGACTGAGTTTGCGGCGGATAAGGGTGTCGATGTGCTGAATCAGCTGCGAATCGAGCTT
ATAGGGTTGTCGCTTACGCTGTTTGATAGTCTGGCTTTGCCGCTGGGCTTTTTCGGCGCT
GTATTGCTGCCCTTGGGTGCGGTGCCGTCTGATTTCGCGGCTGATGGTGCTTTTGTGGCG
GTTCAGCTGTTTGGCGATTTCGGTGACGGTGCAGTGGCGGGACAGGTATTGGATGTGGTA
TCGTTCGCCTTGGGTCAGTTGCGTGTAGCTCATGGCAATCTTTCTTGCAGGAAAGGCCGT
ATGCTACCGCATACTGGCCTTTTTCTGTTAGGGAAAGTTGCACTTCAAATGCGAATCCGC
CGACCTCTTTCAGTTACAGCAGCTTGATCCCTTTCCCTTATCCAACGGGGGAAGGCTAGG
ATAGGGTGGCTTGCAAATATACAGAACAAGGGACAAGAGCCACCCTCTCTCCAACCCTCT
CCCTCCGTACGGGAGGGGGTGGATTCTCGCGGGCGAAGCCCACGCTACGGTTAGCCTTTA
CCCCAGCACAAACAATTCCCGCCCGTGCGCCTTCAGCCAACTTTTAGCATTGTCGGTATG
CGGCGTCAGCGTGTTCACCAAATGCCAAAAGCGCGGACTGTGGTCGGGGTGGCGGAGGTG
GCAGAGTTCGTGGATGCAGACATAGTCGGCGACGTATTCGGGCGTGCCGATCAGCCGCCA
GTTGAGGCGGATGCCGGTGTGCGGGCGGCATACGCCCCAAAAGGTTTTGGCGTTGCTCAG
GTCTGTGGCGGTGGGCGTCAGTCCTGTTTCGGCTGCGTGTTTTTCAAGGCGGGGCAGCAG
GTATTCGCGGGCGCGTTCGTTCAACAGGCGGCGCAGGTGGTCGATTTGTGCGGCGGTTTC
TTTTCGGGGAAGCAGGATTTCAGACGACGTGATACGGATATGGCTTTGGCTGTGGGTATC
CAGCTTGGTCTTTATTCCCCGATACCAAATCCACTCGGGTAAGTTTGGGTGGGAAACAGG
ATGCACGGGCGTTTTGGCAAGCGTGTTCCGCAAAATCGTTTCGTTTGCCGCCAGCCAGTT
TGCTAACGCGTGGTCTTGAAAAAAGGGTGGGACGTTGATGCTGACCGTCTGCATATTGAC
GGGGCGCAGAATCAGATTTTTCTTGGCACTGCGTTTGAGTTCGATTTCGATGCACAAACC
GTCGGAAAGAGTATAGGTGAAGCGTTTCATAGTTGTGAATAGGTTTCAGACCGGATACAT
CGTCTGAAACAGGAATTTTCCATATCAGGCGGCAAACTTCGGATAATATACAAAATCAAA
CATCTGCGCTACAAGGTTCAGCCGAACAAGCCGCCGATATATTTGCTGATGGTGATGGCG
CTGAGTACTGCCATCAAACCGACCACAATCACGCCGGAAACGGTGAGCCACAGCGGGTGT
TTGTAGTCGCCGACAATTTTGGTTTTGTAGGCGGCAATCAGAATCAGACCGAGGGAAATC
GGTAAAATCAGGCCGTTTAATGCGCCTACGAACACCAGCACCTGCGCCGGTTTGCCGATG
GTGGAAAATACGGCGGTGGACACGGCGATAAAGGCAATAATCCATTTGTTTTTATTGCGT
TCGATAGACGGGCTGAGACCGGAGAAGAACGACACCGAAGTATAAGCCGCACCAATCACC
GAAGTAATCGAAGCCGCCCAAATCACCACGCCGAAAATCAGCAGGCCGATGTATCCCGCC
GCATATTCAAACGGTGTGGAAGCAGGGTTGTCGGGATTGAGCTGTACGCCTTGGCTGACC
ACGCCCAAAACCGCCAAAAACAATACAATCCGCATAATCGAGGCAATCAGGATCGCCCGC
ACCGAGCTTTGGCTCACTTCCGGCAACGCCGATTTGCCTTTGATACCTGCGTCCAGCAGA
CGGTGCGCACCGGCGAAGGTGATGTAGCCGCCGACCGTGCCGCCCACCAGTGTAACAATC
GCCATTGCATCGAGTTTTTCCGGCATAAAGGTATGCACGGCGGCATCTGCCAGCGGCGGA
TTCGCCTGCCATGCCACATAAACCGTCAGCGCAATCATTACGAAACCCATCACTTGGGCG
~~AATTTGTCCATCACTTTGGCTGGTTCTTTAAACAGAAACACACCGATGGCAATCACGCCG~~
CTGATCACGGCACCGGTTTCCGGTGACAGTCCGGTCAGCAGGTTCAGACCCAAGCCTGCG
CCGCCGACGTTGCCAATATTGAACGCCAAACCGCCCATCACAATCAGCACAGCCAAGAAA
TAGCCTGCGCCGGGCAAGACCTGATTGGCAATATCCTGCGCCTGTTTTTCGGAAACGGCG
ACAATCCGCCAAATATTGAGCTGCGCCCCGATGTCGAGCAGAATCGAGAGCAGAATCACA
AAGCCGAAACTTGCCGCCAGTGCTTGGGTGAAGGTGGCGGTTTGGGTCAGAAAGCCCGGG
CCGATGGCGGAAGTCGCCATCAGGAATGCAGCGCCGATTAAGGCATTTCTGCGGTTTTTT
TGATCAGACATAATCGCTTATCCTCTATAAAATTGGTTGTTGCTGTGTTTGGGCGAAACC
TGCGGTTTTAGCTACGCAGAAACTCGCTTTGCTCGTTTTGGCGAAACCTGCGGTTTTCAG
ACGGCCTATGAACTGTTTTTCAAGCAGAAACTTTGATGCCTGCCGCCAGTAGTTCCTGCC
GGATTTTTTCGGCAAACACCACGGCGTGCGGCCCGTCTCCGTGCAGACAGATGCTGTCGG
CTTGCACGGCAACCAGGCTGCCGTCCACTGCTTTGACCTGCCCGTCCCGCACCATCTGCA
ATACTTGGGCGATGGCTTCTTCGTCGCTGTCCACCTGCGCATCGGGGCGGCTGCGGGGAA
CCAGCGTACCGTCGGGCATATAGCGGCGGTCGGCGAATACTTCGGAAATCACACCCAAGC
CTGCGGCTTTTCCGGCTTCCAAGAGCAGGCTGCCGGAAAGTGCCATCAATTTCAATTTCG
GGTCGAAATCCGCCACAATTCGGGCAACGGTATCCGCCAGCGCACGGTTTTTCGCCGCTT
GATTGTACATTGCGCCGTGCGGTTTGACATAAGCCATTTCCAAACCCTGATCACGGCACA
AGGCCTGCAATGCGCCCAACTGGTAATTCAGACACGCCCGCAAATCGGCTTCGGACAGAT
TCATTTCGGTACGGCCGAAGTTTTCCCGATCGGGATAGCCGGGGTGTGCTCCGATGCGCA
CGCCGTTTTGTTGGGCATACGCCAATGCCGCCCGAATATCGGCAATGCTGCCGGCGTGTT
GGGCGCAGGCGATGTTGGCCGAAGTAATCAGCTGCAACAAGGCTTCGTCGCTGCCGCAGC
CTTCGGCGAGATCGGCGTTTAAATCAACCTGCTTCATGGGTGATTCTCCGTATTTGGTTC
AGATAGGCTTGTTTTTGCGCCGCAGGGCGGTGGCTTCTTTCAAGCCGATTATTTTGAATT
TGACTTTGCTGCCGAAGCGCACCTGTGCCAGCCTGCCCAAATCGGCGGCGGCAACGGTAG
CGATTTTCGGATAACCGCCGGTGGTTTGCGCATCGGCCAGCAGGATAATCGGTTTGCCGC
CGGGCGGCACCTGCACGGTTCCTGCCTGAACAGCGTGGGACAGCATTTCCAAAGGTTGCG

# Appendix A

ACAGGGTCAGCGGCTGTCCGTCGAAGCGGTAGCCCATGCGGTTGCTATCGCTTTGCAGCG
TCCACGTTTCCCGTTCCAGATTCAGACGCCCTTTTTCACTGAAAGCGGCATATTCCGACG
AAGGAACAAGGTGGACGGTATCGGTAAACGGTATCGGGGCAATGCCGACTTTGGACAATT
CCTGCGCACCTTTGCCGATGGGGAGATAATCGCCTTTTTGCAGCATTCTGCCCTGATGGC
CGCCGAAACCGGCTTTCAGGTCGGTGCTTCTCGAACCCATCACTTCCGGCACATCAAATC
CGCCCGCCACGCACACATAGCCGTACATGCCCTGCACGGCACGCACCAGTTTCAAGGTCT
GCCCTTTGCGGGCGGTATAACGCCAATACGAATAGACCGGTTCGCCGTCCAATTCCGCCT
GATACACGGCACCGGTGAGACAAAACGGCGTATCCCGTTCAAACACCAGCATTATCCCGC
CCAAAGCGATTTCGATTGCGGCCGTGCCTTCGTCGTTGCCCAATAAAATATTGCCCGCCG
CCAAAGCAACCGTGTCCATCGCACCGGCATGACCGATGCCGTAACGCCGGTGTCCGTAGC
GTCCGGTATCCTGAATATGCGCCGGTGCCTGCACTGCCGAAACGTGAATCATGGCTCAAT
CCTTTCTGCAACAAAGCGGACTTGGTCACCCGCCGCCAGCAGGGTCGGCGGATTCAAATC
GGCTCGGAACAAGGGTAATTCGGTTCTGCCGATAATCTGCCAGCCGCCGGGCGAAGCGAA
CGGATACACACCGGTCTGACTGCCGCCGATACCGACCGAACCGGCAGGAACGGACGTTCT
CGGCACGGCACGGCGGGGCGTGTGCAATGCTTCGGGCAAGCCGCCCAGATAAGGGAAACC
GGGCTGGAAGCCCATCATAAATACGGTATAAGTTTGCGCCGTATGGCGGCGGACGATTTC
GGAAATAACCGTCTGATGGAAAGCAGCGACTTCCGCCAAATCCGGGCCGTATTCGCCGCC
GTAGCAGACGGGAATTTCCACCAGTTTGCCCTGATGGTCTGTAACGGCGGTGTGTTCCCA
CACATATTGCAATTCATCGGCAAGCGTCGCCAAATCGGTATCGAAACGGGTAAACACGGT
CAGATTGTTCATGCCGACCACCACTTCCTCAATCCTGTCGTGCTGCCCGAGCGCAGCGGC
AAACGCCCACAACTTTTGCTGTTTGCCCAGTTCGGAAGGCGCATTCAGTCGGTAGACCAA
AGCGGATTCGCTGATTGGTGTGATCTCTATTCTCATTTGTTGTTCATTTTGGTTATGTTT
TAATGAATCTATATGCAGGGGCGGCGGTTTGTCAATATCTTCTGTGCTGCATCATCAAAC
CGTCGATTGGAAAAGTGCTGCCCTGCCGCTGCACTTTTTCAGACGACCTTAAACCGTTTC
TATTAAAATAGCGCATTCCACTTTTCAGACGGCATCCTTATGTTTCCCGACCAATCCGCC
CCCAACCTGCTGCAAGGCTTGAATCCCGAACAACTCTCCGCCGTAACCTGGCCGCCGCAA
TCCGCACTTGTGCTGGCGGGCGCGGGCAGCGGCAAAACGCGCGTGCTGACCACGCGCATC
GCATGGCTGTTGCAAAGCGGACAAGCCAGCGTGCACAGCATTATGGCGGTAACGTTTACC
AACAAAGCCGCCAAAGAAATGCAAACCCGTTTGGGCGCGATGATTCCCATCAATGTCCGC
GCCATGTGGCTCGGCACGTTCCACGGTCTCTGCCACCGCTTTTTGCGCCTGCACCACCGC
GACGCCGGTCTGCCGTCTTCCTTTCAAATCCTCGACGGCGGCGACCAGCTTTCCCTCATC
AAACGCCTGCTCAAAAGCCTCAACATCGCCGAAGAAATCATCGCGCCGCGTTCGCTGCAA
GGCTTTATCAACGCGCAAAAAGAATCCGGTTTGCGCGCTTCCGTGTTGAGCGCGCCCGAT
CCGCACACACGCCGCATGATTGAGTGCTACGCCGAATACGACAAAATCTGCCAACGCGAA
GGCGTGGTCGATTTTGCCGAACTCATGCTCCGCAGCTACGAAATGCTGCAAAACAACGAA
ATCCTGCGCCAGCACTACCAAAACCGCTTCAACCACATTCTCGTTGACGAGTTCCAAGAC
ACCAACAAACTGCAATATGCTTGGCTGAAACTGATTGCCGGCAACCACGCAGCAGTATTT
GCCGTCGGCGACGACGACCAAAGCATTTACCGTTTCCGTGGCGCAAGCGTCGGCAACATG
ACCGCGCTGATGGAAGAATTCCACATCGACGCGCCCGTCAAACTCGAACAAAACTACCGC
TCCGTCGGCAACATCCTTGCCGCCGCCAATGCCGTGATTGAAAACAACGACGAACGACTC
GGCAAAAACCTGCGCACCGACGCCGAAGCAGGCGACAAAATCCGCTACTACTCCGCCTTT
ACCGACCTCGAAGAAGCCCGGTTCATCTTGGACGAAACCAAAGCCCTCGAACGCGAAGGC
TGGGATTTGGACGAAATCGCCGTCCTCTACCGTAGCAACGCCCAATCCCGCGTTATCGAA
CAAAGCCTGTTCCGCAGCGGCATTCCCTACAAAATCTACGGCGGCTTGCGTTTTTACGAA
CGCCAAGAAATCAAACACGCGCTCGCCTACCTGCGCCTCGCCGTCAATCCCGACGACGAC
AACGCCCTCTTGCGTGTCATCAACTTCCCACCGCGCGGCATCGGTGCACGTACCGTCGAA
AATCTTCAGACGGCCTCAAACGAACAAGGCATCACCCTCTGGCAAGCCGCCTGCAACGCC
GGCGCGAAAGCCGCCAAAGTCGTCGCCTTCGTCCGCCTGATTGAAGCCCTGCGCAACCAA
GTCGGACAACTGTCCCTGTCCGAAATCATCGTCGGCATCCTCAAAGACAGTGGCTTGACC
GAACACTACCGCACCCAAAAAGGCGACAACCAAGACCGTCTCGACAACCTTGACGAACTC
GTCAACGCCGCCATCGAATTCAAACCCGAAGACAGCAACTTCGAAATCCTGCCTGAAAAC
ATTTCAGACGACCCCGCCTTCCCCATTCTCGCCTTCCTAAGCAATGCCGCCCTCGAATCC
GGTGAAAACCAGGCAGGCGCAGGCGAAAAGGCCGTCCAACTCATGACCGTCCACGCCGCC
AAAGGCTTGGAATTTAACGCCGTCTTCCTCACCGGCATGGAAGAAGGCCGCTTCCCCAGC
GAAATGAGCCTTGCCGAACGCGGCGGCCTCGAAGAAGAACGCCGCCTCATGTACGTCGCC
ATCACCCGCGCCCGCAAACGCCTCTACATCACCATGGCGCAACAACGCATGCTGCACGGA
CAAACCCAATTCGGCATCGTCTCCCGCTTCGTCGAAGAGATCCCACCCGAAGTATTGCAC
TACCTGTCCGTCAAAAAGCCTGCCTACGACAGTTACGGCAACACGCGCCAAACCGCCGCA
TCCAAAGATAAAATCATCGACGACTACAAACAGCCCCAAACCTACGCAGGTTTCCGTATC
GGACAAAACGTCCGCCACGCCAAATTCGGCACCGGCGTGATTATCGATGCCGCAGATAAA
GGCGAATCCGCCCGACTGACCATCAATTTCGGCAAACAGGGCGTGAAAGAGTTGGACACC
AAGTTTGCGAAATTGGAAGAGATGTAAATTTGAAATGTAGGTCGGATATTCGTATCCGAC
CTACGGCAAAAACCTTAGCAGGAGAGAATAGAAACCCGTAGCGTGGGCTTTTTCTATGAA
TCAAGCCCAAAATTTCAGACGGCATTTTTAGCCGTCATTATCGTGGATGAAGCCCACGCT
ACAATGTACACACAGAGCAAATAGAGATGTGGGTCGGATATTCGTATCCGACAAAAACAT
TTGACGCGTCTATTGTTTCCGAAACACCGCTGTTGGAAATGTCGGATACAAGAATCTGAC
TTACGGCAAAAAACGTAGTAAGGACAAAGCAAAAGGCCGTCTGAAAACGGGAAGGGCAAT
TTTGCCGCAACCGCCGCCGTCATTCCCGCGCAGGCGGGAATCCAGACCTTTCGGCACGGA
AACTTATCGGATAAAAGGTTTCTTTAGATTCCACGTCCTAGATTCCCGCCGGAACATAAA
TGACGGACGGTAAAAGCCGGGTATGAATACCCACCCTCTGTTATCACTGAGATCAATAAG
GAAGAACATTATGTCCCAAGTTTTTAAAGATTTTGACTTGTCCTCCGTATGGAAAACTAA
TAGTTGGGCAGATGAAAACTACAAAGAAGCCCCGTTTACCCCTGAAATTTTGGCTGCCGT
AGAAAGTGAACTGGGCTATAAATTGCCGCAAAGTTTTATTGAATTGATGGCAGTACAAAA
CGGCGGAATATTTGTCAAAAACTGTTTTCCGACCACGCAGAGAAATTCGTGGGCGGAAAA
TCATGTGCAAATTTGCGAGGTATCGGGAATCGGTTTTGAAAAAGAAGGGAGTTTGTGCGG

# Appendix A

CGCGATGGGGCAAAAACTTTGGCTGGAAGAATGGGAATACCCGCCTATCGGCGTGTATTT
TGCCAACGACCCGTCAGGCGGTCATGCCATGTTTGCCTTAGACTATCGGGCGTGCGGCAA
AGACGGCGAGCCGAAAGTGGTGTTTGTCGAACAAGAATCGGATTTTGAAATCGTCGAACT
TGCCCCCGATTTTGAAACCTTTATCCGCAGCTTGCGGCATGAAGATGAGTTTATTGACGA
AGAAATATAAAACGGTGGTTGAAAAACTGAAATCATCAAGAGAAAACGGGCGAAATAACG
GGTAATCGCTTGAATCCGTAAGGAAAACGGTTTGGTGGAACGCGCCATCCAAGACCTTTG
CAAAAAACTGTCCCCGACAGCATTGACATTATTAACAGAACTTATCAATTTTGGAGCTAT
CTCAAATATAATTCGGTTATCCTGTTGTATCCATTAAATCATATGCTTCAATTAATTGTT
GTTCTAGCTCTTATACCAATTTTGGATTGCGAATTCCTGACACAATCTCAAATTCTTCTG
CATCTATGCAAACACCTGCATAAATTTCAATAACAAGGGAACGCAATAATTGAAGCTCTT
CTCTTGTTAAAGAAATAATAATGTCATCACCTTTGTAATTGATTATATTCATAATAATTT
TATTTTTGTTTGTCAAAGTAAGTTTTGCCTAAGGTTGGTCTAAATGCAGTTCCACCATCT
TTTGAATTTGGGTCTCTGATTACAATTGCTCCAGACTTATCATCCCAAATTGCTCTTATG
TGTTTGGATTGTAATCTTCGAATTCCCAAGAAAAAAATCGTAATAAGTTTGAAAGTGTCA
AATCCCAAGTTTCTTTTGAGCAATATTCTAATATTTTATCAATTTCACTTTTAATAATCT
TATGATCAAACTGTTCTAATATTAATGCATTAGACCAAAAAAAACCTTCTTTATTACAAT
GATGGGAAATCCATTTAGGAGAACAAATGCAAAGTGAAAAAATAGATGAGCCTTGTTCTC
CTTCGATTCCGATATCCAAATCTATCCATCTATGGAAATTATCTGGAATTTCGGGGGTAA
ATTTTTCAAAATCAATATCATATAAATTTATGCTTTTTAAATCCAATTTAATCATTAGGG
CTGTCCTAGATAAATAGGGAAATTCAAATTAAGTTAGAATTATCCCTATGAGAAAAAGTC
GTCTAAGCCGGTATAAACAAAATAAACTCATTGAGCTATTTGTCGCAGGTGTAACTGCAA
GAACAGCAACAGAGCCCGACAGCATTGTTTATACGGATTGTTATCGTAGCTATTCATTTA
CGCAAGTTTAACGGCATTCCCAAAGCGCATTTTGAGCTGTATTTAAAGGAGTGCGAATGG
CGTTTTAACAACAGTGAGATAAAAGTTCAAATTTCCATTTTAAAACAATTAGTAAAATCG
AGTTTATCTTAGTTGTCCAGGACAGCCCCATTATTTTTATAACACCGTGAAGCCGCACAG
CAGTTTGAACAGTGATACGCCGTTTGCGGGCTTACGAGTTTATTTTCCCGGCCTGCAGTT
TGAGCAATACGGTGATTTCCTACGGTTAATACAAATGTTTACACATTGATACATTTCATT
TATAGTTCCGCCTATTTGAAAATAGAAAATATGAATTCGACCGCAAGTAAAACCCTGAAA
GGATTGTCGCTGGTGTTTTTCGCCTCTGGATTCTGCGCCCTGATTTACCAGGTCAGCTGG
CAGAGGCTTCTATTCAGTCACATAGGTATCGATTTGAGTTCGATTACTGTCATTATTTCT
GTATTTATGGTCGGCTTGGGTGTAGGTGCGTATTTCGGTGGACGCATTGCTGACCGTTTT
CCTTCAAGTATCATCCCCCTGTTTTGCATCGCTGAAGTATCCATCGGTCTGTTCGGTTTG
GTAAGCAGGGGTCTGATTTCCGGCTTGGGGCATCTTTTAGTTGAGGCTGATTTGCCCATC
ATCGCTGCTGCCAATTTCCTCTTATTGCTGCTTCCTACCTTTATGATGGGCGCGACCTTG
CCCTTGCTGACCTGTTTTTTTAACCGGAAAATACATAATGTTGGCGAGTCTATCGGTACC
TTATATTTTTTCAACACTTTGGGTGCGGCACTCGGATCGCTTGCCGCCGCCGAATTTTTC
TACGTCTTTTTTACCCTCTCCCAAACCATTGCGCTGACAGCCTGCTTTAACCTTCTGATT
GCTGCTTCAGTATGGCTGCGTTACAGAAAGGATGGATATAGTGAACACTAAACCGAATAC
TAGTTTGATTTATATGCTTTCTTTCCTTAGCGGCTTATTGAGCTTGGGTATAGAAGTCTT
GTGGGTGAGGATGTTTTCGTTCGCAGCACAGTCCGTGCCTCAGGCATTTTCATTTACCCT
TGCCTGTTTTCTGACCGGTATCGCCGTCGGCGCGTATTTTGGCAAACGGATTTGCCGCAG
CCGCTTTGTTGATATTCCCTTTATCGGGCAGTGCTTCTTGTGGGCGGGTATTGCCGACTT
TTTGATTTTGGGTGCTGCGTGGTTGTTGACGGGTTTTTCCGGCTTCGTCCACCACGCCGG
TATCTTCATTACCCTGTCTGCCGTCGTCAGAGGGTTGATTTTCCCGCTCGTACACCATGT
GGGTACGGATGGCAACAAATCCGGACGACAGGTTTCCAATGTTTATTTCGCCAACGTTGC
CGGCAGTGCATTGGGTCCGGTCCTTATCGGCTTTGTGATACTTGATTTCTTGTCCACCCA
ACAGATTTACCTGCTCATCTGTTTGATTTCTGCTGCTGTCCCTTTGTTTTGTACACTGTT
CCAAAAAAGTCTCCGACTGAATGCAGTGTCGGTAGCAGTTTCCCTAATGTTCGGCATCCT
CATGTTCCTACTGCCGGATTCTGTCTTTCAAAATATTGCTGACCGTCCGGATAGGCTGAT
~~TGAAAACAAACACGGCATTGTTGCGGTTTACCATAGAGATGGTGATAAGGTTGTTTATGG~~
GGCGAATGTATACGACGGCGCATACAATACCGATGTATTCAATAGTGTCAACGGCATCGA
ACGTGCCTATCTGCTACCCTCCCTGAAGTCTGGCATACGCCGCATTTTCGTCGTTGGACT
GAGTACAGGTTCGTGGGCGCGCGTCTTGTCTGCCATTCCGGAAATGCAGTCGATGATCGT
TGCGGAAATCAATCCGGCATACCGTAGCCTTATCGCGGACGAGCCGCAAATCGCCCCGCT
TTTGCAGGACAAACGTGTTGAAATTGTATTGGATGACGGTAGGAAATGGCTGCGTCGCCA
TCCTGATGAAAAATTCGACCTGATTTTGATGAATACGACTTGGTACTGGCGTGCCTATTC
CACCAACCTGTTGAGTGCGGAATTTTTAAAACAGGTGCAAAGCCACCTTACCCCGGATGG
TATTGTAATGTTTAATACCACGCACAGCCCGCATGCTTTTGCTACCGCCGTACACAGTAT
TCCCTATGCATACCGCTATGGGCATATGGTAGTCGGCTCGGCAACCCCGGTAGTTTTCCC
TAATAAAGAACTGCTCAAGCAACGTCTCTCCCGGTTGATTTGGCCGGAAAGCGGCAGGCA
CGTATTTGACAGCAGCACCGTGGATGCTGCAGCACAAAAGGTTGTCTCTCGTATGCTGAT
TCAGATGACGGAACCTTCGGCTGGGGCGGAAGTTATTACCGACGATAATATGATTGTAGA
ATACAAATACGGCAGAGGGATTTAACCGTCTTAAAGGGTTTCAGGCAACGCAGGTTTTAG
GTAACGTCCTGCTAGTTCAAAAAAACCGCATCACAGCAGTCGGGACAAAATGGTTTAAAC
ATTTTGTCCCGAATTCTTATTCCTATATATAGTGGATTAACAAAAATCAGGACAAGGCGA
CGAAGCCGCAGACAGTACAAATAGTACGGAACCGATTCACTTGGTGCTTGAGCACCTTAG
AGAATCGTTCTCTTTGAGCTAAGGCGAGGCAACGCCGTACTGGTTTTTGTTAATCCACTA
TACCACGAATTACGGTGTAAAAATTTATATGACCTTATAAAATCAAATAAGAATCGTTAT
CATAACATGATTGTATTTATTGGGTTTTTTTGGGCGTTTTGCCGATATTTACCTTTTAAT
GGTTTTTGAAATTCGCTAAAATACGAAATTATTGTAGAAATTTTGTTAACGGATTTGGGT
GTAACCATGTTGTCCGCTTACTTTCCCGTCTTTGTCTTTATCCTCATCGGCCTCGCGGCC
GGCGTGCTGTTTATCCTGCTCGGCACGATTTTAGGCCCGAAACGCCACTATGCCGAAAAA
GACGCGCCTTACGAATGCGGTTTTGAAGCTTTTGAAAACGCCAGGATGAAGTTCGACGTG
CGCTATTACCTCGTCGCCATCCTCTTCATCCTGTTTGATTTGGAGGTCGCGTTTATGCTG
CCGTGGGCAGTCGTGTTCAAAGATTTGGGCGCGTACGGCTTCTGGTCTATGCTGGTGTTT

## Appendix A

ATCGTTGTTCTGACGGTAGGCTTTGTTTACGAATGGAAAAAAGGTGCGCTGGAATGGGAA
TAGAAGGCGTTTTGAAAAAAGGTTTCATCACCACCAGCGCGGATACGGTGCTGAACTATA
TGCGTACCGGTTCGTTGTGGCCGGTTACTTTCGGCTTGGCCTGCTGCGCCGTGGAAATGA
TGCACGCGGGTATGGCGCGTTACGACCTTGACCGTTTCGGTATTATTTTCCGTCCGTCCC
CCCGTCAGGCCGACCTGATGATTGTGGCGGGTACGCTCACCAATAAAATGGCGCCCGCCC
TGCGCCGAGTGTACGACCAGCTCGCCGAGCCGCGCTGGGTATTGTCTATGGGCTCATGTG
CCAACGGCGGCGGCTATTATCACTATTCTTATTCCGTTGTGCGCGGTGCCGACCGCGTCG
TGCCGGTAGATGTTTATGTGCCGGGTTGTCCGCCGACTGCGGAAGCCCTGATTTACGGCC
TGATTCAGCTCCAACAAAAAATCAAGCGCACTTCCACCATTGCGCGTGACGAGTAAGGAG
AGGACGATATGGCAAGCATTCAAGACTTATACGAAACCGTCAGCCGCGTTTTGGGCAATC
AGGCAGGCAAAGTCATTTCCGCTTTGGGCGAGATTACCGTCGAGTGTCTGCCCGAGCACT
ATATTTCAGTCATGACCGCATTGCGTGACCATGAAGAGTTGCATTTCGAGCTTCTGGTTG
ACTTGTGCGGTGTCGATTACAGCACTTACAAAAACGAAGCATGGCAGGGCAAACGCTTTG
CCGTCGTCAGTCAGTTGCTTTCCGTTAAAAACAATCAACGCATCCGCGTGCGCGTCTGGG
TTTCAGACGACGACTTCCCCGTAGTCGAATCTGTAGTCGATATTTACAACAGCGCGGATT
GGTACGAACGCGAAGCCTTCGATATGTACGGCATCATGTTCAACAACCATCCGGACTTGC
GCCGCATCCTGACCGATTACGGCTTCGTCGGACATCCGTTCCGCAAAGACTTCCCGATTT
CCGGCTATGTGGAAATGCGTTACGACGAAGAGCAAAAACGCGTGATTTACCAACCTGTTA
CCATTGAGCCGCGCGAGATCACGCCGCGTATCGTCCGTGAGGAGAACTACGGTGGCCAAT
AAATTAAGAAACTACACCATCAACTTCGGCCCGCAACACCCTGCGGCGCACGGCGTATTG
CGTATGATTTTGGAGCTGGACGGCGAACAAATCGTCCGTGCCGACCCGCATATCGGCCTC
TTGCACCGAGGTACCGAAAAACTGGCGGAAACCAAAACCTATCTGCAAGCCCTGCCCTAT
ATGGACCGCTTGGACTATGTTTCCATGATGGTCAATGAGCAGGCGTATTGTTTGGCAGTA
GAAAAACTTGTCGGTATCGATGTGCCCATCCGCGCCCAATACATCCGCGTGATGTTTGCC
GAAGTAACGCGCATCCTCAATCACTTGATGGGCATCGGTTCGCATGCCTTCGACATCGGC
GCGATGACCGCCATTCTTTACGCCTTCCGCGACCGCGAAGAGCTGATGGACTTGTACGAA
GCCGTGTCCGGCGCGCGTATGCACGCCGCCTACTTCCGTCCCGGCGGCGTTTACCGCGAC
CTGCCCGACTTTATGCCCAAATACGAGGGCAGCAAATTCCGCAATGCCAAAGTATTGAAG
CAGCTCAACGAATCCCGCGAAGGCACCATGCTCGACTTTATCGATGCCTTCTGCGAACGC
TTCCCCAAAAATATCGACACACTCGAAACCCTCCTGACCGACAACCGTATTTGGAAACAG
CGTACCGTCGGCATCGGCGTCGTCTCCCCCGAACGTGCCATGCAAAAAGGCTTTACCGGC
GTGATGTTGCGCGGTTCGGGCGTGGAATGGGACGTGCGTAAGACACAGCCTTACGAAGTG
TACGACAAAATGGATTTCGACATCCCTGTCGGCGTGAACGGCGACTGCTACGACCGCTAC
CTCTGCCGTATGGAAGAAATGCGTCAATCCGTACGCATCATCAAACAATGTTCCGAGTGG
TTGCGTGTCAATCCGGGTCCGGTCATTACCACAAACCACAAATTCGCTCCGCCCAAACGT
ACCGAAATGAAAACAGGTATGGAAGACCTGATTCACCATTTCAAACTCTTTACCGAGGGT
ATGCACGTTCCCGAGGGCGAGACCTACACCGCTGTCGAACATCCGAAAGGCGAGTTCGGC
GTTTACATCATTTCAGACGGCGCAAACAAACCCTACCGCCTGAAAATCCGCGCACCCGGC
TTCGCCCATCTGCAAGGCATGGACGAAATGGCAAAAGGCCACATGCTCGCCGACGTCGTT
GCCATCATCGGTACGCAGGACATCGTATTCGGGGAGGTTGACCGATAATGTTATCCGCAG
AATCTTTAAAACAAATCGACATCGAGTTGGCAAAATATCCTGCCGACCAACGCCGCTCCG
CGATTATGGGCGCATTGCGTATTGCCCAAACCGAAAAAGGCTGGCTTGCTCCCGAGACCA
TCGCTTTTGTCGCCGACTACATCGGCATCACGCCTGCACAAGCCTACGAAGTCGCCACTT
TCTACAATATGTACGACCTTGAGCCTGTCGGCAAATACAAACTGACCGTTTGTACCAACC
TGCCCTGCGCCCTGCGCGGCGGTATGGCTACCGGCGAATACCTCAAACAAAAACTCGGTA
TCGGCTACGGCGAAACTACCCCTGACGGCAAGTTTACCCTTGTCGAAGGCGAATGCATGG
GCGCATGCGGCGACGCTCCCGTTATGCTGGTCAACAACCACAGCATGTGCAGCTTTATGA
CCGAAGAAGCGATTGAGAAGAAACTGGCGGAGTTGGAGTAGGTCGTCTGAAACGACGATT
TAAACGTAGGTCGGATACTTGTAGCCGACAGAGTGGGTAAAAAGGCAAAATGTCGGATTT
AAGAATCCGCCCTACTGAAATACCGAAATGCCGTCATTCCCGCGCAGGCGGGAATCCACC
GGTAAGATTCGGTTTCTGAATTTAATAAGACATTGCTTACCATTGAGGATGGATTCCCGC
CTGCGCGGGAATGACGACAGACAAGCAAGTGGTCGAGATCCAACAAAAACGATTAAAGGT
CGTCTGAAAATATCGATTTGATAAACTAGATTTTATTTCAGACGACGTTACAAGCCGGTA
CACAAAGACATCTTAAGGTCGTCTGAAACAGCGGCCGCAACCGATACGAAAACAAACAGG
CACACCAAAAATGGCTATTTACCAATCAGGCGTGATTTTTGACCAAGTGGATACCGCCAA
TCCCGATTGCTGGACATTGGACGAATACGTCAAACGCGGCGGCTATACCGCCCTGCGTAA
AATTCTGTCCGAAAACATCTCGCAAACCGATGTGATTGACGAAGTCAAAACCTCCGGTTT
GCGCGGGCGCGGCGGTGCGGGCTTCCCGACCGGTTTGAAATGGAGCTTTATGCCCCGTTC
TTTCCCGGGCGAAAAATATGTGGTTTGCAACACCGACGAAGGCGAACCAGGTACGTTTAA
AGACCGCGACATCATCATGTTCAATCCGCATGCCCTGATCGAAGGCATGATTATCGCCGG
TTACGCGATGGGCGCGAAAGCCGGTTACAACTATATCCACGGCGAAATTTTTGAAGGCTA
CCAACGCTTTGAGGCCGCTTTGGAGCAGGCGCGTGCCGCAGGCTTTTTGGGTAAAAATAT
TTTGGGTTCGGATTTTGAATTTGAACTCTTCGCCCACCACGGCTACGGCGCATATATTTG
CGGCGAGGAAACCGCATTGCTCGAATCGCTGGAAGGCAAAAAAGGCCAGCCGCGCTTTAA
GCCGCCATTCCCTGCTTCGTTCGGCCTGTACGGCAAACCGACTACCATCAACAATACTGA
AACGTTCTCCTCCGTTCCATTCATTATCCGTGACGGTGGACAGGCATTTGCCGATAAAGG
TATTCCGAATGCAGGCGGTACCAAATTATTCTGTATTTCCGGCCATGTCGAGCGTCCGGG
CAACTATGAAGTGCCATTGGGTACGCCGTTTGCCGAAGTCTTGAAAATGGCGGGCGGTAT
GCGCGGCGGTAAAAAACTCAAAGCCGTCATTCCCGGCGGTTCGTCCGCGCCCGTATTGCC
TGCCGACATCATGATGCAGACCAATATGGACTACGACTCGATCTCCAAAGCAGGCTCCAT
GCTCGGTTCCGGCGCGATTATCGTCATGGACGAAGACGTGTGCATGGTCAAAGCCCTTGA
GCGTTTGAGCTACTTCTACTACGACGAGTCTTGCGGCCAATGTACCCCCTGCCGAGAAGG
TACGGGCTGGCTTTACCGCATCGTCCACCGCATCGTAGAAGGCAAAGGTAAAATGGAAGA
TTTGGATTTGCTGGATTCCGTCGGCAACCAAATGGCAGGCCGCACCATCTGCGCCCTCGC
CGATGCTGCCGTCTTCCCCGTCCGCAGCTTTACCAAGCATTTCCGTGATGAGTTTGTGCA

## Appendix A

TTACATCGAACACGGCGGGCCGATGAAAGAGCATAAGTGGGGAGGGTGGTAATGGTGGAA
GCTAAAATTTTTATTCTATACGGTGCAGCCAACAAAGGTAAGAGTACGACACTCAATACG
CTTTTTAATCAGATTTGTCGGAAATTTTCTAAATTTCTAGTCTTTTTTGAAAGACATGGA
AACGGCTTAGATTTTGTTGCAGTATTTGATCATGAAGGTCAGAGAATTGGTTTTTATTCA
TCTGGTGATAATGAATACGAGGTTAGGGGAAATTTATACAAACTTTATTCGCATAATTGT
GATTTTATTTTTGGCACGTCAAGGACACGGGGTGGTAGTTGCGATGCAGTAGGATGTTAT
GCAGAGTTATTGCATGGCGATGTAAATATAATTAATTGGTGTGAAAAGTTTGAGCCTACA
GATGAAGACAATGAGCGTGCTGTTAAAGAGTTATTTAAGTCATTTAAAAATATAATAAAT
GAGTTATAGTTTTAGTTGGTTTTATATTGGTTAAAAGCAAAATGCTAAAAATTTAACTTT
GCCGTCATTCCCGCGTAGGCGGGAATCCATAGTGGAATTTACAGAACCCGATATTTGAAA
AGCAGTTGCCGAAATTCAAAAAATGGATTCCCGCCTACGCGGGAATGACGGCGGGAGTAG
GCAGATGTTTTCAGATGAAAACGGTTGTAAATGATATTAAAAAAGTTGTTGTTTATATTG
CAGGAAAAATGAATACGAAACCATCCGCTTACTAGACAACCTGCCGTATATATTTTGGCA
AACGGTAAAAATGGAACACTCTATATCGGTGTTACCATGAATTTGCCGGAAAGGGTTTGG
CAGCACAAAAACCATGTCAATATTGATGGCTTTACTGCCCGATATGATGTGCATGATTTA
GTTTGGTATCAGTTTTTTGAGAATATGCCTGAAGCAGTTGCCAAAGAAAAAACGATGAAA
AAATGGCGACGTGAATGGAAGATTAAACTGATTGAAGAACAAAATACTGAATGATTGGAC
TTGTCGGGCGTGTTGTTTGTTTAGTTTTATTTCTGGAACTTTAAAAACTGTCGTTATTCC
AGCCCCACCTACGCGCAGACAGGCTACGGCGGGAATCACCGCAAAAGTTAAGAAACCAAT
GTTTGAAAACAGTTACCGAAAACCCAAGAATGGATTCACGCCTGTGCGGGAATGACGGCA
AGGTGGCAGTAAACGTTTTAAACAGTATTGATTGTCAATGAAACTCAAAAGGCCGTCTGA
AACCCATTTTTCAGACGACCTCCATAAAAGATTATTTATCAAATACCCGTAACTAGGAAC
GAACCATGTTACAAATCGAAATCGACGGCAAACAAGTATCTGTGGAGCAGGGCGCGACGG
TGATTGAAGCCGCGCACAAGCTCGGTACTTATATTCCGCATTTCTGTTACCACAAAAAAC
TTTCCATCGCCGCCAACTGCCGTATGTGTCTGGTGAACGTAGAAAAAGCCCCAAAACCCC
TGCCTGCCTGTGCCACGCCGGTTACAGACGGCATGATTGTGCGTACGCATTCGGCAAAAG
CCCGAGAGGCGCAGGAAGGCGTGATGGAGTTCCTGCTCATCAACCATCCGCTTGATTGTC
CGACCTGCGACCAAGGCGGCGAATGCCAGTTGCAGGATTTGGCGGTGGGCTACGGCAAAA
CCACCAGCCGCTACACCGAAGAAAAACGTTCCGTCGTCGGCAAAGATATGGGGTCCTTGG
TTTCCGCCGAGGAAATGAGCCGCTGTATCCACTGCACCCGCTGCGTTCGTTTCACTGAAG
AAATCGCCGGTTTGCAGGAAATTGCGATGGTGAATCGCGGCGAACACTCCGAAATCATGC
CCTTTATCGGCAAAACGGTGGAAACCGAATTGTCGGGCAACGTCATTGATTTGTGTCCCG
TCGGCGCGCTGACCAGCAAACCGTTCCGCTTCAACGCGCGTACTTGGGAATTGAACCGCC
GCAAATCCGTTTCCGCCCACGATGCTTTGGGCAGCAACCTGATTGTGCAGACCAAAGACC
ATACCGTCCGCCGCGTGTTGCCGTTGGAAAACGAAGCGATTAACGAATGCTGGCTGTCTG
ACCGCGACCGTTTCGCCTACGAAGGCCTGTATCACGAAAGCCGTCTGAAAAACCCGAAAA
TCAAACAGGGCGGCGAGTGGATGGACGTGGATTGGAAAACCGCGTTGGAATATGTCCGCA
GCGCGATTGAATGTATCGCCAAAGACGGCAAGCAAAACCAAGTCGGCGTTTGGGCGAACC
CGATGAATACGGTTGAAGAACTGTATCTGGCGAAGAAACTCGCCGACGGCTTGGGTGTTA
AAAACTTTGCAACCCGTTTGCGCCAACAAGACAAACGTCTTTCAGACGGCCTTAAAGGTG
CGCAATGGTTGGGACAAAGCATTGAATCTTTGGCTGACAACGATGCCGTATTGGTAGTCG
GTGCGAACTTGCGCAAAGAACAGCCGCTCCTGACTGCCCGCCTGCGCCGCGCCGCCAAAG
ACCGTATGGCATTGAGCGTATTGGCCAGCAGTAAAGAAGAATTGTTTATGCCGCTTCTGT
CTCAAGAAGCCGCACATCCCGACGAGTGGGCAGGCCGTCTGAAAAACCTGTCTGTCAATG
CGGAACACGCCGTTACCGCCAGCCTGAAAAATGCTGAAAAAGCAGCGGTGATTTTGGGCG
CGGAAGTGCAAAACCATCCTGATTACGCCGCGGTTTACGCCGCCGCGCAAGAGCTGGCTG
ACGCGACCGGCGCAGTGCTGGGCATTTTGCCGCAAGCCGCCAACAGCGTTGGTGCGGATG
TCTTGAATGTAAACTCCGGCAAGAGCGTTGTCGAAATGGTAAACGCGCCGAAACAGGCAG
TCTTGCTGCTCAACGTTGAGCCTGAAATCGATACGGCGGACGGTGCAAAAGCCGTAGCCG
~~CGTTGAAACAGGCAAAAAGCGTGATGGCGTTTACGCCGTTTGTCAGCGAAACGCTGCTGG~~
ACGTGTGCGACGTGTTGTTGCCGATTGCACCGTTTACCGAAACCTCAGGCAGCTTCATCA
ATATGGAAGGCCGTCTGCAATCCTTCCACGGCGTGGTACAAGGCTTCGGCGATTCGCGTC
CGCTGTGGAAAGTGTTGCGCGTATTGGGCAACCTGTTTGACCTGAAAGGTTTTGAATACC
ACGATACCGCTGCGATTTTGAAAGACGCGCTGGATGTGGAAAGCCTGCCGTCCAAACTGG
ACAACCGCAACGCATGGACAGGGGAGGGCGTTCAGACGACCTCAGACCGCCTCGTCCGTG
TCGGCGGCGTCGGTATTTATCACACCGATTCTATCGTGCGCCGTTCCGCACCGTTGCAAG
AAACCAGCCATGCCGCCGTGCCTGCTGCGCGTGTAAATCCAAATACATTGGCACGCTTGG
GCCTGCAAGACGGACAAACCGCTGTCGCCAAACAAAACGGCGCAAGCGTATCGGTTGCCG
TCAAAGCCGATGCCGGACTGCCTGAAAACGTGGTGCATCTGCCGCTGCATACCGAAAATG
CCGCGCTGGGTGCGTTGATGGACACTATTGAACTGGCGGGAGCTTGATTATGCAGGAATG
GTTCCAAAACCTCTTTGCCGCAACGCTCGGTCTGGGCGATTTGGGTATTACTGTAGGCTT
GGTGGTATCCGTCATCGTCAAAATTGTGATTATCCTGATTCCGCTGATTCTGACCGTCGC
CTACCTGACTTATTTCGAACGTAAAGTCATCGGCTTCATGCAGCTTCGCGTCGGTCCGAA
CGTAACCGGCCCGTGGGGTCTGATTCAGCCGTTTGCCGACGTGTTCAAACTCTTGTTTAA
AGAAGTAACCCGTCCGAAGCTGTCAAACAAAGCCCTGTTCTATATCGGCCCGATTATGTC
GCTTGCCCCGTCTTTCGCGGCGTGGGCAGTGATTCCGTTCAATGAAGAATGGGTGCTGAC
CAACATCAATATCGGTCTTTTGTACATCCTGATGATTACCTCGCTGTCGGTTTACGGCGT
GATCATCGCGGGCTGGGCTTCCAACTCCAAATATTCGTTCTTGGGCGCAATGCGTGCTTC
CGCGCAAAGCATTTCCTACGAAATCGCCATGAGTGCCGCGCTGGTGTGCGTCGTGATGGT
GTCGGGCAGCATGAACTTCTCCGACATCGTTGCCGCGCAGGCAAAAGGCATCGCAGGCGG
TTCGGTATTCTCTTGGAACTGGCTGCCGCTCTTCCCCATCTTCATCGTCTATCTGATTTC
CGCCGTTGCCGAAACCAACCGCGCACCGTTTGACGTGGCAGAGGGCGAGTCTGAAATCGT
TGCCGGTCACCACGTCGAATATTCCGGCTTCGCATTCGCGCTGTTCTTCCTTGCCGAATA
CATTTTCATGATTCTGATTGCCGCGCTGACATCGTTGATGTTCCTCGGCGGCTGGCTGTC
TCCCTTCCCGCAAAGCTGGGGCATTGTCGGTACGCCTTCCGCATTTTGGATGTTCGCGAA

# Appendix A

AATGGCGGCGGTTCTGTACTGGTATCTGTGGATACGCGCCACCTTCCCACGCTACCGTTA
CGACCAAATCATGCGCTTGGGCTGGAAAGTGCTGATTCCGATCGGCTTCGCCTACATCGT
GATTTTGGGCGTGTGGATGATTTCACCGCTGAATTTGTGGAAATAAGTTTCAGACGGCAT
CTTGAGGCCGTCTGAACAAAGCGATTTTGAATACCTAACGAAATCCCTGTTTTGAGGGAA
CATAATATGGCTAACTTAGTAAAAACCTTTCTGCTTGGCGAATTGGTAAAAGGTATGGGC
GTAACGCTCAAAAACTTTTTCGCCCGCAAAGACACAATTTATTTCCCCGAAGAGAAAACG
CCGCAATCCGTGCGTTTCCGCGGTCTGCACGCGCAGCGGCGGTATCCGAACGGCGAAGAG
CGGTGTATCGCGTGTAAGTTGTGTGAGGCAGTGTGTCCGGCAATGGCGATTAACATCGAA
TCGGAAGAACGTGAAGACGGTACGCGCCGCACCAAGCGTTACGACATCGACCTGACCAAG
TGCATCTTCTGCGGTTTCTGCGAAGAGGCATGCCCGACTGATGCGATTGTGGAAACCCAT
ATTTTTGAATACCACGGCGAGAAAAAAGGCGACTTGCACATGACCAAGCCGATTCTTTTG
GCCATTGGCGACAAATACGAAGCTGAAATCGCCAAACGCAAAGCCGCTGACGCGCCGTAT
CGTTAATGCTTTGGGGCTTCTTGGAAGGTTTTAAATATGGAAGGACTGATTAATGCATTG
AAATATTTAGCCGAACATGAGCCAATAGATAATTTTGAAGAAATTAGAACTAGAAATAGT
CCGATTGAGTTGCCAAGTGGATTAAGTAATTTTGAACAAAATATTTTTTTAAAAGAAAAT
TTATCCCCAAAATTACAAAATGATGATAGCTTGAAGACGCATTATTGGATTATCCGTGAA
TGGGGTGGGATTAAAAGTTTTAAACAATCTGCTGAAAATAGCCAGCTTATTCGTCAATTT
TTATCGGAACTTAATTCGGGAAAATTGAGTAGTGGTTTGTTGAAAATTTCATCATTATCT
AAATTGGCTTCTTTTATAGATTGTGAGCGATTCGCCATTTATGATTCACGCGCTATTTTT
TCGTTGAATTGGTTGTTGTTTAAATTTACAAATGCAGATTTGTTTTTCAGCCACAAGGT
AGAAATAGGGAACTAGAAATCCGAAATATGAACGTATTGTTTCATTTTTCTGATATCAAA
CCGAATTATCGGAAACCAGACGTTTCGTTTCATCAATATTGTGGGTTGTTACAAGATTTG
GCGAAACAAGTTTATGGTAAACAAGCAAAACCGTATCACATAGAAATGTTGTTATTCAAA
ATTGCGACAACGTGGATTTGTGCGGATATGGATCAACTGATTAAGTTTGATTGTTTGCGT
AACCAGGATTTTCAGACTGCTTGAAACCATATTTTTGATTAATAAAGAAAGCATAGACTA
TGACTTTCCAACTGATTTTATTTTATATTTTTGCAGTGATAATTCTTTATGGCGCGCTCA
AAACCGTCACCGCTAAAAACCCTGTTCACGCCGCTTTGCATCTGGTGCTGACCTTCTGCG
TGAGCGCGATGCTTTGGATGCTGATGCAGGCTGAGTTTTTGGGCGTGACGCTGGTGGTGG
TTTACGTCGGCGCCGTGATGGTGTTGTTCCTGTTCGTCGTGATGATGTTGAACATCGACA
TTGAAGAAATGCGTGCCGGTTTCTGGCGGCACGCGCCTGTTGCCGGTGTGGTCGGCACAT
TGTTGGCGGTTGCGCTGATCCTGATTCTGGTCAACCCGAAAACCGACCTTGCCGCATTTG
GTCTGATGAAAGACATTCCTGCCGATTACAACAATATCCGCGATTTGGGCAGCCGTATTT
ATACCGACTATCTGTTGCCGTTTGAATTGGCGGCGGTATTGCTGTTGTTGGGTATGGTGG
CGGCGATTGCGCTGGTTCACCGTAAAACGGTTAATCCGAAACGCATGGATCCTGCCGACC
AAGTCAAAGTACGCGCCGACCAGGGCCGTATGCGTCTGGTGAAAATGGAAGCGGTCAAAC
CGCAAGTCGAATCTGCCGAAGAAAGCGAAGTTTCAGACGACCTCAAGCCGAAAGAGGAGG
GCAAAGCATGATTACCTTGACGCATTATTTGGTATTGGGTGCGCTCCTGTTCGGTATCAG
CGCAATGGGTATCTTTATGAACCGCAAAAACGTGCTGGTATTGCTGATGTCGATCGAGCT
GATGCTTTTGGCGGTGAACTTCAACTTTATCGCCTTCTCGCAACATTTGGGCGATACTGC
CGGACAAATTTTCGTATTCTTCGTATTGACCGTTGCCGCTGCCGAATCTGCCATCGGTTT
GGCGATTATGGTGCTGGTGTACCGCAACCGACAAACAATCAACGTTGCCGATTTGGACGA
GTTGAAAGGGTAAAGGTAGGTTGGGTCGAGACCTGACAAGACACCGATGCCGTCTGAAAA
CCCGATAGGAAAAACGATGAAATCCATAGACGAACAAAGCCTGCATAATGCCCGCCGCCT
GTTTGAAAGCGGCGACATCGACCGTATCGAAGTCGGTACCACCGCGGGCCTGCAACAGAT
TCACCGTTACCTGTTCGGCGGCTTATATGATTTTGCGGGTCAAATCAGGGAAGACAACAT
TTCCAAAGGCGGTTTTCGTTTTGCCAACGCCATGTATTTAAAAGAGGCTTTGGTTAAAAT
CGAGCAGATGCCCGAGCGGACTTTTGAAGAAATCATCGCCAAATATGTTGAAATGAACAT
TGCCCATCCGTTTTTGGAGGGTAATGGCAGAAGTACCCGCATCTGGCTGGATTTGGTGCT
GAAAAAAAACCTGAAAAAAGTCGTGAACTGGCAAAATGTAAGTAAAACCCTGTATTTGCA
GGCGATGGAACGCAGCCCCGTCAACGATTTAGAACTGCGCTTTCTGTTAAAGGACAACCT
GACTGACGATGTGGACAACCGTGAAATCATCTTTAAAGGTATCGAGCAGTCGTATTATTA
CGAAGGGTATGAAAAAGGCTGAGGGTCGTCTGAAAAGCGATTTCAGACTGTTTCAGACGA
CCTGATTCGGTAGGTGATCAGACGGGAGCGGATGAGAAAAGAAATTCTGGGTAAGAATAA
TCCGGTCTGAAATATTGGAAGAAGAATGATGGATAAAAATCAGTTAGAACAAGAATTTCA
TAAAGCCATGTTAAATATTTATCAGGAGGCTTTGAATTTGCCGCAACCTTACAAGGCGAC
ACGATTTTTACAAATTGTAAATGAATTTGGTGGTAAAGAGGCGGCGGATAAATTATTGAG
TACGGGGGAAAAGAAGACTCAGACCGGTTTTACAGAGCTGATTTTGAGTGGTGGCGGAGT
CCACGCCTTGAAATACAGTATGGAATATCTGGTGTTACAAAAGCCGTGGTGTGATTTATT
TACTGAAGAGCAATTAGCTGTGGCACGCAAACGATTGGAGCGTGTTGGATTTGTTTTTCC
GAAGTAATTTTGTACGAAACAAACATAGATTTTTAAATCAATCGGATTCAATCAAATGAA
CGATATGACTTTATATTTGATAATTGCCCTTGTTCCGTTGGCAGGCTCGCTGATTGCGGG
TTTGTTCGGCAACAAAATCGGACGTGCCGGTGCGCATACGGTTACGATACTCGGCGTGGC
GGTGTCCGCCGTGCTGTCGGCTTATGTGCTGTGGGGCTTTATTGACGGCAGCCGCGCCAA
GTTTGACGAGAATGTCTATACCTGGCTGACAATGGGCGGCTTGGATTTCTCCGTCGGCTT
CTTGGTCGATACGATGACGGCGATGATGATGGTCGTGGTAACGGGCGTGTCGTTGATGGT
GCATATCTATACCATCGGCTATATGCACGATGAAAAAGTCGGCTACCAACGCTTCTTCAG
CTATATTTCTTTGTTTACATTCAGTATGTTGATGCTGATTATGAGCAACAACTTCATTCA
GCTCTTCTTCGGTTGGGAAGCGGTGGGCTTGGTGTCGTATCTCTTGATCGGTTTCTATTT
CAAACGCCCGAGCGCGACATTTGCCAACCTGAAAGCCTTTTTGATCAACCGTGTCGGCGA
CTTCGGCTTTTTGCTCGGTATCGGCTTGGTGCTTGCCTATTTCGGCGGCAGCTTGCGCTA
TCAAGATGTATTCGCTTATCTGCCCAACGTGCAAAATGCCACTATCCAACTGTTCCCCGG
TGTGGAATGGTCTTTGATTACTGTAACCTGTTTGCTCCTGTTTGTCGGTGCGATGGGTAA
ATCGGCACAATTCCCGCTGCACGTCTGGCTGCCTGATTCGATGGAAGGCCCGACCCCGAT
TTCTGCATTGATTCACGCCGCAACCATGGTTACCGCCGGTTTGTTTATGGTGTCGCGTAT
GTCGCCGATTTATGAAATGAGCAGCACCGCGCTGTCGGTCATTATGGTGATCGGCGCGAT

## Appendix A

TACCGCCCTGTTTATGGGCTTTTTGGGCGTGATTCAAAACGACATCAAACGTGTAGTTGC
GTATTCCACCCTGTCGCAATTGGGCTACATGACCGTGGCTCTGGGCGCGTCTGCCTATTC
CGTGGCGATGTTCCATGTGATGACCCACGCCTTCTTTAAAGCCCTGTTGTTCTTGGCGGC
AGGCAGCGCGATTATCGGTATGCACCACGACCAAGACATGCGCCATATGGGCAATCTGAA
AAAATATATGCCGGTTACTTGGCTGACCATGCTGATCGGTAACTTGTCGCTGATTGGTAC
GCCGTTCTTCTCCGGCTTCTACTCCAAAGATTCGATTATCGAAGCGGCGAAATACAGCAC
ACTGCCGGGCAGCGGCTTTGCCTATTTTGCCGTCCTCGCCAGCGTGTTTGTTACCGCGTT
TTACGCGTTCCGCCAATACTTTATGGTGTTCCACGGCGAAGAGAAATGGCGCAGCCTGCC
CGAACACCATTCAGACGGCCACGGCGAAGAACATCACGGTTTGGGTAAAAACGACAATCC
GCACGAAAGCCCGTTGGTGGTTACCCTGCCTTTGATTTTGCTTGCCGTTCCGTCCGTCAT
CATCGGCTACATCGCCATCGAACCCATGCTCTACGGCGATTTCTTCAAAGACGTGATTTT
CGTCAACGCCGACGCGCATCCGACTATACACATCATGAAGGAAGAGTTCCACGGCGCATT
GGCAATGGTGTCCCACAGCCTGCATTCGCCCGTACTCTACCTTGCTATCGCAGGCGTGTT
GAGCGCATGGCTTTTGTACGTCAAACTGCCGCACCTGCCAGCGAAAATTGCACAGACGTT
CCGTCCGATTTACGTTTTGTTTGAAAACAAATACTACCTCGACGCCCTGTATTTCAACGT
TTTCGCCAAAGGCACACGCGCATTGGGCACTTTCTTCTGGAAAGTCGGCGATACCGCCAT
TATTGACAACGGTATTGTCAACGGCTCTGCCAAACTGGTCGGCGCGATTGCCGCGCAAGT
GCGTAAAGCCCAAACCGGCTTTATCTACACCTACGCCGCCGCTATGGTGTTCGGCGTATT
GGTCTTGCTCGGCATGACCTTCTGGGGATTGTTCCGATAAGAATAAGGTTTCAGACGGCC
TTAAACCTTCAGGCCGTCTGAAACGAAGAAATATCCACATAAACACATTTTTATTTTAAC
CACAGGTTAACCACTATGTTTTCCAACTACCTACTCAGCTTGGCAATATGGATACCCATC
GCCGCAGGCGTGCTGGTTTTGGCAACGGGGTCGGACAGCCGTGCGCCGTTTGCCCGCGTG
CTCGCCTTCATGGGTGCGCTTGCCGGTTTCTTGGTAACACTGCCCCTGTTTACCGGTTTC
GACCGTTTGAGCGGCGGCTATCAATTTACCGAGTTCCACGAGTGGATTCCGCTTCTGAAA
ATCAACTACGCATTGGGCGTGGACGGTATTTCAGTGCTCTTTATCATCTTGAATGCGTTT
ATTACGCTGTTGGTGGTATTGGCAGGTTGGGAAGTCATTCAGAAACGTCCGGCGCAGTAT
ATGGCGGCATTCCTGATCATGTCGGGTTTGATTAACGGCGCGTTTGCCGCGCAGGATGCG
ATTCTGTTTTATGTGTTCTTCGAGGGTATGCTGATTCCGCTGTACCTGATTATCGGTGTA
TGGGGCGGTCCGCGCCGCGTCTATGCGTCGGTCAAGCTCTTCCTCTACACGCTGATGGGT
TCGCTCCTGATGCTGGTTGCGATGGTTTACCTTTATTATCAAACAGGCAGCTTCTCTATT
GTCGATTTCCAAAACATCGAACAGATTCCGTTGGGCGTACAACAGCTTTTGTTTGTGGCG
TTCTTCCTGTCATTTGCCGTAAAAGTGCCGATGTTCCCTGTGCACACTTGGTTGCCGGAT
GCCCACGTTGAAGCGCCGACCGGCGGTTCGATGGTGTTGGCGGCCATTACGCTGAAACTG
GGTGCGTATGGTTTCTTGCGCTTTATCCTGCCGATTATGCCGGATGCGGCACGCTATTTT
GCCCCCGTGATCATCGTATTAAGTCTGATTGCCGTGATTTATATCGGTATGGTGGCTTTG
GTGCAAACCGATATGAAAAAACTGGTGGCGTATTCGTCCATCAGCCATATGGGTTTTGTA
ACGCTTGGGATGTTTTTGTTTGTTGACGGGCAGTTGGACGACTGGGCATTGAAAGGTGCA
ATCATTCAAATGATTTCGCACGGTTTCGTGTCTGCCGCGATGTTTATGTGTATCGGCGTG
ATGTACGACCGCCTGCACACGCGCAATATTGCTGATTATGGCGGCGTGGTCAATGTGATG
CCCAAGTTTGCGGCGTTTATGATGCTGTTCGGTATGGCGAACGCGGGTTTGCCTGCGACT
TCCGGCTTCGTGGGCGAGTTTATGGTGATTATGGGCGCGGTCAAAGTGAATTTCTGGGTC
GGCGCGTTGGCCGCCATGACCCTGATTTACGGTGCATCTTATACCCTGTGGATGTACAAA
CGCGTTATTTTTGGTGCGATCCACAATCCGCACGTTGCCGAAATGCAAGACATCAATTGC
CGCGAATTTGCGATTTTGGCAATTTTGGCGGTGGCTGTTTTGGGTATGGGCCTGTATCCG
AACGCATTTATCGAAGTGGTGCATCAGGCGGCAAACGATTTGATTGCCCATGTGGCACAA
AGCAAGATTTGAGGTGTGTAAATGAACTGGTCTGATTTGAATTTAATGCCCGCCATGCCC
GAAATCGTGCTGCTGTCGCTGCTGGTGTTATTGTTGCTGGCGGACTTGTGGGTCAGTGAT
GACAAACGCCCGTGGACGCATTACGGCGCGTTGGCAACGGTGGCGGTTACGGCTGTGGTG
GAGTTGGCGGTGTGGAACAGGGCAGCACGTCTTCGTTCAACGGGATGTATATTGCAGAC
GGTATGTCGCGTTTGGCAAAAATGGTTTTATATGCCTTGACCTTTGCCCTGTTTGTCTAT
GCCAAGCCCTACAACCAAGTGCGCGGTATTTTTAAAGGCGAGTTTTACACCCTGTCATTG
TTTGCCCTGTTGGGTATGAGTGTGATGGTGAGCGCGGGGCATTTTTTAACTGCCTATATC
GGTTTGGAACTCTTGTCGCTTGCCCTTTACGCCCTGATTGCCCTGCGCCGCGATTCCGGC
TTTGCCGCCGAAGCCGCCTTGAAATATTTTGTTTTGGGCGCGCTGGCATCCGGCCTGCTG
CTCTACGGTATTTCTATGGTTTACGGCGCAACCGGTTCGCTGGAATTTGCCGGCGTGCTC
GCCTCTTCCTTCAATGAAGAAGCCAACGAATGGCTGTTGAAACTGGGTTTGGTGTTTATC
GTCGTCGCCGTCGCGTTCAAACTCGGTGCGGTGCCGTTCCATATGTGGGTGCCCGACGTG
TATCACGGCGCGCCCACTTCTGTTACCGCCTTGGTCGGCACTGCCCCGAAAATCGCCGCC
GTCGTTTTCACTTTCCGCATCCTCGTTACCGGGCTGGGAACCGTGCATCATGACTGGTCT
CTGATGTTTGCCCTGCTTGCCGCCGCCTCGCTGCTGGTCGGCAACCTTGCCGCCATCATG
CAGACCAATATCAAACGTATGTTCGCCTATTCCACCGTATCGCATATGGGTTTCATCCTG
TTGGCGTTTATGGCGGGCGCGGTCGGCTTTGCGGCGGGCCTCTATTACGCCATTACCTAC
GCGCTGATGGCGGCGGCAGGGTTCGGAGTGTTGATGGTGTTGTCGGACGGGGACAACGAG
TGCGAAAACATCAGCGATTTGGCAGGGTTGAACCAACACCGCGTATGGCTTGCCTTTTTG
ATGCTGCTGGTTATGTTCTCTATGGCGGGCATTCCGCCGCTGATGGGTTTTTACGCCAAA
TTCGGCGTGATTATGGCACTCTTGAAACAAGGCCATGTTTGGTTGTCTGTATTTGCCGTC
ATCATGTCGCTGATTGGTGCGTTCTACTACCTGCGCGTGGTCAAAGTCATCTACTTCGAT
GTGCCTGATCATGACCAGCCGGTCGGCAGCAACTATGCCGCCAAATTTGTTCTGACGGTC
AATGCCTTCTTGCTGCTCCTGTGGGGCATCATGCCGCAAACCGTTATCGACTGGTGCGCC
AAGGCGTTGGAGAACACGCTGTAAGCCGCCGCAACGGCAGCCGTGTCAGAGGCTGCCGTT
TTTGTTAAGATATGCCGTTCCGCAACGCGGTTCAGACGGCATCGCCGCCGACAACGCCTA
AACAGAAAGCCCACCATGACCGCATCCATGTACATCCTTTTGGTCTTGGCACTCATCTTT
GCCAACGCCCCCTTCCTCACGACCAGACTGTTCGGCGTGGCCGCACTCAAGCGCAAACAT
TTCGGACACCACATGATCGAGCTGGCGGCAGGTTTCGCGCTGACCGCCGTTCTTGCCTAC
ATCCTCGAATCCCGTGCAGGATCGGTACACGATCAGGGTTGGGAGTTTTATGCCACAGTC

## Appendix A

GTCTGCCTGTACCTGATTTTTGCGTTTCCATGTTTTGTGTGGCGGTATTTTTGGCACACG
CGCAACAGGGAATAGACAAGCATAGGAATGCCGTCTGAAACCCTTTCAGACGGCATTTGT
TTCATTCAAGTGCAGGCCGGCATCGCTGTGCCGGCACGTTTCAGCCGGCGATATACGCCG
GTTTTAATATTTGCGGGCGACTGCAAATTCTGCCAACTGCCGCAGGCGCAGGGCTTTGTC
GCCGAAGGGTTCGAGCAGCGCGACCGCTTCGGCAACCAGTTTGTGTGCGTATGAGCGCGC
CGCTTCCAAGCCCATCAGTTTCACATAAGTCGGCTTGTCGTTGTCTGCGTCTTTGCCCGC
CGTTTTGCCCAAAGTCGCCGTGTCCGCTTCACAATCCAACACATCGTCAATGACTTGGAA
CGCCAGCCCCAGTTTTGCCGCGTAAGCGTCCAATACGGAAAGTTCCGCATCTGACAGATC
AGGACACGCCGTCGCCCCCAATAAAACCGCCGCACGGATTAGCGCACCCGTTTTCAGGCT
GTGCATCTGTTCCAAATCGGCTTGAACCATTTGTTTGCCGACATTCGCCAAATCGATTGC
CTGACCGCCCGCCATACCCCTGCTGCCGCCCGCTTTCGCCAACACCGACAACATTGCCAA
CTGGCGTGCGGCGGGCAGTTCTGTCGGACGGCTCAACACGTCAAATGCCTGTGTCTGCAA
AGCGTCGCCGGTCAGAAGGGCGGTCGCTTCGCCATATTTGATGTGGCAAGTCGGTTTGCC
GCGCCGCAGGCTGTCGTTGTCCATCGCCGGCATATCGTCGTGAACCAAAGAATAGACGTG
GATCATTTCGATTGCCGCCATTGCCTGTTCTACTGCTTCATGCACGGCTTCGCCTAATTC
CGAAGCTGCCAGAACCAGCATCGGCCGCAGACGCTTACCGCCGTCCAAAGCCGCATAACG
CATCGCTTCGTGCAGTGTGTGCGGTATTTCCCCCTCAGACGGTAAAAACCGTTCAAGCAG
CAGCTCTGTTTGCGCCTGCGCCCTCTGTTGCCACGTTTTCAAATCATTCGTCGGATTCAA
GGTTTAACTCCTTCAGCCCGTCTGTGTCTAAAACCTGTAGCTTTTGTTCGACTTGTGCCA
GTTTGGTTTGGCAGTACCTGACCAGTTCGTTGCCTTCCTGATAGGCGGCAAGCGCGTCTT
CCAAGGGCATTTCGCCCTGCATAGACTGCGTCAGCGATTCGAGGCGCGACAAGGCTTCTT
CAAACGATTTCGGGGCGTTTTTCTTCATCGTATTTCCTTTTCGGTTGAAACCCCGCCCTT
TAGGGCGGCAGGATCAGACTTTATTTGGGAGGGGTGTAACCCTTTCCAAATCAGGGCAAT
ACATAGGGCGGTGCTTTATGTGCCGTCCTGTGTGTTGGAACATAGTTTCGGATGTTCCGG
TAAAAAGCGGATTGTAGCATTTTTGAAAAACGGATGCCGTCTGAAACCCGAATCCGGCTT
CAGACGGCATTTTTTCCGCCCAGGCGGCAAGGCGTTACCCGGGCAGTTCGTCGGTGATGC
CCTGCAAAAAGGCGAGGCGTTCGGGGCTTGCCGCCCCGGTTTGCGCGGCGGCTTTGAAGG
CGCAGCCGGGTTCGGCGCGGTGGGTGCAGTTGTGGAAGCGGCATTGCCCGACAAGGTGGC
GGAAATCGGGGAAATAGCGCGGCAAATCGGCGGCTTGGAGGTGGTGTAAACCAAATTCTT
GCAAACCCGGGGAGTCGATGAGTTGGGTTTCGCCGTTCAAATCATAAAGCCGGGCGTGGG
TGGTGGTGTGTTTTCCCGAGTCGAGTGCGGCGGAAATGTCGCCGGTGCGGGCGGTTTGGC
TGCCCAAAAGGGCGTTGGTCAGGGTGGATTTGCCCATACCGCTCTGCCCGAGCAGGATGT
TGCTGTGCCCTTGCAGGGCGGGGCGCAGGCTGCCGGCGTTTTCCAGTGCGCGGGTTTCGA
TGACGGGATAACCCAGCGTTTCGTAGAATTTGAGTTTTTCGCGCCAAAGGGCGGTTTCGG
GCAGGTCGGCTTTGTTCAGGACGATGACGGCTTCAATACCGGCGGCTTCGGCGGCAAGCA
GGGCGCGTTGCAGCAGCCGCACGCTCGGACTCGGGACGGCGGCGGTTACGATGAGGAGTT
GGGTAACGTTGGCGGCGATGAGTTTGGTTTTCCACGCGTCTTGGCGGTAGAGCAGGCTTT
GGCGCGGTAAAAAATCTTCAATCACAACTTGTTCGGCGTTGACGGGGCTGATGCGGACGC
GGTCGCCGCAGGCGAAATCGACGCGTTTTTTGCGGGTGCTGGCTTCGTAGGTTGTGCCGT
CGGGCGTGCGGACAATGTAGCGGCGGCCGTAGCTGGCGGTAATTTGGGCGGTGTCGTTCA
TGGTTTCTTTGGGGTTGGGTGTGGGAATGCCGTCTGAAAACGGGTGTTCGGACGGCATCG
GTTCAGTCGTGCTGCCACTCGACGTGTTCGTTGAGGAAGCCGCCGCTCTGGTGCGCCCAG
AGTTTGGCGTAAAGCCCGCGTTTTTCGAGGAGTTCGGCGTGTGTGCCTTCTTCGATGATG
CGGCCTTTGTCGAGGACGACGAGCCTGTCCATTGCGGCGATGGTGGAGAGGCGGTGGGCG
ATGGCGATGACGGTTTTGCCGTCCATCATTTTGTCGAGGCTTTCTTGGATGGCGGCTTCG
ACTTCGGAATCGAGCGCGCTGGTGGCTTCGTCCAAAAGAAGAATCGGTGCGTCTTTGAGC
ATCACGCGGGCGATGGCGATGCGCTGGCGTTGCCCGCCGGAGAGTTTCACGCCGCGTTCG
CCGACGTGTGCGTCGTAGCCGCGCCGCCCTTTGGCATCGGAAAGGTCGGGGATGAAGCCG
GCGGCTTCGGCGCGTTCGGCGGCAGAAACCATTTCGGCATCGGTCGCGTCGGGGCGGCCG
TAAATAATGTTGTCGCGCACGGAACGGTGCAGCAGCGAGGTATCTTGCGTGACCAAACCG
ATTTGGGCGCGTAAAGATTCTTGGGTAACGCCGCTTATGTCCTGCCCGTCGATCGAAACC
GTGCCGCTTTGCGGTTCGTAGAAGCGCAAAAGCAGGTTGACGATGGTGGATTTGCCCGCG
CCGCTGCGTCCGATCAAGCCGACTTTTTCGCCCGGGCGGATGGTGAGGTTGAAGCCGTTG
AGCAGCGGTTTGCCCGCTTCGTAGGAGAAATCGACGTGTTCAAATTTGATTGCGCCTTGC
GGCACGTTCAGCGGCAGTGCCCGGGGCTTGTCGAGGATGGTGTGCGGTTTGGACAGGGTT
GCCATGCCGTCGCCGACGGTGCCGATGTTTTCAAACAGCCGCGCGGATTCCCACATAATG
TATTGCGACAAACCGTTGACGCGCAACGCCATGGCGGTGGCTGTAGCAACCGCGCCCACG
CCGACCTGCCCGTTGTGCCAGAGCCAGATGCCCAGTGCGGCGGTGGAGAGGGTCAGGGAG
GTGTTGACGATGAAGCTGCACGAATGCAGCAGCGTCGCCAGCCGCATTTGGGCGCGCACC
GTAACCATAAATTCTTCCATCGACTGCTTGGCATAGGCGGCTTCACGCGCGCCGTGGGAG
AAGAGTTTGACGGTGGCGATATTGGAATAGGCATCGGTAATGCGGCCGGTCATCAGCGAG
CGGGCATCCGCCTGCCATGCGGCGGTTTGCCCCAATTTGGGAATCAGCAGGCGCATCACC
GAAGCGAAACCGACAATCCAGCCGATAAAGGGCAGCAGCAGCCATGAGTCGAGCGAGGCG
AGAATCACGCCGGAGGTAATGAAATACACCGACACATAAACGACCATATCGGCAACCGTC
ATCACCGCGTCGCGCAACGCCAGCGCGGTCTGCATGACTTTGGCGGACACGCGTCCGGCA
AATTCGTCCTGATAAAAACCGAGGCTTTGGTTCAGCATCAGGCGGTGGAAGTTCCAGCGC
AGGCGCATGGGGAACACGCCCTGAAGGGTTTGCAGGCGCACGTTGGACGCGGCAAACGCC
CACGCAACCGAAAATACCATCATCGCCGCCATTGCCGCCAGTTCCCAACTTTTTTCGGCA
AACAGTTCGGCGGGCGCGTATTTGCCGAGCCACTCCACGATTTTGCCCATAAATTGAAAA
ACCAGGGCTTCCATAATGCCGATGCCGGCGGTCAGCGCAGCCAGGGCGGCTATCCATTTC
CGCACGCCGGCCATGCTGCTCCAGACAAACCGCCACAAGCCTTTTTCTGGCGTTTTCGGG
GCGGCTTCGGGATAAGGGTCGATTCGGGACTCGAACCAGGAAAATATTTTGTTCAACATT
GTTTTCGATTTCGGTAAAACAGTTTCAGACGGCATCAAACACAATGCCGTCTGAAAGGAA
GGACAATAACGCCATTTTACGGGAAAAGCCGTCGGGAAGACAGCGCGAGGCGGAAACGCA
GGGTTTCGTCAGGGCAAACGCCGCGCCGCCTTCAGGCGGCATTATTTCAGCAGGTTTTTC

## Appendix A

AAAGCAAGGCGCACGCCTTCGCCCACGTCCGTCCCCTCCGGAACGCCTTTGACCGCCGCT
TTTGCTTCGCGTTCGCTGTAACCCAGCGCAAGCAGCGTGCTGACGATGTCTTCCGTTTCG
TCGGCGGCGGGTGCGGCGGCAAACAGCCCGTCCGTTACCGTATGCGCGACCAGCTTGCCG
CGCAGTTCCAAAACCATACGTTCGGCGGTTTTTTTGCCGATTCCCGGGGCGGAGGAGAGG
CGTTTGACATCTTCTTCTGCAACCGCCCGCGCCAGTTCGTCGGCAGTCATTGCCGACAAA
ATGCCCAAAGCCGTTTTCGCGCCGATGCCGCCGACCTTGATCAGTTGGCGGAAGGTCTTG
CGTTCTTCCGCAGTGGCAAAACCAAATAAAAGATGTGCGTCTTCCCGAATGATAAGCTGG
GTAAACAGTTGTACGCTTTCACCCACGGGCGGCAGGTTGTAGAAGGTCTGCATCGATACG
TCGGCCTCATAGCCGACACCGTTGACATCGATGACGATTTGCGGAGGGTTTTTTTCAACC
AGTTTGCCGGTCAGTCTGCTGATCATGTGTGCCGAATCCTGAAGTGTCGGGTGCAAAATG
CCGTCTGAAACCGGTTTGGGCTTCAGACGGCACGGATTGTATCAAATTCAGTCGTCGCGG
CGGGAGGAAATCACGCGGCCGGTACGGGCATCGACAACGACTTTGTATTCCTGTCCGTTT
TTGACGATTTCGACATCATAGTGCGGACGGCCGTTGTCGTGTTCGAGATCGATGTCGGTG
ATTTTGCCGCCGACACGCGCCAACGCTGCTTTTTCGGCTTGGGCGCGGCTGATGATTTTG
TCTTGTTTGTTGTGTTGGTGTGCGGCGTGTCCGTGGTCGTCATCGCCGTGTCCGTCGTGG
TGGGCGAGCGCGGGGGCGGAAATGCTCAGCAGTGCGGTTGCGGCGGAGGTCAAGAGAAGG
TGTTTGATGTTCATATTTTGCCTTTGTAAATCGTGGGTTGGAAAATGTGGATATTAATAA
GGTATCAAATAACCGTCAGCCGGCGGTCAATACCGCCCGAACCATACCGCGCGCCTGAGC
TTCGGCTTCGGCGGCGCGTTCCTGCGAGGTAAACGGTCCCATTTTGACGACGTATTCGTA
ACGGCGTTTTTCAACCGAGAGGTTCGTACCCGATGACGAAACGGCGAAGTTTTGGGCGGC
TTGGTTCAGATAGGCTTGTGCTTCGTGTTCCGTACCGAAAGATTTCAAGTCGATAAAGAT
GTCTTTGTTTTCGGCAACCGGTGCGGATTGGCCCGGGACGATTTGTTCGATTTTGACGTG
TGCCGTCCCTTGGTTGACAAAGCCCAATTTTTGCGCGGCGGCTTTGGATACGTCGATGAT
GCGGTTGCCGTGGAAGGGGCCGCGGTCGTTGACGCGGACGATGACGCTTTTGCCGTTTTT
GGTATTGGTTACGCGCACATAGCTGGGGATGGGCAGGGTTTTGTGGGCGGCGGTAAAGGC
GTTCATATCGTATCGTTCTCCGCCGGAAGTTTTGCGCCCGTGAAACCTGCCGCCGTACCA
CGAGGCGTTGCCGGTTTGCGTGAATTCGGCGACTTGGTTTTTCGGCGTGTAGCGTTTTCC
GGCGACTTTGTAGCTGCGGTTGGCGGAGGCGTGCAGTTTTTCTGCCTTGACCACTGCGTC
GGCGGATGCCGTCTGAAGGGAGTGTGTGCCGAATGCGGCGGTGAGAAGGAAAAGGGTTTT
TCGGGTTAAAGTCAAAACGTGTTCCGTTCTTGAGTTGAAGACGAATGGGCATCATGCCCG
CCGGATACGTTCCGAACCGCCGTACAGTGCGGACGGCGGTTCGGAATGTGTCCGGATAGG
TTTTCAGACGGCATGAACCTGCGTTCAAACGCCGCCTGCGTAACCGTGTTGCCGCCACGC
TTCAAAGAGAATCACGGCGACGGTGTTGGAAAGGTTCATACTCCGGCTGCCGGGCTGCAT
CGGCAGGCGGATTTTTTGCGCGGCGGGCAGGCTGTCGAGGATGTCGGCAGGCAGTCCGCG
CGTTTCCGGCCCGAACAGTAAAACGTCGCCTTTTTGAAACGCGGTTTCATCGGGGCGCGC
CGTGCCTTTGGTGGTCAGGGCGAAAATGCGCCTGCCTGCGAGTGCCTTGAGGCAGTCGTC
GAAGTTTTCGTGCACCGTCAGGCTGGCGAACTCGTGGTAGTCGAGCCCGGCGCGTTTCAT
TTTGGCGGAATCCAATGGGAAGCCGAGCGGTTTGACAAGGTGCAAATCCGCGCCGGTATT
GGCGCACAGGCGGATGATGTTGCCCGTGTTCGGCGGGATTTCCGGCTGGTATAAAACGAT
GGTAAACATAAATATCAATCACTTATAGGCGCGTAACCTTGCCACAAGGCGGATGGGGTG
TCAAAAAATTTAGTTATTTTTTCATTGGCGTGCGTGCCAGCGTCCAGCAGCAGATTCGGT
TTGCGCCCGATTTTTTCAGCGTCTTTGCCAATTCGTCCAGCGTCGCGCCGGTGGTAAAGA
CATCGTCGATTAACAGAATATTACAGTTTTCCGGTATCGGTGTGCGGATTTCAAAGGCGT
TTTTGATGTTTCGCCGCCGTTCGCCGCCTTTGAGCGTGCTTTGCGGCGGGCGGTGGTGTC
GGAAAACGGTGTGTCGGGGCAGTATCTGCCAGCCGTAGCGTTGTGCCAGCAGCCCGACGA
TGCTTTCACTTTGGTTGAACCCGCGTTGCAGCAGCCGCTCCCTGCTTAGCGGTACGGGCA
GGACGAAATCGAAACATTCGTCTGCAAGCCGGTCGGGCGGATTCTGCATCATCAGGTCTG
CCAGCGGCTGCACCATGCTCAAATCAGCCAAGTGCTTCAGCGCGTGTATCATATTGCTGA
GGGGCGGTTCGTAATGCAGCGAAGCCCACATCCGGTCGAATGCGGGCGGTTTTTTCTGAC
AGCCGCCGCACACCGATCCGCCTTGGATGTGTCTGAAACACAGGGGGCAGCTGTTTGCCG
CGTCGGTGCGGTATGCCGCCAAATCGTCGCGGCAGCCGGCGCAGATGCCGTCTGAAACGC
CAGACGAACCGTGGCATAATACGCAACGCCTGATAGTGGGCGCGTCTGCGATGCGCCGCC
AACGAGAGAGAAAATCCATGCCTGATGCCGTCAAAAAAGTTTACCTGATACACGGTTGGG
GGGCGAACCGCCACATGTTCGACGATTTGATGCCGCGCCTGCCTGCAACGTGGCCGGTGT
CCGCCGTCGATTTGCCCGGACACGGGGACGCTCCGTTTGTCCGACCTTTCGACATTGCGG
CTGCGGCCGACGGCATTGCCGCTCAAATTGACGCTCCGGCCGACATTCTCGGCTGGTCGC
TCGGCGGATTGGTCGCGCTGTATCTGGCGGCGCGCCATCCCGACAAAGTCCGTTCGCTCT
GCCTGACGGCGAGTTTCGCACGGCTGACGGCTGACGAAGACTATCCCGAAGGGCTTGCCG
CGCCTGCATTGGGCAAAATGGTCGGTGCGTTCCGTTCGGATTATGCCAAACATATCAAAC
AGTTTCTACAATTACAGCTTCTGCACACGCCTGATGCGGACGGAATCATAGGCAGAATCC
TGCCCGATTTGGCGCGCTGCGGCACGCCTCAAGCCTTGCAGGAGGCGTTGGACGCGGCGG
AAAGGGCGGATGCGCGGCATTTGTTGGACAAGATAGATGTTCCGGTACTGCTGGTGTTCG
GCGGCAAAGACGCGATTACGCCGCCGCGTATGGGTGAATATCTGCACCGCCGTTTGAAGG
GCAGCAGGTTGGTTGTGATGGAAAAGGCGGCGCATGCGCCGTTTTTGAGCCATGCGGAAG
CGTTTGCCGCGCTGTACCGCGACTTTGTTGAAGGGGGTTTGAGATGAACCATCAGGACGC
ACGCTGGCAGGTTCACCGCCATCTTGCCGAACATACCGACCAACGGCTGACACTCGTCCG
CAACGCGCCCAAGCATATCCTGCTTGCCGGTGCGGATGCGGACATCAGCCGCAGCCTGCT
GGCGAAACGCTATCCGCAGGCGGTATTTGAAGAATACGATTCCCGTGCGGATTTTTTGGC
GGCTGCCGCTGCCGCCCGCAAAGGCGGTTTTTGGCAAAGGTTTACGGGTAAGGGCGTGGT
GCAACACTGCCAATCCCCGATCGCGCCGCTGCCCGAAGCGTGTGCCGATATGTTGTGGTC
GAATCTCGGACTGTTGGCGGCGGAACAAATCCTTCCTGTGCTGCACAACTGGGCGCGCGC
CTTGAAGACGGACGGGCTGCTGTTTTTTACCTGCTTCGGGCGAGATACCTTGGCGGAACT
GAAATGCCGTCTGAAAGAAAACGGCATTGAAAGCCGCAGCGCGCTTTTCCCTGATATGCA
CGACTTGGGCGATATGCTTGCTGAAAACGGCTTTTACGACCCCGTTACCGATACGGCGAA
GCTGGTGTTGGATTACAAAAAGGCGGAAACGTTTTGGGCGGATATGGACACGCTGGGCGT

## Appendix A

TTGGCGGGCGATGGCGTGGAACGATGAAAACGCCGCGCGTTCGTGTGTCGGGACAATATT
TGAGCGGGAAGGCGGTTTGGGCATTACGCTGGAAACGGTGTACGGACACGCCGTGAAAAA
ACTGATGCTGCCGCAAGGGGAGAACGTGGTGCAGTTTTTCCGAAGAGATGATGTGCAGA
TGCCGTCTGAAGCCGTTTCCAGGTTTCAGACGGCATTTGTCTGTGAAAACCGACAGAAAT
AAAGGAAATGCCGATGTATAGTGAATTAAATTTAAACCAGTACAGCGTTGCCTCGCCTTA
GCTCAAAGAGAACGATTCTCTAAGGTGCTGAAGCACCAAGTGAATCGGTTCCGTACTATT
TGTACTGTCTGCGGCTTCGCCGCCTTGTCCTGATTTTTGTTAATCCACTATATGCTGATG
CCGGAGACGTATATTGCGTCTATAACATCAGACTGAAGCAGTACACTGCCTGCCAGGTTA
CCCGAGTTGAAGAACACGGTGGCAAAAAAAACACATGCGACCCTGCTGGCTTTGGACTGG
CAGGGCAACAAACCGCTTGGGGCGGAGGAGCTGGCGGATTTGAAATCGCTTTACAAAGAC
TTAAAGAATAATATTGGAAATATTGTATGAACAAAAAATTAAACTATATTTTTATGTTGG
ACTGTTTAGGGTTGGTGATATTGTTTACTTGTATAATAGCTACTTTTGAAAGAGATTATG
GATTTAAAATTTTTACTAATTCTAAGAGACCTGAATTTTATTATTGGATTGGAATGTTTT
ATTATGGAATTATTTCTTGCTGGTTTGATTATCAATTAATTTCAACAAAGGCGAATTCGT
ATAAAAGAAAAGTTAAACAATATAAAATTTTTTCAGTAATATTTTCAGTTTTGATATTTA
TTTCTACTATAGTAAAACTTTAAATTTTGGAGCAAAAATTTATGAGCGATTCAATTGAAT
ATGTATTGGGAACGCGGTCTGCACATGTATAAGGCAAGTGCCGTCGTGCCGACGGGATAT
GTACGGGTTGGGAATACCGCGCCGCTGGTCGGCGAAGACACGCAACGGTATGCCTCTTTT
TGGGGCGACGGCTACGACGTGTACCGTCAGTTGAGATGGCAGCAGATACCCGAAAAACAG
AGAAAGGCATTCAAAAAAGCCGCCAAAAGCAAAAAGACCGTGATGTTTGCCGGACGGGAA
TACGGCATATCCAAACAGAATTTGAGCGATGTTTGGGATGATTTTGAAGACGCGATGGAA
CTGAAGGCGTTTCCCTGCCTGTCTTCGCTGTTTCTGACCAAATGGCATAAAAATCTATAT
GATAGTGGATTAACAAAAACCAGTACGGCGTTGCCTCGCCTTAGCTCAAAGAGAACGATT
CTCTAAGGTGCTGAAGCACCAAGTGAATCGGTTCCGTACTATTTGTACTGTCTGCGGCTC
GCCGCCTTGTCCTGATTTTTGTTAATCCACTATAAAAACAGGAATTTTTAAATAGAGGCA
ATGCCGTCTGAAACTTGGTAACGGGCTTCAGACGGCATTTCGTTCCAATACCGCCAACAC
CGCCGCACCGTAACGTGCGGCTTTTTCTTCGCCTACGCCGTATACGGCGGCAAGCTCCGC
CAAGCCTTCCGGCTGTTTGGCGGCAATGGCGCGCAGTGCGGCTTTGCTGAGAATGCGGTA
GGGTTCGGACTGTTCGTGTTTTGCCGTTTCGCCGCACCATTGGATCAGGGCGCGCATCAG
GCGGCGTTTGCGTTTGGCGGTTTCATCGATGCCGTCTGAAAACGGACGGCAGACGGCGAG
GATGTCCCGTCCGTATTTGGCGGCGCGTACGCTGCCCAAGCCGTACACGCCTTCGAGGTC
GGTTTCGGTTTCGGGCGTATCGGCAAGCATATCGGCAAGGCTTTCGTCGGAGAGGACGGC
ATGCAGGGCGCAGTTTTCCGCCCTTGCCTGTTCATACCGCCAGGCTTCGAGTTTTTGACG
CAGTTGTTGTTCGCGTTCGGTTTGCGGACGGATGACCGCGTCGCGGCTGAAGCCGGCGGC
GTTGCGGCAGACTTCGAGGATGCCGTGTCCGAAACGGTCGATTTTGGCTTCGCCCAAACC
GTAGATGTCGTGCAGACCGTTGAGGTCTTGCGGCATTTTTTCGACAAGGTCGCGCAGGGT
TTTGTCGCCGAAAATCATATAGGCGGGGATGCCTTCGGCTTCTGCCTGTTTCATACGCCA
AACGCGCAATGCCTGCCACAGGCGTTCTTCGCGTTCGGTACGCAGCCAGTTGTCTTTGAG
GGTGCGGGCGGCGGGCTTGTCGCGCTTGAGCGGACGCAGCATCACTTCGGTTTCGCCTTT
GAGGACTTTTTTGGCGGCTTCGGTCAGTTGCAATGCCTGATATCGGGTAATGTTGACGGT
GAGGTAGCCGAGGCTGATACACTGGCGGATGACGCTGCGCCATTCTTTGTCGGACAACTC
CGTACCGATGCCGAATGTGGACAGTTGTTCGTGCCGGTTGCCGCGTATCCAATCGTCGCT
TTTACCTCGTAAAATGTTGGTGATGTAACCGGCGGCAAAACGTTGTCCGGCGCGGTACAC
GCAGCTGAGTAATTTTTGCACCAACACCGTGCCGTCAAACCGTACGGGCGGATGCAGGCA
GTTGTCGCAATGGCCGCAGGGTTCGGATGCTTCGCCGAAATGTTTGAGCAGCAGTACGCG
GCGGCAGGCGGCGGTTTCGCAGACGGCAAGCATGGCATCGAGTTTTTGCATTTCGATTTG
CTTTTGCACCTCGTCGCTGTTGCCTTCGGCAATCCGTTCGCGCAGCAACACCCAATCGTT
CAAACCGTAACACAGCCAGCTTGCGGCCGGCAGCCCGTCCCGTCCGGCGCGCCCCGATTC
TTGATAGAAATGTTCGACACTCTGGGGCATATCGAGATGGGCGACAAAGCGCACGTCGGG
TTTGTCTATGCCCATGCCGAACGCCACGGTCGCCACCACGATAATATTGTCTTCATGCGT
AAAGCGGCGTTGGTTTTCCTCGCGTACGTCCATGCTCAAACCAGCATGATACGGAATCGC
GTTTAATCCGTTTTCACGCAAAAACTGCGCCACATCTTCCACCTTTTTGCGGCTTAGGCA
ATACACAATGCCGCTTTGCCCCGTCATTTCTTTGCGGATGAAATCCAGCAATTGTTTTTT
GCCGTTGTTTTTTTCGATAACCTGATAATAAATATTCGGACGGTCAAAGCTGGAGACAAA
TTCGGGCGCATCGTCCAAGTGCAGATAATGCTTGATGTCGGCGCGCGTGGCGGCATCGGC
GGTAGCGGTCAGAGCGATGCGCGGGACGTTCGGATAGCGTTCGGCAAGCATGCCGAGCTG
TTGATATTCAGGGCGGAAATCGTGTCCCCATTGGCTGACGCAATGCGCCTCATCAATGGC
AAACAGACTGACGGTTTGTTGGTCGAGAAAACGCAAAAAGCGGTCGGTAACCAAGCGTTC
CGGCGCGACATAAAGCAGCTTCAGACGGCCTTGGGCAAGCCGGTCGGCAATCTCGCGCGC
CTCGTCTGCCGATGTGCCGCTGTTGACTGCCGCCGCTTCGATGCCGGCGGCGTGCAGGTT
TGCCACTTGGTCGTTCATCAGCGCAATCAGCGGCGATACGACAACCGCCACGCCTTCGCG
CATCAGCGCGGGAATCTGGTAACACAAAGACTTGCCACCGCCCGTCGGCATCAGCACCGT
CAAACTCCCGCCGCCTGCCAAAGTATTGATGACAGCCTCCTGCCTGCCGCGAAATTCGGG
ATAACCAAATACTTCGTGCAGAATCTGTTTGGCGGTCGGTCGGTGCATGATGGTTCCGTG
CTCGGTAAGGGTGTTGATCGGTCGGCGGCAATATGCCGTCTGAAATCGGGATTTAGAATA
GTTTGCCCACTTCTGCTTCAATATCGTCGGCACGCATAAACGTTTCGCCGATCAGGAAGG
TATGCACGCCGCGCGATTGCATAAATTCCACATCCGCCTTGCCTGTAATGCCGCTTTCGG
TAACGACGGTTTTGCCTTCCAGCGCGGGCAGCAGCGACAGGGTTTGGTCGAGGGAGACTT
CAAAAGTCCTCAGGTTGCGGTTGTTTACGCCCCACAGCGGCGTGGTCAGGTTGCGGCATT
TTTCCAATTCGGTTTCGTCGTGCAGCTCGAGTAGGACGGTCATGCCCAATTCGTGCGCCA
CCGCTTCAAAGCGTTCCAATTGTTCCTGTTCCAGTGCTGCGGCAATCAGCAGGACGGCAT
CCGCCCCCCATGCGCGCGCCTGATAAACCTGGTATTCGTCGATGATGAAGTCTTTGCGCA
GCACGGGCAGCGATACGGCTTCGCGCGCCTGTTTGAGGTATTCGGGCGAACCTTGGAAAT
AGGGTTCGTCGGTCAGTACGGACAAACACGCCGCTCCGGCGTTTTCATAGGCGCGTGCAA
TCTCGGCAGGGCGGAAGTCCGGACGGATTAACCCTTTGCTCGGGCTTGCCTTTTTGATTT

## Appendix A

CGGCTATGACGGCGGGCAGGTTTAGGCGGTGTTTGCCGCGTATCGAATCGATGAAGCTGC
GGACGGGCGCGGCTTCTGCGGCAAGTGTGCGGATGTGTTCGGCGTTGACGGCGGCTTTTT
GAGCGGCAACTTCCTGTGCTTTGGTGGCAAGGATTTTATTGAGGATGTCGGTCATGTCGG
GTTCCGTATTCGTCTGGGGAAAGGGGGAATATTAGCATCAAACCGTTAACGCCTGTTTGT
GCGGAAGCTGTCGAAATAGGACAGGACGGTCTGCGGCAGCCATTGCAGGTGCAGCCTGCC
GCCGGTGCTGCTGACAAAGCCGACATGACCACCATATGCCGGCTGGAACAGGGTAACGGC
TTCGGATACTTCGTCTGCGCGGGGCAGGGCTTCGGGCGGCAGGAAGGGGTCGTTGACGGC
ATTGAGCAGGAGCAGCGGTTTGGCAACGTGTTTGAGCAGCGGTTTGCAGGAAGTTTGGCG
GTAGTAGTCGTGCCGGTCGGCAAAGCCGTGCAGCGGTGCGGTGAAGCGGTCGTCAAACTC
GCCCAGTGTTTTGCACCCTGCGGCAAATGCCGTCTGAAAACCTTGGAGCGATTTTGCTTT
GGGTATCAGGGTGCGGAGGAAGTAGCGCGTGTAGAGCAGCCGCGTGATGCCGCTGTCGAA
GCGTCTGCCTGCCGCCTCTGCATCGACGGGGGCGGAGATGACGGCAGCGGCTTGCGGCAA
TGCCTTTTTGCCCTGTTCGCCCAAATATTTTGCCAGCGCGTTGCCGCCCAGCGATACGCC
GACGGCGTATATTTCACGGTAACGCGCGGCGAACGTGTCCAAAGTAAAGGCGATTTCGGC
GGTATCGCCCAAGTGGTAGAACACCGGAGCGGTGTTGGCAATGCCGCCGCAGCTGCGGAA
ATGGACGACTACGCCGTGCCAACCCCGATCGCGTACCGCAAGCATCAGTTCGACCGCGTA
ATGGCTGCGGCTGCTTCCTTCCAAACCGTGAAACAGCACGACCAGCGGCGCATCGGGCGA
AATGCCGTCTGAAAAGTCGTAGGCGACTTTGGTTTTACCCGTGCTGTCGGGAAGCAGCTC
TCGGCGGTATGCGGGCGCGGGGCGTTGCAGGAATTTGGCGGCAATCGTGTCGGCATTGCC
GTTGCGGAGGAAAAAGGGCGTGTCCGGCGGTGTTAAAATCATAAGGTATCGGTTTTCTTG
TTTTCAGACGGCATTGATGATGCGGCAGCCCGTCCGGCTGGTGCGGACGTGGGGGATGCG
CGCCCGAATATAGGCGTGGAAAAGCGTTTGCCGAAAAAGGATATCGGCATCGGTCAGTTT
TCCACGCGTTTGAAATGGCGCGGACGGAAGCCCAAAGCCGCCAGTGATGCGAAATACAGT
CCGCCGCCGACGGCAATCAGGATGCAGAGCTGCCCCGCTTTCCGCATTCCGCCGGCGTGC
GCCCATTCAAACGGCAGGTAAGCCTGCGCTGCCCACAGTCCGCCGCACATCACGGCGAGC
GAGAGCAGCATTTTTGCTAAGAACGCTGCCCAACCCTTGCCAGGTTGGTAAATACCGTGT
CTGCGCAACAGGTAAAACAACAATCCGGCATTGATACACGCGCCCAGACCGATGGCAAGC
GAAAGTCCGACGTGTTTCAGTGGGCCGATAAAGGCAAGGTTCATCAACTGCGTGCAGATG
AGCGTGAAGATGGCGATTTTGACGGGCGTTTTGATGTTTTGCCGCGCATAGAAGCCGGGT
GCCAACACTTTAATCATGATTAAGCCGATTAAACCGAAAGAATAGGCAATCAGCGCGTGT
TGCGTCATCTGCGCGTCAAACAGCGTAAATTCGCGGTACATAAACAGCGTCGCCACCAGC
GGGAACGACAACACCGCCAGTCCGACCGCCGCCGGCAGCGTCAGCAGCATGCACAGGCGC
AAACCCCAGTCGAGCAGGGCGGAAAACTGTTCCGTATCTTGGTTTGCCGAGTGTTTGGAC
AAAGTCGGCAGCAAAATCGTACCGAGTGCCGCCCCCAGCACGCCGCTGGGCAGCTCCATC
ATGCGGTCGGCGTAATACATCCATGAAACGCTGCCCGATTGCAGATAAGACGCGAAAATC
GTGTTGATCACCAAAGAAACCTGCGCCACGCTCACGCCCAAAATCGCAGGCGCCATCTGT
TTCATCACGCGGTTGACCGCCGCATCTTTGAAACTCAGTTTGGGCAGTTTCAAAAAGCCC
AGTTTCGCCAGCCAGGGCAGTTGGAAGCCGAGTTGCAAAATGCCGCCGACAAAGACCGCC
CACGCCAGCGCGGTAACGGGCGGATCGAAATACGGCACGAAAAACAGCGCGAATACGATA
AACGACACGTTCAGAAACGTGGGCGTAAACGCCGGAATGCCGAACTTATGATAAGAATTG
AGTACCGAGCCGACAAATGAAGACAGGGAAATCAATAATATATAAGGAAACGTAATCCGC
AGCAAATCGATGGAGAGCTGAAATTTGTCGGCATCTTGGGCAAAACCGGGTGCGGAAACA
TAAATCACCCAAGGCGCGGCAAGTATGCCCAGCGCGGTAACGATAACCAGTACAAACGAC
AGCATCCCCGCCACATGGCGGATAAAAGCCTCCGCCGCCTCTTTTGAACGCGTTTCCTTG
TATTCCGCCAAAATCGGCACAAACGCTTGGGCAAACGCCCCCTCCGCAAACACGCGGCGA
AGCAGGTTGGGCAGTTTGAACGCGACAAAAAACGCATCCGTCGCCATACCCGCGCCGAAT
GCCCGCGCAATGACCGTATCGCGCACAAATCCCAAAACGCGCGACACCATCGTCAGGCTG
CCGACTTTTGCCAAAGCTCCCAGCATATTCATCATTGTTCCTCAACAGTCGTACCCGTCT
GGGGCAACGGCGCGTATTGTACGACAGAAACCGCTTCAGACGGCATCGGGTTTGATGCCG
TCTGAAGCGGTTTCCTGAAACGAAAACGTCCTTTTCCGGCGGCAAACTGTATCAATACGC
GGAAATGCAATAAAATAGCCGGATTCCGATTGATTTCCAACATCTGTTTCCAACATCACG
GAGAACCGTATGAAATCCAGACACCTTGCCCTCGGCGTTGCCGCCCTGTTCGCCCTTGCC
GCGTGCGACAGCAAAGTCCAAACCAGCGTCCCCGCCGACAGCGCGCCTGCCGCTTCGGCA
GCCGCCGCCCCGGCAGGGCTGGTCGAAGGGCAAAACTATACCGTCCTTGCCAACCCGATT
CCCCAACAGCAGGCAGGCAAAGTCGAAGTCCTTGAGTTTTTCGGCTATTTCTGTCCGCAC
TGCGCCCACCTCGAACCTGTTTTAAGCAAACACGCCAAGTCTTTTAAAGACGATATGTAC
CTGCGTACCGAACACGTCGTCTGGCAGAAAGAAATGCTGACGCTGGCACGCCTCGCCGCC
GCCGTCGATATGGCTGCCGCCGACAGCAAAGATGTGGCGAACAGCCATATTTTCGATGCG
ATGGTCAACCAAAAAATCAAGCTGCAAAATCCGGAAGTCCTCAAAAAATGGCTGGGCGAA
CAAACCGCCTTTGACGGCAAAAAAGTCCTTGCCGCCTACGAGTCCCCCGAAAGCCAGGCG
CGCGCCGACAAAATGCAGGAGCTGACCGAAACCTTCCAAATCGACGGTACGCCCACGGTT
ATCGTCGGCGGTAAATATAAAGTTGAATTTGCCGACTGGGAGTCCGGTATGAACACCATC
GACCTTTTGGCGGACAAAGTACGCGAAGAACAAAAAGCCGCGCAGTAAGCCCGTTTGAAA
AATGCCGTCTGAAACTTGGTTTTCAGACGGCATTTTGATTGGGTTTAAAACGTAAAGCCC
GTTTCCAGTTCTTCATCGCCGACCAGTTCGACCAAGAGCGCGTAGAGCGGGGCGAGTTCG
GCATAACGGCGCGATACGCGGCGCAGATAGTTTAAGAAACGCGGGATTTCCGGACGGTAT
TTGTCTTTGCCGTCGCGGTAGTACAGGCGTGCGAAGATGCCTGCAACCTTCAAGTGCCGC
TGCACGCCCATCCATTCGAACCAGCGGTAAAACTCGTCAAACGCTTCGGGGACGGGCAAG
CCGGCAGCCCGCGCCTTTTCCCAGTAGCGGATAACCAAGTCCAAGACAAATTCTTCTTCC
CATTCGATAAAGGCATCGCGCAACAGCGACACCAAATCGTAGGAAATCGGGCCGTAAAGC
GCGTCTTGGAAGTCTAAAACGCCCGGCCTGCCGCGCGTCAGCATCAGGTTGCGGACGATA
AAGTCGCGGTGCACATAGACTTTGGGCTGCGCCAACAGGGGCGGCAGCAGCGTATCGACG
GTTTGCTGCCAAAGTTGGCGTTGTTTGAATGTTAATTCGCGCCCCAATTCTTTTGCGACA
AACCATTCCGGGAACAGGTTGATTTCGCGCAACATCGTTTCACGGTCATATTCGGGCAAA
ACCCCTTCACGGCTCGCCTTCTGCAATTCGACCAACTCGCCGATTGCCTCCAAAAGCAGG

## Appendix A

GCTTTGTGCGCCGTTTCGCCCTGTTCCTGAAGCATTGCGGTCAAAAACGTCGTATTGCCC
AAGTCGTTCAATACCACAAACCCCAGATCCGTGTCCGCGTGCAATACCTGCGGCACATTG
ACCATGTCAAACAGTTTCTGCACTTTCAAATAAGGTGCGACACTCATCTTGTCGGGCGGT
GCATCCATGCAGACGACACTGCTGCCGTCTGAAAACGTTGCACGGAAATAGCGGCGGAAA
TCAGCATCCGCCGCCGCAAAAGTCAGATCGAAGTCCCGTTCGGGATAAACGGTCTGAAGC
CAATTTTTCAGTTTGATTTGTCGTTGCATAACAGTACTAAAGCATTTCAGGTTACAATAA
ACGCTATTCTAACTGGCAAACCGACTTGAGGGGCGATTTTGGCTCGTTTATTTTCACTCA
AACCACTGGTGCTGGCATTGGGCCTCTGCTTCGGCACGCATTGCGCCGCCGCCGATGCCG
TTGCGGCGGAGGAAACGGACAATCCGACCGCCGGAGAAAGCGTTCGGAGCGTGTCCGAAC
CCATACAGCCTACCAGCCTGAGCCTCGGTTCGACCTGCCTGTTTTGCAGTAACGAAAGCG
GCAGCCCCGAGAGAACCGAAGCCGCCGTCCAAGGCAGCGGCGAAGCATCCATCCCCGAAG
ACTATACGCGCATTGTTGCCGACAGGATGGAAGGACAGTCGCAGGTGCAGGTGCGTGCCG
AAGGCAACGTCGTCGTCGAACGCAACCGGACGACCCTCAATACCGATTGGGCGGATTACG
ACCAGTCGGGCGACACCGTTACCGCAGGCGACCGGTTCGCCCTCCAACAGGACGGTACGC
TGATTCGGGGCGAAACCCTGACCTACAATCTCGAGCAGCAGACCGGGGAAGCGCACAACG
TCCGCATGGAAATCGAACAAGGCGGACGGCGGCTGCAAAGCGTCAGCCGCACCGCCGAAA
TGTTGGGCGAAGGGCATTACAAACTGACGGAAACCCAATTCAACACCTGTTCCGCCGGCG
ATGCCGGCTGGTATGTCAAGGCAGCCTCTGTCGAAGCCGATCGGGAAAAAGGCATAGGCG
TTGCCAAACACGCCGCCTTCGTGTTCGGCGGCGTTCCCATTTTCTACACCCCTTGGGCGG
ACTTCCCGCTTGACGGCAACCGCAAAAGCGGCCTGCTTGTTCCCTCACTGTCCGCCGGTT
CGGACGGCGTTTCCCTTTCCGTTCCCTATTATTTCAACCTTGCCCCCAATCTCGATGCCA
CGTTCGCGCCCAGCGTGATCGGCGAACGCGGCGCGGTCTTTGACGGGCAGGTACGCTACC
TGCGGCCGGATTATGCCGGCCAGTCCGACCTGACCTGGCTGCCGCACGACAAGAAAAGCG
GCAGGAATAACCGCTATCAGGCGAAATGGCAGCATCGGCACGACATTTCCGACACGCTTC
AGGCGGGTGTCGATTTCAACCAAGTCTCCGACAGCGGCTACTACCGCGACTTTTACGGCA
ACAAAGAAATCGCCGGCAACGTCAACCTCAACCGCCGTGTATGGCTGGATTATGGCGGCA
GGGCGGCGGGCGGCAGCCTGAATGCCGGCCTTTCGGTTCTGAAATACCAGACGCTGGCAA
ACCAAAGCGGCTACAAAGACAAACCGTATGCCCTCATGCCGCGCCTTTCGGTCGAGTGGC
GTAAAAACACCGGCAGGGCGCAAATCGGCGTGTCCGCACAATTTACCCGATTCAGCCACG
ACAGCCGCCAAGACGGCAGCCGCCTGGTCGTCTATCCCGACATCAAATGGGATTTCAGCA
ACAGCTGGGGCTATGTCCGTCCCAAACTCGGACTGCACGCCACCTATTACAGCCTCAACC
GCTTCGGCAGCCAAGAAGCCCGACGCGTCAGCCGCACTCTGCCCATTGTCAACATCGACA
GCGGCGCAACTTTTGAGCGGAATACGCGGATGTTCGGCGGAGAAGTCCTGCAAACCCTCG
AGCCGCGCCTGTTCTACAACTATATTCCTGCCAAATCCCAAAACGACCTGCCCAATTTCG
ATTCGTCGGAAAGCAGCTTCGGCTACGGGCAGCTCTTTCGCGAAAACCTCTATTACGGCA
ACGACAGGATTAACACCGCAAACAGCCTTTCCGCCGCCGTGCAAAGCCGTATTTTGGACG
GCGCGACGGGGGAAGAGCGTTTCCGCGCCGGCATCGGTCAGAAATTCTATTTCAAGGATG
ATGCGGTGATGCTTGACGGCAGCGTCGGCAAAAAACCGCGCAACCGTTCCGACTGGGTGG
CATTTGCCTCCGGCAGCATCGGCAGCCGCTTCATCCTCGACAGCAGCATCCACTACAACC
AAAACGACAAACGCGCCGAGAACTACGCCGTCGGTGCAAGCTACCGTCCCGCACAGGGCA
AAGTGCTGAACGCCCGCTACAAATACGGGCGCAACGAAAAAATCTACCTGAAGTCCGACG
GTTCCTATTTTTACGACAAACTCAGCCAGCTCGACCTGTCCGCACAATGGCCGCTGACGC
GCAACCTGTCGGCCGTCGTCCGTTACAACTACGGTTTTGAAGCCAAAAAACCGATAGAGG
TGCTGGCGGGTGCGGAATACAAAAGCAGTTGCGGCTGCTGGGGCGCGGGCGTGTACGCCC
AACGCTACGTTACCGGCGAAAACACCTACAAAAACGCTGTCTTTTTCTCACTTCAGTTGA
AAGACCTCAGCAGTGTCGGCAGAAACCCCGCAGACAGGATGGATGTCGCCGTTCCCGGCT
ATATCACCGCCCACTCTCTTTCCGCCGGACGCAACAAACGACCCTGACCGTCGGAAACCT
GGCAGGAGCACCGTTCCCGCACAAGACGGCATTCCACCGACAACCCCAAACCCGCCATCA
AAGGCAGGATTCAAACGATAAGGAAAGAATGATGAAAATCAAAGCCCTGATGATTGCCGC
CGCATTGCTGGCAGCAGCCGATGTCCACGCCGCACCGCAAAAGGCAAAAACCGCATCCGC
CAAAGCTGCCAAAGCTGCCAAAGCTGCCAAAGTTGCCAAAGTTGCCAAAGTTGCCAAAGT
TGCCGCCACGGCGCAAAAAGAAGCCGCACCCGCACAACAGCAGGGCGGTATCCGCTTTTC
AGACGGCATTGCCGCCGTTGCCGACAACGAAGTCATCACGCGCCGCCGGCTTGCCGAAGC
CGTTGCCGAAGCCAAAGCCAACCTGCCCAAAGACGCGCAGATAAGCGAATCCGAGCTGTC
CCGACAGGTGCTGATGCAGCTTGTCAACCAATCCCTGATTGTACAGGCGGGCAAACGCCG
CAACATTCAAGCAAGCGAAGCGGAAATCGATGCCGTCGTCGCAAAAAATCCCGCCCTCAA
AAACCTCAGCCCCGCCCAACGCCGCGATTTTGCCGACAACATCATTGCCGAAAAAGTCCG
CCAGCAGGCAGTGATGCAGAACAGCCGCGTGAGCGAAGCTGAAATCGATGCCTTCCTCGA
GCAGGCGCAAAAACAAGGCATCACCCTGCCCGAAGGCGCACCGTTGCGCCAATACCGCGC
CCAACACATCCTGATTAAAGCCGACAGCGAAAACGCCGCCGTCGGCGCGGAAAGCACCAT
CCGCAAAATCTACGGAGAGGCCCGCAGCGGCACAGACTTTTCCAGCCTGGCGCGCCAATA
TTCGCAAGACGCGAGCGCGGGCAACGGCGGAGATTTGGGCTGGTTTGCCGACGGCGTGAT
GGTTCCCGCCTTTGAAGAAGCCGTCCACGCGCTCAAACCCGGACAGGTCGGCGCGCCCGT
CCGCACCCAATTCGGCTGGCATATCATCAAATTGAACGAAGTGCGCGATGCCGGCACACC
TCAGGAACGTATCCGCAATTCCGTGCGGCAATACATCTTCCAACAAAAAGCCGAACAGGC
AACCGTCAACCTGTTGCGTGACCTGCATTCCGGCGCGTATGTCGACATCCGCTAAGGCGG
TTTGAAGCAAAAAGCCATACCGATCGGCAAAAATCCGGGCGGTATGGCTTTTTGGATTTC
GAGTTACTTTTACACCGTCATTCATCATTCCCGCGAAAGCGGGAATCTAGAAACGAAAAG
TAACAGGAATTTATCGGGAATGGCTGGAGTTTAAAGGACTGGATTCCCGCCGTCGCGGGA
ATGACGGGATTTTGGGTTGTGGTAATTTATCGGAAAAACAAAAAAACCTATGCCGTCATT
CCCGAGCAGGCGGGAATCCGGTTATTTAAAACTGCAGAAATTTATCCGAAGCAACAACAA
TCTTTCCATCGTCATTCCCGCGTAGGCGGGAATCTAGGACGTAGAATCTAAAGAAACCGT
TTTATCCGATAAGTTTCTGTACCGAAGAATCTGGATTCCCGCTTTCGCGGGAATGACGGC
GCATAAGTTCCCGTGCGGACAGACCTAGATTCCCACCTGCGTGGGAATGACGATTCAGAA
GTTGCCTGAAACCTAAAAAACTGAAACCGAACGAGCCGGATTTCCGCTTTCGCGGGAATG

## Appendix A

ACGGGATTTTGGGTTGTGGTAATTTATCGGGAAAACGGAAACCCCTATGCCGTCATTCCC
GCGCAGGCGGGAATCTAGGACGTAGAATCTAAAGAAACCGTTTTATCCGATAAGTTTCTG
TACCGAAGAATCTGGATTCCCGCTTTCGCGGGAATGACGGCGTATAAGTTCCCGTGCGGA
CAGACCTATATTCCCACCTGCGCGGGAATGACGATTCAGAAGTTGCCCGAAACCAAAAAA
CTGAAGCCGAACGGTCTGGATTCCCGCTTTCGCGGGAATGACGGCGCATAAGTTCCCGTG
CGGACAGACCTAGATTCCCACCTGCGTGGGAATGACGATTCAGAAGTTGCCCGAAACCAA
AAAACTGAAGCCGAACGGTCTGGATTCCCGCTTTCGCGGGAATGACGGCGCATAAGTTCC
CGTGCGGACAGGCCTAGATTCCCACCTGTGTGGGAATGACGATTCAGAAGTTGCCTGAAA
CCTAAAAAACTGAAACCGAACGAGCCGGATTCCCGCTTTTACGGGAATGACGGGATTTTG
GGTTGTGGTAATTTATCGGGAAAACGGAAACCCCTATGCCGTCATTCCCGCGCAGGCGGG
AATCTAGGACGTAGAATCTAAAGAAACCGTTTTATCCGATAAGTTTCTGTACCGAAGAAT
CTGGATTTCCGCTTTCGCGGGAATGACGGCGCATAAGTTCCCGTGCGGACAGACCTAGAT
TCCCACCTGCGTGGGAATGACGATTCAGAAGTTGCCTGAAACCTAAAAAACTGAAACCGA
ACGAGCCGGATTTCCGCTTTCGCGGGAATGACGGGATTTTAGATTGCGGGTATTTATCGG
GAACGGCGGCTTGGAAGTTCATTGAAACGGAAAAACAACGGAAACCCAAAAAACCGGATT
CCCGACTGTGGGAATGATGAGATTCAGGTTTCTGTTTTTGCCGGAGTTTGCCGTATCGGG
CTTCAGACGGCATTGCCTGCCGTTGTACCCGCGGGTGCGACTGCCTTGATGTAGTTGAGC
GAGACAAACTGCTTCTCGGCATCCAATTCGGTGATTTTGAACAATGCCTGTGATTTGGGC
AGTGCGTCAAACGGAATACCGGTCGCGCGCGTGACCAGCGGCAGGCCTTCGATGCGGACG
AGGTCTTCTTTGAGGATGGTCGCGGTCAGCTCGCTTGTACCTTGCTGTTGCAGGTACACA
AGGCTCCAGTAGGCTTCCATCTGCCGTTGGAAATCGGCGTAGGCGGTATAGGCGGCATCA
AAGTCGCGCAGTGCGGCGAAAAGCTCGGCATCGCTGTTTTGATACAGCGGCTCGGCAGTG
TCGTCTATCAGGCTGATCAGCTGCTTTTGGTTGATGTAGTCGGCGGCGCGGCGCAGCGGC
GAGGTAAACCAGCCGTAATGCTGCACGCCCATGCCGATATGCGGCTCGGATTTGGTGCTC
ATGCGTACTTTTCCGGTGGGTTGGACGCGGAAGAGGCCGGGCAGGTCGTTGTCATGGAGC
ATTTGTGCCCAAGTGCTGTTGGCAAGAATCATCATCTCGCTGACCAGCGTATCGATGGGT
GAGCCGCGTTCGCGGCGGACGACGGATACCTTGCCTTCCTCATCCAATTCGATGCTGTAA
TCGTATTGCGGCGCGCGGTCGGGTTCGTATTTGCCGCGCGCTTTTTGCAGGGCGGTGGCG
AATTGATAGAACCAAATCAGGTCTTGATGGTGGGCGAACATCATTTCGCCGGCTTCGTCC
AAGCCGGTTTCGGCGTTGAAATGCGGCTCGATGGCTTGGATACGCAGGTTTGTGGCGATG
TTGACCGCTTCGATTTTGCAGGTCGGCGCGCCGACGTTGAACTCGCCGTCCACATCGAAA
TAAATGCTGACGGCAGGGCGGTGTGCGCCTGCATCAAGGCTGAACGCGGCAATCCAGTTT
TCGGGCAGCATCGTGATTTTGCCGCCGGGGAAATAAACCGTGCTCAAGCGTTCCATGATG
TTTTTTTCCATTTTGTCGCCCGGTTTAACGGCAAGTGACGGCGCGGCGATGTGGATGCCG
ACACGCTTCGTGCCGTTGTCCAAGTCGGTCAGGCTTAAAGCGTCGTCCACTTCGGTGGTT
GATTCGTCGTCAATGGAAAAGGCGGTAACGTCGGCCTTGGGCAGGTCGGGCATTTCGGGA
AGGGCAAGGTCGGGGAAGCCTGTTCCTTTAGGGAAGTATTTGATTTCAAACCCGTCTTGC
AGGTATTGGGGAATGGACGTAATGCCGCCCGTTTTTTTCGCCAATTCGTAGGCAGAGGTT
TTCAGCGCGTCGGCGGCTTTGGTAAAGGCTTTGTAGGTCAGCGACTGCTTGTCGGGCGCG
TGCAGGATGGTTTTCAAATCCGCCGCGATTTCAGACGGCATCTCGCCGCGTTTCAAGGCT
TCTGCCCAAGCGTCGATTTGCGCGTCTTGCTGTTTTTTGCGTTCGATGGCGGCAAGTGCT
TGTTTTAAAGTTTCTTCGGGCGCGGCTTTGAACACGCCTTTGGCTTTTTTGTAGAAATAC
ATCGGCGCGGCGTAAAGCGCAATCAAAGTTGCCGCCAGCTCGGTTTTGGTCGGCGCATGG
CCGTAATATTCTTCGGCGATGGCTTCGGCGGTAAATTCCTCTTCGCCGCATACTTCCCAC
AATAAATCGGTGTCGATGTCCGCCGCCTGTGCCTGCGCGTTTTCCAAAAACGCCGCCATA
TCGCCGTCAAACTCGGCAAAGACGTTGTTCGCCTTCACTTTGGTGCGTTTGCCGTGTGGG
GTATCGACTTGGTAGGTGGCATCGTTTTTTTGGATGATGGCGGCGATTTTGAATTGGCCG
GACTCTTCGTAAAAAATATTCATTTTTCGGATTTTTCTGTGGAAACTCAAGCGGGCGATT
TTAGCAGATTACCGAAAATGCCGTCTGAAAAAAGGTTGGGAGAGGGTTGGCGCGGCTTTG
CGGTGCTTGCGTTATAGTGGATTAACAAAAACCAGTACGGCGTTACCTCGCCTTAGCTCA
AAGAGAACGATTCTCTAAGGTGCTGAAGCACCAAGTGAATCGGTTCCGTACTATTTGTAC
TGTCTGCGGCTTCGTCGCCTTGTCCTGATTTTTGTTAATCCACTATACGTTTTTGACGGT
GTACAATCGCTGTTTTTGAACGGAGGATGGAATGGAGAATACAAACCGTGTGCCGGAGCA
GGCACGTTATGATGCCGAACGCAGGCAGGCAGACGAAGCATTGGCGGGCGTGTTTCCGGC
AGTCAGTATCTTCGGCAGCGCGCGCACGCCGCAGAATCATGCGGATTATGCGTTCGCCTG
CCGTCTGGCGCGGCGGCTGTCGGATTCGGGCATTGCCGTCATTTCGGGCGGCGGGCCGGG
GATTATGGAGGCGGCAAACAAGGGCGCGTTTGCAGGGAAGTCGGTTTCGGTGGGGCTGAA
CATCGTTTTGCCGCACGAGCAGAAACCGAATCCGTATCAGGACATCGCCTTGCGGTTTTC
CCGTTTTGCCGAACGCAAGGCGGTGTTTTTCCGCTATTCCCAAGCATATGTCGTGATGCC
GGGCGGCTTCGGGACGCTGGACGAATTGTTTGAAATCCTGACCTTGGTGCAGACGGGCAA
AGTGCCGCCGCGTCCGATTGTTTTGGTCGGAAAGGCGTTTTGGTCGGGCTTGGCGGAGTG
GATAAACGCGCAGCTTTTGGCGCGCGGTCTGATTTCCGAAGGGGCGGTCTCTTTGTTTGC
CATATCGGACGATGAAGACGAAATCGTTGCGTATCTGTCGGAACACGGGCTTCAGACGGC
ATAGCGTCCTGAGAGTGATGTATAATTGCAAACAATTTAACAATTTTTGATGTCTTTCCC
GAACAGGATGCCGAAATGATCAACCCCATCGCCTCGCTTTCCCCTTTAGATGGCCGTTAT
GCCCAATCCGTTGAAGCATTGCGCCCGATTTTTTCCGAATACGGCCTGATGAAGGCGCGC
GTCAAAGTCGAATTAAACTGGCTCAAAGCCCTCGCCGCCGAGCCGAAGATTGCCGAAGTG
CCGCCCTTCAGTGCCGAAACGCTTGCCGAAATCGACACGGTGATTGAAAACTTTTCATTG
GAAGACGCGGCCGCCGTCAAAGCCATCGAAGCCACCACCAATCACGATGTCAAAGCCATC
GAATATTGGCTGAAAAAACGTTTTGCCGAAGTGCCGGAAGTCGCCGCCGTGAGTGAGTTC
ATCCACTTCGCCTGCACCAGCGAAGACATCAACAACCTGTCCCACGCTTTAATGCTGCAA
GAAGCGCGTGAGGCTGTTTTGCTGCCGAAGCTGGCCGAAATCATCGAAAAACTGACCGCT
ATGGCGCACGACCTTGCCGCCGTCCCGATGATGAGCCGCACCCACGGCCAGCCCGCCACG
CCGACCACTTTGGGCAAAGAAACCGCCAATGTCGTGTACCGCCTGCAACGCCAGTTTAAA
AACCTGCAAGCGCAAGAGTTCCTCGGCAAAATCAACGGCGCGGTCGGCAACTACAACGCC

## Appendix A

CATATGGTCGCCTATCCTGATGTAGATTGGGAAACCCACTGCCGCAACTTCGTCGAAATC
AGCCTCGGTCTGACCTTCAACCCCTACACCATCCAAATCGAACCGCACGACTATATGGCG
GAATTCTTCCAAACCCTCAGCCGCATCAACACGATTCTCATCGACTTTAACCGCGACGTT
TGGGGTTATATTTCATTGGGTTACTTCAAACAAAAAGTCAAAGCAGGCGAAGTCGGTTCT
TCCACCATGCCGCACAAAGTCAACCCCATCGACTTTGAAAACTCCGAGGGCAACCTCGGT
ATGGCAAACGCCGTATTGGGCTTTTTGTCCGAAAAACTGCCGATTTCCCGCTGGCAGCGC
GACCTGACCGACAGCACCGTATTGCGCAATATGGGCGTAGGCGTGGGCTATGCCGTATTG
GGTTTCGCCGCCCACCTGCGCGGTCTGAACAAGCTCGAACCCAACCCCGCCGCGCTTGCC
GCCGATTTGGATGCCACTTGGGAGCTGCTCGCCGAGCCGATTCAAACCGTAATGCGCCGT
TACGGTGTCGCCAATCCTTACGAAAAACTGAAAGACCTGACGCGCGGCAAAGGCGGCATC
ACGCCCGAAGTGCTGAAAGGCTTTATCGGATTGCTGGAAATCCCCGCCGAAGCCAAAGCC
AAATTGCTTGAGCTGACCCCCGCGCTGTATGTGGGCAAGGCTGAAGCGTTGGCGAAACGG
ATTTGAGCGTTTACTGAAACCGATGCCGTCTGAACGCGCGTTCAGACGGCATTTTTAAGA
TAACGGGACATACGGGGGCGATATTTATGCAAGCTGTCCGATACAGACCGGAAATTGACG
GATTGCGGGCCGTCGCCGTGCTATCCGTCATGATTTTCCACCTGAATAACCGCTGGCTGC
CCGGAGGATTCCTGGGGGTGGACATTTTCTTTGTCATCTCAGGATTCCTCATTACCGGCA
TCATTCTTTCTGAAATACAGAACGGTTCTTTTTCTTTCCGGGATTTTTATACCCGCAGGA
TTAAGCGGATTTATCCTGCCTTTATTGCGGCCGTGTCGCTGGCTTCGGTGATTGCCTCTC
AAATCTTCCTTTACGAAGATTTCAACCAAATGCGGAAAACCGTGGAGCTTTCTGCGGTTT
TCTTGTCCAATATTTATCTGGGGTTTCAGCAGGGGTATTTCGATTTGAGTGCCGACGAGA
ACCCCGTACTGCATATCTGGTCTTTGGCAGTAGAGGAACAGTATTACCTCCTGTATCCCC
TTTTGCTGATATTTTGCTGCAAAAAAACCAAATCGCTACGGGTGCTGCGTAACATCAGCA
TCATCCTGTTTTTGATTTTGACTGCCTCATCGTTTTTGCCAAGCGGGTTTTATACCGACA
TCCTCAACCAACCCAATACTTATTACCTTTCGACACTGAGGTTTCCCGAGCTGTTGGCAG
GTTCGCTGCTGGCGGTTTACGGGCAAACGCAAAACGGCAGACGGCAAACAGCAAATGGAA
AACGGCAGTTGCTTTCATCACTCTGCTTCGGCGCATTGCTTGCCTGCCTGTTCGTGATTG
ACAAACACAATCCGTTTATCCCGGGAATGACCCTGCTCCTTCCCTGCCTGCTGACGGCAC
TGCTTATCCGGAGTATGCAATACGGGACACTTCCGACCCGCATCCTGTCGGCAAGCCCCA
TCGTATTTGTCGGCAAAATCTCTTATTCCCTATACCTGTACCATTGGATTTTTATTGCTT
TCGCCCATTACATTACAGGCGACAAACAGCTCGGACTGCCTGCCGTATCGGCGGTTGCCG
CGTTGACGGCCGGATTTTCCCTGTTGAGTTATTATTTGATTGAACAGCCGCTTAGAAAAC
GGAAGATGACCTTCAAAAAGGCATTTTTCTGCCTCTATCTCGCCCCGTCCCTGATACTTG
TCGGTTACAACCTGTACGCAAGGGGGGATATTGAAACAGGAACACCTCCGCCCGTTGCCC
GGCGCGCCCCTTGCTGCGGAAAATCATTTTCCGGAAACCGTCCTGACCCTCGGCGACTCG
CACGCCGGACACCTGAGGGGGTTTCTGGATTATGTCGGCAGCCGGGAAGGGTGGAAAGCC
AAAATCCTGTCCCTCGATTCGGAGTGTTTGGTTTGGGTAGATGAGAAGCTGGCAGACAAC
CCGTTATGTCGAAAATACCGGGATGAAGTTGAAAAAGCCGAAGCCGTTTTCATTGCCCAA
TTCTATGATTTGAGGATGGGCGGCCAGCCTGTGCCGAGATTTGAAGCGCAATCCTTCCTA
ATACCCGGGTTCCCAGCCCGATTCAGGGAAACCGTCAAAAGGATAGCCGCCGTCAAACCC
GTCTATGTTTTTGCAAACAACACATCAATCAGCCGTTCGCCCCTGAGGGAGGAAAAATTG
AAAAGATTTGCCGCAAACCAATATCTCCGCCCCATTCAGGCTATGGGCGACATCGGCAAG
AGCAATCAGGCGGTCTTTGATTTGATTAAAGATATTCCCAATGTGCATTGGGTGGACGCA
CAAAAATACCTGCCCAAAAACACGGTCGAAATATACGGCCGCTATCTTTACGGCGACCAA
GACCACCTGACCTATTTCGGTTCTTATTATATGGGGCGGGAATTCCACAAACACGAACGC
CTGCTTAAATCTTCCCACGGCGGCGCATTGCAGTAGCCTGCCTTCTTGTCGGATATTGCC
TTTGGCAGCCTATGCCGCTGTTTGCCGTTCGGGGCGGCGGCTTTTATAGTGGATTAACAA
AAATCAGGACAAGGCGACGAAGCCGCAGACAGTACAAATAGTACGGAACCGATTCACTTG
GTGCTTCAGCACCTTAGAGAATCGTTCTCTTTGAGCTAAGGCGAGGCAACGCCGTACTGG
TTTTTGTTAATCCACTATATTTTGCCGTTTTGAGGCCGGGGTCGGAATAACCGTTTTTTG
ATGATTTTCCCTCCCCGGCTGTGTCATCAAAACCCCAATTGCCTTTCCAAACTCTCCACC
AGATTGTCATCCAGTTTCAAAGCCTGCGACAGGCGGGCGAGGAAGACGGTTTCTTTCCGC
GACAAATCGGCACAGACCAACCTTGCCGCCAGATAGGCCTCCGCCGCCAACGCCTCATCG
TTGCCGACGGCGGCGGCGATGTCTTCGATGCTTGCGGGAAGGCGGTATTCGGCGGCGAGC
CATGCGGCAGTTTCGGGGTCTGTGCCGCTTTCCTGTTCGATAGTCCGGCGTTCGGCTTCG
TCTATCATGCCGTCTGAAGCGGCGGCGGCTATCATGGTGCGCAATACGGTACGGCTGTAT
GTTTCTTCAGTTTCTCCGGCAGGTTGGAAATCGCTTTGTGTTACGGTTGCCCGCCCTTTG
TTTTGCTGCCACATCTGATAGCCCCGGTAGGCGAGGTAGCCCAAAGCGGCGGTCGAACCG
ATTTGGTGATGGTTTTGCGGTTTTTACCGTTCAGCAGCATGGAGGCGACACCGGCAACC
AGCGCGCCTCCGCCGAATGAATTGAGCGGGCTGTCGGAGAATGTGTTGCCTTTTTTTTGA
ACCGTGCTTAAGACTTGGTTGAGCAGTCGGGTAAAGTTCATGAATTTTTCCTTTCTGTTG
CGGGAAGGGCGGTATGTTTACCCTATCCTTTTAAACGGCGGCAGGCCGGCCAATAATTGT
TGGCCGTACCGCTGTGTTTTGATGCGGTTGTCGAGGATGGTTACGCGGCCGTAGTCTTGT
TCGGTGCGGATGAGGCGGCCGACGGCCTGGATGAGTTTGATGCCGGCTTCGGGGACGGTG
ATTTCGATGAAGGGGTTGCCGCCGCGCTGTTCTATCCAGCGGTTTTGGGTTTTTTCGATG
GGGTTGTCGGGCATGGCGAAGGGAAGTTTGGCGATGATGACTTGCACGCAGGCGGTGCCG
GGCAGGTCGAGTCCTTCGGCAAAGCTGTCGAGTCCGAAGATGATGCTGGCTTTGCCTTCT
TCTATGGCCCGGTGGTGTTTTTGCAGGAGGACGGCTTTGGGTAATTCGCCTTGTACGAGC
AAGAGCGGCAGGTAGTCTCCGGGCAGGCGCAGGGCGACATCCTGCATTTGTTTGCGCGAG
GAAAACAAGACGAGCGTGCCGATGGCTTCGGTGGGCGAAATAAGCTTGGGCAGCCATTCG
ATGACGGCGGCGGTGTGGGCTTCGGGGTCTTTGGGGCTGGCGTATATGGGGGGGATGTAG
AGTTCGCCCTGTTTTTCAAAGTCAAAGGGGCTTTTGAGGGCGAGGGTGGTGGTTTCGGGC
AGCCATTGCAGCCCGGTTTGGCGCAGCATCAGGTTGAAGTTGCCCAAGGATTGCAGGGTG
GCGGAAGTCAATACCGCGCCTGCCGCACGCCGCCACAGGCTGTTGGCAAGGTGGGATGCG
CTGCTGATGGGGCTGGCGTTGAAAATGTAGTCGTTTTTGTCGTCGGCGCGGCGGGTTATC
CATTTCGCCAACGGTTCTTCACCCTCGAGGGGGACAGTGGAGAGCAAATCCCAAACCGCG

## Appendix A

CTGATTTGTTCGATACGGGCGATAAAAAGACCGAACTCGCTGGTCAGGCGGTCGAGGAGC
GCGCCGTCCTGTTCTTTTTCGCGGCGTGCGGCAGAAAGCGCATCGTTCAGCCCGATAACG
TGTTTGAGCAGGCTGCGCGCAGCAATGGCCGTATTGGAAACGGTGGTTTCGAGGCCTTCG
GGGATTTTGCCGTCTTCCCACAGCCAAGTCGGTTCGCTGTTGGTTCGTCTGTCGTTTTCA
GACACCCCCAGACTTAAAGACGGCTCTTCCGCCAAATGGAATTGCCATTCATGCAGGCTG
TCGAGCAAGGATGCGGCGGCTTCGTCGGCTAGGTTGGCAAGTTCGGCTTTATCGGTCAGC
GCGGCAATTTTGCCGGTCAGCTGCGGCAGTTTTTCCAGCGTCCAAACGGCAATATTCCAT
GAATGTTCGGCGGCAAAACGGCTGAGGGCTTTTTTGGGCAGGTGGTGCGCTTCGTCGATG
CAATAGAAACTGTTTTCGGGCGCAGGCAGAATCACGCCGCCGCCCATACTGATGTCGGCA
AGCAGAAGATCGTGGTTGGCAACGACGACATCGACGGTTTCCAAGACATCGCGTGCTAGG
TAAAACGGACATTCCGGACGGTTGGGACAGGCGGTTTTCAGGCAGCCGTGGCGGTCGTTG
GTCACTTTGAGCCAAATCGCGTCATCGATTTTTTCCGGCCAAGTGTCGCGGTCGCCGTTG
AACCGTCGGGCGGAAAATTCGTCGGCGATGTCGCGCAGCAGCTTCAATTCTTCGGGCTTG
GGTTTGCTGTCCCACAAGACGGCGGGGGCTTCAAAGCCGAGCAGGTTTTGCTGGGCATTG
CTTTGCGTCAGTCGATAGAGTTTGTAGGGGCAGAGATAGCGGCCGCGCCCTTTGGCAAGT
GCGAAGGTCAGTTCCAAACCGCTTTTTTCGACCAGAAACGGCAGGTCGCGGTCTACCAAC
TGCTCCTGCAAGGCAACCGTCGCGCTGCTCACAATCAGCCGCTTGCCGCGTGTTTGCGCC
ATGATGCCGCCGGCCAAAAGGTAGGCCAACGATTTGCCCACGCCGGTCGGCCCTTCGATC
ACGGCAATGCTCTCGCCTTCGCGCTTGGGCGGCTCGCCGCCTTCTTCGCGCGCCAACGTC
CGCGAAAAAGCGTTGGCAACCGCCGCAATCATTTCCCGCTGCGAAGCACGCGGACGGAAA
CCGGGCAGGTTTTTGCCGATGTTTTGGTAATGGTCGCGGATGGCGTTTTTTTCTAAATCG
GTGAGCATGGCGTTTTGTACGGCGGTAGAAGTGGGCTTATTTTAACATTGCACGGAAGCG
GTACAATATCGTTGTCGGAATGGGGGGTGAGGTGAATCGTGCGGACGTGGTTGGTTTTTT
GGTTGCAGCGTTTGAAATACCCGTTGTTGCTTTGGATTGCGGATATGTTGCTGTACCGGT
TGTTGGGCGGCGCGGAAATCGAATGCGGCCGTTGCCCTGTGCCGCCGATGACGGATTGGC
AGCATTTTTTGCCGGCGATGGGAACGGTGTCGGCTTGGGTGGCGGTGATTTGGGCATACC
TGATGATTGAAAGTGAAAAAAACGGAAGATATTGAGTCATTCGGACGCAATGCCGTCTGA
AACGGAAGTTCAGACGGCATTTGTTTTAGGTTGCCGTACCGCTTAGGGAATACCGGCGAC
AGGATGGGCGGGATAGCCGTGGGTATCGACCGAACAGGCAAACCGCCAAGGCGTGTGGAC
GGTGTCGGCGGACAGGTGGGCAAGCTCGGGAATGTGCCGTCTGACAAAGGTGCCGTCGGG
GTCGGTTTTGTGTGCGGCGGCGGCAATGTCGGGGCAGGTGTGCCGTGAGGCGGCAAGCCG
CCAGTTGCCTTGGTTGATTGCTGCATCGAAATCGGTCAGCTGTCGGGCAAACCATATCTC
GCCTTCGCGGCGGGGGAGGTTTAAAACGTGGCAGAAAAAATCCGCGCTCAAGCGTCTCAG
GGCGGGGTGGAGGCTGCCGGTTTTGTGCAAACAGCGCATCGCGGCATCGATAATCGGAAT
GCCGGTCCGGCCCTGCTGCCAAAGCGTCAGGCGCAGGGTGTGTTCAGGATTGCCGTCTGA
AGGGTCGTCATCCGTGTGCTGCAAGGCAAGTTGAAGGAAAAAATCGCGGCGGATGATGTT
GTCCGCCCACGCGTTCAGACGGCGTTCGAGGCTTTCCCGCGCGAGCAGGCGCGGCGAGAT
GCAGCCGGCACTCAAATACGCGCCCATCAGCGAAGTGTGTTTGCGCGAGGGGAAATCCTT
TAAAACGGAGTAGGAATCCGCCTGTTCGAGAAACCGCCGCCACTGCCGCCAAGCCGCCGT
TTCGCCGCTGTTTTGCGGCAGGAAGATGCCGTCTGAAAGCGCGGCAGGCTGCGGGGCGGA
AAGGTTTTCGGGGAAGGGTTGGCGGTATGCCGCGAATAGGTCCGGACCGGCGGGGGGCTG
CTTGGAAAAGCGGTCGAGCCATACTTCGCGGTAGCGGTCGAAATCGGCATATGCCGTGCC
GCCGTCGGGTATCAGGTCGGTTTTGCCGAAAACGGCGCGGTCGTTGACGAAGGTTAACGC
GATGCCGTGTTTGTCCAATTCGTGCCAAAGGGCGTTGTCGGCGAGTTTGTCGGCAAAAGT
ATGGGATTCGTCGGCGATGACGGTGCGGATATTGAGGCGGACGGCGAGCCGGACGAGCTC
GGCAGGAGATGCCGCCGTGTAGAGCGGGATGCCGCGCCCTGCAAGCCCTTGGGCGAGTTC
GGCGGCGGATTGGCGGTAGAACGCGGCGCGGCGAGGGTTGTCTGTTTCGGCATCGTCAAT
CCAAATGCCGATAATGGGCAAACTTCGGCAACGGCGGCGCATAAGGCGGCGTTGTCGCGG
ATGCGGAGGTTTTGGCGGAACCAGACGAGCGTGTGTGCGGCGCACGTGTCCGCATAAAGG
GGGCGGGCGGTTTCAGACGGCATTTCGGCAGCCTTTCCTGCTGGCGATTTTTTCGTTCAG
AAAATCGATGAAGCTGCGGACTTTCGCGCTTAAGAATGCCCTGTCTGCATAAACGGCATT
CAGCCGGTCGGTCGGGACGGCGTATCCGGGCAGCAGCCTCACCAGCGTGCCGCAGCGCAA
ATCGTGTTCCGCCGCCCAAAGCGGCTGATAACCGATGCACGCGCCCGCCTTAATCATTTC
GCGCATCATCAGCGTGTTGTCGGTACGGATGACGGGGTCAGTTCAAGCCGGTATTTTTT
GCCGTCCGATTTGCGGGTGAGGTCGAGTTTCTGCTGGTTGGTGTAGGTCGGCAGGACGGC
GGGCAGCCCCGCCACTTCTTCCGGCGTTTCCGGCACGCCGTTGCGCCTCAGGAAATCGGG
CGAGGCGAGCAGGGCAAATTCGATTTCCGCCAGTGGGCGCGCAATCAGCGACGGGGACAG
GGTTTGGGAAACGCGCAACGCCAAATCCACGCCTTCGGCAATCAAATCGACGTGGCGGTT
GTCCAAAATCAGTTCTAATGCCACTTCGGGATAACGTTCGCGGTATTCCGCCAGCCAGTT
GCATATCTGGCTGCCGGCAAACCACAGCGGCATCGTTACGCGCAGCAGCCCCTGCGGTTT
TTCCGTCCCCCCGGCGGCTTTTTGCGCGGCATCGTCGAGCGTGTCGAGCGCGTAACTGCA
TTGCCGGTAGTATTCTTCCCCGGCTTCGGTCAGGCTGAGGTTGCGGCTGTTGCGGTGCAG
GAGTTTGGCTTGGACGGTGTTTTCCAAGTGGCTGACGTGTTTGCTTGCCATTGCGGTGGA
GATGCCGAGCGCGTCGGCGGCGCGGGTGAAGCCGCCGCTTTGGACGACTTGGCGGAAAAC
CTTGAGGCTGAACAGGGTGTCCATATTTTCTTGTGTGGAAAAGTTGTATCAATAAAAGCA
GTATATATTTGAAAAGGGGAAACATCTATACTCTACCGCCTGAAATGAAGACAAATATCA
AAGGAGCTTTTATGTCCGATTGCTGCAACCGTATCCAACCGGTTTTGCTTTCTGTTTTGC
GTATCGTAACCGCCTACCTGTTTTTGTTGCACGGTACGTCGAAAATCTTCGCCTTCCCCA
TTGAAATGGGCAGCGGTTCGCCCGGCGGGCTGTTGCTGCTTGCCGGTATTTTAGAAATTG
TCGGCGGCATTTTGCTGGTGTTGGGCCTGTTTGCGCGCCCTGCCGCGTTTGTTTTGTCCG
GCCAGATGGCGGTTGCCTATTTTATGGCGCACGCTTCCGGAAATGCTTTGTTCCCGATTG
CCAACGGCGGCGAGTCCGCAGTGCTGTTCTGCTTCGTATTCCTCTATATCGCGGCGGCGG
GCGGCGGAGCATGGTCGCTGGACAGGCTGTTTTTCAAGCGTAAAGCCTGAATCGGACTGC
CTAAAGTGTATTTTGTTGAATGTTTTTGAGGAAAAGAAATGACCCGTCAATCTCTGCAAC
AGGCTGCCGAAAGCCGCCGTTCCATTTATTCGTTAAATAAAAATCTGCCCGTCGGCAAAG

# Appendix A

ATGAAGTTGTCCAAATCGTCGAACACGCCGTTTTGCACACACCTTCTTCGTTCAATTCCC
AATCTGCCCGCGTGGTCGTGCTGTTTGGCGAAGAGCATGATAAGGTGTGGCAATTTGTCG
AAGACGCGCTGCGTGCCGTCGTGCCTGCCGACAGTTTTGAACCGACCGCGCAAAAATTGA
ACCTGTTTAAGGCGGGTGCGGCAACCATTTTGTTTTATGAAGATCAAAATGTCGTCAAAG
GTTTGCAGGAGCAGTTCCCTGCTTATGCCGCTAACTTCCCCGTTTGGGCGGATCAGGCAA
ACGCGATGGTGCAGTATGCCGTTTGGACGACACTTGCCGCGGTCGGCGTAGGTGCAAACC
TGCAACATTACAATCCCTTGCCCGATGCGGCGATTGCCAAAGCGTGGAATATCCCCGAAA
ACTGGTTGTTGCGCGCACAAATGGTTATCGGCGGTATTGAAGGGGCGGCAGGTGAAAAGA
CCTTTGAACCCGTTGCAGAACGTTTGAAAGTGTTCGGCGCATAATTTCGCGGTCAAAAAA
ATGCCGTCTGAACCCTGTTCAGACGGCATTTTTCAGTATCAGGCGGCGAGTTTTCCGCAT
TCTGAGACCTTTGTTTACAAATATCATGTTCAATATAGTTAAAAGAAATTATTCTCATTT
CCTCCGTGAGGCAATATAATTCGGTTGTTTTGTTAAATTGAGTATAAAAATGAAAATATC
ATTTCATTTAGCTTTATTACCCACGCTGATTATTGCTTCCTTCCCTGTTGCTGCCGCCGA
TACGCAGGACAATGGTGAACATTACACCGCCACTCTGCCCACCGTTTCCGTGGTCGGACA
GTCCGACACCAGCGTACTCAAAGGCTACATCAACTACGACGAAGCCGCCGTTACCCGCAA
CGGACAGCTCATCAAAGAAACGCCGCAAACCATCGATACGCTCAATATCCAGAAAAACAA
AAATTACGGTACGAACGATTTGAGTTCCATCCTCGAAGGCAATGCCGGCATCGACGCTGC
CTACGATATGCGCGGTGAAAGCATTTTCCTGCGCGGTTTTCAAGCCGACGCATCCGATAT
TTACCGCGACGGCGTGCGCGAAAGCGGACAAGTGCGCCGCAGTACTGCCAACATCGAGCG
CGTGGAAATCCTGAAAGGCCCGTCTTCCGTGCTTTACGGCCGCACCAACGGCGGCGGCGT
CATCAACATGGTCAGCAAATACGCCAACTTCAAACAAAGCCGCAACATCGGAGCGGTTTA
CGGCTCATGGGCAAACCGCAGCCTGAATATGGACATTAACGAAGTGCTGAACAAAAACGT
CGCCATCCGTCTCACCGGCGAAGTCGGGCGCGCCAATTCGTTCCGCAGCGGCATAGACAG
CAAAAATGTCATGGTTTCGCCCAGCATTACCGTCAAACTCGACAACGGCTTGAAGTGGAC
GGGGCAATACACCTACGACAATGTGGAGCGCACGCCCGACCGCAGTCCGACCAAGTCCGT
GTACGACCGCTTCGGACTGCCTTACCGCATGGGGTTCGCCCACCGGAACGATTTTGTCAA
AGACAAGCTGCAAGTTTGGCGTTCCGACCTTGAATACGCCTTCAACGACAAATGGCGTGC
CCAATGGCAGCTCGCCCACCGCACGGCGGCGCAGGATTTTGATCATTTCTATGCAGGCAG
CGAAAATGGCAACTTAATCAAACGTAACTACGCCTGGCAGCAGACCGACAACAAAACCCT
GTCGTCCAACTTAACGCTCAACGGCGACTACACCATCGGCCGTTTTGAAAACCACCTGAC
CGTAGGCATGGATTACAGCCGCGAACACCGCAACCCGACATTGGGTTTCAGCAGCGCCTT
TTCCGCCTCCATCAACCCCTACGACCGCGCAAGCTGGCCGGCTTCGGGCAGATTGCAGCC
TATTCTGACCCAAAACCGCCACAAAGCCGACTCCTACGGCATCTTTGTGCAAAACATCTT
CTCCGCCACGCCCGATTTGAAATTCGTCCTCGGCGGCCGTTACGACAAATACACCTTTAA
TTCCGAAAACAAACTCACCGGCAGCAGCCGCCAATACAGCGGACACTCGTTCAGCCCCAA
CATCGGCGCAGTGTGGAACATCAATCCCGTCCACACACTTTACGCCTCGTATAACAAAGG
CTTCGCGCCTTATGGCGGACGCGGCGGCTATTTGAGCATCGATACGTTGTCTTCCGCCGT
GTTCAACGCCGACCCCGAGTACACCCGCCAATACGAAACCGGCGTGAAAAGCAGTTGGCT
GGACGACCGCCTCAGCACTACGTTGTCTGCCTACCAAATCGAACGCTTCAATATCCGCTA
CCGCCCCGATCCAAAAAACAACCCTTATATTTATGCGGTTAGCGGCAAACACCGTTCGCG
CGGCGTGGAATTGTCCGCCATCGGGCAAATCATCCCCAAAAAACTCTATCTGCGCGGTTC
GTTGGGCGTGATGCAGGCGAAAGTCGTTGAAGACAAAGAAAATCCCGACCGAGTGGGCAT
CCATTTGAATAATACCAGCAACGTTACCGGCAACCTGTTTTTCCGTTATACCCCGACCGA
AAACCTCTACGGCGAAATCGGCGTAACCGGTACAGGCAAACGCTACGGTTACAACTCAAG
AAATAAAGAAGTGACTACGCTTCCAGGCTTTGCCCGAGTTGATGCCATGCTTGGCTGGAA
CCATAAAAATGTTAACGTTACCTTTGCCGCAGCCAATCTGCTCAATCAAAAATATTGGCG
TTCGGACTCTATGCCGGGTAATCCGCGCGGCTATACTGCCCGGGTAAATTACCGTTTCTG
ATGAAATCAGGCAAAGGCTGAAATAAAACTAAACACATTTTTTCACTCAAATCGAACACG
GCTTCAATAAAATGCCATAAAATCCGCACATTAATCTGACACACAAGAGATACCTATGAA
ACTGAAAACCTTAGCTTTGACTTCATTGACCCTGTTGGCATTGGCCGCTTGTAGCAAACA
GGCTGAAACCAGTGTTCCGGCAGACAGCGCCCAAAGCAGCTCATCTGCTCCGGCAGCCCC
TGCTGAGTTGAACGAAGGTGTGAACTACACTGTATTGTCTACGCCTATTCCGCAACAGCA
GGCCGGTAAAATCGAAGTATTGGAATTTTTCGGCTACTTCTGCCCGCATTGCGCCCATCT
TGAGCCGGTCTTGAGCGAGCACATCAAAACGTTTAAAGACGATACCTATATGCGCCGGGA
GCATGTCGTGTGGGGTGATGAAATGAAACCTTTGGCACGTTTGGCGGCCGCAGTGGAAAT
GGCCGGTGAATCAGATAAAGCCAACAGCCATATTTTCGATGCGATGGTTAATCAAAAAAT
CAATCTGGCCGATACCGATACCCTGAAAAAATGGCTGTCCGAGCAAACAGCGTTTGACGG
CAAAAAAGTATTGGCTGCATTTGAGGCTCCTGAAAGCCAAGCGCGTGCGGCTCAAATGGA
AGAGTTGACCAATAAATTCCAAATCAGCGGCACACCGACTGTGATTGTCGGCGGCAAATA
CCAAGTTGAATTTAAAGACTGGCAGTCCGGTATGACCACGATTGACCAGTTGGTGGATAA
AGTACGCGAAGAGCAGAAAAAGCCGCAATAAGTTGAGGATTGAATGAGTAAAGGCCATCT
GAAAATAGGATTTCAGACGGCCTTTTGTATTTAGGCTTTATAGAAGAGATGATTGCTTAA
AGCCTTATGGTTTTAAATCAGAATATATAGCGGATTAACAAAAACCAGTACGGCGTTGGC
TCGCCTTAGCTCAAAGAGAACGATTCTCTAAGGTGCTGAAGCACCAAGTGAATCGGTTCC
GTACTATCTGTACTGTCTGCGGCTCGCCGCCTTGTCCTGATTTTTGTTAATCCACTATAA
ATCAGAATATAAAACAAAAACGCCGTCTGAAATTTCAGACGGCGTTTTCTGTTAAATCGG
CTTACAAACCCGGGAACATCCCTTTTATCCCCCTCATTCCTTTCGCCATACGCATCAGTT
TGCCCAAGCCGTTGCCGCTGAACATCTTCATCATTTGTTGCATTTGTTCAAACTGTTTGA
GCAATTTGTTCACTTCCTGCACGGTTGTGCCCGCACCCATTGCAATACGGCGTTTGCGGC
TGGCTTTGAGCAGGGCAGGGTTGGCGCGTTCTTTAGGGGTCATCGAGTTGATGATGGCTT
CTACTTTGCCCATCGCTTTTTCAGCCGTTCCTTCGGGGATTTGTTTCGAGATTTGACCCA
GTTCGCCCGGCATTTTCGACATCAGGTTTTCCAAACCGCCCATATTGCGCATTTGCTGGA
TTTGTTCTTTAAAGTCGTTGAGGTCGAAGCCTTTGCCTTTGTGCAGCTTTTTCGCCATTT
TAGCGGCGGCTTCTTCGTCTATACCTTTTTGAACGTCTTCAATCAGGGTCAATACGTCGC
CCATACCCAAAATGCGGCCGGCAAGACGGTCGGGGTGGAAAGGTTCGAGGCCGTTGATTT

## Appendix A

TTTCGCCGACACCGATAAATTTAATCGGTTTGCCGGTTACGTGGCGTACGGACAATGCCG
CACCGCCGCGCGAGTCGCCGTCCATCTTGGTCAATACGACTCCGGTCAGCGGCAGGGCTT
CATTAAATGCCTGAGCAGTGTTCACCGCATCCTGACCCAGCATCGCATCGATGACGAACA
AAGTTTCCACCGGGTTAACCGCCGCGTGAAGGGCTTTGATTTCGTTCATCATCTCTTCAT
CGATTGCCAAACGGCCGGCGGTATCGACCATCAATACATCGTAAAAATGTTTTTGGCGT
AATCGACGGCGGCAGTTGCAATTTCAACCGGTTTTTGGTTGGTATCGGACGGGAAAAAAT
CCACGCCGACCTGTTCGGCCAACAGACGCAGCTGTTCAATCGCGGCAGGACGGTAAACGT
CGGCGGATACCACCAAAACCTTTTTCTTCTGATCGTTTTTCAACAGGCGGGCGAGTTTGC
CGACGGTCGTCGTCTTGCCTGCGCCCTGCAAACCTGCCATCAACACGACGGCGGGCGGCG
CAACCGACAAATCCAGCGTTTTGTTTTCCCTGCCCATCAGTTCGGTCAGGGCTTTGTTGA
CCACGCCGATAAATGCCTGATCCGGCGTCAGGCTGCCCGCTACTTCCTGACCGAGGGCCT
TTTCTTTGACGTTGTTGATGAACTCTTTGACGACAGGCAGGGCGACATCCGCCTCAAGCA
GGGCGAGGCGGACTTCGCGCAAGGCCTCTTTAATATTGTCTTCGGTCAGTTTGGCCTGCC
CCCGGATGTTTTTGAAGACATTGCTGAAGCGGCCGGTTAAATTGTCTAACATACTGGTCC
TTGGTCTGAATAAGAATAGCTTGCCCCATCAGGGGCATTCTTTGTTAAAATAAAATCAAA
ATAATTTGATGCGGCTTGTGTGCCGGACAGCATATCGGCAAATCCGTCAAGGCTTGACCG
AAATGGGGATTTTACAATTCCAACGTTAAAAGTTCCAATATTTCATAAGCGGCCGCATAC
GGCGCAACAGTATAGATAGAGAAAGTCCACCATGCCGACAGTTTTCATCTTTTTGACGGC
GGTTTACGCAGGATTGGGTGCATTTGCATGGCACTGCCAACAGCAGGGGTGCGGCCGGGA
TTACCCGTGGAAGACGGAATTGCCGGTTTTGGGTGCGGCATTGACCGTCCACGGCGCGGC
ACTGCTTATGCCGGTCATTCAAGACAAAATCATCATTATGGGCTTCGGGTATTCCGGCAG
CCTGATTGTTTGGATGATGCTGTTTATTTATTTTGCCGGCAGCTTCTTTTATCCGCTGCG
CGGAGTGCAGTTGCTGCTGTATCCTTGCGCCGCACTGATGCTGCTGTCAGGTTTGGTTTT
TCCTGGAAAATTCTCGGGATATGAAATTACCGACCTTCCCTTTATGCTGCATATCGGAAC
TTCGCTGCTCGCATACGGGCTGTTCGGCATCGCAACATTATTGTCCGTTTTGACCCTGCT
GCTGAATCGGAGCCTGCACCGCAGGAGCTTCTCCAAGCTCGCAGGATTCCTGCCGTCGCT
GCTCAGTTTGGAAAAACTCATGTTCCAGGCCATGTGGGCAGGTTTCATCCTGCTGACCTA
TTCCGTCGTCAGTGGAACATTTTTGCCGAAGCCGTATTCGGCAAACCCATGACCTTTAC
CCATAAAACCGTATTCGGCATATTGTCATGGCTGATTTACGGCGGACTGCTGCTCAAGCA
CAGCATGACCGCATGGCGCGGCAAAAAAGCCGCCGTGTGGACCATCATCGGATTTGTCAG
CCTTATGATTGCCTATATGGGCAGCAAGTTCGTATTGGAAATCATTCTGAAAAGATAAGA
AGAGCCAACAGATGCCGTCTGAGTCCCCGAGTTTCAGACAGCATATTCACAAAGGCGCAC
CAGCCGGAGGAGGGAGAGGAAAGGATTGTTGGAGGCGGCGCAGTATTTAGCAGAAATAAA
AAACCTTATCCGACAGCGACATGACGAATTTCCCCAAAAAAATCCCGCTGAAAGCATTGA
CCGTTTTTCCCTGTGGGCGTATAGTTCGGTTCTTCGCTGCTGCAGAAGTGGCGGACGAAC
TGAAAAGTATAGCACAGAATGTTGGGGATATCGAGAGATATCTTGACAGGCGGAAGGAAT
ACTTTATAATTCGCAACGCTCTTTAACAAAACAGATTACCGATAAGTGTGAGTGCCTTGA
GTCTCACACTGTTTGAAAGACAGACAAGATAATGTTTTGAACATTGTCCTGTTGGTTTCT
TTGAAGCAGACCAGAAGTTAAAAAGTTAGAGATTGAACATAAGAGTTTGATCCTGGCTCA
GATTGAACGCTGGCGGCATGCTTTACACATGCAAGTCGGACGGCAGCACAGAGAAGCTTG
CTTCTCGGGTGGCGAGTGGCGAACGGGTGAGTAACATATCGGAACGTACCGAGTAGTGGG
GGATAACTGATCGAAAGATCAGCTAATACCGCATACGTCTTGAGAGAGAAAGCAGGGGAC
CTTCGGGCCTTGCGCTATTCGAGCGGCCGATATCTGATTAGCTAGTTGGTGGGGTAAAGG
CCTACCAAGGCGACGATCAGTAGCGGGTCTGAGAGGATGATCCGCCACACTGGGACTGAG
ACACGGCCCAGACTCCTACGGGAGGCAGCAGTGGGGAATTTTGGACAATGGGCGCAAGCC
TGATCCAGCCATGCCGCGTGTCTGAAGAAGGCCTTCGGGTTGTAAAGGACTTTTGTCAGG
GAAGAAAAGGCTGTTGCTAATATCAGCGGCTGATGACGGTACCTGAAGAATAAGCACCGG
CTAACTACGTGCCAGCAGCCGCGGTAATACGTAGGGTGCGAGCGTTAATCGGAATTACTG
GGCGTAAAGCGGGCGCAGACGGTTACTTAAGCAGGATGTGAAATCCCCGGGCTCAACCCG
GGAACTGCGTTCTGAACTGGGTGACTCGAGTGTGTCAGAGGGAGGTAGAATTCCACGTGT
AGCAGTGAAATGCGTAGAGATGTGGAGGAATACCGATGGCGAAGGCAGCCTCCTGGGACA
ACACTGACGTTCATGCCCGAAAGCGTGGGTAGCAAACAGGATTAGATACCCTGGTAGTCC
ACGCCCTAAACGATGTCAATTAGCTGTTGGGCAACCTGATTGCTTGGTAGCGTAGCTAAC
GCGTGAAATTGACCGCCTGGGGAGTACGGTCGCAAGATTAAAACTCAAAGGAATTGACGG
GGACCCGCACAAGCGGTGGATGATGTGGATTAATTCGATGCAACGCGAAGAACCTTACCT
GGTCTTGACATGTACGGAATCCTCCGGAGACGGAGGAGTGCCTTCGGGAGCCGTAACACA
GGTGCTGCATGGCTGTCGTCAGCTCGTGTCGTGAGATGTTGGGTTAAGTCCCGCAACGAG
CGCAACCCTTGTCATTAGTTGCCATCATTCAGTTGGGCACTCTAATGAGACTGCCGGTGA
CAAGCCGGAGGAAGGTGGGGATGACGTCAAGTCCTCATGGCCCTTATGACCAGGGCTTCA
CACGTCATACAATGGTCGGTACAGAGGGTAGCCAAGCCGCGAGGCGGAGCCAATCTCACA
AAACCGATCGTAGTCCGGATTGCACTCTGCAACTCGAGTGCATGAAGTCGGAATCGCTAG
TAATCGCAGGTCAGCATACTGCGGTGAATACGTTCCCGGGTCTTGTACACACCGCCCGTC
ACACCATGGGAGTGGGGGATACCAGAAGTAGGTAGGATAACCACAAGGAGTCCGCTTACC
ACGGTATGCTTCATGACTGGGGTGAAGTCGTAACAAGGTAGCCGTAGGGGAACCTGCGGC
TGGATCACCTCCTTTCTAGAGAAAGAAGAGGCTTTAGGCATTCACACTTATCGGTAAACT
GAAAAAGATGCGGAAGAAGCTTGAGTGAAGGCAAGATTCGCTTAAGAAGAGAATCCGGGT
TTGTAGCTCAGCTGGTTAGAGCACACGCTTGATAAGCGTGGGGTCGGAGGTTCAAGTCCT
CCCAGACCCACCAAGAACGGGGGCATAGCTCAGTTGGTAGAGCACCTGCTTTGCAAGCAG
GGGGTCATCGGTTCGATCCCGTTTGCCTCCACCAATACTGTACAAATCAAAACGGAAGAA
TGGAACAGAATCCATTCAGGGCGACGTCACACTTGACCAAGAACAAAATGCTGATATAAT
AATCAGCTCGTTTTGATTTGCACAGTAGATAGCAATATCGAACGCATCGATCTTTAACAA
ATTGGAAAGCCGAAATCAACAAACAAAGACAAAGCGTTTGTTTTGATTTTTTATTCTTTG
CAAAGGATAAAAATCTCTCGCAAGAGAAAAGAAAACAAACACAGTATTTGGGTGATGATT
GTATCGACTTAATCCTGAAACACAAAAGGCAGGATTAAGACACAACAAAGCAGTAAGCTT
TATCAAAGTAGGAAATTCAAGTCTGATGTTCTAGTCAACGGAATGTTAGGCAAAGTCAAA

# Appendix A

GAAGTTCTTGAAATGATAGAGTCAAGTGAATAAGTGCATCAGGTGGATGCCTTGGCGATG
ATAGGCGACGAAGGACGTGTAAGCCTGCGAAAAGCGCGGGGGAGCTGGCAATAAAGCAAT
GATCCCGCGATGTCCGAATGGGGAAACCCACTGCATTCTGTGCAGTATCCTAAGTTGAAT
ACATAGACTTAGAGAAGCGAACCCGGAGAACTGAACCATCTAAGTACCCGGAGGAAAAGA
AATCAACCGAGATTCCGCAAGTAGTGGCGAGCGAACGCGGAGGAGCCTGTACGTAATAAC
TGTCGAGATAGAAGAACAAGCTGGGAAGCTTGACCATAGTGGGTGACAGTCCCGTATTCG
AAATCTCAACAGCGGTACTAAGCGTACGAAAAGTAGGGCGGGGCACGTGAAATCCTGTCT
GAATATGGGGGGACCATCCTCCAAGGCTAAATACTCATCATCGACCGATAGTGAACCAGT
ACCGTGAGGGAAAGGCGAAAAGAACCCCGGGAGGGGAGTGAAACAGAACCTGAAACCTGA
TGCATACAAACAGTGGGAGCGCCCTAGTGGTGTGACTGCGTACCTTTTGTATAATGGGTC
AACGACTTACATTCAGTAGCGAGCTTAACCGAATAGGGGAGGCGTAGGGAAACCGAGTCT
TAATAGGGCGATGAGTTGCTGGGTGTAGACCCGAAACCGAGTGATCTATCCATGGCCAGG
TTGAAGGTGCCGTAACAGGTACTGGAGGACCGAACCCACGCATGTTGCAAAATGCGGGGA
TGAGCTGTGGATAGGGGTGAAAGGCTAAACAAACTCGGAGATAGCTGGTTCTCCCCGAAA
ACTATTTAGGTAGTGCCTCGAGCAAGACACTGATGGGGGTAAAGCACTGTTATGGCTAGG
GGGTTATTGCAACTTACCAACCCATGGCAAACTAAGAATACCATCAAGTGGTTCCTCGGG
AGACAGACAGCGGGTGCTAACGTCCGTTGTCAAGAGGGAAACAACCCAGACCGCCAGCTA
AGGTCCCAAATGATAGATTAAGTGGTAAACGAAGTGGGAAGGCCCAGACAGCCAGGATGT
TGGCTTAGAAGCAGCCATCATTTAAAGAAAGCGTAATAGCTCACTGGTCGAGTCGTCCTG
CGCGGAAGATGTAACGGGGCTCAAATCTATAACCGAAGCTGCGGATGCCGGTTTACCGGC
ATGGTAGGGGAGCGTTCTGTAGGCTGATGAAGGTGCATTGTAAAGTGTGCTGGAGGTATC
AGAAGTGCGAATGTTGACATGAGTAGCGATAAAGCGGGTGAAAAGCCCGCTCGCCGAAAG
CCCAAGGTTTCCTGCGCAACGTTCATCGGCGTAGGGTGAGTCGGCCCCTAAGGCGAGGCA
GAAATGCGTAGTCGATGGGAAACAGGTTAATATTCCTGTACTTGATTCAAATGCGATGTG
GGGACGGAGAAGGTTAGGTTGGCAAGCTGTTGGAATAGCTTGTTTAAGCCGGTAGGTGGA
AGACTTAGGCAAATCCGGGTCTTCTTAACACCGAGAAGTGACGACGAGTGTCTACGGACA
CGAAGCAACCGATACCACGCTTCCAGGAAAAGCCACTAAGCTTCAGTTTGAATCGAACCG
TACCGCAAACCGACACAGGTGGGCAGGATGAGAATTCTAAGGCGCTTGAGAGAACTCAGG
AGAAGGAACTCGGCAAATTGATACCGTAACTTCGGGAGAAGGTATGCCCTCTAAGGTTAA
GGACTTGCTCCGTAAGCCCCGGAGGGTCGCAGAGAATAGGTGGCTGCGACTGTTTATTAA
AAACACAGCACTCTGCTAACACGAAAGTGGACGTATAGGGTGTGACGCCTGCCCGGTGCT
GGAAGGTTAATTGAAGATGTGAGAGCATCGGATCGAAGCCCCAGTAAACGGCGGCCGTAA
CTATAACGGTCCTAAGGTAGCGAAATTCCTTGTCGGGTAAGTTCCGACCCGCACGAATGG
CGTAACGATGGCCACACTGTCTCCTCCTGAGACTCAGCGAAGTTGAAGTGGTTGTGAAGA
TGCAATCTACCCGCTGCTAGACGGAAAGACCCCGTGAACCTTTACTGTAGCTTTGCATTG
GACTTTGAAGTCACTTGTGTAGGATAGGTGGGAGGCTTAGAAGCAGAGACGCCAGTCTCT
GTGGAGCCGTCCTTGAAATACCACCCTGGTGTCTTTGAGGTTCTAACCCAGACCCGTCAT
CCGGGTCGGGGACCGTGCATGGTAGGCAGTTTGACTGGGGCGGTCTCCTCCCAAAGCGTA
ACGGAGGAGTTCGAAGGTTACCTAGGTCCGGTCGGAAATCGGACTGATAGTGCAATGGCA
AAAGGTAGCTTAACTGCGAGACCGACAAGTCGAGCAGGTGCGAAAGCAGGACATAGTGAT
CCGGTGGTTCTGTATGGAAGGGCCATCGCTCAACGGATAAAAGGTACTCCGGGGATAACA
GGCTGATTCCGCCCAAGAGTTCATATCGACGGCGGAGTTTGGCACCTCGATGTCGGCTCA
TCACATCCTGGGGCTGTAGTCGGTCCCAAGGGTATGGCTGTTCGCCATTTAAAGTGGTAC
GTGAGCTGGGTTTAAAACGTCGTGAGACAGTTTGGTCCCTATCTGCAGTGGGCGTTGGAA
GTTTGACGGGGGCTGCTCCTAGTACGAGAGGACCGGAGTGGACGAACCTCTGGTGTACCG
GTTGTAACGCCAGTTGCATAGCCGGGTAGCTAAGTTCGGAAGAGATAAGCGCTGAAAGCA
TCTAAGCGCGAAACTCGCCTGAAGATGAGACTTCCCTTGCGGTTTAACCGCACTAAAGAG
TCGTTCGAGACCAGGACGTTGATAGGTGGGGTGTGGAAGCGCGGTAACGCGTGAAGCTAA
CCCATACTAATTGCTCGTGAGGCTTGACTCTATCATTTGAAGAACTTCAAGAGATAAAAG
~~CTTACTGACTGATTCAGTCATTACCGAATATATTGATTAAGGCTTTACCGATTTGTAACA~~
GTTTAAGTTTGGCGGCCATAGCGAGTTGGTCCCACGCCTTCCCATCCCGAACAGGACCGT
GAAACGACTCAGCGCCGATGATAGTGTGGTTCTTCCATGCGAAAGTAGGTCACTGCCAAA
CACCCATTCAGAAAACCCCCGATTATTCGGGGGTTTTTGCTTTGCCCGGAAAAAATGTTT
GCTTTGCCCGGAAAAAATGTCGGTGATGGCGGGACGGCATCCGTACGGTGTCCGGTCGGG
TTTGCGGAGGAACGGCTTGAAACTTTGGGATATTCATTTTAGAATGACTCGTTTTATCGT
CGCAAGATGCGGTTTATTGTTTGCAACCCTTAAAGGAAAAACCATGAAGAAAATGTTCGT
GCTGTTCTGTATGCTGTTCTCCTGCGCCTTCTCCCTTGCGGCGGTAAACATCAATGCGGC
TTCGCAGCAGGAGTTGGAGGCGCTGCCGGGCATAGGCCCGGCGAAGGCGAAGGCCATTGC
GGAATACCGTGCGCAAAACGGTGCGTTCAAGTCTGTAGACGATTTGACCAAGGTAAAGGG
CATCGGCCCTGCGGTGCTGGCGAAGCTGAAGGACCAGGCTTCGGTCGGCGCGCCCGCACC
AAAAGCCCCAGCCAAACCGGTGCTGCCCGCGGATAAAAAATAGGGGAACCTGTAAAGGAA
AGGGCATCGGCCGCCGTCGGTGCTTTTTTGTTTGGAAGGGAAATGGCTAAAATATGTAGC
ATTATGTTCTGTATCGTTGTTTACCGCTTCCGCACCTTTGTCCGCCTTAAAGCAGGTAGA
CACCGCAATGAATCGACGCAAAGAAAATGCCGTCTGAACATGCGTTCGGGCGGCGTTTTG
TTGGGGGGTATCGGAGCGGAACGTCTGAAAAAGGGTTTCAGGCGGTCTTTGGGCGTGTGG
TGACAGTCGAAAACGTGATAAGGCTACCTGAAAAGTTTGGGAGATTTTCAGGTAGCCTTT
GGTATTGGGCGCAACAGACGCAGGTACAGATTAGCGGTGTGCCGTAATCGTACGAATGCC
GATTCAACCTAAGCAGACATCAGTATTTAGGAAGTGGATGTTTGATGGAGCAAAGGTTGT
ACGAAGGGTGGAAGGCAACCTGTGGGTGTTTGGTATGGTCGCGCTTGAAAAAACGTGTTT
TAAGGGACAAATGCCGTCTGAAAATCGGTTTCAGACGGCATTTTCTGTTTATTTAAAGCA
AACAGGAAAAGGCAGCAATATTCTGCAGTCTTCCTATTCACACAAGCGTTTTATAGTTAA
TTAAAAACAAAATAGTACAATACTCAACTTTGAAGGTCTAACCATGGCATACTCTGCGGA
CTTAAGAAACAAAGCTTTAAACTAGGGGCTGTACTAGATTAGCAGATATGTTACCCTCGA
AATATGAAGATAACGCACTGCAAATTAAAGAAAAAAGTACAGAAAGAACTGCTCCGTTTT
TTGTGCTGGAAGTTACCGCCCGTTCTGCCGCCGATATTTGGGTATCCATCCCAATTCGG

## Appendix A

CAGTACTGTTCTACCGTAAAATCCGCACGGTTATCAACCATCATTTGGCCTTGGCTGCCG
ATGAGGTTTTTGAGGGCCCTGTCGAGCCGGACGAAAGCGATTTCGGCGGACGGCGTAAAG
GCAGACGTGGTCGCGGTGCGGCAGGAAAAGTGGTTGTCTTCGGCATTCTGAAACGCAACG
GACGGGGCTATACCGTTGTCGTAGATAATGCCAAGTCTGAAACGTTACTCCCTGTCATCA
AAAAGAAAATCATGCCGGACAGTATTGTTTATACCGATAGTCTGAGCAGCTGCGACAAGT
TGGACGTGAGCGGTTTTATCCATTACCGCATCAACCATTCCAAGGAATTTGCAGACCGTC
AGAACCACATTAACGGCATTGAGAATTTTTGGAATCAGGCAAAACGCGTCTTGCGAAAAT
ACAACGGAATCGATCGTAAATCTTTCCCGCTGTTCTTGAAAGAATGCGAATTTCGATTTA
ACTTCGGCACACCGTCTCAACAGCTTAAAATCCTGCGGGATTGGTGTGGAATTTAGGGCT
AATCTAGTACAGCACCTAACAAAAACCAGTACGGCGTTGGCTCGCCTTAGCTCAAAGAGA
ACGATTCTCTAAGGTGCTGAAGCACCAAGTGAATCGGTTCCGTACTATTTGTACTGTCTG
CGGCTTCGTCGCCTTGTCCTGATTTTTGTTAATCCACTATATTTAGATAATGCGTGATT
TCACCGTATGGGTGTCTTACGGGAAATGGCGGAAAAATTGGGACATAAGGTATTGCCTCT
TGCACCTTATTCACCTGAGCTCAACCCGATTGAGAAAGTGTGGGCGAATATTAAGCGGTA
TCTGCGAACCGTTTTGTCTGATTACGCCCGATTTGACGATGCACTACTGTCCTATTTTGA
TTTTAATTGACTATAGAACGTTGCGGCTACGCGGAAGCCGTACTCGTTGGATTTGGAGCG
GCCCATTTTGGTTTTGTCACCGTCCAAGACAATCTCACGGGGTTTGTAGATTGTTTTGTG
ACGGTAGTATGGATCAAACTCGAGACCGACGCTGTCGGTCAACTGTTTGCCTACATTCAG
ACCGATACCGACACTCCAACCTTTGGCGCTTTTGCTGACATCGCGGGAAGCACCCATCTG
GGTCGTCATCACTTTGGTTTTGCCGCGCAAATCTGCATATGCATCCGCCCAAGGGGTCAG
GGATCATCCGTCCCCCAAATCTTGGCGGATTTCGCCATGGACTTTCAAAGCAAGGTTTTC
ATGCTTGGTAACGGTGTTTTTCCTTATCGCCGATGATGGCTTTGCCTTTGCCGTTAGACT
CGGGAATATCGGCTACCGTAACGGCGGACACGGCTGCAAGTGAGAGTGCAAGCAGGGTTT
TTTCATGTTTTTCTTCCTATAATGAGGATAAATAAATGGAAAAAGTGTGGGAAATACCCG
CATTCCCATTAAATCTTTTTTCAAGCAATGAGTTCTTTTTGTTTTCAACATTTTCCTTGA
GACCTTTGCAAAAATAGTCTGTTAACGAAATTTGACGCATAAAAATGCGCCAAAAAATTT
TCAATTGCCTAAAACCTTCCTAATATTGAGCAAAAAGTAGGAAAAATCAGAAAAGTTTTG
CATTTTGAAAATGAGATTGAGCATAAAATTTTAGTAACCTATGTTATTGCAAAGGTCTCT
CCTTGTGTATGAAATTTTGCCGGATGTGAAGGCGGAATCGGCAGCGGGGGTGTTCTGTAC
CGGATTGTCGTGGAAATGGGAAAACGGATGTTCCGTGCAGGTTTGTCCAAATGAATGGCG
GGTATTGTTTTTATCAATCTGTTTCTTTTTATTTGAAATAAAATTTCTAAAATAATAAAA
ATATGAAATTTAAAATCTATAAAAAAAGATATATCAGTTATTTTGAAATAAAATAGCTTT
GTAGTAATATGTTGCACTTGTTTGTGCAAGGTAAACGATGTAACCTAAGCCGCGTATAAA
AACCCATCAGGAAAGATGCAAGATGACACACCATTACCCCACAGACGATATTAAGATTAA
AGAAGTTAAAGAGTTGTTGCCGCCGATAGCCCATCTTTACGAGCTGCCGATTTCCAAAGA
GGCTTCGGGCTTGGTTCACCGCACCCGTCAGGAAATTTCCGATTTGGTTCACGGCAGGGA
CAAGCGGCTGTTGGTTATTATCGGGCCGTGTTCGATTCACGATCCGAAAGCGGCGTTGGA
ATATGCGGAGCGTTTGTTGAAACTCCGCAAGCAGTATGAAAACGAGCTTTTGATTGTGAT
GCGCGTTTATTTCGAGAAGCCGAGGACGACGGTGGGTTGGAAAGGTTTGATTAACGACCC
GCATTTGGACGGTACGTTTGACATCAATTTCGGTTTGCGTCAGGCGCGCAGCCTGTTGTT
GTCGCTGAACAATATGGGTATGCCTGCCTCTACCGAGTTTTTGGATATGATTACGCCGCA
ATATTATGCGGACTTGATTTCTTGGGGGGCAATCGGTGCGCGGACGACCGAAAGCCAAGT
TCACCGCGAATTGGCAAGCGGGCTGTCCTGCCCCGTCGGCTTTAAAAACGGTACGGACGG
CAATTTGAAGATTGCCATCGACGCAATCGGTGCGGCGAGCCATTCGCATCATTTCCTGTC
TGTAACCAAGGCCGGGCATTCCGCCATTGTCCATACCGGCGGCAATCCCGACTGTCATGT
CATTTTGCGCGGCGGCAAAGAGCCGAATTATGATGCGGAACACGTCAGCGAGGCGGCGGA
ACAACTGCGTGCGGCAGGGGTAACCGACAAGCTGATGATAGATTGCAGCCACGCCAACAG
CCGCAAGGATTACACTCGGCAGATGGAAGTGGCACAAGACATTGCCGCCCAATTGGAACA
~~GGACGGCGGCAATATCATGGGCGTGATGGTGGAAAGCCATTTGGTGGAAGGCAGACAGGA~~
CAAGCCGGAAGTGTACGGCAAGAGCATTACCGATGCGTGTATCGGTTGGGGCGCGACTGA
AGAACTGTTGGCATTGTTGGCAGGTGCAAACAAAAAACGTATGGCGCGCGCCAGTTGAGA
TTTTTGACGCAGAATGTCATAAAATGTCGTCTGAAGCGTTCAGACGGCATTTTTGTGGAG
GAAATATGCTCAAAATAACCCTAATTGCGGCGTGTGCGGAAAACCTGTGCATCGGGGCGG
GCAATGCTATGCCTTGGCACATCCCCGAAGATTTCGCATTTTTCAAAGCCTATACCTTGG
GCAAACCCGTCATTATGGGGCGGAAAACGTGGGAATCCCTGCCCGTCAAACCCCTGCCCG
GACGGAGGAACATCGTCATCAGCCGGCAGGCGGATTATTGCGCGGCAGGCGCGGAAACGG
CGGCAAGTTTGGAGGCGGCATTGGCATTGTGCGCAGGCGCGGAAGAAGCCGTCATTATGG
GCGGCGCGCAGATATACGGACAAGCGATGCCATTGGCGACCGATTTGCGGATAACCGAAG
TGGATTTGTCTGTGGAAGGAGATGCATTTTTCCCCGCAATAGACCGGACGCATTGGAAAG
AAGCAGAGCGGACGGAACGCCGTGTCAGCAGCAAAGGCACGCGCTATGCTTTTGTGCATT
ATTTGAGATATTGAAATATAAACTCTCTATAAAATCCCCCGCAAATGATGGGCTGAAATA
GAAAATATTGTTATTCCCCCGAAGATGGGAATCCGGGATTTTAAAGTTAGGGTAATTTAT
CCGAAATAACAACAATCTTCCATCGTCATTCCCGCAAAAGCGGGAATCCGGAAACGAAAA
GCTAAAGCAATTTATCGGAAAAAACCGAAGTTTAAAGAACCGGATTCCCGCCTGCGCGGG
AATGACGAGATTTTAGGTTATGGGGATTTATTGGGAATAATGGAACAAAGAAAGCAGAAA
TAAGGATATAGAGGCTGTCTTTGGATTTGCGATGGTTGTCGGAGAATGCCGTCTGAAGCC
GTTTCAGACGGCATTTTTCCAGCTTGAGAACGGATGCCTGCTCAAATAAGCATTGGTAAA
CATACCGTCGGCAGTGATTTCCCGTCCCAGCCAGTCCGGACGGTCAAAATCGGCATTCTC
GTCGGGCAACTCGATTTCCGCGACGACCAAAGGCGCATTATCGCCAAGAAAAACATCGAT
TTCAAACAGGCTGCCGCCCCATCTGACCGGATAACGCCATTTTTCCATTTTAAACGGGCA
CATCGTTTCCATCATCTTTTCCGCATCGGCAAGCGGGATTTCGTATTCAAACTCACTGCG
GCTGATTTCCGAAATATAGCCTTTCAGCGTCAGCCACGCCTGTTTTCCGGCAATGCGGAC
ACGGACGGTGCGTTCTTTTTCAACAGACAGATAACCCTGCCTCAACAGCAGCGGTTCGTC
GGCGTATTGCCGCCAGTTGTCGTTTCCAATCAAAAAACGGCGTTCGATTTCTATCGGCAT
AAGATGCTCCGTCAAAACGGTTTGAACACGACCAGATACAGCGCGGCAACCATCAGCAGC

## Appendix A

ACGGGGATTTCGTTGAACACGCGGTACCAGCGGTGTGAAAAAGCATTGCTGTAATCCTGA
AAACGGCGCAGCAGCACGCCGCAATACAACTGGTAAGCCAAGAGCATCAAGCCCAAACAC
AGTTTGACGTGTACCCAGCCGCTGCCCCACCAGCCGGCGGCAAACGGTATCGCCGCGCCG
AACACGACCGCGCCGAAGCCCAACGGCGACATAAAACGGTACAGCCGCACCGCCATGCCC
GACAGACGCACATACTCGGGATTGCCGCGCGGCACATCAATCATCGCCATATTGACGAAA
ATCCTCGGCAGGTAAAACAGCCCTGCAAACCACGAAATGACAAAAAACAAGTGAAACAGC
TTGAACCAAGAAAACATCATCGCCCACACCCTGCCGAAAAGCGGTATTGTACAGGCAAAC
CGCTTGGGAAACGTGATAAAATCAGGCGGATAAACAAATCGAATAAATCCTTACCGCAAA
ACGGAGGCAAAATGCTCAAATCCATCGAACTCAATTCCCACATCCGCAACCGCCTTGCAG
AATATCTGAAAGGCAGGGGTATGGATTTTCAGACGGCAATGCAGGAAGAAAAAGGCAACA
AAGAAATCGCCGCCATCGTCCACAGCGGTTTGCCCACTCTGGTCCGCAAACTGTATTCCG
AACAAAAAATGCAGAAGTTTTTTTGGGAAAAGCGGGATTTGATTGCCGACTACATCAGCC
GCCGGATGCAGGGATAGGTGGCTGAAATCTGTTTTCAGGCAAGTGAAAAGACAATATGGC
AGATTGAAATTACGCTTATCGTCATTCCCGCCCGCGCGGGAATCCGACTTGTTTGGTTTC
GGTTATTTTTCGTTTCGTAACTTTTGAGCCGTCATTCCCGCGCAGGCGGTAATCCGGCTT
GTTCGGTTTCGGTTCTTTTTCTCGTTTCGGGTGATTTCTAAACCGTCATTCCCGCGCAGG
CGGGAATCTAGGTCTTTAAACTTCGGTTTTTTCCGATAAATTTTTGCCGCATTAAAATTC
TAGATTCCCGCTTTCGCGGGAATGACGGCGGAGGGTTTTTAGTTTTCCCGAAAATGCACA
TCATCCAAAATCCCGTTATTCCCACAAAACAGAAAATCAAAAACAGCAACCTGAAATCCC
GTCTTTCCCGCGCAGGCGGTAATCTGAACACGTCCGTAGTGAAACCTATATCCCGTCATT
CGCACGAAAGTGGGAATCCAGGATGCAGGGAAAACCGTTTTATCCGATAAGTTTCCGCAC
CGAAAGGTCTAGATTCCCGCTTTCGCGGGAATGACGGCGGAGGGTTTTTAGTTTTCTCGA
TAAATGCACATCATCCAAAGTCCCGTTATTCCCACAAAAACAGAAAATCAAAAACAACAA
TCTGAAATTCCGTCCTTCCCGCCTGTGCGGGAATCCGGCTTGTTCGGTTTCGGTTCTTTT
TCTCGTTTCGGGTGATTTCTAAACCGTCATTCCCGCGCAGGCGGGAATCTAGGTCTTTAA
GCTTCGGTTTTTCTTGATAAATTCTTGCCGCATTAAAATTCTAGATTCCCGCTTTCGCGG
GAATGACGGCGGAGGGTTTTTTGTTTTCCCGATAAATGCACATCATCCAAAGTCCCGTTA
TTCCCACAAAAACAGAAAATCAAAAACAGCAACCTGAAATCCCGTCCTTCCCGCGCAGGC
GGTAATCTGAACACGTCCGTAGTGAAACCTATATCCCGTCATTCGCACGAAAGTGGGAAT
CCAGGATGCAGGGAAAACCGTTTTATCCGATAAGTTTCCGCACCGAAAGGTCTAGATTCC
CGCTTTCGCGGGAATGACGGCGGAGGGTTTTTAGTTTTCTCGATAAATGCACATCATCCA
AAATCCCGTTATTTCCACAAAACAGAAAATCAAAAACAGTAACCTGAAATCCCGTCATTC
CCGCGCAGGCGGGAATCCGGCTTGTTCGGTTTCGGTTCTTTTTCTTGTTTCGGGTGATTT
CTAAACCGTCATTCCCGCGCAGGCGGGAATCCAGACCTTTAAACCCCGACCATCCTTGAT
AAATTCTTGCGGCATTAAAATTCTAGATTCCCGCTTTCGCGGGAATGACGGCGGAGGGTT
TTTTGCTTTTCCTGATTTTTCATTGCGATGTAGTATAATGTAGTATATAATCATTATAAT
TTTAACACTTGACAAAGGAAAATTTCTCATGACACTGAAAGCAAGCAAGCAAGCAAGCAA
GCAAGCAAGCAAGCGGTCGGGTTAATCTATTAACATTATCTGTTTTATCGCTGTTTTGCA
CGCCATATGTTTGAGGTTCGGATGCGTACGATCCCGTCAAAGAAGCCGAGATTAAAAACA
AATTTATTTTAGAAGCGGCGGAAGACAGAAATTCCCACGTTTGGCGCGGCCCGTGCAGCA
TATCTTTTGATTGCTTCGGTATGTTCAGAGCTCAGCTTGGTTCAAATACTCGTTCTACCA
AAATCGGCGACGATGCCGATTTTTCATTTTCAGACAAGCCGAAACCCGGCACTTCCCATT
ATTTTTCCAGCGGTAAAACCGATCAAAATTCATCCGAATATGGGTATGACGAAATCAATA
TCCAAGGTAAAAATTACAATAGCGGCATCCTCGCCGTCGATAATATGCCCGTTGTCAAAA
AATATATTACAGAGAAGTATGGGGCTGATTTAAAGCAGGCGGTTAAAAGTCAATTACAGG
ATTTATACAAAACAAGACCGGAAGCTTGGGCAGAAAATAAAAAACGGACTGAGGAGGCGT
ATATAGCACAGTTTGGAACAAAATTTAGTACGCTCAAACAGACGATGCCCGATTTAATTA
ATAAATTGGTAGAAGATTCCGTACTCACTCCTCATAGTAATACATCACAGACTAGTCTCA
ACAACATCTTCAATAAAAAATTACACGTCAAAATCGAAAACAAATCCCACGTCGCCGGAC
AGGTGTTGGAACTGACCAAGATGACGCTGAAAGATTCCCTTTGGGAACCGCGCCGCCATT
CCGACATCCATACGCTGGAAACTTCCGATAATGCCCGCATCCGCCTGAACACGAAAGATG
AAAAACTGACCGTCCATAAGGATTATGCGGGCGGCGCGGATTTCCTGTTCGGCTACGACG
TGCGGGAGTCGGACGAACCCGCCCTGACCTTTGAAGACAAAGTCAGCGGACAATCCGGCG
TGGTTTTGGAACGCCGGCCGGAAAATCTGAAAACGCTCGACGGGCGCAAACTGATTGCGG
CAAAAACGGCGGATTCCGGTTCGTTTGCGTTTAAACAAAATTACCGGCAGGGACTGTACG
AATTATTGCTCAAGCAATGCGAAGGCGGATTTTGCTTGGGCGTGCAGCGTTTGGCTATCC
CCGAGGCGGAAGCGGTTTTATATGCCCAACAGGCTTATGCGGCAAATACTTTGTTTGGGC
TGCGTGCCGCCGACAGGGGCGACGACGTGTATGCCGCCGATCCGTCCCGTCAAAAATTGT
GGCTGCGCTTCATCGGCGGCCGGTCGCATCAAAATATACGGGGCGGCGCGGCTGCGGACG
GGTGGCGCAAAGGCGTGCAAATCGGCGGCGAGGTGTTTGTACGGCAAAATGAAGGCAGCC
GACTGGCAATCGGCGTGATGGGCGGCAGGGCCGGCCAGCACGCATCAGTCAACGGCAAAG
GCGGTGCGGCAGGCAGTGATTTGTATGGTTATGGCGGGGGTGTTTATGCTGCGTGGCATC
AGTTGCGCGATAAACAAACGGGTGCGTATTTGGACGGCTGGTTGCAATACCAACGTTTCA
AACACCGCATCAATGATGAAAACCGTGCGGAACGCTACAAAACCAAAGGTTGGACGGCTT
CTGTCGAAGGCGGCTACAACGCGCTTGTGGCGGAAGGCATTGTCGGAAAAGGCAATAATG
TGCGGTTTTACCTACAACCGCAGGCGCAGTTTACCTACTTGGGCGTAAACGGCGGCTTTA
CCGACAGCGAGGGGACGGCGGTCGGACTGCTCGGCAGCGGTCAGTGGCAAAGCCGCGCCG
GCATTCGGGCAAAAACCCGTTTTGCTTTGCGTAACGGTGTCAATCTTCAGCCTTTTGCCG
CTTTTAATGTTTTGCACAGGTCAAAATCTTTCGGCGTGGAAATGGACGGCGAAAAACAGA
CGCTGGCAGGCAGGACGGCACTCGAAGGGCGGTTCGGTATTGAAGCCGGTTGGAAAGGCC
ATATGTCCGCACGCATCGGATATGGCAAAAGGACGGACGGCGACAAAGAAGCCGCATTGT
CGCTCAAATGGCTGTTTTGATGCGTCGGGAAATGTTTTGACGCACAGGCGGTACACCGGC
ACGGCACCGCGCGCCGCCCCGCAAACCAATCCGAACCCTGCCGCCCCGAAGGGCGGGGCA
TAATGATGAAACCGGCGGAAAACCGCCGGTTTTTTGCCGCCGTTTGAAACCCGATTCTGG
CTTCAGACGGCATTGTCGCGGCATCGGGCGGCAGGGTTTGGAACAGCGGCATAAAAAACT

## Appendix A

GATACAATCCGCCGATTGATAATGGTTATTTTTATTTTTGTGGAAGACATTTATGCCT
GCACGAAACAGATGGATGCTGCTGCTGCCTTTATTGGCAAGCGCGGCATATGCCGAAGAA
ACACCGCGCGAACCGGATTTGAGAAGCCGTCCCGAGTTCAGGCTTCATGAAGCGGAGGTC
AAACCGATCGACAGGGAGAAGGTGCCGGGGCAGGTGCGGGAAAAAGGAAAAGTTTTGCAG
ATTGACGGCGAAACCCTGCTGAAAAATCCCGAATTGTTGTCCCGCGCGATGTATTCCGCA
GTGGTCTCAAACAATATTGCCGGTATCCGCGTTATTTTGCCGATTTACCTACAACAGGCG
CAGCAGGATAAGATGTTGGCACTTTATGCACAAGGGATTTTGGCGCAGGCAGACGGTAGG
GTGAAGGAGGCGATTTCCCATTACCGGGAATTGATTGCCGCCCAACCCGACGCGCCCGCC
GTCCGTATGCGTTTGGCGGCAGCATTGTTTGAAAACAGGCAGAACGAGGCGGCGGCAGAC
CAGTTCGACCGCCTGAAGGCGGAAAACCTGCCGCCGCAGCTGATGGAGCAGGTCGAGCTG
TACCGCAAGGCATTGCGCGAACGCGATGCGTGGAAGGTAAATGGCGGCTTCAGCGTCACC
CGCGAACACAATATCAACCAAGCCCCGAAACGGCAGCAGTACGGCAAATGGACTTTCCCG
AAACAGGTGGACGGCACGGCGGTCAATTACCGGCTCGGCGCGGAGAAAAAATGGTCGCTG
AAAAACGGCTGGTACACGACGGCGGGCGGCGACGTGTCCGGCAGGGTTTATCCGGGGAAT
AAGAAATTCAACGATATGACGGCAGGCGTTTCCGGCGGCATCGGTTTTGCCGACCGGCGC
AAAGATGCCGGGCTGGCAGTGTTCCACGAACGCCGCACCTACGGCAACGACGCTTATTCT
TACACCAACGGCGCACGCCTTTATTTCAACCGTTGGCAAACCCCGAAATGGCAAACGTTG
TCTTCGGCGGAGTGGGGGCGTTTGAAGAATACGCGCCGGGCGCGTTCCGACAATACCCAT
TTGCAAATTTCCAATTCGCTGGTGTTTTACCGGAATGCGCGCCAATATTGGATGGGCGGT
TTGGATTTTTACCGCGAGCGCAACCCCGCCGACCGGGGCGACAATTTCAACCGTTACGGC
CTGCGCTTTGCCTGGGGGCAGGAATGGGGCGGCAGCGGCCTGTCTTCGCTGTTGCGCCTC
GGCGCGGCGAAACGGCATTATGAAAAACCCGGCTTTTTCAGCGGTTTTAAAGGGGAAAGG
CGCAGGGATAAAGAATTGAACACATCCTTGAGCCTTTGGCACCGGGCATTGCATTTCAAA
GGCATCACGCCGCGCCTGACGTTGTCGCACCGCGAAACGCGGAGTAACGATGTGTTCAAC
GAATACGAGAAAAATCGGGCGTTTGTCGAGTTTAATAAAACGTTCTGATTGCTGTTCCTT
TTCGGAGGAAACCCTGCCGGCGGCGGTATCACGGCGGGCATCGGCGGCTTTCGGGCGGTG
CTTTGCGTGCCGCCGCGTGTGCGGAAACGCATTCCGGTTTTTCCGGCATAACGGCGATGC
GAGGTAAAATGCCGTCTGAAACCCGATTCGGGCTTCAGACGGCATTGTCGCGGTTGCGGC
GGGCGGGTTCACCAGATTCCGTCAAAGGTTTTCGCGCCGCGCCAAAATTTCCACCTGTCG
GCGGGTTTGAAGGTCAGCGTACCGCCGTGTTGTCCGTCCGTGGTGATGTCCAGCCGTTTG
ATTTTGCCGGTGCGGACGGCTTCGTAGATTGGTGCGAACCAGCGTTCTTCCCACTGCTGC
AATATTGCCGCATACCGCTCCCTGTCCCCTGTCAGGGCGGTCAGGCGCAAATCGTCCATA
AACAGGATATGGTGCGTGTCGGGCAGGTGTGCCGCCGTTTCTTCATAGGCGCGGAAGTTG
TCGGGTAATGCGCGGCGGTCGGAGTGGAAACGGCTCCAAACCGTATCGGCGAAAAGCGTG
CCGCCTTGCGCGCCGCCGTTTGTGCCGTCCCAAAGCCATAAGCCGTTCAACTCGGGCAGC
CCGCGTTTCTTGCGGTTATGGTTGACGGGGTGCGCCGCCAGCCACATTTGGATTTCGGTT
TGGACGCGCAGCCATTCCAACGCATCTTCTCCGTCCGGCTGATCGTCAGCGCCCAACAAT
CCGCCCAAGTCCAAAACGGGCTTCGCGCCCCAGCGGTACGCGCAAGGAAGGGAAACCAGC
CATAATTCGGGCAGGACGGGAACGAAACGCCATGGAATGTCGCCGTAAAACGCCGACAGG
TCGCGGCAGATCCGTTCCGCTTCATCCGTACCGACGTTCAGATATTCCGCCGTTAGCACA
TTTGCCTGATGCATCCCCATCTTTTGCCAGACGGGCGTGGCGAGCGCGACGGCTTCAGAC
GGCATATTCAGGCTTTGCGCCGCGCGTTCCACCAGTCTGCCGCACCACAAATAACGCGCG
TAAAATGCCGAAGCCGTGCAGCTTTGGCGGTGCAGCGAGCCGTATTGCAGGATTTTGTTG
AAAGCGTGCAGGCATAGAGGTATTCGGATTTCGTCTTCATCCAAATTGAGCGAGGGAATG
GCGAGGGTGAGTTTCATCGTTTGACGTTTCAGAAATGCAGGTCAGGCGCAACATTATAGA
GGATTCGGCGCAAACGCCGTCAAAAAGGAACAATATGGCTGTCTTCCCACTTTCGGCAAA
ACATCGGAAATACGCGCTGCGTGCGCTTGCCGTTTCGATTATTTTGGTGTCGGCGGCATA
CATTGCTTCGACAGAGAGGACGGAGCGCGTCAGACCGCAGCGCGTGGAACAAAATCTGCC
GCCGCTGTCTTGGGGCGGCAGCGGCGTTCAGACGGCATATTGGGTGCAGGAGGCGGTGCA
GCCGGGCGACTCGCTGGCGGACGTGCTGGCGCGTTCGGGTATGGCGCGGGACGAGATTGC
CCGAATCACGGAAAAATATGGCGGCGAAGCCGATTTGCGGCATTTGCGTGCCGACCAGTC
GGTTCATGTTTTGGTCGGCGGCGACGGCGGCGCGCGAAGTGCAGTTTTTACCGACGA
AGACGGCGAGCGCAATCTGGTCGCTTTGGAAAAGAAAGGCGGCATATGGCGGCGGTCGGC
TTCTGAGGCGGATATGAAGGTTTTGCCGACGCTGCGTTCGGTCGTGGTCAAAACGTCGGC
GCGCGGTTCGCTGGCGCGGGCGGAAGTGCCCGTCGAAATCCGCGAATCCTTAAGCGGGAT
TTTCGCCGGCCGCTTCAGCCTTGACGGTTTGAAGGAAGGCGATGCCGTGCGCCTGATGTA
CGACAGCCTGTATTTCCACGGGCAGCAGGTGGCGGCGGGCGATATTTTGGCGGCTGAAGT
CGTTAAGGGCGGCACAAGGCATCAGGCGTTCTATTACCGTTCGGACAAGGAAGGCGGAGG
GGGCGGCAATTATTATGATGAAGACGGCAAGGTGTTGCAGGAAAAAGGCGGCTTCAACAT
CGAGCCGCTGGTCTATACGCGCATTTCTTCGCCGTTCGGCTACCGTATGCACCCCATCCT
GCACACATGGCGGCTGCACACGGGCATCGATTATGCCGCACCGCAGGGAACGCCGGTCAG
GGCTTCCGCCGACGGCGTGATTACCTTTAAAGGCCGGAAGGGCGGATACGGCAACGCGGT
GATGATACGCCACGCCAACGGTGTGGAAACGCTGTACGCGCACTTGAGCGCGTTTTCGCA
GGCGGAAGGCAATGTGCGCGGCGGCGAGGTCATCGGTTTTGTCGGTTCGACCGGGCGTTC
GACCGGGCCGCACCTGCATTACGAGGCGCGCATCAACGGGCAGCCCGTCAATCCTGTTTC
GGTCGCATTGCCGACACCGGAATTGACGCAGGCGGACAAGGCGGCGTTTGCCGCGCAGAA
ACAGAAGGCGGACGCGCTGCTTGCGCGCTTGCGCGGCATACCGGTTACCGTGTCGCAATC
GGATTGAAGTTTGAACCGGCGACGAAAACAATGCCGTCTGAAAACCTGCAAACAGGTTTT
CAGACGGCATTTATAGTGGATTAACAAAAATCAGTACGGCGTTGCCTCGCCTTAGCTCAA
AGAGAACGATTCTCTAAGGTGCTGAAGCACCAAGTGAATCGGTTCCGTACTATTTGTATT
GTCTGCGGCTTCGTCGTCTTGTCCTGATTTTTGTTAATCCACTATGCAGTTGATTAAAAC
AAAACTAAGCCAAGGAAGCACTGCCGTCATTCCCGTACGGGCGGGAATCCTGACACCACG
GCACGGAAACCCATCCGCTGTCATTCCCACGAAAGCGGGAATCTAGAAATACAACGCGGC
AGGAGTTTATCGGAAATGACTGAAACCCAACGTACCGGATTCCCGCTTTCGCGGGAATGA
CGAAGTGGGCGGGAATCCGGATTTATCCGTTCCGACAGTGTTTGCAAATAAAAGAAAACC

## Appendix A

CAACCGTCCCGATTCCCGGCAGGGCTGTTTTACGGATTTTGCAGCGAGGGCGCGGGGCGG
TCTTGCGCCTGTTTGGTTTGCAGGGTTGTCAGTTTTTTCGTCAGCAGATTCAGTATCACG
CCGTAGGCGGGCAGGAAGAAGAGGGTGCAGACGGTAAGTTTGAACAGGTAATCGACAAAA
GCGATGCCCTGCCAGTTTGCCGCCATAAATCCATCGCTGCTTGCGTAGAAGGCAACGGCG
AAAAATACCAGCGTATCCAAGGCGTTGCCGATGACGGTTGATGCGGTCGGTGCAATCCAC
CACGCTTTCAGACGGCGTAATTTGTTGAATACAAAAATATCAAGGATTTGTCCGATCGCG
TAGGCGGCAAAGCTGGCTAAGGCGATGCGTCCGACAAAGGTGTTGAATTCGGACAGCGCG
CCCAAGCCTGTCCAACTGCCGTTGTGGAACAAAACGGAAAAGACGTAGGAAAGCAAAAGG
GCGGGGAACATCACCCAAAAGATAATCCGCCGTGCCAAGTGAGAACCGAAAATGCGGACG
GTCAGGTCGGTGGCAAGGAAGATGAAGGGAAAGGAAAATGCGCCCCAAGTGGTGTGGATG
CCGAAAATTTGGAAAGGGAACTGCACCAGATAGTTGCTGGCGGCGATGATGAGGATATGA
AAAAGCACCAGCCGGAAGAGTGCCTTCTGTTGCTGTGCGGCGGTAAATGCGTACATAAAA
ATCTTTCGGAAAGGCGTTCAGACGGCATATCGTATCGAAGGAATGCCGTCTGAAATATGG
GAAGGATGGTTTATTGTGCGTCGTGCTCAAACAAGCGTTTGCGTGCCAATGTTTCGAACT
CGGTGCCTGCTTTTCCGTAGTTGGCAAACGGATGAATGGCGATGCCGCCGCGCGGTGTGA
ACTCGCCGAATACTTCGATGTATTTCGGATCCATCAGGGCAATGAGGTCTTTCATGATGA
TGTTGACGCAGTCTTCATGAAAATCGCCGTGGTTGCGGAAGCTGAAGAGGTAGAGTTTCA
GGGATTTGCTTTCCACCATTTTGATGTGCGGAATGTAGCGGATGTAGATGGTGGCGAAGT
CGGGCTGCCCGGTCATGGGGCAGAGGCTGGTGAACTCGGGACAGACGAATTTGACGAAAT
AGTCGTTGTCGGGATGTTTGTTGTCGAATGCTTCGAGAATTTCAGGCGCGTAGCCGGTCG
GATATTGGGTTTTTTGATTGCCCAAAAGAGAGATGCCTTGCAGCTCTTCGTTGTTGCGGG
ACATGAGGGTTTCCTTAGTTTTTTAATGTGGGAGGTTTTCGAACCACGGGCGGCGATTGT
AATATAAGCGGCGGTATCTGTGTAGTTTTCTTCAGACGGCATGGTTTGGACGGCGGCGTT
TTCCGTGTCATATATAGTGGATTAACAAAAACCAGTACGGCGTTGCCTCGCCTTAGCTCA
AAGAGAACGATTCTCTAAGGTGCTGAAGCACCAAGTGAATCGGTTCCGTACTATTTGTAC
TGTCTGCGGCTTCGCCGCCTTGTCCTGATTTTTGTTAATCCATTATATAAACGAAATATA
TTTTCAGTTTTGCCGCCTGAAGCGTTGTTTTTGAATATTGCATCTAAAATACTGACTTG
ATTGCGTTATTGCGCGGATATAGAATCTGCTTCCTATTGAAAGAACATTGTTTATATGAA
ATCAGGAAATTCGGAACCCAATCTTATGGATACGCACACGGACGAAACAAAACTTCAAAA
CACGCAAGCCAAACGCAAACGCCGCCTGACGGCATTGACGCTGCTGTTCGCGCTTGCCGC
CGCAGCCGCCGGGTCGGCGTTTTTTTATGGTGGCAGCACGAAGAGGAAACGGAAGACGC
TTATGTTGCCGGACGCGTGGTTCAGGTTACGCCGCAAAAGGGCGGTACGGTGCGGAAGGT
TTTGCACGACGATACGGATGCCGTGAAAAAAGGCGACGTGCTGGCGGTATTGGACGACGA
TAATGATGTGCTGGCTTACGAGCGGGCAAAAAACGAGCTGGTTCAGGCGGTGCGGCAAAA
CCGCCGGCAAAATGCCGCCACTTCGCAGGCGGGGGCGCAGGTTGCCTTGCGCCGGGCGGA
TTTGGCACGCGCACAGGATGATTTGCGCCGCCGGTCTGCTTTGGCGGAATCGGGCGCGGT
GTCCGCCGAAGAGCTGGCACACGCCCGTGCGGCAGTGTCTCAGGCGCAGGCGGCGGTCAA
AGCGGCTTTGGCGGAAGAATCTTCGGCACGTGCGGCTTTGGGCGGTCAGGTTTCTTTGCG
CGAACAGCCGGCGGTTCAGACGGCAATCGGCAGGTTGAAAGATGCGTGGTTGAACCTTCA
GCGGACGCAAATCCGCGCGCCGGCGGACGGTCAGGTGGCGAAGCGTTCGGTGCAGGTCGG
GCAGCAGGTGGCGGCAGGCGCGCCGCTGATGGCGGTGGTGCCGCTGTCGGATGTGTGGGT
GGATGCTAATTTTAAAGAGACGCAGTTGCGGCATATGAAAATCGGACAGCCTGCCGAGCT
GGTGTCCGATTTGTACGGCAAACAAATTGTTTATCGCGGCAGGGTGGCAGGTTTTTCGGC
AGGTACGGGCAGCGCGTTTTCGCTGATTCCGGCGCAAAACGCAACGGGCAACTGGATTAA
AGTGGTGCAGCGCGTCCCCGTCCGTATCGTGCTGAACCGCGAAGATGTGGACAGGCATCC
GTTGCGTATCGGTTTGTCGATGACGGTTAAAGTGGATACTTCCGCCGCAGGCGCGCCTGT
TTCAAAAACGCCGGGTGCGGCATTGCCGGAAATGGAAAGTACCGACTGGTCGGAAGTCGA
TCGGACGGTCGATGAAATCCTCGGGCAATCCGCGCCCTGATGCCGTCTGAAACGGAGGAC
ACAATGGATTATCCACCGCTTAAGGGTGCGGCATTGGCGTGGGTTACGCTGTCTTTGGGG
~~GTTGCCGTATTTATGGAAGTTTTAGATACGACTATCGGCAATGTCGGCGTTGCGGTCATC~~
GCCGGCAACCTCGGTGCGGCAACCACTCAGGGGACGTGGGTCATCACTTCCTTTTCTGTG
GCAAACGCCGTTTCCGTGCCGCTGACGGGCTTTTTGGCAAAACGCATCGGCGAGGTCAAA
TTGTTTACCGCCGCCGCTGTCGGTTTCGTCATCACATCGTGGCTGTGCGGTATTGCCCCC
AACCTTCAGTCGCTGGTTGTTTTCCGCATCTTGCAGGGCTTTATCGCCGGGCCGCTGATT
CCCTTGTCGCAAAGCCTGTTAATGGCATCCTATCCGCCCGCAAAACGGACGCTGGCACTG
GCATTGTGGGCAATGACCGTCGTTGTCGCCCCTGTTCTCGGGCCGATACTCGGCGGCTGG
ATTTCCGGAAACTGGCATTGGGGTTGGATTTTCTTCATTAATATCCCTATCGGTATCATA
TCGGCATGGATTACATGGAAACATTTGAAATATCGGGAAACGGAAACCGTTAAAATGCCG
ACCGACTATGTCGGGCTTACATTGATGGTAGTCGGTATCGGCGCGTTACAGATGATGCTG
GACAGGGGTAAGGAACTCGACTGGTTCGCCTCTGGAGAAATCATTACCTTGGGCGTAGTC
GCACTGGTGTGCTTGTCGTATTTTATTGTTTGGGAATTGGGAGAAAAATATCCGATTGTC
GATTTATCGCTGTTTAAAGATCGGAATTTTACCGTCGGCGTCATTGCCACGTCATTGGGT
TTTATGGTGTATATGGGGACGCTGACCCTGCTGCCGTTAGTGTTGCAGACCAACCTGGGC
TATACCTCCACGTGGGCAGGGCTTGCCGCCGCACCTGTCGGCATCCTGCCTGTTTTCCTG
TCTCCGTTAATCGGCAGGTTCGGCAATAAAATCGATATGCGCCTGTTCGTAACTGCCAGC
TTCCTGACCTTTGCCTTTACTTTCTATTGGCGTACGGATTTTTATGCCGATATGGATATT
GGCAACGTCATCTGGCCGCAGTTTTGGCAGGGTGTCGGTGTCGCCATGTTTTTTCTGCCG
CTGACCACCATCACACTGTCGCATATGAAGGGCGGGCAGATTGCCGCCGCAGGCAGCCTG
TCGAATTTCTTGCGCGTGCTGATGGGCGGTGTCGGCGTATCCGTCGTCAGCACCCTGTGG
GAACGGCGCGAAGCGTTGCACCACACACGCTTTGCCGAACACATCACGCCCTATTCCGCA
ACATTGCACGAAACGGCCGCTCATTTGTCCCAGCACGGCGTTTCCGACATTCAAACCCTA
GGCATCATCAACAATACCATTACCCAGCAGGGTTTTATTATCGGCTCGAACGAAATCTTT
ATGGCGGGCAGCTTGTTATTCATTATCATGATACCCGTCATATGGCTGGCAAAACCGCCG
TTCCACAACGGCGGCGGCGGTGGACATTGAGGGATTTGAAAACTTGAAATGCCGTCTGAA
AATACTGGAAATATGTTCGGACGGCATTTTGAATGCAGCAGTTCCCGAAATCCGCTATAA

## Appendix A

TCGCGCCCCATCTGTTTCGCACCTGCAAACGTTCCACAGATGCGACAATCGGAAGGATTA
TCCGCGCAAAACAGCCGTTTTTCTTTAAAACACTTGAACTAACACTGTTTTTCGTGGTAT
AAATCGCGTTTTACTATTTTAGAAGTTTGGAGACTGATTATGGCACGAGTTTGCAAAGTG
ACCGGCAAACGCCCGATGTCCGGCAACAACGTATCGCACGCCAACAACAAAACCAAACGC
CGTTTTTTGCCCAACTTGCAATCACGTCGTTTTTGGGTAGAAAGTGAAAACCGCTGGGTT
CGCCTGCGCGTTTCCAACGCTGCACTGCGTACCATCGACAAAGTAGGCATTGATGTCGTA
TTGGCTGATTTGCGTGCTCGCGGCGAAGCTTAATTTAAACACTATTTAATTAAGGATTAC
TGCAATGCGCGATAAAATCAAACTGGAATCCAGTGCAGGTACTGGTCACTTCTACACCAC
TACCAAAAACAAACGCACTATGCCCGGCAAATTGGAAATCAAAAAATTTGACCCAGTTGC
CCGCAAACACGTAGTGTATAAAGAAACTAAACTGAAATAATTTCAGTTTGAAAGCAAAGC
CTCCGACTGCTCGGAGGCTTTGTTATTTTTATCGTGTTTCCTTTCCGCTTGAAACATCTG
CCGTATGCGAATCTGCTGCAAACCGTCTGCCAAGGATATGAAAACCGCAAAACGGTTCAT
AACACAAAAATGCCGTCTGAAACGTTTCAGACGGCATTTCGGCAGTTTTCAACCGGTCAG
TTGTTTGGTGATCAGTTTCTTCAGCGGTGGGAAATTGTTGCTGGCACGCAATACCAAGCC
GCGCAACAGTTTTGCCGGTGCGGTCTCATTGGTAAACAGTTTCAGCATCATATTGGTTCC
GTGATAAAGCGGATGGGCGTGCAGCATATGTTTGCTGCTGTATTTTTCCAATAATGAAGA
TGCACCGATGTCTTGACCGCGCTGTTCGGCTTCGAGTATCAGTTTTGCCAAAATATCTGC
GCTGGAAAGCCCCAAGTTGAAACCGTGTGCTGTAACGGGGTGCATACCGACGGCGGCATC
GCCAATCAGCGCGCTGCGTTTGCCGTAGAAACGTTTGGCAATCATGCCGACAAGGGGGTA
ATGGTGGATGCTGCTGACCAATTCCATATCGCCGAGCCTGCCCTTGAGCTGTTCTTTTAC
GCTTGCCGCCAATTCTTCGGGCGAAAGGTTTTGAACGCTGTTGATTTTATCGGTATCGAC
GGTAATGACGGTATTGGTCAGGTGCTCTTCCAGCGGCAGCAGTGCGATGGTGCGTCCGTA
ATGGAAGCATTCGTAAGCGGTATGTTGGTTGGAAAGGGTATGTTTCATACGGCAGACGAA
CATGGTTCGGCTGTAATCGTGCATATCGGAGGAGATACCGAGTTGTCGACGGGTTTGCGA
GAAGCGGCTGTCTGCCGCCAAAAGCAGGCGTGCAGTCAGTATTTTGCCGTTTTCCAAAAT
GACTTGTGCTTCGTTGTCAGATGTTTTGACTTCTTTGACAACCGTATCGGTCAGAATGCT
GACATTGTCGAGTTGTGATACGACTTCATAGGCGGCGCGGCGGATATTGTGGTTGGAAAT
CAGATAGCCCAAACAGTCGGCAGGTTCGCCGCGCGCTTCAGTCGGTTGGGGAAAGTGGAG
CTGGTAGTCGGAACGTCCGTTCAGCACTTTGGCATCGCGCAAAGGGTAGATTTCGTTTTC
GGGAATTTTGTCCCACATACCCAAACGCTGCATGATTTCGCGGGAAAAATGGGTCAGGGC
GATTTCGCGTCCGTCATATGGAGGATTTTGCAGAACAGTCAGTGGGCTGCGTTCGATCAG
GGTAACTTTCAAACCGCTGCCGGCAAGTTCGGCTGCAAAACTTAAACCCGCCGGGCCTGC
GCCGACGACGAGGATGTCGCTGTGTAAACTCATAAAATATCCTTTGCATAGACGGATGCC
GATGATTTCAGACGGTATTTGTAAGGGTTTGAATGCCGTTTGAACTATCTGTAACAGATA
GGCGATTATATCAAAACCCACTGTTGAAGAAATATGCAGGGGAGGGTGTATGCGGATTTT
TACTTTCAGCTTAATGTGTATCAAATCGGGTGTGGGGTATGTATAGTGGATTAAATTTAA
ACCAGTACGGCGTTGCCTCGCCTTGCCGTACTATTTGTACTGTCTGCGGCTTCGTCGCCT
TGTCCTGATTTTTGTTAATCCACTATAAAAAGCCGCATCGTGAAAAGATGCGGCTTCAGG
TATCGGTTGGATTATTCTTCAGAACCGGTGTAAGGACGGATGCTGACAGTTTTACGGTTC
AGCGCGCCTTTGGTTTTGAATTCGACATAACCGTCAACTTTGGCGAACAAAGTGTGGTCT
TTGCCCATACCTACGTTGTCGCCTGCGTGGAATTTGGTACCGCGTTGGCGTACGATGATG
GAACCTGCGGGAATCAGCTCGTTGCCGTAGGCTTTAACGCCCAAGCGTTTGGCTTCTGAA
TCGCGACCGTTGCGGGTGCTGCCGCCTGCTTTTTTACTTGCCATTTGTAATGCTCCTAAG
TTTTAAGGTTAGGCGATTGCCACGATTTCGATTTGGGTGAAATTTTGGCGGTGGCCTTGG
CGTTTTTGGTAGTGTTTGCGGCGGCGCATTTTGAAGATGCGGACTTTTTCGCCACGACCG
TGTGCCACTACTTTAGCCGTTACTTTTGCACCTTCGATAAAGGGTGCGCCAACTTTTACA
GATTCGCCGTCAGCAATCATCAAAACTTCGGTCAGTTCGATTTGGCTGTCGAGTTCGGCT
GGTATCTGTTCTACTTTCAATTTTTCGCCGACGGAAACTTTATACTGTTTGCCGCCGGTT
TTTACGACCGCGTACATACTCAACTCCATAAGGGTTATGGTTAATATCCGCACACCATTG
~~TGCGGAACTCGGCATTGTATTGTTATTTGCCTGTTTTGTCAAAGTTTGCGCGGTTCGGAT~~
AACCATATGCCGTCTGAAAAGATGTACCCTGATGGCTTTGCTGATATAATTGCCCGCTAT
TTGAATCAGCTTTCAAGCGGTATCTGCCGTTTGACGGAAACGTAAACCTGAGAGTCTGCC
ATGCTCGAGAATCTGCCCTATTTCCAGCGACATCTGCCTGAAGACCTTGCCAAAGTCAAT
GAAGTCATCAACCGTGCGGTGCAATCCGATGTCGCACTGATTTCGCAAATCGGTACATAT
ATCATCAGCGCGGGCGGCAAACGCCTGCGTCCGATTATGACGATTTTGGCGGGTAAGGCG
GTCGGTTATGATGACGAGAAACTGTATTCGCTGGCGGCGATGGTCGAGTTTATCCACACT
TCCACCCTCCTGCACGACGATGTCGTCGATGAAAGCGATTTGCGCCGTGGGCGGGCAACG
GCAAACAATCTGTTCGGCAATGCGGCGGCTGTGTTGGTTGGCGACTTTTTATACACGCGC
GCCTTTCAACTGATGGTTGCCTCGGGCAGTATGCGCGTTTTGGAAGTGATGGCGGATGCA
ACCAACATTATTGCCGAGGGCGAAGTCATGCAGCTGATGAACATCGGCAATACGGACATT
ACCGAAGAACAATATATCCAAGTCATCCAATATAAAACGGCAAAATTGTTTGAAGCTGCC
GCTCAAGTCGGCGCAATTTTGGGCAAGGCTTCCCCCGAACACGAACGGGCGTTGAAAGAC
TACGGTATGTATGTCGGTACGGCATTCCAAATTATTGACGATGTGCTGGACTATTCTGGC
GAAACCGAAGAAACCGGCAAAAACGTCGGCGACGATTTGGCGGAAGGAAAACCGACTTTG
CCTTTGATTTATCTGATGCGTCAGGGTTCCGAACAGGTTGCGAACGATGTGCGTACTGCT
TTGGAAAATGCAGATCGCAGCTATTTTGAGAAAATCCACGATTATGTCGTCCGTTCGGAT
GCGTTGGCATATTCGATAGGCGAGGCGCGCAAAGCAGTCGATTGTGCCGTTACCGCCTTG
GATGCCCTGCCCGACAGCGAAGTGAAGGATGCCATGATTCAGCTGGCGAAGGAATCTTTG
GTCAGGGTGTCTTGAGGCGATGAATTTCAGTTTTGTTCCCCTGTTTCTGGTTACGCTGAT
TCTGTTGGGGGTGGTCAGCAACAACAATTCGATTACCATCTCGGCAACCATATTGCTGCT
GATGCAGCAGACGGCATTGATACAGTTTGTCCCGTTGGTCGAGAAGCACGGGTTGAATCT
CGGTATCATTCTTTTGACCATAGGGGTTTTGAGTCCGTTGGTTTCAGGAAAGGCGCAGGT
TCCTCCCGTTGCCGAATTTTTGAATTTTAAAATGATATCCGCCGTTTTTATCGGTATTTT
CGTGGCTTGGCTGGCGGGACGCGGCGTGCCTTATGATGGGACAGCAGCCTGTTTTAATTA
CAGGGCTGTTAATCGGGACGGTTATCGGGGTGGCATTTATGGGCGGTATCCCTGTCGGGC

# Appendix A

CGCTGATTGCGGCCGGCATCTTGTCTTTTGTCGTCGGAAAGGGTTAAAATCTCCTTTTCA
TTTCGGCTCGCCATAGTTCAACGGATAGAACGTATGCCTCCTAAGCGTAAAATACAGGTT
CGATTCCTGTTGGCGAGGTTTGACGATTTCATTTGTCTGTTTCCCGTGTTGCGGGAAGTT
TCCGATATAAGGCCTTTCAGTGTTGGAGGGCTTTTTGCCATCTGAAAACTTTTTCTTCC
TGCTTGAAAAACCGACCTTTAGGACGGTAGAATCATGAAATGATTTTCAGGCTTCGTAAA
AGATGTTCCGGCTTGGAAATCTGTTGTTTTATGATATAGTGGATTAAATTTAAATCAGGA
CAAGGCGACGAAGCCGCAGACAGTACAGATAGTACGGCAAGGCGAGGCAACGCCGTACTG
GTTTAAATTTAATCCACTATAAAAGCTGTACAGGTATAACAATGAATAAATTTGGGGATA
AGGTCGTATGAGCGTAGGTTTGCTGAGGATTCTGGTTCAAAACCAGGTGGTTACTGTTGA
GCAGGCCGAGCATTACTACAATGAGTCGCAGGCGGGTAAGGAAGTGTTGCCGATGCTGTT
TTCAGACGGTGTCATTTCGCCCAAGTCGCTTGCGGCATTGATTGCGAGGGTGTTCAGTTA
TTCGATTCTTGATTTGCGTCATTATCCGCGCCACAGGGTGCTGATGGGGGTGTTGACGGA
GGAGCAGATGGTGGAGTTCCACTGTGTGCCGGTTTTCCGTCGGGGCGACAAAGTATTTTT
TGCGGTTTCCGATCCGACACAGATGCCGCAAATTCAGAAAACCGTTTCTGCCGCAGGGAT
TGAGGTTGAGTTGGTCATTGTCGAGGATGACCAGTTGGCGGGTTTGCTCGATTGGGTGGG
TTCGCGTTCGACATCGCTGCTTCAGGAGCTTGGGGAGGGGCAGGAGGAAGAGGAAAGCCA
CACCCTGTATATCGACAACGAGGAGGCAGAAGACGGCCCTGTTCCGAGGTTTATCCATAA
GACTTTGTCGGATGCCTTGCGCAGCGGGGCATCGGACATCCATTTCGAGTTTTACGAACA
CAATGCCCGTATCCGTTTCCGTGTGGACGGGCAGCTCCGCGAGGTGGTTCAGCCGCCCAT
TGCGGTAAGGGGGCAGCTTGCTTCACGGATTAAGGTAATGTCGCGTTTGGACATTTCCGA
AAAACGGATACCGCAGGACGGCAGGATGCAGCTGACCTTTCAAAAGGGCGGCAAGCCTGT
CGATTTCCGTGTCAGCACATTGCCGACGCTGTTTGGCGAAAAGGTCGTGATGCGGATTTT
GAATTCCGATGCCGCGTCTTTGAACATCGACCAGCTCGGTTTTGAGCCGTTTCAGAAAAA
ATTGTTGTTGGAAGCGATTCACCGTCCCTACGGGATGGTGCTGGTAACCGGTCCGACGGG
TTCGGGTAAGACGGTGTCGCTCTATACCTGTTTGAATATTTTGAATACGGAGTCGGTAAA
TATTGCAACGGCGGAAGACCCTGCCGAGATTAACCTGCCGGGCATCAATCAGGTTAACGT
CAATGATAAGCAGGGCCTGACTTTTGCCGCTGCTTTGAAGTCTTTCCTGCGTCAGGACCC
GGACATCATTATGGTCGGTGAGATTCGTGATTTGGAAACTGCCGATATTGCGATTAAGGC
GGCACAAACAGGGCATATGGTGTTTTCCACCCTGCACACCAATAATGCGCCGGCGACGTT
GTCGCGTATGCTGAATATGGGTGTCGCGCCGTTTAATATTGCCAGTTCGGTCAGCCTGAT
TATGGCGCAGCGTCTTTTACGCAGGCTGTGTTCGAGCTGCAAACAGGAAGTGGAACGCCC
GTCTGCCTCTGCTTTGAAGGAAGTCGGCTTCACCGATGAGGACCTTGCAAAAGATTGGAA
ACTTTACCGCGCCGTCGGTTGCGACCGTTGCCGGGGGCAGGGTTATAAGGGGCGTGCGGG
CGTGTATGAGGTTATGCCCATCAGCGAAGAAATGCAGCGTGTGATTATGAACAACGGTAC
GGAAGTGGATATTTTGGACGTTGCCTATAAGGAGGGTATGGTGGATTTGCGCCGGGCCGG
TATTTTGAAAGTTATGCAGGGCATTACTTCATTGGAAGAGGTAACGGCAAATACCAACGA
TTAGGTTTGAGAATGAAAATGCCGTCTGAAGCGTGTTTGTTTCAGACGGCATTTGACTTT
CAGGGTGTTTGCCGGGAAGGCGGGGCGGTCAGCGGTATGCCATGTCGGGTTCGGATATTT
CCGGCAAACTTTCCGTTTGGCCGGAAACCGTATATTTCCCGTCTGCCCATCCGCCCAAGT
CGATCAGTTTGCAGCGTTGCGAACAGAAGGGGCGGAATGCGTTTTCGGGTTTCCATACTA
CTGCTGTTTGACAGGTCGGACATTTGACTTGAAGGCGTGTTTGCCGCGATTCAGTCATTG
TGTTTTCCTTGTGTTGGTTTTGAGGCGAAAATCCCTGAATAAAACGCGTGCAGGCGCATT
GTTTTCTCACGCAGGCTTTTGAGGCTGCCGTCATTGAGCAGCACATCGTCTGCAAGCAGC
AGGCGTTCGGATTCGGATGCCTGATGGCTGATGACGGCCGCCACCTCGCCGCGCGTCAGC
CCGCTGCGGGCCATCACCCTGCCGATACGTTTTTCCACAGGGGCACTTATGGTCAGGACA
CGCCGTATCAGGCTGATAAATTGACGCTTTTCCGTCAGCAGCGGAATTTCGACAATGCCG
TAAGCTGCATCAGTAAAGGTTTCTTGCTGTTTTTTGATTTCTGAGAAAATCAGCGGCAAC
ATCACGGATTCGAGCAAGGCTTTTCGCGATGGGGAGGCAAAGACTTCTTTACGCAATATG
TCGCGCCGCAACAAACCCTGTGTGTCAAAAACGGTGTCGCCGAACAGCCGCCTGATTTCC
GGCAGGGCGATGCCGTCTGAAGCCGTCAGCGAGTGCGCCGCCGCGTCTGCATCGATGCGC
GGCACGCCCAAATCGGCAAAACATTGCGCGGCTGCCGATTTGCCGCTGCCGATTCCGCCG
GTCAGTCCGACCCATACCGTCATCTTACAGCACCGGATGGGTCAGCCACCAGTTGACCGC
CCGCCATACGGAATCGTTTGCCGTAAAAATTATCCAGCCCGAAACTGTCAGTGCGGGGCC
GAAGGCAAAATGCTGCCCCTTGGCGACGCGCATAACGATTGCCGCGACCAAACCGATCAG
CGAGGAAACAAAAATCAGTACGGGCAATGCGGATATGCCGAGCCACGCGCCCAATGCGGC
AATCAGTTTGAAATCTCCGTTGCCCATACCGGTTTTTCCTGTGAGCAGTTTATACACTGC
ACATAAGAGCCATAATGAACCATAGCCGGCGACCGCACCTAAAACGGCAGACTGCAAAGG
CACGAAGCCGCCGTCCAAATTAAATATCAGACCCAGCCAAATTAAGGGCAGTGTCATCGA
GTCGGGCAGGTATTGGGTGTCCGCATCGATAAAGGTCAGGGAAATCAGAAACGCGGTCAG
TACCAATCCGCCCAGCGTAATCCAAGACCAGCCGTATTGCCAGGCGACCAGCCCGAACAA
TACGCCGGTCAGCAGCTCGATTAAGGGATAACGTATGCTGATTTTGGTTTGGCAGGAAGC
GCATTTGCCGCGCAGGAGCAGGTAGCTGACAATCGGGATGTTCTGCCACGCGCGTATCGG
CACGCGGCATTTGGGACAGCAGGAATCCGGTTTCATCAGGTTGAAGGTACGGCTTTCCTC
TTCGGTCAGCGGCAGGTTTAAATATTCTTTGGCAAATACCGTCCAGCCGCGTTCCATCAT
GACCGGCACGCGGTAAATGACGACATTTAAGAAACTTCCGACCAGCAGCCCGAACACCGC
TGCCAAAGGCACGGCAAACGGCGACAATACAGACAAATCAGACATATTTTGTTCTCAATG
TATTCAAAACAAAAACAAACCGGCGCAGAGCGAATCCGCGCCGGATCTGTGCGGCAAATC
AGGCGACCACGTTGCCCAAATTAAACAGCGGCAGATACATGGCGACCAGAAGCGTGCCGA
TGACCAAGCCTAAAATCACGATAATGATCGGCTCCATCATAGCGGACAGCCTGCCGACCG
CATTGTCCACCTCGTCTTCGTAAAATTCGGCGGCTTTGTTGAGCATATCGTCCAAAGAAC
CCGATTCCTCGCCGATGGAAGACATCTGCAACATCATATTGGGGAACAGTTCCGTCGCAC
GCATCCCCGAAGTCATAGACAAACCTTGGATGACGCGCGTACGGATTTCCCGGGTGGCTT
CTTCATAGATTAAATTGCCCGCCGCGCCGGCAGTGGAGTCCAATACATCGACCAAAGGCA
CGCCTGCCGCAATCAGCGTCGCCGTCGTCCTGCCCCAGCGGGCAATCGTTCCTTTGCGGA
CAATGTCTCCGAAAATCGGCATACGCAGCAGTATGGCATCCATACGCCGTTGGATTTTAA

## Appendix A

TCGAACGCGCCTTCAATTTAAGGAAGCCGTATATGGCAAAGCCCAGTGCGATCAGCACCA
TCCAGCCGTATGAGACGAAAAAGTCGGACATATCCATCACTGTTTGGGTCAGTGCGGGAA
GCTCCGCGCCCATATTGGCGTAAACTTCTTTAAAGGCGGGCAGTACGAAAATCATCATCA
CGAATACCAAACCGATGGCGACGGCGATGACGGATACCGGATAGGTCAGTGCGGTTTTTA
CCTTTTTGCGGATGGCCTGGGTTTTTTCTTTGTAAATTGCCAATTTGTCCAGCAGGCTTT
CCAATACGCCGCCCGTTTCGCCCGCCGCAACCAGATTGCAGTAGAAGCGGTCGAAATATT
TTGGGTGGTTTGAGAATGCGCGGCTCAACGAGCTGCCCTGTTCCACTTCGCCTCGGATTT
CCATCAGCATTTCCGTCATAGACGGGTTGCCGTGTCCGCGCGCCACGATTTCAAATGCCT
GCATCAGCGGCAGGCCCGCTTTAATCATCGTGGACAGCTGGCGGGTGAAAACGGTGATGT
CTTCTTGTGTGATTTTGCGCTTGGAGCTTGTTTTCACACGGGTAATCTGCAACGGGCGGA
TGCCGCGTTTTGCCAGTTTTTTGCGCGCCTCTTCTTCGGTAAACGCGGATACTTCGCCGT
TGACCAGTTTGTCGGAGGCGGAATGCCTGCCTTCAAAGATAAAGCGTTTTTCTTTCTTTG
CGAACAAAGAAAATCCTCCGTTTTTAGCCATATTCTAGCCCCGTAAAGTAATTGGAATAA
AATGTAAGAAACATCGTTAAAAAACAGTACCGGCGTGTTCCCGGTAAGATGAAAACCGCC
GACATCCCGCCTGCGGGCGGCAAACGGGACAGAATCGGATGCGATTATACCTTATTTAGG
CGGCTGTCCGGCATTTATGCGTACACAATAAATCTTGCAGGATATTGTTGCGGGTCAAAT
GCCGGCCGGAGGGCATTTCCGCCATATGGAAATAAGGTGCTATTGGACGCGGCGGGCGGT
GTTCCGGAGATTCGCCAAAGCCGCTGCCGTTTGTTAAACTACATTCTGCTACATTTTAAT
CCGGTTCTGAAAAATCAAGGAAAACAGATGAATGCTTTTACCCGTGCATGGTATGCGCTC
GAACGCCATTATCAGGATACGCGTCATGTCCTTTTGCGCGACCGCTTTGCCTGCGAACCG
GACCGGTTTGAGCGTATGCACGAGCGTTTGGACGGGATGTTGTTCGATTACAGCAAAAAC
CGTTTGGGCGAAGATACGCTGCAACTGCTCTGCAATCTTGCCGACGCGGCGGATTTGGAA
GGGAAAATGCGTGCTTTGCGGACGGGTGCGAAAGTCAACGGCAGCGAGGGGCGTGCCGCG
CTGCATACGGCTTTGCGCCTGCCCGACGGTGCGGATGCCGTTTATGTGGACGGCAGGGAC
GTGTTGCCCGAAATCCGCCGCGAGTTAAATCGTGCGTTGAAGTTTGCACACAGTTTGGAC
GACGGTTCGTATCAGGGGATAACCGGAAAACGGATTACGGATTTTGTCCACATCGGCATA
GGCGGATCCGACCTCGGGCCGGCAATGTGCGTGCAGGCACTTGAGCCGTTCAGACGGCAT
ATCACCGTCCATTTTGCCGCCAACGCCGATCCTGCCTGCCTGGATGCGGTTTTATGCCGT
CTGAACCCCGAAACGACAGTGTTTTGCGTTGCCAGCAAGTCCTTCAAAACACCGGAAACC
CTGCTCAATGCACAGGCAGTCAAGGCGTGGTATCGCGGTGCAGGGTTCTCGGAATCCGAA
ACGGCGTGCCATTTTTGCGCGGTGTCTGCCGACACTGCGGCAGCTGCGGCTTTTGGTATC
GCGGCGGAACGCGTGTTTGCGATGTACGACTGGGTGGGCGGACGCTATTCCGTCTGGTCG
CCCGTCGGTTTGCCCGTGATGGTTGCGGTCGGCGGGGCGCGTTTCCGCGAGTTGTTGGCG
GGGGCGCACGCGATGGACAGGCATTTTTTCAGTACGCCGACGCGTCATAATATCCCCGTT
TTAATGGCACTGATTGCCGTGTGGTACAACAATTTCCAGCACGCGGACGGGCAGACCGCC
GTTCCGTACAGCCACAACCTGCGCCTGCTGCCGGCGTGGCTGAACCAGCTCGATATGGAG
AGTTTGGGCAAAAGCCGCGCTTCAGACGGCAGTCCCGCCGTGTGCAAAACGGGCGGCATC
GTGTTCGGTGGTGAAGGGGTCAACTGCCAGCACGCCTATTTCCAACTGCTCCACCAAGGC
ACGCGCCTGATTCCCTGCGATTTTATCGTCCCGATGACGGCGCAGGGCAGAGAGGACGGA
CGCAGCCGTTTTACCGTTGCCAACGCCTTTGCCCAAGCGGAAGCCTTGATGAAGGGCAAA
ACCTTGGACGAAGCACGCGCCGAACTGGCAGATTTGCCCGAAGCGGAACGCGAACGCCTC
GCGCCGCACAAAGAGTTCCCCGGCAACCGCCCCAGCAACAGCATTTTGATTGACCGCCTC
ACGCCCTACAATTTGGGTATGCTGATGGCGGCTTACGAACACAAAACCTTCGTCCAAGGC
GCGATATGGAACGTCAACCCCTTCGATCAGTGGGGGGTGGAATACGGCAAACAGTTGGCA
AAAACCATCATCGGCGAACTGGAAGGCGGCACGTCCGTACACGATGCCTCGACCGAAGGG
CTGATGGCGTTTTACCGCGAATGCCGTCTGAAAGGCGGCGGCGCGGCATAAAAGTACTGC
CGCCTTTCTGTATTGATTCGGGCGCGGAAAAGGCAATACCTGCCGCCTGCCCGATTCCGA
AACGCCAATGTTTGGCAACCGCTCGCGTATTGCTGACGAATATGCGTTTGCGTGGCACAA
TAGCGCATTCATTTCAAATGAACATACTGCTTGAAAATACCGGCAAGCGTCCCACGAAAC
ATCTCACATAAGGAAATATTATGTCTTTGCAAAACATTATCGAAACCGCCTTTGAAAACC
GCGCGGACATCACCCCGACCACCGTTACTCCCGAAGTCAAAGAAGCCGTGTTGGAAACCA
TCCGCCAACTCGATTCCGGCAAACTGCGCGTTGCCGAACGTTTGGGCGTGGGTGAGTGGA
AAGTCAACGAATGGGCGAAAAAAGCCGTGTTGCTGTCCTTCCGCATCCAAGACAACGAAG
TCCTCAACGACGGCGTGAACAAATACTTCGACAAAGTGCCGACCAAGTTTGCCGACTGGT
CTGAAGACGAGTTCAAAAACGCAGGCTTCCGCGCAGTTCCGGGTGCGGTTGCCCGACGCG
GCAGCTTTGTGGCGAAAAATGTCGTGCTGATGCCATCTTATGTCAACATCGGCGCATACG
TCGACGAAGGCGCGATGGTCGATACTTGGGCAACCGTCGGCTCTTGCGCGCAAATCGGTA
AAAACGTGCACTTGAGCGGGGGCGTCGGCATCGGTGGTGTACTCGAACCCCTGCAGGCCG
CACCCACCATCATTGAAGACAACTGCTTCATCGGTGCGCGTTCTGAAATCGTTGAGGGCG
TGATTGTCGAAGAAGGCAGCGTGATTTCTATGGGCGTGTTCATCGGTCAATCCACCAAAA
TCTTTGACCGTACAACCGGCGAAATCTATCAAGGCCGCGTACCGGCAGGTTCGGTTGTCG
TATCCGGCAGTATGCCTTCCAAAGACGGCAGCCACAGCCTTTACTGCGCCGTCATCGTCA
AACGCGTGGACGCGCAAACCCGTGCGAAAACCAGCGTCAACGAATTGTTGCGCGGCATCT
GATGCCTTAAACCGTATTTGAAACGTCCAATGCCGTCTGAAATCCGCTTCAGACGGCATT
GCCGTTTGCACGCTGCAACGTGAAAACACAGAAACAGGGACAATTTGCTATAATCAACGG
TTTAGAACGAACCGAACACTATTTGAAGGATACAAAATGGGTTTTCTGCAAGGCAAAAAA
ATTCTGATTACCGGCATGATTTCCGAGCGTTCCATCGCTTACGGCATCGCCAAAGCCTGC
CGCGAACAAGGCGCGGAACTGGCGTTTACCTACGTTGTGGACAAACTGGAAGAGCGCGTC
CGCAAAATGGCGGCGGAATTGGATTCCGAACTTGTATTCCGCTGCGATGTCGCCAGCGAC
GACGAAATCAACCAAGTGTTCGCCGACTTGGGCAAACATTGGGACGGCTTGGACGGTTTG
GTGCATTCCATCGGTTTTGCGCCGAAAGAAGCCTTGAGCGGCGACTTCCTCGACAGCATC
AGCCGCGAAGCGTTCAACACCGCACACGAAATTTCCGCATACAGCCTGCCCGCGTTGGCA
AAAGCCGCCCGTCCGATGATGCGCGGCAGAAATTCCGCCATCGTCGCCCTGAGCTACTTG
GGCGCGGTGCGCGCGATTCCGAATTACAACGTGATGGGTATGGCAAAAGCCAGCCTTGAG
GCAGGCATCCGCTTTACCGCTGCCTGTCTGGGTAAAGAGGGCATCCGCTGCAACGGTATT

## Appendix A

TCCGCCGGCCCGATTAAAACGCTTGCCGCCTCCGGCATCGCCGATTTCGGCAAACTCTTG
GGACACGTCGCCGCCCACAACCCGCTCCGCCGCAACGTTACCATTGAAGAAGTCGGCAAT
ACCGCCGCCTTCCTGCTGTCCGACCTGTCGTCCGGCATTACCGGCGAAATCACTTACGTT
GACGGCGGTTACAGCATTAATGCCTTGAGCACCGAGGGATAATCCGCCGTTTTCAAATCC
GTGCGCCGTCCGTGCCGCATATCGGTTTCGGGCGGCGTTTTGCCGTCTGAAGCGTATTTC
TAGGGAAATGCCCGACTTACGGCAGGCGGGATGGGAAATGCGGACGCTTGTTTTAACCGA
TTGCCTTTGTGCCGACTTGCTGCAGGTGCAGCGGAAACGGTTCGGATGCGAAAATGCCGT
CTGAAACGCCAAACGGGTTTCAGACGGCATTTTTATTTAAAGCATCAGCACACTTCAAC
CAGCCAGCCGTATTTGTCTTCCGCCAAACCATACTGGATGTCGGTAATCGCCTTACGGAT
GGCATAGCCGCGTTCTTGGCTTTTCACTTCGATTTCTTTGCCGCCGATGACGAAGGAAGT
AACGGGCGAGATGACGGCTGCCGTACCGGTCAAAATGGCTTCCGCACCGTTTTCCACCGC
AGCTTTGAGTTCGTCAACCGTGAAATTGCGTTCGCTGACGGTATAGCCCAAATCTTTGGC
AACCGTCAGTACGGAATCGCGGGTTACGCCGTGCAAAAACTCGTCGGTCAGCGGTTTGGT
AATGATTTCATCGCCGTTAATCAGGATAAAGTTGGACGCGCCGGTTTCCTGCACGTCGCC
GTTCGGGCAGAACAGGACTTGATTTGCGCCATATTCGGCTTTCGCCTTCAGCACCCAGTG
CATGGCGGAAGCGTAGTTGCCGCCGCATTTGACGCGGCCCATATGCGGGGCGCAGCGGAT
GTGTTCGGTTTCCACCAAAATTTTGACGGGCGATCCGACTTTGAAATAGTCGCCGACGGG
GGAAGCCAAAATATACAGCAGGGCGGTTTCGGAAGGAGAACCGGCCTTGCCGATAACGGG
ATCGGTACCGATTAAGGTCGGACGCAGGTACAGGGCGGCAGGCGCATCGGGAATTTCATC
GGCGGCACGTTTGACCAATTTGATTAGCGCGTCAAGATAAGCTTCGGTTTCGGGGCGCGG
CAGGTGCAAAATGTCCGCACTTTGCCGCATACGCGCGATATTGGCAGTCGGACGGAACAG
CACGATTTTGCCGTCTGCCTGACGGAAGGCTTTCAGTCCCTCGAAACATTCGCTGCCGTA
GTGCAGGGCGTGCGCGCCCGGTGCGAGGGAGAGGTCTTGGGAAGATTGCCATTCGGTCGG
CTGCCATTTGCCTTCGCGGTAGGCGAGGACGGGCATTTGACTGTGAAAAACGCTGCCGAA
TACGGCGGGTACGGGTCTGCTCATGATGTAAAGCCTTTCTTATTCTGATATGTTTCAATG
AACGGTTTGAATTTGAAGATTGTAAAGATACGCCTGCAAACAGGGTTTTGACAAGTGCGC
GGCGGGTTTTTCTGTCGATGCGGTGTCCAATCCGTTATTTTTCAAATGGAAAGGAACGGT
GTATTTGGTAAAATTGTCGGCAATCGCATACTCCGTATGTCGTCCGAACACGCTGCCGCA
TCCTATCCGAAACCGTGCAAATCGTTTAAACTAGCGCAATCTTGGTTCAGAGTGCGAAGC
TGTCTGGGCGGCGTTTTTATTTACGGAGCAAACATGAAACTTATCTATACCGTCATCAAA
ATCATTATCCTGCTGCTCTTCCTGCTGCTTGCCGTCATTAATACGGATGCCGTTACCTTT
TCCTACCTGCCGGGGCAAAAATTCGATTTGCCGCTGATTGTCGTATTGTTCGGCGCATTT
GTAGTCGGTATTATTTTTGGAATGTTTGCCTTGTTCGGACGGTTGTTGTCGTTACGTGGC
GAGAACGGCAGGTTGCGTGCCGAAGTAAAGAAAAATGCGCGTTTGACGGGGAAGGAGCTG
ACCGCACCACCGGCGCAAAATGCGCCCGAATCTACCAAACAGCCTTAAGAAAGCCGATAT
GGACAACGAATTGTGGATTATCCTGCTGCCGATTATCCTTTTGCCCGTCTTCTTCGCGAT
GGGCTGGTTTGCCGCCCGCGTGGATATGAAAACCGTATTGAAGCAGGCAAAAAGCATCCC
TTCGGGATTTTATAAAAGCTTGGACGCTTTGGTCGACCGCAACAGCGGGCGCGCGGCAAG
GGAGTTGGCGGAAGTCGTCGACGGCCGGCCGCAATCGTATGATTTGAACCTCACCCTCGG
CAAACTTTACCGCCAGCGTGGCGAAAACGACAAAGCCATCAACATACACCGGACAATGCT
CGATTCTCCCGATACGGTCGGCGAAAAGCGCGCGCGCGTCCTGTTTGAATTGGCGCAAAA
CTACCAAAGTGCGGGGTTGGTCGATCGTGCCGAACAGATTTTTTTGGGGCTGCAAGACGG
TAAAATGGCGCGTGAAGCCAGACAGCACCTGCTCAATATCTACCAACAGGACAGGGATTG
GGAAAAAGCGGTTGAAACCGCCCGGCTGCTCAGCCATGACGATCAGACCTATCAGTTTGA
AATCGCCCAGTTTTATTGCGAACTTGCCCAAGCCGCGCTGTTCAAGTCCAATTTCGATGT
CGCGCGTTTCAATGTCGGCAAGGCACTCGAAGCCAACAAAAAATGCACCCGCGCCAACAT
GATTTTGGGCGACATCGAACACCGACAAGGCAATTTCCCTGCCGCCGTCGAAGCCTATGC
CGCCATCGAGCAGCAAAACCATGCATACTTGAGCATGGTCGGCGAGAAGCTTTACGAAGC
CTATGCCGCGCAGGGAAAACCTGAAGAAGGCTTGAACCGTCTGACAGGATATATGCAGAC
GTTTCCCGAACTTGACCTGATCAATGTCGTGTACGAGAAATCCCTGCTGCTTAAGTGCGA
GAAAGAAGCCGCGCAAACCGCCGTCGAGCTTGTCCGCCGCAAGCCCGACCTTAACGGCGT
GTACCGCCTGCTCGGTTTGAAACTCAGCGATATGAATCCGGCTTGGAAAGCCGATGCCGA
CATGATGCGTTCGGTTATCGGACGGCAGCTACAGCGCAGCGTGATGTACCGTTGCCGCAA
CTGCCACTTCAAATCCCAAGTCTTTTTCTGGCACTGCCCCGCCTGCAACAAATGGCAGAC
GTTTACCCCGAATAAAATCGAAGTTTAACCACCACCGAAAGGAACACAAAAAATGCGCTT
ACTCCATACTATGCTCCGCGTGGGCAATCTCGAAAATCCCTCGATTTCTACCAAAACGTT
TTGGGTATGAAACTGCTCCGCCGAAAAGATTATCCCGAAGGCAGATTTACCCTTGCCTTC
GTCGGTTACGGCGATGAAACCGACAGCACGGTTTTGGAACTGACGCACAACTGGGATACG
GAACGATACGACTTGGGCAACGCCTACGGACACATCGCGGTTGAAGTGGACGATGCCTAC
GAAGCCTGCGAACGTGTGAAGCGGCAGGGCGGAAACGTCGTCCGCGAAGCCGGCCCGATG
AAACACGGCACAACCGTGATAGCCTTCGTCGAAGACCCCGACGGATACAAAATCGAGTTC
ATTCAAAAGAAAAGCGGCGACGATTCGGTTGCCTATCAAACTGCCTGATACCGCCGCCGC
CAATGCCGTCTGAAGCCTTTAGGGGTTTCAGACGGCATTTTGTTGCCGTCGACCTGCTGT
TTGAGCCTGTGCCGGTTCAAACTTTATCCGTTACACCGATAAGGCAAAAAAGATGCCGTC
TGAAACGGCATCCTTGATCTGCGAAAGGGCAGTTGGGAATCAAATACCCAATTCCTGCGC
CAATGCTTGGGCACGTTTGAGTACGTCGCCTTCCGCTTCTTCCAGCAATTTCTGCACTGT
CTCGGCAGCGGCATCGCGGTCGCCGATTTCGAGATACATTTCGGCAAGGTCGTATTTCGC
TTCGGAAGGCGCGTCAGAACCTACAGATTCCGAAGGGAAACTGGTATCTGCATTATTTGG
GATATTTTCTTCCGAGAGGTAGATGCTCCAATCTACCGTTTCCTCCTCGCCGTCTTTCAG
GAAGTCGGGCAAAGCGTCTGCCTCAGAGGTGTTGGAATCAGGCGTTTCCAAAGTGATTTC
CGCTGCATTTTCCTCAACGGCCGGTGCTTCAGCAGGTTGCAACAGTGCGGACAAATCATC
GGCAACGGTTTCCGCTGCATTTTCCTCAACGGCAGGTGCTTCAGAAGGTTGAAGTAATGC
GGACAAATCGTCTGCGGTGGCGTTGAAATCGGGTGTTTCGGCAACGGTTTCCGTTACATT
TTCCTCAACGGCCGGTGCTTCAGCAGGTTGCAACAGTGCGGACAAATCATCGGCAACGGT
TTCCGCTGCATTTTCCTCAACGGCAGGTACTTTAGAAGGTTGAAGTAATGCGGACAAATC

## Appendix A

GTCTGCGGTGGCGTTGAAGTCGGGTGTTTCGGCAACGGTTTCCGTTATATTTTCCTCAAC
GGACGGTGCTTCGGCAGGTTGAAGCAATGCGGACAAATCGTCTGCGGCGGCGTTGAAATC
GGGCGTTTCAGGCGCAGTTTCCGCGACGGCATCGGTTTCGTACACTTTCAGGAAATCGTG
CAACTCTTCCGGTGTTTGGACTTCGGCAACTGTTTTTCCAAGATGGTTTCGGGCGAGGA
AGCCTTCAGGAAGCCTGCCAGTCCGGAGGGTGAGGCAGGTTTTGCGGAAGCTGTTTCTTC
TGTGCCGATATGGTTGTTTGAGGGCAGGTTGTCGGAGAAATCGGTATCGACGGTTTCCGG
TTTGTTTTCGGCAGTTTGGGCGACAGATTCCGGTTCGGGCGTGTCGATGACGATTTCGAC
AGGGTTGTACGGGTTGAAGGTCTCGGGCTCGTACACGCTGTCTGTGGATTCGATGGCGTT
CCAATCGGCATCCGCGCGTTTTTGGGTTTCTTCATCCTGCGTAAGTGCGCCGGATAAAAT
GCCGTTTTGCGCGGCTGCCAGGCTGTCGAAATCCAAGTCGATGCGGTTGGAAGGCGTATC
GGTTTCGACATCGAACGTTTGTTTTGCCGATAACTCTTCTTCAGATTCCCCATCTAAGGC
AAGTGTGTCGTTTACATCGTTTTTCGGAGCGGGTTCGGGCGTTGCCGGAGTTTCGACTTC
GGCAAAGGTGATTTCTATGCCGTCGTCTGCCGCGTCGTCAAGGTCAGGCTCTTCCTCAGG
GACGGATTCTTCGGTACGGCGCGCGCGTTTGGATTGGGCAAGGCGCAAAAGCAGCAGCAG
GGCGATTAATGCCGCGCCTCCGCCGGCAAGCAGCAAGGTGTACGAACCGCCGAACAGACC
GTCAAACAGTCCGCTTTCGGTTTCTTCTTCGGCAGAAACCTGTTCGACAGGTTCGGAAAC
GGCGTTACCGGTTTCGTCGGTCGGCGTGTCGATGGCAGAAGCGGCGGCTTCTTGGGGGGC
GGATTCGGCAGCGGTTTCCGATGCGGCAGTATTTGCAGCGGGTACAGGTTCGGGTCGAAC
GGCCGGTTTTTCCGCTTTTGCTTCGGGCGCGGCAACTTTTGCTTCAGGTTTTTCAACCGG
TTTCTCTACCGTTGCCTGTTTGGACGGTTCGGACGGCATGGATGCGGTTTCGGCTTTGGG
TTTCGCCGTTTGCGGTTTGGGTTGTTCCGCTTTGATCCTGTTCAGATTCGGAATGTGAAG
CACGCTGCCCGCACGCAGTCTGCCGTGTGCGGAAACATTTGGGTTTGCCTTCAGCAGCGC
ATCGGCAACCTGTTCGAGCGTCAGGTGTTTCGGGCGGATGGCGGCGGCAATCTGTTTGAC
CGTTTCGCCTTTGCGGACGGTATGGGTTTTGCCGTTGTATGCCGGTTTGACGGCTGCGTT
CGCGCTGTCTTTTTTATCGGTTTTGCGGAGGGCTTTGGCGTTTTGATTTTCTTGGGACTC
TGCTGTCGGAGCGGTTTTGCGGTGTGTCTTGCCGTCTGAAAGTGCAGATTTGGTTTTGGG
CGAGTAGCCGACAGGATCGAGGATGGCGGTGTATTCGCGTACCTGTGCGCCTGCGCCGAT
GCGGAACACCAGGACGGGATCGCGGACTGCCTGTTCGGAAGAAACGGCAATGACGGCTTT
GTCGCCCAACTTGTGGACTTTGGCGGTCAGGCCTTTTTCGGAAACGGTAACGCTGCCGCC
GCCTAGCAGGGCTTTGGCTTCTTCGCCGGTTACGGTAATGCTGCCGGAAAAGGGTTCGTC
AAGGTTGGACTGGATATTCAGTCCGCCCAGTCCAGCATGTGCCTGAAAGGATGCGGCAAC
TGCGACGGAGGCGGCAATCAGTTTGATTTGTCTGTTGTTTTTCAAGATGTATCCCCTGTG
GGTTGGCGGCTGAATACGGTTTGACCGCGTACAGTCTGTAAATTTCGTCATCATCGGGCA
TCGGCGGGGCAGTCGGCCGGCGGGCATTTAATATGTGAATGTACCGACCGCCGCCACATT
TTAAACGGCAATCATTCGCCGTTTTTACAAATTATGACATATCTCCATCTTTTTTCAAAA
ACATCTGTGCATATTTGCATCAATCAAAACAAAATTTGTTGGTTTTGCAGGTGCAAAAAC
AGGGTTCTGCCTGTATGATTAGCGTTTATTTGATTTGCTTTCTCATTTGGATATGAAATT
CGTCAGCGACCTTTTGTCCGTCATCCTGTTTTTCGCCACCTATACCGTTACCAAAAACAT
GATTGCCGCAACGGCGGTCGCATTGGTTGCCGGTGTGGTTCAGGCGGCTTTTCTGTATTG
GAAATATAAAAAGCTGGATACGATGCAGTGGGTCGGATTGGTGCTGATTGTGGTATTCGG
CGGCGCAACCATTGTTTTGGGCGACAGCCGCTTCATTATGTGGAAGCCGAGCGTTTTGTT
TTGGCTGGGCGCGCTGTTCCTGTGGGGCAGCCACCTCGCCGGTAAAAACGGCTTGAAGGC
GAGTATCGGCAGGGAGATTCAGCTTCCGGATGCCGTATGGGCGAAATTGACGTATATGTG
GGTCGGTTTCCTGATTTTTATGGGTATCGCCAACTGGTTTGTGTTTACCCGGTTCGAGTC
GCAATGGGTCAACTATAAAATGTTCGGCTCGACTGCACTGATGCTTGTTTTCTTTATTAT
TCAGGGTATTTATCTGAGTACCTGTCTGAAAAAGGAGGATTGACTGTGGAATATTTTATG
TTGCTGGCAACAGACGGGGAGGATGTGCACGAGGCGCGTATGGCGGCACGTCCCGAACAC
CTCAAACGGCTGGAGACGCTGAAGTCGGAAGGCCGGCTGTTGACGGCAGGCCCGAATCCT
TTGCCGGAGGACTGCAACCGGCGTTTCGGGCAGTTTGATTGTGGCGCAGTTCGAGTCTTTG
GATGCGGCGCAGGCTTGGGCGGAAGACGATCCCTATGTTCATGCAGGCGTGTACAGCGAA
GTGCTGATCAAGCCGTTTAAAGCGGTGTTCAAATAATGCCGGCCGTCGATTTGATCCGCG
AACGCCTGCAGACGCTCGATCCGCTGGTGTTGGAAATCGGCGATGAGAGCCATCTGCACA
AAGGACACGCGGGCAATACCGGCGGCGGACATTATGCCGTTTTGGTCGTTAGCGGCCGTT
TTGAAGGCGTAAGCCGCCTGAACCGCCAGAAAACGGTCAAATCGCTGCTCAAAGATTTGT
TTTCAGGCGGCATGATTCACGCGCTCGGCATCCGGGCGGCTACCCCTGACGAGTATTTCC
ATACGGCGGACTGAATGAAGTCTGCCCGAACATTTCAATTTAAAATTTAAAGAGAGAAGA
TTATGAAAGCAAAAATCCTGACTTCCGTTGCACTGCTTGCCTGTTCCGGCAGCCTGTTTG
CCCAAACGCTGGCAACCGTCAACGGTCAGAAAATCGACAGTTCCGTCATCGATGCGCAGG
TTGCCGCATTCCGTGCGGAAAACAGCCGTGCCGAAGACACGCCGCAACTGCGCCAATCCC
TGCTGGAAAACGAAGTGGTCAATACCGTGGTCGCACAGGAAGTGAAACGCCTGAAACTCG
ACCGGTCGGCAGAGTTTAAAAATGCGCTTGCCAAATTGCGTGCCGAAGCGAAAAAGTCGG
GCGACGACAAGAAACCGTCCTTCAAAACCGTTTGGCAGGCGGTAAAATATGGCTTGAACG
GCGAGGCATACGCATTGCATATCGCCAAAACCCAACCGGTTTCCGAGCAGGAAGTAAAAG
CCGCATATGACAATATCAGCGGTTTTTACAAAGGTACGCAGGAAGTCCAGTTGGGCGAAA
TCCTGACCGACAAGGAAGAAAATGCAAAAAAAGCGGTTGCCGACTTGAAGGCGAAAAAAG
GTTTCGATGCCGTCTTGAAACAATATTCCCTCAACGACCGTACCAAACAGACCGGTGCGC
CGGTCGGATATGTGCCGCTGAAAGATTTGGAACAGGGTGTTCCGCCGCTTTATCAGGCAA
TTAAGGACTTGAAAAAAGGCGAATTTACGGCAACGCCGCTGAAAAACGGCGATTTCTACG
GCGTTTATTATGTCAACGACAGCCGCGAGGTAAAAGTGCCTTCTTTTGATGAAATGAAAG
GACAGATTGCGGGCAACCTTCAGGCGGAACGGATTGACCGTGCCGTCGGTGCACTGTTGG
GCAAGGCAAACATCAAACCTGCAAAATAATTCTGAAAACGGGATATGGCGGCAAGACGTT
CAGACAGGCGTTTTGCCGCCGCGCAGGACAGGGAATACCATGAAACAGAAAAAAACCGCT
GCCGCAGTTATTGCTGCAATGTTGGCAGGTTTTGCGGCAGCCAAAGCACCCGAAATCGAC
CCGGCTTTGGTGGATACGCTGGTGGCGCAGATCATGCAGCAGGCAGACCGGCATGCGGAG
CAGTCCCAAAAACCGGACGGGCAGGCAATCCGAAACGATGCCGTCCGCCGGCTACAAACT

## Appendix A

TTGGAAGTTTTGAAAAACAGGGCATTGAAGGAAGGTTTGGATAAGGATAAGGATGTCCAA
AACCGCTTTAAAATCGCCGAAGCGTCTTTTTATGCCGAGGAGTACGTCCGTTTTCTGGAA
CGTTCGGAAACGGTTTCCGAAGACGAGCTGCACAAGTTTTACGAACAGCAAATCCGCATG
ATCAAATTGCAGCAGGTCAGCTTCGCAACCGAAGAGGAGGCGCGTCAGGCGCAGCAGCTC
CTGCTCAAAGGGCTGTCTTTTGAAGGGCTGATGAAGCGTTATCCGAACGACGAGCAGGCT
TTTGACGGTTTCATTATGGCGCAGCAGCTTCCCGAGCCGCTGGCTTCGCAGTTTGCCGCG
ATGAATCGGGGCGACGTTACCCGCGATCCGGTCAAATTGGGCGAACGCTATTATCTGTTC
AAACTCAGCGAGGTCGGGAAAAACCCCGACGCGCAGCCTTTCGAGTTGGTCAGAAACCAG
TTGGAGCAGGGTTTGAGACAGGAAAAAGCCCGCTTGAAAATCGATGCCCTTTTGGAAGAA
AACGGTGTCAAACCGTAATGGCATTTCCAATACCGATGCCGTCTGAAGCCTTTCAGACGG
CATTGCACGTTCAGGTAAGGAGGACGGCTTATGCGTGCGGTCATACAGAAAACGGTAGGT
GCAAAGGTGGATGTCGTGTCCGAAGCCGGCACGGAAACCTGTGGCAAAATCGACGGCGGG
TTTGTCGTGTTACTCGGCGTAACGCATAGCGACACAGAAAAAGATGCACGCTATATCGCC
GACAAAATCGCCCATTTGCGCGTGTTTGAAGACGAAGCGGGCAAGCTGAACCTGTCTTTG
AAAGATGTCGGCGGCGCGGTGCTGCTGGTGTCGCAGTTTACGCTTTATGCCGACGCGGCA
AGCGGGCGGCGGCCTTCGTTTTCCCAAGCCGCACCTGCAGAACAGGCGCAGCAGCTTTAC
CTGCGAACGGCGGAACTGTTGCGCGGACACGGGATTCATGTCGAAACAGGGCGTTTCCGC
ACGCATATGCAGGTGTCGCTCTGCAACGATGGGCCGGTAACCATACTGCTGGACTCTTTC
ATGACGCGGATTTCCCCAAAAATGAAGGTTGTTCCGGATTGAAATTGAATCCGCAATGAT
AAAATATCGACAATGAACGACAATACACACACCCTTCCCCCGCGCCACCTGTCCGTCGCC
CCCATGCTCGACTGGACGGACAGGCACTACCGTTACCTTGCCCGCCAGATTACCCGAAAT
ACTTGGCTGTACAGCGAAATGGTCAATGCCGGTGCGATTGTTTACGGCGACAAAGACCGC
TTTTTGATGTTCAACGAAGGCGAGCAGCCCGTCGCCCTGCAACTGGGCGGCAGCGATCCG
TCCGATTTGGCGAAAGCCGCCAAAGCCGCCGAGGCATACGGTTACAACGAGGTCAACCTC
AACTGCGGCTGCCCCAGTCCGCGCGTGCAGAAAGGCTCGTTCGGCGCGTGTCTGATGAAC
GAAGTCGGGCTGGTTGCCGACTGCCTCAACGCCATGCAGGATGCGGTCAAGATTCCCGTT
ACCGTCAAACACCGCATCGGTGTGGACAGGCAGACCGAATACCAAACCGTTGCCGATTTC
GTCGGCACGCTGCGCGACAAAACCGCCTGCAAAACCTTTATCGTCCACGCCCGCAACGCT
TGGCTGGACGGTCTTTCCCCCAAAGAAAACCGCGACGTTCCCCCGTTGAAATACGATTAC
GTTTACCGCCTCAAGCAGGAGTTTCCCGGGCTGGAAATCATCATCAACGGCGGCATCACC
ACCAACGAAGCAATCGCAGGACACCTGCAACACGTTGACGGCGTGATGGTCGGGCGCGAG
GCGTACCACAACCCGATGGTGATGCGCGAATGGGACAGGCTGTTTTACGGCGATACCCGC
AGCCCGATTGAATACGCCGATTTGGTGCAGCGTCTCTACACATACAGCCAAGCCCAAATC
CAAGCCGGACGCGGCACAATCTTGCGTCACATCGTCCGCCACAGCCTTGGGCTGATGCAC
GGTCTGAAAGGCGCGCGGACTTGGCGGCGTATGCTTTCCGACGCAACGCTCTTGAAAGAC
AACGACGGCAGCCTGATTCTCGAAGCGTGGAAAGAGGTCGAACGGGCAAATATGCGCGAA
TAGGGCGGGGCTGTATGTGTGAAATGCCGTCTGAAGGCTTCAGACGGCATTTGTGCGTTT
GTCGGGCGGTGTTTAGGGGGCGGTAACGGCGTGTTTCGGCACTTTGTCCATATCCCAGTG
TGCCACCGCCCAGTCGAGCAGTTCGGCAGGGCGGTCGGTTTCCGGTGCTTCGGGCAGCTT
GAGGTAACGGAACACTTGGCGGAGGAGTTGTTCGCGGCGGTTTAAATCCAATGCGGGGGC
GAGCGTCTGTTTCGACCATTTCTGCCCTTGTGCGTTGGTCAGCAGCGGCAGGTGGGCATA
TTGCGGTGTCGGAACGTCCAAACACTGCTGCAAATAGATTTGGCGCGGCGTGGAAACGAG
CAGGTCTTGTCCGCGGACGATGTGGGTAACGCCCTGTTCGGCATCGTCGGCAACGACGGC
GAGCTGGTATGCCCAGTAACCGTCTGCACGAAGCAGGACGAAATCGCCGATGTCGCGGGC
GAGGTTTTGGGCGTAACCGCCGACGATGCCGTCTGAAAAACCGATAATGCGGTCGGGGAC
GCGGATGCGCCACGCCGGCTGTTTGCCTTGCAGTGCAGGGCGTTGGCCGGGGTGGCGGCA
ACGTCCGTTATAGACGAACCCGTCTGCGCCCCGCCTTGCCCCGGCCTGCCAGTCTTTGCG
GCTGCAATGGCAGGGATAGACCAGTCCGGCGGTTTTCAGGCGGCATAGGGTTTCTTCATA
CAGGGCGTAACGGCGGCTCTGATAGGCGACTTCTCCGTCCCACTCGAATCCGAATGCCTC
AAGCGTGTGCAGGATATGGCTTGCCGCCCCCGGCATTTCGCGCGGCGGATCGAGGTCTTC
CATGCGGATCAGCCATTTGCCGCCGTGCGCGCGCGCATCGGCATAGGAAGCGACGGCGGT
CAGCAGCGAGCCGATGTGGAGCAGCCCGGTCGGGCTGGGGGCAAAACGTCCTGTGTACAT
ATCTGGTACAGCCCCTTTATTTAAGACTATTAATCAAAGCCATTATCTCATCTTTATTCA
GTTCCATCCCGGGCTCTTCAAGCAAGGTTAAATCATATAGGGCATTATATTGCTCTTCGG
TAGCTGAACCATCCATAAGAGCAGGCGAGAAAAAATCAAAGGCTCTATCTGCAATTCTCT
CATTACTTGCATTTCTACTAACCAGTTTCGTCAATTCTGTATATTTTGAAAAGTTTATGG
AAAAATAAAACAGCGAAAAAGTTTTGGTTTCGCTGTTTTTGATTTAATTAGCACTGATAA
TCTTCAAATTCCCACGAAAAAAAACGAAGTAAATAAGTCAATGACTTTTCCCAAGTTTCT
TTTGAACATTCTTTAAGAATTTTCTCAATTTCCGATTTAATAACAGAATGATTAAATTCA
TTCATAATCATCATACCCGCCCCCCATTTAACCCTTTGATTTTGGAAACAATTATGCAAA
ATCCATTTAGGAGAGCATATGCGAACAGAAAATATATCTGCAGCATCACTATCATCAGTT
CCTATGTCTAAATCAATTCCCACACAAAAATTGTCTTTGATTTCGGGAACGAAATCTTCA
AAGGCACAATCGTAAAGATTGATGGCTTTCAATTCTAGGTTAATCATTTTATATTCAATA
GTATGGGGAGGTACCGGATCCTTAAAAATCAGATCTGAATAAATTTCATTGGGTGAAATG
ATTTCGATTGCTTTTGCCATGATTCTATTTCCTTTTGTGTTAGTGGGTAATGTCGTGCAT
TAACTTCTTGCCCATTAATATTTTTAGGGTGAATCCTTGATATGCCGCACTGTGTCCGGT
CAAACGGGCGATGCCGTCTGAAAGCCTTTCAGACGGCATCGGGAAAATGCCTAAGCCAAA
GGCGCGAGCAGTTTTTCAAACGCTTCTTCAAACTGTTTCAAACCGTCTTCCTGCAAACGC
GTTGCCAAGGTTTCGACATCGATGCCGAGCGCGGCGGTTTCGGCGAGCTGCGCTTGTGCT
TCTTCTACGCCTTCGGTCAGCGTGGCTTTGGCTGTGCCGTGGTCGATAAAGGCTTTGAGC
GTGGCATCGGGAACGGTGTTGACGGTGTGCGCGCCGATCAGGCTGTCAACGTAGAGCGTG
TCGGGATAGGCCGGGTTTTTCACGCCGGTAGATGCCCATAAAAGCTGCACGCGGTTTGCG
CCTTTGGTTTCCAGCGCGGCAAATTCGGGGCTGCCGAAGTATTGCGCCCAGTCTTGGTAG
GCGGCTTTGGCAAGGGCGATGGCGATTTTGCCTTTGAGGTGGTCGGGCAGTGTTGTGTCC
AGCGCGCCGTCCACACGGGAGATGAAGAAGCTGGCGACAACTTGGATATGGGCAACGCTT

# Appendix A

TGTCCGGCTGCTAAGCGTTTGGCGATGCCGCGCGCGTAGGCGGCGTAGGCTTTGAGGGTT
TGGGCGCGTGAGAACAGCAGGGTCAGGTTCACGCTGATGCCGTCTGAAACGAGGGTTTCG
AGCGCATCGATGCCTGCGTCGGTGGCAGGCACTTTAATCATCGCGTTTTTGCACCCGATG
GCGGCGTAGAGGCGGCGCGCTTCTTCAACCGTGCCTTGCGCGTCTTTGGACAATTCGGGC
GAAACTTCGAGGCTGACGAAGCCGGTTTTGCCGCCGGTGGATTCGTGTTCGGCAAGGCAA
ACGTCGCAGGCGGCACGCACATCGGCAACCGCCATTGTTTCGTAGCGTTGTTTGGGGCTG
AGGTTTTGCTGCTTGAGGGCGGCGATTTCATCGGCGTAAAGCGCGTCGCCGGCGAAGGCT
TTTTGGAAGATGGCGGGATTGGAAGTTACGCCGCACACGCCCTGTTTCAACATTTGCGCC
AATTCGCCGCTTTGCACTAGCGAGCGGGAAAGGTTGTCCAGCCAGATTTGTTGTCCTAAT
GCTTTAACGTCCGATAAAATGGTCATCTCTGATTCCTTTGGATGGATAGGCGGGGTTTGA
GGGCTTATGCTACCCCGATTCGGAAATTTTGGGTAGTTTTATTACAGCAAAGGCGGATGG
CAATGGCAGAAAACGGAAAATATCTCGACTGGGCACGCGAAGTGTTGCACGCCGAAGCGG
AAGGCTTGCGCGAAATTGCAGCGGAATTGGACAAAAACTTCGTCCTTGCGGCAGACGCGT
TGTTGCACTGCAAGGGCAGGGTCGTTATCACGGGCATGGGCAAGTCGGGACATATCGGGC
GCAAAATGGCGGCAACTATGGCCTCGACCGGCACGCCTGCGTTTTTCGTCCACCCTGCGG
AAGCGGCACACGGCGATTTGGGTATGATTGTGGACAACGACGTGGTCGTCGCGATTTCCA
ATTCCGGCGAAAGCGACGAAATCGCCGCCATCATCCCCGCACTCAAACGCAAAGACATCA
CGCTTGTCTGCATCACCGCCCGCCCCGATTCAACCATGGCGCGCCATGCCGACATCCACA
TCACGGCGTCGGTTTCCAAAGAAGCCTGCCCGCTGGGGCTTGCCCCGACCACCAGCACCA
CCGCCGTCATGGCTTTGGGCGATGCGTTGGCGGTCGTCCTGCTGCGCGCACGCGCGTTCA
CGCCCGACGATTTCGCCTTGAGCCATCCTGCCGGCAGCCTCGGCAAACGCCTACTTTTGC
GCGTTGCCGACATTATGCACAAAGGCGGCGGCCTGCCTGCCGTCCGACTCGGCACGCCCT
TGAAAGAAGCCATCGTCAGCATGAGTGAAAAAGGGCTGGGCATGTTGGCGGTAACGGACG
GGCAAGGCCGTCTGAAAGGCGTATTCACCGACGGCGATTTGCGCCGCCTGTTTCAAGAAT
GCGACAATTTTACCGGTCTTTCGATAGACGAAGTCATGCATACGCATCCTAAAACCATCT
CCGCCGAACGTCTCGCCACCGAAGCCCTGAAAGTCATGCAGGCAAACCATGTGAACGGGC
TTCTGGTTACCGATGCAGATGGCGTGCTGATCGGCGCGCTGAATATGCACGACCTGCTGG
CGGCACGGATTGTATAGTGGATTAACAAAAACCAGTACGGCGTTGCCTCGCCTTAGCTCA
AAGAGAACGATTCTCTAAGGTGCTGAAGCACCAAGTGAATCGGTTCCGTACTATCTGTAC
TGTCTGCGGCTTCGTCGCCTTGTCCTGATTTTTGTTAATCCACTATATAAGGCGTTGCAG
CCGTTTCAGACGGCATTTGTGGTAAGATATGCCGTCTGAAAACAAGGAAATCCCATGCAG
GCAATTTCTCCCGAATTACAGGCGCGCCGCCAAAATCAAACTGTTGATCCTGGATGTG
GACGGCGTTTTGACCGACGGGCGCATCTTTATCCGCGATAACGGCGAAGAAATCAAATCG
TTTCACACACTGGACGGACACGGTCTGAAAATGCTTCAGGCAAGCGGCGTGCAGACTGCG
ATTATCACGGGCCGGGACGCGCCCTCCGTCGGCATCCGCGTCAAACAGTTGGGCATAAAT
TACTATTTCAAAGGTATCAGCGACAAACGTGCCGCCTATGAAGAATTGCGCGCGCAGGCG
GGCGTGGAAGAAGCCGAGTGCGCCTTTGTCGGCGACGACGTGGTCGATTTGCCGGTAATG
GTGCGCTGCGGATTGCCGGTTGCCGTCCCCGGCGCGCATTGGTTTACGCGGCAACACGCC
GCCTATATCACGGAACACGCGGGCGGCGCAGGCGCGGTGCGCGAAGTGTGCGACCTGATT
ATGCAGGCGCAAGGGACTTTGGGCGCGGCTTTGAACGAGTACATCAAATGAAAGTAAGAT
GGCGGTACGGAATTGCGTTCCCATTGATATTGGCGGTTGCCTTGGGCAGCCTGTCGGCAT
GGTTGGGTCGTATCAGCGAAGTCGAGATTGAAGAAGTCAGGCTCAATCCCGACGAACCGC
AATACACAATGGACGGCTTGGACGGCAGGCGGTTTGACGAACAGGGATACTTGAAAGAAC
ATTTGAGCGCGAAGGGCGCGAAACAGTTTCCGGAAAGCAGCGACATCCATTTTGATTCGC
CGCATCTCGTGTTCTTCCAAGAAGGCAGGTTGTTGTACGAAGTCGGCAGCGACGAAGCCG
TTTACCATACCGAAAACAAACAGGTTCTTTTTAAAAACAACGTTGTGCTGACCAAAACCG
CCGACGGCAAACGGCAGGCGGGTAAAGTTGAAGCCGAAAAGCTGCACGTCGATACCGAAT
CTCAATATGCCCAAACCGATACGCCTGTCAGTTTCCAATATGGTGCATCGCACGGTCAGG
CGGGCGGCATGACTTACGACCACAAAACAGGCATGTTGAACTTCTCATCTAAAGTGAAAG
CCACGATTTATGATACAAAAGATATGTAAGCTATTTGTTTTAATAGCATTTTTTTCGGCG
TCCCCCGCTTTTGCCCTTCAAAGCGACAGCAGGCAGCCTATTCAGATTGAGGCCGACCAA
GGTTCGCTCGATCAAGCCAACCAAAGCACCACATTCAGCGGAAACGTCGTCATCAGACAG
GGTACGCTCAATATTTCCGCCGCCCGCGTCAATGTTACACGCGGCGGCAAAGGCGGCGAA
TCCGTGAGGGCGGAAGGTTCGCCAGTCCGCTTCAGCCAGACATTGGACGGCGGCAAAGGC
ACGGTGCGCGGACAGGCAAACAACGTTGCTTATTCATCTGCAGGCAGCACCGTAGTCTTA
ACCGGTAATGCCAAAGTACAGCGCGGCGGCGATGTCGCCGAAGGTGCGGTGATTACATAC
AACACCAAAACCGAAGTCTATACCATCAGCGGCAGCACAAAATCCGGCGCAAAATCCGCT
TCCAAATCCGGCAGGGTCAGCGTCGTTATCCAGCCTTCGAGTACGCAAAAATCCGAATAA
TCCCAAAATGCCGTCTGAAATATAAACCCGGTTCGGACGGCATATGCCGACCGAAGATAT
TGAAGAGATATTTATGAGTGCAAACGTCAGCCGCCTTGTTGTTCAAAACCTGCAAAAAAG
TTTCAAAAAACGCCAAGTCGTTAAAAGCTTCTCCCTCGAAATCGAAAGCGGCGAAGTCAT
CGGACTGCTCGGGCCCAACGGTGCGGGTAAAACCACCAGCTTCTACATGATTGTCGGACT
CATCGCCGCCGACGCAGGCAGCGTAACCCTAGACGGACAAGAATTGCGCCACCTGCCCAT
ACACGAACGCGCCCGCCTCGGTGTCGGCTACCTGCCGCAGGAAGCCTCGATATTCCGCAA
AATGACCGTCGAACAAAACATCCGCGCCATCTTGGAAATCAGAACCAAAGATAAAAATCA
AATCGACAGGGAAATCGAAAAACTGCTCGCCGACCTCAATATCGGACACTTACGCCGCAG
CCCCGCGCCGTCGCTGTCCGGCGGCGAACGGCGGCGCGTCGAAATCGCCCGCGTACTCGC
CATGAAACCGCATTTTATTTTGTTGGACGAACCTTTTGCCGGCGTCGATCCGATTGCCGT
CATCGACATCCAGAAAATCATCGGTTTCCTCAAATCGCGCGGTATCGGCGTACTGATTAC
CGACCACAACGTACGCGAAACCCTCAGCATCTGCGATCGGGCCTACATTATTTCAGACGG
CACGGTGTTGGCATCGGGAAAACCTGATGATTTGGTCGGAAACGAACAGGTTCGTTCTGT
TTATCTGGGTAAGAACTTCAAATATTGAAAATATTTTTCAGACGGGCGACCTAATATCGT
CGGGCAGGCGGCAAAAATACGGATTTATGTTGTTTTTACATAAATTAATTCAAATTTAAA
ACATTGACTTAAACCTGTTTTCAAAGAATATTGCCCGATATGCTTGCATGTCGTCCCGTA
ATTTGGTTTAATACGCATCTCTTAACGAGACAGACAAAGGCCAGATAGCTCAGTTGGTAG

## Appendix A

AGCAACGGATTGAAAATCCGTGTGTCGGCGGTTCGATTCCGCCTCTGGCCACCAAAAAAC
CGCCTTGAAGCGGTTATTTTTTTGCCTGCCGTTTTTGGGAAGTTGTCCGTGTCGGACAC
GTTTTGTGTCTGACCGTTATGTAGAAGGGCAAAAATGATAATGACCGCCCCGTTGCGTTT
TGGAGAAGAGGGTAAAGGCAGAAAGCATATGCCGTCTGAATGATATTTCAGACGGCATTT
TATATTGCGGCGGCACTCAGTCCGTGTCGCTTTCAGGCAACTCTGCCGAACCCATGCGTT
TGAGCACGATATTGGTTTTGTTGCGGAGCCGTTTGCTTTTCGGATGGTCGGCGTAGTAGA
GCGGGGCGGGGACGCGCGCCGTCAGTTTTGCCGCCTGCTGTTTGGTCAGCTTGGCGGCGG
GTATTTGATAAAAATACCGGGACGCGGCTTCCGCGCCGAAAACGCCGTAGTGCCATTCGA
TTGAGTTTAAATACAGTTCAAAAATCCTGTCTTTGTCGGTAACGGCTTCCATCATCGCGG
TAATCGCCGCTTCTTCGCCTTTGCGGATATAGCTGCGGCTTTCGTTTAAAAACAGGTTTT
TGGCAAGCTGCTGGCTGATGGTCGAGCCGCCCGCCTTCACTTTGCCGCTGTTCCGGTTGC
GCCTGATGGCGTTTTGAATGCCGCCCCAATCGAAGCCGCCGTGCCCGGCGAAACGGGCAT
CTTCGGAAGCAATCAGGGCTTTTTTCAGGTTGGTGGAAATGCGTTTGTAGGGCATCCAGC
GGTAATCCAGTGCGACATCGCGACCTTCCTGTTCAAACTGCTTCATCCGCATCGACATAA
AGGCAGTCCGATGGGGCGCGACGGCGCGGTAGGTAATGATGTTGCCGTACACATAGGCAT
TGAAAAAGATAAAGATGCCGACGGGCAGGGCAATCAGCCATTTGATGATGCGGAACATGT
TTATAGGGCTTTCATGTATTCGATAACGGGGCGGATATCGGGCGTAAATCCGCGCCAGAG
GGCGTAGGAAGCCGCCGCTTGACCGACTAGCATACCCAGTCCGTCGGCAGTTTTTTTCGC
ACCCGATTGTCGTGCAAAATCTAAAAACGGTTTTGCCGCGCAGCCGTACACCATATCGTA
GGCAAGCGCGCAGTTTTGAAAAATATCGGGCGGAATATCGGGAATCTGACCGTTTAGACC
GCCCGACGTGCCGTTGATGATGATATCAAAACCGCCGTTCACGTCCGCCATCGGGACGGC
TTCAATGCCGAAAAGCTGCGCCAATTCCTCGGCTTTGGCGCGGTACGGTTGGCAATGAC
GATACGGGCAGGACGGTGTTCTTTCAAAACAGGAATCACGCCGCGCACCGCGCCGCCTGC
GCCCAAAAGCAAAATGGTTTTGCCCTCGATGGCAATATTTTTGACCTGCGTGATGTCGTT
GGTCAAACCGATACCGTCGGTGTTGTCGCCACGCAGCTTGCCGTTTTTCAACGGAATCAG
CGTATTGACCGCACCTGCCGCCAATGCGCGTTCGGAATGCTCGTCCGCCAGATGAAACGC
TTCCTGTTTGAACGGTACGGTAACGTTTGCCCCGCAACCGCCTGTTTCAAAAAATGTCGA
AACCGCCTGCGCGAAACCGCCGATGTCGGCGCAAATGCGTTCGTATTCAATGTCAACGCC
TTCCTGAAGGGCAAATTGTTGATGAATTTGCGGCGATTTGCTGTGGGCGACGGGGTTGCC
GAAAACGGCGTAGCGGGGGAGGGCGGTCATGGTCGTGTTCCAAAAGACGGGAAGGCTATT
TTATAACGGCGGCGTACAGATGGAAACGATGCCGTCTGAAACCGCCTTCAGACGGCATCG
TTTCCTGTATCGGTCGGGAAAAATCCGGATGCGGTGCGCCGGCTTGTCCGCATTGTTGAC
AATCTTGCCGTCTGAAACTATATTTTCCGGCTTGAAATTTGACGCAAAACCGGTTTCAGA
CGGCATCGGCGTGGTAAAATCGTGCCGACTTTGCGTCAAGCCGCCGCGTTCCGCATATTT
TGCTATTTCCCTTTTCCAGGAGCTGAAAAATGTCTATTAAAAACGCCGTAAAATTGATTG
AAGAAAGCGAAGCCCGCTTTGTCGATTTGCGCTTTACCGATACCAAAGGCAAGCAGCACC
ACTTTACCGTGCCTGCGCGCATCGTGTTGGAAGACCCCGAAGAGTGGTTCGAAAACGGTC
AGGCGTTTGACGGTTCGTCTATCGGCGGCTGGAAAGGCATTCAGGCTTCCGATATGCAGT
TGCGCCCCGATGCGTCTACAGCCTTCGTCGATCCTTTTTATGATGATGCGACTGTTGTGT
TGACTTGCGACGTTATCGATCCCGCCGACGGTCAGGGTTACGACCGCGACCCGCGCTCCA
TCGCCCGCCGAGCCGAAGCCTATTTGAAATCTTCCGGCATCGGCGAGACCGCCTATTTCG
GTCCCGAACCCGAGTTTTTCGTATTCGACGGCATAGAATTTGAAACCGATATGCACAAAA
CCCGTTACGAAATCACGTCCGAAAGCGGCGCGTGGGCAAGCGGTCTGCATATGGACGGTC
AAAACACCGGCCACCGCCCGACCGTCAAAGGCGGTTACGCACCTGTTGCACCGATTGACT
GCGGTCAGGATTTGCGTTCGGCGATGGTAAACATTTTGGAAGAACTCGGTATTGAAGTGG
AAGTGCACCACAGCGAAGTCGGCACCGGCAGCCAAATGGAAATCGGCACGCGCTTTGCTA
CTTTGGTCAAACGCGCCGACCAAACCCAAGACATGAAATATGTGATTCAAAACGTTGCCC
ACAACTTCGGCAAAACCGCCACTTTCATGCCCAAACCCATTATGGGCGACAACGGCAGCG
GTATGCACGTTCACCAATCCATTTGGAAAGACGGTCAAAACCTGTTCGCAGGCGACGGCT
ATGCCGGCTTGAGCGACACCGCGCTCTACTACATCGGCGGCATCATCAAACAGGGCAAAG
CCTTGAACGCGATTACCAATCCGTCCACCAACTCCTACAAACGCCTCGTGCCGCACTTTG
AAGCGCCGACCAAACTGGCATACTCCGCCAAAAACCGTTCCGCTTCCATCCGCATTCCGT
CCGTGAACAGCAGCAAGGCGCGCCGCATCGAAGCGCGTTTCCCCGATCCGACCGCCAACC
CGTATTTGGCATTTGCCGCCCTGTTGATGGCGGGTTTGGACGGCATTCAAAACAAAATCC
ATCCGGGCGACCCTGCCGATAAAAACCTGTACGATCTGCCGCCGGAAGAAGATGCATTGG
TGCCGACCGTTTGCGCTTCTTTGGAAGAAGCACTGGCCGCCCTCAAAGCCGACCACGAAT
TCCTCCTGCGCGGCGGCGTGTTCAGCAAAGACTGGATCGACAGCTACATCGCTTTCAAAG
AAGAAGACGTACGCCGCATCCGCATGGCGCCGCACCCGCTGGAATTTGAAATGTATTACA
GCCTGTAAGCACGTCTGGTTTTCAGAAAAGCAATGCCGTCTGAACACAGTTTCAGACGGC
ATTTTGCATTTGAACGGCAAACCGGCGGCGCGGGGCGGCATTTTTCAGCAGGCGGGCGAT
ATTTGCTACAATAGGCTTTTGTTTTTTTTGGGCTGCACGAACGATGACTGCATCGAAATC
AGGTTTTATCGGGCAAATCTTTTCCCGCAATATGCTTGTCTGTATTTTTACGGGGTTTAC
CTCGGGGCTGCCGCTGTACTTTCTGATTAACCTGATTCCGGCGTGGTTGCGCAGCGAGCA
GGTGGATTTGAAGAGCATCGGGCTGATGGCGTTAATCGGTCTGCCGTTTACTTGGAAATT
TTTGTGGTCGCCGCTGATGGACGCGGTCAGGCTGCCCGTTTTGGGACGGCGGCGCGGGTG
GATGCTGCTGACGCAGGCAGGGTTGCTGGCGGCTTTGGCGGTCTATGCCTTTTTAAACCC
CCGTAATCATCTGCCGCTGATTGCCGGCTTGTCGGTGCTTGTCGCTTTTTTTTCCGCCAG
TCAGGATATTGTATTGGATGCGTTCAGGCGCGAGATTTTGTCAGACGAAGAATTGGGTTT
GGGCAACTCGGTTCATGTGAACGCCTACCGGATTGCCGCCCTGATTCCCGGTTCATTGAG
TTTGGTGTTGGCAGACAGGATGCCGTGGTTAGAAGTATTTGTTATCACTTCATTATTTAT
GCTGCCCGGCCTTCTGATGACGCTGTTTCTTGCGCGCGAACCCGTGTTGCCTCCTGCCGT
TCCTAAAACGTTGAAGCAGACCGTGGTAGAGCCGTTTAAAGAATTTTTACGCGCAAGGG
CATCGCTTCGGCGGTGTGCGTGCTGCTGTTTATCTTCCTTTACAAACTCGGCGACAGTAT
GGCAACCGCGTTGGCAACGCCGTTTTATCTGGATATGGGTTTCAGCAAGACCGACATCGG
TTTGATTGCGAAAAATGCAGGACTGTGGCCGGCAGTGGCGGCAGGTATCTTGGGCGGTGT

## Appendix A

GTGGATGCTGAAAATCGGCGTAAACAAAGCCTTGTGGCTATTCGGCGCGGTGCAGGCTGT
AACCGTTTTGGGGTTTGTATGGCTGGCAGGGTTCGGACCTTTCGACACGGTCGGCACAGG
CGAGAGGCTGATGCTGGCGGCAGTTATCGGCGCGGAAGCGGTCGGCGTGGGGTTGGGGAC
GGCGGCGTTCGTATCGTATATGGCGCGTGAAACCAATCCCGCATTTACCGCAACGCAGCT
TGCGCTGTTTACCAGCCTGTCCGCCGTCCCGCGCACGGTCATCAATTCCTTTGCCGGTTA
TCTGATTGAATGGCTCGGTTATGTACCGTTTTTCCAACTGTGTTTCGCACTCGCCCTACC
GGGTATGCTGCTGCTGCTGAAAGTTGCGCCTTGGAACGGGGAGAAAACTCAGGATGCAGG
CAGATGAACGCGTCAAACTGGAGCGTTTACCTGATATTGTGTGAAAACAGCGCGTTCTAT
TGCGGCATCAGCCCGAATCCGCAACAGCGGCTTGCCGCCCACACAACCGGTAAAGGCGCG
AAATATACCCGCGTATTCAAACCGGTGGCGATGCGTATCGTTGCAGGCGGGATGGATAAA
GGAACGGCACTCAGGCAGGAAATCGCCGTCAAAAAACTGACCGCCGCACAAAAACGGCAA
TTGTGGGAGCAGGCAGAAAAAATGCCGTCTGAAACCTGACGGTTCAGGTTCGGACGGCAG
TTGGCAGCAATCAGGGAAAAGCGGGGCAGGCGGTAAGGAAAACCGACGTTTCAACACACA
GGACGGTACATAAAGCGTCGCCCTATGAAAGTGAAGGCATATATCAGTATTTTTTATACG
CCAACAGAAAAGAATACGATGAACTGTTTGTTGGATTTGTATTGATTAATCAGTATATTT
TTTATGCCGGGGTATTTTTCCTTATCGGTATCCCTTCTTTTATGAGGATGCCTGCCGCTC
ATATAAAGAACGGGAAAATACGATGGGAAAATACGGTACAGCCCTCGACATCGCACAATA
TGTCAACTTATAGTGGATTAACAAAAATCAGGACAAGGCGACGAAGCCGCAGACAGTACA
GATAGTACGGCAAGGCGAGACAACGCCGTACTGGTTTTTGTTAATCCACTATATTTGTTT
GTTTTATATTGTAAGTATACGTATAGGCTTTGTAAAGGTAAATTGTGAAAAAAGCAGTTT
TTTAAACGAATGAAACGGCTTCGGGCTGAAATATATGCTGATGCCCTGTCCTTCCCGTAT
ATCTTGTGTGTTGTCAAAGTGCAGGCTGCTTTGAAATCGGTATTGCCATCTATGAACCAC
CACTTTGTTTTATTTCAGCGGGCTTGAGATGTGTATAAGAATATTGTTTTGAATAAATTT
AAAAAAATGATAATCGTTATTGAAGATTTTTAAAGGAAAGCGTAGAGTGCCAATTCTATG
AAGCAATACGGTAAGTAACAATGAAAATATCTACTGCTTGGGTATAGAGCATATTTCACA
ACCCGTAACTATTCTTGCGGAAACAGAGAAAAAAGTTTCTCTTCTATCTTGGATAAATAT
ATTTACCCTCAGTTTAGTTAAGTATTGGAATTTATACCTAAGTAGCAAAAGTTAGTAAAT
TATTTTTAACTAAAGAGTTAGTATCTACCATGAATATATTCTTTAACTAATTTCTAAGCT
TGAAATTATGAGACCATATGCTACTACCATTTATCAACTTTTTATTTTGTTTATTGGGAG
TGTTTTTACTATGACCTCATGTGAACCTGTTAATGAACAAACCAGTTTCAACAATCCCGA
GCCAATGACAGGATTTGAACATACGGTTACATTTGATTTTCAGGGCACCAAAATGGTTAT
CCCCTATGGCTATCTTGCACGGTATACGCAAAACAATGCCACAAAATGGCTTTCCGACAC
GCCAGGGCAGGATGCTTACTCCATTAATTTGATAGAGATTAGCGTCTATTACAAAAAAAC
CGACCAAGGCTGGGTGCTCGAACCATACAACCAGCAGAACAAAGCACACTTTATTCAATT
TCTACGCGATGGTTTGGATAGCGTGGACGATATTGTTATCCGAAAAGATGCGTGTAGTTT
AAGCACGACTATGGGAGAAAGATTGCTTACTTACGGGGTTAAAAAAATGCCATCTGCCTA
TCCTGAATATGAAGCTTATGAAGATAAAAGACATATTCCTGAAAATCCATATTTTCATGA
ATTTTACTATATTAAAAAAGGAGAAAATCCGGCGATTATTACTCATCGGAATAATCGAAT
AAACCAAACTGAAGAAGATAGTTATAGCACTAGCGTAGGTTCCTGTATTAACGGTTTCAC
GGTACGGTATTACCCGTTTATTCGGGAAAAGCAGCAGCTCACACAGCAGGAGTTGGTAGG
TTATCACCAACAAGTAGAGCAATTGGTACAGAGTTTTGTAAACAATTCAAGTAAAAAATA
ATTTAAAGGATCTTATTATGAATGAGGGTGAAGTTGTTTTAACACCAGAACAAATCCAAA
CCTTGCGTGGTTATGCTTCCCGTGGCGATACCTATGGCGGTTGGCGTTATTTGGCTAATT
TGGGTGACCGTTATGCGGATGATGCTGCTGCAATTGTCGGTAAGGATGCAAACTTAAATG
GTTTGAATTTATGGATGAAAAAAGGGGTGGAAAACCTATGGGATGATACGGTCGGTAAAA
AGACCCGTTTAATGTGTATTTCCGTTTTTGGATTGTGGTTTTCAATTTGTAGCGAATCG
GATTCGGCATATACGGCATTGCAAAAAGCGTTTGACTCTCCAATGCCGTCTGAAAACCGG
TTTCAGACGGCATTTGCGTTCAGTGAGAAAGGTCGCGCCTGCCGCCCGAACGTCTCGCCG
CAGCCTCTGCATAACGGCGCACCTCTTTTTCCAAATTTTCCAAGTTCAAAGGAAAATCAG
GCAGTCTGTCTCCCTGTTTCTCTTCGCGGACAATCCGCCCGCCATCCAAATACCACGTCT
GTTGCGCATGATAGGTCTGCATATCCGCCGTTACGCCATCCGCTTTCAATGCTACCGTCG
AAGATTGTGCAATAAAAAGATTTCCGTTTTTCAAATAATATTCGAAACTCTGGCGTTTTT
TTCCATTGTCGAAACTCCAATAGACTTTTTGCGGCAGACCGTCCGCATCATAGCCGACCA
CAAGACTGTTCGCCTTCATCCCTCGGGGCATCAATTCCCGCATATTCTGATAAAACACAG
AATTGCGCGAGTCCGACGCAATTCGGTTGCTCTCTTTGCGGAAGTCCCAAACCTTCTGCT
CGTCATTCGCGACATCCCGGTATTTCGCCAAATATACCTGGGCCATCTGATAACACCCGA
GGCAATGCTCATAAACATCTTCCCCGATTTTCCCGCGCCCCGCCGCATCAAATACCGAAC
CGTCTGGTTGCCAAACAACCCGATATTCTCCTGTCGTTTCATAATTTTCCCCGTGAACCG
TTCCGCCGTACACATTTACAGAAAACGGACGATCGTTCCGATACAGATATTCGGCATTAA
CAAATGCTTCCGGCGAGCGTTGCGAAAGCGAAACCGCAACCAAACCGCCCTCGCCGATAT
GGTAATCCAGCCAAACCTCTTTCCCATGTTCCTGCTCCGTTACGTGAAACCATTTCGCCT
TTTCTTTCAAACGACTGAGCCGGATAGCGAGCGCGAGATAATCCTTCTCCGACTGCAACG
GACCGTCATCCACAGTTCCGGCAAGATTTTCCTCCGTCCTTATCGATTCCTTCACGATGA
CAACCGCCCTGTCGGCATTTCGGAACAGGCGGGCAAGTTTCGCCACAAAAGCATTCGGAT
TTTTAGGTACTTCAGTTGCCGTATCGCTCAAAAACCAACGCGGATTAATCTCATAGGCAA
TACCCGTTCCCAGCCAAAAGGCAAATACAAGTGCAAAAAATGACAACAGTACCGGTTTGA
ATTTTTTAAACATATTTATTTTTCGTTTAACAGAATATATCGATTATATCAGACGAGCTT
TGATTGCCGGGTTTTGCTATTTTTTGTTGTAATAATCAAATTGCACGTTGACTATGTCTT
TCTCGGTAAAAATATAACGGAGCATTGTTTTAAGCCTTTCATAACGTTCATTAATTCCTA
CGCTATCAGGTAGCCAAGGGGAAGCTTTAATTTCAAAAAGTTTCCAATTTGGAACCATTA
AGAAATCAATAATGGTACCGATTCCAATGACAACATATCTTGGTATGTCCATCGGATAAG
GATATTTTTTTCTAACCTCGATTAAATCATTCTCCAACTTCCAATATTCTTCATCATCCC
ACACCCCGTCATCATACCATTTGCCAATAAATGAATTTTCGTCATACCCCTCAAAACAAG
TAATATTTCTTCTGAAGTTTTTTAACTCACACATAATACACATAATAATTAATCTCCAAT
ACGATTTAGGTTTTTATCAAATGTACCGTTTCTTGTTTCTTTTCTGTAATGTTATTCATC

## Appendix A

GTAGTAAGGTTCTGTTGAATAATTGTCTTTGCCCCCGGCAATGATAGTAACAATTTTCCC
TTTTGCTTCCCAAGCTTGTACTCCTATTTCATCAAACTCATAGACATATGTCGGATAAGA
TTCATTTGATAAATAATATTTATCAACACCGTATGATTTAGGGTAATGGAAAAGCTGTTT
AAAATCTTCAAAATTCAGACCTATTATATTAACGCCCATAAAATATAGCTCCTGATAACA
AAATATCGAAATAATTTTGTTTTTTTTTTGACGGAAATGAGTAAATTTGAGTCGGGAGA
TTCATAATATTCTGTTCTAAACTCATCAGGAGGTTCATACATAAAAGTTTCCAGTATGTT
TTTGTACTGTGTTATATCCGCACCAAAACGGAATATTCCTACAGAAGTAAAAGGTAAAAA
TTCGGGAGTTTTAACGACCGCGTCGACCATGCTCTTCTCCTTTTGTTTTTCGATTGGCAT
TTTTGGCAATATTTCTGATTTTTTGCTTAATCTTTAAGCGTTCATTTTTGGACATTCCGG
GAATAATTTTATTTGTTAATTCAGCAATTTTTGATTCCGCTGATATTTGACTTCGACCGC
CATCTCCATGTTTTTCATTCTTGGAGCTTCCTGTTCTTTTAGGCGGACAAGAATTATGAA
CCCAAACCCCTTCCGTTTCCGCCTGATTACCCTTGACGAAGTAAGTATGCCAATCGGCAA
CGGTCAGATTGTAGGCTTTGAGCGGTTTTGGTTTGACAACGGTTTTGCGGACGGTTTGGG
TTCTGCCGCTTTCGGATAACAGCCTGCTTCCCGCTTTCAAATCTTCCGCTTTAATCCATT
TGCCGTCCGAATAAAACGGATGGATGCGGTTGGAAATCAGGATTTGGCTGTTGCCGATGC
CGTCTGAAAGCCGGATATCGCTTCAGACGGCATTTTGATTGCCGGGTTTTGCTATTTTTT
GTTGTAATAATCAAATCGCACGTTGACTATGTCTTTCTCGGTAAAAATATAACGGAGCAT
CGTTGTGAATCTTTCATAACGTTCATGAATTCCCACACTATCAGGCAACCAAGGGGAAGC
TTTAATTTCAAAAAGTTTCCAATTTGGAACCATTAAGAAATCAATAATGGTACCGATTCC
AATGACAACATATCTTGGTATGTCCATCGGATAAGGATATTTTTTTCTAACCTCGATTAA
ATCATTCTCCAACTTCCAATATTCTTCATCATCCCACACCCCGTCATCATACCATTTGCC
AATAAATGAATTTTCGTCATACCCCTCAAAATAAGGAACGTTTCTTATAATATCCTTGAA
CTCACACATAATAATGTATCTCCAATATAATTAAACTTTTCGTCTCAATCTACCTTTACT
ATGTTGTATTGGAAAGTAAAAAAATTTCCAGTCCTCTACATCTAGATCAGTAAAAATATA
ACGGAGCATTACCCTGAACCTTTCATAACGCTCATTAATTTTGACACTTTTAGGCAACCA
AGTAGAAGCTTTAATTTCAAAAAGTTTCCAATTTTGAACCATTAAAAAATCAATAATGGT
ACCGATTCCAATCACGATGTCCCTTGGTATATCCATCGGATAAGGATATTTTTTTCTAAC
CTCAATTAAATCATTCTCCAATTTCCAATATTCTTCATCATCCCACACCCCGTCATCATA
CCATTTGCCAATAAATGAATTTTCGTCATACTCCTTAAAACAAGGGATGTTTCTTCTAAA
ATCCTTGAACTCGCACATAATAATTAATCTCCAATACGATTTAGGTTTTTATCAAATGTA
CCGTTTCTTGTTTCTTTTCTGTTCAGTTTTTCGGGTGAAGATGCCTCTTTCCAAGCACCT
CCATTATGTGAATCTACATCGCGTGATATATAACTCTTTCCTTTTTTAAAAATAGCAGCA
TCATTTCTCGTTCTTTCTTTTATTTTTCTATATCCCAATTCCTTTGCTGCTGCATATGCT
TCTGAATCATTCCCATATATGGGGGTAGATGGTGTTTTTCTTGGCGGACAATCATTATGA
ACCCAAACCCCTTCCGTTTCCGCCCGATTGCCCTTGACGAAGTAAGTATGCCAGTCGGCA
ACGGTCAGATTGTAGGCTTTGAGCGGCTGCTGTTTGAGGGTAATGTTTTGAACCGTCTGT
TTTGCACCGCTTTCGGAAAGCAGGGTGTCGCCTTTTTTCAGACGACCTGCCTGTATCCAT
TTTCCTTGACTGTAAAACGGGTGGATTTTATTGGAAATCAGGGTTTGGTTGTTGCCGATG
CCGTCTGAAATTTCAATGTAAACGGTTTCTTGATACGGATTGCCGTATCGGGCGGTAACG
GGTTTGTATCCCGTTTTTCCGCTTGCCTCGTCCTTGGCGAAGACGCGGTCGCCGGTTCGG
ATACGGGCAATGGCTTTGTAGCCGTCTGCCGTTTTGACCAAGGTGCTGCCGTGGAAGGAG
CAGGTGTAGGATTTTTAAAGAACTTAGTTCTCAATATCCTGTTTCATTCATCAAAATGCC
GTCTGAAAGCTGAATACCGCTTCAGACGGCATTTTGGTGGTTGGGTTTTTAAGCCAACCT
ACGCTTACTGAAAACCAAATTGAGTTTCAGACAGTTTTTAGGTTTGGGTGTCCAATCTAA
CTTATATTTGTCCATTTAATTAGTCGTTTGTATCAAATTTCCATTATTTATTTTCCAATT
TACTTTATAATTATCTTCATAATAATCTAATTCAAAAAAACCTGATATTTCAATATCCAA
TTCCATTATTGTTTTAATACATTTTTCAAAATAAATAATGAAATAAGATTTTACGCATGC
ACCAAAAAAAAATATAGCTGCTCCAATTAAAACTATTTGTCGGGAAAACCCACCCGCTTTT
ATATATTTTTGCAGATTCTTTCTCTTCGATATTAAAGGGACAATTATTCCAAAAATTATT
AATGTTTTCTTGGATGATTTTTATATCATCGTCAGAGCATTCAATCCATCCTTTTATGGA
AACATATGATGCCATGTTTAATCTCCTAAACCTGTTTTAACAATGCCGCCTTTTGATTCA
ATATATGACTTAACTTGTGAATGAACACCGTATTTAAACCAAAATTCTGCACGTTTTCCC
TGTTGGTTTGCTGCTTCGATGGTTGCTTTAATTTGCTTTCTATTTTTTTGATTTAAGAAA
TTTTTAGGTTTATCTATTGCTGAAATTGTTCTTTTGGCTTGTATTAAAGCATCATTCGTA
ACAGCGTCAATTTCTCTGCCGTTAATAAATTTTGATGAACCATCAGTTTTTCTTCTAATT
AAATCTTCATAATGTATATCTAGAGCTTCTCTATACTTTGCATTTTGATATAACTGTCTC
GCACTATCAGACAAAGCCAATTTCTTTTTATAAGAATCAGCAAAATCCCCGCTAACCGCA
GCCTTCCCTGGTTTTGCCGCCTTTGCCAACTTCGCGACTTTGGCTGCTGCGGCAACGTTG
AAGACGGCTTCGACGGTTTCGGCGGCATTGGGATTTTCCTGTATCCACCGGTCAACGGCT
TCGCGCGTATTCTTTTCAAAGCCCGCCACGCTGCCCAAGCCGCCGATGACGGCGAATTTG
CCCTCGGCGGGCAAGGGGGCGATGTTGCGCATTGCGGCTTTGTCTATGGCATAGCGCGTT
CCGTACAGTATGTCGCCTATGCCCAAGGCTTCGCCCGCGCTGATAAAGGGGTTGAGCGCG
CCGGCGGCGACGCCGTTGATAAACTCCATGCTGTTGCCCCAGCGGTCGAGCTTGGCATTG
TGCTCGAACATTTTTCTGTTGGCTTCATCGGCGCGGTCGGAGAAATTGCTGCCGAGGTTG
CTGTAATTGTCGGATATGCGTTGCCGGATGCTGCGGGTGTCGGTCGGATTGAGTTTGATA
CTGCGGGCTGTGCCGTTGACGTGATAGGTGTATTCGTCTCGTGCGCCCGTAGGTTTGGGG
TAATTGCCGCCCTTCGGGCCGTCGTAGGCATCGGCGGGATGATGTTCGTGTCCTTCCCAG
TTGAGCCGGTATACGGTAAAGCCTTCGTCAACGTTGCCTTTTTCTTCGCTCGCGCTGTCG
GCGGCGTGGTTGTCGAAGGGGGCGTGTTCTTCGTGTCCGTGTCCGGAAAAGCGGGTGTGG
TAGCCGATTGTGCCGTTGATGTTTGCCTGTTGGATGAGCAGGTTGCCCATCTGGTGGGTA
TAGTCTTGGATGACGTTGATTTTGCCGGTGCGGTCGGAAACGCTGCCGCGCGGGTCGCCG
AAGAGGTGGTATTTGCCGCCGGGTTCGTAGTGCTGCCGTTGGGCGTTATCGGTAATGAAC
GGGTCTTGCGCCAAGTCCGCCGCGAGGGCGGGCTGTATGAGTGCGGCCGCCGCTACGGCG
CAGGCGGCAAGGAGGTTTGTCAGTCTGCGCAGCGGTTTCACGGTTTATCCTCCTTTGCGG
CGGCGGATGACTTCGTTGCCGACATCGGGTTTTTTACCGTTGTTTTGTTTGAAGTCGGGA

## Appendix A

CGGTTTTGGGCGGTTGTGTCGCCGTAGGGGGTAATGTCGGAGAAATCGACCATCAGGCGG
TCTGAGGCTTTGACGGTTTTGCTGACTTTGTAAGGGCCGGTCCAAAGGGCGTATTGTTCT
TGGTATTGGGATTCGTAGGCGGCGGTTTTAGGGGTAATCAGCAGTTTCCGGCTGTCGCGG
TCAACGGCGAAATATTCGAGCTTGGTTTGGGCTTTAAGGGTTTCGGCGTTGTAGAGGTGC
AGTTCGGTACGGCTGCGGACGGTGCCGAATACGTCGACGGTTACGAATACGTCGGTGTCG
GCGTATTCGGGCGGTACGACTTCGATGCCGCGCAGGTAGAAGACGGTTTGGATGAGGTTG
GTCAGGAAGGAAACGTCGCGGGGGTTGGCGAGCAGGGTTTCGTTGCGGTAGTCGCCCGTG
CCGTTGACGGACAGTCCGGCGGAGCGTTCGCCTTTGCGTCCGCTGTTTTTCGTCAGGGCG
GCGGCGGGGGCGTTCAAAAGCGATGTGGAAGTGGTTACGCTGGAGAGCGCGTCGGATTTG
GTGGTGGCGGTAGTGTCGTAGGCGGGGTAGCTGTATTGGGTGGCACTTTCGGGGTTGTTG
TGGTAGCCGCCGCGTATCAGTGCGTCGATAGAGTAGCGTCCGCCGCTTATGTTGCCCGAA
CCTTGGTCGCCCATAACGGAGACGTAAAGGGCGGCTTTGCGTCCTTTTAGGGCGGACAAA
TCCATTTCTTTGACGGCGGCGCGGGACGATGCGGCGACGAGTTCTTGTTCGACGGCAAAG
CGTTTGCCGCCGCCGTGGGCGGGTATGCCGGTCAGTGTGCCGCAGGCTGTGAGGACGAGG
GGGATGAGGAGGAGCAGGGTTTTCATAGCGGGGTTTGTTTGATGTTGAACGGATTTTGAG
TGTAAAGGGATTTTAAGGGTTTGTAAACAAAAGGGGCGAAAATGCCGTCTGAGCGGCGGA
AATGGCTTTCAGACGGCATTTGCGCTCAATAATAATATCCCGCGCCCAGAATACACGGTT
TGGATGCGCCGGTTGCTTTGTGCGGACTACCGGGAATGCGATTAATCCAACACGCCGCCA
ACCACGCAAATGCGGCGGCTTCCACCCATTGCGGATCGAGGTTCAGGTCGGCGGTGCTGT
GCAGGGAAACGCGTGTGCCGAAACATTCTGCCAAATCCGCCATTAAAACAGGATTGCGGA
TGCCGCCGCCGCAAATGTACATTTGACGGGCATCTGCCGCTGCGTGTGAGACGGCGTCGC
AAACGGTTTGCGCGGTAAAACGGGAAAGCGTCCGCAATACGTCGTATCGGTTTTCGCCGC
CGTCAAGGTAGGTTTCGAGCCAATTTAGGGCAAACAGTTCGCGCCCCGTGCTTTTAGGGT
GGGGTTGTGCGAAATACGGGTGGGCGAGCAGCCTGTCGAGCAGTTGCGGCAATATGTTGC
CTTGTGCCGCCTTTGCACCGTTTTTGTCGTAAGGAAGCTGCCAGTGTGCCTGCGTCCACG
CGTCCATCAGCATATTGCCCGGCCCTGTGTCGAAGCCGAAGGCGGGTGCGTCGGGGGGGA
GTACGCTGATGTTGGCAATCCCGCCGATGTTCAGTACCGCGCGTGTTTCCCTGTTGTCGC
GGAACAGGGCTTCGTGAAAGGCGGGGACGAGTGGCGCGCCTTGTCCGCCGGCCGCAAGGT
CGCGGCTGCGGAAGTCGCCGACGGTAAAAATCCGCGTCCGTTCCGCCAGCAGCGGCAAAT
CGGCAAGCTGTATGCTGTAACCGTGTTCCGGCGCGTGTCGGACGGTTTGCCCGTGGCAGC
CGAGGGCGGTAATGTCGGACGGTGCGAGGTTTTGACTGCACAGCAGTTCGGCGGCGGTTT
GCGCATATAGGCGGCTGAGTTCTTGCGACAAAATCCTGCTGCGGTGCAGTTCGTCTGCGC
CTGTGTCCTGCAAATCCAGCAATTGGCGGCGTAACCTGCCGGGGTAGGGGGTAAAGGCGT
GCCCTTCCGCGCCCAGCCATTTGCCGCCGTCCATCCGTATCAGTACGGCATCCGCCCCGT
CCATGCTGGTTCCCGACATGATGCCGATGTAAAGCTGTGTTTCCATCATCACTCCCAAAC
TGGTGCAAAACGCCATTTTAACGTGTATTGACGCTCGTATACCGATTTGCCGCCGCAGTG
TAAATAAAGTGTAAATAAATGTTTCAAGACGGATGGAAAAATATTATAATGCGCCCGCAA
CATCCAGTAGTAGAAGTGTCATACAAACCGTTTCCGGCAGCAGTTTTGCATTCGGTCAGG
TTTGGGGGTATTCGGATGCGGTTAGGAAGGATGCGTCTGCCATATCCCGAAACGGCAGTT
CGACCGGAGGCAGCAGTACAGTGTCGGCAACACTCATGATTTCCACCACATTAAAGGAAG
ATTGCCATGGCTCAAATCCAAATGAGCGCAAATGTTAAAACCATCAACGCCGTCTTTGCC
GCCATGCTGGTAGGTACAGTCGGCTATTTTATTTATTGGGGCTTGGGTTATACCCATTAC
AATTACGCCGCCTTATTCATTATTGCCACGATGTTCGGCGTGTTTATGGCGTTCAACATC
GGCGGCAACGATGTTGCCAATTCTTTCGGCACCAGCGTCGGTGCGGGTACGCTGACCATC
CCGCAGGCTTTGCTGATTGCGGCGGTATTTGAGGTCAGCGGCGCGGTCATCGCGGGCGGC
GAGGTAACCAATACCATACGCAAAGGCATCGTCGATTTGAAGGGTGTTGATTTCGAACCC
ATACAGTTTGTGTTTATTATGATGTCCGCGCTTTTGGCGGCGGCGTTGTGGCTGTTGTTT
GCCTCGAAAAAAGGGCTTCCGGTATCTACCACCCATTCCATTATCGGCGGCATTGTCGGC
AGCGCGGTATGTATGGCGGTAATGAACGATGCCGCATCGGGCGATTTGATACGTTGGGGC
~~AAGCTGGGCGGTATTGGTGTTTCTTGGGTATTGTCGCCCCGTGTTGGGCGGCGCGGTGTCC~~
TATTTTCTGTTTTCGCGCGTCAAGAAAAACGTCTTAGATTACAACGCTTGGGCGGAAGGC
ACGCTCAAGGGCATCAAGCAGGAAAAAAAGGCCTATAAAGAACGGCACCGCCTGTTTTTC
GAGGGTTTGTCCGAAGCCGAAAAAGTCGAGTACGCCACCAAAATGGCGCACGACGCGCAA
ATTTACGACGAACCCGAATTCGATCCGCAAGAGCTGCAATCGGAGTATTACCGCGGTCTT
TATGCGTTCGACAACCGTAAAAACAATGTCGATTCCTACAAGGCACTGCATTCTTGGATT
CCCTTTATCGCTTCGTTCGGCGCGATGATGATTTCCGCTATGCTGATTTTCAAGGGCTTG
AAAAACCTGCATTTGGGGATGAGCAACGTCAACAGCTTCCTGACCATCTTTATGATAGGC
GCGGCGGTGTGGATGGGGACGTTTGTTTTTGCCAAAAGCCTCAAGCGTAAAGACTTGGGC
AAATCGACCTTTCAGATGTTTTCATGGATGCAGGTCTTTACCGCCTGCGGCTTCGCATTC
AGCCACGGTGCGAACGATATCGCCAACGCCATCGGTCCGTTTGCCGCGATTATGGATGTT
TTGCGTACCAACAGCGTTGCCGCGCAAAATGTCGTCCCCCCGATTGCGATGCTGACTTTC
GGCATCGCGCTGATTGTCGGTTTGTGGTTTGTCGGTAAAGAGGTGATTAAAACCGTCGGT
ACGAGTTTGGCGGAAATGCATCCTGCTTCGGGTTTTACCGCCGAACTGTCCGCCGCCTCC
GTCGTGATGGGCGCGTCGCTGATGGGGCTGCCCGTGTCCAGTACGCATATCTTGGTCGGC
GCGGTACTCGGTATCGGTCTGGTCAACCGCAATGCCAACTGGAAACTGATGAAGCCCATC
GGTTTGGCGTGGGTCATTACCCTGCCTGCCGCCGCCGTATTGTCGGTTGTCTGCTACTTG
GTTTTACAGGCAGTATTCTGATTGTAAAATACTGATGCCGTCTGAACCCGTGTTCAGACG
GCATTTTGTTGATGGAATGTGCGGGCTTGTGCCTTATGCACAATCTGTTCTGTCGGGATA
TGCCGTTTGGTATAGTGATTAACAAAAATCAGGACAAGGCGACGAAGCCGCAGACAGTAC
AGCTAGTACGGCAAGGCGAGGCAACGCTGTACTGGTTTTTGTTAATCCACTATATCTTGG
TTTCGGAACGGTCGGACACAAAGGTGCGGAACGTTATGATATGCCGCCGCCTGTTCTTGA
AAACACTTATCCTGCCGGCAGCAAAATGCCGTCTGAAAAAGCCTTTCAGACGGCATTTGT
ACGTTAGCCACAATCACACTGTTTGCGAATATTTCGCCTTGGTTTCTTTATGGCGCAGGT
GGTAATCGAAGACCATGGCGATGTTGCGGATGAGGAAGCGTCCTTTCGGGGTAACGGTCA
GCCCGTGGCTGTTCAGGCGCACCAATCCCAAACCGGCGAGTTTTTCCAAATCCGCCAGTT

## Appendix A

CGTCTTTGAAGTAGCGGTCGAACGGGATGCCGAACATACTTTCGTAAATCCGATAGTCGA
GCGCGAAACGGCACATCAAATCCTGAATGATGTTGCGGCGCAGGATGTCGTCCTGATTGA
GCTGGTAGCCGCGCATGATGGGCAGTCTGCCTTCGTCGATGGCGGCATAGTAGGCATCGA
TGTCGCGTTCGTTTTGGGAATAGGTGCTGCCGATTTTGCCGATGGACGACACGCCGATGG
CGACCAAATCGCAATCCGCGTAGGTCGAATAGCCTTGGAAGTTGCGCTGGAGGAAGCCTT
CTTTGAGGGCGATGGAGAGTTCGTCGTCAGGTTTGGCGAAATGATCCATGCCGATGAAGA
CGTAGCCGCGTTCGGTTAGGGTTTGGACGCAGTATTGCAGCATATCGAGCTTCTCTTCGC
TGTCGGGAACGGCGGCGGTATCGATGCGGCGTTGCGGTTTGAACACGTGCGGCAGGTGGG
CGTAGTGATAAAGGGCGAGGCGGTCGGGATCGAGCGACAAAACGGTATCGATGGTGGTTT
TGATGCTTTCCGAAGTCTGGTGCGGCAGGCCGTAAATCAAATCGACGCTGACGGATTTGA
ACCCCGCTTCGCGCGCCGCATCGATGACTTCTTTGGTTTCTTCGTAACTTTGGATGCGGT
TGACCGCCGCCTGCACTTTGGGGTCGAAATCCTGAATGCCGATGCTCATGCGGTTGAAGC
CGAGTCTGCCGAGCATGAGGACGGTGTCGCGGCTGACTTTGCGCGGGTCGATTTCGATGG
AGTATTCGCCGGTGGGGATTAACTCGAAATGTTTGCGTATCATGCGGAAGACACGTTCGA
TCTGTTCGTCGCTCAAAAAGGTCGGCGTGCCGCCGCCGAAGTGCAGTTGGGCAAGCTGGT
GCCGTCCGTTCAGATGTGGAGCGAGCAGTTCCATTTCTTTTTCAAGATATTCGATGTAGG
CATCGGCGCGGCTTTTGTCTTTGGTGATGATTTTGTTGCAGCCGCAGTAGTAGCAGATGG
TGTTGCAGAACGGAATGTGAATGTAAAGGGAAAGCGGTTTGTTTAACGCGCCCATACCGC
GCAAATGTAAAGCTTTGATATATTCGCCTTCGCGGAAACCGTCATGGAAACGGTCGGCGG
TAGGGTAGGAAGTGTAGCGCGGGCCGCTGGCGGGCAGGCTGGCAATCAGCGCGCGGTCAA
ACTCGGGGCGGTCATCGTTTACATTGTGATTGTTCTGTATCTGAATGATTTTCATGGTGT
GTGTGTGCGGTTTTATGATGTTAGTCAAATTTTGGATAGTTTGGTAGAATGCCACAGTAT
GATAAACCTGTCTTGATATGTGTCAATAAGCACATATAGTGGATTAAATTTAAATAAGGA
CAAGGCGAGGCAACGCCGTACTGGTTTAAATTTAATCCACTATAATCATGATGGGGCAAA
GCGCACAAAAAGGTACGGTATGGCTTCGCATAATACTACACATCAGATGAAAACGCTGTG
TTCTTCCTGTTCTTTGCGGGAACTCTGCCTGCCTGTCGGGCTGCTGCCCAACGAGCTCAG
CCAACTCGATGCCGTCATCCGTCAAAGCCGCCGCCTGAAAAAGGGCGAATACCTGTTCTG
TGTCGGCGAAGCCTTTACCTCGCTCTTTGCCATCCGTTCGGGCTTCTTCAAAACAACCGT
CGCCAGTCAGGACGGCCGCGATCAGGTAACGGGTTTCTTTATGTCGGGCGAACTCATCGG
CATGGACGGCATCTGTTCCCATGTGCACAGTTGCGACGCGGTCGCCTTGGAAGACAGCGA
AGTGTGCGAACTGCCGTTTACCCACATCGAAGAACTGGGGCAAAACATCCCCAGCCTGCG
TACGCACTTCTTCCGCATGATGAGCCGTGAAATCGTGCGCGACCAAGGTGTTATGCTGCT
GTTGGGCAATATGCGCGCCGAAGAGCGGATTGCCGCCTTCCTGCTGAACCTTTCCCAACG
CCTTTATTCCCGAGGTTTTGCTGCCAACGACTTCATCTTAAGAATGTCCCGCGAAGAAAT
CGGCAGTTATCTCGGGCTGAAACTTGAAACCGTCAGCCGCACATTATCTAAATTTCATCA
GGAAGGATTGATTTCCGTCGAGCATAAGCACATCAAAATCCTCAATCTGCAGGTGTTGAA
AAAAATGGTGTCCGGCTGCTCGCACGCCATTTGATTAACCCGTACGAACATTTCAGACAG
CATTCTCAATAAACACAGGGCAGACGAAAACATCTGTCCTGTTTGTTGTATCTGCCGCAA
AGTGCCGTCTGAAAACCGGCAGCCGCCTAAATCGAAAAATCCTCGCTGATGGGCGTGTAC
AGAATCCTATCCACCTTCTCGCGTGTCAGGTGCGGCGCGAACGCTTGGATAAAGTCGTAG
GCATATCCGCGCAAATAAGTATCGCTGCGCAAAGCAATCCACGTCGGCGACGGCTCGAAC
AGGTGTGCCGCATCCACAAGCTGCAAATCGCCGTCCGTATCCGGGTTGTACGCCATTTTC
GCCATCAGTCCCACGCCCAAACCCAAGCGCACATAAGTCTTCAATACGTCCGTATCTGCC
GCAGCCAATGCGACATCGGGTTGTTCCAAACGGGCTTTGGAAAATGCCCGCGCGATGCTG
CTGCCCGCATTGAATGCAAATTCATAAGTAATCAGCGGAAACCTCGCCAAATCTTCAATA
CGGAGGGGGTTTCTGCATTCGAGCAAGGGGTGGTCGTTCGGTACGATAACCGCATGAGTC
CAGTCATAGCAGGGAAGTTTTCCCAGTTCGGGATGGTCGTCTATCCGTTCCGTAACAATC
GCCAAGTCCGCCTCGCCTGAGGTAACCATACGTGCGATGGCGGCAGGGCTCCCCTGTTTG
ATGGTCAGGTTGACTTTCGGATAGCGTTTCACAAAATCGGCAACAATCAAGGGTAGGGCA
TAGCGTGCCTGAGTATGCGTCGTGGCAACCGTCAGCGAACCGCTGTCCTGTCCGGTAAAC
TCGCTGCCGATATTTTTAATGTTCTGAACATCGCGCAAAATACGTTCCGCAATATCCAAA
ACCACCTTGCCCGGCTGCGAGACCGAAACCACGCGCTTGCCGCTGCGGATAAAAATCTGA
ATGCCGATTTCTTCTTCCAGCAATTTGATTTGTTTGGAGATGCCGGGTTGCGAAGTAAAC
AAGGCTTCGGCCGCTTCGGAAACGTTCAGGTTGTGCTGGTAAACTTCTAAGGCGTATTTC
AATTGTTGTAATTTCATGGCGGGTCGGTGTGGGTCTGTGTCGGGTGGCTGAACATTGTTT
ATAATTTATCATATTTTCTTGCCGGTACGGTATGGGGCTTTGCCGTTGTGTTTGTTGTTT
TTGTGCAACGGCAATCGTGCGATATGGAAAAAATCCCCCTAAAGTAATGACACGGAATTG
ATTTTTCGGCATGATAGACTATCAGGAAACAGGCTGTTTTACGGTTGTTTTCAGGCGTTG
AGTATTGACAGTCCGCCCCCTGCTTCTTTATAGTGGAGACTGAAATATCCGATTTGCCGC
CATGTTTCTACAGCGGCCTGTATGTTGGCAATTCAGCAGTTGCTTCTGTATCTGCTGTAC
AAATTTAATGAGGGAATAAAATGACCAAACAGCTGAAATTAAGCGCATTATTCGTTGCAT
TGCTCGCTTCCGGCACTGCTGTTGCGGGCGAGGCGTCCGTTCAGGGTTACACCGTAAGCG
GCCAGTCGAACGAAATCGTACGCAACAACTATGGCGAATGCTGGAAAAACGCCTACTTTG
ATAAAGCAAGCCAAGGTCGCGTAGAATGCGGCGATGCGGTTGCTGCCCCCGAACCCGAGC
CAGAACCCGAACCCGCACCCGCGCCTGTCGTCGTTGTGGAGCAGGCTCCGCAATATGTTG
ATGAAACCATTTCCCTGTCTGCCAAAACCCTGTTCGGTTTCGATAAGGATTCATTGCGCG
CCGAAGCTCAAGACAACCTGAAAGTATTGGCGCAACGCCTGAGTCGAACCAATGTCCAAT
CTGTCCGCGTCGAAGGCCATACCGACTTTATGGGTTCTGACAAATACAATCAGGCCCTGT
CCGAACGCCGCGCATACGTAGTGGCAAACAACCTGGTCAGCAACGGCGTACCTGTTTCTA
GAATTTCTGCTGTCGGCTTGGGCGAATCTCAAGCGCAAATGACTCAAGTTTGTGAAGCCG
AAGTTGCCAAACTGGGTGCGAAAGTCTCTAAAGCCAAAAAACGTGAGGCTCTGATTGCAT
GTATCGAACCTGACCGCCGTGTGGATGTGAAAATCCGCAGCATCGTAACCCGTCAGGTTG
TGCCGGCACACAATCATCACCAACACTAAGGCTAGGCAATATCTTGCCGATGCATGAGGT
TAGTGGATTTTGTACCAGGTACTGTTGCAATATTCGTGAAACGTCGGTCGGCATCGATGA
TGTGAAACAAACCCCCGCTTTTGCGGGGTTTGTTTTTTTGGGTGGTTTTCTGAAACGGCT

## Appendix A

ATCGTCAGAATCGGGGTGCAGGTTCGGATTCGGATTCAGATTCAGATTCAGATTCAGATT
CAGATTCAGGTTTGTGTCCCATTGCCGCGCTTTATAGTGGATTAACAAAAATCAGGACAA
GGCGACGAAGCCGCAGACAGTACAAATAGTACGGAACCGATTCACTTGGTGCTTCAGCAC
CTTAGAGAATCGTTCTCTTTGAGCTAAGGTGAGGCAACGCTGTACTGGTTTAAATTTAAT
CCACTATATCGGTTGAAACTCTGATTTTAAGGCGGTAGGATGTGGGTTTGCCCATAGAAA
GGGAATCCTTTCTGTATCAAGCCCTGAAAGGGATAATTCATACAAATTCACGCCTTTCCC
CCTCATTGGGAAATGGATGGAATCGTGCCAGATGTGTGCGGCACTGTATGCCGGATATGG
TTTTATCATCAGCCCTTTTCGGTTGAAACCCCGTCAGTTGCAGCGATTGAGCCTAATCGG
TGGCGGAAGTTGCCGCTTTGCATTCGGGGCGGCGTGCAGTGCGGTGCTTTGATATGCCGT
TTGTGTGTTGAAACAGGGTGGTCGGTGCATACGGGTACGGTATGGCCAAAGCTAAAAGTG
AAATACGCTGAAACACTGAATGAGCCGCTTTATTGTTTGTACGGCCTTTGCTGCCTTGCT
ATGATTTAAATTGGATTCGCCCGCCGGATATTTTGGGATATGAAAGAATTTGACTTCATC
AAACGGTATTTGCAAACAGGCACGGATAATGATGTCGTATTGGGCATAGGCGACGATGCG
GCGATTGTCCGCCCGCGTGAAGGCTTCGATTTGTGTTTCAGTGCGGATATGCTTTTGAAG
GACAGGCATTTTTTTGCAGATGTCAAACCTGAAGACTTGGCTTGGAAGGTTTTGGCCGTC
AATATTTCAGATATGGCGGCGATGGGTGCGATACCGCGTTGGGTGTTGCTGAGCGCGGCT
TTGCCCGAATTGGATGAGGTATGGCTGAAACGGTTTTGCGGCAGCTTTTTCGGTTTGGCA
AAAAAGTTTGGCGTAACGTTAATCGGCGGCGATACGACCAAGGGCGATATGGCGTTCAAT
GTAACCATTATCGGCGAATTGCCGAAGGGTAGGGCGTTGCGGCGTGATGCGGCGGTTGCG
GGCGACGATATTTGGGTGTCGGGGCGTATCGGTATGGCGGCGGCGGCTTTGAACTGCCGT
CTGAAACGGTGTGTGTTGCCAGATGAAGTGTTTGCCGAATGCGAACAAAAGCTGCTCCAT
CCTGAACCAAGGGTTGGGCTGGGGCTTGCGCTGTTGCCGTTTGCCAGGGCGGCGCAGGAT
GTTTCAGACGGCCTCGCGCAAGATTTGGGGCATATCCTGACCGCTTCTGGCAAGGGTGCG
GAAATTTGGGCCGATTCGCTGCCGTCTTTATCCGTATTGAAAGATATTTTGCCCCGAGCG
CAATGGCTGTCTTATACTTTGGCGGGCGGCGACGATTACGAGCTGGTGTTTACCGCGCCG
GAAAGTTGCCGCAGCCGCGTATTTGATGCGGCGGAACGGTGCGGCGTGCCGGTAACGCGC
ATCGGCAAAATCAACGGAGGATGCCGTCTGAAGGTTTTAGATGCCGACGGCAGGGAATTG
GAACTACATTCTTTAGGATTCGATCATTTTGGCTGATTTTAAACCTGACTTTGCGTGGCT
GTTGAAACGGCCGTTGTGTTTTTTGGCTTTCGGTTTCGGCAGCGGGCTGGCTCCGTTCGC
GCCGGGCACATTCGGCACTTTGGCGGCACTGCCTTTGGCGTTTGTGCTGATTTTGCTCGG
CATAGACGGGCTACTGCTGGCTTTTTTGTGTATCGTGCTGTTTATGTGGGGCATACGCAT
TTGCGCTTATGCGGAACGTGAAACGGGTGTCAGCGACCACGGTGGGATTGTTTGGGACGA
GATTGTCGCCATGCTGTTTGTGCTGGCGTTTGTGCCGTTCAGGTGGACGTGGTGGCTGGC
GGCATTTGTCCTATTCCGTCTGTTTGACGCGCTCAAACCGTCTCCCGTCGGTTGGTTTGA
CAAGAATCTGCACGGCGGTTTGGGCATTATGGCGGACGATATGGCGGCTGCGGTGATGAC
TTTGATTGTCTTGAGGATTGCAATGCTGTTTTAAACGGTGCTGCCTTGTAAAAATGCCGC
CTGAAAGCCTTTCAGACGGCATTGTTTCGGAGGTTAACGCGTTACCGGTTTGTATTTGAT
GCGTTTCGGTTTCGCGCCTTCTTCGCCCAAACGGCGTTTCTTGTCGGCTTCGTATTCCTG
ATAGTTGCCGTCGAAGAACACCCATTTAGAGTCGCCTTCACACGCCAAGATATGCGTGGC
GATGCGGTCGAGGAACCAACGGTCGTGCGAAATCACCATCACGCTGCCGGCAAATTCCAA
CAATGCGTCTTCCAACGCGCGCAGGGTTTCCACGTCAAGGTCGTTAGACGGTTCATCCAG
CAGCAATACATTGCCGCCGCTCAACAAGGTTTTTGCCAAGTGCAGACGACCGCGTTCGCC
GCCAGACAATTGACCTGCAATTTTGCTTTGGTCGCTGCCTTTGAAGTTGAAACGCCCCAA
ATATTGGCGGGCGGGAATTTCAAACTGACCAACCTGCAAAATGTCGCGGCCTTCGGCAAT
GTTGTCGAACACGGTTTTGTCGTTTTGCAAACCTTCGCGGCTTTGGTCAATCAAGCTCAT
TTTCACGGTTTGTCCGATTTTCACCTCGCCGGAATCAGGCTGCTCTTTGCCCGAAATCAT
TTTGAACAGCGTAGATTTACCCGCGCCGTTCGGGCCGATGATGCCGACAATCGCGCCCGC
AGGCACTTTGAAGCTCAAATCGTCAATCAGCACTTTATCGCCGAACGATTTGGAAACATT
TACAAATTCAATCACTTCGTTACCCAAACGCTCGGCAACGGGAATAAAGATTTCCTGCGT
TTCATTGCGTTTTTGGTATTCGTAGTTGCTCATTTCTTCAAAACGAGCCAAACGCGCTTT
GGACTTGGCTTGGCGGCCTTTGGCATTTTGGCGCACCCATTCCAATTCCTGCTTCATCGC
CTTCACGCGCGCGGCTTCGGATTTTGCCTCGTTTTCCAAGCGTTTTTCTTTCTGCTCCAG
CCAAGACGAGTAATTGCCTTTCCACGGAATACCATGGCCGCGGTCGAGTTCCAAAATCCA
TTCGGCGGCGTTGTCGAGGAAGTAGCGGTCGTGCGTTACCGCAACGACTGTGCCGGGGAA
GCGCACGAGAAATTGCTCCAGCCACTCGACCGATTCCGCATCCAAGTGGTTGGTCGGCTC
GTCCAGCAAAAGCATATCGGGCTTGCTCAACAAGAGTTTGCACAAGGCAACGCGGCGTTT
TTCACCGCCGGACAAATTATCGATTTTGGCATCCCATTCCGGCAGGCGCAGCGCGTCGGC
GGCGATTTCCAATTCGTGTTCCGCACCGCCGCCCGTGGACGAACCTGCCGCAATAATCGC
TTCCAAGCGGCCCTGCTCTTCTGCCAACGCGTCAAAATCCGCATCAGGATTGGCGTACTC
GGCATACACTTCTTCCAAACGTTTCTGCGCGGCAGCCACTTCGCCCAAACCGCTTTCCAC
TTCCTCGCGCACGGTTTTTTCCGGATCAAGCTCAGGCTCTTGCGGCAGGTAGCCGATTTT
GATGCCGCCCATCGGCACGGCTTCGCCCTCAAATTCCTTATCCACGCCCGCCATAATCCG
CAGCACGGTGGACTTGCCCGCGCCGTTCAAACCGAGCAGGCCGATTTTCGCGCCGGGGAA
GAAAGAAAGGGAAATATCTTTAATGATGGTTTTCTGCGGCGGCACAACCTTGCTCACGCG
CAGCATAGAATAGACGTATTGTTGGGACATGGTTTTCTCGTTTTCATCAAACAAATTTCA
GACGGCCATTTTAACCGATAATTTGATTTAAGCCAGTTTATCCGCGAACCGGTATTGCCA
AAATCGGGCAGGATTCATAAAATCCGCTTATCCCTTTGAAATTATATAGACAAAAAAATA
ATAATGATAGGGGATCGCCGCCCCGGCAACCATTTCGGATTTTCCAAAGCAAATATAGTG
GATTAACAAAAATCAGGACAAGGCGACGAAGCCGCAGACAGTACAGATAGTACGGAACCG
ATTCACTTGGTGCTTCAGCACCTTAGAGAATCGTTCTCTTTGAGCTAAGGCGAGGCAACG
CCGTACTGGTTTTTGTTAATCTACTATACTTTTCAAATCAAAAAAGGATTTACCTTATGT
CGGAATATACGCCTCAAACAGCAAAACAAGGTTTGCCCGCGCTGGCAAAAAGCACGATTT
GGATGCTCAGTTTCGGCTTTCTCGGCGTTCAGACGGCCTTTACCCTGCAAAGCTCGCAAA
TGAGCCGCATTTTTCAAACGCTAGGCGCAGACCCGCACAATTTGGGCTGGTTTTTCATCC
TGCCGCCGCTGGCGGGGATGCTGGTGCAGCCGATTGTCGGCCATTACTCCGACCGCACTT

## Appendix A

GGAAGCCGCGTTTGGGCGGCCGCCGTCTGCCGTATCTGCTTTATGGCACGCTGATTGCGG
TTATTGTGATGATTTTGATGCCGAACTCGGGCAGCTTCGGTTTCGGCTATGCGTCGCTGG
CGGCTTTGTCGTTCGGCGCGCTGATGATTGCGCTGTTAGACGTGTCGTCAAATATGGCGA
TGCAGCCGTTTAAGATGATGGTCGGCGACATGGTCAACGAGGAGCAGAAAGGCTACGCCT
ACGGGATTCAAAGTTTCTTAGCAAATACGGGCGCGGTCGTGGCGGCGATTCTGCCGTTTG
TGTTTGCGTATATCGGTTTGGCGAACACCGCCGAGAAAGGCGTTGTGCCGCAGACCGTGG
TCGTGGCGTTTTATGTGGGTGCGGCGTTGCTGGTGATTACCAGCGCGTTCACGATTTTCA
AAGTGAAGGAATACGATCCGGAAACCTACGCCCGTTACCACGGCATCGATGTCGCCGCGA
ATCAGGAAAAAGCCAACTGGATCGAACTCTTGAAAACCGCGCCTAAGGCGTTTTGGACGG
TTACTTTGGTGCAATTCTTCTGCTGGTTCGCCTTCCAATATATGTGGACTTACTCGGCAG
GCGCGATTGCGGAAAACGTCTGGCACACCACCGATGCGTCTTCCGTAGGTTATCAGGAGG
CGGGTAACTGGTACGGCGTTTTGGCGGCGGTGCAGTCGGTTGCGGCGGTGATTTGTTCGT
TTGTATTGGCGAAAGTGCCGAATAAATACCATAAGGCGGGTTATTTCGGCTGTTTGGCTT
TGGGCGCGCTCGGCTTTTTCTCCGTTTTCTTCATCGGCAACCAATACGCGCTGGTGTTGT
CTTATACCTTAATCGGCATCGCTTGGGCGGGCATTATCACTTATCCGCTGACGATTGTGA
CCAACGCCTTGTCGGGCAAGCATATGGGCACTTACTTGGGCTTGTTTAACGGCTCTATCT
GTATGCCTCAAATCGTCGCTTCGCTGTTGAGTTTCGTGCTTTTCCCTATGCTGGGCGGCT
TGCAGGCCACTATGTTCTTGGTAGGGGGCGTCGTCCTGCTGCTGGGCGCGTTTTCCGTGT
TCCTGATTAAAGAAACACACGGCGGGGTTTGAGCGATGAGCGATACCCCCGCTACCCGCG
ATTTCGGTCTGATCGACGGGCGTGCCGTAACCGGCTATGTGCTGTCCAACCGGCGTGGTA
CGCGTGTCTGCGTGCTGGACTTGGGCGGGATTGTGCAGGAATTTTCCGTTTTGGCAGACG
GCGTGCGCGAAAACCTCGTGGTGTCGTTCGATGATGCGGCTTCCTATGCGGACAATCCGT
TTCAGATTAACAAACAGATAGGGCGCGTGGCCGGACGCATCCGCGGTGCGGCGTTCGACA
TCAACGGCAGGACTTACCGCGTGGAGGCCAACGAAGGCAGGAACGCGCTGCACGGCGGTT
CGCACGGGCTGGCCGTTACCCGTTTCAACGCGGTGGCGGCAGACGGCCGTTCGGTGGTGC
TGCGCAGCCGCCTGCAACAGTCGGCCGACGGTTATCCCAACGATTTGGATTTGGATATTT
CCTACCGCTTGGACGAGGACGACCGGCTTACCGTTAGCTATCGCGCCACCGCGCTCGGCG
ACACGGTGTTCGACCCGACGCTGCACATTTACTGGCGGCTGGACGCGGGCCTGCACGATG
CGGTTCTGCATATTCCGCAGGGCGGACATATGCCGGCCGATGCCGAAAAACTGCCCGTCT
CAACGGTTTCAGACGACCTCGAAGTATTTGATTTCAGCCGGCCCAAGCCGCTGGATGCCG
CCGTTGCCGCCCTGCGCCGCGAAACGGGTCGGGCCGGTTTTGACGACGCTTACCGCGTGC
CGTCCGATATAGGCCGTCCCGCCGCTGTGTTGCAAGCCGGACGCCGCCGTCGTATCAGCA
TATACAGCGACCGCAATGGCTTGGTCATCTTTACCGCCGCCCCGCAGGATTTCGCGCGGC
ACGATGCGGGCGTTTACGACGCGCTGGCGACCGAGGCGCAGACGCTGCCCGACAGCCTGA
ATTGGCCCGAGTTCGGCAATATTCGTCTGAACAAGGGTGATACCAGGGAGGCGACGATTG
CTTACGGCATCGAATCCCTTTCTTAGGAGCTTCCTAACACCGGTTGCAGACGACCTTTTT
ATAGTGGATTAACAAAAACCGGTACGGCGTTGCCTCGGCTTAGCTCAAAGAGAACGATTC
TCTAAGGTGCTGAAGCACCAAGTGAATCGGTTCCGTACTATTTGTACTGTCTGCGGCTTC
GTCGCCTTGTCCTGATTTTTGTTAATCCACTATAAGATTTCACCATTCCCTCAAATCAAT
CCAAACAGGAGCTTCATAAATGTACACAAGAATCATGGAAATCAGCCCTTGGACGCTGCG
TTCGGCAAAACTGGAAAAAGAACACAAACGGCTGCAAGAGAGCCTGACCAGCTTGGGCAA
CGGCTATATGGGTATGCGCGGCAGCTTTGAGGAAACCTATTCCGCCGACAGCCACTTAGG
CACCTACATCGCCGGCGTGTGGTTCCCCGACAAAACCCGCGTCGGCTGGTGGAAAAACGG
CTATCCCAAATATTTCGGCAAAGCCATCAACGCGTTCAATTTCAGCAAAGTCAAAATCTT
TGTCGACGGGCAGGAAGTGGACTTGGCGAAAAACGACGTTGCTGGCTTCTCCGTCGAACT
CGATATGCAGCACGGCGTGTTGCGCCGCTCGTTCACCGTATTCGGTGTGCGTTTCAATGT
GTGCAAATTCCTGTCTGTCGCACAAAAAGAGCTGGCGGTCATCCGCTGGGAAGCCGTATC
CGTTGACGGTAAAACCCACCAAGTCCGCATCGATTCCATCATCGATGCCGACGTGAAAAA
CGAAGACTCCAACTACGAAGAAAAATTCTGGCAGGTATTGGACAAAGGCGTTTCAGACAG
TCTCTCCTACATTGCCGCCCAAACCGTCGCCAATCCCTTCGGCGTGGAACAATTCATCGT
CAACGCCGAGCAAACCTTTGCCGGCAGCTTCAAAGCCCTCGGCGGCAGCCAAACCGACTG
GCAGGTCTCCAATTCTTTTGAATCCGAAGTCGGCAGCACACCCGAAACCTTTGAAAAACG
CGTGATTGTTACCACCAGCCGCGATTATCAGAGCTTGGAAGCAGTGAAAGCCGCAGGCCG
CGCCTTGTCGGAAAAAATTGCAGGCGTTGCGTTTGAAACCTTGCTGGACGCGCACAAAGC
AGGCTGGCTGCACCGTTGGGAAATCGCCGACGTGGTCATCGAAGGCAGCGACGAAGCGCA
GCAGGGCATCCGCTTCAACCTGTTCCAACTGTTCTCCACCTACTACGGCGAAGACGCGCG
ACTGAACATCGGCCCGAAAGGCTTTACCGGCGAAAAATACGGCGGCGCGACCTATTGGGA
CACCGAAGCCTACGCCGTACCGCTCTACCTCGCACTGGCCGAACCCGAAGTTACCCGCAA
CCTGCTGCAATACCGCCGCAACCAACTGCCGCAGGCGCAGCACAACGCGCGCGAACAGGG
CTTGGCGGGCGCACTCTATCCGATGGTAACGTTTACGGGCATCGAGTGCCACAACGAATG
GGAAATCACCTTCGAGGAAATCCACCGCAACGGCGCGATTCCTTACGCCATCTACAACTA
CACCAACTACACCGGCGACGAGGGCTATCTTGCCAAAGAAGGCTTGGAAGTTTTGGTCGA
AGTGTCCCGCTTCTGGGCGGACCGCGTCCACTTCTCCAAACGCAACGGCAAATACATGAT
TCACGGCGTAACCGGTCCGAACGAATACGAAAACAACATCAACAACAACTGGTACACCAA
CACCCTCGCCGCATGGGTATTGGACTACACCCGCGAAGCCTTGGCGAAATACCCGCGTCC
GGATTTGAACGTGCGTGCCGACGAGTTGGAAAAATGGGCGGACATCAGCGCGAATATGTA
CCGTCCGCATGACGAAGAACTCGGCGTATTCGTGCAGCACGACGGCTTCCTCGACAAAGA
CATCCGCCCCGTGTCCGCGCTTTCGCCCGACGATTTGCCGCTCAACCAAAAATGGTCGTG
GGACAAAATCCTGCGTTCGCCCTTTATCAAACAGGCGGACGTATTGCAAGGCATCTACTT
CTTCAGCGACCGTTTCAATATCGACGAAAAACGCCGCAACTTCGACTTCTACGAACCGAT
GACCGTGCATGAAAGCTCGCTGTCGCCCTGTATTCACTCTATTCTCGCCGCCGAACTGGG
CAAAGAAGAAAAAGCCGTGGAAATGTACCAGCGCACCGCCCGCCTGGACTTGGACAACTA
CAACAACGACACCGAAGACGGCCTGCACATCACCTCCATGACCGGCTCGTGGCTCGCCAT
CGTCCAAGGTTTCGCCCAAATGAAAACCTGGGGCGGCAAACTCAGCTTCGCACCGTTCCT
GCCGAGTGCGTGGACAGGCTACGCCTTCCACATCAACTACCGCGGCCGTCTGATTAAAGT

## Appendix A

CGCCGTCGGCAAAGAAAACGTCGTCTTCACTCTGCTCAAAGGCGAGTCGCTCGATTTGCA
GGTGTACGGCAAAGACATCACGCTCGACGGCAGCCACACCGTTGCGTTGGAAAAATAAGG
AGGGCGCAAAATGACTTTCACTGCAGTCCTATTTGACCTCGACGGCGTCATCACCGACAC
CGCCGAATACCACTACCGCGCATGGAAAAAGCTCGCCGAAGAACTGGGCATCAGCATTGA
CCGCAAGTTTAACGAGCAGCTCAAAGGCGTGTCGCGCGACGATTCGCTCAAACGCATCCT
CGCGCACGGCGGCAAAACCGTCAGCGAAGCCGAGTTCGCCGAACTGACCCGCCGTAAAAA
CGACAACTACGTCGAGATGATTCAGGCAGTCAAACCCGAAGACGTGTATCCCGGCATTTT
GCCCCTGCTGGAAGCATTGAGGGCAAACGGCAAAAAAATCGCCCTTGCGTCCGCCAGTAA
AAACGGCCCGTTCCTGCTGGAACGCATGGGGCTGACCCACTTCTTCGACGCCATTGCCGA
CCCTGCCGCCGTCGCACATTCCAAACCCGCCCCCGACATCTTCCTCGCAGCAGCCGAGGG
CGTAGATGCGGACATCCGCCAATGCATCGGCATTGAAGACGCCGCCGCCGGCGTCGCCGC
CATCAAAGCCGCCGGCGCCTTGCCCATCGGCGTGGGCAAAGCCGAAGACTTGGGCAGCGA
CATCGCGCTGGTCTCCGGCACCGCCGAGCTGACCTACGCCTACCTGCAAAGCGTGTGGGA
ACAGTCGGGCAGGTAAAACGCGTCAGATAAAGTGTCAAGGAAGCAAAAGACCGTCTGAAC
AGTGTTTCAGACGGCCTTTTTGCTTTTAGAACAGAATGATAACCCAACTTACGCAACCCT
AAAAACTAAATGCCAATCTCTTAACCATGCTATTCAAATTTATTTGAACGATTTTTTTC
TAACCAGCCAACCTTAACAATCACTATTAAAATGCGCGCCGATGTTCTGTCTCCGCCTGT
ATGCGGCTTGGGCGACGGCGAGGCTGCATTCGAGCAGGTTGCGGTTTTCGTATTCGGACG
CGGTGTGCGGTTCGGCTTGGTTTTGCTTCCAAAGCTGCAGTTGGGCGATGGCGCGGCGCA
GGCCGGTATCGTTGCGTAGGATGCCTAGATGGCGTTGGTTGAACGTTTGCAGGACGGGGC
GGCTGAATGTGTTTTGAAGGTCGTCTGAAAAGATGCCTGCTTCGGCGGAGAGGCTTTCAG
ACGGCCTTTGGAATGGTTCGGCTTGGAATGCTTGTCCGTCTGCGATGGCTTGGGCGCAGA
GCCTTGCGGTCACGACGCATTCGAGCAGGGAGTTGCTGGCAAGGCGGTTGGCTCCGTGCA
GCCCAGTGCAGGCGGTTTCGCCCAAGGCGTAGAGCTGCGGCAGGGAGGTTCTGCCGCAGG
GGTCGGTTTGGATGCCGCCGCAGGTGTAGTGTTGCACGGGGCGGACGGGGATGGCTTGGC
GCGTGATGTCTAGGCCGCATTGGGATAAACAGTGTCGATGGATGGATGGGAAATGCCGGC
GGACGAACGCTGCGGGTTGATGGCTGATGTCGAGCGAGACGAAGTCTTGCGTTTGTTTGG
CGATTTCGGCTGCGATGGCGCGGGCAACGATGTCGCGCGGTGCGAGTTCGGCGCGGCGGT
CGTAATGCGGCATAAATCGTTCGCCCGCTTGGTTGGTCAGGATGCCGCCTTCGCCGCGCA
CGGCTTCGGAAATGAGGAAGGTGCGTCCGTTTTCAGACGGTCTTGCCAAGCCTGTGGGGT
GGAATTGGATAAATTCGAGGTTTCCAACTGCGCAGCCTGCGCGTATCGCCATGGCGATGG
CGTCGCCCGTGCATTCGGGCGGCGTGGTGGTGGCGGCGTAAATCTGTCCCAAGCCGCCGC
CTGCGAGTACGGTATGGCGGGCGCGGATGCGGTAGGTTTCTTGTGTTCGGCAGTCGAGGA
CGGTCAGTCCGCACGCCGCGCCTGATTCGGTTTGAATGTCCAACGCCATCTGCCGCTCGC
AAACGCGGATGTTCGGGCGGCGGCGTATTTGGGCAATCAGGCTCTGCATGACGGCTTCGC
CCGTGTAGTCGGCGACGTGGGCGATTCGTCGGCAGGTATGCCCGCCTTCACGCGTCAGGT
GCAGGCCGTTATGATTCCGGTCGAACGCCACGCCCTGCGCCAGCAGCCATTCGATTGCCG
GTTTGCCCTGCGACAGGATGGCGCGGACGGCGGCTTCATCACACAAACCCGCGCCCGCTT
CCAAAGTATCGGCAACGTGTTTTTCGATGTCGTCCTCTCCCGACCACGCCGCCGCAATCC
CGCCTTGCGCATGACGGCTGGCGGTGTCGTCCAGCCGGTTTTTGCACAAAATAACGATGC
GGAACGATTCAGGCAGCGACAGGGCGAGCGTCAGTGCCGCCAGCCCGTTTCCGGCAATCA
ATACGTCGCAATCGGTTTGCATGGTGTTGTCCTTGTTTGAGAGGCCGTCTGAAACGGTAT
AGTGGATTAATCAATGCCCCGACATATGCGACATGGTATTGAGAAGCACCACGCCCAGCA
AAATCAAACCGATGCTGACAATCCCAATGAAATCAGCTTTCTCACCGAAAAACACCACGC
TGACTAAAGCCGTTAAAACCAGTCCCACGCCTGCCCAAATGGCGTATGCTGTAGCCAGCG
GCATGGTTTTCAGTGTCATAGACAAGGCCCAAAAACACACCGAAAAGCTGACTACCACGC
CAATAGAAGGCCACAGTTTGCTAAACCCGCCACTCAGTTTGAGCATGGAAGAACCGCAGA
CTTCGCTTAAAATTGCTACAGTCAGAAAGAGCCAGTGCATTTGCATGTTTTTACCTGATA
AATGAAAGAAAGTATAATTATATCAATGCAATAAAATAAAAAAACAGTCTTGTTGTTAAA
~~GATTTTTTGTGTGCAAATCCCGTCTTGGGAAAGCAGGCGGGCGGTATTTTCAGGCTGCAC~~
CCATTACGAACGACAAATCAGGCGGGGCCCATGCCGTTGAACACATCTTTTTTCTTCAGC
CCTGCCGCAAAGTCGAGCATACGCTGCAAAGGCAGTTTGGCGGCTTCGCCCAGCTTCCTG
TCCAACAGGATTTCGTTACGTCCGCTTGTCAGGGCGTATTTGATGCCGCCCAGCGAATTC
ATCGCCATCCACGGGCAGAACGCGCAGCTTTTACAGCTTCCACCGTTGCCCGCCGTCGGC
GCGGCGATAAATTGTTTGTCGGGCGCCTGCTTTTGCATTTCGTGCAGGATGCCCAAATCG
GTCGCCACGATGAATTTTTTTTCAGGACGCGATACGGCGGCTTTGAGCAGTTTGCTGGTC
GAGCCGACCACGTCGCCCAGTTCGATGACGCTTTGCGGCGATTCAGGATGAACCAGCACC
ACCGCTTCGGGGTGTTCCGCCTTCAACGCCGCCAGCTCTTGCCCTTTGAATTCGTTGTGA
ACGATGCACGAACCCTGCCACAACAGCATATCCGCGCCCGTTTCGCGGCAGATGTAGTCG
CCGAGGTGGCGGTCGGGTCCCCAAATCAGCTTCTCGCCGCGTGATTTCAAATACGATACG
ATTTCTAACGCCACCGAAGACGTTACCACCCAATCGGCACGCGCTTTCACGGCGGCGGAA
GTGTTGGCGTACACCACCACCGTGCGGTCGGGGTGTTGGTCGCAAAACGCTGAAAACGCT
TCTTCCGGGCAACCCAAATCCAAAGAACATTCCGCCTCCAAATCAGGCATCAGCACCGTT
TTTTCAGGGCAGAGGATTTTCGCGCTCTCGCCCATGAAGCGCACACCAGCCACCACCAGC
GTACCGGCTTCGTGTTCCGCACCGAAGCGCGCCATTTCCAGCGAATCGCCCACGCATCCG
CCCGTCTCCAAAGCCAAATCCTGAATCAGCGGATCAACGTAATAATGCGCCACCAAGACC
GCGTTTTTCTCCTTCAGCAAAGCCTTGATTTCGTCTTTCAGACGATCTGCCGTCTCGCGG
TCGGGCGTGTCGGCAACCTTCGCCCACGCCTGACGGATTTGGCAGGCGGAAGTCGGCGTT
TGGATGAGTGGCATATCGTAGTCGAACGAGCGGCGGGCGGCGGTTTGCATGATGTTTCCT
TGTAGCTGTTTTTCAGACGGCATGAAGGTTTGCCGTCTGTTTTTCAAACTGTTTTTACAT
TATGCTCAACTTGAGTATAATATGCAAGGTCGTCTGAAAACAGGTTTGCAATACCGTAAA
ACCGACCCGCTTCGTTCCGACAAACCGCTTTGGTTTACAATAAAGCCTTTCCCACCCGCA
GAAAGCCGAGCATGGATGCCTACCCCGAAGCCGAAGCCCCGCCGCAAAGCATCGTCGAGC
TGGTTCCCGTATTGATTGCCGTTACCGACGGCGGCCTGCGGGTATTGACCGTCGCCCAAG
GCATGCTCCTGCCCAACGGCCCGCTCTCCCCCCTGCGCAATTCCTTGCAGGCAGGCGTAA

## Appendix A

AACTGTGGGTCGCCAAGCAGACTTCGCAGCCTATGGGCTATGTGGAACAGCTTTACACCT
TTGTCGATACCCACCGCCGCAACGAACACGGCATGCCCGTGCTGTACGTCAGCTATTTGG
GGCTGGTGCGCGAGGCAGCCGACAGCATCCTGCACCCGGATGCGAAATGGCAGGACTGCT
ACGGCTATTTCCCGTGGGAAGACTTGCGCACCGACGGCGGGCAGCGCGACGCCGTCGTCG
GCCGCCTGCGCATTTGGGCAAACTCGGCGGACACGGAGGAAGTGCGCCAAAAGCGGCTCA
AGCGCATTCATTTGTGCTGGGGGGTCGAACCGGAAAACTGGTCGGAAGAATACGTTTTGC
AACGCTATGAAATGCTGTATGAAAGCGGCCTGATAGCGGAAGCCGCCGAGCCGCAGGCAA
ACTTCGACTTCGCGCTTACGGGGCAGCCCATGCGCCACGACCACCGCCGCGTACTGGCGA
CCGCCCTGTCTCGCCTGCGCGCCAAAATCAAATACCGCCCCGTGATTTTTGAACTGATGC
CGCCCGAATTCACGCTGCTGCAACTGCAAAACAGCGTCGAAGCCATCAGCGGCAGATTGC
TGCACAAGCAAAACTTCCGCCGCCAGATTCAGCAGCAAAACCTCATCGAGCCGTCGGATA
CCGGCGTATCGGGCAGCAAAGGCCGTCCCGCGCAGCTTTGCCGCTTCCGCGACGACGTCC
TGCCCGACAGGCTGATTTCGGACATCGGACTGCCGCTGGGCAGCCGTTAGCCCGTTTTCA
GACGACCTATAGTGGATTAACAAAAATCAGGACAAGGCGACGAAGCCGCAGACAGTACAA
ATAGTACGGAACCGATTCACTTGGTGCTTGAGCACCTTAGAGAATCGTTCTCTTTGAGCT
AAGGCGAGGCAACGCCGTACCGGTTTTTGTAAAATGAAGTTTTGCCCCATCGGTGCAACA
TCAATCTTTTTCAACAAAGGAAACCCCATGCCGTCTGAAAAAACCCTCTTTCCCCTGCCC
GACACCCTGTTGCGCCCCATAGTAGAACAAGCCTTGAGCGAAGACTTGGGCAGGCGCGGC
GATATTACGTCCGCCGCCGTCATCGCCCCCGACAAAACCGCCAAACTCTTCCTTGTCAGC
CGCGAAGACGGCGTTATCGCCGGCATGGACTTGGCGCGTCTCGCCTTTCAGACGATGGAT
CCGTCCGTCCGCTTCCAAGCCGAAATCCGAGACGGGCAAGCCGTCCGCGCAGGTCAGACG
CTTGCCGCCGTCGAAGGCAACGCCCGCGCGCTGCTCGCCGCCGAACGCACCGCGCTCAAC
TACCTCACGCACTTAAGCGGCATCGCCACCGCCACCGCGCGTGCCGTTGCCGAAGTCGCC
GAATACGGTACAGACATCGTGTGCAGCCGCAAAACCATCCCCCTGCTGCGTGTCCTGCAA
AAATACGCCGTCAGGGCAGGCGGCGGTGTGAACCACCGCATGGGTTTGGACGACGCCGTG
CTCATCAAAGACAACCACCTCGCCTATTGCGGCAGCATCGCCCAAGCCGTGCAGCAGGCA
AAACAGGCTGTCGGAGCATTGACCTGCGTGGAAATCGAAGTGGATACGTTGGCACAACTG
GACGAAGCCATCGCAGCGGGCGCGGAACGGATTTTGCTGGATAACATGGACGACGAAACC
CTGAAAGAAGCGGCAAACCGCTGCCACACGCAAACCGCCCACCCCCACACCATCTATTGC
GAAGCATCGGGCGGCATCGGCTTCGACCGCCTGAAGCGCGTGGCGCAAACCGGAGTGGAC
GGCATCGCCCTCGGCTATCTGACCCACAGCAGCCGTTCGTTGGACATAGGTTTGGATTTC
GTGGCGTGAGTTTTAGGGTGCGGGCGGCTGTCTGATATGTCAGGCAAGGAACCGCTTAAC
CCTAATCCGGTTATTGCCTCAGGGAGGAAATGCCGTCTGAAAGATTCTTCAGACGGCATT
TTTCGTAAAGGTCGTGATGCTTTAGAAAAAACAGCATTTCAGGCAGGTATTTTGTTTGCC
CGACAGCGCGGCGGCATCGGTAGGGCAGGAAAAAGGACGGGGGGCGGCAGTTTTATGCCG
TCTGAAAGCCCGCCTTTACGCTTGTTTGCAAAAAAAGTGGAAAAGGAACATACAATCCT
GTACAATCATCCATAAATATTTGATTTATAATACGATTTATAAAGATAATCACAATCATC
CATATCTGCCGCCCGTCAATCCGCTTGGCGGGCGGCAAAGGTTTTAGGAATACCGATGAA
CACAATACCGCTCCACACCATACTCAAACTTATGGCGCATCCCGAACGTATGGCGATACT
GATTCAATTGTTGGACAGCGAACGCAATATCGCCGAACTGGCAAAATCCTTATCCCTGCC
GGCCACCGCAGTTTCCAACCATTTGAACCGCCTGCGCGTGGAAGGTCTAGTCGATTTTAC
GCGTTACCACCGCATTATCGAATACCGCCTGGTTTCCGAAGAAGCGGCGGCGATTCTGCA
CACGGTTCGCGATTTGGAAAACAAACGCGTGGCATAGTGTTAGAATCCTTTCCTTTTGCC
GTCTGAACGTTTCAGACAGCATTTTTCGGAAATGTTATGAAAATCACCACTTGGAATGTC
AATTCGCTCAATGTGCGGCTGCCGCAGGTGCAAAACCTGCTTGCCGACAATCCGCCCGAT
ATTTTGGTTTTGCAGGAACTCAAACTCGATCAGGACAAATTTCCGGCCGCCGCTTTGCAA
ATGATGGGCTGGCACTGTGTTTGGAGCGGGCAGAAAACCTACAACGGCGTGGCAATCGTC
AGCCGCAGCGTGCCGCAGGACGTGCATTTCGGTTTGCCCGCACTGCCGGACGATCCGCAA
CGGCGCGTGATTGCGGCAACCGTCAGCGGCGTGCGCGTCATCAATGTCTATTGCGTCAAC
GGCGAGGCTTTGGACAGCCCCAAATTCAAATATAAGGAACAGTGGTTTGCCGCACTGACG
GAGTTTGTCCGCGATGAAATGACCCGCCACGGCAAACTGGTGTTGCTGGGCGATTTCAAT
ATCGCGCCTGCCGATGCGGACTGTTACGACCCTGAAAAATGGCACGAAAAAATCCACTGT
TCGTCCGTCGAACGGCAGTGGTTTCAAAACCTGCTGGATTTGGGACTGACCGACAGCCTG
CGCCAAGTCCATCCCGAAGGCGCGTTCTATACCTGGTTCGACTATCGCGGCGCGATGTTC
CAACGCAAACTGGGCCTGCGTATCGACCATATTTTGGTGTCGCCTGCGATGGCGGCGGCG
TTGAAGGATGTCCGCGTCGATTTGGAGACGCGCGCGCTGGAGCGTCCGAGCGACCACGCG
CCGGTGACGGCAGAATTCGATTGGTAAAAGACCGTGTTTTGATATGGCGTTGACAAGCAT
CCTTATCTTCAATTTATTCAATAGGATAGCTTTCTATCTGACTGAAAAATAATTGCCTTT
CCCCGGCAAACAGCCGAAATCGGCGGATTGTTCAAACACAGCCTATTTTCCTGAAAAATT
TATGAAATACATAGGGTTAATATCAGATTTTGGAGCAGTAAAATTTATTATGTACACTAA
TCCAAAACAAAATCAAATATTGAAAACTAGATTTATTTTCGAATAAATAGAAAGCCGTCT
TATATATAGTAATAAATTAATAACCCTGTTTTTCCTATTGCCTTTATTGTGCCATGCAGT
TGAGTTTGATGAAACTCAATATAACGACTGTAAAGATAAATCTATGTTATGTGCTGTCAG
AATTGATTCTCCCAAAGGCAATAACTATAGTGGATTAACAAAAATCAGGACAAGGCGACG
AAGCCGCAGACAGTACAAATAGTACGGCAAGGCGAGGCAACGACGTACTGGTTTAAATTT
AATCCACTATATAAATCTATGTGGTTTGACAATGGCAAGTTAGTATTTATATCCTTTACT
AATCAACAAATGGAAAATCAAAGTCGCCCATCTCTAGCGATGTTTATTAGTGATGACAAA
ATATCCAGTACCAATATTGATGAATTTTTAGCATCTTTCGATCCTGATAAATATCGAATA
TTTCATGATCCAAGATATAAATTTTTACCTAGTATGTCGAACTCATTGTAATCCTTATTC
TCTTTTTGATATTGATAGCAAATATAAACCTGATGAGAAAGATAAAATCTTTTTTTCAAT
CCCGACAGATAACACAGATTTTTATAAGGGTTTTTATTTAAATAAGGATTATATAGAAGG
TATATATCCTAGTAGGCATAATGGCAGCTATTACAAAATATAGTGGATTAAATTTAAACC
AGTACAGCGTTGCCGTACTATTTGTACTGTCTGCGGCTTCGTCGCCTTGTCCTGATTTTT
GTTAATCCACTATATCTGCATCAGTTTCATGAAACGCAAGTCGGAAGCGTCAAACAACTG
ATTGCCCATTTTGACCGGCTGATTGACGAATTGGACAAACAAATCGACGACCACACCCAC

## Appendix A

ACGCATTTTGACGGCAAAGCCCAAGTGGCAGAACAAATCAAAGGCATCGGTTCGATAACG
ACGGCTACGCTGATGGCGATGCTGCCCGAATTGAGGCGGCTGTCGCACAAACGGATAGCG
GGTTTGGCCGGCATTGCCCCGCACCCGAGGGAGAGCGGGGAAACCAAATTCAAAAGCCGC
TGCTTTGGCGGAAGGTCTGCGGTGCGTAAGGCACTGTATATGGCTACCGTGGCAGCGACA
CGTTTTGAACCGCTTATTCGGGATTTCCACCAACGCCCGCTGTCCGAGGGTAAGCCGTAT
AAGGTTGCCGTTACGGCATGTATGCGCAAACTGCTGACGATATCGAATGCCCGGATGCGT
GATTATTTTGCCGAAAACGATACCGCCGAAAACGGTATCTAAACGGCTTGATTTGAGTTT
TGGTATTTTTGCCCGACGGGGTGAAAAATACAGTTGCTACGGCTCGATGAATCGTCAGAA
ATACCTGCATCGTCATTCCCGCGCAGGTGGGAATCCAGACCGGTCGGTGCGGAAACTTAT
CAGGTAAAACGGTTTCTTGAGATTTTTCGTCTTGGATTCCCACTTTCGTGTGAATGACGG
AATGTAGGTTCGTGGGAATGACGTGGTGCAGGTTTCCGTATGGATGGATTCGTCATTCCC
GCGCAGGCGGGAATCTAGTCTGTTCGGTTTCAGTTATTTTCGATAAATGCCTGTTGCTTT
TCATTTCTAGATTCCCACTTTCGTGGGAATGACGGGATTTTAGGTTTCTGATTTTGGTTT
TCTGTCCTTGTGGGAATGACGGGATGTAGGTTCGTAGGAATGACGTGGTGCAGGTTTCCG
TGCGGATGGATTCGTCATTCCTGCGCAGGCGGGAATCCAGTCTGTTCGGTTTCAGTTATT
TCCGATAAATGCCTGTTGCTTTTCATTTCTAGATTCCCACTTTCGTGGGAATGACGGTTC
AGTTGCTACGGTTACTGTCAGGTTTCGGTTATGTTGGAATTTCGGGAAACTTATGAATCG
TCATTCCCGCGCAGGCGGGAATCTGGAATTTCAATGCCTCAAGAATTTATCGGAAAAAAC
AAAACCCTTCCGCCGTCATTCCCACGAAAGTGGGAATCTAGAAATGAAAAGCAACAGGAA
TTTATCGGAAATGACCGAAACTGAACGGACTGGATTCCCGCTTTTGCGGGAATGACGGCG
ACAGGGTTGCTGTTATAGTGGATGAACAAAAACCAGTACGGCGTTGCCTCGCCTTAGCTC
AAAGAGAACGATTCTCTAAGGTGCTGAAGCACCAAGTGAATCGGTTCCGTACTATCTGTA
CTGTCTGCGGCTTCGTCGCCTTGTCCTGATTTTTGTTAATCCATTATAAAAATGCCGTCT
GAAAGGTTTTCAGACGGCATTGGTTCACGGGCCGCGCCCGGGTATTTCGGCAAAATCAGT
CGGCGACCGCCATCAGGCTGGCGTTGCCGCCGGCGGCTGTGGTGTTGACGCTGCAAGAGA
TTTCTTCAAACACTTGCAGGATGTCGAGTCCGTTTTCCGAAGGGAGGATGCGGATGAGTG
CGCCGTCGTGGGCGGCAAGTTCCTGTTTGCGCGCGCTGTCCAAAGGCGACAGGGCGGCAA
CGTGGCTGATGCCGGCGGTTTCGGGTTTGCCGTTGACCAGCAGCAGACCTTCCAAGTCGG
CAGTGTAGGAAGCCAAGGGGCTGTCGGGTTCGACCACTGCCTGTATGCCGGAGGCGGCAA
GTTCGGTCAGTGCGGCAAAGGCTTGAACCGTGCTGCCGCCGTGTATCCAAACGCGTTTGG
GCGCGTGCCATGAGATGCTGTTGCGCTCGCCGGTCGGTCCGGTAAGGACGGTTTCGGCAC
GGCGCAGGGTGCGGATGCGGGCGTGTCCCAAAGCGGCCGCTGCGGCTTTTTTCTCTTCGG
CGTTGAACGGTAGTTTGTGAACCAGTGCTTCGAGGCGTTTGAGTGCGGCTTCGTCCGCCT
GTCCGATTTGGCTCAGGGTCGGGGCAACCCATTCGCCGGCGCGGGTCAGTTTTTGCAGGT
AGAACGAACCGCCTGCTTTGGGGCCTGTGCCGGACAGACCGTGTCCGCCGAAGGGCTGTA
CGCCGACGACTGCGCCGACGATGTTGCGGTTGACGTAAACGTTGCCGGCTTCGATGCGGC
TGCGGATGTGGCGTACCGTGCCTTCGATGCGGCTGTGTACGCCGTGGGTCAGGGCGTAGC
CTTTGCTGTTGATTTGGTCGATGACGTTGTCGAGTTCGTCGGCGCGGTAGCGGACGACGT
GCAGGACGGGACCGAAGACTTCGCGTTGCAGTTCGTTGAGGTTGTTCAATTCAAACAGGA
TGGGGCGAACGAACGTGGATTTTTTGGAATCGACATCGGCGGCGGTTTTGACTTCGTGGT
AGGACTTGGCAACACCTTTCATTTTGTTGATGTGGTTCAACAGGTTTTGCTGTGCTTCGG
CATCGATGACGGGGCCGACATCGGTAGTGAGCTGAATCGGTTTGCCGACGACGAGTTCGT
CCATAGCGCCTTTGATCATGTCGAGCATACGGTCGGCAACGTCTTCTTGGACGCACAAAA
TGCGCAGGGCGGAGCAGCGTTGTCCCGCGCTGTCGAAGGCGGAGTTCAATACGTCGGCGC
AGACTTGCTCGGCAAGTGCGGTGGAATCGACAATCATGGCGTTTTGTCCGCCGGTTTCGG
CAATCAGGACGGGATTGTCGCCGCGTTTGGCAAGGGCTTTGTTGATCAGGCGCGCCACTT
CGGTCGAGCCGGTGAAAATCACGCCGCCGATGCGGGCATCGTTGGTCAATGCCGCACCCA
CGTCGCCTGCGCCGAGGACGAGTTGCAGGGCGGAAGTCGGGATGCCGGCTTCGTGCATGA
GGGAAACGGCATAACCGGCAATCAGGCTGGTTTGTTCGGCGGGTTTGGCGATGACGGTGT
TGCCTGCCGCCAATGCGGAAACGACTTCGCCGGTAAAGATGGCGAGCGGGAAGTTCCACG
GGCTGATGGCGACAATCGCGCCGACGGCTTTTGCGTCTTGAGGCAGGGTATGTTCGGCTT
CGTTTGCGTAGTAGCGGCAGAAATCGACGGCTTCGCGCACTTCGGCAATGGCGTTGTTCA
GCGTTTTGCCTGCTTCGCGCACGGCAAGCATCATCAGTGCTGGGGTGTGCTGCTCCAGCA
AATCGGCAAAACGGCGCAGGCAGGCGGCGCGTTCGGCGGCAGGTGTCGCACTCCATTCGG
GGAACGCGGCAACGGCTGCGCCAACCGCTTCTTGGGCAAGCGCGGCATCGGCAAAGCTGA
CTGTGCCGACGATGTCGTCGTGGTCGGCAGGGTTTTTAATCGGTTGCGCTTCGCCGACAT
CGCGGGCTTTGCCGTTGACGATGGATGCGGCGTGGAAGTCTTGCGCGGCGGCTTTGTTCA
TCTGTTCTTGAAGCTGCTGCAATACGTTTTCGTTGCTCAAGTCCACGCCTTGCGAGTTCA
GACGGCATTTGCCGTACAAATCGCGCGGCAGCGGCAGGGCGTTGTGCAGGTGGATGCCTT
GTTCGGCGATGGTGTCGAACGGGCTGCGGATGAGCGTGTCGATGCTGATGTTTTCATCGA
CGATTTGGTTGACGAAAGACGAGTTCGCGCCGTTTTCCAACAGGCGGCGCACCAAGTAGG
CGAGCAGGGTTTCGTGTGTGCCGACTGGGGCGTACACGCGCACGCGGCGGCCTAAGTTTT
GCGGGCCGACGACTTGGTCGTACAGGGTTTCGCCCATACCGTGCAGGCATTGGTGTTCAA
AATCTTTGCCTTTACCCATTTGGTAGATTGCGCCCAAAGTGTAGGCGTTGTGGGTGGCAA
ATTGCGGGAATACCGCGTCTTGCGCGGAAAGCAGTTTGCGCGCGCAGGCGAGGTAGGAGA
TGTCGGTGTGGACTTTGCGGGTGTAGGTCGGATAGCCGTTCAAGCCGTCCACTTGCGCCC
ATTTGATTTCGCTGTCCCAATACGCGCCTTTGACGAGGCGGATCATTAGTTTTTGGTTGT
TGCGGCGGGCAAGGTCGATCAGGTAGTCGATAACGAACGGACAACGTTTTTGGTAGGCTT
GGACAACGAAACCGATACCTTTGTAGCCAGCCAAGTCAGGGTCTGAAACCAAAGCCTCCA
TCAAATCCAAAGACAGCTCCAGACGGTTGGCTTCTTCGGCATCGATGTTGATACCGATAT
CGTATTTTTTACCCAAAAGGAACAGCTCTTTCAGGCGCGGCAACAGTTCGCCCATCACGC
GGCCGTGTTGGGTGCGCGAGTAGCGCGGATGGATGGCGGAAAGTTTGACGGAAATACCGT
TACCTTCGTAAACGCCTTGTCCTGCCGCATCTTTGCCGATGGCGTGGATGGCTTCGACAT
AGTCGCGGTAGTAGCGGTCGGCATCGGCTTGGGTGTAGGCGGCTTCGCCCAACATATCGA
AGGAGAAGCGGTAGCCCATTTTTTCGCGTTCTTTGCCGTTTTGCAGGGCTTCTTCAATGG

## Appendix A

TCTGTCCGGTTACGAACTGTTTGCCCAGAAGCCGCATGGCGTAATTTACGCCTTGGCGGA
TGAGCGGTGCGCCGCCTTTGCTGATCAGGCGGCTGAGTGCGGAACTCATTTGTTTGTCGT
TTGTGGCGGTCAGTTTGCCGGTAATCAGCAGGCCCCAGGCGGCAGCATTGACGAAGAGGG
AAGGGCTGTTGTTCAAATGGCTTTTCCAGTTGCCGTCTGAAATCTTGTCGGCAATCAGGC
GGTCGCGCGTGGCGTTGTCGGGGATACGCAGCAGGGCTTCTGCCAGACACATCAGCGCGA
TGCCTTCTTCGCTGGAGAGTGAAAACTCGTGCATCAGCGCATCCACGCCGCCGGCTTTGG
TGCGGCCGGCGCGGACTTGGGTAACCAAACGGCGGGCAAGCTCGGAGGCGGCGTTGCGCT
CTTCGTCGCTCATCTGTGCACGTTGCAACATATCCTGTACGGCTTCGATTTCATTACGGC
GGTAGGCATCGGTTATCGCTTGGCGCAGGGCAGTTTGTGCCGGAAATGCAAAATGAAACA
TTTTTTGGATTCTCCAAAGTTTTTCGGGGGGCAGGCGGCATCGGTGCGGCCTGAATACGG
TAATATCGTAATAAATCCGCAGATGAAATACAAGGCTTCAAATGCGGGCAGGGTAGGTGC
TTCCGTTTCTTTGAAAATGAAACGGGTAAAACACAAATAAGGCCTGTATGCAGGCAAGGT
TTATTTGTGTTTGACCCGGAAACGGGTTCAGACGGCACGAACCGGGATGCCGTGCCGTCT
GAAAGGGGTTTATCGGGTGGCGCGGTAATCTGCGTCGGCTTTTTCAAAGCGTTCTTGGGT
TTCGCGCGAAGGTTCTTTGTTGAACAGGGAAACCAACACGGCAACGATCAAGCAAACAAT
AAAGCCCGGCACGATTTCGTACATCGTCAACAAGCCGCTTTCTCCTGCCGCTTGAGCCGG
TTTTTTCACCCATTCCGCCCATACGACTACGGTTAACGCACCTGCAACCATACCCGACAA
CGCGCCGTAGGCAGTGATGCGTTTCCACAATACGGACAGAATCACAATCGGGCCGAATGC
CGCGCCGAAACCTGCCCACGCGTAAGACACCAGTCCCAATACTTTGCTGTTCGGATCGGA
AGCAATCAGGATGGAAATCACGGCAATCGCCAAGACCATCAGGCGGCCGACCCATACCAA
TTCCGACTGTTGCGCGTTTTTACGCAAAAAGCCTTTGTAGAAGTCTTCGGTAATCGCGCT
GGAGCAAACCAAAAGCTGGCAGGACAGGGTGGACATCACCGCCGCCAAAATCGCGCTCAA
AATAATGCCGGCAATCCAAGGGTTGAACAGCAGGGTGGAAAGCGCGATGAAGATGCGTTC
GTGGTTGCCGCTCATAGAAGAAACTTTGTCGGGATTTGCACCGAAATACGCAATGCCGAA
ATAACCGACCGCTACCGCGCCCGCAAGGCACAACGCCATCCAAGTCATACCGATGCGGCG
TGCGGATACCAGCGATTTCGCGCTTTCGGCCGCCATAAAGCGCGCCAAAATGTGCGGCTG
TCCGAAATAGCCCAAGCCCCATGCGGCGGTGGAAATGATGCCGATGACGGTCGTACCGGC
AAACAGGCTGCCGTATTCTTTGCCCGTGCCTGCGGCGACACTTTGAATCGCGGCAGACAT
CTGTTCCGCGCCGCCCAAGCCCAGATAGACCATCACAGGCGTTAAAATCAGCGCGAAAAT
CATCAAAGAAGCCTGCAGCGTATCCGTCCAGCTTACCGCCAAAAAGCCGCCCAAGAAGGT
ATAGGCGATGGTCGCGCCCGCGCCCAGCCACATTGCCTGATTGTAAGTCATACCTTCAAA
CAGGCTTTGGAACAGGGTTGCGCCCGCCACAATGCCCGAGGCGCAATAAATCGTGAAGAA
AAACAGGATAATCAGTGCGGAAACCACTTTCATCAAGTGTCCGCCCGCGCCAAAGCGGTG
GAAGAAATAATCCGGCAGCGTCAGCGCGTTGTTGGCGTATTCGGTATGTACGCGCAGACG
GCCCGCCACCAAAAGCCAGTTGAAATACGCGCCGACCAAGAGGCCGATGGCAATCCAAGC
CTCATTCAAACCGCTCAAATAAATCGCGCCCGGCAGACCCATCAAAAGCCAGCCGGACAT
ATCGGACGCGCCTGCCGACATCGCGGTAACAAACGGGCCTAGGCTGCGCCCGCCCAAAAT
ATAATCGTCGAAATTGCGCGTAGAAAAATAGGCGGCAAGCCCGATGAGAAGGACTGCAAC
CAGATAGATTGCAAAAGTAATGTACATGGGATTCATGTGCTATTCCTCGTCTAAAACTTC
AGAATTACAGGCTTTGAAATTGCAAGCAACTTGCGCCTGAAATGTTTTTCTAATAAAAGT
ACAACGGAAAATCCGGATACCCGAAAGGGGGATTCGGATAAATTATCTTCAATCACAATA
AGATATGTAATAAAACTATATGAAATTGTAAATAATCCGTTTCAGGATAACCCAATTTCT
GTTGTTTGCAAAGCACTTAATGGCTTAAAAAGCCGAGTTTGAAACGATGCGCGTCGGAAA
AATCATTTAAAACAGCATATTGTTTTGTAGTGTCTTGTAATCGGGCGTTGCGCGGAATAT
GAAATCCGTTTTCAGGCGGCAGGTGTTTTGAGGTGTAATTTAGCAACCGCAAAGGAGGCG
CGGTATGTTTTGCCGATTATCCGCCGCCCGTTTTCAGACGGCATTTTTCCTTATACAATA
GCCGATTGAATTTGATATGTTCAGGAAGGATACAGATTATGTTCGGCAAGCAGCTTTTTG
AGGAAGTCGGCTCGAAAATCAGCGAAACCATCGCCAACAGCCCTGCCAAAGATGTGGAAA
AAAATATTAAGGCGATGCTGGGCGGCGCGTTCAACCGTATGGATCTGGTTACGCGCGAAG
AATTCGACATCCAGCAGCAGGTTTTAATCAAAACCCGTACCAAACTGGCGGCTTTGGAAG
CGCGTTTGGAAAAACTCGAAGCCGCGCAAAATCCCGAACGGGCAGCATTGGAAGCGGCTG
AAGCCGCTGCCGAAGAAGCCGTCGCCGAAATCAGGCAGCAAACCGAAGCCGGCGAATAAG
GTCGTCTGAAATATGTCGCTTGCCTTGGTTTACAGCCGCGCCTTGAGCGGTATGAATGCG
CCGTTGGTCGAAGTGGAAGCCCACCTTGCCAACGGCCTGCCACATTTCAACATCGTCGGA
CTGCCCGATATGGAAGTAAAGGAAAGTCGCGACCGTGTCCGTGCCGCCATTATTCAAAGC
GGTTTTGAATTCCCCGCCAAAAAAATTACCGTCAACCTCGCCCCCGCCGACCTGCCCAAA
GAGTCGGGGCGTTTCGATTTGCCGATTGCAATCGGCATCCTTGCCGCATCGGGGCAGGTT
GCGCCCGAAAAACTGGAGGAATACGAGTTTGCGGGGGAATTGGCACTGTCGGGGCTGTTG
CGCCCCGTGCGTGGCGCGTTGGCGATGGCGTGGCAGGGTATGCAGGCAAAACGTGCATTT
GTTTTGCCTGAAGAAAATGCAGGACAAGCCGCCGTGATGCGCGGCATTACCGTTTACGGC
GCGCGCTCTTTGGGCGAAGTCGCCGCCCATTTGAACGGCATCGAACCTTTGGCGCAAACC
GAATGCCAAGTTCCTCAGATGCCGTTTGAACATGGCGGACAACCTGATTTGTGCGATGTG
AAAGGTCAGCACACCGCGCGCCTTGCTTTGGAAATCGCTGCCGCAGGCGGACACAGCCTC
TTGATGATGGGTCCGCCGGGAACGGGCAAGTCTATGCTCTCCCAACGGCTGCCCGGCATC
CTGCCGCCGCTGACCGAAGACGAATTGGTAGAAGTTTGGGCATTGCGTTCGCTCCTGCCC
AACCACCAACAACAACTCGACAGCAACCGTCCTTTCCGCAGTCCGCATCACAGCGCCAGC
GCGGCGGCTATGGTCGGCGGCGGTTCGGATCCGCGTCCGGGCGAAATTTCATTGGCGCAC
CACGGCGTTTTGTTTTTGGACGAGCTGCCCGAGTTTGACCGCAAAGTTTTGGAAGTTTTG
CGCGAACCGTTGGAAAACGGCGAAATCCACATTTCCCGCGGCGCGCCAAGCCGTCTAT
CCTGCCAAATTCCAACTTGTTGCCGCCATGAACCCCTGCCCGTGCGGTTATCTCGGGCAT
CCCGTCAAACCCTGCCGCTGCACGCCCGAAAGCGTCGCGCGTTACCGCAGCAAGATTTCC
GGGCCGCTGCTCGACCGCATCGATTTGACCATCGAAGTCCCGAGCCTGTCCGCCGCCGAA
CTGATGCAGCAGGAAGCAGGGGAAAGCAGCGCGTCCGTTTTGGAACGCGTTATCGCCGCT
AGAGACAAACAATACGCACGGCAAGGCAAAGTGAATGCCGCCTTGAGTGTCAGTGAACTC
GACACATCCGCCCGCATTCAAAAAGAAGCGCAGGAAGCATTGGGCGGCCTGCTGGAAAAA

## Appendix A

CTCTCCCTTTCCGCCCGCAGCTTCCACCGCATTATGCGCGTGGCGCGTACATTGGCGGAT
TTGGCGGGCGACGAAGAAGTCGGCAGAAGCCACGTCATGAAAGCCATAGGTTTCCGTCGT
GCTTTATAGGAATGGGAATGGAAGCAGGTTTTGCCCAAATATGGCGATATTGTTAGAATA
TCCGCCCGTAAGCAAACGGCGTTAATGCCGTCTGAAACACATTAAGGTATGTTTATGAAC
AAATTTTCCCAATCCGGAAAAGGTCTGTCCGGTTTTTCTTCGGTTTGATACTGGCGACG
GTCATTATTGCCGGTATTTTGTTTTATCTGAACCAGAGCGGTCAAAATGCGTTCAAAATC
CCGGCTTCGTCGAAGCAGCCTGCAGAAACGGAAATCCTGAAACCGAAAAACCAGCCTAAG
GAAGACATCCAACCTGAACCGGCCGATCAAAACGCCTTGTCCGAACCGGATGCTGCGACA
GAGGCAGAGCAGTCGGATGCGGAAAAAGCTGCCGACAAGCAGCCCGTTGCCGATAAAGCC
GACGAGGTTGAAGAAAAGGCGGGCGAGCCGGAACGGGAAGAGCCGGACGGACAGGCAGTG
CGTAAGAAAGCGCTGACGGAAGAGCGTGAACAAACCGTCAGGGAAAAAGCGCAGAAGAAA
GATGCCGAAACGGTTAAAAAACAAGCGGTAAAACCGTCTAAAGAAACAGAGAAAAAAGCT
TCAAAAGAAGAGAAAAAGGCGGCGAAGGAAAAAGTTGCACCCAAACCAACCCCGGAACAA
ATCCTCAACAGCGGCAGCATCGAAAAAGCGCGCAGTGCCGCCGCCAAAGAAGTGCAGAAA
ATGAAAACGTCCGACAAGGCGGAAGCAACGCATTATCTGCAAATGGGCGCGTATGCCGAC
CGTCAGAGCGCGGAAGGGCAGCGTGCCAAACTGGCAATCTTGGGCATATCTTCCAAGGTG
GTCGGTTATCAGGCGGGACATAAAACGCTTTACCGGGTGCAAAGCGGCAATATGTCTGCC
GATGCGGTGAAAAAAATGCAGGACGAGTTGAAAAAACATGAAGTCGCCAGCCTGATCCGT
TCTATCGAAAGCAAATAATTATGAAGCTCAAACATCTGTTGCCGCTGCTGCTGTCGGCAG
TGTTGTCCGCGCAGGCATATGCCCTGACGGAAGGGGAAGACTATCTTGTGTTGGATAAAC
CCATTCCTCAAGAACAGTCGGGTAAAATTGAGGTTTTGGAATTTTTCGGCTATTTCTGCG
TACATTGCCATCATTTCGATCCTTTGTTATTGAAACTGGGCAAGGCATTGCCGTCTGATG
CCTATTTGAGGACGGAGCACGTGGTCTGGCAGCCTGAAATGCTCGGTTTGGCTAGGATGG
CGGCTGCCGTCAATTTGTCGGGTTTGAAATATCAGGCAAACCCTGCTGTGTTTAAAGCAG
TTTACGAACAAAAAATCCGCTTGGAAAACAGGTCGGTTGCCGGAAAATGGGCTTTGTCTC
AAAAAGGCTTTGACGGCAAAAAACTGATGCGCGCCTATGATTCCCCCGAAGCTGCCGCCG
CCGCATTAAAAATGCAGAAACTGACGGAACAATACCGCATCGACAGCACGCCGACCGTTA
TTGTCGGCGGAAAATACCGCGTTATCTTCAATAACGGCTTTGACGGCGGCGTTCATACGA
TTAAAGAATTGGTTGCCAAAGTCAGGGAAGAACGCAAGCGTCAGACCCCTGCTGTACAGA
AATAGCCGAACTCCCGTATCCGAAAGAAGCGCAAGCAATGGATTTTCTGATTGTCCTGAA
AGCCCTGATGATGGGCTTGGTAGAAGGTTTTACCGAATTTTTACCGATTTCCAGCACCGG
ACATTTGATTGTGTTCGGCAATCTGATTGGTTTTCACAGCAATCACAAGGTTTTTGAAAT
TGCCATCCAGCTCGGTGCAGTTTTGGCGGTAGTGTTTGAATACCGGCAACGTTTCAGCAA
TGTGTTGCACGGCTTGGGAAAAGACCGGAAAGCCAACCGCTTCGTCCTTAATCTTGCCAT
TGCTTTTATACCTGCCGCCGTGATGGGGCTGTTGTTCGGCAAACAAATCAAAGAGTATCT
GTTTAACCCCTTGAGTGTTGCAGTCATGCTGGTTTTGGGCGGTTTTTTTATTTTGTGGGT
GGAGAAACGCCAAAGCCGAGCAGAGCCTAAAATTGCCGATGTTGATGCATTGCGTCCGAT
TGATGCCTTGATGATCGGCGTTGCCCAAGTGTTTGCACTGGTTCCGGGTACGTCCCGTTC
GGGCAGTACGATTATGGGCGGATGCTTTGGGGCATCGAACGGAAAACTGCGACAGAATT
CTCGTTTTTCTTGGCTGTGCCGATGATGGTTGCCGCAACGGCTTATGATGTCCTGAAACA
TTACCGATTTTTCACCCTGCATGATGTCGGTTTGATTCTGATAGGCTTTATTGCTGCCTT
TGTTTCAGGCTTGGTAGCGGTAAAAGCGTTGCTGAGGTTTGTTTCCAAGAAAAATTATAT
TCCTTTTGCCTATTACCGCATTGTTTTTGGTATTGCCATCATTATATTGTGGCTGTCAGG
CTGGATAAGTTGGGAATGAAACCATAAACCCGACCTGAAGACATTATTCGGGTCGGGTTT
GTCTGGCGGGCTGATATAGTGAATTAACAAAAATCAGGACAAGGCGACGAAGCCGCAGAT
AGTACGGCAAGGCGAGCCAACGCTGTACCGGTTTAAATTTAATTCACTATAAAATCAGGA
CAGGCGGGGCGATAGGTTTAAAGTCGATTGCCTGTTTTGAAGGCAGTGGTTTATTCTTTA
TTTGCTGGCAATCAGGCAATAAAAAAGCACATACCTTTTTACGGTCTGTGCTTTTTTATC
TGGTGGAGGTAAGCGGGATCGAACCGCTGACCTCTTGCATGCCATGCAAGCGCTCTACCA
ACTGAGCTATACCCCCGAAAATTTGGTGGCGAATCAGGGACTCGAACCCCGGACACAAGG
ATTATGATTCCTCTGCTCTAACCGACTGAGCTAATTCGCCGTTTCGTGAAGACGCTATTA
TATGTTTTTCTGTTTTTTTGACAAGCCGTATTTTTTAATTTTGAATTAGTTGACTGTTTT
TAAATGTTAAAAAGTTTATGCCGTCTGAAGCGGATTCAGGCGGCATGAGGGTTAGAGTTT
GTGGCAGATGTCGCCGAAGCGGAATCCTGCCCAGTCGATGCCGATATTTTTTCCGAATGC
GATGACTTTAAACAGTTCGCCCATTTCATGCTGGTCAATCAGTTTCTGAACGGCAGCAGC
TTCACAGATGTAGGCTGCCGAATCCGTTTTCCCCGTCTGTGCCAATAGCTCGGTAATGCC
CAAGTTCAATAAGAAATGGGATTGGGGAAGGTAACCTATCAAATCTAATCCGGCATCCGT
CCCTGCTTGTGCAATGTCGGTAAAGTTGACATGTGCGGTCAGGTCGGCCAATCCGATGAA
GTCAAAAGGATTGTGGATAATGTGATGTCGGTAGTGTCCGATCAGAGTACCTTGATTGCG
TTGAGGGTGGTAATACTGCGCTGCATCAAAACCGTAGTCGATGAATATCATGCAGCCGTG
TTCGAGTCTTGAGGCAAGGGTGCGGATAAAGGCATATTGTTGCGGATGTAGTTCGCTGGT
ATAGGGATAATCTGTTTGAGGAAAATAGAGGGAAGCCAAGGCAGATAGCTGCAAGTCGTG
CAGCGGTCGTGCCGAATAGGTAAAACGGTCATTATCTAGGCAAACGCCGACATGCTCGAA
TGAGCCGCCTTCATTTTTACGGACGATTTCGACAGGCATGGCATCGAGTACTTCGTTGCC
GATGATGATGCCGTCAAACGCTTCGGGAAGTGCGGTCAAGTGGACAACTTTTTGAGATGC
TTCCGGTGCGCGTGCTTGAATCAGGTTTTTCTGACGTGCTGCCAGCTCCGGCGATATTTC
AATAATATAGTAACGGCTGATGCCGTCCGAAATGCTGCCCAACAAATCGGCGGCAAGCTG
TCCGGTTCCCGCGCCGAATTCATAGATATTGCCCGCCGTTTGGGATAGAAGTTCTTGAAG
TTGGCGTGCCAGTGTCTGTGCAAACAGAGAGGTGAGGGTCGGTGCGGTAATAAAATCCCC
GGTATTGCCGATTTTATGGCTGCCGCCGGTGTAGTAGCCGTATTGCGGAGCGTATAAAAC
CAATTCCATAAAACGTGAAAATGGAATCCAGTTGCCGTGTTTGCCGATTTTTTCGGCAAT
GAGGGTTTGCAGTTTGAGCGAGAATTGCCGTGCTTCGGGAGAGGGGAGGGGCATGATAAG
TGTTAGCTTGTGTAAATTTATTGGATTTCCCGACATATTACACGTTGGTACGGGTGCTGT
CATGGCTTTATCTTAATACTATATATTGTGTTTATATTATTAAATTAATCATATATAGTT
GTTTATTGGTTCGATTATTCTGTACCGCACCCGCCGTGCCGTTGTCGTCATTTTTTATCT

## Appendix A

TATTGTTTTTAAAAGGAATAAAAATTTCAGATATGTTAATGAGTTTTCATGCCCTGATTT
GACCGAGTGTTTAAAATTTCTTATAGTGTCGATTGGTGGGGAATTGTGGGGCAAAGTGTC
TCTTTTACCCTTGTGATTTTGATTTCGGCTTGGGACATGTCATGTTCGGCGGCGCACACG
AATTAAGCATCGACAGTAAGGGGCGGTTGGCTGTTCCTGCCAAATTCCGTGACATTCTGT
CGCGCCTCTATACGCCTGCCGTAGTGGTAACGCTCGAGTCGAAACACAAGCTGTTGATGT
ACCCTGTTGCGGAGTGGGAAAAGGTTGCGGCGCAACTTTTAAACTTAAAAGTGGCGGATA
ACCCTGTTTTGCGGCGGTTTCAAAATCTTTTGCTGCATAACGCGGAAATTTTGGAATGGG
ACAGCGCCGGCCGGGTGCTGGTTTCTGCCGGACTGAGGAAGAGGGTGGATTTCGACCGTG
AAGTCGTTTTGGTCGGTCGTGCCAACCGTTTGGAGCTTTGGGGTCGCGAGCAGTGGGAGG
CTGAGATGGTTCAGGCTTTGGATGACGATCCTGACGAACTTGCCTTCCAGTTGAGTCAGA
CGGATTTGCAATTGTGAGTGGAGCAGAAAGTTACCGGCATATCACGGTCTTGCTGAATGA
GGCGGTGGATGCGCTTGCCGTGCGCGAAGACGGTGTCTATGTGGACGGTACGTTCGGCAG
GGGAGGGCATTCCCGGCTGATTTTGTCGCGTTTGGGCGATGCGGGGCGGTTGATTGTTTT
CGACAAAGACCCGCAGGCGATTGCTGTGGCAGAAGAGCTGGCGCGTTCGGACAAACGGGT
CGGTGTCGTGCATGGCGGTTTTGCTTCGTTTCAGACGGCATTGGACGGTTTGGGTATCGG
CAAGGTGGACGGTGCGCTGTTTGATTTGGGGATTTCGTCCCCGCAAATCGATGACGGCAG
CCGCGGTTTCAGCTTCCGTTTCGATGCCCCTTTGGATATGCGTATGGATACGACGCGCGG
TATGTCTGCCGCAGAGTGGATAGCGGTTGCGTCGGAACAGGATTTGCACGAGGTAATCAA
GAATTATGGTGAAGAGCGGTTTAGCCGCCGGATTGCGCGCGCCATTGTTGCGCAACGGGC
GGAAAGTCCAATCGATACAACCCGCAAGCTGGCGCAGATCGTGGCACAAAACGTCCGTAC
TCGCGAGCGGGGGCAGGATCCTGCGACGCGCACCTTCCAGGCGGTCCGCATCTTTATTAA
CCGCGAGCTTGAAGAAGTAGGGGCAGTATTGCCGCAGGTCATGTGTCGTCTGAAAGAGGG
CGGACGTTTGGCGGTCATTGCTTTCCATTCGTTGGAAGATCGCATTGTGAAGCAGTTTGT
CAAAAAATATTCGCAACACGCGCCCCTGCCGCGCTGGGCGGCGGTCAGGGAAGCGGATTT
GCCCGAGCTGCCCCTGAAAATCGTGGGCAGGGCATTAAAGCCGGGTGAGGCGGAAATTGC
CGCCAATCCGAGGGCGAGAAGTGCGGTTTTGCGTGTGGCGGAGCGGACTGCCGGTCCGAT
ACCGGAACAATCACAGAGAAAAACGTCTGAATGGCAATGAACAAATTGAATTTCCTTCTG
CTGCTTGCGGTGTGCGTTTCCGCTTTTTCCGTTGTGATGCAGCAAAACCAGTACAGGCTC
AATTTCACAGCTTTGGATAAGGCGAAAAAACAGGAAATCGCCTTGGAGCAGGATTATGCG
CAAATGAGGCTGCAACAGGCGCGTTTGGCGAACCACGAAGCGATCAGGGCGGCGGCAGAA
AAACAAAACCTCCATCCGCCGGTTTCGGGCAATACCTTTATGGTGGAGCATCAAAGATAG
AAGCAGCCTGTGTGCCGGAATCGGATTCCTGCGTCAGGATAATAATAACGAGAAGTAAAA
ATGTTGATTAAGAGCGAATATAAGCCTCGGATGCTGCCCAAAGAAGAGCAGGTCAAAAAG
CCGATGACCAGTAACGGACGGATCAGCTTCGTCCTGATGGCAATAGCGGTCTTGTTTGCC
GGTCTGATTGCTCGCGGACTGTATCTGCAGACGGTAACGTATAACTTTTTGAAAGAACAG
GGCGACAACCGGATTGTGCGGACTCAAACATTGCCGGCTACACGCGGTACGGTTTCGGAC
CGGAACGGTGCGGTTTTGGCGTTGAGTGCGCCGACGGAGTCCCTGTTTGCCGTGCCTAAA
GAGATGAAGGAAATGCCGTCTGCCGCACAATTGGAACGCCTGTCCGAGCTTGTCGATGTG
CCGGTTGATGTTTTGAGGAACAAGCTCGAACAGAAAGGCAAGTCGTTTATCTGGATTAAG
CGGCAGCTCGATCCCAAGGTTGCCGAAGAGGTCAAAGCCTTGGGTTTGGAAAACTTTGTA
TTTGAAAAAGAATTAAAACGCCATTACCCGATGGGCAACCTGTTTGCACACGTCATCGGA
TTTACCGATATTGACGGCAAAGGTCAGGAAGGTTTGGAACTTTCGCTTGAAGACAGCCTG
CATGGCGAAGACGGCGCGGAAGTCGTTTTGCGGGACCGGCAGGGCAATATTGTGGACAGC
TTGGACTCCCCGCGCAATAAAGCCCCGAAAAACGGCAAAGACATCATCCTTTCCCTCGAT
CAGAGGATTCAGACCTTGGCCTATGAAGAGTTGAACAAGGCGGTCGAATACCATCAGGCA
AAAGCCGGAACGGTGGTGGTTTTGGATGCCCGCACGGGGGAAATCCTCGCCTTGGCCAAT
ACGCCCGCCTACGATCCCAACAGGCCCGGCCGGGCAGACAGCGAACAGCGGCGCAACCGT
GCCGTAACCGATATGATCGAACCCGGTTCGGCAATCAAACCGTTTGTGATTGCGAAGGCA
TTGGATGCGGGCAAAACCGATTTGAACGAACGGCTGAATACGCAGCCTTATAAAATCGGA
CCGTCTCCCGTGCGCGATACCCATGTTTACCCCTCTTTGGATGTGCGCGGCATCATGCAG
AAATCGTCCAACGTCGGCACAAGCAAACTGTCTGCGCGTTTCGGTGCCGAAGAAATGTAT
GACTTCTATCATGAGTTGGGCATCGGTGTGCGTATGCACTCGGGCTTTCCGGGCGAAACT
GCAGGTTTGTTGAGAAATTGGCGCAGGTGGCGGCCTATCGAACAGGCGACGATGTCTTTC
GGTTACGGCCTGCAATTGAGCCTGCTGCAATTGGCGCGCGCCTATACCGCACTGACGCAC
GACGGCGTTTTACTGCCGGTCAGCTTTGAAAAACAGGCGGTTGCGCCGCAAGGCAAACGC
ATATTCAAAGAATCGACCGCGCGCGAGGTACGCAATCTGATGGTTTCCGTAACCGAGCCG
GGCGGCACCGGTACGGCGGGTGCGGTGGACGGTTTCGATGTCGGCGCGAAAACCGGCACG
GCGCGCAAGTTCGTCAACGGGCGTTATGCCGACAACAAACACATCGCTACCTTTATCGGT
TTTGCCCCCGCCAAAAATCCCCGTGTGATTGTGGCGGTAACCATTGACGAACCGACTGCC
CACGGTTATTACGGCGGCGTAGTGGCAGGGCCGCCCTTCAAAAAAATTATGGGCGGCAGC
CTGAACATCTTGGGCATTTCCCCGACCAAGCCACTGACCGCCGCAGCCGTCAAAACACCG
TCTTAATCCGAGTATCAACGAGATTGTTTTATGTTCAGCAAGTTAACCCCTTTGGCTGAA
ACCGGCATCCCGACTCTGTCGTGTGCAAACGCGGCAGGGCGTTTGTTGCATTCAGACAGC
CGCCAAATCAAACAAGGCGATATTTTCGTTGCCTGTCCGGGCGAATATGCCGACGGACGC
AGTTATATCCCCGCCGCCGTTGCCAACGGCGCGGCTTTTGTTTTTTGGGACGACGACGGC
AAATTTGCGTGGAATCCCGAATGGAAAGTCCCCAATCAAGGCATCAAAGATTTGAAACAC
CGTGCCGGCATATTGGCGGCGCAAGTTTACGGCAACGTTTCAGACGGCCTCAAAGTTTGG
GGCGTAGCCGGAACCAACGGCAAAACCTCCATCACACAATGGCTGGCGCAAGCTGCCGAT
TTGTTGGGCGAAAAAACCGCCATTGTCGGCACGGTCGGCAACGGCTTTTGGGGTGCATTG
GAAGAAACCACGCATACCACACCCGCCCCCGTCGATGTCCAAACCCTGCTCTACCGTTTC
CGTCAACAAGGCGCAACAGTCGCCGCGATGGAAGTCTCCAGCCACGGGCTTGACCAGTCG
CGCGTCAACGGCGTGTCATTCCGCAGCGCAATCTTTACCAACCTCACCCGCGACCACCTC
GACTACCACGGCACGATGGAAGCCTACGGTGCCATCAAGTCGCGCCTGTTTTACTGGCAC
GGCTTGAAACACGCAGTCATCAACGTGGATGACGAATACGGCGCGGAACTCGTAGGTCGT
CTGAAAAAAGACTGTCCCGATTTGGCCGTTTACAGCTATGGTTTCAGCGAACACGCCGAC

# Appendix A

ATCCGCATTACCGACTTTACCGCCTCTTCAGACGGCATAGCAGCCGTATTCCAAACCCCG
TGGGGCGAAGGGAAATGCCGCACGCGCCTGCTCGGACGGTTCAACGCGCAAAACCTCGCC
GCCTGCATCGCCTTGCTGTGCGCCAACGGCTATCCGCTTGATAAGGTATTGGATGTGCTG
GCAAAAATCCGTCCCGCTTCAGGGCGCATGGACTGCATCATGAACAGCGGCAAGCCCTTG
GTCGTTGTCGATTATGCCCACACGCCCGACGCATTGGAAAAAGCACTCGCCACCTTGCAG
GAAATCAAACCGCAGGGTGCGGCTTTATGGTGCGTATTCGGTTGCGGCGGCAACCGCGAT
CGCGGCAAACGCCCGCTGATGGGCGCGGCAGCCGTACAGGGCGCGGATAAAGTCGTCGTC
ACCAGCGACAACCCGCGTTTGGAAAATCCGCACGACATCATCAACGACATCCTGCCTGCC
GTTCCCGCGCCCGAATGCGTCGAAGCCGACCGTGCCGCCGCCGTCCGTTATGCGGTTGAA
CAAGCCGCCGCAAACGACATCATCCTGATTGCCGGCAAAGGGCATGAAAACTATCAGGAT
GTACAAGGCGTGAAGCACCGTTTTTCCGATCTTGAAATCGTCGGACAGGCTTTGTTAACT
CGTAAATAATGGGATATTCGGACGGCATCGTATGAAACAATCCGCCCGAATAAAAAATAT
GAATCAGACATTAAAAAATACATTGGGCATTTGCGCGCTTTTAGCCTTTTGTTTTGGCGC
GGCCATCGCATCAGGTTATCACTTGGAATATGAATACGGCTACCGTTATTCTGCCGTGGG
TGCTTTGGCTTCGGTTGTATTTTTATTATTATTGGCACGCGGTTTCCCGCGCGTTTCTTC
AGTTGTTTTACTGATTTACGTCGGCACAACCGCCCTATATTTGCCGGTCGGCTGGCTGTA
TGGTGCGCCGTCTTATCAGATAGTCGGTTCGATATTGGAAAGCAATCCTGCCGAGGCGCG
TGAATTTGTCGGCAATCTTCCCGGGTCGCTTTATTTTGTGCAGGCATTATTTTTCATTTT
TGGCTTGACAGTTTGGAAATATTGTGTATCGGGGGGGGTATTTGCTGACGTAAAAAACT
ATAAACGCCGCAGCAAAATATGGCTGACTATATTATTGACTTTGATTTTGTCCTGCGCGG
TGATGGATAAAATCGCCAGCGATAAAGATTTGCGAGAACCTGATGCCGGCCTGTTGTTGA
ATATTTTCGACCTGTATTACGATTTGGCTTCCGCGCCGGCACAATATGCCGCCAAGCGCG
CCCACATTTTGGAAGCAGCAAAAAAAGCGTCAACATGGCATATCCGTCATGTTGCGCCCA
AGTATAAAAATTATGTTGTGGTTATCGGTGAGAGCGCGCGTTCGGATTATATGAATGTTT
ACGGTTTCCCATTGCCCGATACGCCTTTTTTGAGTCAGACCAAAGGGCTGTTGATAAACG
GTTACCAATCGACCGCCCACGCGACGAATCTTTCGCTGCCGCAGACTTTGGGGCTGCCGG
GAGAACCGAACAATAACATCGTCAGCTTGGCGAAGCAGGCGGGTTTTCGGACGGCGTGGC
TGTCTAATCAAGGAATGTTGGGGCATTTTGCCAACGAAATTTCCACCTATGCCCTACGCA
GCGATTATCCGTGGTTTACCCAAAGGGGTGATTATGGCAAAAGCGCGGGGTTGAGCGACC
GCCTTTTGTTGCCGGCGTTCAAACGGGTTTTGATAGGAAATGCAGGCACGAAGCCTCGGC
TGATTGTGATGCACCTGATGGGTTCGCACAGTGATTTTTGCACACGTTTGGATAAGGATG
CGCGGCGGTTTCAGTATCAAACTGAAAAAATATCCTGCTATGTTTCCACCATCGCGCAAA
CCGATAAATTTTTAGAAGATACAGTTAAGATATTGAATGAAAATAAAGAAAGCTGGTCTT
TGGTTTACTTTTCCGACCACGGTTTGATGCATGTCGGTAAAGGCGGCGAGCGAACGTTGA
CACATGGTGCGTGGAAGCGTCAAAGCTACGGCGTGCCGCTGGTTAAAATTTCGTCCGATG
ACACGCGGCGCGAAATGATTAAAGTGAGGCGCAGCGCGTTTAATTTTTTACGCGGATTCG
GCAGTTGGACGGGTATCGAAACCGACGAGTTGCCCGATGACGGCTATGATTTTTGGGGGA
ATGTTCCCGATGTGCAGGGCGAAGGCAATAACCTTGCCTTTATCGACGGACTGCCCGACG
ACCCCGCGCCGTGGTATGCGGGAAAAGGCAAATCGACTAAAAATACGTCTAAAAAATGAT
ACGTACAGAAAAAATGCCGAATGAGAATGGGAAAATAATCTGTGTTTTACCACAGCAAAA
CAGGCGATAAAAAAATCAGCCGCTACCGATGTGTCCGCCGCCCGAATATTAACGAAAGTA
AATATGAAACCACTGGACCTAAATTTCATCTGCCAAGCCCTCAAGCTTCCGATGCCGTCT
GAAAGCAAACCCGTGTCGCGCATCGTAACCGACAGCCGCGACATCCGCGCGGGCGATGTG
TTTTTCGCATTGGCGGGCGAGCGGTTTGACGCGCATGATTTTGTTGAAGACGTATTGGCT
GCTGGTGCGGCGGCGGTTGTGGTTTCGCGCGAAGATTGTGCTGCAATGGATGGCGCGTTG
AAAGTCGATGACACGCTTGCCGCATTGCAAACGCTGGCAAAGGCGTGGCGTGAAAATGTG
AATCCGTTTGTGTTCGGCATTACCGGTTCGGGCGGCAAGACGACGGTGAAGGAAATGCTG
GCTGCGGTATTGCGCCGCCGTTTCGGCGATGATGCCGTGTTGGCGACGGCAGGCAACTTC
AACAACCATATCGGATTGCGGGTGACTTTGTTGAAGTTAAACGAAAAACACCGCTATGCC
GTGATTGAAATGGGCATGAACCATTTCGGCGAACTGGCGGTTTTAACGCAAATCGCCAAA
CCAAATGCCGCATTGGTCAACAACGCCATGCGCGCCCATGTCGGCTGCGGTTTCGACGGA
GTGGGCGATATTGCCAAAGCGAAAAGCGAGATTTACCAAGGTTTATGTTCAGACGGCATT
GCACTGATTCCTCAAGAAGATGCCAATATGGCTGTCTTCAAAACGGCAACGCTTAATTTG
AATACGCGCACTTTCGGCATCGATAGCGGCGATGTTCACGCGGAAAATATTGTGCTGAAA
CCGTTGTCGTGCGAATTTGATTTGGTGTGCGGCGATGAGCGCGCCGCCGTGGTGCTGCCT
GTTCCCGGCCGCCACAATGTCCACAACGCCGCCGCTGCCGCCGCGCTGGCTTTGGCTGCG
GGTTTGAGTTTGAACGATGTGGCGGAAGGTTTGAAAGGCTTCAGCAATATCAAAGGCCGT
CTGAACGTCAAATCCGGAATCAAGGGCGCAACCCTGATTGACGATACTTATAATGCGAAC
CCTGACAGCATGAAAGCTGCGATTGACGTGTTGGCGCGTATGCCTGCGCCGCGTATTTTC
GTGATGGGCGATATGGGCGAACTGGGCGAACTGGGCGAGGACGAAGCCGCCGCTATGCAC
GCCGAAGTCGGCGCGTATGCCCGCGACCAAGGCATCGAAGCGGCTTATTTTGTCGGCGAC
AACAGCGTCGAAGCGGCGGAAAAATTTGGCGCGGACGGTTTGTGGTTCGCCGCCAAAGAC
CCGTTGATTCAAGTGTTGCGCCACGATTTGCCCGAACGCGCCACCGTGTTGGTGAAAGGT
TCGCGCTTTATGCAGATGGAAGAAGTGGTCGAGGCATTGGAGGATAAGTGAAAATGAAAA
GCCGACGTTTTTTAAAGCCTTATTGCTGATTGCCGCGCTGGTCGGCGCGTTTTATGCCG
GAATGCGGACGCAGGCGTATCTTTATGAAGATTTATGTTTAGACTTGGGCGGCGGTAAAA
ATCCGGGGAGTTACCCAATTTGCGTGATTGAGAAAGTCCCTGCACGTTAATCTGCAAAAG
CCGTCCGAAACCTTGCCGGGCGGCAAGCCAACCTCAAACGGGCGCAGGCCCGATGTATAG
TGGATTAACAAAAATCAGGACAAGGCGACGAAGCCGCAGACAGTACAAATAGTACGGAAC
CGATTCACTTGGTGCTTCAGCACCTTAGAGAATCGTTCTCTTTGAGCTAAGGCGAGGCAA
CGCCGTACTGGTTTTTGTTAATCCACTATAACGACAAAACAAAAAAAGGAAGCCCCATGT
TTTTATGGCTCGCACATTTCAGCAACTGGTTAACCGGTCTGAATATTTTTCAATACACCA
CATTCCGCGCCGTCATGGCGGCGTTGACCGCCTTAGCGTTTTCCCTGATGTTCGGCCCGT
GGACGATACGCAGGCTGACCGCGCTCAAATGCGGGCAGGCAGTGCGTACCGACGGTCCGC
AAACCCACCTCGTCAAAAACGGCACGCCGACGATGGGCGGTTCGCTGATTCTGACCGCCA

## Appendix A

TTACCGTGTCCACCCTGTTGTGGGGCAACTGGGCAAACCCGTATATCTGGATTCTCTTGG
GCGTATTGCTCGCCACGGGCGCACTCGGTTTTTACGACGACTGGCGCAAAGTCGTCTATA
AAGACCCCAACGGCGTGTCCGCCAAATTCAAAATGGTGTGGCAGTCAAGCGTTGCCATTA
TCGCCAGTTTGGCATTGTTTTACCTTGCCGCCAATTCCGCCAACAATATTTTGATTGTCC
CGTTCTTCAAACAAATCGCCCTGCCGCTGGGCGTGGTCGGCTTTTTGGTGTTGTCTTACC
TGACCATCGTCGGCACATCCAATGCCGTCAACCTCACCGACGGCTTGGACGGCCTTGCGA
CCTTCCCCGTCGTCCTCGTTGCCGCCGGCCTCGCCATCTTCGCCTATGCCAGCGGCCACT
CACAATTTGCCCAATACCTGCAATTACCTTACGTTGCCGGCGCAAACGAAGTGGTGATTT
TCTGTACCGCCATGTGCGGCGCGTGCCTCGGTTTCTTGTGGTTTAACGCCTATCCCGCGC
AAGTCTTTATGGGCGATGTCGGTGCATTGGCATTGGGTGCCGCGCTCGGTACCGTCGCCG
TTATCGTCCGCCAAGAGTTTGTCCTCGTCATTATGGGCGGATTATTTGTCGTAGAAGCCG
TATCCGTTATGCTTCAGGTTGGCTGGTATAAGAAAACCAAAAAACGCATCTTCCTGATGG
CGCCCATCCATCACCACTACGAACAAAAAGGCTGGAAAGAAACCCAAGTCGTCGTCCGCT
TTTGGATTATTACCATCGTCTTGGTGTTGATCGGTTTGAGTACCCTCAAAATCCGCTGAA
CCTATGCCGTCTGAACATCTTTCAGACGGCATTTGAACGCGCAATAAACCTGCGGCGACA
ATCCGCCCAGCCCTATCGTTAACGGTGGCTGAAACCCGCCTTATACTAAAACAGAAGTAA
AACCATGAAACAGACAGTCAAATGGCTTGCCGCCGCCCTGATTGCCTTGGGCTTGAACCG
AGCGGTGTGGGCGGATGACGTATCGGATTTTCGGGAAAACTTGCAGGCGGCAGCACAGGG
AAATGCAGCAGCCCAATACAATTTGGGCGCAATGTATTACAAAGGACGCGGCGTGCGCCG
GGATGATGCTGAAGCGGTCAGATGGTATCGGCAGGCGGCGGAACAGGGGTTAGCCCAAGC
CCAATACAATTTGGGCTGGATGTATGCCAACGGGCGCGGCGTGCGCCAAGATGATACCGA
AGCGGTCAGATGGTATCGGCAGGCGGCAGCGCAGGGGGTTGTCCAAGCCCAATACAATTT
GGGCGTGATATATGCCGAAGGACGTGGAGTGCGCCAAGACGATGTCGAAGCGGTCAGATG
GTTTCGGCAGGCGGCAGCGCAGGGGGTAGCCCAAGCCCAAAACAATTTGGGCGTGATGTA
TGCCGAAAGACGCGGCGTGCGCCAAGACCGCGCCCTTGCACAAGAATGGTTTGGCAAGGC
TTGTCAAAACGGAGACCAAGACGGCTGCGACAATGACCAACGCCTGAAGGCGGGTTATTG
AACAGCTCGCGATGCCGTCTGAAAGCGGCTTGGGCAGGGGCGGACATCTCCTGCTCAATA
TGATTTGTTTTAGGACAAACCAAAATGACTTTTCAAAACAAAAAAATCCTCGTCGCCGGA
CTCGGCGGTACGGGTATTTCCATGATTGCCTACCTGCGCAAAAACGGCGCGGAGGTTGCT
GCGTATGATGCGGAGCTGAAGCCGGAACGCGTGTCGCAAATCGGTAAGATGTTTGACGGG
TTGGTGTTTTACACGGGCCGTCTGAAAGATGCGCTGGACAACGGTTTCGATATTCTGGCT
CTCAGTCCCGGCATCAGCGAGCGGCAGCCGGATATTGAGGCGTTCAAGCAAAACGGCGGA
CGCGTGTTGGGCGACATCGAATTGCTGGCGGACATTGTGAACCGCCGGGACGACAAGGTA
ATTGCGATTACCGGCAGCAACGGCAAAACCACGGTAACGAGCCTGGTCGGCTATCTCTGT
ATCAAGTGCGGGCTGGATACCGTTATCGCGGGCAATATCGGCACGCCGGTTTTGGAGGCG
GAATGGCAGCGCGAAGGCAAAAAGGCGGACGTGTGGGTGTTGGAGCTTTCCAGCTTCCAA
CTGGAAAACACCGAAAGCCTGCGTCCGACTGCGGCGACGGTGCTGAACATTTCCGAAGAC
CATCTCGACCGCTACGACGACTTGCTCGACTATGCGCATACCAAAGCCAAGATTTTCCGT
GGCGACGGCGTGCAGGTTTTGAATGCGGACGATGCGTTCTGCCGCGCGATGAAGCGTGCC
GGGCGCGAGGTAAAATGGTTTTCGTTGGAACACGAAGCTGATTTCTGGTTGGAACGCGAG
ACAGGCCGCCTGAAACAAGGCAATGAAGATTTGATTGTCACGCAAGACATTCCGTTGCAA
GGTCTGCACAACGCCGCTAACGTCATGGCTGCCGTGGCTTTGTGTGAGGCCATCGGTTTG
TCGCGCGAAGCATTGCTCGAACACGTCAAAACCTTCCAAGGCCTGCCGCACCGCGTGGAA
AAAATCGGCGAGAAAAACGGCGTGGTGTTTATCGACGACAGCAAAGGCACGAATGTCGGC
GCGACTGCCGCCGCGATTGCCGGTTTGCAAAATCCGCTCTTCGTGATTTTGGGCGGCATG
GGTAAAGGGCAGGACTTCACGCCCCTGCGCGATGCACTGGTAGGCAAGGCAAAAGGCGTG
TTCTTGATTGGTGTCGATGCGCCGCAAATCCGCCGCGATTTGGACGGCTGCGGCTTGAAT
ATGACCGACTGCGCCACTTTGGGAGAAGCCGTTCAGACGGCATATGCCCAAGCCGAAGCA
GGCGATATTGTGTTGCTCAGCCCCGCCTGCGCGAGCTTTGATATGTTCAAAGGCTACGCG
CACCGTTCGGAAGTGTTTATCGAAGCGTTTAAGGCTTTGTGATGCCGTCTGAAATGCAAA
CGCCGTCATTGTTGGGCGGCAAGTAAAGATTTAGAATACCGATTTGGGATGTATCGTATG
TTCGGACGGCATTGTCTGCCGTCTGAAATTTTTGCCCTTTGCGGCAGGTGCAAACAGACT
GGCAGGTGGTTTTTTTGAAGATTTCGGAAGTATTGGTAAAAGTGGGCGACGGTGTCCACA
CTCTGCTGCTCGACAGGCCGATTGTGCGCGACGGCAGGAAATTCGACGCGCCGCTTTTGT
GGATGGTGGTGCTGATGACGGCGTTCAGCCTGCTGATGATTTATTCGGCTTCTGTGTATT
TGGCATCAAAAGAAGGCGGCGATCAGTTTTTCTATTTGACCAGACAGGCGGGGTTCGTCG
TTGCCGGCTTGATAGCGAGCGGTTTGTTATGGTTTCTTTGCAGGATGAGGACATGGCGGC
GGCTTGTGCCGTGGATTTTTGCCCTATCCGGCCTGTTGCTGGTAGTCGTATTGATTGCCG
GGCGCGAAATCAATGGCGCGACCCGTTGGATACCTTTGGGTCCGTTGAATTTCCAGCCGA
CCGAGCTGTTCAAGCTGGCGGTCATCCTTTATTTGGCAAGCCTGTTCACGCGCCGTGAAG
AAGTGTTGCGCAGCATGGAAAGTTTGGGTTGGCAGTCGATTTGGCGGGGGACGGCCAATC
TGATCATGTCCGCCACCAATCCGCAGGCACGTCGTGAAACATTAGAAATGTACGGCCGTT
TCCGGGCGATCATCCTGCCGATTATGCTGGTGGCGTTCGGTTTGGTGCTGATAATGGTAC
AGCCGGATTTCGGTTCGTTTGTCGTCATTACCGTCATTGCCGTTGGAATGCTGTTTTTGG
CAGGATTGCCGTGGAAATATTTTTTCGTCCTGGTAGGCAGCGTCTTGGGCGGGATGGTGC
TGATGATTACCGCCGCTCCCTACCGTGTGCAGCGGGTAGTGGCATTTTTGGACCCGTGGA
AAGACCCGCAGGGTGCCGGCTACCAGCTTACCCACTCTCTGATGGCAATCGGGCGCGGAG
AGTGGTTCGGTATGGGTTTGGGTGCGAGTTTGAGCAAACGCGGCTTTCTGCCGGAAGCGC
ATACCGATTTTATTTTTGCCATCATCGCCGAAGAATTCGGTTTCTTCGGTATGTGCGTGC
TGATATTCTGTTACGGCTGGCTGGTGGTGCGGGCGTTTTCCATCGGCAAGCAGTCGCGCG
ATTTGGGTTTGACTTTCAACGCCTATATCGCTTCGGGTATCGGCATTTGGATCGGTATCC
AAAGTTTCTTCAATATCGGTGTGAACATCGGTGCTTTGCCGACCAAAGGTCTGACGCTGC
CGTTGATGTCCTATGGCGGTTCGTCAGTCTTTTTCATGCTGATCAGCATGATGCTGCTGT
TGCGTATAGATTATGAAAACCGCCGGAAAATGCGCGGTTATCGGGTGGAGTAAATCATGG
GCGGTAAAACCTTTATGCTGATGGCGGGCGGAACGGGCGGACATATTTCCCCGCGCTGG

## Appendix A

CGGTGGCGGATTCATTGCGCGCGCGGCCATCATGTGATTTGGCTGGGCAGCAAGGATT
CGATGGAAGAGCGTATCGTGCCGCAATACGGCATACGCTTGGAAACGCTGGCGATTAAAG
GCGTGCGCGGCAACGGCATCAAACGCAAACTGATGCTGCCGGTTACTTTGTATCAAACCG
TCCGCGAAGCGCAGCGGATTATCCGCAAACACCGTGTCGAGTGCGTCATCGGCTTCGGCG
GCTTCGTTACCTTCCCCGGCGGTTTGGCGGCGAAGCTATTAGGCGTGCCGATTGTGATTC
ACGAGCAAAACGCCGTGGCAGGTTTGTCCAACCGCCACCTGTCGCGCTGGGCGAAGCGGG
TGTTGTACGCTTTTCCGAAAGCGTTCAGCCACGAAGGCGGCTTGGTCGGCAACCCCGTCC
GCGCCGATATTAGCAACCTGCCCGTGCCTGCCGAACGCTTCCAAGGGCGTGAAGGCCGTC
TGAAAATTTTGGTGGTCGGCGGCAGTTTGGGCGCGGACGTTTTGAACAAAACCGTACCGC
AGGCATTGGCTTTGCTGCCCGACAATGCGCGTCCGCAGATGTACCACCAATCGGGACGGG
GCAAGCTGGGCAGCTTGCAGGCGGATTACGACGCGCTGGGCGTGAAAGCCGAATGCGTGG
AATTTATTACCGACATGGTGTCCGCCTACCGCGATGCCGATTTGGTGATTTGCCGTGCCG
GCGCGCTGACGATTGCCGAGTTGACGGCGGCGGGATTGGGTGCGTTGTTAGTGCCGTATC
CTCACGCGGTTGACGATCACCAAACCGCCAACGCGCGTTTTATGGTGCAGGCGGAGGCGG
GATTGCTGTTGCCGCAAACCCAGTTGACGGCGGAAAAACTCGCCGAGATTCTCGGCGGCT
TAAACCGCGAAAAATGCCTCAAATGGGCAGAAAACGCCCGTACGTTGGCACTGCCGCACA
GTGCGGACGACGTGGCGGAAGCCGCGATTGCGTGTGCGGCGTAAACTGCCGAACCATGCC
GTCTGAAAAGCCGTTCAGACGGCATGGATGTTTTTTATTTCAATCCGCTATATATTTGTC
AGAAAACTATGGCGCGCAAACGGTCAGCCCTTTAAAATAACGCCTTTACGCATCGAAAAT
CCACCGGAACGCAACATTATGATGAAAAATCGAGTTACCAACATCCATTTTGTCGGTATC
GGCGGCGTCGGCATGAGCGGCATCGCCGAAGTCTTGCACAATTTGGGCTTTAAAGTTTCC
GGTTCGGATCAGGCGCGAAATGCCGCTACCGAGCATTTGGGCAGCCTGGGCATTCAAGTT
TATCCCGGCCATACCGCCGAACACGTTAACGGTGCGGATGTCGTCGTTACCTCTACCGCC
GTCAAAAAAGAAAATCCCGAAGTTGTCGCTGCGTTGGAGCAGCAAATTCCCGTTATTCCG
CGCGCCCTGATGTTGGCGGAGTTGATGCGCTTCCGTGACGGCATCGCCATTGCCGGCACG
CACGGCAAAACCACGACCACCAGCCTGACCGCCTCCATCCTCGGCGCGGCAGGACTTGAC
CCGACTTTCGTTATCGGCGGCAAACTCAACGCCGCAGGCACTAACGCCCGCTTGGGCAAA
GGCGAATACATCGTTGCCGAAGCCGACGAGTCGGATGCATCCTTTCTGCACCTGACACCG
ATTATGTCCGTCGTTACCAATATCGACGAAGACCATATGGATACCTACGGGCACAGCGTC
GAAAAACTGCATCAGGCGTTTATCGATTTCATCCACCGTATGCCCTTCTACGGCAAAGCC
TTTTTGTGTATTGACAGCGAACACGTCCGCGCGATTTTGCCCAAAGTGAGCAAACCTTAT
GCTACTTACGGTTTGGACGATACCGCCGACATCTACGCCACCGACATCGAAAACGTCGGC
GCGCAAATGAAATTCACCGTCCATGTTCAAATGAAAGGACATGAGCAGGGGTCGTTTGAA
GTCGTGCTGAATATGCCCGGCAGACACAACGTGCTGAACGCATTGGCAGCCATCGGCGTG
GCGCTGGAAGTCGGCGCATCGGTTGAAGCGATCCAAAAAGGCTTGCTCGGCTTTGAAGGC
GTCGGCCGCCGCTTCCAAAAATACGGCGACATCAAGTTGCCAAACGGCGGGACCGCGCTC
TTGGTGGACGACTACGGACACCACCCCGTCGAAATGGCGGCGACCCTTGCCGCCGCACGC
GGCGCGTATCTGGAAAAACGTTTGGTACTCGCCTTCCAGCCGCACCGCTATACCCGCACG
CGCGATTTGTTTGAAGACTTTACCAAAGTCCTCAATACCGTTGACGCGCTGGTGCTGACC
GAAGTTTATGCCGCCGGTGAAGAGCCGATTGCCGCCGCCGATTCCCGCGCTCTTGCCCGC
GCCATCCGCGTGTTGGGCAAACTCGAGCCGATTTACTGCGAAAACGTTGCCGATCTGCCC
GAAATGCTGTTGAACGTTTTGCAGGACGGCGACATCGTGTTGAATATGGGCGCGGGAAGC
ATCAACCGCGTCCCCGCCGCGCTGCTGGCATTGTCGAAACAGATTTGAGGCACACCCGCC
TGACAGACGGAACATCATATAAAGATCGTCTGAAACCGCAAATCAGGTTTCAGACGACCT
CTGGCAACAAGCATAAAGCAATCAGGAAAGAACAAAAACAATGCAGAATTTTGGCAAAGT
GGCCGTATTGATGGGCGGTTTTTCCAGCGAACGAGAAATCTCGCTGGACAGCGGCACCGC
CATTTTGAATGCTTTAAAAAGCAAAGGCATAGACGCATACGCCTTCGATCCTAAAGAAAC
CCCATTGTCTGAATTGAAGGCACAAGGTTTTCAGACGGCATTCAACATCCTTCACGGTAC
TTACGGCGAAGACGGGGCGGTTCAGGGTGCATTGGAACTGTTGGGCATTCCCTATACCGG
CAGCGGTGTCGCCGCATCCGCCATCGGCATGGACAAATACCGCTGCAAACTGATTTGGCA
GGCATTGGGATTGCCCGTTCCCGAGTTCGCCGTCCTGCACGACGACACTGATTTCGATGC
CGTCGAAGAAAAATTGGGCCTGCCGATGTTTGTGAAACCGGCGGCCGAAGGCAGCAGCGT
AGGCGTGGTAAAAGTCAAAGGAAAAGGCCGTCTGAAAAGCGTTTACGAAGAATTGAAACA
CCTTCAGGGCGAAATCATTGCCGAACGTTTTATCGGCGGCGGCGAATATTCCTGCCCCGT
CCTGAACGGCAAAGGGCTGCCCGGCATACACATCATTCCCGCAACCGAGTTTTACGACTA
CGAAGCCAAGTACAACCGCGACGACACCATTTATCAATGTCCTTCGGAAGATTTGACCGA
AGCCGAAGAAAGCCTGATGCGCGAACTGGCGGTTCGCGGCGCGCAGGCAATCGGTGCGGA
AGGCTGCGTGCGCGTCGATTTCCTCAAAGATACCGACGGCAAACTCTATCTGTTGGAAAT
CAACACCCTGCCCGGTATGACGAGCCATAGTTTAGTACCGAAATCCGCTGCCGTTACGGG
CGTGGGTTTTGCCGATTTATGTATTGAAATTTTGAAGACCGCACATGTGGGATAATGCCG
AAGCGATGGAACGGCTGACGCGCTGGCTGCTTGTCATGATGGCGATGCTGCTTGCTGCGT
CCGGGCTGGTTTGGTTTTACAATTCGAATCATCTGCCCGTCAAGCAGGTGTCGCTGAAGG
GCAACCTGGTTTATTCCGATAAGAAGACATTGGGCAGTTTGGCGAAAGAATACATCCATG
GGAATATTTTGAGGACGGACATCAATGGCGCACAGGAGGCCTACCGCCGGTATCCGTGGA
TTGCGTCGGTCATGGTGCGCCGCCGTTTTCCCGACACGGTTGAGGTCGTCCTGACCGAGC
GCAAGCCGGTCGCGCGTTGGGGCGACCATGCCTTGGTGGACGGCGAAGGCAATGTTTTTG
AAGCCCGCTTGGACAGACCCGGAATGCCGGTATTCAGAGGCGCGGAAGGAACGTCTGCCG
AAATGCTCCGCCGTTATGACGAATTTTCGACTGTTTTGGCAAAACAGGGTTTGGGCATCA
AAGAGATGACCTATACGGCACGTTCGGCGTGGATTGTCGTTTTGGACAACGGCATCACCG
TCAGGCTCGGACGGGAAAACGAGATGAAACGCCTCCGGCTTTTTACCGAAGCGTGGCAGC
ATCTGTTGCGTAAAAATAAAAATCGGTTATCCTATGTGGATATGAGGTATAAGGACGGAT
TTTCAGTCCGCTATGCTTCCGACGGTTTACCCGAAAAAGAATCCGAAGAATAGTGGGAAC
AGGTATCGGACAGATTACGGCCGTGCCGTCTGAAACGGTGCGACGCAAATTTCAATCAGT
TTTAAGAGCAGACGAACAATGGAACAGCAGCAAAGATACATCAGCGTACTGGATATCGGT
ACGTCTAAAGTCCTCGCACTGATCGGGGAAGTTCAAGATGACGACAAAATCAACATCGTC

# Appendix A

GGTTTGGGGCAGGCTCCTTCACGGGGCTTGCGCGCGGGCATGGTAACCAATATCGATGCC
ACCGTCCAAGCCATCAGGCAGGCGGTCAATGATGCCGAGCTGATGGCGGATACCAAAATT
ACTCACGTTACCACAGGTATCGCAGGCAACCACATCCGCAGTCTCAATTCGCAAGGTGTG
GTTAAAATTAAAGATGGGGAAGTCACGCAGGCAGACATCGATCGCGCCATTGAAACGGCA
AAGGCAATCAATATCCCGCCCGATCAAAAAATTCTCGATGCCGTGGTTCAAGACTACATT
ATTGACACCCAACTTGGCGTGAGGGAGCCCATCGGTATGAGCGGTGTGCGTCTGGATACG
CGGGTGCACATCATTACCGGTGCAAGTACGGCAGTGCAGAATGTCCAAAAATGTATCGAG
CGGTGCGGTTTGAAAAGCGATCAGATCATGCTTCAGCCGTTGGCAAGCGGGCAGGCGGTG
CTGACTGAAGATGAAAAAGACCTCGGCGTATGCGTCATCGACATTGGTGGCGGAACGACC
GATATTGCCGTTTATATGAACGGTGCCATCCGCCATACGTCCGTCATTCCGGCCGGTGGT
AATCTGATTACCAAAGATTTGTCCAAATCGTTGAGAACACCTCTCGATGCCGCCGAGTAC
ATTAAAATCCATTATGGCGTGGCATCATGCGATACGGAAGGCTTGGGTGAGATGATTGAA
GTTCCGGGCGTGGGTGACCGGACATCGCGTCAGGTTTCCAGTAAGGTTCTGGCAGCAATC
ATCAGTGCACGGATTCAGGAGATTTTTGGCGTAGTGCTGGGCGAGCTGCAAAAATCGGGT
TTCCCCAAAGAAGTGCTGAATGCGGGTATCGTTCTGACCGGCGGTGTGTCCATGATGACC
GGGATTGTGGAATTTGCCGAAAAAATCTTCGATTTGCCTGTACGCACCGGTGCACCCCAA
GAAATGGGCGGTTTGTCCGACCGCGTCCGCACACCGCGTTTTTCTACCGCTATCGGGCTG
CTTCATGCAGCATGCAAGCTGGAAGGAAACTTGCCGCAGCCGGAAAACGGTGCAGTGCAA
GAGAGGGAAGGGGGCGGCGGTTTGTTGGCAAGATTGAAACGGTGGATTGAAAACAGCTTC
TGAACAGGTGGATTGCCGTTTGACAGGTGAGAAGTATTTTGCCAGCAGCAAGATACTTCT
TATATAATGAATAATAATTTATTTAAACCGTCCTCTGAATGGGGCGAGCAGGAGTTTTTG
AATGGAATTTGTTTACGACGTGGCAGAATCGGCAGTCAGCCCTGCGGTGATTAAAGTAAT
CGGCTTGGGCGGCGGCGGTTGCAATGCAATCAATAACATGGTTGCCAACAATGTGCGCGG
TGTGGAGTTTATCAGTGCCAATACGGATGCGCAGTCTCTGGCAAAAAACCATGCGGCGAA
GAGAATCCAGTTGGGTACGAATCTGACACGCGGTTTGGGCGCGGGCGCGAATCCCGATAT
CGGCCGTGCGGCAGCCCAGGAAGACCGGGAAGCCATTGAAGAAGCCATTCGCGGTGCGAA
TATGCTGTTTATCACGACCGGTATGGGCGGCGGTACCGGTACCGGTTCCGCGCCGGTTGT
TGCTGAGATTGCCAAGTCTTTGGGCATTCTGACCGTTGCCGTGGTTACCCGACCGTTCGC
ATATGAAGGTAAGCGCGTCCATGTCGCACAGGCAGGGTTGGAACAGTTGAAAGAACACGT
CGATTCGCTGATTATCATCCCGAACGACAAACTGATGACTGCATTGGGTGAAGACGTAAC
GATGCGCGAAGCCTTCCGTGCCGCCGACAATGTATTGCGCGATGCGGTCGCAGGCATTTC
CGAAGTGGTAACTTGCCCGAGCGAAATCATCAACCTCGACTTTGCCGACGTGAAAACCGT
GATGAGCAACCGCGGTATCGCTATGATGGGTTCGGGTTATGCCCAAGGTATCGACCGTGC
GCGTATGGCGACCGACCAGGCCATTTCCAGTCCGCTGCTGGACGATGTAACCTTGGACGG
AGCGCGCGGTGTGCTGGTCAATATTACGACTGCTCCGGGTTGCTTGAAAATGTCCGAGTT
GTCCGAAGTCATGAAAATCGTCAACCAAAGCGCGCATCCCGATTTGGAATGCAAATTCGG
TGCGGCTGAAGACGAGACCATGAGCGAAGATGCCATCCGGATTACCATTATCGCTACCGG
TCTGAAAGAAAAAGGCGCGGTCGATTTTGTTCCGGCAAGGGAGGTAGAAGCGGTTGCTCC
GTCCAAACAGGAGCAAAGCCACAATGTCGAAGGTATGATCCGCACCAATCGCGGTATCCG
CACGATGAACCTTACCGCTGCGGATTTCGACAATCAGTCCGTACTTGACGACTTTGAAAT
CCCTGCGATTTTGCGTCGTCAACACAATTCAGACAAATAATGTGCTGTTTGCCCGTAAAC
CTGCTGCCTCCCGAATCGGTTTGTCCGGTTTGGGAGGTATGTTTTTCAAGATGTTGCAAT
TTCGTACGGTTTGCGGTCGGCGGATTCAGATTTTTCCACTTGATACAGACTTTCAGATAT
GGACACTTCAAAACAAACACTGTTGGACGGGATTTTTAAGCTGAAGGCAAACGGTACGAC
GGTGCGTACCGAGTTGATGGCGGGTTTGACAACTTTTTTGACGATGTGCTACATCGTTAT
CGTCAACCCTCTGATTTTGGGCGAGACCGGCATGGATATGGGGGCGGTATTCGTCGCTAC
CTGTATCGCGTCTGCCATCGGCTGTTTTGTTATGGGTTTTGTCGGCAACTATCCGATTGC
ACTCGCACCGGGGATGGGGCTGAATGCCTATTTCACCTTTGCCGTCGTTAAGGGTATGGG
CGTGCCTTGGCAGGTTGCGTTGGGTGCGGTGTTCATCTCCGGTCTGATTTTTATCCTGTT
CAGCTTTTTTAAAGTCAGGGAAATGCTGGTCAACGCACTGCCTATGGGTTTGAAAATGTC
GATTGCTGCCGGTATCGGTTTGTTTTTGGCACTGATTTCCCTGAAAGGCGCAGGCATTAT
CGTTGCCAATCCGGCAACCTTGGTCGGTTTGGGCGATATTCATCAGCCGTCCGCGTTGTT
GGCATTGTTCGGTTTTGCTATGGTGGTCGTATTGGGACATTTCCGCGTTCAAGGCGCAAT
CATCATCACCATCTTGACCATTACCGTCATTGCCAGCCTGATGGGTTTGAATGAATTTCA
CGGCATCATCGGCGAAGTACCGAGCATTGCGCCGACTTTTATGCAGATGGATTTTGAAGG
CCTGTTTACCGTCAGCATGGTCAGTGTGATTTTCGTCTTCTTCTTGGTCGATCTATTTGA
CAGTACCGGAACGCTGGTCGGCATATCCCACCGTGCCGGGCTGCTGGTGGACGGTAAGCT
GCCCCGCCTGAAACGCGCACTGCTTGCAGACTCTACCGCCATTGTGGCAGGTGCGGCTTT
GGGTACTTCTTCCACCACGCCTTATGTGGAAAGCGCGGCGGGCGTATCGGCAGGCGGACG
GACCGGCCTGACGGCGGTTACCGTCGGCGTATTGATGCTCGCCTGCCTGATGTTTTCACC
TTTGGCGAAAAGTGTTCCCGCTTTTGCCACCGCGCCCGCCCTGCTTTATGTCGGCACGCA
GATGCTCCGCAGTGCGAGGGATATTGATTGGGACGATATGACGGAAGCCGCACCTGCGTT
CCTGACCATTGTTTTCATGCCGTTTACTTATTCGATTGCAGACGGCATCGCTTTCGGCTT
CATCAGTTATGCCGTGGTTAAACTTTTATGCCGCCGCACCAAAGACGTTCCGCCTATGGT
ATGGATTGTTGCCGTATTGTGGGCACTGAAATTCTGGTATTTGGGCTGATTGATTCGATA
TTAAAAATGCCGTCTGAAAGGTTTTCAGACGGCATTTTGTTTGCCGATATATTTAATTTT
TATTAAATTATATAAAAATCAAATACATAATAAAATACATCGGATTGCTTAAAAATAATA
CATTGTTTTTATGTATAAAATATTTTATAAGTTTTCAGGATTTTGATTATCAAAAATTTT
TCTTGATTTCCTGACAATTTTATTGAAACAAATAATTCAAAATTAATCTAGTTTAATCAT
GGAATTAAAATAAAATATTAAAATTATGTAATGAGTCTCCTTAAAAATGTTTGACATTTT
CAGTCTTGTGTTTTAGATTATCGAAAAATAAAACTACATAACACTACAAAGGAACATTAC
TATGAAACCAATTCAGATGTTTTCCCCTTTTCTGAATAATCCCCTTGTTTTCTTCTTGTC
TGCGGTTTTGCCGCATAATTCCGAACGGTCTGCTGTTTTTCTTTGATTCGTTTTAAATAT
CAATAAGATAATTTTTCCCATATATTTTTAATGATTGGATTGGGATGCCCGACGCGTCGG
ATGGCTGTGTTTTGCCGTCCGAATGTGATGGAAGCCTGTCCATACTGAAAAAAAGTCTAT

## Appendix A

AAAGGAGAAATATGATGAGTCAACACTCTGCCGGAGCACGTTTCCGCCAAGCCGTGAAAG
AATCGAATCCGCTTGCCGTCGCCGGTTGCGTCAATGCTTATTTTGCACGATTGGCCACCC
AAAGCGGTTTCAAAGCCATCTATCTGTCCGGCGGCGGCGTGGCAGCCTGTTCTTGCGGTA
TCCCTGATTTGGGCATTACCACAATGGAAGATGTGCTGATCGACGCACGACGCATTACGG
ACAACGTGGATACGCCTCTGCTGGTGGACATCGATGTGGGTTGGGGCGGTGCATTCAATA
TTGCCCGTACCATTCGCAACTTTGAACGCGCCGGTGTTGCAGCGGTTCACATCGAAGATC
AGGTAGCGCAAAAACGCTGCGGCCACCGTCCGAACAAAGCCATTGTATCTAAAGATGAAA
TGGTCGACCGTATCAAAGCTGCCGTAGATGCGCGCGTTGATGAGAACTTCGTGATTATGG
CGCGTACCGATGCGCTGGCGGTAGAAGGTTTGGATGCCGCTATCGAACGCGCCCAAGCTT
GTGTCGAAGCCGGTGCGGACATGATTTTCCCTGAAGCCATGACCGATTTGAACATGTACC
GCCAATTTGCAGATGCGGTGAAAGTGCCCGTGTTGGCGAACATTACCGAGTTTGGTTCCA
CTCCGCTTTATACCCAAAGCGAGCTGGCTGAAAACGGCGTGTCGCTGGTGCTGTATCCGC
TGTCATCGTTCCGTGCAGCAAGCAAAGCCGCTCTGAATGTTTACGAAGCGATTATGCGCG
ATGGCACTCAGGCGGCGGTGGTGGACAGTATGCAAACCCGTGCCGAGCTGTACGAGCATC
TGAACTATCATGCCTTCGAGCAAAAACTGGATAAATTGTTTCAAAAATGATTTACCGCTT
TCAGACTGCCTTTCAACAAATCCGCATCGGTCGTCTGAAAACCCGAAACCCATAAAAACA
CAAAGGAGAAATACCATGACTGAAACTACTCAAACCCCGACCCTCAAACCTAAAAAATCC
GTTGCGCTTTCTGGCGTTGCGGCCGGTAATACCGCTTTGTGTACCGTTGGCCGTACCGGC
AACGATTTGAGCTATCGCGGTTACGACATTCTGGATTTGGCACAAAAATGCGAGTTTGAA
GAAGTCGCCCACCTGCTGATTCACGGCCATCTGCCCAACAAATTCGAGCTGGCCGCTTAT
AAAACCAAGCTCAAATCCATGCGCGGCCTGCCTATCCGTGTGATTAAAGTTTTGGAAAGC
CTGCCTGCACATACCCATCCGATGGACGTAATGCGTACCGGCGTATCCATGCTGGGCTGC
GTTCATCCTGAACGTGAAAGCCATCCGGAAAGTGAAGCGCGCGACATCGCCGACAAACTG
ATCGCCAGCCTCGGCAGCATCCTCTTGTACTGGTATCAATATTCGCACAACGGCAAACGC
ATTGAGGTTGAAAGCGACGAAGAGACCATCGGCGGTCATTTCCTGCAACTGTTGCACGGC
AAACGCCCAAGCGAATCACACATCAAAGCCATGCACGTTTCACTGATTCTGTATGCCGAA
CACGAGTTCAACGCTTCTACCTTTACCGCCCGCGTGATCGCCGGTACAGGCTCTGATATG
TACTCCAGCATTACCGGAGCAATCGGCGCGTTGAAAGGTCCGAAACACGGCGGCGCGAAC
GAAGTGGCTTACGATATTCAAAAACGCTACCGCAATGCCGACGAAGCTGAAGCCGACATC
CGCGAACGCATCGGCCGCAAAGAAATCGTGATCGGTTTCGGTCATCCGGTGTACACCATT
TCCGACCCTCGCAACGTTGTCATTAAAGAAGTGGCACGCGGTTTGAGCAAAGAAACCGGC
GATATGCGCCTCTTTGACATTGCCGAACGTTTGGAAAGCGTGATGTGGGAAGAGAAAAAA
ATGTTCCCGAATCTGGACTGGTTCTCTGCCGTTTCCTACCAAAAATTGGGCGTACCGACC
GCTATGTTCACACCGCTGTTCGTAATTTCCCGTACAACCGGTTGGAGCGCACACGTTCTT
GAGCAACGCAAAGACGGCAAAATCATCCGTCCGAGCGCAAACTACACAGGCCCTGAAGAT
TTGGCGTTTGTGGAGATTGAAGAACGATAATTGAAGAATGCAATAGCAGTTTGTTCTTTA
ATTTCGGTATGCAAAGCTAAGGATTTCAGACGACCTTGCCTTATTGGAAAGGTTGTCTGA
AATAAGTTTAATCTAATAGGAGAAGATAATCCTGTATTGGCGCAAGTAACAGGATAAGAA
ACATGGAAGATTTATATATAATACTCGCTTTGGGTTTGGTTGCGATGATTGCCGGATTTA
TCGATGCGATTGCGGGCGGGGGTGGTTTGATTACGCTGCCCGCACTCTTGTTGGCAGGTA
TTCCTCCCGTGTCGGCAATTGCCACCAACAAGCTGCAAGCAGCCGCTGCTACGTTTTCAG
CTACGGTTTCTTTTGCACGCAAAGGTTTGATTGATTGGAAGAAAGGTCTCCCGATTGCCG
CAGCATCGTTTGTAGGCGGCGTGGCCGGTGCATTATCGGTCAGCTTGGTTTCCAAAGATA
TTCTGCTGGCGGTCGTGCCGGTTTTGTTGATATTTGTCGCACTGTATTTTGTGTTTTCGC
CCAAGCTCGACGGCAGTAAGGAAGGCAAAGCCAGAATGTCTTTTTTTCTGTTCGGGCTGA
CGGTCGCACCGCTTTTGGGTTTTTACGACGGTGTGTTCGGACCGGGTGTCGGCTCGTTTT
TTCTGATTGCCTTTATTGTTTTGCTCGGCTGCAAGCTGTTGAACGCGATGTCTTACACCA
AATTGGCGAACGTTGCCTGCAATCTTGGTTCGCTATCGGTATTCCTGCTGCACGGTTCGA
TTATTTTCCCGATTGCGGCAACGATGGCGGTCGGTGCGTTTGTCGGTGCGAATTTAGGTG
CGAGATTTGCCGTCCGCTTCGGTTCGAAGCTGATTAAGCCGCTGCTGATTGTCATCAGCA
TTTCGATGGCTGTGAAATTGTTGATAGACGAGAGAAATCCGCTGTATCAGATGATTGTTT
CGATGTTTTAAACCCTTTCAGACGACCCCTTCAAAACGTCGGCTGAAACCTCAAACCACA
AGAAAAACAGATCCACAGGAGAACCGACATGGCTGCCAACCAACGTTACCGCAAACCGCT
GCCCGGTACGGATTTGGAATACTACGACGCGCGTGCGGCGTGTGAGGACATCAAGCCCGG
CTCTTACGACAAGCTGCCTTACACGAGCCGCATTTTGGCGGAGAATTTGGTCAACCGCGC
GGACAAAGTCGATTTGCCGACGCTGCAAAGCTGGCTGGGGCAGTTGATAGAAGGGAAGCA
GGAAATCGACTTTCCGTGGTATCCGGCGCGGGTGGTGTGCCACGATATTCTGGGGCAGAC
CGCGTTGGTGGATTTGGCAGGCCTGCGCGATGCGATTGCCGAAAAAGGCGGCGATCCTGC
CAAAGTGAATCCGGTGGTGCAAACCCAGCTCATCGTCGACCACTCTCTGGCGGTGGAGTG
CGGCGGTTACGATCCTGATGCCTTCCGCAAAAACCGCGAAATCGAAGACCGCCGTAACGA
AGACCGTTTCCACTTCATCAACTGGACAAAAACCGCGTTTGAAAATGTGGACGTGATTCC
GGCGGGCAACGGCATCATGCACCAAATCAATCTAGAAAAAATGTCGCCCGTCGTCCAAGT
CAAAAACGGCGTGGCTTTCCCCGATACCTGCGTCGGTACTGACTCACATACGCCGCACGT
CGATTCATTGGGCGTGATTTCCGTGGGCGTGGGCGGATTGGAAGCGGAAACCGTAATGCT
GGGACGCGCGTCCATGATGCGCCTGCCCGATATTGTCGGCGTTGAGCTGAACGGCAAACG
GCAGGCGGGCATTACGGCGACGGATATTGTGTTGGCACTGACCGAGTTTCTGCGCAAAGA
ACGCGTGGTCGGGGCGTTTGTCGAATTCTTCGGCGAGGGCGCGAGAAGCCTGTCTATCGG
CGACCGCGCGACCATTTCCAACATGACGCCGGAGTTCGGCGCGACTGCCGCGATGTTCGC
TATTGATGAGCAAACCATTGATTATTTGAAACTGACCGGACGCGACGACGCGCAGGTGAA
ATTGGTGGAAACCTACGCCAAAACCGCAGGCTTGTGGGCAGATGCCTTGAAAACCGCCGT
TTATCCTCGCGTTTTGAAATTTGATTTGAGCAGCGTAACGCGCAATATGGCAGGCCCAAG
TAACCCGCATGCCCGTTTTGCGACCGCCGATTTGGCGGCGAAAGGGCTGGCGAAGCCTTA
CGAAGAGCCTTCGGACGGCCAAATGCCCGACGGCTCGGTCATCATCGCCGCGATTACCAG
TTGCACCAACACTTCCAACCCGCGCAACGTTGTTGCCGCCGCGCTCTTGGCACGCAATGC
CAACCGTCTCGGCTTGAAACGCAAACCTTGGGTGAAATCTTCGTTTGCCCCGGGTTCAAA

## Appendix A

AGTAGCCGAAATCTATTTGAAAGAAGCGGGCCTGTTGCCCGAAATGGAAAAACTCGGCTT
CGGTATCGTCGCCTTCGCCTGCACCACCTGCAACGGCATGAGTGGCGCGCTGGATCCGAA
AATCCAGAAAGAAATCATCGACCGCGATTTGTACGCCACCGCCGTATTATCAGGCAACCG
CAACTTCGACGGCCGTATCCACCCGTATGCGAAACAGGCTTTCCTCGCTTCGCCTCCGTT
GGTCGTTGCCTACGCGCTGGCAGGCAGTATCCGTTTCGATATTGAAAACGACGTACTCGG
CGTTGCAGACGGCAAGGAAATCCGCCTGAAAGACATTTGGCCTGCCGATGAAGAAATCGA
TGCCGTCGTTGCCGAATATGTGAAACCGCAGCAGTTCCGCGATGTGTATGTACCGATGTT
CGACACCGGCACAGCGCAAAAAGCACCCAGTCCGCTGTACGATTGGCGTCCGATGTCCAC
CTACATCCGCCGTCCGCCTTACTGGGAAGGCGCGCTGGCAGGGGAACGCACATTAAGAGG
TATGCGTCCGCTGGCGATTTTGCCCGACAACATCACCACCGACCACCTCTCGCCGTCCAA
TGCGATTTTGGCCGTCAGTGCCGCAGGCGAGTATTTGGCGAAAATGGGTTTGCCTGAAGA
AGACTTCAACTCTTACGCAACCCACCGCGGCGACCACTTGACCGCCCAACGCGCTACCTT
CGCCAATCCGAAACTGTTTAACGAAATGGTGAAAAACGAAGACGGCAGCGTGCGCCAAGG
CTCGTTCGCCCGCGTCGAACCCGAAGGCGAAACCATGCGCATGTGGGAAGCCATCGAAAC
CTATATGAACCGCAAACAGCCGCTCATCATCATTGCCGGTGCGGACTATGGTCAAGGCTC
AAGCCGCGACTGGGCTGCAAAAGGCGTACGCCTCGCCGGCGTAGAAGCGATTGTTGCCGA
AGGCTTCGAGCGTATCCACCGCACCAACCTTATCGGCATGGGCGTGTTGCCGCTGCAGTT
CAAACCCGACACCAACCGCCATACCCTGCAACTGGACGGTACGGAAACCTACGACGTGGT
CGGCGAACGCACACCGCGCTGCGACCTGACCCTCGTGATTCACCGTAAAAACGGCGAAAC
CGTTGAAGTTCCCGTTACCTGCTGCCTCGATACTGCAGAAGAAGTATTGGTATATGAAGC
CGGCGGCGTGTTGCAACGGTTTGCACAGGATTTTTTGGAAGGGAACGCGGCTTAGAGGTC
GTCTGAAAAGCAAGACGTAGCGTGGGTCGGGTTCAACATTTTGCTCATTCACGTAATTCT
CGATATGGCAGGCATCTACTGTAAATCGTCATTCCCGCGCAGGCGGGAATCCAGAAAGTG
GAATTGAGGAAACCTTATTTATCCGATGAGTTTCTGTGCGGACAAATTTGGATTCCCGCC
TGCGCGGGAATGACGGGGTTTAATAATCTGCCGTATCACAACACAGTAGCCGTAGATTGT
GGCGAACCCCGACAGTTTGCGGAATCAAACGGCTTTGTCGGAGTGGCAGCCTAATGTACT
TCTGGAAAGTGGGTGTAGCGTGGGCTTTGCCCGCGAAATAAAGGCTGAATTGACATGGTA
TAGAGGATTAACAAAAATCGGGACAAGGCGGCGAAGCCGCAGACAGTACAGATAGTACGG
AACCGATTCACTTGGTGCTTGAGCACCTTAGAGAATCGTTCTCTTTGAGCTAAGGCGAGG
CAACGCTGTACTGGTTTTTGTTAATCCACTATAAATTTAATCCACTATACTGTAAATCGT
CATTCCCGCGCAGGCGGGAATCCAGAAAGTGGAATTGAGGAAACCTTTTTATCCGATGAG
TTTCTGTGCGGATAAATCTGGATTCCCGCCTGCGCGGGAATGACGGGGTTTAATAATCTG
CCGTATCACAACACAGTAGCCGTAGATTGGGGCGAACCCCGACAGTTTGCGGAATCAAAC
GGCTTTGGTCGGAGTGGCAGCCTAATCCACTATAAAAATCGTGGGCAGAGCCCACGCTAC
ATAAGGAGAATCTAGAAATGCCGCAAATTAAAATTCCCGCCGTTTACTACCGTGGCGGTA
CATCAAAAGGCGTGTTTTTCAAACGTTCCGACCTGCCCGAGGCGGCGCGGGAAGCGGGAA
GCGCACGCGACAAAATCCTCTTGCGCGTACTCGGCAGCCCGGATCCCTACGGCAAGCAGA
TAGACGGTTTGGGCAACGCCAGCTCGTCCACCAGCAAGGCGGTGATTTTGGACAAGTCCG
AACGCGCCGATCACGATGTCGATTACCTTTTCGGGCAAGTTTCCATCGACAAACCTTTTG
TCGATTGGAGCGGCAACTGCGGCAACCTCACCGCTGCCGTGGGCGCATTCTCCATCGAAC
AGGGCTTGGTCGATAAAGGCAAGATTCCTTCAGACGGCATCTGCACGGTCAAAATCTGGC
AGAAAAACATCGGCAAAACCATTATTGCCCATGTACCGATGCAAAACGGCGCAGTTTTGG
AAACAGGCGATTTTGAGCTCGACGGCGTAACGTTCCCGGCAGCCGAAGTACAAATCGAAT
TTCTTGATCCAGCCGACGGCGAAGGCAGTATGTTCCCAACCGGCAATTTGGTCGATGAAA
TTGATGTGCCGAATATAGGCCGTTTGAAAGCCACGCTCATCAACGCGGGCATTCCGACCG
TTTTCTTGAATGCCGCCGACTTGGGCTACACAGGCAAAGAGTTGCAAGACGACATCAACA
ACGATGCCGCGGCTTTGGAAAAATTCGAGAAAATCCGCGCTTACGGTGCGCTGAAAATGG
GTCTGATCAGCGACGTATCCGAAGCTGCCGCTCGCGCACACGCCGAAAGTCGCCTTCG
TCGCGCCCGCCGCCGATTAGAGCGGCTCCAGTGGCAAAACCGTGAACGCCGCCGACATCG
ATTTGCTGGTACGCGCCCTGAGCATGGGCAAACTGCACCACGCGATGATGGGTACCGCCT
CTGTTGCCATTGCGACCGCCGCCGCCGTACCCGGTACGCTGGTCAACCTTGCCGCAGGCG
GCGGAACGCGTAAAGAAGTGCGCTTCGGGCATCCTTCCGGCACATTGCGCGTCGGTGCAG
CCGCCGAATGTCAGGACGGACAATGGACGGCCACCAAAGCGGTCATGAGCCGTAGCGCAC
GCGTGATGATGGAAGGTTGGGTCAGGGTGCCTGAGGATTGTTTTTAAATTGACGTAGCAT
GGGTTTGCCCGCGAGCCATAAAAAGGTCGTCTGAAAAACAAGTAAACATCAAATCACTGA
CCATTCCTTTCCCTTGCCCTGTGGCGGAAGGCGGCAAATCACAAGGAAGAACACGGAAAC
CCCGATAAAAGACAGCTTCCCGTATTACCGTCATTCCCGCGCAGGCGGGAATCCAGACCT
GTCAATATGGAGGATTGGCAGGGGAAAACAGGTTTCGTGAGTTCTACATTCTGGATTCCC
GCCACAGCCTGTCCTCGCGTAGGCGGGGACGGAATAACGATAGAAAATGCGGCATACGCT
TTGCCCAAAGAGGCCGTCTGAAACACCTTGCGCCTGATGTCTGCCTTTTTCAGACGACCC
CACACCAAAAAAACAACCACAAACTACAAGGAGAAACATCATGTCCGACCAACTCATCCT
CGTTCTGAACTGCGGCAGTTCATCGCTCAAAGGCGCCGTTATCGACCGAAAAAGCGGCAG
CGTCGTCCTAAGCTGCCTCGGCGAACGCCTGACCACGCCCGAAGCCGTCATTACGTTCAA
CAAAGACGGCAACAAACGCCAAGTTCCCCTGAGCGGCCGAAATTGCCACGCCGGCGCGGT
GGGTATGCTTTTGAACGAACTGGAAAAACACGGTCTGCACGACCGCATCAAAGCCATCGG
CCACCGCATCGCCCACGGCGGCGAAAAATACAGCGAGTCTGTTTTGATCGACCAGGCCGT
AATGGACGAACTCAATGCCTGCATTCCGCTTGCGCCGCTGCACAACCCCGCCAACATCAG
CGGCATCCTTGCCGCACAGGAACATTTCCCCGGTCTGCCCAATGTCGGCGTGATGGATAC
TTCGTTCCACCAAACCATGCCGGAGCGTGCCTACACTTATGCCGTGCCGCGCGAGTTGCG
TAAAAAATACGCTTTCCGCCGCTACGGTTTCCACGGCACCAGTATGCGTTACGTTGCCCC
TGAAGCCGCACGCATCTTGGGCAAACCTCTGGAAGACATCCGCATGATTATTGCCCACTT
AGGCAACGGCGCATCCATTACCGCCATCAAAAACGGCAAATCCGTCGATACCAGTATGGG
TTTCACGCCGATCGAAGGTTTGGTAATGGGTACACGTTGCGGCGACATCGATCCGGGCGT
ATACAGCTATCTGACTTCCCACGCCGGGATGGATGTTGCCCAAGTGGATGAAATGCTGAA
CAAAAAATCAGGTTTGCTCGGTATTTCCGAACTTTCCAACGACTGCCGCACCCTCGAAAT

## Appendix A

CGCCGCCGACGAAGGCCACGAAGGCGCGCCTCGCCCTCGAAGTCATGACCTACCGCCT
CGCCAAATACATCGCTTCGATGGCTGTGGGCTGCGGCGGCGTTGACGCACTCGTGTTCAC
CGGCGGTATCGGCGAAAACTCGCGTAATATCCGTGCCAAAACCGTTTCCTATCTTGATTT
CTTGGGTCTGCACATCGACACCAAAGCCAATATGGAAAAACGCTACGGCAATTCGGGCAT
TATCAGCCCGACCGATTCTTCTCCGGCTGTTTTGGTTGTCCCGACCAATGAAGAACTGAT
GATTGCCTGCGACACTGCCGAACTTGCCGGCATCTTGTAGCCAAAAAAGGGACGAGTCCG
CAAAAATGCCGTCTGAAACCCCAAACGCCCGATTAGGCTGATGAGGATTTTAGACGGCAT
TGTTCATTTTTTTGTTATCTTGCATTTTTGTGCGGACGGTGGAATTTCATCCTGTAAACA
TAAATATTTGTCGGAAAACAGAAACCCTCCGCCGCCATTTCTACGAAAGCAGGAAACCAG
CAACGCAAAGCGACAGGGATTTGTTGGAAATGACCGAAACCGAACGAACCGGATTCCCGC
CTGCGCGGGAATGACGGGATTTTCTGTTTTTGTGGAAATGACGGGATTTTGAATTTCGGG
CGTACAATACGGAAAACATGACGATAAGGAAACAAACCATGGCACAGTTTTTCGCTATTC
ATCCCGACAATCCCCAAGAACGCCTCATCAAGCAGGCGGTTGAAATCGTCAATAAAGGCG
GCGTGGTCGTTTATCCGACCGATTCCTGTTATGCCTTGGGCTGCAAACTCGGCGATAAGG
CGGCGATGGAACGCATACTCTCCATCCGCAAAATCGATTTGAAACACCACCTGACCCTGA
TGTGCGCAGATTTGAGCGAGTTGGGCACATACGCCAAAGTCGACAACGTACAGTTTCGTC
AGCTTAAAGCCGCCACACCCGGGCCTTATACTTTTATTTTACAGGCGACGAAGGATGTGC
CGGCGCGCACGCTGCACCCGAAACGCAAAACCATCGGGCTGCGTATTCCCGATAATGCCA
TTGCACAAGCCCTGCTGGGGGAATTGGGCGAGCCGCTTTTAAGCTGCACCCTGATGCTGC
CCGAAGACGGCGAACCATTGACCGATCCTTATGAAATCCGCGAGCGTTTGGAACACGCCG
TCGATTTGGTGATTGACGGCGGCTGGTGCGGAACCGAGCCGACCACCGTCGTCGATATGA
CCGACGGCACGGAATTGGTGCGCCAAGGTTGCGGCGATACGGCGGTGTTCGGTTTGTAGG
GAAACCGATGCCGTCTGAAGCATCGGCTGTTCAGACGGCATTGCGCGCCTTGCCGGCGGC
AGTCCGAAATGCCGGCGCGTATCGCGCTCGGTCGGAATATCCGTTTGAAACGGCATTTTG
ATGCATTACTGCACCGCAATCGGAATTCTCGGTTCGTAGAGCAGGTCGTAGGTCGGCTTG
TTGAGCAGGTCTTGGAGCGTGAAACCGTCCAGATACGTGAAAAACGACTTCATCGCGCCG
CCGAGTATGCCCGTCAGCCGGCAGGACGGTGTAATCAGGCATTCGTTGTTCTCGCCCATG
CACTCGACCAGCTGCATCGGTTCGAGGTGGCGGACAACCGAGCCGATGTTGATGCGGTCG
GGCGGTGCGGCAAGCCGCAGACCGCCGCCTTTTCCGCGCACACTGTGGAGGAAGCCGCCT
TTGACCAGCGCGGTAACGACCTTCATCAGATGGCTTTTGGAAATGCCGTAGGTTACGGCG
ATGGTACTGATGTTGACCAGCGCATCGTCGTTGATGGCAGTGTAGATAAGGACGCGCAGC
CCGTAGTCCGTATGTTGTGTCAAATACATGATTTTCTCGGTATGGATTGTTATTCTTATC
GGTACGGTTTAAGGTTCACGGACAATACCTTAATGGTTGAAACCCTGTCCGTCGGGGCGG
TAGAATGCAGCCTGTCTGCGGCGGTATGCCGTCTGAAACATCCGCGCTACCGTTTGAGAA
TTTGTTATTGTAACTCAAAATCATGAAACCGTTGAAACGACATCCCGCCCTTATCGGGCT
TTCGCGTGACCACCACCATTCGCTTTCCCTGTGCGTGCGTCTGTTGCGGACGCCGGAAGA
AAGGCATCGGGACGAACTCGAACCGCATTTTTCCGAATTGGAAACCCATTTTCGCGAAGA
AGAAACCAAGTTTGCCCCAATTTGGCAGAATGTCGCCCCCGAATTGAAACAACGTTTCGA
GAAAGACCACGCCCGACTGCGGCAGATGATGGCAAGCCCCGAATACGGTAACGCGGCGTG
GAATACCGCTTTTGCCACAACCCTGCGCGACCACGCGCGCTTTGAAGAACGCGAGCTGTT
TCCCGCCGCCGAACCGTTTTTGCCGGCATGATTCCGTTTTGCGGTAAATATATTAATGAT
AAACAAGGAACACACATGAAATTTACCAAGCACCCCGTCTGGGCAATGGCGTTCCGCCCA
TTTTATTCGCTGGCGGCTCTGTACGGCGCATTGTCCGTATTGCTGTGGGGTTTCGGCTAC
ACGGGAACGCACGAGCTGTCCGGTTTCTATTGGCACGCGCATGAGATGATTTGGGGTTAT
GCCGGACTGGTCGTCATCGCCTTCCTGCTGACCGCCGTCGCCACTTGGACGGGGCAGCCG
CCCACGCGGGGCGGCGTTCTGGTCGGCTTGACTATCTTTTGGCTGGCTGCGCGGATTGCC
GCCTTTATCCCGGGTTGGGGTGCGTCGGCAAGCGGCATACTCGGTACGCTGTTTTTCTGG
TACGGCGCGGTGTGCATGGCTTTGCCCGTTATCCGTTCGCAGAATCAACGCAACTATGTT
~~GCCGTGTTCGCGCTGTTCGTCTTGGGCGGCACGCATGCGGCGTTCCACGTCCAGCTGCAC~~
AACGGCAACCTAGGCGGACTCTTGAGCGGATTGCAGTCGGGCTTGGTGATGGTGTCGGGT
TTTATCGGTCTGATTGGTACGCGGATTATTTCGTTTTTTACGTCCAAACGCTTGAATGTG
CCGCAGATTCCCAGTCCGAAATGGGTGGCGCAGGCTTCGCTGTGGCTGCCCATGCTGACT
GCCATGCTGATGGCGCACGGTGTGTTGGCTTGGCTGTCTGCCGTTTTTGCCTTTGCGGCA
GGTGTGATTTTTACCGTGCAGGTGTACCGCTGGTGGTATAAACCCGTGTTGAAAGAGCCG
ATGCTGTGGATTCTGTTTGCCGGCTATCTGTTTACCGGATTGGGGCTGATTGCGGTCGGC
GCGTCTTATTTCAAACCCGCTTTCCTCAATCTGGGTGTGCATCTGATCGGGGTCGGCGGT
ATCGGCGTGCTGACTTTGGGCATGATGGCGCGTACCGCGCTTGGTCATACGGGCAATCCG
ATTTATCCGCCGCCCAAAGCCGTTCCCGTTGCGTTTTGGCTGATGATGGCGGCAACCGCC
GTCCGTATGGTTGCCGTATTTTCTTCCGGCACTGCCTACACGCACAGCATCCGCACCTCT
TCGGTTTTGTTTGCACTCGCGCTTTTGGTGTATGCGTGGAAGTATATTCCTTGGCTGATT
CGTCCGCGTTCGGACGGCAGGCCCGGTTGAGACAAACCGCCGCAGATTTCGGGTCTGGGC
GGTTTGCTTTTCAGACGGCAGGGCGGTCAGTTGCCGTCCAGCCAGCGGTCGCGTGTGGTT
TTGGCTTCTTCAAAATAGCGGTACAGGGCTTCGCGGTCGTCGGTGGTCAGGATGTTTGCC
AAAACGTCCAACTGTTTGCCCAAGCCTTGAACCAGTTGCAGCAGGCTGTCTTTGTTGGCA
AGGCAGATGTCCGCCCACACGGCGGGATGACCGGAGGCGATGCGGGTGAAGTCCCGAAAG
CCCGTGGCGGCGAATTTCAGATATTCCTGTCCGTCGGGGTGGTCGAGAATCTGGTGGACA
TAGGCGAAGGCGGTCAGGTGGGGCATATGGGAGACGGCGGCGAAAACCGCGTCGTGGCGT
TGCGCGTCCATCGTATAAATTTCCGCACCGACCGCGTGCCACAGGTTTTCTACCAAGGCA
ATGCCGTCTGAATGTTCGCCGCCGTGTGGCGTGATGATGAGTTTTCTGTGGCGGAACAGC
CCGAACTGCGCGGCTTGCGCACCGCTTCTGTCCGAACCGGCAATTGGGTGGGCGGCGATG
CAGTGGTGCAGGCGGTCGGGCAGACAGCGGCGGAAGGCTTCGATGACCGAAGATTTGGTG
CTGCCGACATCGGAAATCCAAGTGTGTTCCGGCAAAACGGGGCGCAGCGCGGTCAAAATG
GCGGGAACGGTGGCGACGGGCGTGGCAATCAGTACCAAGTCCGCACCGCCGATGCTGTCC
GCGTCGATGGCAACGGAAGCCTGGTCAATCACGCCGCGTTCCAATGCACGTTCGAGGTTG
TCGCGGTCGGTGTCGATACCGGTAACGGTGCGGACGAGTCCCTGCCTTTTGAGGTCGAGA

## Appendix A

ACGAACGAACCGCCGATCAGCCCTACACCGATGAGGGCAATATGGTTCAAAATGGGCATT
TGTGTAAACGGTTTTCGCAAAGTACCGTCATGGTAGCCTATCGGCGGAATATGCCGCAAG
GTCGGCAGGAAAAAGGAGAAGAAATGGACAAAATCAGAGTTGCCGCCGTGCAGATGGTGT
CGGGCGTGTCGCCGGAAACCAACGTCGCCGCCATGAAACGCCTGGTCGCACGGGCGGCGG
AGCAGGGTGCGGATTGGGTGCTGCTGCCCGAATATTGGGTGCTGATGGGCGCAAACGATA
CCGACAAACTCGCGCTTGCCGAGCCTTTGGGCGGCGGACGCTTTCAGACGGCATTGAGCG
AAACGGCGAAAGAATGCGGCGTGGTGCTGTTCGGCGGGACTGTGCCGCTGCAAAGCTGCG
AGGCGGGTAAAGTGATGAATACGCTGTTGGTGTACGGACGGGACGGCGTAAGGACGGGGC
TGTACCACAAAATGCACCTCTTCGGTTTTTCCGGTTTGGGCGAACGCTATGCCGAAGCCG
ATACCATCCGCGCGGGCGGGGATGTGCCGCACTTGTCGGCAGAAGGCGTGCCGGTGGCGG
CGGGCATTTGTTACGATGTCCGCTTTCCCGAATTTTTCCGACGCCAGTTGCCGTTTGACG
TATTGATGCTGCCCGCTGCGTTTACGCACACGACGGGCAAGGCGCATTGGGAGCTGCTGC
TGCGCGCGCGTGCCGTCGAAAACCAATGTTACGTCGTGGCGGCGGCACAGGGCGGTTTGC
ACGAAAACGGACGGCGCACGTTCGGACACAGCATGATTGTCGATCCGTGGGGCGACGTGT
TGGACGTATTGCCCGAGGGCGAAGGCGTTGTTACGGCAGACATCGATGCCAACCGCCTGA
ACAGCGTCCGCAACCGCCTGCCCGCCTTGAAATACCGGGTTTTGGATGCCGTCTGAAGGT
TCAGACGGCATCGGTGCCGGGGAATCAGAAGCGGTAGCGCATGCCCAATGAGACTTCGTG
GGTTTTGAAGCGGGTGTTTTCCAAGCGTCCCCAGTTGTGGTAACGGTATCCGGTGTCCAA
GGTCAGCTTGGGCGTGATGTCGAAACCGACACCGGCGATGACACCAAGACCCACGCTGCT
GATGCTGTGGCTTTCGTGATAGGGAGGTTTGCTGGGATCAGTTTGTATAATAGGGCCTCC
CTGTGGAGAGCCGTTCTTTGGTTTAGAGGTAATAGTCGTGGTTTTTGTTTCCACCGAATG
AACTTGATGTTTAACGTGTCCGTAGGCGACGCGCGCGCCGATATAGGGTTTGAATTTATC
GTTGAGTTTGAAATCGTAAATGGCGGACAAGCCGAGAGAAGAAACGGCGTGGAAGCTGCC
GTTTCCCTGATGTTTTGTTTGGGTTTCTTTGTAGTTGTTGTTTATCTCTTCAGTAACTTT
TTTAGTAGAAGAATTACTTTCTTTCCATTTTCTGTAACTGGCATAATCTGCCGCTATTCT
CCAGCCGCCGAAATCATAGCCGACCGACACCCGGGGGTGGATGGAATGCGCACGGATGTT
TCTGAAATAATCGCTTACCGTGCTTGTGTTGTTTGCACCGGTTGCTTGCGGATAATCGTG
GGTAATGCGTTCGGCGGCATAAGCTAAATCCGCCTGCACATAATACGGGCTGCGGCTGCC
GTCTTCACTTGCCGCCTGCGCTGCGGAAGAGAAGAGAAGAGAAGAGAAGAGAAGAGAAGA
GAAGAGAAGAGAAGAGAAGGTTTTTGGGGGCTGGATTCATTTTCGACTCCGTATTCGGT
TTTAACTGATTAAAAAGAAAGATTTTCACTGATGTTGCAGGGGTGGATTGTATCGGGTTT
GGGGCGATGTTTCAACACAATATAGCGGATGAACAAAAAAGAGAACGATGCTCTAAGGTG
CCCAAGCACCAAGTGAATCGGTTCCGTACTATAGTGGATTAACAAAAACCAGTACAGCGT
TGCCTCGCCTTAGCTCAAAGAGAACGATTCTCTAAGGTGCTGAAGCACCGAGTGAATCGG
TTCCGTACTATTTGTACTGTCTGCGGCTTCGTCGCCTTGTCCTGATTTTTGTTAATCCGC
TATAAAGACCGTCGGGCATCTGCAGCCGTCATTCCCGCGCAGGCGGGAATCTAGACCTTA
GAACAACAGCAATATTCAAAGATTATCTGAAAGTCTGAGATTCTAGATTCCCACGAAAGT
GGGAATCCAGGATGTAAAATCTCAAGAAACCGTTTTATCCGATAAGTTCCTGCACTGACA
GACCTAGATTCCCGCCTGCGCGGGAATGACGGGATTTTAGGTTTCTGATTTTGGTTTTCT
GTCCTTGTGGGAATGACGGGATGTAGGTTCGTAGGAATGACGTGGTGCAGGTTTCCGTGC
GGATGGATTCGTCATTCCCGCGCAGGCGGGAATCTAGACCTTAGAACAACAGCAATATTC
AAAGATTGGCGGATTCGCATTTGAAGTGCAACTTTCCCTAACAGAAAAAGGCCAGTATGC
GGTAGCATACGGCCTTTCCTGCAAGAAAGATTGCCATGAGCTACACGCAACTGACCCAAG
GCGAACGATACCACATCCAATACCTGTCCCGCCACTGCACCGTCACCGAAATCGCCAAAC
AGCTGAACCGCCACAAAAGCACCATCAGCCGCGAAATCAGACGGCACCGCACCCAAGGGC
AGCAATACAGCGCCGAAAAAGCCCAGCGGCAAAGCCAGACTATCAAACAGCGTAAGCGAC
AACCCTATAAGCTCGATTCGCAGCTGATTCAGCACATCGACCCCCTTATCCGCCGCAAAC
TCAGTCCCGAACAAGTATGCGCCTACCTGCGCAAACACCACCAGATCACGCTCCACCACA
GCACCATTTACCGCTACCTTCGCCAAGACAAAAGCAACGGCAGCACGTTGTGGCAACATC
TCAGAATATGCAGCAAACCCTACCGCAAACGCTACGGCAGCACATGGACCAGAGGCAAAG
TACCCAACCGTGTCGGCATAGAAAACCGACCCGCTATCGTCGACCAGAAATCCCGTATCG
GCGATTGGGAAGCCGACACCATTGTCGGCAAAGGACAGAAAAGCGCATTATTGACCTTGG
TCGAACGCGTTACCCGCTACACCATCATCTGCAAATTGGATAGCCTCAAAGCCGAAGACA
CTGCCCGGGCAGCTGTTAGGGCATTAAAGGCACATAAAGACAGGGTGCACACCATTACCA
TGGATAACGGCAAAGAGTTCTACCAACACACCAAAATAACCAAAGCATTGAAAGCGGAGA
CTTATTTTTGTCGTCCTTACCATTCTTGGGAGAAAGGGCTGAATGAGAACACCAACGGAC
TCATCCGGCAATACTTCCCCAAACAAACCGATTTCCGTAACATCAGTGATCGGGAGATAC
GCAGGGTTCAAGATGAGTTGAACCACCGACCAAGAAAAACACTTGGCTACGAAACGCCAA
GTGTTTTATTCTTGAATCTGTTCCAACCACTAATACACTAGTGTTGCACTTGAAATCCGA
ATCCAAGATTATCTGAAAGTCTGAGATTCTAGATTCCCACTTTCGTGGGAATGACGGGAT
TTTAGGTTTCTGATTTTGGTTTTCTGTCCTTGTGGGAATGACGGGATGTAGGTTCGTAGG
AATGACGTGGTGCAGGTTTCCGTGCGGATGGATTCGTCATTCCCGCGCAGGCGGGAATTT
GGAATTTCAATGCCTCAAGAATTTATCGGAAAAAACCAAAACCCTTCCGCCGTCATTCCC
ACGAAAGTGGGAATCTAGAAATGAAAAGCAGCAGGCATTTATCGGAAATGACCGAAACTG
AACGGACTGGATTCCCGCTTTTGCGGGAATGACGGCGACAGGGTTGCTGTTATAGTGGAT
GAACAAAAACCAGTACGGCGTTGCCTCGCCTTAGCTCAAAGAGAACGATTCTCTAAGGTG
CTGAAGCACCAAGTGAATCGGTTCTGTACTATTTGTACTGTCTGCGGCTTCGTCGCCTTG
TCCTGATTTTTGTTCATCCGCTATACTTTTGTATGACCATCTGACTTTATCACTCACTAT
GTTTTACCAAATCCTTGCCCTGATTATCTGGAGCAGCTCGTTTATTGCCGCCAAATATGT
CTATGGCGGCATCGATCCCGCATTGATGGTCGGCGTGCGCCTGCTAATTGCCGCGCTGCC
TGCACTGCCCGCCTGCCGCCGTCATGTCGGCAAGATTCCGCGTGAGGAATGGAAGCCGTT
GCTGATTGTGTCGTTCGTCAACTATGTGCTGACCCTGCTGCTTCAGTTTGTCGGGTTGAA
ATACACTTCCGCCGCCAGCGCATCGGTCATTGTCGGACTCGAGCCGCTGCTGATGGTGTT
TGTCGGACACTTTTTCTTCAACGACAAAGCGCGTGCCTACCACTGGATATGCGGCGCGGC
GGCATTTGCCGGTGTCGCGCTGCTGATGGCGGGCGGTGCGGAAGAGGGCGGCGAAGTCGG

# Appendix A

CTGGTTCGGCTGCCTGCTGGTGTTGTTGGCGGGCGCGGGCTTTTGTGCCGCTATGCGTCC
GACGCAAAGGCTGATTGCACGCATCGGCGCACCGGCATTCACATCTGTTTCCATTGCCGC
CGCATCGTTGATGTGCCTGCCGTTTTCGCTTGCTTTGGCGCAAAGTTATACCGTGGACTG
GAGCGTCGGGATGGTATTGTCGCTGCTGTATTTGGGTTTGGGGTGCGGCTGGTACGCCTA
TTGGCTGTGGAACAAGGGGATGAGCCGTGTTCCTGCCAATGTTTCGGGACTGTTGATTTC
GCTCGAACCCGTCGTCGGCGTGCTGCTGGCGGTTTTGATTTTGGGCGAACACCTGTCGCC
CGTGTCCGCCTTGGGCGTGTTTGTCGTCATCGCCGCCACCTTGGTTGCCGGCCGGCTGTC
GCATCAAAAATAAAGTTGGGAAGCGGTATTTGATGATTGCCGAATAGGCTGAAATCTTTC
CATCTCCATTCCTGCGAAAGCGGGTATCCGGAACGAAAAGACGGATATTTATCCGAAATA
ACGACCATCTTTGCGTCGTCATTCCCGCGCAGGCGGGCATCCGGTTTTTTGAGTTTCGGT
TATTTCCGACAAATTGCTGCAGCGTTGGATGTCCGGATTTCCGCCTGCGCGGGAATGACG
GGATTTTATAGTGGATTAACAAAAATCAGGACAAGGCGGCGAGCCGCAGACAGTACAGAT
AGTACGGAACCGATTCACTTGGTGCTTCAGCACCTTAGAGAATCGTTCTCTTTGAGCTAA
GGCAAGGCAACGCTGTACTGGTTTTTGTTAATCCACTATATCGTTCCGGTTCGTCCGGTT
TTGCCGGGGCTTTTGTTGCCGCCTGTTTGTGCCGGTGTGTTAAAATTTTCCGTTTCCGCG
TATTGTGTTTTCCGCCGCCGGGCGGTTTGTTTGCGAATCGGACGAGAATTTATGCCTTCT
GCCCATTATCCTGAAATGAGCGAAAAACTGATGGCGGTTTTGATGGCGATGCTGGTTACG
CTGATGCCGTTTTCCATCGATGCCTACCTGCCCGCGATTCCCGAAATGGCGCAATCGCTG
AACGCGGATGTTCACCGCATCGAACAGAGTTTGAGTTTGTTTATGTTCGGCACGGCGTTC
GGACAGGTGGTCGGCGGTTCGGTGTCCGACATCAAAGGGCGCAAACCCGTCGCCCTGACC
GGTTTGATTGTATATTGCCTTGCCGTTGCCGCCATCGTATTTGTTTCGAGTGCCGAACAG
CTCCTCAACCTGCGCGTCGTGCAGGCATTCGGTGCGGGCATGACTGTGGTCATCGTCGGC
GCAATGGTGCGCGATTATTATTCCGGACGCAAAGCCGCCCAGATGTTTGCCCTTATCGGC
ATCATTTTGATGGTTGTGCCGCTGGTCGCACCCATGGTCGGCGCATTGTTGCAGGGCTTG
GGTGGCTGGCAGGCGATTTTTGTTTTTCTGGCGGCGTATTCGCTGGTGCTGCTCGGTTTG
GTACAGTATTTCCTGCCCAAGCCCGCCGTCGGCGGCAAAATCGGACGGGACGTGTTCGGG
CTGGTGGCGGGGCGGTTCAAGCGCGTATTGAAAACCCGTGCTGCGATGGGTTATCTGTTT
TTTCAGGCATTCAGCTTCGGTTCGATGTTCGCCTTTCTGACCGAATCTTCCTTCGTGTAC
CAGCAGCTCTACCGTGTTACGCCTCATCAATACGCTTGGGCGTTTGCACTCAACATCATC
ACGATGATGTTTTTCAACCGCGTTACCGCGTGGCGGCTCAAAACCGGCGTGCATCCGCAA
AGCATCCTGCTGTGGGGGATTGTCGTCCAGTTTGCCGCCAACCTGTCCCAACTCGCCGCC
GTGCTGTTTTTCGGGTTGCCCCCGTTTTGGCTGCTGGTCGCGTGCGTGATGTTTTCCGTC
GGTACGCAGGGCTTGGTCGGTGCAAACACGCAGGCGTGTTTTATGTCCTATTTCAAAGAA
GAGGGCGGCAGCGCAAACGCCGTATTGGGTGTATTCCAATCTTTAATCGGCGCGGGGGTG
GGTATGGCGGCGACCTTCTTGCACGACGGTTCGGCAACCGTGATGGCGGCAACGATGACC
GCGTCCACCTCTTGCGGCATTGCGCTTCTGTGGCTCTGCTCGCATCGTGCGTGGAAAGAA
AACGGGCAAAGCGAATACCTTTAACGGAAAATGCCGTCTGAAACCGTTTCAGACGGCATT
TGATGTTAGAATGCACGATAAATTACTGTTCAGGCGAAATTATGTCCCAAACTATCGACG
AACTCCTCCTTCCCCACCGCAACGCCATCGACACCATCGATGCCGAAATCCTGCGCCTGC
TCAACGAACGTGCGCAACACGCCCACGCCATCGGCGAGCTGAAAGGCACGGGCGCAGTGT
ACCGCCCCGAACGCGAAGTCGCCGTGTTGCGCCGCATTCAGGATTTGAACAAAGGCCCGC
TGCCCGACGAATCGGTAGCACGCCTGTTTCGGGAAGTGATGAGCGAGTGCCTCGCCGTCG
AACGCCCGCTGACCATCGCCTATCTGGGGCCGCAGGGCACGTTTACCCAGCAGGCGGCAA
TCAAACATTTCGGACACGCCGCGCACACCATGGCGTGTCCGACCATAGACGACTGCTTCA
AGCAGGTTGAAACGCGTCAGGCGGATTATCTGGTCGCCCCCGTGGAAAATTCGACCGAAG
GCTCGGTCGGTCGCACGTTAGACCTGCTTGCCGTTACCGCGTTGCAGGCGTGCGGCGAAA
TCGTTTTGCGCATCCACCACAACCTTTTGCGTAAAAACAACGGCAGCACCGAAGGCATTG
CCAAAGTCTTTTCCCACGCGCAGGCGTTGGCGCAGTGCAACGACTGGTTGGGCAGACACC
TGCCCAACGCCGAACGGATTGCCGTGTCCAGCAATGCCGAAGCCGCAAGGCTGGTTGCCG
AATCGGACGACGGTACGGTTGCCGCCATCGCCGGACGCACGGCGGCGGAAATCTACGGAC
TCGATATGGTTGCCGAGTGCATCGAAGACGAACCGAACAACACCACGCGCTTCTTGGTGA
TGGGACATCACGAAACCGGTGCAAGCGGCAGCGACAAGACTTCGCTGGCCGTTTCCGCGC
CCAACCGGGCAGGCGCGGTTGCCTCGCTGCTGCAACCGCTGACCGAATCGGGTATTTCCA
TGACCAAGTTTGAGAGCCGTCCGAGCAAATCCGTTTTGTGGGAATACCTGTTCTTCATCG
ACATCGAAGGACACCGCCGGGACGCGCAGATTCAGACGGCATTGGAACGCTTGGGCGAAC
GCGCTTCGTTCGTCAAAGTCATCGGTTCGTACCCGACCGCCGTTTTGTAGCGGCGGCAGC
GTTCAGACGGCATTTCCCCAACGATTATGTCCGAATACCGAGTCAACCATGAACCCGTTT
TTATGCTGGCATCTTCGCCCTGGCGCGAAAGCAGCCTGTGGGTTGAAGCATTCAGCCGCC
GTTACGGGCGTGTGGCTTTGCTGGCGCGCAGCGCGCGCAAAAGGCAGAGCGAGCTGCGCG
GCGTATTGGTGCCGTTCGTGCCCGTCAGCGTGTCGTGGTACGGCAGTCAGGAACTCAAAA
CCCTACACCGCGCCGAATGGGTCGGCGGTTGGCGGCAGCCTCAGGGCAGGGCGTTGTTCG
GCGGATTGTATGTGAACGAGTTGGTGTTGAAACTGACCGCCCGCGAAGACCCGGTGCCCG
AGTTATACGACGCGTTGGCGGAAGTGATGGAGGCGGTGTGCTGCAAAGCCGCTTATATCG
ACGACTTGCGCCGTTTCGAGTGGCGGCTGCTGAACCTGTTGGGCGTTGCCCCCGATTTGA
ACCGCGACGGGGACGGCGGGACGATTGCGGCAGGCGGCACATACCTTGTCCGCCCGGAAA
CAGCCGTCTTCCCCGTCGGAAAAGGATTTGCCGTACCGCCGCACGCCGCCGGCGTTGTCG
CCCCCGGGCAGAGCCTGATCGATTTGCGCGAAGGCAGTTTCCGCACTGCCGAAAGCCTGC
AACAGGCATTGAAAATCACACGGCTTTTTATCCGCCACCTGTTGCCCGAGGGGCTGAAAT
CGCGGCAGGTGTTGGAACAGATACGGCAGTTTGACCGCAAAGAAACCGCCCGGGAAACCG
TCCCGACTTCGGACGGCACGGCTTCAAATGCCGTCTGAAGGCAGAAATAAAAGGAAAGAT
TATGCTTTTAGGTGTCAACATCGACCACATCGCCACCGTCCGCAATGCGCGCGGTACGAC
TTATCCCAGCCCCGTGGAGGCGGCACTGGTTGCCGAAACGCACGGTGCGGATTTGATTAC
CATGCACCTGCGCGAAGACCGCCGCCACATCAAAGACGCGGACGTGTTTGCCGTCAAAAA
CGCCATCCGCACGCGCCTGAACCTTGAAATGGCGTTGACGGAAGAAATGTTGGAAAACGC
TTTGAAAGTGATGCCGGAAGACGTGTGCATCGTGCCTGAAAAACGTCAGGAAATCACGAC

# Appendix A

CGAAGGCGGTTTGGACGTATTGGCGCAACAGGAAAAAATCGCCGGGTTCACCAAAATCCT
GACCGACGCAGGCATACGCGTGTCTTTGTTTATCGATGCCGACGACAGGCAAATCCAAGC
CGCCCGTGATGTCGGCGCGCCCGTTGTCGAGCTGCACACAGGCGCGTATGCCGACGCGCG
CAGCCACGCCGAACAAATCAGGCAGTTCGAGCGCATCCAAAACGGCGCGCATTTCGCCGG
CGATTTGGGCTTGGTCGTCAACGCCGGACACGGACTGACCATACACAACGTTACCCCCAT
CGCCCAAATCCTCGCCATCCGCGAACTGAACATCGGGCATTCGCTGATTGCCCAAGCCCT
CTTCCTCGGACTGCCCGAAGCCGTGCGCCAAATGAAGGAGGCGATGTTCAGGGCAAGGCT
GCTGCCGTAAGGGCAGGCAAACCCTTTCAGACAGCATTTCACGACAGGGATATGTTATAG
TGGATTAAATTTAAATCAGGACAAGGCGGCGAAGCCGCAGACAGTACAAATAGTACGGCA
AGGCAAGCCAACGCCGTACTGGTTTAAATTTAATTCACTATATGAATCAAAAGTATATTT
TATCTGCAAACAATAATAGTTTGATAGAAGAAATTCACAATACAGTACAGAGTATTGGGT
ATTGTATTGTTCGAGGTCTTAATCTAAACCATCTTGATGGCAGCCGGAGAAACAAGAAAT
TATTTGACTTTCTATCTCAATTAGGAATGCTGACAAACCACAAAGGCGATGGTTTTAAAT
CTATATTTTGGGATATTAAATATTGAGGCGATGATTATGTAATATAGTGGATTAACAAAA
ATCAGGACAAGGCGACGAAGCTGCAGACAGTACAGATAGTACGGAACCGATTCACTTGGT
GCTTCAGCACCTTAGAGAATCGTTCTCTTTGAGCTAAGGCGAGGCAACGCCGTACTGGTT
TTTGTTAATCCACTATAAATAATGATATAACTTTCTCGGAAGATGTTGGAGAATGTCCAC
TTCATAGTGATTCATCTTTTAGTGAAAACCCGGAAAGTTATTTGGTTATGTATGTAGTAA
AATCAGCCAATGATGGAGGTAATTCCCTATTTTTAAGTTCATCAGATATTGTCAATCAGT
TATCTAAAACAGAAACCGGTAAAAAACACTTAAAAACATTAACGGGCAATTTATATCCAT
TTAAAACACCAGCATCATTTGATAAAAAACAAGGTGTGAGATGGGGTAATATCTTATCGG
TCAATACTCAAATGATTAGATTTAGAAGTGATTGTATCTATAAAGGTATTGAAGAAAATA
GAAATAAAGTATCAAAGGAAATGGTACTTGCACTTGATTATCTTATAAATGTTATAAAAA
ATGCGAGTGATATTCAAGAATTTTCTGCACAAGATGATGGTTTGATTATTATTGACAATG
TCAATGGCTTGCATGCCAGAACTGATTATACGGATAAAAACAGGCATTATATTAGAGCAA
GAATTACTGTATAAAGGACGGTTATGCAAGAAATAATGCAATCTATCGTTTTTGTTGCTG
CCGCAATACTGCACGGAATTACAGGCATGGGATTTCCGATGCTCGGTACAACCGCATTGG
CTTTTATCATGCCATTGTCTAAGGTTGTTGCCTTGGTGGCATTACCAAGCCTGTTAATGA
GCTTGTTGGTTCTATGCAGCAATAACAAAAAGGGTTTTTGGCAAGAGATTGTTTATTATT
TAAAAACCTATAAATTGCTTGCTATCGGCAGCGTCGTTGGCAGCATTTTGGGGGTGAAGT
TGCTTTTGATACTTCCAGTGTCTTGGCTGCTTTTACTGATGGCAATCATTACATTGTATT
ATTCTGTCAATGGTATTTTAAATGTATGTGCAAAAGCAAAAAATATTCAAGTAGTTGCCA
ATAATAAGAATATGGTTCTTTTTGGGTTTTTGGCAGGCATCATCGGCGGTTCAACCAATG
CCATGTCTCCCATATTGTTAATATTTTTGCTTAGCGAAACAGAAAATAAAAATCGTATCG
TAAAATCAAGCAATCTATGCTATCTTTTGGCGAAAATTGTTCAAATATATATGCTAAGAG
ACCAGTATTGGTTATTAAATAAGAGTGAATACGGTTTAATATTTTTACTGTCCGTATTGT
CTGTTATTGGATTGTATGTTGGAATTCGGTTAAGGACTAAGATTAGCCCAAATTTTTTTA
AAATGTTAATTTTTATTGTTTTATTGGTATTGGCTCTGAAAATCGGGCATTCGGGTTTAA
TCAAACTTTAATTCATTATTAAATGCCTTAACTCCTTATTAAATAATTGGCACGATGTTT
TAGAATTTCAAATGCAAAAGGTTACAGTGAAAATTGTTACCGACAAAACCCCAAAAGTGG
ATATTCACGCCATTTTAACGCCCCAAGAAATTGACGGCATTCATCATCACATTCATCACT
ACCCGCAACCAAGGGCGAAGGAGCGCAAATATGATTTACGGCATCGGCACAGACATTGTT
TCCCTCAAGCGCATCATCCGCTTAAACAAAAAATTCGGACAGGCGTTTGCCGGGCGCATC
CTCACTCCGGAAGAGCTGCTTGAATTTCCGCAAGCGGGCAAACCCGTCAACTACCTCGCC
AAACGCTTTGCCGCCAAAGAAGCCTTTGCCAAAGCCGTCGGCACGGGCATACGCGGCGCG
GTTTCCTTCCGCAACATCGGCATCGGGCATGACGCATTGGGCAAGCCCGAATTTTTCTAC
GGCCCCGCCCTGTCCAAATGGCTGGAGGAACAAGGCATCAGCCGCGTCAGCCTCAGCATG
AGCGACGAAGAAGACACCGTATTGGCGTTTGTCGTTGCCGAAAAATAATGCCGTCTGAAA
TGCGGCAAACCCGTTGACGGCATTGCCCGTCCCTCATTTGCACTCCGACCGACCAACCGC
GTACCGGCCATGATTCAAGACACCCGACCCCTTATCCGCGTCGTTGCCGGCATCCTGCTC
GATTCAGACGGCAACTACCTGCTCAGCTCGCGCCCCGAAGGCAAACCCTATGCCGGATAT
TGGGAATTTGCCGGCGGCAAGGTCGAAGCGGGCGAAACCGACTTCCAAGCCCTGCAACGC
GAGTTTGAAGAAGAACTCGGCATCCGCATCCTCGCCGCCACGCCTTGGTTGACCAAAATC
CATTCCTACGAACACGCCCGCGTCTGCCTGAAATTCCTATGGGTCAACCCCGACCAATGG
ACGGGCAAACCGCAATCCCGCGAAGGGCAGGAATGGTCTTGGCAGAAGGCGGGTGATTTT
ACCGTTGCCCCCATGCTGCCCGCCAACGGCGCGCTTTTGCGTTCGCTGTCCGTCCCGCGC
CGTTTGTACGGCAGCCTGAAAACGGGTTTGCACGGAGAAAACAGTATGGGCGCGTACCGC
GTCCTGCCTTTGGGTTCGGCAGAGGGAAGCGGTGCGAACGTTTTGATGGAGGCGGCGCAA
TGGCAGGACAGACCCGAACACGCCGACAGCGTGTGGATGGTGGTGCAGACCCGCGAACAA
TGGCGGCGGGCGCAGGAAAAGGGCGCGGATGCGGTCGTTTGGCGCGTGTGCGATGATGTT
CAGGCACAAGAGGCGGCAGAAGCCCTGCGGCAGGGCGTATCCGTGCCGCTCGTACTTGCA
GCAAACGGACAGACGGTTGCACGTTATGGAAAACTATGGCTCGGATTGGGGGCGCACGTG
GTGGTAAGGGATGAAACAATAGGGAAGAATCATGAATAAAAACCGTAAATTACTGCTTGC
CGCACTGCTGCTGATTGCCTTTGCCGCCGTCAAGCTCGTTTTGTTGCAATGGTGGCAGGC
GCAGCAGCCGCAAGCTGTGGCGGCGCAATGCGATTTGACCGAGGGTTGCACGCTGCCGGA
CGGAAGCCGCGTCCGCGCCGCCGCCGTTTCAACCAAAAAACCGTTTGATATTTATATCGA
ACACGCGCCCGCCGGCACGGAACAGGTCAGCATCAGCTTCAGTATGAAAAATATGGATAT
GGGTTTCAACCGCTATATGTTCGAGCGGCAACCGTCGGGGACTTGGCAGGCAGTACGCAT
CCGCCTGCCCATCTGTGTCGAAGGCAGGCGCGATTTTACGGCGGACATTACAATCGGCAG
TCGGACATTTCAGACGGCATTTACCGCCGAATAAACCTTTCAATCCGCCATTGCCGGAAC
ATCCGTCCGGAAAGGACACGTTATGAATACTTTATATACACTTTTCGCCACCTGCCCGCG
CGGCTTGGAGACCGTTTTATCTCAAGAACTCGAAAGCCTCGGCTGTACCGATGTACAAGT
GTTTGACGGCGGCGTTTCCTGCCGGGGCGGATTGGAACAGGTTTACGCCGCCAACCTGCA
TTCGCGTACTGCCAGCCGTATCCTGCTGCGCCTGACCAAAGGACATACCGCAATGAGCG
CGACATCTACAAACTCGCCAAAAATATCAACTGGTTTAATTGGTTTACTTTACAGCAGAC

## Appendix A

GTTCAAAGTCAAAGTCGAGGCAAAGCGTGCCAACGTTAAGAGCATCCAATTTGTCGGACT
GACCGTCAAAGATGCCGTCTGCGACGCTTTCCGCGACATTTACGACGCACGTCCGAGCGT
GGACAAAGCCGCGCCCGATGTCCGCATCCACGCCTTTTTGAACGAACGCAATGTCGAAAT
CTTTATTGACACTTCGGGCGAAGCCCTGTTCAAACGCGGCTACCGCCTGGATACCGGCGA
AGCCCCGCTGCGCGAAAACCTTGCCGCCGGACTGCTGCTCTCGGCAGGCTACGACGGCAC
GCAGCCGTTTCAAGACCCGTTTTGCGGCAGCGGCACGATTGCTATCGAAGCCGCTTGGAT
TGCCGCCCGCCGCGCGCCGGGTATGATGCGCCGTTTCGGTTTTGAAAAACTGCAAAATTT
CGATAAAACGCTGTGGTCGGATTTGCGGCGCCGCGCCGAAGCGCAAACCCGCCCCGTCCG
CGCCCCGATTGCAGGCAGCGACAACGACCGCCGCATCGTTCAGACGGCATTGGACAACGC
ACGCCGCGCCGGGGTGGACGACATCGTTTCCTTCAGCGTTGCCGACGCGCAGTCCGTCCG
ACCGAACGGCGAAAACGGCATTATGGTGTCCAATCCGCCCTACGGCGTGCGCCTTGAGGA
AGTCCGCGCCTTGCAGGCACTGTATCCGCAGTTGGGGACGTGGTTGAAAAAACATTACGC
AGGCTGGTTGGCGGCAATGTTTACCGGCGATAGGGAAATGCCCAAATTCATGTGCCTGTC
GCCCAAGCGGAAAATCCCGCTTTATAACGGCAACATCGACTGCCGCCTGTTCCTGATTGA
TATGGTGGAAGGATCGAACCGTTGAGGAAAGTGTACAAAAATGCCGTCTGAAAAATGTTC
AGACGGCATTTATTTTTCGGAATCAACCCCGCTTCAATACGGATGTATTGATGTAGCGTT
GGACACCCGAGGCAATGGATTGGGCGCACTGCCGGCGGAAGGATTCGCTGCCCAGCAGCT
TCTCTTCGGCAGGATTGGACAGGAAGGCGGTTTCGACCAGGATAGACGGCATATCGGGTG
CGCGCAAAACGGCGAAATTGGCTTCGTCCACCCTGCCTTTGTGCAGATGGTTGAGCCTGC
CCAATTCTTCAAGCACCAGTTTGCCGAGTTTGCGGCTGTCGCGCAGCGTGGCGGTTTGGG
TCATGTCGAGCAGGGCGGTATCGACATTGCGGTTGCCGCTGGTCGGTACGCCGCCGACCG
CGTCGGCATTGTTTTGCGTCTGTTCCAAGAATTTGGCGGCAGAGCTGGTTGCGCCTTTGG
TGTTTAACATATAAACCCCCGTGCCGCGCGCGGAGGGGCTGGTGAAGGCATCGGCGTGGA
TGGAGACAAATACGTCCGCCCGCCGTGCTCGCCCTTTGGCGACACGCACGCCCAATGGGA
TGAACACGTCTTCGTTGCGCGTCATAAATACATTGTAACCTAATGCTTCCAACTGATTTT
TGGTTTCCCTGGCAATGGATAGGACGACATGTTTTTCCTGTAGACCGCCCGGGCTGATGG
CGCCGGGGTCTTCACCGCCGTGTCCCGGATCGAGCATGATGACGGGTCTGCGCCCGTTTC
TGCCGCGCCCGGGTTGGGGCGTGGTGTTTTGGGCGAGGTCGGCTTCGGGAGAGCCGCGCA
GGGTTTTATTCAGGCTACCGTTGAGCAGTGCCATCATCGGATCGTCGGCATCCATCCCGT
GCGGATAGAGGTCGACGACGAGGCGGTTCTTAAAGCCGCCGACGGGCGGAAGCGCGAAGA
CTTGTGCGTGGGTGGGCTGTTTCAAATCGATGACGAGGCGGACGGTGGTCGGCGTGTTCT
GACCCGCGCGTATGCTGCGGATAAAGGGGTCGTCTGCCATGACTTTCTGAGACAGTCCGT
GCAATACGGTATTGATGTTCGCGTTTTGTATGTCGACGACCAGCCTGCCCGGGTTGTCGA
GCGTGAAGTGCTGGTATTTGAGCGCGGCGGTGCTTTCCAGCGTCAGGCGGGTGTAGGTGT
GCGACGGCCATATCCGTGCGGCGGTGAATTGCGGGGCGCGTACCGTTTTGGCAACGGCGG
ATGCGATGGGGCTTAGGGCGAACAGTGTGCCGGCGGTGCGGCGGATGATTTGTCTTCGTG
TCAGTTTGATCATAGCGGCAGGCTTTCGCGTCCTCGTTCGGTATGGGCGGTCAGCAGGCA
TTTTCTGCCGTCGCCGTCGTGTGTCAATGTTGCGGTGATGTCGGCGGGCGGCGTAAATTC
CCCGCCCTGTTGCGGCCATTCGATCAGGCAGACGCTGTTTGCGGCAAACAGTTCGTCAAG
CCCCGCGTCTTCCCATTCTTCGGGGAACGAGAAGCGGTAGAGGTCGAAATGGTGCAGGGT
GAAGCGTTCCAGCGGATAAGATTCGACGATGGCGTAGGTCGGACTTTTGACTGCGCCCTG
ATGACCCAATCCGCGCAGGATGCCGCGTGTCAGCGTGGTTTTGCCCGCACCCAAATCCCC
TTCGAGATAAATGACCAGCGGTGCGTTTAAACGGGAAGACCACGCCGCGCCCAAATCGAG
TGTGGCGGCTTCGTCGGCAAGGAATCGGGAGATAGAGGGTAAATCAGACATGGAAACGGT
TTGTTGTAAGGTCTAGGGTATTATGGGCAGTTTTGCAGGTTTTGCAAACTTTGCACCCGA
GGGGCGGATGCTTCTTGTCCGAGCATTATAACAGCCAAATCCGCGTTCTGCTTTCAGACG
GCAACGGCTGTCAAGAAAAAGCGGCGCGTGTACAATACGCGGATTGTATGTTTAGGACGG
ATTGGAAAAAGAATGGAAAATATCGGCAGGCAGCGACCCATCGGCGTTTTTGACTCGGGA
ATGGCGGTTTGACCAATGTGCGAGCGCTGATGGAACGGCTGCCGATGGAGAACATCATT
TATTTCGGCGACACGGCGCGCGTGCCTTACGGGACGAAATCTAAGGCGACCATCGAAAAT
TTCTCGATGCAGATTGTCGATTTTTTATTGGAACACGATGTCAAGGCGATGGTTATCGCG
TGCAATACGATTGCGGCGGTGGCGGGGCAGAAAATCCGTCAAAAAACCGGCAATATGCCC
GTTTTGGACGTGATTTCCGCCGGCGCGAAAGCCGCGCTGGCAACGACGCGCAACAATAAA
ATCGGCATTATCGCCACCAATACGACAGTCAACAGCAATGCTTATGCGCGCGCCATCCAT
AGGAACAACCCCGACACGCTCGTCCGCACGCAGGCCGCGCCGCTGCTCGTCCCTTTGGTG
GAAGAGGGCTGGCTGGAACACGAAGTTACCCGCCTGACCGTATGCGAATACCTCAAACCA
TTGCTTGCAGACGGCATCGATACGCTGGTGTTGGGCTGCACGCACTTTCCCTTGCTCAAG
CCCTTAATCGGCAGGGAGGCGGGCAATGTCGCGTTGGTTGATTCTGCAATTACAACGGCC
GAAGAAACCGCACGCGTCCTTGCTCAGGAAGGATTGCTCAATACCGACAACAACAATCCC
GACTACCGTTTTTACGTCAGCGATATTCCTTTGAAATTCAGAACCATCGGCGAGCGTTTT
CTGGGCAGGACGATGGAGCAGATTGAAATGGTGTCTTTGGGTTAAAACGATGACGGAAAG
CTGCCCGAGATTACAGAAACCTAAAATCCCGTCATTCCCACGAAAGTGGGAATCTAGACC
TGTCGGTGCGGAAACTTATCGGATAAAACGGTTTCTTTAGATTTTACGTTCTAGATTCCC
ACTTTCGTGGGAATGACGGGATTAGAGTTTCAAAATTTATTCTAAATAGCTGAAGCTCAA
CGCACTGGATTCCCGCCTGCGCGGGAATGACGAATTTCAGGTTTCTGTTTTTGGTTTTCT
GTTTTTGTGAAAATAACGGGATTTCAGCTTGTGGGTATTTACCGGAAAAAACAGAAACCG
CTCCGCCGTCATTCCCGCGCAGGCGGGAATCTAGACATTCAATGCTAAGGCAATTTATCG
GGAATGACTGAAACTCAAAAAACTAGATTCCCACTTTCGTGGGAATGACGGAATGTAGGT
TCGTGGGAATGACGGGATGCAGGTTTCCGTATGGATGGATTCGTCATTCCCGAGCAGACG
GGATCTAGACATTCAATGCTAAGGCAATTTATCGGGAATGACTGAAACTCAAAAAACTAG
ATTCCCACTTTCGTGGGAATGACGGGATATAGGTTTCCATGCGGACGCGTTCGGATTCAC
GACTGCGCGGAAATGACGGGATTTTGGTGTATTCCCTAAAAAAATAAAAAAACATTTGCA
ACTTTGTTAAAAATAAAGGCTGTGTTTTAACGATGTGTTGATATTTAATTTTAGAAAGGT
AGCTATTTAATAGTTACCTTTTCTTATTTAAAAATAGCTTTCTCAAATTCCATGAACGCC
TCAATACGATATGCAGATGCTCTATCGAAATTAAGTTTCAACATTTTGTTTATTAAACAT

## Appendix A

TTTATTTTAGCCATTTTTCAATATACCCCCAAATATACCCCCAATTTGCACAAGTCAAAA
TTTTAGGCAGGGGTTTTGTTGCTCCCATGATACGCGGGCGGATGCCGGCTTTTCGCGGGG
GAAATACAAGGGGTCTCGGTTCGGGTGTCAAAATCCCTGTTTCGTGTTAGTCATGTGGGG
GGGAAGAGGGGGTTAGAATGAAGTAAAGCTGTTGCCCTCTCCCCGCAATAGTTCCATTAG
GCGCGGATGAATGAATAGTTTGTCCCTGCCGATGACGATTTCTTGCAGCACACCTATGTC
TGAAAGCTCTTTCAGGTACTTAGAGGCCGTCTGCCGTTTGGCTATCCCTGCCGCTTCTAG
GTTGGCAATGCGTGTATATGGCTGCTCAAACAGAAGATTTACCAGTTCGTGCGTGTAGAT
TCCTTGTGCGTGTGTCCGTATGTGTTGCCGTGTCTGCTCGAACAGGCGGCGTATCGCATC
TATTTTCGATACCGTCCAATCGGCGGTGTCAGCTACGCCGTCTAAGATGTAGATTATCCA
GCTTTCCCAGTCCTGCCGTTCGGTTACGCCTAAAAGCAGGCGGTAATAGTCCGCCCTGTT
TTCGATGATGTAGCGGCTCAAATACAAAATAGGCAAATCCAAAAGCCCTTTTTCAATCAA
TAGCAGGCTGTTCAATATGCGCCCCGTCCGCCCGTTGCCGTCCGTAAACGGATGGATGGC
TTCAAATTGGTAATGTGCCGCCGCCATGATGATAAGCGGGTCTAAATCGCCGCTTTCGTG
AATAAACCGCTCCCAATTTGCCAGCTTGCCGCGTATGGTTTCTTCTCCTTCGGGCGGGGT
ATAGACAACATTTCCGCTGTTGCCTCCTTTTAGGGCTGTGCCGCCTGTTTTGCGGATGGC
CATTTCGTAGGGGTGCTTGATGGCGTTGCAGACCATGATGGCGGTTTGTGTGCATAAAGG
GCGGCTCGTCAGTGATTCATAGCCTGCAAACAGGGCGGTGCGGTATTGCAGGGCTTCTTT
CGTGGCAGGGTCTTGCCGTTCCGTATCCATTTGCAGGGATTGAAACAGCTTGTCCGTGGT
GGTTACGATGTTTTCAATTTCCGAACTTGCACGGGCTTCCATAACAGGAAGGGTGTTAAT
CAGCATGGCTTGATTCGGTATCAATTCTGCCGCCTGCTTTAAACGGGCAAGGGATGCACG
GGCGGCTATACAACGTTTCAGGATGGTTTTGCTTTCAATATCCTGTTTTGGCGGCAGGGG
TGGTAAATCGTTATAGGGAATATTGGGTTTCCAGTTGCTCATATTTAAAATTTCGGAAAA
TTTAAAGATGTTTCCAGTATATGTTTACGCCGTGTATATATCAAGGATATATGTTTAAAA
ATTTGGCTTTTGTAAGTATATGGAGGTAAAACCGCCCGCAAAAGTCAATTTGCATGGGG
TTAATTCAAAATCCGCCCGCCCTTCATCTGCCCGCTTCCCTAAAAAAACCGTATATATAT
AACTGTCCGCATTCTATCGCTCCGGCGACGATACCCATATTTCCAAGTTTGTGTATCAAA
ATTGTATATCGGGCATAGACTATTTCGGCGAGGACGAAGATATAGATTTCCACGATTGAA
TACATGGAAGCCAAGTACGTCTATCAACACTATATTAAAACACAGCCTTTTTTTTTGAGG
TTTCGGGTAACTTTTAAACCGTCATTCCTACGAAAACAGAAAATCAAAAACAGAAATCTC
AAATCCCGTCATTCCCGCGCAGGCGGGAATCTAGACATTCAATGCTAAGGCAATTTCTCG
GAAATGACTGAAACTCAAAAAACTGGATTCCCACTTTCGTGGGAATGACGGAATGTAGGT
TCGTGGGAATGACGTGGTGCAGGTTTCCGTATGGATGGATTCGTCATTCCCGCGCAGGCG
GGAATCTAGACATTCAATGCTAAGGCAATTTATCGGGAATGACTGAAACTCAAAAAACTG
GATTCCCACTTTCGTGGGAATGACGCGATTAGAGTTTCAAAATTTATTCTAAATAGCTGA
AACTCAACGCACTGGATTCCCGCCTGCGCGGGAATGACGAAGTGGAAGTTACCCGAAACT
TAAAACAAGCGAAACCGAACGAACTGGATTCCCACTTTCGTGGGAATGACGGAATGCAGG
TTCGTGGGAATGACGGAATGCAGGTTCGTGGGAATGACGTAGTGCAGGTTTCCGTATGGA
TGGATTCGTCATTCCCGCGCAGGCGGGAATCTAGACATGCAATGCTAAGGCAATTTATCG
GGAATGACTGAAACTCAAAAAACTGGATTCCCGCCTGCGCGGGAATGACGAAGTGGAAGT
TACCCGAAACTTAAAACAAGCGAAACCGAACGAACTGGATTCCCACTTTCGTGAGAATGA
CGGGATGCAGGTTCGTGGGAATGACGTGGTGCAGGTTTCCGTATGGATGGATTCGTCATT
CCCGCGCAGGCGGGAATCTAGGTCTGTCGGTGCGGAAACTTATCGGGTAAAACGGTTTCT
TGAGATTTTGCGTCTTGGATTCCCACTTTCGTGGGAATGACGCGATTAGAGTTTCAAAAT
TTATTCTAAATAGCTGAAACTCAACGCACTGGATTCCGCCTGCGCGGGAATGACGAAGTG
GAAGTTACCCGAAACTTAAAACAAGCGAAACCGAACGAACCGGATTCCCACTTTCGTGGG
AATGACGAATTTCAGGTTACTGTTTTTGGTTTTCTGTTTTTGTGAAAATAATGGATTTC
AGCTTGTGGGTATTTACCGGAAAAAACAGAAACCGCTCCGCCGTCATTCCCGCGCAGGCG
GGAATCTAGGTCTGTCGGTGCGGAAACTTATCGGATAAAACGGTTTCTTGAGATTTTTCG
TCCTGGATTCCCACTTTCGTGGGAATGACGCGAACAGAAACCGCTCCGCCGTCATTCCCG
CGCAGGCGGGAATCTAGACATTCAATGCTAAGGCAATTTATGGGGAATGACTGAAACTCA
AAAAACTGGATTCCCACTTTCGTGGGAATGACGTGGTGCAGGTTTCCGTATGGATGGATT
CGTCATTCCCGCGCAGGCGGGAATCTAGACCTTCAATACTAAGGCAATTTATCGGAAATG
ACTGAAACTCGAAAAACTGGATTCCCACTTTTGTGGGAATGACGCGATTAGAGTTTCAAA
ATTTATTCTAAATAGCTGAAACTCAACACACTGGATTCCCGCCTGCGCGGGAATGACGAA
GTGGAAGTTACCCGAAACTTAAAACAAGCGAAACCGAACGAACTGGATTCCCACTTTCGT
GGGAATGACGGAATGTAGGTTCGTGGGAATGACGGCGGAGCGGTTTCTGCTTTTTCCAAT
AAATGACCCCAACTTAAAATCCCGTCATTCCCGCGCAGGCGGGAATCTAGGTCTGTCGGT
GCGGAAACTTATCGGGTAAAACGGTTTCTTGAGATTTTGCGTCCTGGATTCCCACTTTCG
TGGGAATGACGGAATGTAGGTTCGTGGGAATGACGGGATATAGGTTTCCGTGCGGACGCG
TTCGGATTCATGACTGCGCGGGAATGACGGGATTTTGGTGTATTCCCTAAAAAAATAAAA
AAGTATTTGCAAATTTGTTAAAAATAAATAAAATAATAATCCTTATCATTCTTTAATTGA
ATTGGATTTATTATGAACAATCCATTGGTGAATCAGGCTGCTATGGTGCTGCCTGTGTTT
TTGTTGAGTGCTTGTTTGGGCGGAGGCGGCAGTTTCGATCTTGATTCTGTCGATACCGAA
GCCCCGCGTCCCGCGCCAAAATATCAAGATGTTTTTCCGAAAAACCGCAAGCCCAAAAA
GACCAAGGCGGATACGGTTTTGCAATGAGGTTGAAACGGAGGAATTGGTATCCGCAGGCA
AAAGAAGACGAGGTTAAACTGGACGAGAGTGATTGGGAGGCGACAGGATTGCCGGACGAA
CCTAAGGAACTCCCTAAACGGCAAAAATCGGTTATCGAAAAAGTAGAAACAGACAGCGAC
AACAATATTTATTCTTCCCCCTATCTCAAACCATCAAACCATCAAAACGGCAACACTGGC
AACGGTATAAACCAACCTAAAAATCAGGCAAAAGATTACGAAAATTTTAAATATGTTTAT
TCCGGCTGGTTTTACAAACACGCCAAACGAGAGTTTAACTTAAAGGTGGAACCTAAAAGT
GCAAAAAACGGCGACGACGGTTATATCTTCTATCACGGTAAAGAACCTTCCCGACAACTT
CCCGCTTCTGGAAAAATTACCTATAAAGGTGTGTGGCATTTTGCGACCGATACAAAAAAG
GGTCAAAAATTTCGTGAAATTATCCAACCTTCAAAAAGTCAAGGCGACAGGTATAGCGGA
TTTTCGGGCGATGACGGCGAAGAATATTCCAACAAAAACAAATCCACGCTGACAGATGGT
CAAGAGGGTTATGGTTTTACCTCAAATTTAGAAGTGGATTTCCATAATAAAAAATTGACG

## Appendix A

GGCAAACTGATACGCAACAATGCGAATACCGATAACAACCAAGCCACCACCACGCAATAC
TACAGCCTTGAGGCTCAAGTAACAGGCAACCGCTTCAACGGCAAGGCAACGGCAACCGAC
AAACCCCAACAAAACAGCGAAACCAAGGAACATCCCTTTGTTTCCGATTCGTCTTCTTTG
AGCGGCGGCTTTTTCGGCCCGCAGGGTGAGGAATTGGGTTTCCGCTTTTTGAGCGACGAT
CAAAAAGTTGCCGTTGTCGGCAGCGCGAAAACCAAAGACAAACCCGCAAATGGCAATACT
GCGGCGGCTTCAGGCGGCACAGATGCGGCAGCATCAAACGGTGCGGCAGGCACGTCGTCT
GAAAACGGTAAGCTGACCACGGTTTTGGATGCGGTCGAGCTGAAATTGGGCGATAAGGAA
GTCCAAAAGCTCGACAACTTCAGCAACGCCGCCCAACTGGTTGTCGACGGCATTATGATT
CCGCTCTTGCCCGAGGCTTCCGAAAGTGGGAACAATCAAGCCAATCAAGGTACAAATGGC
GGAACAGCCTTTACCCGCAAATTTGACCACACGCCGGAAAGTGATAAAAAAGACGCCCAA
GCAGGTACGCAGACGAATGGGGCGCAAACCGCTTCAAATACGGCAGGTGATACCAATGGC
AAAACAAAAACCTATGAAGTCGAAGTCTGCTGTTCCAACCTCAATTATCTGAAATACGGA
ATGTTGACGCGCAAAAACAGCAAGTCCGCGATGCAGGCAGGAGAAAGCAGTAGTCAAGCT
GATGCTAAAACGGAACAAGTTGAACAAAGTATGTTCCTCCAAGGCGAGCGCACCGATGAA
AAAGAGATTCCAAGCGAGCAAAACATCGTTTATCGGGGGTCTTGGTACGGATATATTGCC
AACGACAAAAGCACAAGCTGGAGCGGCAATGCTTCCAATGCAACGAGTGGCAACAGGGCG
GAATTTACTGTGAATTTTGCCGATAAAAAAATTACTGGTACGTTAACCGCTGACAACAGG
CAGGAGGCAACCTTTACCATTGATGGTAATATTAAGGACAACGGCTTTGAAGGTACGGCG
AAAACTGCTGAGTCAGGTTTTGATCTCGATCAAAGCAATACCACCCGCACGCCTAAGGCA
TATATCACAGATGCCAAGGTGCAGGGCGGTTTTTACGGGCCCAAAGCCGAAGAGTTGGGC
GGATGGTTTGCCTATCCGGGCGATAAACAAACGAAAAATGCAACAAATGCATCCGGCAAT
AGCAGTGCAACTGTCGTATTCGGTGCGAAACGCCAACAGCCTGTGCGATAAGCACGGCTG
CCGAACAATCAAGAATAAGGCCTCAGACGGCACCGCTCCTTCCGATGCCGTCTGAAAGCG
AAGATTAGGGAAACACTATGCAACAGCAACATTTGTTCCGATTCAATATTTTATGCCTGT
CTTTAATGACTGCGCTGCCCGCTTATGCAGAAAATGTGCAAGCCGGACAAGCACAGGAAA
AACAGTTGGATACCATACAGGTAAAAGCCAAAAAACAGAAAACCCGCCGCGATAACGAAG
TAACCGGGCTGGGCAAGTTGGTCAAGTCTTCCGATACGCTAAGTAAAGAACAGGTTTTGA
ATATCCGAGACCTGACCCGTTATGATCCGGGTATTGCCGTGGTCGAACAGGGTCGGGGCG
CAAGTTCCGGCTATTCAATACGCGGCATGGATAAAAACCGCGTTTCCTTAACGGTGGACG
GCGTTTCGCAAATACAGTCCTACACCGCGCAGGCGGCATTGGGCGGGACGAGGACGGCGG
GCAGCAGCGGCGCAATCAATGAAATCGAGTATGAAAACGTCAAAGCTGTCGAAATCAGCA
AAGGCTCAAACTCGGTCGAACAAGGCAGCGGCGCATTGGCGGGCTCGGTCGCATTTCAAA
CCAAAACCGCCGACGATGTTATCGGGGAAGGCAGGCAGTGGGGCATTCAGAGTAAAACCG
CCTATTCCGGCAAAAACCGGGGGCTTACCCAATCCATCGCGCTGGCGGGGCGCATCGGCG
GTGCGGAGGCTTTGCTGATCCACACCGGGCGGCGCGCGGGGGAAATCCGCGCCCACGAAG
ATGCAGGACGCGGCGTTCAGAGCTTTAACAGGCTGGTGCCGGTTGAAGACAGCAGCAATT
ACGCCTATTTCATCGTTAAAGAAGAATGCAAAAACGGGAGTTATGAAACGTGTAAAGCGA
ATCCGAAAAAAGATGTTGTCGGCAAAGACGAACGTCAAACGGTTTCCACCCGAGACTACA
CGGGTCCCAACCGCTTCCTCGCCGATCCGCTTTCATACGAAAGCCGGTCGTGGCTGTTCC
GCCCGGGTTTTCGTTTTGAGAATAAGCGGCACTACATCGGCGGCATACTCGAACACACGC
AACAAACTTTCGACACGCGCGATATGACGGTTCCGGCATTCCTGACCAAGGCGGTTTTTG
ATGCAAATAAAAAACAGGCGGGTTCTTTGCCCGGTAACGGCAAATACGCGGGCAACCACA
AATACGGCGGACTGTTTACCAACGGCGAAAACGGTGCGCTGGTGGGCGCGGAATACGGTA
CGGGCGTGTTTTACGACGAGACGCACACCAAAAGCCGCTACGGTTTGGAATATGTCTATA
CCAATGCCGATAAAGACACTTGGGCGGATTATGCCCGCCTCTCTTACGACCGGCAGGGCA
TCGGTTTGGATAATCATTTTCAGCAGACGCACTGTTCTGCCGACGGTTCGGACAAATATT
GCCGCCCGAGTGCCGACAAGCCGTTTTCCTATTACAAATCCGATCGCGTGATTTACGGGG
AAAGCCACAGGCTCTTGCAGGCGGCATTCAAAAAATCCTTCGATACCGCCAAAATCCGCC
ACAACCTGAGCGTGAATCTCGGGTTTGACCGCTTTGGCTCTAATCTCCGCCATCAGGATT
ATTATTATCAACATGCCAACCGCGCCTATTCGTCGAACACGCCCCCTCAAAACAACGGCA
AAAAAATCAGCCCCAACGGCAGTGAAACCAGCCCCTATTGGGTCACCATAGGCAGGGGAA
ATGTCGTTACGGGGCAAATCTGCCGCTTGGGCAACAATACTTATACGGACTGCACGCCGC
GCAGCATCAACGGTAAAAGCTATTACGCGGCAGTTCGGGACAATGTCCGTTTGGGCAGGT
GGGCGGATGTCGGCGCGGGCTTGCGCTACGACTACCGCAGCACGCATTCGGACGACGGCA
GCGTTTCCACCGGCACGCACCGCACCTTGTCCTGGAACGCCGGCATCGTCCTCAAACCTA
CCGACTGGCTGGATTTGACTTACCGCACCTCAACCGGCTTCCGCCTGCCCTCGTTTGCGG
AAATGTACGGCTGGCGGGCGGGTGTTCAAAGCAAGGCGGTCAAAATCGATCCGGAAAAAT
CGTTCAACAAAGAAGCCGGCATCGTGTTTAAAGGCGATTTCGGCAACTTGGAGGCAAGTT
GGTTCAACAATGCCTACCGCGATTTGATTGTCCGGGGTTATGAAGCGCAAATTAAAGACG
GCAAAGAAGAAGCCAAAGGCGACCCGGCTTACCTCAATGCCCAAAGCGCGCGGATTACCG
GCATCAATATTTTGGGCAAAATCGATTGGAACGGCGTATGGGATAAATTGCCCGAAGGTT
GGTATTCTACATTTGCCTATAATCGTGTCCGTGTCCGCGACATCAAAAAACGCGCAGACC
GCACCGATATTCAATCACATCTGTTTGATGCCATCCAACCCTCGCGCTATGTCGTCGGCT
TGGGCTATGACCAACCGGAAGGCAAATGGGGTGTGAACGGTATGCTGACTTATTCCAAAG
CCAAGGAAATCACAGAGTTGTTGGGCAGCCGGGCTTTGCTCAACGGCAACAGCCGCAATA
CAAAAGCCACCGCGCGCCGTACCCGCCCTTGGTATATTGTGGACGTGTCCGGTTATTACA
CGGTTAAAAAACACTTTACCCTCCGTGCGGGCGTGTACAACCTCCTCAACTACCGCTATG
TTACTTGGGAAAATGTGCGGCAAACTGCCGGCGGCGCAGTCAACCAACACAAAAATGTCG
GCGTTTACAACCGATATGCCGCCCCCGGTCGCAACTACACATTTAGCTTGGAAATGAAGT
TCTAAACGTCCAAACGCCGCAAATGCCGTCTGAAAGGCTTCAGACGGCATTTTTTACACA
ATCCCCGCCATTTTCCATCATCCCCGATACACCGTAATCTCGAAACCCGTCATTCCCGCG
CAGGCGGGAATCCAGTCCGTTCGGTTTCGGTTTTTTTGAGGTTTCGGGTAACTTCTAAAC
CGTTATTCCCGCGAAAACAGAAAATCAAAAACAGAAACCTCAAATCCCGTTATTCCCGAG
CAGACGGGATCTAGGGCGTAAAATCTAAAGAAACCGTTTTATCCGATAAGTTTCCGCACC
GACAGACTAGATTCCCGCCTGCGCGGGAATGACGTTATATTTTTCGCATTTGATAAAAAA

## Appendix A

GACCGTTTGAAATTTTTTCAGCGGACGCAAAGTATTGCGTAAAATGCTGCTTATAAGAAA
CGCAAGGCGGGCGTAGGATGTGCAGCGTGGACATAGGGCTGGCTGTAGGGTGGGCTTCAG
CCCACCAATCCCACCGTTTCCACCTATTCCCCCAACTCCGTCAATGTTATCCATTCCGCC
CATTCCCACCGAAAACCGAAACCGCCGTATTCCCAAAAACCTTTGATGCGGTGAAATTGG
TGGGCTGAAGCCCACCCTACAGCCCACCCTACGGCTCGCCGAAATTTCGTCATTCCCGCG
CAGGCGGGAATCCAGGTCTGTCGGTGCGGAAACTTATCGGATAAAACGGTTTCTTGAGAT
TTTACGTCCTAGATTCCCACTTCCGTGGGAATGACGGGATGCAGGTTTTCGTGCGGACGC
GTTCGGATTCACGACTGCGCGGGAATGACGGGATTTCAGTTTGCGGATTGATTTGAAGTT
GCAAAATCCCAACGGATCGGATTACCGCTTTCGCGTTTCAAAGTTACGGCGTTATCGGAA
AAACAGAAAATCAAAGCTGCAAGAATTTATTTAAAACAACCGAATTTCAACGGATCGGAT
TCTCGCCTGTAGGGAATGACGGCGGAAGGTTTTTGTCTTTTCTGACAGATGTCCGCAAT
CTGAAATCCTGACCGTGGGAACGACGGTATAGTGGATTAACAAAAACCAGTACGGCGTTG
GCTCGCCTTAGCTCAAAGAGAACGATTCTCTAAGGTGCTGAAGCACCAAGTGAATCGGTT
CCGTACTATTTGTACTGTCTGCGGCTTTGTCGCCTTGTCCTGATTTTTGTTAATCCACTA
TATAAATATTTCTATTTCAATCCAATATAAAATGCCGTCCGAACATCGTTCGGACGGCAT
TTTTAATGCTTCAAATCAGTTGGTGCCGACTTTGATTTTCTGCCACAGGCTGACAGACAG
TTTTTTCGCATCCGTGCTCATTTGCGGCATCACGAAACCGTCTTTCATATCCTGCTCGTT
CGGGAAGATGGAACGGGTGTTCACCAGCTCGGCAGGCATTTTTTCGCGCGCCGGTTTGCT
GGCGGGGGCAAAGGTTACGGCGATGCCGTTTTTCGCCGCGATTTCGGGGTCGAGCGTGTA
GTTGATGTATTTGTGGGCATTGGCGACGTTTTTCGCATCGGCGGGAATCAGCCAAGACTC
AATCCAGAAGCCCATACCTTTCGGTGTCAGCACTTCGATGCCGACGTTGTTTTTCACTTC
CTCGGAACGTGCTTTCGCCAAGTTCAAATCGCCGCCGTTGCCTGCCGCCAGGCAGATGTC
GCCGCGTGCCAGCTCGTCGATGATGGACGGGCTGAAACGTTTGACATCCGGACGGATAGA
CTTCAACACTTCCGCCGCCGCCTTCAAGTCTTCAGGATTCGAGCCTTTGGGGTCTTTGCC
CAAGTAGTTCAGCAAAATCGGGAACATTTCACTCGGGGTGTCCCACAGGGCGATGCCGCA
GGATTTCAGCTTGTGGGTGTATTCGGGTTTGAACAGCAAATCCCAGCCGTTTTCGGGCAG
CTTGCCGCCCAAAAGCTCTTTGCCCTTCGCCGTAATCGCAATCGTGTTCACGCCGGAGAA
ATAGGGGACGGCATACTGGTTGCCCGGGTCGGCGGTTTCCAGCATTTTCAAGAGTTCGGG
ATCGATGTTTTTATAGTTGGGAATCAGGTCTTTGTTGACTTTTTGATACGCGCCCGCCTC
GATTTGGCGCGGCAGGAAGGCGATGCCCGGCACGACCAAATCGTAACCGGATTTGCCGGT
CAGCATTTTGGCTTCCAGCGTTTCATTGTTTTCGTACAAGTCGTAAGTCAGCTTCAGATT
GTTGGCTTTTTTAAAGTCTTCGACCGTACTCTCATCAACATAGTTCGACCAGTTGTAGAT
GTTCAGAGTATCGGTGGCAGCGGCTTCGGCATTGGCAGCAGACGCAGCGTCTGCTTGAGG
TTGCACGGCGTTTTTTTCGCTGCCGCCGCAGGCTGCCAGAGACAGCGCGGCCAAAACGGC
TAATACGGATTTTTTCATACGGGCAGATTCCTGATGAAAGAGGTTGGAAAAAAAGAAATC
CCCGCGCCCCATCGTTACCCCGGCGCAAGGTTTGGGCATTGTAAAGTAAATTTGTGCAAA
CTCAAAGCGATATTGGACTGATTTTCCTAAAAAATTATCCTGTTTCCAAAAGGGGAGAAA
AACGTCCGCCCGATTTTGCCGTTTTTTTGCGCTGTCAGGGTGTCCGACGGGCGGATAGAG
AGAAAAGGCTTGCATATAATGTAAACCCCCTTTAAAATTGCGCGTTTACAGAATTTATTT
TTCTTCCAGGAGATTCCAATATGGCAAACAGCGCACAAGCACGCAAACGTGCCCGCCAGT
CCGTCAAACAACGCGCCCACAATGCTAGCCTGCGTACCGCATTCCGCACCGCAGTGAAAA
AAGTATTGAAAGCAGTCGAAGCAGGCGATAAAGCTGCCGCACAAGCGGTTTACCAAGAGT
CCGTCAAAGTCATCGACCGCATCGCCGACAAGGGCGTGTTCCACAAAAACAAAGCGGCAC
GCCACAAAAGCCGTCTGTCTGCAAAAGTAAAAGCCTTGGCTTGATTTTTGCAAAACCGCC
AAGGCGGTTGATACGCGATAAGCGGAAAACCCTGAAGCCCGACGGTTTCGGGGTTTTCTG
TATTGCGGGGGCAAAATCCCGAAATGGCGGAAAGGGTGCGATTTTTTATCCGAATCCGCT
ATAAAATGCCGTTTGAAAACCAATATGCCGACAATGGGGGCGGAGATGAATACACGGAAT
ATGCGCTATATTCTTTTGACAGGACTGTTGCCGATGGCATCCGCTTTTGGAGAGACCGCG
CTGCAATGCGCCGCTTTGACGGACAATGTTACGCGTTTGGCGTGTTACGACAGGATTTTT
GCGGCACAGCTTCCGTCTTCGGCAGGGCAGGAAGGGCAGGAGTCGAAAGCCGTACTCAAT
CTGACGGAAACCGTCCGCAGCAGCCTGGATAAGGGCGAGGCGGTCATTGTTGTTGAAAAA
GGCGGGGATGCGCTTCCTGCCGACAGTGCGGGCGAAACCGCCGACATCTATACGCCTTTG
AGCCTGATGTACGACTTGGACAAAAACGATTTGCGCGGCTGTTGGGCGTACGCGAACAC
AATCCGATGTACCTTATGCCGCTCTGGTACAACAATTCGCCCAACTATGCCCCGGGTTCG
CCGACGCGCGGTACGACTGTACAGGAAAAATTCGGACAGCAGAAACGTGCGGAAACCAAA
TTGCAGGTTTCGTTCAAAAGCAAAATTGCCGAAGATTTGTTTAAAACCCGCGCGGATCTG
TGGTTCGGCTACACCCAAAGATCCGATTGGCAGATTTACAACCAAGGCAGGAAATCCGCG
CCGTTCCGCAATACGGATTACAAACCTGAAATTTTCCTGACCCAGCCTGTGAAGGCGGAT
TTGCCGTTCGGCGGCAGGCTGCGTATGCTCGGTGCGGGTTTTGTCCACCAGTCCAACGGA
CAGAGCCGTCCCGAATCGCGTTCGTGGAACAGGATTTACGCCATGGCAGGCATGGAATGG
GGCAAATTGACGGTGATTCCGCGCGTGTGGGTGCGTGCGTTCGATCAGAGCGGCGATAAA
AACGACAATCCCGATATTGCCGACTATATGGGGTATGGCGACGTGAAGCTGCAGTACCGC
CTGAACGACAGGCAGAATGTGTATTCCGTATTGCGCTACAACCCCAAAACGGGCTACGGC
GCGATTGAAGCCGCCTACACGTTTCCGATTAAGGGCAAACTCAAAGGCGTGGTACGCGGA
TTCCACGGTTACGGCGAGAGCCTGATCGACTACAACCACAAGCAGAACGGTATCGGTATC
GGGTTGATGTTCAACGACTTGGACGGCATCTGAACCGCGTGTTCAGACGGTATATCAAGT
TGCCGTGCCGTCTGAAGCCGCCGGCGGTTTGGCGGGGCGGATAAAAATTTCGTTTGAAT
CCGGGCAAACGCGTATAATGTGCGGTTTGTACGGACTTTGTCGGAATCAAGCAAGATGAC
GGAACCTGCGGCCGAAGGCGGCAAAGCTGCCAAGGCGTTAAAAAAATATCTGATTACGGG
CATTTTGGTCTGGCTGCCGATTGCGGTAACGGTTTGGGTGGTTTCCTATATCGTTTCCGC
GTCCGATCAGCTCGTCAACCTGCTGCCGAAGCAATGGCGGCCGCAATATGTTTTGGGGTT
TAATATCCCGGGGCTGGGCGTTATCGTTGCCATTGCCGTATTGTTTGTAACCGGATTGTT
TGCCGCCAACGTATTGGGTCGGCAGATCCTCGCCGCGTGGGACAGCCTGTTGGGGCGGAT
TCCGGTTGTGAAATCCATCTATTCGAGTGTGAAAAAAGTATCCGAATCGCTGCTGTCCGA
CAGCAGCCGTTCGTTTAAAACGCCGGTACTCGTGCCGTTTCCCCAGCCCGGTATTTGGAC

# Appendix A

GATTGCTTTCGTGTCAGGGCAGGTGTCGAATGCGGTTAAGGCCGCATTGCCGAAGGACGG
CGATTATCTTTCCGTGTATGTTCCGACCACGCCGAATCCGACCGGCGGTTACTATATTAT
GGTAAAGAAAAGCGATGTGCGCGAACTCGATATGAGCGTGGACGAAGCATTGAAATATGT
GATTTCGCTGGGTATGGTCATCCCTGACGACCTGCCCGTCAAAACATTGGCAGGACCTAT
GCCGTCTGAAAAGGCGGATTTGCCCGAACAACAATAAAGCCGCCGTTCAGACGGCATTTT
CTGTTTTCAGTTTAAATCAATAAAAGGTGATTTTATGCGTACCAACTATTGCGGCCTGAT
CAGTGAGCAATACTTAGACCAAACCGTTACCGTCAAAGGCTGGGTACACCGTCGACGCGA
CCACGGCGGTGTGATTTTTATCGACCTGCGCGACCGCGAAGGCATCGTCCAAGTCGTGAT
CGATCCCGACACGCCCGAAGCGTTTGCCGCTGCCGATTCCTCCCGCAACGAATACGTTTT
GAGCATTACCGGCCGCGTACGCAACCGTCCCGAAGGCACGACCAACGATAAAATGATTTC
CGGCAAAATCGAAATCCTTGCCAAAGAAATCGAAGTCTTGAACGCCGCCGCCACGCCGCC
GTTCCAAATCGACGATGAAAACATCAGCGAAAACGTTCGCCTGACCAACCGCGTTATCGA
CTTGCGCCGTCCGGTGATGCAACGCAACCTGCGCCTGCGTTACCAAGTTGCTATGGGCGT
TCGCCGCTACTTGGACGCGCAAGGTTTCATCGACATTGAAACCCCGATGCTGACCCGCTC
CACGCCTGAAGGCGCGCGCGACTACCTCGTGCCGAGCCGCGTTCATCCGGGCGAGTTTTT
CGCGCTACCGCAATCGCCGCAATTATTCAAACAACTGTTGATGGTGGCGGGTTTCGACCG
TTACTACCAAATCACCAAGTGCTTCCGCGACGAAGACCTGCGTGCCGACCGCCAGCCCGA
ATTTACCCAAATCGACTTGGAAACCTCGTTCTTAAACGAGGATGAAATCATGGACATCAC
TGAAGGCATGGCCAAACAAGTCTTCAAAGATGCTTTAAATGTAGATTTGGGCGACTTCCC
ACGCATGCCTTACTCTGAAGCCATGTTCTACTACGGCTCTGACAAACCGGATATGCGCAT
CAACTTGAAATTTACCGAGTTGACCGACCTGATGAAAACGGAAGAATTCAAAGTCTTCCG
TGGCGCAGCCGACATGAAAGGCGGCCGCGTGGTCGCTCTGCGCGTGCCGAACGGCGCAGA
ATTCAGCCGCAAAGAAATCGACGAATACACCAAATTTGTCGGCATCTACGGCGCGAAAGG
TCTGGCATACATCAAAGTAAACGATGTCAGCAACCTTTCCAACGGCGAAGACAGCGGCCT
GCAATCTCCAATCGTGAAATACCTGTCCGAAAACGCCCTGAAAGAAATTATCGCGCGTAC
CGGCGCGCAAAACGGCGACATCATCTTCTTCGGCGCAGACAAAGCCAAAGTCGTGAACGA
AGCCATCGGCGCACTGCGTATCAAAGTCGGCTTGGAGCACGGCAAAGACAACGGCTATTT
CACAGACGAATGGAAACCTTTGTGGGTCGTTGATTTCCCAATGTTCGAATACGACGAAGA
AGCCGACCGCTACGTTGCCGTACACCATCCGTTTACCGCGCCAAAAGAAGGTCATGAAGA
CCTGATGGTTTCCGACCCGGCAAATTGTTTGGCACGCGCCTACGATATGGTATTGAACGG
CTGGGAAATCGGCGGCGGCTCTATCCGTATTCACCGCGCAGACGTACAAGAGAAAGTGTT
TGCCGCGCTGAAAATCAGCCCTGAAGAGCAACAAGAGAAATTCGGCTTCCTCTTGGACAA
CCTGAAATTCGGCGCACCTCCTCACGGCGGTCTTGCATTCGGCCTCGACCGTCTGGTAAC
GCTGATGACCGGTGCCGAATCCATCCGCGACGTGATTGCCTTCCCGAAAACACAACGCGC
CCAATGCCTGCTGACCAACGCGCCCAACAGCGTGGACGACAAGCAGTTGCGTGAATTAAG
TTTGCGTTTGCGCCAGAAGGCAACCGAAACTAAAGAAGTATAAGGAAAACGGAGCCGTTT
GACGGCTCTGTTTTTTTCAGACGGCATTTACGCTTCTTGACTTCCCTCTAATTCAAACCT
AATTTTGCTGTGTTTTAATGGCGGTATTGAAAAACACATCTTGTTCAATCAACCGATAAA
AAAAGGACTGAAAATGAAAAAACTGTTATTGGCTGCCGTTGTTTCTCTGAGTGCCGCTGC
CGCATTTGCCGGCGACTCTGCCGAGCGTCAGATTTACGGCGATCCCCATTTTGAACAAAA
CCGCACAAAAGCTGTGAAAATGTTGGAGCAGCGCGGTTATCAGGTTTACGATGTCGATGC
CGACGACCATTGGGGTAAGCCTGTGCTGGAAGTGGAAGCCTATAAAGACGGCCGCGAATA
CGACATCGTGTTGTCTTACCCCGACCTGAAAATCATCAAAGAGCAGCTCGATCGCTGACT
CCTTTGATGGAAAGATGAACCAAAATGCCGTCTGAAGCGTTCAGACGGCATTTTGCCTGT
TCCTCATCAGGTATGAGGCAGGCTTTTCTTATTAAAAAAATGACATTTCACGCTGATTTG
TTATAATCATTCCTTTTCAACACGACAGACGGAGCAGGTTTATTATGCCTATCCTTACCA
TCCGTGAAGTGTGCAACATTAATCATTGGGGCATAGGTTATTATGATGTTGACGATTCCG
GCGAAATCATCGTCCGCCCCAATCCCTCGCAACACAATCAAACTGTTTCACTGCAAAAAC
TGACTGAAGCCGTGCAACAAAAACATCAGGCGCCTGCCTGTTTTGTTTTGTTTTCCGC
AAATCCTCGAACACCGCCTCCGCGACATTAACCGCGCCTTTCAGACGGCACGGGAAGAGT
GCGGCTATAAGGGCGGTTATTGTTTGGTTTACCCTATCAAGGTCAACCAACACCGCCGCG
TCATCGAATCGCTTATGTCAAGCGGACAACCGCATGGTTTGGAAGCTGGTTCTAAAGCCG
AACTGATGGCGGTTTTGGCACACGCCGGCAACCGGCAAACATTAATCGTCTGCAACGGCT
ATAAAGACCGTGAATATATCCGTTTCGCCTTGATGGGCGAAAAACTGGGGCATCAGGTTT
ATTTGGTGATTGAGAAGCTGTCCGAAATACAAATGGTATTGGAAGAGGCGGAAAAACTCG
GCATCAAGCCCCGTTTGGGTGTGCGCGCCAGACTGGCTTCCCAAGGTTCGGGAAAATGGC
AGTCTTCGGGTGGGGAAAAATCAAAATTCGGCTTGTCGGCTTCCCAAGTTTTGCAACTGG
TCGATATTTTGAAACAAAAAAACAGGCTGGATTGCCTGCAGCTTTTGCATTTCCATTTGG
GCTCGCAGCTTGGGAACATCCGTGATGTTGCCACAGGTGTACACGAATCGGCTCGGTTTT
ATGTTGAGTTGCACAAACTGGGGGTAAATATCCGCTGTTTTGATGTAGGCGGCGGGCTTG
GCGTGGATTACGAAGGAAACCGCACACAATCGGATTGTTCCGTTAATTACAGCCTCAACG
AATATGCCGCCACAGTCGTATGGGGCATCAGTCAGGCTTGTCTCGAACACGGGCTGCCGC
ATCCGACAATCATCACCGAGAGCGGGCGCGGCATTACCGCACATCACGCCGTTTTGGTTG
CTAATGTTATAGGCGTTGAACGTTACAAACCGCGCCGGCTGGATGCGCCATCGCCCGAAG
CACCGCGTGTGTTGCACAGTATGTGGGAAACTTGGACGGATATTTCCGCCTCGCGGGAAA
AACGTTCCTTACGCAGCTGGATACACGAAGGGCAGTTTGATCTTGCTGATGTGCATAATC
AGTATAATGTCGGGCTGTTGAGTTTGGCGCAACGTGCGTGGGCGGAGCAACTGTATTTAA
ATATCTGTCATGAAGTCGGCGAATTGTTTAATGAAAAACACCGGTCTCACCGAACCATTA
TTGACGAATTGCAAGAACGTTTTGCCGATAAGCTGTATGTCAATTTCTCACTCTTCCAAT
CTTTGCCCGATGCTTGGGGCATAGATCAACTTTTCCCTGTTTGTCCCATTACCGGTTTGA
ATGAACCGATTGCGCGCCGCGCCGTGTTGTTGGACATTACCTGCGATTCAGACGGTACGA
TTGACCACTACATCGACGGAGACGGCATCGCCGGTACGATGCCTATGCCTGATTATCCCG
AAGAAGAGCCGCCGCTTTTAGGCTTTTTTATGGTGGGAGCATATCAGGAAATACTCGGCA
ATATGCACAATCTTTTCGGCGACACTGCCACTGCCGATGTTGTTGTAGGGGAAGACGGAC
AATTTACCGTCATCGATTACGATGAAGGAAACACCGTTGCCGATATGCTCGAATACGTTT

## Appendix A

ATCAAGATCCGAAAGAGCTGATGAAACGCTATCGCGAACAAATCGAACATTCAGACCTTC
CTGCCTCGCAGGCTATGTCTTTCTTAAAAGAACTCGAAGCGGGGCTTAATGGTTATACCT
ATTTGGAAGACGAATAGACGCATCAAGGCATCGGATATGTCGTCTGAAGCCCGATTTTCT
TACTCAAACACCAATCATCACGACCGATTGAAACCAATTACAAGGAATCATTACGATGCA
ATACAGCACACTGGCAGGACAAACCGACAACTCCCTCGTTTCCAATAATTTCGGGTTTTT
GCGCCTGCCGCTTAATTTTATGCCGTATGAAAGTCATGCCGATTGGGTTATTACCGGCGT
GCCTTATGATATGGCGGTTTCAGGGCGTTCCGGCGCGCGTTTCGGTCCTGAAGCCATCCG
GCGCGCCTCCGTCAACCTCGCTTGGGAGCACCGCAGGTTTCCATGGACATTTGATGTGCG
CGAACGCCTGAACATTATTGATTGCGGCGACTTGGTTTTTTCTTTTGGCGACAGCAGGGA
TTTTGTCGAAAAAATGGAAGCGCACGCCGGCAAATTACTTTCTTCCGGCAAACGCTGTTT
GAGTTTGGGCGGCGACCATTTCATTACCCTACCGTTGTTGCGCGCCCACGCCCGCTATTT
CGGCAAACTCGCACTGATTCATTTTGACGCGCACACCGACACCTACGACAACGGCAGCGA
ATACGACCACGGTACGATGTTCTATACCGCCCCCAAGGAAGGCCTCATCGACCCGTCCCG
TTCCGTACAAATCGGCATACGCACCGAACACAGTAAAAAATTGCCTTTTACTGTGTTGTC
CGCCCCTAAAGTCAATGAAGACAGTGTTGAAGAGACCGTCCGTAAAATCAAAGAAACCGT
CGGCAATATGCCCGTTTACCTGACTTTCGACATAGACTGCCTGGACCCGTCGTTCGCCCC
TGGGACCGGTACGCCCGTATGCGGCGGCTTGAGCAGCGACAGGGCATTAAAAATCCTACG
TGGGCTGACGGATCTCGACATCGTCGGTATGGATGTTGTAGAAGTTGCCCCCTCTTACGA
CCAATCCGACATTACCGCTTTGGCCGGTGCCACAATTGCCTTGGAAATGCTTTACCTTCA
AGGTGCGAAAAAGGACTGAACGTCCGGCATCCCCCGGGTTTTCGCCGTGCCGTTCAAACG
GCGTATTCAGTCTAATGAAAATTCAAATACTGAAACAAAAGTTGCCCGGAGCCGCATATC
GGAAAGACGGTGAAATATCAGAATATATCTTATAAAACAATTAGTTAAATATTATTTTTC
CGATTTTTCGGGACGGTCTTTTTTACGGAGGTCAATATGATGAAATTGGGTTTCAAACCG
ATACCCCTCGCCATTGCCGCAGTATTGTGCGCCCTGGTTTTGGCACTGCCCGTACCCGAC
GGGGTCAAGCCTCAGGCTTGGACGCTGCTGGCCATGTTTGTCGGTGTGATTGCCGCCATT
ATCGGCAAGGCCATGCCGTTGGGCGCGCTGTCGATTATTGCCGTCGGGTTGGTCGCAGTA
ACCGGCGTAACCGCCGACAAACCGGGCGCGGCGATGAGCGATGCGTTGAGTGCGTTCGCC
AATCCGTTGATTTGGCTGATTGCCATCGCAGTTATGATTTCGCGCGGTTTGCTCAAAACA
GGGCTGGGGATGCGTATCGGATATTTGTTTATCGCCGTTTTTGGAAGAAAAACGCTGGGC
ATCGGTTACAGTCTCGCTCTTTCCGAACTGCTGCTGGCTCCCGTTACCCCTTCCAATACC
GCGCGCGGCGGCGGCATTATACATCCGATTATGCAGTCGATTGCCGGCAGTTACGGCTCC
AATCCCGCAAAAGGCACAGAAGGCAAGATGGGTAAATATTTGGCTTTGGTCAACTATCAT
TCCAATCCCATTTCGTCGGCTATGTTTATTACTGCAACTGCCCCCAACCCTTTAATCGTC
AACTTGATTGCCGAAAATTTAGGCAGTAGTTTCCGTCTTTCTTGGGGGGCGTGGGCGTGG
GCAATGGCTGTTCCCGGCGTTATCGCCTTTTTCGTTATGCCTTTGATTTTATATTTTTTG
TATCCGCCTGAAATTAAAGAAACGCCCAATGCCGTTCAATTTGCCAAAGACCGTCTGAGG
GAGATGGGTAAAATGTCGGCAGACGAAATCATTATGGCGGTCATTTTCGGTATCTTGCTG
CTGTTGTGGGCAGATGTTCCCGCCCTTATTACCGGCAATCACGCTTTTAGTATCAACGCC
ACCGCCACCGCATTTATCGGATTAAGCCTGCTTTTGCTTTCCGGTGTATTGACTTGGGAC
GATGTTTTGAAAGAAAAAAGCGCGTGGGATACGATTATTTGGTTTGGCGCATTGATTATG
ATGGCCGCATTTTTAAATAAACTCGGACTGATTAAATGGTTCTCCGGAGTGTTGGCGGAA
AGTGTCGGCGGTTTGGGCGTTAGCGGCACGGCTGCGGGCGTAATCCTCGTGCTTGCTTAT
ATGTATGCGCATTATATGTTTGCCAGTACTACTGCACATATTACCGCTATGTTCGGCGCA
TTTTTCGCTGCTGCCGTTTCACTGAATGCCCCGGCGATGCCGACCGCGCTGATGATGGCG
GCCGCATCCAACATTATGATGACCCTCACTCATTATGCGACCGGTACTTCGCCTGTGATT
TTCGGTTCGGGCTACACCACAATGGGAGAATGGTGGAAGGCGGGTTTTATCATGAGCGTA
GTCAATTTTCTGATTTTTTTCGTTATCGGCAGCATTTGGTGGAAAGTTCTGGGGTATTGG
TAAGGGAAAAATAAAATAAATTTCCAATCTGTGTTTATTTGATTGGGCGACTATTATCGT
GAAATATGCCGTCTAAAGCCTTCAGATGGCATATTTGTGCGCTTGAATGTTGCAGAAAGC
~~GGCAGGCGGCGGTGTAGGAAAAGCCAAACAAAAACCAAACCGCCTATCAACTTCTGATAA~~
ACATAAGCATTAAATAATCAGAAGGTTATTCAATTACCTAAACGCAAATTTCCCTGCCGT
ATCACATCTATTGAAAATAATACATCAACCGGCTCGGAAGCAGCCTGATCAGGTGTTTCT
ACTTGCGGCGATGAATCGGCAGCCGGTTCGGTATAGGCAGTCGGCGTGCCGTCGGATTGG
GTATTTAAATGCGGTTGTGCCTCGGGCGTGTGTACATCAGGCACTTCGGGCGTGCGTGTC
TCGGATATTTCGGCAGAGTTGGTTTCCTCAGTTTGTTCAATGACTTCAGCTTGGCTGTAT
GAGGAAGAACCCTGTATCCACGCCAGCGATTTGAGCGGCATCTTCATCTTGCCGTTTTTG
CCGCAGGTCAGGCAGACGGCCGATCCGGTGCGGTCTTTGAGTACAGCATCGACTTTCTCG
GGCGCGATGGTTTTACCCTGTGTTTTTGCCCATTGCGCAATACTTTTTTCAGAGAGGCG
ATGTCAAGGTTGTTCTTACCGTAAGGGTCGCGGAAGGCGGCAAGCCACAGGTTGCGATCC
GCATCTTCATTGAGACCCGCCATTTGATAGATGACGGAGGCGGGCGAGCCGCTGGCGATG
GTTTTGGCAAACTCGAGCGCGTCGGGCGATTTCATGCGGACGCAGCCGTGACTCCGAACC
CCGGGGACGCTGGCCGGCGCATTGGTCCCGTGTATGCCCAAACCGAGTTTGGGGTCGCCT
AAGCGGACAAAAACCGGCCCCAAAGGGTTGTCCGGGCCGGCGGCTATGGTTTTTACGCCG
TCGCCGCGTTCTTTCTGTATGGATTTGGGGATGTACCAAACAGGGTTATAGGCTTTCGCA
CCGATTTTATGTTCGCCTAGATTGGTTTGCGTCATCGCCCGACCTACTGCAACGGGATAA
ACCTTGGTCAGTTTGCCGTCGGTGTAGAGGAACAGGCGTTGCTGAGGGATGTTAATGAAG
ACATGTTGACCTTGTGCGACGGGGGAGACATCGGGAATGATGGTGTTTGCGTATGAAAAA
CCGCTTATCAATAGTGCAGCAGTGCGGCAGATTGTTTTATTCATATCAAAATATGGTGTG
TGTCCGATAGGTTTTCGGCAAATCATACCTGAAACCGTACCAATTTGTGCGAAAATATGC
GCTTCGGTACAGTGCGGACGGATTGGGTAATGGCAACGGAAACAAATGTCGCGGAAATTT
CCGCCTTGGATTATGAAGGCAGGGGTGTGGCAAAGGTCGGCGGCAAAACGGTTTTTATTA
AAAGGGCATTACTTGATTGTTTGATGCTGGGTTGGTTCAGGCTTTAACTCAGGAATATTT
ACATCATAATGAAGGTTTTTAAACAACAGCTTGAACAACTCGGCGCGCAAAACCAATATC
GTTCGATTCCGGATTTGATTCATCAAGGGCGGTATATTACGCGGGAAAACCGCAAAATGC
TGAATATGTCGTCTAATGATTATTTGGGTTTGGCATCAGATGAAAACTTGCGCCGGTCTT

## Appendix A

TTTTGCAGCAATACGGCGGTAATTTTCCCTCTTTTACCAGTTCTTCATCGCGTTTATTAA
CGGGCAACTTTCCTATTTATACCGATTTGGAAGAGCTTGTCGCACAACGTTTCCAACGGG
AAAGCGCGTTATTGTTCAACAGCGGCTATCACGCCAATCTCGGTATTTTGCCTGCTTTGA
CGACGACGAAAAGTTTGATTTTGGCAGATAAATTTGTTCACGCCAGTATGATTGACGGCA
TCCGTTTGAGCCGGTGTGCGTTTTTCCGTTATCGTCATAATGATTATGAACATTTGAAAA
ATCTGCTTGAAAAAAACGTCGGAAAATTTGACCGCACTTTTATCGTTACCGAATCTGTTT
TCAGTATGGACGGCGATGTGGCGGATTTGAAACAGCTTGTCCAATTAAAAAAACAGTTTC
CCAATACTTATCTTTATGTGGATGAAGCCCACGCAATCGGTGTTTATGGGCAAAACGGAT
TGGGGATTGCCGAACGGGATAATTTGATTGCCGAGATTGATTTATTGGTTGGCACTTTCG
GTAAAGCCTTAGCCTCGGTGGGGGCGTATGCCGTCTGCAACCAAGTATTGAAAGAATGTT
TGATTAATCAAATGCGCCCATTGATTTTTTCAACCGCATTGCCGCCGTTTAATGTGGCTT
GGACTTATTTTATTTTTGAACGATTGCCGCAATTCTCAAAAGAAAGAAGCCATCTTGAGC
AGTTAAGCGCATTTTTACGGCGGGAAGTGGCGCATCGGACGCAAATAATGCCGAGCCAAA
CCTGTATCGTCCCCTATATTTTAGGCGGGAATGAAGCCACCCTTGCCAAAGCGGAATACC
TGCAAAGGCAGGGTTATTATTGCCTGCCCATCAGACCGTCGACAGTACCCAAAAACACAT
CCAGAATCCGCCTGTCTTTAACGGCAGATATGACAACGGATGAAGTGCGGCAGTTTGCGG
CGTGCCTGTAAGGATATGATATGGAAACAAAATTTTACAATCATCAAGGCGGACATTTAA
TCCTGTATTTTGCAGGTTGGGGAACGCCGCCCGATGCTGTAAATCATTTGATTTTGCCGG
AAAATCACGATTTATTGATTTGCTATGATTATCAAGATTTAAATTTGGATTTTGATTTTT
CCGCCTATCGGCACATCCGTTTGGTGGCGTGGTCAATGGGCGTTTGGGCGGCAGAGAGGG
CATTGCAAGGAATAAGATTAAAATCCGCAACGGCAGTGAATGGCACAGGTTTGCCTTGCG
ATGATAATTTCGGTATCCCTTGCACCGTTTTTAAAGGCACATTGGAGAACCTCACGGAAA
ACACCCGTTTAAAATTTGAACGCAGAATGTGTGGCGATAAAGCATCTTTTGAAGATTACC
AACAATTTCCCGCACGCCCGTTTGGCGAAATTCATCAAGAACTTATCGCACTTTTTGCGA
TGATCGGGCAAGATAGACGTACAGATCTTATCCGCTGGACAAATGCCTTGGTCGGATCGG
GCGATAAAATTTTTATGCCTGCCAATCAGCACCGATATTGGACACCGCGTTGCACCGTTC
GGGAAATTGACGTCGGACATTACCTGTTTTCAAGATTCACCCATTGGTCGGCACTATGGA
ATCACTGACTGCCATAAATAAATCGCGCATTCGGCAGGCTTTCCAAAAAGCATTAAACGA
TTATGACCGGCACGCCTTAATCCAACAAAAAATGACGATTAATTTAATGACGCATTTGCA
AGATTATTTGCCGGATATGCCATTGGAAAACGTGTTGGAATTGGGCTGCGGCTCAGGAAT
GTTGAGTGCCTTGCTGCAAAAACAGATTTCAGCGAATTATTGGTTATTTAATGATTTGTG
CAATGTGCAGCCCCAACTGGCTGAAAAACTGCCGCAATCCTTTGATTTTTATTGCGGCGA
TGCGGAAAACTTTCCTTTTCAACGACAATTTGACTTAATCGCAAGCGCATCTGCCGTGCA
ATGGTTTCATCAACCCGACGCTTTTATCACCCATTGCAAAACAGGCTTGAAAACAAACGG
ATTATTGGCGGTTGCAACCTTTGGCAAAGACAATTTAAAAGAAGTCCGCCAAATTACAAA
TATAGGCTTAAATTACCCGACTTTATCCCAATGGCAGGCTTGGTTAGCCAAAGATTTTGA
GCTTTTATGGTGTGAGGATTTTACGGTAATACTAGACTTTGATACGCCGTCAGATGTACT
CAAACACCTTAAATATACAGGCGTAACAGCCACGAACCAAAAAAATTGGACAAGAAAAAA
TCTCAATGGATTTATTGGCGATTACTTGTCGGCGTTCGGTATGCCGTCGGGCAAAGTGCG
CCTGACTTATCATCCGTTATTTTTTATCGCGCGCTACTCTGCGGCGGGCAGGCAATAAGG
CAGCTTATGGGCAAAGTTATTTTTATATCGGGTATTGATACTGATGTGGGTAAAAGGTAA
TATGGCGAGGCTTGTGCAGAAGGCATATTGTTAAACGTTAAATTATGGTATGATTTAAAA
CTTACAAGTCTATTTCAGTAAATCGTTAATAATAAAAGCGGACAATGGCCGTTGCAGGCG
GTCAGCTATCCGCTCGTTACGGTATCGGGAGTATCCTTTGGAAAACTCATTAATTATCGG
TTTGGCACTGGCGGTATTGCTGATGCTTTTGGTTATGCGTGCGAAGCAGGGTAAGAAAAC
ACCGAAGCGCAAATCCCAAGGTGTCGGCAATACGCAGGGGCAGACACCCGGAAGCAATGA
TTCGGACTGGGTCGATCAGATTGGACAATCGGTTTCAGACGGCACGCAACCCGACTGGTC
TTGGAACGAAAGTGCCGAGACCGCATCCGCCGCCGTATCCGCGCAAGAAGTCGATCCGCT
TACGGAGTATCAGGTTTATAAGCAATTCGGTTATCAGGGCAAGGCTGCCGAATCTTTGGC
TGCCTATCTGGACGGCATTCCGGATGGTGAAGCGAAACCTGAAAACCTTATCCGCGAGCT
GCTCGATATCAATCTCGAAGTGGGGGATGTCGATGTTTTGGCAGACAATCTGCAAAAATA
CGGCAAACTGATTCTTTCCGAACTTTTGGCAAAATATATCGAACAGGCATTACAGCGCGA
TTCAAACCATTTGCGTATCCGCGTCTTGGCGGAAGAAGGTTTGGGATGGGGTACTCAGGA
GATTGAAAAACGTGCGGAAGGCGGTTCTGCGACGGCAGCTTCCGCATCGCCCCCGCCGGA
TGCCGGCGGTAAGGCTTATGAAGCCGAAGAAATCAAGCGCATCCCGATTGTGCGGGGCAA
AAAAGACGTGTCCGGAATCAGTCAAGAGGAAATCGGTGCGATTGCCGGTTTGGTCCGTGC
CGATCAAGGTGCGAAAATCCTTAAAGACAAAGTCAGCTATGAAACGGCATCGAAACAATA
CGACCGTGCCATCCAAACTTCCGAAAAACCTGCAAACCTGATTATCGATGCGTTGAAACT
CGATTACCAACACGCGGACATAGACCGTTTTGCCGGACATTTGTGGAAACTTTACCAAAC
GTTGGGCAACTACGGCAGGCAGGTTAAAGAGCGGATGCTGGGGTGGGGGTACAGCTTGGG
TTACCATGAAGTTTTCGATGATTTGGAAAAAGGGCCGAACGACCGGCAAATCAAAGACAT
CGGTATGGGGCACGGGTATCTGCCGAAAAATATACAGAAATTCAAATCGCAACATCGGGA
TTTGGTGCTTCAAGATTCTTCGTTGATTAACACCGGTTCGTCTCCGGCAGACGATGCGGT
TAAGGAAGTAGAGTCGTTGCTGATGTATGGTCAGATTGAAGCGGCAATGGATGTGTTGGA
GCAGGCGGTATTGAAATATCCCGACGAGTCCCAGCTTTATATTACGTTGATCGATATTTA
TGAACGTACTGAAGATTGGGATAGGTTGGGGCAGTTTTTAAGGGTATTGAGGGAACGTGC
GGACAGGCTTCCTGAAGAGGTCGTTATGCTGATGAGCCGGCTGCTGCAGCGTATGAATCA
AAATATTAAAAAAATAAAACGGTACGGAAAATAAAAATGGAAGTTCAACTGCCGAAAATT
AAAACAGTACGCGTAATGTTGGCGGGGATGACGGCGCAGCAGGAATCCGTTTTCAAAATG
GCATTCAAAATGCACAATACCACCCGTTATGAAACAGTATCCCCTTCAGACGGCAGTGCC
GTGCCCGATTTGGTTTTGGCGGATACCGATGCCGAGGGCGGTTTTGAACTTTGGAAAGAG
CTTGCCGAGCGTTATAAGGATATACCCGTCGCCGTCTGTTCGGAGAAAGTTCCCGATTCT
GAAGTTCCCTACCTGCCCAAACCGATTCGGTTTGAAACATTGTTTCCTATGCTCCGCAAA
TTGTTGCAGGGCGAGAATGTTTATGGGAAATCGTTTATTGCACCCGCAGACCGGTCGGCG
AAAAATAACGGGAATGTGCAGCGTACGGTTACGATACGCCAGTTTAACCCGAATAAAGGA

## Appendix A

TTATTGGGGGCGTTGCGGTTTGCGGAAAAGAACAGGCAGGACATCGCTATCTTGCATGGA
AATAAGCCGGTCCTTATTGTTTTCCCCTCGATACAACGGGTTTTGCTGACAGAAAGTGTG
CAAAAACTCGAAGAATTGTGCAAAGACGAAAATTTGCAGGTCAGCTGCAAGACTGTTCCC
GATAACCCGCAATGGCGCGAAAAGGCTAAAGTAGGCATTATGTCCTGTATGTGGCAGTTT
TCCATTTGGACAGCGCAGGGCAGGTTGATTTATCCGATTTCTCCCGATACTCCGTTTACG
TTGAAATCTTGGCCAAACCTGACCCGGTTGGCAAATGTGCCGGGGTCGATACGCTTGTCG
GCATTTCTGACCAAGGCATCCGTCAACCTTAACGTGTTGTATAAAGTGATGCCTTTAAAC
CTCAATGATATTCTGAATTATCTTGCGGCAACCTATACAACCGGGTTTTTGTCGGTAGAT
TTAAAAACGGTTTCACAACAGGCATACTCCGATATGGCGGATAAAATAAATATCGGAGCC
GATTCTGCCTCTGATAGTGAAATGATGAAAAAAGCGGAAAAAATCACAACACCATCCCAA
TCCCAGTCGCGCGGCCTTCTGCAAAGGCTGATGAAAAAACTGTTGGGCAGCTAAGAGGCG
GAGAGATGAGAGAAAATAAAATTATTTTCACAGGACCTGTCGGCGTAGGGAAAACCACTG
CCATTGCGGCTATTTCGGACGAAGCACTCGTTCAGACCGATGCTTCCGCATCCGATATGA
CTTTGGATAGGAAAAGGAATACGACAGTGGCGATGGACTACGGGGCCATCAGCTTGGATG
AGGATACCAAAGTCCATTTATATGGTACGCCCGGTCAGGAACGGTTCAACTTTATGTGGG
AAATCTTAAGCCAAGGCAGTATGGGTTTGGTCTTGCTTTTAGATAATGCCCGAACCAATC
CGTTGAAAGATTTGGAATTCTTTTTACATTCGTTTCGAGGGCTGCTGGAGAAGGCACCCG
TCGTTGTCGGTATTACCAAGATGGATATACGCTCTCAGCCCGGTATCGACGTGTATCACA
AATATCTTGCAAAACATAATCTTAATGTTCCGGTTTTTGAAATTGATGCCCGTAAGGAAG
ATGACGTAAAACAATTGGTTAGCGCAATGTTATTTTCTATTGATCCGGGACTGGAGGTTT
AATATGGAATCAACACTTTCACTACAAGCAAATTTATATCCCCGCCTGACTCCTGCCGGT
GCATTTTATGCCGTATCCAGCGATGCCCCCAGTGCCGGTAAAACTTTGTTGCACAGCCTG
TTGAAAGCAGATGCGGACGAAATGGTCAGCAGTGAGAAGCTGCTTACTTGGGCGGACACC
GCCGACATCGATACCGCTTTGAACCTGTTGTACCGTTTGCAAAAACTCGAATTCCTCTAT
GGCGATGAAAACGGTCATTCAGACGGCATCAATTTGTCGGACGAGCAATTGCCGTTGCTG
ATGGAACAATTGTCCGGCAGCGGTAAGGCGTTATTGGTCGATCGGAACGGTCTGTATCTT
GCCAACGCCAATTTCCATCATGAGGCGGCGGAAGAGTTGGGGTTGTTGGCGGCAGAAGTC
GCACAGATGGAAAAGAAATACCGGCTGCTGATTAAGAACAACCTGTATATCAACAATAAC
GCTTGGGGCGTTTGCGATCCTTCCGGTCAGAGCGAATTGACATTTTTCCCATTGTATATC
GGTTCAACCAAATTTATTTTGGTTATCGGCGGCATTCCCGATTTGGGCAAAGAGGCATTT
GTTACTTTGGTAAGGATTTTATACCGCCGTTACAGCAACCGCGTGTAAAACTTGGGAGAG
AGGAGGGGTTATGCAGCAATTATTGATTTCAATCCTTGAAGATTTAAACAATACATCTAC
GGATATTATCGCGTCTGCCGTTATCTCAACCGACGGATTGCCGATGGCGACAATGCTTCC
TTCACATTTGAATTCGGACAGGGTAGGGGCGATTTCTGCCACTTTGCTTGCTTTGGGGAG
TCGCTCGGTGCAGGAACTCGCCTGCGGGGAATTGGAACAAGTGATGATTAAAGGAAAATC
AGGCTATATCCTTTTAAGTCAGGCGGGTAAAGATGCCGTGTTGGTGCTGGTGGCAAAAGA
AACCGGCAGACTTGGTTTAATCCTATTGGATGCCAAACGTGCGGCAAGGCATATTGCGGA
AGCCATATAACATATAAAGATTGCGGGCTTGCAGATAAAGTGCAATCGATTGTCAATTTA
TATTGACACGTTCGGTATTTCTGTTTTATTATTCGCGCTTGTTCCCCGATAGCTCAGTCG
GTAGAGCGACGGACTGTTAATCCGCAGGTCCCTGGTTCGAGCCCAGGTCGGGGAGCCAAA
TTTCAAAACCCTCTAAGTATTTTCTTAGAGGGTTTTGTTTTACCGGCGGTCAGAAACGCA
TTTTTGAGATGATTGTTTTGAGATGGAATAAAATCTTTGCAAAATTCCTTTCGTGATGGT
TATGAAAAAATAGGGGCTGTCCTGGACAGCTAGGATAAACTCGATTTTATAGTGGATTAA
CAAAAACCAGTACGGCATTGGCTCGCCTTAGCTCAAAGAGAACGATTCTCTAAGGTGCTG
AAGCACCGAGTGAATCGGTTCCGTACTATTTGTACTGTCTGCGGCTTCGTCGCCTTGTCC
TGATTTTTGTTAATCCACTATACTAATTGAGACCTTTGCAAAATTCCTTTCCCTCCCGAC
AGCCGAAACCCAAACACAGGTTTTCGTCTATTTCCGCTACCAATCACTCCCTAATTCTAC
CCAAATACCCCCTTAATCCTCCCCGGATACCCGATAATCAGGCATCCGGGGTACCTTTTA
GGCGGCAACAGGCGCACTTAGCCTGAGACCTTTGCAAATTTGTCGGTTTCGGGGTCGTAT
~~TGGTAGCCTCGTGCCTGTATGTCTTCTTTGAAAGTTTCGTATACGTCGTGGGCTAAAAGG~~
GCTGTTCCGACATAGGGAACCGCCCTTGTGCTGAATTTCGCGCCTAAGCGGGCAAGTTTG
CCGACCCCCGCCAATACGCCGGCGCGGATACGCTGGCGGTTATTTTGGCGTTGATTCGG
GCTTTTGCGCCCGTAGGGATGTGTGTTAAATCTACCGTTTTTATTAAATCAGATGAATAA
GTTTTACTATTTTTAGGTACAAACTTATGAATTTTCGCACCTTGTCCGGTATCAACTGAA
ACAGTTTCAGATATTTTTACTGCATTTGCATTCGCTTCAAACGAATACATCATCAAAATT
GCAATTATCGACAATTTCGCAAAATTCAAATTTGTATATTTTATGACCATCTTTCAGGGA
TTCTTTAATTACCATTTCTGAATTATCAGAAAATGAGATTAGCCAAATATCATGTTTAAT
TCTTCTATTCCAGAAAAAAGAGAAACAATCAATAACATTTTCAGACTTATTAATCTTCGC
AAATTCAACAAATTCAGATTGCGCTATAACCGCCATCGATTGCCCAAAATACTTGCTGGA
CGGCTGATATTTATAAAGTGCCAACTGCGCCTGAGTGATAAACGGCTTGTTCATGGTTCT
GCCTTTCAATGATTGTTTTGAAAGCCTGATTTTGACACCATAACTTCATGCGCTCAATTC
TTAAACAGAACCGCCCCGATTAATACGGGTACGGAAACGCCGAGATAAAAATAAAAATCC
ATCATTTCAAAACCTTTTTCAGCAGGGAAACAAAGTAAACGGACGCGAGGATGCCGAATA
CTATCCAGCCTGTTTCAAGACCGCTTTGCAGGTTGTCTTTCGGACTGCATTCCGCCAATA
AAAGCCTTAGCGGCTGACCGTCCGACATCTTCCACAGGCTGCCGTTATATTCCGGCCTGA
CAATCTGTCCGTTTTCTTTGATTCTTGGTACTACCAAGCTGAAATAAAGGTTTTCAGCCT
GGTGCTTCTCAAGACATTTATTTCCGACTTGGTAGTACATGCCGTCTTACTTCATCACTC
TCTTAACGATGGAAAATACAAAAAGCGCGGCGAAAATGCCCACTACAATCCAACCGGCTT
CCATACCGTCCGCTTTTGCGGCTTCCAAAGCGTTTTTTGCCGTATCGGGCAACGTTGCAT
TTGCATGTGCGGCCAAAGCCAGGGGAGCAGCTGTTACAACAGCCAGTTTTGCGCCGTATT
TACGGCAGGTGTTAATAAATTTCATGATATTTTCCTTCAAAAAGTGTTTGGCGGTAATGG
ATGGAGCGTTTTTCAGACGACCGCCGAACATCCGAAAATCAGTCTTTCAAAAATCCGAAT
ACGACAAATTCGTATTGGTTGCCGATTTCTTCCAAACCTGCGTTAATCGCTTCTTCGAAG
TcGTAGAAATAATCGGCATTGGTGATTAATTTGGTATGTCCGATGTCGCCCGTTTCAGGA
GAGAGATACAGAAAGTCCCCTGTTGATACGGACTGGACAACATAGACTTTCTGCATTCAA

# Appendix A

TCAGCCTTTCTTCACGAGTTGAAAACCGATGACTTTCAGTTTTTGGGTTTTGCCCGTAGT
GACGATTTCTACGTTCAGGTTTGCTTCGATCGGAAATTGGGCGTTTCGGAACTGCTCGAA
ATTGGCAGAGCCGCCGAAATCGTATTCAGTAGTAGAGCTGCCCAATGCGTTGCCTTGGGA
GCTGTCTAAGGGTGTGGCGACAATCAGGCAGCAATAGTCGAAGCTCTTGCCTTCGATTTG
TCCGTTGATTTTTTTAACGCCGACGATGTGGCCTTGAAGTTGGATGTTCATTTTTTGGTT
TCCTTGTGTGATTAAACGTCTTTCGGGCAGACACTTTAAGCCCATGAAATCGGTAGTCTT
GCGAATTTGTCGTAAATGAAGTTGTTATAGCTTTCTTCATTGTTGACGTGTTTTTGCTGT
TCAAGCTGTTTTTCAAGATTCTCGTAATATTCGTACATATAGTAAGGGTCTTTGTACGGT
TTGAATGCGGGCTGTTCATGAATGGCTTGAGCTTTCAAAAAGGCGCAGTCGTAGGCTTCG
GGAGCCAAAGACTTGGGCAGCTTGTGATGACTCGGCTCAATCAGTTCAAACAGTTTGGCT
TTGTCCAATTCGGGAAAAATGAATTTCAGACCGTTTGCCGCACGTCCGAACTGTTTTTTT
ACCCATTCAAGGTAGCGGTCGGCTGAAATGACCTTATCTTCCTTAACCGCGTGTATGCGC
GTTGCCTTTTGGGCGAATCGTTCGCAAATCGGATATGCGCCGCCGAAATATTCGCCCGGA
TTCTGCAAAACTTCGAAAGGGATAACGATGTCTTTTGCTTTGAATTCAATTTCAAATCGC
GTCCATGTGCTTGTTTTATCGCCCAACTGCTTGCCTTTTTCATAGACGCGGACATATTTG
GACGATTCACGGGAGCCGATACCATAGGTCTTGCCTTTGGTCATTTTGGCTTCATCGTCT
TCTTCCCAATCTGACCCCAAACATTCGCCTTTTGGTTTGACGTGATGACAGGTAAACATA
CCTTTATTTCGGTCTTCACGGGCTTGGTTCGGGCTGTATTCGCCGTTGAAAAAGTCTTTT
GCGATGTCAACGCGTGTGATTTTTGGGCGGATTGCATTAGTCAGGAATGCGAAAAGTCGT
GATTCCCAGCCTTCTTTTGCGACGCCGCAACCGGTGCCGGTCAGTTCGAAAAGAATGGTA
TTTTGTTGGCCGCCAAAATGGACGCGACCGTATAGGGCGTCTTCCGAACCCATCAACCAA
CAGCGCTCATAGAAACGACCGCCCGAACCTTTGGATTCTTTGTAGATACCGAAACCGAAA
ACTTCTTCGGCGAGCATGGACGCGGCGCGAATAAAATCTTCGTCTTCCAAAAGACTTACA
CGAACGCCGTATTTATCGAAAAAGGTTTTTTCATGAAATGAAAAGCTAATTTGATCAATG
AAAGCCGAATCTGATACACCGCGCCGAAGAGGAACGCCTAACAGGTTTCCTTTACCGTCC
GTTATGTACGTTTCGTAACATTCGAAGACTTCCTGAACCCTGCCTGCCGTTTCGGTTTCT
GTCCCCCCCTGTTAGATAAGGGGGGAAGATTTGAAGCGGTTGTCGGCTTCCTGCCGTCCG
CTAGCGCGTCCGTCATCACGCCGGCAACCGCCTTTGTCATCCCTTGCTTATCTTCCATGG
TGCGAATCCTCAAAAACGGGCAAAAAAAAGCCCTGTTACTTGTAGAAAGTAAAGGACGTT
AATTTTTGTTAATCGTCCCTTCTTAGGGACGCAATATATAAGGCCGTCTGAAACGGTTTT
TCTGTTTTTAGACGGCCTCTTGGCTTAGACCTTGAGAACCGCATGCGTGCTTAATTTATT
ATCTAATGAAAAAAGTTTCCGGCTTTCAGACGACCTTTTGTAATATTATCGGCAGCGGCT
CAATGCCAACTTTAAACCTGCTCCGATTTCTTCAGGGCTGTTATCCAATGATAAAATTAC
ATCGTCTGCATCAATGGCATCCCACGCTTCCAGCTTGACATGGCGGCTCGGGCTGATTTT
CAGGCAGCCGTTGTGCAGCCAAATATCTACGCTCATCATGTTTTTAAATAGGGCGCGTCT
GGTTTTATAGCCCAAGTTCCCGCATAGCTTGGCAACCCAATCCTCATAGCGTTGCCGAAT
TTTTTCGGTATCAAAAAAATCTTGGTCTTCTGGACTGTCATAAACGAAAGTCCTGCTGTT
TGCCAATGCTTGCAAGACCGTTGTGCCTAAAGTTTCATTGTCGGTATCCAATGGCAGGAT
ATGGGGGGGATATAGGTGGTCTGGAGCATATCGCCCAAATCCTGACCATGTTTGAATAAT
CAAGGCTCTTTCATTTGCCTTATAGCCAGCCCAATAATCTTGTTCTTGATTGAAAGTCAT
TTATTCGATCTCCGTAATTTTGACTGTAATGTTTTGACTTTTGCCATACTCTACCACACG
TTGCAACTGCAATCTTTGCTCCTTATTAGTTTGTGCGGGTATGGCCAGATGGATTTCGCG
CTGTTTGATCATGTCTGCCCTTAACGGTACTTCTGATAATTCATAACTTTTGAAATTTGC
CGTCTTATCGATGTACCCTTTCATGGTACTGTAAAGCTGTTCGGGTTTGGACAGGCGTGC
CGTAGTTTGCGTATCCAGAGTTTTGGCACTGATTGCCGTGCCTGTACCACGATCAAAATA
ATCAAATGTTTTAAAATTTTTAGGTAACCTTGCATTGGCAGACAAGCCCTTACCGACATA
ATCCTCCCAAGGCATTCCCTGTCCTTCAATCCCCTTGCCCCACTTGATACCGACTTCGGA
TTGGGACAGGATATTTCGCTGTACGTCAGCAGTTTTCGGAGTCAAGGAAGTTTTCACACC
CGTTGCCAAGTTTCCCAATTTGCGCGTAATCAGCGTTTCCAATCCCCATACGGCTAGATT
~~TTTCGCATCAGATAGTAACGGCGATTCGTATTCTATTGGTGTACCCAAAGATAGGACAAC~~
TTTTTTACCAAAATCGGACTGGTAAGTGCCAAACAATTGCCGGCTTCCTTCTGAGGCCTG
TGTATAACCACCGGTCATACCTGCCGTTGCAATAAGTCCACCGGCCGCACAGCCAATCCC
GGTACTGCACAGACCTCCGCCTATAGCACCCGAACCGACAAAAGTCGTTGCACCCAATCC
CATATTGCCCGCACCCTTAATTTTGGTGGCAGCACGGTCGTAACTGCTGCGTATATCATT
CAGGCTGTTCCATGTTCCATATTTAAATGCATCCGTCCGCATCAATACGTTTTGTTCCGC
TACAAACTGTTTACCGGCATCTTGGAGGTTTTTTAGTCCTTTATAAAGAGGGTCGAAGTC
AGGTACGCCTTCCGCGCACCGGGTTAATGCACATGCAGCGGCTTTTAGGCGGTACTGTTC
TTCAGCCGATTTGCCTTTTGACAGTTTGTTAAGGATTTGTGTTTCTTTCGGATGCAGCTG
CCTATTGTTCCAATCGACATTCGCCCCTACTGCCGCCGTACCGACATTCCCGCCCGCCGC
ATAGCCGATTGCCGCCCCGCCCCGTGCAGTATGCTCCTGCCTATGCCGCCTTCTTTCCAG
GTGTCGTAGCGGCTTTGGTTTTCGGCAAGATAGGCGTTTACTTGGCCGAGGGATGCGCGG
AAGGCGGCTTTTTCGGCTTCGCTGTCCGTGTTTTGCAGTTCGGCCTCCAGCAGGGTTCGG
GCTTCCTGATACCGTTCGTAACTTTGGGTATTGCCGAGTTTGTCGGCAACGGCCGCTACG
GCTTGGGTGGCGTTTCTGCCGAACTCCTTCGTTACTTCCCTTTGCAGGTTGATCTCTTTG
GCGACCGCGTCTTTGTCGAAGCTGTTTTTCAGACGGCCTGAGTGTTGATCCGCAGTTTCG
GTGTCGATGCCGGTGTAGATACGCGCTTCGGTTTCTTTTGCAGTCCTGCCTGTTCGGGCA
AGTTGTCCCGCTTCGTCGGTGATGTGTATGTTGCGGGTGTTGATGCCGCTTTTCGTGATG
CTGCTTTGACTGTCGCTGTCGCTGCCGTAGCCGGCTGCCAGGCTTATCCTGTCGGTAGGT
CTGCCTTGTTTGTCGGTAACCGTGCCGTCCCAGCCGCCGTTCAGGTCGAAACTGCCGCCT
ATGCCGAAGCTTTTGCCTTCGTAGCGGCTGTGGTTTTGAATGTCGCTATGGGTGAGGGTG
GCCGTCTGAAAAAGGTTTTTGCCCTTATCTTCTGCGCTTTGGCTAGACGTGATGATACCG
CCCTTGAGGTCTGTGTTGTCTCTGACTTTGATTTGATAGCCGTCTTCTCCGGCATAAATA
CCGCTTTGCTCGGTTACCGAAGCATGGTCGGCTCGGATTTTGCTTTGGCTGTAATCGCCA
CTGGCACTGAAGCCATAACCTACGGTCACTTGTGCACTGGCGTTTTGTTGTTTGCTTTGA
TAGGTTTCAGTATCTTGAACACTTTCTATATGCAGGTTGCGCGTATCTGCCTGTATGCCT

## Appendix A

TTGCCGATGAGCTGCGCACCTTTGAGGGTGGTATCCCCGCCGCTTCGAATGGTAGTTTTA
CCGGTTGTGCTGCCGACATGGGTGTGGCGGTGGGTTGCTTTTGTCTGATTGCTGTGGTGG
CTTGTTGAAAGAAAGGCTGTCTGAAACGTATTTGTTGTTTCAGACAGCCTCCTGGCTCAA
ACCTTGAAAACTACATATGTGCGTTCCGCACATCCTACGTATTGAGTTTAGGTTTCACAT
GAGCTACGGCTTGCTATGCCGTCTTTTTTCCAGGTGTGGCCGCGGCTTTGGTTTTCGGCA
AGGTAGGCGTTTACTTGGTCAGATGGCGGATTTTTTGTTCGTCGTAGATGATGGAGACGC
TGATACCGGAGTAAGCGTAGATTGGGTCTTGACCTCAAACCTACACTTGTTTTACATAAA
ATTTCGTGTCTCTATTTGAAAAATCTAAATAACAACATTCTACTTTACCTATTGAATTGA
TTATAGTTGAAACAGGAATATTAAGAAGCCTAATACCCAAATCATCAATTTCAAAATCAT
TAATTCCACTCTTATAAAGATAGCTTATTATTTCATCATTAATTTTTCCAAGCCAATTAA
AAGAAATATCTTCTAAAAAAAACTTATTTGGTTCAAATATCTCTATCGCTTCAAGCTGAT
TTTTATCATCATAAAAACAATGGATATTCAATTCGGGAAAAACATCCATAGGAACCCGAG
AGTATGATGACTTATAAATTTCTTGTACATCAGAACTAAATATTGCACGAACTTGTTTTC
GATTCAGATTTGATGAGATAATATTTCTTTCTATTTTTTTTGAACTAAAATAAAAAATTG
CCATAATTTTTTTGCCTAAACAATATTACCATTTTCGTAAGATGCATAGAACAAACCATG
TCTTATCCATTTTGTTCCATCGGCAGACAGATAACGACTATATCTAAATTTTATTTTTCA
CTCTCATAAAAAATTTTCTGCAATATTCAATATATTTACTTTCTTAACCATAGCGTAAAT
TCCTCAGGCTTATATATTTCAGTATAAGTATGACTTAAAGGATATGACGCCGCGTGTTAC
GAGTTGCTTCTTTTGATTTCAGGGTTTATATAAGTTATGGCTTGCCTGGGCTCGAAGTGA
TAAAGAGAGTATTTACTTTTCAACTATAAAAATATGAGATAGTTCCATGGGAAAACCGTA
ATTTAAGTTTTAATAAAGCACCTTCTAGGCGATATAAAAATTTTCTATAATTTTCATTTG
GTTTATATTTATATATAAGCTGTATTTCAATAGTCTCATAGCTACTTTCTCCAAAATCTT
CAAAAATTTGACCTGATTCAATATATTGAATATATGAATTTATTTTTTCTTGCAACAAAA
ACAAATGCTCGTTATCCCATTTTAAGTGATCAGAAATAGTTAATATGAGTATTCCGTTTT
CAATAGAGGCTGAATCAATTACATTCTCTTCCAAAATAGACATTATCTTTTCCTTTCAAT
TATAACTTTAGTAGGTTCAATTTTGGTCCCCTTTGGATAGCCCGGTTTTCCCTTACCGAC
CACTGTTGCTCCCGTTCTTTCAATTTCAGGAAAAGCTTTTTTCTGATTTTTAGTAAGTGG
CGCAGTTATTGAAGCCTTACACTCTGTACAAACATCAAGACCACCTTCTTTCGAAATAAT
ATCAAGCCTAGTTTTTACACCACTTTTTGTTTTAACTGTAATCTGTCTTTGCGGTTTAAA
GCCTTGTTTAACTTTCTTCTGATAAATTTCCATCTCAAAATCCTCACCAGATTTTTTATT
TTTTTCCAGTTGATCTTTACGATTTTTATGTTTGATTCCCTTGCTAGCCAATGCCGTATC
CGGAATCCTGTCCCCCTTCGCAACATTGCCGTTTGCAGGGATACGGATATTCCCCGCACC
CGCCAATAAGGGATCGCTGCCGGTAACTGTCGGCTTGATGTTTTTCAGGTTGCGGATGCC
TGCAAGAATCGGGACTTTTATCCTCGGATTGGGGTTGACAAGGCTCGTCAGTCCTTCGGC
AACGTTCAGTGCAAACTCTTCGTTTTTCCGCTCTCCCTGCCTGATTTTGGTGTCTTTCGT
CCCGCTGTGCAGCCAGGTCAGGTTGCGGTATTCGGGTTTGTCCTGTCGGCGGTATTCCTC
AAACAGCTTCGGATCGTTGTAGGTTTGCGGATTTCTTGCGCCGTAGTCGCGGTAGTCCCA
AGTATAACCCAAGGCTTTGTCTTCGCCTTTCATTCCGATAAGGGATATGACGCTTTGGTC
GGTATAGCCGTCTTGGGAACCTTTGTCCACCCAACGCAGTATCTGCCTGCGGATTCTCAT
TGCCGCTTCTTGGCTGCTGATTTTTCTGCCTTCGCGTTTTTCAACTTCGCGCTTGAGGGC
TTCGGCATATTTGTCGGCCAACGCCATTTCTTTCGGATGCAGCTGCCTATTGTTCCAATC
TACATTCGCACCCACCACAGCACCACCACTACCACCAGTTGCATAGCCGATGGCCGCACC
GCCCAGTGCGTTGACCGCCGCTTTGCCCGCCGGACCGAGGTTTTCCGCCGCTTTGTCCAA
ATACGGTGCGGCAAGGGAAGTGCCGCCGCCGGCCAGTATGCCGCCGAGGCTGCCGGTCGT
CAGTCCGCCTGCCGCCCCGTGCAGTATGCTCCTGCCTATGCCGCCTTCTTTCCAGGTGTC
GTAGCGGCTTTGGTTTTCGGCAAGATAGGCGTTTACTTGGCCGAGGGATGCGCGGAAGGC
GGCTTTTTCGGCTTCGCTGTCCGTGTTTTGCAGTTCGGCCTCCAGCAGGGTTCGGGCTTC
CTGATACCGTTCGTAACTTTGGGTATTGCCGAGTTTGTCGGCAACGGCCGCTACGGCTTG
GGCGGCGTTTCTGCCGAACTCCTTCGTTACTTCCCTTTGCAGGTTGATCTCTTTGGCGAC
CGCGTCTTTGTCGAAGCTGTTTTTCAGATGGCCTGAGTGTTGATCCGCAGTTTCGGTGTC
GATGCCGGTGTAGATACGCGCTTCGGTTTCTTTTGCAGTCCTGCCTGTTCGGGCAAGTTG
TCCCGCTTCGTCGGTGATGTGTATGTTGTGGGTGTTGACGCCGCTGCGGGTGGTGCTGTT
TTTGCTGTCTCCGTCGCTGCCGTAGCCGGCTGCCGGGCTTATCCTGTCGGTAGGCCTGCC
TTGTTTGTCGGTAACCGTGCCGTCCCAGCCGCCGTTCAGGTCGAAACTGCCGCCTATGCC
GAAGCTTCTGCCTTCGTAGCGGCTGTGGTTTTGAATGTCGCTGGCAGTAAGGGTGGCCGT
CTGAAAAAGGTTTTTGCCCTTATCTTCTGCGCTTTGGCTAGACGTGATGATACCGCCCTT
GAGGTCTGTGTTGTCTCTGACTTTGATTTGATAGCCGTCTTCTCCGGCATAAATACCGCT
TTGCCCGGTTACGGAGGCATGGTCTGCTTTGACTTTGCTTTGGCGGTAACTGCCGCTTGC
ACTGAATCCGTAACCGACAGTAACTTGGACATTGCCGTTTTGCTGTTTGCTCTGATAGGT
TTCAGTATCTTGAACACTTTCTATATGCAGGTTGCGCGTATCTGCCTGTATGCCTTTGCC
GATGAGCTGCACACCTTTGAGGGTGGTATCCCCGCCGCTTCGGATGGTAGTTTTGCCGGT
TGTGCTGCCGACATGGGTGTGGCGGTGGGTAGTACTTCCCCCTTGCTCTTTACCTTTACC
GATATTTCCTCCGGCGGTAATTCCAAACCTGATGCCGTTGCCTATTTTGACGGCTACGCC
TGCATTCCAACCACTGCTTTTGTTTTTGCTTTGCTCGCTGCCGTCCTGTTTGGCAGATTG
GAGTCTGATATGGTTGTCGGCAATGAGGGCAGTACCTGCATGGCCGATGACATCGGAACC
TGTAATATTGATATTGGACTGCTCCCCACTTCCTGTTGCCGCAAGTGTGGTTTGCCCTTT
GCCGATAATTTGACTTGCTGCCGCTTCGGTGTAATGTCTTTTTTGCTCGTTACGACTTTT
CTGTTCGCCGTAGGTAATGGACACACTGATACTGGGGCTTTGATTGTTGTTTTGACCTTG
TCCCGCACTGCTGCTTGGAGCAAATTGTTGCATTTGTTGGGTTGCTTGATAACTCTGCCA
TGCAGCATTGGCTGCAGCCATGGCATTAACGCGTTTATTTTTACTTTTGCCCACATTTTG
GGCTGCTTGTATGAAGTTTTGTGCAGCTTGGACAACCGGGACATTGAGGGCGACGGTAAG
GCCTTTTTGTTCCTGGGTATGGGCGTAGTCAGTGGCATACCGGTTGTTTGCGAACTCTAC
ATCTATGCTTTTGGCTGTGACGGTATTGCGCCCCTCGGGGCTGGAGACGGTACTGCCGGT
TTGTCGGTAGCGGTTTCCTGCAACTGTAACGGTGTCTCCATTCAGGCTGCCTATAATGCT
GCCTGTATGGACAATATTGGTACGATCAGTGTCATCGGTAGTTTTCCGGTTACCGATAGT

## Appendix A

AAAGCCCAATCCGCCAGTACCCATGACGCCTGATTTTTGCTCTCGTGGTATTCATTGCC
GGTATAGCGATTATGGGCAGTAGAAATATCGATGTCGTGTCCTGCTTTTAAAACAATGCC
CTTATCAGAAATAAGGTTGCTGCCGCGTACATTGATATCCTGCCCGGCTGCAACAATCAT
TTTGCCGCCGCCGATGTTGCTGCCGACTGCTTCATCATGACTGAAGCGGTAGCGGTCGTG
TGTTTTGGTACTGGAAAGGATGCCTTTGCTTTTTCCGCTTACCGAGGTATCCAGTTCGGT
TATTTGGCGTCCTTCGCTGATAGTGACATCACGTCCTGCGGCAAGGACGGTTTTGCCTTC
TTCGGCCTCCAGTTCGCCTTGGCGGATTTTTAAGTCGTTACCGGCTCTAAGCAGTGCGCC
GTTTTGCGTGCGGATACTGCTGCCGACTTCGGTACTTTGGCGGACATGGCGATGGTTCTC
GTCATCTAATGTACCATAGGCTTCGCGATGTTCGGTACGGATGGTGCCGAGGTTGAGATT
ATTGCCGGCGGTAATTTGGGTAGTGCCGTCTTTAACTTGGTTAGAGACGGTGGCCGCATT
GAGGTTGATATCGTTGCTGGCATGCAGGGATAGGATGCCGTCTGAAGTTCTGTTATCTAC
TTGTTCAGTATGGCTTCCGACCACGTTAATGCCGGCCATACGATCGATGGCGGTATTGCC
GTTACGTTCATTACCGGAAGTTTGGGTTGTACCGTTAAGGTTGATATTTTGCGCTTGGGC
AGTCAGCAGTCTGCCTGCTTGTACCTGCCCGCCGTCGATATTGATACTTTTTTCAGCTTT
TAAGCCGATTTGGTCGGCTTGAATGTTACCGTTGCTGTTAATATTCCGTGCCTGGATGAG
TACGGCCTGTCGCCCCGCAATGGTACCGCTGTTAGTCAGGTTGCCGTTTTGCAGTTTAAG
TAAGACTTGTTCGGCACTAATCAGGCCACCGGAGGTATTGAGATCACCTTTGCGCGCCAG
GGCATAGACTTTAGGAACCAGTACGGTTTGAGTCGAACCGTCAGACAGGGTGACGGTTTG
ATTTTCCATCCAAACGATATCTGAAGTTAAGCGGGCAACTTGCTCTGCACTCAAGGCGAT
ACCTGGGGTGAGACCGAATGTTTTGGCAGCAGTAAGGCCGTTGTCCATCAGAGCTTTGAA
TTGTTCTTCATCACTCCTGTAGCCGTCGAGTCGGCGGTAGCCTGTTAACTGATGGATTTG
TTCATTAACAAGTTTTTGTTCGTAGTAGCCGTCGCCAAGCCGTTTGTGTAGATGATTGGT
GTCCAATTGCAGTTGTTGCAACATGTAGTCGCTGCCCAACCAGCGGCGGTAGTCTGCAAA
TTGAGGATCGGTTTCAACCAACCAGCCTTTATTGTCAGGATGGGTGGTATAGAGGCTGCT
GTTAGGCAGAGTAACAGTAGCGTTATTTAACGAGACCACATTACCGGTATGGATGCGCTG
ACCATTGACGGCTGCCGTGGATACTCCGTCAATCAGTTTGATTGCAGATGCGGCGGGTTG
AAAGGAAGGGGAGGCGGCATTCTGTTGGATGACGGATACAGGCGTGTCGAAGTCGTGGGT
AAATAGTTGGGTATCATGGTAAGGAGTATGGTTTCTTTCAGTACGGCGTTGTCTTTTTCT
ACCGCTGTACCATCCTTTTTTTGTAACTGAATCCCACTGTGTGCCGACAGCATCTGTGCG
ACCTTTGCCTGTTGTACTTTGATTGGTAATTTCTTTCTGGTTTAAATCATCAGTGATAAT
ACGCCCGCCTACTACAATCCGGCTGTCTTTGTTCAGCCAATTTTGACCTGAGGCAGTCAA
ATCACCGCCCACAGTAATGTGTGCCGGCCGGTTTTCGATGATGCGTTCTTTATAAGTCTC
GATGTGGTAGTCTCGGACATGCCATTGGTTGGCCTCAATACGAGAACCATTTTTTAAATG
GAACGTAGCAGTAGTTTGGTCTTTTTGTCCTTGCGAGTTGTCGAATAAACCGTCTTTTCC
CGCCTGATAGTAGGTATTTTGCCCCAGTACGGTGTAGTCGCGGACTTGCTTTTCCGCTTT
GGCTAAGTATGTCTCTGTTTTAAAGTGATTATTGATATTCTGCATATTCCGAACGGACAT
CAATGCATCACCTTGTACTTCCAAACCGGCACTGCCATTAACAAAGGTATCGGCCATGCC
TGCCGCATGATGTTGTTCATCCAGTCGATTACCTACGGCAAAAATACCTTCGCTGGATAG
TAGGGCACCTTCTTGGTTATGAATCTCTTTCGCTCCAATATCCAAACGTTTCCTTGCAGC
TATTGCCCCCGCTTTGGTACTGCCTTCCGTCGTTTCTTCCCGGTTAAGCAGTATTTGCGC
GTCCAGGGCAATATGGTTGCCATAGATTTTGCCTGTCCCGGTGTTGGTCAGGGTTTGACC
TGCACCGATGTGGGTCAAACCGTCGCTGTTGATCAAGCCCCTGTTGTCAACATGCTGTTC
GGATGTGATGTCCGTTTGTTCTCCACCAATAATTTTGCCTGTAACTTGGTTATCTATATT
GCCGGCATTGAGTTTGAGCGTATGGCCTGCTTGTAGGGTATGGGTATTTTTCAGACGGCC
TTTTATGCTTAGATTTAATTGTTTGCCTGCAGTGAGGTCGCGCTCTACGACGAAATCGTC
CGTCAAAGCAATATCCAGTTTGTTACCGGCTGTTAATGTGCCATTGTTGGCGAGTGATTT
GGCTTGTAGCGATACATTACCGGCAGATTGAATCGTGCCATCCGCATTGTTTAACGCCAA
AGTGTTTTGATTTTTATCGTGAATAGACAACTGCCGGTTGGTGGCAATTTCACCATGTTG
GTTGTATAGGCCGTCTGAAACAGCTAATTGTGCTTGGTTTGCAGATAGGAGTTTGCCGTT
TTGGTTGTCTACATTTCGACTATTGATAGTCAGTTGTTCGGTAGCAGTAATATGGCCGCT
TTGGTTAGTCAGTTGCTGACTTTGGATGTTAACCGTTTCAGCCTCTATACGTCCGCGCGT
ATTATCTAGAGTCTGACCTTCGGTATTCAGTCGTGCAACAGAAACTTTTCCTGTATTGCG
AAGATTATTCTTGGTGGTAACGGTTCCACTATCGGCAAGAATGTTACCTGCATTATGTAA
ACCGGCGGTATCAAGCTGTAAATGTGCAGCTCGAATATTGCCTTTTTTATCATTGCTCAA
GCGGCCCGAATGAATGAGTGTCAGATTGTTGGCGGCAATTTCTCCGGCATTATGCAGTTC
ACGGTTATCAATCTTGCCGGTTTGAGTTAATAAGTGACCGCTGTTTTTAGCAGTTTGAGT
GTTAACAGCCAGATCGTGTGCCTGGAGTTTGCCTTTTACCGTATTGTTAAATGAATCGCC
TGATACTCGTAGTTTAGCCGCATTCAGACTACCCGAATTTCCCAAACCGTTTTGGGCGGC
AATGTCAATTTGCCCACCCGCATTAATTGATCCTGCATTGTCAAATGCTCCTGTTGTTTG
AATGCGTCCTACGGCGTAGTTTTTTGCAGGTGCTGTAGGTGAAACGGGATTGTTTGAACC
AGGCTTAGATACCGAGACAGTGCTGCTGCCTGAACCTGTTGCAGTACTCGGAATCTGTGG
TATGACTGATGGATTGGGATTCAAACCGGTCTGTGGAACGTCACTTACACCAATCTTGCC
ACTGTTATCGAATTTGCCGGCAGATACCAAATCCAATGCTTGTGAACCTGTTTGAGTAAT
ATTACCTGTGTTATCCAAACCACCTGTTGACATATCTAAGCGGGCAGCCTGGATCGTACC
GTTGTTTTGGTTTTTCAGACGGCCTAAATTACGAACAGTCAATCGACCTGAGGATAAGAC
CGTACCTGAATTGTCCAGCGTCTGGCTGTGAATATTGGCATCATCCTGTGAGGCAACCGT
ACCGCTATTATGAACATTGCGGGCATGAAGTGAAACCGCATGATTTTCTCCCGTCGCTGC
AATCATGCCCGTGTTGACCAGTTTACCCTCAGCATTCACTGCCACATTGCCGGCTGAGGC
AAACCATTGCCCTTGATTACGAATGCCTGCTTGCTCGACCGTACTGATCAAGGTGATTTT
GTTGGCATACATACCTCCTAATTTGCCTGTATCAATCGCAAATAAAGGGATATGTGTGCC
GTTGTTGGCTGTATTGTTTGACGTATTGGCAGCAGCATTATTGAGAATAGGCGAATGTGC
ATCACCTGTTGCGGCCACATCGTTTTGTCCCGCGACGACACGAACATCTTGTCCCCATAC
GGGTGCATCAATTTTGGAATGATAACTGAGAATACGTGTGTAATCGGTATCACGTGCATC
-CAAACCGTGTCCGGCGATTACAACATTGCCTTGCCTTATCTTAAAGCCGCTAAGGTCTCC
TGCTTGATATTGCGGTTGGGCTGTCGTCAAAGTGGCACGGGAAGCATTGATAAAACCACC

# Appendix A

ACCATTGACTGCAATCCCTGCCGGATTGGCAATAACGACTTCTGCACGTCGTCCGCCCAC
TTCAATATAGCCATTCAGTTGTGAAGAATGGCTGCTGTTGATTTGGTTTACAACCACACG
TGCTTCGCCCCTTGCCAACCAAGGATTGCCTTGAATCCAACCGCCTAGCTGTGTTTGGGT
GTTGCTGCGACTGTTGTTTAAAATCGCCCCGCGATTACCCACATCAAACTGGGCGTATTG
ATTAACAGAAACCCCTGCCGAAGTAGGGGTTTGAATATTGACTTGCGGTATGCCGTTACC
TGTTTGCAGGATGGTAGGCTGTTGCTGTGCAGGTGCGGATTTGTCGGCAACGATACCTTG
GGCAGTAGCAGAAGAAGAAGTCAGGATAAGGGCAGAACCGAGCAGTAATGAAAGGGAGAA
TGAGATAACAGAGATAGAATGGATAAAACCCGCAAAGCCCGCAATATCATTTGGCAAAAT
ACCTACAGCTTGGGTGTCGGCTGTGTTTTTGCCCTCGCGTTTGGCATTTTCAGCAACGGC
TATCATGCAGTTTCGATGTTTGTTAAATACAACTTTGTACAGGGTGCGGTTCATAGTAAG
GGCTTTCTTAATAATATTTTTATAATCGTAAATTAGATTAATTTTTAGGGGCTGACGTAG
ATTAACAGTTATGCCAGGCTACGAAAATAAAGATAACCAATTGTAAATTAAACAATAGAG
TTCAAAAGAAACTGCTTGAATTTTTCGTACTCCAAGCTACCGCCCGTTCCGCTGCCGATA
TTTTGGGTATGGCGCTGCGGGCAATTTCCGTTCCCACTTCGGCGAGTTGGCGCATAATGG
AACGCTCGCGCACGATTTCGGCATGGCGCCGGATGTTGGCGGCAGACGGAGTATTTTGCG
CCAGCGTAATCAGATATTCGAATCCCCCCGCCGCTTCCAGCTCTTCGTTCCGCTGCAAAT
CTTCCTGAACCGTGATGACATCGGCAGGACGGCTCTCATTGATCAGTTTGGCAATGGATC
GGAAAATCAGGCGGTGTTCGTGGCGGTAGAAATCCTCTCCCGAAACCACATCGGCAATCC
TGTCCCAAGCCGGATTTTCCAGCATCAACCCGCCCAAAACGGATTGTTCCGCCTCCATTG
AGTGCGGCGGAAGCGATAATGAGCCGATTCCTCCGTCTTCAGACGGCATGGCTGTGTAAT
CGTTCATGGTACATCCGACAAAATTGCAATCTTCTATTGTAGCGTAAAGCAGGTTCAATT
GGTTTCCGTACCGCAAAACAGGTAGAATACGCGAGTTGCCGGGTTAAATACCTTCCTCAA
CCATCACAGTTAACATAGGAAATAATTTGGCAATCTGAGAATCGGCTATCCACCTGTTTG
TCCCTTCAGTCCTAAGCATACCTGAATCTTTAACCCAAATTGTTCCATCCTTGTCCTTAA
AACGTGTGCCATTAGAAATCTTTTCCCATTCGTTTAAAACGACTTTTGCATTTTTGTTTT
CAGGATTTTTGGCCCCATTATCTTTAGCCACATCTTCAAATCCCCAACGTTCCTCTACGG
CTTTTTTCAGAATATTCAGCCTATGGGCTTTAGTCACGTTCTGACCTTTTGCAATGAGCG
AAGCGATATATGCTTCCGCCCTGACCCGTATCGTTCCGGCTTCCAAATCAGTCATTCCGG
CAAAAAGTTCCGATTGATTTTCAAGAGGGATGTCTTTCGACCCTATTTTATGTAGGATTG
AGAATGTAAAACCTACAATTTTTCGTCCTTCTTTATGCTGCTCGTAGGTAATGGAAATAT
CCGTTTTATCATTGATCTGCTTGACGGCGAAATCCAAAACCTTACGTTTGAATAGCTCCA
TTTTTTGATACTCGTCAGGCATCATACCCAAACGTTCGCGCAACTCCATTGTACTGAACA
TCGGTGTCTTACCGGCTGCACGCCATGAAATAATAATTTCGTAGAGCCGCACCGCGTATT
TACTGCTCAACGATGAGACCTGATCAAGCTCGTAGCTTGTGAAGTTTTTTTCTAGCATCG
TAATCAAAGGGGCAACATTTGGTGCAAAAACTAACTCTACCGTTGCCTGTTGTTCAATAT
AGGCGACTTGAGATACCCACCTTGTCCGTACTACCTTTTCCCCTTTTGGTGTTTTTTCGA
TAAAACTGAATTGGCGTTCAAAAAGGTTGTTACAGGCATCTTTCAAAGCCTTATACGCCG
TATTACGGTTGGTATGGAAATTATTAACGATGCAGAACTTATCCGTTCCATGCAGCGTCA
GCAGCACATAGATGCTGAATTGTTAACTGATGCAAATGTCCGTTTCGAGCAACCATTGGA
GAAGAACAATTATGTCCTGAGTGAAGATGAAACACCGTGTACTCGGGTAAATTACATTAG
TTTAGATGATAAGACGGTGCGCAAATTTTCTTTTCTTCCTTCTGTGCTCATGAAAGAAAC
AGCTTTTAAAACTGGATGTGTTTAGGTTCCAATAATTTGAGCAGGCTACAAAAAGCCGC
GCAACAGATACTGATCGTGCGTGGCTACCTCACTTCCCAAGCTATTATCCAACCACAGAA
TATGGATTCGGGAATTCTGAAATTACGGGTATCAGCAGGCGAAATAGGGGATATCCGCTA
TGAAGAAAAACGGGATGGGAAGTCTGCCGAGGGCAGTATTAGTGCATTCAATAACAAATT
TCCCTTATATAGGAACAAAATTCTCAATCTTCGCGATGTAGAGCAGGGCTTGGAAAACCT
GCGTCGTTTGCCGAGTGTTAAAACAGATATTCAGATTATACCGTCCGAAGAAGAAGGCAA
AAGCGATTTACAGATCAAATGGCAGCAGAATAAACCCATACGGTTCAGTATCGGTATAGA
TGATGCGGGCGGCAAAACGACCGGCAAATATCAAGGAAATGTCGCTTTATCGTTCGATAA
CCCTTTGGGCTTAAGCGATTTGTTTTATGTTTCATATGGACGCGGTTTGGCGCACAAAAC
GGACTTGACTGATGCCACCGGTACGGAAACTGAAAGCGGATCCAGAAGTTACAGCGTGCA
TTATTCGGTGCCCGTAAAAAAATGGCTGTTTTCTTTTAATCACAATGGACATCGTTACCA
CGAAGCAACCGAAGGCTATTCCGTCAATTACGATTACAACGGCAAACAATATCAGAGCAG
CCTGGCCGCCGAGCGCATGCTTTGGCGTAACAGACTTCATAAAACTTCAGTCGGAATGAA
ATTATGGACACGCCAAACCTATAAATACATCGACGATGCCGAAATCGAAGTACAACGCCG
CCGCTCTGCAGGCTGGGAAGCCGAATTGCGCCACCGTGCTTACCTCAACCGTTGGCAGCT
TGACGGCAAGTTGTCTTACAAACGCGGGACCGGCATGCGCCAAAGTATGCCTGCACCGGA
AGAAAACGGCGGCGATATTCTTCCAGGTACATCTCGTATGAAAATCATTACTGCCAGTTT
GGACGCAGCCGCCCCATTTATTTAGGCAAACAGCAGTTTTTCTACGCAACCGCCATTCA
AGCTCAATGGAACAAAACGCCGTTGGTTGCCCAAGATAAATTGTCAATCGGCAGCCGCTA
CACCGTTCGCGGATTTGATGGGGAGCAGAGTCTTTTCGGAGAGCGAGGTTTCTACTGGCA
GAATACTTTAACTTGGTATTTTCATCCGAACCATCAGTTCTATCTCGGTGCGGACTATGG
CCGCGTATCTGGCGAAAGTGCACAATATGTATCGGGCAAGCAGCTGATGGGTGCAGTGGT
CGGCTTCAGAGGAGGGCATAAAGTAGGCGGTATGTTTGCTTATGATCTGTTTGCCGGCAA
GCCGCTTCATAAACCCAAAGGCTTTCAGACGACCAACACCGTTTACGGCTTCAACTTGAA
TTACAGTTTCTAACCTCTGAATTTTTTACTGATATTTAGACGGTCTTTCCTTATCCTCAG
ACCGTCAAACTTTACCTACGTACTTGGCGCGCAGTACGTTCATCTTCAAAATGGAATAGA
CATGAATAAAGGTTTACATCGCATTATCTTTAGTAAAAAGCACAGCACCATGGTTGCAGT
AGCCGAAACTGCCAACAGCCAGGGCAAAGGTAAACAGGCAGGCAGTTCGGTTTCTGTTTC
ACTGAAAACTTCAGGCGACCTTTGCGGCAAACTCAAAACCACCCTTAAAACTTTGGTCTG
CTCTTTGGTTTCCCTGAGTATGGTATTGCCTGCCCATGCCCAAATTACCACCGACAAATC
AGCACCTAAAAACCAGCAGGTCGTTATCCTTAAAACCAACACTGGTGCCCCCTTGGTGAA
TATCCAAACTCCGAATGGACGCGGATTGAGCCACAACCGCTATACGCAGTTTGATGTTGA
CAACAAAGGGGCAGTGTTAAACAACGACCGTAACAATAATCCGTTTGTGGTCAAAGGCAG
TGCGCAATTGATTTTGAACGAGGTACGCGGTACGGCTAGCAAACTCAACGGCATCGTTAC

## Appendix A

CGTAGGCGGTCAAAAGGCCGACGTGATTATTGCCAACCCCAACGGCATTACCGTTAATGG
CGGCGGCTTTAAAAATGTCGGTCGGGGCATCTTAACTACCGGTGCGCCCCAAATCGGCAA
AGACGGTGCACTGACAGGATTTGATGTGCGTCAAGGCACATTGACCGTAGGAGCAGCAGG
TTGGAATGATAAAGGCGGAGCCGACTACACCGGGGTACTTGCTCGTGCAGTTGCTTTGCA
GGGGAAATTACAGGGTAAAAACCTGGCGGTTTCTACCGGTCCTCAGAAAGTAGATTACGC
CAGCGGCGAAATCAGTGCAGGTACGGCAGCGGGTCGCACTGGGCGGTATGTACGCCGACA
GCATCACACTGATTGCCAATGAAAAAGGCGTAGGCGTCAAAAATGCCGGCACACTCGAAG
CGGCCAAGCAATTGATTGTGACTTCGTCAGGCCGCATTGAAAACAGCGGCCGCATCGCCA
CCACTGCCGACGGCACCGAAGCTTCACCGACTTATCTCTCCATCGAAACCACCGAAAAAG
GAGCGGCAGGCACATTTATCTCCAATGGTGGTCGGATCGAGAGCAAAGGCTTATTGGTTA
TTGAGACGGGAGAAGATATCAGCTTGCGTAACGGAGCCGTGGTGCAGAATAACGGCAGTC
GCCCAGCTACCACGGTATTAAATGCTGGTCATAATTTGGTGATTGAGAGCAAAACTAATG
TGAACAATGCCAAAGGCCCGGCTACTCTGTCGGCCGACGGCCGTACCGTCATCAAGGAGG
CCAGTATTCAGACTGGCACTACCGTATACAGTTCCAGCAAAGGCAACGCCGAATTAGGCA
ATAACACACGCATTACCGGGGCAGATGTTACCGTATTATCCAACGGCACCATCAGCAGTT
CCGCCGTAATAGATGCCAAAGACACCGCACACATCGAAGCAGGCAAACCGCTTTCTTTGG
AAGCTTCAACAGTTACCTCCGATATCCGCTTAAACGGAGGCAGTATCAAGGGCGGCAAGC
AGCTTGCTTTACTGGCAGACGATAACATTACTGCCAAAACTACCAATCTGAATACTCCCG
GCAATCTGTATGTTCATACAGGTAAAGATCTGAATTTGAATGTTGATAAAGATTTGTCTG
CCGCCAGCATCCATTTGAAATCGGATAACGCTGCCCATATTACCGGCACCAGTAAAACCC
TCACTGCCTCAAAAGACATGGGTGTGGAGGCAGGCTCGCTGAATGTTACCAATACCAATC
TGCGTACCAACTCGGGTAATCTGCACATTCAGGCAGCCAAAGGCAATATTCAGCTTCGCA
ATACCAAGCTGAACGCAGCCAAGGCTCTCGAAACCACCGCATTGCAGGGCAATATCGTTT
CAGACGGCCTTCATGCTGTTTCTGCAGACGGTCATGTATCCTTATTGGCCAACGGTAATG
CCGACTTTACCGGTCACAATACCCTGACAGCCAAGGCCGATGTCAATGCAGGATCGGTTG
GTAAAGGCCGTCTGAAAGCAGACAATACCAATATCACTTCATCTTCAGGAGATATTACGT
TGGTTGCCGGCAACGGTATTCAGCTTGGTGACGGAAAACAACGCAATTCAATCAACGGAA
AACACATCAGCATCAAAAACAACGGTGGTAATGCCGACTTAAAAAACCTTAACGTCCATG
CCAAAAGCGGGGCATTGAACATTCATTCCGACCGGGCATTGAGCATAGAAAATACCAAGC
TGGAGTCTACCCATAATACGCATCTTAATGCACAACACGAGCGGGTAACGCTCAACCAAG
TAGATGCCTACGCACACCGTCATCTAAGCATTACCGGCAGCCAGATTTGGCAAAACGACA
AACTGCCTTCTGCCAACAAGCTGGTGGCTAACGGTGTATTGGCACTCAATGCGCGCTATT
CCCAAATTGCCGACAACACCACGCTGAGAGCGGGTGCAATCAACCTTACTGCCGGTACCG
CCCTAGTCAAGCGCGGCAACATCAATTGGAGTACCGTTTCGACCAAAACTTTGGAAGATA
ATGCCGAATTAAAACCATTGGCCGGACGGCTGAATATTGAAGCAGGTAGCGGCACATTAA
CCATCGAACCTGCCAACCGCATCAGTGCGCATACCGACCTGAGCATCAAAACAGGCGGAA
AATTGCTGTTGTCTGCAAAAGGAGGAAATGCAGGTGCGCCTAGTGCTCAAGTTTCCTCAT
TGGAAGCAAAAGGCAATATCCGTCTGGTTACAGGAGAAACAGATTTAAGAGGTTCTAAAA
TTACAGCCGGTAAAAACTTGGTTGTCGCCACCACCAAAGGCAAGTTGAATATCGAAGCCG
TAAACAACTCATTCAGCAATTATTTTCCTACACAAAAAGCGGCTGAACTCAACCAAAAAT
CCAAAGAATTGGAACAGCAGATTGCGCAGTTGAAAAAAAGCTCGCCTAAAAGCAAGCTGA
TTCCAACCCTGCAAGAAGAACGCGACCGTCTCGCTTTCTATATTCAAGCCATCAACAAGG
AAGTTAAAGGTAAAAAACCCAAAGGCAAAGAATACCTGCAAGCCAAGCTTTCTGCACAAA
ATATTGACTTGATTTCCGCACAAGGCATCGAAATCAGCGGTTCCGATATTACCGCTTCCA
AAAAACTGAACCTTCACGCCGCAGGCGTATTGCCAAAGGCAGCAGATTCAGAGGCGGCTG
CTATTCTGATTGACGGCATAACCGACCAATATGAAATTGGCAAGCCCACCTACAAGAGTC
ACTACGACAAAGCTGCTCTGAACAAGCCTTCACGTTTGACCGGACGTACAGGGGTAAGTA
TTCATGCAGCTGCGGCACTCGATGATGCACGTATTATTATCGGTGCATCCGAAATCAAAG
CTCCCTCAGGCAGCATAGACATCAAAGCCCATAGTGATATTGTACTGGAGGCTGGACAAA
ACGATGCCTATACCTTCTTAAAAACCAAAGGTAAAAGCGGCAAAATCATCAGAAAAAGCA
AGTTTACCAGCACCCGCGACCACCTGATTATGCCAGCCCCCGTCGAGCTGACCGCCAACG
GCATAACGCTTCAGGCAGGCGGCAACATCGAAGCTAATACCACCCGCTTCAATGCCCCTG
CAGGTAAAGTTACCCTGGTTGCGGGTGAAGAGCTGCAACTGCTGGCAGAAGAAGGCATCC
ACAAGCACGAGTTGGATGTCCAAAAAAGCCGCCGCTTTATCGGCATCAAGGTAGGCAAGA
GCAATTACAGTAAAAACGAACTGAACGAAACCAAATTGCCTGTCCGCGTCGTCGCCCAAA
CTGCAGCCACCCGTTCAGGCTGGGATACCGTGCTCGAAGGTACCGAATTCAAAACCACGC
TGGCCGGTGCGGACATTCAGGCAGGTGTAGGCGAAAAAGCCCGTGCCGATGCGAAAATTA
TCCTCAAAGGCATTGTGAACCGTATCCAGTCGGAAGAAAAATTAGAAACCAACTCAACCG
TATGGCAGAAACAGGCCGGACGCGGCAGCACTATCGAAACGCTGAAACTGCCCAGCTTCG
AAAGCCCTACTCCGCCCAAACTGACCGCCCCCGGTGGCTATATCGTCGACATTCCGAAAG
GCAATTTGAAAACCGAAATCGAAAAGCTGGCCAAACAGCCCGAGTATGCCTATCTGAAAC
AGCTCCAAGTAGCGAAAAACGTCAACTGGAACCAGGTGCAACTGGCTTACGATAAATGGG
ACTATAAGCAGGAAGGCTTAACCAGAGCCGGTGCAGCGATTGTTACCATAATCGTAACCG
CACTGACTTATGGATACGGCGCAACCGCAGCGGGCGGTGTAGCCGCTTCAGGAAGTAGTA
CAGCCGCAGCTGCCGGAACAGCCGCCACAACGACAGCAGCAGCTACTACCGTTTCTACAG
CGACTGCCATGCAAACCGCTGCTTTAGCCTCCTTGTATAGCCAAGCAGCTGTATCCATCA
TCAATAATAAAGGTGATGTCGGCAAAGCGTTGAAAGATCTCGGCACCAGTGATACGGTCA
AGCAGATTGTCACTTCTGCCCTGACGGCGGGTGCATTAAATCAGATGGGCGCAGATATTG
CCCAATTGAACAGCAAGGTAAGAACCGAACTGTTCAGCAGTACGGGCAATCAAACTATTG
CCAACCTTGGAGGCAGACTGGCTACCAATCTCAGTAATGCAGGTATCTCAGCTGGTATCA
ATACCGCCGTCAACGGCGGCAGCCTGAAAGACAACTTAGGCAATGCCGCATTAGGAGCAT
TGGTTAATAGCTTCCAAGGAGAAGCCGCCAGCAAAATCAAAACAACCTTCAGCGACGATT
ATGTTGCCAAACAGTTCGCCCACGCTTTGGCTGGGTGTGTTAGCGGATTGGTACAAGGAA
AATGTAAAGACGGGGCAATTGGCGCAGCAGTTGGGGAAATCGTAGCCGACTCCATGCTTG
GCGGCAGAAACCCTGCTACACTCAGCGATGCGGAAAAGCATAAGGTTATCAGTTACTCGA

## Appendix A

AGATTATTGCCGGCAGCGTGGCGGCACTCAACGGCGGCGATGTGAATACTGCGGCGAATG
CGGCTGAGGTGGCGGTAGTGAATAATGCTTTGAATTTTGACAGTACCCCTACCAATGCGA
AAAAGCATCAACCGCAGAAGCCCGACAAAACCGCACTGGAAAAAATTATCCAAGGTATTA
TGCCTGCACATGCAGCAGGTGCGATGACTAATCCGCAGGATAAGGATGCTGCCATTTGGA
TAAGCAATATCCGTAATGGCATCACAGGCCCGATTGTGATTACCAGCTATGGGGTTTATG
CTGCAGGTTGGACAGCTCCGCTGATCGGTACAGCGGGTAAATTAGCTATCAGCACCTGCA
TGGCTAATCCTTCTGGTTGTACTGTCATGGTCACTCAGGCTGCCGAAGCGGGCGCGGGAA
TCGCCACGGGTGCGGTAACGGTAGGCAACGCTTGGGAAGCGCCTGTGGGGGCGTTGTCGA
AAGCGAAGGCGGCCAAGCAGGCTATACCAACCCAGACAGTTAAAGAACTTGATGGCTTAC
TACAAGAATCAAAAAATATAGGTGCTGTAAATACACGAATTAATATAGCGAATAGTACTA
CTCGATATACACCAATGAGACAAACGGGACAACCGGTATCTGCTGGCTTTGAGCATGTTC
TTGAGGGGCACTTCCATAGGCCTATTGCGAATAACCGTTCAGTTTTTACCATCTCCCCAA
ATGAATTGAAGGTTATACTTCAAAGTAATAAAGTAGTTTCTTCTCCCGTATCGATGACTC
CTGATGGCCAATATATGCGGACTGTCGATGTAGGAAAAGTTATTGGTACTACTTCTATTA
AAGAAGGTGGACAACCCACAACTACAATTAAAGTATTTACAGATAAGTCAGGAAATTTGA
TTACTACATACCCAGTAAAAGGAAACTAACTAAATATGAGTAACTTTGAAAAAAAATATA
TTTTAGAATTAAATGATGCTTTAAGCCATTTAAATCATAACTCTACCTCATTTGATTTAT
TGAAAGTTTTGATTTCATGGTTATCAAACGATATTGTCATTGATAAATTTAAAATTTTAG
GTTATGACTTTAGTAAATATATCGAAATGAATCCCGATGACTATCCGGTTGAAAAATCTA
TATTGAATAGAGAGGAAATTATTTATCTCAAAAACAATATTTATCGTAAAATATCATCAG
GAAATTTTAAATTTCAATACTTTGTACAATATATTAGAGATATTTTAGAATATTTATTTA
TTGAACATATTGAAAGAGTCTGTCCTTACTGCGAATGGGGTGAAATGCAAAAATTAGAAG
AACAAAATACGCATGAAACGGTGTATCTCTGTACTCAATGTGGATGTGCTTTTTATAACG
ATAATTCACAATTTTTATTAAAAACCCCTTTAACCATTCCAATGAAACGTGATGAATTTA
AATAAACAAGCCGTAGCCTGCATGAACCCTAAAATCCACGTGTAGCGTGTGTGCGCCAGC
ACGCATGCGTTCCATGATTTACGGCTCAATGCCGTCTGAAAAGCTCACAATTTTTCAGAC
GGCATTTGTTATGCAAGTAAATATTCAGATTCCCTGTATGCTGTACAGACGCGGGAGTGT
TAAGCCCCCCTTGTTTGAAGCTCCGCGGCTCCTGCCGAGCTTCACCGACCCCGTTGTGCC
CAAGCTCTCTGCTCCCGGCGGCTACATTGTCGACATCCCCAAAGGCAATCTGAAAACCGA
AATCGAAAAGCTGGCCAAACAGCCCGAGTATGCCTATCTGAAACAGCTCCAAGTAGCGAA
AAACGTCAACTGGAACCAGGTGCAACTGGCTTACGATAAATGGGACTATAAGCAGGAAGG
CTTAACCAGAGCCGGTGCAGCGATTATCGCGCTGGCTGTTACCGTGGTTACTGCGGGCGC
GGGAGTCGGAGCCGCACTAGGCTTAAACGGCGCAGCCGCAGCAGCGGCCGATGCCGCCTT
TGCCTCACTCGCTTCTCAGGCTTCCGTATCGCTCATCAACAATAAAGGCGATGTCGGCAA
AACCCTGAAGGAACTGGGCAGAAGCCGCACGGTAAAAAATCTGGTTGTAGCGGCGGCAAC
GGCAGGCGTATCCAACAAACTCGGTGCCTCTTCCCTTGCCACTTGGAGCGAAACCCCTTG
GGTAAACAACCTCAACGTTAACCTGGCCAATGCGGGCAGTGCCGCGCTGATCAACACCGC
TGTTAACGGCGGCAGCCTGAAAGACAATCTGGAGGCAAATATCCTGGCGGCATTGGTGAA
TACCGCGCATGGGGAGGCGGCGAGTAAGATCAAAGGACTGGATCAGCACTATGTCGCCCA
CAAAATCGCTCATGCCGTAGCGGGCTGTGCGGCTGCAGCGGCGAATAAGGGCAAATGTCA
GGACGGCGCGATCGGTGCGGCTGTGGGTGAGATTGTCGGGGAGGCTTTGGTTAAAAATAC
CGATTTTAGCGATATGACCCCGGAACAATTAGATCTGGAAGTTAAGAAAATTACCGCCTA
TGCCAAACTTGCGGCAGGTACAGTTGCAGGCGTAACGGGAGGAGATGTCAATACTGCTGC
ACAAACCGCACAAAACGCGGTAGAAAATAATGCGGTTAAAGCTGTTGTAACTGCTGCAAA
AGTGGTTTATAAGGTAGCCAGAAAAGGATTAAAAAACGGGAAAATCAACGTTAGAGATTT
AAAACAGACGTTGAAAGACGAAGGTTATAATTTAGCCGACAACCTGACCACCTTATTCGA
CGAAACATTGGATTGGAACGATGCCAAAGCCGTTATTGATATTGTCGTCGGAACAGAGCT
GAATCGCGCTAATAAAGGGGAAGCGGCACAAAAGGTCAAGGAAGTTTTAGAAAAAAATCG
TCCTTATATCCCTAATAAAGGTGCTGTACCGAATATGAGTACATACATGAAAAATAATCC
TTTTGGAAAACAGCTGGCTCAAATTTCAGAAAAGACAACGCTTCCGACGCAGCAAGGGCA
GTCTGTCTTCTTGGTAAAAAGAAACCAAGGGTTATTAAAAACCGGTGATAGGTTTTATTT
AGATGGCCAACATAAAAATCATTTAGAGGTTTTTGATAAAAATGGGAACTTTAAGTTTGT
TCTAAATATGGATGGTTCGCTTAACCAAATGAAAACTGGGGCAGCAAAAGGTCGTAAATT
AAACTTAAAATAGGAAATTTTATGGAAACATTGAATGATATAAAAAAAATCTTGATTAAT
GTGGGGCTTTATCAAGGGTTTGATTTGACAGATCCAAAAGTATCAGAAGAAGTTAATCAT
GAAACAGCTAATATGAAATGGATTAAAGATTATACTTCAGACGGGAATTGGGATAATGAA
TTTAAGGAGGATTTAAAAAACTTTTTAGATTATATGGAAGTATGCCAATTAGCCCTAAAC
GATAAAAATTTCAAAATTGCCAGTAATTCTTTATTTATGGCTATGATTTACGCAGGTAAT
CTATCTCTTATATTTGATTCAATAAAAACTGATATATCAACATTATTGAGTGCTGAGTAT
AAAAAGAATAGTTTTTCATGGCCATCTCTTGATGAATAGAAAGCAAGTTGTAGCCTGCAT
GAAATCTAAAACCCATGCATAAGGTGTGGGCTTCAGTATACGCGTTCCATGATTTACGGC
CATATGCCGTCTGAAAAGCTCAATTTTTTCAGACGGCATTTGTTATGAAAGTAAATATTT
AGATTCCCTGTATACTGTTTAGACTCGTGTGTGCTGAGTAAGCTGTAGTCTGCATGAAAC
CTAAAACTCGCTCAAAATTAAGCTAAGACATTAGCAGGGCAAGGGCGAAAATTGAATCTT
AAATAAGGTGATTCAGATGAAAAATTTTAATGTAGTAAAAGAAAGTTTAAGAGAGTTAGG
AATTAAACAAGGATTTGATCTTTATGAGAAAGCCACAACTGAAAAATTGAATAGTGAAGA
TCCTCTTGACTTACAATGGCTTTCTAACTATTCATCTGATTGGAATGATGAATTAGAAGA
AGACTTTGATTCTTTTTTTCAGCATATGAAGGAATATCAATATGCTATTGACAATGAAGA
CATTAAATCTGCATGTAGTTCACTATGTGAAGCTATGCTCTATGTTGGTAATATTAAAAA
TTTTTTTGAGTTTCTCAAAAGCGATATGATTAGACTGTTGAGAGGTGAAAGTAAAACAAC
AGACTTTCAATGGCCGCAATTTGATGAATAGCAGCAAGCTGTAGCCTGCATGAAACCTAA
AATCCATGCGTAAGGTGTGTGCTTCAGCACGCACGCGTTCCATGATTTACGGCTCAATGC
CGTCTGAAAAGCTCACAATTTTTCAGACGGCATTTGTTATGCAAGTAAATATTCAGATTC
CCTATATACTGCCCAGATGCGTGCGTGCTGAAGACACCCCCTACGCTTGCTATTTGAAAC
AGCTCCAAGTCACCAAAGACGTCAACTGGAACCAGGTACAACTGGCGTACGACAAATGGG

## Appendix A

ACTATAAACAGGAAGGCTTAACCGGAGCCGGAGCAGCGATTATTGCGCTGGCTGTTACCG
TGGTTACTGCGGGCGCGGGAGCCGGAGCCGCACTGGGCTTAAACGGCGCGGCCGCAGCGG
CAACCGATGCCGCATTCGCCTCGCTGGCCAGCCAGGCTTCCGTATCGCTCATCAACAACA
AAGGCAATATCGGTAACACCCTGAAAGAGCTGGGCAGAAGCAGCACGGTGAAAAATCTGA
TGGTTGCCGTCGCTACCGCAGGCGTAGCCGACAAAATCGGTGCTTCGGCACTGAACAATG
TCAGCGATAAGCAGTGGATCAACAACCTGACCGTCAACCTGGCCAATGCGGGCAGTGCCG
CACTGATTAATACCGCTGTCAACGGCGGCAGCCTGAAAGACAATCTGGAAGCGAATATCC
TTGCGGCTTTGGTGAATACTGCGCATGGAGAAGCAGCCAGTAAAATCAAACAGTTGGATC
AGCACTACATTACCCACAAGATTGCCCATGCCATAGCGGGCTGTGCGGCTGCGGCGGCGA
ATAAGGGCAAGTGTCAGGATGGTGCGATAGGTGCGGCTGTGGGCGAGATAGTCGGGGAGG
CTTTGACAAACGGCAAAAATCCTGACACTTTGACAGCTAAAGAACGCGAACAGATTTTGG
CATACAGCAAACTGGTTGCCGGTACGGTAAGCGGTGTGGTCGGCGGCGATGTAAATGCGG
CGGCGAATGCGGCTGAGGTAGCGGTGAAAAATAATCAGCTTAGCGACAAAGAGGGTAGAG
AATTTGATAACGAAATGACTGCATGCGCCAAACAGAATAATCCTCAACTGTGCAGAAAAA
ATACTGTAAAAAAGTATCAAAATGTTGCTGATAAAAGACTTGCTGCTTCGATTGCAATAT
GTACGGATATATCCCGTAGTACTGAATGTAGAACAATCAGAAAACAACATTTGATCGATA
GTAGAAGCCTTCATTCATCTTGGGAAGCAGGTCTAATTGGTAAAGATGATGAATGGTATA
AATTATTCAGCAAATCTTACACCCAAGCAGATTTGGCTTTACAGTCTTATCATTTGAATA
CTGCTGCTAAATCTTGGCTTCAATCGGGCAATACAAAGCCTTTATCCGAATGGATGTCCG
ACCAAGGTTATACACTTATTTCAGGAGTTAATCCTAGATTCATTCCAATACCAAGAGGGT
TTGTAAAACAAAATACACCTATTACTAATGTCAAATACCCGGAAGGCATCAGTTTCGATA
CAAACCTAAAAAGACATCTGGCAAATGCTGATGGTTTTAGTCAAAAACAGGGCATTAAAG
GAGCCCATAACCGCACCAATTTTATGGCAGAACTAAATTCACGAGGAGGACGCGTAAAAT
CTGAAACCCAAACTGATATTGAAGGCATTACCCGAATTAAATATGAGATTCCTACACTAG
ACAGGACAGGTAAACCTGATGGTGGATTTAAGGAAATTTCAAGTATAAAAACTGTTTATA
ATCCTAAAAAATTTTCTGATGATAAAATACTTCAAATGGCTCAAAATGCTGCTTCACAAG
GATATTCAAAAGCCTCTAAAATTGCTCAAAATGAAAGAACTAAATCAATATCGGAAAGAA
AAAATGTCATTCAATTCTCAGAAACCTTTGACGGAATCAAATTTAGATCATATTTTGATG
TAAATACAGGAAGAATTACAAACATTCACCCAGAATAATTTAAAGGAAAAATTATGAAAA
ATAATATTTTTCTAAACTTAAATAAAAAATCTATAAATAACAACCATTTTGTTATTTCGA
TTTTTTTTGAAACAATTTACCAATTTGAAACTAAAGATACGCTTTTAGAGTGTTTTAAAA
ATATTACAACTACCGGACATTTTGGAGTAATAGGTGCTCAATATGAAAAAATAGATGCTA
CCAGATGGATTGGAGATTATGAAGAGGTAAATGGATTTGAGTATATTGATAAAGCTCCTT
CTATTTATTTTTCAGTTGGAGATGATTTCAATCCTGAAGAATTAATTATACCTATTAATT
TAGCATATCATTACTTTAATATTGCAATATCTGATTTCTTAATAGCTCACCCTGAATATC
AAAAAAAGTGTAAAGAAATACAAAAAACATATTCTCAAACAAACTGTAGCCTGCATGAAA
CCTAAAATCCATGCGTAAGGTGTGTGCTTCAGCACGCACGCGTTCCATGATTTACGGCTC
AATGCCGTCTGAAAAGCTCACAATTTTTCAGACGGCATTTGTTATGCAAGTAAATATTCA
GATTCCCTATATACTGCCCAGACGCGTGCGTGCTGAAGACACCCCCTACGCTTGCTGCAG
AACTTTCGGGTAAAACCGGTGTGAGCATTAGCGCACCGTATGCCAATGAGAACAGTCGCA
TCCTGCTCAGCACCACGGATATCAGTTCGGAAAACGGCAAAATCAAAATTCAATCTTACG
GTGACCAATATTACTATGCGAGACAGAGCGAACTCTATACCTTTGAACGCCGCAGCTACA
AAACTGGCAAATGGTACAACCGCAAACACATTACCGAAGTCAAAGAACACAAAAACGCCA
AGCCCGACGCAGTAACCCTCAGCGCATCCCAAGGCATCGACATCAAATCTGGTGGCAGCA
TCGACGCCTACGCCACCGCATTCGATGCCCCCAAAGGCAGCATTAACATCGAAGCCGGGC
GGAAATTGACACTCTATGCCGTAGAAGAGCTCAACTACGACAAACTTGACAGCCAAAAAA
GGCGCAGATTTCTCGGCATCAGCTACAGCAAAGCACACGACACCACCACCCAAGTCATGA
AAACCGCGCTGCCCTCAAGGGTAGTTGCAGAATCTGCCAATCTGCAATCAGGTTGGGATA
CCAAACTGCAAGGCACACAGTTTGAAACCACACTGGGTGGCGCAACCATACGCGCAGGCG
TAGGCGAGCAGGCACGGGCCGATGCCAAGATTATCCTCGAAGGGATCAAAAGCAGCATCC
ACACAGAAACCGTGAGCAGCAGCAAATCTACTCTATGGCAAAAACAGGCAGGACGGGGCA
GTAACATCGAAACCTTGCAATTGCCGAGTTTCACCGGTCCCGTTGCGCCCGTACTGTCCG
CACCCGGCGGTTACATTGTCGATATTCCGAAAGGCAATCTGAAAACCCAAATCGAAACCC
TCACCAAGCAGCCCGAGTATGCTTATTTGAAACAACTTCAAGTTGCGAAAAACATCAACT
GGAATCAGGTGCAGCTTGCTTACGATAAATGGGACTACAAACAGGAGGGCATGACACCCG
CAGCAGCAGCTGTCGTCGTTATCGTCGTAACCGTATTGACCTACGGCGCACTGTCCGCCC
CGGCAGCCGCCGGAACGGCGGGCGCGGCAGGCGCAGGAGCGGGAGGAGCCGCAGCAGGAA
CGGCAGCCGGAACTGGAGTAGCAGCAGGAACGGCAGCCACAACCGGAGTAGCAGCAGGCA
CATCAGCTGCAGCTATCACCACAGCCGCAGGCAAAGCCGCACTGGCCAGTCTCGCCAGCC
AAGCCGCAGTTTCCCTCATCAACAACAAAGGAGACATAAACCATACCCTGAAAGAACTGG
GCAAAAGCAGCACCGTCAGACAGGCCGCCACCGCCGCCGTAACCGCAGGCGTACTGCAGG
GCATAAGCGGGCTGAACACCCAAGCAGCCGAAGCCGTCAGCAAACATTTTCACAGTCCCG
CAGCAGGCAAACTGACCGCTAACCTGATCAACAGCACCGCTGCCGCAAGTGTCCATACCG
CCATCAACGGCGGCAGCCTGAAAGACAACTTGGGCGATGCCGCACTGGGTGCGATAGTCA
GTACCGTACACGGAGAAGTAGCGAGCAAAATCAAATTTAATCTCAGCGAAGACTACATTG
CCCACAAGATAGCCCATGCCGTAGCAGGCTGTGCATCGGCGGTAGCAAATAAAGGCAAAT
GTCGGGACGGCGCAATCGGCGCGGCAGTCGGCGAGATGGTGGGAGAAACCCTGTTGGACG
GACGCGATGTAGGCAAACTGTCACCCCAAGAACGCCAAAAAGTCATAGCCTACTCGCAGA
TTATCGCAGGCAGCGCAGTGGCATTGGTTAAAGGGGATGTGAATACGGCGGCGAATGCGG
CTACTGTGGCAGTGGAGAATAATAGTCTTTTAGCTCGCAGGAGGGTAAATATACGTTGGA
CTTCGCGACAAGAATTGGAACATGAATATGCCATTCTTGAAATCCAGGCCATTACCAATC
AAATCCGAAGGCTGGATCCGAAATTTAACGGGATTGCTATTATGAGGAATCCTAGAGAGC
CGTGGACAAGACATGATGTACAAACATACAGGCAATATTATAATCAATTAAGGGAATCCA
GAGGCTTTGCTGTTGACCCAATTTATAGAATCAGGATAAACAACGGCAATGAATTTAACC
GTATCATGTCATCAAAATACCCTTATAATGAGCTTTATGTAGCCAATCCTAAATCGGCGA

## Appendix A

CGGGGTATTTTAGGGTAGATTCGTATAATCCTGCGACAGAGGAAATTATTTCAAGAAAAT
TTACCCAATTTTCTCAAATCCAAGAAAGTACGGGGATTGGTTATATCAAGGAGGCTGTTA
GAAAATATAGCCCTGGTGCTGTCATTTCCAATGTTCCAAGTACACCTACTACGATAAGAG
GAAGAAAGCTTGAAGGAAAACTTATTTTAGAAGTTCCTGCTCAGGTCAATCCAATTCCAC
AATCTGTATTAAGGGCGGCACAAGAAGAAAATGTTATCATTAGAGATACAACAGGAAGGA
TTTACAAATGAAGAAAGATATTTTTTATTGTGAGCAGTGGTCTTATGGTTATAAGAAACT
TCATAAGCCTTTTTCTGAGAAACAAGCTGAGGAAAAACATCTTAAAGGGGAGTTATATAC
TGCCGTAATAGGTTCGGCGACACAACCTGAATATGTAATTACCTTGCGAGAGGAAGTAGG
TTTTTTTTCGGTACATTTTTTCGATAAATTTGGAAGGATTATTTAACCCATCAATTTCA
AAAATATTCCAATTCGAATTATTATTTTCTTTCTATGGCTGTATGGAGAGATTATATAAC
TTTGGAATCTCATGACTTAGCAGAAGGATATACTTATTTCTTCAATGAAAATACGGATGA
TTGCTATGTTTTGAAAGAGGATTTTATTAATAATGAGCGATATGAAAAAACAGAATTATA
TTCCCAAAAAGATAAGGTAATTCTATTTCCAAAGTTTGGCGAATATGATTTGGTGTTAAA
TCCGGACATTATTTAATTGAGTTTTAAGGCCGTCTGAAAAAATTTCAGACGGCTTTTATT
ATTGGGTTTGGAATCTGAGGATAAAGCTGATAAAAACCAGGAAATTATCAGGTTGCTATA
TACGTATTGTTGTACAGACTAAAGGCAGCAATCAAATCACTACTGCTTACCCACAAAAAT
AAATCGATTATATGGAGTAATCATGAATAAGAGAATGAAAATGTGTCCTGCTTGTCAACA
AGGCTATCTCTACCATTCGAAACCTAAATATCTTCATGATGAAATTATTCTGTGTGATGA
ATGCGATGCAGTATGGCTCAAAGGTATGAATATATTTTATGGAGAATATGAAAAAGATTT
TTATTCTTATGTTCCTTTCATGGAATCCCAAGGTATAACGAGTGAATGTATTTGGGAAGG
AGATTTGTTTGATCATCCATATTATGAAGATGAAAACTCAAATGATATGGATTGATGGAA
ATTTTAAGCCTGCGTAGGTACGATTAGCCATCAAACGGCGTAATCATACGCAAGATTATC
AACAGAGAGGGCTGGCAGCGATATACCACCCACAAGATTGCCCATGCCATAGCGGGCTGT
GCGGCAGCGGCGGCGAATAAGGGCAAGTGTCAGGACGGCGCGATTGGTGCGGTCGTGGGG
GAGATTGTCGGGGAGGCTTTGGTTAAGAATACCGATTTCAGCGGTATGACTGCTTCTGAA
ATTGAAAAAGCTAAAGCGAATATTACTGCGTATGCAAAATTGGTAGCCGGAGCGACTGTA
GGTGTTACAGGAGGCAATGTTGATGTGGCGGCAAATGCTTCCGAAACAGCTGTTAAAAAT
AATGCATTAGATATTATTTGGGATATTGGCAACCTCGTATGGGACGGCGGTAAATGGATT
TACGCCAAATCTATTGGCGATAAGCAGATGGCTCGAGAAGCGGCGATTGATTTTGGTGTG
GATGCCGCCGCAGCTGCCGTTCCCTTTGTTCCGGCAGGTGCGACTAAAATCAGCCGAGGC
GGGGCTTATGTTCTGAAGGCGGGAGACGAAGCAGTTGATACGGCTAAAGCCATACAGGAA
ATTCAGAAGCAGACCGGAATCAAGCTTACTTATGATAAGGTTAATAAGGTTTGGACAACA
CCGGCGGGGTTAGATTATGGGTTAGATGCTAAGCATGGTAATAGGATTAAACATGTTTTA
GCCCATACAATTCCAAATCCAAACAAACCTGTTCATTCTGTTTTTAATGTGTCCCGTAAA
GAAGTTTTGCCTTTGGTTGATGAAGCTTGGAGAATGAAAGGAAATCCTTTGCCAAATGAT
TCATCCGTATATCTTGTAGATATGAAGAAACCTATTGGAACAAAAGGAGAAACAAAAGTG
CGGATTGTTGTGCAAAAAGGAACAAATAAAATCATTTCTGCATATCCTCAGAAATAATTA
AGAAAGGAATCTCTTATGGATAAAGAAATTAAAATTTGCCCAAGATGTGAGCAAGGCTAC
CTTTATCATGCAAAGCCTAAATATTTCTCTGGGGAGGTCATTTTATGCGATGAATGTTAT
GCTATGTGGCTTGGGGATATGAAAATTTTTTACGGACAATATGGAAAAGATTTTTATGAT
TATCATGAGTTTATGAAAGATAAAGGCATAGAAGAAATAAATATGTGGGAAGGAGAGCTT
TTTGATCACCCATATTATGAGGATGAAAAATTTAAATAATTGATTTTCTGTTCCCCGAAT
TTGGGAAATACGATGATATTTTAAACCCAAATATTATTTAAAGTAGCAATAGGCCGTCTG
AATATCCGTTTTTCAGACGGCCTCAATGCAACTGCTGGCAGCCGAAGGCATTCACCAACA
CCAATTGAATGTTCAGAAAAGTACCCGTTTCATCGGCATCAAAGTGGGTAAAAGCAATTA
CAGCAAAAACGAGCTGAACGAAACCAAACTGCCCGTACGCGTTATCGCCCAAACAGCCAA
AACCCGTTCCGGCTGGGATACCGTACTCGAAGGCACCGAATTCAAAACCACCCTTTCCGG
AGCCGACATACAGGCAGGGGTGGGTGAAAAAGCCCGAGCCGATGCGAAAATTATCCTAAA
AGGCATCGTTAACGGCATCCAAACCGAAGAAAAGCTGGAATCCAACTCGACCGTATGGCA
AAAGCAGGCCGGAAGCGGCAGCACGGTTGAAACGCTGAAGCTACCGAGCTTTGAAGGGCC
GGCACTGCCTAAGCTGACCGCTCCCGGCGGCTATATCGCCGACATCCCCAAAGGCAACCT
CAAAACCGAAATCGAAAAGCTGGCCAAACAGCCCGAATATGCCTATCTGAAACAGCTTCA
GACGGTCAAGGACGTGAACTGGAACCAAGTACAGCTCGCTTACGACAAATGGGACTATAA
ACAGGAAGGCCTAACCGGAGCCGGAGCCGCAATTATCGCACTGGCCGTTACCGTGGTCAC
CTCAGGCGCAGGAACCGGAGCCGTATTGGGATTAAACGGTGCGGCCGCCGCCGCAACCGA
TGCAGCATTTGCCTCTTTGGCCAGCCAGGCTTCCGTATCGTTCATCAACAACAAAGGCAA
TATCGGTAACACCCTGAAAGAGCTGGGCAGAAGCAGCACGGTGAAAAATCTGATGGTTGC
CGTCGCTACCGCAGGCGTAGCCGACAAAATCGGTGCTTCGGCACTGAACAATGTCAGCGA
TAAGCAGTGGATCAACAACCTGACCGTCAACCTGGCCAATGCGGGCAGTGCCGCACTGAT
TAATACCGCTGTCAACGGCGGCAGCCTGAAAGACAATCTGGAAGCGAATATCCTTGCGGC
TTTGGTGAATACTGCGCATGGAGAGGCAGCAAGTAAAATCAAACAGTTGGATCAGCACTA
CATTGCCCATAAGATTGCCCATGCCATAGCGGGCTGTGCGGCAGCGGCGGCGAATAAGGG
CAAGTGTCAAGATGGTGCGATCGGTGCGGCGGTCGGTGAAATCCTTGGCGAAACCCTACT
GGACGGCAGAGACCCTGGCAGCCTGAATGTGAAGGACAGGGCAAAAATCATTGCTAAGGC
GAAGCTGGCAGCAGGGGCGGTTGCGGCGTTGAGTAAGGGGGATGTGAGTACGGCGGCGAA
TGCGGCTGCTGTGGCGGTAGAGAATAATTCTTTAAATGATATACAGGATCGTTTGTTGAG
TGGAAATTATGCTTTATGTATGAGTGCAGGAGGAGCAGAAAGCTTTTGTGAGTCTTATCG
ACCACTGGGCTTGCCACACTTTGTAAGTGTTTCAGGAGAAATGAAATTACCTAATAAATT
CGGGAATCGTATGGTTAATGGAAAATTAATTATTAACACTAGAAATGGCAATGTATATTT
CTCTGTAGGTAAAATATGGAGTACTGTAAAATCAACAAAATCAAATATAAGTGGGGTATC
TGTCGGTTGGGTTTTAAATGTTTCCCCTAATGATTATTTAAAAGAAGCATCTATGAATGA
TTTCAGAAATAGTAATCAAAATAAAGCCTATGCAGAAATGATTTCCCAGACTTTGGTAGG
TGAGAGTGTTGGTGGTAGTCTTTGTCTGACAAGAGCCTGCTTTTCGGTAAGTTCAACAAT
ATCTAAATCTAAATCTCCTTTTAAAGATTCAAAAATTATTGGGGAAATCGGTTTGGGAAG
TGGTGTTGCTGCAGGAGTAGAAAAAACAATATACATAGGTAACATAAAAGATATTGATAA

## Appendix A

ATTTATTAGTGCAAACATAAAAAAATAGGAGTTAGTATGAAATATATGATTAGTTTTCTA
AAAAAAACATTTGAATTAATGAGTTGGGTGTTAGTCATACTAATAATTGGGACATTTTAT
GACTATTATCAAATAAGGCAATATGCTGAATTAGAAAAGAAATCTATATCAAATATCTTG
CTATATGCCCAAAAAGAAAAATTTCGCTTAGAGAGTAAAGATAAATACATGCGAGGAGGA
TATACAAAATATAAATTTATTTTTTCAGAATATAGTAATACTACTTTTTTAAATTTCATA
AATGACCTGAAAAAAGATAATTATTTACCACTTGACGGCTATGGACATGGTTTTCTATGT
AGAAAAGGAGAGTCTATATCAATCAATATATACCCTGAAATTAATAAATTTATTTTAGTA
TGGGGATACCCTGAAAATCTTTGCGCTGATTCTAATTAAGTAAGCAAGAATAGGTTATAG
GGAAATAAAATTCAAATGAGAAAATTGAATAATCACGATGTTCATAAACGGTATCAAGAT
CGTTTAGAAGAGGATGTAGAGTTCACTATCAACTATGAGCTTCCTTTGAGTTGTTTGTGG
TCAACCATCAAAGACTTTTCCAGCGATTTTGAGGAAAAAACTGAAGCGTTCTTTATTCTT
TTCAAAGAGCTGCTGCGCAGAGGTCATCTGAAACTGCAACGCGACGGGCAAATTATCGGG
CATACGCCCGAAGAATGGGAACAAATATTTAGGGAAGTATGGCCTGAATATGAAATCGAA
CCCAATCCACTTCCCGGCTATGCCCCATTTGATATTGGAATGTGGCTTACGGTCGAGGCT
CCTGCCTACGCCGTATGGATAGATCCCGAAGACGGTAGCGAATACTGGGCGGGATAAAAT
ACCAATGTTTGGAATAAATCCCGTCTGAAAAACAGCTTTTTCAGACAGGATTTATTCCAA
TTATCGGTGATATACAGAGTTTTGTACAAGCACAGACCGCTGCCGATCACCTGTTTGCTT
TGCTGGGTGTGGTTCCGGGTATCGGTGAATCGATACAGGCCTATAAAGTAGCGAAAGCGG
CAAAAAATTTACAAGGCATGAAAAAAGCCTTGGACAAGGCAGCAACCGTTGCCACTGCAC
AGGGCTATGTCAGTAAAACCAAAATCAAAATCGGTCAAACTGAATTAAGGGTTACTGCAG
CAACTGACAAACAATTGCTGAAAGCTATTGGCGAAGGAAGGACACGACAGGTAAAATGAC
CGAGCAGTTATTTGACTCTTTAGCTAAACAAAATGGCTTCAGAGTGCTTTCGGGCGGCAA
ATACGGCGGAAATAACGGTTTTGATCATGTATGGCAGGCTGCCGATGGTAGTGTTGTTTT
GATTGTAGAAAGTAAGCAGATTAGGAACGGTACGGTACAGCTGAATCCGAATGGTGCGGG
TGGATATACGCAGATGAGTCGTGAATGGATTAAACAAGTTGTAAAAAGTTTACCTGATGG
TAGTCCTGCTAAGGCAGTTGTCTTAAAAGCAAATCAGAACGGCAAATTAAAAACGGCAAT
AGCAGGCGTTGATCGTCAAACAGGTAAGGCCGTTATTCTTTCTGTCAAAGTTCCTTCTAA
AACCAATATAAGGAGATAACAATGGGGCACAATATGATGACCACCCAAAAATGGTATGAA
CATATTACTAATGTAATCATAGGCAATACTGCTAATTTCAATAGCGGTTGCCCCGAATCT
ATAGATTATGTAGATGAAAAAAAAGGCGTGCCGCTTGCAGCGATGAAATACATTTTAATG
TACACTGAAGCTGCGGCTTCCCATGCCTATCTATTTGAACATGATCTTAAGAAATTCAAG
CAATATGCTTATGTTGCAGGAAAGTTGGGTATTTTGCAGAGTGTAGATGATGAAGACCCC
GAACCCTTCTTCTTTCCCTGCGACATGCTCAACATTCAAGATCCGATGTTTCTGATGCTG
ATGAGCGACAGCCCGCAGCTGCGCGAGTTTTTGGTGCGCAATATCGACAACATCGCCAAC
GATACAGAAGCCTTCGTAAACCGATACGACCTCAACCGTCATATGATTTACAATACTCTG
CTGATGGTGGAGGGTAAGCAGCTTGATCGGTTGAAACAACGTAGCGAGAAAGTCTTGGCG
CATCCCACCCCTAGCAAATGGCTGCAAAAGCGGTTGTACGATTACCGCTTCTTCCTCGCT
TTCGCCGAACAGGATGCCGAGGCGATGAAGGCCGCCTTAGAGCCGCTTTTTGATAAAAAA
ACCGCGCGTATGGCTGCCAAAGAAACATTGTCCTATTTCGATTTCTACCTGCAGCCGCAA
ATCGTTACCTACGCCAAAATCGCATCCATGCACGGTTTCGATTTGGGCATAGACCACGAA
ATCGCGCCGAGGGATTTGACTGTTTACGATCCGCTGCCGGCAGACGAATATCAAGACATC
TTCGATTTTATGAAACAGTATGACTTGTCTTATCCGTATGAATATCTGCAGGATTGGATA
GATTACTATACGTTCAAAACCGATAAGCTGGTATTTGGTAACGCGAAGCGAGAGTGAGCC
GTAAAACTCTGAGCTCCTGTTTTATAGATTACAACTTTAGGCCGTCTTAAAGCTGAAAGA
TTTTCGAAAGCTATAAATTGAAGCCCTTCCATAGTACATAGATCTGTGTTGTGGCGAGGC
TTTACCACGCTGATTGCCGGAGAAGAACTCAACCTGCTGGCAAAACAAGGCATGAGATCT
TTGCAATAACATGAGTTGAGACCTTTGCAAAAAAGCCCTTCCCCGACATCCGAAACCCAA
ACACAGGATTTCGGCTGTTTTCGTACCAAATACCTCCTAATTTTACCCAAATATCCCCTT
AATCCTCCCCGGATACCCGATAATCAGGCATCCGGGCTGCCTTTTAGGCGGCGCGGGCGC
ACTTAGCCTGTTGGCGGCCTTCAACAGGTTGAGACCTTTGCAATAACATAGGTTACTAAA
ATTTTATGCTCAATCTCATTTTCAAAATGCAAAACTTTTCTGATTTTTCCTACTTTTTGC
TCAATATTAGGAAGGTTTTAGGCAATTGAAAATTTTTTGGCGCATTTTTATGCGTCAAAT
TTCGTTAACAGACTATTTTTGCAAAGGTCTCAGGTTCAAACACATCGCCTTCAGGTGGTT
TGCGTACTCACTTTGTCATTTCCAATGTTCCAAGTACACCTGCTCCGCTAAGAGGAAGAA
AACTTACAGGAAAACTTATTTTAGAAGTTCCTGCTCAGGTCAATCCAATTCCACAATCTG
TATTAAGGGCGGCACGAGAAGAAAATGTTATCATTAGAGATACAACAGGAAGGATTTACA
ATAGGTGGTGGTTTAGTATTAGGTGGTTGTGCAGGTGCACATCTTGCAAGAAAAGAACCA
TTGATACTAACAGGGAAAACAGGGGCAGGTGCGTCAGCAATTGCAAATGCAAGCATTGGA
TATCAATGGACTGTCAATTTGTCAAAGCCAAAAGAAGGAGCTAAATAATAATGCATTCCC
ACTATATATTTGGTATTTTGATGATTTCATATGTTTTCGCAATGTTATTTAATTTTATAA
TATCATATAAAATATTTAAAGAAGAAAAATTAATTAATGGTTTTTTTGATTTTCTAATTA
AATCAAGCTACCTTAACTTTAAATATTTCAATATATTATTTGGAAAATAAAAAATCTCAA
ATATTTTTTATTTGAAATTATTAAGAATTAATCTGGCGTTGGGGGTTTTTATCTTATCCT
TAATAATTATAAATATTTTTTGTTTTTAGTAAAAATATGGTACAGATATGTACAGCTAGC
TTTGTTTCAGTAAGGTATAACTGTATATAATACTCAGATTTTTCACGTTGGGCTATACAT
GGAAATATATCTGTGATTAAAGATGTTAATGGTAAGTATCGATTAGCACCTGAAAAGCAT
GATTTTAAAATGCATTCCTTTGGGGGGAGAAAAAAGTAATGTAAAAACAATATTTAGAAA
TATGGAAACTATAATTGGTAGCCCAGGGTAAGGGGTACCTTTCAGGATTGAATTTAAAGG
AGAGGTAAATATTGTTAACTAAGTTGAAAATTTTGCTATTTTTGTTCTTATTTGTTTTTG
TATTGGCTATTAATTTGCTTTTCTTCTTTTTTAGTTCGGATATCGAGAGTTTCGGGAACT
ATCAGTTTGAATATGTTTACGATAAAGGTTGGCCTGCTAATTATATTTTAGTCATGAAAG
ATGGAAATGAAGGGAATTTTGATAAAATAATATCCGGATTGGTTTTAGAATATTATAAGG
AGGATGATAACATTTATTTTTCTTATATTGACGGGCAAGGATTTGCTTCAGACTCTTGCT
ATTACAAACCGGAAATTTTATATGGAAAAATTATTTTAAATAAAAATCATATCATTAATA
TTAATAGCATGGAAAAAAATAATTTTCTTTCAGAAGATAAAATAATGAAGGGAACAAGAA

## Appendix A

ATTGGCTAGCAGACCCTAAAAATAAATGTAATATACAGACTCTAGACTAAACGCGTCTTG
CGAAAATACAACGGAATCGATCGTAAATCTTTCCCGCTGTTCTTGAAAGAATGCGAATTT
CGATTTAACTTCGGCACACCGTCTCAACAGCTTAAAATCCTGCGGGATTGGTGTGGGATT
TAGGGCTAATCTAGTACAGCCCCTTGTTTTTCGATACGGAACCGGATAGAGGAAAAATC
GAACATTGCGCCTGCCTTGCTATGATTCACGAAGAAATCTCCGCCATGCCTATGGGCTAT
GAAACCTTGATCGGCGATATGGGCAGCGCACTGTCAGGCGGACAAAAACAACGCATCGTA
TTGGCGCGGGCCTTAATATTGCGAACCGAAAATCCTATTTTTAGATGCAGCGACCAGCCA
TTTGGATATTGCCAATGAAAAAGCAGTCAATGCAAACTTGAATGGCTTGTCTATCATAAA
AATTATGGCGGCACACAGAAAGGAAACGGTGGAATCAGCAGATAGGAAAATGTCTTTAGG
ATAAAAATACAGTTTCAAAAATACTCAAGACTACTGCCGTTTTTTCGCCTGAGCGTCAAA
CTCTGCCAGCGTCATGTTCAAAGTCTGCAAACACGGTGTCATTACCGCATCGACAGCTTG
GTTCACATGATCCCTTTCCACAGGCAACGGACGGTAAACGAAGAGCTTGAAGAGTTCGTT
CAACTCAATCGAATCCGCCCCCGTTTTCAACACCCAACCCTGTCTGCCGGAATAGATGTA
GCCGTGCCGCGCCAGCTTTTCCAAAAGCTCGCCCAACTCGTCGTAGCCCATATTGATATG
CCGTCTGAACTCCTGAACAGGCAAGGCTTTGCCTTCTTTTTGCGCCGCATCCAGAAGCAG
CAGGATTTTCAACACGTCGTCAAACCGTCCGCGCGAGTCGAAGCCCCTGCGGAACGCTTC
TCCCTGCCAGTAGGAGAGTGAAGAAGTCAGCACCGCGCCGCCCAAGACCAGCGTCCACAA
CAGGTTCAGCCACAACAGAAAAAACGGCACGGCGGCAAACGCGCCGTAAATCGAGCGGTA
GCCGTCGAAATTGCCCATATACCAAGTGAAGAGGGAGCGCGCGGTTTCCAGACAAAACGC
TGTTGCCAAAGCCCCGACAAACGCCTGCCGCGCGGGAACGAAGCGGTTTGGCACGAAGCG
GTACAGCCCCCACAGCAAAAGCGTCATGAAGGTCAGCGTCGCCGCCGTTCGCAACGCGCC
CGACCACTGCGGCGCACCTGAGGCAAGCGCGGCATCCTGTACCGAGCCGACCATAAAGGA
AATGCCCACGCCCAAAGACAGCGGCCCGAACGTCAGTAAAGCCCAATAGACGAGAAACTG
CATCATCCACGGACGCTGGGAATTGACCCGCCAGATGCGGTTGAACGTATTGTCTATCGT
CCGAATCAGCATCAGCGAGGTAACGACCAGCATCACGCTGCCGATTGCCGTCAGCCGGTT
CGCCTGCTCGCGGAACGCATTGATATAGTCGAACACCATGTCCGCGCCCTGCGGCACAAT
GGTTTGGTTGACGAAGGAGACGAACGAATCCGACCAGCGGTCGAACACGGGGAAAATCGA
AGCGACCGCCACCATCACGGTCAGCACGGGGACGAGTGCCAGCAGCGTCGTAAACGTCAT
GCTTGCCGCCGCCTGCGGTACGCGTTCTTCATCAAAGCGGCGGACGACGAACCATGCAAA
CGCACAGATTTTATTGTCTGCCAAACCTTGCAAACGTTGTAAAAAGGTCATAATTTCTTG
CCCGGTCAGTAAGTTGGGCATTGATGCCCGATGTTATAGCCAATTTTGCCGTCAGGAACA
AATGCCTGAACTGCGGCTGTTTCAGACGGCATCGGAACAACTGTTATGCCGTCTGAAGAC
CGAACCATTTTAACGGAATCCGCCCATGAACCCAAATCCCCCTCAAAATCCTCGTCCTCT
ACTATTTCCAAAACGGCAGCACCCGCAATCCCGCACTCCGAATCGCTCGCGGCATCGACA
GCGTTGAAGGTTGCGAAGCCGTATTGCGCACCGTCCCCAAAGTCTCCGCCGTCTGCGAAG
CCGTCAAAAAAGATATTCCCGACAGCGGCTCCCGTCCTGACCGCCGAAGAAAACAATATC
GCCTTCGCACAAAGCAAACGCTTGGCGGAACTCGCCGTCAAGTCGGCATAAGCCGCGTGT
TCAGACGGCATGGCGTTCAGATGCCGTCTGAACACGTTTGCCTGTATAATCCGCATCTTT
ACTGTCCAACTTCGCGGTTCGCAAACCTCCCGCGTTACCAAAACTAGGATTCGATATGTC
AAACCAACAAGCCTTGGTCATCTTTTCGGGCGGTCAGGATTCGACCACCTGCCTGATTCA
GGCAATCCAAACCTACGGGCGCGAAAACGTCCAAGCCATTACTTTCCAATACGGGCAACG
CCATGCCGTCGAGCTGGAACGTGCCCGCTGGATTGCGCAGGATTTGGGCGTCAAACAAAC
CGTACTCGACTTGAGCCTGATGCGGCAGATTACGCACAATGCCCTGATGGACGACACCGC
CGCCATCGAAACTGCCGAAAACGGCGTTCCGAATACCTTTGTAGACGGCCGCAACGCGCT
TTTCCTGCTCTATGCCGCGATTTACGCCAAAGGGCAGGGGATACGGCACATCATCGCGGG
CGTGTGCGAAACCGACTTCTCCGGCTATCCCGACTGCCGCGACGTGTTTGTCAAATCGAT
GAACGTTACCCTTAATTTGGCGATGGACTATGATTTTCAAATCCACACGCCGCTGATGTA
TCTGACCAAGGCGCAAACGTGGGCGTTGGCGGACGAAATGGGCGTGCTGGACTATATCCG
CGAGCAAACCCACACCTGCTATAACGGCATCGTCGGCGGCTGCCGCGAATGCCCGAGCTG
TATCTTGCGCGAACGCGGGCTGGCGGAATATCTGGAAAGTAAAAAGGCCGTCTGAACACG
CGCAAACCATAAGGAATACGATATGCCCAAGCTCCATATGTTTTACCTCGGCGGCAATGC
CGGCAGGTCGAATATCGAAGTGCACGACATCCAATTTGCCGTGTGCGACAACTACCGCGA
GGCCGTCCCCGCGCTCAAAGCCGCGTGGTTCGGCGATGCGGACAAAATCCACATCGACGG
CTGGCAGATTGTCGAATGGGCGGACGGTTACGACATCGCCGTATCCGAAACGCCCAAAAC
GAAAATGCCGTCTGAACACGCCCCGCGCCTGTATTTCGCCAATGTCGGCGGTTATCGCGC
GGGTCAGCTTGCCGAGGCACACGCTTTCGGGCTGTTCGCCGCCGCCACGCCTGCCGAAGC
CAAACAAAAAGCCCTGCAAACCCTGTTGACCGACAGCTATGTTCAGCAGCATAAAGACAA
CTTAAAAGACGTGGACAACCTGCTTGCGCTCGACCGCATCGGCAATTTCCATATCCGCCT
GACCCCGAATCCGCACGGCAAACCCGCCGAAATCGGCTTTCAAGGCTATTTGCCCATTTG
AGAACCCATGAAAATCACCAAAATCTTCACCTTCGACTCCTCGCATATGCTCGACGGGCA
TGACGGCAAATGCCAAAACCTGCACGGACATACCTACAAACTCGAAATCACCGTTTCAGA
CGGCATTATCAAAGGCGGCGCGAAAGACGGTATGGTGATGGACTTTACCGACTTGAAAGC
CATTGTCAAACAACACATTACCGACCCCTTCGACCACGCCTTCATCTACCACGGCGGCAA
CAGCCGCGAATGCCAAATCGCCGCGCTTTTGGAGGGCTGGAACATGAAAACCCTGCGCCT
GCCCTGCCGCACCACTGCCGAAAATATGGCGGTCGAAATGTACGGCCGTCTGAAAAACGC
GGGGCTGAACGTGTGCCGCGTGAAATTGTGGGAAACGCCGACATCGTGTGCGGAGTATGA
AGGGGAGTAGGGAATATCTTGAACGTATCGATATAGTAAATTCCAATAAGACATGCCCAA
CCGCGTCATTCCCGCGCAGGCGGGAATCCAGACCTTGATTTATCAGGAATATTTAAAAAT
TGCAGCAATTCCAACTCTCTGGATTCCCGCCTGCGCGGAAAGGACGGTTTAGAGCGTCCT
TATTTGAATTTACCGTAAAACGGTTTTTTCTCCTGTACGGATTCCCCGTTTTTTCAGACG
ACCTTCCATATCAAATACACCCATTAAAAGGAATACCCATGAAACTCCTCTTCATCCTCC
TAGTCCTCTTCGTCGCCGTCGAACATTTCTACATCGCCTGGCTTGAAATGACACAGATTC
CCAGCGAAAAAGCGGCGGAAATATTCAAGCTGCCTTATGAATTTATGGAACAAAAGCAAG
TGCAGACCTTGTTCAGTAATCAAGGGCTGTATAACGGCTTTCTCGGCATCGGGCTGGTGT
GGTCGCGGTTTGCCGCGCCGGACAACGCCGTTTACGGCGCGACGACTCTGTTTCTCGGTT

## Appendix A

TCGTATTGATTGCCGCCGCGTGGGGCGCGTTTTCGTCCGGCAACAAAGGCATACTCGTCA
AACAAGGACTGCCCGCGATGCTGGCGGCGGCAGCGGTGTTGGCGGTATGAAAAAAATCAA
TGTTGCCCCCGAAAATCCGCAATACCGTATCGTCGAAATTTTCGAGAGCCTGCAAGGCGA
AGGCTGGAACACGGGCATGCCCGCCGTTTTCGTCCGCTTGGGCAAATGCAATCTGGCGTG
CGGCTGGTGTGATACCGATTATTTGACATTCGGTATGATGGGCTTGTCCGATATCTTAGG
CCGTCTGAAAACCTACGCCGCCCGCAACATCATCATCACCGGCGGCGAGCCGACCATACA
GCCGCATCTCGATATGCTGCTGGACACGCTCAAGGCGGAAGGCTATTTCCTCTGTCTCGA
AACCAACGGACTCAATCCCGCGCCGCCGCAAATCGACTACGTCGCCACCAGCCCCAAAGC
CTGCTACGCCGCCAAATATGAAAATAGCTGTATCGAAACAGCCGACGAAGTGCGGATTGT
TGCCGATGGTGATGTCCTTGCGTTCTGCGAAAACATGGAACGCAAAATCCGCGCACATCA
TTACTACCTTTCGCCCTGTGAGCAAGACGGTGCGATGAACATCTACGACACCATCCGCCA
AATCGGTATTTTAAACAGTCGCCCCGACGCATCCGTGCATTGGCAGTTGAGCGTGCAGAC
GCACAAATGGGCGGGAATAGAGTAGTTTAAGCAGTGTAACTCAAAGGGACGGCGTACGGT
TTTACCGATGTTTGACATACGGGGAAAGTGTGCCGCTTCTGCGTGGAAATGCCGGCATTT
CCACCGCCCAATCAGGACGGAGCCTTACTGAATAAGATGCTGCCGTTGGGTACAAGCTCG
GCTTCCTAAATTCCGATGGTCTTTTGAACCTTGCCGATACTCTGTGCCAGTGCGCGCAAA
TGGCAGGGTTAGGGAAAACGAAATGCCGTCTGAAACAGCATTCTGTTTCAGACGGCATTT
TTCTGTTGCCGCCAAAAGGAAAAACCGCCTCGGCAATGGATGCCGAGGCGGTTTGAATAT
GGTCGGAATGAGAGGATTCGAACCTCCGCCCCCTTCGTCCCGAACGAAGTGCGCTACCGG
GCTGCGCTACATTCCGAATTAAGTAAGGCGTGATTATAGCGCAAAAAGTGCGGCGTGCCT
ATACCGTTTTGCCTTTTTGCCGCGTGTCGGGCGGATTTAAAACGTTGTGTTTGAATACAG
TGTTGATAATCATCATTATCTTTAAGTAATTCAATAAGATAACTTTCTACCTGACCGAAA
AAATCATTGCCTTTCCCTGACAAACGGTTGATGAAATCGGCAGATTGTTGAAACGCAGCC
GGTTTAAAAGGCTTCGCCGACTTTCACGCCGCCCGCCGTGTCCTGCGGCGAGGCAAGGCC
GGCAACAAAGGCTTGCGCCGCTTGGAAATCCGCCGTCTGCATCACGGCTTGCGCGGCGGC
ACTGCCGAGCGTGTTGGCCATATATTGCCAACGTTGCGCCAAAGTGGGATTGTCAGGAAT
GCGGAAATCTTCGCGCAGTTCATCCACAAGGTCGGGACGGTTGCAGACGAGGACGATGTC
GCAACCTGCCTCAAAGGAAATGCGGGCGCGTTCTTTGATGCCGCCTGCCCCGCACGCGCC
CTCCATAGTCAAATCGTCCGAGAAAATCACGCCTTTGAACCCGATGTCGCGGCGCAAAAT
TTGTTTGAGCCAGATTTCGGAAAACCCTGCGGGCTTTGTGTCCACTTGTGGATAAACGAC
GTGGGCGGGCATAACCGCCGCCATACCTTCGCGGCTCATAATGCGGAAGGGGGCGAGGTC
GGCGGTTTCGAGTTCGGACAGGCTGCGCCAGTCTTCCGGCAAGACCAGATGGCTGTCTCC
TTCGACAAATCCGTGTCCGGGAAAATGTTTGCCGCAGGATTTCATACCGCCTTTTGTCAA
ACCTTTTTGAAGGGCGAGGGCGAGGCGGGCGACCGCTTCGGGATTGCGGTGGAAACTGCG
GTTGCCGATGACGGGGCAGTTTCCCCAGTCCAAATCTAAGACGGGCGTGAAGGACAAATC
GATGCCGCAGGCGGAAAGCTCGGTTGCCAAAACCCGGCCGACTTGTCCGGCGGCGGTTTC
GGCGGCGGACGCGCCGTCTTTGTCCCAAATCTCGCCGAGCGTACTCATTGCGGGCAGGCG
GGTGAAGCCTTCGATGAAACGTTGCACCCTGCCGCCTTCGTGATCGACGGCGATAATGAG
TTCGGGTGTGCGCAGGGCTTTGATTTCGGCGGTGAGTGTTTTGAGTTGTTCGATGTTTTG
GAAGTTGCGGCGGAAGAGGATGATGCCGCCTACGGCGGGATCGAGCAGGCGTTGCTTTTC
CTCTTCGGTCAGGCGGAAGGCGGCAATGTCTGCCATGACGGGGCCGCGCGGAATATGGGG
GACGGTCATTGCGGTTTGCTCCAAAAAGCTTCAGACGGCATATGCCGTCTGAACAGGGAA
AGGGGTCAGGCGTTGGCGCGTTTTTTATCTTTCAACAGAAAAATCAGCACCGCCAATACA
ATGCCTGTCGTGCCAAAGCCCAACAGCGCGGATTTTGTCAGACCCAATGCGAGGTAGCCC
GATGCGGCGGCGGCGGCAACGGTTAAGGCGTAAGGCAGTTGCGAGGTAACGTGGTCGATG
TGGTTGCAGCGCGCGCCGGTGGACGACAGGATGGTCGTGTCGGAAATGGGCGAGCAGTGG
TCGCCGCATACCGCCCCCGCCATTACTGCGGACATACACGGGATAATCAGCGCGGGTTCG
ACTTTGACCGCCATGGCGGCGGCAATCGGCAGCATAATGCCGAACGTCCCCCAGCTTGTG
CCTGTGGCAAACGCCATCACGCTGGCGAGCAGGAAGAGGATGACGGGCAGGAAGCCGGGA
TGGATGTTGCCCGCAACCAGTGTGGAGAGGTAATCGCCGGTGTGCATTTCGCCGACAACC
GTACTGATGAGCCAAGCGAGGATTAAAATGGCGATTGCGCCGAACATAGATTTCGCACCC
TGCCAAACGGCTTTGGGATAGTCGGCGGTTTTAATCGTGCCGAGCGTGCAGAGAACGACG
GCAAGGACGCCGCAAGTGCCGCCGAATACCAGCGAAGTGTTTACGTCCGTGTTTTCAAAT
GCCCCCAAAATGCTGAAGGTTTCGCTTGCCTGCGCGCCGGTGTAGATCATGGCGGAAACC
GTTGAGGCGATTAAGGCCAAAACGGGAATAATCAGTGCGTAAACACGACCTTTGGTAGCG
TCTGAAACGGCAGTTTCATCGTGGGCTTCGTTCAACGCGGCTTGTTCGAAACGTGCCATC
GAGCCGATGTCGAAGGAAAACCATGCGACGACGAACACCATAATCAGGGCAAACAGTGCG
TAATAGTTCATCAGGCTCATGGCGACAAACGTCCCCATCGGCGTGTATTCGGTGATTTTG
TAGGTAACGAGCAGTCCGGCAAGCGTGGCGATAATCGACGCGCCCCAGCTTGAAACGGGC
ATCAGCACGCACATAGGAGCGGCAGTGGAGTCGAGGATGTAGGCGAGTTTGGTGCGGGAA
ACTTTAAACTTGTCGGTAACGGGGCGGGCAATCGCACCGACGGCGAGACTGTGGAAATAG
TCGTCGATAAAGGTTACGAACACGAGGCAGGCGGTCAGCATTTTCGCGCCGCGCCGGTTT
TTAATGTGCCGTTTTGCCCAGTCGGCAAACGCCTGATTGCTGCCGGAGTAGGTCAGCAGG
GAAGTAAAAATACCCAAAAGTATCAGGAAAACCAAGATTTTTGGTTTGCCCAGCGACCAA
TCGCCGTCTGACCAAGCCAAGCCGACGACCATGTCTTTCAGGTGTGTCAGACCGTCGACG
GGGTTGCCGCCGACCAAAAAGGCAACGCCGACCAGAATACCGATGCCTAAAGACAGCAGT
ACGCGGCGGGTAATGACGGCAAGTGCCAGTGCCAAAAAGGGTGGCACAACCGAGAAAAAT
GAATGTGAATAGTCGATCAGCTGCATGGTTATGGGGGTGTTAAGCGTCCGGATGGGAGCG
TATCTGTCCGCCTCCGGTTTGGGTTTTGTTGGCAAAATGGGCGGAAATATTTTTTGTCGT
AAAAAATATTTGTTTAAAATCAACCAACTGATTTTTGTAAAATGCCCGTTAATCGGTATT
GACGGGCATTTTATCATTTAAAAAATATTTTGGTTAAATTATGTGTGTTATTGCAGGTTT
AATGCGATAAACAGCGTGTTGCCACGGCGCATGATCAGCAGGGGGACGTTTTTGCCTGCC
TTGTCCATAGCTTTGCGGAAACCGGCTTCGTCATTGACGGGGACTTGCCCGACGGCAAGA
ATTTCGTCGCCGCGCCTCAAGCCTGCGCGTTCTGCCGCGTCGGAAACCCGTACGACGACG
AGGTGTCCGCCGCTGCTGTCGGTATGTGTCTGAAGGGTAATGCCTGCGGATTCGACCGAG

## Appendix A

AACGTACCGGATTGCTGTTCGGTGTAGGGGGCTTCATCTGTTTTGGATGATGCGCCGATA
TGCTCGGCGGCGTTGCCCAGCTTGACTTTGATTGTGATTTCTTCGCCTTTGCGCCATACG
CCGAGGCTGACTTCTTTTCCCGGCGTAATGGCGCCGACCATAACGGGAAGGTCGCCGGAA
GAACGTATTTCTCCGCCGTCGAGGCTGAGGACGATGTCGCCCGCCTGCAGGCCGGCACGT
TCTGCGGGGCTGCCGGGCAGGATTTTGGCAATCAGTGCGCCGCCGGCTTTGTCCAAACCG
AACGATTGTGCCAAACCGTAGGATACTTCTTGAATAATCACGCCCAGTTGTCCGCGTTGG
ACTTTGCCGGTGTTTTTCAGCTGTTCGGCGACATTCATGGCAACGTCAATCGGGATGGCG
AAGGAAATGCCCATGAATCCGCCGCTGCGGCTGTATATTTGCGAGTTGATGCCGACGACC
TGTCCTTTTAAGTTGAACAGCGGGCCGCCGGAGTTGCCCGGATTGATGGCAACGTCGGTT
TGGATGAAGGGTGTGTAGCTTTCGTTGGGCAGGCTTCTGCCTTTGGCGGACACGATGCCG
GCGGTCACGCTGTTGTCGAAGCCGAAGGGCGCGCCGATGGCGGCGACCCATTCGCCCGGT
TTCAAATCTTTGGGATTGCCGATTTTGACGACGGGCAGCTCTTCCGTTGCGTCGATTTTC
AGAAGGGCGACATCGGATTGGACATCCGAACCGATGAGTTTGGCGGTATATTCGCGCTTG
TCGTTGAGCAGGACTTTGATACTGCCCATGCCGGTAACGACGTGGGTATTGGTCAGGATG
TAGCCGTCTTTGCTGATGATGAAGCCCGAACCGAAGTTCAATCCGCCGTCATCTGCTTCT
TCTTGGGGGATTTCGGGCATATTCGGGACGAGGCGTTTGAAAAATTCGTAGAACGGGTCG
TTGTCGGCAATCGGGTCGGAATCGTTTTCGGCATTGCCGCTGCCGTTTTGGGTGCGCGGG
GCGGGGGCTGCCTGAATATTGACGACTGCCGGACCTTCACTTTGAACCAGTTGGGCAAAG
TCGGGCAGCAGCATACTGACGCTGCCGTCGTCTTTGGTGTGTTCGATGCGTTCTACGAAG
GATGCTTCTTTTTTGTCCGCACCGAAAAAGCTGCCTGCCTTGTCGCAGCCTGCCAGCGAG
GCGGCACACAGTGCTGCCAAAGCGAGGTATTGGTATTTTTTGAACACGTTTTGTCCTTTG
TCGGATGCCGGTACCGGCTTTAATGCCGTCTGAAGCGCATTTTGTCGGCTTCAGACGGCA
TAGGTTGAAATTCTACAACGTCCGTCCGAATTTTCAAGCGTTTCATTTTGAAGGGCGGCG
GCGGTCAGGCTTTGGCGGGATATTCGCACAAATCGTTGATGATGCAGGTTTGGCATTGCG
GTTTGAGTGCCTTGCAGGTGTAGCGTCCGTGCAAAATCAGCCAGTGGTGCGCGTCCATCA
GAAATTCTTTAGGAATGAAGCGCATCAGTTTGTCTTCGACTTCGCGCACATCTTTCCCGG
GGGCGATTTTGGTTCGGTTGGATACGCGGAAAATATGCGTATCGACCGCCATGACGGGAT
GGCCGAACGCCGTGTTCAATACGACGTTTGCCGTTTTGCGCCCCACACCCGGCAATGATT
CCAAAGCCTCGCGGTCTTCCGGCACTTCGCCGTTGTATTTTTCCAGCAGGATGCGGCAGG
TTTGCATAATGTGTTTGGATTTGGTTTTATACAGCCCGATGGTTTTCGTGTATTCCATCA
CGCCGTCCAAACCCAAATCCAGCATCGCCTGCGGCGTATCGGCAACGGGAAACAGCTTCG
CCGTCGCCTTGTTTACGCCGACATCGGTCGCCTGCGCTGAAAGCAGAACGGCAATTAAAA
GCTCGAAAGGGGAGTTGAAATTCAGCTCGGTGGTCGGATGGGGGTTGGCGGCGCGGAAGC
GTTCGAAGATTTCTTGGCGGATGTGTCTGTTCATTTTTTTATACGGTGGGTTTGTGTGTT
CGGCATTATAACGTATGGTTCAGGCGGCGTAATATTGCATTCCCCACAGAATGAAGGCGT
AACGCGCCGTTTTGCCGATAACCAGCATCAGCCCGCTTGTCCACGGATTCAACCGCAGCC
AGCCGGCGGCAAGCGGCAGTGCGTCGCCGACGACGGGCAGCCAGGTAAACGCAAGCAGCC
AAATACCGAAACGCCGCATCAGATTCAGTGTTTTTTCAGACGGCATTTTTCGGGAGGGCA
GCAAACGCCCCATCCAATAGGAAACCATACTGCCCAATCCGTTGGCAAGGCCGGCGCACA
GCAACGCGCCGTATGCGTGTTCGGGAAAGCGGTGGACGAACAGGGCAAAGGCGGCTTCGG
ATGTGCCGGGCAGGAGGGTGGCGGAAGTGAATGCGGAAAAGGCGAGGGCGGCGTAGGTGT
AGGAGGGTATCATTGCAAACAGTCTCAAACAGGTAACAATCGGCGACGGATTGTACGGTA
TAGTGGATTAACAAAAACCAGTACGGCGTTGCCTCGCCTTAGCTCAAAGAGAACGATTCT
CTAAGGTGCTGAAGCACCGAGTGAATCGGTTCCGTACTATTTGTACTGTCTGCGGCTTCG
TCGCCTTGTCCTGATTTTTGTTAATCCACTCTATTTTCACGCCCCCGCCGAAGGGCGGAG
GACGGTGCAAAAAATACGGCACAGCCGTATGCCCCTTTTTTGTCGGGCATACGACATTCT
TTCCGCTCCGGTTTTGATGCCACGATGCGGCATTTCCGAATTTTCCGGATACGGCGGCGG
ATTTTCATTTTATTGGGAACGGTTTTTGCAAGTCCGCCGGAATTTTTTAAAATCTATTAA
AATCTATGCAAGCAACTGTAAAATATTAATTTCTGCTGCTTGAATTTCAGATCGGCGCAT
TGCCTGCATCCGATAAAGTTTGCAAAATGTTCAAATATCAGTATGATTTGCATTGCCGTT
AAGAAATGTCAATTTCTATTTTCTTGAAACGGGTAATATTCCGACACCACGAAAGGCAAA
TCATGTCTGCGCAATCACAAAACAATCATACGTCCCCATTGGTCGTCTTGACCACGCTGT
TCTTCATGATGGGTTTTATTACCTGCATGAACGACATCCTTATCCCTCATTTGAAAGAAA
TTTTCGACCTGTCTTACGTTCAGGCGATGCTGATCCAATTCTGTTTCTTTACCGCCTATG
CGGTGATGTCCATCCCGATGGGGGCTTTTGTCGGCAAAGTCGGCTACAAAAACGGCGTTA
TCGGCGGCTTTCTGCTGACGGCGGTCGGATGCCTGCTGTTTTATCCTGCTGCGGGCAGCC
ATTCTTACGCGGTATTTTTGGGCGCGTTGTTTATTTTGGCTTCCGGCGTAACGCTGCTTC
AGGTCGCCGGTAATCCTTATGTTACCCTGCTGGCGAAACCCGGCAAGGAATCGGCAACAC
TGACGCTGGTTCAGGCGTTTAACGCTTTGGGTACGACCATTGCGCCGCAAATCGGCGCGT
TCCTGATTCTGGCGGACGCAACCCAAACCGTCAGCAAGGCGGAACAGATTTCTTCCGTAC
AGATTCCCTATTTGGGACTGGCGGGGCTGCTGATTATCCTTGCCGTTTTCGTGAAAATGA
TCCGGCTGCCCGACGCGCGCAAAATTGCCGCCGAGGAAAGCGCGCACAACCACGACGGCA
AAACCAGCGTATGGCAATACAAACATCTCGTGTTCGGTACGGCAGGCATTTTCTGCTATG
TCGGCGCGGAGGTGTCTATCGGTTCGTTGATGGTCAACGTATTGGGTTATCTGAAAGGGC
TGGATCATGCTTCTGCCGCGCATTACCTGTCGTTCTATTGGGGCGGCGCGATGGTCGGAC
GTTTCCTCGGTTCGGCGGTGATGGCGAAATTCGCGCCCAACCGTTATTTGGCGTTTAACG
CATCGGCTGCGGTCGTACTGCTTGCCGTCGCGATGGCGACGGGTAGCGGCAATGCGGATG
TGGCGATGTGGTCGCTGCTTGCCATCGGTTTTTCAACTCGATTATGTTTCCGACGATTT
TCTCTTTGGCAACCAAAGGATTGGGAAAATTTACCAACGCGGCTTCCGGTGTACTGTGTA
CCGCGATTGTCGGCGGTGCGGTCGTTCCTGTCGTGCAGGGCTGGGTGGCAGATACTTACA
CCCTGATGTCTTCGTTTGTCGTTTCCGTCATCTGTTATCTGTATATCGTGTTTTTTGCGG
TGTACGGATATAGGGCGGACAAATAATCTTTTTCTTGAGAAATGTCGTCTGAACATCTTT
CAGACGGCATTTTTGCGTACCGGTGTTTGCGGCGTGTGTGCCGAGGTTTTAATACTTCAA
TCCATAAAAGTCTTATATGTCAACAAACAAAAAAAATAAAAAATTATATTTCAAAAAAATT
AATTTAAATTGAGAAAATTGCCGTTTTGTTTCTGTCCGGCTTTTGTAAAACGCTAAAATG

## Appendix A

CCGTCTGAAAACGTCGGGCGGATTCGGTATGGTGTGTTAGAATCCGTTAACTTTATATCA
AATCGGGCAAAGAATCATGTTCGCTTTCAAATCCTTACTCGATATGCCGCGCGGTGAGGC
ACTTGCCGTCGTCGTCGCTCTGATTGCCGCGATGGGCTATACCATCATTTCATTGGAGTG
GTTGCCGCATATGTCCATTATTGCCGCCATCGTCGTGCTGATTTTGTACGGCTTGGCGCG
CGGTTTGAAATACAACGATATGCAGCAGGGCATGATAGGCGCGTTGAATCAGGGTATGGG
CGCGATTTACCTGTTTTTCTTCATCGGGCTGATGGTCAGCGCGCTGATGATGAGCGGCGC
GATTCCGACGCTGATGTATTACGGTTTCGGACTGATTTCCCCGACTTATTTTTATTTTTC
CTCCTTCGCGCTGTGTTCCGTCATCGGCGTGTCCATCGGCAGCAGCCTGACCACCTGCGC
CACTGTCGGCGTTGCCTTTATGGGGATGGCGGCGGCGTTTCAGGCCGATATGGCGATGAC
GGCGGGCGCGATTGTTTCGGGCGCATTTTTTGGCGACAAAATGTCCCCGCTTTCGGATAC
GACGGGTATTTCCGCGTCCATCGTCGGCATCGACTTGTTTGAGCACATCAAAAATATGAT
GTACACCACCATCCCCGCGTGGCTCATTAGTGCGGCACTGATGCTTTGGCTTTTGCCGAA
TGTCGCCGCGCAGGATTTGAACAGCGTCGAATCCTTCCGCAGCCAGCTTGAAGCCACGGG
ATTGGTGCACGGCTATTCGCTGATTCCGTTTGCGCTGTTGGTCATTTTGGCATTGATGCG
CATCAACGCCGTCGTCGCCATGCTCTTTACCGTCATGGTTGCCGTTGCTGTAACGTATCT
GCACAGCACGCCCGATCTGCGTCAGCTCGGTGCGTGGTTTTACGGCGGCTACAAACTCGA
AGGCGAAGCGTTTAAAGATGTTGTCAAACTGATTTCGCGCGGCGGTTTGGAAAGTATGTT
TTTCACGCAAACCATCGTGATTCTCGGGATGAGTTTGGGCGGACTGTTGTTTGCGCTCGG
TGTGATTCCTTCCCTGTTGGAGGCCATCCGTACCTTCTTGACGAATGCCGGACGCGCGAC
GTTCAGCGTTGCCATGACTTCGGTCGGGGTTAATTTCCTGATCGGCGAGCAATATTTGAG
TATTTTGTTGTCGGGTGAAACGTTCAAACCCGTTTACGATAAGCTCGGTCTGCATTCGCG
CAATCTGTCGCGGACGCTGGAAGATGCGGGGACGGTGATTAACCCGCTCGTACCGTGGAG
CGTATGCGGCGTGTTCATCAGCCACGCGCTGGGCGTGCCGGTTTGGGAATATCTGCCGTA
TGCCTTTTTCTGCTATTTGAGTTTGGCTTTGACCCTGTTATTCGGTTGGACGGGGCTGAC
TTTGAGCAAAAAATAAGCGGATAAGCGAAATGCCGTCTGAAACTTGCAACGGTTTCAGAC
GGCATTTTTATGTTTGGCGGATGGGGCGGATTGAAACAGAAAACGCCCGTACCGTCATCC
TAAACTGTGCAGAAACGGCGGTGCTTACTTCACGCGGGTCGCCATCAGCGTATGCAGGCG
GCGGTTGTCGGCGCGTGCGACGGTGAACTGCAAACCGCCGATAAGGACTTTTTCGCCGCG
CACGGGCAGATGTCCCAACTCTTGAATGACCAGGCCGCCAATGGTGTCGGCTTCTTCGCT
GCTGTATTCCGTGCCGAAGAAGGTGTTGATGTCTTCGATTTCGGTAGCTGCATGGATGCG
CCAGCGTTCGGAAGAAACGGCATGGATATTGTCGGCGCTATCGTCTTCGTCAAACTCGTC
TTCGATTTCGCCGACGATTTGCTCGATGATGTCTTCAAAGGTGACCAAGCCGGATGTGCC
GCCGTATTCGTCGATGACAATCGCCATATGGTTGCGCTGTTCGCGGAACTCTTTTAAAAG
GGCGGTCAGCGATTTGCCTTCGGGGACGAAGACGGCGGGGCGGAGAATGGATTTGAGGTG
GAACTGCTCGGGGTTAAACATATATTTGAGCAGGTCTTTGGCGTGCAAAATGCCCAAAAC
TTCGTCTTTGTCTTCGCCGATGACGGGGAAGCGCGAATGGGCGGTATCGATAACGTAGGC
GGTGATGCGCTCGATGCTGTCGTTTTCTTTTAAAACGTTCATACGGCTGCGCGTAATCAT
CGCGTCGCGCACTTCCAAATCGGAAAAATCGAGGACTTTTTCCAATCTTAAAAGCGTATC
CGCATCAAAAACTTCCTGCTCGTGCGCCTGCCGAAGCAGGTTTAATACGTCTTCGGCGGA
ATCGGGTTCGCGGGCGAGTCGGGCAATCAGGCGTTCAAAAAAATTCGTTTTCGGTTGTGC
GCCGTCCATTTTAATGTCAGTCCTCTTGGTAGGGGTTGGGGAAGCCTGCCGCCCGCATCA
GGCGGATTTCTTCGGCTTCCATTATTTCGGCTTCGTCGTCTTCGATGTGGTCGTAGCCCA
TCAGGTGTAAAGTACCGTGTATGGTCAGGTGGGCAAAATGCTGCTCGGGTGTTTTGCCTT
GTTCGGCGGCTTCTTTCAAAACCACTTGCGGGCAGATAATCAAATCGCCGTACAGTTTTT
CCGAAACTTGGCAGGGCAGGATTTCGCCTTCGTTGAGCGCGAAACTCAATACATTGGTGG
CGTAATCTTTGCCGCGGTAGTCGCGGTTGTAGGCTCGGGCTTCTTCTTCGTCCAGAAGAA
TCAGGCTGATGTCGGCGCGGCGGTATTCATTTTTCAAGGCAGACCACGCCCAGCGGTAGA
AATCGCGTTCGGCTGGGATGCCGGCGGCGGAAGAGGCGTTTTCAAAGTTCAAATGGAAAC
GTTGCCGCTGCAACGTTAAGAAAGGGTATTTTTTGGTGCGTTTCATTGTGGCGGGTTTCG
TGTTTTGTGGGTGTAAATATAACATAGACCTGACGGTGCCGTCTGAAGAAACGTTCAAAA
TATGATAGACTTCACGCCGTTTCCATTCTTTGAACGCATTGAACATGAACCCGAAAAAAC
TCGTCATCGCCAGCCGCGAAAGCCTGCTTGCTATGTGGCAGGCAAAGCATATCCAAGGCC
GTCTGAAGGCTCTGTATCCCGATTGCGAAGTCGAGATTTTGGGCATGACCACGCGCGGCG
ATCAGATTTTGGACAAAACTTTGTCAAAAGTCGGCGGTAAAGGCTTGTTTGTCAAAGAGT
TGGAACAGGCTTTATATGACGGGCGCGCCGATTTGGCGGTGCATTCGATTAAGGACGTGC
CGATGGATTTGCCTGAAGGTTTCGCGCTTGCCGCCATCGGCGAACGCGCCAATCCGTTTG
ACGCGTTTGTGTCCAACCAATACACGCGTTTGGAAGAAATGCCCGAAGGCGCGGTTGTCG
GCACATCCAGCCTGCGCCGCGAAGCCCAGTTGCGTGCGCGCTATCCGCATTTGCTTATCA
AACCTTTGCGCGGCAATGTGCAAACCCGTTTGTCCAAACTCGATAACGGCGAATACGACG
CAATTATCTTGGCTGCCGCCGGTTTGCAGCGTCTGAAATTGGACGGACGCATCCGCATGA
TTTTGTCGGAATCCGACAGCCTGCCTGCCGCCGGACAAGGCGCATTGGGTATCGAAATTG
CCGCGCACCGCGAAGATTTGTATGAAGTTTTGAAACCCTTGAACCACGGTGTTACCAATG
CCTGCGTTACCGCCGAACGCGCCCTCGCACGCGCTTTGGGCGGAAGCTGCCAAGTGCCTT
TGGCCGCATATTGCACGGAAGAAAACGGCTTGCTGACCTTGCGCGGCTTGGTCGGACACC
CCGACGGTTCGGTTGTGTTGCGGGCGGACGCGCAAGCCCCTGCCGAATATGCCGACGCGC
TCGGACGCGCTGTCGCTAAGAAATTGGCGGACGACGGTGCGCGGGAATTGATTGGAGCAG
TATTGAATACGGAAAATTGATTTTATCGAAAATTTAAATAAAATAATATAAGTTATTGTT
TTTAATCAATTTGTTTCATCAGTTTCACTCGCCTTATTTCGTCATTCCCGCGCAGGCGGG
AATCCAGTTTGCTCGGTTTCAGTTGTTTCTAATCAATTCTTGCAGCATTGGATTCCCGGA
TTCCCGCCTGCGCGGGAATGACGGCGGAAAGGTTTTTGTGGCTTCGGATAATACTGTGGC
GTTCAAATTTTGAATTTGAGAATGATGATATTCGTATTTTTTATTTGAGTTCATCATATT
TGGTTGATTTTATAGATGTTTTTAGCTTGTTTGAAATTGTTATGGTTTATTGTTTTTTAA
CAAAAAACAGATGCCGTCTGAACTGGTTAAGGTTCGGACGGCATTTTCATATGGCTGTGC
TTTTTACAGTACTTTCACGATGCTTTCGCACAGATAATCGATGTTGTTGTCGGTAATGCC
GGCGACGTTGATGCGGCCGGAGCGGACGGCATAAATGGCAAACTCGTTTTTCAGGCGGTC

# Appendix A

GACTTGTTCGGGAGTCAAGCCGCTGAAAGAGAACATACCGTTTTGTTTGATAATGAAATC
AAAGTTTTGGCTTGCACCTTTGGCTTTGAGCAACCCGACAAATTTTTGGCGCATGGCTTT
GATGCGGCCGCGCATTTCATCGAGTTCGGCAATCCATTGTGCTTTCAAATCATCATTTTT
CAACACCAGCGCAATGGTGTTCGCACCGTGTGAAGCCGGGTTGGAATACAAGGTACGGAT
GATGGTTTTGACTTGGCTGTGGGCGCGGGCTGCTGTTTCTTCATCTTCGGCCACCAAAGT
GAACGCGCCGACGCGCTCGTTGTACATACCGAAGTTTTTGGAATAAGAGCTGGCAATCAG
CAATTCTGTATTGTGTTTCAAGAACACGCGCAAGCCGTAGGCATCTTCTTCCAAACCATT
GCCGAAGCCTTGGTAGGCAAAGTCAAACAGCGGCAACCAGCCTTTTTCGGCAGAAAGTTT
TGCCAAAGTTTCCCATTGTTCGGGCGTAGGGTCGATGCCGGTAGGATTGTGGCAGCAGCC
GTGCAGCAGGACGATGTCGCCTTTTTGCGCTTGGCTCAAGTCCTCAATCATGCCGTCCCA
ATCCAAACCGTGTTTGGCGGCATCATAGTAACGATAAGGTTTGTCTTGGATACCGACCGC
TTTGGCGATGGCGTTGTGGTTGGGCCAAGTCGGATTGGAAATCCAGATGGTTTGCGCGTT
CAACTGGCGTTTGGCAAACTCGGCCGCAATACGCAATGCGCCCGTACCGCCGAGGCTTTG
CGCTGTTTTGGCGCGACGGCTGGCGATGATTTCGTGGTCTTTGCCGAACAGCAGGATTTG
GGTTTGCGCGTTGTAGTCGGCAACGCCGTCGATGGTGAGGTAGTTTTTGGTGGTTTCGCT
TTCCAACAGGCGTTTTTCGGCTTCTTTGACGGCTTTGACGAGGGGTGTCGCGCCGGATGC
GTCTTTATAAACGCCGATGCCGAGGTTGACTTTTTCGGGGCGGGTTTCGGCTTTGAACGC
TTCGCCCAAACCGAGAATCGGATCGGCGGGGGCGGCTTCGATGTGCTTGAAGAACATAGC
TTGCTCCTTGATGGGGACGGAAGGTCATTCGGGTTTGCCGATTTTACGCTGTTTTACACG
GGCTGGAAACAGACGCAATCACGCCTGCCCGATATGGGCGAAGGTTTCCCAGTTTGACTG
TATGTGTTCTGCAAGCAGGGGCAGGTCTTGTTCGGCGGCTTCGTAGTATGCGCCGTCCCA
TTCTTCAAATTCGGGGAACTGTTTGCGCAGGGAAGGGATGTCTGCGCCTCCGTCGGCGAG
CGTTTCGATGGTTTTGCCGTGTTGTTCGTAGAGGGCTTTGGCTTTGTCCAAGACTTTCGG
CACGGCTTTGATTTTCCAGCGGCGCAGGCTGTCGGCAAGCGGGTTGCGGAAAATATATTC
GCCGTAACCGGATGCGATGAGTTGCACGAAGCCGCCTTCTTCGACTTGGCTGTCGAGGTA
GCAGAATGCGGTCAGCGTGTGCTGGTCGTCGGACAGGCAGGAGAGGGATTCGTCGCCGGT
TTGGGCGGTGTGTTCGAGGTAGGCGGAAACGAGGGTATAGAGCAGGGCGGATGGCTCTTG
TTGGCGGATGTCTTCCGGAAGGGTAAGCGCAGTCATGGTATGCCGTCTGAAAAGTGGGGA
TTATAGCGGATTGCGGCTTTGCGCCGAAAATATCCTTTAGCCTGCCGATGGCGTAAAATA
GGCGCACGCCAACCACGCAAAGGAAAATCAAATGGACAATCTGAATCCGCAGGAAATTTC
CGTGTTGCCGGAAAATCTGCCGCTGTATTGCTCGGGACCCGGCAACGAGCAGTGGAACGG
GCATCCGAGAGTGTTTTTGCCTTTGTGCGAAGGAGAATCGGGCAGCGTTGCCTGCCCGTA
TTGCGGCACGCGCTACCGCCTTGACGGCAAGATGCCGCATCATCATTACGCCTGAACGCA
AACGGCGGAAAAATGCCGTCTGAAGCCTTTTTCGGTTTCAGACGGCATTTGTTTGGCGGG
GCGGGCTTGTTCCGGCACCGGGATTCTGCCCGACGCGCCGCCCAGACGGTGAACGGCGGT
TTCCGTTCCCCGCGTGCTGCCGCTATGGATGGTGGCGTTTCGCCTAGAGGAAGAAAATCA
TTGCCGCGACGACGGCAATCACGCCGTAAATCGCCATCGGGATAACGGTTTTCTTGATAA
TCGCACCTTCGGAATTTTTCACATCCAATACGGTACATACGGCGATGATGTTGTTGAGGC
ACACCATATTGCCCATCGCGCCGCCGACGGACTGCAACGCCAGAATCAGGGTAACGGACA
GGCCGGTATCCAAGGCGATTTGCTGCTGAATCGGGCCGAAGGTCAGGTTGGACACGGTGT
TGGAACCGGAGAAGAACGCACCGATCGCGCCCAGATACGGCGAGAAATAAACCCAGTGTT
CGCCCGCCATTGCGGCAAATTCCTTACCGATGATTTTCACCATCGAATTGTCGCCGCCGA
CCAGCATCAGCTGAACCATAATCAGCGCGCCCATCAGGGCAAGCAGCGGTTTTTTGGTTT
GATTGAAGGTTACGGAATAAATCGTCCAGGCATCTTTGAATTTGGTTTTATACAGCAGGA
TGCAAATCCAAACGGTCAGCACAAACGGAATCCAAGCCGGGACGTACAGCGTTTGGTAAG
ACGCGCTGACATCTTGTCCGAAAATATTGCCGAAGGTAATCGTCAGGGAGTCGCTGACGG
TGATTTTGGACAAATCAAACGGCAGTTGGAAGCTGAACCATTCTTCTTTGCTGGTCAAAA
TGCCTTTGATGCCGAGCTGTTTGATGCGCGTAACCACCAGCATGCCGATCAGCATACCCA
AAGGGGCGAGTGCTTTGGCGACTTGGGCGAACGGCACTTTTTCGGCATTCGGGTCTTTGG
CGTGGTCTTTGCTCAAGCCCCAGCCTTGGTTGGCGGCGAATACGGACACCATCAGGCCGA
TCGCGCCGGCGACGAGCGACGGGAATTCTTCGTTGACCATCGCCAATGCGACATAAGGAA
TGGTGCAGGAGAAGACGGCAATGGCGACGAAGCCCAAGTTTTTGCGGATTTCAGACCAAG
GTACGATGAAGCCCAAGCCGATGACGGGGATGACGAAACCTGCGAAGAAGTGCATTACGC
CGGTCTGCCTGCCGATGGCGAGGATGTCTTCGGCACTCAGGTTCAGCGGTGCGAAACCGA
ACCAGGTCGGCGTACCGACCGCGCCGAAAGAGACGGGGACGGAGTTCATCACCAAAGTGA
AAATCGCCACTTTCAACGGGTTGAAGCCCAAGCTCATCAGAATCGGCGCGGCAATCGCGG
CAGGCGTACCGAAGCCGGATGCGCCTTCAATCATAAAGGCAAAAGCCCAGCCGATAATCA
TCAGTTGCGCTACGGGGTTCGGGCTGATGGTCGCCAGCCATTTGCGGATGACATCGATGC
AGCCCGTGGTTTCCATCATACGGTTGAACATAATCGCGCCGAAAATCACGGTAATCGGCG
TGAGCGTTTTGACGAGGCCGGAAGCGGCGGTGGCGTTGAGCAGCATGCCCGCATCGTCGA
AGTAGAAAAGTTTGATGGCGTAAATCAGCACTGCGGTAATCGGCAGCGCGACGTAGGAGG
GCATACTGTTTTTTTTCACCATCAGCCAAATCAGCAGGACGATGGGGAATATGCTGAGGA
AAAGTGCCATAACGAATCCTTTTTAGGCATTTGCATCATAAGGCGCGTCGAGGTTTGGAA
AGACGTTCAAATCCCGTACACCCGATATTTTGGTTAAAAGATAAATTGGTAAGACCAATT
GTTATGCGTTTGCACACTTTACGTAATCTTATGTAATCGGTCAAGCATTTTATCGATAAT
GGCTATTAATGCGGGTTAAGGATAGTGATGATGCGGCGGAGGGAGTGTGGGAAAGGGGCG
TGTAAAAAAAGCCGCCCGAAAGGCTTCAGACGGCATTTTCAGTATTTTTCCAGCGGCACG
AATACCGCGCCGTCCCCGTCCACGCGGAGGATTGAAGCATAGTCGTTATGCCAGTCGCCC
AAAACGATGCGGGTAAAGCCGTTTTCGTGATGGATATGCTCGCGGTGGGTGTGTCCGTGT
ATCAGCCTTTCCGCACCGAAGGCGCGTACCTGCCGCGCGGTAAAGGCGGCATTGACATCC
ATAATATCGGCGGGCTTGACCTGTTTTTCCATTTTGCTGACACGCCTGATTTTGGTGGCA
AGGCGCGTGCGCCACTTCAGGGGCAGCATTAGGAACAGTTTTTGCAGCCGCTTCCGATGC
ACGATTTTGCGGAAACGTTGGTATGCCCTGTCATCTGTACACAGAGTGTCGCCGTGGCAG
ATGAGGGTTTTGCAGCCGAACAAGTCCAAAACCGAGTAATCCGGCAGCAGCGTCATGCCC
GCCTGCCGGCAAAAATCCTGACCGATCAGGAAGTCGCGGTTGCCCCTGACGAAGAACACG

# Appendix A

GCAACGCCTTTGTCGGACAATTTCCTGATTTCACGCGCAACCGAAGTATTCAACTCGGAA
ACTTCGTCATCGCCCACCCAAAAATCAAACAAATCGCCCAAAATGTAAATCGCCCGCGCC
TGCCCGGCGGCGGAAGAACGTAAAAAACGCAGCAGCAGCGCGGTCAGTTCGGGCTGCTTT
TCGCTCAAATGCAGGTCGGAAATGAAATAGGCGGGTTTCATAGGCAGGTTTCCAATCGGG
CGGATGTCGGGGCGGATTATAACGCGCCCGGCGGCGGGGCAATACGGCAAATGCCGCGCC
AAGCATCGGGCATTGGCGGAACCGGGGTTCGGGCGCGTTAAAAATGCCGTCTGAAGGCTT
CAGACGGCATCGAGGGTGCGGGATGCGGTAAGGTTTTGCCGGCAAGATATGGGGTGGTGC
GGCGGGATTTCCGTTAAAATACGCTTCTTTTTTATTTTTTCCGACCATTATGCGCCTGAC
CCATATCAAACTCTCCGGCTTCAAATCTTTTACCGACCCGACCACGATTCATGTGCCGGG
GCAGCTTGTCGCGGTTATCGGGCCCAACGGCTGCGGCAAGTCGAATGTGATTGACGCGGT
GCGCTGGGTGTTGGGCGAGGCTTCGGCGAAGCAGCTTCGTGGCGAGAGTATGCAGGACGT
GATTTTTAACGGTGCGGCGACGCGCCGTCCTGCGCCGAGGGCTTCGGTGGAGCTGGTGTT
TGACAACAGCGACCACAGTTTGCAGGGGGCGTGGGGGCAGTATGCCGAGGTGAGCATCAA
GCGGCAGCTGACGCGGCAGGGCGAATCGACTTATTTCATCAACAATCAGACCGTGCGCCG
CCGCGACATTACCGATTTGTTTCTGGGTACGGGCGTGGGCGCGCGCGGTTATGCCGTTAT
CGAGCAGGGGATGATTTCGCGCATCATCGAAGCGCGGCCGGAGGAGTTGCGCGCCTATAT
CGAGGAGGCGGCGGGCGTGTCCAAATATAAGGAACGCCGCAAGGAGACGGAAGGTCGTCT
GAAAGACACGCGCGAGCATTTGCAGCGTTTGGGCGATTTGCAGAACGAGTTGGCGCGTCA
GGTGGAAAAGCTGGAAAAACAAGCGGAAACCGCCGAACGCTACAAATCCCTGACCGCGCA
GCTGAATCAGCAACAGGATTTGCTCGATTACGCCCAATGGCGGCAATCGCTTGCCGCCGC
CGATAAGGCGACCGCGCAGCATCAATCTTTGCAGGCGCAGCAGGACGAAACCGCCGCGCA
GGTTCAGGCGTTAAACGACGAAGTACACGCCTTGCAGACTGCCGAACAGTCGCAGCAGCA
GGCAGTGCATGAATTGAGCAACAAGCGCGGCGTGTTGCGCGAGCAGATTGCCCGTTTGGA
AGAACAAATCCGCCATCAGCAAAACCTGCACCAACGCATCGAACGCGACAAGCAGGCAGC
GCAGGCGCAGTTACAACGCATTCATCAAGAGCAGCAGCAAATCCGCGTGCAGCTTGAAGA
AAACGAGTTGCAGGTCGAAGAAAAACAAACCGAGCTGGCGGAATGGGCGATGCAGGTTGC
CGAACACGAGGAGCGTCTGCCCGAATTGGAAGAAGCCCAAGCCACGCTCAACGCCGCCTT
CCAAACCCAGCAGGACGAGGCAAACCGCATCCGCCGCGAACTGGCGTTGAAGCAGCAGCA
GCTTGCCCATGCCGAACAAACGATTGCCAAGCACGAAGAGCGCAAAGGTCGTCTGAAACA
GGAAAACCAAGCCTTAAACCTGCCCGACGAAGCCGAAACCGCCGCCGCGCAGGAAGCAGC
CGCCTTGTTGCAAAGTCAGCAAGAGCATTACGAAGAACAAATCATTGCCGCCGAAGAAGC
CTTACACGCCGCCCGCGAGGCGTTTCAGACGGCCTCAAACCGCTTCCAAAGCCTGAAGCA
GCAACACATCACCTTGCAGGCGCAGCAGCAGGCGTTGTCGCAAATCCTGTCGCAACAGCA
GGAAGCCGCCGATTTCTGGCAGGCAACCGACCACGCCGCCGCGCCGCAACTGTGGCAACA
CATCACCGCGCCCGCCGAGTGGCAGCACGCCTTGTCCGTCATTCTTGCCGAACGCCTGCA
CGCCCGCGCCGTGCCGCAAGGTTTCGTGCCGCCCGAGCCTTTGCCGCAGGGGCAGGCGGC
ATGGCTTTCAGACGACCTCTCAGGCGGCATCAAAAAATCCCTGCCCGTACAGGCATTGCT
GAACCAAATCCAAGCGCAGCCGCCGTTTCAGACGGCATTGCACTACTGGCTCGACGGCGT
ATTGTGCGCGCCCGATTTGAGCTATGCCCTCGCGCATCAAAACGATTTGGGCGCACACCA
AATCTGGCTCACGCCCGAAGGTCATCAGGTCGATAAAGTCAGCGTCCTGCTCTATGCCAA
ACCCGCGCAGGAAAGCCTGATTGCCCAAAAAGCGCGCCTCGACGGCATCGCGTCCGAACT
GGAAAACCTCGCCCCCGAACTTTCCGCCGCCGAAGCCGCGTTCAAACAGGCGGAAGCTGC
CGTGCGCTCGTCTGAAGTGCAACATAAAAACCTGATGCAGCAGCAACAGCAGCACACGCG
CCAATACAGTCAAGCACAGCAACGCGCCGCCGAACTCTTAGCGCGTACCAACCAAGGGCA
AATCCGCCGCGAACACATCGAGCGCGAACTGGCGCAGTTGGCGGAAGAACAGACCGTGTT
GCAACACACGTCCGACGGGCTTTCAGACGACATCGTTACCTTGCAGGAAGCCGCCGCCGA
ACTCGAACACCAGCAGCAAACCACCGCGCACAGCCGCCAAGAGCAGCAAGGCCGTCTGAA
ACAGGCGCAGCTTGCCCTGTTGGAAGCCAACCGCCAATACGGGCTTGCCGAAGTCGCCGT
CCACAAACTCAACCAGCAAAAACAAAACTACCGGCAGCAAATCGCCCAGCTTGAACAGCA
AACCCTCGACTGGCAGGAACGCCAGCAAGAGCTTGCCCTCGCCTATGAAACCGAGTTCCA
AAACGACGAGCAGCACATCAAGCTTGAAGAATTAAGCGAAGCCGTACAGACCTTGGACGA
AGAATATATTGTTGTGCAAGAGAAACTCGCGCAGATTCAGGAACAGGGCAGGGAGCAATA
CGCTAAAGTGCAAACCCTGCAAACCAAGCTGCCGCAGCTTCAGGCCGCCACCCAAACCGC
CTTGTTGCAGCAGCAGGAAGCCCTGATCAACGCCAAACGCTACCATCAAAACCTGACCGA
ACGCGCCGCCGATTTGGACGCGCTCGAAGCGTTGGCGAAAGAATCGCCGAAAGTATTGAA
CAGCAGCATCGGCAGCCTTTCGCAACAAATCGAAGCACTCGGCGCGGTCAACCTCGCCGC
CCTGCAAGAACTCGAAGAAGCGCGCGAACGCGACGGCTACTACCGCAGCCAAAGCGAAGA
CGTGCAGGCAGCCATCACCCTTTTGGAAGAAGCCATCGCCCAAATCGACGACAAAACCAA
AGCGCGTTTCAAAGAAACCTTCGATGCCGTCAACAGCAAAGTCCAAACCTTCTTCCCGAC
CCTGTTCGGCGGCGGCGAAGCCACTCTCAAAATGATAGGCGACGACCTACTGACCGCCGG
TGTGTCCATTATGGCGCGTCCGCCCGGCAAGAAAAACAGCACCATCCACCTCCTCTCCGG
CGGCGAAAAAGCCCTCACCGCCATGAGCCTCGTGTTCGCTCTGTTCAGCCTCAACCCCGC
TCCGTTCTGCCTTTTGGACGAAGTCGATGCCCCGCTGGACGACGCCAACACCTCGCGTTT
CTGCAGGCTGGTCAAAGAAATGTCGGCGCAAACCCAGTTCCTCTACATCTCCCACAACCG
CCTGACGATGGAAATGGCGGAGCAGCTGGTCGGCGTAACCATGCAGGAAAAAGGTGTCTC
GCGCGTCGTCGCCGTGGACATCAAACAGGCGTTGGAAATGGCGGAAGCCGTTTGAACGGG
TTGCAGAACGGCTGAATCTTGCCGTTTTTAATGAAGTGTTGCGATATGGGTTTTCAGACG
GTATTTCAAACAGAACAGATTAAAATCAAATCCAAATCCATAAAAAATGCCGTCTGAACA
GCGTTCAGACGGCATTTCGATGTGTACTGCCACGTCAAATCAGTGGTGATGGCCGCAGCC
GCATTCTTTTTTCATATCGATCACCATACGGCCGGTGATTTTGCCTTCGCGCATTTCTTG
GAAAATGGCGGGTGCTTCATCCAAAGCACGCAGTTGGACTTTCGGCACAACCAAACCTTC
CGCGCCGAATTGGAAGGCTTCTTCCAAATCTTTGCGCGTGCCGACCAAAGAGCCGACCAC
TTCGATGCCGTCCAAAACCAAACGCGGGATGGACAAATCCATCGATTCCGGCGGCAGCCC
GATGGCAACCACACGTCCGCCCGCGCGGACGCAATTCACGGCAGAGTTGAATGCGGCAGC
AGATACGGCGGTTACGACCGCAGCGTGTGCGCCGCCGGTTTTTCCTGAATCACTTTGGC

## Appendix A

AGCGTCTTCTTTGGCGGCGTTGACAACCAAATCCGCGCCGGTTTCTTTGGCAAACGCCAG
TTTGTCGTCGTTGATGTCGATGGCGACAACGTGCGCGCCGAATACTTTTTTCGCGTATTG
GACCCCCAAGTTGCCCAAACCGCCCGCGCCGTAGATGGCAATCCACTGTCCCGGACGAAC
GCCGGAAACTTTAATGGCTTTATAAGTGGTTACACCGGCACAAGTAATGCTGGAAGCTTG
CGCAGGATCCAAACCTTCAGGGACTTTGACCGCGTAATCGGCACTCACGATACAGTGGGT
CGCCATACCGCCGTCGGCGGTGTAGCCCGCGTTCAATACGGAACGGCACAGGGTTTCGCG
GCCGGTATTGCAGTATTCGCAAGAGCCGCAGCTTTGGAACAGCCAAGCGATGCTGACGCG
GTCGCCGACTTTCAGATTTTTCACACCGTCGGCAACTTCTTTAACCAAACCGATGCCTTC
GTGTCCCAACACGCGGCCCGGTTTTTCGCCGTAGTCGCCTGCCGCAACGTGCAGGTCGGT
GTGGCACACGCCGCAATATTCGACTTCGACCAATGCCTCGCCGTATTCCAACGGGCGAAC
CTCGCGTTCGATTACTTCCACATCGCCCGCTACATTTTTATTCACAACAACTGCCTGCAT
TTTCATGATTGCGCTCCTTAGTTACGGCAAAAAAACCTGTAAACGGAATGTTGTCCGATA
TAAGGGTAAGCATACGCTTCCGCATCTCACAGGTCAAGTGGTATGTTGTTGAAAAATATA
GATTATATGTTATATTATAACATCTTGGAAAGGCACGGCATCGGGGCGGTTGCCGGATGA
GGGGCGGCAGGTTTCAAGTTTGAAAAACCGGACGGCAAACCCGTAAAGATACCGTCTGAA
GCTGTGTCCGGACGGCATCTTTACGGGTTTGCGGGCTTCGGCGGAGGATTAGTCGAAGCC
GGGGCAGGATTGGTTTGTACCGGAAGCGGCAATGGTACCGCCGTCGTTGAGCGTAACGAC
GCAGGTTTCGCCGTCGTTGGTGGTGGGATTGGGGTCGGCCTGAAGGGTAAAGTGGTCGGG
GCTGACTTCGCTTAAAGTGATATCGAAATATTCGTTTTGTTTCAGTTTGTTTTTGTCGTA
GGTTTTAAACGTCCCTTTTTGGCGGTAGTAACGTTCCATGGTCTGCGCGTTGTGCAGCAG
GGTCGTCCTGACTTCCGACAGGCGGACGCGCCGGATGTAGGTTTTATAGGAAGGGTAGGT
GATGAGCGTCAGGATGCCGAGGATGGCGACGGCAATCATCAGCTCGAGCAGCGTAAAGCC
TTTTTGAACGTTTTTCATAGCAATGTGTTTCCATTTGTTTGTCGGTCGACGGCATTATAA
CGCCGTATCGGAAATGGCGGAATATGTAAACGGATTGAAATTTTCGGGAAAGCAGATTGT
ATAAGCCATTTAAAACAAATGGTTATTTTTATTGTCGGCAGTTTGCCGCCTTGGATGGGG
CAGGGACTTGCGGTAGAATCCGCTTCCGATTTATGGGATTGACGCATACAGAGAATTGAA
AACATGGCAAAAATGATGAAATGGGCGGCTGTTGCGGCGGTCGCGGCGGCAGCGGTTTGG
GGCGGATGGTCTTATCTGAAGCCCGAGCCGCAGGCTGCTTATATTACGGAAACGGTCAGG
CGCGGCGACATCAGCCGGACGGTTTCTGCAACAGGGGAGATTTCGCCGTCCAACCTGGTA
TCGGTCGGCGCGCAGGCATCGGGGCAGATTAAGATACTTTATGTCAAACTCGGGCAACAG
GTTAAAAAGGGCGATTTGATTGCGGAAATCAATTCGACCTCGCAGACCAATACGCTCAAT
ACGGAAAAATCCAAGTTGGAAACGTATCAGGCGAAGCTGGTGTCGGCACAGATTGCATTG
GGCAGCGCGGAGAAGAAATATAAGCGTCAGGCGGCGTTATGGAAGGAAAACGCGACTTCC
AAAGAGGATTTGGAAAGCGCGCAGGATGCGTTTGCCGCCGCCAAAGCCAATGTTGCCGAG
CTGAAGGCTTTAATCAGACAGAGCAAAATTTCCATCAATACCGCCGAGTCGGAATTGGGC
TACACGCGCATTACCGCAACGATGGACGGCACGGTGGTGGCGATTCTCGTGGAAGAGGGG
CAGACTGTGAACGCGGCGCAGTCTACGCCGACGATTGTCCAATTGGCGAATCTGGATATG
ATGTTGAACAAAATGCAGATTGCCGAGGGCGATATTACCAAGGTGAAGGCGGGGCAGGAT
ATTTCGTTTACGATTTTGTCCGAACCGGATACGCCGATTAAGGCGAAGCTCGACAGCGTC
GACCCCGGGCTGACCACGATGTCGTCGGGCGGTTACAACAGCAGTACGGATACGGCTTCC
AATGCGGTCTACTATTATGCCCGTTCGTTTGTGCCGAATCCGGACGGCAAACTCGCCACG
GGGATGACGACGCAGAATACGGTTGAAATCGACGGCGTGAAAAATGTGCTGATTATTCCG
TCGCTGACCGTGAAAAATCGCGGCGGCAAGGCGTTTGTGCGCGTGTTGGGTGCGGACGGC
AAGGCGGCGGAACGCGAAATCCGGACCGGTATGAGAGACAGTATGAATACCGAAGTAAAA
AGCGGGTTGAAAGAGGGGGACAAAGTGGTCATCTCCGAAATAACCGCCGCCGAGCAACAG
GAAAGCGGCGAACGCGCCCTAGGCGGCCCGCCGCGCCGATAAACGAATATGCCGTCTGAA
CACGGAAACGGTTTCAGACGGCATTTGTTATTGATTTACGGAATATTATGAGCTTGATCG
AATGTAAAAACATCAACCGCTATTTCGGCAGCGGCGAGAACCGCGTCCATATTTTGAAAG
ACATCAGCCTGTCGATAGAGAAGGGCGATTTTGTCGCCATCATCGGGCAGTCCGGTTCGG
GCAAGTCCACGCTGATGAACATACTCGGCTGTTTGGATACCGCCGGTTCCGGTTCGTACC
GAATCGACGGCATCGAAACTGCCAAAATGCAGCCTGACGAGCTGGCGGCATTGCGCCGCG
AACGCTTCGGTTTCATCTTCCAACGCTACAACCTCTTAAGCTCGCTGACCGCAAGGGACA
ACGTCGCGCTGCCAGCCGTCTATATGGGCGCGGGCGGCAAAGAGCGTTCCGCGCGGGCGG
ACAAACTCTTGCAGGATTTGGGTTTGGCAAGCAAAGAGGGCAACAAGCCCGGCGAACTCT
CGGGCGGACAGCAGCAGCGCGTCTCCATCGCCCGCGCCCTGATGAACGGCGGAGAAATCA
TCTTCGCCGACGAGCCGACCGGCGCGCTCGATACCGCCAGCGGCAAAAACGTGATGGAAA
TCATCCGCAGGCTGCACGAAGCCGGGCATACCGTCATTATGGTCACGCACGACCCCGACA
TCGCCGCCAATGCCAACCGCGTCATCGAAATCCGGGACGGCGAAATCATTTCCGACACCT
CGAAAAATCCCGAAATCCCCGCAAGCAATGTCGGGAGGATTCGGGAAAAAGCTTCGTGGT
CGTTTTATTACGACCAGTTTGTCGAAGCCTTCAGAATGTCGGTGCAAGCAGTATTGGCGC
ACAAAATGCGTTCGCTTCTGACGATGCTCGGCATCATCATCGGTATCGCGTCGGTGGTTT
CCGTCGTCGCATTGGGCAATGGTTCGCAGAAAAAAATCCTTGAAGACATCAGTTCGATAG
GGACGAACACCATCAGCATCTTCCCGGGGCGCGGCTTCGGCGACAGGCGCAGCGGCAGGA
TTAAAACCCTGACCATAGACGACGCAAAAATCATCGCCAAACAAAGCTACGTTGCTTCCG
CCACGCCCATGACTTCGAGCGGCGGCACGCTGACTTACCGCAACACCGACCTGACCGCCT
CGCTTTACGGCGTGGGCGAACAATATTTCGACGTGCGCGGACTGAAGCTGGAAACGGGGC
GGCTGTTTGACGAAAACGATGTGAAAGAAGACGCGCAGGTCGTCGTCATCGACCAAAATG
TCAAAGACAAACTCTTTGCGGACTCGGATCCGTTGGGTAAAACCATTTTGTTCAGGAAAC
GCCCCTTGACCGTCATCGGCGTGATGAAAAAAGACGAAAACGCTTTCGGCAATTCCGACG
TGCTGATGCTTTGGTCGCCCTATACGACGGTGATGCACCAAATCACAGGCGAGAGCCACA
CCAACTCCATCACCGTCAAAATCAAAGACAATGCCAATACCCAGGTTGCCGAAAAAGGGC
TGACCGATCTGCTCAAAGCGCGGCACGGCACGGAAGATTTCTTCATGAACAACAGCGACA
GCATCAGGCAGATAGTCGAAAGCACCACCGGTACGATGAAGCTGCTGATTTCCTCCATCG
CCCTGATTTCATTGGTAGTCGGCGGCATCGGCGTGATGAACATCATGCTGGTGTCCGTTA
CCGAGCGCACCAAAGAAATCGGCATACGGATGGCAATCGGCGCGCGGCGCGGCAATATTT

## Appendix A

TGCAGCAGTTTTTGATTGAGGCGGTGTTAATCTGCGTCATCGGCGGTTTGGTCGGCGTGG
GTTTGTCCGCCGCCGTCAGCCTCGTGTTCAATCATTTTGTAACCGACTTCCCGATGGACA
TTTCCGCCATGTCCGTCATCGGCGCGGTCGCCTGTTCGACCGGAATCGGCATCGCGTTCG
GCTTTATGCCTGCCAATAAAGCAGCCAAACTCAATCCGATAGACGCATTGGCACAGGATT
GAGGTTGGACAAAGATGCCGTCTGAAGCTGCAGGACCGGTCATTTTGGAGCAGAAACTTA
TTGGATAAAAACGGTTTCTTAGATTCTACGTTCCAGATTCCCACTTGCGTGGGAATGACG
GCGGCGGGGGTTCGATGATTGCACACAACGCTCGAGTCCCGTCATTCCCGTAAAGACGGG
AATTCGGTTCGTTCGGCTTTGCTTGTTTCGGATAAATCACGGTAACTCAATATTCCAGAT
TCCCGCCCGCGTGGGAACGGCGGCGGGGCTTCGTATTGTTCAATTTATTATTTTCAATCA
TTCAATGGGTTAGGATGTGTTTGTTGGCTTGCTAACTTTCAGGGCGGATTGGTTTTCAGG
CGGCATTTCCCGCAAAAAAGGCTTGGAATTTTCCAAGCCTTTTTTGCGGATGGATTATTG
ATTTTTGCGGATGATTTCCTCCAGTTGGGGCATCGGGCTGTAGCCGCTTTGGCTGCGCCC
GTTGGGGAAGACGAGGGTCGGCGTGCCGTTGAAGCCGAATTGTTCGCCCAAGGAAGTGGT
TTCCGCGACGGGATTGTCGCAGATGCTGCCGCCGACCGGGAATTTGCCTTTACGCATCCA
ATCCGTCCACGCTTTGGCGCGGTCGGGCTGACACCATAAGATTTGCGCCTTGCGCGCGGC
ATCGGGGTGCAGGCCGGCAATGGGCATCATAAAGCTGTAAACCGTCACGTCGGTCATTTT
TTCAAACTCGTGTTCCAAGCGTTTGCAGAACGGACAATCGGGGTCGGAGAAGACGGCGAC
TTTCAGCTTGCCGTTGCCGCGCACTTCTTTGATGGCTTTGTCCAAAGGCAGGGAGGCGAA
GTCGATTTTGTTCAAATCGGCGGCGCGTTCTTCGGTCAGGTTTTTGCGCGTGTCGATGTT
GATGAGTTCGCCGACGAACATATAGCCGCCTTCGGCATCGGTGTAGATAATCTGCCTGCC
GCTGACGACGACTTCGTAAATGCCTTTGACCGGTGTTTCGCTGACGCTCAACACTTTCAA
ATCTTGGGCGGAATAGGTTTTTTCCAAACGCGCTTTCAAAGAGGCGGCAACGGATTTGCC
GGCGGACTCGGCTTTGACGGCGGGTTCGGCGTTGGCATTGGAAACGGGCGTTTGCCCGCA
AGCCAGCAGCGGGAGGACGGTAAAGGGGGTCAAGATTTTGATTAACTTGGTTTTCATATA
AAGATGATTGCGCGTGTTGGAAAAGCGGAATTGTATCAAATCTCTGTTGCGCCTGCATTG
CGCCTAGGCTCAATTTATCGTCTGAAAATAGCTTCCGGCTGTTAAAATACGCAAAAAATG
ATTTGCTTGTTTGTATGATTTACCACCGCATCGCCGTAAACGTGCCGCTTTCAGACGGCC
TTTTGACTTATTCCCATTCCGATCCGCTTCCTCCGGGAACGCGGGTGCTTGTGCCTTTCC
GCAATAAAACCGTGGTCGGGATGGTGTGGGAAACGGATATTGCGCCCGATATGGATATGG
CGCGGATTTTGAGTGTTCAGACGGCCTTTGTGGAAGAAAAGCCGTTGCCTGAAAGCTGGC
GTGATTTGTTGGCATTTACGTCGCGTTATTACCACTATCCGACTGGGCAGGCGGTGTTTG
CCGCGCTGCCGCAGGGTTTGAAGGAAACGCGCGCGGTGGAAATGCCGCAGCCGCCGTTGT
TTTATGCTTTGAACGAAGCGGGCAGGGCGCAAACGCCGCCACCAGCTCGGTTCAACAAAA
AAGCGGCTTTGTGGACGCACTGCTTTCGGGCGGAATGACGATGGCAGCGTTGAAGCAGG
TAAACGCGCAGGCGGCGAAATTGATTGAAGATTGGGCGGAGCAGGGTTGGATTGAAACAA
CGGAAGCGGCGAAACCTGTATTGAGGTCGTACCACGGGCAGGCTTCGCACTCTGAATTTG
TGTTGAATGCCGACCAGCAACAGGCTTCCGATGAAATTCAGACGGCCTTCGGCAGCTTCC
AGCCGTTTTTGCTGTACGGCATCACCGGCAGCGGCAAGACCGAGGTGTATTTCGATGCGA
TGGCGAAAGTGTTGGCGCAGGGGCGGCAGGTGTTGTTTCTGTTGCCCGAAATCAACCTCA
CGCCGCAGCTTTTGAAGCGGGTGGAAAACCGTTTTGCCGACGTGCCGACCGCCGTGTTGC
ACAGTCAGATGGCGGCAGGCAAGCGCACGCAGGATTATTTGCGCGCGATGTTGGGGCAGG
CGAAATTGGTCATCGGCACGCGGCTGGCGGTGTTCACGCCGATGGATGATGTCGGGCTGA
TTGTGGTCGATGAGGAACACGACGGCTCGTTCAAACAGGACAACGAATTGCGCTACCACG
CCCGCGATTTGGCGGTGTGGCGGGCGAAGCAGGGCGGCTGCCCGATCATATTGGGCAGTG
CCACCCCCAGCTTGGAGAGCTGGCACAAGGCGCAAAGCGGCGCGTACCGCCTGCTGCAAC
TGACCGAACGCGCCCATACCGCCGCGCAACTGCCGCAAGTGGACATCCTCAACGTAGGCC
GTCTGAAACTTGACAACGGCTTCTCGCCGCAAGCCTTGCAGCTTTTGAAACAGAACTTTG
AAGCAGGTGGCATGTCGTTGGTGTACCTCAACCGTCGCGGCTTCGCGCCCGCGCTGTTTT
GCGGCGACTGCGGTTATACCTTCGGCTGCCCGAACTGCTCCGCCAAAATGGTGCTGCACC
AACGCGCCCGCCAACTGCGCTGCCACCACTGCGACCACCGCGAACCCATCCCGTACAAAT
GCCCCGACTGCGGCAACCAAGACCTGACCGCCGTCGGCCACGGCACGCAGCGCGTCGAAG
AAACCCTGCGCACCTTCCTGCCCAAGGCAGCCGTCGTCCGTGTTGACAGGGACAGCACCG
CGCACAAAAACGACTGGGCGGATTTGTACCGCCGCATCGCCGACAACAAAATCGACATTT
TGGTCGGCACGCAGATGCTCGCCAAAGGGCATGATTTCGCGCGGCTCAACCTCGTTATCG
TGTTGAACGCTGACGGCAGCCTGTACAGCGCGGACTTTCGCGCCCCGGAAAGGCTGTTCG
CCGAGCTGATGCAGGTGTCCGGCAGGGCGGGGCGCGCCGACAAACCCGGCAAGGTGTTGA
TACAGACCCAACTGCCCGAACATCCCGTCTTCGCCGCCGTCAAAGCGCAGGACTACGCCG
TGTTTGCCGAAAACGAATTGAACGAGCGGCAAATGTTCGCCATGCCGCCCTTCGGTTTCC
AGACCGCCGTCCGCGCCGACGCGCCGCGCGTTGCCGATGCGATGGAGTTTCTCAATGCCG
CCAAAGAAACCCTCGCCCCGCTTTTGCCCGAAAGCGTTTCACAGTTCGGCGCCGCCCCGA
TGCTGATGGTGCGCCTCGCCGAACGCGAACGCGCGCAAATCTTCCTCGAATCTCCGTCCC
GACAGGATTTGCACCGTGCCGTGAGTTTGTGGGCGCAGGTGTTGCAGCAAAACCGCGACG
GCAAAATCAGATGGTCGGTGGATGTCGATCCGCAGGAGGCTTGATTATTGGCAATCCGAT
GCCGTCTGAAAACCGTTTCAGACGGCATTTTTATTCCGGATCGTCTGTAAACGCATTCGC
CCGAAATATCGGTATAAACGTGAAAAGATACAGTACGAATACGGCGGCGGTCAGAATCGC
AGGAACGGTAATGAAAAATATCGGGTTCACGTTCATCAAGAAAGCGCGCGAGACGGCGGC
GGCGAAAAGGATGGGGACGGCAATGCGGCAGAGTTTGGGGTAGTCGAGTTTGGTAAAGCC
GCTGTGCCACAGTCCGGCGGTCAGCCACACCATCATCACGCCGCCCATCATGCCGCCGAG
GGTAATCAGGTGCAGGGGCGCGGAGGCGGGCAGGTTTTGTAATTTCGCCGCGCCTGTCCA
CAAATAGCCTGCGGCGGCAAAGAGTTGGAGCAGGTAATAAGTGCGGACGTAGTGTTTACG
TAAGAGTTCGTGATGGTGAAGCTCACGCAGCTTGGCGAGCAGGATGAAGCCGACGGCGAG
CGCGGTAAAACCGGCGGTTTGCGCGGGCAGCCAAAGTTCGGCGGCGGCGTGCAAGAGCAG
GAAAGTAATGGCGATGTTTTTATAAACGATATTTGGAATAAAAACAGGGTCTTTCAGACG
GCATTCTTTCAGGGCTTCCGCGCCCAAAAGAATACTGACGCGCACGGATACGAACATCAC
CGCCGCCATATTTAGATGCACTTGCGCGCGCAACAGGTTCAAATCGCCGCTGACGGCATA

## Appendix A

TGCCGTCTGAAAAACAGTGAACGCGGCAAGTAACATTAGCAGGGCGAAGTTGTCGGTGTT
TCGGTCTAGCCAAATCAGCCGGGCGCAGAACAGCAGCAACACCAGCCAATAGGCGGCGAC
GAAAAACGAGGCAGTTTGCGGCGAAAAGGGCAGTATAGCGGATGCGGCGAGCAATAATGC
CGCCATCAAAGTCGCGACAGGTTTCAGGTTACCCGAAAAACCCGTCCAGTCCAACAAAGC
CGCAGTCAAAAAACCGCCGTATGCCGCCGGCAGCATAAGTTCCAAGAAAATTTGGCGGTG
CAGGACGATGGCACCGGGGTTGATGAAAAACACCAGCGCACCGAGTATGGCAAGCACCGC
CGCGCCGACGAAAAACGGCCGCATAGCAACTGTATTTTTCACCCCGTCGGGCAAAAATAC
CAAAACTCAAATCAAGCCGTCCGGATACCGTTTTCGGCGGTATCGTTTTCGGCAAAATAA
TCACGCATCCGGGCATTCGATATCGTCAGCAGTTTGCGCATACATGCCGTAACGGCAACC
TTATACGGCTTACCCTTGGACAGCAGGCGTTGGTAGAAATCCCGAATAAGCGGTTCAAAA
CGTGTCGCTGCCACGGTAGCCATATACAGTGCCTTACGCACCGCAGACCTTCCGCCAAAG
CAGCGGCTTTTGAATTTGGTTTCCTCGCTCTCCCTCGGGTGCGGGGCAATGCCGGCCAAA
CTCGCTATCCGTTTGTGCGACAGCCGCCCCAATTCGGGCAGCATCGCCATCAGCGTAGCC
GTCGTTATCGAACCGATGCCTTTGATTTGCTCCGCCACTTGGGCTTTGCCGTCAAAATGC
GTGTGGGTGTGGTCGTCGATTTGTTTGTCCAATTCGTCAATCAGCCGGTCAAAATGGGCA
ATCAGTTGTTTGACGCTTCCGACTTGCGTTTCATGAACCTAATGCAGACGGTTTTTCTCG
GCAGTCCGCATATCCACCAGTTGGTTGCGGCGGTTAACCAAGGCTTCCAACACTTCTTCC
ACTTCGGTGGGCGGGTGGTAGGGCATGGTTTGCGAACCTTCTTTCTGCGTCATCATCTGT
GCGAAGAAGGCGAGCATTTTGGCATCTTTGGCGTCGGTTTTGGTCAGCGGCTGCGATTGG
GCAAACTGATGCGTCTGACGCGGGTTGGCGATAATCACGGCTATGCCTGCTCGGCGGATG
GCTTTGGCGGCGGGGATTTCGAGACCGCCGGTACTTTCCGTCACGACGAGGGCGACCTTG
TGTTTTTTAAGGTATTCGATAGTATGGGCGATACCTTTGGGGTTGTTGGTTTCGGTTTTG
GTTTTAGACAAAGACGAAACGGCGATGACGAAGTTTCGTTTGGCGATGTCGATATAGTGA
ATTAACAAAAATCAGGACAAGGCGGCGAGCCGCAGACAGTACGGATAGTACGGAACCGAC
TCACTTGGTGCTTCAGCACCTTAGAGAATCGTTCTCTTTGAGCTAAGGCGAGGCAACGAC
GTACTGGTTTTTGTTAATCCACTATAACAGCAACCCTGTCGCCGTCATTCCCGCAAAAGC
GGGAATCCAGTCCGTTCAGTTTCGGTCATTTCCGATAAATTCCTGTTGCTTTTCATTTCT
AGATTCCCACTTTCGTGGGAATGACGGCGGAAGGTTTTTGTTTTTTTCCGATAAATTCTT
GAGGCATTGAAATTCCAGATTCCCGCCTGCGCGGGAATGACGATTCATAAGTTTCCCGAA
ATTCCAACATAACCGAAACCTGACAGTAACCGTAGCAACTGAACCGTCATTCCCACGAAA
GTGGGAATCTAGAATCTCAGACTTTCAGATAATCTTTGAATATTGCCGCTGCCTTAAGGT
CTGGATTCCCGCTTGCGCGGGAATGACGAATCCATCCGCACGGAAACCTGCACCACGTCA
TTCCTACGAACCTACATCCCGTCATTCCCACAAGGACAGAAAACCAAAATCAGAAACCTA
AAATCCCGTCATTCCCACGAAAGTGGGAATCTAGAAATGAAAAGCAACAAGCATTTATCG
GAAATAACTGAAACCGAACAGACTAGATTCCCGCCTGCGCGGGAATGACGAATCCATCCG
CACGGAAACCTGCACCACGTCATTCCTACGAACCTATATCCCGTCATTCCCACAAGGACA
GAAAACCAAAATCAGAAACCTAAAATTCGTCATTCCCGCGAAAGTGTGAATCTAGAAATG
AAAAGCAACAGGCATTTATCGAAAATAACTGAAACCGAACAGACTAGATTCCCGCCTGCG
CGGGAATGACGGCTGCAGATGCCCAACGGTCTTTATAGTGGATTAACAAAAATCAGGACA
AGGCGACGAAGCCGCAGACAGTACAGATAGTACGGAACCGATTCACTTGGTGCTTCAGCA
CCTTAGAGAATCGTTCTCTTTGAGCTAAGGCGAGGCAACGCTGTACTGGTTTAAATTTAA
TCCACTATATAAAAAATTTCCAGAGAACCGATACAACAGTTGGAACTTGGGTTTGGGAAT
ATTACGGTAGATGAACTTGGAACCTCTGTTATGCTATGGTCTTTTATCTCAATTGAAAAA
AGCGCGAATCAAACGGTTCGCGCTTTTTTCAGACGGTATTAATTATTTTTTGTCGTCTTT
TACTTCTTCAAAGTCGGCATCTACGACATCATCGTCTTTCTTTGCAGAAGCATTGGCTTG
TTCGCTTTCGCCTGCTTGGGCTTCAGCTTGTGCTTGAGCGTAAACCATTTCCCCCAGTTT
TTGGCTGGCTGCGCCCAGCGCCTCGGTTTTGGCATCGATAGCGGCTTTGTCGTCGCCTTT
AACTGCTTCTTCGGCTTCTTTCAGCGCGGCTTCGATTTTTTCTTTCTCGGCTGCGTCGAG
TTTGTCGCCGTAGTCGGCCAAAGATTTTTTCACAGAGTGAATCAGGGCTTCGGCTTGGTT
GCGGGAAGCGACCAATTCAGTCAGTTTTTTATCTTCCTCGGCATTGGCTTCGGCATCTTT
CACCATGCGTTCGATTTCTTCTTCGCTCAAACCTGAAGAACCTTGGATGGTGATGTTGGC
TGCTTTACCGGTGCCTTTGTCTTTGGCGGAAACGTGCAGGATGCCGTTGGCGTCGATGTC
GAAGGTTACTTCGATTTGCGGCATACCGCGCGGTGCAGGTGCGATGTCGCCCAAGTTGAA
CTGACCCAAAGATTTGTTGGCAGAAGCGCGTTCGCGTTCGCCTTGCAGTACGTGGATGGT
TACTGCGCTTTGGTTGTCTTCGGCGGTAGAGAACACTTGCGACGCTTTGGTCGGGATGGT
GGTGTTCTTCTGAATCAGTTTGGTCATCACGCCGCCCATGGTTTCGATACCCAAAGACAG
AGGAGTTACGTCCAGTAGCAATACGTCGCTGCGGCCGCCGCTCAATACTTCGCCTTGGAT
CGCTGCGCCTACGGCAACGGCTTCGTCAGGGTTCACGTCTTTGCGCGGTTCTTTGCCGAA
GAAGGCTTTAACGGCTTCTTGTACTTTCGGCATACGGGACTGCCCGCCGACCAAGATTAC
GTCGTCGATGTCGCCGGTGCTCAAGCCGGCATCTTTCAATGCAATTTTGCAAGGTTCGAT
AGAGCGGGTAATCAGGTCTTCAACCAGGCTTTCGAATTTGGCGCGGGTAATTTTCATCGC
CAAGTGTTTCGGGCCGGTTGCGTCCATGGTGATGTACGGCAGGTTAATTTCGGTTTGCTG
GCCGCTGGACAATTCGATTTTGGCTTTTTCGGCAGCTTCTTTCAGGCGTTGTAGAGCCAT
CACGTCTTGTTTCAAATCAATGCCTTGTTCTTTTTTGAACTCGGCGATGATGTGGTCGAT
GAGGCGTTGGTCGAAGTCTTCACCGCCCAAGAAGGTATCGCCGTTGGTTGCCAATACTTC
GAATTGTTTGTCGCCGTCGAGGTTGGCGATTTCGATGATGGAAATATCGAAAGTACCGCC
GCCCAAGTCATATACGGCTACTTTGCGGTCTTTGTTGTCGCCTTTGTCCATACCGAATGC
CAAAGCGGCTGCGGTCGGCTCGTTGATGATGCGTTTCACGTCCAAACCGGCGATACGGCC
TGCGTCTTTGGTGGCTTGACGTTGGCTGTCGTTGAAGTAGGCAGGGACGGTAATCACGGC
TTCGGTTACTTTTTCGCCCAAGTAAGCTTCGGCGGCTTCTTTCATTTTACGCAGGACTTC
TGCGGAAATTTGAGGAGGAGACAGCTCTTTGCCTTGTGCTTTTACCCATGCGTCGCCGTT
GTTGGCTTTGATGATTTCGAAAGGCATAGATTCGATGTCGCGTTGGACTTCTTTGTCTTC
AAATTTGTGGCCGATCAAACGTTTGGCGGCGTAAATAGTGTTTTTGGCGTTGGTTACCGC
TTGGCGTTTGGCAGGCGCACCGACGAGGATTTCGCCGCCGTCCAAATAAGCGATAACGGA
CGGCGTGGTGCGTGCGCCTTCTGCGTTTTCGATCACTTTGGTTTGACCGTTTTCGGAAAT

## Appendix A

GGCCAAACAAGAGTTGGTTGTACCTAAGTCGATACCGATTACTTTTGCCATGTGGATAAT
CCTATTTGATTTTGCTTATTTTGAGAAATATGTTGGAACATTTTGTCCCGATGGGCTGTA
AATAGGGCGGGCGGCGGGCTGTTTCAAGCTACAGCATGGCTATAAGTATATAACTTTATG
AATATATTGGTTTTATATTTGATTTAATACATTTGGCTCCAATGCATTCAAGCATAATGT
TTCAAATGGCAGGCAGGTTTATTCATAGACGATGCCGGCGAGCATTTCCTGTTCGTTCAA
GTTGCCGTACTCTTTTTCCCAGTCGTGTGAAGACTCGATGATGTCGCATTCTTTGGAAAG
GGAGACTTGTTCTGCATCCATATCTTTGGCGTTCAGTATGTTGAATTGTTCGCACAGGGA
TGCGGATAAAGTGATGTCGGGCTGTTTGGCTTCAGAACGGTTTTCTTGGAAGGCAAAGCA
GAATGCGGTAAATGCCGCAGTATAGATAAGATATTTGCCGGTTTTCTTCATTTTTCTATC
CTTTTTCTGTCAATTCAGGATTAAACCTATGGAAAAATCTGAAAAATTATGTATTAAGTA
AGAAAAATCATAATTTAAATTTAGTTTATCATAATTGTTCCGTTTTTTGGATAGCTAAGG
TAAAATATATTTCATGTTTACTTTAGATGATTGAATGAAGGGGAGTGGAAGGATATTTAT
GGAAACCTTTAAAGACAGACTGGTTTTTTTATGGAAAAGCGAAGCGAGGCAGGCAAAAAT
CGCATCCGATATTGAAATGACGATTGCGGGCTTCAGCAGGATATGGAATGAAGGCGGTCT
GCCAAAGTCTGAAACATTGAAAAAAATCAAGCAGTTGAAGGGGTGTAGTATCGATTGGCT
GCTGACCGGGGAGGGTAATCCGTTTCCGGATGAAGCCCCAAAAAAATCCCTTGCTTACGA
TACTTTGGGCAATGAAGTCGATACGGACGAGTTTGTCTTCGTGCCGAGATATGATATTCG
GGCGGCTGCGGGATACGGGCAGTTTGTCGATCATGAGGAACCGGTATTTACAATGGCGTT
CAGACGGCATTGGATTGAGAATTATGTTACCCGCGATACGAAAAACCTGTCTGTAATTTC
CGTCAAGGGGGATTCGATGGAGGGGGTTTTGAATGACGGCGATTCGATTTTGGTCAATCA
TGGTGAAAATACGCCGAGGGACGGTCTGTATGTGTTGCGGATTAATGAAAATCTGCTGGT
TAAACGTTTACAGATTGTACCGGGCGGGATTATCAATGTGATTTCTGCAAACGAGGCTTA
TCCTGCTTTTGAAATCAATTTGAACGATTTGACCGATGATGTGGAGATTATCGGGCGTGT
CGAGTGGTTCGGCAGGACGATTTGAGTTTGGGGCTTGAAATTGCAGGCGGTCAAACTTAT
CTATTGGAACAATTCCTTTTTCAAAGGCGAAGCCTGCTTGCCTTTGAAGGGGGTTTGAGA
GAGAATGCAGAAAATATTATATTAAGGAATAACACCATGTCGGATGAAAGCCCTATTATT
TTTACTGACAGCTGCTGTGCCAAAGTTGCCGATTTGATTGCCGAAGAAAACAATCCCGAT
TTGAAATTGCGGGTTTTTGTCAATGGCGGCGGCTGTTCGGGTTTCCAGTACGGATTTACT
TTTGACGAAATCAAAAACGACGACGATTTTGAAATTGAGAAAAACGGTTTGGTCTTTTTG
GTCGATCCGATGAGCTATCAATATCTGGTCGGTGCGGAAATCGACTATACGGAAAGTTTG
CAGGGTTCGCAATTCGTCATCCGCAATCCGAATGCGGAAACAACCTGCGGTTGCGGATCG
TCGTTTTCCGTATGACCGCTTGGTTTGTGTGATGCCGTCTGAACGTTCAGACGGCATTTT
TACTTTTAGAAAATATATTATCGGGATGAATTCACATATAATCCGATTGTTTGAAGATGA
ATCGGGTTTCCCGAAAGGAACGGGCGGAACGGTATCAGGCGTATTTGTTCCCTTATGATT
GAGATGAGTAAAGATTACCGAAACGATTTGTACGATGTATATGTTTCTTACCCGCCCCAA
GTGGATCGCGGGCTTATCCGGGAGTGCCTTAAGGAGAATCTCGGCGAGGAAAAGGCGGAA
GGATTGATCGAATCGCTCGATTCCAAACCTCAAGTGCTGGTTGAGGAAAAATGCACTTGG
GCGAAACGGGAAGAGTTGCATGATTATTTCAGCTATTTGGGTTTGGATATTATTACCCGG
AGATATATGGAGTTGGAAACGGTCGTGCCGCCGGAGGAAGGGGAGGGCGAAGGAGAAGGC
GCGGATGGGGAAATGCCCGAATATCTTGAACTTCACGGCGGGCGGGAAGATGATATTTCC
GCACCTTCGCAACCCGAACCGCCGTCCCGCAATATCAAACTGCTGGTTTTCGGGCTGCTG
ATTGCCTTTTTGGGCTATCTGCTCGGTAAGATTTTTTGATTGTCCGATAAATGCTGTATT
CGGGATTTTATATATGAAATGGTTGAAACGCCTGACGGTTATTGTCGGGACTTTTTACCG
CTATCGGCTGGCAGGTCTGTGTGCTTCGCTGATGGGTAGCGGTTGGATATGCGCTCTGCT
GAAAATGATGCCGCAGTCGTCCAAATTGAAAAACGAACCGCCTGCTGTCCGTCTGCGCCT
TGCCTTGGAAAGCCTGGGGCCGATTTTCATCAAGTTCGGGCAGGTTTTGTCCACACGCCC
CGATTTGATTCCGCACGATTACGCCGTCGAACTGGCAAAGCTGCAAGACAAAGTGCCGCC
TTTTGACGCGCGGCTTTCGCGTGAACAAATCGAAAAATCGTTGGGTCAGTCCATCGAAAA
GCTGTATGCGGAATTTGAAACCGAGCCCATCGCCAGCGCGTCCATCGCCCAAGTACACAA
AGCCCGCCTGCATTCGGGCGAACAAGTGGCGGTTAAAGTTTTGCGCCCCAACCTTTTGCC
CGTGATCGAACAGGATTTGTCGCTGATGCGCTTTGGTGCAGGCTGGGTCGAGCGTCTGTT
TGCCGACGGCAAGCGTCTGAAGCCGCGCGAAGTGGTGGCGGAGTTCGACAAATATCTGCA
CGACGAGTTGGACTTGATGCGCGAAGCCGCCAATGCCAGCCAGCTCGGACGCAATTTCCA
AAACAGCGATATGCTGATTGTGCCGAAGGTGTTTTACGACTACTGCACCAGCGACGTGCT
GACCATCGAATGGATGGACGGCACGCCGGTTTCCGACATCGCCAAACTCAAAGCAGACGG
CATCGATTTGCACAAACTCGCCGATTACGGCGTGGAAATCTTCTTCACGCAAGTCTTCCG
CGACGGCTTTTTCCACGCGGATATGCACCCCGGCAATATTTTGGTTGCCGCCGACAACCG
CTACATCGCCCTCGATTTCGGCATCGTCGGCACGCTGACCGATTACGACAAACGTTATCT
CGCCATCAACTTCCTCGCCTTCTTCAACCGCGATTACCGGCGCGTCGCCACCGCCCACAT
CGAATCGGGCTGGGTGCCCGCCGACACGCGCGCGGAAGAGTTGGAAGCGGCTGTCCGCGC
CGTGTGCGAACCAGTGTTCAACAAACCGATTTCGCAGATTTCCTTCGGCTTGGTGCTGAT
GCGCCTGTTTGAAGTCAGCCGCCGCTTCAATGTCGAAATCCAGCCGCAGCTGGTATTGCT
GCAAAAAACGCTGCTCAACATCGAAGGCTTGGGACGGCAGCTTGATCCCGATTTGGACTT
GTGGAAAACCGCCAAACCGTTTTTGGTGAAATGGATGAACGGGCAGGTCGGCCCTAAAGC
CCTTTGGCGCAACCTCAAAAACGAAGCCCCCGACTGGGCGCAAATCATCCCTTCATTGCC
GCGCAAAATCAGTGCGTTGATTGATGAAAACCGCCAGCAGGAAATGCGTGATGCCTATAT
CCATTTGGTCAAAGTGCAGCAGCGGCAAAGCCTGTGGCTGGCTGTGATTGCGGTTGTTTT
GCTGCTGATTTTGCTTTTGAAATAGGCTTTGTCCGAATCATCGCCCGACTCCGCCCGTTT
ATAAGGAAATCGGTTATAGTGGATTAACAAAAACCAGTACGGCGTTGTCTCGCCTTAGCT
CAAAGAGAACGATTCTCTAAGGTGCTGAAGCACCAAGTGAATCGATTCCGTACTATCCGT
ACTGTCTGCGGCTTCGTCGCCTTGTCCTGATTTTTGTTAATCCACTATATTTCCGGTTGC
GTGGGAATCGGGTGTATTGAATAAAAGGCATTTTGTCCGACTGGCAAGTGCCGACATCGG
CGGCATATCAAGGCGCAGGCTTGAAGCGGGCAATGTCGTCTGAAGCCCGTTTGGCGTTTC
AGACGGCATTGGTGCGGATATTCAAATCATAAAGTCGATTTCGGTAAACTGGATATTTTG
ATCCATATCCGCCGACGGTGTTTTGAGCGATCGCGCCACGGGTTTGGCGGGTACGCCGAC

## Appendix A

AACCGTGATGGACGGCGGCACGTCTGAAACCACGACGCTGCCCGCCCCGATTTTGGCATT
GCTGCCGATGCGGATATTGCCCAATATCGAGGCGTTTGCGCCGATCATCACGCCGTCGCC
GATTTTAGGGTGGCGGTCGCCGCCTTCTTTGCCCGAACCGCCGAGCGTTACGCCGTGCAA
AATCGAAATATTGTTGCCCAACACGGCGGTTTCGCCGGCAACAAAGCCGGTGGCGTGGTC
GAGCATCAGCCCGTATCCGAAACGGGCGGCGGGATGGATGTCCACGCCGAATACTTCGGA
CATACGGTTTTGCAGGAAATACGCCAGCGTTTTGCGCCCGTCGAGATACAGCCGATGGTT
GATGCGGTGTGCCTGAATCGCGTGGAAGCCTTTGAAATATAAAAGCGGCAGCGAATATTC
GTCGCAGGCGGGATCGCGTTCGTAGATGGCTTTTAAGTCTGCTTCGACGCATTTGCCGAT
TTGGGTGTCGCTGCCCAACGCCTGCTGGTAGATTTCAAACAGCGCGCGCACGTCCATAAT
CGGGCTGCCGAGTTTGCTGGAAAGGTGGTAGGCAAGGACGGAGCCGAGGGACTCGTGGCG
CAACACGGTTTGGTGCAAAAAACTTGCCAGCATCGGTTCGGCGGAGACCGCGGCCGCGGT
TTCTTCGCGGATGGTGTGCCAGAGGTCGAAACCGGTTGTGTTTAAATGGTCTTTTTTCAT
GAGTGATGACGTTTGAAAATCGATATGGTCGGCAGTATCTTACCGTCTATATTATTTTTT
CGGTAGGGGATTTGAAAATGAATTTGAAATTCTCTGCTTTTGCTTGAAGTTTCTTGAAAA
TGTCCTTATCTTGCGCGGGTAATAACTGGATTTTGATTTCCAATTTGTTTTAAGGGATAC
GATATGAGCGAACAGACACAGCAGCAAAACAGTGAAGAAGCGGTTGAAAATGTGGAGGCG
GTGGAAACCGTCGAGACAGTAGGAAATGCGGACGGTGTGCAGGAACAGGCTGCCGCAGAG
CCGGCTTATGAGGATTTGCAGGCGCGGATTGCCGAGCTGGAAGCGCAGTTGAAAGACGAG
CAGCTGCGCGCTTTGGCAAACGAGCAAAACCTGCGCCGCCGCCACCAGCAGGAAATTGCG
GATACGCACAAGTTCGCCGGACAGAAGTTTGCCGTGGAAATGCTGCCGGTCAAGGATTAT
CTGGAAATGGCGCTTTTGGATCAGAGCGGCAATTTCGATGCGCTGAAAATGGGCGTGCAG
ATGACTTTGAACGAGTTGCAGAAAGCATTTGATGCTACGCAAATCAAGGAAATCAACCCT
AAAGCGGGCGATAAGCTCGATCCGAATATCCATCAGGCGATGCAGGCGGTGGCAAGCGAA
CAGGAGCCGAATACCGTGGTGGGTGTGATGAAGAAGGGTTATACGCTGTCCGACCGCGTG
TTGCGCCCGGCTATGGTTACGGTGGCGCAGAAGGAAGCCTGAAGGCGTCTGGGGAATAAT
CTGATTTATTTCCTGAAGCGCGTTTTGCGTATAAACCGATCGAAGTAAAGCGGCAATGCC
GTCTGAACCCGCCTGTCGGGCTTCAGACGGCATTTTATAGTGGATTAACAAAAATCAGGA
CAAGGCGACGAAGCCGCAGACAGTACAGATAGTACGGAACCGATTCACTTGGTGCTTCAG
TACCTTAGAGAATCGTTCTCTTTGAGCTAAGGCGAGGCAACGCTGTACTGGTTTTTGTTA
ATCCACTATATTTCGGCGTTAACGGTCAACCCGTATGCCGCCTGCCTGTTTTTCTTCATC
CAGTTTCTTTTGCAGGGTGTCGCAAGGTGTGTCGCAGTCGCACATTTTTTTCATACCCAA
GGCAGTAATGCCGCCGCAACTGCCTTTGATGCTGCGTTTGGAGAAAATATAGCCGACCGC
CATACCGATGATGACGGTCAGGAAGATGCCGAAGGTAAGGAGCAGGGTTTTCATGGTGTT
TCCTAATCGGTTTGTATGTTTAGCGGAGCAGTTTTTCAAATTCGGAAGACATGGCGGTGC
GGTAGCCGCCTTTATCCCTGACAATCAGGAAAACAGCGAGTTTTTCGCGCTCTGCCAGCT
TTAAGGCTTCGGTTTCGCCCAATACGAATAATCCTGTGGACAAGCCGTCCGCCGTCATCG
CACTGTCTGCGACCACGCTGATGGAGGCGAGGTTGTGGCTGATGGGTCGTTTGTTGTTCG
GGTTGATGATATGGGAGAGGCGTTTGCCGTTTTTATCGACGTGGAAAATACGGTAATCGC
CGGAAGTGGCAAGCGAACGGTTGTTCAGCGGGACGATAATCTGCGTATTGCCGCCTTGGA
CGATATTGGGCTGCTCGATACCGATGCGCCACGGTTCGCCGCGCGCGTTTTTGCCTTTGC
CGTGCAACTCGCCGCCGATTTCGACCAGATAATTTTGAATGCCGTATTTTTCCAGTTCGC
CCGCAACTTTATCAACGCCGAAGCCTTTGGCAATCGAAGATAAATCCAAATAGGCCTTGG
GGTGGGTTTTGCTCAAGGAAGCGTAATCTTTGCCTTGTTTCAAAATGATTTTGTCTATGC
CCGTATAAGATGCCGCCTGTTTGATTTGTTCCGGCGACGGTTCACGGGTAACGGATTTGT
CGGGGCCGAATCCCCAAAGGTTGACCAAGGGGCCGACGGTTACGTCCAGCGCGCCGTGTG
TCAGGCGGTTCAGGCGGACGGCTTCGGCAGTAACGTGTGCGAAGTCGCTTGAAATGCGGA
GGGGCTTGCCGGCTGTGTGTTGGTTGAACCGGCTGATTTCGGAGTCGGGCTGATAGGTGG
ACATCTGCCGGTTGACTTCTTTAAGCGCGTCATCGATGCGTTTTTGTATTTCGGCAGGTG
AGGGGAGTTTGTCCCGATTATTTGAAAGGTATTTGACGGTATAGGTCGTGCCCATCGTTT
CGCCTTGCAGGGTAACGGTTTGCGCGGTTTGTTCCGAACAGGCGTTCAGGAAGATGAAAC
CCAGGGCAAATATCAAGACGCGGATAAAGTTCGGCAGGCGTGTTTCAGACGGCATAGTGT
TTGACGGTTTTGGCAAATGGTTTGAATTATATCGCAAAACGGCCGGTATGTTTCTATGCC
GATGCCGTCTGAAGGGTGTTCGGATGGCATCGGCATAGAAAAAGGAAGAAACCGAGGTTT
CTTCCTTTTGTATTTGAAGCCGAATATTTAACCGCCGAAATCGTCCAAGAGGATGTTTTC
GTCTTCCACGCCCAAGTCTTTGAGCATTTTGATGACGGACTGGTTCATAATCGGAGGGCC
GCACATATAAAATTCGCAGTCTTCCGGTGCTTCGTGGTTTTTCAGGTGGTTTTCGTAAAC
CACGTTGTGAATGAAGCCCGTGTAGCCGTCCCAGTTGTCTTCCGGCAGCGGGTCGGACAG
GGCGACGTGCCACGTGAAGTTCGGGAACTCTGCCGCGAGTTGGTCAAAGTCTTCGACATA
GAACATCTCGCGTTTGGAACGTGCGCCGTACCAGAAGGTAATCTTACGTTTGGAGTTCAA
ACGTTTCAACTGGTCGAAAATGTGGGAACGCATCGGAGCCATACCCGCACCGCCGCCGAT
AAATACCATTTCGGCATCGGTGTCTTTGGCGAAAAATTCGCCGAACGGGCCGGAAATCGT
AACTTTGTCGCCGGGTTTGAGCGACCAGATGTAGGACGACATTTGTCCCGGAGGCGCATC
AGGTACGCGCGGGGGCGGCGTGGCGATACGCACGTTCAGCATAATGATGCCTTTTTCTTC
AGGATACGAAGCCATAGAGTAGGCACGCAAAATCGGCTCGTCCACTTTGGAAACGTATTG
CCACAAATTGTATTTGTCCCAGTCTTCGTGATATTCCTTAGGAATGTCGAAGTCTTTGTA
GGCAACAGTGTGAGGAGGAGCTTCAATTTGAATGTAGCCGCCGGCGCGGAAGGGGACTTC
TTCGCCTTCGGGAATGGCAAGCTTGAGTTCTTTAATGAACGTGGCTTTGTTATCGTTGGA
GATGACGGTGCATTCCCATTTTTTCACGCCGAACACTTCTTCGGGGACTTCGATGTCCAT
GTCGGTTTTGACGTTGACTTGGCACGACAGACGGCAGCCTTCGCGTGCTTCGCGTTTGCT
GATGTGGGACAGCTCGGTCGGCAGGATGTCGCCGCCGCCGCTTTTTACGACGACGCGGCA
TTGTCCGCACGAACCGCCCCCGCCGCAGGCGGAGGGGATAAAGATGCCTTCGTTGGCAAG
CGCGCCCAAGAGTTTGCCGCCGGCGGGCATCGTCAGCTCTTTTTCGCCGTTGACTTTGAT
GGTGATGTCGCCTTCGCTGACCAGTTTGGATTTGGCAAACAGAATCATCAGTGCCAAAAC
CAAAACGATGACGGTAAACATCACGATACCTAAAATAATCTCCATACCGATCCCTTTCTT
ATAACTGGATGCCAGAGAACGACATAAACGCCATCGCCATCAGGCCGGCGGCGATAAAGG

## Appendix A

TAATGCCCAAGCCTTTGAGGCCTTTGGGAGCGTCCGAATATTTCATTTTTTCGGTAATGC
CCGCCAAAGCGACAATCGCCAACATCCAGCCCAAGCCCGCGCCGAAGCCGTATACAACGG
ACTCGCCGAAGTTGTATTCGCGTTGCGCCATAAACGATACGGCGCCGAAAATCGCGCAGT
TCACGGTAATCAGCGGCAGGTAGATGCCCAATGCGTTATAGAGGGCGGGGACGAATTTAT
CCAAGAACATTTCCAAAATCTGCACCAAAGCGGCAATCACGCCGATGAAGGTGATGAATT
TCAAAAAGGTCAAATCCACGCCTTCGGCAATCGCGCCGTCTTTGAGCAGCGAGTAAACGA
GTTGGTTGACAGGGACGGACAGCCCGAGTACGAAAATTACCGCCACACCCAAACCGAATG
CGGTGGATACTTTTTTGGATACCGCCAAAAACGTGCACATACCCAAAAAGAAGGATAGTG
CCATATTTTCAATGAAGACGGATTTGATGAAGAGGCTCAAATAGTGTTCCATAGCTTATT
CCTCCGCCTGTTCGGGTTTCCAGGTACGCAGTCCCCAAATCAAAAAGCCGATGATGAAGA
ACGCGCTGGGGGCGAGCAGGAACAAGCCGTTGGTCTGATACCAGCCGCCGTCCTGCACGG
TTTGGAAAACGGTGTAGCCCAAGAGTTTGCCCGAGCCAATCAGTTCGCGGACGGTGGCGA
CGACAAGCAGCATTATCCCGTAGCCCGCGCCGTTGCCGATGCCGTCGATCAGGCTTTCCA
GCGGCGGCTCTTTCATCGCAAATGCTTCGGCGCGGCCCATCACGATACAGTTGGTAATAA
TCAGACCGACGAATACGGAAAGCTGTTTGGACAATTCGTAGGCAAATGCCTGCAAGAGTT
GGTCGACCAGCGTAACCAGCGACGCGATAATCGCCATTTGCACGATAATACGGATGCTGT
TGGGGATGTAGTTGCGTACCAGCGAAATGAAGAAGCTGGAAAAACCGGTTACCAAAGCTA
CGGAAATACCCATCACGATGGCCGTCTGAAGTTTGGTGGTAACCGCCAAAGCCGAACAAA
TACCCAAAACCTGCAAGGCAATCGGGTTGTTGTCGATAAAGGGTGAAAACATCAAATGTT
TCAAGCGTTTCATATCAGCCATTATTGCGCTCCTGCTGATTTCAATTTGTTCAGGTAGGG
GATATAGCCGTTTTCGCCGAACCAGTAGGCGAACGAACCTTGCACGCCTTTGGATGTCAG
CGATGCGCCGGAGAGGGCATCTACGCCGTGTTCTTTGTCCGAACCCGCGCCTTTGCCGAC
GTGCAGGGCGAGTTTGCCTTGTCCGTCAAACAGTTTTTTGCCGACGAATTTTTGCTGCCA
CAACGGATTGCCGATTTCGCCGCCCAAGCCCGGGGTTTCGCCTTGTTCGTAGTAGGTAAT
GCCGTTGATGGTGTTGCCGTCGGGCTGGATGGCGACAAAGCCGTACATGACCGACCACAA
ACCGTTACCGTGCATAGGCAGGATGATTTGCCCGATTTTGCCGTCTTCGCCTTTTACCAA
ATAAACCTCGGTGTATTTGGCACGGCTTTTGATGCCTGCCAAATCGTCTTCGGTTTTGAT
GCGGATGCTTTGGGCAGGGTCTTTGCCTGCGATGCGCGCGCTGAAGTCTTTAGGCGCATC
GGCGACGTATTCGCCGGTCGCCAAATCGACAACACGTTGCTCGATACGCTCGGCAAAGGT
TTTACCGATGTCGGTGTCCTTATCCATCAAACCGGCTACGCTCAAGATATAGCCTTGTTT
GTCTTGGAGTTTTTGTTTCTCTTGGATGGGTTTCAAGCCGACGACCGCACCGGCAACGAT
GACCGAGCAAATCAGGCTGACCGCCAACACGACAATCAGCGTGCCGCTGAAGCTGTCTTT
ATCGAATTTCTTAGCCATTGCTGCGCGCCTTTCTGCGTTTGATGTTCGCTTGTGCGACGA
AATAGTCGAAAATCGGGGCAAACAGGTTGGCAAACAGAATCGCCAACATCATGCCTTCGG
GGTAAGCCGGATTGACCACGCGGATTAATACGCACATCACACCGATCAGTGCGCCGTACC
ACCATTTGCCGACATTGGTAAAGGAAGCGGAAACAGGGTCGGTCGCCATAAACAGCATAC
CGATGGCGAAGCCGCCGACCACCAAGTGCCAGTACCAAGGCATAGCAAACATAGCGTTGG
TGTCCGAACCGATGAAGTTGAACAGCGAAGACATCGCAATCATACCGATCATCACGCCGG
CAATAATGCGCCAAGAAGCGATGCGGGCAAACACGATAAACGCGCCGCCGATTAAGAGTG
CCAAAGTGGAGACTTCGCCAATGGAGCCGGGCAGTTTGCCGATAAACGCGTCCATCCAAG
TGATGGTTTGACCGGTTACGGCGTTTTTCAGGCCGTCTGCACCGTGTGCCGCCCATTGCG
CCAGTGCGGTTGCGCCGGAATAGCCGTCAACCGCCGTCCAAACCGCATCGCCGCTCAAGT
TGGCAGGGTAGGCGAAGAACAGGAAAGCACGGCCTGCCAGCGCAGGGTTCATGAAGTTTT
TACCTGTACCGCCGAATACTTCTTTCGCAACCACAACGCCGAAAGAAATACCCAAAGCCG
CCTGCCACAGCGGCAGCGTGGGCGGAACGATTAAGGCAAACAGAATCGAAGTAACGAAGA
AACCTTCGTTGATTTCGTGTTTGCGCACGGTGGCGAACAAAACTTCCCAGAAACCGCCCA
CAACAAATACAGTCGCGTAAATCGGCAGGAAGTAAATCGCGCCAAACAGCATTTTGTCCG
ACACGCCCGCTTCAGACGACATATTGATGCCCAAAGCGTTGGCAAAGGCGTAATGCCAGT
CGTTGGCGATGTTTTGTTGCAGCAAATCAGGCGTTAACGCACCGAATGCCTGCGCGCCGA
~~CGTTGTACATACCGTAGAACATGGCAGGGAACAAAGCCAGCCACACCAAAATCATCATGC~~
GCTTGGAGTCGAGCGCGTCGCGGACGTGCGCCGCTTTGCGCGTTACCGCGCCGGATGTGT
AGAAAATTGTCGCCGCAGCTTCGTAGAGGGCATACCATTTTTCATGTTTGCCGCCCGGCA
GGAAGTGCGGTTCGATTTTTTCCAGAAAATGTTTCAAGCCCATAATCAGCCTTCCTTCTC
AATGGTTTCCAGCACTTTGCGCAACAGCGGGCCGTATTCGTATTTGCCCGGGCAGACGAA
GCTGCACAAAGCGAGGTCTTCTTCGTCCAATTCCAAGCAACCCAATGCCTGCGCGCTGTC
GGTATCGCCGACGATTAAATCGCGCAAAAGCAGGGTGGGCAGGATATCCAAGGGCATCAC
GCGCTCGTAAGTACCAATCGGCACCATGGCGCGGTCGCCGCCGTTGACGGCTGTGTTGAA
CTTGAAGAGTTTGTTTTTCAGGAAATGGCCGAGGGTTGTACGCGTGATGGAGTATTTGTC
CGGCTGCGGCGCAACCCAGCCGAACAGCTCTTTGCTGCGGCCTTCTTCGATAACGGAAAT
CTGATTGTGGTAGCGTCCCAAATAATCGTGCGCGCCTTGTGTAATCGCGCCGTTCAATAC
CGAACCGGAAATCACGCGGTTGTCTGTGTCAACCAATTCGCCCGCAGTAATTTGCGATAC
TTTCGCACCCAAAACGGTACGCAAGAGGCGCGGTTTGTTGACTTGAGAACCACCTAGGGC
AATCACGCGCTCGGTGTTCAGACGGCCTGTTGCAAACAAACGGCCAATGGTAATTACATC
TTGATAATTGATGGTCCACACGGTTTTATTCGCGCCGACCGGCTCGATGAAATGAATGTG
CGTGCCACTCAAACCGGCAGGATGCGGGCCGCCGAATTCATGTGTTTCGATGTTGGCAGC
ATTTTCAGACGGCACGTCTGCGCCAGCTGCCTTACAAACATGGATTTTGCGTTCGGTCAA
ACGGCTCAATACCAACAGGCCGCGTTTGAAATCCTCGGCGGCTTCTTTGATAATGACCGT
AGGGTCGGCAGCCAGCGGATTGGTGTCCATCGCATTGACGAAGATGGCGAACGGCTCGGC
ATCGACGGCAGGAATTTTGCTGAACGGACGGGTGCGCAGCGCAGTCCACAAACCGGATTG
GATCAGGTTGCGGCGCACTTCTTCGCCGCTTAAGTTTGCCAGCGCTTCAGGTGCGTAGCG
TTCAAACTCGATTTCGTCGTTGCCTTCAACGGCAATCACGACTGACTGAAGTACGCGCTT
TTCGCCACGGTGAATCGCGGCGATTTTGCCTGAAGCCGGCGCAGTAAACACCACGCCCGG
ATTCTTTTTGTCTTCAAACAGCACTTGGCCTTTTTTGACGGCATCGCCTTCCTTGACTTT
CATCGAGGGGCGCATACCGGCATATTCTTCGCCAAGCAACGGACTTCGGTAATGGCCGG
GCCGTCGTAAACGGCTTGCTCCGGTCTGCCCGCGATGGGCAGGTTTAGACCTTTTTTGAT

## Appendix A

TTTAATCATATATTTGCATTACTTGTGATGGTTAAGGTAAAAACGGCGTGTTTTGATACC
GTGTCGCGTGGCATCAAAAGCATTGAATAAATTAATGTAGCAAAGTGTTAGATTCTATCA
GGAATTGTACCTGTTTGTCAGATTTGCTGCTTTTTTCCTTGCGGAAGCCGTTTTTATAGT
GGATTAAATTTAAACCAGTACGGCGTTGCCTCGCCCTTGCCGTACTATTTGTACTGTCTG
CGGCTTCGTCGCCTTGTCCTGATTTTTGTTAATCCACTATAAATTGTCGGAAGGGGGGAT
ATTGATTTGATTATGCCGGAATTTAAAATGCCGTCTGAATGTTCAGACGGCATAGCGTTT
ACAGCAGTTTGAAAACGAAAAAGATAAGGGTATGTACGATGAAGACGGGTGTCAGGAAGG
CGACCGACCACATCATATAGCCGAAGAAAGTCGGCATCGGTACGCCGCGCTGTTCGGCAA
TGGCCTTGACCATGAAGTTCGGTGCGTTGCCGATGTAGGTCAGTGCGCCCATGAATACCG
AACCCATAGAAACCGCCAGCAGCGAATGAAACAGGGTACCCGTCATCAAGGCTTGGGCAT
CGCCGCCCGCCATATTGAAAAAAACGAGATAAGTGGGCGCGTTATCCAAGAATGCCGACA
ATATGCCGCTCATCCAAAAATACATCACATTAATCGGATGACCTGCCGTATCGTGAACCA
GCGATACCACCCCGCCCAGCGCGCCTGCCTCGCCTGCTTTCAGAATGCTCAGGACGGGAA
AGATGGTGATGAAGATGCCGAGGAAGAGTTTGCCCACTTCGGCGATGGGTTCAAAGTTGA
ATTCGTTGCCTGCGCGGACTTGTTTGGGCGTGATTGCCATAGATACGGCGGTCAATGCAA
TCAGGATGACATCGCGGACGAGGTTTTGCAGGGCGTAACGGCTGCCGAGGATTTCAAATC
CCGGGTGTTCGGGTTTCCAAAGGCCGGACATTAGAACCGCGCCGACCACGCCCGAAAGCA
GGAGGAAGTTCCATTTGCCGAAGATGGCGATTTTTTCGGGTTTTTCCTGTTGTGCCGGCG
TATCTTGTGCAATGCTTTCCTGTTTGAAGAAACGGTTGTCGATGAAATAGAAGGCGGTCA
ACAGGACAGCGGTGCTGATCAGGACGGGGGCGAACATATGTTTGACCGTCCACATGAAAT
CTACGCCTTTGAGGAAGCCGAGGAAGAGTGGGGGGTCGCCCAAAGGGGTCAGACCGCCGC
CGATGTTTGCAACCAGGAAAATGAAGAAGATGACGATGTGCACGCGGCGGGTACGGTTTT
GGTTGGCTTTCAGCAGCGGACGAATCATCAGCATTGCTGCGCCGGTCGTTCCCATGATAG
AGGCAAGTGCCGTACCGACGGCAAGCAGGGCGGTGTTGAGCTTGGGTGTGCCGTTCAAGT
CGCCCCAAACCAAAATGCCGCCTGAAATGGTGTACAGGGCAAGCAGCAGCAGGATGAAAG
GGATGTATTCTTCAACGAGTGCGTGTGCGACGGTATGGATACCGGCGGACGCGCCAAAAA
CCAAACTGAACGGGATGAGGAAGAGCAATGTCCAAAAGGCGGTAATTTTGCCGTAATGGT
GATGCCAGGTATGCGAAAAAAACAAGGGACCCAATGCGATAGACAGCAAAATCAGGGCAA
AGGGCAGGCCCCACAGCAGGTTTAGGTTTGCGCCGTCCAAATCTGCGGCGTAAACCGATG
CTGGGAAAAGCATTAGTGAAAACAGGGGTAGGTGGCGCATCGTGTTTCCTCGATTCAAGC
ACTGCCTTGCGCGGCGCGTGGGAGTGATACAGGCACCGTGCCGCCCGGACATAGGCGGAC
TGTGCTGCCTGTTTGTTTTTGAAAAAAGTTTTCCGATTGATTGTAAAGTAAATAATCAGG
ATAAACCAGTTTCCCAAACCGGAAGGCGGCGGGAAGGCGGATTGCTGTGCTTGGGAATAT
ATGTTCTTTTTGATAAATAATTTTATTTAAAACAAATAAATATATGTCTTTAATAAATAA
TCTATCGAAAACGAAAAATGAATTTATTTTAACATATATTTGCAATGAAACAGGTTTGCC
CCCCCCCGTTTGTTTGCCCTTATCCCTTTCAGTACGGCATTCAAGATTCGGGCCTGCGCC
ACATCCATATGGCGACAAGGGAACAAAAAACCGATGAAACCGCCCCGACCCACCAGCGTT
GGGGAAACTGCCAAAACATTATCAGGCAGGATGCGGTCATCATACTGATGGCGAATATTT
TGGCTTTGCGCGGCACTGCGCCGTTTTGTTCCCAGTTATGAACCATCGGGCCGAAATAGC
GGTGCCGGTGCAGCCAGCGGTAAAAGCGCGGGGATGCCTTTGCCCAGCAGGCGGCGGAGA
GCAGTACGAACGGCGTGGTCGGCAACAGCGGCAAAAAAATGCCGATGATACCCAACAGTA
GGGAAATGCAGCCGCAGGCAATTAAAAGATAACGTATCATTTTGAAATATTTTTCTTATT
GTGCGGATAAGGGCAGGATGTGATACCGAGTTTTGCCCAGCCTTCATGTCCCATTTTTTC
CAGCAGGGCGATATTGCGTTCGAATATTGCCGAAGCGTCGGGAAAGGCTTGTGCGGCTTT
GGCAATGCTGTCTTCGCGGATGAGGTGCAGCGTCGGATAGGGAGAACGGTTGGTGTAGTT
GCCAATGTCGTCTGAATCCGTGCCTTCAAATTGGAAATCGGGATGAAACGGGGCGATTTG
GACGATGCCTTCTAAGCCGTTTTCGACAACGGCGGCATCGGCAATGTCGAGCATATCGTT
GAATACGTCGAAATCGGGGAATAGGGTCGGGTGAACCAGCAGGGTGGTTTCCAGTTCGGT
GGCGGGTGTATTGCCCAGTCGCTGCAGTTCTTCGTCCAAGTCTTCCAAAAAACCGTCAAG
GTGTTTGGCTTCGCTGATCGCGATGCGGACAAGGTTTTTAACGTGGGGGGCTTTGGCAAA
GGGACACAGGTTCAGACCGATGACGGCTTTTTCCAACCATTGTCCGGTGTGTTCGGCAAC
AGCATCTTTATTTTCGGAAGTATTGATATTCATTATTGTCATGTAAATGTGTTTGCAGAT
TGCACGTGCGGGAAAATCGGGAAGGGCACTATTCCTTCAGCAGGTGGTTGAGCGGCAGGG
AGGTGGTGTGTTTGATTTCTTTTAAAACAAAGCTCGATTGCGCATCTTGTACGCCGTGGT
GGGACAGGAGCGTATCCAAAACAAAATGGGAAAACGCGTTCATATCGGTAAAAAACGCCT
GAAGCAGGTAGTCGGTTTCCCCTGTCAGGGCGAAGCAGCTCAAGACTTCAGGCCATTTTC
GAACCGATGCGGCAAAGTCTTCCCGCGCGTCTTTTGCTTTGCGGATGGAAACGCGGATAA
ATGCCTGAAGTCCCAAGTTGACAGATTCCGGAGACAGCAGCGCGGCATATTGGCGGACGA
TACCGGCATCTTCCAACTGCTTCAGACGGCGCAGGCACGGAGAAGGCGAAAGTGCGACAC
GTTCGGACAGTTCGACATTGGTCAGCCTGCCGTTTTCCTGGAGAACCTGTAAGATTTTAA
TATCGGTTTTGTCTAAAGTGAGTTGGGGCATATTTGCGTTCCGTTTTAAGGAATTCGGAT
TGTCTGTCCGTATGTTTGCGGCAATCCGCACAGATGGAGACCATATTAACATATAAAAAG
TTATACCGTCATCCGGGACAAATTTTGTTTTCGGAAAATCATGTGAAAACAGAGGCGGTC
GGTTTGCATCTCTTTAAGACGGCTTGCCCAAACCGCCGATTCAAGACATAATCGGGAAAT
GTGCAGGAGAGTGTTACACCCAACTACAATGTAACCACCGAAGGCGCAGACACCCTTAAA
TCGCTCAGGTATCAGGGACTGCACATTGAAACAAACAATCTGGAGAGCGGCGTTGGAATA
ACGTCCACCGAAGGGGAGAAGGCCGTCTGAACCACCATTCAGACAACCGCGCAAAGCAGT
GAGCAGACTGGTTTGCCATCATGCGGATACAGCCGAAAATCTCAGGTTCAAGGACAGATA
GGGTCATCCGCGCACAGGTGCGCGGGCGGCATCTGAACAAAAAATCCGGAGAAACTTGAG
AATGACTGCTCTGAAAACCACCCCATTTCATCAAGCCCATCAAGATGCAGGCGCGAAGCT
GGTCGATTTTGCCGGCTGGGAGCTGCCCATCCATTATGGTTCACAAATCGCCGAACACGA
AGCCGTGCGCACCGACGCCGGTATGTTTGACGTATCCCATATGCTCGTTACCGACGTAGC
AGGCGCAAATGCCAAAGCCTTTTTCCGCAAATTGATTGCCAACGATGTCGCCAAGCTCGC
TTTTGTCGGCAAAGCCCTTTATTCCGCTTTGCTCAACGACAACGGCGGTGTGATTGACGA
CTTAATCGTTTACCGCACCAATGAAGCCGAAACCCAATACCGCATCGTGTCCAACGGCGC

## Appendix A

GACCCGCGAAAAAGACACGGCGCAATTCCACAAAGTCGGACAAGAGTTCGGCGTCGCCTT
CAATCCGCGCTACGACCTCGGCATGCTCGCCGTACAAGGCCCTAAAGCCATTGAAAAACT
CCTGACCGTCAAACCCGAATGGGCAGATGTCGTCCATAACCTCAAACCGTTCCAAGGCGC
GGATTTGGGCAACGACTGGTTTGTCGCCCGCACCGGCTACACCGGCGAAGACGGCGTCGA
AGTCATCCTGCCCGGCACCGAAGCCGTCGCATTCTTCAAAGCCCTGCAACAAGCCGGCGT
ACAGCCCTGCGGCCTCGGCGCGCGCGACACCCTGCGCATGGAAGCCGGCATGAACCTCTA
CGGCAACGATATGGACGACGACACCAGCCCGCTCGAAGCAGGTATGGGTTGGACCGTTGA
CTTGAAAGACGAAAGCCGCGACTTCGTCGGCAAAGCCGCCTTGCTGGCATTGAAAGAAAA
AGGCGTTGCCGTCAAACAGGTCGGCCTGTTGCTCGAAAAAGGCGGCATCCTGCGCGCGCA
TATGGAAGTGTTGACCGACAAAGGCCAAGGCGAAACCACCAGCGGCGTATTCTCCCCAAG
CCTGAAACAATCCATCGCCATCGCGCGCGTACCGAAAGATTTTGACGGCGATACCGCCAA
AGTGCTGATGCGTGGCAAAGAAGTGGACGTGCGTGTACTGAAGCTGCCGTTTGTCCGCAA
CGGACAGAAACAGTTTGATTGATGCGGTTTCAGACGGCATTTTCATTTCATATGCCGTCT
GAAAGCAGGTTTTAATTGTTGTCCGATACGGACGTTTGTAGAAAGCATTGAACAAGGCAT
CTGTGGATATTGATTCATGCAGATGCCGTCTGAAAATAACCCCTATCAATGGAGTATCAA
ACCATGAGCAACAACATCCCGGCCGAACTGAAATACGTTGCCAGCCATGAATGGCTGCGC
CTTGAAGAAGACGGTACGATTACCGTCGGCATTACCCACCACGCGCAAGAGCTGTTGGGC
GACATCGTGTTCGTCGAGCTGCCCGAAGTCGGCGCGAACCTTGCCGCTGAAGAGCAAGCC
GGTGTGGTTGAGTCTGTAAAAGCCGCGTCCGACGTGTACGCACCGATTGCAGGCGAAGTC
GTTGCCGTCAACGAAGATTTGCCAAGCGCTCCGGAAACTGCCAACAGCGATCCTTACGGT
GCAGGCTGGTTCTTCAAACTCAAACCGGCAAACGTTGCCGATTACGACAGTCTGCTGACT
GCCGAACAATACGCGGGCGAAGTGGATTAAACCGCCCGGCTGCCCGACGGCAACCGCCGG
ACAAACGGAAACTGCACCTTCAGACGGCATTTTTGCGGTCGGAGGTGCAGTTTTTTGTCC
GTGTTTTAAGGAAGCAGTTAGGCTATAATAACGGTCTATATTCATCTTTACCGATTTTTT
CATGCAACTTACCGCTGTCGGACTCAATCATCAAACCGCACCTTTAAGCATACGGGAAAA
GCTGGCGTTTGCCGCCGCCGCCCTGCCTAAAGCCGTCCGCAATCTTGCCCGAAGCAATGC
GGCAACGGAGGCGGTAATCCTTTCTACCTGCAACCGCACCGAGCTTTACTGCGTCGGTGA
TTCGGAAGAAATCATCCGATGGCTTGCCGATTACCACAGTTTGCCGATTGAAGAAATCCG
TCCGTATCTGTACGCGCTGGATATGCAGGAGACTGTGCGCCATGCTTTCCGCGTCGCCTG
CGGGCTGGATTCGATGGTGTTGGGCGAGCCGCAGATTTTAGGACAGATTAAGGATGCCGT
TAGGGTTGCTCAAGAGCAGGAAAGTATGGGTAAGAAACTCAATGCCCTGTTCCAAAAAAC
CTTTTCCGTTGCTAAAGAGGTCCGTACCGATACTGCCGTCGGCGAAAACTCGGTTTCCAT
GGCTTCCGCTTCCGTCAAATTGGCGGAACAGATTTTTCCCGACATCGGCGATTTGAATGT
CTTGTTTATCGGCGCAGGCGAAATGATTGAGCTGGTTGCCACTTATTTTGCCGCCAAAAG
TCCCCGGCTGATGACGGTTGCCAACCGGACGCTGGCGCGTGCACAGGAGTTGTGCGACAA
GCTCGGTGTCAACGCCGAACCGTGCCTGCTGTCCGATCTGCCTGCCATTCTGCACGATTA
CGACGTAGTGGTTTCTTCAACGGCAAGCCAGTTGCCCATTGTCGGCAAAGGCATGGTGGA
GCGTGCATTGAAACAAAGGCAGAGTATGCCGTTGTTCATGCTTGATTTGGCAGTGCCGCG
TGACATTGAAGCGGAAGTCGGCGATTTGAATGATGCCTATCTTTATACGGTGGACGATAT
GGTCAATATCGTCCAAAGCGGCAAGGAGGCAAGGCAGAAGGCCGCCGCCGCCGCCGAAAC
GCTGGTGTCCGAGAAAGTTGCCGAATTTGTCAGGCAGCAGCAGGGCAGGCAGAGTGTCCC
CTTGATTAAGGCGTTGCGGGACGAGGGCGAGAAAGCGCGCAAACAGGTGTTGGAAAATGC
CATGAAACAGCTTGCCAAAGGCGCAACGGCAGAAGAGGTTTTGGAACGGCTGTCCGTCCA
ACTGACCAACAAGCTGCTGCATTCGCCGACCCAAACCTTGAATAAGGCGGGGGAAGAAGA
TAAAGATTTGGTTCATGCCGTCGCGCAGATTTATCATTTGGACAAATAACGGTGCGCCGG
GAAAAATGCCGTCTGAAGAGGTTTCAGACGGCATTTTTTTGTGCCGCCTGACAACATCGT
GAAATCCCACATTATATCGATGTAATCACAAAGTATAGTGGATTAACAAAAATCAGGACA
AGGCGACGAAGCCGCAGACAGTACAGATAGTACGGCAAGGCGAGGCAACGCTGTACTGGT
TTAAATTTAATCCACTATATTATCCCGTATGCGGATTGGTTTTAAGATTTGTAAATTTGA
TTTGCATCAAAAAATCGCCGATAGATGATTCATATAATATCAATATTAAAGAGTATCGGT
ATATCGGGGATAGTCATGTCCTGTTTTTCAATCAAACGTATGTCCGCGTTTCGGGCGCGG
ATAACGGCGTTTTTTGCCGCCTTTGTCTTTTTGACGGCGGCACTGCCCGCTTATGCGGAG
CGTCTGCCTGATTTTCTGGCGAAAATACAGCCTTCGGAAATTTTTCCGGGTGCGGACCGT
TACGGCAAGCCGGAAGGTAAGCCTATGGTTGCCCGCGTTTACAAAGGCGATGAGCAGTTG
GGCTTGGTCTATATCACGACCGATGCGGTCAATACGCGCGGTTATTCGAGCAAACCGATT
GATACGCTGATGGTGTTGGCAAACGACGGCACGATAGCCGGGGCGAAACTGGTCGACCAT
CACGAACCGATTATGCTGATCGGTATCCCGCATTTGCCCGCGCCCGGGCGGGCGATACGC
TCAAACTGGCTTCCGGCGTATATAAAACCAAACTTCACATTGACAAACCGATTACGATTG
AAGGGCCTGCCGACCGTTCCGCAACCATCGAAGGCGACAGGAGCGGGCGTACCATAGCCG
TACACGCGCCGGACGTAACGCTCCGCAACCTGACCGTTACCCGTTCCGGTATGAGCCTGC
CCGCAATGGATGCCGGTATTTATCTCGAAGAAACTGCCCCGCGCGCCCTGATTGAACACA
ACAATATTTTGGATAATTCGGTCGGCGTATATCTGCATGGTTCTGCCGATGCGATGGTGC
GCGAGAATAAAATCGTCGGCGACGCGACTTTGCGCGTGAACGAGCGCGGCAACGGCGTTA
CCGTTTGGAACGCACCCGGTGCGCAGGTCGTCGGCAACGACATTTCCAAAGGGCGGGACG
GCATTTTTTCCAATACCAGCACGCACAACACCTACAAAAACAACCGCTTCAGCGATTTGC
GTTTCGCCGTCCACTATATGTACACCAACGACAGCGAAATCAGCGGCAATATTTCCGTGG
GCAACAATATGGGCTATGTGCTGATGTTTTCCGAGCGGCTCAAAGTATTCGACAATATCG
CCGTCGGCAGCCGCGATCAGGGCATTATGCTCAACTATGTCAACTATTCCGATATTCACG
ACAACATTATCAACAAGGCAGGCAAGTGCGTATTTGCCTATAATGCCAACTACGATAAAC
TTTTCGCCAATCATTTTGAAAACTGTCAAATCGGCATACACTTTACCGCCGCCATCGAAG
GCACGTCCTTGCATGACAATTCCTTTATCAACAACGAAAGCCAGGTCAAATACGTCAGCA
CGCGCTTTCTCGATTGGAGCGAGGGCGGACACGGCAACTATTGGAGCGACAACAGCGCGT
TCGATTTGAACGGCGACGGCTTCGGAGACAGCGCGTACCGCCCCAACGGCATCATCGACC
AAATCATCTGGCGCGCGCCCGTATCGCGCCTTTTGATGAACAGTCCCGCAATCAGCATCG
TCAAATGGGCGCAGGCGCAGTTTCCCGCCGTTCTGCCTGGCGGCGTGGTGGACAGCAAAC

## Appendix A

CGCTGATGAAGCCTTATGCCCCCAAAATTCAAACCCGTTATCAGGCGATGAAGGACGAGC
TACTCAAAGAAGTCGAAACGCGGCAGTCGGAATGGGGCAGGGCGGAAAACGGTTCTTTGA
ACTAGTCTGCTTCAGACGGCATCCGGATTCAAATGCCGTCTGAAAACACAAAGGAACAA
CCATGACCACACATCATGTCGAATTGAGGAAGGTAACCAAACGGTTCGGGGCGCAAAAAG
CCGTCAACCAAGTCGATTTGGTTTTGAAGGCAGGAGAAAGCGTCGGGCTTGCCGGACACA
ACGGCGCGGGCAAGTCCACCATTATGAAGCTGATACTCGGGCTGATTACCCCGACCGAAG
GCGAAGTGATGCTTTTGGGCGAACGTACCGGTAGCAAAGCGGGGGCGCGGCTTCGCAGCC
AAATCGGCTACCTGCCCGAAACCGTTGCGCTGCACCCTTCGCTGATCGGCATCGAAACGC
TGGATTTTTATGCCAAACTTAAAAAACAGCCGCTCACGCAGAACCGGGGGCTGCTTGAGC
GCGTCGGCATTTCACAGGCGGCACACCGCCGCGTCGGCACTTATTCTAAAGGGATGCGCC
AACGCCTTGCCTTGGCACAAGCCCTGCTGGGCGAGCCCAAAGTCCTGCTGTTTGACGAAC
CGACAACCGGTCTTGACCCTGCATCACGACAAATGTTTTACGAAGTCGTGCGCGAACTCA
ACGGGCGCGGCGCGACCGTATTGCTCAGCACCCACGCCCTTGCCGAGTTGGACGGGCACG
CCGACCGCATTATCGTGGATTAAATTTAATCCACTATATGCGGGTATGGCGGGTTTGAGC
GGACAAATCAGCCTGACCGTCCCCGTTTTGCTGACCGCTCAGGTTTTATGGGTTATCATT
CCGCTTGTTTTGGCAGCCGGAATTTTTAGAAAGCGACAAATATGAAAAAAACCCTGTTGG
CAATTGTTGCCGTTTCCGCCTTAAGTGCCTGCCGGCAGGCGGAAGAGGGACCGCCGCCTT
TACCCCGGCAGATTAGCGACCGTTCGGTCGGACACTATTGCAGTATGAACCTGACCGAAC
ACAACGGCCCCAAAGCCCAGATTTTCTTGAACGGCAAACCCGATCAGCCCGTTTGGTTCT
CCACCATCAAGCAGATGTTCGGCTATACCAAGCTGCCCGAAGAGCCTAAAGGCATCCGCG
TGATTTACGTTACCGATATGGGCAATGTTACCGATTGGACGAATCCCAATGCCGACACGG
AGTGGATGGATGCGAAAAAAGCCTTTTACGTCATCGACAGCGGCTTTATCGGCGGTATGG
GTGCGGAAGACGCGCTGCCGTTCGGCAACAAAGAGCAGGCTGAGAAATTTGCAAAGGATA
AAGGCGGTAAGGTTGTCGGTTTCGACGATATGCCTGATACCTATATTTTCAAATAATATT
GAAAAAACCGGCCGAAACTTTAAAAACGAGTTTCGGTCGGTTTTTCTTTTCTTGTTCGGT
ATAGTGTCGGCAGGAAAGAACCTTCACATCCCGCCGTAATTCGGCCCGCTCGCGCCTTCG
GGGCAAATCCAAGTGATGTTTTGCGTCGGGTCTTTGATGTCGCAGGTTTTGCAGTGCACG
CAGTTTGCCGCGTTGATTTGCAGGCGCGGATTGCCGTTTTCTTCAACAATTTCGTACACG
CCGGCCGGACAATAGCGCGTTTCGGGCGAGGCGTATTCTTTGTAGTTCACGTCTATCATC
GTTTGCGGATTGTTCAGCACCAAATGGTCGGGCTGGTTTTCTTCGTGCGCGAGATTGGCA
AGGAAGACGCTGCTCAAGCGGTCGAAGGTCAACACGCCGTCGGGTTTCGGATAATCAATC
GGCTTACACGCGGCGGCTTTTTTAAGCTGCTCGTTGTCTTTGCCGTGATGTTTCAAGGTC
CACGGGGCTTTGCCTCTGAAAATCATCTGATCGATGCCGGTGTAGATTGAGCCGAGGTAA
ACGCCCCATTTGAATGACGGACGGACATTGCGCGCGGCGTAAAGCTCTTGATACAGCCAG
CTTTGTTCAAAACGTTGCTGATAATCCGCCGCCTCTTTGCCGCTGTCGAAACCCTCCACT
TCTTCAAGGTTTTCCAACAAGGGGAACACGGCTTCGGCGGCGAGCATGGCGGATTTCATC
GCGGTATGAATGCCTTTGATGCGCGGCATATTGAGGAAACCCGCCGCATCGCCGACCAAA
ATGCCGCCTTTGAACGAGAGCTTCGGCAAACTTTGCAAACCGCCTTCAATCAGCGAACGC
GCGCCGTAAGCAATGCGGCGGCCGCCTTCAAAGGTTTTGCGGATTTCGGGATGGGTTTTG
AAACGTTGGAACTCTTCAAACGGCGACAGATAAGGATTTTGATAGTCCAAACCGACCACG
AAGCCGACGGCGACTTTGTTGTCGTCGAAATGGTAAACAAACGCGCCGCCGTAGGTTTTG
CTGTCCAGCGGCCAGCCTGCGCTGTGCACCACCAAACCGGGCTGATGCTGTTCGGACGGC
ACTTCCCAAACTTCTTTAATGCCCAAGCCGTAAGTTTGCGGCTGGCTGTTTTGGTCGAGT
TGGAAACGTTCGATGATTTGTTTGGAAAGCGAACCGCGACAACCTTCGGCAAACAGGGTT
TGCTGCGCCCAAAGCTCCATGCCGGGTTGGAATGAATCGGTCGGCTCGCCGTCTTTGCCA
ATGCCCATATTGCCGGTTGCAATGCCTTTGACCGAACCGTCTTCGTGATACAGCACTTCG
GCGGCGGCAAAGCCCGGATAGATTTCCACGCCCATATTTTCCGCCTGCTCCGCCAACCAG
CGCACGACTTCGCCCAAGCTGACGATGTAGTTGCCGTGATTGTCGAAATTCGGGGTAATC
GGCAGGTTGAACGCTTTTTTCTCGGTCAGGAACAACACTTTGTCCTGCGTTACTGTGCGT
GTCAGCGGTGCGCCTTTTTCTTTCCAGTCGGAAATCAACTCATTCAGCGCAATCGGATCG
ATAACTGCGCCAGCCAGCGAATGCGCCCCCACCTCCGAACCTTTCTCCACCACGCAAACG
CTGATTTCGCGCCCGTTTTGTTCGGCAAGCTGCTTGAGTTTGATGGCGGCAGACAAACCC
GACGGGCCTGCGCCGACAATCACGACATCGTATTGCATACTGTCGCGGGTGATGGATTCT
GTCATGGCGGTTCCTGTGTATTTATTATTGAATTGCAAATCCGTAATTATACAACGGGAA
CATATAGTTACCAAATACAACAAAGGTCGTCTGAAAACCATATTTTCGGTTTTCAGACGA
CCTTTGTCGAAATTTCAATAAGCACGCCACCATTTTACCTGTCCGACCGCAAACTCCGTC
TGACGTTTCGGACTGCGTGTGAAAAACGCCTTATCCCCGCCGGCATCCCTCCCTTTCGGC
ACAACCGCCAAAATCTTACCTGCCAAATTTCCCTCACGGGTTTGCCAAGCATCCAAAAAC
TGCGCCCTGCTCATTGAAACATGACCCAGCGACGGGTCGGCAAGCAAAACCGTATTGCCG
TCTATACCGCGCAATACCGAGAAATGATCATCCTTGCGGTATTTCAGATACACGATGACG
GGGATTTGCAACTGTGCAAGCTGCTCGAAAGACAGGGCATAGCCTTTCGCTTCAAAACCC
AAATCAGGCATAATGCGCCGCATATCCTCAAACGACGCGGGCATCTGCTCCTTATCCAGT
TTTTTTAACACGTCCTCTTCCGTCAGCTTTTGCCCGTAAAAATTGTTCAAAAGCGTCACC
ACCGAAGCCGCCCCGCAGGAAAAATCCAAATCCTGCTTTACAATATTGAAATCGCGCCTT
TCTTTCCAACTCTGCACTTTGATTTTTCCATAAGCAACAGGATTATAGTGGATTAAATTT
AAACCAGTACGGCGTTGCCTCGCCTTGCCGTACTATCTGTACTGTCTGCGGCTTCGTCGC
CTTGTCCTGATTTTTGTTAATCCACTATAGGTTTCCGTGCGGACGTGTTCAGATTCCCGC
CTTCGCTGGAATGACGGCGGAGCGATTTCTACTTTTCCGATAAATGACCGTAACTTAAAA
TCCCGTCATCCCCACGAAAGCAAAAATCCCGCCTGTCGGATTTCGGTTTTTTTGGGCGTT
TCGGGAAACTTATAAATCGTCATTCCCGCGCAGGCGGGAATCCGGTTTGCTCGGTTTCGG
TTTTTCGGGCGTTTCGGGAAACTGATGAATCGTCATTCCCGCGCAGGCGGGAATCTAGAA
CGCGGGACGGCGGCAATATTCAAAGGTTGTCTGAAAATTCAGAGGTTCTAGATTCCCACT
TTCGTGGGGATGACGGGATATAGGTTTCCCTACGGACGTGTTCAGATTCCCGCTTTCGCG
GGAATGACGGCGGAGCGATTTCTACTTTTCCGATAAATGACCGTAACTTAAAATCCCGTC
ATCCCCACGAAAGCAAAAATCCTGCCTGTCGGATTTCGGTTTTTTTCGGGCGTTTCGGGA

## Appendix A

AACTGATGAATCGTCATTCCCGCGCAGGCGGGAATCTAGAACGCGGGACGGCGGCAATAT
TCAAAGGTTGTCTGAAAATTCAGAGGTTCTAGATTCCCACTTTCGTGGGAATGACGGGAT
ATAGGTTTCCCTACGGACGTGTTCAGATTCCCGCTTTCGCGGGAATGACGGCGGAGCGAT
TTCTGCTTTTCCGATAAATGACCGCAACCTAAACCCCATCCTTCCCGCAAAAACAGAAAA
ACAAAAACCTAAAATCCCGTCATCCCCACGATAACAGTTGCGTAATTGCGTAGAGTGGGC
TTCAGCCCACCGTTTTTTCTTTTTCGGTCGTTGATTGGTGGGCTGAAGCCCACCCTTGTA
TATCGGAACTCCCGTATCATAGCAACAAACCGCCCGGCCGCCACCCGCGCCCACCCAAGG
CACACAACCGTTGCGTAGCACAGGGAGCGGCAGGGCAACCCATCGACACAACCGGACAGT
TGCCGGACAACACAACCGAATGTAAGGCAGGTTGATGATGAGTACCCGATACCATTACGC
AGGTATAGTGAATTAAATCTAAGGGGCTGTACTAGATTAGCCCTAAATTCCACACCAATC
CCGCAGGATTTTAAGCTGTTGAGACGGTGTGCCGAAGTTAAATCGAAATTCGCATTCTTT
CAAGAACAGCGGGAAAGATTTACGATCGATTCCGTTGTATTTTCGCAAGACGCGTTTTGC
CTGATTCCAAAAGTTCTCAATGCCGTTAATGTGGTTCTGACGGTCTGCACACTCCTTGGA
ATGGTTGATGCGGTAATGGATAAAACCGCTCACGTCCAACTTGTCGTAGCTGCTCAGACT
ATCGGTATAAACAATACTATCCGGCATGATTTTCTTTTTGATGACAGGGAGTAACGTTTC
AGACTTGGCATTATCCACCACAACGGTATAGACCCGTCCGTTGCGTTTCAGAATGCCGAA
AACAACCACTTTTCCTGCCGCACCGCGACCACGTCTGCCTTTACGCCGTCCGCCGAAATC
GCTTTCGTCCGGCTCGACAGGGCCCTCAAAAACCTCATCGGCAGCCAAGGCCAAATGATG
GTTGATAACCGTGCGGATTTTACGGTAGAACAGTGCTGCCGAATTGGGATGGATACCCAA
AATATCGGCGGCAGAACGGGCGGTAACTTCCAGTACAAAAAACGGAGCAGTTCTTTCTGT
ACTTTTTTCTTTAATTTGCAGTGCGTTATCTTCATATTTCGAGGGTAACATATCTGCTAA
TCTAGTACAGCCCCAAAAATATACCAAAAACAGCAAAACAAATTGTAAGGATACGTATAG
GCTTTGTAAAGGTAAATTGTGAAAAAAGCAGTTTTTTAAACGAATGAAACGGCTTCGGGC
TGAAATATATGCTGATGCCCTGTTCTTCCCGTATTTCTCGTGTGTTGTCAAAGTGCAGGC
TGCTTTGAAATCGGTATTGCCATCTATGAACCACCACTTTGCTTTATTTCAGCGGGCTTG
AGATGTGTATAAGAATATTGTTTTGAATAAATTTAAAGAAAATGATAATCGTTATTGACG
ATTTTTAAAGGAAAGCGTAGAGTGCCAATTCTATGAAGCAATACGGTAAGTAACAATGAA
AATATCTACTGCTTGGGTATAGAGCATATTTCACAACCCGTAACTATTCTTGCGGAAACA
GAGAAAAAAGTTTCTCTTCTATCTTGGATAAATATATTTACCCTCAGTTTAGTTAAGTAT
TGGAATTTATACCTAAGTAGTAAAAGTTAGTAAATTATTTTTAACTAAAGAGTTAGTATC
TACCATAATATATTCTTTAACTAATTTCTAGGCTTGAAATTATGAGACCATATGCTACTA
CTATTTATCAACTTTTTATTTTGTTTATTGGGAGTGTTTTTACTATGACCTCATGTGAAC
CTGTGAATGAAAAGACAGATCAAAAAGCAGTAAGTGCGCAACAGGCTAAAGAACAAACCA
GTTTCAACAATCCCGAGCCAATGACAGGATTTGAACATACGGTTACATTTGATTTTCAGG
GCACCAAAATGGTTATCCCCTATGGCTATCTTGCACGGTATACGCAAGACAATGCCACAA
AATGGCTTTCCGACACGCCAGGGCAGGATGCTTACTCCATTAATTTGATAGAGATTAGCG
TCTATTACAAAAAAACCGACCAAGGCTGGGTTCTTGAGCCATACAACCAGCAAAACAAAG
CGCACTTTATCCAATTTCTACGCGACGGTTTGGATAGCGTGGACGATATTGTTATCCGAA
AAGATGCGTGTAGTTTAAGCACGACTATGGGAGAAAGATTGCTTACTTACGGGGTTAAAA
AAATGCCATCTGCCTATCCTGAATACGAGGCTTATGAAGATAAAAGACATATTCCTGAAA
ATCCATATTTTCATGAATTTTACTATATTAAAAAAGGAGAAAATCCGGCGATTATTACTC
ATTGGAATAATCGAGTAAACCAGGCTGAAGAAGATAATTATAGCACTAGCGTAGGTTCCT
GTATTAACGGTTTCACGGTACAGTATTACCCGTTTATTCGGGAAAAGCAGCAGCTCACAC
AGCAGGAGTTGGTAGGTTATCACCAACAAGTAGAGCAATTGGTACAGAGTTTTGTAAACA
ATTCAAGTAAAAAATAATTTAAAGGATCTTATTATGAATGAGGGTGAAGTTGTTTTAACA
CCAGAACAAATCCAAACCTTGCGTGGTTATGCTTCCCGTGGCGATACCTATGGCGGTTGG
CGTTATTTGGCTAATTTGGGTGACCGTTATGCGGATGATGCTGCTGCAATTGTCGGTAAG
GATGCAAACTTAAATGGTTTGAATTTATGGATGAAAAAAGGTGTGGAAAACCTATGGGAT
GATACGGTCGGTAAAAAGACCCGTTTAGAGAAATTTGATCGGGTTGCATTGCAACATTTC
AGCCAATATGTAGATCTAATTAATGAAAATAATGGTAGATTACCTAACACTAGTGAAATT
GAGAGAAGTTACTATAAAGCCGTTACCGAAAATGGTGTTTCTTCTAGTGCAGCTATTGAT
TTAGTTATTAATCGCTCACTTCCGGATATGGCAGATGGTTATTGGGCATTAGGTTTGGGG
ATAGAAGCCGAACGTATCCACAATGAGCAAGCAGTAAATAATCCGAACGGTAGCGAAAGG
GATAATAGAAAGCAGTTAATATCTGCTTTAGATAAAGGATTTGATGGATCTTTTAAAGAG
AAGCATTTTACTTTTTTACAATCTGTGATAATGGATGTAACAAAGTTAGGTGTTGAATAT
ACAATAGATGGTTGGCAAAAAATTGGAGGTTGGGGTAATGGGATAATCAATGATTTATAT
AAAAGTGTTGTAAAAAGAGAGTGGACTGGAATATTTGAGATCGTTAATAATAACATCAAG
CAATTTAGAGATCTGTTCCCAAATCCGGAAGGCTGGATCGATGATGGTCACCAATGTTTC
GCTCCTTGGGTTAAAGAAACTAAAAAACGCAATGGCAAATATCATGTCTACGACCCCCTT
GCCCTAGATTTGGACGGAGACGGCATAGAAACTGTCGCTGCCAAAGGCTTTTCAGGCAGC
TTATTTGATCACACCAACAACGGTATCCGCACCGCCACCGGTTGGGTTTCTGCCGATGAC
GGTCTGCTTGTGCGCGATTTGAACGGCAACGGCATCATCGACAACGGTGCGGAACTCTTC
GGCGACAATACCAAACTGGCAGACGGTTCTTTTGCCAAACACGGCTACGCGGCTTTGGCC
GAATTGGATTCAAACGGCGACAACATCATCAACGCGGCAGACGCCGCATTCCAATCCCTG
CGTGTATGGCAGGATCTCAACCAGGACGGCATTTCCCAAGCTAATGAATTGCGTACCCTT
GAAGAATTGGGTATCCAATCTTTGGATCTCGCCTATAAAGATGTAAATAAAAATCTCGGT
AACGGTAACACTTTGGCTCAGCAAGGCAGCTATACCAAAACAGACGGTACAACCGCAAAA
ATGGGGGATTTACTTTTAGCAGCCGACAATCTGCACAGCCGCTTCAAAGACAAAGTGGAA
CTCACTGCCGAACAGGCAAAAGCCGCCAATCTTGCGGGCATTGGCCGTCTGCGCGATTTG
CGCGAAGCTGCCGCATTGTCCGGCGATTTGGCCAATATGCTGAAAGCTTATTCTGCCGCC
GAAACTAAAGAAGCACAGTTGGCATTGTTAGATAATTTGATTCACAAATGGGCGGAAACC
GATTCGAACTGGGGCAAAAAATCGCCAATGCGACTTTCAACCGATTGGACGCAAACGGCT
AATGAAGGTATTGCACTGACACCATCCCAAGTAGCACAACTAAAAAAGAACGCTTTAGTT
TCCCTTTCTGATAAAGCTAAAGCAGCTATTGACGCCGCCCGCGACCGCATTGCCGTGCTT
GATGCCTACACGGGGCAGGATTCCAACACACTCTATTACATGAGCGAGGAAGATGCGCTT

## Appendix A

AATATCGTCAAAGTAACCAACGATACATACGACCATCTCGCCAAAAACATCTACCAAAAC
CTGTTGTTCCAAACCCGTTTGCAGCCATATTTGAATCAAATCAGTTTCAAAATGGAAAAT
GATACGTTCACTTTGGATTTTAGTGGTCTTGTTCAAGCATTTAACCATGTCAAAGAAACT
AATCCGCAAAAAGCTTTTGTGGATTTGGCCGAGATGCTTGCATATGGCGAACTTCGTTCT
TGGTATGAAGGCCGAAGACTAATGACCGATTATGTGGAGGAGGCAAAAAAAGCAGGTAAA
TTTGAAGATTACCAGAAAGTGTTGGGTCAGGAGACCGTTGCATTATTAGCTAAAACATCG
GGTACGCAAGCAGATGATATCCTGCAAAATGTAGGCTTTGGTCATAATAAAAATGTTTCT
TTATATGGTAATGACGGCAACGACACTCTAATCGGCGGCGCCGGTAATGACTATTTGGAG
GGCGGCAGCGGTTCGGATACTTATGTCTTCGGCGAAGGCTTCGGTCAGGATACGGTCTAT
AATTACGACTACGCTACCGGACGCAAAGACATCATCCGCTTTACCGACGGTATTACAGCC
GATATGCTGACTTTTACCCGAGAGGGCAACCATCTTCTTATCAAGGCAAAAGACGGCAGT
GGACAAGTGACTGTTCAGTCCTATTTCCAGAACGATGGCTCAGGTGCTTACCGTATCGAT
GAGATTCATTTCGATAACGGCAAAGTACTGGATGTTGCCACTGTCAAAGAACTGGTACAG
CAATCCACCGACGGTTCGGACAGATTGTATGCCTACCAATCCGGAAATACCTTAAATGGC
GGATTGGGCGATGACTATCTGTACGGTGCCGACGGGGATGACCTGCTGAATGGTGATGCA
GGCAACGACAGTATCTACAGTGGCAATGGCAATGATACGCTCGATGGAGGAGAAGGCAAC
GACGCCCTGTACGGCTATAATGGTAACGATGCACTGAATGGTGGCGAAGGCAATGATCAT
TTGAACGGCGAAGACGGTAACGACACTCTAATCGGCGGTGCAGGCAATGATTACTTGGAG
GGCGGCAGCGGTTCGGATACTTATGTCTTCGGCAAAGGCTTCGGTCAGGATGCGGTCTAT
AATTACGACTACGCTACCGGACGCAAAGACATCATCCGCTTTACCGACGGTATTACAGCC
GATATGCTGACTTTTACCCGAGAGGGCAACCATCTTCTTATCAAGGCAAAAGACGGCAGT
GGACAAGTGACTGTTCAGTCCTATTTCCAGAACGATGGCTCAGGTGCTTACCGTATCGAT
GAGATTCATTTCGATAACGGCAAAGTACTGGATGTTGCCACTGTCAAAGAACTGGTACAG
CAATCCACCGACGGTTCGGACAGATTGTATGCCTACCAATCCGGAAATACCTTAAATGGC
GGATTGGGCGATGACTATCTGTACGGTGCCGACGGGGATGACCTGCTGAATGGTGATGCA
GGCAACGACAGTATCTACAGTGGCAATGGCAATGATACGCTCGATGGAGGAGAAGGCAAC
GACGCCCTGTACGGCTATAATGGTAACGATGCACTGAATGGTGGCGAAGGCAATGATCAT
TTGAACGGCGAAGACGGTAACGACACTCTGATCGGCGGTGCAGGCAATGATTACTTGGAG
GGCGGCAGCGGTTCGGATACTTATGTCTTCGGCGAAGGCTTCGGTCAGGATACGGTCTAT
AATTACCATGTGGATAAAAACTCTGACACTATGCACTTTAAAGGATTTAAAGCAGCAGAT
GTTCATTTTATCCGTTCCGGAAGTGATTTGGTGCTTAGCGCTTCTGAACAAGACAACGTA
CGTATTTCCGGATTTTTCTATGGTGAAAACCATCGTGTAGATACATTTGTCTTTGATGAT
GCAGCTATCAGTAATCCAGATTTTGCCAAGTATATTAATGCTGGCAATAATTTGGTACAG
TCTATGTCTGTGTTCGGTTCTAATACTGCTGCGACAGGAGGAAATGTGGATGCCAATATA
CAATCCGTACAGCAGCCGTTATTGGTAACGCCATCTGCATAAGGAGCCTAATTACATTCA
TGGCTTAAACTGAAAAACAGCAATCAAGTTTATTTTGATTGCTGTTTTTCTTAATATTGG
GATAAGGGTCGTATTTTAATTAACCTTAATCGGTGCACTTCTAGCAATATAGTGGATTCA
CAAAAACCAGTACAGCGTTGCCTCGCCTTACCGTACTATCTGTACTGTCTGCGGCTTCGT
CGCCTTGTCCTGATTTTTGTTAATCCACTATAATTTTCAGACGGCCTTTTGCCTTTTCAA
ATTCAAACCAATCAAACGGTTTTATTGCTTCATCGCGTTGGTCAAGGCTTTGATGTTGTG
GCGGTACATTCCGATGTAGGTGTCTGCGGGCGCGTTGCCGAGTGCGTCGGAATACAGTTT
GCCGCTGACGTTGACACCGGTTTCTTTGGCGATACGGTCAACCATACGGGTGTCCTTGAT
GTTTTCGGTAAAGACGGCTTTGATGCCTTCGCGTTTGATTTGTCGGATGATGGCGGCGAC
TTGTTTGGCCGAAGGCTCGGCTTCGCTGCTCACGCCTTGCGGGGCGATGAATTCGATATG
GTAACGTTTGCCCATATAGGAAAAGGCATCGTGCCCGGTCAGGACTTTGCGTTTGGCAGC
AGGGACGGCATTAAATGCGGCTTGTGCGTCGCTGTGCAGTTTTTTGAGCTGCATTTGGTA
GTTGCCCAAGCGTTGTTGATAATAAACTTTGCCTTCGGGATCGGCCTTTATCAGGGCTTT
GGCAACGTTTTGGGCATAGGCGGACATAAGGACGGGGTCGTTCCAGACGTGCGGGTCATA
TTCGCCGTGGTCATGGTGGTGTCCTTCGTGGTCATGATCGTGGTCGTGATGGTGTCCGCC
TTCTTCTTCGGCTTTGAGGGGTTGGATGCCTTTGGTCGCTTCGGTATAGGATACTTTGCT
TTGTTTGACGGCGCGTTGCACATCGGCAGCTTCAAGTCCTAAGCCGTTGAGCAGGACGAG
TTTTGCACTGCGGATTTTTTTAATGTCGCCACTGGTCATATGATAGGCGTGCGTATCTTG
GTTGGCTCCGACCAAACTTTGTATGGATACGCGCTCTCCGCCGATTTGTTTGGCTACGTC
GCCTAAAATGCTGAAGCTGGTTACAACCGGCAGGGGGGCGGCAGTTGCGGAGGCGGTCAG
CAATGCGGCAATAAGGGTGAGTTTGAGGTGTTTCATAACTGTTCTCCTGTGATATAACGT
AACATCTGTTATGGTAAAACAAGCCGCCTGTTTGTTCAAGCGGCTTGCGGGGTCAGGTGG
TGTGGTGGCGGTGGTTTTTGAGCCATTTGGTCAGAATGCCGCCTTCTTTGCCGAGTATGA
CGGAAAAGAGATAAAGGACGCTGCAACAGAGGATGATGGCGGGACCGGAAGGAATTTCGA
TGTGGTAGGAAATGAGCAGTCCGCTCAAGCCGCACAGCAGGGCTGTCAGAACGGATAGGA
GGATGAGTGCGCCCATATGCTTCGCCCACAGGCGGGCGGTAATGGCTGGCAGCATCATGA
GTCCGACGGACATGAGTGTGCCGAGGGCTTGAAAGCCGGATACGAGGTTCATGACGACCA
GGACGAGAAAGAGGACGTGCCAAAGCCCGCCTTTGCCGCCGACGGATTTGAGAAACAGGG
GGTCGATGCTTTCGAGTACGAGCGGGCGGTAGATGACGGCAAGGGTAATGAGCGTGAGGC
TGGAGACGGCGGCGATGAGCTGCAGGGCAGGAATATCGACGGCAAGTACAGAGCCGAAAA
GGAGGTGGAGCAAATCGACGCTGCTCCCGTTTTTGCTGACGAGGACTACGCCGATGGCGA
GGCTGCTGAGATAAAAGGCGGCAAAGTTGGCATCTTCTTTCAGGGTGGTGAAGCGGCTGA
CGAGTCCGGCAAGCAGTGCCATCAGCATGCCTGCGGCTACGCCGCCCAAACCCATGGCGG
GCAGGCTCAAGCCGGCAAACATGTAGCCGACGGCGGCACCGGGCAGGACGGCGTGGCTCA
ATGCGTCGCCTATCAGGCTCATACGGCGCATGACGAGGAATACGCCGACGGGTGCGGCAC
TGAGGGACAGGCAGAAGACGGATGCGAGGGCGTAGCGCATAAAGTCGAATTCTGCAAAGG
GGGCAAGGAGCAGGTCGTAGAGATTCATGGTTTTTCGGTTTCAGACGGCATTTATGAGGC
GCACCAGTCGGGGCTTTCCTGTTGCTGCATTTTGGCGTTGGCTTGGGCGAGGTAGGGTTC
TGTCAGAATGGTCTCGGTTGCGCCTGCCGCAATTTTTTCGCGGGCGAGCAGCAGGGTATT
GGGAAAGTAGGCACGGACTTGTTCGTAATCGTGCAGTACGGCGATGATGGCGTGTCCGCC
GCAATGGCATTTCTGCAATACGTCGAGAAGCTCGTAGGTTGTCCGTGCATCAACGGCATT

## Appendix A

GAAGGGTTCGTCGAGCAGCAGGAATTTGGCATTTTGAACCAGCATTCGGGCAAAAAGGAC
ACGCTGAAATTGTCCGTTTGAGAGATAGGCAATCTGACGGTCGGCAAACCGTTGCATTCC
GACGCGCTCCAAGGCTTCGTGAACGCGTTGTTTTTGAGCGGTATTTATCCCTTTGAAAAA
GCCGATTTCATACCATAGCCCCATTGCCGCCAAGTCGAAAACGGTCATAGGCTGGGAGCG
GTCGATATCGGACTGCTGGGGAAGGTAGGCGATGTTCTGACGGGTCAATCCGTCCAGCCG
GATGCTGCCTGTATCGATAGGCTGCAATCCCATCAAGGATTTGAGAAAGGTGGATTTCCC
TGCGCCGTTGGGACCGAAAACCGCCCACATACTATGTTCTTCAAAAGTAATGTCCACATG
GTGCACGGCAGGTCGGCGGCGGTAGCTGACGGTCAGGTTTTCGACAATGATGCTCATGCG
GATACTGCCCAAAAGTAAACGCCCCATAAAAGGGATACGGCAATCAGGGCAAGGATGAGG
CGGAAGGTCAATCCTGATAGTAAAAGGGAAGGTGTCATGATGATTTGCGGTTTTGAAAGG
GAAGGCGGTAAAGCGTTTATCGTTATATGGCTGATATGATACTGTATAACGTTTGGTCTG
TAAATTATGCTTGAATAGGCGGGAGTGATTGTTAATCAAGGTGGATGAGGGGCAGGCATA
TCGTTGACTTGCCGGCATCGCAGCAATAAGAAATGCCGTCTGAAGGTTCAGACGGCATTG
GGGGAAAACGGTTTGAATCAACCTTTGCGTGCAGGCAGTTTTTCTTTGATGCGTGCAGCT
TTACCGGTCAGGCCGCGCAGGTAGTACAGTTTGGCACGGCGTACGTCGCCACGGCGTTTG
ACTTCGATTTTTTCGACGGTCGGAGAGTACAGTTGGAAAGTACGTTCAACACCTTCGCCG
CTGGAGATTTTGCGGACGATGAAGTTGCTGTTCAGACCACGGTTGCGACGGGCAATAACC
ACGCCTTCGTAGGCTTGCAGACGGCTGCGGGTACCTTCCACGACGCGTACGGATACGACT
ACGGTGTCGCCCGGTGCGAATTCGGGGATTTCTTTATTCAGGCGGGCAATTTCTTCTTGC
TCGAGCTGTTGAATCAGGTTCATTGTTTTTTTCCTAAATTATGATTGGATTTCCCGTTGC
TCTTGCCGGATGGTTTCTAAGAGGCGGGATTCCTTTGGGATTAAAACGCGCTTTTCCAAA
AGATCGGGTCTGCGCTCCAAGGTGCGGCGCAGCGATTGTTCCAACCGCCATTCCGCTATC
AAGCCATGATTGCCGGAACGCAATACTTCCGGAACAGCCATACCTTGAAATTCTAAGGGT
TTGGTGTAGTGGGGGCAGTCCAAAATGCCGCTTGAGAACGAATCCTGTTCGGCAGACTGC
ATATCGCCCAATACGCCGGGTACGAGCCTCAATACCGCATCCATCAGCATCATGGCGGGA
AGCTCTCCGCCGGAAACAACGAAGTCTCCGATGCTGATTTCTTCATCGACGCTGCTTTGC
AGAAGCCTTTCGTCTATGCCCTCATACCGTCCGCACAGCAGAATCAGATGCGGAAGTTCT
GCCAGTTCTACCGCTTTTTGGTGTGTCAAGCGGTTTCCCTTGGGGGCTGAGGTAGATGAC
TTTTGCAGCTTGGGAGGATTGTGTTTTGGCGTGTTCTATTGCCGCATGAAGCGGCGGAGC
CATCATAATCATTCCCGGGCCGCCGCCGAACGGGCGGTCGTCGATGTAGCCCAATCTGTT
GTCGGCAAACTTTCGGGGATTGACTGCTTCAAACTGCCAGATTCCCTGTCTGTTCGCGCG
TCCCGTTACGCCGTAGCGGGTAATGCTGTCGAACATTTCGGGGAAAATGGTAACTGCCTG
GATAAGCATCAGTAGTCCAAACCCCAGTCGGCAGTAATGGTCTTGCTGCCGGTATCGACG
GTTTCGATATATTGGGAAACGAACGGAATCAGAATCTGCCCGTGTTCTCCGTCAATCATC
AATACGTCGTTTGCGCCGGTTTCCATCAGGTTGCTTACCTTGCCTAAAACGGTATGGTCT
TTGTTGACAACGGTCATGCCGACCAAGTCTGTCCAGTAGTATTCGTCTTCTTCTGTCGGG
GCGAATGCTTCACGGGGTATTTCGATGGTGTAACCGCGCAATGAGAATGCCAAGTCGCGG
TCGTTTATGCCTTCGAATTTGACTTGGAGTTCGCCGTTGACGACTTTTCCGGCTTCAAGG
GTAACGCTGATGGTTTTGCCGTCCTTGACCAAATGCCACTCGGGGTAGTCCAAAAGGCTG
TCGGAATATTCGGTGTTGGCGGCAATTTTCAACCAGCCTTTTATGCCGAATACGCCTTTG
ATGTAGCCCATGGCTACCCGGTTTTGAGTGTCTGTCATGGCGGCAAATGCGGATTAGGCG
GCTTTTTGTTCTTTAATCAGTTTTGCAACGGAGTCGCTGACTTGCGCGCCTTGTGCAATC
CAGTGGTTCAGGCGGTCTGCATTGAGGCGGACGCGCTCTTGTTTTTCGTTGGCTACGGGG
TTGTAGAAGCCTACGCGTTCGATGAAGCGGCCGTCGCGGCGGCTGCGTGAGTCAGTAACG
ATGACGTTGTAGAAGGGGCGGTGTTTCGAGCCGCCGCGTGCCAAACGGATAACTACCATT
TTGAGTCCTTTTGAGAAAATCGGATATATGGAAACTGCCGATTTTAGGTTATTTTGTGGT
CGGTGCGCAAGTTTTTATTTGTTTTTTCTGTTGTTTTGTCTGCCGCAAGGTTCAGATATG
CGCGGTACAGGTTTTTTTCGGTGTCCGATTCCTTGAGGGTAAATCCTGATTTTTCAGCAA
GTTTGATCATGGGGGTATTGGTTTTGAGAATGTCGGCACTCATAGTCCGGTAGCCTTGCT
GTGCGGCGGTTTGGATGATGAGTTCCATCATTTTCTGTGCCAGTCCGCTGCGGCGCATAT
GTTCCGCCAGTGTGATGCCGAATTCGCATTCGTTGCGATTCAGGCGGCTGTGGCGGACGA
CGGCGACGATGTTGCTGTCGGCATCCTTTGCCGTCCATGCGGCTTCACAGTGGTAATCGG
GGTTGCACAGGCGTGCCAACGTGGCTGCGGGCAGTTCGTTGGTGTGGGTCATGAAGCGTG
TGTACCGTGCTTCGGGACCGAGGCTGCGGACGAACTGCTGTTTGGCTTCTGCGTCTTCGG
GCAAAATGGGGGTAATGGTAACGGTCGTGTTGTTTCTTAGGGACAGTGTTTTGGGGTATG
CTGCGGGATAGGGGGCAAGTACGTTGGGTACGGCTGCTCCGGTTTCGGTTTTGCTGCCGA
GCAGTTCTGCGGCGGCTTCGCTTGTGTGGCGGAGAAATTCGGCGGCTGTCGGGTTTTTGT
GTTTCAGGTATGCGGCGGCACTCTGCATTTTTGCGGCGGCATGTTCGAGGGTTTGGGCGG
CTTTGCCTGTGTTCTTGCGTTTGGGCGTGTCGTGTGTTTCGGGTGTCTTTAAGAGGAAAT
CGCTGCTGTATTGTCCGCCGTTGAGGTTGAGGGTGATGCCGAGAATGTGTTGGCGGTATT
CGGGAATGACGGTCAGTGTGTGCAGGAACTGGTCGAGGGTTTGTGTGCCGTCGAGTTCGG
CAAAGCGGGCAAGGTGGCGGCTGTCGAGCGTGGTAAACGGCGGGAGTACGGCAGTGGTTT
GTCCGTTGCAGCGTGCGGTCAGGATGTCGCCATAGAGGGGGTGGCTGTCGAATTGGAATT
GTACGGCGTTATGGGTGGTGTGCCGGTAGGGGGGGAGGTGCAGGGCTTCGGCGAGCAGGG
AGGGGTTTGCCGCTGCAAGGGCTTTTTTGATGTTTTGGGGTTGCGGTGTTTTCAGACGGC
ATGGCTGCGGCGGTGCAATGTCGAGCTGTGCCTGTTTCAGGGCGGCGGCGGTGTTGCGGT
AGGAAAGGGTGCGGATTGCCTGAGTGGGGGTGTCGAAATGGGTTATGCCGTCTGAAAAGG
GGCTGCTGACGAGCAGGGGTTTGGCGGTCTGTTCGGACAGGCGGATAAGGGCGCGTGCTG
TTTTTTTGTAATCCTCGTGTCCGGAGGGACTGAGGATGGTTAGGACGGCTTGGGTGTCGG
GGTGGGCAAGCTGACGTGAGGCGATGTCGTGGCAGATTGAGGGTGTGGGTGTGCCGGTCA
GGTGTCCGTTGCGGATGTGGTGGGGAAGGTTGGGAAAGTGGAGGGTGAGGTTTTTTGGCG
CGTGCGCGTGCAGCCATTCGGCAGGCGTGTCGGACAGGATGTCGAGTCGGGACAGGGGTG
GAAGGTCGGACAGTTGGGCGCGCAGTGCGGCTTCGAGGTCGTCGGCGTTGAAACTGACGA
GGAAGTTGCAGTGTCGGGCGAGGCAGTGCAGTACGGCACGGTCGGTTTCTGTCGTGCGGC
AGGTGATGTGGAGAATCAGCGGCGTATGGCGGGTAAATTGGCGGATTGCGCTGAACAGTT

## Appendix A

TGCGCTGATCCTCTTCAGGGTTGTGGTGTAGGACGGCGGTTTTGGTGTGCAGGCTGTGTC
CGAAGCGGTTGAGCCAATCGGCGGATGTGATGGGGCTGATGCCGGGATGCAGGCTGATGT
GGCGGGATGTGCCTTGACGGAGTTTGTTCAGGATGTTGTCGATTTGGCGGCTGACGGCGG
CATTGCCGGTCAGTATGGCGGTATGGCCTGCGGCGTATCCGTCTTGGGTACTGATGTTGA
GTCCGAGTGAGGGCAGTTGGATGCCTGCGGTGGTGCAGGCGGTGATGTTGAGTCCGTTGC
CGTGGTGTTTGCGGATGGCAGTTTCGGCGGTGTGCAGTTCTGCGGCAGACAGGTTGTCCC
AGTCCTGTATGAGGATGATGTGTCGGAGCTGCTTTTTGCGGCAGGTTTTGAAGAGGGTGT
CGTAACTGTCGGGTAGGGTAACGGCAATAATCAGGTCTGCATTGCCGGGGATTTTGTTGA
GGCTGGTGTAGGCGGGCAGTCCGGCTATGGTGTGGTGGCGCGGGTTTACGGGGGTGATTT
TTCCTTGAAAGGGCGTACTCAGCAGGTTGCTGAGTACACGTTCGCCCAGGCTGTACGGTT
GTTCGCTCGCGCCTATCAGGATGATGTGGTTGGGCATGAAGAAGTAGCCCGGATCGGTTT
GTGCCGACATGATATATTCCTTTGCGGACGGTATGTGCGTGATTTTTGGAGAGACACCCG
CTGTGTGTTTGTTTTGGGGTAACTGTTTGTGCAATGCCGTCTGAAGCCGGTTCAGACGGT
ATTATGGTCAGTTCGCACTTTTTTCTGTTTTGGAACCGGTTTTTTTCTTGGGCAGGATAA
AGCGCATCCGCAGACCGTTCGGTTTGATGTTTTCGGCGATGATTTTGCCGCAGTGCTGTT
CAATAATATGTTGGGTCAATGCAAGCCCCAGTCCTGTTCCGGGTTTGTTGGCACTGGAGT
CTGCACGGTAGAAAGCGGTGAAGATGTGCGGGAGCTGCATTTCGTCCACGCCGGGGCCGT
TGTCGGTAACGTCGATTATCCAGTGTTTGTGGTCTTGTCCGATGTTGATCAGGATGGTGC
TGCCTTCGGGACTGTAGTTGACGGCGTTGCGGATGACGTTGTCGAAGGCGCGGTACAGGT
AGCTTTCGTTGGCAAGGATGGTTGTGTTTTCGGGGATTTTTCCGTCGGCAGACAGGGTAA
CCGTTTGTCCGTTTTTCTGGGCAATGCTTTGATTGTCTTCTACCAGGTTGCCCAGGAAGG
GCAGGAGTTTCAGGCTTTCTTTTTCCAAAGCCATATTGGAAGTTTCGAGACGGGACAGGG
TTAACAGTTCCCCGGCCAGCGTATCCATGCGGGTCAGTTCGCCTTCCAGCCGTTTGAGAT
ATTGCTCCTGTTTTTGGGGCTGCGCCTGAATCAGTCCGACAATTGCCTGCATGCGCGCAA
GGGGAGAACGCATTTCATGGGAGACGTGATGGAGCAGGTGGCGTTCTTTGGCAACGAGTT
TTTCGAGTTTTTCCACCATTTTGTCGAATTGGATGGCAAGATGGGACAATTCGTCGTCGC
GGTCGTCGACCTGTTGGGAGATACGGGTTTCAAGTTCTCCGTTTGCCACCCTGTCCATGC
CGTTGCCTAAGATTCTGATGGGTTTGGCAATGTTGCCGGCGAGGATATATGCCATCAGCA
GTCCGACGATGATGATGAAGGACAATATGATGAGTTCGTGCCAAATCGGGGCGAGCGGCA
GGCCGGGGATCAACAGGGGGCTGGGCAGGCGGCGGGCTTGGAGTTTGTCCCAGTCTTTGG
TGAAGAACAGGTATTCTTCGCCGAAGCGGTCGTATTCGATATGGACGAGGTTGGAATGCG
GGTGTCCGGCGGCGAAAAGCCGGGCGCGTTCGATGGTATAGCTGTCGATATACCGGTTCA
GGATATCTTTTTTCTCGTCGCCCTGTATAACGTACACGCCCGATGAGACGGGGCTGTCTT
TCCATTCCGTCAGGATTTCGCGCGCACCCGCGTCCCCGCGTGCCCGGAATGCGGAAATGA
TGCTGCCCATCAAAGTGGTTTCGATGGTGCGGCGTTGGTTGAACTGGTTTTCGGCAAGGG
TGTTCTGCACCAGCCAGAAAGAAAAACTCGCCACAAAGATTGCACAGACGATAACCGCGC
AAAATGTGGCGAAAATGCGTTGGAACAGTTTCATTTATCTGTTTATTTCAGTTTTTGACA
AACAGGTAGCCCAAGCCGCGTACGGTTTGAATCAGAGAGGCATCGCCCAACTTGTGGCGG
ATGCTGGAGATGTGTACGTCGATACTGCGGTCGAATTTTGCCAGCTTGCGGTCGAGTGCT
TCGACGGACAGGGTTTCTTTGCTGACTACCTGTCCGGCATGGCGCATCAGGACTTCGAGC
AGGTTGAATTCGGTGCTGGTCAGTTCGAGCGGCATGTCTTTGACGGATGCCTGGCGTTTG
GCGGGGTACAGGACGACATCGCTGACGGAGATGCTGTTGGGTGCGTTGTTCTGTTCGCCG
CTGTGTTGTGCGCGGCGCAGGATGGCATTGATGCGTGCCAAGAGTTCGCGTGGTGTGCAG
GGTTTGGGGACATAGTCGTCCGCGCCCATTTCCAAGCCGATGATTCGGTCGATGTCGTCG
CCTTTGGCGGTCAGCATGATGATGGGGACGGTGCTTCGGGCGCGTACGTTTTTCAAGACA
TCCAAGCCGTTCATTTTGGGCATCATGGAATCCAATACGACTACATCGTACTGCCCGCTC
AGGATTTCCTGTACGCCTGCTTCCCCGTCGGGAACGCTGCGGACGTTCAGACCTTCGGCG
CTCAGGTATTCGGTCAGCAGTTCGGTTAGCAGGGCATCGTCATCTACGAGTAATACGCGG
CTCATGGTGTTTCCTTTTCGTAAGGGTATGCCCCGACCCTGTTTCGGGCGGGGCGTGAAA
AGATTGTTTGACGGTTTATCTTAACACGGCTGCAATGTTTTTTGATAGCGTATTTCCCTA
CCGGTTTGCTGTTTTTTGCAATGTCTTGCATGGAGCTTTACATTTCGGGCGGTATCCGCA
TCCGCCGGCGCGGGTCATTTGCAGGGTTTTGCTTCCGGATGACCGGGCGCGGCGGCGAAG
GCTTTGCAGTCTTTGAGCAGTTCGGGTAGCAGCGGCGCCCATACGGGCAGTTTGCGGATT
TCGTCGGCGTATCGGGGCATCAGGTAGGGGTAATAGGACTGTGTCGCCCGCATCCATTGT
TTTGCTTCTGCAACTTTGCCTTGCCGCATCAGGTAGAGGGCGATGCGGTAGGTGGCGGAG
TGGGGGCGGTATTTTAGTGATTTGAGGGTTGCTTCTTCCGCCCAAGTCTGGGTTTCGGGG
TATTCCGGCAGGGCGAAGTTTACGAGGGAGAAGTCGGCATAAAAGGACAGCATCGGACTG
TTTGCGGAAATATAGCGCAACTCGTTGATTTTCCGGTTGAGGGTTTTGGCACTGTCGTCA
GTGGCGGGGGAAAAGGCGTTAACCAGCCGGGTGTATGTCCAGTCCAAGTGCAGCAATCCT
GCGAATATGGCGGCGGAGGCGGTCAGTATGCCGAGATTGGCGGCTTTTTTGAAGGCGATG
CCGTCTGAAGCCTCTGCGGGGGACAGGAAGAGCATCAGTCCGAAAGGGATGAGGAAATAG
ACATACCACAAAGGATATTCGAGCATACTGTGGCACATACTGACGGCAAGCGTGCAGATT
AGGAAAAGCGATGCGGGGGTCAGGGGGCGTTTAAGCAGCCCGGCAATGCCCGTCAGCAGG
GTTGCGGCAACCAGAAGCGTGCCGCTGATTCCCATCTCTGCAAGGAGTTGGAGGACGATG
TTGTGGGAATGGGTGAACAAGTTGCTGAGGAGGTTGTCGTATATGTTGTGCTGTTCGGCA
TTGATGAGGAAGGTTTGTTGGGCAAAACTGTTCCAGCCGTGCCCGAATATCGGGGCGGAC
TGGAAGGCGGCAAGGGCTTTATTCCATTCGATTTGGCGCGGCAAGTCTGTGAAACCGCCG
TTGGCGACGCGTTCGACGGCAGTTTCGTAGCGGATGCCAGTAAAGGTTTCCAGAATGGTG
TTCATGGAAAATTGGAACAGCGCGGTAAGGAATACGGCTGCGGCTATGCCGAGCATCGTC
CGCCTGTTGGATTTGTCCGAACGGAAATACCAGAAGGGAAGGATGAGGGCGATGGCGGCT
ATGTAGGTCAAGATGGTGCGCGAGTTGACCAAACCTAAAACGGCGGTCTGCATAATCAGG
CAGATTACGCCGAGGGCGGCGGGGATTTTTCGTTGTCCGTTGAGGTAGGCGGCGGCGAGT
ATGCCCCACATGAGGTAGTGTCCGAGGTTGTTGCGCTGCCCGATGTGTCCGATTACGCCT
TGCCCGCTGTAAACGATGATGTTTTGAAACAGAGGGGTGTCTTCCCAGCCGGCAAACTGG
ATGACGACGATGCAGGATTGAAGCAGGGAGCCGATAAGCAGCGACCAGGCAAACAGGGTC

## Appendix A

ACGATGCGTTCTTGTCCGAAGTGTGCGACCAAGCTCCGGCAGGCCCACGCGCTGACGGCG
AGCAAGATGAAAATCCAAGAGACGATGTCGTTCATACCGGGGTAAATCAGGTTCATCAGG
CGTGCCTGAAGATACCAAAACGCCGCCATTGCAAACAGAAGGAAGCTGATGGCGGGGATT
TTGACATCAAACAGTTTTTTTCCTGCCGTGAGGAACAACAGGACAATCAGGCCGGCTGCG
GCGGCGGCATCGTGGTAAAAGTCGGGCGACGGTTTCAGTTTGAGCGCGAAGGTAAAGGGG
ACGATGCCTATCCAAAGGAAGCAGGGCAGGATGTAAATCGGCAGTTTGGCGGCGGGGTGC
GCGCCGGATACGGTCGTTTCAGCGGGCATTGTTTGTTTCCTTGTATTGTTTGACGAACGA
CAGGCAGGATATGAAGAAGATGATGCTGAATACTGCGGAAAGCGCGGCGCAAATCTGTTC
TGCGGGATAGGCGTCGAACAGGCGCATGACGGCTTCGGCAAAGTAAATCAGAACCAGCAT
GGAGCTGTATTGGTAAGTATAGATTTTCTTTTTCAAGATGCCTGAAAGCGGCAGACAGAG
GGGGAGGGCTTTGAGCGCGAGCCACGAGCCGCCCGGGCGCAACGGTGCAATCCACAGTTC
CCAGGAAAGGGACAGGATTATCAGTGCGATCAGGCTGAAAGAGGCAAGGAGGTAAGCGGT
TTGTCTGTTCACGGCGGTCTTTACGGTTTAAGGGCGGACAAGGGGGAGCGGTATCCCAAA
TCCTGCAACATCGAAACGGTTTCATAAACGGGCAGTCCCATAATGCCGCTGAAGCTGCCT
TCGATAGATTGGATAAAGATGCCGCCTATGCCTTGTACGGCGTAGGCACCGGCTTTGTCC
ATCGGCTCGCCGCTTTGCACATAGGCGGAAATTTCTTCCGAACTCAGGGGCTTGAAAACG
ACGCGGTTGGTTTGGACGCGGCTTGACGTTTTGCCGCGATAATGAATGCAGACAGCAGTC
AGGACGGTATGTTGTTTGCCGGACAATCGGTTTAAAAATTCGATTGCTTCGGCTTGGGAG
CGGGGTTTGCCCAATATGATGCCGTCTGAAACGACGCAGGTGTCGGCGGTAATCAGGGGG
AAATCGGGCATTGTGCCGTTGGTTTCGCAAAAGAGGGTCAGGGCGGTTCGGTTTTTTTCT
TCTGCCATCCTTTGAACGTAAGCGAAAGGTGTTTCGCCGGCTTTAACGGATTCGTCGATG
CCGGCAGGCAGTTGGATGACGCGGTAGCCCAACTGTGTCAGGATTTCCATTCGGCGCGGG
CTGTTTGAACCTAAATAGAGGGTATTCAAAGGTATTCCTTAATCTGTTGCGGTATGAGGC
GGAGGTTCGGACGGCATAGTGTCAGGTTGTTGCAGGCGGCCGTATGTCGCCATCCTGTTC
TGAACGTGGCGTGAAAAAGCGTCCGAACCAAATACCTGCTTCGTATAAGAGAATCAGCGG
AATGGCAAGCAGGGTTTGTGAAATCACATCGGGCGGCGTGATGATGGCGGCAATGACAAA
CGCGCCGACAATCACATAGGGGCGGGCGCGTTTGAGCTGTCCGGTTGTTACCACACCAAT
TTTGGTTAACAGGATAACGACAATGGGGACTTCAAACGTTGTGCCGAACGCAACAAACAT
CCCCAAGATGAAGGAGAGGTATTTGTCGATGTCTGTCGCCATATTGACACCGACAGGGGT
AACGCTGGCAAGGAATTTGAAAATGACGGGGAAAACCAAAAAGTAGGCAAATGCCATGCC
GATGAAAAACAGGCTGACGCTGGAGAGGACGAGCGGCGTAATCAGGCGTTTTTCGTTTTG
GTAGAGTGCGGGCGCGACAAATGCCCAGATTTGGTAGAGCGTATGCGGCAGCGAAATTAA
AAATGCCGCCATCAGGGTAACTTTGACCGGCACGAAAAATGGTGCGATGACATCGGTGGC
AATCATGCTGGTGTCTTTGGGCAGGTTTGCCATCAGCGGGTCGGCGATAAAAGTATAGAG
TTGTTGGGCAAACGGCATTAGGCCGAAAAAGCAGACTAAGATGCCGACAACCGTCCACAT
CAGGCGGCGGCGCAGCTCGATGAGATGCTCGACAAGCGGTTGGACGGGTTGTTCGTTTTG
TGTTTCGGACACCGGATTGCTCTCTTTATGATTTACGGACGCGCAATTTAGGTTTGGCGC
GGTGTTTCGGACGAAAATCGCGTTTGCGGCTTATTGCCTGTTTGCGCAGGGAAGTGGTGT
GCGGAACAGGCGTTTCAACAGCAGTATCGATATAGCTGACTTCGACGGTCTGTACGACGG
GTGCGGCGGCAGAAGCAGTCAGGTATTCCCGCCATGCGCGGTCTTGGTCGGTTTCCGCGG
GTTCGGCTGTACTGCCGGTTTGCCCGCTGTCCCCAAGGGTTTCGGCGGAAGCGTAGGAAC
GTTCGGACGGCATAACGTCGGAAATGCCGTCTGATAGGGTGTTTGCCGCATCGGGAAGCG
GATTGCCGTTTTCATCGACACCGAAATCGGCAGGTGTCCGCTGTTCGGGCAGTTTTTCCC
AAGGCTTCAGACCGTCGGAAATGTCGTGCAGATTGCCTTCCATATCCGTACCGGTTTCTT
TGAGGCTGTCTCGAACCTGAGCGGCGGCAGCTTCAAATTCCTGCTTTGCCTTCCTCAGTT
CTTCCAGTTCGATTTGAGTGTCAAATTCCTGTTTGACGCTGCCGACAAAGCGTTGCAGCC
TGCCGATGAGCCGTCCGGCGGTGCGGGCGGCCTCGGGCAGGCGTTCGGGGCCGAGGACAA
TCAGGGCGATAATGCCGACAAAAACCAGCTCGCCCAAACCGAAATCAAACATAAATTACG
CTTTGTCTTCGTCTTTTTTGTGTTCGATTACATGGTCTTTTTGGCTTCTTTGCCGTCTG
TACCTTCGTTCAGCCCCTGTTTGAAGTCATGAACCGCACCGCCGAGGTCTTTGCCGACGT
TGCGCAGTTTTTTGGTGCCGAATATCAAAACGACGATAATCAGTACGATAATCCAGTGCG
TCAGAGAAAAACTGCCCATGATGTATCCTTAAGTAAGTATTAGGGGTTGATTGTGAAATA
ACGGTTTATACGGGTGTACCCATGATGTGTATATGCAGGTGGAAGACCTCTTGTCCGCCG
CCTTTTCCGGTATTGATCAGGGTTTTGAAGCCGTCTGCCAGTCCTGCCGCTTTGGCGATT
TCGGGAACTTTCAACATCATTTTGCCCAGCAGCATCTGATGTTCGGGCGCGGCGTGTGCC
AACGAATCGAAATGGACTTTGGGAATCAGCAGCAGATGAACCGGAGCAGCGGGGTTGATG
TCTTTGAAACAAACCATTTCGCCGTCTTCATAGACGGTTTGCGCCGGAATGTCTTTGGCG
GCGATTTTGCAGAAAATACAGTTGTCCATAACGGCTCCGATGCCGTCTGAAAAGCGGTCA
GACGGATTGAATGTGGGAAAGTGCGGATTTTAATATAAATTCAAGATTCTGTGCGAGCGG
CTTTTTCGACCAGCCCCGACAGCCCCTGACGGCGCGCAAGTTCGTCCAATACGTCTTCCG
CCTTCAGGTCGTGGTGTGTCAGAAGAATCATGGTGTGAAACCATAAGTCGGCAACTTCGT
AAACCAGGTGGGACGGGTTTTTGTCTTTGGATGCCATCAACACTTCGCCCGCCTCTTCAA
TCACTTTTTTTAGGATTTTGTCTTCGCCCTTATGCAAGAGCTGTGCGACGTAAGATTCGG
ACGGATTGGCAGATTTTCGCTGGGTGATGGTTTGTTGGATGGCGGATAGTACGGAATCTC
CCATGATTTTCCTTCTGTTTGTTTCTGTTTGTTCGGAATGATAGGCTAAACGGCTGCTCT
CGGGCAATACGCCTGTTGCGCTTCGTTGGAAAATGCCGTCTGAGCGTTTCAGACGGCATT
TGTGCTGTTGCAAATGTAATTTGCTTACAGGTTTGGACTCACAATAATTTTAACGGCGGA
TTCGTTGTTGTGAATCAGACGCTCGAAGCCTTTGGAAACCAGCTCGTCCAGCTTGATGCG
CTGGGTGATGAAAGGCTCAAGGTTGATTTTGCCTTCTTCGACCAGTTTGATGGTTTCGGC
GTGGTCGTTGCAGTAGGCAATCGTGCCGCGCACGTCCAACTCTTTCATCACGACGCTGTG
GACGTTGATGGTGGCGGGGTGGCTCCAGATGGATACGATAACCAAATTGGCGGCAGGTTT
GCAGGCTTCGACCAAAGTATCCAACACTTTGTTGACGCTGGTGCACTCAAATGCCACGTC
CACGCCTTCGCCGTTGGTCAGTTTTTTCACTTCTGCAACAACATCGACTTCGGACGGGTC
GAGGATGTAGTCGGCAACGCCGGATTCGCGCGCTTTGTCTTTGCGTGCTTTACTCAACTC
GGTGATGATGACTTTGATGCCTTTGGCTTTCAACACGGCAGCCAACAGCAAACCGATCGG

## Appendix A

ACCTGCACCGCCGACCAATGCGACGTCGCCTTCTTTCGCGCCGCTGCGTACATAGGCGTG
GTGTCCGACAGACAGCGGTTCGATCAAAGCGGCTTGATCCAACGGGATTTTGTCGGAAAT
CGGATGCACCCAACGGCGTTTGACGGCGATTTTTTCGGACAGACCGCCGCCGCAGCCGCC
CAAGCCGATAAAGTTCATATCTTTGGAGAGGTGGTAGTTGCTGCCTTCTCCGGTCGGTAC
GTCATCGCGGATGATGTAGGGTTCGACCACGACGTGTTGGCCGACTTTGATGTCGTCCAC
GCCTTCGCCGACGGCATAGACCACGCCGGAGAACTCGTGTCCCATCGTTACGGGTGCGGA
CTCGCCGGAAATCGGGTGCGGATGACCGCAAGGCGGAATGAAAATCGGGCCTTCCATGAA
TTCGTGCAGGTCAGTACCGCAGATGCCGCACCAGGCGACATTGATGCCGACAGTGCCGGG
GGCGACGGTCGGTTCGGGGATGTCTTCGATGCGGATGTCGCCTTTGTCGTAAAAACGTGC
TGCTTTCATTGTAACGCTCCTTGTTTTCAAGTAGGAATACCGTCTGAATCTGGCAGGCGG
CGGTTGAAATGGGAATGGCGTGAAGAAGCTTGACCGTTTCCAGTTGAATCTGTTTAGATA
TTTTACTACAAGAGGAGACCTTTGCAATAACATAGGTTACTAAAATTTTATGCTCAATCT
CATTTTCAAAATGCAAAACTTTTCTGATTTTTCCTACTTTTTGCTCAATATTAGGAAGGT
TTTAGGCAATTGAAAATTTTTTGGCGCATTTTTATGCGTCAAATTTCGTTAACAGACTAT
TTTTGCAAAGGTCTCAAGAGATGTGTTTAAGCACGCGGAAGGCTTTCTGTTTGCGTCAGG
TCAAATAATGATGTCGTCTGAAAACCGAATCGGCTTCAGACGGCATTTATAGTGGATTAA
CAAAAACCAGTACGGTGTTGCCTCGCCTTAGCTCAAAGAGAACGATTCTCTAAGGTGCTC
AAGCACCAAGTGAATCGGTTCCGTACTATCTGTACTGTCTGCGGCTTCGTCGCCTTGTCC
TGATTTTTGTTAATCCACTATATGTCGTAACGGTCGGATTGGGTAGGTTGGCGCACCTGT
CCGGTTTTCGGTTTGGCAAACCGTTTTTTTGTTGGGTCCAGTGTTTTCTGATAGGCGGTT
GCGGCATCGGATTTGCCCAGCCCTGCCAGCACGCGGATATGCTCGGCAGCAGATTGTGCC
AGAGGTTCAAGGGTGTAGCCGCCTTCGAGTACGGATATGATTTTGCCGGGGCAGCCCGAT
GCCGTCTGAATGATTTTGTGTGTCAGCCAGGCAAAATCCGCCTCGTGCAGGTTGAGCCTG
CCCGATTCGTCTAGACGGTGTGCGTCGAATCCTGCCGACAGCAGCACCAGTTCGGGTTTG
AATGCGGCAAGTCGGGGTAGCCACTGCCTGCGGACGGCTTCGCGGAATGTGCGGCTGCCC
GTTCCTGGCGGCAAGGGCAGGTGCACCATATTGCCGCCGTCGGGCATATCGTTGTTTTCG
GGGAAGGGGAAAAGGTCGGTTTCAAACAGGTTGAAAAACAGGATGCGCGGATCGTCTTTG
AATATTTCTGCCGTACCGTCGCCGTAGTGGACATCGAAATCGATGACGGCAATGCGTTTC
AGGCGGTATTCGGCAATGGCATGCATGACGCCGGCGGCAACGTTGTTCAGCAGGCAGAAT
CCGCCGGCTTTGCCGCTGCCCGCATGGTGTCCGGGCGGGCGGGCGGCGCAAAAGGCATGC
CATGCTTTACGGTTCATGACCATGTCGACTGCCTGAACTGCCGAACCGGCGGCAAAGCGT
GCGGCAGACAGCGATCCTGTGCTGATTGCAGTGTCGTTATCCAGGCGGGAAATCTTGCCT
TTTTGGGGCAGGCAAGATTCCAAACGGTTCAGATATTTGCTCGAGTGGACAAGTGCGAGG
CGCGTATCGCTGATTTCTTCCGCCTCTATGGTTTGGAGGTGCTGCCAAATACCGGCGCGG
CGCAATGCCTGCTCGATGCAGAGGATGCGGTCGGGCGAATCGGGATGGTTTGCGCCGGGT
TCGTGCCCGGCACAGGCGGGATGCGAAATCCATGCGGTGCGGGCGTTTTTGCCCAAAAAA
AGGCGCAACAGTGCATAGAATTTCAAGATTAGGCGGGTCAAGGACATGGGTTTGTGGACG
GGCAGGCTGCGGTATACGGTCGGTACGGACGGCAAACCCGATATATTGTTTACGGTCTTA
TGTTATTATATACCCCTCTCGATTTTCAACCATATTAGAAAGAACGGATAAATTATGAAT
CAAGCTGTTGCACAATTTGCTCCTTTAGTGTTGATTATGGTGGTGTTCTACTTCCTGATC
ATGCGTCCGCAGCAAAAGAAATTCAAAGCGCATCAGGCAATGCTTGCCGCCTTGAAAGTC
GGCGACAAAGTGGTCTTGGCGGCAGGTTTCAAGGGTAAGGTAACCAGAGTCGGCGAACAG
TTTTTTACCGTGGATATCGGACAGGGTACAAAAATCGAGGTCGAAGTGGAACGCAATGCG
ATTGCCGCAAAAGTCGATTGATTTGTGCCGACAAGCCGCATCTGGAAAGCCCGAATGCGG
CACTTTGTTTTGAATTCCAACCGAAGGCTTGACCATGTTCCGACACGCAGGGCGGCATAT
TCAGGATGCCGCTTTCCGGTCTTGCCTGGCTGGGAAGGGTTTTTGCCTCTTCTGAAATAG
CCCGATTCCGACACCACCGAAAGGGTGGGGTTCCAACCATTAAGGAACAATGATGAACCG
TTATCCTTTATGGAAATATCTGCTGATTGTGTTCACGATTGCGGTTGCCGCAGTGTATTC
GCTGCCCAACCTATTCGGCGAAACACCCGCCGTGCAGGTATCGACCAACCGACAAGCCAT
~~CATCATCAACGAACAGACTCAATTCAAAGTGGATGCCGCGCTGAAAAACGCAGGTATTCA~~
GACCGACGGGATGTTTGTTGTGGACAATTCACTGAAAGTGCGTTTCAAAGACACAGAAAC
GCAGCTTAAAGCGCGCGACGTCATCGAAAACACTTTGGGCGAAGGGTATATTACCGCGCT
CAACCTGTTGGCGGACAGCCCCGAATGGATGGCGAAAATCAAAGCCAATCCGATGTTTTT
GGGTTTGGACCTGCGCGGCGGCGTGCATTTCACCATGCAGGTCGATATGAAAGCGGCGAT
GCAGAAAACGTTTGAACGTTATTCGGGCGACATCCGCCGCGAACTGCGCCGCGAAAAAAT
CCGCAGCGGCACGGTGCGTCAGGCTGGAAACAGCCTGACCGTCCCTTTGCAGGATGCAGG
TGATGTGCAAAAGGCTCTGCCGCAGTTGCGCAAGCTGTTTCCTGAAGCAACGCTGAATTC
AGACGGCAGCAATATCGTCTTGACGCTTTCGGAAGAGGCGGTCAATAAAGTGTGTTCCGA
TGCGGTCAAACAGAACATCACTACCCTGCACAACCGTGTGAACGAGTTGGGCGTGGCCGA
GCCCGTCATCCAGCAGTCCGGTGCAGACCGTATCGTCGTGCAGCTTCCGGGCGTTCAGGA
TACTGCCAAGGCAAAAGACATCATCGGCCGTACCGCGACTTTGGAATTGCGTATGGTGGA
GGACGATCCTGCCAAGTTGCGCGAGGCATTGGAAGGCAACGTGCCGAGCGGTTATGAGCT
GCTTTCAAGCGGCGGAGATCGTCCCGAAATTCTGCTGATCAGCAAACAGGTCGAGCTGAC
GGGCGACAACATCAACGATGCGCAACCGAGTTTCGACCAAATGGGCGCACCTGCCGTCAG
TCTGAGCTTGGACAGCGCGGGCGGCAGCATTTTCGGCGAACTGACTGCCGCAAATGTCGG
CAAACGCATGGCGATGGTTTTGATCGACCAAGGAAAATCCGAGGTTGTAACCGCGCCGGT
TATCCGTACTGCCATTACCGGCGGACGCGTGGAAATTTCCGGAAGCATGACGACAGCCGA
AGCCAATGATACGTCTTTGCTGTTGCGTGCCGGTTCTCTTGCCGCACCGATGCAGATTGT
CGAAGAACGTACCATCGGTCCGTCTTTGGGTAAGGAGAACATCGAAAAAGGCTTCCATTC
GACTTTATGGGGTTTTGCCATCGTTGCTGCATTCATGGTGGTTTACTATCGTCTGATGGG
TTTCTTTTCTACCATTGCATTGAGTGCCAACATACTGTTCCTAATCGGTATTTTGTCTGC
CATGCAGGCAACGTTGACGTTACCGGGTATGGCCGCGCTGGCGTTGACTTTGGGTATGGC
AATCGACTCCAACGTCTTGATTAACGAACGTATCCGCGAAGAATTGCGTGCCGGCGTGCC
GCGGCAGCAGGCAATCAATCTCGGTTTCCAACACGCATGGGCGACCATTGTCGATTCGAA
CCTGACTTCGCTGATTGCCGGTATCGCGCTTTTGGTATTCGGTTCCGGCCCGGTACGCGG

## Appendix A

TTTTGCGGTCGTACACTGTTTGGGTATTCTGACTTCGATGTATTCATCCGTCGTCGTATT
CCGTGCGTTGGTCAATCTGTGGTACGGACGCAGACGCAAATTGCAGAATATTTCCATTGG
TTCGGTGTGGAAGCCGAAAGCCGAAATGGCAGGAGGCAAGGAGTAAGCTATGGAACTCTT
TAAAATCAAACGCGATATTCCGTTTATGAGCTACGGCAAACTGACGACCTTCATTTCGTT
GGTTACGTTTATCGCTGCCGTGTTCTTTTTGGTTACCAGAGGTCTGAATTTCTCTGTCGA
ATTTACCGGCGGTACGGTAATGGAAGTCCAATATCAGCAGGGTGCGGATGTCAATAAGAT
GCGCGAACGCCTCGATACGCTGAAAATAGGTGATGTACAGGTTCAGGCATTGGGTACGAA
CAAACACATCATGATCCGCCTGCCGAACAAAGAAGGTGTTACTTCCGCACAGTTGTCCAA
TCAGGTTATGGATTTGCTGAAAAAAGACAGTCCCGACGTTACCTTGCGCCAAGTCGAATT
TATCGGCCCGCAAGTCGGTGAGGAATTGGTAAGTAATGGATTGATGGCTTTAGGTTTTGT
CGTTATCGGCATCATTATTTACCTGTCGATGCGTTTTGAATGGCGTTTTGCCGTATCTGC
CATTATCGCCAATATGCACGACATCGTGATTATTCTCGGCTGCTTTGCCTTCTTCCAATG
GGAATTTTCGCTGACCGTCTTGGCGGGTATCCTTGCCGTATTGGGCTATTCTGTGAACGA
ATCCGTCGTCGTCTTCGACCGTATCCGTGAAAACTTCCGCAAGCCGGCGATGCGCGGACA
TGCCGTGCCGGAAGTCATCGACAACGCGATTACCGCAACGATGAGCCGCACCATCATTAC
CCACGGTTCGACCGAGGCGATGGTCGTATCCATGCTGGTGTTCGGCGGTGCGGCCTTGCA
CGGCTTTTCTATGGCGTTGACCATTGGCATCGTGTTCGGCATTTATTCTTCCGTATTGGT
TGCCAGCCCGCTTCTGCTAATGTTCGGTTTGAGCCGCGACAATATCGGTAAAGAACCGAA
GAAGAAAGAAGAAATCGTGGTTTGAAGCGCATATGCCGTCTGAACATTGCCGTCTCAAGC
AGACAATGCTTCAGACGGCATTTTTAACGGTTACTTCCACGGTCTTAAAATATTGTGCAG
AAATGCGGGAATTGTGTCATAATGCCACGTTGTCCTATCTTGGGCATAGGGAGTTTGCCG
TTGTCTTCAGGCTTGGCAAACTTGTCTGAATCCCTATGGGGATTCTTATATTTTTGGAGT
TTTCATTATGGCACTGACCGTAGAACAAAAAGCACAAATCGTTAAAGATTTCCAACGCAA
AGAAGGCGACACCGGCTCTTCCGAAGTACAAGTCGCTCTGTTGACTTTCCGCATCAACGA
CCTGACCCCCCACTTCAAAGCCAACCCCAAAGACCACCACAGCCGTCGCGGCCTGTTGAA
AATGGTCAGCCAACGCCGCCGCCTGCTGGCCTACTTGCGCCGTACCCAGCCCGATACGTA
TCGCGCGTTGATTACCCGCTTGGGTCTGCGTAAATAATTACGCTTTCCGACACCGCCCAG
AAAAATGGGCGGTGTTTTCTTTTCTGTTGCTTTCCGACAAGCTCAAATCCATATTTATAG
TGGATTAAATTTAAATCAGGACAAGGCGACGAAGCCGCAGACAGTACAAATAGTACGGCA
AGGCAACGCAACGCTGTACTGGTTTAAATTTAATCCACTATATTGCCCGAAAACCGCATA
AACTAATATAATATAAAGTTCTTTGGAATCTTGTTCCATTTCATGCTGCCCGTGCGCTTT
ACAAGAGTTTCAGACGGCATCAAACGTTTAACTCCCGCCAGCAATCAAACAGCTTTTTAT
CACCCATTCGAAAATCCGTTTTGCCGGTACTCGTCTTTTTATTGGAGTATTGCCATTATG
ACCGCAACCACTGCGTCTTCAGCCAAACCTTATCTCAAAATCCAAGGTTTGGTGAAAAAG
TTTGGTGACAATTACGCTGTCGATAACATCGACTTGGACATTTATCAACACGAAATCTTC
GCCCTTTTGGGCAGTTCCGGCAGCGGAAAATCTACACTGCTGCGTATGCTGGCGGGTATG
GAAAGTCCCAATCAGGGAAAAATTATCCTTGATGGTCAGGATATTACCAAACTTGCACCC
TATGATCGCCCCATCAATATGATGTTCCAAAGTTACGCGCTTTTTCCGCATATGACCGTA
GAACAAAACATTGCCTTCGGTCTGAAACAGGACAAAATGCCTAAAGGCGAAATCGCCGCG
CGCGTCGAAGAAATGCTCCGCCTGGTTCAGATGACCAAATTTGCTAAACGCAAACCGCAC
CAATTGTCCGGCGGTCAGCAGCAGCGCATTGCTTTGGCACGCAGTCTGGCAAAACGTCCG
AAAATTCTACTGCTGGATGAGCCCCTCGGTGCATTGGACAAAAAACTGCGCCAACAAACC
CAGCTTGAGTTGGTCAATACGCTGGAACAAGTCGGCGTAACCTGTATTATGGTTACGCAC
GACCAAGAAGAGGCGATGACGATGGCGACCCGCATCGCCATTATGTCTGACGGTCAGTTG
CAGCAAGTCGGCACACCCAGCGACGTGTACGACTATCCCAACAGCCGCTTCACTGCCGAG
TTTATCGGCGAAACCAACATCTTTGACGGTGTGGTGATTGAAGATCATGCCGACTATGCC
GTTATCGAATGCGAAGGTTTGGAAAACCACGTCCGCATCGATCACGGTTTGGGTGGTCCG
AGCGAGCAGGACCTTTGGGTTAGTATTCGACCAGAGGATATTGATTTATATAAAGAAAAA
CCCGAATATTTGGGCGACTACAACTGGGCGAAAGGCACGGTAAAAGAAATCGCCTATTTG
GGCAGCTTCGCCATTTACCATATCAAGCTCGGCAACGGGCGCGTCGTCAAAAGCCAAGTC
CCCGCCCCTTACTGGTATGTGCGCAACATTACACCGCCGACTTGGGACGAAACCGTCTAT
ATCAGCTGGCCGGAAAACCAACCGACTCCGTTGTTCCGTTGATTTAAGGGGAATGCAATG
AACCTTAATAAACTGAAAAACAAACTGTTCCGCCGTCCGGGGCAGCGTGCGGTGATTGCC
GTACCGTATATTTGGCTTTTGGTGCTGTTTCTGATTCCGTTCGCCATCGTGCTGAAAATC
AGCTTTGCCGAACAAGAAATCGCCATCCCGCCGTTTACTCCTTTAACGACGATAGATGAG
GATTTGGGTCGTCTGAATATTGCTGTCAGCTACCAAAATTATGCAGACATCTTCCAAAAT
TTTTGGAGTACGCTCAATCCGTTCGGCGACGGTGAAAACAGCAATATCTATCTGATGACT
TATTGGTCTTCAATTAAGACTGCGCTGACTACGACGGTAATTTGTCTGTTGGTCGGTTAT
CCGACCGCCTATGCGATTTCTCGTGCCAATCCTTCTGTCCGCAATGGTTTGCTGCTTGCC
ATTATGCTGCCCTTTTGGACATCGTTCCTGTTGCGCGTCTATGCGTGGATGGGTCTGCTC
GGGCATAACGGCATTGTAAACAACCTGTTGATTAAAATGGGTATTATCAGCGAGCCTTTG
GATTTGTTCTACAATGCCTTTTCGCTCAATTTGGTGATGGTTTACGCCTATCTGCCGTTT
ATGATTCTGCCGCTATACACGCAACTGGTGAAACTCGACAACCGCCTGCTTGAAGCGGCT
TCCGATTTGGGCGCGGGGCCGGTCAAATCGTTCTTGACGATTACCCTGCCTTTGTCGAAA
ACCGGCATTATTGCAGGCTCCATGCTGGTTTTCGTCCCTGCTGTCGGCGAGTTCGTCATT
CCCGAGCTGGTCGGCGGTTCGGAAAACCTGATGATTGGTAAAGTCTTGTGGCAGGCGTTC
TTCGATCAAAACAACTGGCCGCTGGCTTCCGCCGTCGCCGTCGTGATGGTCGCGCTGCTG
GTCGTGCCGATTGCCCTGTTTCAGCATTATGAAAACCGCGAATTGGAAGAAGGAGCCAAA
TAATGCAGAAATCCAAATTATCTTGGTTCTTGAAACTGATGTTGGCACTGTCGCTGGCGT
TTCTGTATATCCCGCTGGTTGTTTTGGTCATCTATTCGTTTAACGAATCCAAGCTGGTAA
CCGTTTGGGGCGGCTTTTCGACCAAGTGGTACGGCGCATTGCTGGAAAACGACACCATCT
TGGAAGCCGCTTGGCTGTCGCTGCGGATTGCCGTTGTGTCTTCGCTTGCCGCCGTCGTTT
TGGGCACGCTGGCAGGCTATGCGATGGCGCGGATTAAACGTTTTCGCGGCAGTACCTTGT
TCGCTGGCATGATTTCCGCACCTATGGTGATGCCCGACGTGATTACCGGTCTGTCTATGC
TGCTGCTGATTATTCAGGTACAGATATTTTTGCAGGGCAGCGAATGGTTACAACATCTCT

## Appendix A

ACTTCGATCGTGGCTTTTTCACCATCTTCCTCGGACATACGACGCTGTGTATGGCGTACA
TTACCGTTGTTATCCGTTCGCGTCTGGTTGAGCTTGACCAGTCGCTCGAAGAAGCCGCAA
TGGATTTGGGCGCGCGCCCGCTGAAAATCTTTTTTGTCATCACTTTGCCTTTGATTGCCC
CTGCCATCGCTTCAGGCTTTCTGCTCGGCATTACCCTGTCTTTGGATGATTTGGTGATTA
CCTCATTCCTCTCCGGCCCCGGTTCATCCACATTGCCGCAGGTGATTTTCTCCAAAATCA
AGTTGGGTCTCGATCCTCAGATGAATGTCTTGGCGACCATCCTAATCGGCATCATCGGAA
CATTGGTCATCATCGTCAATTATTGGATGATGAGGCAGGCAACCAAGCGTGACCGAGAAG
CGGCAGAAGCCTACCGCCAGGAAAAATTGGCTGCCGAGAAAGCAAATTAATTAATAAGGC
AGGCTGACCGCATGACTGGGTCAGCCTGTTTTCTTCAACCGATTTTCTGTTTGGACGATA
TGGCCCGACAGCCTGTATCATTCCGTCCGAAAATACACCTGATAAAGCAAACACAATGAT
TCGCCCTGATTTTCAAGAATATCTGCCTTCTTATTATTTCAGTTCGGTTAATCCTCATAC
TGTTTATCCGAAACTTCAATGCCGTCTGAAAACCGATACCTGTATCATCGGCGGCGGATT
GGGTGGTTTGTGCACTGCATTGCCCTTGGCGGAGCAGGGACATGAAACGGTTGTGTTGGA
AGCCGCGCGTATCGGTTTCGGCGCGTCGGGACGGAGTGGCGGGCAGGTTATCAGCGATTA
CGCCTGCGGTATGGGGGAAATTGAAAAACAGGTCGGCTTGGAGCAGGCGCAATGGTTTTG
GCAACAGTCTTTGCAGGCGGTCGAACTGGTGGACGAACGCGTCCGCAAACATGCCGTCGA
TTGTGATTGGCAGCGCGGTTATGCCACGGTTGCCGTCCGTCCGCAGCATTGGGAAGAGTT
GCAGCAGTGGCATGAACACGCCCAACGGCATTACGGTGCGAGTCATTATCAACTTTGGGA
TAAAGCCGAGTTGAAACAGCAGCTTGACAGCGATATGTACCAAGGGGCACAATTCGACCC
CTTATCCGGACACCTGCATCCGCTCACTTACACTTTGGGCATCGCTCGTGCCGCTGCCGA
AGCCGGTGCGCAGATTTTCGAGCAATCCCCGATGACGTGCATCGAACCGCATCAAAACGG
TTGGCTGGTTTACACGCCCGAAGGCAGCGTCGAGTGCAAAAATGTGGTCTATGCTGTCAA
TACTTATGCAGGTTTGAACCCGATATTCCGGCCTTTGGAACGCAAGGCGATTGCTGTCAG
CACCTTTATTATTGCGACCGAACCCTTGGGGGCGCGCGCAAAAGGGCTTATCCGTAACAA
TATGGCAGTATGCGACAACCGCCATATTTTGGATTATTACCGCCTCAGCGCGGACGGCAG
ACTGCTTTTCGGCGGTAAGGATAACGAGTTTATCGACAATCCTGAGCGTATGACCGAGCT
TGTCCGCCAAGATATGCTTAAAGTTTTCCGCAGCTTGCCGATGTCAAAATCGAATATTC
GTGGGGCGGGGAGTGCGACATTACCGCCAACCTTGTCCCGCATTTCGGACGTTTAGCCCC
GAATGTTTTTATGCGCAAGGTTATTCCGGACACGGGATGGCGATAACAGGCATTGCAGG
TCTGGCGGTTGCCGAAGCAATTTTAGGGGACGAATGCCGTCTGAAGCCGTTTGAGCGGTT
GCGCCAGCCGAATATTATCCTGCAACCGTTTTTGCGCAAACTCGGTTCTTTCCTCGGCTC
GAAATATTATCAGTGGAAAGACAGCCGTTAAGCGTCGCAGGCAGTATAGTGGATTAACAA
AAACCAGTACGGCGTTGCCTCGCCTTAGCTCAAAGAGAACGATTCTCTAAGGTGCTGAAG
CACCAAGTGAATCGGTTCCGTACTATCTGTACTGTCTGCGGCTTCGTCGCCTTGTCCTGA
TTTTTGTTAATCCACTATATGTTTATCCATCGGCGGCAAACGTGAAAAATGCCGTCTGAA
ACCCGATTTTCAGGCTTCAGACGGCATAGCCGCCCTTATTCCACGCGTTCGCCGTGGATA
TTCAGATCCAAACCTTCGCGTTCGACATCCTTGCCGACGCGCAGGCCGCCGCAGATTTTC
CCCACGACCTTCAAAATCGCCCAACTCATTAGCCCGCTGTATGCCGCCATAACGACCCCG
TCTTTTACCTGTATCCACAACTGCTGCCAAACTGCCGCATCCCCGCCGAAAATGCGGTTG
TCGAAAAAGATGCCGGTCAATATTCCGCCCACCAGCCCGCCGAATCCGTGTATGCCGAAA
GCGTCCAAAGAATCATCGTAACGCAATTTGTGTTTGACGACGGTGACGGACACAAAGCAC
GCGGCGGCAGTCAATATACCGATGGCGGCCGCGCCCGACGGGCCGGTAAAGCCGGCGGCA
GGGGTGATGCCGACCAGACCGGAAACCGCGCCGGAAGCCAGCCCCAAAGCGGAAGGTTTG
TGTCCCGCTATTTTTTCGCAGGCAAGCCAGCCTGCCGCGCCGAATACGGCCGACACCTGC
GTTACCGCCATCGCCATACCCGCCGCCGCGTCTGCCGCAAGCGCCGATCCGGCGTTAAAG
CCGAACCAGCCGAACCACAACATTGCCGCGCCGATCAGTGTCATCGCCATATTGTGCGGA
GGCATCGCCTCGCGCCCGTAGCCTATGCGCCTGCCCAAAACCAAGGCGGCGACGAGTCCC
GCGATACCGGCATTGATGTGCACCACCGTACCGCCGGCATAATCCAATACGCCGCCCTTG
CTCATAAAGCCGCCGCCCCACACCCAATGCGCGCCCGGCACATAAACCAATAAAAACCAT
ATGCCCGAAAACAGCATCATTGCCGAATATTTCATCCGTTCGGCAAACGCGCCGGTAATA
ATGGCGGTCGAAATAATGGCAAACGTCATCTGAAAAAACATAAATACCGGTTCGGGAACA
GTCGGCGCATTGGGCGACACGGTCAGCATCTGTGCGGTAGCGTCTATCTGCATCCCGCTT
AAAAATACGCGCCCCAAACCGCCGATAAAGGCATTTCCCGGCGTGAACGCTAAAGAATAG
CCGACGGCGACCCAAAGGATGCCCACCAATGTCGCGATGGAAAAGCTGTGCATCATCGTC
GAGAGCAGGTTTTTTTCCGCACCATACCGCCGTAGAATAAAGCCAGCCCGGGAAGCGTC
ATCAACAGTACCAAGGCAGCCGCAGTCATCACCCAGGCGGTATCGCCCGAATTGACGGCG
GAATAAGGCTTCCACCAGTTTAAAGGTTCTGCCGATAGGGATGCCGGCAGCAAAGATGCC
GCCCATATGTGTTTTTTCATTTTGACTAAAGTTTCCTTAATGGTTGAGCCCGTCTTTCGG
AAAGGCGGGGTCGGGGCTTGTCCGGGAGGGACGCAAGCCCTGCCGGACCGGGGCGGCGCG
GGGATTTTGCCGATGTGCCGCCAATCCCTTGTTTGAATATGGAAATATCGCATCCGATCC
CTTGCACCCGTTGTCCGGCGGGAGGATTTATCCTTAGGCGGCGCATATGTGGGCGTATGG
ATTGTCAACAATTTACTGTAGGAAAATATACAGAGGTTTGGGCGATAAGGCAAAATATTG
TTGACAATATTTTTATTTTATAAAATTAATTTATTGATTAATATATTAAAAATTTTTAAT
TGGAAATATAGTGGATTAACAAAAATCAGGACAAGGCGACGAAGCCGCAGACAGTACAAA
TAGTACGGAACCGATTCACTTGGTGCTTCAGCACCTTAGAGAATCGTTCTCTTTGAGCTA
AGGCGAGGCAACGCCGTACTGGTTTTTGTTAATCCACTATAAAAATTTATGGGGCTGTCC
TAGATAACTAGGATAAACTCGATTTTACTAATTGTTTTAAAATGGAAATTTGAACTTTTA
TCTCGCTGTTGTTAAAACGTCGTTCGTACCCCTTTAAATACAGCTCAAAATGCGCTTTGG
GAATGCCGTCAAACTTGCGTAAATGACGTTTTGCCCGGTTCCAAAAGTTCCCAATTCCAT
TGATATGGTTTTGTCGTTCAGCAAAATAACTTTCATCTGCTTCTACTTCGCCGTCAAACA
TTTCCAAATGCGGACTGTTTTGATAAATAAGTAATCGTAAACGATGAAAATAATAGGCTG
AGGTACTTTTATTAACGCCTACTAACTCTGCTGCTGTTCTTGCAGTTACACCTGCGACAA
ACAGTTCAATGAGTTTATTTTGTTTATACCGGCTTAGACGAATTTTTCTCATAGGGGCAA
CTCTAACTTAATTTGAATTTCCCTAGTTATCTAGGACAGCCCCAAATTTATACAAAAATG
AGTGCGGTTCGGCGCAACCTTGAATCAAGTTCCCGCATCGGTTTTCATTGCCGGTACGGA

## Appendix A

TGCGTTCAAGCCGGCTTTGCAAAGGCCGCGCTTTCGGCAAGCGGACACGGACACTGCCGA
CGGTTGCGCCGTTAACGGGGGGAGGGAGGAGCTGCGCCGACCGTGTGAATGAAAGTGCCG
TCTGAAACCCGATTTTCAGGCTTCAGACGGCATTTCGCATTAATGCGGGCGGCGCGTTTA
TTTGCCGCGCATCAGTTCAAAGAAATCGTCGTTGTTTTAGAGGCTTTGATTTTCCCGAT
TAAAAATTCGGCTGCCTCGATTTCGTCCATCGGGTGCAGGAACTTGCGTAAGAGCCACAT
ACGTTGTAACTGGTCGTTTGGGACAAGCAGCTCTTCGCGGCGCGTGCCGGATTTGTTGAT
GTTGATGGCGGGGAAGAGGCGTTTTTCCGCCATACGGCGGTCAAGGTGCAATTCCATATT
GCCGGTGCCTTTGAATTCTTCGTAAATCACATCGTCCATACGGCTGCCGGTTTCAACCAA
TGCGGTGGCGATGATGGTCAGCGAACCGCCTTCTTCCACGTTGCGCGCCGCGCCGAAGAA
ACGTTTGGGACGATGCAGCGCGTTGGCATCGACACCGCCGGTCAGGATTTTGCCCGAGGT
AGGCACGACGGTATTGTAGGCGCGGGCAAGGCGGGTAATCGAATCCAGCAGGATGACCAC
GTCTTTTTTGTGTTCCACCATACGCTTGGCTTTTTCAAGCACCATTTCGGCAACTTGGAC
GTGGCGTTGAGCCGGCTCGTCAAAGGTGGAGGAGACTACTTCGCCACGGACGGAGCGGCT
CATTTCGGTTACTTCTTCGGGACGTTCGTCAATCAAGAGGACGATGAGTTCGACTTCGGG
ATAGTTTGCGGTAACGGCGTGGGCAATGTTTTGCAGCATCACGGTTTTACCGCTTTTGGG
CGGGGCAACCAAGAGGGCGCGCTGACCTTTGCCGATAGGGGAAATCAGGTCGATGGCACG
TCCGGTCAGGTTTTCTTCGGACTTTAAGTCGCGTTCCAGCTTCAACTGTTCGGTCGGAAA
CAGCGGGGTCAGGTTTTCAAACAGGATTTTATGGCGGCATACTTCCGGGTGGTCGCCGTT
GATGGTATCAAGCCTGACCAGGGCAAAATAGCGTTCGTTGTCTTTTGGGACGCGCACGCT
TCCTTCGATGGTGTCGCCCGTATGCAGGTTGAAGCGGCGGATTTGGGTGGGCGAGACATA
GATGTCGTCGGGGCCGGCAAGATAGGACGTGTCCGCGCTGCGGAGGAAGCCGAAGCCGTC
GGGCAGGATTTCAAGCGTGCCGGAGCAGGTGAAACCCTCGCCTTTTTTCATCATCTGGCG
GACGATGGCAAATACGAGGTCTTGTTTGCGGAATCGGTTGGCGTTTTCGATGCCGTGTTC
TTCCGCCAATTCTAAGAGTTTGGAAATGTGCAGGGTTTGTAATTCGGAGACGTGCATAAT
AATGATGTATTTTGAAGAGGAAAAAGACAGGCAGATGCCGTCTGAAAGAAGAAGCTGACC
GTTGCCGGTTGCTCGGGGAAGGGGGAATTGTAGGCAGTCGGCGCGTGGGTGTCAAATATT
ATCGCGGACGGGGCATCGGCAGGAAATGCCGTCTGAGCGGAGCTGCTTGGAAAAAAATAC
CCCCGCGCTTTTCAGGCTCGGGGGTATGGGCATTGATTATTTGTTCAATTCATTCGCCAA
ATATAGCCAAGTTTCGATGACGGTATCCGGGTTCAGGGAAACGCTTTCAATGCCTTCCTC
AACCAGCCATTTGGCGAAGTCCGGATGGTCGGACGGGCCTTGACCGCAGATGCCGACATA
TTTGTTCTGCTTGCGGCAGGCGGAGATGGCAAGGTGCAGCATCACTTTGACGGCAGGGTT
GCGTTCGTCAAACGATTCGGATACCAAGCCGCTGTCGCGGTCGAGACCGAGGGTCAGTTG
GGTCATGTCGTTCGAGCCGATGGAGAAGCCGTCGAAGTATTGCAGGAATTGTTCCGCCAA
TACCGCGTTGCTCGGCAGCTCGCACATCATAATCAGGCGCAGGCCGTTTTTGCCGCGTTC
CAAGCCGTTTTCTTTCAGGGCTTTGACAACGGCTTCGGCTTCGCCCAAAGTGCGGACGAA
CGGAATCATGATTTCAACGTTGGTCAACCCCATTTCATCGCGGACGCGTTTCAAGGCTTT
GCATTCCAAGGCGAAACAGTCTTTGAAGTTGTCGGCGACATAACGCGCCGCACCACGGAA
GCCCAACATCGGGTTTTCTTCATGCGGTTCGTATACGTTGCCGCCGACCAGGTTGGCGTA
TTCGTTGGATTTGAAGTCGGACATACGGACGATGGTTTTACGCGGATAAACCGATGCGGC
CAATGTCGCCACGCCTTCGGCGATTTTATCGACGTAGAAGTCGACAGGGGACGCGTAACC
GGCGATACGGCGGGTAATTTCCGCTTTTAATTCGTCGTCTTGTTTGTCAAATTCCAACAA
GGCTTTGGGGTGGATACCGATTTGGCGGTTGATGATAAATTCCATACGCGCCAAGCCGAT
GCCTTCGCTGGGCAGGTTGGCGAAGCTGAATGCGAGTTCGGGATTGCCGACGTTCATCAT
GACTTTTACAGGTGCTTTAGGCATATTGTCTAAGGCGACATCGGTAATCTGTACGTCCAA
CAGACCGGCATAGATAAAGCCGGTATCGCCTTCGGCACAGGATACGGTAACTTCTTGACC
GTTTTTCAGCAATTCGGTTGCATTGCCGCAGCCGACAACGGCAGGAATGCCCAATTCACG
CGCGATGATGGCGGCGTGGCAGGTACGGCCGCCGCGGTTGGTAACGATGGCAGAAGCACG
TTTCATCACGGGTTCCCAATCCGGATCGGTCATGTCGGTAACGAGTACGTCGCCGGCTTC
GACGGAATCCATCTCGGAAGCATCTTTAATCAGGCGCACCTTGCCCTGACCGACTTTCTG
ACCGATGGCGCGGCCTTCGCATAATACGGTTTTGTCGCCGTTGATGGCGAAGCGGCGCAG
GTTGCGGTTGCCCTCTTCTTGGGATTTTACGGTTTCGGGACGGGCTTGCAGGATGTAGAG
TTTGCCGTCCAAGCCGTCGCGTCCCCATTCGATATCCATCGGGCGGCCGTAGTGTTTTTC
GATGGTCAGTGCGTAATGCGCCAACTCAGTAATTTCTTCGTCGGTAATGGAGAAGCGGTT
GCGGTCTTCCTCGGGGACATCGACGTTGGTTACGGATTTACCGGCTTCTGCTTTGTCGGT
AAAAATCATTTTGATGTGTTTTGAACCCATGGTTTTACGCAGGATGGCGGGCTTGCCCGC
TTTGAGCGTGGGTTTGAACACATAAAATTCGTCCGGGTTGACCGCACCTTGTACGACGTT
TTCGCCCAGACCGTAAGAGGAGGTAACAAAGACGACTTGATCGTAGCCGGATTCGGTGTC
GAGGGTGAACATCACACCTGATGCGCCGCTGTCGGAACGCACCATGCGTTGAACGCCGGC
GGAAAGGGCGACGATGTCGTGTTCGAAGCCTTTGTGGACACGGTAAGAAATGGCACGGTC
GTTATACAGGGAAGCGAATACATGGTGCATCGCTTCTTTAACGTTATCCAAGCCGTTGAT
GTTCAAGAAGGTTTCCTGTTGTCCAGCGAATGATGCGTCCGGCAGGTCTTCGGCAGTTGC
GGAAGAACGTACGGCAACGGAAATGTCCGCACCGCCGGCATCGGCAACCATTTTGTTCCA
TGCCGCTTCGATTTCGGCATCGAGCTGTTCGGGGAAAGGCGTATCCAAAATCCATTGGCG
GATTTCTTTGCCGACGCGTGCCAGTTCGGCAACGTCTTCGACATCCAATTTTGCCAGTGC
GGCGGAAATGCGTTCGCTCAGACCGTTGTGTGCGAGGAATGCGCGGTAGGCTTCGGCCGT
GGTGGCAAAGCCGCCGGGGACGCGAACGCCTTTTTCGGTCAGCTGACTGATCATTTCGCC
CAGCGAGGCGTTTTTACCGCCCACGCGTTCAACATCTGTCATACGCAGGTTTTCAAACCA
GATTACGTAGTTGTCGGCCATTTGTGTGTCCAATCCAAAATATGTTAAAAAAGAAACAAA
TCCGCGTGCTTATTTTAAGCGATTCGTTCCTCTGCTGTCATGTGTTTTATCCGTTTTAAA
ATCATGATGCCGTCTGAAAAATTGCGGTTTCGGCGTGTGTAGCGGTTTGAAACTTACAGC
CGGTATACTTCTTTTTTTGGGTATTTTCTTTGTAAAACAGGTGGTTTGAATAGGTTAATG
TTTTTTCTGTTTGATTTTTTTGTTTATTTTTTAAAATTTTCTGCCAAAAAATACTTTATA
TAAATAATTTTATTTCAAAATTATATTGTGTCTGTTTGGGTGTAATCCGAGGTAGGTGTG
CTGCGGGTGCTTTCCTTGTGTCTGCTGCTGCTGTTATGATGGGATTTTAAACCTGTGTTT
TAAGGATGGAAGATGAGCAGTCCGCGCCATGTGTTTTACATTTCCGACCGTACCGGTCTG

## Appendix A

ACTGCTGAGAATATCGGCGAGGCGTTGCTGAACCAGTTTGGCAATCTGTCGTTCAAACGC
CATACGCATCCGTTTGTCGATACGCCGGAAAAGGCGCGCGGTGGTGGAGAAGGTCAAT
CGGAGCCGGCAGGAAAACGGTCAGCGTCCGATTGCGTTTGTCAGTGTGGTTGATGACGAA
ATCCGTCGGATTATCAAAGGGGCGGATGCTTTTCAGATTAATTTCTTTGAGACTTTTTTG
GGACTGTTGGAGAAGGAACTCAATACCGAAGCCACGGCATCCGGGCAGGGGCATCACAGT
ATCGGTAATACGAAGCGTTATGATGCGCGTATGGAAGCGGTCAATTTTTCTTTGAACCAC
GACGACGGGGTCAGCGATAAGAACCTTCAGGAAGCGGATGTAATCTTGATGGGTGTATCG
CGTTCGGGCAAAACGCCGACCTGCCTTTACCTCGCCCTGCAATACGGCATCCGTGCGGCA
AACTATCCGCTGATTCCCGACGATTTGGAATCGGCCGATCTGCCGCGTATGGTCAAGCCT
TATAGGGATAAGCTGTTCGGGTTGACCATCCAGCCGGAACGTTTGCAGGCCATCCGCCAA
GAGCGCCGCCCGAATTCAACTTATGCCAAAATCGATACATGCCGCAGCGAGGTGGCGGAC
GCGCAGAGTATGTTCAGACGGCATGGGATTCCGTTTGCGAATACGACGGATAAGTCGGTT
GAGGAATTGGCGGTACACATCCTTCAGGCGTGCAAGCTCAAACGCAGGTTTTGACGGGCT
TTGATTCGGTTTGAAGGCGGAACTGCCGTCTGAAATCAGGTTTCAGACGGCAGTTTTATA
GTGGATTAACAAAAATCAGGACAAGGCGACGAAGCCGCAGACAGTACAAATAGTACGGAG
CCGATTCACTTGGTGCTTCAGCACCTTAGAGAATCGTTCTCTTTGAGCTAAGGCGAGGCA
ACGCCGTACTGGTTTTTGTTAATCCACTATATGTTTGTGGGGCGGATATTTTTCAGGGCT
GTATTTTGTCCAGACATTCGAGCAGATCGAGTGGCGTGCGGATGTGGAAATCCGCCTGCC
ATGAGCCGGTATCGTCTTCGGGAGCGATGTAGCCCCATTCGGCGAGGACGGTCGTCATAC
CGGCGTTGCGCCCCGCCTGTATATCGCGTTCCGCGTCGCCGACGTAGAGTGTGTGTTGCG
GGTCGGCGTGGATTTGTCCGCACGCATACAGCATGGGTTTGACGCTGGGCTTGGGCTCGC
CGCAGGTGTCGCCGCTGACGACGACGGCGGGTGGATGATGAAGCCGAGTTTGGGGACGA
GTTTGTCGGTGAAGCGCATGGGTTTGTTGGTGATGATGCCCCATTTGATGCCGCGTTTTC
CGAGTTCGGCGATGAGTTCGTTTACGCCGTCGAAGAGGGTGGTGTCTTGGGCGTAGCGGC
TGTCGTATTCGTCAAGGTATTCGGTGCGCCATCGGGCATAGTCGGGATGGTCGGGGGTGA
TGCCTGCGCCGAGCTTGATCAGTCCTGCCGCGCCGTGGCTGGCTTGGGTGCGGATTTCGT
CCATGCTTTTTGCAGGTAGTCCGTGGCGGGCGAGCAGGGTGTTGAGTGCGCCGCCGAGGT
CTAGGGCGGTGTCGGCGAGCGTGCCATCGAGGTCGAACAATACGGCTTGTATCATGTGTG
TTCCTTTTTTATAAAGTGCGGGACGAAGGGTTTCAGACGGCATGTTTATTTTGTTTCAAA
CCCTGCTCGAAATCTTCCAACATATCCAATTCAAAGCGGCTGAAGCCTGCTTTTTCGCGC
GCTTCGATGTTCACATAGCCCCGGAAGATAAACATATCGTAACGGGCAATCAGGCTGCGG
AACAGGGCGACAGGCTCCAAACCGCGTTCGCGGCAAAGGTGTTGATACCACCGGTTGCCG
ATGGCGACGTGTCCCACTTCGTCGCGGTAAATGATGTCCAACACGCCGCAGGTTTCCGAA
TCACCGCGCTGCGCCACCTTCGCGCGTATGCCGGGCGTAACGTCCAGCCCGCGCGCTTCC
AAAACGCGCGGCACTAAAGCCATACGCAACAAAGGATCGTAGGCGGTTTTGTATGCCATA
TCCCATAAATGATTGTGTGCTTCAAAATCGCCGTAATCGAAGCCGAAAGCGCGCAGCCTT
TCGCGCATCAGGCGGAAATGGTACACCTCTTCCTTCGCCACTTTCACCCAGTCGCGGACA
AACTGAAACGGCAGCGTGCGGAAACGGTATGCCGCGTCCAAAGCCAGATTGATGGCGTTG
AATTCGATATGCGCAATCGCGTGCAGCATCGCCGCATAGCCTTCGGTTGTGTTCATTTTG
CGTGGCGTCAGCTGCGACGGCGCGACCAAAACAGGCTTGTCCGGTCGTCCCGCGCGGGGG
AAGTCCGCCGGCGGTGCGTTTGTTTCCGCCCCGTCCGCATTTTGAACGGCGGCAAACGCC
TCATCCGTCAGCCGTCCTTTTTCATCTGGGTCGCCCGAAAGCAGGGCGCGTTCCAGCAAA
GCATAAATATCGGGTTTCATCTCAAGTCCGCCGTGTTCGGAAAACAAATATTATAGCGTT
TAAAAAAAACAAGATGAGGCATATAATCTCCGCGATTCGGCATTCCGCGCCCAAACCGTC
AAATATAGTGGATTAACAAAAACCAGTACGGTGTTGCCTCGCCTTAGCTCAAAGAGAACG
ATTCTCTAAGGTGCTGAAGCACCAAGTGAATCGGCTCCGTACTATTTGTACTGTCTGCGG
CTTCGTCGCCTTGTCCTGATTTTTGTTAATCCACTATAACGCGGCACACATTAAAGGGCA
GCGTGGCGCGCCGCCTTTTCCGGTGGCAAAAAATCAGCCCTCGGAAAACGCGGTTTGCA
AAATGCAAACCGCCCGTAACGCCGCCCGTATGATTGTTTTGCTGCGCCGATACTTTACGC
CACACTCATCCCGACAAGGAAAAATAATGATGAAACCGCACAACCTGTTCCAATTCCTCG
CCGTTTGCTCCCTGACCGTCGCCGTCGCTTCCGCACAGGCGGGCGCGGTAGACGCGCTTA
AGCAATTCAACAACGATGCCGACGGTATCAGCGGCAGCTTCACCCAAACCGTCCAAAGCA
AAAAGAAAACCCAAACCGCGCACGGCACGTTCAAAATCCTGCGACCGGGCCTTTTCAAAT
GGGAATACACCAAACCTTACAGGCAAACCATCGTCGGCGACGGTCAAACCGTTTGGCTCT
ACGATGTTGATCTGGCACAAGTGACCAAGTCGTCCCAAGACCAGGCCATAGGCGGCAGCC
CCGCCGCCATCCTGTCGAACAAAACCGCCCTCGAAAGCAGCTACACGCTGAAAGAGGACG
GTTCGTCCAACGGCATCGATTATGTGCTGGCAACGCCCAAACGCAACAACGCCGGCTACC
AATACATCCGCATCGGCTTCAAAGGCGGCAACCTCGCCGCCATGCAGCTTAAAGACAGCT
TCGGCAACCAAACCTCCATCAGTTTCGGCGGTTTGAATACCAATCCCCAACTCTCGCGCG
GCGCGTTCAAGTTTACCCCGCCCAAAGGCGTGGACGTGTTGAGCAACTGATGCCGTCCGC
CCCGATGCCGTCTGAAAGCCGCCGAGGCTTCAGACGGCATTTTTACGCAGGCGGAACAAT
GTCCCGCATTACCGCCCGATCGGGCACCGGAACGGCAAACCGGTGAAAATTAACGGTTGC
GCCCCGGCTGTTTTTGCCGTTTAATGCAAACCTTGCTGCACCAAGGGCCAAGAAAGCCGA
CCGGCCGCCCCCACAGCTTCCGATGCAGGCGGCCCGTCCGTCCCTGCAATGTTTTTTATT
TTTGAACGAAAGGTCGAAAACCATGAAAAAAACACTGGTGGCGGCGGCAATCCTGAGCCT
CGCCTTGACTGCGTGCGGCGGCGGAAGCGATACCGCCGCCCAAACCCCCTCCGCCAAGCC
CGAAGCCGAACAATCGGGCAAACTCAACATCTACAACTGGTCGGATTATGTCGATCCCGA
AACCGTTGCCGCCTTTGAAAAAGAAACCGGCATCAAGACGCGTTCCGATTATTACGACAG
CAACGAAACACTGGAGGCAAAAGTCCTGACCGGCAAATCCGGCTACGACCTGACCGCGCC
GTCCATCGCCAACGTCGGCCGGCAAATCAAAGCGGGCGCGTATCAGAAAATCGACAAGGC
GCAAATCCCCCATTACGGCAACATCGATAAAGATTTGCTGAAAATGATGGAAGCCGTCGA
TCCGGGCAACGAATACGCCGTCCCCTATTTCTGGGGCATCAATACCTTGGCAATCAATAC
CCAGCAGGTGAAAAAAGCATTGGGTACGGACAAGCTGCCCGAAAACGAATGGGATTTGGT
GTTCAAACCCGAATACACCGCCAAACTCAAATCCTGCGGCATCAGCTATTTCGACAGCGC
AATCGAACAGATTCCCTTGGCGTTGCACTATTTGGGCAAAGACCCCAACAGTGAGAATCC

## Appendix A

CGAAGACATCAAAGCCGCCGTCGATATGATGAAAGCCGTCCGGGGCGACGTGAAACGCTT
CAGCTCTTCCGGCTATATCGACGATATGGCGGCGGGCAACCTGTGTGCCGCCATCGGTTA
CGGCGGCGATTTGAACATTGCCAAAACCCGTGCCGAAGAAGCCGCAAACGGCGTGGAAAT
CAAAGTATTGACCCCGAAAACCGGCGTGGGCGTGTGGGTGGATTCCTTTATGATTCCGCG
CGACGCGCAAAACGTTGCCAATGCCCACCGCTATATCGACTACACGCTCCGGCCCGAGGT
GGCGGCGAAAAACGGCAGCTTCGTTACCTACGCGCCCGCCAGCCGTCCCGCGCGCGAGCT
GATGGATGAAAAATACACCTCCGACGCATCGATTTTCCCGAACAAAGAACTGATGGAAAA
AAGTTTCATCGTATCGCCCAAATCCGCAGAATCCGTCAAACTGGGCGTGAAGCTGTGGCA
AGGGCTCAAAGCGGGCAAATAACCGGAATCCCTGCCGTCTGAAACCTTTCGGGCGGCAGG
AAACGGCGCGTCCGTTATCAAACAGGGGGGCGTTTCCCCTCCTGCCGGTTATGATTGGGT
TAAGATTAAAATGATTTAGTAAAATGAGAAAGATATGGATTTAAGTATCGTAGTTCCTAT
TTATAATGTCGAAAGTTATTTGGAAGCGTGTTTAAGTTCCATAGAATCTATATTAAGTAA
TGAAAATGTCGAACTTATCCTTGTGAATGACGGGTCAAAAGACGGAAGTGAAGATATATG
TTACAAATATATAGATAAAATATCAAACAGCAAACAACAAACAACAAACAACAAACAACA
AACAACAAACAACAAACAACAAACAACAAACAACAAACAACAAACAACAAACAACAAACA
ACAAACAACAAACAACAAACAACAAACAACAAACAACAAACAACAAACAACAAACAACAA
ACAACAAACAACAAACAACAAACAACAAACAACAAACAACAAACAACAAACAACAAACAA
CAAACAACAAACAACAAACAACAAACAACAAACACCGGACACCAAATATCAAATATATAT
ATCAGGATAACCAAGGGTTGTCGGAGGCGAGAAATACCGGAATAAAAAATTCAAACGGAA
AATATATAGTCTTTATTGATTCGGATGATTTTATTAACTGTCAGATTTTGCTGGATTTTC
TTAGTAAAGATGATACTGATATGCCGGATGTGGTGTTTTTAAATGCGGTTAAATATGATA
AGGGAAGTGTTTCATATTTTGGCGAAGATTATCAGCCTGAAAAAATACTCAATCAATCCA
AAGTCGAAGTTTTGAAAGGATTATGCCGATTTAGAAAATTTCCGGGTTCGGCGTGGAATA
AGATTATAAAAAGAGAATTGATTATTAGAGAAAAACTGTTTTTTGAAAGGGGAATTTATT
CTGAAGATATCGAATGGTCAATGAGGTTATTTAATGCGGCAACAACTTTTTCTTATTTGG
ACGGTTGTTATTACTATTATCGGCAGGGAAGAAAAGATTCTATTACGGGAACTGTTTCGG
AAAAAAGTATAAAGTCATTATTATATATTTTGGAGAAAAATGCGGAAATGGAATTTAATA
GGGATATATCGAGTTATCTTTATTCTTTTCTTTCCTACGAATATCTCGTTTTGCTTTTTA
TAATGACGAGTAAAAATATAGAGTGTGATGCTGATATAAAAAGAAGGGCGTATCATTTAA
GGTTTATGCTGTTAAAGTCCAATAAATTGATATATAAGCTGATATTCCCGATAATCACAT
TACTCGGGGTCGATATTACAGGCAGGATTTTAAAAGCAATCAGGGGGAATATTTAATAAA
TCCTTTAACAATATATACCTTACCGAAGGAGGAAAAATGAACGCAATCCGAACTTTCCAA
AACCGCACGCCCGAAATCCACGAAACCTGTATGATAGACGAAGCCTGCGTCGTCATTGGC
GAAGTGTCGCTTGCCGAAGATGTTTCCGTGTGGCCGTGCGCCGTGTTGCGCGGCGATGTG
AACAGCATCACCGTCGGCGCGCGCAGCAATATACAGGACGGCAGCGTCTTGCACGTTTCC
CACAAAACCGCCGCCAAACCCGAAGGATCGCCGCTGGTTATCGGCGAAGACGTTACCGTG
GGGCACAAAGTGATGCTGCACGGCTGCCGTATCGGCAACCGCGTCCTGGTCGGCATGGGG
ACGACGGTTCTGGACGATGCCGTGATTGAGGACGAAGTGATGATCGGCGCGGGCAGCCTC
GTTCCGCCGCGCAAACGCTTGGCGGGCGGCTATCTTTATGTCGGTTCGCCGGTCAGACAG
GTGCGCGTGCTGACCGATGAGGAAAAAGCCTTTTTGAAATATTCCGCCGCGCATTATGTG
AAGCTGTCGAAACAGTACGGGATGTGAAATCACATCGGCGTTCTTGCGTCAGCCCCAAAT
TCATGCGGATGGGACGCATCCGATAACGGTATCCGATGCGCCTTGATTTTGACCGTCTGC
GTTTGAATTGCAGGCAAAAATGCCGTCTGAAAGCCTTTTTTCGGGTTCAGACGGCATTTT
ATTGCCGATTGTTTTTTAAAGTTTGACCGAATGTTCGCGCGTTTCGTGGAACACGATGTC
CGGCCAGCGTTCTTGCGTCAGCCCTAAATTCATGAGGACGGGATGCCCGATAACGGTATC
CGATGCGTCTTGATTTTGATCGGTGCATTTGAGTTGCAGGCAAAAATGCCGTCTGAAAGC
CTTTTTTCGGGTTCAGACGGCATTTTATCGCCGATTGCTTTTTACAGTTTGACCGAATGT
TCGCGTGTTTCGTGGAACACGATGTCCGGCCAACGTTCTTGCGTGAGTCCCAAATTCACG
CGGTTGGGGGCGAGGTAGGCGAGGTTGCCGCCTGCGTCGATGGCGAGGTTGCCCGCGTTG
GCTTTTTCAAATTCAGCCAGTTTTTCTTGTCGTCGCACGATACCCAGCGCGCCGACCAG
ATGGATGCGCTGTCGAACACGGCTTCTACGCCGTATTCGTTGGCGAGGCGCGAGGTAACG
ACTTCAAACTGCAACACGCCGACCGCGCCCAAAATCAAATCCGCGCCGCTCATCGGTTTG
AACACCTGCACCGCGCCTTCTTCGCCGAGCTGTTGCAAGCCTTTTTGCAGTTGTTTGATT
TTCAGCGGGTTTTTGATGCGTACGCTGCGGAACAGTTCGGGTGCGAAGAATGGGATGCCG
GTGAACGCCAGTTGTTCGCCTTCGGAGAAGCTGTCGCCGATTTGGATGTTGCCGTGGTTC
GGGATGCCGATAATGTCGCCGGCGTAGGCTTCTTCAACCAGCTCGCGGTCGTGCGACATG
AAGGTAACCACGCTGGAGGCGGCGATTTCGCGGTTGATACGCAGGTGTTTCATCTTCATG
CCGCGCTCGAATTTGCCGGAGCAGACGCGCAAGAAGGCAATACGGTCGCGGTGTTTCGGG
TCCATATTGGCTTGGATTTTGAAGATAAATCCGGAAAACTTCGGCTCGTCCGGCTCGACC
ATACGTACGGTCGCGTCGCGCGGTTTCGGCGCGGGCGCCCAGTCAATCAATGAATTGAGG
ATTTCCTGAATACCGAAGTTGTTAATCGCAGAGCCGAAGAATACGGGCGTGAGTTCGCCG
GCGAGGAATTCGTCGAGATTAAACTCGTTGGAAGCCGCCTGCACCAATTCGATTTCGTCG
CGCAACTGCTGGATTTCCAACGGAAAGCGTTGTTCCAATTCAGGATTATCGATGCCTTTG
ATGATGTCGAACTCGTGCGGCAGGCGTTCGCCGCCAGCTTCAAAGAGATAAATTTCATCG
TTCAGGATGTGGTACACGCCCTTGAAGTTTTTGCCCATACCGATCGGCCAGGTAACGGGC
GCGCAGCGGATTTTTAAAATGTTTTCCACTTCGTCCAAAAGTTCCAGGGAATCGCGCACT
TCGCGGTCGTATTTGTTCATAAACGTAACAATCGGTGTATCGCGCAGGCGGCAGACGTTT
AAGAGCTTGATGGTTTGCGCTTCCACGCCTTTTGCCGCGTCGATGACCATTAATGCGCTG
TCCACGGCGGTTAAAACGCGGTAGGTGTCTTCGGAGAAGTCTTGGTGTCCCGGCGTGTCC
AAGAGGTTGACGGTGTGGTCTTTGTAATCGAACTGCATCACACTTGATGCCACGGAAATG
CCGCGCTGCTTCTCGATTTCCATCCAGTCGGAAGTGGCGAATTTGCCGGTTTTCTTGCCT
TTTACCGTACCCGCGCTCTGAATCGCGCCCGAAAACAGCAAGAGTTTTTCAGTCAACGTG
GTTTTACCTGCGTCAGGGTGGAGATGATGGCAAACGTGCGGCGGCGGCGCACTTGGTCG
AGGATTTCTTGGGACATGGTTTTCTTTGCAAAAAGGTTCAGGCCGCTTTTCAGACGGCCC
GGACAGTGTTTGAGACGGCGAAATTGTACAAAAAAATGCCTGATAATTCAATGTTGGAGG

## Appendix A

CGGTCAGTGCGTGCTGCCGTAAATCTCTTTTTCGTCTTTCAGGACGGCATCGGCGGTTTC
CCACGCGCTGCCACGCCAGACTTTGTAAAAGCAGCTTTCTCGCCCGGTGTGGCAGGCGAT
GCCGCCGTTTTGGGCGATGAGCATCACAATGGCGTCGCCGTCGCAGTCGAGGCGCAGTGC
GCGGACTTTTTGCGTGTGTCCCGACTCTTCGCCCTTCATCCATTGTTTTTGGCGCGAACG
GCTGTAATAGTGGGCAAAGCCGGTTTCGACGGTTTTTTGCAGGGCTTCGGCGTTCATCCA
CGCCACCATTAAAATACGTTTGGTTTCGGCATCTTGGGCGATGGCGCAAACCAAACCTTT
TTCGTCAAATTTGACGGCTTCAAGCAGGTTTTTATCCATATTTCCTTTCAGACGGCATAG
TCGAGGCGGTCAGAGGCGCACTTCGATGCCGGCTTCGCGCATAGCGCGTTTGGCTTCGCG
GATGGCGATTTCCCCGAAATGGAAAATGCCGGCGGCAAGTACGGCATCGGCTTTGCCTTC
GGTTATGCCTTCAATCAGGTGCCGGACATTGCCGACCCCGCCGGAGGCGATGACGGGGAT
GTCGACGGCTTCGGCAACGGCGCGGGTCAGCGGCAGGTTGAAACCCTGTTTCGTACCGTC
CCTGTCCATACCGGTGAGCAGGATTTCGCCCGCGCCGCGTTTTTGCATTTCGACCGCCCA
TTCCACCGCATCCAAACCGGTCGGATTTCGCCCGCCGTGGGTAAAGATTTCCCAGCGTGT
GTTTTCGGGGTTGGCGGCTTTGGCATCGACGGCGGCGACGATGGCTTGCGAACCGAAAAA
TCCGGCGGCTTCGTCAATTAAATCGGGACGGGTAACGGCGGCGGTGTTGATGCTGACTTT
GTCCGCGCCGGCATTGAGCAGGCGGCGGATGTCGGCAACGGTGCGTACGCCGCCGCCGAC
GGTCAGGGGGATGAAGACTTGTCCGGCAACCTCTTCGATGATGTGCAGGATGGTGTCGCG
GTTGTCGGATGAGGCGGTGATGTCGAGGAAGGTCAATTCGTCCGCGCCTTCGCCGTTGTA
GCGTTTGGCGGCTTCGACGGGGTCGCCCGCGTCGCGCAAACCGATGAAGTTCACGCCTTT
GACGACGCGCCCGTCTTTTACGTCGAGACAGGGGATGATGCGTTTTGCCAGTGCCATAAT
CGGATGCCTTTAGTCGAGGGAATCTGCCAGTTGCTGCGCTTGGGCAAAATCGATGCTACC
CTCGTAAATCGCGCGGCCGGTAATCGCGCCTGCTACGCCATGTTTTTCGGCGGCACACAG
GGCGCGGATGTCGTCCAAGCCGGTCAGTCCGCCGGAGGAGATGACGGGAATGCGGACGGT
TTGGGCGAGTTTGACCGTCGCGTCGATGTTCACGCCGCTCATCATACCGTCGCGCCCGAT
GTCGGTGTAGATGATGCTGTTGACGCCGTCGTCTTCAAAGCGTTTTGCCAAATCAATTAC
ATGATGCCCGGTTACGGTTGCCCAGCCGTCGATGGCGGCCATACCGTCTTTGGCATCCAG
CCCGACAATAATCCTGCCGGGGAAGGCTTTGCACGCCTCGCGCACGAAGTCGGGGTTTTT
GACCGCCGCCGTGCCGATAATCACGTCGTTTAAGCCCAAATCCAAATATTGTCCGATGGT
TTTCAAATCGCGTATGCCGCCGCCGAGCTGTACGGGGATGTCTTTGGCGACAGCGGCAAG
GATGTCTTTGATGGCGGGCAGGTTTTGCGGAACGCCGGCAAACGCGCCGTTCAAATCTAC
CAGATGCAGGCGGCGCGCGCCTTGTTTGAACCAGTGCAGCGCGGTTTCGGCGGGCGAATC
GGAAAAGACGGTCGCCTCTTCCATCAGCCCTTGTTTCAGGCGGACGCAGCGTCCTTCTTT
CAAATCGATGGCGGGTATCAGCAGCATAATTTTTCTCCTTGTGCGGGGCCGTGTCCGGCT
TACCAGTTTAAAAAGTTTTTCAACATCGTCAGCCCGGCATCGTGGCTTTTTTCGGTGTGA
AATTGCGTGGCGAATACGTTGTCTTTGCCGACGATGCAGGCAAACGGGGACGGGTAGTCG
CTTTCGCCCAATATGGTTTCGGGATTTTCGGGGGCGAAATAGTAGCTGTGGACGAAGTAA
AAACGCGTGTCTTGGGGAATATCTTTAAACAGCGGGTGGTTTTGGGTTTGGCGCACGGTG
TTCCAGCCCATATGCGGGACTTTCAGACGGCATCCCTGCGGGTCGCGGAGGTCGCGCTCA
AAGCGTCTGACTTTGCCGCCGAACCAGCCCAAGCCGTCGGTGTTTCCTTCTTCACTGTGG
TCGAATAAAAGTTGCGCGCCGACGCAGATTCCGAAAAACGGTTTGTTTTTTAAGGCATCT
TTGACTGCCTCGTCCAAACCGTCTCGTTTTAATGCCGCCATACAGTCGGGCATCGCGCCC
TGACCGGGAAAAATGACTTTGTCGGCGCGGGACACGCGGTCGGGGTCGCCGCTTAAAAAG
ATTTCGGTATTTTTTCCGGCAAGCTGCCCCGCCGTCCGGACGGATTTCAATACGGAATGC
AGGTTGCCCATACCGTAATCGATAATGGCGGTTTGCATGGCTTCCTCCTCTTTTTTTGCA
ATATGGCTGCGATTTTAACAAACAAATGTGCCGTGCTGATAAAAATGCCGTCTGAAAACG
GGAGTCTGTCTTCAGACGGCATAGGGTTTAAACCCGGAAAGCCGTTTGTCAGCCTTCCAT
TTGTTTTGCCTGAACGGCAGTCAGGGCGATGGTAAACACGATATCTTCTACCAGTGCGCC
GCGGGAGAGGTCGTTGACCGGTTTACGCAGGCCTTGCAGCAGCGGGCCGACGCTTAAGAC
GTTGGGGTTGCGTTGGACGGCTTTATAGGTGCAGTTGCCGGTGTTCAGGTCGGGGAAGAC
CAAAACGGTTGCCTGTCCTGCCACCGGGCTGCCCGGAGCTTTGGATTTGCCCACACCCGG
CACGGTTGCCGCATCATATTGCAGCGGGCCGTCGATGGCGAGGTCGGGGCGTTTTTCCCG
GGCAAGTTTGGTTGCTTCGATGACGGTATCGACATCGGGGCCGCTGCCGGAGTTGACGGT
GGAGTAGGAAATCATCGCCACTTTCGGGTCGATGCCGAAGGCTTTTGCGGAATCGGCAGA
CTGGATGGCGATGTCGGCAAGCTGTTGCGCGGTCGGGTTCGGATTAACCGCGCAGTCGCC
GAAGACGAGGACTTGGTTGGGCAGCAGCATAAAGAATACGCTGGACACGAGGCTTGCGCC
CGGTGCGGTTTTAATCAGTTGCAAAGCGGGGCGGATGGTGTTGGCGGTGGTGTGAACCGC
ACCGGATACCAAACCGTCCACATCATTTTGCGCCATCATCATCGTACCGAGTACCACGGT
GTCTTGCAGTTGCTTGCGCGCGTCTTCGGGTGTCAGGCCTTTGGATTTGCGCAGTTCGCA
CATCGGCTCGACGTATTGTTCGACCAATGAGGCGGGATCGATGATTTCCAAAGAGTCGGG
CAGGCTGATGCCGCGTTCTTTGGCAACGGCTTCGACTTCTTCGCGTTTGGCAAGCAGGAC
GCAGCGGGCAATGCCTTTTTCGTGGCAGATGGCGGCGGCTTGGACGGTGCGGGGTTCTGC
GCCCTCAGGCAGGACGATGCGTTTGTCGGCTTGGCGGGCGAAGTCGATCAGGTTGTAGCG
GAATTGCGCCGGCGACAGGCGTTTTGCTTCGCGGCCTGCCAATACGGATACGTCTTTCAG
CGCGTCGCTCGAACCGAAGAAGGTCAGGCCGGTTTTTTCGGCTGCCGCTTCGGCAACGGA
GGCTGCCGCGCCGTCCACGACAAAACCTTCCAATACGCCCGGCGCGGCGGCGAAGAACTG
TTTGGCAAGGTTCACCCGATTTGCCAGTTCGTCGGCATCGGCGTTGTCGGAACGGACGGC
GAAGACGGCTGCCGCGTCAAGGGACAATGCCAGTTCGACGTTTTTGCCTGCGAGGTAGAT
TTTGTCGGCATCGGGCGCGATGCCTTCGATGACGAGGTTGGCGGCATCGAGTGCGGCAAC
TTTGCCGACCAGTGCGTCGAACCAGTCGTCGCTTTTGCCTTGCGCGAGCAGGGTTTCGGC
GGTTGCGTCAACGGCTTGGAAAATTTGTGCGTCCAGTGCTTTTGCAAAGGCTTGTGCGGC
GGCGGAGGCGTCCAGTCCGGCAGATACGGGTACGATGAGTACTTTTGCCATGATATATCC
TTTCGTATGCTGCGGTGTGCGGCATATGTGGTTGGAAGGGGCGGCATATAGGCAGAAACG
GCTGCCTGCGTGCCGTGCGTGCCGTGTTTGGCTTGAGGCGCGCAGGTTGAATATAGCAAA
CAAATTCTGTTTCCAACAAGATAAATATCCGCAGGCTTGTGGATGCTGCCGCCTTTCAGA
GGGTATTTCCGGGGAAGAACAGGGCGGACCGTCCAAATGGAGGACGGCGGAAATGCCGT

## Appendix A

CTGACAGGGTGGGGGCGGAAGGGAGGTTGAGCGTGAGGACGGTTTGTCCGACCCGGAGGC
TGATTTCGGTATGCCGCGCTTTGGGCGTGGTTTTGAGAACCACGGCGTGAATGGAGGCGG
TGGGTGCGGAATGGGGGTGAAGGCTGAACTGTTCCGGACGGATGAGCAGTGTGCCGCGTG
TGCCTGCGGGTGCGCCGCTTTGAACGGGCAGGCGGCCCAATCCGCAATCGGCGGTGCCGT
CGGCGTTGAGCGCGGCGGGGAACACGATGCCTTCGCCGATAAACAGGGCGGCATCAAGGT
CGGCAGGTTGTCGGTACAATTCGTGAGGGCTTGCAGTTTGGAGGATGCGCCCCTGTTTCA
TCACGGCAATCCGGTCGGCGTATTGCAGGGCTTCTTCGCGGTCGTGGCTGACGAAAACGG
CAGATTTGCCGTTGGCGCGCAGGGCGGCAATCATGTCTTCGCGAATCTGGCGGCGCAACT
GTTCGTCCAGCGCGCTGAAGGGTTCGTCCAACAAAATCAGTTCGGGATCGGGTGCGAGGG
CGCGGGCGAGGGCGACGCGCTGTTGCTGTCCGCCCGAAAGTTCGTGCGGATAGCGTCCGG
CAAGTTCGGAAATGCCGGTCAATTCCAACATAGCTTCGATGCGCTGCCGCTCTTGCGCCG
TCTTGCCTTTGCCGTTGCCCAGCCCGTAGGCGGTGTTGCGGTAAACGGTCAGGTGGGGGA
ACAGCACACCTTCCTGTACGACATAACCCAAACGGCGTTCGCGGACGGGGAGGTTGGTAT
TTTTCGAGAAGATGGTTCTGCCGGAAAGCGAAATTTCGCCAAAATCGGGTTGTTCAAAAC
CGGCAAGGCAGCGTAAAAGGGTGGTTTTGCCGCAGCCGGACGCGCCGACGATAAAGAGGA
TTTCGCCCGGGTCGAGGCTGAGCGAAATGTCGTTTAAAACTGGGGTGTTTTGAAAACTTT
TGGACAGGTGTCCGATGTGCAGGGCGGCGGTCATGGCGGTACTTCCTCAAGCTGTTATTT
GAAGGCGTATTTCTTCAGCAGGAATACGGGGATGCCGGAAAATAATACCAGCATCAGCGC
GTAAGGGGTGGCGGCGGCGTATTGTGCGTCCGATGTGTATTCCCAAACGGCGGTGGAGAG
TGTGTGGACATCGTCGGTGGTCAGCAGCAGGGTGGCGGTCAGCTCTTTCATCAGTTTGAG
GAAGACGAGTGCGAATGCGGCGGTAATGCCGGGCAGGATGGACGGCAGTACCAACGTCCT
GAAAATAAAGAAGTGTCCGCGCCCCAATGTTGCGCCGACCTGTTCCATCCCTTTTGGGAG
TTGTTCCAAGGAAGTCCTCAGGGTGGTTTGCGCCATCGGCAGGTAAAGCATGAAATAGGC
AAGGATGACGACGATAAAGGTTTGGTAAACGGCAGGGGTGTAGTTGATGCTGAAATAAAC
CAAGGATAGGGCGATAACCAAACCGGGGACGGCGTGCAGTAAAAACGGCAGCCTGTCTAT
CCAAACGGTTAAAAAATTGCGATAGCGAACCGATGCCCAAACAAGGGGCAAGGCACATAA
TATAGTCAAAATCGCACCTAAAGCCGATACGCTTAAGGAACGGATAAAGGCATCAAATAC
GGATACGAGCGCGAATGTGCCGGAAGTGCCGACCATCATCCAATGTATCAATACGCCAAA
GGGGATAATAATGCCCAAAGTCAACAAGCTGCTTAAAAAAACAATCGCGCCAATCTGACC
GGGCAGTTTGAGGGTTTTGACGGGATAAGGACGGGCAACGCCTTTGCCGCTGTGGTAAAT
CTTGGCTTTGCCGCGAAATATGCTTTCTCCAAATACGACGATGCCGCACACCGCCATTAA
AACAGCGGAAAGCAGGGCGGCGGTATTGTTGTTGTAGGACATTTCGTATTCTTGGAAAAT
GGCGGTGGTAAAAGTGGGGTAGTTCAAAATGGATACCGCGCCAAATTCGACCAGCATATG
CAGGGCAATCAGTAACACGCTGCTGCCGATGGCGGGTTTGAGCTGGGGGAGGATGGCGGA
AAAAAAGGTTTGCAGGCGGCTTTTGCCCAAGGACAGGCTGACTTCTTCGTAAGACAGGCT
GATGCGTTTGAGTGCCGCCTCGACGGGCAGGTAGGCGAGCGGGAACGAGGACAGGCTCAT
AATCATCACTGTCCCCCAAAAGCCTTCGACACGGAAGGTCAGGCTGATCCAGGTGAAACA
GCTGACAAATGCGGGGATGCACAAAGGCAGGGTGATTGCCGTCTGAAAAAAGGTTTTGCC
GAAGAAGCGGTAACGTTGGAACAAAAGGGCGCAGGCAATGCCCAAAACAATGGAAATCAG
GGTAACGCCCGCCATCATCGTCAAGGTGTTGGAGAGCAAATCCCACATACGCGGGCGGAA
CAACAGTTCGACGGCGCGGTTGATGCCGACCTGCCACGAACGCATAGCGACATATAAAAA
AGGCAGGGTAAGCGGTAGGGCAATCAGTAGGATGAGGCCGGTAAGCCAAATGGGTATTTT
TTTAGGAGACATAGTGTTTTTTATCGGCAAAACGGGCGGACAGTATAAATGTCCACCCGT
TTGACAATCCGAAAACGGCTTATTTCATACCGGCTTGCTCAAGCAGCCGGGTGGCGTGTT
CTTTTTCGGAAACAGTGGTGGCGGACACTTGGGGTGCTTCCAACTTGGCGATGGGTTCCA
AATTGAAAGTGGATACCACGTGCGGATTCAAAGGATATTCGGCACGGACGGCGGTCAGGG
CGCGCTGTCCTTCCTTGCTGGCGAGGAAGGCGACGAATTTTTTCGCCTCATCCTTGTTTT
GGGAGGATTTTAACACGGCTGCGCCGGAATAGGTAACGAGTGCGCCGGGATCTCTGTGGC
GGACGAAATTCAGGCGGGTGTGGACATTTTGTACGCCTTTTTCACGCGCAAAAGCGTGCC
AGTAGTAGTTGTTGATGAGGGCGGCATCGATTTCGCCGTTTTCAACCGCTTGAAGGGCGA
CGGAGTTTTTAGCGTAAGGCTTGCCGTATTCTTTCAGACCTTTGAGCCATTTCAATGCGG
CCGCTTCGCCTTTCAGTTTGACGATGGCGACAACCTGTTCCAAGAACGCGCCGGAAGTGG
GGGCGTAACCGATGCGGTTTTTCCATTTCGGCGTGGCGTAATTCAGGACGGATTTTTCCA
AATCTTTTTCAGACAGTTTGCGGGTGTCGTAAACGACGACGCGCGAACGTCCGCTCAGTG
CCACCCAGTCTTTTTTGGCGGCAACCGGCACGCCCTTGCCGCGTGTTTCGTTGATGGTGG
AGGCGGGCAGGGGCTCTAGGAGGTTGGCGGCGGAAAGGGTGGCGAGTGCCGGGATTTGTT
CGGAATAGAATACGTCGGCGGGGCTTCGGCTGCCTTCTTCTTTGATTTGACCGGCAAGCT
GGTCGCCTTTGGCGCTGTTGAGTTTGACTTTGATGCCGGTAGCCCGGGTAAAGGCATCTG
CAACGGCTTGTGCTGCTTCTTTGTGTTGGCCGTTGTACACGGTAATGTCTGCCAGCGCGG
GGGTTGCGGCGGTCAGGGCTGCGGCAAGCAGTGCGTATCGGATAGATGTTTTCATATCGA
TTTTCTCCTAATGAATGAGAGTGTATACCTTGTTAAGACATAACGGTGTGTAGTGTATTC
CTTCTTTTTTATAAATGCAAATAATTATTTTTTAAATTTGTTGTTGTCCGATCCGGTTAT
TGTTTGTTCTGACTTGTATTTTTTCCGTGAGTCTCGCCCGTAAGGCGGAAGTGGCGGGCA
ATGCGTGGCGGAATGTGGGTAAAGGCGGCATTTTGATTTGTCGGAATGCTTGAGAACCCC
TCTCTTTAAAACACCCTTGGATTCGGATTTCAAGTGCAACACTAGTGTATTAGTGGTTGG
AACAGATTCAAGAATAAAACACTTGGCGTTTCGTAGCCAAGTGTTTTTCTTGGTCGGTGG
TTCAACTCATCTTGAACCCTGCGTATCTCCCGATCACTGATGTTACGGAAATCGGTTTGT
TTGGGGAAGTATTGCCGGATGAGTCCGTTGGTGTTCTCATTCAGCCCTTTCTCCCAAGAA
TGGTAAGGACGACAAAAATAAGTCTCCGCTTTCAATGCTTTGGTTATTTTGGTGTGTTGG
TAGAACTCTTTGCCGTTATCCATGGTAATGGTGTGCACCCTGTCTTTATGTGCCTTTAAT
GCCCTAACAGCTGCCCGGGCAGTGTCTTCGGCTTTGAGGCTATCCAATTTGCAGATGATG
GTGTAGCGGGTAACGCGTTCGACCAAGGTCAATAATGCGCTTTTCTGTCCTTTGCCGACA
ATGGTGTCGGCTTCCCAATCGCCGATACGGGATTTCTGGTCGACGATAGCGGGTCGGTTT
TCTATGCCGACACGGTTGGGTACTTTGCCTCTGGTCCATGTGCTGCCGTAGCGTTTGCGG
TAGGGTTTGCTGCATATTCTGAGATGTTGCCACAACGTGCTGCCGTTGCTTTTGTCTTGG

## Appendix A

CGAAGGTAGCGGTAAATGGTGCTGTGGTGGAGCGTGATCTGGTGGTGTTTGCACAGGTAG
GCGCATACTTGTTCGGGACTGAGTTTGCGGCGGATAAGGGGGTCGATGTGCTGAATCAGC
TGCGAATCGAGCTTATAGGGTTGTCGCTTACGCTGTTTGATAGTCCGGCTTTGCCGCTGG
GCTTTTTCGGCGCTGTATTGCTGCCCTTGGGTGCGGTGCCGTCTGATTTCGCGGCTGATG
GTGCTTTTGTGGCGGTTCAGCTGTTTGGCGATTTCGGTGACGGTGCAGTGGCGGGACAGG
TATTGGATGTGGTATCGTTCGCCTTGGGTCAGTTGCGTGTAGCTCATGGCAATCTTTCTT
GCAGGAAAGGCCGTATGCTACCGCATACTGGCCTTTTTCTGTTAGGGAAAGTTGCACTTC
AAATGCGAATCCGCCACCGTCTGAACAGGGTTGCTGGAATAGTATTGCCATCCCAGCAGA
TACAGTTTGTCGGGGTCTTGCCAATATTGTTCATCCAGACTGTTCAGCAGTGAGGCGGTT
TTGTTGTCGAGATGTTATATCCCACATTTCTTTCAGGTTTTTACCTTCCGATTGGAGGCG
GCGGATTTCTTTGTCCAATGCGTCTGCCGCCCGATGGATCAGCATTGCGCTGTGTACCGC
GCCGATTTTTTTCAGAACGGAACAAGTCTTTTCGGCGGCGGGGAAACCCCAGTTGCAGAC
AAATTGCAGTATGCTGCCGTTACCGATATCGGCTTCTGTCCGCCAAATCAAAACCAGCTC
CTGTTCCCGCCCGTCCATGCTTTCGAGTTTGCCGTCATGCTGTTCAAAAAGTTTGTCCAC
CGCCTGCCACATCATCTGTTCGAAGCGGTCGGCTTGGGTATCCGTATCGGTCATCGTGTT
CTTGCCTTTTAAAAATGCCGTCTGAACATTTCTTCAGACGGCATTTGGGGGTTAAGCCAA
CATTTCCCGCCAGCGTTTCACTTGGAAGCGGACTTGTTCCGGCGCGGTACCGCCCAAGTG
GTTGCGGGCGTTTAAGCTGCCTTCGGGTGTCAGCACGCCGTAAACGTCGTCGGCAATCAA
ATCGCTGAAACCTTGTAAGACTTCGAGCGGCAGTTCGCTCAAATCGACGCCCGCTTGGTC
GGCGTGGCGCACGGCTTGGGCGACGACTTCGTGGGCATCGCGGAAAGGCATGCCTTTTTT
GACCAGATAATCCGCCAAGTCGGTGGCGGTAGCGAAGCCCTGCATCACGGCGGCGCGCAT
ATTGTCGGGTTTGACGGTTACGCCGCGCATCATATCGGCGTAAATCCGCAACGTGTCGAT
AAGCGTGTCGGCGGTGTCGAACAAGGGTTCTTTGTCTTCCTGATTGTCTTTGTTGTACGC
CAAGGGTTGGGATTTCATCAGGGTAATCAGACCGATAAGGTGTCCGATGACGCGGCCGGA
TTTGCCGCGCACGAGTTCGGGCACGTCGGGGTTTTTCTTCTGCGGCATGATGGACGAACC
TGTGCAGAAACGGTCGGCGATGTCGATAAAGCCGAAACGCGGGCTCATCCACAAAATCAA
TTCTTCAGACAGGCGGCTCAGGTGAACCATAACCAGCGAGGCGGCGGCTGTGAACTCAAT
GGCGAAATCGCGGTCGGATACGGCATCGAGCGAGTTCTGGCAGATTTGTTCAAAGCCCAA
TAGCTCGGCGGTGATTTCGCGCTGAATCGGGTAGGTCGTCCCGGCAAGGGCGGCTGCGCC
GAGCGGCATACGGTTGACGCGGCAGCGGCAGTCCGCCATCCGTTCGTTATCGCGTCCGAG
CATTTCGACGTAGGCGAGCATATGGTGTCCGAAGCTGACGGGCTGGGCGACTTGCAGGTG
GGTAAAGCCTGGCATGACGGTTTCGGCGTTTTGTTCCGCCAAATCCAGCAATGCCGTCTG
AAGGCTTTGAATCAGGCTTTGTATAACGGTAATCTGGTCGCGCAGCCACAGGCGGATGTC
GGTGGCGACTTGGTCGTTGCGGCTGCGGCCGGTGTGCAGGCGTTTGCCCGCGTCGCCGAT
TTTGTCGGTCAGGCGGCGTTCGATGTTCATATGGACATCTTCCAAATCGGACGACCATTC
GATTTTGCCGCTGCGGATTTCTTCGAGGATTTCCGCCATACCCCGGCGGATGTCCGCCAA
ATCGCCTTCGTCCAACACGCCGGTTTCTTTCAGCATTTGCGCGTGTGCCAGCGAGCCTTG
GATGTCCCATTCGGCAAGCCGTCGGTCGAAACCGATGGAGGCGGTGTATTGTTTGACGAG
TTCGGAAACGGGTTCGTTGAAACGTCCGGACCAGGTTTTGTCGTGCATAAGGATTCCTTG
ATGGGGTTATTCGGTGCGGTATTTTTCCAAAAGCCGGCGGAAGGGTTCGCCGGTTTCGGG
ATGTTTCAGACCGTAGGCGACGGTGGCTTCGAGGTAGCCCAGTTTGCTGCCGCAGTCGTA
GCGCGTACCTTCAAAGGGGTGCGCCAGGACAAATTCGTGATCGAGCAGCTTGGCGATGCC
GTCTGTAAGCTGGATTTCGTTGCCCGCGCCGCGCGGAAGATTGGTTAAGAGGTCGAAAAT
GCGCGGGGTGAGGATGTAGCGTCCAACAACGGCAAGGTTGGAGGGCGCGTCTTCGGGCTT
GGGTTTTTCGACAATGCCGGTAATGCGTTGGAACTGTTTGAGCTGTTCGGTTTCGACGAT
GCCGTATGAGCCGGTTTGCGATGCTTCAACGGTTTCTACGCCCAAAATGCTGTTGCCGCT
GCGCCCGTACACTTCGACCATTTGTTTGAGCGCGCCTTTGGGGGCATCAATCAGGTCGTC
GGCAAGGATAACGGCAAAGGGTTCGTCTCCGATGGCGGCGCGGGCGCACAAGACGGCGTG
TCCCAAGCCCAGTGCTTCCGCCTGACGGATGTAGAGGCAGGTAATGTTCGGCGGCAGGAT
GTTGCGGACGTGTTCCAACAATTTGTCTTTATGGCGCATTTCCAACTCGGTTTCGAGTTC
GTATGCCTTGTCGAAATGGTCTTCGATGCTGCGTTTGTTGCGTCCGGTAACAAACACCAT
TTCCGTGCAGCCGGCTTCCACGGCTTCTTCTACGGCGTATTGGATCAGCGGCTTGTCGAC
GATGGGCAGCATTTCTTTCGGGCTGGCCTTGGTGGCGGGCAGGAAGCGGGTTCCCATCCC
TGCGACGGGGAAAACGGCTTTCCGTATGGGTTTCATTCTTTTTCCTTTGTATTGTTTTGA
TGTTTAAAGGGCGAGTTTGCGTAAGAGTTCGGCAAGTGCCTGCGCGCGGTGGCTTTCGCG
GTTTTTGACCTCCGTATCCAATTCGGCGGCGGTTTTGCCGTGTTCGGGCAGATAAAAATA
CGGGTCGTAACCGAAACCGTTTTGCCCGAGCGGCGTGTCGTTCCACTGCCCGTGCCATAC
GCCCTCGGCGATAATCGGGCGCGGGTCGTCTTTATGGCGGACAAAAACCAATACGCAGAC
ATAGCAGCAGCTTTTGTCTGCCTTGCCGACAAGTTCGGCGGCAAGTTTCAGGTTGTTGGC
GGTATCGGATTTGGGATTGTCGCCCGCGTAACGTGCGGAATGGATGCCCGGCGCGCCGTT
TAAGGCGGCGGCACAGATGCCGCTGTCGTCGGCGAGTGCGGGCAGCCCGCTGTATTTGGC
GGCATGGCGTGCTTTTGCCAGCGCGTTTTCGACAAAGGTGGGATAGGGTTCGGGGCATTC
GGGTATGCCGAATGCGGATTGCGGCAATACGGTGATGCTGTAAGGTTTGAATAAGTTGCC
GAACTCTTCGAGCTTGCCGGCATTGCCGCTTGCCAAAACGATTTTTTCCGGTTTTTCAGA
CATAGCGGTTTTCCTTTGTGGCGGATTGGGCGGCGCGTAGGGATTTGTGCCGCAGGTAGA
GGGCGAGGCTGCCGATTTGTCCGAACAGTGCGCCGAATGCGAAAAGAAAGGAGGCGACGG
CGAAGGCTTTGCTGCCGACGGTCAGCAGATAAGCACCCAAGAGGACGAGCAGCATAAATG
CCAGTGTGAAGAGGGCAACAGACAACAGATAGATTTTTCGGCGGTTCATGGCGTTCGGTC
GGAAACGGTATGTTCGGATTATAGCCGATTGGGACGGTATTCCCTAGAGCTTGGAAAAAT
GCCGTCTGAAACGGCGTTCAGACGGCATGGCGGGCGTTATGCCTGTTTGTTCCAACGTTC
GATGGATTCTTTGATGACTTTTTTCGCTTCTTCCGCATCGCCCCACGATTCGACTTTGGT
CGTACCTGCTTTTTTCAGGTCTTTGTAATGGTTGAAGTGGAACTCGATTTGTTTGATGAG
CTGTTGCGGCAAATCGGACAAAGTTTTGTAGGCGTTGCCGTTGTTGCGGTCGTCGGCAGG
AACGCAGACGATTTTGTCGTCCACTTCGCCGTCGTCAACGAATTTCATCACGCCGATAAC
GCGCGCTTCCAAGAATACGCCGGTTGCCAAAGGTTGTTCGGTAACGAGCAGCACGTCCAA

## Appendix A

TTCGTCGCCGTCTTCGTCCAAAGTTTGGGGAATGAAGCCGTAGTTGGTCGGTTTGGCGAA
GATGGCGGGTTCGACGCGGTCGAGTTGGAATGCGGCCAGTTTGCGGTTCCATTCGATTTT
GTGGTTGCTGCCGGCGGGGATTTCGTTGACAACGTTGATGATGCCGCCGTCCACGTCGCC
GGGGGTCAGGATTTGGTTGAAGTCTGCCATTTGGTTTCCTTTGTTTGGGAAAGTGTGAAG
TTTGAAAGTATAGCACAAACGTCCGGCTGAAAATGCGCCCGATGCCTCTGAAAGGGTGTA
CGGGCGCGTGTTACCGTTTGCCCAAAAACCTGCCCAGTTCCAAAATCGCGCGCCTGTTGG
ACGGGGAAAATACTTTTTCCGCTGCTTCTTCCATATCGAACCAACCGTAGGAGACGTGTT
CTTCGGGTTGCAGGGCGATGGGCGTATCACGCGGGATTTCGGCAGAGAAGAGGTGTTCGC
GGTTTTCAAACACGCCTTTTGGATAGCGGTGCCGCCAGTGGTGGTAGATTTCGTAAACCG
TGCTGTCGTGCCAGTCTTGAAGCTGCCCGTCCGCCAGCAGGATGCCGGTTTCTTCCCAAA
CTTCGCGCCTTGCCGTTTGGGCGACGGTTTCGCCCGGTTCGAGGCTGCCGGTTACCGACT
GCCAAAATCCTTCCGGATGCGTGCGTTCGATGAGCAGGATGCCGCCGTCCCCGCTATAAA
GGACGACCAGTGCGGAAACGGGGTATTTGAGCGGTTTTGCCATCGGCATCTTTCGGCGGG
CTGCGGTAATGAAGGGGCGGATTATAGCAAACGCCGCACGTTATGGCGTTTATCCTTTTC
CGTATCCTTTTTTCTGCACGGATGGGACGCGCCGGTGTTTGCCGGTAAATTTTCCGTTGT
GTCAAAAAGATAAGGGCGGTTGTGATTTTAATGCTTGCCAAAGCGTCGGGCGGAAACTAT
AATCCGAAACTTATCGAGTCGGAGTGTGGCGCAGTCTGGTAGCGCACTTGCATGGGGTGC
AAGGGGTCGAAGGTTCGAATCCTTTCACTCCGACCAAAAATTCCGAAAGCCGCTTTCAAA
AGCGGCTTTTTTGCCGTCCGTATGATTATGATGTAGAGTACGCGGCGACAGACATTCAAA
TGCCGTCTGAAAACCGTTCAGATGGCATCTCTTTATCTTAGTTTCATTCCGTACCATCTT
AAGGAACATCAAATTGGGCATTTCCCGCAAAATATCCCTTATTCTGTCCATACTGGCAGT
GTGCCTGCCGATGCATGCACACGCCTCAGATTTGGCAAACGATTCTTTTATCCGGCAGGT
TCTCGACCGTCAGCATTTCGAACCCGACGGGAAATACCACCTATTCGGCAGCAGGGGGGA
ACTTGCCGAGCGCAGCGGCCATATCGGATTGGGAAAAATACAAAGCCATCAGTTGGGCAA
CCTGATGATTCAACAGGCGGCCATTAAAGGAAATATCGGCTACATTGTCCGCTTTTCCGA
TCACGGGCACGAAGTCCATTCCCCCTTCGACAACCATGCCTCACATTCCGATTCTGATGA
AGCCGGTAGTCCCGTTGACGGATTTAGCCTTTACCGCATCCATTGGGACGGATACGAACA
CCATCCCGCCGACGGCTATGACGGGCCACAGGGCGGCGGCTATCCCGCTCCCAAAGGCGC
GAGGGATATATACAGCTACGACATAAAAGGCGTTGCCCAAAATATCCGCCTCAACCTGAC
CGACAACCGCAGCACCGGACAACGGCTTGCCGACCGTTTCCACAATGCCGGTAGTATGCT
GACGCAAGGAGTAGGCGACGGATTCAAACGCGCCACCCGATACAGCCCCGAGCTGGACAG
ATCGGGCAATGCCGCCGAAGCCTTCAACGGCACTGCAGATATCGTTAAAAACATCATCGG
CGCGGCAGGAGAAATTGTCGGCGCAGGCGATGCCGTGCAGGGCATAAGCGAAGGCTCAAA
CATTGCTGTCATGCACGGCTTGGGTCTGCTTTCCACCGAAAACAAGATGGCGCGCATCAA
CGATTTGGCAGATATGGCGCAACTCAAAGACTATGCCGCAGCAGCCATCCGCGATTGGGC
AGTCCAAAACCCCAATGCCGCACAAGGCATAGAAGCCGTCAGCAATATCTTTATGGCAGC
CATCCCCATCAAAGGGATTGGAGCTGTTCGGGGAAAATACGGCTTGGGCGGCATCACGGC
ACATCCTATCAAGCGGTCGCAGATGGGCGCGATCGCATTGCCGAAAGGGAAATCCGCCGT
CAGCGACAATTTTGCCGATGCGGCATACGCCAAATACCCGTCCCCTTACCATTCCCGAAA
TATCCGTTCAAACTTGGAGCAGCGTTACGGCAAAGAAAACATCACCTCCTCAACCGTGCC
GCCGTCAAACGGCAAAAATGTCAAACTGGCAGACCAACGCCACCCGAAGACAGGCGTACC
GTTTGACGGTAAAGGGTTTCCGAATTTTGAGAAGCACGTGAAATATGATACGAAGCTCGA
TATTCAAGAATTATCGGGGGGCGGTATACCTAAGGCTAAGCCTGTGTTTGATGCGAAACC
GAGATGGGAGGTTGATAGGAAGCTTAATAAATTGACAACTCGTGAGCAGGTGGAGAAAAA
TGTTCAGGAAATAAGGAACGGTAATATAAACAGTAACTTTAGCCAACATGCTCAACTAGA
GAGGGAAATTAATAAACTAAAATCTGCCGATGAAATTAATTTTGCAGATGGAATGGGAAA
ATTTACCGATAGCATGAATGACAAGGCTTTTAGTAGGCTTGTGAAATCAGTTAAAGAGAA
TGGCTTCACAAATCCAGTTGTGGAGTACGTTGAAATAAATGGAAAAGCATATATCGTAAG
~~AGGAAATAATAGGGTTTTTGGTGGACAATACCTTGGCAGGATACATGAATTAAAATTTAA~~
AAAAGTTGACTTTCCTGTTCCTAATACTAGTTGGAAAAATCCTACTGATGTCTTGAATGA
ATCAGGTAATGTTAAGAGACCTCGTTATAGGAGTAAATAAAAATGGCAATTTGGAATTAT
CGGTACTACCTTATCCCATCAGCTGCTATCAGAAATAAATTTAATGCAAATAAAGATATC
ATACTTGATGAGTATCGATCTAATGGTTTTCAGAATTTTAATGAGAATAAAAGTTTTGAA
AATTACTTTATCGATAATGATGTTATATTATTATCAATAATAAATGAAGCAAAAAAACAG
CTTAAATTGAAAGAATCTTGGGATAAAGACGCAATCATGTTTTGTGATAATTTTGGTAAT
AGTCTTACCGTTTGGCCAGATGATATAGAGTGCGAACTTGATTTAAGATTTGATTATACT
AAATTTATTCAGAAAACCATTGATTGGGCAATAAAATATAATTGTCTACTTGTAATAGAA
AAAACAGGAAATGTAGTTTCCCCTAATATAAATAATCTGATGTATGAAATAAAAGCATAT
TTGGAAAGCAAGCCGTGGCCCATATGAAACCTAAACTCAACAAGTAGGATGTGTGCGGAA
CGCACGTATGCGGTTCTCAAGGTTTGAGCTAAGAGGCCGTCTGAAAACAGAAAAACTGTT
TCAGACGACCTTTCTTTTAACCAGTTGCCACAGCAACCGGACAAAAGCAGCCTACCTCCA
CATCCATATAGGCAATACAGGGGAGATATTTTGTAAATTCTACGAATATTTTACCTGCTA
AACAGGGTAGGATATGGTATGAAGCGAACATTGGCTTAATAAACACTATGTCAAGATCGA
ATCAGGCTGGTACTAGATTGTTGTATTCCAATTATGGATTGCTATATATAACAACTGATC
ATTATATCTCTGCAACACGGTTTGTAGCTTGGAAATAGGAGTATAACTTATGCAATTAGA
GATTATCGGTAGTAAAATTTATACGGAACAAGATTTTCATAATCAAATTTCAAAAATATT
TTCTATACAAGATTATTATGGGAACAATCTTGATGCTTTATGGGATTTATTAAGCACAAA
TGTAGAACGACCGATTACTTTGGTATGGAAAGATGCTATGTTCTCAAAAAATCAATTAGA
AAATATATTTATTGAAATCGTAAATGTTCTAGAAAGAGTTAAGAAACAAGATGAGGATTA
TGGATTCGAAGAAAAATTTAATTATATTTTAGAGTAAGTAAACCCTAATTACATTACGTA
CACAGGCTTAAAACTCCCCAGAGCCAATTAAGCAAGCCGTAACCCATATAAAACTTAAAC
TCAACAAGTAGCATGTGTGCGGAACGTACGCATGCGGTTCTTAAAGTTTGAGCTAAGAGG
CCGTCTAAAAACAGAAAAACCGTTTCAGACGGTCTTTGTTTAACGCCACCGATCCAGCGG
GTTACAAAGCGCAGTCAATGCCGCTGCGCCTTATGCCTCCGAAGCAATAGGCAGAACATT
TGGACACGGTGAAAACAAAAACGAAACCGCCCAAGCCGTCGGACATTTCCTTTTAGGAGC

## Appendix A

AGCTATTGCCCGCGTCAACGGTGGTAATTTTGCTGCCGGCGGCTCGGCAGCAGTTGCAGC
TGAAAAGGCGGCGGAACATCTTGCCCAACAGTATAACGACGGTAAAACCGCAATCGATCC
GCAAACAGGCGAGTTCAATGCCAACCTGCTGCCGGAACATATCAAAGAGGAAATCAAATC
AAAGAGCGGGGTGATTGCATCGCTGACGGGCGCGGCCGTGGGCGGCACGCCGGTAGATGC
GCAAACCGGAGGTGCGGTCGGACAGAATGCGGTGGAAAACAACCTCTATCTGACATCGGA
AGCCTTAAAGAAGGACAAGCAGACAGCTCGTAAAATTTATTCCGTCATAAAAGAGCAAGT
CAAGCATGAATGCAGTTCCACAGGAAGAATTACCGAATGTCGTCAAAATATAGGACGCAT
TATCGAATTTACCCAAGACAAACGCTTTGACAGTAGGTTTAAGGACTTAAAAAAAGAATC
CTTATATTACCTAAATAAACATCCTGATTTAGTAGCCTCTTATTTGAAGGCTGAATACGA
AAAGCTGGATAGGGAAGACAAAAGTATCCTGCACCGCTACATCTCACCCGGGGCTGAAAT
CGTTTCGGGCAGTTTGGGGGTTGTTCTTTCAGGAGTAGCCGGAGGCGGATCTTGTGCCGA
GACTTTCGGCTTAGGCTGTGCCGCCGCTTTGGTTGGTGTAACGTCTTCCTACGATCATGT
CATTACTGGGACGAAAAACTTCGGAAAAAAAGCCAGCGAGCAACGACCGACGATTGCGGT
TCAGGCCTTGAAGCAGTTGGGGCTGTCGGAGCAGGCTGCGGAATATGTTCAGTTCTCTAT
AGATTTGTTCAGTGTGGGTAAATCGGGGGGCGGTATACCTAAGGCTAAGCCTGTGTTTGA
TGCGAAACCGAGATGGGAGGTTGATAGGAAGCTTAATAAATTGACAACTCGTGAGCAGGT
GGAGAAAAATGTTCAGGAAACGAGAAGAAGGAGTCAGAGTAGTCAGTTTAAAGCCCATGC
GCAACGAGAATGGAAAATAAAACAGGGTTAGATTTTAATCATTTTATAGGTGGTGATAT
CAATAAGAAAGGCACAGTAACAGGAGGGCATAGTCTAACCCGTGGTGATGTACGGGTGAT
ACAACAAACCTCGGCACCTGATAAACATGGGGTTTATCAAGCGACAGTGGAAATTAAAAA
GCCTGATGGAAGTTGGGAGGTGAAAACGAAAAAAGGTGGGAAAGTGATGACCAAGCACAC
CATGTTCCCAAAAGATTGGGATGAGGCTAGAATTAGGGCTGAAGTTACTTCGGCTTGGGA
AAGTAGAATAATGCTTAAGGATAATAAATGGCAGGGTACAAGTAAATCGGGTATTAAAAT
AGAAGGATTTACCGAACCTAATAGAACAGCATATCCCATTTATGAATAGTAATATTTATG
AAAAATTAGGAGATTAATGATGAAAAGAATTAAGTGCTTTTGTGATAAATTTCCATCAGG
AGATACATTTAGAATGTGTATCATTCTGGATGACTATGATAATAGGGTTGATTATTATGT
AGGAATATATGATTACATTACGTCTACCTTAATGAGCGATATTTACTATCGATCCACGAT
TGATGAGCATTTCAAGATTATAGAATTAATAGAAAATAATCCAAATGAAATTTATGATGA
TGGCGGTGGTCAACAATTTTGCCTAGAATTTCATCATGATAAGGTCATTTTTTACCACAA
TGAATTTGATGAAGAAGATGGTTATCCAGTATTAAGCTGTTCGCTGCATACTTTTAAAAC
TGCTTTAATTGCTTGGAATGCTTTTTTGCAATTGCCTAAAAGTATTCATTCGGTGGTGGA
GACTGTGATTGAGGAATAAGCATAATTAGCTTAATGAATAGAATCAGCGATATAGATTGG
ACTGCAAATCCACGCTTATACGCTGTGCCATGATTAAGATGTTAGAACTTGTATTGAATA
CAAGTTCTCATAAACGAATGGCAGTAAGCATTTGATTTAGATAAAATCCTTGAATTAGAA
TAATCAGGTCTAAGAGCTCGACAGGACAAATGAGGCTGGCAACCAAGGATTTGGCGGAAG
CCATTAGGAAAGGACAGGTTCGCAAATCAAGCTTTAACACAGAACAATTAAGGGCAATTG
AAAAAGGAGAATCTAAAATACCGGATTACACTTGGCATCATCATCAAGATACAGGAAGGA
TGCAATTGATTCGTGAAGGCTTGCATCATGATACCGGCCATATTGGTTGGGAAGCAATGA
ACAAAGGAAGGTAACTATGTGGAAAATCATAAAAGAGGATAGTGATGATTTAGAATTTGC
AATTAAATGCTTATTCTCTCAGTCTATTGATTTAAATGAATTCAAGTTATGGATTGAACA
AGTAATACGCGATATGCCCATCGAGGACATCCCTTTTTATATTTTTGATTTGGCGGATTT
TGATGGGGGAATTGCCGATATTGACAATATTGTAGGTTTTGTTTCAAGTTGCAGACTATC
AAAATCGAAAAAAAATGCCTTGACCGGCATTGCCTTCTTAAGGGGGATAGATGTCTATGA
TCCGCCTATTTCAAAAGAAAAAGCATTAAAAGCCTTAGAGAAACATCCTGAAATTTATCA
GAAATTTCAGCATTTCTTTCCGTTTGTAGAACTGCCCCCGCTTTAAACAGTCAAAATGCC
GTCTGAAACGATATTCGGCTTTCAGACGGTATTTTTGATATAAAGCGGGTAACTAAAAGA
GCGTTTGACGGCAAAGGAAGATAATTATGTGGAAAATCATAAAAGAGGATAGTGATGATT
TAGGATTTGCAATTAAATGCTTATTCTCTCAGTCTATTGATTTAAATGAATTCAAGTTAT
GGATTGAACAAGTAATACGCGATATGCCCATCGAGGACATCCCTTTTTATATTTTTGATT
TGGCGGATTTTGATGGGGGAATTGCCGATATTGACAATATTGTAGGTTTTGTTTCAAGTT
GCAGACTATCAAAATCGAAAAAAAATGCCTTGACCGGCATTGCCTTCTTAAGGGGGATAG
ATGTCTATGATCCGCCTATTTCAAAAGAAAAAGCATTAAAAGCCTTAGAGAAACATCCTG
AAATTTATCAGAAATTTCAGCATTTCTTTCCGTTTGTAGAACTGCCCCCGCTTTAAACAG
TCAAAATGCCGTCTGAAAGCCATTTCCGCCGCTCAGACGGCATTTTCGCCCCTTTTGTTT
ACAAACCCTTAAAATCCCTTTACACTCAAAATCCGTTCAACATCAAACAAACCCCGCTAT
GAAAACCCTGCTCCTCCTCATCCCCCTCGTCCTCACAGCCTGCGGCACACTGACCGGCAT
ACCCGCCCACGGCGGCGGCAAACGCTTTGCCGTCGAACAAGAACTCGTCGCCGCATCGTC
CCGCGCCGCCGTCAAAGAAATGGATTTGTCCGCCCTAAAAGGACGCAAAGCCGCCCTTTA
CGTCTCCGTTATGGGCGACCAAGGTTCGGGCAACATAAGCGGCGGACGCTACTCTATCGA
CGCACTGATACGCGGCGGCTACCACAACAACCCCGAAAGTGCCACCCAATACAGCTACCC
CGCCTACGACACTACCGCCACCACCAAATCCGACGCGCTCTCCAGCGTAACCACTTCCAC
ATCGCTTTTGAACGCCCCCGCCGCCGCCCTGACGAAAAACAGCGGACGCAAAGGCGAACG
CTCCGCCGGACTGTCCGTCAACGGCACGGGCGACTACCGCAACGAAACCCTGCTCGCCAA
CCCCCGCGACGTTTCCTTCCTGACCAACCTCATCCAAACCGTCTTCTACCTGCGCGGCAT
CGAAGTCGTACCGCCCGAATACGCCGACACCGACGTATTCGTAACCGTCGACGTATTCGG
CACCGTCCGCAGCCGTACCGAACTGCACCTCTACAACGCCGAAACCCTTAAAGCCCAAAC
CAAGCTCGAATATTTCGCCGTTGACCGCGACAGCCGGAAACTGCTGATTACCCCTAAAAC
CGCCGCCTACGAATCCCAATACCAAGAACAATACGCCCTTTGGACCGGCCCTTACAAAGT
CAGCAAAACCGTCAAAGCCTCAGACCGCCTGATGGTCGATTTCTCCGACATTACCCCCTA
CGGCGACACAACCGCCCAAAACCGTCCCGACTTCAAACAAAACAACGGTAAAAAACCCGA
TGTCGGCAACGAAGTCATCCGCCGCCGCAAAGGAGGATAAACCGTGAAACCGCTGCGCAG
ACTGACAAACCTCCTTGCCGCCTGCGCCGTAGCGGCGGCCGCACTCATACAGCCCGCCCT
CGCGGCGGACTTGGCGCAAGACCCGTTCATTACCGATAACGCCCAACGGCAGCACTACGA
ACCCGGCGGCAAATACCACCTCTTCGGCGACCCGCGCGGCAGCGTTTCCGACCGCACCGG
CAAAATCAACGTCATCCAAGACTATACCCACCAGATGGGCAACCTGCTCATCCAACAGGC

## Appendix A

AAACATCAACGGCACAATCGGCTACCACACCCGCTTTTCCGGACACGGACACGAAGAACA
CGCCCCCTTCGACAACCACGCCGCCGACAGCGCGAGCGAAGAAAAAGGCAACGTTGACGA
AGGCTTTACCGTATACCGGCTCAACTGGGAAGGACACGAACATCATCCCGCCGATGCCTA
CGACGGCCCGAAGGGCGGCAATTACCCCAAACCTACGGGCGCACGAGACGAATACACCTA
TCACGTCAACGGCACAGCCCGCAGTATCAAACTCAATCCGACCGACACCCGCAGCATCCG
GCAACGCATATCCGACAATTACAGCAACCTCGGCAGCAATTTCTCCGACCGCGCCGATGA
AGCCAACAGAAAAATGTTCGAGCACAATGCCAAGCTCGACCGCTGGGGCAACAGCATGGA
GTTTATCAACGGCGTCGCCGCCGGCGCGCTCAACCCCTTTATCAGCGCGGGCGAAGCCGT
TGACCAGTGGATGCAGGAAAACCCCAATGCCGCCGAAACCGTCGAAGCCCTGGTCAACGT
CCTGCCGTTTGCCAAAGTCAAAAACCTGACAAAGGCGGCAAAACCGGGGAAGGCTGCGGT
TAGTGGGGATTTCTCAGACTCCTACAAGCATAACACTGCTTCAAGATTATCTCAGTCTGT
AGATGGAGAAATGTTTCAAACCCGCAATGTTGATTTTAAAGCAAAATCTATTGGGACTAA
AATTCATGATGGAGCTCAAGGGAAACATATTTCAGGACATAGAAACTACATTGAAGGTAA
GAGTACTTTAAATCAAAACATTAATCCTCAAGAATTGTTGAACGGAATACATTCAGGTGC
TTATCCAGTTATTTCTAAAGGAGCAAGAGGAAATCCTGTTGTTGATTTTGGGTATCCTAT
AGGCAGCGATGGGAAATCAGGATTAAGTACCAATTTTGGTACGATTCATTCAGGTAAAAA
TGGAGTTCACATTGTTCCGGCTAACCCTAAAACCATTAAAAAGGTGCAATAGTTATGAAT
ATATTACCAAGCTGGCTGCGAGTCGGTATGAATATAGCAATGCTGGGCATGATACACTCA
GATATCAGGTTAATTACCGTAGATTACGAGGAAGGAAGAAGGTTTTTAAAAATCAAAAAT
TATTTATCAAGAGAAGCCATCACAGAAGACCATGAAGATATGGAATATTTGATTACAGAG
TTATGGTCTATGTGTGGAGAATATTTTGATGAAGCTGACTTTGAATGTATTTATTCTAAT
CATTCTTCTATGGAGTTAAACCAAATAAATGGTGCAGTATTCAGGAGAAAGGAATTAATT
TCGCAAGCGTAGGTTAAAAAAACCAACAATCACAATGTCTTCTGAAACCGTGTTTAATTT
TCAGACGGCATTTCCTTCATTTGAAATAGGATATTGAGAACTGAGTTCTTCAAAAATCCT
ACACCTGCTCCTTCCACGGCAGCACCTTGGTCAAAACGGCAGACGGCTACAAAGCCATTG
CCCGTATCCGAACCGGCGACCGCGTCTTCGCCAAGGACGAGGCAAGCGGAAAAACGGGAT
ACAAACCCGTTACCGCCCGATACGGCAATCCGTATCAAGAAACCGTTTACATTGAAATTT
CAGACGGCATCGGCAACAACCAAACCCTGATTTCCAATAAAATCCACCCGTTTTACAGTC
AAGGAAAATGGATACAGGCAGGTCGTCTGAAAAAAGGCGACACCCTGCTTTCCGAAAGCG
GCGCAAAACAGACGGTTCAAAACATTACCTTCAAACAGCAGCCGCTCAAAGCCTACAATC
TGACCGTCGCCGATTGGCATACCTACTTCGTCAAGGGCAGTCAGGCGGAAACGGAAGGGG
TTTGGGTTCATAATGATTGTCCGTATGATAAAGGCAACCAACGATATAAAGACGCTTCTT
ATCATGGCAAAAATGATAATTCTGTGAAAAGTAGAGCACCAACAAACGGACAAGCAGCTC
TTGATAATTCCGTTCAAGTTAAATCAACTTCTCCTCGAAGAGTTGGGGTTGATAAAGCCA
ATAATGAAATCGTTGTATTAAACAAAACTCAAACTTTTAATAACGGTTCTGCGGAATATC
ACGGGCATGTCAGAAGTTGGCAAGATTTGCATACCGATCAGAAAAATGCTTTAAAAAAAG
CAGGATTGGATTAGTTAATTCAAAAGGAAAAATTAAAAAATGACTGATAAAAGTAAAACA
GAAAAGTTGATTTCTTCTGATGATAAACAAAGTGTTATAGATGGCATTCTTGATATGGTA
TTTAATTCCAAAGCATATGAAGTACCGTGGATTTCTGAGAAATTGATGGAATTATCGAAA
AATAAAGACTTGGATATTGCCGGATTATCGCTAACCTGTTTCGGACATCTCGCCAGGCTA
CATTCAAATATCGGTGATTACGATAAAGTTATTCCTTTACTACATTCAAAGCAAGATGAT
CCAGAGCTTCAAGGTAGGGCTGAAGATGCGTTAGAAGATATTTCTTTATTTTTATCTGAA
AATCATTAGGAACCGTAGGTCGGGTTGAAAACCCAACAATCAAAATGCCGTCTGAAACCG
TGTTTAATTTTCAGACGGCATTTCTTTCATTTGAAATAGGATATTGAGAACTGAGTTCTT
CAAAAATCCTACACTTGCTCCTTCCACGGCAGCACCTTGGTCAAAACGGCAGACGGCTGA
AAAGCAAACACCGTCCGTCGTGTTGCCGTTTGCGGATGAGTACGGGTCAACCCCAATGCC
GCCGAAACCGTCGAAGCCGCCTTCAACATTGCCGCCGCCAAAGCCGCAAAGTTGGCAAAA
ACGGTAAAACCGGGGAGATAAAAGCCGATGGCAGGAAAGTAAATGTGAGGATAGACAGTA
CGGAGGCAGACCTGCTTTATCCGGCAGGGCAATAAGAAAACAAAAATTAGATATGGAAAA
CGATTGTGAAGATTAAACCATTACAATTTTCTAACAATAATCACAGATTTTATGTGGACA
AAATTTGTGTTACCGAACAAGATGTGAACATTTTGGCATCCGACCGAAATTATGAATTAA
ATATTGAAATATTTATTGACAATATAATTCATTTTCAAATAACGGATGAATCTTATAAAG
TAAAATTTTCAGAATATTTATTTGAAAATAAAGAAAAAAATGATTGGGATAGAAATCCTG
CTATAAATTATTTTTTCGAGATAATAGATGATAGTTATATGGACTGGTTGAAAGAAGAAA
GTTTTGATTTTTTTGAAAAGAAATATTATAAGGCTTATATTTCTTTTTTAGCGATTCTG
TAATAGAAGTTATCAGCTCGACAGAACCTGTATTTTATTCAAAATAACAAATTATCAAAC
AAAGCTCTGATTAAAAACCCAACAATCAAAATACCGTCTGAAACGATATTCGGCTTTCAG
ACGGTATTTTTGACACAAAGCAGGTAACCAAAGGAGTGTTTGACGGAAAAGGAGAAGCTA
AAATACCGGATGTATCGGTTGGGAAGCAATGGATAAAGGTAAATAATTATGTGGAAAATT
AGTAAAGAAAATTGTGAAGATTTAGGATTTGCAATAGTCTGTATGTTCTATGATGCTATT
AATCTTTCTGAATTTAAATTATGGTTGGATATAGTTGTCAGAGATATTCCTATTGATACA
ATTCCATTGTATATTTTTGATTTGATTGATTTTGATAAGAGTATAGGGGAAATTTATGAT
GTAATTGGAGTCGTTAATTATGGTTACATTTCAAATGATCAAAAAAATGCATTAACGGGC
ATTGCCTTCTTAAGGGGGATAGATGTCTATGATCCGCCTATTTCAAAAGAAAAAGCATTA
AAAGCCTTAGAGAAATATCCTGAAATTTATCAGAGGTTTCAGCATTTCTTTCCTTTTGTA
GAGCTTCCGCTTTTTTAAAAGACAATATGCCGTCTGAAAAGTTTTCAGACGGCATTTTTT
ATTTCTTCCAGTAGGCGGGGGTGAAGAGGATGAAGACGGTGAAGATTTCCAGCCTGCCCA
AGAGCATGGCGGTAACGCAGATCCATTTCTGCATCACGTCCAAACCGGCGTAATTGCCGG
CGGGCCCGACTTCGCCCAGGCCGGGGCCGGCGTTGGTGATGCAGGCGATGACGGCGGTGA
AGGCGGTGGTAAATTCCATACCGCTCGCCATCAGCAGGAAGCTGAAGAGGACGACGGTCA
TAAAGTAGATGAAGATGAAGGACATAACGGTCAGCGCGAGGCGGTCGGGTATGGCCTTGC
CGCTGATTTTGACGGTGCGGACGGCTTTGGGGTGCAGCAGCACCATCATTTCGCGCAGGC
TGAATTTGAACAGGACGAGGGCGCGTATGGTTTTGATGCCGCCGCCGGTCGAGCCGGAGT
TGGCGAGGATGTTGGCGAGGAAAAACATCCACAGGGAAATCAGGAGCGGCCATTGTGCGA
AGTCGGTGTTGGCCAGCCCGTTTGCCAGTCCGATGGAGACGAAGTTGAAGGCGGTGTAGC

## Appendix A

GCAGGGATTCGGTAAAACCGGCGTAATAGCCGGTGTGCCACAGGTACAGGGCGGCGGCAA
GGATGCTGCCGGAGAGCAGCAGCAGCATCGTCCGGCATTCTTCGTCTTTCCAATAGGTTT
TGAGGCTGCGGCTGTTGAGGGCGGCGAAATGGTTGGCAAAATTGATGCCGCCGACAATGG
TGAAAACGATGATGACCGCTTCGATGAGGGGGGAGTTGTAATAAGCTATGCTGGCATCGT
GGGTGGAAAACCCGCCCAGCGAGAGGGTAGCCATCGCGTGACAGACGGCATCGAACCAGC
CCATCCCGGCAAAATGCAGGCAGGCTGCCGCGAGGATGGTGATCAGGGTGTAGCCGAACC
AAAGTTTTTTCGCCACTTGGGAAATGCGCGGCGACATTTTGCTTTCTTTGTCAATGCCGG
GGATTTCGGCTTTGAATAACTGCGTGCCGCCTACGCCGAGCATAGGCAGGATGGCGACGG
CAAGGACGATGATGCCCATCCCGCCCAGCCAGTTGAGCATATGCCGCCAAAAGTTGACGG
AGGGGGCGAGCCCGTCGACGTGGGGATGACGGTCGCGCCGGTGGTGGTCAGTCCCGACA
TCGATTCAAAAAATGCGTCGGTAAAGCCCATATTCGGGAAATACAGGTACATCGGCATCG
CAGCCATAGCGGCAAACGCCAGCCACAACATCAGGACGAGGGTAAAGCCGTCGCGCGGGC
GCAGTTCGCGCCTGAACCGGAGGGTGGCGAGCCGGACGATGCACGAGCCGGAAAGGGTAA
CGGTCGCGGTGGTGGCGAAGGCGGTGTACGCGCCGTCCGAAAAGGCGTAGGAGAGGGCGG
CGGGTATCAGCAGGATAAAGGAAAACAGCATACCCAGTCGGGAGAGGACGTGGGCGATGG
GCAGGATTTTGTGCATAGTGGGGCGGTCCGTTATTTTGCGAAGCTTTTCCAGTCTATGCC
GCCGGCGGCTTGGACTTGGGTAATTTCTTCCGTTTCGAGGTTGACGGCGGTCAGCTGTCC
GCCCCACAGCGCGCCGGTGTCCAGCGAGATGACGTTGTCGGCATTCGTGTAGCCCAGCGA
GGACCAGTGTCCGAAGATGATGTGCGTCGAGGTTTTGCCGGTCGGGGGCTTTGAACCACG
GGCGCAGGTAAGGCGGCATTTTTTTCACTGTGGATTTGTAGTCGAAATCCAGTTCGTTTT
TAAAGGTCAGGGCGCGCATCCGCGTGAAGGCGTTGACGATGAAGCGCAGGCGGGCATAGC
CTTTCAAACCTTCGTCCCACGCGGCCGGTTTGTTGCCGTACATTTTGGAGAAGAATTTGA
CGTATTTTTTGCCGCGCAGTTCGGCTTCGGCTTCTCTAGCGAGCGATTCGGCTTTGGTTA
TGCGCCATTGCGGCAGGATGCCGGCGTGTACCATCACGCGGCTGCCCTCGCGTATCAACA
GCGGTTGCGCACGCAGCCAGTCGAGCATTTTTTTCCGTCGGGGTGTTTGAGTATGGGTT
CGATTGTGTCGCTGCGTTTGGGCGCACCTTCGCCGCAGCCGACAGCGAGCAGGTGCAGGT
CGTGGTTGCCGAGGACGATTTGCACGCTGTTTTCGTGCCGGATGCAGAATTGCAGCGTTT
CGAGGGATTTCGGGCCGCGGTTGACGATGTCGCCCGTCAGCCAGAGGGTGTCCGTGCCGT
GGTTGAAACCGATTTTGCCGAGCAGCGCGGTCAGTTCGTCGAAACAGCCTTGTATGTCGC
CGATTGCGTAATGTGCCATTGCAGATGTTGTGAAGTGGGAAAGTGTTGCGGTTCGGACGG
CATGGTTTTGAAATATCATGCAGTCCGAACGTGGAATTATGCGTTCAAAACGAGGACGGC
TTCGGCTTCGACCTGCACGCCTTTGGGCAGCGAGGCAACGCCGACGGCGGCGCGGGCGGG
GAAGGGCTCGGCGATAAATTCCGCCATGACTTCGTTGAAGACGGCAAAATTGCCCAAGTC
GGTCAGGTAGGCGTTGAGTTTGACGATGTCGGCCAGCGTGCCGCCTGCCGCTTCGGCGAC
GGCTTGCAGGTTTTGGAACACTTGGCGCGCTTCGGCGCGGAAATCGCCGTTGCCGACGAC
GGTCATCGTGGCGGGATCGAGGGGGATTTGACCGCTCATGTAAACGGTGTCGCCTGCTCG
GACGGCTTGGCTGTACGCGCCGATGGCGGCGGGGGCTTTGTCGGTGTGGATGATGGTTTT
GGACATTTCGGATTCCTCAAAAAATAGGGCGGCAGAAGCCGCAGCATTCGGGATTATCGT
ACAAAACCGCCGGCTTGTGTAGTTGCGGTGGCAGAAAACAAAACCGCCGAAGGCTCGGCG
GTTTGCAGAATAAGGCGCATATCAGAATTTGACGCGCACACCGGCGGACAGTTCGCCGGA
ACGGACGTTTTTGACAGTGTTGACTTTGCCGATGTAGTTGTAGCGGTAGCCGGCATCCAA
ATCGACATTCGGGGTAACGGCATAGCTTACGCCCGTCAATACGCCGAGGCCGATGGAGGT
TTGGCTGAAGCTGTCGCTGCCGCCCAAGTCGACGGAGGCGCGGTTGAGGCTCAAGCGCGC
GCCGAGATACGGTTTGACGGGCGATTGGGTGTCGAAGTCGTAAATGGCGGACGCGCCGAT
GCTGTAAAGTTTGAAATCGGTGGATGGGGCTTTATAGTTTTTGTAGCGCGTGTAATCGAC
GGCGAAGCGGAGGTCGTTGATGCGGTAGCCTGCGGAGATGCGCGGGCTGAAGCCTTTGGC
AGAACCTAAAGAGCTTGAGGCTTTTGCGTGTGCGGCATCGGCTTGGACGTAAAAGCCGGA
TGCGCCTTCCGCCAGTGCGGCGGCCGGGAGAGCGAGGGCAATCAGTGTGGCAAGTGCTTT
TTTCATATTTTGGTTCCTTTATGGTCAGTTAGAAAAATTGTTAAGAATCCGTTAAAGAAT
CCTGCTGTATTATACTTAAATTTTCTTTTTGCATCGTAATATTTTCAATACTTCAAGATA
CGTAGCGGTATCCGGCTGCTTTGCCGACGGCAAAGCCGTTAACCCGCGCGTTGCCTTTAA
ATGGTGGCGGCGGCATCACGCGGCGGATGGGTGAAACTTGCAAACGGTTTGGAAAAAACA
GCGGTATCTGTCGGATTGTTGCAGGTGCAGGCATACGGTTTTGTGTGCGTCTGTGCCTTA
AGCGTCGGACATTTCCGGCGGCGGCTGTGCCGTCTGAAACGCCCGGCGGGGGATGCGGCT
GCGTTTTCCATCGATAAGCATATTTTCCGGACGCGTTCGGGGCGGGTTTTCCCGGGCGGC
CGCCGATTTGTTTGCGCTTATATAGTGGATTAACAAAAATCAGGACAAGGCGACGAAGCC
GCAGACAGTACAAATAGTACGGAACCGATTCACTTGGTGCTTCAGCACCTTAGAGAATCG
TTCTCTTTGAGCTAAGGCGAGGCAACGCCGTACTGGTTTTTGTTAATCCACTATAAAAAA
CACTGCAGCAAATCGTTTAAAAACAAGCGTCCTTTTTCGGTCGGGCGGAATACGGCAGGG
TCGGTTTCCAGCAGGCCTTTTTGCCTTGCCGTTTCGATTTGCGCCATGATTTTGGCACTC
GGTACGCCCGTGCGCTCCTGCAACATCGCGGCGGGTACGCCGTCGGTCAGGCGCAGGGCG
TTCATCATGAATTCGAACGGCAAATCTTCGGCAGCGACGGTTTTGCGTTCGACGGCTTCA
CTCGGTTGGCTTTGCATTAAGGCGAGGTAGTCGTTGGGGTGGCGGCGGCGGACGGTGCGC
TCGATGCGGTCGGGATAGGAAATTTTGCCGTGCGCGCCCGCGCCTATGCCTAAATAATCG
CCGAACTGCCAGTAGTTCAAATTGTGGCGGCACTGCATGGCTGGTTTCGCAAAAGCCGAT
GTTTCGTAGTGGACAAAACCCGCGCCTTCCAGCGCGCCGTGTACCGCGTCTTCGATGTCG
AGGGCGGCTTCGTCTTGCGGCAAACCTTTCGGCGGCGTATGACCGAACGGCGTGTTCGGT
TCCATCGTCAGGTGATACGCGCTGATGTGGGTTGCGCCCGTAGCGATAGCGGTTTGTACG
TCGTCCAATGCCGTCTGAACGGTTTGGTTCGGCAGGGCATACATCAAGTCGATATTGACT
TTATCAAATAATTTCAAGGCGGTATCGATAGCGGTTAAGGCTTCCTTACCGTTGTGGACG
CGCCCCAGCCTTGAGAGCATATCGTCGTTGAAACTCTGTACGCCGATAGAAAGCCGCGTA
ATACCCGCGTCTTTAAATCCTTGAAACTTCTCGATTTCAAATGTCCCCGGATTGGCTTCC
AAGGTAATTTCCGCTTCCGGCTGCAAGCGCAACAGCGAACGCACGCCGCTTAACAAACGG
TCAATCGATTCCGCCTGAAACAGGCTGGGCGTACCGCCGCCGAAAAAGATCGTTTCCACC
GGCCTGCCCCAAATATTGGGCAATTCAAGCTGCAAATCGGTCAGCAGCGCGTCGATATAG

## Appendix A

GCGGCTTCGGGCAATCCGTTTTTCAGGCTGTGGAATTGAAGTCGCAATACGGGCATTTT
TTGATGCACCACGGGATGTGGATGTAAAGCGACAGGGGCGGCAGGGCGGTGAGTCGGGTG
CGGTTTGGAAAGGAAATGGTGTGCATGGTGTGGTTCGGAAAAGTGGGCAATGCCGTCTGA
AGGCGGTTCAGACGGCATGGGTTCAGCCGAGCAGGGTAAGCAGTTCGGCTTCGCTGAGGA
CGGAAACGCCCAAGGCATTGGCTTTTTCCAGCTTGCTGCCCGCGGCTTCTCCGGCGACGA
CGTAATCGGTTTTTTTGGACACGCTGCCGGAAACTTTGCCGCCTGCGGCTTCGATTAGGG
ATTGGGCTTGGTCGCGTTTGAGGGTGGGCAGGGTGCCGGTTAACACGAAGGTTTTGCCCG
CCACGGCTTTATTGATGCCGTCTGAACCTTGCGCCGCCTCGTCTTCAGACGGCATTTGCG
CGAAGAAGGTTTTCAGGTTTTCGAGCAGGGCGGCGTTTTGTGGTTCGCTGCGCCACGCCT
GCCAGTCGGTGGGGAGGGCTTTGTCGGTTTGCAGCCCTTCTATGTTTTTGCCGGCGAGTT
CCCATAAGGCTTGGGCTTTGTTTTCGCTGATTTTGAAACCGGGCAGGCGGGCGATCCAGC
GTTGCGGTTCGGCGTGGCGGGCGGGCGGGATGGTAACGGCTTGGGTTTGCGGGGCAACGC
CTGCGGCGAGCAGTTCGTCTATCATCGCCTGCTGTTCGGCTTGGGCGAAGAAGTGGGCAA
TGGAACGCGCCACTACCGTACCGATGTCGGGCAGGCAGGCGAGGACGGGTTCGGGGGCGC
GGCGGACGCGTTCCAATGTGCCGAATGCCTGTGCCAGCGTTTTGGCGGTGCGTTCGCCGA
CGTGGCGGATGCCGAGCGCGAACAGGAAGCGGGCGAGTTCGGGCGTTTTGCTGGCTTCTA
TGCCTGCGAGGATGTTTTCCGCCCACTTGACTGGTTGTTTTTTATGTTTGCCCGACGCGC
CGACCGAACTGCCTTCAGACGGCATTTGATCCGATTCGGCAACGGTTTTGTCCGCTGTTT
CCTTCATTTTTTGCAAGGTCGGGATGTCGAGGCGGTAGAGATCGGCGAAGTGGCGGACGA
GGTCTTGCGCGACAAGCTGTTCGATTTGTTTTTCACCCAAGCCGTCGATGTCCATCGCTT
TGCGCGAGGCGAAGTGGATTAAGCCTTGCGCGCGTTGTGCCTGACAAAGCATACCGCCGC
TGCATCGGGCGACGGCTTCGCCTTCTTCGCGTTCGATTTCGCTGCGGCAGATGGGGCAGT
GGGTCGGCAGGCGGTAGGGCTTGTGGAGCGGAACGGATTGGGTTTGATTGGCGGACGGTG
TTTCGGCAAACAAATCGTCCTGCCGATGCCCGATGCCGTCTGAAACGGCAACGGCGGTTT
CCCGCATCGGGCGGCGTTCAAAAATCACGCGCACAACTTCGGGAATCACGTCTCCGGCAC
GGCGTACGACGACGGTATCGCCGACGCGAACGTCTTTGCGCGATACTTCGTCCTGATTGT
GCAGGGTGGCGTTGGTAACAGTTACGCCACCGACGAATACGGGCTGTAATCGGGCAACCG
GCGTTACCGCACCCGTCCTGCCGATTTGCACGTCAATCGCTTCGACAATGGTCAGGGCTT
CTTCGGCAGGGAATTTGTGGGCAACCGCCCAACGCGGCGCGCGGGAGATGAAGCCGAGTT
CGTGCTGTTGCGCCAAGCTGTTGACTTTGACGACCATGCCGTCGATTTCGTAGGGCAGTT
CGGGGCGTTTTTGCTGCATGTGTTCGTAAAACGCCAATACTTCGTCGATATTTTTGAAAC
AGCCGAAATTGCCATTGGGCAGACTGAAGCCGAGTGCTTGGAAATAGGCGAGTTCCTGGA
TGTGTTCTTCCGCGACGAAACCATCTTGCTGGCGGGCGACGGAGTAGGGGAAAAAGTGCA
GTTTGCGTTGCGCGGTGATGCGCGAATCGAGTTGGCGTAGGCTGCCGGCGGCGGCGTTGC
GCGGATTGGCAAAGGGTTTTTGCCCGTTTTCGGCTTGTCTTTTATTGAGGGCGACAAAAT
CGGCTTTGAGCATCAGCACTTCGCCGCGTACCTCGATGAGTTCGGGCGTATTTTCGCCGT
GCAGCCGCAAGGGGATGTTGGATACGGTTTTGATGTTTTGGGTAACGTCTTCGCCCGTCG
TGCCGTCGCCGCGCGTTGCCGCCTGCACCAATACGCCGTCGCGGTAGAGCAGGCTGATGG
CGAGGCCGTCGAATTTGGGTTCGATAACGTATTCGGGATTGCCGCCGTCCAAGCCGTCGC
GCACGCGTTGGTCGAAGGCGTACATTTCGGCATGGTCGAACACGCCGTTTTCATCTTGCG
GGGAAAAAGCGTTGGTCAGCGACAGCATCGGCACTTCGTGGCGTACTTCGGCAAATCCCG
CCAAAGGCTCGCCGCCGACGCGCTGGGTCGGGCTGTCGGGCAGTTTGAGCTCGGGATGGT
TTAACTCCAACGCTTCGAGTTCGCGGAACAATTTGTCGTATTCGGCATCGGGTACGCTGG
GCGCGTCGAGGGTGTAGTATTCGTAGGCGTAGCGGTTGAGGAGGTCGGTGAGGCGGCAGA
TGTGTTGTGCGGCAAATTGTTTTATATCACTATCAGACGGTTTAAGAAGATTGGTAAAGT
TAGTGTTATGTTTTGAGTTTGGATTCATGAGAGAAGGTTTTCAGACGACCTTTGTCTGAT
ACGGGATGAAACGGGCAAAGGTCGTCTGAAAAATGATAGGTTGAAAACAGCTGAATTTTA
CCCGAAAAAAAGCGGATATGCCGTAACGACATATCCGCTTTGATTGCATTCGATTTTAGG
AGAACAGGCGCAATGCGGTTTTGCCGCCCGGTTCGATACCGACTTTGAGCATCTCGGACT
GACGCGCCAATACATAAGTGCGCACGTCTTTGAGCCATTGGGTCGAAACTTCTTCCATTT
TGTCGTTGACCAGATTCAGGTTCAACTGGCCGGACAGGCGTACCGCCAAATCCATAAACA
AATCGTCGAAGGTTTTTTCGCCTGCCGGAGAGTGCGGATGTCGAGCAGCATACTGAAGC
CTTTGTAGGACTGGTTGTCCAAAAGGGCGTTGGTAAACGGCTCGTTGTTGAGCGAGCAGA
TGGAGAACATGGTCGAGCCCGACGTGTCGGTATAGTGGAACGCGCCGTCGTCTTCCAAAA
CGAAACCCACGCCCGTTACGGCGGAACGCAGTTCTACGCCGCTGATGCTGGTCGGGGAAA
CCAAATGGATGGCGATGGTCTGGTCGACGCGCGCGCAGAATGCGTCCAGTGCGGAAGCCA
CTTCGATAAAGGCGGCAAGGTCGGTGTGCAGCGTCTGACCGCCCATGCTTTGTGCGAATG
CGTCCACCTGGCGGTTGAATGCGGAGAGTTCTTCCTGCGAGGCAAGTCCGTTGCGGCTGA
CTGCCTGAATACCCACGATAAATGCCTGATAGCGGATGCCCGGGATGGGTTCGGCAATCT
GGAAATGGTCGTCCATGGTGCAGCCGACAATCTGGTAGCGGCAGCGGTTGGAAAGGCGCG
GCAGTGCGTGCAGTTCTTTGGCTTCGGTCAGCGCGATATAGGAGATGAAGTCGAAGCGCA
CGTCAAACCAGGGTAATTCGACTTTTGACAGTTCTTTGAGCGTAATCAGCGGTTTTGCAG
GTGTTTGCGGAACGGGTGCAGGTTTTGCCGGCGCGTCGGCAGGTTTCGGTGCGGAATGTC
CGGTTTGGGGTTCGGAAACGGTGTGGGCGGAGTTGCCGATAATGCCGCTTTCTTCCAAGG
CGGTTTCGATTTCGGTTTTGAACGGGGAGGCTTTTGCCTGTTTCTGCTTGGCGATGTAGA
CGGCATCCTGTTCTTGCAGGTTGCGCATGGCGGGGTCTTGGGGTTTTGCCGTTTTTTTGA
CCGCCGGTTGGGGTTTCGGCATCATGACTGACCCGCCGGACGGTTTGCCGTCGCGGACAT
GGCTGGTTTTGCTGTTGAGCAGGGCATCTTTGTCGGAGTGTCCGAACTGGTCGCGCACTT
TTTTGCGGTATTGGTTTTCCTGATACATGTTGTAGGCGACAACGGCGAGGACGACAGCTA
GAAACAGTACGATGTAAATCATGGCAATCACTTGTTAAATTTCGGGATGCAGGATACGCA
AAGTGCGGGTACTGCGGTTAAATCGGGCTTGCACTGCGGTTAAATCGGGCTTGCGTTTCC
GGCAGTCTGACGGAACGGCCGATTATAACGTTTGAATTATAACGAAAATTGCAGGGTCTG
ACAGCAGTGTGTCGAAATAAGCGGAAATTTTCCGAAATGCCGTCTGAAATCTGTGGTTTT
CAGACGGCATTTCTGTCCACGAGAAACCCTTTCTCCCGTATCCGCCGCCAGTCGAAAAAA
TGGCCGGGGTCGGTTTTGCGGCCGGGCGCGATGTCTTGGTGCCCCGTTACCGCCGTGACG

# Appendix A

GGGTAGTGGCGGCAGATTGCGTCCAACAAGGCTTCGAGCGAACGGTATTGCGCTTCGGCA
AACGGTTCGAAATCGCAGCCTTCCAGTTCGATGCCGATTGAAAATGCGTTGCATTTTTCC
CTGCCGCCGAATGAAGATACGCCGGCATGGTATGCCATATTGTCGCAGGAAACGAACTGT
ACCGTTTCTCCGTCGCGTTTGATTAAGAAATGGCTGGATACGCGCAAAGTGTGTATCAGG
CTGAAGAACGGATGTCCGTCGGGGTCGAGCCGGTTGGCAAACAGCTTTTCCACCGCATCC
GTGCCGTATTCGAACGGCGGCAGCGAAATGTTGTGCAACACGATCAGGGAAACCGTTTCC
CCCGTTTCCCTCGGGCTGAAATTGGGCGACGGGGTATGGCGTATGCTTTGAAGCCAGCCG
TTTTGCCAGTGTGCTTCGGCGTGATTGTCCATGATGTTCTTCCTGTCCGGCGGGCAATTT
GGGTTATACTGTCGCCCGAATTTTAAGACGTATTCCGAATGCTGGGAATCCTACCATGTT
GAGAAAATTGTTGAAATGGTCTGCCGTTTTTTTGACCGTGTCGGCAGCCGTTTTCGCCGC
GCTGCTTTTTGTTCCTAAGGATAACGGCAGGGCATACCGAATCAAAATTGCCAAAAACCA
GGGTATTTCGTCGGTCGGCAGGAAACTTGCCGAAGACCGCATCGTGTTCAGCAGGCATGT
TTTGACGGCGGCGGCCTACGTTTTGGGTGTGCACAACAGGCTGCATACGGGGACGTACAG
ATTGCCTTCGGAAGTGTCTGCTTGGGATATCTTGCAGAAAATGCGCGGCGGCAGGCCGGA
TTCCGTTACCGTGCAGATTATCGAAGGTTCGCGTTTTTCGCATATGAGGAAAGTCATCGA
CGCAACGCCCGACATCGGACACGACACCAAAGGCTGGAGCAATGAAAAACTGATGGCGGA
AGTTGCGCCCGATGCCTTCAGCGGCAATCCTGAAGGGCAGTTTTTCCCCGACAGCTACGA
AATCGATGCGGGCGGCAGTGATTTGCAGATTTACCAAACCGCCTACAAGGCGATGCAACG
CCGCCTGAATGAGGCATGGGAAAGCAGGCAGGACGGGCTGCCTTATAAAAACCCTTATGA
AATGCTGATTATGGCGAGCCTGGTCGAAAAGGAAACAGGGCATGAAGCCGACCGCGACCA
TGTCGCTTCCGTCTTCGTCAACCGCCTGAAAATCGGTATGCGCCTGCAAACCGACCCGTC
CGTGATTTACGGCATGGGTGCGGCATACAAGGGCAAAATCCGTAAAGCCGACCTGCGCCG
CGACACGCCGTACAACACCTACACGCGCGGCGGTCTGCCGCCAACCCCGATTGCGCTGCC
CGGCAAGGCGGCACTCGATGCCGCCGCCCATCCGTCCGGCGAAAAATACCTGTATTTCGT
GTCCAAAATGGACGGCACGGGCTTGAGCCAGTTCAGCCATGATTTGACCGAACACAATGC
CGCCGTCCGCAAATATATTTTGAAAAAATAAACCATGCCGTCTGAAAAGTTTGTGTTTTC
GGACGGCATACCCTTACCGGAACTGCAAGCATGAAACCGCAATTCATCACTTTGGACGGC
ATAGACGGTGCCGGCAAATCCACCAACCTTGCCGTCATCAAGGCATGGTTTGAACGGAGG
GGGCTGCCCGTGCTGTTCACGCGCGAGCCGGGCGGAACGCCGGTCGGTGAGGCCTTGCGC
GAAATCCTGCTCAACCCTGAAACCAAAGCCGGTTTGCGTGCGGAAACCCTGATGATGTTC
GCCGCGCGTATGCAGCACATCGAGGAAGTCATCCTGCCCGCGCTTTCAGACGGCATACAC
GTCGTGTCTGACCGTTTTACCGACGCGACCTTCGCCTATCAGGGCGGCGGGCGGGGGATG
CCGTCTGAAGACATTGAAATTTTGGAACATTGGGTGCAGGGCGGTTTGAAGCCGGATTTG
ACCCTGCTGCTGGATGTGCCGCTCGAAGTGTCGATGGCGCGTATCGGGCAGACGCGCGAG
AAAGACCGTTTCGAGCAGGAGCAGGCGGATTTCTTTATGCGTGTGCGCGGCGTTTATCTC
GACCGAGCCGCCGCCTGTCCCGAACGGTACGCCGTTATCGACAGTAACCGCAACTTGGAT
GAAGTCAGAAACAGCATAGAAAAAGTGTTGGACGGACATTTCGGCTGCTGATGCGGCAAA
TATTGAAACAAGCGCATCCGCCCGCGCCGAAAATCAAACGGCAGTGCCGCAGGTGAAAAT
GGCGGTATGCGCCAAACTTTCGGCATGATAGAATTACGCTCGGTTACAAGGCAGGATGCG
TCGGCAATATTAACGAACCGCCCGTAACATGATGACCCGAAAGCGTTTCGGACAGTCCGA
TTCAAATCTTTTTCTCGCAACAGGATTGACACATGGAAAACTCATTGAAAGAAGCCGCCC
TCAAGTTCCACGAATTCCCCGTGCCGGGCAAAATTTCCGTTACCCCGACCAAATCTCTGG
CGACCGACAAAGATTTGGCGTTGGCGTACTCTCCGGGCGTAGCCGCTCCTTGTATGGAAA
TCCATGCCGATCCGCAAAATGCCTACAAATACACCGCCAAAGGCAACTTGGTCGCTGTCA
TTTCCAACGGTACGGCCGTTTTGGGCTTGGGCGACATCGGCGCGCTGGCGGGCAAACCCG
TGATGGAAGGCAAAGGCGTATTGTTCAAAAAATTCGCCGGTGTGGACGTGTTCGACATCG
AAATCGATGAAAAAGACCCGCAAAAACTCGTGGACATCATCGCCGCTTTAGAGCCGACCT
TCGGCGGCATCAACCTCGAAGACATCAAAGCACCCGAGTGTTTCTACATCGAACGCGAAT
TACGCAAACGCTGCAAAATCCCCGTATTCCACGACGACCAGCACGGCACGGCCATCATTA
CCGCCGCCGCCGTATTGAACGCCCTGCGTTTTACCGGCCGTAAAATCGAAGAAGCGACTT
TGGTGTGTTCCGGCGCAGGTGCGGCCGCGATTGCCTGCTTGAACCAATTGCTGGATTTGG
GCTTGAAACGCGAAAACGTGACCGTTTGCGACTCCAAAGGCGTGATTTACCAAACCCGCG
AAGACAAAGACCGTATGGACGAGTCCAAACAGTTCTACGCCATTGAAGACAACGGCCAGC
GCGTGCTTGCCGATGCCGTCAAAGGCAAAGACATCTTCTTGGGCCTCTCCGGCGCGAACC
TGCTGACGCCTGAAATACTGAACACCATGAACGAAAAACCCATCGTGTTCGCTATGGCCA
ACCCGAATCCGGAAATCCTGCCGCCGCTGGCGAAAGAAACCCGTCCGGACGTGGTTATCG
GTACCGGCCGCTCCGACTTCCCGAACCAAGTGAACAATGTATTGTGCTTCCCGTTCATCT
TCCGCGGTGCGTTGGATGTCGGCGCGACGACCATCAACGAAGAAATGAAACGCGCCTGCG
TGTATGCTTTGGCGGATTTGGCGATGGAAGAAGTAACCGAAGAAGTGGTTGCCGCTTACG
GTAAGAAATTTGAATTCGGCGCGGAATACCTGATTCCGACTCCGTTCGATTCCCGCCTGC
TGCCGCGCGTTGCTACGGCTGCCGCCAAAGCAGCGATGGAAAGCGGTGTGGCAACCCGTC
CGATTGCAGATTTGGAAGCTTACGCTGCCAAGCTGAGCGAATGGAAGCTGTAAGCCGTTT
GCGGTTTAAAATGCCGTCTGAACTGTTTTCAGGCGGCATTTTGCTGTCAGATTGATATAG
TGGATTAACAAAAATCAGGACAAAGCGACGAAGCCGCAGACAGTACAAATAGTACGGAAC
CGATTCACTTGGTGTTTCAGCACCTTAGAGAATCGTTCTCTTTGAGCTAAGGCGAGGCAA
CGCCGTACTGGTTTTTGTTAATCCACTATAAATGAAAGATACTGAAAAATGAAAGAGATG
AAACCTGTCCGTTATCATATTGGCGATATGCCCGAGACTTCAAAACAAACCGCCTCCCGT
CATGACGACAGGGCAGTGGGTGTTGACGATGATTGTTTTCATGATTCCTTTGGTCAATTT
TTGTTTGGGTGTTCGGCAGAGGCAACCCGAACCGCGCCAATTTCTGTAAAGCGCAGTTGC
TTATTTACCTGATTGGTTCGCTTATCGGTTTGGTCTTCGCGTTGTTTATAGGTGGGTCTG
TATCAGGTACGCATGATTAATGCCCCGGGCTGATTTTGCTTCGAGGATTTGTATCGAATA
TGCCGAATTGTTTCAAATTTCATACCGTTATCGAACGGCATTGGCAAAAACCTTATCCGG
TTTTGTCTTTTCTGCTTAAGCCGCTCTCCGGGCTGTTTGCCAAAATTGCGGCAAAACGGC
GGACGGATTTTTTATCGGGAAAACGGCAAAGCGAAAAGCTGCCCGTGCCTGTGGTCGTGG
TCGGCAATATTCACGCGGGTGGACGGGGAAAACGCCGATTGTTGCCGCGCTGGTGTCGG

## Appendix A

GTTTGCAGGAAAAGGGCGTCAAGGTCGGCATCATCAGCCGGGGCTACGGGCGCAAGAGCA
AGGCGGTTCATGTATTGAATGCTGAGAGCCGAGCGGAAGATGCGGGCGATGAGCCTTTGC
TGCTGTTCCGCAAAACCGGTGCGCCGACGGCGGTGGGCAGCAGCCGTGCAGAGGCAGGCA
GGGCGTTGCTGGCGGCGCATCCCGACATCGGACTGATTGTGGCGGACGACGGTTTGCAGC
ATTACGCCCTGCGGCGAGATGTGGAAATCGCGGTGTTTCCGGCGGCGGATACGGGGCGCA
CGGATTTGGATTTACTGCCCAACGGCAGTTTGCGCGAACCTTTGTTGCGGCTGGATTCGG
TGGATGCGGTCGTCGTCAGCGGCGGCAAGGCGGATGCGCTGTTTAGGCCGTCTGAAAATA
TGTTTCACAGCCGTATCGAAGCGGGACGGATTTACCGTTTGAACAATCCGTCCGAAATAC
TGGACACAGGCCGTCTGAAAAATCAAACCGTCGTCGCCGTGGCAGGTATTGCCAAGCCGG
CGCGGTTTTTTGATTCGTTGCGGAATATGGGCATTACCGTGAAGCGAACCGTCGCGCTGC
CCGACCACGCCGACATTTCGGCGGCAGATTTGCCCGATGCGGACGCGGTCATTATTACGG
AGAAAGATGCGGTCAAATTTTCAGACGGCATTTGCACCGATAATGTTTGGGTGTTGCCCG
TTTGTGCGATAATCGAACCTGATTTGGCGGCGTTTGTGTTGGAGCGGTTGGAAGATGTAC
CGAAGGCCGTCTGAAAGCACGGTTTGGGCGGAGTGATTACGGATTTGAATAAGAACGCCT
CGCGCCATCATTCCCGCGCAGGCGGGAATCTAAGTCTCGAATTTTCAGGAATGCCTAGGA
GGCTCCAGAAATCCCAAATCTCCGGATTTCCACTTGGACAGGAATGAGAAAACCGGTCGT
ATTTTTTATCTGCATTAATCATTCATTAAAGGATTGAATATTAAACTGAAAACCTTGTTA
TTGCCCTTCGCCACGCTGGCATTGTGCACCAATGCTTTTGCCGCCCCGCCCAGCGACGCG
TCGTTGGCGCGTTGGCTGGATACGCAGAATTTTGACCGGGATATAGAAAAAAATATGATT
GAGGGCTTTAATGCCGGATTTAAACCGTATGCGGACAAAGCCCTTGCCGAAATGCCGGAA
GCGAAAAAAGATCAGGCGGCAGAAGCCTTTAACCGTTATCGTGAGAATGTTTTGAAAGAT
TTGATTACGCCCGAAGTGAAACAGGCTGTCCGCAATACTTTATTGAAGAATGCCCGTGAG
ATATACACGCAAGAAGAAATTGACGGCATGATTGCCTTTTACGGTTCGCCTGTCGGTCAG
TCCGTCGTTGCCAAAAATCCGCGCTTAATCAAGAAATCGATGAGTGAAATAGCGGTATCT
TGGACTGCATTGTCAGGGAAAATCGCGCAACATCATCTGCCCGAGTTTACGGAAGAGTTG
CGGCGCATCATCTGCGGCGGTAAAAATCCCGATGCGGGCTGTAAACAAGCCGGACAGGTT
GGGAAAAGGCATCAGAAATAAATGATAGCCGTCTGAAATATTGAAGAGGGCATCCGATTG
ATTGAACCATCAAACCCGAAAGCAACCCTATGGAAAAAAAATTCTTAGACATCCTCGTCT
GCCCCGTTACCAAAGGCAGGCTGGAATATCATCAGGACAAACAGGAATTGTGGAGCCGTC
AGGCGAAGCTTGCCTATCCGATTAAAGACGGCATTCCCTATATGCTGGAAAACGAAGCGC
GAGCGTTGAGCGAAGAGGAACTCAAAGCATGACCGAATTCGTCGTATTGATTCCGGCGCG
GCTGGATTCGTCGCGCCTGCCCGGAAAAGCCTTGGCGGACATCCACGGCAAACCGATGGT
CGTGCGCGTTGCCGAACAGGCGGCAAAAAGTAAAGCCGCGCGCGTCGTCGTTGCCACCGA
CCATCCCGATATTCAGACGGCCTGTCAGGCGCACGGTATCGAAGTCGTCATGACTTCAAA
CCGGCACGAAAGCGGCACGACGCGCCTTGCCGAAGCCTCTGTCGCGCTGAAGCTGCCGCC
GCATTTGATTGTTGTGAACGTACAGGGTGACGAGCCGCTGATTGCCCCCGAACTCATCGA
CCGCACCGCCGAAGTACTCGTCGAAAACAACGTCCAAATGGCGACCGCCGCCCACGAATT
GCACGATTTCGACGAATTGATGAATCCCAACGCCGTCAAAGTCGTCCTCGACAAAAACCG
CAACGCCATCTACTTCAGCCGCGCCCCGATTCCCTATCCGCGTGATGCGATACGTGCCGG
AAAACGCGAAATGCCGTCTGAAACCGCCGTCCTGCGACATATCGGCATCTACGCTTACCG
CGCCGGCTTCCTGCAACGCTATGCCGAAATGAGCGTTTCGCCGCTGGAAACCATCGAATC
GCTGGAACAGCTGCGCGTCCTGTGGCACGGTTATCCCATTGCCGTCGAAACCGCCAAAGA
AGCCCCCGCCGCCGGTGTGGATACGCAAGAGGACTTGGACAGGGTTCGCGCCGTATTTCA
GACCGTATAAAACAGGTTCAAAGGGAAAAGATATGCAGCAACATATTGAAAAGTGGCAAC
ACTTGAGCCGGGAAGAACAGAAAATCCTTGCTGAAGTATGGGGTCTCGTGCAAAACGACG
ATCAGGAGGTTCACTATGAAATGCTCAAATTGAACGCACCCGATGAAGCCAGCGGCGAAT
TTTGGTTCAGAATGGCAGAAACACTCAGCACCCTGCCGCCCAACCGTTCCCTCGGCCTTA
GAATGAACGGCGGCAGGCTGGCGACCGCCGTATCCATCCTTTCCGTCATGATTGAAGACA
ATCCCGACATACCGCAGCTTTGGGCGCAAAAAATTACCGCGCTCAATTATAGTGGATTAA
ATTTAAACCAGTACGGCGTTGCCTCGCCTTGTCGTACTATCTGTACTGTCTGCGGCTTCG
TCGCCTTGTCCTGATTTTTGTTAATCCACTATATTTGGCACACGGGCACAAAGCCCGTGC
CGACGGTTTGGCACAACAGCCCGACAAAGCGGCAGAAGCCAACGAGGAGGAATACCTGAC
CAAAGCCCTGTCGCAAAACCTGCTGTCAACATTGGATGTCGCGCTTGCACGTTTTCCTGA
AGACGCGTGGTTTCAGGAAATCAAACAGGATGCACAAAAGCATTTTGCTTGAGGATGTGG
CAGTCAGGAATATTTCCATTCAGGAAGAAAAGAAGTGCCTGATTGGGTATAATCAGGGTA
AATCTTATTTTATTTCAAAAGATTAATATTTGCTTTCTGTTTTTCCTTGACGGTATCGGA
AAAGTTGATTATAGTTACAGCTTCCTTAGGAGTAATGGCTGAGAGGCTGAAGGCACTTCC
CTGCTAAGGAAGCATGTGGGGTCAACCTGCATCGAGGGTTCGAATCCCTCTTACTCCGCC
AGATAAAAAATAGACGCTGTGTTTTACAGCGTCTATTTTTTATGCAATTTTATAGCGGGT
TGGTGCAAAACCAGTATGGTATTGCCCTGTCTTGATTCTGAATTTTGTTATAGTGGATGA
ACAAAAACCAGTACGGCGTTGCCTCGCCTTAGCTCAAAGAGAACGATTCTCTAAGGTGCT
GGAGCACCAAGTGAATCGGCTCCGTACTATTTGTACTGTCTGCGGCTTCGTCGCCTTGTC
CTGATTTTTGTTAATCCACTATAATCCGAGATGCTTGCCGTTTATTTCCGCCTCGTTCAA
ACGGCGGCTCTGATTTGCGCGGTTTCTGTTTGCCGTATTCGCCTATCCGTACCGCAAATG
TTATACTGGGAAAAATTTACTGATTGTGTTTTACGGCATATTTGCCGATAGGATGGAAGA
GACAAATGAGCAGAATCCGGCAGGCTTTTGCCGCTTTGGATGGCGGAAAGGCATTGATTG
CCTATATTACGGTGGGCGACCCCGATATTCGGACAACTTTGGCATTGATGCACGGCATGG
TTGCAAACGGTGCGGATATTTTGGAGTTGGGTGTGCCGTTTTCCGATCCGATGGCGGATG
GGCCGGTTATTCAGCGTGCGGCGGAGCGGGCGTTGGCAAACGGGATTTCGCTGCGCGATG
TCTTGGATGTCGTCAGAAAATTCCGTGAAACCGACACGCAAACGCCGGTTGTTTTGATGG
GATATTTGAACCCTGTACATAAGATGGGTTATCGGGAGTTTGCTCAGGAAGCCGCAAAGG
CGGGTGTGGACGGCGTGTTGACGGTGGATTCCCCTGTCGAAACCATCGATCCGCTCTATC
GCGAGCTGAAGGATAACGGGGTCGACTGTATTTTCCTGATTGCGCCGACGACGACGGAAG
ACCGTATTAAAACCATTGCCGAGCTGGCAGGCGGATTTGTCTATTATGTTTCGCTCAAGG
GCGTAACGGGCGCGGCAAGTTTGGATACGGATGAGGTTTCGCGTAAAATAGAGTATTTGC

## Appendix A

ATCAGTATATCGATATTCCCATCGGTGTCGGTTTCGGCATCAGCAATGCGGAAAGTGCAC
GCAAAATCGGCCGGGTTGCCGACGCAGTTATTGTCGGCAGCCGGATTGTGAAAGAAATCG
AAAACAATACAGGCAACGAGGCTGCCGCCGTCGGTGCTTTGGTAAAAGAGTTGAAGGATG
CCGTGCGCTGACGGCGGTTCCTCATCCTGAATATTTTAGGAGTTGTCCATGAGCTGGTTA
GATAAAATCCTGCCACCCAAAATCAAGAATCGCGGAAAAGACGGTTCTTCCAATGTTCCC
GAGGGTCTATGGCACAAATGCCCGTCTTGTTCGGCAACCGTTTATTCTACCGAGTTGCAG
CAGAACAATCAGGTTTGTCCGAAATGCAACCACCACAATCCGTTGTCGGCACGACAACGC
CTGAACCTGCTTTTGGATGAGGATGGCAGGGAGGAAGTTGCCGGAAATGTCAAACCGACA
GATCCTTTGAAGTTTAAAGACGGCAAAAAATATCCGGATCGTTTGAGTGCGGCACGCAAG
CTGACCGGGGAAGATGATGCTTTGGTGGTGATGAAAGGCAAGATGAACGGCCTGCCCGTC
GTCGTTGCTGCGTTTGAGTTCCGCTTTATCGGCGGTTCGATGGGTTCGGTTGTGGGCGAA
CGATTCGTACAAGGTATCCGTCGGGCGGTTGCCGACAATTGTCCGTTCGTCTGTGTGGCG
GCTTCCGGCGGCGCGCGTATGCAGGAGGGTGTAAACTCGCTGATGCAGATGACGAAAACC
AGTGCCGCGCTGCATTTGCTGACGGAAAAACGCCTGCCATTTATATCGGTGTTGACCGAT
CCGACTATGGGCGGCGTATCCGCCAGCTTCGCATTTTTGGGCGATGTCGTGCTTGCCGAA
CCGAACGCGCTGATCGGTTTTGCCGGTCCGCGCGTGATTGAGCAGACGGTGCGCGAAACG
CTGCCGGAAGGCTTCCAACGCGCCGAGTTCCTGCTGGAAAAAGGCGCAATCGACCAGATT
GTCGACCGCCGCGATATGAAGCGGCGCATCAGTGATTTGATTACGCTGTTGTGCCGTCAG
GACAAAGTTTCCGCCGCCTGATGGCTGATGAATCGAGTACCGTCTGAAACCGATGTTTCA
GACGGTATTTTTGTGTCTGGTTATTTGTTGTGCGGCTTTATCGATGGGGCATAGCGTCCG
GCACGTTCTTTCAGGCGTTGTACCAAACCTTTCGTGTCGGCGGGTACACCGCCCTCGCAG
AATGCCTGATACAGGACGGTGCGCAGTGCGTCGTTGCGGCTTAATGTACCGCCTATCGGT
TTCCATTCGGCGTTTCGGGGCTGTATCCAGCGGCGGTTGACCGTGTCGCCGTAGCCGAAC
ACTTTATAGGAGGAAGGTTTGCCGTTGCCGAACCTGATGGAGAAGCGGGCGCAGAATATG
CCTTTGGCAGTCAGGTTGTCGTAGCCTTTGTCGGAGCGGATATTGAGAATGTAGCGGATG
CTGCCGTCGGGCGCGGGCATAATTTGCAGGCTGTCGAGCAGGATTTTCGGCTGTTTGCCG
TAATTTTCATCCACATAAATGTCGAACCAGCCGTCCGAGTGCGTATCGGGCAGCGGCGGC
AGTTTGGCGGTATGTTCTTTAAATTCGCGGGCGGCGGCCTCTTCGGGCGTTTCGCGGTAG
CGGGTGTTGATCGGCGTGTCTTTTTGGCTGAAGCCGGCAGCGAGGGACGTGCCGGCGGCG
AGTGCGAGCAGCAGGAGGGCGGTGCGGCGCATAAGTTTCTCCAAATTGAAAACGGCGTTA
TTTTATGGGTTGGCAAAGGGGGCTGCAAGCAACTGGGGTATAATCTCCCCCCGATTCCCA
TTTTTTAAACGGTACAAACGATGAACAGCGAAACTTTAGACGTAACCGGATTGAAATGTC
CCCTGCCGATTTTGCGGGCGAAAAAGGCTTTGGCGCAAATGCAGCAGGGCGACGTGTTGA
CCGTTCTGGCAACCGACGGCGGCGCACCGGGGGATTTTGAGGCTTTTTGCCGCCAAACCG
GTCATGTGCTGTTGGATGCTTCCGAACAGGACGGCGTGTTCACGCTGGTCGTCCAACACA
AATAAATGCCGTCTGAAATGCGGATGTCCCGCCCGACGGCGTTGTTTTTGAATATCGTAT
GTGCCGCGTGCCGTTTCAAAAACAAACCGTCCGGCGTGCAGTGTCGGCATTTCGGGCGTA
TCGCCGCCGATTTGTTCCGGAATGGCTGTTAACCGCCTTGCCGTCGGCAAAGAAGCAAAA
CCCAAGCACAATCAAAATCTAAAGGCTGTGTTTGAAGATTCCGTTGATGCAACCCAATCA
GTCGGACAAAATGCCTTTCATCCAATGGAACCGGTTTCCGACCGGACGGAATAACCGGCT
TTCCCCCGGCAAACGGATGGAATCGACCGGGTATTCAAACGCAGCCAAAACCTAAAAAGG
AACAACCATGCAAACCCTGACCATTATCCGCCCCGACGATATGCACCTGCACCTGCGCGA
CGGCGACGCGCTCAAAGCCGTTGCCCCTTATACCGCCCGCCAAATGGGGCGCGCCGTCAT
TATGCCCAACCTCAAACCGCCTGTCGTCAGTGTAGCCGACGCGCTTGCCTACAAAGCGCG
CATTATGGCGGCGTTGCCCGAAGGTAGCGCGTTTGAGCCGTTGATGACGCTTTATTTGAC
TGATAACGCCACGCCCGAACTTGTACGCGAAGCCAAAGCCGCCGGCATCGTCGCCTTCAA
ACTCTACCCTGCCGGCGCGACCACCAATTCCGATTCCGGCGTAACCGACCTGTTCAAGCT
CATCCCCGTGTTGGAAGAAATGGCGAAACAGGGCATTTTGTTCCTCGTTCACGGCGAAGT
AACCGAGCGCGAAATCGACATCTTCGACCGCGAAGCCGCCTTTATCGGGCGCGTGATGAA
ACCCGTTTTGGCGCAAGTGCCGAATCTTAAAGTCGTGTTCGAACACATCACCACCGCCGA
AGCCGCCCGCCTGGTTTTGGAAGCAGGCGACAACGTAGCCGCCACCGTTACCCCGCAACA
CCTCCTGCTCAACCGCAACGACCTCTTGGTCGGCGGCGTGCGCCCCCATCATTTCTGCCT
GCCCGTACTCAAACGCGAAACCCACCGTCAGGCATTGGTCGCCGCCGTTACCGGCGAGAA
GGCGCATAAATTCTTCCTCGGCACCGACTCCGCGCCGCACGCCAAATCCGCCAAAGAAAA
CGCCTGCGGCTGCGCCGGTATGTTCAGTGCGATGACCGCTATCGAGCTTTACGCCGAAGT
ATTTGAAAAAGCAGGCGCGTTGGACAAACTCGAAGCCTTCGCCTCAAAAAACGGCGCAAG
GTTCTACGGCATTCCTGAAAATACCGACACGATCACCCTCGTCAAACAAAGCCAAACCGT
TCCCGCAAGTGTTCCCTACGGCGACGGCGAACTTGTCCCGATGCGCGCGGGCGGCGAAAT
CGGCTGGACGGTGCAGTATTGATGGGCTGGAAACAAAATGCCGTCTGAAGGGAGGGCTTT
CAGACGGCATTTGTGTTGCTGACTGATTGATACGTCAACGGCGTTTGAGTTTGTTACGTT
TCGGTTATTTCCGATAAATTCCCACAATTTTCAAATTTCGCCATTCCCACGAAGGCAGGA
ATCCAGAAATTCGATGCGACCAGAGTTTATCAAAAACGGCAGCAACTCAAAAAACCGGAT
TCCCGCCTGCGCGGGAATGACGAGATTGAAGTTTCAGAATTTATTTGAAATACCCAAAAT
TCAAAAAACCAAATTCCCACCTGCGTGGGAATGACGAAACAAAGAAAGCAGAAATAAGGA
CATAGAACTTTCTTTAAATTTGTGATGCATCAACGGCGTTTGGGCTCGTCGGGGCGGATT
TGGGCGGCGAGTTTGTCGAGGATGCCGTTGACGAATTTGTGCCCGTCCGTGCCGCCGAAG
GTTTTGGTAACTTCGATGGCTTCGTTGATAATGACGGGGTAGGGCGTTTCGGGCATGGCG
GACAGCTCGTGGCAGGCGGTCAGCAAAACGGCGCGTTCGATGGGGTTGAGGTCTTTTTCG
TCCCTGTCAAGTAGCGGGCGGATTTGTCGGATATACTCTGCCGCATTGGTTTGCGTGCCG
AAGAAAAGTTTGTTGAACAATTCTTCGTCTGCCTTGGCAAAGTCGGACATTTCGCGGATG
TTTTTAGCAATTTCGGGCGCGGCGGTGCGGTTGATAAGGGATTGGTAAACGGCTTGTACG
GCAAGCTCGCGGGAACGGCGGCGGGCTGTTTTCATGATTTTTCCTTGAAACGGTTGGGCG
GCACGGTATGCCGTCTGAAACGGAAAGGGTGTATTGGTGTACGCCCTGTTTGTTATTCTT
CGTCTTCAAACTGTTCTTCGAGCAGCAGGTTGACGAGGTTGGCGCATTCGACGGCGACTT
TGGCGGCATCCGAGGCTTTTTCTTCAATCCGTTCGATTGCCTGCGCGTCGTTTTCGGTGG

## Appendix A

TTAGGACGGCATTGGCAATCGGGATATTGTAGTCGAGTGCGACGCGGCTGACGCCTGCTC
CGGATTCGTTGGAAACCAGCTCGAAATGGTAGGTTTCGCCACGGATGACGACGCCGATGG
CAATCAGTGCGTCAAACTTTTCGGAAGAGGCAAAGTTCATCAGCGCGATGGGGATTTCAA
GCGCGCCGGGTACGGTGGCGACGGTAATGTTTTCGTCTGCCACGCCCAATTCTTGGAGGG
TGCGGCAGCAGACTTTGAGCATTTCGCTGCCGATTTCGTTGGTGAAGCGTGCCTGTACGA
TGCCGATGCGGAGGTGTTTGCCGTCGAGGTTGGGGGCGATGGTGTTCATTGGGTGTCCTT
TGGTATTCGGAGGTTTCGGAATGCCGTCTGAAGGTTTCAGTCTTGCGGCTGCCAGTCGGC
GACGGTTTGGAATGTGCCGTCTTCGGCAAGCTCCCATGCGCTGCCTTCGGGTTGGGAGAG
CAGTGCGGCGGTTTCAGGGTTGGTTTTGGCGATGTCGGCGAGGCTGACGATGCTGAAGTT
GTCCGGATCGTCGGTGTATTCGTCGGTCTCGTCGCCGCTGAAGAAACGCCAGCCGCTGTC
GTTTTCAAAAACGGGGGCTTCGCGGTAGAGGAAGCCGACGGGCCGGTTTTGTTTGGCGAC
GGTGTTGGTGGCGATACAGCGGTCGAGTGCCGAGGAAAGTGCTTGTGCAAATGCGTTCAT
TACGGGAATACGTTGGGGGAAAACTTACGGATTTTACCACGATTCGTGCGTTGTCGGCAG
ACGGCGGCGGTTTGGTGGTACAATGTGCGCCGTTTGCAGCCTTAAGGTGTTTCTGTATTT
TTGGAGTATGGAAACGCATTCGGGCTGTTTTTTGCGGAAGACGGTAATGAAAGACGATGT
TTTGAAACAGCAGGCACACGCGGCGATACAGAAGAAACTGGGCTACGCGTTCCGCGATAT
TTCGCTTTTGCGGCAGGCTTTGACGCACAGGAGCCATCATGCGAAGCACAACGAGCGGTT
CGAGTTTGTCGGTGATTCGATTTTGAATTATACGGTGGCGCGGATGCTGTTTGACGCGTT
TCCGAAGTTGACCGAGGGCGAGTTGTCGCGGTTGCGGGCAAGTCTGGTCAATGAGGGCGT
GCTGGCGGAAATGGCGGCGGAAATGAATGTCGGCGACGGCCTGTACTTGGGGGCGGGCGA
GTTGAAGAGCGGCGGCTTCAGACGGCCTTCGATACTGGCAGACGCGATGGAGGCGATGTT
TGCTGCCGTCAGCTTCGATGCCGATTTCAACACGGCGGAAAAGGTGGTGCGCCATTTGTT
TGCCGATCGCGTCCGGCGCGCCGATTTTCAAAATCAGGCAAAAGACGGCAAAACTGCTTT
GCAGGAGGCGTTGCAGGCGCGCCGTTTCGCCTTGCCGAAATACCGTATCGAAGAGCAAAT
CGGTTATGCCAACGACAGTATGTTTGTCATTTCCTGCGATTTGGGCGAACTGGGTTTCGT
GTGCCGTGCCAAAGGGACGAGCCGCAAGGCGGCGGAGCAGGAAGCGGCGAAAGAGGCTTT
GAAATGGCTGGAAGAGAAGCTGCCGCTGAAGAGGAAAAAGAAATGAGGCGGCGCGTGAAT
ATGCCGTCTGAACATATGGATACGAAAGCAAATATGGATATTGAAACCTTCCTTGCAGGG
GAACGCGCCGCCGGCGGATACCGTTGCGGCTTCGTAGCGATTGTCGGCCGTCCGAACGTG
GGCAAATCAACGCTGATGAACCATCTCATCGGTCAGAAAATCAGTATTACCAGCAAAAAG
GCGCAGACGACGCGCAACCGCGTAACGGGGATTTATACCGACGATACCGCGCAGTTCGTG
TTTGTCGATACGCCCGGCTTTCAAACCGACCACCGCAACGCGCTCAACGACAGGCTGAAT
CAAAATGTTACCGAGGCGCTCGGCGGCGTGGATGTGGTGGTTTTCGTCGTGGAGGCGATG
CGCTTTACCGATGCCGACCGCGTCGTGTTGAAACAACTGCCCAAGCACACGCCGGTCATT
TTAGTGGTCAACAAAATCGACAAGGACAAGGCGAAAGACCGTTACGCGCTGGAGGCGTTT
GTTGCCCAAGTGCGCGCCGAATTTGAATTTGCGGCGGCGGAGGCGGTCAGCGCGAAACAC
GGATTGCGGATTGCCAACCTGTTGGAGCTGATTAAGCCGTATCTGCCCGAAAGCGTGCCG
ATGTATCCCGAAGATATGGTTACGGACAAATCGGCGCGTTTTTTGGCGATGGAAATCGTG
CGTGAAAAATTGTTCCGCTATTTGGGCGAGGAATTGCCTTATGCGATGAACGTCGAAGTG
GAGCAGTTTGAAGAGGAAGACGGTTTGAACCGCATCTATATCGCCGTTTTGGTCGATAAG
GAAAGCCAAAAGGCAATTTTAATCGGTAAAGGCGGAGAACGTTTGAAGAAAATTTCCACC
GAAGCGCGGTTGGATATGGAAAAACTGTTTGATACCAAAGTATTTTTGAAGGTCTGGGTC
AAAGTCAAATCCGGTTGGGCGGACGACATCCGCTTCCTGCGCGAGCTGGGTTTGTAGTTT
TTCTTGCTGAACTTTACGCAAATGCCGTCCGAACAGGTTTCAGACGGCATTTTGTTTCAA
TCGGGAATATCTTTGTTAAAAACGGGTTGATATTATCTGTGCATATTATAGTGGATTAAC
AAAAACCAGTACGGCGTTGCCTCGCCTTAGCTCAAAGAGAACGATTCTCTAAGGTGCTGA
AGCACCAAGTGAATCGGTTCCGTACTATTTGTACTGTCTGCGGCTTCGTCGCCTTGTCCT
GATTTTTGTTAATCCGAGACCTTTGCAAAAATAGTCTGTTAACGAAATTTGACGCATAAA
AATGCGCCAAAAAATTTTCAATTGCCTAAAACCTTCCTAATATTGAGCAAAAAGTAGGAA
AAATCAGAAAAGTTTTGCATTTTGAAAATGAGATTGAGCATAAAATTTTAGTAACCTATG
TTATTGCAAAGGTCTCAATCCACTATAAAGACCGTCGGGCATCTGCAGCCGTCATTCCCG
CGCAGGCGGGAATCTAGTCCGTTCGGTTTCGGTTTTTTTGGCTAGTGCCGCAACATTAAA
TTTCTAGATTCCCACTTTCGTGGGAATGACGCGATTAGAGTTTCAAAATTTATTCTAAAT
AGCTGAAACTCAACGCATTGGATTCCCGCCTGCGCGGGAATGACGAATTTCAGGTTGCTG
TTTTTGGTTTTCTGCTTTTTCCAATAAATGCCCCCAACCTAAAATCCGTCATTCCCGCGT
AGGCGGGAATCTAGACATTCAATGCTAAGGCAATTTATCGGAAATGACTGAAACTCAAAA
AACTGGATTCCCACTTTCGTGGGAATGACGAAGTGGAAGTTACCCGAAACTTAAAACAAG
CGAAACCGAACGAACCGGATTCCCACTTTCGTGGGAATGACGGGATGCAGGTTTCCGTAT
GGATGGATTCGTCATTCCCGCGCAGGCGGGAATCTAGGTCTGTCAGTGCGGAAACTTATC
AGGTAAAACGGTTTCTTGAGATTTTGCGTCCTGGATTCCCACTTTCGTGGGAATGACGCG
ATTAGAGTTTCAAAATTTATTCTAAATAGCTGAAACTCAACGCACTGGATTCCCGCCTGC
GCGGGAATGACGAATTTCAGGTTTCTGCTTTTTCCAATAAATGCCCCCAACCTAAAATCC
GTCATTCCCGCGTAGGCGGGAATCTAGACATTCAATGCTAAGGCAATTTATCGGAAATGA
CTGAAACTCAAAAAACTGGATTCCCACTTTCGTGGGAATGACGAAGTGGAAGTTACCCGA
AACTTAAAACAAGCGAAACCGAACGAACCGGATTCCCACTTTCGTGGGAATGACGGGATG
CAGGTTTCCGTATGGATGGATTCGTCATTCCCGCGCAGGCGGGAATCTAGGTCTGTCAGT
GCGGAAACTTATCAGGTAAAACGGTTTCTTGAGATTTTGCGTCCTGGATTCCCACTTTCG
TGGGAATGACGCGATTAGAGTTTCAAAATTTATTCTAAATAGCTGAAATTCAATGAACCG
GATTCCCGCCTGCGCGGGAATGACGAAGTGGAAGTTACCCGAAACTTAAAACAAGCGAAA
CCGAACGAGCCGGATTCCCGCTTGCGCGGGAATGACGGGATTAAGTTTTCAAAATTCATC
AGAAATTACTGATTTAATAGCATAAATTTTTTAGATTATAGTGGATTAACAAAAATCAGG
ACAAGGCGACGAAGCCGCAGACAGTACAAATAGTACGGAACCGATTCACTTGGTGCTTCA
GCACCTTAGAGAATCGTTCTCTTTGAGCTAAGGCGAGGCAACGCCGTACTGGTTTTTGTT
AATCCACTATAAGTCATTCCGGCGGCAATTTTTGTTGCTTTAACGGGATAGGCGGTTGGC
GGTTGCGATAAAGGCGGCGACTTTGGCGGCATCTTTTTTGCCTTTAGACGCTTCCACACC

## Appendix A

GCCGGATACATCGACCGATTCCGCTCCGGTGATGCGGACGGCTTCGCCGACGTTTTCAGG
GGTCAGCCCGCCGGCAAGCACCCACGGTTTGCCCGAATATTCCGCCAGCAGCGTCCAGTC
GAAGCGGTTTCCGGTGCCGCCGTATTCCGAAGGATGGTAGGCATCGAACAGCAGTGCCTG
AGCGTCGGGGAAGCGCGTGGCGGCGTTTCGGATGTCTGATGCCGTCTGAACACGAATGGC
TTTGATATAGGGGCGGTGGAACTGGCGGCAGAATGCGTCGTCTTCGTCGCCGTGGAATTG
GATGATGTGTATCGGCACTTCGGCAAGGATGCGGCGGATGTTTTGCGCGCTTTCGTTGAC
GAAAAGGGCGACAACGCTGACAAACGGCGGCAGTGCGGCGGTGATTTTTTTGGCGCGGGC
AATATCGACGGCCCGGCTGCTGCCTTGGAAAAAGACCAGCCCGACGGCATCCGCACCTGC
CGCTGCGGCGGCAGCTGCGTCTTCCGGTGTGGTGATGCCGCAGATTTTGGTGCGGATTTT
CCTCATTCGGTATTCCTTTATTTGGGAAACGGCGCGCTTTTGCCGTTTCAGACGGCATTC
CCGATCAGTCGATTTTGATGTATTCGACAGAAAGGATTTCAATTTCCTCACGCCCTTCCG
GCGTGTTCAAAACCACTTCGTCGCCTTCGCGCGCTTTAATCAGACAACGAGCCAGCGGCG
AAATCCAAGAAATTTTGTTTTGCGCGGTATCGATTTCATCGATGCCGACGATTTTGACGG
TTTGCTCGCGCCCGTCGTCGCGCAACAGTCCGACCGTCGCGCCGAAAAACACTTGGTCGG
TCGCTTCGCGCAATTCGGGATCGACGACGACGGCAGCCTCCAAACGTTTGGTCAGGAAAC
GGATGCGGCGGTCGATTTCGCGCATACGGCGTTTGCCGTAAAGATAGTCGCCGTTTTCGC
TGCGGTCGCCGTTGCCTGCCGCCCAGTTGACGATTTGGACGATTTCGGGGCGTTCTTTGT
TGACCAGTTGATAAAGCTCGTCTTTCAATGCCTGCCATCCGGCGGGCGTAATGTAGTTTT
TGGTTTCGGTACTCATATTGTGTGCGGATGAAACGGGAAATGTGATGCCGATATGGGAAA
TGCCGTCTGAAAACCCGGCGTTCGGATTTCAGACGGCATCGCGGTTTGGGAAGCCTTATT
CTTCGTCGCCCGCATCGCTGATGCTGATGCTGTGTTCCATCCTGCTCGGGTGGATTTTCA
GACCGCCGCAGCCGGATTTCTCGGCAGACAGGCGGTCGAGGTAGGCATCATCGATGTCGC
CGGTCTGATAAATGCCGTTGAAACAGGACGAATCGAAGGATTCGATTTTCGGATTGAGTG
CTTTGACGACGGCTTCCAAATCGCCCAAGTCTTGAAATACGATGCCGTCCGCGCCGATTT
CGGCGGCGATTTCCGCCGCGCTGCGCCCGTTGGCAATCAACTCTTCGCGCGTGGGCATAT
CAATGCCGTACACATTGGGATAGCGCACTTCGGGCGCGGCGGAGGCGATATAGACTTTGC
GCGCGCCCGCCGCGCGTACCATTTCGACGATTTCGCGGCTGGTCGTCCCGCGCACGATGG
AGTCGTCCACCAGCAACACGCTTTTGCCTGCAAATTCGGTTTCCATCGGGCTGAGTTTTT
GGCGCACGGATTTTTTGCGCGTCGCCTGTCCGGGCATAATAAAGGTGCGGCCGATATAGC
GGTTTTTAATCAAACCCTCGCGGTAGGGTTTGTCGAGATGGACGGCAAGCTCCATCGCGC
TGGGGCGGCTGGTGTCGGGAATGGGCATCACGACATCGATGCCGTCCACGGGCAGCTCGC
GTTTGATTTTTTCCGCCAGCGACACGCCCATATCCAAGCGCGATTGGTAAACGGATACGC
CGTCAATCACAGAGTCGGGGCGGGCAAAATAAACATATTCAAAAAGGCAGGGGCTGAGTT
TGGCACGGTCGCTGCATTGGCGGGCAATCATTGTGCCGTCAAAGCCGACAAATACCGCTT
CGCCCGGCCGGATGTCGCGTTCCAAATCGTAGGTAAGCGCGTTGAAGGCGACGGATTCGG
AGGCGACGGCATAGGATTTTCTGCCTTCGCTGTCGGTTTGCGAACCCAATACCAGCGGGC
GGATGCCGTAAGGGTCGCGGAAGGCGAGCATACCGTAGCCCGCAATCATGGCAATCACAC
CGTATGCGCCGCGCACCAGGCGGTGGACTTGGGCAACGGCGTTGAAAATATTGTCGGCAT
TGAGCCGGTGCGGGTCGGCGTTTTTAGAGACTTCGCGGCGTAATTCGTGCGCGAATACGT
TGAGCAAGACTTCGGAATCGGAGCTGGTGTTGACGTGGCGCAGGTGTTTGTTACACACGT
TTTCATACAGTTCGGCAGTGTTGGTGAGGTTGCCGTTGTGCGCCAAAACGATGCCGAACG
GCGAGCTGACGTAGAAAGGCTGCGCCTCCGCGCTGCTGCCTGCGTTGCCCGCAGTAGGAT
AACGGACGTGGGCGATGCCGGCGTTGCCGGTCAAATCGCGCATATTGCGTGTGCGGAACA
CTTCGCGCACCATGCCTTTGCCTTTGTGCATATGGAAGGTACCGCCTTCCGCCGTTGCAA
TGCCCGCCGCATCCTGCCCCCTGTGCTGCAACATCTGCAAGCCGTCGTACAGAAGCTGGT
TCACGGGTTCATGACTGACCAAACCTAATACGCCGCACATATCGTCTTCTCCGATTCGAG
GTTTAAGGGTAAAACGGAATTATAAAGTAAACGGTGGTTTTTTGCCTGAATTGTTGACAA
TATTTGAGCGAAGGACAGATAGGTGGGTTTATGGAGAATAAGATTTATAGTGGATTAAAT
TTAAATCAGGACAAGGGGAGGAAGCCGCAGACAGTACAAATAGTACGGCAAGGCGAGGCA
ACGCCGTACTGGTTTAAATTTAATCCACTATAATCTGTGATATGGCTGAGGAAAGGAAAA
AACATTTCAGACGGCATAAAAGAGGATGCCGTCTGAAATATCCGTATGGCAATCAATCGT
CTTCCGGAGTTTCCGCCGTGCCGCCGCTATGGTTCAACACGGCTTCGGAAAGCGATACGA
AAAACGGCAGTGTGTAAGATTGCCGCCATTCTTCGGTATCGGGCAGGTCGGTTTTTGAAG
CAAGCATGACCAGCAGGGTAACAATCAAAACGCCTTTCAATGCACCGAATACGCCGCCCA
AAATGCGGTTGGCAAAGCCCAAACCGACCGCCGAAACTGCGCTGGTCAGCAGCGAACGGA
GCATTTTCTGGATCAGACAGGCAATGACGAACAGGGAAATGAACGACAGAGCCAATGCAA
ACAGGCGGGGTTGGAACGAGGCAAAGGCGAGGTCGGCGAAGGAGGCGGCAAAGAGTTTGG
CGAAAAAGAAGGAAACCACCCATGCCGCCATTGAGCCTGCCTCCGCAATCACGCCGCGCA
TCGCGGATAGCACGATGCAGGCGGCGATGACGGCGGAGACGAGGAGGTCGGCAATGGGGA
GGCTATTCATTCGTTACCTGACCGGCGATACCGTGTACGCGCAATTTGTTCAAATCGCGT
TCGGCATCCCTTGCGTTTTTATAGTTGCTTGATTTGACGCGGTAAACTTTGCCGTTGTCG
GTCATAATTTCGGTGATGGTCGAATCGATACCCGCCGCCTTCATTTTGCGCTGGAGGCTT
AAGGCGCGTTCTTTTTCGGCATAACCTGCCTGAATGGCGGCTTTTTTACCGGATTTTTCC
TTCTCGGCGGTTTTGGTCTTGTCCGCTTTGTCGGTTTTTTGTGCCGTTTCGTGTTTTTTG
CCGTCCGAACGGTCTTTTTCGGCTGTTTTTTTGCTTTCAGCCTTGTCGGCTTTTTTCGCT
TCTTTTACCGCGCTGTCGGATTTTGCCGTATCCGGTTTGGTTTTTTCGGCGGCAGTTTTC
GGTTTGTCGGCAACTTTTTCGGCGGTTTTGGTTTCTTTGGCTTTGGGCTTGGCTTTGGCA
GTGCGTTCCGCTTTTTGCGGTTTTGTTTCGGCAGTGCGTTTCGGTTTTTCAACCGCTACC
GTATCCGTACTGTCGGCAGTTGCCGGCACTTTTTCGGCAGCGCGTTGTTTTGCCTGCTTC
GGTGCGGTTTTGGCGGTTTCTGCCTGTTGCAGTTTCTCGGATGCTTCCAAACCTTTGATG
TTGCTGTCTTCGAGGCGCTCGTTAATCAGCACCAGCGGCGCGCCTACGTTTTCAGGCTCG
CTGATTTCGCTGTCGGCGGCAGAAGGCTTGTCTTCGCCTGCCAAGTCCTGCGGTTTGTCG
GCGGCGGATTTCAAGGCAGGGGTTTGTGCCGCACCTGCCGCTTTGTTTTCTACGCCGCTT
GTTTCGCCGGCAGTCTGTTCGGCAGGGCCGGAACTGAGGGCGGCTGCCAGCAGGATGCAG
GAGGCGGCAACCAGGCAACTTGCCGTTACGAGGCGGCGGCGGTTGCGCCGTTTGAGTTGT

## Appendix A

TCGTAACCGCTCAGGACTTCGTTTTGTTTGTTTTCGGACATAGAAGTTTCCTTTTAAAGT
ACCGACATGACATCGGCAACGGTATGAAATGAGCCGAAAACGACGATTCTGTCGTTCTCG
CCCGCTTTTGAGGCTGCCGCCCGGTATGCTTCGCGGACGGCGGCGAATGTTTGTATGTTT
TCGATGCTGTGTTCGTGCAGTTTGTTTTGCAGGCTCTCGAGCGTCATGCCGCGCGGTACA
TCCAACGGTGCGATATACCACTCGTCAAACTGGTCTTTAACGGTTTCCAACACGCCGTCT
ATGTCTTTGTCGGACAACATGCTGAACACGGCGGTGCGTTTTTGCGCAAACGCCAAATTA
ATCAGGCTGCGGCGCAGGGCGCGGGCGGCGTGGGGATTGTGTCCGACATCCAAAACGGTC
AGCGGCCGGCCGGGCAGGACTTGGAAGCGTCCGGGATTTTCAACCAGCAACAAACCGCGC
TTGATGGCACCGATGTCCACCGGCAATTTGTCGTTCAAGCATTCCAATACGGTCAGCGCG
CAGGCGGCATTGGAAAGCTGGTATGTGCCGCGCAATGCGGGGAAGGGCAGGGCATTGCGG
TTGCGCGCGGGGTCGTCTGAATGTTGCGGCCGGAAGCGGTAGTTCCATTGGATGTTTTCC
ATCGCGTGAAACTCGAAATCGCGCTGCACCATCAGCAGTTTCGCGCCTATGGCTTCGGCG
TGTGAAAGCAACGATTTGGGCGCGGGGTTTTGACCGCAGATGGCGGGTTTGCCGCTACGG
AACACGCCTGCTTTTTCAAAGCCGACCTGCTCGACCGTATCGCCCAAAAATGCCTGATGG
TCCAAATCCACGCTGGTAACCACCGCGCAATCGCCGTCAAACGCGTTGACCGCGTCCAAG
CGGCCGCCCAAGCCGACTTCCAATATCATCACGTCAACCTGTTCGCGCATGAAGATGTCG
ACAGCCGCCAAGGCATTGAATTCAAAATAAGTCAGTGATATTTCGCCGCGCGCGGCTTCG
ATGCGCTCGAAAGAGGCAATAATCGTATCGTCCGAAACGGGTTCGGCGTTGATGGCGATG
CGTTCGTTGTAACGCAATAAATGCGGGCTGGTCAGCGTACCGATTTTGTAGCCCGCCTGT
TTGTAAATCTGTGTCAGGTAGGCACAGACCGAACCTTTGCCGTTGGTTCCCGCGACAACG
ACGACGGGGCATTGCGGCTCGAGCTTCATGCGTTTTTTCACTTCGCTCGTGCGCTCCAAA
CCCATGTCGATCAAACCGCCGCTGTGGGCGGTTTCCAAATGCGAGAGCCAGTCTTGTAGT
GTTTTCATGAGTGTTTCGTTTTCAAATGCCGTCTGAAATCAGTCTGATGTATCGGTTTCG
GCGGTTTTTTTCGGCTGCCGCCAAAGTACCCAAACTTTCAGCTTGCGGTAGGATTCTTTG
TCCGTCATGTCGGGCATGATGCATTGGCGGACGGTTTTGCCGCCGGTGTCCCATTGTAAG
AATAAGGCATAAGGCGTAACCATACTGCTGCCCGACAGTGCCGCCGCCTTTGCCGTTTTG
TCTTTGCCGGATACGATTTCCGCCTGTCCGTCGCGGTCTATGGTAATGGCGGTTATGGCA
TGGCGGTGTTTCAGATTCGTTATCCTGAGCGAGTATGCGTAACTTGCCACCAAAGCCGCC
AAACCGAACCACATCATCCGGCCGTAAAACCAAGTCAGGCAGACGGCAAGGGAGGCGGCG
TGAAGCGATACAGTCAGGATGTTCAGGATGCGGGACGGCCTCAATGCCGTCTGAAAGGCG
CGCACAGCCTTACATCATGTTGTCGAACACGGGGGTAATGTTCAATTCCGCTTCTTCCAT
GTTCAACACTATATCGTGGATTTCGATGTCGAAAAATTCCCAAAACGCCTTCAGCCCCAT
ATCTTGCGGCCATTTATCCTTATCGATGTCCCAACCTGCCAGCTCCGCCTCGAAAATCTG
CCGGTAGCGTTCGTCGAAGTAGGAAACGACGGCTTCCGGTTCGTCGAACTGCGGAACGAG
GAAGACGGAACAGTTGGCACGAAGCTGCTCTATGGTCAGGTCGGGCATATTTTCGTCGGT
GCTTTTGAGCCATTCCAAAAAGCGCGCGGTCGGCTTGAGGACGACGGCGGTGCGGTCAAC
AAAATACATGGTTTTCTTTCTCAATCATCTTGCGGTGTCGGGATATGCTGTCTGAACGTT
CGGTTTTCAGACGGTATAGCATCAGTGGGTCATGACCTGTTGCAGGAACTGCTTGGCGCG
TTCGTGTTTCGGGTTGGTAAAAAACGCTTCGGGCGTTTCGTCTTCGAGGATTTGCCCTTT
ATCGACGAAAATCACGCGGTCGGCAACTTCGCGGGCAAACCCCATTTCGTGGGTTACGCA
CATCATCGTCATGCCGCTTTCTGCCAAGTCTTTCATCACTTTCAACACTTCGCCGACCAT
TTCGGGGTCGAGTGCGGAGGTCGGTTCGTCAAACAACATTACGCGCGGTTCCATCGCCAA
ACCGCGTGCAATCGCCACGCGTTGCTGCTGGCCGCCGGAAAGTTGGGAAGGGAAGGCGTC
TTTTTTGTGTGCCAGTCCGACGCGTTCCAAAAGCTCCATTGCCTTTTTCTCCGCCTGTTC
CGCATTTTGCCCTTAACCTTCATCGGTGCGAGGGTAATGTTTTCCAACACGGTCAGGTGC
GGGTAGAGGTTGAAGCCTTGGAATACGAAGCCGACTTCTTCGCGGATTTTGTTCAAATCG
GTTTTGGGGTCGGCAACGTTGACACCGTCCACCCAAATCTCGCCGCTTTCGATGCTTTCA
AGCTGGTTGACAGTGCGGATGAGTGTGGATTTGCCGCTGCCCGAAGGCCCGCAGACGACG
ACCACTTCGGGTTTTTTGATTTCCAAGTTTACGCCGTTGATGACGTGCAGGTCTTTGAAA
TGTTTGTGTACGTTTTTGAATTTGATCATGTCTTTTCTTTCAGACGGCATTTGCCGTTGC
AGGTTGTCGTTACGGGAGCTCCATATGATGAAGCGTGTAGCGTCTGCCGTCAAAAAAACG
GTCGTTCGGATTGGTCAGGCAGGCTGCAAGCGGCAGTATCAGGGGTAAAAGCAGGTATTT
CGTCATCGGCTTACTCCCTTTTCAGACGACCTTGCCCGCCAGATAATTGCTCAACGCCAC
GTATTTCTCTGGCGCGATATGTTCGGCGCGGTCTTGCGGATTGATGCCGACTGCCTGCAA
ATCATCGTCGCCTGCAAGCTCTTTCAGATTGTTGCGTATGGTTTTGCGGCGTTGGTGGAA
GGCGAGTTTCACGAGTTTGGCAAAATGCTCGAAATCGTCCGCCTTGCCGATGCGGTGTTT
CACCGGAATCATACGGACGACGGCGGAATCCACTTTCGGCGCAGGGTCGAACGATTCGGG
CGGTACGTCAATCAGCATTTCCATATCGAAAAAATATTGCAGCATCACGCCCAAGCGGCC
GTAGTCGTTGCTTTTCGGCGCGGCAACCATACGCTCGACCACTTCTTTTTGCAGCATAAA
GTGCATATCGACGACATCGTCCGCCACCTCCGCCAGCTTGAACAAAAGCGGTGTGGAAAT
GTTGTACGGGAGGTTGCCGACGATTTTCTTTTTGCCTGCGATGCCGTTGAAATCAAACTG
CAATACATCGCCTTCGTGAATCACCAGTTTATCCGCAAACGGCAGCGTTTTCAGACGGCA
TACGATGTCGCGGTCGATTTCGACAACGTGCAGGCGGTTCAGCTTTTTCGCCAAAGGTTC
GGTAATCGCCGCCAAACCCGGGCCGATTTCAATCACGACATCATCCGCCTGCGGGCGCAC
GGCGTTGACAATATCGCTGATAATCCGCGTGTCCTGCAAAAAATTCTGCCCGAAACGCTT
GCGGGCTTTGTGTTCTTTCATCGTGTTTCCTTTTCGGTTGAAACCCCGCCCTTTAGGGCG
GTAGAATCAGACTCTATTTGGGAGGGGCGTAACTCTTTCCAAATCAGGATGGCACATAGG
GCGGTGCTTTATGTGTCGTCCTGTGTGTTGAAACATAAATGTGTTTACAGTATCCGTTTG
ATGTCGGCATTGTAACCGAAAACGGCAGGGCGTGATAATGCTGTTTGAAGGCTTGCCGTG
TTTGGCGGTTTGGTGCAAAAACCGGCTGTCTGCCGTTTTGCCTGTTGGAGGATTGAACGT
GTCTGAAAATCTGCTTGAAATCGAAACCCATCCCTTCGATCCCGTGTTGCCGCCGAAGGC
TGCTGTCATGATGATGGGGACGTTTCCGCCCAAGGAAGACAAACGCGCGATGCAGTTTCA
TTATCCGAATTTCCAAAACGATATGTGGCGCGTTTATGGGCTGGTGTTTTTTAATGATGC
GGCGCATTTCCAAAGGTTGTCTGAAAAAGCGTTTGATGCCGAGAAAATCAAGGCGTTTTT
GCACGAACGGGGGATTGCGTCCTGTCCGACCGTTTTGAAGGCGGTACGTCAGCACGGCAA

## Appendix A

TGCGTCCGACAAGTTTTTAAAGGTAGTTGAAACCGTCGATTTGGCGGCGGTGTTGGCAAA
AATACCCGAGTGCCGCCATATTTGTACGACAGGCGGCAAGGCGACGGAAATCCTGCTCGA
TATTCAGGGCGGCGGTATCAAAATGCCGAAAACGGGCGAAACCGTGCCGTTTCCGTTTGC
CGGACGGGATTTGACGCTGACGCGCCTGCCTTCGACTTCGCGCGCCTATCCTTTGAGTTT
GGCGAAAAAAGCGGCGGCGTATCGGGCGTTTTTTGAAATGGCGGGCTTGTGTGAAAAACA
GTTATAATTGCCGACAATTTCCCGTTCAGACGGCATGTTTGCAAAAACGGAAATGCCGTC
TGAAAATTTGAAGCACAAGGAAGAATCCGATGAAGAACTACCACGCGCCCGACGAGAAGG
GCTTTTTCGGCGAACACGGCGGGCTTTATGTCTCCGAAACCCTGATTCCCGCCTTGCAAG
AGCTGGCGGATGCCTATAAGGCAGCGAAAAACGATCCTGAATTTTGGGAAGCGTTCCGCC
ATGATTTGAAACATTATGTCGGCAGGCCCAGCCCCGTTTACCACGCCGCGCGGTTGTCCG
AACATCTGGGCGGCGCGCAAATCTGGTTGAAGCGCGAAGACTTGAACCACACCGGCGCGC
ACAAAGTCAACAACACCATCGGTCAGGCACTGTTGGCAAAACGCATGGGTAAAAAACGCG
TCATCGCCGAAACCGGCGCGGGTCAGCACGGCGTGGCGAGTGCCACCGTTGCCGCACGCT
TCGGTATGACTTGCGACGTGTATATGGGCGCGGACGACATCCAACGCCAAATGCCCAACG
TGTTCCGTATGAAATTATTGGGTGCGAACGTGGTCGGTGTAGAAAGCGGCAGCCGCACGC
TGAAAGACGCGATGAACGAAGCCATGCGCGAATGGGTCGCCCGCGTGGACGACACGTTCT
ACATCATCGGTACCGCCGCCGGCCCCGCGCCGTATCCCGAAATGGTGCGCGATTTCCAAT
GCGTGATTGGCAACGAAGCTAAAGCGCAGATGCAGGAAGCCATCGGCAGACAGCCCGACG
TTGCCGTTGCCTGCGTGGGCGGCGGATCGAACGCCATCGGTTTGTTCCACCCGTATATCG
GCGAAGAAAACGTGCGCCTCGTCGGCGTGGAGGCTGGCGGTTTGGGCGTGAACACCCCCG
ATCACGCCGCGCCGATTACTTCGGGCGCACCGATTGGCGTATTGCACGGTTTCCGCAGCT
ATCTGATGCAGGACGAAAACGGTCAGGTTTTGGGTACGCACTCTGTTTCCGCAGGCTTGG
ATTACCCCGGCATCGGCCCGGAACACAGCCATCTGCACGACATCAAGCGCGTCGAATACA
CTGTTGCCAAAGACGACGAAGCACTCGAAGCCTTTGACTTGCTCTGCCGCTTCGAGGGCA
TCATCCCCGCGCTCGAATCCAGCCACGCCGTTGCTTGGGCGGTGAAAAATGCGCCGAAAA
TGGGTAAAGACCAAGTGATTTTGGTCAACCTCTCAGGTCGTGGCGACAAAGACATCAATA
CCGTCGCGAAGCTCAAAGGGATTAAACTGTAACCTCGTCCGTCTGATATAGTGGATTAAC
AAAAACCAGTACGGCGTTGCCTCGCCTTGCCGTACTATCTGTACTGTCTGCGGCTTCGTC
GCCTTGTCCTGATTTTTGTTAATCCACTATAAAAATGCCGTCTGAAGCCTGAGTTCAGAC
GGCATTTTATTTTGCTATGAATTTAGTATTTTAGAAACGAATCTGTATTTTAATTTGTCC
GGATTTTTGTTTTTCCAATTGTTTTCCTTTTGTAATACTGCCATTTACGTTTAATGTAAC
ATTACGGTACAGTAACGCGGCACCTGCTGAATATTGCTGTTGATTATCTGCTTTATAGAC
GAAGGAATTACCGCCCACATTCACGCCGCCTTTGCCATAATTGGCAAAGTAAGCTGCAGA
TAACAAGGGTTTTACGGTAAGGTTGCCGACTTTAAACCGATAAGCAAAATCCAGTCCGGC
CGTTAGTGTTTTCACTGACATAGAACTTACTTTAACACTGTCGTTACCCAACTTGTAATC
TGCAGATGACAGGCGGCTGTAACGGATCCCCGCACTGGGGACAATCTCGAATTGATTGAT
TTTCAGCGTATTGCCCAAAGTAAGGCCGGTTTGGATGCTTGCTCGGTTAAAGTTTGCTTT
TTGCTGCGTTTGTAACCGGCTTCTCAAGCTGCCTGCACCAATATCGCCGGCCACATACCA
AGCATCATTTAAATAATACTTACCATAAAGGTTGGCTTGCACAAAAGTATTTTTGCCGCT
CGCCTGATCAAAAGTATGCTGACTGTCAGAGTAAGTCAATACGCCGCCTATCTGCATATT
TTCGGACAAGCTGCGGTCAATGCCGATTTGTGTTTGCGTGCGTTTCGAACTAAACCGGCG
ATATTGTGCGGAAGCATAATCACGACCATAACCGGTGTTCGACATCCAAACACTGTTTTT
TTCGGCATCAGCGCGTGATTTTTGTGCAATGTGCCGTGTTAATGAAGCACCTGTATCCAA
CAAGATAGATTGCGTGCTTGCCATTGCGTCAGATAAAGCCGAGTTGGTATTGGTGCTGAC
TGCATCGGCTTGCGCGGCGGCTTGGGCTTGCAGCTGAGTGGCGGCTTGGGCTCGCGGCTG
CGCGGCTCTTGGTTGCAAACTCACTGTCTCAACTTTTTCATGAAGTTCCGTATTGTCTTG
AGGCTGTTTGTCTGATGTGTCAACCGATTCGGATACATCTTCATCTTCCAACGCATCCAA
GGGGATTTCTTCATAATCATTTTCATATTTTTCATGAAGTTCCGTATTGTCTTGAGGCTG
TTTGTGTGATGTGTCAACTGATTCAGATACATTTTCATCTTCCAACGCATCCAAGGGGAT
TTCTTCATAGTCATTTTCATACCAGTCCGGATTATGCAAGGCCCTGGGTGCTGCGTAAGC
TGACGAATCAAATGATGGCGAGGGCGGTGCCGGCAGAGTAGATCTGCGTCCGCGACGTTT
CGGACGATTTTGGGAAGCTGCCATATAATCCTGAGGTGCGGCACGGCGTTTTCGGCGTTG
CGGCTGGGATTGGGCTGCTTGACGGTGCTCTTCTTCCTCAGCTTTTCGTTTCGCCGATTC
TGCCGCTTCTCGATCTGTTTCGGCTTTTTGTTTTGCCGACAACTCTGCTGCTTGGCGTTC
CGCTTCTTGACGACGTGCAAGTTCGGCGGTCTGGCGAGCTTCTTGCTGGCGTTTTGCTGC
TTCGGCTGCTGCCCTTTGCTTGGCAAGTTCGGCGGTTTTGCGTTCGGTTTCCGCTTTTTG
TTTGACGGCTAACTCTGCAGCTTTTCGTGCTTCTTCCTGTTGACGCGCAAGGGCTTTTTG
TTGGCGTGCCGCCAATGCTTGGGCTTCAGCTGCGGCTTTTTGGTTTGCCGACAATTCTGC
CGCTTTGCGTTCTGCTTCCTGGCGATGTGCAAGTTCGGCAGCTTGGCGTTTTGCCTCTTC
GGCTTCTGCTTTTCGGCGCGCAGCCAATGCTTGAGCTTCACGTTCGGCTTCTGCCCTTTG
TTTTGCCAACAACTCTGCCGCTTTGCGTTCTTCCTGCTGTCGGCGCGCAAGTTCTGCTTG
GGCTTGGGCAATTTCCACATTGTTTTGCTGTACCGAACGGTGTCCGCGGCGTTTAGGACG
GTCTTGGGAAGCTGCCATATAATCCTGCGGTGCGGCACGGCGTTTGCGGCGTTGCGGCTG
GGATTGGGCTGTTTGACGGCGTTCCTCTTCCCCGACCCTTTGTTTTGCCGACAACTCTGC
CGCTTCGCGTTCTTTTTCATGCCGGCGTGCAAGCTCTGCAGCTTGGCGTTTTGCCTCTTC
GGCTTCTGCTTTTCGGCGTACCGCCAATGCTTGAGCCTCACGTTCGGCTTCGACTTTTTG
TTTTGCCGACAACTCTGCCGCTTCGCGTTCTTTTTCATGTCGACGTGCAAGTTCGGCGCT
GCTGCGTTCTTGTTCTGCTTTTTGGCGGGTCGCCAGCTCTCTTGCTTCGCGTTCGGCTTC
TGCTTTTTGTTTTGCTAACTCTGCCGATTTACGCTCTGCTTCTGCTTGCTGACGCTTCAC
TTGCTCCGCTTTTGCTTGCTGGCGTTTTGCTTCTTCGGCTTGATTTGCCTGCGGGCTAGG
CGGTGCGACGACGATATTTTGAGGCTTGGCAATTTGTGCACCGTCCGTTTGTGTTGCCTT
TTGTGCTTGAGAAGCCGTGTTTGTGGCAGGAGACGGGGCCGGTTTGACTCGGCGGCGGTT
CTCGGCATAAGGATTGTACAATCGGGTAATACCGTTTTCTGTTTTGATTGTATAACGCAA
TGCCCCTAAATCTACATGGTTATTCGCCAAGGAAACAGAAAGGCGGGAGCGGTCTTGTAC
GGATGATGCATCAAAGAGATTCAGCCCTTCCTGATTGGGTTCGCCTGTTTTATCTTGAAC

## Appendix A

ATGGAGCTGATAATGACCGGATGCGGATTCCTTCACAAGCACTTTATCCCCAAGATTTTT
CGCCAGGTGCGTCAGATAATGAAAATGCCCGTTACCGGATAAATGATTGATTTTGAGCGT
GTGGTATTTATTTGCACTTTGCGCATCGGAGGCATTGTTCAAATGAATATGGCTATCCGC
CAATGACAGATTGTGTACTTGGCTGTCGCCGGTCAAATGCCATTTGCTATGTTGGTTTAG
GCTGACACGGCTGTTTCCTTGCCCTTGAATTTGCCCCCATAATGCAGCCTTGCCCAAGAC
CAATGCCGCATTCTGGTTCAGATTCACATTGCCGTTAATCTGTGTCGCTCCAAAGCTGTT
TAAAGCCTTATCGGATAAGTTGCCTGTGTTGCAGGTAACGTAACCGGTATAGTCCGAGCG
CACGCAAACCTCATCGCCGTTTTTGTAACCCAAATTTACTTTGGCGTTGTCTGTTGCGGT
GATGTTGGCGGTGATGTCGGATACATTTCTGCCGGAAGAGAATGATGCGGATTGATTAAC
CGCAATTTCTGTGGCTTTGAATGTGCGGTTTATCCAGTCGTCTTCAAATACGACTTCATT
GTTTTTGGAGAAATGTGCGTCTTTTCGGGCTGAAGATTTGTTCACAAAATCTCTTGCGTG
TGGTGTTGGACGACCTGATAACAAGACATTGCCTTGAGTTACGCTTATTTTTCCGTTTAA
ATTAGTGCCGCCTGTTAACAAGAAACGGTTTTGCGCGCTTTTGCCGTTGAAATTAAGGTT
TAATGCACCGTTATGTCCTTTTCCGTTTTCTTCGCCAAAGAAACCGCTAAAACCGCTAAT
ACGCTGATTGTTTTTGTGGTTCATCGCGTTTTTCTTGGCTTCTTCTTGTGTGCTGCCCAT
TAAAATCCAGTCGTTATTTTCCGTTTGTCCGTTTTCGGGCATCGGTGCGTTCACGCTGCC
GCCGGATTTTAGGGCGTAATAACGGTAGTTTTTGAAATAAAGATCTTTGCCTTGTGGAAT
CGGTTTCCTAGGGCGGTAATAGTAATAACCAGCATCGTCATCATCATTATTTTGAATATA
ATGAATAGAGATGGTTTTGGGATCGGTAATCAAAGATTTACCCGTTAGCGTGATTGTGGA
GGCGTGGCCTGTGTTGTGGTTGACAATGCGCGCGCCTTCATCCACGTTGCGGATGTGTTC
AAAAGTCAAGTCATTGCCATTGGCATCCAAACGACCGCCACGGAAACCGAAATATAGGTT
ATCGGGATTAATCTGATTTGAACTATTTAATACCAATGTACCGCGTCCGCTGACAATGCC
GACTTGGGAGAAAGCCTGGACTTTTTTGTCGGCATCGGCTTGTTGATTCAGAATAACCGT
ACCGTCGCCGACTTTTAATTGCCCTTGGTTAACGCCTGTGCCGTTTATTTCTAATGTGCC
TTTGCCGATTTTTGCCAATCTGTCGCCATTCGGATTTTTGACTTGCCAAACGACTTTTTT
GCCGTCGGCAACATCAATCCCCGCACCTAGCCAAGTGATGTCATTATTTATACCTTTGAC
TGTGTAATCGCCTTTGAAAAACAGACCGCCCGCGCCTTGGTTGATGTTTTGATCCAATAC
CAAAGTGCCGTTGTTTTCAAAGGTAACATTCTGTCCGTTGTTTGCATCCCTTTCATTGTT
GGCAAGCCTTACCGCTGTCGAACCGATATGGCTGTTTGTGCCCGTGGTTTTCCAATGATG
TTCTCCATTACCTTTGATGGTGCCGGCGTTATCGCGTTGTTTGATTTCATCTGCAAATTC
TTTTTTATAGATATTCCATTCTTGCCAAGAGTTTTTTTGATAGCCTGCCCAATAATCGTA
AGCACCTAAAAAGACCCAGCGTTTTTCTTGTTTATCATAAGCAAATAATGGTGAACCGCT
ATCGCCTAACACAGCATAGTTAGTCAATGCGTTTTGAGATAAAACTTCTTTGAGTTTTTC
TGGGGAATGATGTTTTGAATTATCACCGAAGCCAATCAAGCCTTCTTGATTTAGATTCGA
TGTGACATTCACGTCTTGATAAGGCGTACCTGCAATGGCATAACGATAAGCTCGTGAAAG
CTCTGTCATATTGTAGCGGCTGTTATATTCAAATTGCGTACCTGCTCCAACTCGCACAAA
CTCAGAAAAACGGTTTTTATCTTTATAGGTTTCAACGCCGCCGCCTGCACTGGTTGGTGC
AATAGGTGCGACTTCTGTTACGAATTTATTAAGGCGTGCCATGTTGTAGTCTTCAAGACG
ACCTTGATTACCGTGATGCCAATTTTTATTTGGTTCGTAGTCATTTTGTGCAACTGAACG
ATATTCGTTTTCATCATTGCTAACATCTAAGTGCCCATTGTGATGCCCGTAATAAGAGAT
TTCGTCTCCTTTTACATGTTTGACGCTGACGGCATACTGGGGATCGATGACGGTTAATGT
ACGTCTGTTGACATCTGCAACGCTAAAATCAATCATCGGTACGTTGGATAATGCGTTGCC
GATGTTTTGACCTTGTTTGTTTTTCACTGATAAATCGGTTGCGCCGACAAAAAATTTGCC
TTTGTTTTCTGCAAAGTCACGGAATATTTGATAATCGACATCGTCTCTGACCAATGCCGC
TTCTGAGTATGGCGTAAGGGCATAGGCAAGAAAGATGGATAAGGATATGGCGTTAATTTT
AAAACGTTTGGTTTTCATAAGGTTTTACCGTTTTAAGGGTGATAATGTTTTGTATTTTAC
GCCGGTTTGGTGGTGTTGACTATTTTTTGTTGTGTAAATCATTCATTTTGGTGTTTTATT
GCCAATTTAAAAAAAGAATCCCGATGTTTTTATTTCCGCTTCCTTTGTTCTGTTATTCAA
GCGAAGGCGGGAAGCCGATTTTCGGGGTTCGGTTCTTCCGTTAAATTTCTGCGGCTTTTT
GTTTTTGGATTCCCGCTTTTGCGGGAATGACGGGATTTTAGGTTTCTGATTTTGGTTTTC
TGTTTTTGAGGGAATGACGGGATGTAGGTTTTCTTAACCCTGAGTCCTAGATTCCCGCTT
TCGTGGTAATGACGAGATGGGGGTTCGTGGGAATGACGCGGTGCAGGTTTCCGTACGGAT
GGATTCGTCATTCCCGCGCAGGCGGGAATCTAGACCTTAGAACAACAGCAATATTCAAAG
ATTATCTGAAAGTCCGAGATTCTGGATTCCCACTTTCGTGGGAATGACGGGATTTTAGGT
TTCTGATTTTGGTTTTCTGTTTTTGTAGGAATGATGAAATTTTGAGTTTTAGGAATTTAT
CGGGAGCAACAGAAACCGCTCTGCCGTCATTCCCGCGCAGGCGGGAATCTAGACCTTAAG
GCAGCGGCAATATTCAAAGATTATCTGAAAGTCCGAGATTCTAGATTCCCGCTTTCGCGG
GAATGACGAAAAGTGGTGGGAATGACGGTTCAGTTGCTACGGTTACTGTCAGGTTTCGGT
TATGTTGGAATTTCGGGAAACTTATGAATCGTCATTCCCGCGCAGGCGGGAATCTAGAAC
GTGGAATCTAAAGAAACCGTTTTACCCGATAAGTTTCCGCACCGACAGACCTAGATTCCC
ACTTTCGTGGGAATGACGGGATTTTAGGTTTCTGATTTTGGTTTTCTGTTTTTGAGGGAA
TGACGGGATGTAGGTTTTCTTAACCCTGAGTCCTAGATTCCCGCTTTCGTGGTAATAACG
GGATGTGGGTTCGTGGGAATGACGATGGAAAGTTTGCCGTTGTCTCGGATAATACTGAGG
CTTTTCGTTTGCATTCTTATAGTGGTTTAACAAAAACCAGTACAGCGTTGCCTCGCCTTG
TCGTACTGCTTGTACTGTCTGCGGCTTCGTCGCCTTGTCCTGATTTAAATTTAAACCACT
ATATCATTTTCAAATCTTGTTATGACGGTTTTTCGGATTTGCTTTATTATCCGTTTATTT
TTGAAATATCTGGGGTGGGGAGACGTGTTCCGTCGTTGGTTTTTGCCGTGTTGGGTTGTC
GGCGCGGCGGCTTCGTTTGCGCTGCCGGTCGTGCCGCATTGGCTGTTTTGGCTGGCGGCT
TTTGCGGTTTTTGCTGTGTTTGCAAGGCGTTTTGCGTTTGCCGGTCTGATGCTGTGCGTG
TTGGCGGGCGCGGCTTACGGCGTATTCAGAACGGAAGCGGCACTGTCTTCGCAATGGCGG
GCGGAGGCGGTTTCAGGTGTGCCGTTGACGGTGGAAGTGGCGGATATGCCGAGGTCGGAC
GGGCGGCGCGTGCAGTTTGCGGCAAAGGCTGTGGACAGCGGTGGTCGGACGTTTGATTTG
CTGCTGTCGGACTACAAACGGCGCGAATGGGCGGTCGGGAGCAGATGGCGGATAACGGCA
CGTGTGCACCCCGTCGTCGGCGAATTGAACCTGCGCGGTTTGAACCGTGAGGCGTGGGCA
TTATCCAACGGGATAGGCGGCGCGGGGACGGTCGGTGCGGACAGGGTTTTGCTGCATGGC

## Appendix A

GGAAGCGGTTGGGGGATTGCGGTTTGGCGCAGCCGCATCAGCCGTAATTGGCAGCAGGCG
GATGCGGACGGCGGGCTTTCAGACGGCATCGGGCTGATGCGCGCGTTGAGCGTGGGCGAA
CAGTCGGCATTGCGCCCCGAATTGTGGCAGGCGTTCCGACCGTTGGGGCTGACGCATTTG
GTCAGTATTTCGGGTTTGCACGTTACGATGGTGGCGGTGATGTTTGCGTGGCTGGCGAAG
CGGCTGCTTGCCTGTTCCCCGCGCCTTCCTGCCCGGCCGCGCGTGTGGGTTTTGGCGGCG
GGGTGTGCAGGCGCGCTGTTTTACGCGCTGCTTGCCGGTTTTTCCGTGCCGACGCAGCGC
AGCGTTTTGATGTTGGCGGCGTTTGCGTGGGCTTGGCGCAGGGGAAGATTGTCGGCGTGG
GCGACGTGGTGGCAGGCGTTGGCGGCAGTGCTGCTGTTCGACCCTTTGGCGGTCTTGGGT
GTGGGGACTTGGCTGTCTTTCGGTTTGGTGGCGGCCCTGATATGGGCGTGTTCGGGGCGT
TTGCACGAAGGGAAACGGCAAACCGCCGTGCGCGGGCAGTGGGCGGCTTCGGTGTTGTCG
CTGGTTTTGCTCGGTTATCTGTTTGCTTCGCTGCCTTTAATCAGCCCTTTGGTCAATGCG
GTGGCGATTCCGTGGTTTTCTTGGGTATTGACGCCGCTGGCGTTGCTGGGTTCGGTCGTG
CCGTTTGCGCCTTTGCAACAGTTGGGGGCATTTTTGGCGGAATATACTTTGCGGTTTTTG
GTGTGGCTTGCCGATGTGTCGCCCGAGTTTGCCGTTGCCGCCGCACCTTTGCCGCTGTTG
GTGTTGGCGGTGTGTGCCGCTTTGCTGTTGCTGCTGCCGCGCGGCTTGGGTTTGCGTCCG
TGGGCGGTGTTGCTGTTGGCAGGGTTTGTGTTTTACCGTTCACCCGGCGTGCCGGAAAAT
GAGGTTGCGGTTACGGTTTGGGATGCGGGGCAGGGTTTGTCGGTGTCGGTTCAGACGGCA
AATCATCATCTTTTGTTTGACACTGGAACTGCATCGGCGGCACAGACGGGGATTGTGCCG
AGTTTGAATGCGGCGGGTGTCCGCCGTTTGGACAAGCTGGTTCTGTCGCATCACGACAGC
GACCACGACGGCGGTTTTCGGGCGGTGAGGAATATTCCCGCCGGCGGGATTTATGCCGGG
CAGCCGGAATTTTATGAGGGGGCGCGGCATTGTGCGGAACAGCGTTGGCAATGGGACGGC
GTAGATTTCGAGTTTTTGAGGCCGTCTGAACGCAAAAACATCGATGATAATGGGAAAAGT
TGTGTTTTGCGTGTTGTGGCGGGCGGTGCGGCACTGCTGGTAACGGGCGATTTGGATACG
AAGGGCGAGGAAAGCCTGGTCGGCAAGTATGGAGGCAACCTGTACAGCCAGGTGTTGGTG
TTGGGGCATCACGGCAGCAATACGTCCTCGTCGGGCGTGTTCCTCAATGCCGTTTCGCCC
GAATATGCCGTTGCTTCAAGCGGTTATGCCAACGCCTACAAACATCCGACCGAAGCGGTG
CAGAACCGTGTCCGCGCACACGGCATTAAACTGCTGCGTACCGATTTGTCGGGTGCGCTG
CAATTCGGCTTGGGACGCGGCGGCGTGAAGGCTCAACGTTTGAGAGGGTATAAATTCTAT
TGGCAGAAAAAACCGTTTGAGTGAGGTTTGAAACATAAAATGCCGTCTGAAACGGATTCA
GACGGCATTTTGGCGTTAACGCCGGTTCGTGCTGGCAAGGCATATCGTTTGATTTTCAGT
GTGCGTCAAAAACAGAAAGGGCGTTGCGGTTAACGGCAGGGAGAACCGTTTGAATGAACG
GAAAGGTTTGCGCCAGAAGGGGAAATGCCGTCTGAAAGGGCTTCAGACGGCATCCGGACA
TCGGTGCGGAATCAGTGCCAGTAACGCCACCAGGGCATATCGTCAGATCGCCACGGCTGC
TTTAAGAACGGGCTTTTCGGGAAGTTGGTTTCCAACACGCGGCGCGTATCGGCGGCAAGC
CGGGGCTTGTCCAACTTCTTGTAGGCAAGTTCCAATATGGCGAGCGATTCTTCGACATAG
CGTGTATTTTGATAGCTGCCGATAATTTTTTGGGCGCGGTTGGCGGCGGCGATATATGCG
CCGCGTTTCATGTAGTAACGCGCTACCGACATTTCATTGCCGCCCAAAGCATCGACCAGT
TTGACCATGCGTGCGGTCGCATCGGCGGCGTATTTGCTGTTCGGGAAGCGTTGGACGAGT
TCCGCAAAGGCCTGATACGCTTCGCGGTTGGCTTTCGGGTCGCGGTCGGACCAGTCTTGC
GAGGCCAGCTTGTTCAAGAAAGATTGATCTTCGTTGAACAGTACCAAACCGCGCAGGTAT
AGCGCGTAGTCCATATTCGGGTGTTGAGGGTGAAGGCGGCGGAAGCGGTCAATGGCGGCC
AGCGCCTTATCCTTCTCATCATCTTTATAGTAGGCGTATGCCGTATCCAGTTGGGATTGC
TGGGCATGGCGGCTGGTAGGGAAGCGCGATTCCAAGATTTCGTATAATTTGACAGCTCGC
GTATAATTGCTGCTGTTCAGCTCGTCCTGGGCTTCGGCATAGAGTTTTTCCACACTCCAG
TCTTGGGTAATCTGGGCATCTTTATCTACCGTACCTTGAGTGGCACAGGCACTCAGTGCC
AAACCTAATGAAACCGTTAAAAGAATTTTTTTCATGCAGAATACTTCCTTTGATAATGAA
TCCGATTATAGCGACGATTCAGACTTTGCGTCAGCTTCCGAAACTGAAAACCGTATCGGT
CTGACCGTTCCGCTCGAGCTTGCAGGCGGGCGGTTGGATGCGGTATTGGCGAAACTTCTG
CCCGACTACTCGCGCAGCCGCCTGACATCATGGATTAAAGAAGGCGCGGTTATTGTAAAC
GATAAACCTTCGCAACCCAAAGACAAAATGATAGGCGGCGAGCAAATTTGTGTAACCGTC
CGTCCGAGTGAGGAAAATCTGGCGTTTGTTCCAGAGCCTATGGCTTTGGATATTGTTTAC
GAAGACGATACCGTCATCGTCGTCAACAAACCGGCCGGACTGGTGGTGCATCCGGCGGCG
GGCAACTGGACGGGGACGCTGCTCAACGGCCTGTTGGCGCACTGTCCCGAATTGAGCCAA
GTACCGCGCGCGGGCATCGTACACCGTTTGGACAAGGAAACCAGCGGGCTGATGGTGGTT
GCCAAAACCCTGCCGGCGCAAAATTCCCTCGTGAGGCAGCTTCAAGAGCGCACGGTCAAA
CGCATCTACCGCGCCGTCGCCAACGGCATCGTCCCCTTCGACGGTAAAATCGAAACCCAA
ATCGGACGCGATCCGCACAACCGCCTGAAAATGGCAGTCGTCAAATTCGGCGGCAAACCA
GCCGTTACCCACGTCAAAGTGTTGGAACGCTATCTTACCCACAGCTATATCGAATGCTCG
CTCGAAACGGGCAGGACGCACCAAATCCGCGTCCATATGCGCGAGGCCAACCATCCGCTT
GCCGCCGACCCGGTTTACGGCAACCCGCGCCATCCGTGCGGCGACACGGTGAAAGAAGCC
GTTAAAAGTTTGGGTGCGCGTCAGGCGTTGCACGCCTACCGCTTGAGTTTCACCCATCCG
GAAAGCGGCGAAACCGTTTCGTTTGAAGCACCGATTCCAAACGACATATATCATTTGTTA
TCCGTCCTCCGTCTTGAAGCCGGTTTGGATTCGTCTTTGAGCAATGAAGAAGAATGGCAG
GACAAATTCGGCGCGGACGACGACGATGATTGGAACGAAGACGACTATGATGTCGAAGTG
GTTTATGTAAGGGAGTGAGGCGGCTTGAAAGGCGGGGCGAACGCAGGCAGCCGAATCGGA
GCAGCCGGGCAATCGTCCCCGCCGATTTCAAACAAAGGCCGTCTGAAGGGACCGGGCAGA
AACCGCCGGTTTTGTTTGCCCCGTTCAGACGGCATTATGATAAAAGGCGTTTAGGGTTTT
TTATGTTTACCGGCTTTGGCCGCCCAATAAGTTGCCAGCAGCGAGCCGGAGATATTGTGC
CACACGCTGAACAATGCGCCCGGAACGGCAACGACCGGCGCGGCGGCAAAGTGTGCGGCG
GCAAGCGCGGCGGCCAGGCCCGAGTTTTGCATACCGACTTCGATGGTCAGCGTTTTTTGT
GCATCATAAGGCAGGCCGGTCCATTTGGCGGCAAAGAAGCCGAGCAGGTAGCCGATGCCG
TTGTGGAGTACGACAACCGCAAAAATCAGCAGGCCGCTTTCCATAATCTTGCCTTTGCTT
GCCCCAACAACCGCGCCGATAATCAGCACGATGGCGGCAACGGAAACCAGCGGCAGCGCA
TCGGTCAGCTTTTCGGTTTTACTGCCCAAAACCTTATGGACAATCAAACCCAAAACAATG
GGGAGCAAAACCATTTTGACGATGGACATCAACATACCGGCCGCTTGGATTTCCAGCATT

## Appendix A

TCGCCGGCAAGCATCAGGAAGATGGCGGGAGTCAGCAATGGGGAAATCAGGGTGGAAACA
GACGTAACGGCAACCGACAAAGCCACATTGCCACGCGCCAGATAGGTCATCACATTGGAA
GCCGTACCGCCCGGGCAGCAGCCGACCAAAATCACGCCGACCGCGATTTCGGCAGGCAGG
TTCAACAGTTTGGACAGCAGCCAGGCGGTTGCCGGCATAATGGCGAATTGTGCGATTACG
CCGATGATGACGACTTTGGGATGTTTGAACAAAATATCGAAGTCGGAAGGTTTGAGCGTC
AAACCCATACCGAACATAATAATGCCCAACAGCCAAGGAATATAAGGCCCCGCCCATTTG
AAGGTGTCGGGCGCGAAAAAAGCGGCGGCGGCAAAGAGCGCGGCCCAGAGGGAAAATGTT
TTTCCGATAAAGCTGCTGATTTTACTGAGGATATTCATAAATAATGCGTTGCGTGTTTGT
TTTTAAGGGAAGGCAAGCATACACGCCTTAACCTTAATTTGCAAAATGACCGTGCCTAAA
CAATGCCGTCTGAAAGTGGAGATTGGTTTTCAGACGGCATCGCCCGAGAGATGTCGGAAA
TGGACTTTATCCCCATTCCTTTTCGGTTGAAACCCGTCTGTTTATGGCGATAGAATCTAA
TCGGAGGGTAGTCTCGTTCGGGCAACACGCAGTGCGGTGCTTGATGTGCCGTCCCCTGTT
GAAACATATAAAGCTCGGAGAAAGTATAGTGGATTAAATTTAAACCAGTACGGCGTTGCC
TCGCCTTGCCGTACTATTTGTACTGTCTGCGGCTTCGTCGCCTTGTCCTGATTTAAATTT
AATCCACTATATATAAGGGCATCATTCCTGCACCGGCAAGAATCCGAACCCGAACGTTTG
AAAACAATCCCGAATCTCCGAATTCCCGCCTGTGTGGAATGACGAAAAAACAAGCATTC
ATTTGCCCCGAAGGCAGTTAATCAACCCTTTCCGCCACACACCTATTCCAATATCCAATG
AAAACCATCACAGAAACCCTAAATCTCGCCCCGAAAGGCAAAAACTTCCTGACCGCCGAT
TGGCCCGCGCCCGCCAATGTGAAAACCCTGATTACCACGCGCAACGGCGGCGTGAGCCAA
GGTGCGTATCAGAGTTTGAACCTCGGTACGCACGTCGGCGACAATCCCGAAGCCGTGCGC
CGCAACCGCGAAATCGTGCAACAGCAGGTCGGACTGCCCGTTGCCTACCTCAATCAAATC
CACAGCACCGTCGTCGTCAATGCTGCCGAAGCGTTGGGAGGCACACCCGATGCGGACGCT
TCCGTAGACGACACGGGCAAGGTTGCCTGTGCCGTGATGACCGCAGACTGCCTGCCCGTT
CTATTTTGCGACAGGGCGGGTACGGCGGTTGCCGCCGCACACGCGGGCTGGCGCGGTTTG
GCGGGCGGCGTACTGCAAAACACCATAGCCGCAATGAAGGTTCCGCCCGTCGAAATGATG
GCGTATCTCGGCCCCGCCATCAGTGCGGATGCGTTTGAAGTCGGACAGGATGTGTTTGAT
GCGTTCTGCACGCCCATGCCCGAAGCCGCCACCGCATTTGAAGGCATAGGCAGCGGCAAA
TTCCTTGCCGACCTTTACGCGCTCGCCCGCCTGATTCTGAAGCGCGAAGGCGTGGGCGGC
GTATATGGCGGCACGCATTGTACGGTTTTGGAACGGGATACTTTCTTTTCCTACCGCCGC
GACGGAGCGACAGGGCGTATGGCGAGCCTGATTTGGCTGGACGGCAATGCCGTCTGAACA
CGCCGCTGATATAATCTACCGACTTTGTGTTTTTGAGAAAGGCAAGCCATGAACAAACTG
TTTCTTACTGCCGCAGTGCTGATGCTGGGCGCGTGCGGTTTCCACCTGAAAGGTGCAGAC
GGCATTTCTCCGCCGCTGACCTACCGGAGCTGGCACATCGAAGGCGGACAGGCATTGCGG
TTTCCTTTGGAAACCGCGCTGTATCAGGCTTCGGGCAGGGTGGACGATGCTGCCGGCGCG
CAGATGACCCTGCGTATAGACAGCGTTTCCCAAAACAAGGAAACCTACACCGTTACCCGT
GCGGCAGTCATCAACGAATATCTTTTGATATTGACGGTTGAAGCGCAGGTATTGAAACGC
GGCGAGCCGGTCGGTAAACCGATGACCGTGTCCGTCCGCCGCGTCCTTGCTTATGCCGAC
AACGAGATCTTGGGCAAACAGGAAGAGGAAGCGGCATTGTGGGCGGAAATGCGGCAGGAT
GCCGCCGAACAGATTGTCCGCCGCCTGACCTTTCTGAAGGCGGAATGACGTGGCGGCACA
TATCGGACGCATTGATACGGACGCGCCTTTGAAACCCCTGTACGTCATCCACGGCGAGGA
AGAACTGTTGCGTATCGAGGCATTGGACGCATTGAGGGCGGCGGCGAAGAAACAAGGTTA
CCTTAATCGGGAAGTTTATACGGCAGACAATGCCTTCGATTGGAACGAGCTGCTGCAAAC
CGCAGGCAGTGCGGGTCTGTTTGCCGATTTGAAGCTGTTGGAACTGCATATCCCTAACGG
CAAGCCCGGCAAAACCGGCGGCGAGGCGTTGCAGGATTTTGCCGCCCGATTGCCGGAAGA
TACGGTAACGCTGGTTTTGCTGCCCAAACTGGAGAAAACCCAGCTCCAGTCCAAATGGTT
TGCCGCATTGGCGGCAAAGGGGGAAGTGTGGGAAGCCAAACCGGTCGGCGCGGCGGCTTT
GCCCCAATGGATACGCGGACGGCTGGACAAAATCGGTTTGGGTATCGAGGCAGACGCATT
GGCACTGTTTGCTGAGCGCGTGGAAGGCAATCTGTTGGCGGCGCGTCAGGAAATCGACAA
GGTGGGGCTGCTGTATCCGAAAGGGCATACCGTCAATATCGATGAGGCGCAAACCGCCGT
TGCCAACGTCGCCCGCTTCGACGCGTTCCAACTGGCAGGCGCGTGGATGAAGGGCGATGT
CCTGCGCGTATGCAGGCTTTTGGACGGATTGCGGGAAGAGGGCGAAGAACCGGTGCTGTT
GCTGTGGGCGGTTGCCGAAGACGTGCGGACGCTGATCCGGCTTGCTGCCGCCCTGAAGCA
GGGGCAGAGCATCCAATCCGTCCGCAACAGCCTCAGGCTTTGGGGCGACAAGCAGACGCT
CGCACCGCTTGCGGTCAAGCGGATTTCCGTCGTCCGCCTGCTTGACGCGCTCAAAACCTG
CGCCCAAATCGACCGAATCATCAAAGGTGCGGAAGACGGCGACGCATGGACGGTATTCAA
ACGGCTTGTCGTGTCGCTGGCGGAATAAAGCGGTAATCCCCAAAATCCGAAAATACTGTA
AAATACCGTTAATCCTGAAAAGTATTCACCAATCCGTCCGAAAACATTTCAGACGGCACG
ACCACCTCAATAAAGGAACATTAACCCTATGGACAATAAGACCAAACTGCGCTTGGGCGG
CCTGATTTTACTGACCACCGCCGTTTTAAGCCTCATTATCGTATTGATTGTCGATTCCTG
GCCGCTTGCCATCCTGCTTGCAGCCGTCATTGTCGCTGCCGCTGCGGGCGGTTTTGTTTG
GACATCCCGCCGACAGCAACGCCAGTTTATCGAACGCCTGAAAAAATTCGACATCGATCC
CGAAAAAGGCAGAATCAACGAGGCAAACCTGCGCCGTATGTACCACAGCGGCGGACAACA
CCAGAAAGATGCGATTACCCTGATCTGCCTGTCGCAAAAATGTTCGGTGGACGAGGCGCA
CGCTATGTTCAAAAAACGCCCGACACGTCAGGAAATCAATCAAATGGCGGCAAAACAGTC
GCGCGGTCAGAAACGTCCGCACCGTTAACCGCCGCAAGGCATCTTTGCATAAATGCCGTC
TGAAGCCTGTTGGCGTTTCAGACGGCATATTCTGATTGAAAAGATGATGACACTGAAAAC
CGCCCCGCTCAAACGCCGCTTTGCCGCCATGCTGTACGAAATGCTGCTGGTCGGTGCGGC
AACCTGTTTGGCAGCATTGATTGCCGGTATTGCCGCCATTTTTCTGAATCCCGTTTCTAT
CGCGGTTTCTGCATTGGTAACAAGTATCCTGATAATGGGAGCATGGTGGCTTTATTTCCG
CGCCAACTGGCATGGTCAGGGGCAGACCTTGGCGATGAGGACATGGAAAATCGGCTTGTG
CGACCTTAACGGCATACAGCCGTCTTTGCACCTGCTGCGCCTGCGCTTTATTTGGGCGTG
CATATTTATCGTATTTATCCCTATGTTAGCCTATGCCGGATTACGCCACTTCCTCGGCAT
TCCGCCCAAGGGCGCGGCCGGCGCGGCATTGATTTGGCTGATTTTACCGTGGGGTTCGC
ACTGCTGAATCCCGATCGGCAGTTTCTGTATGATTTCTTGCAGGAACAAGATTGGTGGC
GGTCAAAGGAAAGCCTTAAGCCTTTATACCGCAAAGGTTTCAACCTGAAAAAATGCCGTC

## Appendix A

TGAAAGGGCTTTCAGACGGAATTTGCTTATCGGGGAAACCGATTATTCGATATTCTGCAC
TTGTTCCCGCATCTGCTCGATTAAGACTTTCAGTTCGACCGAGGCTTGGGTGCATTCGGC
GGCAATGGATTTGCTGCCCAAAGTGTTGGCTTCGCGGTTTAATTCCTGCATCAGGAAGTC
CAGCCGTTTGCCGCTGCTGCCTTTGTGTTCGGTAACGATACGGCGCACTTCGGCAATGTG
GGTGCGTAGCGGCTGAACTCTTCGTCGATGTCGGATTTTTGGATAAAGAGGGCAAATTCC
TGTTGCAGGCGGTCGTTGTCGATGCTGCCGACCGCTTCGACGAGGCGGGCGCGGATTTTT
TCTTTATGTGTTTCCAACAGGGTAGGAAAGAGTTCGCTTAATGCATCTATGATTTCTTCC
ATAGCCTCAAGGCGTTGCAGCAGGTGCTCGCCTAATTTCTTACCTTCCCGCTTGCGTGCG
GCAGTAAAGTCTTTTAACGCTTTTTCGGTCAGTTCGGTAATGCTTTTTGCCAATTCTTCC
GTATTTTCCCTTTGGCTTGCCAATACGCCGGGGAAACGCAGGATGTCGGCAACGCCCAGT
TTTGCCAAATCGTGATGCTTGCGGAGGTCTTTGTTGATTTCGGCAAGCTGTCCGACCAAG
TCGCGATTCAGTTCCAAGGACTGACTGCCGTTTTCCGCATCTTGAATTTGGATTTTGCAT
TCGACTTTGCCGCGTGCGATATGGGATGAAATTTTCTCGCGGATACCGCTTTCCAAATAG
CGCAAATCGTCGGGCATCCTGATTTGAATATCTAGAAAACGGTGGTTGACCGCACGCAGG
TCGAGATTGATGCGTTTGCTGCCGCACTCTGCCGCCGCGTTGGCAAATCCGGTCATGCTG
TGGATGTGGATATTTCCGCTGCTCATGTCGTTCTCCGAAGCCCGTTAAAATGGAATCAAT
ATATCACATCTGTATGGCGGCAAGCGTTTTCGGGTGTGAAAAATTGAAGATTTTGCAGCG
GCAGATTGGAATCACGCGCTTTTGTTGCTGCAAGGAAGGGAAATGTATAGTGGATTAACC
AAAACCAGTACGGCGTTGCCTCGCCTTAGCTCAAAGAGAACGATTCTCTAAGGTGCTGAA
GCACCAAGTGAATCGGTTCCGTACGATTTGTACTGTCTGCGGCTTCGCCGCCTTGTCCTG
ATTTTTGTTAATCCACTATATCAATTCCGCCAATCTGTCGGAAAAGCAGCTGATGCGGCA
GTGTCTGGTGCATGTCTGCTTTTTGATTTCGGCAATTGCAACGGCGTGGACGGATAAAAT
CGTGTACAGCACGACGCACAAACCGCATTGATGTTTACCAAATAAAATACCCGACAAAAC
AATTTGTCGGGTATTTTATTGCGTATATTTCAAACCGCTTCGGCTTCTTCGGTCAGGAAA
CCACGCAGTTTCTGCATGGCTTTTGCTTCGATTTGGCGGATGCGTTCGGCAGATACGCCG
TATTCGGCGGCAAGCTGGTGCAGCGTCAGCCCGCCGTCGTCTTGAAGCCAGCGGCTTTCC
ACAATACGGCGGCTCCTGTCATCCAGTTGCGCCAAAGCGTTTTGTAAACCTTCTGTTTGC
AGGGCGTAATGCGCCTGTTTCGATAGTTGTCGGCTCGGTTCGGAATCGTGGTCGGCAAGC
CAGTCGATGGGGGCGAAACTATCCTCGTCGTCGCTGTTGTCTGCCATGATGGCGATGTCG
TGTCCCGTCATTCGCTGTTCCATTTCCAGAACTTCGGAAAGTTTGACACCCAAATCGTCG
GCGATGTCTTGTGCCTCTTTGGGAGACAGGGCGTTGAGGTTTTTACGCATGCTGCGCAGG
TTGAAAAACAGCTTGCGTTGCGGTTTGGTGGTGGCAACGCGAACCAAACGCCAGTTTCTC
AAAATAAACTCGTGGATTTCGGCTTTAATCCAGTGTACGGCAAATGAAAACAGACGCGCG
CCTCTACCGGGCTCGTAGCGTTTGACCGCCTTCATCAGTCCGATATTGCCTTCCTGAATC
AGGTCTGCCTGATTCAGCCCGTAGCCGTCATAGCCGCGCGCGATGGAAACGACGACGCGC
AGGTGGGACAGGATGAGTTGTTTGGCGGCGTTGAGGTCGCCTTTGTGTTGGCGTTCGGCA
AGGCGTGTTTCTTCCTCTTGGGTCAGCATGGGAATTCTGTTGACGGTGTGGATGTATTGT
TCGAGGCTGCCGTTGCCGCTTTGGATGGCGGGTAATGCGAAAGCGTTATTCATTTGGGAC
ATTTCCTTTCGGCTGAAACTGCGTATCGGCGGTTTGCTGTGTTGGGATGCAGTATATCAC
TGCTTGGCTTGTATTTTGTATATTTGGCAGGAGATATGCGCTAAGGTTTGAAAGACAGGA
AAAATTTTGTAAGGCAAGTTTGATTGATTTTGTAAACCTGATGGCTCAATTCGATTTTGG
AATTATATTACATACGTGGTTGTATGTAAATAGCCGTTTTGAAAAAAGACAGCCCGTCCG
GACGGGCTGTGCAGGTATCAGTGTTCTTTGTTTCGGAAGATGAAAAGAATCAGTGCGGCT
AGGGCCAATATGCCCATCAACCACCATGAACTGCCGGTTTTCATATAGGGCGTTTCGCCG
ACATAGCCTTTGATGTGTCCTTCCAATACGGTTTCCGTATCGGGTTGGGCTTGGGCGATG
ATGTTGCCTTTGGGGGAGATGATGGCGGTTGCGCCGGTGTTGGTGGCGCGGACCATATAG
CGTCCGAGTTCCATAGCCCGCGCCTGCGATTGTTGGAGGTGCTGGTACATGGCGTTGGAT
TTTCCGTACCACGCCATATTGCTGGCATTGGCAAGCAGGGTGGCATCTTTTGCGGCGGCA
ATCAGTTCGTCGCCGAATCCGTCTTCGTAACAGATGTTGAAGGCGATTTTTTGGTTTTTC
ATCAGCAGGGCGGATTGCTTGCCGCCGCCTTTGCGGAAGTCGGAAAGGGGCATATCCATC
ATTTTGTAAAGCGGCGTGGTCAGGAAAGGCAGCGGTTTGTATTCGCCGAAGGGGACGAGG
TGGTTTTTGGCGTAGTAGGGGATACCGTCCTGATTGTTTTCCTGATAACCGGTCAGGTTG
ATGACGGCGTTTTCGTAACCGTTGCCGTCCGAAGTGTATTGGCTGATGCCGACGGCGAGC
GCGCTGCCGTTGTTTTGCGCCTGTTCGGCAAATTTCGCCAGTATGTTTTCCGGCAGGTTT
TGGCGCATAACGGGGATGGCGGTTTCGGGCAGGATGACGATGTCGGCGGTGGTTTTGCCG
ACTTGTTCGTAATATTTCTGTATGGTCGGGATAACTTGGTCTTCACGCCATTTGAGGGTT
TGGTCGATGTTGCCTTGAAGCAGGGCGACGGTGCTGCGGCTGCCGTCGGGGCGGGTGAAG
TCGGTTTGTCGGGCGGTGTAGCCTGCGGCAAGCAGGGCGGCAATCAGGATAATCGGAAGC
AGGCGTTTGCCCGAACGTGCGGTGTTATTACTCGCCAAAACCAGCCAGACACCGAGAAAG
GCGGTTGCCAGTGTAACCATGTGGATGCCGCCCAATGGGGCAAAGCCGGCGAGCGGGCTG
TCCGGGGTGATTTGGGAGTAGCCGATTGCGCCCCAGCCGAATCCGGTCAGGAAACGTTCG
CGGGCAAACTCGGTCAGCGTCCACAGGATGGGCAGTACCAAACCGATTTTTATGCCCCGA
GGCAGGGTAAATTTTTTCCACAGCCAGAAACACAGTGCCGGATAAAGGGCAAGGTAGGCG
GGGAGTAGGAAGGTCAGCGGTACGGCATAGAGGTCGGGCAGGCCGGAAACGTCGTGCAGG
GCGGTGTGTATCCAGTAGAACTGTGTCGTGTATGCGGTCAGGCCGAACAGGTAGGCGGAA
GAGACAGCAAAACGCGGACGCAGTTCGATGAGGCGGACGAAGGCACCGAAAATCAAGGGC
ATCAGCCAAAAGTGGTAGTAGGGTGCGAAGGTAAAGGGGGTGGCGGCGGCAAAAAGGATG
AGCAAAGGCCAGTAGAGGGCGGGGTGCTGCCAGTATTTGTCCAGTTTGGAAACCATATTC
ATCTGTCTGTTCGGAAGATACCGTCTGAACATCTTTCAAACGGCATCGGTATTTGAAAAA
GGAATCAATGCCTGCCGAAACGATTCATCAGCGGCAAGGCGGGGGCGCAGGCAATCGAAC
CGGAAAGCAGCCCGACAACGAGGTTGGCGAAGTACTCCGCCCAGCCAGCGTCCCAGTGTT
GCGCGTGCAGGAAATCGTGCAGAAGGCCGAGGTTGTGGGCGACCAGTACGCCGCCGATAA
GAAACATGGCAAGCGTGCCGACCACGCTCAAACCGCGCATAAAGCAAGGCATAAAGGCAG
TCAGCATTTGCCCCAAACTGCGCGAAAAGGTTTGTGGGCGGCGCATCAGCAGCATGCCTA
AGTCGTCGAGTTTGACGATGACGGCAACGATTCCGTACACCAAAACAGTCATGCCGATGC

## Appendix A

CGATTGCCGCCATTACGAGCATGCGCGTCATGTCGGTGCAGAAACTTGTGCAGCAGCTTT
TCTACGCCTTCAAAGCACAGATAAATGCCGCCTGCCGTCAAAAGCGGCGTAATGAGTTGC
GGCAGGAAGGCGGAAAGCAGCAGGGCCGCAGGCACCAAAACCGGCTTGTTGGAAAAAGAA
CCTTTCGCCATCGACCAAACAATCGGCAACTCGCGTTCTGCCGATACGCCCGTAACCCGG
TTGGCATTGGGTGCCAAATCGTCGCCGACCACGCCGGCGGTTTTCTTTGCGGCGGCTTTG
GTCATCAGGGCAACATCGTCCAAAACGGCGGTGATGTCGTCCGGCAGGGTAAATAGTGAG
GCAAATGCCATTAAAGAATCCTGAAATGCGGCGCAAAGTCCGACATTATATAGGAGAACG
CGGATTTGGGCGGTTTCAGGCGGCATGAAACAGGAAAATGCCGTCTGAACGCTGTGGCGG
ACGTGAAGTAAAGTTTCGTGAAAAGAAAATACCGTGTTACAGTCTTTCGATTTTAATTTC
ATGAATTTTAAGGGAGAATCGTTAGCGTGGATTGGATGGGCAGTCTGTTCCTGCCGGGTG
GCGCACTGTTGTTTCTGAGCGTGGTTTCGACCACTTTGTCCGCACGTTTGGGAATGCCTT
TGCTGCTGGTTTCTCCTGCCAACGTGTTGGACAGGGCGGCGGAAGCCTTGGCGATTGCGG
CGTTCCTGATGCTGGTCGCGCGTCCGTCGGCAGTGTTCGGCGGTTTGTGGAAATTCAATT
ACAGCCTGCGTGAAAAGGCGTATAGCCGAATAGAAATGCAGTCCGACACCGTGCTTCAGG
CGGGGATTTGGCGTGGTACATCCTGCCCGACGGCAAGGTCGATATAGTGAATTAACAAAA
ATCAGGACAAGGCGGCGAGCCGCAGACAGTACAAATAGTACGGAACCGATTCACTTGGTG
CTTCAGCACCTTAGAGAATCGTTCTCTTTGAGCTAAGGCGAGGCAACGCCGTACTGGTTT
AAATTTAGTTCACTATAAAATGGCGAAATACTTTACCGAGACGGGTATTAGCGTCCGTGA
GCATTTTGATTTCTTCGGTGAGTTTGTCGTTTCGCCGGCAGCACGTTCGGGTGATTTGGC
ACTTACTTACGGTTTGAGGCTGGAAGCGGGCGAAGAGGGTTTGAGCCTTGCCGAGCTTTT
CGATAAGCGTTCCGATAGTCAGGAGCCGGTCGAGGGCGGCCGTATTGACATCGGCGGCTT
TATGCTGACCGCAAAGGAGGTTGACGGTGGCGGCAATATCGGGTCTATGGGGCTGAAAGT
GCTGCGTTAGAAAGGTTTGATTTGAATGCCGTCTGAAGCCGGATTGCCGGTTTCAGACGG
CATTTTGTCTGTTTAGTTTTTTTTGCTTTTTGCCTGTTTTACGTCTTTTTCGGTAACGCT
TCCGCCGCCGTTGTCAAAGGCGTTCATGATATAAGTGGCGACGGCGGCAATGTCCGCATC
GCTGATGGCGGTTGCGGGCATGAATCCGTTGTAGGTTTTGCCGTTGACTTTGATTGTACC
GTTGATGCCTTTGACCATGCTGTGCAGCAGCACCTGCGGTTTTTTCATGATGAAGTCGGA
GCGGTAGAGCGGCGGAAACATGGTTCCGCGGCCTTCGCCCTTTTTGCCGTGGCAGGCGAC
GCAGTTGGATTCGTACACTTTTTGCCCTTTTGTCATGATGCTGTTGTCGGCGGCAGAAGC
GGCGGCGCAGAAGCAGCCCAAGACGAGGGCGGTCGGCAGTCGGGTTGTGTTCATTGGTGT
TTCCTTCATGTTTGAAACCTTGTTGTTGATTTTGCGTAGCGGGTGAAAGATTTTTTTGCC
GAATCAGTAGTATAGTGGATTAACAAAAATCAGGATAAGGCGACGAAGCCGCAGACAGTA
CAAATAGTACGGCAAGGCGAGGCAACGCCGTACTGGTTTAAATTTAATCCACTATAAGGT
TGCACTTGATGTTGTTGTCCAGCATAGATGCCATCATACGCTAAAGTAGCGGGAAAATGC
CGTCTGAACACGGCGTTCAGACGGCATTTTAGACATGGGTCAAACAGTTTCAACGCCAGC
TGCCAAGGTTTTCTTCGGCAAGTGCGACGAGTGCATCTATCCAGTCGGGGTTGTCGTTGA
GGCAGGGGATGTAGCGGTAGCTTTTGCCGCCTGCTTCATAAAACTGTTCCCGCCCCATCA
GGGCGATTTCTTCCATGGTTTCCAAACAGTCTGCCAAAAAGCCCGGGCAAAATACGTCCA
GCTCGGTTACCCCCTGTTTGGGCAGTTTGCCGAACAAATCCTGCGTGCTCGGTGTAACCC
ATTTTGCCCTGCCGAATTGGCTTTGGAACGATACGACATATTGGTCTTCGGTCAGTTCCA
GTGCTTCGGCAAGCAGTTTGGCGGTGTGGCGGCACTCGTCGGGATAGGGGTCGCCGAGGT
CGTGGTGCTTCTGCGGTACGCCGTGAAAACTCAACATCAGTTTTTTCCCGCGCCCGTGTT
CCGCCCAATATCGGAGGATGTGGTTTTTCATCGCATCAATGTAGCCGGTATCGTCATAAA
AGCGCGAAACGGTGCGGACGCTCATTTGGTTCCGTTGCAGCAGTAATTGTTCGCACACCT
TATCTACTGCCGCTCCGCTGCTGGAAGCGGCATATTGCGGGTACATCGGGATGACCAGCA
GTCTGCCCGCGCCTTGCGCCTTCAGTTCCGACAATACGTCTGCCACCGAAGGATTGCCGT
AGGTCATGGCGTGGCGGACGATGAGGTCGGGCATACGTTTGGCAAGCGCGGCAGCTTGGC
GTGCTGTGTAAACTTCTAAGGGCGAACCTTCCTTAAACCAGATTTTTTCATAGGCGTGCG
CGCTTTTTTTGGGGCGGAGCGTCAGTACCAGACCATGCAGAATGGGATACCACAGCCATT
TGGGCAGTTCGACGACGCGCCGGTCGGTCAGAAAGGACTTCAGATAAGGTCGTACCGCCT
GCGCGGTCGGCGCGTCGGGCGTGCCGAGGTTCAACAGCAAAACGGCGGTACGGTTTTGTT
GCGTATAGGAAAGGGAGGGTTCTGGAAAGAATGGAAGCATGATCGGTTTCTGAAAAATAG
TGCGGGTAGGGTAAAGCGGCAAAATGCCGTCTGAAGCGGCTTCAGACGGCATTGCAGGGA
ATCAGTCTGTGCCGCGTGCGCGGTTTTCGTGGAATCGCGCCTGCCAGTCGGCAAATTTGC
CTTGTTCGACGGCTTCGCGCATTTCCGCCATAATGACTTGGTAGAAATGCAGATTGTGGA
TGGTGTTCAACTGTGCGCCCAAGATTTCGCCGGTGCGGTGCAGATGGTGCAGGTAGGCGC
GGCTGAAGTTTTGGCAGGCGTAGCAGGTGCAGCTTTCGTCTATCGGACGCTTGTCGAGCT
TGTGTTTGGCGTTTTTGATTTTCAAATCGCCGAAACGGGTAAACAGCCAGCCGTTGCGTG
CATTGCGGGTGGGCATCACGCAGTCGAACATATCGATGCCGTGTGCCACGCCGTACACGA
GGTCTTCCGGCGTGCCTACGCCCATCAGGTAATGCGGCTTGTGTTCCGGCAGAATCGGAC
CGACGGCGCGCAGCATACGGTACATTTCGGGCTTGGGTTCGCCGACGGACAAACCGCCGA
CGGCAAGGCCGGGAAAATCAAACTGTTCCAAACCGCGCAGCGATTCTTCGCGCAAATCCT
CATACATCGCGCCTTGCACGATGCCGAACAGCGCGTTCGGGTTTTTCAAATCTTCAAAGG
CTTTTTTGCTCCGTTCCGCCCAGCGCAGGCTCATTTGCAGCGATTTTCGCGCCTGTTCGC
GCGTCGCCTCGCCCGGCGTGCATTCGTCCAACTGCATCGCGATATCCGAGTTCAAAACCG
TTTGGATTTTCATGGAAATTTCAGGCGATAAAAACAGCTTGTCGCCGTTAATCGGGCTTT
TGAACGTACAGCCTTCTTCCGTCAGCTTGCGCATATCCGACAAAGAAAAAACCTGAAAAC
CGCCCGAGTCGGTCAGAATCGGTTTGTCCCAGCCGATAAAACCGTGCAGGCCGCCGAATT
GCCCGATAACTTCCAAACCCGGACGCAGCCACAAATGATAAGTGTTGCCCAAAATAATTT
GTGCCTTGATATCGTGCAGGTTTTGCGGGTTCATCGCCTTAACCGAACCGTAAGTACCGA
CAGGCATAAATACCGGCGTTTCAATTTTGCCGTGGTTCAACTCCAGCGTGCCGCGTCGGG
CGAGACCGTCTTTTTTGTGTAAGGTAAATTTAAGCATAAGATTGAATGTCAGTTGGGCGA
CAGGGGTCGAAATATATTTTAAAAGACGGCATTATAAATGATTTCCCACGGTTTTTCAGA
CGACATCCCCAAATCTTGCCGCAATGTTGCATAAAGAAACGCACATACCTCTTGCAAAAA
TTAAAACGACCCGATAAAATGCAAAAATTCTTTGAAGGCACGTAGCTCAGTTGGTTAGAG

# Appendix A

CACCACCTTGACATGGTGGGGTCGTTGGTTCGAATCCAATCGTGCCTACCAAATTCCCA
TAACGGCATTTATGCCGTTATTTTTTAATCTTTCGGAGCGTTTGATGTTGAATATTACCT
TGCCGGACGGCTCAGTCCGCCAATACGAATCCCCCGTTACCGTGGCTCAAATTGCTGCGT
CTATCGGTGCCGGTTTGGCGAAGGCGACGGTGGCAGGCAGGGTAAACGGCAAATTGGTCG
ATGCGTGCGACCCGATTGTTGAAGATTCTGCTGTTCAAATCATTACTCCGAAAGATCAGG
AAGGCATCGAAATCATCCGCCATTCCTGCGCGCATCTTGTCGGGCATGCCGTCAAGCAAC
TCTATCCTAATGCAAAAATGGTTATCGGCCCCGTCATTGAAGAGGGCTTTTATTACGACA
TCGCCACGGAAAAACCGTTTACACCGGAAGATGTTGCCGCCATTGAAGCGCGTATGAAAG
AATTGATTGCCCAAGACTATGATGTGGTCAAAATCATGACTCCGCGTGCGGAGGCGATTA
AAATTTTTCAAGAGCGCGGCGAAGAATACAAACTGCGCCTGATTGACGATATGCCCGAAG
TGGAAGCGATGGGGATGTATCATCACCAGGAATATGTCGATATGTGCCGCGGCCCGCACG
TTCCGAATACCCGTTTCCTGAAAAACTTCAAACTGACCAAGTTGGCGGGCGCATACTGGC
GCGGCGACAGCAATAATGAAATGCTGCAACGCATATACGGTACGGCTTGGGCGACAAAAG
ACGAATTAAAAGCCTATATTCAACGTATCGAAGAAGCCGAAAAGCGCGACCACCGCAAAC
TTGGCAAGCAATTGGATCTGTTCCACCTGCAAGACGAAGCGCCGGGCATGGTGTTTTGGC
ATCCTAAAGGCTGGGCTTTGTGGCAAGTGATTGAACAGCATATGCGTAAAGAGCTGAACG
CCGCCGGTTATAAAGAGGTCAAAACGCCTCAAATCATGGATAAAACCTTTTGGGAAAAAT
CCGGCCATTGGGACAACTACAAAGATAATATGTTCGTAACCAGTTCGGAAAAACGCGAAT
ATGCGGTTAAACCGATGAACTGTCCGGGTCATGTTCAAATTTTTAACAACGGTTTGCGTT
CGTATCGAGATTTGCCGATGCGTTTGGCGGAATTCGGTTCTTGCCACCGCAATGAGCCGA
GCGGTGCGCTGCACGGTCTGATGCGGGTTCGCGGTTTTGTGCAGGATGATGCGCATATTT
TCTGTACCGAAGATCAAATCGTCAGCGAGGCTCGTGCGTTCAATGAATTGTTGATTCGCA
TCTACAAACAGTTCGGTTTCCATGATGTATCCGTCAAGCTTTCTCTTCGCCCTGAAAAAC
GCGCAGGTTCAGATGACGTGTGGGATAAGGCAGAGCAGGGTTTGCGCGAGGCATTGACTG
CCTGCGGCGTGGAATGGGGCGAATTGCCGGGCGAGGGTGCGTTTTACGGGCCTAAAATCG
AATATCATGTCAGAGATGCCTTGGGTCGTTCTTGGCAATGCGGTACATTACAACTGGATT
TCGTATTGCCGGAGCGTTTGAATGCCGAATATGTAACTGAAAACAACGACCGTGCGCGTC
CTGTTATGTTGCATCGCGCCATTTTAGGTTCTTTGGAGCGGTTTATCGGCATTCTGATTG
AGAACCATGCAGGCTCATTCCCGTTATGGTTGGCTCCGGTTCAATTGGTAATTATGAATA
TTACCGAAAATCAGGCAGATTATTGTCGGGAAGTGGCTGCCAAATTGCAGGCGGCAGGAT
TCCGCGCCGAGTTGGATTTGCGTAACGAAAAAATCGGTTACAAAATCCGCGACAACAGCC
AATACCGTTTCCCTTATCAAATCGTTGTCGGCGATAAGGAGAAGCAGGAAAACAAAGTGG
CGGTACGCCGCAAAGCAGAAGATTTGGGTTCTTTGGATTTGGATGATTTCATTGCGCAAT
TGCAGCAAGAAATCACTGATGCCCTCGTCAATCATTAATTTTTATAGGAGTATTCATCAT
CGCTCAAGAACGCGAAGCACGAATCAACGGCGAAATTACCGCCAAAGAAGTGCGTTTAAT
CAGTGAGTCAGGCGAACAGCTTGGTGTCGTTTCAGTTCGTGAAGCTTTGGCTATGGCCGA
AGGGCAGGATGTCGATTTGGTAGAGATTTCCCCAACTGCTAAACCGCCTGTGTGCAAACT
GATGGATTACGGTAAATACAAATACCAGCAGGCCAAGAAACGCGACGAAGCCAAGAAAAA
CCAAAAGCAGGTGCAAATCAAGGAAATCAAATTCCGTCCGGGTACGGATGAGGGCGATTA
TCAAATCAAGATGCGCAACATTAACCGCTTCCTTGCCGACGGCGATAAAGTCAAAGTGAC
ATTGCGTTTCCGCGGCCGTGAAATGGCTCACCAGCAACTCGGCGCGCAACTTTTGGAACG
TGTAAAAGAAGATTTGGCTGAAGTGGCGCAAATCGAGTCCTTTCCCAAAATGGAAGGTCG
CCAAATGGTGATGATGATTGCGCCGAAGAAAAAATAAAGCTATAATTCTCCGCTTACTCC
GATTGCCGCTTCGGAGTAAGTTTTCAATTGCGGCAAAAAACCGTGTCATTGTGGGTTCAA
GTGTTTGAAACCGATGTTTTAAAACCCCCTAATGCCTTATCCGATAACGAATGGAGTTTT
CCCATGCCTAAAATGAAAACCAAGTCTAGCGCGAAAAAACGCTTTAAAGTACTGGGTAAC
GGCGGTGTGAAACGCGCTCATGCGTTCAAACGCCACATCTTGACTAAAAAGACCACCAAA
AACAAACGCCAACTGCGCGGTACCTCTATGGTAAATGATCGCGATTTGGCTTCTGTTGCT
AAAATGTTACCCTACGCTTAAGGAGTTTAGAATATGCCACGCGTAAAACGCGGTGTTACC
GCTCGTGCCCGTCACCAAAAAATCTTCGCGTTAGCCAAAGGCTACCGCGGCCGTCGTAAA
AACGTTTACCGCGTTGCCAAGCAGGCGGTAATGAAAGCCGGTCAATACGCGTACCGTGAC
CGCCGCCAACGCAAACGCCAATTCCGTCAATTGTGGATTGTCCGTATCAATGCAGGTACG
CGTGAAAACGGGTTGTCTTACAGCAAATTTATGAACGGTCTGAAACGCGCCTCTATTGAA
ATCGACCGCAAAGTATTGGCTGATTTGGCCGTGTTCGATAAAGCCGCTTTTGCACAATTG
GTTGAAAAAGCCAAAGCTGCTTTGGCTGCTTAATCCAAAAAATTGAAAAGGAAGCTGCGG
CTTCCTTTTTTCTTTGTTTGCAGAAATTCTATGTGATTGATTTTCTTTCTTTAAAGTCTA
TTTTTTTAAATAAATTTGCGTTAAAATATAGTGGATTAAATTTAAATCAGGACAAGGCGA
CGAAGCCGCAGACAGTACAGATAGTACGGCAAGGCGAGGCAACGCCGTACTGGTTTAAAT
TTAATCCACTATACAGAAAATTTATCCAATGGATTGACCGTGAAGAAAATAAGGTCGTCT
GAAGAGTCTGATATGTCAGGCTATACAGGCGGCCTCGTTGTTTCAGGTGGTATATCATTA
ATTGACAGACTTGATATTATGGAAAATGTAAACCGCATCGTTGCAGAAGGCATTGCCGCA
GTAGAAGCTGCGCAAGACTTCAACGCTCTAGAACAAATCAAAGCCCGTTATCTTGGTAAA
ACCGGCGAGTTGACCGGACTTCTGAAAACTTTGGGGCAAATGTCGCCTGAAGAGCGCAAA
ACCATAGGTGCGCATATCAATGAATGCAAAAACCGGTTTCAGACGGCTTTTAATGCCAAA
CGCGAAGCCCTCAACGAAGTCAAGCTGCAAGCCCGACTTGCCGCCGAAGCCCTCGATATT
ACCCTGCCCGGACGCGCTCAGGAAGGCGGCAGCCTGCATCCCGTAACCCTGACCTTGCAA
CGTGTGGTCGAACTCTTTCACGGAATGGGTTTCGAAGTGGCGGACGGGCCTGAAATCGAA
GACGATTTTCACAATTTCCAAGCCCTGAACATCCCTGCAAACCATCCTGCCCGTGCGATG
CAGGATACGTTTTACGTTGAAAACGGCGATGTTTTGCGTACGCACACTTCCCCGATTCAA
ATCCGCTATATGCTCGATAAAAAAGAGCCGCCCATCCGCATTATCGCCCCCGGCCGCGTT
TACCGTGTGGACAGCGATGCCACGCACTCGCCTATGTTCCATCAGGCGGAAGGTTTGTGG
GTAGAAGAGGGCGTAACTTTTGCCGACTTAAAAGCAGTGTTCACGGATTTTATCCGTCGC
TTCTTTGAACGCGATGATTTGCAAGTACGTTTCCGCCCGTCTTTCTTCCCGTTCACCGAA
CCGTCTGCCGAAATCGACATTATGGGCGAAAACGGCAAATGGCTGGAAGTAGGCGGTTGC
GGTATGGTACATCCTAACGTGTTGAAAAACGTCAATATCGACCCTGAAAAATATACCGGT

## Appendix A

TTCGCCTTTGGTATTGGTCTCGACCGCTTCGCTATGCTGCGTTACAACGTGAACGACTTG
CGCCTGTTCTTCGATAATGATTTGAACTTTTTGAAGCAGTTTGCGAAATGATCGTGCAGA
CTGCCTGAATATGGAAAAGCAGCCTACTCTTGGTTTTCAGGCTGCTTAGGAAAATTCAAA
TGTAAGATATAAAACATTTGATATTTTGTTGTGAAATTACATTCCTAATTTTGTTTAAAG
AGGCATAATTTATTGCTTTGTAGAGATTATATAGTTAATTTGGGTTTGGTTCTATGATGA
TAGGGGCTTCTTTGTTTTCGAGTGCAGGGATTGCAGAAACCTACTTGCATAATGCGGGTA
TTAAGATTATAGCTGCAAATGAATTGGTGCCAGAACGTGCTAATTTATATAAAGCTCTAT
ATCCCGAAAGTAAAATGATTATAGGTGATATACTTCATGAGGAAGTGTTTCAAAATTTAA
TACAGAGCGTGCCGAATCGATTAGATTTTTTAATTGCTTCTCCTCCTTGTCAAGGCATGA
GTGTTGCAGGGAAAAATCGTAACATTCAAGAGATGGCTAATGATAAACGTAATCATTTAA
TATTCAGAGTAATCGAATTTATTTTATTAAAACGCCCAACTTTTGTTTTGATTGAAAATG
TTCCATTTTTTTTAAAAATTAAGTTACCTTATAAGGGGACATTACAAACAGTAGAAGTAA
TTTTGCAAGATTTATTTGGTTGCGAATATTATATTCAAACTCATATTTTTGATTCTGCCG
ATTATGGTGTTGCACAACATCGTAAACGAGCTATTATTCGTATGAATAAACATTCAACTA
TTTGGGGAATGCCGGAAAAAGTTACAAAAACCATTTCTGTTCGTGATGCTATTAGTTTTT
TGCCTAGTATTGAGTCTGGACAAAAGTCTAATGTGAAATGGCATTTTGCACGTACACATG
CTCCGGAGCACATTATATGGCTAAAAAATACGCCAACAGGACGATCTGCTTTTGATAATA
TAGAACATTATCCAAAGAAAAAAAATGGTGAAAAAATTAAAAGTTATAATACAACTTATC
GCCGTATGGAGTGGGATGCTCCTGCCCCAACTATTACTATTCGTAATGACGCTATCAGTT
CACAATTAAATGTTCATCCTGGACGGTCTATGCCTGATGGAACATATTCAGATGCAAGAG
TTTTAACACCATTAGAGTTAATGCTTTTAACATCATTACCTATTGATTGGAATATTCCCG
ACGATACATCAGAATTATTAATTCGGCAATGTATTGGTGAATCTATTCCTCCATTGTTAA
TTAAAAAAATTGTAGAGAGAATAGGAAAATAGATATGACAACTGCGCGCTGGGTAATAGA
TAAACATTTACAGAATTTTCATATTTTATGTAAATTTGCAGGTATTTTGAAAACAAATTC
TTTTATATCTGTAGAGGATAAAGCTAAGTTATCTGAAAAATTGGAAAAACTAGATTTATA
CCATAGACGAAATACAGGTAAATCATTGGATGCTACTACTCATAAAATAAAAGAATTATC
ATTCTATATGTTTGGTTATCGTGATGTGTGTGGGCAAGTTACACAGAAATTCCTGTTCAG
TCCATTGGGTAATTTATTTTTGAAACACTTGGATAATAATGAATATATTCAAAAAATTTT
TCTTACTATGTTGTGGGCGATACCATTTCCTCATCCGTACATTAAGACTGATGAAAGTAT
TCAATTATATCCCATGAGACTAATATTTAAGTTGTTATCTGATGAAAGATTGGATTGTAA
ACTATTTTCTTATGAATATATCTATTTAATTTCATTTGTGAAATCTGCTGATCAGAATAG
CTATGAAAAATTAGTACAAGACATTTTGGTGTTACGAACATGTGCTGAAGTAAAAATTAA
ACATCAATTAACTGCGGAAAATAGTCGTAGTCATGCTTATGTAAATGCAGCACATGAGTG
GGAATCTTATTTTTCAAAAACATTGACTGATGCAGGTGTTTTGCAAAAAACAGATGGTAA
AATTATTTGCCGTCTAAAGCATGGTAAGACCGAAACATATCGTAAAGTAACATCAAGTGA
GTTTTCGATTCCTAAGCAACTTCAGGAATTTGTGAAAAAATTGCAAAGTGCTTATTCGTT
TTCAGAAATGCCATTAAATCTGAACGATAGTGATCGTTTGAAAATTGATGTCATTAAGGA
AATTTATAGCTTCTATCCAAAAGAGTTATTGGAGGAAATTGGTGAGCTTAAGGATGAAGC
AGCATATGAATTATTGCACTTACCTAGGTTGATTGAACAATATGCAGATAATAATAATGG
AACAGAGGCATATCTATTTGAAGATGTTCTAGAAATGGGGTTCAATATGTTTTATAACGT
AGAAGCTAAAAAAATTGGTGGACCAGGTAATACGGATTTAGAGTGCTTATATATTACGCA
AAAGAGAAAATTTGCAGTGGAGGCAAAATCAACTAAAAATAAGTTATCAGGTATTAATTC
AGGAAGATTGGAAGATCATAAAAATAAAATTAAGGCCATTTACACAATTGTTGTCACACC
ACGTTATGTCCCTGCCGTATTATCCGATATTCGTAATTGCCCAATTGTAATTATTCGTGC
CAATACATTTGCTGAATTTTTATATAATTGTTTGATTAATCGCTCCAGTATTCCAGAGAT
TGATTATCGGTATTTTGATGAAATTATTATTAAAAATCTTGGAAAAGATATTAGTTCAGA
AATTTCCAATTTGACTATGCAACAGTTTGCAAGTAACACCACAATGGAAGCGTATAGTAC
ATGATAACTATTTCAAATGAAGATAACATGATCTTAATGTCTCGGTATCCTGACAAGTAT
TTTGATTTGGCAATTGTAGATCCTCCTTATGGGATTTTGAATAAAACTAAACGTGGTGGT
GATTTATAAATTCAATATGAATGAATACTCACAATGGGATATTAAGCCAGACCAAACTTAC
TTTAATGAATTATTTCGCGTGTCAAAAAATCAAATTATTTGGGGTGGGAATTATTTTGGC
GAGTTATGGTTGAGAAGTGAATATAATAAAGGATTTATTATTTGGGATAAGAATCAACCA
GAGACATTAAATAATTTTTCTATGGCGGAAATGGCTTGGTCGTCATTCGATAGGCCATCT
AAAATTTTCCGGTTTAGTGTGCGGAAAAATCGTAATAAAACTCACCCAACACAAAAACCA
GTCGAATTATATCAGTGGTTGTTAAAAATGTATGCAAAGCAGGGTGATAAGATTTTAGAT
ACACATTTAGGAAGTGGAACTCTTGCTATTGCATGCTGCATTGCACAGTTTGATTTGACA
GCTTGTGAAATCAATTCCGATTATTACCAACAATCGATTGAGAAAATAAAAAATAATTTA
CCTGAAGCTAGAATCAGTTTTGGGCATCCAGGTTATTGTATTATTGAATAACTTAAAAAT
ATAGAGAAATTAACCATGCAATTCTCCTACTCATGGCTGAAAACCCAAGCCGATACCGAA
CTTTCCTCCGATAAGCTGGAACATCTGTTAACGATGTCCGGCTTGGAAGTGGAAGAGGCT
GAAACTGCCGCGCCTGCGTTTGCGGGCGTGGTGATTGCCGAAGTGAAATCCGTTGAAAAA
CATCCGGATGCAGACCGTTTGAACGTTACCCGAGTTGATGCGGGTACGGGCGGGTTGGTG
CAGATTGTGTGCGGTGCGCCGAATGTGAAAGCGGGCATCAAAGTGCCGTGTTCGCTGCCG
GGTGCCGTTTTGCCGGGTAATTTCAAAATCAAGCCGACCAAAATGCGCGGCGAGGTGTCG
GACGGGATGTTGTGTTCCACCGACGAACTCGGTCTGCCCGACGACGGTGTGAACGGCCTG
CACATTCTGCCTGAAGATGCGCCCGTCGGTACCAATATCCGCGAATACTTGGATTTGGAC
GATACGCTGTTTACGTTGAAAATTACGCCTAACCGCGCCGACTGCTTGAGCATCAAAGGC
ATTGCGCGCGAAGTGTCCGCATTGACGGGGTGCGCGTTCAGGCAGCCCGAAATCCATACC
GCGCCGATCACGGGCAGTCGAAAACAGCCCGTGCAGATTAACGCGCCTGCCGATTGCGGC
CGTTTTATCAGCCGTGTGATTGAAAACGTGAACGCGCGCGCTACTACGCCGGATTGGATG
AAACAACGTTTGGAGCGCAGCGGCATCCGCAGTATTTCCGCGCTGGTGGACATCGGCAAT
TATGTGATGCTGGAAATCGGTCAGCCGATGCACGTTTTTGATGCCGACAAACTTTCCGGC
AGCCTGCACATCCGCCGCGCGCGCGAAGGGGAAACGCTGGAATGCCTGAACGAGAAAACC
GTTTCCCTGTCTGAAAACACGCTGGTCGTGGCGGACGAAAAAGGCGTGTTGAGTTTGGCG
GGCTTAATGGGCGGCGCGGCGAGCGCGGTTTCAGACGGCACGCAAAATATCGTGCTGGAA

## Appendix A

GCGGCTTGGTTTGCGCCCGAAATCATCGCCGGCAAATCGCGCCAATACGGTTTCGGTTCG
GATTCGTCGTTCCGCTTCGAGCGCGGCGTGGATTACCGTTTGCAGGCGGATGCCATTGAA
CGTGCTACCGAATTGGTGTTGCAGATTTGCGGTGGTGCGGCAGGCGAGATGGTGGAAGCG
CAAGGCGAATTGCCTGAAGCGAAGCAGGTTGGATTGCGTTTGGACCGTCTGAAAACCGTG
TTGGGCGTGGACATTCCTGCCGAACAGGTGGAAACCATTTTGCAACACTTGGGCCTGCAG
CCCGAGAAAACGGCGGAAGGCTTCCGCGTTACCGCGCCGAGCTTCCGTTTTGACATCGAA
ATTGAGGCTGATTTGATTGAAGAAATCGGACGCGTTTACGGCTATGAAAACATCCCCGAC
GATTACACGTCAGGCCGTCTGAAAATGCTGGAACTGCCCGAAACACGCCGCCCGCGTTTT
GCCGTTTACAACGAAATGGCGGCTCGCGGTTACCGCGAAGTGGTCAGCTATGCCTTCGTT
GACGAGCAGTGGGAACAAGATTTTGCCGCCAACGCCGACCCCATCCGCCTGCAAAACCCG
CTGGCGGCGCAGTATGCCGTGATGCGTTCCACGCTCATCGGCGGCTTGGTGGAAATTCTG
CAAAACAATCTGAACCGCAAACAAAACCGCGTGTGCGTGTTTGAAATCGCCCGCGTGTTC
AGCAAAGGTTCAGACGGCCAGTTTGTCCAAAACGAACGCATCGGCGGATTGTGGTACGGC
GCGGTCATGCCGGAACAATGGGGCGGGAAAACGCGCAATGCGGATTTTTACGACATCAAG
GCGGACGTGGAAAATCTGTTGAAAAACAAAGCAGTCGAGTTCGTTAAAACCGGACATCCC
GCCCTGCATCCCGGACGTGCCGCCAATATCGTTTCAGACGGCAAAGTCATCGGCTTTGTC
GGCGAACTGCATCCGAAATGGCTGCAAAAATACGACCTGCCGCAAGCGCCGCTGGTATTT
GAAATCGATATGGCGGCCGTGTTGGAATGCGGGAAAACGCGCTATCGGGTCGTATCGAAA
TTCCAGCCGGTGCGCCGCGATTTGGCGTTTGTGATGCCGGAAGCTATGAGCCATGATGAT
TTGCTGCTTGTCTTGAAAGGCGCGGCAAACAAGTTGGTACAGGAAATCAGCGTGTTTGAC
GTGTATCGCGGCACGGGACTGCCCGAAGGGATGAAGAGCGTGGCGGTCAAAGTGATTTTG
CAGGATATGGAAAACACGCTGACGGATGAGGCAGTCGAGCCGCTTATCGGAAAACTGATT
GGCGCGGCAACGGCGGCAGGGGCGCGGCTTCGCAGTTAAAAAATAAATATTCGCTTGAAT
TTTAAATAAAAATTGGTAATAATCCACAACTGTTACAACAGAAGGTAATCATATGACTCT
CACTAAAGCAGAACTGGCCGATATTTTGGTAGACAAAGTCAGCAACGTCACCAAAAACGA
TGCCAAAGAAATCGTCGAACTCTTTTTGAAGAAATCCGCAGCACTTTGGCAAGCGGCGA
AGAAATCAAAATTTCCGGTTTCGGAAATTTCCAGTTGCGCGACAAGCCGCAACGCCCGGG
TCGTAATCCTAAAACCGGCGAGGAAGTGCCGATTACCGCCCGCCGCGTGGTAACTTTCCA
TGCCAGCCAGAAACTCAAAAGCATGGTGGAACACTACTATGACAAACAACGTTGATTTGA
AGGTTCCCGCAAAACGCTATTTCACGCTGGACGAGTTGTGCGGACTGTTGCAAATCAGCC
CCTATGGTTTTGCGCAATGGCAGCATGATCACGGTGTGGTTGTCGGTTACGGCGGCGAAC
GCTACACCCGTTTGGATGTGGTGAAACTGTTGAAATTGCAGAGCACGTTTGCACCGTATG
CAGAAGGTGCGGAATCGGGTTCGGACGGCAACCGTCCGGTTACGCTTCAGGAAATCGGAG
ACGCTCTGAAAGACCTGTTGGCGGATTTGGATAAGGAATTGTGCTGATTTGAGGCCGGTT
GCAGGTATGCAGCCGGTTTTGTTTTACACGCTAAAAAATAATTATAGTGGATTAACAAAA
ATCAGGACAAGGCGACGAAGCCGCAGACAGTACAAATAGTACGGAACCGATTCACTTGGT
GCTTCAGCACCTTAGAGAATCGTTCTCTTTGAGCTAAGGCGAGGCAACGCTGTACTGGTT
TTTGTTAATCCACTATATTGCGTGATTTCACATTGTTTCGGCTTGAAGCACATGGTTTTG
TAATCATTTACAGGCAGCTCGCTTGGAGTCCTGTTCGGGCGGTTTGCTGTTTACTTAAAT
ATAAGGATGACGGTCAATGAGATTTTTCGGTATCGGTTTTTTGGTGCTGCTGTTTTTGGA
GATTATGTCGATTGTGTGGGTTGCCGATTGGCTGGGCGGCGGCTGGACGTTGTTTTTGAT
GGCGGCAGGTTTTGCCGCCGGCGTGCTGATGCTCAGGCATACGGGGCTGTCCGGTCTTTT
ATTGGCGGGCGCGGCAATGAGAAGCGGCGGGAGGGTATCCGTTTATCAGATGTTGTGGCC
TATCCGTTATACGGTGGCGGCTGTGTGTCTGATGAGTCCGGGATTCGTATCCTCGGTGTT
GGCGGTATTGCTGCTGCTGCCGTTTAAGGGAGGGGCAGTGTTGCAGGCAGGAGGTGCGGA
AAATTTTTTCAACATGAACCAATCGGGCAGAAAAGAGGGCTTTTCCCGCGATGACGATAT
TATCGAGGGAGAATATACGGTTGAAGAGCCTTACGGCGGCAATCGTTCCCGAAACGCCAT
CGAACACAAAAAAGACGAATAAATATGAATGGAATGCCGTCTGAAGGTTCAGACGGCATT
TTTCCGGTTTGAAAATATAGTAGATTAACAAAAACCAGTACGGCGTTACCTCGCCTTAGC
TCAAAGAGAACGATTCTCTAAGGTGCTGAAGCACCAAGTGAATCGGTTCCGTACTATTTG
TACTGTCTGCGGCTTCGTTGCCTTGTCCTGATTTTTGTTAATCCACTATAAAATAGGGCT
GTAACCTTCAATCGGAATTTGTTGCCTGCGGGATATACGGTATGAATGTTTGGTATATAT
GGGACAGGATGGTGGAAATCTATCATAAGTATAAGAAGCCGTGCCTGGTTTTGGCGGTGG
ATTTTGTGATGGGTATGGTATTCATAGAGCCGAATGAGGAGCCGTGCATCGGTAGGTGCT
ATGCGCCTATGTCGGAGTCCCCTGATTTTGCTAACGCTGTTGCGATGGCTGTTGCTATGA
TCTGTATCGTATGGATTGCCGTTTTATAGTGGATTAAATTTAAACCAGTACGGCGTTACC
TCGCCTTGCCGTACTATTTGTACTGTCTGCGGCTTCGTTGCCTTGTCCTGATTTTTGTTA
ATCCACTATATCTATGACTGATTGAAGCGTTGGGCGGAGGCTGCGTGAAACGGTATTGGG
CGTTGGGCCGTCTGATTCCAATCGGGCTTGGGGAATGCGAAACGGTGTGCGCTTATACTG
CGGACGATTTGTTTCGCGGTTTTGCGCCCGAAACGGATGGAGAGGTGTGGGAAACGGTCT
GTCGGAGTAGAATACGCGTTTTGCGTTTGAATACAGTAAGAAGAAAAGAGAGAAACTTAT
GCCGTCTGAACATCAACACATATCATCATTGCTTGATTTCGACCGTACCCATCTGCTTCA
TCCCTATACTTCCATGACCGATCCGCTGCCCGTTTATCCTGTCAAACGTGCAGAAGGGGT
GTTTATCGAATTGGCGGACGGCACGCGGCTGATTGACGGGATGTCCTCCTGGTGGTGTGC
GATACACGGCTACAATCATCCTGTTTTGAATCAGGCGGTTGAGACGCAGATGAAACAAAT
GGCGCACGTGATGTTCGGTGGTTTGACGCACGAGCCAGCGGTGGAGCTGGGCAAGTTGTT
GGTCGGGATTTTGCCGCAGGGGCTGAACCGTATTTTTTATGCGGATTCGGGTTCGATTTC
GGTGGAAGTTGCGCTGAAGATGGCAGTGCAATACCAGCAGGCGCGGGGTTTGACGGCGAA
GCAGAATATCGCGACGGTGCGCCGCGGGTATCACGGCGATACTTGGAACGCGATGTCCGT
CTGCGATCCGGAAACGGGGATGCACCATATTTTCGGCAGCGCGTTGCCGCAGCGTTATTT
TGTCGATAATCCGAAAAGCCGTTTCGACGATGAATGGGACGGGGCGGATTTGCAGCCTGT
CCGCGCCTTATTTGAAGTGCATCATGCGGATATTGCCGCCTTTATTTTAGAGCCGGTCGT
GCAGGGCGCGGGCGGCATGTATTTTTATCATCCGCAGTATCTTCGCGGATTGCACGATTT
GTGCGACGAATTTGATATCATGCTGATTTTTGACGAAATCGCCACTGGATTCGGGCGCAC
GGGCAAGATGTTTGCCTGCGAACACGCGGAGGTCGTGCCGGATATTATGTGTATTGGCAA

## Appendix A

GGGTTTGAGCGGCGGCTATATGACGCTGGCGGCAGCAATCACTTCGCAAAAAGTTACCGA
AACGATTTCGCGCGGCGAAGCGGGCGTGTTTATGCACGGCCCGACGTTTATGGCAAACCC
GCTGGCGTGTGCCGTTGCCTGCGCTTCGGTCAAACTGCTTTTGTCTCAAGACTGGCAGGC
AAATATCCGCCGCATTGAAAGCATCTTAAAAGGCCGTCTGAAAGCCGCGTGGGACATTCG
CGGCGTGAAAGACGTGCGCGTTTTAGGTGCCATCGGGGTGATCGAGCTGGAAAAAGGCGT
GGATATGGCGCGTTTTCAAGCGGACTGCGTGGCGCAGGGCATTTGGGTGCGCCCGTTCGG
CAGGCTGGTGTATCTGATGCCGCCCTATATCATTTCAGACGGCGTTTTGACCAAACTTGC
CGACAAAACCGTGCAAATCTTGAAGGAACACAGCAAATGAAAGGCGTTTACTTCGTCAGC
GGCATAGACACGGACATCGGCAAAACCGTCGCCACCGGCGTGTTGGCAAAACAATTGTTG
CAGCAGGGCAAAAGCGTGATTACGCAAAAGCCCGTGCAAACCGGTTGCCAAAACATTGCC
GACGACATCGCCGTCCACCGCAAAATTATGGGCATACCGATGCAGGAAGCCGACAAACGG
CGGCTGACTATGCCCGAAATCTTCAGCTATCCCGCTTCGCCTCACCTCGCCGCCCGACTG
GATGGCAGGGCTTTGGACTTGGACAAAATCCGCACCGCCACACAAGAATTGGCGGCGCAG
TACGAAGTCGTTTTGGTCGAAGGCGCGGGCGGATTGATGGTTCCGCTGACGGAAAACCTG
TTAACCATTGATTATATCCGTCAGCAAGGCTATCCCGTCATCCTCGTTACCAGCGGACGG
CTCGGCAGTATCAACCACACTTTACTCAGTTTCGCCGCGCTCAAACAATACGGCATTCGC
TTGCACAGCCTCGTGTTCAACCACATCCACGACAGCCGCGACGCACACATCGCCCAAGAC
AGCCTGAGCTACTTGAAATGCCGTCTGAAAGCCGATTTTTCCGAAGCGGAATGGATGGAG
TTGGCAAAAACAGACGCGGTATAAAGATTGGGAAAAATATGGAACACCTATTTGGGAAAT
GGCTGCCCGACTTGCCCGCCGCCATTTCAGACGGCATCAGCCTGCCGATGGTGCGGCTGC
TGCACACCCGGTCGCTGACCGCCGCATTGCGCGCCTTGCCGCATACATTTTCGGTGGAAC
TGCTGAAACTGGGCGAATTGGAGACGGAATGCGGAGGGAGGCTGGTGCGCGAAGTTTTGT
TGAAGCTGGACCGTATCCCTGTTGTTGAGGCAAGGAGCGAATGCCGTATCGGTTCGGCGT
TTTGGCAAAACATTTTGGACTGCGGCACGCGTCCTTTGGGCGAGCGTCTGTTTCAAGCCG
ATTTGGAAGGGGCGCGTTCGGCGTTTGAGTTTGCCGTTGCCGGCGAAGGATGCGGACGGT
ACTTTGCCGCGCGGCGTTCTCGGTTTTCCCGTCACGGCGAGGAAATGCTGCTGACCGAGT
ATTTTCTGCCCGAACTGAAACGTTTTATCGGATAAAATACCGTTTTTTCAAGCTGCGCGG
CAATATGAATCCTAAATCCCCTTTATTTTTACGCCTGTCCGACCGTTTGGATGTGTACCT
GCGCCTGATGCGGGCGGACAAGCCCATTGGGACGCTGCTTTTACTGTGGCCGACCTACTG
GGCATTGTGGCTGGCTTCAGACGGCATTCCCGATTTGGCGGTATTGGCGGCGTTTACAAT
CGGCACGTTTTTAATGCGCAGTGCCGGCTGCGTCATCAACGACTTTGCCGACCGCGATTT
TGACGGTGCTGTCGAGCGTACAAAAAACCGTCCGTTCGCACAGGGCAGGGTCAAGAAAAA
AGAAGCGCTGCTGCTGACGGCATTTTTGTGCCTGCTTGCCGCATTGTGCCTGATTCCGCT
GAATCATCTGACTTGGCTGATGAGCCTGCCCGCGCTGTTTCTTGCGCTGACTTACCCGTT
TACCAAACGTTTTTTTCCGATTCCCCAACTCTATCTCGGGCTTGCCTTTTCCTTCGGTAT
CCCGATGGCGTTTGCCGCCGTTGCCGGAAACGTGCCGCCTCAAGCGTGGATACTCTTTGC
CGCCAATGTGTTATGGACTCTGGCGTATGACACGGTTTATGCAATGGCGGACAAAGAAGA
CGATTTGAAAATCGGCATCAAAACCTCCGCCGTCACGTTCGGGCGTTACGACATCGCCGC
CGTTATGCTGTGTCACGGAGGCTTTACCCTGCTGATGGCAGTATTGGGTGCGGTTATCGG
TGCGGCATGGGCATATTGGACGGCAATCCCCATCGTCCTGCTGCTGCAATACCGCCAATA
TGCCGCCATCAAAAGCCGCGTCCGGCAAATCTGTTTTGAAACGTTTTTGGCAAACAACAG
AATTGGTTGGGTGTGGTTTACCGCCATTTTTGCCCATACGTTTTTCGCGAAATAAGGCAG
GGCAATGCCGTCTGAAGAGCCGTAAACTGCTTTGGACGGCATTTCTATCTGTGCCGAAAA
GCGTTAAAATATGTTTTTAAAACGCTGTGTTATGTCAGCCCGTACCGTATGCGGGATTGA
GATTTGCCCCGGCAGTCGGTACAATCTTTCTGTTTTGCGATGTCTGAAAAGAGAAGCTTA
TGAGCCTTATCGGCGAAATTTTGCCTTTGTCCCATATTGTTTTGGATATGGAGGTAGGCA
GTAAAAAAAGGCTGTTTGAGGAAGCAGGCCTGCTTTTGGAACGCGAATCCTCATTGTCCC
ATGCTGATGTTTTCGAATGTCTTTTTGCCCGTGAAAAACTCGGTTCGACCGGTTTGGGGC
AGGGCGTTGCCATCCCGCACGGCCGTCATGCCGGCGTGAAGCAGGCGACGGGCGCGTTCA
TCCGCACGCGCGAACCCGTCGGATTTGACGCACCGGACGGCAAGCCGGTTTCCCTGATTT
TTATCTTGCTGGTTCCGGAAAACGCAACCGGCGAGCATTTGGAAGTCTTATCCAAACTGG
CCGGCAAGTTTTCCCAAAAAAGCATCAGAGAATCGCTGATGACGGTTTCCTCTGCGGAAG
AAGTCCGTGCCATCCTGACTGAAGAATAATATGCCCAGTATCTCCGTCCGCCGCCTGTTT
GATGACAACCAATACAAACTGCAACTCGCTTGGGCCGCCGGCAATTCGGGTGCGGACAAC
CGTATCGGCGTAGAGGCGGACAAGCCCGTCCTCGCCCTAGTCGGACACCTGAATTTCATT
CATCCCAACCAAATCCAAGTGGTCGGTTTGGCAGAGTCGGAATATCTGAACCGCCTCGAA
TCGGGGGAAACGGGTTATCAGTTTGGCGACCTGTTCGATATTTCTATGTCTTTGGTTATT
GTGGCAAACGGCTTGCCGGTTTCCCCGGGACTGCGCGACTATTGTCATAAAAACGATATT
CCACTGCTGACTTCCAAACTCGAAAGCCCCTATCTGATGGACGTGTTGCGGATTTACCTG
CAACGCACCTTGGCGGCATCGTCCGTCAAACACGGCGTATTTCTCGATGTGTTTGAAATC
GGCGTGCTGATTACCGGGCATTCCGGCCTGGGTAAGAGCGAATTGGCATTGGAACTGATT
TCGCGCGGCCACAGCCTGATTGCCGACGATGCGGTCGAGCTGTTCCGCATCGGCCCGGAA
ACGCTGGAAGGGCGTTGTTCGCCTATGCTGCGCGATTTTTTGGAAGTGCGCGGCTTGGGG
ATACTCAATATCCGCCATATTTTCGGCGAAACTTCCATCCGCCCCAAAAAAATCCTGCAA
CTCATTATCAATTTAGTCGAGGCGGACGACGAGTATATGAAGCAGCTTGACCGGTTGAGC
ATCCGCACCGAAACCGAATCCATCCTCAACGTCAACGTCCGTTCGGTTACGCTGCCCGTC
GCCGTCGGACGCAACCTCGCCGTTTTGGTTGAGGCGGCGGTACGCAATTACATTTTGCAG
TTGCGCGGTAAGGACAGTACGCGCGAATTTTTGGAACGCCATCAGACGCAACTTAAAGAA
AACGAACAACACAATGAAGATCGTCCTGATTAGCGGCCTGTCCGGTTCGGGCAAGTCCGT
CGCACTGCGCCAAATGGAAGATTCGGGTTATTTCTGCGTGGACAATTTGCCTTTGGAAAT
GTTGCCCGCGCTGGTGTCGTATCATATCGAACGTGCGGACGAAACCGAATTGGCGGTCAG
CGTCGATGTGCGTTCCGGCATTGACATCGGACAGGCGCGGGAACAGATTGCCTCTCTGCG
CAGACTGGGGCACAGGGTTGAAGTTTTGTTTGTCGAGGCGGAAGAAAGCGTGTTGGTCCG
CCGGTTTTCCGAAACCAGGCGAGGACATCCTCTGAGCAATCAGGATATGACCTTGTTGGA
AAGCTTAAAGAAAGAACGGGAATGGCTGTTCCCGCTTAAAGAAATCGCCTATTGTATCGA

## Appendix A

CACTTCCAAGATGAATGCCCAACAGCTCCGCCATGCAGTCCGGCAGTGGCTGAAGGTCGA
ACGTACCGGGCTGCTGGTGATTTTGGAGTCCTTCGGGTTCAAATACGGTGTGCCGAACAA
CGCGGATTTTATGTTCGATATGCGCAGCCTGCCCAACCCGTATTACGATCCCGAGTTGAG
GCCTTACACCGGTATGGACAAGCCCGTTTGGGATTATTTGGACGGACAGCCGCTTGTGCA
GGAAATGGTTGACGACATCGAAAGGTTTGTTACGCATTGGTTACCGCGTTTGGAGGATGA
AAGCAGGAGCTACGTTACCGTCGCCATCGGTTGCACGGGAGGACAGCACCGTTCGGTCTA
TATTGTCGAAAAACTCGCCCGAAGGTTGAAAGGGCGTTATGAATTGCTGATACGGCACAG
ACAGGCGCAAAACCTGTCAGACCGCTAATTCCGTCAAACCATTATGCCGTCTGAAACCCC
GTCCTCCGTTTCAGACGGCATTTCAAATATTGAAAAGAAAGAACAGACATGGCAATCCAA
TGGTTTCCCGGCCATATGAACAAGGCGAAAAAAGCCATCGCCGAGCGTGCAAAAAGCGTT
GATATGGTGATTGAAATGCTGGACGCGCGTATGCCCGCCTCCAGCGAAAACCCCCTGCTT
GCCCAGCTTTCCAAAGGTAAACCCAAACTTAAAATCCTCAACAAACAAGATCTTGCCGAC
CCCGAGCGCACCAAAATCTGGCTCGAACACTATAACAGCCGCCCCGACACCTGCGCCATC
GCCCTCGATTCCTCCGAAACAGGCGCACACGGCAAAATTACCCAAGCCTGTCGTGCCATG
ATTCCCCACCGCCAAGGCATAGATAAACCCCTGCGCGTCCTCATCTGCGGCATCCCCAAC
GTTGGCAAGTCCACCCTCATCAACGGCATGATAGGCAAAAAATCCGCCAAAACCGGCAAC
GAACCCGGCATCACCAAAGCCGAACAACGCCTCTTCCTCGCCGATGACTTCTGGCTCTAC
GACACCCCCGGAATGCTATGGCCGAAAATCATCGTCGAAGAAGGCGGCTACAACCTTGCC
GCCGGCGGCGCAGTCGGACGCAACGCGTTGGACGAAGAAGAAGTCGCCCTCGAACTTTTA
GACTACCTCCGCCGCCACTACCTCCCTATGTTGCAAGAACGCTACCAAGCCGACAAAGAC
CCCAGCAGCCACTGGGACGAAAACGTTTGGCTCGAATGGATAGCCAAAAAACGCGGCGCA
GTCCTCAGCGGCGGACGGATCAACTACCAAAAAGCCGCCGAAAACATCCTCACCGACTTC
CGTGAAGGCAAAATCGGCAGAATCACCCTCGAAACGCCGAACCAATGGAAACTTGGCTC
AAAAAAGCCCGTCAGAAAGAAGCCGAACTCAAAGCCATACGCGAAGCCAGAAAAGCAGAG
CGTAAAGGGCAGAAGCTTCGGAAGCATAAAGAATGCCGTCTGAAAAATATTTTTCAGGCA
GCTTCTCTCTACTCCAACCGATTTCAGACGGCATATCCAAACCCATGCCGTTTCAGCACG
GATACCCGTATGACCGACAAAATTTCTCCCGACGCGCTGATTGAAGCCGCACTGCTGACC
CAAACCGAACCGCTGACCGAAAAATCTATGCGCGAACTGTGTGTGCCGCCGTTGTCGCAA
GACAAACTGATTGATGTGTTGGCGCAGTTGAAAACGCGTTGGCAGGATAGGGCGTTGCAA
CTGGTGCATACGCAAGAGGGCTGGCGTTTTCAGATTGTTCAGACGGCATTCGAGCGGCTG
GGCAGCCTGCAAGAACAGCGTGCGCCGCGCTACTCCCGCGCCGTGATGGAAACACTGGCG
ATTATCGCCTACCAGCAGCCCGTAACGCGCGGCGACATCGAGGGCATACGCGGCGTGGCG
GTGTCGCAGAACGTGATGCAGACTTGCAGGATCGGGGGTGGATTGAAGTCATCGGACATC
GGGACACATTGGGAAAACCCGCATTGTGGGCGACAACGGCAACGTTCCTCAGCGATTTGG
GTTTGAACAGCTTGGAAGAACTGCCGCCGCTGACCGAACTGGGCGAACTGGTTTTGCCCG
ATTTGATAGAAATGCCGCCTACGGATGAAGAAGAGCCGGAAACCGTACCGTCCGATACCC
TGCCCAACTGAAATTCCAAATGCCGTCTGAAACGCACATTGCTTCAGACGGCATTGCAAC
AAATAAGCAGATAAAAACAAGCACTAAGAAAAATTAAGGAAAAACTTATTTTTAATTTAA
AAAATCTTAGTTATAATTCGTATATCTAAAGTTGATATTGCTTTTGTCGGTAGAATTGCT
AAGGAATCCTCACGATGCTTCTAACACTTTCTTTGCGTGATTTTGTCATTGTTGAAAATC
TGAATCTGGATTTTCAAAGCGGCTTTACCGTATTGACCGGAGAAACTGGCGCGGGCAAGT
CCATTACTTTGGATGCGATTGGTCTGCTGTTGGGCGATAAAGCCGATTACAGCCAAGTCC
GCAGCGGCGCAAAAGAAGCGCAGTTGTCGGCGTTGTTTGATATTTCCCATTTACCTGTTT
TAAAAGCAGAATTGTATGAACAGGGGCTTTTAAACGACGGAGAAGAAGAACTCAGTATCC
GCCGCATTATCGATGCCAAAGGCAAAAGCCGCAGCTTTATCAACAATCAGGCCGCTACCT
TGGCGCAACTCAAAGCCGTCGGTAGCCAGCTTATCGACATCCACGGGCAAAACGCCCATC
ATTCGCTTAATCAGGAAGCCGCCCAGCGCGAATTGTTGGACGCATTTGCGGGTAGCAGGG
AGCAGGCGGAAACCGTCAGGCAGCTTTATCAAAATTGGGCCAATGCGAAAAAAGCCCTCC
AAGAGGCGCAGGAACACGCCGATGCCGTCATTATCGAGCGGGAGCGTCTGGAATGGCAGT
TTAACGAATTGAATCAGTTGGACATTAAACAAGGCGAGTGGGAAGCCCTCAGCCAAAGCC
ACGACAGCCTTGCCCATTCTGCCGAGCTGTTGCAGGCTGCCGAAGAAGTCGGAAGCAAGA
TTGACGGCGACAACGGCATCCAACGCCATATCTATCAATGTCAAAAACTATTGGCCAATC
TGCAAAACATCGAGCCGCGCTTTGCCGAGAGCCTGAATATGTTGGCAAGCATCGAAGCCG
AATTGGGCGAAATCAGTGCCAATATGCGCGATGTGGCAGGTCGCAGCGACATCAATCCCA
ACGAACTTGCCGCACAAGAGCAGCGCATGGGCGAGCTGATGGGGATGGCGCGGAAATACC
GGATCGAGCCTGAAGAGTTGCCTGCCAAGTTGGCAGAAATCGAAGAACGCCTGCAAAGCC
TGCAAGCTGCCGCCGATTTGGACGCGCTCGAGCATAATGTTGCCCACAATTTTGCCGAAT
ATCAGGAAGCTGCCCACATCCTTTCTGCCATGCGCCATCAGGCGGCAGAGCGTTTGAGCG
GCGAAACGACCGAGCATATGCAACACCTTGCCATGAAAGGCGCGCGTTTCGACATCGTCC
TGTTGCCTTCGTCGCCGACGGCACACGGTTTGGAGCAGGTTCAATTTCAAGTTGCCGCCA
ACAAAGGCAATCCGCCCCGTCTGCTGAATAAAGTTGCCTCCGGCGGCGAATTGGCGCGTA
TCAGCCTTGCCTTACAGGTTGTTGCCAGCCAATATACCCAAGTTCCCACCCTGATTTTTG
ATGAGGTCGATACCGGTATTGGAGGGGGAGTGGCTGAAATGGTCGGCAAGGCATTACGTG
CGTTGGGCAGAAAACATCAGGTGCTTGCCGTTACCCACCTTCCCCAAGTCGCATCCTGCG
GAGAAAACCACTGGCGGGTGCGCAAGCACAGCGAGGGAGAGCAAACCGTCAGCGAAATCA
GTATATTGGATGAAATCCAACGGATCGAAGAGGTTGCCCGTATGTTGGGCGGAGAAGTCA
TTACCGATACGACGCGGCAACATGCGGCAGAATTGCTGCAACTTGCGTCGAAAAATAGTT
TATTTTAAAATCAATCAGTTAAAAAATAACTAAAAATAAAAGTCTAAAACAATAGACAGA
ACTCAGATAAATCCGTATTATCACGCTTTCTTAATCACTTGAACAAGTGATTGTGCTGCA
CCCGTAGCTCAGTTGGATAGAGTATCTGGCTACGAACCAGAGGGTCGGGCGTTCGAATCG
CTCCGGGTGCGCCAGTAAGAAAATACAATATGCGCCCATCGTCTAGCGGTTAGGACATCG
CCCTTTCACGGCGGTAACCGGGGTTCGATTCCCCGTGGGCGTGCCAATTCAAAATGCCTC
CGATTATATCGGAGGCATTTCTCATTTCTCATTTCTCATTTCTCATACTGAGACCTTTGC
AATAACATAGGTTACTAAAATTTTATGCTCAATCTCATTTTCAAAATGCAAAACTTTTCT
GATTTTTCCTACTTTTTGCTCAATATTAGGAAGGTTTTAGGCAATTGAAAATTTTTTGGC

## Appendix A

GCATTTTTATGCGTCAAATTTCGTTAACAGACTATTTTTGCAAAGGTCTCGGATTAACAA
AAATCAGGACAAGGCGATGAAGCCGCAGACAGTACAAATAGTACGGAACCGATTCACTTG
GTGCTTCAGCACCTTAGAGAATCGTTCTCTTTGAGCTAAGGCGAGGCAACGCCGTACTGG
TTTTTGTTAATCCACTATATTGAGTCCTCGAGAAGGGAAATAAAAATTAACATCCTTATA
TATTGAGTTCCTGAGAAGGGAAGATTAACAAAAATTAACGCCCTTTACTTCATACAATCA
ACAGGGCTTTTTCATTCCTTCCTTATCTAACAGGGGGTACAGAAACCGAAACGGCTGGCA
GGGTTAAGGAAGTCTTCGAATGTTACGGAACATTCATCTTGGACAGCAAAGGCAATTTGT
TAGGCATTCCTTACTCCTTATTTTGGGAAGAAAACGTTATGGGTGTTTTCGATATTTTAC
CGTCAGGATTGGTATGTTTATTTGAATATGATTTTCTGTGGTCGGGACGGCATGCGGCAA
AGACTTAAGGGGTTAGATCCTTCCTTCTGACGATGGCGCGGATGATGGTGCGGTTGGGGT
GTAGGGCGTGGCGCAGGCGTTGTGAAAAGGGATGGGGCAAGCCTAGGATTTGGGCTGCAA
TGGCGGCGGCGCAGATGGGGGCGGTGGCGAGTCCGCGGGTGCCGTGCGCGGTGTTGACGT
AGGCATTAGGCAGGTATGGGCATGGGGTGTCGATGCGGTAGTTTTTGTCCAGCGCGAGTT
TGGTGTAGGTCTGCCGCATGGCGGCAATGTCGCCGAGTGCGCCGACTAGGGGAAGGTGGT
CGGGGCTGTCGCAGCGTATGGCGGCGTGCCCTTGGTGTTTTTGGGGGTTTGGGTTGGCGG
CAAACAATGATTCGGAAAGGGCGGGGTTAAGGTGTGCCAATGCTTGGCGGTTTGAGGCTT
CTTCGGCTTCGTTCCATCCGGTATGGCTGCTGTTGGGAATAAAACTCGCGCCGTAGCAGT
GCAGTCCGTGCCACGACGGGCTGATGTAGCTTTCGCCTGAAACGGCGCAACGCAGTTGTT
CGGAAAACGGGGTGGACGGTGTGAGGCCGGTTTGTCCGCGTATTTGCCTGAGAGGCAGGG
CGGCGAGGTTGGTTTCGGGTAGGTAGGGGCTGTTCGCACCGGTGCAGTAGATGATGTGTG
TGGCGGTAAATGTGCCGTTTGGCGTGCTTGCAATCCACTTTTCCCCGTCGTGGGAAATGT
CGGTCAAGGGTGTGTCTTCGTGTAGTCCAATGAGCGGATGGTTGAGGAGGGTGCGGACGA
ATGCGGGTGGATTGAGCCATACGCCGTGTTGCCAGTAGAGTCCGCATGAAGGGTGGTCGT
ATGGGACGGACAGTGGGATACCGGCGATTTTTTCGGCTTCTGCAGATGTGATGCTGCGGT
AGAGGTGGTTATGGTGTTTTTGCAAACCCAATTCGTGATTGCGTTGTTGTTCGGTGCGGC
TGTAATTGAGGTGGATGATGCCGTTGCCGCCCCAGGTTTCGGATTCGGGCAGGATGTGTC
CGAGCAGGCGTTTGGTGTAGCCGTAGCCGGCAAGCAAAAGTTCGGTCTGTTCGGTGTCGT
GCGGCGAGATTTTGGCGTAGAGCAGCCCTTGGCGGTTGCCGCTGGCGGCTTGGGCGGCTT
TTCGGGCTTCCAATACGGTAACGGAAATGCCGTGTGATGCTAAGGCGTGGGCGGTTGCCG
CGCCGGATATGCCCGCGCCGATAACGAGGATGTGTTCCGGTTTTTGCCGTTCGGATGTTT
GTGGAAGTGCAAACCAGGGTTTGTCGGGCTTGCTTTCGGTTTGCGGGATGGCTTCGGTCT
GCCAGGAAATGTGGTGGAAATGTTCGTGCAGGTGGTGGGTGCGTGAAGGGGGAACCAGCC
AGAAGCGGACATCGGGCAGGATGTGTTCGATGAGGTTGATGCTGTCGAACTGGAGGCACT
GCATTGCCTGATCCAAACGGTGCTTCAGACGGCATTCCGCGTCCGAAGCATCTTGTGCGG
TTTGAAAATCGGGAATCTGATTATCGGGGAGGCAGATAATCAGGTTGAGCGGGGGTGCGT
GTTTGCGGATGGCTTGGTCGAGTGTGCGGATGTCGGGAATGCCGTCCCATACGAGATTGT
CCATATCAATGCCGTTTAAAGTGTGGGTTTGAATATCGGTATCGGGATAAAGCTGTTAAA
ATACGCGCCGTTTGAAGGCACGCCTGCGCCTGCCGGATATTGTATGCCGAACCGAGGTGT
TTTTTGAATAATATTCCTGTTGAAATCCGTTTGTTGAAAAACCGTACCGTGTTGGTTTTG
ACTTATGGGGACGAACCTAAAAATCTGCCTGCCGAATTTTTACGCGTCTATTCGCCGAGT
GCGGAAGTGCGCGGACACGGCGTGGGACAGGATGTTTTGCAGACCGGCAAGGCGGATGTC
CAAATCGCGGATTTGCAGCCTGTCGGACAGTACGCGCTGAAAATCAGTTTTTCAGACGGG
CACGACAGCGGTCTTTACGATTGGGCGTATCTGCACAGACTGGCATACGGATACGATGCG
ATGTGGCAGGAATATTTGGACAAATTGGCGGCGGCGGGCGCGTCGCGTTTTGAAGAGAAA
TAAGACCGGTCGGATGGTAATCTGACGGGCAAAGGTATCAGAGAGGTGGTTAGAATATGG
GCGGACAGAAAACGCATTTCGGATTCAGTACGGTCAACGAAGATGAAAAAGCCGGCAAAG
TGGCGGAAGTGTTCCACTCCGTCGCCAAAAACTACGACATTATGAACGATGTGATGTCGG
CAGGGCTGCACAGGGTGTGGAAGCATTTCACCATCAACACGGCGCACCTGAAAAAAGGCG
ATAAAGTGTTGGCAGATTGCGGCGGTACGGGCGATTTGTCGCGCGGTTGGGCGAAACGGG
TCGGCAAGGAAGGCGAGGTTTGGCTGACCGATATTAATTCCTCTATGCTGACCGTCGGGC
GCGACCGTCTGTTGAACGAAGGCATGATTTTGCCCGTATCGCTTGCCGATGCGGAAAAAC
TGCCTTTCCCCGACAATTATTTCAACTTGGTTTCCGTGGCGTTCGGCTTGCGGAACATGA
CGCATAAAGATGCCGCGCTGAAAGAGATGTACCGTGTTTTGAAACCGGGCGGCACGTTGC
TGGTGTTGGAGTTTTCCAAAATCTACAAACCTTTGGAAGGCGCGTATGATTTCTATTCGT
TCAAGCTGCTGCCGGTCATGGGCAGGCTGATTGCGAAAGATGCGGAGAGTTACCAGTATC
TTGCCGAATCCATCCGTATGCACCCCGATCAGGAAACTTTGAAACAGATGATGCTGGATG
CGGGCTTCGACAGCGTGGATTATCACAATATGAGTGCGGGCATCGTCGCGCTGCATAAGG
GCGTGAAATTTTAAACGGACTGGCTGTGCAGCCAATGCCGTCTGAACACGTTTCAGACGG
CATTTTTGTGTTTTTTTGAGAAAGAGATTTTAAATCTTAAAATTAATTCACATATCCATC
AATAATTTATAAATTTTTTAAAAAATAGGAACAATTATCATTTGCAAGATTGGGAGATGT
CTGTATAATGCAGTCAATCCAGTAAACAACGCAGCAGACGAAAGGAGGGAAAAATGCCGG
AAAGTATTTTCAAACAGATTTCCCTTGATATTTTGAAACTGCATCGGGATTCTGTTTATT
CGCTGCTTGCCACTTCCGGCTGCAACTGTCAGGTGCATGAAGCGGCGTATGTCAACATCG
ACGGCAAATATTATATTGCGCTTTCATGCGAGCCCGAGGTGGGGGAAGTCAAAACAGGCA
TTTTGCTGATTGAGGATGAAAGCCGCAACCTTCGTTTGAGCTGGGTCGGCAGTGCGCGGG
AGCTTGACTGCAAGGATAATGCCTACAAACGCGCCCTGTCCGCGTTGTCCAGAAAGCTGG
GGCGGTGTAAGGACAGGCTGCATACGGCGGTTCAACCGTTTCTGTTGGAGCTGGTACCGG
AGAAAGGCAGATTTTCTGTCGGCGATGAAGAAGTTTGGATTTCTCGAAACGATTTAGTGA
GGGCTTTATATCCTGTCGGATACAGTATGCGGCAGGCAGTGTTTCAGATTTAAAGTTTTG
GTAGTGGTTTGTGTTCCTTTTGCGCCCCCTTTTCCAAGGGGCGATTTTTTTGCACGCGTG
TTGGCGGCAAAGGAAAAATGCCGTCTGAAAGGCTTTCAGACGGCATCCGCGTGCGGAATT
ACCTGTCCGGTAAAAGACGGATACCTTGATTGCCCAGCCGTTTTGACAATTCGGCAACCT
TTCCGTGTTTTCCTAAAACAAAATCAGGGAGGATTTCTGCCAAAGGGCGGATGACGAAAC
TGCGTTCGTGCGCGCGCGGATGCGGCAGGGTGAGTCGGGTGTCGTCGCTGGAGATGCCGT
CAAAGTCGATAATGTCCAAATCCAATGTGCGCGGCGCGTTGCGGAAGCTGCGTTCGCGTC

## Appendix A

CGAAATCAGCCTCGATACGGTTGAGTTCGGCAAGCAGGGCAATGCCGTCCAGAGTGGTGG
AAACGGTGCAGACGGCATTGACAAAATCGGGCTGATTGTCGTAACCGACGGGCGCGGTCA
TATACAGTGAGGAAGCCTGTTTAAGACGGATGTCAGGATGGACGACAGCGTGTCCAATG
CGGCGCGTACCTGTTGGGCAGGGTTTTCAAGATTACTGCCCAGGGCGATGACGGCAAAAT
GTCTGTTGTTCATAACGGTGTTTCAGAAAGGCAGGACTTTGGTTTTGGCAAGGTAAACGA
TGCAGGCGACGCAACACATGGCGAGCAGGTAAACGGTGTAGAACTTGGTCGAACGCGGAC
GGGCGCGCATCATCATCATACCCAATGCGATATAGGCGAGCAGAAGCAGGATTTTTGTAC
CGAGCCAAGGCGCGTTGAACGGGGAGAAATGGGTAATTTTCATCAGCCACAATCCCGTAA
ACAGCAGCATGGTGTCGTTAAGGTGGGGCAGTGCCTTCCAAAAGCCCGCCAAGGGCTTTT
CTGGATTTTTCCAAAGTAGGAAAAAACGGATGTTGAATACCAAAATGGTGATGGTAACGA
AGATTTGGTGGCTGTATTTGACAATCAGATACTGCATGGTCGGCTCGTATCAAAATAAGG
GTTAGAATCGGCTTATTTTACCGCAAACAGTTATTTTTGACGCAGTTTTTCAAATACCAA
AAGATAGGGTGGGCTGTTTTTCCGGTTGGTAAAGCCGTAACGCAAAACGGCAAACTGTTC
TTGAGGCAGGTTTTTTGCCCATTGTTCGATTGCTTCTGCCTCCTGTTTGCCGTTTTCGTG
TCCCGGATAGAGGACGGCAATAAGCATACCGTTTTCTTTCAGTAGGATAAGGTGGCAGA
AAGGGCGGCAATGCTGGTTTCCGTGCGGGTGGTAAGGCTTTTGTCCCCGCCGGGCAGCCA
GCCGAAATTGAAAATGGCTGCATCCAGCGGCTTTGGAATATATTGCTTCAGGTTTTCATG
TCCGTCCAAGATGAGCCGTACATTGCTGTAACCTGCTTCCTGCAGACGGCATCGGGTGTT
GTTCAGGGCTTGCGGCTGGATGTCGAATGCCCACACTTTCCCCCGGATGCCTGCGGTTTG
TGCGAGGAAAAGGGTGTCGTGTCCGTTGCCGGCGGTGCCGTCCAAAGCATTGCCACCTTC
GGGAAGTGCCTTCCGCAAAAGGCAATGGGCGAATGGAAGGATGTTTTGCAATAACATTTT
TAAATGCTGTCTGAAAATAAAAATACCTTACCGTTGTCCGGTAAGGTATTGAAAGATATG
ACACGTCATGCTTCGTGCGGATTATTCGGCAGGCTGCTGGACGGTTTCCACTTGGACGAG
GGTCGAAGTCGGTGCGGCTTGGGGTCTGTTTTCATGTTGTAGGTTGACGGAGCGTGCCGG
TACGATGGTGGAAATGGCGTGTTTGTAAACCATTTGGGTGACGGAAGTGTTTCTCAGGAG
AACAACGTATTGATCGAAAGACTCAACCTGACCTTGTAATTTGATACCGTTAACTAAGTA
AATCGAAACCGGAACGTGCTCTTTACGCAGGGCGTTCAGGAAGGGATCTTGCAACATTTG
TCCTTTAGCTGTCATATTTTTAACTCCGTTATTATGATTGTGAAATCGGGCAGACGCCCT
GTGCGTGTCGAATTGTAGCCTTGAATAGGAAAAATTACCAATTTTTTTGTGTTTGGGCG
GTTTTCCGCCGGGCATTTGTATGTCAGGAGCGTTGCTGCAGCATCCACGATTCGATTTTG
CGGGCGTCGTTGACGCGTGTGGCGGATGTGGGCGAGTTCAGCAATACGATGGTAACGGGT
TGGTTTTGAATGTTGGCTTTGACAACCATAGACCTGCCTGCTTCGCGTATGTAGCCGGTT
TTCTGCAATTCGATGTTCCACATGCCTTCTCTGACCAGGGCATTGGAGTTTTTGTAGTTC
TGCTGCCCGTTTTTGGTCTGTACCGAGGCGTAGTTGGAAGTCGAGTTGGTGCGGATTTGC
GGATATTGGGCGGCGGCGTTGACCATAAGGCTCAGGTCTTTGGCGGTAGAAACGTTTTGG
AAGTTGAGTCCGGTCGGTTCGTAAAAGCGGCTGCCGTACATACCGAGGCTTTGGGCTTTG
CGGTTCATGGCGGCGACAAATGCGCCCATGCCGCCGGGGTAGGTTCTGCCCAATGCATGG
GTGGCGCGGTTTTCGCTGCTCATCAGGCTCAGGTGCAGCAGTTTTTTGCGTGTAAGTGCC
GTACCTATGGCAAGACGGCTGCCGGTCCCTTTGATGCGGTCGATTTCGTCGGGCGTAATG
GTAACGGTTTCGTTCATGTCCAAGTTTGCATCCAAAACGACCATCGCGCTCATCAGTTTG
GAAATGGAGGCGATGGGCATAATCCTGTCGGCGTTTTTCTGATACAGTATCTGTCCGGTT
TTGTTGTTGACGACCAGGGCAGACTGTGAGGAGAGAATCAGACCGCCTGTAATGGCTTGG
GTGTTGGTGGGGTGTATCGTGCTTTCGGCGAATATTTCTATCGGATCGGAGGAGGTAAGC
ATGTTCTGTTCTAAAAATTGCCCTAAAATGTCGTTGTCGGCAAAAAGGTGGGCTGACGGC
AGGGAAAGGCAGAGACCGAGCAGCAGCGATGAGGGCAGGCGTTTCAGAGTATGGATGGAC
ATTTTTGATTCCATATTTTTGAGTATTGGCGTTATTTTGTTGAAAAAACAGCCATCTGTA
AAGTATGTCGTCTGACAGGAGACTGCCCGTAGGCGGGCGGTCTTGTTGTGTTTTGGGGAT
TGGGTTTTATAACATTCTGTTTTTAAATCGGAACATATTTTGTGGTTTGACATGGATATT
TTTCATGCCGTCGTGTGTCGGTTTGGATGTTTCCGGCGGTTGAATCCTTGTCCTTTGGGG
CGGTAGAATCGGGGTTGGTTTGGCAATTGCGGCGGTGCGTCTGCGTGCCGTTTTGAATAA
TGGGAATATCGGGAGTAGGACTATGGATGTGAAATATGAATTTACCCTGCCTTCGAGCAG
CGGTGCGGATTTTCATTCGGCAGAACATCTGCCTTTGGTCGTGTATTTTTATCCGAAAGA
CAGTACGCTGGGCTGTACGACGGAAGGCTTGGATTTCAATGCGCGTTTGGAACAGTTTGA
GGCATTGGGTTATACCGTGGTCGGTATTTCCCGCGACGGCGTAAAGGCGCATCAGAATTT
TTGCGCCAAGCAGGGTTTCCGGTTCGAGCTGTTGAGCGACAAGGATGAAACAGTGTGCCG
CCTGTTTGATGTCATCAAATTGAAGAAACTGTACGGGAAAGAGTCGTTAGGTATCGAGCG
CAGTACGTTCGTCTTGAATAAGGATGGAGAAATCGCCCATGAATGGCGGAAAGTCAAAGT
GGCGGGTCACGCGCAGGAAGTATTGGAAACGCTTTCCCGATAATGTGAACCATGCCGTCT
GAAGAAGATTCAGACGGCATTTGTTTGGAACGGTATGGAAGAAGGTTTGATCGACAGGCT
GCTTGAAACGCTGTGGTTGGACAGGCGGCTCAGTCAGAATACTTTAAACGGTTACCGGCG
CGATTTGGAAAAAATCGCCCGCCGCCTGTCCCAATCGGGCAGAATGCTGAAGGATGCGGA
CGAAGCGGATTTGGCGGCGGCGGTTTATGTTGACGGAGAGCAACGGAGTTCGCAGGCGCG
CGCATTATCGGCATGCAAACGCCTGTATATATGGATGGAGCGTGAAGGCATAAGGACGGA
CAATCCCACCCGTTTGCTGAAACCGCCCAAAATCGACAAGAATATTCCGACCCTGATCAC
CGAGCAGCAGATTTCCCGACTGCTTGCCGCCCCGGATACCGACACGCCGCACGGTTTGCG
GGACAAGGCTTTGCTCGAATTGATGTACGCGACCGGCTTGCGCGTCAGCGAGGCGGTCGG
GCTGAACTTCGGCAATGTGGATTTGGACAGGGGCTGTATTACCGCGCTGGGAAAGGGTGA
TAAGCAGAGGATGGTCCCGATGGGGCAGGAGTCGGCGTATTGGGTGGAACGCTATTATAC
GGAGGCACGCCCACTTCTGCTGAAAGGCAGGAATTGCGACGCATTGTTTGTCAGTCAGAA
AAAGACGGGCATTTCCCGTCAGTTGGCATGGATGATTGTCAAAGAATATGCAAGTCAGGC
AGGCATCGGGCACATCAGCCCGCACAGCCTGCGCCACGCCTTTGCCACGCATCTGGTGCG
GCACGGCTTGGATTTGCGCGTGGTTCAGGATATGTTGGGACATGCCGATTTGAATACGAC
GCAGATTTATACCCATGTTGCCAACGTATGGTTGCAGGGTGTAGTGAAGGAACACCATTC
CCGAAACTGAGGCTTCTACTTTGGTTTAATAAGAAATCAATCGAAAATGCAAACTTGATA
AAAAATTACCAAGACAAACCGTGTATACCGACCTTGCAATGCGAACCGTTTTTATTTATA

## Appendix A

TTCGCATACGATAATAAAAGCCGCTATCGGTACGATAGTTTGAGAACACACGGAGCACAA
AATGTTTGTCTGCATCTGCAATGCCGTTACCGACCATCAAATCAAGGAAACCATCGCCGC
CGGCGCGACCACAATGGGCGATTTGCAGTCGCAATTGGGCGTAGCGAGCTGCTGCGGCTG
CTGCGGGGAGCTTGCCGCTTCGTTTCTGACGGCGCACAATGCGCAACCGACGGTTACGGC
GGGTATCAACGTTCAAGCGTAAAACGGTTTTCGAAATGCCGTCTGAACTGTTCAGACGGC
ATTTTTACTGTTTTTGGCAGGACTTGAGTATCATCTTCCTCGAAAACATTGTTTTTTCCC
AAATAGACCATGATTCTGCTGCGTCTAAGGCTTTGGCGTGTGCAAATTGACAGATAAGGA
AACGCGGATGAAATTGACCTTGATGTTTCGTGAATATTGCAGCTTGTGCCACAAAATGCG
CGACGAACTCAAACCTTTTCAGGATGAATACGGGTTCGGGCTGGAAGTGGTCGATGTGGA
TGAAAATCCTGTTTTGGAAGAAAAATACAATGAGCTGGTTCCCGTTTTGTTGGCGGGAGA
TGAGGAAATCTGTCACTGGTTTTTGGATGAGGACAGGTTGAAACAGTTTCTCGAACGGTA
AAAAAATGCCGTCTGAAGCAGGACTTCAGGCGGCATTTTTTTCAAATCAACGTTCTTTAC
GTTTTTGCGGGGCGGATGACCTGCCGGTAAAGGAAGCACGTTTGGATGCTTGGTAAATTG
CTAGTCTTCTTCGATGTTGCATATTAACCCTTTCTTTATTTTATTTGTCGGTTGGGAGGA
TTCTTATTTATTGATTTTTTCAATAAAAATTAGAAAATTTATTGTGAGATGTTATTGTTG
GCAATCATATCATGTTTTACTGTTGATGGAAGCATGATTGTGTAAAGATGATATGTGTTT
GTGTAATCGGTAGATTTTATAGTGGATTAACAAAAATCAGGACAAGGCGACGAAGCCGCA
GACAGTACAAATAGTACGGCAAGGCGAGGCAACGCCGTACTGGTTTTTGTTAATCCACTA
TATAAGAAAACCGCCAAGGCGGTGCTTGACGGCGGAAACAGAGGGGCGGGCGGCATCAGA
GCAGCTTGGAAACGACCTGTGCAAGGCTTTTTGCCAGTCTGACGGTTTGATGCCGAAGTC
GTTTTCGATTTTGCGGCAGTCCAAAATGCTGTATGCGGGCCTGGGGGCGGCGGTCGGATA
TTCCTTGTCTGAAACGGCAGTCAATTCGGGAACGGGGAAGGATGTCTGCTGTTGCGATGC
CGCTTGGAAAATATGTTGGGCAAATTCGTACCAGGATACGGATTTGCTGCCGGCGTAGTG
GTAAATGCCGCGAACGGGATTGGAGTGCTGCAACAGGCGGATGATGGTGGCGGACAAGTC
GCCGGCATAGGTCGGGCAGCCGATTTGGTTGTGGACGGCGGACAGCGGGGAACGTTCCCG
CGCAAGGTTCAGCATCGTGCGGATAAAGTTGTCCCCGTATTCGCTAAACAGCCAAGAAGT
CCGCAGGATAAGGCTGTCGGGATTGGCAGACAGTGCGAGCAGCTCGCCTGCGGTTTTGGA
TTGTCCGTATACATTGGAAGGATTGGTAAAGTCGCTTTCCTGATAGGGTCTTTTCCCTTT
ACCGTCAAAGACATAGTCGGTTGAGATGTGGATGAATCGGGCATGGGCGCGATGTGCTGC
CAAGGCAAGGTTGTAAACGGCGGAAGCATTGACGGCAAATGCCGCTGCCGCATCGCCTTC
CGCCTTGTCGACGGCAGTATAGGCAGCCGTGTTGACAATGGCGTCGGGTTGGAAACTTTT
GACCATGTTGCAGACGGCATCGGCATCGGTAATGTCTAGGGATGCGGAATCCGTCGCAAT
GGTTTCCCAGTCTTCCGGAAGACGGTCGCGCAGGCAGCGTGCCAGTTGGCTTTTCGAGCC
TGTCAATAGGATTCTCATGAGGTATTTCCTTTGGTAAAAGTGTATTGTAGGACTTGCTGT
CGGTATTATAGTGCCAAAATTTTGCCGACGGTTGACGGGTTGGCTTTTTGTGCCATGGGT
ATTGTTTTGCGCCGACTTCGGCTAGAATATCGGTTTGTGATTCAAACCTGTCGGGTGTCG
GGTCGGTATTTTCAAGATATTTTCAGGGGGGGGCGAAATGAGTGAGATTGGTTTGGTAAA
GATCTATTTTGGAAAAGTGCGCGATTTATATGAAATCGACGATAAACGTATGCTGATGGT
CGCTTCCGACCGCCTGTCCGCGTTTGATGTGATTTTGGACGACCCGATTCCGAGCAAAGG
GGAGATTCTGACGCAGATTTCCAATTTTTGGTTTAAAAAACTGGCGCATATTATGCCCAA
CCACTTTACCGGTCAAACGGTTTACGATGTTTTGCCTGAAAACGAAGCCAAAGCTTTAGA
GAAACGCGCCGTCGTGGCTAAAAAGCTCACTCCGGTGAAAGTAGAGGCGATTGTGCGTGG
TTATCTGGCAGGCAGCGGTTGGAAAGATTATCAAAAAACCGGCTCGGTTTGCGGTATTCA
ACTGCCTGAAGGTATGCAGGAAGCGCAACAACTGCCTGAAGTGATTTTTACGCCCTCAAC
CAAAGCCGCAGTCGGCGATCACGATGAAAACATCAGCTTTGAAGAATGCGGACGCATTAT
CGGCAAAGAATTGGCGGAAGAAGTGCGCGCCAAGGCGGTTCGGCTTTACACCGAAGCGGC
GGAATATGCCAAATCGCGCGGTATTATTATTTGCGATACCAAATTTGAATTCGGTTTGGA
TGAAAACGGTACGCTGACGCTGATGGATGAGGTATTGACTCCCGATTCGAGCCGTTTTTG
GCCTGCCGACCAATACAAAGTCGGCACCAATCCGCCGTCTTTTGACAAACAATTCGTCCG
CGACTGGCTGGAACAAAGCGGTTGGAACAAAAAAGCCCCTGCGCCGAAAGTGCCTGCCGA
TGTGATTCAGAAAACTGTCGAGAAGTATCGGGAAGCATTGACTTTGCTGACTCAGGATTG
ATTTTTAAGTTTGAAGGCCGTCTGAAAGAAATATGGTTCAGACGGCCTTTTTATTGTATC
AATACTGGATTTTAAGGATGGTTGCCTTTATAATCCGCAATTGCTTTCAGCGTCCGAAAT
GCCGTCTGAAAGCTTGTTTATAACCTGCCGCACGGTCTGAAACCCTAACTATGCACATTC
GGATTTTAGTGTGCATTATTAGTGTTTTAGCAGTGCGGTATTTTGAAAGGAACAATGATG
TTCGACAAACACGTTAAGACCTTCCAATACGGTAATCAGACCGTTACTTTGGAAACCGGC
GAAATTGCCCGCCAAGCCGCCGCTGCCGTTAAAGTCTCTATGGGCGACACCGTTGTTTTG
GTTGCCGTTACCACCAACAAAGAAGTGAAAGAAGGTCAAGACTTCTTCCCCCTGACCGTC
GATTATTTGGAACGCACTTACGCCGCAGGCAAAATTCCCGGCGGTTTCTTCAAACGCGAA
GGCAAACAAAGCGAAAAAGAAATCCTGACCAGCCGTCTGATCGACCGTCCGATTCGTCCG
CTGTTCCCTGAAGGTTTCTACCACGACATCCAAATCGTAGCGATGGTCGTGTCCGTCGAT
CCTGAAATCGATTCTGATATTCCTGCAATGTTGGGTGCATCTGCCGCGCTGGTGTTGAGC
GGCGTACCGTTTGCCGGCCCGATCGGCGCGGCACGCGTCGGTTATGTAAACGGCGTGTAC
GTTTTGAATCCGACTAAAGCCGAATTGGCGAAATCGCAATTGGACTTGGTGGTCGCCGGT
ACTTCAAAAGCCGTGTTGATGGTGGAATCCGAAGCCAAAATCCTGCCCGAAGACGTGATG
CTGGGCGCGGTGGTTTACGGCCACGATCAAATGCAGGTTGCCATCAATGCAATCAATGAA
TTTGCCGACGAAGTCAATCCGGAACTTTGGGATTGGAAAGCACCTGAAACCAATGAGGAA
CTGGTTGCCAAAGTCCGCGGGATTGCCGGCGAAACCATTAAAGAAGCGTTCAAAATCCGT
CAAAAACAAGCGCGTTCTGCCAAATTGGACGAAGCTTGGAGTGCGGTAAAAGAAGCCTTG
ATTACCGAAGAAACCGACACTTTGGCAGCCAACGAAATCAAAGGCATTTTCAAACACTTG
GAAGCCGATGTCGTCCGCAGCCAAATTTTGGATGGCCAACCGCGCATCGACGGCCGCGAC
ACCCGCACCGTCCGTCCGCTGAACATCCAAACCAGCGTATTGCCGCGCACGCACGGTTCT
GCATTGTTTACCCGTGGCGAAACCCAAGCTTTGGCCGTTGCAACTTTGGGTACTTCGCGC
GACGAGCAAATCATCGACGCGCTGTCCGGCGAATACACCGACCGCTTTATGCTGCACTAC
AACTTTCCGCCGTACTCTACCGGCGAAGTGGGCCGCATGGGCGCACCGAAACGCCGTGAA

## Appendix A

ATCGGTCACGGCCGTTTGGCTAAACGTGCATTGTTGGCCGTATTGCCGAAACCTGAAGAT
TTCAGCTACACCATGCGCGTGGTCTCCGAAATTACCGAATCCAACGGCTCTTCCTCTATG
GCTTCCGTCTGCGGCGGCTGCCTGAGCCTGCTGTCTGCCGGCGTGCCTTTGAAAGCACAC
GTTGCCGGTATCGCGATGGGTCTGATTCTGGAAGGCAACAAATTTGCCGTCCTGACCGAC
ATTTTGGGCGACGAAGACCACTTGGGCGATATGGACTTTAAAGTGGCCGGTACGACCGAA
GGCGTTACCGCGCTGCAAATGGACATCAAAATCCAAGGCATTACCAAAGAAATTATGCAA
ATCGCTTTGGCACAGGCCAAAGAAGCGCGTCTGCACATCTTGGATCAGATGAAAGCCGCC
GTTGCGGGCCCGCAAGAGCTGTCCGCACACGCGCCACGCTTGTTCACGATGAAAATCAAC
CAAGACAAAATCCGCGAAGTTATCGGTAAGGGCGGTGAAACCATCCGTTCGATTACCGCT
GAAACCGGTACGGAAATCAATATTGCCGAAGACGGTACGATTACCATTGCCGCAACCACT
CAAGAAGCCGGCGATGCGGCGAAAAAACGCATCGAGCAGATTACTGCCGAAGTGGAAGTG
GGCAAAGTGTACGAAGGCACTGTGGTGAAAATCCTCGATAACAATGTCGGCGCGATTGTC
AGCGTGATGCCGGGCAAAGACGGTTTGGTACACATCAGCCAAATCGCCCACGAGCGCGTA
CGCAATGTCGGCGACTACCTGCAAGTCGGTCAGGTGGTGAACGTGAAAGCATTGGAAGTG
GACGACAGAGGCCGTGTCCGTCTGTCCATCAAAGCCCTGCTGGACGCGCCTGCCCGTGAG
GAAAATGCCGCCGAGTAACGCTTAGGGTGAAAGTGCCGTCTGAACAGGTTTCAGACGGTA
TTTTTTACGGGTATCGGGAATGAATGGGGCTTACAGCCACAGGACGGCAAGTTTCCATAA
TGCCCATAATGATACGGATAATCCCGTACACAGGCGGATATATCGGTTTTGCATGATTTT
TTTCAGTTGCAGGGAAAAAATGCCGATTGCTAAAAGATTGGGCAGCGTACCCAGTGCAAA
GGCAAGCATATATAACCCGCCCGTTGCCGCACTACCGCTTCCCAGCGCGTAAAGCGACGC
GCTGTAAACCAGTCCGCACGGCAGCCAGCCCCATAATATTCCGACCGCAAGGCAGGCGGG
TATGGATTTTATGGGTAACAGCCGGTTGAGTATCGGGTTCAGGTTCCGCCATATCGGTTT
GCCGATTTTCTCGATTTTTGCCGCCAAGGAAGAAATACCGCTCAAGTATAAGCCTAAAAA
GAGCAGCAGGAGGTTGGCGGCCGTGTATAAAATATTCTGCAGGACGCGGGTTTGGTCGAG
TGAAACGCCGACCTGTCCGATTAATCCGAGTATCAGGCCGATTGCCGTATAGCTGCTTAC
CCGTCCTGTGTTAAGCAGCAGGATCAGCCAAAAGCGGTTGATATGCGGGGGGAGTTGGAG
CGCAAACGCGCTGCTTAATCCGCCGCACATACCGATGCAGTGCGTTCCGCCGAAGAAACC
GAGTAGGAACAGGGTGAGGAAAGTGATGTCGTGGTTCATAGGCAGTTTGAAGTCAAATAT
TTTTCGGGAAAAGGGATGATTTGCGGCAGTCCGGCACATAGGATCCGCCGAGGGCATTGC
CCGTGCTGTTAAAGTCTTGAATAAGGATGCAGTTTGCACCCTGTATTTCGATAATTTTGT
AAAATCCGCCCTTTACTGCGCCGTCGGCGGGTTTGCCGTGTGCGTCAAAATACAGGATGG
TGCGGTTTTGAAGATGCGCGCAATTTGAAACGGCCGGGTTTGCCGGTATGTTTCGGGTGC
AGGCGGCAAGGATTGCACAAGGGAAAAGCAACAGTAATATGCGGAACATGGTGTTTCTTG
TAAGGGGTAACAAACAGTATAATGGCTGATTTTAATCCTCAGGCGGCGGGAGATGGAAGC
ATTTCCCTTCGGTGCGGGGGATTTCGGATTCGGAAGCAACAGACGATACGGGATTTCGGA
ACAATATGAACACTTTGAAATTTACCAAAATGCACGGTTTGGGCAACGATTTTATGGTGA
TTGACGCGGTCAGTCAGGATTTTACCCCCGAGGACGCGCCGATTGCGGAATGGGCGGACC
GCTTCCGGGGCGTGGGCTTCGACCAGCTTTTGGTGGTCGGGCGTTCGGAAACCGAAGGCG
TGGATTTCCGTTACCGTATTTTCAATGCCGACGGCAGCGAGGTCGGGCAATGCGGCAACG
GAGCGCGTTGTTTTGCCCGTTTTGTTGCAGACAAGGGTTTGACCGATAAGAAAGAAATTT
GTGTTGAAACGGCAAATGGCGTTATTTTTCCGAAATTGTCCGATAACGGTATGGTTACGG
TCAATATGGGCAAACCGAAGTTTATGCCGTCTGAAATACCGTTTGTCCCCGAATCGGGCG
AGGGGGATGATGCCTGTATTTACGGGGTGCATCTCGAATCCGGCATTCAGCCTGTCAGCT
GCGTCAATATGGGCAACCCCCATGCGGTGATTGTGGTCGATGACGTGGAATGCGCGCCGG
TGCGCGAAACCGGTTCGCTTATCGAACCGCACAGGCAGTTTCCCGAACGCGTCAATGTCG
GCTTTATGCAGGTTGTCGGCCGAACCGCGATTCGTTTGCGCGTGTTCGAGCGCGGCGTGG
GCGAAACCCAAGCTTGCGGTACGGGCGCGTGTGCGGCTGTGGTGGCGGGTATCCGTCTGG
GGCTGTTGGATGAAGGGAAAACGGTAGAGGTGGTTTTGCCGGGCGGGACTTTATATATCG
AATGGGCCTGCGGCGGCGATGTGATGATGACCGGCCCTGCGGAAGCGGTGTTTGAAGGTG
AGTTGGCGTATTCATGATTTTGCTGCATTTGGATTTTTTGTCTGCCTTACTGTATGCGGC
GGTTTTTCTGTTTCTGATATTCCGCGCAGGAATGTTGCAATGGTTTTGGGCGAGTATTAT
GCTGTGGCTGGGCATATCGGTTTTGGGGGCAAAGCTGATGCCCGGCATATGGGGAATGAC
CCGCGCCGCGCCCTTGTTCATCCCCCATTTTTACCTGACTTTGGGCAGCATATTTTTTTT
CATCGGGCATTGGAACCGGAAAACAGATGGAAACGGATGGCAGGCAGACCCCGAACATCC
GCTGCTCGGGCTTTTTGCCGTCAGTAATGTATCGATGACGCTTGCTTTTGTCGGAATATG
TGCGTTGGTGCATTATTGCTTTTCGGGAACGGTTCAAGTGTTTGTGTTTGCGGCACTGCT
CAAACTTTATGCGCTGAAGCCGGTTTATTGGTTCGTGTTGCAGTTTGTGCTGATGGCGGT
TGCCTATGTCCACCGCTGCGGTATAGACCGGCAGCCGCCGTCAACGTTCGGCGGCTCGCA
GCTGCGACTCGGCGGGTTGACGGCAGCGTTGATGCAGGTCTCGGTACTGGTGCTGCTGCT
TTCAGAAATTGGAAGATAATACGGTGCGGCCGTATCGGAACAAAAAGTTATAAAGAAAGA
AATTTTGGATATTGGTTTTTTAGGCGGCATAGGTTTAGGATAAAGCCATATCCGAAATTT
GTTTATGTTTCGGCGCAAATCCCCTGCAATCGGACAGGATGCCTATGGGGATTGCGCCTT
ACTGTCGAAACCTTATTATTCAGGAGCAGAAGATGAAAATTGCAAACAGCATTACCGAAC
TAATCGGCAACACGCCTTTGGTCAAACTGAACCGTCTGACCGAAGGTTTGAAGGCAGAGG
TTGCCGTGAAACTGGAATTTTTCAATCCGGGCAGCAGCGTCAAAGACCGCATTGCCGAAG
CAATGATTGAGGGTGCCGAAAAAGCGGGCAAAATCAACAAAAACACCGTCATTGTCGAAG
CAACCAGCGGCAATACGGGTGTCGGTTTGGCAATGGTATGTGCCGCACGCGGCTACAAGC
TGGCGATTACCATGCCGGAAAGCATGAGTAAGGAGCGCAAAATGCTGTTGCGCGCGTTTG
GTGCGGAGCTGATTCTGACCCCTGCCGCCGAAGGTATGGCGGGCGCGATTGCCAAAGCGA
AATCCTTGGTGGACGCGCATCCCGACACTTATTTTATGCCGCGCCAGTTCGACAATGAGG
CAAACCCCGAAGTCCACCGCAAAACAACCGCCGAGGAAATTTGGCGGGATACGGACGGCA
AAGTCGATGTCTTCGTTGCCGGCGTCGGCACGGGCGGTACGATTACCGGCGTGGGCGAAG
TGTTGAAAAAATACAAACCCGAAGTTAAAGTGGTTGCCGTCGAGCCTGAAGCTTCACCCG
TATTGAGCGGCGGCGAAAAAGGCCCGCACCCGATTCAAGGCATCGGCGCAGGCTTTATTC
CGACCGTTTTGAATACCAAAATTTACGACAGCATTACCAAAGTGTCGAACGAAGCGGCTT

## Appendix A

TTGAAACCGCCCGCGCAATAGCGGAAAAAGAAGGCATTTTGGTGGGTATTTCTTCCGGTG
CGGCGGTTTGGAGTGCGTTGCAGCTTGCCAAACAGCCTGAAAACGAAGGCAAGCTGATAG
TCGTGCTGCTGCCTTCTTATGGCGAACGCTATCTCTCTACGCCACTTTTTGCAGATTTGG
CATAATGCTTTAATCGGATTGTCGAAACATTCAGACGCATTTTTCGGTATCGGTGTAACG
CCGTGCCGGAAAATGCGTTTTTGCATATATGCCGAAAACGCCGGTTGTGTTTTAATCAGG
TGTTGGTGTCGCCGCATCGCTTGAGGGAAATATTTTTTATAGTGGATTAACAAAAATCAG
GACAAGGCGACGAAGCCGCAGACAGTACAAATAGTACGGAACCGATTCACTTGGTGCTTC
AGCACCTTAGAGAATCGTTCTCTTTGAGCTAAGGCGAGGCAACGCCGTACTGGTTTTTGT
TAATCCACTATATTCGGGTTTTATTTGGCAGGACGGTTTTTTGCCCCAACGGAAAATAGC
CTGCCTGCCCGTAAAATCAGCCGTTTGTCCGGGTGCAGCCGGGGCTTTGGGCTTCAGACG
GCATATTTTCGGAATGGCGGCATTCTTGCCGTCGGCGCGGCAGCCGTATGGGGAAGGGAG
GGGATATTGTGGTCGGTAACGGCAAAAAATATGCCGCACCATTGCTGGTGCTGGGTTGCG
TGGTGTTCGGTCTGGGCAGTCTGATTGTCAGATCCGTCCCCGTCGGTTCGTATGCAATCG
CATTTTGGCGGTTGCTGATTTCGGTGTTCGTATTTTGGTTTTTAGCACGGTTTTTCAGGC
AAAAATTCCCAAAAAACAGGAAAACCGTCCGATATGCCCTGACGGCGGGCGTGTTTCTCG
CTTTCGATTTGGCGTTGTGGCACGAAAGCATACACGCGGTCGGGCCGGGTATTTCCACCC
TGCTCAACAGCCTGCAAATCTTTTTCTTGTCGGCAATCGGTGTTTTCTTTTTCGGCGAGC
GTTTGAGCGGGCTGAAAAAGGCAGGCTTAATATCGGCAGTTGCCGGCGTGGCGATGATTG
CCGGTGCGGAATTCGGCTACAACGGTAATGCGGTTTGGGGATTCGCCAGCGGTTTGGTAT
CGGGACTGATGCTCGCCCTGTCGATGGTGTTTGTCCGCAAAACCCATGAAATCGAGCCGG
TGGCGCTTTTCCCTTCAATGATGATTTTGAGTTTGGGCGGCGCGGTATCGCTGGTTGTTC
CGGCATTGCTGATGGATGGCGGCGCGCTTTATCCGACGACTTGGAAAGATGCGGGTTTGG
TGCTTGTGTACGGCGTGGTGATGCAGTGCTTCGCGTGGGCGATGGTTGCCTATGCGATTC
CGCTGCTTTCGCTGTCGCTGACGGGGCTGCTGCTTTTGTCCGAACCGGTTGCCGCCCTGT
TCATCGATTATTTCGGGTTGGGCAAACCGATTGAAGGCGTGCAGTGGGCAGGGGTGGCGC
TGACGCTTTCGGCAATTTACCTCGGTTCGCTGAAACGGCAGTCTTCACATTGATTTCATC
AGGGCAATATTGGCAGTTCGCCGTGCAAAACCTGCGTATATTGGACTGATAGGTAGGAAA
ATCCGACAACGTTAGACTCGCCTGTAAAAGTGAGGAATAGCAAATGCCGTCTGAAACTAT
TTTCAGACGGCATTCTTGGCTTCCTGGCCTAACGGATTGCCGTACCGGACCTGCCGAAAT
CGCCGAAGTTCATCAAAATGAACATTGCCTTGCCGACAACCAGCTTGTCATCCACAAATC
CCCAGTAGCGCGAATCGGCACTGTTGTCGCGGTTGTCGCCCATAGCGAAATAGCGTCCTT
CGGGAACTTTGCACACGAAACCGCTGCCGTCGTCGGCATATTGGCAGTGTTCCAAACCGC
TTTGCTCTATGGAATATCCGTTTTCAGACATAATATCGGAGGTATATTTGCCCAATACGG
GCAGGGAAACGGCAGGCTGTCCTTCTTTTTTCAGAATATTGAAGGATTTGCCGTCTAGAC
CGCTGCGGAACATATCCGTGTTGTGGATTTCGGAAGGGTCGGTGTCGTCGGGATAACGGT
ATGTGCCGTCAGGAATGTCGGAAGTGGGTTTGCCATTTACCGTCAAAATCTTATCCCGAT
ATTCGACCACATCGCCCGGAATGCCGACAATACGCTTGATGTAGGTCATCTCCGGCTGCA
GAGGATAATTAAAAACAACGACATCGCCCCGTTCGATTTTGCCTGTAGGAATAAATATAT
TGTTTAAAACGGGTACGCGCAGGCCGTAGGAAAATTTGCCGACCAAAATGAAATCGCCCT
TGATCAGGCCCGGGCGCATCGAGCTGGACGGGATTTGGAACGGTTCGGCGATAAACGACC
GGATGAGGAACAATACCAAAACGGTAGGGAAGAAACTGCCGAAATAATCGCCGAAGTGGC
TGCTTTCCGAGATTTCGGGATGAGTCTTCAGGCGGTATTTATATACCCCCCAAGCCGTAC
CGCACAATACAACGAAAATCAGGAAAACGGCGGTAAAGCTCATAAACAGGGACAAAGCGG
CAAACACGCCGACCGCTGTCAGGATATAGGCGTATTCAAGGCCGGAACTCCATTCCCCGT
TTTCCTGCCGCTTCTTGTCGCTTTTGAAATAAAGGATGATGCCGGCAAGCAGCGCGGCAG
CCGCGCCCGACATTAGCATTGTGTTCATTGTTGTTCCTTAATGCTTAAAAACCCGCCTGT
CCGTGCAACCGTTTTAAGGCGGCAAATTGCAAAATTTGTTTGCGGGCGCGTGCCCCTGAA
ATCAGGGCGGTTTGAGGGGTGTTCCCGACGCGCCGCCCTGTGTGCCGGAGTTATTTGTCG
CTCACCTGCAAAATCGCCAAGAACGCGCTTTGCGGAATTTCCACATTGCCCACTTGTTTC
~~ATACGGCGTTTACCTGCCTTTTGTTTTTCAAGCAGTTTTTTCTTACGCGTAATATCGCCG~~
CCGTAACATTTCGCCAAGACGTTTTTACGCAGTGCTTTGACGTTTTCGCGGGCGATAATC
TGGCTGCCGATGGCGGCTTGGACGGCAATGTCGAACATTTGGCGCGGAATCAGCTCGCGC
ATTTTCGATGCTAGCTCGCGGCCTCGGTGAACCGCGCTTTGACGGTGCACAATCAGGCTT
AAGGCATCGACTTTTTCGCCGTTGACCATAATATCCAGCTTAATCAAATCAGACGGTTGG
AACTCTTTGAAATGATAATCCAACGAAGCATAGCCGCGCGAAGTGGATTTGAGTTTGTCG
AAAAAGTCCATCACCACTTCGTTCATGGGCAAATCGTAAGTCAGCATCACTTGGCGGCCC
ATGTACTGCATATTGACCTGCACGCCGCGCTTTTGGTTACACAAAGTCATGACGTTGCCG
ACGTATTCCTGCGGCACAAGGATGGTCGCGGTAATAATCGGCTCGAGTATGGTTTCGATG
CTGCCGATGTCGGGCAGTTTGGACGGATTTTCGACTTCGATTTTTTCGCCGCTTTTCAAC
ACGACTTCATAAATCACCGTCGGCGCGGTGGTAATCAAATCCATATCGAACTCGCGCTCC
AAGCGTTCCTGCACGATTTCCAAGTGCAACAGACCCAAGAAGCCGCAACGGAAGCCGAAA
CCCAATGCTTGGGAAACCTCAGGCTCAAATTTCAACGAAGCATCGTTAAGCTGCAATTTT
TCCAAAGCATCGCGCAAAGCTTCGTAGTCGTGGCTTTCTACGGGATAAAGTCCGGCGAAT
ACCTGGCTTTGCACCTCTTGGAAACCGGGCAGCGGCTCAGTGGCAGGGTTGGCAACCAAA
GTAACCGTATCGCCGACTTTCGCCTGTCCCAATTCTTTTACGCCGGTAATCAAAAAGCCC
ACTTCGCCGGCTTTTAGTTCTTGTTTTTGAACTGATTTCGGTGTGAATACGCCCAGCTGC
TCGACCTGCGTTTCCGCCTTGGTGCTCATAAAGCGCACTTTGTCTTTCAGTTTGATGGTG
CCGTTTTTCACTCGAATCAGCATAACCACGCCGACATAGTTGTCAAACCACGAATCGACG
ATAACCGCTTGCAGCGGCGCGTTTTCGTCGCCGGTCGGTGCGGGGATTTTGGCAACGATT
TCTTCCAAAACGTCTTCCACGCCGATGCCGCTTTTGGCGGAACATTGCACCGCGCCGACG
GCATCGATGCCGATGATGTCTTCGATTTCCTGTTCCACGCGTTCGGGGTCGGCGGCGGGC
AGGTCGATTTTGTTCAAAACCGGCACGACTTCCACGCCCAAATCAATCGCGGTGTAGCAG
TTCGCCACGGTTTGCGCTTCCACGCCTTGCGACGCGTCAACGACCAAAAGCGCGCCTTCG
CAAGCCGACAGCGAACGGGAAACTTCGTAAGAGAAGTCGACGTGTCCCGGCGTGTCAATC
AGGTTGAGTTGATACACCTGCCCGTCGCGTGCTTTATAGTTGAGCGCGGCGGTTTGCGCT

## Appendix A

TTGATGGTAATGCCGCGCTCTTTTTCGATGTCCATGGAATCGAGCACCTGCGTACTCATT
TCGCGCAAATCCAAACCGCCGCAGTATTGGATGAAGCGGTCGGCAAGCGTCGATTTGCCG
TGGTCGATGTGGGCAATGATGGAGAAATTTCGGATATTTTTCATTAGAGTTGTTTTGAAT
GTCGGACAGTGGGTTTGGGAAATGCCGTCTGAACAAACGGCGTTGCGTCCGAATATCGGG
TGCAACGTGGAAATAGCCCGTTATTCTAACGGAAAACCGCTGTTTTGGCATAAGTTTGAT
AAAGGTCTTATAAAGATTTGACGATTTCTGCCACCATTTTTGCGGAATTTGCCGCCGCCG
TTTTCAAGAACTCGTCAAAGCTGATGTCTGCTTTTTCATCTGCCGAATCGGAAACCGCGC
GGATGATGACGAAAGGCGTTTCCAACTGATGACAGGTTTGGGCGATTGCCGCCGCTTCCA
TTTCCACTGCTTTGACTTCGGGGAAGTGCTTGCGGATTTCCGCCACGCCTTCGCTGCTGT
GGACAAAGCGGTCGCCGCTGACAATCAGCCCTTGTTCTACCGCCGCGCCTTCAAACGTCC
GCGCCGCCCGTTTTGCCGCCTCAATCAAAATGCCGTCTGAAGCAAACCTTGCCGGCAGTT
GCGGCACTTGTCCCCAGGCATAGCCGAATGCGGTTACGTCGACATCGTGGTGTGCGGTTT
CCGTGCCGATGACTACGTCGCCGACTTTCAAACCCTTGCCCAAACCGCCCGCGCTGCCGG
TGTTGATGACGCAGTCCGCTGCGAATTCACGGATAATCCAAGCCGTTGCAACCGCCGCGT
TGACCTTGCCGATGCCGCTCAATGCAAGCACCATGCGTTTTCCCGCCAATTCGCCTTCAT
AGGCGGAAAATCTGCCGAAAGAGACGGCTTTGACATTTTCCATCATCTCGCGCAAAAGCT
CGATTTCTTGTTCCATTGCGCCGATAACGGCTACTGTTTTCAAAGACATATTGCTGACCT
GTTGTGAATTTCGGATAGAATGCCTGATTATACACGCTAACACGGCAGGATTGAGTGGAG
GTGGTTTGTCCGTGCCGTCTGAAACGGTTTCAGACGGCACGGCGGGTTTTTGGTAGAATG
GGAAGGTACAGATTGTTTGAAGATTAGGGGACGAGGATGTTTACCGATGAAAATATGACC
GCAAAGGAAGAACTGTTCGCATGGCTGCGCCATATGAACCAAAACAAAGGTTCCGACCTG
TTCGTGACAACCCATTTCCCGCCCGCAATGAAGCTGGACGGCAAAATCACCCGCATCACG
GACGAACCGCTGACGGCGGAAAAATGTATGGAAATCGCCTTTTCGATTATGAGTGCGAAG
CAGGCGGAAGAATTTTCATCGACCAACGAGTGCAACTTCGCCATCAGCCTGCCGGACACC
AGCCGCTTCCGCGTCAATGCGATGATACAGCGCGGCGCGACGGCGTTGGTATTCCGTACG
ATTACCAGCAAGATTCCCAAGTTTGAAAGCCTGAACCTGCCGCCAGTCTTGAAGGATGTC
GCGCTGAAAAAACGCGGGCTGGTTATTTTTGTCGGCGGCACCGGCTCGGGTAAATCGACT
TCGCTTGCCTCGCTTATCGACTACCGCAATGAAAATTCGTTCGGACACATCATCACCATC
GAAGACCCGATCGAGTTTGTCCACGAACACAAAAACTGCATCATCACCCAGCGCGAGGTC
GGCGTGGATACGGAAAACTGGATGGCGGCGTTGAAAAACACGCTGCGTCAGGCGCCTGAT
GTCATCCTTATCGGCGAAATCCGTGACCGCGAAACAATGGACTACGCCATTGCCTTTGCC
GAAACGGGGCATTTGTGTATGGCGACGCTGCACGCCAACAGCACCAATCAGGCACTCGAC
CGCATCATCAACTTTTTCCCCGAGGAGCGGCGCGAACAATTGCTGACGGATTTGTCGCTC
AACCTTCAGGCGTTTATTTCGCAACGCCTCGTTCCGCGAGACGGCGGCAAGGGCAGGGTG
GCGGCAGTCGAGGTGCTGCTCAATTCGCCCCTGATTTCGGAGTTGATTCACAACGGCAAC
ATCCATGAAATCAAAGAAGTGATGAAAAAATCCACTACCCTGGGTATGCAGACCTTCGAT
CAACACCTTTACCAATTGTATGAAAAAGGCGATATTTCCCTGCAAGAAGCATTGAAAAAT
GCCGATTCCGCACACGATTTGCGTTTGGCGGTACAGTTGCGCAGCCGCCGCGCGCAAAGT
TCCAGCCCCGATTTGGAACTGCTCTGATGGCGGTATGGATTTCCGGACGGATGGTTTGAA
ATGATTTATCCGTGGCATAATGAGCAATGGCGGCAGATTGCGGAACATTGGGAGCGTCGT
CCCAATGCATGGCTGTTTGCCGGCAAAAAAGATACGGGGAAAACTACATTTGCCCGCTTT
GCGGCGAAGGCACTGTTGTGCGAAACCCCTGCACCGGGCTGCAAACCCTGTGGCGAATGT
ATGTCCTGCCATCTGTTTGGACAGGGAAGCCATCCCGATTTTTACGAAATCACCCCCTTG
TCGGACGAACCCGAAAACGGACGCAAACTGTTGCAGATCAAAATCGATGCCGTCAGGGAA
ATCATCGATAATGTGTACCTGACTTCGGTACGGGGCGGTTTGCGCGTGATTCTGATTCAT
CCTGCGGAAAGTATGAATGTCCAAGCCGCCAACAGTTTGTTGAAAGTGTTGGAAGAACCG
CCGCCACAAGTGGTCTTTTTGCTGGTCAGCCACGCGGCGGACAAGGTTTTACCGACCATT
AAAAGTCGCTGCCGGAAGATGGTTTTGCCCGCTCCTTCCCATGAAGAGGCATTGGCATAT
CTGCGTGAAAGGGGTGTGGCGGAACCTGAGGAACGTCTGGCTTTCCATTCCGGAGCGCCG
CTGTTTGATGAGGCGGACGGTGTCCGTGCGTTGCGGATTAAACTGTTGGATATTTTGGCA
GAACCAAGGTTGTTGAAGATTTTGGATTACGCCGCGCTTTTCGATAAGGAAAAACTTCCG
CTCGCCGTATTTGTCGGGTGGATGCAGAAATGGCTGGTCGATTTGGGATTGTGCCTGCAA
CACATGAAACCCGTCTATTATCCCGCTTATGAAGACAGGCTGCTTCAGACGGTATCCGGT
TTCCGTCCGCGCAATGTATTTGCGGCGGAGGATATGCTCAAACAGCTTGCCCCCTACGGG
TTTCATACTTTAAATGTCAAAATGCAGATCGAGCATCTGCTCATCAACTATTTGGAATTG
AAGAAAGAGAACGGGTGAATTATGTCAGACGGACAAAATATTCCGGCAAAAATGATGTCG
TTGCAGCTGAAAGACATGAATCTGCTGTACAGCTCCTACATGCCGTTTTTGGAACACGGC
GGTCTGTTTGTGCAGACCAACGACGTATTTTCCATCGGGGACGATATTCTGCTTGCCGTA
GAAATCCTCAACTTCCCCAAACTGTTCCTGCCGACCAAAGTCGCCTGGATCAATCCTGCG
CGTACTTCCTCCAAACCCAAAGGGGTGGGGCTGGCATTCACAAAACACGAAAACTGCCTG
AAAGTCAAAGACCAGATCGAAGTCGAACTGGGCAACACAATCGGCGGCAGCAGACCTACG
TTTACCATGTAACGCCATGCATATCATCGATTCGCACTGCCACCTCAATTTTGAAGGTTT
GAAAGAACGCCTGCCCGAAGTTTTGTCCAACATGGAAGCAAACGGCGTGGGGCAGGCACT
CGCCATCAGCGTCAGTAGGGAAAGCTTCTCCGAAGTCTTTGCCATCGCCGAAGCGCACGA
ACACATCTATTGCACCATAGGCGTACATCCCGACAGCAAGGAAGCCGAAGAATTTTCCAT
TGCGGAAATGGTCGAAGCCGCCGCCCATCCGAAAGTGGTCGGCATCGGCGAGACGGGTTT
GGATTATTACTGGTGCAAAGGCGATTTGTCCTGGCAACACAAACGCTTTGCAGACCACAT
CGAAGCAGCCAATCAAACCGGACTGCCCGTTATCGTCCATACGCGTGATGCGGCGGCGGA
CACCTTGTCTATCCTGAAAGAATGCCGGGTTAATTCGGGCGTTATCCACTGTTTTTCCGA
AGACATCGGTTTTGCCCGTGCAGCAATGGATTTGGGGCTTTATATTTCTTTCTCGGGAAT
CGTTACCTTTAAAAACGCACCCTTGGTTCAGGAGGCGGCGAAATATGTGCCGGACGACCG
CATTTTGGTGGAAACCGATGCGCCGTTCCTCGCCCCCGTTCCCAAACGCGGCAGGCAGAA
CGAACCGGCTTTTGTGCGCCATACCGCCGAACATATCGCCAAATTGCGGAACCAAACATT
GGAACAGGTTGCGGCATATACGACGGAAAACTTTTACCGGCTGTTTAAAAAAGTACCCGA
TATGCGGACCGTCTGACCCTGTACCGACGATAAGGAAAACCATGAAGGCAATTCATCCGT

# Appendix A

ATGCATGTCCGCGCTGCTGCCGGCTGCCTGCCAACACGTTTCGGACAGGCATGGCAAATT
CCGCTTCCAAATTCTGCATTGCCAAAGGCGGCAGACGGGAAGCAAAAAAAGACGAAAGCG
GCGGCGGATATGCCCTGTGCCATTTGCCGGACAGCAGGATTGTCGAGGAGTGGGAATATT
TCCGTTCACAATATTGATACTGCGCGATATACGGCAAATATTGTGGGAAGTTTCCGCTTT
TGCGTATAATGCGCCCTACCTGACAAATTTTGTCAACTTTATCAAAAGGAATAAGCGATG
GCTTCCATCCACGACCAAATTAAAGAAGTAGTAACGACACACCGCGTCGTATTGTTTATG
AAAGGTACGAAGCAGTTTCCGCAATGCGGTTTCTCTTCCCGCGCCGTGCAAATCCTGAAC
GCGGCAGGCTGCACCGATTACGTTACCGTCAACGTATTGGAAAATCCCGAAGTGCGCCAA
GGCATTAAGGAATACAGCGACTGGCCGACCATCCCCCAACTTTATGTGAACGGCGAGTTT
GTCGGCGGTTCGGACATCCTGATGGAAATGTATGAGGCAGGCGAGCTGCAAGAGCTGCTG
AAAGCCTGATGGATTCGGCAATGCCGTCTGAACGTGTTTCAGACGGCATTTTCTTTTCCG
GCAAATCAAAAAAAAGTATAATGGCGCGTCTCAAAATCACATTGGAACACCGCGATGAAC
GTTAATGTTATCAACCATCCGCTCGTCCGCCACAAATTAACCCTGATGAGGGAGGCGGAT
TGCAGCACCTACAAATTCCGGACGCTTGCCACCGAGCTGGCGCGCCTGATGGCATACGAG
GCAAGCCGTGATTTTGAAATCGAAAAATACCTTATCGACGGATGGTGCGGTCAGATTGAA
GGCGACCGCATCAAGGGCAAAACATTGACCGTCGTTCCCATACTGCGTGCAGGTTTGGGT
ATGCTTGACGGTGTGCTCGACCTGATTCCGACTGCCAAAATCAGTGTAGTCGGACTGCAG
CGCGACGAAGAAACGCTGAAGCCTATTTCCTATTTTGAGAAATTTGTGGACAGTATGGAC
GAACGTCCGGCTTTGATTATCGATCCTATGCTGGCGACAGGCGGTTCGATGGTTGCCACC
ATCGACCTTTTGAAAGCCAAGGGCTGCAAAAATATCAAGGCACTGGTGCTGGTTGCCGCG
CCCGAGGGTGTGAAGGCGGTCAACGACGCGCACCCTGACGTTACGATTTACACCGCCGCG
CTCGACAGCCACTTGAACGAGAACGGCTACATCATCCCCGGCTTGGGCGATGCGGGCGAC
AAGATTTTCGGCACGCGCTAACTGACTGATTTTCGGAGTTGATATGAATTTTCAAGACTA
TCTCGCCACATTTCCTTCAATCGACCATCTGGGCGGTTTGGACGTTCAGGATGCCGACGG
CAAAACGGTTCACCACATTCCTGCCGTTCAGGGTAAGCTCGGTTCGCTCAAGCTGTACAA
TGCTTTGGCGGAACGTTTTGACGGAAAATTGGGTAAAGAAGCGGCAGAACAGGGTTTGAT
ATGGTTTGCCGAACACGTTGCCGACGCGCGTGCCCATCCGGGCAAGCATCCGAACATCGA
TCTGCTGGAAAATGTCGTGCAAAGCGGTGAAACCTGGTTGCTCAAGCCGCTTTCCGCGCA
ATAATTTTCGACCATGCCGTCTGAAATCCGTTTCAGACGGCATTTTGTCGGAAAGAAGAC
CGTAAAACGGGCATTTTCTTTTCTATTTCAGGATACGGGCAATGATGTTTCAACACACAG
GACGACACATAAAGCGCCGCCCTATGTGTTGCCCTAATTTGGAAGGGGTTACACCCTTTT
CAAATAAAATCTGATGCTGCTGCCACGAAGGACGGATGTCCGAGTGGCGGGGTTTCAACC
ATTAAGGAAATACGATGAAAAAAATGTTCCTTTCTGCCGTATTGCTTCTGTCGGCTGCCG
CCCAAACCGTGTGGGCGGATACGGTGTTTTCCTGTAAAACGGACAACAACAAATACATAG
AAGTCCAAAAAATCAACCGCAATCTTTACGAATATTCGTTCGGCAGTGCGGCAAAAAAAG
AAATTGCCATACGCAACAGCAAAGCTGACCTGTTGGGGCGTTCCGACAGGTGGCAAGGTA
TGGGCAGCGGTCGTTGGGCAACGATGAAATTCCAAAACGGCGAATTTATGTACACCATAT
GGACAGGCTTCGATTCCGTGACTCATACGGAAAGCAGCGGTGTCGTTGTGGAGCGTAGGG
GCAAGGAAGTCGCACGGGTCGGCTGTACGCCGAAAACCGCGCAGGCGAATTTCAACGATG
ACGATTTTTCCTAGTAATCGGGGCGGATAAGGCGATGGAAACAGCGAAACCCGTCATGCT
GATTGTCCGTCCGTCGGGCAGGGCCGCAGAAGATGTCGAAACTTGCCTGAATGCCGGTTG
GCGCGCGGAAGTATTGAGTCCGGTCGAAATCGAAGCAGATGCTGCCGGACTGGAACTTTT
GTCCGAACAATATGCCCGTGCGGATGCCGTGTTTTGGGTCAGTCCGACCGCCGTTGAAAC
CGCCGTCCCGTACCTTAACCTTTCAGACGGCATAAAGGCGCAGATTGCCGTAGGGCAGGG
CAGCCGCCGCGCATTGGAACGCTGTTTGGTCAGAACGGTCATCGCGCCTGATGACGGCAA
CGACAGCGAGGCGGTTTTGCGCCTGCCGGTTTGGAACAGTCTGCCCGAAGGTGCGCGCGT
ATTGTTTGTGCGCGGACACGGCGGGCGGGATTTTTTGATGAATGCCTTGCAGGAGAAAGG
TTTTCGGACGGAGGTGGCAGAAGTCTATTTCAGACGGCATAAACCTTTGAACTTTCAAAA
TTTCCAAACCGAAAATATTGCCGCCGCCTATATTACGTCGACCGAGCTGGTGCGCTTGCT
GTTCGGGCAGCTTCCGCCGCAATTTTCCCGATTCTTCAAATCCTTGCTATACTTTACCCA
TCATCCGCGCATTGCGGAGGCATTGAAGCGCGAAGGCGTGTGTTCGGTCGAAACCGTCCC
TACGCTGGAAGCCGCGCTTTCCCATTCTTCCATTTCCGTTTCAGACGGCATGGTCTTTCC
CGGAACCTCAAATTAATAAGGAGCAAAACGGTGGGCGAACCTGAAAACAAATCATCCGAA
CCCGTACGCGAGATACAGGCATCAAAAGAAATGCCGTCTGAAACCTCTTCCCCACGCAAA
GAAAACGAAACAGAAGTACACATTCCTGCCGCTCCTTTTATCGTCAAACAGTCCGGCAGC
AACGCTTTGGCAGTCTGCGCCCTGGTATTGGCGGCATTGGGTTTGGGTACAAGTGGTTTT
TTGTTTGTCCAAGGACAGAATGTCTTGAAAAACCAAGAGCTGGCATTCAACCAAAAAATC
GACAAAGCCGCCTTGGGCGAGTCGGAAAACGCCGCCCTGTTGAAAGACAACCTCAACCGG
CAAGCCGCCATACAATCAGAGCTCGACCGTTTGGACGGAAACGTCAAAGCAAACGGCGAA
CAAATCTTGGAAATGCAAAAATCCTATCGCGAGTTGACCAAAGGACGCGCCGATTGGCTG
GTGGACGAAACCGAGACCATACTCAATCTGGCGGCGCAACAGCTGGTGTTGACTGGCAAT
ATCCAAACGGCAGTCGGCGTATTGGAGCATATCGACAGCCGCCTGTCCCGTTTCAATCAG
GCAGAGCTTCTGCCGATCAAGCAGGCGGTCAGCAGCGACTTGGCGGAACTGAAAAACCGT
CCCTATGTCGATATTTCCGGCACGGCATTGCGCCTCGACAGGCTGGAAACCGCCGTATCC
GGACTGCCGCTGATGCTCGACGGCGTGCTGAAACCGGGCGTACAGGTGAAGAACGAAGCC
GCTTCCGCTTCATGGTGGCAGAACGTATGGGAAAAATCCCTCGGCACATTGAAGGGGCTG
GTCGAAATCCGACGTTTGGAAAACAACGATGCCATGCTGATTTCTCCCGAACAGGCATAT
TTTGTGCGTGAAAACCTGCGCCTCCGCCTTTTGGATGCGCGCACTGCATTAATGCAGCGC
AACAGCGAAGTCTATCAGGGCGATTTGAACAATGCCGAAGCCGCCGTCAGACAGTATTTC
GATGCCAAGTCTCCCGCCACGCAGTCGTGGCTGAAAGAACTGGCGGAATTGAAGGCGTTG
GATGTGCGGATGACTGCGGATGACGGTTTGAAAAACAGCCTAAATGCCGTCCGCGCCTAT
CGCGACGGTACGCGCATGACGGCGGCGGAAAATCAAGAAGCGGAACAGGCGGCTTCCGAA
CCGGCAAACGAAAAAACAGCTTCCGAACCGGCTGCCGCATCGGATGTGAAGACCATAGAA
GCACCGTCCCTGCCTTCGGAACGCAAACCGGAACAGCCTGCAAAAAAACAGACCGTACCG
GAAAAGGCAGGGCGTTCGCCGTCCGCTAAAGGAGAACGCGCATGAAAACGGTAGTCTGGA

## Appendix A

TTGTCGTCCTGTTTGCCGCCGCCGTCGGACTGGCGCTGGCTTCGGGCATTTACACCGGCG
ACGTGTATATCGTACTCGGACAGACCATGCTCAGAATCAACCTGCACGCCTTTGTGTTAG
GTTCGCTGATTGCCGTCGTGGTGTGGTATTTCTTGTTTAAATTCATTATCGGCGTACTCA
ATATCCCCGAAAAGATGCAGCGTTTCGGTTCGGCGCGTAAAGGCCGCAAGGCCGCGCTTG
CCTTGAACAAGGCGGGTTTGGCGTATTTTGAAGGGCGTTTTGAAAAGGCGGAACTAGAAG
CCTCACGCGTGTTGGTCAACAAAGAGGCCGGAGACAACCGGACTTTGGCATTGATGCTGG
GCGCGCACGCCGCCGGACAGATGGAAAACATCGAGCTGCGCGACCGTTATCTTGCGGAAA
TCGCCAAACTGCCGGAAAAACAGCAGCTTTCCCGTTATCTTTTGTTGGCGGAATCGGCGT
TGAACCGGCGCGATTACGAAGCGGCGGAAGCCAATCTTCATGCGGCGGCGAAGATGAATG
CCAACCTTACGCGCCTCGTGCGTCTGCAACTTCGTTACGCTTTCGACAGGGGCGACGCGT
TGCAGGTTCTGGCAAAAACCGAAAAACTTTCCAAGGCGGGCGCGTTGGGCAAATCGGAAA
TGGAACGGTATCAAAATTGGGCATACCGCCGCCAGCTGGCGGATGCTGCCGATGCCGCCG
CTTTGAAAACCTGCCTGAAGCGGATTCCCGACAGCCTCAAAAACGGGGAATTGAGCGTAT
CGGTTGCGGAAAAGTACGAACGTTTGGGACTGTATGCCGATGCGGTCAAATGGGTCAAAC
AGCATTATCCGCACAACCGCCGCCCCGAGCTTTTGGAAGCCTTTGTCGAAAGCGTGCGCT
TTTTGGGCGAGCGCGAACAGCAGAAAGCCATCGATTTTGCCGATGCTTGGCTGAAAGAAC
AGCCCGATAACGCGCTTCTGCTGATGTATCTCGGTCGGCTCGCCTACGGCCGCAAACTTT
GGGGCAAGGCAAAAGGCTACCTTGAAGCGAGCATTGCATTAAAGCCGAGTATTTCCGCGC
GTTTGGTTCTAGCAAAGGTTTTCGACGAAATCGGAGAACCGCAGAAGGCGGAGGCGCAGC
GCAACTTGGTTTTGGAAGCCGTCTCCGATGACGAACGTCACGCAGCGTTAGAGCAGCATA
GCTGATTTTGGGAAATATCTTTATCTGGGAGAATTTGATGGGGTCTTCAGATTCCTTTAA
GGAAAAGAAAGAAATATTTGAAATTGGAACGCCTGCTTATCGCCAAAAGTTAATTGATGT
TTGGAAAAAGAGCATTAATGGAAACGAAAAATCTTGGGTGCTCTTTGAAAATGGGACTTG
CGTCATTTTACTTGAACCGGAAAAAGATTTGGCGAAACAAGCTAAAGAGATGTTAAGCAA
ATGGGGCAAGGTTCAAATAGGAACACCATCTGCAGATTTTGGCATTATCACTTTAGATAG
TGGCGATGGATATGCCGTTTCATGCCATCATCCCGAAATTTTTACGCTAATCCTAAAAGA
AGAAGGATTGGATGAAGATTTCAAAATCGGTATCGAAGGGCGCTCTCATCGCGATTGTGA
TGCTGAAGAACCCAAAGTTATCCATATCGAAGATAAACGCACCATTGAAACCCCATGAAA
ACCTGCTGCCGTTTAATCATCTACTGATGATTACTTAGGCAAATGTGCCCGTCCCTCCCT
TTTCAGACGACCTTTCATTGCGGAAACCGCCGCAAAGGTTGTCTGAAAACCGTTTTCCTT
CCCCGTTTTACAAACAAACCGAAAGCCCCACATGATCTCTTTGAAAAACGACACTTTCCT
CCGCGCCCTGCTCAAACAACCTGTCGAATACACGCCGATTTGGATGATGCGCCAGGCGGG
GCGTTATCTGCCCGAATACAAAGCCACACGCGCGAAAGCGGGCAGCTTCCTCGATTTGTG
CAAAAACACCGAATTGGCGACCGAAGTTACCATCCAACCTTTGGAACGTTTCGATTTGGA
CGCGGCGATTTTGTTTTCCGACATCCTGACCGTCCCTGACGCAATGGGCTTGGGACTGTA
TTTTGCCGAAGGCGAAGGCCCGAAATTCAAACGCGCCCTGCAACACGAGGCCGACATCGC
CAAGCTGCACGTTCCCGATATGGAAAAACTGCAATACGTTTTCGACGCGGTAACTTCCAT
CCGTAAAGCATTGGACGGCCGCGTACCGCTCATCGGCTTCTCCGGCAGTCCGTTCACGCT
CGCCTGTTATATGGTCGAAGGCGGCGGCAGCAAAGAATTCCGCACCATCAAAACCATGAT
GTACTCGCGCCCCGATTTGCTGCACAAAATCCTCGATACCAACGCCCAAGCCGTTACCGC
CTACCTCAACGCCCAAATCGACGCGGGCGCGCAGGCGGTGCAGATTTTCGACACTTGGGG
CGGCGTGTTGAGCGATGCGGCGTTTAAAGAATTCAGCCTCAAATACATCCGCCAGATCGT
CGCCGGACTCAAACGCGAAAGCGAAGGCCGCCGCGTGCCTGTTATCGTATTTGCCAAAGG
CGGCGGGCTGTGGCTGGAAAGTATGGCCCAAATCGGCGCAGACGCATTGGGCTTGGACTG
GACGTGTAACATCGGCGAAGCACGCCGCCGCGTCGGCAAGCAAGTCGCCCTGCAAGGCAA
CTTCGACCCGTTCGCCCTCTTCGGTACGCCGGAATCCATCCGCACCGAGGTCGCACGTAT
CCTAGCCGACTACGGACACGGCAGCGGCCATGTCTTCAACCTCGGACACGGCATCAACCA
ACACGCCGACCCCGAACACGCCAAAATCTTAGTCGATACCGTACACGAGCTGTCTCGGCA
GTATCACGGCGGGTAAGCCGGCAGGAAACCGCCCGATATGCCGTCTGAAGCCGAGAGATG
GCCGGTTAGGGTAAAAATAAGGCAATGCGGCAATATCCGCCGTGTACGGATAGTACATGA
CGGCGGCGTTGTCGTATTGGCGCAATCCCAACCGTCCCTATGTTCAGACGGCATTTTTGT
TTTCAGATGCAGGGAAAACCGATGGCAAAAACGCTTAAAACCCTTTACCAATGCACCGAA
TGCGGCGGCACTTCGCCGAAATGGCAGGGCAAATGCCCGCATTGCGGCGAGTGGAACACG
CTTCAGGAAAGCCTTGCCGCGCCCGAGCCGAAAAACGCCCGTTTCCAATCTTGGGCGGCG
GATACCTCGACCGTCCAATCCCTCTCCGCCGTTACCGCCACCGAAGTGCCGCGCAATCCG
ACCGGTATGGGCGAACTCGACCGCGTATTGGGCGGCGGTTTGGTCGATGGTGCGGTCATC
CTGCTCGGCGGCGACCCCGGCATCGGCAAATCCACGCTGCTGTTGCAAACCATCGCCAAA
ATGGCGCAAAGCCGTAAAGTGCTATACGTTTCCGGCGAAGAATCCGCCCAACAAGTCGCC
CTGCGCGCGCAGCGTTTGGAACTGCCGACCGACGGCGTAAACCTTCTTGCCGAAATCCGC
ATGGAAGCGATTCAGGCGGCCTTGAAACAGCATCAGCCCGAAGTTGTCGTCATCGACTCT
ATCCAAACCATGTATTCCGACCAAATCACGTCCGCCCCCGGCTCCGTGTCGCAGGTGCGC
GAGTGTGCCGCCCAACTGACGCGCATGGCGAAACAGATGGGCATCGCCATGATACTGGTC
GGACACGTGACCAAAGACGGCGCGATTGCCGGCCCGCGCGTGCTGGAACACATGGTTGAT
ACCGTGCTGTATTTCGAGGGCGACCAACATTCCAACTACCGCATGATACGCGCCATCAAA
AACCGCTTCGGCGCGGCAAACGAACTGGGCGTGTTCGCGATGACGGAAAACGGTTTGAAA
GGTGTGTCCAACCCGTCCGCCATCTTCCTCGCCAGCTACCGCGACGATACGCCCGGCTCG
TGCGTTTTGGTTACACAGGAAGGCAGCCGCCCGCTTTTGGTCGAAATTCAGGCATTGGTC
GATGACGCGCACGGCTTCACGCCCAAACGCCTCACCGTCGGACTGGAACAAAACCGTCTT
GCGATGCTGCTTGCCGTGTTAAACCGCCACGGCGGCATCGCCTGTTTCGATCAGGATGTG
TTCCTCAACGCCGTCGGCGGCGTGAAAATCGGCGAACCGGCGGCGGATTTGGCGGTCATC
CTCGCGATGCTTTCCAGCTTCCGCAACCGCCCTATGCCTGAAAAAACCGTGGTTTTCGGC
GAAATCGGCTTAAGCGGCGAAGTCCGCCCCGTCGCACGCGGGCAAGAGCGGCTCAAAGAA
GCGGAAAAACTCGGCTTCAAACGCGCCATCGTCCCCAAAGCCAATATGCCGCGCAACGCC
AAAGAGTTTCCGAACCTGAAAATCTACGGCGTTTCGAGTTTGCAGGAAGCCATCGATATT
TGCCGCGACAGCAGGGAATAAACGGAAATGCCGTCTGAAATCGGGTTTCAGACGGCATTT

## Appendix A

GGTTTGTGGCGGATTGAAACAAGAAGGCATACCGGCGACAGATAAGATTTGCGGCAAAGT
TGCCTGTGATGTGGCAAAAACACACACGCCCGTCATCCCCGCAAGGGTGGGAATCCGGAA
TCGTCCGTTTCGGCAATGATTGAAAATCACGGTAACCCAACCGATTGGATTCCCGACTTC
GTGGGAATGAGGGGCGTGTGCATTTGATTTCCATCCGCCATATGTCGGCGACGGGCTTAT
TCGCCTACGGTTTTTGTATCAGTTTTTCGGCGTTTGCCAAAGTGTTTGCCACTTCGTCG
AAACCGATGCGGCTGCCGGCGATGAGGGCGCGCGTATCGGTATAGGCGGCGCGTACTTTG
CCGTCCGTTTCGGTAACGAGGACGCGTAGGGGCAGTTGCAGGGCGAAGGCGGGGTCTTTG
ACCATCAGCGGCGTGCCGGCTTTGGGCGTGCCGAAGACGATGACTTTTGCCGGCTGCATC
GTTAAGCCGTTTCGGCGGGCGGCTTCCTGATGGTCGATGACGGCAAAAATGTCCATCCCT
TTGCTTTTTATGGCGGTTTCAAGGCGGCTGACGGTTTCGTCAAAACTGTATTTTGAGGTG
AGGGTATGCGTGGTCATAGCGGTTTCGTTTTGGGTGGACGGTTCGCTGGCAGGATGTGCC
GAAGCGGTTGAAATGCAGAGTGCGGATGCGGCAATCAGGGGGAGTATGTGTTTCATCGTA
TTTCCTTTTTCCTTTTTGGTTGAAACGGTAGAATCAGACTTTATTCGGGAGGGGTGTAAC
CCTTTCCAAATCAGGGCAACACATAGGGCGGTGCTTTATGTGTCGTGAAACATCATTGTT
CCTTATCGGTTTGTTTATCAGGCTTCGGACGGGGCGGCGCGCCGCGCCCGATGTTGCG
CCGCGTGCCGGAACGCCGTATGCCGTCTGAAAGCCTGTCCTTTCAGACGGCATTGCGTCA
TTTCATCCCTTTTTTGAGCAGGTCTTCATAACCGCCGTGATTGGCAACATTTGTATAACC
TGCTTTTTTCAGCTCTTGAAGGGCGGCTTCGGCACGCCGTCCGCTGCGGCAGTAGAGGTT
GACCGGCGTGTCTTTGTCGGGCGCGGCTTCGTGTATGCGGCGGACGATTTGGTCGACGGG
GATGTTGACCGCGTTGTGCAAATGCCCTTCGCTAAATTCCTGTTCGGAACGGACATCGAT
CCAAACGGCCGGATGTTGCGCGGTTTGGGCGGCGGATACGGGTTTTTGCGGGGCTGCCTG
CGCGGCAAAGGCGGCTGAGGCAATGAGTGCGGCGGTAATCAGGTGTTTGATATTCATAGG
GTTTTCCTGCGGTTGTTGTCCGAAAGGACGGGAAGTTATTTTATCTGTTCCAAAGCGGCG
GCATCTATGTCCCAACGCCAAACGCCGCCGCCTTTGCCATCCAATCCGCGCAAAAACAGG
TAGCGGACGGAAACGGCGGCGGGCGGTTGTCCGCGCAGCTTGAAGTAGCGTGCGGCGGCA
ACGGCGTAAATCAGTGCCTGAAGGTAATAGTGGTGGTGTGCGACGGCTTCGTCCATTGCC
TGTTGCGTGTAGGCGGATGCGTCCGTACCGAGGTGGTTTGATTTGTAGTCGATGACGCAG
ATATTGCCGTCGGGGTCTTGGCAGACCATATCGACAAAGCCGTTTAAAAAGCCGTTGACG
GTGTGGAAGTCGAGCGTTTCGGCAGCGGCACGGCAGACTTCGGGCAGCCTGATGTCGTCG
CGGGCAAACCAGTCGCGCAGGCGTTTGAGGCTGAAGTCTTCGGTGTGGAGGGTAAAGCCC
ATTTCGGGACAGCGGCACTCGGGTGAGATGTCGGACAGGTCGTATGCCCCCGTCAGCGGC
GTTTTGCGGCAGGCTTCCGCCATTTCGGCAACGGCGGGCAGCCATATTTCTTCAAAACCG
TATTTTTTCAGCTTGTCGGCAATGAGGGTTTCCTGTCCGGCGGCTGCTTGTCCGAATTTG
AAATCTTCAAGAATTTCGTGCAGGCACAGCCCCGCCTGCGTGCCTTTCGGAAAATCGTGT
ATCGATATGCCGTCTGAAGCCGTCGGCGTTTCAGACGGCATCGCCGGCACCGAGGTTTCG
GCGGCATCCAAGGACGGGCAGGCATCTTCTTCGCCGCCGTCGGGCGTTTGGGTATGGCGG
CTTAAGGCGGTAAAGCTAGTGTGGCGGACAAATCGGAATCCGCGTTCGGGAATGCTGTTT
GCGGCAAATTCGGCGGTTTGACCGGCGTTGCTGCGATAGGCGGCAGGGGGCGGCGCATTT
TCGGTGAAGGCGAAATTTGTGCCGGAAGGGGCGTTGTCCGCCACGCGCCGCCAGTTGCGT
TTGAGCATCGCGATGCCGTCTTTTTCACACGCATAGGCACGGCGGACGGTTTCACGGCTG
TCTTGGGGCGAGCCTTCAATCAGGTAGGCGAGGGGGTTGTCGGCAGTATTGGTGGAGTAC
GCGGCGTAGATGTTGAGCTGTTCCTCGGCACGCGTCAGCGCGACATAAAGCAGGCGCAGG
CGTTCCGCCATTTCTTCATCGGCGTATTGTTTCTGTTCGTCTTCCGACAGTTGCGCCTTT
GCCAACAGTTCGGTTCGGTTTGCGCCTTGGTGGAGGATTTGCCAGTCGGACGGTCCGGTA
TCTTGCGCGTCCCACGCAAACGGGCAGTACACCAGCGGATACTGCAAACCTTTCGAGGCG
TGCATGGTAACGATTTTGACCAAATCTTCGTCGCTTTCCAGACGGATGGCGCGGTTGTCG
CCGCTGTTGTTTTCGGCAAGGCTGATTTGGTCGCCCAGCCATTTGTGCAGCGCGGCGGGG
TTGCGGTTTTGCGCGTCTTCGGCGGCAAGCAGTTCGAGCAGTTGGAAATAATTGGTCAGA
CTGCGCCCGTTGTTCCGGCTTAAGAGGCGCGTTTCGATGCCGTGTGTTTGGGAAAATTGC
TGCATAGCGGCGAAAATGCCGTATTTATTCCAGTTGTCGAGTGCGGTTCGGGCAGATTCC
GCCCAATGCAAAATCTCGCTTTCGTTTTGGTTGAAGTCGTGCAATTGCTGCGCGTCATAA
CCGAATATGCTGCTTGTCAGGACAAAACGCAGCGTTCCGGCGCGGCGCGGTTCGAGCCAG
AAGCCGATGAGTGCGGACAGGGCGGCGGCTTCGGGCGAGGCGAACACAGATTCGCGCGAA
AGCAGGACGCTTTGCACCTGCCGTTTTTTCAGGGCGGCGGAAACCATCACCGCCTCGTTG
TGCGTGCGTACCAGCACGGCAATATCGCCCGACTGCAACGGGCAGCCTTTGAAATTCAGA
CGGCCTCTGGCGGCTTCGTTGAGCGCGTGGGCGATTTCGTCGGCGCAATAGTCGGCGGCA
CGGCGGCGCAAAACGTCTTTGTTGGCTTTTTCATTGTCGTTTTCGTGCAGCCAACGAACC
TGTACGGCAGGACGTTCGGGGGACAGCCTGCTTTCGGCACGCGCCGCACCGACTTCCGAA
TAGCCGATGTTTTCCAAAACGAACGGGCGTTCTTTGAGGCGGAACAGCGCGCCTATGCTG
CCGATAAGCGCGGCGTGGCTGCGGTAGTTGGTGGCGAGCGTGTAGCGGTGCCGCGCGTCT
TCCGCCGCCTGAAGGTAGGCGTAAATGTCCGCTCCGCGAAAGCTGTAAATCGCCTGTTTG
GGATCGCCGACGAGGAACAGCGGTCGGTTTTGGGCGATGAAAATCTTTTGGAAGATTTCG
TATTGCAGCGGGTCGGTGTCTTGGAACTCGTCGATCAGCGCGGTTTCCCAGTTTTCGGCA
ACGGCGCGGGCGAGAGTGTCGGCGTGCGGATTGTCGGTCAGCGCGGTGTGGACATCGAGC
AGCAGGTCGTCGAAACCGCGTTCGCGGCGCGATTTTTTCATCTCGGCAAGGCTGCGGTTG
AGGTATTCGATTAAATCCAGTTGCAGCCGGATCATTGTTGCTTCTTCCGCTTCTTCGAGT
GCGTTCAAATCGCGCCCGAAGTCTGCCAGTTTCTGCAATTCGGCAAATACTGCCGCATCG
GGCGTTTTGCCTTTTTTCAGTCCGGCTTCGAGTTTGTCGGATGCAAGTTTCAAGAGTCTG
TCGTGTGTGTCTTTGTCCAGAAAGGGCAGTTGTCCGGCGGCGGATTTTTGTGCCAGTTCT
TTAAAAAGGTTGCCGAAGCTGTTTTTGCGGTAACTGTTGCCGTTGAGGTCGGGATGAATG
CGCCAAAAGCCGGCTTCCAGTTCTGGCAGCAGGCGGCAGATGGTTTGCCATGAGGTTTCG
GCGTTGCGCTGCGCCTGTTTCAAATCCGCCTGCGGACGGCGGAAATTCAGGTACGGGCGG
GAAAGATAGCGCGAAATTTGGGCAAGGACGGTTTGCGGCACAGCTTTGCGTTTAAGCGCC
AATGCGGCAAGCACCGGATCATTGCTGACGCGTTCCCGCCAAAAATCTTGCGCCGGGATA
AGCAGGCGGTCGCCGTCTTCTTCGGTCATTTCGACATCGAACGGTGCTTGGCACAGGAAG

# Appendix A

GCGTAGTCGCGCAGGATGCGCTGGCAGAAGCCGTGGATGGTATAGATGGCGGCGTTGTCG
AATTGCCCGATGGCGGCCTTGAGGCGGACAATCAGACGCGTCCGGCCCTCTTTTTGCAAA
GCCTGTTTTAAGAGTTCGGGCAGGAAGGTGTCGCCTTCGTGGTGTTCGGCGCAGTAGGCG
GCAATGCCGTCTGAAAGCGTGTCGTCTCCAAGTTTGGCAATTCCTTTGCTTTCTAAAACT
TGTAACACATCGTCCAAACGCCCGCGCAGGCGTGTTTTCAGCTCGGCGGTGGCGGCTTTG
GTGAAGGTTACGACCAATACGCGTTCGACGTTTTTTTGTTCTAATACGATCAGGCGTGTA
AACAGGGCGGCAATGCCGTAGGTTTTGCCGGTGCCGGCAGAGGCCTCAATCAGGTTGGTG
CCGGAAATGGGGACGGTTAGCGGGTCGAATGCTTGGATGCTTGCAGACATAGTGCGCGCT
CGGAAAACGGTTGGACGGTAAAACGGGAAAATGCCGTCTGAAAAATGGTTTCAGACGGCA
TCGTCCGGCTTAGAGGTTTTGCAGGCGTTCGACAGACGGCGCGTAGTAGTATCCGCCCGA
AACGGCGGCGGAGAGATGTCGGAGCAGCAGGTCGGTTTTGCCGTCCGTATCGCCGAACAT
ACTCAATAATTGCGCTTCGATATTGTGCAGCGTGCGGCAGTATGCGGTAAACATCAAACC
GTGTTCGCCGCTGATTTTGCCGAAGGGCAGGCTGCGGCGGACGATTTTCAGGCCGACTCC
GTTTTCTTTCAGGTTGACGCGGCCGAGGTGCGAATCGGGCAGGCGGACATCGCGGCCGAA
TTCGTCGTCGGTTTCCTTGCCGCGTCCGACCGAGGCCTCCTGTTCGGCGACGGGGACGGC
ATCCCATTTTTTCAAATCGTGCAGGTATTTTTGCAGCAGGACATAGCTGCCGCCCGCATC
GGGTTTTCCTTCGGGGATGATGGCGACTTCGCGGACATTTTCATCGCCCTGCGGGTTTTC
CGTGCCGTCGACGAAACCGTCCAGCCCGCGATCCTGATACAGGCGCAAACCGTGTTCTTC
GGACGCGACGCATATGCTGTCGCCGAACGCGCCCAAAACGGATTGGGCAAGCGCGTAGGC
GGCGTTTTGGCGGAAGGATTGGATGTGGATGGACATATCGTGCTGCGTGGACGGCGCAAG
CCCGTTGCCCATTTCGGAGAAGGGTTTGATTTCACTGCCTTCGTCCGTATGTCCGAATGT
TGCCCAGGCTTTGCTGCCGAAGGCGATGGTCAGACCCAAAATATCGTCCGGAAAGCGGGC
TTTCAAGGCAGTTAACGCGTCGAGCGAAGCGCGGCAGGCGGCTTTAATATCGTTGAGGCG
ATTGGCGGCGAAGTCGGCTTCGATAAAGATGCCGGCTTGGGCGTGGTCGGGAATGATGGC
GGATTGGGGCGTGTTCATGAGATGTTCCTTTTTGGTGTCATCTGTTTCGGATAGATTATA
GCACCGAATCGGCAGGCGGATTTTTGCCGGAACGGCGTGCGTGAATCCGCCGTTTACATA
CCTGATGCCGCTTTTCGGTTTCGTGCCGCCCGCCGCCTTTCCCGCCCCCTTTATTTCCGC
TTCCGGCGGCTTCGGCATATCTTTTCCATTCCGATTTGGAATAACCATATAAAAAAAGTA
TTCTTTGTGTTTGCCGCAATTTCACTTAGAATGCCGCACTTGCACACTTTTTACAGGAGA
GGATGATGTTGAAAAAATTCGTACTCGGCGGTATTGCCGCATTGGTTTTGGCGGCCTGCG
GCGGTTCGGAAGGCGGCAGCGGAGCATCTTCCGCGCCTGCACAATCGGCAGTTTCCGGTT
CTTTAATCGAGCGCATCAACAATAAAGGCACGGTTACCGTCGGCACGGAAGGCACTTACG
CACCGTTTACCTACCACGACAAGGACGGCAAACTGACCGGTTACGATGTGGAAGTAACCC
GCGCCGTGGCGGAAAAACTGGGCGTGAAAGTCGAGTTTAAAGAAACGCAATGGGATTCGA
TGATGGCGGGTTTGAAGGCGGGGCGTTTCGACGTGGTGGCAAACCAAGTCGGTCTGACCA
GCCCCGAACGCCAAGCGACATTCGACAAATCCGATCCTTACAGCTGGAGCGGTGCCGTAT
TGGTTGTCCGTAACGACAGCAACATCAAATCTATAGCCGACATCAAAGGCGTGAAAACCG
CACAATCCCTGACCAGCAACTACGGCGAAAAAGCCAAAGCTGCAGGCGCAGATTTGGTGG
CTGTTGACGGTTTGGCGCAATCGCTGACCCTGATTGAACAAAAACGTGCCGATGCAACCC
TGAACGACGAATTGGCGGTTTTGGACTATCTGAAGAAAAACCCGAATGCGGGCGTGAAAA
TCGTTTGGTCCGCACCTGCCGATGAAAAAGTCGGTTCCGGCCTGATTGTCAACAAGGGCA
ATGACGAAGCCGTGGCGAAATTCAGTACGGCAATCAACGAGCTGAAAGCCGACGGTACGC
TGAAAAAACTGGGCGAACAATTCTTCGGAAAAGACATCAGTGTTCAATAATTTCCTTGCT
TCGCTGCCGTTTATGACGGAAACACGCGCCGATATGATTGTCAGCGCGTTTTTGCCTATG
GTCAAAGCCGGCTTCGCGGTCTCTCTGCCTTTGGCGGCAGCTTCTTTCGTTATCGGTATG
ATGATTGCGGTAGCCGTGGCTTTGGTGCGGATTATGCCCGCCGGCGGCATCGTGCGGAAA
ATCCTGCTGAAATTGGTGGAATTTTATATTTCCGTCATTCGCGGTACGCCGCTGTTGGTT
CAGCTTGTGATTGTGTTTTACGGGCTGCCTTCCGTCGGCATCTATATCGACCCGATTCCT
GCCGCCATCATCGGCTTTTCGCTCAATGTCGGCGCATACGCTTCCGAAACCATACGCGCG
~~GCAATTTTGTCCGTACCTAAAGGCCAATGGGAAGGAGGTTTGTGCATCGGCATGACCTAT~~
ATGCAGACGTTCCGCCGCATTGTCGCGCCGCAGGCATTCCGCGTTGCCGTGCCGCCTTTG
AGCAACGAGTTTATCGGTTTGTTTAAAAACACCTCGCTCGCGGCAGTCGTGACGGTAACG
GAATTATTCCGCGTCGCGCAGGAAACGGCAAACCGCACTTATGACTTTTTGCCCGTCTAT
ATCGAAGCCGCTTTGGTTTACTGGTGTTTTTGTAAAGTGCTGTTCCTGATTCAGGCGCGT
TTGGAAAAACGTTTCGACCGCTACGTCGCCAAATAAGGAGTTGTCATGATTAAAATCCGC
AATATCCATAAGACCTTTGGCGAAAACACTATTTTGCGCGGCATCGATTTGGATGTGTGC
AAAGGGCAGGTGGTCGTCATCCTCGGGCCTTCCGGCTCAGGCAAAACGACGTTTCTGCGA
TGCCTAAACGCGTTGGAAATGCCCGAAGACGGACAAATCGAGTTCGACAACGAGCGACCG
CTGAAAATCGATTTTTCTAAAAAACCAAGCAAACACGATATTTTGGCACTGCGCCGCAAA
TCAGGCATGGTGTTTCAACAATACAACCTCTTTCCGCACAAAACCGCCTTGGAAAACGTA
ATGGAAGGACCGGTTGCCGTACAGGGCAAGCCTGCCGCCCAAGCGCGCGAAGAGGCTCTG
AAACTGCTGGAAAAAGTCGGCTTGGGCGACAAAGTGGATTTGTATCCCTACCAGCTTTCC
GGCGGTCAGCAGCAGCGCGTCGGCATTGCCCGCGCATTGGCGATTCAGCCTGAACTGATG
CTGTTTGACGAACCGACTTCCGCGCTCGATCCTGAATTGGTGCAAGATGTTTTGGATACC
ATGAAGGAATTGGCGCAAGAAGGCTGGACCATGGTTGTCGTTACGCATGAAATCAAGTTC
GCCTTAGAAGTGGCAACCACCGTCGTCGTGATGGACGGCGGCGTTATTGTCGAACAAGGC
AGCCCGCAAGATTTGTTCGACCACCCCAAACACGAACGGACGCGGAGATTTTTAAGCCAA
ATCCAATCTACCAAGATTTGATTAAGCATTTTTCCTGTGTTTACAGAGGCCAGATTAGAT
TCGGATTGCTTTCGATGACGGCTTTGAATTGGTTTTGAATCCGCTCGATGGCTTCTTGCG
TATCCGCCTCAAAACGCAACACCAGAATCGGCGTGGTATTGGAAGCACGCATCAGACCGA
AGCCGTCGGGAAATTCAACGCGCAGACCGTCGATGGTGATGATTTCGGTTGCGCCTTCAA
ATTCGGCTTTGGCGGCGAGTTCGTCGATAACCTGATGGCCGTTGCTGCCTTCGGGCAGGG
CGATGTTGAGTTCGGGCGTGGAAATGCTTTGCGGCAGGTTGTTTAACACTTCGGACGGAT
TATCGGAGGCAGACAGGATTTCCAAGAGGCGTGCGCCGGCGTACAGACCGTCGTCGAAGC
CGAACCAGCGTTCTTTGAAGAAGATGTGTCCGCTCATTTCGCCGGCAACCGGCGCGCCGG

## Appendix A

TTTCTTTCATGGCGGATTTGATAAAGCTGTGGCCGGTTTTTCCATTATGGCTTTGCCGC
CGTGTTCTTTAATCCAAGGCGCAAGCAGGCGGGTGGACTTCACGTCGAAAATGACTTTCG
CGCCGGGATTGCGGTTCAAAACGTCTTGGGCGAACAGCATCAGTTGGCGGTCGGGATAAA
TAATGTTGCCGTCTTTGGTAACCACACCCAAGCGGTCGGCATCGCCGTCAAACGCCAAGC
CGATTTCGGCATCACCGTTTTTCAGCGCGGCAATCAAATCTTGCAGGTTTTTCGGTTTGG
ATGGGTCGGGATGGTGGTTGGGGAAAGTGCCGTCCACGTCGCAGAAAAGCTCGGTTACTT
TGTTGCCCAAGCCTTTGTAGAGTTTGCCGGCAAACGCGCCGCCCACGCCGTTGCCCGCGT
CAATGGCGATGTTCATCGGGCGTTTGAGCCTGATGTGTCCGGTAATGTGTTTGAGGTATT
CGCCGGAGATGTCTTTTTCGGTGACGCTGCCTTGTTTGCCGGCGGCAGCAAAACCGTCTT
TTTCAATGATGGACAAAAGTTCTTGGATGGCTTCGCCGGCAAGCGTGTCGCCGCCGAGCA
TCATTTTAAAGCCGTTGTAATCGGGCGGATTGTGGCTGCCGGTAATCATCACGCCGCTGC
CGCCGCATTCGTTGACGGCGGCGAAGTAGAGCATAGGAGTGGCAACCATACCGACATTGA
GGACATTGATGCCGCTGTCGGTAAAGCCGCGCCGGATGTGTTCCATCAGTTCGGGACCGC
TCAAGCGTCCGTCGCGTCCGAGCGCGATGCGGGTAATGCCTTTTTCGGCGGCTTTGGCGG
CGATGGCTTTGCCGATAAGGTAGGCGGCTTCGTCGGTCAGGGTTTTGCCGACAATACCCC
GGATGTCGTAGGCTTTGAAGATGTCGCGGGCGATGCTTGCCATAAGGTTTCCTTTGTGTC
CGTTTAGGAAAAACGGGCATATTTTAACATAGCGGTATGCCGTCTGAAGGCTTGCGTCCG
GTTTTCAGACGGCATAGCACGGTTACATCAAATAACATGCCGTCTGAAATAAAAGCAGCC
TTTGTGCAGGCTGCTTTCGGATTGTCGGTTTATACCGCTTCGGCTTTAATGATGACGACA
GGTTCGCTCGGTACGTCGTCGTGGTAACCATGACGTTTGGTAGAAACGCCTTCGATGGCA
TCGACAACGTCAAAACCGTCAACGACTTTACCGAATACGGCATAGCCCCAGTCTTGGACG
ACGGTTTTGCCGTACAGCTCTTTAGAACGGAAGTTCAGGAAAGCGTTGTCGGCAGTGTTG
ATGAAGAATTGCGCGCTGGCGGAATGGGGGTCGGAAGTGCGTGCCATGGCGATGGTGTAT
TTATCGTTGGGCAGGCCGTTGGACGCTTCGTTTTGAATCGGATCGCGGGTTTCTTTTTCG
TTCATGTTTTCATCCATGCCGCCGCCTTGAATCATGAAGCCTTTGATGACGCGGTGGAAG
ATTACGCCGTCGTAGAAGCCGTCTTTGACGTATTGCTCGAAGTTTTTGGCGGTAACAGGG
GCTTTGTCGAAATCGAGTTCGATTTTGATGTCGCCTTTGTTGGTGTGCAGGATAATCATG
GGTTTCCTTTCGTTAGAATCTGGTTTTGAATGATTCGACAAATTGTGTCTGAACGACAAA
CTTCAAGGTCGTCTGAAAAATATTCTTTCAGACGGTCTTGTTGTTTAGGTCGATGGTTTA
CATCAGTACAGCATAAGCCCACAGAGCAACCAATACTACGCAGAGGATGTTCAGCAGTAT
GCCGACATTCATCATTTCGCGTTGCTTGATTAAGCCCGTGCCGAACACAATCGCGTTAGG
CGGTGTGGCAACCGGCAGCATGAAGGCACAAGATGCGCCGATGCCGATGACGAATACCAA
GACTTGTTCGGGCAGCCCCATCTGCATAGCGATGCCGGAGAAAATCGGTACAAGCAATGC
GGCGGAGGCGGTGTTGCTGGTGAACTCGGTCAGAAAAATAATGAAGGCGGCGACGATGAG
TATCACCAAAAATGCGGGCGCGCCGGAAAAGGTGGCGGCAACCTGCTGTCCCAAGGCTTC
GGACGCGCCGGATGTTTTCAACAGCGTGCTCAGGCTGATGCCGCCGCCGAAGAGCATCAA
CACGCCCCAGTCGGTATTGCGGGCGACTTCCTTCCATTGCGCCACGCCGAAGACGACGAC
GGCGACGGCGGCACTCAGGGCGATAACGGTGTCGGGATTGGAAATGCCGAAGGCGGTTTT
GATTTTGGAGCTGAATATCCACGCGGCGGCTGTGGCAAGGAAAATCAACAGCGCGATCAC
GCGGTGCAGCGTCCAAGGGATGGATTCGGCTTTGATTTCCACGCGTTCGTTCAAATTAGG
TTTGAGGATGACGTACAGGGAGAGCAGCATCAAGGGCAGAATCAACAGCATCATCGGCAG
GCCGAGCTTCATCCAGCCGACGAAGTCCAGATTTAGGGCTTTGGCGGCAATCAGGTTGGG
CGGCGAGCCGACGAGCGTGCCCAAGCCGCCGATGCTGGCGCAATAGGCGATGCCGAGCAG
GAGGAAGACGTAGGTTTTGTGTTCTTTTTCCTGGTCGAGGTGGCTCAGCATACCCATTGC
TAGAGGCAGCATCATCGCGGCGGTGGCGGTGTTGCTGATCCACATGGACAGAAAGGCGGT
AACGAGGAACAACATCAAAACCGCCACTTTCATATTGCCGCGCGACAGGCGCAACAGGCT
GACGGCGATTTTACGGTCCAGCCGCTGCATATGCAGGGCGGTGGCAAGCGCGAAGCCGCC
GAAAAAAATGTAGATAATCGGGTTGGAAAAATCAGCCATCGCCTTTTTGATGTCCATGTC
GGGGAAACCGAGTACGACGGCGAGAATCGGCACCATCAGTGCGGTTACGGTAATGTGGAC
~~GGCCTCGGTAAACCAAAGTGCGGCAACGAAAATCAGCAGCGCGATACCTTTATTGGCATC~~
GGGGCTGTAAGGCAGGATGTGGTAAATGCCGAAACAGACGACGGCGGAAATAATGGTGGT
CAGCAGGCCCTTAAAGTCGGTAATCGGCTTCTGCGCACTGAGCAGCTCGACGTTTTCGGG
ATGCTGGGTTTTGTCCTTTGCATGCAGGTTCATGAATACTCCTTTAAGGCAACAAAATCG
GTTTTTCTTTTGTGTCGTGCAATCCGAAACGGTTTGTGGAATCGCCGCTTCTGCAACTGG
TTCGAGTATATTTGTAATCTGATGTAGTGTAAATATATTGTAAACGATTTGTCGGTTTTG
TTTATGAGATGGGATTGATATGTAAGGGGAAAAATAGGATATATCGGGAAGAGGTGCATC
CTGTTTGGGGAAAATAAACCGATTTAGTCCGGGCGGCAGGCAGCTCGCGCGGTAAAGAGG
GCAAGGGCTGCGCCCGTCAGGTCGGCAAGGACATCGCCCAAACTGCCGGTTCTCGTTGCG
GTAAACCATGCCTGCGCGCATTCGCTGAAGAGGGCGAAACAGAGGGCAAAGACCATCAGG
CTGCGATAGGGGATGGGGCGGTTGTCGGTTCTGAATGCTTTGGTCAGAAGCCAGATTTGT
GCGAAAAACAGGGCGAGGTGCGCCACTTTGTCAAAATGCGGAAAAGGCGGTGGCGCGGTT
TCGGCAGCTTTGAAAAGCAGTGAGTAAATGCTGCCTGCAAACCACAATGCCGAGAGCAGG
ATAAAGCGGTTGCGTGGCAGATTCATGCTTGTTCCTCTTCAAGCCATGTCTGGCATAGTT
TGGATAGGCGCAGGAATTTTCCGCCGCGTGCGGCCAGCATATCGCGCCAAACGGCAATTT
CTTCGGCGGAGGGGGCATCGTCTATGCTGCATTCGTAGAGCAGGAAATCGAGGGTTTCTT
CGATGACGGGGATGGATTCGGTTTGGATAAGCTGCTTGAGTTCGGTCATGACTGTTCGGA
TATGGAAATCGGGAACATGCCGTCTGAAAGGGCTTCAGACGGCATCGGGTCATTTGCTGT
GCAGGAAGCGGGTTGCTTCTTCCCATTTGCCGGCAAGGATGTCGGGTATGGCTTGCAGGG
ATTTGGCGACGGCATCGTCAATCTGTCGGCGGTGTTCCGTACTGGGTTTGTTCAGGACAT
AGCCGACGACGAGGTTGCGGTCGCCCGGGTGGCCGATGCCGAGGCGCAGGCGGTAATAGT
CTGCCGTGCCGAGTTTTGCCTGAATGTCTTTCAAGCCGTTGTGTCCGCCGTTGCCGCCGC
CGAGTTTGAATTTGATCCGTCCGCAGGGAATGTCGAGTTCGTCGTGGACGACGAGGATTT
CTTCGGGTTTGATTTTGTAGAACTGTGCAAGCGCGGCAACTGCCTGTCCGGAACGGTTCA
TGAACGTGGCAGGTTTGAGCAGCCAAACGTCGCCGTCGGGCAGGGCGGCACGGGCGACTT
CGCCGAAGAATTTTTTTCTTCTTTAAATGAAGCCTTCCATTTCCACGCCAGTTCGTCGA

## Appendix A

GGAACCAAAAACCCGCATTGTGGCGTGTCTGTTCGTATTCTTTGCCCGGGTTGCCCAAGC
CGACAACCATTTTGATTGTGTTTGACATGATATTTTCCGTGTTTCTGTCGAATGCTGTCT
GAAGGCTTCAGACGGCATGGTTATTCTTCTTGATTTTGAACGCGTTTGCGGCGCGCTTCT
TTGGGGTCGATCAACAGCGGGCGGTACACTTCGATGCGGTCGCCGTCGCGCAGCGGCGTG
TCGTCTTTGACGGCTTTGCCGAAAATGCCCAAAGGCGCGGAATGCAGGTTTAAATCTTCA
AATATGCCGTCCAAACCGCTTTGCAGTGCGGCGGCGCGGACGGTTGTTCCCTCGGCAAGC
TGCATGGTTTTCAAAACCTGTCGGTCGGGCAGCCCGTACACAATCTCAATTTCAAGCATA
ACGGCGGTCTGCCTCTTTGACGAACGCTTCGACCAGCGTGGTGGAAAGGTGGTTGAAGAC
GGGGGAAATTAAGGCGGACAAAACGGCATTGGAAAAATCGTATTCCAAATTGAATTCGAT
TTTGCACATATCGTCGCCCAAATCGATAAATTTCCACGTTCCACGTAAGGTTTTGAACGG
ACCCTCGAGCAGTTCCATACGGATTTCCCTGCCCGGAATGTTGCGGTTGTGCGTGGCAAA
CGATTGGCGAACGTGCATATAATCCATAAACAGCCGCGCCTTCAGTTCGTTGCCGCTACG
CCCGATGACTTCGGTCTTGCTGTACCACGGCAGAAAGTGCGGATAGTCTTCAACCTTGTC
GACCAGCTCGAACATTTTGTCCGCGCCGTGCAGCACCAAGATGTTTTTTTCAACTTTTTT
CACGGGCATCACCAATGCGGGCGTGTATTCGAAGGGCGGTATTATAGCGGTTTAATTTTA
AACCGGTACAGCCTTGCCCCGTTCTGATTTCAATTTAAACCGCCATATCGCAGATTCGTA
AAAACCGATACGGCTTTGGCGTTTCAGACGGCATTGAGTGGAAAATGCCGTCTGAAAACG
GGATGGGAAAACGGCAGCTTGCGGGCTGCCGTTTGTTCGGGAAGTTAAGCCTTGTTTTCA
GGCTGCTGCTCGGACTCTGTCGAAGCGGTGTCTGCCGGGGCAGGTGCTTCGGTTTCTTCC
CGAGTCGGAGCGTGCGGCGCGTTATCTTCCGCCGCGTCCGCATTCTCGCGCAGGTTGTGG
CTGTAATCTTTGGGCGGGCTGGGTTGTTTGCCCGCCATGATTTCCAGTACCTGATCGCGG
TCGATGGTTTCCCATTCCATCAGGGCTTTGCACATCGTTTCCATCTTGTCGCGGTTTTCA
TCGAGGATTTTGTAGGCAACCTGATATTGCTCGTCCAAAATCCGGCGGATTTCCGCGTCG
ATGTCCTGCTGGGTTTTCTCGGAAATGTTTTGCGAACGGGTTACGCTGCGTCCCAAGAAG
ACTTCGCCTTCGTTTTCCGCATAAACCATCACGCCCATTTTGTCGCTCATGCCGTAGCGC
GTTACCATTTCGCGCGCCATTTGGGTTGCGCGTTCAAAGTCGTTTGATGCGCCGGTGGAG
ATGCGTCCGACGAAGATGTCTTCGGCAATCCGTCCGCCGAACAGGATGGAGAGCTGGCTC
AACATCTGATCTTTATACATACTGATGCGGTCGCGCTCCGGAAGCTGCCAAGTCAGACCC
AGCGCACGTCCGCGCGGCATAATGGTTACTTTGTGGACGGGGTCGGTAAAGGGCAGGCTT
TCGGCAACAATCGCGTGTCCGGATTCGTGATACGCCGTCGCACGTTTTTCGTCTTCGTGC
ATCACCATACTGCGGCGTTCCGGACCCATATAGATTTTGTCTTTGGCGTCTTCAAAATCG
CTCTGATCGACTTTGACTTTATTGCGGCGGCCGGCAAACAGGGCGGCTTCGTTGACCAAG
TTCGCCAAATCCGCGCCGGAAAAACCCGGCGTGCCGCGCGCGAGGGACAATAAATCCACA
GATTCGTCCAAAGGCACTTTTTTAGAATGGACGTTCAAAATCTGTTCGCGCCCTCGGATG
TCCGGCAGGGGGACAACCACTTGGCGGTCGAAACGGCCGGGGCGTTGCAGCGCAGGATCG
AGTACGTCGGGGCGGTTGGTTGCCGCAATCACAATTACAGTCTGATTGCTCTCAAAACCG
TCCATTTCAACCAACAATTGGTTTAATGTTTGCTCGCGCTCATCATTGCCGCCGCCCAAA
CCTGCGCCGCGTTGGCGGCCGACTGCGTCAATCTCGTCGATAAAGATGATGCAGGGGGCG
TTTTTCTTCGCCTGCTCGAACATATCGCGGACGCGGCTCGCACCGACACCGACGAACATT
TCGACAAAGTCGGAACCTGAAATGCTGAAGAACGGCACGCCGGCTTCGCCTGCAATCGCT
TTCGCCAAAAGCGTCTTACCCGTACCCGGGCTGCCCGCCAGCAGGATGCCGCGCGGCACG
CGCCCGCCCAGGCTTTGATAGCGGTTCGGCGCTTTGAGGTAATCGACGATTTCCTGTACT
TCTTCTTTGGCTTCGTCGCAGCCGGCGACATCGGCAAAGGTCACTTTGTTGGCATCTTTG
TCCAGCAGGCGGGCGCGGCTTTTACCGAATGAGAATGCGCCGCCTTTTCCGCCGCCGCCC
GTCTGCATACGCATGAAGTAGAACCATGCGCCAATCAGCAGCAGGACGGGCAGCAGGCTG
TAAAACAGGGCAGCCAGCGCGCTCGGTTTTTCTTCCGGCGTTACTTTTACGCGGACGTTT
TTGTCGAGCAGTGTTTTAATTAGGTTGTCGTCCAAAGGCGCGTTGGTGAAGAAAGTGCTT
TTGTCGGTGCGCTCGCCCTTAATCAGGTAGCCGCTGACGACGGATCCTTCGATGTTGACG
CCGGATACTTCGCCGTTGTTGACCTGTTGGATGAACTGAGAGTATTCGATTTGCCCGTTG
~~TCTTCTTTTTTACCGTCTAAAGCGTTGAACGCAGCCATCAGGCCGATACCCAAGGCGACC~~
CAGACAAGGATTGATTTAAAGGTGTTCCCCACTTAGCAAGGCTCCATAATTGAGGTGTAA
AACGGAAATGATTGTAAAGCACGCCGTCTGTATTGTCAGCGTTTATTTTTGCCCAATAAA
TAAATCTCACTGGAGCGATTGCGCGAGGCTTCGGGTTTGCGCGTCTGCACCGTGCCGAAA
ATTTCGCGCATGGCTGCCATGTATTCCTGATAGCCTGCACCCTGAAAGACTTTGACCAAA
AAGCTGCCGCCGGTTTTCAGGTGTTGCGAGGCGAAGTCTAAAGCCAGTTCGCACAGATAA
AAGCTGCGTGCCTGATCGCTTACGGCGTTTCCCGACATATTGGGCGCCATATCGCAAATT
ACAAGGTCGAGCGGGCGGTTGTCCAACAAGGTTTCGAATTGTGCCAGTACGTCGTTCTCG
CGGAAGTCGCCCTGAATGAAGGAGACGCCCCCTATGGCTTCCATAGGCAGGATGTCCAAG
GCGAAAACTGCTCCGGAAGTACCCGTCAGCTTGGCGGCAACCTGCGACCAGCTTCCCGGC
GCGCTGCCCAAGTCGGCAAGTACCGTGCCGGGTTTGATTAATTTGTCTTTTTCGTTGATT
TCCAAAAGTTTGTATGCGGCACGGGCGCGGTAGCCGTCTTTTTGCGCCATATGGACGTAG
TGGTCGTTGACGTGTTCGTGCAGCCACGCTTTTGAGGATTTGGAACGTACAGCCATAGTG
GTTCGCGGGTCGGAATGGAAACCGCGTATTGTACGTTAATTTTGCCGATGTCGTGCCAAA
TCGCGTACAATGCCGCATTATTCTTTTTATTCAAGCAGTAGGAAAATGACGGATACCAAA
TTGAACACCAAAGAAATTTTGGAACTGAAAGCGCGCGCGCACCATCTCCATCCTGTTGTG
ATGGTCGGTCAGCAGGGTCTGACGGACGCGGTCATCAAGGAAACCGATGCGGCATTGACG
GCGCATGAGCTGATTAAAGTGCGCGTATTCGGCGACGACCGTGCCGAACGTATCGAAATC
TGCACTGCCTTATGTGAGGCGGTTGATGCGCAACTGGTTCAGCATATCGGAAAACTTTTG
GTATTGTGGCGTAAGAATATCGAAGCCTGACAGCCTGAAGCAGTTGTTTTGCTATTGTTC
TTTAACGGGCGGGACGCCGTCCTTCGGCGCGGCATTTCGGCGGGCCGAAACCCTTTCCGG
TGAAAACGGATTTTGATTGCCGCCCGATGCTGTCTGCAAGTTGCGGCGGCTTCCGTATGG
TTTGAATTGTTGACAGGATGATTGGAGGGCTTATGCAGTTTCCTTACCGCAATGTTCCGG
CTTCGCGTATGCGCCGTATGCGCAGGGACGATTTTTCACGCCGCCTGATGCGCGAACACA
CGCTGACCGCCGATGATTTGATTTATCCGGTGTTCGTATTGGAGGGGTCGGCGCGCGAGG
AGGATGTGCCTTCTATGCCGGGTGTGAAGCGTCAAAGTTTGGACAGGCTGCTGTTTACGG

## Appendix A

CGGAAGAGGCGGTAAAGCTCGGTATTCCGATGTTGGCACTGTTCCCCGTGGTTACGGCAA
ACAAAACCGAGCGTGCGCAGGAGGCGTACAATCCCGAAGGACTCGTGCCGTCAACTGTCC
GCGCCTTGCGCGAGAGGTTTCCCGAACTGGGCATTATGACGGATGTCGCGCTCGATCCTT
ATACGGTTCACGGTCAGGACGGGCTGACGGACGAAAACGGTTATGTGATGAACGATGAAA
CCGTAGAGGTTTTGGTCAAGCAGGCTTTGTGCCACGCTGAAGCGGGCGCGCAGGTGGTTG
CCCCTTCCGATATGATGGACGGGCGTATCGGTGCGATTCGCGAGGCGTTGGAGGATGCCG
GGCATATCCATACGCGGATTATGGCGTATTCCGCCAAATATGCTTCTGCATTTTACGGCC
CTTTCCGTGATGCGGTAGGCAGTTCGGGCAATTTGGGCAAGGCAGATAAAAAGACCTACC
AGATGGATCCGGCAAATACCGATGAGGCGTTGCACGAAGTGGCGTTGGACATTCAGGAAG
GTGCGGATATGGTAATGGTCAAGCCCGGTTTGCCGTATTTGGACGTTGTCCGCCGCGTAA
AGGACGAGTTCGGTGTGCCGACTTATGCCTATCAGGTTTCGGGAGAATACGCGATGTTGC
AGGCAGCGATTGCCAACGGCTGGCTGGACGGCGGCAAAGTGGTTTTGGAAAGCCTGCTGG
CATTCAAACGTGCGGGTGCGGACGGGATTTTGACCTATTACGCTATTGAGGCGGCAAAGA
TGTTGAAGCGTTGATTTTCGGCCGGGTTAATTGAAATGCCGTCTGAAACCATGGTTTCAG
ACGGCATTTTTACAGTTTACAAAGTTGTATCGAGTGCGGCGGAAATATCGTTCCAAATAT
CGTCCGCGTCTTCGATGCCGATGGAGAAACGCAGCAGACCGACTTTGATGCCCATTTCCA
TTTTCACATCATGCGGTACGCCGCTGTGGGACTGGGAATAGCAATGGTTGACCAAACTTT
CCACACCGCCGAGGCTGGAAGCCATTTTGACCAGTTTCATGTTTTTAATCACGCTGTTTG
CCGCTTCACGCGTGTCGTTTTTGAGATAAACCGTAACCACGCCGCCGATGCCTTTGGGCA
TTTGTGTTTTCGCCAGTTCGTAATGTTCGTGAGACGGCAGGCCGGGATGGAACACTTTTT
CAATGGCAGGATGGGCTTCCAAACGGCGCGCGATTTCGAGTGCGTTTTGGCAATGGGCGT
TCATGCGCAGAGCCAGTGTTTTGATGCCGCGCAACACCAGCCAGCAGTCCAGCGGGCCGG
CAACCGCGCCGGTATGCACCATCATATCGTGCAAAGGCTGCGCCAGTTCTTTGGTTTTGG
CAACGACGATGCCCATCAACACGTCGGAATGGCCGCACAAATATTTGGTAGCGGAATGGA
ATACAAAATCGCAACCCATATCCAACGGCTGTTGCAGATACGGCGTGGCAAAAGTGTTGT
CGATACCGACCAGCGCACCGGCTGCTTTGGCTTTTGCGGCAAGGACTTTGATGTCTACCA
AGCGTAAAAGTGGATTGGACGGCGTTTCCAGCCAAACCAGTTTGACCTTGTGCGCTTTAA
GCAGTTCGTCCAAATTATCCGGATTGCCTAAATCGGCAAAAACAACGTTCACCCCCCATT
TTTGATAAACATCGACCAATAAATCATAAGCGCCGCCGTAAATATCGGCGACGGCGACAA
TGGTATCGCCCGGGCGCAGGAAAGTGCGCCATACGGCATCAATTCCCGCCATACCGCTGG
AAAACGCAAAACCTGCCGCACCGTGTTCCAAATCGGCAACGGTGTCTTCTAAAATCTGAC
GGGTCGGGTTGCTCAGGCGCGAATAACGGTAAGGCACATTTTCGCCAATCTCGTGCAACG
CAAACATACTGTTTTGATAAATCGGCGGCATCAGCGCGCGGTTGTGTTCGTCGCAATCGT
AGCTGGAATGAATGGCTTTCGTGGCGAATTTCATTTGGTCTCTGCCTATGTAGATGTGAA
TAATCGAAGATTTTATCACTATCTGAAAAATAGAAACAATCAATGCAGCTGCTAAAAGCG
AGTGATATAATCTCGCATTTGCAGATTGACGGTATATTCCCCGGCGGAAACGCCATACCA
TGCACACATCTCAACAATTACATGAATATTAAGGAAAAACAACTCATGAACACTATTGCA
GACGTGCAATCCAGCCGAGATTTGCGCAATCTGCCGATCAATCAGGTCGGTATCAAAGAC
CTGCGCTTTCCGATTACCCTGCAAACTGCAGAAGGCATCCAGTCCACCATTGCCCGTCTG
ACCATGACGGTTTACCTGCCTGCTGAGCAGAAGGGGACGCATATGTCGCGTTTTGTCGCA
TTGATGGAGCAACATGCCGAAGCCTTGGATTTTGCACAATTGCGCAAGCTGACTACCGAA
ATGGTCGCGCTTTTAGATTCCCGCGCCGGCAAAATCAGCGTTTCTTTCCCATTTTTCCGC
AAGAAAACCGCCCCGGTTTCCGGTATTCGGTCCTTACTGGATTATGATGTCTGTCTCACG
GGCGAAATCAAAGACGGGGCATACGGCCACAGTATGAAGGTCATGATTCCCGTAACCTCG
CTTTGCCCGTGTTCCAAAGAAATTTCCCAATACGGCGCGCACAACCAGCGTTCGCACGTT
ACCGTCAGCCTGACTGCCGATGCCGAAGTCGGTATCGAGGAAGTCATCGATTATGTGGAG
GCGCAGGCGAGCTGCCAACTCTACGGCCTGCTCAAACGCCCCGATGAAAAATACGTTACC
GAAAAAGCCTACGAAAACCCGAAATTCGTGGAAGATATGGTGCGCGATGTCGCTACTTCG
CTGATTGCCGACAAACGCATCAAGAGTTTCGTCGTCGAGAGCGAGAATTTCGAGTCTATC
~~CACAACCATTCGGCTTATGCCTATATCGCCTACCCGTAGGCGCGTTTGCGATGAACCAAA~~
TGCCGTCTGAAAGGCGTTTGGGCGTTATTGGCGAATCTGCCGCCGTATCGGAAATCAATT
TGCAATACAAGTAATAAAAGGATGCACGATGACAGTATTAAGCAAAGAGCAGGTTCTATC
CGCATTTAAAAACCGTAAATCATGCCGGCATTACGATGCGGCACGCAAAATCAGTGCCGA
GGATTTTCAGTTTATTTTAGAACTCGGGCGTTTGTCGCCCAGTTCGGTCGGTTCGGAGCC
TTGGCAGTTTATTGTGGTTCAAAACCCTGAAATCCGACAGGCAATCAAGCCGTTTTCTTG
GGGTATGGCGGATGCTTTGGATACCGCCAGTCATTTGGTGGTGTTTTTGGCGAAGAAAAA
TGCCCGCTCCGACAGCCCGTTTATGTTGGAAAGCCTCAAACGGCGCGGCGTTACCGAACC
GGATGCCGTAGCAAAATCTTTGGCAAGGTATCAGGCGTTTCAAGCTGACGACATCAAGAT
TTTGGACGATTCTCGCGCCTTGTTTGACTGGTGTTGCCGTCAGACCTATATCGCGTTAGC
CAACATGATGACGGGTGCGGCGATGGCAGGTATCGATTCCTGCCCGGTGGAAGGTTTCAA
CTATGCCGAGATGGAGCGCATATTGTCCGGGCAGTTTGGTTTGTTCGATGCGGCAGAATG
GGGCGTGTCCGTCGCCGCGACATTCGGCTACCGCGTTCAGGAAATCGCCACGAAAGCGCG
TAGGCCCTTGGAAGAAACCGTTATTTGGGCATAAGGCAATGCCGTCTGAAAACGCAAGGA
TTTTCAGACGGCATTTTTTAATGCTTGGCGGATTCGCATTTGAAGTGCAACTTTCCCTAA
CAGAAAAAGGCCAGTATGCGGTAGCATACGGCCTTTCCTGCAAGAAAGATTGCCATGAGC
CACACGCAACTGACCCAAGGCGAACGATACCACATCCAATACCTGTCCCGCCACTGCACC
GTCACCGAAATCGCCAAACAGCTGAACCGCCACAAAAGCACCATCAGCCGCGAAATCAGA
CGGCACCGCACCCAAGGGCAGCAATACAGCGCCGAAAAAGCCCAGCGGCAAAGCCGGACT
ATCAAACAGCGTAAGCGACAACCCTATAAGCTCGATTCGCAGCTGATTCAGCACATCGAC
CCCCTTATCCGCCGCAAACTCAGTCCCGAACAAGTATGCGCCTACCTGTGCAAACACCAC
CAGATCACGCTCCACCACAGCACCATTTACCGCTACCTTCGCCAAGACAAAAGCAACGGC
AGCACGTTGTGGCAACATCTCAGAATATGCAGCAAACCCTACCGCAAACGCTACGGCAGC
ACATGGACCAGAGGCAAAGTACCCAACCGTGTCGGCATAGAAAACCGACCCGCTATCGTC
GACCAGAAATCCCGTATCGGCGATTGGGAAGCCGACACCATTGTCGGCAAAGGACAGAAA
AGCGCATTATTGACCTTGGTCGAACGCGTTACCCGCTACACCATCATCTGCAAATTGGAT

## Appendix A

AGCCTCAAAGCCGAAGACACTGCCCGGGCAGCTGTTAGGGCATTAAAGGCACATAAAGAC
AGGGTGCACACCATTACCATGGATAACGGCAAAGAGTTCTACCAACACACCAAAATAACC
AAAGCATTGAAAGCGGAGACTTATTTTTGTCGCCCTTACCATTCTTGGGAGAAAGGGCTG
AATGAGAACACCAACGGACTCATCCGGCAATACTTCCCCAAACAAACCGATTTCCGTAAC
ATCAGTGATCGGGAGATACGCAGGGTTCAAGATGAGTTGAACCACCGACCAAGAAAAACA
CTTGGCTACGAAACGCCAAGTGTTTTATTCTTGAATCTGTTCCAACCACTAATACACTAG
TGTTGCACTTGAAATCCGAATCCAAGCTTATTTAAAACGATTCGCCGGGAGCGAGATAAC
GCCATTTGCCGGGCGGCAGCCTGCCGAGTTTGACCTTGCCCATGCGGATGCGTTTCAGCC
CGACGACGCGCAGTCCGACCAGTTCGCACATACGGCGGATTTGCCGCTTTTTACCCTGTT
TCAACACGAAGCGCAGTTGGTCTTCGTTTTGCCATTCTACTTGGGCGGACGCAGTTTCT
CGCCGTCCAAACTCAATCCGTGATTCAGTAAGGCAAGTCCTTTTTCGTCCAATTTGCCGC
GCACGCGCACCAAATATTCTTTTTCACTGCCGCTGTTTTCGCCGATAAGCTGCTTGGCGA
TACGGCCGTCCTGAGTCAATACCAGCAATCCGACCGAGTCGATGTCCAGCCTGCCGGCGG
GGGCGAGGCCGATTTTGTGTTTCGGATCGAAACGGATGCGGCCGGTATCGCCTTCCCAGT
GATTTTCAGGGGTAATCAGTTCGGCGGCGGATTTATAGCCTTTTTCCGCTTGTGCGCTGA
CATAGCCGACGGGTTTGTTCAACAGGATGGTAATGCGTGCCGCCTGCTGTTCGTGGGCTT
TCTTGTTCAGTTCGATACGGTCTGCCGGTGAAACTTTCTGACCGAGTACGGCGGTTTTGC
CGTTGACCGTTACCCAACCCTGTTCGATATAGCCGTCGGCTTCGCGGCGTGAACAAAGCC
CCAGTTGCGCCATGCGTTTGGAGAGGCGCACGGCATCTTCTGTATGGTCGGAGGAAATTT
TGGGATTCATGGATACTTTCGGGTAAAGGCCGGCTTAGACTAATTGCTCGCCCCAGTGCA
GTTTTTGGCGCAGGGTTTTGAAATATTGGTAGTCGGTCGGGTGCAAAATGCGTAAGGGAT
TTCGGTAGCGGCGGATGGTGATGCGGTCGAGGTTTTGCACGTCGATATGGGTTTGACCGT
CGAAATGGACGCGCGCGTCGCCGCCTTGGGTAACGAGGATTTCGATTTCGGACGTGTCTG
GAATGGCGATGGGGCGGTTGGTCATGGATTGTGGGCAGATGGGGACGAGCGTGAAGGCGT
GTAATCCTGCCTGCATGATGGGGCCGCCGGCGGCAAGCGAATAGGCGGTCGATCCGGTGG
GGGTGGAGACAATCAGCCCGTCCGAACGCTGGGTATAGACGAATTCCCGATTGACGAAGA
CTTCAAACTCAATCATCTGTCCGGCACCGCCACGGGAGAGGACGGCATCGTTGAGGGCGA
TGGCGCGTTCGGCGGTTTTGCCTTCGCGGATGAGTGCGGCCTCAATCAGGATGCGCTCTT
CGGCAAGGTATTTCCCTTCTAAAACGGGCAATAGCTTGTCCGTCATATATTCGCGGGGAA
TTTGGGTCAGGAAGCCCAAATGCCCTTGGTTGATGCCGATAATCGGAACGGCGCGCAGGG
CGATTTCGCGGGCGACGGAGAGAAAGGTGCCGTCTCCGCCTAAAACGGCGACCAGGTCGC
AGTATTGCCCCAGTTCGGTCTTGTTGACGATATGGCAGCCGACGGTGTCTTGGGTATAGA
TGCAGCCTTCCTTTATGCCGACTTCGTCGAGATAGACGGTAAAGCCGTGCTGCTTCAAAA
AGGTAATCAGCGTGTGTGCGGTGTCTTGGATGTCGGGCGTGTTGGGGCGGGTTACGATGC
CGATGTTGTGAAAAGGGCTGTTCATGTCGGATGCCGTCTGAAGGTTAGTCTATCCAAATG
TCGCGTTCGAGCCGGTCGAGGCGTTCGTTGAGGCGTTCCACGCCGTCGCGCAGTCTGCTT
ATTTCGTCGAGGCAGTCGGCAAGGGCTTCGTTGCCGATGTTTGCGGACTCGGATTCGCGG
GAAAATCCGCCGATTTGTTCGGCGATGTTCCTGCCGATTTGTTTGATGCCGTGTCCGATG
TCGGCGGCACGGCTGCCGATGTCTGCCTGCGTGCCGAAAATCCGTGCCAATTCGTCCGAT
GCGCGGGAACGCAGGCTGCCGAGCAGGGACAGTACCGCGATGCCGAGGATGAGGTCGCCT
TCGAGCCCGATGTCGCCCGCCCCGGGTTCGCCTCCTTGGAGGATTTTCTGTACCGCGCTG
TTGCGGAAGGTAATTTCGGTGTCTGCAAAGCCGTTTCCCGCCGAGAGCAAACCGTCTTCC
GTGATGCGTCCCGCCAGTTTCAGCCCGGCAATGTTCAGGGTCAGTGTTTTGCCTGCAAAG
GCGGCAAGTTCCGAGCGGCTGTCCGGGCTTTGCAGAATCAGGCGGTTGATGATGGGGAGG
AGGGCGGACATATTTTCTGATCGGGGCGGAGAATGCCTGTTTGTTGCTGTTGCCGCTTAT
TTTACAGGCTTAAGCCGTTATCGCAAACGGTACGGATGATTTTGCCCACGCTGTCGTCGG
GTGCAAAATCCGGCGCGGGCAAGCCGAGTTTGGCGGCTTCTTCCGTATAGAATTCGCGTC
GTGTCGGGTGGCGCGGTTCGATAATGTTTTTCAGCCGCCTGCCGCCGGGGTTAAATGCCG
TCTGAAACAGGCTTTCGACGGCGATATTACGGTGGACGATGTTGATGGGGCGGTTGCCGC
CCGGGATGTTTTGCTTTTGAACAAGGCGGCCGACGGGATGGCGTTCGGCGCAATAAAGCC
CGCCCAGCCGCAGGATGTCGATGTTCGGAACGCCGCTGTCGAGCAGGTGTTGTTCGGCGG
CGAGGATTTGGCGGGCGGACTCGGTTTGCGGATCGGGTAGGGCGATTTCGTCGCATTCGC
GCGCTGTATCGCCGTAAACGCTGGTACTGCTTGTGAAAATCAGGTGTTGCACGTTGCACG
CCCGGGCAAGTTCTGCCCATTGTTTGACGGTATCGGCGTAATGTGTCAGCGATGATGGCG
GCAAGAGGCAGAACCAAACGGGTTTGTTGGCATGGTGCCGCCAAAAGCTTGTATCTCGGG
CAAGGTTCGCGCTTTGAAACGCGCTGTCTTGATTGAGGTCGATGGTATCGAGGTGTATGG
GCAGATTGATATCGTCCGAAGTCAGGCTGCGTTTGACGGCGGCAACGCGGCTGCCGTGTT
GGTAAAACTTTTGTGCCAGCGGCAGGCCGAGGTAACCTAGGCCTGTGATGGAGATATGGG
GCGGGGGGACTGCGCGCATTCGCTGATACCGTCGGGTAAGTGCCGTCTGAAGGCTGATTC
GGACGCTGTGGGTTTACGGGTTGCCGTTGCCGATTTTCCGGTCGTATTTCTTGCGGTGTT
CGGGCAAAAGATAAGGACGAAGGAGGCTTAAGGTGCGGCGGATGCCGCGTGCTTTGGAGT
CTTCGGTCAGCTTGGTGCGGCCGCGCAGGGAGCTGTCGAAGTCGAACCAGTATTTCGTGA
CCATCCACATATTGACGGCGAGATCGTTCATGGCGGTTTGGTCGGCTTGGATGATGTTCA
GACCGTTGAGTTGGGTGAGCAGGTTGACCAAGAGCGGGGAGACTTTGGCTTGGGTGAAGG
TATTGTGTTCGCCCAACAATTCGGCACTGCGTGCAAGCAGGGTGTTCACGTCGCTGAATA
GGAAGCGGTATTCCCACATCACATCATAAATACCGGCCATATAATTGATGGAGTCTTCCA
CATCAGACGGCAACACGGCTTCATTCAGGTATGCCAGCAGGGCTTCGCTGTAACGTTTGA
ACAGTTGGACGATGATTTCGTCTTTGTTGCGGAAGTGGTAATAGAGGTTGCCCGGACTGA
TGCCCAAGTGGGCGGCAATATGGTTGGTGCTGATGTTGCGCTCGCCTTCCTCGTTGAAAA
GCGCAAGGCTGGCGTCGATGATGCGTGTGTAAGTATTGATTTTGGCGGGGCGGGTCACGG
GCATACTCCTTGGATTTACAGGCTGAACGAAGCAGGCAGCCAATTAGGTTCGGCGTGCAA
TTCTACCTGAAACCGAGCAAATACTGTAAATTTGATGTGTTGCGCCAACTGCCGGACATC
GTTTGCCGAGGCGTTGTTTTTGTTCACTAAGACCAAAGCCTGCCTGTCATGTACCGCCGC
GCCGCCGATTTGGAAGCCTTTCAGACGGCATTGGTCGATCAGCCAGCCGGCGGCGAGTTT
GACCGAACCGTCGGGCTGCGGATAGCGCGGCATATCAGGATGCCGCTGCAACAAGGTGGC

## Appendix A

GGCTTTTTCTGCGCTGACGACGGGGTTTTTAAAGAAACTGCCGACATTGCCAAGCACGTT
AGGATTAGGAAGTTTACTGTTGCGGATTGCACACACTGCATCGGAAACATCTTTCGCCGT
CGGGACCCTGCCCGCGCTCAGTTCGGCAACGGCGGCCGCCAAATCGCCGTAACCCAAAGT
CGGCACAAAATGCGTTTTTAATGCAAATACGACCGAAACAATCACATAACGCCCTTTACC
CTCCTGCTTGAACAGGCTTTCGCGGTAGGCGAAGCGGCAGTCGGCATTGGCAAGCTCGAC
AAAGGTCTCCGTATCCAAATCAAAGCAGCGCACGCTGTGAATCACGTCTTTCGCCTCCAC
GCCGTATGCGCCGATGTTCTGCACGGGCGATGCGCCGACCGTACCCGGAATCAGGCTCAG
GTTTTCCAAACCGCTCAAACCCAGCGCAACGGTGTGCAGGACAAAATCGTGCCAAATTTC
GCCCGCCTGCGCTTCAATCAGAACCATGCCGTCTGAACGCGCAATCTCGCGTATGCCTTT
GTTTTCCATGTGTACGACCAGTCCGGCGTAATCCTGCATCAAAAGGATGTTGCTGCCGCC
GCCCAGCCATAAAACAGTATCGCGGTCGAACTCCAGCAGTCGGACGATGTCGCGCAACTC
GTCGGCATGTTCGAGCGCGATAAAGGCCCGGGCTTGGGCGCGAAGACCGAAGGTGTTGTA
GGGGGTAAGGTCGGTTCGGTATCGGATGGGTTGCATGGTTTGAACTTTAACTGTATTTGA
ATTGAAGTGTACTGCGTTTTCAGACGGCCTTATGCGATCTGACCATCTCCCTACTGCACA
ATTCCAAGCACCACACCAAGGCGATGGCGGCGATGGTGAGCGAGGCAATCAATGCCGTCC
AGAAGCCGTAAATGCCCATATTGAAACGGTAGGCGAGCAGATAGCCCGGCAGCAGGCCGC
AGCCCCAAAAGGCGGCGGCGTGGATGAACATCGGCACCTTTGTAACTTTGTAGCCGCGCA
AGGCGTAGGAGGCGATACATTGGGTGAAGTCTGCCGGTTGGAACAAGCCGGCGAACAGTA
AGACGGTGGCGGCGATGCTTAAAACCGCCGGATCATTGTTGTACATACTTACCAGCGGCG
AACGGAATAATACCAAGGAAAGCACGGTAATCACGGCGAGCATCCATCCTAACACCAGTG
ACACGCCCGAAATATAACGCGCCCGCGAAAATTCGCGCCGCCCAAGCGAAAAGCCGATGC
GCACCGTCCCCGCCGAGCCGACGCTTTGCGGAATCATATAGAGAATCCCCGACAAACTGA
TGCCGACCTGCTGCGCCGCCACATAATCCTCGCCGAAAGGCGCAATCAAAAACACGATAA
ACGAAAACGCGCTGGCTTCCAAAAAATAAGACAGCCCGATGGGTGCGCCGATTTTCCAAA
TCTGTTTGAACACCGCCCAATCCGGTTTGCCGAATTTCGCCGTCAGTCCGAATGGGCGGA
AGAAATTTTCCTTGGCGATATAAATCCACAATGCCAGCGCGCTGAACCAAAACACCGCCA
TCGTCGCCAGTCCGCAGCCTGCGCCGCCCAAAGCGGGCATACCGAATTTGCCGTAAACGA
AAATATAGTTCAGCGGCACGTTCAACACAAACGCCGCAAAGCTGACCAACATAATCAGGC
GCGGGCGGTTCAGGCTGGAAGTGTAGGCGTGCAGCGCGCGGTGTACCATTGCCGCCGGCA
TCGCCAAGCTGGTGAACAACATATACTGCGCCATCGTGCCTTCCACATAATCGCTCAAGG
TCAGCCAGTTGCGGAACGGCGTAATCGCCGCCCACATCAAGACCATGCCGAACACGCCCA
AAAACAGCCCGAACCAAATCCCCTGCCGCCCCGTTTCGCCCACTTCGTCGGTTTTACCCG
CGCCGTAAAGCTGGGCAATCATCGGGTTCAGCGCCGCCATAATGCCCATAAAGGTAATAT
AAACCGTGGCAAACGCGCTGCTGCCCAAAGCCACCGCCGCCAAGTCTTCCTTGCCCGCAC
CGCCCGCCATCACAGTATCGACAAAACCGATGCCCACCTGCGCGACCTGCGCCAACAGCA
TGGGCAGGGCAAGAGTGGTCAGCAGGCGGACTTCTTTCAGGAAGACGGGAAAGGAAAAGC
GGTTGAGGTCGAGCAGCATAAGTGTTCAATCAACAAAAATGCCGTCTGAAGCAGAAAACG
GCAGCAGGAAGTGATGAGAAATAATGGTGCACATTATATCGTAAAAAAATGCCGTGCCGT
CAGACGGCGGATACAGGGTATAAAAGTATATTCAGATTGTGTGTATTTTATGGTAAAGTT
TGGTTTTAACGACTTGACGGCATTGAGCCGTCGGACAGGGGCTGTTCGGATTCTGAATCG
GAAAGAAGCACCGCCGTTTTGACAGCGGCGTGATGCGTTGCGGCAAAGATGCCGTCTGAT
TGCGGATCGGGCAGTCTTTTGTGTTTACAGGATAAAATAGAAGGCAGATTCTCATGCAGA
CATGGCAGCTTCCGGAACATATCGCCGACGTACTGCCCACGAACGCGCGGCAGCTTGAAA
GCGCGAGGGAGCAGTTGTTGGCACTGTTCCGCGTACACGGTTATGAACTGGTACAGCCTC
CGCTGATGGAGTACGCACATTCCCTGCTGACGCATATCGATGCGGGGCTTTCCCTGAAAA
CCATTTTGGTAACGGACAGGCTCAGCGGCAGGCAGTTGGGCATACGCGCCGACATCACGC
CGCAGGTGGCGCGTATCGATGCCCATCTTTTATCCGCCAACCAAGGGATTAACCGGTTGT
GTTATGCCGGTCCGGTGTTGCACGCGCAGCCCGACGGTCTGCTGAATATGCGCGAACCCT
TGCAGGCAGGGGCAGAAATGTACGGTTTTGCTGACATCCGTGGCGACATCGAGCTGATAG
ACCTGATGCTGAAAAGCATGAAAATTGCCGATATGGGCAAAGTGCTGCTTTCGCTGGGGC
ATATCGGCATATTTCGCGCCTTGTCCGATGCGGCACATTTGGATGCGGGGCAGTCCGCAA
CGCTGCTTGCCTTGATGCAGGATAAAGATACCGGGGCGGTCGAAGCGCAGGTCAAGGCTT
GGAAGCTGGACGGCATGTGGGCAAAAGCATTCTCGCTGTTGCCGCGCCTGTACGGCGGGC
GTGAAGTGTTGTCCGACGCGCGGACGGTTGCCGGATTTGTCGGCGGTCGGCGGCGCGT
TGGGCGAATTGCAGGCGGTGTGCGACGCATTCCCCGATTGTGAAATCCATATCGACTTGT
CCGAGCTGCGTGTCGACAATTACCACACGGGCTTGCTGTATGCCGCCTATGCCGCCGATT
TCCACGACGCGGTCGCGCGCGGCGGGCGTTATGACGGATTGGGCGGATATTTCGGTAGGG
CGCGCCCGGCAACGGGATTCAGTTTCGACTTGCGCAGCTTTATCGGGCGTTTGCCCGCCA
TCGAACGGCAGCCCGCCGTGTTGGTCGATGCGGAAGATGCCGAAGCGGCGCACGAAGCGG
TCGAAGCCTTGCGTGAACAAGGGCAGTGTGTCGTAATCGATTACGGTATCGGACACAATG
TTTCGGAAGAGCTTGCAGGCCGTCTGAAAAAGACGGACGGCGTTTGGCAGGTCGTGAAAC
GCTAAATACCCGTTCATGGCGGATGAAAGGCAAATCGTGGCGGGGCGCAAAGCCGCACCG
GTTTGGGGATTTCCGCAATAATTTTTAATATCGATAGGTTATATGGCTATGGCTAAAAAT
GTTGTAGTAATCGGCGCACAGTGGGGCGACGAGGGTAAAGGTAAAATCGTTGACTGGCTG
GCGGAAGAAGCCGGCGGCGTGGTGCGCTTCCAAGGCGGCCACAATGCGGGCCATACCTTG
GTTGTCGGCGGCAAAAAAACCATTTTGCGCCTGATTCCGAGCGGCATCCTGCATGAAAGT
TTGGACTGCTTCATCGGTTCGGGCGTTGTCGTCTCCCCCGAAGCCCTGTTGGGCGAAATC
GACGAGTTGAACGCGGCAGGCGTGAAAAACGTCGAAGGCCGTCTGAAAATCGCGCCGACC
TGCCCGCTGATCCTGCCTTACCACATCGCGCTCGACCAAGCCCGCGAAGCATCGCGCGGC
AAAGGCAAAATCGGCACGACCGGCCGCGGCATCGGCCCTGCCTACGAAGACAAAGTGGCA
CGCCGCGCCATTCGCGCCGCCGATTTGCTGCATCCTGAAAAACTGCGTGAAAAACTGGAT
GCCGTCCTTGCCTATTACAATGTCCAACTGCAACATCTGCACAATGCCGAGCCGGTTAAA
GCGGAAGACGTGATGGCGGTTATCGAAAAAGTCGCGCCGCGCATTGCGCCGATGATTACC
GACGTGTCCCGCGTGTTGAACGAGAAAAACAAAAACGGCGAAAAACTGCTGTTTGAAGGC
GCGCAAGGTGCGTTGTTGGACATCGACTACGGCACTTATCCCTTCGTTACCTCGTCCAAC

## Appendix A

TGTCTGGCGGGCGCAGCTTCGGCAGGCGCGGGCGTAGGTCCTCAAATGCTGGATTATGTT
TTGGGCATCGTCAAAGCCTATACCACGCGCGTCGGTTCGGGCCCGTTCCCGACCGAATTG
TTCGACGAAGTAGGCGTAGGTTTGGCAGAACGCGGACACGAATTCGGTTCGGTAACCGGA
CGCGCACGCCGCTGCGGCTGGTTTGATGCCGCCGCCCTGAAACGCTCCATCCAAATCAAC
GGCATTTCCGGTATGTGTATTACTAAACTCGATGTAATGGACGGCGTTGAAACCATCAAT
ATCTGCGTCGGCTATGAATTGCCCGACGGCGGCAAAACCGACATCCTGCCTTGCGGTTCC
GATGCGGTGGAAACCTGCAAGCCGATTTACGAAACCATGCCCGGCTGGCGCGAATCCACT
TTCGGCGTGAAGGACTACGGCGCATTGCCCGAAAACGCCAAAGCATATTTGAAACGGATT
GAAGAAGTCTGCGGCGCGCCGGTCGCCATCGTCTCCACCGGCCCCGACCGAGAAGAAACG
ATTGTGCTGCATCATCCGTTCGCATAAGGTTTTGCAGTAAAATTGCCGTCTGAAGCCCTA
ATCCGCCGTTGGTCATAAATAGTAGGGTATCAACATTTCGGGCTACAATGGTACGTCAGC
CAATGCCAAGACGTGCCAGCCTGATTTGTTGATGTGTGTACTATTGCATAGGCGGTTAAG
CCATGCAGGAGATGAAAGTGTATATGTCGCGCAAAGCCCATTAGCCGCAAGCGAGGGGCG
GGGTGCGCGAGAGAGAGGGGGGCAGGTGCTAAGTCATTGGTTATCTCCTTACTATCTATC
TAACTACGGGTGCATTATGCGCCTATGCCTGTTTTTTGTCAAACACTATACAGTTAAAAT
GTGTAAATATTTAGTAAGGACATATACCCTTTTCTTTACATTTAGCTCCATCGGATACCA
GTGCGTTCAGAGAAAATTTGAAAATTTCTATATTTTGGTTGTATAATGCTTTCATTTTAG
AAAGGTCTATAATGCCAAAATCACTCTCGCTACAAGATGTTCAGTTAAGGTTTTCCAGTA
AATTTCCTGATAAGACGGTTTTGAAGTTCACTAAAAACTACGAACCGGTAACAATCCAAT
GCCCTTTGCATGGGTCGGTTGTTTACGGGAATCTACAAGCTGCCATGAAATCCTCAACAG
GATGTCCTGAATGTACTCGAAATCCCGAAAAGCCCTCTCCAAACGCCGTTGCCATTAGGT
TGGAGGACACAGAGACAGGGGAAATCTACGAGTTTGCAAGTACACTGGCTGCCAGTAAAT
TTATCGGTTGCTCCAACAGCACTTTAGCTGTACGTTTAAGCGGACGCACTCCGTTTGACC
GATTGATTAGGTATCGTTATAGGTTGGTAAAGTAAGTTCACAACCACTATGGCACTTACA
TTTACTCAAGCAGTTTCTAAGCTAACCTCTAAATTTCCACATTTGAATCTTGTGGAGTTC
AATGGCGTTCGTTACCCGACGGTAATCGTCTGTCCTATACACGGGAGGGTTACTTGCTCT
ACATTCAAAAGTATGTTGGACTCTAAAAGTGGGTGTCCAAAATGTGCATCTTATGGTGTC
AATTCCCACAAAATTCCAGAAGATACAATAGATAAATTATCTAAAAATACAGTATTGGAG
GATACTGTAACTGGCGAAACACTTACATTCCCCTCAAGAGCATCTGCTGCAAGGTCATTG
GGTATAAACCCAGCAGCTATCACTGACCGTATAAAAGGTCGGGTTCACACAGAGACTTTA
CTTGCAGGGAGGTATAAGGTTCACATCTGCACTAAATGACGTATACACTTTTTAACAGTG
TATACCCCTCGCCACTATCAGGTGCTTCTCGAAAAATTTGACATTTTTATAAATTTGCTA
TTAAATCCATTTGACAATCAATTTTGGAGTCTCAAATGGCCAAATCTTTCAACCAAGCAG
CTTCTGAACTTACTGATATATTCCCTAATATCTCTCTAACCGGCTTTGACGGTGTGAATT
ACCCAGTAACCGTTAATTGTCCTATGCACGGGAACGTTCGTTATTCGACATTTAATGCCC
TTATAAAGTCAAAGTACGGGTGTCCTGAGTGTGCTAAGATGTCAAAAACCCAAACACCTC
CAAATGTAGGGAAGCCCCTCCTCATCCTCGACACAACGACCAACGAAACACTCACGTTCC
CAAGCGTTACTGCCGCAGGTGCTGCTTTAGGTGTACATTTCCAACAAATCAATCACAGGC
TCAAGGGTCGAACGTCGCCCGACAACCTTATTTCAAACAGGTACAAAGTGCTTGGGTACG
ATAGGTAAGGTTCACAACACCCTAAATCCTACGAACCAAGCTCGACGGACGTTCAAATCC
CGGTTAGAGTTAATTTATGTAAAGATTTAGTAAAGACGTATACCATTTTTCTTTACATTG
TGCTTGCGCAGGATTTCAGGTAGGTCTCAAAAAATTTGAAAATTTCTATATTTTGGTTGT
GTAATCCATTTGCACATAACCTATGGAGACAAATTATGGGTAAGCGAATGACTTTCGATA
CCGCCAAATCACGCTTTCAAGAGAAATTTCCACATTTAGAATTGTTGGAGTTCAGTGGCA
TTTATAAACCTTCCAGTGTTAGATGTCCTACGCACGGGGTTGTCCAACTTTTGTATTACG
ACACAGCTATAAAGTCAAAGTATGGGTGTCCGGAGTGCGGGAAACTTAAAATGAAGGAAA
ATACGCCTCCCCAAAACCAAAAACCTGTCTCCATCCTCGACACCGCCACAGGCGAAACAC
TCACGTTCCCCAGCGTACAAGCTGCCGCCAAAGCCCTAAACACCCCCTACGGCTCTATAC
GAACCAAGCTCGACGGACGTTCAAATCCCGACAACCTTGTCTGTAACAGGTATAAGGTTC
TGCTATAATCAACCTATGGAAAATATTGAAGAATACGCACTGCTGTCTCCCGAAGCCCTG
CTGGAACGCCTGGATACCGTTTTGAGTATCAGAATCGGCGGCAAGGGTTGGGAATCCAGT
TATGACCGCCAACTTTGCACAGACGCTGGTCGAAATACAGGACAGTCTGTACAGGGTTGT
GTCAACCGTCCAATACGGGGATGACAACCTCAAGCGGTTGACAGCGGACAAACGGAAGCA
GTATGAGTTGAACTTCAAGATTTCCGAGGGTTCTACGCGTGTAGAGTCCGACTTTAAAGA
GACTTTGGTTCGGTTCGGTAGAGATATGCTTCAAGATATGCCCCCTAAAATCCGTTCGGC
AACGCTGGTAGCGTTGACGACCCTGCTTGTCGGAGGGGCGTTGGGTTACGGTTATTTGGA
ATACCTGAAGCAGGTTGCTTCGGAAGGGTATCAGACCGAGCGTCTGTATAATGCCGTCGA
CCGTCTTGCAGAATCCCAAGAACGGATAACGTCCGCCATCCTGAAGGGTGCTAGAGGTGC
CGATTTCGTGCAAATCGGCAGACGTTCCTACAGTAGGGAGGATATATCGGAGGCAAATAG
ACGTGCAGAGCGTGTCCCGTATGGCGCAGAGTTGGTTTCAGACGGCAATTTTACCGCTGT
TTTATCTGATATAGGGGATTAACAAAAATCAGGACAAGGCGACGAAGCCGCAGACAGTAT
AAATAGTACGGAACCGATTCACTTGGTGCTTCAGCACCTTAGAGAATCGTTTTCTTTGAG
CTAAGGCGAGGCAACGCCGTACTGGTTTTTGTTAATCCACTATATTTCTGTCCGGATAC
GGTTTATCAGGGTATATCAATGCGGCGTATCCGGTGCGGAAATGGATACGGTTGGTGTCG
GTATGGAAACCTGATGTTTTCAGACAGCATATACAAAAAACCGTACTGCTTGCGCGTACG
GTTTTTGCCGTTTCAAATCGGTTTAAAGCGATTTGAGGCGGGCGATACGGTTGTCCAGCG
AAGGGTGGGTGCTGAGCAGGGAGTCGCGCGTATCTCCGGCGATGCCCATTGCGTTCATTT
CTTCGGGCAAATCGACCGGGTTGCCTTTGAGCCTTTGCAGGGCGGAAATCATTTTCGGCG
CGCCGACCAGTTTTGCCGCGCCCGCATCGGCGCGGTATTCGCGTTGTCGGCTGAACCACA
TGACAATTAAGCTGGCAAGGAAGCCGAACAGGATTTGGAATACCATGCTGACCAGGAAAT
AAGTTCCCTGGGACTGGCTGCCGTCGTTGTTTCGGGCAATCAGGTTGGCAATAATGCGCG
ACAGGAACACGACAAAGGTATTGACCACGCCTTGAATCAGCGTCAGCGTAACCATATCGC
CGTTGCCGACGTGTGCCATTTCGTGCGCCAATACGGCTTCCACTTCGTCACGCGTCATAT
GGTCGAGCAAACCGGTGCTGACGGCGATCAGGGAGCTGTTTCTCGATGCGCCCGTGGCAA
AGGCATTGGGTTCGGGGGAGTGGTAGATGGCGACTTCGGGCGTTTTCAGGTTCCATTGCC

## Appendix A

GCGCTTGGGCTTCGACAGTGTTCAAAAGCCAGGCTTCTTCTTCGGTGCGCGGCGTGTCGA
TAACTTCCGCGCCGACCGATTGTTTGGCGATAAATTTGGACATCAGCAGCGAAATAATCG
AACCAGTGAAGCCGACGACGGCGGAATACGCCAACAGGCTGCCCGTGCCGCCCCGGCTGT
TGATGCCCAAAACCGCCAAAACAATGTTGATTACGACCAAAACAGCGATATTGGTAGCCA
AAAACAGAAAAATTCGTTTCACGGATGTTCCTTTTTGGTAGGGTGTGATGTTTTGAAATT
TTGGGGGATTGTCCCAAAAAGTTGCCGGCTTGTGAATATCAGACTCGGCAAAGGTATGCA
AAACATTTGCTTGCAAATGGCAGTTTGTGCAGTTGGTTTTTGAACTATTGTGCCAAGCCG
TGTAGAATCGTAAACCATCTGTTTGATTCCAATAAACACATTTCAAAGGATCACTTCATG
AAAGCATTACTTTTAGGCGCGCCGGGCGCGGGCAAAGGCACTCAGGCGCAATTCATCACC
GCAGCGTTCGGCATTCCGCAAATCTCTACCGGCGACATGCTCCGTGCCGCGATTAAGGCA
GGCACGCCCTTGGGTTTGGAAGCGAAAAAAATCATTGACGAAGGCGGCTTGGTGCGCGAC
GACATCATTATCGGCATGGTCAAAGAACGCATCGCGCAAGACGACTGCAAAAACGGTTTC
TTGTTTGACGGTTTCCCGCGCACATTGGCACAAGCCGAAGCGATGGTTGAAGCAGGCGTG
GATTTGGATGCAGTCGTTGAAATCGATGTGCCTGACAGCGTGATTGTCGACCGCATGAGC
GGCCGCCGCGTGCATTTGGCTTCCGGCCGTACTTACCACGTTACCTACAACCCGCCCAAA
GTTGAAGGCAAAGACGACGTAACCGGCGAAGATTTGATTCAGCGCGACGACGACAAAGAA
GAAACCGTGAAAAAACGCCTTGCCGTTTACCACGAGCAAACCGAAGTTTTGGTCGATTTT
TACAGCAAACTGGAAGGCGAACACGCGCCTAAATACATCAAAGTTGACGGCACCCAAGCA
GTAGAAGCCGTGAAAGCCGAAGTATTGGGCGCATTGGGCAAATAAATCGAAAAGGTCGTA
CCCACGGGCAGGCTTCGCACTCTGAAAACAGAAAATCAGGTTTTCAGACGACCTGTTTTT
GATAAACAGCGTGTTGCAACCGAAAAATAATCATTTGGCGTCATTCCCGCGCAGGCGGGA
ATCCATTTCTGAATTTGGGCAATCGCTGTTTAAATCTGATGAACTGAGTTTTATCAATGG
ATTCCCGCCTGCGCGGGAATGACGGCTGATGTACCGGTTCAAATTTATCCGAAACAGTTT
GTCGGAGGCTTGAGTCCGCGTAGGTCGGACATCAATGCCCGACCTACGGTTTGAATTTAC
GTTGTTATAGTGGATTAACAAAAATCAGGACAAGGCGACGAAGCCGCAGGCAGTACAGAT
AGTACGGAACCGATTCACTTTGTGCTTCAGCACCTTAGAGAAGCGTTCTCTTTGAACTAA
GGCGAGACAACGCCGTACCGGTTTAAAGTTAATCCACTATACTGCGAAAAAGACGATAAA
GGTCGTCTGAAAACCCGAAACGAAAACACCATGAATCCTTTAATCTCCGACTTCCAAACT
CCGCAACAACGCACCCCCGTTATCGTCGCCCTTGATTTTTCCAACGAAAAAGACACGCTC
GGATTCGTCCGCAACCTTGACCCGACATTGTGTCAAATCAAAATCGGCAAAGAGCTGTTT
ACCGCGACGGGACGCAATTTGGCAGAAAGCTTAATCAATCAAGGTTTCAAACTTTTCCTC
GATTTGAAATACCACGATATTCCCCACACCGTCGCGCAAGCCTGCAAAGTCGCTGCCGAT
ATGGGCGTTTGGATGGTCGATATGCACGCATCGGGCGGCCGCCGTATGATGGAAGCCGCA
GCAGAAGCCGTTGCCGGATACGGCACGAAGCCGCTCTTAATCGGCGTAACCGTGTTGACC
AGCATGGAACAAAGTGATTTGGCGGAAATCGGTTTGAACACCGCCCCTGAAGAACAAGTC
ATCCGCTTGGCAAAACTGGCGCAAAGTTCGGGCTTGGACGGCGTGGTCTGTTCCGCCCAA
GAAGCCGCGCCGCTGCGCCGCGAATTGGGACAGGATTTTGTCTTGGTCACGCCCGGCATC
CGCTTGGACGTTGCCGGCAATAATGATGACCAGCGCCGCATCATGACACCGGCCGAAGCC
TTGGCTGCCGGTTCGACTTATTTGGTAATGGGTCGTCCTGTAACCCAAGCTGCCGATCCG
GTAGCCGTATTGCGCGAAGTGAACCGCGTGGCAAACCTTGAAGCAAACTGATTTTCAGAC
GGCCTTACAGGCTGAGGCCGTCTGAAAAAATACAACGGAGGCAATATGTCCGCCAAGTTC
CAACAAGAAACCCTCAAATCCCGTTTCGCGCAAGCCAAAGTCCTGGTTGTCGGCGACGTG
ATGCTCGACCGCTATTGGTTCGGCGATGTGTCCCGTATTTCGCCCGAAGCCCCCGTGCCG
GTGGCGAAAATCGGACGAATCGACCAACGCGCGGGCGGAGCGGCAAATGTCGCGCGCAAC
ATCGCTTCGTTGGGCGGCAGGGCAGGGCTGTTGTCCGTAACCGGCAACGACGAAGCCGCC
GACGCGCTCGATGCGCTGATGGTGCAGGACGGCGTCGCCTCCTATCTGATGCGCGACAAA
CAAATCGCCACCACCGTCAAACTGCGCGTCGTCGCCCGCAACCAGCAGCTTATCCGTCTT
GATTTTGAAGAACATCCCAACTGCGAAGTGTTGGAACAAATCAAGCAGAAATACCGCGAA
ATCTTGCCCGAATACGACGCAATCATTTTTTCAGACTACGGCAAAGGCGGCCTGTCGCAT
ATCTCCGATATGATCGATTGGGCGAAACACGCCGGCAAAACCGTCTTAATCGACCCCAAA
GGCGACGATTACGAAAAATATGTCGGTGCAACTCTGATTACGCCTAACCGCGCCGAATTG
AAAGAAGTGGTCGGCAGTTGGAAAAACGAAAGCGAGCTGACCGAAAAAGCGCAAAACCTG
CGCCGCCACCTCGACCTGACCGCCGTTTTACTGACCCGAAGCGAAGAAGGCATGACCTTG
TTCAGCGAAGGCGAACCGATTTACCAGCCCACCCGCGCCCAAGAAGTTTACGACGTATCC
GGTGCGGGCGACACCGTCATTGCCGGAATGGGCTTGGGTTTGGCGGCAGGCTGCACCATG
CCCGAAGCCATGTACCTTGCCAATACTGCGGCCGGGGTTGTCGTGGCGAAACTCGGTACG
GCGGTTTGCTCGTTTGCCGAATTGATCAAGGCATTGTCAGGGCAATCAACAATGTAGTTT
TCATATTGATAAGATAAACAGAACGATATAAGTATGACTATTTCGACAATGGCACAGACA
CACGATACTCGATTACAAAAAACTTTGCTCTTTCCCAGTCAACAAGGTTGGAAATCTAAA
ACTTTTCTTAAACCTGATTCACATATTAGATTAGCAACCGTATTCAGCGGGATTGGTGCG
GTTGAACAGGCATTCCACCGATTAAATTTAAACCATACCATTGTTTTTTCAGGAGATATT
GATCCATACGTTAAAAAAAGTTATCTTGGAAACTATAAATTAAATGAAGATTTTTGGCAT
AACGACATTACTCAATTTGATGCGAGAAAGTTTAGAAATCAAGTTGATATTTTAGTTGGA
GGCAGTCCTTGCCAAGCATTTTCCATGGTTGGCAAACGTGCAGGATTAGAAGATACACGA
GGAACATTATTCTATGAATTTGCTCGCGTTGTGGATGAAGTCCAACCAAAGATATTTATT
TATGAAAATGTAAAGGGCTTGCTTAATCATGATAATGGAAAAACTTGGAAAGTTGTAAAA
AGTGTTTTTTATTCACTTGGTTATGACTTATATTTCCAAATAATGAATAGTAAGGATTAT
GGGATTCCTCAACATCGTGAGCGTATTTTTGTTGTTGGCTTTCATACCCCTCCTATAAAT
GGTTTTCAGTTTCCTGAAAAGATTGAATTAGAACATACTATGCAAGATTTTTTGGAGGAC
TATACTGATAGCAAATATTTTTTACGTGAAAAGGGTGCGAAATTTGTTACCAGTTCTAAA
AATAGACAAAAACGTTATACACAGATTAATGGAGAAATTGCCTTATGCCAAAAAGCAAAT
CAACAATTTAATTGGCATGGTGATTTTATTTTTCAGGCAGCCCGCGAATCTGAATTTGAT
GACTTTATTTTTGATGTAAATAACGTTGAGGAAAAATATTATCTTTCTGAAAAAATCAAA
AATTATGTTTTGGCAGGAGGAACAAAAAATTTTAAAACCAGTACGGAAACTGATTTGCCT
GTAGCTCGCCCATTATTGCAAACTATGCATAAAATGCATCGTGCCGGGGTTGATAATTAT

## Appendix A

GTTACTCATAATCGTGGACGTATTCGTAAATTAACACCTAGGGAATGTTTGCGGCTAATG
GGTTTTAGAGATGATTTTAAAATTTTAGTTAGTGATACTCAAATGTATCGCCAAGCGGGA
AACAGTATTGTTGTTGATGTATTAATCGCTATATTAAAACAAATGGATATTACGCTTTAT
GGAGAGTAAAATGCCGGATTTTCAAAGAATTACTATTGAAAACATAGAGTATTTTATTGT
CGATAGTATTCAAAATTTACGTGCGGAAGATAGTTTTATTCACAGGAATAACAAGTTATC
TGTTTTTGGTGGTAATGGGGAAGCACGCAAATATGTAGGTAGTTATATTGATGATAGTGG
TCGAAAACTAAGTACATTTTTTGAGTATGAAAATTGGGGGATTACTGAGACAAAAAATGG
AAGGAGGGTAGAATATCCTTTCATTCAGAAAAATTGTTTTTTTATAAGAAAAATTTACA
GCGTTATTTGGTTGATGCAAAAGTTGAATATCACAAACAAGAACAGAAATATCATCATGA
TATTTCAAGGTTTTATGATGATTACGTTTTAAGTATTAATAATCTGCCTACAGAAAATAT
TTTCTTTTCAATCCATGATGTATCAGACCATTTGGAAAATTCTAAAGGTTACCGTAGGGG
TTATATCCGTTCGGAGGATGATATATGGTCAATTTGGCGTAAGATTGTATTGCCCAAAAT
TAGTTATTTATCAATTCTTAAACTATTGCCTGTCAAAGATATAGAAGATTCAGAACCATT
ATTTTATTTCCGAATATTTTTGGATTATCAATTTCGCTCTATCGTGCACCCGCAACTCTT
ATCAAGAGAAAAATTAGAAATACCGGCTTCAGAATTTAATCAATTCGTAGAGCAAGAAAT
CATTAAACAGAAAAGTAGAAAAGGGCAGCAGAAATATCGCAAGGATGTTATAAATCATAT
GTCGCAATGTCCATTTACATTAATTACAGATGAGATTTTGTTAAGGGCTAGTCATATTAA
ACCTTATATGGTTTGTATTACTGAGAAAAATGAGAAAGAGGCATTAGATTATTTAAATGG
GTTAGCTTTAACGCCGACTTATGATTGGTTGTTCGATCAAGGTTATATTACTTTCTTGGA
TGATGGTCGTTTGATTTGCGGTACTCGACTAAGCCGTTACACATGGGAAAAACTTAATAT
CAATCCAAATGCAAAAAATTAAGATGAGAATATATCCCGAAGGAAGAGAAAAATATTTGG
AATATCATCGCAAATTTGTGTTTCAGGACAATATCGATGATTTCTTGTAACTAAGTTTAT
TAATTTTACTCAAAACAAAGGAAACCGAATATGACCATCATCGTAACAGGCGCGGCCGGC
TTTATCGGCAGCAACATCGTCAAAGCACTTAATCAACGCGGTATTACTGACATTGTTGCC
GTCGATAATTTGAGCAAAGGCGAAAAATTCAAAAACCTTGCCGAGTGCGAAATCGCCCAT
TATCTCGACAAACACGAATTCATCCGCCAAGTGAGGGAACACATTTTACCTTATCAAAAC
ATCGAAGCCGTTTTCCATCAAGGCGCGTGTTCCGATACGATGAACCACGACGGTTTGTAT
ATGATGGACAACAACTACCAGTACACGCTGGATTTGCTGGACTGGTGTCAGGACGAACGC
ATCCCCTTCCTTTATGCCTCCAGTGCGGCGGTTTACGGCAAAGGAGAAATCTTCCGCGAA
GAGCGCGAACTCGAAAAACCGCTCAACGTGTACGGCTACTCCAAATTCCTGTTCGACCAA
GTATTGCGTCGCCGCATGAAAGAAGGTCTCACCGCCCAAGTCGTCGGCTTCCGCTACTTC
AATGTTTACGGACAACACGAACAACACAAAGGCCGCATGGCATCCGTCGCCTTCCACCAC
TTCCACCAATACCGCGAACACGGTTACGTCAACCTGTTCGGCAGTAACGACGGCTACGGC
AACGGCGAACAAACCCGCGACTTCGTCAGCGTCGAAGACGTTGCCAAAGTCAACCTCTAC
TTCTTCGACCATCCCGAACTTTCCGGCATCTACAACCTCGGTACCGGCCGCAGCCAACAG
TTCAACGAACTCGCCGCCGCCACCGTCAACGCATGCCGCGCCGCCGAAGGCAAACCTGAA
ATGAGCTTGAAAGAGTTGGTAGAAGAAGAACTTATCCGCTACATTCCCTTCCCCGACGCG
CTCAAAGGCAAATACCAAAGCTTCACCCAAGCCGACATCACCAAATTGCGCGAAGCCGGA
TATAAGGAAGAATTTTTCGATGTCAAATCAGGCGTCGACCGCTACGTCAAATGGATGCTG
GAAAATTTGGCTTAATTTGAATGCCCGTAAAAAAATCGTCTGAAAATATCAGGCGATTTT
GATTTGTTTAACTTTTATATGGATTTCGATGATGACCGAAATGCAACAACGCGCCCAACT
GCACCGCCAAATTTGGAAAATTGCCGACGAAGTACGCGGCGCGGTGGATGGCTGGGACTT
TAAACAATACGTTCTCGGCACACTTTTCTACCGCTTTATCAGCGAAAACTTCACCGACTA
TATGCAGGCAGGCGACAGCAGTATTGATTACGCCGCTATGCCGGACAGCATCATCACGCC
CGAAATCAAAGACGATGCCGTCAAAGTTAAAGGCTATTTCATCTACCCCGGCCAGCTTTT
TTGCAATATTGCCGCCGAAGCCCATCAAAACGAAGAGCTCAACACCAAGCTGAAAGAAAT
TTTTACCGCGATTGAAAGCTCCGCCTCCGGCTATCCGTCCGAACAGGACATCAAAGGCCT
GTTTGACGACTTCGACACCACCAGCAGCCGGCTCGGCAGCACTGTTGCCGACAAGAACAA
ACGCCTTGCCGCCGTCCTCAAAGGCGTGGCGGAACTCGATTTCGGCAATTTTGAAAACCA
CCACATCGACCTTTTCGGCGATGCCTACGAATACCTGATTTCCAACTACGCTGCCAACGC
AGGCAAATCCGGCGGCGAATTTTTCACCCCGCAAAGCGTATCCAAGCTGATTGCGCGGCT
GGCGGTGCACGGACAGGAGAAAGTCAACAAAATCTACGACCCAGCTTGCGGCTCGGGCAG
TCTGCTCTTGCAGGCGAAAAAACAGTTTGACGAGCACATCATCGAAGAAGGCTTCTTCGG
GCAGGAAATCAACCACACCACCTACAACCTCGCCCGCATGAACATGTTCCTGCACAACGT
CAATTACAACCAATTCCACATCGAATTGGGCGACACACTGACCAACCCAAAGCTCAAAGA
CAGCAAACCCTTTGATGCCATCGTTTCCAATCCGCCTTATTCCATCAACTGGATAGGCAG
CGACGACCCCACCTTAATCAACGACGACCGCTTTGCCCCCGCAGGCGTACTTGCCCCGAA
ATCCAAAGCCGATTTTGCCTTCATCCTGCACGCACTGAACTACCTTTCCGGCAGAGGCCG
CGCCGCCATCGTCTCATTCCCCGGCATTTTCTATCGCGGCGGCGCAGAACAGAAAATCCG
CCAATATCTGGTGGAGGGCAACTACGTGGAAACCGTGATTGCCCTTGCGCCCAATCTCTT
TTACGGCACCGGCATCGCCGTCAATATCCTGGTTTTGTCCAAACACAAAGACAATACCGA
CATCCAATTCATCGACGCAAGCGGCTTCTTTAAAAAAGAAACCAACAACAACGTCTTAAT
CGAAGAACACATTGCTGAAATCGTCAAACTCTTCGCCGATAAAGCCGATGTGCCGCATAT
CGCCCAAAACGCTGCCCAGCAAACCGTCAAAGACAACGGCTACAACCTCGCCGTCAGCAG
CTATGTCGAAGCCGAAGACACACGCGAAATTATCGACATCAAACAGCTCAACGCCGAAAT
CGGCGAAACCGTCGCCAAAATCGAACGGCTGCGGCGTGAAATTGACGAAGTGATTGCAGA
GATTGAAGCATGAGCATCATCCTATACACCGCCAACGACGGCACTGCCCAATTTGCCTTG
CAGGAATTTGGCGGACAGCTTTGGCTGACGCAGGCGGACATGGCAGAACTGTACCAAACC
ACCAAACAAAATATCAGCAAACACATTAAAACCATTCTTGCAGAGCAAGAATTGGAAGAG
AAGGCAACTGTCAACTTCCAGTTGACAGTTCAAAATGAAAACGGGCGCAAGGTAAACCGC
AAAATCGCCCATTATTCCCTGCCCATGATTATTGCCGTCGGCTACCGCGTCCGTTCCGCG
CGGGGCATCCAATTCCGCCAATGGGCAACCGAACGGCTGGACGAATATCTGACCAAAGGC
TTTGCCATAGACGACGAACGCCTGAAAGGCACAGGCGGCGGCGACTATTGGAAAGAACTG
CTCAACCGCATTCGCGACATCCGCAGCAGCGAAAAAGCCCTATACCGGCAAGTGCTTGAT
TTATATGCCACCAGCCAAGACTACAACCCCAAAAGCAGCGAAAGCCAAACCTTTTTGCC

## Appendix A

GCCGTTCAAAACAAACTGCACTATGCCGCCAGCCGGCAAACCGCAGCTGAGCTGATATAC
AGCCGTGCCGACAGCAGCAAAGACTTTATGGGGCTGACCACCTTTCAAGGCGCAATCCCC
ACGCTGAATGAAGCCAAAATCGCCAAAAACTATCTGACCGAAGACGAACTGTTCCGCCTG
AACCGTCTGGTTTCCGCCTTCTTCGACCTAGCGGAAATCAAAGCGCAGGAGCAAAGCCCC
ATGTATATGCGCGACTGGATAGCCGAATTGGACAAATTTTCCGGGCTGTACGGACAAGGC
ACATTACAGGGTGCAGGCAGCATCAGCCGCAAACAGGCAGAGCAGAAAGCCGAACGCGAA
TACCGCGCCTATGAAGCGCGCATCCTGTCGCCGGTGGAGCAAGCCTATCTGGAAAGCGTT
AAAGCGTTGGAAAAAACAGCCGTGCAACAGATCAAACAGAAAAAAGACCGCACAAAATAA
CTACCATTCAGGCTACCTGAAAAAGCAGCCTGCACACCATTGCAGGCAGCCTGAAAGCAG
GACGGACTTCAGCCCGCAGAAATAACGGCAAACGGACAGAGTGAGCCGAAGCACCCCGCA
ACTGCCCCACATCCCGCCGCAACGGGAAAGAAACGGAAAACAACCATGGATATGCAAAAC
AAAGCGAAAAAATTGATTGAGATGATTCAGACGGCACCGGTGGAGTGGAAGCCGTTGGGG
GAAGTGGCGAAAGTATTAAGAGGAAAACGTTTGACAAAGAAAGAGCTAATTGAAGGTGGG
AAATATCCGGTTTTTCATGGCGGATTGATTCCTCTTGGTTGGTTTGACCAGTTTAATCGT
AGAGCTAATCAAACGATGATTATTAATACGGGAAGTATTGGTGAAGTTATATGGAGTGGC
GTAGATTTCTGGTCATCTGATGGTACTTTTGTGATTCAAACACCAAACTATCTTGATGAT
AAGTTTATATTCTACTTTTTAAAAACAAGAGAAGGATATATAAAATCCCAAAAGAGAGTT
GGTGGAGTTCCTACTATTGATAGATTAGTAGTTGAAAATATTTCGATCCCCATCCCACCC
CTGGAAACCCAACAAAAAATTGTAAAAATACTTGACAAATTCACAGAGCTGTAAGCTACG
CTGGAAGCTACGCTGGAAGCGGAATTAACCCTGCGCAAACGCCAATACCGGTATTACCGC
GACTTTCTTTTAGATTTTAACAATCAAATCGGGGGGGATAGCTGATGGCTATAAAGGCCG
TCTGAAAGATGTGGTTTGGAAGACGTTGGGGGAGGTATTTAATATTTTTGCTGGAGGCGA
CGTACCAAAAGACGCTTTCTCTGAAGTGGAAACGGAAGAATTTTGTATCCCCATTTTATC
TAATGGTATAGGAAGTAAGGCACTATATGGCTGGACTAATGTTGCTAAAATCAATCAACC
TAGCTTAACTATATCAGCTAGAGGAACTATAGGTTGGGCTAGCTTTCAGAATAAACCTTT
TTTCCCAATAGTACGCCTGTTAGTGTTAACACCAAAAATTGAATTAAACCTAAAATATGC
CTACTACTTTATGAAAAGTATTGAATCAAATTATAAAGTTCCTGAAAGCGGTATTCCACA
GCTAACGAAACCAATGATAAAAGATATTTCAATCCCCATCCCTCCACTCCCCGAACAGGA
AAAAATCGTCGCCATCCTCGACAAATTCGACACCCTGACCCACTCCATCAGCGAGGGCCT
ACCGTACGAAATTGCCCTGCGCCGGAAACAATACGAATATTACCGCGGGCAGTTGTTGAG
CTTCCCAAAGGCTGCCTGAAAAGTCATAGCTGGTCTTTAAATCATGCCGTCTGAAAAATA
TTGATAAGGAAATATCATGGGAAAAAGTTTAACCGAAATTGCTGAGGAACTAAAAGGAAA
CGATAAAAAAGTCCAGCTAATCTATGCTTTTAACGGAACAGGGAAAACACGTTTGTCCAG
AGAGTTTAAGAATTTAATTGCTCCAACCAGTTCAGAAGAGCCAGACGGAGAGCCAACAAG
AAGAAAATTTCTCTACTATAATGCATTTACTGAGGATTTATTCTTTTGGGACAATGATTT
GTTAGCGAACGAAGCTCCAAGATTAAAAATTCAAAAGAATAGTTTTACCGACTGGTTGCT
TAGGGATAATGGACTGGATGGAGCTGTTATTAAAAACTTTCAATATTATACAGATGATAA
GTTGACTCCTGATTTTAATGATGATTTTTCAGAAATTGCATTTTATTTTGCTCGTGGTAA
TGATGAGCAGATTGAAAATATCAAAATTTCCAAAGGTGAAGAAAGTAATTTTATTTGGAG
CATTTTCTATGTATTAATCAGACAAGTCATCGCTGAATTGAATATTCCAGAAGATAGCGA
AGAAGGACGTTCCACAGATCAGTTTGACGATTTGGAATATATTTTTATTGATGACCCTGT
CAGCTCTTTGGATGAAAATCATCTGATTCAGCAAGCGGTTGATTTGGCTGATTTGATAAA
GCTTAGCAAACCGAGGTTAAAGTTTATCATTACTACACATAATGTTTTATTTTACAACGT
TCTATACAATGAACTAAAAAAATTAGAAAAGGAAAAGAAAAGTTATCTTCTGTTAAAAAA
TGAAGATGGTAGTTTTGATATTCTTGAAAAACAAGGTGATTCCAATAAAAGTTTTTCATA
TCACCTTCACTTAAAAGGAGTTATTGAAAAAGCTATCGAGAATCAGCAGGTAGAACGGTT
TCATTTTATGTTGCTGAGAAACCTGTATGAAAAAACAGCTAATTTTTTAGGCTATAAGCA
AAGGTCTGATATTTTGCCCGAAGACAGCAGACGAAACTATTTTCAACGTATTATTAACTT
TACAAGTCATTCTACATTATCTAATGAGGCATTTGCCGAGCCAACACCACAAGAACAAGA
AACTGTCAAATTGCTTTTGCAACACTTGCTGGATAACTATAATTTTTTTCAAGATGATGA
ACAAAGAGATAAGCCATGAACCTCGAAACCAAACCCATCGCTGAAACGCCGAATTTCATC
GTGCTCGACCAATATGAAAAAATCGAACAGTCGGGCAGCTACCAATCGGAAAACCGGTTG
GAAGCGGAGTTAATCGCCGATTTGCAGAATCAGGGTTACGAATACCGCAAGGATTTGAAC
AGCCAAAGCAGGCTGCTGGAAAACCTGCGCGCGCAGTTGCAGCGGCTGAACGATGTGGCG
TTTTCAGACGGCGAATGGGCGCGGTTTTTGACGGAATATCTGGACAGGCCGTCTGAAAAC
ATTACCGATAAAACCCGCAAAATCCACGACGACCATATTTACGATTTCGCTTTTGATGAC
GGTCCTTGGATTCGGATTTCAAGTGCAACACTAGTGTATTAGTGGTTGGAACAGATTCAA
GAATAAAACACTTGGCGTTTCGTAGCCAAGTGTTTTTCTTGGTCGGTGGTTCAACTCATC
TTGAACCCTGCGTATCTCCCGATCACTGATGTTACGGAAATCGGTTTGTTTGGGGAAGTA
TTGCCGGATGAGTCCGTTGGTGTTCTCATTCAGCCCTTTCTCCCAAGAATGGTAAGGGCG
ACAAAAATAAGTCTCCGCTTTCAATGCTTTGGTTATTTTGGTGTGTTGGTAGAACTCTTT
GCCGTTATCCATGGTAATGGTGTGCACCCTGTCTTTATGTGCCTTTAATGCCCTAACAGC
TGCCCGGGCAGTGTCTTCGGCTTTGAGGCTATCCAATTTGCAGATGATGGTGTAGCGGGT
AACGCGTTCGACCAAGGTCAATAATGCGCTTTTCTGTCCTTTGCCGACAATGGTGTCGGC
TTCCCAATCGCCGATACGGGATTTCTGGTCGACGATAGCGGGTCGGTTTTCTATGCCGAC
ACGGTTGGGTACTTTGCCTCTGGTCCATGTGCTGCCGTAGCGTTTGCGGTAGGGTTTGCT
GCATATTCTGAGATGTTGCCACAACGTGCTGCCGTTGCTTTTGTCTTGGCGAAGGTAGCG
GTAAATGGTGCTGTGGTGGAGCGTGATCTGGTGGTGTTTGCACAGGTAGGCGCATACTTG
TTCGGGACTGAGTTTGCGGCGGATAAGGGTGTCGATGTGCTGAATCAGCTGCGAATCGAG
CTTATAGGGTTGTCGCTTACGCTGTTTGATAGTCCGGCTTTGCCGCTGGGCTTTTTCGGC
GCTGTATTGCTGCCCTTGGGTGCGGTGCCGTCTGATTTCGCGGCTGATGGTGCTTTTGTG
GCGGTTCAGCTGTTTGGCGATTTCGGTGACGGTGCAGTGGCGGGACAGGTATTGGATGTG
GTATCGTTCGCCTTGGGTCAGTTGCGTGTAGCTCATGGCAATCTTTCTTGCAGGAAAGGT
CGTATGCTACCGCATACTGGCCTTTTTCTGTTAGGGAAAGTTGCACTTCAAATGCGAATC
CGCCGTCGTCTGAAAAACATTTATCTGCTGGACAAGAAAAACCTTGCCCGCAACCATGTG

## Appendix A

CAGGTTATCAACCAGTTTGAGCAGACGGGCACGCATGCAAACCGCTATGACGTTACCGTG
TTGGTAAACGGCCTGCCGCTGGTGCAGATTGAATTGAAAAAGCGCGGTGTGGCGGTGCGC
GAGGCATTCAATCAGGTGCACCGTTACAGCAAAGAGAGCTTCAACAGCGAAAATTCGCTG
TTCAAATTCCTGCAAATCTTCGTGATTTCCAACGGCACGGACACGCGCTATTTCGCCAAC
ACCACCAAGCGCGACAAAAACAGCTTCGATTTCACGATGAATTGGGCGCGGTCGGACAAT
CATCCGATTAAGGATTTGAAAGACTTTACCGCCACGTTCCTGCAGAAAAGCGTATTGCTG
GGCGTTTTGCTGCATTACAGCGTGTTCGATGCGAATGATACGCTGCTGATTATGCGGCCG
TATCAGATTGCCGCCGCCGAACGCATTTTGTGGAAAATCAACAGCTCGGCGCAGGCGAAG
AATTGGAGCAAACCGGAAAGCGGCGGCTATGTCTGGCACACCACGGGCAGCGGCAAAACG
CTGACCAGCTTTAAGGCGGCGCGTCTGGCGACGGAATCGGCATTTATCGACAAGGTTTTC
TTCGTGGTGGACAGGAAGGATTTGGACTATCAGACGATGAAGGAATACCAACGTTTTTCG
CCCGACAGCGTGAACGGTTCGGAAAGCACGGCAGGCTTGAAACGCAATTTGGAAAAAGAC
GACAACAAAATCATCGTTACCACCATCCAAAAGCTGAACAACCTGATGAAGGGCGAAGAT
AATCTGCCGGTTTACCATCAGCGAGTTGTCTTTATTTTCGACGAATGCCACCGCTCGCAA
TTCGGCGAAGCGCAAAAAAACCTGAAAAAGAAATTTAAAAAATTCTGCCAGTTCGGCTTT
ACCGGCACGCCGATTTTTCCCGAAAACGCTTTGGGCGCGGAAACCACGGCGGGCGTGTTC
GGGCGGGAGCTGCATTCTTATGTGATTACCGATGCCATCCGCGATGAAAAAGTATTGAAA
TTCAAAGTGGATTACAACGACGTGCGCCCGCAGTTCAAAGCCGTGGAAGCGGAACAGGAC
GAGAAGAAACTGAGTGCCGCCGAAAACCACAAAGCCCTGCTGCACCCTGAACGCATCCGC
GAAATCACGCAATATATCCTGAATCAGTTCAGGCAGAAAACGCACCGGCTGAATGCGGGT
GGCAAAGGCTTTAACGCGATGTTTGCCGTCAGCAGCGTGGATGCGGCGAAGTGCTATTAC
GAAGCGTTCAAAACACAACAGGCAGGCAGCTTGCACCCGCTGAAAGTGGCCACCATTTTT
TCCTTTGCGGCCAACGAAGAGCAAAACGCCGTCGGTGAAATTGTCGATGAGACTTTTGAA
CCGGAAGCGATGGACAGCAGCGCAAAAGAATTTTTGCAGGCTGCCATCAACGATTACAAC
GCCTGTTTCAAAACCAATTTCGGCACGGACAGCAAAGCCTTTCAAAACTACTACCGAGAT
TTGGCAAAACGGGTGAAAAATCAGGAAATAGATTTGCTGATTGTGGTCGGCATGTTTTTG
ACGGGTTTTGACGCGCCGACGCTGAACACGTTGTTCGTCGATAAAAACCTGCGCTATCAC
GGCCTGATGCAGGCGTTTTCGCGCACCAACCGCATTTACGATGCCACCAAAACCTTCGGC
AATATTGTCTGCTTCCGCGATTTGGAGCAGGCAACCATTGATGCGATTACCTTGTTTGGC
GACAAAAACACCAAAAACGTGGTGCTGGAAAAAAGTTACGAAGAATACATGAACGGCTAT
ACCGACAGCCAGACCGGCGAAGCACGGCGCGGTTATCTGGATGTGGCAAAAGAATTGCGC
GAGCGTTTCCCCGATCCCGACAAAATCGAAACGGAAAAAGACAAAAAAGATTTTGCCAAA
CTCTTCGGCGAATACCTGCGGGCGGAAAACGTATTGCAGAACTACGATGAATTTGCCGCG
CTGCGCGAGTTGCAGAGTGTGGACGCGGCGGACGAAGATGCGATGAAGGCGTTTCAAGAA
AAATACTACCTGAGCGATGAAGACGTGCAGGAAATGCGGCAAGTGCCGATGCCGTCTGAA
AGGGCGGTGCAGGACTACCGTTCCGCCTACAATGACATCCGCGACTGGCTGCGCCGCCAA
AAAGCAGGCGAACAGAAAGAGCAATCAAAAATCGACTGGGACGATGTGGTTTTTGAGGTG
GATTTGCTCAAATCACAGGAAATCAATCTGGATTACATCCTGCAACTGGTTTTCGAACAC
CACAAAAAAATCAAAGGCAAAGCGGAGCTGGTGGAAGAAATCCGCCGCATCATCCGCGCC
AGCATCGGCCACCGCGCCAAAGAGGGTCTGATTGTGGATTTCATCAACGATACGGATTTG
GACAAAGTACCCGACGTTCCCGCCATACTGGAAACCTTCTACACCTACGCGCAAGAGGTG
ATGCGGCACGAAGCGGCAGGATTGATTGCCGCCGAAGGCCTGAACGAAACCGCCGCCAAA
CGCTATTTAATCAGCTCGCTCAAACGCGGCTATGCCAGCGAAAACGGCACGGAACTGACC
GAAACCCTGCCGAAAATGAGTCCGCTCAACCCGCAATATCTGACGAAGAAACAAAGTGTT
TTTCAAAAGATTGCGGCGTTTGTGGAGAAGTTTGCCGGAATAGGGACCGATATTTGACAA
AATGCCGTCTGAAATTTCAGACGGCATTTTTGATTTTATGCGGAGGCGGTTTTTATTTTG
ACCTTGCTTTTCTTAAACTTCAACACGGCTTCTTCTTTTGCCGCATCCCAGTCTATCCGT
ACGAAGCCGCCGTCGGATAGTTTGCCGAACAGGAGTTCGTCGGCGAGCGGTTTGCGGATT
TTTTCCTGAATCAGGCGGTGCATCGGGCGCGCGCCCATTTGCGGGTCAAAACCTTTTTCC
GCCAGATATTTGTGCAATGCCGACGTGAATTCGGCTTCGACTTTTTTGTCGAGGAGCCGG
TGTTCGAGCTGGAGCAGGAATTTGTCCACGACTTTGGTGATGACGGGTTCGGATAAGGGC
GCAAACGGGATAATCGCATCCAAGCGGTTGCGGAACTCGGGCGTGAAGAGCTTGTTGATA
GCCTGCATTTCGTCGCCGCGCTCGCGTTTGGCGGTAAAGCCGAGGCTGGGTCGGCTGAGA
CTCTCCGCACCTGCGTTAGTGGTCATAATTAGGATGACGTTGCGGAAATCGGCACTCTTG
CCGTTGTTGTCGGTCAGTTTGCCTGCGTCCATGACTTGCAGGAGGACGTTGAAAATGTCG
GGGTGGGCTTTTTCGATTTCGTCCAAGAGCAATACGCAATGCGGCTGTTTGTTGATGGCT
TCGGTCAAAAGGCCGCCTTGTTCAAAGCCGACGTAGCCCGGTGGTGCGCCGATGAGGCGC
GATACGGCGTGGCGTTCCATATATTCGGACATATCAAAGCGTTGCAGCGGTACGCCCATC
GAGTAGGCAAGCTGTTTGGCGACTTCGGTTTTGCCGACGCCAGTCGGACCGGAGAAGAGG
AAACTGCCTATCGGTTTGTCGGGCAGGGCAAGGCCGGAACGCGACATTTTGACGGCAGCA
ACCAACGCGTCGATGGCGTTTTCCTGACCGTAAACCATGTTTTTCAAATCGCGGCCGAGG
AATTGCAGCACCTGTTTGTCGTCGTGCGACACGGTTTTTTCTGGAATCCGCGCGACTTTG
GCGATGACGGTTTCGATTTGCGCTTTGCCGATGACTTTTTTCTGTTTGGATTTGGGCAGA
ATCCGTTGCTCCGCGCCTGCTTCGTCCATCACGTCGATGGCTTTGTCGGGCAGGAAACGC
TCGTTGATGTAGCGTGCGGAGAGTTCGGCGGCGGCTTCGAGTGCGCCTTGAGTGTAGCGG
ACTTGGTGGAAGGCTTCAAACATCGGTTTCAAGCCGCGCAGGATTTGAACGGTTTCGGAA
ACGGTGGGTTCGACCACGTCGATTTTTTGGAAGCGGCGGCTTAAGGCATGGTCTTTGTCG
AAAATGGTGCGGTATTCGTCGTAGGTGGTCGCGCCGATGCAGCGCAGCGAACCTTTTGCC
AGCGCGGGTTTGAGCAGGTTGGACGCGTCCATGGTGCCGCCGCTGGTGCTGCCCGCGCCG
ATGATGGTGTGGATTTCGTCGATAAACAAAATGGCGTGCGGGATTTTTTCGAGCTGTTTC
AAGACGGATTTGACCCGCGCTTCAAAGTCGCCGCGGTATTTCGTGCCCGCCAACAGCGAG
CCCATATCCAGCGCGTACACTTCGGCATCTTTAAGCGCGTCTGGAATGCCGCCGTTGACG
ATTTGATGTGCCAAACCTTCCGCCAGCGCGGTTTTGCCCACGCCCGCTTCGCCGACCAAA
AGCGGATTGTTTTTGCGGCGGCGGCATAGGATTTGCACCAGCCGTTCCATTTCGTGTTTG
CGACCAATCAAAGGGTCGATACGGCCGGCTTTGACTTCGGCGTTGAGGTTGACGGTGTAC

## Appendix A

GCCGATAAAGGGTTTTTGCCCGGTTTGGTGCGGTTTCCATTATCGTCGTCCATGCCGTCT
GAAGAATAGTTGCCATCGTCTTCATCTTCATCTTCATCTTCATCTTCATCGGGAGAGCCG
TGGGCAATACAGCGCAAAACTTCAAAACGCGTAACCGATTGCAGCTTGAGGAAATAGACG
GTGTGGCTGTCGGTTTCGCTCATCAGCGCGACCAAAACGTCCAACGGTTCGACTGCGGCT
TTTCCGGCAGACTGGGTATGCACCATCGCCCGTTGCATCACGCGTTGGAAGCCGAGCGTG
GGCCGGGTTTCGACCGTGTCTAAAAGGTGTTCGGGAATCAGGGGGGTGTTTTCGGCAACG
CTGGCGGCGAGCTGTTCGGACACCACTTTCAAATCCGCGCCGCAGAGCTTGAGGACGTTG
TGGACGGAGGCATCTTCTTCGATGAGTACCAAAAGCAGATGCTCGAGGCTGATAAATTCA
TAATGAGCCTTACGCGCCTCGCGGTAAAGCTGCTGCAAAATCTGTTCCAATTCGGGTGCA
AGCATATTAAATCTCCTCGACAATACATTGCAGCGGATGCCCTTCGGCTTTTGCCCGCTG
CATGACTTGTTGTTGTTTGGTTTGGGCAATATCGCGCGTGTAAGTGCCGCACAGGCCTTT
GCCTTCGTGATGAACCAAGAGCATTACCGCTACCGCCTGTTCTTGTCCGAGCATAAAGAT
TTCGGTCAGGATTTCGACGACAAATTCCATCGTGGTGTAATCGTCGTTCAATAGGAAAAC
GCCGTAACGTTTCGGCGGCAGGGTGTTCAGACGGTGCAAGAGCGTGTCGGATTGGTGTTG
CGCGGTCATAGTGTGTCCCATTTGAAAGCCGCGTTCAGACGGCATTTTTGTTGTATTTTC
GGTACTTTTGCCTATTTTCCCACTTTTTTGAAAACATAGCTTGACGTTTTGTCTTAACAA
ATGTAAAAAGACGGTTAAGCAGGATTGGGCATCCGCCGAGTATATCAAGCTGGAAGGAAC
GCCAGACGGCTCGGTTTGCCGTATGCCTTGTTTTGCTGATTTTGTTAATTTTTGAGTATA
GGAAGTTTCTAATGGCAACCGGTATCGTAAAATGGTTTAACGACGCTAAAGGTTTTGGTT
TCATCACGCCTGATGAAGGCGGCGAAGATTTGTTCGCTCACTTCTCAGCAATCAATATGG
AAGGTTTCAAAACCCTGAAAGAAGGCCAACGCGTCTCTTTCGACGTAACCACCGGCCCTA
AAGGCAAACAGGCCGCCAACATTCAGGCTGCTTAATTCCTGATGTACGGTCAAATGTATA
TTTGAAAACGGCGGGACAGGCAATGTCCCGCCGTTTTTGTCTGCCGTTTTGCCGGCGGCG
GAAAAACCCCAATCCCCGCACGCCTTATCCTGAACTTGTGTGTACCCTGTTGTGGACAAG
TGGCTTAGTATTTTGACGGATAAGGGAAAATCAGTGCTGATGAAAAAATGTGCAATGTTG
TCGGCAAAAGGCGGTGCGGCATAAAACGGCAAACGGGTAGGCACGGGGCAAAACGTGCTG
CCTTCGTCTTCAGACGGCATCGGCAGGGCGTTCAGCTTCCGGCAACCGTCATCCCCGCAA
TCAGAATCGAGCCGATTTTGTTGGACGAACGCCGCAAAGCGTCATCCGCCACGCCGACAA
TGTCGCGGTACATATCCTGCAAGCGTCCGGCTACGGTAATCTCGTGGACGGGGTAGGCAA
TCACGCCGTTTTCCACCCAAAAACCCGCCGCGCCGCGCGAGTAGTCACCGGTAATGGTGT
TCGCGCCCTGTCCCATCAGTTCGGTTACCAACAATCCCGTCCCCATTTCTTTCAGCAGGT
CGGATTGCGTTTCGTGCGTATGGTTCAAATACAGGTTGTGCGCGCCGCCGGCGTTGCCCG
TGGTCTGCATACCGAGTTTGCGCGCGCTGTAACTGCTGAGGAAATAGCCTTCGACAATGC
CGTTTTGAATCACGAAGCGCGGTGCGGTGGCAACGCCTTCCGCATCAAAATAGCTGCTGC
GGAAAGAGCGGGGGATGTGCGGTTCTTCGCGCAGGTTGAGGAAATCGGGCAGGACTTTTT
TGCCGATGCTGTCGATCAGGAAACTGCTTTGGCGGTAGAGCGCGCCGCCGGAGAGTGCGC
CGACGAGGTGTCCGATAAGACCGCCCGAAACGGTGGTATCGAAGAGGACGGGGTAGCTGC
CTGTCGGGATGCTGCGGCTGCCGAGTCGGCGCAAAGTGCGGCGGGCGGCGGTTTGACCGA
TGGTTTCGGGGCTGTCCATATCCGGATGGCGGCAGGCGGAATCGTACCAGTAGTCGCGCT
GCATGCCGTTTTCGTCGGCGGCAACGACGCTGCAGGAAATGCTGTGGTGCGTGCCTTGCC
GGTGTGCGGCAAAACCGTGGGTGTTGCCGTAAACGTATTGGTAATGGCCGGTTTGCACCG
CCGCGCCTTCGGAGTTTTCGATGCGCTCATCCTCGTTCAGGGCGGCTTGTTCGCATTGTT
TTGCCAAGCCGACGGCGGCTTCCGTATCCAAATCCCATTCGTGGTAAAGGTCGGGGTCGC
CGATGTGTTTTGCCATCAGACAGGCATCGGCAAGTCCGGCGCAACCGTCTTCGGCGGTGT
GGCGGGCGATGTCGATGGCGGCTTTGACGGTGTCTTGCAGGGCTTTTTCGGAGAAGTCGG
CAGTACTGGCGCGGCCTTTGCGTTTGCCGACGTAAACGGTAATGTCCAGCGACTTGTCCT
GCTGGAACTCGATTTGTTCGATTTCGCCCAGCCGCACGCTGACGCTTTGTCCCAATGATT
CGCTGAAATCGGCTTCGGCGGCGGTTGCGCCCGTCGCTTTTGCCAAGTCGAGCGTGCGGC
GGCAGAGGTCGAGGAGTTCGGAAGCGGTGTGGTTGAACAGCATAGAAATCTTTCTTGATG
~~ATGCTTTGCGGCATTTTAACCGTTTCGGGCGGCAGGGGCAAAAGCGCGCCGTTTGCAGGG~~
CGGACGGTGCAAAATGCCGTCTGAACGCGGCGGCATTCTGTTAAAATGCGCTATTGGAAA
AATTCGAGAATCAAGATGTTTGAACAAGAAGACGAATGGATCAGCAAAACCCAAATGAAA
AAGCAGATGAACGATTTGCAGGATTTGGGTATGGCGTTGACCAAGCTCTCAAACGATACG
CTGAAAAAAATCGGTTTGGATGCGGATTTGTACGAGGCGGTAACCGCCTATAAAAAAATC
ACATCCAACGGCGCGCTCAAACGCCAGGCACAATTTATCGGCAGGCTGATGCGCGATACC
GATCCCGCGCCCATCGAGGCGTTTCTTGCCAAGCTGCGCGGCGACGATGCGGCGCACAAC
GCCTTTTTGCAACGCGTGGAACAGGCGCGCGTACGGCTGTTGGCAGACGACGGCGCGTTG
ACGCAGTTTATGTCGGATTTTCCGCATGCGGACGCGGGCAAGCTGAGGACACTCATCCGC
AATACCAAAAAAGAGCAGGAGCAAAACAAACCACCAAAAAATTTCCGCGCCCTGTTTCAA
GAGTTGAAAACCGTGATGGAAAACGGGGACGCGGAAATTTAGGCATATTTTCAGACGACA
TCCGCCGTTATTTAGATTGGAGGATAAAATGTTGTTCCGTAAAACGACCGCCGCCGTTTT
GGCGGCAACCTTGATGCTGAACGGCTGTACGTTGATGTTGTGGGGAATGAACAACCCGGT
CAGCGAAACAATCACCCGCAAACACGTTGACAAAGACCAAATCCGCGCCTTCGGTGTGGT
TGCCGAAGACAATGCCCAATTGGAAAAGGGCAGCCTGGTGATGATGGGCGGAAAATACTG
GTTCGTCGTCAATCCCGAAGATTCGGCGAAGCTGACGGGCATTTTGAAGGCAGGGCTGGA
CAAACCCTTCCAAATAGTTGAGGATACCCCGAGCTATGCTCGCCACCAAGCCCTGCCGGT
CAAACTCGAATCGCCTGGCAGCCAGAATTTCAGTACCGAAGGCCTTTGCCTGCGCTACGA
TACCGACAAGCCTGCCGACATCGCCAAGCTGAAACAGCTCGGGTTTGAAGCGGTCAAACT
CGACAATCGGACCATTTACACGCGCTGCGTATCCGCCAAAGGCAAATACTACGCCACACC
GCAAAAACTGAACGCCGATTACCATTTTGAGCAAAGTGTGCCTGCCGATATTTATTACAC
GGTTACTGAAGAACATACCGACAAATCCAAGCTGTTTGCAAATATCTTATATACGCCCCC
CTTTTTGATACTGGATGCGGCGGGCGCGGTACTGGCCTTGCCTGCGGCGGCTCTGGGTGC
GGTCGTGGATGCCGCCCGCAAATGAACAGCAATGCCGTCTGAAAAGCTTTCAGACGGCAT
TTTAAGCACACACGCACAGTAAAACCCCACGTTATGTCAGTCAAAATCCAAACCCGATCC
GTCAATACCGACGTTTTTAATCATTTGCTCACCGCCGGTGCCGATCCTTTAATCGCCCAG

## Appendix A

CTTTGTGCTTCGCGCGGTGTGCAAAGTCCTGCCGAATTGGACGACAAACTCGCTTCCCTC
CTGCCTTATCAATCGTTGACGAATTGCGAAGCCGCCGCCCGCCGTTTGGCGGATGCGGTT
GGGCGCAAGGAAAAAATCCTGATTGTTGCCGACTATGATGCCGACGGTGCGACGGCGTGT
GCCGTCGGTATGAGCGGTTTGGCGGCGATGGGGGCGAAAGTGGATTTCCTTGTGCCCAAC
CGCTTTGAACACGGCTACGGCTTAACGCCCGAACTTGCCGAAATCGCTGCCGCGCAGGGC
GTGGATTTGCTGATTACGGTGGATAACGGTATCGCCAGCATCGCAGGCGTGGCGAGGGCG
CAGGCTCTGGGTTTGGATGTCATCGTTACTGACCACCACTTGCCGGCCGAGACCGTGCCC
GACTGCATCATCGTCAATCCGAACCAAAAAGGCTGCGGTTTTCCAAGCAAAAGCTTGGCG
GGCGTGGGCGTGATTTTTTATGTATTGATGGCGTTGCGTGCCGAATTGCGCCGCCGCAAT
TATTTTTCAGACGGCATCAAAGAGCCGAATTTGGGCGAACTTTTGGATTTGGTCGCACTC
GGCACGGTTGCCGATGTCGTCCCTCTCGACCACAACAACCGCATCCTCGTGTCGCAAGGT
TTGAAACGGATGCGCTCCGGCAAAATGCGCCCCGGTATCCGCGCCTTGTTTGAAGTGGCG
CGGCGCGATTGGCGCAAGGCGCAGCCGTTTGATATGGGTTTTGCGTTGGGCCCGCGCATC
AACGCCGCCGGACGGCTGGACGATATGTCGGTCGGCATCGCCTGCCTGTTGGCGCGAGAT
GATTCCGAAGCTCAGGAACTGGCGGCTCAGTTAAACAACCTCAATATCGAGCGCCGCGAA
ATCGAGCAGTCTATGCTGCAAGACGCACTGAATGATTTCCCCGAAACCCTGCCTTCAGGT
CAGATGACTTTGGTGGCGTATCGCGACGACTTCCATCAAGGTGTGGTCGGCATTGTCGCC
AGCCGCCTCAAAGACCGTTTTTATCGTCCGACCATCGTGTTTGCGCCTGCCGACAACGGC
GAAGTACGCGGTTCGGGACGTTCCATTCCCAATTTGCACCTGCGCGATGCTTTGGACTTG
GTGTCCAAACGCCATCCCGATTTGATTTTGAAATTCGGCGGACACGCGATGGCGGCGGGT
TTGAGCATACTTGAACACAACATTCCCGCGTTTCAGACGACCTTTGAAGAAGCCGTGCGC
GAAATGGTGTGCGAAGACGATTTGTCGCAAACCTTCATCACCGACGGCAGCCTGCCCGCC
TGCGACATCACGTTGGAACAGGCGCAAAACCTTGCCCGTCACGTTTGGGGGCAGGGCTTC
GCGCCGCCGAGCTTTACCGACGAGTTCCACGTCGTCCGCCAGCAACCTTTGGGCGCGGAG
GGCAAACACAAAAAAGTCTGGCTGCAAAAAGACGGCTGCGAATTTGAAGCGATGTTTTGG
CGTTGCAGCGAAGACATTCCCGAATACATCCGCACGGTTTACCGCCCCGTTGCCAACGAA
TGGCGCAACAATCTCGAATTGCAGCTGTATATCGATTACTGGGAAGCCGCGTAGAGGCGG
CGGAACACTGTTTGAATGTGATTTCTGTTCCTTCATTTGCCTGTTTGTACGACGGGAATG
TTCCCAATCGGAGAAGGCGCATCAAATTTCAGACTCTGCCACAAAAGCAGGGTCTGATTT
TTTTGGAGGGCAATCTGTTATAATGACGCGTTGCCGCCGCGAGGGCGGCGTGATTCGGAC
GGCGTAGTTTCTACGCCTTTTGTTTATGGTTACGGCATCTTGCAAACCGCGCCTGATGCC
GTCTGAACACGGTTGCCTGTGGAGATGCCGCTCTTCGGGTCAGAATATTTATGCTGAAAA
AATGGTTGAATAAGATGCTGCCTTCCGGTCGGAGCAGTAAAAAAGCGGAAAGTAAAACGG
TCATTCCTGCCGAAAGACACAACATCCGTGCCGAAATGTTGAGCTTTGCCGCCGAAAACG
TCATACGCCGCCTGAAAGGGACGGGGTTTCAGGCTTATGTGGTCGGCGGCGCGGTCAGGG
ACCTGCTGCTCGGCATCGAACCCAAAGATTTCGATGTCGCAACCGATGCCACGCCCGAAC
AGGTGCACAAACTCTTCCGCCGCAGCCGCATCATCGGCAGGCGTTTTCAGATTGTCCATG
TGATGAACGGTGCAGAGACTATCGAAGTAACGACGTTTCGCGGCGGTGCGAAAGTACATC
AGAATGCACGCGGCAGGATTATGAAAGACAATACCTATGGCAGCATCGAAGAAGATGCGA
TGCGGCGCGATTTTACCTGCAATGCCTTGTATTACGATCCTGAAAAAGAAGAGATTTTGG
ATTTCCACAACGGGATTGCCGATGTTGCCGCCCACAGGCTGGTTATGATTGGCGATGCCG
CCGAACGCTATCAGGAAGACCCTGTCAGGATTTTGCGCGCCATCCGCCTGTCGGGCAAAT
TGGCTTTGAGCTGTCGGAAGAAACCGCCGCACCGATTGCCGAATCGATATGCCGTCTGA
AGCACGAACCGGTAGCGAGGCTGTTCGACGAAATTATGAAATTGCTGTTTTCAGGGCACG
CTCGCGAGTGTCTAAAACGTTTGAACGGATTTGACATACCGGACGACATCCATCTGCTGC
TCAATGCCTTGCGCGTTTCAGACGGCATCGCCGGAAAAATGACGGTGCTTGCCCTGAAAA
ATACCGATGAGCGGCTGCGTGCCGACAAATCGGTTTCGGTCGGTTTTGTATTGGCGGCTC
TGATGTGGCCCGAGTTGGAACGCCATTGGAAAAGCAATCTGCAACAGGGTTTGAAACCCG
CGCCCGCCCTGTCCGATGCAATCAATACGATGCGCGAAACCGTCGAACGCGGTTGGGGCG
~~TGCCGCAACGCTTTTCCGCCACGATGCGCGAAATTTGGATGTTCCAGCCGCAGTTTGAAA~~
ACCGCAAAGGCGCAAGGCCGCACAAACTGTTTGCACAGGCGCGTTTCCGTGCCGCCTATG
ATTTCCTGCTCTTGCGCGCCGAAACCGGCAATGCGGACCGCGCCCTTGCCGAGTGGTGGA
CGGCGTTTCAGACGGCATCGACGGAACAGCGGTCGGAGATGACCAAAAACGAAGCCGCCG
CCCGACACGAAAAAAACGAAGGACAGGCGAAAAAACGCCGCCGTCGCAGGCGCAAACCCA
AGCCGAAGGTTGTGGGAACGGATTGGGAATAAGGGTCAACAGACATGGAGCAATGAAGTT
TCAACACATGGGATGAAGCATAAAGTGCCGTTCTATGCATTATCCTGATTTGTAAGGGGA
TTCATCCCCGTAAATAAAGTCTAACCCTGCCTCTCGGAAAAAGGATGTCCGGGTGGGCAG
GGTTCAAGCAACAAGGAAAAATTGATGAAAAAATGTATTTTGGGCATTTTGACCGCGTGT
GCCGCCATGCCTGCATTTGCCGACAGAATCGGCGATTTGGAAGCACGTCTGGCGCAGTTG
GAACACCGTGTCGCCGTATTGGAAAGCGGCGGCAATACCGTCAAAATCGACCTTTTCGGT
TCAAATTCCACCATGTATGTATGCAGCGTTACGCCTTTTCAGAAGACGTTTGAGGCAAGC
GATCGGAATGAAGGCGTGGCGCGGCAGAAAGTGCGTCAGGCGTGCAACCGCGAAACTTCG
GCAATGTTTTGCGAAGATGAGGCAATCCGATGCAGAAAATTCGATTGATGTATCGGTTGG
ACGGATAAAGAAACGGATACGGATACGGAGCTTGGCTTCCGTATCTGTTTTTCTCTGCCT
GATTTTCCATGCATCGGGTTTCAGACGGCATTGGAATGTCAGTCGTGTTCTGCCGATTCG
TAGGCTTCGACGATTTTTTGCACCAAAGGATGCCGGACAACGTCTTCGCCGGTAAAGGTG
TGGAAATACAGCCCTTCCACGTTGTGCAGTTTCTCACGCGCATCTTTTAATCCCGATTTG
ATGTTTTTGGGCAGGTCGATTTGGCTGGTGTCGCCGGTAATGACGGCTTTCGCGCCGAAG
CCGATGCGGGTCAGGAACATTTTCATTTGTTCGGGCGTGGTGTTTTGCGCTTCGTCGAGG
ATGATGTATGCGCCGTTGAGCGTCCTGCCGCGCATATAGGCGAGCGGGGCGATTTCAATC
AGGCCTTTTTCAATCAGCTTGGTTACACGGTCAAAGCCCATCAGGTCATAGAGGGCATCA
TAAAGCGGACGAAGGTAGGGATCGACTTTCTGGGTCAGGTCTCCGGGCAGGAAGCCCAGT
TTCTCGCCGGCTTCGACGGCTGGGCGCACTAAAATGATGCGTTCGACTTGGTGTTTTTCC
ATCGCATCGACGGCGGCGGCAACGGCGAGATAGGTTTTGCCCGTACCTGCCGGCCCGAGA
CCGAATACGATGTCGTGGTTGAGCAGGGCGCGGATATAGCCGTTTTGCCGTGGCGTTCTG

## Appendix A

CCGCCGATGCTGCCGCGCTTGGTGCGGAAATAATAGGCGTGGTCATGGTTTTTTTCTTGA
TGACCGGCATCTTCGGTTTGGGCTTCGACGGCGGCAAGCCTGATGTCGCCGTCGTTTAGG
TCGCGCGTCTGCGCCGTTTCCAAGAGTTTGAGCAGTGCGCGTTTGCCGGCGTGTGCAAAT
GCGCCGTTGAAAGTGAAATGTTCAAAACGGCGGCTGATGTGGATATCGAGTGCTTTGGCA
AGTAAATCAAGGTTGTTGTCAAAAGAACCGCACAGACGCTGCAACGCCAAGTTGTCGGTT
TCTTCTAAATGCAGGTGGACGGTATGTGTCATATGAAGGTCCGAATAGTTGGATATTGTG
TGATTTTAATCTATAGTGGATTAGATTTAAACCAGTACGGCGTTGCCTCGCCTTAGCTCA
AAGAGAACGATTCTCTAAGGTGCTGAAGCACCAAGTGAATCGGTTCCGTACTATTTGTAC
TGTCTGCGGCTTCGTCGCCTTGTCCTGATTTTTGTTAATCCACTATATTTCACCGGTATT
TTCTTACCGTATTCTGCGATTGCCTGTCGGAAAATGCCGATCAACCTGCCTATAACGGCA
TTTTCGCCAAATTCGTTCAGACAGTTTTCTCTAAGTCGGGCAGGTTCGAAATCAGAGTGG
TGTTCACACATTTTGATGAGTGCGTCGGCAAGGGCATCGTCGTCGTCAACAGGAACCAAA
TATCCGTTGCCGTCTGAAACAATAGATTCCGCACCGCCGCAGCGTGTTGCAATGACGGGC
AATCCTTGGGACAGTGCTTCGATATAGACTACGCCGAAGGTTTCTGTGCGGCTGGCAAGG
ACGAATGCGTCGCTGTTCCTCATCAAATCCAAGACTGCTTCGGGCTGCAATGCGCCCAAA
AATGTAACGGCATGGGTAATGCCCAAGTCTGCCGCCTGCTGTTTCAGCCGCTGTTCTTCC
TGTCCGCTGCCGCCGATGTTCAGGCGCAGTTGCGGGCATTGTGCCAACGCCCGGGCAAAG
GCAGTGAGTAGGACATCGTGTCCTTTGAGACGGCGAAGGTGCGAGACGGTGCAGAACACG
AAATGCGGTTTGTTGTTTTTTTTCTCTTTGTTCAAACGGTCGGTTGAATATTCCGCCCAG
TATGTTGGGGGAGGTACTGCCATTCGCAGCCGTATTTGTGTTGCAGGACGTGTGCGAAAT
GGCGGCTGACGGCGAGACGTGCGGCGGCGTGTGCCGCCGCATTTTTCATAGGCTGCCATT
GGTGCGGGCGCACCAAACCGCGCGTAATGGTGCTGCTGTGTTCCGTGACGACATAGGGGA
TGCCGTATTTTTGGGAAATCTTGAAGGCAAGTATGCCGGCATAGTTCATACAGTGGGCGT
GAATCAGGTCGGGCAGCCCGTTTTCGCGGATGTAGTGTTTGAAAGCTTTCAAACCCGCAC
ACACCCAGCGGATGCGGTCGATGTCGATGAACGGAAAGCGGGGGAAGAAATACATGCCGT
GCCATGCATAGATGTCCAAACCGCTTTGCCGATATAGTGGATTAACAAAAATCAGGACAA
GGCGACGAAGCCGCAGACAGTACAGATAGTACGGCAAGGCGAGGCAACGCTGTACTGGTT
TAAATTTAATCCACTATATTTGCAAAACCGTAAGGGTCGGTCAGGATGCTTGCTGTTTC
TTTCCGCAAGTAGCGGAACATCGGTGCAAGCACGGCGGTTTTGATGCCTTTCCTCTGCAA
TGCCAGTGCCTGATTTTGAAAAAAATCCCGTCCACATCCTGTTCGGATTGCGGATACCAT
GAGGGGATGACGAGGACGTGCAAGGGTTCGGGCATAGTGGGATTCCGTATCGGAAAGGCG
GTTATTATAAGACAGACGCAGACCGAATATTTAAATTGTTGCCTTACGCTAATGCAATTT
GGCGCGCCGGTGTGTTAGATTGGCAGTTTTATCGGTAAGGAGGCGGATATGTTGCGTCTT
GTTTTGGCGGCTTCGCTGTCGGCGGTATCTTTTCCGGCAGCGGCTGAAGCATTGAATTAC
AATATTGTCGAATTTTCCGAATCGGCGGGTGTCGAGGTGGCTCAGGATACAATGTCCGCA
CGTTTCCAAGTGACGGCGGAAGGACGGGACAAAAATGCCGTCAATGCTGAGTTTGTTAAA
AAATTCAACAAGTTCATCAGAAAATCGAAAAATGGTAGCTTTAAAACCGAATTGGTATCG
CGCAGTGCGATGCCGCGCTATCAATATACCAACGGCAGACGCATTCAAACAGGCTGGGAG
GAGCGTGCGGAATTTAAGGTCGAAGGTAGAGATTTTGATGAGTTAAACCGTTTTATTGCC
GATATTCAAGCAGATGCCGCGTTGGAATATACGGATTTCCATGTGTCGCGCGAACGCCGC
AACGAGGTCATCGATCAGGTCAGCAAGGATGCCGTTTTGCGTTTCAAGGCGCGTGCCGAA
AAGTTGGCGGGCGTTTTGGGTGCGTCCGGTTATAAAATCGTCAAATTGAATTTGGGACAC
ATCGGCAGCCATATCGCGGGAGGGGGAGCTGCTCAGGCAAAAATGCTTCGTGCCATGCCG
ATGGCGGCAAGCGTCAATATGGAGGGTGCGGATTCCGCCGCGCCTGGTGTGGAGGAAATC
AGCATCAGCGTCAATGGGACGGTTCAGTTCTGATTTGAGGTGAACGGCAAATGCCGTCTG
AAACCCGACGATAAGGGTTCAGACGGCATTTATATTTCAGGCTTTGGGCAGGGTAACGCC
GGTTTGCCCCATATATTTGCCGTTGCGGTCTTTGTATGAGGTTTCGCAGATTTCGTCGCT
CTCGAAGAAGAGGACTTGCGCCACGCCTTCGCCTGCGTAGATTTTGGCGGGCAGGGGGGT
GGTGTTGGAAAATTCGAGGGTAACGTAGCCTTCCCATTCCGGTTCGAACGGGGTAACGTT
~~GACGATAATGCGGCAGGGGGGTAGGTGGATTTGGCGAAGGAGAGGGTCAGGACGTTGCG~~
CGGGATGCGGAAATATTCGACCGTGCGCGCCAGTGCGAAGGAATTGGGCGGGATGATGCA
GCAGTCGTCTTCAACGGTAACGAAGTTTTTCGGGTCGAAGTTTTTGGGATCGACGATGGT
GCTGTTGATGTTGGTGAAGATTTTAAATTCGTTTGCGCAGCGGATGTCGTAGCCGTAGCT
GGACGTACCGTAGGAGATGATGCGTTTGCCGTCGGCTTCTTTGATTTGGTTCGGCTCGAA
AGGGTCGATCATGCCGAATTCTTCGCTCATTCGGCGTATCCATTTGTCGGACTTGATGCT
CATAATGTTTTCCTTGTTTCTTGCAGTGTTCGGACAAAGCATTGGGGGATGCCGTCTGAA
AACGGGGCTTATTTGTTTTTGGGCAGTTTCACTTCTTTAATCATGCCGTTTTCGCATTTC
ATAATGAAGAGGGTTTCGCCGTTTTGCGAGACGGTAAATCTGCCGTGTGCCAAGCCTTTT
TTGAACGTGCCCGAGAGTACCATGTTGCGGAATTTGGTACTGTCGGAATTGAACGGTTCG
ATAAATATTTCGCGGTTGGCGGCAACGGTATAAACGCCTTGCCCGTCGAATTTGCCGTTT
TTAAACGAACCGGTATAGTTGCGCCCGTCTTGGCAGCGCCATGTGCCTTTGCCGGCGGGT
TTACCGTCTTTGCCGACATTGCCGTCGTAGGTGCAGCCTGGTTCTTGATAGGAAGTCAGG
ACGGCGGCCGAAGTGGGGAGGGCGAACATCATGGCGGGCAGTAGGAATGCGAGATGTTTA
AGCATAAGGGTTATTCCATTGGATTTTGGTTGACGGTATTTTGTCGTGAAAAAGCCGTCT
GAAAAATCAATCTTGCCAGCCGCCCAAATAGGAAACCAGTTCTTCCAACATGGTGCTGAC
GGATTCCGCCATCAGAATTTGCGAGGCGAAGGCAAGGCCGGCGGCATCGTCGCCGTTGCT
TTCGGCTTCTTCCTGCAATACGTCGAGGTATTGGATGCGCTTGAGTGTGAAGTCTTGAGT
GAGGATAAAGGCAATTTGTTCGCGCCACACCAAGCCCAGTTGGGTAACGGTTTTACCGTT
TTTGACGTGTTGAACCACTTCGTCGGCGGTTAAATCTTGTTTGGATACTTTGACGACGGG
AACAATATCGCCCGTACCTTTGAGTTCGCAATCGCTGTCTAATTCAAAACCGCCTTCGCA
ATGCCCTTGCAACAGCCAGCCGGTCATCAAGGAAGAGGGCGATTGCTTGGTATTCGGCAG
CGAGGCTTCCAAACCGCCCAAAGCTTCGCGCAGCTTGGTCAGGATGTTTTCTGCTTTGGC
GGAAGCCGCGTTATTGACGAGCAGGTAGCCGTGGCGGGTGTTAAACACCGCTTCTGTACG
GCTGCTGCGGGTAAACGCTCGGGGCAGCAGGTCGTCTGTAATTTGCTCTTTAAGCTCTTG
TTTTTCTTTACGGCCGACATTGCGGGCTTCATTGTTTTGGATTTCCGCTACCTTCTCTTC

## Appendix A

CAAAATATCGCGGATGACGCCGGCAGGCAGGACTTTTCTTCTTTTTCAGGGCGACGCG
CAAGGTAAAGTCGGCAGGGAAAACGAGTTCAGGGGAGAATGAAACCGGTGCGGTAAAGCC
TTCGCTGAACCAGTCTAAGCCTTGGCAATGGGTAAATTCAGCTTCAGCAAGTTTGTCGGC
AAGTACGTCTGCCTCAGGCAGCTTTTCTTTGTTGAGCGGATAAAAACTAATCTGCTTGAA
CCACATAATGTTTCCTATTGTTTGAAATGTCGGGAATTATTTGCTGAATTGTTTTTTCAC
ACTGACTTTGGTTTTCTTCTTGAAGCGGTTTTTCTCTTCCTTTCGGCCTTCGCGTTTCTC
AATACGGTTGCTCAGGCTGACGCGGCGCAGCGGTTTTTTCTTCGGTTTGTCTTCCGCATT
TTCATACGGGTTTTCCGAAACATTGTATTGAATTCTGAGCGGCGTGCCTTGCAGATTGAA
GGCTTTGCGGAACGTTTGGGTCAGATAGCGCGTATAGCTGTCGGAAATCGCGTGCAGCGA
ATTGCCGTGTACCACAATTACGGGAGGGTTCATGCCGCCTTGGTGGGCATAACGCATTTT
CGGACGCACCAAGCCGGCACGCGGCGGTTGCTGACGCTCGATCGCGCTTTGTAATACGCG
CGTGATTTTCGGCGTCGGCATCTTAATCATCGCCGCGTTGTAGGCAGCCTGAATGCTGTC
AAACAAACCGTCTATACCGCGCTCTTTCAATGCGGAAATAAAGTGGAACTTGGCAAAATC
GAGGAAATACAGTTTGCGGTTGATATCGCGTTTCACTTGCTCGCGACGTTCTTCGCTGAT
GCCGTCCCATTTATTGACCGCCACCACCAAAGCACGGCCTGCCTCCAAAGCGAAACCTGC
AATCGTCGCATCTTGGTCGGCGATGTCCTGCTGCGCGTCCAATACCAAAACAGCGACGTT
TGCCGCTTCAACCGCCTGCATCGCTTTGATAACGGAGAATTTTTCCACTGCTTCATCCAC
TTTGCCGCGACGGCGCACACCTGCGGTATCGATGATGGTAAACGGTTTGCCTTCGCGCTC
GAAATCGATATGGATACTGTCGCGCGTCGTACCTGCCATATCGAAGGTGATGACGCGCTC
TTCGCCGAGAATGGCGTTAACCAGCGTAGATTTGCCGACGTTTGGACGACCGATAACGGC
AAAAACAGGATGTCTTGCATCGGCTTCTTCGGCTTCCGGCTCGGGGAATTTTTCCAAAAT
ATCTTCAATCAGATAATACACACCATCGCCGTGTGCACCTGAAATAACATAAGGGTCGCC
CAAAGCAAGTTCGTAGAACTCGGCGGCAAGTACAGCCCTATTGCCCCCCTCGCCTTTATT
CACGGCCAAATAAACAGGGCGCGGACTTTGGCGCAAACGGTCGGCAATAATCTTGTCTTG
CGGTGTTAAACCGGTACGGCCGTCCACCAAAAACACAACTGCATCAGCTTCATCGACAGC
CTGTAAGGTTTGTTTTGCCATTTCGTGCAAAATGCCGCTGTCCACAACCGGCTCGAAACC
GCCGGTATCGATGACCAAATAAGGTTTGCTGCCGACTTTGCCGTGTCCGTAATGGCGGTC
GCGCGTCAGACCGGGCAGGTCATGCACGAGCGCGTCTTTGGTGCGCGTCAAACGGTTGAA
TAAAGTAGATTTGCCGACGTTGGGGCGACCAACAAGCGCGATGGTTGGTTTCATGATTGA
GTCTTTCTGTGTCAAGTGCCGTTCGGGAGAACTGAACACGAGCAGGTGTCCGTTGGACAC
GGCGGATGGGTTTTACGGGAATTGCCGTAGGATAGTGTTGTCTGAAATGCCGTCTGAAGA
GAGGGTGGCATTTCAGACGGCATTTATTTCAGCGAATCAAGTTTCATTTGAACCAATTCG
CGACCGACAGAATCTTGAGGCATTTTTTCTAAAGCCTGTCCGTAGTTTTTTAAGGCTTCC
TGGCTTTTTCCCTGTGCGGCATAGACATCGCCTTTGGTTTCCATCAGCAGGGGGGCGAAG
TCCGCTTCAACCGGCGTATCGAGCGCGGCAAGCGCGGCATCGTATTTTTTTTGTTGCAAC
AACACAACGCCCAGACGCTGCGCCGCCAACGCTTGAATCAGGCTGTCTTTTTGGTTGGAC
AACACCCATTTCAAATGGCCTTCGGCAACATCGTAACGCTGCGCGTCAAATTCGGTTGCC
GCCGCCATCAGTGTGGCTTGGGCGGCGGAAATGGAATGCGGGTAGCTTTGTTGGAGTTTG
GTCAATTCGGCATTGATTTCGCTTTGCGGGGCTTTGCTTTGCGCCTTTTCTACGATGTTT
GCCAGCACCGCCGCCGCTTCCTGATTTTGGGAAACTTTACGGTTTTGGTAAACCGTGTAT
CCCAAGTAGCCGAGTGCCGCCAAAATCAGCAAGGCAAACAGCCATTTGCCCGTGGTTTTC
CAAAAATATTTAAAGTTGTCTAACTCTTGTTGTTCTTCGAGATGGGCTGCCATTTATGCG
TTCTTCCATTGTTGTAAAGTAGGGGTTAAATCCTCGGCGGCGACAGTTTGCTGACCGTGT
GCGCCGTTCATGTCTTTGAGCGTAACCGTACCGTTCGCCAGTTCGTCTTGCGCGACAATC
AGGGCAAAGCGTGCGCCGCTGTTGTCGGCTTTTTTCATTTGCGCTTTCAGGCTTTGATAG
CCGGAATGCTGCATTACATTGAAACCTTGCGCGCGCAAGGCTTGTGCGTATTTCATCACC
TGCAAGTCCGCCCCTTCGCCTTGGTGCATTGCATAGACATCAGGCGCAGCGTTCACTTCC
AGAGAGCCGTATTCGCTCACCAAAAGCAGCAGCCGCTCGATACCCATTGCAAAGCCGATA
GACGGCGCAGGTTTGGCGGCGAGTTCTTCAATCAAGCCATCGTAACGGCCGCCGCCGCAC
ACAGTCGCCTGCGCGCCGAGTTTGTCGGTCGTCCACTCAAAAACCGTCTGATTGTAATAA
TCCAAACCGCGAACCAAGCGCGGATTTTCAATATATTGGATACCCAAACCATCCAACATC
GCCTTGAAGCGTACATAGTGGTTTTGCGAATCCTCGCCCAAGTAATCCACCAAACGCGGC
GCCGCGTTGCAGATTTCCTGCAAATCTGGGTTTTTCGTATCCAAAACGCGCAAAGGATTG
GTTTTCAGACGGCGTTTGCTGTCTTCATCCAATTTATCTTCATAACGGGTCAGATATTCA
ACCAATGCCGCACGGTGTGCCGCGCGTTCCTCACGGTTGCCCAAGCTGTTGATTTCCAAA
GTCAGGTATTCGCGGATACCCAATTTTTCCCATAAGTCGGCAGACATCGCGATGATTTCC
GCATCAATATCCGGCCCTTCAAAACCCAAAGCCTCGATACCGACCTGATGGAACTGACGA
TAACGGCCTTTTTGCGGACGCTCGCGGCGGAACATCGGCCCCATATACCACAGCTTTTGC
GGGCTATTGTACAGAAGGTTGTGTTCGACCACCGCACGCAGGCAGGAAGCCGTACCTTCA
GGACGCAAGCTCAAACTCAAAGAATCGTTTGAATCGGAGAAGGTGTACATTTCCTTGCCG
ACCACATCGGTTTCCTCGCCGATGGAGCGGACAAACAAACCGGTTTGCTCGACAATCGGC
GTACGGATTTGCTGATAACCGTAAGCGCGTGTCCAGCGGCCGACCGTATCTTCAAACGCC
TGCCAAAACGCAGCCGTCAGTTTGAAATCTTTTTGCTTGACAGGCAGAAGGTCGTTCATG
CCTTTGACGGATTGGATTTTTTGTGCCATTTCAAGTAAGAATGCTTAAATCAAATTGCGG
GCGATTATAGCGGATTTTAAAGGGTTTGTGAGGTTGGAGGTGGTTTGCGGACGGCATTTG
ACTTACTCTGCACGTGCTTGCCTGATTTGTCCGACTGTAAACTCCGTCTGCCGTTTTGGG
TGTTGTGTGAAAAACAATTTATTTGAAATTGTCTCGGCTTTTTTCGGTATGACAGCCAAA
ATCTTACCTGCCAAATTTCCCTCACGGGTTTGCCAAGCATCCAAAAACTGCGCCCTGCTC
ATTGAAACATGCCCCAGCGACGGGTCGGCAAGCAAAACCGTATTGCCGTCTATACCGCGC
AATACCGAAAAATGGTCGTCTTTGCGGTATTTCAGATACACGATGACGGGGATTTTCAAC
TGCGCGAGCTGCTCGAAAGACAGGGCATAGCCCTTCGCCTCAAAACCCAAATCAGGCATA
ATGCGCCGCATATCCTCAAACGACGCGCGCATCTGCTCCTTATCCAGCTTTTTCAACACT
TCTTCTTCCGTCAGCGTTTGCCCGTAAAAATTGTTCAAAAGCGTCGCCACCGAAGCCGCC
CCACAGGAAAAATCCAAATCCTGCTTTACAATATTGAAATCCCGCCGCGCTTTCCAACTC
TGCACTTTGATTTTTCCGTAAACAACAGGATTATCGTTAAACATCGGTGCAGCATTCAAA

## Appendix A

CGATAAGATAAAGAAACGACAACACACGCCAACAGAAAAACATATTTGAACTTCATCATA
TTGTCCACATAAAGGGCAGCCTGAAAATCTTTCAGGCTGCCCTTGTCAAATTATTCCTAG
CTTTCGGCTTTTTTGGCAAACCAAACAATCCGATTACCCGCATAATACTTTCCATTTATT
GAAATCCGACAAGCCGCGCCCAAAAAATGCCATGCACTGTCGATTTCCGCAGCAATCTTT
GTACCGTTTTCTTCAAATTCCAAATATTCACCCAATAATAAACTTGAAACAGAACGCGTG
GGCAGCAAGTGCCCACCCTACGCTTATTCAAATAATTTGATTAAATAAAGAATAAAGAAA
ATGCATCAGGAACAAAATTATAATCTGCCACCTGACTCACACCGCTTTTAAAGTAAGGGG
CATCAAAATCAAAACCGCAAAAAAAATAATTTTTGCATTGATTTTTAATAGATTTAAAAT
TCGAATATAGTGTTTCTCTAGATTTAAAAAGTTTATCTTTATCTATATATTTGATGCTTT
CCCTATCCAAAATAAATATTTCAAACATTAAAAAATCATTACATGACCAAGCCAAATCAT
AAATTTGATTCAAATGGGTATCATAAAGATAAAAATAATAAGGTTTGGGAACAGGTAAAA
TATTTAATGGAAAAGGAGGACTAATTTTCTTAATATCTGATGACTGATATCCATATCTTT
CTATATTCTTAAAAATTTCATCTTTCTTAAGATAACAAGACATTCTGACAATCACAACCT
GTTCATCGGATATATTATCTATTTGCATGGCGCGTATAACACGCCATGCCTGATTAAAAT
TAGTCTCCCTTACCTTAAAATCTATTATCTTTTCCAAAGATGAAAATGCCCCTTATCTTG
AACTCTCCAGTTAGAACCTGGAAGTTTCGTACCTCTTAAATGTGTATTAATATTTCTTAT
AGTTTCATTAAAATGCCACGCGCTGCCGATTTCAACGGTAATTTTCGTACCGCTTTCTTC
AAATTCCAGGTATTCCCCCATTAGCTAACGCAAAGAAGCAGACGCCATTTCGGCTTCGTT
ATGATAAACCCGCCTTCCGTTGATATAGACTTCCGCCCCTGTCCGCTCCAAATTCCAAAA
ATTCCGTACTGAAATTTCCATATCCCGATATTGTGCAGACCATGTTTTTTCGAAGGTTTT
CATAAAATTTCCTATACCTGTCCAATCGGCACATATCAATTGCATTATTACATCTCAATA
CGATAAATATTTCTTAAGTCAAAATGCAAGCCTGACCGTACCTTAACTGTCAAAATTTTA
TTATTTTTTATTGATTTTAAAACAATTTCTGTAAAATTCTCTTCGCTTTCTCTCTTTTTT
AGAAGCACATAAGAAAAAATAAAACTTCCCCGATTAAATTCATAAATATGTTTCAACCAT
TCGCCTCCTCTTTCTGTAAGACAAGATTCAGTTTCATTCTTCCTTATTGTATAAATATTT
CCTTCACAAAATCTGAAATAAATCCATAAATCCATCTTATCCATAATTAAAGAAAAAGTT
TCACCTCGAGATTTTGTCAACAATTCGCAAGGTTGCGATGTTGCAATCAAATAGCCGAAA
GACATTTTTTACCTCATACATGGTCGAAATCAGTTTCTGTTAGTTCAGAATCCATTTTTT
CGTCAACAACTGAATCCGCATTTTTGAATTAACGTTTTCATCAGCTGCCGTTTATCTAAA
CCGGCAGGTTCAGTTTCAAAATAAGCCTTATATGAAGACTGTAAGCATTTCAGAAAAAGA
TCATCAGAAGACATATCTGCCGAATCAAATACAACTGTTTTGATTTTGGTACTTACCCAA
AACCCTTTTTGCTCTTTTTCTACTATACGGAAATTCAGAATATTTCCAACCGAATCAAAA
GCACGGTAAACATCATCCATCAAATCCTGCGGCTCTATTTTCTTTTCCAATTCCGACAAT
CCTTGAAATATATCCAAAGACACATCTTCAAATAGAAAAAAAGGAGGAGTTAGAAGCGGT
TTTTCCATGATCTGTCCGTAGATTTTGATTCCCAAGGGCGATGACGACCAATTCCCTGTC
CAGGCAAAGTCTTGCCCGTATTATCCGTAACTCGACGATGATAATGGGGAAATTTTCCAA
TAGGATGACCTGTTCTATTACCGAAAGGGGCTATCCGCATATTATTGCCGATTTTAATCT
CACGTCCATATTTAGCAAAGGAAACAACCTTTCCTGCGGCGCCTACACCACCAGGAATTG
CGCCTAATCCGCCAGCAATAGCAACATCTCTAACAGAAGCTGGTCTGCCTGTCGTTGCAT
AACTAAAACCATGCTGTGTCCACATACCAATGGCAGCACCACCCAAGATAGCCAATGGAA
GAAACGCCCCCTCTGTCTCCTTCATCTCCTTTTGAGAAAGCTCCGCCAACTGCATCGGTG
CATCTGCCCGCGTGTGGAACACTTGGTCTTCAAATGCCTGATTGTCCAAGCCGTTTGCCA
TTGCGGGGGCAATCATAGACAGCATCATTACGGCTGCGGTGATTTGTTTTTTCATAATAA
CTCCTTTGGATTACAAGGTTGGAAAATCAAAGCCCTGCTTAGAACGTATGTTGCACACCC
AATTTCAATAGGTAAATCAGATTGCAAATCCAGCAATTTGAATATTGTCATTGTTCCGTG
CAAAAGGGATTTTTATTGATGAGTTGTGTACTGGGTTTCAGCTTGGCTTTTTAGATAATC
TTTTCACAAGAACATACATCAATACTGTCCAAAAAAAAAGATTTGATAAATATATTGCAA
CATATTTTATTCCACCTCCATCCGAAACAGAAACCAATATATTTTTAATTAATACATAGC
TGATTATTACATTTATTATTCCTATAATATATAAGGATCTTGCCAAAATTTTTTTTGATT
~~TTATTTTTAGGAATATCTCTTATCCATGCTAAAATACAGCCCAATGTCGAAAAGAAAATAA~~
CAGCAAACACTACCTTACCTCCTCTCACTTCCAACCAAACCGATAGCAGGTTTGGTTGGA
AGTTGGTTTATTTATTATTTAACGGCGAATGTCAGTGTTCTTACCCGTAGAACCTGCATA
ACCATTTATGCCACCAGCAACAGTCCCCACTGCTACACCTCGCATACCATTAATCAGAAT
TCTTCCTATTTTTTCGAATCCACTATTTCTTCCAAAGATGCAGCAAACGCAGGTTGAGCC
TAGACCCAATCTACGTTTGCTGCCGCATCTGATCCCTATTGTTTCTTATCCTTACATCTT
CCTGCCTTGTCAATCAAATAAAGACAGAAGACTATACAAAAACTGACCGACATACTGAAA
ATACCTATCCCCTTCCATGCAATCTCCGCACCATTGACCCAATAGATTAGACTGAGAAAC
AACAAAGCCACAGTATAAATCAACGTAAAAAATATTGCGTAAAGCACAATAAAGGGAACT
TTTATCTCACGGTTGTTTTTTATAATATATTCAGCAACTTGATTTCCGAATATACCTGAT
AAAAAATATAGTATTAGATCATCCATTTCGTTTATCTTCTATGTTTTCCCATTGCGGCGC
TAGAAGACTGATTTAATGCTGCACCATTTGCACGAATAACTGCATTAGGAATAGCATTTC
CCCCTTTCCAAGCAGAACGGGCAAAAACACTAGTAGTAATCCCTGCACCGCGCAACATCG
CACTGCTATATCCGCCGCCTATCATACCACCAGCGGTTGCAGCCAAGGTACTTCTTGTTG
AAGCAAATTGCCCTGTTTTAATTTTAGAAATGCCGTGATTAGCCCAAGCACTAATTGCCC
CTCCCATCAAAGCACCTGCCACAATAGGAAGAAACGCCCCCTCAGTCTCCTTCATCTCCT
TTTGAGAAAGCTCCGCCAACTGCATCGGCGCATCTGCCTGCGTGTGGAATACGTATTTTT
CAAATGCCTGATTGTCCAATCCGTTTGCCATTGCGGGGGCAATCATAGACAGCATCATTA
CGGCTGCGGTAATTTGTTTTTTCATAATAACTCCTTTGGATTACAAGGTTGGAAAATCAA
AACCTGCTTAAAATGTATGCTGTACGCCAAATTTCAGTTCGGAACTGCTTTGCCCTGAAA
CGTTGAAACGTGCGGATGCGTTTAAAGCCGTGGTTTTGGTGAAACCGAAACCTGCGCCGA
AATGGGCGTAGGTGGATGTGTTTCTGGAGGATTCCCGTTTGCCGTCCGTCCGGTCGGGCT
GCCTGCCCAGCCATTGGATGCCTCCGGTCAGGCTGATTCTGTCGTTGGCAGCAAATGAGA
TGTTGGGGTTGAGCAGCAGGTAGTTGCCCGATTTGTAGCGGATGCCGTCTGAAAGGGTTT
TGCTGCCGTTGATGCGGTAGGCGGCGGTGAGGGAAAGGACAATCGGATCTATGGCTTTGT
AGGTGGTGGCGCCGATGAGCCAGGATTTTCCCGACGAGGCTTTGTTGCGCGATTTTTCGT

## Appendix A

AAACCGTGCTTTCAAGAAAGCTGATTAGGGCGGGGTTTTTGTCGTCTTTAAGGAAAGTGT
GGCTGATGCCGAGGGATACGTCGGACATCCGTTTGTTGCGGGTTTTGCTGTTGCCGTCGA
GTTTGCGTTCTTCGTGCCACAGATAGCTGCCGCTGCCGTAAATGTCGGTATTCCCGGTCA
GTCCGTAGCGCAAACCGAGCGTGCCGACGAGCATATCGGTATTGCTGCCGTTTTCTTGGA
TTTCGGTCGGAATGGGGATAAACGAGGTTGCGCCGGTTTGAATGTAAACCGGTGCGGCAA
GTTCGGCGCGGTTGTTTTCGCTGTTCAGGTAGGTAAGGGAAGTTTCCAGTTTCCATTTTC
CCTTGTCGGTCATTATGTCTTCAATCGTCAAGGGCAGGTCGGCATAAGTGGATAAAGGCA
GGATGGCGGGCAAGGCGGGCAAAAAGATGCGCTTCATATTTCTCCTATTGATATTAATTC
TTATTTACTAATAATAATGAATATCCGATAATCTTGTCTAAATAAATTTTTAAATCATAA
AAAACAATATGTTTGTTTTTGTCAATTATGTTTCGATATGCCGTCTGAAAAGTTTGTGAA
AAACGGTTACAATCCGCCGCATGAAAAAACGCAATAATCCTCTTCCGCTGTTGAACGGTG
TCAAACCCAGTTATTTGGTGCTGCCGCATGAAAAGCAGTTTTACGGGCTGCCGCTGCTGC
ATTTTCTGTGCATCCGCTTTCCTTTTGTGGGCGCGGACGATTGGCGCAGGCGGTTGAACA
GCGGTTTTGTGGTCGGTTCGGATGGTGCGGCGTTGGACGAACATTCTTTGTTCGAGCCGG
GTAAGGTGGTGTTTTATTACCGTGAAACCAGCCGTGAGAGCGAGCCGCGTATTCCGTTTG
AAGAAAAGATTTTGCATATTGATGAGCATTTGATTGTGGTGGACAAACCGCATTTTCTGC
CCGTCATCCCCAGCGGCAGGTTTTTGCGGGAAACCCTGCTCACGCGCCTGCGTTTGCGGC
CTGAATTGCAGCATTTGAATGTTGAGGACATTACGCCGCTGCACCGCTTGGACAAGGATA
CGGCAGGCGTGATGCTGCTGTCGCACAATCCTGCCACGCGCGGAGCCTATCAGACGATGT
TTCAAAACAAAACGGTATGGAAAACGTATGAGGCGCTTGCGCCGACAAGGACGGATTTGC
CGTATCCGCTCGATGTGGTTTCGCGTTTGGTGAGGGGTGAGAAATTTTTTACGACGCAAG
AGGCGGAAGGCGAACCAAACGCACATACGACGGTCGAACTGATTGAAAACAGGGGGGAAT
TCAGCCTTTACCGCCTTACGCCGCATACGGGCAAGAAACACCAGTTGCGCGTGCATATGA
TGGGTTTGGGTATGCCGCTGTTGAATGACGCGCTCTATCCCGTGCCGTCTGAAGCGGGCA
GCGAGGATTATCGGAAACCTTTGAAGCTATTGGCAAAAAAGATTGCGTTTGCAGATCCTT
TGTCGGGTCGGGAAAGGGTGTTTTGCAGCGGATTTTGCCTATAATGAGAGGGAACGCAAA
CCCGTATCGGCGGATGCCGCCGATACGGGTTTTACATTATCGATGCCGTCTGAAAGGGGT
TTTATCCGTTCAGGCATACGGGCGCGAAGCGGAGGATGGTGTCGCGCAGGACGATGAGTT
TGCCGTGAAAGAAATGCGTGGAACCGGCGATGGTAATGACGGGCAAATCTTGCGGTTCCG
CCCATTTCAGTGCTTTCCCGATTTCGACGACTTCGTCTTCCGCGCCGTGTATCATCAGCG
TTTTGGCAACGTTGGGAACGGCGGACGGTTCCGGACGGTCGGTATAGTGGCAGACTGCCG
CGCCGATGAGCAGCAATAAATCGGGTGTGCGCGCTTGTGCGGCAAATGTGGCGACATAAC
CGCCGAAGGAGAAGCCGGATAAGGCAAATTCGGGGGCTTCGGGGTGTTGGGCGCGGGCAT
AATCGATGACGGCGAGGCAGTCTTGCGTTTCGCCGCGTCCGTAATCATGTGTGCCTCCGC
TGCCGCCTACGCCGCGAAGGTTGGGCAGGTAGCAGTGGAAGCCGAGTTTGCTTAAGGCTT
TGGCGGCAGTTTGGATGACTTTGTTGGTGTTTGTTCCGCCCTGGAGGGGGTTGGGATGAT
TGATGACGGCAACACCGCGTGCCGGTACTTGTTCGGACGGGATATGGATGGTTTCTAAAA
TGCCGGCAGGACCGGATATGTGTATGGTTTCGGGTTTCAGCATAATGATTTCCTCTTGAT
TTTCCGCATGTTCCGATGCAGCCGTGAAGATAGTTGCTTAAAGGTGGAAATGCTTGCCGT
TGTTGGGGTTGGACGCTTTCAGGCCGGCAAGCATACGGTGGAAGTCCAATCCGTATGCGT
TGCTCAGTTTGTCGGCACGTTTTTTGAGTTTGTCTATGTTTTGCTCTTTGGGGCTGCTTT
GGAAGGCCATTACCGCGACATTGCCGTGGCTTTCGGCAGGAAGTTCCAAGACGCGCCCTT
CAAAAACGCTCAACAACCGTTCGATGAAGCGTTGGTAGCGTTTGTCGCCGCTCCACCAGT
TGGTTACGAATATGCCGTCTGAAGAGAGTGCGTTGCGGCAGTCTCGGAAGAACGGTTCTT
CAACCAGCGCATCGATGATTTGTTCGCCGTCGAACCCGTCCACCAAAATCACATCGGTGT
TGTGGCGGAAGACTTTGATATATTCTGCACCGTCTGCTTCAATAATTTCAAATTTCTCGC
CCTCGAAAGGCAACTCGAACAGGTTGCGGGCAATGGCGATGACCTGCGGATTGATGTCCA
CGGCGGTTTGGCGCGTGTCGGGCAGGTAGGTATCTATCCAGCGTGCAAACGAGCCGCCGC
CCAAGCCGATTTGGGTGATATGTTGCGGAAGAGCGTCGGTAAACAGCAGCCAGCCCATCA
TCGCGCGGCTGTAAGAGAGCACCAGCTCGGACGGGTGGTCGAGGTTCATCGAGCTTTGAA
CGGTGTCGCTGCCCAAGTGAAGCGAACGGATATTGCCTTCTTCGGAAATGCCGACTTCGG
GAAAGCCGGATTTTGCAGGACGCAGGCGGCGGTAGGGATGTCTTGCCATCGGTGTGAACG
GTCGGTTTGAAAGGCGGATATTTTATCAGAATGCCGATGCTTGTTCTGTTTCAAATTAAT
TTCTTTTCAAATAAATTACTTATTCGGATTTGCCGGGGCTTTCGGATAAATTCCTTGCCA
AGGTGCGGCATTGCCTGCATAATTCGCTTTCTTTGCCGGTATAGCTCAGTTGGTAGAGCA
CCTGACTTGTAATCAGGGGGTCCCGAGTTCGACTCTTGGTGCCGGCACCATTTCTGCTGA
TGGTGTTGCAAAACATTTCAGTAAGCCGGTATAGCTCAGTTGGTAGAGCACCTGACTTGT
AATCAGGGGGTCCCGAGTTCGACTCTTGGTGCCGGCACCAGATTTGACAGTCCGATTGTG
TAAAACAGTCGGGCTGTTTTGCATTTGCGGCACGGTCGGTGGCAGGGCTGCGGGCATTTC
ACTATAATAGCCCGTTTCAAACTGAAAAGGCCGTCTGAAAAGGGCGGGGTAACAATATCT
GATGATTACTGTGAACACACTGCAAAAAATGAAGGCGGCGGGCGAAAAAATCGCTATGCT
GACCGCTTACGAATCCAGTTTTGCCGCGCTGATGGACGATGCCGGCGTGGAAATGCTGCT
GGTCGGCGATTCTTTGGGGATGGCGGTTCAGGGGCGGAAATCGACGCTGCCCGTCAGCCT
GCGCGATATGTGTTATCACACCGAATGTGTAGCACGCGGTGCAAAAAATGCGATGATTGT
CAGCGATTTGCCGTTTGGTGCATATCAGCAGAGTAAGGAGCAGGCGTTTGCCGCCGCCGC
CGAACTGATGGCTGCCGGCGCGCATATGGTTAAACTCGAAGGCGGCGTGTGGATGGCGGA
AACGACTGAATTTTTGCAAATGCGCGGTATTCCGGTTTGTGCGCACATCGGTCTGACCCC
GCAATCCGTGTTTGCCTTCGGCGGATATAAAGTTCAGGGGCGCGGCGGCAAGGCGCAGGC
GTTGCTTAACGATGCCAAGGCGCATGACGATGCGGGGGCGGCGGTCGTGCTGATGGAGTG
CGTACTGGCGGAACTGGCAAAAAAGGTAACTGAAACTGTTTCCTGTCCGACCATCGGCAT
CGGCGCGGGTGCGGATTGCGACGGGCAGGTTTTGGTGATGCACGATATGCTCGGCATTTT
CCCGGGTAAGACGGCGAAATTCGTCAAAAACTTTATGCAGGGGCATGATAGTGTTCAAGC
GGCGGTTCGGGCGTATGTTGCCGAAGTCAAAGCCAAAACCTTCCCTGCTGCGGAACATAT
TTTTGCAGATTGAGCGGTTTACATGCAGCATGCCGTCTGAAAGCCGTTTCAGACGGCATT
TTGTTTTGCCGTGCGCGGCGCGTATAATCGGCGCGTTTTGTCGGGCAGGAAGCCCGAAGG

## Appendix A

ATAAGGATTACCGTTATGCAAATCATACATACCATTCGAGAACTGCGCGCGTGGCGTAAA
AATGCGGGAAAGGTGGCATTTGTGCCGACCATGGGCAATCTGCATGAAGGACATCTTGCG
CTTGTGCGTGAGGCGAAAAAACGCGCGGACAGTGTCGTGGTCAGCATTTTCGTCAATCGC
CTGCAATTCGGTCAGGGCGAGGATTTCGACAAATATCCGCGCACTTTGCAACAGGATGCG
GACAAACTTGCCGCCGAAGGCATTGCCGTTGTTTTCGCGCCCGATGAGAAAGAACTCTAT
CCGAACGTGGAACAGCGTTACAACGTCGAACCGCCCAATCTGCAAAATGAGTTGTGCGGC
AAATTCCGCCCGGGGCATTTTCGCGGTGTGGCAACGGTTGTTTCTAAATTGTTCCACATC
GTTTCCCCGGACATTGCCTGTTTTGGTAAGAAGGATTACCAGCAGCTTGCCGTGATTAAA
GGTTTTGTCGAAGATTTGAATTTTGATGTTGAAATAGTGCCTGTTGATACAGGGCGCGCG
GAAGACGGGTTGGCACTGTCGAGCCGCAACCAGTATTTGAGTGCGGCGGAACGCGACGAA
GCACCGCGCCTGTACCGCGAATTAAAGGCTGTTGCCGAATCCTTGGTGCAGGGCAGTTTG
GATTATGCAGGTTTGGAAAAACGTGCCGTCCAATCCCTGACAGAATACGGCTGGGTGGTC
GATTATGTCGAAATCCGCCGCGCCGATACGCTCGAAGTGGCGCGGGCGGGAGATAAGAAA
CTGGTGGTCTTGGCCGCCGCCTGTCTGGGGACGACGCGCCTGATTGACAATTTGGAAATA
AAACTCCCTTAAACCGCAAGCGTCGGGAATGCCGTCTGAAGCGGATTTGCGTTTCAGACG
GCATTTATTTTTTGAACGGGGTTTCGCAAATCTACAAACGATTCTGCTTGTGATAAAGTT
ACGCCTGATTATATGCCGTCTGAAGGTTCGGACGGCTGTCGGATAAAGGATGATTATGTT
ACCTAACCGTTTCAAAATGTTAACTGTGTTGACGGCAACCTTGATTGCCGGACAGGTATC
TGCCGCCGGAGGCGGTGCGGGGGATATGAAACAGCCGAAGGAAGTCGGAAAGGTTTTCAG
AAAGCAGCAGCGTTACAGCGAGGAAGAAATCAAAAACGAACGCGCACGGCTTGCGGCAGT
GGGCGAGCGGGTTAATCAGATATTTACGTTGCTGGGAGGGGAAACCGCCTTGCAAAAGGG
GCAGGCGGGAACGGCTCTGGCAACCTATATGCTGATGTTGGAACGCACAAAATCCCCCGA
AGTCGCCGAACGCGCCTTGGAAATGGCCGTGTCGCTGAACGCGTTTGAACAGGCGGAAAT
GATTTATCAGAAATGGCGGCAGATTGAGCCTATACCGGGTAAGGCGCAAAAACGGGCGGG
GTGGCTGCGGAACGTGCTGAGGGAAAGAGGAAATCAGCATCTGGACGGACTGGAAGAAGT
GCTGGCTCAGGCGGACGAAGGACAGAACCGCAGGGTGTTTTTATTGTTGGCACAAGCCGC
CGTGCAACAGGACGGGTTGGCGCAAAAAGCATCGAAAGCGGTTCGCCGCGCGGCGTTGAA
ATATGAACATCTGCCCGAAGCGGCGGTTGCCGATGTGGTGTTCAGCGTACAGGGACGCGA
AAAGGAAAAGGCAATCGGAGCTTTGCAGCGTTTGGCGAAGCTCGATACGGAAATATTGCC
CCCCACTTTAATGACGTTGCGTCTGACTGCACGCAAATATCCCGAAATACTCGACGGCTT
TTTCGAGCAGACAGACACCCAAAACCTTTCGGCCGTCTGGCAGGAAATGGAAATTATGAA
TCTGGTTTCCCTGCACAGGCTGGATGATGCCTATGCGCGTTTGAACGTGCTGTTGGAACG
CAATCCGAATGCAGACCTGTATATTCAGGCAGCGATATTGGCGGCAAACCGAAAAGAAGG
TGCTTCCGTTATCGACGGCTACGCCGAAAAGGCATACGGCAGGGGGACGGAGGAACAGCG
GAGCAGGGCGGCGCTAACGGCGGCGATGATGTATGCCGACCGCAGGGATTACGCCAAAGT
CAGGCAGTGGCTGAAAAAAGTATCCGCGCCGGAATACCTGTTCGACAAAGGTGTGCTGGC
GGCTGCGGCGGCTGTCGAGTTGGACGGCGGCAGGGCGGCTTTGCGGCAGATCGGCAGGGT
GCGGAAACTTCCCGAACAGCAGGGGCGGTATTTTACGGCAGACAATTTGTCCAAAATACA
GATGCTCGCCCTGTCGAAGCTGCCCGATAAACGGGAGGCTTTGAGGGGGTTGGACAAGAT
TATCGAAAAACCGCCTGCCGGCAGTAATACAGAGTTACAGGCAGAGGCATTGGTACAGCG
GTCAGTTGTTTACGATCGGCTTGGCAAGCGGAAAAAAATGATTTCAGATCTTGAAAGGGC
GTTCAGGCTTGCACCCGATAACGCTCAGATTATGAATAATCTGGGCTACAGCCTGCTGAC
CGATTCCAAACGTTTGGACGAAGGTTTCGCCCTGCTTCAGACGGCATACCAAATCAACCC
GGACGATACCGCTGTCAACGACAGCATAGGCTGGGCGTATTACCTGAAAGGCGACGCGGA
AAGCGCGCTGCCGTATCTGCGGTATTCGTTTGAAAACGACCCCGAGCCCGAAGTTGCCGC
CCATTTGGGCGAAGTGTTGTGGGCATTGGGCGAACGCGATCAGGCGGTTGACGTATGGAC
GCAGGCGGCACACCTTACGGGAGACAAGAAAATATGGCGGGAAACGCTCAAACGTCACGG
CATCGCATTGCCCCAACCTTCCCGAAAACCTCGGAAATAATGCAGGTCCATCCTTTCAGA
CGGCATAAGGTTTGCCGGGAAGCCGGGGCATTCGGGCAAACGGCACGCAGTTCGCACGCG
TTTTGCACGGCACGCCGAACCCATCGGCCGGCAGGATGGCATCCGTTAAGGAAATTCTGA
TGAAACACACCGTATCCGCATCGGTCATCCTGCTTTTGACCGCTTGCGCGCAATTACCTC
AAAATAACGAAAACCTGTGGCAGCCGTCCGAACACATCAGCAGTTTTGCAGCAGAAGGGC
GGTTGGCAGTGAAAGCGGAAGGGAAAGGTTCGTATGCAAATTTCGATTGGACATACCAAC
CGCCCGTGGAAACCATCAATATCAATACCCCTTTGGGCAGTACGCTCGGGCAGTTGTGTC
AAGACAGGGACGGCGCATTGGCAGTGGACGGCAAAGGAAATGTCTATCAGGCGGAAAGTG
CGGAAGAATTGAGCAGGCAGCTGGTCGGTTTCAAACTGCCAATCCAATATCTGCATATCT
GGGCAGATGGCAGGCGTGTGGCGGGCGCGCCTTACCGCATCCTGCCGGACGGCATATTGG
AACAATACGGTTGGACTGTCGGCAGAACCGCCGACAGTGGGGGGCAAGTCCGAACGTTGC
AACTGAATAACGGAAATTTGAACATCAGGCTGGTTTTCACCGAAATCGGTATGCCGTCTG
AAACCGAAACCCCGGAACGCTGTGCGGCGCGCACGAGATAAGGCGGACAGATGAATATTG
CGGACGGACGGCAGGCGTTTTCCGCACCTGCAAAACTGAATCTCGATTTGAGGATTACCG
GCAGGCGGGAAGACGGTTATCACAATATCGAAAGCATATTCTGCCTGATAGATTTGCAGG
ATACCGTATATTTGAAACCGAGGGACGACGGCAAAATCATCCTGCACAATCCGGTTGATG
GCATGCCGCAGGAAGTAGATTTGAGCTACCGTGCCGCATCGTTGCTGCAAAAATATGCGC
GCAACCCCGCCGGCGTGGAAATATGGCTGGACAAAAAAATCCCGACAGGGGCGGGTTTGG
GCGGCGGAAGCTCGGATGCGGCAACCGTTTTGCTGGTGTTGAACCGTTGGTGGCAGTGCG
GTCTGACGCAGCGGCAGCTCATTGATTCGGGCGCGGCTCTGGGGGCGGACGTACCGTTTT
TTATTTTCGGCAAAAATGCGTTTGCGCGGGGTATAGGCGACAGGCTGGACGAAATGGATA
TTCCGAAACAGTGGTATGTCATCGTCAAACCGCCCGTCCACGTTTCCACTGCAAAAATTT
TCACACACGAAAGCTTGACACGAAATTCCGCCTCAAGCATAATGCCGACTTTCCAAAATC
TGCAACCGTTTAGAAATGATATGCAGGCAGTTGTATTTAAAGAATACCCTGAAGTTTGGA
AAGCCTATTCCGAGTTGTCCCGATATGGATTTGCCTTAATGACAGGTTCCGGTGCGTGTG
TATTCACGGCGTGTCAAGATAGGAATAGCGCATACAATATATACCGACAAGTTTCAGATT
TGTACGAGGCATATTTGGCAGAGGGTCTTTCAAAACATCCTTTGTTGTCCGTATAAACAT
TGTTGGGGAGTCGTCAAGCGGTTAAGACACTGGATTTTGATTCCAGCATGCGAAGGTTCG

## Appendix A

AATCCTTCCTCCCCAGCCAAGTCAAACGAGTTGGGGAGTCGTCAAGCGGTTAAGACACTG
GATTTTGATTCCAGCATGCGAAGGTTCGAATCCTTCCTCCCCAGCCAGCCCTTTTTGGGG
AGTCGTCAAGCGGTTAAGACACTGGATTTTGATTCCAGCATGCGAAGGTTCGAATCCTTC
CTCCCCAGCCAAATAAAAGCGTGTAAGCCTGCTTACACGCTTTTATTTCATAGAAATAAA
AATATTGAAATGCCTTTGTTTGTGTCGGATGTTGCAGGTATAATGTCGGGCTTGGTACAA
GCAGAGGGAAGCATTGTGTTTTCTGAGCGGAAGTTAAACATAAAATCAGGTGAGAATATG
GCTGCGTACGACAGTTTGATGGTATTTACAGGCAATGCCAATCCCGAATTGGCACAACGT
GTTGTCAGGCATTTGGACATTTCTTTGGGCAATGCTTCCGTATCCAAGTTTTCAGACGGC
GAAGTTGCCGTCGAACTGTTGGAAAACGTACGCGGGCGCGATGTTTTCATCCTTCAGCCG
ACCTGTGCGCCGACCAATGACAACCTGATGGAAATCCTGACGATGGCGGATGCACTGAAG
CGTGCTTCGGCAGGTCGTATTACCACAGCCATTCCGTATTTCGGCTATGCGCGCCAAGAC
CGCCGTCCGCGTTCCGTCCGCGTTCCGATTTCTGCCAAACTGGTGGCAAATATGCTGTAT
TCGGCAGGGATCGACCGTGTTTTGACTGTCGATTTGCATGCCGACCAGATTCAAGGTTTC
TTCGATATTCCGGTGGACAATATTTATGCCACCCCGATTCTGTTGAACGACATCAAACAA
CAGCGGATTGAAAATCTGACCGTCGTCAGCCCGGACATCGGCGGTGTCGTCCGCGCCCGC
GCCGTGGCAAAATCCCTGAATGCCGACTTGGCAATCATCGACAAACGCCGCCCGAAAGCC
AATGTGGCGGAAGTCATGAACATCATCGGCGATATTCAAGGTAGAACCTGTCTGATTGTG
GACGATATGATTGACACTGCAAATACGCTGTGCAAAGCCGCCGTCGCCCTGAAAGAGCGG
GGGGCTGAACGTGTTCTAGCCTATGCCAGCCACGCCGTATTCTCCGGAGAGGCGGTCAGC
CGTATCGCCTCATCCGAAATCGACCAGGTGGTCGTAACCGATACCATTCCTTTGTCTGAA
GCGGCTAAAAACTGCGACCGTATCCGTCAGGTAACGATTGCCGGTCTGTTGGCCGAAACC
GTCCGCCGCATTAGCAATGAAGAATCCGTCTCATATCTTTTCAATGAAGAAGTGATGACA
GGCAGCATGTTGCTGCCATAAGCCCGAAGCCGTCTTAAGCTGGTCGCGGCCGATGACGGC
GATTTTACCTAACTTGGAGTATTTAACATGACTTATGAAATTCAAGCCTCTGTTCGTGAA
GCACAAGGCACTGGTGCGAGCCGCCGCCTGCGTCGCGAAGGCCAAATCCCCGGCATTCTG
TACGGTGAAGGTCAAGAGCCTGTTGCAATCGCTGTGGATCACAAAACCGTATTCTACGCA
TTGGAAAAAGAATCTTTCCATACTGCGTTGATTAAGTTGTCTCTGAACGGTGAAACCAAA
GACGTTATCGTCCGTGATTTCCAAATGCACCCGTTCCGCCGCGAAGTTCAACACATCGAC
TTCCAAGCTGTGAAAGCCGATCAACTTGTACGCATCCGTGTTCCCCTGCACATCGTTAAC
GCTGAAAATTCCCAAGCGGTCAAACTGCAAGGCGGCCGCGTATCTCTGTTAAACACTTCT
GTTGAAGTAGTTGCTTTGCCTGCCAACATCCCTGCTTTCTTGGATTTGGATTGTGCTGAA
GTGGTTGCCGGCGACATTCTGCACTTGTCAGACATCAAACTGCCTGAAGGTGTAGAAAGC
GTTTCCCTGAAACGTAACGAAAATCTGGCTGTTGCTACCGTTACCGGTAAGAAACGCTAA
TTGATTTCAGCAGCAGGGCGGCGCGTATGCAATACGTACCGCCCTGTTGTTTTATGCCGT
CTGAACCGTGTTTCAGACGGCATTTCTTTATTTGTTGGAAAAACGGGATATTTGAAACGG
CAGATTACTGCCCTGTCAGACACGCCCAAAGCCTTTGCCACCGGCTTCTTTTTTTTACAT
TTTCCAGTGCGACGATTTCTTTTTCGGCAATGGTGTATCCGTTTTGTCTGATTTTGATTT
TTCCTAAAATTTGCCCTTTTTTTACTGGGGCGGGAATCGGCTGTATGGTTTCTAGAATTT
GTTCTGCCATTTTCGCTTCCTTATGTGGCAGAGTGATGTAGGCTTCTTTGAGGAAGCCTG
CGCGGACGGTTTTTTTGCTGCCTCCGGAAATTTGGATTTGGGCAACGGTTTTGCCTTTCG
GATATATTTTGGGCGTATCGAAGGCCTGCAATGCCCAGTTCAGCAGCTTGCTGTTGTCTG
ATGCGCGTGTTTCCGCCGATTCCGAACCCAATGTGATGACAAGGATGTGCCTGCCGTTGC
CGGAGTATGACACGGCAAGGTTGTAGCCGCCGCTTTCTGTGTGTCCGGCTTTCAGACCGT
TTACATTGTTGTCCCTATATAAAAGGATATTGCGGTTGTTTTGTTCTATATTTTTGAATT
TGAAAGATTTGATGGAAAACAGCGGGTAATATTCCGGAAAGTCGCGCATCAATGCTTCAG
ACAGCAGGGCGAGGTCTTTGGCGGTGGAAACCTGTCCTTCTCTACTCAAGCCTGTCGGGT
TTTTGAATACAGTGTTCTTCATGCCCAAGCGTCGGGCTTCTTTGTTCATTTGTTGCACAA
AATTTTCAATCGAGCCGTTGCCCAGCCGGCCGGCAAGGGTTAGGGCGGCATCGTTTGCGG
ATAGTGCAATCATGCCTTTTAAGAGTTTGTCGGTGCTGACCGTATCGCCGGGACGTACAA
ACATTCTGCTTCCTTCTGAAGCCCATGCGGATTCGGGTATTTTTAAGTTTTCTTCAGATT
GGATATTGCCCGATTTCATGTTTTTGAAAACCAGATATGCGGTCATCAGTTGGGTTAGTG
CCGCCGGTTCAACAGGGGTATTGATGTTTTTGGCGGATAAAATCTGTTTGCTTTGAAGGT
CGATAACGATGTGTGCCGCTGTGAGGGTTTCGGGTGTTTGGAACGTGGGGGCGGCGTGTA
CCGTCGGTCTGTTGGGCGCGGGCGATGCAGCCGTTGCGTGAGAAACGCCTAAGATGATGG
AAAGCAGGACGGGCAGGATTTTATGTGCTGTCATGAAATATTCTAATTGTGTGCGTGTTT
CAGTCTGCCGATTATACGCTTAGGGTGTCTGATCGGGCGGATTTTTCTTGATTTCGCGCC
GTCTTGGGCGTATGGTTTTGGGTTTTGCGATTTTAATAAACCGATTATCCCATATTGAAT
TATGAACACGCCCCTTCCTTATTCCGATTACCTCATCCGCATCCTGACGGCATCTGTCTA
TGATGTGGCGGTCGAAACGCCTTTGGAACCGGCACGCAGCCTTTCTGTACGTTTGAAAAA
CAACATCCTTTTGAAACGCGAAGATTTGCAGCCGGTTTTTTCGTTCAAAATACGCGGCGC
GTACAACAAAATGTCCAAGTTGCCGAAAGATGCGCTCGCTTGCGGCGTGATTGCGGCAAG
CGCGGGCAATCATGCTCAAGGCGTGGCATTGTCCGCACAGCGTTTGGGCTGCCGTGCCGT
TATCGTTATGCCGGAGACTACGCCGAAAATCAAAGTGGATGCGGTTAAAAGCCATGGCGG
CGAGGTGGTTTTGCGGGGCGTTTCATACAACGATGCCTACGATTATGCGATGGAGTTGGC
GGAAAAAGAAGGGTTAACCTATATCGCGCCGTTTGATGATCCTGATGTGATTGCGGGACA
GGGGACGGTGGGGATGGAAATTGTCAGCCAGCATCCCGATCCAATCCGCGCCGTATTCGT
ACCGATAGGCGGTGGCGGTTTGGCGGCGGGCGTGGCGGCATTTATCAAGCAGGTCCGTCC
CGAAATCAAAGTTATCGGCGTTCAGACCAACGATTCCTGCTGTATGAAGCAGTCGGTCGA
AGCGGGTGAAATCGTCCATTTGAAAGATGTCGGGCTGTTTTCAGACGGCACTGCGGTCAA
AGTCGTCGGAAACGAAACCTTCCGCCTCTGCAAAGAACTTTTGGATGAAATCATTACAGT
CGATACCGATGCGGTTTGCGGCGCGGTCAAGGATATTTTCGATGACACGCGCAGCATTAC
CGAGCCGGCGGGCGCGTTGGCGTTGGCGGGTCTGAAAGCCTATATCGCCCGAGAAGGCGC
GGAAAACCAAACCCTGATTGCCGTTACCAGCGGTGCGAATATGAATTTTCACCGTTTGCG
CCACGTTTCGGAACGGAGCGAATTGGGCGAGGGCAACGAAGGTATTTTTGCCGTTACCAT
CCCTGAAGAACGCGGCAGCTTCCTTAAGTTTGTCAATATATTGGGAAATAGGAATATTAC

## Appendix A

CGAGTTCAACTACCGCTACGGAGACGATGAAAAAGCGCATATCTTTGTCGGACTTCAAGC
GGCAGGCCCGCAGGATTTGGCGGTTATCGGCAGCCGGTTGGATGAGGCGGGATTGCCCAA
TGTCGATTTGACCAACAATGAGATTGCCAAAATCCATATCCGCTATATGGTCGGAGGGCG
GACGGACAAAGTAGAAAACGAGCGTTTGGTCAGTTTTGAGTTTCCGGAGCGTCCGGGGGC
ATTGGCACGCTTTTTGAACCATATGCAGGGAGGGTGGAATATTACGCTTTTCCATTACCG
CAACCACGGTGCGGATTACGGGCGGATTTTGGTCGGTATCGATGTGCCGCCGCACGATGC
CGCCGCATTTGACGGTTTCTTGGAAAGTCTGGGATACAGCTATCACGAGGAAACGCAAAA
TGCCGCGTACAAGCTGTTTCTTGCCTGACGCTTGAAAGCACAATGCCGTCTGAAAGCCTT
TCAGACGGCATTGCGCTTTCATGGTTAAATCGAATATTCAATCAGTTCGTTTTGAGAGAA
GACATAAACCTGTTTCGGAATCAGCTTCAATTCTTTGCCTTCGGCGATTGGGTAACGCGC
GGCATCGCTGCCTGCCAGCGTGATATGTACGTCCTGTTTGTCGTGTTTTACCAGAATATG
CGTCAATGCGCCGACGGCGTGGATTTTTTCGATTTCGGCACAAATCATCGGTGTTTCGTG
TTCGGCGGCGATCTGCCATTCGTGCGGGCGGATATAGCCGGTGGCGGTTTGTTCCTGCCA
TTTGTATTGCGCGTCCAATTTCCACGCGAAGCCGTTGTAATGCCAGAAGCCTTTTTCGAT
GCGTCCTTCAAAAGCGTCGGTTTCGCCGAGGAACTCGGTAACGAAGGCATTTTCGGGTTT
GCGGTAAATAGCTTCGGCGCTGCCGGTTTGTTCGATTTTGCCGTGGTTCATCACGACGAT
TTCGTCGGAAACTTCGAGGGCTTCTTCTTGGTCGTGCGTAACCAGAATGCTGGTTACACC
CAGGTTGTGATGGATGTCGCGCAGCCAGGTGCGTAATTCTTTGCGTACTTTGGCATCCAA
CGCGCCGAAGGGTTCGTCCAAAAGCAAGAGTTTGGGTTCGACCGCAAGCGCGCGGGCGAG
GGCGATGCGCTGGCGTTGCCCGCCGGAGAGTTGGTGCGGATAGGATTTTGCCAAATGAGA
GAGCTGCACGAGCTTGAGTAATTCTTCGACTTTGGCGCGGATTTGTCCTTTGGACGGGCG
TTCGGACTTGGGCAATACGGTCAAACCGAAAGCGACGTTGTCAAACACGTTCATATGGCG
GAAAAGGGCGTAGTGTTGGAACACGAAGCCGACTTTGCGCTCGCGCACATGTTTGGCGGT
TACGTCTTGCCCGTCAAACAGGATATTGCCGCCGTCGGCGTTTTCCAGTCCGGCGATAAT
GCGTAAAAGTGTGGTTTTGCCGCAGCCGGACGGGCCGAGCAGGGAAACGAGTTTGCCGGT
GGGGACGTTGAGGTTGATGTTTTTCAGCGCGTGAAAATTGCCGAAGTGTTTGTTTAAGTT
TTGGATGGTGATACTCATATTGCATTCCTTTCGGCGGCGGCGAGTTTTTTGTCTTGTAAT
TTGGTAATGATGTTCTGCACCGCCAGCGTCGCCAGTGCCAAAAGTGCCAATACGCCGGAG
AGGGCGAATGCGCCGGTGAAGTTGTATTCGTTGTAGAAGATTTCGACCAAAAGCGGGACG
GTGTTGGTTTCGCCGCGTATGTGTCCCGATACCACGCTGACCGCGCCGAACTCGCCCATC
GCGCGGGCGTTGGTGAGGATGATGCCGTAGAGTAACGCCCATTTGATGTTGGGCAGGGTA
ACGCGCCAAAACATCTGCCAGCCGCTTGCGCCGAGTATCAATGCCGCCTGTTCTTCGCTG
TCGCCCTGTGCCTGCATCAGCGGGATGATTTCGCGTGCGACAAAGGGGAAGGTAACGAAC
AGCGTCGCCAAAACAATACCGGGGATGGCGAAGATAATCTGTATGCCTTGCGCTTCGAGC
CAGCCACCCAATGCCGTATGCGCGCCGAACAATAAGACGAACATCAAACCGGCCACCACG
GGCGATACGGAAAACGGCAAATCGAGCAGGGTGGTCAGCAACTGCTTGCCGCGAAAATCA
AAACGGGTCAGCAGCCACGCCATCGCCACACCCAATACGGCATTGACGGGAACGACAATC
AGCGCGGTAATCAGCGTCAATTTGATGGCAGACCACGCTTCGGGATCGTTTAAGGATTTC
AGGTACAAATCCCAACCGCCTTTTAAGGCTTCGTAAAACACGGCGACGAGCGGCACGACC
AGCATCAGCAGCAGAAAGCCCAGCGCGGCGGCAATCAGCAACACGCGCAGCCGGCGCGGT
TCGGTCAGGTTGGGATTGGCGGAATAGGGTTTCATGGCGGTGGTGTTCTCTGGTTGGTTT
TAAAATGTTTGGATGCCGTCTGAAAAACCGTTTTTTGTGGGGCGGATTCGTTTTCAGACA
ACCTTTTATTGATTAAGGGAATAAAAACGTCTAGCTACCGTCATTCCCGCGCAGGCGGGA
ATCCACCGCAGGGCAACAGGAAAACAGAAAATAAATAAGGCAGCCGAAATTCACCAATGG
ATTCCCGCCTGCGCGGGAATGACGGTAACAGGTATTTCAGACGACCTCAACCCTTCGCGC
CCGAACGCCTGCCCAACGCCCACTGCATCACGTTCAGCGCAAACAGAATCACAAACGAAA
CCAGCAGCATAAACAACGCCACCGCCGACGCGCCCTGCACGTCGAACTGTTCCAGCTTGC
CCGTAATAATCAGCGGCAGGATTTCAGAAACCATCGGAATGTTGCCCGCGATAAAAATCA
CCGAACCGTATTCCCCCGTTGCCCGCGCAAACATCATTCCCGCGCCGGTCAAGAGTGCCG
~~GTGTGATTTCAGGCAAGAGGAGAGGGCGAAACGTAGTCCAACGGCTTGCGCCCAAAGTTG~~
CCGCCGCTTCCTCATATTCGCCCGACAATTCTTCCAATACCGGCTGCACGGCGCGGACGA
TAAAGGGCAGGCTGACGACGACCAGCGCAATCCAAATGCCGACGGGTGTAAACGCGATTT
TGATGCCCAAAGGCTCGAAAAAACGGCCTATCCAACCGTTGGGCGCATACAGGGTTGCCA
ACGCGATACCCGTAACCGCCGTCGGCAGCGCAAACGGCAAATCGACCAGCGCGTTCGCCA
GACCCTTGCCCGGGAATTCATAACGCACCAATACCCACGCCACCAGCGTGCCGAACACGA
CATTGGTCAGCATCGCATAAAACGACATCCGCAAGCTCAGCCATACCGCCGCCAACACGT
TCGGCTCGGCAATCGTGTTCCAAAAGCCGCCCCAGCCGATTTCCGCCGCCTTCGCCGCCA
TCATCGCAAACGGCAAGACCACAAGCAGCGACAGGCACAATACGGTCAGACCAAGGCTGA
GTTTGAAGCCGGGCAGTACGCCGGGCGTTTTGAGCGCTAACATAAAACAATGCTGAAAAT
AAGGAAAAGGAAGGACTACTTTAACGATGCCGTCCGAAAAACGGAAAGAATGGAAAGTTT
GGTGCAAAGACGAATTTGTTATAAAGCGGTTGGCAGTTTCTCAAGCGGGCGCGATGTTTT
AAAATATAGTGGATTAACTTTAAATCAGGACAAGGCGACGAAGCCGCAGACAGTACAAAT
AGTACGGCAAGGCGAGGCAACGCCGTACTGGTTTAAATTTAATCCACTATAACACCTTGT
TTTGACGGAAAACCATCATATAAAGGAACACTTATGCAGATTTTATCTTTTCAACCGGAC
ATTGCGGAACGTATGCTGGAAGGTACGGAAGGCGAGTCGGTCAACGAAAACGCACAATTC
GTCCGTACGGACAACGGCTATTGGATTGCGTGGCATGAAGGCGTAGCGGCACTGCTTGCG
CCCGATATGCCGCCGGGCATTCCCTGTTTTTGGGTGGAAGGCGCGGAAAGCCTTGAAGAG
TTGTGCGTCATGGTGGAACGCGGCGAGTTTGACGAAGTGGAAGAGTTTGACGGCGATGAC
GACGAATGGCTCGAAACGGCACAGGGTTGCGGGCACCACGGCGACGCTTGCGCCTGCGGA
CATTAAAGGCATTGCAGGCTTGCCGCAAGGGGGGCAAGGCTTTGCCGTTTTTTAAATAAT
CGGTGGATTGCATCTGTTTGCAATGGGCTGAACGCTTCCCTTTGTATTTTATTGAATAAA
AAAACTTATCTTGGTATTATAATTAAGGCAGCATCAATTATTTTGGGATGGCAATAAACG
CAAAGCATTGATTTGCGCCGATTGCAGACTTATTATAGCAGGTTGCGGCGCGGACTTAAT
GGGAATTGTTTATAGAGCATGATGGTCTGAAACATCATTAATTAAAAATACCATTAATCC
GATTTATATTTATTTCAATTTCAATGGAAAAACATCAATGACAATGATTTTAAGCATTTT

## Appendix A

AAGCCTGTTTTTTATCATCAGACTGTTATTTTTAGCCGTCTCTATTAAACATGAAAAAGC
CTTGATTGCCAAAGGGGCGAAACAATACGGAAAAACCAATTCCACGCTGCTTGCGGCAGT
TCATACGCTTTATTATTTGGCGTGTTTTGTTTGGGTATGGCTTTCTGACACTGCTTTTAA
TGGCATATCCTTGATTGGTACGCTGACGGTGATGGCTTCGTTTGTGATATTGTCATTGAT
TATTAAGCAGTTGGGGGAGATTTGGACGGTTAAAATCTATATTTTACCAAATCATCAAAT
TAATCGTTCGTGGTTGTTTAAAACATTCCGCCACCCCAATTATTTTTTAAACATCATACC
CGAACTGATTGGCATCGCCTTATTATGTCAAGCGTGGTATGTTTTATTGATTGGCCTGCC
CATTTATTTGCTGGTCTTATTTAAGCGTATCCGACAAGAAGAACAGGCGATGGCAACACT
TTTTTAACCCGTTTCATCAATTATAGCGGATTAACAAAAACCAGTACGGCGTTGCCTCGC
CTTGCCGTACTGGTTTTTGTTAATCCGCTATATTCCGCCATCTCTAAGATTTACAGCGAT
ACACGGGTAATTTAAGGAATGCCCGAACCGTCATTCCCGCCACTTTCCGTCATTCCCGCA
AAAGCGGGAATCTAGGACGCAGGGTTAAGAAAACCTACATCCCGTCATTCCCGCCACTTT
CCGTCATTCCCGCGAAAGCGGGAATCTAGAATCTCGGACTTTCAGATAATCTTTGAATAT
TGCTGTTGTTCTAAGGTCTAGATTCCCGCCTGCGCGGGAATGACGATTCATAAGTTTCCC
GAAATTCCAACATAATCGAAACCTGACAGTAACCGTAGCAACTGAACCGTCATTCCCACG
AAAGTGGGAATCTAGAAATAAAAAGCAACAGGCATTTATCGGAAATAACTGAAATTCAAT
ACCGCAAAAATCTACCCGAAATGATATAGCGGATTAACAAAAATCAGGACAAGGCGGCAA
AACGTTTGGCGACTTCGTCCCAGTTGACGATTTCCCAAAAACCTTTCAGGTAGTTGGGAC
GGCTGTTGCGGTAGTCGATGTAATAGGCGTGTTCCCACACGTCGCAGGTCAGCAGCGGCG
TGTTTTCAGTGGTCAGCGGCGTAGCGGCGTTGGAAGTAGAAACCAAATCCAATCCGCCGG
CAGGGGTTTTTACCAGCCACGCCCAACCGGAGCCGAAAGTACCGGCCGCGCAGGCATTGA
ACGCTTCTTGGAATTTCTCGAAGCTGCCCCATTTCGCGTCGATGGCGGCGGCCAGTTCGC
CGGCAGGTTTGCCTTGACCTTTGGACGTGAAACCCAGCCAGTAGAAGGTGTGGTTCCAAG
TTTGTGCCGCGTTGTTGAACACGCCGCCTGAAGATTTTTCACAATCTCTTCCAAAGGCA
GGTTTTCAAATTCGGTGCCTTTGATTTGATTGTTCAGGTTGGTGATGTAGGTTTGATGGT
GTTTGCCGTAGTGGAACTCCAAAGTCTCTTTGCTCAGATGCGGGGACAATGCGTCCAGTT
CATAAGGCAGTTGCGGCAGCTTATGTTCCATTTTGTACTCCTGAATATTGTTTTAAATGT
TGTATTTTGGCAGTGTTGCTGCAAATAACTCGGCAGCCCGTGTATTCTACCTGTTTTGCG
GTGCGGAAACCAATTAAACCTGCTTTACGCTATAATAGAAGATTGCAATTTCGGCACGAC
AGATAGGATGTACCATGAACGATTACGCAGCCATGCCGTCTGAAGACCGTGAGGTCGGCG
CATTGTCTCTGCCCCCACACTCAATGGAGGCGGAACAATCCGTTTTGGGTGGGTTGATGC
TGGAAAATCCGGCATGGGACAGGATTGCCGATGTGGTTTCCGGTGAAGACTTCTACCGGC
ATGAACACCGCCTGATTTTCCGATCCATTGCCAAATTGATTAATGAGAGCCGTCCCGCCG
ATGTGATTACGGTTCAGGAAGATTTGCAGCGGAACGAAGAATTGGAAGCGGCAGGCGGAT
TCGAATATCTGATTACGCTGGCGCAAAACACCCCGTCTGCCGCCAACATCCGCCGCTACG
CCGAAATCGTGCGCGAGCGTTCCATTATGCGCCAACTCGCCGAAGTGGGGACGGAAATCG
CCCGCAGCGCATACAATCCGCAAGGCAGGGACGCGGGGCAGCTTTTGGACGAGGCGGAAA
ACAAAGTATTCCAAATCGCCGAAAGCACCGCCAAATCCAAGCAGGGCTTTTTGGAGATGC
CCGATTTGCTGAAAGAAGTCGTACAGCGCATCGATATGCTCTACTCGCGCGACAATCCCG
ATGAAGTTACCGGCGTGCCGACGGGGTTCATCGACCTCGACAAAAAAACCTCGGGTCTGC
AACCCGGCGACCTGATTATCGTTGCCGGTCGTCCGTCTATGGGTAAGACCGCCTTTTCTA
TCAATATCGCCGAACACGTTGCCGTAGAAGGCAGGCTGCCCGTTGCTGTTTTCTCGATGG
AAATGGGCGGGGCGCAACTGGTCATGCGTATGCTCGGCTCGGTCGGACGGTTGGATCAAA
GCGTTTTGAAAACCGGCAGGCTCGAAGACGAACACTGGGGTCGCCTGAACGAAGCAGTCG
TCAAACTCTCCGACGCGCCCGTGTACATCGACGAGACCCCGGGTCTGACCGCGCTCGAAC
TGCGCGCCCGTGCCCGCCGTCTCGCCCGTCAATTTAACAATAAGCTTGGATTGATTGTCA
TCGACTACCTGCAACTGATGGCAGGATCCGGCCGTTCCGACAACCGAGCTTCGGAGCTGG
GAGAGATTTCACGTTCGCTCAAAGCGTTGGCGAAAGAATTGCAAGTCCCCATCATCGCCC
TGTCGCAATTGAGCCGCACGGTCGAATCGCGTACCGACAAACGCCCCATGATGTCCGACC
~~TTCGCGAGTCCGGCGCAATCGAGCAGGATGCCGACCTGATTATGTTCATGTACCGCGACG~~
AATACTACAACCAGGACTCACCCATGAAAGGCCTTGCCGAATGTATCATCGGCAAACACC
GCAACGGTCCCGTCGGTAAAATCTTCCTCACATGGACGGACAATTCACCAAATTCGACA
ATGCTGCCTATATTCCCGAGGAGGCAAAGATAGAGGATTAAATGGCTATATAAAAATTTA
TTAGGCGAAATCAGGCAAAATCGTTTAAAATCATGCTGAGAGATTGCCCTAAAAAATAAA
ACGCGGTCTTGAGGCATTTTTGCATTCAGCCCGCATATAATTGAAAATATAGTGGATTAA
CAAAAATCAGGACAAGGCAACGAAGCCGCAGACCGTACAAATAGTACGGAACCGATTCAC
TTGGTGCTTGAGCACCTTAGAGAATCGTTCTCTTTGAGCTAAGGCGAGGCAACGCCGTAC
TGGTTTGAATTTAATCCACGATACATTACCAGTTAACGTTCTATTGCTTATGTGTACACG
AAAACAACAAGGTTTCACGCTAACAGAGCTGCTCATCGTGATGGTCATTGCAGCCATTAT
GGCGATGATAGCCCTCCCCAATATGAGCCAATGGATTGCATCCCGCCGCATTGCCAGTCA
CGCGGAGCGGATTGCCAACCTTTTGCGTTTCTCCAGGGGCGAAGCCGTCCGGCTCAATCT
CCCTGTCTATATCTGTCCTGTTCAAGTTAAAAAAGACGGTACGCCCAACAATAAATGTGA
CTCCGGCAAGAAGGGGCAGGGAATGTTGGCTTTCGGCGACAAAAACGGCAATAAGGGATA
TGACAATGATACGGAGGATGTTCTTCTCCGCAGTGTGGTATTGAATGATGATATCAATGA
TAAGCGGATTAATTATGCCTTCAACCATATCGCTTTCGGTCAGACTCAGCCGACCACCGA
CCGTGTAGTTTGGACATTCAATCAAAACGGGACGTTCGGTTATACGAAAGACCAGCATCT
TACAAAACAATCCAGCTTTTTTTATTCTGACGGTTATATCCAAATCGTGTTGACAGATGC
GAAGGCGGTTTCTGCCGATGAAAAGAAATTCCGTTCGGCGGTGGTTTTGATTAACAGCAG
CGGCAGGGTCGAAGTTTGCCCTAGGGGTGATCGGCGTACTATGTGCCAGTATAAATAACA
GTTTCAGTTTTAAAAATGAATATGAAGAATAATGATTGCTTCCGCCTGAAAGATTCCCAG
TCCGGTATGGCGCTGATAGAAGTCTTGGTTGCTATGCTCGTTCTGACCATCGGTATTTTG
GCACTATTGTCTGTACAGTTGCGGACAGTCGCTTCCGTCAGGGAGGCGGAGACACAAACC
ATCGTCAGCCAAATCACGCAAAACCTGATGGAGGGAATGTTGATGAATCCGACCATTGAT
TCGGACAGCAACAAGAAAAACTATAATCTTTACATGGGAAACCATACACTATCAGCTGTG
GATGGCGATTTTGCGATTGATGCCATGAAAACTAAGGGGCAATTGGCAGAGGCACAATTG

## Appendix A

AAGAGATTTAGTTATGAGCTGAAAAATGCCTTGCCGGATGCGGCAGCCATCCATTACGCC
GTCTGCAAGGATTCGTCGGGTAACGCGCCGACATTGTCCGGCAATGCTTTTTCTTCAAAT
TGCGACAATAAGGCAAACGGGGATACTTTAATTAAAGTATTGTGGGTAAATGATTCGGCA
GGGGATTCGGATATTTCCCGTACGAATCTTGAGGTGAGCGGCGACAATATCGTATATACT
TATCAGGCAAGGGTCGGAGGTCGGGAATGAGACGTAAAATGCTAAACGTACCAAAAGGCA
GTTATGATGGTATGAAAGGTTTTACCATTATTGAATTTTTGGTTGCGGGCCTGCTCAGTA
TGATTGTCCTGATGGCGGTCGGATCGAGTTACTTCACATCCCGGAAATTAAATGATGCGG
CAAACGAGCGTCTTGCCGCGCAACAGGATTTGCGGAATGCGGCAACATTGATTGTCCGCG
ATGCGAGAATGGCAGGCGGCTTCGGTTGTTTCAATATGTCCGAGCATCCTGCAACTGATG
TTATTCCCGATACGACGCAACAAAATTCTCCTTTTTCCTTAAAAAGGAACGGTATAGATA
AACTTATTCCCATAGCGGAATCTTCAAATATCAATTATCAGAATTTTTTCCAGGTTGGTA
GCGCATTGATTTTTCAATACGGAATCGATGATGTTAATGCAAGCACCGCGACTACCGTCG
TCAGCAGCTGTGCCGCAATATCGAAACCGGGCAAGCAAATCCCTACTTTAGAAGATGCAA
AAAAAGAATTGAAGATTCCGGATCAGGATAAGGAGCAAAATGGCAATATAGCGCGTCAAA
GGCATGTGGTCAATGCCTATGCGGTCGGCAGGATTGCCGATGAGGAAGGTTTGTTCCGCT
TCCAATTGGATGATAAGGGCAAGTGGGGTAATCCTCAGTTGCTCGTGAAAAAGGTTAGAC
ATATGAAAGTGCGGTATATCTATGTTTCCGGCTGTCCTGAAGATGACGATGCCGGCAAAG
AGGAAACATTCAAATATACGGATAAATTCGACAGCGCCCAAAATGCTGTTACGCCCGCCG
GGGTGGAGGTTTTATTGAGTAGCGGTACTGATACCAAGATTGCCGCTTCTTCAGACAATC
ATATTTATGCTTACCGTATCGATGCGACAATACGCGGGGGAAATGTATGCGCAAACAGAA
CACTTTGACGGGAATCCCGACTTCTGACGGACAGAGGGGGTTTGCACTGTTTATCGTGCT
GATGGTGATGATCGTCGTGGCTTTTTTGGTTGTAACTGCCGCGCAGTCTTACAATACCGA
GCAGCGGATCAGTGCCAACGAATCAGACAGGAAATTGGCTTTGTCTTTGGCCGAGGCGGC
TTTGCGGGAAGGCGAACTTCAGGTTTTGGATTTGGAATATGATACGGACAGTAAGGTTAC
ATTTAGCGAAAACTGTGGAAAAGGTCTGTGTGCCGCAGTGAATGTGCGGACAAATAATGA
TAATGAAGAGGCTTTTGACAATATCGTGGTGCAAGGCAAGCCCACCGTTGAGGCGGTGAA
GCGTTCTTGCCCTGCAAATTCTACCGACCTGTGCATTGACAAGAAAGGGATGGAATATAA
GAAAGGCACGAGAAGCGTCAGCAAAATGCCACGTTATATTATCGAATATTTGGGCGTGAA
GAACGGAGAAAATGTTTATCGGGTTACTGCCAAGGCTTGGGGTAAGAATGCCAATACCGT
GGTCGTCCTTCAATCTTATGTAAGCAATAATGATGAGTAATAAAATGGAACAAAAAGGGT
TTACATTGATTGAGATGATGATAGTCGTCGCGATACTCGGCATTATCAGCGTCATTGCCA
TACCTTCTTATCAAAGTTATATTGAAAAAGGCTATCAGTCCCAGCTTTATACGGAGATGG
TCGGTATCAACAATATTTCCAAACAGTTTATTTTGAAAAATCCCCTGGACGATAATCAGA
CCATCGAGAACAAACTGGAAATATTTGTCTCAGGCTATAAGATGAATCCGAAAATTGCCA
AAAAATATAGTGTTTCGGTAAAGTTTGTCGATAAGGAAAAATCAAGGGCATACAGGTTGG
TCGGCGTTCCGAAGGCGGGGACGGGTTATACTTTGTCGGTATGGATGAACAGCGTGGGCG
ACGGATACAAATGCCGTGATGCCGCTTCTGCCCAAGCCCATTTGGAGACCTTGTCCTCAG
ATGTCGGCTGTGAAGCCTTCTCTAATCGTAAAAAATAAGGTTGTTTTGCCAATACCGTCT
GAAAATCAATGTTCAGACGGTATTTTTATGGGTATAGTGGATTAACAAAAATCGGGACAA
GGCGACGAAGCCGCAGACAGTACAGATAGTACGGAACCGATTCACTTGGTGCTTCAGCAC
CTTAGAGAATCGTTCTCTTTGAGCTAAGGCGAGGCAACGCCGTACTGGTTTTTGTTAATC
CACTATACATCCCGTCATTCCCACGAAAGTGGGAATCTAGAAATTTAATGTTGCGGCACT
AGCCAAAAAAACCGAAACCGACAGGTCTAGATTCCCGCCTGCGCGGGAATGACGAATCCA
TCCGTACGGAAACCTGCACCACGTCATTCCCACGAACCTGCATCCCGTCATTCCCACGAA
AGTGGGAATCTAGTTTTTTGAGTTTCAGTCATTTCCGATAAATTGCCTTAGCATTGAATG
TCTAGATTCCCGCCTGCGCGGGAATGACGAACCTATCCGTACGGAAACCTGCATCCCGTC
ATTCCCACGAAAGTGGGAATCTAGTTTTTTGAGTTTCAGTCATTTCCGATAAATTGCCTT
AGCATTGAATGTCTAGATTCCCGCCTGCGCGGGAATGACGGATTTTAGGTTGGGGTCATT
TATTGGGAAAAGCAGAAACCGCTCCGCCGTCATTCCCACGAAAGTGGGAATCTAGAAATT
TAATGTTGCGGCACTAGCCAAAAAAACCGAAACCGAACGGACTAGATTCCCGCCTGCGCG
GGAATGACGAATCCATCCATACGGAAACCTGCATCACGTCATTCCCACGAACCTGCATCC
CGTCATTCCCACGAAAGTGGGAATCTAGTTTTTTGAGTTTCAGTCATTTCCGATAAATTG
CCTTAGCATTGAATGTCTAGATTCCCGCCTGCGCGGGAATGACGGATTTTAGGTTGGGGT
CATTTATTGGGAAAAGCAGAAACCGCTCCGCCGTCATTCCCAGGAAAGTGGGAATCTAGA
AATTTAATGTTGCGGCACTAGCCAAAAAAACCGAAACCGAACGGACTAGATTCCCGCCTG
CGCGGGAATGACGGATTTTAGGTTGGGGTCATTTATTGGAAAAAGCAGAAACCGCTCCGC
CGTCATTCCCACGAAAGTGGGAATCCAGTTTTTTGAGTTTCAGTCATTCCCGATAAATTG
CCTTAGCATTGAATGTCTAGATTCCCGCCTGCGCGGGAATGACGAACCTATCCGTACGGA
AACCTGCACCGCGTCATTCCCACGAAAGTGGGAATCCAGTTTTTTGAGTTTCAGTCATTT
TCAATAAATTGCCTTAGTATTGAATGTCTAGATTCCCGCCTGCGCGGGAATGACGAATCC
ATCCATACGGAAACCTGCACCACGTCATTCCCACGAAAGTGGGAATCCAGTTTTTTGAGT
TTCAGTCATTTTCAATAAATTGCCTTAGCATTGAATGTCTAGATTCCCGCCTGCGCGGGA
ATGACGGATTTTAGTTTGGGGGGCATTTATTGGAAAAAGCAGAAACCGCTCCGCCGTCAT
TCCCACGAAAGTGGGAATCTAGTTTTTTGAGTTTCAGTCATTCCCGATAAATTGCCTTAG
CATTGAATGTCTAGATTCCCGCCTGCGCGGGAATGACGATTCATATAGTGGATTAACAAA
AATCAGGACAAGGCGGCGAAGCTGCAGACAGTACAGATAGTACGGAATCGATTCACTTGG
TGCTTCAGCACCTTAGAGAATCGTTTTCTTTGAGCTAAGGCGAGGCAACGCTGTACTGGT
TTTTGTTAATCCACTATAAAAAGGCATATTGAATGCGGGCAAACCGGCTGCTTTCCGTTT
TTGGATTTCGGAGAATGCCATCGCCCAGCTTTCATCACACATAAAAAACAGTGCGGGCAC
GGCTTTTTTCAGCGGTATTCCTTTCAGGTGCGGGGCAAGCGCCGCCCCCATCAGGATATG
CCGAGAATTAATCATAAAGGTTACGGTGGCGATAAGCAGTATCGGCAGAGGTTCCGCCCA
CAGGTTGACCGTGGCAAACTCGGAGCCGCCGGCGAAGTTCATACTGGTCATCAACAACAT
TTCCAGCCAGCTCATGCCTTTTTGTCCGCCCTGCATACCGAGTATTAATGCCCAAGGCAG
CAGCCCAATCAGCATAGGCGAACTTTCTTTGATGCCGCGTATAAATTCGTTATGCGGGGA
AGGTGTGCATAATGTTCGTCTTCATAACCGGAAGGGCGGGAATTATACACTGGCAACGGA

## Appendix A

TTTCAAAACAAAACCGATTTGCCGTGTTTCAGCGTAAACACGGCTTGTGTATAATCTCCC
ATCTTTGAAACCGGCCGTATGCAGGAGCAAGACGATGAATATTGAAGTAGAAATGAAAGT
ATTGGACGAACGGATGGCGGATGTTGTCCCTGTCTATGCAACGGAGGGTTCTGCAGGTTT
AGATTTGCGCGCCTGTTTGGATGAGGAAGTCGTTTTGCAGCCGGGTGAAACGTTTCTTGT
GCCGACGGGTTTGGCAATTTATTTGGCGAATCCCGCATATGCCGCCGTTTTGCTGCCCCG
TTCCGGCTTGGGGCATAAACACGGCATTGTTTTGGGCAATTTGGTCGGTTTGATTGACTC
CGATTATCAAGGGGAATTGAAGGTGTCGTTATGGAACAGAAGCAGCGAACCGTTTACTGT
CAAACCGTTTGAGCGTATCGCGCAGATGGTTGTCGTGCCAATCGTGCAGGCGGGCTTCAA
ACGTGTCGAGGAGTTTGTCGGAAGCAGCCGGGGTGAGGGCGGCTTCGGCAGTACGGGTTC
TCACTAAAAATATAGAATGCCGTCTGAAAGACACGTCAGGTTCAGACGGCATATCTTCCG
ATATACCGACAACCGGAGAAAATCATGAATACCTTACTCAACCAACTCAAACCATATCCC
TTTGCCCGACTGCGTGAAGCGATGCAGGGCATTTCCGCGCCCGAAGGTCTGGAAGCCGTC
CCCCTGCACATTGGCGAACCGAAACATCCGACACCGAAAGTCATTACGGATGCGCTGACC
GCCTCATTGCACGAGTTGGAAAAATATCCGCTGACGGCCGGTCTGCCTGAACTGCGTCAG
GCGTGTGCAAACTGGTTAAAACGCCGTTACGATGGCTTGACAGTGGATGCGGATAATGAA
ATTCTGCCGGTTTTAGGCAGTAGGGAGGCGTTGTTTTCTTTTGTTCAAACCGTGTTGAAC
CCTGTTTCAGACGGCATCAAACCCGCAATTGTCAGCCCGAATCCCTTTTATCAGATTTAC
GAAGGTGCGACACTTTTGGGCGGCGGTGAAATCCATTTTGCCAATTGCCCCGCGCCGTCT
TTCAACCCCGATTGGCGCAGTATTTCCGAAGAGGTTTGGAAACGCACCAAACTGGTGTTC
GTCTGCTCGCCCAACAACCCCAGCGGCAGCGTGCTGGATTTGGACGGCTGGAAAGAAGTT
TTTGATTTACAGGATAAATATGGTTTCATTATTGCCTCGGATGAATGCTATTCCGAAATC
TATTTCGACGGCAACAAACCTTTGGGCTGCCTGCAAGCCGCTGCACAGTTGGGTCGAAGC
AGGCAAAAACTGCTTATGTTCACCAGTTTGTCCAAGCGTTCCAACGTTCCGGGCCTGCGT
TCCGGTTTTGTCGCCGGCGATGCCGAACTGCTTAAAAACTTTCTGCTTTACAGAACCTAT
CACGGCAGTGCAATGAGTATTCCCGTGCAGCGCGCAAGCATTGCCGCTTGGGATGATGAA
CAGCACGTTATCGACAACCGCCGTATGTATCAGGAAAAATTTGAGCGCGTTATTCCCATT
TTGCAACAGGTATTTGACGTTAAATTACCGGATGCCTCGTTTTACATCTGGTTGAAAGTC
CCTGATGGCGACGATTTGGCACTTGCACGCAATTTATGGCAAAAAGCGGCTATCCAAGTA
TTGCCCGGACGTTTTTTGGCGCGGGATACCGAACAGGGCAATCCCGGGGAAGGTTATGTG
CGTATCGCTTTGGTTGCCGATGTCGCAACTTGTGTCAAAGCTGCGGAAACCATTGTTTCC
CTATATCGGTAAAGAATAAAAAAATGCCGTCTGAACTTTTGTTCAGACGGCATTTTTCAA
TATTTTACGGTTGAATTTGCTATAACGGTATTTATAGTGGATTAACAAAAATCAGGACAA
GGCGACGAAGCCGAAGACAGTACAGATAGTACGGCAAGGCGAGGCAACGCTGTACTGGTT
TAAATTTAATCCACTATACTTCACTTTTAATCGGCTTGCCCGCAAACACGTTTAAACTTA
AAATCCCCGTGTTTGACACAATACCGAGCAGATTATGTTTTTTGTCCTTTCCCCTGCGAA
GAACCTTAATGAAAAAGACCCTGCCCCTGTCAGCGAGTTTACCCAACCCGACCTGCTGGC
AGAGTCCGACATTCTAATGCAGCAGTTGCGCGAGCTTGCGCCGCAACAGATTGCCGAACT
GATGCACGTTTCCGACAAAATTGCCCTCTTAAACGCGCAGCGCAATGCAGAATGGAACAC
GCCGTTTACGCCGGAAAACGCCAAACAGGCGGTCTTTATGTTCAACGGCGATGTTTACGA
AGGTATGGATGCAAACACATTGGATATTGGACAGATACGCTATCTGCAAAACCATGTCCG
CCTGCTGTCCGGTCTGTACGGTCTTCTTCGCCCGTTAGACCTGATACAGCCCTATCGTTT
GGAAATGGGGACGGCATTTGCCAATTTGCGCGGCAAGAATTTGTATGAGTTTTGGGGCGA
CATCATTACCAACCTTTTAAATGATACGCTTGCCCAAGCAGGCAGCAATACGCTTGTCAA
CCTTGCCTCACAGGAATATTTCAAGTCCGTCAACACGAAAAAACTTCGGGCGCGGCTGAT
TACCCCAATATTTAAAGACGAAAAAAACGGTAAATATAAAATCATCAGTTTCTATGCCAA
GCGCGCGCGTGGATTAATGGTGCGCTATGCGGCAGAACATCATATTACCGACCCTGAAAT
GCTGAAAAATTTTAATTACGAAGGCTACGCATTCAATGACGCGGCTTCAAATGAAAGCGA
ATGGGTTTTTATGCGTTCGGAACAAATAAAGTGAAAACAATAAATTAAGTATTTTCCGAA
AAAAGTGCTTGGCAAAATGTATAAATTTCATTATTATTCCTAATCTTCAAGAAGACGGAA
~~GCGTGGCAGAGTGGTTTAATGCAACGGTCTTGAAAACCGTCGAGGGTTGATAGCCCTCCG~~
TGAGTTCGAATCTCACCGCTTCCGCCAATTTTTGAGCGTAAAACCAAATAAGAATGCAAT
AGCCGCAAATATTGTATTTTTATTTGGTTTTACTGCATTATCGGAAACGTGGCAGAGAGG
CTGAATGCAGCGGACTCGAAATCCGCTGAGGGTGCAAATCCTCCGTGGGTTCGAATCCCA
CCGTTTCCGCCACAAAACAAAACCGCCCTGATTCGGGGCGGTTCTTTTTTGTTCAAGTTG
TATCAATTACCATATAAAAATCATCGGTTTGCCCTATCATAACGCATCAAAGCAAATCAT
TTGCAATCTTGCGGCATCTTCTTATTGCATTTTTTTATGGTAATGTGTATGGTAATTTTT
GGATAAATGGGAAATTACCATAATGGCGAAAATCATTACGCCGCTGTCGGCAAATCAGGT
TAAAAATGCGAAGCCGCGCGATAAGCTGTATAAGTTGTCAGACGGGGCGGGCTGGCTTTG
TGGGTCTACCCGACGGGCGGGCGGAGTTGGAAGCTGTCGTTTGTGCAGGATGGAAGGCAG
CAGACAATTTCGCTGGGGCGGTATCCTGATTTTTCGCTGGCCGATGCGCGGGAATGGCGG
GAGGAGGTGCGCCGAAAACGGGCGCACGGGGAAAATGTCGTCAATAAGAAGGTGCGGGCG
GATTTTGCTTTTGAGAAGGTGGCGCGTGATTGGTTTGTGCGTTGGTCGAAGGGGCGGTCT
GAAAAGTATGCCGGACAGGTTATGCGGAATTTTGAGCGGTGGGTTTTTCCGGCTATCGGC
AATCTTGATATTCGTCAAATCAGGACGGCGGATGTGGTCGGCTGTCTGCGTGTGATGGAG
GCGCGCGGTATCGTTGATACGTTGCGCAAAACGAAAAACAGTCTGAAGATGGTGTTTGCG
TTTGCGGTCGGTTCGGGAATGATGGAAATCAACCCTGTCGCGCAAATCGGTTCGGGTGTG
TTTGAACGGGCGAAAACGGGGAATATGGCAGCGTTGAGTCCGTCTGAATTGCCGCGCCTG
ATTGATTTTTTGGAGCAGCGCAATGAATTTGCGGTTTATGCGGGCAGGGTGCGTATCCAT
CCTGTAACGCGGCTTTGTATCTATTGGCTGCTGTTGACAATGACGCGGATTCAGGAGGCG
GCGTTGATGGAGTGGTCGGAGTTGGACGGGGAGGTTTGGCGTATCCCCGCCGAACGGAAA
AAGGAGCGGCGGGGGCATGATGTGCCGCTGTCGCGGGCGATGCAGTGGGTGTTGGATCAG
GCGCGGGCGTTGAATGTGAACGGGCGGTTTGTGTTTGAAAGTGTGAATTTTCAAGGGCAT
ATCAATAAGGAAAGTCCGCGCGTGGCGATGCAGCGGGCGGGGCTGGATACGACGGCGCAC
GGTTTGCGCTCGCTTGCGCGTACTTATTTGCGCGAGGTTCTGAAGGTGGATAGTATTATG
CGGAAACGCAAATAAAAAACCGTTTCCGCATTTTTATTGGAAGGCTTTTTTGCAACCGCT

## Appendix A

TTACACAAAGGCGGTTTTTTGTGTAAGAACTGCTATAATAGCAGCCCGTCATCGTCAGGA
GCGGCTAATGCCTTTAAAATTCCAACCAAGGGAACGTTCGGTTATCATGTGCGACTTTCG
CGGTTATGAAGAACCGGAAATGGTCAAGAAACGCCCTGTCGTCGTCATAGCGCGAAACAG
GCACAACGGCAAACTGGTAACGGTCGTACCCTTAAGCAGCACAGAACCTGTCCCTTTGGC
GGACTACCACCACAAAATGAGTGGAAACCCCTTACCGGACAAGCCGCACATCCAATGTTG
GGCAAAATGCGACATGACGGCAACAGTCGGATTGGCACGATTAGACCGATACAAACCCAA
AGGGCGCGACCGCTGCATTCCAATAATCAGTGAAGAGGATTTTCAGGCGATTAAAACAGC
CGTTGCCAAGGCATTCAAACTGTACTAGAATAAAACCGTTCCCTTAAAGGGGCTTGCAAG
ACTGTTCTGAAATATGGGCAGCCGCGCACGGGCGACAGGCGATGACAAGCCGTCCGTGCG
TGTGATGGGGCGCGGAATGCGCCCCTTGTCGTATCTGCAAACGCCTACAAATCCCCAATC
AGCCTTTCAATCAAGGCTGTTTTGGACAAACCCGCCTTTGCCGCCTCCTGTTCCAGTTTG
GCTATCGTTGTGTCCGCCTTAACGGGCGATTTAAGAACCGCTTTACACGAAGGCGGTTTT
TTTGTATAGTCCGGTTCACGAGGTACAGAATCTTGAAAATAGTCAAGCAATGCCGTATAT
TCCGACGCAAGGATTTATTTTCAACATCAGCTTAAGGGGATGACAATGGGACATATTTAT
TCGGAAAGCCGTGATTTCCGCCTTTCAGACGGCTGGGACGGCAATGACTGACAACCTAAT
ACAGATAGCAACGCCGATATTGACTGTTATCGGCGTTTTTGTTGCCGCTTACGGCATCAT
GAGGAATACAGAAAACGCCAAAAAGCGCGCTGATCATGGCCGAACGTAACAATGCCGCCC
TTCAAGAAGCCATAACCATAGTAAACGGGCTGGCAAAAACAGACGGATGCATACTCGCCA
CCTATACATCGGATACCCCGGACAAGAAGAAAGACCGTGAAGCCATACTGACAGTTTTAA
ACCAGCGCGAATTTGTCTGTGCGGGCGTATTAGGCGGAGCACTGCACGAGAAAATGTATA
AAGATTTCGAATACTCCATGCTGTTACGTGACTGGGACAACCTAAGCAGCTTTATTTTTG
AAATACGCCGTATCAGGAGCGCACCGACGGCCTTTCAAGAATTTGAAGCCGTAGCCCGAA
AATGGAAGAAAAAGCCTCTGAAAACCAAATAGCTTAATAGCTTAACATCCGCCGCAACAT
AGGCCGTCTGAAATTCAGACGGCCTTTCAGTTTGCCGCCTACGGTTTTTTGGGAAACCCC
TTGCATGTGCAGGGGGTTTTGTTTTATATTCCTGTTCGTGGCGTCAGAAACCACACTACA
GCGGCAATCACTCCGTCAATGTGATTTTTTCATGTCTATAGTTTTCTTTCCTTGTTGTTT
GTTTCGATAGCAGGAAGTTTCTATGACCGCGTGGGCGACGAATACAAGACCCGAAAGGGG
AATAAGTCCGCCCTCCTATGTGGGTTCTTAACCGCGTGTCCGCCCATTTGGGCTAATTCT
CTTGACACATTTCCATAACTCTATATAATATTTCCCACGGTGCTTGAAAACACCTGACAA
ACAGCGTATATCCAACACGATAGAGTGGAATTTTTTACGTCTATACGTATCAAATCGATT
TACTCCTATGTGGGGGTGCGCCTACCCGTAAGGCTGGCGGCGCGCCTGTTTGCGTGTTTT
CAACACCCCTGCGCCCAATTTGGGCATTCCTAAATCCTACATGCTGTTGAAGACCGCGAC
CCTATCCGCCACATGGCGGCTTTTTTATGCTTGCAGAAAATAGAAAGATTGGATATATTA
CGAAACACGAGGCGTCGAAAACCTCTACTAGAACGGCATTTACCCCGTCAGCGTGAATTT
TTTACGTCCATAAGTTTTCTTGTTTGGTTGTTTGTTTCGATATATCCGAACTAGTTTCCT
ATGGTCGGGAGGGTGCGGAATACAATACCCGCAAGGGGAATAACGCCGGCCTTTTCTAGT
AGGTTTTCGAACCTCCCGACCACCCATTTGGGTCTTTCGAAACTAAACTAGGAAACTATC
ATGAACGTATCTGTTCTCAATTTTGGTAACACCCCTGTATCTTTCCGTCAAGACGGTTTT
TTAAATGCAACCGCCATTGCATCTCACTTTGGCAAGTTACCTAAAGACTACCTAAAAAGT
GAACAAACTCAACAATATATCTCTGCACTTGCTGAGAATTTAAGCGTTAGGAGAAAAATC
CTAACGGAAGCAAATCAAATAGTTATCGTGAAGCGTGGTGGCAGTGAGCAAGGCACATGG
CTGCATCCCAAACTCGCTATTCACTTTGCCCGTTGGCTTAATCCGAAATTTGCGGTTTGG
TGCGATGAGCAGATTGAAATTTTACTTAACGGCAAAATTTCAGACGGCATAAAAACAGTT
ACCCCCAAACCCACCCGCGCCCTTCCGGACGGCTTGACCGGCGAACAAATCGAAGCCGTC
AAAAAACTGCACAACGCCCTGACCAAATCCGCACCCAAAGAAGCGCAGGCGCGTATCGCC
ATTACCCTTTGGTCTGCCGTCAAAAGCAAGTTCGGATGCAGCTACAAAGAAGTACCTGCC
GAACAGTTCCCCGAAGTTTTAAGCGTGATGGGCCGCGTGGCAGTTGAAAACGGCGTGCTG
TACGGCGAAGTCCTCGACCGCGAACCATTGCCCGCACCGCAACCTGCCCTGCCCATCAGC
GGCAACGCCCTGTACGACCTCGCCGTTGCCGTCAGATACGGCGCGTGGGCCATCCAAATG
GGCAGAGACGTTTCCCTGCCGCTGAAGCAGCTCGGCTGCAAACAGGCGGTAACGATGTGG
ACGGTCTGGGCGGAAACACGCAGCCGCCTCAAAGCCGCCGCAAACGCCCTCGAAGCCTTA
AACGCACACGCCGACGCGGAACACGCGGCAAAAATCCGCCCGATGCTGCCCGAAATCCGC
AACCTGTCGTCGGTTTGATGCAGTAGGGAATACAAAAGCCGTCTGAATGTGAAAACGCCC
TAATCGGGCGTTTTTTTATTGCTGTAACCCCAGGGCTTCCAAAACTTCGCGGGTGTCCCA
TACGGGGGATGTGAGTGGGGTTTTCAGACGGCACGGTATCCGTCCGTCTTTTTCCATACG
CAGCAGTGTCGAGTTTGAAATGGGGCGGTTGCGGCAGGTTGCGTAGGCAATCAGTTCGCG
GATGGTCGGGCGGTCTATCCTTGCGCCCAGTTCGTTGATGTTCATTTTTTTACTCTCCTG
TAATGACTCGGTTTCTGGAAGCGGCGTAATACGGCATCGGCGGCCGTGATGAAAAGCCAT
ACCGCCAATGCCTCAGCGCCGGAAAATGACAGCGCGATGATGATTTTTAATAGGGTGTCC
ATCAGGCTTTCCTTTTTTCTCTAGTTTCGTACTCATAAATGAACATCGGGAACGCCTGCC
CGCTTCTGACAAGATTTAAATCCGGTATCTGATCGGTAATCAGACAAGAAAACCGCCCGT
CCCCGCCGTTTCCCGTCGAACAGCAAATCACAAGGTTGCCGCCAAATGGAATCGTATCTT
GATTCGTGTTCATCTTTACTGCTCCGGCAAATACGTTTTAAAAATCAAATCTTCAAGTCG
GCGGTAAACCTTCCCGGTCTGCACCAATGACTCGGTTTCGGGAAGCGCGGCCAAAAGCTT
CCGCATATGACCGGCAATCGCCTGTAACACAAGCTGCTCGCCGCGCGTTTTGTTGTGTTT
GGCAACCATCGCCGCCAGCCCGTAAACCGCCAAGTCCATCATTGCCTCCACGCTGTCGGC
TTCAGCGGAATGCAAGAGATTAATCTCTATGTCATTCTTGATGTTCTCGTTTTCTTCCTG
AAAGTTCATTTTTCCAGCTCCGGCACTTCCGCGTCGCCGTGTATCCATCGGTAGTCTTTC
AATTCTTCGGCTTCGCGTTGTCTGATTTCGGCGTCGATTTGGGCGTTCAACCCGTCAATC
TCTGCCTGTTTGTCGGCGACGGCTAAGCGCATAGCGGTCAGGCTGTTTTCAGCCTTGACT
TGCACTGCGGCTTCGGATTGCGGTTGGCAGGCGTAGATGCCGGCGGCTGCGACTGCGAGT
AAGGCGGTGCGGATCAAGTATTTCATTTTGATTCCTCATTATTGGGGTAACGGCTTAATA
TCAGGCAGCGTTTTTGAGGTTGTCTTTTGTCAGACAGATGAGCGCCTTTCTGACGGCGGC
AAAGGGGGTTTTGTACTCTGGCTCTATGCCGACAAGTAGCTCCCCGTCTGTGTTGATGAT
GTCGCATCCGTAGCGTTCGGTGGGGTAGCCGTAGTCGTTTTTCCCCTGTTCTGAGGTTAC

## Appendix A

TCGGATGTCGATGGAGTATTCTTCTGTGATGGTCATTTTGGGGTCTTTCGGATTTGGGTT
TTTGTGGAGGTCGTCTGAAGGTTCACGATTTCAGACGACTTTTTTTAGGCGATTACGCC
GAAATAGATGTCGATTTCAGGGAAGGCTTTTTGTACGGCTTCGGAAATGTCTTTTGCCGC
TTGTTCGATTACAGCATCGATTTGCTGCAATTCGTACCACAGGACGAGTGCGCCGCTGTT
TTTGTCGATGCGGAATTTCAACAGGGCTTCAACAAAGTAGGATGCACCGCCTTGATGCGG
GGTGAACTCGATGCCGAAACGTTCAAACATTTTGAGGTTTTTCTCTGTTTGCCCTGAGTC
TTCGGATTGGAAGGTAAAGTTGATTCTGCCGTCCTGTTCACGATAGCCTTGTTTGAAGGT
GGTTTTTTCGGTGTACTCGAGATTGAGCGCGAAATCCAATACTTCGGCGGCGGTCGGGTA
AACGGAATTTTCGTTACCGGGATTTTTGGATACGATGTTGCGGGCATTGTTGGTCAGGAA
ATGGGAAAACTCCATCTGATTCATGCGGTGCGCATTGTTGTTCAGCCAGTTGGATGCCGA
TGTAGTGTGTTGCGGGGTGTATACGGCATAAAAATCGAGCCAGCCCGGAGCTTGTTGCGT
ATGGCCATTGATGACGGCGGTGACATCAATACGCCCTGATTTGAAATCGGCATCAATGTA
GATTTGTGTGCCGTCCTGTTTGTGTTTTTGTACAAACTTAATAAGACTGGCGGTATCGTG
CATGAGGAATTTGCCGCACTTGCGGTACGGGTTTTGCATCAATTCGGGGTGTGATTTGTA
TCTCCAGCCACCGTCTTGGTCTGGTGTGAATACAAGCGGAGTATTGTTCGGTGCAAACTC
AAAAAAAGGTTTTTGAGCTGCTTGTAAGGCGGTTTTAATCATGTTTTCTTGGGTTTCCAT
TTTAGATTTCCTTTTGTGTTTGAGTTAGTTGGATCTGACCATTTTCAACGTGCTTGACGT
ATTGGAAACTTGTTTGAGTTTCAATTTTCCTTGTGCCGGATCGTCGGCTTGGATGTTGCC
GTCAGGTGTAGCAAAGACGATGCCGCCTTCGCGTTTTTCTTTGGGCAGTTTGGTTGCTAC
ATCGTGGCTGATTTTTACCGTTCCGCTTTGGATGTTTTGGGGTTGGATTTTGAGCTTGAC
AGTAATTTCTGACTGTTTACCGTGAGCAAGGCAGGCACGCACCGCTTCACTCATGGCTTC
GCCTAATTCACGGTCTAACCAGCCGCTATTTACGGTCGGTATTTGCTTGGAGGCAGGAAC
AAATTTCTTTTCTTGGGTTTCCATTTTTGATTTTCCTTTAAAGAGAGGTTAGTTAGGTTG
CCGGTATCACGGGATGACAATACCGAGGAGGTTATTTAATTTTGAGAATTTCCAAAGATT
CCACGGTGGAGCGGGCTATTCCAAACTCGTTAAGTGCGGCAAAGCCGGCTGCTGGTATGG
TGGATGCTCCGTAATTTATTTTTGGAATAAAATTTTCGTTCAGGGCGACGGCGGTTCTTG
CGAGATTTAGCAAAGCGTCGAAATTTTCTTTAGGGATGGTGATGGTTTCCATGTCGGTAC
TCCATGTGGCTGTTGTTTGTTTCGATGGGTGTATTTAAACATAGCGTTTAATAATATGCA
ACAATCTGTTTAAGATTTTTGTTTAAGGTTTATAAACATTTTGATTATTAAAAGAATTTA
TTTTTGAGATTTCGCAGGCGCAAAAAAACCGCCTATTAAGGCGGCTTTGTCGGTTTTGTG
TTGTTTTCAGGTTCGGCGGGGCATGAAAAAAGCCCGCATAATGCGGGCGGGTTGTTTAAT
CTGTGGGGTCGTATGCCACATCAACTATCAGCTTCGTTTTCCTTAGGTGTTGGTTGATAG
CATAAATTTCGTGCCATGCCTTGCTTAGATAATACTGCCGAAGCATCGGGATTTTAGATA
GCCGGTGTGGCATTGGGATATTTCGTGCGCACCATAGCCGCAACAGAGTGTCTTCGTCAT
CGATTTGGATATGTTTTAAGATTAAGCCGCTTGAACCGACGGGCAATAGATACCTTGCGT
CAGGGTTGGCAAAATGCTTACCGGAACCATCAAAGTCATACAATACATTTGGCTTGAGGT
CTTTGTAGTTCTCTTTTCTGCGCGGCGCAGATACTTCGCCAAGCCACTCAAAGCACAGGG
TTGCTGTTCTGTGATGCAGCTCGTGAACCTTCAACTCTCCTTGTACGCCAAGGAAATTCA
TACCGGCATCGTACTCACCAGGCCACCAAGGGGAATCAAAACGCCTATTTTTGACAATCT
TGAAAGCCCTATCAATATTATCTCGTCGTAATAGCAACATTTTTCAATCCAGCACGCTCC
ACCAAAATACCCTGCCGATAACGGTCAGGCTGTCCAAGGGGGCGTTTTCGTCGCCATAGA
AACCGCTGTTGTGGCTGCGTATCAGAACGCTGTTGCCAGGCTGCCGTATCAGGTACTTCA
CGCGGAACATACCGTCCTGGGCGAAGGCGTAGATTTTGCCGTCGCGTATGGCGGTTTCGC
CCGTATCTACGGCAATTGCCGCGTCTTCTGCGATTTTTTCCTCCATACTGTCGCCGGTCA
GGGTGCAGCAAAACACGTTGTCGGGATTGATGCCTTTGCGTTTAAGCGTGGATTTGCCGA
ACGGCAGGCGGTAGCCGTTGTAATCGGGGATTTCATACGTGCCTACTCCGCCTTTGAAGC
AGCTCTCTTTGAGGTAGGGGACGAAAACATAATCATCGTCGGGCAGCGGGTCGTTGCTGC
TCCACGTCATCGGGCGGTGGATGTCTTTGACTTCGTGGGGTAGGTCGGGGTCAATAAGGA
CGGGCGCGGTTCGGCTGCCTTCACCTGTTCTCAGCCATGTTTCAGATACACCGAATGCTT
TTGCTACTTCAGGCAGCGCCTTTGCCGCTATGCCACGACTTTCCCAGTTTTTCAAAGCCT
GTTGGCTGATATTCAGACGCTCTGCTATGTCAGCCGGCTTTAAAACTCCCTGCTCTTTGG
CTATCTCAAAAAGTCTGTCAGTTGTCTCGTGCATTGTCATTTTTAATCTTATTCGCGGTT
GGCTTAATTATTCTCCCATATTTAAACAAAATGTTGTTACACAAGACTTGATTTTTATCT
AAACATAGTGTTTAATATTAGCATTAGATTTAAACATTTGGTTTATTTATGGATAAAAGA
GTCAATGAAGACAAACGCCTGTTGCAATCAATCGGCAGTTACGCGGAAGTTGGTCGAATA
ACAGGGAATAGCCCTCAATGCGTTTTCAATTGGACGAAGCGCGGGATACCTGCACGAATA
AAACTTAAGTATCCCGACCTGTTTTTGAACTCAAAGAAACCAGACGACCAACCCAAATAA
AAAAAGCCTGTCGTGGAAAGCTCGCCGCGGGCTGGGGAAGCCGCATTGATGACGATAATT
TTTAATATTGCTTGGATTCGGATTTCAAGTGCAACACTAGTGTATTAGTGGTTGGAACAG
ATTCAAGAATAAAACACTTGGCGTTTCGTAGCCAAGTGTTTTTCTTGGTCGGTGGTTCAA
CTCATCTTGAACCCTGCGTATCTCCCGATCACTGATGTTACGGAAATCGGTTTGTTTGGG
GAAGTATTGCCGGATGAGTCCGTTGGTGTTCTCATTCAGCCCTTTCTCCCAAGAATGGTA
AGGGCGACAAAAATAAGTCTCCGCTTTCAATGCTTTGGTTATTTTGGTGTGTTGGTAGAA
CTCTTTGCCGTTATCCATGGTGATGGTGTGCACCCTGTCTTTATGTGCCTTTAATGTCCT
AACAGCTGCCCGGGCAGTGTCTTCGGCTTTGAGGCTATCCAATTTGCAGATGATGGTGTA
GCGGGTAACGCGTTCGACCAAGGTCAATAATGCGCTTTTCTGTCCTTTGCCGACAATGGT
GTCGGCTTCCCAATCGCCGATACGGGATTTCTGGTCGACGATAGCGGGTCGGTTTTCTAT
GCCGACACGGTTGGGTACTTTGCCTCTGGTCCATGTGCTGCCGTAGCGTTTGCGGTAGGG
TTTGCTGCATATTCTGAGATGTTGCCACAACGTGCTGCCGTTGCTTTTGTCTTGGCGAAG
GTAGCGGTAAATGGTGCTGTGGTGGAGCGTGATCTGGTGGTGTTTGCGCAGGTAGGCGCA
TACTTGTTCGGGACTGAGTTTGCGGCGGATAAGGGGGTCGATGTGCTGAATCAGCTGCGA
ATCGAGCTTATAGGGTTGTCGCTTACGCTGTTTGATAGTCTGGCTTTGCCGCTGGGCTTT
TTCGGCGCTGTATTGCTGCCCTTGGGTGCGGTGCCGTCTGATTTCGCGGCTGATGGTGCT
TTTGTGGCGGTTAAGCTGTTTGGCGATTTCGGTGACGGTGCAGTGGCGGGACAGGTATTG
GATGTGGTATCGTTCGCCTTGGGTCAGTTGCGTGTGGCTCATGGCAATCTTTCTTGCAGG

# Appendix A

AAAGGCCGTATGCTACCGCATACTGGCCTTTTTCTGTTAGGGAAAGTTGCACTTCAAATG
CGAATCCGCCGTGCGCTGAAATTCGACCAGTTGATACTCGAATTTCCTGAGCGCGGGGAC
GGCGCATGGGTACACATCGGTTTCCGACGCAACAGCCCGCAACGCAACCAGATACTGACC
GCAACCAAGAAAAACGGCAAAACCGTGTATCTGCCCGGGCTGCATCCTTGAGGTCGTCTG
AAATGGATATTTTATTGAAATACTGGAAGCCGGTAGGTGTATTGCTGCTAATCGTCCTGA
TTTTTACCGCATGGCATTTCGACCGTGCCGAAAAATACCGCATGGGACGGGAGGCTGCTG
CTGCCGAAATCTCGAATCGTCTGAAAGACGGCTATATCGAGCAGGCAAAGCAGGCGCGTT
CTGCCGAGCAGAAGGCCGCTGCCGCGTTTGCCGAACGACAAACCAAATTAGAAGAGGAAA
AACAAAATGCTGAAAAAACTGTTGCCGCTATGCGTCTTGAGCTTAACCGCCTGCGCCACT
ACGCCGCCGCCCAAAATCGCAACAGAAACCTGCCCGCAACCGCTACCGCCGCCACCGCAT
CTGATGGCGCGTCAGATTCCCAAGGCTGGCTATTATTCGGACAGTGCGCTGAAAAATATG
CAGGACTGGCAGAAACAGCCGACGGACATGCAGCCGATTTAAGGGAATGGCAGGCATATG
GTCATGCAGTAAGCAGTCAGCGTGCCGAATAAGCAACCGCCCGAACCTGTAAGAAAAGAT
TACAGGTTCGGGCGGTTTCAGCATTTAATCGAATAAGACGGCGCCGATGCGCGCCAGCAC
ATCGTCCAATACATAATCGGGTACAGTTTCTTTAAAAGAAGCCTTGCGGATTTGCCAGTC
TAGAGATTTGAGCTGGTGGCAGTGGACATTGCCCTGCGTTTCCGTTCCTGCACCGAGTAA
GGTTGAAATCATGCCGCTGCTTCGTGCAGCTGCTGCATTCCCCTGTGAAATGGGGCAGGC
AAAAACCAATCCCGTTGCGCGGTTGAATGCTTTTGGAGACAGAGCCAGCGCAAACCGCCC
GCCCTTGATTTCCTTGCCGCTGGAAGGGTCGAAATTCAAATGGAAAATATCGCCTTTGTC
GGGAATATACATTTCAGACGACCTCGTTGCCGGCATCATCCAAGATTTCCCAGCCTTCTA
CGCGCGGCGGGGTTTCTTCCATTTCGGCAAGCAAGTCTGCCAAGCGGAAACGTCGGGCAG
CACGCACACGGAGTTCGCCGTTATGTACTTCCGCTACCAAAGCGTCGCCGATTTTAAAAT
CCAATTGTTTCAGCATGTCGGCAGGCAGTCGGACGGCCGCCGAGTTCCCCCATTTTTGGA
CACGCAACATAATCTTCACCTTTATTGTATCTACAAAGTAGATACATATTACCATAAAAT
TTCAGTTGTTCAAATACTTGTGCAGAATACGCCAAAAGCCGTCCGAACTGTTTCGGACGG
CTTTTGTACTGTATTTGCGCCTTCAGGCAATATTTTGTTATCCATTTTCAAGATGCAAAA
GCTTTCTAATTGCTTGATGTCGGATTTCGGTTGTTTAGGGATACAAAACCAAGTAAACTA
AAACTGTTTGATTGGAAAATGCTCCGCAAGGAGAAAATTATGTTCAAAAAATCACTTTAT
AAGGCTGCTTTGGCGTATTTCGGCGATTGCGTGGCTGCCCATATTTCAGAACAGTTTTGA
CTGTTTATTCACAAATCAGATGCCTTTAGGGGGTTTGCTTTCCATAATGCAACCAAAATT
TCCAACTCTCTAAATATTGTGTCTTTGCGTTCTTCTTCGCGTATTTTCATAACAAGAGGC
GAAACTGCATTCCACAATTTTATGAAGTTGGTGCAATGCAGCCGTTTAAACAAATCCTCA
TCCAATACTCCCGAGTTGATTGCGCACGCATAAAACTCGTGCCGATTGATTACGGTAAGT
AAATGCCCTCTCTCTTTTTCCCATTGTCCGCCCTTATTTTGATAAATTTCATACAGGTCG
ATCCGCGCGTTGTTGTCGTCAACTGCCGTGCCGCGAATATAAGGCGAAATATGATTGTCG
GCTTCTACAAATTGTGCATCTTGGTAATCATTCAAAATAACATCTAATGTCGCCTTTTGC
TTTGAAGTTTTCTTATTGATGAATATTGTCCCCAGTGCAATGACGGCGGTTACTGAAACA
ACGGTCAGTTGCCAAAACATCAGCCATTCGGCCGTCCCTCATAGGTTAAACTGTACGCTG
TGCCTGAATATGTAAAATAAAGTGAATACAGATACAAACAAGGAGGTTAGGGCAATTACT
GACAACCAACCTCCTTGTTTTATTTGTTTCCGCACCTTACCAGCCATTAAAACCCTCATT
TTTGTATTTCATCGTATTTCCTTAATGGTTTTAACCTTGCTTGTCGATATCTGTATATTA
ATGCCGTGCGCCCGTTCTTTCAAGGGGTGATTTAAAAATCAGGCATCCTTGACATCCTCT
CCTGCTTAAAGGCGGGGGACTCCTGCCGTGTAAACCAATGCCGTCTGAAGGGCTTTCAG
ACGGCATAAAAAAACCGCCTTTGTGTAAAGCGGTTGAAGAAAAGCCTTTCAATAAAAATG
CCGTCTGAATTTCAGACGGCATTGTTGTCGGATATGCCTATTCCTTATCCAGCCGGCGCA
GGGTTTCGGCGAGCTGTTTGAAGTCGGTTTCTCCCGCCGCCAAACTGACAATCAAGTCGT
CAAGCCCTTGGTCGGCGGCAATGCTGATGCCCTGCAAATCCAGATAGGTCAACATTGTTA
AAAGCGCGGTGCGCTTGTTGCCGTCGGGAAAGGCGTGGGCTTTGGCTATGGCTTGTGCAT
AGAGGGCGGCGATTTCGTAGATGTCCTCAAGGTTTTCATACTGCCGCCAGTTGGCAATCC
~~GCGACAATGCGCCGTCCAAGCGCGCCATATCCGCCCGCCCTTTCAAACCCGCTTCATCCG~~
CCAATACGGTTTGATGGATAAGCGCGACCAGTTCGCCGTCTATCATTTGTCGGCAAGTGC
CTTGACGGCTTTTTGATGGGTTTTGGCAATGCGGCGGGCGGCGGCAAGCAGTACGCGTTT
GCCTGCTTCGCCTTTAAGCTCGATTTTGACGGGGCGTGTATTTTGGTTTTGCATCGCTGC
TCCTTGTCTGTTTGCGGGCATTTTAGCTTTTTTCCGGCAGCTTGGGAAATGCCGTCCGAA
AACACTTCAGACGGCATTCTTCTAATAGTGTAATGCAATAGTTACTCCAAGATTTTTGTA
TATGGCTGTTTTTATGGTAAATTAAATTTTCATAAGAAATAAATATATTTAAATCAATGA
AATAAAATTTAGTCGAATCCCACCGTTTCCGCCACAAAGCAAAACCGCCCTGATTCGGGG
CGGTTCTTTTTTGTTCAAGTTGAGACCTTTGCAAAATTCCTTTCCCTCCCGACAGCCGAA
ACCCAAACACAGGTTTTCAGCTGTTTTCGCCCCAAATACCTCCTAATTTTACCCAAATAC
CCCCTTAATCCTCCCCGGATACCCGATAATCAGGCATCCGGGCTGCCTTTTAGGCGGCAG
CGGGCGCAAATCAGTCCGAAATAGGCCGCCCGGGCGTAGCGGAATTTACGGTGCAGCGTA
CCGAAGCTTTGTTCGACCACATAACGGGTCTTAGATAAATACCGGTTGCGTTTGGTTTGC
GTTTCCGTCAGCGGACGGTTGCGGCAGGCTTTGCGCATAATGCCGTCCTGCAACTGATGT
TCTTCCAGATGTTGCCGGTTTTCCGCACTGTCGTAGCCTTTGTCGGCATAGATGGTCGTA
CCTTCGGGTAACCCTTCCAACAACGGCGACAGGTGTTTGCACTCATGGGCATTGGCGGGA
GTAATGTGCAGTTTCTCGATATAGCCTTCCGCATCGGTACGTGTATGTTGTTTGTAACCG
AGTTTGTAGAGGCTGTTTTTCTTTGTCCAACGGGCATTTTTGTCCTTACTCAGTGTGGTT
TGACCGCTGACTTGTCCCTCTTCATCGACTTCTATGGCCTGGCGCTGTTTGCTGCCGACG
GTCTGAATAATGGTGGCGTCAACGACGGCGGCGGATGCTTTCTCTACTTTTAAGCCTTTT
TCGGTCAGTTGGCGGTTAATCAGTTCCAACAGTTCGGACAGGGTGTCGTCTTGCGCCAGC
CGGTTGCGGTAGCGGCATAAGGTGCTGTAATCGGGGATGCTCAGTTCGTCAAAACGGCAA
AACAGGTTGAAGTCGATGCGGGTGATGAGGCTGTGTTCGAGTTCGGGATCGGAGAGGTTG
TGCCATTGTCCGAGCAGGACGGCTTTGAACATGGACAACAGGGGACAGGCGGGACGACCG
CGGTGGTCTCGGAGGTAACGGGTTTTTGACGGTTCAGGTATTGTTCGATCGGCTGCCAA
TCAATCACCTGGTCCAACTTCAATAGCGGGAAGCGGTCGATGTGTTTGGCAATCATGGCT

## Appendix A

TGGGCGGTTTGTTGAAAGAAGGTGCTCATGAGAAATCCCCTAAATGTCTTGGTGGGAATT
TAGGGGATTTTGGGGAATTTTGCAAAGGTCTCGACCTTGTGTTTTTTAAGGTATTCGATA
GTATGGGCGATACCTTTGGGGTTGTTGGTTTCGGTTTTGGTTTTAGACAAAGACGAAACG
GCGATGACGAAGTTTCGTTTGGCGATGTCGATATAGTGAATTAACAAAAATCAGGACAAG
ACGACGAAGCCGCAGAAAGTACAGATAGTACGGAACCGATTCACTTGGTGCTTCAGCACC
TTAGAGAATCGTTCTCTTCGAACTAAGGCGAGACAACGCCGTACCGGTTTTTGTTCATCC
ACTATAACAGCAACCCTGTCGCCGTCATTCCCGCAAAAGAGGGAATCCAGTCCGTTCAGT
TTCGGTCATTTCCGATAAATTCCTGTTGCTTTTCATTTCTAGATTCCCGCTTTTGCGGGA
ATGATGACGGAAGGGTTTTGGTTTTTTCCGATAAATTCTTGAGGCATTGAAATTCCAGAT
TCCCGCCTGCGCGGGAATGACGATTCATAAGTTTCCCGAAATTCCAACATAACCGAAACC
TGACAGTAACCGTAGCAACTGAACCGTCATTCCCACGAAAGTGGGAATCTAGAATCTCAG
ACTTTCAGATAATCTTTGAATATTGCCGCTGCCTTAAGGTCTGGATTCCCGCCTGCGCGG
GAATGACGAATCCATCCGCACGGAAACCTGCACCACGTCATTCCTACGAACCTACATCCT
GTCATTCCCACAAGGACAGAAAACCAAAATCAGAAACCTAAAATTCGTCATTCCCGCGAA
AGTGTGAATCTAGAAATGAAAAGCAACAGGCATTTATCGAAAATAACTGAAACCGAACAG
ACTAGATTCCCGCCTGCGCGGGAATGACGGCTGCAGATGCCCAACGGTCTTTATAGTGGA
TTAACAAAAATCAGGACAAGGCGACGAAGCCGCAGACAGTACAGATAGTACGGAACCGAT
TCACTTGGTGCTTCAGCACCTTAGAGAATCGTTCTCTTTGAGCTAAGGCGAGGCAACGCC
GTACTGGTTTTTTATATCCAATGGGTGCGGCGTTTAATCATAATCAGGCAGATAGGGATA
ACTAATGCCGTCTGAACGACGAATGTTCAGACGGCATTTTTACCTTTGTGCTTATAAGGC
GTTTAGTGCCTGATTAAAGGTTACGCTCGGACGCATCACTTGTGCGGCTTTTTCAGGATT
GGCGGCGTAGTAGCCGCCGATGTCGGCCGCTTTGCCTTGTACGGCGGAAAGCTCGGCAAC
GATTTTCGCTTCGTCGGCGGTCAAAGCGGCTGCCAATGGCGTAAATGCGGCTTTCAGTTC
GGCATCTTTGTCTTGCGCCGCCAATTCTTGCGCCCAGTAGAGGGTGAGGTAGAAATGGCT
GCCGCGGTTGTCGAGTTCGCCGGCTTTACGTTTAGGCGATTTGTCGTTCAACAGCAGTTT
TTCGGTGGCTGCATCCAAAGTGTCGGCGAGGACTTGGGCTTTGGCATTGCCGGTTTTTTG
CGCCAAATGTTCAAACGATACGGCGAGTGCGAGGAATTCGCCCAGCGAGTCCCAGCGCAA
ATGGTTTTCTTCGAGGAATTGTTGAACATGTTTCGGTGCAGAACCGCCCGCGCCGGTTTC
AAACATACCGCCGCCGTTCATCAATGGAACGATAGACAGCATTTTCGCGCTTGTGCCGAG
TTCCAAAATTGGGAACAAGTCGGTCAGGTAGTCGCGCAAGACATTACCGGTTACGGAGAT
GGTGTCTTCGCCGTTTTTCAGACGACCCAAGCTGAACTTGGCGGCTTCTTCAGGAGCGAG
GACGCGGATGTCGAGGCCATTGGTATCCAGTTCGGCAAGGTAGGCTTTAACCTTGGCGAG
CAGGCTCTTGTCGTGCGGACGGTTTTCGTCGAGCCAGAACACGGCAGGCGTGTTGCTCAG
ACGGGCGCGGTTGACGGCAAGTTGTACCCAGTCTTTAACCGGAGCGTCTTTGGTTTGGCA
CATACGCCAGATGTCGCCGGCTTCAACGTCGTGCTGCATTAGGACTTTTCCTGCCGCATC
AATGACTTGGACTTGGCCGTCGGCTTCGATTTCAAAGGTTTTGTTGTGCGAGCCGTATTC
TTCCGCCGCTTGCGCCATCAGTCCGACGTTGGGCACAGTACCCATGGTTGTCGGGTCAAA
TGCGCCGTGTTCGCGGCAGAAGTCGATGGTTGCTTGGTAAACGCCGGCATAGCTGCTGTC
GGGAATCACGGCTTTGGTGTCTTGCGCTTTGCCGTTTTTGTCCCACATACGGCCGGAATT
GCGAATCATCGCAGGCATAGAGGCATCGACGATGACATCGCTGGGAACGTGCAGGTTGGT
GATGCCTTTGTCGGAATCAACCATCGCCAAATCGGGGTTGGCAGCGTAAACGGCGGCGAT
TTCGGCTTCGACGGCGGTGCGGGTGTCCGCATCCAGTTTGTCCAGATTGGCAAGCAGGTT
GCCGAAGCCGTTGTTAACGTTGACGCCGGCAGCAGCCAGTTTGTCGCCGAATTTTTCAAA
AACAGGCGCGAAGAATACTTTGACGGCGTGTCCGAAGATAATCGGGTCGGACACTTTCAT
CATAGTGGCTTTCATGTGCAGCGAGAACAACACGCCTTTTGCTTTCGCATCTTTTACTTG
TTCGGCAAGGAAGGCGAGCAGGGCTTTTTTACTCATCACGGTCGCGTCGATGATTTCGCC
GGCTTTCAGGGCGACGGGCTCGCGCAGCTCTTTTTTGTTGCCTTGTTTGTCGGTGAATAC
GATGGATACGGAAGTCGCTTCAGGTACGATAACAGATTGTTCGTTATGAAAAAAGTCGCC
GCTTTGCATGGTGGCAACGTGGGTTTTGGAGTCTTTGGTCCATGCGCCCATGCTGTGCGG
ATTTTTTTTCGCAAAGTTTTTCACTGCTTTAGGGGCGCGACGGTCGGAGTTGCCTTCACG
CAGGACAGGGTTTACCGCGCTGCCTTTGATGCGGTCGTAGCGTTCGCGTACGGCTTTTTC
TTCATCGGTTTGCGGGTCGGCGGGATAGTCGGGAACGGCAAAGCCTTTAGATTGCAATTC
TTTAATCGCGGCAGTCAGTTGCGGTACGGACGCGCTGATGTTCGGCAGTTTGATTACGTT
TGCATCGGGTTGTTTCACCAGTTCGCCCAATTCGGCAAGCGCATCAGGTACGCGTTGCGC
TTCGGTCAGGTATTCGGGAAACGCCGCCAAAATACGGCCGGAGAGAGAAATGTCGCTGGT
TTTGACATCAATATCGGCGTGGCGGGCAAAAGCCTGCACAATCGGCAGCAGCGATTGGGT
CGCCAGCGCGGGTGCTTCGTCGGTATGGGTATAAACAATGGTGGATTTTTGAGTCATAGG
ATTATTCTCTTGTAGGTTGGTTTTTTCTTTTGGAACACATTGCGCGGGGAATGTGCGTGG
CTATTATGGCATATTTTGGCGGCTTTGTTCGCGCTTTGTTCGATCTTGGCGTGTTTGAAC
GCGGCGGCGTGAAAGGAAGGGGGAAATGGTTTTCCCGCGTTTGGCGGCGGTCGGAGGTGC
TGTGCCTGATGTGCGGCGGCATATTTTCGGTGAAATTGATTTTATAGTGGTTTAAATTTA
AACCAGTACAGCGTTGCCTCGCCTTGTCGTACTGCTTGTACTGTCTGCGGCTTCGTCGCC
TTGTCCTGATTTAAATTTAAACCACTATAATATTCGGTAACTGTCGGAATATCTGCTAAA
ATTCCGCATTTTTCCGTCCCGGGACACTCGGGGCGTATGTTCAATTTGTCGGAATGGAGT
TTTAGGGATATGGGCTTGAAAAAGGCTTGTTTGACCGTGTTGTGTTTGATTGTTTTTTGT
TTCGGGATATTTTATACATTTGACCGGGTAAATCAGGGGGAAAGGAATGCGGTTTCCCTG
CTGAAGGAGAAACTTTTCAATGAAGAGGGGGAACCGGTCAATCTGATTTTCTGTTATACC
ATATTGCAGATGAAGGTGGCGGAAAGGATTATGGCGCAGCATCCGGGCGAGCGGTTTTAT
GTGGTGCTGATGTCTGAAAACAGGAATGAAAAATACGATTATTATTTCAATCAGATAAAG
GATAAGGCGGAGCGGGCGTACTTTTTCCACCTGCCCTACGGTTTGAACAAATCGTTTAAT
TTCATTCCGACGATGGCGGAGCTGAAGGTAAAGTCGATGCTGCTGCCGAAAGTCAAGCGG
ATTTATTTGGCAAGTTTGGAAAAAGTCAGCATTGCCGCCTTTTTGAGCACTTACCCGGAT
GCGGAAATCAAAACCTTTGACGACGGGACAGGCAATTTAATTCAAAGCAGCAGCTATTTG
GGCGATGAGTTTTCTGTAAACGGGACGATCAAGCGGAATTTTGCCCGGATGATGATCGGA
GATTGGAGCATCGCCAAAACCCGCAATGCTTCCGACGAGCATTACACGATATTCAAGGGT

## Appendix A

TTGAAAAACATTATGGACGACGGCCGCCGCAAGATGACTTACCTGCCGCTGTTCGATGCG
TCCGAACTGAAGACGGGGACGAAACGGGCGGCACGGTGCGGATACTTTTGGGTTCGCCC
GACAAAGAGATGAAGGAAATTTCGGAAAAGGCGGCAAAAAACTTCAAAATACAATATGTC
GCGCCGCATCCCCGCCAAACCTACGGGCTTTCCGGCGTAACCACATTAAATTCGCCCTAT
GTCATCGAAGACTATATTTTGCGCGAGATTAAGAAAAACCCGCATACGAGGTATGAAATT
TATACCTTTTTCAGCGGCGCGGCGTTGACGATGAAGGATTTTCCCAATGTGCACGTTTAC
GCATTGAAACCGGCTTCCCTTCCGGAAGATTATTGGCTCAAGCCGGTGTATGCCCTGTTT
ACCCAATCCGGCATCCCGATTTTGACATTTGACGATAAAAATTAATCGCATAGCAAATCA
AAATAGAAAATGGCGGAGTGCGTGGGGTAAAAATAAGGATAGCGTTTTTTCATTTGGATT
GACGATAATTTCTGATTGCTTTGCGTGTGCTGAAATGGCAAAGAAAATGCCGTCTGAAGT
CTTCAGACGGCATTGTTTTGTTTTGGATGTTATTCGGGCGCGCGGAAACTGTCGTGGCAG
GATTTGCAGCTTGCGCCGGTTTCGCCGTAGGCGGCTTTGATTTCGTCCAGTTTGCCGGTT
TGGGCGGCGGCGTTGAGTTTTTCGACGGCGGCGGCGAATTTTGTTTTTTCGGCTTCAAAT
TTTGCACCATCCGACCAAACGGCGGGCAGTGCGCGGCCGTTGCCTTGCGGATCGGACTCA
AAAAGTGTGAACGGTTTCTTGCTGCTTTCGGCAAACGACGCTGCCGCCTGTTTGAATTTT
TCGACATCGTAAGGTTCTTCGTCTTTGACCATTTTGCCCATGCGTGTGAATTCGGGCATC
ATGGATTTGAACGCGGCGGTGCGGTTTTCGGAAATTTCGCCTTTGGGTTGGGAAGGTATT
CCGCTGCCTCCGCAGGCGGAAAGGAGCAGTGTGATGGCGGCAGCAGCAAGGCTGATTTGG
GTTTTCATATTGAATGTGTCCTGTCGTGGTGGTATGGTTGTTGTCATTTTCAGTCGGCGC
GATGCCGTCTGAAGGGTGTTATGATACCTGAAACAGGTTTGGGAAACGAGAAAGAAAGGA
AAAACAATGGCTGTTTTAATTACCGGTGCTTCGGCAGGATTCGGCGAAGCGATGTGCCGT
GCGTTTGTCGGGGCGGGATACCGCGTTATCGGTGCGGCGCGCCGTGCGGACAGGCTTCAG
GCCTTGGCGGATGAATTGGGTGCTTTGTTTTACCCTTTGGAAATGGACGTGTCGCGACGC
GAGTCGGTGGAAAACGCCTTAAACGGCATCCCCGATGAATTTTCCGACATCGACTGCCTC
ATCAACAATGCCGGGCTGGCTTTGGGTCTGGACACGGCGGACAAGGCGGATTTTGAAGAT
TGGGAAACGATGATTCAAACCAATGTTTTGGGTTTGACGTTCCTGACGCGCAAAATTTTG
CCGCAAATGGTGGAACGCGGCGGCGGTTATGTGATGAATTTGGGTTCGATTGCAGGCAAT
TATGCTTATGCCGGCAGCAACGTTTACGGGGCGACCAAGGCGTTTGTGCGCCAGTTCAGC
CTGAATTTGCGCGCGGAGTTGGCGGATAAGAACATCCGCGTTACCAATATCGAGCCGGGT
TTGTGCGGCAATACGGAGTTTTCCAATGTGCGCTTCAAAGGCGATGACGAGAGGGCGGCG
GGCGTGTATGAGGGTGTGGAATTTATCCGCCCCGAAGATATTGCGGAAACCGCATTGTGG
CTGTACCAGCGGCCGGCGCATATGAATGTGAACACGATTGAAATTATGCCCGTGGCGCAG
ACTTTTGCAGGAATGAAAGTGATAAAAAAAGCCGTGCCCGAAGTGCGGGAAGACTTTGAA
AAACAGAGTATGTCGCTGTTTTCCCGCATCAGGTCCTGGTTCAAATGATGCAAAATGCCG
TCTGAAGACAGTTTCAGACGGCATTTTTACGGGTATTTTTACGGAGTAGGCAATAAGCCC
GCCAATTTGGGGTTGCCTTCTTTCGGAATCGGGCGCGGATTGCCTTCCGCATCGATGGCA
ACATAAGTGAACACGGCTTCGGTTACGAGGTAGCGGTCTTCGGTAACGCAATCGTTCATC
AAAGTTTTCACCCAGACGTCGACTTTAAGCTGGAGGGAAGTGTTGCCCACGCGGACGCAA
TGCCCGTAGCAGCAGACGACGTTGCCGACCTTGACCGGGCGGATGAAGTTCATTTCCTGA
ACGGCGACGGTAACGATGCGTCCCCGCGCGATTTCCGCCGCCAATATGCCGCCGCCCAAA
TCCATCTGCGACATAATCCAGCCGCCGAAAATGTCTTGGTTGGGATTGGTATCGCGCGGC
ATAGCGACGGTACGCAGGAGCAGTTCGCCTTGAGGGCGTTGGCGGTTGCCTTCTTCGTGC
TGCATAAAGTTTCCTTGTTTTATTGAAATATAAATCGAACCTGCACCCCTGCCCGAAACG
GGAAGTCGGGAAGTGTGTAGTTTAACCCATTTGAGACATACAAGGGCGGGCGCGGAACAG
ATTCGCAAGGCGTATTGTAGGGCGGGGCTGTAGAGTGGGCTTCAGTCCGCCAATCCCGCC
AAATCCTACCCTAAGCAACTGAACCGTCATTCCCACGAAAGTGGGAATCTAGAACGCGGG
GTTTCAGTCATTTCCGATAGATTCCCGCCGCGTCGGGGGTCTGGATTCCCGCCTGCGCGG
GAATGACGAATCCATCCATACGGAAACCTGCACCACGTCATTCCCACGGAAGTGGGAATC
TAGAATCTCGGGGTTTCAGTCATTTCCGATAGATTCCCGCCGCGTCGGAGGTCTGGATTC
CCGCCTGCGCGGGAATGACGGGTTTTAAGATTACGGTGTTGTCGGACGGATTTCAAGATT
ACGGTGTTGTCGGAACGCAACTGAACCGTCATTCCCACGAAAGTGGGAATCTAGAATCTC
GGGGTTTCAGTCATTTCCGATAGATTCCCGCCGCGTCGGGGGTTTGGATTCCCGCCTGCG
CGGGAATGACGAATCCATCCATACGGAACCTGCACCACGTCATTCCCACGAAAGTGGGAA
TCTAGAACGCGGGGTTTGGGCAACTGTTTTTATCCGATAAGTTTCTGTGCGGACAGGTCT
GGATTCCCGCCTGTGCGGGAATGACGAATTTCAAGATTGCGGTGTTGTCGGACGGGTTTT
GAGATTACGGTGTTGTCGGAGCGCAACTGAACCGTCATTCCCACGGAAGTGGGAATCTAG
AACGCGGGGTTTCAGTCATTTCCGATAGATTCCCGCCGCGTCAGGGGTCTGGATTCCCGC
CTGCGCGGGAATGACGAATCCATCCATACGGAAACCTGCACCACGTCATTCCCACGGAAG
TGGGAATCTAGAATCTCGGGGGTTTCAGTCATTTCCGATAGATTCCCGCCGCGTCAGGGG
GCTGGATTCCCGCCTGCGCGGGAATGACGAATTTCGAGATTACGGTGTTGTCGGGAATGA
CGAATCCATCCATACGGAAACCTGCACCACGTCATTCCCACGGAAGTGGGAATCTAGAAC
GCGGGGTTTCAGTCATTTCCGATAGATTCCCGCCGCGTCGGGGGTCTGGATTCCCGCCTG
CGCGGGAATGACGAATCCATCCATACGGAAACCTGCACCACGTCATTCCCACGGAAGTGG
GAATCTAGAATCTCGGGGTTTCAGTCATTTCCGATAGATTCCCGCCGCGTCGGAGGTCTG
GATTCCCGCCTGCGCGGGAATGACGGGTTTCGAGATTGCGTTGTTGTCGGGAATGCAACT
GAACCGTCATTCCCACGGAAGTGGGAATCTAGGACGTAAAATCTAAAGAAACCGTTTTAT
CCGATAAGTTTCTGTGCGGACAGGTCTGGATTCCCGCCTGCGCGGGAATGACGGGTTTCG
AGATTACGGTGTATCGGGAATGATGGGAAACGGTGGGAATTGTGTAAAAAATGCCGTCTG
AAGGTTCAGACGGCATCGGTATCGGGGAATCAGAAGCGGTAGCGCATGCCCAATGAGACT
TCGTGGGTTTTGAATCGGGTGTTTTCCAAGCGTCCCCAGTTGTGGTAACGGTATCCGGTG
TCCAAGGTCAGCTTGGGCGTGATGTCGAAACCGACACCGGCGATGACACCAAGACCCACG
CTGCTGATGCTGTGGCTTTCGTGATAGGGAGGTTTGCTGGGATCAGTTTGTATAATAGGA
CCTCCCTGTGCAGCGCCTTGCGTTGGTTTAGAGGTAACAATCGTGGTTTTGGTTTCCACC
GAATGAACTTGATGTTTAACGTGTCCGTAGGCGACGCGCGCGCCGATATAGGGTTTGAAT
TTATCGTTGAGTTTGAAATCGTAAATGGCGGATAAGCCGAGAGAAGAAGAGGCGTGGAAG

## Appendix A

CTGCCGTTTCCCTGATGTTTTGTTTGGGTTTCTTTGTAGTTGTTGTTTATCTCTTCAGTA
ACTTTTTTAGTAGAAGAATTACTTTCTTTCCATTTTCTGTAACTGGCATAATCTGCCGCT
ATTCTCCAGCCGCCGAAATCATAGCCGACCGACACCCGGGGGTGGATGGAATGCGCACGG
ATGTTTCTGAAATAATCGCTTACCGTGCTTGTGTTGTTTGCACCGGTTGCTTGCGGATAA
TCGTGGGTAATGCGTTCGGCGGCATAAGCTAAATCCGCCTGCACATAATACGGGCTGCGG
CTGCCGTCTTCACTTGCCGCCTGCGCTGCGGAAGAGAAGAGAAGAGAAGAGAAGAGAAGA
GAAGAGAAGAGAAGAGAAGAGAAGAGAAGAGAAGAGAAGAGAAGAGAAGAGAAGGTTTTT
TGGGGGCTGGATTCATTTTCGACTCCGTATTCGGTTTTAACTGATTAAAAAGAACAATTT
TCAATGATGTTGCAGGAGCGGACTATATCAGGTTTGTGGCGATGTTTCAACACAATATAG
CGGATGAACAAAAAAGAGAACGATTCTCTAAGGTGCTGAAGCACCGAGTGAATCGGTTCC
GTACTATCTGTACTGTCTGCGGCTTCGTCGCCTTGTCCTGATTTTTGTTAATCCACTATA
AAGACCGTCGGGCATCTGCAGCCGTCATTCCCGCGAAAGTGGGAATCTAGAAATGAAAAG
CAGCAGGAATTTATCGGAAACGACCGAAACCGAACGGACTGGATTCCCGCCTGCGCGGGA
ATGACGGGATTTTAGGTTTCTGATTTTGGTTTTCTGTTTTTGAGGGAATGACGGGATGTA
GGTTCTTAGGAATGACGTGGTGCAGGTTTCCGTGCGGATGGATTCGTCATTCCCGCGCAG
GCGGGAATCTAGACCTTAGAACAACAGCAATATTCAAAGATTATCTGAAAGTCTGAGATT
CTAGATTCCCACTTTCGTGGGAATGACGGTTCAGTTGCTACGGTTACTGTCAGGTTTCGT
TTATGTTGGAATTTCGGGAAACTTATGAATCGTCATTCCCGCGCAGGCGGGAATCTAGAC
CTTAGAACAACAGCAATATTCAAAGATTATCTGAAAGTCCGAGATTCTAGATTCCCACGA
AAGTGGGAATCCAGGATGTAAAATCTCAAGAAACCGTTTTATCCGATAAGTTCCTGCACT
GACAGACCTAGATTCCCGCCTGCGCGGGAATGACGGGATTTTAGGTTTCTGATTTTGGTT
TTCTGTTTTTGAGGGAATGACGGGATTTTAGGTTTCTGATTTTGGTTTTCTGTCCTTGTG
GGAATGACGGGATGTAGGTTCGTGGGAATGACGTGGTGCAGGTTTCCGTGCGGATGGATT
CGTCATTCCCGCGCAGGCGGGAATCTAGACCTTAGAACAACAGCAATATTCAAAGATTAT
CTGAAAGTCCGAGATTCTAGATTCCCGCTTTCGCGGGAATGACGAAAAGTGGTGGGAATG
ACGGTTCAGTTGCTACGGTTACTGTCAGGTTTCGGTTATGTTGGAATTTCGGGAAACTTA
TGAATCGTCATTCCCGCGCAGGCGGGAATCTAGTCTGTTCGGTTTCAGTTATTTCCGATA
AATGCCTGTTGCTTTTCATTTCTAGATTCCCGCTTTTGCGGGAATGACGGCGACAGGGTT
GCTGTTATAGTGGATTAACAAAAACCAGTACGGCGTTGCCTCGCCTTAGCTCAAAGAGAA
TGATTCTCTAAGGTGCTTAAGCACGAGTGAATCGGTTCCGTACTATCCGTACTGTCTGCG
GCTCGCCGCCTTGTCCTGATTTTTGTTAATTCACTATATCGCGATTTTTCGGCATTTGCC
TTTCGGGGCGGCTTGTGTCTCGTGCGTGATGTTGCGTGTGGGAATGTTCGGATTGTCAGA
AGCAATATGGGAGAAGATGATGTATGAGATAAAACAGCCTTTTCATAGCGGATACTTGCA
GGTGTCTGAAATTCATCAAATTTATTGGGAGGAATCGGGCAATCCCGACGGTGTGCCGGT
TATTTTTTTACATGGCGGGCCGGGCGCGGGGGCTTCGCCTGAATGTCGGGGTTTTTTCAA
TCCCGATGTGTTCCGCATCGTCATCATCGACCAGCGCGGTTGCGGACGTTCGCGCCCGTA
TGCTTGTGCGGAAGACAATACGACTTGGGATTTGGTGGCGGATATTGAAAAAGTCCGTGA
AATGCTGGGTATCGGGAAATGGCTGGTGTTCGGCGGTTCGTGGGGCAGCACTTTGTCGCT
GGCTTATGCCCAAACCCATCCTGAACGGGTAAAGGGATTGGTGTTGCGCGGGATATTTTT
GTGCAGGCCGTCTGAAACGGTGTGGCTGAACGAGGCGGGCGGTGTGAGCCGGATTTATCC
GGAACAATGGCAAAAATTTGTCGCGCCGATTGCTGAAAATCGGCGGAACCGGCTGATTGA
GGCGTATCACGGATTGCTGTTTCATCAAGATGAAGAAGTGTGCCTGTCTGCCGCGAAGGC
TTGGGCGGATTGGGAAAGCTATCTGATCCGTTTCGAGCCGGAGGAAGTGGATGAAGATGC
TTATGCCTCGCTGGCAATCGCGCGTTTGGAAAACCATTATTTTGTCAACGGCGGTTGGTT
GCAGGGCGATAGGGCGATTTTGAACAATATCGGCAAAATACGGCATATCCCGACTATTAT
CGTACAGGGGCGGTATGATTTGTGTACGCCGATGCAGAGTGCGTGGGCGCTGTCGAAAGC
CTTTCCCGAAGCGGAATTGAGGGTGGTTCAGGCAGGGCATCGTGCGTTCGATCCGCCTTT
GGTGGATGCGTTGGTTCAGGCAGTTGAGGATATTTTGCCCCATTTGTTGTAAAAAGTTCC
GCATAAAAAAGCAGCTTCTGTTTGGAAGCTGCTTTTGTTTTGAATGGTTTAACGCAGTTC
GGAATGGAGTTTGCCCAATAATGCGGATGCGTCTTTGCCGGCATATGCGCTGCCGTCTTT
GTTGAGCAGGACGATGCGCGAGCCGTTGGCGACAGGTTCTGCATAGACAATCAGTTCCGG
CTGTTCGGCAGGTTTCTCCGCTTTGCCTTTGCCCAGCAGGCGTTTGAACAGGCCGGGTTT
TTGTTCGGTAACTGCATTGCTTTCGTTCGGGGCTTTTTGAACCAGGAAGGCGTGGCGTTC
GGTGTTTTGACCGACGACGGTCAGCCCGATGCGGTCGAGGGCGAGCACGGTGCGCCGCCA
GTTTCTGCCGTAGTCGCCAAAGACAATCAGGCTTTTGCCTTCGATACGCGCCATTTCGTT
GGCGGCGGGAAGGGTAGGTTTTTTTGCCGATGCGTTTTCCGCCTGCTGTCCGTCAACGCC
CAAATATTGCATAAAGCGCGTCAGGAAAGCGGCTTCGAGGTTGGGATCGGACGGGGAGGG
CTGCCATACGGTCGTGTCTTTGTCTTTGCCGCCGTACACTTCTTTCATGGCTTTGTGGGC
GAAGAAGATGTCGGAAACGCCGTTTTTGCCCTGTTCGATACGGACGATGAATTTGTCGCG
CTCGCCGGTGGAGTAGATGCCGCCCAAGCCGACTTTGTCGAAGAGGCGGCGCAAGCTGTC
TTGGGGGATTTTGGCGCGGTTTTCCGCCCACTCGGTTTCCATTTGTCCGATGGCGGGTTC
TTCGGATTTGATGTCGAAGCCGTTTTCCTGCCAAAAGGCTTTCAGGAGCGGCCAGATTTC
GGCAGGAGACTTGCCGTCGACAACGAGCCAGCGTTGGCTGCCGTCGCGCTCGAGGCGGAC
ACCTTTGACGCTTTTCAATACTTCGGCATCGGCAGGCTGTTGGACGGCGGGTGTGCGGCG
TTTTTCCAAATCGCTGGCGCGGACGGCGCCCGAACCGGCAGGCAGGCGGTAGAGGTTGCC
TTGGTCGGGGTTGTTCAAATCAGGTGGGACTTCAAGTTTGATCAGGCGGTGCGACCGGCT
TTGGTAGTCGAGCTTGGGCTGTTCGGTTTTGCTGCCGGAGCAGGCGGCAAGCCCGATGAG
TGCGAGCGCGGCAATGACGGGTTTGATATGGGTCATCGTGTCATCCTGTGTGATGGATAT
TAAAGTGTTTGTTGCGTTATGCCGTCCGAACGGTTCGGACGGCATGGCTATATTTAAAGT
TGTCCTGAGGCTTTCAGGGCGGCGCGGACTTTTGCTTGTCCGTTTTCCGTCAGCGGAACG
AGCGGCAGGCGGACGTGCGGTTCGCATCTGCCCAGGGCGGATACCGCCCATTTCGGTGCG
GCGGGGCTGGGTTCGCAGAACATGGTGTCGTAAATCGGAATCAGTCGGTCGTTGAGTTCG
CGTGCAAGGGCGATATCGCCTTGAAGCGCGGCGCGGCACATATCGGCAAAGAGCTTGGGC
GCGGCGTTGGCGGCTACGGTAATCACGCCGTGTCCGCCGCAGAGCATGAACGGCAGGGCG
GTGTGGTCGTCGCCGGAAAGGACGACGAAGCCTTCGGGCGCGCGGTTGATGAGTTCGATG

## Appendix A

TTGCTGCCGATGTTGCCGCTGGCTTCTTTCACGCCGACGATGTTGGGGATTTCGGCAAGG
CGCAGGATAGTGTCGTTAGTCATGCTGACGACGGTACGGCCGGGCACGTTGTAGATAATC
ATCGGAATCGAAGTGGCTTCGGCGATGGTTTTGAAATGTTGGTAAATGCCTTCTTGGGAG
GGCTTGTTGTAATAGGGGACGACGGAGAGGGTGTAGTCCGCCCCGGCTTTTTCGGCGGCT
TGGGAAAGGGCGATGGCTTCGACGGTGTTGTTTGCCCCTGTGCCGGCGATGACGGGGACG
CGTTTGGCAACGTGTTTGACGACGGCTTCGATGACGGCGGTGTGTTCTTCGACGGAGAGG
GTGGCGGATTCGCCTGTCGTGCCGACGGCAACGATGCCGTCCGTGCCGTTTTCAATGTGC
CAGTCGATTAAGTCGCGGAGTTGTTCGTAATGGATGCTGCCGTCTTGATTCATCGGGGTA
ATCAGGGCAACCAAGCTACCTTGTAACATACAGAACCTTTTATCAGTTGTGGTGTAGGGG
CGGTAATGCTTCCGATTGTAGCCTACTTTACCGCAGGTGTGAAATCCGGCGGGTTGCAGA
TGTGGGGCGTTTGCGCCGAAAGGTATGGTGGAAATTGATTTTTCCTGTTTGAAATCATTT
TATTATATTCGCCGGTTTATGCCGGTGCCGTCGGATTTATAGTGGATTAACAAAAACCAG
TACGGTGTTGCCTCGCCTTAGCTCAAAGAGAACGATTCTCTAAGGTGCTGAAGCACCAAG
TGAATCGGTTCCGTACTATTTGTACTGTCTGCGGCTTCGCCGCCTTGTCCTGATTTTTGT
TAATCCACTATAAAATGTGGTAAACGTGTGGACCAGACGGATGCCGTCTGAAATGCAAAT
TGAAGCCGTGCGGCAGATTCGCTACAATCCGCGCTTGGATTTTTCAACCTTTAAAATAAG
GAAATACAATGAGCGGTCAGTTGGGCAAAGGTGCGGATGCGCCTGATTTGGTGTACGGTT
TGGAAGACAGGCCGCCGTTCGGTAATGCGCTCTTGAGCGCGGTTACCCATCTTTTGGCGA
TTTTTGTGCCGATGATTACGCCCGCGCTGATTGTGGGCGGCGCGCTGGAATTGCCGGTGG
AGATGACGGCGTATCTCGTGTCGATGGCGATGGTTGCGTCGGGTGTCGGCACTTATTTGC
AGGTCAACCGCTTCGGGCCGGTCGGTTCGGGGATGCTGTCCATCCAGTCGGTGAATTTTT
CGTTCGTTACCGTGATGATTGCGCTGGGCGCGGGGATGAAAGAGGGCGGTTTGACTAAGG
ATGCGATGATTTCGACGCTCTTGGGCGTATCGTTTGTCGGCGCGTTTTTGGTGTGTTTCT
CGGCGTGGCTTCTGCCGTATTTGAAAAAAGTGATTACGCCGACGGTCAGCGGCGTGGTCG
TGATGCTCATTGGTTTGAGTTTGGTACACGTCGGCATTACCGATTTCGGCGGCGGCTTCG
GCGCGAAGGCGGACGGCACGTTCGGCTCGATGGAAAACTTGGGGCTGGCATCGCTGGTGT
TGCTGATTGTGTTGGTGTTCAACTGCATGAAAAACCCGCTGTTGCGCATGAGCGGCATTG
CGGTCGGGCTGATTGCCGGCTATATCGTCGCGCTGTTTTTGGGCAAGGTGGATTTTTCCG
CGCTGCAAAACCTGCCGCTGGTTACGCTGCCCGTACCGTTTAAATACGGTTTTGCTTTCG
ACTGGCACGCGTTTATTGTGGCGGGCGCGATTTTCTTGTTGAGCGTGTTTGAGGCGGTCG
GCGATTTAACCGCGACGGCAATGGTGTCCGACCAGCCGATTGAAGGCGAGGAATACACCA
AACGCCTGCGCGGCGGCGTGTTGGCTGACGGCTTGGTGTCGGTGATTGCGACGGCTTTGG
GTTCGCTGCCGCTGACGACGTTTGCGCAAAACAACGGCGTGATTCAGATGACCGGCGTGG
CTTCGCGCCATGTGGGCAAATATATTGCCGTGATTTTGGTGCTGTTGGGTCTGTTCCCCG
TTGTCGGTCGCGCGTTTACGACGATTCCGAGTCCGGTGTTGGGCGGCGCGATGGTTTTGA
TGTTCGGCTTAATTGCGATTGCGGGCGTGCGGATTTTGGTCAGTCACGGCATCCGCAGGC
GCGAAGCGGTGATTGCGGCAACGTCGGTCGGTTTGGGCTTGGGTGTCGCGTTTGAGCCGG
AAGTGTTTAAAAACCTGCCCGTCTTGTTCCAAAACTCTATTTCCGCCGGCGGCATTACGG
CAGTCTTGCTGAATTTGGTCTTGCCCGAAGATAAAACCGAGGCGGCGGTCAAGTTTGATA
CCGACCACTTGGAACACTGATTTTGAAAATGAATGCCGTCTGAAACAGAATCCCTGTTTC
AGACGGCATTGTTTTTGAGGCTTATACTTTTTCGTTTTTAATACGCGTTGTCGGCGTGT
TTCACTTAATACCATTCCGGCAGACACGGAGACGTTCATGCTTTCGACTGTGCCGAACAT
GGGTATAGACACCAGCATGTCGCAATGTTCGCGCGTGAGGCGGCGCATACCGTCGCCTTC
GTTGCCCATTACCCACGCCGCGCTGTCGGGCAGATTGCAATGGTAAAGGTCGGACTCGCC
GCTCATATCGGTGCCGATAATCCAAATGCCGTATTCTTTCAATTCGCGCAGGGTGCGGGC
GAGGTTGGTTACGGTGATATAGGGGACGGTTTCCGCCGCACCGCAGGCGACTTTGCTGAC
GGTGGCGTTCAGCCCCGCGCTTTTGTCTTTCGGTGCGATGACGGCGTGTACGCCCATTGC
GTCGGCGGTACGCAGGCACGCGCCGAGGTTGTGCGGATCGGTGATGCCGTCGAGTATCAG
CAGCAGCGGCGGTTCGCTGAGGTTTTCCAATACGTCTTCGAGGTGGACGTGGTTTTTGGA
GGCATCGATAAATCCGACCACGCCCTGATGGCGCGCGCCTTTGCTGATGGCGTTGAGGCG
GTCGGCATCGGCAAAATATACGCGGATGTTTTCGTTTGCCGCCTTTTCCAACACTTCGCG
CGTGCGTGCGTCTGATTTGCCTTCTTGGATGTAGAGTTCGACGATGGATTTGGGGTTTTG
CCACAATCGGGCGTTGACGGCGTGGAAGCCGTAGATGGGTCTTTGGTTTGCCATGATGGT
GCTTTGTAAAAAGGGTTCAGACAGCATTATAGCAATTTGCCGGTATGCCGTCTGAAAGGG
TTAAAACAGGTAGGCGATGTATTTCACCAACAGGATAAACAAGATGGATACGGCGCAGCC
GATTTTGAACGCCGTGCCGACGACAAGCCCCAACAGCGTACCCAAGCCCGCTTTACCTGC
CTGAAGCATATTGCGCCGTTCGATCAGTTCGCCTGCCGCCGCGCCGATAAAGGGACCGAG
TATTAGTCCGGGAAGGGAGAAAAATATGCCGATGATGCTGCCGGCCAATGCGCCGCGAAC
GGCGAGCTTGCCCGCTCCGGTATATTTGTCCCCCATATGCCTGCCACATAGTCCGCCAG
TATGCCGGCAAGGCTGATGAGTCCGACCGTCCACAAAACGCCCGCGCCGTAGATTTGGTA
GCCGCCGGCATAGGCAAGCAGCCATGTTCCGGCAAACATCAATGCCAATCCGGGCAGGGC
GGGGTAAACGATGCCCGCCGTGCCGACGGCTATCAGGGCGAGGGCGAGGATGACGGTCAG
TACGGTCATAGGTTCAACCTTTTCTTTTGTTTTGAAAAAAACGGCTTAACACGGCGCGGC
ATTCTTCTTGCAGGATTCCGCCCCGTATGGCGGTGTGCGTATTGAGGCGTTTGTCGGCAA
ACAGGTTGACGATGCTGCCTGCCGCGCCGGTTTTGGGTTCTGCCGCCCCGTAGATCACAC
GCCTGATTCGTGCCTGTATCAGTGCGGACGCGCACATGGCGCAGGGTTCGAGGGTGATAT
ATATGTCGCATCCGTCAAGGCGGTAGTTTTGTATTTCTCTGCCTGCCTGTGCCAAGGCGT
TGATTTCGGCGTGTCGGCTGACATTGCAGTCGGCAATGCAGGTGTTGTGTGCCGATGCGA
TGATTTTGCCGTCTGAAACGATGACTGCCCCGACGGGTATTTCGCCGTCGGCGGAGGATT
GTTCTGCTTGGCGCAGTGCTTCGCACATGAAGTGTTCCATTTCTTCCTGCGGCGGAAAGG
CGGCGACGGGCGGATGGTTTTTTAACTCGGCAAGCAGGCGGGCTTTATGCGCTTGGGACA
TTTCTTGCGGCGGCGTGCCGTCCAGCAGCGACTCGAGTTGCCACAGTGTGCTTTTCGTGA
GGGTCAAACCCGATGCTTTGAGCAGCAGAAAGGCTTTGACCGAACCGTTTTGCCGCAGTT
CTTCGAGTGTACGGATACCGAGCCTGTGCAGGGCGGCGACGGTTTTGGGGGCGAGCGGCG
GTGTGGTCAGCATGGTTTATGCGCCGAAAAACCGTTTTGCCGCCTCAATCAGGCGTGTGC

## Appendix A

ATGAAGTGCAGTCTGAAAACGGGTCGGCAACGCAGTCTAAAGGTGTTTTGCGCAACCAAG
TCAGTTGGCGTTTGGCAAGTTGGCGGGTGGCGGCAATGCCTTTCTCGACAAATGCCGGGA
AATCGGTTTTTCCATCCAGATATTTCCATGCCTGACGGTAGCCGACGCAGCGGATGGCGG
GGGAGTAGGCGGTCAGGCCGGGATAGCGGCGGCGCAGGTTTTCTACTTCGCCGATAAAGC
CCTGTTCAAGCATCAGGTGGAAACGCAGGGCGATGTTTTCATGCAGGCGGGCACGGTTTT
CGGGAATCAGGGCGGCGGTATGCAAATCAAAAGGGAGCGTATGGGAGGTCAGGCTGCCGA
GATGTGTGCTCATCGGTTTGCCGGTTAAATAATAAACTTCCAAAGCGCGTCCGATACGCT
GGCTGTCGTTCGGTTTCAGACGGCATGCGGTTTCAGGGTCGACTTTTTGCAGGGTGCGGT
AGAGGAAATCCAAGCCGTACATCTGTTTTTGTTCGTCCAAGTCGGCACGCAGGCAGGCGT
CGGCTTCGGGCAAATCGTTCAAACCTTGGGTCAGGGCGCGGAAATACATCATCGTGCCGC
CGACAATAAGGGCAAACCTGCCGCGTGAGGAAATTTCCCCGACCAAGCGCGTGCAGTCTT
CGACAAAGCGGGCGGCGCTGTATGATTCGGTAGGCGGGATGATGTCGATAAGGTGGTGCG
GGACAAAGGCGCGTTCGGAGGCGGACGGTTTCGCCGTGCCGATGTCCATATCGCGGTAAA
CCAGCGCGGAATCGAGGCTGATGATTTCGACAGGCAGGGTTTCGGCAATTTTGAGGGCGA
GCGCGGTTTTGCCTCCGGCGGTCGGCCCGAGCAGGGCAAAGGCTTTCGGGGTCGGCATAA
CGTTTCAGGTTTGGAAAAATACGGATTATAGCGGAAAGCGTGCCGACGTTATATTTTGGT
TTGCGGAAGCACGCCGACGGCAAGGGGGCGTGTTTACCGTATGCCTTTATATAGTGGATT
AACAAAAACCAGTACGGTGTTGCCTCGCCTTAGCTCAAAGAGAACGATTCTCTAAGGTGC
TGAAGCACCGAGTGAATCGGTTCCGTACTATCTGTACTGTCTGCGGCTTCGTCGCCTTGT
CCTGATTTTTGTTAATCCACTATAAAATTTCAAACCGACGCGCCGGGTTTTCAATATGCC
CGCGCCCGATGCCGCCTTGTCCGCAGGCATCAGCGGCAGTGTCCGATTTTTTGGGGAATG
CCCGTCCCGGGCGTATTTAAAGGTTCGGCGGTGCGGCGTTTTCCTGCGGCAAGGCTTCAG
ACGGCATCTCTGGTGCGTCCGTTAGACAAGGCGTGCGCTTGGGGCGATAATGGCGTTTTG
CTTTTTTGAAAGCCTTGCAATGTCCCGAAACCTGCTTGTCCGCTGGCTTGCCGTCTGCCT
CATCCCGTTGGCGACGCTTGCCGTTTTCGCCGCCAATCCGCCCGAAGACAAACTCCAGCA
TCTGATCAACGGCATCATCCTTGCCTGCGAAGCGACGTTTTTGTTTAAATTCGTCCTTTT
CGACACCATCAAGCATCATTTGAAACAAGAGTTTGATTTGAAACGTCAAACTATGTTGCT
GTTTATTCCGATTATTTTGCTGATTGTGTATTTGTTCCACTATTTTGGCGCGTTTTAGCC
CGTTTCCGTTATTTCTATGAATACTCCTCCTTTTGTCTGTTGGATTTTTTGCAAGGTCAT
CGACAATTTCGGCGACATCGGCGTTTCGTGGCGGCTCGCCCGTGTTTTGCACCGCGAACT
CGGTTGGCAGGTGCATTTGTGGACGGACGATGTGTCCGCCTTGCGTGCGCTTTGCCCTGA
TTTGCCCGATGTTCCCTGCGTTCATCAGGATATTCATGTCCGCACTTGGCATTCCGATGC
GGCAGATATTGATACCGCGCCTGTTCCCGATGTCGTCATCGAAACTTTTGCCTGCGACCT
GCCCGAAAATGTGCTGCACATTATCCGCCGACACAAGCCGCTTTGGCTGAATTGGGAATA
TTTGAGCGCGGAGGAAAGCAATGAAAGGCTGCATCTGATGCCTTCGCCGCAGGAGGGTGT
TCAAAAATATTTTTGGTTTATGGGTTTCAGCGAAAAAAGCGGCGGGTTGATACGCGAACG
TGATTACTGCGAAGCCGTCCGTTTCGATACTGAAGCCCTGCGAGAGCGGCTGATGCTGCC
CGAAAAAAACGCCTCCGAATGGCTGCTTTTCGGCTATCGGAGCGATGTTTGGGCAAAGTG
GCTGGAAATGTGGCGACAGGCAGGCAGCCCGATGACACTGTTGCTGGCGGGGACGCAAAT
CATCGACAGCCTCAAACAAAGCGGCGTTATTCCGCAAGATGCCCTGCAAAACGACGGCGA
TGTTTTTCAGACGGCATCCGTCCGCCTCGTCAAAATCCCTTTCGTGCCGCAACAGGACTT
CGACCAACTGCTGCACCTTGCCGACTGCGCCGTCATCCGCGGCGAAGACAGTTTCGTGCG
CGCCCAGCTTGCGGGCAAACCCTTCTTTTGGCACATCTACCCGCAAGACGAGAATGTCCA
TCTCGACAAACTCCACGCCTTTTGGGATAAGGCACACGGTTTCTACACGCCCGAAACCGT
GTCGGCACACCGCCGTCTTTCGGACGACCTCAACGGCGGAGAGGCTTTATCCGCAACACA
ACGCCTCGAATGTTGGCAAACCCTGCAACAACATCAAAACGGCTGGCGGCAAGGCGCGGA
GGATTGGAGCCGTTATCTTTTCGGGCAGCCGTCAGCTCCTGAAAAACTCGCTGCCTTTGT
TTCAAAGCATCAAAAAATACGCTAGAATAGCGCGTTTTACGACAACCGATTTGATTGGAA
AATCACAATGAAAACAGCACAAGAACTGCGCGCCGGCAATGTATTTATGGTCGGCAACGA
TCCTATGGTCGTTCAAAAAAACCGAATACATCAAAGGCGGCCGCTCTTCCGCCAAAGTCAG
CATGAAACTGAAAAACCTGCTGACCGGCGCGGCTTCCGAAACCATTTACAAAGCCGACGA
CAAATTCGACGTGGTCATCCTGTCCCGCAAAAACTGTACGTACAGCTACTTTGCCGACCC
GATGTACGTCTTTATGGACGAAGAATTCAACCAATACGAAATCGAAGCTGACAACATCGG
CGACGCGTTGAAATTCATCGTTGACGGTATGGAAGACCAATGCGAAGTAACCTTCTACGA
AGGCAACCCTATCTCCGTAGAACTGCCCACCATCATCGTGCGCGAAGTCGAGTACACCGA
GCCTGCCGTCAAAGGCGATACTTCCGGCAAAGTGATGAAAACCGCCCGCCTGGTCGGTGG
CACCGAAATCCAAGTGATGTCTTACATCGAAAACGGCGATAAAGTCGAAATCGACACCCG
CACCGGCGAATTCCGCAAACGCGCCTGATTTGCCGCATTGAAAAATGCCGTCTGAAAACG
TTTCAGACGGCATTTTTTTATATTCGCCCCGTGTTTGGATTGAAGTAGATGTTTTTTTC
GTAAACGACAATACGCGTGATTTTGCCATTTTCGTCAAAATGGATGTCTAAAAACGGTTT
GTCGGGATTGCGTTCACGGTTGGACAGCCGGAAGAGTGATTGGTTTTCAAGCATTTCTCC
GTGTATTTTCAGATAGCCGGGCAATTGGCTGATGTATTGGAAATTGCCGCCAAGTCCGTT
GTTGTGCCGGCTCGAATAGGCAGGGGTTGCCGAAACTTCAAAATAACGCGTGCGTTTCGG
CTCCTCGCCTTGTCCGCGGGTTTCTTGACTGCCCTGTATCAGCAACAGCGTCGCTTCTCG
GAGGCGGCGTTCCTGTTCCATCAAGGCATTTTGTGCCTGCCGGCTGATCGTGCGCTTTCC
GTAACGCAAGTCTTCAATTGCCAGTATGATTTTCTGATGGTAGTCGGGATCTTCAGGGCG
GATGTCGGGAAACAGCAGCCGCTGTATGTTGTCATAGCCACCGTCGGGTAAGCCGAAACG
GGTTTCCAGTTCGTGATGGGAAAGGGCAAACAGACAGTCCGAGTCAATGTGTTCGGCGAT
TTGCTGCATGAGATCGTCAATGCCGGTGGCGGGGTGCAGGCTGGTGCAATTTGACGGCGG
CGCGCTTTCTGCCTGCTTGACGTTATCAGGGGCGGATGCGGTCGGTTGCAAATCGGTTTG
GGAAGGAGCTGATGACGCGGAGGACGAAGCAGCGGATGCACCGACTTCTTTGGTTTTATC
GTCTTTGCTCGGAGAACACGCGGTCAGCATGATTGCGGTAAGCCATAAGAGAAGTGATGA
GGTTTTGTTTTTCATTCTATTGTTTCCAGTATTAAAGAGGCCGTCTGAAAACCTACCGTT
TCATTTTTCAGACGGCCTGTTGTTAATAGAACCGAAGAACCTGTTAATGCCGACAAGGTT
CTCAACCTGTCTTACCCGACGCGGTAAAACGCCAGGCTGCCCAAAAGGTTGGGGAAATGT

## Appendix A

TTGACCTGTCTGTTGCCCGTCATCACGGCGCGCTCGAGGATGCGGATGTTGTTTTTGGCG
CACAATAAATCAAAGTCTTTGAGCGTGCACCAATGGATATTGGGCGTGTCGTACCAATGG
TAGGGCATACGTTCGGAAACCGGCATATGTCCGCCGAGTGCGATTTGGACGCGGTTGCGC
CAGTAGCCGAAATTCGGGAAGCTGACAATCGCCTGTTTGGCAACGCGCATCAGGCAGCGC
AGGATTTTTTCGGTATTCTGCATCGCTTGGATGGTTTGGCTCAACACAATCACATCAAAA
CTTTGATCGTTGAATGCGGTTAAACCTTCTTCCAAATCGGCTTGGATAACATTTACGCCG
CGCGACATCGCGGCGATGACGCTATTTGTGTCGATTTCGATGCCGTAGCCGCTGCATTTT
TTGTGTTCGACCAATGCGGCAAGCAGTTCGCCGTCGCCGCAGCCCAAGTCCAAGACGCGG
CTGCCTTCGGGTATCCGGTCGTAAATCAGTTGCAAATCATCGCGCAGGTTCATTGCTGAC
ATTCCTTATAAACGTTGTTCATATAGGCGGCGACCGCACGCATATAGGCTTCGTCTTCCA
TTAAAAAGGCATCGTGCCCGTGTGCGGATTTGACTTCGATATACTGCACGGATTTTTGGG
CGGCAATCAGTGCCTTGACCAGTTCGTGCGAACGTTCGGGTGCGAAACGCCAGTCGGTGC
TGAAGCTGGCGACAAAGAATTTCGCTTTCACATTTTGCAGGGCGCGGGTCAGGCTGTCGC
CGAAATCTGCCGCCGGATCGAAATAGTCCAAAGCCTTGGTCATGAGCAGGTAAGTGTTGG
CGTCGAACCGTCCGACGAATTTGTCGCCCTGATAGCGAAGATAGGATTCCACTTCAAATT
CAACACCAAAGCCGTATTGATAACCGTTGGAACGCAAATCGCGTCCGAATTTTTTGCCTA
AACCGTCTTCGGCAAGATAAGTGATGTGTCCCATCATGCGGGCAATCCGCAAGCCCCGTG
CAGGAACGGTATTGTGGCTGCGGTAATGTCCTTCATTGAAATCAGGGTCGGTCAAAATTG
CCTGACGCGCCACATCGTTAAACGCGATATTTTGCGTGGACAGTTTCGGCGCAGACGCAA
TCACTAAAGCATGGCGCACGCGCTCGGGATAGGAAATCGTCCACTGCAAGGCCTGCATAC
CGCCCAAGCTGCCACCGACAATCGCCGCCCATTGTTCGATACCGAGATAGTCGGCAAGCG
CGGCTTGGGATTTTACCCAGTCCTTCACCGTAACCACCGGAAAATCCGCGCCGTATTCCC
TGCCCGTTTCAGGATTAATCGACAAAGGCCCGCTGCTGCCGTCGCAGCCGCCCAGATTAT
TCAAACCGACCACGAAAAAACGTTCCGTATCAATCGGTTTGCCAGGTCCGACCATATTGT
CCCACCAGCCCGTATATTTATCTTCCGCCGAATGCCTGCCCGCAACATGATGGTTGCCCG
ACAGCGCGTGGCAGATTAAAACCGCATTGTTTTTTCAGCATTCAGCTCGCCGTAGGTTT
CAATCATCAGATCGAAACGCGGCAAAGTTTACCGTTTTCCAAAACCAGCGGCATCTCAA
ACGGAATTTTTTGGGGCATTACAATGCCCACCGAGGCATTTTGACTCATATCCTGTTCCA
ACAAATGCGGCGAAAAGCGTTATTATATCGCAAACGGCATGACTTTTTGACACGGTCGGA
CAAGCAGCCGGACGCGTTTGACCCTCATCCGCCGCACACGAATCATACTTTTTCAGACGA
CCTCCACCGCTTCCCGACATGATAGGCAGACTTTTCCGTATTTTTTTCTTTTTCGCACTT
GCCGCGTTGATTATCAACCGCCTTTTCAGCCGCAGGCAAAAACGCGCCCTGCGCGAAGTC
GCCGAAATCAGCGCATGGGTACTGCTCGGTGCAGCCGCCGCGATGCTGTTTTGGTATCTG
TTTATGCTGTATTTCAAACACATTCCGGATTCGTATTGACGGAAAAAATGCCGTCTGAAA
CGCATTTTTCTGTTTCAGACGGCATATTTGATGAAAAGGGCTTGCGGTAGGAGGTGCTTT
ATAGTGGATTAACTTTAAACCAGTACGGCGTTGCCTCGCCTTGCCGTACTATTTGTACTG
TCTGCGGCTTCGTCGCCTTGTCCTGATTTTTGTTAATCCACTATACAACCGAAGCAGGAA
GGGCAGGGGGTCAGCGTTGGCGCGCTTTAAAACGCGGATTGCTTTTGCAGATGACGTAAA
CTTTGCCCCTGCGCCTGACGATTTGGCAGTCGCGGTGGCGTTGTTTGGCGGTTTTGAGTG
AAGACAGAACCTGCATTATTTGTCCTTTCTAAACGATGACATTACGGATTGGAAACGTTG
GTTGAATTTGCTGGCACGGCCTTCGGTGTTGACGTTGCGCTGTTTGCCAGTATAGACGGG
ATGGGATGCGGAAGAAGTATCCAGCGAAAACAGCGGATATTCTTTGCCGTCTGTCCAAAC
CATCGTTTTTCCGTGTGTTTCGGCACAGGAGCGGATTAACCAGCCTTCATTGGCGCTGCT
ATCGAAAAAAAGGACGGTTCGGTAATTGTCGGGATGAATATTCGGTTTCATATATTGCCT
TGCTTTCAGTGTTATAACATAACAAACTCTAGCATAGTTTAGAAGGGCTGTACAAGGAAA
TTTAACTATTTTTGTAATATATTAGAAATTTTCATGATAAATCTGAAAATTTTGAAATTG
ACTCATGTTTGGCGCAACTTTATTATGTTGCCTGAAACATCATATAAAAGATAATAAAAG
GTACGCAGCCATGAATTACGCAAAAGAAATCAATGCGTTAAATAACAGCCTTTCCGATTT
GAAAGGCGACATCAACGTTTCATTCGAATTTTTCCCGCCGAAAAACGAACAAATGGAAAC
CATGCTGTGGGATTCCATCCATCGCCTGCAAACCTTGCACCCGAAATTTGTTTCCGTAAC
TTACGGTGCAAACTCAGGCGAGCGCGACCGCACACACGGCATCGTCAAACGCATCAAACA
GGAAACCGGCTTGGAAGCCGCGCCTCACCTGACCGGTATCGACGCTTCTCCCGACGAATT
GCGCCAAATTGCCAAAGATTATTGGGACAGCGGCATCCGCCGCATTGTCGCCCTGCGCGG
AGACGAGCCGGCCGGTTATGAGAAAAAACCGTTTTACGCCGAAGACTTGGTTAAGCTATT
ACGCTCCGTCGCCGACTTCGACATCTCTGTAGCAGCATATCCCGAAGTGCATCCCGAAGC
GAAATCCGCACAAGCCGACCTGATTAATTTGAAACGCAAAATCGATGCGGGCGCGAACCA
CGTCATCACCCAATTCTTCTTCGATGTGGAACGCTACCTGCGCTTCCGCGACCGCTGCGT
GATGTTGGGTATCGATGTGGAAATCGTCCCCGGTATTTTGCCTGTTACCAACTTCAAGCA
GCTCGGTAAAATGGCTCAAGTAACCAACGTCAAAATCCCAAGCTGGCTGTCGCAAATGTA
TGAAGGTTTGGACGACGACCAAGGTACGCGCAATCTGGTGGCGGCAAGTATCGCCATCGA
TATGGTCAAAGTCCTGTCCCGCGAAGGCGTGAAAGATTTCCACTTCTATACGCTTAACCG
CAGCGAGCTGACTTACGCCATTTGCGATATTTTAGGCGTGCGCCCTTAAAGCCGTATCAA
ACAGTTTCAGACGGCATCTAAGGTGTCTAAAAAGCAAAACACCGCCCCATCCGAGCCATT
CTGATTTACAATACCGGCCGATTCGGATTGAACCGGTCCTTACAAAATCCAACTGGAGAG
TTCAACATGACAACATTACATTTCTCAGGCTTCCCGCGTGTCGGCGCCTTCCGCGAATTG
AAATTCGCACAAGAAAAATACTGGCGCAAAGAAATCAGCGAGCAAGAATTGCTGGCTGTT
GCTAAAGACTTGCGCGAGAAAAACTGGAAACACCAGGTCGCTGCCAACGCCGATTTCGTT
GCCGTAGGCGATTTCACTTTCTACGACCACATCCTCGACCTGCAAGTCGCCACCGGCGCG
ATTCCCGCCCGCTTCGGCTTCGACAGCCAAAACCTGTCTTTGGAACAATTCTTCCAACTG
GCGCGCGGTAACAAAGACCAATTCGCTATCGAAATGACCAAATGGTTCGACACCAACTAC
CACTACTTGGTGCCTGAATTCCACGCCGATACCGAATTCAAAGCCAATGCCAAACACTAT
GTTCAACAACTGCAAGAAGCCCAAGCCCTCGGTCTGAAAGCCAAACCGACCGTTGTAGGT
CCGTTGACTTTCCTGTGGGTGGGTAAAGAAAAAGGCGCCGTCGAATTCGACCGTCTGAGC
CTGTTGCCTAAACTGTTGCCTGTTTACGTTGAAATCCTGACTGCTTTGGTTGAAGCCGGT
GCCGAGTGGATTCAAATCGACGAGCCTGCTTTGGCTGTCGATTTGCCTAAAGAATGGGTG

## Appendix A

GAAGCCTACAAAGACGTTTACGCTACTTTGAGCAAAGTAAGTGCCAAAATCCTGTTGAGC
ACTTACTTCGGTTCTGTTGCCGAACACGCCGCATTGTTGAAAGCCCTGCCTGTTGACGGT
CTGCACATCGACTTGGTACGCGCCCCCGAGCAACTGGACGCGTTCGCCGACTACGACAAA
GTCCTGTCTGCCGGCGTGATTGACGGCCGCAACATTTGGCGCGCCAACCTGAACAAAGTT
TTGGAAACTGTCGAGCCTCTGCAAGCCAAACTGGGTGACCGTTTGTGGATTTCCAGCTCT
TGCTCGCTGCTGCACACTCCATTTGACTTGTCAGTTGAAGAAAAACTGAAAGCCAACAAA
CCCGACCTGTACTCTTGGTTGGCATTCACCCTGCAAAAAACCCAAGAATTGCGCGTTCTG
AAAGCTGCATTGAACGAAGGCCGTGATTCTGTTGCCGAAGAACTCGCCGCCAGCCAAGCT
GCTGCCGACTCCCGTGCCAACAGCAGCGAAATCCATCGTGCAGACGTTGCCAAACGCCTG
GCCGATTTGCCTGCCAACGCAGACCAACGCAAATCTCCATTTGCCGACCGTATCAAAGCG
CAACAAGCATGGTTGAACCTACCTCTGCTACCGACTACCAACATCGGTTCTTTCCCGCAA
ACCACCGAAATCCGCCAGGCACGCTCAGCCTTCAAAAAAGGCGAACTGTCTGCCGCCGAT
TACGAAGCCGCGATGAAAAAAGAAATCGCCTTGGTGGTTGAAGAGCAAGAAAAACTGGAC
TTGGACGTACTGGTACACGGCGAAGCCGAGCGTAACGACATGGTTGAATACTTCGGCGAA
TTGTTGAGCGGTTTTGCATTCACTCAATACGGCTGGGTACAAAGCTACGGCTCACGCTGC
GTGAAACCACCGATTATCTTTGGCGACGTAAGCCGTCCTGAAGCCATGACCGTGGCTTGG
TCTACTTACGCACAAAGCCTGACCAAACGCCCGATGAAAGGTATGTTGACCGGCCCTGTA
ACCATTCTGCAATGGTCTTTCGTCCGCAACGACATTCCTCGCTCTACCGTGTGCAAACAA
ATCGCACTGGCTCTGAACGACGAAGTATTGGATCTGGAAAAAGCCGGCATCAAAGTCATC
CAAATTGACGAACCTGCCATCCGCGAAGGCTTGCCGCTGAAACGCGCCGATTGGGATGCC
TACCTGAACTGGGCGGGCGAATCCTTCCGCCTGTCCTCTGCCGGTTGCGAAGACAGCACC
CAAATCCACACTCATATGTGTTACTCCGAGTTCAACGATATCCTGCCTGCGATTGCTGCA
ATGGATGCGGACGTGATCACCATCGAGACTTCACGTTCCGACATGGAACTCTTGACCGCG
TTCGGCGAATTCCAATACCCGAACGACATCGGCCCGGGGGTTTACGACATCCACAGCCCG
CGCGTACCGACAGAAGCCGAAGTGGAGCACCTGTTGCGCAAAGCCATCGAGGTTGTACCG
GTTGAACGTCTGTGGGTTAACCCGGACTGCGGCCTGAAAACACGCGGCTGGAAAGAAACT
CTGGAACAACTCCAAGTAATGATGAACGTAACCCGAAAACTGCGTGCCGAATTGGCGAAA
TAAGCCGAGACCGTATGAATAAATACCGTCTGAAAGCCTTTCAGACGGTATTTTGTCCTG
ATTTGCGGCGCAAGGGCGCAGTTGCCGGAAAATCTTTTCATTGCAGCTTGTTTTTTTCTA
ATTCGGCTTTATATGTGGGAAACAGGCAAATCGGAGTTGTGTTTGATAGTTTTAAATAAT
TTATATTATTTGAACTATAAATTATACAAATCATTTTGCATGGGGTAGAATGCCCAGCGA
TTCACAATTATTTCTCAAACCAATCTATTAAGGAGCTTAAAATGGCTTTGCAAGATCGTA
CCGGTCAAAAAGTACCTTCCGTAGTATTCCGCACCCGCGTCGGCGACACTTGGAAAGATG
TGTCTACCGATGATTTGTTCAAAGGCAAAAAAGTAGTCGTATTCTCCCTGCCCGGTGCAT
TTACCCCGACTTGTTCTTCTTCACACCTGCCGCGTTACAACGAATTGTTCGGCGCGTTCA
AAGAAAACGGCGTTGACGCAATCTACTGCGTATCTGTAAACGATACGTTCGTAATGAACG
CTTGGGCTGCCGAAGAAGAATCCGACAACATCTACATGATTCCTGACGGCAACGGCGAAT
TTACCGAAGGTATGGGTATGCTGGTCGGTAAAGAAGACTTGGGCTTCGGTAAACGCTCTT
GGCGTTACTCCATGCTGGTTAACGACGGCGTGGTTGAAAAAATGTTCATCGAACCTGAAG
AACCGGGCGATCCGTTCAAAGTATCCGATGCAGATACTATGCTGCAATTCGTTGCTCCCG
ATTGGAAGGCTCAAGAGTCTGTGGCAATTTTCACTAAACCAGGTTGCCAATTCTGCGCTA
AAGCCAAACAAGCTTTGCAAGACAAAGGTTTGTCTTACGAAGAAATCGTATTGGGCAAAG
ATGCAACCGTCACTTCCGTTCGCGCCATTACCGGCAAGATGACTGCCCCTCAAGTCTTCA
TCGGCGGTAAATACATCGGCGGCAGCGAAGATTTGGAAGCTTACTTGGCTAAAAACTGAT
AGCTGTTTGCTTAAGGCGGTTTAATTAAACTGTCTGATATACCGGATAGAGTTATTCGGG
CGGTTCTACACTACCGCTCCGAATAACTCTATATTTATAAGAGAATTTGGATATTGTTGC
ACTCAATCGAAATTTTGTTTTTATTTATCTGAATGATGTTTTTGATTGGGAAAATATTTA
AATGCCGTCTGAAACCGATATGTTCTGTGTCGGCAATGTTTCAGACGAAAACGGAAGGAC
AAAGATTATGAAAAAAATTCAAGCGGATGTCGTCGTAATCGGCGGCGGTACTGCCGGTAT
GGGTGCGTTTCGCAATGCCCGTTTACATTCGGATAATGTTTACCTGATTGAAAACAATGT
GTTCGGCACGACCTGCGCGCGCGTGGGCTGTATGCCTTCCAAACTCTTGATTGCCGCCGC
AGAGGCGCGTCATCACGCATTGCATACCGACCCGTTCGGCGTGCATTTGGACAAAGACAG
CATCGTCGTCAACGGTGAAGAGGTCATGCAGCGCGTTAAATCCGAGCGTGACCGTTTTGT
CGGCTTTGTCGTTGCCGATGTGGAAGAGTGGCCTGCCGACAAGCGCATTATGGGTTCGGC
TAAATTTATCGACGAGCATACCGTCCAAATCGACGAGCATACTCAAATTACGGCAAAAAG
TTTCGTGATTGCTACCGGTTCGCGTCCCGTCATCCTGCCGCAATGGCAGTCTTTGGGCAA
TCGTTTGATTATCAACGATGACGTTTTCTCATGGGATACGCTGCCTAAGCGCGTTGCCGT
GTTCGGGCCGGGTGTTATCGGTTTGGAACTGGGTCAGGCATTGCACCGTTTGGGCGTGAA
AGTTGAAATTTTCGGTTTGGGCGGAATCATCGGCGGCATTTCCGACCCCGTCGTTTCAGA
CGAGGCGAACGCCGTGTTCGGCGAAGAATTGAAACTGCATCTGGATGCTAAAACCGAGGT
CAAACTCGATGCAGACGGCAATGTAGAAGTCCATTGGGAGCAGGATGGCGAAAAAGGCGT
ATTTGTTGCCGAATATATGCTGGCAGCCGTGGGCCGCCGTCCGAACGTTGACAATATCGG
TTTGGAAAATATCAATATCGAAAAAGATGCGCGCGGCGTACCTGTTGCCGACCCGCTGAC
CATGCAGACCAGTATTCCGCATATCTTCATCGCAGGCGATGCGTCCAACCAACTGCCTCT
GCTGCATGAAGCTGCCGACCAAGGCAAGATTGCCGGCGATAACGCGGGCCGCTACCCGAA
TATCGGCGGCGGTTTGCGGCGCAGCACCATCGGCGTGGTGTTTACCAGTCCGCAAATCGG
CTTTGTCGGTCTGAAATACGCGCAGGTTGCCGCGCAATACCAAGCCGACGAATTTGTCAT
CGGCGAAGTATCGTTCAAAAACCAAGGCCGCAGCCGCGTGATGCTGGTGAACAAAGGCCA
TATGCGCCTGTATGCCGAAAAAGCCACCGGCCGCTTTATCGGCGCGGAAATCGTAGGCCC
TGCCGCCGAACATTTGGCGCACCTGTTGGCTTGGGCACATCAAATGAAGATGACCGTTCC
GCAAATGCTGGATATGCCGTTCTACCATCCCGTTATCGAGGAAGGTCTGCGTACCGCGTT
GCGCGATGCCGATGCGAAATTGAAAGCCTGACCGATATGGCAAAACAATGCCGTCTGAAA
TTTTTCAGACGGCATTTTGTTTTTGGGGATGGGGTCGGATGCTGATACCGTGTCGGGAA
GGGGGCGGCAAAACTAAAAATCTTTCTTTTAATCTGCTGTTTCCACGCGTGTTTGTCAAA
ATCTATCAGTTTGTTTTTAAAATACACTGTTCAAAATGGGATAAAACAGGTAAATTAACG

## Appendix A

TTTATGTAACCCAGTGTAGCAATGGGTTTACGGTTTTTGAGTCGATATATAACTACAGAG
GAATTGACTATGTCTGCCAAACCGCGTCCTGTTTATCTGGATTTGCCGAACATCCGTCTG
CCGATACCCGGGATAGTTTCCATCCTTCACCGCATCAGCGGGGTCGGGCTGTTTATTATG
CTGCCTTTCCTGCTGTATTTCCTGTCCGGTACCCTGAGTCAAGAGTCTGCATTTGAAACT
TACCGTGCCATTGTTTCCCATCCTTTGGTCAAGCTGGTTTTAATCGGTGTATTGTGGGCT
TATCTGCACCATTCTCTCGCCGGTATCCGCTTTTTATTTTTGGATGCGCACAAAGGCCTT
GAGCTGAATACTGCGCGCAATACCGCTAAAGCCGTATTTGCTTCTGCATTGGTTTTGACT
GTCGTTTTGGGAGCGTTGTTATGGTAGAACGTAAATTGACCGGTGCCCATTACGGTTTGC
GCGATTGGGTGATGCAACGTGCGACTGCGGTTATTATGTTGATTTATACCGTTGCACTTT
TAGTGGTTCTATTTTCCCTGCCTAAAGAATATTCGGCATGGCAGGCATTTTTTAGTCAAA
CTTGGGTAAAAGTATTTACCCAAGTGAGCTTCATCGCCGTATTCTTGCACGCTTGGGTGG
GTATCCGCGATTTGTGGATGGACTATATCAAACCCTTCGGCGTGCGTTTGTTTTTGCAGG
TTGCCACCATCGTTTGGCTGGTCGGCTGTCTCGTGTATTCAGTTAAAGTGATTTGGGGGT
AAGTATGGGTTTTCCTGTTCGCAAGTTTGATGCCGTGATTGTCGGCGGTGGTGGTGCAGG
TTTACGCGCAGCCCTCCAATTATCCAAATCCGGTCTGAATTGTGCCGTTTTGTCTAAAGT
GTTCCCGACCCGTTCGCATACCGTAGCGGCGCAGGGCGGTATTTCCGCCTCTCTGGGTAA
TGTGCAGGAAGACCGTTGGGACTGGCACATGTACGATACCGTGAAAGGTTCCGACTGGTT
GGGCGACCAAGATGCGATTGAGTTTATGTGCCGCGCCGCGCCTGAAGCCGTAATTGAGTT
GGAACACATGGGTATGCCTTTTGACCGTGTGGAAAGCGGTAAAATTTATCAGCGTCCTTT
CGGCGGCCATACTGCCGAACACGGTAAACGCGCGGTAGAACGCGCCTGTGCGGTTGCCGA
CCGTACAGGTCATGCGATGCTGCATACTTTGTACCAACAAAACGTCCGTGCCAATACGCA
ATTCTTTGTGGAATGGACGGCACAAGATTTGATTCGTGATGAAAACGGCGATGTCGTCGG
CGTAACCGCCATGGAAATGGAAACCGGCGAAGTTTATATTTTCCACGCTAAAGCTGTGAT
GTTTGCTACCGGCGGCGGCGGTCGTATTTATGCGTCTTCTACCAATGCCTATATGAATAC
CGGCGATGGTTTGGGTATTTGTGCGCGTGCAGGTATCCCGTTGGAAGACATGGAATTCTG
GCAATTCCACCCGACCGGCGTGGCGGGTGCGGGCGTGTTGATTACCGAAGGCGTACGCGG
CGAGGGCGGTATTCTGTTGAATGCCGACGGCGAACGCTTTATGGAACGCTATGCGCCGAC
CGTAAAAGACTTGGCTTCTCGCGACGTTGTTTCCCGCGCGATGGCGATGGAAATCTACGA
AGGTCGCGGCTGCGGTAAAAACAAAGACCATGTCTTACTGAAAATCGACCATATCGGCGC
AGAAAAAATTATGGAAAAACTGCCGGGCATCCGCGAGATTTCCATTCAGTTCGCCGGTAT
CGATCCGATTAAAGACCCGATTCCCGTTGTGCCGACTACCCACTATATGATGGGCGGCAT
TCCGACCAATTACCACGGCGAAGTTGTCGTTCCGCAAGGTGAAGATTACGAAGTGCCTGT
AAAAGGTCTGTATGCGGCAGGTGAGTGCGCTTGTGCTTCCGTACACGGTGCGAACCGCTT
GGGTACCAACTCCCTGTTGGACTTGGTGGTATTCGGTAAAGCTGCCGGCGACAGCATGAT
TAAATTCATCAAAGAGCAAAGCGACTGGAAACCTTTGCCTGCTAATGCAGGTGAGTTGAC
CCGCCAACGTATCGAGCGTTTGGACAACCAAACCGATGGTGAAAACGTTGATGCATTGCG
TCGCGAACTGCAACGCTCTGTACAACTGCACGCCGGCGTGTTCCGTACTGATGAGATTCT
GAGCAAAGGCGTTCGAGAAGTCATGGCGATTGCCGAGCGTGTGAAACGTACCGAAATCAA
AGACAAGAGCAAAGTGTGGAATACCGCGCGTATCGAGGCTTTGGAATTGGATAACCTGAT
TGAAGTGGCGAAAGCGACTTTGGTGTCTGCCGAAGCACGTAAAGAATCACGCGGTGCGCA
CGCTTCAGACGACCATCCTGAGCGCGATGATGAAAACTGGATGAAACATACGCTGTACCA
TTCAGATATCAATACCTTGTCCTACAAACCGGTGCACACCAAGCCTTTGAGCGTGGAATA
CATCAAACCGGCCAAGCGCGTTTATTGATGCGTTTTCAGACAGTCTTCGCCTCAAAGGTC
GTCTGAAATCTAACCATACCCACATTGAACTGCTTGAATTTATAATACAAAATCATTGGG
CAGTTGATGAGAAAAGGAACACTTCTCATGGAAAAAATGAGTTTTGAAATTTACCGTTAC
AACCCGGATGTTGATGCCAAGCCTTATATGCAGCGTTACGAGTTGGAATTGGAACCGACC
GACGTGAAACTTTTGGATGCTTTGGTACGCCTGAAAGCACAAGACGATACCTTGTCTTTC
CGCCGCTCCTGCCGCGAAGGCATTTGCGGATCGGACGGTATGAACATCAACGGCAAAAAC
GGCTTGGCGTGTTTGACCGATCTGCGTGGCTTGAAACAGCCAGTTAAAATCCGTCCTCTG
CCAGGTCTGCCTGTTATCCGCGACCTGATTGTGGATATGACCCAGTTCTTCAAACAATAC
CATTCCGTCAAACCTTATGTTGTCAACGATAATCCGATTGATGCGGACAAAGAGCGTCTG
CAAACTCAGGAAGAGCGTAAAGAGTTGGACGGTTTGTACGAGTGTATTTTGTGCGCCTGC
TGTTCGACTGCCTGCCCGTCATTTTGGTGGAACCCTGATAAATTCGTCGGTCCGTCCGGT
TTGCTGAATGCTTACCGTTTCATTGCGGACAGCCGTGATACCATCACTAATGAGCGTTTG
GATAATCTGAACGACCCATACCGTTTGTTCCGTTGCCACACCATTATGAACTGCGTAGAC
GTATGTCCTAAACACTTGAATCCGACCCGAGCCATCGGTAAGATTAAAGAGATTATGTTG
AAACGGGCCGTTTAAGAAATGATGGTTTTTGACGATATTGCCAAACGGAAAATCCGTTTT
CAAACCCGCCGGGGATTGTTGGAATTAGATTTAATCTTCGGCAGGTTTATGGAAAAAGAA
TTCGAGCATTTGAGCGATAAAGAGCTGTCCGAGTTTTCCGAAATCCTTGAATTTCAAGAT
CAAGAATTGCTTGCCTTGATTAACGGGCATTCGGAAACGGACAAAGGGCACCTTATCCCG
ATGCTTGAAAAAATCAGACGGGCATGAGGCATCTGAAATCTGCCTGCAGGCAGATTTCAA
AATGCAAAAGCCGTCTGAAGGCAAAGAACGTGCTGCGGATGCAGTAACGTGGGTTATAAC
TTGCAAAGGAGCAATAATATGTCCAAATCAATCAAACTCAACGTACCGGGTCAGGCAGGT
TTGGAGCTGCCGGTATTGGAAGCCAGCATCGGGCACGATGTGGTTGACATTCGGGGGCTG
ACAAAAAATACAGGTTTGTTTTCCTTCGACCCCGGATTTGTTTCAACCGCAAGCTGTGAG
TCTAAAATTACTTACATCGACGGCGATCAAGGCTTGCTTTATTATCGCGGATACCCCATC
GAGCAGCTGGCCGAAAAGTCCGATTATTTGGAAGTCTGCTACCTGTTGATTTACGGCGAA
CTGCCGACTCCCGAGCAAAAGGCAGAATTTGACAATACCGTCCGCCGCCACACGATGGTG
CATGAACAGCTGACTTGGTTCTTCCGGGGGTTCCGCCGCGACGCGCATCCGATGGCGATG
ATGGTCGGCGTGGTCGGCGCACTGTCTGCGTTCTACCAAGACAGCTTGGACATTAGCAAT
CCCGAACACCGCAAAATCGCGATTTACCGCCTGATTTCTAAAATCCCGACCATTGCGGCA
ATGTGCTACCGCTATTCAAACGGTCTGCCGTTCAATTATCCGAAGAATAATCTTTCTTAT
TCCGAAAACTTCCTTCATATGATGTTCGCCACGCCGTGTGAAGACTACAAACCCAATCCC
GTTTTGGCACGCGCGCTCGACCGCATCTTTATTTTGCATGCCGACCACGAGCAAAACGCC
TCAACTTCAACCGTCCGTCTGGCAGGGTCTTCGGGTGCGAACCCGTTTGCCTGTATTGCT

# Appendix A

GCCGGTATCGCCTGCCTGTGGGGTGCTTCGCACGGCGGTGCGAACGAAGCCGTGTTGAAA
ATGTTGGACGAAATCGGCGATGTGTCTAATGTTGCCGCATACATGGAAGGTGTGAAACAA
CGCAAATACCGTCTGATGGGCTTCGGTCACCGCGTGTACCGCAATATGGATCCGCGTGCC
AGCATTATGCGCGAAACCTGCTATGAAGTTTTGAAGGAATTGGGCTTGGAAGACAGTCCG
AAATTCAAACTGGCGATGGAATTGGAACAGATTGCGCTGAAAGACCCGTTCTTTATCGAA
CGCAAACTGTATCCAAACGTCGATTTCTATTCCGGCATCGTCCTGTCCGCGCTGGGCATC
CCGACCGAAATGTTTACCGTCATCTTCGCCCTGTCGCGCAGCGTGGGCTGGATTTCGCAC
TGGCACGAGATGATTAGCGATCCTTCGCTGAAAATCGGCCGCCCGCGCCAGCTTTATACC
GGTTCGGAACGCCGCGATTATGTGCCGCCAGGCGAGAGGTAACATACATTAATATATTGT
CAAACAGGCAATATCAGAGAACCGGATTGTTTCCCGAATCCGTCTGATTGTAGTCGGATG
AAATCAAGACAAGCAATCCGGTTTAAAATAGGGTAGAATAAAATGTCTTTTCAGGCGGCA
TCAGTTTAGCCGTCAGGACGCGGACTTCTACCCTTTGTTTATATTTTAAAGAAAAGAGCG
CACGCCATGATGGACGAAAAACTCAATTTCTCTTACCTGTTCGGTTCAAACGCACCTTAC
ATTGAGGAATTGTACGAGGCTTTTTTGGAAAACCCCGATGCGGTTGATGAAAAATGGAAG
CAGTATTTCACCGATTTGAGCAAACAGCCGGGGACGGTTGCTGTCGATGTCGCACACACA
CCGATTCGCGAATCATTTGTTACTTTGGCGAAAAAGAAAATTGCATCTGCCGTTGCGGGC
GGTGCGGATGAGGCAATGCTGAAAAAGCAAGTCAGCGTTTTACGGCTGATTTCCGCCTAT
CGTATCCAAGGCGTGGGTGCAGCCCAACTTGATCCGCTCAAACGTATCCCCCCGCGCGAT
ATTGAAGCCCTCGATCCGAAATTCCACGGTCTGTCAGATGCCGATATGGCGCTTCAATTC
AATATGGGCGAGGGCGATTTTGCCAATCGCGGCAAACTGCCTTTGTCCCAAATCATCAGC
AACCTCAAACAAACCTACTGCGGCCACATCGCATTGGAATATATCTATATTCCCAATACC
GAAGAGCGCCGCTGGGTACGCAATTATTTTGAAAGCGTATTGTCCACACCGCATTACAAT
GCCGATCAAAAACGCCGTATCTTGAAAGAGATGACTGCTGCCGAGACTTTGGAACGTTAT
CTGCATACCAAATATGTCGGTCAGAAACGTTTCGGTGTCGAAGGCGGCGAAAGCGCGATT
GCCGGTTTGAACTACCTGATTCAAAACGCCGGTAAAGACGGTGTGGAAGAGGTCATCATC
GGTATGGCGCACCGTGGCCGTCTGAATGTTTTGGTGAACATTTTGGGCAAAAAACCCGGC
GATTTGTTTGCCGAATTTGAAGGTCGTGCCGAAATCAAACTGCCCAGCGGCGACGTGAAA
TACCATATGGGCTTCAGCTCCGATATTGCCACGCCGCACGGCCCGATGCACGTTTCTTTG
GCGTTCAACCCGTCACACTTGGAAATCGTCAACCCGGTGGTGGAAGGTTCTGCGCGCGCC
AAACAAAAACGTTTGGGCGAAAACGGCCGCGACAAAGTCTTGCCGGTATTGATTCACGGC
GACTCCGCATTTATCGGTCTGGGAGTCAACCAAGCGACATTCAACCTGTCTAAAACGCGC
GGTTATACCACCGGCGGTACGGTTCATATCGTCATCAACAACCAAATCGGCTTTACCACT
TCCGATATCCGCGATACCCGTTCAACCGTACACTGTACCGATATCGCAAAAATGGTTTCC
GCCCCGGTTATCCATGTGAACGGCGATGATCCCGAACGCGTTTGCTTTGCTATCCAAGCC
GCTTTGGATTACCGCAAAAAATTCCATAAAGACATCGTGATTGACGTTGTCTGCTACCGT
AAATGGGGTCACAACGAGGGCGATGATCCGACCTTGACCCAACCGATGATGTACAAAAAA
GTATCGCAACACCCCGGTGCGCGTGCTTTGTACACCGAGCAACTGATTGCCGAAGGCGTG
GTAACCCAAGCCGAGGCTGACGGTTACATCCAAGCTTACCGTGATGCTTTGGACAAAGGC
GAGCACGTCGAGCAAACAACGTTGAGCAACTTCCAACGCACGCAAATCGACTGGAGCAAA
TACCAAGGCAAAGATTGGCGCGAACACATCGAAACCGGTTTGCCTGCCGCAGATATTGAA
CGTCTCACTGAGAAGTTTACCGCCGTACCGGAAGGCTTTGCCCTGCATCCGACTGCAAAA
CGTGTGATTGAAGCGCGTAAAGCCATGGCATCCGGCAAACAGGCCATAGATTGGGGTATG
GCCGAAACCTTGGCATACGCCAGCCTCGTAACCAAAGGTCACGGCGTGCGTATTTCCGGC
GAGGACTCGGGACGCGGCACGTTCTCGCACCGCCACGCCGTATTGCACGATCAAAAACGC
GAAAAATGGGACGACGGTACTTATGTTCCTCTGCGCCATATGGGCGAAGGCATGGGCGAG
TTCCTGGTTATCGACTCCATTTTGAACGAAGAAGCCGTGATGGCGTTCGAGTACGGCTTT
GCCTGCTCCGCACCTGACAAACTGACCATTTGGGAAGCTCAATTCGGTGACTTCGCCAAC
GGCGCGCAAGTGACTATTGACCAATTCCTGTCTTCAGGCGAAACCAAGTGGGGTCGTTTG
TGCGGTCTGACTACCATCCTGCCGCACGGCTACGACGGTCAAGGCCCCGAGCACTCTTCT
GCACGCGTAGAACGTTGGTTGCAACTGTGTTCTGAGAACAATATGCAAGTCATTATGCCG
TCTGAAGCGTCGCAAATGTTCCACCTCTTGCAACGTCAAGTCTTGGGTTCATACCGCAAA
CCGCTGGTGATTTTCATGTCCAAACGCCTGTTGCGCTTCAAAGGTGCAATGAGCCCGCTG
GAAAACTTCACCGAAGGTTCGACCTTCCGTCCGGTTATCGGCGATACCGCAGAACGCGCA
AGCAACGACAGCGTGAAACGCGTGGTATTGTGTGCCGGTCAGGTTTACTATGACTTGGAA
GCCGGCCGTGCCGAGCGTAAACTGGAAGATGATGTTGCTATTGTCCGCGTTGAGCAGCTG
TATCCGTTCCCATATGACGAGGTTAAAGCTGAACTGGCGAAATATCCGAACGCAAAATCT
GTGGTTTGGGCACAAGAAGAGCCGAAAAACCAAGGCGCGTTCTACCAAATCCGCCACCGC
ATCGAAGATGTTATTAGCGAAGAGCAAAAACTGTCTTATGCCGGTCGTCCAAGCAGCGCA
TCGCCTGCAGTGGGCTACTCAAGCAAACACATTGCTCAATTGAAACAATTGGTTGAAGAC
GCTTTGGCATTGTAAACCAAGTAGCATTCCGTCTGAGTCTGCTCAGATGGAATGCCCATA
TGCAGAATTAAAAACACACAACAGGCCGTCTGAAAGGGCCATTGGAGACACAAAATGATT
ATTGATGTAAAAGTACCTATGTTGTCTGAAAGCGTATCTGAAGGCACGCTCTTGGAATGG
AAGAAAAAAGTTGGCGAAGCCGTTGCCCGTGACGAAATCCTGATCGATATCGAAACGGAC
AAAGTGGTTTTGGAAGTACCTTCTCCACAAGCCGGCGTATTGGTTGAAATCGTAGCTCAA
GACGGTGAAACCGTTGTTGCCGACCAAGTTTTGGCGCGCGTCGATACAGCTGCTACTGCC
GCTGCTGAAGCCCCAGCCGCCGCTCCTGCAGAAGCTGCCCCAGCTGCCGCTCCTGCTGCT
ACACAAAACAACGCCGCTATGCCTGCTGCCGCCAAACTGGCTGCCGAGACCGGTGTTGAC
GTGAACGCATTGCAAGGTTCCGGCCGTGACGGTCGCGTATTGAAAGAAGACGTACAAAAT
GCCGCTGCCAAACCTGCCGGAGCCGCTGCTCCTGCTGTTGCACTTCCTGCCGGCGCACGT
CCTGAAGAACGCGTACCAATGAGCCGCCTGCGTGCCCGTGTTGCAGAACGCCTCTTGGCT
TCTCAACAAGAAAACGCCATTCTGACTACATTCAACGAAGTCAACATGAAACCAATCATG
GACTTGCGTGCGAAGTACAAAGAAAAATTCGAGAAAGAACACGGCGTGAAACTGGGCTTT
ATGTCCTTCTTCGTTAAAGCCGCTGTTGCCGCCCTGAAAAAATACCCGGTTGTGAATGCT
TCTGTTGACGGCAAAGACATCGTGTACCACGGCTACTTCGACATCGGTATCGCAATTGGC
AGCCCACGCGGTTTGGTTGTGCCAATTCTGCGTGATGCCGACCAAATGAGCATTGCCGAC

## Appendix A

ATCGAACAAGCAATTGTTGATTACGCGAAAAAAGCCAAAGACGGCAAAATCGCTATCGAA
GATCTGACCGGCGGTACATTCAGTATTACCAACGGCGGTACTTTCGGTTCTATGATGTCT
ACCCCGATCATCAACCCACCTCAATCTGCGATTTTGGGTATGCACGCCACTAAAGAGCGC
GCTGTGGTTGAAAACGGCCAAGTTGTTGTCCGTCCGATGATGTATCTGGCTCTGTCTTAC
GACCACCGTATCATTGACGGCCGCGAAGCTGTATTGACCTTGGTAGCCATTAAAGACGCG
TTGGAAGACCCGGCCCGCCTGTTGTTGGATCTGTAATCGTTTCAGACGGCCTTTTATTTG
TTAATGAAAAGGCCGTCTGAATTTTTATAGTGGATTAAATTTAAACCAGTACGGCGTTGC
CTCGCCTTGCCGTACTATCTGTACTGTCTGCGGCTTCGTCGCCTTGTCCTGATTTAAATT
TAATCCACTATATTTAGATGTAGCGTAATGTAGTATCGTGCTACAATAGGCTCAACGAAC
GATTGAGGCCGTCTGAAACATTTGATTCGAATGAATCGGCAGATATGGACTTTCAGACGG
CCTTTTCTTAAAACCATCAAAACGCAGTCATTCAAAATAAAAAGAAACAAAAAGTATCG
TTTTTATTTTGAGATACTGTTAAAAGCAAAGGATGACACGATGTCTCAATATGATGTAGT
AGTGATTGGTGCAGGCCCGGGTGGATACGTTGCCGCCATCCGTGCCGCGCAACTGGGTTT
CAAAACCGCTTGCGTCGATGCAGGCGTTAACAAAGCAGGCAATGCCCCTGCATTGGGCGG
TACTTGCTTGAACGTAGGCTGTATCCCTTCTAAAGCCCTGTTGCAATCCAGCGAACATTT
CCACGCTGCGCAACACGAGTTTGCCGAACACGGTATCACTGTCGGCGACGTAAAATTCGA
CGCGGCCAAAATGATTGAGCGCAAAGATGCCATCGTGACCAAGCTGACCGGCGGCGTCAA
ATTCCTGTTCCAAAAAAATAAAGTAACCAGCCTGTTCGGTACGGCTTCCTTTGCCGGTAA
AAATGGCGATGCTTACCAAATCGAAGTCGATAACAAAGGCGAGAAAACCGTTATCGAAGC
CAAACACGTCATCGTAGCCACCGGTTCCGTACCGCGTCCGCTGCCACAAGTCGCTATCGA
CAATGTGAACGTATTGGACAACGAAGGTGCATTGAACCTGACCGAAGTACCTGCCAAACT
CGGCGTGATCGGTTCCGGCGTGATTGGTTTGGAAATGGGTTCCGTATGGAACCGCGTGGG
TGCGGAAGTTACCATTCTTGAAGCCGCGCCGACTTTCCTGGCTGCCGCCGACCAACAAAT
CGCCAAAGAAGCCTTCAAATACTTCACCAAAGAGCAAGGTCTGAGCATCGAATTGGGCGT
GAAAATCGGCGACATCAAGTCTGAAGGCAAAGGTGTTTCCGTTGCTTACGAAACTGCTGC
TGGCGAAGCCAAAACCGAAGTATTCGACAAACTGATCGTTGCCATCGGCCGTATTCCAAA
CACCAAAGGCCTGAACGCGGAAGCCGTAGGCTTGGAAAAAGACGAGCGCGGCTTTATCAA
AGTAGATGGCGAATGCCGTACCAACCTGCCTAACGTATGGGCAATCGGCGACGTGGTTCG
CGGCCCGATGTTGGCACACAAAGCCAGCGACGAAGGCGTTGCCGTTGCCGAACGCATTGC
CGGTCAAAAACCGCATATCGACTTCAACAACGTACCGTTCGTGATTTACACCGATCCTGA
AATCGCTTGGGTGGGTAAAACCGAAGAGCAGCTCAAAGCCGAAGGCGTGGAGTACAAAAA
AGGTACTTCAGGTTTTGGTGCGAATGGTCGCGCATTGGCAATGGGCAAAGCCAAAGGTAC
GGTTAAAGTGTTGGCAGATGCCAAAACCGACCGCATCTTGGGCGTACACATGATTGGTCC
GGTTGTCAGCGAATTGGTTACCGAAGGCGTGACTGCGCTCGAATTCTTCGCCAGCAGCGA
AGACATCGCCCGCATTATCCATGCCCACCCAACCTTGTCCGAAGTGGTTCACGAAGCTGC
ATTGGCGGCCGACAAACGCGCTTTGCACGGTTGATAGACATTAAGGCCGTCTGAAATTTT
TCAGACGGCCTTAAGGCCTTCGACAAATTGAATGTTCCGAGAGCTCCGTTTTCTGATTTA
TAATTCCGTCAGACAAACAAACAGCATTTACATTCATTATGAACAAAGAAATAGTCGGTA
TTTTCTTTATACCGGCGGGCATCATCAGCATGTGTATGGCCGCATTGTGGCAGATGTATG
TGATGATGACCGAAACTTATACGCTCAACCGTTTCAAAGATAAAGAATTGGTTTGGCGCG
TGGCATTGTTGTTTATCAGTTTCAGCCTTGCCGTTTATCTGCTCTGTCCGAATTCGCGTA
AAAAAGGCATCGTCTTTTTTATTCTCGGGGGAGGCGGTGCAGCCATGTATCTGCTGGCGC
GGATGTGGTTGCCTTTCAGCAAGTGAAACGACGATTTTCCGACCGCCGAAAGGTAGTCTG
AAACGCACGGGCTTGCCATTTGGAGGCAGACTCGGGGCATTCCACTAATCTAAAGGAGAA
ATCCATGAATTTACACGAGTATCAGGCTAAAGAACTGCTGGCTAGCTACGGTTTGCCCGT
ACAAGGCGGTATTTTGGCACACAACGGCGAAGAAGCCGCTGCAGCTTACGACAAATTGGG
CGGCAAATTCGCTGTTGTCAAAGCACAAGTACACGCCGGCGGCCGCGGTAAAGCGGGCGG
CGTAAAAGTCGTTAAAAGCCGCGAAGAAGCTAAAGAAGTGGCTGAAAGCCTGATTGGCAC
CAACTTGGTAACTTACCAAACCGATGCCAACGGCCAACCTGTCAACAGTGTTTTGGTTTG
CGAAGACATGTATCCGGTTCAAACCGAGCTGTACTTGGGCGCAGTGGTTGACCGTTCTAC
CCGCCGCATTACATTCATGGCCTCTACCGAAGGCGGCGTGGAAATCGAAAAAGTTGCTGC
CGAAACTCCTGAAAAAATCTTCAAAGTAACCGTTGATCCGCTGGTCGGCCTGCAACCTTG
CCAAGCTCGCGAAGTTGCGTTCCAACTGGGCTTGAAAGACAAACAAATCAACGAGTTCGT
CAAACTGATGACCGGTGCGTACAAAGCGTTTGTCGAAAATGACTTCGCCCTGTTTGAAGT
CAACCCGCTGGCAGTTCGCGAAAACGGCGCGCTCGCCTGCGTGGACGGCAAAATCGGCAT
CGACAGCAACGCGCTCTACCGCCTGCCGAAAATCGCCGAATTGCGCGACAAATCTCAAGA
AAACGAACGCGAGTTGAAAGCTTCTGAATTTGACCTGAACTATGTTGCCCTGGAAGGCAA
CATCGGCTGTATGGTGAACGGTGCCGGTTTGGCGATGGCCACTATGGACATCATCAAACT
GAAAGGCGGCCAACCTGCCAACTTCTTGGACGTTGGCGGCGGCGCAACCAAAGACCGCGT
GGTTGAAGCGTTCAAACTGATTCTGGAAGACAAATCCGTTCAAGGCGTATTGATCAACAT
CTTCGGCGGTATCGTACGTTGCGACATGATTGCGGAAGCCATCGTGGCAGCCGTTAAAGA
AATCAACGTCAACGTTCCTGTCGTTGTTCGTTTGGAAGGCAACAACGCCGAACTCGGCGC
GAAAATCCTGAACGAATCAGGTCTGAAACTGACTTCTGCAGACGGCCTGAATGACGCAGC
CGAAAAAATTGTTGCAGCCGTAAACGCCTAAGGAGAAAAGAATGAGCGTATTGATTAATA
AAGACACTAAAGTATTGGTTCAAGGTTTCACCGGTAAAAACGGTACTTTCCACTCCGAAC
AAGCTCTGGCTTACGGCACTAAAGTTGTCGGCGGCGTTACCCCGGGCAAAGGCGGTCAAA
CCCACCTGAACCTGCCCGTGTTCAACACCATGAAAGAAGCCGTTAAAGAAACCGGCGCGG
ATGCATCCGTGATTTACGTTCCTGCTCCGTTTGTGTTGGATTCTATCGTTGAAGCAGTTG
ATTCAGGCGTAGGCTTGGTCGTTGTGATTACCGAAGGCGTGCCGACTTTGGACATGCTCA
AAGCCAAACGCTACTTGGAAACCAACGGTAACGGAACACGTTTGGTCGGCCCTAACTGCC
CGGGCGTGATTACTCCGGGCGAGTGCAAAATCGGCATTATGCCGGGCCACATCCATACTC
CCGGCCGCATCGGCATCATTTCCCGTTCCGGTACATTGACTTACGAAGCCGTGGCACAAA
CCACCAAACTGGGCTTGGGTCAATCAACCTGTATCGGTATCGGCGGCGACCCGATTCCGG
GTATGAACCAAATCGACGCACTGAAACTTTTCCAAGAAGACCCGGATACCGACGCCATCA
TCATGATCGGTGAAATTGGCGGTACTGCGGAAGAAGAAGCAGCCGAATACATCCAATCCA

## Appendix A

ACGTAAGCAAACCTGTTGTCGGCTATATCGCCGGTGTTACCGCACCTAAAGGCAAACGCA
TGGGTCACGCCGGTGCGATTATCTCCGGCGGCAAAGGTACTGCGGAAGAAAAATTCGCCG
CTTTCGAAAAAGCCGGTATCGCTTACACCCGCAGCCCTGCCGAGTTGGGCACTACCATGC
TGGAAGTGTTGAAAGCAAAAGGTTTGGCATAATCAGGTTTGACAACTGATTGAACATCAA
ATGCCGTCTGAAACCGGAAATCGGGTTTCAGACGGCATTTTGTTTGTCATTTCAAAAAGA
GGCAGCCTCAACATACCCACATTATTTTTGCCCTTTTGGGGCAGTCAGAGACCTTTGCAA
AATTCCCCAAAATCCCCTAAATTCCCTAAATTCCCACCAAGACATTTAGGGGATTTTGGG
GAATTTTGCAAAGGTCTCGGGCTAAGTGTGCCTGTTTGCGCCTAAAAGGCGGCCCGGATG
CCTGATTATCGGGTATCCTGGGAGGATTAAGGGGGTATTGGGGTAAAATTAGTGGATATT
TGAAACGAAAACAGCCGAAAACCTGTGTTTGGGTTTCGGCTGTCGGGAGGGAAAGGAATT
TTGCAAAGCTCTCGTATTGGCTTTGAAGTTCCGTGTAATTCACAGGTAGGGCGTGTGGCA
CAGCCACGCACGCGGTCGGTTGGGTATGCAGGCTACGGCTTTCTCTGTTGAAACTGCAAC
ACGTCAGCTACCAACCGTGTGCGTAGCGCACACGGTTGGTTGGGATACAAGTTACTGTTT
GTTCTTAAATGGAGTACCAACATCAAGGGCTTTTGATAATCCTGAAAATATTAAATATTC
AGTTTCAGTTTTTATTTTAGGAGAAATATTTGCATAATTTCTATCTTTAAAGCACCAATG
GATATATGGTTTCGTTTCATCTTCTGGGTTATCTAAATAAGCAATAACATTCAAGTCAAA
TAAAAATTGCAAAAACTCATTAGCAGTACTCATAAATTTAGGTATTTCCACTGATGTTGT
TTGTAAGTGCTTTTTCAAACGTTCAAATGCTTTTAAAAAATCACTATATTTAAATCTATC
TTTCCCGTTTAAAAATTCAAAAAATTTCAGGAAATTTTGATAATCACTTTGACTATAATA
AAACAAAAGATGATCTTTGATTTCACCAAGTAAATATATCGAGTATTCTCTTTGAAAAGA
AGTATTATCAAAATCTTCTGCTACGACATAATCTTCCTTACTTTTCTTATTTTTTTGTAG
CAAAGTAAGCATCTGAAGAATATCGCGAGGTCGATAATACGATTTTCTTAGGAAGCTAAT
AAATGAAGTTAAATTTTTATACTCATCATGTAAATTAGGAGCATTCCATGGAAAATAATA
ATCCCATGAGTTGCCTTTTTCTAAACTATCTTGTTTTTCTTGCTGGGTTCTCAAAAGATG
ATCAAAAACGCCAAAAATCTTTGAACTTCTATAAGATTTATAATCCGTCCTCCAGTCTAA
AAATACTGAATTATCTTGAAGTTTGGTATTTTGATTTTGTAAACCTAATGAATCAAAGAT
ATCAGGTCTAATCAATAACACAACTCTCATCCTTCCCTTACTATCTTTAATGGAAGGGAA
GATATCATTATTTAACATCCATATGGCGTTAGCAAGACCTTTTACACACTCATGATATTC
ATCAAATGGAATCTGTGATGGTCTAATATCTATCCCATCAATAAACAAAATATGATTATC
TTTTAGCTTTAACTGAGATAAAGCATCTTTAAATTTTCTTTCAATAAAACCTAAATTTGC
TTGGAATTTACTTTCTGTAAAAGTTATTTGTTGGGATTCCTCTTCACCTAGTTTAACAAA
TTTTCCAAAAATCATTTCCGCAGCTTCTTTTGAATTTTCTATTAAAGTTATTGCTTGTAC
AATTTCCGGATCAAAAGCGCCATAATAATATTCATTTATAGCCTCATCTAAGGCTTTAAA
TTTATTAAATATTGAAGATAATATTCCGTTTTCTTTACATTTGATTTGATTTGATATCAA
CAGATATAAAATGACTTTCCAAATACTTGTAAAATCTGAAACAGTTAAGTGTCTTGCTTT
CTTTAGCTGAATAAATTTTGAATAATCGGTTTCACGAACAAACTTAGTAGTGGCATGTAT
GTTTTTATAGAAGTTATTAGTTAAATAAACAGCATATGCTGTCTTTCCAGTTCCCTTTTC
TCCGATTAAAAACGAAATATTTGGTTCACATAATTCATCCAAATATTCTCCTTTTACAAA
TATTCGGTTAAATAAATCTTTATTTTCTCTTCTTCTGTAGTTTGCAGCATCCACAAATCC
AAATTCTAATGTTTTTAACGGTTTCATCTTAATAATCTCCTATTTAATTTTGAATTAAAC
TTACCTCAAAACCACCTTCAAATACTTCCCAGTATAACTCCCCTTAACTTTCGCCACCTG
TTCAGGACTACCTTTAGCAATAATCCTCCCCCCGCCATCTCCGCCTTCCGGCCCCAAGTC
CACAATCCAATCCGCTGTTTTAATCACATCCAGATTATGCTCGATAATCACTATCGAGTT
GCCTTTGCCTTTCAGACGGCCTATGACTTCCAGCAGCAGGGCGATGTCGGCGAAGTGCAG
GCCGGTGGTGGGTTCGTCGAGGATGTAGAGCGTTCTGCCGGTGTCGCGTTTGGAGAGTTC
CAAGGCGAGTTTCACGCGTTGGGCTTCGCCGCCGGAGAGGGTGGTGGCGGACTGTCCGAG
GCGGATATAGCCTAGGCCTACGTCCATCAGGGTTTGCAGTTTGCGCGATACGGTGGGGAC
GGCGTCGAAAAATTCGCGGGCTTCTTCGACGGTCATGTCGAGGACTTGGCTGATGTTTTT
GCCTTTGTATTGGATTTCGAGCGTTTCGCGGTTGTAGCGTTTGCCGTGGCAGACTTCGCA
GGGGACGTACACGTCGGGCAGGAAGTGCATTTCGACTTTAATCACGCCGTCGCCTTGGCA
GGCTTCGCAGCGGCCGCCTTTGACATTGAAGGAGAATCTGCCGACGTTGTAGCCGCGTTC
GCGAGAGAGGGGGACGCCGGCGAAGAGTTCGCGGATAGGGGTGAACAGGCCGGTGTAGGT
GGCGGGGTTGGAGCGAGGAGTACGGCCGATGGGGGACTGATCGACGTTGATGACTTTGTC
GAGGTGTTCGAGGCCGTGGATGTCGTCGAATGGGGCGGGTTCTTCTTGGGCGCGGTTGAG
TTCGCGGGCGGTAATTTTGGCGAGGGTGTCGTTAATCAGGGTGGATTTGCCGCTGCCGGA
CACGCCGGTGATGCAGGTAATCAAACCGAGCGGCAGCTCAAGGGTAACGTTTTTGAGATT
GTTGCCGCGTGCGCCTTTGAGGACGAGCATCCGGTCGGGATTGACGGGCGTGCGTTCAGA
CGGCACGGCAATGGATTTTTTGCCGCTGAGGTATTGTCCGGTAACGGAGTTTTCGCATTG
GGCGACGTTTTCGGGCGTGTCGGCAATCAGTACGTTGCCTCCGTGTTCGCCTGCGCCGGG
GCCCATATCGACCACGAAATCGGCTTCGCGGATGGCGTCTTCGTCGTGTTCGACCACAAT
CACGCTGTTGCCCAAATCGCGCAGGCGTTTGAGGGTGGCCAGCAGGCGGTCATTGTCACG
CTGGTGCAGGCCGATGGAGGGTTCGTCCAGTACATACATCACGCCGGTCAGGCCGCTGCC
GATTTGGCTGGCGAGGCGGATGCGCTGGGCTTCGCCGCCGGAGAGGGTTTCGGCGGAGCG
GCTTAAATTCAGGTAATCCAGCCCGACGTTAATCAGGAAGCCGAGGCGTTCGGTGATTTC
TTTGAGGATTTTTTCGGCGATTTGTTTTTGTTGCCGTCTAAATCCAGTGTTTCAAAGAA
TTGGTGGGTTTTGGTGAGCGGCCAGGCGGAAACTTCGTGCAACGGCTCACCGCTGACGTA
AACGTAGCGGGCTTCTTTGCGCAAACGTGCGCCGCCGCAGCTTGGGCAGGCGCGGTGGTT
TTGGTATTCGCGCAGTTTTTCGCGCACGGTTTCGCTGTCGGTTTCGCGGTAGCGGCGTTC
GAGATTGGGGATGATGCCTTCAAAGGCGTGGCTGCGGTTGAAGGTGGTGCCGCGTTCGGA
CAGGTAAGTGAAATCAATGACTTCTTTGCCTGAGCCGTGCAGCACAACTTTTTTCACTTT
TTCAGGTAGTGTTTCCCAAGCAGCCTGCACATCGAAACCGTAATGCCGCGCCAATGATTG
AATCATTTGGAAATAGAATTGGTTGCGCTTGTCCCAACCGTCAATCGCACCTGTTGCCAG
CGACAATTCGGGATGGGCGACCACTTTTTCGGGGTCGAAGAAATTGGTGTTGCCCAAGCC
GTCGCAAGTCGGGCAGGAACCCATCGGGTTGTTGAACGAAAAAAGGCGAGGCTCTAATTC
GGGCAGGCTGTACGAACACACGGGGCAGGCGAAACGTGCGGAAAACCAATGTTCTTCGCC

## Appendix A

GCTGTCCATCTCCATCGCCAGCGCACGCTCGTTGCCGTGGCGCAGCGCGGTTTCAAAACT
TTCCGCCAGCCGCTGCTTGATGTCCGCCTTCACTTTCACGCGGTCGATGACCACGTCGAT
ATTGTGCTTGATGTTTTTTTCCAGCTTCGGCACTTCGTCCAACTGATAGACCTCGCCGTC
CACGCGCACCCGCGCAAAACCCTGCGCCTGCAAGTCGGCAAAGAAATCGACAAACTCGCC
CTTACGCTCGCGCACGGTGGGGGCAAGAATCATCACACGCGTGTCTTCCGGCAGTTTCAA
TACGGCATCGACCATCTGCGATACGGTTTGGCTCGACAGCGGCAGCTTGTGTTCGGGACA
ATACGGGGTACCGACACGGGCGTATAAAAGACGCAGATAGTCGTGGATTTCAGTTACCGT
ACCGACGGTGGAGCGTGGGTTGTGGCTGGTGGATTTTTGCTCGATGGAAATTGCAGGCGA
CAGACCTTCAATTAAATCGACATCGGGTTTGTCCATCATCTGCAAAAACTGCCGCGCATA
GGCGGAAAGGCTCTCGACATAACGCCGTTGCCCTTCGGCATACAGCGTGTCAAACGCCAG
CGACGACTTGCCGCTGCCCGACAATCCTGTTACCACCACGAGTTTGTGGCGCGGAATGTC
TAAATCGATGTTTTTCAAATTATGCGTGCGCGCCGCGGATGCGGATGGTGTCGTTGTC
GTGCGAATGTTGGGGATGATGGTTGCACATAATGGATGCCGCCTGAAAAATAAAGGAAAA
CCGGTATTGTAGCACTTTCTCGGATGCCGTCTGAAGCCGCGTTCAGACGGCATTTGCCAG
CGGAGTACGGCAGATTCCGCTATAATGTCGGCAATTTTAACCCGCTTGAACAAAAGGATG
ACAAATGAACCGTCTTTACCCCCACCCGATTATCGCCCGTGAGGGCTGGCCGATTATTGG
CGGCGGTTTGGCTTTGAGCCTGCTGGTGTCGATATGTTGCGGCTGGTGGTCTTTGCCGTT
TTGGGTGTTTACCGTATTTGCATTGCAGTTTTCCGCGACCCTGCGCGTGAGATTCCGCT
AAATCCTGAAGCGGTGTTGAGCCCGGTTGACGGCCGTATCGTGGTGGTCGAACGCGCACG
CGATCCGTATCGTGATGTCGATGCTTTGAAAATCAGTATTTTTATGAACGTGTTCAACGT
GCATTCGCAAAAATCGCCTGCCGATTGTACGGTAACGAAAGTGGTCTATAACAAAGGCAA
ATTCGTGAATGCGGATTTGGACAAAGCCAGCACGGAAAACGAACGTAATGCGGTGTTGGC
GACTACGGCTTCAGGTCGTGAAATTACTTTTGTTCAAGTGGCCGGTTTGGTGGCGCGCCG
TATTTTGTGCTACACCCAAGCAGGTGCGAAACTGTCCCGCGGCGAACGTTATGGCTTTAT
CCGCTTCGGTTCGCGCGTGGATATGTATCTGCCTGTCGATGCGCAGGCGCAAGTGGCGAT
TGGCGATAAAGTAACCGGCGTCAGCACTGTATTGGCGCGTTTGCCGCTGACTGCGCCGCA
AACTGAATCTGAGCCTGAATCTGAGCCTGCTTTACAAACTGCTCCGGTTGAAACAGCGGC
AAACCCATCTGCCGAACAACGGCAAATCGAGGCAGCGGCGGCTAAGATTCAGGCGGCTGT
GCAAGATGTGTTGAAAGATTAATTTTGCGGACTGAAATAGAAAATATCAGTACCATCATT
CACACGAATGAGGAAGTTTGGTTTTTTGAATTTTTGCTAATGTTCACACCGTCATTCCCA
CGAAAGTGGGAATCTAGAAACTTAACGTTACGACGATTTATCGGAAACGACTGAAACCGG
ACGGACTGGATTCCCGCCTGCGCGGGAATGACGACTTATTAGTTACCTAACACTTAAAAA
ACAGAAACCTTTCCGCGTCATTCCCACGAAAGTGGGAATCCGGGAACTTAACGTTACAGC
GATTTATCGGAAACGGCTGAAACCGAACGAATTGGATTCCCGCCTGCGCGGGAATGACAA
CTCATTAGTTACCTAAAACTTAAAAAACGGAAACCTTTACGCCGTCATTCCCACGAAAGT
GGGAATCCGGGAACTTAACGTTACAGTGATTTATCGGAAACGGCTGAAACCGAACGAATT
GGATTCCCGCCTGCGCGGGAATGACAACTCATTAGTTACCTAAAACTTAAAAAACAGAAA
CCTTTACGCCGTCATTCCCACGAAAGTGGGAATCTAGAACCCAAATGCTAAGGCGATTTA
TCGGAAACGGCTGAAACCGAATGAATTGGATTCCCGCCTGCGCAGGAATGACAACTCATT
AGTTACCTAAAACTTAAAAAACAGAAACCTTTACACCGTCATTCCCACGAAAGTGGGAAT
CTAGAACCCAAATGCTAAGGCGATTTATCGGAAACGGCTGAAACCGAATGAATTGGATTC
TCGCCTGCGCGGGAATGACGACCCATTAGTTACCTAAAATTTAAAAAACAGAAACCTTTC
CGCGTCATTCCCATGAAAGTGGGAATCTAGAACCCAAATGCTAAGGCGATTTATCGGAAA
CGGCTGAAACCGAACGAATTGGATTCCCGCCTGCGCGGGAATGACGGATCTTGGGTTTC
TGCTTTTGATTTTTCTGCTTTTGCGAGAATGACGGCGTGAAAGTAAGAATGATGAAACAA
AAAAAATGGGAATGATGGCATAGTGGTTTGTTCTTTGTCTTTGCCATATTTCCTAACAAA
TTGATTAAAAAGAAAAAAGGTTTTCAGAATGCCGTCTGAAAACCTTTTTTGTTTGCCTGT
CCGATTTTAAAACTTCACGTTCACGCCGCCGGTAAAGCTGCGGCCCATTTGCGGCGTATC
AGAGAGAAAGCTGCTGTGGGCGTAAACGGATTGGTTGAGCAGGTTGTCGGCTTTGACGTA
CCAATTCCACTCGCCATAGCGCGTATTGCGGCGGTAGTTTGCGCCGAGGTTGAGCATATG
GTGTCCGGGCGTGCGCGTTTCGTAGCGGGCGAGTTTGTTTTGGGCGAACACGCGGTAGTA
GTCCAAATTGGCATCGATACGGTCGGTCAGCGAGGCTTTCAGGTGGAAGCCGAGGCGCGC
AGCCGGAACACGGGGGGCATTTTGGTCGTCCTGTGCGATGAAAGGACGGTTGCCGTAGGC
ATCTTCTCTGCCGGGTAGGGAAGGCAGGTTTTTCAGACGGCCTCGTACATAGTCGCCGGA
AACGCCGATGCGGTAGCGCGGTGTCGGTTTGAAGTAGATTTCGCCTTCCGCGCCGTAGAA
GTCGGCGCCGGATTGGTTGTAGCGCACGAGCTTCATTTCGCTGTCGTCTTCGATGGATTT
GGGGCCGCGTCCGTCGTTTAAGGTTTGGGCGTAAATGTAGTTACCGAAGCGGTTGCGGTA
GAGTGCCAGATTGTATTGCCAGCGGTCGCCTTCGTAGCCCAGCGCGAGTTCGATATTGTT
GGAACGCTCTTTGTTGAGGTGTTTGTTGCCGACTTCAAAGGTGTTGGTGGCGACGTGTTT
GCCGTGTGCGTACAGCTCTTGCGTTGACGGCAGGCGTTCCTGATGGGAGGCGGTCAGGCT
GAGTTTGTGTTGTGGCGTGAAATACCAGTTGCCCGAAAGTGCGAATGAGCGGGCGGTTTG
GCGGTGCGCGCCGAGGTCGGGCAGGGGGTGGTTGTAGTAGTTTTCCCGATCAATCAATGC
TTTGTCGTACTGAATGGAGGCTTTTTGTTTTTCCACGCGTACGCCTCCTTCAAGCGTGAA
GTTGTCCCAGTTTGCCTGTTCTACACCGAAAAAGCTGTAATGTTGCACTTTGTTGTCAAG
CAGCATCGGTTGTTTAACCGCTTCGGATATGGCAGATAAAGCACTGGATTTTTGTTGTAA
ATATTGCACGCCCCAGCTGCCTTTCAGACGACCTATGGGTTGGTGGCGCAACTCGATGCG
GGCGTTTTGCGTTTGGTTGTTAAAAAAGTTTTCGACTGCATCGCCTGCTTTTTCGTCGTG
GCGGTAGTCGTTGCGGTTCAGGTGTACGCGCAGGGCTTCAAAACCGGGGAACGGTTGCTT
CCATTCGGCACGGAGTTCGTAGCGTTTGTTGCGCAGGTCTATCCACGGTCTGCCGCTGTG
GGTGTGTGCGTGTGCATTATCGTCGTCGTGGAAGCCGCAGCTCAAGCCCGGATTGTCGTA
ATCGATGTCTTCTTCGGTCAACAGGTGCGGATAAAGCTGTAAATAGCGTTTGTTAATCAA
GCTCTTTTGCCAGATGATGTCGGCGTGGCAATCATCGTATTCGTGGCTGTGGGCAGGCAG
ACCATATTGGTCGCGACGGTCGCTGTACGCTACGCCGATAAAACCTTTTTCGCCAACCCA
AGACAGCCCGATGCTGCCCGTTTGCGAATCGGCGTGGCTGTCGGGCAGGCGTTTCAGATT
GCGGTAACGCGGTACGGCGTAATCCCCCGATTTGCGGTACAGCCCTTCCGTGTGCAATAC

## Appendix A

AAAGTTTTTGCCCAAACCGATATTGATGCCGCCGGACGTGAGTTTTTCCAGATTGCCGCT
GCTCAAACGCAATCCGAGTTCGCCCGATACGCCGTTTTCAGGCATTTTTTCGGGGATTTT
GCCATCGGCAACATCGACCAGCCCCGCCACATTGCCCGAGCTGTACAAGAGCGTAACCGG
CCCGCGCAGGATTTCGACCTGTTGCGACAAGGCGGTATCTACCATAATGGCGTGATCGGG
CGAAAAATCCGCCATATCGCCTGTTTCGCCGTGATGGTTCAACACTTTAATCCGCCTGCC
TGTTTGACCGCGAATGACGGGAGCAGACGCGCCGCCGCCGTATTGCGAAGCGTGGATGCC
CGGTACGCCGTCTAAAGCGTCGCCCAAGTTGACGGCTTTTTGGCGCAAGGTATCGCCGGA
GATGATTTTGTCGGAGGCGGTCGAAGTGTGCAACAGCCCCGACGTGGCGCGCGGACGGCT
TTTGCCGACGACGCTGACCGTTTCCAAATCCACCGATTGCTCAGTTTCATGCGCTTGGGC
GAGGAGGGGTGTGTTGATTAAAAGAATTGATAAAACAATGGGTTTGAGTGTAGTTTGTGC
CATTTTGGCTTCTCGTCGCATTTCAAAAGTTTGTTATTATATAACATTACATTTTTTATA
TCATAAGATTTTGAGAACACTCAGAGGGCATAGGCAAAAGTTTTTCAAATGAAACGGTTG
CGGCATCGGGCGGTGTCCATTTGTATCCGCCGTCCTTCGGGGGCGCGGTTGATGTTGACG
CAGATTCCGCTGTGTTTGCCCATTATGTTCGGCTGCGGGATAACCAAAATTTATGAGTGC
ATAAAAACGGCACGTTCCCGAACGGTCGAAAAGGTGGCAAAATGGCGTACTTGTCAGAAC
GCGAGGCTCTGCGCCAGGTTCACGAGGGCGCATCGGGCACGCTAAGATATAAGAGGGTAT
GGATGGGGGTATCGGAAATGCAGTTAATAACAAACAAATTATAAATCAATAGGTTAATCA
CAAAATGCTTTTGTTTATCGACAATTACGACAGTTTTACTTACAACATCGTCCAGTATTT
CACTGAATTGGGGCAGGAAGTTGCCGTGCGCCGCAACGATGATATTACGTTGGAGGAAAT
CGAGGCATTGAATCCGCAATATCTCGTTATCGGCCCCGGCCCGTGTTCGCCCAAAGAAGC
GGGGATTTCCGTGGCGGCGATGCGCCATTTCGCCGGCCGGCTGCCGATTATGGGCGTGTG
CCTCGGGCATCAGACGATAGGCGAGGCGTTCGGCGGCAGGATAGTCCGCGCCAAAACGCT
GATGCACGGTAAGGTGTCGCCCGTGTCCCATTCGGGCAAGGGTATGTTTAAGGGTTTGCC
CAATCCGGTTACCTGTACGCGTTATCACAGCCTCGTTATCGATCGGAATACGATGCCCGA
ATGTTTGGAAGTAACGGCTTGGACTGAGGACGGCGAGATTATGGGTGTGCGCCATAAGGA
ATATGCCGTCGAGGGCGTGCAGTTCCACCCCGAAGCCCTCTTGACCGAGCACGGACATGA
TATGTTAAACAATTTTTTAATCGAATTTCAAAACTTCAAACCGCAAAAAATCTGACGTGA
TGCCGTCTGAAGCCCTTCAGACGGCATTTTCGTCCGAATATTGAACGGAGGACAAAAAAT
GATTACACCGCAACAGGCCATCGAACGATTAATCAGCAATAACGAGTTGTTTTACGATGA
AATGACCGACTTGATGCGTCAGATTATGAGAGGACAGGTTCTGCCGGAGCAGATAGCGGC
CATTTTGACAGGATTGCGTATCAAGGTTGAAACCGTTTCCGAAATTACCGCAGCTGCAGC
CGTCATGCGCGAGTTTGCGACAAAAGTGCCGCTGGAGAATGCAGAGGGGCTGGTCGATAT
CGTCGGTACGGGCGGGGATGGCGCGAAAACCTTCAATATTTCGACGACTTCGATGTTTGT
TGCCGCAGCGGCAGGCGCGAAGGTTGCCAAACACGGAGGCCGGTCGGTCTCTTCCTCCAG
CGGTGCGGCTGACGTGGTGGAGCAGATGGGCGCAAACCTCAACCTGACTCCCGAACAGGT
TGCCCAAAGTATCAGGCAGACCGGCATCGGGTTTATGTTCGCGCCCAATCACCACAGTGC
CATGCGCCATGTCGCCCCTGTACGCCGTTCGCTCGGTTTCCGAAGTATTTTCAACATATT
GGGTCCGTTAACGAATCCTGCGGGCGCGCCGAACCAGCTTTTGGGCGTGTTCCACACCGA
TTTGTGCGGCATTTTGTCGCGGGTCTTGCAACAACTTGGTTCAAAACACGTTTTGGTTGT
TTGCGGGGAGGGCGGTTTGGATGAAATTACACTGACGGGCAAAACACGCGTTGCCGAGCT
CAAAGACGGAAAAATCAGCGAATACGACATCCGCCCAGAAGATTTCGGTATCGAAACCCG
CCGCAATTTGGATGAAATCAAAGTTGCCAATACTCAGGAATCTTTGTTGAAAATGAATGA
GGTGCTGGAAGGAAGAGAAGGGGCTGCGCGCGATATCGTATTGCTCAACACCGCCGCCGC
CCTGTATGCCGGAAATGTCGCTGCTTCGCTTTCAGACGGCATATCTGCCGCACGGGAAGC
CATCGATTCAGGCAGGGCCAAATCGAAAAAAGAGGAGTTTGTCGGTTTTCAACCACAACA
AAGATGCCATTTTCTTGGAAAGATGGAGCTTGGGTGATGCCGCCATGATTATGGAACTTT
TGTGGCAAAACATAAGCACTTCACGAAGAGAACTTACCAAACTGTTTTTATATAAAAACT
TGGGGCTGTACTAGATAACCAGACCAAATTCCCATTAACTAATTGTCTTAAAATCTGAAT
TTGAGATTCTATTTAAAATGCCATTGGCATTTCTTTAAATGCAGCCCCAAATGCTCTTTG
GGAATGCCGTTAAACTTACGTAAATGGCTAAATTCACTAATATCAAGGAGATCATAACTA
CGAAAGGTATCCGTGTATACAATGCCATCAGGCTTAACTTTCTTTCGGATAATTGGCAAC
AATGTCGCTGATTGCGCATTAGGAACAACGACGGTATAAACCTTATATATTGCGTCCCTA
AGAAGGGACGATTAACAAAAATTAACGTCCTTTACTTTCTACAAGTAACAGGGCTTTTTT
TTGCCCGTTTTTGAGGATTCGCACCATGGAAGATAAGCAAGGGATGACAAAGGCGGTTGC
CGGCGTGATGACGGACGCGCTAGCGGACGGCAGGAAGCCGACAACCGCTTCAAATCTTCC
CCCCTTATCTAACAGGGGGGGACAGAAACCGAAACGGCAGGCAGGGTTCAGGAAGTCTTC
GAATGTTACGAAACGTACATAACGGACGGTAAAGGAAACCTGTTAGGCGTTCCTCTTCGG
CGCGGTGTATCAGATTCGGCTTTCATTGATCAAATTAGCTTTTCATTTCATGAAAAAACC
TTTTTCGATAAATACGGCGTTCGTGTAAGTCTTTTGGAAGACGAAGATTTTATTCGCGCC
GCGTCCATGCTCGCCGAAGAAGTTTTCGGTTTCGGTATCTACAAAGAATCCAAAGGTTCG
GGCGGTCGTTTCTATGAGCGCTGTTGGTTGATGGGTTCGGAAGACGCCCTATACGGTCGC
GTCCATTTTGGCGGCCAACAAAATACCATTCTTTTCGAACTGACCGGCACCGGTTGCGGC
GTCGCAAAAGAAGGCTGGGAATCACGACTTTTCGCATTCCTGACTAATGCAATCCGCCCA
AAAATCACACGCGTTGACATCGCAAAAGACTTTTTCAACGGCGAATACAGCCCGAACCAA
GCCCGTGAAGACCGAAATAAAGGTATGTTTACCTGTCATCACGTCAAACCAAAAGGCGAA
TGTTTGGGGTCAGATTGGGAAGAAGACGATGAAGCCAAAATGACCAAAGGCAAGACCTAT
GGTATCGGCTCCCGTGAATCGTCCAAATATGTCCGCGTCTATGAAAAAGGCAAGCAGTTG
GGCGATAAAACAAGCACATGGACGCGATTTGAAATTGAATTCAAAGCAAAAGACATCGTT
ATCCCTTTCGAAGTTTTGCAGAATCCGGGCGAATATTTCGGCGGCGCATATCCGATTTGC
GAACGATTCGCCCAAAAGGCAACGCGCATACACGCGGTTAAGGAAGATAAGGTCATTTCA
GCCGACCGCTACCTTGAATGGGTAAAAAAACAGTTCGGACGTGCGGCAAACGGTCTGAAA
TTCATTTTTCCCGAATTGGACAAAGCCAAACTGTTTGAACTGATTGAGCCGAGTCATCAC
AAGCTGCCCAAGTCTTTGGCTCCCGAAGCCTACGACTGCGCCTTTTTGAAAGCTCAAGCC
ATTCATGAACAGCCCGCATTCAAACCGTACAAAGACCCTTACTATATGTACGAATATTAC
GAGAATCTTGAAAAACAGCTTGAACAGCAAAAACACGTCAACAATGAAGAAAGCTATAAC

## Appendix A

AACTTCATTTACGACAAATTCGCAAGACTACCGATTTCATGGGCTTAAAGTGTCTGCCCG
AAAGACGTTTAATCACACAAGGAAACCAAAAAATGAACATCCAACTTCAAGGCCACATCG
TCGGCGTTAAAAAAATCAACGGACAAATCGAAGGCAAGAGCTTCGACTATTGCTGCCTGA
TTGTCGCCACACCCTTAGACAGCTCCCAAGGCAACGCATTGGGCAGCTCTACTACTGAAT
ACGATTTCGGCGGCTCTGCCAATTTCGAGCAGTTCCGAAACGCCCAATTTCCGATCGAAG
CAAACCTGAACGTAGAAATCGTCACTACGGGCAAAACCCAAAAACTGAAAGTCATCGGTT
TTCAACTCGTGAAGAAAGGCTGATTGAATGCAGAAAGTCTATGTTGTCCAGTCCGTATCA
ACAGGGGACTTTCTGTATCTCTCTCCTGAAACGGGCGACATCGGACATACCAAATTAATC
ACCAATGCCGATTATTTCTACGACTTCGAAGAAGCGATTAACGCAGGTTTGGAAGAAATC
GGCAACCAATACGAATTTGTCGTATTCGGATTTTTGAAAGACTGATTTTCGGATGTTCGG
CGGTCGTCTGAAAAACGCTCCATCCATTACCGCCAAACACTTTTTGAAGGAAAATATCAT
GAAATTTATTAACACCTGCCGTAAATACGGCGCAAAACTGGCTGTTGTAACAGCTGCTCC
CCTGGCTTTGGCCGCACATGCAAATGCAACGTTGCCCGATACGGCAAAAAACGCTTTGGA
AGCCGCAAAAGCGGACGGTATGGAAGCCGGTTGGATTGTAGTGGGCATTTTCGCCGCGCT
TTTTGTATTTTCCATCGTTAAGAGAGTGATGAAGTAAGACGGCATGTACTACCAAGTCGG
AAATAAATGTCTTGAGAAGCACCAGGCTGAAAACCTTTATTTCAGCTTGGTAGTACCAAG
AATCAAAGAAAACGGACAGATTGTCAGGCCGGAATATAACGGCAGCCTGTGGAAGATGTC
GGACGGTCAGCCGCTAAGGCTTTTATTGGCGGAATGCAGTCCGAAAGACAACCTGCAAAG
CGGTCTTGAAACAGGCTGGATAGTATTCGGCATCCTCGCGTCCGTTTACTTTGTTTCCCT
GCTGAAAAAGGTTTTGAAATGATGGATTTTTATTTTTATCTCGGCGTTTCCGTACCCGTA
TTAATCGGGGCGGTTCTGTTTAAGAATTGAGCGCATGAAGTTATGGTGTCAAAATCAGGC
TTTCAAAACAATCATTGAAAGGCAGAACCATGAACAAGCCGTTTATCACTCAGGCGCAGT
TGGCACTTTATAAATATCAGCCGTCCAGCAAGTATTTTGGGCAATCGATGGCGGTTATAG
CGCAATCTGAATTTGTTGAATTTGCGAAGATTAATAAGTCTGAAAATGTTATTGATTGTT
TCTCTTTTTTCTGGAATAGAAGAATTAAACATGATATTTGGCTAATCTCATTTTCTGATA
ATTCAGAAATGGTAATTAAAGAATCCCTGAAAGATGGTCATAAAATATACAAATTTGAAT
TTTGCGAAATTGTCGATAATTGCAATTTTGATGATGTATTCGTTTGAAGCGAATGCAAAT
GCAGTAAAAATATCTGAAACTGTTTCAGTTGATACCGGACAAGGTGCGAAAATTCATAAG
TTTGTACCTAAAAATAGTAAAACTTATTCATCTGATTTAATAAAAACGGTAGATTTAACA
CACATCCCTACGGGCGCAAAAGCCCGAATCAACGCCAAAATAACCGCCAGCGTATCCCGC
GCCGGCGTATTGGCGGGGGTCGGCAAACTTGCCCGCTTAGGCGCGAAATTCAGCACAAGG
GCGGTTCCCTATGTCGGAACAGCCCTTTTAGCCCACGACGTATACGAAACTTTCAAAGAA
GACATACAGGCACGAGGCTACCAATACGACCCCGAAACCGACAAATTTGCAAAGGTCTCA
GGCTAAGTGCGCCTGTTGCCGCCTAAAAGGTACCCCGGATGCCTGATTATCGGGTATCCG
GGGAGGATTAAGGGGGTATTTGGGTAAAATTAGGAGGTATTTGGGGTGAAAACAGCCGAA
AACCTGTGTTGGGGTTTCGGCTGTCGGGAGGGAAAGGAATTTTGCAAAGGTCTCTTTTCG
TCATTCCCGCCACTTTTCGTCATTCCCGCGAAAGCGGGAATCTAGAATCTCGGACTTTCA
GATAATCTTTGAATATTGCTGTTGTTCTAAGGTCTAGATTCCCGCCTGCGCGGGAATGAC
GATGCAGGTATTTCTGACGATTCCCGGCTATGATGTTGAGGCAGAAATCGAAAAATTCGT
TTGGATGGATGCTGTGATTTGGCAGATGCCGGGCTGGTGGATGCACGAGCCTTGGACAGT
GAAAAAATACATAGACGGAGTATTAACCGCTGGACACGGCAAACTCTACCAAAGCGACGG
CAGACACAGCGTCAATCCGACTGAGGGCTACGGCACAGGCGGCTTGTTGCAAGGCAAAAA
ACATATGCTTTCACTGACTTGGAATGCGCCGATTGAGGCGTTTACCCGCGAAGGCGATTT
CTTTGAAGGCAAAGGCGTTGATGTTTTGTATATGCACTTCCACAAAGCCAACGAGTTTTT
GGGTATGACCCGCCTGCCGACATTCTTATGTAACGATGTGGTTAAAAATCCGCAAGTGGA
AAAATACTTGGCAGATTATCAGGCACACTTGGAAAAAGTGTTCGGCTAATTAAAAATCCA
TCTTCAACACGGAGATGGATTTTGTTTGTTTCGTTGATTTTGTGTCAGTTTCAGATGTAG
GTGCTTATTCGGACGGGCCGTCTGAAAATGTTTGCCCCAATGCAAAAAAATCACTGCAAA
CCTTCATAAACGGGGTTTGCAGTGATTTTTTCAAATCAAACAGATTGAAAACCTGCGCCG
~~AATTGTTCAGACGGCATTATTTTTTCAGTTCGGACAGAATGTCATCTACGGTTTTCTTCG~~
CATCTCCGAAACACATCACGCTGTTTTCGTTGAAGAACAGTGGGTTTTGTACACCTGCGT
AGCCGGTATTCATCGAGCGTTTGAAGACGACGACTTCTTTTGCCTTCCACACTTCCAACA
CGGGCATACCCGCAATCGGGCTGTTCGGGTCGGTTTGGGCGGCGGGGTTGACGGTGTCGT
TCGCACCGATGACCAAGACCACATCGGTTTCGGGGAAGTCGTCGTTGATTTCGTCCATTT
CCAAAACGATGTCGTAGGGGACTTTGGCTTCGGCGAGCAGTACGTTCATATGACCGGGCA
GGCGGCCGGCGACGGGGTGGATGCCGAAGCGTACTTCGGTGCCGTTTTTACGTAAAAGCT
CGGTGATTTCGGCAACGGGGTATTGCGCTTGTGCGACTGCCATACCGTAGCCCGGGGTAA
TGATGACATTGTTTGCGCCTTTCAGCATTTCGGCAATATCGGCAGCTTTGACTTCTCGGT
ATTCCCCTATCTCTTGGCTGCCGGAAGATAATGTGCCGCTGTCGCTGCCGAAACCACCGG
CAATTACCGAGACAAACGAGCGGTTCATGGCTTTGCACATAATGTAGGACAGAATCGCGC
CGCTTGAGCCGACCAGCGCGCCGGTAACGATGAGCAGGTCGTTGGAGAGCATGAAGCCTG
CCGCTGCGGCCGCCCAGCCGGAGTAGGAGTTGAGCATGGACACGACCACGGGCATATCTG
CGCCGCCGATGGAGGCAACCAAGTGCCAGCCGAATGCGAGGGCAATCAGGGTCATAATCA
GCAGGATGAAGCCGCTGCCGTCAATGCCGACAAATACGAGCAGCAACACAAACGATACGG
CAAGTGCCAGTGCGTTGAGCTTGTGTTTGGCGGGCAGTTGCAGCGGGCTGCTGCTGATTT
TGCCGTTGAGTTTGCCGAATGCGACCAGCGAGCCGGTAAAGGTTACCGCGCCGATGAAGA
TGCCTAAATACACTTCGACCAGATGGATGGTGTGCATATCGTGCGAAACGTTGCCCGGCG
CGATATAGCTGTTGAAGCCGACCAAAACCGCCGCTAGGCCGACGAAGCTGTGCAGCAGGG
CAATCAGTTCGGGCATTTCGGTCATTTCCACCTTTTTGGCTTTGTAGATGCCGATTGCCG
CGCCGATGAGCATGGCGATGATGATCCAGCCCAGTCCGTGGGTATTGTCGGAAAAAACAG
TTACAAAAAGGGCGACCGCCATACCGGCGATACCGGAATAGCAGCCCTGTTTGGCGGTTT
CCTGTTTGGACAGCCCCGCCAGTGAGAAGATGAATAAAATTGCGGCAACGATATACGCCG
CTGTTACGAGTCCTGAAGACATAGAAATTCTCCGATTTTCGATGATTTGTTTTCAATGCC
GTCTGAAAAATTGACGTTCGTGTTTTCAGACGGCATCTGTTTCAAGCAGCCGCGACAAAC
AGCCCGACGGTGCAGGCAAATCCGCCTGCCCACATCAGTGAGCGCATAGCGGCTTTGTCG

# Appendix A

GCGATATAGCACCAGATAAAGGCGAGGCGGAACAGGATGAACAGGCAGGCAAGCGTGTTG
ATGGTCGATTGCGCCGCATTGCCGGTTGCGTGTGCCGTCAAAACGGCGGCGGCAAACGGT
GCAAAGGCTTCAAAACCGTTTTGCTGTGCGGCGTGGGCACGGGCGGCTGCGCCTTGCGTG
TGCGCTAGAAAACCGCGCGGATTGTGGTTGTCTTTAAACCGGAATCCGCCCGCTTTTTTG
GCATACGCCGCACAAAAAAGCGGCAATAGGCAGGCAATCAGAATACACCAATAGGCGAAA
GTCATGGCTTACCCTTTCTTAAACATATTCAGCATACGCCGTGTTACCGCAAAGCCGCCG
AAGATGTTGATGCCGGCAATCAGGATGGCAACAAACGACAGCAGCGAAACGAAGCCGTTG
CCCTGACCGATTTGCAGCAGCGCGCCGACGACGATGATGCCGGAGATGGCGTTGGTTACC
GACATCAGCGGTGTGTGCAGCGAGTGGCTGACGTTCCAGACGACGTAGTAGCCGATGACG
CAGGCGAGAACGAACACGATAAAGTGGTTCAGGAATGCTGCGGGTGCGACCGCGCCGACC
CACAGTACCAAGACGGCGGCGATGACGGCGGGCGCGAGTTTTTTCCACAGGGGAACGGGT
TTTGGCTCGGGCTTGGCGGCAGGCACGGCTTTTTCAGACGGCGTTTGCTGCGGCTGGGCG
GAAACTTGAATCGGCGGAGGCGGGAAGGTGATTTCGCCGTCGTGGGTAACGGTCATGTTG
CGGATAATCACGTCTTCGAAGTCCAACGTGATTTCGCCGTCTTTGTTCGGGCTTAACAGC
TTGGTCAGGTTGACCAAGTTGGTGGCGTAAAGCTGGGAAGACTGTCCGGCAAGGCGGTTT
GCCATGTCGGTGTAGCCGATGATTTTCACGCCGTTGCCGGTTACGGACAATTCGCCCGGG
CGGGTGAGTTCGCAGTTGCCGCCCGTCGCCGCCGCCAAATCGACGATGACGGAGCCGGAT
TTCATGCTTTCCACCATTTCTTTGGTAATCAGCTTGGGCGCGGGTTTGCCCGGAATGGCG
GCGGTGGTGATGATGATGTCCACTTCTTTCGCCTGCTCGGCAAAGAGCTTCATCTCGGCT
GCGATAAATTCGTCGCTCATCACTTTGGCGTAGCCGTCTCCGCTGCCGCCCGATTCTTGT
GGGAAGTCGAGTTTCAGGAACTTGCCGCCCATCGATTCGATTTGTTCCGCCACTTCCAAG
CGGGTATCGAACGCGCGTACCACTGCGCCGAGCGAGTTTGCCGTACCGATCGCCGCCAAA
CCTGCCACACCTGCACCAATCACCAAAACCTGCGCGGGCGGCACTTTGCCGGCGGCGGTA
ATTTGACCGGTGAAGAAACGGCCGAAGGCGTTGGCGGCTTCAATTACGGCGCGGTAGCCG
CTGATGTTTGCCATCGAAGACAAAGCGTCCAAAGCCTGCGCGCGCGAAATGCGGGGCACC
ATATCCATCGCCAGCGCGTTCACTTTCTTGGCGCGCAAGGCTTCGACCAAAGCCTCGTTT
TGGCGCGGCCACAGGAAGCTGACGATGGTTTGACCTTCGTTCAAAAGCGGCAGTTCCTGT
TCGGACGGCGCGTTGACCTTATAAATCAAAGGGCAGACCCAAACCGCCGCTTTGTCGGCA
ACGGTTGCGCCTGCTGTTTGGTAAGCGGCATCGTCCAAACTTGCCGCCAAACCTGCACCG
CTTTCGACAACGGTTTCAAAGCCCAGTTTGCCCAGCAGGGCGACGGTGGCGGGCGTACAG
GCGACGCGGGTTTCGCCGGATAATGACTCGCGTGGGATACCGATTTTCATCTCTGAATCC
TTTTTCGGGTTGTTTATATGTATCGTGGGTTAAATTTAAATCGGGGCGGGGCGGAGCAAC
GCCGTACCGGTTTAAAGCCGACTCACTTCAAATGTTAATATATTTTAGATAATCCCCTTA
TAACGAATTTTCATCAGGCTGGCAATAGTTGCGGCATTTTCCCGTGTTGTCCGACACATA
CTGCCGTTTGTGTTTGAAATCGTTTTCCGATTGCAGTCCCGCCCGCCGTAACATTCTTTG
CCGTTTCACTATATAATCCGCATTTTTTGAGCCGCCGTTATGCCGCACGCCCTCGTCCTC
CAATTTCCCTCCGCCGCAGCCCTGCCTTCCGACTTCCCCTTACGCCTGCCCGAACCTGAT
TGCGCCGATGAAAAGCGTATGCGTTTTATCGTTGAAGAAGGGTTTTCTTTAAGCGAAAAA
GACGCGGCGTTGCTTGGCAGCCGTCAAATCGACCACGCCGTGTTGCCGGATATGGATTTC
GACGAACTCGGTTTGATTGTCAGCGATATGGATTCGACGCTGATTACCATCGAATGCGTC
GATGAAATTGCGGCAGGCGTGGGTTTAAAAAACAAAGTAGCGGAAATTACCGAGCGTTCG
ATGCGCGGCGAACTCGATTTCGAACAGTCTTTACGCAGCCGCGTCGCGCTGTTGGCGGGA
TTGGACGAACGGGTTTTGGCGGACGTTTATGAAAACGTTTTGAAGCTCTCGCCCGGTGCG
GAATTTTTGTTGGACGAATGCAAAAGGCACGATGTGAAATTCCTGCTGGTGTCGGGCGGC
TTCACGTTTTTTACCGAAAGGCTGCAACAACGCCTCGGCTTCGAATACCAACACGCCAAT
GTTTTGGAAATTGAAAACGGCAGGCTGACCGGCCGTCTGAAAGGCAGAATCATCGACGCG
CAGGCAAAGGCAGATTTGTTGCGCGAATACCGCAGCCGCCTCGGATTGCAGCCGCATCAG
GTGTTGGCGGTGGGCGACGGTGCGAACGATATTCCGATGCTCAAAGAAGCGGGCATAGGC
GTGGCTTACCGTGCCAAACCGAAAGCGCGGGCCGCCGCCGATGCCTGTATCAACTTCGGC
~~GGTTTGGAGCGTGTACGCGGCCTGTTCGGATAGGCGGATAGGAAACGGATGTCGTCCGAA~~
AGGTTTTCAGACGGCATTTGAACGGCAGGAACGACAGTGGGACGCAGAAAACTTTGGTTT
GCCCTGGCAGCAGCGGGCGTTATCGGCGGTTTGGTCGGCATTGTGCTGACGGAACTGATG
CACTTCATACAGCATACGGCATACGGTTATGGCGCGGACGGCGTGTACACTTCGTTCCGC
GAAGGCGTGGCACAGGCTTCCGGTATGCGGCGCGTTGCCGTGCTGACGCTGTGCGGCGCG
GTCGCAGGCAGCGGCTGGTGGTTGCTGAAACGTTTCGGCAAGCCGCAAATCGAAATCAAA
GCCGCCTTGAAACAGCCGTTGCAGGGGCTGCCGTTTCTGACGACGGTTTTCCATGTTCTG
CTGCAAATCATAACGGTCGGACTCGGTTCGCCGCTCGGACGCGAAGTCGCCCCGCGCGAA
ATGACCGCCGCGTTTGCTTTTGCCGGCGGCAAACGCTTGGGTTTGGATGAAGGCGAAATG
CGGCTACTGATTGCTTGCGCTTCGGGTGCGGGTTTGGCGGCCGTGTATAACGTGCCGCTC
GCCTCCACACTTTTCATTCTCGAAGCCATGCTGGGCGTGTGGACGCAGCAAGCCGTCGCC
GCTGCATTGTTAACTTCAGTCATCGCCACCGCCGTCGCGCGCATCGGCTTGGGCGACGTG
CAGCAATATCATCCGGCCAACCTTACCGTCAATACTTCATTACTTTGGTTTTCCGCCGTC
ATCGGCCCGATACTGGGCGTAGCCGCCGTCTTTTTCCAGCGTACCGCCCAAAAGTTCCCC
TTTATCAAGCGCGACAATATCAAAATTATTCCCTTGGCCGTCTGTATGTTTGCACTCATC
GGCGTGATTTCCGTTTGGTTTCCCGAAATTTTGGGCAATGGCAAAGCAGGCAATCAACTG
ACCTTTGGCGGATTGACCGATTGGCAACACAGCCTTGGGCTGACCGCCGTCAAATGGCTG
GTCGTCTTAATGGCGCTTGCCGTCGGCGCATACGGCGGTCTGATTACCCCGTCCATGATG
CTCGGCAGTACCATCGCCTTTGCTGCTGCCACCGCGTGGAACAGTGTTTTTCCTGAAATG
TCCTCTGAAAGCGCAGCCATTGTCGGCGCCGCAGTTTTCCTCGGTGTTTCCCTTAAAATG
CCCCTGACCGCCATAGCCTTTATTTTGGAGCTCACCTACGCCCCTGTTGCCTTGCTCATG
CCATTATGTACAGGCATGGCAGGTGCAGTATGGGTGGCAAAGAAAATGGGATTTAAATAG
GCAAAAGCAAAAGGCCGTCTGAAACCAAGTTTCAGACGGCCTTTTACAATAAAATTGTTA
ACAATATTTGCAAAAACCTACTGCCAAAAATGCGAAACTGGGGGATAATACCGCCCTGAA
AATTCATCCCATACTGATTAAACCTTCAACAAAGGAAATCCAAATGTCTTCCATCAAACG
CGCCCTGATCAGCCTATCCGACAAGACAGGCGCAGTCGAATTTGCCCAAACCCTGCACAA

## Appendix A

ACTCGGTGTCGAAATTCTTTCTACCGGCGGTACAGCAAAACTCTTGGCTGATGCAGGCGT
TCCCGTTATCGAAGTTGCCGACTATACCGGTTTTCCCGAAATGCTCGACGGCCGCGTGAA
AACCCTGCATCCGAAAATCCACGGCGGTATTCTCGGTCGTCGCGATTTGGACGAACACGT
CGCCAAGATGGAAGAACACGGCATCGGCAATATCGACCTCGTGTGCGTCAACCTCTACCT
CTTCGCTGCCACCATCGCCAAACCAAACTGCACGCTGGAAGACGCGATTGAAAACATCGA
CATCGGCGGCCCGACCATGGTGCGCTCTGCCGCGAAAAACTGGAAACACGTCGCCATCGT
TACCGACACCGCCGATTTCCCGGCCATAGCTGCCGAACTCGAAGCCAACAACGGCGCATT
GAGCGACAAAACCCGTTTCAACCTCTCGCGCAAAGCATTCAGCCATACCGCCCAATACGA
CGGTATGATTTCCAATTACCTGACCTCGCTTTCAGACGACGTCTTGAGCGGCACGCCCGA
AATCGCCGGATTCCCCGGCCGGTTCAATCAAAGCTGGATTAAAGTGCAAGACATGCGCTA
CGGCGAAAACCCGCATCAGCGCGCCGCGTTCTACCGCGATATTGACCCCGCCGCAGGCAG
CCTCGCTGCATACAAACAACTGCAAGGCAAAGAATTGTCTTACAACAACATCGCCGATGC
CGATGCCGCATGGGAAGCCGTCAAATCCTTCGACGTGCCCGCCTGCGTGATTGTGAAACA
CGCCAATCCGTGCGGCGTAGCCATCGCCTCCAATACCTTGGATGCCTACAAACTCGCCTA
CGCCACCGACACCACCAGCGCGTTCGGCGGCATCATCGCTTTCAACCGCGAAGTTGACGG
CGCAACCGTCAAACAAATTACCGACAACCAGTTTATGGAAGTCCTCATGGCGCCTAAGTT
CACCGCCGAAGCCCTCGAAATCGCCGCCGCCAAGAAAAACGTGCGCGTATTGGAAGTGCC
GCTTGAGGCAGGCGCAAACCGCTTCGAACTCAAACGCGTCGGCGGCGGACTGTTGGTGCA
AACGCCCGACATCCACCGCATCAGCCGCGCCGATTTGAAAGTCGTCTCCAAACGCCAACC
GACCGAGCAGGAATGGAACGATTTGCTGTTCGTCTGGAACGTCGCCAAATACGTCAAATC
CAACGCCATCGTATTCGGCAAAGGCGGTCAAACCTACGGCATCGGCGCAGGCCAAATGAG
CCGCGTGGACAGCACCCGCATCGCCGCCCGCAAAGCGCAAGATGCCGGTCTCGACCTCAA
CGGCGCGTGTGCCGCATCCGATGCCTTCTTCCCCTTCCGCGACGGCGTGGACGTGATTGC
CGAACAGGGCATCAAAGCCATCATCCATCCGGCAGGCTCGATGCGCGATCAGGAAGTTTT
CGACGCAGCCGACGAACACGGCATCGCCATGGTCGTAACCGGCATCCGCCATTTCCGCCA
TTGATGCAGATAAACAAGGTAATGCCGTCTGAAGGGCTTTCAGACGGTATTTTGCGCTAT
TTTGCGAAGGTAGGGATGACGGTTCGGGTATTCCTGACAGGGTGGATTTTCAAGGTGTTG
TATAGGGTGTAGGAGGATTCGTAAAAGGTGGGATGCAGGGTGTGCTTCAGCCCGCTGCAT
CAAAAATTTTTGGAGAACCGGCGGGAGTCGGCGGTTTTGGTTTCGGCGGGGACGGTGGAA
ATGGGTAACATTGACGGAATCGACGGAAGCGGTGGACTGAAGCCCACCCTTGTATATTGG
AATCACCGTATCATAGCAACAAACCGCCCAGCCGCCACCCGCGCCCACCCAAGGCACACA
ACCGTTGCGTAGCTCAGGGAGCGGCAGGGCAACCCATCGACACAACCGGACAGTTGCCGG
ACAACACAACCGAATGCAAGGCAGGTTTATGATGAGTACCCAATACCATTACGCAGGTAT
AGTGAATTAAATCTAAACCAGTACAGCGTTGGTTCGCCTTAGCTCAAAGAGAACGATTCT
CTAAGGTGCTGAAGCACCAAGTGAATCGGTTTCGTACTATTTGTACTGTCTGCGGCTCGC
CGCCTTGTCCTGATTTTTGTTAATTCACTATATCGACATCGCCAAACGAAACTTCGTCAT
CGCCGTTTCGTCTTTGTCTAAAACCAAAACCGAAACCAACAACCCCAAAGGTATCGCCCA
TACTATCGAATACCTTAAAAAACACAAGGTCGCCCTCGTCGTGACGGAAAGTACCGGCGG
TCTCGAAATCCCCGCCGCCAAAGCCATCCGCCGAGCAGGGCCGTGATTATCGCCAACCCG
CGTCAGACGCATCAGTTTGCCCAATCGCAGCCGCTGACCAAAACCGACGCCAAAGATGCC
AAAATGCCCGCCTTCTTCGCACAGATGACGGCACAGAAAGAAGATTCGCAAACCATGCCC
TACCACCCGCCCACCGAAGTGGAAGAAGTGTTGGAAGCCTTGGTTAACCGCCGCAACCAA
CTGGTGGATATGCGGACTGCCGAGAAAAACCGTCTGCATTAGGTTCATGAAACGCAAGTC
GAAAGCGTCAAACAACTGATTGCCCATTTTGACCGGCTGATTGACGAATTGGACAAACAA
ATCGACAACCACACCCACACGCATTTTGACGGCAAAGCCCAAGTGGCAGAGCAAATCAAA
GGCATCGGTTCGATAACGACGGCTACGCTGATGGCGATGTTGCCCGAATTGGGGCGGCTG
TCGCACAAACGGATAGCGAGTCTAGTCGGCATTGCCCCACACCCGAGGGAGAGCGGGGAA
ACCAAATTCAAAAGCCGCTGCTTTGGCGGAAGGTCTGCGGTGCGTAAGGCACTGTATATG
GCTACCGTGGCAGCGACACGTTTTGAACCGCTTATTCGGGATTTCTACCAACGCCTGCCG
TCCGAGGGTAAGCCGTATAAGGTTGCCGTTACGGCATGTATGCGCAAACTGCTGACGATA
TCGAATGCCCGGATGCGTGATTATTTTGCCGAAAACGATACCGCCGAAAACGGTATCTAA
ACGGCTTGATTTGAGTTTTGGTATTTTTGCCCGACGGGGTGAAAAATACAGTTGCTTTTT
TATGTCTGTCCGTTTCGCAAAAAACATCGGCTTAATACTATATATTGTGTTTTATGGGTT
TGAGATGCGCCGGGCGTTGATTGCGAAAATTAAGATTGCTCAAAAGGAGCTGGGCTTGGA
TGACGGTACCTATCGCGCGGTGTTGGAGCGTGTGACGGGCAAGCGGTCGTGTGCGGATAT
GGATGTTTCCGAACTTGAGTCTGTTGTCGCTGATATGCGGTCGCACGGATTTAAGCCTAA
AGCAAAAGGTAACCCACACGGCAAACCGCATCTGCGTCGGACATCATCAGCGGCAATGTT
GGACAAAGTCGAAGCCCTGCTGACCGTCGGCGGCAAACATTGGAACTATGCACACGCAAT
GGCGCGGCGGATGTTTGGTAAGGATAAGGTCGAATATTTAGACGATACGCAGCTACATAA
ACTGGTTGCTGCGTTGCAGATTGCGGAAAACAGGAAAACGGAAAAAGCGGGTGGGGATGA
TGGGGTTCGAAAAAGTTGAACATTTATTGCCGGATACCGTGTTGGACATTGTGGATGTCA
TCGGACTGGCAGCGACGGAACAGCTGGTCAAGGCGATTGGCGGGGCGCGGTTTAAATTTG
GTAAGGGCAAGGTGGACACCGAGCGTTTGGCAATTTTGGTCGAAGCCATCGGCGAAGTGA
AAACACATGAGCTGTTGCAGGTATATGGTGGCGAGGAATTGTATGTCCCACGGTGCGGCA
AGGCGTTAATACAGTTGAGAAACCATAGGTTTTATCAGGAGTTTGTCAAATTGCGCGATA
TTGATAAGAAGAGCGGGCTTATGGCGATGACGAAGCTATGCCCTAAATACGGCATCTCTT
CACGAACGGGATATACGATTATCAATGAAATGAGCCGACCTGCGGCACAGCAGGCAGCTT
TATTTTAGGCAGTGATGTGTGACCAGGCTTTGGCCGTCTGTATTCAGACGGTCTTTTTTT
TGGTTTGCAGGGGTGAAACATCTACCGTTCGGGACGATGGGTTAAAGACGGTTTAATGGG
GTTTTCAAATGTTATAGTGGATTAACAAAAACCAGTACGGCGTTGCCTCGCCTTAGCTCA
AAGAGAACGATTCTCTAAGGTGCTGAAGCACCAAGTGAATCGGTTCCGTACTATCTGTAC
TGTCTGCGGCTTCGTCGCCTTGTCCTGATTTTTGTTAATCCACTATATGCAATCAATATT
TTTTGGAGATGATGAATGGGCAAAACCGTAACCTTAACCGCTGGACACAGCAACACCGAC
CCGGGTGCGGTCAACGGAAGCGACCGTGAGGCGGACTTGGCGCAGGATATGCGCAACATT
GTGGCTTCAATCCTGCGTAACGATTACGGCCTGACCGTTAAAACCGACGGCACGGGCAAA

## Appendix A

GGCAATATGCCGCTGCGCGATGCGGTCAAGCTGATTCGCGGCTCGGATGTGGCGATTGAG
TTCCACACCAATGCGGCGGCGAACAAAACGGCGACAGGCATCGAAGCCTTGTCCACGCCG
AAAAATAAACGCTGGTGTCAGGTGCTGGGCAAAGCCGTTGCCAAGAAAACCGGCTGGAAA
CTGCGCGGCGAAGACGGCTTTAAGCCGGATAACGCAGGGCAACATTCGCGCCTGGCTTAT
GCGCAGGCAGGCGGCATTGTGTTTGAGCCTTTTTTCATCAGCAACGACACTGATTTGGCC
TTGTTTAAGACGACCAAATGGGGCATCTGCCGCGCGATTGCGGACGCGATTGCGATGGAA
TTGGGAGCGGCGAAGGTATGAAAAAGTCTTTGATTGCTTTATGTGTTGCCCATTGTGCAA
AGTTGAAAAACGATTTTGGCGTACCACCGTTACCTGAAATCAAAATCACGCCAAGCCCTG
TTCGGGTAGGCTCTTTGAAACAACATCCGAGCCTGCGCTTGGGTAAATCAGGCGTGGCGG
CTGCTAAACGTGCGGCGCGCAAACGCAAGAATCGTCGTTAATCATGGGACAGGTTGCGTT
TTACGAAAAGATGATTGGGCTGTGGTCGGCCAAAAGCCGTGAGGCAAGCGAACAGGCGGA
CTTGGCTGCGTTTGAATTTGCGGAGGGCGAACTGGCCAATTATCGGGAAATGCTGAAACG
GCACCTGCAAACCAAAAGTGTGGAATAGCAATGCGTATTTTGGATATTTTTAAAAACCCG
GCGACAGGCAATGTGTCGCACTCGAAACTGTGGGCAAACGTTGCCTGCGCGGCTGGGACG
TTTAAGTTTGTGATGTTGCCCGATCCGTCGGCGGAAATTTGGGCGGTGTATTTGGGCATT
GTCGGCGGCTATGCGGTGGCGCGTTCATTTGTCAGCGTGAAGCGTCAGGAGGTCGAGAAT
GAATCTCGTGAAACTGCTGGCGAATAACTGGCAACCGATTGCCATTATCGCGCTTGTCGG
CACGGGCTTGGCTGTGTCGCACCATCAAGGCTACAAGTCGGCATTTGCGAAGCAGCAGGC
GGTCATCGACAAGATGGAGCGCGACAAGGCGCAAGCCCTGCTGTTGTCGGCTCAAAACTA
TGCGCGCGAACTGGAACTGGCACGCGCGGAAGCTAAAAAATATGAAGTCAAGGCGCACGC
TGTCGGCATGGCTTTGGCGAAAAAACAGGCGGAAGTCAGCCGTCTGAAAACGGAAAGAGA
CCTTTGCAAAATTCCTTTCCCTCCCGACAGCCGAAACCCAAACACAGGTTTTCGGCTGTT
TTCGCCCCAAATACCGCCTAATTTTACCCAAATACCCCCTTAATCCTCCCCGGATACCCG
ATAATCAGGCATCCGGGCTGCCTTTTAGGCGGCAGCGGGCGCACTTAACCTGTTGGCCGC
TTTCAACAGGTTCAAACACATCGCCTTCAGGTGGCTTTGCGCACTCACTTTAATCAGTCC
GAAATAGGCTGCCCGCGCATAGCGGAATTTACGGTGCAGCGTACCGAAGCTCTGTTCGAC
CACATATAGTGGATTAAATTTAAACCAGTACGGCGTTGCCTCGCCTTGCCGTACTATTTG
TACTGTCTGCGGCTTCGTCGCCTTGTCCTGATTTAAATTTAATCCACTATAACGGGTCTT
CGATAAATATCGGTTACGCTTGGTTTGCACTTCCGACAGCGGGCGGTTGCGGCAGGCTTT
GCTCATAATGCCGTCCAACAACTGATGTTCTTCCAGATGTTGCCGGTTTTCCGCACTGTC
ATAGCCTTTATCGGCATAGACGGTCGTACCTTCGGGTAACCCTTCCAACAACGGCGACAG
GTGTTTGCACTCATGGGCATTGGCGGGGGTGATGTGCAGTTTCTCGATATAGCCTTCCGC
ATCGGTACGGGTATGTTGTTTGTAACCGAGTTTGTAGAGGCCGTTTTTCTTGATCCAACG
GGCATCGCTGTCCTTACTCGGTGTGGTTTGGCCGCTGATTTGTCCTTCTTCATCGACTTC
TATGGCCTGACGCTGTTCGCTGCCGGCGGTCTGAATAATGGTGGCGTCAATGACGGCGGC
GGATGCTTTCTCTACTTTTAAGCCTTTTTCGGTCAGTTGGCAGTTAATCAGTTCCAACAG
TTCGGACAGGGTGTCGTCTTGCGCCAGCCAGTTGCGGTAGCGGCATAAGGTGCTGTAATC
GGGGATGCTCAGTTCGTCAAAACGGCAAAACAGGTTGAAATCGATGCGGGTGATGAGGCT
GTGTTCGAGTTCGGGATCGGAGAGGCTGTGCCATTGTCCGAGCAGGACGGCTTTGAACAT
GGATAGCAGGGGATAGGCGGGACGGCCGCGGTGGTCTCGGAGGTAACGGGTTTTTTGACG
GTTCAGGTACTGCTCGATCGGCTGCCAATCAATCACTTGGTCCAACTTCAATAGCGGGAA
ACGGTTGATGTGTTTGGCAATCATGGCTTGCGCGGTTTGCCGGAAGAAGGTGCTCATGAG
AAATCCCCTAAATGTCTTGGTGGGAATTTAGGGGATTTTGGGGGGATTTTGCAAAGGTCT
CAGGCGGCAAATCGCCACCCTTCCCTTCAAACCTTCCGCCTGTCCCAACAGCAGACAGGC
GAAAAAGCCCTTACCACTGATAACCGACAGATGCGGAAGCACCGAAATGGCCGCGCGAAT
TGCCGGAAGCCGTGCCTTTGATAATCCAATTTCCGCCGTCGGAAATACTGGAGTAGCCGA
TGGCGTAACCGGCTTCGCCGCGATAAGTGCCGCCGCCGATCGCCATCATACTCTTGCCGG
GCAAATACGCCTGAACCAGACCTGCGGTTGCAATCGCTTGGGCGATGCCCGCACGCGCGT
TGCCGTCCACATTGTCGATGCGGTTGTTCAAGTTTTGCGCCACGCCTTTAAGTTGTGCGA
CGTTTGTAACATCCCCCTCTTTAACGCCCGGGGCGACATTGGTAATGCGGACGGGTTTGT
TGTCCTTCTTGCTGCCGACATTCAATGCGTCCCCATCCACGCTCAAAGTGGGCGCATCCG
CCCCCGCGCCGAGCGAAACGCTGGAAAACTGCGGGGTCATCGAAGTGGCGATGTCGATAT
TTTTACCGTTGCGGGTAATCTCGATGTTGTTGCCGGCATTAATGTTGACGGTTTCATCCA
TCTTTCCCTTGCTCGGCGAAACATTGCCGCTGATGACTTTGCCCGAAGAACCTGCAACCG
CTTTGGAATCCAAATTCCAACCGCTGTTTTGCAGCTGATTGACGTTTAGGGCATCGCCGA
CATTTACATCATACATAACAGTGATGTTGCCTTGATCATCTTTACTTACAGTCGCAGTTG
TACCTTTACCACTAGCAAAGGTTACATTTGTGCCTGATGTAACGGTTTCAAACTTGTCAG
CTTGACCTGTTTGACCATTAGCGGTTGTTGTTTTCATTCTCCAACCAGCCTTGTTTACTG
CATCAATCACTTCTTTTGCAGTCACTAAGCCTTCGCCTTCGTCTGTAGAAGAACCATTCT
CGCCTTTGTCTTTACCAGTAACCAACTTACCGTCTTTTTCTTTAATAACAGAAGTCTTCG
CACCGATTTTAACTTCGGTTTTCTTGCCGTTGTCTTTGCTTTCCACATTAACAGTCGTTG
TTTTCGTATCTGCGCTCAAGAACTCGACTGTGTCGTAAGTGCGGACGAAATCAACGTTAT
CGGAAGCTGTTGTACCGGGTTTAACGCCTTTAATGTTCCAGCCAGCGTTTAATACGTCTT
TAACGCTTGCCGCACGTTTTTTCTCGTCATCGGTAACGTTGTCGTTGGTTACGTTTGTGG
TCGCTCCGGTATTCAGCAGCGTATCGGTCAAAGTCGAACCAATACCGTTCAGATGAACCG
TGGTGTCGCCGTTCGTCCCAGCCGTTTCTTTCGCAAAATTCAAGCCTTTGGTGTCGCTTG
TGATGTTGACTTTATTGCCGTTTGCGCTAAACGATAATTTTTCAGTTCCAACACTGGTCA
GATCTGTGAGGTCTTTTTTCAGCGAGTAGGTGAAGTTTGTGCCGTTTTGTTTGATTTTCA
GGTTGTCGCCGGCTTTGAGGGTGATTTCTCTGGCTGTTAGTACTCCTTTCTCGTTGAAAT
ATACTGCCCAATCTGAATTTTCTTCTACTTTTTCTTTTTCTCCCGTGCCTTCTTTATCGG
AATTGACTATCAACACGGCAACAGTGCGTTGTACGGGGTCTAAATATAAATCTTCTTCTT
GCTCTTCATTGTTAGCACTTGCCTGAACCGTTGCAAACAACAGTGTCGCCAATACGGCGG
TCTTCACGGTTGCGGAGGCGCGTTTGGTGTGGTTGCGTGTGAGCTCGGATACGACGACCC
AGGCATTGAGGGCACTATTCCAAATGATGCGGTATATTTGTTCATTTTGTTTTTCTTT
TGGTTTGTTTGAATGGTTAAATCGGGGTTTGGGGGCGGATGGTGCGGCATCCGCCCGGTT

## Appendix A

TTTGGGGGTTGGGGGTTTTCTGATAAATTCCCCCAACTTAAAATCTCGTCATTCCCGCGA
AGGCGGGAATCTGGGACGTGGAATCTAAGGAAACTGTTTTATTCGGTAAGTTTCCGTGCC
GACGGGTCTGGATTCCCGCTTTTGCGGGAATGACGGCGGTGGGGTTTCTGTTTTTTCTGA
TAGATTCCTGTGGTTTTTCTATGGATTCAATCATTCCTGATAAATTCCCATAATCTAAAA
TCTCGTCATTCCCGCGAAAGCGGGAATCTAGGACGTGGAATCTAAGGAAACTGTTTTATC
CGGTAAGTTTCCGTGCCGACGGGTCTGGATTCCCGCTTTTGCGGGAATGACGGTCGGTGG
GGTTTCTGTTTTTTCCGATAAAGTCCTGCCGCGTTGTGTTGCTGGATTCCCGCCTGCGCG
GGAATGACGGCGGTGGGGGTTTCTGTTTTTTCTGATAGATTCCTGTGGTTTTTCTATGGA
TTCAATCATTCCTGATAAATTCCCATAATCTAAAATCTCGTCATTCCCGCGAAGGCGGGA
ATCTAGGACGTGGAATCTAAGGAAACTGTTTTATCCGGTAAGATTCCGTGCCGACGGGTC
TGGATTCCCGCTTTTGCGGGAATGACGGCGGTGGGGTTTCTGTTTTTTCCGATAGATTCC
TGTTGCGTTGCGTTTTTGGATTCCCGCTTTTGCGGGAATGACGCGGTGGGGGTTTCTGTT
TTTTCTGATAGATTCCTGTGGTTTTTCTATGGATTCAATCATTCCTGATAAATTCCCATA
ATCTAAAATCTCGTCATTCCCGCGAAGGCGGGAATCTAGGACGTGGAATCTAAGGAAACT
GTTTTATCCGGTAAGATTCCGTGCCGACGGGTCTGGATTCCCGCTTTTGCGGGAATGATG
GCGGTGGGGGTTTCTGTTTTTTCCGATAAAGTCCTGCCGCGTTGTGTTTCTGGATTCCCG
CTTTTGCGGGAATGACGCGGTGGGGGTTTCTGTTTTTGCTGATAGATTCCTGTGGTTTTT
CTATGGATTGAATCATTCCTGATAAATTCCCATAATCTAAAATCTCGTCATTCCCGCGAA
GGCGGGAATCTAGGACGTGGAATCTAAGGAAACTGTTTTATCCGGTAAGTTTCCGTGCCG
ACGGGTCTGGATTCCCGCTTTCGCGGGAATGACGGCGGTGGGGTTTCTGTTTTTGCTGAT
AGATTCCTGTGGTTTTTCGGTTGCTGGATTCCCGCTTTTGCGGGAATGACGGCGGTGGGG
TTTCGGTTTTTTCCGATAAATTCCTGTTGCGTTGCGTTTTTGGATTCCCGCTTTTGCGGG
AATGACGGTCGGTGGGGTTTCGGTTTTTTCCGATAAAGTCCTGCTGCGTTGTGTTGCTGG
ATTCCCGCCTGCGCGGGAATGACGGCCGCCGGACGGCAAACGACCATACACAATTATTGA
CAACCCCATTTATTGCGAAAGTCAGCCTAGGAGAATCGATCTAATTGTCAACATTCCCTT
ATGATCAAAGGGATTACACTTTATTTTACGCAAATGCGGGGGGGGGGGTGATTTTTGACG
TTTTTTGCCGAAAATTTACATTCGGACGACGAAAAGGAAAAAGCCGTGTCGCATCTGTGC
AACACGGCTTGGCGGGCGCAAACGGATATAGTGGATTAACAAAAACCAGTACGGCGTTGC
CTCGCCTTAGCTCAAAGAGAACGATTCTTTAACAAGTGAATTGGTTCCGTACTATTTGTA
CTGTCTGCGGCTTCGTCGCCTTGTCATGATTTTTGTTAATCCACTATAAAACGGTGTTCC
CTGCCGCCGCAGGCGGAACGCCGGATGACGGGGTTTTCCCTAAGGGTGCGGCTGCCGCTA
TATCACGAAATCCAACAGGTAGAAATCTTCTTTGCCCACGCCGCATTCGGGGCATTTCCA
GTCGTCGGGGATGTCTTCAAACTTGGTTCCGGGGGCGATGCCGTGTTCGGGGTCGCCGTG
TTCTTCATCGTAAATCCAGCCGCAGGGGCCGCACATATATTGCGCCATTTGTGTTTCCTT
GTTTTTTGTATAGTGGGTTAACAAAAACCGGTACGGCGTTGCCTCGCCTTAGCTCGAAGA
GAACGATTCTCTAAAGTACTGAAGCACCCGTACTATTTGTACTGTCTGCGGCTTCGCCGC
CTTGCCCTGATTTTTGTTCATCCGCTATAAATCAGGGTTTGGGAGAATGGTGCGGTATCC
GCCCGGTTTTTTTGGGGTTGGTTTTTTTCGATAGATTCCTGTGGTTTTTCGATTACTGGA
TTCCCACTTCCGTGGGAATGACGGTTTGGAGGTTTCGGTTTTTTCGATGAATTCCTGTTG
CGTTAGGGGGGGGGCTGGATTCCCGCTTTTGCGGGAATGACGGTTTGAGGGTTTCTGTTT
TTTCCGATGGATTCCTGTTACGTTGGGGGCTGGATTCCCGCTTTTGCGGGAATGACGGTT
TGAGGGTTTCTGTTTTTTCCGATGGATTCCTGTTACGTTGGGGGCTGGATTCCCGCTTTT
GCGGGAATGACGGTTTGAGGGTTTCTGTTTTTTCCGATGGATTCCTGTTGCGTTGGGGGC
TGGATTCCCGCTTTTGCGGGAATGACGGTTTGAGGGTTTCTGTTTTTTCCGATGGATTCC
TGTTGCGTTGGGGGCTGGATTCCCGCTTTCGCGGGAATGACGCGGTGGGGGTTTCGGTTT
TTCCGCCTGTTTATTTTGCGGCTTCGATTGCCGCTATTTCTTTGCGTAGGTGTTTGATAG
CGGGGGTTACGATGGCAACAAACATTGCTTCGCGGACGCGCCTTTGGGCGGGACTGCGCA
TCAGGTAGCCTTTTGCGCCGGAATGCAGGGCGGCGGATTGGGCGGCGGCAAGTGCCAGTA
CGGCGGCGGCTTCGCGCAGCTTGAGGGTAGCAAGGTTGTCGGGCGTGCCGCTCCAAGCCA
AGCCGGCGAGCCGTTCGGTTTCTGCCCACGCGCCGTCCAGCCTTGTTTTGAGGCTGTCGT
AGCCGTCGTTGAGGTAGTTGTTGACTTCGGCGTTGACGACGTTGGCGAGGCGGATGATGC
CGAGGCTGCCGTCGATTACGCCCGCGCCGATGCCGATTTGCAGGAGGATAAAGCCTGCTT
TGATGCTTTGGATGTAGTCGGCAAACTGTTCGGGCGCGGCGATGATGTCTTCGTCGGGGA
TAAATACGTCTTTGAAATTCAGGCTGAAGGTGCGCGTACCTTCGAGGGCGCAAAATTCGG
GGCAGTTTTGCAGGCTTACGCCTTCCCATTGTCCGCCTGTGATGAACATAACGTAGCCGT
CGCCGATTTGGGCGGTATTCGCCCAGATGTGGTCTTCACCGATGTTGGACACCCACGGCA
GCGCGCCGTTGACTGTGTAGCCGCCTTCCACGCGTTCGGCTTGGAGGTTGTGTTTTTCGA
TGTCGGCAAGGTGTTTGACGGTATTGGACATGCCCGTACCCGCCAATACTTTGCCTTGCA
GGATGTCGGCAAGGTATTTGTCTTTGACGGCCCGGTTGGGCGTTTGGTGCAGATACCACG
CGCAAGCCGCCTGACACCACGCACTGAAAGAGGTTGCGCCGCATTCTTTGCCGATTTCGC
GCAATACGGCGATTTGCGTTGCCAAACCCAAGCCGTTGCCGCCTTCGGCTTCTGTACCGA
CTGCGCCGAATCCACCGATTGCGCCGAGTTCGCGCATAAATGCTTCGGGGTAGTATCCTT
TGCGGTCGATGTCGTCCACTATGGGTTTGAGCTTGGTTTTGACGAATTCGGCAACGTTGG
CAATCAGGGTTTGGGCGTTCATCTTTGTTCCTTAAGGTTTGCGGGGAAATCGGGGGCGCG
CCTGATGCGCGGCTGCTTCCCTGCCGCTTAGGCGGCTTGTTTTTGTTTGAGGGGGTGTTT
TTGAAAAACCGCCCGATATTCGGGCGGTTTGCCGTATCAGGCGTAAGCCTGCAATTCGGG
GTTGATTTCGGTTTGTCCGAGGTTGTTGACGTAGTTGCACAGGGTTGCCAAGGCTACGCC
CATCACGACTTCGACTGCCTGCTGCTGGTTGTAGCCCGCATCGAAAAATGCTTTGAGTTC
CTCGTCGGATACCGCGCCTTTTTTCGCCATTACGGCTTGGGTGAAGGCGGCGAGCGCGCC
GAGTTTGGCATCGTCAAATTCGCCTGCTGCCAAAGCGCGCGCGGCTTTGACGGATTGTTC
GGACAGGAGTTTTTTCAGGGTTGCGAGTTTGGTGTGCCCTGCCACGCAAAAACCGCATTG
GTTGGTACGGGCGGCGATGATCTGGATGACTTCGACTTCGCCGGCGGTCAGGCTGTTGGC
GGCGTTGAGCTTGCCGACTTCTTGGTAAAACGCCAAGGCTTCGGGGGCGTTTGATAATAC
GCCGATAAGGTTGGGGATAAAGCCGTTGTTTTGAAGTACCGCCTCGACGCGCGCTTTGGC
GGCTTCGGGGGCGGTTTCGAGGGTGTGTACGGTTAAACGTGCCATTTTCTTTCCTTGTTC

## Appendix A

GCAAATATTGGGTACGGGCGCATGGTATGCATTTCGGAACGGAATAGGAAAGACTGATTG
GTTATGTGCTGCAAACAAAAGGTTATAAGAAATGCCGTCTGAACATTTTTCAGACGGCAT
GATGGAAAAGAAAACGCGCTTATCGGCCCCCGCGCCCGAAATATTGCGCCAATGCGGCTT
GGGGATTGGCTGCCTCAACCGTTTCGGGCAATTCGGTATAGCCGCGCGATAAAAGGTGGC
GTGCATAAAGGCTGTTGCCCTTTGCGCCGAACCAGACGGAAACCGCCAAGCCCAATACGT
TAAACGCCGAGTCTGTGGCGGGCGTGTCGCCGTAAACCAGTTGGGCAAACACAGCCAGCA
CGAATATCGCGCCCGTCAGCCCCAGCCCCAAACCCCACATTCTTTTGAAACACGCCCACA
GCGTGCCGAACAAGAGCCCCGGCCATGACCAGCCTTGTTTGACGGCTTGGGGCGGCAGGG
CGGGATGGGTGTAGATTTTGTATGGTTTCATCGTGTTTCCTTTTCGGTTGAAACCCTGCC
CTTTGGGAAGGTAGGATCAGACTTTATAGTGGATTAAATTTAAACCAGTACGGCGTTACC
TCGCCTTGCCGTACTATCTGTACTGTCTGCGGCTTCGTTGCCTTGTCCTGATTTAAATTT
AATCCACTATATTTGGGAGGCGCGCGCGCCTGTGCCGGCATACGGCTTGAAAGCGATTAC
CCGATGGGGAACTTCAAACCCGACAATGCCGTCTGAACGGTGTCTTGCCTTCAGACGGCA
TTGCCTGCCTTCAAAGCGGACGCGCTTATTCCGCCCAGTTTTTCTTTTTGCTGGTTTTGC
CTACGCCCGGGTTGAAGCTGTTGGTCGGGTCAAGTTTGCGGTAAAACTGTTTGAGCGCGG
GCTTGGCTTCGTACAAATGGCCGACGTTGTGTTCGGCTGGATATTGCGCGCCGCGTTGAT
CCAAGAGATGCAGCATTTCGTGTTCCAATGCCATGCAGTCGTTGCCTTTTTTGATGATGT
AATCCTGATGGAAAACGTGGCACATGAAATGTCCGTAGTAGAGCTTGTGGATGATTTTAT
TGTCGATTTCCGGCGGCAGTTTTTCAAACCAGTCGCGGTCGTCGCGGCGCAGGGCGATGT
CAAGCGCGACCAAGTCCTCCACTTCGTCGTCGTGTACGGCACGGTAGCGGATGGCGGCTG
AGGCGACGGCGAAACGGTGCAGCATCGCGGCTTGGGTTTCTTCGGCGTTGCACTCGAAAA
ACGCGCCGCCGTGATGTGCAAAATACTCTTTCAGGAACGCGCGCGCCTCATCCACGCCTT
TTCCGCCCATTTTCAGAATCAGGTGGTGTTCGTATTTGTCGCGGTAATCGCGCATGGATT
TGGGCAGATGGTCAGGCAGGAATTTGCTGACGAACTGCATTGCCTTGTCGGAAAAATGTT
TGGGCAGGAAGCTGACTTTTTTGCCGAACCTGTCCACGCGTGCCTTCAAATCAAATAATT
TCGGCAGTTGGTGCGTACCGAATTTTTTGATGACGTAAAACGTATCTTTGCCGTACACGT
CGGCAATGTCGAAAGCGTGGCGGTGGATGTATTCGCCGGAAACGGGCAGGCTTTCAAATT
CGCCCAAGGCGGCGCGGCGGATGTCGGTCAGCTCGTTGATGTCGTTCGTGCCGATGTAGA
ACACGGCCGTTTGTTTTTCTTGCGGAAAGGTATCCAAGCGGACGGCGAATACCATCAGCT
TGCCCGCGCAGCCCGAGGCTTCGTAATGGCGCGCTGGGTCGGCATTGAAACGCGCGGCGG
TCGGTTCGTCCACTTGGCGGACATGTTCGCAATAGGCGTGGTCGTGTCCTTTGCCCGCGT
CTTGCGTGATGTCTTTGTTTTGATAATGATGACCTTGAAGATTGGTCAGGATTTCTTCGG
GCGTGTTGCCCAAGTCTATGCCCAAGTGGTTGACCAGTTCCAACCTGCCTTCTTCGTTGA
TTTGGGCGAACAACGCCATTTCGGTGTAGGCCGGGCCGCGCTGTACCAACGCGCCGCCAG
AGTTGTTGCACACGCCGCCCAAGACGGACGCGCCGATACAGGATGAGCCGATAACCGAAT
GCGGTTCGCGCCCCAAAGGTTTCAGCAGCAATTCGAGCTGGTTCAGGGTCGAGCCGGGCA
GGCAGACGACTTGTTCGTTGTTGTTGATGGTTTGGATGATGTTCATCCGCATGGTGTTCA
CAATCACGATGTCGCGGTCGTAATCGTTGCCGTCGGGGGTCGAGCCGCCCGTCAAACCGG
TATTCGCCGCCTGCGTAATCACAATCACGTCTGCTTCGACGCACGCCTGCAGAATTTTCC
ACATTTCCAGAATGCTTCCGGGGCGAACCACCGCCAACGCCTTACCCTCGCCGAAGCGGT
AACCTTGGCGGTATTGTTCGGTTTTCGCGGGGTCGGTGATGATGTATTTTTCGCCTACGG
TTTGGGTCAGTCTTGACAGTAATTGTGATGCGCTCATGGCAGTTTCCTTAAAATTGTCGG
CAGGTGCATTGCACATTGGAATTGTTTTCACATTGTAGTTATACGTTATGGCAAAGTAAA
GAAAATGCCGTCTGAACGGCTTTCAGACGGCATCGGTGCGATACGGGAACGCCGGAACAT
CGAAGCTCCGGCGTTTCAAATAGGGCGGCGGGCCAAACCCCCGGCACTGGCGCATTGGAG
TGGGCTGCTGGCTTCCGCCCCTGACCCGGTGTTCCGATTTGCCATGCGGGGAGACCCGCC
TCAGAGAAACGGCATTATAACGGGTTTTCTGAAAAACTCAACCGTTTTGATACGGTCATA
CGCCGGAAACACCACCTAAAATTTATATTTGATAATATTGTCAACAATTTCTCAAAGCGT
TATTTTGTTTCTATAAGGGTATTTCCTGTTTCGGCATTGAAAAGTATCAAAAATTGAACT
ACATTATCGCCTTTTCAAACTCGCCTGAAACCGACTTTTCAGACGGCATTCAAATAAAAA
CTGCCAAACACGGACACACCATGACCACGACTACCGCCCCTCAGCGTATTCGGGAAATCC
CCTACAACTATACTTCCTACACCGACCGCGAAATCGTCATCCGATTATTGGGCGACGAAG
CGTGGCAAATCCTGCAAGACCTGCGCGGTCAACGCAAAACCGGGCGTTCGGCGCGGATGC
TGTTTGAAGTGTTGGGTGATATTTGGGTGGTCGTGCGCAATCCGTATCTGGTCGATGACT
TGCTGGAGCACCCAAAACGCCGCGCCGCGCTGGTACGTGAAATGCGCCACCGCTTAAATG
AAATCCGCAAACGCCGCGACGATAATCGGCAAGTGGATGTGTTGGTTGCCGCAGCAGAAA
AAGCAGTCGAGCGTTTTGATAGCAGTTTTGATGAAACCAGCCAAAAACGGCGGCAGATTT
TGGAGCGTTTGAGCAAAATCACCAAGCCGCACAATATTATGTTCGACGGGCTGGCGCGGG
TAACGCACGTTACCGATGCAACCGACTGGCGCGTGGAGTATCCGTTTGTCGTCGTCAATC
CCGACACGGAGGCTGAAATCGCGCCTTTGGTGCGCGCCTTAATCGAGCTGGATTTGGTCA
TTATTCCGCGCGGCGGCGGCACGGGTTATACCGGCGGCGCGATTCCTTTGGACGCAAACA
GCGCAGTCATCAATACCGAAAAACTCGACAAGCATCGTGGTGTTGAATACGTTGAGCTGG
CAGGCTTGGACGGCAAGCATCCGATTATCCGGTGCGGCGCGGGCGTGGTTACGCGGCGGG
TGGAAGAAACCGCGCATCAGGCAGGTTTGGTGTTCGCCGTCGATCCGACTTCTGCCGACG
CGTCATGCGTGGGCGGTAATGTGGCGATGAACGCGGGCGGCAAAAAAGCCGTGCTGTGGG
GGACGGCGTTGGACAACCTCGCCTACTGGAACATGGTTAACCCTCAAGGCGAATGGCTGC
GTATCGAGCGCGTGCGCCACAATTTCGGCAAAATCCACGACGAAGAAACCGCCGTGTTCG
ACGTTCACACGCTGGATTCAGACGGCATCAATATCGTTAAAACCGAACGCTTGGAAATCC
CCGGCCACAAATTCCGCAAAGTCGGTTTGGGCAAAGACGTTACCGACAAATTCTTGAGCG
GCCTGCCCGGCGTGCAAAAAGAAGGTACAGACGGCATCATCACCAGCGTTGCCTTCGTGT
TGCATAAAATGCCGAAATACACGCGCACCGTGTGTATGGAGTTTTTCGGTACGGTCGCCA
CCGCCACGCCATCTATTGTCGAAATCCGCGACTTTTTGCTTGCCCATGAAAGCGTGCGGC
TGGCGGGTTTGGAACATTTGGACTGGCGTTATGTCCGCGCCGTCGGCTACGCCACCAAAG
CGGCGGGCAAGGGACGACCGAAAATGGTTTTGCTGGCAGACGTGGTTTCAGACGACGAAG
CCGCCGTAGAGGCAGCCGCCGAACACATCTGTGAACTCGCACGCGCCCGCGACGGCGAAG

## Appendix A

GCTTTATCGCCGTATCGCCCGAAGCCCGCAAAACCTTCTGGCTCGACCGCAGCCGCACCG
CCGCCATCGCCAAACATACCAACGCCTTTAAAATCAACGAAGACGTGGTCATCCCGCTCG
AAAGGCTCGGCGAGTATTCGGACGGCATCGAACGCATCAACATTGAGCTTTCCATCCAAA
ACAAGCTCAAACTCTGTGCCGCCTTGGAGCAATATCTTTCGGGCAAACTCCCCATCGACA
AAATGGGCACTGACCTGCCGACCGCCGAACTGTTGGGCGAACGCGGCAAACACGCCCTGG
CCCACGTTTCCGCCGTCAAAACGCGTTGGGAATGGCTGCTCGCCAATCTTGACACGCCGC
TTGCCGACTACAAAGCCCGCTACGGCGCAGCCGTCCACGCCGCACCCGAAGCCAAAAACA
ATGAAAGCTGCTTTATTGCCTTCCGCGATTTCCGCCTGCGCGTGTCTGTCAAAGCGGACG
TAATGAAACCGCTTTCTGAAATCTTCAGCGGCAAAACCGACACCAAAATTATCCAAGGCT
TGGGAAAAATCCACGCAAAAACCGTACGCAGCCGCGTCTTTGTCGCCCTGCATATGCACG
CCGGCGACGGTAACGTTCACACCAATATTCCGGTTAACTCAGACGATGCCGAAATGCTTC
AGACGGCATACCGCTCAGTCGAACGCATTATGAAAATCGCCCGTTCGCTTAACGGCGTGA
TTTCCGGCGAACACGGCATCGGCATTACCAAGCTCGAATTTTTAAGCGACGAAGAAATGC
AGCCGTTTTGGGACTACAAAAACCAAGTCGATCCGAAACACACCTTCAACCGTCACAAAC
TGATGAAAGGCTCGGACTTACGCAACGCCTACACGCCGTCCTTCGAGCTGTTGGGCGCGG
AATCGCTGATTATGGAAAAATCAAACCTCGGCACGATTGCCGATTCCGTCAAAGACTGCC
TGCGCTGCGGCAAATGCAAACCCGTCTGCTCTACTCACGTTCCGCGTGCCAACCTGCTGT
ACAGCCCCCGCAACAAAATCCTCGGCGTGGGCTTATTGATCGAAGCCTTCTTATACGAAG
AACAAACCCGCCGCGGCGTTTCCATCAAACACTTTGAAGAACTCATGGACATCGGCGACC
ACTGCACCGTGTGCCACCGCTGCGTCAAACCCTGCCCCGTCAACATCGACTTCGGCGACG
TTACCGTAGCCGTCCGCAACTATCTTGCCGATTCCGGCCACAAACGATTTGCGCCTGCCG
CAGCTATGGGTATGGCGTTTTTGAACGCCACCGGCCCGAAAACCATCAAAGCCCTTCGCG
CCGCCATGATACAGATCGGCTTCCCAGCGCAGAATTTCGCCTACAAAATCGGCAAACTTC
TTCCAATCGGCACGAAAAAGCAAAAAGCCGAACCCAAGGCAACCGTCGGCAAAGCCCCGA
TTAAAGAACAGGTTATCCATTTCATCAACCGCCCACTGCCCAAAAACGTACCCGCCAAAA
CACCGCGCTCCTTATTGGGCATCGAAGACGGCAAAAGCATCCCCATCATCCGCAACCCCG
CCGCGCCCGAAGATGCCGAAGCCGTGTTCTACTTCCCGGGTTGCGGCTCTGAGCGTCTGT
TCAGCCAAATCGGACTTGCCGTTCAAGCCATGCTCTGGCACGTCGGCGTACAAACCGTCC
TGCCGCCCGGCTATATGTGTTGCGGCTATCCGCAAGACGCAGGCGGCAATAAGGCAAAAG
CCGAAGAAATGAGCACCAACAACCGCGTGGCTTTCCACCGTATGGCGAACACCCTCAACT
ACCTCGACATCAAAACCGTCGTCGTCAGTTGCGGCACTTGTTACGACCAGCTCGAAAAAT
ACCGCTTTGAAGAAATCTTCCCCGGCTGCCGAATCATCGACATCCACGAATACCTGCTCG
AAAAAGGCGTGAAACTCGACGGCGTAAAAGGTCAGCAATACCTCTACCACGACCCCTGCC
ATACCCCCATCAAAACCATGAACGCCACCCAAATGGCCAGCAGCCTGATGGGGCAGAAAG
TCGTTTTAAGCGACCGCTGCTGCGGCGAATCCGGTATGTTTGCCGTCAAACGGCCAGACA
TCGCCACTCAGGTCAAGTTCCGCAAACAAGAGGAAATCGAGAAAAACCTCAAAGAGCTGC
CGCAGGGCGAACCCGTCAAAATGCTGACCTCCTGCCCCGCCTGCCTGCAAGGCTTGAGCC
GCTACGCCGACGACAACAATATGCCTGCCGACTACATCGTCATCGAAATGGCGAAATACA
TCCTCGGCGAAAACTGGCTGGATGAGTTTGTAAAAAAAGCCAACAACGGCGGTGTAGAGA
AAGTGTTGCTGTAACAACGGACACGGAAATGCCGTCTGAACGCCGAAAGCCTTCAGACGG
CATTGTTTGAACCAAATATAGTGGATTAACAAAAATCAGAACAAGGCGACGAAGCCGCAG
ACAGTACAAATAGTACGGCAAGGCGAGGCAACGCTGTACTGGTTTAAATTTAATCCACTA
TACCTACCCAAATCATGATTGACAGACAAACAAACGAACCCAAACAAAAAACCCGAATCA
TCCTTGCCCCTATGCAGGGTCTGGTCGATGACGTGATGCGCGACCTGCTGACGCGTATTG
GCGGCTACGACGAATGCGTCAGCGAATTTGTACGCATTACCCATACCGTGCATTCCCGAT
CCATATGGTTAAAATATGTCCCCGAAATCGCCAACGGAAACAAAACGTTTTCCGGCACGC
CTTGCACCGTCCAACTTTTGGGCAGCGATGCGGACAATATGGCGGCGAATGCGCTGGAAG
CCGTCCGCTTCGGTGCGAACAAAATCGATTTGAACTTCGGCTGCCCCGCGCCCACCGTCA
ACAAACACAAAGGCGGCGCAATCCTTTTAAAAGAGCCGGAACTGATATTCCACATCGTCA
AAACGCTGCGCGGACGTTTGCCCGCACATATTCCGCTCACCGCAAAAATGCGGCTCGGTT
ACGAAGACAAAAGCCGGGCTTTGGAATGCGCCTGTGCGATTGCCGAAGGGGGCGCATGCG
GACTGACCGTACACGCGCGTACCAAAGCCGAGGGTTACGAACCGCCGGCGCATTGGGAAT
GGATAAGGAAAATCCGAGACAGCGTCAATATTCCCGTTACCGCCAACGGCGACGTTTTCA
GCCTGCAAGACTATATCGGCATCAAAACAATCAGCGGCTGCAACAGCGTGATGCTCGGTC
GCGGCGCGGTCATCCGCCCCGATTTGGCGCGGCAAATCAAGCAATACGAGAACGGCGGGC
CGGTCAAAGACACGGATTTTGCCGAAGTTTCCAAATGGATACGGCAGTTTTTCGAGCTGT
GCCTGACAAAAGAGGCAAACAACAAATATCCGCTGGCGCGGCTGAAACAGTGGCTGGGTA
TGATGAAGAAAGAATTTGCAGCAGCACAAAATCTGTTCGACCGCGTCCGAACGGTTAAGG
ATGCGGACGAAGTTCGGAACATCTTGGCTGAATTTGAGCGAGAAATGAATACTTGAATAT
GTATAGTGGATTAACAAAAACCGGTACGGCGTTGCCTCGCCTTAGCTCAAAGAGAACGAT
TCTCTAAGGTGCTGAAGCACCAAGTGAATCGGTTCCGTACTATTTGTACTGTCTGCGGCT
TCGTCGCCGTGTCCTGATTTTTGTTAATCCACTATATCCGCTCCAAAGCAAATGCCGTCT
GAAAACCTTTCAGACGGCATTTGTTGTCTTTATTGCCGTTTTTCGTCCGTATCCGGATTT
TTGTTTTTCAGCTTCGCACCCAAGCCCAAACGCCTTTCATAATCCGATTGCGGAGTATCG
TCTTCCTGCATACCGAACGCGCCGGCATTGACCCACAGCGACAGCAGCGCGACGACAAAG
GCGCAAAAGCCAATCACATACCAAAACATTGCCCCTCCCGATTTGTTAAAATCATATCAA
ATACAGTGCCGAATTTATCACAAACGCACGGGCAAATATAGTGAATTAAATTTAAATCAG
GACAAGGCGGCGAGCCGAAGACAGTACAAATAGAGACCTTTGCAAAATTCCCCAAAATCC
CCTAAATTCCCACCAAGACATTTAGGAGCACCTTCTTCCAGCAAACCGCCCAAGCCATGA
TTGCCAAACACATCGACCGTTTCCCACTATTGAAGTTGGACCAGGTGATTGATTGGCAGC
CGATCGAGCAGTACCTGAACCGTCAAAAAACCCGTTACCTCCGAGACCACCGCGGTCGTC
CCGCCTGTCCCCTGTTGTCCATGTTCAAAGCCGTCCTGCTCGGACAATGGCACAGCCTCT
CCGATCCCGAACTCGAACACAGCCTCATCACCCGCATCGATTTCAACCTGTTTTGCCGTT
TCGACGAACTGAGCAGTATAGTGGATTAACAAAAACCAGTACGGCGTTGCCTCGCCTTGC
CGTACTATTTGTACTGTCTGCGGCTTCGTCGCTTTGTCCTGATTTTTGTTAATCCACTAT

# Appendix A

ACTTTATGCCGCTACCGCAACTGGCTGGCGCAAGACGACACCCTGTCCGAATTGCTCAAA
CTGATTAACTGCCAACTGACCGAAAAAGGTTTAAAAATAGAGAAAGCATCCGCCGCCGTC
GTTGACGCCACCATTATTCAGACCGCCGGCAGCAAACAGCGCCAGGCCATAGAAGTTGAC
GAAGAAGGACAAATCAGCAGCCAAACCACACCGAGTAAGGACAGCGATGCCCGTTGGATC
AAGAAAAACGGCCTCTACAAACTCGGTTACAAACAACATACCCGTACCGATGCGGAAGGC
TATATCGAGAAACTGCACATCACCCCCGCCAATGCCCATGAGTGCAAACACCTGTCGCCG
TTGTTGGAAGGTCTGCCCAAAGGTACGACCGTCTATGCCGACAAAGGCTATGACAGTGCG
GAAAACCGGCAACATCTGAAAGAACATCGGCTGCTGGACGGCATTATGCGCAAAGCCTGC
CGCAACCGTCCGCTGACGGAAACGCAAACCAAACGCAACCGGTATTTGTCGAAGACCCGT
TATGTGGTTGAACAAAGCTTCGGTACGCTGCACCGTAAATTCCGCTACGCTCGGGCAGCC
TATTTCGGACTGATTTGCGCCCGCTGCCGCCTAAAAGGCAGCCCGGATGCCTGATTATCG
GGTATCCGGGGAGGATTAAGGGGGTATTTGGGTAAAATTAGGAGGTATTTGGGGAGAAAA
CAGCTGAAAACCTGTGTTTGGGTTTCGGCTGTCGGGAGGGAAAGGAATTTTGCAAAGGTC
TCAGAGTGAGTTTATTTTGGGGCGGCGGCAGGTCGGGGGCAAGCGGCGTGGGGCTTGGTT
GTGGTTTTTAGGTTTTTGGGGGTAAAAAATGCCGTCTGAACTTTTCAGACGGCGTTTGTT
TTTTCTATCCAATCGAGGAACTGCCGCCATTTTTCCAGCGGCATATCGGCCCGACGGGTT
TCGGTATCGGGTTCGGCTTCCCAGCGGCCTACCTGTATGTAGTGCTTCACCCCGACGATT
TGCGCCAACTCGGCCTGTGTCAGTTTGCAGCGGTTGCGAAGGGTGCGGAGGTTGTAAGGC
GTGTAGCCGAGTTCCATGTCGTTGCGGTTTATTTTGTTTCGCATATTTTTTTGACTGCCC
GGCGGCAGGTTTCGGTAAGGATGGCGGCAAATCGGGCTTCGTCTGCCGGTTTGCCGTCGA
AAAAAATAAAATCGTAAAGGGTGATGCCGTTTTCGGAATGGCGGTAATGCCTGCCCGGGC
ATTCGCCGTCTGTCTGCCTGTGGATTTTGGCAATCAGGCGGGGATAGCGGCAATGGGTAA
TGAAACGGCTTGCGCCGTCTTGCCCGATAATCCATTCGGGGTAGCGGTTGAATAGTGCGG
CTCTGTTCATTTTGTTCGTGGGATAAAGCCCCTCGCGGGGCTTGTGGTCAGGCAAATTTG
AATATCAGTGCCAACACGGCGGCGATGGCGGTAACCAGCCCGGTGGCCGCTATCATGGGA
TACCAGCGGGACTCTTGGGCTATTTTTACGGATTCGGCGTTGATTTTGTGCGCGTCGGCA
ATGATTTTGGCGATTTCGGCATCTATTTTTCTCAGACTGGAGTGTTTCATTTCTTGTTCG
ATTATGTTCATCGTACTTCCTTTCGTTTTTGGCGGTTGCCGCCGCTTGTCGGATGGTAGG
ATGTCTGCCATGTGTATATATTGATACCTTTTAGGTTTTATTGCAAGTGTTTTGGGCGGC
GGCTTCGTATGCTTGGCGGTGGCGGCGGCTGTACCATTCGGCGAGTTCGCGCCGCTCTTG
GAGGCGGTAGCTGTCGGCGGTCAGGTAGTGGTGCAGGCTTGAGAAGCCGGCGCGTTGGAG
GGCGGCGCGGCTGATGTGGCCGAGGGCGAGGTCTGTTGTGAGGGTGTCTTTTCCGGCGGT
CAGGCTGATGTTGGTGAAGAGGTGGCGGAATCCGTGCATTGTGTGTTTGGATTTGCCGGG
GGTGCTGCCGTCATAGCCCAGTCGTCGGATGGCGTTGTGGGCGAATTTGATGCTGATGTG
GTCGGGATGGGGGGCGGGTTTGCGCCGTGGGCGGATGCCGGGGAAAAGGTGGATGTTGTC
GCCGGTCTGTGTGTGCAGCTCTCGGAGTATTTCTACCGCCCAGTCCGACAGTAGGACGGT
AAAGGGGTGTTTGGTCTTCATGTCGGCGGCGGGGATGTGCCATAACCGGGCGGTGAGGTC
GATGTCTTGCCAGCGGGCGGAAAGCAGTGCGGACGGACGGGATGACTTCCCCGGTTTCCC
CATCAATCCGCGCCGCCTCGATTTTTCCGTTGGCGCGCAGCATTTCCACCGCCTGCCGCA
CCGACATCAACCGCTTGCCGTTGCGCCTCATCGCCTCCACCAAAACCGCCGAAATCAATT
TGGCTTCTTCCGGTTTAAGCTCCGTCTTGCCCGCATCGCTGCGCCGTTTGCGCGTCGGCT
TGACGCTGACCGCCTCCAGCTTGCGGTATAGCGTGGCAAGGCTGATGCCCAATTCCTGCG
CCTGCTGCTTAAGATATGCAGAGCGTGCGCCGCGTTCCATTGCTTCCGCCTGATTCTCGA
CTGCCTTAAGACGCTCAATCATTGCCGGATTCATCGCCTTCTCCCGTTTCACCGCCCAAC
CATTCCGGCACATTGTCTGTCGGTGCTTCAGTCGGCAGGGCATAGCTTTCGCGCAGTTGC
TCGCAGTCCAAAATAATTTGATTGAGCGTGCCGACCATCTTTGCCTGATGGCTGATCCCG
TGTGCCTCACTGTGCGCATTAAGTTGGTCGAACAAATCTTTCAGACGGCTCACTTGACTG
CGGATACCGACCTCAAGGCTTGTTAACTGCATCGCCAACTCGCTGCCTACGTCTTCCGCC
TTCGGCTCTCTGACAACGGTTTGGTTCTTAGCCAGCTTCTCGGCCAGCTCATCAATTTTG
GCTGTTTTGGTTTTCATCACTTCGTCTTTGGCGGCGAGGTTTTCGCGGCTTTCGCGCAGG
GCGACGCGCAGCTCGCGCACCGTCATTCGGTCCACATCGTCAAAGGTCATGCCGTTGACT
TCTTCCCCTTCGGCCAAACCCACCAGCGTAACGTCTTCTTCGACCAGCAGCTCAAGCAGC
TTCGACTTGCCCAAATCCATCAGCTTCGGCGCGGCTTTCTGCATTTGCGGGGTCGCAAAG
CGGCGAGTGGCTGACATCAGACGTGATGTTTCTGCGATACCCAGCCCAAATTGGCTTTTC
ACAATTTCCATAAACCGTCCGTGTTCCGTATGCTCTTTTAAAATAATCAGCGCACGTCCC
AGTTCGAACATGCCTTCCATCGTCTGCCGTACTGCTTGACGACCGCGTTCAACCCAACGC
TCTTCGCTATAAGTCTCGCCGTTACCCCACTGCTCCATCACCAGCACACTGCGCATCGCT
GCCTGATTGCTTACTTTGTCGGTTGCGATAATTTCAATTTCTGTATTCATTTTTTATCTC
CAAAGTTTCCGACGTCGGAAACTTTCAAAATCCGTTAATCCACATCGACCCGCTTGCCGA
TTTCGGCAATCTTGCTTTGCAGCCGTTCATGCTGCTGCCTGAACCGCTCTGCGATTTGCA
GGGTTTTGATGCCGTAGGCGTAGTTGCCGTTTTCAAGTTTGATGACCAATCCCGAGGCAA
CCAAATCATCAATATCCCTGCTGACTTGCGATGGCGTCAGCCCCAGTCCGACCGATAAAT
CCTTATTGCTCAGACCGATAATCGGATGCTCGTCAAGCGCGATAAAGACCCTCAATAGCC
GTTGTACCCTTTTACTTTCTGCCATCCGCATCCTCCTTATTTCAGTCCCAGCTTCTTGGC
AATTTCGTGCCCCTTGCCGTAATTGCCTTTGCGCTGTCCGCCGATCACCAGATACACATC
GCGCGGCTTAAAGCCGTTCTCTCTTGCCCACTGCGCCAGCGTCTGGCCGTTTTTGGCAAA
ATTTTCTTTCAATTCGTCTGCCGTAAGCATTTCTTTGGCCTGTACGCGGGAAAATTTCAG
TATCCGTCCGTACGGTTGGTTAATACACTCATGATTAATCATCGCTTTTCTCCTTCATCC
CCAGCAAAACCGCCGCTTCGTGGCTTTTGCCAAAATTGCCTTTCAGCTTGCCGCGCAAGA
GGTGCTCCACCGTGGTGCGCTCCAGATTGAAATATTTCGCCCAATGCGCCTTGCACACCC
CGTTGCGCTTAAACCACCCGGCCGCGCTCTCGCGCGTTTGCGGATAGGAAATAGGCTTGA
AATTCAAAGGTTTTTCCATATTATTTATGCCTTTCGTGTGATATAATGTGTTTGTTTTTA
GGTCTCTTTGCACGATAAACATCCGGTCTGTTGTGCAGCAGCAGGGTCTTGGCTTGTTTT
AAAGTAAACAAGCTTTTTCTCGTAGCTGGGTGTATGTGATCCATCATGAGCTGTCGCGCA
TGAGCCTTCAAAGTGTTCATTTTTTCGTCCTTTCTCGTGATGATTTAGGGTGTTTGTGTT

## Appendix A

TCGATGTGGAAATTATAGGAAGAATTCTTCCTATTTTGCAAGGAATATTTATGAATAACT
CTTCCTTTTTTGGCAACCGATTGAAAGAAGAAAGAAAAAAATTAAAAATGACTCAAGCTG
AAATCGCTGAAAAATGTGGGGTTTCAGGAAGAATGTGGGGGATTATGAACGTGGCATCA
GCCAGCCAAAAGCGGAACTTTTCTTCCAATTTGAAAAGGTGGGTATAGACGTTCAATACG
TCATGCACGGCAGACGCGGCGAAACAGCGGTCATGCCGTCTGAAACCCTGAACGCCGAAG
AACAAGAACTGCTGGTCTTGTTCCGCGAGGCGGCAGCTGCCGACCGTGAAATGATTCTGA
TGGTTGCGCGCAGGGCAGAGAAAAAAGCCCAAACTGCGCTTGGTAAAGTGAGTAATGGAT
AAAATGGATCAATTCGAATTGTGCCAAAAAGAACATGTCAATCCATTTGCCTTGTCAAAG
CAATATCTATTGGTTGTAACATTCGTCAAATCCAGCAGTAAAAATTTTCAGGCAGCATTA
CTTTGGGCAAGAAGTGCCAAATTATTTGAGAATCTTGAGATTGGAAAAGAAACCATCTAT
TGCTGCGCTTTCGATAAAACAGCAGAACAGGCTGGGATGGCCGGGGTATTTTTGAATTAT
ATTGAAAATTGGAATGGCAAACAGATTTACATCAATGGCCGAATCCATAGTGGCAGTATT
TATGATTTGTTAGGGGTTTTAGACTGCTATCAAAAATCACAGTCCTGTCCCAACCCTAAA
AGCCACTGTTGCTTTGTTTCAGACGACATTTTTCTATGGCATGGATCAAGACCAACGTTT
GAAATCAGTCTTGATCTAACTGGAAAGAAAAAAGAAACATCCTCTGCAAAGAAATTTGTG
ATGCCTTGTATTAATTTCCGTCACCATAGGATTGAAAAAGAAACCTACTTAGGAAATTGG
AATGAACAAATTGCCGCATTGGCAGTAAAACAAAATATAGATTGGTGTCCAAGTTTTGAT
ATTGAGAATTTTAGACAGTATGAATAATTACTATCTATATAGGAATTGCAGCAGCGATGT
GTTATGGGTCAAACGTATCCAACGCCAAATCGACGGCAGCCTACTCTTGATTTCTGACAA
TTCAACCTATCCACCCATGCCCTTGGCACTGGCGGAACACCCCGATATTCAAATCATCGG
GCAGGTAGTGCAGGTATCAAAAGACTTGAACTAGACACAATCAAAAAGGGAAATAGAATG
AAAATACTCGCTTTATTAATTGCCGCTACCTGTGCTTTATCTGCGTGTGGCAGCCAATCT
GAAGAACAACCGGCATCTGCACAACCCCAAGAGCAGGCACAATCCGAATTAAAAACCATG
CCGGTAAGCTATACCGACTATCAATCAGCAGCCAATAAAGGGCTGAATGACCAAAAAACC
GGTCTGACCCTTCCTGAACATGTTGTCCCTATCGACAATGCGGAAGGAAAGAATCTGCTG
CATGACTTTTCAGACGGCCTCACAATCTTAACCGTTGATACCGATAAAGCCGACAAAATT
ACTGCTGTCCGAGTAGTCTGGAATACAGATGCAATGCCTCAAAAAGCGGAAAAACTGTCC
AAAGCTGCCGCAGCCTTGATTGCGGCAACCGCTCCGGAAGACCGCACAATGCTGCGTGAT
ACCGGCGACCAAATCGAAATGGCGATTGACAGCCATAATGCGCAAAAAGAGCCAACCCGA
GAATGGGCGCGTGGTGGGATTGCTTATAAAGTCACTGTTACCAATTTACCGAGCGTGGTT
TTGACGGCAAAAGCTGAGTAAATCTATTAAGTAGAAAAAATAGAAAGGGAAATGATGATT
GAGAAAAGTATTTCTATTGTAGATGGAAAGGAATACTCCGTTTTTGCTGTATCACACGAG
TTTCGTTATACCTTTGATGAGCCTATTTTAGTCGCTGACTTGATTAGTTCTCTAAAAGCT
TATGAAACACTGACAAGTAGTTATCTTCCAGCAATTTTGAATCAGCTGTTTGATGTCAAA
ATCCAAAAAATCAAAGTAGCTGTATCTGAAATTGAAAGAGGATCTTTCCTTGAAAAACTG
ATTTTCAATTTATTCTTCAAAGATGAAGATGCTTATAATGAATTTTGTCTTAAAATACGA
AAATTTCTAGGAACAGAAAATCAGGACGGAAGTATTAATATGTCCAAAATCATTATGTTT
GCAATGACTACACTTTTAGGGGTAGGTGCTGGTTATCTCTTGTTTAAAAACCCGCCACAA
GAGAAGCAGGCAATAACCAACAACATCGTTACCGTCATTAATGCTGATAGTTCTGTCGCA
CTGGATGGTGAACATTTGGTTTCAGTGGTAAAAGAAGTAACAGGAAGCAGCAAGCAAAAA
ACTGCAGAAAATGTGGCAAAAGTATATGCTCCAGCAAGTAAAAATAATGGCAGTATTACC
CTTGGGACAGATGATGTTCGGATTGAACCTGTTGCACAACAAACTGTAGCAACTTTGCCT
AAAGATGTGGACTTACGTGATACGCCATTGACTGAAGATTACACCGATATTGATGTGCAA
ATTCGTGCTACTGACCGTGATAAAAATTCAGGGTGGTATGCAGTCATAGACCAAATTGTT
CCATCACGTGTTCGATTAGAACTGCCTGAAGATATTGATTTGAATAGGCTGGCTAACAAT
GCTACTATCCGTGCAAATGTAACAGTTGAGTTTGACTTAAAGCAAAATGGCTCTCGTAAG
CCTAAAAAAATCATCCTCACATCTCTCTCTACTGATTAAGTTTTAACCCGTATTAAAGGC
TTAGTCAGACGGCCTTTCCTACAATCCCTGTATTGATTTTTAATTCAATACAGGGATTTT
TCCATGTCAGACAAGTTCAACCAATTCATCAACCGCGTCCTCTCTCACGAGGGTGGTTAC
GCCAACCATCCCAAAGACCCCGGCGGCGAAACCAATTGGGGCATCACTAAGCGCACCGCA
CAGGCAAACGGCTACAACGGCTCCATGCGTGCCATGACGCGTGAACAGGCAATCAGCATT
TACCGTAAAGCGTTTTGGGAGCGTTACCGCGCCGACCAAATGCCGGAAGCGGTCGCGTTC
CAATTTTTTGATGCCTGCGTCAACCACGGTTACGGCAATGCCGCCCGTATGCTGCAACGC
GCCGCAGGCGTACCGGACGACGGCGTTATCGGAGCAGTCAGCCTCAAAGCCATCAATTCC
CTTCCCGAAAACGACCTTTTATTGCGGTTCAACGCCGAGCGTCTGGTCTTTTATACCAAG
CTCGGTACGTTCACCTCTTTCGGCAAGGGCTGGGTACGCCGTGTGGCGCAAAACCTGATT
CACGCGTCTGCAGATAACACTGATTAAAGGGAGATAAACCATGTCAAAAAAGTCACTCAT
CGCCCTAATGACCGCAGCCATGCAGCCCGATTTCAGCCACAGCGACCTAGGCATTCGCTA
CGCCATGCCGACTCAGGGATGTTGGACGCAAGCCCACCGCAAGAGCGGGGTAGCCGCCGC
GAAACGCGCAGCCAAAAAAAAGCGTCACAAATAACCGCCTTTTTCCGATGGCTGGGCGGC
TTGGTCTCTAATCCGGCCACAGGAAAAATCAGCCATACCAAACTATGGGCAAACGTCGCC
GCAGCCGCCATGACTTGGAAGTTCGTGCAGGCGGCGGACGCGCCCGAATGGCTCTAGTGG
GCTTATGGCGCATTGGTCGGCGGGTATGCATTAATCAAACGCGGCATCGCGGCGATTCCG
CAGTTGGCAGAAATCAAAAAATCCGCAAATCAGGAAGGGGGGCGGCAATGATTGAATTTG
TCCGAGCCAAAAAACGGCTGCTTTGGGCATTTGTGCTTTTGCTTGTGTGGACGTGCGGTT
ACCGATACGCCGCCGACAAGGCCGAAGCGAAACAAACCGCCCTGATTGCCACCTATCGGC
ATTCTTCTATGGTTGCGGCGGAACAATATGCCTTGCAGCTTAAAAAAGCGCAGGACGAAA
GGCAGCGGTGGTACGACTTTTCCCAAAAACAAGGAAGAAAGCCCGTGAAAAAACAGTATC
CGCCGCAAACGAAAAAAGCCGGCTATCTGAAAACCAAGGAAGAACTGCTTGCGGAATTGG
CTTGCCTTAAAGCGGAAATGGTTGCCCTAAAAAAGCCCGATGCCTTAATCCATGGGAAAG
AAGTGCGGCAGAAAGAACGCAACTCGTCGCAGGGTTAAGGCAATGCCATCCGTTGAACTG
CTGTTGGAGATTGTCCTTCTATTACCAATTGGCCGTCCAATCGGCAGAAGACAAATATGC
CGATTTGAAACGGCATATCCATGATATTTATCGACGACATAAGGGAAGATACGGCTACCG
GAGGATTGCGGCAGCCATCCGTCACGCAGGAACACCGGTCAATCACAAGAAAGTCAGCCG
TCTGATGGCGAAGACGGGGCTGAAGGCAGTGATACGGCGGCGCAAATACCGCTCGTTCAA

## Appendix A

AGGAGAAGTCGGCAAAATTGCGCCGAATATCCTGCGACGCTGTTTCCATGCAGAAAAGCC
GAATGAGAAATGGGTAACGGACGTTACCGAGTTCAATGTAGGCGGAGAAAAGATATACCT
TTCTCCGATTATGGATTTGTTTAACGGGGAAATCGTCAGTTACCGTATTCAAATCCGCCC
GACTTTCGATTTGGCCGGCGAGATACTGAAAGGTGCGCCGGAGAAACCGGGATCGTCTGA
AAAGCCGATACTGCATTCGGATCAAGGTTGGCAATATCAGATGTTTTTTATCAAAAGCAG
TTGAAAGGCAACGGTCTGGTTCAGAGTATGTCCCGCAAGGGAAACTGCTTGGACAATGCG
GCAATGGAAAGTTTCTTCGGAACGTTGAAATCGGAATGTTTCCATACGTGCAAATATGAT
TCCGTTACCGAATCGTAAGCGGCACTGCACGAATATATCCGTTACTACAACAACGATAGA
ATCAAGTTGAAATTAAAAGGACTGAGCCCTGTTCAGTACAGAACTCAGTCCCTGAAAGCC
GCTTGATTAAACTGTCCGACTTTTTGGGGTCAGTTCGGCTTCGGCATTTTTTTATCCGTT
TTGGGGGTAACTTGTTTGGAAAGCTGCAAGCTTATAAATAAAGGATTACATTTAAGTTTT
GGGTAACCTTTTTAAAAAAATGCGTGATGACTTTTGCATTTTTAAGGCGTTTTTTGGGGT
AATTCGTGAAAAGTTACCCCAAAAGTTACCCCATAAATGGCGAAAACTCAAGCATACGCC
AGCATCCTGCAACACAAAAAAGCCTTGAAACTGTTGAAGTTCAAGGCTTTTTTGTGTTGC
AGGATGCTGCTGAAAATAGGGTATGGTGGAGGCGGGGGGAATCGAACCCCCGTCCGAAAG
TCCTCTACAAAGCGTTCTACATACTTAGTTGTGTCTATTTGAAAATCTTATTTCCATCAT
GCCGACCAACAGGCCTTTTGGAAACCAGTTACCTTAAGTCTTATTTCCTGCCAAGTAACC
CGGTAGGAAACCAGTCAATGTAAGATGACGTTGCGGTGGCTTTCGCCACACAGCCCATTG
ACCGACTGCTGCAACGGCTAGCCTTAAGCGGCTAAAGCGTAAGTTTCGTCGTTTGCGACT
ATTTGAATTCAGTGTTTTACGGGAATCTGAGACCCCGGTATGCCCGCATCTGCTTCGCAA
CCCTCGTCGAAACCAAGGTCGCCCCCAGAAATGGTTTGCAAATTATACGGATATTGTGCG
GTGCTGCCAAGTCTGTCGGAGAAATTTGTCAGTCTTGCTGCCTTAATTTGCGTTTGAGCA
GGATGCGGACGCAGCCGTCGTTGCCTTCCCGGGGTTCGGCGTAGGCGAGTACGTCGGGGT
GTTGCATCAGCCAGTTTCGGGTCATATTTTTCAGAACGGGTTTGTAGCCTTTGGAACCTA
ATCCGCTGCCGTGGATGATTTCGCCGCATACGCCGCGTTTTTGGGTGAATGCGATGAATT
CGTTGAGGACTTTTTGGGCTTCTTCCTGTGTGTAGCCGTGCAGGTCGACATCGGTAACGA
CGGGATAGTATCCGTTTTTCAGGCGTTGGATGTCGTTTTTTCCCTGTCCGTTTTTGCTGA
AGCTGGCGGGCGGGTCGTTGTATGTGCTGCCGATGTAGAAGTAGTTTTCTTCGTCGGCGC
GGTTGTCTTTGGGACGGACTTTGATGGGGGTTTTGTCGGGCGGCGCATAATATTGCTGCC
GGTTTTTTAATGGGGAGAGTTGTCCGACTGCTTGTGAAAAATCGAAATCCTGTTCTTGTT
TTTGCTTGTTTTTTTCGGCTGCCTGTTTTTCTGCCGCTTCTTTTTGGGCTTGTTTGCCCA
GTTGTTTGAGGATGTTTTGGAAGTCGGTATTCATATTTTTTCCTGTTATTTGTCCGATGG
CTGTTTCGGGCGGGGTTTTAATTTGCCGGAATGTTTGCCAATCGGGGGAGGATGATTTTG
TTGCCTGCGTATGTTTTTTGAAAGTGTGATTGTATATCAAAAAGAAATGCGGCAACCGTC
GGCAGTGTTGATTGCCGGAAATGCGGACCGGTCGAACCGATATGCCCGAACGCCTGATAA
AGTTTTAAAAACCTGCCTTGCGAAGCAGGCTGACGTGTTTTGCCAATCTTGAATTGCCGG
AAACGCGAAACACGGAAATCTGATGTTTTATAGTGGATTAACAAAAATCAGGACAAGGCG
ACGAAGCCGCAGACAGTACAGATAGTACGGAACCGATTCACTTGGTGCTTCAGCACCTTA
GAGAATCGTTCTCTTTGAGCTAAGGCGAGAACGCTGTACTGGTTTTTGTTAATCCACTAT
AAATGTTCCGATACGAACTGCAAAATATTGGTTTTGTTTCTGACAGGCAAAAGCACTGTT
TATTTGGCTGTCAAAAGGATGGTTAAGGAAAGTTATGCGCCCCTGAAGCGGGCCCCAGAT
AAGGATGGTTGCGCCGACGGCTTCAGACGGCATTTTGGCGGCGGTGTTGGGTTTTGTATC
CGGTTTGCCGTTGTGTTTTGTGATGATGATTTTGGGCGCGGTTTTTCTGTTTTGATGTGT
GAAATGCCGTCTGAAAGGCGGTTCAGACGGCATAGCGGTCATTTTTGTGCGGTCAGGCGG
TCGAATATGCCGCCGTCGGCGAAGTAGGTTTTCATGATGTTGTCCCATCCGCCGAATTTT
TTTTCGGGAGAGAAGGTGTCTAAGTCTGGGAAGTCGGCTTTGTGTCTTGCCAATACTTCG
GGGTTGCGGGGGCGCAGGTAGAGTGAGGCGGCGAGTTCTTGCGCCGGTTCGCTCCAAAGG
TATTCGAGATAGGCGCGGGCGGTTTTTTGCGTGCCTTTTTTCGCGACGACGCTGTTGACG
AGGGGGAGGGGGCTTTCGGCGGAAATGGTGTAGCTCGGATAGACGATTTCAAACTGTCCT
TGGGTCAGTTTTTTGCTGACGTAGTTGGCTTCGTTTTCAAAAGTGATGAGTACGTCGCCG
ATGTTGCGTTGTGTGAAGGTGGTGGTGGCGGCGCGTCCGCCGTTTTCAAAAACGGGGGTG
TTTTTGAGGATGGATGCGACGAGTTTTTGGGCTTCCTGTTCGTTGCCGTTGGTGGTTTTC
AGACCGTAACCGTATGCGCCGAGGAAGGCGTAGCGTCCGTTGCCCGAGGTTTTGGGATTG
GCGATGACGATGTTAACGCCGTCTTTGGCAAGGTCGTTCCAATCGCGGATCTGTTTGGGG
TTGTTTTTTCGGACAAGGAAAACCATAGTGCTGGTGTAGGGCGCGGCGTGGTCGGGGAGG
GCTTGTTGCCAGCCTTTTTCTACCAGTCCTTTTTTTTCGAGCAGGTCGATGTCGGAGGAT
TGGTTCATGGTTACGACATCGGCTTGAAGGCCGTTGGCTACGGATAATGCCTGTTTGCTG
GAGCCGCCGTGGGACTGTTGGATGCTGACGGATGTGCCGGGGTGTTCGGATTGGTATGTT
TTGATAAATAAGGGGTTGTATTCTTTGTAAAAATCCCGTGCCACATCGTATGAGGCGTTG
AGCAGGGTAATGTTTTTTCCGTCGGATTCGGTATTGGCCGGGGCATTTTGTCCGGACGGA
TGGTTTGAATCGGCTGCGGGGCTGCAGGCGGTGAGCAGGGCTGCGGTATAGAGTGCCGGT
GCGTAGGTTTTCATATGCTTGTCCTGTCGGTTGGTAGATGGGGCAACTTTATACGGCTGT
CTGCGCTTGTGGAAATAATGTTTGATTTGAAGATTATCAGTTTTGGTTATAAGGACGGAT
CAGAGGTGTTTCCGCATCAGTTCGCATTTGATTTTGATGCTGGGGTCAAGCTGCAATACT
GCCGAACCGAGCGATTCGTAGCGGTTGAGAAGGTAAAACGGGACGGAGTTGAGCGATGCG
TAGAGTTTCAGAAAGCTCAAACCGGATTTGTGGGCGATGGTTTCCGCCTGATGAAGTAGG
GCAGTGCCCAGTCCGAGGTTGTGGAACAGGGGGTGGACGTAAAGTGCATCGAGTTGTGCT
TCTTGGCAGTCGATTTGGAAAAATCCCTGTATGTTGCCTTTGTATTCGGCAACCCAAAGT
GCTTTGTCGGGATCGGAAATGGTCGGCAGGTAGCTTTCTGTGTTTAGCAAGCCTTCCCAT
ACTTTTAGGGCGTGTTCGTTGTAGCTGAGGATGCAGGTGTATTGGACGGAGTGCAGGTGG
ACTTTGAAGATGTCTTTGCAGTCTTGCACGGTGGCGGGGCGTAACAGTGTCAGCAGGCTC
ATGGCGGTATGTCGGCGGCTTCAGACGGCATCTGTGCCGTTGGTCGGATTATAGGGACTG
ATGCAGTTTTTTTGCTTCTTGAAATGCGGTGTCCGAATCGGTGGTTAAAACGGTAAAGTG
TCCCATTTTCCGCCCTTTGTGCGCGGTTTTTTTGCCGTAAAGGTGCAGGTGTGCATTCGG
ATGGCTTTGCAAGGGCAGCCAATCCGGTTCGCCGCCGTCTTCCTGCCAAACGTCGCCCAA

## Appendix A

AATATTTGCCATACAGCAAGAACTCAGTAATTTGGTATCGGCAGGCGGCAGGTTGCACAT
AATGCGTACCTGCTGCTGGAACTGGTCTGCTGCGCAGGCATCTATCGTATGGTGTCCGGA
ATTGTGCGGGCGCGGGGCGATTTCGTTGACGACCAATTCATGCGTGTCACCGACAACAAA
CATTTCTACCGCCAATACGCCGACATAATCCAATTCGTCCGCCAAGCGTTGCGCCATCTG
CCGCGCCTGTTGCTGCACGTCGGCACTCAGTCGCGCGGGGACGATGGAATAAGCCAAGAT
GCCGTTTTCGTGGATGTTTTCGGCAGGGTCGAAAGTTTGCACGTTGTCATTGTTCAAACG
GCATACGATTACGGAAATTTCACTGCGCAAATCCACCATTTTTTCCAAAACGCAATCCAC
GCCGCCGTGTTCGGCAAACGCGGCTTTGAGTTCATCCAATGTTTTTACGCGGATTTGACC
TTTGCCGTCGTAGCCCAACGTAGCCGTTTTCAGGATGCCGGGCAAAAATTGCGCGCTTGC
TTCAGTGATGTCTTCAGCCTTACAAACCACTTGATACGGCGCGGTTTGCAATCCCGCTTT
GCGTATCCATGCCTTTTCCTGAATGCGGTTTTGTGCAATCGCCACACAATCGCCGCTAGG
GGAAACATTGGTATGTTTTGCCAAAAAGCGCATCGCATCGGCATTGACGTTTTCAAATTC
AGTGGTCACCGCCGCGCATTTTGCCAATTCGTCCAAAGCAGCTTGGTCGTTAAACGGCGC
GCACAAATGGCGGTCGGCAAATTCTGCTGCCGGCGCGTCCGGATCGGGGTCGAGAACGGT
TACTTTGTAGCCCATGGTTTTGGCGGCAACGGTAAACATTCTGCCTAATTGTCCGCCGCC
GAGGATGCCAAGCATGGCGGGCGGAGAAAGAGATATGTTTTTCATGCTGACTCTTCAAAT
TGTACAAGTTGATAGCTATAACTAATTCTTGACGGATGTCTTGTATCGCTGGAATTACCA
GTTTCAGAAATACAGAATACTTTTTCCATAAATTTTTCTGCTTTTAGAAATTCCAGTATT
CTGTTTTTTTCATCCTTATAAGCACCGCGGTCTGTACCCCATGCAAGAATAATCATATCA
GCATCTTCCAAACATCCTTTAAATTTGGAAAAATCGGTTTGGGTGTTTGCCCTAATTCCT
GTTTGCTGTGTAGAGTAACTGGAAAAAATATTTAACATTTTGAAGTTGGTAAAACCGTAC
ATATCCAAGAAACGTGCAAGTTGGGTAAGGGTTTTGTCGCTTCTTTCATCATTTGCTTTA
CTTGGATTAATTCCTATAGCGACAGCTGAGAAATTTTTAGGATTTTCTGTGTTGCCGCTC
CATCTAACCGTTAGGATTTCTCGATTTTTTTCATTATCTGTATAGAGACCGTCTTTGGTA
GTCGGTCTGAGTGTTTGGCGAAGCTCATAAAGTTTTTCATAAGTCATTTATCCAACCCTT
CCTGTACCATTTGCGCGGCGTGTATGACGGCTCGGGCTTTGTTTTGTGTTTCCTGCCATT
CGGAGGCCGGATCGGAGTCGGCAACCACGCCCGCGCCGCTTTGGACGTATAGCGTGTTGT
TTTTTACTACGGCGGTGCGGATGGCGATTGCCAAATCCATGTCGTTGTTGAAACCCCATA
CGCCGACGGCACCGCCGTAGATGCCGCGTTTGCTCGGTTCGACTTCTTCGATGATTTCCA
TGGCGCGGACTTTGGGTGCGCCGGAGAGTGTGCCGGCAGGGAAGGTAGCGGCGAGGATGT
CCATGTTGGTCATGCCGTCTTTCAGACGGCCTTCGACGTTGGAAACGATGTGCATTACAT
GGGAGTATTTTTCAATCACCATTTTGTCGGTAACTTTGACTTCGCCGGTTTTACTGATGC
GGCCGACGTCGTTGCGTCCTAAGTCAATCAACATGACGTGTTCGGCGATTTCTTTGGCAT
CGCTTAACAAATCTTGTTCGTTGGCAAGGTCTTCGGCGGGGGTTTTGCCGCGCAGGCGCG
TGCCGGCGATGGGGCGGACGATAACGTCGTTGCGTTCGCGTCGGACGAGGATTTCGGGCG
AGGAGCCGACGATGTGGAAATCGCCGAAATCGTAGTAAAAGAGATAAGGCGAAGGGTTGA
GCGTACGCAGGGCGCGGTAGAGGGCGAGCGGGCTGTCGGTGAATTCCATGCTCATGCGCT
GGCTGGGGACAACCTGCATGCAGTCGCCTGCGAAGATGTAGTCTTTGATTTTGTTAACGC
AGGCTTTGAACGGCTCTTCGCCGAATTCGCTGACGGCTTCGGTGTGTTTGCTGCCGAGCG
AGAGCGGGATGGCGCAGCTTTGGCGCAACTGGGTGCGGATGTCTTCGAGGCGTTCGCGGG
CGCGTTCGTAGCCGTCGGGCTGCGACGGATCGGCGTAAACGACGAGATGGATTTTGCCGC
TCAAATTGTCGATCACCGCCAATTCTTGCGACAGCATCAGCAAGATGTCGGGCGTGCCGA
GCGGGTCGGCTTTGGTGGTGTTTTTCAGGCGGTGGGCGAAGTGTTCAAAATTGTAGATGG
TTTCGTAACCGAAGTAGCCGACCAGTCCGCCGGTAAAGCGCGGCAGGCTTGGGATTTCGG
GTGTTTTGAAGCGGTTGTGGAAGGCTTCGATAAAGGGCAGGGGGTTGCCGTCGTGTTGCT
CGACAATTTCGCCGTTTTGATAAACATCGACGTGTTTGCCGCTGGCTTTGAGATAGTGGC
TGCAAGGCAGGCCGATAAAAGAATAGCGGCCGAAACGTTCGCCACCGACAACGGATTCGA
GCAGGTAGGTATAGGGGCGGTTGGCGAGTTTGAGATAGAGGGAAAGCGGCGTATCCAAAT
CGGCAAGGAGTTCTTGCACGAGCGGGATGCGGTTGTAGCCTTGGGCGGCTTGGGCTTGGT
ATTCTTGTTTGCTGATCATTTCTGCTTTCCCAAAGGGCGGTTTCGGACGGCGCGGCAACG
GGCGCGAGTATAGCATTTTATCGGAATTGTTGACAGTCTGACCGGAGATGCCCTTGGATT
CGGATTTCAAGTGCAACACTAGTGTATTAGTGGTTGGAACAGATTCAAGAATAAAACACT
TGGCGTTTCGTAGCCAAGTGTTTTTCTTGGTCGGTGGTTCAACTCATCTTGAACCCTGCG
TATCTCCCGATCACTGATGTTACGGAAATCGGTTTGTTTGGGGAAGTATTGCCGGATGAG
TCCGTTGGTGTTCTCATTCAGCCCTTTCTCCCAAGAATGGTAAGGGCGACAAAAATAAGT
CTCCGCTTTCAATGCTTTGGTTATTTTGGTGTGTTGGTAGAACTCTTTGCCGTTATCCAT
GGTGATGGTGTGCACCCTGTCTTTATGTGCCTTTAATGCCCTAACAGCTGCCCGGGCAGT
GTCTTCGGCTTTGAGGCTATCCAATTTGCAGATGATGGTGTAGCGGGTAACGCGTTCGAC
CAAGGTCAATAATGCGCTTTTCTGTCCTTTGCCGACAATGGTGTCGGCTTCCCAATCGCC
GATACGGGATTTCTGGTCGACGATAGCGGGTCGGTTTTCTATGCCGACACGGTTGGGTAC
TTTGCCTCTGGTCCATGTGCTGCCGTAGCGTTTGCGGTAGGGTTTGCTGCATATTCTGAG
ATGTTGCCACAACGTGCTGCCGTTGCTTTTGTCTTGGCGAAGGTAGCGGTAAATGGTGCT
GTGGTGGAGCGTGATCTGGTGGTGTTTGCACAGGTAGGCGCATACTTGTTCGGGACTGAG
TTTGCGGCGGATAAGGGGGTCGATGTGCTGAATCAGCTGCGAATCGAGCTTATAGGGTTG
TCGCTTACGCTGTTTGATAGTCTGGCTTTGCCGCTGGGCTTTTTCGGCGCTGTATTGCTG
CCCTTGGGTGCGGTGCCGTCTGATTTCGCGGCTGATGGTGCTTTTGTGGCGGTTCAGCTG
TTTGGCGATTTCGGTAACGGTGCAGTGGCGGGACAGGTATTGGATGTGGTATCGTTCGCC
TTGGGTCAGTTGCGTGTAGCTCATGGCAATCTTTCTTGCAGGAAAGGCCGTATGCTACCG
CATACTGGCCTTTTTCTGTTAGGGAAAGTTGCACTTCAAATGCGAATCCGCCGCCGTCTG
AAACGCCAAACGGGCTTCAGACGGCATTTTTGACGGCGGAGGTCTATGAGCCGCAGGTTT
TCGGCTTGTTCGCCAGAATATTGATGACTTTGCGTTCGGCTTTTTGCGGCTCGATTTTGA
TTTCGCTCTCGTCTTCTTCGCTGCCGTCTGAAAAACGTTCGGGCATTTTTTCGCTGTCAA
ACGCCAAATCGCCGCCGTGTTTCAGGCTTTGACCGCGTTCCAATCCGACAAAGTCGAAGA
GTTCGGTATCGGCAAGGTGGGAAGGACGACGTTTTGCAGGGCGGAGAACATCGATTCGA
TGCGGCCGGGGAAGCGTTTGTCCCAATCGCGCAGCATATCGCCGATGACTTGGCGTTGCA

## Appendix A

GGTTGGGTTGCGAGCCGCAGAGGTTGCACGGGATGATGGGGAATTGTTTTAATTCGGCGT
ATTTGATTAAGTCTTTTTCTTTCACATACGCCAGCGGGCGGATGACGATGTGTTCGCCGT
TGTCGCTCACCAGCTTGGGCGGCATGGCTTTGAGTTTGCCGCCGTAAAACATATTTAAAA
ACAAGGTGGCGAGGATGTCGTCGCGGTGGTGTCCCAAGGCGATTTTGGTGCAGCCCAATT
CTTTTGCAGTGCGGTAGAGGATGCCGCGGCGCAGGCGGCTGCACAGCGAACAAGTCGTTT
TGCCTTCGTCTAATACGCGTTTGACGGTGGAGTAGGTGTCTTCTTCAACGATTTTGTAGG
GAACGCCGATGCTTTCGAGATAGGTCGGCAATACTTCTTCGGGGAAGCCCGGCTGCTTTT
GGTCGAGATTGACGGCAACCAGTTGGAAATCAATCGGCGCGCTGGCTTGGAGCTGGCGCA
GGATGTCTAACAGGGCATAGCTGTCTTTGCCGCCGGAGAGGCAGACCATGATTTTGTCGT
CCGGCTCGATCATATTGAAATCGTTAATCGCGTCGCCGACGGCGTGGCGCAGGCGTTTGC
TGAGTTTGTTGTTTTCGAGTTCTTGTTTGGTTTTTTTGGACATGGCGGTTTGGGTTTGAA
AATTAGAAAGGCGGCATTGTAACCGATTGGCGGGGCGGCAATGCCGTCTGAAGGGCTTCA
GACGGCATCGGCGGCTTATTCTGCATTTTCGGTTTTAAAGAAGAGATGAACCGCTTTGAA
GATACCGCCGTTTGGGACGGTCAGTGTTTTTTGTGCGGCGGAGAATTTAATCACGGTAAG
GGCGGTAAAGTTTTCCGAATCTTGAACGCTGTCGAGTACGATGCCGGCCTCTTCGCCGTC
CGCCGTCAGCAGGGTTCCTGCTTCGACGGCCGAATTTCCCGACAATACCGCCAAGCCGCG
TTTGACCTGCCCCCGATACTGGGCACGGGCAATGATTTCCTGTCCCGGATAGCAGCCTTT
TTTGAAGTGTACGCCGCCGATGATGTGCTGGTTGAGCATTTGGGCGACGGCGGTTTCTTT
GGTAGCCGCGCATATCCACGGATAACCGCTACGGATTTCGTGCAGCCGCCACGCGTTTTC
GGCGGCGGCATCATAAGGCGGCAAGGCGTTTTTGGGGGCGATGTGCAAAATGCCCCGATG
TGGCAGGACGACGGAACAGATGCCGTCTGAACCGCATTCGGCGGTAAAGGCGAGGCTGGG
TTCTTGCGCGGCAAGCGGTTCGGCGGATGCTTCTAATTCCGCGCCGACGGCGTAATCTTC
AAGGATTTCAAAAACGGCTTTGGCGCGTAACACAAACATCCGCAAACGTTTGACCGTTGC
TTCAAGCAGGTCTTGCGCCATAATCAGCAGCAAATCGCCGCCTCGGTTGACGACAATCAT
ATTGGCGATGACGCGGCCTTTGGGCGTGTTGTAAGTCGCATAACACGCCTGCCCGGTCTG
AAGGTGGTTGATGTCGTTGGAAAGCTGTCCGTGCAGGAAGGTTTGGCGGTCTTCGCCGCT
GACGCGCACCACGCCGAAAAAGGGCAGTAAGGTTTTCATCATTTGCGTACTCTGAAATAT
AAAGGAAATCTGTTTATGCAGTTGCCGCGTCTCTCTTCACGGCGGTTATTTTGATTTCGA
CGCGCCACTCCGGACGGGCGAGCCGTGCTTCCACGCAGGCGCGGGCGGGCGTTCTGCCGG
CGGCAACCCAAGCGTCCCACACGCCGTTCATTTCCGCATAGTCGCCCATATCGCGCAGAT
AGATGACCGCATCCAAAACGTGCGCTTTGTCCGAGCCGCATTCCGCCAGCCAGCGGTCGA
TTTGGGCAAGCACGTCGGCAGTCTGTTCGGCAGCCGTTTCACCGTTTTCGGGAACCATGC
CGGAGAGGAAAATCAAGCCGTTTGCGCCGACGGCTTCGGAATAGCGGGGCGTTGTGCCGA
AATATCGGATATCCATATCGGTTTCCTTCGATAAAGGGGATATATGGTAACATTGCGCTT
GACCGATTTCCATGTTTTGCATGACGAAAAATGAGTAAACACACTTATCCGATAACACCT
GCCGTGCGCGTTTTGCGTGAAAACGGCATCGAATTTGAACCTTTTACCTATGCCTATGAG
GAACACGGCGGCACGGCGCAGTTTGCCCGACTATTCGGCAAAGACGAACACTTGGTCATT
AAAACCATTGTTTTGCAAGATGAAAACGGTAAGGGGCTGATTGTCTTGATGCACGGCGAC
AAGCAGATTTCAACCCGCAATCTGGCGCGGCATTTGGGTGCGAAACACATCGAACCCGCC
ACGCCCGTACAGGCAAACAAGTGGACGGGCTATCTGGTCGGCGGCACAACGCCGTTCGGC
ATCCGGACAAAGTTGGATATTTACGTCGAACAGTCGGTGATGGATTTGGAAACCATCTAT
ATCAACGGCGGAAAACGCGGGTTCATTATCGGCATCCGTCCCGGAGATTTAAATATTTTG
AACCCGAAAACAATACAGGCGGCGGTTTGACGGGAAAGTATAAAGGAACAATATGGACAA
AGATTTGTATGCCGTATTGGGCGTGTCGCCGCAGGCGGGAGCGGACGAAATCAAACGCGC
CTACCGCAAGCTGGCGATGAAATATCATCCCGACCGCAATCCGGGCAATCCGAAGGCGGA
AGAAAAGTTCAAAGAAATCCAACGGGCTTACGATACGCTTTCCGACCTGTCGAAACGGAT
GCAATACGACGCGTCCTTCAGACGGCATGAGGAACGCGGGCGGCAGGAAGAGGCATTCCG
CCGCGAACAGGCGCGCAGGGAGCAGTTTTACCGCGAACAGATGCGCCGCGAACAGGCGTT
CAGACAGGCGTTTGAACGGCAGGCATCACGTTCGTGCCATACTTACGAACCGTCCGGCGG
CGGAAGCGGGCGCAACTATGTCCTCGCCGCCTACATCCTGTTCGGTTTGGGTGCAATCAT
GCTGTTCATGCCCATAGTCGGCGTGATTTTCGCCTATATGCCCATAGTCGGCGTGATTCT
CGCCTATATGAAACGGAACAGTTTGGACAGCATTGTCTATGCCGCACATACCGAATACCT
GATTAAAACCTTTTGGCGCACATTTTGGCTTTATATTTTGGGTGCGCTGACTGCCCTTTT
GGGTATCGGCGTGCTGATTATTATTGCAACGAACGTCTGGTATTTCTACCGCATCATCGC
CGGCTTTATCCGCTTCAACGGCGGCAGGGCGGTTGCACCCGAGAAATGGATATAGTATGG
CTTACCTGTTAATCAGCATCGTGTTCAGCGTGTCGGTTTCCATTTTGCTGAAAATGGCAA
GGAAGAAAAAAATCGACATCGCGCAGGCGGTCGCCGTCAATTATGTGGTCGCGGTCATAC
TGACCCTGCTGGTATTGAAGCCGGATATCGGCAATATCGGCGCATTTTTGCCGACGTGGC
CGCTGTTTGCCGCTTTGGGCGTGCTGCTGCCGTCCGTATTCGTGATAATGGGCAAATCTG
TGGAAGCCGCCGGTATCGTCAAATCCGACGCGGCGCAGCGTTTGTCGCTGTTTTTGCCGA
TTGTTGCCGCCTTGACGCTGTTTGGCGAAAAACTCAGCGAAGGCAAACTAATCGGGCTGT
GCCTCGCATTTGCCGCACTGTTCTGCCTGCTTTGGAAACACAGCGGTGGCAAAAAATCAG
GAAGCGCGTGGCGGCAGGCGGCATTGCTGCTGGGCGTGTGGGCAGGTTACGGCATTATCG
ATATCCTGTTCAAACAGCTTGCCAAAAGCGGAACGGCATTTGCGGGCAACCTGCTGGTTG
CATTTGTGCTGGCGGGTGTGCTGATGTTTGCCTGCCTGTTTGCCAAATCGGTCAGATGGC
GTGTTGAGAGTGTGGTCGGCGGCATATTCTTGGGCGGTTTGAATTTTATGAATATCGTAA
CCTACATCACCGCGCACCAAATGATGAAGGATAATCCGACCTTGGTTTTTGCCGGTATGA
ATATCGGCGTGATTGTTTTGGGTACGCTTTCGGGCGCATTGTTCTTTAAGGAAAAAATCA
ACACAATCAATACGGCGGGAATCGTGTTGGCACTGTGTTCTATCGCCTGCCTGTTTTATT
GGGGGGAAGTCAAGGTACTGTTCGGCATATAATCCGGCTGATTGATATAACAAAATGCCG
TCTGAAGCAGCATCCCTGCTTCAGACGGCATTTGTCTGCAACGTTACAGATGGGGGTTCA
TCAGGTTCTCGGGAGAGAGGATGCGGTTGAGTTCTTCTTCGCTCAACAGCCCGCGTTCCA
AGACAACCTCGCGCACGCCTTTGCCGGTTTGGGCGCAGATTTTGCCGACCAAATCGCCGT
TGTGGTGTCCGATATACGGATTCAGATAAGTCACCAAACCGATGGAGTTGAAAACGTAAC
GTTCGCAGATTTCGCGGTTGACCGTAATGCCTTTGACGCATTTGTCGGACAGGTTGACTG

## Appendix A

CGGCATTGCCCAAGAGGGAAATGGTTTCAAACATACATTGGGCGATGACCGGCTCCATCA
CGTTTAATTGCAGTTGCCCGGCTTCGGCGGCGAAGGTAATCGTCGTGTCGTTGCCGATGA
CTTTGAAGCAGACTTGGTTGACCACTTCGGGAATCACGGGATTGACTTTGGCGGGCATTA
TGGAAGAACCGGCCTGCAATTCGGGCAGGTTGATTTCTTTCAAGCCGGCACGCGGGCCGG
AAGAGAGCAGGCGCAAGTCGTTGCAGATTTTGGAGAGTTTGACCGCCGTGCGCTTCAATG
CGCCGTGTACCATCACATATGCGCCGCAGTCGGAGGTCGCCTCAATCAGGTTTTCGGTCA
GTTTGCAAGGCAAGCCGCTGACTTCGGAGAGTTTTTTGACCACCAGTTCGGCGTAGCCTT
TGGGCGTGTTCACGCCCGTGCCGATTGCCGTTGCGCCCAAATTGACTTCCAACAGCAGTT
GGCGGGTGCGGTCGAGGTTGAGGATTTCTTCTTCCAACAACACTTGGAAAGATTGGAATT
CTTGGCCGGCAGTCATCGGCACGGCATCTTGAAGCTGGGTGCGGCCCATTTTCAAAACGT
CTTTAAATTCTTCGGCTTTGGCGGCAAAGGCGTTTTTCAGGACAGTCAGTTTGTCGAGCA
ATTCGCCGATGCTGTAATACACGGCAAGGCGGAAGCCCGTGGGATAGGCATCGTTGGTCG
ATTGGCTGGCATTGACGTGATCCATCGGATTGACGATGTCGTAGCGGCCTTTTTCGTATC
CCAAGACTTCCAATGCGAGGTTGGCGATGACTTCGTTGGTGTTCATATTGACCGAAGTAC
CCGCACCGCCCTGATACACGTCGGACGGGAATTGGTCGAGGCAGCGGTTGTTCAGCAGAA
CTTCGTCGCAGGCTTTTTCAATGGCGGCGGCGATTTCGGGCTTGACCGCGCCCAACTCAC
CGTTTGCCTGTGCCGTCGCCTTTTTCACCATCACCATACTGCGGACAAACTGCGGCACGT
CAGAAATTTTTTGTGTGGAGATTTTAAAGTTTTCAATGGCGCGCAGGGTGTGGATGCCCC
AATACACTTCGGCGGGAATCTCGCGGTCGCCCAATAAATCGTGTTCGATACGGACAGTCA
TGTTTTTACCTTTGTAAGTCGGATAATTAATATTGAAAAAATGCGCCATCGGAAAGATGC
CGCCGCAGGATGAACACTATACCGGCCGGATGAAATTGTCCATATCGTATGCCGTCTGAA
AACGGGAAACGTTGTTTTCGGGTGTTATAGTGGATTAACAAAAATCAGGACAAGGCGACG
AAGCCGCAGACAGTACAAATAGTACGGAACCGATTCACTTGGTGCTTCAGCACCTTAGAG
AATCGTTCTCTTTGAGCTAAGGCGAGCCAACGCCGTACTGGTTTTTGTTAATCCACTATA
CTTTCCGGACTTTCCGGCAAGCCCTGCCTGCCGCTGAAATATCTTTCGGCGGATTGTGCT
CCGCCATATCGGCTTACCGTTGGCGGGGCGGTTTGATGAAGACGGCACAAATGCCGTTTG
AAGGACGTTCAGACGGCATTTGTGCTGATTCGCATCAAGATTTATTGTTTGGCTGCCTCG
ATTTGGATGTCGATGCGGACGCTTTTGGTCATACCAACGTTAACGAGGTAGTCCATGCCC
CATTTGGTGCGGTCGATGGTGGTGCTGAAGTCGCCGCCACAAACTTCGGTTTTCTCCATC
GGGCTTTGGTAGCAGTTGAATTTTTCGGCTTTGAGTTTGACGGGGGCGGTTTTGCCGTGC
ATGGTCAGGTTGCCGTCAACGGAAACCAGTTTTTTGCCGTTGAAGTTGAATTTGGTGGAA
ACAAAGCGGATGTCCGGATATTGGGCGGCATCGAAGATGTCGGCTGATTTCAGGTGGTCG
GTAAAGTGTTGCGAACCGCTTTGCAGGTTGGCAATGGGGATGGTGATGTCGATTTTACCG
TCGCGTTTTGCTTGGTCGAACTCGACGGAACCGGTCAGACCGTAAAAACCGCCGACGTTG
GTGCTGGTGTTGAAATGGTCGATGGCGAAACGGGCGTTGGCGTGATATTCGTCCACTTTG
TAGGTGGCGGCGGAGGCAGTACTGATGGCGGCGGCTGCGAGTGCGGCGAAGATGATTTTT
TTCATGATGATAATCCTTTGTGTGGGCCGGTAAAGGCGTTTATCCTAACATAGGCAGGGA
TTTATGGCATTTCCTGCCGGTAAACGGTGGGTTTGCAGGCGGTTGACGAACGGGGCGGGC
GGAAAAGGGCGGATGAAAAAGCGCGGTGTGATCCGCGCTTTGTTTTTTACAAGGCGGCG
AGTACCGCATCGCCCATTTCGGAGCAGGAAACGAGTTTTGTGCCTTCTTCGTAAATGTCG
CTGGTACGCAAGCCTTGTTGCAGCACTTTTTGGACGGCGTTTTCAACTTGTTGCGCGCGC
GCTTCGTCGTTCAGGCTGTAACGCAGCAACATTGCGAGCGAGAGGATGGTGGCCAGCGGG
TTGGCTTTGTTTTGTCCGGCGATGTCGGGGGCGGAGCCGTGAGACGGTTCGTACAGGCCT
TTGCCGTTTTCGTCCAGCGAGGCGGAAGGCAGCATACCGATGGAGCCGGTCAGCATGGAG
GCTTCGTCGGAGAGAATGTCGCCGAAGATGTTGCCGGTGGCAATGACGTCGAATTGTTTG
GGCGCACGCACGAGCTGCATGGCGGCGTTGTCGACGTACATATGGGAAAGCTCGACATCA
GGGTACTCTTTGCCGATTTCTTCAAAGATTTCGCGCCACAATTCGGTGGTTTCCAAAACG
TTGGCTTTGCCTACGGAACAGACTTTTTTGCTGCGTTTTTGGGCGGATTGGAAGGCAACA
TGGGCAATACGGGGGATTTCGCTTTCGCTGTATTTCATGGTGTTGTAGCCTTCGCGTTCG
CCGTTTTCCAGAACGCGGATGCCGCGCGGTTCGCCGAAATAGATGTCGCCGGTGAGTTCG
CGCACAATCAAAATATCCAAACCGGCAACGATTTCAGGCTTCAGCGTGGAGGCGTTGGCT
AATTCGGGATATAAAACAGCAGGACGCAAATTGGCAAACAGGTTCAAATCCTTACGGATT
GCCAACAGGCCGCGCTCAGGGCGCAACGGACGGTCGAGGTTGTCGTATTGAGGAGAACCG
ACTGCACCAAGCAGGACGGCATCGGCTTTGCGGCAGAGGTTTTGCGTAAATTCGGGATAA
GGATGACCGTATTCGTCATAGGCTTCGCCGCCCAATGGGGCGTATTCGTAGCCGGCATCC
AAACCTTGGGCGATGAGTTTGTCGAGTACGCGGACGGTTTCGGCGACGATTTCGGGACCG
ATGCCGTCACCTCGGAGGATGGCGATATGTTTGGTCATTTCAAGTTTCCTTATGGGTTGA
TGGTTGAAGGGTTATTTCTTTTTGTATTTGTGTGCAATTTCGTGCCAACGAGGTATGGAA
ATCGATCGGTTGTAGTGTTTTTTATAGGCTTCCTCAAATTTCTTTTTCCATAAGGATGCG
TTATGCCGTGTTGCCGGGTTTTGATAAACGGTTTCTTCAATTGCGGAAACAAAATCTTCC
AAGCATTCAAACATAGGCATATTCTTTTTATTTATGCTGTACCAAACACCCGGGCGGATT
TTATGCACCACGCCTTTTTTGACTTGAAGATTAATTGCCCACCCATCCATGCTTCTCGTA
ATTTGCCATACTTTTTTAAGCCATAAATCCTTGATGGTTACATTGCCGTTGTCGTCCATA
TCGTAACCGAATATTAAATAGTCTACATCCAGCATATAGGGTTTATGAATGATTTCATCA
GAATACATTTTAAAATCTGCAATATCAAAACCCGGACTGGCATTTCGGTTGAACGCCTTT
ACTTCCAACAATTCTCTACTGCGGTCTTTTTTATTTAAAAAGAAATCGGGGGGCATTTGG
GTATTGGTTGAAACATCAAATTCAATTTCCCTTTTTCTCAACCATCCGCCGAGCCATTCC
TGAATGATGTTGCCGACGACATCTTTTTGTTTGACGATAATATCCACATCGCCCAAGAAA
AATCTAATTTGACCATTGACCGATAAGATTTTTTCCTCATTCAGCAACTTATCAAATATT
TGTTGTGCAGTAAGTTTTACCATTTTATCCCTATCGGTTTATAAAGTATGCAGAAGCCTT
TCAGATACCGCCTTAATCACAGGAACGGCAACGGTATTGCCCAATAAATCGTATTTGTCT
TTTTTAGGAATATCAAACGAATAATCGTCCGGATAGCCGAATAAGCGTAAACCTTCTTTT
CCGGTAAGTGTGCGCAAACCGCCGTTGTCAACGACGAAAAGGTGCTCCATATCCATTGCA
ACTAAGGTTGGCGCAACATCATTTGGGTCTAATATTTTATTGATTTCAAATGATTTTTTT
CCTGAAACAATATTGTAGCCTTTGGGCAGGGATTCATCTTTGATTCTTCGCCCGCCAATT

## Appendix A

TTTTGTTTCGGATGCTCCAAAACCAAATAGCCTTTATCTGTCAGGCTGTCCAAAATATTT
TGAAGATTGGGGTGTTTATAGAAAGTTGAAATTTGCGCTTTTGTCAAAGGCATCCCATCC
ATCCAATCGATGCCGATTTCTGAAGCCCATTTTTTCTTCCTCCGTTCTTTTAGAAGCATA
TTTAATAATTGCTTCTCTTCTTCGGTTACTGTGCCTTTTAATTCAATATCCCAACTGTGG
ATATTGTTTTTCCCTCCCCGTTTGTCCTTTACTGATTTTCCGTACAGTTCGGACGGGGGA
AATTTCTTTAGCAATTTTTTGATGAAAGGACTGCTTTCGGTAGGCAGTCCCGATTCCAAA
ATATTTTTTAATTTCGGACTTAGGGTTGTTTCAAAAGATAAGTCGGGTTTGGATTTCAAA
CTGCCTGTCAGATAAATACGCTTCCTGTTTTGGGGAATGCCGAAATCTTTTGCATTTAAA
ACTTTCCAAGAAACATAGTAGCCCAATGTTTCCAAGGTTTCCAAAATAACGGTCAGGGTG
CGTCCTATTTTTTGTGTCGGATCTTTTCTATCGTGCGTCACCAATCCTTCCACATTTTCC
AAAATAAAACCTTTTGGTTTTTTTGCCTTTAAAATCCTTGCCACATCAAAGAAAAGCGTT
CCCCGCGTATCTTCAAAGCCCAATCTTTTTCCGGCGAAAGAAAAAGCCTGGCAAGGGAAG
CCTGCCAACAAGATGTCAAAATCGGGAATATCTCCCGTTTCAATTTTCGTTATATCTCCA
TACGGCACTTCATCAGGGTAGTTTTGCTTCAATACTTCCAAAGCTGCCGGTTTGATTTCT
GAGGTAAAAACACATTCGCAAGCAACCGACTGTTTCCGACAGGCTTGTTCAAATCCTTTC
CTGATACCGCTCATCCCGGAAAATAAGTCAATAAATTTAATTTGTTGCATATTAAAAATC
TAAAAATTTATTTGAAATGGAGAGTTGCATTATTGCATTAATTTAGAGTGTCGCTAAGCC
CGCTTAAAAGATGAAAGCAATTTATCGCCCCTCTGTTTACATTAGCCGCAACAATTATAT
GTTATCAGGAATGCCGTCTGAACGGCCTTCAGACGGTATAGGTTTTAACCGTTAAACAGC
CAAGGCTGGCTTTGGCGGCGTTTTTCTTCAAAGGCGTGAATTTCGTCGGCGTGTTGCAGG
GTCAGACCGATTTCGTCCAAGCCGTTTAAGAGGCAGTGTTTGCGGTGTTCGGTAATGTCA
AATGTGAACGTTTCGCCGCTTGGTGTGGTCAGGGTTTGTTCGGCAAGGTCGATGGAGAGC
TGATAGCCTTCGTTGGCTTCAACTTCTTTGAAAAGTCGGTCAACCCGTTCTTCGGTCAAC
ACGATAGGTAAAAGGCCGTTTTTGTAGCAGTTGTTAAAGAAGATGTCGGCGAAGCTGGGG
GCGATGACGGCGCGGAAGCCGTAGTCGTCCAATGCCCAAGGGGCGTGTTCGCGTGAAGAG
CCGCAACCGAAGTTTTTACGCGTCAACAGGATTTGCGCGCCTTGGTAACGCGGCTGGTTC
AGCGAGAAATCAGGGTTCAACGGGCGTTTGCTGTTGTCCATGCCTGGTTCGCCGTGGTCG
AGGTAACGCCATTCGTCAAAGGCATTGGGGCCGAAGCCGCTGCGTTTGATGGATTTTAAA
AATTGTTTGGGGATGATGGCGTCGGTATCGACGTTGCTGCGGTCGAGCGGGGCGACGATG
GCGGTAATTTTGGTAAAGGCTTTCATGGGTTTGCGTCTTGTGCTGACGATGCCGTCTGAA
GCGGTTTCAGACGGCATCGCGAATCGGTTATTCGGTGGCGTTTTCGATTTTTCCGCCGAG
ATGGGAAATGCCGCGTCCGACGGCATTGCCGCCTTTTTTGACGGCTTCTTTGGTTTTGTC
CCAGCCTTTTTCGACGGCGTTGCCTGTTTGTTCGGCGGCGCGTTCGGCGGCGGCCTGTGT
TTTGTCAAGGTTGCGGGCGGTGTCTTGTTTCGCGCCCTCCCAAGTGCCGGCGCAGGCGGA
CAAGGCGAGGGCGGACAGGGCGGTAATGAAAAGTTTGTTCATGGTTAAACTCCTTGGTTT
GAATATTAAAGGTGTTTCTGCCTTACGGGACATATTTCAGACGGCCGCGTCAAATTCTTA
AAGACCGCCTGAAAATACTTACGCCATCATGCGGATGTCGGTAAAGCGGCCGGTAACGGC
GGCGGCTGCTGCCATAGCGGGGCTGACGAGGTGGGTACGTCCGCCGTTGCCTTGACGGCC
TTCAAAGTTACGGTTGGAGGTGGAGGCGCAGCGTTGCCCCGGGGTCAGGCGGTCGGCGTT
CATGGCGAGACACATCGAACAGCCCGGTTCGCGCCATTCAAAACCGGCTTCGATGAAAAT
TTTGTCCAAGCCTTCTTTTTCGGCTTGTTCTTTAACCAAACCGGAGCCGGGGACGATTAA
CACGCGCTGTACGTTGGCGGCTTTTTTGCGGTCTTTGGCGATGGCGGCGGCTTCGCGCAA
GTCTTCGATGCGGCTGTTGGTGCAAGAGCCGATGAATACGATGTCGACGGGGATTTCGTT
TAATGGCGTACCGGCTTCCAAGCCCATGTATTCAAGGGCGCGTTCCATACCGCTGCGTTT
GACCGGATCGGTTTCTTCGGCAGGATTCGGCACTTTGCTGCTGATGTCTAAAACCATTTC
AGGCGAGGTACCCCAAGTGACTTGCGGTTCGATGTCTTCGGCGTTGAAACGGTATTCTTT
GTCGAATACCGCACCTTCGTCAGACACCAGCGTACGCCAGTACTCGACGGCTTTGTCCCA
CGCTTCGCCTTCGGGTGCGAAGGGTTTATCTTTTACGTAGTCGATGGTGGTTTGGTCGAC
GGGAAGGATGGCTGAGCGCGCGCCTGCCTCAATCGCCATATTGCATAAAGTCATGCGGCT
TTCCATAGAAAGGCTGCGGATGGCTTCGCCGCCAAACTCGATGGCGTAGCCTGTACCGCC
TGCCGTGCCGATTTGCCCGATGATGTAGAGCGCCACGTCTTTGGCGGTAACGCCCGCTTT
TAATTTGCCGTCAACGGAAATCAGCATGGATTTGGATTTTTTCGCGGTAATACATTGGGT
CGCCATGGTGTGCTCGACTTCGGAAGTGCCGATGCCGTGCGCCAGTGCGCCGAATGCGCC
GTGGGTGGAAGTGTGCGAGTCGCCGCAGACGACGGTCATACCGGGCAGGGTCGCGCCTTG
TTCGGGGCCCATAACGTGTACGATGCCCTGACCTTTGTCCATAAACGGAAAATAGGCGAG
TGCGCCAAACTCTTTAATGTTTTTGTCCAAAGTATCGACTTGCAGCTTGGAAATCGGGTC
TTGGATGCCTTTGTCCCAATCGCCGGTCGGGGTGTTGTGGTCGGCGGTGGAGACGACGCT
GTCGATGCGCCACAGCTTGCGCCCCGCCATTTTCAAGCCTTCAAATGCCTGAGGGCTGGT
AACTTCGTGCACCAAATGGCGGTCGATGTAGAGCAGGACGGTGCCGTCTTCTTCTTCGCG
GACGACGTGGCTGTTCCAAAGTTTGTCGTAGAGGGTTTGTGCTGTCATGATGTTGTTCTT
TTGGATAAATGGTAATGCGGATTGGGCGGATTTTAGACGTATTCTTTATACCGCGCAACA
GATTTTGTCTAATTTTTGAGTCGGTGTTATTTTGTAAACAATTTTAACAAAAAAATTAGA
CATATTGTCCATTTCAGTAAGCAGTTATATCTAAAGCATGATTCGATACGAAAGAATACT
TGTCGTCATTCTTTCAAAGGCATTATCATCTGCATCTTGTCAAAAAACACACAGAGGTAG
ACGAAAGATGAAATTACCGGTGATGTCGCCCGAACATTCGGCGCAACTTCAGGCGTTTGA
GGCAAAAATCCTGTCCAATCACGCCAAAATCGAGGCGTGGTTCCGCACGCAGTGGAGCGT
ACACCGCCCGCCGTTTTACGGTTCGGTCGATATACGCAATGCCGGTTACAAAATTTCGTC
TATCGATATGAATTTGTTCCCCGGCGGCTTCAATAATCTGAATCCCAACTTTATCCCGCT
GGCGGCGGTTGCCGCGCAAGATGCGGTGCAACGCGCCTGCGAAACGGCGAAATCCGTATT
GATTATTCCTGAAAACCACACGCGCAATACGTTTTACCTGCAAAACGTTTACGCCCTCGG
CGAGATTTTGCGTTCGGCAGGGTATGAAGTGCGCTTGGGCAGCCTGAATCCGGAAGTAAC
CGAACCGACCGAATTTGAAACCGCATTGGGCGACAAAATCCTGTTGGAACCTTTATTGCG
TACCCGCGATCGCGTCCATCTTGCAGACGGCTTTTCGCCTTGCGTGGTTTTGTTGAACAA
CGATTTGTCCGCCGGCATTCCCGACATCCTCAAAGGCATCAGCCAAACCGTTTTGCCGCC
GTTGCACGGCGGTTGGACGACGCGCCGCAAAACAAATCATTTCGGCGCGTACAACCAAGT

## Appendix A

TACCGCCGAATTTGCCAAGTTAATCGGCATCGACGAATGGCAAATTAACCCTTATTTTGA
AAAAATCGGCGGTTTGGACTTCCAAGGGCGTGAAGGCGAAGACGCGTTGGCGGAAGCGGT
AGAACGTGTGCTGGCGAAAATTCAAGCCAAATACGACGAATCGGGCATTACCGACAAACC
TTTCGTCATCGTCAAAGCCGATGCCGGCACTTACGGCATGGGCGTGATGAGCGTCAAATC
CGCCGACGAAGTGCGCGGATTGAACCGTAAAAACCGCAATAAAATGGCGAAAGTCAAAGA
AGGCTTGGAAGTCAGCGAAGTGATTGTCCAAGAAGGGATTTATACTTATGAAACCTTAAA
CGGCGCGGTGTGCGAACCCGTCGTGTATATGATGGACCGCTTCGTCATCGGCGGCTTTTT
CCGCGTACACGAAGGGCGCGGTGCGGACGAAAACCTAAACGCCGGCGGTATGGTGTTTGT
TCCGCTGTCTAACAGCATTCCTACCGGTAACGGCGATAATTCCCAAGAAGCGCCCGAAGC
CTGCAAGCGCGTATTCGAACAATGGGACTCGCTGGGTATGCCGCGCTCTGAAAAAGACTG
CGACGTGGACAACGAACACAACCGCCTCTACGTTTACGGCGTAATGGCACGCCTGTCGCT
TCTGGCGGCTTCAATCGAGTTGGAAGAAACGGCGTAAGACTGTTTTGAAATACAGATGCC
GTCTGAAGCGGAAATCCGGTTCAGACGGCATTTCGGATATTTGGCGTGTGGGAACATCTG
TTTCAGACGGCATCTCAGACTATTTAAAAAAGGGAAAACATGAGCATCAAGCAATGGCCG
GAAGGCGAAAGACCCAGGGAAAAGCTGTTGGAACGCGGGGCGGCGGCGTTGAGTGATGCC
GAACTTTTGGCAATCCTGCTGCGCGTCGGCACGCGCGGAATGAGTGCGGTCGATTTGGCG
CGTTATTTGCTGCAGGAGTTCGGCAGTTTGGGGAGGCTGATGAGCGCGGAGGTCGGCAAA
CTGTCGGCATACAAAGGGATGGGGACGGCAAGTTTCACACAGTTTGCCGTGGTCAGGGAA
ATCGGGCGGCGGATATTGGCGGAAGAATTGCAGGAGAGCATCGTCCTGTCCGATCCGGAT
ACGGTGGCCGATTATTTACGCTTTCATTTGGGGCAGGAAAAAGTCGAAGTCAGCGTCGCG
CTGCTGCTGAACCGCCAAAACCAACTGATTGCGGTCAGAGAGCTGTCGCGCGGTACGGTT
GCGGAAAACACGATTTACATCCGCGAAATCGTCAAACTGGCATTGGACGAATATGCCGAC
AGCCTGATTATTGCGCACAACCATCCGGGCGGCTCGCCCGAACCTTCGCAGGAAGACATC
ATGTTCACAAGGCGGCTGGCACAGGCAATGTCGCTGGTCGATGTGTCGCTGCTCGACCAT
TTTATCGTTACCTCGCAAAGCGTCTGTTCGTTCAGACAGCTCGGGTTGATGCCCTGACAC
TCTGTTTTACATGCGGCGGCTCTGATAAAATAGCCGCTTCAACCGTATTCAACAGATATT
GTTAAGTTAATGGAAACACAAAACAAACCTACCGTTACCGACATTGACCGCCCTATACTC
GTCCCGCCCGGTGGACATAAAAAAGTCTTGCTGCATTCCTGCTGCGCCCCGTGCAGCGGC
GAAGTGATGGAAGCCATGCTGGCCAGCGGCATCGACTACACCATTTATTTTTACAATCCC
AATATCCATCCGCACAAAGAGTATATGCTCCGAAAAGAGGAAAACGTGCGCTTTGCGGAA
AAGTTCGGCATTCCTTTCATCGATAAAGACGACGACTACGAAAACGACCGCAAAGAATGG
TTTGCCAAAGCCAAAGGCATGGAGTTTGAGCCGGAACGCGGCATCCGCTGCACCATGTGT
TTCGATATGCGTTTTGAAAAGGCGGCGCAATACGCGCATGAACACGGGTTCCCCGTCTTT
ACCAGTTCGCTGGGCATTTCACGCTGGAAAAATATGGCGCAAATCAACGACTGCGGACAC
CGCGCCGCCGCGCCTTACGATGATGTGGTGTATTGGGATTTCAACTGGCGCAAAGGCGGC
GGCAGCGCGCGCATGATTGAAATCAGCAAACGTGAAAACTTCTACCAGCAGGAATATTGC
GGTTGTGCCTATTCCCTGAGGGATTCCAATGCCCACCGCAAATCACAGGGCAGAATCCCC
ATCAAACTCGGCGTGCTGTATTACGGCGACGAATCGACACAATACGAACCTGCCCCCATC
CGGGTGGACAAATAAACACCCGATGCCGTCTGAAGGTTCAGACGGCATCGGGTTCGGCAT
CGGCACGGGGAAAGGTTTGCCGGTTTGGCAATCTGCAATCGGAAACCGCATTGGCAAGTT
TGCCGTTTTGATAAAACACCCCGTTGCCGCGTCGGGAGGACGGCATTATGAAATCCCTTT
TTATTCGGCTGCTCCTGTTGGGTTCGGCGGCAGGCGTTTTCTACCATACCCAAAACCAAT
CCCTGCCCGCGGGCGAACTTGTCTATCCGTCCGCACCGCAAATCAGGGACGGCGGCGATG
CGCTGCACTACCTCAACCGCATCCGAGCCCAAATCGGTTTGCACAAGCTGGCACACGCGC
CGGTTTTGGAAAACTCCGCCCGCAGGCACGCAAGCTACCTCACGCTCAATCCCGAAGACG
GACACGGCGAACACCATCCCGACAATCCGCACTACACCGCACAAAAGCTGACCGAACGCA
CACGCCTTGCCGGGTATCTCTACAACGGCGTGCATGAAAACATCAGCACGGAAGAAGAAG
CCGCCGAATCGTCCGACAGCGACATCCGCACGCAGCAACGCCAAGTGGACGGATTAATGA
~~GCGCAATCTACCACCGCCTTTCCCTACTTGACGGGGATACGGATGAGGCAGGAGCGGCAT~~
TTGTGCGCGAAAACGGTAAAACCGTTCTCGTATTCAATCAGGGCAACGGCAGGTTTGAGC
GGCATTGCGCCCAAGGCAGAAATCAGCCGGAAGCAGGACGGAAATATTACCGCAACGCCT
GCCATAACGGTGCGGTCGTGTACACCGACGAAGCCATGCCCGCACAGGAGCTGCTCTATA
CAGCCTATCCCGTCGGCAGCGGCGCACTGCCTTATTTCCACGGCGAGCGTCCAGACCCCG
TGCCGGAATATGAAATCACGGGCAATCCTGCCAGCATTGATTTTTCCGAGGCGGCAGGCA
AAATTACGATGAAAAGTTTCAAGCTGTATCAGGGTAAAAACGAAATCCGCCCCGTCAGGG
TTTTAACCGCCGGCAACGACCCCAACGGCAGGCTGACCGCGTACCAATTCGCGCTTTTTC
CGCTCAAGCCTTTGGAATACGGCACGCTTTATACGGCGGTATTCGACTATGTCCGCAACG
GACGGCGAGCGCAGGCGAAATGGCAGTTTAGAACCCGAAAACCCGATTACCCTTATTTTG
AGGTAAACGGCGGCGAGACACTTGCGGTTAGAAAAGGCGAAAAATATTTCATCCACTGGC
GCGGACGCTGGTGTTTGGAAGCGTGTACCCGTTATACCTACCGGCAGCGACCCGGCAGCC
GCCTGTCCATAGGAAGGCACGAGGCGGGCGGCATCGTCTTCAGCGTTGACGGAATGGCGG
GCAGCCGCATCACGCTTGCACCGGAAGGAGAAACGGAACGAGGCGTAACCCTTTATTTAC
AGGATTGAATACATGACAGGCAGAACAGGCGGCAACGGCAGTACCCAAGCGCAACCCGAA
CGCGTCATGCTGGTGGGCGTAATGTTGGACAAAGATGGTACGGGCAGTAGTGCCGCCCGT
CTGAACGGTTTTCAGACGGCATTGGCGGAAGCTGTCGAGCTGGTCAAAGCGGCGGGCGGC
GATTCCGTGCGCGTGGAGACTGCCAAACGCGACCGTCCGCACACCGCGCTGTTTGTCGGC
ACGGGCAAGGCGGCGGAGCTGTCAGAAGCAGTTGCCGCAGACGGCATCGATTTGGTCGTA
TTCAACCACGAACTCACGCCCACGCAGGAACGCAACCTTGAAAAAGAACTGAAATGCCGC
GTATTGGACAGGGTAGGGCTGATTCTGGCGATTTTCGCTCGCCGCGCCCGCACGCAGGAA
GGCAGGCTGCAAGTCGAGTTGGCGCAATTGAGCCATTTGGCGGGACGCTTGATACGCGGT
TACGGCCATCTGCAGAGCCAGCGCGGCGGTATCGGCATGAAAGGCCCCGGCGAAACCAAA
CTGGAAACCGACCGCCGATTGATCGCCCATCGGATCAATGCCTTGAAAAAACAGCTTGCC
AACCTCAAAAAACAGCGCGCCCTGCGCCGCAAGTCCCGCGAATCGGGCACAATCAAAACG
TTTGCGCTGGTCGGCTATACCAATGTCGGCAAATCCAGCCTGTTCAACCGGCTGACCAAG
TCGGGCATATATGCGAAAGACCAGCTTTTCGCCACACTCGACACGACGGCGCGGCGGCTG

## Appendix A

TACATCAGTCCCGAATGCAGCATTATCCTGACCGATACCGTCGGATTCGTCAGCGATCTG
CCGCACAAACTGATTTCCGCCTTTTCCGCCACGCTGGAAGAAACCGCGCAAGCCGATGTG
CTGCTGCACGTCGTCGATGCCGCCGCTCCGAACAGCGGACAGCAGATTGAAGACGTGGAA
AACGTACTGCAAGAAATCCATGCCGGCGATATTCCGTGCATCAAGGTGTACAACAAAACC
GACCTGCTGCCGTCTGAAGAACAAAACACGGGCATATGGCGCGACGCTGCGGGAAAAATT
GCCGCCGTCCGCATTTCCGTTGCTGAAAATACCGGTATAGACGCACTGCGCGAAGCCATT
GCCGAGTCTTGTGCCGCCGCACCAAACACAGACGAAACCGAAATGCCATGAAAAAAACCT
GTTTCCACTGCGGTCTGGATGTTCCCGAACACCTCCACCTGACTGTCCGTTACGAAAACG
AAGACCGCGAAACCTGCTGCGCCGGCTGTCAGGCGGTCGCACAAAGCATTATTGACGCGG
GCTTGGGCAGTTATTACAAACAACGCACCGCCGACGCGCAAAAAACCGAGCTGCCGCCCC
AAGAAATCCTCGACCAAATCCGCCTGTACGACCTGCCCGAAGTCCAGTCCGACTTTGTGG
AAACCCACGGCGGCACGCGCGAGGCGGTTTTAATGCTCGGCGGCATCACCTGCGCCGCCT
GCGTCTGGCTGATCGAACAGCAGCTTTTGCGTACAGACGGCATCGTCCGCATCGACCTCA
ATTACAGCACGCACCGCTGCCGCGTCGTCTGGGACGACGGCAAAATCCGCCTTTCCGACA
TTCTGTTGAAAATCAGGCAGATAGGCTACACCGCCGCACCCTATGACGCGCAAAAAATCG
AAGCCGCCAACCAAAAAGAACGCAAACAATACATCGTCCGCCTCGCCGTTGCCGGGCTGG
GGATGATGCAGACGATGATGTTCGCGCTGCCGACCTACCTTTACGGCGGCGACATCGAAC
CCGATTTCCTGCAAATCCTCCATTGGGGCGGCTTTTTAATGGTGCTGCCCGTCGTATTCT
ATTGCGCCGTCCCGTTTTATCAAGGCGCGCTGCGCGACTTGAAAAACCGCCGCGTCGGCA
TGGATACGCCGATTACCGTCGCCATCATCATGACCTTTATCGCCGGCGTTTACAGCCTTG
CGACAAATGCGGGGCAGGGGATGTATTTCGAATCCATCGCGATGCTGCTGTTTTTCCTGC
TGGGCGGACGCTTTATGGAACACATTGCCCGCCGTAAGGCAGGCGATGCCGCCGAGAGGC
TGGTGAAGCTGATTCCTGCGTTTTGCCATCATATGCCCGATTACCCCGATACGCAGGAAA
CCTGCGAGGCAGCTGTCGTCAAATTGAAAGCGGGCGATATCGTGCTGGTCAAACCGGGCG
AAACCATCCCCGTTGACGGCACGGTGCTGGAAGGAAGCAGTGCCGTCAACGAATCTATGC
TGACCGGCGAGAGCCTGCCCGTCGCCAAAATGCCGTCTGAAAAAGTAACCGCCGGCACAC
TCAACACGCAAAGCCCCCTGATTATACGCACCGACCGCACCGGCGGTGGCACGCGACTGT
CGCACATCGTCCGCCTGCTCGACCGCGCCTTAGCGCAAAAACCGCGCACTGCCGAGTTGG
CGGAACAATACGCCTCGTCTTTCATATTCGGCGAACTCCTGCTTGCCGTCCCCGTCTTCA
TCGGCTGGACGCTGTACGCCGACGCGCACACCGCATTGTGGATTACCGTCGCCCTGCTGG
TCATTACCTGCCCCTGCGCCTTATCGCTTGCCACGCCGACCGCGCTGGCAGCTTCTACCG
GTACGCTGGCGCGCGAAGGTATTTTAATCGGCGGAAAGCAGGCAATCGAAACCCTCGCCC
AAACCACCGACATCATCTTCGACAAAACCGGCACGCTGACCCAAGGCAAACCCGCCGTCC
GCCGTATCTCATTGTTGAGAGGCACAGACGAAGCCTTTGTTCTCGCGGTGGCGCAGGCTT
TAGAACAACAGTCCGAACATCCCCTTGCCCGCGCCATCCTCAACTGCCGCATTTCAGACG
GCAGCGTCCCCGACATCGCTATTAAACAACGCCTCAACCGCATCGGCGAAGGCGTGGGCG
CGCAACTGACCGTCAACGGCGAAACACAGGTTTGGGCATTGGGCAGGGCATCCTATGTCG
CCGAAATTTCAGGTAAAGAACCGCAAACAGAAGGCGGCGGCAGCGCGGTTTACCTCGGCA
GTCAAAGCGGTTTCCAAGCCGTGTTCTACCTGACCGACCCCTTGAAAGACAGCGCGGCGG
AGGCGGTGCGGCAGTTGGCAGGCAAAAACCTGACCCTGCACATCCTCAGCGGCGACCGCG
AAACCGCCGTTGCCGAAACCGCACGCGCCCTGGGTGTCGCGCACTACCGCGCCCAAGCCA
TGCCCGAGGACAAACTGGAATACGTCAAAGCCTTGCAAAAAGAAGGGAAAAAAGTGCTGA
TGATAGGCGACGGCATCAACGACGCGCCCGTTTTGGCGCAGGCAGACGTATCCGCCGCCG
CAGCGGGCGGACGGATATTGCGAGGGACGGCGCGGACATTGTGTTATTGAACGAAGATT
TGCGTACCGTCGCCCACCTGCTCGATCAGGCGCGGCGCACCCGCCATATTATCCGGCAAA
ACCTGATATGGGCGGGCGCGTACAATATCATTGCCGTACCGCTTGCCGTTTTGGGCTATG
TCCAACCGTGGATAGCCGCACTGGGTATGAGCTTCAGTTCGCTGGCGGTTTTGGGCAACG
CCCTGCGCCTTCACAAACGGGGGAAAATGCAGTCTGAAAAAATGCCGTCCGAACAATGAC
~~GGACGGCGTTGCTTTAGACGTATAGTTGATGAAAACAAAAATAAGACGATGAAGAATTGC~~
AAACTTAAAGTATGTATTGTTACCGCTCAAACACGTTGGCGTTCAAAATTTGAGATCGAA
TTGCTAGGTATTTGATTTTATTAAATAAGAGATTGTATTAATGAGGAAAAAAATTAAAAA
GTGGATTTATGCGATAAATGGAATAATGATGTCAATATGGATTATTTTTTTCCAAATAC
CGGTTCTGTGTATGACGGTGGCAGAACAACCATTTTCAATGAAAACCATCCTTTTCATTT
TATTTTCTGCATAACATTTCTTATTGGGACAATTTTTCTTATATATCATGAATATAATGA
TAACTAATTTTTAACATCCTTATTGTTATATCATGATGAAATGACAATAAGGATGGTTTT
CTGCTTTGGCTACTGCAGAACACCGTCGTCAGTCTCGCGTAGGGGGGAATCCATATGCTT
GGTTTTTCTTTTATTTTCAAATGCTAATTAACGGATAGGTCTGGATTCCCGCCTGCGCGT
GAATGACGGAAATGTGCATTTCTAATTTTTACCCACTATATAGTGAATTAAATTTAAACC
GGTACAGTGTTGGCTCGCCTTGCCGTACTATTTGTACTGTCTGCGGTTCGCCGCCTTGTC
CTGATTTAAATTTAATTCACTATAAAAACCCCGAATCCTGATTGGCAGGATTCGGGGTTT
TTGATTGCTGGTGCCGTTCAGACGGGATTTTCAAACAGCTTATTGATCTACAAACGCACG
CTCAATCAGGTAATCGCCGCGTACGCCTGTTTTCGGAGAGACGGTCAGTCCGAAATCGTC
CAAAACTTTGCAGGTATCTTTCAGCATCGCGGGGCTGCCGCACAGCATGGCGCGGTCGTC
TTGCGGGTTGATTTTGGGCAGGCCGATGTCTTCAAACAGTTTGCCGCTCACCATCAGGTC
GGTTAGGCGACCGTGGTGTTCGAATTCTTCGCGCGAAACAATCGGGTAGTAAATCAGTTT
TTCTTTAACCAAGTCACCGAGGTATTCGTGTTCGGGCAATTCTTTGGTAAAGCGGTCGTA
GTACGCCAAATCTTTTTTGTAGCGCACGCCGTGTACGAGGATGATTTTTTCAAATTGCTC
GTAAATTTCGGGGTCTTTGGTGATGCTCAAGAAAGGAGCGATGCCGGTACCGGTGCTCAA
CAAGTAAAGGTGTTTGCCCGGATTCAGGTCGCCGGCAACCAGGGTTCCGGTCGGTTTTTT
GCTGATTAACACGTCGTCGCCGACTTTGAGGTGTTGCAGGCGGCTGGTCAGCGGGCCGTC
TTGGACTTTAATGCTGAAAAATTCGAGGTGTTCTTCCCAGTTGGCGGAGGCGACGCTGTA
TGCACGCATCAGCGGCTTGCCGTCCACCATCAATCCGACCATAACGAACTGTCCGTTTTC
AAAGCGCAACGATTCGTCGCGGGTGCAGGTAAAGGTAAAATATGCGTCTGTCCAGTGGTG
TACGGACAATACTTTTTGGGTATTGAATGCTGCCATTTGGGTTTCCTGTCAGTAAAAGAA
ATGGATAGTGCTTGTTCGGGAGGTGCGGCAGAGTGGAAATGTCTGCCCGATTCGGGATAA

## Appendix A

AGCCAAAATTCTAAACGAAACGGATGGTTGCGACAATGCTTGATGCGCGTTGGTTATATG
CCGTCTGAAGGGCTTCAGACGGCATCGCGGGGCAGGCGGCGGCTTACGGCGGCATATCGG
CAAGGGAAATCAGGGAAATCGAGGCTGCCAGCCTGAGTGCGTCCCGTCCCCAGCGGGGGT
GTTCCAGCCTGCGTATCGGGAGGATCGGTTTGACGGTGGCGGCAATCAGCTTTTGTATTT
CGGCAGGCTGATCTGACAATACGCCGCCCCATTTTTCCGCAGCCGCTTCCAGCAGTCCGC
CGTTTTTCAATATCAGCGCGGTCGAATGGATGTAGCAGAGCCAATCGCGGGCTTGGCATT
GCGCTATGGTCAGGACTTCGGAAGGGTCGTCTTCAAAATCCAAAAAGCTGATGTTTTTTC
CGTCCGACATCATATTTCGCGCAAACGCCTGACTGAGGAACTGCCGTTTTTTATGCACGC
GTGCAATGGCTTCCAAACCGGCAAGCCAAGCGTCCGACTTTCCAGCCTCGGCTTCTTGGC
GGATTTGCGCATCGAGCGGGATGCCTTCCAAATTGCCGAACATAAGGGCATTTTTCCTGA
CGGCGAGCAATTCGGGAACGGCTATCCCCGCCGAGCGCAATTCGTACAGGCGTTTTGATT
CGGTTGCAATGGCAGGCTCGCCGCCGAGGCTGGGAACCGGCTTCAACACCCCCAGTTTCA
AATATCGGGCAACCATACCGAGCAGCGCGTAACGCCATCGTGCATTGTGCCTGCCTGCTT
TGCGTATCCATACCTTGGTTCCGTCGGCAAGCAGGTGGGGGGCGATGGCTGCCTCCTGTT
TTGCCGCCAATTCGTCTAGCAGTATGGAAAAACGGGTTTCCTGCATAGGTAAGGTCATTT
TCTTTCAATCTTAAGTTCGGACGGAATGCCCCTGTACGGAATATCAGGCAAGGGTTTGTT
CGATGATGCCTTTTACCGCATCCGCACTGTTCGGCACGGTTTGCACGCGCTGCGGCAGGT
TTTCCAAACCTTCCAGCGCGGCAGGGCGCGGAATGGCGGCATCGCCGACGGCTTCGCGTA
TGGTCGCATCGAATTTCGCCGCCAACGCGGTTTCCAAACAAACCACCATTTCACCTTCTT
CGCGCACTTCGCGGGCGACTTTTACGCCGTCGGCAGTGTGCGGGTCGATGAGTTCTTGGT
CTTGCTCGTAAACCTGCTTGATGGTGGCGAGGCGGTCGGCGTGGGTGGATTTGCCAGAGG
TAAAACCGTATTTGCCACCGACTTTGTCCAAGGCAAATCGCAGGTCAAAGCCTTTGCCTG
CAGCCACTTCCGCCCACAGCGTATTGATTTCCGCAGGATCGCGATCCATCAGGTCGAACA
CGAAACGCTCGAAGTTGGACGCTTTGGAAATGTCCATAGACGGGCTGGAGGTTACATAAG
TATGCGCGCTGTTGCGCGGGCGGTATGCACCGGTTTTGAAAAACTCGTCCAACACATCGT
TTTCATTGGTCGCGACAATCAGGCGGCGGATAGGCAGGCCCATTTGTTTGGCAATGTGTC
CCGCGCAAACATTGCCGAAGTTGCCGCTCGGTACGCAGAAGCTGACGGTTTCGTCATTGC
TTGAAGTGGCGTTGAAATAGCCTGCAAAGTAATAAACCACTTGCGCGACGATGCGTCCCC
AGTTGATCGAGTTGACCGTACCGATATGGTATTTTTCCTTGAACGCGGCATCGTTCTGCA
CCGCCTTCACAATATCCTGACAGTCGTCAAACATTCCCTTCACGGCGATATTGTGGATAT
TCTCGTCTTGCAGGCTGTACATTTGCGCGCGTTGGAACGCGCTCATTTTACCGTCGGGCG
ACAACATAAATACGTTCACGCCCTTTTTGCCGCGCAAGGCATATTCCGCAGCCGAACCCG
TATCGCCGCTGGTCGCGCCCAAGATATTGAGTTTTTTGCCTTCTTTGTTTAAAACATATT
CAAACGCATTGCCCAAAAACTGCATTGCCATATCTTTGAACGCCAGCGTCGGGCCGTTGG
ACAAGGCTTGGATTTTGATGCCGTCTGAAAGCGTGCGGACGGGGGTGATTTCCTTAGTAC
CGAACGCCGCTTCCGTGTAAGTACGGTTCAGAATGTCGCGCAAATCGTCCTCCGGAATAT
CCGTAACAAACAGGCGCATAATTTCAAACGCCAATTCGGGATAAGCTAAACCGCGCCATT
TGTCCAAGGTTTCGCGCCCGATTTGCGGATAATGTTCCGGCAGCATCAGGCCGCCGTCGG
GGGCAAGCCCCATCAATAAAACTTCGCTGAACGGTTTGTGTGCGGTTTCGCCGCGCGTGC
TGATGTATTTCATGATTTTTCTCGTCTGTCGAAATTGCAGGAAAACGGCTTCAGACGGCA
TCTGCCTCATGCCGTCTGAAGAAGGTTAGCGGTACAGGTGTTTGAAGCAGGCGGAAACCG
TTTTGGCGGTCAGGGCGGCAAGTGCCTGATTGCGCGTGGACGGAGCCAGCATCTGCATCA
CATCGTTGCCGGTCATCCGTTCGGGTGCTTCTTGGGCGACGCAAGCGCAAATCTTGTTTT
CCCACTCCGCCTGTTTTTCGGCACTCATCGCCAGCGCGGTCAAACGCCATTCGCTGCGTT
TGTCCAATTCCGCACGGCATTGGCTCCCAACCGCCATTTTGACGATGCTGCCGCCCATGC
CTGTGCCACCGTCTAAGCTGCCGAATGTGTTACCGCCTCCGGCGGCGCAGCCGCCGAGTA
AGATTGCCACCGGCAAAATAGACAAGGTTTTATTCATCTCAATTCCTTTTCGGTTGAAAC
CCCGCCTTTTATGGCGATAGAATCTGATTAGCCGCCCCGTTCGGGATAACGCGAAGGGCG
GCGTTTTATGCGCCGTTCCGAGTGTTGGAACAAACCGTTTTGAATATCCGGTTGAAGCCC
GGCAACATTATACTTCAATCGGGAAAATAAAAAATCCCGCCGCCGTCATTTTGCCTGTTT
GCAAAAATGCCGTCTGAAAGCGGTTCAGACGGCATTTCCGATTTCAGCCTAGCCCAAAGA
TTTGAAGTGTTCCAAAAACGGCGGGATACCGGGCAGCATCCCGACCGCACCCATCGCCAC
ACACAAGATCAAGAAACCTACTGCGGGTATCAACACGCGTCCGGCGAAACCTAATTGCGC
ACTGCGCTCTTTGCAGCCGATTAAGCCCAAATTATCCAACAGCATCGTCAACGCCCAGCC
GAAAACCGGATTGACCAAGGCGGAAGAGAACACCACGATGGCGGCGGATTGGGTGGTTTT
GCCTTTGCGCGTCATTTCCATGCCCGCTTCCAAAAGCGGTAAGTATACGCCTACGACCAA
GGCTACGCTCAATACCGGCTGCCAAATCGCCAAGTCCATCGGATAGCCCCATAACCCGGC
GATAATACATAAAACCGCCGTTAAAACCGCACCGCCCGGAATGGGGCGTTTGGCAATCGA
TGCCGGTACGATATAAGTTCCCCAAGAAGAGGTAAAATTTGCACCCCCTAAAATAGAACC
CACTGCTTGACGGACAGAACAACTTGTCATGGTGTCGTCTATATTCATCAATACCTTATC
GGTTTTTTCCGGATAGCTGATTTTTTGGAACACTTGATGTCCTAAAAAATCGGGCGACCA
CATTGCAACAGCCAATACCGCAAATGGAAAGACAACCAAAAAACTTTCTGCCGTCGGCAA
CCCCAGATGCCAGCCGCTGTTTTCACCCCACCAATAAGCAGGACTCATTGGAGGCAGGCC
GGGGGCGGTGTGAAACTCAAACGGCGCACCCAATGCAAATGCCACCACACCGGCAATCAA
GCATCCCAAAGGCACGGCTAACCAGCGTTTTTTCCAATGCTCCAACAAAGCGTACATCAC
AATCGTTACAATAATGACGGTAAAAGCGATGTAGGGCATATTAAAACCGCCTGCCCACGA
AAACAATTTTTTTACCTGCCCCGTCGTGCCGATAAAGCCCAAATAGAGTAATAATCCGCC
GCATACGCCGTTGCTTGTCAGCTTCGCCATAATACTGCCGCCGCGAAATAAAGCCATCAG
CAGACCTAAAACCGCAATCGAAATGCCGAACGCCAAAGGATGCCCGCCTGCCGACACAAC
GATGGGAATCATCGGAATCAGCGGCCCGTGCGTACCGGGCAGGTTGGCGCCGGGCAGAAA
AAAGCCCGATACCAATAAGATAAACGCGGCGGCGATTAAAAGCTCATAGCGCACATTTTC
CAGTACAAAGCTGTCAGGCAGCCCCAAAGGTGCGGCAAACGCCGCCGCCACCGCCCCCAC
CATCACCACTTTTCCAATCGTTCCCGCCATCGCAGGAATCAAATCCTCCCACTCGAAGCG
GTAATCGCGAAAGGGCAGGTTGGGCCGCCAGCGTTTTGGTTGCATAATCTGCAATTCATG
TTCCAAATATTCGTCCCGCGTCGCAAATTCCGAAGCTGGACGGTGCAAATCCCGATAAGT

## Appendix A

CCCATTATGTTTTTCCATAACCTTCCTCCTTATATATCGCGCCTCGTAAAAGGGGCGCAT
GACTTTTCTTTTTGATACGGGCTGCGTTCGGAAGCCGTAACCCCATTTAAAGCCCAAACA
GGCAATAAAACCAATCTTTTTTTTTGATAACCATCATCCGGAAAACTGATACAATTTACA
AACCACTTGATTAAAAAGTTAATTTTCAGCAACAATCCACCTAAAAGATTTCGATTGCAC
AAATATAGAAAACATCCGCACAAGGAGGATATATGGATGCCGTACAATTAAAATCATTT
GTCGCCGTCGCGCACGAGGGCAACCTTACCCAAGCCGCCAAACGACTTTTCCTTTCCCAG
CCTGCCGTTTCTGCCCAAATTAAAGCCCTTGAAGAATATGTCGGCACGCCGCTGTTCAGG
CGCACGGGGAAAGGCATGGTATTGACGCGGGCGGGCGAAATACTGTTGCCCGAAGCGGAA
TCCCTGCTGCAATACAAACACAAGCTGGAGCATTTTGCCAAAACGCTGGCAGGCGATTAT
TCGGAAGAGACCAGTTTGGGCATTATCCACCCCATCGATTCGGCAAAACTCGTCGCGCTG
ACGGACAATATCGGTCAAACAGCCCCCAAAACGCGCCTGCACATCCAATACGGAATGAGC
GGCGAAATCCTCTCGCGCATCCAACACAAAACCCTGCACGGCGGCTTTATACTCGGCAAC
GCCGCCCAACGCGGCATCCGCAGCGTATTCCTGCAAAACCTGACCTACGCGCTGATTTGC
CCGCAAAGCCAATATCCCCATCTGACCCGCTCCCTTCCGCAGAGCCTGCAAGAATGCGTA
TGGATAGAAATGTCGGGCGTGTCCGGAAGTAGGAAGCACCTGCACCAGTTTTGGCGCAGC
AACCGGCTCTCACCCAAAAAACAGATCTTGTGCGACTACCCCCAAACCATTATCGATTTG
GTTGCAGGCGGTATAGGTGTGGCAATGGTGCCGGGAAACAAAGCCGAAGCGGCGGCAAAA
GAAGGCGCGGGCGTGGCTATTATCGAATCGTGCCGCCACAGTATGCCGCTCAATTTCATT
TATGCGGAAGAATACGAGGATAATCCCCACGTCTCACTCCTGCTCGAGTGCATTGAAAAA
GTATGGGGAGTGCAGGCGGTGCAGCCGCCCGTTGTCTCGGACAACTGAAATAAATCCTGC
TTTGCTGATTGTTTTAAAATAGAAATTTGAATTTTATCACGCTGAAAACACTGAAAACGC
CATCCGCATTCTCTCAAATACGGCTTAAAATGCCCTTTGGAAATGCCGTTATAGTGGATT
AACAAAAATCAGGACAAGGCGACGAAGCCGCAGACAGTACAAATAGTACGGAACCGATTC
ACTTGGTGCTTCAGCACCTTAGAGAATCGTTCTCTTTGAGCTAAGGCGAGGCAACGCCGT
ACTGGTTTTTGTTAATCCACTATAAACTGACGCAAATACCGTTTTGCACAATTCCAAAAG
TTTTCAATTCCGTTAATGCGATTTTGCCGTTTGGCGAAATGCGTACTGTTCCAGTCGTGG
ATTGAACCCCCACCCTGTATAGTTCTTTCGAAGCATTGGGGTATTGTTTTTTCAAAGCAT
CTTGGATTCGGATTTCAAGTGCAACACTAGTGTATTAGTGGTTGGAACAGATTCAAGAAT
AAAACACTTGGCGTTTCGTAGCCAAGTGTTTTTCTTGGTCGGTGGTTCAACTCATCTTGA
ACCCTGCGTATCTCCCGATCACTGATGTTACGGAAATCGGTTTGTTTGGGGAAGTATTGC
CGGATGAGTCCGTTGGTGTTCTCATTCAGCCCTTTCTCCCAAGAATGGTAAGGGCGACAA
AAATAAGTCTCCGCTTTCAATGCTTTGGTTATTTTGGTGTGTTGGTAGAACTCTTTGCCG
TTATCCATGGTAATGGTGTGCACCCTGTCTTTATGTGCCTTTAATGCCCTAACAGCTGCC
CGGGCAGTGTCTTCGGCTTTGAGGCTATCCAATTTGCAGATGATGGTGTAGCGGGTAACG
CGTTCGACCAAGGTCAATAATGCGCTTTTCTGTCCTTTGCCGACAATGGTGTCGGCTTCC
CAATCGCCGATACGGGATTTCTGGTCGACGATAGCGGGTCGGTTTTCTATGCCGACACGG
TTGGGTACTTTGCCTCTGGTCCATGTGCTGCCGTAGCGTTTGCGGTAGGGTTTGCTGCAT
ATTCTGAGATGTTGCCACAACGTGCTGCCGTTGCTTTTGTCTTGGCGAAGGTAGCGGTAA
ATGGTGCTGTGGTGGAGCGTGATCTGGTGGTGTTTGCACAGGTAGGCGCATACTTGTTCG
GGACTGAGTTTGCGGCGGATAAGGGTGTCGATGTGCTGAATCAGCTGCGAATCGAGCTTA
TAGGGTTGTCGCTTACGCTGTTTGATAGTCTGGCTTTGCCGCTGGGCTTTTTCGGCGCTG
TATTGCTGCCCTTGGGTGCGGTGCCGTCTGATTTCGCGGCTGATGGTGCTTTTGTGGCGG
TTCAGCTGTTTGGCGATTTCGGTAACGGTGCAGTGGCGGGACAGGTATTGGATGTGGTAT
CGTTCGCCTTGGGTCAGTTGCGTGTAGCTCATGGCAATCTTTCTTGCAGGAAAGGCCGTA
TGCTACCGCATACTGGCCTTTTTCTGTTAGGGAAAGTTGCACTTCAAATGCGAATCCGCC
GTCGTTTGAACATTTTTTTCTTCCTGTTTGATTTCAGACGGCATTGCCGTTCCGTTTGGT
TTCCAGCAGCTCCCAGCGTTCCAGCTTTTCCAAAAGCAGCATTTCGATTTCTTCGGCGCG
GTTTTGCAATGCACCTGCTTTTTCGTAATCTTTGAAAATTTCAGGATAGGAAAGTTGGGT
ATTGATTTCAGCCTGGTGGCGTTCCAAAGCGGCGATTTCGTCGGGCAGGGCATCGAGTTC
GCGCTGCTCTTTGTAGGACAGTTTGACCGTGCGGTTGGCTTTGGGTTTTTCTTTGGCGGG
TTCGGCATCGGATGCTTTGGGTGCGGATGCCGTCTGAATTTTATCTTCCCGCGATTTTGC
GTCGATATAGTCCTGATAGCCGCCGATGTATTCTTTCAGACGGCCTTGTCCTTCGAAAAC
AATGCTTTGGGTAATTACGTTATCAAGGAACATACGGTCGTGCGAGACAAGGAATACTGT
GCCTTGATAATCGCGCAACAGGTCTTCGAGCAGCTCTTGGGTGTCGATGTCTAAGTCGTT
GGTCGGTTCGTCCAAGACCAGGATATTGGCAGGACGGGTAAAGAGTTTTGCCAGCAAAAG
GCGGTTGCGTTCTCCGCCGGAGAGCGATGAAACAGGGCTTTGCGCACGGGCTGGATGGAA
CAGGAAATCTTCCAAATAGCTCATGACGTGTTTTTTCTTACCGCCGACTTCAACGTAATC
GTTTCCCTGTCCGAGGGTGTAAAACACGGTGTCGTTTTCATTCAACGCGCTGCGGAACTG
GTCGAAATAGGCGACTTCCTGCTTACTGCCGATACGGATTCTGCCGTAGGTCGGCTGCAA
TTCGCCCAAAATCAGCTTAAGGAAGGTGGTTTTGCCGATGCCGTTGGGGCCGATTAGGCC
GATTTTGTCGCCGCGCTGCAAGATAGCGGAGAATTTGTCCATAATGACTTTGCCGCCATA
GGCAAACGAAGCGTGTTCCAATTCGGCGATGATTTTGCCACTTTTCTCACCGCTATCGAG
CTTGAAGTTGACTTGTCCCTGTACGTTGCGGCGTTCTGCACGCTGGCGGCGCAGCTCTTC
CAAACGGCGCACGCGGCCTTCGTTGCGGGTACGGCGCGCTTCGATGCCTTTGCGTATCCA
TGCTTCTTCCTGTGCGTGGAATTTGTCAAAGAGGCGGTTGTGTTCCGCTTCGACTGCCAA
CTCTTGCGCTTTTTTCTCGCTGTATTTAGAGAACGAGCCGGGATAGGAACGCAAAATACC
GCGATCGAGTTCGACAATCCGCGTGGCGATATTGTCCAAAAAACGGCGGTCGTGGGTAAT
CACAACCAAGCTGCCTTCAAACGCTTTGAGCAGATTTTCCAGCCAAATAATCGCGTCGAT
ATCCAAATGGTTGGTCGGCTCGTCCAGCAGCAATACGTCGGGCTTTTGCACCCAAGCCTG
AGCCAAGGCGACGCGCTTTTTCTGACCGCCGGAAAGGTTGCCGATTTTTTCATTTTCCGG
CAAACCGAGTTCCCCCAAAGTCTGCTTGACTGCCGCATCCAGTTTCCAGCCGTCCTTCGC
TTCGATTTCAAGTTGCAATTCGTTGAGTTCTTTCAACAAAGCCTCACTCGAACCATTTTC
CAACTCATGGCTGACATGATGATAACGGCGCAATAAATCACGAATTTCGCCCAAACCTTC
GGCAACGGTATGAAATACGGTTGCGTCCTTATCAAAAAAGGATTCCTGCGGTACATAAAC
GATTTTGAGGTTGTTTTGAACAATAATCTGCCCGTCGTCGAGCTTTTGCAAACCGGCGAG

## Appendix A

GATTTTTAAAAACGAAGACTTGCCTGCGCCGTTGCGTCCGATTAAGCCGACTTTTTCGCC
GCTGTCGAGTTGAAAAGAAGTTTTGTCGAGCAAGGCAACGTGGCCGATGGCAAAAGAAGC
GTTTTCTACAGATAATATATTCATGATACAAATTCTCAACAGTTACCGTTTGGATTTTAC
CGCAAGTTTGGCGCGGGCAATTTCAACCGCACCCGGCAGGACGGAAACAATAATGATGCC
GCCCATCACCAAGCCCAGATTGTTTTTTACGACGGGGAAGTTGGCAAAGAAATAGCCCGC
GTAAGAAAACAGGATAACCCACAACAAGCCACCGATGATGTTGTAGCGGATAAATTTGGC
ATAGTGCATTTTCCCCATACCGGCGACGAAGGGGGCGAAGGTGCGGACGATGGGCATAAA
ACGGGCAATGATGATGGTTTTGCCGCCGTGTTTTTCGTAAAAACGGTGGGTTTTATCGAG
ATATTCACGTCGGAAGATTTTAGAATCGGGGTTGGCGAACAGCCTGCCGCCGAAATATTT
GCCGACGGTAAAATTGAGCGCGTCGCCGAGTATGGCGGCAAGGCTTAATAATGCAACCAT
CAAATGAATATCCATACCGCCCAGCGCGGCAATCCCGCCGGCGGCAAACAGCAGCGAATC
GCCGGGCAGTAAGGGCGTAACAATCAGGCCGGTTTCGCAAAAAACAATCAAAAACAGAAT
CGCATAAATCCACACACCGTATTGCGCCGACAGCGCGAGCAGGTGTTGGTCGATATGGAG
GATGAAGTCGATGGCGGAGGCAAGCACGGCGCGTTCCTAAAAAAACAAACCGCGTATTTT
AACCGATTGGAAAAATGCCGTCTGAAAAGTTTCAGACGGCATCGGCTATTCAAATTCATT
TCACGTAAAAACCGCAAACCAAAATAGTTTGCGGTTTGGCATTTAAAGTGACAATGATGA
TTTCAAATCATCAGAATTTTATGCCGACGCGCAAGCCGTATTCACGAATACTGGTTTTCG
GGATGGTGAGCGATACGTCGCCACTCTTGGTTGTTACACTAAACTCGCCGGATTCTTTGT
AAGTGCGTTGTTTGTAGAACGGCCCCGCCTCGATGCTGGCGGATTCGCCCAGTTTTTTAC
CAATATTTGCACCCAATCCAAAGCCCCAGCCGTTGGTTTTTTGATTGATGTCTTTTTTGA
GATTGGTAACGCCGGTGTTTAATTTATAGCGGGAATTGAGGTCAAATTTCACTTCAGACC
AAGGGTTGATATACCAGCCGTTACCCAGTTGGGAAAGCAAATCCGCGTGAACTTTGGCTA
ACCACGACTGACGGCTGCTGTGAAGCGTATGCTTGGTGGTTTTAATGCTGTCTTTTGAAG
ATTCAAAACCCAAGCCGGCACCCACACGGAAATTTAAAGAATCACTTAACGTTTGGGTGT
AGGTGTAGCCTGTGTAAAGATCGATACGGTTTTCAGGAACGCCGGTGGGCAGTTTTACAT
TTTTAGTCTTACCCAGCTTGTTCTCATCTGTTTCCAAATTAATAATATTTTTTTTGCTGC
GCCCGAAACCGGCTTCCAAGCGGATGCCTTGGTTGGCATCAAAAGGAATATCAGCACGCA
CGCTGATGTGTTTGGCAGCTTTGTGTTTTTCTTTCAGGAAAGCACGAGTTGAAGAAATGG
AAGAGAGGTCGGTGTGGACGGTAAACTCATTAGCGGTTTGAAGCTCTTGTGCAGCGGCGG
CAGTACCGGTCAGGGCAATCATGGCACATGTAAAAACTGTTTTTTTCATAGTTAAAACCT
CTAAAATTTGGATTGTAGTCGGATATGGTAACATAACGTAAATAATCGTTACGCTTACAA
TTATATTCTTAAGCTTTCGGGGGGGGGGGGATTTTACATATATTAATAAAAATTAACAAA
TAGTTATTTGTTTACAACGAATTGTTATTCTCACTTGGTTTTCTGTTTTTTATGGGAATG
ACGAAATTTTAGTTTGTGTGTATTTATCGGAAAAACAGAAACCCGCCGCCGTCATTCCCG
CGCAGGCGGGAATCTAGAACCCAACGCGACAAAAATTTATCCGAAGCGACAACAATCTTT
TCATCGTCATTCCCGCGCAGGCGGGAATCTAGAACGTAAAATCTAAAGAAACCGTTTTAC
CCGATAAGTTTCCGTGCCGACAAACCTAGATTCCCGCCTGCGCGGGAATGACGGGATTTT
AGGTTTCTGATTTCGGTTTTCTGTTTTAAGGGAATGACGAGACTTGAGATGGCGGCATTT
ATCGGGAGCAACTGAAACCACCCTGCCGTCATTCCCGCGAAAGCGGGAATCTAGGTTCGT
CCGGTTTCGGTTATTTCCGATAGATTCCTGCCGCGTTGGGGGTCTGGATTCCCGCCTGCG
CGGGAATGACGGGACTTTAGGTTTCTGTTTTTGTTTGAGACCTTTGCAAAATTCCTTTCC
CTCCCGACAGCCGAAACCCAAACACAGGTTTTCGGCTGTTTTCGCCCCAAATACCGCCTA
ATTTTACCCAAATACCCCCTTAATCCTCCCCGGATACCCGATAATCAGGCATCCGGGCTG
CCTTTTAGGCGGCAGCGGGCGCACTTAACCTGTTGGCCGCTTTCAACAGGTTCAAACACA
TCGCCTTCAGGTGGCTTTGCGCACTCACTTTAATCAGTCCGAAATAGGCTGCCCGCGCAT
AGCGGAATTTACGGTGCAGCGTACCGAAGCTCTGTTCGACCACATATAGTGGATTAAATT
TAAACCAGTACGGCGTTGCCTCGCCTTGCCGTACTATTTGTACTGTCTGCGGCTTCGTCG
CCTTGTCCTGATTTAAATTTAATCCACTATAACGGGTCTTCGATAAATATCGGTTACGTT
TGGTTTGCGTTTCCGTCAGCGGACGGTTGCGGCAGGCTTTGCGCATAATGCCGTCCAACA
ACTGATGTTCTTCCAGATGTTGCCGGTTTTCCGCACTGTCATAGCCTTTGTCGGCATAGA
CGGTCGTACCTTTGGGCAGTCCTTCCAACAAAGGCGGCAGGTGTTTGCACTCATGGGCAT
TGGCGGGGGTAATGTGCAGTTTCTCGATATAGCCTTCCGCATCGGTACGGGTATGTTGTT
TGTAACCGAGTTTGTAGAGGCCGTTTTTCTTGATCCAACGGGCATCGCTGTCCTTACTCG
GTGTGGTTTGGCCGTTGATTTGTCCTTCTTCATCGACTTCTATGACCTGACGCTGTGTGC
TGCCGGCGGTCTGAATAATGGTGGCATCAATGACGGCGGCGGATGCTTTCTCTACTTTTA
AGCCTTTTTCGGTCAGTTGGCGGTTGATCAGTTCCAATAATTCGGACAGGGTGTTGTCTT
GCGCCAACCAGTTGCGGTAGCGGCATAAGGTGCTGTAATCGGGAATGCTCAGTTCGTCAA
AACGGCAAAACAGGTTGAAGTCGATGCGGGTGATGAGGCTGTGTTCGAGTTCGGGATCGG
AGAGGCTGTGCCATTGTCCGAGCAGGACGGCTTTGAACATGGACAACAGGGGATAGGCGG
GACGGCCGCGGTAATCTCTGAGGTAACGGGTTTTTGACGGTTCAGGTATGGTTCGATCGG
CTGCCAATCAATCACCCGGTCCAACTTCAATAGCGGGAAACGGTCGATGTGTTTGGCAAT
TATGGCTTGTGCGGTTTGCCGGAAGAAGGTGCTCATGAGAAATCCCCTAAATGTCTTGGT
GGGAATTTAGGGGATTTTGGGGATTTTTGCAAAGGTTTCCGCCTGAAACATTATGAGATT
TCAGGCGGCATTGGATTGCTTGGCGGAATATTTTTAAAAAGGCTTACGCGCCGTAAACGG
GGTATTTATTGCACAAAGCAGTTACTTGTTTGCGGACTTTGGCGAGGTTGGCTTCGTCTT
CGGGGTTAGACAATACGTCGGCAACCAAGTTCGCCAATACGCGCGCGTCGGCTTCGTTAA
AACCGCGTGTGGTCATGGCAGCGGAGCCGATGCGGATGCCGGAGGTAACGAAGGGTTTTT
CCGGATCGTTCGGAATGGCGTTTTTGTTGACGGTGATGTGCGCTTTGCCCAAAGCGGCTT
CGGCGGCTTTGCCGGTAATTTTCATCGGTTGCAGGTCAACGAGGAAAACGTGGCTTTCGG
TGCGGCCGGAAACGATGCGCAAACCGCGTTTAACCAACTCTTCCGCCATGGCGGCTGCAT
TGATTTTCACTTGTTTTGCGTATTGTTTGAACTCGGGTTGCAATGCTTCTTTAAACGCCA
CGGCTTTGGCGGCGATAACGTGCATCAGCGGACCGCCTTGCAGGCTTGGGAAGATGGAAG
AGTTCAACGCTTTTTCGTGGGTATTGTCGCGGCACAAAATTACGCCGCCGCGAGGACCGC
GCAGGGTTTTGTGGGTGGTGGTGGTCACGAAGTCGCAGAACGGCACCGGGTTGGGATATT
CGCCGCCGGCAACCAGACCGGCATAGTGCGCCATATCGACAAAGAGGTATGCGCCGACTT

## Appendix A

TATCGGCGATTTCGCGGAATTTTGCCCAGTCGATTTGTAACGCGTAGGCAGACGCACCCG
CCACAATCATTTTGGGTTTGTGTTCGAGCGCGAGGCGTTCGACTTCGGCATAATCGAGCA
CTTCGTTTTCATCCAAACCATAAGTAACGGCGTTGTAGAGTTTGCCTGAGATATTAACGC
TCGCGCCGTGGGTCAGGTGGCCGCCGTGCGCTAGAGACATACCCAAAATGGTGTCGCCTG
GTTTTAAAACGGAAGCGTACACGGCTTGGTTGGCTTGCGAGCCGGAGTGCGGTTGGACGT
TGGCATAGGCTGCGCCAAACAGTTCTTTTACGCGGTCAATCGCCAATTGTTCGACAATAT
CGACGTATTCGCAGCCGCCGTAGTAGCGTTTGCCGGGGTAGCCTTCGGCGTATTTGTTGG
TCAGCTGGGAACCTTGCGCGTCCATTACGGCGCAGCTGACGTAGTTTTCGGAAGCAATCA
GCTCGACGTGGTCTTGCTGGCGTTGGTCTTCTTGGGCAATGGCTGCTGCCAAATCGGGGT
CGTATTGTGCGAGGGTAACGCTTTTTGAAAACATGTTCTCGGCTCCTTTGTGTAATCAGG
GTATCATGAGTGTTTTTTGTATAAAAAAATATTTCAAAACCTAAGGCAGATAGCCCATAA
TGCGTAAATTTTCTTTGGCATTATCAGGTAATTTATTTAACATGCTGGTTTTTAGCGTCT
CATTACCTTTATTTAGTACAAGACTAATCAAAAGCAATACATTAAATGGTAAATTTTCGG
CAGTTTGTGCAAAATCAATCAAATAACCAGCACCAACCACATCTTTATCAGCCATAACCC
GCTGATACACCACTTGCTAAGACAACTCTCCTGCCCCACCATGTCTTAAAATCAGATGAA
TGGCTTGTAATGCTGTGTTTGGTGTCTCACAAATTGCCAAGATCTGCTCAGAAGCTTGCT
TGATGGCTTGTATTTTTGTTTGATTACTCACAATTTCCCCCTATTTTAATAATTAACTTA
AATGCGGTCAAATTCACAAAATACAAGCTTTACCTCTAATCACCCATCACTCGACCTTCT
CGGCGTGGATCGGCACCACCAACCAGCCTGCTTGGCTCGATAATAATGGCTTGAACACCT
GAATTTAGCTCACGCACATCAGTCTTATAGCCCAAATCATTTAATGCTTGTTGCCACTGG
ACGGCGGTTGTACCCGTTTCTAGTTCATAGCTACCAAAGCGATTTAATAAATTGGGTGCA
CTGATGGCATTTTGGATATCCATATTCCAGTCACTATGTGCCACAATCGTCTTAGCGACA
TAGCCAATGATACGGCTACCACCTGGGGAGCCGATTGCCATATAAGGCTTGCCTGCTTTA
AATACGATGGTTGGTGCCATTGAGGAGCGTGGTCTCTTGCCGGGCTCGACACGATTGGCG
ACCTGTTTGCCCTGCTTTATTGGCTCAAAACTAAAGTCTGTCAGCTCATTATTCAGCAGG
TAGCCATTTGCCATCAAAGTTGAGCCAAACGCATTTTCAATGGAAGTCGTCATTGATAGC
ACATTGCCCGCCTTATCCACAATTGATATATGACTGGTAGAAGGTAACTCAATCGCTTGT
GAGGACACCCACTCATGAATAAAATCGCCTGCAGATACGCTAGGCAATGCCTTATCCGAC
TGCTCAAGCAGCTGGCTGCGATGTTTTAGGTAGTCTTTAGAAATCAACTGGCGAATGGGT
ACTGGTACAAAATCAGGGTCGCCCAAATATACATCACGATCCGCAAACGCAAGCCTAGAA
GCGTCGCCCAAGAGACGTAAACCTTCAGCATCATACCCCACCTGATTGGGTGAAAATTCA
TTTAAAATCCCCAAAATCTGACCCACAGCAATCCCACCTGAGCTTGGTGCACCCATACCG
CATACTTCATAAATACGATAAGTCACACAAACAGGCGGGCGTTCCACCACTTGATAATCA
GATAAATCTTGTAAGGATAATTGACCGGGGTTATCCTTAGCATTTTGGACAACTGAAACG
ATATTTTGGGCATATTTACCAGTATGCAGAGCTTTTGCACCTTGAGCTGCTAACGCCTGA
ACACTGTCAGCAAATTCTAAATTTTTCAGCAAGCTGCCTGCTTGTAGCGGCACACCATTC
GGCAAAAAATAAGCGGCTGTTTTTGGATAGCGTGCCAAATGCTGCTGATTTTGCTCAACC
GAGATGGCAAGCCTTGGCGACACCTCAAAGCCTTGTTTTGCCAAGCGGATCGGTGTATCA
AATAATTTTCCCCAAGGCAATACACCGTATCGCTGATGTATTGTCTCCATCAGTTTAGGG
ATAGCAGGCGTACCCACCGAGCGACCACCGACCACCGCTTCCATAAATTTCAATGGTTGA
CCATCTTTATCCAAAAATAATTCCGGCGTCGCACGCATCGGTGCCGTCTCACGCCCATCA
AATGTGGTCAATGTTTTGGCGGTATTATCCCAATACAACACAAATGCACCACCGCCCAAG
CCTGACGACTGTGGCTCTACCAAGCTTAGTGTCGTCTGCACCGCCACCATCGCATCTGCA
GCGCTACCGCCTTGCTTTAAGATATCATAGCCAGCTTGTGTTGCTAATGGATTGGCTGAC
GCTACCATAAAATCACTTGCAATCACCTGCTTTTGTTCGGTCAGTCCCGTTGCATGTTCA
GGCGTGTGAGCGTCTGCACCTGTGATGACAGCAGAATGAGTATTAACCTTACCTTGATTG
GCATGGATGACTTGACATCCAGAGATTGTCATAGACATTATCAATGCAGTCAATAAATAT
GTTTTAGCCACAAGCACTCCTTCGCCTGAGTTTGATTGATGATTCATACAAGGCATGCTG
ATTATTGTATTTAATATGGCTAAATAATTCAATCCAAACTATCAATCTTGACCATCAAAA
AAAGACCGCTAATGTCATCAGCAGTCTTTTTTGATATTTATTTTAAGATATTAAGTAATC
AGACCTTTGGGCTATGCTCTTCAATGAGTGGTTTTAGCTCACCTGATTGGTACATTTGTA
GGATAATATCACTACCACCGATTAACTCACCATTAACCCAAAGCTGTGGAAAGGTTGGCC
GACTGGCGATGAGTGGTAGAGTACTGCGAATTTCTGGGTTTTCTAGGATATTAACAAAAG
CAAAGGGTCTGCCAATTGGGTCAGCACCTCTACTGCACGCGCTGAAAATCCACATTGGGG
AAACTGGGGCGTGCCTTTCATATATAGTGGATTAACAAAAACCAGTACGGCGTTGCCTCG
CCTTAGCTCAAAGAGAACGATTCTCTAAGGTGCTGAAGCACCAAGTGAATCGGTTCCGTA
CTATTTGTACTGTCTGCGGCTTCGTCGCCTTGTCCTGATTTTTGTTAATCCACTATACAG
TAGGAAAGGCTGAAAATTTATGCGTAAAGCGTGATATTGTCAACGTTTTTATCAACCGGA
CGGCGGTGTTAAAAGAAAATTTTGCCGTATCCGATAAAACACTGGATAAAAATATTATCT
TTGTTATAATTAATGTAAAGATTCAATTTGACTTTTTAACCGTAAACCAAGAGAGGAAAG
CGATATGTTCCCAGAATACCGTGATTTGATTTCCAAATTGAAACAGGAAAATTCCCGCTT
CGCCCGTCTGTTCGACGAACACAACGAGCTGGACGATAAAATTACCGGTCTGGTCAACAA
TCCGGTTACCAGCGGTGCGGAAACCATCGATGAGCTGAAAAAAGCCAAATTGAAACTGAA
AGACGAGTTGTACGCCATCCTGCAAAAAGCAGCGGGAAAATAATTCGGGTTTGAGTTTTT
GAAATGCCGTCTGAAATGTGTTCAGACGGCATTTTTGTCATTTGACCGGAAGGCTTGTGC
TGTTTGAAATAACGGCGGCGGTATCGGATTGCCGCCGCCGTGTACTTGTGTGAACGGCTG
TCTGTCTATTTTGCGTGCAGGCGGTCGAGATAGGCGACTTCTTCGCTGCTGCCCATGAAG
ACGGCGACGCGTTGGTGCAGGTTTTCGGGCTGTATGTCGAGCATGGCTTGATATGCGTTG
CTTGCCGATGCGCCCGCCTGTTCGAGTATCAGGCTCATAGGGTTGGCTTCGTACATCAGG
CGCAGTTTGCCGGGTTTAGCGGGGTCGCGTTTGTCTTGCGGATACATGAACACGCCGCCG
CGCATCAGGATGCGGTGGATTTCGGCAACCATACTGGCTACCCAGCGCATATTGTAGTTT
TTGCCGCGCGTACCGGTTTCGCCCGCCAAGAGCTCGTCGATGTATTGTTGGACGGGGGGC
AGCCAGTGGCGGCGGTTGGACATATTGATGGCAAATTCTTTGGTACTTTCGGGTACTTTC
GGGTTTTCTTTGGTCAGCACAAATTCGTTTTCGGCATTGAGCGTGAACATATATACGCCA
TGTCCGAATGTGAATACGAGCTGGGTTTGAGGCCCGTAAAGAACGTAACCAGCGGCAAGC

## Appendix A

TGCTGTCTGCCCGTTTGAAGGAATGATTCGGTTGCCAATGCGCCTTCGGGTTTTTCAAGG
ATGGAGAAAATCGTACCGACGGAAATGTTGACATCAATATTGGACGATCCGTCTAAAGGG
TCGAATAGGACGAGATAGCGTCCGTTTTCACCGGCATTTACGAAAGTGTCTTCTTCCTCG
CTCGCCAGCCCGGCAACGGCAGAATTGGCTTTGAGTGTGTCAATCATGATGTTGTTGGCG
ATAACATCCAGTTTTTTTTGGTCTTCGCCCTGAATATTGCCCGTGCCCGCCATACCCAAT
ACGCCGGCCAGTGCGCCGAGGCGGACTTTGGCGTTGATTTCGGTGCAGGCGGAAACAACG
GACAGTAAAACGCCGCCGAGTGCTTCGGGCAGCTGGTTTTGTTGCAGGTGTTCGGGGAGG
AATCGGGTCAGTGTGTCCATAGTTTGCTCGTTTCGGAAAGGTTTGTGCCGTCTGAAAGGC
GGCAGGTTATTGTGGCGTATTCCTTTGGTGCGTTTTGCAGGATAGTCTAGGGGATTGTAG
TGTAAAAAAACGGCATGGGGCAAGTCGGAACGCGGCAGGCGGATGAGGGGATATTTATTC
TTAAAAGTGCCGACTGCCGGTATATCGTCCGGTTTTGTTTATTTGACGGGAGATGTTGTC
TGAAGGGTTTCAGACGGCATCGGGGTCAGCGGATTTTGCTGTCCAAAAGGTAGCGCGAGC
CTTCGTCTTGCGCCAGCAGCCGCGTCAGGGCGGGGAGGTTTGCCGCCAATTGTTCCGCCA
ACAGATAGGGCGGATTGATGACGAACATTCCGCTGCCGTGCATACCGAAACCGTCGGCTT
TCGGCGCGTGGACGTGAAGTTCGGCGTGAAGGTAGTTGTCGGGCAGGAGTTTTTCAATT
CTTCGGGCAGCTTGCGGCTTTCTTCGCGGCTGAGGCAGGGATACCAAATGAGATAACAGC
CGGACTCAAACCGTTTTAAAGCGGCTTTCAGCGTTTCCGTTACACGCCGGTAGTCCTGTT
TTTCCTCATAGGGCGGGTCGATGAGGACGGTGGCGCGGCGCGGCGGGGGCGGCAGCAGGG
AAATCAGCCCTTTGTAACCGTCTTCGCGTAATACTTGTCCGCGTTTGCCCAATCCTGCTT
CGCCCATATTGTTTTGCAGATGGACAAAGTCGGTGGGGTGCAGCTCAAACAGGCGTAATT
TGTCGCCGACGCGGGTCAGCGATTGCGCCAGCCACGGAGAACCGCAGTAAAGTTTGGGCG
AGGGCAGGATTTTTTGTATGTGCGCGGCAAAGTCAGAGAGTTCGGCAGGCAGGTTTTGCG
CCTGTCGGAGCAGGGCGATGCCTTGTCGGTATTCGCCGACTTTCTGCGCCTCGCTGCCTT
CGAGATTGTACACACCCGCGCCGCCGTGCGTGTCGATGTACCAGTAGGGCTTGTCTTTGC
GGTTGAAATATTGCAGCACTAAAAACAAGGTGAAATGTTTGAGCATATCGGCGTGGTTGC
CGGCGTGGAATGCGTGTCTGTAACTGAGCATAGTCGGTAAAACGGCGGGATATTCGGATG
CCGTCTGAAGCGGGGTTCAGACGGCATAAACAGGGATGGATGGGAAATCAGCGGCTGCCG
CCGATTTTGCTTTCTCTGCCTTCGAGCAGCTTGACGATATTACTTTTGTGGCGGAACAAC
ACCAGCAAAGCAATGGCGACGGTCGCCCAAACCCACGAGACGTGCGGCATAAAGAAGGAT
GCGGCGACCGGTGCGGCGATTGTGGCGGTTAATGCGGCAAGGGAGGACACCTTGAAGCCG
AATGCCATAACAAGCCAAATCAACGCGCAGACCAAGGCAGTTGCGGGAGAGAGTGCCAGA
AGCACGCCCAATGCCGTTGCCACGCCTTTGCCGCCTTTAAATCCGAAAAACACCGGCCAC
ATATGCCCGACCAGCGCGGCGAGTGCGACGGCCGCGATTGCGCTGTCGGATAAACCGAGC
GGTTCTTGAAGCACGCGTGCAAGCAAAACGGCAACTAAACCTTTGGCGGCATCGCCCAAG
AGCGTCAGCGCGGCCGCCTTTTTTTTGCCGCTGCGTAAAACATTGGTTGCCCCCGGATTG
CCCGATCCGTAGGTGCGCGGGTCGTCCATGCCGTAATACTTGGACACGATGACGGCGAAA
GAAAGTGAGCCGATCAGATAGGAAACAGCAACAGCCGGTATGTTGAACATTTGCGGTACT
TTACTTAGAATGGTGCGGTTATTTTAGCAAAAAACGGGGCGGATTATGGATAAAATCTTT
TTGCACGGCATGAAGGCAGATACGCTTATCGGCGTGTACGGCTGGGAACGCGAACGGTTG
CAGACCCTGATTGTCGATTTGGACATCGGTGTTCCCGAGAAAGCGGGTTCGGACGACGAT
ATTGCCAATACGGTGCATTATGCCGAGGTATGCGAAACGCTGCGCCGACATCTGAAAGAA
CAGGATTTCCTGCTTTTGGAAGCGTTGGCGGAATATATTGCCGATTTGGTTTTGGGATAT
TTCGGCGCGGTGTGGGTGCGCGTGAAAATCGTCAAGCCGGGTATTTTGGAAGGCGTGCGC
GAGGTTGGCGTGGAAATCGAGCGCGGCAAGCGTGAAGATTGAACGGCAGAATAGGAAACG
GAAAGGAGATATGAAGTGGATTTGAGGGAAGTAAAATTAGGCGGCGAAACCATTTACGAG
GGCGGTTTCGTCAGTATCAGCAGGGATAAGGTCAGGTTGCCCAACGGCAATGAAGGGCAG
CGTATCGTCATCCGCCATCCGGGTGCGGCATGCGTGTTGGCGGTTACGGACGAAGGGAAA
GTGGTTTTGGTGCGGCAGTGGCGTTATGCGGCAAATCAGGCGACATTGGAACTTCCTGCG
GGCAAGCTGGATGTGGCCGGCGAGGATATGGCAGCGTGTGCGCTGCGAGAATTGGCGGAG
GAAACACCTTATACCGCCGACAGCGTACGCCTGCTTTACAGTTTTTATACGGCGGTCGGT
TTTTGCAACGAAAAAATGTATCTGTTCGAAGCGGAAGGCGTGCGTTTGGGCAGTACGCTT
GCCAATGACGAAGACGAGATTACGGAAACCGTATTGATGTCGAAAGAAGAAGTCCGTCAG
GCATTGGCAAACGATGAAATTAAAGACGGCAAGACATTAATCGGTTTGCAATACTGGTTG
ATGAAGGATTGACAGGATGTTGGACTTGCCCGCCGGATTGGATCGGCGGGCGGTTTGTTT
GGCGGATGGGATATGCCTTTTCGGCTTGTATCTGGGCGCGTCCTTTAAAGTCATTCGTGC
TTTAGTAATAAGAGAGAAAAGGGGATGATAATTACCTAAAAGAACGTGATAATTTTTAAA
ATGGTTAATAATGAATATCTTTGTTACTAATTTTTGTTATTGGTTTATTAGTTTATTGGC
TATTTCTTATATACCATCTATTAATGCATGGCATGATGAATTAATAGATGATATTAATTT
TGGCAAAAGGGTTATGATGGTTACTTTTTTTGCATTTTTAGGCACGGTAATAGAGCGTTT
TTTTAAGAAAAAGCCTTGGTGGTTTTATCCTGCCAAGGCTTTTTCTTTGTTACAGACCTA
AAAGCTCAATTTGAATTTGAGAACGGTAATTGGCACAGCCAGTATTTAAACAAGCGAAGC
TAATTTATAGATTATGTCAAAACAAAGGGAGGCAATTTGTTGCGGTTATTTGACTGCCGC
CCCTATCTTCAGCCCGAGCCAGGTCAGCAGCAGCGAACCTGCCGTGTGCAGGAAAATATT
GGCAAGTGCTGAAGCGGGACGGTTCAATTGGAGCAGGGTTACGGTTTCCAGCGAAAATCC
GGAAAGCGTGGTCAGGCTGCCGAGAAAACCGGTAATCAGCAGCAGCTTCCATTGCGGGTG
GTTGACGGTTTCGGCAAAGATTCCGATAAGAAAAGCGCCTATCCAGTTGGCAAACAGGTT
GCCTGTGGCGGGAGGTATTGATGCGGGAACGGCGAGGTTGAGCAGCCAACGCGCCGTTGC
ACCGAGTGCCGCACCGATGGAAAGGGGGATGATGTTGGAAAGCATGGTTTTGCCTGTCTA
TGCCGTCTGAAGGCTACCGCCATATGCCGCGGTCGGACTTAAGATAGCGGTTGTCGTCGA
AAGTGTTAATCCAATGGGGCTTCAGTGCAACAAATATGGCAGTTGAAATGCCGCTGAGGA
AGGCTTCCGCCCACGCCAGCAGAATAAAGACGGGCAGGGCGGTCGTCCACAATATTTCGG
ACGGAAAAGCGTTTGCGGCATCCAAAATACCGGTCAGCACCAGCCCGGTCAGCAGAATGC
CGGCGGCGGAAGCGAGAAAGCCGTTGACGAAAATAAAGATGAAAATATTGGGCGGCAGGC
GGTTGACCAGCATACGCGACAGGCGGTTGACGGTCAGCGCGGGCAGTATCAGCACCAAAG
CGTTCGGCGGATATGCGCCGACAGAACCGGCAAACAGCAGGCAGTAGGGCAGCATCAGCA

## Appendix A

GCGCGGCAAGCCAAAGGGCGGCGGAAGTGCCCATCATCAGTGCAACCAAATTGACGGCGA
GCAGGTGGTAGTTCATCTGGGCAAGCTGTCCGCCGCCGGCAGAGGCGTTCAGACACCATG
CTGCGGAAAAAATTACGGTACACAGGGGAAGGGCGGAACGGTAGCGGGCAAGCGAGCGGA
ATGCCGACGGCGCGGAAGCTGCCAGTATCAGGATAAGGACAATCCACGAAACCGACAGTA
CCATATCTGAAAACCAGACTGTTTGGAAAATCATGGCAATGCCGCAAAGATTAAGGGAAG
GGACGGCTATTATACTGTCGGCGGGGGCAAACCGAAAGCCGAATCGGTTTCGGCAGAATT
GCCGGCCGGTTGTTTTTTTTGGGATGGAAACACGTTAAAATAAACCCGTTTAATCGTTTG
TCTTGCGGGAAACGGCATATGTTTTGGATAGTTTTGATCGTTATTTTGTTGCTTGCGCTT
GCCGGCTTGTTTTTTGTCCGCGCACAATCCGAACGCGAGTGGATGCGCGAGGTTTCTGCG
TGGCAGGAAAAGAAAGGGGAAAAACAGGCGGAGCTGCCTGAAATCAAAGACGGTATGCCC
GATTTTCCCGAACTTGCCCTGATGCTTTTCCATGCCGTCAAAACGGCAGTGTATTGGCTG
TTTGTCGGTGTCGTCCGTTTCTGCCGAAACTATCTGGCGCACGAATCCGAACCGGACAGG
CCCGTTCCGCCTGCTTCTGCAAACCGTGCGGATGTTCCGACCGCATCCGACGGATATTCA
GACAGTGGAAACGGGACGGAAGAAGCGGAAACGGAAGAAGCAGAAGCTGCGGAGGAAGAG
GCTGCCGATACGGAAGACATTGCAACTGCCGTAATCGACAACCGCCGCATCCCATTCGAC
CGGAGTATTGCTGAAGGGTTGATGCCGTCTGAAAGCGAAATTTCGCCCGTCCGTCCGGTT
TTTAAAGAAATCACTTTGGAAGAAGCAACGCGTGCTTTAAACAGCGCGGCTTTAAGGGAA
ACGAAAAAACGCTATATCGATGCATTTGAGAAAAACGAAACAGCGGTCCCCAAAGTCCGC
GTGTCCGATACCCCGATGGAAGGGCTGCAGATTATCGGTTTGGACGACCCTGTGCTTCAA
CGCACGTATTCCCATATGTTCGATGCGGACAAAGAAGCGTTTTCCGAGTCTGCGGATTAC
GGATTTGAGCCGTATTTTGAGAAGCAGCATCCGTCTGCCTTTTCTGCAGTCAAAGCCGAA
AATGCACGGAATGCGCCGTTCCACCGTCATGCAGGGCAGGGGAAAGGGCAGGCGGAGGCA
AAATCCCCGGATGTTTCCCAAGGGCAGTCCGTTTCAGACGGCACGGCCGTCCGCGATGCC
CGCCGCCGCGTTTCCGTCAATTTGAAAGAACCGAACAAGGCAACGGTTTCTGCGGAGGCG
CGAATTTCTCGCCTGATTCCGGAAAGTCAGACGGTTGTCGGGAAACGGGATGTCGAAATG
CCGTCTGAAACCGAAAATGTTTTCACGGAAACCGTTTCGTCTGTGGGATACGGCGGTCCG
GTTTATGATGAAACTGCCGATATCCATATTGAAGAACCTGCCGCGCCCGATGCTTGGGTG
GTCGAACCACCCGAAGTGCCGAAAGTTCCCATGACCGCAATCGATATTCAGCCGCCGCCT
CCCGTATCGGAAATCTACAACCGTACCTATGAACCGCCGTCAGGATTCGAGCAGGTGCAA
CGCAGCCGCATTGCCGAGACCGACCATCTTGCCGATGATGTTTTGAATGGAGGTTGGCAG
GAGGAAACCGCCGCTATTGCGGATGACGGCAGTGAAGGTGCGGCAGAGCGGTCAAGCGGG
CAATATCTGTCGGAAACCGAAGCGTTCGGGCATGACAGTCAGGCGGTTTGTCCGTTTGAA
AATGTGCCGTCTGAACGCCCGTCCTGCCGGGTATCGGATACGGAAGCGGATGAAGGGGCG
TTCCCATCTGAAGAAACCGGTGCGGTATCCGAACACCTGCCGACAACCGACCTGCTTCTG
CCTCCGCTGTTCAATCCCGAGGCGACGCAAACCGAAGAAGAACTGTTGGAAAACAGCATC
ACCATCGAAGAAAAATTGGCGGAGTTCAAAGTCAAGGTCAAGGTTGTCGATTCTTATTCC
GGCCCCGTAATTACGCGTTATGAAATCGAACCCGATGTCGGCGTGCGCGGCAATTCCGTT
CTGAATCTGGAAAAAGATTTGGCGCGTTCGCTCGGCGTGGCTTCCATCCGCGTTGTCGAA
ACCATCCCCGGCAAAACCTGCATGGGTTTGGAACTTCCGAACCCGAAACGCCAAATGATA
CGCCTGAGCGAAATCTTCAATTCGCCCGAGTTTGCCGAATCCAAATCCAAGCTGACGCTC
GCGCTCGGTCAGGACATCACCGGACAGCCCGTCGTAACCGACTTGGAAAAGCACCGCAT
TTGTTGGTTGCCGGCACGACCGGTTCGGGCAAATCGGTGGGTGTCAACGCGATGATTCTG
TCTATGCTTTTCAAAGCCGCGCCGGAAGACGTGCGTATGATTATGATCGATCCGAAAATG
CTGGAATTGAGCATTTACGAAGGCATCCCGCACCTGCTCGCCCCTGTCGTTACCGATATG
AAGCTGGCGGCAAACGCGCTGAACTGGTGTGTTAACGAAATGGAAAAACGCTACCGCCTG
ATGAGCTTTATGGGCGTGCGTAATCTTGCGGGCTTCAATCAAAAAATCGCCGAAGCCGCA
GCAAGGGGAGAAAAAATCGGCAATCCGTTCAGCCTCACGCCCGACGATCCCGAACCTTTG
GAAAAACTGCCGTTTATCGTGGTCGTGGTCGATGAGTTTGCCGACCTGATGATGACGGCA
GGCAAGAAAATCGAAGAACTGATTGCCCGGCCTCGCCCAAAAAGCCGCGCGGCAGGCATC
CATTTGATTCTTGCCACACAACGCCCCAGCGTCGATGTCATCACGGGTCTGATTAAGGCG
AACATCCCGACGCGTATCGCGTTCCAAGTGTCCAGCAAAATCGACAGCCGCACGATTCTC
GACCAAATGGGCGCGGAAAACCTGCTCGGTCAGGGCGATATGCTGTTCCTGCTGCCGGGT
ACTGCCTATCCGCAGCGCGTTCACGGCGCGTTTGCCTCGGATGAAGAGGTGCACCGCGTG
GTCGAATATTTGAAACAGTTTGGCGAACCGGACTATGTTGACGATATTTTGAGCGGCGGC
GGCAGCGAAGAGCTGCCCGGCATCGGGCGCAGCGGCGACGACGAAACCGATCCGATGTAC
GACGAGGCCGTATCCGTTGTCCTGAAAACGCGCAAAGCCAGCATTTCGGGCGTACAGCGC
GCCTTGCGTATCGGCTACAACCGCGCCGCGCGTCTGATTGACCAGATGGAGGCGGAAGGC
ATTGTGTCCGCACCGGAACACAACGGCAACCGTACGATTCTCGTCCCCTTGGACAATGCT
TGATTTTTTGCAAATGGAAATGCCGTCTGAAGACTGTTTCAGACGGCATTTTTATAGTGG
ATTAACAAAAATCAGGACAAGGCGACGAAGCCGCAGACAGTACAAATAGTACGGAACCGA
TTCACTTGGTGCCTCAGCACCTTAGAGAATCGTTCTCTTTGAGCTAAGGCGAGGCAACGC
CGTACCGGTTTAAAGTTAATCCACTATATCAGACATTTGAATTCGGATTATTCCCTGACC
TGTCCCGTGCCTTGTACGATGTATTTGTAACTCGTCAGCTCTTTCAAACCCATCGGGCCC
CGGGCGTGGAGTTTTTGCGTGGAGATGCCCATTTCGCAACCCAAGCCGAATTCGCCGCCG
TCGGTAAAGCGCGTGGACGCGTTGACATACACGGCGGCAGAATCGATATGAGTCGTGAAA
TAGTCCGCAGCGTGGCGGTTTTCGGTAACGATGCCGTCTGAATGGTGTGTGCTGTGGGTT
TCGATGTGCCAGACCGCCTCTTCGACCGAAGCGACGGTTTTCACAGCGAGGATGTAGTCT
AAAAACTCGGTATCGAAATCGTCTGCACCCGCCGCTTCGCCGCCGATATGCCGCGCCGCC
TGCGGATCCAAACGGAAGCGGATGGGCGGCAGTCCGGCTTCTATGCGGTCGCGAACCAAC
AGCCGTTCGAGCTTGGGCAGGAAGTCGGCAGCAATGTCTTCATGTACCAGCAGCACTTCC
ATCGAGTTGCACACGGACGGACGGCTGGTTTTGGCGTTGTACACGATACGGAGCGCCTTG
TCCCAATCCGCGTCCTTGTCGATATAAATGTGGACAATGCCCGTTCCCGTTTCAATGACC
GGCACGACGGCATTTTCAACCACCGCCCGTATCAGCCCCGCCCCGCCGCGCGGAATCAGC
AGGTCTAGATAATCTTTCGCCCTCATCATTTCGTAACTGCTTTCGCGCCCGGTGTCTTCA
ATCAGTTGGAGCGCGTCGGGGTCGATGCGGGTTTGCGCCAACCCCGTTTTCAGGGCGGCA

## Appendix A

ACGATGGCGCGTGCGGATTGGAATGCATCTTTGCCGCTGCGGAGTACGACCGCGCTGCCG
CTTTTCAGTGCCAAAGCCGCCGCATCGGAAGTAACGTTCGGGCGGCTTTCGTAAATAATG
CCGATAACGCCCATCGCCACGCGCTTTTTGACGATTTCCAAGCCGTTGGGCAAAGTCGAG
GTTTCCAGTATTTCGCCCACGGGGTTGGGCAGCGCGGCAACCGCCCTGATGCCGTCCGCC
ATCGCGCAAATGCGTTTGCCGTCCAACAAAAGGCGGTCGGTCATGCTTTCGGGAATGTTG
CCTGCCGCGGCTTCCAAGTCTTGACGGTTTGCCGCCAAAATATCTGCCGTCGCCGCTTCC
AAGCTGTCCGCCATCGCAAGCAGCGCGCGGTTTTTTTCTTCCGTATCCGCCGTGTTGACG
GATTTTTTTGCCGCTTTGGCAAGGGCAAGCTGTTTTTGTGTGTTTGACATGGGTTTCCTT
TTCTAAAATTCGGTCAGAAGCAGGCGTATTTCGGGCGTGATGGAAATCCAGTCGTCCCGA
TGGATGAACACGCCTTTCGCCTTACGCGATTTGAGCAGGTCTTCGGCGGCGGCAGAGCCG
AACAGGACGCGCCCTTTGCCCAGGGGCTGTTTGGTTGCCTTGCTGTACACGGTTACGGTG
TCCATACGGGAAAAATGCCCTTCGATTCCGGCAATGCCCGACATCAGCAGGCTTTTCCCC
TGTTCGGACAAAGCGTGTTCCGCACCTTCGTCCACATAAACGCTGCCCCGGCTTTCGGAA
TAGAACGCCAGCCATTGCTTCTGCGTCCGCAAACCTTTGGCACGGGGGACGAAAAACGAG
CCGTCCGCCTGATGTTCGGCAGCTTCGGCAAGTGCATCGGGTTTGAGCGAGGAACAGATA
TACACCGGTACGCCGGATTCGGCGGCGATGGTTGCCGCTTTGATTTTGGTCAGCATACCG
CCCGTGCCGTTTGCCGAACCCGAGCCGCCCGCCATTTCGATGATTTCATGGTTGATGTGT
TCGATTTTGTCCAGCCGTACGGCATCGGGATTGCTGTTCGGGTTGCCCGTGTAAAGACCG
TCTATGTCGGTCAGCAGCACCAAGAGGTCTGCCTGTATCATCGCCGCCACTTGCGCACTC
AATGTGTCGTTGTCGCCGATTTTCAATTCCTCAACCGAAACCGTATCGTTTTCATTGATG
ATGGGGACGGCGCGGCGTTGCAGCAGCACGGAAAGTGCGCCGCCGGCATTTTGGTAGCGG
CGTTTGTCGGCAAAGTCGGCGCGGCTGAGCAGGATTTGCGCGGACACGATGCCGTCTGAA
GACAGGTTTGCCGTATATTCTTCCATCAGCAGCCCCTGCCCGACGGCGGCGGAAGCCTGT
TTGTCGGCGATTTTGACCGGACGTTTTTGAAACCCAGCGCACCGAACCCTGCCGCAACC
GCGCCGGAAGACACCAAGACCAGCTCGTGTCCCGCATGATGCAATGCGGCAAGCTGGCAG
GTGATGGTTTGGATTTTGCCGCGCGAGAGACTGCCGTCCGAATGGGTAATCGAAGATGTG
CCGACTTTAAATACGATTCTTTTGTATTTCATTGTTTCCGTCCTTGTTGGTTTGTCCTGT
CTCGTTGCCACCTTGTGCCGCCGAATTTGCCCTGTTCTGCCGCAATTGTCAACAATCACG
CCGCGTCTGCAATAAAATGGACAAAATGTATAAAATTAATAAAATCTATGGCGGCTTATT
GAGATTTTTCAAATTTATATTGCCGTTTTGTCCAAAATGCGTATAATCCTGTCCATATTT
CTGCTGTAGGCTGATTTATTTTAGACAAGGACTACCATGCAATTAGATATAGACCGCTTG
GTTGCTTATTTCGGCGGCGTGAACGCGCTTGCCGAAGCGTTGAAACAGCACGATCCCGAA
AATGCCGCGACGACCGCCGCCATCTATAAATGGCGCACGCGCGGCTCGCTGCCTCTGGCG
CAACTGCAAAAGCTGACCGCGTTGGCGGAAGCGCAAGGCAGGCCGCTGGATTTGAATGCT
TTTTTACAAAAAAACGAATCTCTGGAGAGAACAGAAATGACACAGACCAACCGCGTTATC
ATTTTCGACACCACCCTGCGCGACGGCGAACAATCGCCCGGCGCCGCTATGACCAAAGAG
GAAAAAATCCGCGTCGCCCGCCAGCTGGAAAAATTGGGTGTGGACATCATCGAAGCGGGT
TTTGCCGCTGCCAGCCCGGGCGATTTCGAGGCGGTCAATGCGATTGCGAAAACCATTACC
AAATCAACGGTCTGTTCATTGTCCCGCGCCATCGAGCGGGACATCCGTCAGGCGGGTGAG
GCCGTTGCGCCCGCGCCGAAAAAACGCATCCACACCTTCATCGCCACCAGCCCCATCCAT
ATGGAGTACAAATTGAAGATGAAGCCGAAGCAGGTGATTGAGGCGGCGGTCAAAGCGGTG
AAAATCGCTCGTGAATACACCGACGATGTGGAATTTTCCTGCGAAGACGCGTTGCGTTCG
GAAATCGATTTCCTTGCCGAAATCTGCGGCGCGGTGATTGAAGCGGGCGCGACCACCATC
AATATTCCCGATACCGTCGGCTATTCCATCCCGTATAAAACCGAAGAATTTTTCCGCGAA
CTGATTGCCAAAACGCCCAACGGCGGCAAAGTCGTTTGGTCGGCACACTGCCACAACGAT
TTGGGCTTGGCGGTTGCCAATTCGCTTGCCGCATTAAAGGCGGCGCGCGTCAGGTGGAA
TGTACTGTCAACGGCTTGGGCGAACGTGCAGGCAATGCTTCGGTTGAAGAAATCGTGATG
GCGTTGAAAGTGCGCCACGACTTGTTCGGCTTGGAAACCGGCATCGATACCACGCAAATC
GTGCCTTCGTCCAAACTGGTGTCGACCATTACGGGCTATCCCGTGCAGCCCAACAAAGCC
ATTGTCGGTGCCAATGCCTTTTCGCATGAATCGGGCATCCATCAGGACGGGGTGCTGAAA
CACCGCGAAACTTACGAGATTATGTCCGCCGAATCGGTCGGCTGGGCAACAAACCGTTTG
AGCTTGGGCAAATTGTCCGGCCGCAACGCCTTCAAAACCAAGCTGGCGGATTTGGGCATC
GAGTTGGAAAGCGAAGAGGCACTGAACGCGGCATTTGCACGCTTCAAAGAACTCGCCGAC
AAAAAACGCGAAATCTTCGATGAAGACCTGCACGCACTGGTATCCGACGAAATGGGCAGC
ATGAATGCCGAGAGCTACAAATTCATCTCCCAAAAAATCAGCACCGAAACCGGAGAAGAA
CCGCGCGCCGACATCGTGTTCAGCATCAAAGGTGAAGAAAAACGCGCTTCCGCAACCGGT
TCCGGCCCCGTGGATGCGATTTTCAAAGCGATTGAAAGCGTGGCGCAAAGCGGCGCGGCT
TTGCAGATTTATTCCGTCAACGCCGTCACGCAAGGTACGGAAAGCCAGGGCGAAACCAGC
GTCCGTCTGGCGCGCGGCAACCGCGTCGTCAACGGTCAGGGCGCGGATACCGACGTTTTG
GTCGCCACCGCCAAAGCCTACCTTTCCGCTTTGAGCAAGCTGGAATTTAGTGCCGCCAAA
CCGAAAGCGCAGGGCAGCGGTACGATTTGAGCGTGAAAACAGACGATGCCGTCTGAAGCA
TAAAAAGGCTTCAGACGGCATTGCGGCGATAATAGGGCGCAAAACCCATTTGAAAAGGAA
AATGATGGATTCCCGAAAATTTACCGAAGCATCCAAACGGCGGTTGAGCGAATTGTTGGA
TGCCAAAAGCGAACAAGGCAACACGATGCGTTGCGACGAGGTTCAAGGTTTTATGACGGC
GCTGTTGAGCGGGCCGGACAAATTGACACCGCTCGACTGGCTGCCCGAAGTGTTGGGCGA
CGAATCGCAATTTACCGCCGCCGAACGTTCCGAAATCGAACGGCTGGTTTTGGCAATGGC
GATGGAAACAACCGCCGCGATGTCGGATAAAAAACTGCCCGATTTGTGGCTGTATGAAAA
CGAAGACGGCGGCAGCGATTTTTACACATGGTGCAATGCTTATCTTTACGGTTTGGATAT
TGTGCCGACCGATTGGTTTGAAGCCGTCGATGATGAAGCGTTTGAAGAGTTGTTTTATCC
CATCATGGCATTGGGCGGTATTTACGACGAAGAGGAAAACGGCGCTATCCGTCTGCAATT
CACAGAAGGCGAGCTGGCGGAACTGGAATCCGAGTTGCCTTATGCATTGGCGGATATCTA
CCGCTACTGGCAGGCAGTCATCAACAAACCGCAAACCGTCCGCAGGGAAGGCGAAAAAAC
AGGCAGGAACGATCCCTGTCCGTGCGGCAGCGGCAGAAAATACAAGGCGTGTTGCGGTAA
GAATTGAAGCGTTTGTTTCCATGAACCAAACGTAAAAATACCGTCTGAAACCGGATTTCC
ATGTTTCAGACGGTATTTTTCACAGGCGGTCAGTGCTGTTTTTTCATGCCGAACCGGACA

## Appendix A

AAGCCGACGATACCCAAAACAATCATCGGGACGCTCAACCATTGCCCCATCGACAGCCCC
AAGGTCAGCAGCCCGAGATAGTCGTCGGGTTGGCGTGCGAATTCGGCAATGAAGCGGAAT
ATGCCGTAGCCGCCGAGGAAGAGCGAGGCGACTTGTCCGGTCGACCGCTGTTTTTTAGAG
AACAGCCAAATGACGGTGAACAGGCAGATGCCTTCAAGTGCAAACTGATAAAGCTGCGAG
GGATGACGCGGCAGCATACCGTATTGTTGCAGCCATTCTGCCCAAAGCGGATTGTGCGCG
GCGGCTTCGGCATCTTCGTAACGCGCCTGCGGGAAGCCCATTGCCCAAAATGCGTTGATG
TCGGTAACGCGTCCCCAAAGTTCGCCGTTGATGAAGTTGCCGATACGTCCCGAAGCGAGA
CCCAGCGGAACGAGCGGTGCGACCGTATCCATCAGTTTGAGGAAGCCGATGCCGTGTTTG
CGGCCGAACAACCGTATGGCAATAACTACACCCAAAAAGCCGCCGTGGAACGACATTCCG
CCTTCCCATACCTTGAAAATATCAAGCGGATGGGCGAGGTAGTCGGAAAACTTGTAAAAC
AGGACGTAACCCAAACGCCCGCCCAAAATTACGCCCAAAATGCCCCATGTCAGGAAGTCG
TCGAGCGATTCTTTGGTAAAAACGGACAAGCCTTGCGCGATGCGCCTTCTGCCGAGAAAG
GTAAAAAGAATAAATCCGAGGATGTAGCTTAGGGCATACCAGCGGACGGCAAGCGGGCCG
ATACTGATAAGGACGGGATCGAATTGGGGATGGGTAATCATAACGGGCTTTCGTTTTCAA
ATGCCGTCTGAAAGGCATGATGCTTCAGACGGCATTTCTGCAATAAGGGTTTCAGCGCAA
ATCGCCGATGACGTTGAGGATAGCGGACAACGCGGCTTCGCCCAGCCGTAAAGAACGCTG
ACCGTTCCAGCCGAAGTCGTCGTCGGGCAGATTGGCATTGTCTTTGAACGGCATTTCCAG
CGTATAGGCAAGGCAGTTGAAACGGTTGCCGACCCAGTTGGTCGCCAAGGTCATATTCGC
TTCGCCCGGCGCATCTTTTTCGTAACCGTATTCGTCTTGGAAATCGGGGCTGGCGTTTAA
AAGGGCATTTTTAAACTGCGCTTCCAACGCGGCGATGCGCGGATTGTAGTTCGGCACGCC
TTCCGTACCTGCGACAAAGACAAAGGGCAGCCCTTCGTCGCCGTGGATGTCCAAAAACAA
ATCCACTCCGGTTTCCAGCATTTTTTCGCGCACGAAGAACACTTCCGGGCTTTTTTCTAC
CGTCGGGTTTTCCCACTCGCGGTTGAGGTTCGCGCCGGCGGCGTTGGTACGAAGGTTGCC
CAGTGCCGAACCGTCGGGGTTCATATTGGGGACGATATAGAACGTGGCGCGGTCGAGCAA
GGCGCGGGCGGTAGGGTCTTGCGGGTCGAGTAATCTGCCGAGCAGCCCCTCGATAAACCA
TTCCGCCATGGTTTCTCCCGGATGCTGGCGGGCGGTAATCCAGATTTTCAAATCGCTTTC
GACCTGATTGCCTATGGTCAGCAGATTGATGTCGCGCCCTTGCACGGTGCTGCCCAAGTC
GTCGATGCGGCACAGGCCGCTGCCTTGCGCGTCGCCGAGGAGGTTTAAATGCTGTTCTTC
GGAGTAAGGTTCGAAATAGGCGTAATACACGCTGTTGGACAGCGGAGTATGATTGACGGT
CAGTACGCCGTTTTCGTAGGAAGTCGGTACGCGGAACCAGTTGCGGCGGTCGTATGAGGC
ACACGCCTGATAGCCTTCCCAGCCTTTCGGGTAGGCGGCTTCTGCCGCGTTTTCAAAATG
CATGATGCAGTTTTGATATGCCGCGCCTTGCAGCCGGAAGTAGAACCATTGTGCAAAATC
GGAGGCGTTGTCGGGACGCAGGGCGAGGCGGATGTTGGAAGGATCGGTCAGGTCTTTGAC
GACGACCGAGCCGGCATCGAAGCGGGTGCTGATTTTAATCATGGGAAAGTCCTTGCTGTC
GCCGGTTTCTCGAACCGGATAAACCGCGATTTTACCGCCCGTATCGCAAGGCTTCAACCT
GCCCGAAAGTCTGCCGGATGCCGTCTGAAGATTGTTTCAGACGGCGTTTGGCGTTAACAT
AAGCCGAAATTGTCAACAATAGGGAGCCGTTATGGAGTCTGAAAACATTATTTCCGCCGC
CGACAAGGCGCGTATCCTTGCCGAAGCGCTGCCTTACATCCGCCGGTTTTCCGGTTCGGT
CGCCGTCATCAAATACGGCGGCAACGCGATGACCGAACCTGCCTTGAAAGAAGGGTTTGC
CCGCGATGTCGTGCTGCTGAAGCTGGTCGGCATTCATCCCGTCATCGTTCACGGCGGCGG
GCCGCAGATCAATGCGATGCTTGAAAAAGTCGGCAAAAAGGGTGAGTTTGTCCAAGGAAT
GCGCGTTACCGACAAAGAGGCGATGGATATTGTCGAAATGGTGTTGGGCGGCATGTCAA
TAAAGAAATCGTGTCGATGATTAACACATATGGCGGACACGCGGTCGGCGTAAGCGGACG
CGACGACCATTTCATTAAGGCGAAGAAACTTTTGATCGATACGCCCGAACAGAATGGCGT
GGACATCGGACAGGTCGGTACGGTGGAAAGCATCGATACCGGTTTGGTTAAAGGGCTGAT
AGAACGTGGCTGCATTCCCGTCGTCGCCCCCGTCGGCGTAGGTGAAAAAGGCGAAGCGTT
CAACATCAACGCCGATTTGGTAGCAGGCAAATTGGCGGAAGAATTGAACGCCGAAAAACT
CTTGATGATGACGAATATCGCCGGTGTGATGGACAAAACGGGCAATCTGCTGACCAAACT
CACGCCGAAACGGATTGATGAACTGATTGCCGACGGCACGCTGTATGGCGGTATGCTGCC
GAAAATCGCTTCTGCGGTCGAAGCCGCCGTCAACGGTGTGAAAGCCACGCATATCATCGA
CGGCAGGTTGCCCAACGCGCTTTTGCTGGAAATCTTTACCGATGCCGGTATCGGTTCGAT
GATTTTGGGCGGTGGGAAGATGCCTGAAGCAAAGTCGGAAAATGCCGGCTTTGGCGGAA
AACCTGTTTGTCTGGTTTCTGTTTTTGGGGTTTCGGGCAATTTCCAAACCGTCATTCCTG
AAAAAATATAGTGGATTAACAAAAACCAGTACGGCGTTGCCTCGCCTTAGCTCAAAGAGA
ACGATTCTCTAAGGTGCTGAAGCACCAAGTGAATCGGTTCCGTACTATTTGTACTGTCTG
CGGCTTCGTCGCCTTGTCCTGATTTTTGTTAATCCACTATAGAAACAAAAACAGAAGCCT
AAGATCCGTCATTCCCGCCGGGCATCTGGTTTTTTGAAATCCGGTTGTTTGGGATAAATT
CTCCGGCTTTGATTTTTTGTTTTTCCGATAACGCCATAACTTTGAAATTTCGTCATTCCC
GCGCAGGCGGGAATCTAGACCTGTCGGCACGGAAACTTATCGGGAAAAAAGGTTTCTTTA
GATTTTATAGTGGATTAACAAAAACCAGTACGGCGTTGCCTCGCCTTAGCTCAAAGAGAA
CGATTCTCTAAGGTGCTGAAGCACCAAGTGAATCGGTTCCGTACTATTTGTACTGTCTGC
GGCTTGGTCGCCTTGTCCTGATTTTTGTTAATCCACTATACGTCCTAGATTCCCACTTTC
GTGGGAATGACGGGATGTGGGTTTTTGTGCGGATTTGAACCGGTAAGGGTGGTGTGGGAT
TGGTGGTTTGCTTAGGATCTTTTGGATTGTATTTTGTATATACATTTACTTGTTGATAAA
AGATAAAATAAAATTAGAAACTAAAAGTGAGAAAAAATTAATAATAATAGGGATGTATAA
ATGTAAAGGCTCCGTTTCATAGCTAAGGTTATCTGAATATATGGAAAAAAAGTAAAAGTC
CATAAAACTAAAATATATAGATAATGCTAATGATAATAGAATTATCACTTTCCTAGAGTA
AGGATAATATTTTTTTATCGTAAAAAAATATAAAATAGGATAAATAATTGCCATATAGAT
AGATTTTGTAACAAAATTAAAAATAAATAATAAAAATAATGTAATAAAGTATATTTTTTG
GCTATGAAATAAAATTGTACATAATTGAACGAGCAGATCAAAAAATGAACTACATATAAC
AATAAATAATAACGTATTTACCATACTAAATTTAATAGGTCTCATTATCATATTTAATAA
CCACTTCATAGTATAGTGGATTAAATTTAAACCAGTACAGCGTTGCCTCGCCTTGCCGTA
CTATCTGTACTGTCTGCGGCTTTGTCGCCTTGTCCTGATTTAAATTTAATCCACTATAAA
TGCAGAGTGGGTGGAAACACTCACTTTATGGTTTGCTACGCTCTGCTCAATTAGCAACCC
GATAACCCAATATGGATAATAGGGTAATTAATCCAATCTAATTTGTCAGCATCCGTTAAT

## Appendix A

TTATTGCAAAATAAAGTATTGAATTATGTCGGGTGCAAATGACGAAATATAAGTTTCCGT
GCGGACGGATCAAGATTCCCACTTTCGTGGGAATGACGGTGGAAAGATTGTTGTTTTTCC
CGATGAATTCCTGTGTTTTTTGTTTTTCCGGATAAATTCCTGTGGCTTTGAGTTTTTTGG
ATTTCAGCCTCAATGCCGTCTGAACGCCGAATCGGGCTTCAGACGGCATTGCGTCATTTG
AAATTCAAAACCGGCCAGCCTTTTTCTTTGGCTTCTTTTTCCAGCTCGGCATCGGGGTTG
ACGGCGACGGGTTCGCTGACAAGGCGCAGCAGCGGCAGGTCGTTTTTGGAGTCGCTGTAA
AAATAGGTTTTGCCGTAGCTTTGGAGCGTTTCGCCGCGTTCGGCAAGCCATTGGTTCAGG
CGGGTGATTTTGCCTTCTTTGAGGCTGGGCGTGCCGATGTAATTGCCGGTGTAGCGGCCG
TCAGAACCGGTTTCGAGTTGTGTGCCGATGATGTTGGTGATGCCGAAAAGGTGGCAGACG
GGGGTGATGATGAACTCGTTGGTTGAGGAAATCACAAGGGTTTCGTCGCCTGCCATTTGG
TGGCTCTGCACCAGCATACGCTGCATAGGCGAGATGTGGGGATGATGTATTCCGCCATA
AATTCGCGGTGAAACTCTGCCAGCTCTTCTTTGCTGTAACGAGCGAGCGGGGCAAGGTGG
AATTTGAGGAATGCGTCGATGTCGAGGCAGCCGTTTTGGTAGTCGCGGTAGAATTTTTCG
TTTTGCGCTTCGGTTTCGGCAGCGTCAACCAAGCCTTTTTTGATGAGGTATTGCGGCCAG
GCGTGGTCGGAATCGGTGTTGATGAGGGTGTTGTCGAGGTCGAAGATGGCGAGGTTTTTC
ATTGGGTTTCCTGTTGTTTCAAAAGCTGGCGCAAAAGCGGCAGGGTGATGCGTTTGCCCA
TCGTGACGGCGTAGTTGTCCAGCGTGTCGAGCATCATCATCAGGCTGTCCATATCGCGCC
GCCAGTGTTTGAGCAGGTATTCGAAAATTTCGGAATCGACGGTTACTTGGCGTGCCGCCG
CCATACTGGCGAGCGCGTCGATTTTTTCTTGGTCGGTTAAGGGTTTGACTTCGTAAACGA
GGCAGTACGCCATACGCGTCCGCAAATCTTCGCGGATGACAAGCTGCTGGGGCGTGTATT
CCGAACCGAGCAGCAAAAAGCCTTTGCCGCTGTTGCGGAAGCGGTTGAAGATGGAAAAAA
GCAGGGCTTGTTCTTCGTTGCCCAGTTTTTCGACTTGATCGACGGCGAGGTATTCCGCCT
CGAACGCGGCATCGGTCAGCGGCATGGAGGCGGCATCGATATAGGCGGCGTTTTTGCCGG
CTTCGAGCGCCTGTGCGACCCACGCCTGCAAAAGATGGCTTTTGCCCGCGCCTTCTTCAC
CCCAGACATAGATAAACTGTCCGTGTTTGTGTCGGAGGACATAGACCAGTTCCGCGTTTT
CCGTGCCGAGGAATTTGTCGAAACTCGGATAGTCGTGTGCGGCAAAGTCGAAAATAAGCT
GGTTCACGGTTCGGCATTCCGAGGGGTGGTAAACGGGTTTATTGTACGTTGTTTTCGCGC
GCCTTTCCAATTTGAACGATGCCGTCTGAAAACGGCTTCAGACGGCATCGTTCAACCGCA
GGCAACGTTGCCGACATCGAGGCGCATATTGTGGAACGCGTTGAGCGTGCTGCGGTGGCC
GATGCTGATGATGATGCTGTCGGGCAGTTTTTGTTTCAGTGCGCGGTAGAGCAGGGCCTC
GGTCGGTTCGTCCAAAGCGGCGGTGGCTTCGTCGAGCAGGACGATTTTGGGCTTGGAAAG
CAGGGCGCGGACGAAGGCGACGCGTTGCAGTTCGCCCGGGGAGAGTTTGTGTTGCCAGTC
GTCGGTTTTATCTAATTTATCAACCAGATAACCCAAGCGGCAGGTGTTCATGGCTTCGGC
TAACTCGGGATGCTGCTTGTCAATGTCGGGGTAACAAACCGCGTCGCGCAGGCTGCCCTG
TGCCGTGTACGGGCGTTGCGGCAGGAAGAGGATGTCTTGATGCGGCGGACGGCTGACTTT
GCCGCTGCTGCCGAACGGCCAAAGCCCCGCCAGCGCGCGCAACAGCGAGGTTTTGCCGCA
ACCGCTCGGGCCGCGTATCAGCAGGGAATCGCCGTTTTTGAGGTTTATGTTGATGCCGCT
CAACAGGATTTCGCCGTTGTGGCGGAACAGAGCGACGTTTTCCTGTGGGGGACTGTTAGT
TTTTGCACAAGGAACAAATAGAGTAAAAAAACGCTGAAATCTTCGGAAGACGTGGATTTC
GGCGTTTTTTTGTATCCGGAAAAGTTACGCCAGCTTTTTCACAAAACCGCGCCGGAATGC
GCGGTTTTCTGTTTAAAGCTGACGAGATTAGGGAATTTTTAAAACTGTTTTAAGAGGTTT
TTAAAATGGATTTAATCAATACTCCGGCCATACCATTCAACACGGCCTATGATGGCGATG
TCGTCTTGGGCATTGCTCAAATCTATTTCAAACGGTGCGTAACGTGGATTTTCAGACGTT
ACAAGCAGTTTGCCCGGTATACGTTGCACACGTTTGACAAAGAGGTCATTGCCTATACGC
AAGACATATAGGCCGTCACGCGGGTCAGTTTCGGCGTGGTTGATGAGAATGGAATCCTCA
TGATTGAGCACGCCCTCCATTGAATCGCCTTTAACGGTAATTACAGACAGTTTTTCCGGC
TGTTTGGTCACATAGTTGTCAATCCAATATTTCCGGAAAGCCAAGCAGAATAAAGGTTCT
TCGCCGAAGACTGGTGCGCCATACCCTGCTGCTGCGGCTACGTTGTAGCGCGGCACGAAT
ACAAACTCGGACAGGTCGACAGGATTGCGGATAGTGTCGGTGATTCCATCAGAATTTCTA
CTTACAGAGAATGCTCCGGCGTTTTCCGGCCTGGCTTTATCGAGATACGGCAAGCCTTTT
CCGGTCAGCAGCCAGTTTAAATCACAACTGAATTTTTACTAGGTAATCGGCTGTTGGGAT
AGCTCCCTCTTTCCAAACTCTATTAAATCCAGAAGCCGACATTTCTATTTTGTTATAGAT
GTCAGATGGCTTAGCCCCATGAGGCCAAAGAAATTTGAGCCTATCTAAAAAAGTATCCAT
AGTAATCCTAATTTAACTCATTTAAGCAAAACATTAAGCAAAAAAAGAAACTCTTTTGCT
TAAATAAGATTACTCAAATAATCAATATTTTGTAAAAATAATTACGTTTTTGAGAAAATA
TTTTAGCAAAAGAGTTTCATGAAGCTGTTTTGCTAAATGTAATTCACTCATTTGCTAAAT
GACGGCGGTTAATAAACCTACTTAATTAAGGAATTGCGAGAATGAAAAAAAGCAAGAAAG
CATGACTGATTGGCATCGTGCTGACATTGTGGCTCGTCTTAAAAAGGCAGGCTGGTCTGT
GAGAGCGTTATCTATCGAGGCTAATTTAGCACCAAATACATTAGGGAAAGCTTTAGATGC
TCCTTATCTGAAAGGCGAAAGAATCATTGCAGCAGCGATTGGAGTACCCGCAGAAGAAAT
CTGGCCATCTCGTTTTGAGAAACGAAACCATAAGCCAACCTTCCCAAGATCTATAAATAG
ATAACTGTTTTGCTAAATAGTTCCAAAAGAGTACCGCATTTAAGCAAAAATAGAAAGCGG
AAAAAATGAAAATATCTGCATCTGATATTGCGAAATTAGGAATTCCGAGCCTACCAACTG
ATAGACAAGGGATTGAATACCATGCCAAGAAAAATAATTGGCAACACTGTTTTGAGCAAA
AAGGAAGAGGCCGTCCTAAAAAACTGTATGAAATCGCTTCCCTCCCTGCCGAAATCCGAG
CAGCCATCATGAAACGGCAGTCGGACGAGCTGGCGGAGAAGATGCCGAAAATGCTGCCCA
AAGTCAGACCGGGGACGGCGATGTCGGCTCAAGCACTGGCTGAAGCGGCCAAGCTGTTGA
ACGAGAAACAACGGTCGGTGGCGGATGCGCGATGTGCGGTGGTAGCGGCGGTATTGGGGA
TTAAATACGAATACGATTGCTCTGCCAAGGCTGCGGTGGCTCAGTTTTTGGGCTTGCTGG
CAGAAGGTAAATTGGACGCGGTCACGCTTGGGAACTTGGAAAAGGCCAATGACCGCAGCC
GGACGGCGAAGGTCGGCGAACGTACTTTAGACGGCTGGATTTCTGCTTATTTGAAAGCGG
AAAACGCGACGGAGCGGTTGGTTGCTTTGGCTCCGAAGACGACGAAGGCGGTCAAGCCGA
TTGAAAGTTACGGATGGTTGCCGATGTTTATGCAGTTTCACAATATTCCGTCCGCGCCAA
AGCTGGCGCACAGCTACCGCTGGTTTGTGCAGTGGGCGGAAGCGGAAAATATGCCGGTCA
ATGATGTGCCTAACTTGAGTATGGTGCGGCGCGTTTGGGAAAAGCTCCCGTTGATTATGC

# Appendix A

AGGAGCGCGGCAGGAAAACGGGGGCGGCTTATAAATCGCTGCTGCCTTATGTGAAACGTG
ATTGGGGGCTTTGAAGCCGAACGATGTTTGGATCGGCGACGGCCATAGCTTTAAGGCAA
AGGTGGCGCATCCGGTACATGGCAGACCATTTAAGCCGGAAGTGACGGTGATTATTGATG
GTTGTACGCGGTTTGTGGTCGGGTTTTCGGTCTCTCTTGCTGAAAGTTGTGTGGCGGTAT
CGGACGCTATGCGTATCGGGGTCAAGCATTTTGGTTTGCCGATTATCTATTACTCGGATA
ACGGCGGCGGCCAAACCGGCAAGACGATAGACCATGAAATCACGGGTATTACGTCCCGAC
CGGGTATCCGCCATGAAACGGGTATCGCGGGCAACCCGCAAGGGCGCGGCATCATTGAGC
GATGGTGGAAAGACAATCTGATTGAGATGGCGCGCCAGTATGAGACGTTTGCGGGTGCAG
GGATGGACAGCAGCACGAAGAACCTGATGTACCGCAAGATGGAAAGTGCGTTTAACGCTT
TGGAAAAAGGCAAGGATTTGACGGAGGAACAACAGAAATATTTGAAAAAACTGCCGAGCT
GGTCGCGTTTTATAGCGGATGTGGTCAAGTGTATCGACGAATACAACAACCGCCCGCACG
GCGAGCTGCCCCGACATCCTGACGGCGGGCATTATACGCCTAAGGCTTATCGGGAAATGA
GGCTGGAACAGGACGGTATCGCGCCGGATATGTTGTCGGCGCAAGAGCTGGCGACGATGT
TTATGCCGCAAGAGGTGCGAAAGGTACAGCGCGGTTGGCTGGATTTGTTTAACAACTCTT
ATTTCTCAACCGAGCTGGCGGAGTATCACAAAGACGAGGTACGGGTCAGCTACGATTTGA
GCGATGCGTCGGCGGTCAATGTGTTTGATATGGACGGCAAGTTTATTACTAAGGCGCAGG
CCAACGGCAATACCCGCGAGGCTTTCCCGACGGCTCGTATCGACCAACTGGCGGAAAAAC
GTCGAAAAGGCAAAATAAAGCGGGCGGAAAATGCAATCAAGCTCGCAAACGCGGAAGTCA
ATCCTGCTCTGGAACAGGCTGCGGTTTGGGACGAGCTGGGACATTTGGGCGGAAACGACA
TCGAGGCGGAGTATGCGGTATTGCCGAAAACGGGCACAGACGATTTTGTGTTGTTTGAGG
CGGATAGATAAAGGAAAACATGATGGACAAACAGCAAAATGCAGCGTTTTCGGCCGAGCT
TGTTGAAAAATTGAAACTCAAGCGAGCTCTTGGGCGGATTCAACGAGCTCAAGCAAAGAT
TCAAGGTGTTCCCGCTGAACGGAATCAGGCTCAAACGTTTTTGCCTGCGCTTGAAGGAAA
CTGCGAACCTGCTCAATCGAAGTCGGCTCTTGACGGGTAATCCGCTGGAGCAGCCAGGAA
AGTACGAAAGAATCGGCAAGTGACCTGTCTTCCAAGTCTTGAACGGCGACTTCCAGCATG
ATCAGGCGTTTTTCTAAATCGGGAAACTCTTTCATTTCAGACGGCCTTTAAAGGTTGTTT
AAAACTCAAGGATATTAAAAATGAAACAAATTAATCAAGCATTGCAACAAAAACTGGTTG
AATTTAAAGAAAAATCAGGCATGAACCAAACCCAACTGGCACGCGGTATCGGTACTTCGC
CGGCATCCATCAGTATGTATCTGAACGGCACTTATGCGGAAAAAGGCGGCAATTATGAAA
CCATCGAGCCGAAAATCGAGGCGTTTTTGGAGATGCAGGACAGTAAAGCGCAACGCGAAG
AGCTGGTGTTGGGTTTTGTATCGACTAAGACGACCCGCCGTATTGCAGAAGTGATGCGCG
ATGCGCACGAAGGCGGCGAAACAGTGGTGATCTACGGTCAGGCGGGATTGGGCAAGACTC
AGGCGGTCAAAAACTACTGCGAGAAAAACCCTGCGGCCATCTTGATTGAGGCTAATCCGA
GCTTTACGGCTTTGGTCTTGATGCGCAAGTTGGCGACTGCGGCGAAGGTATCGGCGATGG
GCAGCCTGAATGATTTGTTTGAGTCTGTATCTGACCGCCTGCGCGATTCGGGCCGTCTGA
TTGTGGTCGATGAAGCGGAAAACCTGCCTTTACGCGCCCTTGAAATTGTACGCCGTCTGC
ACGACGAGACTGGCTGCGGCTTGGTGTTGAGCGGTATGCCCCGACTGGTGGCCAACCTGC
GCGGTAAGCATGGCGAACTGGTACAGCTTTACAGCCGCGTGTCTGTTGCGCTGAATTTGG
GCGAATCTTTGCCGGATGACGAACTCTTTGAGATTGCGAAAGCGGCTTTGCCTGATGCGG
TCAAGCATCTGCTCCCTGATAGTGTACAAGCGTTGATTACGGTCATCGGGTTTAATGAAA
CGCTGGAGCTGGTGCGCCTGATGGGCGGTACGACTTATCCTTTGCGGCAGGGTTATACGA
AAAACAGTCAATCCCGTGTTGCATACTTGGAAGAGATTATCGGCAGTGAGGCGGCCGGTC
GGCTGGTGGAGGCAATGGCTCCGTGCAATCTGTTTATACCCCGTTGCGAGACGGCCTTGT
ATGAGTTGCGAAACCGTAAAATCCGCAGTCAGTTTGACCGGCAGACGGCAGGCGGTACCC
CTGCTTATGAGGCCGTTAACGATTTGGCCTTGGCACACCGCCTAAGCGACCGCCATGTGT
GGCGAATTTTAAAGCAGGCGGATAAGGAAGCGGAGCAGGAGAATTTGTTTTAGAATGGAA
TGCCATGCAGATGTATGGCATTTTATTTTGGAGAAAAATATGAAAAAGTTTTATTTTGTG
CTGCTGGCGTTGGGTTTGGCAGCGTGTGGGCAAGAACAATCGCAGAAAGCTGATGCGGAG
CAGTATTTTTTTGCCAATAAATATGAATTTGCAGATGAGAAACAGGCTTTTTATTTTGAA
CGCGCCGCCCGTTTCCGTGTATTGCAACAAGGCCTTGGCGGGGATTTTGAGAGGTTTTTA
AAAGGAGAAATACCTAATCAAGAAAATCTTGCAAAGTATCGTGAAAATATTACTCAAGCA
GTCGCTTATTATGCGGACACGAATGGAGATGATGACCCATACCGCGTCTGCAAACAGGCT
GCGCAAGATGCAGAAATCCTGATGAAGAGTATGGTAACAAGCGGTGGAGGCGGTACAACT
GATTTAGATAAGGAAAGTTATCAAAATTACCGAAAATCAATGCAAGAATGCCGTAAAACA
ATAACGGAAGCTGAAGCCAATTTGCCGAAAAAATAAAATAAACGATTCTAAGGCCGTCTG
AACAACAGGCGGCTTTTTTGTTGCCTACTGACACTGTTTCGCCCGCTGCAAAAGCCATGC
CGTTTGAAAATGTAAGCCTCTGAAAGTGCATTTTAATCTGATTTTGAGGGAGGCTTTAAT
GAGCAAAATTATTTGTCTGACTGCCGGACACAGTAACACCGACCCGGGCGCAGTCAACGG
AAGCGACCGTGAGGCGGACTTGGCGCAGGATATGCGCAACATTGTGGCTTCAATCCTGCG
TAACGATTACGGCCTGACCGTTAAAACCGACGGCACGGGCAAAGGCAATATGCCGCTGCG
CGATGCGGTCAAGCTGATTCGCGGCTCGGATGTGGCGATTGAGTTCCACACCAATGCGGC
GGCGAACAAAACGGCGACAGGCATCGAAGCCTTGTCCACGCCGAAAAATAAACGCTGGTG
TCAGGTGCTGGGCAAAGCCGTTGCCAAGAAAACCGGCTGGAAACTGCGCGGCGAAGACGG
CTTTAAGCCGGATAACGCAGGGCAACATTCGCGCCTGGCTTATGCGCAGGCAGGCGGCAT
TGTGTTTGAGCCTTTTTTCATCAGCAACGACACTGATTTGGCCTTGTTTAAGACGACCAA
ATGGGGCATCTGCCGCGCGATTGCGGACGCGATTGCGATGGAATTGGGAGCGGCGAAGGT
ATGAAAAAGTCTTTGATTGCTTTATGTGTTGCCCATTGTGCAAAGTTGAAAAACGATTTT
GGCGTACCACCGTTACCTGAAATCAAAATCACGCCAAGCCCTGTTCGGGTAGGCTCTTTG
AAACAACATCCGAGCCTGCGCTTGGGTAAATCAGGCGTGGCGGCTGCTAAACGTGCGGCG
CGCAAACGCAAGAATCGTCGTTAATCATGGGACAGGTTGCGTTTTACGAAAAGATGATTG
GGCTGTGGTCGGCCAAAAGCCGTGAGGCAAGCGAACAGGCGGACTTGGCTGCGTTTGAAT
TTGCGGAGGGCGAACTGGCCAATTATCGGGAAATGCTGAAACGGCACCTGCAAACCAAAA
GTGTGGAATAGCAATGCGTATTTTGGATATTTTTAAAAACCCGGCGACAGGCAATGTGTC
GCACTCGAAACTGTGGGCAAACGTTGCCTGCGCGGCTGGGACGTTTAAGTTTGTGATGTT
GCCCGATCCGTCGGCGGAAATTTGGGCGGTGTATTTGGGCATTGTCGGCGGCTATGCGGT

## Appendix A

GGCGCGTTCATTTGTCAGCGTGAAGCGTCAGGAGGTCGAGAATGAATCTCGTGAAACTGC
TGGCGAATAACTGGCAACCGATTGCCATTATCGCGCTTGTCGGCACGGGCTTGGCTGTGT
CGCACCATCAAGGCTACAAGTCGGCATTTGCGAAGCAGCAGGCGGTCATCGACAAGATGG
AGCGCGACAAGGCGCAAGCCCTGCTGTTGTCGGCTCAAAACTATGCGCGCGAACTGGAAC
TGGCACGCGCGGAAGCTAAAAAATATGAAGTCAAGGCGCACGCTGTCGGCATGGCTTTGG
CGAAAAAACAGGCGGAAGTCAGCCGTCTGAAAACGGAAAATAAAAAGGAAATCGAAAATG
TCCTTACTCAAGACCGTAAAAATGCAAGCGGCGGTTGCATTGACGGCTTTGGCTCTCACG
GCCTGCAGCTCTACAACCGCGCCCTCGGCTACGGAAATTAAGGTTGTCGAAAAGGCAGTC
ATGCCGACCCCGCCTGCTGCATTGATGGTCGCGCCGGTACGCCCGAATCCGCCGAAAGAC
GGCAAGACGGCCACGCTGTTGGAACACGCCGCTGAGTTTGGTGGCTATGTGGCGGAGTTG
GAAAATCAAAATCAGGCTTGGCGCGACTGGGCGGGCAATCACTCCCGCAAAGTCGGAAAC
TGACAAAAAAGCCCGCGTAGGGCGCGGGCTGAGGGTGAAAGCGGATTTTATACCTCTTTT
ACAGGGGTAGCGGCGGTAGTGCTTTTCAGCAAATCGACTGCGTGCTGACAGTTTTGCTTG
CTGGTGTAGCCTTCGCCCTGAGCGATGATTTCATGGTTGGCTGCTTTCAAACGCCAACGG
TATTCGCCTTTTGCGTCTTTATAGATTTCAAAATACATAAGGTTTCTCCTATGAATGAGT
ACACGTTTTCTTACCGCTTTAACGGCAAGTCCTGGTCATTGAGCATTTGGGCGGACAACC
CTGAAGAAGCCAGGGCGAAATTTCGGGCTGCACGAGAAAATGCGCACTATGACGGCGAAG
TTGTAGCAAAGGTTTATACATTTGTAAATATTTCGTGGGTTAAGAAATTGTACAAGCGGA
CAAAATATTTAATGGGTATCAAAGAATGACCTACCGTGAATTAGTTGAACGTCAGTTGGC
TGTGCGCCATGCCGATTTGGAATTGGGCTTAAGCCGCGCACGCGAGCAAGAGCCGTTTGT
CATTCATGTTTCCGATCTGTTGGATAAGGCAGGCATTGAGTACGCGGTACGCATGGATAA
GGATTTTCAGACGACGTTTCACCTTGAATATCCAATTACGAACTATGACACCTTTAAACG
TGCGGTTTGGCAAACTTTGGGGGCGTATTACTGTGTTTGTAATGATGGTGATGGACTGGA
GATTGCCAGCAATCGCCCTGACGGTTACGCCGTCCGTATCGTATTCGGCGATGTGCCGGT
TTAAAGGGGTTTTAAATGGACTTTGAATTTGGTTTCAGAACCCTGTGGCCGATTGCGACG
GCGGCATTTTGGTTTTGGGTCAACGGCATTTCAGGCCGCCTGAAAGAGGCGGACAAGCGT
ATCGACGACCTTAAAGAGGAGTTGCACGCGGTCAAGCTCTCTTATCACACCAAGGCGGAC
GCCAAGGCAGACAGCACTAATATTGCGGCGGCCTTGGAGCGAATTGAAAACAAGTTAGAA
AAAGTAAACGAAAAACTGGACAGGAAAGCAGACAAATCATGAGCGACCCGATTTTGGATG
CCTTGGCGCGTATTGAAAACAAGACTGATCAAACGCTGAAAAATCAGAAGGAAATGCAGG
CGGAAATTGCGCAAATTCGCCAAGACACGAAACGCACGGCCATTACATTCGGCGCACTGG
GCGGCGGCGTGATTACGGTCGGCTGGGAATTGCTTAAAGCGAAAATGGGACTGTAATTAT
GGCTCACCCGCAAGAAATCCGTGAAAAGTTACGCCGGCTCTATGTGAGCGGCGAGCAAAC
TTTGGAAACGGCGGCCTTGATGTGCGAAATCCCGCAGACCACTGCGCGTGCGTGGAAACG
TGCGGATAAGGAAAAAGGCGACGACTGGGATAAGATGCGCGCCGCTTACACTTTGGCCGG
TGGCGGTATTGAGGATTTGAGCCGTGCGATGTTGGCCGGTTTTATGGTGCAGTACAACAG
CACGATGACGATGCTGCAGGATTCGAGTACCGAAGATTTGCCACCATCCGACCGCGCCAA
GCTGTTGGCCAGCCTGGCCGATGCGTTTACGAAAACCGTATCGGCCAATGCGCGTGTGAT
GCCGGAAACGTCAAAACTGGCGACGGCTTTGGAATTGATTGAGTTCTTGATGGCGTTTGT
ACAAGAAAAACATCCCAAACATTTGCCTGCCTTTGTGGAGGTATTGGAGCCGTTTGGGGT
GGAAGTGGAGAAGAAGTTTGGTTAGAGGCCAACAAATTTTTTAAAAGAGTAATGAGGGT
GGCGGCAGGGATAGCATTAATTGCATTTTCCGTAAGCGTACTTAATGCAGTGTCCTTGAT
TTTCCCTAATTCTTTTTTCAACCAGCTTTTTTCTGAATCAGAAATCTCTGCTTGGTCTAT
TTTTGCCGCAATTAAAGCCTGAATAGTGTCGCTGTGTAGTTTGACTGTGACAACACCAAG
AATGGCGGATAGGCCGCCATCATCGGTAAGGAAGTCTATGCCCTTATGATTAATTTTGCA
GTCAAAATTTTTATGAAGTGAATCTATCGAAGTAATTTCAATTAAACCAGATTCTTCTAA
GTAATAAATATTTTTTAAAAAATATTGGAATTCATCTGATTGTAAAGTTGCTAGGTGTTG
ACTTTGAATTTCACAACCAAGAGTCAAAGCCAAGCCCAATCCTTGTTTATCAACAGGGAT
GTTAGAAGTAATAGGTAAAGAACTACTAGGGAAAAGAGAGTTATACACCTTGGTTGCTTT
TAGGCAGTTCGGGTAATTATCACTTAAAACTCGTAAGATTTTTCCTGAATACCTCTATT
TAACCAGTTCATAAATTATTCCTCATGAAAACAAAAGAATTCCTCAAATCCCTTGCCGAA
CTGGCCGCCAGTTTGCGCCAAGTCATCGAAGCGGAAGTGGACGGCTTCGATGCGTCGCCC
AAGGCTATTGCTGCACGCCGTGCCAAGGTGTTTGACCCGGTAGGCGGTTACGAGTATTTC
GTGAATACCTACTTCCCCCATTATATCCGCTCGCCTGAGAAATCCGAACTGCATGCGTTT
TTATTCAGCCGTCTGCCGGAGATTATCCGCTCCCCCAAAGGGGAAAATGAGGCGGTGGGT
GCGCCGCGTGGAGAGGGTAAGTCGACGAAGGTTACTCAGTTGTTTACGCTGTGGTGTATT
GTGACCGGCCAAAAACATTATGCTGTTATTGTGATGGACAGTATCGACCAGGCATATCCG
ATGCTGGAAGCCATCAAGGCGGAACTTGAATTTAACCCGCGCTTGAAAACCGACTTTCCG
GAAGTATGCGGGCAGGGCCGTGTATGGCAGGCCGGTACGATTGTGACGGCCAATGACGTT
AAAGTCCAAGTGGCCGGTAGCGGTAAAAAGCTGCGCGGTTTGCGTCACGGCCCTTACCGT
CCTGACTTAACTGTTTTGGACGATATTGAGAATGACGAGCAAGTCCGCAACCCCGAACAG
CGCGACAAGCTCAATGCGTGGCTGACTAAGACCGTATTGCCTCTGGGCGGTGTCGGTCAG
AAATACGATGTGATTTATATCGGCACGATTTTGCATTACGACAGCGTACTTAACCGCACT
TTGAATAACCCGTTTTGGCACGGTATTAAGTTTAAGGCGATGAAACGCTGGCCTGACCGC
ATGGATTTGTGGGACAGGTGGGAGGAACTTTTCCGAAACGACGGCGAGACGGTGGCCGAG
GCGTTTTATCAGGCAAACAAAGACGAGATGGAGCGCGGCGCGGTCACTTCTTGGGCGGCG
CGTGGCGTACTCGCGCTGATGAAAATCCGTGCGCGTGACGGCCATGCGACGTTTGATTCA
GAATATCAAAACGATCCGGTCAGTGGCGAAGATGCGCCGTTTGCCAAGTCGATGAAGTTT
TGGAACGACCTGCCGTCCGATTTGGTGTATTTCGGTGCGCTCGACCCGTCACTCGGAAAG
GCCGGGGCGAGCCGTGACCCGTCCGCGATTATCATTGGCGGTTATCAACGTGTAACCGGC
AAACTGTATGTCGTGGAGGCTCAGATTAAAAAACGTCTCCCTGATTTGATTATTGAGGAC
GTTATTCGATTGCACCGTCAATATCGTTGCAAACTGTGGTTTGTTGAGACTGTTCAATTT
CAGGAATTTCTGAAAGACGAGCTGGTCAAGCGCAGCGCGGCGCGTGGAATACCTGTCCCA
GCGCGGGCGGTCAAACCGGTATCGGACAAGCTGTTGCGGATCGAGACTTTACAGCCTCAC
ATGGCGAACGGTTTGATTCTGTTGAATGAGAGCCAACAAACGCTGATACAGCAGTTCCGC

## Appendix A

CATTTTCCAAAGGCTGATCATGATGATGGTCCTGATGCCGTGCATATGCTCTGGTCGGGG
GCGGTGGCCAATTGTGTGCCGATAGAATGGCAAAGCCCTACCGATAACGATTTTGATGAC
GAGATAAAAAGTAAATGGAGCCGATAATGGCAAAAAAGAACAATAAAACTAAAATCCAAA
AGCCCGAAGCTGCATTGCAGACGGACGTGGCTCAAATTACGGCGACCGGTCGGGTTATCG
CCGAGCATCCGTCCAATTTTATTACGCCGCAAAAGATGCGGGCCCTCTTCGAGGACGCAG
AAAGCGGCGACATCCGCGCCCAACACGAGCTTTTCGCGGACATTGAGGAGCGCGACAGCG
ACATCGCGGCAAATATGGGGACGCGCAAACGCGCGCTGCTGACGCTCAACTGGCGCGTCG
CCCCGCCGCGAAATGCGACGCCCGAAGAAGAAAAGCTGTCCGACCAAGCCTACGAAATGA
TGGACAGCCTGCCTACCCTCGAAGACCTGATTATGGATTTGATGGACGCGGTAGGGCACG
GATTTTCTGCGTTGGAGGTCGAGTGGGTATTTTCAGACGGCCTTTACCTACCCCGAAACT
TTATCCACCGCCCGCAAAGCTGGTTCAAATGGGACAAAGACAACGGGCTGCTGCTGCGTA
CCCGCGAAAATCCGGAAGGCGAAGCGTTGTGGCCGCTGGGCTGGGTCGTTCATACCCAAA
AATCGCGCAGCGTCCAGCAGGCGCGCAACGGGCTTTTCCGCACGCTTTCCTGGCTGTATA
TGTTCAAACACTACGCCGTCCACGATTTTGCCGAGTTTTTGGAGCTGTACGGCATGCCCA
TCCGTATCGGCAAATACGGCGCGGGCGCAACCAAAGAGGAAAAAAACACCCTGCTTCGAG
CGGTGGCGGAAATCGGTCACAACGCGGCAGGCATCATGCCAGAAGGTATGGAAATAGAGC
TCCACAACGCGGCAAACGGTACGACGGCAACCAGCAATCCGTTTTTGCAGATGGCCGACT
GGTGCGAAAAATCGGCGGCGCGGCTGATTTTGGGGCAAACGCTGACCAGCGGTGCGGACG
GAAAATCCAGCACCAACGCGCTGGGCAATATCCACAACGAGGTACGCCGCGATTTGCTGG
TGTCGGACGCAAAACAGGTGGCGCAAACCATCACAAGCCAAATCATCGGACCGTTCCTGC
AAATCAACTATCCCCATGCCGACCCAAACCGCGTGCCGAAATTTGAATTTGACACGCGCG
AGCCGAAAGACATCGCGGTCTTTGCCGACGCTATCCCGAAACTGGTGGATGTCGGCGTAC
AAATCCCCGAAAGCTGGGTGCGCGACAAACTGGTCATTCCAGATGTGCAGGAGGGTGAGG
CTGTGTTGGTGCGGCAGGTACCGGACAATCCGGTAAACAGAACTGCATTGGCGGCTTTAT
CCGCCCACACCGTACCATCTAAGGCTACGGGCAGGCATCAGGAAATATTGGACGGCGCGT
TGGATGACGCGCTGGTTGAGCCCGATTTCAATTCTCAGCTCAACCCGATGGTGCGTCAGG
CGGTTGCCGCACTTAATGCTTGCAACAGCTACGAGGAGGCAGATGCCGCACTGAATGCGC
TTTATCCGAATTTGGACAACGCGAAACTGCGTACCTATATGCAGCAGGCCTTGTTTATCA
GCGATATTTTGGGACAAGACCATGCCCGCGCCTGATTTGGGATTTGCCTTAAGTCTGCCG
CCAAAAAAGGCAATCGAGTGGCTGGAAAGTAAAAAGGTTACGGCGGAGAGCTACCGCAAT
CTGACAGCCTCCGAAATTGCCAAAGTCTATACGATTGCCCGCATGACCGACTTGGATATG
CTCAACGACATCAAAACTTCGATGGTTGAATCGGCAAAAAGTGGACAGTCGTTTGACGAT
TGGCGAAAAGGTATCTTGAATCTGCTCAGCAACAAGGGCTGGCTGCATCCGAACGGGCAT
AACGGTAAGGATATCATCGACCCAGCCACCGGCGAGGTATTCGGTTCGCCGCGGAGGTTG
GAGACGATTTACCGTACCAATATGCAAACTGCCTACAACGCCGGTCAATATCAAGGATAT
ATGGCAAATATTGATGCACGACCTTATTGGATGTATGACGCGGTAGGCGACAGCCGCACC
CGTCCGGCGCATTCGGCAATAGACGGGCTGGTGTACCGCTACGACGACCCGTTTTGGGCA
ACGTTTTACCCGCCCAACGGCTACAACTGCCGCTGCTCGGTCATCGCGCTGTCGGAGCGG
GATGTGGAACGCCAGGGGCGGATTGTTGGGCAAAGCACGGCGGACAATCTGGTCGAGACC
CATAAAATCTACAACAAAAAAGGCGATACTTATCTGACCCTTGCCTATAAAGCACCGGAT
GGCAGTCTGTACACGACCGATCGAGGATTTGATTACAACGCCGGACGAATGAACTACCGC
CCCGATTTAGACAAGTACGACCGTGCGTTGGCGCATCAATTTGCCAAAGCGGAAATGGGT
GGTGCGGATTTTAAAACCAGCTTTAAACAGCTTGAAAAAGAGTTTTATGAAGTCAAGCAA
CGTTTGGATATTGATGGCAAGCCCGATAAAGAGCAGAAAATCAAAATCCGAAATGCGCTA
TCAAGACAGCTTAAATTTGCTGCGGGTGTATTGAGCAAGGAAACGCAAGAATTGGCAGGT
ATGACACGAGCGACGGTGTGGCTGTCTGATGATACGTTGGTTAAACAGGTAGACAGCCGT
GAGGGGCAGAATTTCGATGACTCCTACTATGCTTTTTTGCCGGATATGCTGCAAAACCCT
GAACATGTCATCCGCGACAATCGTGAATTGATTTTCACAGCTCGCTATAAAGGCTCGGCA
TTGTGGCAGTTTTAAAATATATTAAGGAGGTGGATGAGATTTATCTACAGTCGTACCGA
ATCAGTAACGACAAAGAGATTGCCAAATTTATGGCGAAGAAGAAAGTATTGAAATAGACG
TTGGGCAAGGCTCGAAATCACTTGCACACGCTCTCGGACGCCCTAACGGGCAGGCTGCGG
TATCGAGGTTATCACCGCTTTTCCAACGTCTGTACGGAATAATACCATGATTGATGTCAA
AATAGACAATATCTTTGTCGTCCTAAACCAAATCGAGCGGCTTGGCAACGGGATCGAAAA
CCGCTACCTGCTGATGCGCCGACTGTCCGAAACCATGCACACGGCGGTCAAGCTCAATTT
CCGCTACGCAGGCCGTCCGAAATGGTTGGGCTAAAATACCGCGACGGCAAGCCGCTTTCG
GATTCGGGTCGTCTGAAAGACAGTTTTTCCACACTGTCAGACAACGATACAGCCCTTGTC
GGTACGAATATCGTCTATGCCGCCATCCACAACTTCGGCGGTATGGCGGGGCGCAACCGC
AAAGTTCGGATTCCGCAACGGGAATTTTTGACGCTGACGGACGACGACAAACAGGCTTTG
ATGGACGATGTGCAGGATTATTTTTCGGGTCTGATACCGTGAATTTATAAAACCCTCAAA
AACGCGCTTTTTAGCGCGTTTTTTTATGCGGGTAATACAAACCCCTGCCCAAGATATAAA
AATCAATCCTAGACGCTTCTAAAAAGCCCCTGAAAACGATTAATTGTGTATCGCGCGGAC
AGGTTTTAAAAAAATGGCGGGAGGGTTTGAAGCACGCCTACTCTTTGTTGTTTTTTCAAA
TAGGCAAAATGACCGTATTGAGAGAGGTACACATGTCCAAAAATGCACAAAAAACCCTAC
TTGCCGTGTGCAGTTTCGAGGTGCAGCCAAAAGACGGGCGAATCCAACTGCTGCCATATG
GCGAATTTCGCGCAGTAGACGGTCGTCCGACTGATGTCCCTGCGTGGTATCTGACCGAAG
AAAACGGTCATGATGTCGCGTTGTTGGCCAACAGCTCGCGCAATCAGTTGGTTGTCGATT
ATGAACACCAGACGCTCTACAAAGAGAAAAACGGACAACCTGCACCTGCCGCCGGTTGGA
TGCGTTGGCTGGAGTTCACGCCTAAAGGCATGTTTGCCGAAGTGGAGTGGACGGACAAGG
CGGCTGCGGCAATTGCCGCAAAAGAGTATCGCTACATCTCTGCTGTGTTTTCCTATGACA
CAAAGGGATATGTAAGCAAAATTTTTCACGCCGCGCTGACAAATTTCCCCGCGTTGGACG
GTATGGACGAGGTGCTGGCGGCAGCGTCGGCGCAAATTTTAAAACCGGAAACGGAGCAAA
ACCCTATGAAAGAGTTGTTACAGCAACTGTTCGACCTGCCTGATGCGGGCGAAGAAGAAC
TGAAGGCGGCATTGTCCGCGCTCGTGGAAGCCAAGCCGAAAGACGTGGCATTGTCTGCCG
-ACGTGTTCGCGCAGCTGGCGGAAAAAGACAGCCGCATCGCGGCATTGACGGCGCAAACCG
CCAAGCCTGATTTGACTAAATACGCGCCTATCTCAGTGGTTCAAGAGCTGCAAAGCAAAG

## Appendix A

TCGCCGCGCTGACTGCCAAGCAGGAAGCAGACAAAGGCAACGAATTGATTACCGCCGCGC
TGACTTCAGGCAAATTGCTGCCTGCTCAGAAGGAGTGGGCAAAAGGCGTATTGAAACAGC
CGGGCGGCTTGGCATTTTTGACCGGCTTTATTGAAAACGCCCAGCCGGTCGCTGCACTGG
CAGGCTCGCAAACGGGCGGCAAAGCACCCGACGAACGCGTCGCCGCACTGACTGCGGAAG
AGGCAGCCGCAGCAAAAATGCTGGGCATGTCCGGCGAAGAATTTGTAAAAATCAAAGAAA
GCGAAGGTAAGTAATGGACAAATCAGCGATTTTGACCGCAATCACGGCAGCATTCCGCAA
AGAATTTCAAACGGCCTTGGATTCGGATTTCAAGTGCAACACTAAGGTACCAGTGGTTGG
AACAGATTCAAGAATAAAACACTTGGCGTTTCGTAGCCAAGTGTTTTTCTTGGTCGGTGG
TTCAACTCATCTTGAACCCTGCGTATCTCCCGATCACTGATGTTACGGAAATCGGTTTGT
TTGGGGAAGTATTGCCGGATGAGTCCGTTGGTGTTCTCATTCAGCCCTTTCTCCCAAGAA
TGGTAAGGACGACAAAAATAAGTCTCCGCTTTCAATGCTTTGGTTATTTTGGTGTGTTGG
TAGAACTCTTTGCCGTTATCCATGGTAATGGTGTGCACCCTGTCTTTATGTGCCTTTAAT
GCCCTAACAGCTGCCCGGGCAGTGTCTTCGGCTTTGAGGCTATCCAATTTGCAGATGATG
GTGTAGCGGGTAACGCGTTCGACCAAGGTCAATAATGCGCTTTTCTGTCCTTTGCCGACA
ATGGTGTCGGCTTCCCAATCGCCGATACGGGATTTCTGGTCGACGATAGCGGGTCGGTTT
TCTATGCCGACACGGTTGGGTACTTTGCCTCTGGTCCATGTGCTGCCGTAGCGTTTGCGG
TAGGGTTTGCTGCATATTCTGAGATGTTGCCACAACGTGCTGCCGTTGCTTTTGTCTTGG
CGAAGGTAGCGGTAAATGGTGCTGTGGTGGAGCGTGATCTGGTGGTGTTTGCACAGGTAG
GCGCATACTTGTTCGGGACTGAGTTTGCGGCGGATAAGGGGGTCGATGTGCTGAATCAGC
TGCGAATCGAGCTTATAGGGTTGTCGCTTACGCTGTTTGATAGTCCGGCTTTGCCGCTGG
GCTTTTTCGGCGCTGTATTGCTGCCCTTGGGTGCGGTGCCGTCTGATTTCGCGGCTGATG
GTGCTTTTGTGGCGGTTCAGCTGTTTGGCGATTTCGGTGACGGTGCAGTGGCGGGACAGG
TATTGGATGTGGTATCGTTCGCCTTGGGTCAGTTGCGTGTAGCTCATGGCAATCTTTCTT
GCAGGAAAGGCCGTATGCTACCGCATACTGGCCTTTTTCTGTTAGGGAAAGTTGCACTTC
AAATGCGAATCCGCCGCCGTCTGAAACAAGGAGTCATCATGGCAAAAACCAACAACAAAC
CAGAAACCGCCGAAACCGCCGCCCCGTCGTTTGAAGACATCAAAGCCGAATTGGACGCCG
TGCAGGCAGAGCTTGCCGCCGCCCGAAACGATGTCGAAATGCTGACCACAGCCTTGGAAA
AAGCCGAAGACGACAAAAAGGCACTGTCCGCCGAACTTGCCGAACTCAAAGTGCAGCATA
CGCAACGTGCCGCCGACGCTTTGGCGGACAGCCGCGATGTGATGCTCGTCAGTACCGGCG
CAGACGGCAAAGAATTTTGGCGCGGCGGCCTGCTGTTTGACGGCGGCTGGCGCGAAGTGA
AGCGCGCCGAAGTCGGCGAAGCGGTGTGGAAGGCAATCTGCGCCGAGCCTATGCTGCAAC
GCAAGGCGGTCGAGTAATGGCATACGCGACGGTTGAGGATATGGTTGCGCGTTTCGGTGA
GCTGGAAGTCTTGCAGCTCACCGACCGCAACAACGAGGGGCTGATTGACCGCGAGGTCGC
ACAAACCGCGCTGGTGGACGCCACTGCCGAAATCGACGCGTATCTGGGGCGGTTCAGACG
ACCTTTTGAGGATCTGCCGCCCATCTTGGTGCGCCTTTGCTGCGACATTGCCCGCTACCG
TCTGACGGCGGCTCAGGGCGTGTTGATTACCGACGAAATCCGCAACCGCTACAAAATCGA
CGTGCTCGACCTGCTGCGTGCTATGGCCAAAGGCGAAGTGCAGCTGGGCGTGGATGATAG
CGGCGAAGAAGTGGCCGCGGGCGAAGACGGTATTGTGTTTGTAAACGGTAAAAATAAGGT
GTTCGGGCGTGATCACTGATATTGAGCAAGCGATAACAGACCGTCTGAAACGGGGCTTGG
GTCGCATGGTGCGCACGGTTAAAAGCTACAACGGCGAGGCCGACGATTTGGCGGGGCAAA
TCCATACGCTGCCTGCGGTTTGGGTAACGTATGGCGGCAGCAAAGTTGAGCCTGCCAGCA
CCGGCGGCGTATGCGGACGTTATCAGGATACCGCCGAATTTGTGGTGATGGTGGCGGCCC
GCAATCTGCGCAACGAGCAGGCGCAGCGGCAAGGCGGCATCGACAGCCGCGAAATCGGCA
GCAACGATTTAATCCGCGCTGTTCGCCGCCTGCTTGACGGCCAGCGGCTCGGTTTTGCCG
ATAGCCGCGGCTTGGTGCCCAAAGCGGTGCGCGCGATTGCCAATCATGTGCTGGTGCAAA
ACGCCGCAGTAAGCATATATGCGGTTGAGTATGCCATCCGCTTTAACACCTGCGGGTTGG
AAAATGACCGCTACCCCGAACGCACCGACAATCCCGACGACCCCAACCATATCTTTACCA
AGTATCAGGGTACATTGAGCGAGCCGTGGCCTGATTTCGAGGGGTTGGACGGCAAAATTT
ACGACCCGCAATCCGGGCATGAAATACCTGTAAACCTAACCCTTAAGGATAAGCAATGAG
CAAAATCAAAGTAACGGCGGCAGATGGCCTGCGTGTGCCGACCGAACACAACCCGCACGA
ATATATCGGCCAAGAGCCGGTGGAGGTGGACGGCAACAGCCTGTATTACCGCCGCATGAT
TGATGACGGCGATTTGGTGGTGGTTGAGGATGCCGCCCCAAATACCAAAACCCGCAATAC
TAAGGGAGAGTAATGATGCCCCATATTGATTTTGACACGATTCCGGGCAGCATCCGCGTG
CCCGGGCAGTATATTGAATTTAACACCCGCAATGCCGTACAAGGTTTGCCGCAAAATCCG
CAAAAGGTATTGATGGTTGCACCCATGCTGACCGCGGGCATACAGCCCGCCTTAGAGCCG
GTGCAACTATTTAGCGATGCCGAGGCGGCCGATTTGTTCGGACAAGGCTCGCTGGCGCAT
TTGATGGTGCGCCAAGCATTTGCCAACAACCCTTATTTGGATTTGACCGTTATCGGTATT
GCCGACCACAGCGCAGGCGTGCAGGCAACCGCAACCGTTACCCTTTCCGGCACGGCCACC
GCGCCGGGCGTGGTGGAAATCACGATTGGCGGCAAGCAGGTAAGCACGGCCGTTAACACC
GGCGAGACCGCCGCCACAGTGGCAGACCGTCTGAAAACCGCCATCACTGCCGCCGATGTA
ACCGTTACCGCATCCGGCAGCGGCGCAGCCGTTACGCTGACGGCCAAACACAAAGGCGAG
ATCGGCAACGAGAGCGGCTTAACCGTGAGCACCGGCAATACCGGCCTAACTTATCAAGCC
AATGCCTTTACCGGCGGTGCCAAAAATGCGGACATTGCCACGGCCTTGTCCAAAGTGGCG
GGCAAGCATTATCACATTATTTGCAGCCCGTTTAGCGATGACGCCAACGCCAAAGCCTTG
AGCAACCATATTACCAACGTATCCAACGCCATCGAGCAGCGCGGCTGTATCGGCGTATTG
GGTATGAGTGCGGCCTTGAGCACGGCCACCACCGCTACCGGCGAAATCAACGACGGCCGC
ATGACCTGTGCTTGGTACAAAGGTGCGGTAGAGCCAAACGGCATCATCGCCGCAGGTTAT
GCGGCGGTGTTGGCCTTTGAAGAAGACCCTGCCAAGCCGCTGAACACGCTGGAAATCAAA
GGGCTGGCCGTTACACCTGATGCGCAATGGCCGCTGTTTGCAGAATGCAACAATGCGCTG
TACAACGGCTTGACCCCGCTCACAGTGGTCAACAACCGCGTGCAGATTATGCGTGCCGTA
TCCACCTATACCAAGTCGGCCAACAACACCGACGACCCGGCACTACTCGACATTACCACC
ATCCGCACGCTGGATTATGTGCGCCGCAGCGTTAAAGAGCGCATTGCCCTGCGTTTTCCG
CGCGACAAATTGAGCGACCGCCTGCTGCCCAAGGTTAAGAGCGAGATTTTGGACGTGCTG
ATTAAGCTCGACCAAGCCGAAATCATCGAAAACGCCGAGGCCAACAAAGGCAAGCTGGTG
GTGGCGCGTGCGCAAAACGACCCCAACCGTGTTAATGCCATTATCCCCGCCGATGTGGTC

# Appendix A

AACGGCCTGCACGTCTTTGCCGGGCGCATTGATTTGATTTTGTAACCCTTTTCAGACGGC
GTTTAAAACAGGTTTAAAGGCCGTCTGAAACCTTAAAAAAGGATAAAGCATGAGCGACGC
TACCTATGCCGACGCGGTGATTATGGAGATGAACGGCCGCGATATCGAGATTGTGAGCAT
CAAGCCGCAAACCACTACAGGCCGCAAGCCGGTCAAAACGATGAACCGCAACGGCCGAGT
CAACGGTTATTGTGACGGCGTAACCGAACACAAATTAAGCGTTACCGCCGCCATTCCGAT
CGACGGTACGGAAATCGACTGGGACAACATCACCAAGGCGAAAATCACGATTTACCCCAT
CAACGACGAAGATCGCCGCACTTCCTACCTCGACTGCTTTACCGTCGATACCGGCGAGCA
ATATGAAGTCGATAACGAGGCACGCATCGACATTGAGATGATTGCTTTGCACAAAATCAA
GGAGTAATGCGTGATTACCGTCAAACTGACCCACGGGCTGACCTACAATGGCAAAGTCGT
ATCTGAATTACGCCTCAAGCCACTGACCGTCGGCGGCGAACTGGCGGCGTTCGCCCTGAT
TGATGACTTGCCCGAGCTGCCCGAAAACGCCACAAAAGCCGAACTGCTGCAACGCGACGT
CCTAGAGACGCTGACCTACTGGTCGCAGCAGATTGAAGCCCAAGGTATCCCATCCGACAT
CCTGACGGCGCAGTGGCTGATGGAAAACCTCTCTACCGAAGACTACCATACCGTGATGGC
GGCTCAGGAGGATTTGCGCCTAAAACCGTCCGCCGCTACGGCGAGCCCCGATGCGCCGTC
GGCGGCGGAGCAGTAAAACGCAGCTACCTGACGGCACACAAAAGCTACCGTCAGGCGGTC
ATCCTGATGGCAAGGCGGGGATAGGGGCGGACGCAGTGCGCGATATGTGCCACGCCGAGC
TGTCGGCATGGTTTGAAGACATCCTGTCGAGCCTGGGCATAAAAACGCCGAAGGAAGAGG
GAGTGATTGTGTCGAAACGGCTGAGAAAGCCGGAGGGAAAATAAAAAGGTCGTCTGAGTT
TCAGACGACCTTTTTTTTATTTGACGGTTAGGGTTGTTTTCTGCCGATTATTGCAATGGT
GTTTGTTTCTTTTTCAAAAACAACGCTATATAAAATACCATCTGCCGGAACGTCTTTTTG
CGCTGTTGCTCCTGTCTGATTGGATTCTTTGACGACTTCGGTTAAAGCTGTAAAAAGTTG
TTTTCCTGCTTCCGATTCGCCCAATTTGTCTGTGGTAGTTAAAGACAGAACTGCCTGAAT
ACTGTGTAATCCATTTATGGCATTATTGACTAGATTTTGTCCTTTCTCTCCGTCGGTTTT
TAAAATGTAGCCTACTGAGATAGCTCTAATTTTTTGCTGCTCGTCCAGTTTGAGCGCAAT
CGCCCCAAAATCCATTACCAAAGTAAGGTCATAACCACAATCGGTCTTGACAATATTTTT
ATTATGGATTTTTGTTGAGACGTTCTGAGTTTTAAATGCTTGCTCCAAATTGATGAGATA
TTGTTCAACTGTGATGTCCATTGGGGCGGTACAATCAGCAGCCTGGATACTTTGGACAGG
TTCTTCGGCTTGTGCCGTTTTTGATTGTTGGTTGCAGGCTGATAAAACAATTGAAACACA
AATTGCGGAAATCAATTTTTTCATATTCATAAAATATCCCTTTGAATAAAATGGTTATCA
TTCTAGTATTATAACGCAACAAACAAATAAAGCACGAAAACGGGGTTGAAGCCCATACCG
CCTCCCTTAAACAGCCTTTAAACGATAATTGACCTTGAGTTAATACGTTTAAAGGCTGCT
TTTTATGGCAAACGGGAACATGAAACTGTCGTTGGTGTTAACCGCCCGAGATGACGGAGC
GAGACGGCTACTGGCTGATACTCAACGACAATTAGATCGTACCGCGAAATCGCGGGCGCA
ACTTGAACGGCAAAGCCATACTTATGCGTTGACCGGCATCCGCTCAGAAAAACAGATTCA
ACGCGAAATCATGCTGACACAGGCTGCGTTTAACCGTTTGGCGCGCAGCGGCAAGGCATC
ACAAAATGATTTGGCACGGGCGGCGGTCGCTACGCGTAACCGAATTCGCGAGCTGAACGC
GGAACTGAAACAGGGCACGGGATTTGCGGACAAGATGGGAAAAATCGGAAGATTCGGTGC
AGCTGCGGTGGCTGGTGGCGCGGCAGCGTATACGGTGCTTAAGCCTGCTATGGACAACAG
AAAGCAGCTTGATGAGAACATCAACCGCGTGTCCAGACAGGCATTTATTGAGGATAACAG
TAAATCGGCAGCGTGGATTGCAACTGAAGGTGCGCAACAGATCAAGGATTTGGCACTTGA
ACTTGTCGAGAAAAATGGCGGGACCCACGATAAGGCTTTGGATTTAATCAGCGGCATGAT
GACCACCGGTCTGAATTTTGCCCAAACCAAGAATGAAGCGCAGGCGGCATATGCTTTTGC
ACTTGCCTCAGAAGGCAGTGGCGAGGATACGGCAAAACTGATTAAAACCCTGAAAGATGG
CGGCATGAGCGGTAAAGACCTGCAACTCGGGCTTGAGCACGTCTTGCAATCGGGTTTAGA
CGGCACTTTCGAGGTGCGGGATATGGTTCGGGAGCTGCCGAGCCTGCTCTCTGCCGCGCA
ACAGGCAGGGATGAATGGTGTCGGCGGTTTGGACTACCTGCTCTCACTCTTACAATCTGC
GGCGAATAAATCGGGCAGTCCTGCCGAAGCGGCGACTAATGTGCAAAATCTTTTGAGTAA
AACTCTGTCGCCTGACACGATAGGTCGTCTGAAGAAGATGGCAAATCCGAATGACCCGAA
GAAAGGTGTCGATTGGATAGGCTCGGTTGTGCAAGGCAAGCAAAACGGCGAAAACGCAGT
GCAGGTGTTGTCCCGTCTTGCCGATGCCATGCTAGTAAAGGATAAGCAATACCAAGATTA
TAAGAAACGCGCGGCTGCAGGCGATAAGACGGCGGCGGAGCAGGCAAATATGCTTAAGGG
CGCGCTTTTGGCGCAACTGCTGCCTGATTTGCAGGCAAAACAAGGTTTGCTGGCTGCAAC
GGATATGACGCAAATCCGTGAATATATGGCTTCGTTGGCTGGCGTAACGTTGGATAACGG
AAAAATTGCTAAGAACAACGAGGCGCGAATGTTGTCGGCAGCGGCGCAACAAGAGCAACA
GGAATCGCTGGCAATGTTGCGGGAAAGTCTGACGGGAACATTGGTGGATATGGAAACCTC
GTTTAAAAAGCTGGCAGCGGAATACCCTAATGCCACTCTAGCCCTGCAAGCATTGACGAC
GGCGGCAACAGCGGCGTCTGCCGCAATGTTATTAACCGCCGGTGGCGGTAAAGGTGCAGG
CTTTCTGAAAGATGTAGGTAGTAAAGCGTTGGGATGGGGTAAGGCTTCCGCAGGCGGCGT
GGCAGCAGGTGCCACAGCGGCAGGCGGTAAGTTGCTGTCATGGGGAAAATCTGCCGGTAG
CGGGCTCATGAATAATCCAGCGTTAGTTAAACGGGCGGGTTTGTTAGGTATGTTGCTGTA
TTCCGAGTCTTTGGGTGACGGCACATTGCCAAAGGGTTTGCGTGGTACCAAGACAACTCC
TGAAATGATTAATCGTCTGAAAAACAACGGTATCCGATTTGAACCTGCGCCGAAGCGGGA
ACAGGCGCGGGGTGGTGTCCCTCAGTATTTGGCTGCTCCGTCAGCGCAGCCTACCGATAA
GATGTTGTCTCCGTTGTTTTCAACTCAGACGGCGGCGTATCAGGCAGCCATTCAGCAGCA
GACGGCGGCGTATCAGGCAGCATTGGCGCAGGATACGGCTGCAGTTACAACAGGTTTGGC
ACAAGTGCAAAGTGCGATGGCGTCGGCAAGTCAGACCATCAATACCAATGTGAGCCTGAA
TATCGACGGACGTGTTATCGCGAATGAGGTATCGCGGTATCAAGTGGCCATGTTCGGCCG
TGGAGCGGGTCAATAATGAGCGGATGGCATACCTTATTGCAGGACGCATCTTACAAGGGC
GTCGGCTTTGATATTGAGGTGGTGGACGAGAGCAACGGCAAGGCATTGGCCGAGCATGCG
CGGCCGTTTGTGCAGGGTATCGACCTTGAAGACATGGGCATGACCGGGCGGCAGGTGCAG
ATTAATGCGGTGTTTTGGGGCAAGGGCTATGCAGGCCGTCTGAAAAAGCTGCTGGATGCG
CTGGAGCAGCCGGGCGGCGGCGTGCTGGTGCACCCTGTTTGGGGGCGGATGCACAACATG
ATTGCGGCATCATGGAGTTACCGACATGAGGCCGATTATGTGGATTATGCGGGCATCGAT
ATTACTTTCCGCGAGGCGGCCGAAGCGCAGGAAATCTTTGTTTTTGAAAACGCCTTTTTG
GTCGAGCTTGAGGCGTTGATTGCTAATATCGACACCTACCGCGAGGCGGCTATCGGCTTT

## Appendix A

GTTGATGCGGTGTTGGCGGTGGATGCGGGCGTATCAGCTTTATGGGGCAGCGCGCTGGGC
ATTTGGAGTGCGGCATCGGGTACGTTTGGCGCGGTGCGCCGTTTGTTTGATTTGGACAAA
ATTGCCTTTCCCGATCGGGGCGGATACAGTGCAGCGGCGTTTAAAAACGGCTCGGCCAAG
CTGTTTGCGGATATATCGGTCATGGTAGATACTGGCATACGCCGTGAGGCGGGTTTGGCC
GATAATGCCATGCACCATGCCGGTTGGTCGCCGCGACAGCGGTTTGACGGGGCTGCGGCT
GTTGCCGACCGCGCCGCCGCTATCCCTGATAATTTGCTGACCGGCCGCTTTTCAGACGGC
CTGCAAAACCGCCTGAACCGGTTAACCGCCAAACAGGTGCAGCCGGTAGCGCAGGCGGTG
CGCCTGTTATCCACGTCATCGCTGTTGTCGGTGGCAACGGCATTAATCGAGGCGCATGGC
GAAGAGATGACCGCGCCCGATTTGATTGAGGTTAACCGCGCCATGCGCCGCCGTATGCAG
GCCGAGATTGCCGCCTTGCGGGCGGTGCAGACGGCTGCTGCCGAGTCTGGTGGGCTGACG
GCCAACGCCGTGTATACCGAGGCTTACCAAACGGCAGAATCCCTGCGCGCGGCGGCAGGC
CGTCTGAATGCGTTGGTTGCGGCGGTCATCAACCAAAAGCCGCCGCTGATTGTGCGCCAA
GCCCCAATCGACGGTACGATACACCAAATCGCCCACGAGTTTTACGGCGATATAGCCCGC
GCAGCAGAGCTGGTGCGGCTCAATCCCCATATCCACCACCCCGCGTTTATCAAGCGCGGC
ACTTTGGTCAACAGCTATGCAAAATAATTCATACGGCTATGCCGTGTCGGTGCGCGTGGG
CGGTAAAGAGCACCGCCACTGGGAGCGCTACGACATCGACAGCGACTTTTTAATCCCTGC
CGACAGCTTCGATTTTGTCATCGGCAGGTTGGGACCGGAGGCGGCCATACCCGATTTAAG
CGGAGAGAGCTGCGAGGTAGTGATAGACGGGCAAATCGTGATGACGGGCATCATCGGCAG
CCAGCGCCACGGCAAAAGCAAGGGCAGCCGCGAGTTGAGCTTGAGCGGGCGTGATTTGGC
CGGTTTTTTGGTGGATTGCTCCGCGCCGCAGCTCAATGTAAAGGGCATGACGGTATTGGA
TGCAGCCAAAAAGCTGGCCGCGCCGTGGCCGCAGATTAAAGCGGTGGTGCTTAAGGCCGA
AAACAACCCCGCTTTGGGCAAAATCGACATCGAGCCGGGCGAAACCGTATGGCAGGCATT
AACCCATATTGCCAACTCGGTCGGGCTGCATCCGTGGCTGGAGCCGGACGGCACGTTGGT
GGTGGGCGGTGCGGATTACAGCAGCCCGCCGGTGGCGACATTGTGTTGGAGCCGCACCGA
CAGCCGCTGCAATATCGAGCGCATGGACATTGAGTGGGATACCGACAACCGCTTTTCCGA
GGTTACTTTTTTGGCGCAATCGCACGGCCGCAGCGGCGACAGCGCCAAACACGATTTAAA
GTGGGTGTACAAAGACCCGACGATGACGCTGCACCGCCCTAAAACGGTGGTGGTGTCCGA
TGCCGACAATTTGGCCGCATTGCAAAAGCAGGCTAAAAAGCAGCTGGCCGACTGGCGGCT
GGAGGGATTTACACTCACGATAACCGTGGGCGGCCATAAAACCCGCGACGGCGTATTGTG
GCAACCTGGCCTGCGTGTGCATGTGATCGACGACGAGCACGGTATCGATGCGGTGTTTTT
TCTGATGGGGCGGCGGTTTATGCTATCCCGCATGGATGGTACGCAAACCGAGCTGCGGCT
CAAAGAGGACGGTATTTGGACACCCGACGCTTACCCCAAAAAGGCCGAGGCGGCGCGCAA
GCGCAAAGGCAAACGCAAAGGCGTGAGCCATAAGGGCAAAAAAGGCGGCAAAAAACAAGC
AGAAACGGCGGTGTTTGAATGAGTTTGAGTAAATTGGCGAAAAAAACGGCACAAACTGCT
AAAAATATCGGCGAAACCCTGCGCGCGGCCTTTCGGGGAAAAATCACGCTGGTGGTGTCG
TCCGAGCCGATACAGCGCGTGCAGTTGAGCGGCTTGGCCGACGAAACCCTGCAAGACCTT
GAACATTTGCAGGAATACGGCTTTGCCAGCCATCCGCCCGACGGCAGCGAAGCGGTAGTG
ATACCGCTGGGCGGCAATACTTCGCACGGTGTGATTGTGTGCAGCCAGCACGGCAGCTAC
CGCATCAAAAACCTTAAGCCCGGCGAGACGGCGATTTTTAATCATGAGGGTGCAAAAATC
GTGATTAAGCAAGGCAAAATCATTGAGGCCGATTGCGACGTGTACCGGGTTAACTGCAAA
CAATACGAGGTTAATGCGGCCACGGATGCCAAATTTAACGCTCCGTTGGTGGAGACCAGT
GCAGTGTTGACGGCGCAAGGCCAAATCAACGGCAACGGCGGCATGGCCGTCGAGGGCGGC
GACGGAGCCACCTTTAGCGGCGATGTTAACCAAACGGGCGGCAGCTTTAACACCGACGGC
GACGTGGTGGCCGGCAATATATCGTTGCGCCAGCACCCGCATACCGACAGCATCGGCGGC
AAAACCTTACCGGCGGAACCGGCATAGACAAGCAGACCTTTGGCAGCCTTCGGGCTGCTT
TTTTTGTGCGTGTGGGATTGAAGCCCGTGTACTCCGTGAGGCCGTCTGAAAACGGCAAAA
TGCCAACATGGACAAAGAGCTAAACCCCAGCATCGGCGACTATACCGGCCGCACCGTCGA
TACGCTGCAAAATGCCGTGTATATCCGCTTGATGACACCGTTGGGCAGCTGGTGGGCGGA
TAAAACGCTCGGCTCGCTGCTGCATTTGTTGCAGCGCGAAAAAGACCTGCAACGGGTCAG
CCTGTTGGCCGAGCAATATGCCGATGAGGCACTGCAACCGATTGTTAAGAGCGGGCGTGC
CGACAAGATTACCGTGCGCGCAGAGCAGCCGCACGACGGCCGCCTGATCCTGCATATCCG
GATGGATACGGCGGCGGGCGGTTTGATTACCGCCACGAAGTGCCCGTGATTTAAAGAGG
TTTTAAACGTGTTTGAAACGCCGACATTTGAGCAAATCCGCGAGCGTATCCTGCGCGATA
CCAAAAGCCTGTGGCCGGATGCCGATATCAGCCCCGACAGCGACCATTATGTGCACGCCA
GCCGTTTGGCCAGCTGCGCCGAAGGGCAATATGCGCATCAAAGCTGGATTGTGCGGCAGA
TTTTCCCTGATACCGCCGACCGCGAGTATTTGGAGCGGCATGCCTCCATGCGCGGCTTGA
GCCGCCGCAATCCTACCACGGCCAGCGGCACGCTGACCGTAAGCGGTATTGCGCAATCCA
TGCTTTCAGACGACCTGCAAGTGCGTATCGGCCAGCGTTTTTACCGCACTACCGCCCGCG
CCGTTATCGGCAGCGGCGGCACGGCGGAAATACCGGCAATCGCCGACGAGCCGGGCGCGG
CCGCCAATGTGGCGACGGCGAGGCGCAACTGATGGCCGCCCCCGCCGGTGTGGCCACCGA
ATGCCGCCTTACCGTACAAGGCGGCACCGACCGAGAAAGCGATGCCTCACTGCTGGCGCG
TCTGTTGGAAATCATCCGCCGACCGCCCGCAGGCGGCAACCGTTACGACTATAAAAACTG
GGCGTTGAGTGTTGACGGCGTAACCAGCGCATATGTTTATCCGCTGCGCCGCGGCTTGGG
TACGGTGGATATTGCCATTACCTCCGCCGACGGTGTGTCGTCGGAAGAAACTGTGCGCCG
CGTACAGGCTTATATCGACGAGATGCGCCCGGTAACGGCAAAAAATGCGCTGGTACTCAA
GCCAACCGTAACGGCGGTGCCTGTTACCGTGCAAGTCAAGCTCGACGGTATCGACTTGGA
CGAGGCCAAGCGCCGCATACGGACGGCCCTAAAAGAATATTTCGACACCCTGATCCCCGG
CGACGGCCTGACTGTGTCGCAAATCGAGGCTGCTATCAGCAATGTGGATGGTGTGATCGA
CCGCCGTCTGACTGCGCCGACGGCCAACCGTGCCGCCGATACGGTTAACCGCATCGAGTG
GTTTAAAGCGGGCGCGATTAATGTAACGGAGATGCCGTCATGAGCTATCAAGACATCTTG
CGGGGCCTGTTGCCCCCCGTGTCGTATGCCCGCAATGCCCCGCGTGTGCGGGCGCAGGCA
GAAATAGACGGCGCAGCGCTGGATGCGGTGGCGGAATCGGCTCAAAGCGTTGCCGATGCC
GTCGACCCGCGCAGCGCCGGCCAAATGCTGGCCGATTGGGAGCGCGTATTAGGTTTGGAC
GGTACGGGCAAAAACCGCCAGCACCGTGTGTTGGCCGTCATGGCCAAGCTAAACGAAACA
GGCGGCTTGAGTATTCCTTATTTTGTGCGTTTGGCCGAGGCGGCGGGCTATCAAATCCAA

## Appendix A

ATCGACGAACCGCAGCCGTTCCGCGCCGGTGTAAACCGCGCCGGCGACCGTCTTGCGCCG
CAGGAAATCATGTGGGTGTGGCACGTTAACGTGCGCGGCGGCAACAACCGCATTACCCGA
TTCCGCGCCGGTATCTCGGCGGCGGGCGACAGGCTGACCGATTACAGCGATGCCGTGATC
GAGAGCCTGTTCAACCGCCTCAAGCCCGCCCACACCGCTATCCGATTTACCTACCGCTAA
AGGACGATTTATGCACCCCATCGAAACCCCCGATAAGACCTTCCACGACGGCGACGGCGT
GTCCGAATTGGGCACCATCCTGCCCGCGTGGTGGCTCAACCAAGTGCAATCCGAGCTGCT
GGCCGTGCTGACTGCGGCCGGTATCCAGCCGGATAAGTCGCAGCCTAATCAATTACTGGC
AGCACTAAATAGGCTGGCTGTAGTTATCACCGGCGACCAAACCGTCAACGGCCAAAAAAC
CTTTACCGCCCAAACCCAATTCCAAAGCGGCATCCATTTATCCGCCAACCAGACGAACTG
GAACGGCGGCCACAAAGCCTACATCGGCGCGGATGCCGACAACGCCCACATCGTCTTCGG
CGACGACACCCTCCGCCTGCACGGCGCAAACAACCGCATTTCCTACAACAACCACGACAT
CTTCCACAAAGCCAACAAACCGCGTTTTGCCGAAGACATCCAAGGCAAACCGAACACACT
GTCCGGATACGGCATCGGCAATTTCAAAGTCGAAACATTCCGGGGCGATTTGAACACCCT
CAAAACAGACGGCATCTATTCCCTGCCGACGGCGGTCGGCAGCTCCAACCTGCCCGTTGA
AAACACCGCCTGCCATATCCAAGTCATCGCCGGCACGAAACACGGCTGGTGCAGGCAGTT
GGGTTATCCCGCCTACACGTCCGACGTGTACGAACGCCACCAAACGAGCAGCGCAAACGA
CAACTGGTCCGCATGGAAAAAACTCAATTCGGACGGCATCCCCGTCGGCGCGATCGTATC
CTTTCCCAAAGCCGTACAAAACCCCGCAGGCTATCTCAAAGCCAACGGCACGACCTTTGC
ACAAAACACCTTCCCCGACCTTTACCGCGCCTTGGGCAACAGCAACCGCCTGCCCGATTT
AAGCCGTACCGACATCGGCATCACCGCGTGGTTTCCGTCCGACCAAATCCCGACCGGCTG
GCTGGCGTTTGACGACATCCGCACGCGCGTAACCGAAACCGCTTATCCCGAGCTGTACCG
TCTGCTGACCGGAAAATACGGCAGCATCCAAAACGTCCCGCAGGCGGAAGACCGCTTTAT
CCGCAACGCGGGCAACAGCTTGGCAGTCGGAACGAAGCAGGAAGACGAAATCAAACGGCA
CGTCCACAAAGTATTTTCACACTGGACAAACCACACAGACGCGGCAGCCCTCGGTTACGA
AGACCGCAACGAAAGGCAGAGAAGCGCGCTCGTATCGACTTGGACGGACGAAAATTTAAA
CGACAACGGCTTTTTAACCCCGCGCTCGGACAGCAAAATGGCGACAGGCGGCGACGAAAA
CCGCCCCAAAGCCCTGGTTTTAAAACTGTGCATCAAAGCCGCCGACACCTTGGGCGAAGC
CGTGTTTTGGATAAAGTCCCACGGCGAAACCATCAACGCAGGCGCGCTGGACGCGGGCAC
GCTGGCGCAAGGTTTGCAAGACAAAGCCGACCGAGACCACACCCACACCGCCGCCCAAAT
CCAAGGGCTGGACGAAAAAATCAGCACCGCCGTTGCCGCGCAATTCACACGCCAAACCAT
CGGCGGCGTGGATATTGTCAGATTCCCCGACGGCACAATGATACAGACCGGCAGCTACAG
GTTCACACGAAGCGGCGGCCCCATCGAAAACGAAGTCGTCTTCCCCGTCGCCTTTGCCGA
CGGCAACGTCAAATGCTTCGTATCCGAACGCCATTCGGAACGCGTTACCGGCGATCGAAG
GCAACACAACTGGCTGTTTATCCGCGCAAAAAACCACGCCGCCGCCATTATCACCAACTG
GTACGAAGGCAGTTGCGACTGGATGGCCATCGGCAAAGCCGCCTCGGGAAACGCCGCCAG
CTCCCCGATAGGCCCCGAAATACCTGAAACCAACGAAGAACCGCAAAGAGAGAGTGGAAG
AACATCAACCGGACCCCGAAACCGCCGCCGCCGAGACGGCTTGCTCGAGGCACTGCAAGA
CTAGCGGGCTGTAGAGATGGCTGTAGAGACGGGCTGTAGAGATGGCTGTAGAGACGGGTT
GTAGAGACGGGTTGTAGAGACGGGTTGTAGAGATGGGTTGTAGAGATGGCTGTAGAGATG
GCTGTAGAGATGGCTGTAGAGATGGCTGTAGAGATGGGCTGTAGAGATGGGTTGTAGAGA
TGGGCTGTAGAGATGGGCTGTAGAGATGGGCTGTAGAGATGGGCTGTAGAGATGGGCTGT
AGAGATGGGCTGTAGAGATGGGCTGTAGAGATGGGCTGTAGAGATGGGCTGTAGAGATGG
CTGTAGAGATGGCTGTAGAGATGGCTGTAGAGATGGGTTGTAGAGATGGGTTGTAGAGAC
GAGTTGTAGAGATGGGCTTCAGCCCGCCGATCCAAGCAATCCGACCGAAACCGGCCGCGC
CGCCAATCCCCCGAAACCTATGCCCCGCCAATCCTGCCACTCTTCGTCATTCCCGCCGCT
TTCGTCATTCCCGCGAAAGCGGGAATCCAGACCCCCCGACGCAACAGGAATCTATCGGAA
AAACCGAAACCCCCGCCACCGTCACTCCCGCGAAAGCGGGAATCCAGCCCCCAAACGCGG
CAGGAATCTATCAGAAAAAACAGAAACCCCCGCCGCCGTCATTCCCGCGCAGGCGGGAAT
CCAGACCCCAAACGCGGCAGGAATCTATCGGAAAAAACAGAAATCCCCGCCGCCGTCATT
CCCGCGCAGGCGGGAATCCAGACCCCAAACGCGGCAGGAATCTATCGGAAAAAACCGACC
CCCCGCCACCGTCATTCCCGCGCAGGCGGGAATCCAGACCCCAAACGCGGCAGGAATCTA
TCGGAAACGGCTGAAACCGAACGGACTGGATTCCCGCCTGCGCGGGAATGACGGCGGCAG
GGGTTTCGGGATTCCCGCCTTCGCGGGAATGACGGAAAGTGGCGGGAATAACGAAAGGCG
GGAATGACCGCGCAAAAAGCCGCTGCCCCCTTCGGACGGCACCGGCAACAAAAAACCGCA
CGGCCGAAACCGCGCGGGAAAGGATAGTCGGGCGCGCCCGATAAGCAGCGGCCGCCCCCG
TTATTTCAATTGGGCGATATATTGGCGCAAAACCTCGTTGATGCGCGTCTGCCAGCCCTT
GCCGCCGGCGCGGAATTTTTCGACCACATCGGCGGACAGGCGTATGGTAACGAGTTGTTT
CGGGGTTTTGCCTGTGTTTCGTTTTTGCATTACGCCCTTTTCTTCCAATTGTTTTTGATG
GGAAAAAAGCACCTGTGCCAAGTCTTCGGGCAGGGCTTCGGCAATGGGGCGGGCAAGCGC
AAAATCTTCGGCGGCAAGTTCCCGCACTTCGCCGTCAGCGTTTGTTAAGGATTGACGTTG
CATATTTTTTAACCTCTCTTTTATTCGCTTTGCGAAAACTGATGACACGGATGCCGTCTT
TTATCGGCGTAAAACAGACAATGTGCAGGCGTTGCGTATCGCCTAGATAAGCAGCGGCAA
CATAACGCGGTTCGGGGTAATCAAAGCGGACATCGGGCACAATAACGGCCGTTGTCCAGC
GTATTTGCCCGACTGATTCAAAGGGCAAATTCCGCTCTTCGATATTGCGTTGATTTTTTT
CGGAGTCAAATTCAATCTTCATTGCAGCTTGCAGCGTATTTTGTCGTTACATTATAAGCG
GCAAAAAACCAAAATGTAAATACAAAAAAGGAAACCCCAAAATGACCATCTATTTCAAAA
ACGGCTTTTACGACGACACATTGGGCGGCATCCCCGAAGGCGCGGTTGCCGTCCGCGCCG
AAGAATACGCCGCCCTTTTGGCAGGACAGGCGCAGGGCGGGCAGATTGCCGCAGATTCCG
ACGGCCGCCCCGTTTTAACCCCGCCGCGCCCGTCCGATTACCACGAATGGGACGGCAAAA
AATGGAAAATCAGCAAAGCCGCCGCCGCCGCCCGTTTCGCCAAACAAAAAACCGCCTTGG
CATTCCGCCTCGCGGAAAAGGCGGACGAACTCAAAAACAGCCTCTTGGCGGGCTATCCCC
AAGTGGAAATCGACAGCTTTTACAGGCAGGAAAAAGAAGCCCTCGCGCGGCAGGCGGACA
ACAACGCCCCGACCCCGATGCTGGCGCAAATCGCCGCCGCAAGGGGCGTGGAATTGGACG
TTTGATTGAAAAAGTTATCGAAAAATCCGCCCGCCTGGCTGTTGCCGCCGGCGCGATTA
TCGGAAAGCGTCAGCAGCTCGAAGACAAATTGAACACCATCGAAACCGCGCCCGGATTGG

## Appendix A

ACGCGCTGGAAAAGGAAATCGAAGAATGGACGCTAAACATCGGCTGAAAAAATACGTTTA
CCACCTGTTGGTAGCCATCGACCAACTGTTCAACGCCCTAACCGGCGGCGCGGCGGACGA
AACCCTCTCAAGCCGCACCTATCGCCGCGCGCGGCTCGCCCAAAAGCCCAAAACCCGCTG
GAAGATTTTATATACCCTGATCAACGGCGTGTTTTTCGACCGCCACCACTGCCGGCAGGC
GTATATCAGCGAACTGAAAGGCAGGCAGCACGACGCGCGGTTCAACCAAAGCCGCGCCGC
CGGGGAAAAGGGGACGCGATGAACTACTTCGGCATGGTAGAGTTTCTGCGCCTGATGGCA
ATGGTGCGGCCGCCATTTGTCTTCTTCTCCGGCACCCGCAGCGAACTGCCTGCTTATCTT
GACCTCGTGGCAGAACTGCGCCTGACCGGATGGGAGCGTTTTGCAGGCAGCCAAACCTTG
ACTGTGAGCAGCACATCAACAGCAATTCAAGCTACGACGACCACCTGATTTATAAGTTCT
GACCGCAAGTAGCGTACTACTTTTAAAGGCATAAGATAATCCCCGTTTAACAGACCATTA
AACGGGGATAAATTTGTGCAAAAGCTAATACAATTTCCTACGCTTCGGCGGTGCAAAAGC
TGCCGCCAATTCGTGCAAAAGCTGCCGCCGCCTTACATTCCAGTGCAATGCCGTCTGAAA
CTTCGCTAATCTCGGGTTGCCGCGCGCTGTGTTGTTCTTCGGTACTCAGCAAAAAGCCGT
ACAGACGCTCCAATCGGGCGCGGTAGGCGGTGAATTTGTTGTAGAACATCCGGAAGAAAG
AAAGCGCGTTTTGCAGTCGCGCGAAGGCTTGGACGGTCTGCTGGATGTCGCCGATTTTGA
TTTGTCCGGCAAACAGGCGCGGCGCTTGCAAAATAATGGGGAAGAGCTTGATGCCGTTGG
TGAACATATCATTAAAGCCGCTCAGGCAGACGCTTTGTCGCGCAATACGCCAGCGGTTGC
GGATAATGGCTTTAAAACGGTCAGAAAGCTGGTCGTGTTCGTGTTTTTCGCCGCTGTAAA
ACGCCACGCTTTCGGCGTGGTCGCGCACGCGGATGAGGGAATAACGGTAGTCGCCGTTGA
GTTTTTCGTTTTCATAATTGTAACGAATCAAAGGGTTGCCTATCCACATGGCGATAAAGG
TCGCCAAAATCACAAATATATAGACAAACCAAACGATGCCGTGCGGAATGTCGAAGCCGA
ACACGGTCAGGATGCCAGCCAAGCCCCGCAAAACAACGGCAAATTCCAGAGAAGTAACGA
CCGAATTGACCATGCCGCGCACAAATTCGATGGTCGAAGCGATAAATTCCTGCGCGTCCT
GTTGGATACGCTGGTCGATGTTGTCCGGCGCGTGGCGGCGCATTTGCAGGCGGTAGTAGT
TTTTGTCGGCAAGCCAGCGTGCTGTCAGCACTTCGTTGAGCCGCTCCGACCATTTAATCG
CCAAGCCTTGATCGAGGAAGTCGTTGACGACGTTGTTAAACGCCCGTATCAGCACCACGC
CCGCGTTCATTGCAGCAAACATCCAAAATGCCGAAGCATTTCAAATCCTGCATCGAGTCG
TAAAGCCCTTTGGACATAAAGGTACTCAACACATTCAGCCGCATTTCGGTTAACACCAGC
GTAATCATCGCCGTAATCAGCAGCAAGACTTTGACCGCGCTTTTCGGTGTCAGACAAAGC
CAAAGCGGTGTGGAATAAAGCTCGGTTTGCCATTTCTGCATGGGAAATTTCTTACGGTAT
CAATGCCGTCTGAAAAAGACGGGTACAGTTGATTTTTTGATGAAGTTTGGGGAAGTTTTG
CCGGTCAGGGTACATTGCGTGTTAATTTATAGTGGATTAAATTTAAACCAGTACAGCGTT
GCTTCGCCTTAGCTCAAAGAGAACGATTCTCTAAGGTGCTGAAGCACCAAGTGAATCGGT
TCCGTACTATTTGTACTGTCTGCGGCTTCGTCGCCTTGTCCTGATTTTTGTTAATCCACT
ATACCATACAACCACGCCGGAATTAAGTTTAAATTTGAATAAAAGGTTCGGGTTCTGCAA
AATACAGAACCCGAACCTTGTTCGGATATTGAAACCGGCTGCCCGATTTTGGGCGGTGCG
GCTTGCAAGTATCAAGATTCGCATATGCCGTCTGAAGCTCGGAGAGGTTCAGACGGCATA
TGCTTATTTGGGCTGCTCTTCAACGAATCTCGGACCTTTCAAGATGCCGTTGTGAGAATA
GGGCGACAGCAGGTTGTATGCGGCGGTTTTGGAAACCTGATAACCGCGGTCGGTCAGGCT
GTTGGCAATCTGATTGACCACTGCGCTGACCAAAGCCCCCAACAGGCCGCTGTTGCTGTT
GTTGCTGCCTTCGCGGATGCTGGCCGAACCCGACCACAACTCTTTTCCGTTGCGGGAATC
GACCAGCCGTGCTTTGGCGGATACGGTCGTCACGCTGTCTAAAATTTGATATGAAGTGCC
GTATTCGGTAACCGTAATGTACAAAACCGCATCATTGCCGAAAATCTGATGCAGTTTTTC
CGGCCGGACGGCGTGAATATCGGCGGCATTGGTCAAGCCGTTTTGTTTGAAGGTTTCCTC
CACGACTGCGGCGGGGAAGACGTAATAGCCGGCTTCGGAAAGCGGCGCGGCGGTCGAAGC
CAGTACACCCCATGTTCCGTTGACATCGGGCGATTCGTTCAGCGGCGGAACCACCAAAAT
TGAAGCCGGTTTGCTTTCCTTGAATGACGTGTAGTCGAAATCGGGCGCTTTTTGAACTTG
GCAGGCAGACAGCGCCAACACGGCGGCAAGCCCTAAAATCAAAGGTTTCATCGCTTGCCT
CCTTTACCGGTTTTCATCAGGAAGTCCATAAATACGCCCGATTCGGGAAACAGCCTTTTC
TCTTCTTCAAACTGGCGGAAGCGGGGCTCTTTGTCTCCCGAACGGGAAAGCAGCAGTCCC
AGATGGGCGTGCGCACCCGGGGCGGCATTCATTTTTTTGTTGCCGGCTTCCACAAAGTAT
TTTTCCATCTTTTCGGTCTGCTTGCCCAACGAAGTGTCGTCGTTTTTCAAACCTTCATAG
ACGGTATCGGGATAGCCGCCGTAATAATACAGGGATTTTTGCCCGTTGCCGCCGCAGGCG
GTCAGAGCCAAGACCGCCGCACACAGCGACAAACGGCTCAAGGTTTTCGGATTCATCATT
TCTCCTTAACGGTTGGGTTGCCATGCGCCGTTGTCAACAGCCTGAACCAGGCTGTTGACG
GCTTCGCGGATTGCCAAGTCTAAAACTTTGCCGTTCAAAGTCGCATCGTAGCCGGAAGTG
CCGCCGAAACCGATGATTTCACGGTTGGAAAGTGCGTATTCGCCCGCGCCCTGTGCGGAA
TAGACGATTTCGGAAGTATTGACGTTGACGATATTCAGAGCCACTTTTGCATAGGCGATT
TGCGATTTGCCGCGACCCAAAATGCCGAAGAGCTGATGATCGCCGACATCTCTGCGTCCG
AATTCGGTTACATCGCCGGTAACGACATAATCTGCGCCTTTCAGGTTATGCGCTTTGCCG
GAAATGCCGGATTCCTGTTTTAATGCGTTCAAATTGGTGCGGTTCAGTACGTTGAAGCGG
TTGGTCTGTTGCAGGTGCGTTACTAGAATGGTTTTTGCCTGGCTGCCCAAACGGTCTTCC
CCGTCGGAGAAAATGCCTTTTTGGAAGCTGGAGCGGTTGTCGAATGTTCCGACGGAAATC
GGGGTACGAACACCGTGATATTGCGTATTGTAGGAGGCGACTTTCTCTACCTCGAGACTG
CGTGAGGATTCGGTCGCACAGCCGGTCAGTGAAACGGCAGCGGCGGCAAGGACAACGGCG
GTGGAAACGGTTTTCATAAAATTTACCCTAAGGTCAAGTTAAGGAAATAACGGGTTGTCA
TTATTGTCCTTATGTAAATTTAAGTCAAGGTGTTTGTCTGTGCGGGACGGATGCGCGCGG
AAGGTACGGATATTTTTCAAACGGAATCGCGGCGCGGGCGGGATGCCTGCCTTTTCCGGA
TTTAGAGGTAAACGATTTCGCCACTCCGCCCTTTGCTTTCGGCACTTGCCCACCAGACAA
ATGCGGGCAGCACGTCCCCGTAGCTTTTGCGTTCGCTTTTGGCTTCGCCCGGATGGGATT
TGATGCGTTGCGGGGAATTGATGGGGCCGGGGACGAGGACGTTGGCGCGCAGGTTGCCGA
AGCGTTCCCATTCGTCGGCGGCGACTTTGCACAGGTAGTTCAACGCGGCTTTGGACGCGC
CGAAGCCGCCCCAGTAGGCTTTGGGTGTTTCGCCGTGGCTTTCGCCGACGAAGATGACGG
ACGCGTCGGGCGACTGCTTCAGCAGCGGGAACAGGGCGCGGGTCAGCCCCATAGGTGCGA
CGGTGTTGATGCGGTATTGGTTGACCCATTCGGCGACGGTTTGGAAATCCAGCGGCGAGA

# Appendix A

GGGCGTAAAAATAGCCGGCGCAGTGGACGATGCCGTCCAGTTTGCCTTGCGTGGCTTCGG
CAATGGTGGCGGCGAAATGTTCAAATTCTTTTTCTTCCGCGCTAATAAGGTCAAAGCAGA
TGGCGAATGGTTCGGGGTATCCGGCTTCGACAATCGCGTCATACACTTTTTCCAGTTTTT
TCTGATGACGGGCAACCAAAATCACGGTTGCGCCTGCCGCCGCATAGGCTTTGGCGACCT
GTTCGCCCAGACCTTGCGATGCGCCGGTTACTAAGATGGTTTTGTCGGACAGTGTCGCCA
TACTTTTTCCTTTTTGGTTGTCGGTTAAGGTATTTTAGCGTTTTGCCGCACCTTGTAAAG
CGTCCCGATGTCGGCCGGCGTTTTCAGACGGCATCGCTCAGGCTTTGCAGGCAGGCTTCC
GCCGACGCGAAACCTGATTGTACGGCGGCTTCGAGCGTGGCGGGGTAGTCCGGGTGGAGG
TAGTCGCCGGCGGGGAAGATGCGGTGCCGGTGCAACCACGACAAGTCCGGCGGCGGGGCA
TCGGCTGCGGTTGTGGCGCGTTTTTCGGTGATGACGCGCACGGCTTCGGGTTCGCCCAAA
TGCGGAAGGATGCGTTTGAGGTCGGCGTGGGCTTTGTCCGCCCACGCCCGGTTTGCAAAC
GCGCCGACGCGGTCGGAAACGCTGATGACGGCGGACACTTCGTTTTCAGGCAGTCCGAGC
CTGCCCCGGCAAAGCAGCCATTGCACCGTGCCGTCGGCAAGGCCGGTCAGCGGGGCGGGC
AGGCGGACGGGTTCGGCGTAGCGCAGATAGACGGTGGTGATGGCGTGGTAGCGAAGGTTT
TGATATGCCGTCTGAACGTGTTCGGGCGTGCCTTCGGGCAGGAGCGCGGCGGCGTGGTAG
GGCGCGGTGGCGGGGACGGCGGCATCGAAAGCTTCGCCGTTGACGAGCACTTTCCCGTCC
GGGAGGGTGTTCAGACGGCATACGCGCGTTTCGAGGCGGATGTCCGCGCCGAGCCGTTGA
AGATCCGCCAAGGCGGGTTCGGCGACGATTGCGCCCAAATCCTGCTTGGGTAGGAGATAG
TCGCTGCCGGATTTTTTCGTCAGCACGCCGTCGGACAAAACGTTGCACAACACGCGCAGG
CTTGCGGTTTCCAAAGGCGTGTTGAGCGCGCCCCAAACCAAGGGCTGCCAAAACTGCATC
ACGGCGGCACGCGGCACGTTCCGCTGTTTCAGCCATTGCGCCACTGTCGTGTCGGGCTGT
CCGAGGCGTGCGGACTTCTGCAAATCGGACATATCGGCAAGCAGTTTGGCTTTGAATGCA
GTCGGTGCACGCCGGGCAAGCAGCACGCCGCCCAAAATATGCAGCGGCGCGGGCAGGGGG
AGGGCGCGGAACTGCAAACCGCCGTGCATATGCCAGTGCAGCGGTACGCGCAAAAAGGCG
GCACGGGGATCCGAACCGATGGTTTTCATCAGGCGCAACACGCCCCGGTATGCGCCGAGC
AAAATGTGCTGCCCGTTGTCCAAAAAACCGAAACCGTCGGTATTTCCGGCCAGTGTGCGC
GCCCTGCCGCCCGCCTGCCGGCCGGCTTCAAACAGGGTAACGTCGGCGTGCCGCGCCAAG
GTGACGGCGGCGGACAGTCCTGCCCAGCCTGCGCCGATGACGGCGATTTTCGGGCGCGGA
TGCGGCGTGTTCATCATTTATTCCTCCAATGGTTTTGCAGCCGTATCTATTTCCGTTTCC
GAAAATGACGGTAGAAAAGGATACAGGCTAAAAATAAAGGCAGCAGAAAGCGCGCATAGG
GATACCACGGATGGTCTGCATGCCAGTATCCGTACCGTCCCTGTGGAACGGTATCATAGC
GGTAACTGTCGGGGAAAAAGTTGATCCAAACCGTTACTGCCAGCCATAAGGCTATATCCC
TGACTGCTTTCATTTACTTCTGCTCCTGTTTAAATTCCCAGCAATTCCATTTCAAAGCGC
GAACGCCAACGGGATTGCGCGGTTACGATGCAGACTTTCAGACGATGGTTGAAACCCCGT
CCGGCGGGGCTGTGGGAATCGGGTTTGCCTGACGGGCGGCGGGCGGTATGCGCCTTATGC
CCGTTCCGGCGTGCCGGGGCGCGGTTTGAATCCGAATAACCAGGTTTTCAGGGCAATGCG
TTTTTTGCGCGGCGAAGGGAGGGCGATTTTGTATTTGAGGACGTTTTGTGCGCCGTCTCG
GTCGATTTCGTTCAATAGCTCGTAATAAACCGCCGCCATGACCAGTCCGACTTTTTGGGC
TTTTTTATCGGCATCAGGCAGCAGCGATACGGCTTCGCGGTAGGTTTCGCGGGCGCGTTT
GATTTGGAACGCCATCAATTCGGCAAAATTGCCCGTCGGGCTGCATTGCAAAATCACGCT
TGCGGGTACGTCAAACCGCCGCATTTCCTCCATCGGCAGGTAAATCCGCCCCCTGCGCGC
GTCTTCGCCGACATCGCGGATGATGTTGGTCAGTTGCAGCGCAAGTCCCATCTTGTCGGC
GTATTCCAGCGTTTGGTCGTCTGAAAACCCCAAAATCCGCGCAATCAGGCAGCCGACCAC
GCCTGCGACGCGGTGGCAATACAGTTTCAATTCTTCAAAACTGCCGTAACGGGCTTGAAC
CAAATCCATCTGCATCCCGTCGATTAAGGCTTCCAGTTCATATTTCGGCAGCTTGAAGGT
TTCCTTAACTTGCCGCAAGGCCTGATTGACGGGGTGTTCCGGCATCGCGCCGCCGAATAC
CTTGTCCAAATCGCCGCGCCACCAGTTCAATGTCGCCTGTGCAACATCGGGGTTGGAACA
TTCGTCAACCACATCGTCCAATTCGCGGCAAAAAGCATATAAAACCGTTACCGCATCCCG
TTTTTCCTGAGTCAGGAAACGGAAGCCCGACAAAAAACTGGAGCGGCTTTCTTCTGCTTT
TTGGCGGCAATAGTCGAGTCCTTTCACGATTTATATTCCTAATGATGGGCGGGAAAGGCG
GATTTTATCGGCATTTGGCGGTAGAGGGCAATTTCGGCGGCACGACCTAATCCTTAGCGG
TTTGCTCAACTATCGGCGCAAATTCTGTTAAAATGCCGCTTTCCTTCCTTTACACACCGC
ACCGACAGGCAGAATTTATGGCTCTTTTGCAGATTTCAGAACCGGGTATGTCCGCCGCCC
CGCACCGGCACCGTTTGGCGGCAGGCATCGATTTGGGTACGACCAACAGCTTGGTCGCCA
CCGTCCGCAGCGGCAGTGCCGCCTGCCTGCCCGATGCCGAAGGGCGCGTTACCCTGCCTT
CCGTCGTCCGCTATCTGGAAAACGGCGGCATTGAAGTCGGCAAAACCGCCCTGTCCGCCC
AAAAAACCGACCCGCTGAACACCGTCAGCTCCGCCAAACGCCTTATCGGGCGGACGCTTG
CCGATCTGCATCAAAATACGCACTACCTGCCTTACCGTTTCGGCGACAATCAACGCGTTA
TCGAACTGCATACGCGGCAGGGGGTGAAAACGCCTGTCGAAGTGTCGGCGGAAATCCTCA
AAACCCTTAAATCGCGCGCCGAAGAAACCTTGGGCGGCGATTTGGTCGGCGTGGTGATTA
CCGTCCCCGCCTATTTCGACGATGCCCAACGCCAGGCCACCAAAGATGCCGCGCGTCTGG
CGGGTTTGAACGTATTGCGCCTGCTCAACGAACCCACCGCCGCCGCAATCGCCTACGGGC
TGGACAACGCCTCGGAAGGCACGTTTGTCGTGTACGACTTAGGGGGCGGCACATTCGACG
TATCCGTATTGCAACTGACCAAAGGACTGTTTGAAGTCAAAGCCACCGGCGGCAACAGCG
CGTTGGGCGGCGACGATTTCGACCACCGCCTGTTCTGCCGCCTGCTCGAACAAAACGGAC
TCTCCCAACTCAACGAACAAGACAGCCAACTCCTGCTCTCGCTCGTCCGCGCCGCCAAAG
AACAATTAACCACGCAAACCGAAGCGCGCATTCAGGCGACGCTTTCAGACGGCATGGCAA
TCGACACAAGCATCAGTCGCGCCGAGTTCCACAACCTGACGCAGCATTTGGTGATGAAAA
CGCTCGAACCGGTCACACAGGCGTTGAAAGATGCCGGTGTCGGTAAAAACGAAGTCAAAG
GCGTGATTATGGTCGGCGGTTCGACCCGTATGCTGCACGTCCAACAGGCAGTCGCCACCT
TCTTCGGACAAACCCCGCTGAACAACCTCAACCCCGACGAAGTCGTCGCGCTCGGCGCCG
CCATACAGGCAAACGTCCTCGCAGGCAACAAAACCGACGGCGAATGGCTGCTGCTGGACG
TTACGCCCTTGTCGCTCGGTTTGGAAACCTACGGCGGCTTGGCGGAAAAAATCATCCCGC
GCAATTCCACCATCCCCACCGCGCGCGCGCAGGACTTTACCACCTTCAAAGACGGTCAGA
CCGCGATGACGATACACGTCGTACAAGGCGAACGCGAACTGGTTGCCGACTGCCGCAGCC

## Appendix A

TTGCCAAATTCACCCTGCGCGGCATTCCGCCTATGGCGGCGGGTGCGGCGCGTATCCGCG
TAACCTTCCAAATCGATGCGGATGGGCTGCTGTCCGTTTCCGCCCAAGAACAAAGCACCG
GCGTACAGGCGCAAATCGAAGTCAAACCCTCCTACGGCTTGGACGACGACACCATCACCC
AAATGCTCAAAGACAGCATGAGCAATGCCGCCGAAGATATGGCGGCACGCGCCCGTGCCG
AAGCCGTAGTCGAAGCCGAAAGCCTGACCGATGCCGTCAACGCCGCCCTCGAGTTGGACA
GCGATTTGCTGGATGCCGAAGAATTGCAACAGATTCGGCAAGGCATCGCCGATTTGCAAG
GCCGTCTGAAAGACGGAAAAGCCGAAGACATCCGTGCCGCCGCCGCCAAACTCGGCAGCA
TCACCGACAATTTCGCCGCCAAACGCATGAACCGCAACATCCAACGCGCGCTGACAGGCC
AGAGTGTCGATAATATTTGATACTTAAACGGTTTCAGACGGCATAGAAATAATCCGATGC
CGTCTGAAGGCTCGAAAACACTTGAAAAACATCGATATGGAAAAGTCAGGCATTGTCTAT
CTGATGAAAACCGTCATCAAGGGCGTGTATAAAATCGGCATTTCGGATGTAAGCAATTTT
GAAGGCAGAATGCGCCATTTGGAAAACAACGGTTATGCGAACGTTGCCGGATTGGAACGC
ATCCTCGCCGTCAAAACCGACAATTACAAAGAAAAAGAAAACCTGCTCCATGAAATTTTC
AGCAAAAGCAGGATAGGCGATACCGAATTGTTCGCCGTGGACGAAAACCTTGTGAAACGT
TTGTTTTTATCGCTTCGCGGCGAAATCGTGTTCCCGAAAAACGAAACGGCGGAATCGGAA
TTTGAAAAAAGCGTCCACGAACGCAGGCAGGAAGGGAATGCCGGGTCAGGCCGCAAACAA
CTGCTTGATTTGGTACGGCGCGGACACCGGGAATACCCTTACGCGCTGCCCCGGCTTTTG
GCGGGCGCGGCATTCTACAAGCCGAAAAAATCGAAAATCCGCCTTTTTAAAGAAGCATAT
TTCGGCAAAAGCGGCACGAGGCTGACCGACGAAATTGCAGACGGCATCCATATTTACACC
TGTTTTTCGCGGGCGGATTTGGAAAAAGCCTATTCCGAATATTTGGAACTTTTCAAATCC
GAATCGGATGCCGAAGGCAGAAAGCCGCAGTAAGGTGCAAACAGATACCGTACACGTTGA
GGAGCAGATATGATGGGCGATTCCGTCATTTATTATGTAGAACAGGCAGACGAACCGGTA
AACCGTGCCGACGAACGCGCCCGTAAAACATTCAAATATTTTTGGCGCGAGCTTTTTTGG
GAACGCCGCCGCATTATTTCCGCCTTGGATTTTGCCATGGTCAAAGTCCCTTTTTTCCAA
GACGGCGAAGACGGCGAAATTTGCGAACATATGTGGATCGACGATATTTATTTTGACGGT
CTTTATATTTACGGTGTGCTGAACAATGAACCCGGCGAACTGACCAATGTCGAACAAGGC
GAAAGCGTTTGCGTTCCGGTTGACGACATCAGCGACTGGATGTTCGTGTGCAACGGCATC
CCCTACGGCGGCTTTACCATACAGGCAATGCGCGGGCAGATGACGGAAGAGGAGCGCACC
GAACACGATGCCGCATGGGGAATCGATTTCGGCGATCCCGGGCAGATATTGCTGGTGTAT
GAAGAAAAAGAACATCCCGAAAATCTGGAAGAGCATCCGATGTGCCGGAACTGTATTGAC
GATTTTCGGCAACAGTTGTCCCAAAACTCGGATTATCTGCGGGAACAGGACGAAGACGGC
TATACGCCGCTTCATCATGAAGCCATCGCAGGAAATGCACTTATGGTTCAAGCCATGCTT
GAATACGGCGCAAATCCTGCCTCAACGACATCGGAAGGCTATACCGCCCTCGATTTTGCC
TGCCTGACGGGCTGGCAAAATGTTGCCGACCTGCTCGAACCGCGACATTAGGCAGACAGT
TTTCCGAAAACGAACACAAACACTTTTTACAGAAAGACAATAAAAATGCCCAAAATCACC
GTACTTCCACACACGACATTATGCCCCGAGGGTGCAGTCATCGATAACGCACCCGAAGGT
AAAACCGTCCTTGACGTGCTGCTCGACCATGATATCGAAGTCGATCACGCCTGCGAAAAA
TCCTGCGCCTGCACAACCTGCCACGTGATTATCCGCAAAGGTTTCGACAGCCTAGAAGAG
CCGACCGAATTGGAAGAAGACCTGCTCGATCAGGCTTGGGGTTTGGAAGCCGATTCGCGC
CTGAGTTGTCAGGCGGTTGTCGCCGGCGAGGATTTGATTGTGGAAATCCCCAAATACACC
ATCAACCACGCGCGCGAAGAACACTGAAAACAGGCCGTCTGAAGCCGGCACGCTTCAGAC
GGCATTGTTGCGCGGATAAGGCGCAATCGCCCGAAAACAGGCGTTCGTACAGGCGGAACT
TTCGATTCTATAGTGAATTAACAAAAATCAGGACAAGGCGGCGAGCCGCAGACAGTACAG
ATAGTACGGCAAGGCGAGGTAACGCTGTACCGGTtTAAATTTAATTCACTATATATTGAT
TTTTATCGGTTTTCTGACGGAATAATCCAGTGCGGCATCCGAGGCGGATTACTCGGACGC
GATGCACCGGTATTTATCGGTTTTGCAGCCGGAAAAACCGCCGGCGGGTTATAGTGGATT
AAATTTAAACCAGTACAGCGTTGCCTCGCCTTGCCGTACTATCTGTACTGTCTGCGGCTC
GCCGCCTTGTCCTGATTTTTGTTAATCCACTATACTTTTAGGGCGACGGTCGGGCAGTAT
GCCGGATAGCGTTCCACTCTCGCTTCTATATTGATTTTGATTGGTTTTCTGACGGAATGA
CCCGATGCGGCATCCGGGACGGCTTGTGTTTTTTCCTGCCCGCCTGCCGGATTTTCCCAT
CCTTGCGTGAAACCGAAAGAGACGGCGGCGCGGCGGACAAGCTCGAGATAGCGTCCTTCA
AGCTCCGGACAGGCGGCGGACACGCTTTCTACCGTAACCGTGAAACCGCCGCCCGACCCG
GCAAGGCGTTCCGCAATTTGCGTGTAGATGAGCCAACGTTGGGCGCGCAACATTGCCGAA
TCGCAACCGGCAGCCGCTTTATCCAAGGCAATCAGGTCGCGCCGGTTTTGGTGGTGAAGG
CAAACCGTTACTCTGGAGAGGATATGTTGCCCGTCCAATATTTGATACAGTTTGCGTATC
AGCAGAATCAGGCGGTCAAACTCCTCCATCTCCGACAGGGAATCAGGATGGTCGGAAGCG
GTATAAACCAGTTTGGACAATTTTGCGGCAAACATATTGTTCATCAATCTTCCTTGTCGG
TTGAAACCCCGCTCTTCGGGGCGGTAGAATCAGATTTGTTTGGGAGGGACAACTCCTCCC
AGATCAGGACGACACATAGGCTGGTGCTTGATGTGTTGTCCGGCGAGTTGAAACATTCAG
CAATCCTCAAGGGGCGGCAGTTTTGCCGAAACATATTCTACACGGCTTCAATGCCGGACG
ATAAAAGGAAATTCATATGAAATGGACCGACACCCAGCGCATCGCCGAAGAACTCTATGA
CCTGCACGGCGAAACCATCGATCCCAGAACCGTGCGCTTTACCCAACTGCGCGACCTGAT
TATGGCATTGCCCGAATTTGACGACGACCCCGCCCGTTGCGGCGAACGCATCCTCGAAGC
CGTGCAGCAGGCATGGATAGACGAGGCGGAATAAGTTTCGGGAATGCCGTCTGAAATGCG
GCGGTACGCGGTTCGTGCTTCTGTTTGCAGCGGGAATGGTTTTACCAGTCTCCTTTTTTC
AGCCTGTCCAGTTGGCGGCGGTCGCGCTTGGTCGGTCTGCCGTCGGGATAGGCGGAAGTG
ATGCGGCTGAATTGGTCGAGCTGTTTGCGCTCTTCCCTCAATGTTGCCGTTTTCGCGTCC
TCTTCATACAGAAGCCGCGCCTCGGATGCCGGGCGGCGTTGGTGGTTCAAACCTTTAACC
TTGATTTTATAGGGAAGGGAATTGAGCGTCAGGTCGATAATATCGCCGATGTCTATGGTT
TTACTGTTTTTGACCTTCGAGCCGTTTACTTGAACCCTACCCAGTTCGATGTGCTTTTGC
GCAAGGGAACGGGTCTTGAAAAAACGTGCCGCCCAAAGCCATTTGTCCAGCCGCATGGCG
GAAGAATCGTGCTTGTCTTTCATACGATTTTGTTTGAAATAATTGAATTTGTTTCGAGTT
TAGCATAAGATACGCCGCCTTATAACTAGTATATATGCACTAATCCACTGTTTTCCATGC
TGTCCGAACACAAAAAGAGGGTATGGAAAAGCCGTTTTGGACAATAAATTAACTGCGGAA
TATGCACAAATAGCGTATGATAGCGGCAGAATCTGTTGATGAGAGCTTCATTCTATGAAA

# Appendix A

CCTGTTTTTTTGGATTTTGAACAACCCATAGCCGAACTGACCAACAAAATCGATGAGCTG
CGTTTCGTCCAAGACGAGTCTGCCGTCGATATTTCGGACGAAATACACCGTTTGCAGAAA
AAAAGCAACGACCTGACCAAATCGATTTTCAGCAAACTCACACCCGCCCAAATTTCACAG
GTTTCCCGGCATCCGCAGCGTCCCTATACTTTGGATTACATTGAGGCACTGTTTACCGAT
TTTGAAGAACTGCACGGCGACCGCCACTTTGCCGACGATTATGCGATTGTCGGCGGATTG
GCGCGTTTCAACGGACAAAGCGTGATGGTCGTCGGGCATCAGAAAGGGCGCGACACCAAA
GAAAAAATCCGCCGCAACTTCGGTATGCCCCGTCCTGAAGGCTACCGCAAAGCCCTGCGC
CTGATGAAGACGGCAGAAAAATTCGGCTTGCCCGTAATGACCTTTATCGATACGCCGGGC
GCGTATCCCGGCATCGGCGCGGAAGAACGCGGGCAGTCGGAAGCCATCGGCAAAAACCTG
TACGAACTGACGCGCCTGCGCGTTCCTGTTTTGTGTACCGTCATCGGCGAAGGCGGTTCA
GGCGGTGCGTTGGCGGTCGCCCTAGGCGATTACGTCAATATGCTGCAATACTCGACCTAT
TCTGTTATCTCCCCCGAAGGCTGCGCGTCTATTTTGTGGAAAACCGCCGAAAAGGCGGCG
GATGCGGCTCAGGCTTTGGGCATTACTGCTGACCGCCTGCAAAAGCTGGACTTGGTCGAT
ACCGTCATCAAAGAACCATTGGGCGGCGCGCATCGGGATTTCGGGCAAACCATGAAAAAC
GTAAAAGCCGTTTTGGAAAAACAACTGCACGAAGCGCAAAGCATCCCGCTTGCCGATTTG
CTTTCGCGCCGTTTCGACCGCATTATGGCTTACGGCAAATTTTCGGAACAATAATTCAGG
TAGAACAAGCAGCAAGCAGTTTGTCTGAAACTGCTTGCTTTTTCTTTATCGGGACGGAAC
CGTGCTGACTTTAGATGCGTTTGAGCAATGCTTGAAGGATTGTTTTCCTCAAGGTCTGAA
TGGAAAAAAAAACAGCGGTGGCATTAAGCGGCGGCTTGGATTCCGTCGTTTTGCTGCATCT
GCTTGTCCGCGCCGGAAAAAAGGGCGGTTTTATTCCGGATGCATTGCATATCCATCACGG
CTTGAGTCCCCGTGCCGACGATTGGGCAGATTTCTGCCAAAACTATTGCGATATGCTCGG
GGTGGGGCTGGAAACGGTTAAGGTCTGCGTGGAAAAAAACGGTTTGGGCATCGAGGCGGC
GGCAAGGCAAAAGCGTTATGCCGCGTTTGCCGAAAAAGGCTTTGACGTTTTGGCGTTGGC
GCACCACAGGGACGATCAAATCGAAACCTTTATGCTGGCGGTCGCGCGCGGCGGCGGTTT
GCGCGCTTTGGCGGCTATGCCCGCCGTCCGCCCTTTTGGGGAAAAAGGCATCATCTGGCG
GCCCTTGCTGCCTTTTTCACGCCAAGATATATGGGATTATGCCCAAAAACACGGTTTGCC
GAATATCGAGGATGAAAGCAATACCGATACGGCTTATTTGCGAAACCGCTTCCGGCACCG
TATTTTGCCCGAACTTTCGGCGCAGATTCCCCATTTCGGGCGGCATGTGCTGAACAATGT
CCGCGCTTTGCAGGAAGATTTGGCTTTGTTGGACGAGGTCGTCGTTCAGGACTGCCGTTG
GGTTTGCGGGGCCGGTTATTTCGATACGGCGCGGTGGCTGACGTTTTCCCCGCGCCGGAA
AACCCATATTTTGCGGCATTTTCTGAAGGAAAACGGCATTCCCGTGCCGAATCAGAATGC
CCTTGCCGACATTGCCCGGGTTTTGACGGAGGCAAAAACCGGACGTTGGAACTTGCAAGG
CTTTGAATTGCATCATTATGCAGGCAGGCTGTTTGTGTTCCGACTGGAAAAAACGGATAA
ACTGCGGTTTTTGAAAGACAGGCAGATAAGCGGAAATTTAAGGGAAATATTGACGGGGCA
GGGATTTGTGTTGAAGCGGCATCCGTTTGGGCTTCCTGAGCATCTTTTGGAGCAGGACGG
AATTTTGAGGACGGTAGCGGCATCGGATACGTTGGCCATGGGCGGCATCCATAAGGATGT
GAAAAAAATCCTTCAGGGGAAACGGGTTTTGCCTGTCCTGCGCCCAATTTGGCCGCTTGT
TGCCGACAGCGGAAACCGTCCATTGGCGTTGGCAAACTGTTGTGCGGATTTCCAATACTC
GGTTTCAGACGGCATTTTGCCCGTCCATCCTGACTTTCCCATTTTATTTTGATAATATCG
CAAACAGATTTCGGCGGCGTTCAGTCGGGTATTGTCCGGTTGCATATTTCTAAAAGGCTT
GTGAAGTGAAACACATCAGTTCGACCAATAATGAACACATCAGACACCTGCACCGCCTGT
TGTCGCAAGGAAAGTTCAGACGGCAATACGCCCAAACCGTTTTGGAGGGCGTGCACCTGC
TTCAGGTTTTCCTGCAATCCGGCGGGATGCCGGTCGGGGTATATATTCCCGAAGCGAAAA
TGCCGTCTGAAGAAGTCCGTAAATTGACGGCGGTTTTGCCGGAAGACGGGTTTTTTTCCG
TTTCAGACGGCATATTGAAGAAAATCAGCAGCCTGACTTGTGCGGATGATGTGCTTGCGC
TGATTGATATTCCAGATGCGGGTGCTTTGCCGGCCGGCGGCGATTGCGTGGTTTTGGACG
GCGTGCAAGACCCGGGCAATGTCGGCACGGTGTTGCGAAGCGCGGCGGCGGCGGGAATCG
GCGCGGTCATTTTGGGCAAAGGTTGTGCGGACGCGTGGTCGCCCAAAGTGCTGCGAGCCG
GAATGGGCGCGCATTTCTTGTCGGAGATTTATCCGCAGGCGGATTTGGAAATATGGTTGG
TGCGCTATAAAGGCCGTGTGTTTGCCACCGCCTTGCGCGAGGAAAAGCAGGCGGTTTTGT
ACGGCGAAGATTTGTGCGAACCGACAGCCTGGGTGTTTGGCAACGAAGGCGCGGGGGTCG
GTAAAGCAGTTTTAGATAGGGCGGACAAGTGTGTCAGGATACCGATGCACGATGCAACCG
AGTCTTTAAATGTCGCGATGGCGGCGACAATCTGCCTGTTTGAACAAATGCGCCAACGGG
CGGCGTATTGAGGAAGAGAAATGCCGTCTGAAAAAATCTATTACGGCGTATTGATTTTCT
TATGTATCGCTTCTATGCTGCTGTCGCCGTTTTTTTATGCGGGTGCTTTGAAGCCCAAGA
AGGCGGCATTGCGGAAGGACGGGCAGTGGAAACTCATCTGATTGTCCAATGCCGTGGCGG
CGGCGGTTTTGGCTTGGGTGTGGTGGAAATGGTTTTGACAGATATTGCTCAAAATCGTGC
TAATGGAATCCGAACAAATAAAGAATTTGGTAAAAAATTTGTTAAATCAACGAATTAAAG
TTTTGTGGAAAACAAAACAGCTCTAAGCAAATAGGGCGTTTGTCGGTAAATACGGAAGAG
TTGCGGCATTATCGGGCATCTTTAACAAGTAGTGCCGTCTTGACAGGCAATCGGTTTTTA
TGGGCAGCTTGCAAAATCGCGGATATAAAATTGCGAATCGGTTAAAGTGTGGGGACGCTA
TGAAAAATTGCGAATTTTTTATGACCCGACAAGGGCAATCTATGATAGCGGTGCAGATTA
CTTAACTAGGGAAAAACATAGATTAGTCGTAATTGCAAATAGTGCTTGGGGGCTATTGCT
TAATTTATCTTGTTATTATGACGAGGTTTTGGAAAAGCGGAAAATACCGTTCGGCAAACA
GGAAATTGATGACGATATGGACAAAGTGTCCGCCCTTAAGCGGAAGTTTAAAGATATTTC
TGAAATCAAAGTAGGGGATGGTTGGGAATACCCGTTCAATTATGAGCAGGGAATGAAAGA
ATTAGATGAAGTGCTATTGAAATACATTCCCTTTTTTGAAGAAGAACGATAAAGGAGGTT
GATATGCGCGTATCTAAAATAATTGGAAGTATGTTGCTTGTTACAGCGGTTCAGACCGTA
TTTTCGGCAAATGTTTACGCGTGCCGCCATAATGGTAAAACCAGTTACAGCCAAACTCCG
GGAAAACATTGTACCAACGCGGGTTTGGGGCGGACCGGGTGTACAGTTCGGTTAGACCG
GCAGTAAAAGACAGGGCGGAAGACGCAGGGGTCGGCGATTATTCGGACACGGTGAGGGAC
GAACACGTCCAAAATCCGAAAGGAAATGCACAGAAAGACGGTTCGGCTGCCGGCATCAAG
CCGCACTGATTGAAGCCGAATCAGCCCTTGCGCTGTCGGACGGCAAAATTTGAACGATTG
GGGAACTTTGATGAAACACATCCATATTATCGGTATCGGCGGCACGTTTATGGGCGGGCT
TGCCGCCATTGCCAAAGAAGCGGGGTTTGAAGTCAGCGGTTGCGACGCGAAGATGTATCC

## Appendix A

GCCGATGAGCACCCAGCTCGAAGCCTTGGGTATAGACGTGTATGAAGGCTTCGATGCCGC
TCAGTTGGACGAATTTAAAGCCGACGTTTACGTTATCGGCAATGTCGCCAAGCGCGGGAT
GGATGTGGTTGAAGCGATTTTGAACCTCGGCCTGCCTTATATTTCCGGCCCGCAATGGCT
GTCGGAAAACGTGCTGCACCATCATTGGGTACTCGGTGTGGCGGGGACGCACGGCAAAAC
GACCACCGCCTCCATGCTCGCATGGGTCTTGGAATATGCCGGCCTCGCGCCGGGCTTCCT
TATTGGCGGCGTACCGGAAAATTTCGGCGTTTCCGCCCGCCTGCCGCAAACGCCGCGCCA
AGACCCGAACAGCCAATCGCCGTTTTTCGTCATCGAAGCCGACGAATACGACACCGCCTT
TTTCGACAAACGTTCTAAATTCGTGCATTACCGTCCGCGTACCGCCGTGTTGAACAATCT
GGAATTCGACCACGCCGACATCTTTGCCGACTTGGGCGCGATACAGACCCAGTTCCACTA
CCTCGTGCGTACCGTGCCGTCTGAAGGCTTAATCGTCTGCAACGGACGGCAGCAAAGCCT
GCAAGATACTTTGGACAAAGGCTGCTGGACGCCGGTGGAAAAATTCGGCACGGAACACGG
CTGGCAGGCCGGCGAAGCCAATGCCGACGGCTCGTTCGACGTGTTGCTCGACGGCAAAAC
CGCCGGACGCGTCAAATGGGATTTGATGGGCAGGCACAACCGCATGAACGCGCTCGCCGT
CATTGCCGCCGCGCGTCATGTCGGTGTCGATATTCAGACCGCCTGCGAAGCCTTGGGCGC
GTTTAAAAACGTCAAACGCCGGATGGAAATCAAAGGCACGGCAAACGGCATCACCGTTTA
CGACGACTTCGCCCACCACCCGACCGCCATCGAAACCACGATTCAAGGTTTGCGCCAACG
CGTCGGCGGCGCGCGCATCCTCGCCGTCCTCGAACCGCGTTCCAACACGATGAAGCTGGG
CACGATGAAGTCCGCCCTGCCTGTAAGCCTCAAAGAAGCCGACCAAGTGTTCTGCTACGC
CGGCGGCGTGGACTGGGACGTCGCCGAAGCCCTCGCGCCTTTGGGCGGCAGGCTGAACGT
CGGCAAAGACTTCGATGCCTTCGTTGCCGAAATCGTGAAAAACGCCGAAGTAGGCGACCA
TATTTTGGTGATGAGCAACGGCGGTTTCGGCGGAATACACGGAAAGCTGCTGGAAGCTTT
GAGATAGCCCGGGCGATGCCGTCTGAAAGCCCTTCAGACGGCATCGCCCGGCTGCGCGGC
ACAAAGGCGGAAAAACCGTTTGCCCCGTATTTTCAAACGCGTTACACTTGCCGCCGCTGT
TTTCAGCCATTTGATTACCCGCAACCGCCGTCATTGCGCCGGCGGTTTGCCTGTCAGCGT
CATTGCGCCGCTGTAAATACGAAAGAACACATTATGACCGTATCCCCCGTCGCCTTGCGC
CGTAAGACCGAGTGCAAGCCTCATCCCACCGCGCGCTATTGGAAAAAATGCGATGTCGAA
GCCCTGTTCGGACTTCCCTTCCTCGACCTCATTTACCAAGCCGCCGAAATCCACCGCCAA
AATTTCAACCCGCGCGAAATCCAGCTTTCCACGCTGTTGTCCATCAAAACCGGCGGTTGT
CCCGAAGACTGCGCCTATTGTCCGCAATCGGCGCACCACAATACCAATCTGGGCAAAGAG
CAGATGATGGATGTGGATGAAATCGTCGAAAAAGCCAAAATCGCCAAATCGCGCGGCGCA
AGCCGGTTTTGTATGGGCGCGGCGTGGCGCGGCCCTAAACCCAAAGACGTGGAGACGGTT
TCCGCAATCATCAAAGCCGTCAAGGGCTTGGGTATGGAAACCTGCGGCACGTTCGGTATG
CTCGAAGAAGGTATGGCGGAAGACTTGAAAGAGGCGGGCTTGGATTATTACAACCACAAC
CTCGACACCGACCCCGACCGCTACAACGACATCATCCACACCCGCCAACACGAAGACCGA
ATGGACACCTTGGGCAAAGTCCGCAACGCCGGTTTGAAAGTCTGCTGCGGCGGCATCGTC
GGGATGAACGAAACCCGCGCCGAACGTGCCGGGCTGATTGCCAGTCTCGCCAATCTCGAC
CCGCAGCCCGAAAGCGTGCCGATTAACCGGTTGGTCAAAGTGGAAGGCACGCCGCTTGCC
GATGCCGAAGATTTGGACTGGACGGAATTTGTCCGCACCATCGCCGTGGCGCGGATTACG
ATGCCGCAAAGTTATGTCCGGCTGTCGGCAGGGCGCAGCAATATGCCTGAAGCAATGCAG
GCGATGTGCTTTATGGCGGGCGCGAACTCGATTTTTTACGGCGACAAGCTGCTGACCACG
GGCAATCCTGATGAGGACGGCGACAGAATCCTGATGGAAAAGCTCAACCTGTATCCCTTG
CAGTTTGAACCGGAAGGCGAGGTCGCCGAAGTGGAAAAAGCCTCTGGGATTAAAGTGGAT
TATTGACGATTGAAAAATGCCGTCTGAAACCCGGAAAAAGGCTTTCAGACGGCATTTGTC
CGGACGGCATTTCCAATATCTTTTTACCGGCGCGTGATGCTGCCGTCGGGCGAGACATCC
AGCCCGTTCCCCTTGGGGAAATCGCTGCGGTTCAGATAAATAATCCGCCCGATAACGGTT
TTCTCGGGGTCGATTTTGGGGATTTTATCGCCGACTTGAGTGATGGGGATAATGTTGCCG
GAAACGAACCGCCCCTGTTTGTCGGTGATAATTTTAAAAATGGGGGCGATGCCGCTGATG
CCGGAGGTGTTGATTGCGCCGTAGGTGGCAAAGTTGCCGCCGCTGTAGGAGATGAAGCGG
TCGCGGTAAAGTTCGACGGCGCGAGTAACGTGCGGCCCCTGCCCGAATACGACATCCGCG
CCGGAATCGACGGCAAGCCGCGCAAACTCAACGACGTTGCCCCTGTTTTCCCCATAGAAG
ATTTCGGTATCGAACGGCAGGTGTTCCGCCTGTTTCCCTTCCGCGCCGCCGTGGAACATC
ACAATGACGATGTCGGCTTTCTGTTTGGTTTTGGTAATCCGTTTTCTAACTTTGGCATAA
TCGTTCAGTTTGACGGCGGCAAGGTTGGGGGCGAAGGAGACGAAGCCGGATCTTACGCCG
TTTTTCTTCAGGATGGCGGTTTCAAACCTGTTTTCGATGCCCGAATATTTGATGTTCAAT
TCGTCAAGGTTCGCCCTTTATGCCGTTTCCGCACCGCAAACCGCCGGGGTCAAGCCCTCG
GCGCATCCTGCCGGAACGGAATCCCCGTGCTGCCGATTGACGGTTCAAACCCCGCGCCCG
TTTCAAATACCGGCGATGTGGACGGACAGGATGCGCCTGACGAAAAGACAGCCGATACCG
TTTCCATTATCGGCGTGGGCGACATTATGCTCGGCAGCAATTATCCGGTCGATTACCTGC
CCGATACCAATATTCTGAAAAACGTCGAATCTGCCTTGCAAGACGCGGACATTACCGTCG
GCAACCTCGAAGGCACGCTGTTTGACGAAGGCGGTACGCCGAAAAAATGTGCAAACCCCC
AAAATATGCTATGCATTCCGAACGCCCTCCGCATACGGGCAATACCTTGCCGACGCGGGA
TTCGACTACCTCAGCTTCGCCAACAACCACAGCAACGACTTCGGCGCGCAAGGCATCACG
GCAACGGCGGCGAGCGGCAGCTCTTTTACATACTCGATCGCGCTAAAGCCGCTGCCGATA
ACGAGGCCAAAATTGCGGAAAATACCGCCATCGCCCAGATAAATTTGTCCATCATCAGAC
CTTTACTGTTCAGACGAGACAGCATTTGCCGCACGTTTTGGGGCTTATCTTTCGATTTGC
GCTACGTCGCGCACCGCGCCTTTGTCGGCGGAAGTCGCCATCGCGCCGTAAGCTCTTAAT
GCGGCGGAGACGTAGCGGTCGCGGTTTTTAGGCTTCCATGCTTTGCTGCCGCGCGCTTCC
ATTTCGGCACGGCGTGCGGCAAGCTCTTCATCGGAAATGGCAAGGTGGATGCTGCGGTTG
GGGATGTCGATTTCGACGGTATCGCCTTCGTGTACCAAACCGATCGCGCCACCTTCCGCC
GCTTCGGGCGAGGCGTGTCCGATGGACAAACCTGATGTGCCGCCGGAGAAGCGTCCGTCG
GTTAAGAGAGCGCAGGCTTTGCCGAGGCCTTTAGATTTCAGGTAGGAAGTCGGATACAGC
ATTTCCTGCATGCCCGGGCCGCCTTTCGGGCCTTCGTAGCGGATGATGACGATGTCGCCA
GCGACGATTTGGTTGCCCAAAATGCCTTCTACTGCGTCTTCTTGGCTTTCAAACACGCGG
GCGCGGCCGGTGAATTTGAGGATGCTCTCGTCCACGCCTGCGGTTTTTACCACGCAGCCG
CGCTCGGCGATGTTGCCGAACAAGACCGCCAAACCGCCGTCTTGCGAGTAGGCGTGTGCC

## Appendix A

ACGTCGCGGATACAGCCTTTTTCGCGGTCGAGGTCGAGGGTTTTCCACATACGGTTTTGC
GAGAACGCTTGGGTGGTGCGTACGCCGCCCGGCGCGGCTTTGAAGCGTTCGATGGCACGG
GTGTTTTCGGGATTGGTCACGTCCCATTGTTCAATCGCGTCTTTCAGCGTCGGCGCGTGG
ATGGTGTGCACGTCGGTGTGCAGTTTGCCCGCTTTGTCCAGTTCTTTCAGGATGGCGAAG
ATACCGCCGGCGCGATGCACGTCTTCCATATAGTAGTCGTGGTTGTTGGGTGCGGTTTTG
CAGATGCAGGGCACGACGCGGCTTAAGCGGTCGATGTCTGCCATTTTGAAATCGACACCG
GCTTCGTTGGCAACGGCCAACAGGTGCAAAATGGTATTGGTGCTGCCGCCCATCGCAATA
TCCATCGTCATAGCGTTTTCAAACGCTTTTTTGGTGGCAATGCTGCGCGGTAACACGGTT
TCATCGTTTTGCTCGTAATAGCGTTTGGTGATTTCGACAATCATACGGCCGGCTTCGAGG
AACAATTCTTTGCGGCCGGCGTGGGTCGCCAAATACGAACCGTTGCCGGGCAGGGAAAGG
CCGAGTGCTTCGGTCAGGCAGTTCATCGAGTTTGCCGTAAACATACCCGAACACGAGCCG
CAGGTCGGGCAGGCGTTTTGTTCGACTTCCTCGACTTGCCGGTTGCTGACATTGTCGTCC
GCCGATTCAATCATCGCGTCAATCAAGTCCAAACGGCGTTCGGGCTGGATGTTTGCCACG
CCGATAACCTTGCCCGCTTCCATCGGGCCGCCGGAGACGAAGATGGTGGGGATGTTCAGG
CGCATCGCGGCAATCAGCATGCCCGGGGTGATTTTGTCGCAGTTGGAAATGCACACCAGC
GCGTCGGCGCAGTGGGCGTTGACCATATATTCGATAGAGTCGGCAATCAAATCGCGGCTG
GGCAGGGAGTACAGCATGCCGCTGTGTCCCATAGCGATGCCGTCGTCGATGGCGATGGTG
TTGAATTCTTTGGCGATTGCGCCGGCTTTTTCGATTTCGCGGGCAACCAGCTGGCCCATA
TTGTGCAGGTGGACATGGCCGGGCACGAATTGGGTGAAGGAGTTGGCAACGGCGATGATG
GGCTTGCCGAAGTCGGTTTCCATCACGCCGGTGGCGCGCCACAATGCACGTGCGCCCGCC
ATATTGCGGCCGTGGGTGGAGGTTTTGGAGCGGTATTCAGGCATAGTGTGTTTCCTTGTG
CCTATACCGTCTGAAAGACAGGGCTGTTTCAGACGGTATCGGGTACGGTTTTTTAGAGTG
GGAAAAGAGGGTATTTTATACCAAGTATCGGAATTTTGCGGGATTGAAACGGCGTGCGGC
AAAAAAGAAAATCCCCGCAGGAATGCGGGGACGGGTTCAGGCGCGGGCAATCGCGACGGC
TTTGGATGCGTCCCAAAAATCAACGGGTGCATTTAATACGGGTTTGACGATGCCCGTCCG
TATGGCGAAATCGCCGAAACCTTCGCCGATATTGCGTTCTGCCGCCCATTTGCCGATCAG
GTCGTCCAATTCGGCAAGGATTTCGGGCAGGGTGATGTTTTCTTTGTAAAGACGGGGGAT
GCGTACGCCTTCACGGTCGCCGCCGATATGGAGGTTGTAGCGTCCGACGGCTTTGCCGAC
CAGTCCGATTTCCGCCAACATCGCCCGTCCGCAGCCGTTCGGGCAGCCGGTAATGCGGGT
AACGATGTAGTCGTCCGACGTGCCGTGTTTCGCCATAATCTTATCCAGCTCGCCGATGAA
GTCCGGCAGCACGCGTTCGGCTTCCGCCATTGCCAGCGGGCAGGTCGGAAAGGAAACGCA
GGACATCGCATTTTCACGCAGCTTGCTGACATCGTTGCGGATTAATCCGTATGTTCGGGC
AAATTCTTCGATTTTTGCTTTGTCTGCTTCGGCGACATTTGCCACGATGAGGTTTTGGTT
GGCGGTGATGCGGAAATCGCCTTTGTGGATTTTGGCGATTTCCAACACGCCGGTCAGAAG
CTGTTTCCCGCCTTCGTCAACCAAACGCCCGCTTTCGATGAAAAGGGTTAAATGCCAGTT
GCCGTCTATGCCTTTCACCCAGCCGATGCGGTCGCCGCGCCCGGTAAATTTGAACGGGCG
TACGGGTTCGAACGGCATACCCATACGGCGTTCAACTTCCGCGCGGAAGTTGTCCAAGCC
CATATTTTGAATGGTGTAGCGGGTGCGGGCGTTTTTGCGGTCGCTGCGGTTGCCGAAGTC
GCGCTGCGTGGTTACCACCGCTTCGGCGGCCTTCAGCGCGTGTTCCGGAGGCACGAAACC
CAGTTCCAGTGAAATGTTCGGATAGGTTTTGGTGTTGCCGTGTTCCATCGAAAGCCCGCC
GCCTGCCAAAACATTGAAGCCGGCAAGCTGTCCGTTACCGTCTGAAACGGCGACGAAATC
CAAATCGTTGCCGTAGCAGTCCACATCGTTCAAGGGCGGGATGACGACTGCGGTTTTGAA
TTTTCGCGGCAGATAGGTTTTGCCCAAAATCGGCTCGTCTTCTTGAAGGAAGTCGTCGGA
ACTTTGAACTTTTTTGCCGTCCACCCACACATCCAGATAACCGCGCGTGCGCGGCAGCAG
GTGTTCGGAAATCTTTTTCGCGTATTCGTAAGCCTGCCGGTGCAGTTCGGACTCGATCGG
GTTGGACGTGCAAAGCACGTTGCGGTTCATATCCGCCGCCGTGGCGATGGAATCCAAACC
CAGTTTGTGCAAGAGGCGGTGCATCGTCTGCAACTTGGCTTTCGGCACGCCGTGAAATTG
GAAGGTTTGCCGGTTGGTCAGCCGGATGGAGCGGTAATGACTGTTTTCCCGGGCAAATTT
GTCCAGTTCTATCCATTGGGACGGTTTGATGATCCCGCCCGGCAGCCGGCAGCGCAAAAG
CATAAATTTCAAGGGCTCGAGTTTTGCCTCGGCGCGTTCGGCGCGGATGTCGCGGTCGTC
CTGCTCATACATACCGTGGAAGCGGATGAGTTGGAAGTTGTCGCCTTTGAAGCCGCCCGT
GAGCGGGTCTTTCAAATCGTCCAAAATCGTGCCGCGTAAAAAATTGCTTTCGGTTTTCAG
ACGTTCGTTGTCGGATAGCGGTTTTTCTTGCCACGCCAAACCTTTTGTCTTGGTCTGTAC
GGTCATTTTGTGTTCCTCCCGATTATATTTAATCAATAAACATCACGCTGATAGCGTTTT
TCTTCGCGCAGCATATCCAAATATTCTTCTGCGCCCTCTTCGTCCAAATGTCCTGCCCCG
ATAATCACATCCAGCAAGGCGGCTTCCACGTCTTTTGCCATTTTTGCCGCATCGCCGCAC
ACATAGATATGCGCGCCTTCCTGCAGCCATTGCCAAAGTCCTTCCGCCTGTTCGCGGATT
TTGTCCTGCACATAGATTTTTTCTTCCTGATCGCGGGACCAGGCGAAATCGTACCTGTGC
AGGAAGCCGTCTTTGGCAAACTGCTGCCATTCGGTTTGATAGAGAAAATCACGGGCAAAA
TGCGGATTGCCGAAAATCAGCCAGTTTTTGCCTTCCGCATTTTCTGCGGCACGTTGTTGG
ACGAAAGCGCGGAACGGTGCGACGCCGGTGCCCGAGCCGATCATCACAATCGGCTTGCGG
CTGTCTTCGGGCAGCCTGAAGCCGTCGTTGCGTTCCACAAACACGCGCACCGTGCCGTCC
TCTTCCAGCCGGTCGGCAAGGAAACCCGATGCGCCGCCCGTTCTGGCGCGGCCTTCGTGT
TCAAAACGAACCACGCCGACAGTTAAATGCACTTCATCGCCCACTTCCGCCTGTGCTGAA
GAAATCGAATACAAACGGGGTGCAAGCGGACGCAGTAAACGGATGAATTGTTCTGCCGTC
AGGCTTGCCGGGAAGCGGTGCAGCACATCGACAATAGGCGTGTTTTGCACGAAATCCTGC
AAAACGGCGTTATCGGCAATGATTTTATCGAGTTCTTCATAATGGGCGAACGCGGCATAG
CCTTTGACGAAAGCCGGAGTGTTTTGCGTGAGTTCGAAATGAGATGAAAGTGCGCGCGCA
ACCGGCATCATCTTTCCGCCCGCCTGTATTTCCGTTGCCGGATCGATGCCGAGCAGGTCT
AGGATTTCCCTGACCAGTGCCGGATCGTTGTCAAACCAAACGCCGAGCGCGTCGCCCGGG
AGGTAGTGCAAATCCGAACCGCTCAAATCGATTTCGATGTGGCGCACGTCTTTATCGGAT
TGGCGGGCGGTGATTTTCTGATTGGCCAGCAGGGCGGCGGGAAAGGGGGCTGCCTTGCAG
TACCTGCCATCCGGTGCCGTCTGAAGGCCGGCGGGGGGCGTTGTCTGCGGCGCGGGCGTT
GCCCGGTTTTTTGCGGCTTCTTCTTTAAGAGTGCGGCGATATTATCTGTCCAGGCGTTT
GCGGAGGCGGTAAAGTCCAAATCCGCATCAACGCGTTCGAGCAGCCGTTTTGCGCCCAAT

## Appendix A

TCTTCAAAACGCCGGTCGAAATCTTTACCTGCCTGACAGAAATTCGGATAGGAACTGTCG
CCCAAACCCAGTACGGCAAATTGGAGTTTGTCCAATTTCGGGGCTTTTTGCCGTTCAGC
AGTTTGTGCAGCACGACGGCTTCTTTCGGCGGTTCGCCTTCGCCTTGGGTGGAGGTAACC
AGCAGCAGGCGGCGTTCGCCGGCGATGTTTTTCGCCTTATAGTCTTTCAGTTCGGCGCGA
CTGACTTGGATGCCGGCGGCTTCCAGGCTGTCCGCCGCTTTGTCGGCAACGGATTTCGCA
TTGCCGGTTTGCGAGGCGGAAAGGACGGTTACGGAAAAAGGTTCTGCCGCCGGCAATGCC
GTCTGAAGCGCGGGCAGTCCTGCAGATGCCCCGTTTCCTGCTTTTGCCCAAGCGTAGCCG
GACAGCCACGCCCATTGTGCCGCGTCCAGCCCCGACAGGAGCTGCGTGATTTCGGGCGGC
AGAGGCGGTAATGGCGGATTTGTGTTCTGCATATCGTGTTCACTCATAAAATCATACCTG
CCGCAACAGTGCCGTATGTCGCTTCGTCTATCAGGATAAACGAACCGGCGGCGGTGTTTT
CCGCATAAGGCGTTGCCGTAACGGGTTTTTGAAGGTTGATGCGGACTTTGGCGATGTCGT
TCATCTTCAAGGATTCCGCGCCGGCCTCTTGTTCCAGCGTGCGGACATCCAAAACGCTTT
CAATTTCCCCGACTTTTGCCGGCACGGTTTGCGTGCCGTGCTTGAGCAGGTATTTGCGCG
CGGTGTTGAGCGGACGTTCGTCAAACCAGCAAAGCGTGGCTTCCAGATGTTTTTGCGGGG
CGAGCGGGGAATTTTTATCGACAAAAAGGTCGCCGCGCGAAACATCGATGTCGCGGTCCA
GCCGGAGGGTTGCCGCCTCGCCGGCAAAAGCCTGCGCCACTTCCCCTTTCGGCGTGATGA
TTTCGGACACTTCGGCGGTCAGCCCGTTCGGTTCGATGCGGACGGTTTGCCCGACGGCGA
CCGAACCGCGTTCGATGCGCCCCTGATAGCCTCGGAAATCATCGGCCTTGTCGGCATCTT
GGCGGACGACCAGTTGCACGGGGAAATAAAAATCGTCGGCGGTGCGGCTGACTTCGTCCG
CCCCCGGCAGGGTTTCCAAAATGGACAATAAGGGTTCGCCTTTATACCAAGGCATATTGC
CGCCGGGGTAAACAATGTTGTCGCCCAAGAGTGCGGACATCGGTACGAAATGCGCGTCTT
TCAAACCGAGCTGTTCGGCAAGTCGGCGGTATGCCTCCACAATGGCGTTGAATTTGTCTT
CGCTGTAATCCAGCAGGTCCATTTTGTTGACCGCCACCACAATATGCGGGCAGTTGAGTT
GGCGGAGGATGGCGGAATGGCGTTTGGTCTGCGGCAGAAGCTGCAAGGGCTGCGCGCCGA
AATCCAGTTGGGATGCGTCAACCAGCACGACTGCCGCCGAAGCGGTGCTTGCGCCCGTAA
CCATATTGCGCGTGTATTGTTCGTGCCCCGGCGTGTCGGCGATGATGAATTTCCGTTTCG
CCGTGGAAAAATAGCGGTATGCCACATCGATCGTAATGCCCTGTTCGCGTTCGGCTTCCA
GTCCGTCGGTCAGGATGGAGAAGTCTATGGCTTCTTTCAAACCTTTGCTTTTGCCGGATT
CCAAGGTTTTGATTTGGTCGGACAGCAGGGCTTTGCTGTCGTAGAGCAGTCGTCCGATCA
GGGTGCTTTTGCCGTCATCGACGCTGCCGGCGGTAATGAAGCGGAGCGGGGTTTGGTGTT
GTGCCGTCATATTTTCTTCCTCATATCTGCTTAAAGGGTTTTTGAAATTTAGAAATAGCC
TTCTTTTTTGCGTTTTTCCATTGCCGCCTCGCTTGCCTGATCGTCCAGCCGGGTCGCGCT
GCGTTCGGAAATGTCGGCAACCGCTGTTTCTCTGATAATCTCCGTCGGCGTGGACGCGGT
GCTTTCTACCGGGCAGGTGCAGCTGATGTCGCCGACGGTGCGGAAGCGGACATCAAGGAT
TTCGGAGGTTTCAGACGGCATTTTCGGGGTGAGCGGCGTTACAGGGACCAGCAGCCCCCT
GCGTCTGACCACTTCGCGCCTGTGGCTGTAATAAATCGGCGGCAGCTCGAGGTTTTCGCG
GGCGATGTATTGCCAGATGTCGAGTTCCGTCCAGTTGGAAATCGGGAAGACGCGCATATT
TTCGCCTTTGTGCAGCCTGGTGTTGTACAGCGACCACAGCTCGGGGCGTTGCGCCTTCGG
ATCCCATTGTCCGAACTCGTCGCGGAACGAGAAAATCCGTTCTTTGGCGCGGGCTTTTTC
TTCGTCGCGCCGCGCCGCCCATAAGCGCGTCGAAGCCGTTTGCCTCGATGGTTTCCAA
CAAGGTAACCGCCTGTGCCGCATTGCGCGAATCGGTTTCTTTGCGTAAGACCACCGTGCC
TTTGGCAATGGAGTCTTCCACGCGCCCCACTATCAGGCGGGCATTGAGTTTTGCCGCCTG
CGCGTCGCGGAAGGCAATCACTTCGGGGTAGTTGTGTCCCGTGTCGATATGCACCAGCGG
GAAGGGCAGTTTCACCGGCCGGCTGCCCAGCCGGAAGGCTTTGCAGGCGAGGGCGAGCAG
GACCACGGAATCTTTGCCGCCGGAAAAGAGCAGGGCGGGGTTTTCGCATTCTGCCGCCAC
TTCGCGGATGATGTGGATGGATTCGGATTCCAACCAGTCGAGTTGGGCGTTGTTCGGTTC
GGTTTTCGTCATACCATATTCCTTATTTCTTCTGTCTGATATTTATGAATTATTTGTGCA
GCCCGCATTCTTTGCTGTTTCTGCCTTCCCACCACCACCGCCCGGCGCGGATGTCTTCGC
CCGCCTTGACGGGGCGGGTGCAGGGGTCGCAGCCTATGCTGGGAAATCCTTGCCGGTACA
AATCGTTGTAAGGCACATTGTTGGCGAGGATGTATGCCCACACGTCGTGTTCCGACCAGT
CGAAAATCGGGTTGTATTTGCCGATGCCCCGTCCGGCATCGTATTCGGCAAACGGCAGTT
CCGTGCGTGTGGCGGATTGTTCGCGGCGTTGCCCGGTAAGCCAGGCGTCCGCGCCTGCAA
TGGCGCGGTTGAGCGGTTCGGTTTTTCGGATGCGGCAGCATTCGCGGCGCGCTTCAACGC
TGTCGTAAAAGGCAAACCTGCCTTTGCTTTCCACATAACGGTCGGCATCTTCTCGAACCG
GCCGGAAACGCTTTATCCGCAAATGGGGATATGCGCGTCCGAGCCTGTCCAGCAGGTTCA
GGGTTTCCGTGTGGAGCAGCCCCGTATCCAAGGTAAAAATGCCGATATTGAGGTTTTCGC
CGGCGATAAGGTCGGTAATCACCATATCTTCTGCCGCAAGGCTGCTGGCAAACCGTGCAT
CCCGGTGGCTGCCGACAATCCGGTGCAGGCGTTGTTTGAGGGTTTCCGTTTTTTCCGCAA
GGGCGGTTTCGCCGCCGGATCCGATATGCGGTATCTGCCACAGGGCGGGTTTGAACAGTG
TCGTTTCCATTTTTCCCGCCTTATGCCGCCCGTTGTCCGGCATTCAGTCCGCCCAATGCG
GGATACGTCTGCCCGACCCGGTTTTCTCCTTCGCCGTTTTCACCGAACCAGGCGAGTTTT
TCGTGCAGCCCCACCACTTCGCCTATGACAATCAATGCCGGATTCGGCGCGGTTTCGGCG
AGTTCGGCAAGGTTGGCGAGCGTGCCGGTTGCGGTTTTTTGAGCCGGCAGCGTGCCTTGG
CTGATAACGGCTGCCGGCGTGTCGGGCGAGCGTCCGTGCTGTTGCAGCCGTTCGGCAATC
AGGGCGGCTTTGAGCGCACCCATATAAATCACCAAGGTCTGGCGGCTGCGGGCGAGGGTC
TGCCATTCGATGTCGGGCGCATCCGCCTTGCGGTGGCCGGTTACGAAAACCGCACTTTGG
GCATAATCGCGGTGCGTGAGCGGGATGCCGGCATAGGCGGTCGCGCCGACGGCGGCGGTA
ATGCCGGGGACGACCGAAAACGGAATCTGATGGCGTGCCAAGGTTTCCAATTCTTCGCCG
CCGCGTCCGAACACGAAAGGGTCTCCGCCTTTCAAACGCACCACGCGCCTGCCTTCGCGG
GCCAGCCTGACCATAAGCGCATTGGTGTCCTCTTGCGGGGTGCGCTCGCCCCGGGCGCGC
TTGCCGACAAAAATCCGTTCCGCATCGCGGCGGACGAGGGACAGTATGCCGTCTGAAACC
AGCGCGTCGTAAAGCACCACGTCTGCCTGCTGGATTTCCTGCAGCCCTTTGAGCGTCAGC
AGCCCCGCATCGCCGGGACCCGCGCCGACCAGCGAGACGGAGCCGCCTTGATCATTTTGA
CGACTTTGTTCCAATTGGCCTGCCAATTCCCGTTCGGCAAGGGTGTTTTGCCGGTTTTTG
ACGAGGGCGGCGAAACGTCCGTTAAACTGCTTTTCCCAAAAGCGGCGGCGTTCGGTAACG

## Appendix A

GATTTCAGTTTGCCCTTGACGGCATCGCGCCACCTTCCTGAAATTTCCGCCATATCGCCC
AAAGACGGCGGCAGCAGGGCTTCCAGCCTTTCACGCAGCAGTCGGGCGAGGACGGGCGCG
CTGCCGGAGCTGGAAACGGCAATCTGAACCGGGTTGCGGTCGATAACCGACGGGAAGATG
AAGCTGCAATGGTCGCGGTCGTCCACCACGTTGACCGGCTTTTGGCAGCTTTCGGCAAGA
TGGAAAACGCGCCGGTTGAGGGCTTGGTCGCTGCTTGCCGCAATGATGAGGAAAACCGTG
CGGATGTGTTCGGCACGAAATTCTTCGGCAAGCCACAGGATTTTGTTTTCCGCCGCCAAC
GCGGAGAGTTCGGCATTCAGGTGTTTTGCGGCAACCCTGACCTCTGCGCCCGCCTTCAGC
AGCAGGCTGATTTTGCGTGCGGCGACCGCGCCGCCGCCTACGACCAATACGGGGCGGCCG
GCGAGGTTGGCGAAAATAGGGAAATAATTCACTGGCTGACTCCTTTGCTGTTTGCCCGCA
CCTTGTTTCCGATACGGTGCGTCGCGGCATTTTTGTCGGAATGCGGGTCATTTTAGACAA
AAGGATTTTCCCCGGTTAAATAATAAAAAGGTATTTGTTAGAAGCTGAAAGCTATATGGG
GGCGGCTGCGGATGCGGCGGTTTTCCGTTTTATAACGGTTTCGGAAGAAAAACGGCCTGA
AGCCGTTTCGGGCATTCAGACCGTTTGCGTGGTGAGGGGATGCCGTCCGAAGGGCGAAAA
GGGCTTCAGACGGCATTGATGTCGGGTTTCAGGACAGGAGCAGGATGGCGGCTGCGGCAA
GCGAGGCAACCGATAATGCGGCGGCAAGCGCGGCTTTGCCTGCAAAGCGGATTGAGGTTT
TGCCTTCGATGTATTTGAAGCCGGTTATCATCGGGAGGATGAGGTTTTTCTTTTTGAATA
CGCGGTATGCGGCGACGGCGGCGATGTGGATTGCAGAAAAAACGGCGAGCAGCTTGAAAA
AGTTGAGGTGGATTTTCCGCATAAGGCTGCCCGTATGTTCGGAAACCAAATGGTTGAGGT
AGCCGTTGGTGCTGAAGGTGTTTTCATCGGCGGCAAAAAGCCCGGTGCCGACTTGGAAGG
ACACGGCGGCCAAAAGCGCAACGACCATCAGTGCGCCCAAGGGGTTGTGTCCGGGCTGGA
TGTGTTCGGGAATACCGTTTTTCAGATAGCCGCGTATGCCTGCCCAGCCTTGGACGAAAC
GGGAAAAACGGGCGGTATCGCTGCCCCAAATGCCCCAGCAGAGGCGAAATACGAGCAGGA
AAAGGACGAACAGCCCGACGCGCGTGTGCCATTGCAGCATATCGCCGCCGGCTTTCGCGC
TATACCACATAAAGGGCAGGGACGCGGCAAGCAGCCAGTGGAAAAGGCGGGTGGGGAGGT
CCCAGACTTTGGTTTTGTTTTTCATAATCGGTTTCCGGCGGTAGAATCGGTTTGTTTTCG
AGCCTTATTTTAACCGATTGGAGGGGCAATGTTTCCCGTTTTTCATCTTTCAGGCGAGAG
CCGCCGCCAGATGCTTCAGACGGCATTGCGTTTTCCCCATGTTTTCAAAGCCCGTGCGGA
AGATTCGCACAAAGGGACTTTCGGCACGCTCGCCGTAGTCGGCGGATCGGCAGGGATGAG
CGGCGCGCCCGTATTGGCGGCATCGGCGGCAATGTATCTCGGCTGCGGCAAAGTGTGGGC
GGGTTTCAATCAGGATACGCTACCTTTTGCCGTTATTGCCGGTTTTCCCGAGATTATGCT
GGATACGGCGGACAGTTTGGCCAAACGTCAAGATATAAACGCCTGGGTTGTCGGTTGTGG
ATTGGGTACAGGTAGGGCGGCGGTCGGAACGCTTGCCGGAATTTTGACGGAACACACGGA
CAAGCCCGTCGTTTTGGATGCGGATGCGCTGAACATATTATCAACCGATGCCGAAACCCG
AAATCTGGCGCGCGGGTGTAAAAACCTGATTTTAACGCCACACCCCGCCGAAGCCGCGCG
CCTGCTTGGAACGACGGTTGCGCAGGTTCAGGCGGATCGGACGGCGGCAGTGAGGAAGAT
AGGGGCAATTTTCGGCGCAACCGTGGTTTTAAAGGGGCACAAAACATTGGTTGCCTCACC
CGATACGGAAATCTATGTCAACGAAAGCGGCAACGCGGGATTGGCAACGGCGGGCAGTGG
CGACGTATTGGGCGGCATCATCGGCAGTCTGCTCGCACAGGGCGTGCCGGTTTTTGAAGC
CGCCTGCGCGGGCGCGTGGCTGCACGGCGCGGCGGCGGATGTCATAAAAGAATCGGCAGG
CATTGCGGCAGGGCTGTTGGCAGGGGAAATCGCTCCGGCGGCAAGGTGGCTGCGCAACCG
GATAACTAAAAGTATGTAAGAAGATATAGTGGATTAACAAAAACCAGTACATCGTTGCCT
CGCCTTAGCTCAAAGAGAACGATTCTCTAAGGTGCTGAAGCACCAAGTGAATCGGTTCCG
TACTATTTGTACTGTCTGCGGCTTCGTCGCCTTGTCCTGATTTTTGTTAATCCACTATAC
CATACAACCACGCCGGAATTAAGTTTAAATTTGAATAAAAGGTTCGGGTTCTGCAAAATA
CAGAACCCGAACCTTGTTCGGATATTGAAACCGGCTGCCCGATTTTGGGCGGTGCGGCTT
GCAAGTATCAAGATTCGCATATGCCGTCTGAAGCTCGGAGAGGTTCAGACGGCATATGCT
TATTTGGGCTGCTCTTCAACGAATCTCGGACCTTTCAAGATGCCGTTGTGAGAATAGGGC
GACAGCAGGTTGTATGCGGCGGTTTTGGAAACCTGATAACCGCGGTCGGTCAGGCTGTTG
~~GCAATCTGATTGACCACTGGGCTGAGCAAAGGCGGCAACAGGCCGCTGTTGCTGTTGTTG~~
CTGCCTTCGCGGATGCTGGCCGAACCCGACCACAACTCTTTTCCGTTGCGGGAATCGACC
AGCCGTGCTTTGGCGGATACGGTCGTCACGCTGTCTAAAATTTGATATGAAGTGCCGTAT
TCGGTAACCGTAATGTACAAAACCGCATCATTGCCGAAAATCTGATGCAGTTTTTCCGGC
CGGACGGCGTGAATATCGGCGGCATTGGTCAAGCCGTTTTGTTTGAAGGTTTCCTCCACG
ACTGCGGCGGGGAAGACGTAATAGCCGGCTTCGGAAAGCGGCGCGGCGGTCGAAGCCAGT
ACACCCCATGTTCCGTTGACATCGGGCGATTCGTTCAGCGGCGGAACCACCAAAATTGAA
GCCGGTTTGCTTTCCTTGAATGACGTGTAGTCGAAATCGGGCGCTTTTTGAACTTGGCAG
GCAGACAGCGCCAACACGGCGGCAAGCCCTAAAATCAAAGGTTTCATCGCTTGCCTCCTT
TACCGGTTTTCATCAGGAAGTCCATAAATACGCCCGATTCGGGAAACAGCCTTTTCTCTT
CTTCAAACTGGCGGAACGCGCCCTCTTTGTCTCCCGAACGGGAAAGCAGCAGTCCCAGAT
GGGCGTGCGCACCCGGGGCGGCATTCATTTTTTTGTTGCCGGCTTCCACAAAGTATTTTT
CCATCTTTTCGGTCTGCTTGCCCAACGAAGTGTCGTCGTTTTTCAAACCTTCATAGACGG
TATCGGGATAGCCGCCGTAATAATACAGGGATTTTTGCCCGTTGCCGCCGCAGGCGGTCA
GAGCCAAGACCGCCGCACACAGCGACAAACGGCTCAAGGTTTTCGGATTCATCATTTCTC
CTTAACGGTTGGGTTGCCATGCGCCGTTGTCAACAGCCTGAACCAGGCTGTTGACGGCTT
CGCGGATTGCCAAGTCTAAAACTTTGCCGTTCAAAGTCGCATCGTAGCCGGAAGTGCCGC
CGAAACCGATGATTTCACGGTTGGAAAGTGCGTATTCGCCCGCGCCCTGTGCGGAATAGA
CGATTTCGGAAGTATTGACGTTGACGATATTCAGAGCCACTTTTGCATAGGCGATTTGCG
ATTTGCCGCGACCCAAAATGCCGAAGAGCTGATGATCGCCGACATCTCTGCGTCCGAATT
CGGTTACATCGCCGGTAACGACATAATCTGCGCCTTTCAGGTTATGCGCTTTGCCGGAAA
TGCCGGATTCCTGTTTTAATGCGTTCAAATTGGTGCGGTTCAGTACGTTGAAGCGGTTGG
TCTGTTGCAGGTGCGTTACTAGAATGGTTTTTGCCTGGCTGCCCAAACGGTCTTCCCCGT
CGGAGAAAATGCCTTTTTGGAAGCTGGAGCGGTTGTCGAATGTTCCGACGGAAATCGGGG
TACGAACACCGTGATATTGCGTATTGTAGGAGGCGACTTTCTCTACCTCGAGACTGCGTG
AGGATTCGGTCGCACAGCCGGTCAGTGAAACGGCAGCGGCGGCAAGGACAACGGCGGTGG
AAACGGTTTTCATAAAATTTACCCTAAGGTCAAGTTAAGGAAATAACGGGTTGTCATTAT

## Appendix A

TGTCCTTATGTAAATTTAAGTCAAGGTGTTTGTCTGTGCGGGACGGATGCGCGCGGAAGG
TACGGATATTTTTCAAACGGAATCGCGGCGCGGGCGGGATGCCTGCCTTTTCCGGATTTA
GAGGTAAACGATTTCGCCACTCCGCCCTTTGCTTTCGGCACTTGCCCACCAGACAAATGC
GGGCAGCACGTCCCCGTAGCTTTTGCGTTCGCTTTTGGCTTCGCCCGGATGGGATTTGAT
GCGTTGCGGGGAATTGATGGGGCCGGGGACGAGGACGTTGGCGCGCAGGTTGCCGAAGCG
TTCCCATTCGTCGGCGGCGACTTTGCACAGGTAGTTCAACGCGGCTTTGGACGCGCCGAA
GCCGCCCCAGTAGGCTTTGGGTGTTTCGCCGTGGCTTTCGCCGACGAAGATGACGGACGC
GTCGGGCGACTGCTTCAGCAGCGGGAACAGGGCGCGGGTCAGCCCCATAGGTGCGACGGT
GTTGATGCGGTATTGGTTGACCCATTCGGCGACGGTTTGGAAATCCAGCGGCGAGAGGGC
GTAAAAATAGCCGGCGCAGTGGACGATGCCGTCCAGTTTGCCTTGCGTGGCTTCGGCAAT
GGTGGCGGCGAAATGTTCAAATTCTTTTTCTTCCGCGCTAATAAGGTCAAAGCAGATGGC
GAATGGTTCGGGGTATCCGGCTTCGACAATCGCGTCATACACTTTTTCCAGTTTTTTCTG
ATGACGGGCAACCAAAATCACGGTTGCGCCTGCCGCCGCATAGGCTTTGGCGACCTGTTC
GCCCAGACCTTGCGATGCGCCGGTTACTAAGATGGTTTTGTCGGACAGTGTCGCCATACT
TTTTCCTTTTTGGTTGTCGGTTAAGGTATTTTAGCGTTTTGCCGCACCTTGTAAAGCGTC
CCGATGTCGGCCGGCGTTTTCAGACGGCATCGCTCAGGCTTTGCAGGCAGGCTTCCGCCG
ACGCGAAACCTGATTGTACGGCGGCTTCGAGCGTGGCGGGGTAGTCCGGGTGGAGGTAGT
CGCCGGCGGGGAAGATGCGGTGCCGGTGCAACCACGACAAGTCCGGCGGCGGGGCATCGG
CTGCGGTTGTGGCGCGTTTTTCGGTGATGACGCGCACGGCTTCGGGTTCGCCCAAATGCG
GAAGGATGCGTTTGAGGTCGGCGTGGGCTTTGTCCGCCCACGCCCGGTTTGCAAACGCGC
CGACGCGGTCGGAAACGCTGATGACGGCGGACACTTCGTTTTCAGGCAGTCCGAGCCTGC
CCCGGCAAAGCAGCCATTGCACCGTGCCGTCGGCAAGGCCGGTCAGCGGGGCGGGCAGGC
GGACGGGTTCGGCGTAGCGCAGATAGACGGTGGTGATGGCGTGGTAGCGAAGGTTTTGAT
ATGCCGTCTGAACGTGTTCGGGCGTGCCTTCGGGCAGGAGCGCGGCGGCGTGGTAGGGCG
CGGTGGCGGGGACGGCGGCATCGAAAGCTTCGCCGTTGACGAGCACTTTCCCGTCCGGGA
GGGTGTTCAGACGGCATACGCGCGTTTCGAGGCGGATGTCCGCGCCGAGCCGTTGAAGAT
CCGCCAAGGCGGGTTCGGCGACGATTGCGCCCAAATCCTGCTTGGGTAGGAGATAGTCGC
TGCCGGATTTTTTCGTCAGCACGCCGTCGGACAAAACGTTGCACAACACGCGCAGGCTTG
CGGTTTCCAAAGGCGTGTTGAGCGCGCCCCAAACCAAGGGCTGCCAAAACTGCATCACGG
CGGCACGCGGCACGTTCCGCTGTTTCAGCCATTGCGCCACTGTCGTGTCGGGCTGTCCGA
GGCGTGCGGACTTCTGCAAATCGGACATATCGGCAAGCAGTTTGGCTTTGAATGCAGTCG
GTGCACGCCGGGCAAGCAGCACGCCGCCCAAAATATGCAGCGGCGCGGGCAGGGGGAGGG
CGCGGAACTGCAAACCGCCGTGCATATGCCAGTGCAGCGGTACGCGCAAAAAGGCGGCAC
GGGGATCCGAACCGATGGTTTTCATCAGGCGCAACACGCCCCGGTATGCGCCGAGCAAAA
TGTGCTGCCCGTTGTCCAAAAAACCGAAACCGTCGGTATTTCCGGCCAGTGTGCGCGCCC
TGCCGCCCGCCTGCCGGCCGGCTTCAAACAGGGTAACGTCGGCGTGCCGCGCCAAGGTGA
CGGCGGCGGACAGTCCTGCCCAGCCTGCGCCGATGACGGCGATTTTCGGGCGCGGATGCG
GCGTGTTCATCATTTATTCCTCCAATGGTTTTGCAGCCGTATCTATTTCCGTTTCCGAAA
ATGACGGTAGAAAAGGATACAGGCTAAAAATAAAGGCAGCAGAAAGCGCGCATAGGGATA
CCACGGATGGTCTGCATGCCAGTATCCGTACCGTCCCTGTGGAACGGTATCATAGCGGTA
ACTGTCGGGGAAAAAGTTGATCCAAACCGTTACTGCCAGCCATAAGGCTATATCCCTGAC
TGCTTTCATTTACTTCTGCTCCTGTTTAAATTCCCAGCAATTCCATTTCAAAGCGCGAAC
GCCAACGGGATTGCGCGGTTACGATGCAGACTTTCAGACGATGGTTGAAACCCCGTCCGG
CGGGGCTGTGGGAATCGGGTTTGCCTGACGGGCGGCGGGCGGTATGCGCCTTATGCCCGT
TCCGGCGTGCCGGGGCGCGGTTTGAATCCGAATAACCAGGTTTTCAGGGCAATGCGTTTT
TTGCGCGGCGAAGGGAGGGCGATTTTGTATTTGAGGACGTTTTGTGCGCCGTCTCGGTCG
ATTTCGTTCAATAGCTCGTAATAAACCGCCGCCATGACCAGTCCGACTTTTTGGGCTTTT
TTATCGGCATCAGGCAGCAGCGATACGGCTTCGCGGTAGGTTTCGCGGGCGCGTTTGATT
TGGAACGCCATGAATTGGGCAAAATTGCCCGTCGGGCTGCATTGCAAAATCACGCTTGCG
GGTACGTCAAACCGCCGCATTTCCTCCATCGGCAGGTAAATCCGCCCCCTGCGCGCGTCT
TCGCCGACATCGCGGATGATGTTGGTCAGTTGCAGCGCAAGTCCCATCTTGTCGGCGTAT
TCCAGCGTTTGGTCGTCTGAAAACCCCAAAATCCGCGCAATCAGGCAGCCGACCACGCCT
GCGACGCGGTGGCAATACAGTTTCAATTCTTCAAAACTGCCGTAACGGGCTTGAACCAAA
TCCATCTGCATCCCGTCGATTAAGGCTTCCAGTTCATATTTCGGCAGCTTGAAGGTTTCC
TTAACTTGCCGCAAGGCCTGATTGACGGGGTGTTCCGGCATCGCGCCGCCGAATACCTTG
TCCAAATCGCCGCGCCACCAGTTCAATGTCGCCTGTGCAACATCGGGGTTGGAACATTCG
TCAACCACATCGTCCAATTCGCGGCAAAAAGCATATAAAACCGTTACCGCATCCCGTTTT
TCCTGAGTCAGGAAACGGAAGCCCGACAAAAAACTGGAGCGGCTTTCTTCTGCTTTTTGG
CGGCAATAGTCGAGTCCTTTCACGATTTATATTCCTAATGATGGGCGGGAAAGGCGGATT
TTATCGGCATTTGGCGGTAGAGGGCAATTTCGGCGGCACGACCTAATCCTTAGCGGTTTG
CTCAACTATCGGCGCAAATTCTGTTAAAATGCCGCTTTCCTTCCTTTACACACCGCACCG
ACAGGCAGAATTTATGGCTCTTTTGCAGATTTCAGAACCGGGTATGTCCGCCGCCCCGCA
CCGGCACCGTTTGGCGGCAGGCATCGATTTGGGTACGACCAACAGCTTGGTCGCCACCGT
CCGCAGCGGCAGTGCCGCCTGCCTGCCCGATGCCGAAGGGCGCGTTACCCTGCCTTCCGT
CGTCCGCTATCTGGAAAACGGCGGCATTGAAGTCGGCAAAACCGCCCTGTCCGCCCAAAA
AACCGACCCGCTGAACACCGTCAGCTCCGCCAAACGCCTTATCGGGCGGACGCTTGCCGA
TCTGCATCAAAATACGCACTACCTGCCTTACCGTTTCGGCGACAATCAACGCGTTATCGA
ACTGCATACGCGGCAGGGGGTGAAAACGCCTGTCGAAGTGTCGGCGGAAATCCTCAAAAC
CCTTAAATCGCGCGCCGAAGAAACCTTGGGCGGCGATTTGGTCGGCGTGGTGATTACCGT
CCCCGCCTATTTCGACGATGCCCAACGCCAGGCCACCAAAGATGCCGCGCGTCTGGCGGG
TTTGAACGTATTGCGCCTGCTCAACGAACCCACCGCCGCCGCAATCGCCTACGGGCTGGA
CAACGCCTCGGAAGGCACGTTTGTCGTGTACGACTTAGGGGGCGGCACATTCGACGTATC
CGTATTGCAACTGACCAAAGGACTGTTTGAAGTCAAAGCCACCGGCGGCAACAGCGCGTT
GGGCGGCGACGATTTCGACCACCGCCTGTTCTGCCGCCTGCTCGAACAAAACGGACTCTC
CCAACTCAACGAACAAGACAGCCAACTCCTGCTCTCGCTCGTCCGCGCCGCCAAAGAACA

## Appendix A

ATTAACCACGCAAACCGAAGCGCGCATTCAGGCGACGCTTTCAGACGGCATGGCAATCGA
CACAAGCATCAGTCGCGCCGAGTTCCACAACCTGACGCAGCATTTGGTGATGAAAACGCT
CGAACCGGTCACACAGGCGTTGAAAGATGCCGGTGTCGGTAAAAACGAAGTCAAAGGCGT
GATTATGGTCGGCGGTTCGACCCGTATGCTGCACGTCCAACAGGCAGTCGCCACCTTCTT
CGGACAAACCCCGCTGAACAACCTCAACCCCGACGAAGTCGTCGCGCTCGGCGCCGCCAT
ACAGGCAAACGTCCTCGCAGGCAACAAAACCGACGGCGAATGGCTGCTGCTGGACGTTAC
GCCCTTGTCGCTCGGTTTGGAAACCTACGGCGGCTTGGCGGAAAAAATCATCCCGCGCAA
TTCCACCATCCCCACCGCGCGCGCGCAGGACTTTACCACCTTCAAAGACGGTCAGACCGC
GATGACGATACACGTCGTACAAGGCGAACGCGAACTGGTTGCCGACTGCCGCAGCCTTGC
CAAATTCACCCTGCGCGGCATTCCGCCTATGGCGGCGGGTGCGGCGCGTATCCGCGTAAC
CTTCCAAATCGATGCGGATGGGCTGCTGTCCGTTTCCGCCCAAGAACAAAGCACCGGCGT
ACAGGCGCAAATCGAAGTCAAACCCTCCTACGGCTTGGACGACGACACCATCACCCAAAT
GCTCAAAGACAGCATGAGCAATGCCGCCGAAGATATGGCGGCACGCGCCCGTGCCGAAGC
CGTAGTCGAAGCCGAAAGCCTGACCGATGCCGTCAACGCCGCCCTCGAGTTGGACAGCGA
TTTGCTGGATGCCGAAGAATTGCAACAGATTCGGCAAGGCATCGCCGATTTGCAAGGCCG
TCTGAAAGACGGAAAAGCCGAAGACATCCGTGCCGCCGCCGCCAAACTCGGCAGCATCAC
CGACAATTTCGCCGCCAAACGCATGAACCGCAACATCCAACGCGCGCTGACAGGCCAGAG
TGTCGATAATATTTGATACTTAAACGGTTTCAGACGGCATAGAAATAATCCGATGCCGTC
TGAAGGCTCGAAAACACTTGAAAAACATCGATATGGAAAAGTCAGGCATTGTCTATCTGA
TGAAAACCGTCATCAAGGGCGTGTATAAAATCGGCATTTCGGATGTAAGCAATTTTGAAG
GCAGAATGCGCCATTTGGAAAACAACGGTTATGCGAACGTTGCCGGATTGGAACGCATCC
TCGCCGTCAAAACCGACAATTACAAAGAAAAAGAAAACCTGCTCCATGAAATTTTCAGCA
AAAGCAGGATAGGCGATACCGAATTGTTCGCCGTGGACGAAAACCTTGTGAAACGTTTGT
TTTTATCGCTTCGCGGCGAAATCGTGTTCCCGAAAAACGAAACGGCGGAATCGGAATTTG
AAAAAAGCGTCCACGAACGCAGGCAGGAAGGGAATGCCGGGTCAGGCCGCAAACAACTGC
TTGATTTGGTACGGCGCGGACACCGGGAATACCCTTACGCGCTGCCCCGGCTTTTGGCGG
GCGCGGCATTCTACAAGCCGAAAAAATCGAAAATCCGCCTTTTTAAAGAAGCATATTTCG
GCAAAAGCGGCACGAGGCTGACCGACGAAATTGCAGACGGCATCCATATTTACACCTGTT
TTTCGCGGGCGGATTTGGAAAAAGCCTATTCCGAATATTTGGAACTTTTCAAATCCGAAT
CGGATGCCGAAGGCAGAAAGCCGCAGTAAGGTGCAAACAGATACCGTACACGTTGAGGAG
CAGATATGATGGGCGATTCCGTCATTTATTATGTAGAACAGGCAGACGAACCGGTAAACC
GTGCCGACGAACGCGCCCGTAAAACATTCAAATATTTTTGGCGCGAGCTTTTTTGGGAAC
GCCGCCGCATTATTTCCGCCTTGGATTTTGCCATGGTCAAAGTCCCTTTTTTCCAAGACG
GCGAAGACGGCGAAATTTGCGAACATATGTGGATCGACGATATTTATTTTGACGGTCTTT
ATATTTACGGTGTGCTGAACAATGAACCCGGCGAACTGACCAATGTCGAACAAGGCGAAA
GCGTTTGCGTTCCGGTTGACGACATCAGCGACTGGATGTTCGTGTGCAACGGCATCCCCT
ACGGCGGCTTTACCATACAGGCAATGCGCGGGCAGATGACGGAAGAGGAGCGCACCGAAC
ACGATGCCGCATGGGGAATCGATTTCGGCGATCCCGGGCAGATATTGCTGGTGTATGAAG
AAAAAGAACATCCCGAAAATCTGGAAGAGCATCCGATGTGCCGGAACTGTATTGACGATT
TTCGGCAACAGTTGTCCCAAAACTCGGATTATCTGCGGGAACAGGACGAAGACGGCTATA
CGCCGCTTCATCATGAAGCCATCGCAGGAAATGCACTTATGGTTCAAGCCATGCTTGAAT
ACGGCGCAAATCCTGCCTCAACGACATCGGAAGGCTATACCGCCCTCGATTTTGCCTGCC
TGACGGGCTGGCAAAATGTTGCCGACCTGCTCGAACCGCGACATTAGGCAGACAGTTTTC
CGAAAACGAACACAAACACTTTTTACAGAAAGACAATAAAAATGCCCAAAATCACCGTAC
TTCCACACACGACATTATGCCCCGAGGGTGCAGTCATCGATAACGCACCCGAAGGTAAAA
CCGTCCTTGACGTGCTGCTCGACCATGATATCGAAGTCGATCACGCCTGCGAAAAATCCT
GCGCCTGCACAACCTGCCACGTGATTATCCGCAAAGGTTTCGACAGCCTAGAAGAGCCGA
CCGAATTGGAAGAAGACCTGCTCGATCAGGCTTGGGGTTTGGAAGCCGATTCGCGCCTGA
GTTGTCAGGCGGTTGTCGCCGGCGAGGATTTGATTGTGGAAATCCCCAAATACACCATCA
ACCACGCGCGCGAAGAACACTGAAAACAGGCCGTCTGAAGCCGGCACGCTTCAGACGGCA
TTGTTGCGCGGATAAGGCGCAATCGCCCGAAAACAGGCGTTCGTACAGGCGGAACTTTCG
ATTCTATAGTGAATTAACAAAAATCAGGACAAGGCGGCGAGCCGCAGACAGTACAGATAG
TACGGCAAGGCGAGGTAACGCTGTACCGGTTTAAATTTAATTCACTATATATTGATTTTT
ATCGGTTTTCTGACGGAATAATCCAGTGCGGCATCCGAGGCGGATTACTCGGACGCGATG
CACCGGTATTTATCGGTTTTGCAGCCGGAAAAACCGCCGGCGGGTTATAGTGGATTAAAT
TTAAACCAGTACAGCGTTGCCTCGCCTTGCCGTACTATCTGTACTGTCTGCGGCTCGCCG
CCTTGTCCTGATTTTTGTTAATCCACTATACTTTTAGGGCGACGGTCGGGCAGTATGCCG
GATAGCGTTCCACTCTCGCTTCTATATTGATTTTGATTGGTTTTCTGACGGAATGACCCG
ATGCGGCATCCGGGACGGCTTGTGTTTTTTCCTGCCCGCCTGCCGGATTTTCCCATCCTT
GCGTGAAACCGAAAGAGACGGCGGCGCGGCGGACAAGCTCGAGATAGCGTCCTTCAAGCT
CCGGACAGGCGGCGGACACGCTTTCTACCGTAACCGTGAAACCGCCGCCCGACCCGGCAA
GGCGTTCCGCAATTTGCGTGTAGATGAGCCAACGTTGGGCGCGCAACATTGCCGAATCGC
AACCGGCAGCCGCTTTATCCAAGGCAATCAGGTCGCGCCGGTTTTGGTGGTGAAGGCAAA
CCGTTACTCTGGAGAGGATATGTTGCCCGTCCAATATTTGATACAGTTTGCGTATCAGCA
GAATCAGGCGGTCAAACTCCTCCATCTCCGACAGGGAATCAGGATGGTCGGAAGCGGTAT
AAACCAGTTTGGACAATTTTGCGGCAAACATATTGTTCATCAATCTTCCTTGTCGGTTGA
AACCCCGCTCTTCGGGGCGGTAGAATCAGATTTGTTTGGGAGGGACAACTCCTCCCAGAT
CAGGACGACACATAGGCTGGTGCTTGATGTGTTGTCCGGCGAGTTGAAACATTCAGCAAT
CCTCAAGGGGCGGCAGTTTTGCCGAAACATATTCTACACGGCTTCAATGCCGGACGATAA
AAGGAAATTCATATGAAATGGACCGACACCCAGCGCATCGCCGAAGAACTCTATGACCTG
CACGGCGAAACCATCGATCCCAGAACCGTGCGCTTTACCCAACTGCGCGACCTGATTATG
GCATTGCCCGAATTTGACGACGACCCCGCCCGTTGCGGCGAACGCATCCTCGAAGCCGTG
CAGCAGGCATGGATAGACGAGGCGGAATAAGTTTCGGGAATGCCGTCTGAAATGCGGCGG
TACGCGGTTCGTGCTTCTGTTTGCAGCGGGAATGGTTTTACCAGTCTCCTTTTTTCAGCC
TGTCCAGTTGGCGGCGGTCGCGCTTGGTCGGTCTGCCGTCGGGATAGGCGGAAGTGATGC

## Appendix A

GGCTGAATTGGTCGAGCTGTTTGCGCTCTTCCCTCAATGTTGCCGTTTTCGCGTCCTCTT
CATACAGAAGCCGCGCCTCGGATGCCGGGCGGCGTTGGTGGTTCAAACCTTTAACCTTGA
TTTTATAGGGAAGGGAATTGAGCGTCAGGTCGATAATATCGCCGATGTCTATGGTTTTAC
TGTTTTTGACCTTCGAGCCGTTTACTTGAACCCTACCCAGTTCGATGTGCTTTTGCGCAA
GGGAACGGGTCTTGAAAAAACGTGCCGCCCAAAGCCATTTGTCCAGCCGCATGGCGGAAG
AATCGTGCTTGTCTTTCATACGATTTTGTTTGAAATAATTGAATTTGTTTCGAGTTTAGC
ATAAGATACGCCGCCTTATAACTAGTATATATGCACTAATCCACTGTTTTCCATGCTGTC
CGAACACAAAAAGAGGGTATGGAAAAGCCGTTTTGGACAATAAATTAACTGCGGAATATG
CACAAATAGCGTATGATAGCGGCAGAATCTGTTGATGAGAGCTTCATTCTATGAAACCTG
TTTTTTTGGATTTTGAACAACCCATAGCCGAACTGACCAACAAAATCGATGAGCTGCGTT
TCGTCCAAGACGAGTCTGCCGTCGATATTTCGGACGAAATACACCGTTTGCAGAAAAAAA
GCAACGACCTGACCAAATCGATTTTCAGCAAACTCACACCCGCCCAAATTTCACAGGTTT
CCCGGCATCCGCAGCGTCCCTATACTTTGGATTACATTGAGGCACTGTTTACCGATTTTG
AAGAACTGCACGGCGACCGCCACTTTGCCGACGATTATGCGATTGTCGGCGGATTGGCGC
GTTTCAACGGACAAAGCGTGATGGTCGTCGGGCATCAGAAAGGGCGCGACACCAAAGAAA
AAATCCGCCGCAACTTCGGTATGCCCCGTCCTGAAGGCTACCGCAAAGCCCTGCGCCTGA
TGAAGACGGCAGAAAAATTCGGCTTGCCCGTAATGACCTTTATCGATACGCCGGGCGCGT
ATCCCGGCATCGGCGCGGAAGAACGCGGGCAGTCGGAAGCCATCGGCAAAAACCTGTACG
AACTGACGCGCCTGCGCGTTCCTGTTTTGTGTACCGTCATCGGCGAAGGCGGTTCAGGCG
GTGCGTTGGCGGTCGCCCTAGGCGATTACGTCAATATGCTGCAATACTCGACCTATTCTG
TTATCTCCCCCGAAGGCTGCGCGTCTATTTTGTGGAAAACCGCCGAAAAGGCGGCGGATG
CGGCTCAGGCTTTGGGCATTACTGCTGACCGCCTGCAAAAGCTgGACTTGGTCGATACCG
TCATCAAAGAACCATTGGGCGGCGCGCATCGGGATTTCGGGCAAACCATGAAAAACGTAA
AAGCCGTTTTGGAAAAACAACTGCACGAAGCGCAAAGCATCCCGCTTGCCGATTTGCTTT
CGCGCCGTTTCGACCGCATTATGGCTTACGGCAAATTTTCGGAACAATAATTCAGGTAGA
ACAAGCAGCAAGCAGTTTGTCTGAAACTGCTTGCTTTTTCTTTATCGGGACGGAACCGTG
CTGACTTTAGATGCGTTTGAGCAATGCTTGAAGGATTGTTTTCCTCAAGGTCTGAATGGA
AAAAAAACAGCGGTGGCATTAAGCGGCGGCTTGGATTCCGTCGTTTTGCTGCATCTGCTT
GTCCGCGCCGGAAAAAAGGGCGGTTTTATTCCGGATGCATTGCATATCCATCACGGCTTG
AGTCCCCGTGCCGACGATTGGGCAGATTTCTGCCAAAACTATTGCGATATGCTCGGGGTG
GGGCTGGAAACGGTTAAGGTCTGCGTGGAAAAAAACGGTTTGGGCATCGAGGCGGCGGCA
AGGCAAAAGCGTTATGCCGCGTTTGCCGAAAAAGGCTTTGACGTTTTGGCGTTGGCGCAC
CACAGGGACGATCAAATCGAAACCTTTATGCTGGCGGTCGCGCGCGGCGGCGGTTTGCGC
GCTTTGGCGGCTATGCCCGCCGTCCGCCCTTTTGGGGAAAAAGGCATCATCTGGCGGCCC
TTGCTGCCTTTTTCACGCCAAGATATATGGGATTATGCCCAAAAACACGGTTTGCCGAAT
ATCGAGGATGAAAGCAATACCGATACGGCTTATTTGCGAAACCGCTTCCGGCACCGTATT
TTGCCCGAACTTTCGGCGCAGATTCCCCATTTCGGGCGGCATGTGCTGAACAATGTCCGC
GCTTTGCAGGAAGATTTGGCTTTGTTGGACGAGGTCGTCGTTCAGGACTGCCGTTGGGTT
TGCGGGGCCGGTTATTTCGATACGGCGCGGTGGCTGACGTTTTCCCCGCGCCGGAAAACC
CATATTTTGCGGCATTTTCTGAAGGAAAACGGCATTCCCGTGCCGAATCAGAATGCCCTT
GCCGACATTGCCCGGGTTTTGACGGAGGCAAAAACCGGACGTTGGAACTTGCAAGGCTTT
GAATTGCATCATTATGCAGGCAGGCTGTTTGTGTTCCGACTGGAAAAAACGGATAAACTG
CGGTTTTTGAAAGACAGGCAGATAAGCGGAAATTTAAGGGAAATATTGACGGGGCAGGGA
TTTGTGTTGAAGCGGCATCCGTTTGGGCTTCCTGAGCATCTTTTGGAGCAGGACGGAATT
TTGAGGACGGTAGCGGCATCGGATACGTTGGCCATGGGCGGCATCCATAAGGATGTGAAA
AAAATCCTTCAGGGGAAACGGGTTTTGCCTGTCCTGCGCCCAATTTGGCCGCTTGTTGCC
GACAGCGGAAACCGTCCATTGGCGTTGGCAAACTGTTGTGCGGATTTCCAATACTCGGTT
TCAGACGGCATTTTGCCCGTCCATCCTGACTTTCCCATTTTATTTTGATAATATCGCAAA
CAGATTTCGGGGGCGTTCAGTCGGGTATTGTCCGGTTGCATATTTCTAAAAGGCTTGTGA
AGTGAAACACATCAGTTCGACCAATAATGAACACATCAGACACCTGCACCGCCTGTTGTC
GCAAGGAAAGTTCAGACGGCAATACGCCCAAACCGTTTTGGAGGGCGTGCACCTGCTTCA
GGTTTTCCTGCAATCCGGCGGGATGCCGGTCGGGGTATATATTCCCGAAGCGAAAATGCC
GTCTGAAGAAGTCCGTAAATTGACGGCGGTTTTGCCGGAAGACGGGTTTTTTTCCGTTTC
AGACGGCATATTGAAGAAAATCAGCAGCCTGACTTGTGCGGATGATGTGCTTGCGCTGAT
TGATATTCCAGATGCGGGTGCTTTGCCGGCCGGCGGCGATTGCGTGGTTTTGGACGGCGT
GCAAGACCCGGGCAATGTCGGCACGGTGTTGCGAAGCGCGGCGGCGGCGGGAATCGGCGC
GGTCATTTTGGGCAAAGGTTGTGCGGACGCGTGGTCGCCCAAAGTGCTGCGAGCCGGAAT
GGGCGCGCATTTCTTGTCGGAGATTTATCCGCAGGCGGATTTGGAAATATGGTTGGTGCG
CTATAAAGGCCGTGTGTTTGCCACCGCCTTGCGCGAGGAAAAGCAGGCGGTTTTGTACGG
CGAAGATTTGTGCGAACCGACAGCCTGGGTGTTTGGCAACGAAGGCGCGGGGGTCGGTAA
AGCAGTTTTAGATAGGGCGGACAAGTGTGTCAGGATACCGATGCACGATGCAACCGAGTC
TTTAAATGTCGCGATGGCGGCGACAATCTGCCTGTTTGAACAAATGCGCCAACGGGCGGC
GTATTGAGGAAGAGAAATGCCGTCTGAAAAAATCTATTACGGCGTATTGATTTTCTTATG
TATCGCTTCTATGCTGCTGTCGCCGTTTTTTTATGCGGGTGCTTTGAAGCCCAAGAAGGC
GGCATTGCGGAAGGACGGGCAGTGGAAACTCATCTGATTGTCCAATGCCGTGGCGGCGGC
GGTTTTGGCTTGGGTGTGGTGGAAATGGTTTTGACAGATATTGCTCAAAATCGTGCTAAT
GGAATCCGAACAAATAAAGAATTTGGTAAAAAATTTGTTAAATCAACGAATTAAAGTTTT
GTGGAAAACAAAACAGCTCTAAGCAAATAGGGCGTTTGTCGGTAAATACGGAAGAGTTGC
GGCATTATCGGGCATCTTTAACAAGTAGTGCCGTCTTGACAGGCAATCGGTTTTTATGGG
CAGCTTGCAAAATCGCGGATATAAAATTGCGAATCGGTTAAAGTGTGGGGACGCTATGAA
AAATTGCGAATTTTTTATGACCCGACAAGGGCAATCTATGATAGCGGTGCAGATTACTTA
ACTAGGGAAAAACATAGATTAGTCGTAATTGCAAATAGTGCTTGGGGGCTATTGCTTAAT
TTATCTTGTTATTATGACGAGGTTTTGGAAAAGCGGAAAATACCGTTCGGCAAACAGGAA
ATTGATGACGATATGGACAAAGTGTCCGCCCTTAAGCGGAAGTTTAAAGATATTTCTGAA
ATCAAAGTAGGGGATGGTTGGGAATACCCGTTCAATTATGAGCAGGGAATGAAAGAATTA

## Appendix A

GATGAAGTGCTATTGAAATACATTCCCTTTTTTGAAGAAGAACGATAAAGGAGGTTGATA
TGCGCGTATCTAAAATAATTGGAAGTATGTTGCTTGTTACAGCGGTTCAGACCGTATTTT
CGGCAAATGTTTACGCGTGCCGCCATAATGGTAAAACCAGTTACAGCCAAACTCCGGGAA
AACATTGTACCAACGCGGGTTTGGGGCGGGACCGGGTGTACAGTTCGGTTAGACCGGCAG
TAAAAGACAGGGCGGAAGACGCAGGGGTCGGCGATTATTCGGACACGGTGAGGGACGAAC
ACGTCCAAAATCCGAAAGGAAATGCACAGAAAGACGGTTCGGCTGCCGGCATCAAGCCGC
ACTGATTGAAGCCGAATCAGCCCTTGCGCTGTCGGACGGCAAAATTTGAACGATTGGGGA
ACTTTGATGAAACACATCCATATTATCGGTATCGGCGGCACGTTTATGGGCGGGCTTGCC
GCCATTGCCAAAGAAGCGGGGTTTGAAGTCAGCGGTTGCGACGCGAAGATGTATCCGCCG
ATGAGCACCCAGCTCGAAGCCTTGGGTATAGACGTGTATGAAGGCTTCGATGCCGCTCAG
TTGGACGAATTTAAAGCCGACGTTTACGTTATCGGCAATGTCGCCAAGCGCGGGATGGAT
GTGGTTGAAGCGATTTTGAACCTCGGCCTGCCTTATATTTCCGGCCCGCAATGGCTGTCG
GAAAACGTGCTGCACCATCATTGGGTACTCGGTGTGGCGGGGACGCACGGCAAAACGACC
ACCGCCTCCATGCTCGCATGGGTCTTGGAATATGCCGGCCTCGCGCCGGGCTTCCTTATT
GGCGGCGTACCGGAAAATTTCGGCGTTTCCGCCCGCCTGCCGCAAACGCCGCGCCAAGAC
CCGAACAGCCAATCGCCGTTTTTCGTCATCGAAGCCGACGAATACGACACCGCCTTTTTC
GACAAACGTTCTAAATTCGTGCATTACCGTCCGCGTACCGCCGTGTTGAACAATCTGGAA
TTCGACCACGCCGACATCTTTGCCGACTTGGGCGCGATACAGACCCAGTTCCACTACCTC
GTGCGTACCGTGCCGTCTGAAGGCTTAATCGTCTGCAACGGACGGCAGCAAAGCCTGCAA
GATACTTTGGACAAAGGCTGCTGGACGCCGGTGGAAAAATTCGGCACGGAACACGGCTGG
CAGGCCGGCGAAGCCAATGCCGACGGCTCGTTCGACGTGTTGCTCGACGGCAAAACCGCC
GGACGCGTCAAATGGGATTTGATGGGCAGGCACAACCGCATGAACGCGCTCGCCGTCATT
GCCGCCGCGCGTCATGTCGGTGTCGATATTCAGACCGCCTGCGAAGCCTTGGGCGCGTTT
AAAAACGTCAAACGCCGGATGGAAATCAAAGGCACGGCAAACGGCATCACCGTTTACGAC
GACTTCGCCCACCACCCGACCGCCATCGAAACCACGATTCAAGGTTTGCGCCAACGCGTC
GGCGGCGCGCGCATCCTCGCCGTCCTCGAACCGCGTTCCAACACGATGAAGCTGGGCACG
ATGAAGTCCGCCCTGCCTGTAAGCCTCAAAGAAGCCGACCAAGTGTTCTGCTACGCCGGC
GGCGTGGACTGGGACGTCGCCGAAGCCCTCGCGCCTTTGGGCGGCAGGCTGAACGTCGGC
AAAGACTTCGATGCCTTCGTTGCCGAAATCGTGAAAAACGCCGAAGTAGGCGACCATATT
TTGGTGATGAGCAACGGCGGTTTCGGCGGAATACACGGAAAGCTGCTGGAAGCTTTGAGA
TAGCCCGGGCGATGCCGTCTGAAAGCCCTTCAGACGGCATCGCCCGGCTGCGCGGCACAA
AGGCGGAAAAACCGTTTGCCCCGTATTTTCAAACGCGTTACACTTGCCGCCGCTGTTTTC
AGCCATTTGATTACCCGCAACCGCCGTCATTGCGCCGGCGGTTTGCCTGTCAGCGTCATT
GCGCCGCTGTAAATACGAAAGAACACATTATGACCGTATCCCCCGTCGCCTTGCGCCGTA
AGACCGAGTGCAAGCCTCATCCCACCGCGCGCTATTGGAAAAAATGCGATGTCGAAGCCC
TGTTCGGACTTCCCTTCCTCGACCTCATTTACCAAGCCGCCGAAATCCACCGCCAAAATT
TCAACCCGCGCGAAATCCAGCTTTCCACGCTGTTGTCCATCAAAACCGGCGGTTGTCCCG
AAGACTGCGCCTATTGTCCGCAATCGGCGCACCACAATACCAATCTGGGCAAAGAGCAGA
TGATGGATGTGGATGAAATCGTCGAAAAAGCCAAAATCGCCAAATCGCGCGGCGCAAGCC
GGTTTTGTATGGGCGCGGCGTGGCGCGGCCCTAAACCCAAAGACGTGGAGACGGTTTCCG
CAATCATCAAAGCCGTCAAGGGCTTGGGTATGGAAACCTGCGGCACGTTCGGTATGCTCG
AAGAAGGTATGGCGGAAGACTTGAAAGAGGCGGGCTTGGATTATTACAACCACAACCTCG
ACACCGACCCCGACCGCTACAACGACATCATCCACACCCGCCAACACGAAGACCGAATGG
ACACCTTGGGCAAAGTCCGCAACGCCGGTTTGAAAGTCTGCTGCGGCGGCATCGTCGGGA
TGAACGAAACCCGCGCCGAACGTGCCGGGCTGATTGCCAGTCTCGCCAATCTCGACCCGC
AGCCCGAAAGCGTGCCGATTAACCGGTTGGTCAAAGTGGAAGGCACGCCGCTTGCCGATG
CCGAAGATTTGGACTGGACGGAATTTGTCCGCACCATCGCCGTGGCGCGGATTACGATGC
CGCAAAGTTATGTCCGGCTGTCGGCAGGGCGCAGCAATATGCCTGAAGCAATGCAGGCGA
~~TGTGCTTTATGGCGGGCGCGAACTCGATTTTTTACGGCGACAAGCTGCTGACGAGGGGA~~
ATCCTGATGAGGACGGCGACAGAATCCTGATGGAAAAGCTCAACCTGTATCCCTTGCAGT
TTGAACCGGAAGGCGAGGTCGCCGAAGTGGAAAAAGCCTCTGGGATTAAAGTGGATTATT
GACGATTGAAAAATGCCGTCTGAAACCCGGAAAAAGGCTTTCAGACGGCATTTGTCCGGA
CGGCATTTCCAATATCTTTTTACCGGCGCGTGATGCTGCCGTCGGGCGAGACATCCAGCC
CGTTCCCCTTGGGGAAATCGCTGCGGTTCAGATAAATAATCCGCCCGATAACGGTTTTCT
CGGGGTCGATTTTGGGGATTTTATCGCCGACTTGAGTGATGGGGATAATGTTGCCGGAAA
CGAACCGCCCCTGTTTGTCGGTGATAATTTTAAAAATGGGGGCGATGCCGCTGATGCCGG
AGGTGTTGATTGCGCCGTAGGTGGCAAAGTTGCCGCCGCTGTAGGAGATGAAGCGGTCGC
GGTAAAGTTCGACGGCGCGAGTAACGTGCGGCCCCTGCCCGAATACGACATCCGCGCCGG
AATCGACGGCAAGCCGCGCAAACTCAACGACGTTGCCCCTGTTTTCCCCATAGAAGATTT
CGGTATCGAACGGCAGGTGTTCCGCCTGTTTCCCTTCCGCGCCGCCGTGGAACATCACAA
TGACGATGTCGGCTTTCTGTTTGGTTTTGGTAATCCGTTTTCTAACTTTGGCATAATCGT
TCAGTTTGACGGCGGCAAGGTTGGGGGCGAAGGAGACGAAGCCGGATCTTACGCCGTTTT
TCTTCAGGATGGCGGTTTCAAACCTGTTTTCGATGCCCGAATATTTGATGTTCAATTCGT
CAAGGTTCGCCCTTTATGCCGTTTCCGCACCGCAAACCGCCGGGGTCAAGCCCTCGGCGC
ATCCTGCCGGAACGGAATCCCCGTGCTGCCGATTGACGGTTCAAACCCCGCGCCCGTTTC
AAATACCGGCGATGTGGACGGACAGGATGCGCCTGACGAAAAGACAGCCGATACCGTTTC
CATTATCGGCGTGGGCGACATTATGCTCGGCAGCAATTATCCGGTCGATTACCTGCCCGA
TACCAATATTCTGAAAAACGTCGAATCTGCCTTGCAAGACGCGGACATTACCGTCGGCAA
CCTCGAAGGCACGCTGTTTGACGAAGGCGGTACGCCGAAAAAATGTGCAAACCCCCAAAA
TATGCTATGCATTCCGAACGCCCTCCGCATACGGGCAATACCTTGCCGACGCGGGATTCG
ACTACCTCAGCTTCGCCAACAACCACAGCAACGACTTCGGCGCGCAAGGCATCACGGCAA
CGGCGGCGAGCGGCAGCTCTTTTACATACTCGATCGCGCTAAAGCCGCTGCCGATAACGA
GGCCAAAATTGCGGAAAATACCGCCATCGCCCAGATAAATTTGTCCATCATCAGACCTTT
ACTGTTCAGACGAGACAGCATTTGCCGCACGTTTTGGGGCTTATCTTTCGATTTGCGCTA
CGTCGCGCACCGCGCCTTTGTCGGCGGAAGTCGCCATCGCGCCGTAAGCTCTTAATGCGG

## Appendix A

CGGAGACGTAGCGGTCGCGGTTTTTAGGCTTCCATGCTTTGCTGCCGCGCGCTTCCATTT
CGGCACGGCGTGCGGCAAGCTCTTCATCGGAAATGGCAAGGTGGATGCTGCGGTTGGGGA
TGTCGATTTCGACGGTATCGCCTTCGTGTACCAAACCGATCGCGCCACCTTCCGCCGCTT
CGGGCGAGGCGTGTCCGATGGACAAACCTGATGTGCCGCCGGAGAAGCGTCCGTCGGTTA
AGAGAGCGCAGGCTTTGCCGAGGCCTTTAGATTTCAGGTAGGAAGTCGGATACAGCATTT
CCTGCATGCCCGGGCCGCCTTTCGGGCCTTCGTAGCGGATGATGACGATGTCGCCAGCGA
CGATTTGGTTGCCCAAAATGCCTTCTACTGCGTCTTCTTGGCTTTCAAACACGCGGGCGC
GGCCGGTGAATTTGAGGATGCTCTCGTCCACGCCTGCGGTTTTTACCACGCAGCCGCGCT
CGGCGATGTTGCCGAACAAGACCGCCAAACCGCCGTCTTGCGAGTAGGCGTGTGCCACGT
CGCGGATACAGCCTTTTTCGCGGTCGAGGTCGAGGGTTTTCCACATACGGTTTTGCGAGA
ACGCTTGGGTGGTGCGTACGCCGCCCGGCGCGGCTTTGAAGCGTTCGATGGCACGGGTGT
TTTCGGGATTGGTCACGTCCCATTGTTCAATCGCGTCTTTCAGCGTCGGCGCGTGGATGG
TGTGCACGTCGGTGTGCAGTTTGCCCGCTTTGTCCAGTTCTTTCAGGATGGCGAAGATAC
CGCCGGCGCGATGCACGTCTTCCATATAGTAGTCGTGGTTGTTGGGTGCGGTTTTGCAGA
TGCAGGGCACGACGCGGCTTAAGCGGTCGATGTCTGCCATTTTGAAATCGACACCGGCTT
CGTTGGCAACGGCCAACAGGTGCAAAATGGTATTGGTGCTGCCGCCCATCGCAATATCCA
TCGTCATAGCGTTTTCAAACGCTTTTTTGGTGGCAATGCTGCGCGGTAACACGGTTTCAT
CGTTTTGCTCGTAATAGCGTTTGGTGATTTCGACAATCATACGGCCGGCTTCGAGGAACA
ATTCTTTGCGGCCGGCGTGGGTCGCCAAATACGAACCGTTGCCGGGCAGGGAAAGGCCGA
GTGCTTCGGTCAGGCAGTTCATCGAGTTTGCCGTAAACATACCCGAACACGAGCCGCAGG
TCGGGCAGGCGTTTTGTTCGACTTCCTCGACTTGCCGGTTGCTGACATTGTCGTCCGCCG
ATTCAATCATCGCGTCAATCAAGTCCAAACGGCGTTCGGGCTGGATGTTTGCCACGCCGA
TAACCTTGCCCGCTTCCATCGGGCCGCCGGAGACGAAGATGGTGGGGATGTTCAGGCGCA
TCGCGGCAATCAGCATGCCCGGGGTGATTTTGTCGCAGTTGGAAATGCACACCAGCGCGT
CGGCGCAGTGGGCGTTGACCATATATTCGATAGAGTCGGCAATCAAATCGCGGCTGGGCA
GGGAGTACAGCATGCCGCTGTGTCCCATAGCGATGCCGTCGTCGATGGCGATGGTGTTGA
ATTCTTTGGCGATTGCGCCGGCTTTTTCGATTTCGCGGGCAACCAGCTGGCCCATATTGT
GCAGGTGGACATGGCCGGGCACGAATTGGGTGAAGGAGTTGGCAACGGCGATGATGGGCT
TGCCGAAGTCGGTTTCCATCACGCCGGTGGCGCGCCACAATGCACGTGCGCCCGCCATAT
TGCGGCCGTGGGTGGAGGTTTTGGAGCGGTATTCAGGCATAGTGTGTTTCCTTGTGCCTA
TACCGTCTGAAAGACAGGGCTGTTTCAGACGGTATCGGGTACGGTTTTTTAGAGTGGGAA
AAGAGGGTATTTTATACCAAGTATCGGAATTTTGCGGGATTGAAACGGCGTGCGGCAAAA
AAGAAAATCCCCGCAGGAATGCGGGGACGGGTTCAGGCGCGGGCAATCGCGACGGCTTTG
GATGCGTCCCAAAAATCAACGGGTGCATTTAATACGGGTTTGACGATGCCCGTCCGTATG
GCGAAATCGCCGAAACCTTCGCCGATATTGCGTTCTGCCGCCCATTTGCCGATCAGGTCG
TCCAATTCGGCAAGGATTTCGGGCAGGGTGATGTTTTCTTTGTAAAGACGGGGGATGCGT
ACGCCTTCACGGTCGCCGCCGATATGGAGGTTGTAGCGTCCGACGGCTTTGCCGACCAGT
CCGATTTCCGCCAACATCGCCCGTCCGCAGCCGTTCGGGCAGCCGGTAATGCGGGTAACG
ATGTAGTCGTCCGACGTGCCGTGTTTCGCCATAATCTTATCCAGCTCGCCGATGAAGTCC
GGCAGCACGCGTTCGGCTTCCGCCATTGCCAGCGGGCAGGTCGGAAAGGAAACGCAGGAC
ATCGCATTTTCACGCAGCTTGCTGACATCGTTGCGGATTAATCCGTATGTTCGGGCAAAT
TCTTCGATTTTTGCTTTGTCTGCTTCGGCGACATTTGCCACGATGAGGTTTTGGTTGGCG
GTGATGCGGAAATCGCCTTTGTGGATTTTGGCGATTTCCAACACGCCGGTCAGAAGCTGT
TTCCCGCCTTCGTCAACCAAACGCCCGCTTTCGATGAAAAGGGTTAAATGCCAGTTGCCG
TCTATGCCTTTCACCCAGCCGATGCGGTCGCCGCGCCCGGTAAATTTGAACGGGCGTACG
GGTTCGAACGGCATACCCATACGGCGTTCAACTTCCGCGCGGAAGTTGTCCAAGCCCATA
TTTTGAATGGTGTAGCGGGTGCGGGCGTTTTTGCGGTCGCTGCGGTTGCCGAAGTCGCGC
TGCGTGGTTACCACCGCTTCGGCGGCCTTCAGCGCGTGTTCCGGAGGCACGAAACCCAGT
TCCAGTGAAATGTTCGGATAGGTTTTGGTGTTGCCGTGTTCCATCGAAAGCCCGCCGCCT
GCCAAAACATTGAAGCCGGCAAGCTGTCCGTTACCGTCTGAAACGGCGACGAAATCCAAA
TCGTTGCCGTAGCAGTCCACATCGTTCAAGGGCGGGATGACGACTGCGGTTTTGAATTTT
CGCGGCAGATAGGTTTTGCCCAAAATCGGCTCGTCTTCTTGAAGGAAGTCGTCGGAACTT
TGAACTTTTTTGCCGTCCACCCACACATCCAGATAACCGCGCGTGCGCGGCAGCAGGTGT
TCGGAAATCTTTTTCGCGTATTCGTAAGCCTGCCGGTGCAGTTCGGACTCGATCGGGTTG
GACGTGCAAAGCACGTTGCGGTTCATATCCGCCGCCGTGGCGATGGAATCCAAACCCAGT
TTGTGCAAGAGGCGGTGCATCGTCTGCAACTTGGCTTTCGGCACGCCGTGAAATTGGAAG
GTTTGCCGGTTGGTCAGCCGGATGGAGCGGTAATGACTGTTTTCCCGGGCAAATTTGTCC
AGTTCTATCCATTGGGACGGTTTGATGATCCCGCCCGGCAGCCGGCAGCGCAAAAGCATA
AATTTCAAGGGCTCGAGTTTTGCCTCGGCGCGTTCGGCGCGGATGTCGCGGTCGTCCTGC
TCATACATACCGTGGAAGCGGATGAGTTGGAAGTTGTCGCCTTTGAAGCCGCCCGTGAGC
GGGTCTTTCAAATCGTCCAAAATCGTGCCGCGTAAAAAATTGCTTTCGGTTTTCAGACGT
TCGTTGTCGGATAGCGGTTTTTCTTGCCACGCCAAACCTTTTGTCTTGGTCTGTACGGTC
ATTTTGTGTTCCTCCCGATTATATTTAATCAATAAACATCACGCTGATAGCGTTTTTCTT
CGCGCAGCATATCCAAATATTCTTCTGCGCCCTCTTCGTCCAAATGTCCTGCCCCGATAA
TCACATCCAGCAAGGCGGCTTCCACGTCTTTTGCCATTTTTGCCGCATCGCCGCACACAT
AGATATGCGCGCCTTCCTGCAGCCATTGCCAAAGTCCTTCCGCCTGTTCGCGGATTTTGT
CCTGCACATAGATTTTTTCTTCCTGATCGCGGGACCAGGCGAAATCGTACCTGTGCAGGA
AGCCGTCTTTGGCAAACTGCTGCCATTCGGTTTGATAGAGAAAATCACGGGCAAAATGCG
GATTGCCGAAAATCAGCCAGTTTTTGCCTTCCGCATTTTCTGCGGCACGTTGTTGGACGA
AAGCGCGGAACGGTGCGACGCCGGTGCCCGAGCCGATCATCACAATCGGCTTGCGGCTGT
CTTCGGGCAGCCTGAAGCCGTCGTTGCGTTCCACAAACACGCGCACCGTGCCGTCCTCTT
CCAGCCGGTCGGCAAGGAAACCCGATGCGCCGCCCGTTCTGGCGCGGCCTTCGTGTTCAA
AACGAACCACGCCGACAGTTAAATGCACTTCATCGCCCACTTCCGCCTGTGCTGAAGAAA
TCGAATACAAACGGGGTGCAAGCGGACGCAGTAAACGGATGAATTGTTCTGCCGTCAGGC
TTGCCGGGAAGCGGTGCAGCACATCGACAATAGGCGTGTTTTGCACGAAATCCTGCAAAA

## Appendix A

CGGCGTTATCGGCAATGATTTTATCGAGTTCTTCATAATGGGCGAACGCGGCATAGCCTT
TGACGAAAGCCGGAGTGTTTTGCGTGAGTTCGAAATGAGATGAAAGTGCGCGCGCAACCG
GCATCATCTTTCCGCCCGCCTGTATTTCCGTTGCCGGATCGATGCCGAGCAGGTCTAGGA
TTTCCCTGACCAGTGCCGGATCGTTGTCAAACCAAACGCCGAGCGCGTCGCCCGGGAGGT
AGTGCAAATCCGAACCGCTCAAATCGATTTCGATGTGGCGCACGTCTTTATCGGATTGGC
GGGCGGTGATTTTCTGATTGGCCAGCAGGGCGGCGGGAAAGGGGGCTGCCTTGCAGTACC
TGCCATCCGGTGCCGTCTGAAGGCCGGCGGGGGGCGTTGTCTGCGGCGCGGGCGTTGCCC
GGTTTTTTGCGGCTTCTTCTTTTAAGAGTGCGGCGATATTATCTGTCCAGGCGTTTGCGG
AGGCGGTAAAGTCCAAATCCGCATCAACGCGTTCGAGCAGCCGTTTTGCGCCCAATTCTT
CAAAACGCCGGTCGAAATCTTTACCTGCCTGACAGAAATTCGGATAGGAACTGTCGCCCA
AACCCAGTACGGCAAATTGGAGTTTGTCCAATTTCGGGGCTTTTTTGCCGTTCAGCAGTT
TGTGCAGCACGACGGCTTCTTTCGGCGGTTCGCCTTCGCCTTGGGTGGAGGTAACCAGCA
GCAGGCGGCGTTCGCCGGCGATGTTTTTCGCCTTATAGTCTTTCAGTTCGGCGCGACTGA
CTTGGATGCCGGCGGCTTCCAGGCTGTCCGCCGCTTTGTCGGCAACGGATTTCGCATTGC
CGGTTTGCGAGGCGGAAAGGACGGTTACGGAAAAAGGTTCTGCCGCCGGCAATGCCGTCT
GAAGCGCGGGCAGTCCTGCAGATGCCCCGTTTCCTGCTTTTGCCCAAGCGTAGCCGGACA
GCCACGCCCATTGTGCCGCGTCCAGCCCCGACAGGAGCTGCGTGATTTCGGGCGGCAGAG
GCGGTAATGGCGGATTTGTGTTCTGCATATCGTGTTCACTCATAAAATCATACCTGCCGC
AACAGTGCCGTATGTCGCTTCGTCTATCAGGATAAACGAACCGGCGGCGGTGTTTTCCGC
ATAAGGCGTTGCCGTAACGGGTTTTTGAAGGTTGATGCGGACTTTGGCGATGTCGTTCAT
CTTCAAGGATTCCGCGCCGGCCTCTTGTTCCAGCGTGCGGACATCCAAAACGCTTTCAAT
TTCCCCGACTTTTGCCGGCACGGTTTGCGTGCCGTGCTTGAGCAGGTATTTGCGCGCGGT
GTTGAGCGGACGTTCGTCAAACCAGCAAAGCGTGGCTTCCAGATGTTTTTGCGGGGCGAG
CGGGGAATTTTTATCGACAAAAAGGTCGCCGCGCGAAACATCGATGTCGCGGTCCAGCCG
GAGGGTTGCCGCCTCGCCGGCAAAAGCCTGCGCCACTTCCCCTTTCGGCGTGATGATTTC
GGACACTTCGGCGGTCAGCCCGTTCGGTTCGATGCGGACGGTTTGCCCGACGGCGACCGA
ACCGCGTTCGATGCGCCCCTGATAGCCTCGGAAATCATCGGCCTTGTCGGCATCTTGGCG
GACGACCAGTTGCACGGGGAAATAAAAATCGTCGGCGGTGCGGCTGACTTCGTCCGCCCC
CGGCAGGGTTTCCAAAATGGACAATAAGGGTTCGCCTTTATACCAAGGCATATTGCCGCC
GGGGTAAACAATGTTGTCGCCCAAGAGTGCGGACATCGGTACGAAATGCGCGTCTTTCAA
ACCGAGCTGTTCGGCAAGTCGGCGGTATGCCTCCACAATGGCGTTGAATTTGTCTTCGCT
GTAATCCAGCAGGTCCATTTTGTTGACCGCCACCACAATATGCGGGCAGTTGAGTTGGCG
GAGGATGGCGGAATGGCGTTTGGTCTGCGGCAGAAGCTGCAAGGGCTGCGCGCCGAAATC
CAGTTGGGATGCGTCAACCAGCACGACTGCCGCCGAAGCGGTGCTTGCGCCCGTAACCAT
ATTGCGCGTGTATTGTTCGTGCCCCGGCGTGTCGGCGATGATGAATTTCCGTTTCGCCGT
GGAAAAATAGCGGTATGCCACATCGATCGTAATGCCCTGTTCGCGTTCGGCTTCCAGTCC
GTCGGTCAGGATGGAGAAGTCTATGGCTTCTTTCAAACCTTTGCTTTTGCCGGATTCCAA
GGTTTTGATTTGGTCGGACAGCAGGGCTTTGCTGTCGTAGAGCAGTCGTCCGATCAGGGT
GCTTTTGCCGTCATCGACGCTGCCGGCGGTAATGAAGCGGAGCGGGGTTTGGTGTTGTGC
CGTCATATTTTCTTCCTCATATCTGCTTAAAGGGTTTTTGAAATTTAGAAATAGCCTTCT
TTTTTGCGTTTTTCCATTGCCGCCTCGCTTGCCTGATCGTCCAGCCGGGTCGCGCTGCGT
TCGGAAATGTCGGCAACCGCTGTTTCTCTGATAATCTCCGTCGGCGTGGACGCGGTGCTT
TCTACCGGGCAGGTGCAGCTGATGTCGCCGACGGTGCGGAAGCGGACATCAAGGATTTCG
GAGGTTTCAGACGGCATTTTCGGGGTGAGCGGCGTTACAGGGACCAGCAGCCCCCTGCGT
CTGACCACTTCGCGCCTGTGGCTGTAATAAATCGGCGGCAGCTCGAGGTTTTCGCGGGCG
ATGTATTGCCAGATGTCGAGTTCCGTCCAGTTGGAAATCGGGAAGACGCGCATATTTTCG
CCTTTGTGCAGCCTGGTGTTGTACAGCGACCACAGCTCGGGGCGTTGCGCCTTCGGATCC
CATTGTCCGAACTCGTCGCGGAACGAGAAAATCCGTTCTTTGGCGCGGGCTTTTTCTTCG
TCGCGCCGCGCGCCGCCCATAAGCGCGTCGAAGGCGTTTGCCTCGATGGTTTCCAACAAG
GTAACCGCCTGTGCCGCATTGCGCGAATCGGTTTCTTTGCGTAAGACCACCGTGCCTTTG
GCAATGGAGTCTTCCACGCGCCCCACTATCAGGCGGGCATTGAGTTTTGCCGCCTGCGCG
TCGCGGAAGGCAATCACTTCGGGGTAGTTGTGTCCCGTGTCGATATGCACCAGCGGGAAG
GGCAGTTTCACCGGCCGGCTGCCCAGCCGGAAGGCTTTGCAGGCGAGGGCGAGCAGGACC
ACGGAATCTTTGCCGCCGGAAAAGAGCAGGGCGGGGTTTTCGCATTCTGCCGCCACTTCG
CGGATGATGTGGATGGATTCGGATTCCAACCAGTCGAGTTGGGCGTTGTTCGGTTCGGTT
TTCGTCATACCATATTCCTTATTTCTTCTGTCTGATATTTATGAATTATTTGTGCAGCCC
GCATTCTTTGCTGTTTCTGCCTTCCCACCACCACCGCCCGGCGCGGATGTCTTCGCCCGC
CTTGACGGGGCGGGTGCAGGGGTCGCAGCCTATGCTGGGAAATCCTTGCCGGTACAAATC
GTTGTAAGGCACATTGTTGGCGAGGATGTATGCCCACACGTCGTGTTCCGACCAGTCGAA
AATCGGGTTGTATTTGCCGATGCCCCGTCCGGCATCGTATTCGGCAAACGGCAGTTCCGT
GCGTGTGGCGGATTGTTCGCGGCGTTGCCCGGTAAGCCAGGCGTCCGCGCCTGCAATGGC
GCGGTTGAGCGGTTCGGTTTTTCGGATGCGGCAGCATTCGCGGCGCGCTTCAACGCTGTC
GTAAAAGGCAAACCTGCCTTTGCTTTCCACATAACGGTCGGCATCTTCTCGAACCGGCCG
GAAACGCTTTATCCGCAAATGGGGATATGCGCGTCCGAGCCTGTCCAGCAGGTTCAGGGT
TTCCGTGTGGAGCAGCCCCGTATCCAAGGTAAAAATGCCGATATTGAGGTTTTCGCCGGC
GATAAGGTCGGTAATCACCATATCTTCTGCCGCAAGGCTGCTGGCAAACCGTGCATCCCG
GTGGCTGCCGACAATCCGGTGCAGGCGTTGTTTGAGGGTTTCCGTTTTTTCCGCAAGGGC
GGTTTCGCCGCCGGATCCGATATGCGGTATCTGCCACAGGGCGGGTTTGAACAGTGTCGT
TTCCATTTTTCCCGCCTTATGCCGCCCGTTGTCCGGCATTCAGTCCGCCCAATGCGGGAT
ACGTCTGCCCGACCCGGTTTTCTCCTTCGCCGTTTTCACCGAACCAGGCGAGTTTTTCGT
GCAGCCCCACCACTTCGCCTATGACAATCAATGCCGGATTCGGCGCGGTTTCGGCGAGTT
CGGCAAGGTTGGCGAGCGTGCCGGTTGCGGTTTTTTGAGCCGGCAGCGTGCCTTGGCTGA
TAACGGCTGCCGGCGTGTCGGGCGAGCGTCCGTGCTGTTGCAGCCGTTCGGCAATCAGGG
CGGCTTTGAGCGCACCCATATAAATCACCAAGGTCTGGCGGCTGCGGGCGAGGGTCTGCC
ATTCGATGTCGGGCGCATCCGCCTTGCGGTGGCCGGTTACGAAAACCGCACTTTGGGCAT

## Appendix A

AATCGCGGTGCGTGAGCGGGATGCCGGCATAGGCGGTCGCGCCGACGGCGGCGGTAATGC
CGGGGACGACCGAAAACGGAATCTGATGGCGTGCCAAGGTTTCCAATTCTTCGCCGCCGC
GTCCGAACACGAAAGGGTCTCCGCCTTTCAAACGCACCACGCGCCTGCCTTCGCGGGCCA
GCCTGACCATAAGCGCATTGGTGTCCTCTTGCGGGGTGCGCTCGCCCCGGGCGCGCTTGC
CGACAAAAATCCGTTCCGCATCGCGGCGGACGAGGGACAGTATGCCGTCTGAAACCAGCG
CGTCGTAAAGCACCACGTCTGCCTGCTGGATTTCCTGCAGCCCTTTGAGCGTCAGCAGCC
CCGCATCGCCGGGACCCGCGCCGACCAGCGAGACGGAGCCGCCTTGATCATTTTGACGAC
TTTGTTCCAATTGGCCTGCCAATTCCCGTTCGGCAAGGGTGTTTTGCCGGTTTTTGACGA
GGGCGGCGAAACGTCCGTTAAACTGCTTTTCCCAAAAGCGGCGGCGTTCGGTAACGGATT
TCAGTTTGCCCTTGACGGCATCGCGCCACCTTCCTGAAATTTCCGCCATATCGCCCAAAG
ACGGCGGCAGCAGGGCTTCCAGCCTTTCACGCAGCAGTCGGGCGAGGACGGGCGCGCTGC
CGGAGCTGGAAACGGCAATCTGAACCGGGTTGCGGTCGATAACCGACGGGAAGATGAAGC
TGCAATGGTCGCGGTCGTCCACCACGTTGACCGGCTTTTGGCAGCTTTCGGCAAGATGGA
AAACGCGCCGGTTGAGGGCTTGGTCGCTGCTTGCCGCAATGATGAGGAAAACCGTGCGGA
TGTGTTCGGCACGAAATTCTTCGGCAAGCCACAGGATTTTGTTTTCCGCCGCCAACGCGG
AGAGTTCGGCATTCAGGTGTTTTGCGGCAACCCTGACCTCTGCGCCCGCCTTCAGCAGCA
GGCTGATTTTGCGTGCGGCGACCGCGCCGCCGCCTACGACCAATACGGGGCGGCCGGCGA
GGTTGGCGAAAATAGGGAAATAATTCACTGGCTGACTCCTTTGCTGTTTGCCCGCACCTT
GTTTCCGATACGGTGCGTCGCGGCATTTTTGTCGGAATGCGGGTCATTTTAGACAAAAGG
ATTTTCCCCGGTTAAATAATAAAAAGGTATTTGTTAGAAGCTGAAAGCTATATGGGGGCG
GCTGCGGATGCGGCGGTTTTCCGTTTTATAACGGTTTCGGAAGAAAAACGGCCTGAAGCC
GTTTCGGGCATTCAGACCGTTTGCGTGGTGAGGGGATGCCGTCCGAAGGGCGAAAAGGGC
TTCAGACGGCATTGATGTCGGGTTTCAGGACAGGAGCAGGATGGCGGCTGCGGCAAGCGA
GGCAACCGATAATGCGGCGGCAAGCGCGGCTTTGCCTGCAAAGCGGATTGAGGTTTTGCC
TTCGATGTATTTGAAGCCGGTTATCATCGGGAGGATGAGGTTTTTCTTTTTGAATACGCG
GTATGCGGCGACGGCGGCGATGTGGATTGCAGAAAAAACGGCGAGCAGCTTGAAAAAGTT
GAGGTGGATTTTCCGCATAAGGCTGCCCGTATGTTCGGAAACCAAATGGTTGAGGTAGCC
GTTGGTGCTGAAGGTGTTTTCATCGGCGGCAAAAAGCCCGGTGCCGACTTGGAAGGACAC
GGCGGCCAAAAGCGCAACGACCATCAGTGCGCCCAAGGGGTTGTGTCCGGGCTGGATGTG
TTCGGGAATACCGTTTTTCAGATAGCCGCGTATGCCTGCCCAGCCTTGGACGAAACGGGA
AAAACGGGCGGTATCGCTGCCCCAAATGCCCCAGCAGAGGCGAAATACGAGCAGGAAAAG
GACGAACAGCCCGACGCGCGTGTGCCATTGCAGCATATCGCCGCCGGCTTTCGCGCTATA
CCACATAAAGGGCAGGGACGCGGCAAGCAGCCAGTGGAAAAGGCGGGTGGGGAGGTCCCA
GACTTTGGTTTTGTTTTTCATAATCGGTTTCCGGCGGTAGAATCGGTTTGTTTTCGAGCC
TTATTTTAACCGATTGGAGGGGCAATGTTTCCCGTTTTTCATCTTTCAGGCGAGAGCCGC
CGCCAGATGCTTCAGACGGCATTGCGTTTTCCCCATGTTTTCAAAGCCCGTGCGGAAGAT
TCGCACAAAGGGACTTTCGGCACGCTCGCCGTAGTCGGCGGATCGGCAGGGATGAGCGGC
GCGCCCGTATTGGCGGCATCGGCGGCAATGTATCTCGGCTGCGGCAAAGTGTGGGCGGGT
TTCAATCAGGATACGCTACCTTTTGCCGTTATTGCCGGTTTTCCCGAGATTATGCTGGAT
ACGGCGGACAGTTTGGCCAAACGTCAAGATATAAACGCCTGGGTTGTCGGTTGTGGATTG
GGTACAGGTAGGGCGGCGGTCGGAACGCTTGCCGGAATTTTGACGGAACACACGGACAAG
CCCGTCGTTTTGGATGCGGATGCGCTGAACATATTATCAACCGATGCCGAAACCCGAAAT
CTGGCGCGCGGGTGTAAAAACCTGATTTTAACGCCACACCCCGCCGAAGCCGCGCGCCTG
CTTGGAACGACGGTTGCGCAGGTTCAGGCGGATCGGACGGCGGCAGTGAGGAAGATAGGG
GCAATTTTCGGCGCAACCGTGGTTTTAAAGGGGCACAAAACATTGGTTGCCTCACCCGAT
ACGGAAATCTATGTCAACGAAAGCGGCAACGCGGGATTGGCAACGGCGGGCAGTGGCGAC
GTATTGGGCGGCATCATCGGCAGTCTGCTCGCACAGGGCGTGCCGGTTTTTGAAGCCGCC
TGCGCGGGCGCGTGGCTGCACGGCGCGGCGGCGGATGTCATAAAAGAATCGGCAGGCATT
GCGGCAGGGCTGTTGGCAGGGGAAATCGCTCCGGCGGCAAGGTGGCTGCGCAACCGGATA
ACTAAAAGTATGTAAGAAGATATAGTGGATTAACAAAAACCAGTACATCGTTGCCTCGCC
TTAGCTCAAAGAGAACGATTCTCTAAGGTGCTGAAGCACCAAGTGAATCGGTTCCGTACT
ATTTGTACTGTCTGCGGCTTCGTCGCCTTGTCCTGATTTTTGTTAATCCACTATACCGTC
TGAAAGGCAAGGGCTTCAGACGGCATCTTCATTTCCCAAATACTGTCCGGTAAAGCGTGG
TATAATGCGTAGTTATCTCTTACAGTTTTTGAAAAACATTAATTATGAAACAAATCCGCA
ACATCGCCATCATCGCCCACGTCGACCACGGCAAAACCACATTGGTCGACCAACTGCTGC
GCCAATCCGGCACATTCCGCGCCAACCAGCAGGTTGACGAGCGCGTGATGGACAGCAACG
ACCTTGAAAAAGAACGCGGCATCACCATCCTCGCCAAAAACACCGCCATCGATTACGAAG
GCTACCACATCAATATCGTCGACACGCCGGGACACGCCGACTTCGGCGGCGAAGTAGAGC
GCGTTTTGGGGATGGTGGACTGCGTCGTCTTGTTGGTGGACGCGCAGGAAGGCCCGATGC
CGCAAACCCGTTTCGTGACCAAAAAAGCCTTGGCTTTGGGGCTGAAACCGATTGTCGTCA
TCAACAAAATCGACAAGCCGTCCGCTCGTCCGAGCTGGGTTATCGACCAAACTTTCGAGC
TGTTCGACAACTTGGGCGCGACCGACGAGCAGTTGGATTTCCCGATTGTTTACGCTTCAG
GGTTGAGCGGTTTCGCCAAATTGGAAGAAACCGACGAGAGCAACGACATGCGTCCGCTGT
TCGATACTATCTTAAAATATACGCCTGCACCGAGCGGCAGCGCGGACGAAACGCTGCAAC
TGCAAATTTCCCAACTCGACTACGACAACTACACCGGCCGCCTCGGTATCGGTCGTATCT
TGAACGGACGCATCAAACCCGGCCAAACCGTTGCCGTCATGAACCACGATCAGCAAATCG
CCCAAGGCCGCATCAACCAGCTTTTGGGTTTCAAAGGTTTGGAACGCGTGCCGCTTGAAG
AAGCCGAAGCCGGCGACATCGTGATTATTTCCGGTATCGAAGACATCGGTATCGGCGTAA
CCATCACCGACAAAGACAATCCCAAAGGCCTACCGATGTTGAGCGTGGACGAACCGACGC
TGACGATGGACTTTATGGTCAACACCAGCCCGCTGGCGGGTACGGAAGGCAAATTCGTAA
CCAGCCGCCAAATCCGCGACCGCCTGCAAAAAGAATTGCTGACCAACGTCGCCCTGCGCG
TGGAAGATACCGCCGATGCCGACGTGTTCCGCGTATCCGGGCGCGGCGAGCTGCACCTGA
CCATTTTGCTGGAAAACATGCGCCGCGAAGGCTACGAACTCGCCGTCGGCAAACCGCGCG
TCGTGTACCGCGACATCGACGGTCAAAAATGCGAACCGTATGAAAACCTGACCGTGGATG
TACCCGACGACAACCAAGGCGCGGTAATGGAAGAACTCGGCCGCCGCCGTGGCGAACTGA

## Appendix A

CTAATATGGAAAGCGACGGCAACGGACGCACCCGCCTCGAATACCATATTCCAGCGCGCG
GCTTGATCGGTTTCCAAGGCGAATTTATGACCCTGACGCGCGGGGTCGGGCTGATGAGCC
ACGTGTTCGACGATTACGCGCCCGTCAAACCCGATATGCCCGGCCGCCACAACGGCGTGC
TGGTGTCCCAAGAGCAGGGCGAGGCAGTCGCTTACGCCTTGTGGAATCTGGAAGACCGCG
GCCGTATGTTCGTATCGCCCAACGACAAAATCTACGAAGGCATGATTATCGGCATCCACA
GTCGCGACAACGATTTGGTGGTCAACCCGCTCAAAGGCAAAAAACTTACCAACATCCGTG
CCAGCGGTACCGACGAAGCCGTTCGCCTGACCACGCCAATCAAGCTGACGCTGGAAGGTG
CGGTTGAGTTTATCGACGATGACGAACTCGTTGAAATCACGCCGCAATCCATCCGTCTGC
GCAAGCGTTACTTGAGCGAATTGGAACGCCGCCGCCACTTTAAAAAGCTGGATTGATGTT
TACTGGATTAATGTTTAAATGATACGCGATGCCGTCTGAAAAATTTCAGACGGCATTTTT
TATTCGGACGGGCTTTGCGGCTTCTTGAAGCTGTTTCAGACGGCGTTTTTCCTACCCAAT
CAAGAAACTGCCGCCATTTTTCCAGCGGTATATCGCCCCGTCGCGTTTCGGTATCGGGTT
CGGCTTCCCGGCAGCATGTCGAACAATGCCGTTTGAAGGAATACGCCTTTTGAGTCCTTA
CAGGCTGAGGAAGAGGGTAAAACATACCGCAAAACATACCACAAAAAACGGTAACGGATA
GTTGTAAGCGGTTGATGACGATTATAGACAATAACGGGTTTTCCCAATGAAATTATTGTT
TGAATAATAAAAAATCCCAAACCGTAAAAGTTTGGGATTTGTATTTGGCAGAGAGGAAGG
GATTCGAACCCTCGATACGCTATTCACGTATACACGCTTTCCAGGCGTGCGACTTAAACC
ACTCATCCACCTCTCTAATGGCGGAAATTATCCCAATCGGGATAATTTATTATTTGGTGC
CCGGGAGAAGACTCGAACTTCCACACCCATGAGGATACCAGCACCTGAAGCTGGCGCGTC
TACCAATTCCGCCACCCGGGCAATCTAAATATTGAAATAAAGCAAAGCATTTGATTTGGT
GCCCGGGAGAAGACTCGAACTTCCACACCCGTGAGGATACTAGCACCTGAAGCTAGCGCG
TCTACCAATTCCGCCACCCGGGCTTTGCTTTATTTGCTGTTTTGCGGTACAGTGTCCTGC
CGCAAAGAAGTCGCTATTATATAGTTCATAAAGAGAATGTCAACAGTCCAAATGAATAAA
AATATTAAATCTTTAAATTTACGGAAAAAGACCCGTTTTTAAGTCGTGAAAAACAGCGT
TATGAACATCCTTTGCCCAGTCGGGAATGGATAATCGAATTGTTGGAGCGCAAAGGTGTG
CCTTCAAAAATCGAATCGCTTGCGCGCGAGCTGTCGATTACGGAAGACGAGTATGTCTTT
TTTGAACGCCGTCTGAAGGCGATGGCGCGGGACGGTCAGGTTTTAATCAACCGTCGGGGC
GCGGTTTGCGCGGCGGACAAATTGGATTTGGTCAAATGCCGCGTCGAGGCGCATAAGGAC
GGTTTCGGTTTTGCCGTGCCGCTCACGCCCGCCAAAGACGGTGATTTTGTTTTGTATGAA
CGCCAGATGCGCGGCATTATGCACGGCGATATTGTCACTGTTCGTCCTGCCGGCATGGAC
CGTAGGGGCCGCCGCGAAGGGACAGTTCTGGATATTGTCGAACGCGCGCAAAGCAAAGTG
GTCGGCCGTTTCTATATGGATAGGGGCGTGGCGATTTTGGAGCCGGAAGACAAGCGTCTG
AACCAAAGCATCGTATTGGAACCGGACGGCGTGGCGCGTTTCAAACCTGAATCCGGTCAG
GTCATCGTCGGCGAAATTGAGGTTTATCCTGAGCAAAACCGGCCGGCAGTGGCAAAAATC
ATCGAAGTTTTGGGCGATTATGCCGACAGCGGCATGGAGATTGAAATTGCCGTGCGCAAG
CATCATTTGCCGCACCAATTCAGTGAAGCGTGTGCCAAAGCTGCGAAAAAAATTCCCGTC
CATGTACGCAAAAGCGATTTGAAAGGCCGCGTCGATTTGCGCGACCTGCCTTTGGTAACG
ATAGACGGCGAAACGGCGCGCGATTTCGACGACGCGGTGTTTGCCGAAAAAGTCGGACGC
AATTACCGTCTGGTCGTGGCGATTGCGGATGTCAGCCATTATGTCCGCCCTGACGATGTG
ATTGATGCAGATGCTCAAGAACGCAGTACCAGCGTATATTTCCCGCGCCGTGTGATTCCG
ATGCTGCCGGAAAACCTGTCTAACGGCATTTGCTCGCTCAATCCCGATGTCGAGCGTTTG
TGTATGGTGTGCGATATGGTCGTTACCTATGCGGGCAATATCAAAGAATACCGCTTCTAC
CCCGCCGTAATGCGCTCTCATGCCCGCCTGACCTACAACCAAGTTTGGAAATGGATTTCA
GACGGCATCGACCATCCGTACAAAGCCCAAATCGACACCCTTTACAAACTCTTCAAAATC
CTTCAGAAAAAGCGTTTCGAACGCGGCGCGGTGGAGTTTGAAAGCGTCGAAACCCAGATG
ATTTTCGATGACAACGGCAAAATCGAAAAAATCGTCCCCGTTGTCCGCAACGATGCCCAC
AAGCTGATTGAAGAATGTATGCTGGCGGCGAATGTTTGCGCAGCGGATTTCCTGTTGAAA
AACAAGCATACGGCTTTGTTCCGCAACCATTTGGGCCCCACGCCCGAAAAACTCGCCACC
CTGCGCGAGCAGCTCGGTCTGTTGGGGCTTCAACTTGGCGGCGGCGACAACCCGTCGCCG
AAAGACTATGCCGCGCTTGTCGAACAATTCAAAGGCAGACCTGATGCCGAATTGCTGCAA
GTCATGATGTTGCGCTCCATGCAGCAGGCGGTTTACGAACCGCATTGCGACGGACACTTT
GGTCTTGCCTACGAAGCATACGCCCACTTCACCTCGCCCATCCGCCGCTATCCCGACCTG
ACCGTACACCGCGCCATCAAAGCCGTGTTGAATCAGCAAACCTACACGCCAAAAAAAAGC
TGGCAGGCTTTGGGCGTGCATACCTCGTTCTGTGAGCGCCGTGCCGACGACGCCAGCCGC
GACGTGGAAAACTGGCTGAAAACCTATTATATGCGCGATAAGGTCGGCGAAGTATTCGAA
GGTAAAATCTCCGGCATGACCAGTTTTGGTATCTTTGTAACACTGGACGGCATCCACATT
GACGGCTTGGTGCATATCAGCGATTTGGGCGAAGACTATTTCAACTTCCGCCCCGAAATC
ATGGCAATCGAAGGCGAACGCAGCGGCATCCGTTTCAACATGGGGGACAGGGTTGCCGTC
CGGGTCGCCCGTGCCGATTTGGATGACGGAAAAATCGATTTTGTCCTGATTGCCGGGGGG
AGCGGCAGGGGGCGGAAAGTTAAATCATCCGCGTCTGCCAAACCGGCAGGGACGGCGGGG
AAAGGGAAGCCGAAAACCGCCGCCGAGAAAAAAACAGCCCGAGGCGGCAAAGTAAGGGGA
AGGGGCGCGTCTGCCGCCGCAGAATCGAGGAAAAAGGCAAAGAAACCGGTTCCGATTAAG
GTAAAAAAACGGAAAGGCAAATCATAATGCTGACGGGGTGGCTTGAGGAGGCGGGGCATA
TTTTCCCGAAGCCTCCACCCGATTTTTTGATGTGGGCGGACGGAGCGTTATTTCCAGCAA
ATTGAAACGCCCGTATTGAAAGATTGCGTTTATTTCCACCGCCGTTTTAAAGGCCGGCGG
TATTCGGCAGACGGGGCGCAAACGGCGTTCAGACGGCATTTTCATTCTTTCGGCGTGTCC
GTCCGAATTGCTTTGCCCGTCCGCGCAATCAGCCCGTGCGGCTTGCCTTGAACGGACAAA
AAATGCCGTCTGAAACCCGAAAATCAGGTTTCAGACGGCATTTTTCCTTGAAAAGGCTGT
TCAAATCAGCGATGGTAGTTCGGTGCTTCTTTGGTAATTTGAACGTCGTGAACGTGCGAT
TCGCTCATACCTGCGGAAGTGATTTCCACAAATTCTGCTTTTTCGTGCATTTCGGCAATA
TTGGCGCAACCCAAATACCCCATGCTGGAGCGCAGTCCGCCGGTCAGTTGGTGGATGATG
TTCACAATCGGGCCTTTGTAAGGAACGCGGCCTTCGATGCCTTCGGGGACGTATTTGTCG
GTGCTGTCGGTTTTGTCTTGGAAGTAGCGGTCGGCAGAACCTTGGCTCATCGCGCCCAAG
GAACCCATACCGCGATAGGATTTGTATGAGCGGCCTTGGTAGAGTTCGATTTCGCCCGGC
GCTTCTTCCGTGCCTGCAAACATACCGCCGAGCATGACGCTGTACGCGCCTGCGGCGAGG

## Appendix A

GCTTTGGCGATGTCGCCGGAGAAGCGGATGCCGCCATCGGCAATCAGCGGAACGCCCGTG
CCTTTGAGGGCTTCGGCAACGTTGTGAATGGCGGTCAGTTGCGGCACGCCGACACCTGCC
ACGATACGGGTGGTGCAAATCGATCCCGGACCGATACCGACTTTGACGGCATCCGCGCCG
GCGGCGACCAAATCCAAAGCGGCTTTGGCAGTGGCGATGTTGCCGCCGATGACTTGGATG
TGCGGATAGGTTTCTTTGACCCAACGCACGCGGTCGATCACGCCTTGGCTGTGCCCGTGG
GCGGTATCGACGACAATCACGTCCACGCCGGCCTCAACCAAGGCTTTGACGCGCTCTTCG
GTGTCGCCGCCGGTGCCGACTGCCGCACCGACGCGCAGACGGCCTTCGGAGTCTTTGTTG
GCATTGGGAAACTCGGTGGTTTTTAAAATATCTTTGACGGTAATCAGACCTTTGAGTTCG
TCTTTTTCGTTCAGAACCAAAACGCGCTCGACTTTGTGCGTGTGCATCAGTTCGCGCGCT
TCGTCTATGCTTGTGCCTTCGGGGACGGTAACCAGACGTTCGCGCGGGGTCATAATGGCG
GAAACGGGCAAATCGACGCGGTTTTCAAAACGCAGGTCGCGGTTGGTTACGATGCCGACG
ACTTTGCCGTTTTCAACGACGGGCAGGCCGGACATTTTGCGTTTGCGCTGCGCACGCATT
TCCAAGACTTCGCGGATGAGCGTTGTCGGTGCAACGGTTACGGGGTCTTTGACCACGCCG
CTTTCGTGGCGTTTCACTTTGGAAACGGCGCGCGCCTGCATTTCGGGCGGCATGTTTTTA
TGGATGATGCCGATGCCGCCTTCTTGTGCCATCGAAATGGCGAGGCGCGCCTCGGTAACA
GTGTCCATCGCGGCGGAAAGCAGGGGGAGGTTGAGTGTGATTTCGCGGGTGAGCTTGGTT
TGAAGTTTAACGTCTCGCGGCAGCACGGTCGAATGTGCGGGAACCAACAAAACATCGTCG
AAAGTATAGGCTTTTTCTACGATACGCATAATGCTCGGTCTTTCAGTTTGTGCAAGATGC
ACGGCATTATAGCACGTTACCGGCGGCTTGACAGTTTATCAGGTTTAATTTTGGTCCCCT
TTGAATAGCTCGGTTTTCCTTTGCCGACCACTGTTGCTCCCGTTCTTTCAATTTCAGGAA
AAGCTTTTTTCTAATTTTTGGTAAGTGGCTCAGTTATTGAAGCCCTATATCGGGCGGTAA
CTTCCAGCACAAAAAAACGGAGTAGTTCTTTATTTATTTTTTCCTTTAATTTTCAGTATA
TTATCTTAATATTTCGAGGGTAACATATCTGCTAATCTAGTTACAGCCCCATATATTATA
GATTCAATTGAAAAATAACAGATTCAACTGTACCTTTCTTATACCTGATTTCTTTAAAGT
TTTTCCCATTGTCAAAACTATAAAAAAGTCTCTCTTCCTCACCAGAATATCCCATTTCTA
ATACTAAACAATCTATTTCACAATTACCCCCTCCACTCATATATGTAGTATCAATAGAAA
ATATATTGCATAATTCCAGAGAATTATATTTATCTATATCAAATAGTTGCTCTCCAAAAT
CTAAACCATTGTTTCCACCTGCATAATACTTCTCTTCAAAGGATGACTTCAAACTATTAA
AAACTGCATTTAGCGTAAAATATCCATATGGGCATATCTTAGGATATATATTCTCTCCTA
TTATTATCTGAATTTTTCCATAAATAAAACCATTACATTCAGAGTTTTCTTCCCATAAAA
TCCCAAATTTTCTTGTTGAGTCCATAATAATATTCATATAAATCCTTATATTAATAAATT
ATTTACAATATCCCCCGCTTTCAGACGGCATACGGCGTGGCGGCGGAATGCCGTCTGAAG
GCGGGCGTTATGATAATTGTTCCAGCAGCGTCTGCTTCAATGAGGACTGGATTTTTGGGT
TTTTCAGGTCGGGGCTAAAAACGGTAAAACTGTCTTCGGCGCGTTCATCCAGTGTGGAGA
TTTTGGCATAGCGCAGGCTGACGTTGTGGGCGAAAAAGACTTCCGCCATATCGGCGAGCA
GGAAGGGGCGGTTGACGGCGGTGATTTCGACGGAATACCAGTCGGGATAGTCTTCTTCGG
GGGTGATGGTGATGCTCGGTGCGATCGGCATATAGCGGCTGCGGCGGCTGATGCGGCGGC
TGCGGCTTTGGGTTTCGGCAACGGTGTGTCCGTGGATAAAGCTGTTGAGTTCGGCTTCGA
GCGCGCTTTGGATGTCGGGGTAGTCTTCGGGGGCGTGCTGCGAGGGGATTTGCACGATGA
AGGTGTCGAGGATGTAGTCGTGTTCGGTGATGAAGGCGCGGGCGGCGAGGATGTCGAAGC
CGTGGCGGCTGAAGATGCGGCAGAGGCGGGCGAACAGGCGCGGGCCGTTGGGCATGAAAA
CCATGACTTGAAAGCTGTCGCTTTTGGGCAGGATGCGGCTGCGGACGATGGGGGTTTCAA
AGTCGTGGACTAGGTTGGCGGCGTGCCACAGGATTTCGCGGGACTGGTGGCGGGCGAAGT
AGGCGGAACCGAGCGCGTTCCATAGTTTTTTCTGCTGTTTTTCGGGGACGGCGGCGCGGG
TGAGTAAGTCGGCGGCTTCCTGCCGGCGGCGGCCGAAGAGGGTGTGCGGGTTGCCGCCGT
TGCCTGTAAGGTAGCGTCCGGCGGCATGGAAGAGGCTTTCCAGCAGGCTGGCGCGCCATG
CGTTCCACAGCTTGGGATTGGTGCCGCGTATGTCGGAAATGGTCAGAAGGTAGAGCGCGC
TGAGGCGTTCGTGGGTTTGCACGCGTTTGCAGAAGGCATCGAGTACGCTGGGGTCTTGGA
TGTCTTCTTTTTGGGCGACGGCAGACATCAGCAGGTGGTTTTCGACCAGCCAGGCGAGCA
GGTCGCTTTCTTCTCCGGTCAGGAAGTGGTCAGCGGCAAATTGGCGCGCGTCTGCGATGC
CTTGTATGGCATGGTCGCCGCCGCGTCCTTTGGCGATGTCATGGAAAAAGGCGGCAAGGT
AGAGGATGTCTTGTTTTTCAAAGGACTGCATCAGTGCAGAGGCGTAGGGCAGCTCGTGGC
TGTGCATATCCAAGGCAAGGCGGCGGACGTTGCGGACGACGGTGAGGATGTGGTCGTCCA
CGGGATAGATGTGGAACAGGTCGTGTTGGAGCAGGCCGATGATTTTTTCCCACGCGGGCA
GGTAGCGGCCCAACACGCCGTAGAGGTTGAGAAAGCGCAGGGTCTGGGTCAGCCCGTTGC
CGTTGCGGAAAAAACCGGCGAAGCGGCGGCGGTTTTCGGGATTTTGGTAGAAGCTGCGGT
TGATTTTGCGCGTCGCGCCCCACCAGGCGCGCAGGGTTTGCGGTTCGAGCGCGGTAATGT
CGTTGCGCTGCTGCATGATTTCGACGATTTTGAAAATGTGTTCGGGCCGTCTGAAAAAAA
TATCGGTGTGCCGCGCGGCGATTTGGTTGTTGACTTGGATGTAGTCGTCGTCAATCCGCA
GGGTAACGCGCAACGGGGTGGAGGAAACGCGGCTTTGCAGCATAGGCGTGAGGATGCCGC
CCAGTTGTTTGACGGTTTTAATCGCGCGGTAAAACACGCGCATCAGTTCTTCGCTTTGGC
GGCGGAGGTTCAAGCCTTCATAACCCATGCTTTCGGCGACTTGCGGCTGCAAATCGAACA
GCAGGCGGTCTTCGGCGCGCTTGGCGTTTAAATGCAGATGGATGCGGATGTGGGCGAGGC
GGCGGTAGCCGTGCGAAAGCATACCGGCTTCGGCACGCGTCAAAATCCGCTGTTTGAGCA
GGTCGGGCAGGTCGGTCGCCAAGCCTTGCGCCTTCGCTATCCAAAGCAGGGTGTGGATAT
CGCGCAGACCGCCCGGACAGCTTTTGATATTCGGCTCCAATACTGCCCCCGAACCTTGCG
ATTTGGCGTGGCGGTGTTCCATCTCCACCAGTTTTGCCTCGACAAACGCCGCCACATTGC
GCTGCGCGTTCATTTTTTCCGCCAGTTTATCCACCGTTTGGCGGTTGCCAAACAAAAACC
TAGCCTCTAAAAACGCTGTGTCCCCCGTAATATCATTGCGCACGCTTTCACATAGTTCAT
CAACGCTGCCGCTTTTTACAGACGGCATCAGTTTGCAGTCCCACAGGGTTTGAACAAACC
GGGCAATCTGTTCCTGAATGCCGTCTGAAAGCGGGGCAGGGGAGACAACCGCCAAATCCA
CATCCGAACAGGGATACAGTTCGCCGCGTCCGAAGCCGCCTACCGCCATCAGGCATAACG
CGCTGTTTTGAAAATACTCTGCCCACAATGCCGCCAGTAAGGTTTCGACTGCCGCCGTGT
ATTCTCTGAAAAATACCGACACGCGGTTGGCTTTCAAATAATGCGCTTCGGCGGCATCGC
GCTGCTGTTTGAAGGTTTCCAGTGCTGAAGACAGGTTTGCAGGCATTTTTATTCTTTCGA

## Appendix A

TTGGCGGGAAAAAGGGAGGCGGATGGTTCGGCGGTCAAATACCGCTTTCAGACGGCATTT
GTCGGGTATCGGCGGAATTGGTCATCAGCTTCAACCGTTCCTGCGGCGGCAGCAGCAACT
GATACGCCGCCACGCCCAAAGCCGCCGCCATTTTTTCGATATTCGACAGGGCGATGTTCC
AGCGTTTGCGCTCGACTGCCGACACATAAGTCCTGTCCAAACCGCATTGCCGCGCCAATT
CTTCTTGCGACCAACCCTTGTTCACGCGGAAAAGCCGCATATTGTATGCCAATACCGCCC
GCAAATCCTGTTCGTCAGGCAGTTCGGCAGGCAGAGTCAATTTGTTGCCCATCATTTGCT
TCCAGATAAATGGTTAAAGTTAAGCATTTGCTGTTACGGATTTTACTTCACATAAAAGCC
AAAAAAAAGGGGTGGCGGCAGCCACACCCAAACACACACACATCAAGAGGAAAGAGATAA
AATCAAGACAGATTGGGAAACAGCAAACAAGCACCCTGAATTTCATATCGGTATTATCAA
ACAAAAACCCTTATCAGGTATTGATTCAAATCAATTTAGATTTTATTCGCAAACCGAAAA
AGAAAAACGGCATATCCGTTTATACGGATATGCCGTCTGATGGTGCGGGGGACTGCTGTA
TTTGGCAGTGGTTTGTGTTTTAGTCCTCTTGCGCCGCTGTTTGCAGGTAGTTCGGCAGAC
CGATTTTGCCGATAAGCTCTTGCTGGGTCTCCAGCCAGTCTATATGTTCTTCGTTGGTGT
CTTTTTGTTTTTCCAACAAATCGCGGCTGACGTAATCCTGTTGCGCTTCTGCTGTGGCGA
TGGCGGCAAGCAGGGCTTCGTGTTTTTCCTGTTCTTTGGTCAAATCGCAGGCGATGATTT
CTTCGGTGGACTCGCCAATCAGAAGCTTGCCCAGTTCTTGCAGGTTCGGCAGACCTTCGA
GGAACAGAATGCGCTCGATCAAATCGTCGGCAGCTTTCATTTCAACGATGGACTGTTTGA
AGAAATGTTCGCCCAGTTCTTCAAAGCCCCAGTTTTTCAAAATACGGGCGTGAAGGAAAT
ATTGGTTGATGGTTACCAGCAGCAAGCCTAAGTTTTTGTTCAGCTCGCGGATAACCAAAC
GGTCGCCTTTCATACGGACTCCTTTTATTCCGAATACGGATTACAGTTGGCTTTGGTAGT
AGTTGCCTTCGCCAATTAACTCGATCAGGCGAAGCTGCTGTTCCAGCCAGTGGGCGTGGT
CTTCTTCGGTATCTTTCAGTTGGGCAACCATCAGGTCGCGCGTAACATAGTCTTGAGCCT
CTTCGCACAGTTTGATGCCTTTTTTCAACGCATCACGTACTTCATATTCGGTTTGCAGGT
CGGCTTTGAGGCAGGAAACCACGTCCGTGCCGATATTCAGTTCGGCGCGTGCCATTTTCG
GCGTACCGCCCAGCATCAGGATGCGGCGGATGAAGTCTTCGGCGTGTGTGGTTTCTTCTT
CCATCTCGTGGTTGAGACGTTCAAAAAGTTTGGTGTAGCCCCATTCGGAGTAGAGGCGGG
AGTGGATAAAGTATTGGTCGCGTGCCGCCAGCTCGCCAGACAGCAATTCGTTCATATAAT
CAACAACAGCTTGATTGCCTTGCATAATATCTCTCTTTCTGTAACTTGGTTTTCGGTATG
TCTGAACAGCATCTGTGTCGCTATTCGGGATGCGTGCATTGTATGCAAAAGCTGTCGGCA
TGACAAATTTTCTATTTAAAATACAAACAATTATCAAAAGAAATAGGGCGTATTCCCCGA
TGCCTTCCAAATCAGTATGCTGTTTCTTATCGGTTTTTTTGCTGCTAAAACTAAGAATCC
ATCTCATCCATAAAGAACATTTACACTTATTAACCATAGCAAAAAACAAATAGAGCACGG
TTTTTTATCAAAATTTATAATGAATCGTTCTCATTAACCGACAGATTCTTTTGAGATTAT
CGGATTTTGGGAATAAATTATGCCGTCTGAGGGGCTTTCAGACGGCATGCCTGCCCGGAC
AGACGCTGCTTGGAGCGGCTGTCGGTTTGAAGGTGTGTTTAATGCGAAGGGCAATCTGTC
GTATGATGTGTTTGCGGCAGGGGCTTTGAAGAAGCCCGGTATTTTAGGACGAAGCGCGCG
GCTGCCGGGGTCAATATCCGCTATTTATTCCAAACCTTATTAAAACTTAAATTTAAAATC
ATGAAGATAGAAAATATCGATATTATTTCGCCGGAACTGTTCCCGCAGGAAACATTCAAT
GAAACCGAAGCATTCGGCGCTTTGGTATGGTTGTGGGCAGTTTCGCCTATTTATCAGCAT
GCCGGCGTACAAGAAGCCGCCGTCAATATCTTGCCGGTATTGAAAAACGGGCAATTTGCC
TTGTTCAGCAGCAACGGACACCCCGTCGCCTACTGCACTTGGGCTTATTTCGATGAAGAA
ACCGAATGGCAATATCTCCAATCCAATGATGTTCTGCGCCACTCGGAAAACTGGTGCAGT
GGAAACAGAATGTGGCTTATCAACTGGTTCGCACCTTTCGGCGACAGCCGTATGATGAAA
AGAATTCTGGTGCATCTGTTTCCGAAACGTGAAATAAGGTGGCTGTACCACCGGGGGAGT
GAAAAAGGAAAACGGATTATGAGGTTTCCAGCGTTGTCGAAACAATAAAACAAGGCTGCC
TGAAAGTTTCCCTTCAGGCAGCCTTGTTTCAGTTCTCTCGAGGCCATTCGTATTTTTTGT
TTTCGGGATAAATGTCGTCACCTACACGATACCAACCTCTTTTTCTCATGCAGGCATCTG
CTTTACTTCCTCCTCCCCCACCTATTGGGTCATAGCCGCACTCTTTCCAGTCTTTTTTTC
~~CTTAGCAAAAAGATCATCTATTTGTTTGCTATATTGTTCATAAGAATATATGGGTAAATT~~
GATATTTTTACTCATCATTGCCGGATATTCTTGTTCTATACGGGAATACTTCCAGTATAC
CGAGTCATCGGGCGGAGGTTTGAATCCCCCAAACGAACAGGCAGCCAATCCCATAGCCAA
AGAGATTGATACGATATATTTCATTTTGTTTTTCCTTCTTCTCGAGGCCATTCGTATTTT
TTGTTTTCGGGATAAGGGTCGAAACCCTTACGACACCAGCCTTTTTTCCTCAGGCAGGCA
TCTGCTTCAGACTTTCCGCCGTCTATTGGGTCATAGCCGCACTCTTGCATGTCTTTCAGT
TGCCTTCGTCTTGCTTTCGATGGATATTGGTCAAGTGCCGCCGAGGCTGATCCCGGATAG
GGATTGGCGTAATTTTTCAATTCCCAAAATGACGCGGCGTCCCACGGATTTGGTTTAAAT
CCCCCAAACGAACAGGCAGCCAATCCCATAGCCAGAGAGATTGATACGATATATTTCATT
TTGTTTTTCCTTCTTCTCGAGGCCATTCGTATTTTTTGTTTTCGGGATAAATGTCGTTAC
CTACACGATACCAACCTCTTTTTCTCATGCAGGCATCTGCTTCACTCCCACCGCCCCCAC
CTATTGGGTCATAGCCGCACTCTTTCCAGTCTTTTTTTTCTCTAGCAAAAGAATCATGCA
GTTGTCTTCGTCTCTCTTCAGGTGGGTATTGGTCAAGTGTCGCTGAGGTTAATCCCGGAT
AAAGTTTGGCGTAATTTGTCAATCTCCAAAATGCCGAGTCATCGGGCGGAGGTTTGAATC
CCCCAAACGAACAGGCAGCCAATCCCATAGCCAGAGAGATTGATACGATATATTTCATTT
TGTTTTTCCTTTCGGATGTTGCAGATCATAAATGCGTACAGGACGGTACATAATTTCGAT
TTCGTTGGACACTCCTCTTTCAAATACACCTGTCGGACTTTGGGTTTGTTTATTTTCAGG
GTCTCGATAACCATCTGTTACACATTGTTTATTAGCCATACCGTAGCAATTATGCGGCGA
GGTGTAATCCCCTATAATATCCCTTATAGCCTGCCATTGGCTTTTTTGCCGGGTATTGGT
TCCGACTGTTGCCGGATTTCTGCCAATCAGCATGCCGATAAGGTCTAATTCGTGGTTTTC
TATTTGGATGGACTGACGGGCGAAATCTGCCGTTCTGGGTGTCGTTACCCCCTGCTGCAG
TTGAAACAGCCTTTTATCTGCCCGGACAACGTTGGCGGCAGGGCCGACCATTTTCAGTTC
GGTATTGGATAAAATTTTCTTATCCCGATTGGCTTGGGTATTCAAGGTATTGAGTACATT
GTCGACCACCAGGGTTCCACGGCTGTGCGAGCCGACAAACAGGCCGTTTTTATCCTTCCC
GTAGTCTTCCATGATATTGCCTAAAGCCAAGCCTGAATTGCTCAAGCCGATAACGGTCTT
ATTGCCTATTTTTGCCCCTTCGAGCATTTTATGAAAGGCCGCAACGGCGATTTCGGGCAA
TTTTGAAAAGCCCCGCCCATTGGTTTCCGGATTGTGCAGGAAATAAACATTTTCATAGGT

## Appendix A

GCGTTCATATCGGTTTTTCTCGGGATTGAAACGCCCCACATATTGTTGGGCGGCAAATTT
GGCGGCTGCTTGTATATTATTGAAAATGCCGTTGACGCCGACAACGATTTTTTCAACGGT
TTTGCCGGTCTCCGGGTCGGTGTAACGGGCAGTTTTCAGATTTTCCGTTCCTGGTCGGA
TACTTCGCGCAATTCGTAGATATTTCCCCCAATGGCTTTATAGGCTTCCCAATAATCTTT
AAATGAGGCAAATTCTTTTTTCAACTTTACTGAGTTGTCAAACTGTTTATTTATATCCTC
TTCAAGCTGCTTATCTTCAATCGGCTCGCCGTTTTCATCCATTTTAAAGGTCATCAGGCG
GTGTTCTGCAATAAACTGGCTGCGGTAGGCTTCGTCTGCAATGCCGCCGATCAGCGTCTC
ATTGATAAACTCTTTGGCAACATCCCTATTGAGTTCGACTTCTTTCAGCAAGCCTTCGCG
GTCTGCTTTTGCCAATGCCTTATGTGCGGATGAAGTGCCTTTTTCAATACCGGCAATATT
TTTCCTGCCCTGCGCGGAAGCAATTTGCCAATCTCCCTCACTGATGACGGAGCGGGTAAT
GGATTGGCGGCTTTCTGATTCTTTGGCATTGCCCAACAGATTGCCCAAACCCATTTTTGC
CAAGGCGTATTTGTCGTTGCTGTGTATATCGTTTTGCTTCAAACCGAATTTCAAGCCGCC
GGCGTTGTTTTGATTTAAATTTGCCTCTTTAAAGGTTTCCCCTTTTTCAATACGCTCACT
TCGATATTTTTCGTTCAACTCGTCGTCTTTCGCCGAAACCTGGTCAAAACGCATCAGGCT
GACCGGTTTGCCGCCGATTTTACCGATTTCTGCTTCTTTTTTGGTGGAATACTGTCCGCT
GGTAGGCTTCGGGCTGTATGAAAAACCGCCGCTCAAGCCCAAGGCGGAAGCAGCCGCCGA
AGCATGGTTTTGAATATCTTTATGCCAGATTTCGCTTGTTTTCAGCAGGTTTTTTGATTT
GTCGGCATCTGAAACAACAGCGGCGCCGACCAATCCGGTTTTGCCGTTTACGCGAATCCG
ATAGCCGTCTCCTCCTGCAAAGATACCGCTTTGCTCGTTGACGGATGCATAATCCGAGCT
GCTTTTCGAGCGGTTATAGCTGCCACCGACACTAAAGCCGTAGCCTACCGTAACTTGGGC
GGAAACATTTTCCTGTTTGCCTTTAAACACGGCGGTATCCTGCAAACTTTCGATATGCAG
ACTCTCTGCCGTTACGCCAACGCCTTTGCCTTTAAGCTGCCCGCCTTTGATGACGGTATC
GCCACCGCTTTCAATAGCGGTTTGGCTGTCTTTGCTGCCGATATGGCTGTTGCGGTAGGC
GGTTTCGTCGCCGTTGCCGTAACCTTTGCCGTAGTTTGCTCCGGCTGTGAAGCCGAAACT
GATGCCTTTGTTGATGGCGATGGCGACTCCGGCATTAAAGCCTGCGGATTTGTTTTCGCT
GCGTTCCTGATGCGTTTGGCGGGCGGCTTCAATCTGAACGGCATTTTCTGCTTTGAGGCG
TGTTCCTTTGCCGCCGTACACATCGGAGCCGGTAATCGTGATGCGGGAGTCTTTGCCTGC
GCCTGAAGCAGTCAGGGAAACTTTGCCGCCGCCGGTGATTTTGCCCTCTTGCACCTGCGT
GCCTTTGATGCGGCTTTCGGAGGTGTTCTTCTGTTCGCCGTAGGTAACGGAGACACTGAT
GCCCTGACCGGCTGCTTTTTTGGGATTTCGGGCGGCATTATAGAGTGCCACGCCGGAATC
TACTCCTTTATTCAAGGCGTTGGCAGCAGCCATGGCATTGACCCGGCTGTTTTTGCTTTT
GCCGACGGTTTGGACTGCTTTTACGGCGTCAACCGCGCCCATTACGGTATTCACAACCGG
AACGCTAATGGCGACGGTTACGCCTTTTTGTTCGTAAACCTGCTTACTCTCTTGGCTGTA
ACGGTTTTGTGCGGCATCGATGCTGATTTTTCCGGAGGAAATGCCGACATCGCCTTGGGG
CGAGGATATGGTCGAACCGGTTTGGGTGTAATGTTTTCCTGCCGAAATCAGGGTATTGCC
GTTCAGGCTGCCGACGACGCTTTCTGTGTGGCTGACGGTCTCGGATCGGTTGGTTTGCGT
GTCTTTTTTGCTGCCCGCCGTAAAGCCGATGCCGCCGCTGCCCATCAGTCCGGATTTTTC
TTTTTTGTTCATTTCGGCACTGCGGCTGCGTGTTTCGGCTGCTTTAAGGACGATATTGTT
TTTTGCCGAGAGAATGGTATGGTTGTCTGCAATGATATTGCTGCCAGTAACGGTAATATC
GCGTCCTGAAACCAGAATGATTTCTTTGCCGTCCAGCGTGCCGGATACGGCTTGTCCGTT
TTGGTTCTTGAGATGGCGGGTCATCTTCTGTTTGATGCCGCCCCCGCTTCTACCGGTGTA
TTTCAGGGCATCTTCGGTTTCGGTATGGGCTTTGCCGGCTTCGACTTTGATATCCCGTCC
GGCTGCCAGTTTCAGACGGCCTTGTTCGCTGCCGACCTCTGCTGCACGGATACGGATGTC
TCCTTTTGCATTCAGACTGAGATTGCCCCGGGTGCGGATGGTGCTGCCGACTTCGTTTTG
TTCTTTGCGAATCACATAGTTGTCGGAATCAAAGATAGTGTTCTGATTGCGGGAAATGCC
CGTCGTATCCGAGCGGATGTCGCCGCCGGCATTCAGTACGGTTTGACCGTCTTCAGATTG
ATTGGTCAATTCGGAAGCCGTCAGGACGATATTGTTGCCTGCATCCAGCAAGACGCTTCC
ATTCTGCCTGCCGGTCAGATAAATGCCTGCCACCCGGCCGATATTGCGTACCGAGCCTTG
~~CTCATTCTGATTGCTGCGGGTCTCGCTGCGGCTTTCTATATTGTTACTCGGTTTGAGCAG~~
CAGGGCGTTTTCTGCGCCGATGCTGCCGGTATTCGTAATGTCGCTGCCTGCTGCGGCGAA
GATGTTTTTGCCCTGCAAATCACCTTGCAGATTTTTAATATTCTGTGCGTTTAAAATTAG
TGCTTCGCGCCCGGCAATTAAGCCGCCCCGGTTTTCAATGGCGCCGCTGCCGATATCAAC
AACGCTGCCGGACAGCAACGCCCCTTGTCCGTTCATATCTTTGGGGCGTGCGCGGACATA
GACTTTGGGTTTCAATACGGTTTGAGTTGTCCCGTCGGGCAGGGTAACGGTCTCGTTTTC
CAGCCAAACAATGTCGGAAGTCAGACGGGCAACCTGTTCGGCAGACAGGGCAATACCCGG
AGTAAGCTGCAATTCTTTGGCTATGGTAATGCCGTTATCCATCAAAGCCTTGAATTGCTC
TTCGTCATTGGTATAACCGTCCAAGCGGCGGTAGCCTGTCAGCTTGGCGATTTGTTCGTT
TACCAGTTTCTGCTCGTAATAGCCGTCGCCCAAACGCTTGTGGATATGGTTCGGGTCTTG
TTGCAGTGCGGCAAGCATATAGCCGCTGCCCAGCCATTTGCGGTAGTCGGTAAAGGCAGG
GTCGGTTTCAATCAAATAGCCTTTGTTGTTTGGCGCAATGGCAAACAAGCTGCTGTTCGG
CAGAGTAAATGTAGGATGGATGCCGTTTTCAGCAACAACGGGTACAACAGTGCCGGGTAT
ATCAGATGCCTGTTGGGGCGCATAGCCTTTATAGGCTGAAATACCCATACGGATGGAGCT
GACTTCGGGGGCCGGTTCGTAGGGAGACCGACTGTATCCCGTAGAATCCCGCCCTTTCTT
GTGATGACGGTGGTAACGGTGCAAGTCCCCTTTATCGGTTATGGTTTTTGTTCCCAAAGT
TTCATCATTGTCCAATGCGGATTCGGGCGTACCGACGATCAATCTGCCGCCGGCAATAAT
CCGGCTGTTATGATTTTTCAGCTTGGCGGCATCTAAAACCAAATTGCCGCCACTGATGAT
TTGACCGGGCTTGGATTCGGTAATTTTGCTCTCGGCGGTGATTCGTTCGTAATCGTATTT
GTACCAGACAGAGTGGGGGCTGCCGTCCGGAGTCCGCATATGTAAAGACTCATCTTCAAA
TATTTCCCAGCCCAACTCTTTTTGAGAACCTTCCGGATAGCGTTCTGTTCTGCCTTCCGC
CTGATATTCGATACGGTGTTCGCGATGGGTTTCTTCCGTATGGAAACGCAGATGCTCATT
GGTATTCTGCAAATCTTTTGTAGCGATACGGATATTGCCTGATGATTCGATTGCCGCACT
GCGGTTGTGCAGTGATGTATTTGCTCCTTGCACCTGTCGGCTTCCATTCAATGCAGAACC
GATATGAAGATCGCCGGAGCTGGACAATAATGCCGCCTCTCGGTTCTCAATTTCCCGCGC
TCCAATATCCAACCGCTCCCGCGCTGCAATTACCGCCGCTTTGGTTTCGCCGTTGACCGT
TTCTTCCCGGTTCAACAAGGTATCTGCCTCAACTGCCACACGGCTGCCGTAAATTCTGCC

## Appendix A

CGTGCCGGCATTGTCCGACTTGGCTTCGGTTTGCAGCAGCGTTATACCGTTGCTGTTGAT
TAAACCCCTGTTGGTGATGCCGTTTTTGCCGTTTAACCCGGTGCGGTTTCCGGATTGGAT
TTTACCGGAGACAGTGTTGTCGATTTGGGCGGCGGTCAGGGATACGGTATCGCCTGCCTG
TATGATGCGGGTGTTTTTCAGACGGCCTTGGGTGGAGAACGTGAGTGTGCGTCCGGCTTC
AATATCGCGTTTGCCGGCAAAATCATCATGAAGAGAAACGGAAACATCGCGTGCGGCGGT
TAATATGCCGTCGTTGTCCAGTGATTTTGCCTGTAAGGAAACATCTTTGCCCGCAATAAT
CGTGCCGTCTGTGTTATTGATATGCAGGCTGCTTTTGCCTGTATCGCGAATATCCAGCAA
ACCGGCCGAGCCGATTAAGCCTGCTTGATTATTTATGCCGTCTGAAACCTTCAATACACT
GCCTTTGCCGCTGCGGATAAAGCCTTGGCGGTTATCAACGGTTTGAGCGGAAATGGTGAT
TTCGGCAGCATCAATCGAACCGCTGTTGCTCAATTTGCCGTCTGCGCTTAACGTAACGCC
GCCTGTTGCGGCAAAAATCCGACCCTTGTTGCGGATTACGGCGCCGTTGTCGGTGCTGAT
TAAAGTGATTTTGTCTGCGTACATCCCACCCAGTGTGGCGGTGTCGATGGCAACGGTAGG
AGTAACAGAATCCGAAGAAGATGGCGCAGAAGCTGTTTTGGCAAGAGAGCCGTCAAAATC
CAATTTGTTCTTACCCGAAACCACCTTGACATCTTTACCCCAAACGCCCGCATTGATTTC
AGCAGCACGACTAAGGATACGGGTGTAATCGGCATCAGAGGTATCCAAACCTTTGCCCCC
AATCACGACTTTACCCGAAGAAACATCAAAGCCCGTCAGATTGCCGTTATTCAAAACAGG
AACGCCCGAAGTCAGCGTAACCGAAGCGGCATTAATCAATCCGCCGCCATTCACACGGAT
GCCCGACGGATTGGCAACGACTACTTCGGCGCGTTTGCCGCCGACTTCGATATAACCGTT
CAACAACGAAGGATTACTGCTGTCAATCTGGTTCACAATTACCCGCGCTTCGCCGCGTGC
CAGATGGGGATTGCCTTGAATCCATCCACCGAGTTGCGTTTGCGTATTGCTGCGGCTGTT
GTTTAGTATTACGCCTTTTTCATCAACATCGAACTGCTTGAATCGGTTAACAGAAACGCC
TTGGGATGACGGAGTTTGAATATTGACTTGCGGCAAACCGTTTGCTGTCTGAAGAATAAC
GGCTTGTTGGTTTTTAGGGGCGGATTTGTCGGCAATGATGCCGGAAGCAGGGGCAGGGGA
AAACGCAGCAACACCCAAAGCCAACATGACAGAAAAGGCAGCCATACGGAAACCGAAGGC
TGCCCGGGCAGAAGAAACAGAAGCGGCACCGGTCACTCGAACCGAAGCCGCCTCACTATC
CTGCATACTCTTGCCGTCACGATGAACATTCTCTGCTACAGCCATCATACAACTGCGTTT
CTTGTTGAAGATAACCTTGTAGCATCGCTTGTTCATGATGGGTTTTCTTAAATGAAATGT
AAGGAATAGTTAAGGACAAAATATAGGAAATTTGAATTAAATTGTCAATAAAAAACTGAC
AGCGTACCCTGATAATCAGATGTTGTACGCTAATTGTGAGAAGTATGTCGGGATGATTAT
TTTGAAGTTTTCTTTTTATTCAAAAGAGTTACTATGAAAGTTAACCGGGCGTAAATAGGC
TTCAATAAACAGGTTATCGTTATTTTTCAATTGGGAAAAAATCTTCCCTAACCCATCCAT
TCGCCAAATTATTATGTGGGAATTCGGCAATTTCAATAACGGCTGTTGATCCATATCTGC
TCGGTCATTTAGCGTTATAAAGTCGCAAATAGCGTCAGAATTTACAAGATTATCGGATTT
TGGGAATAAATTATGCCGTCTGAAGGGCTTTCAGACGGCATAGGCGGCTAACACGGGTGC
GGCTTGCGCCAAACGGCGCGGCAGGCGCAGGGAATCGCTTTTTACTTTGGCGCGGTGTTC
CGGGTTTAATGATGCCCGCAACCGCCGTGGCTTTCCCTCAAGGCTTCCGCCGCCTGTTCG
TCGGCGTGGTAGCTGGAACGTACCATCGCGCCGATGGCGGCATTGCTGAAGCCCAGTTCG
TATGCTTCTTTTTCAAAGATTTTGAACTGCTCGGGCGTAACGTAGCGCAGGACGGGCAGG
TGTCCGTCTGAAGGCTGGAGGTACTGTCCGATGGTAATCATTTCGATATTGTGCGCCCGC
ATATCGCGCATAATTTCACGCACGTCTTCGTCTGTTTCGCCCAAGCCGACCATGATGCCG
GATTTGGTCGGGATGTGCGGCATCATTTCTTTATAACGTTTTAATAAGTCTAAAGAATGT
TGATAATTGGCACCGGGACGGGCTTTTCTGTACAGGCTCGGATGGGTTTCTAGGTTGTGG
TTCATCACGTCGGGCGGGGTTTCGGCAAGGATTTTGAGTGCGATGTCCAAGCGTCCTCGG
AAGTCGGGGACGAGGATTTCGATTTTGGTGTTCGGGCTGGTTTCGCGGATGGCTTTGATG
CAGTCGGCGAAATGCTGTGCGCCGCCGTCGCGCAGGTCGTCGCGGTCGACGGAGGTGATG
ACGACGTAACGCAGGTTCATGGCTTTGACGGATTCGGCGAGGTTTCTCGGTTCGTCGGGG
TCGAGCATATTGGGCCGACCGTGTCCCACGTCGCAGAACGGGCAGCGGCGGGTGCAGATG
TCACCCATAATCATGAAGGTCGCCGTGCCTTTGCTGAAGCATTCGCCGATGTTGGGGCAG
~~GAGGGTTGCTGGCAAACGGTGTGCATCTTTTGTTGCGGCAAAATGTCTTTGATTTCAAAG~~
AATTTGCGCGATGGGAGTTTGGCGCGTATCCATTCGGGCTTTTTCAGTTTTTCCTGAAGG
GGGACGACTTTGATGGGGATGCGCGCGGTTTTGTCCGCGCCTCTGAGTTTGATGCCGCGT
TTGGGGTCGTCGGTTTTGATTTCACTCATTGTTGTCTGCTTTCGGTGTGAATTGTGTTTC
AAGGTGTGCGGTGAGTTTGGCGGCGACTTCGTCCGGCGTGGGGCAGGGTTGGACAAAATC
CGCGATTTGCGTCATTTCCATACCGGCGTAGCCGCAGGGGTTGATGTGGGTAAACGGGCT
TAAATCCATATTGACGTTGAGCGCAAGCCCGTGATAGACGGAGCCGTTTTTGATACGCAG
CCCCAGTGAGGCGATTTTGCGTTCTCCGACATAAACGCCGGGGCGTTTGGGGTCTGCCGC
CGCTTCGATGCCGTATTCTGCCAATGTGGCGATGATGCTGTTTTCAAGCGCGGAAACGAT
GTTTCTAACACTGGTTTTGCGCCGTTTGAAATCAATCATCGTATAAACGACCAATTGCCC
GGGCCCGTGATAGGTAATCTGCCCGCCCCGGTCGATTTGGACGACGGGAATGTCGTCGCG
AATCAGCAGGTGTTCGGGTTTTCCCGCCAGTCCTTGTGTGAACACGGGCGGGTGTTCGAC
GACCCACAGTTCGTCTTCGGTGTCGGCATTCCGTCCGGCATTAAAGGTTTTCATCGCTTC
AAAAGTCGGCAGATATTCGACCAAACCTTTGTGTATGATTTTCATCTCAAAGTACCACTT
TGACCAGTTCGTGCGAAGTCAGCGCACGGTAGATGTTGTCCAATTGTTCTTGGTTTTCAA
CCTTTACCTGTACGGTGGCGCCAGTATAGTTGCCTTTGCTGCTCGGACGCGTGGTGATGT
GGTGCGCCTGCGTGTCGGGGGCGTGGAGGCGGACGGTGTCTAAAACCGCCTGCTCGAACT
CGGGATGCACCGCGCCCATTACTTTCAATGGGAAGGTGCAGGGAAATTCGATGAGGGATG
TTTTGTTTTTTTGTTCGGTCATGATGTGCTGCCTTGTCGTGTACGGTATGCCGTCTGAAG
GCGGGTTTGCCTTTCAGACGGCATCGGATGTGCGTTATTTTAGCCTAAACCGCGATAACA
GGCTATCGGGAAGGCGGAGGCTTTTTTGACGGCGCGGCGGTTCTGCTATACTGGCGCACA
ATATTATTTTTAGAAGGGTGGTTTTTATGTATCGGAGGAAAGGGCGGGGCATCAAGCCGT
GGATGGGTGCCGGTGCGGCGTTTGCCGCCTTGGTCTGGCTGGTTTTCGCGCTCGGCGATA
CTTTGACTCCGTTTGCGGTTGCGGCGGTGCTGGCGTATGTATTGGACCCTTTGGTCGAAT
GGTTGCAGAAAAAGGGTTTGAACCGTGCATCCGCTTCGATGTCTGTGATGGTGTTTTCCT
TGATTTTGTTGTTGGCATTATTGTTGATTATCGTCCCTATGCTGGTCGGGCAGTTCAACA
ATTTGGCATCGCGCCTGCCCCAATTAATCGGTTTTATGCAGAACACGCTGCTGCCGTGGT

## Appendix A

TGAAAAATACAATCGGCGGATATGTGGAAATCGATCAGGCATCTATTATTGCGTGGCTTC
AGGCGCATACGGGAGAGTTGAGCAACGCGCTTAAGGCGTGGTTTCCCGTTTTGATGAGGC
AGGGCGGCAATATTGTCAGCAGTATCGGCAACCTGCTGCTGCTTCCCTTGCTGCTTTACT
ATTTCCTGCTGGATTGGCAGCGGTGGTCGTGCGGCATTGCCAAACTGGTTCCGAGGCGTT
TTGCCGGTGCTTATACGCGCATTACAGGCAATTTGAACGAGGTATTGGGCGAATTTTTGC
GCGGGCAGCTTCTGGTAATGCTGATTATGGGCTTGGTTTACGGTTTGGGATTGGTGCTGG
TCGGGCTGGATTCGGGGTTTGCCATCGGTATGCTTGCCGGTATTTTGGTGTTTGTCCCTT
ATCTCGGGGCGTTTACGGGATTGCTGCTTGCCACCGTCGCCGCCTTGCTCCAGTTCGGTT
CGTGGAACGGCATCCTATCGGTTTGGGCGGTTTTTGCCGTAGGACAGTTTCTCGAAAGTT
TTTTCATTACGCCGAAAATCGTGGGAGACCGTATCGGGCTGTCGCCGTTTTGGGTTATCT
TTTCGCTGATGGCGTTCGGGCAGCTGATGGGCTTTGTCGGAATGTTGGCGGGATTGCCTT
TGGCCGCCGTAACCTTGGTCTTGCTTCGCGAGGGCGTGCAGAAATATTTTGCCGGCAGTT
TTTACCGGGGCAGGTAGGCGGTTCCGAAACATATAGTGGATTAACAAAAATCAGGACAAG
GCGACGAAGCCGCAGACAGTACAAATAGGGCAACGCCGTACTGGTTTTTGTTAATCCACT
ATATTTGAAGCGGAATACAACCTTGCCCGGGTTTAAATAAAAATGCCGTCTGAACCCCGA
AAACTGGACTTCAGACGGCATTTTCATCACGGCTTATTTGGCGGTTTTGCTGCTGTCGAT
AATTTTCATACCGGCAGAAATCAGGCTGCCGATGTCGGCAACATTGGCGGGCATAATCAG
CGTATTGCTTTCTTTGGCAAGATTGTTGAACGCAGCGACGTATTGTTCCGCAATCTTCAG
ATTGACCGCATCCGCACCGCCTTGGGTTTGAAGGGCGGCGGCAATTTGACGGATGGCTTC
GGCATTGGCTTCGGCAACAAGGCGCAAGGATTCCGCTTCACCTTTGGCGCGGTTGATGCG
GGCGATTTTCTCGGCATTTGACGCATTGACCGCAGCCTGAGCCTCGCCTTCGGATTGTTG
GATTTCGGCTTCGCGCTGACCACTGGCAAGGTTGATTTGTTCGATTTTACGACCTTCGGA
TTCGGCGATACGGGCGCGTTTTTCGCGTTCGGCAGTAATTTGCGCCTGCATTGAGCGAAG
GATTTCTTGCGGCGGAACCAAGTCTTTAATCTCATAACGCAAAACCTTCACACCCCAAGC
CCCGGCCGCCTCGTCCAAAGCCGCAACAACAGTACTGTTGATTTCGTCGCGTTCTTCAAA
CGTTTTGTCCAACTCCATACGCCCGATAACGGAACGCAGCGTCGTTTGGGCAAGCTGGGT
AATCGCCATAATGTAGTTGCTCGAACCGTATGAGGCGAGTTTGGGGTCGGTTACTTGGAA
ATAGATGATGCCGTCAACAGTCAGCTGCGTATTGTCGCGCGTGATGCAGACCTGGCTGGG
TACGTCTAAAGGGATTTCTTTCAGCGAATGGCGGTAGGCGACGCGGTCGATAAAGGGAAT
CAAAATATTCAAACCGGCCGTCAGGGCGCGATGGAAACGCCCCAGCCTTTCGACAACGTG
GACTTCCTGTTGTGGGATGACAACAAAGGATTTGAAACCGAAAACGGCGACGGCTACCAA
CAAGATAATGAAAAATTCCATAATTCCTCCGAGTGTTAAGGGTGTGTGATAATAAGAAGG
TTGCCTTCCTTGCGGACAATGAGGGCGCGAGTTCCTGGTTCAAGCTCTTCTTGCCCCGTA
TTTTGAGCCTGCCAGTGCGTACCGCGATAAAAAACTTCGTAACGGTTGCCGCCTGTGTGT
CGGAGGATTTCGACATATTGTCCGGCATCCAAATCCTGATATGAATCCGTTTCAACTTTT
CTAACGGCGGTTTTGGCGTGTACGAACCAAATACCCAGCGCGGAAAGCAGAGCGGCGGTC
AAGACGGCGGCAGGCGTACTGCCGGTCAGCCCGTAAGCAATGCCCGAACCCGCCAAAGCC
GCGCTGACAACCAAAAGATAAACCGTTCCCGTCAATAATTCGATGATTAAGACGGCAACA
GCGGCAACAAACCATACAGTCATACATTTCCCCACAAAGCGCGTCGTTTGACAAAATAAC
GCAATATCAGCAGTATAGCCGAATTTGAAAGGATAGGGCAGATATGGACACTTGGCACGA
TGCACTCGGCGGCGAAAAACAGCAGCCGTATTTTCAGGAAATTTTAAATGCAGTCAGGCA
GGAACGTTTGTCGGGACAAATCATCTATCCGCCGGCGGCGGATGTGTTCAACGCATTCCG
CCTGACAGCGTTCGACCGGGTCAAAGCCGTCATTCTCGGACAAGATCCGTATCACGGGGC
AGGGCAGGCGCACGGTTTGGCATTTTCCGTCCGGCAGGGTATCCGCATACCGCCGTCTTT
ACTCAATATCTACAAGGAGTTGGAAACCGACATCGAAGGCTTTTCCATTCCCGCGCACGG
CTGTCTGACAGCGTGGGCGGAGCAGGGCGTATTGCTTCTGAACACGGTTTTGACGGTGCG
TGCAGGACAGGCGCATTCGCACGCCCTTTTAGGCTGGGAACGCTTTACCGATACCGTTAT
CAGGCAGCTTGCGACACACCGCAAGCACCTTGTCTTCATGTTGTGGGGTGGGTATGCACA
ACAAAAAGGGAGGCTGATAGACAGTCAAAATCATTTGATATTGACCGCACCGCATCCGTC
TCCTCTGTCGGCATATCGCGGTTTTTTCGGCTGCCGCCATTTTTCACAGGCAAACAGCTA
TTTGAGCCGGCACGGTATCGATCCGATAAACTGGAAGCTGTGAATGCCGATATAGCCGTT
GCCGCCGGCGTGTTAAAATCGCGTTTGATTTGTAATTTCCATTTATTAGGCAAAACCTTA
TGGCAATCAAAAAAAAATCAGCTTTATGCATCACTTTGGGCGGGCTGCGACGAGTTGCGC
GGCGGTATGGATGCGAGCCTTTATAAAGAACTATGTGCTTACGCTATTGTTTTTAAAATA
TGTTTCTGATAAGCATAAGTACGGCGGCGGCATGATTGAGCTGCACGCCGGTACGACTTT
TGACGACATCGTCAAACTCAAAAACACCGCCGACATCGGCGACCGCCTGAATAAGATTAT
CGCCCAAATTGCCGAAGCCAACGACTTAAAAGGCGTGATCGACGTTACCGACTTCAACGA
CGAAGACAAACTGGGTAAAGGTAAGGAGATGATCGACCGTTTGAGCAGGCTTGTCGGCAT
TTTTAAAAAGCTCAACCTTTCTTCCAACCAAGCCGAAGACGACGATTTGTTAGGTGATGC
CTACGAATACCTGATGCGCCATTTTGCGACCGAGTCAGGCAAATCCAAAGGGCAGTTTTA
CACGCCTGCCGAAGTCTCCCGCATTATGGCGAAGATTATCGGAATCAGCGCAGATTGCCG
TCCAGCACCAGCGTTTATGACCCGACCTGCGGCTCGGGTTCGCTGTTGCTCAAAGCCGCC
GCCCAAGCCGGCAGCCAAATCAGCCTTTACGGTCAGGAAAAAGATGTGGCAACCGCGTCC
CTTGCCCGTATGAATATGATTTTGCACAACAACGAAACCGCCGAAATCAACACCGGGAAC
ACCTTGTCCGATTCGTCTTTCCGTGATGAAAACGACGGGCTTAAGACCTTCGATTTTGCC
GTTGCCAATCCGCCTTATCCCGCCCGAAAAAAACGGCGATTACGCCTTTTTGCTGCATCT
GCTCAAAAGCCTGAAACCAAGCGGCAAAGGTGCGATTATTCTTCCGCACGGTGTGCTGTT
TCGCGGCAATGCCGAAGCGCGTATTCGCACGGAATTGCTTAACCTTGACCTTATTAAAGG
CATTATCGGGCTGCCTGCCAACCTGTTTTACGGCACGGGCATTCCTGCCTGCATCATCGT
CATCGACAAAGAACACGCCCAAACCGCCCAATTTGCCGAAGAGGGAACAAACCAAGTTAT
CAGCGGCGGCAGCGTGTTTATGATTGACGCATCGCGCGGCTTCATTAAAGACGGCAACAA
AAACCGTCTGCGTGAGCAAGACATTCACAAAATCATCGACACTTTCACAAACCTCGTTAC
AGCCGTATGGTGCATTTAAGCGAAATCGCAGCACAAGATTACAACCTTAATCTGCCTCGC
-TATATCGACAGCGGAGAAGTCGAAGACCTGCAAAATCTCGCCGCCTATCTATCTTTATGG
CGGCATACCTGCGCACGATATGGACGCATTGGAAGCCTATTGGCAAGTTTTAGGCCGTAT

## Appendix A

GAAAAACGAGTTGTTTGCCGAACACGATGGCCACTTTACCACTATACAACGGAATCGATT
GCAAATCTTTCCCACTCTCAACAGCTTAAAATCCTGCGGGATTGGTGTGGAATTTAGGGC
TAATCTAGTACAGCCCCAAATTTAATCCACTATAAAATCGAAAGCAGCCAAATCAAAGCC
CATATATTGGCGCACCCCGATTACGCCGCCTTCAAAGCCGGACACCTAGCAAAGTTTGCC
GCGTGGCACACTCAAAACGACCTTGCCGCCATCCAACCGGGCAGGCTTATCCGGAAATGG
AGCGAAAGCCTGCTGGACGCGTTCAAACCCGGCAGCCTGATTGAAGAATACGATTTCTAC
CAAATCCTGACGGACTACTGGGCGGAAACCCTGCAAGACGATGTTTATCTCATCGCCCAA
GATGGCTGGAAGGCGGTTAAAAACCTGGCCGAAATCACCAAAGAAAGCGATGAAGCCGCG
AACCTGACCGTCGTCTTTGAGGAAACCGAAACCGACAAAAAAGGCAAAGCCAAAACCAAG
CGCATCAGCAAAAAATACCGCAGCGAAGTCATCGCCCCCGAGCTGGTTGCCCGCCGCTAC
TTTTCAGACGGCATCGCCAAGCTGGAAGAAAAACAAAGCGAGCTGGAACGCCTAAGCCAA
GAATTGGAAAACCACATAGAAGAACACGGCGGCGAAGAGGGTGCGCTGAACGACGTATTG
GATGCAAAAGGCAAACTTTCCGCCAAACTTCTGAAAACCGCATTGGAAGAAAGCGGCATA
GAAGAAGGCGAACGGGCTGTTTTACAAACCACCCAAACACTGATGACGCAGGAAAAAGCC
GCGAAAGACGCAGTCAAAACCCAAATCGAAGCCCTGAACCTTGCCGTATTCAAACAATTT
GGCCGACTTTCCGAAGCCGAAATCAAGCAGCTTGCCGTTCAAGACAAATGGCTTGCCGAT
TTACAAAGCCGAATCGAAAATCGCTTGGAAAACAGTATTCAGCAGCTTATCAGCCGCTTG
AACACGCTGGAAGACCGCTACCGCAGCCCGATGGCCGAGCTTGCCCGAGAAGTGGAAAAG
TGGCAAAGCAAAGTCAATGCCCACCTTGAAAATATGGGTTTTGGAGGCTGAAATGGCAGC
ACAGACAGGCTATAAGGCGAGCGGGTTTTGAGACCTTTGCAAAATTCCCCAAAATCCCCT
AAATTCCCACCAAGACATTTAGGGGATCGCGGTTCGGGTGTCCGCACCGCTTAATACGTC
GTCGTCCACGAACTGACCCATTTGCTCGAACGCCATCGCAACGCCCGTTTTATGGCGCAT
ATGGACAACTTTCTCCCAAACTGGCAAAGCATCAAACAACAGCTTAATGCCTTGGAGTTA
TTTGCACAAATATATAATTTAACATAATATACATTATGCGAACTATCGGAAACAGTTGTA
CGTGTCCCTGTGGTCTTTCCAAGTAGGAAAATTAAAGTATGGCCAATGCGGCTGAATGTA
TAGCCCGGAGCATCCGCGTATCCGAAGGTACTGAACGACATGCCCTGCATCCCTTTCGAC
GCTTCCATCCAGCCGGTATTGGGAAATACGTTGACCTTCAATATAATATACAGGCAGCCG
ACACACAGGATATGCCGCTGCTTTTTCATCATTTCTTCTGTCAAATCCTTGGAACGGTCG
ATTTGAAAACACGGCTTTACATACGCCCCAGTTCCCTTTGCAGGGCTTGAATATTTTGCT
GCCTGTCCAATACATTGCTTTGTAATGCATTTATTTCTTGATGGTTGATGTTGCCGCCCT
TTGCCAGACGTGCTTGTGATAACATTTTTTGGGCTTCAACCAATGCTTTGCGTTCGTTGC
TCAATTCTGTTTCGAGAATGGAGCGTCTGCTGTTGTTTGAGGGTGCTTGTTGCGGCGGCG
GCGTATTGGATTTTGCCGGCTTGGATACTGTTTTGACCGGGGCTTTATATTTGACAACCT
GTCCGCCGTTTGACGGTGATGATACCGGTTCGGGCGTTTGGGGCGGGATATAGCGTTCGC
TGCTGTAGTTGCCGATTGGGGGCAAATCGGTTGAGTGGCAGCTTTTGGACGGCTTGGTGG
TGTAAACGGTTTCTCCGTTGATTGTGCAGGTGTAGATTTTGGCCGCATTCGCACCCAATG
GGCTTGAAATCAGGGAAAAGTTGATTAGGATTAAGAGGAGTTTTGATTTCATAATGTGCT
TGATTTTCGGATAATCATTTGATTTTTTGGTATTTTTTGTAATGCTTTCTTGGCGTTTTA
CGGGCTTTTCTTTTCGGTTGGTTGCTTTTGATTTTCTTTCGGGGCTTTGGCAGCCTGCTT
GTTCTTTTTCCCGGCAGGTTTTTTGTTTTTCTTTTTACTTTCTATGTGTATTTTGGCTTC
TTAACTGAGTTTTTTAATTTTCAGGCGGTATCCGCCTCCCTGATGGCTGCTGATTTTAGG
TAAATCCGCATCGGCGCACAATCCTGCTGGGGCTGATGTATATACGCTTTTGCCATTTGA
GTTGCAATGGTATACGGAGGCTTGCGCCGCCGCGCCGGAAAGGGACAGGAGACCCAAGGC
GGCAAAAAGTCTGATGTTTATACCGGTAAAAGGCGGTGATAACGCCCGAATTATACCGTT
ATTGGCAGGCAAAGATAAGCACCCTGCCCGCGCTTCTTTATCGCTCGGCAAACTGTTTCT
CGATTTCAGTTTCAATCTGTTCCAATTCTTCTTGGCAAGCCAGCCAAACCTCTTCGATTT
GGGCAAGTTGTGTTTTGACTTTTGCCAGCTCGGATAAGGTGTCCTGCAATTTTTCTTTGT
TTTCCTCGAAGTAAGCTTCTTCTTGTGCTAAAAATGCTTCACATGCCGTCTGAATTTCGG
AAAGCTGCGCCATTTCTTTTTCGGCACGGTCTATTTTCTGCTGTATCGGCTTGCCGCGTC
GGGCTTTTTCCTGACGGATTTGCGCTTCGATGCGCTTGGTGTCTTTGCGGCTTTGGCTTT
GTGCGGATGCTGCGGGCGCGACGGCGGCGTTTTCCTGTGCCAAACGCCATTGGCGGTAGT
CGTTCAAATCGCCGTCGAAGTTCTTCAGACGGCCTTTATCGATCAGGAGGAAGCTGTCGG
TCGTGGCTTCAAGCAGGCTGCGATCGTGCGATACGACGATTAAGGCGCCTTGGAAACTTT
GCAGAGCGAGCGTCAAGGCGTGGCGCATATCCAAATCCAAATGGTTGGTCGGCTCGTCAA
GCAGCAGCAGGTTCGGCTTTTGCCAGATAATCATGGCAAGAGCGAGTCGGGCTTTTTCTC
CGCCGGAAAATGGTTCGGTTTTCTGCAACGCCATATCGCCGACAAAATTGAAGCCTCCGA
GGAAATTTCGGATTTCTTGTTCGCGTACTTCGGGAGAAAGCTGCTGAATATGCCAAACAG
GGTTTTGGTCGGAGCGGATGGTATCGAGTTGGTGTTGGGCAAAATAGCCGATATTGAGTT
TTTCGGAACGGACGATGCTGCCGGAGAGTAAATCGATTGTGCCTGCCAAAGCTTTGATAA
AGGTAGATTTACCGCTGCCGTTGACACCCAATAAACCATAGCGCGCGCCGCTTTCCAGCG
ACAGGGTAATGTCGTGCAAAACAGTTTTGCCTTCGTAACCCAAATCTGCGTGTTCTAGCT
TTAACAAAGGATTGGGCAGATGGTCGGGATGGTAAAACTCAAAGGAAAACTCGCTGTCCA
GATGCGCGGGAGCGATGCGTTCGAGCTTCGCCAAAGCCTTCATGCGGCTTTGCGCTTGAA
CGGCTTTGGTGGCTTTGGCTTTGAAGCGGTCGATAAAGGATTGCAAATGTTTGATTTGCG
CCTGCTGTTTGACATAGGCAGCTTGTTGTTGCGCGAGACGCTGCGCACGTTCGTTTTGGT
AAAAATCGTAATTGCCGCCGTATTGCGTGAGTTTTTGCTGCGATAATTCAATGGTTTGGG
TAGTTTCCGCGTTGAGAAAATCGCGGTCATGGGAAATGATGATTTGCGTGCAGGGTAAAG
AAGCAAGGTGGTTTTCCAGCCACAAGACGGTTTCCAAATCCAAGTGGTTGGTCGGTTCGT
CAAGCAAGAGCAAATCGGCGCGGCAAATCAGGGCTTGCGCAAGATTCAGGCGCATACGCC
AGCCGCCGGAAAAGGATTTGACGGGGCGGCTGTGTTCTTCTTGCGAAAAACCCAGCCCGT
TCAACAATTTTGCCGCACGCGCCGGCGCGGTATAAGCGTCGATTTCTTCCAATTTAGCAT
GATATTCCGCCTGCTTCATGCCGTCATTTTGCGCTTCTGCCTGCCTCAATGCCGTCTGAA
AAGCCTGCAACTCGGCATCGCCCTGCAAAACGTAATCCAAAGCGGAAATATCCAAATCGG
GCGTTTCTTGGGAAACGGAAGCGAGCCGCCAGTTTTTCGGAATCGAGACATCGCCGCCGT
CCTGAGTGATTTCACCCTTGATTAAGGCAAACAGGCTCGATTTGCCCGTTCCGTTTTTGC

## Appendix A

CGATCAAACCGACGCGCTGACCGGGATTGACGGTAGCGTTGGCTTTGTCGAGCAGGACTT
TCAAACCGCGTTGCAGGGTGAGGTTTTTGATTTCAATCATAACGGAAACATCGTCGGGCG
GGAAAAGCCCGTATTTTACCTGAAAGTCAGTGCCGATGCCGTCTGAAACGGGAAATTTAC
GGCTGAAGCCAAGCCCAAGCCCTGCGCCCTTCCGAGTGCAGGAAAACCAATGTCCTGAAT
GCCGAATCGGTATTCATGCATTCCACGCTGATTCCGATTCGGGAAAAATCCGCCATGATT
TTGGGATGGATAAACTCCTGAGCCGCGCCCGTCCCGATAATCAATATTTCCGGATAGTCA
ACAGGTTTGACGTCGGACAACAGGTTTTCCGGAGTCAGATCGGACAAGGTTCGGCATTGC
GACAGGCAGACCGAATCCTTATGTACAAGCACGGGTTTATGGAAACTTTGCCCCGCCAGC
CGGATTCCGCCCGCACCGCATTCATATTCCGCAAACTGTCCGTCTATCGGATTTTCTTCA
AACAACATTTTTTTACCCCGTTGCCGCATCATCTACACCGAAAGGGATGCAAAATCAGAC
AAATTCATGTAGGATTGGCAGATTTCATCTGACCCGCCTGCCGATTTCAGACGGCATTTG
ATTCAAAGTGCGGCACAATTATATCGGCAGCGGATATTTTCGTCTTTCAATATTTACATT
TCAGTCGGCTTACAAGGAGACACAATGAAGCCAGTAAACATCGGTCTTTTAGGTTTGGGT
ACGGTCGGCGGCGGTACGGCTGCCGTGTTGCGGGACAACGCGGAGGAAATTTCCCGTCGC
TTGGGGCGCGAAATCCGTATTTCTGCCGTGTGCGATTTGAGTGAAGAAAAAGCCCGACAA
ACCTGCCCGTCCGCAGCCTTTGTCAAAGATCCGTTCGAACTGGTCGCACGTGAAGACGTC
GATGTCGTCGTCGAATTGTTCGGCGGTACCGGCATTGCCAAAGATGCGGTGTTGAAAGCC
ATTGAAAACGGCAAACACATCGTTACCGCCAACAAAAAACTGCTCGCCGAATACGGCAAC
GAAATCTTCCCGCTGGCGGAAAAACAAAACGTCATCGTCCAATTTGAAGCGGCAGTAGCG
GGCGGTATCCCAATCATCAAAGCCCTGCGCGAAGGTTTGGCGGCAAACAGGATTAAATCC
ATCGCCGGCATTATTAACGGCACCAGCAACTTCATCCTCTCCGAAATGCGCGAAAAAGGC
AGCGCGTTTGCCGATGTACTGAAAGAAGCGCAGGCATTGGGTTATGCCGAAGCCGATCCG
ACCTTCGACATCGAAGGCAACGATGCGGGCCATAAAATCACCATCATGAGCGCACTGGCA
TTCGGCACGCCGATGAACTTTTCCGCCTGCTACCTCGAAGGCATCAGCAAACTCGACAGC
CGCGACATCAAATACGCCGAAGAACTTGGCTATCGCATCAAACTGTTGGGCATTACCCGC
AAAACCGGCAAAGGCATCGAGCTGCGCGTCCACCCTACCCTGATTCCCGAAAGCCGCCTC
TTGGCAAACGTCAACGGCGTGATGAACGCCGTGCGCGTCAACGCCGATATGGTTGGCGAA
ACCTTATATTACGGCGCGGGCGCGGGCGCATTGCCGACCGCTTCCGCCGTGGTTGCCGAT
ATCATCGACATCGCCCGCCTGGTTGAAGCCGATACCGCCCACCGCGTACCGCATCTGGCG
TTCCAACCCGCGCAAGTCCAAGCGCAAACCATCCTGCCTATGGACGAAATTACCAGCAGC
TACTACCTGCGCGTCCAAGCCAAAGACGAACCGGGCACGCTGGGGCAAATCGCCGCGCTG
TTGGCACAAGAAAACGTGTCCATCGAAGCACTGATTCAAAAAGGCGTGATTGATCAGACC
ACTGCCGAAATCGTGATTCTGACCCACAGCACGGTCGAAAAACACATCAAGTCGGCAATC
GCAGCCATCGAAGCACTGGATTGCGTGGAAAAACCGATTACCATGATCCGCATGGAAAGC
CTGCATGACTGAGCCGAAACACGAAATGCTGACGAAAGAGCAGGTTGCCGCGCGCAAAAA
AGCAAAAGCCAAAATCCGCACCATCCGCATTTGGGCGTGGGTCATTTTGGCGTTGCTCGC
TTTAACCGCCCTGCTCTCCCAATGCGCGATGTCCAAACCGCAGGCAAAACAGAAAATTGT
CGAGTCTTGCGTGAAGAATATTCCGTTTGCCGAAAAATGGCAAAACGATTTGCGGGCCCG
CGGTTTAGATTCAAACAATACCCGCCTCGCCGTCGACTACTGCAAATGTATGTGGGAGCA
GCCTTTGGACAGATTGAGCGAGAAACAGATTAGATCCTTCGGCAAACTCGGCGCACAAGA
ACAGCTTGACCTGCTCGGCGGCGCAAATGCCTTTGAAGCACGTGACAAGCAGTGTGTTGC
CGATTTGAAATCAGAATAATGTGGACCGATAAAAAAGCCGATTCTTTAAAGAATCGGCTT
TTTTCATAAAAAACGGCTTACAGTGCGTCTTTCAAAGCTTTGCCGGCGCGGAATTTAGGC
GTTTTGGCGGCGGCAATGGTCAGAGGCTCGCCGGTTTTGGGGTTGCGGCCTTGGCGTTCC
GCACGTTCGCCCACGTAGAAAGTACCGAAACCGACCAAAGTAACGGTGTCGCCTTGTTTC
AGGGCGGTGGTTACTGCATTGGTAGTGGCATCCAAAGCTTTTTGTGCGGCGGCTTTGGAA
ATGTCGGCTTCTTGAGCAATCGCTTCGATCAATTCAGACTTGTTCACAATCAGTCCCTTC
CTGTCTTAAAAAATGATGAAATGCCCGAATACTCGGGGTTTGTACTGCTTGAGCAACTTT
CGCTTTATAGCAATTCTGAAATTGCCGTGTCAAGCAAAAAATACGGAATCACCCTATTTG
ACAGGCTTTCAGGACGAAACCGCATTTTTACAACACATTTCCTGCGTTTCAATGTTTGGT
TGCCCTGCTGCGGGGTTTTGGTTTTGAAGCGGATTCCGCCGCCGCTTCCGCACCAGAAGG
TTCTGCCCAAGGCTCAGGCTGGCTTTCCAAACCCAGAGCCAATACCTCGTCTATCCATTT
GACCGGATGGATGGTCAGGCCGGTTTTCACGTTTTCAGGGATTTCTTCCAAGTCTTTGAC
GTTGTCTTTCGGAATCAGGACGTGTTTGATGCCGCCGCGCAAGGCGGCCAACAGTTTTTC
CTTCAAACCGCCGATGGGCAAAACTTCGCCGCGCAGGGTAATTTCGCCCGTCATCGCCAC
ATCGGCGCGTACCGGGATTTTGGTAAAGGCAGATACCGCCGCCAAGGTCATCGCAATACC
CGCACTAGGGCCGTCTTTCGGCGTCGCGCCTTCGGGAACGTGGATGTGGATGTCTTTTTT
CTCGTAAAAATCAGGAGCCAAACCCACTGATTCCGCACGGGAGCGGACAACCGACCACGC
TGCGGACACGGATTCCTTCATCACATCGCCCAACTGGCCGGTGCACTGAATCACGCCCTT
ACCCGGCAATGCTGCGGCTTCGACGGTCAGCAATTCGCCGCCGACTTCCGTCCACGCCAA
ACCGGTAACCTGCCCGATACGGTTTTCGCTTTCGGCAACGCCGTAATCGAAGCGGCGCAC
ACCCAAATAGTCGTGCAGATTTTTCTCATTTACTTTAACCGCTTTAGGTTTGGCTTTGCT
GGTTTTCTTGGTTTCAGACAACCTCTTCTTATCTTCGTCCAAGGTAATCTGCATCACCAC
CTTGCGGCAGATTTTGGCAATTTCGCGGTCGAGCGAACGCACGCCCGCCTCTCGGGTGTA
ATAACGGATAATATCGCGCACCGCGCTTTCTTCGATTGCCAATTCCCCTTCTTTTACACC
GTTGCGCTTCATTTGCTTCGGTACGAGGTACTGCATCGCGATATTGATTTTTTCGTCTTC
GGTATAGCCGGACAGACGGATGATTTCCATACGGTCGAGCAACGGAGTCGGAATATTCAG
ACTATTGGATGTGGCGATAAACATCACATCACTCAAATCGTAATCCACTTCCGCATAATG
ATCGGCAAACTTGTTGTTTTGTTCGGGATCGAGCACTTCGAGCAACGCGCTGGCGGGATC
GCCTCGGAAGTCGTTACCCAATTTGTCGATTTCGTCGAGCAGGAACAAGGGGTTTTTCAC
GCCGGCTTTTGCCATATTCTGCAAAATCTTACCGGGCATAGAGCCGATATAGGTGCGGCG
GTGTCCCCTGATTTCGCTTTCGTCGCGCACGCCGCCCAAAGCCATGCGGACATATTTCCG
CCCCGTTGCTTTGGCGATGGATTCGCCCAAAGAGGTTTTGCCCACGCCCGGAGGGCCGAC
CAGGCACAGAATCGGGCCTTTGAGTTTGTCCATACGTTTTTGGACGGCGAGGTATTCCAA
AATCCGTTCTTTGACTTTTTCCAGGCCGTAGTGGTCGGCATCCAGCACCAGTCCGGCTTT

## Appendix A

GGCGATGTCTTTGCTGACGCGGGATTTTTTCTTCCACGGCAGCTCGAGCAAAGTGTCGAT
GTAGTTGCGTACGACGGTGGATTCCGCAGACATCGGTGGCATCATTTTGAGCTTTTTCAG
TTCGGACAGGCATTTTTCTTCCGCTTCTTTGGTCATACCCGCCTTTTTGATATCTGCTTC
CAAGGCATCCAGTTCGCCGTTTTCGTCTTCTTCGCCCAGTTCTTTGTGTATCGCTTTAAT
CTGTTCGTTCAGATAATATTCGCGCTGGGATTTTTCCATTTGGCGTTTGACGCGTCCGCG
TATGCGTTTTTCGGCCTGCATAATGTCGAGTTCGGATTCCAGCTGTGCCAGCAGGAATTC
CATCCGTTTGCCGATTTCGGGAATTTCCAAAATCTGTTGGCGTTGCGCCAGTTTCAACTG
CAAATGCGCTGCGACCGTATCGGTTAGCCGGCTGTTTTCGGCAATGCCGTTGATGCTGCC
GATAATTTCGGCGGGGATTTTTTTATTGAGTTTGGCGTATTGTTCAAACTGCGCCAACAG
GGTGCGGCGCACGGCTTCGAGGTCGGTATTGCCGCCCGTGTCTTCTTCCACGACCGTCTC
TATATGGGAAACGAACAGACCGCCCGTGTCTTCAATGGTCAGAACACGTCCGCGATACAG
CCCTTCGACCAATACTTTTACCGTGCCGTCGGGTAGTTTCAACACTTGCAGGACTTGTGC
GACCGTACCGGTCTGATACAGGTCGGCGGCAATCGGTTCTTCTACCGCCGCATCGGTTTG
CGCCAACAGGAAAACCGGCTCCTCGCGGGTAATGGCGTTTTCCAGTGCGGCGATGGATTT
CGGTCTGCCGACAAACAGCGGCAGAACCATATGCGGGTAAACGACGACATCCCGCAAAGG
AAGGGTTGCCAAGGCGGCATATTCCTCAAAATGCTTTTCTTTTTGTGTCATAGGTACTCT
CTTGTGTCTGACAGATTGCCGATTTTCGCGTACATTGGGGTTGAAGGTATTATTTCAAGC
ATATGTGGTTTATTTATGGAGTTTGATGCGATGCCGTCTGAAACATTCCGGCTTCAGACG
GCATGGGCTTGGAAAGACAAGGCGGGAACAAAAAACTGTTCTGTGTTGCCGCTCCTTGCT
GTACCATCCGTATGGTTTGCGGTTCTGCCGCCCTATTTTCAAAACATGACGCAGGTATCC
CATGTCTTCTTTTATTTTTCCCGATGACGGGTTCGAATGGCGCAACGAAAGTCTGCAAAA
GCCGCCCAAAGGTTGGCATTATGTCCGCGAAAGCGGTGCAGACGGCATTTTGAAGGCTGC
CTATCAAAATATTGCAACTGTCTAGCAGGGCGATTTCCACAATGCCAAACAGGTGCTTTC
TGCAATGAAGAAGAGGGTTCGCAAACCTGCCGCCGTCCGTTCCGATGCGGATATCGCCGC
CCTTTTCCATGCCCACCGTATGAAGCAGGCGCAGCAGAGCCGTATTCTGAATATGCTTGC
CGTTGAAATCCGCCCCGGTTTTGTGTTGGACAACAAACGCGCGCCCGATATACGCTCCGC
TTTGCTCGACGTGTACGGAGAGGCGGACGGCAAACCGTTTTTCCTGCCGCTCAATCTGCT
GCTGGGGTTTATGGGTGCGCACGAGTGGCATAAGAAAGGGGTTGCCGTTCCGCAGCTGGG
CGGCAGCATACACGTTCCTTTCGGCGTATTCTCGCCGTTGCGCGGCGAATACCTCGACCT
GCTCGCCCATGCGCCGTCAACGGGTTTTCAGACGGCATTCGATATCGGGACAGGCTCCGG
CGTGCTTGCCGCCATTTTGGCGAAACAGGGCATTCCTTCCGTCATCGGCACGGATACCAA
TCCGAAAGCCGTCGCCTGCGCCCGTGCCAATATTGCCCGTTTGGGCTTTGAAAAACAGGT
TGAGATACGGGAAACCGATCTGTTTCCCGAAGGGTTTGCCGATCTGATTGTCTGCAATCC
GCCCTGGCTTCCCGCCAAGCCGACTTCCGCCGTCGAATCCGCGTTATACGACCCCGAATC
TGCGATGCTGGCTGCGTTTTTGCGGGATGCGCCGAAACATCTGAATCCCGACGGAGAAAT
CCGCCTGATCATTTCCGATCTTGCCGAACATCTGCACCTGCGTCCATCCGATTTTCTGGA
TAAGGCATTTGCTCAGGCGGGTTTGCGTGTTGCCGATATGATGAAAACCAAGCCGAAGCA
CAAAAAAGCCGCGAATCCGAGCGATCCGCTTGCTTTTGCGCGAACCCGGGAAACCACTTT
CCTATACCGTTTGAAAAAGGCATAAGGGGCGGCGGCGCGGCATTCGGGCGGATTATTCTT
GTGAAAATACCCGCTCGAGCATACTGCCCAATGCCGTCTGACGCGTTTTGACGGTGGCGG
CAGCTTGCCGGAAGGCTTCTTCCCCGCCGAAGAATACGAACTTCTCTCTCGCGCGGGTAA
TGGCGGTATATAACAGCTCCTTACTCAATCCGGACAATGCATCGTCCCCTTCGTCCGAAG
GTGCGGCGGAAGGCGGCAGCAGCCATACTTCCCGGTATTCCGAACCTTGGCTTTTGTGGA
CGGTCATGGCGAATGCGGGTTCAAATTCGGGCAGGCAGCTTACCGCTACCTTTTTAAATC
CGTCCGCATCGGCAAAATAGGCGGCAAGGCTGCCCTGCCGTCCGACATCTTCCATAATCA
GTCCGATGTCGCCGTTGAACAGTTCAAGCGCGTAGTCGTTCTGCCTGATCATAATCGGCT
CTCCGGCAAAATATGCCAAATGTTCCGGTATGTTCATTTTGCGGCGTACATGGCGGCAAT
AGGCTTCGTTGAAGTCTTCCGCATCCTGCCGCCAAGCTGCCAGAACCACGATATCCGAAA
TGCCCGCGTATGCGGCTTCGATATTGCCGTCTTTTACCGCCTGCCAATAGGCTTTGTGTG
CCCGGTACAACCTTTCGACTCGAGCGTTCGGACTGCATTCCGAATGTTCCAGTTCGTCCG
GAAACCGGTCAAACAATGCCCACGCCCCTTCATCGCCCGATACGGCGGCACGGGCAAGGC
AGCCGATGCCGCTGTTGTCGCCGAAGCGGTGGCTGAACGACAGATGGGCGGTGTTTTGCG
CCAACACGGGTGGATTTGCGCTGACGCTGAAACCGTGTTCCGGAAGGAAGCCGGCCAGCC
TTTGGTGCGTTTCTCCGTCCAAAACGGTTTTTTGTGACAAAACGGACAGCACCGCCCCTA
TTCCGACGGACGGGAGCTGGTTTTCATCCCCCAGCAGAATCACGCGCGCGCCGGTTTTGA
CCGCTTTTAAAAGTTGCAGCATCAATGCCGTATCCAGCATAGAGGCTTCATCGATAACCA
ATACGTCAAACGGCAGCGGGTAAACAGGGTTGAACGCCGCCTGCATTTTGGGCGGGCGCA
GCTTCAGCAGTCGGTGGACGGTTTGCCCTTCCAGTTTGAGCAAATGGCGGCGGACGGCCT
CCGGCGCGTCAAAACCGTTGATTGCACGGTGCAGCGCGCGCGCCATATGTGCCGCCGCTT
TGCCCGTCGGTGCGGCAAGCGCGATGTTGGGAAGATTTTCGTCTTCACCGCAAATCAGCG
CCAGCAGTTTGGCAACCGTTGTCGTTTTGCCCGTTCCCGGCCCGCCGGTAATCACCATAA
AAGACTGCAACAGTGCCAAGGCGGCGGCATCGCGCTGCCCTTCGCTGCCCGTGCCTTGAA
ACCATTTTGCGAGGTTTTGCCTCGCGCCTGCCGCGTCGGGGGCGGATGTGCCGGCTGCCG
CCAAGCGTTTTATCTCGGCAGCCAAATCGTATTCCAACTGCCACATCCTGCCCAAAAACA
GCCTTCTGCCTTCCAAAATCAAAGGCGCGGCGGATGTTCCGACAACGGGTGCGAGTGCCG
ACAGCGCGTCAGCCTCGCCACCGCTCAAACGGATAAACGAATGACCGTTTTGCAATGCCT
GAAACAGGCGTTCGGTGCAGTTTGCAAGCACTTCGTCGCCGGAACCCGCATAGTGTTCCA
AAAAACGGATTGCCGCCCTTGCCGCCGCTTGGGCAAATTCATCTGTCTGCCGTTCCATTT
TATTCCTTATCCAAATGCCGTCTGAAGGCGTGGGGCTTCAGACGGCGCGGTGTTGTTTCG
GCTGTTTAGGCGTTTGCGCCCTGTTTGGGGTGCAGGCTGCCGTCTTTCATGACCATCACG
CGCTCGAAGCGGCCGGCGAGTTCGTCGTCGTGCGTTACGACCACCAGCCCCGTTCCCAAT
TCTGTTTTCAGTTCCAGCATCATATCCAAAACATTCCTGGCGTTCGCACGATCGAGATTG
CCGGTCGGTTCGTCGGCAAGCAGGCATTTGGGTTGGGTAACCAAGGCGCGTGCAATGGCG
GCACGCTGCCGCTCACCGCCTGAAAGTTCGCCCGCGCGGTGCGTCGAACGGTGTTTCAGT
CCGACCTTTTCCAGCATCGCCATTGCCGCCTCCGCAGCCTCTTCACGGCTTTTTTGCCG

## Appendix A

ATCAGAAGCGGCATCATCACATTTTCCAGTGCCGAAAATTCAGGCAGAAGATGGTGGAAC
TGGTACACGAAACCGAGATGGCGGTTACGTAAATCGCCCAAACGCCGCTGGTTTAAGGTA
CGCAAATCCTCGCCCATCAGCAGCACCCTGCCTTCAGACGGCATATCCAGCCCGCCCAAA
ATATGCAGCAGCGTCGATTTGCCGCTGCCCGAAGAACCGATGATGCCGGTGCTTTCCCCT
GCGTGGATTTCCAAATCCAAGCCGTGCAGCACCCGAACGTCCAAACCGCCGTCCCGATAG
CGTTTGCCCACGCCTTCGCATTTCAAAATCAACTCACTCATAACGCAAAGCCTCCGCCGG
TTGGGTTTTTGACGCGCGCCGGCTCGGGTAGAGCGTGGCAACGAAAGACAGTCCCAAAGA
AATGCAGGCAATCAGGGCAACGTCGCCCATATCGACATCGCTGGGCAGGTAGTCGATAAA
ATAAACCTGCGAATTGATGAGGTGGACACCGAGCAGGTTTTCAAAAAACGCCACGACCCT
GCCGACGTTCCAACCCAAAAGCACGCCGCAGACCACACCCGCCAGCGTGCCGAAAAAGCC
TGAAAACGCGCCCTGCACCATAAAAATCTTCATCACGCCAGCAGGGGAAAGACCCAAAGT
CCGCAAAATCGCAATGTCCGCCTGCTTTTCCGTAACCGCCATCACCAGGGAAGAGACAAG
GTTGAACGCCGCCACAGCGATAATCAGCGTCAGGATGATGAACATCATCCGTTTTTCCAG
TTCGACCGCTTCAAAATAGCTGCGGTTGCTGTACGTCCAATCGCGCACCCAAACCGCGTC
CCTTTGCGCCTCCGGAATCAGTGTTGCCGTCAAGGCGGGAGCGTTTTGCGGATCGGCGAG
CTTCAGCCGCAGCCCCGCAACTTCCTTATCCAAACGGTACAGCACGCGCGCGTCTTGGAT
ATGCGTCATTGCCAATGAGTTGTCCACTTCGTAAACGCCCGTCTTAACCAGACCGACCAC
GGTAAACTGTTTCAACCTCGGTACGACTCCGGCGGGCGTAACATTGCCCTCCGGCGTGAT
GACGGTAACTTTATTGCCGACTTCCGCCCCCAAAGCCTCCGCCAAGCCGACACCGAGGAT
AATGTCAAACTCGCCCGGAATCAGATCTTCAAATTTGCCTGCCGGCATTTTGTCGCCGTA
TTCCACCACTTTGCGTTCTTCAGACGGCAAAATGCCGCGCATCTGCACGCCCCTGATTTC
GCCCGCATTGGCCAGCAATGCCTGATTGGAAACATAGGGCGCGGCAGCCAAAATACCTTT
GCGGTTTTCGGTAAACCGAAGCAGGTTGCGCCAATCCGTATCCGTATTATCGATATAGCC
GATTTCGGCGTGCGGCGCGACATTCAGGAGCTGCCCGCGTATTTCTTTCTGAAAGCCGTT
CATAACCGACAAGACGACAATCAGCGCGGTTACGCCCAAGGCGATTCCGGCAATCGAAAC
CATCGTGATAAACGACATAAAGCCGTTGCGCTTTTTCGCCCTGAGATACCTCAAGCCTAT
CCAAGCCTCTAGAGAAAACATAACGCTACCTTAAAAATGTCTGCAAACGTGCCGCCCCGG
ACGGCGGTTTGGGGGCGGCGGCAAAAGTTTATTGTACCGTAAAACCCAGGCAGCGTCCGA
ACGCCCGAGTGCGGGGGACGGGGCGGCAGAGCGGGCGGAAAACTTGCACAACGCGTCAAA
CTGCCCTATCCTTTCCTTCAGAAAAACCGTTTCTTGAGGAAAACAATGAATATCCGAACT
GCTTTTGCTTTGTGCGCCATCGCCTTATCCGCCGCTGCGGCTGCCTACGCCAAAGAAATC
AAAATCGATGCCAACAACACGCCTTATTCCGAAGCCGACGCGCAAAAGCTGGCGGCAACG
GCAGTCGGTATGGGTGTTAAGGAACCTATCAGCCTGAACGGCGGCAGCGGCAGCATTACC
GTGTCCGGCAGCAGCGCGACGCAGTGCGTGTTCAAAGTCGGCAACGGAGGCGCATTGCAG
ATTCAAGGGCTAAACTGCAAGTAAACCGCCCGGAAAAATGCCGTCTGAAGGCTTCAGACG
GCATTTTGCATTGGCGGCGTTATGCCCCGCCTTCTTTAATCAGGCGGCGTTCGTACACCG
CCTGCGCCAGCGTTCCCGCATCGACATATTCCAATTCGCCGCCCAAGGGAATGCCCTGCG
ACAGCCTGCTGACTTTGTAAGGCAGGTTTTTAAAAAACTCGGACAGGACATACGCCGTCG
CATTGCCTTCTGCGGTAAAAGCGGTTGCAATAATGATTTCTTCGACTTCCCCGCCGCCCA
GCCGTTGCGCCAGCCTGTCCAATGCGATGGCGGATACGTCCATTCCCAATGCCGTATTGA
TTTGCCCCATCAGGACGAAATACAGCCCGTCGTGGCAGTTTGCCGCTTCCATATTCGACA
CGTCGGCAGGCATATGCACCACCATCAGCCGCCGCCCGTCGCGTGTTTCATCGGCACAAA
TATCGCACAATCCGCCTTCGCAAAACGTGTTGCACATCGCGCAATGGTAAACCTGCTTCA
ATGCCGTCTGCAAGGCATCCACCAGTTTTTCAGCCTCTTTGCGCTTGTGTTGGAGCAAAT
GATACGCTATCCGCTGTGCCGATTTCGGCCCGACGTTGGGTAAAACCTTCAGCGCGTCGA
TCAATCCTTGGAAGGCATCTTGTTTTTTGTGGCTCATCATATTCCGCCGTATGGGAAAAC
GGCCGGAATATTCCGACCGTTATTTTGTCAACAAAAGTGTCAATTACTGACCGTCGCCGT
TGTCGACCGATTGCGCTCCTTTGGTCTGTTTGATTTTGCCGTTGAAATAACGTATCAACA
AGTCGAAAGTATTGGCAGACTGCTGTTGCGCCAAAGCCTGTTTTGCAAGCGGAAGCTGTG
CGGCGATATCATCCGGCGGGGTTACAGCCTGTACTTCGACAATCACGGGTGCCGGCAGAC
CGATCAGCCTGACGTAGGCGGGTTTGCCGTTTGCCGGTTTTGCTTTCAGCAGTTCCGCAT
AAGCCTCGGGCGGCATGGACTGCCTTGCCTGCTGTGCGCCCAAAACGGACACTTCCGACC
ATTTCACGTCAACAGCCTTGCCGCCGTTCAGTTGGGTAAGCACGTCTTTTGCCTTGTTTT
CGGCAAGTTTGGCGGCTTCGGTACGGATATAAGCCTGACGTACCGCGTCTTTGGCTTCGG
CAAACGGCAGGGTTTTCTCTTCGCGGACTTCTTTGGCGCGGACGACCCACGCGGTTTCGC
TGTTGATGGTCAGCACTTCGGAATTGTGTTTTTTCTTCAATACGTCGTCGCTGAATACGG
CATTGATCAGGTTTTCGGGCATACCGGACATTTGCGCGTCCTGCCTACTCAGCCAAGTTT
CTTGGGTTTCGACTTTCAAACCGCTGTTTTTGGCGGCTTCGGCAAGCGAGGAAGGATGGT
TGAACGCATCGTCGCCCAATTTTTCTTTTGCCTTGTTGAAGTCGGCAACCGCCTTTTTCA
TTTTCAATTCGTTTTCGACGGCGGCTTTTTCCTGCTCGAAAGAAGGTTTGGCTTCATTTG
CCGGCAAACGCGCCACGCGCTCTTCAAATGCATTTTTCACTTCCGTTTCACTGACGGTCT
GCTTGTCTGCAAAATCCTTCAGATTCAAGGCGACATATTCCAATTTGACCGCCTGCGGCA
GCAGATAGTCTTTTTTGTTCGCATTATAAAATTTCTGCAAATCGGCTTCAGACACTTTGA
CTTGGGCGATGAACTCGTCGGGGTTGAAAGTGTGCGAACGGATGGTGCGGTTGACCTGTG
TCAGCCTGATCAGCTGTTCCGCCTGCGCGTCGCCGACCAATACGCCGTTTTGGACGAGGT
TTACCAAATTCTGCAAGGCAAACTGATCGCGGATTTCTTCGACAAACTGGTCTTCAGACA
TATGGCGTTGGGAAAGGTAGCGGTTTAAAAGCGCGTGGTCGAATTTGCCGTTTGCGTCGT
GGAAATTGGGATCGTCCACGATAATTTGCTTGATTTGTTCGGAAGAAACCGAAATGCCCA
TCAGCTTCGCGCCCTGTTTCAGGTAGGCGCGTTGCAGCAGGGATTGGAACACCGCGTCGC
GCGAAGGGCCGCCGCCGTCCGCCTGTTCGTTCTGTATGGCGTTGTTGATGGAGTGGTCGC
TGATTTTTTCGTCGCCCACTTGGACGATGTAGTCGGCACCCGGATGGGATACCGTGCTGA
CCCCGAAGCCGACGAAGGTTAATGCAATCAGGCCCAAAAGGACTTGGGCGGGCGTTCTGT
ATTTTTCGATGGAATGGAACATATTTTAAATCGGGATATAGAATGGGAACGGAAATTCA
AGTCGGGTATTGTAACGGTTTTTATCCCTGTCTGCACGGGGCTTGCCGGTTGAAGATGCC
GTCGTAGGTTTCTTCGCTGAAGCCGGCGTAAACCTTGCCGCCGCACTCCAATACGGGACG

## Appendix A

CTTGATCAGGCTCGGCATTTCGGACATCAGTTTGACGGCCTCCGCCGTCGAGGACAGCAC
TTTTTGCTGTGTTTCGGCATCGAGTTTGCGCCAGCTTGTCCCGCGTTTGTTGAGCAGGGT
TGCCAAAGGCACTTGTTCCAGCCACGAGCAGATTTCCGCTTCAGACGGCCTCTGTTTTTT
AAAATCCCGAAATTCAAACTCCAAGCCGTATCCGGCAAGCCGGTTTTTGGCTTTTTTGAC
CGTATCGCAATTTGGGATGCCGTGAAGGACTATCATTTGGAAACCTTTTGTCTGAAATAA
TAAAACGGATATTTTACTATAAGTGTCTGAAAATTTGCCCGTCTGTTTCAGACGGCGGGG
CGGTTATGTTACAATCCGAAAATTCGAAAAATTTAATCTCTTGTTCAATAAAGGCTTTAC
CAATCATGATTTCTACCAACGGCATCACCATGCAGTTCGGCGCAAAGCCGCTGTTTGAAA
ACGTATCCGTCAAATTCGGCGAAGGCAACCGTTACGGCTTGATCGGCGCCAACGGCTCAG
GCAAATCCACCTTCATGAAAATCCTCGGCGGCGATTTGGAACAGACGGCCGGCGAAGTGG
CGATTGAAAACGGCGTGCGTTTGGGTAAATTGCGCCAAGACCAGTTTGCCTACGAAGACA
TGCGCGTGCTGGACGTGGTGATGATGGGGCATACCGAAATGTGGGCGGCGATGACCGAAC
GTGATGCGATTTACGCCAATCCCGAAGCCACCGAAGACGACTACATGAAAGCCGCCGAAC
TGGAAGCCAAGTTCGCCGAATACGACGGCTACACCGCCGAAGCGCGTGCCGCCGAACTGT
TGAGCGGCGTGGGCATTTCCGAAGATTTGCACAATGCGAAAATGGCGGAAGTCGCCCCGG
GCTTCAAACTGCGCGTATTGCTGGCGCAGGCGCTGTTCTCCAAGCCGGATGTATTGCTCT
TGGACGAACCGACCAATAACTTGGACATTAATACCATCCGCTGGTTGGAAGGCGTGTTGA
ACCAATACGACTCCACGATGATTATCATCAGCCACGACCGCCACTTTTTGAACGAAGTCT
GCACGCATATGGCGGATTTGGACTACAACACCATCACCATCTATCCGGGCAACTACGACG
ACTACATGCTCGCCTCCGCCCAATCGCGCGAACGCGCCCTGAAAGACAATGCCAAAGCCA
AAGAGAAACTGCAAGAGCTGCAAGAGTTCGTCGCCCGCTTCTCTGCCAACAAATCCAAAG
CCCGTCAGGCAACCAGCCGTCTGAAACAGGCCGACAAAATCAAATCGGAGATGGTCGAAG
TCAAACCTTCCACCCGTCAAAACCCGTATATCCGTTTTGAAGCCGATGAAAAAGCCAAGC
TGCACCGTCAGGCTGTGGAAGTTGAAAAACTGGCGAAACGCTTTGAAACCCAGTTGTTTA
AAAACCTGAACTTCATCCTTGAAGCGGGACAACGCCTCGCCATCATCGGCCCGAACGGCG
CGGGCAAATCCACCCTGCTGAAACTCTTGGCCGGCGCGTACAACCCCGAATATTCAGACG
GCCTGTTGCCGGACGAAGGCACCATCAAATGGGCGGAAAAAGCCAGTGTCGGCTACTATC
CGCAAGACCATGAAAACGACTTCGACGTCGATATGGACCTGAGCGAATGGATGCGCCAAT
GGGGGCAGGAAGGCGACGACGAACAAGTCATCCGCGGCACTTTGGGGCGTTTGCTCTTCG
GCAGTAACGATGTCGTGAAAAAAGTGAAGGTTCTCTCCGGTGGTGAAAAAGGCCGTATGC
TTTACGGCAAACTGTTGCTGTTGAAACCCAATGTCTTAGTCATGGACGAACCGACCAACC
ATATGGACATGGAAAGCATCGAATCCTTGAACATGGCACTGGAAAAATACAACGGCACGC
TGATTTTTGTCTCCCACGACCGTCAGTTCGTTTCCTCCTTGGCAACCCAAATCATCGAAC
TGGACGGCAAAGGCGGATATGAACACTACTTGGGCGATTACGAAAGTTACTTGGAGAAAA
AAGGCGTAGCATAACCGCCGGTTGGAACAATGCCGTCTGAAGCCGCTTCAGACGGCATTG
TTGATAACTTTAAAATAGGAAGCATATGCAGACTTATCTCGTCGGCGGTGCCGTCCGCGA
TTATCTTTTGGGCTTGCCCGTCAAAGACCGCGATTGGGTGGTCGTCGGCGCAGACGCACA
AACCATGCTGGCGCAAGGCTTCCAGCCGGTCGGCAAAGATTTTCCCGTGTTTCTCCATCC
CGAAACACACGAAGAATACGCCCTCGCCCGCACCGAGCGCAAAACCGCCAAAGGTTACGT
CGGTTTCAGTTTCCACGCCGACAAAGACGTTACGCTGGAGCAGGATTTGATGCGCCGCGA
CCTGACCATCAACGCGATGGCGCAAGATGCGGACGGCAAGATTATCGACCCTTTCGGCGG
ACAACGGGATTTGGCGGCAGGCATTTTGCGCCACGTTTCCCCAGCCTTTGCCGAAGACCC
CGTCCGCATCCTGCGTACTGCCCGCTTTGCCGCGCGTTACAAGTTTGAAATCGCCGAAGA
AACCATAAAGCTGATGCGGCAGATGGTGGAAAACGGCGAAGCGGACGCATTGGTTGCCGA
ACGCGTCTGGCAGGAGTTTGCGAAAGGTTTGATGGAAAAAAATCCGCGCAAAATGATTGA
AGTGTTGCGCGAATGCGGCGCGCTCAAAGTCTTGCTGCCCGAAGTCAATGCCCTCTTCGG
CGTGCCGCAACGCGCCGACTACCATCCCGAAATCGACAGCGGCATCCATACCCTGATGAC
GCTGCAACGCGCCGCCGATATGGGCTTGAGCCTGCCCGAACGCTATGCCGCCCTGCTGCA
CGACTTGGGCAAAGCCAAAACACCGTCCGACATCCTGCCGCGCCACCACGGACACGACCT
CGCCGGTGTCGAACCCGTGCGCGAAGTCAATCAGCGGCTGCGTGCGCCGAAACATTGCGC
CGAGCTTGCCGAATTGGTTTGCCGTTGGCACATTATTTTCCACCAAGTCGGACAGCTTAA
AAGCCAAACCATTCTGAACGTTTTGAAAAAAACCGACGCTTTCAGACGACCCGAACGCTT
TCAGACGGCATTGAACGTCTGCATTGCCGACACGCAAGGCCGTCTGAACCGCGAACACAC
GCCCTACCCGCAACGCGCGCACTGGCTCGCCTTACTCGAAGCCGCCAATCAGGCGGATTC
GGGCAAAATCGCCGCCGAATGCCGCGCACAGGGAAAAGCGCACCTTATCGCCGAACAAAT
CGACCGGGCGCGGCTGGCACAAATCGCCCCATTGCAAAAAGCGTTTCGAGCGGCGCAAGA
CAAAACAGAAAAACATTAAAACGTCCAATGCAGCCACTTTTATAGTGGATTAACAAAAAT
CAGGACAAGGCGACGAAGCCGCAGACAGTACAGATAGTACGGCAAGGCGAGGCAACGCTG
TACTGGTTTTTGTTAATCCACTATAAAGTTTTGAGGACGATACCCAATCCAAGCTTTGCA
ACAGCCGCCGCCATATCCGCTATAATTCACGCTTCAGCCATTCCGCCCCCGACATAAAAT
CATGACCCTGAAAACCGATTTATTGCCTAAAATCAACAACGAAGATTATCAACGCCTCAT
CCTCAAACACAGTGCGGAATTTAGCGGTGGCGAAATCCGCCTGTTGAACGAAATCCTCGA
AAAATTCAATTTCGACGTTGTTCAGGCGCAGGCATTGGCGCAAGCCGTCATGCAGCAAAT
CCGCTTCGACCCCAACGCCTACCACATCGACAGCGACGACGAAGACACCACCGGCATCTG
CCCCCACTGCATCAACCCGCCTATGCCGCCCCTGCGCGACTATCTCGTTTGGCGCGAAAC
CCGCGGATAAAACGCTTTTGACCGTTATCTTTTCAATGCCGTCTGAAACGCCGCCGACCG
TTCGGACGGCATACCCGACAAAGGGAACACTATGCTGCAAACCGACAACCTGACCGCCGC
GCAACCGCAACGCATCGTTGCCGCCCAAACCGCCTCCGCACAGGAAGAACTGCTCGAACG
CGCCCTCCGCCCCAAAACGCTGGACGACTACATCGGGCAAGACAAAGCCAAAGAACAGCT
TGCCATTTTCATCCAAGCCGCCAAAAAACGCGGCGAAGCACTCGACCACGTTTTGCTCTT
CGGCCCGCCCGGACTGGGCAAAACCACACTGGCGCACATCATCGCCAAAGAATTGGGCGT
AAATTTGCGCCAAACCAGCGGCCCCGTCCTCGAACGCGCAGGCGACCTCGCCGCCCTTTT
GACCAACCTTGATCCGCACGATGTATTGTTCATCGACGAAATCCACCGCCTCAGCCCTGT
TGTCGAAGAAATCCTCTATCCCGCGCTCGAAGACTACCGGCTCGACATTATGATAGGCGA
AGGACCCGCCGCCCGTTCCGTCAAAATCGACCTGCCGCCCTTCACGCTCATCGGCGCGAC

## Appendix A

CACCCGCGCCGGTATGCTGACCAATCCGTTGCGCGACCGCTTCGGCATCGTCTCCCGCCT
TGAGTTTTACGAAAACCGAGACCTTACCACCATCGTCAGCCGTTCGGCACAACTGTTGCA
GCTCGATATGTCCGAAGAAGGCGCGGAAGAAATCGCCAAACGCAGCCGCGGTACGCCGCG
CATCGCCAACCGCCTGTTGCGACGCGTGCGCGATTTCGCCGACGTGAAAAACAACGGCAC
AATCGACGGCGGCATCGCCGATGCCGCTTTAAGTATGCTGGACGTGGACGCGCAGGGGCT
GGACGTGATGGACAGGAAATTTCTCGAAGCCGTTTTGCACAAATTCGGCGGCGGCCCGGT
CGGTTTGGACAATGTTGCCGCCGCCATCGGCGAATCTACAGACACCATCGAAGACGTTAT
CGAACCCTACCTTATCCAACAAGGCTTCCTGCAACGCACCCCGCGCGGCAGGATGGCGAC
CGAACGCGCCTACCTGCATTTCGGGCTGCCCGTCGAAAAATAACGCAATGCCGTCTGAAA
CAGAGCTAATTTTCAGACGGCATTTCTATTTCAATCATTGGCGCAAGGTTCAGCCTGCCG
CTTTTTTCCAGTTCCGCCCTCATCGCATCAATCACCGCCTTATAGTCTGGTTTGCCGAAA
ATCGCAGAACCGGCAACAAAGGTATCCGCACCAGCTCGGGCAACGGCGGCAATATTGTCG
GTTTTGATGCCGCCGTCCACTTCGATGGCGATGTGCCGCCCGCTTTGTGCTTCGTACCGA
TCCAGCATCGCCCGCACCCGGCGGATTTTTTCAAGGGTGTGCGGGATGAAGCTTTGTCCG
CCGAATCCGGGGTTGACCGACATCAGCAAAACCATATCCAGCCTGTCCAATACGTTTTCC
AACAGATATACGGGCGTTGCCGGATTCAACACCAGCCCCGCCTGACAGCCCATATCACGA
ATCAGGCTCAAGCTGCGGTCGATATGGCGGCTCGCCTCGGGATGGAACGTGATGATTGAT
GCTCCTGCTTTGGCAAACGACTGAATCAGGTCGTCAACGGGTTCGACCATCAGATGCACA
TCAATCGGCACGCTTGCATAAGGCTTCAACGCCGCGCAAACCATAGGGCCGAAGGTCAGG
TTCGGCACATAATGGTTGTCCATCACGTCAAAATGGATCAGATCTGCACCTGCCGCAATG
ACGCTTTCCACCTCTTCTCCGAGGCGGGCAAAGTCTGCCGATAAAATGCTGGGTGCGATA
CGGTAAGTAGTCATGTTTTTCCTTCAATATCCTTTTATAGTGGATTAACAAAAATCAGG
ACAAGGCGACGAAGCCGCAGACAGTACAGATAGTACGGAACCGATTCACTTGGTGCTTCA
GCACCTTAGAGAATCGTTCTCTTTGAGCTAAGGCGAGGCAACGCTGTACTGGTTTTTGTT
AATCCACTATACGTTCCGATTCCGCCGTTATGTCTGCCTGCCGGACATACGTACGCATTA
TTAACAAAAGTTAACCGCGATAATACCATCTTTCACACGTCAATCTAGTATATTTCCTAA
AATTTCCAACAAGAGGAAAAGCCGTGCCACTGCCTGCTCCCTGCCGTTTTGCCAAACCTG
CCGCCTCTTTTTTAAGTATGGCTTTGCTTTCCTGTCAGCTTTCCCACGCCGCCACGGCTT
ATATCCCCCCGAACGATTTTCAACCGAACTGCGACATACGCCGACTCGGGCTGACCCAAA
GTCAGCACAATGAGCTGCGTAAAATCCGCACCGCCTTCAAAATGGCGGGCGACAGGGCGC
GTTTGAAGGTTATGCATTCCGAACACAGCCGCCGCCGGTCTGTCGTCGAAATCATTTCCT
CGGATGTTTTTAATCGGAACGAGGCGCGCGATTATGTCGAAAGCCGCTATTTGTCCGGTA
TGGATTTTGCGGTGGACGAATTGGAAATCCAACACCGGTTCTTCCATATCCTCACACCGC
AACAGCAGCAAATGTGGCTTTCTTCCTGCCTCAAATAATCCCCGAAACGCTCACAACGCC
CGTTGTTTCGGCAGCCTGCCCGCCCAGTCGCAGGCAAACTGCCACGCGGAACGTCCCGAA
CGGCTGCCCCGCGTCTGCGCCCACTGCAATGCCGCCATCTGTGCGGTTTCATCATACGGC
ACGCCGAAATCTTCCAGCCAATTTTGCACCGCCGCCAGATAATCGTTTTGATCGAACGGA
TAAAAACTGAGCCACAATCCGAATCGGTCGGACAGGGATATTTTTTCTTCCACCGCTTCT
TTCTGATGGATTTCCCCCCGCATCCCCGTCGTACCGGCATTCTCGTCAAAATATTCGGGC
ATCAGGTGCCGTCTGTTGGAAGTCGCGTAAACCAAAACGTTGGCGCAACGTTGAGACAGA
CCGCCGTCTAACGCGGTTTTCAATGCCTTATAGGTTTCATCGCCGCTTTCAAACGACAAA
TCGTCGCAAAATACGATAAATTTTTCCGGACATTCCTTCAAAAGCGTCAACAGGTAAGGC
AGGCCGATTAAATCGCTTTTATCGACTTCGATCAGGCGCAATCCCTTATCCGCATATTCG
TGTAGCAGGGCTTTGACCAGCGAGGATTTGCCTGTTCCGCGCGCGCCGCTCATCAATACA
TTGTTCGCGGGTCTGCCGACAATGAACTGTTCGGTATTACGCACCAGCAATTCGGTTTGC
CTGCCGACTCCCGCCAGCCTTACCAAGGGAAAGGTGTGCGGATCGGGCAAGTGTTCCAAA
AAACCTTTTTTGCCCGCACTCTGCCAGCGGAAGGCAAGCGCGTTCCAATCCGTATGCCCG
GGTTCGGGCGGAAGCACGGCATCCAAACGCCGCAAAACGGCATAGGCTTTATCGAGAAAT
TCGTTCAATTCCATCTCTGCCTCACTTTGCATATCTTTGCGCCATCAGCCGTTCGACGGT
ATCGACGATTGCCTGCGTATTCGGATCGATTTCGATGTTGATCCTGTCGCCGACCTTTCT
GCTGCCGAACAGCGTCCGTTCCAAAGTTTCGGGAATCAGATGGACATTGAAACGGCCGTC
TTCGACTTTGCCTATGGTCAGGCTGCAACCGTCCAAGCCGACGAACCCTTTGGTCAGGAT
ATAGGGTTTGAGTTCATGCGGGAGCGAAAACCAAACCGTGCGGTTGAACCCGTCCCGTTC
GATTTCGACAATAGGCACGGTTGCCATAATGTGTCCGCTCATGACGTGTCCGCCGATTTC
GTCTCCGAAACGCGCCGCCCGTTCGATGTTGACGCAATCGCCTTCCTTCAGCAGCCCCAA
ATTGGTTTTTGCCAAAGTTTCCGCCATTAAATCGAAACTGACGCGGTTTCCTTCGATTTC
GGTAATCGTCAGGCAGCAGCCGTTATTGGCGACCGATGCGCCGCGTTGCAGATTGTCCGC
CGCCTCTTGCGGAAGCTCGACGACATAAGTGTGAAATGCCTCCGACGGGCGGTGGATTGC
CGTCAGTTTTCCCAATCCTTGAACAATGCCTGTAAACATAATCCTGTTTCCCTGTGTCGG
TAAAAATGGTGCAAATTGTAGCATCTCCCCGCGAAAAATGCCGTCTGAAATGCCTTCAGA
CAGCATTATGCCTCCGATTCGGGCAAAAACCGCCCGGTATGGCTTGACCTTTCCTTTCCA
CGCCGGTCGGCGGTCTTGCCCTTATCCCTCCTGCAAATCGATTTGCGTGTTCAAGTCGGC
AAAATGCCCGTCAAACTCGAATCTGACCGGCCGCGCCCTTTGCTGCTGCAACCAGCTTCT
TAATGTTTTCATACCCGAAAACAGATAGGGAATCGCGCTTTGCAGAATCTGCGGGCGGAT
ATACATAATGTTGTAGTGCATCGTTATCGGCGTTTCCACATAATACGCATTGCACAACGG
TGTGCGTTTCGACACGGTTTCAAACCTCGCCACCAAATCGTCCGGCAGATACGGCATATC
GCACGGCACAACCAAAAGCCAGTCAGCAGCCGCCAACTGCAAATCGTTGGCTGCGGTACA
CAATGCCGAAAGCGGGCCGAAATGCTGCCACTGCCGCGCATCGGGAAAAATATGCGGACT
TCTTCGAGCATATTCTTCCAAATTCCGGTTGGTGCTGATGGCGATATGGCTGACCTGCGG
CCTGACCCTGTCGATGACATGGTCTATCAGTGCCTTACCCCCAAAAGAGCAAGCCCTTTG
TCCTCGCCTCCCATACGGCTCGCCTGACCGCCGGCCAGTATCAGGGCAAAAGTTTTCATT
GCGGATGTTCTCTTGGAAAAGTTCGAGGTTTTCATGATTGCAGTCTGCCGTTCCCAATGA
CTCAAAATGCCGTCTGAAGCAGACGGCAAATAAATTCATATTATCTGAATTTTATCATAA
CATGATTTAACACTGAAGCGGTGCGGATTAAGTTTTCCTTAACCAATTCTTTCTGAGCAG
TTGTATCTAATTCCAAAGAATGATATTGTTTGCATTATTTGGAACAATTTTTCGCCGAGC

## Appendix A

ATGATACTGCCAGCCCGTTTTTCAGACGGCATCAGCCTTTCCCTGCGCCTGAAACTCCTG
ACCGGACTGTGGGTCGGGTTGGCGGCATTGTCTGTCGTTTTGACACTGCTGCTCTCTTTG
CGTCTGGAAAACGCGGCCTCCGTCATCGAAGAGGCGGGCAACTTGAGAATGCAGGCATAC
CGTCTGGCATACATGGCGGGTGAAGGCTCGCCCCGTGCGCAAATTGACAATCAGGTTGCC
GAATTTGAAAAAAGTTTAAAACGCATTGCCCAAAGCGATGCCATCCATCCGCTGATTCCT
TCGGACACCCCTCTTGCTTATGATTTGATACAATCCATGCTGATTATAGATTGGCAGGCA
CACATCCTCCCCCCGCTCCAGTCCTACCGGCGACCGACTCAGGTCGATCTCTACCGCTTT
GCCGGAAACATCGAACTGTTTTTGCAGGCATTGGAAAATGCCAACGAAAAAAACACATGG
TGGCTCAGGCGTTTTCAATGGGCAATTATGTTGATGACGCTGGTGTCGTCTGTACTGATG
CTGTTTTGGCACCAGATTTGGGTTATCCGGCCGCTGCAGGCGTTAAGGGAAGGTGCGGAA
CGCATCGGACGGAGGTGTTTCGATATTCCGGTTCCCGAAGGCGGTACGCCGGAATTCAAA
CAGGTCGGGCGTTGTTTCAATCAAATGGGCGGCAGGTTGAAAATTTTATATGATGATTTG
GAAGGACAAGTCGCCGAGCAGACACGCAGTCTCGAAAAACAAAATCAAAACCTGACCCTG
CTGTACCAAACTACACGGGACCTGCACCAATCCTACATACCGCAACAGGCTGCAGAACAT
TTTCTAAACCGTATCCTGCCCGCCGTAGGAGCAGATTCCGGCAGAGTTTGTTTGGACGGC
GGATCCGATGTTTATGTTTCCATTCATCATGCGGATTGCGGCACAGCAGCTTCGGATTTG
GGGAAGTACCATGAGGAAATCTTCCCCATTGAGTACCAGAACGAAACATTGGGCAGGCTG
TTGCTCAGCTTTCCAAACGGCATTTCTCTTGATGAAGACGACCGCATCCTGCTTCAAACA
CTAGGCAGGCAATTGGGCGTATCGCTTGCCGGCGCAAAACAGGAGGAAGAAAAACGCCTG
CTTGCAGTATTGCAGGAACGCAACCTGATTGCGCAAGGATTACATGACAGCATCGCACAA
GCATTAACGTTCCTAAACCTACAGGTACAGATGCTGGAAACCGCCTTTGCCGAAAACAAA
CGGGAGGAAGCCGCAGAAAACATCAGCTTTATCAAAACAGGCGTGCAGGAATGTTATGAA
GATGTCCGCGAACTGCTGCTCAACTTCCGTACCAAAATCAGCAATAAAGAATTTCCCGAA
GCCGTTGCCGACCTATTCGCCCGCTTTACGCAACAAACCGGGATAACGGTCGAAACCGCC
TGGGAAAACGGTTCGTTCCTGCCGCCTCAGGAAGCGCAGCTCCAAATGATTTTTATCCTG
CAGGAAAGCCTGTCCAACATCCGCAAACACGCCCGCGCCACCCATGTAAAATTCACCCTT
TCCGAACACGGCGGACGCTTTACCATGACCATCCAAGACAACGGACAAGGTTTCGACACG
GAGAAAATAGGAGAACCCACGGGCAGCCATGTCGGACTGCACATCATGCAGGAGCGTGCC
AAACGCATCCATGCCGTTTTAGAAATCCGTTCCCAAGCTCAACAGGGAACCACCGTCTCA
TTGACGGTTGCATCTGAAGAAAGCTTGAAATGACTATTAAAATTATTCTGATAGACGACC
ATACCCTCTTCCGCAGCGGCATTAAAGCCCTTTTGTCGCGCCAACACGGTTTTGAAGTCA
TCGGCGAAGCCGCAGACGGCCTCTCGGGTATCAAAATGATCAGTCGGCTGCAACCCGATG
TCGTCCTGCTTGACCTTGATATGCCCGGTATGAACGGACGCGAAGCACTCTCCCAAATCA
TCAGCATCAATCCGCAGCAGGCAGTGATTATGCTGACCGTTTCCGAAGACAGCGACGATT
TGACCGAATGTATGCGCATCGGCGCGCGCGGCTACCTGCTGAAAAACATCAACGCCGACT
TTCTGCTCGAAAGCATACGCAAAGCCGCTGAAGGCGATAATGTATTCTCGCCCGAGATGA
CCGCCAAACTCGTCAAAAGCCTGATTTCCCCCCAACCTGCCCAAGGGACGCAGGCACTCT
CCTCACTTACCCCTCGTGAACTGGAAATCTTGGGCTATCTCGCCGCAGGACACAGCAACA
AAATCATCGCCCGCCACCTCGATCTTGCCGAATCCACCGTCAAAGTCCACGTTCAAAACC
TGCTCCGCAAACTCAACCTCAGCAGCCGGGTGCAGGCCGCCGTTTACGCCATCCGGCACA
ACGTCCCCCAACCTGTGCCGGAATAGGCGTTCAGACGGCATATTAGGGGTTTAATCCCC
GTACGGTCATTCGGATAACAGACCAAGCATGTAAGTTTATGCCCCCATAAGTACGCTTGG
CATAGCAGTAATATTGTTCGGTTTAGTGTTTTCCGTTTGCCCCTATCTGATACTGCAATA
TCAGCTATGCCGTCTGAAAACGCATCATCATGATATTTTCAGACGGCATAATAAAAAGCG
GAAATACTAATGCAGGGTAAAATGTTCCATTCCGAATCCCATAAATATACAATGGCTTAT
ATCGTTTAAGCATGTGTAACCCACCCTCATATCAATCAATATATAGTGGATTAACAAAAA
CCGGTACGGCGTTGCCTCGCCTTGCCGTACTATCTGTACTGTCTGCGGCTTCGTCGCCTT
GTCCTGATTTTTGTTAATCCTTGGATTCGGATTTCAAGTGCAACACTAGTGTATTAGTGG
TTGGAACAGATTCAAGAATAAAACACTTGGCGTTTCGTAGCCAAGTGTTTTTCTTGGTCG
GTGGTTCAACTCATCTTGAACCCTGCGTATCTCCCGATCACTGATGTTACGGAAATCGGT
TTGTTTGGGGAAGTATTGCCGGATGAGTCCGTTGGTGTTCTCATTCAGCCCTTTCTCCCA
AGAATGGTAAGGGCGACAAAAATAAGTCTCCGCTTTCAATGCTTTGGTTATTTTGGTGTG
TTGGTAGAACTCTTTGCCGTTATCCATGGTAATGGTGTGCACCCTGTCTTTATGTGCCTT
TAATGCCCTAACAGCTGCCCGGGCAGTGTCTTCGGCTTTGAGGCTATCCAATTTGCAGAT
GATGGTGTAGCGGGTAACGCGTTCGACCAAGGTCAATAATGCGCTTTTCTGTCCTTTGCC
GACAATGGTGTCGGCTTCCCAATCGCCGATACGGGATTTCTGGTCGACGATAGCGGGTCG
GTTTTCTATGCCGACACGGTTGGGTACTTTGCCTCTGGTCCATGTGCTGCCGTAGCGTTT
GCGGTAGGGTTTGCTGCATATTCTGAGATGTTGCCACAACGTGCTGCCGTTGCTTTTGTC
TTGGCGAAGGTAGCGGTAAATGGTGCTGTGGTGGAGCGTGATCTGGTGGTGTTTGCACAG
GTAGGCGCATACTTGTTCGGGACTGAGTTTGCGGCGGATAAAGGGGTCGATGTGCTGAAT
CAGCTGCGAATCGAGCTTATAGGGTTGTCGCTTACGCTGTTTGATAGTCCGGCTTTGCCG
CTGGGCTTTTTCGGCGCTGTATTGCTGCCCTTGGGTGCGGTGCCGTCTGATTTCGCGGCT
GATGGTGCTTTTGTGGCGGTTCAGCTGTTTGGCGATTTCGGTGACGGTGCAGTGGCGGGA
CAGGTATTGGATGTGGTATCGTTCGCCCTGGGTCAGTTGCGTGTAGCTCATGGCAATCTT
TCTTGCAGGAAAGGCCGTATGCTACCGCATACTGGCCTTTTTCTGTTAGGGAAAGTTGCA
CTTCAAATGCGAATCCGCCATCCTCTATAAAAATGCCGTCCAAACCCATGTTTGAGACGG
CATTTCGCTATAGAAGCAATCAGGCAACCTGGGTTTGATGCTCGTCTCCCTGACGCTCAC
GGATCAAACCTAAACGGTAAACTGTTTCACCTTGTTCACCCAAGAGACCCTGAACCGCAT
CGGCATCTTCGGCAGCAACAATAACGACCATGCCGATGCCGCAGTTAAAGGTTCGGTACA
TTTCTTGGGTTTCCACATTGCCCGCCTTTTGAAGCCATTGGAAGAGCTTGGGCAATTCCC
ACGATTTAGCATCGATTTGTGCAACCGTGTTTTCAGGCAACACGCGCGGCACGTTTTCGG
TAATGCCGCCGCCGGTAATGTGTGCCATACCTTTAATGGTAAATTTTTCCAAAGCGGCAA
GAATCGGTTTCACATACAGACGGGTCGGCGCAATAACAGCCTCCCGCAAGGTTTTGCCAT
TATCAAACTCGGCATCCAGATCGGGATTGTCGCGTTCGATGATTTTACGGATAAGGGAAT
AGCCGTTTGAATGTGCGCCGTTGGAAGCCAAACCCAATACCACATCGCCTACGCCGATGC

## Appendix A

TGCGGCCGGTAATGACATTCTCTTTTTCCACCACGCCGACGGCAAAACCCGCCAAATCGT
ATTCTCCGACGGGATACATACCCGGCATTTCGGCAGTTTCCCCGCCAATCAGGGCGCAAC
CGGATTCTTCGCAACCTTGGGCAATGCCTTTAATAACATCGGTCGCGCGCGGAACATCCA
ATTTACCGCAGGCAAAATAGTCCAAGAAAAACAAGGGCTCAGCCCCTTGAACCAAAATAT
CGTTGACACTCATTGCAACAAGGTCGATGCCGACCGTATCATGTTTATCCCAATCAAAGG
CAAGCTTGAGCTTGGTACCCACGCCGTCCGTACCGCTGACCAATACGGGATTTTGATATT
TCTTGCCGATTTCGACCAATGCGCCAAAACCGCCCAAATCCCCCAATACTTCCGGGCGCA
TCGTGCGTTTGGCAAACGGTTTGATGTTTTCGACCAGTTGGTCGCCTGCGTCGATATCGA
CACCTGCATCGCGGTAACTCAATGAAGTACTCATCGTTTTTCCTTGGTAAATGGGGATTG
GACGGTAAAATAACGGGGCGTATTCTACCTTATTTCACGTTTGCAGGTTCAGATTTTTAG
ACAATATTGTAAACAGTCCGCCATATGCCCGCGCGTGTCGGGTTTGGCGGGACCGTCCGC
AGGATTAACGGGCAGAAACCCGCCTGCCCTTCCCCTCAATTCCTTATATATCGCGTTCCA
TCAAAAGACGCATTGCTTTTCTTAACCATTCCTTTTGGCAGACGAGCGGAAGGGGTTTTT
TGATGCCATCATCAAAATCAATATTTTCTTCTTTCCGGTTGAAACCCCGGCATTAGGGGT
GGTGAATCTGATTGCGTGCGGAAGCACCCGTTTCCGATTCGGTGCGGAACAAATGGCGGC
ACTTTATGTACCGTTCTGCGTGTTGAAACATATAGGCAGATAAAAAAGCCGCCCGCTGAA
AAGCAGACGACTTATGTTTTGTGGCACTAATTTGTCCCGATAAGCATTAACTATATAATT
TATTTATCATTATTGGTGCGGACGGAGAGACTCGAACTCTCACACCTCTCGGCGCCAGAA
CCTAAATCTGGTGCGTCTACCAATTTCGCCACGTCCGCATGGGAATTGGACGATTATACA
GATTTTGTTTTTTTGTGCAAGGTTTTCGGCGGGGCTGTTGATGGCTTGGGGTTTGGGGCG
GTAAAATCTGTTTTTCGTCCGCCTGACATCGGAATCGGGCGGTTTTTTGTTTTTATTGAC
GGAATTTGGGTATGCCTGCTGCTTTGATTAAGGATTTTCTGCTGACTCAGGGTTTGAAGC
TGCCGCTTGACGAGGTTCGGGCGGCGTATCTGACGGCGCAGACGGTAATGGATATGGGGA
CGGCTTCGATAGACCGTTCGGTTTTGTGGCGCAGTGATGAGGGTTGGAAACTTGCCGATT
ACCTGTCGTGCGACAATGTCCGCGAAGATGCACTGAAACGGCTTTTCATGGCTTTGGATT
CGGTGTTTTCGCGCTCGACAGGCGTGCGGAGTGCGGCGGTCTATGCCTTGATGCCATCTG
AAAACCAGGCTTTCCAACTGATATGCCTGTCCCGACAGGGCGAGGTTTTGGAAAACCTGT
GGGATTTGGATGAAGCGGCAGGCAAGGTTTCGCTGGCTTGCCGTTCGGCGCAAAGCGGTT
GGATGAATGTTGCCTCGGATGTACGCCGTTGGTTGGATTTGGGGGAGCTTTCGGGAGAAC
GCAATCATGCTTCGGCGGCGCAAATTTCCATTCCGGTCTGCACGGAAAGTGGCGGTGTGT
TGGGCGTGGTTCATGTGGAATTTGAATGCGCAGAGTGTGCGGGTACGGCAGCACAGGTGG
AATGGGTGGCTCTTGCCTTGGCTTTGTCCGAACCTTTGAAACTGCTGTTGGGTATGTCTG
CCGGAAAAGATGGGAGTGAAGATGTCTGAAATGTTGAACCATGTGGCATCCTGCCGCCTG
CCGACCGAATGGGGCGTATTTACGATGCACGGCTTTGAAGAGGCAAACGGGCAGGAACAC
GTCGCGCTGACCGTCGGTAATTTTTCAGACGGCAATCCGGTTCTGACGCGCATCCACTCC
GAATGCCTGACGGGCGATGCGCTGTTCTCGAGAAAATGCGACTGCGGACCTCAACTTGAA
GCGGCCATGAGGGCGGTACAGACAGAGGGGCGCGGCATCATCGTCTATCTGCGTCAGGAA
GGACGCGGCATCGGGCTGATTAACAAAATCCGCGCCTATCATCTGCAAGACCAAGGTATG
GATACGGTTGAAGCCAATTTGGCACTCGGGCTGCCCGTCGATGCCCGCGATTTCCGTTTG
GCGCAATCTATCTACGAATATCTGGGCATCCGCTCGGTCAAACTGCTGACCAACAACCCC
GAAAAAATCCAAACCCTGAAAGATGCGGGGATTAACGTGGTCGAACGCATCCCCCTGCAC
GTCGGGGAAAACCTTGAAAACAAACGCTACCTCCAAACCAAAGCAGACAAGCTGGGACAT
CTGATGTCGGAATAAGGCAAAGTTGCAGGGAACGGGCATCCTGCGCCGCCTTTCGGGAAA
CAGGTTTCCATACCTTGATAAAGCAATAAGTTTTATAGTGGATTAACAAAAACCAGTACA
GCGTTGCCTCGCCTTAGCTCAAAGAGAACGATTCTCTAAGGTGCTGAAGCACCAAGTGAA
TCGGTTCCGTACTATTTGTACTGTCTGCGGCTTCGTCGCCTTGTCCTGATTTTTGTTAAT
CCACTATATAAAGTTACAGGGTGCGGATGCAAACGCATTGCGAGCGCGGGTTTGAGGCAT
ACGCGCAAACATCTTAATATAATGGATTGATATTTATGATTTTCTCCATCATCGTCCCTA
TTTACAATGTGGAAAATACCTCCGCTGCTGCGTGGATTCCGTGCTTGCCGAAAATTTTGC
CGATTATGAAATGATTTTGGTCGATGACGGTTCGCCGGACGGCTGCGGGAAGATTTGCGA
CGAATATGCAGGCAAATATCCGCATATAAAAGTGATTCATCAAGAAAACGGCGGGCTGTC
GGATGCCCGCAACGCCGGTATCCGGGCGGCAAAAGGCGATTACCTAATCTTTTTGGACAG
CGACGATTATTGGGCCGATACCAACCGTTCAAAAAACGCGGGGGGGATTCTCTTTGATT
TACAACAACTTGCAGACAAAAAGGTTGATTTGATCCTGCATCCCTCGTCCTTCAATTACC
GCGACATCCCCAAAGGGGCGGACTTTTCGGATAATGATTTTGTCCGCCATTTTGAAACGC
TGGTGGAGGGGCGGTACTATATCGCCAACGCGTGGACAAAGATTGTCAGGCGGGAAATCA
TCATTAAAAACAATCTGTTTTTCCCAAAAGGATACATTCACGAGGATTTCCCGTACAGTT
TGCAATTGGCGCGTTTTATCAAGACTTTTGCCTTTTACGATAACCCTTTTTACCAGTACC
GCGTTCTCGGCGGCTCTATCAGCCACAACATCAAATACAAAAATTTCAGCGATGTGCTGA
CGCATCTCGACTGGGGTGTGGATTTTTTAGTCGAAAACAAAAATTCCCCCATCTACGGCG
GTTTGCAAAAATTTGTCTTCGACAATATCGGCTATCTGAGGTCTATATTGGTAAGGCTTT
ATTTTTCCAAAAACATTATCCTCATCTACCGGAAATATTTTTCATTTAAAGAAAAATGCA
GAAAGATATTCGGCGCGAAGGCAATCCGTCCGGTTTTTATCGGGAAGACCGCATTCATCA
TAGGATTGCCGATATTGCGCCTGCTCGTACCGCCTATGCTGTACCCGGCAATCAAGGCCG
TTTATCAGAAATTTTTTTCGGAATAAGCTTCCGGCAAACCCCGAATCGGAAGCGGGCGGG
AAGAAACAGCCGCCCCGCCGGCGGGGATTGCGGCAATGCCGTCTGAAGCCACGAATCCGG
CTTCAGACGGCATCTGTTTACCAAAAAGCAAATAATTCGGTTTGGCGAAAAAAACAGATT
TGCTTTTTGGTAAATACGCGATTACAATCCGCTACATCCGATTTCTACAAAGGATGAAAC
GATGACCGACACAGCCGGTCTGCGCCGCCACAACCTGCGGCAGTGGATAGAAAAATACTA
CGGCGGTTTGCAAACTCGTTTTGCTGAAGCCGTTGCCCTCAACACAGGCGAACTCTCCGC
CCTTTTGAAAAACAAATCCTTCGGCGAGAAAAAAGCCCGTAAAATCGAACAGGCGGCAAA
AATGCCCGCCTTTTGGCTCGATACCGAACACACCGCCCGCCCGTCCGAACACACAGGAAA
ACACACCATGTCCCATATCTCCCCCATCCCCGAAATCCTAGCCGACATCAAAGCCGGCAA
AATGGTCATCATCACCGATGCCGAAGACCGAGAAAACGAAGGCGACCTGCTGATGGCGGC
GCAATTCGTCACGCCCGAAGCCATCAACTTCATGATCAAACACGCGCGCGGCTTGGTCTG

## Appendix A

CCTGCCGATGGACGGCGAAATGGTCGAAAAACTCGGGCTGCCGATGATGACCCAAAAAA
CGGCGCGCAATACGGCACCAACTTTACCGTCTCCATCGAAGCCGCACACGGCATTACCAC
CGGCATTTCCGCCGCCGACCGCGCCCTGACTATTCAAACCGCCGTTTCCCCGACCGCTAA
ACCCGAAGACATCGTCCAACCCGGTCATATCTTTCCGCTTCGCGCCCAAAAAGGCGGCGT
ACTCGTCCGCGCCGGACACACCGAAGCCGGCGTCGACCTGGCGCAAATGAACGGGCTGAT
TCCTGCCTCCGTTATTTGCGAAATCATCAACGACGACGGCACGATGGCGCGTATGCCCGA
ACTGATGAAATTCGCCGAAGAACACAAGCTCAAAATCGGCACGATTGCCGACCTCATCGA
ATACCGCAGCCGTACCGAAAGCCTGCTTGAAGACATGGGCAATGCGCCTGTACAAACCCC
GTGGGGCGAGTTCCAACAACACGTTTACGTCGACAAACTCTCCGGCGAAACCCACCTCGC
CCTCGTCAAAGGCACGCCCGCCGCCGACACCGAAACCCTCGTCCGCGTCCACGAACCCTT
CAGTGTGATGGACTTCATTCAAGCCAACCCGCGCCATTCATGGTCGCTGCCCAAAGCCCT
TGAGCACATCCAACAAGCCGAAAGCGGCGTCGTCATCCTCTTACACCGCACCGAAGACGG
CGCATCCCTGCTCGACCGAACCCTACCCAAAGGCGCAAACCAAGCCTACAAATGGGACAG
CAAAAGCTACGGCATCGGCGCACAAATCCTCGCCGGCCTCAACGTCAAAAAACTGCGCGT
CCTCGGGCAGCCCTCATCTTTCACCGGCCTGACCGGCTTCGGTTTGGAAGTCGTCGGCTT
TGAAGAAGCGGAAAAATAATATAGTAAATTCAAATACTTTATATTTGCTTTATTTATTGC
ATTATTTCCGTGCAAACGAAAACCCGGTCTGTTGGGTTGGATTTTGTTTTTTCAAATTTC
GGGTAACTTCTAATTCGTCATTCCCGCGCTGGCGGGAATCCGGTTCGTCGGGTTTTTGTC
ATTTCCGATAAATTCCTGTGGCTTTGGTTTTTTGGATTCTCTCTTTCAGGGAAAGAACGG
CATAAGTATTTTCCAAACCAAACAAAATGCCGTCTGAAAGGCTTTCAGACGGCATTTTAA
GTTTGACCGGTTTCATTCATCGGTATTTATGAATTGAATTTCAACATCGCCAATCTATCC
TTAATCTCTTTTTCCAATTCGGCAGATTCGGCGAAAAGTTTATCCAAATCCGCTGAAAAT
CCCGCCATTTTTTGCGCAAATTCGTCGGCGGAAATATCCACATAATCAATCTTTACCTCG
AAATACTGCCCCGCCGACAAGCTGTGATTCTTCGCTTTGATTTCATCGTAGCCGATTACC
ACACTGAAATCTTCCACTGCCTGTTTGTGCGTGAAGGTATTGCAGATTTTTTTGTTCTTC
TTCGCGGGAAAGTACGGTTTTTTTGCCGTCTTTAATTTTTTCGCCCAAGCCCGATGCGTC
GATTAATATAGTGGATTAACAAAAATCAGGACAAGGCGACGAAGCCGCAGACAGTACAAA
TAGTACGGAACCGATTCACTTGGTGCTTCAGCACCTTAGAGAATCGTTCTCTTTGAGCTA
AGGCGAGGCAACGCCGTACTGGTTTAAACTTAATCCACTATACCACGTTGTCTTTATTGG
CTTTATCAATAAACAGGATAAGACCTGAAAAAAAGCCGATACGCCTTTTTGGTGTACCGG
CTTTGCCATACTGTTCTGCTTCAGACAGCATTGCTTCATTTTGCCTTTAATACTTCTTCG
TCCAGCGATTTCAACCATTCCAGCTTTTCGCCGATTTTGATTTCCAACCCGCGCGGGACG
GGTTGGTAGAAGTCCGGTTCGTCCAAGCCGTCGGGCATATAGCTTTCGCCGGCGGAGTAG
GCGTTCGGTTCGTCGTGGGCGTAGCGGTATTCGCGTCCGTAGCCCAATTCCTTCATCAGC
TTGGTCGGGGCGTTGCGCAGGTGGACGGGCACTTCGTCGCTAGCGTTTTCTTTGACGAAG
TGGCGCATTTGGTTGTATGCCTTGTAGCCCGCGTTGGATTTCGCGGCGGCGGCAAGATAC
AATACCGCTTGCGCCAAAGCCAGTTCGCCTTCGGGCGAGCCTAAGCGTTCGAAGGTGGCG
GCGGCATCGTTGGCGATTTGGAAGGCGCGCGGGTCGGCAAGCCCGATGTCTTCCCAAGCG
ATACGCACGATGCGGCGGGCGAGGTAGCGCGGGTCGGTGCCGCCGTCGAGCATACGGCAG
AACCAATACAGCGCGGCGTTCGGATGCGAACCGCGCACGGATTTGTGCAGGGCGGAGATT
TGGTTGTAGAAACTCTCGCCGCCTTTGTCGAAACGGCGGATTTGCGCCCCGAGACTGTCG
GCGAGAAATTCGGCGGTTAAGTTTTTCAGACGACGTGTATCGGCGGCGCGTAAAAGTTGT
TCCAACAAATTCAACAATCTGCGCGCATCACCGTCGGCGGTATTCACGAGTAATTTTTGC
GCATCCGTTTCAATCGTAAACTCTTGGTATTCAGGCAAAGCCAATACCTTGGCAATCAGC
TTTTTCAGGTCGTCTGAAGACAAGGGTTGCAAAACATACACCTGAGCGCGGCTCAACAGC
GCGGGATTGACTTCAAACGACGGATTTTCCGTCGTCGCACCAATAAAGGTTAGCAAACCG
CTTTCGACATGCGGCAAAAACGCGTCCTGCTGCGCCTTGTTGAAGCGGTGGACTTCATCG
ACAAACAAAATCGTCGCGCGTCCCTGCTGCAAAGCGATTTCGGCTTTATCGATTGCCTCG
CGGATGTCCTTCACGCCGGAAAATACGGCGGAAACAGGCAAAAACTGGGCGTTGAAACTC
TGCGCCAAAATCCGCGCCAACGTCGTCTTGCCCACGCCCGGCGGCCCCCACAGCAACATA
GAATGCGGCTTGCCGCCTTCTACCGCCACGCGCAAAGGTTTACCTTCGCCGATGAGGTGT
TCCTGCCCCACCACGTCGTCAAGCGTATGCGGACGCAATCGTTCGGCAAGCGGCGCGTCG
GGTTCTCGGGCAAACAAATCGGTCATAACGGCTCCGTCAACAGGTTTTCAAACAATATGA
TTATACGGCAGGGAACGGCGGCGTGCCGCATACGGATTCCGCCCCTCCGTTTGCCTTAAG
CCGATATTAGGCGCATACTGGAAAAGACGAGAGACTTCACACAATATATCCGGCACGGAG
ACCGATTCCGCATCGGCATGACAATACCCAAATCAGCGTTTCAATTAAACATTAAGGAGA
CTAAAATAGAAAATTTGCTTTATCTACCATTGCTTTGTTGATTTAATCGGCATTATGTTT
TGAGGCGGAAGCCCATGAATATACTAATATTCAAGAGATGGAATGGGTGTCTTTATTTTC
TGATCCGCAAAGAGACGATGATAGTCTTATAACCCTTAAAGATGAAAAAATCACTGTAAA
AAACTATATTGTGCCTTGGTGGAAAAAAGGTGAAAACTTTAGAAAATTAGAACTTGGCGG
ATTCGCATTTGAAGTGCAACTTTCCCTAACAGAAAAAGGCCAGTATGCGGTAGCATACGG
CCTTTCCTGCAAGAAAGATTGCCATGAGCTACACGCAACTGACCCAGGGCGAACGATACC
ACATCCAATACCTGTCCCGCCACTGCACCGTCACCGAAATCGCCAAACAGCTGAACCGCC
ACAAAAGCACCATCAGCCGCGAAATCAGACGGCACCGCACCCAAGGGCAGCAATACAGCG
CCGAAAAAGCCCAGCGGCAAAGCCGGACTATCAAACAGCGTAAGCGACAACCCTATAAGC
TCGATTCGCAGCTGATTCAGCACATCGACCCCTTTATCCGCCGCAAACTCAGTCCCGAAC
AAGTATGCGCCTACCTGTGCAAACACCACCAGATCACGCTCCACCACAGCACCATTTACC
GCTACCTTCGCCAAGACAAAAGCAACGGCAGCACGTTGTGGCAACATCTCAGAATATGCA
GCAAACCCTACCGCAAACGCTACGGCAGCACATGGACCAGAGGCAAAGTACCCAACCGTG
TCGGCATAGAAAACCGACCCGCTATCGTCGACCAGAAATCCCGTATCGGCGATTGGGAAG
CCGACACCATTGTCGGCAAAGGACAGAAAAGCGCATTATTGACCTTGGTCGAACGCGTTA
CCCGCTACACCATCATCTGCAAATTGGATAGCCTCAAAGCCGAAGACACTGCCCGGGCAG
CTGTTAGGGCATTAAAGGCACATAAAGACAGGGTGCACACCATTACCATGGATAACGGCA
AAGAGTTCTACCAACACACCAAAATAACGAAAGCATTGAAAGCGGAGACTTATTTTTGTC
GCCCTTACCATTCTTGGGAGAAAGGGCTGAATGAGAACACCAACGGACTCATCCGGCAAT

# Appendix A

ACTTCCCCAAACAAACCGATTTCCGTAACATCAGTGATCGGGAGATACGCAGGGTTCAAG
ATGAGTTGAACCACCGACCAAGAAAAACACTTGGCTACGAAACGCCAAGTGTTTTATTCT
TGAATCTGTTCCAACCACTAATACACTAGTGTTGCACTTGAAATCCGAATCCAAGGGCAT
TTTAAAATCCCGAAGCAGACGGCACGCGCCCCGAACATTCGTTCTTTAACGCCCGTTTTC
AGAATGCCCGCCTGCGGGCATATTTTGCCCGATCAGTTCGGCTATCCTCTCGCCGTCAAA
CTGCGTTTTGAACACCACCTTGATTTCTTTGGAAATCTCGGCAAACAGCTTTTCGGCATG
TTTGATTTTCCGCTCTTCGCTTTTTCGCAAATCTTCCGCCCCGTCAGTCCCTTTGGCTTC
AATCACAAAGTTCAGGATCTCGCCGCTTTTGGTTTTCACGATATAGGCAAAATCGGGCGA
ATACGTGCCGCCGCCGGCAACAGGGATTTTGATGGAGTTTCTCGGTATTTTGGTAAATAC
GATTACGCCTTCAATTTGGTTGTTGGCGACATTTTCATGTTCTATATCCGAATCGTAGAA
AATCTCGCCGAAGAGATAGCCGGCGGCAGGCCGGTGCTCCGTATCTTCAAATCTGCCCAA
ATCTGCTTTTTTCACCGCGCGCGGTTTGCCGTCTTTATTGGTAAATTTGGTCGGATGGAT
TTTGCTGCCGACAAGCCGGTAATCCAGTTCGAATTTATGGAAGGAATGATGAAGCAAAAA
CCGGTTGAAGCCGTTTTTGATTTGGGCGATGGTTTGCATATTCAAAAAATCGCCAATGTT
CAGTTCGTTGCGGATGCAGTAAAACGCCTGATGCAAAGTCTGCATACGGATTTTTGCCGT
TTGTGCCAGTTTTTCCAGAAACTCTCGGTAAGTCATTGTGTTGAAACGGATAAAATCTTC
ATCTTCAAAACTGTCTATGCGGCGGGAAAGCATAAGCCCGTTGTTGATATAAGCTTCGTT
TACCGCCGTGCGTATGCCTGCCTGTGGGAATTTGGCAGCGTTTTCATGCAAATAGGCGGT
AAATAAATCGGCAAATTCGGCTTCATCCTTGATTTTATACTGCAAAACGGCTTTATGGTG
AATCAGCTCCCACAAGGCTTTGAGTTCTTCATATTTGCCTTCGCGCATGATGATGGTGTC
TTTGCCTTCGTCTTTGGCGTTGCTGACTTTGCCTTTGTCCAAACCTTTGGGGAAGGCTTC
GGGATAGGCGGCTTTTAATTTGTCATAGCCGTCTTCGGCAAAGTTTTCATTGTCGTCAAT
GATGCCATCTAAAAACAGTTGGTTTACCAATACCAGCGGTTTGATATCGGGGTATTTTTG
CAATATTTTTTGTTTCAGCTCTTCGGTAAACTTTTTGGAGATTTCTTCCTGAAAAGAATT
GTCGTTGATTTCGCCGACAAGCTGCTTCACAAAGTCTTTTTCGCTGCTATCGACAAAATA
ATTCAGTTTGTACGGTACATCGCGCACCCGCGCCATCAGCTCGTTTACCGGCAGGCGCAG
GCCGCGTCCGACTTCTTGCAGCTTGGAAGTCGTGCTGCCGCTGGAACGCAGTTTGCAAAT
CTGGAAAACGTTGGGATTGTCCCAGCCTTCGCGCAGCGTCCATTTGGAAAAAATAAAGCG
GCGCGGGTTGTCCAAAGACAGCAGTTTTTCCTTATCGTGCAGGATTTCATTGATTTCCTG
CTCGATTCTATCGTCGCTGTCTGTATTGTCTTTGGAAAAATAGCCGCCGTGGCAGGCGGA
TACATCGTCCAACGTCTTTTGCAGGTAATCGCGGTAAAACGGGTCGCTTTCCGTTTTCAG
ACGGCGTGCCGCTTCCGCGCGAATCCAGCTTTCAAATTTATCTTTCAGGCTGCCTGAAAG
CTCGTTGCCGCTGCGGTAGCCCGCGATATCGTCAATAAAAAACAGCGTCAGCGGCTTGAT
TTTGGGCTGTGGCGCGCGTTCTGCCAAAAGCGCGCGTTCCAGCTTGAAATGTTCGGCAAC
CGCCCGCTGCATCATCGCATCCTGCACCGTTTGCGAATAGGAATAAGGGTTGATGACGGC
ACCCGTTTTCAACTCCAAGCCGTTGCTTAACACCACCACGGTTTTATTCATTTTGTCGAT
TTTCAAATCCGAAATAGCCGGATGGATTTGCGCCAAATCTTCGCCTTTTGCCAGTTTGAA
CGTCTGCTTTTTGTCCTTTTCGTTTAATTCAAATTTCGCTTCTTTGCCGTCCGACGACAC
CAGTTTTACCGCCGCATCCATGCCGCCCTGCATTTCTTCCTGAAACACGCGCACGCCTTT
GACCAGCCCGTCGTTAAACGCGTCTACTGCCGTCAAACGGTAAAGCAAGTTGTAATATTC
ATCGTTAAATGTTGCACCGTAGCGCAAAATATATTGCGGTTTTAAGCGTTTGATATTGCC
CCACGTTTTCGCGCTATCTCGGGTCGGGAATTTATGCGGTTCGTCCACAATCATAAACGG
GCGCACGGCAGCCAATGCATCAACGGGATTGTCAAACAAATCCTTCAATGCCTTGTCGCC
CGTATCGTTCATGGACGACGAATTAACCATGCCCGCGTTAATCAGCAGCACATGAATTTC
CTTTTTGTTTTCCGCTTTGACAAATTGCTCAATCGTTATGGGCGCATTGGACTTTTTGCC
CTTATTCTTTTTCGCGCTTTCCACCACATAGGTTTTCAGGCGTACGCCTTCATAATCGCC
GCCGAAATCCTGTTCAAAATGCTCTGCCAAAGCCTTGCTTTGCAAAAACTGCTGTGTTCC
CGCCTTAATGGACAAAGTCGGCACGACCACGATAAATTTGAACACGCCCAGCCAACGGTG
CAGCTCGAACATGGTTTGTGTCTAGGTATAGGTTTTGCCCGTGCCCGTTTCCATGGAAAT
ATCAAGGATATTTTGGTCGTCCGAACGGTCGGGGAATCGGCCGTCTATACCGTTTTGGCT
TTGCACTTTCAGGATATTGTTTGCGTATTGTTTTGCAGCAAACAAAAGTTCGGGATTTTC
GTCTGCCGTCCGATATTTGGGCGTTGCCCCGTCAAACACGCCCAAAACCGCCGAAACCGC
CCGCATTTGGTGCGGCTGGTTTTTCTCGTAATTAAAACCGCTCATGAATTGCCTCCGTCA
AACCCTGATAACCACATTCAACTCAATCTCTTTTTTATTGGCATAACCGCGAACCGCCTG
GTCAAGTTCGTGCTGCATGGCGCTTGCCATATTGCTGCCGAATACAATCACGCGGTTGGG
ATTGAAATCCGCATCGTCGTCCAGCTTGCGGATAAACGCCAACAAATCGGCGGAAGTAAA
ACCGGCATTCATCAGATACAGCCGTTTTTCGCACAGATACGCCGTGTAAGCCCCTAACCG
CACAGGCTCAACCGGCGTGGTCAGTGCCGCCCCGTCATACAGCGTCCAGGTGGTCAGAAG
CGTTTGCAGCTGTTCTTCGCTTAATTCATCGTTAAGCGGCAAATCCGGTTGTTCGGGCGA
AAAATCCTTGTCCGGATGCTGCCTGAAATTGTCTGCCGTTTGAAAGATTTTGAAGCCCGA
ATCGCCCGTGTAATCGGGATGTTCGACGCGGATTTTGGCGGCGGCTTTTTCTATGCGGGC
TTTGGTGATGTCGAAGATGGTCGGGTAGCCTGCTTTACGGGCTTCGGATTTTTCAGCGGT
TTTTTCGGGAAGCTGTACACAGATATAGCGGCGGTTACCGTTTTGTCCTTCGGCGTTAAG
CTGCATCACGGCGTGGGCGGTTGTGCCGCTGCCTGCGAAGAAGTCTAGGATTAGGTCATT
ACTCTTTGAACTTATTGAAACTAAAAATTTAATCAATTGACTAGGCTTGGGGAAGGTAAA
TATTTTGCTACCAAATAAATCTGTGATTTCTTTTGTGCCTTCTTTAGTCATTCCGATATT
TTCAGGTAGCGTCCTACTAAAAATAGCCAAATATTCGGCTACTGCATCGGCTAATGTACC
AACATTTTCAGGTAATCTACTGCTTACAGCTACTTTGCCAAAATCCTCGCCAGCTTTTTT
CATGTCGTCATCTTTAAAGTAACGCATAACTGGATTGCTGATATTTAAGAAATCATAATC
ATCAGGGAAAACGATTTTTCCTTTATTATAATAATCTTGAAATGTATCTTTGGTTACACG
CCAAGTTGCATTTGGATTTGCTGGATATTTTTTTCCTGTCTTGGGATCAACCATTGTGAA
AAAACTATTTGGCCTTTCCGCCGCAGTTGTTTGTTTCGTTAAGTCGTGGGTACGCCAAGG
ACGATCGGGGAAATCATCAGTCTCATAATAGCGTCGTTCCTTGCCTTTAGTTGCTGCAAT
AAATTGGCAAGATTTTGCGAATACAAATATCCATTCATAATCCTGCGAAATACCAAAAGG
CACATCTGATTTAGCTGTTCTTTTTCGCCAAGGCAATTGTGCAACAAAATTCCCTTCCCC

## Appendix A

AAACACTTCATCACACAACAATTTCAACTGCGCCGCTTCGTTATCGTCAATCGAGATAAA
AATCACACCGTCGTCCTTTAACAGTTCGCGGGCGACATACAGGCGCGGATACATAAAGGT
GAGCCATGCGCTGTGCGAGTTTGAGCCTTTGTCGGTGAAATCTAAAATCCGCGCGGCTTC
GTCTTCATCAATATTGGCTAGGCGGGCAAGTTCAGCGGGTGTGAATTTGCGGTCGTCCTG
ATAGACAAAGCCGTCTGATCCGGTGTTTTAGGGCGGGTCGATGTAAATCATCTTCACGCT
GTTTGTGTAGGCGTTTTTTAAGTGTTTCAACACTTCTAGATTGTCGCCACGAATCAGCAG
GTTTTGGCTGCCTGCATTTTCGGGCTTGGCGTTGTGCGTCTTGTCTTCACTTATCAGGGT
TTCGGGCGGCAGGTTGCGAAGCAGGCGAGCATATGATTTGCCCAGCCAGTTCATTTCGTA
AAATTCGCGTCCGATGTCGGTCTGCGGCGCGATTTCGGCTTGTAATCTGTCGATAAGGAA
ATTTCCGTCTGCGTCAAAACAGGCGGGAAACAGTTTTTTGAGCTGTTCGAGTTGGGTAGA
GTTGGCGGTAATGCCGTCTGAAGTGTAGATTGCCTCAGTGTTCGCCCCGGCTGTGTCGCT
TAGATCAGGGCTTGGGTTGGGTTGGGTTGGGTTGGGTTGGGTTGGGTTGGGTTGGGTTGG
GTTGGGTTGGGTTGGGTTGGGTTGGGTTGGGTTGGCAGCATTTTAAAATCCTCGGTTTGA
ATTTGTCAATATCAACTGTCTGTTTTAAAATATTTTTTTACTTTAAACGGCGTTTTTTGG
GAAACGGGCGACGCCGTCTGAACGTCTGTCTGCGTGTTACTGCCCGACAACAACGCGACG
GATTTTGACGGGCTGTACGGGTACGTTTTGATAAAAGCCGCGCGTGGCGGTTTTGACGCG
GGCGATTTTGGAAACGGTGTTCATGCCGCTTTCGACCCTGCCGAAAACGGTATAGCCGTA
TTGTCCGTTTTTGTAGTCGAGCGAAGCGTTGTCCGCCAGATTGATAAAGAATTGGCTGGT
GGCGGAATCGGGGGCTGTCGTCCGCGCCATGGCGATGGTGCCGGCGGTGTTTTTCAAGCC
GTTGCCGGATTCGTTGGCAACGGCCTTATCGCTTGCCTTTTGTGCCAAGTCCTCGGTCAA
TCCACCGCCCTGGATAACAAAACCGTCGATAACGCGGTGAAAAACGGTGTCGTCGTAAAA
GCCTTTTCGGGCATAGCGCACGAAATTAGCAACGGTTTTGGGGGCTTTGGATTCGTCCAA
AACCAAACGGATATTGCCCATATCGGTTTCCATCAAAACATGGGTTGCCGCCATAGACGG
CAGGGAAACCGCCAAAAGCAGCGCGGTTAAAACGGTTTTGAATTTGGGTTTCATCCCGTC
CTCCTCAGACCTTCAGACAGCATTTTCATTTCCTATGCCGTCTGAAGGCTCGTTAACGCT
ATTCCAATGCGTCTTTGAGTTTTTGTTCGATTAAATCCGCATCAAACGATTTGGCAATCA
ATTCAAAACGCGAGTCGCGCCGCCAAGACACTTGGTTCGCACCCCATTGCCCGTCCACCC
AGTTGAGCCACACCCACGTTCCCAATACTTGGAACACGCCTTTGGCACGGACGAGTCCTT
CGGTCATATTGGGCAAATCATTGAAGAAGTTGGTCAATTTTTCACCGTCGAAATCGCGTC
CGGCGGGGAATGTGAAACCTTGCGACTGGAAGCCCATCGTGTTGTCCGGCAGGGCTTTGA
GGCGGTAGCGTGATTTTTCGATGACGGGGATGTCAAGCCATTGGATATCGAGTTGTGCGT
TTTGAACTTCGACCACTTTAGCCTTGGGCGGGAACAGTTTTGCGGCTTTGTCGTGAAATT
CGGCAAGCTGTTCGGGGGTGCATAAATCGGTTTTGCTGGCAACCAATACGTCGCAGATGC
CGATTTGGTCTTTATACAATGCCTGCTGCGCGTAATCGGGGTTGATGAACTGGCGCGGAT
CGACGACGGTAAAGACTGCGCCGATTTCCAAAAGGCTGTCCAGCGGTTTGGTTTTCAGTT
CATCAATGACACTGGCGGCGTGCGCCAGTCCGCTTGCTTCAATCATCAGGCGGTCGGGCT
TGGCGTCGCGCAGCATTTTCTGCACGGTTACGCCCATTTGCGGGCCGGCGGTGCAACACA
AACAGCCGCCGGCGATTTCTGCCACAGGGATGCCGTTGTCGCTCAATACCGCGCCGTCAA
TGCCGATTTCGCCGAACTCGTTGACGATGATGACCCATTTTTCGTTCGGGTCTTTTTGCG
CCATCAGGCTTTTGAGCGCGGTGGTTTTGCCTGTTCCCAGAAAACCTGAAATCAGGTGGA
CTTTGGTTTTTTTCATTTCTATGTGATGTCCCACTTTAAAATTTGAAGATAGGGTGTTTT
AAATGATTAAATAATGTTAAAAGTGATGGCAGCTGTTATCATGTTCTTCATCAATTGACA
ATTGTTCCAGCAAATTCGATATATCGGCTGACATCGCCGGTATGACGTTCAAAACCGTCT
CCCAAACGACAGACAGGTTCATTTCAAAGTAGCCATGGGCAATCCTATTGCGCAATACCC
CCTCATCCCTATCCAATTCAAATATTTGGTTTCCTCGGCAAATTCCGGATACGATTTAAG
GATATGGGTTGCGTTTTCACCGATAAGGAACAAATTAAAAACGACTGCCTGCACAGTCTT
TGTGTCGGCAGAAAATTGCCCATAATCCATTTTGTCGGTATATAGCCGGATATATTGCGC
TGCCTGCAATATCTGTTTCAAATAGACCGACAATTCTTTCTGCATTTTCATAATGCGACA
GCCTCAGTCAACACCTTATCTCTAAAGTGGGCCGAGATGTCATGGGTGTGAGCAAATGG
ACCTTTATGCCCAAGAGTTTCTGCAATTCCTCTTGCAGCCCGCCCAAATCCAATAATGTT
GTCCCTGTTTTTGCATCCACCAACAAATCAATGTCGCTGTTTTCCGTATCATCTCCGCGA
GAAACCGAACCGAATACCCTTGGATTGCAAATCAATGGATATTTCCCGAAAACTGCCAAT
ATTTCTTTCTTTCTGCTTTGCAACAAAAGAGACGGTTTCATTATCTGCTCCTTTCGAAAG
GCTTATTATCAATGCAAACCACCGATTACTGCTGACATTTTTTACAAATCCCGGTTAAAA
CAACGTGTTCTTCTTTCAGCGCAAAGCCGCTTTCGGCAACGCCTGCGCGCAGTGCCGCCC
ACTCGTGGCTCAGGGTTTGCTCGTCCGCCGTGCCACATTCGGTGCAGACCAAAATAAACG
CGCTGTGGTGCGCTTCGGCTTCTTCGTGGTCGTGGCAATGGTCGTCGCACTCGTGCTGCG
CGTGGCTGCACAAAATATAGCCGTTGACCGCCGCCACTTTGTGCAAAACGCCCTGCTCCG
CCCAAAAATCAAGGGCGCGGTAGGCGGTAGGCGGTGCAAGCACGCCCTCGCTTTGCTGCT
GCATCTGCGACAAGACGTTGTAGGCTTTAATCACGCCGCTTTGCTGCAAGACAATATCTA
ATACCTGCTCGCGCAAAGCGGTTACCTGCAAGCCTTCGCTGCGTGCCTGTTCGATAATTT
TCTGTTTGAAATTTGTTTTCATAAATTCTCTGTTTATGCCGTCTGAACAACCGATACGGC
AGGAGGCGGTTTTATATTTGTATTCAATTGCTTTATTTGGAAATCTTTTCCAACAATGCC
CGACAGCCCGCATCCGCAAGCCTGAAGGTTTCTTCAAAATCGCCCGTATACCACGGATCG
GGGACATGGTCGTAACCGCTTTCGGGTATCAGGTCGGTCAGCTTGAATATTTTTTCCGGC
CGCCTGCCGAAGGTTCTTTCCAATTCGGACAAATTCTTGCCGTCCATCGCGATGATGCAG
TCAAACGCCGCCGCATCGCTTTGGCGGATTTTGCGGCTGGTAAAGCCTGAAGCATCGATA
CCGTGTTTTTTCAATATCTTTGCCGTCTCGCGGTGCATATCTTCGCCGTCGTGCCAGCCC
GATGTCCCCGCGCTGTCCGCTTCAAGGGGAATGCCCGCTTCGGCGGCGCGGCGGCGCAAA
ATGTATTCCGCCATCGGCGAACGGCAGATGTTGCCGAGGCAGACAAAAAGGATTTTCGGT
TTTTTCATATCCCCTCCCTGTTCCGGCGCGATGCCGTCTGAAGCGGAAACCCTTTCAGAC
GGCATCGGTCGGTTAATCGTAACCGCATAAGGCAGAACCAGCCAAAACATCCATCTGGAA
GAAATGGTTTGGCTAAATCTTAGGCATATTTAATAAGTGTCCAATATTAGAAGCCGTATG
CTCCAAATAGAGGCTGGCATTTTTCAAACTATCTTCTAAAGGTTCACTTTTCTCCAAAAT
AGAAAAGGCAGCTTGGATATTTTCAAATGGCAGGGAAGGCAAATCTTCAACGAGACTGCC

## Appendix A

ACAAATAGCGACAACAGGAACTCCGACAGGGGTTCTTTTTGCTACACCAATAGGCGCTTT
CCCTGCTAAACTTTGACGATCTAGTCTTCCTTCACCAACGATAACCAAGTCAACATCTGA
CACTTTCTTATCAAAGTCAATCAAGTCCAGGCAGGTGTCAATTCCAGATACGATACTTGC
CTGAGCAAAGGCGCACAAACCACCAGCGATGCCTCCACCAGCTCCTGCTCCTTTAAGTTT
TAATGTTGCAGGGGAGACTTTTTCATAAAAATCTTGTATTGCCTGATCTACGGCCTCAAA
CATAGTAGAATCCAACCCTTTTTGCTTGCCAAACGTATAGGTCGCACCTTGGTGTCCACA
TAAGGGACTCACAACATCTGCTAAAATACGAATGTGAACATCTTCAGGAATTTCATAGCG
ATTTTCTGTTGAAACAGAAGCTAGGTTTAGTAAGGATTGACCGCAAACGGGTAAGGCATT
TCCATCCTCATCATAAAATTGATAACCTAAACCAGCAGCAATCCCAATACCTCCATCATT
ACTGGCCGTACCGCCAACGCCAATATAGATTTCTTTAATTTCTTGACTAATGAGGTGGCG
AATCAATTCTCCAATACCACGAGTTTGGATTTGTAATGGATTTCGTTTCTCTAGCGGGAT
TTTTCCAAGACCAACCAAATCAGCAACTTCGAATAGGGCTAGTTGTTCTTTTTGAAAATA
GCGCATGACTTCTTTTTGTCCAAAAGGTCCTGTCACTTGGAGACATTTTTCTTCTAGGTC
AAGAGAATGTCGGATAGCATCTACAGTGCCTTCTCCCCCATCACCGACAGGACAGAGGAG
ACATTCCACATCTGCTATCGATTGTTGGAAGCCTCTTTTTATTGCTTCAGCTACCTGTTG
AGCTGTCAAGCTTTCCTTAAACGAATCCGGTGCAATTACAATCTTCATATTTATAATTCA
TCCTTTCGTTTCACTCAAGGCACAACACAGAATGAAAAAGTGTTGTGCTCTTTATTTTGA
TTTATTATATAAATGAGAAAGCCTATCACTACTACAAATCACTATGCGCTGAAAAACGGA
TTGTGCCCTTCCCGTTTCAATGCTTCCGCATAGCTCGGGATGCTTTCCTGTTCGCCCAAG
GGATTGTGCAACAGGTAAACGTGTTCCACGCGGCACTCCGCCATCAGGTCGAGGAAATTT
TGCTGGATGACGGCGATGTTGTCGGTAAAGGAAAGCTGCCACGAAAACACCTGCGGCACG
ACTTCAAACGCGCCGTCGGTCGCATTAAACCCGAATAAAATCTGTCCGCCCGCCTTTGCC
GCAGCGACCACGCCGACGCGCGGACTCTGCATACGGACGGCGCGTATCGCGTTTGCCGCA
AACACATCCATCGCCATACGTTGGCGCGTGTGGCTGCCGTCTGAAAGCGCGCGTACCGGA
GAATCCGGGGAAAGCGGATTGGCAAACAGCGTAACGCCTTCCGATGCAAGATTTTCCTGC
CATACCTGCATCAGGGGCAGACCTGCCTGAAGAAGGTTCTGACCCAATGCCGTCTGAACT
TTGCCGCTGCCGAAACCCAGCGCGTAAACGACGTGGACGGACTGCCCTTCGGGCAACAGC
AAAGGGCGCGGGGCGAAACGGCGCAAGTGTTGCGCCGCCGCTTCGGTATTTTGTTTGGCA
CGCCACCACTCATCCGGCGCGACCGCGCTCAAATCGGAAGAATGCACAAGATAAGGCAGC
CATTGCGTCTCTTGCGTCAACGCGCGCAGGTGCGGATAGTTTTGCAGGCAGGCAAACAAT
GCCTCCGTCGGCAGCTTCATCTCGATTGCACGTTCTTTTTTGCAGCCGGACACCAGCACG
ACCGGCAGGGCGAACCACTGCACTTCGCCTTCTTTCTCGCAATCGAGTACCGCGTTCACA
CTGGAAAGCAGCGCGGCATAAGTTCCGGCATCGGGCGACATCGTCAGCGCGAGGGAAAGG
TTGATATAGTGGTTTTGCTCAAGCATTCCCCTGATTTCGGTTTGAAGTTGGCCGGACGAG
AGTTTGCGCGAAGCCTGGGAAGAATTATGCGCCAACTGGTAGGCATTGAGCAGCAGGTGG
TTTTTAATCGGATTTTGGGGATACGGGCGCGTATCGGGCAAGGTAAATGTCTGGTTCATA
TGTGTCGTGCACCGCTTTTCGCAGTGTGTGTCTTTCCTGTCTGAAAATATATCGGACGGA
TTGCCGCCGGGACTTGCCCGTCAATCCGCCGAAACGAGAAAATGCCTGTCTGCCAAGTCT
GCCAATATTTCTTCCACATACACTTCGGCAGGCGGATGGAATGTCAAACCGTCGGGCGTG
GTGCTGACGATGTTCAAGTTTTCGGCAACCGTTTCAATCAATGCCTGTTTGCTCGTCGGC
CGCGACAATTGGCGCATAATGTACAAATGCCCGTATCCGAATGAGGGGGTGCTTTCGTTG
TAACGGTTGGCAGGCCGGACAAACGCTTCCGCGCCGTGTTCCACAAAGGCGGCGGCATAG
TTTGTAAAGCGCGCCGGCACATAGGTTTTATGGTCTTCAAAAAATGGTTTGCCCGCATTG
TCTGATGAAACAGAAAAACGCCCGAGAATGGTTTGCAATAAAAGCTGGGAATTGATTTTC
ATGCGGTCAAATTCCAAACCGGGAAATCGGCGGGCGAGATTTTCCGCAACATCTTCCGTT
TTAAACGCCTCTCCGGTTTTCATCACATCGCGTATGCCCGAAAGCAGGATATCGTTTTCA
TCAAAGTTGATTGTTTCCCCTCTTGCCGGGCGGAAATTCAAACTTTCTATCACTTCGACG
GCAACCGACTCATCACGCCTGACAGTATCCCCGACTTCCTCACGGCATAAAAGCGAACGG
CGGAATTGGCGGTCGGATAAAATATCACTGTAAAATTCTTTGGCAATATAATCGTCCCCT
GCCAATGCCAGAATCCGCTCCCGCGTATGCTCCGCCATCCAAGAAACAAAAGACACGTGC
AAATTGGTATCCCCGATATATGCGAGCCTGTGGCGGTTAGCCCATTCGATGAAGCCGTTG
ACGTAAATCGGGTCGTTAAACGCCTCCATATATTCGTGTGCGATGTAATAAAAATTATGA
TTCAATATTTTTTGAATCGCCGGAAGTTTGCCGCCGCCGTCCAAGCCCTTGTCGTTTTCC
AAAATTTCCGCCAGCGCCTTGAGCGCGTCCAAGCCTTTCCGCGTCCGCGCTTCCAAGGGT
TCTTCAAGCACATCCCTGCCGGCAAAGTACATAATTTCGCGCAACTGCTCCTGCCGTTTC
CAGCCGGGGTAAACATTGTATGAAATATAGGCAATGCCGTGTTTGGTCAGGTTGTTCCAG
CAAATCGAAAAAATTTTGTCTTTAACTGCGTCAGGCACCCACGACCAAATGCCGTGGACG
ATGATATAGTCAAACTTCCCGAATGACTCATCGATGGTCAAAATATCTTTTTCTTCCAGA
CGCACATTTTTCAAGCCCATTTTTTCAATGATGGCGTTGCCCTGTGCAACCTGCCTGCCG
GACAGGTCGATACCGACAAATTCCGCATCCGGGTAATAAAGTGCCTGCGTGATGATGTTT
CCGCCCATCGAACAGCCCAGCTCCAAGACCTTGGCATTGGCGGCGGGCGCGGGCTGCAAA
CCCATCAGGCGGGCGCGCGCCTCCAAATTATTGATGGCGGTTTGAGAGAATGCGCCGGAT
TCGTACATCAAATCATCATATGAATTTTTGATGTTGGACACGTCCGGCACACCGTTCTCC
GTTGCAGCACGCGCAAAGGCGGCTTTTTTCTTCTTGTTCGGCATATTTTGTTTGTCTGAA
GGCACTATTGCCCGCAAGTTTAACCAATTCATCCTACCCGTTCAACTAAATCAAATGCCA
TCTGAAGGCGCGGAGCGTACTTCAGACGGCATCTGGGAGGCGCGAAGGCTTCAGACGGCA
TTTTTGCCGCTTTATTTCAACGCGGCATCATAGCCGCCGTCTTCAACGGCTTCAATCAAC
GCCCCTGCATCGGTTTGCGCGGGGTCGTATCCGACGGTCGCACTTTTGTTTTCAAGGCTG
ACTTCGACGCTTGCCACGCCTTTTACGCCTTCCAATATCCGGGTAACGCTTTTGACGCAG
CCGCCGCAGCTCATGCCGCCGATGTCGAGGATAAGGGTTTCCATGATTTTTCCTTTCGTT
GGTACTGCATTCTGACGGGCGTTATTGTAAGTCGGGGCGTGAACTTGGGCAAACGCGGAA
ACGGTGCGGCGGTTTGAAAAAATACGGACGCTTGCGCATAATGGCGGCAATTCCCATCAG
GACAACAACAATGAACGCTTCGCAAAAACCCTGGTTGAGCATCATTGCCTTGGCAATCGG
CGCATTTATAGTGGATTAACAAAAACCAGTACGTCGTTGCCTCGCCTTAGCTCAAAGAGA
ACGATTCTCTAAGGTGCTGAAGCACCAAGTGAATCGGTTCCGTACTATTTGTACTGTCTG

## Appendix A

CGGCTTCGTTGCCTTGTCCTGATTTTTGTTAATCCACTATACAACGTCGGTATCGGCGGC
GGCGCGCTGCTGGGGCATTGGGTTACGCAATACTCGGGCATTTCCTGCATCGGCGTTGCG
GGTATGCTGACGGCGGCGGCAGGTTTGTGGGTCTGCCTGAACCTGAACCGCCATATCCGA
ACCTGACCGCACGATGCCGTCTGAAGCCCCTCCCGCCCTTCAGACGGCATTTTGATTGAA
GAAATATCCACCCCTGCTTAAAATAACGGGCTTTGCCGTGTTTTAACGCCTATTTTTTGT
TCCAAGGATGGTTTATGCCGCTGCTGTCTGTCGAGTTCGCACTGTTCTTTCTCGCCTTCC
TGCCGATTTACTGGGGCTTGGCGAAATACCCGTCCGTCCAAAACCTGCTGCTTTTGGCTG
CCGGTATGGGCTGGCTCTACCATATCGGCCCTGTATTTGCGGCAATCATCGTCCTTTATT
CCTCCTGCGTGTACCTTTTGGGCGAACTGCTCCGTTCCGATCGCGAAAATACGCGCCGTT
TCTGGCTGGGGTGCGGCATTGCCGCCTCGCTGACCGTCTTGGGCTTTTTCAAATATTTCG
ACTTTTTCCGCCCGATGATTGCCCAATATGCCGGAAAAGGCGGCGCAATCGACATCCTGA
TGCCGCTGGGGCTTTCGTATTACACCTTCCAGTCGCTCGCCTATCTGGTTTACTGCTTCC
GCGCCCCGCACGCCGCGCGTTTCAGCTGGCACGAGCTGCTGCTGCACCTGAGTTTTTTCC
CCACCGTTACCTCCGGCCCGATTATCCGCGCCGCCGCATTCAAAAGCGCAGACGGCGAGC
AGGCAGGCGCATTGGCGCAAATCCGTACCCGCCGAGCGCGTTCGCCCGTCCGCCCCGCAC
TCGCCGTTTCCCTGATTCTGCTGGGTATTGCCAAAAAATGGTGGCTGGCGGGGATGCTGG
CGGAAAACTGGGTGTCGCCCGTATTTGAAAATCCCGCCCAATTCGACGGCTGGGGCGTAT
TGGGCGGCGTGTACGGCTATACCTTCCAACTCTTTTTAGACTTTTCCGGATATTCCGATT
TGGTTATCGGCATGGCGATGCTGCTGGGCTTTAGGCTGCCCAAAAATTTCTCCGCACCGC
TTCGTGCTTTAAACATCCGCGCATTTTGGGACAAATGGCACATCAGCCTTTCCACCTGGA
TACGCGACTACATCTACATCCCCTTGGGCGGCAGCAAAAAAGGCTTTTTACGGACACAGC
TCAACCTGATGGCGGCAATGGTGCTCTCAGGCATCTGGCACGGCTACGGCTGGAACTTCC
TCATTTGGGGCGCGCTGCACGGCACGGCACTGGTGCTGCTCAACACGGGCGACCGCTATT
TCGGACGCGACGCGCTATGCCGTCTGAAATACTTCGCGCCGCTCTCATGGCTCATTACCT
TCCATTTCGTCTGCCTTAGCTTTGTCGTCTTCAATACCGCAAATCCCGACGATGCAGGCG
CAGTTTTCAGTGCCCTCTTTGCCAATGCCAACGGCTGGAATGCGCCGCAACAGGCAAACA
TGCTGTTGCTTGCCTCGTTTGCATCCGTGATGCTGCTCTACCCTTACCTGCAACGCGCTT
TCGACGGCGCGGTCAAAGGTTTGGAAAAAATCCCGATGTGGCTGTGGTTTATCCCCGTTT
CCGCCGTCCTGCTGCTGATTATCGTCCTCGCCCCCTCGGGGATACCCGGCTTTATTTATG
CCAATTTTTAAGGGTTTGGACATGAAAAACTTTCTTTCCCTTTTCTCCTCCATACTGATG
TCTGCCCTGATTGCCGTGTGGTTCAGCCAAAACCCCATCAACGCCTACTGGCAGCAGACC
TACCACCGCAACAGCCCGCTCGAACCGCTTGCCGCCTACGGATGGTGGCGGAGCGGTGCG
GCGTTGCAAGAAAACGCCTACGCCCTTTCAGACGGCATCAAAGCCTTCCTGTCCGGCGAA
ACGCCGCCGACGGCTCAAGACGGCGGTTCGGCAGATATGCCGTCTGAAGCCGCCGCATCC
GAAGCCGTCCCTCAAACCGGTGAAACAGAATGGAAACAAGACACCGAAGCCGCCGCCGTC
CGCAGCGGCGACAAAGTCTTTTTTGTCGGCGACTCGCTGATGCAGGGCGTTGCCCCCTTC
GTGCAAAAAAGCCTGAAACAGCAATACGGCATCGAATCCGTCAACCTCAGCAAACAAAGC
ACGGGGCTGTCCTACCCCTCATTCTTCGACTGGCCGAAAACGATTGAAGAAACCCTGCAA
AAACATCCCGAAATCAGCGTACTCGCCGTCTTCCTCGGACCGAACGACCCGTGGGATTTC
CCCGTCGGCAAACTCTATCTCAAATTCGCTTCCGACGAATGGGCGCAAGAATACCTGAAA
CGTGTCGACCGCATCCTTGAAGCCGCACACACGCACCGCGTCCAAGTCGTCTGGCTCGGC
ATCCCCTACATGAAAAAAGCCAAGCTCGACGGACAGATGCGCTACCTAGACAAACTGCTT
TCGGAACATTTGAAAGGCAAAATCATCCTGATTCCCACCACGCACACCCTGAGCGGCGGG
AAAGACCGCTACACCGACTCCGTCAACGTCAACGGCAAACCCGTCCGCTACCGCAGCAAG
GACGGCATACACTTTACCGCCGAAGGACAAAAACTGCTGGCGGCAAAAATAATGGAAAAA
ATCGTTTTTGAACCAAGTACGCAACCATCAAGTACACAGCCATGAACCCCAAACACCTCA
TCGCATTTTCCGCCCTATTCGCCGCCACGCAGGCAGAAGCCCTACCTGTCGCCTCCGTCA
GCCTCGACACCGTTACCGTTTCCCCGTCCGCCCCCTACACCGATACAAACGGGCTGCTGA
CCGACTACGGCAACGCCTCCGCCTCGCCTTGGATGAAAAAACTCCAATCCGTCGCACAAG
GCAGCGGCGAGACCTTCCGTATCCTGCAAATCGGCGACTCGCATACCGCCGGCGACTTCT
TTACCGACAGCCTGCGCAAACGCCTGCAAAAAACTTGGGGCGACGGCGGCATAGGCTGGG
TTTACCCCGCCAACGTCAAAGGGCAGCGCATGGCGGCCGTCCGGCACAACGGTAACTGGC
AAAGCCTCACCAGCAGGAACAACACCGGAGACTTCCCGCTCGGCGGCATCCTCGCCCACA
CCGGCAGCGGCGGCAGCATGACCCTGACCGCATCGGACGGCATAGCAAGCAAGCAGCGCG
TTTCCCTGTTTGCCAAACCCCTGCTTGCCGAACAAACCCTGACCGTCAACGGCAACACCG
TCTCCGCCAACGGCGGCGGCTGGCAGGTACTGGATACGGGCGCGGCACTGCCCCTGACCA
TACACACCGAAATGCCGTGGGACATCGGCTTCATCAACATCGAAAATCCCGCCGGCGGCA
TTACCGTTTCCGCGATGGGCATCAACGGCGCACAATTAACCCAGTGGTCGAAATGGCGTG
CCGACCGTATGAACGACCTCGCCCAAACCGGCGCCGATTTGGTTATCCTTTCCTACGGCA
CCAACGAAGCTTTCAACAACAACATCGACATTGCCGACACCGAACAAAAATGGCTGGATA
CCGTCCGCCAAATCCGCGACAGCCTGCCTGCCGCCGGCATCCTCATCATCGGCGCACCCG
AATCCCTGAAAAACACGCTCGGCGTATGCGGCACACGCCCCGTCCGCCTGACCGAAGTCC
AACAGATGCAGCGGCGCGTCGCCCGTCAGGGGCAGACGATGTTCTGGTCTTGGCAAAACG
CCATGGGCGGCATATGCAGCATGAAAAACTGGCTCAACCAAGGATGGGCCGCCAAAGACG
GCGTACACTTCTCCGCCAAAGGCTACCGGCGCGCGGCGGAAATGCTCGCCGACAGCCTCG
AAGAACTCGTCCGCTCCGCTGCAATCAGGCAATAATCGGACAGGAGGCGGACGGTATTTC
CGCAACAGGGGGATGCCGTCTGAAACGCATACCTTCATATTGCTTCAGACGGCATAGCCA
CCCCGCGCACGGTTTGCCGGACGCAACCGGCATTCGCCTCAGGCATCGGAAGGACGCAGG
CGAACCTCCGGCATACGGCGCAAAGGCGGCGTTTGATATGCCGTCTGAAGGCAAAGATGA
TAAACTGCCGCCTTCCGTTTTCAGACGGCATATTGTTTTCAAATGAGGGCGTTCTCCGTC
CGCAACCATAAAGGAAGTTTCATGAACCGGACTTATGCCAATTTCTACGAAATGCTCGCC
GCCGCCTGCCGCAAAAACGGAAACGGCACGGCAGTGTTCGACGGCAAGGAAAAAACCGCC
TACCGCGCGCTCAAGCAGGAGGCCGAAGCCGTCGCGGCGTATCTGCAAAATATCGGCGTG
AAGTTCGGCGACACGGTCGCGCTGGCGGTTTCCAATTCCACAGAATTTATTACCGCCTAT
TTCGCCATCTCCGCCATCGGCGCGGTCGCCGTACCGATGAACACATTTTTGAAAAACAGC

## Appendix A

GAATACGCGTATATCCTGAACGACTGCAAGGCGCGCTTCCTGTTCGCCTCGGCCGGCCTG
TCAAAAGAATTGGCGGGCTTGAAGGCGCAAACGCCCGTCGAAAAAATCATTTGGACGGAC
AAAAGCCGTCCGACCGGCGAAACGGCGGAAGGCGATGCCTTTTTTGAAGACGTGCGCCGC
TTCCCCGAAAAACCCGACTTGGGCCGCCAACCCCGGATAAATGATTTGGCACACATCATC
TACACCTCCGGCACGACGGGGCATCCCAAAGGCGCGCTAATCAGTTACGCCAACCTGTTC
GCCAACCTGAACGGCATCGAACGCATCTTTAAAATTTCCAAGCGCGACCGCTTTATCGTT
TTCCTGCCGATGTTCCACAGCTTCACGCTGACGGCTATGGTGCTGCTGCCGATTTATATG
GCGTGTTCGATTATTTTGGTCAAATCCGTTTTTCCGTTTTCCAACGTTTTGAAACAGACA
CTGCTCAAACGCGCGACCGTGTTTTTGGGCGTACCCGCGATTTACACCGCGATGAGCAAG
GCGAAAATCCCTTGGTATTTCAGATGGTTCAACCGCATTCGCCTGTTTATCAGCGGCGGC
GCGCCTTTGGCGGAACAAACCATCCTCGATTTCAAAGCCAAGTTCCCCCGCGCCAAATTG
CTGGAAGGCTACGGACTGAGCGAAGCCTCTCCCGTCGTCGCCGTCAATACGCCCGAGAGG
CAAAAAGCCCGCAGCGTCGGCATCCCCCTGCCCGGTTTGGAAGCCAAAGCCGTCGATGAA
GAATTGGTCGAAGTGCCGCGCGGCGAAGTGGGCGAACTGATCGTCAGGGGCGGTTCGGTG
ATGCGGGGCTACCTCAATATGCCTGCCGCCACCGATGAAACCATCGTCAACGGCTGGTTG
AAAACGGGCGATTTCGTTACCATAGACGAAGACGGCTTTATCTTTATCGTCGACCGCAAA
AAAGATTTGATTATTTCCAAAGGTCAAAATGTCTATCCGCGCGAGATTGAAGAAGAAATC
TACAAACTCGATGCCGTCGAAGCCGCCGCCGTCATCGGCGTGAAAGACCGTTATGCCGAC
GAGGAAATCGTCGCCTTCGTCCAATTGAAGGAAGGTATGGATTTGGGCGAGAACGAAATC
CGCCGCCACCTGCGTACCGTGCTGGCAAATTTCAAAATCCCCAAACAAATCCACTTTAAA
GACGGGCTGCCGCGCAACGCTACGGGCAAGGTATTGAAACGGGTGTTGAAGGAGCAGTTT
GACGGAAACAAATGAACGCCGTGCCGTCCGAAGCCCCGTCCGGCAAAAAAATGCGGTGAA
TCTGATTCACCGCATTTTTTTGACAAACACCGGAGGGAGGGCATTTACTCTGCCAATTCC
ACCTGCTCGTGCGCCATCAGTTCCTGACCGACATCATCCAACAACATCAAATAACGTTCG
TAGTTTTTCAAAATGTCGGCAATCAGCTCGTCCTTGTTCATATACTGCACATCGTACCCG
ACGCGCCCGTCGAAAAAATAAGCGTAGGGTTTGTAAGTTGTCTGATGCCGGATATGCGGC
AGCTTGCCGTCGTTAATCAACTGGTCGGATACATCCTGCCCGACAGACTTAATCCCGTAC
ATAAAATCGCGCATCGTCTCTTTCCGAATGACGAACTCGATTGCGGGCTCGTCCCGATGA
AACATTTTATCGACCCGGACGCTCAAGCCGTATTCTTCCGAAAGCTCCCGTTGCAACTCG
TGCATAGCGGGCGATGCAGTCTGTTTGAGGAATTTTAAAATATCCTGCTCCTGCGTCTGG
CTCATTATCTGCACCAGCCGTTCTTTCCACTTGCCGCCCGTCCAAAATACACTGGTAGGG
TTAACCCGGGTCTCAAAATATTTCTTATCCGCACTCAAGCCTTTCCACAGGCTGAAACAC
ATTATCAGCATCAGCAGGGCAAACGGCAGGGAAACAATCAGGGTCATAGACTGCAGGTTG
CCGAGTCCGCCCGAGCGCATCAGCAAAACGGCAACGGCAGACATCAGCACGCCCCACATA
ACCGCCTGCCACCGTGGCGCGCTCAAGCCTTTGTCCCGAGAGGTAATATTGTTCAGGACA
TAAATCCCGGAATCGGCAGAAGTTACAAAAAACAGAGAAATGACCAGCAGGCTGACGATG
CTCGTCAATTCGGGCAGGGGGAGGTAATTAAAGAATTTAAAAAGCAGCGTTTCCGGAGAG
GAGGTCATCTTTTCGAGCATTCCCCCCGCAACCCCGTCATTCAGCCAAATCGCCGTATTG
CCGAAGACGGTAAACCACAAAACGCCGAACAGGCCGGGGATGAGCAAAACCCCGAAGACA
AACTCGCGGATGGTGCGCCCCTTTGAAATGCGCGCGATAAACAAACCCACAAACGGCGCC
CAAGAACACCACCACGCCCAATAAAGCACCGTCCAAGATTCAAACCACGGCTTGTGTTCC
CGTTCGTACGCATAAGTTTTAAAACTGAGGCGCACCAGATTTCCGAGGTAGTTCCCTATG
TTGTCGCCGAATGCCGACAACAGGTAAACAGTGGGTCCCGCCGCCAAAACAAAAAACAGC
AGCAAAAACGCAAGGCCCAGGTTCAACTCGCTCAACACCTTCACGCCCTTCCCCACGCCG
GATATTGCCGAAACGACGGCGAGGGACATGACGGCGGCGATAATCAAAACCTGCACGCTG
AAGCTGTTTTCGGCAATCCAGCCCATTTCCTGCAATCCGGCGCCCAGTTGCGAAGCCCCG
AACCCCAATGTGGTGATGATGCCGAAAAAAGTAGCAAGCAACGCCATAATATCAATGGCA
TCGCCGAACCTTCCGGAAATTTTTTCTTTCAACAGGGGGTAAAAACAAGAACGCAGGGCA
~~AGCGGCAGCTTGTAGCGGAAACCGAAATAAGCCAAAGCCAATGCAATGGTACGGTAGAGC~~
GACCAAGCGTGAACGCCCCAATGGAACACCGTGTGCAGCAATGCCTGCTGCTGCCTGTGT
TCCGGCGTGCCGGCCGTAATGTCCGAAAAATAATGCATCAACGGCTCTGCCACGCCGAAA
AACATCAGACCCACGCCCATCCCGGCCGCAAACAGCATCGCCAGCCACGACAGGAAGCCG
AATTCCGGCACATCTTCATCCCGTCCGAGCCTGATGTTTCCCAAACTGCTGACCGAGAGT
ATCAGCAGGAAACCCAGAAAAATGGAAAACGTTAAAACATAAAACCAGCTGAACTCGGTA
AAAATGACTTCTTTTGCCCGATCGAGCCACATCTGCACCTGATCCGGCACGGTTAAAACC
AATACCACCAAAACACACACAAAAAACAAAGTCGTCAAAATAACCATCGGATTAAATGAC
GTTCGGCGTTCTATAAATTCAGACAGGGACAAACCTTCTCACTCCTTTGTTAAAAACAGA
CAAACCCGGTCATCGGGCAAAGCGGTCAAAACCTGCCGGTAAAATACCGGCTTCCGGATG
ACGAAATGCACAAACCGGCCGAAATTGTTATAATCGAAAAAATTAAAAATCAATACGGAT
TATTCTATCAGATTGATTTATCGATATTTATTATATCATTAATATAGTTTGATTTCAAAC
CGGCGGAAAATGCGCCGCCCACCGCAGGCGCGCCCTCCCCGCTATCGGGGCGTTTAACGC
CGGATTGCCGATGCGGTACAATGGCTGATATGAAGAAAATTACCCCTCAAAACCTGCGCC
CCCTGCTTTCGGAAAGCTTGGGACATACCGATTTTGTCAACGTCCTCAACGCACTGATTA
AATTTTTGCGCCGTGGCGGCAAAAAATGTGCGGGGGAACGTTTCGACCTGATTATCGACA
CATTCAAACAAGACAGGGAATTACTGTCCCGCTTCAGCCGGTGTTTTTACATTTGGCTCG
CGCAAATACACATTTATCCGGCACTCATCAAACTCGGCATCTTCTCGCGCCACAGCTTTG
CCCGGGAAATGGGCATACGCATCTACGAACGCTTCAGCCCGTCATATAAAGATTTTGCCA
ACTTGGGCGAAGTCTTCCTTTATCTTTTCCATTCCGAAAACGACGACAAATGGCTGCAAA
CGCTCAATATCCGCCAATGGCTGGTTTTATACGAACTCATCCGGAGCCACGCCGAGCCGT
CCAAATTGCAGACGGCGGGCATCCGCCTTGCCGATGCGCGTTTGCGCGCCATCGAAATGC
TGTCTGTCTGGACGGCATCCGAAGCCATCGAACCCGACCTCATCCGCATCGCCCCGCGCC
TGCTGGAAGCCGATTCTTCCTTCGTCGCCCTCCAACGCGAAACCGCCAAACTGGTCGAAC
ACTACCGCAACGGCACCACGCCTTACGACACCGCCCACCTCGAAGTGATGTTCGACCAAT
GTTTCAGCCAGATTGACTATTTGCGCCGCAAAGGGACGGGCGCCGGCTCCGGTTCGTCGG
TCAAAGTCGCCCACCTGCTCGAACGGCTCCGGCAGACCGTAGACCGTCTGAAGCTGCTCA

## Appendix A

CCGACATCCAAACCGGCGCCGGCAACAGCAACCGCCTGACCATCGCGCTGATGAACTCCC
TCATCTACGCGGCGGTCGAACAATACAGCACCCGCCACCTGCGCCGCAGCAGCATCCGTA
TGCTCGCCCGCAGCATTACCGAAAACAAAAGCCACCACGGCGAACACTACATCACCCGCA
ACCGCAAAGAATATTTCAAAATGTTCTACTCGGCGGCAGGCGGCGGCATCATCATCGCCC
TAATGGCGCTGCTCAAAATCCGCATCGGCTCACTCGGCCTCAGCCCCTTCCTCACTTCCT
TGTCGGCTGGGTTCAACTACGGCATCGGCTTTATGATCATCCATATGCTGCACTGCACCG
TCGCCACCAAGCAGCCCGCGATGACTGCCGCCAGCTTTGCCGAACAGGTCGATCTCAACG
AAGGCGGCAAAGCGGTGGACAACAAACTCGCCAAGCTCCTCATCGACGTATGCCGCTCCC
AAAGTGTCGCCGTCTTCGGCAACGTTTCCATCGCCATCCTTTTGGCGTGCGCCATATCGT
TCGGCTATGCCCATCTGTACCGGCTGCCCATACTCGATGCCCACACCGCCGCCTACCAGT
TCAAATCCATAGACATCATCGCTTACCCGACGCTGTGGTATGCCGCCATTGCAGGTCTGT
GGCTGTTCTGCTCCGGCATCATCGCAGGTTTTTCGACAACCGCGCCGACTACCTCAACC
TGCGCCAACGCCTGCCCTTCAACCCCTTGCTGCGTAAAATCATGCGCCCCGGCCCCGCC
GCGTCCTCGCCGCCTACATCCACAAACACTACGGCTCGCTGGTCGGCAACTTCATCTTCG
GGATGCTCTTGGGTATGACCGGCTATTTCGGACACCTCCTCGGGCTGCCGCTGGACATCC
GCCACGTCGCCTTTTCCTCCGCCAACCTCGGCTATGCCGCCGTCAGCGGCAACGTCGGTT
TGGGCACGTTCGTACTCGGCATTTTCAGCGTCCTCGCCATCGGCCTGGTCAACCTCTGCG
TCAGCTTCAGCCTCGCCCTCTTCGTCGCCCTGCGCTCGCGCGGCACGAAAATCGGCAGCA
TCCGCAATCTGATTAAAAGTTTTTGGAATCAGATTAAAAGCAATCCCTGCATACTTTTCC
TCCCGCCCGCCAAAGAACAGGGACATCCTCCTTCGGACAAGCCTTGACCGGCAATGCCGT
CTGAAGCGGGATTCGCCCCGAATACCGCCCTGATGCGGGAAATCCCCATAAAAGGATGCA
AAAATGCCGTCCGAACCGAAACGTGGTTCAGACGGCATTTTAAAAAACATTACAATCCCG
GGCCGCCAAGCGAATTGGCAATGTCCCTGACCGCCGTTACATACATCCGGCTGTGGTTGT
ACTGCCATACCGTATAAAAATTGTTCAAGCCCAAATAATATTCAAACACGCCCGGTGCGG
TTTCCAGTTTGAACAAAACCGCCTTTTCATCATCTGCAAGCTCTTCGCCGGGGATGATGC
CGTACGCCTTCAAATCCGCCACCGTCCGCGTCAGGGCGGTTTTTCGCCAATGATTGCCT
GAACATCCGCACCCGGCGCCAATGTTGCAGACACCAGCATTTTCCCGCCCGTGCGCCAAC
CGTGCTGCTTCATATAATTGGCAACCGATGCCGCGACATCGCCGACGTTGCCCCATATGT
CCCGATGTCCGTCCCCGTCATAATCCACCGCCCATTTCCGGTAGCTCGAAGGCATAAATT
GCGGCATCCCCATTGCGCCCGCATAGCTGCCTTTAAAGGCGAAAACATCGCCGCCTTCTT
CTTTTGCCAGCTTTAAAAGCTCGACCAATTCTTTTTGGAAAAACCCGGCGCGGCGGGGGT
AATCAAAGCCTAAGGTCGCCAATGCGTCCGCCACACGGAAACTGCCCGTATTTTTGCCGT
AATTCGTTTCAATCCCGATAACCGCCACGATAAGTTCGGCAGGCACGCCGTATTTTTGCG
CCACATCATCGATAAGCGCGCGGTTTTCCGCATAAAACCGGCGCGCGCCGCGAAATTTCG
CCTTGCCCGAATTTCCCGTGCGGAACACATACCACGGACGCGATGTGGAGGGGCGGTGCA
TAATCTTGACGATGTCCGCCTTGTAAGCCGCTTTGTCAAAAAAATCCTGCCATTCCGCCC
GGGAAAAATCCCCTTTCCCGACTTCATCGTCCACAAAACGGCGGACATTTGCATTGGCGG
CAAACCCGCTGTCGGATACCGGTACGGCTGCCGCGTCAAATACGGCTGCCGCGTCAAACG
CGGGGCGGCTTTCTTTTTTCATTTCAACCGCGCGGGGGGCTTGGGCTTCATTTGCCCGGG
GTGGGCGTGCCTCCATCGCCGTACAGGCAGACAAAGCCGCCAAACAAATTGCCAGCGGCA
GTATTTTTCTCTTTTTCATAAATATGTTCCGAACAAATAGGGTAAGTGGGAAAGCGGCAC
AAGGGGGCGGCGCGAAATGCGGCATCCGCCGCAATCGGCGGCTTTGCGGAATGCCGCACG
TTGCCTCTTGCACCGCCCGAAATCCGTATGTCGTCGCCGAAAATGCCGTCTGAAGGCACT
TCCCCTTTCAGACGGCATTGCCTGCCGCCGTATTTGCCCGCTACCCGCAATATCGGCAGT
CCAATATATCTTTGCGGATGTCGTTCAGCAGGAAGGCTGCGGTGTCTTCGTGTTTCGCCT
GTACGAAGACAAAGAGTCGGGCGATGATTTTGCCGATAAAGACTTTTTGCAGGGCGCAGG
CATCGGTCAGGGTGTGTTCCTGCAGGAAGCTGCGGATGTCTTCAGGCAAAGTGTAGTCGC
GGGCGACGGCGGCAAAGCGGGCATCGGTTTGCAGGCGGTCGAGCAGGGTTTGGTTTTTGT
CCAACTTCATGCCTGCTTCTTTTTCTTCGGCGGTGGCGCGGACAAACTGGTCGTAAATTT
CGGCATAAAGCATATCGTTCGGGCCTTCAAAAATCGTGAAGGGGCGGATGTCGATAGCGA
TATTGCCGGCGGTGTGTCCGCGTTCAAAACCCTTCGCACCCAAGAGTTTTTGCAACATTT
GCGCGGCGGCGTAAGTGTATTCCGTGGCGAGGGTTTTGACGATGTTCGCCTCCATCAGCT
GATGGGCGACGGGGGCAACAGGCGAAACGGAATGGCAGACGTAGCGGTAAAGAATCTCGG
AAACCTGATGGCGGCGCCGGATTTCGCGGCGTTCGTAATCGACGAATTTGATGTCGTTGC
GGACGTATCGTTCCAGATTTTCAAGGATGTATTCCATAATGCCGTGCGTCATGCCGATCA
GTTGCAGGCGGCTGCGGATAAAGATGTTTTGGAACGCGCGCAAACCGGCAGCGTCGCTCT
GGGAGAGTTTCATCACGGCGGTTGCAGGCATTTCGGCATCGATGCGGTTGACGGCGTAAC
GGACGGCGCGCAAGCCTTCGGATGCGAGGGTTTCGCAGCGGATGTATGTTTTGGGGACGA
GCAGCAGGTCGATGACTTTGGCGAGTTTGCCGTTTTTGCGCTCTTTGGCGGCAACGAGGA
GGAAGTCGCTTTGCGAGTTGCCCTGCCAGTATTTCGCGGCGTTGACGTAAATGGTTTGTC
CGTCGATATATTCGTAGTAGGACTGCATTTCGCGTGCAATCGCCGCGCCGGAGGTTTCGG
GTTCGGTAACACCCAAACCGCCGCCCTCGCCTTTGAAAATCATCTCCAAACCTTGCGCGA
CTTGCGCTTCATCGCCGAACTCTTGCAGTGGCTGCAACACCAGCGCGCCTTCGATGCCGG
TACGCAGCGTAACGGGCACGCCGTAATGCCCCGCAATCCGCAGGACTTCTTGGATTTCAA
ACTGGCTGCCCTTGCGCCCGCCGTATTTTTTGTCGAGGAAGGGCAACAGCAAACCCGCCT
GCTTCAAGGCAAGCCATTTGTCTTCGGGCAGGTATCGCATCAGGTCGATACCGTCTGAAA
AAATGCGGCGGAATGCGGATTCGATGTGCTTTAAAAAAGCAGCCGTGTCCATAGTTGACG
GCTGCGCGCTCGGTTCGGTGTGTATCATCGGCTTCCTCTGTCGGTTCCCATTAATCGGCG
GCCGGTCAAACCGCCTGCCACAGTTTAGAGTTGATTTTCTAAACTTTACCACAAAGTGCG
CCGGGCAACAATCCGCCGACCTTTCAGACGGCATCGCGTCCCCTCCCGTGCTAAAATGAC
CGTTTGCATCACTGTCCGCCGATTGCCGCACTATGACCTACCCCATCCCCAAACCCCGTG
AAAAATCCCGTTGGCCCAATCTTTCGCAAGGCTCGCTGCCCTTGGCTTTGGCGCGTTATC
TGCCGCACAAGCGGCTCAAGGTCGTGCTGACCCAAGATGCGGAACAGGCGTTGCGCCTTC
AGACGGCATGGCGGTTTTTCCGTCCGCACGACACGGCGGTGTTCCTGCCGGACTGGGAAA
CGCTGCCTTACGAGCGTTTTTCGCCGCATCAGGATTTGGTGTCGGAGCGGCTGTCGGCGT

## Appendix A

TGTGGCAGATTAAAAGCGGCGCGGCGGATGTGTTGTTCGTGCCGGTTGCCACGGCGATGC
AGAAGCTGCCGCCCGTGCCGTTTCTGGCAGGGCGCACGTTTTGGCTGAAAACGGGGCAGA
CTTTGGATATAGGCCGTCTGAAAAGTGATTTGGTGGATGCGGGCTACAACCATGTTTCCC
ACGTTGTCGCGGCGGGCGAATTTGCCGTGCGCGGCGGTATAGTCGATTTGTTCCCGATGG
GCAGCGAAATGCCGTACCGCATCGATTTGTTTGACGATGAAATCGACAGCATCAAAACCT
TCGATACCGAAACGCAACGCACCATTTCCCCCGTTTCCGAAATCCGCCTGCTGCCGGCGC
ACGAGTTCCCCACCGACAGCGAGGCGCAAAAAATCTTCCGCAGCCGCTTCCGCGAGGAAG
TCGATGGTAATCCGAACGATGCGGCTGTGTACAAAGCCGTCAGCAACGGTCATTTCGGCG
CGGGCGTGGAATACTACCTGCCGCTGTTTTTTGAAAACGAGTTGGAAACGCTGTTTGACT
ATATCGGCGAAGATGCGCTGTTTGTCTCTTTAGACGATGTTCATGCCGAGGCAAACCGTT
TTTGGAGCGATGTCAAATCGCGTTACGCGATGGCGCAGGGCGACGAAACCTATCCGCCTT
TGCTTCCACAGTATTTGTATCTCTCTGCCGATGTGTTCGCAGGCCGTCTGAAAAACTACG
GACAGGTGCTGCCCGATGTTTCCGGCAAGGAATACACCCTGCCCGACCTTGCCGTCAACC
GCCAAGCAGATGAGCCGTTGCAGGCATTGAAGGATTTTCAGACGGCGTTTGACGGACGGA
TTTTGCTGTGCGCCGAAAGTTTGGGACGGCGCGAAACTATGCTCGGTTTCTTGCAGCAAA
ACGGTTTGAAAGCCAAACCCGTGTCCGACTGGCAGGGCTTTTTATCGGCACACGAGCCGC
TGATGATTACAGTGGCGCCGTTGGCATACGGGTTCAAACTGGGCGGACTGCAATCGCCGA
ACCAACAGCAACCTACTCCCTCCCCCGTGGGGGAGGGTTGGGGAGAGGGCAAAGCAGTTG
CCGCTCAAACTGAATTTTCCGCAGCCGCAATAAACCCTCTCCCTAGCCCTCTCCCACAGG
AGAGGGAACAAAGTGCAGCCGCCGTTTCAGACAGTCTGAAAGCAGCCGCCGTTTCAACCG
AAAGCAGCCTGCCCCTCGGTACAAGTAATCTGCACGGGCAAATCCGACAGCAACCTGCCC
CTTCCCCCGTGGGGGAGGGTTGGGGAGAGGGCAAAGCAGTTGCCGCTCAAACCGAATTTC
CCGCATCCGCAACAAACCCTCTCCCTAGCCCTCTCCCACAGGAGAGGGAACAAAGTGCAG
CCGCCGTTTCAGACGACCTGAAAACCAAAAGCAGCCTGCATCCCGTCGCAAATAATCTGC
ACGGGCAAATCCGACAGCAACCTACTCCCTCCCCCGTGGGGGAGGGTTGGGGAGAGGGCA
AAGCAGTTGCCGCTCAAACCGAATTTTCCGCAGCCGCAACAAACCCTCTCCCTAGCCCTC
TCCCACAGGAGAGGGAACAAAGTGCAGCCGTCGTTTCAGACAGTCTGAAAGCAGCCGCCG
TTTCAACCGAAAGCAGCCTGCCCCCCGGTAAAAGTAATCTGCACGGGCAAATCCAACAGC
AACCTGCCCCCTCCCCCGTGGGGGAGGGTTGGGGAGAGGGCAAAGCAGTTGCCGCTCAAA
GTGCCATCGCCGTCATCACCGAATCCGATCTCTACCAATACGTCGCCCGTTCGCGCATCC
ACAACCGCCGCAAGAAACACGCCGCCGTTTCAGACGGGCTGTTGCGCGACCTTGCCGAAA
TCAATATCGGCGACCCCGTCGTGCACGAAGAACACGGCATCGGGCGGTATATGGGCTTGG
TAACGATGGACTTGGGCGGCGAAACCAACGAAATGATGTTGCTCGAATACGCAGGCGAAG
CGCAGCTTTATGTGCCTGTTTCGCAACTGCATTTAATCAGCCGCTACTCCGGTCAGGCGC
ATGAAAACATTGCCCTGCACAAGCTCGGCAGTGGCGCGTGGAACAAGGCGAAGCGCAAAG
CCGCCGAAAAAGCGCGCGACACCGCCGCCGAATTGCTCAACCTCTACGCCCAACGCGCCG
CCCAATCGGGACACAAGTTTGAAATCAACGAGTTGGACTATCAGGCGTTTGCCGACGGCT
TCGGCTACGAGGAAACCGAAGACCAGGCCGCCGCCATCGCCGCCGTGATTAAAGATTTGA
CGCAAGCGAAGCCGATGGATCGCCTTGTGTGCGGCGATGTCGGCTTCGGCAAAACCGAAG
TCGCCCTGCGCGCCGCGTTTGTGGCGGTGATGGGCGGCAAACAGGTCGCCGTACTTGCTC
CGACCACGCTTTTGGTCGAGCAGCACGCGCAAAACTTCGCCGACCGTTTCGCCGATTTCC
CCGTGAAAGTCGCCAGCCTTTCGCGTTTCAACAACAGCAAAGCCACCAAAGCCGCGCTGG
AAGGCATGGCAGACGGCACGGTCGATATTGTTATCGGTACGCACAAACTGGTGCAGGACG
ACATCAAATTCAAAAACTTAGGTTTAGTGATTATCGACGAAGAACACCGCTTCGGCGTGC
GTCAGAAAGAGCAGCTCAAACGCCTGCGCGCCAATGTTGATATCCTTACCATGACCGCCA
CGCCGATTCCGCGTACTTTAAGTATGGCGTTGGAAGGACTGCGCGACTTCTCGCTGATTA
CCACCGCGCCCAGCCGCCGCCTCGCCGTCAAAACCTTTGTCAAACCCTTTAGCGAAGGCA
GCGTGCGCGAAGCCGTGTTGCGCGAACTCAAACGCGGAGGACAGGTATTTTTCCTGCACA
ATGAAGTAGATACGATTGAAAATATGCGCGAGCGGCTGGAAACCCTGCTGCCCGAAGCCC
GCATCGGCGTGGCGCACGGACAACTGCGCGAGCGCGAGCTGGAACAAGTCATGCGCGACT
TTTTGCAGCAACGATTTAACGTGTTGCTCTGTTCCACCATCATCGAAACCGGTATCGATA
TCCCCAACGCCAACACCATCATCATCAACCGCGCCGACAAATTCGGACTGGCGCAACTGC
ACCAGCTTCGCGGGCGCGTCGGCCGCAGCCATCACCAAGCCTACGCCTACCTGCTCACGC
CCGAATACATCACTAAAGACGCAGAAAAACGCCTCGATGCCATTGCGGCGGCAGACGAAC
TCGGCGCAGGTTTTACCCTAGCCATGCAGGATTTGGAAATCCGTGGTGCAGGCGAAATCC
TTGGCGAAGGACAATCCGGCGAAATGATACAGGTCGGCTTCACGCTCTACACCGAAATGC
TCAAACAAGCCGTTCGCGACCTCAAAAAAGGCCGCCAGCCCGACCTCGACGCACCGTTGG
GCATCACCACCGAAATCAAACTGCACAGCCCCGCCCTGCTGCCCGAAGATTACTGCCCCG
ACATCCACGAACGGCTCGTCCTCTACAAACGCCTCGCCGTCTGCGAAACCGTGCAACAAA
TCAACACCATACACGAAGAACTCGTCGACCGCTTCGGCCTGCCCGAACAACCCGTCAAAA
CCCTTATCGAAAGCCACCACTTACGGCTTATGGCAAAAGAATTGGGTATCGATGCCATTG
ATGCGGCCGGCGAAGCGGTAACGGTAACCTTTGGTAAAAACAATAATGTCGATCCAACCG
AAATCATCCTGCTGATTCAGAACGACAAAAAATACCGCCTTGCCGGCGCCGATAAGCTGC
GGTTTACCGCAGAGATGGAAAATATCGAGGTCAGAATCAACACCGTAAAAAACGTTTTAA
AAACCTTGCAAAACAGATGCCTGCCCAAATAAAGCCGACACCGCAATGCCGTCTGAAACA
CCGTTTTCCTTGTCCGAAAGCCGCCATTATGAATTTGAAGGAAACTCCACTATAATACGG
CATTCAGATTTCCAGACGGCATCGCGCCCGTCAAACCGCACACAAACCAAAAGGAAATAC
ATGTTCCGTACCATGCTTGGCGGAAAAATCCACCGCGCCACCGTTACCGAAGCCGATTTG
AACTATGTCGGCAGTATTACCGTCGATCAAGACCTGTTAGACGCGGCAGGCATCTACCCC
AACGAAAAAGTCGCCATTGTCAACAACAACAACGGCGAACGTTTTGAAACCTATACCATT
GCAGGGAAACGCGGCAGCGGCGTGATTTGTCTGAACGGTGCTGCAGCCAGGCTGGTACAG
AAAGGCGATATCGTCATCATCATGTCTTACGTCCAACTCTCCGAACCCGAAATCGCCGCA
CACGAACCCAAAGTCGTCTTGGTAGACGGAAACAACAAAATCCGCGACATCATCTCCTAC
GAGCCGCCGCACACCGTGCTGTAATTCCGCAAACGGACATCGATTATGGATATTAAAATC
AACGACATCACCCTCGGCAACAACTCGCCCTTCGTCCTATTCGGCGGCATCAACGTTTTG

## Appendix A

GAAAGCTTGGATTCCACCCTCCAAACCTGCGCGCATTACGTCGAAGTTACCCGAAAACTC
GGTATTCCCTATATCTTTAAAGCCTCTTTCGACAAGGCAAACCGTTCCTCCATCCATTCT
TATCGCGGCGTAGGCTTGGAAGAAGGCTTAAAGATTTTTGAAAAAGTCAAAGCAGAGTTC
GGCATCCCCGTCATTACCGACGTACACGAACCCCATCAGTGCCAACCCGTCGCCGAAGTG
TGCGATGTCATCCAGCTTCCCGCCTTTCTTGCGCGGCAGACCGATTTAGTGGTTGCCATG
GCAAAAACTGGCAACGTCGTCAACATCAAAAAACCTCAGTTCCTCAGCCCCTCTCAAATG
AAAAACATTGTGGAAAAATTCCACGAAGCCGGCAACGGGAAACTGATTTTATGCGAACGC
GGCAGCAGCTTCGGCTACGACAACCTCGTTGTCGATATGCTCGGTTTCGGCGTGATGAAA
CAGACTTGCGGCAACCTGCCGGTTATTTTCGACGTTACCCATTCCCTGCAAACCCGCGAT
GCCGGTTCTGCCGCATCCGGCGGTCGTCGCGCACAGGCTTTGGATTTGGCACTTGCAGGC
ATGGCAACCCGCCTTGCCGGTCTGTTCCTCGAATCGCACCCCGATCCGAAACTGGCAAAA
TGCGACGGCCCCAGCGCGCTGCCGCTGCACCTTTTAGAAGATTTTTAATCCGCATCAAA
GCATTGGACGATTTAATCAAATCACAACCGATTTTAACAATCGAGTAACACGGTTTCGCC
TTATGATGCAGACTTTCCGAAAAATCAGCCGGTATGTCGCAACCTTGTGGCTCGGTATGC
AGATTATGGCGGGTTATATCGCCGCACCGGTGCTGTTCAAAATGCTGCCCAAAATGCAGG
CGGGCGAAATTGCCGGCGTATTGTTCGACATCCTCTCTTGGAGCGGGCTTGCCGTTTGGG
GCGCGGTACTGGCTGCCGCCTTTGCCGCCCTAACCCGGCGGCAAACCGCCCTGCTGCTTT
TTTTATTGTCCGCCCTTGCCGCCAACCGATTCTTGATTACACCCGTTATCGAGGCACTGA
AATACGGACATGAAAATTGGCTGTTGTCGTTTGTAGGCGGATCCTTCGGAATGTGGCACG
GCATTTCCAGTATTGTTTTTATGGCAACCGCCCTACTTTCAGCAGTTTTAAGTTGGCGGC
TTTCCGGCAAAGATGCCGTCTGAAGCCCTCCCATTTTTTTACCTCCCTTCACTTCACTTG
GAGAACATTCATGAGCGCAATCGTTGATATTTTCGCCCGCGAAATTTTGGACTCACGCGG
CAACCCCACAGTCGAGTGTGATGTATTGCTCGAATCCGGCGTAATGGGACGCGCAGCCGT
ACCGAGCGGCGCGTCCACCGGTCAAAAAGAGGCTTTGGAACTTCGCGACGGCGACAAATC
CCGTTATTCGGGCAAGGGCGTATTGAAGGCGGTCGAACACGTCAACAACCAAATCGCCCA
AGCCCTCATTGGTATCGATGCCAACGAGCAATCTTATATCGACCAAATCATGATCGAATT
GGACGGTACTGAAAACAAAGGCAATTTGGGTGCGAATGCGACTTTGGCGGTTTCTATGGC
GGTTGCACGCGCCGCTGCCGAAGACTCAGGCCTGCCGCTTTACCGCTACTTGGGCGGCGC
AGGCCCGATGTCCCTGCCCGTACCGATGATGAACGTCATCAACGGCGGCGAACACGCCAA
CAACAGCCTGAACATCCAAGAGTTTATGATTATGCCCGTCGGCGCAAAATCTTTCCGCGA
AGCGTTGCGCTGCGGTGCGGAAATTTTCCACGCCTTGAAAAAACTGTGCGACAGCAAAGG
CTTCCCGACCACAGTCGGCGACGAAGGCGGTTTCGCCCCCAACCTGAACAGCCACAAAGA
AGCCCTGCAACTGATGGTCGAGGCGACCGAAGCCGCCGGCTACAAAGCGGGCGAAGACGT
ATTATTCGCATTGGACTGCGCCTCCAGCGAGTTCTACAAAGACGGCAAATACCACTTGGA
AGCCGAAGGCCGCTCCTACACCAACGCGGAATTTGCCGAATATCTGGAAGGCCTGGTCAA
CGAGTTCCCCATCATCTCCATCGAAGACGGCATGGATGAAAACGACTGGGAAGGCTGGAA
ACTGCTGACCGAAAAACTGGGCGGTAGAGTTCAATTGGTTGGCGACGACTTGTTCGTAAC
CAATCCAAAAATCTTGGCCGAAGGCATCGAAAAAGGCGTAGCAAACGCATTGCTGGTCAA
AGTCAATCAAATCGGTACTTTGAGCGAGACCCTGAAAGCCGTCGACTTAGCCAAACGCAA
CCGCTACGCCAGCGTAATGAGCCACCGCTCCGGCGAAACCGAAGACAGCACCATTGCCGA
CTTGGCAGTCGCCACCAACTGTATGCAGATCAAAACCGGTTCTTTGAGCCGTTCCGACCG
CATGGCGAAATACAACCAACTGCTGCGTATCGAGGAAGAATTGGCGGAAGCCGCCGACTA
CCCCAGCAAAGCCGCATTCTACCAACTGGGCAAATAAAAAAGGTTAAGGTATGAAGTGGG
TAACTGTCGTTTTATCCTTCGCACTTGTCTGTTGCCAATACAGCCTCTGGTTCGGCAAAG
GCAGCATCGGACGCAACAGCAGTCTGAGAGAACAGATTGCCGTTCAAGAAGAAAAAAACC
AGACACTCGCCCTACGCAATCATTCCCTTGCCGCCGAAGTCTATGATTTGGAAAACGGTC
AAGAAGCCATTTCGGAAATCGCCCGGGTAGAACTGGGTTATATCCAAGACGGTGAAACCT
TTTACCGACTCATCAGGCATAACCGGTAATACCGTCAAAAAGCCGTCCGAACCAATGTTC
~~GGACGGCTTTTATTTCAACAAACTGTCAGACAGCCCCTCATCCTCCCCCGACAAACCGCA~~
ATCCAGCCTGACATCCCCCTCGACGCAACAGCAGCACGGCAGTATCTCGTCCCGCCCCAA
AAAAGCCAAAGGCGGCTCCCGATAAGTAACGCTTCCCTCCAAAATCTTCACTCGGCACGA
TCCGCAATATCCGCTTCGGCACTGATATTCCACCATATGCCCCGTCCGTTCCAAGCCTTC
CAACAGAGTCTCGCCCTCCAAGAGTTCAAAAAAACCCTTATTCGTACCAATGCGCGCCAT
TTCCGACCAATCAAAAATATAGTGGATTAAATTTAAACCAGTACGGCGTTGCCTCGCCTT
GCCGTACTATTTGTACTGTCTGCGGCTTCGTCGCCTTGTCCGGATTTTTGTTAATCCACT
ATAATCCACTATAATCCACTATAAAAGGAACAATAACCGATCCTACCCGCTGTTTTTCCC
ATCATACAACATACAAATGCCGTCTGAAACATCCGGCTTCAGACGGCATTTTTTCAAAAA
ACATTTACAACTCAAAATCGCCCAAATCATCCGTATTCACTTCAGAATCTATCTGACCGA
TCAAATAAGAGGATATTTCCACTTCCTGCGGCGCGACCTGTACGTTGTCGGACGACAGCC
ACGCATTAATCCACGGAATCGGGTTTTGATTTGCGCCTTCAAATCCGGCCGGCAACCCCA
CCGCCTGCATACGCAGATTGGTAATATATTCGACGTATTGGGATAAGATTTCTTTGTTCA
AACCAATCATCGAACCGTCTTTAAACAAATATGCCGCCCATTCTTTTTCCTGTTCCGCCG
CTTTTTTGAAGAGTTGGAAACATTCGTCCTGCAACTCGGCGGCAATTTCTGCCATTTCAG
AATCATCAACACCAGAACGCATCAGATTAAGCATATGCTGCGTGCCGGTCAGGTGCAGGG
CTTCGTCGCGGGCAATCAGTTTGATGATTTTGGCGTTGCCTTCCATCAACTCGCGCTCGG
CAAAAGCAAACGAGCAGGCGAATGAAACGTAGAAACGGATGGCTTCCAACACGTTGACGC
ACATCAGGCAGAGATAGAGTTTTTTCTTCAACCCGCGCAAAGACACGGTAACGGGTTTGC
CGCCGACATTGTGCACCCCTTCGCCCAACAGGTTGTAATACTGGGTGTATTCGATTAAGT
CATCGTAATAGCAGGCAATGTCTTCGGCGCGGGCGGTAATGTATTCGTTTTCGACAATAT
CATCAAACACGACCGACGGATCATTCACAATATTGCGGATGATGTGGGTATAGCTGCGCG
AGTGTATGGTTTCGCTGAAGCTCCACGTTTCAATCCACGTTTCCAACTCGGGAATCGAAA
CCAAAGGCAGCAAGGCAACATTCGGACTGCGCCCTTGGATGGAATCGAGCAGTGTTTGGT
ATTTCAGATTGCTGATGAAAATATGTTTTTCGTGTTCGGGCAGATTGGCGTAGTCGATAC
GGTCGCGCGAGACATCGATTTCTTCAGGCCGCCAGAAGAAAGACAATTGTTTTTCAATCA
GTTTTTCAAATACTTCGTATTTCTGCTGGTCATAACGGGCAACATTAACCGGCTGACCAA

## Appendix A

AAAACATCGGCTCATTCAGCGCGTCGTTTTTGGTTTTGGGAAAGGTGCTGTATGACATAG
TTAAGTGTTCGCAGGACATAATCTATTCTCTATATTAATTAAAAATGATCTGTAAGTTTT
AACTAACCTGCTGAGACTTTATATATTTAATAAAATTTTCTAAAGTTAAAACATGGGTTT
TCCCAAAAGTTCCAAATGTTTTTACGGATTCTTTAGGGACAACTAAGGTTAAATTCTCAT
CACTCATTTCCTGAATTTGAGTATCAGATATACCTTCTTGTAGAGTAAATAAATGCTTAT
GAGGAATTCGATCTGCTTCATTTAAAACTTGGCGCCAGCGATCCTTACAAGTAGTCTTTG
AACCTAGCATTGTCAGCTTTTCAGTTTGAAATATGAAATTCCCTGATTCATCCATCGCAT
GGTACTCTTCACTTCCAGGAAATAAAAAATCTGGTTTCTTTTTCCCTTCTGTCTTCGCTT
GCGTCTCAAATTTTAAGGAAAATTCGTCAAATATTCTTGATAAATGTAACTCTAAAGATT
TTCCTGCTCTTGCTTTTCTGCGATTTGTAAAAGAATGTGCAAAATTAATGAAGCTATCCA
AATCAGTAATTTTTGATTTAATAATTTCAAATTCACGTTTTTCAAAAATGGAAAATAATT
CATATTCCTTTGAAACCCATTGTTTTAACACATTACTAACGTTAGTTTTATTTAATGCAA
TATTTTCCCTTGCCAAAACTGCCATTTCTTCAGTTTTGGGGAAGTTAGGAAATAGTGAAA
ATAATTTTGTTAGGTTATCCTCTACTTCTTGTTTTTTAGGTAAATATAAACTTCCTGGTA
AAATGTTAGTTTCTGCGATAAATATTTCAATATCTTCATCTGAAGATAAAACAAAAGCAT
GGAATAATAAATCTTTATAACAAGCTCTGCACAATACCAATAAGGAACCACTATATTTAT
CACTTAAAAATTCAAAATTCTTGCCAAAACCCGTAATTCTCGCCTCATTTCTCGTGCCTT
GGCCGTAATATTTAAAATTGCTATTAGTGACGCTTCCATCTTGCCAATTAATTTTGATTG
AAATAGTTTTATTTGTTCCCTTTTGGCAAACTACATCAAAGAGGTCGTCTGAAAAATGTT
TTTGAATATAAAATCCTGATTGGTGACTACCAGTAGTACCAACATCATTGGGACGAATAT
AACGACAGTATACTGCAATTGATTTATTTGCTGTCTGTATTGCTGAAGCTACTAAGTCAT
CGGATTTATTTACTACTTGTATTGTCTGAGCTGCCAAGTCATTCATTTTTTAACTCATTT
ATAATTTGTTTAGCAATAGCTTGAACCAACGGTACAGCAATTGAATTGCCAAACTGCTTG
TATGCAGCTGTCTTGGATACTGCATCAATAACAAAATCTTTAGGAAATCCCATTAAACGC
GAGCACTCCCTAGGTGTCAGCTTCCTAGGATTTTTTCCTTTCTGAGGGATGAGTATTTCG
GAACCATCTTTGTAATATCGTGCAGATAGAGTTCGTGATATTCCATCTAAATCAACTAAT
CCAAAACCAAATCCATTACCCTTTGCCTTATGTTTTTTAGCGTAATTTTGAAGGTAAAGC
CATAAGTTATCAGAAAGAGTAAAAGAATTATCTACATCATCTTCCAAAATTTGCTTTAAT
TTTGGTTGTGATTCTGGTGGGGAAGGAAAATTGAAATTTATTTCCTTATTAAAATATTGT
CTATCAAAACCTACAATAAAAATACGCTCCCTATTTTGAGGAACATAATATTTTGCATTC
ATAACTTGATAAAATATCTGATAGTCAAGCTCTTCTAAAGTCCCTTTAATTACTTTAAAT
GTATTTCCTTTGTCATGCGAAACAAGGTTTTTCACATTCTCTAAAAGAAAAATTTTAGGT
CGATGTTTTCCAATAATTTCAGCAACATCAAAAAATAGAGTTCCCTGCGCCTTATCTAAG
AAGCCTGTTTCTCGTCCTAGGCTTTTTTTCTTTGAAACACCAGCTATAGAGAATGGCTGA
CACGGGAATCCTGCTGTTAATACATCAAACTTACTTGGAATAGCTGCTTTGGTTTCCTTT
AATGTAATATCTCCATAAGGAATATCATTAAAATTTACTTGGTAGGTTTGACGGGCTTTA
TCATCCCATTCACTAGAAAATACACATCGCCCACCAACATTCTCCATTGCAATGCGAAAA
CCACCTATTCCCGCAAATAAATCAATAAAAGTAAATTTCTCATTATTTAAGGTAACTATA
TTATCTAATTGATTTTGTATTTTATTTTTCATATTTTATATTTCAGATGATTTATTAATC
TAAAGGCCATCTGAAAACTCCCCCTTTCATCAAATCTTACAAGCCCCGCCCGCGCAGCCG
TCATCCTGAATATCGGTCTGCGTATCGTCCGCACCGTCGCGGGTGTTATGGTAGTACAGG
GTTTTGACGCCGTATTTGTAGGCGGTCAGCAGGTCTTTGAGCATTTGTTTCATAGAAACT
TTGCCGCCTTCGAATTTGCCCGGGTCGTAGGCGGTATTGGCGGAAATCGATTGATCGACG
AATTTTTGCATCACGCCGACAAGTTTCAGGTAGCCTTCGTTGCCGGGAAGCTGCCACAGG
GTTTCATAGGCATTTTTCAGGGTTTCAAACTCCGGCACGACTTGTTTCAAAATGCCGTCT
TTCGATGCTTTGACCGTTACCAATCCGCGCGGCGGCTCGATGCCGTTGGTGGCGTTGGCG
ATTTGAGAGCTGGTTTCAGACGGCATGAGCGCGGTCAGAGTAGAGTTGCGCAGGCCGTAT
TTGACGATTTCGGCACGCAGGCTTTCCCAGTCGTAATGCAAAGGCTCGCCGCAGACGGCA
TCCAAATCTTTTTTGTAAGTGTCGATGGGCAGTTTGCCTTGCGAATAAACGGTTTGGTTA
AAGAGCGTGCACGCACCGTATTCTTTGGCAAGGTTTGCCGATGCTTTGAGCAGGTAATAC
TGTATGGCTTCAAAGGTACGGTGGGTCAGACCGAGCGCGGAACCGTCGCTGTAGCGGACA
CCGTTTTTCGCCAGATAATAAGCATAGTTAATCACGCCGATGCCGAGCGAACGGCGGCCC
ATAGTAGAGGTACGCGCGGCTTCTACCGGATATCCCTGATAATCTAAAAGTGCATCGAGC
GCACGAACGGTCAAGTCGGCAAGCCCTTCCAATTCGTCCAAGCTGTTTAATGCGCCCAAG
TTAAAGGCAGACAGTGTACACAGGGCGATTTCGCCGTTCGGATCGTTGATATTGTCCAGC
GGTTTGGTCGGCAGGGCGATTTCCATACACAAGTTGGACTGATGAACAGGCGCGACGCGC
GGATCGAACGGGCTGTGCGTATTGCAGTGATCGACGTTTTGAATGTAGATGCGCCCGGTT
CCGGCACGCTCCTGCATCAGCGTGGAAAACAGGTCGGCAGCCGGAATGATGCGCTTGCGG
ATATCAGGGTCTTGCTCGTATTTCGTATAGAGCCGCTCAAATTCGTCTTGGTCGGCAAAA
AACGCTTCGTACAATCCCGGAACCTCGTTGGGCGAAAACAGCGTAATGTTGCCGCCCTTA
ATCAGGCGGGTGTACAGCAGGCGGTTGATTTGCACGCCGTAATCAAGCTGACGGATACGG
TTGTCTTCCACACCGCGGTTGTTTTTCAACACCAGCAGGCTTTCGGCTTCGATATGCCAC
AAGGGGTAGAACAAGGTTGCCGCGCCGCCGCGCACGCCGCCTTGCGAACAGGATTTGACC
GCCGCCTGAAACATTTTAAAGAAGGGAATGCAGCCGGTATGCCGCGCTTCGCCGCCCCGG
ATTTCGCTGTCCAAACCGCGGATACGTCCGGCATTGATGCCGATGCCCGCACGCTGGGAA
ACGTATTTCACAATCGCGCTGGTAGTGGCATTGATGGAATCCAAACTATCGTCGCATTCA
ATCAGCACACAGCTTGAGAACTGGCGCGTAGGCGTACGCACGCCGCTCATAATCGGAGTC
GGCAGCGATACTTTAAATGTAGAAACGGCATCGTAAAACCGTTTGACGTAACCCAAGCGC
GCCTCTTTCGGGTATTTGCTGAAAAGGCACATCGCCACCAAAACATATAAAAACTGCGGC
GTTTCGTAAATTTGGCGGGTAACGCGGTTCTGTACCAGATATTTGCCTTCGAGCTGTTTG
ACAGCGGCATAGGAAAAGGACATATCGCGTTCGTGGTCGATATAGGCGTTCAGTTCGTCA
AATTCTTCGCGGCTGTAATCCTCAAGGATATGCCTGTCGTATTTTCCGGCATCGGTAAGT
TTTTTAACGTGGTCGTAAAGGTGCGGCGGCTCGTACTCGCCGTAGGCTATTTTACGAAGA
TGGAAAATCGCCAAACGCGCGGCAAGGTATTGGTAGTCCGGGGTATCTTCCGAAATTAAA
TCGGCAGCGGCTTTGATGATGGTTTCGTGGATGTCGTCGGTGCGGATGCCGTTGTAGAAC

## Appendix A

TGGATGTGCGATTTCAACTCGACCTGCGACACGGAAACATTTTCCAATCCGTCCGCCGCC
CAAGTGACGACACGGTGAATCTTATCCAAATCAATGGCTTCTAATCTTCCGTCTCGTTTG
GTTACTTTCAAATCAGTCGGTGTATTCATCGCTTCCTCTTCCACTCTTGATATTCAAGAC
ACAGTCTTTTCAAATAAATTAAGGCAGACAATATAGTGGATTTTTGGCATTTTCTCCAGT
CTTGACAACGGTTGTATTTTTCAGTTTCAGGCAATGCGCGATAAAACCCGCCTGTCGTAT
TTTTCCTATAATATTTGTTTTATCTGATAATTCTTTACCGATAAAAAACGGGTAAATTTT
TTGCCTTTTGACCGGCTCCGGCTACAAGGCGGTGAAATAAGGATTTTCCGACGAAAAAGG
AAAGCTTCCTGTTTTCTGCCCCTGCAAATTGTTAAATTTTGCAAAGTATGATTTTGCCGC
GCCGCCGCCGACAAATTCCATTTTCTTACCGATTGGAATTTATTATTGAGATTAATGTGT
TATTTGAATCTGCATATCAAACGGCAAGTTTTGTCGGCTGAATGAGTCTGAAACTGCCGA
CAATTTGCCCGTTCCATCCTTCCATCCTATACTGGAAAGAATGACAAACTGAAAGGATCG
TCATGTCTGTCAAAAAGATGCTTGCCATACTGTTGTCTGCAATATTGGGACTGGTATCGA
CAACTGCCGCTGCCGGTACGTCAGAACCCGCCCACCGCGATACCAAACATATCCGCAAGG
CAAACAAGCAGATGCTGCACCCCGAATGCAGGAAATATTTGGAACGCCGTGCCGCGTGGT
ACCGATCGCAAGGCAACGTGCAGGAATTGCGCGAAAACAAAAAGGCGCGCAAAGCATTCC
GCTCCCTGCCTTATGCGGAACAGAAAATCCAATGCCGGGCGGCTTATGAGGCTTTCGATG
ATTTCGACGGCGGCAGTTTCCGCCGTTAATCCCATATAAAATATGCCGTCTGAACACAGG
TTCAGACGGCATTTTCCATAGATACAACAAACTATCCTTACGCCATTTCGTTTGAGGTCC
TCCCGTTGCCGCAGCCTTAATATAGTGGATTAACAAAAATCAGGACAAGGCGACGAAGCC
GCAGACAGTACAGATAGTACGAAACCGATTCACTTGGTGCTTCAGCACCTTAGAGAATCG
TTCTCTTTGAGCTAAGGCGAGGCAACGCTGTACTGGTTTTTGTTAATCCACTATACATCC
ACAAAAGCGAATCATACTGCCACATTCCGACAACCGGTTTCAGACAGCGATATCCGCATC
GTCGGCGACAACGGCACGGATGCTTTCTTCGATTTTATCTTTTAAAGCATAACGGTCTTC
GCTTTCCGCCGCATCCGCCACGCAAACGAAATCGACTCTTATCGTCAATTTTTTCATAGA
CACGATGCGCCACAGGCAGGTCGGCAAACCGACATCGGCATATGAGGGACGAGCCGTCCT
TTTTCCCGTTTCGTCATAATAACGCAGCGCGACCGCCAAAACCTTTGCCCCCGCATCGAT
GGCGGATTGGAACAGCGCGGCTTTGAACGGCAAAAGCCCCAATCCGGAGGAAGTCCGCGC
TTCGGGGAAAAAACTGACGTTTTGACCGCGTTGCAAGGTTTCGCAGACGGCGCGGTTAAT
CGGTTCGATGTCGCGCCGCGAATTGCGGTTGATGAACACCGTTCCCGCGTTCTGCCCCAT
CTTGCCCAATACCGGCCAGCTTTTGATTTCCTGCTTGGCGATAAAGCTGCTCGGATAAAC
CGCGCTCATCGCGAAAATATCCAGCCAGGACACGTGGTTGGCGGCAACCAAGACACCGTT
CGGATGTTCGGGTGCGGGTCTGCCCACCTCCAATCCGATATCCAAAGCCGCCAAAACCCC
CCTGCCCAACTCGATTACCGCCCGATTGCGCGACTCGGGGCAACCGCCGTCAATACCGCG
CAGGTTTTTCCCGGTTTTGAACAGCCAGACCGCCAAACGGCACAAGCGGCGCAGACGTGT
AAAAAATGAAGCTTTATTTGAAGACATGAGCAATTCTTTCCCGATAATCGGTGTTTGTTG
TTGAAACGTTCGGACGGCATACCGATAAAATGCCGTCTGAAACCGTTTTCAGTCCTATTT
CTGACAGTTCGGGCAATAAAACGTGCCGCGCTGCCCCAAAGTTTCTTTCACAACCAAACC
GCCGCACCGGGGGCACGGCTGATTGTGCCGCCCGTACACTGTATATTCCTGTTGGAAGTA
GCCGCTTTTGCCGTCGCTGTCCACAAAATCCCTCAAGGTACTGCCGCCCGTTTCAATGGC
GCGCTGCAACACCGCTTTGACGGTTTCAACCAAAAGCGCGCACTCTTTCTTTTTCAGGCG
GTTGGCAGGACGGTGGGGCGAAATGCCCGCTCTGAACAGGCTCTCGTTGGCATAAATGTT
GCCCACACCGACCACGACCGCATTGTCCATCAGGGCAAGTTTGACCGCGCGCTTCTGCGC
CTTCAGCCTTGCATACAGATAATCCGCACAAAATGCCTCCGACAAAGGCTCCGGCCCCAG
TTTTTCCAACAGCGGATGATGTTCTTCGATTCCCTCATACCAAAGTATCGCGCCGAACTT
TCTCGGATCGCGGTAACGCATGACCGTGCCGTCTGAAAACACAATATCGACGTGATCGTG
TCTGTCCGGCCTGCCGATACGTCCGTCCGACGGCGTAAAAATCCGCAAGCTGCCCGACAT
CCCCAAGTGAATCAGCAGCACGCCCGTTTGAAAGCGGATAAGCAGGTATTTCGCCCTCCT
GCCGCAGGACAACACCTGCCGGCCGGACAAAATCTCCCCCAAATCGGGATTAATCTGCCA
~~GGGCAGCTTGAATTGGGGCAATACCACGGCTTCCACCGTTTTCCCTTCAATATGCGGCGC~~
GATGCCGCGCAACGTCGTTTCCACTTCCGGCAATTCAGGCATAACCCCTCCCGACATTTC
TTCTGACAGATGCCGTCTGAAAGACGGCTGTCCCTAATCCGCAACCCTTGCCGCACCCGC
CGCAAGGGCTTTGCCGCCCAAATACCCGTCCCACGCGGGCAGGGGCGTTACGCCCGCTGC
CCTTTTGACCAAAACCAAACCGTACACTGCGGCACACTGCGGCCACCAACGGTCGCCCGC
CTTTTCCATAAACCGCCAAAAGCGTATTTGCCCGAGCGACGAAACCGGCGGCAGATACAC
CATAAATTTCCCAAATTCAATATCGAAACCGACATCCGCAAGCCGTCTTTTCAACTCGGG
CAGCGGCAGACAAAACCGTTTTTCCGGCAGGCGTTCGCCGTCAAACCAACGGCTGAATCC
CCAGAGCGAATACGGATTGAAACCCGTCAGCATCAAGCGTCCGCACGGTTTCAATATCCG
GTGCGCTTCCGACAGGATTTGCGAAGGAACACCGCCTTCAAGCGTATGCGGAAAAAGCAG
CATATCCGCAGAAACATCCGCCAAAGCCATATTCTCCGCCGACATCGACATATCTCGCGG
CACACAGACAACATCTTCAGACAGGCTCAGCCACGGACCGCCCACCTGAACCGCACACAT
TCCCGAAAAACGGTATGAATCCAGATACCGCCCGAAGAAATCCTGTTCCAATTTTGCAAC
ATACCGCCCCATCGCCGTATCTTCAAACCATGCATCCATATTGCGTCCGTTTCAAACAGA
TTGCCTGCCGATATTCTATTCCAAACAGGATTTCTGTCAAAAAACACATCGGCCGCCCAT
TTCCGAATCCGCATAAAGTTCCTGTAAAACTTGACGCTTTTTCAGTCAAACAGTACCATC
GGACGATAAAATATGTTTATTCCGCCGAATATAAATCATGTCCAAACTCAAAACCATCGC
TCTGACCGCATCAGGTCTGTCCGTTTGTCCGGGTTTCCTATACGCCCAAAACACCTCATC
ACACCAAATCGGTTTGGCGATTATGCGCTTAAACTCTTCAATACTCGACCTGCCCCCGAC
AAAACAATATTTCCAATCCGGCAGCCTGTGGGGCGAGCTGCGCCAAGGCTTCCGGATGGG
CGAAGTCAATCCCGAACTGGTACGCCGCCACGAAAGCAAATTCATCGCAAGCCACAGCTA
TTTCAACAGGGTCATCAACCGGAGTAGACCCTATATGTACCATATCGCCAACGAAGTCAA
AAAACGCAATATGCCCGCCGAAGCCGCCCTGCTTCCCTTCATCGAAAGCGCGTTCGTCAC
CAAAGCCAAATCACACGTCGGCGCATCAGGATTATGGCAGTTTATGCCCGCTACCGGCAG
GCATTACGGCCTGGAAAAAACACCGGTTTACGACGGCAGGCACGACGTTTACGCCGCCAC
CGATGCCGCACTCAACTATCTGCAATACCTCTATGGACTGTTCGGCGACTGGCCGCTTGC
CTTTGCCGCCTACAACTGGGGTGAAGGCAACGTCGGACGCGCCATCAACCGCGCCCGCGC

## Appendix A

CCAAGGGCTCGAACCGACCTACGAAAACCTGCGTATGCCCAACGAAACGCGCAACTATGT
CCCCAAGCTGCTCGCCGTGCGCAACATTATTGCCACTCCCCAATCTTTCGGCATGAATAT
CAGCGACATAGACAACAAACCCTATTTTCAGGCAGTCGAACCGGATCGTCCGCTCGACAA
CGAAGCCATCGCCCGGCTTGCCGGCATCACGCAAAGCGAGCTGCTCGCCCTAAACCCCGC
ATTCAACGTCCCCGCGTTTATCCCCAAAAGCAAACGCAAACTGCTGCTTCCTGTCGCGTC
CGTACAAACCTTCCAAAGCAACTACCTCAACGCCGCACCCGACAGCCTGTTTTCATGGGA
AGTCTATACGCCTGCCGCCAAAACCAGCCTGTCCGACATCTCGACGGCAACCGGCATGAG
CATTGCCGACATCAAACGCCTCAACAACCTGAACGGCAACCTTGTCAACGCAGGACGCAG
CATCCTTGTCGCCAAGAACGGCAAAACCCTTCAGACGGCATCGGAATCCGTCGTTTCCAT
CGACATCGACAATACGCCCGACACCTACCGTTCCAATATGCCGGCAGGCACGGTGAACGT
CGGCATTGCCCGAATCCGACCCGCCGCCGCACAGACAGCGGACATTACCGTCGCACCTTT
GCCGCAGAAAACCGTCCGTACGGAACCCGATCCCCTTGTCCGTATTGCCGAACCTGCCCT
TGCGACAGCCGCAGCGCAACCTCAAACCGAAAAACAGACCGCCATGCCGTCTGAAACCCA
AACCGCAACACTCGCGCAGATCATCCCCCAAAACGACATGCAGGCGGCAGACGAACTCAT
GCAGCTTGTTGCCCGAAACAACCTGCGCCGACAGGCTGAAGAAACCATCTCCGCCGTCAT
CGGCACGCCTGACACAGTTGCCGAACACAAAATTTCCGCATCTCCGCAACATACCGCTGC
CGCCGACGGCAAACGCCGGGTACGTTTGGAAACGCGCGTAGCCAAAGCTGCCGACGGCGA
AGCCGAAATCTCCCCGCTCCATGCCAGCATCCACCGCGTTGTAGAAGGCGACACCCTGTT
CAACATTGCCAAACGCTACAACGTCAGTGTAGCCGACCTGATTGTCGCCAACAACATCAA
AGGCAACACCATCCAAAAAGGACAGGTACTCCGCCTGGCGCAGGCAGCCCCTGCCCAAAC
CCGTATTGAAAAAGTATCCTACACCGCGCGCAAAGGCGACACCTTCAAAAGTATCGCCGC
GCGCTTCAATATCCATATCGACGACATCCGCCGGCTCAATCCCAACCTGAACACCATCAA
TCCGGGACAGAGGGTCAAACTGATTGGAAGCTGATTCGGATACGGCACATGACAGGACTT
TCTCAGTCCTGTCTTTTTCATCCCACATTTCCCATACCATCATGAAACTTATCAAATACC
TGCAATATCAAGGCATAGGAAGCCGCAAGCAGTGCCAATGGCTGATTGCCGGCGGTTATG
TTTTCATCAACGGAACCTGCATGGACGACACCGATGCAGACATCGATTCCTCATCCGTCG
AAACGTTGGATATTGACGGGGAAGCAGTAACCGTCGTTCCCGAACCCTATTTCTACATCA
TGCTCAACAAGCCTGAAGATTACGAAACTTCGCACAAACCCAAGCACTACCGCAGCGTAT
TCAGCCTGTTCCCCGACAATATGCGGAACATCGATATGCAGGCGGTCGGCAGGCTGGATG
CAGATACGACCGGCGTATTGCTGATTACCAACGACGGCAAACTGAACCACAGCCTGACTT
CGCCGAGCAGAAAAATTCCCAAGCTGTACGAAGTAACGCTCAAACACCCCACAGGAGAAA
CGCTCTGCGAAACCTTGAAAAACGGCGTGCTGCTCCACGACGAAAACGAAACCGTTTGTG
CCGCCGATGCCGTTTTGAAAAACCCGACCACCCTGCTGCTGACCATTACCGAAGGAAAAT
ACCACCAAGTCAAACGCATGATCGCCGCCGCCGGCAACCGCGTGCAACACCTTCATCGCC
GGCGATTCGCACATCTGGAAACAGAAAACCTCAAACCCGGGGAATGGAAATTTATCGAAT
GTCCAAAATTCTGAAATAACATCCAAGAATTCCATTTATATTCATCCACATACTCATTAA
ATATATGGTTTAACCCAATTTAACGCAAAAAATCAATTTACGATATAATCCATTTGTCTG
AGTAACGCCTGTTCAAGCAGGCTTATGAGTAAGACGTTTTCCCCGTAATGTGTTTTGCCA
TCTATACTTCTCCCCTTGTATAGATGGTTTTTTTATAGTGGATTAAATTTAAACCAGTAC
AGCGTTGCCTCGCCTTGCCGTACTATTTGTACTGTCTGCGGCTTCGTCGCCTTGTCCTGA
TTTTTGTTAATCCACTATATTTCAAATTCCCTCTCTTTCATCATTATTTAAATGCTATCA
TTAATATAATTAACAAAATTTCATTTCCATACACCGATGTAGAAGAATAACAAATTAATC
TATTTATAAACTCCGATTCTTCCTCATCGGGTTATACTCATCAGAAATACTCACTGAAAT
ATATGCCTTTTACATGGAAAATCACTAGACAGTTGAAAAGCAATCAGTCAGTTCCTGTTA
AAAAACGCGTGTAGAATTTCCGCTTTATCTCAATCGGACACGGTTTCAATATGTCTTCCC
CTTCAAATACCAATCGTCAAACCTGGTCCAGCCGATTAACCTATATCCTGACCGTTGCCG
GCGCGACTGTCGGTTTCGGCGCGACGTGGCGTTTCCCGTATTTGGTCGGTGAAAACGGTG
GTGCGCGTATGTGTTTTTATTCTGTATCGCGATGCTGGTTATCGGCATCCCGATGATTTT
GGTGGAAAATGTCATCGGACGGCGCAAAGGCGTGAACGCGCTGGATGCGTTCGGCGGCCC
GATGAACGGCAAACCCATTGCCAAAATTTGGAAACTGGTCGGCTGGATGGGCTGCTCGGC
GCGTTCGGCATCATGGCTTATTACATGGTACTCGGCGGCTGGGTAATCAGCTATATCGTT
AATATTATTGGAGGAAATTTGAATATTTCCAGCCCCGTCGACGGTGTGGTTACAAAAGGC
TTCTTTGCCGAACACATTGAAACAGCCCTTGGGAAATTGCGTTTTATACGCTGCTTTTTG
TCGCCGTGAACCAATGGATTTTGGTCAAAGGCGTTATCGGCGGCATTGAAAAAGCGGCAA
AATAGCTGATGCCGCTGCTGTTTTTGTTCCTAATCGCGATGGTCGTCCGCAACGTTACCC
TTCCGGGCGCAATGGAAGGGGTTGCTTTCTATCTGAAACCTGATTTCAGCAAGATTACCG
CCGAACTGTTCGTCTTCGTTTTGGGGCAGGTATTTTTTGCCCTGAGCTTGGGTTTCGGCG
TGATGATTACCTTGTCCAGCTATTTGGATAAAAACGAAAATCTGGTTCAGACGGCAGTTA
TCACGGCAATTACCAATACCATCATCGCCATACTTGCGGGCTTTATGATTTTCCCGTCGC
TCTTCAGCTTCGGCGTTGCCCCCGATTCCGGCCCGACTTTGGTGTTCCAAAGCTTGCCGA
TTGTGTTCTCACATATGTGGGCGGATCTGTGTTCGCCGTGATTTTCTTCTCGCTGCTCC
TGATTGCCGCGTTGACAACTTCGCTGACCATTTATGAAGTGTTGATTACGACCATTCAGG
AAAAAACCAAAATCCGCCGTACCGCCGCGATTACGATTGTATTGGCTGCCATCTTCATTT
TCGGCAACATCCCGTCCATTCTGAGCTATGGTCCGTGGAAAGACGTTCCGTGTTCGGCAA
AAATATTTTCGATGCCTTCGACTACATCAGCGGCAACATCTTGTTTATGCTGACCGCGCT
CGGTTCCGCGCTGTTTGCCGGTTTTGTGATGAAGGACGAAGCGAAGGACGAATTGCTTTA
TAAAGGCAACCATACGACGGTCAATATTTGGTTTGCTTATGTGAAATATCTTGTGCCGCT
GGTGATTCTGCTGATTTTCGTCAGCAACCTGTTCTAATCCGCAGCAATCGATGCCGTCTG
AAGGTCATACCTCTTTCAGACAGCATTGCCTTGATGCCCGCCACAATACCCGCAACGCCG
AAATCCGAACCGGCTTGCCGGCTTCTGTCGGATGTTTCCGGGCAGGCGGCGTTCCGTCTG
CTTAGACAATCGTCCTTTAAAACAGGTAGAATCCGCCCCAACGGGAAACACATCCTTCAG
ACGGCAAAACCCATACCCCAAACCATCAGGAATCCCCCTTATGAACAGACAAAAAGTCAT
CGCCATCGACGGCCCGGGCGCATCGGGCAAAGGCACGGTTGCCGCCCGCGTTGCCGCCGC
ATTGGGATACGATTATCTCGATACCGGCGCACTCTACCGCCTGACTGCCCTATATGCACA
AAAACAAGGCGTGGATGGCACGATGAAGAAAACGTTTCCGAACTGGCAAAAAAACTGCC

# Appendix A

CGCCGTATTTCAGGCAGCCGCATCCTGCTCGGCGGCGAAGACGTTTCAGACGGCATCCG
GACAGAAGCCATCGGCATGGGCGCATCCGCAGTCGCACAGTTGCCTAAAGTCCGCGCCGC
CCTGCTGCAACGCCAACGCGATTTTCTGACCGAAAAAGGACTGGTTGCCGACGGACGGGA
CACCGGATCGGTCGTCTTCCCCCAAGCCGAACTTAAAATCTTCCTGACGGCAGAATCCAA
AATCCGTGCCGAACGCCGCGCCAAACAAATCGGCATCCCCTGCGAAGGTTTGGCATTCGA
GCGCATCCTGTCCGACATCGAAGCCAGAGACGAGGCAGACCGAAACCGCAAAGTTGCCCC
CCTGAAACAACAGCCCGATGCCCTGCTTTTGGACACAAGCCGCCTGACTATAGAAGAAAC
TGTAAAAAAAGTGCTTGATTGGTATCGTGAAGTTTAAATTTTCAGGTATAATCGCACAAA
TTACGTTTCAGACGGCATAAAAATCCCCCATATGCCGTCTGAAACCTTATGTACCCGTCT
GCCCTGCCAAGGACGGCAGATATCCACCAACCCACCCCGCACCCCTTGGCGGTGTACCGA
AAAGAGTTATATATGTCTATGGAAAATTTTGCTCAGCTGTTGGAAGAAAGCTTTACCCTG
CAAGAAATGAACCCGGGTGAGGTGATTACCGCTGAAGTAGTGGCAATCGACCAAAACTTC
GTTACCGTAAACGCAGGTCTGAAATCAGAATCCCTGATTGATGTAGCTGAATTCAAAAAC
GCTCAAGGCGAAATTGAAGTTAAAGTCGGCGACTTCGTTACCGTTACCATCGAATCCGTC
GAAAACGGCTTCGGCGAAACCAAACTGTCCCGCGAAAAAGCCAAACGTGCAGCCGATTGG
ATTGCCCTGGAAGAAGCCATGGAAAACGGCGACATCCTGTCCGGCATCATCAACGGAAAA
GTCAAAGGCGGCCTGACCGTTATGATTAGCAGCATCCGCGCATTCCTGCCGGGTTCTTTG
GTCGACGTACGTCCTGTAAAAGACACTTCTCACTTCGAAGGCAAAGAGATCGAATTCAAA
GTGATCAAACTGGACAAAAAACGCAACAACGTCGTTGTTTCCCGCCGCGCCGTTCTGGAA
GCCACTTTGGGTGAAGAACGCAAAGCCCTGCTGGAAAACCTGCAAGAAGGCTCCGTCATC
AAAGGCATCGTTAAAAACATTACCGATTACGGTGCATTCGTTGACTTGGGCGGCATCGAC
GGTCTGTTGCACATCACCGATTTGGCATGGCGGCGCGTGAAACACCCGAGTGAAGTCTTG
GAAGTCGGTCAGGAAGTTGAAGCCAAAGTATTGAAATTCGACCAAGAAAAACAACGCGTT
TCCTTGGGTATGAAACAACTGGGCGAAGATCCTTGGAGCGGTCTGACCCGCCGTTATCCT
CAAGGCACCCGCCTGTTCGGCAAAGTATCCAACCTGACCGACTACGGCGCATTCGTCGAA
ATCGAACAAGGCATCGAAGGTTTGGTACACGTCTCCGAAATGGACTGGACCAACAAAAAC
GTACACCCGAGCAAAGTCGTACAACTGGGCGACGAAGTCGAAGTCATGATTTTGGAAATC
GACGAAGGCCGCCGCCGTATCTCTTTGGGTATGAAACAATGCCAAGCCAATCCTTGGGAA
GAATTTGCCGCCAACCACAACAAAGGCGACAAAATCTCCGGCGCGGTTAAATCCATTACC
GATTTCGGCGTATTCGTCGGCCTGCCCGGCGGCATCGACGGTTTGGTTCACCTGTCCGAC
CTGTCCTGGACCGAATCCGGCGAAGAAGCCGTACGCAAATACAAAAAAGGCGAAGAAGTC
GAAGCCGTCGTATTGGCAATCGACGTGGAAAAAGAACGCATCTCCTTGGGTATCAAACAA
CTGGAAGGCGATCCGTTCGGCAACTTCATCAGCGTGAACGACAAAGGTTCTTTGGTTAAA
GGTTCCGTGAAATCTGTTGACGCCAAAGGTGCTGTTATCGCCCTGTCTGACGAAGTAGAA
GGCTACCTGCCTGCTTCCGAATTTGCAGCCGACCGCGTTGAAGATTTGACCACCAAACTG
AAAGAAGGCGACGAAGTTGAAGCCGTCATCGTTACCGTTGACCGCAAAAACCGCAGCATC
AAACTTTCCGTTAAAGCCAAAGATGCCAAAGAAAGCCGCGAAGCACTGAACTCCGTCAAT
GCCGCCGCCAATGCGAATGCCGGCACCACCAGCTTGGGCGACCTGCTGAAAGCCAAACTC
TCCGGCGAACAAGAATAAGGTTGCAGACATGACAAAGTCTGAGTTAATGGTTCGTTTGGC
AGAAGTGTTTGCCGCCAAAAACGGCACGCATCTTCTGGCAAAAGACGTAGAGTACAGCGT
AAAAGTCTTGGTTGACACCATGACTAGATCGCTTGCCCGAGGTCAACGCATCGAAATCCG
CGGTTTCGGCAGCTTCGATTTGAACCATCGTCCTGCCCGCATCGGTCGCAATCCCAAAAC
CGGCGAGCGTGTGGAAGTACCTGAAAAACATGTACCCCACTTCAAGCCCGGTAAAGAATT
GCGCGAGCGGGTCGACTTGGCTTTAAAAGAAAATGCCAATTAAACCTTAGCATCAAAACG
CCGCTGTTACGCGGCGTTTTTTCTGTGGTTTAACTTCATCCGTTGCTTCAATACCTTGAG
CCAAGCAAGCAACGGATTAGAGCGTGGATTTTTTTATAGTGGATTAACAAAAACCAGTAC
GGCGTTGCCTCGCCTTAGCTCAAAGAGAACAATTCTCTAAGGTGCTGAAGCACCAAGTGA
ATCGGTTCCGTACTATTTACACTGTCTGCGGCTTCGTCGCCTTGTCCTGATTTTTGTTAA
~~TCCACTATATCATTGCTTACAATCCGCTTTTTAAACAACAAATTTTTGATTTGTATTAGG~~
AACAGGACAAAAATCCTGCTTATTGCACTAAAACTAAGCCGTTTCAGGAATTTGCGGCAA
ATTTACAGCTTTTACCGAGCCTAATGCTTTCGCTTTTTGGTAAAACGCCAATTTGTATTC
AAGCAAATCTAAATAGCGTTTTAATTCGGCAATTTGACACTTCACATTTTCTATTTGATT
TTCAAACAAGGAAAGGCGTTCTTCAATGGTATCGTCGCCAATGACGGTACATTCCGCAAA
GCGTTTGATGTCTTTTAAGCTCATTCCCGTATTTTTCAAGCATTGCAATAAGCCCAACCA
TTGCAAATCGTTATCGGTAAAACAGCGGTTACCGTATTCATCACGTCCGATATTGGGCAA
CAAACCTTCTTTGTCGTAAAAACGTAGGGTGTGGGCGGAGATGCCTATTTTTTCGGCGGC
TTTGGCAGTAGTATAAGTCATTTTCCCTCCTTCTAAACAAAAACAGTAAAAAACACTTGC
TTTAGAGTTAACTCTAAAGTGTAAACTGTTGCTATGTTGCTCAGGCAAGGCAACTTTGTC
AATGAATTAAGAGGAAAGACAATGGAAATGAAACAAGCCGATTCAACCATCAAATCTCGT
GCGGCGGTGGCATTCGCCCCTAACCAACCCTTACAAATTGTGGAAATCGACGTAGAAATG
CCGCGTAAAGGCGAGGTGTTAATCCGCAATACCCACACTGGCGTGTGCCATACTGATGCG
TTTACGTTATCAGGAAGCGATCCTGAAGGCGTATTCCCTGTGGTGCTTGGACACGAAGGT
GCGGGTGTGGTCGTTGCTGTGGGCGAAGGTGTGTCAAGCGTAAAACCGGGTGATCACGTG
ATTCCGCTTTACACCGCCGAATGTGGCGAATGTGAGTTTTGTTGTTCAGGTAAAACCAAC
TTGTGCGTCTCAGTGCGTGATACACAAGGTAAAGGCTTAATGCCGGACGGCACGACGCGT
TTTTCTTATCAAGGTCAGCCAATCTATCACTATATGGGCTGTTCGACTTTCAGTGAATAC
TCCGTTGTTGCCGAAGTTTCACTGGCGAAAATCAACCCTGAAGCCAACCATGAACAAGTA
TGTTTGCTCGGCTGCGGCGTTACCACAGGTATTGGTGCGGTACATAATACGGCAAAAGTG
CAAGAAGGCGACTCTGTTGCCGTGTTTGGTTTGGGGGCGATTGGTTTGGCGGTGGTGCAA
GGTGCGCGTCAAGCCAAAGCCGGCCGCATTATCGCCATTGATACCAATCCATCAAAATTT
GAGTTGGCAAAACAGTTCGGTGCAACGGATTGTTTGAACCCGAACGATTACGATAAACCG
ATCAAAGATGTGTTGTTAGACATTAATAAATGGGGCATTGACCATACCTTTGAATGTATC
GGCAATGTAAACGTAATGCGTCAGGCATTAGAAAGTGCACATCGTGGTTGGGGTCAATCC
ATTATCATCGGCGTAGCAGGTGCAGGACAAGAAATTTCAACGCGTCCGTTCCAGTTGGTA
ACAGGTCGTGTTTGGAAAGGTTCAGCATTTGGCGGTGTGAAAGGTCGCTCTGAACTGCCG

# Appendix A

AAAATGGTGGAAGATTCAATGAAAGGCGACATTGAGTTAGAACCGTTTGTAACCCACACA
ATGACACTCGATCAAATCAATAAAGCCTTTGACTTAATGCACGAAGGTAAATCGATCCGC
GCCGTTATTCACTACTAAGGTATGCGATGAAACTGATTGAACAACATCAAATTTTTGGTG
GTTCGCAACAAGTTTGGGCGCATCATGCCCAAACGCTGCAATGCGAAATGAAATTTGCCG
TCTATTTGCCAAATAATCCAGAAAATCGACCGCTTGGTGTGATTTATTGGCTTTCCGGCT
TGACGTGTACGGAACAAAATTTCATTACCAAGTCAGGCTTTCAGCGTTATGCGGCAGAAC
ATCAAGTAATTGTGGTGGCCCCCGATACCAGCCCTCGCGGAGAGCAAGTGCCGAACGATG
ATGCTTACGATTTAGGACAGAGTGCAGGCTTTTATTTGAATGCGACCGAACAGCCTTGGG
CGGCGAATTATCAAATGTATGATTACATTTTGAACGAGCTGCCCCGTCTGATTGAGAAAC
ACTTTCCTACCAACGGCAAACGTTCCATTATGGGACATTCAATGGGCGGACACGGCGCAT
TGGTATTGGCGCTGCGGAATCAGGAACGTTATCAAAGTGTTTCTGCCTTTTCGCCTATTT
TATCGCCAAGCCTCGTGCCGTGGGGAGAAAAAGCCTTTACTGCTTATTTAGGGAAAGACC
GTGAAAAATGGCAGCAATATGATGCTAACTCACTCATTCAACAAGGCTATAAAGTGCAAG
GTATGCGCATCGATCAAGGCTTGGAAGATGAGTTTTTGCCGACACAATTGCGTACCGAAG
ATTTTATCGAAACCTGCCGTGCGGCAAACCAGCCGGTCGATGTGCGTTTCCATAAAGGCT
ACGATCACAGCTATTACTTCATCGCCAGTTTTATTGGCGAGCATATTGCTTATCACGCCG
CGTTTTTGAAGTAAACCAAAGAGCGTTCAGTGTTCAAAGCAGTTTTGGGATAGCCGGCAC
GAGGGCGGTAAGAAGTGCCGGCATAAACGTATGCCGTCTGAGCCGAAAGGAGCCGACTCT
ACGGATTATAGTGGATTAACAAAAATCAGGACAAGGCGACGAAGCCGCAGACAGTACAGA
TAGTACGGCAAGGCGAGGCAACGCTGTACTGGTTTAAATTTAATCTACTATAAAAGGCAT
TTGAGCTCATATCTGCACCATATTGAAACGCCGCCTTTGCTTATACCCCCTTGTGCGCGT
CATTATTCTTTTCCACGGAAAATGCCAAGTTTGAAGGAAATCATTTATAATACCGACGGT
AAGCATTTTCTTTCTTAGCCGCAAGAAGTATAACAAGGTTAAATATGAGTAATAGAGACC
AACTTTTTAAAGCCCCGCCGTTTGAAAACCACAGCCCGCTGACCTGGTATCAGGCTGCCT
CACAACTGCCCAACTTCATCCGCGACGACGCGCAGGCAGCCGCCATCGAACACCTCGATC
GGCTTTGGACCGAATTGATGATGTTCAAACGCAAAAGAAACCGTTTTTTAGGCAGGAGTT
TGCGTTCCCCGCAAGTCCCCAAAGGGCTTTATTTCTATGGCGGGGTCGGACGCGGCAAAA
GCTTTCTGATGGACGCTTTTTTCGGCTGCCTCCCGTACCGCCGCAAACGCCGCGTCCACT
TTCATGCCTTTATGGCAGAAATCCACCAGCGGCTGAAAACCCTGAAAAGCGAAAGCAACC
CGTTGAAATCCGTTGCCGCCGAGATTGCCAAAGAAACCCGCGTATTGTGTTTTGACGAAT
TTCATGTCAGCGATATTGCGGATGCAATGATTTTAGGCCGTCTGCTGGAAAACCTGCTTA
ACGAGGGCGTTGTTTTGGTGGCGACTTCAAACTACGCGCCTTCCGAACTCTACCCGCAAG
GTCAAAACCGGAGCAGTTTTCTTCCCACAATCGCGCTCATCGAGTCCAGCCTGACCGTCT
TAAACGTTGACGGCGGTGAAGACTACCGACTGCGTACCCTCCGCCCCGCCGAGATTTTCT
TTACGCCTGCCAATGAAGAAAATGAGGCAAAACTGGCAAAACTGTTCAAAGAAATGACAG
GCATTACCGATTTGAACCCCGGCATCAGCACCATCCACGGTCGGGAGATTCCCCACAAAG
CCGAGTCCGGCCGTGCCATATGGTTTGATTTCCGCGCACTGTGCTTCGGCCCCCGCTCAC
AGTCCGACTATCTGTATTTGGCCGAACATTATGAAATGGTTTTTATTTCAGGTTTGGAAC
AACTCTCACCGCAAGAAAAGGCGGAGGCGCGGCGGCTGACTTGGCTGATTGACGTACTCT
ACGATTTCCGGGTCAAACTGTGTGCCACCGGCGCGGTAGATGTCAACCATATCTATACGG
AAGGCGATTTTGCCGAAGAATTTACCCGCACCGCCAGCCGGATGGTCGAAATGCAGTCCG
AAGTTTATTTGGAACAGCCGCACCTGACCCTATCTCCCAAGGCTTCAGGCGGATAAGTTA
TTTTTTTGATAGAATACCGATTTGATTCTTCTTAAGTAAAAATAAGGATATAGCATGGCG
ATTGAACGTACCATCTCCATCATCAAACCCGATGCCGTCGGCAAAAATGTTATCGGCAAA
ATATACAGCCGCTTTGAGGAGAACGGTCTGAAAATCGTTGCCGCCAAAATGAAGCAGCTT
ACTCTCAAAGAGGCGCAAGAATTTTATGCGGTTCATAAAGACCGCCCCTTCTACGCCGGA
TTGGTTGAATTTATGACCGGCGGTCCGGTTATGATTCAGGTATTAGAGGGTGAAAACGCC
GTCCTGAAAAACCGCGAACTGATGGGTGCAACTAATCCTTCCGAAGCCGCCGAAGGCACG
ATACGCGCGGACTTTGCCACTTCGGTCAGCATTAATGCCGTACACGGTTCCGACAGCGTG
GAAAATGCCGCTTTGGAAATTGCCTACTTTTTCAGCCAAACCGAAATCTGCCCCCGTTGA
TACAATACACCGCCCAACTCCTCTTCAGACGGCATAAATATATCCATGCCGTCTGAAAAC
TCTGTTGCAAAAGGCTTCAAATCAAACTTGCCTGCCCTGCAATTTTTTATTTGAAGCCTT
GATTTAAGAAAAACACAAACACATGAAAACCAATCTGCTCAACTACGACCTTCAAGGGCT
GACCCGACATTTTGCCGATATGGGCGAAAAACCTTTCCGTGCCAAACAGGTTATGCGTTG
GATGCACCAATCCGGCGCGCAAAATTTTGACGAAATGACCGATTTGGCAAAATCGTTGCG
CCATAAACTGAACGAACAGGCAGGCATCGAAATTCCCAAGCTGATGATGTCTCAAAAATC
TTCAGACGGCACTCGAAAATGGCTTTTGGATGTCGGTACGGGCAACGGCGTGGAAACCGT
CTTCATCCCCGAATCGGATCGCGGCACGCTCTGCATTTCCTCACAAGTCGGCTGCGCTTT
GGAATGTACATTTTGTTCGACCGGCCGGCAGGGCTTCAACCGCAATTTGACTGCTGCCGA
AATCATCGGGCAATTGTGGTGGGCAAACAAAGCGATGGGCGTTACACCGAAAAACGAGCG
CGTGATTTCCAACGTCGTCATGATGGGCATGGGCGAGCCGATGGCGAACTTCGACAATGT
CGTTACCGCCTTAAGCATCATGCTGGACGACCACGGCTACGGTTTGAGCCGCCGCCGCGT
AACCGTTTCCACTTCGGGTATGGTTCCCCAAATGGACAGGTTGCGCGATGTCATGCCGGT
GGCTTTGGCGGTTTCCCTCCACGCTTCCAATGACGAAGTCCGCAACCAAATCGTACCGTT
GAACAAAAAATATCCCTTGAAAGAATTGATGGCCGCATGCCAACGCTATCTGGTCAAAGC
ACCCAGGGATTTCATCACTTTCGAATACGTCATGTTGGACGGAATAAACGATAAGGCGCA
ACATGCGCGCGAACTGATCGAACTGGTCACAGATGTTCCCTGCAAGTTCAATCTGATTCC
GTTCAATCCCTTCCCAAACTCCGGATACGAACGCTCCAGCAATGAGAACATCCGTGTGTT
CCGCGATATTTTGCAGCAGGCAGGATTTGTCGTTACCGTACGAAAAACGCGCGGCGACGA
CATCGATGCCGCCTGCGGACAGTTGGCGGGGCAGGTTCAGGATAAAACGCGCCGCCAACA
AAAATGGCAGCAGATTTTAATCGGACAACAGGGGTAATTATGCCTTTTAAGCCATCCAAA
CGAATCTCTTTATTACTCGTTCTTGCCTTGGGCGCGTGCAGCACTTCCTACCGCCCCTCG
CGGGCAGAAAAAGCCAATCAGGTTTCCAATATCAAAACCCAGTTGGCAATGGAATATATG
CGCGGTCAGGACTACCGTCAGGCGACGGCAAGTATTGAAGACGCCCTGAAATCGGACCCT
AAAAACGAGCTTGCCTGGCTGGTCCGTGCCGAAATCTATCAATACCTGAAAGTTAACGAC

## Appendix A

AAGGCGCAGGAAAGTTTCCGGCAAGCCCTCTCCATCAAACCCGACAGTGCCGAAATCAAC
AACAACTACGGTTGGTTCCTATGCGGCAGGCTCAACCGCCCTGCCGAATCTATGGCATAT
TTCGACAAAGCTCTGGCCGACCCCACCTACCCGACCCCTTATATTGCCAACCTGAATAAA
GGCATATGCAGCGCAAAACAGGGGCAATTCGGATTGGCGGAAGCCTATTTGAAACGTTCC
CTCGCCGCCCAGCCGCAGTTCCCACCCGCATTTAAAGAACTGGCGCGCACCAAAATGCTG
GCCGGGCAGTTGGGCGATGCCGATTACTACTTTAAAAAATACCAAAGCAGGGTAGAAGTC
CTTCAGGCCGATGATTTGCTGCTAGGCTGGAAAATTGCCAAAGCCCTCGGCAACGCACAG
GCGGCATACGAATATGAAGCACAATTGCAGGCGAATTTCCCCTACTCGGAAGAATTGCAA
ACCGTCCTCACCGGTCAATAAACAGATTCAAACCATATGAACACACTCCAACGCCGCAAG
ACGCATCAAGTCCGCATCGATCATATTACCGTCGGTTCAGAAGCACCCGTCGTTATCCAA
TCTATGACCAACACCGACACTGCCGATGCAAAAGCCACCGCATTGCAGATTAAGGAATTG
AGCGATGCCGGATCCGAAATGGTGCGTATTACCGTCAACAGCCCCGAAGCCGCGTCCAAA
GTTGCCGAAATCCGCCGCCGCTTGGACGATATGGGCTATGCCACACCGCTTATCGGCGAT
TTCCACTTCAACGGCGAACGCCTGTTGGCGGAATTTCCAGAATGCGGCAAAGCATTGTCC
AAATACCGCATCAATCCCGGCAATGTCGGCAAAGGCGTAAAAGGCGATGAAAAATTTGCC
TTTATGATTCGGACTGCTGCTGAAAACGATAAAGCCGTCCGCATCGGCGTAAACTGGGGT
TCTTTGGATCAAAGCCTCGCCAAACGTATGATGGATGCCAACCTCGCTTCTTCCGCGCCG
AAACCGCCCGAAGAAGTGACGAAGGAAGCACTGATTGTCTCCGCTTTGGAATCTGCCGAA
AAAGCCGTTCTATTGGGACTGCCCGAAGACAAAATCATCCTGTCGTGCAAAGTCAGCGCG
GTTCAGGATTTGATTCAGGTTTACCGCGAACTGGGCAGCCGTTGCGCCTATCCGCTGCAT
TTGGGTTTGACCGAAGCCGGTATGGGCAGCAAAGGCATTGTCGCATCAACGGCGGCATTA
TCCGTCTTGCTTCAAGAAGGAATCGGCGACACCATCCGCATTTCACTGACTCCGGAACCT
GGCAGCCCGCGTACTCAGGAGGTCGTCGTCGGGCAAGAGATTTTACAGACTATGGGATTG
CGTTCGTTTACGCCGATGGTTACCGCCTGCCCCGGCTGCGGGCGTACCACCAGTACCGTA
TTTCAAGAGCTGGCACAAGATGTTCAAAATTACCTGCGCCAAAAAATGTCTATATGGCGT
ACCCTTTATCCTGGGGTTGAATCCCTGAACGTTGCCGTAATGGGCTGCGTTGTCAATGGG
CCCGGAGAAAGCAAATTGGCCGACATCGGCATCAGCCTGCCCGGTACGGGAGAAACACCC
GTTGCCCCTGTTTATGTAGATGGTGAACGTAAAGTAACGCTGAAAGGCGACAACATTGCA
ACGGAATTTCTGGCTATTGTTGAAGAGTATGTCAAAACCAATTATGGGGAGAACGGACTC
AAACGCCATCAAGGGAAGGTTATCCCGATACACTCCCTATAAAATCCAACCGCCAGCCTA
CCTTGGCTATTTTTAAATAAAAACCGTTTATTTTCATTGATATAAAACCCATCCCATTGG
AAAAGGCATTTTTTTAAACCGATAAGGAATGGAGGCGCAATATGAAAATACAATCGGCAA
ATACGGAGATGAATTGCAGGTATAAATAAGGAAGGCGGGATACCGCTTCCTGCCCTTGTC
TTTTCTCAATAACCGTACCGGCAGATTCAGGAGGATGCGAGTGTCGCGCCCTTGCAAAAA
CTGCTGCCCCATTTGGCTTTCAGACGGCATCCGTCCATCACAGGCGGGAACGGGGATCCT
TTAAAAAACTCCAAATCCTTCTTCCGTCCCGTGGATGACGGTATCGATACCATATCAAAC
GCAGCTTGAAACAAATGCCGTCTGAACGTTTCAGACGGCATCGGTTTCTCTCAGGTTTCT
TTTATTAAAGCCGCAAAGAAGCGCGGTTTTCCAAAATCTGGTCAATCAAACCATATTCTT
TGGCTTCTTCGGCAGACATGAAATTATCACGGTCGGTGTCGCGTTCCAAATCTGCCAAAT
CGCGGTCGCAATGTTTCGCCATCAGGCGGTTGAGTTTTTCTTTGATTTTTAAAAGTTCGC
GTGCGTGAATTTCAATGTCGGATGCCTGACCGCCCAGACCGCCGCTGATTAAAGGCTGGT
GAATCATAATCCGGCTGTTGGGTAGGGCAAAACGTTTGCCTTTCTCGCCTGCCGACAATA
AGAACGCGCCCATACTTGCCGCCTGCCCCAAGCACAAAGTCGATACATCGGGCTTGATGA
AATTCATGGTGTCGTAAATCGACATACCGGCCGTTACCGAACCGCCCGGCGAGTTAATAT
AGAAGAAAATATCCTTATCCGGATTCTCACTTTCCAAAAACAACAGTTGGGCAACCACCA
GATTGGCGGACTCGTCGGTTACCGGTCCGACCAAGAATACGATGCGCTCTTTCAAAAGCC
GGGAATAGATATCGAATGCACGCTCACCGCGACCGCTCTGCTCGATAACGGTAGGGACAA
GATAGTTATCAAAAGACATTTCGTCTCCTTTCATGATGGAAAAGCACCAAAGCGAGCTTT
~~AAAAGCGGCTTCGGTGCTTTCAAAAACTGCCTTCAGACGGCATTTTCAGGATAATCAGGC~~
TTGCGCGCCCATCACTTCGTCAAAAGACAAAGCTTTTTCATTTACTTTGGCTTTGCCCAA
AACGAAATCAACGACGTTGCTTTCTACCGCCAAAGAAGTCGGGGCTTGCAGGCGGGAAGG
ATCTGCGTAGTACCAGTCAATCACTTCTTGAGGATCTTCGTAGCTTTCTGCAAAGTTGGC
AACAACGGCTTTGATTTGCTCTTCAGTCGGTTCCAGTTTGTTTTCGTCAACCAGTTTGGC
TAAAATCAGACCTAAAGATACGCGGCGTTCGGCTTGTTCTTTGAACATATCCAAAGGCAG
ATCCAAGTTGGCAGCATCAGCCATACCTTGGTTAACAAAATTTTGTTTCATTTCGTTTGC
CAAGCGTGCGGCTTCTTCATTGACCAAAGCAACAGGTGCTTTCAGCTCTACGGCTTTGAG
CAGCGCGTTCATTACGGATTCTTTGGTTTGTTCGTTTACGCGGCGTTCCACTTCGCGGCT
TACGTTTTTCTGCACTTCTTCGCGCATTTTGGCAACGTCGCCATCCGCAATACCCAAGGC
TTTTGCAAAATCTGCATCGACTTCAGGCAGAGTCGCTTCGGAAACGTTGTTCAGCGTAAT
GGTAAACACGGCAGTTTTACCGGCAACGTCTTTACCGTGGTAGTCTTCAGGGAAATTGAC
GGTAACGTCTTTACTTTCGCCAGCCTTCATGCCGACTACGCCGGCTTCAAATTCAGGCAG
CATTTGACTTGCGCCCAATACGAAGGCGTAGTTTTTGGATGCGCCGCCGGCAAAAGGTTC
GCCGTCGATTTTGCCTTCAAAGTCAATGATGACGCGGTCGCCGTTTCGGGCTTCGCGTTC
GACATGGTTGAAGCGGGTGCGTTGTTTGCGCAGGATTTCTACGGTTTGGTCCACTTCGGC
ATCACCGACGGAAGCGGTTACTTTTTCAACTTCTTGTGCAGACAAATCGCCGATAACGAC
TTCGGGGAACACTTCAAAAATGGCGGCAACTTTGAAAGACTCTTTATCGTCTTGTTCTTC
AACGCCTTCAAAACGGGGGAAGCCTGCCACTTTCAACTCTTGGGCAACGGCAACATCGTA
GAAGCGGCGTTGCACCAGCTCGTTGATCACGTCGTTTTGTGCGCTCGCACCGTACATTTG
GGCAATCATTTTTAAAGGTGCTTTACCCGGACGGAAACCGTCGATTTTTGCACGGCGTTG
GGTTTGTTTCAGTTTTTTATCGGTTTCTGCGTTGATTTCGGACCAAGGCAGGGACAACAC
TACTTTGCGTTCCAGATTTTCTAAAGTTTCAACAGTTACGCTCATCATAAGCCCTTAAAT
TTGTTGTGTTGATAAAATGATAAACTTTCTTCCCTACATGGGGAAGCAAACAGCGCAACG
GTACGATATTTGAACCGCATTGCCGCAAAGGGGAAATTTTAGCTGGCAAGTATATCACAA
TGTTTCGCCTGAAACATAATATGCCGTCTGAAACGCCAATTCCGCCGTTCAGACGGCATT
TTGCAATACGGGCTACAAATGGTCCTTGTGCGCCAAAATTTTACGGCTGCCGTTGAGGTC

## Appendix A

GGTGGAAGAAACGACACCCGCATTTTCCAGTGCCTCCATCAGGTTTGCCGCGCGGTTATA
GCCGATGCGCAGCTGCCGCTGCAAAGACGAAATGGAGGTTTTTTTGCTTTCCAAAACATA
GGCGACTGCCTGATCGAACAATTCGTCGCTGTCTGCATTCGGATTAACGATATTGGCAGT
TTCCAGCGCGGCCTCGCCGCTGAGCAGACCTTCAATATAGTCGGCTGGGGCTTGCGATTT
GACATAGTTGACGACTTGATGTACTTCGTCGTCTGAAACAAACGCGCCTTGCAGGCGAGT
CGGTTCGGCACTGCCGGGCTGGAGGAACAGCGAATCGCCATATTTGAGCAGTTCGTCCGC
GCCCATTTGGTCGAGGATGGTACGGCTGTCGATTTTGCTTTGCACGGTAAACGCCATACG
CGTCGGGATGTTGGCTTTAATCAGGCCGGTAACGACATCGACACTGGGACGTTGGGTGGC
GACAATCATATGGATACCGGCGGCGCGCTTTTTGGGCGAGACGGGCGATTTGCTGCTC
GACGGCTTTGCGTTCGGTCATCATCAGGTCGGCAAGTTCGTCGATAACGACCACAATCAA
CGGCAGTTTTTCCAGCGGCTCGGGCTCGTCGGGGTTCAGGCTGAACGGATTGAGCAGCGG
CTTGCCTGCCGCTTTTGCGGCTTCGACTTTTTGGTTGAAGCCCTCCAAATTACGCACACC
GGCATGGGAAAGCAGGCGGTAGCGTTTTTCCATTTCGGCGACGCACCAGTTCAACGCCTG
CCCTGCTTCGCGCATATCGGTCACGACGGGACAGAGCAGGTGCGGAATACCGTCGTAAAT
GCTCAACTCGAGCATTTTCGGGTCTATCATAATGAAGCGGACTTCGTCGGGCGTAGCTTT
GAAAAGCATAGACATAATCATGCCGTTCACGCCGACGGACTTGCCCGAACCAGTCATACC
GGCGACCAAAAGGTGCGGCATTTTCGCCAAGTCGCCGACAACGGGGGTACCGGCAATGTC
TTTGCCCAGCGCGACGGTCAGCTTGGATTTGGCTTCGGCAAACACGGGCGAGGACAAGAT
TTCACTCAACATCACGTCTTGGCGTTTGTCGTTGGGCAACTCGATGCCCATCGTGTTTTT
ACCTGCGATGGTTTCGACGATACGCACGGACTGCAGCGACATAGAGCGTGCCAAATCTTT
CGACAAGGCAACAATTTGGCTGCCTTTAACACCTTGCGCGGGTTCGATTTCGTAGCGCGT
GATGACGGGGCCGGATGTGGCGGATACGACTTGTACGCCGATGCCGAATTCTGCCAGTTT
GGATTCGATCAGTTCGGCAGTGCGCTCCAATTCGGCGGGATTGATGCTGACGGGTTCGCT
GTCAGGAATCCGCAATAGGTTCAATGTAGGCTTGTGGTATTCGCCCGCCTGCCGAGGTTC
GTCATCTTCAAACAGAGAAACCTGAATTTTGGGCGGCGGCGCGACGGAAACCGCGACGGA
TTTGCGGTTGCTGCTGCTGCCTTCGGGCAAGGCAACGGGTTTGGCCGTAATATTCTTGGC
TTCTTTTACCATGCGCCGTGTATTTTGGGTATCGACACCGTCTGTTTTGGTATTCGGCCG
GCGTTTTCCTAAAGCCATGACCTTGCCGGATAAGGCACTCAGGCGGTTTTGAACCGCCCT
GCCCGCACCGTTCAAAAATTCCAGCCATGAAATCTGCACCAGCAGGGACAACGACAACAG
CAGAACAACCAAGATAATCAGCAGGCTGCCCGATTTCCCCAGCAGCCACGCAAACACTGC
GCCGACGCGTATGCCGACCATACCGCCTGCTCCGACAGGCAGGGAGTCGGCATATTTTCC
GCCCAGCACAAAATACTCCAAGACGGGGCTGAAGACCGTCAGGACAAACAGCGCGGCGGC
AGCGATTTTGTGGTTGTATGCCTCGTTTTCCGTCTGTTTTGCGTGCAGGCGGAAATTTTT
ATACAGCACGACGCAGGCAGCCGCTATCCACCACCAGAACGACCAGCCGAAAAGATAATA
GCCGACATCGGCAACATACGCGCCGAACAGTCCGCCCCAATTGGCGACATCTTCCACAAC
CGGCGAACTGTGCGACCAAGACGGATCGCCCATATCGAAACTGATCAGGGAAATCGCCAA
ATACAGGGTTGCCGCCAAACCCATCAGCCACAGTGCGTCGCCGATAAGGTTGACGACATG
TTCGGGACGCGCCTTTTTGGTTTCGGTTTTCTGCAACTCTTTGACCGCCTTGAGCCGCTC
GGAAACTTTATTGCCCCGTGCGCCGTTGTCGGCTTTCTTGTTGCGTGCGGACGTTGGGGA
CGGGCTTCCCGCCCTGCCTTTTGCTGTTTTTTTATGGGATTTTTCTGTCATGCCGTATTA
CCGGAAAATGCCGTCTGAAATAAGGAAGCCGGACGGCTTGCGGATTGAATATGGAAAGTG
TCGGATTATACTCCATTTCTCCTGCTTTCATCCTGCAACAGACAGACATTGCCTTTCAGA
CGGCATATCTGTTGCCCGACGTATGTATTTTTACGCAGACCCTCGGCAAACCAGTATAAT
CCGTGCCGTTTGAACCGATTGAAAGAAGATGGTATGAACCAACTGAAACTTGCCGTTTCC
GGTGCACAGATTTTATTTGTGGCATTCGGCGCAATGGTGCTGGTTCCCCTGCTGACCGGT
CTGAATCCGGCTCTTGCGCTTTTGGGCGCAGGCTTGGGAACGCTGCTGTTCCAAATCACA
ACCAAACGCAAAGTGCCGATTTTTCTTGGTTCTTCGTTTGCCTTTATCGCACCGATTATC
TACTCCGTCGGCGAATGGGGGCTGCCTTCCACCATGTTCGGACTGTTTGCCGCCGGCTTT
ATGTATTTTGTGTTTGCCGCGCTGATCCGTTGGCGCGGACTGGCAGCGGTACACAAACTG
CTGCCTCCGGTCGTCATCGGCCCCGTCATCATGGTCATCGGTCTGTCTGTTGCCGTGGCG
GCAAGCAGCATGGCAATGGGTCAGGCGGACGGCAAACAGGTCATCGACTATACCGATTCG
CTGATTCTTTCCGGCTTTACCTTTGCCGTTACCGCCATCGTATCGGTTTTCGGCAGCAGG
ATGATGAAGCTGATTCCCATCTTGATCGGTGTCGCTTCGGGTTATGTTTTGGCACTGCTG
ATGGGACTGGTGGACACGGCAAGCATTGCACACGCGCCCTGGTTCGCCGTTCCCCATTTT
GAAACGCCTCAGATCAACTGGCAGGCTGCACTGTTTATGCTGCCCGTTGCCGTCGCCCCC
GCCATCGAACACATCGGCGGCATCATGGCAATCGGCAATGTGACGGGGAAAGACTATACG
AAAGACCCGGGCTTGGACAAAACCCTTGCAGGCGACGGTTTGGGCGTATGCGTTGCGGGT
CTGATCGGCGGCCCGCCGGTTACGACCTACGGCGAAGTAACGGGTGCGGTGATGATTACC
AAAAACAGCAACCCCGTCATCATGACTTGGGCGGCGGTTTTTGCCGTCTGCATGGCGTTT
TTCGGCAAATTCAATGCGTTTTTGGCTTCCATTCCGATGCCAGTAATGGGCGGCATTATG
CTGCTGCTGTTCGGCACGATTGCTTCTTTGGGCGTGAAAACGCTGATTGATGCCAAAGTC
GATTTGATGCTGCCGAAAAACCTGGTCATCGTCAGCTCGGTACTGACCACGGGCATCGGC
GGCATGACGCTCAAATTGGGCAGCTTCAGCTTTGCCGGCGTGGGCTTGTGCGCCGTACTT
GCCATTATGTTGAACAGCCTGCTGCCCGATCCGAAAGAATCCTGACCGTCGATATAGAAA
TGCCGTCTGAACATCTTTCAGACGGCATTTTCCGTTTTATTTGAGATTTTGAATCAAAGA
GCGCACAGTTCCGCCGTAATAAGAAGAAGATGTGCAATACACTGTTTCCAAGCCGCATTG
TCCCTGTACGCCGTATTTTTTGATGCACTGGTTGAGTGCGACCTGATGAACGCTCGTAAA
ACGCGGAGAAGTAATCACGACGGCGTTGTCGACACGCAGCGCGCCCAAGGCTTTCGGGTA
TGCCAGCGCGACACAGGTATTGTTCAGCGACACGACCGACCGGCATCCGGTCGGCTCGTC
TTCAGCAATGCCCGCAAGCGTGTCCTGACCTTTGCAGAAGGCTTCCAACTCGGAAAACGC
TTCGCTTTTCGTCGAATCTTCTTTTGTGGTTTTAACCTGCAAAACATCGTCCGCATTCTG
CGGATTCTGCCAAACGGCGAGATAGCCGTAAGTATCGGCAGCCCGTGCCGCCGCAGTCAT
CAGGCATAGTGCCGATACGGCCAGTATCTTTTTCATCATGATAAATTCCCGACGGTTCGT
CCAAATTCTGTTGCATTATAAACAAAAAACAGGATAAGTCCCGCCTTATCGGCTTATCCC
TCCCCGCAGATTGCACCGCCGGGTATGGCAAACCGATTTCAGCAGCGCAAATCCGCATAC

## Appendix A

CGCCGCCTTAGCGGCAAGCCGTTGTTTTCAGACGGCATTGCGGCCAACCTTTGCGGCGGG
CGAAAAACCTTGTCCTATAATTTATCCCGTTTCAAAATCAGCATACGGTCGGAAATGCAA
AAAATATCTTTCAATTTGTTGAAGCCTGCAAACTCCCCGAAAATAGGGAAACGCCGCCCC
GGTTTGAACGGCGCGCCGCATATTCCGATGCCCTCCCCCCGATACCTTCCGGCAAGCCCA
GAAATGCCCGGCAACAACATCCATCCGGCAAAAATCCGAAACAACACACCCGGCGGCAGG
CAGAGTCAAACCGCCCCGCAAAGCATCCGCCATCAGAAAAACAAACCGCCTCCGAGGGCT
TCATCCTAAAGGGCGTATTGTTCGATAATGGTTTGGGTTATAATCCCCTATCGATTCTCC
ACGTCCGTGAGACACTTCAGCTATGGAAACCCCGACCAACACCCCGCAACGCTCCCTGCG
TCAAAACAGTATCTACCTGCTGCCCAATTCCTTTACTATCGCCGCGCTGTTTTCCGCGTT
TTACGCAATCACCCAATCCATGCACGGACGTTATGAAACCGCCGCCATCGCGGTATTCAT
CTCTATGTTGCTGGACGGTATGGACGGGCGCGTGGCGCGGCTGACCAACAGCCAAAGCGC
GTTCGGGGAGCAGCTCGACAGCCTTGCCGATATGGTCAGCTTCGGCGTTGCTCCCGCTCT
GATTGCCTACAAATGGCAGCTTTGGCAGTTCGGCAAAATCGGTTATTCCGTCGCCTTCAT
CTACTGCGCCTGCGCCGCCCTGCGCCTCGCCCTGTTCAACACACTCATCGGCAAGGTGGA
CAAACGCTGGTTTATCGGCGTGCCCAGTCCGACTGCCGCCGCGCTGATTGTCGGGCTGAT
TTGGGTCAACCACAGCGTCGAAAAATTCCCCGCCGTCCACTGGTGGGCATTGGGCATCAC
ACTGTTTGCCGGCCTGTCGATGATTGTCCAAATCCCTTTTTGGAGTTTTAAAGAAATCAA
CATCCGCAGACAAGTCCCCTTTGTCGGAATGCTGCTTGCCGTCTTACTGCTGCTTCTGGT
CACTTGGGAACCGTCGCTCGTCCTCTTCCTGTTCTTTCTCGGATACAGCCTGTCCGGCTA
CATTATGGCGGCACGCCGATTTTGGAAAAAGTACAGAAAGGCGGATTAAATGTGGCATTG
GGACATTATCTTAATCCTGCTTGCCGTAGGCAGTGCGGCAGGTTTTATTGCCGGCCTGTT
CGGCGTAGGCGGCGGCACGCTGATTGTCCCTGTCGTTTTATGGGTGCTTGATTTGCAGGG
TTTGGCACAACATCCTTACGCGCAACACCTCGCCGTCGGCACATCCTTCGCCGTCATGGT
CTTCACCGCCTTTTCCAGTATGCTGGGGCAGCACAAAAAACAGGCGGTCGACTGGAAAAC
CGTATTTACGATGATGCCGGGTATGATATTCGGCGTATTCACGGGCGCACTCTCCGCAAA
ATATATCCCCGCGTTCGGGCTTCAAATTTTCTTCATCCTGTTTTTAACCGCCGTCGCATT
CAAAACACTGCATACCGACCCTCAGACGGCATCCCGCCCGCTGCCCGGACTGCCCGGACT
GACTGCGGTTTCCACACTGTTCGGCACAATGTCGAGCTGGGTCGGCATAGGCGGCGGTTC
ACTTTCCGTCCCCTTCTTAATCCACTGCGGCTTCCCCGCCCATAAAGCCATCGGCACATC
ATCCGGCCTTGCCTGGCCGATTGCACTCTCCGGCGCAATATCGTATCTGCTCAACGGCCT
GAATATTGCAGGATTGCCCGAAGGGTCACTGGGCTTCCTTTACCTGCCCGCCGTCGCCGT
CCTCAGCGCGGCAACCATTGCCTTTGCCCCGCTCGGTGTCAAAACCGCCCACAAACTTTC
TTCTGCCAAACTCAAAAAATCTTCGGCATTATGTTGCTTTTGATTGCCGGAAAAATGCTG
TACAACCTGCTTTAAAACACACGAAAAAACCTTTTTACCGTTTGCACAAGCAATTAATCA
GGACAAAGCTGCCCAGTCTCCTGTTCCGACAAAAGGACAGACAACCTGACCGAGACCTTT
GCAGAATATACGAAAAACAAAACAAATACCGTCTGAAACCACATTCCGACAATCGGCAGG
GTTTCAGACGGCATCTGATAATTTCAATTACTCGGTTGCGGCAACGACGGCAACGGTAAT
TTTAGCAACGGCATCAGTGTGCAAAGCCACTTCCACTTCGTACTCGCCAACGGCTTTCAG
AGGACCGTTCGGCAGACGTACATTTGCTTTCACGGCTTCGATGCCGGCAGCAACGATTGC
AGCAGCAATGTCGGCATTGGTAACGGAACCGAACAGGCGACCGTCCACACCAGCTTTTTG
AGCAACGGTAACGGTTTGACCGTCCAATTTTTCCTGACGGACTCGGGCATCTGCCAAAAT
TTCAGCCTGTTTGGCTTCCAGTTCTGCGCGGCGTGCTTCAAACTCTTTCATATTCGCTTC
GGTCGCACGTTTTGCCTTACCTGCGGGAATTAGAAAGTTGCGGGCGTAGCCGTTTTTAAC
GGTTACGATGTCGCCCAAGTTGCCCAGACCGCCGATTTTTTCTAACAGAATAATTTGCAT
GATTCAATCTCCAAAATTATTTGTGTTGGTCGGTGTAAGGCAGCAGAGCCAGGAAGCGTG
CGCGTTTTACGGCAACAGCCAATTGGCGTTGGTAGAATGCCTTCGTTCCTGTGATGCGTG
CAGGAATGATTTTACCGTTTTCGGAGATGAAGTCTTTCAGCAAATCAACTTGTTTGTAAT
CGACTTCTTGGATTTTTTCAGCCGTGAAACGGCAGAATTTTCTACGTTTGAATGATTGAC
GAGGGATTGTGGTTTAACCTTTATATTCTTGAATATTTTGTATCCTGAGCATCGGCATAA
GGGAACGTCTGCTTTTTTGAGCTAAAAAACCTTCGACGTGAACATATACACCTTGCCGAT
ACTGCCACTCTTCCGCCTGCCTGCCTAAAATCCGTGCCGGAATTTCCAATTGGACAAGGC
ATTGCTGCCCGTTTTCCTCCTGCCACGATTCGTGCTTTAAAATAATATCTAAAACAGGGA
TTCCGGCAGGCGTATATCGAATAGGGAAAACCTTTTCAATTAACGCGGCAAGCGAAACAA
GATTATTGAATCCCAATTATTGGGCGACCGCTTCTTCAGACGCACCGCTCAACAGGTTCT
TAGCCTTTTCACCACCCAACATAGGGGATGCTTCGGTAACGGCGTGTTTGGTTTTGATGG
TCAGATGACGCAATATTGCATCATTGAAGCGGAATGCGGTTTCCAGCTCTTCAACCACTT
CGGGAGTGGTTTCGATGTTCATCAAAACGTAATGGGCTTTATGGATTTTGTTAATCGGGT
AAGCCAGCTGGCGGCGACCCCAGTCTTCCAGACGGTGAATCTTACCGTTTGCTTCGGCAA
TCATGGTTTTGTAACGTTCAACCATAGCGGGTACTTGCTCGCTTTGATCGGGATGAACGA
TAAACACGATCTCGTAATGACGCATGTTATCTCCTTATGGATGGTAAAAACAGCCTTCTG
CCATGCGAAAGCAGAAGGCAAGGTTTAAAGAAGCGGCATTATATTGGGGTTTGCCGACGG
AATCAAGGATTTGGTGCGAAAAATTTGCATTCCGCCGAAAATTTCGGTTTCAGACGGCAT
TCAAATGTTTTGGCTGCCCAGCCAGCGTTCCGCGTCCAAAGCCGCCTGACAGCCGGAAGC
CGCGCTGGTAATCGCCTGACGGTAGGTATGGTCTTTTACGTCGCCCGCCGCCCATACGCC
TTCGATATTGGTTGCGCCGACATTGTCCGCCGTGCCGCCTTTGGTTTTCAGGTAACCGGC
TTCGTCCATTTCCAACTGACCTTTGAAAATATCGGTATTCGGCTTGTGCCCGATGGCGAT
AAAAATGCCGCTGACGGCAATTTGTTGCTCAGAACCGTCGTTGTTTTTAATAATGCGCC
GTTTACGCCCCGATCGTCGCCCAGTACTTCTTGCAGGTTGCTTTCCAGCTTGAGGATGAT
TTTGCCCTCTTCCACGCGTTTCATCAGTTTGTCGATCATGATTTTTTCGGCACGGAACTC
GCTGCGGCGGTGGATCAGGGTAACGGTTTTGGCGATATTGGCAAGGTAGAGTGCCTCTTC
AACTGCCGTATTGCCGCCGCCAACTACGGCAACATCTTGGTTTTTATAGAAGAAACCGTC
GCAGGTGGCACAAGCGGAAACGCCTTTTCCTGCAAACGCTTCCTCACTCGGCAAACCGAG
GTATTTGGCGGACGCGCCTGTTGCGACAATCAGGGCATCGCAAGTGTACTCGCCCATATC
GCCTTTGAGTGTAAACGGGCGTTTTTGCAGATCGACGGCGTTGATTTGGTCAAAAATGAT
TTCCGTTCCGAAACGTTCGGCGTGGGCGAGAAACCGCGCCATCAATTCCGGCCCTTGCAC

## Appendix A

GCCGTCGGCATCGGCAGGCCAGTTGTCCACTTCGGTGGTGGTCATCAGTTGCCCGCCTTG
CGCGATACCTGTAATAATGACGGGGTTTAAATTGGCGCGCGCGGCATAGACGGCGGCGGT
GTATCCGGCGGGGCCGGAACCCAAAATAATCAGTTTGCGGTGTTGGGACATTGTTTTTCC
TTTGCTGTGTCAAGTTTTCGGATTCTACTCGAATTATCGGCGCGTTTGAGAAATTTCGAC
CATACCGGCGCTCAGACGGCATCCCGCAGCCTTAACTGCCGTCTGAATATCAAAGCAGGA
ATCACGCTTATGCAACAAAAAATCCGTTTCCAAATCGAAGGCATGACCTGCCAGGCCTGC
GCTTCGCGCATTGAAAAAGTGTTGAACAAAAAAGATTTTGTCGAATCGGCGGGGGTAAAC
TTCGCCAGCGAAGAGGCGCAGGTAGTGTTTGACGACAGCAAAACCTCAGTAGCCGACATT
GCCAAAATCATTGAGAAAACCGGTTACGGCGCGAAGGAAAAAACGGAAGATACATTGCCG
CAACCCGAAGCAGAACACCATATCGGCTGGCGGCTGTGGCTGCTGTTCACCATCAACGTC
CCGTTCCTTATCGGCATGGCGGGGATGATGATCGGCAGACACGATTGGATGATTCCGCCG
TTGTGGCAGTTCGCATTGGCAAGCGTGGTGCAGCTTTGGCTGGCAATCCCGTTTTACAAA
AGCGCGTGGGCGAGCATTAAGGGCGGACTGGCGAATATGGACGTGCTGGTTACCATCGGC
ACGGTCTCGATTTACCTGTATTCCGTCTATATGCTGTTTTTCAGCCCGCACGCGGCGTAC
GGTATGGCGCATGTGTATTTTGAAGTGGGCGTGATGGTGATCGGTTTTGTGTCACTGGGT
AAATTTTTGGAACACCGTACCAAAAAATCCAGCCTCAACAGCTTGGGCTTGCTGCTCAAA
CTTACACCAACCCAAGTCAACGTGCAACGCAACGGCGAATGGAAACAGCTTCCCATCGAC
CAAGTGCAAATCGGCGACCTTATCCGCGCCAACCACGGCGAACGCATTGCCGCAGACGGC
ATCATTGAAAGCGGCAGCGGTTGGGCGGACGAGAGCCATCTTACCGGCGAATCCAATCCT
GAAGAAAAAAAGGCGGGCGGCAAAGTGTTGGCGGGCGCGTTAATGACCGAAGGCAGTGTG
GTGTACCGCGCCACGCAGCTCGGCAGCCAAACCCAGCTCGGCGACATGATGAACGCGCTC
TCTGAAGCACAAGGCAGTAAAGCACCGATTGCGCGCGTAGCCGATAAAGCGGCTGCGGTA
TTCGTGCCTGCCGTCGTGGGCATTGCGTTGTTGACTTTTATTGTTACTTGGCTGATTAAG
GGCGATTGGACGGTTGCGCTGATGCACGCCGTCGCCGTTTTGGTGATTGCCTGCCCGTGC
GCGCTGGGTCTGGCAACCCCTGCCGCGATTATGGTCGGTATGGGCAAAGCGGTTAAACAC
GGTATTTGGTTTAAAGACGCGGCAGCAATGGAGGAAGCCGCCCACGTCGATGCCGTCGTG
TTGGACAAAACCGGTACGCTGACCGAAGGCAGCCCGCAGGTTGCCGCCGTTTATTGCGTT
CCCGACAGCGGCTTTGACGAAGACGCTTTGTACCGCATCGCCGCCGCCGTCGAACAAAAC
GCCGCCCATCCGCTCGCCCGTGCCATCGTCTCCGCCGCCCAAGCGCGCGGTTTGGACATT
CCCGCCGCACAAAACGCACAAACCGTTGTCGGCGCAGGCATTACCGCCGAAGTGGAAGGC
GTGGGTTTGGTGAAAGCAGGCAAAGCCGAATTTGCCGAACTGGCCTTGCCGAAGTTTTTA
GACGGCGTTTGGGATATTGCAAGCATTGTTGCGGTCTCAGTCGATAACAAACCCATCGGC
GCATTCGCACTTGCCGACGCGTTGAAAGCCGATACCGCCGAAGCCATAGGCCGTCTGAAA
AAACACAATATCGATGTCTATATTATGAGCGGCGACAACCAAGGCACGGTCGAATACGTC
GCCAAACAACTGGGCATCGCACACGCCTTCGGCAACATGAGTCCGCGCGATAAAGCTGCC
GAAGTGCAAAAACTCAAAGCCGCCGGCAAAACCGTGGCGATGGTCGGCGACGGCATCAAC
GACGCGCCCGCGCTTGCCGCCGCTAACGTCAGCTTCGCCATGAAAGGCGGAGCGGACGTT
GCCGAACATACCGCATCCGCCACGCTGATGCAGCATTCGGTCAACCAACTCGCCGATGCT
CTGCTGGTGTCGCAAGCCACTTTGAAAAACATCAAGCAAAACCTGTTTTTCGCCTTCTTC
TACAATATTTTGGGCATTCCTCTCGCCGCGCTTGGCTTTTTAAATCCCGTCATCGCTGGC
GCGGCAATGGCGGCAAGCTCGGTTTCCGTGTTGAGCAATGCCTTGCGCCTGAAACGGGTA
AAAATCGATTAGCAGCATGTAACCGCCCTGCAGCCTTGTCCGAACGGATAAGGCTGTCTC
CAGCGATATGGTAATATGCCGTCTGAAACCGTTTTTCAAGTAATTGATATGAATAAAGAA
ACCCGTTTTCCGGAACACTTCGACATCCCACTTTTCCTCAAAAACCTGCCCAACCTGCCA
GGCGTATACCGTTTTTTCAACGAAAGCGGCAACGTCTTATACGTCGGCAAAGCCGTCAAC
CTCAAGCGGCGCGTGTCCGGCTATTTCCAGAAAAACGACCATTCCCCGCGCATCGCATTG
ATGGTGAAACAGGTTCACCACATCGAAACCACCATCACCCGCTCCGAATCCGAAGCCCTG
ATTCTCGAAAACAACTTCATCAAAGCCCTGTCGCCCAAATACAATATTCTTTTCCGCGAT
GACAAAAGCTATCCTTATTTGATGCTCAGCGGCCATCAATATCCGCAAATGGCGTATTAC
CGCGGCACGCTGAAAAAGCCTAATCAATATTTCGGCCCATATCCCAACAGCAACGCCGTG
CGCGACAGCATTCAAGTGTTGCAAAAAGTCTTTATGCTGCGTACCTGCGAAGACAGTGTA
TTCGAGCATCGCGACCGTCCTTGTCTGCTTTACCAAATCAAACGCTGCACCGCGCCTTGT
GTAGGCCACATCAGTGAAGAAGATTATCGTGACAGCGTGCGTGAAGCCGCGACTTTCCTT
AATGGCAAAACTGACGAATTGACGCGTACCCTGCAACACAAAATGCAAACCGCCGCCGCT
AATCTACAATTCGAAGAAGCCGCACGTTACCGCGATCAAATCCAAGCGCTCGGCATCATG
CAAAGTAATCAGTTTATCGACAGTAAAAATCCGAACAATCCCAACGATATCGATTTGCTT
GCACTGGCGGTTTCAGACGGCCTGGTTTGCGTACACTGGGTCAGCATCCGCGGCGGACGG
CACGTCGGCGACAAAAGCTTTTTCCCCGACACCAAAAACGATCCCGAGCCAAACGGACAA
GATTACGCCGAAGCCTTCGTCGCCCAACACTATCTGGGCAAAAGCAAACCCGACATCATC
ATCAGCAACTTTCCCGTTCCCGATGCGCTAAAAGAGGCTTTGGAAGGCGAACACGGCAAG
CAGATGCAATTTGTCACCAAGACCATAGGCGAACGCAAAGTCCGGTTGAAAATGGCGGAA
CAAAACGCGCAAATGGCGATTGCACAACGCCGCCTGCAACAAAGCAGCCAGCAACACCGC
ATTGATGAACTGGCAAAAATCCTCGGCATGGATTCAGACGGCCTCAACCGCCTTGAATGT
TTCGACATCAGCCACACACAAGGCGAAGCCACTATTGCGTCCTGCGTTGTGTACGATGAG
CAAAACATCCAGCCTTCGCAATACCGCCGCTACAACATCACGACCGCCAAACCCGGCGAC
GACTACGCCGCCATGCGCGAAGTGTTGACGCGCCGTTACGGCAAAATGCAGGAGGCCGAA
GCCAACGGCGAGACCGTCAAATGGCCGGATGCCGTGTTGATTGACGGCGGCAAAGGGCAA
ATCGGCGTAGCCGTATCGGTATGGGAAGAACTCGGGCTGCACATCCCTTTGGTCGGCATT
GCCAAAGGCCCGGAGCGCAAAGCCGGTATGGAGGAGCTCATACTGCCTTTTACCGGCGAA
GTCTTCCGCCTGCCGCCCAACAGCCCGGCCTTGCATCTATTGCAAACCGTACGCGATGAA
TCGCACCGTTTCGCCATTACCGGTCACCGCAAAAAACGCGACAAAGCCCGCGTTACCTCC
TCCTTAAGCGACATCCCCGGCGTAGGCAGCAAACGCCGCCAAGCCCTGCTCACCCGCTTC
GGCGGTCTGCGCGGCGTGATTGCCGCCAGCCGCGAGGACTTGGAAAAAGTGGAAGGCATC
AGCAAGGCATTGGCGGAAACGATTTACAATCATCTGCATTAGCATGCTGTCAAAGACAAA
ATCCGTCTGTAAAAAATATGATACAGCAGGTCGGTATACCGATATATAGTGGATTAAATT

## Appendix A

TAAACCAGTACGGCGTTGCCTCGCCTTGCCGTACTATTTGTACTGTCTGCGGCTTCGTCG
CCTTGTCCTGATTTTTGTTAATCCACTATAAACCTAACTTCATAACGAATAACGATGATT
CGACAAAACGGAAAACGATCTGACATGAACAATCCCGACTTACCCTATCGGCAGGCCTTA
GAATGCCTGTCTCAAAAACAATATAACTTTACCGAAGTCCGCCGACTGCTGACAGAAGCG
TTCTCGGCAGGTCATCCCGCCGCCGCATTCGAGTTGGCAAAACACCTGATGGACGCGGAC
AGCCCCTACCAAGACCGCGAACAAGGTATGGAAATGCTCCGCATCGCCGCTGAACAGGGA
CATCCCTACGCGCGTTACAATCTGGCATATATCCAAGAATTGGAAGGCGCACCCCCGGAA
ACCCTGATACCGCTTTACAGACCGTTGGCAGAAGAAGGACTGCCCGAAGCGCAAGTCCGC
CTGATGTACCTTCTGTACGCGTCCCGACATTTTGAAGAAGCCTTGGAATGGGCAAAAACA
AGCGCAAAAAACAACAACCCCCACGGGCAATACCTGCTTGCCCAATACTGCCGGTACGGC
ACACCGCCGGATTTTGAAACGGCGCACCTGCTCTACCGAAAATCGGCGGCACAAGGCTTG
CCGGAGGCACATTGGCAGCTCGGGCTGCAATATCGTTTCGGGCAAGGGACGAAAGTCGAC
ACGGCACAGGCCGTCAATCATTTGCGCGCCGCCGCACAACAAGGATACATTCCTGCCTAC
ACCCCACTTGCCGAGCTCATCCTACCTACGGCTCCTGATGAAGCCGTTCACTGGTTTCAA
CAGGCCGCACAGGAAAATGACCCCGATGCCCATGCCGCACTTGCCGACATCTACCTGCAA
GGCAAGCATCTGGAAAGAAACCACAAACTTGCCCTGCATCATGCCGAAGCAGCCGCCGCC
GAACGCCATCCCGAAGGTTTGCGGATACTGGGCGACATCTGCCGCTACGGTTTGGGCATA
GCCCCCGATACGGAAAAAGCCCGGCATTATTATCGGCAGGCAGCCGAAGCCGGCAGCCTT
TCCGCCTATCAGAAACTCATATCCGACAGCGCGTTAAACCATCCTGACCAATACGGCGGC
ATTAAAGATTCCGCCATCAGGCGGCAAAGGGCAGAACGGCTTTATCAAAAAGCCCAAGCC
CTGCATTACGGATTACAATGCGCGCCCGAATACGCAGCCGCGCTCAAACTCTACACAGAA
GCCGCAGAACTCGGACACAGCAAAGCCCAAACCAATCTGGGCAGCATGTATTACTTCGGA
CAGGGTATGACCGCCGACTACAATGAAGCACGCAAATGGTTTGAAAAAGCCGCCGCGAAA
AAAGACAGTATGGCGTTCTACAACCTCGCCTGCATCCATTACAGCGGACACGGCGTCGAG
CCGGACAAAGAAAAAGCCTGCCGCTACCTGCAAGAAGCCATAAACAACGGATACGGGCAA
AAAAGCGTCCTGCAAGAACTGCTGCAACAATGGCAAAATGCCGTCTGAACAGCGTTACAC
CTACCCTGCCGAAACGAAACAGGTATAATCGCCCCTTTCCCTTCCCGCCGTCCGAACAGG
CATTTCACATTCAGACGGCATCCTGATTGCACAAGCGTACGAAAGCATTATGACAGACAC
CGCCGAGAACCAAACACAAAACAACTGGCAAGCCGGACACCCCCGCAGCATCCGCAGCTT
CGTCCTCCGCCAAAGCCATATGACCGCCGCGCAGCAACGCGCCATCGATACCTTATGGGA
CAGCTTCGGCATCGACTACCAAGCAACACCGGCCGATCTTGATGCCCGTTTCGGAAGCAG
CCGACCCAAAATCCTCGAAATAGGCTTCGGTATGGGGACGGCAACCGCAGAAATCGCCCG
CCGCCTGCCCGAAACCGACTTCCTCGCCATCGACGTACACGGTCCCGGCGTAGGCAACCT
GCTCAAACTCATAGACGAAAACCATTTAGAAAACATCCGCGTGATGCGGCACGATGCCGT
AGAAGTTGTCGAAAATATGCTGCAAGACGGCTCGCTCGACGGCATCCACATATTCTTCCC
CGACCCGTGGCACAAAAAACGCCACCACAAACGCCGTCTGATACAAGCCCCCTTCATCGC
CAAACTACTGCCCAAACTCAAAACCGGCGGCTATATCCACCTGGCGACAGACTGGGAAGA
ATATGCACAGCAGATGCTTGAAGTCCTCAGTAGCTTCGACAGCCTGCAAAATACGGCGGC
AGACTACGCCCCCACCCCGGACTACCGCCCCGAAACCAAATTCGAAGCGCGCGGCAAACG
CCTCGGACACGGCGTTTGGGACTTGGTATTCAAACGGATCGGATAACAAACCACTGTTTG
AAAATGCCGTCTGAAACATGTTTGCTTACAGACGGCATTTTTTCAAGATAAAGCAGCAAG
TGATGTTTCGATATAAAGTTTAAAACAATAGTTTGAACGGCAAAACGCGTGTGTACCGCA
CGCATCCTTATAGGTTTTATGCACATCGGTTTTAAAGTTTGTGCCGCCCGCAGGTAGTAT
GTGATAGCTACGCACGCGGTTGGTGTGATGTAGGCTACGGCTTGCTGGTTACAACCGTAA
AAAAGTAAGTGCCGCCATTGCGGTAAAAACGAAGGGATTTCATAGTGTTATGCTCGTAAT
GATTTTGTAGATTGGATTCTCGAATCCGACCTTTTGGGCATTGCTGCAATGGATTGCAAC
GACGGGAATGTTGAAGGTTTTGTCGGATACAAGTATCCGACCTACGCTTGTTGCTATATA
TCTTTTGATTTAATGACTAAATATGACAAAGTTAAAGTGCAGATTACAGCAAGCATGATA
TGCTTCTTTAGGCTTTTATCATTCCATGATATAGATATTTCTTCCTTTTGATTTTCTTTA
TAAAATTTTAAACCTATATCACCATTTTTCCATTCCTGGTGGTTTACTATGATTTTATTT
TTAAAAGAATCTCTTAAACTTTCATGTAAAGAGTTAAATTTTCTTGATTTACTTCCCTTA
GTACATGGTGAGCAATTGTATTTCTAATTTTATTTAATCTCTCCCCTATATCATATACTT
CGCTAAATAAGCCAAGATTACGCGCAATTTTTAGTTTTGTGCGAAATCCAATTTGTGTAT
CATTGAAAAAATCTTCTTTATTACATTTTGCATATATCCATGCCTCTAAAATTCTTTCAA
AAAATAAATGTGTTCGTAAGATTGAACCTATTTCATCCTGTGTTTCAATAGCTTCTTTCA
CGATATCAAAAATCTTATAATCATCAAAATTTGAATTTTTAATAAATAATTCTAAATTAA
AATCTAATTGAGACATAATAAGTGCCCATTTCAAAAATAAATCTATATTCTAGTTAATAT
AATAGTTATTCTAATATCTAAATTAAATAATAAACTACTATTTTTATATCCACGACAAAG
TCTAAGTCTCACTCCGCCCCAAACAACAAATTCTCTTTAATATCCCTAATCCTATCCCGC
AACACAGCCGCCTCTTCAAACTGCAAATCCCTAGCCGCCTGCTGCATGGCTTTTTCGAGT
TTGGCGATTTCTTTAATCGCATCTTCTTCGTTGTGAATCTCGCCCACTTTAACCTTGTTT
TTACCTTTCAGACGGCCTTTACTGCCGTCTTCTTCGTGGTACACGCCGTCGATGATGTCT
TTGACCTGTTTTTTAATCTGCTGCGGCACGATGCCCTGTTCTTCGTTGAATTTAATCTGT
TTTTCACGGCGGCGTTCGGTTTCGTCGATAGCGGCTTTCATGGAGTCGGTAATTTTGTCG
GCGTACAGGATGGCGACGCCGTTCACGTTGCGCGCGGCGCGGCCTATGGTTTGAATCAGG
CTGCGGTGGGAGCGCAGGAAGCCTTCTTTATCTGCGTCGAGGATGGCGACGAGAGAGACT
TCGGGGATGTCGAGGCCTTCGCGTAAGAGGTTGATGCCGACGAGTACGTCAAACAGGCCG
AGCCGTAAATCTCTAATGATTTCAACGCGCTCGACGGTGTCGATGTCGCTGTGCAGGTAG
CGCACTTTGATACCGAGTTCGCTGTAATAGTCGGTGAGTTGCTCCGCCATGCGTTTGGTG
AGGGTAGTAACGAGTACGCGTTCGCCTTTTTCAATGCGGTCGTTGATTTCGCTCATTAAG
TCGTCGACTTGGGTGGCAACGGGGCGGATGATGATTTGGGGATCAACCAGCCCTGTGGGG
CGGACGACTTGTTCGACCACTTGTCCGGCGTGTTCTTCTTCGTATTTGGCGGGGGTAGCG
GAAACGAAGATGGTTTGCGGCATGACTTTTTCAAATTCGTGGAATTTGAGCGGGCGGTTG
TCGCGGGCTGAAGGCAGGCGGAAGCCGTAGTCGACGAGGTTTTGCTTGCGCGATGCGTCG
CCTTTGTACATGCCGCCGATTTGGGTTACGGTAACGTGGCTTTCGTCGATGAACATGATG

## Appendix A

GCGTTGTCGGGCAGGTAGTCCATCAGCGTAGGCGGCGGTTCGCCTTCTTTTTTGCCGGAA
AAGTGGCGGGAGTAGTTTTCGATTCCTTTGCAGAAGCCCATTTCGTAGAGCATTTCGAGG
TCGAAACGGGTGCGCTGTTCGATGCGTTGTTGTTCGACGGGGCGTTGTTCGCGGGCGAAA
AATTCGATGCGTTCGCGTAATTCTTCTTTGATGGACTCGCAGGCGCGCAAGACGGTGTCG
CGCGGGGTAACGTAGTGGCTGGACGGGAAGACGGTGTAGCGGCCGACGCGCTGGATAAGG
CTGCCTGAAAGCGGGTCGAACATATCGAGGCGGTCGATTTCGTCATCAAACAGGCTGATG
CGTAAGGCGTTTTCGGAGCTTTCGGCGGGGTACACGTCAATCACGTCGCCGCGCACGCGG
AAGCTGCCGCGTTTGAAGTCCAAATCGCCGCGTTCGTATTGCATGGAAACGAGCGTGGCG
ATGATGTCGCGCTGCTCGATGGTATCGCCTTCTTTGACGGACAACACCATTTGTTGATAC
TCGGTCGGGTCGCCGATACCGTAAATGGCGGACACGGTGGCGACGATAATCACGTCGTTG
CGCGTCATTAGGTTTTTGGTGGCGGAAAGGCGCATCTGCTCGATGTGTTCGTTGATCGCG
CTGTCTTTTTCGATGAACAAATCGCGGCTGGGCACATAGGCTTCGGGCTGGTAATAGTCG
TAGTAGGAGACGAAATATTCCACTGCGTTTTCGGGGAAAAATTCGCGCATTTCGGCGTAA
AGCTGGGCGGCAAGGGTTTTGTTGTGCGCCATGATGATGGCGGGGCGGCCGCTTTGGGCG
ATGACGTTCGCCATGGTGTAGGTTTTGCCCGAACCGGTTACGCCGAGCAGGGTTTGATAG
GCAAGGCCGTCTGAAAGCCCTTCGAGCAGGCCTGCAATGGCGGTGGGCTGGTCGCCTGCG
GGCGGGAAGGGTTGGTGGAGTTTGAAGGGGGAATTTGGGTATTGGATAACTTCCATAATC
TTGCCTGTGATGCGTTTGCGGACAAAGCGTGCAGTAGGGATGGGTCGGAAACGTCTTTCA
GACGGCATAAGGCGGTGAAATCCTGAATGTATGCCGTCTGAAACCCAATCGCTACCCAAG
TATAGTGGATTAACAAAAACCAGTACGGCGTTGCCTCGCCTTGCCGTACTATTTGTACTG
TCTGCGGCTTCGTCGCCTTGTCCTGATTTTTGTTAATCCACTATAAATGCCGCACGGTTC
AAATTCCGGTAAAAAATCGCTCATAACCTGTCCTTTCAAACATAATATGCCGTCTGAAAT
CCTTTCAGACGGCATCGTCAAAACCTACTTCTTATCTTTTTTATCTTTCTTATCTTTATT
TGAAACCGGCTTTTTCGCCGCCAGCCCCAAAGACTTCTGCCACTGCTCGGGCGACTTGAC
TAAATCCAAAGCTTTCCGCAACTGGTCGTCTTTGGCAGGGTTGGGAATCCGCCTTGAAGA
CAAATCCTCGTCCTTTTTCTTTTTACCTTTTTCTTTTACAGCGGGCTTATCCGCATCTTT
TTCAAGCGGCACGGCAAGGGTTTCACCGTTCACATCCTCGCCGCCCAAGGGATTGCCGAT
GTGTCCGACCAAATCCGCCTCGCGGCTTTCAAAAATGCGTTCCTTATCTTTTACTTCGAC
ATCGGGAACAATCCCCTGCGCCTGAATAGAACGGTCGTTCGGCGTATAATACAGTGCCGT
TGTCAGCTTGACCGCGCTGCCGTTGGACAAAGGAATCAAAGTCTGAACCGAACCTTTGCC
GAAGCTCTGCGTACCGACGATGACCGCGCGTTTATGATCCTGCAATGCACCTGCGACAAT
CTCCGACGCGGAAGCCGAACCGGAATTGACCAATACCGTCATCGGTATGGTTTTCAACTC
GGCAGGAATGCCCGCCAACGAATCGCCGCCCATCCCGTACACATAATCTTCAGGAATGGC
TTTCAGTACCATGCGGTCTTTGCCGTCGCGTCCCTTGGTGCTGACGACGACTGCTTCAGA
CGGCAGAAATGCCGCCGACACGCCGACCGCGCCAGTCAAAAGCCCGCCGGGGTCGTCGCG
CAAATCCAACACCAGCCCCTTGAGCGGTTTTCCTTTATTTTCCTTTACCAGCTCTTTTGC
GGCGGTATTGACGCTTTCGACCGTCCGCTCTTGGAACTGCGACACGCGGATATAGCCGTA
ATCGGGTTCGATCAGGTGATGGCGGACGCTTTTCACTTTAATAATGGCACGGGTCAGGTT
GACGACTATCGGCTTGTCGGCATTTTTGCGCGACAGCGTCAAAGTAATCTTCGTACCCGG
CTTGCCCCGCATTTTCTTCACCGCTTCGCTGACCGTCATGCCGCGTGTCGAAACATTATC
GATTTTCACAATGAAATCGCCGCTTTTCACCCCCGCCCGTTCCGCAGGCGTGTCCTCAAT
CGGCGAAACCACTTTGACAAATCCGTCTTCCTGCCCGATTTCCATCCCCAAGCCGCCAAA
TTCGCCGCTGGTGGACTCCTTTATCTCGGCATAACCTTTTTTATCCATATATTCGGAATG
CGGATCCAAACCGGCCACCATACCCTTCATCGCACCTTCAAACAAATCGGCATCGGGTTT
GTCCTGATAGTAGTTTGCCTTGATTTGACCGTAAACCTCCGCCATTGTGCGGATGGATTG
CACCGGCAGGACTTCGTTATCCCGCCTGTCCTTCTCGGCGGCAAAACCCTGCACCGCCAG
ACTGACGGCCACGCCGCTGATTGCACCCAAAGTATAAAGTGCGATTTTCTTAAAAACAGG
TTTCGACATTCTTCTTTAACTTTCTCTCTTGATTTCCAAAAACCGGAAAATACAGGTACG
GGAAACGGCAAAGTTCAGGGAACAGCGCACCATATCGGCACGATTTGCATAAAGCCTACC
GTTTCGGCAATCCGATCAACGTATCCAGCTCGAAGGGTTCAATACCTGACCTTGATAACG
TATTTGCAGGTAAAGCCCCTCTTCCCCGTCCGGCAGCGACCCGCTCGAGCCGATTTTGCT
TCCTGCCGCGACCATATAACCCTTGCCGACGGAAATTTCGCTCAAACCGGCATAGATGCT
GATGTAGTTCTCGCCGTGATCGACCACGACCACTTTGCCGTAGCCGTCCAACTCGTCCGC
ATAGCTTACCGTTCCCGGCGCAATGCTTTCAACCGTTGCCGGTGCAGTGGAATAGAACAC
GCCTTTCCAAATATCGCCGCCGCTCCGGTTCTGCCCGAAAAGTCCGGTCGGCACACCGTC
AACCGGTTTTTTCAAACGTCCTTGCATGCGGCTGAAACCGTCGGCACTGCCGATACCCAT
AACCGAAGGCGCTTGGATGTTCCTGTCTTCGGCGGTCAGGTTGGACATTTCCGCACGTCG
TGCTTCAGCCTTCTGCTGCGCCGCTTCTTTTCTGGCTTTTTCGGCTGCCGCCAGTCTGGC
TTCAGCCAATTTTCTTTTTGCTTCCGCATCCTGAATGCGGTGTTCGGCCTTTTTCTTCTC
CAAATTGCTCAAGAGCTTGTTCAGCTGCTGCTCGTTCCCTTTCTGTTCCAGCAGTTTTCG
GGCATCTTTGGCGATTTTGGCATTCTGTCTGCGGCTTTCCGTCTGTTCCGCCGCATCGGT
TACACCCTGTTTTTTCAGCAGAGATTGCACGTTTGCCTGAATTTTCTTCAAACGGGCAAG
CTCATTGTTGATTTTCTGCTCTTGTACCGCCAAAGCCTTCTGCTGTTTTTCCAAATCCTT
GACAACTTCCCGATTGGAGGCGTTTACATAACGCGTATAACGCAAAAAGCGGTTTTTCTG
ACCCGGTTCGGCGTTTTTCAGGAACAGGGCAACCGCATTCGGCTGGCTGTTTTTATAGTT
CCCCGATACGAAACGGGAAATCTGCGCTTTCGTAGCGGCGACTTCCGTTTTCAAACGGTT
CAGCTCGGTATTGAGTTTTTGGAACTTGTCCCAAGCCTCGCGCTGTTTGCGGTTGACGGA
AGCAAGGTTGCCGCGCGCCTGACGGATACGCTCTTGGCGGATACGCTCTTCCTGAAGCTG
TTTGAGGGAATTGCTGACATGAAGCAGCATTCCTTCGCTTTGTTTGAGCAGGGCTTTTTT
GTTTTCGACATCATTGGTGGCAGCGGCAACGGCGGCTTTCAATTCGTCGGAATCGGTTTT
GGCATTTTTCTCTTCCCTGTATTTTTTGTCCTGTTTCACTGCTTTGCCGTTCTTGTCGGA
ACGGACTTTTTTATTTGCAGAAACAGTGTCTTTTTCCGCCTTGCCGCCCTTGCGCGGATT
GCCCTGTCCTTTTGCCTCTTTTTTGCCTTGTTTGTTTTCAGGCTGTGTTTTTGCGTTTTT
TTCTTTGGATCCGGACACGGGCTTGCCATGTGCCTTTTTGTGTTCCGCCTTCGATTTCTT
ATCCCCTTCGCGTCCTTTGCGCGCAGACTGCCTGGATGTCGCCTCCTTCTCTGCTTCTTT

## Appendix A

GCGGTTTTTGGCGGTTTTTTTGGACTCTTTGCCCTCTTTTGCCGCCTCTTTGCCGCCTGT
TTTTTTATCTTTCACTGCGCCATTTTTGCCTTTTTCTTTTTTGCCTTCCGCCGCTTCGGG
CTGTTCTTTTTTGTTCTTCGTCTGTTTTTTCACTTCGGCGGAACGGTTGTGTGCCGCGTC
GTGGGCGGCAACGGCGGGCGTGGAAAAAACGAGCATCAGGGCAAGCAGAAGGGGTTTGTA
GCGCATGGTTCGACCTTCGGAAAAAGTTGGATAATACTGAAGGCTGCACGAAAGCAGCCG
GACGTTTGGATTATACTGTCAGTTATGCCGTCTGAAAATGCCGTTTGCCCAATCTTGCGC
CTTCTTTGCGCGGATACTTGCAATCGGCTCAAACAGCCTTATATTGTGCGTCATATTTTC
AATGCCGCAACGGATATTGTGTTCCGACACACAGGGTAGCACATTAAGCCGCATACCGTA
TGTTGCCCGATTTTGGGAACGTGCGCCCCTCCAAACAAAGCAAGCCCTGCCGCTTTCACG
GAAAACGGGGATTCAACCGATAAGGAAATTTTGATGAACAGACTGCTACTGCTGTCTGCC
GCCGTCCTGCTGACTGCCTGCGGCAGCGGCGAAACCGATAAAATCGGACGGGCAAGTACC
GTTTTCAACATACTGGGCAAAAACGACCGTATCGAAGTGGAAGGATTCGACGATCCCGAC
GTTCAAGGGGTTGCCTGTTATATTTCGTATGCAAAAAAAGGCGGCTTGAAGGAAATGGTC
AATTTGGAAGAGGACGCGTCCGACGCATCGGTTTCGTGCGTTCAGACGGCATCTTCGATT
TCTTTTGACGAAACCGCCGTGCGCAAACCGAAAGAAGTTTTCAAACACGGTGCGAGCTTC
GCGTTCAAGAGCCGGCAGATTGTCCGTTATTACGACCCCAAACGCAAAACCTTCGCCTAT
TTGGTGTACAGCGATAAAATCATCCAAGGCTCGCCGAAAAATTCCTTAAGCGCGGTTTCC
TGTTTCGGCGGCGGCATACCGCAAACCGATGGGGTGCAAGCCGATACTTCCGGCAACCTG
CTTGCCGGCGCCTGCATGATTTCCAACCCGATAGAAAATCTCGACAAACGCTGATATGAA
CCTCTCCAACCACTTTCTCATCGCCATGCCCGATATGGAAGACGCGTTTTTTTCACAATC
GGTCGTCTATATCTGCAAACACGATGAAGACGGCGCACTCGGCATCGCCATCAACAAACC
CTCTCCGATTACGATGGACATGATTTTTTCCGCCACCGGCAAAAACATCCCCATGCGGAT
GCAGCACGACAGCGTGATGATGGGCGGTCCGGTGCAGGTCGAGCGCGGTTATGTCGTGCA
TACCCCGATCGGCAACTGGCAAAGCAGTATCGGCGTTTCAGACAATATCGCGCTAACTTC
TTCCCGAGACGTGATTGAAAATATTTCACGCGAAGGTGCGGTTGACAAAGCCTTGATCAG
CATAGGCTATTCAAGCTGGAGCAAAGGGCAGCTCGAACGCGAACTTGCCGACAATGCGTG
GCTGACTGTTCCCGCCGACGAACACATCCTGTTCGACATCCCCTACGAACACCGTTACGC
CGCCGCATTCGCCAAACTCGGCATCGACCCGCTCGCCCTGTTTTCAGGAGCCGGCCATGC
ATAAAATTCCAAAAGGAACGGCACTGGCATTCGACTTCGGCGAAGCGCGTATCGGCGTGG
CACAAGGAGACGCGGAATTAGGGCTATCCCATCCTTTGAGCACCGTTACCGGCGGCAGCA
ACGATGAAAAGTTCGCGGCAATCGCCAAGCTGGTTCAAGAATGGCAGCCGCGTTATTTTG
TCGTCGGACTGCCCGTGCATACCGACGGCACGAAACATGAAATGACGCACCTGTCGCGCA
AGTTCGGACGCAGGCTGAACGGCAGGTTCAATCTCCCCGTCTATTGGGTTGACGAACGGC
TGTCGTCCGTCTATGCCGAAAGCCTGCTTTCGGAAGCACAGGTCTTCGGCAAAAAACGCA
AATCGGTGCTCGACCAAGTGGCGGCGCAAGCCATCTTGCACGGTTTTTTCGAGGGCGGTC
CGGCGGAATGTTTCAACGGGCGTGAGGGTTAAGCGGCGCGGTTAACACCCTACCGTGAAA
GAGGCGCGCACCAAGCCGTCCAGCTCCAATGCCAAATTGTCCCCCGCACCGATTGCGCCC
ACGCCGGAGGGCGTTCCGGTAAACACCAAATCCCCTTTCCCCAAACCGTAATCTGCCGCC
AGTTTGTGTAAAATTTCCCGAATCGGGTAAATCATCAAACCGGTATCCCCGCGCTGTTTC
AATACGCCGTTTTGTTTTAATGAAAACAACACCTTCTCGGGATTGCCGATTCTGCCTGCC
GCCGCAAAATCCGACACGCACGCGGAATGCCTGAACCCTTTTGCCTTCAGCCAGGGCAGC
CCTTTTTCCTTCAGACGGCATTGGATATCCCGTGCCGTAAGGTCCAGCCCTACACCATAT
CCTGCGACACATCCCAAAATATCTTTACCCTCGCCCGTGCCGTCTGAATCCTTACCGACC
AGCAGCACGAGTTCGCACTCAAACTGCACATCCCTACTAAACTCGGGCAGCAAGATTGTA
CCGCCGCTGTTCAAAATGCTGCCTGACGGCTTCATAAACACCACAGGTTCGGAAGGTATT
TCGTTTTTTAACTCTTCGATATGTGCGGCATAGTTCCTGCCGATACAGAAAATATTGCCG
ACCTCGACTGCCTCTCCTTCTAAAAATACTGAAGCCACTTCACTTTCCCCCTAAGTAAAA
ATGCCGTCTGAAATTATTTTCAGACGGCATTCGACCAAGCTTACGCATTTAATGAAGCTG
TTACACGTGCAACAATTTCTCCGATTGCAACTGCCTGCGCTTCGTTGTCGCGGCGTTCGG
CGTATTCGACATTGCCTTCTTTCAAGGCGCGGTCGCCGATGACGATGCGGTGCGGAATAC
CCAACAGCTCGGAATCGTTCAGCAACACGCCTGCGCGTTCGTCGCGGTCGTCGAGGAGGA
CGTCTGCGCCTGCCGCCAGCAATTCGGCATAGATTTTGTCGGCGGCTTCGCGTACGGTGT
CTGATTTTTTGTAGTTCATCGGCACGATAACGACTTCAAACGGCGCCATTGCTTTGGTCC
AGATGATGCCTTTTTCGTCGTTATTCTGCTCGATGGCGGCGGCAACGACGCGGGTGATGC
CGATGCCGTAGCAGCCCATTTCCATAATTTGCGATTTGCCGTTGTTGTCAAGGAAGCTTA
CGTTCATGGCTTGGGTGTATTTGTCGCGCAATTGGAAAACGTGTCCGACTTCAATGCCGC
GCGCCAGTTTCAGACGGCCTTGCCCGTCGGGGCTTTCGTCGCCCTCGACGACGTTGCGCA
AATCGACAAACTCAGGTTCGGCAGCGTCGCGGCCGAAATTGAAGCCGGTATAGTGGTAGT
CGTCTTCGTTTGCGCCGATGACCCAGTCCGCGCCTTTTTCGGTAGCGAAATCGGCATAGA
CTTTGCCTGCAAAACCGACAGGGCCGAGAGAGCCGCCGTTTGCGCCGAACTGTTCGACAA
TCGCGGCAGGGCTTGCCATCGTCAGCGGCGATTTCACGCCCGCGAGTTTCTCGGCTTTGA
TGTCGTTAAATTCATGGTCGCCGCGTAACAGCAGCAGGATAAGTTCGCCTTCGTTTTCGC
CTTCAACCACGATGGATTTCAGTGTTTTTTCAATCGGAATACTGAGGAAATCAACCAATG
AATCAATGGTTTTGACGTTTGGCGTGTGTACTTTGACGAGTTCTGCCTGAGCGGCTGCAC
GTTCGCCTTTGAGCGGCAAGGTCGGCGCTAACTCGATATTGGCGGCGTAATCGGAAGTGT
CGCTGTATGCAATCACATCTTCGCCGCTTTCCGCCAACACTTGAAACTCGTGCGAACCGG
TACCGCCGATGCTGCCGGTATCCGCAGCAACGGGTCGGAACGCCAAGCCTAGTCGGGTAA
AGATGCGGCAATAAGCATCATACATATCTTGATAGGTCGTCTGGAGCGAGGCATAGTCGG
CGTGGAAGGAATAAGCGTCTTTCATCACAAACTCGCGCGCGCGCATCACGCCGAAGCGCG
GGCGCACTTCGTCGCGGAATTTGGTTTGGATGTGGTAAAAGTTTTTCGGCAGCTGTTTGT
AGCTGTTGATTTCTTTGCGCACGATGTCGGCGATGACTTCCTCGCAGGTCGGGCCCATGC
AGAAATCGCGGTCGTGGCGGTCTTTCAGGCGCAGCAGTTCTTTACCGTAAAACTCCCAGC
GGCCGGATTCCTGCCACAGCTCGGCAGGCTGCACCACCGGCATCAGCAACTCCACGCTGC
CCGCGCGCGCCATTTCCTCGCGCACGACGTTTTCGACTTTGCGTAACACGCGCAGCCCCA
TCGGCATCCAAGTATAAAGACCCGATGCGTTGGCCTTAATCAGGCCGGCGCGAATCATCA

## Appendix A

GCTTGTGGCTGGCAAGCGCGGCTTCGGCAGGGGCTTCTTTTAAAGTAGAGATAAAGAATT
GGCTGGCTTTCATAAAAGTATTTTTCCAAACAGGCAAATTCAAAAGTAAATCGGGTGCAG
ATTGTAACGCGAAAAAAGCAGGTTTTGCACCAACCTCCAAAATTCACCCCCTGCCCCAAG
CGCGGGACAAATCCCATAACAGACGGCAAAAACATGACCAGAAACATCATATTGAACATA
AGCACATGATTTTTATAGATTTAAATGTGCCTATTTTTTAATCAAAATAAGCGTACATTT
GTTGCGTAAGACTTTTTTAACACAAGCCGTGGCTTATCAACACGGTTATCCACAAAGCTT
GTGTATAGATTTTCTACAATAGGAAAATTGCCGACAGAGACATAATGATTCGATATACCA
CAATTCCGAAAAAATATCGCCAAAATCAAACAGAATATTTCGAAATCAAAAAGACTTGAC
CTTACCAAACGCCAACTTCAGTATAAAACCTGCTTTTACAGGCATGGTTATTTGCCAGCA
GACCCGATTGCTGATAGGATTTCGTGTGGAGCAGATCGAACATTTTTTTCAAGTTTTCCC
TTGTTTCCAAAACTTTTATAATTTTTTGAAAACATTAAACTTAAATTATTTTTTTCGGTT
TGATTTAGAAATTTTCGTTTTTGCTTATTATTTTTCACAAACGAAAATAAAGGGGTTGGC
TACACCCTCCCTGCCGATTAAACACTCAACATAAAGGATAGATACTATGTCCACCCAATT
ACACGATGTTGACCCTATCGAAACCCAAGAGTGGCTGGACGCGTTAAGCTCCGTCCTCGA
ATATGAAGGCGGCGAACGCGCGCAATACCTCTTGGAAAACCTGGTCAAATACTGCCGCGA
CAAGGGCGTACGTATGCCACACGGCACGACCACCCCGTATTTGAATACCGTTTCGGTTGA
AAACGAAAAAGGCATTCCGGGCGACCAAAACATCGAACACCGCATCCGCGCATTCGTGCG
CTGGAACGCCGCCGCCATCGTATTGCGCGCCGGCAAGAAAGATTTGGAACTGGGTGGGCA
CATCGCATCTTTCCAATCTGCCGCCACCATGTACGAAGTCGGTTTCAACCACTTTTGGAA
AGCCAAAGGCGAAGGCGAAGAAGGCGATTTGGTCTTCTTCCAAGGTCACGTCGCCCCGGG
CATCTATGCACGCGCATTCGTCGAGGGCCGTCTGACCGAAGACCAGCTGAACAACTTCCG
CCAAGAAGTGGACGGACACGGTCTGCCTTCCTATCCGCACCCCCACCTCTTGCCCGACTT
TTGGCAGTTCCCGACCGTATCCATGGGCTTGGGGCCCATCATGGCGATTTATCAGGCGCG
TTTCCTGAAATACTTGGAATCGCGTGGTTTGGCAAAAACCAAAGGCCGTAAAGTATGGTG
TTTCTGCGGCGACGGCGAAATGGACGAACCCGAATCTCAAGGTGCAATCGCACTGGCTGC
ACGCGAAGGCTTGGACAACCTGATTTTCGTCATCAACTGCAATCTGCAACGCTTGGACGG
TCCGGTACGCGGCAACGGCAAAATCATCCAAGAATTGGAAGGCAACTTTGCCGGCGCCGG
CTGGAATGTCGTCAAAGTCATTTGGGGCCGCCGCTGGGACCGCCTCTTGGCGAAAGACAA
AGACGGTATCCTGCGCCAACGTATGGAAGAATGTTTGGACGGCGACTACCAAACTTACAA
ATCCAAAGACGGCGCGTATGTGCGCGAACACTTCTTCAATACGCCCGAACTGAAAGCATT
GGTTGCCGATATGACCGATGAGCAACTCTGGGCATTGAACCGCGGCGGCCACGACCCGCA
AAAAGTGTACAACGCCTACGACCGCGCAGCGAACCATGCCGACGGCAAACCTACCGTCAT
CTTGGCGAAAACCATTAAAGGTTACGGTATGGGCGCATCCGGCGAAGGTCAGAACGTTGC
CCACCAAGCCAAAAAAATGGACAAAGCGTCCCTGAAACAATTCCGCGACCGCTTTGACAT
TCCGGTTACCGACGAACAAATCGAAAGCGGCGATCTGCCTTACCTGACTTTTGCCCCCGA
TACGGAAGAATACAAATACCTGCACGCACGCCGCGATGCTTTGGGCGGCTACCTGCCGCA
ACGCAAACCGACGCAGGAAGTATTGGAAGTGCCCGAGCTGTCAGCATTCGACGCACAACT
CAAATCCAGCGGTGAACGCGAGTTCTCGACCACGATGGCATTCGTCCGCATCCTGTCCAC
TTTACTGAAAGACAAAAAAATCGGCAAACGCGTCGTACCTATCGTTCCCGACGAAAGCCG
TACTTTCGGCATGGAAGGTATGTTCCGCCAATACGGTATTTGGAATCCGAAAGGTCAGCA
ATATACCCCTCAAGACAAAGACCAACTGATGTTCTATAAAGAATCCGTTGACGGTCAAAT
CTTGCAAGAAGGTATTAACGAACCGGGCGCGATGGCCGACTGGATTGCGGCTGCAACCAG
CTACGCCAACAGCAACTTCGCCATGATTCCGTTCTACATTTACTATTCTATGTTCGGTTT
CCAACGTATCGGCGACTTGGCTTGGGCGGCGGGCGATATGCACGCGCGCGGCTTCCTGCT
GGGCGGTACTGCCGGCCGTACGACGCTGAACGGCGAAGGCCTGCAACACGAAGACGGCCA
CAGCCACATCCAGGCCGACCTGATTCCGAACTGCGTATCTTATGACCCGACTTTCCAATA
CGAAGTCGCCGTCATCGTACAAGACGGTCTGCGCCGTATGTATGCCAATAATGAAGACGT
GTTCTACTACATCACCCTGATGAACGAGAACTACACCCATCCGGATATGCCCGAAGGTGC
GGAACAAGACATCTTGAAAGGTATGTACGTGCTGAAAGCCGGCGGCAAAGGCGATAAGAA
AGTTCAATTGATGGGCTCCGGTACCATCCTGCAAGAAGTCATTGCCGGTGCCGAGCTGCT
GAAAGCCGACTTCGGCGTAGAAGCAGACATCTGGTCTTGCCCGTCCTTCAACCTGCTGCA
CCGCGACGCTGTCGAGGTAGAACGCTTCAACCGCCTGCATCCGCTGGAAGCCGAAAAAGT
ACCTTTCGTTACTTCCCAACTGCAAGGTCATGACGGTCCGGTTATTGCCGCTACCGACTA
TATCCGCAGCTATGCTGACCGTATCCGCGCCTACATCCCGAACGACTACCACGTCTTGGG
CACTGACGGTTTCGGCCGTTCCGACAGTCGCGCCAACCTGCGCCGCTTCTTTGAAGTGGA
TCGCTACAACGTTGCCGTGGCCGCATTGGCCGCATTGGCGGAACAAGGCAAAGTCAGCAA
AGAAACCGTTCAACAAGCCATTGAGAAATACGGCATCAAAGCCGATTCAGCTCCTAGCTG
GAAACGCTGATTGATGTTTCAGACGGCCTGTTTGCCCCATTCCGACATCAGGCCGTCTGA
AAACCGAATGCCCGAATGGTTTGAGCAGACAAACCGTACCGATGCCGCCTGAAGCAGCTT
TCAGACGGCATCCAATGAAAAAGATTAAAGGAACTCAAATGAGTATCGTAGAAATCAAAG
TCCCCGATATCGGCGGTCACGAAAACGTCGACATCATCGCCGTAGAAGTTAAAGCGGGCG
ACACCATCGCCGTTGACGACACCCTGATTACACTGGAAACCGACAAAGCCACGATGGATG
TGCCTGCCGATGCGGCCGGTGTCGTGAAAGAAGTAAAAGTCAAAGTCGGCGACAAAATCT
CCGAAGGCGGCGTAATTCTGACCGTTGAAACCGGTGCCGCCGCCGCCGAAGCCGCCCCGG
CTGCTGCCGAAGCACAACCTGCACCTGCTGCCGCACCCGCTGCCGCAGGCGGTGCAACCG
TTCAAGTAGCCGTTCCCGATATCGGCGGCCATACCGATGTGGATGTAATCGCCGTTGAAA
TCAAAGTGGGCGACACCGTTGCCGAAGACGACACGCTGATTACTTTGGAAACCGATAAAG
CGACAATGGACGTACCTTGTACCGCTGCCGGTGTCGTTAAAGCCGTATTCTTAAAAGTCG
GCGACAAAGTATCCGAAGGCTCTGCCATTATCGAAGTAGAAACCGTCGGCTCTGCCGCAG
CAGCCCCTGCTCAAGCCGCTCAAGCTGCCGCACCGGCTGCCGCTCCGCCTCCGACTGCTG
CCGCCGCACCCGCCGCCGCGCCTGCACCTTCTGCACCTGCCGCTGCCAAAATCGACGAGG
CCGCTTTCGCCAAAGCACACGCCGGTCCTTCCGCACGCAAACTGGCGCGCGAATTGGGCG
TGGATTTGGGCCAAGTCAAAGGCACCGGCTTGAAAGGCCGTATCATGGGCGACGACATCA
AAGCCTTTGTGAAATCCGTGATGCAGGGCGGCGCGGCAAAACCTGCCGCAGCCAGCGCAT
CTTTGGGCGGCGGTCTGGACTTACTGCCGTGGCCTAAAGTGGACTTCTCCAAATTCGGCA

## Appendix A

ATGTCGAAGTTAAAGAATTGTCCCGCATTAAGAAAATTTCCGGTCAAAACCTGTCCCGCA
ACTGGGTTGTGATTCCCCACGTTACCGTACACGAAGAAGCGGACATGACCGAGCTGGAAG
AATTCCGCAAACAGCTGAACAAAGAATGGGAACGCGAAGGCGTGAAACTGTCCCCGTTGG
CGTTCATCATCAAAGCCTCTGTTTCCGCGTTGAAAGCATTCCCCGAATTCAACGCCTCAC
TGGACGGCGACAACCTGGTGCTGAAAAACTACTTCAACATCGGTTTCGCAGCCGATACGC
CGAACGGCTTGGTTGTTCCCGTCATCAAAGACGTGGATCAAAAAGGCTTGAAACAAATCA
GCCAAGAATTGACCGAATTGTCCAAAAAAGCCCGTGAAGGCAAGCTCAAACCGCAAGAAA
TGCAAGGCGCGTGCTTTACCATTTCCAGCTTAGGCGGCATCGGCGGCACAGGCTTCACGC
CAATTGTGAACGCTCCCGAAGTCGCCATCTTGGGCGTGTGCAAATCCCAAATCAAACCTG
TTTGGAACGGCAAAGAGTTTGCCCCGCGCCTGATGTGCCCGTTGAGCCTGTCCTTCGACC
ACCGTGTCATCGACGGTGCGGCCGGTATGCGCTTCACCGTATTCTTGGCGAAGCTGTTGA
AAGACTTCCGCCGCATTACCTTATAAAATAAAACATCCCTCTCAAGCAGTCTGATAATGT
TTGGATTGCTTGAGATTGATGAGTAATGGTGTTAAATTCAACCTTTAAATTAATAACTTA
TGGGAAATTTCTTATATAGAGGCATTAGTTGCCAACAAGATGAGCAAAATAATGGACAGT
TAAAACCTAAAGGTAATAAAGCTGAAGTTGCAATTCGTTATGATGGTAAGTTTAAATATG
ATGGTAAAGCTACACATGGTCCAAGTGTGAAGAATGCAGTTTACGCCCATCAAATTGAAA
CAGGTCTATATGACGGATGTTATATATCTACGACAACAGACAAGGAAATTGCCAAGAAAT
TTGCAACAAGTTCCGGCATCGAAAATGGCTATATATATGTTTTAAATAGGGATTTGTTTG
GTCAATATTCTATTTTTGAATATGAGGTTGAACATCCAGAAAACCCAAATGAGAAGGAAG
TAACAATCAGAGCTGAAGATTGTGGCTGTATTCCTGAAGAAGTGATTATTGCTAAAGAGT
TGATAGAAATTAACTAAGTTGAAAGGTCAATATAATGGCTTTAGTTGAATTGAAAGTGCC
CGACATTGGCGGACACGAAAATGTAGATATTATCGCGGTTGAAGTAAACGTGGGCGACAC
TATTGCTGTGGACGATACCCTGATTACTTTGGAAACCGATAAAGCGACTATGGACGTACC
TGCTGAAGTTGCAGGCGTAGTCAAAGAAGTTAAAGTTAAAGTCGGCGACAAAATCTCTGA
AGGTGGTTTGATTGTCGTCGTTGAAGCTGAAGGCACGGCAGCCGCTCCTAAAGCCGAAGC
GGCTGCCGCCCCGGCGCAAGAAGCCCCTAAAGCTGCCGCTCCTGCTCCGCAAGCCGCGCA
ATTCGGCGGTTCTGCCGATGCCGAGTACGACGTGGTCGTATTGGGTGGCGGTCCCGGCGG
TTACTCCGCTGCATTTGCCGCTGCCGATGAAGGCTTGAAAGTCGCCATCGTCGAACGTTA
CAAAACTTTGGGCGGCGTTTGCCTGAACGTCGGCTGTATCCCTTCCAAAGCCTTGTTGCA
CAATGCCGCCGTTATCGACGAAGTGCGCCACTTGGCTGCCAACGGTATCAAATACCCCGA
GCCGGAACTCGACATCGATATGCTTCGCGCCTACAAAGACGGCGTAGTTTCCCGCCTCAC
GGGCGGTTTGGCAGGTATGGCGAAAAGCCGTAAAGTGGACGTTATCCAAGGCGACGGGCA
ATTCTTAGATCCGCACCACTTGGAAGTGTCGCTGACTGCCGGCGACGCGTACGAACAGGC
AGCCCCTACCGGCGAGAAAAAAATCGTTGCCTTCAAAAACTGTATCATTGCAGCAGGCAG
CCGCGTAACCAAACTGCCTTTCATTCCTGAAGATCCGCGCATCATCGATTCCAGCGGCGC
ATTGGCTCTGAAAGAAGTACCGGGCAAACTGCTGATTATCGGCGGCGGCATTATCGGCCT
CGAGATGGGTACGGTTTACAGCACGCTGGGTTCGCGTTTGGATGTGGTTGAAATGATGGA
CGGCCTGATGCAAGGCGCAGACCGCGATTTGGTAAAAGTATGGCAAAAACAAAACGAATA
CCGTTTTGACAACATTATGGTCAACACCAAAACCGTTGCAGTTGAGCCGAAAGAAGACGG
CGTTTACGTTACCTTTGAAGGCGCGAACGCGCCTAAAGAGCCGCAACGCTACGATGCCGT
ATTGGTTGCCGCCGGCCGCGCGCCCAACGGCAAACTCATCAGCGCGGAAAAAGCAGGCGT
TGCCGTAACCGATCGCGGCTTCATCGAAGTGGACAAACAAATGCGTACCAATGTGCCGCA
CATCTACGCCATCGGCGACATCGTCGGTCAGCCGATGTTGGCGCACAAAGCCGTTCACGA
AGGCCACGTTGCCGCCGAAAACTGCGCCGGCCACAAAGCCTACTTCGACGCGCGCGTGAT
TCCGGGCGTTGCCTACACTTCCCCCGAAGTGGCGTGGGTGGGCGAAACCGAACTGTCCGC
CAAAGCCTCCGGCCGCAAAATCACCAAAGCCAACTTCCCGTGGGCGGCTTCCGGCCGTGC
GATTGCCAACGGTTGCGACAACGGCTTTACCAAGCTGATTTTTGATGCCGAAACCGGCCG
CATCATCGGCGGCGGCATTGTCGGTCCGAACGGTGGCGATATGATCGGCGAAGTCTGCCT
~~TGCCATCGAAATGGGCTGCGAGGCGGCAGACATCGGCAAAACCATCCACCCGCACCCGAC~~
CTTGGGCGAATCCATCGGTATGGCGGCGGAAGTGGCATTGGGTACTTGTACCGACCTGCC
TCCGCAAAAGAAAAAATAAATCCGACTGAATAAACAGCCGATAAGGTTTATTTGAGCAAA
TGCCGTCTGAAATGTTCAGACGGCATTTTCTATTTTACAGCGGATTAAAATATCTTCTCC
GACCTATAGTGGATTAACAAAAATCAGGACAAGGAGACGAAGCCGCAGACAGTACAAATA
GTACGGAACCGATTCACTTGGTGCTTCAGCACCTTAGAGAATCGTTCTCTTTGAGCTAAG
GCGAGGCAACGCCGTACTGGTTTAAATTTAATCCACTATAAAAACGAATCCGACACGGCT
TATCTAAAGGAATGGTTGAAAACGGCAGTTTCCAATACAACAAAATGCCGCCTGAACATT
TCAGACGGCATTTGACCCATTACTGCTGCGGCTCTGAAACCATACCGCCTTCATCAAAAT
CCGGCTCCGGTTCGTTTTGCAACGTTTTACCGTTCAATTTCAACTGATTGTTTTTCAGAG
AAATGGCAGTATCAATCTGGTCGCCGTTCAAAGTCAGATATTTTTCCCTTGCCATACTCT
GAACCGTACTGTCCACCATCAGGCGCAAGGTCTCGTTGATGTCGTCAAGACTTGCCCTGC
CTTCCGCCTCATCTTCGGCATTGACGCTGAAAATATTGCCTGCTTGACTGACCGCCAAGT
CTTCCAGCATTTTTTGGGGAATACTCATTCTGATGTCGGCTTCGGTTTTCTTCAGCATCA
AACCCAATTGATTCAAATCTTCCTTCTTCATGTCTTTAAACATGATTTTTCCGCCCACAT
CGATTTTTCCCGATGGCAGCGTGAATCGGAAAGTTTTAATGTCCAATACGGGATTGTTGG
TGAACAGTCCGGAAGCCTCTCCTTTGACGGCGGCAATCAAATCATTGCGGATTTGTTCCT
CGGTCATTTTTTTGGCGGAAATTTGTGCAAACTTGCGTTTCAATACGGTTAAGGCAGAAG
CATCGAGGTGTTCGGCAGCGATATGGATGTCCAGCGGGCCGTATTTTTCATCGCCGTACA
CCAGTGTATCGAAACGGAACTGCCCTTCACTGTTGATAAACGCGCCTGATTCCCCGGTCT
TGGTTGAAAAAGCCAGTTTGCCGACTTCGATTTTGGAAGGTGCGATGCTGCCGTTGGGAT
TGATAAACGCGCCAATCTGCAAATCGGTAACAAGATTGACCAGTTCGTTTAACTTGACGT
TGTAATCGACACCCTCTTTCCATTCTAGGGAGAATTTTTCCAAGGTCAGATTGCTGCTGC
CCAAAGCAAGCGGATTGATGCCGTCTGAAGTTTCCGAATCGAAATGCACTTTTTCAAACG
CGGCATCGCCTTTGTCTGCCAGCTTGATTTTAAACAAGGGGGCATCATAGCCGTTCCGGT
AGCTTTTGAAACCTTTTTGATAAACCGTTTCTCCCGTCAGGCCTTCCCAGTGCAGCCTGA
TGCCCGACAGCTCTTCATAATCGAAGGCGGGAACACTGACTTCCATTTTACCGCTGCCGT

## Appendix A

TAAAATAAACGGTATTGGCAAGGGAAGCCGGGACTTGTTTTCCAAAAAAGCGTTCCAGAA
CTTTTTCCGTTTCAGGCGCGTATTTGAACTCGGTTTCAATGTACGCCTGCGTGCCGAATC
CGCCGGCGAAAGGGCCGTGCGTGATATGGTTAACCAGCGTAACCGGCTGTTCCAACACTG
TTTTCAGGTTATCCGGCAGGTATTTTCGGGCATTATTCAGCAACTCGGGTTTCAGACGGA
TGACCGTCGTTTCCATAGAGGTAAACCAGCCGCGCTCATATTGGTGCGATTCGACGGTCA
AGAAGCCCGTTTCCTGCAATATTTTTTGCTGCTGCGTCAAGCTTTCTTCGGCTTTGACAC
CCAAATAATAAGGCGTGCCCAAAGCAACGCCGAGCAATGCTGCCGCAACCGAAATCAAAG
GTTTTTTCATCACTTCAAACAAGCAGGTTTCAAAGACGCTAGAATAGCATTATTTAAGCG
TATCCCGCCATATCTCTTTAAAAGAAATGCCGTCTGAAACCTGTTCGGACGGCATTTTCC
GGATATAGGGAAATCAGAAATCCAATTCCGCCTTCAGCCAGTAAGTGCGCGGCATACCGA
CGACGGCGAAGCTGCGGTCGTATTGGCCGCGCTGTACCTGCCAATAGTTTTTGTTGAACA
GGTTTTCCACCGAGCTGCTGACGGTCAGAGTGTTTTTGCCAAGCTTGGTTTTGTAGCGCG
CGCCTACGTCAATCAAGGTATAGGACGGGAAGGCGTATTGTTTTTGCGTGTCTTGGTCAG
ACTTGCCGAAATACGAAACATTACCGTTTAAAGTCAAGCCTTTGGCAAACGGTGTATCCC
ATTCCAAACCTGCTTTGGCAATTACGCGCGGATTGGCGACTTGTACGCCGTTAACCAGCA
TATCGCGTGAATTTGGATACTCTTTCACGGTCGATTGCAGATACATCAGACCCAAAGTCG
GACGCAAAGTATTGTTGAGCAAGTTCGCGTAGGTGTTGAACTCAATACCGCGATTGCGTT
CCATACCTTGCTCGTCGCCGGCCGCGCCGCCTTGCGCCTTATAGCGGGCGAAATCAGAAT
TATTGCCATAGGTCAGCGTTGTTGTTACCCCTTTTGTTGTCTTGGTAGTTGTATGACCGC
GCCAGTAGCCCGGGCGTTTGATTTGGAACGCGTTTAACGTGGTTACGAAATTGCCCCAGT
TTTTACGCACGCCCACTTCAAACTGGCGGCTGACACGCGGTTTCGCCATTGTCGTTTCGC
CGGAATCATCGGTTTTGATGTCGGCAGGCTCCAAGTCTTCCATATAGTTGCCGTACACAA
CCAAATCAGGTTGCGGCACCCACGCCGCCATCAGCATCGGGCTGAAACGTTTGGCATCGC
CGCTCTGTGATTTTTTGTCGGTATATTCGACTGTTTGGAAACGTCCGCCCAAAGTCAGGC
GGTATTTGTTATCCACGAAGCCCAAGGTGTCGGACAAAGCCAGGCTGTTGACTTTGATAT
TGGCATCCAAGTTGGCAGAGTTCTCCCAAGAATTGGGATAGTCGGCTGTAAACGATGCCA
ATTGATGCTCAATATTTCCGTTTGCCTTCACTTCTACCTTGCTAGCTCCGGCTGCCGTTC
CGCGTGATTTTTTCTTATTGGTGTATTCAACCGCTTGGAAACGTCCGCCCAAAGTCAGGA
CAAGCGTCCGCCGGCGTTTTGAATGTCCTGCATACGCGCGCGACCGCCGTTGGTTTTGCG
TTTTGCGTAGATGGAATCGAACGCTACGCGCAGTGTTTCGCCGCGATAGTCGGCATTTAC
CGCAAATTCTTTGTTGTCTTCGCTGTAACCGTGGCGCGGGGTGTCGCCGTGGCGCAGTTT
GCCGTTGGCGCGCACGCCGAATGCTTTGTTTTCGCCGAAACGTTGGCCCAAGTCGAACGT
ACCTTGGGCGCGGTTGTTGCCGAACCGGGCCAAACCGATTTTGCGGTTGCCTTCATCAGC
GGCTTTTTTGGTTTCGATATTGACGGAACCGGATACCGCGCCATCAGGGTTCATGCCGTT
TACGGCGGTGGACGCGCCTTGAATCAGTTGTGCGGAGCCGACTTGCACGCTGGTCGTGCC
TTGCGTGCCGTACATACCTGTCAAACCGTTGACGCTGAATTGGCGCGCATCAAGCTGATA
ACCTCTGAAATACAATCCGGTCAGCGTGTTGCTTTCGCCGCCGAACTGCCAAACGGAAGC
GTCTTTTTTCGCTACGGCATCCACCAAAGTACGCGCCTCGGTGTTGTTGAGGGCTTGTTC
GTCGTAGTTGACGACGGTAATCGGCGCGGTAAAGGCGTTGGCTTTGCCCATTCACTTGGT
GCTTCAGCACCTTAGAGAATCGTTCTCTTTGAGCTAAGGCGAGGCAACACCGTACTGGTT
TTTGTTAATCCACTATAAACAAATCGTACAGGGTTCTCCGTTTAATCAGATATGGGTTTC
CATCTTCGGCAGTTTCGGGCATTTAGCCGTTTCCACCTTCCTGCCCCCGCTGCCAGTAAA
AATGCCGTCTGAAATATCGGCGCGATACTTCAGACGGCATACCCGCCCGTTTTCCCGCAT
TCCCGGGAGCGGGCTGAAATTTAAACGTGTGCGGAAATGATTTTCAACATTTGCGCCAGC
ACTTTGGGATTGGCAGCCACAATATCGCCGCTTTCCAGCCAGCCGTCTTCACCCGACATA
TCGGTTACGATACCGCCCGCTTCCTGAACAATCAATGCACCGGCGGCAATGTCCCACGGT
TTGAGGTTAAACTCGAAAAAGCCGTCAAAACGTCCTGTTGCTACGGCGCACAAATCCAAA
GAAGCCGCACCTTCACGACGGCCGCCGGCGGTTTTTGCCAAGAAATCTTTCAAAATCGCC
AGATACTTGTCCATCATGCTTTGATCGACAACAGGGAAGCCGGTACCAATCAGGCAGCGG
TTCAGTTCGATGCGGTTGGAAACGCGGATGCGGCGGTCGTTGAGCAACGCGCCTTTGCCA
CGCGAAGCCATATATACGTCGTTGCGTTCGGGGGCGTAAACCAAAGCTTCTTGCAACACG
CCTTTGTGCAGCAGCGCCATAGAGATGGCGTATTGGGGATGACCGTGAAGGAAATTGGTC
GTGCCGTCGAGCGGATCGATAATCCATTCGTACTCGGCTGCGGCTTTGCCGTGGGAGCCG
CTTTCTTCACAAGTGATTTTGTGGTGCGGATAGGCTTCTTTCAAAGCCTCAACCAGGATG
ATTTCGGAATTGCGGTCAACATCGGAAACAAAATCGTTGAAGGCTTTGCTGTCGGTTTTG
ACGGCATCGAGATTGCCCGCGGCGCGTATCATCATCTGACCGGCACGGCGGGCGGCTTTA
AAGGCTGTATTCAAAAACGGATTCATCAGATTTCCTTAAGGGTGGCATACCGCCGGTTCG
GACGTACAGTCCTTCGGAGCGGCAAAATCGGAGTTTATTTGGTTGGGGTAAATCCTGCCA
AATCGGGTAAAATACCGCCTGACGCGTGTCTGCTTCAGGCGCAACGTTAAATTTCCGACG
TTGTTAAAGAACATTTCAGACGGCATTTGACCGTCCGAACGAAAAAGACGGCGCATTATA
CCCTATTCCATTCCGACCGAAAACCGAACATGACTACTCTCAAACCCGCCCTGCCCGCTT
ATCTGGACAACATCCGCATCATCCTCACGCGCACCAGCCATCCCGCCAACATCGGCTCTG
CCGCGCGCGCGATGAAAACAATGGGTCTGCACAAACTGACCATCGTCGCCCCAAATCTGA
TGGCAACGCCGATGACGGAAAACCCGCCCGTGTTTGACCCGGAGCATCCTCAATCGTTTA
AATTACCGGAAGAAAGCTTCATCCTCGCTTCCGGCGCGGCAGACGTTTTGGAAAATGCCA
CCATTGCCGCTTCTTTGGACGAAGCCCTTGCCGACACCACCATCGCCTGCGCCCTGACCA
GCCGCCGCCGCGAAATTACTGCGCCGCTGCAAACCCCGCGCGATTTGGTATCCGAATTAC
TGCAGACCGCAAACCGAGGCGAGAAAGTGGCACTGGTTTTCGGCAACGAGACTTTCGGCT
TGAGCATCGAAGAAGTCCAAGCCTGCAACCGACTGATGACCATCAACGGCAATCCCGACT
ATTTCTCGCTCAACCTCGCCCAAGCCGTGCAGGTCGTGTGCTACGAAATCTTCAGCCAAA
CCGGTTCGCCCATGACCCATCTTCAACAAGAAGACCACGCTGCGACCCACGAGCAAATCA
AAGGCATGGTCGCCCACATGGAAAGCGTGATGAACGACATCGGCTTTTTCAACCGCCGCA
ACGGCGAGCGTCTGATGCGCCGTATGCAGAGCCTGTTCGGCCGCGCCAATACGCAAACCG
AAGACATCGATATCCTGCGCGGTTTTTTCAATACCGTCAGCCACCGTATCCATAAAAAAG
ACTGATTAAGGCCGTCTGAAAACATTTCCAGCTTTTCAGACGGCATGACTGATATTCGGA

## Appendix A

TAAGCATGAATTACGCCCTAGACGCATTATGGTGGAAACTTACCAGCCAACCCGTCCGCG
ACCTTGCCTCGCTGCTGACTGCGCCGCCTTTGTGGCAAAGCGGCTGCGAATTGAGCGTGC
GAGAACTACTGGGAGAACACGGTTTCCGTTACCTTTTGGCATTGGATGCCGATCCCACGC
GGCTGACGGATTACCTCGCCCAACGCGCCCCGTTCGGCCACCGTCTCGGCATTTATGCCG
AAGAGCTGCTGGCTTTTTGGTTTGCCAATGCACCGCACGCCGAACTGCTCGCGCACAACC
TCACGGTTTCCGGTTCGGACGGCAATACGCAAGGCGCGGCGGATTTTGTGGCAAGGCTTA
ACGGCAAACCCTACCATATCGAGCTGACCTGCAAATATTACGGCGGCGACACGGACAGTC
CCGAAGGGATGCGCGGATTCGACCCCAAAGACACGCTGTTGGGAAAAGCCGCCAAACTGA
CCGCCCAACTCGGTCTGCCGCACACTTCAGACGGCATCCGGACCTTGCGGCAGCACGGTT
TGCCGCTTAACGTAAAACCCGTTTCCATCGTGCGCGGCATCGGATTTTTTCCACACGGTT
TCCATGCTTTTGAGCCACCGCTTAATCCATACGGTTGGCGCGGCATCTATATTCAAGATT
GGGCGGAATACGGGTTTAAACGCCAAGAAGTCCGCTACCATCTGCTCGACCGTATGGCCT
ACCTCGCGCCTGCGCGTGTCGCCGAAACCGAAACATTGAACGCAACCGAAATCCGCCGTA
TCGACCAAGGCTTGATTGCCGTTTTGGAATGTCGGCCGGACGGCTTTTGGCACGAAATCG
AACGCATTATGAAGGCCGTCTGAAACCCTTTCCCAACATTAACGCGTATATCTATTGAGA
GGCTTAGTGATGGAAATCTCATTTCCCATACAATTTATGAAAGAGTCATCCGAGTTAATA
AGGATATTGGATATGATAAATATAACAACAACATGCCAACTAATATTATGACGATCCAAA
CAAATAAGTATGGTAATTTAATAACTACGACCCCAGGTAGAATACAATGAAGAATAATGT
TAAAAATTGGACAACTAAAGAAGTCAAGCAATCATTAGATAAATTTAATAATATTTTAAT
TAAAAATACTTTTCTTCTTCAGTATCTGAAAAAAGAGTTTTCAGCTTCAAGTGCTTATTG
TTTGTCTATGCTCCCTGAAGAAGAAGATATATATGAAATATTGGTTAATGGGAATATTAT
TATTGATTTAGAATTTAATAAACATACAAATGAAACAGTTGTTATTAATGTTACTGATGT
TGATGAATACTTGAAAACTTTAACCAATGAGAGTGGTAGAGTATTTTTTACATTAGCAAA
AGAAATCGGCAAACAGAAAAACATTTAACAAGAGCGAAATACAAATTAAAAACTCAATGG
AGTGATGGCATATTTAGGATAGATATACTGAACGAAGAAAATAAATTTTGTTTTTTTCCT
CATGTTTTAGGGAGTGATTACAAAATGAAATCGCTGATGTGATTATATCGGATGCTGTTC
AAGCGACCTGAAAATAGAACTTTTTTCAGGCTGCCTTTGTAGTTAACGGAGAAATTTAGA
CAAATCCCGATTGCGCACTTTTAACACATCTTTCTTATTGCGGATAGAATACTAAGTAAT
GATAAAGATGCTATTGTTATTTTAAGGACGTTAGATTGATTATGAATAACCCACAGTAAG
AGAACCCATTACATTATGAACGCCGCACAACTCGACCATACCGCCAAAGTTTTGGCTGAA
ATGCTGACTTTCAAACAGCCTGCCGATGCCGTCCTCTCCGCCTATTTCCGCGAACACAAA
AAGCTCGGCAGTCAAGATCGCCACGAAATCGCCGAAACCGCCTTTGCCGCGCTGCGCCAC
TATCAAAAAATCAGTACCGCCCTACGCCGTCCGCACGCGCAGCCGCGCAAAGCCGCTCTC
GCCGCACTGGTTCTCGGCAGAAGCACCAACATCAGCCAAATCAAAGACCTGCTTGATGAA
GAAGAAACAGCGTTCCTCGGCAATTTGAAAGCCCGTAAAACCGAGTTTTCAGACAGCCTG
AATACCGCCGCAGAATTGCCGCAATGGCTGGTGGAACAACTGAAACAGCATTGGCGCGAA
GAAGAAATCCTCGCTTTCGGCCGCAGCATCAACCAGCCTGCCCCGCTCGACATCCGCGTC
AACACTTTGAAAGGCAAACGCGATAAAGTGCTGCCGCTGTTGCAAGCCGAAAGTGCCGAT
GCAGAGGCAACGCCTTATTCGCCTTGGGGCATCCGCCTGAAAAACAAAATCGCGCTTAAC
AAACACGAACTGTTTTTAGACGGCACACTGGAAGTCCAAGACGAAGGCAGCCAGCTGCTT
GCCTTATTGGTGGGCGCAAAACGAGGCGAAATCATTGTCGATTTCTGTGCCGGTGCCGGC
GGTAAAACCTTGGCTGTCGGTGCGCAAATGGCGAACAAAGGCAGAATCTACGCCTTCGAT
ATCGCCGAAAAACGCCTTGCCAACCTCAAACCGCGTATGACCCGCGCCGGACTGACCAAT
ATCCACCCCGAACGCATCGGCAGCGAACACGATGCCCGTATCGCCCGACTGGCAGGCAAA
GCCGACCGTGTGTTGGTGGACGCGCCCTGCTCCGGTTTGGGCACTTTACGCCGCAATCCC
GACCTCAAATACCGCCAATCCGCCGAAACCGTCGCCAACCTTTTGGAACAGCAACACAGC
ATCCTCGATGCCGCCTCCAAACTGGTAAAACCGCAAGGACGTTTGGTGTACGCCACTTGC
AGCATCCTGCCCGAAGAAAACGAGCTGCAAGTCGAACGTTTCCTGTCCGAACATCCCGAA
TTTGAACCCGTCAACTGCGCCGAACTGCTTGGGGTTTGAAAATCGATTTGGATACCGGC
AAATACCTGCGCCTCAACTCCGCCCGACACCAAACCGACGGCTTCTTCGCCGCCGTATTG
CAACGCAAATAAACCGGTTTGAACAAAATGCCGTCTGAACCCTTTTCAAAGCGTTCAGAC
GGCATTTCATCAATTATAGTGGATTAACAAAAATCAGTACGGCGTTGCCTCGCCTTAGCT
CAAAGAGAACGATTCTCTAAGGTGCTGAAGCACCAAGTGAATCGGTTCCGTACTGTTTGT
ACTGTCTGCGGCTTCGTCGCCTTGTCCTGATTTTTGTTAATCCACTATATTTTTGGGAAT
CTGTTTTACCCCAATATATAAAGCACCATATTAAGGCGGAGTGTCTTCCCCACTTTGACC
CGAACCCGGAAAAGACACCGCCCAAGCCAATCCTGATGCTGCCCCGACAGCCAACCATTA
AGGAAATCCTAATGAACTTTGCTTTATCCGTCATTATGTTGACCCTCGCCTCTTTCCTGC
CCGTCCCGCCTGCCGGAGCCGCCGTCTTTACTTGGAAGGACGGCGGCGGCAACAGCTATT
CGGATGTACCGAAACAGCTTCATCCCGACCAAAGCCAAATCTTAAACCTGCGGACGCGCC
AAACCAAACCGGCGGTCAAACCCGCCCAAGCCGACGCAGGGAAGCGCACAGACGGCGCGG
CACAGGAAAACAATCCCGACACTGCCGAGAAAAACCGGCAGCTTGAGGAAGAAAAGAAAA
GAATTGCCGAAACCGAACGGCAGAACAAAGAAGAAAACTGCCGGATTTCAAAAATGAACC
TGAAGGCGGTGGAAATTCAAATGCAAAAAACAAGGATGATTTGATTCGGAAATACAATA
ACGCCGTAAACAAATACTGCCGTTAATCGGCTCTAGCGCAAACCCGATGCCGTCTGAAGC
GGCACGGGGTTTGTCATTTCTGCCAGTAGGTTTTGACGTTGACGAACTCGTACAGCCCGA
ATTCGGACAATTCGCGCCCGTAACCGGAATCTTTGACTCCGCCGAAAGGCAGGCGCAAAT
CGCTGCTGGTATGGCGGTTGATAAACACCGATCCCGCCTGTATTTTTTCGGCAAACCGCC
AAGCGCGTTCGGTATCGGCGGTATAAATGCAGGCACCGAGCCCGAACGGGGAATCATTGG
CAAGGGCGATGGCATGTTCTTCGTTTTCGGCGCGCAAAATCAGGGCGGCCGGCCCGAATA
CTTCTTCTCTCCAGACGCGGCAGGCAGGATTTACCCTGTCTAAAACCGTCGCGGGATAAA
ACCAGCCTCGCCCTTGTGGGATTTTTCCGCCGGTCAGGCATACCGCGCCGTTTGAAACGG
CATCTTCAACCTGCCCGTGAACCCTGTCCCGCAAATCTTCGCGGTGCAGCGGTGCAAGCG
TAGTATCGGGATGTTTGGGGTCGCCCATTTTCAATTTAGCGCATTCGGCAAGAAACAGCG
TGATAAAACGATCGGCTGCGGCTTCGGTTACGATGATGCGCTTGGCGGCGTTACACGATT
GCCCCGCATCGCGGAAACGGGAATAACAGGCTTCTGCGGCGGCACGCTCCAAATCAGCAT

## Appendix A

CGGGCATCACGATAAAGGCGTTGCTACCGCCGAGCTCCAACACGGTTTTCTTAAGGTTTG
CGCCCGCGTGTGCCGCAAGGATGCGCCCCGTATGCGTTGAACCGGTAAACGCCATTGCAT
CGGTATCTTCAACCGCCTTGAGCGTGCCCGCCTCATCCAGCCACACGCCTGCCAGAGGAA
TGCCGTCTGAAGCCAAATCGAACAGTGCCTGACTGACGCGTGCCACGCTGGGCGCGGGTT
TGACGGCGCACGCGTTGCCCGCGCACATAGCGGGAACGGCGAAACGCAATACCTGCCAGA
CGGGATAGTTCCAAGGCATGACGGCAAACACCACGCCCAAAGGCTCGAAGCGCACCTGAC
TCAAACTCGCCTGCGTCGCGATGGTTTTGTGGGCAAGCAGTTCGGGGGCAAGGCGGGCGT
AATAGCGTATCAGTTCGATAGACTTGCCGATTTCCGCACGGCATTCGTGCAAGCAGCGTC
CGACTTCCTCACACACCATTTCCGCAAAACGCTCTTTCTCCGCCTCCAAACGGTCGGCAA
ATTTTTGCAGGCGCGCGGCACGTTCGGTTACGCCCAGTTGCGCGAACGCCCCGCCGCGCA
TTTTCAAATCCGCCAGCCGCCGTTCAAACTCCGCATAATCTTGAGCGGGGCGGCGGTAAA
GCGTTTCGCCCGTAAATACATTGACACTGTGAAACATCGAATCAACCTGCCAGTTGCGGG
AATATCGTTTTCAGTCCCGACACAATAATCTCCACCGATACCGCCGCCAGCATCATACCC
ATAATGCGGTTTAAAATCGTCAGCCCCGTCGCGCCCAGCAGGCGGCTGACCTTCCCGGCA
ACGATTAAAATGGCATAACAAATCGCACTGACCACCAAACCGGCCGCGATAATCAACGCG
ATGTCGCCGTATGTTTTAGCCGCCGAAGCGTAAATAATCACGGTCGAAATACCGCCCGGG
CCGATGGTGATCGGTATGGCGATGGGCACGACGGCAATCGCTCCGGCATTGCGGGCGGGG
CGCGCCTGCCCCGTTTCCGGCTGCGCGCCGAGATTCTGCTTGGCGGGATTGTCGTTGCCG
TTCATCATCGAAATGGCGATCAGCAGCACCAAAATCCCGCCGCCGACCTGAAACGAACCG
ACGCTGATGCCCAAAACCTTCAGCAGCGTACCGCCGATCAGCGCAAATACCGCAATCACG
GCAAACACGGCAACGGCGGCCGTCCGCGCGACCTTCCTGCGCTCCTTCGTGCTGTGCCCG
TTGGTCAGGTCAAGGTAAAGCGACAACGCGCTAAACGGATTAATCAGCACCAAAAAAGCC
ACAATCAGCTTGCCGATTTCCATGCCCAATCCCATTATTTCCCCCTCCTTCAAACCCGTG
CGGCAGGCATCCGATGCTGCAAATTGCCGCCGCAACGGATTTTTCCGTTATAATTAAAAA
TTCAAGCAATACGCCCCATCATACCCGAACGACGGTATCTTTACCATCAGACAAGGATGC
TTTTCATGGCACTGACACTTGCCGACGTAGACAAAATCGCCCGACTCTCCCGACTGCACC
TGACTGCGGAAGAAAAAGAAAAATCGCTTCAAGAATTAAACGACATTTTCACTATGGTCG
AACAGATGCAAACCATTAACACAGACGGCATCGAACCGATGGCGCACCCGCACGAGGCCG
CCCTGCGCCTGCGCGAAGACGAAGTAACCGAAACCGACCGCGCCGCCGAATATCAGGCGG
GTGCTCCGGAAGTACGCAACCGTCTGTACATCGTACCGCAAGTTATCGAAGAATAATCCG
AATATGCTTCAGACGGCATCAGCAATACCGCCCGAAGCCCTTTAAGGATGGAAGATTTAT
GACCCAATACACATTGAAACAGGCAAGCGTCCTGTTGCAGTCCAAACAGATTTCCGCCGT
CGAACTGGCAAGCGCATACCTTGCCGCCATCGCCGAAAAAAATCCCGCCCTCAACGGCTA
TATCACCATCGACCAAGATAAAACCCTTGCAGAAGCCCGTGCCGCCGACGAACGTATCGC
GCAGGGCAACGCCTCCGCGCTTACCGGCGTACCCGTCGCCTACAAGGATATTTTCTGCCA
AACCGGCTGGCGCAGCGCGTGCGCTTCCAAAATGCTCGACAACTTCATCTCCCCCTACAC
CGCCACCGTCGTCCAAAACCTGCTCGACGAAGGTATGGTAACGCTCGGCCGCACCAATAT
GGATGAGTTCGCTATGGGTTCGACCAATGAAAACTCATTCTACGGTGCAGCCAAAAACCC
ATGGAATCTTGAGCACGTCCCCGGCGGTTCGTCAGGCGGTTCCGCCGCCGTCGTTGCCGC
GCGCCTCGCCCCTGCCGCGCTCGGTTCGGACACCGGCGGCTCTATCCGCCAACCCGCATC
GCACTGCGGCATTACCGGCATCAAACCCACATACGGCACGGTTTCCCGCTTCGGTATGGT
CGCCTACGCCTCCAGCTTCGATCAAACCGGCCCGATGGCGCAAACTGCCGAAGACTGCGC
GATTCTGTTAAACGCGATGGCAGGTTTCGACCCCAAAGACTCCACCAGCCTCGAGCGCGA
AAAAGAAGACTACACCCGCGATTTGAACCAACCGCTCAAAGGTTTGAAAATCGGCCTGCC
CAAAGAATATTTCGGCGAAGGCAACAGCGCCGATGTTCTGACGGCATTGCAAAACACCAT
TGATTTGCTGAAAGCCCAAGGCGCGGAATTGATTGAAGTTTCCCTGCCGCAAACCAAGCT
GTCCATCCCCGCCTACTACGTCCTCGCCTCCGCAGAAGCCAGCACCAACCTTTCACGTTA
CGACGGCGTACGTTACGGACACCGTGCCGCCCAATTCGCCGATTTGGAAGAAATGTACGG
CAAAACCCGCGCCGAAGGTTTCGGCAGGGAAGTGAAACGCCGCATCATGATCGGCACTTA
TGTACTGTCGCACGGCTACTACGATGCCTACTATCTCAAAGCCCAAAAACTGCGCCGCCT
CGTTGCCGATGATTTTCAGACGGCATTTGCACGGTGCGACCTCATCCTCGCGCCGACCGC
ACCCACTGCAGCCCCAAAAATCGGAGCGGATGCTTCGCCGGTTGAAACCTACTTGAGCGA
TATCTACACCATCGCCGTCAACCTCGCCGGACTGCCCGCATTGACCCTGCCCGCAGGCTT
CAGCGGCGGCGGACTGCCCGTCGGCGTTCAGCTTGTCGGCAACTACTTCGCCGAAGCCAA
AATCCTCGGTGCGGCGCATCAAATCCAACTCAACAGCGATTGGCACGGCAAACGACCCGA
ATGAAGCAGAACCGCACCTTTACCTTCCCCGATTTTCGCACCGTTTACAGCTATGCGCCT
TTATATCGGCTGCAACATTTAAAATACACATTGCGAAAATTTTTCGGAAAAAAAGAAATT
TACGCCTTCGAGCAGTTTGTCAACGCATCCCCTATCCGTCAGGGGCTGTTCCTCCACTGC
CCGCAAAATGCCTATCCGCTGCTGCGCGAATTTGTTGACAGGCGTTTTAACTGCAAACGC
CGTTTAGATGCGATGACGGCAGATTTTCTCATGGCGGAAAAACTGTTCGGCACAGACATC
CTGCACCAAATGGAAGACTACCGCTTCCATTTGGTCTTGGCGCACCTTTCAGACGGCATC
AGCTTGTGGCTCAACCGCAACGACAACTGCGTCGAAGAAGGCGCGTGGTCTTTATCTTTG
CGCGACGAAGCAGGCAACCGGCTGTATATGGCGACTTTCGCCTTTGTCGGCACACACCTG
CTGACAGCCTCCGTACAAGGGCCGGCGGGTGAAGAAGCCAAAGACACCGTCCGCCGCATA
ACCAAACAACTCCACGGCTTGCGTCCCCAACAACTGATGGTAACCGCCCTGCAATATTTC
GCCGCCGTACTCGGCTTGGACGGCGCAATGGGCATTGCACAAAAACATCAGGTCAAACTG
CGCTGGAAACTTAAAAAGCGCGTCAAAATGAATTACGACGCATTCTGGCAGGAATACGGC
GCAAGTTTGGAACGGGACGGCTACTGGCATCTCCCCCAAACCCCCGCCCGCAAAGACCTT
GCCGACATCGAAAGCAAAAAGCGTTCGATGTACCGCAAGCGTTATGAAATGCTGGACAAT
ATGGTTGCAGAGATGAAAGACAGTCTGAAAACAGAAGCACGCGGCATTTCAGACGGCATC
CAAACGGAAAAACCGCCCCGCCGGACAGCCTGACGCGAAGACTATCGAATTGATATTTTA
GAGAAAGAAGCTCTTATGACCTGGGAAACCGTAATCGGCTTGGAAATCCACGTCCAATTG
AACACCAAATCCAAAATCTTCAGCGGCGCATCGACCGCATTCGGCGCAGAACCCAACGCG
CACGCCAGCGTAGTGGAATGCGCGCTGCCGGGCGTTTTGCCTGTGATGAACCGTGAAGTC
GTTGAAAAAGCCATCAAATTGGGTTTGGCTTTAGATGCGAAAATCAATCAGAAAAACGTG

## Appendix A

TTCGACCGCAAAAACTACTTCTATCCCGACTTACCAAAAGGTTATCAAATCAGCCAGTTG
GACTTACCGATTGTCGAACACGGCAAATTGGAAATCGTAGTCGGCGACGATGTGAAAACC
ATCAACGTAACCCGTGCGCACATGGAAGAAGACGCAGGCAAGTCCGTGCATGAAGGCTTG
AACGGCGCAACCGGTATCGACCTGAACCGCGCCGGCACGCCGCTGTTGGAAGTGGTATCC
GAACCTGAAATGCGTTCCGCCGCCGAAGCCGTTGCCTACGCCAAGGCCTTGCACAGCTTG
GTAACCTGGCTGGACATTTGCGACGGCAATATGGCGGAAGGCTCGTTCCGCGTCGATGCC
AACGTATCCGTGCGCCCGAAAGGTCAAGAAGAGTTCGGCACGCGCCGCGAGATTAAAAAC
CTCAATTCCTTCCGTTTCTTGGAGCAGGCGATTAATTACGAAGCGGAAGCGCAAATCGAG
ATTTTGGAAGACGGCGGCAAAGTACAGCAGGCAACCATGCTGTTTGATCCCGAAAAAGGC
GAAACCCGCGTAATGCGCCTGAAAGAAGATGCGCACGACTACCGCTACTTCCCCGACCCT
GATTTGCTGCCCGTTATCATTTCAGACGCCCAAATGCAAAAAGCCAAAGCAGAAATGCCC
GAGCTGCCGAAAGAAATGGCAGCGCGTTTCGTGGCGGATTACGGCGTGTCCGAATACGAC
GCGCGCCTGCTGACCGCAAGCCGTGCGCAGGCTGCCTATTTTGAAGAAGCCGCCAAAGAA
AGCGGACAAGGCAAGCTGACTGCCAACTGGATGAACGGCGAACTTGCCGCCGCGCTGAAC
AAAGAAGGCATGGAACTTGCCGACAGCCCGATTACCGCCCCGCGCCTCGCCGCGCTGGTT
GGCAAAATCGCCGACGGCACATTAAGCAGCAAGTTAGCGAAAAAAGCCTTTGAAGCCATG
TGGGCAGAACCCGAAGCCACCATTGCCGAAATCATTGAAAAACACGGTTTGCAACAGATG
ACCGACACCGGCGAGATTGAAGCCATGGTGGACGAAGTGCTGGCAAACAACGCCAAAGCC
GTGGAACAGTTTAAATCCGGCAACGAAAAAGCCCTGAATGCGATTGTGGGACAAGTGATG
AAGGCCAGCAAAGGCAAAGCCAACCCCGCGCAGGTTCAAGAGCTGATTAAAGCCAAACTG
GCTTAATCCGTTATCACACAGGTCGTCTGAAAGCAAAGTTCCAACGAAGGTAAAACAGGA
AATAAGCTTTCAGACGGCCTTTTATAGTGGATTAAATTTAAACCAGTACGGCGTTGCCTC
GCCTTGCCGTACTATTTGTACTGTCTGCGGCTTCGTCGCCTTGTCCTGATTTAAATTTAA
TCCACTATAACTTAATCTGCTCAAACCATACCAAGACATGAACCACACCGTTACCCTGCC
CGACCAAACCACCTTTGCCGCCAACGACGGCGAAACCGTTTTGACCGCTGCCGCCCGTCA
AAACCTCAACCTGCCCCATTCCTGCAAAAGCGGTGTCTGCGGACAATGCAAAGCCGAACT
GGTCAGCGGCGATATTCAAATGGGCGGACACTCGGAACAGGCTTTATCCGAAGCAGAAAA
AGCGCAAGGCAAGATTTTGATGTGCTGCACCACTGCGCAAAGCGATATCAACATCAACAT
CCCCGGCTACAAAGCCGATGCCCTACCCGTCCGCACCCTGCCCGCACGCATCGAAAGTAT
TATTTTCAAACACGATGTCGCCCTCCTGAAACTTGCCCTGCCCAAAGCCCCGCCGTTTGC
CTTCTACGCCGGGCAATACATTGATTTACTGCTGCCGGGCAACGTCAGCCGCAGCTACTC
CATCGCCAATTTACCCGACCAAGAAGGCATTTTGGAACTGCACATCCGCAGGCACGAAAA
CGGTGTCTGCTCGGAAATGATTTTCGGCAGCGAACCCAAAGTCAAAGAAAAAGGCATCGT
CCGCGTTAAAGGCCCGCTCGGTTCGTTTACCTTGCAGGAAGACAGCGGCAAACCCGTCAT
CCTGCTGGCAACCGGCACAGGCTACGCCCCCATCCGCAGCATCCTGCTCGACCTTATCCG
CCAAGGCAGCAACCGCGCCGTCCATTTCTACTGGGGCGCGCGTCATCAGGATGATTTGTA
TGCCCTCGAAGAAGCACAAGGGTTGGCATGCCGTCTGAAAAACGCCTGCTTCACCCCCGT
ATTGTCCCGCCCCGGAGAGGGCTGGCAGGGAAGAAATGGTCACGTACAAGACATCGCGGC
ACAAGACCACCCCGACCTGTCGGAATACGAAGTATTTGCCTGCGGTTCTCCGGCCATGAC
CGAACAAACAAAGAATCTGTTTGTGCAACAGCATAAGCTGCCGGAAAACTTGTTTTTCTC
CGACGCATTCACGCCGTCCGCATCATAATTCCCCGGTATAAAGAGGATTCGAGCTTTCCG
TTCAGAACACAAAAAACTTCCCGTCCGTGTTTTCCCCGTGAAAAAATGCCGTCTGAAACC
CGATTCCGGTTTTCAGACGGCATATGTTTTTTCCTGTTCAAGGCGACAGCCGCTCGCGTA
TCCAGCCACCATCCAGCAAACGGTATTGGATGCGGTCGTGCAGCCTGCTCGGTCTGCCCT
GCCAGAACTCAAGCAAATCGGGAATCACAATATAGCCGCCCCAATGCGGCGGACGCGGCA
CATGCAGAGGATGTTTGAGTCCAACCGCCGCCGCCTTTGCCACCAATACCGCCTTGTTCG
GAATAACCTCGCTCTGCGCACTTGCCCACGCACCCAAACGGCTCTGATACGGGCGACTCT
CAAAATATTCGTCCGACAACTTCTCCGCCAGCCTTTCAACACGCCCTTCCACGCGCACCT
GAGGCTGCAGCTCCGGCCAAAAAAACGTCATCGCCGCAAATGGATGAGCATCCAGCGAAC
GCCCCTTGCGGCTGTGATAATTCGTAAAGAAAACAAAACCTTCAGAATTAACTTCCTTCA
GCAGCACCATACGGCTGTTGGGCCTGCCGCGTCCGTCAACCGCCGCCACATTGACCGCCG
TCGGCTCGTTGACCTGTGCGCGTACCGCCTCGTCCAACCACCGCTCGAACTGCTCGATCG
GATTATCGGCGCAATCGGCTTCCGACAATTCCCGTTTGCTGTAATCTTCCCGAATATTGT
GCAAATCCATTTACTGCTCCTCTTATCATTTGAAAGATTCTACTCCCGCACGCAAACCGA
TTTCAACCGTCGCACAAACTTTGCCCCGACCCCAAGCCGCAGCGACGATTTCATCCGCAA
AACCGCCGCATCAGGTACAATATCGAACCGTCCGACCGAGGACGGCATTTTATCAACCCG
TCCTGCCGCACACGCCGCAGAAGAACCGCCTTATCAGGCGAGTTAGGAAAAATGATGTCC
AAACAGCCCACCAGCAAACGCCAATGGCGCGACGGCGCAGCCCCGTCTGCCAAGAAAACC
GCCAAACCGTTCAAAAGCAAAGCCCGTCCCAAAGATGAAACGGGCAAAACCGCTTCCCAA
CCTTACGGACAAAAAGCTTCAGACGGCATCAAACCTCAAAACGTCCCCAAACAGCGCGCC
GCCAAAGCCAAAAAACTCGTCGTCCGCAATCCCAACCAAAAAATTATGGAACACGCGCGC
GATTTGAAAGAACGCCGCAGCGACCTGTCGCGCATGGAACCCGAACGCCTGCAAAAAGTG
CTTGCCGCGTCCGGCGTCGGCTCGCGCCGCGAAATGGAAGAATGGATTACCAACGGCTGG
ATAACGGTCAACGGCAAAACCGCGCAACTGGGCGACAAAGTTACCCCCGACGACCACGTT
ACCGTCAAAGGCAGCATCATCAAGCTCAAATGGGCGGACCGCCTGCCGCGCATCATCCTG
TATTACAAACAAGAAGGCGAAATCGTTTCCCGTGACGACCCGCAAGGCCGCGTCAGCATA
TTCGACCGCCTGCCGCAGGCCGCCAGCAGCCGCTGGGTCGCCATCGGACGCTTGGACATC
AACACCAGCGGACTTCTGATTCTTACCACCTCCGGCGAACTCGTCCAACGTTTCGCCCAC
CCCAGCTTCGAAGTCGAACGCGAATACGCCGTGCGCGTCTTGGGCGGGCTGACCGGCGAA
CAAATGCGCGTCCTCACCGAAGAAGGCGTGATGCTCGAAGACGGCTTGGCAAAAGTCGAA
CGCATCCGCGAACAAGGCGGCGAAGGCGCGAACAAATGGTACAACGTCGTGATTAAAGAA
GGCCGCAACCGCGAAGTGCGCCGCATTTTTGAAAGCCAAGGACTCACCGTCAGCCGCCTC
GTGCGCATCGGCTTCGGTCCCATCGGACTGCCCAACCGCCTCAAACGCGGGCAGTTCTAC
GAACTCAACCCCGCCGAAGTCGCCAACATCATCAAATGGGCGGACATGCTGCTGCCGGGC
GAACGCCGCCGCAAAAAAGCCTAAACCCGCCAAAACACAAAAATGCCGTCTGAAACATCT

## Appendix A

GCTGTTTCAGACGGCATTTTATTCGGGCGTTTTCAGGAGAAAAGGTCGAGTGCTTTGACA
AAGACCATCACCACGCCGTAGGCGAGCGGTATGACGGCCAATGCCCAACGCCACCAGACC
GATATGCCGCCGCCGGAAACTTTTTCGCCCACAAGGTAAGCGTCGGATATGGCGGTTTCG
TCATCGGGGTTGCCGCTGTGTGCGGCGGTTTTGATGTCTTTTTCGTGGTGTTTTTCGTGT
ACGGATTTGACGGCGAGGTTGCACAACAAACCGATAATCAGCAGGCACGCCATGATGTAC
ATGGTTACGCTGTATGCCTGTGCCGCCGGTATGCCGCTGTCGATTTGGCTTTGGCGTATG
TAATTGACCAGTACCGGGCCGATGACGGCGGCGGTTGACCAGGCCAGCAGGATGCGTCCG
TGAATCGCGCCGACCTGATAGGTGCCGAACAGGTCTTTCAGGTAGGCGGGAATGGCGGCA
AATCCGCCGCCGTACATGGAAATAATCACGCAAAAGCCGATGATGAACAGGGCTTTGCTG
CCGCCCTCGCCGATGGAGGGAACGGCGAAATACAGCAGCGAACCGAGTACGAAGAAGATG
GTGTAGGTGTTTTTGCGTCCGATTTTGTCGGAAACGCTCGACCACAAAAAGCGTCCGCCC
ATGTTAAACAGGCTCAGGAGGCTGACGAAGCCTGCCGCCGCACCTGCGCCGACTGCTGCC
TGCCTGCCTATGGAGGTTTCGGAAAAGAGTTCCTGAATCATCACGGATGCCTGACCCAAT
ACGCCGATGCCGGCAGTTACGTTCAGGCACAATACCCAGAACAACAGCCAAAACTGCGGC
GTTTTCATGGCTTGGGACACGTTGACATGATTGCTGCTGACCAGCTTGTTTTGCGTTTTC
GGCGCGGTATAGCCTTCAGGTTTCCAGCCGTCGGCAGGTACGCGGATGGTAAACGCGCCG
AACATCATCAGTGCGAGGTAAAGCAGACCCAATACGGCGAAGGTTTCGGCAACCCCGACC
GAAGCAGCGTTTGAAAAGGTGTTCATCAGTGATACGGAAAGCGGCGAGGCCAGCATTGCG
CCGCCACCGAAACCCATAATCGCCAAACCGGTCGCCATACCCGGCTTGTCGGGAAACCAT
TTCATCAGTGTGGAAACCGGCCCGATGTAGCCCAAACCCAAGCCTACGCCGCCGATGACG
CCGTTGCCCAAATAGAGCAGGAAGAGGTTGTGCGTACGCACGCCGAATGCGGATACGAAG
AAGCCCAGGCTGAAGCAGCAGGCGGCGGCAAATATGGCTTTGCGCGGCCCTACCCGTTCC
ATCCACGTACCGAACAGGGCGGCCGACGCGCCCAGCATCGCGAGTGCGATACTGAAAATC
CAACCTACGGTCGTCAGCTTCCAATCTCCGGCCGCCGATTCGGTTATGCCGATAAGTTTG
GTCAGCGGCGCGTTGAATACGGAATAGGCGTAAATCTGCCCGATGGCAAGGTGTACCGCC
AATGCTGCGGGCGGTACGAGCCAACGGTTGAAACCCGGCTTGGCAATGCTTGCCTCACGG
TCTAAAAACTTCATAACATCCTCTTTCTGTCAGTTGAAAAATAAAATTTCATTTGCCCAA
TGGAAACTTATTGAAAATTATAAAAAAATATCGGGTCGGGTTTTTATCCGCCCCAAGATG
CGCCGTCTGAAACATTTCGGGTGTACGGAAAGGTTTCTGTTTTTTCCGACAAATTCCTGC
GGCTTTTCGCTTCCGGATTCCCGCTTTTTCAGGAATGACGAATTAAAGATTATCTTAAGG
TCAAGGGACTGGATTCCCGCTTTCGCGGGAATGACGGCGGCGGGGGAGCGGTTTTTCCGA
TTGGGTTTAAATGCAATCGAACAAATCCTGCTGCCCTTGTTCTTTGCTTACGCGCACGTC
GGTTTCGCCGTCGGCGAAGATAATGTGCAGCTTCTGCCCCTGCTTCAAAACATCGGCGTT
GCGGATGACTTGTCCGCGTGTGTTTTTGACGACGGAAAAGCCGCGCTCCAGAATGTGCTG
CGGCGAAACGGCTTCGAGCAATGCGGCTTGGGCAGTCAGGCTTTGGCGGCGGTGGGTAAG
CAGTTGGTGGAAGGCGTGCGACAGGGCCGTCTGAAAGCGGTCGATGTTTTTTTTGCAAAC
GGAAACATCAGGACGGCAATGTTTCAGGGCTTGGGTTTGGCGTTCGAAACGGGCGGTGTG
GGTACGGACGTTTTGCGTCATCGAGTAAGACAGCGTTTGCGCCAGCTTGCCGATTGAAGC
GCGCTGTTCGTCGAGTTTTTGGCGCGGATGACGGATTTGCCGCGCCAGCCAGTCGAGTTT
TTGGCTGGCATCGAAATAGCGTTGTTCCAAAACGGTTTTCAGACGGCATTGGGCTTGGGC
GAGGCGGTGCAGCGATTCTTGGCGGTTGGGGCTGACCAGTTCCGCCGCACCGGTCGGCGT
GGGCGCGCGCATATCGGCGACGAAATCGGCGAGCGTGAAATCGGTTTCGTGGCCTACGCC
GCTGACGACCGGAACCGTGCAGGATTCGATGGCGCGCACGACCGGTTCTTCGTTAAACGC
CCACAAGTCTTCAATGCTGCCGCCGCCGCGACAGACAATCAACACATCGCATTCGGCGCG
TTGCGAGGCGGTTTTAATCGCTTGGGCAATTTGCAATTCGCTGCCTGCGCCTTGAACGGG
TGTCGGATAAACGATAACGGGGATTTCGGGTGCGCGGCGTTTCAAGGTAGTAACGACATC
GCGCAAAGCCGCCGCCGCCAGACTGGTTACGATGCCGATACATTGCGGACGGACGGGCAA
AGGTTTCTTGCGTTCCGCCGCAAACGCGCCTTCCGCCTGCAACTGCGCCTTCAACCGCTC
ATAGGCTTCGTAAAGCTGCCCCAAACCTTTGAGCCGTACTTCGTTTACGGTAATCTGAAA
TTCGCCCCGCGCTTCATAAATACTGATTTTTCCTGATACCTCGATATGGTCGCCTTCTTT
CAAAGGCTTCGCCAAACGCACCGCCGCACCCTTGAACATCGCGCAACGCACCTGTGCGCG
GCTGTCTTTGAGCGAGAAATAATAATGCCCGCTGGCGGCACGGGTCAGGTTGGATACTTC
GCCGGCAATCCACAAACCGGCAAGGTGGTTTTCCAAAAGACTTTTGGCAAATGCGTTCAA
CTCGGAAACGGACAACACGTCAGAATGAAAAAAATCAGACATCGAATCAATCAAATAGTA
AAAAATATGAATATGTTTTGAAGCCTAAGGCGGCACCGGGCCGCCTAAATTGTCAACAAT
ATTATAACACGCGCCATCTTGCCGCCCGCCTTTTCCCGTATGACTTTTTTAAGCGGGGAA
TGGGAAAAATATTCATCAACCTGCCTGCAATCTATTCAAATTGCACCGCCGGCAGGCTAT
GATGCGGATATTTTCGACAGGAGGAAAAATGGATACGCAGGCAGTTATCACACATATCGC
CCGATGGTTGGACGAATACGCCGCCCGGGCAAATGCAAAAGGGTTTGTCGTGGGCGTTTC
CGGCGGCATCGATTCCGCCGTCGTCTCCGCACTCGCCGCCCGCACCGGCCGCCCCACGCT
GCTTCTGGATATGCCGATACGCCAACACCCCGGCCAGCTTGAGCGGGCAAGGCTGCACAT
CCGCAATCTGCAACGGCAATATGCCAATGTAAGCGCGCAAACGGTCGATCTGACCGACAC
CTTCCAGACCTTTGAACAAACCGTCGGTGCTCATCAGACGGCATTTGACAGTCAGCCGCT
TTCCCTCGCCAACGCCAGAAGCCGCCTACGTATGCTGACCCTGTACTACTACGGGCAGAT
ACACGGACTGCTGGTTACGGGGACAGGTAATAAGATTGAAGATTTCGGCGTGGGCTTTTT
TACTAAATACGGCGACGGCGGCGTGGACATCAGCCCGATTGCCGACCTGACCAAAACGCA
GGTTTACCGGCTTGCCGAAGCATTGGGCGTGGACGAGGCGATTCAAAAAGCCCCGCCGAC
CGACGGCCTGTGGGATACGGAACGCACCGACGAAGAACAGATGGGCGCAAGCTATCCCGA
ACTGGAGTGGGCAATGGGCGTGTACGGCACGCGCAAACCCGAAGATTTTGAAGGGCGGCA
GCGCGAAGTTCTAGAAATCTATACGCGACTTCACCGCGCCATGCAGCACAAAATCAACCC
GATTCCCGTATGCCGCATTCCGCCCGAATTGCTGGGCTGAAACACGGAAATGCCGTCTGA
AACGGAAAACCGTATTTCAGACGGCATGGAAATATCCGACTCCTATCCCTTAAGAATCGA
GTACGCGGGCAAACAAAATATCGTTTTCCAAATGAATGTGGTCGTTCAAATCCTCCACCA
TTTCTTTCGCCAGCGCGTAAAGCCGCGTCCAGCTTCCGCAAGCCCCTTCTGGCGGTTGGA
AATTGTCGGTCAGCTCTTTGAGCCGTGCGATGGCGCGGTCGTGTTCTTCGTGTTCGTGCA

## Appendix A

TCATCACGCCGATGGGCATCGCCGCACCGCGTCCGACACCCTGATTAATCATCGGAAACA
GCATCCTTTCCTCTTTCATCATATGCATCAGCAGTTCGTTCTGCATATAGGCAAGCAGCT
CGGCAATTTCCGCCGGAAAGGTGTCGGCATGAACTTGGGCCACTTTTTGCGCCAGCGGCA
CCAATTCTTCAAATTGTGCACGGTGGACATTGTGGTAGCGTTGCAGGATATGATCGACGG
TTGCACCAAAGGGGGCGGTCTCCCAAACGGAAAAATCAGTCATCGCAGTGTTCCTTTTAC
AGGGTTTCGGGTTTGGTTTTGAACATTCATACTTTAAGAATCAATTCAAACGGAGCATAC
ACCGCCCGCGCGCTTCTGTACAGCCTCAAACGTATTCCTTACATTTTGATAATAAAAGTA
ATTTTCAGAAATAAAATACTGTCCGAACCGTTTTTTAGAATTTGCAAAGGCGATTGGGGC
GGTACAGAAAAACTATTATCCCGCCCGCCCACTTGAAATTTTTATGCCCAAGCCCTATCC
TGCACGCTATCGTGCCAATCCCAACCGAAAAGGAAAAATAATGAGCAGCGAATTGATTGT
ACACACCAGCGATGCCGCTTTTGAAAAAGACGTTTTAAATGCAGATATCCCCGTCCTGCT
GGACTTTTGGGCTCCGTGGTGCGGCCCCTGCAAAATGATTGCCCCGATTTTGGACGACAT
TGCCGCCGAATTTGAAGGCCGTCTGAAAGTGGTCAAAATCAACATCGACGACAACGAAGC
CACCCCGTCCCGTTTCGGCGTGCGCGGCATTCCGACCCTGATGGTGTTCAAAAACGGCGA
AGTCGTCGCCACCAAAGTCGGCGCATTGGCAAAAGGTCAGCTGACCGCCTTTGTCGAAGC
CTCTATCGCCTGATAAAGCGCAATCGAAAAAGCCGCCGGAAGATTCCGGCGGCTTTTTCG
CACCCTTAAGATTTGTGGCGGATTTCCCAGCACCTATGGATTTTTTTGTTGCGGAAATCT
TCGGGAACGGATTGTTTGGAAATGTCTTTGACGGCGTATTGTTCCGATACCAAGTCGTCT
AAGACGAAGCTGCGCAGGTTGTTGGAAAAGTACAAAATGCCGTCTGAAGCGAGCAGCTTC
ACCGCGCCGTCAATCAGCTTTTTGTGGTCGCGCTGGATGTCGAGGATGTCGGACATTTTC
TTGCTGTTGGAAAAACTGGGCGGGTCCATCACAATGAGGTCGAACCGCCTGCCTTCCCCA
TATGCCGTCTGAAGATATTGGAACACGTCGGCGCGGACGATTTTGTGTCGTTCCGTATCG
ATGCCGTTCAATTCAAAATTGCGTTTCGCCCAATCAAGATATGTGTTGGACAAATCGACG
GTTTCGCTGGATGCCGCGCCGCCGGTGGCGGCATAGACGGTGAAGCTGCCGGTGTAGGAA
AACAGGTTTAAAAAACGTTTGCCCGCCGCCGTTTCGCCGACTTTTTTGCGCGTGTTTCGA
TGATCCAAAAAAAGCCCCGTATCCAAATACTTATCAAGGTTGACCCAAAACTTGCGGCCG
TTTTCGGTGATGACGAAATCGTCGCCCGCCTTGCCGGTTTTCTCGTACTGCTGCAAACCT
TTTTGGCGTTCGCGGCGTTTGAGGCGGATTTGTTCGGGCGCAAAACCGGTAACGAAAGCG
ACGGCTTCCAAGACTTCGGCAAGCCACGCTTCGTATTCTTCGGGCCGCATCAGCCAGCCG
GTATCGTATTCCTGAAGGTGGATTCGATCGCCGTAAACATCGGCGGCAAAGGGGAATTGG
GGGATGTCGCGGTCGTAAATGCGCCAGGCTTCGATGCCGTTGCGTTTCGCCCATTTCATA
AGGTGTTTGATGTTTTTGCCCAAGCGGTTGGCAAACGGTGTGATGTCGGTCATTGGTTTC
AGGCGGAATAAAGTGGAAAACGGCAATTTTACTGTAATTAACGCCCGATTGCTTGACCGT
TTCGGGCAAACCCTATACCATCCGTCGCTTATCTTGTCATACGAAGCCATCGCCTTCCAA
CCTAAACCGCCCTTACGGGCGCGTTTCTTCTGTTGCTTTGATTTTGCAAAGCATATCTGT
GCAGGTTGCCGTCGATGTAAACCACAAGCAAGCCGCTTGCGACAACCCTGTAACTTCACA
TTCCCCGTATCGTTACCCTTCCCTGCTTCAGGCCGTCTGAACCTTTCGGACGCGGGCGTT
GTTGTCTTCCAAGGATAGCCATGTCTATTAAATTTGCCGATTTGAACCTTGATAAAAACA
TTTTGTCCGCCGTCAGCAGCGAGGGTTACGAAAGCCCGACGCCGATTCAGGCGCAAGCCA
TTCCGTTTGCTTTGGAAGGCCGCGACATCATGGCTTCGGCGCAAACCGGCTCCGGCAAAA
CCGCCGCCTTTCTGTTACCGACTTTGCAAAAACTGACCAAACGCAGCGAAAAACCGGGCA
AAGGCCCGCGTGCTTTGGTGTTGACCCCGACCCGCGAACTGGCGGCTCAAGTCGAGAAAA
ACGCGCTGGCGTATGCCAAAAATATGCGTTGGTTCCGCACCGTCAGCATCGTCGGCGGCG
CGTCTTTCGGCTACCAAACCCGTGCCCTGAGCAAACCGGTCGATCTGATTGTCGCCACGC
CGGGCCGTCTGATGGACCTGATGCAAAGCGGCAAAGTTGATTTTGAACGTTTGGAAGTGC
TGATTTTGGACGAAGCCGACCGTATGTTGGATATGGGCTTTATCGACGACATCGAAACCA
TCGTGGAAGCAACGCCGAGCGACCGTCAGACTTTGTTGTTCTCCGCCACTTGGGACGGCG
CGGTCGGCAAACTGGCGCGCAAACTGACCAAAGACCCTGAAATCATCGAAGTCGAACGCG
TGGACGATCAAGGCAAAATCGAAGAACAACTGCTGTACTGCGACGATATGCGCCACAAAA
~~ACCGCCTGCTCGATCATATCTTGCGCGATGGGAATATGGATCAATGCGTGATTTTCACGT~~
CCACCAAAGCCATGACCGAAGTCATTGCGGATGAACTGTACGAAAAAGGTTTCGCCGCAA
ACTGCCTGCACGGCGATATGCCGCAAGGCTGGCGCAACCGCACGCTGATGGATTTGCGTA
AAGGCCGCTGCAAAATTTTGGTTGCCACCGATGTTGCCGCACGCGGTATCGACGTACCGA
CCATTACCCACGTTATCAACTACGACCTGCCGAAACAGGCGGAAGACTACGTCCACCGCA
TCGGGCGCACCGGCCGCGCAGGCCGCACGGGTATTGCGATTACGTTTGCCGAAGTGAACG
AATACGTCAAAGTCCACAAAATCGAAAAATACATTAACCGAAAACTGCCCGAACTGACCA
TCGAAGGCATGGAACCGACCCGCAAACGCAAATCCGCAGGCGGCAAGCCGAAAGGCAAAG
GCGGCTGGGGCGATCGTAAATCCGGCGGTTGGCGCGGCGATCATAAACCGAGCAAAGAAG
GCTTCGGCGGCAAAACGCGCGGCGAAGGTTTCAAGAAAGAAGGCTTTAAGAGAGACGGTT
TCAAAAAAACCGGCGAAGGCTTCAAAGGCAAACGCAAAGCCGGCGATTCTTTTGCAGGCA
AAGGCGAACGCCGTTACAAAGACCGCTAAGCCCCAACCTGCCGCATAAACCAATGCCGTC
TGAAACCGATTTCGAGTTTCAGACGGCATTTTTGCAATGTTTCAGCACCGCCCGGCTTTG
ATACCCAAAGGATTAGGCTGTAATAAAAACCCTTTTCCGCTTTGGCAACGATTGAAAATT
TCCGTAAATTCAAATATCTAGATTCCTTCCTGCACGGGAATGACACGGAAGGGTTTCAGA
TGCAGGGTGGGCATTCCTGCCCACCCAATCCCGCCCTTGCAACGGTGGGCAAGAATGCTC
GCCCTACGGCTTGACTGTTCGATATGATGCCGTCTGAAAACCCAACGGCGGCATGACAAT
GCCACCCTGCCAACGCACGTAAATCAGAATTGCCATCCCGACATCAAACGCTTGGAAACA
AAATGCCGTCTGAAAATCAAACGGCAACATAACAATGTCCCTAACAAATGCAAAAATGCC
GTCTGAAAGCTCTTCAGACGGCATTGGCGCGCCGGGTTTACCGCCTCCTGCCGAAACCGC
GCATAGCGGGGCGGCGGTAATTGGCGGGCGGGCGCGTTGTCGGGCGGTAACGCTGCGCCT
GCGCCGCCTGTTGTTTTGCACGGAGGCTGCGCGTGTTCAAATCCCTGCTGGTGCGCGCAT
TGGGGCGTGCGGACTGATGGTAGGCTGCACGCGCGCGCCGGGCGACGGGACTGTCTTGGT
TGCCTGCCCGTGTGAATTTGTTTGCCAGCGCGTTGCCGATAAACGCGCCTGCCGCCGCGC
CGACCAGGCTTTGCAGCAGCCAGCTTCCTGTCGATTGGTCGTAAATATACTGCTGCCCGT
CTTTACCGGTAACGGGTTGCCCGTTGTTGCCGTTTGCCTGTGCTTCGGCAGGAATGGTGT

## Appendix A

CTTTGACTGCTTCGGGAGTCAGTTGGTAAACCGTATCGTCTGCCTGCTGTGCGAGCTGCT
GTTGCAGGGCTTCAATCTGTTTCTGCTGCTGTTCGAGCCGCGCCTGCGTGTCGTCTTGGC
AGGCGGCGAGTGCGAATGTTGCGATAAGCGCGGAGGCGATGATTTTTTTCATGTGTGTCC
TGTTTGGGTGGAAAATCGGTTTTATTGTATCGCCGTCGGGAATTTTGGCAAGCATTCTGC
CGGCAAATCGTGATGTTTACAGGGGCAGGGTGTGCAATTTGCGGACAAATGCGAGGCTGT
TGGCGACTGGGTTGCCTTTGTTTTCGACTTCGTGTTCGGTTTCTACGGTCAGCAGGCGGC
GGTTTTTGTGTTTGTTCAGGCTCAATTCGGCTTGTGCGGGTGTGAAAAACAGGCGGTGTT
TTTCGGCAAAGCGGCGGGCGCGGCTGTTGGCGGTTTTCAAAATCTGAAGCAGCGATaCGT
CCAGATGGAAGCGTCGCACGCCCAATACGAGAATCCGTGCGGCAAAAAAATCGGCGGCAT
CGGTAAACTCGGCGGGGCTGCTGCGGCTGAAGTCGTGCCGTTCGGCGGCTATCAGGGCGG
CAACGGTGCGGATTTGCGGAATCATCGATTCGCTGATAAGTGTGTCGTCCCGCCCTGCAT
CGAGAAGCATGGGGGAAAAATCCTGTGCATCATCGACAATAATGCTACAAGTGTGCAGGG
TTTCGTTTTGTGCGGCGGTTTGGGGCATTGCATTCATGGTCATTTTCCTGATTCTGTCGT
GTGTTGCCGAATCGGGCGACCTGTGTGAAGGTAACAAAAAAGCCGCCCCGTTTTCGAGCG
GCCTGTTTTGCGTATGGGATGGATTTCAAGCAAGCGCAAAAAAGTACCGCACGTCTGTGT
GGTACCAATAGCAATAAGCGGTTGTAAATTTTTTGCCTTGCATGATGAAATGCCGTCTGA
AGATAAAAATATTGGGGAGATTCTAAATCAAAACGCTGCCGCGCCTCAAGCATTTTATCG
AAATTTTTTTGATTTTTCATCTATCCGATTGAAAATATTTCGGTTTATTTTTACCGCTGC
CCGATATTGTCGGCAATTTCCCTTTATCTGCTTTGAAAAACGGTGCATAATCCCGAGCAA
AACCGCAATCAGGAGCAATTATGCAAAACTATCTGACCCCCAATTTCGCCTTTGCCCCGA
TGATTCCCGAACGCGCTTCAGGCAGCCGCGTTTGGGATACGAAAGGGCGTGAATATATTG
ATTTTTCAGGCGGTATCGCCGTCAATGCGCTGGGACACTGCCACCCTGCCCTTGTCGATG
CTTTAAACGCGCAGATGCACAAGCTGTGGCACATTTCCAATATCTATACGACGCGTCCAG
CGCAGGAATTGGCGCAAAAATTGGTTGCAAACAGTTTTGCCGACAAGGTTTTTTTCTGCA
ACTCGGGCTCGGAAGCGAATGAGGCGGCGTTGAAGCTGGCGAGGAAATACGCCCGCGACC
GTTTCGGCGGAGGAAAAAGCGAAATCGTCGCCTGTATCAACAGTTTTCACGGACGCACGC
TGTTTACCGTGTCCGTCGGCGGTCAGCCGAAATACAGCAAGGATTATGCACCCCTGCCGC
AAGGCATTACGCACGTTCCGTTCAACGATATTGCCGCGCTGGAAGCTGCCGTCGGCGAAC
AGACCTGCGCGGTCATCATCGAGCCGATACAGGGCGAAAGCGGCATCCTGCCCGCCACTG
CGGAATATTTGCAAACCGCGCGCCGTCTGTGCGACCGGCACAATGCGTTGTTGATTTTGG
ACGAAGTTCAAACCGGGATGGGGCATACGGGCAGGCTGTTTGCCTATGAACATTACGGCA
TTGTTCCCGATATTTTGAGTTCGGCAAAAGCCTTGGGCTGCGGCTTTCCGATCGGCGCGA
TGCTGGCGACAGAAAAGATTGCCGCCGCCTTCCAACCGGGCACGCACGGCTCGACTTTCG
GCGGCAACCCGATGGCGTGTGCGGTCGGCAGCCGCGCATTCGACATCATCAATACGCCCG
AAACTTTAAACCATGTCCGTGAACAGGGGCAGAAACTTCAGACGGCATTGCTGGATTTGT
GCAGGAAAACGGGCTTGTTCTCACAAGTTCGCGGGATGGGGCTGCTACTCGGCTGCGTGT
TGGACGAAGCCTATCGCGGACGCGCATCCGAAATCACCGCCGCCGCCTTGAAACACGGCG
TGATGATTTTGGTTGCGGGTGCGGACGTATTGCGTTTCGCGCCTTCGCTACTGTTGAACG
ATGAGGATATGGCGGAAGGTTTGCGACGTTTGGAACACGCGCTGACGGAATTTGCCGCGA
CATCAGACAATCCGTAAAACTCAAATGCCGTCTGAAGGCGGGAAGGCTTCAGACGGCATC
AGAAACAAAAAACCGCTTCAGAAACGTGGTTCAACGTTCCGAAGCGGTTTTGTTTGCCAT
CAGGACTCGAACACCAATTCCGGTTCCCTGCCCTCTTCGATGACTGCCTTACCGACCACC
ACTTTTTTCAAGCCTTTCAAATCAGGCAGGCGGTACATCGTATCGAGCAGGCAGCGTTCG
ACGATGGACCTCAGGCCGCGCGCCGGTTTTGCGTTCCATTGCCTGACGCGCGATGGAA
CGCAATGCGCCTTCTTCAAACTCCAGTTCGACATTTTCCATACCGAACAACGCCTGATAC
TGCTTGACCAAAGCATTTTTCGGCTCGGTCAAAATATTAATCAGCGCGTCTTCGTCCAAT
TCTTCTAAAGTTGCAATCACAGGCAAACGTCCGATTAATTCTGGAATCAGACCGAATTTA
ATCAAGTCTTCCGGTTCGACGATGCCGAACAGCTTGGTAATGTCGGCATTTTCGTCCTTG
CTGTGAACGGACGCACCGAAACCGATACCGCCTTTTTCAGTACGCTGGCGGATGACTTTT
TCCAAGCCTGCAAACGCGCCGCCGCAGATAAACAGGATGTTGGTGGTATCGACGTTGATA
AATTCCTGATTCGGATGCTTGCGGCCGCCTTGGGGCGGAACGCTGGCCACTGTACCTTCA
ATCAGTTTCAACAAGGCTTGTTGCACACCTTCGCCGGATACGTCGCGGGTAATCGACGGG
TTGTCGCTTTTGCGTGAAATTTTATCGATTTCGTCGATATAGACAATGCCGCGCTGGGCT
TTTTCGACATCGAAATCACATTTGCCCAAAAGCTTGGTAATGATTTGCTCGACGTCTTCG
CCGACATAACCTGCTTCAGTCAAAGTTGTGGCATCCGCCATCACGAACGGCACATCCAGT
TTGCGCGCCAAAGATTGCGCCAGCAGGGTTTTACCCGATCCGGTCGGGCCGATAAGCAGG
ATGTTGGATTTCGACAATTCGACATTAGCTCCTGCTTTAGGATGGCGCAGGCGTTTGTAA
TGGTTGTACACCGACACCGCCAAGGCTTTCTTGGCTTGTTCCTGACCGATAACGTGGTCG
TTGAGGTTGGCGACGATTTCGGCGGGCGTGGGCAGCTTGCCGGATTCTTCCGGCTCCCCT
CCGGCACTTTCCGAAGGCGTGCCGTCATTTCCGTCTTCATGCAATATTTCAATACAGTTT
GAGACGCATTCGTCACAGATAAAGGCGTTTTCGCCCTCAATTAAATGTTTGACGTGTGAT
TTGGATTTTCCGCAAAAGGAACAAGTACGGTTTTCGTTGGACATGGCTTTCTTTACAATG
TATGCGTTACAGAAAACGGCACGTGCCGTTCGGGTTGCCAAGTATAATAACTATATCCGT
TCTTATCAATGTATTACCTTAAAATCCCGCCGATTAGGCTATAATACGCCCTTTCGCAAC
CGCCCCGGCGGCAAAAATGCCGTCTGAAACCAAATCTGAAATCTGAGGATATTCATGAGA
AAACCCCAACGCGGCTATGCCCGCCAAGACCGTGTCAAAGAACAAATTATGCGCGAGCTT
GCCGAACTCGTCCGTACCGGACTGAAAGACCCGCGCGCCGGCTTCATTACCGTCAACGAA
GTCGAAGTTACCCGCGATTACAGCCACGCCACCGTGTTCTACACCATTTTAAACCAAGAC
GCGCGCGAAATTACGGAAGAAGTGCTGGAACACGCGCGCGGACACCTCCGCAGCGAATTG
GCCAAACGCATCAAGCTGTTCAAAACGCCCGAACTGCATTTCAAATACGACGAATCTTTG
GAACGCGGTTTGAACCTGTCCGCCCTTATCGACCAAGTAGCGGCGGAAAAACCGGTTGAA
GACTGACGGATATGCCCATGCCGTCCGAACATCGAACCATGAATACAGGCAAACCCCAAA
AACGTGCCGTCAACGGTGTTTTGCTCTTGGACAAACCCGAAGGCCTTTCCAGCAACACCG
CGCTGCAAAAAGCGCGGCGTTTGTTTCATGCCGAAAAAGCCGGACATACCGGCGTGCTCG
ACCCTTTGGCAACCGGACTTTTGCCCGTCTGCTTCGGTGAAGCGACCAAGTTCGCCCAAT

## Appendix A

ACCTGCTGGATGCCGACAAAGCCTACACCGCCACGCTGAAACTCGGCGAAGCCAGCAGCA
CGGGTGATGCCGAAGGCGAAATCATTGCCACCGCCCGCGCCGATATTTCCTTAGCCGAAT
TTCAGACGGCCTGCCAAGCACTGACAGGCAACATCCGCCAAGTGCCGCCAATGTTTTCCG
CGCTCAAGCACGAAGGCAAACCGCTGTACGAATACGCCCGCAAAGGCATCGTCATCGAAC
GCAAAGCGCGCTACATTACCGTTTACGCCATCGATATTGCCGAATTTGACGCGCCCAAAG
CCGTCATCGACGTACGTTGCAGCAAAGGCACCTACATCCGCACCCTCAGCGAAGACATCG
CCAAACACATCGGCACGTTCGCCCACCTGACCGCCCTGCGCCGCACTGAAACCGCCGGCT
TTACCATCGCCCAAAGCCACACGCTTGAGGCCTTGGCAAATTTGAACGAAACAGAACGCG
ACAGCTTGCTGCTACCCTGCGACGTATTGGTTTCACACTTTCCCCAAACCGTTTTAAACG
ATTATGCCGTCCATATGCTCCACTGCGGACAACGTCCGCGTTTCGAAGAAGACCTGCCTT
CCGACACGCCGGTACGCGTTTACACGGAAAACGGCCGCTTTGTCGGTCTGGCGGAATATC
AAAAAGAAATATGCCGTCTGAAAGCCTTGCGCCTGATGAACACGGCGGCATCCGCCGCCT
GAACGGCGGTTAAAAATACAGGCTGTGCTTGAATAATGTGTTGATATTTCCGCAAAATCC
CGACACACTCGGACACCCGCCCCGCTTATCGCAACTTTGCGAACGCCCCCGGAAACAGCA
AAGACATCAAATAATTGATTTTATTAGAATCTATTTGCAAAGCCATTTGCCGTTACACAA
GAATGGCACATTAAAATAACTGATGAGGATTTATAACGATGAAGACAGACATTCAAACCG
AATTAACCCAAGCCCTACTACCACACGAAAAAGTATGGGCGAACGAAGAAAAAACCATTT
TAGCCAAAAACATCCTGTTGGATTTGGTGGAAAAAACCGACCCGACCATTATCGGTTTGT
TATTGAGTAATGATGAGTTAAAACGCCATTTCTTTGTGGAAGTGAATGGTGTGCTGGTGT
TTAAATTGCAGGATTTCCGTTTTTTCTTGGACAAACACAGCGTCAATAATTCCTACACAA
AATACGCCAACCGCATTGGTTTGACGGACAGCAACCGCTTTTTGAAAGACAGCAGTGATA
TTGTGTTGGATTTTCCGTTTAAAGATTGTGTGTTAAATGGCGGACAAAGCACCGAGGAAG
GCGAAGAAATTTATTTTAAACGCAATAACAGCCAGCCAGCCAGCCAGCCAGCCAGCCAGC
CAGCCAGCCAGCCAGCCAGCCAGCCAGCCAGCCAGCCAGCCAGCCAGCCAATTATACACT
AAATTAACCCGAAAAAGACAAGAAATCTTTTTTAATCAAACCCTTGCTTTTGATGAAATT
GATCGGCTTTTTGACGCAAAAGCATTCTCAAAATTCTCTCGCTATACCGCAGACGGCAAA
CAAGCCGTTGGCGAAATCAAACGACATTCAGACGGCACACCCGCCGAAAATCTCATTATC
AAAGGCAATAATCTGATTGCCCTGCATTCGCTTGCCAAGCAGTTTAAAGGCAAAGTGAAG
CTGATTTATATTGACCCGCCATATAACACGGGTAATGACGGTTTTAAATACAACGACAAA
TTTAATCATTCCACTTGGCTGACTTTTATGAAAAACCGTCTAGAAATCGCCAAAGAGCTG
CTTATGAAAGACGGTTCGATTTTTGTGTCAATTGACGACAACGAACAGGCATATTTGAAA
ATTTTAATGGATGAAGTTTTCGGAAATGAAAATTTCATCTGCAATTTTATTTGGAAAAAA
AAGACAGGTGCGTCCGATGCCAAACAGATAGCGACTATTACAGAGTTTGTCTTATGTTAC
TCAAAGAACTTTAAAACAGTTAAATTAAATAAAAACACGTTTTCTTATGATACAGAGAGA
TACAAATTAAGTGATAAGTTTGAACAGGAAAGAGGCAAATATTATATCGACAATTTAGAT
AGAGGGGGATTGCAGTATAGTGACAGTTTGAATTTTGCAATCCAATGTCCAGATGGCACT
TTTACGTATCCGAATGGCAGGACTGAATTTGTCAATGATGGCTGGATATGGAAATGGAGT
AAAAATAAAATTGATTGGGCAATAACAAACGGTTTTTTGGAGTTTAGAAAATCAAAGTCT
AAAAAATCTGGATGGAGTGTATGTTATAAAAACTATATGTTGGTTGATAACGAAAACAAG
CCGATAGAACGTTCTGCTCCCTATAAGAACTTAATACAGGATATCTTAAATACACATGCG
ACAGATGAATTGAAAAAACTGTTCGGCAGCAAAGTTTTTACTACTCCAAAACCTGAGAGC
TTATTGCAGTATCTTATTCAAATTGCCACATCCGAATCCGACATCGTCTTAGACTACCAT
CTTGGTAGTGGCACAACCGCCGCCGTTGCCCACAAAATGAACCGCCAATATATCGGTATT
GAACAAATGGATTATATTGAAACGCTTGCTGTTGAACGCTTGAAAAAAGTGATTGATGGC
GAGCAAGGCGGTATTTCCAAAGCCGTGAATTGGCAAGGTGGTGGCGAGTTTGTTTATGCC
GAACTTGCCCCATTTAACGAAACCGCAAAACAACAAATTTTGGCTTGCGAAGATTCAGAC
GGCATCAAAACGCTGTTTGAAGGTTTATGCGAACGCTATTTCTTGAAATACAACGTCAGC
GTAAATGAATTTAGTCAAATCATTCAAGAGCCTGAATTTCAATCTTTGCCATTAGACGAA
CAAAAACAAATGGTGCTTGAAATGTTGGATTTAAATCAAATGTATGTTTCATTATCCGAA
ATGGATGACGAACAATTTGCAGATTGCCTGAACGATGATGATAAAGCCTTAAGCCGTGCA
TTCTATCAATCAGTAAAAAATCAAGCGGAGAAAAAAGATGGCGAATAATAAAACGTTGTT
TGAAGTGATTGAAAATGAACGTAAAGCGGTTAAAAAATACAAGCCTGAATTACTTGAAAT
GCCAGAATTTACGTCCAAAAACTTAAAATATGATTTTTTTGAATGGCAAAAATCTGCCCT
TGAAAACTTTTTGATTTTTGACCGCACTTCAAAGCTAGACGATTTCCCTGATTTAAAAAA
TAAGCCAACGCATTTGCTGTTCAATATGGCAACAGGTGCTGGCAAAACGATGATGATGGC
GGCGTTGATTTTGTATTATTTTGAAAAAGGTTATCGGCATTTTCTGTTTTTTGTGAATCA
AAACAATATCGTGGATAAAACGGAAAATAATTTTACCGATCCGACGCACGCAAAATTTTT
ATTTACCGAGAAGATTTTGCAGGGCGATACGGTAATTCCTATTCGCAAAGTGGAGACATT
TAGCCCACATTCAGACGGCATTGAAATTAAATTTACCAGCATTCAAAAGCTGTATAACGA
TATTCGCACCCGGCGGGAAAATCAAACCACATTGGCGGATTTGCACAAATTGAACCTTGT
GATGCTGGGTGATGAAGCGCACCATTTAAACGCGCAAACCAAAGGCAAAAAACAAGGCGA
ATTAGATTTAGAAAAGGAAATGAACGACCGCACCAGCAATGCCGAAATTGAACGTAAAGG
CTGGGAGCATATGGTTTTGGAATTGTTACTCAATAAAAATGGCAATCATAGCCAAAATGT
GCTGTTGGAATTTACCGCCACGCTGCCTGAAAATGCCGATGTACAACAAAAATACGCTGA
TAAAATCATCACAAAATTTGGCTTAAAAGAATTTTTGCAAAAAGGTTATACCAAAGAAAT
CAATTTGGTATCCAGTACGCTGGGTAAGAAAGAGCGAGTGTTACACGCTTTATTGTTTGC
TTGGTATCGACATCGAATTGCGTTGAAATATGGCATTGCCAATTTCAAGCCTGTGATGTT
GTTTAGAAGTAAGACGATTGATGAATCAAAAGCGGATTATCTGGCATTTTTAAATTGGGC
AGAAAATGTGCAGGCGGTTGATTTTTCGTTTTTAACTACATTTTCAACAAGCTTGAACGA
TAGCGATAGCGATAACGCCAACGAACAAGGCAAAACCCGCACTGAACAAGCCCTAAAATT
TATGCAGGAAAAAGGCGTTGAGTTTGCACATTTGGCAGATTGGGTAAAACAAAATTATCA
AAAACACAATGTGATTATTACCAACTCCGAAACCAACAAAACCAAAACCGAAAAAACCGA
CAGCGAAACAGAAAAATTGCTGAATAATTTGGAAGCGGCTGATAATCCGATTCGTGCCAT
TTTTACGGTGGACAGATTAACCGAAGGTTGGGACGTTCTGAATTTGTTTGATATTGTGCG
TTTGTATGAAGGGCAAAACGGCGGCGGTTCAAATAAAAAATCAGGCAAAACGGCTGCCGC

# Appendix A

TACTGTATCGGAAAAGCAGTTGATTGGTCGCGGCGTGCGTTATTTTCCATTTGCGTTTGA
AGGTAAACAGCCGAATAAACGCAAATTTGACAACGATATGCAACACGAATTGCGTATTTT
GGAAGAATTGTTTTATTACACGCACGATGAGCAATCTCGCTATATTACAGAACTGAAAAA
CGAGTTACGAAAAGACGGTTATTTGCCTGAAAAAGACGATGATAAGGTATTGGCAACATT
TAAACTCAAATCTGAATTTGCCGATAATCAGGATTTTAGAGAGTTGTTAATTTGGGCAAA
TAAAAAAATCCCCAATCCCAATGCCAGAGCCAATAATGCAGACAGCCTGAAAGCCAATCC
GCAAACGCTTCCATTCCAAGTTCACGGCAATCAACTGTTGCAGGAAACGCAATTTACAGC
CGATGAAAATGATGAAATAGCCCGACAAATCGACACACAAAATAATTTTACTCAAATCAT
AAAAATGAGTGAAATGGAACGGCACATTTTCAATAAATCCCTGCATATCAAAGGAAAAAA
TGGTCAATCTTTATTCCATTTTGACCGCTTGCAAAGCAAACTCAACATTTACAATCGCAA
TGAATTGCAAAATAACTTGTTAAAAGATTGACAAATTGAATTTTTGGGATTAGGGCAAGA
CAAACAGATCAGCCCAGATGACAAACTTGCAGGCTGCCTAAAAATCTTGGAAATGGTTGA
AAAACATTTGAATGAAAGTGATATGCCATTTATCGGTACAAAAGAATTTACGCCTAAAAA
ATTGTGGGAAATTTTTGGCACACCAAAACAAAAATGGGTCAAAAAAGATGATATAAAAAC
TGCCATTGCCACGCAAAATGATTGGTATGTGATGGATAATTTTGCTGGAACGAGTTTGGA
AGAAGCGTTAATTCAATTTATTTCAGAGCATTTGGGCGATTTGAAGTCTAAATATGATGT
TCATTTAATCCGTAATGAAGAAGTGTTTAAATTGAATAACTTTTCCGATGGTGAAGGATT
TATGCCGGACTTTATTTTATTGCTGAAAAATAAACAAAAATCTTCTTCCAATGGTGTGGA
TGACTTTTTGCATTACCAAATTTTCATTGAACCAAAAGGTGAGCATTTGGTGGAAAATGA
TTCGTGGAAAGACGCTTTTTTAAAGGCAATTACAGCGGAATACGGGACGGATAAGATTCT
GCAAAAAGATACACCGCATTATCGTTTGATCGGTTTGCCGTTTTTTACTGACAATCAGGA
AAATGAACAATTTACAAAGTCATTCCCTTTAGGGGCGGCATCGCTTGAAAAATAGAGTGG
TGCATTGCAGGCAACCCCGTTTGACAAAACTTCCTTTACAAAAGGGCGTTTTGTCAGATA
TTTAATCAACACATTATTAAAATACAGCCAAATTTTAATGCCGTCCGAACCCTGTGTTCA
GACGGCATCGTATTTTTCAGTATCTAAACCGTTTCCCTGCCCCAATCTTTGCCTCTCAAA
ATCGAAGCATCGACATCTTGAATATCGCGGTGTCCCGTAAACGCCATAGATATATCCATT
TCTTTATACAGGATTTCCAGCGCACGGGTTACGCCTTCTTCTCCATACGCGCCCAAGCCA
TACAGGAACGCCCGACCTATCATTGTACCTTTCGCGCCCAAAGCCCACGCCTTCAAAATA
TCCTGACCGCTGCGGATGCCGCTGTCCATCCAAACTTCGATGTCGCTGCCCACTGCGCTG
ACGATGTCGGGCAAGGCTTTGATGGCAGACACGGTATCGTCGAGCTGTCGACCGCCGTGG
TTGGAAACAATCAATGCGTCCGCGCCGCTTTTCGCTGCTTTTTCCGCGTCTTCAGGTTCC
ATAATGCCTTTGATAATCAGCTTGCCGCCCCACAAATCTTTAATGCGCGCCACATCGTCC
CAGCTCAGGCGCGGGTCGAATTGTTCGGAAGTCCATGAAGACAGCGAAGACAAATCGCCG
ACGTTCTTCGCGTGTCCGACGATATTGCGGAACGTGCGGCGTTCCGTGTTCAGCATTTTC
ATACACCATTCGGGCTTGGTCGCCAGATTGATTAAATTGGCGATGGTCGGTTTCGGCGGC
GCGGACAGGCCGTTTTTGATGTCTTTGTGGCGTTGCCCCAAAACCTGCAAATCAGCGGTC
AATACCAATGCCGAACATTTGGCATCCTTCGCGCGCTTAATCAGGTTTTCCATAAACTCG
CGGTCGCGCATCACATAAAGCTGAAACCAAAATGGTGCGGAAGTGTTCTCGGCAACGTCT
TCAATCGAGCAGATAGACATCGTGGACAGCGTAAACGGAATGCCGAACTTCTCCGCCGCC
CGCGCCGCCAAAATTTCACCGTCGGCGTGTGCCATACCCGTGAAACCCGTCGGCGCAATC
GCCACCGGCATTTTCACATCCTGCCCGATCATTTTGGTTTCCAGGCTTCGGCCTTCCATA
TTGACCAATACTTTTTGACGGAAGCGGATGTCTTTGAAATCCGAAGTGTTTTCACGGTAG
GTAGTTTCTGTCCACGAACCCGAATCGATGTAATCGTAAAACATACGCGGCATTTTGCGC
TTGGCAACGCGGCGCAAGTCTTCGATGCAGGTCATTTTGCTCAAATCACGTTTCATTTGT
CGCCCCCTGAATACCTGAATAACTTTATATGAAATCGATAATGTATATCAATATTGATTA
TAAGGCAAATCATTTCAACATTTGCCGCATCCGCCGCAGCTCCCTACTTTAAGCGACATA
AGGTTTAAAATTCAAAAATAACAAATTAAAATCAAAATATTAAAAATCAATCAATCTATC
GATTTAAACAGCCAATCACACAATCCGCCCTCATACTTGACTGAAACACTCAGATATTGG
ACAATTCCACCCACTAATAAAAAAACCGACATGGGCAACCACCACCATGAGACTGACCAC
CAAAGGGCGTTTCGCCGTTACCGCTATGCTGGATTTGGCGATGAACGCGCAAACCGGCGC
CGTCAAACTCAGTGCCATCAGCGAACGCCAAAACATATCCCTCTCCTATCTCGAGCAATT
GTTCGGCAAACTCCGCCGCGCCGGACTTGTTGAAAGCCTGCGCGGGCCCGGCGGCGGCTA
CATCCTCGCCGCACCGGCGGCACGCATCAACATCGCCCAAATCATCGCCGCCGCCGAAGA
CCGGCTGGACGCAACCCAATGCGGCAGCAAAGCCAACTGCCACCACGGCGCGCCCTGCCT
GACGCACGATCTTTGGGAGAATTTAAACAAAACCATCAACGACTACCTCGGCAGCGTTAC
CCTGCAAAGCATCATCGAACAGAAAAACAACGGCGACGGCAGCCGCGTCGTCCAATTTAC
ACACATCCATTAAATAACACCCGAAAAAGAAAGAGCAAACCATGACCGTCAAAACCCCCG
TTTACCTCGACTACGCCGCCACCCCCCCCGTTGACAAACGCGTTGCCGAAAAAATGATTC
CCTATCTGACCGAAACCTTCGGCAACCCAGCCTCCAACAGCCACAGCTTCGGCTGGGAAG
CAGAAGAAGCTGTAGAAAAAGCACGTGCAGACATTGCCGCCCTGATTAACGCCGACTCTA
AAGAAATCGTTTTCACCAGCGGCGCAACCGAGTCCAACAACCTCGCTATCAAAGGCGCGG
CGCACTTCTACAAATCTAAAGGTAATCACCTCATCACTGTAAAAACCGAACACAAAGCCG
TACTCGACACCATGCGCGAACTCGAACGCCAAGGTTACGAAGTAACTTATCTGGACGTAC
AAGAAAACGGTTTGGTTGATTTAGACGTACTGAAAGCCGCCATCCGCGAAGACACCATCC
TCGTTTCCGTAATGTGGGTAAACAACGAAATCGGCGTGGTTCAAGATATTCCTGCCATCG
GCGAAATCTGCCGCGAACGCAAAATCATTTTCCACGTTGACGCAGCACAAGCATGCGGCA
AAGTGCCTGTTGATGTTGAAGCCGCAAAAGTTGATTTGCTGTCTATGTCCGGCCACAAAG
TATACGGCCCTAAAGGCATCGGCGCCCTGTATGTACGCCGTAAACCACGCGTCCGCCTCG
AAGCCCAAATGCACGGCGGCGGTCACGAACGCGGTTTCCGTTCCGGCACATTGCCGACCC
ATCAAATCGTCGGCATGGGTGAAGCCTTCCGCATTGCCAAAGAAGAATTGGCACAAGACA
CTGCACACTACCTGAAACTGCGCGATATTTTCCTCAAAGGTATCGAAGGCATCGAAGAAG
TCTATATCAACGGCGACCTCGAACATCGCGTCCCGAACAACCTAAACGTCAGCTTCAACT
TCGTCGAAGGCGAAAGCCTGATTATGGCAGTGAAAGAACTCGCCGTATCCAGCGGCTCCG
CCTGCACCTCCGCCTCGCTCGAACCCAGCTACGTCCTGCGCGCGCTCGGCCGCAACGATG
AACTGGCGCACTCATCCCTGCGCATCACCTTCGGTCGCATGACCACCGAAGAAGAAGTGC

## Appendix A

AATTCGCCGCCGAACTGATTAAATCCAAAATCGGCAAACTGCGCGAACTGTCGCCGCTGT
GGGAAATGTTCAAAGACGGGATTGATTTGAACTCGATTGAATGGGCAGCGCATTAAAGCG
TACCAACATGCCGTCCGAACCTTTAGACGGCATTCCAAAAACAAAGCAATCAAGAGAAAA
TATGAACGAACAAGATTTAGATTTGGACAATCTCGACAACCTGCTTGAAGATTTTGACGG
CGTTACCGTGGAAGGCGGCGTCGATTCGGAAAACGACGACGGCTGCGAAGGCGGGGCGTG
CAAAATCTAAACTTCAGGCCGTCTGAAAATCGGCAAACAAACCACTTAAACCATCAAAAA
ACATTAAGGAAACCACATCATGGCATACAGCGATAAAGTAATCGACCACTATGAAAATCC
GCGCAACGTCGGCACATTCGACAAGGGAGACGATTCCGTCGGCACCGGCATGGTCGGCGC
GCCCGCCTGCGGCGACGTCATGCGCCTGCAAATCAAAGTGAACGACGAGGGCATCATCGA
AGATGCGAAATTTAAAACTTACGGCTGCGGCTCGGCCATCGCTTCGTCCAGCCTGATTAC
CGAGTGGGTTAAAGGCAAAAGCCTGGATGACGCGCTGGCAATCAAAAACAGCGAAATCGC
CGAGGAGTTGGAATTGCCGCCGGTAAAAATCCACTGCTCCATCTTGGCTGAAGATGCGGT
AAAAGCGGCCGTTGCCGACTACCGCAAACGTCAGGAAAACAGATAAAGCCCTTCAGACGG
CATCATCCCGCAATGCCGTCCGAACCACCCGCCGCTTCAGGTCCGTCCGGGGCGGTACAA
CAAGGAAGAAATATGATTACCCTTACCGAGAATGCCGCAAAACACATCAATGACTATCTC
GCCAAACGCGGCAAAGGCTTGGGCGTACGCTTGGGTGTGAAAACCAGCGGCTGCTCGGGG
ATGGCGTACAACCTTGAATTTGTCGACGAAGCCGATGGCGACGACCTGATTTTCGAAGGA
CACGGCGCGCGCATTTATATCGATCCGAAAAGCCTGGTTTATCTGGATGGCACGCAAGTC
GATTACACCAAAGAAGGTTTGCAGGAAGGTTTCAAATTTGAAAACCCCAATGTCAAAGAC
TCCTGCGGCTGCGGCGAAAGCTTCCACGTTTAAGGCATAAAAACGGCGGGACCGTATCAA
AACCGTCCCGCCATTTTTTCGCTTCCTGCCTGTTGTAGCTGCCTTTGCCTTTCCTTTTCC
GTTCCACCTTGTGCCGGAACAAATCGGATTTCACTAAGGCTTTTAAAGCATTGTCGCGTA
TTTTGCCTTTATTGTGCTGCACTTTGCCGCCCATATTCAGTCCTTTCGTTTAAGAAGCGG
CAGATTATAAGGCAAAAACAGTTTTCTGCCAAAATCTTACATTTATCATCCTACTATGTC
CCAATATTTCACCCTCTTCCGGATTGAACCCGCTTTCGATATCGACACCGAAAACTTGGA
ACAAACCTACCGCGCCTTGGCCGCCCGTTTCCATCCCGATAAATTCGCTTCAGCTTCCGC
CTTTGAGCAAAAGCAGGCAGTGATGATGTCTTCCACCATCAACGATGCCTACCGCACCTT
GAAAAACCCCATCGACCGCGCCGCCTACCTGCTGAAAACATCGGGCATCGATGCCGACGC
GCCGGAGCATACCGCTTTCGCCCCCGAATTCCTTATGCAGCAAATGGAATGGCGCGAAAC
GCTGATGGAGGCACGGGCAGGCAACGACCTTGAATCCTTGAAAAATCTCGACAACGAAAT
CCGCGACGAACAAGAAAAACTGTTCTGCGGTCTGAAACAGTCGTTTGCCCGACAAGATTA
CGACACAGCCGCACAACAAGTCCGCCAAGGCAGGTTTCTCGACAAACTCCGCAACGAAAT
TTCCTCGGCATTATAATCCGCACCGTGTTTCAGACGGCGTAACCGCCGCACCGTTCCGCG
TCAAAATATGCTAAAATAAGCAACAATTTTTTGCCATACGAAACATTGAAACCATGACCG
ACGCAACCATCCGCCACGACCACAAATTCGCCCTCGAAACCCTGCCGGTAAGCCTTGAAG
ACGAAATGCGCAAAAGCTATCTCGACTACGCCATGAGCGTCATTGTCGGGCGCGCGCTGC
CGGACGTTCGCGACGGTCTCAAGCCGGTACACCGCCGCGTACTGTACGCGATGCACGAGC
TGAAAAACAACTGGAATGCCGCCTACAAAAAATCGGCGCGCATTGTCGGCGACGTCATCG
GTAAATACCACCCCCACGGCGATACCGCCGTATACGACACCATCGTCCGTATGGCGCAAA
ATTTCGCTATGCGTTATGTGCTGATAGACGGACAGGGCAACTTCGGATCGGTGGACGGGC
TTGCCGCCGCAGCCATGCGCTACACCGAAATCCGCATGGCGAAAATTTCCCACGAAATGC
TGGCAGACATTGAGGAAGAAACCGTCAATTTCGGCCCGAACTACGACGGTAGCGAACACG
AGCCGCTTGTACTGCCGACCCGTTTCCCCACACTGCTCGTCAACGGCTCGTCCGGCATCG
CCGTCGGCATGGCGACCAATATCCCGCCGCACAACCTTTCTGATACCGTCAATGCCTGCC
TGCGCCTGCTCGATGCACCCGACACCGAAATCGACGAACTGATCGACATTATCCAAGCCC
CCGACTTCCCGACCGGGGCAACCATCTACGGCTTGAGCGGCGTGCGCGAAGGCTATAAAA
CAGGCCGCGGCCGCGTCGTTATGCGCGGTAAGACCCATATCGAACCCATAGGCAGAAACG
GCGAACGCGAAGCCATCGTTATCGACGAAATCCCCTATCAGGTCAACAAAGCCAAGCTGG
TCGAGAAAATCGGCGATTTGGTTCGGGAAAAAACACTGGAAGGCATTTCCGAGCTCCGCG
ACGAATCCGACAAATCCGGTATGCGCGTCGTTATCGAGCTGAAACGCAACGAAAATGCCG
AAGTCGTCTTAAACCAACTCTACAAACTGACTCCGCTGCAAGACAGTTTCGGCATCAATA
TGGTGGTTTTGGTCGACGGACAACCGCGCCTGTTGAACCTGAAACAGATTCTCTCCGAAT
TCCTGCGCCACCGCCGCGAAGTCGTTACCCGACGTACGCTTTTCCGGCTGAAGAAGGCAC
GCCATGAAGGGCATATTGCCGAAGGCAAAGCCGTCGCACTGTCCAATATCGATGAAATCA
TCAAGCTCATCAAAGAATCGCCCAACGCAGCCGAGGCCAAAGACAAACTGCTTGCGCGCC
CTTGGCGCAGCAGCCTCGTTGAAGAAATGCTGACGCGTTCCGGTCTGGATTTGGAAATGA
TGCGTCCGGAAGGATTGGCTGCAAACATCGGCTTGAAAGAGCAAGGTTATTACCTGAGCG
AGATTCAGGCAGATGCTATTTTACGCATGAGCCTGCGAAACCTGACCGGCCTCGATCAAG
AAGAAATTGTCGAAAGCTACAAAAACCTGATGGGTAAAATCATCGACTTTGTGGATATCC
TCTCCAAACCCGAACGCATTACCCAAATCATCCGCGACGAACTGGAAGAAATCAAAACCA
ACTATGGCGACGAACGCCGCAGCGAAATCAACCCGTTCGGCGGCGACATTGCCGATGAAG
ACCTGATTCCGCAACGCGAAATGGTCGTTACCCTGACACATGGCGGCTATATCAAAACCC
AGCCGACCACCGACTATCAGGCGCAGCGTCGCGGCGGGCGCGGCAAACAGGCGGCTGCCA
CCAAAGACGAAGACTTTATCGAAACCCTGTTTGTTGCCAACACGCATGATTATTTGATGT
GCTTTACCAATTTGGGCAAGTGTCATTGGATTAAGGTTTACAAACTGCCCGAAGGCGGAC
GCAACAGCCGCGGCCGTCCGATTAACAACGTCATCCAGTTGGAAGAAGGCGAAAAAGTCA
GCGCGATTCTGGCAGTACGCGAGTTCCCCGAAGACCAATACGTCTTCTTCGCCACCGCGC
AGGGAATGGTGAAAAAAGTCCAACTTTCCGCCTTTAAAAACGTCCGCGCCCAAGGCATTA
AAGCCATCGCGCTCAAAGAAGGCGACTACCTCGTCGGCGCTGCGCAAACAGGCGGTGCGG
ACGACATCATGCTGTTCTCCAACTTAGGTAAAGCCATCCGCTTCAACGAATACTGGGAAA
AATCCGGCAACGACGAAGCGGAAGATGCCGACATCGAAACCGAAATTTCAGACGGCATCG
AAGATGAAACCGCCGACAGCGAAAACGCACTGCCGAGCGGCAAACACGGTGTTCGCCCGT
CCGGTCGCGGCAGCGGCGGTTTGCGCGGTATGCGCCTGCCTGCCGACGGCAAAATCGTCA
GCCTGATTACCTTCGCCCCTGAAACCGAAGAAAGCGGTTTGCAAGTTTTAACCGCCACCG
CCAACGGATACGGAAAACGCACCCCGATTGCCGATTACAGCCGCAAAAACAAAGGCGGGC

## Appendix A

AAGGCAATATTGCCATTAACACTGGCGAGCGAAACGGCGATTTGGTCGCCGCAACCTTGG
TCGGCGAAACCGACGATTTGATGCTGATTACCAGCGGCGGCGTACTTATCCGCACCAAAG
TCGAACAAATCCGCGAAACCGGCCGCGCCGCAGCAGGCGTGAAACTGATTAACTTGGACG
AAGGCGAAACCTTGGTATCGCTGGAACGTGTTGCCGAAGACGAATCCGAACTCTCCGACG
CTTCTGTAATTTCCAATGTAACCGAACCGGAAGTCGAGAACTGAAAATCATCTCCCGATG
CCGTCTGAAGATTCAGACGGCATTTATTTTATCCCTCATCCGTCATCCAGCTTCTCACAA
TATAGCGGATTATAGTCAATTAAAAACAAGGGGCTGTCCTAGATAACTAGGGAAATTCAA
ATTAAGTTAGAGTTGCCCCTATGAGAAAAAGTCGTCTAAGCCGGTATAAACAAATAAACT
CATTGAACTGTTTGTCGCAGGTGTAACTGCAAGAACGACAGCAGAGTTAGTAGGCGTTAA
TAAAAGTACCGCAGCCTATTATTTTCATCGTTTACGATTACTTATTTATCAAAACAGTCC
GCATTTGGAAATGTTTGATGGCGAAGTAGAAGCAGATGAAAGTTATTTTGCTGAACGACA
AAACCATATCAATGGAATTGAGAACTTTTGGAACCGGGCAAAACGTCATTTACGCAAGTT
TGACGGCATTCCCAAAGCGCATTTTGAGCTGTATTTAAAGGGGTACGAACGGCGTTTTAA
CAACAGTGAGATAAAAGTTCAAATTTCCATTTTAAAACAATTAGTAAAATCGAGTTTATC
CTAGTTATCTAGGACAGCCCCAAAAACAAAATAGTACAATATTCAACTTTGAAGGTCTAA
CCATGGCATACTCTGCGGACTTAAGAAACAAAGCTTTAAACTATAGTGGATTAAATTTAA
ATCAGGACAAGGCGACGAAGCCGCAGACAGTACAAATAGTACGGCAAGGCGAGGCAACAC
CGTACTGGTTTAAATTTAATCCACTATATTACGAACAATGCAAAAACATCAGCCAAACCG
CAGCAACGTTTAACTTGTCAAGAAACACGCTTTACCTGTGGATTCGCCTTAAAAAACAAA
CAGGCAGCCTAAAACATCAAGTTACCGGTCTAAATGCCGTCAAATCGGATAGGCAAAAAC
CGGCTCAATATGTTGGGCAACACCCGGATGCCTATCTGCATGAAATCGCCAAACATTTTG
ATTGTACGGCAGCCACCATTTGCTATGCACTCAAACAGATGGGGATAACGCGCAAAAAAA
GACCACCACTTACAAAGAACAAGACCCGGCCAAAGTAACGCATTATTTGACACAGCCGGC
CGAATTTTCCGACTACCAACGTGTTTATTTGGATGAAACAGGATTTGACCGCTACCTGTT
CCGTCCCTATGCCCGCAGCCTGAAAGGGCAAATAGTGAAAGCGCAGATAAGTGGAAAAAG
ATATAGTGGATTAACAAAAATCAGGACAAGGCGACGAAGCCGCAGACAGTACAAATAGTA
CGGAACCGATTCACTTGGTGCTTCAGCACCTTAGAGAATCGTTCTCTTTGAGCTAAGGCG
AGCCAACGCTGTATCGGTTTAAATTTAATTCACTATAAAAACGACAAAAACGCAAAAGCC
GCCGACATTCCCGCATCCAAGTTTCAGTCAATCAGATAACCTTGGATTTCTTTGGTTTTC
GCATTGATTTCTCTGGTACGGCAGTCAGATTGTGCCACGCCGTATTCGTCGCCGTCGGCG
CATTTGGCATTCAAACCGTTTTGTCAGTTGCGGTACTCGGGCATGACGGTTTCGGCAATA
TCGGCGGGCAGTGCCTGAACCGCGCTGTTCAGAGCCGCTTTGACAGTTCTTTCTTGTCCG
CTTCCCTGGCCATTTCGTCGATAAGGGCTTTTTTACGGTTGTGCAGCTCTTCCAAGCGTG
CTTCGGTTTTCGCCGTTTTGCATGCCAGTTCGCTGAATTTTATGCCGTTTGGCGTTTTAC
CTTCAGCTTTCGCATTGTTGGCACAGATTTTATCCATCCCGCTTTTCCATGTTTTCTGTG
AGGCTTGCAGCTTATTCTGTACGGTTTGAGGCAATCCTTTCCAGAACTGCTCGAACTCGC
TGCGCGACAGCTTGTAGCGTTCTTCCCATTCGGATACGCGGGCGGCTTCCTGCTCCCGAC
CCAATGCCTCCTGCGCCGCCGCTTCTTCTGCCGCTTCAAGTTCTTCGTTCCTTTGTTTCA
CTTTGTCCAACGGCTCTTTAATCAGTGCCATAGCTGCGGAACATATATGTTTAAATTTAT
GCAAACCATCATATCGGGATTGCACACGCTCTGCAAGTTTACCGACGGTTTTCCTGTTCG
ATAAAAATGCCGTTTGAAACGGTCGGCGTTCAGACGGCATTTTTCCGCAGGTTTTATTTG
CGGTTGGTCTGCAGGTAGAGGTTGATCAGGCGTTCGGTCGAACTGTCGTGCTTTTGCGGC
CCGGTTTCGCCGGTCAGTTCGCCCAAAATGGTTTTAGCCAGTTGTTTGCCGAGTTCCACG
CCCCACTGGTCGAAGCTGTTGATGCCCCAAATGATGCCTTGTACGAAGGTTTTGTGTTCG
TACATGGCAATCAGGCTGCCCATATTGCGCGGGTTGACCTTGTCCATGAGAATGAGGTTG
GTCGGGCGGTTGCCGGAGAAGGTTTTGTGCGGGACCAGCTCTTCGATGCGCACCTCATCC
ATACCCTGCGCTTTGAGTTCGGCGCGGACTTCGTCGGGGGTTTTGCCGCGCATAAAGGCT
TCTGCTTGGGCGAAGACGTTGGCAAGCAGGATTTCGTGGTGTCCGGGCAGGTTGCTGCGT
TTTTCAAGCGAGGCAATCAGGTCGATGGGGGTAATGTGCGTGCCTTGGTGCAGCAGTTGG
AAAAAGGCGTGCTGGCCGTTAATGCCCGTTTCGCCCCAGATAATCGGCGAGGTTTCGTGT
CCGACTGCTTTGCCGTCCAACGTAACCTGTTTGCCGTTACTTTCCATATCGAGCTGCTGG
ATGAATTTGGGCAGGCGGTGCAAATGTTGGTCGTAAGGCGCGATGACGTGGCTGCCGCCG
CCGTAGTAGTTGATATACCAGATGCCGATGAGGGCGAGAATGACGGGCAGGTTGCGCTCG
AGCGGTGTGTTGATGAAGTGTTGGTCCATCAGGTGCGCGCCGTTGAGCATTTCAATGAAG
TTTTCTTCGCCGAGATACAGCATAATCGGCAATCCGATGGCGGACCACAGGCTGTACCGA
CCGCCGACCCAATCCCAAAATTCAAACATATTGGCGGTGTCGATGCCGAATTCGGCGACG
GCTTTTTGATTGGTGGAAACGGCGGCGAAGTGTTTGGCAACGGCTTCTTCGTCGCCCGCA
TGATTCAAAAACCATTCGCGCGCGGTCAGCGCGTTGGTCAGCGTTTCCTGCGTGGTAAAT
GTTTTGGAGGCGATGATGAACAACGTGGTTTCGGGGTGGACTTTGGACAATACGTCGCGC
AGTTGCGAGCCGTCCACGTTGGAGACGAAGTGCATATTGAGGCGCGGATGACCGAAAGGT
TTGAGCGCGGTACACATCATCAGCGGACCCAAATCCGATCCGCCGATGCCGATGTTGACA
ACGTCGGTAATGACTTGGTTGGTATAGCCCAGCCAGCTTCCGCTGCGGACTTCGTGTGCA
AATTCGCCCATACGTTGCAAAACGCGGTTGACTTTGGGCATCACATCTTCACCGTCAACC
ACAATCGGCGAATTGGTGCGGTTGCGAAGGGCGACATGCAGGACGGCGCGGTTTTCGGTG
GTATTGATTTTTTCGCCGTGGAACATCTGCCGCATCCGCTCCGGCACGCCTGCTTCTCGG
GCAAGCTCGAACAAAAGCGACATGGTTTCGTCGTTGATGCGGTTTTTGGAGTAGTCCAGC
GTCAGTCCGCCGACTTGCAGCCAGTAGCGTTCCGCACGCTGCGGGTCTTGCTCGAACATT
TCGCGCATATGCAATGTTTTGCTGTCGTCAAAGTGATTCCACAATTTCGACCATGCGGGT
AAGTCGTGAAGGTGTTTCATCTATATGCTCCTGAATGAGGTTTTTTGTTGTGGGATGAAA
AGGCTGCCGGAAACTGCCGCAAGCCGCCGACGACCGTTGTTCGGCATTTCAGACGGCATT
TGTGGGATGCCGTCTGAAGGTCAATCTTTGTCGTAATCGATGTGCTTGTTGTGTATGCTT
TTTTTGCTTTTCTGCAATTGCAGGCTGGCAGCATCGCCCAAGCGCAGGGCAAGTCCGATG
GCGAGAATGTCGATGACGGCAAGCTGCAAGAGGCGGGAAACCATGGGCGTGTAGAGTTCG
GCATTTTCCTGTGTGGCAACGCTCAACACGCAGTCGGCAAGTTGCGCCAGAGGCGAATCG
TTGCGGGTCAGTGCGATGACAGACGCGCCGTTTTCTTTGGCGATGCTGACCGCATCCAAA

## Appendix A

AGTTCGATAGACGAACCCGTGTTGGAAATGGCAACCAAAACATCCTGATCGCTCAAAACA
GATGCCGCCATCAGCTGCGTGTGCGTATCGACATAGGCGACGGTGGACATGCCGAAACGG
AAAAATTTATGCTGCGCGTCCTGTGCCACAATGCCGGAATTGCCGACACCGTAAAACTCG
ACGCGACGGGCGTGCATCAGCGTGGCAATGGCGTTTTCCAGCTCCGACTCTTTCAGGAAG
CGGCGTTCGCCCAACAGCGAGGCGGCGGCATTGCCCAACACTTTCTCGACCACGCTTGCC
ATATCGTCGTCGGCGTTGAGTTCTTCGTGGACATAGGGCATACCCTCATGACCGATGCTG
GCGGACAAGGCGAGCTTGAACTCGGGCAGCCCTTTATAACCCAAGCTGCGGCAGAATCGG
ATGACGGTCGGCTGGCTGACGGACGCACGTTCGGCAATTTCGGCAACGGCGGCATGGACG
AACCATTTGGGTTCCGCCAATGCACATTCGGCGACTTTGCGTTCCGCACCGGAAAGGTTT
GCCAGTGATTCGCTGATTTTGCTTAACATAATGATATGCCCTTCGATAATGCAGCCCCGC
TGCAAGGAGCCGCTGTGGTTAAACGTTTCTCAAATGGTTGTCAAGAGCCGCAGCCGCACC
GGAAATTCCGGGAAACTCGCTCAAGACGACATACACGGGAATCGCGGCAAGATATGCTTC
AAACCTGCCCTTGTTCTCGAAACGGCTGCGGAACGGGGAAGTTTTGAAATATTCCAACAC
GCGGGGAATAATGCCGCCACACAGGTACACGCCGCCGCGCGCGCCCAGCGTCAGGGCGAG
GTTGGAAGCAACCGTGCCGAGCATGGCGCAGAAGATGTCCAAAGTCTGACGGCACAAAGG
CGACGCGCCGCTCAAAGCCTTTTCCGTGATTTCAGACGGCATCAGTTTGGCGGGTTTGGC
TTTCTGTTTTGCAGCCAAAGCCTCGTAAACCAAGCTCAAGCCCGCGCCGCTCAAAAAGCG
TTCGGCGGAAACATGGCCGTATTTGTTTTTGGCGTACTGCCAAATCAGCACTTCCATATC
GTCAAACGGCGGGAAACTGGTATGCCCGCCCTCGCCCGCCAAAGCCACCCAGCCTGCGTG
GCTGTGCACCAATCCGCTCACGCCCAGGCCGGTACCGGGGCCGATAACGGCTTTGGGGGC
AAATTCGACAGGCTTTTGCCCGCCTACCTGCATCAGGTCTTTGCTTGAAGTCTGCGTTAC
CGCCAATGCCTGCGCGGTAAAGTCGTTCAAAAGGATGAGGGTGTCCAGCCCCAAAGTCTG
ACGGGTGGTTTCGATGGAAAACGCCCAATGGTGGTTGGTCATCTGCACCCAGTCGCCCAA
AATCGGGTTGGCGATGGCAAATGCCGCGTGCCGTACGGCTGTTGCACCGCTTTGATTCAG
ATAGGCACGCACCGCATCGGTAACCGTATCGTAGTCTTTACACGGAAGCACGGCGGCTTT
TTCAATGACGCGCGGCGCGGTTTCCAGCGCAAAGCGTGCATTCGTCCCGCCGATATCGGC
GACCAGTCGGGGATATCCGGCTTGTTTATTCGGCGTAGAAGACATGGCAGTTCACTCCTT
GATGGTTCAAAACGAGGTTGATCGGATATTCGCGGTTTTCGCCTTGTGCGGCTTGGTCGA
ACACGGCTTTTTTCTCTTCGCCCCGTATCGCCAAAAACACATGCCCCGTATGGGCAATCG
CATCCAAGGTCATACTGACGCGCTCGTGCGGCGCGGTAACGGGCGTGGTATGCACCAACG
CGACACCTGCCGAACCGTCGATTGCCGTCTGAAACTGCGGAGCTTTCGGGAAAATCGAAG
CCGTATGCCCGTCGTTTCCCATACCCAAAACCAAAACATCGGGCTGTTTGTAATGTTTCA
GTGCATAATCGACAACAGCATCGGGATGTAATTCGGTTTCAGTTTTTCCGTCTTCCACCA
TAGGAATCCACATTGCCGCTTCCGCTTTGTTCTTCAACAGGTATTCGCGCACCAAACCGG
TATTGCTGTCGGCGTGGACGGTCGGCACGATGCGTTCATCTGCCAAGGTGATGCCGACGT
TTTTCCAATCCAAATCTTTTTGCGACAGGGCGTTGAAAAATGCAATCGGCGAACGTCCGC
CGGAAACTGCCAACACCGCACCGCCCTTCTCGTCCAGTGCGCCCTGCAAAGCATCCGCCA
CTGCGTCAGCCAAAGACTGCGCCGCTTCTGCCGCATTTTCGTATTCGTGCCAAACAAACA
TATTTGTGTCCTTTTTTTATTTCAGACGGCATATTCCGTTATGGAAACGGGTTGAGCAAT
ATGTCGGCCGAACAGTTGTTTATGCTTTTGATACCAAATATCGGGACTGCTTTTTATAGT
GGATTAAATTTAAACCAGTACAGCGTTGCCTCGCCTTGCCGTACTATTTGTACTGTCTGC
GGCTTCGTCGCCTTGTCCTGATTTTTGTTAATCCACTATACTACTTTACTTATGTTCAGA
CGGCATTTCAAACCCCATGCCGTCTGAACGCATTATTGTATTACTGCTCTTCGTGCCACT
TGTGTCCGTCGCGCGCCAATAGTTCGCGCGCGGCTTCAGGCCCCCACGAGTGTGCGCCGT
AGCCGTGCGGCGGCGTGGTGTTATTTGTCCAGTTTTCCAAAATCGGCATCACATATTCCC
ACGCGGCTTCAAGTTCGTCGCGGCGGTTAAACAAAGCGAGTTTGCCGTTAATCACATCCA
GCAGCAGGCGCTCGTAAGCTTCCGCGCGGCGGCCTTCCAATGCTTTGCCCAAATCGGTTG
CCAGCGGCACGGTTTCGACCTTATTTCCTGCCCCCGGGGTTTTCATCTGCGTATAGAGGC
GCACGGATTCATATGGTTGCAACTCGATAACGAGCCGGTTGGGCGCGGTGCGGCTGCCTT
CAAAAATATGGCTGTTCAAATCTTTGAAGTTCAAAAGGATTTCGGCCACTTTGCCCGCCA
TGCGTTTGCCGGTACGCAGGTAGAAGGGAACGCCCTTCCAGCGTTCGTTTTCGATTTCGG
CTTTAATGGCGACGTAGGTTTCGGTAAAGCTGTCTTGCGGAACGTTGATTTCTTCAAGAT
AGCCGTTCATGCCTCTGGCGGCGGTATATTGTCCGCGCACGACGTTTTCATTGACAGACT
CGACGGTCAGCGGCTTCAATGACTTGATGACTTTGACTTTTTCATCGCGCACCGCGTCGG
CATCCAAGCTGGCGGGGGCTTCCATCGCAGTCATGCACAACATCTGCATCAAATGGTTTT
GCACCATATCGCGCAACGCGCCGGTAATGTCGTAAAACTCACCGCGCTCTTCCACACCGA
GCTGTTCGGCGATGGTCAACTGCACGCTTTCGATATATTTATTGTTCCACAGCGGCTCGA
ACATTACATTGGCAAAACGCAGCGCAAGCAGGTTTTGCAGGCTTTCTTTGCCAAGGTAGT
GGTCGATGCGGTAAATTTGCCCTTCTTTGAAATAACGCGCAACATCGGTATTGATTTGCT
GGGAAGAAGCCAAATCCGTACCCAACGGTTTTTCCAAAACTACGCGCACATTGTCGGCAT
TCAAACCGATCGCAGCAAGGTTTTCACAGGCTTGCGCGAAGAATTTGGGCGCGGTGGACA
GATAGATGACGACGTTGTCGGTTTCTTTGCGCGCTTTGACCAAATCGCCCAAAGCGGCAA
AATCGTCCGGCTGCGTAACATCGACTTTGAGATATGCGAAACGTTCGACAAACGATGCCC
AAGCCTCATCGGAAAAATTTTCTTTCACATGGATTTTGGAACTGGTTTCCACCTTCGCCA
GAAAACCTTCGGTATCCAACTCGCTGCGGCTGACCCCCAAAATACGCCCTTCGGGATGAA
GCAGACCGGCAACATGCGCCTGGTACAGACAGGGCAACAGCTTGCGCATCGCCAAATCGC
CGGTCGCACCGAACAACACCAAATCAAAATTTGTTTGTGTACTCATCGTATTATCTCGTC
AGGAAAGAATTTTTCGATGCCGTCTGAAACCTGTTTCCCCCATCACGCTGCATCGCAATA
TCGGAAACAAAGGCAGGCGGCATAATGAGTAGTAATACTACACACCGCTACACTTTTTGT
CTATTCCCATTTTTACAATTTATTTGACCTAGTCCAAAAATCGGGCAGGTTTCCCCTATT
CCGTTACAACAATCGAAAGATTCTGCGATTTAAATCAAATTTCTTTTCAATGCCTGATTT
TTTTGTAACAAAATTACAAATTTTGTACTATAATAACACCCGCTTCCCACTTTCAGACGG
CATACCTTTTAAAATATAGTGGATTAACAAAAATCAGGACAAGGCGACGAAGCCGCAGAC
AGTACAGATAATACGGAACCGATTCACTTGGTGCTTCAGCACCTTAGAGAATCGTTCTCT
TTGAGCTAAGGCGAGGCAACGCCGTACTGGTTTTTGTTAATCCACTATACTTACCGTCTG

# Appendix A

AATACCCGATACAAAAATCAGAAACGCACAAACAAATCCCCAATACCCCCCCGTTCCGA
CAGGAGACCGACCGTGAACACTACTCCTATCCACTCCAAACTCGCCGAAATCACCGGGCG
CATTATTGAACGCAGCCGTCCGACGCGTGAAAAATATCTGGCGAAGATCCGCAGTGCCAA
ACAAATGGGACGCTTAGAGCGCAACCAGCTCGGCTGCAGCAACTTGGCACACGGCTATGC
TGCCATGCCTAAAAGTATCAAAATCGAAATGCTTCAGGAAACCGTCCCCAACTTAGGCAT
CATCACCGCCTACAACGACATGGTTTCCGCACACCAGCCGTTTAAAGACTTCCCTGACCA
AATCAAAGACGAAGCGCAGAAAAACGGCGCGACCGCCCAAGTCGCCGGCGGCACGCCCGC
CATGTGCGACGGCATCACGCAAGGCTACGCCGGCATGGAATTGTCGCTGTTCTCCCGCGA
CGTGATTGCCATGAGTACCGCCATCGGGCTGTCGCATCAAATGTTTGACGGCAGCCTGTT
TATGGGCGTATGCGACAAAATCGTTCCAGGTTTGATGATAGGCGCGCTTTCGTTCGGTCA
TATTCCGGGTATCTTCGTCCCCGCAGGCCCGATGTCCAGCGGTATCGGCAACAAAGAAAA
AGCCCGCACCCGCCAGCTTTTCGCCGAAGGCAAGGTCGGACGCAACGAACTTTTGAAAAG
CGAAATGGGTTCTTACCACAGCCCGGGCACCTGCACTTTCTACGGCACGGCAAACTCCAA
CCAAATGATGATGGAAATGATGGGCGTGCACCTGCCTGCCGCCGCCTTCGTCCACCCTTA
CACCGACCTGCGCGAAGCGCTGACCCGCTACGCCGCCGGACACCTCGCGCGCGGCATCAA
AAACGGCACGATTAAACCTTTGGGCGAAATGTTGACCGAAAAATCCTTTATCAACGCCTT
GATTGGCCTGATGGCAACCGGCGGTTCGACCAACCACACCATGCACCTCGTCGCTATGGC
GCGTGCGGCCGGCGTGATTTTGAACTGGGACGACTTCGACGAAATTTCCTCCATCATCCC
GCTGCTCATCCGCGTTTATCCGAACGGCAAGGCCGACGTGAATCACTTTACCGCAGCGGG
CGGACTGCCTTTCGTTATCCGCGAATTGCTGAATGCAGGCCTGTTGCACGACGATGTCGA
TACCGTCGTCGGACACGGTATGCGCCACTACACCAAAGAGCCTTTCCTTATCGACGGCAA
ACTCGAATGGCGCGAAGCCCCCGAAACCAGCGGCAACGACGACATCCTGCGCAAAGCTGA
CAACCCGTTCTCCCCCGACGGCGGTCTGCGCCTGATGAAAGGCAACATCGGACGCGGCGT
GATTAAAGTGTCCGCCGTGCGCGAAGGCTGCCGCATTATTGAAGCGCCTGCCATCGTGTT
CAACGACCAACGCGAAGTGTTGGCTGCGTTTGAACGCGGCGAGTTGGAACGCGATTTTGT
GTGCGTCGTCCGCTACCAAGGCCCGCGTGCCAACGGTATGCCCGAATTGCACAAACTGAC
CCCGCCTTTGGGCATCCTGCAAGACCGCGGCTTCAAAGTGGCGCTGCTGACCGACGGCCG
TATGTCCGGCGCGTCCGGCAAAGTTCCAGCCTCCATCCACATGACACCCGAAGCCCTGAT
GGGCGGCAACATCGCCAAAATCCGTACCGGCGACCTGATCCGCTTCGACTCCGTTAGCGG
CGAACTCAACGTCCTGATTAACGAAACCGAATGGAATGCCCGCGAAGTCGAAAGCATCGA
CTTGGGCGCGAACCAACAAGGCTGCGGCCGCGAACTCTTCGCCAACTTCCGCAGCATGAC
CAGCAGCGCGGAAACCGGTGCCATGAGTTTCGGCGGCGAATTTGCCTGATGCGCGTTTCA
GACGGCCTTTTCAGACCGAAGGCCGTCTGAAAAATTATTCAAGCGTTTTAAGATAGACGT
AGGTTGGATTCTCGAATCCGACACAGCCGTCCAAGATGTCGGTTTCTTGAATCCGACCTA
CAACCTGTCCCATCTTAATAAAATACCCCATTCCACCCGGAGAACCGAAATGTCCAAACT
GACCCCCCGCGAAATTTTGACCGCCGGCGCAGTTGTGCCGGTAATGGCGATTGACGACTT
AAGCACCGCCATCGATTTGTCCCACGCCCTTGTCGAAGGCGGCATCCCTACCCTCGAAAT
CACCCTGCGCACCCCTGTCGGCCTCGATGCCATCCGCCTGATTGCCAAAGAAGTGCCCAA
CGCCATCGTCGGCGCAGGTACGGTAACCAATCCCGAACAGCTCAAAGCCGTCGAAGACGC
AGGCGCGGTTTTCGCCATCAGCCCGGGGCTGCATGAATCCCTCGCCAAAGCCGGCCACAA
CAGCGGCATCCCCCTGATTCCCGGTGTTGCCACCCCGGGCGAAATCCAACTGGCTTTGGA
ACACGGCATCGACACCCTCAAACTCTTCCCCGCCGAAGTCGTCGGCGGCAAAGCCATGCT
CAAAGCCCTGTACGGCCCTTACGCCGATGTTCGCTTCTGCCCGACAGGCGGCATCAGCCT
CGCCACCGCGCCCGAGTACTTGGCACTGCCCAACGTCCTGTGCGTCGGCGGCTCTTGGCT
GACACCGAAAGAAGCCGTGAAAAACAAAGACTGGGACACCATCACCCGCCTCGCCAAAGA
AGCGGCGGCGTTGAAACCCAAAGCCTGATTCGCATCGTAAAAATGCCGTCTGAAAAACCT
TTCCCGTTTCAGACGGCATTTTGCCGATTGAGGGCACAGTCGGCATACACGGCAGCACTG
ATCAGACATACCGCCCCTAAAATGCCCATCCGCCTTCCGCATAATAAAAATAACGTTCAG
TTCATTCGACAGCAGCCGGACAGCCCATACTACGCGGCTGAAAAAATGCCGTCTGAAACG
~~CATTCAGACGGCATCCACTTAAAAAAAACAACTGATTCAACGCCGATTAATCCGCTTCCA~~
AAACCACTTTCATCACTTGGTTTTCGGCGGCGTGTTTGAACACGTCGTAGGCTTTTTCCA
ATTCACTGAATTTGAAATGATGGGTCAGCATTTTGGTGTAATCGACGGAGCTGCTGGAAA
TCGCCTTCATCAGCATTTCGGTGGTATTGGCGTTTACCAGACCGGTAGTGATGGCAAGCT
TTTTAATCCAGAGTTTTTCCAGTTTGAAATCAACGGATTGACCATGTACACCAACCACAG
CGATATGGCCGCCGGGTTTCACAATGTCTTGGCACATATTCCATGTAGCAGGGATACCGA
CGGCTTCGATGGCGCAATCCACGCCGTCTTCGCCGACGATGGCAAAGACTTGTTTGGATA
CTTCGCCGGAAGCAGGGTTAATGGTATGGGTCGCACCCAATTCTTTCGCCAGTTTCAAAC
GGTTTTCGTCCATATCGCAAACGATGATGGCGGCGGGACTGTACAGTTGGGCGGTCAACA
GGGCGGACATACCGACAGGGCCTGCCCCAGCGATGAATACGGTGTCGCCGGGTTTGACAT
CGCCGTATTGCACGCCGATTTCGTGGGCGGTCGGCAAAGCGTCGCTCAACAACAGGGCGA
TTTCTTCGTTGACATTATCGGGCAGCGGAACGAGGCTGTTGTCGGCATAAGGCGTACGGA
CGTATTCGGCCTGAGTACCGTCAATCATGTAACCCAAAATCCAACCGCCGTTACGGCAGT
GTGAATAGAGTTGGGTTTTGCAGTTGTCGCAAGTGCAACATTTGCTGACGCATGAAATAA
TGACTTTATCGCCGACTTTGATGTTTTTACAGCCTCGCCGACTTCTTCTACAATACCGA
TGCCCTCATGACCGAGAATACGGCCGTCGGCAACTTCGGGGTTTTTGCCTTTCCAAATAC
CCAAATCGGTACCGCAAATCGTGGTTTTGACGATTTTCACCACCGCATCGGTCGGATCGA
TAATCTGCGGACGGGGTTTTTCTTCAAAACGGATGTCGTTTGCGCCGTGATAAACCATTG
CTTTCATGCTGATACTCCTTGCTTGTTGATAAATAATTTCAATACCGCAATAAAGTTTCT
TTATATGAGTTATATGCCCCTACAAAAAATAAGTCAATAAGAATTATTTTCACAATGTTA
TACAATAACATACCGTTTTAAATATAAATAAAACCACCGATTGATATTAATGAACACACC
CATCCCCTTCTCCGAACGGCTCATCCGCTGGCAAAAACAACACGGTCGCCACCACCTCCC
TTGGCAGGTCAAAAACCCTTATTGCGTCTGGCTTTCCGAAATCATGCTCCAGCAAACGCA
AGTCGCCACCGTGTTGGACTACTATCCGCGCTTCTTAGAAAAATTCCCGACCGTTCAGAC
GCTTGCCGCCGCGCCGCAAGACGAAGTGTTGTCGTTGTGGGCGGGCTTGGGCTATTACAG
CCGCGCGCGCAACCTGCACAAAGCCGCGCAACAAGTCGTCAGGCAATTCGGCGGCACGTT

## Appendix A

TCCGTCGGAGCGCAAAGACTTGGAAACCCTCTGCGGCGTAGGCAGAAGCACCGCCGCCGC
CATTTGCGCCTTCTCCTTCAACCGCCGCGAAACCATTTTGGACGGCAACGTCAAACGCGT
ACTCTGCCGCGTGTTCGCCCGCGACGGCAATCCGCAGGACAAAAAATTTGAAAACTCGCT
CTGGACACTTGCCGAAAGCCTGCTGCCGTCTGAAAACGCCGATATGCCTGCCTATACACA
AGGTTTGATGGATTTGGGCGCGACCGTGTGCAAACGGACGAAACCCTTGTGCCACCAATG
CCCGATGGCGGACATCTGCGAAGCGAAAAAGCAAAACCGCACCGCCGAGCTGCCGCGCAA
AAAAACCGCCGCCGAAGTACCGACCCTGCCGCTTTACTGGCTGATTGTCCGCAACCGGGA
CGGCGCGATTTTGCTGGAAAAACGCCCCGCCAAAGGCATTTGGGGCGGGCTGTATTGCGT
GCCGTGTTTTGAAAGTTTGAACGGGCTTTCCGACTTTGCCGCCAAATTCTCCCTGACCAT
GGCAGATATGGACGAACAAACCGCCCTGACCCACCGCCTGACGCACCGGCTGCTATTGAT
TACGCCCTTTGAAGCACAAATGCCGTCTGAAAGCCCTTCAGACGGCATTTGGATAAAGCC
GGCGCATTTGAAAGATTACGGTTTGCCCAAGCCTTTGGAAATTTATTTAAACGGTAATAG
GTTAGAATAAACAAAATAAACCCATTGAACTGTTGTTTGCAGGTATCGCAGCAAGAACAA
CCGATGAATTTGGGTCGTATTTTAGGCGGCGGGATAATGTTCAAATGGGACATTTGGAAC
GGAAGAAGTCGGCAATTTAAAAAGGATTTAAAAAGCAAAGAAGGTCAAAAACATGAACAC
AAACTTAAATGACAAAGACAAAGCCATGGATACCGCAATCAGGTTTCAGAAAAGGATGAG
GATTCCGAAATTTTTCTTTTTAATTCTCGGAATCACAATGGTTTTGGCATTTATCCAAGA
CGTGATAACGGGTTCTAATTTTCTGCAAATAACAATTAATGTAAAATTTTCGTAAAAATT
TATCGGCTTTTAAAACAAAATTGACTAAAATAGTCGCGAGTTTTTACTGCAATAAAGGAG
ATTGCAATGAATATGAAAACCTTATTAGCACTAGCGGTTAGTGCAGTATGTTCAGTTGGT
GTTGCGCAAGCACACGAGCATAATACGATACCTAAAGGTGCTTCTATTGAAGTGAAAGTG
CAACAACTTGATCCAGTAAACGGTAACAAAGATGTGGGTACAGTGACTATTACTGAATCT
AACTATGGTCTTGTGTTTACCCCTGATTTACAAGGATTAAGCGAAGGCTTACATGGTTTC
CACATCCATGAAAACCCAAGCTGTGAGCCAAAAGAAAAAGAAGGTAAATTGACAGCTGGT
TTAGGCGCAGGCGGTCACTGGGATCCTAAAGGTGCAAAACAACATGGTTACCCATGGCAA
GATGATGCACACTTAGGTGATTTACCTGCATTAACTGTATTGCATGATGGCACAGCAACA
AATCCTGTTTTAGCACCACGTCTTAAACATTTAGATGATGTTCGCGGTCACTCTATTATG
ATCCACACGGGTGGTGATAATCACTCCGATCATCCAGCTCCACTTGGCGGTGGCGGCCCA
CGTATGGCATGTGGCGTGATTAAATAATTCGATTGTTCGAAACGAAAAGTGCGGTGAATT
TTGACCGCACTTTTTTGCTAGATATTTAGCATTGAGACCTTTGCAATAACATAGGTTACT
AAAATTTTATGCTCAATCTCATTTTCAAAATGCAAAACTTTTCTGATTTTTCCTACTTTT
TGCTCAATATTAGGAAGGTTTTAGGCAATTGAAAATTTTTTGGCGCATTTTTATGCGTCA
AATTTCGTTAACAGACTATTTTTGCAAAGGTTTCAATTCATAAGTTTCCCGAAATTCCAA
CATAACCGAAACCTGACAATAACCGTAGCAACTGAACCGTCATTCCCGCGAAAGCGGGAA
TCTAGACCTTAGAACAACAGCAATATTCAAAGATTATCTGAAAGTCCGAGATTCTAGATT
CCCGCTTTCGCGGGAATGACGAAAAGAGACCTTTGCAAAATTCCTTTTCCCCGACAGCCG
AAACCCCAACACAGGTTTTCGGCTGTTTTCGCCCCAAATACCGCCTAATTCTACCCAAAT
ATCCCCTTAATCCTCCCCGGATACCCGATAATCAGGCATCCGTGCTGCCTTTTAGGCGGC
AGCGGGCGCACTTAGCCTGTTGGCGGCTTTCAACAGGTTCAAACACATCGCCTTCAGGTG
GCTTTGCGCACTCACTTTAACCAGTCCGAAATAGGCTGCCCGGGCGTAGCGGAATTTACG
GTGCAGCGTACCGAAGCTCTGTTCAACCACATAACGGGTCTTCGACAAATATCGGTTGCG
TTTGGTTTGCACTTCCGTCAGCGGACGGTTGCGGTGGGCTTTGCGCATAATGCCGTCCAG
CAACTGATGTTCTTCCAGATGTTGCCGGTTTTCCGCACTGTCGTAGCCTTTGTCGGCATA
GACGGTCGTACCTTTGGGCAGTCCTTCCAACAACGGCGACAGGTGTTTGCACTCATGGGC
ATTGGCGGGGGTAATGTGCAGTTTCTCGATATAGCCTTCTGCATCGGTACGGGTATGTTG
TTTGTAACCGAGTTTGTAGAGGCCGTTTTTCTTTATCCAACGGGCATCGCTGTCCTTACT
CGGTGTGGTTTGACCGCTGATTTGTCCTTCTTCGTCAACTTCTATGGCCTGACGCTGTTT
GCTGCCGGCGGTCTGAATAATGGTGGCGTCAACGACGGCAGCGGATGCTTTCTCTATTTT
TAAACCTTTTTCGGTCAGTTGGCGGTTAATCAGTTCCAACAGTTCAGACAGGGTATTGTC
TTGCGCCAGCCGGTTGCGGTAGGGGCATAAGGTGCTGTAATCGGGGATGCTCAGTTCGTC
AAAACGGCAAAACAGGTTGAAATCGATGCGGGTAATGAGGCTGTGTTCGAGTTCGGGATC
GGAGAGGCTGTGCCATTGTCCGAGCAGGACGGCTTTGAACATGGACAGCAGGGGATAGGC
AGGACGGCCGCGGTGGTCTCTAAGGTAACGGGTTTTTGACGGTTCAGGTATTGTTCGAT
CAGCTGCCAATCAATCACCCGGTCCAACTTCAATAGCGGGAAGCGGTCGATGTGTTTGGC
AATCATGGCTTGGGCGGTTTGCTGGAAGAAGGTGCTCTTGAGAAATCCCCTAAATGTCTT
GGTGGGAATTTAGGGGATTTTGGGGAATTTTGCAAAGGTCTCTAGATGAGTGAAAAAGAA
GTGCAGGCTGCCTAAAAAGACAGAAAAAGTCTTTCCGGCAGCCTGCACTTTGGTTTCATT
TCAGTCAGTAAACCCAGTAAACGACGGTCTGAAAACGCAGAACGTTACGAAAAAAGCAGC
CTACACGCCCATCCCCCGCCTTCTACCCGTTCTGTAAATCATACAGATAGCGGTAATATC
CGTTCGGCTTCGCCAGCAATTCCTGCTGTGTTCCCGCTTCCACAATCCTGCCTTTATCCA
TGGCAATGATCCGGTGTGCCGTTTTAACAGTGGACAGACGGTGGGCGATAATCAGCACCG
TCCGGTTGGCGCAAATGGCCTGCATGTTCTGCATAATCGCTCGTTCACTTTCATAATCCA
GCGCGCTGGTGGCTTCATCAAAAATCAGAATGCGCGGATTGGTGATTAACGCGCGGGCAA
TCGCAATACGCTGCCGCTGTCCGCCCGACAAGCCGGCCCCTTGTTCGCCCACCACGGTGC
CGTAGCCTTCCGGCAGCTCCATAATAAACTCGTGTGCGCCCGCCAGTTTGGCTGCTTCGA
TAATGCGTTCCAGCGGCATACCCGTATCCGTCAGCGCGATATTGTCGCGTATGCTGCGGT
TGAGCAGCACATTCTCCTGCAAGACCACGCCGACCTGCCGCCGCAGCCAGGCAGGAGCGG
CCAAAGCCAAATCGTTGCCGTCCACCAACACCCGTCCCTGCTCCGGTACATACAGACGCT
GCACCAATTTGGTGAGTGTGGATTTGCCCGACCCCGAACGTCCCACAATCCCCAGCACTT
CCCCCGCCCGAATCCGCAGGTTCAAATCCTGCAAAATCAGCCTGCCGTCCGCCTTATAGC
GGAAATCGACATGTTCGAACGTAATCTCCCCCCGGATATCGGGCAAAGCCAAATGCGAAG
ACGCATTCTCGGTCGGCGCATTCAGAATATCCCCCAAACGCGCCACCGAAATCCCCACCT
GCTGGAAATCCTGCCACAACTGCGCCAAACGGATAACAGGCGCCGCCACCTGTCCCGAGA
GCATATTAAACGCAATCAGCTGCCCCACCGTCAGCTTGCTCTCAATTACCAGCCGTGCGC
CAATCCACAACGTCGCCACCGTCACCAGCTTCTGAATCAGCTGCACCCCCTGCTGGCCGA

## Appendix A

CCACCGCCAACTTCGTTACCCGAAATCCCGAAGCCACATAAGCCGCCAACTGATTGTCCC
AACGCTGCGTCATCTGCGGCTCCACCGCCATCGCCTTTACCGTACCCACCGCAGTGATGC
TTTCTACTAAAAACGACTGGTTGTCTGCATTGCGCGCGAACTTATCGTTCAGACGCGTCC
GCAGTATCGGACTGATAAATGCCGACCAAAACGCATAGGCAGGCAACGAAGCCAATACCA
CCCAAGTCAGAGTGGAGCTGTAATACCACATCACCGCCAGAAAGATAAACGAAAACGCCA
AATCCAACACCGAAGTCAGCGCCTGACCGGTCAAGAAATTGCGAATCTGCTCCAATTCCC
GCACCCGAGCCACCGTATCACCCACTCGTCTGTGCTCGAAATAGGATAAAGGCAGGGAAA
GCAGATGCCGGAACAAACGCGCGCCCAATTCCACATCAATACGTGAAGTCGTATGTGCAA
ACAGATACGTCCGCAAACCGCCCAACACAATCTCAAACAGCGACACCACCAACAAAGCCA
CCGACACCACATCCAAAGTAGAGAATCCCCGATGTACCAGCACCTTGTCCATCACCACTT
GGAAAAACAGAGGCGTAATCAGCGCAAACAGCTGCAACACCACCGACACCACCAATACTT
CAAAAAACAACCGGCGGTATTTGATTACCGCCGGAATAAACCAGGTAAAGTCAAACTTTG
CCAAACTGCCCAATACCGAAGCGCGGGAAGCAACCAATATCAGTTTGCCCGAATATCTGT
TAGAAAATTCGGCAAAAGACAATACCGCAGACTTATTCGTAACCAAATCCTGTATCAAAA
ATTGGGCATGCTCACCCTCACCGTCTGTTTTGGCCAAAATGAAATGGTTGCCGTCATCAC
ACCATACCAATGCGGGTAAAGTCGCCATAGCCAAACGTTTAATAGGCTGGCGGACTACCT
TTGCCTTCAATCCCAAAGATTTGGCGGCTAACAGCCATTGCGTTTCATTTAAATCGCTCT
GTGCGGAAGTACAAAATTCATGCTGTATATCGGCAGGATTGGCGGCAATGCCGTGGTAAT
GGGCGAGGATGATGAGGGCGGAAAGGGCGGGGAGCGGTGCGGATACGATAGACATAAATA
AAATATAGTTAGATTGGATGTGGATAACGGCTGGCTGGAAAAGGAATATATTAAGTAGAA
AGAAATATATAAATAAAACAGCAGAACGCATTGTAAGGATATATATGGGAATTGTAAAGA
GAAAGTATGGAAAAGTTCTCGTTTCAGGAAGGTAAAACGGCTTAGGAATCGAGTTAGATG
AGGATGCCTCGCACCTCTCGTGCCTCCTGCATACCGTTAAGGCACAGGGTTAAGGTGCAG
GCTGCTCCGAACTCTGTTGCGGTCGGGTAATGTTATTTTTTGTGTTTCAGGCAGCCTGAA
ATATCTGTATATTTTTGTTTTAAATAGATTTTAAAGATTGATAACTGTTCTTGACGATTT
TTCAAGAAAGGAGTAAATTTCAAGAAAGGAGTAAAGTGACTTATTATCAATGACAAGCAA
CGCGCGAAGTGACAAGGAAAACTATCTACTTAAATTCTAAGGAGGCTTCGAATATCATAA
ACCAATCAGAAACATAGAGATAAAAATTATGTACAAATATAATCCTCTTATACAATTTAT
TGCACAGTTGATTATGTCTTATGGAGCAAGCGTAGGGTGGGCACTTGCTGCCCCACGCGT
TTCATATTTCAAGGCAGCCTGAAACCGTGTGGGCATAAATGCCTACCCTACATCCCAAAA
AACAAGCGCAGCCTGCGTGTGTAGGGTGCGAACTTTCGGCAGGTAGACACGCAGTTTTAT
ATTTTCAAGCTGAGGGATGCTTAAGAAAAGTACAAAACATTAAAAAATAAGGGGCTGTAC
TAGATTAGCCCTAAATCCACACCAATCCCGCAAGATTTTTAGCTGTCGGGACGGTGTGCC
GAAGTTAAATCGAAATTCGCATTCTTTCAAGAACAGCGGGAAAGATTTGCGATCAATTCC
GTTCTATTTGCGCAAGACGCGTTTTGCCTGATTCCAAAAGTTCTCAATGCCGTTTATGTG
GTTCTGACGGTCAGCAAATTCCTTGGAATGGTTGATGCGGTAATGGATAAAACCGCTTAC
GTCCAACTTGTCGTAACTGCTCAGGCTGTCGGTATAAACAATGCTGTCCGGCATGATTTT
CTGTTTGATAACAGGCATTAAAGTATCGGACTTGGCATTATCTACGACAACGGTATAGAC
CCGTCCGTTACGTTTCAGAATGCCGAAGACAACCACTTTTCCTGCCGCACCGCGACCACG
TTTGCCTTTACGCCGTCCGCCGAAATAGCTTTCGTCCAACTCGACAGAGCCCTCGAAAAC
CTCATTGGCAGCCAAGGCCAGATAATGGCTGATGACCATACGGATTTTGCGGTAGAACAG
GACTGCCGAATTGGGATGGATACCCAAAATATCGGCAGCAGAACGGGCGGTAACTTCGAG
TACAAAAAAACGGAGCAGCTCTTTCTGTACTTTTTTCTTTAATTTGCAGTGTGTTATCTT
CATATTTCGGGGGTAACATATCTGCTAATCTAGTACAGCCTCAAACAAAAAAGAGAAATT
TTAATTTCGCTAAATCGCATAAAGATTAATCAAGAGTATCATTAAATGATATGAGTGAGC
ATCTATAATGCCAAGAAGAGTTGTTAAGACATAACGATTATTGAAATAGATTGTAAAATA
GATACTTAGATAGTCTGAAAAACGGATTTGTGAAACTTTTTATTACGCGCCATCATTTGA
AAATGAAACTTAAAAAACACTTATCATAATAAATATTTTCTTTACGTTGTTTGCTAATAA
ACTCAGTGCAATATCAGCGCAATATTTTATGGAAATTTTATGGATAACAAAAAAGAATTT
ATTAATAATTTAACAAATAGGTATATGTGGATCTATCCATTGGTCTTAAATATTCTATTT
CTACCTTTTTACCAGTCCTACCAATCTTTTTTTATTGCGCTTGGTTGTTTGTTTGCACTG
GTTAGAAAAATGCAAAGCTTAGATTTTAAATTACAAAATCATATTGTATTGTTAAATATA
AAAAGTGCTTGGGCAGATAAAAAAGTATTTTTGATTAGGATAGTAGTGTCATGGTTGGCA
GTAATGGAAATATGGATGTGTTTTATTTCGGAATCATCAACGTGGGTATGCGGTGCTTTT
TGTTTAAATAGTGAAATATTGGAAAAAATTTTTCGTGGCTTTGGTTATTCTGGTAGTTTA
TATTTTTTATTTATATTGATGATTGATCTCAATAAGTTAAGAGAGAGTATTTGAATTTCA
TTTTTTGTTTGACTTAAACTCAAGGAGAGTAACAATGATTGGTAGTGGTGATACTAAACA
ATGCAAAAAATTTTCTGCGTGTGATGGAAAATACCACGTCTACGATCCCCTCGCCCTAGA
CTTGGACGGCGACGGCATAGAAACAGTCACCGCCAAAGGCTTTTCAGGCAGCCTGAAGAC
TGAGAGAGTGAATACGATGAGTATACACTCTATGCCACTAAATTGATATTCACTAAATCA
TACCAGCTATATTTTATTTAATGAGACATATGAAAAATAAAAATTATTTACTAGTATTTA
TAGTTTTACATATAGCCTTGATAGTAATTAATATAGTGTTTGGTTATTTTGTTTTTCTAT
TTGATTTTTTTGCGTTTTTGTTTTTTGCAAACGTCTTTCTTGCTGTAAATTTATTATTTT
TAGAAAAAAACATAAAAAACAAATTATTGTTTTTATTGCCGATTTCTATTATTATATGGA
TGGTAATTCATATTAGTATGATAAATATAAAATTTTATAAATTTGAGCATCAAATAAAGG
AACAAAATATATCCTCGATTACTGGGGTGATAAAACCACATGATAGTTATAATTATGTTT
ATGACTCAAATGGATATGCTAAATTAAAAGATAATCATAGATATGGTAGGGTAATTAGAG
AAACACCTTATATTGATGTAGTTGCATCTGATGTTAAAAATAAATCCATAAGATTAAGCT
TGGTTTGTGGTATTCATTCATATGCTCCATGTGCCAATTTTATAAAATTTGCAAAAAAAC
CTGTTAAAATTTATTTTTATAATCAACCTCAAGGAGATTTTATAGATAATGTAATATTTG
AAATTAATGATGGAAACAAAAGTTTGTACTTGTTAGATAAGTATAAAACATTTTTTCTTA
TTGAAAACAGTGTTTGTATCGTATTAATTATTTTATATTTAAAATTTAATTTGCTTTTAT
ATAGGACTTACTTCAATGAGTTGGAATAGTTTTGGTAATTTTATGAGCGCACGCTCATCC
GCGTTAGCAGAATTTGGAAATATGGTTGCTAATTTAGTTTCTGCAAAAAATGAGAAAGAT
ATCTCGAAACGTAATGAATATTACAAACAAGCTGGTTATAGTGCATTATTAGCATTTGGT

## Appendix A

AATTTGGCTAGTAATATTGCACCAGGTAGTACGTCATCGCATATTGTAAACGGAACAAAT
GCCTCTGTGATTGCAAGCCGTCTCTCTGGAAATATATCTTCAGCTATTCAGGAGCATAAA
GATGGTAAAGTTAATATCAACCGTTTTCAAAATATTTTAGCGGATTTATATTCATTGGGA
GGGTTAGGAAGTACATTAATAGAGAAGAATGGAAATATGCAGAGTTGGGGGATTCCATTA
GCAATTGCTGGAGATATAATTGCAGCAACGGCTATTGCCACAGGAGATACTGGTACGATA
TCTACAGAGGAATTTTATAATTTTGACAACTGGAAAGGTTTTGGGTATGAGCTATTTGAA
GACTGGTCTCGTTGGGTATACGACTGGCTGCCCGACGGCTGGAATCTGTGGAAAGAATTG
GACAGAAACCGTTCAGGCCAATACCACATCTACGACCCCCTCGCCCTAGACCTAGACGGC
GACGGCATAGAAACAGTCGCCGCCAAAGGCTTTTCAGGCAGCCTCTTCGACCATAACGGC
AACGGCATCCGCACCGCCACTGGCTGGGTTTCTGCCGATGACGGTTTACTCGTCCGCGAT
TTGAACGGCAACGGCATCATCGACAACGGCGCGGAACTCTTCGGCGACAACACCAAACTG
GCAGACGGTTCTTTTGCCAAACACGGCTATGCAGCTTTGGCCGAATTGGATTCAAACGGC
GACAACATCATCAACGCGGCAGACGCCGCATTCCAATCCCTGCGTGTATGGCAGGATCTC
AACCAGGACGGCATTTCCCAAGCTAATGAATTGCGTACCCTTGAAGAATTGGGTATCCAA
TCTTTGGATCTCGCCTATAAAGATGTAAATAAAAATCTCGGTAACGGTAACACTTTGGCT
CAGCAAGGCAGCTATACCAAAACAGACGGTACAACCGCAAAAATGGGGGATTTACTTTTA
GCAGCCGACAATCTGCACAGCCGCTTCACGAACAAAATGCTATCCATTAGCCATGTTCGG
GAAAACACGATTTCCCCGTTTGTTTTAGGCTGTCTAAACAAATAACCATAAATGTATATC
ATTATTTAAAATAAATAAAAGTATTTAACTATTATTGACGAAATTTTAGAGAAAGAGTAG
ACTGTCGATTAAATGACAAACAATAGTGAGAAAGGAAATATTTACTATCCGAGCACAGAG
CATATTTTAGGTAGCCTGTAACTGTTCCTGCTGGCGGAAGAGGATGAAGGTTGACTTACC
CGAGAATAAATGTCCTGTTGTGTGATATGGATGCCATGCCGCGAAGCAATTGATGCAATC
ACGGCAGTCCTACTTGAATGAAACCTGTCGTTGCAGAATTTGAAAACGCTATTTTTAAGA
AAGGATAAAGGGAGAAAGAATTTTTGGTTTTTAAGCTGCATGAAACCGTGTTGGAATAAA
TGCACACCTACGATAATTAATAATTTTCGTTTTTTATTCTACAAGCTATTTATATATGAT
TGCTAAAAGTTTATTTTTTAGATGCCAAAAAATATATTTTATATACTTCATATTGTTTAT
ATGTCTTTATTTGAATATATCTTACGATGGGGAAATATTTATATATTTTATAATAAATTT
TACTCATTTGCTAATATGTCATGGAATATTACTTGTATTTTGTAGAATTTTTCCATATGA
AAATATTCCATTTACTATTTTTCTGAACTTTATTAGTTTATTTTTAATATTTTTACCTCT
TATATTTACCATAAGAGAGCTAATTGATTCATATTATATTGAGTCGATAATTAATTTATT
CTTAATTTTAATTCCTCACGTTATTTTTTTAATTTACTTGAAAGGAAAGCAGATATGACA
TCTGCAAATTTTAATATTAACGGTTTTGGAGATGTGAAATTAACACCCTATTCACCACTC
TTGGGATATAAAGCTTGGGATTCATTTATTGGTTCTATTCAATCCTTATCTGATTTAATC
TATAATGTGGATAACAATAGAAATAAAATGGAAATTACTGTTAATAATGCTATTCAAGCT
GCAGATAGCTTTTTAAGCAGTAATTGGAAGAGATAACAAAATAACAAAATAACAAAATAA
CAAATACTGCTTCTTTACTTGCATCCTTCGATAACATTTTTTAAATTTAAGAAATGTATC
TCGAGATATACGAGAAACAGGAAAATTTAAACCTAATGATATTCAACAAGCAATTGGTGA
TATATTCATTGCTGCTGGTGATGGATTACAATATATAAAACAACAAACAGAGGCGATGGC
TCAAAGCAAATTCTTACCAACTAAATTAAAAACTGGTTTAAATGATGTCCTTAATTCTAG
AATGCTAAAATCCTCTACTGTTTTACAGCATGAATTGAATTAAATAAGGATTATGGAAAC
GAGAGGCTTGGCGAATCTATAATGAATATAGATGATTTTACACCAAGTAAGATAGCAAAC
TTTTTTGCGGATCCTGATACATACAGCAATGTATTAGAAGAAGTATCTAGGTTTATATAT
TCCTTAGTTCCTGATGATGCAAACCCTTGGAAAGGGGGCGAAGATTATATTGGACGAGGG
ATAAGTGAATGGGGAGAGTTACTGGAAAAATGGTATAAACAAGATTTTCTCCCTTATCTT
GAAAAGAATGGGACCAATTTCCGAAATTTGAAGATTGGCTGCCTGAATTCCCTGAATGGG
CAAGAGAGTGGTTGAAATTAGCTCTCAAACGTTCAGGCAAATATAACGTTTACGATCCCC
TCGCCCTAGATTTGGACGGCGACGGTATAGAAACCGTTGCCACCAAAGGCTTTTCAGGCA
GCTTATTTGATCACACCAACAACGGCATCCGCACCGCCACGGGCTGGATTGCTGCATATG
ACGGTTTTCCTGTGCGCAAATTAAACAGTAACGGGGGCATTATTAGCACGACAGATACCA
~~TATTGGAATGTTTGGATACATGGCTTGATCATCAACCAAGATGATATTTCCCAAGCACAG~~
CATGATGCATGCCATTGAAAAATATAGAAAATTAATTGAAAGCTTAAATGGATATTGAAA
TGAATGATCACATAGTACAAATTATAAGAAGGTTCGGGCTAGGTAGGATATTTTTTTATT
CGTATAGCAAATCATCTATAATAATTTTTTCTTCGTATGTTGTTTATTATATAATTTACA
ATTATCAATTTAATTACCTTTCGCTTTTAATTTATTTATTACCAATATTGTGCAGTATAT
ATATGTTTATATTTTTTTTAGGGAAAACTAAGGATACATTAACGACAGAGCGAAGAAAAA
AATTTTTTAATTCTATTTTTCCACTTAGAATTCTAATGATAATAGGTTCTGAGAAAAAGA
GGTTAGGCATCGGTAGTTTTTATTTGCTAAACCTACTATGGATTATTTGGTGTCTTATGA
TTCATAGAGAACAAGTCCCATTAAATAACTTAACCCTCCTATTATCCTTCATATTTTCAT
TATTTTTTTTATTATGTGATTTTGTTTTATTATTGAATGTTTATGTTTATTTTTTTAAAT
TAAGAGAGGCTTAATATGGTTAATCAAATCAAATCTGATAATAATTCAGTTTCTATTGAA
TTTATATAAGATTTTATAACTGCAAGTACGGATGTAATTAATCTGAGTTACGAAAATTTT
CGTAAAAATTTTTATACACAAATGTCAACTGATTCTACCAATTATGCAGCCAAACATGAA
AGTTTAGGAAAATCGGTACAACGTGAATTACAAAAAACACAAAGTCAGTTGAGACAAGTT
GTAAGAAAAATGCAGAGTAAATATAATATAAATAATAAAGCACGAGTAGCAGAAATATCT
TTGTTAAGGCAAATGCAAAGCCAATTTTCTCGAAAATATGTAAACAAAAATCTTGGTAAC
AGCAACACTTTGGCTCAACAAGGCAGCTACACCAAAAAAGACGGCACAACCGCGCAAGCA
GGCGATTTGCTGTTGGCTGCTGACAACCTGCACAGCCGCCTCACGGACAAAATGCTATCC
ATTAGCCATGTTCGGGAAAACACGATTTCCCCGTTTGTTTTAGGCTGTCTAAAACAAATA
ACCATAAATGCATATCATTATTTAAAATAAATAAAAGTATTTAACTATTTTTGACAAAAT
TTTAGAAATAGAGCTAGAGTTTTAGTTAAGTAGAAATTGATAGTGCTTCAAGGGAAGTAT
TCTCTATGTTTGCATTAAAGGGGGTCTGATAAAGCTATTATTCATTACTATGGACTTTTA
TTTCATTATTTTCAGGCGGAAATCTCATAGCCGTTTTGAATTTTTCTCTTCCTTATTAAT
TATACAAATAATTAGTATATTCTGATATGGATTTTTTGGAAATTTTTATTATGTCTGCAT
TTAGAAAAATATTATTAATAATATCTTGCCTATTGATTGCTAGCTGCAGTTTTGTTGAAA
CTATTTTTTATATGGCTATTAGCCCAGAACCTGTTGTGGTAGACTTTCCTCTTGGTAAAA

## Appendix A

AAACAAAAAGATCTATTGAACTCAAACAGAAAATTGGTAAACCTTATGCAATATCGTTAG
GAACTAATTTTATACATTATGATCCAAAACAGGGGGAGAGGTGGATTGATGATAAGTTAA
ACTATCCATATAATATATCGGTTAAAATATTTAAAGTGGAAGAAGATGGTAAAAAACTTA
TTATAGATGAGTTGCTTACAGAGAGAAGTAGAAAATTAGGAGGCGGAGTATTTGGAGCTG
GGGGAAAATACAGTATGCATATTTATGATTTTTATTTGCCGGAAGGGGAATATTTATTTG
AGATTTCTGATAATAGTGAATATATTCCACTTTACGATGAAATAAATAATTCTATAAGAA
TAGTAGTTAATGCACGAATTCAGTAAATTTTTCTAGAAATGTGGGGTTACTTATGGCTGA
TTATTATGCGATAACTGTAAAATTTGCGAAGCAGGGTACGCCACTGAAACAAGAGGGGGT
GTATCCAAGACGGGTACGTTTGGGTTGAACTGTATTCGGCTAGAGATAAAAAAATCGGGG
CTGTACTAGATTAGCCCTAAATTCCACACCAATCCCGCAGGATTTTAAGCTGTTGAGACG
GTGTGCCGAAGTTAAATCGAAATTCGCATTCTTTCAAGAACAGCGGGAAAGATTTACGAT
CGATTCCGTTGTATTTTCGCAAGACGCGTTTTGCCTGATTCCAAAAATTCTCAATGCCGT
TAATGTGGTTCTGACGGTCTGCAAATTCCTTGGAATGGTTGATGCGGTAATGGATAAAAC
CGCTCACGTCCAACTTGTCGCAGCTGCTCAGACTATCGGTATAAACAATACTGTCCGGCA
TGATTTTCTTTTTGATGACAGGGAGTAACGTTTCAGACTTGGCATTATCTACGACAACGG
TATAGCCCCGTCCGTTGCGTTTCAGAATGCCGAAGACAACCACTTTTCCTGCCGCACCGC
GACCACGTCTGCCTTTACGCCGTCCGCCGAAATCGCTTTCGTCCGGCTCGACAGGGCCCT
CAAAAACCTCATCGGCAGCCAAGGCCAAATGATGGTTGATAACCGTGCGGATTTTACGGT
AGAACAGTACTGCCGAATTGGGATGGATACCCAAAATATCGGCGGCAGAACGGGCGGTAA
CTTCCAGCACAAAAAAACGGAGCAGTTCTTTCTGTACTTTTTTCTTTAATTTGCAGTGCG
TTATCTTCATATTTCGAGGGTAACATATCTGCTAATCTAGTACAGCCCCAAAAATATACC
AAAAACAGCAAAACAAATTGTAAGGATAGGTATAGGCTTTGTAAAGGTAAATTGTGAAAA
AAGCAGTTTTTAAACGAATGAAACGGCTTCGGGCTGAAATATATGCTGATGCCCTGTCC
TTCCCGTATATCTTGTGTGTTGTCAAAGTGCAGGCTGCTTTGAAATCGGTATTGCCATCT
ATGAACCACCACTTTGTTTTATTTCAGCGGGCTTGAGATGTGTATAAGAATATTGTTTTG
AATAAATTTAAAAAAATGATAATCGTTATTGACGATTTTTAAAGGAAAGCGTAGAGTGCC
AATTCTATGAAGCAATACGGTAAGTAACAATGAAAATATCTACTGCTTGGGTATAGAGCA
TATTTCACAACCCGTAACTATTCTTGCGGAAACAGAGAAAAAAGTTTCTCTTCTATCTTG
GATAAATATATTTACCCTCAGTTTAGTTAAGTATTGGAATTTATACCTAAGTAGTAAAAG
TTAGTAAATTATTTTTAACTAAAGAGTTAGTATCTACCATAATATATTCTTTAACTAATT
TCTAGGCTTGAAATTATGAGACCATATGCTACTACTATTTATCAACTTTTTATTTTGTTT
ATTGGGAGTGTTTTTACTATGACCTCATGTGAACCTGTGAATGAAAAGACAGATCAAAAA
GCAGTAAGTGCGCAACAGGCTAAAGAACAAACCAGTTTCAACAATCCCGAGCCAATGACA
GGATTTGAACATACGGTTACATTTGATTTTCAGGGCACCAAAATGGTTATCCCCTATGGC
TATCTTGCACGGTATACGCAAGACAATGCCACAAAATGGCTTTCCGACACGCCAGGGCAG
GATGCTTACTCCATTAATTTGATAGAGATTAGCGTCTATTACAAAAAAACCGACCAAGGC
TGGGTGCTCGAACCATACAACCAGCAAAACAAAGCGCACTTTATCCAATTTCTACGCGAC
GGTTTGGATAGCGTGGACGATATTGTTATCCGAAAAGATGCGTGTAGTTTAAGCACGACT
ATGGGAGAAAGATTGCTTACTTACGGGGTTAAAAAAATGCCATCTGCCTATCCTGAATAC
GAGGCTTATGAAGATAAAAGACATATTCCTGAAAATCCATATTTTCATGAATTTTACTAT
ATTAAAAAAGGAGAAAATCCGGCGATTATTACTCATCGGAACTATCATAGGTATGGAGAG
AACGATTACAGCACTAGCGTAGGTTCCTGTATTAACGGTTTCACGGTACGGTATTACCCG
TTTATTCGGGAAAAGCAGCAGCTCACACAGCAGGAGTTGGTAGGTTATCACCAACAAGTA
GAGCAATTGGTACAGAGTTTTGTAAACAATCCAAGTAAAAAATAATGGGGCTGTCCTAGA
TAACTAGGATAAACTCGATTTTACTAATTGTTTTAAAATGGAACAAGAACTTTTATCTCA
CTGTTGTTAAAACGCCATTCGCACTCCTTTAAATACAGCTCAAAATGCGCTTTGGGAATG
CCGTTAAACTTGCGTAAATGACGTTTTGCCTGGTTCCAAAAGTTCTCAATTCCATTAATA
TGGTTTTGTCGTTCAGCAAAATGTGTGCTGTGATTGATACGAAAACGAAGTTTCAGCGAA
GCTAAAATGGCTAAATTCGCGCACATCTAATACATCATAGCTACGATAACAATCCGTATA
AATAATGCTGTCAGGTTTCACTTGTTCACGGATAATAGGAAATAAAGTAGCGGTTTGAGT
ATTCGGTACTGTAACCGTATAAACCTTACCATTTCGCTTCAAAAGACCGAATACGGCGAC
TTTACCGGCAGCACCGCGACCGCGTTTGCCTTTGCGTTGTCCGCCAAAATAACTTTCATC
TGCTTCTACTTCGCCATCAAACATTTCCAAATGCGGACTGTTTTGATAAATAAGTAATCG
TAAACGATGAAAATAATAGGCTGCGGTATTTTTATTAACGCCTACTAACTCTGCTGCCGT
TCTTGCAGTTACACCTGTGACAAATAGCTCAATGAGTTTATTTTGTTTATACTGGCTTAG
ACGACTTTTTCTCATAGGGATAATTCTAACTTAATTTGAATTTCCCTAGTTATCTAGGAC
AGCCCCTATTCTTTAACTAATTTCTAAGCTTGAAATTATGAGACCATATGCTACTACCAT
TTATCAACTTTTTATTTTGTTTATTGGGAGTGTTTTTACTATGACCTCATGTGAACCTGT
TAATGAACAAACCAGTTTCAACAATCCCGAGCCAATGACAGGATTTGAACATACGGTTAC
ATTTGATTTTCAGGGCACCAAAATGGTTATCCCCTATGGCTATCTTGCACGGTATACGCA
AGACAATGCCACAAAATGGCTTTCCGACACGCCAGGGCAGGATGCTTACTCCATTAATTT
GATAGAGATTAGCGTCTATTACAAAAAAACCGACCAAGGCTGGGTGCTCGAACCATACAA
CCAGCAGAACAAAGCACACTTTATTCAATTTCTACGCGATGGTTTGGATAGCGTGGACGA
TATTGTTATCCGAAAAGATGCGTGTAGTTTAAGCACGACTATGGGAGAAAGATTGCTTAC
TTACGGGGTTAAAAAAATGCCATCTGCCTATCCTGAATACGAGGCTTATGAAGATAAAAG
ACATATTCCTGAAAATCCATATTTTCATGAATTTTACTATATTAAAAAAGGAGAAAATCC
GGCGATTATTACTCATTGGAATAATCGAGTAAACCAGGCTGAAGAAGATAATTATAGCAC
TAGCGTAGGTTCCTGTATTAACGGTTTCACGGTACAGTATTACCCGTTTATTCGGGAAAA
GCAGCAGCTCACACAGCAGGAGTTGGTAGGTTATCACCAACAAGTAGAGCAATTGGTACA
GAGTTTTGTAAACAATTCAAGTAAAAAATAATTTAAAGGATCTTATTATGAATGAGGGTG
AAGTTGTTTTAACACCAGAACAAATCCAAACCTTGCGTGGTTATGCTTCCCGTGGCGATA
CCTATGGCGGTTGGCGTTATTTGGCTAATTTGGGTGACCGTTATGCGGATGATGCTGCTG
CAATTGTCGGTAAGGATGCAAACTTAAATGGTTTGAATTTATGGATGAAAAAAGGTGTGG
AAAACCTATGGGATGATACGGTCGGTAAAAAGACCCGTTTAGAGAAATTTGATCGGGTTG
CACTGCAACATTTCAGGCAATATGCGCGTCTAATTAATCAAAATAATGGTAGATTACCCA

## Appendix A

ATACTAGTGAAATTGAGAGAAGTTACTATAAAGCCGTTACCGATAATGGCGTTTCTTCCA
GTGCAGCTATTGATTTAGTTATTAATCGTTCACTTCCGGATATGGCGGATGGTTATTGGG
CATTAGGTTTGGGGATAGAAGCCGAACGTATCCACAATGAGCAAGCAGTAAATAATCCGA
ACGGTAGCGAAAGGGATAATAGAAAGCAGTTAATATCTGCTTTAGATAAAGGATTTGATG
GATCTTTTAAAGAGAAGCATTTTACTTTTTTACAATCTGTGATGATGGATGTAACAAAGT
TAGGTGTTGAATATACAATAGATGGTTGGCAAAAAATTGGAGGTTGGGGTAATGGGATAA
TCAATGATTTATATAAAAGTGTTGTAAAAAGAGAGTGGACTGGAATATTTGAGATCGTTA
ATAATAACATCAAGCAAGGAAATGAAGCTTTTAAAAATGAAATCAATAGCTTGGTTCATG
ATATGAAAGCTGCTGGCAAGGAATTTGGAGATGACTTAAATACACAGTGGAATAATCTCA
CTCAGGCTGCCGAAATAATCTATAATGACATAGTAGACAATACTAGTCAAGGAATAGAAA
AAGGTGTCAAAGCCATTAAAGAATTGTCTGAAAAAATGAAAAATGCTGCTTCCGATTTGG
CTGACGGTTCAGCAGAGAAAGCTAAACAAGTAGTGGAAGATTTGGCTCAAGCCGCCAAAG
AAGCATACGAAAATGCCAAATCCACAGCCGAGAAGGCTGCTCAAGCAGCTCGAGAATTTT
TTAAGGGCTTGCCCAGTTTTAAAGATCTGGCCGAAAAATTTAGAGATCTGTTCCCAAATC
CGGAAGGCTGGATCGATGATGGTCACCAATGTTTAGCTCCTTGGGTTAAAGAAACTAAAA
AACGCAATGGCAAATATCATGTCTACGACCCCCTTGCCCTAGACCTAGATGGCGACGGTA
TAGAAACCGTTGCCACCAAAGGCTTTGCAGGCAGCTTATTTGATCACACCAACAACGGTA
TCCGCACCGCCACCGGTTGGGTTTCTGCCGATGACGGTTTACTCGTCCGCGATTTGAACG
GCAACGGCATCATCGACAACGGTGCGGAACTCTTCGGCGACAACACCAAACTGGCAGACG
GTTCTTTTGCCAAACACGGCTACGCGGCTTTGGCCGAATTGGATTCAAACGGCGACAACA
TCATCAACGCGGCAGACGCCGCATTCCAAACCCTGCGTGTATGGCAGGATCTCAATCAGG
ACGGCATTTCCCAAGCTAATGAATTGCGTACCCTTGAAGAATTGGGTATCCAATCTTTGG
ATCTCGCCTATAAAGATGTAAATAAAAATCTCGGTAACGGTAACACTTTGGCTCAGCAAG
GCAGCTATACCAAAACAGACGGTACAACCGCAAAAATGGGGGATTTACTTTTAGCAGCCG
ACAATCTGCACAGCCGCTTCAAAGACAAAGTGGAACTCACTGCCGAACAGGCAAAAGCCG
CCAATCTTGCGGGCATTGGCCGTCTGCGCGATTTGCGCGAAGCTGCCGCATTGTCCGGCG
ATTTGGCCAATATGCTGAAAGCTTATTCTGCCGCCGAAACTAAAGAAGCACAGTTGGCAT
TGTTAGATAATTTGATTCACAAATGGGCGGAAACCGATTCGAACTGGGGCAAAAAATCGC
CAATGCGACTTTCAACCGATTGGACGCAAACGGCTAATGAAGGTATTGCACTGACACCAT
CCCAAGTAGCACAACTAAAAAAGAACGCTTTAGTTTCCCTTTCTGATAAAGCTAAAGCAG
CTATTGACGCCGCCCGCGACCGCATTGCCGTGCTTGATGCCTACACGGGGCAGGATTCCA
ACACACTCTATTACATGAGCGAGGAAGATGCGCTTAATATCGTCAAAGTAACCAACGATA
CATACGACCATCTCGCCAAAAACATCTACCAAAACCTGTTGTTCCAAACCCGTTTGCAGC
CATATTTGAATCAAATCAGTTTCAAAATGGAAAATGATACGTTCACTTTGGATTTTAGTG
GTCTTGTTCAAGCATTTAACCATGTCAAAGAAACTAATCCGCAAAAAGCTTTTGTGGATT
TGGCCGAGATGCTTGCATATGGCGAACTTCGTTCTTGGTATGAAGGCCGAAGACTAATGA
CCGATTATGTGGAGGAGGCAAAAAAAGCAGGTAAATTTGAAGATTACCAGAAAGTGTTGG
GTCAGGAGACCGTTGCATTATTAGCTAAAACATCGGGTACGCAAGCAGATGATATCCTGC
AAAATGTAGGCTTTGGTCATAATAAAAATGTTTCTTTATATGGTAATGACGGCAACGACA
CTCTAATCGGCGGCGCCGGTAATGACTATTTGGAGGGCGGCAGCGGTTCGGATACTTATG
TCTTCGGCGAAGGCTTCGGTCAGGATACGGTCTATAATTACGACTACGCTACCGGACGCA
AAGACATCATCCGCTTTACCGACGGTATTACAGCCGATATGCTGACTTTTACCCGAGAGG
GCAACCATCTTCTTATCAAGGCAAAAGACGGCAGTGGACAAGTGACTGTTCAGTCCTATT
TCCAGAACGATGGCTCAGGTGCTTACCGTATCGATGAGATTCATTTCGATAACGGCAAAG
TACTGGATGTTGCCACTGTCAAAGAACTGGTACAGCAATCCACCGACGGTTCGGACAGAT
TGTATGCCTACCAATCCGGAAATACCTTAAATGGCGGATTGGGCGATGACTATCTGTACG
GTGCCGACGGGGATGACCTGCTGAATGGTGATGCAGGCAACGACAGTATCTACAGTGGCA
ATGGCAATGATACGCTCGATGGAGGAGAAGGCAACGACGCCCTGTACGGCTATAATGGTA
ACGATGCACTGAATGGTGGCGAAGGCAATGATCATTTGAACGGCGAAGACGGTAACGACA
CTCTGATCGGCGGTGCCGGTAATGATTACTTGGAGGGCGGCAGCGGTTCGGATACTTATG
TCTTCGGCAAAGGCTTCGGTCAGGATACGGTCTATAATTACGACTACGCTACCGGACGCA
AAGACATCATCCGCTTTACCGACGGTATTACAGCCGATATGCTGACTTTTACCCGAGAGG
GCAACCATCTTCTTATCAAGGCAAAAGACGGCAGTGGACAAGTGACTGTTCAGTACTATT
TCCAGAACGATGGCTCAGGAGCTTACCGTATCGACGAGATTCATTTCGATAACGGCAAAG
TACTGGATGTTGCCACTGTCAAAGAACTGGTACAGCAATCCACCGACGGTTCGGACAGAT
TGTATGCCTACCAATCCGGAAATACCTTAAATGGCGGATTGGGCGATGACTATCTGTACG
GTGCCGACGGGGATGACCTGCTGAATGGTGATGCAGGCAACGACAGTATCTACAGTGGCA
ATGGCAATGATACGCTCGATGGAGGAGAAGGCAACGACGCCCTGTACGGCTATAATGGTA
ACGATGCACTGAATGGTGGCGAAGGCAATGATCATTTGAACGGCGAAGACGGTAACGACA
CTCTAATCGGCGGTGCAGGCAATGATTACTTGGAGGGCGGCAGCGGTTCGGATACTTATG
TCTTCGGCAAAGGCTTCGGTCAGGATGCGGTCTATAATTACGACTACGCTACCGGACGCA
AAGACATCATCCGCTTTACCGACGGTATTACAGCCGATATGCTGACTTTTACCCGAGAGG
GCAACCATCTTCTTATCAAGGCAAAAGACGGCAGTGGACAAGTGACTGTTCAGTCCTATT
TCCAGAACGATGGCTCAGGTGCTTACCGTATCGATGAGATTCATTTCGATAACGGCAAAG
TACTGGATGTTGCCACTGTCAAAGAACTGGTACAGCAATCCACCGACGGTTCGGACAGAT
TGTATGCCTACCAATCCGGAAATACCTTAAATGGCGGATTGGGCGATGACTATCTGTACG
GTGCCGACGGGGATGACCTGCTGAATGGTGATGCAGGCAACGACAGTATCTACAGTGGCA
ATGGCAATGATACGCTCAATGGAGGAGAAGGCAACGACGCCCTGTACGGCTATAATGGTA
ACGATGCACTGAATGGTGGCGAAGGCAATGATCATTTGAACGGCGAAGATGGCAACGACA
CTCTAATCGGCGGTGCAGGCAATGATTACTTGGAGGGCGGCAGCGGTTCGGATACTTATG
TCTTCGGCAAAGGCTTCGGTCAGGATGCGGTCTATAATTACGACTACGCTACCGGACGCA
AAGACATCATCCGCTTTACCGACGGTATTACAGCCGATATGCTGACTTTTACCCGAGAGG
GCAACCATCTTCTTATCAAGGCAAAAGACGGCAGTGGACAAGTGACTGTTCAGTCCTATT
TCCAGAACGATGGCTCAGGTGCTTACCGTATCGATGAGATTCATTTCGATAACGGCAAAG
TACTGGATGTTGCCACTGTCAAAGAACTGGTACAGCAATCCACCGACGGTTCGGACAGAT

## Appendix A

TGTATGCCTACCAATCCGGAAGTACCTTAAATGGCGGATTGGGCGATGACTATCTGTACG
GTGCCGACGGGGATGACCTGCTGAATGGTGATGCAGGCAACGACAGTATCTACAGTGGCA
ATGGCAATGATACGCTCGATGGAGGAGAAGGCAACGACGCCCTGTACGGCTATAATGGTA
ACGATGCACTGAATGGTGGCGAAGGCAATGATCATTTGAACGGCGAAGACGGTAACGACA
CTCTGATCGGCGGTGCAGGCAATGATTACTTGGAGGGCGGCAGCGGTTCGGATACTTATG
TCTTCGGCGAAGGCTTCGGTCAGGATACGGTCTATAATTACCATGTGGATAAAAACTCTG
ACACTATGCACTTTAAAGGATTTAAAGCAGCAGATGTTCATTTTATCCGTTCCGGAAGTG
ATTTGGTGCTTAGCGCTTCTGAACAAGACAACGTACGTATTTCCGGATTTTTCTATGGTG
AAAACCATCGTGTAGATACATTTGTCTTTGATGATGCAGCTATCAGTAATCCAGATTTTG
CCAAGTATATTAATGCTGGCAATAATTTGGTACAGTCTATGTCTGTGTTCGGTTCTAATA
CTGCTGCGACAGGAGGAAATGTGGATGCCAATATACAATCCGTACAGCAGCCGTTATTGG
TAACGCCATCTGCATAAGGAGCCTAATCACATTCATGGCTTAAACTGAAAAACAGCAATC
AAGTTTATTTTGATTGCTGTTTTTCTTAATATTGGGATAAGGGTCGTATTTTAATTAACC
TTAATCGGTGCACTTCTAGCAATATAGTGGATTCACAAAAACCAGTACAGCGTTGCCTCG
CCTTACCGTACTATCTGTACTGTCTGCGGCTTTGTCGCCTTGTCCTGATTTTTGTTAATC
CACTATAATTAATATGACTTTGCGGCCGTTTTGCCATTGCGTAATAAAACGATGGGGAAG
TGATGATAAAACGTGTGTGTAACTATATCAGACGGCATTGTTTTTCTGTTTGACGGCCTC
AATCCAAAATTTTGCCGACGATTTCGCCCACGTCTTTCGACAATCCTTCCTGCGCCCGAA
TGCGCTGCAATGCTTGTTTCACCAAGTTTTTGCGGTGCGGCTCGAGCTTGTTGCAGAGGT
TGAACGCCTGCACTAAGCGGGCGGCGACCTGCGGGTTGAAGCGGTCGATTTCGATGACTT
TGTCGGCGATGAAGCGGTAGCCGCTGCCGTCTTCTGCGTGGAAATGCGGGACGTTGCGGC
TGAAGCTGCCGATGAGCGAACGGGCTTTGTTGGGGTTTTCGAGGCTGAATTTCGGATGCT
GCAAGGCGGTTCGAACCTGTTGCAGGGTGTCGCTGCGGCGGCTTGAGCCGACGAGGGCAA
AATATTTGTCCATCACCAGCGCGTCGTCTGAAAACTTGTCGGCAAACTGCGCCAGCAGGC
GGTTGCGCGTATCGCTTTCGTTGCCGTTGACGGCGGACAGGATGCCCCATTCGTGGGTCA
TGTTTTGCGCCATTTCGCCGTATTTTTCGGCAACGGTTTCGATGTGCGCGGGGTCGGCGC
GCAGGACAAAGGCGCGGCAGACGTTGCGCAGCGTGCGCCAGCCGGCGGCTTCGGGGCTGT
ATTCGTAGCTTTGGTTTTCCTGCTTCGCCGCCTGACGGTTCAATTCGTGCCATTTCGGCA
GGAAGTGGACGGCAAGCGTATCCAACAAGGCTTCGCGCGCCTGATGGTAGCGCAGCGGGT
CGATGTTTTCTGCGCCGTCCCACAGCTCGGCTTCGGATGGCACGCCCAAAAGCAGGGCTT
TGAAGGCGTTGTCTAAGAGGTCGTCTGAAATGACTTTTTCGACGGCGGCAAGCAGTTTTT
CGTGTTTCGGCAGCTCAACGCCGTCTGAAAGCGTGGCAAGGTTGGCGGCGACGGCGCGGC
GGTAGAGCGTTTGGGCGGCTTCCCAGCGCGTGAAGGCGTCGCTGTCATGGGCGAGCAGGA
GCAGCAGGTCGTCGTCGCTGTACGGATAGTTCAGATGCACCGGCGCGCTGAACCCGCGCA
GCAGCGAGGGAACGACGGCTTCGGTTACGCCTTCGAGCAGGAAGGTCTGTTCGGCTTCGG
TCAGCAGCAACACGGCTTCGGTCGCGCGTTTGCCCTGATAGTCGAATGCCACCGCTTCGC
CGTTGCGGTTCAGCAGCCCGACCTTGACGGGAATCATCATCGGCTGTTTATCCGTCATAT
CGGGCGTGGGCGGCACGGTTTGTTTGACGGTCAACTCGAAAATATTGTTTTTCAGACGAC
CTTCCGCTTCCAAAACGGGCGTGCCCGCCTGGCTGTACCACAAGGCGAACTGGTCGAGAT
TGATGCCGTTCGCGTCCGCCATCGCCGCGCGGAAATCGTCGCAGGTAACGGCCTGTCCGT
CGTGGCGTTGGAAATAGAGCTTCATGCCTTTCTGGAAGCCCTCTTCGCCGAGCAGGGTGT
GATACATCCGCACTACTTCCGCGCCTTTTTCATAAACGGTCATGGTGTAGAAATTGTTCA
TCTCCTCATAGCTGGCGGGGCGCACCGGATGGGCGGTCGGGCCTGCGTCTTCGGGGAACT
GGTGCTGGCGCAGCAGGCGGATGTTTTCGATGCGGCGCACGGCGCGGCTGGCGCGGTCGC
CGGAAAATTCTTGGTCGCGGAACACGGTCAGCCCTTCCTTCAGCGAAAGCTGGAACCAGT
CGCGGCAGGTTACGCGGTTGCCCGTCCAGTTGTGGAAATACTCGTGTCCGACCACGGATT
CGATGCCTTCGAAATCGGTATCGGTGGCGGTGCGGCTGTCGGCAAGGACGAACTTGGTGT
TAAAGATGTTCAAACCCTTGTTTTCCATCGCGCCCATATTGAAATCGCCCACGGCGACGA
CCATGAAAATATCCAAGTCGTATTCCAAACCGAAGCGCGTTTCGTCCCATTTCATCGCGT
TTTTCAACGATTCCACGGCAAAGCCGACCTTGGGCTTGTCCGCTTCGGTGGTGTAAAACT
CGATTTTGACGTTTCTGCCGCTCATGGTGGTGAAATAGTCTTCCGTTACCGCCAAATCGC
CCGCGACCAAAGCAAACAGATAGCTCGGTTTGGAAAACGGGTCTTCCCATTTCACCCAAT
GGCGGCCGTCTGAAAACTCGCCGCCGTCGATTTTGTTGCCGTTGGAAAGCAAAACGGGAT
AGCGTTTTTTGTCGGCGACGATGGTGGTGGTGAACTTGGACATCACATCCGGACGGTCGA
TGTAAAATGTGATTTTGCGGAAGCCCTCCGGCTCGCACTGGGTAAACAAATTGCCGCCGG
AAGCATACAGCCCCATCAGCGATTTGTTTTCCGCCGGCAGGATTTCGGTTTCCACTTCGA
CGGTGAAGCGTTCGGACGGCACGCCCGCAATCGTCAGCGTCTCTCCTTCCAACACATAAT
CCGCCGCCGCCCCGTTGATTTTGACGGACAAGAGTTTCGCCGAACCGTCCAACACCAGCG
GCTCCCCTACCCTCTGCGGCTCAACCGTCAAACGCGACTTCACGACGGTTTGCGGTTCAT
TAATATCAAAATGTAAATCGGTTTTGAGAATATGGTAGGCGGGCGTTTGATAGTCTTTGA
GATAATGCACGGTTTTGCTCATTTTTTTCTTTCAATGTTATTTTGTTTGACTGGAAAAGG
CTTCAGACGGCACGGGCGCATCCCGCGTATGCCGTCTGAAGCCGCAGCGGCGGCACGGGC
GCGCCGCCGGACAACCGGTTTGAATTCAATCTTTATTCCCACGCGCGGACAAACTCTTCC
CAATGCGGCTTTTCCCCGGCTTGTGCGGACAGGTAATTCCGCATCCGTTTGATTTCCATT
TCGTATTCGTCCGCATCCAGCCTGCCGCTGACCAGACAGAAACGCAGGTACATCAGATAA
GTGTTTGCCGCGTCGGTTTCGCAATAATTGCGGATTTCCTTCAGCCTGCCCGTATGGAAT
GCCTCCCAAACCTTGCTGCCGTCCATACCCAGCTTGCCCGGAAAACCGCACAGTTTCGCC
ATATCGTCCAGCGGCACGTTTGCCCTCGGCTGGTAAAGCGCGAGCAAATCCATCAAATCG
CAGTGGCGTTGGTGATAACGGCTGATGTAGTTGTTCCACTTGAAATCGCGGCTGTCGCCG
AAATCGCCGTCGCCCATATCCCAATAGCGCGGCGTTGATGCCGTATATCAGGGAGCGG
TAATGCAGTACGGGCAGATCGAAACCGCCGCCGTTCCAACTGACCAGTTGCGGCGTATGT
TTTTCAATCAATTCGAAAAATTTAGCAATGACCACTTCCTCGCCGTCATCCATCTCGCCG
ATGGTGCCGACATGTACTTTATCCTGCCCCCAACGCATGCAGCACGAAATCGCCACAACC
TGATGAAGATGATGCTGCATAAAATCGCCGCCCGTCTGAGCACGGCGTTTTTGCTGGGCA
AACAGCACCACTTCATCGTCGGGCAGCGAGGACGGCAGCTCGTACAATGTTCGGATACCC

# Appendix A

TGCACATCGGGTACGGTTTCAATATCGAAAGCCAAAATCGTGGTCATGACAGCACCTTGT
ATTTAAAACGGATGCACCTATTGTGTCATTAAAAGGCGGATAAAAAAAGAGGCAACCCCC
CACAGGATTGCCCCAATACCTCAAATCAGAGATTTACGCTTCACAAACAATACAGGCTTT
CGCCTGCGGCTTTACCCGCGTAGCTCAACTCTACGCCGGCAAACTTTCGTTTCACCGTTT
CCGATGAAACCCCGACCAATCGCAAGACTGACCGGAAAATCCTTTCAGACGGCATTTCCT
GCCTGTCGTGTAATTCCATGTAGCGAAATGTACGCCATTTTCTACGCTTTGCCAAGCATT
TTTTACAATATAAATGTCAAAACATTAATTTTATAAAATTGCTGAAAATATTAAATATAT
GGATTTTTATTTTTTATATTTCAATAAATATAAATTTAATTTTGATTTATATTTAAATTT
AAGCATAAAATGTCAAATATTAAAGTAAACATGAAAGGCATATATTAAATATTTATTTAT
AACGCTATGTTTTTAAAGAAAATTAATTTTAATATATTAACTAGATTGTCTGCATATATT
CATAGGTTTGCGGTATTTCTTCCAAAACCTGCTTCGAATTTCCCGACCAAGTCTTAAAAA
TATTGTTTTTGAGATACTTAAATAGCAGCGATTATCAAATGAAATCTGTTCATATAATCT
GCCATTTTGCATTTAAAAAAACAATCAGGAGTTTCGACTCGAAACGCCTGATATGTTTTG
TAATTTTACGTAGTCAGTAAAAATCGGGGCTGCCTTCCGGACGGGTTTTAAAACGCTTGT
GCAGCCAAAAATATTGTTCCGGATGTTCGCGCACCCTGTCTTCGATAAAACGGTTCATGC
GCTGCGCGTCGGCTTTCGCGTCTTCACCCGGAAAGGATTTCCAAGCAGGGTAGAAATGCA
ATGTAACCGTATTGTCTGCCTCGCGGACGGGAATGGCGGGTATCACTTTTGCATTTGCAA
GCGCGGCAATGCGGCTCAATCCGGTAATCGTTGCCGTCTGAATACCGAAAAAATCCACAA
AAACCGAATCGTTGCGTCCGAAATCCTGATCGGGCAGATACAGAAACGGCGCGCTGCTTT
TGCGGAACTGTTTGACGAGGGCGCGCAGCCCTTCGGTGCGCCCGATAAGGAAGACGTTGT
GATAGCGGTTGCGGCCTTTCAAAATCTGTTCGTCCAATATCTTGTTTTTTTGATGGGAAT
ACATACTGATCAGCGGGATATCCTGATTAAGCGCGTACACCGCCATCTCGAACGCGGTGA
AGTGCGGATACAGGATGATGACTTTTTCCCCCGCCGCCAGCGCGTCGTCCAAATAATGCT
TATTGCGGTAGCGCACCAGCGATTTCAAACGTCCGGCAGGCGCGTACCAATATAAACCGT
ATTCCAACATCAGTTTCGCCATGTGTTTGAAATGCTGTTTCAACACGGTTTTACGCTTTT
CCTCACTCCATTCGGAAAAACATTTTGCCAAATTGATTTCGCCGATACGGCGGCGCGGTT
TGACCAGAAGGTAGGCAAGCAAACCCGTCAGGTCGGCAATCTTGTGCAGCAGCGCAAACG
GCAGAAACTGCAAAACATACAGTACAAAAAATATAAATTTCATCTCGATACACATTTTCT
TTTCAGACGGCAAAATACAAATGCCGTCTGAAACTATTGAAACCTGCCGCGCTTGACCTG
CATCCCCGAAGGATTGAGTTTGGCGGCAAGCCCGTGGTTGCGTAAGGCGTGGGTCAGCGC
GACGGCAAGACCGTCCGCCGCATCCGGCTGGGGCGTTCCCGAAAGTCCCAACATCTGCAC
CACCATATGCTGCACCTGTTCTTTTGCCGCCTTGCCCTTGCCGACTACCGCCTGTTTGAC
CTGCAAGGCCGTGTATTCCGAAACGGGCAGCTTATGGCTGACCAATGCCGCCAATGCCGC
GCCCCTAGCCTGACCGAGCATCAGCGTCGATGCCGGATTGACGTTGACGAACACCTGTTC
CACTGCCGCCTGTTGAGGCTTGTAAACGGTAACGACTTCGCCGATGTGCCGGACGATGAC
GGCAATCCTGTCTGCCAGAGGCGCATCGGCAGGCGTTTTGATGCAGCCGGAGGCGACGTA
AAAATGATCCCGCCCCCTGACATCGATGACACCGAAACCCGTTACGCGACTGCCCGGGTC
GATGCCTAAGATACGGACGCTTGCAGCCATATTCACAACAAACCGTGTTGAATCAGCTTC
TTACGCAGGGTATTGCGGTTCAGCCCCAGCATCACGGATGCTTTGGACTGGTTGCCGCCG
CATTGCTCCATCACGCACACCAGCAGCGGTTTTTCCACCTGATGCAATACCATATCGTAC
ACGCCGCAAGGTTCGGTACCGTTCAGGTCTTTGAAATATTGTTCTAAATTTTGTCTGATG
CATTGGGAAATATCGGGAAGGGTATGGGGCATGATTGCACTTTCAAAGGATAATCAAGTG
TTCAGAAGGCATTTGGGCGGTAGGCGCACGCCCAACTGTCGGTTTTTTCGGCAAGTCTTT
CAAGATAACCTGCAAGCATGTCGTATTGCGCCGCCGCACTGTCCAAGCGGTTGATTTCAC
GACGTGTCTGTTCGCCGTCGGGCATTTCGTCGATGTACCAGCCTATGTGTTTGCGTGCGA
TGCGCACACCGGCGGTGTCGCCGTAAAACGCGTGTATGGCGCGGATGTGGTTCAAAATAG
CGGCGGCGCATTCTGCCAAACTCAAGGCAGGCGGCAAAACACCGTGTTCGGCATAATGTT
TCAAATCGCGGAAGAACCACGGCCTGCCTTGCGCGCCGCGCCCTATCATAATGCCGTCGG
CGGCGGTTTGTTTGAGGACGGCTTGGGCTTTTTGCGGCGAAGTAATGTCGCCGTTGACCC
AGACCGGGATGTTCAGACGGCATTTGGTTTCGGCGATGAGTTCGTAACGCGCTTCGCCTT
TGTACATTTGCGTACGCGTGCGTCCGTGGACGGCAAGGGCGGCGATGCCGCAATCTTCGG
CGATTTTGGCGATGACGGGCAGGTTTTGATGGTCGTCGTGCCAACCCAAACGGGTTTTGA
GGGTAACGGGTACGCCTGCCGCACGGACGACGGCTTCCAAAATGGCGGCAACCAGCGGCT
CGTTCTGCATCAGCGCGCTACCGGCTTGGACATTGCAGACTTTTTTAGCGGGACAGCCCA
TGTTGATGTCGATAAGCTGCGCCCCAAGGCTGACGTTGTAACGCGCGGCATCCGCCATCT
GCTGCGGATCGCTTCCGGCAATCTGCACGGCAACAATGCCGCCTTCATCGGCAAAATCGC
TGCGGTGCAAGGTTTTTCTAGTATTTCTGAGCGTCGGGTCGCTGGTCAGCATTTCGCACA
CCGCCCAACCTGCGCCAAAATCTCGGCAAAGTCGGCGGAACGGTTTGTCGGTAATGCCCG
CCATCGGCGCAAGTGCGATGGGGTTGTCGATAAAATAGCCGCCGATGTGCATAATGGATC
CGCGTTTCAAAAAAGTACGCCATTGTACATTTTTTAAGCAGGATTTCCAATCTCCGGACG
CGCCCGCGATTGGGTCGGACACCGTTTTATGGCATAATCCGCACACAGATTCCCTGCCCC
GCCACTCACAGGCGGGCAGTTTATAGTGGATTAACAAAAACCAGTACGGCGTTGCCTCGC
CTTAGCTCAAAGAGAACGATTCTCTAAGGTGCTGAAGCACCAAGTGAATCGGTTCCGTAC
TATCTGTACTGTCTGCGGCTCGCCGCCTTGTCCTGATTTTTGTTAATCCACTATATTTCC
CCGTCCTATCGGTTTCCCGTTTCAGACGACATAAGGTCTGAAAGAAAGACTACAATTATG
AGTAATCCATTTTCCTCTTTAGGTTTGGGTACGGAACTCGTTTCCGCACTGACCGCGCAA
GGTTACGAAAACCCGACGCCCATCCAAGCCGCCGCCATTCCCAAAGCACTCGCCGGTCAT
GATTTGCTAGCCGCCGCGCAAACCGGCACAGGCAAAACCGCCGCCTTTATGCTGCCCAGT
CTGGAACGCCTCAAACGTTACGCCACCGCCAGCACCTCGCCCGCGATGCACCCCGTGCGT
ATGCTCGTCCTCACCCCCACGCGCGAACTTGCCGACCAAATCGACCAAAACGTGCAGGGC
TACATCAAAAACCTGCCGCTGCGCCACACCGTCTTGTTCGGCGGTATGAATATGGACAAA
CAGACCGCCGACCTGCGTGCCGGCTGCGAAATCGTCGTCGCCACCGTCGGACGGCTGCTC
GACCACGTGAAACAGAAAAACATCCATTTGAACAAAGTCGAAATCGTCGTTTTGGACGAA
GCCGACCGTATGCTGGATATGGGTTTTATCGACGACATCCGCAAAATCATGCAGATGCTG
CCCCGCCAACGCCAAACCCTGCTCTTTTCCGCCACCTTCTCCGCCCCGATACGCAAACTG

## Appendix A

GCGCAAGACTTCATGAACGCGCCCGAAACCGTCGAAGTCGCCGCGCAAAACACCACCAAC
GCCAACGTCGAGCAGCACATCATCGCCGTCGATACCATTCAGAAGCGCAACCTGCTCGAA
CGGCTGATTGTCGATTTGCATATGAACCAGGTCATCGTGTTCTGCAAAACCAAACAAAGC
GTCGACCGCGTAACGCGCGAACTGGTGCGCCGCAACCTGTCCGCACAGGCGATACACGGC
GACCGTTCCCAACAAAGCCGGCTCGAAACACTCAACGCCTTCAAAGACGGCAACCTGCGC
GTCCTCGTCGCCACCGACATCGCCGCGCGCGGGCTGGACATTGCCGAACTGCCCTTCGTC
ATCAATTACGAAATGCCCGCCCAGCCCGAAGACTACGTCCACCGCATCGGGCGCACGGGG
CGCGCGGGCGCGGACGGCGTGGCGATTTCCCTGATGGACGAATCCGAACAGAAAATGTTT
GAATCCATTAAAGAGCTGACCGGCAACAAGCTGCTCATCGAGCGCATCGAGGGCTTCGAG
CCGCAATGGTGGGAACAGGGCGGCGCAAAACCGGAAAAACCCGAAATGCGCGAACCGAGA
CAACGCAACCGCTACGAATCCGCCAAAGCGCAACGCGAAAAAAACACCCGGCCGGAAAAT
GCGGCAAACGATGCGGGCGCGGCTTGCGGAAAAATTGCCGGACGCAGCCGCCGAAGCCGC
CGGGAACACCGGACGTGCGCCCTGCTCCAACCGCGTTACGGCGTAAAATAGCCCTGAAAA
TCAAATGCCGTCTGAACATTTCCCGTTTCAGACGGCATTTTTCAAACCGGACTGACGCAT
CGGGAGCAACCGCCCGCACCGGATAAATTTCTGCCGCAAACAGTTTCAGACGGCATTTGC
CGCCTGTACAATATAGTGGATTAACAAAAATTAGGACAAGGCGGCGAGCCGCAGACAGTA
CAAATAGTACGGAACCGATTCACTTGGTGCTTCAGCACCTTAGAGAATCGTTCTCTTTGA
GCTAAGGCGAGGCAACGCCGTACTGGTTTAAATTTAATCCATTAATAGTGTATATTAAGT
ACGTCTGATATACACGATACCCTACGAGGGTGTAAGCTTTAGTTCACATTTAAAATGACC
TCTTTAAACCTGTCTTTCGGCAGGTTTCTTTTTAGGTTGTTTGGAAATCGTGTGCAGACA
AGGTGTAAAATAGGTAACAGCATAAAATAATGCGGTTTTACCGCCCATATATTTACAAAA
GCCAAATTTTTAAACATATATCCTTGATATATACACGGCGTAAACATATACTGGAAACAT
CTTTAAATTTTCCGAAATTTTAAATATGAGCAACTGGAAACCCAATATTCCCTATAACGA
TTTACCACCCCTGCCGCCAAAACAGGATATTGAAAGCAAAACCATCCTGAAACGTTGTAT
AGCCGCCCGTGCATCCCTTGCCCGTTTAAAGCAGGCGGCAGAATTGATACCGAATCAAGC
CATGCTGATTAACACCCTTCCTGTTATGGAAGCCCGTGCAAGTTCGGAAATTGAAAACAT
CGTAACCACCACGGACAAGCTGTTTCAATCCCTGCAAATGGATACGGAACGGCAAGACCC
TGCCACGAAAGAAGCCCTGCAATACCGCACCGCCCTGTTTGCAGGCTATGAATCACTGAC
GAGCCGCCCTTTATGCACACAAACCGCCATCATGGTCTGCAACGCCATCAAGCACCCCTA
CGAAATGGCCATCCGCAAAACAGGCGGCACAGCCCTAAAAGGAGGCAACAGCGGAAATGT
TGTCTATACCCCGCCCGAAGGAGAAGAAACCATACGCGGCAAGCTGGCAAATTGGGAGCG
GTTTATTCACGAAAGCGGCGATTTAGACCCGCTTATCATCATGGCGGCGGCACATTACCA
ATTTGAAGCCATCCATCCGTTTACGGACGGCAACGGGCGGACGGGGCGCATATTGAACAG
CCTGCTATTGATTGAAAAAGGGCTTTTGGATTTGCCTATTTTGTATTTGAGCCGCTACAT
CATCGAAAACAGGGCGGACTATTACCGCCTGCTTTTAGGCGTAACCGAACGGCAGGACTG
GGAAAGCTGGATAATCTACATCTTAGACGGCGTAGCTGACACCGCCGATTGGACGGTATC
GAAAATAGATGCGATACGCCGCCTGTTCGAGCAGACACGGCAACACATACGGACACACGC
ACAAGGAATCTACACGCACGAACTGGTAAATCTTCTGTTTGAGCAGCCATATACACGCAT
TGCCAACCTAGAAGCGGCAGGGATAGCCAAACGGCAGACGGCCTCTAAGTACCTGAAAGA
GCTTTCAGACATAGGTGTGCTGCAAGAAATCGTCATCGGCAGGGACAAACTATTCATTCA
TCCGCGCCTAATGGAACTATTGCGGGGAGAGGGCAACAGCTTTACCTCATTCCAATCCCT
CGTTAAAGCATAGCCAAAATAATCAATAATCCGGAGGTCAATATGGCAAGAAGGTCAAAA
ACATTTGAAGAAGCTGCTGCTGAGGTTGAGGAACGTTTCGGTCATCGTGGCATTAAGTTG
GTCGAGTTTGAGGGTACAGCCAAGCCGTGTGTAATCAACTGCCCTAAACATGGAAACCAA
ACCTGTTCGAGGTACTCCAATATGTTCATAGGAAGTAGCTGGGGTTGCCCCTCTTGTGGT
AATGAGCAAGCTGCAAAAGCCGGTATAGCGACCCTTAGGAAGAATCACATAGCGTTAGAA
ATGCTGAAACAGGCTGTAACAGGTATGACCAAGCAAGAGCGCATCACGACGCAAGCCTAC
AATGAGATGACCAAATCCGTGGCAGGTTCAAACAGCATAGTCCTTAACGATGTCCAAGGC
GATACGACCATCAACAACCATCATACGCATACGCACAACCACAGCGATGCCGATGGCAAA
GCACTGTCGATGAGGCTCACACCCCGTCCTTTGTTGTCAGACCGTCAGGCGGCGGCTTTC
GCCCGTACAGGCAAACTCACGGGCAGTTTCGACCTGTTTGCTTCGGTGGTCGCCCCCTCG
CAGTACACGTTTGCCGTTGCCATGCCCGACACGTCCATGTCGCCGGTTATCGAAAAGGGA
GACTTGCTGGTGGTCGAGCCGCGTATGTGCCCTGCGGACGAAGACATCGCGCTGATTGAA
CTGTCCGACAAGCGGCTGGTCGTCGCGCACCTTGTTATCGATATTGCGGGCAGGATGCTG
ATTTATCAGACGGGCAGGCCGTCTGAAGCCTTTGACCTGCCCGAAGGCAGCACGATTTTA
GGTGTGGTGCTGGAGTCAAAAAACGGTTTATGTCCGCCGCACAGGCAAGAAGGCGTGTTG
ATTCGGATTACCGCCCCTGATGTGTGGACGGTTGGTATGATTTCCGCTTCCAAAACGTCG
TGTACGCGCCCGACCGCAGCCCGGAAATCAGCCGTATGCTTTCTTCGATTTTGGCAGGCT
ACGCGTGGGATACCGAAAACCCGTTCGTGGCGAAATCCGAACAACGCCTGACTGCCTTGT
CCGAATGGGTCGGTCAGTTGGAAACCGAATAAATCCGTACCGCCATACAAAATGCCGTCT
GAATCCAATCGGGTTCAGACGGCATTGCCATTTCAACTGTTTTTATGATTACTCGGGGCG
CATCTGCGGAAACAGAATCACATCGCGGATGGTTTGCGAATCGGTCAGCAGCATTACCAA
GCGGTCGATACCGATGCCGCAACCGCCGGTCGGCGGCAAACCGAATTCCATCGCGCGGAT
GTAGTCGGCATCGTAGTGCATGGCTTCGTCGTCGCCCGCGTCTTTTTGCACCACTTGCGC
TTTGAAGCGTTCGGCTTGGTCTTCGGGGTCGTTCAACTCGGAATAGCCGTTTGCCAGTTC
GCGGCCGACAACGAACAATTCGAAACGTTCGGTCAGACCTTGTTTGGTATCCGAAGCGCG
CGCCAACGGTGAAACTTCGACCGGGTAATCGACGATGAAGGTCGGATTCCACAGCTTGCC
CTCGGCGCAACCTTCAAACAGCGCGAGTTGCAGGCTGCCGATGCCCGGGGACGGCGGCAG
GCTTTCGCCGTGTTTGACGATTTCTTTTTTCAGCCATTCCGCATCGTTCAACTGCTCGTC
GGTGTAGTGCGGATTGTATTTTTTGATGGCTTCGAGAATGGTCAGGCGTTCAAACGGGCT
TTCCAAATCGACTTCTTTGCCGTTGTAAGTGATGTTTGCCGTGCCGTTTACCGTGCGCGA
TGCGTTGCGGATGATGTCTTCCGCCATCTGCATCATGCGTTCGTAGTCGGAGAAGGCTTC
GTAGAATTCGATCATGGTGAATTCGGGGTTGTGGCGCACGGACATGCCTTCGTTGCGGAA
GCTGCGGTTGATTTCAAACACGCGTTCCAAACCACCGACAACCAGGCGTTTCAAATACAG
CTCAGGCGCGATACGCAGGTAAAGCGGAATATCTAAGGCATTGTGATGGGTAACGAAGGG

## Appendix A

TTTTGCCGTCGCGCCGCCGGGAATCGGGTGCATCATCGGGGTTTCGACTTCGAGATAATG
CTCGCCCACCATAAAATTACGCACGGATTGGATGATTTGGCTGCGTTTGATAAAGGTATT
GCGCGATTCTTCATTGGCAATCAAATCAACATAGCGTTGGCGGTATTTGGTTTCCTGATC
GCTCAAACCTTTGTGTTTGTCGGGCAGCGGGCGTAGGGATTTGGACAGCAGGCGGATGCC
GGACACGCGTACGGTCAGTTCGCCGTGGTTGGTTTTGAACAAAGTGCCTTCCGCGCCGAC
GATGTCGCCCAAATCCCAATGGTTGAAGTCGTCCAAAACTTCTTGGCTCACGCCTTTGTT
GTTCAGATAAAGCTGGATTTGCCCGGACACGTCTTGAATGGTGGCAAAACTCGCCTTGCC
CATTTGACGCTTCAGCATCATGCGGCCGGCCACTTTGACGGGAATGCCTTGCGGATCGAG
TTCTTCTTTGCCGATTTCGCCGTATTGGGCGTGCAAATCGGCGGCGAAGCTGTCGCGTTT
GAAGTCGTTGGGATAGGCGTTGCGCTGTTGGCGGATGTTGTGCAGTTTTTCGCGGCGCAG
GGCGATGATTTGGTTTTCGTCCAACTGCGGCTCGGTTTGCGGATGGTTTTGTTCGCTCAT
AAGGTTTTCCGAAAAAATAAATCAGGCGCAATCTGTTTCAGACGACCTGACCGAATCACA
AAATTTGCGCATATTTTACGCGATGTCGGCATTTTTTTCCATAAACGCGACAATGCCGTC
TGAAAGCGGTTTGCGGTTTCAGACGGCATCGTTATCATTTGAACATTCCCGCCAAATTCA
ATAAGAACAAAACGGTAAAACCGGTCAGATAAATCAAGCCTGCCAATGCAAGGGCATTCA
TACCTGATGTGAGTTTGTGTTTTTCATCACCTTTAACCAAACGGTAATTCAGCCAGGCAA
ACACAGGGGCGGACACAAAAGCGGCAATCATCGCAAATTTGAGCAGATTCGCCATTACGC
CGTCAAACCAGAAAATCACCGCCAAACCGCTGCCCGCCACCCAAATATTCCAGGCAAAGA
ATTCGGCGTTGCCCGTTTTGTCTTTTCCGCGCAGCAGGCGCACGGGTTCGGCAATGGCAC
GGGCATAGCCGTCCACGACGGTAATCGTCGTGCCGTACATACAGGCAAACGCGATAAACG
CCACCAGCGGGCGCGACCAGCCGCCGATGGTAACGGCGTACATATTGATCAATTGCCCGA
TATATTTGCCGCCCGCCATCTGCACTGCTTCGCCGTTGCCGTATTGCACAAACGCGCCCA
GTGCAAGGAAAACCAAAGCCAAAACCGCACTGGCGATATAACCGACGTTGAAATCAAAAA
TCCCGTCGCGGTATTCGGAAGGATTGATGCGTTGTTTTTCGGTTACCCACAAAGAATTGA
TGGCGGAAATTTCAATCGGCGCGGGCATCCAGCCCATCAGCGCGATCAGGAAGCCCAAAC
CGGCAAGCGTCCACGGTGTCGGCTCGATAAAATCGGACTGCATCTGCATACCGCGCGACA
TAGCGATGCCGGCGGCGGCAAGCGTGGCGATACTCAAAGTAACGATGATGATTTTGGAAA
CGCGATCCAAAGCGCGGTAACGTCCGCTCACCAAAATAATCAGGCAGGATGCCATAATCA
AGGCGGCAACCGTGCCGGCATCAAACATCAGCGAGGGAATCGCCATTTTGACGATGGCGG
CGGTTACAATGGCGACCGCGCCCGCGTTAATCGTGGCGGAGAGGATGCACAAAATCAGGA
ATACCCACAAATAAACGCGGCTTTTCTCGGCATAACCTTCAATCAGGCTCTTGCCCGTGT
CCAGCGTGTAATGCGCGCTGAAGCGGAAAAACGGGTATTTGAAGAGGTTGGTCAGGATGA
TGATGAGCGCGATCTGCCAGCCGTAAAGCGCGCCCGCCTGCGTCGAGGCAATCAGGTGCG
AACCGCCGACCGCCGCCGAAGCCATCATGATCCCCGGACCCAATGCGTTGATTTTACTTT
TCCAAGTCGAAATATGTTGTTCGGACATAAAGTCTTCCGTATTTTTAACTGTGTTTCAAC
ACACAGAGCCGCATATTCGGACACAGCCCTATCTATTGCTCCAATTTGGGCGGGATTGCC
CCCAAACAAACCCAAATCCTACCGTCTTCAAAAACAGGATACCGCCCGGTAGGGAAATTT
TGATGAAAACACGTATTGTAACGTAATCCAAATACCTGCCAACACACACTATTAGAACTT
CATGCTCAAACTTGACTATATTTTCCATATTACTTCCAAAAAAAGGCATAAAACGACATT
TTATGCCTAAAATTTTACAACAAACAACCTTACATCGCTTTTTTCGCGCAAACACGCACC
ATCCGATCAGCCCGTCCGTTTTGCAGCAGGCTGGCGATTTGATAAGATGGTTATGTTTTT
CAGACGGCATTTCAGATTTCCGTCCATGCCATCTGAAGCCGCAAAACCCGATTGGAGGAA
CTGTTATGAATACCGTATCGAATTATCTGTCCGCATTACGCGAAGCCATGAAGGCGCAAG
GCTTGGATGCACTCGTCATCCCTTCCGCCGACCCCCACCTGTCCGAATACCTGCCCGAGC
ATTGGCAGGCGCGCCGCGAATTATCGGGCTTTACCGGCTCGGTCGGCACGTTTGTCCTGA
CCACCGATGAAGCGGGCGTGTGGGTGGACAGCCGCTATTGGGAACAAGCCGCCAAACAGC
TTGCGGGCAGCGGCATTGTGCTGCAAAAAAGCGGGCAAGTGCCGCCGTACAACGAATGGC
TCGCGGCAAGCCTGCCCGAAAACGCCGCCGTCGGCATCCCTTCCGATATGGTCTCGCTCA
CCGGCAAACGCACTTTGGCGCAATCACTCGCCGCCAAAAACATCCGCATCGAACACCCGG
ATAATTTACTGAATCAAGTGTGGACAAACCGCCCCGCCCTCCCCGCCGAAACGGTGTTCA
TCCACGACCCCGACTATGTTTCTGAAACCGCCGCCGAAAAACTCGCCCGCGTGCGCGCCG
TGATGGCGGAAAAAGGCGCGGATTACCACTTGGTTTCCTCGCTTGACGACATCGCCTGGC
TGACCAACCTGCGCGGCAGCGACGTGCCTTTCAATCCCGTTTTCGTGTCCTTCCTGCTGA
TTGGCAAAGACAACGCCGTCCTGTTTACCGACCGATGCCGTCTGAACGCCGAAGCCGCCG
CCGCGCTGCAAACCGCCGGCATCGCGGTCGAACCTTACGCCCAAGTTGCCGACAAACTCG
CGCAAATCGGCGGCGTGCTGCTCATCGAGCCGAACAAAACCGCCGTCAGCACGCTTGTGC
GCCTGCCCGAAAGCGTGCGCCTTATCGAGGGAATCAACCCATCCACGCTGTTCAAATCCT
GCAAATCCGAAGCCGACATCGCCCGCATCCGCGAAGCGATGGAACACGACGGCGCGGCGT
TGTGCGGTTTCTTCGCCGAGTTTGAAGACATCATCGGCAACGGCGGCAGCCTGACCGAAA
TCGACGTGGACACCATGCTTTATCGCCACCGCAGCGTGCGCCCAGGCTTCATTTCATTGA
GTTTCGACACCATCGCAGGCTTCAACGCCAACGGCGCACTGCCGCATTACAGCGCGACAC
CCGAAAGCCACAGCACCATCAGCGGCAACGGGCTTTTGCTCATCGACTCCGGCGCGCAAT
ACAAAGGCGGCACGACCGACATCACCCGCGTCGTCCCCGTCGGCACGCCGAGTGCCGAAC
AAAAAAGCGACAACACCCTCGTTCTCAAAGCCCATATCGCGCTTGCCGAAGCCGTGTTCC
CCGAAAACATCCCCTCGCCGCTGATTGATGCGATTTGCCGCAAACCCCTGTGGCAGGCGC
AATGCGACTACGGCCACGGCACCGGACACGGCGTAGGCTATTTCCTCAACGTCCACGAAG
GCCCGCAGCGCATCGCCTTCGCCGCCCCCGCCACGCCCGAAACCGCCATGAAAAAAGGCA
TGGTTACCTCCATCGAACCCGGACTCTACCGCCCGGGAAAATGGGGCATCCGCATTGAAA
ACCTTGCCGCCAACCAAGCCGTCGCCGCCCCTCAAGAAACCGAATTCGGCAGCTTCCTCT
GTTTTGAAACCCTGACCCTCTGCCCCATCGACACCCGCCTGATGGACACCGCCCTCATGA
CCGACGGCGAAATCGACTGGGTCAACCGCTACCACGCCGAAGTCCGCCGCCGCCTCGAGC
CGCTGACCGAAGGCGCGGCAAAAGCGTGGCTGATCAAACGCACCGAACCGCTGGCGCGTT
AAACAGCACGGCGCAAAAAATGCCGTCTGAAAGCCCTTCAGACGGCATTGGTTTCCCAAA
ACATCCCGCACCGTTTTCATCTTGCCGCAAGCAAATATAGTGGATTAACAAAAATCAGGA
CAAGGCGACGAAGCCGCAGACAGTACAAATAGTACGGAACCGATTCACTTGGTGCTTCAG

## Appendix A

CACCTTAGAGAATCGTTCTCTTTGAGCTAAGGCGAGGCAACGCCGTACTGGTTTTTGTTA
ATCCGCTATATTCCGCCATCTCTAAGATTTACAGCGATACACGGGTGATTTAAGGAATGC
CCGAACCGTCATTCCCGCCACTTTTCGTCATTCCCACGAAAGTGGGAATCTAGAAATAAA
AAGCAGCAGGAATTTATCGGAAATAACTGAAACCGAACAGACTAGATTCCCGCCTGCGTG
GGAATGACAATTCGAGACCTTTGCAATAACATAGGTTACTAAAATTTTATGCTCAATCTC
ATTTTCAAAATGCAAAACTTTTCTGATTTTTCCTACTTTTTGCTCAATATTAGGAAGGTT
TTAGGCAATTGAAAATTTTTTGGCGCATTTTTATGCGTCAAATTTCGTTAACAGACTATT
TTTGCAAAGGTCTCACTATATGTGCAAACCAAGCCAAAAATGCGAAATACCGTCTGAAAA
TCTTTCAGACGGTATTTGCTGTCTTTATTGCCGTTTTTCTTCCGTATCCGGATTTTTGTT
TGGGGCTGAAGCAGATTGGCAGTCAGATTGCAATCAAAGAATGAAGGCGAGCCGTCAAAA
ACAAAGCTATCCGCTTCACCGCCCCGATATTTAGAATTTGTGGCGCAAACCGACGGAGGC
GGCATTAATTTGAGTGTAGTTGCCGATGCCGGTATTGCGTTTCAGCCAAGCGCCAGACAC
GATGGCGGAAGTGCGTTTGGAAAAATCATAATCAACGCCGGCGATGATTTGATCGTAGCT
GGTATTTTCGCCTTTTTTACCGCGTTCGATAAAGTCGAAACCATGGGCATAGCTGATGCG
TGGAACTGCATTACCGAAGCGGTAGGAAGCAGTGGCGGCAATTTCGGTCGTACTGTTTTT
GGTTTTGTCGCCATTTTCAGACAAATCCAACTGAGCCGCCAAGGCGAGATTCAAGCCGCC
TTCCTCATAGCCGCCCGTCAGACGGTGTACCTGATGGTTTTTCAAGGGATCGGTACCTTT
GGCTTGATCACTCCCGCTGCCGATCAAGAACAACTCAAAAGCATTACGTCCGACATTGGC
GTGTCTCGCATATTTAAAGGCATAGTTCCCGGCAAAACCGCCATTTTTGTAATTCAGACC
GGCATAATACACATCCGATCCGGGCTTGCCGACAACAGCCGGAACGAGAGTAAGATTATT
GTTTGTATTCTTAGTATAATAAGCCGGCGTATAGGCGGACTTGCTGTTTTGGATCGGAAC
GAATTGAACGCTGCCGCTGAAACCGGAAAATTCGGGGGAATCGTAGCGTACGGAAACCGG
CATGTCGTCGTGGCGTTTGAAAATACCCAATTGCGAAGCCACATCATTATTGCTGTCCCA
AGGATCAATGGCTTGGCTGGCATCGTCAAACTGATTCGCAACGCGACCGGCGCGCAGCGT
ACCGAATTCGCCTGCCAAGCCGATAAAGGATTCCCTGTTGCCCCACTGGGTCGCGCCGCC
GCCGGCAACGGATACGTCTTGCTCAAGCTGCCAAACAGCCTTCAGCCCGTCGCCCAAATC
CTCACTCCCCTTAAAGCCGATAAACGAGCCGAAATCACTGATTTTCGTCCTGATGCGGCT
TTTGGCCTTAGTAACTTTAGTAACTTTTACCTGACCGCTCGCTCCACCGTTAGCGGCTTG
TGCTTCAGTCAATTGCAGCTGGTAGTTCCTGCCTTCCACGCCGGCTTTGATTTCGCCGTA
TAGGCTGACATCGGCAACGGCCGCAAGCGGCAGTGCGGACAATACGAGGGCGGTAAGTTT
TTTTCGCATATCGGCTTCCTTTTGTAAATTTGATAAAAACCTAAAAACATCGGGCAAACA
CCCGATACGTCTTCAATTATACCCCCCCCCCCGCAAAAAACCATTTTTCAGAACAAATAT
CTGATAAATGCCGCAACCTTTATTTTAAAAATGATTATATTTTGATATAAAACAATAGCT
TATTTTTTCAAAAACGTTGTGTTTCTACAACACAATTCAAGCGCAGACCTCGTGCGAGCC
GATGCGCTGCTGCCCGGATGCAGTCTCGGCTTTTTAAAACGCCATAAAAAAACACACGCG
GCACTTTATAGTGGATTAACAAAAACAAGTACGGCGTTGCCTCGCCTTAGCTCAAAGAGA
ACGATTCTCTAAGGTGCTGAAGCACCAAGTGAATCGGTTCCGTACTATCTGTACTGTCTG
CGGCTTCGTCGCCTTGTCCTGATTTTTGTTAATCCGCTATAAAGACCATCGGGCATCTAC
AGCCGTCATTCCCGCGCAGGCGGGAATCTAGAATTTCAATGCCTCAAGAATTTATCGGAA
AAAACCAAAACCCTTCCGCCGTCATTCCCACGAAAGTGGGAATCTAGAAATGAAAAGCAG
CAGGAATTTATCGGAAATGACCGAAACTGAACGGACTGGATTCCCGCCTGCGCGGGAATG
ACGGGATTTTAGGTTTCTGATTTTGGTTTTCTGTTTTTGAGGGAATGACGGGATGTAGGT
TCTTAGGAATGACGTGGTGCAGGTTTCCGTACGGATGGATTCGTCATTCCCGCGCAGGCG
GGAATCTAGAATTTCAATGCCTCAAGAATTTATCGGAAAAAACCAAAACCCTTCCGCCGT
CATTCCCACGAAAGTGGGAATCTAGAAATGAAAAGCAGCAGGAATTTATCGGAAACGACC
GAAACTGAACGGACTGGATTCCCGCCTGCGCGGGAATGACGGGATTTTAGGTTTCTGATT
TTGGTTTTCTGTTTTTGAGGAATGACGGGATGTAGGTTTTCTTAACCCTGCGTCCTAGAT
TCCCACTTTCGTGGTAATGACGGGATGTGGGTTCGTGGAATGACGTGGTGCAGGTTTCC
GTGCGGATGGATTCGTCATTCCCGCGCAGGCGGGAATCTAGACCTTAGAACAACAGCAAT
ATTCAAAGATTATCTGAAAGTCCGAGATTCTAGATTCCCGCTTTCGCGGGAATGACGAAA
AGTGGTGGGAATGACGGTTCAGTTGCTACGGTTACTGTCAGGTTTCGGTTATGTTGGAAT
TTCGGGAAACTTATGAATCGTCATTCCCGCGCAGGCGGGAATCTGGAATTTCAATGCCTC
AAGAATTTATCGGAAAAAACCAAAACCCTTCCGCCGTCATTCCCACGAAAGTGGGAATCT
AGAAATGAAAAGCAACAGGAATTTATCGGAAATGACCGAAACTGAACGGACTGGATTCCC
GCTTTTGCGGGAATGACGGGATTTTAGGTTTCTGATTTTGGTTTTCTGTTTTTGAGGGAA
TGACGGGATGTAGGTTTTCTTAACCCTGCGTCCTAGATTCCCGCTTTTGCGGGAATGACG
GGATGTGGGTTCGTGGGAATGACGTGGTGCAGGTTTCCGTGCGGATGGATTCGTCATTCC
CGCGCAGGCGGGAATCCAGACCTTAGAACAACAGCAATATTCAAAGATTATCTGAAAGTC
CGAGATTCTGGATTCCCGCTTTCGCGGGAATGACGAAAAGTGGTGGGAATGACGGTTCAG
TTGCTACGGTTACTGTCAGGTTTCGGTTATGTTGGAATTTCGGGAAACTTATGAATCGTC
ATTCCCGCGCAGACGGGAATCTGGAATTTCAATGCCTCAAGAATTTATCGGAAAAAACCA
AAACCCTTCCGCCGTCATTCCCACGAAAGTGGGAATCTAGAAATGAAAAGCAGCAGGAAT
TTATCGGAAATGACCGAAATTGAACGGACTGGATTCCCGCCTGCGCGGGAATGACGAATT
TTAGGTTTCTGATTTTGGTTTTCTGTTTTTGAGGGAATGACGGGATGCAGGTTTTCTTAA
CCCTGCGTCCTAGATTCCCGCTTTTGCGGGAATGACGGCGACAGGGTTGCTGTTATAGCG
GATGAACAAAAACCAGTACGGGGTTGTCTCGCCTTAGCTCAAAGAGAACGATTCTCTAAG
GTGCTGAAGCACCAAGTGAATCGGTTTCGTACTATCTGTACTGTCTTCGGCTTCGTCGCC
TTGTCCTGATTTTTATTAATCCACTATAATTTCCTGCGTGTGTCGGGTGTATCGAAATCA
AGCCGAATCAAATATATCGGACTTCGATAATGTCGTATTCGCGCACGCCGCCCGGGGCTT
GGACTTCCGCCGTATCCCCCTCTTCCTTGCCGATTAAGGCGCGGGCGATGGGTGAGCCGA
CATAGATTTTGCCCTGTTTGATGTCGGCTTCGTCTTCGCCGACAATTTGATAGATAACGT
GTTCTTCCGTTTCCAAATCTTCCAGCGTAACCGTCGTACCGAACACGATTTTGCCTTCGG
CGTGGATTTCGGTCGGATTGATGATGTGGGCAACGGAAAGTTTGTGTTCCAGCTCGGAAA
TGCGGCCCTCGATAAAGCCTTGGCGTTCTTTGGCGGCTTCGTATTCGGCGTTTTCGGACA
AATCGCCGTGCGAACGGGCTTCGGCAATCGCTTCGATCACTTCGGGACGCGCCACGCTTT

## Appendix A

TGAGCTGCTGCAATTCCTGTTTCAGCAATTCCGCACCGCGTACGGTCAGGGGGATTTTTT
GCATCGGTCTTTTTTCTCCATATTCCGGCACACCGGTTTGCGGCAGCAAGCATACCGCGT
ACCGTCTTGTTTTGTGCGTCCGGATATTAAAATAAAAATACAAGCCGCCCGGAAAATCGG
CGGCTTGTCTGTCGTTGAACAGCGGCTATTCTACCAAATTCTATGAAATTGGCAATCGTG
CCGCGCCGCCGGCAAACGCGCCATGTCCGCAACAAAAGCTGAAAATATGCCGACAAAGAA
ATTTTAGAAACAAAAAATTTAAAAATAATCAATTTTCGGCATAAAAAACCACATTTACGG
ACTTTAAAACCGAAAATGCCAAGCCTGAGATTTTTCATACAGCATTTGCACCAGTATAAT
GCAGGCTGTTTTTATCTTTAATAATATTGACGTTTTGCCATGACCGAATCCGTCCGCCTC
CCCGTCGCCCGTCTCAAACCTTCCACCGTCGCCCTGCCCGGCTCCAAAAGCATCAGCAAC
CGCACCCTGCTGCTTGCCGCCTTGTCCGACAATGCTTGCGAAATCCATTCCCTGCTCAAA
TCCGACGATACCGACCGTATGCTCGAAGCACTCGATAAACTCGGCGTTCAAATCGAATAT
CTTGCCGAAGACCGTCTGAAAGTGCACGGCACAGGCGGACGCTTCCCCAACCGCACTGCC
GATTTGTTTTTGGGCAACGCGGGCACGGCGTTCCGCCCGTTAACCGCCGCTCTGGCCGTT
TTGGGCGGCGATTATCATCTGCACGGCGTGCCTCGTATGCACGAACGTCCTATCGGCGAT
TTGGTCGATGCGTTGCGGATTGCCGGGGCCGATGTCGAATATCTCGGCAAGGAACACTAT
CCGCCGCTTCATATCGGCGAACGCCAAGACAACGGCGAGCGCGTGATTCCGATTAAAGGC
AATGTGTCCAGCCAGTTTCTGACCGCCCTTTTAATGGCGTTGCCGCTGACCGGGCAGGCG
TTTGAAATCCGTATGGTCGGCGAATTGATTTCCAAGCCCTATATCGACATTACTTTAAAA
CTGATGGCGCAATTCGGCGTACAGGTTATCAATGAAGGCTACCGCGTCTTCAAAATTCCC
GCCGATGCGCACTACCACGCGCCCGAACACTTGCACGTCGAAGGCGATGCCTCCAGCGCG
TCCTACTTCCTCGCAGCCGGTTTGATTGCCGCCACGCCCGTCCGCGTTACCGGTATCGGC
GCAAACAGCATACAGGGCGATGTCGCCTTTGCCCGCGAGCTGGAAAAAATCGGGGCGGAC
GTGGTTTGGGGCGAAAACTTCGTCGAAGTTTCACGCCCGAAGGAACGTGCCGTCCAATCC
TTTGATTTGGATGCGAACCATATCCCCGATGCCGCCATGACCCTCGCCATCGTCGCGCTT
GCTACAGGGCAAACCTGCACGCTGCGCAACATCGGTTCGTGGCGCGTCAAAGAAACCGAC
CGCATCGCCGCAATGGCAAACGAGTTGCGCAAACTCGGGGCAAAAGTCGTCGAAGAAGCC
GAAGCAATTCACATCACCCCGCCCGAAACGCTGACACCCGACGCCGTCATCGACACGTAC
GACGACCACCGCATGGCGATGTGTTTCTCGCTGGTTTCGCTGTTGGGCGTACCCGTCGTC
ATCAACGATCCGAAATGCACCCACAAAACCTTCCCGACTTATTTCGACGTGTTCTCATCG
CTGACCGAAACAGCGGAATAAGGCGGCATTTTGCCGCGATTCCGGCGCGGCGCGGCGGGC
GGCTCATTCTGTAAAAAAAGTATGTGCGCCGAGGTAGTTTTTGGCGTAAAACGGTGTGGA
GAGTTTTTCGGTTTTGATGGTTTTGCCGCTGCTGGGGGCATGGATGAATTCGCCGTTGCC
GATGTAGAGTCCGACGTGTGAGTAGCGGTGTGCGCCGCCGGTGTTGAAGAATACGAGGTC
GCCGGCCTTGAGGCGGCTGTCGGGGATTTTGCGGCTTGCCGCCGCCATGTCGCGGGCGGT
GCGCGGCAGCTTGACGTTGAGGGCGTTTTTGTAAACGAATTGAATCATGCCGCTGCAATC
GAAGCCGGTTGCGGTGCTGCTGCCGCCCCATTTGTAGGGCGTGCCGATGAGTCCGAGGCT
GTGGAGCATGAGTTCCTGCGAGCCTTGTGTGCGGTCGATGTGGCTGATGCGGACGGCTTG
GATTTGCCGGACTGTCTGTTTGGGTTTCGGTTGGCGGTGTTTGCCGGAGGTCGTGCCGCA
TGAGGCGAGGAGCAGTGCGCTGAGACAGAGGAAAAGGGTTTTGTCGGGGGGAAACATGGT
TTTTCCTTTGCGGGTTCGGATATCCGTCTGAAGGTGTTTCAGACGGTATAGTGGATTAAC
AAAAATCAGGACAAGGCGACGAAGCCGCAGACAGTACAAACAGTACGAAACCGATTCACT
TGGTGCTTCAGCACCTTAGAGAATCGTTCTCTTTGAGCTAAGGCGAGGCAACGTCGTACT
GGTTTTTGTTAATCCGCTATATTTCTATAATAAACCTTCTATGGGCAGCAGGGATAGGAT
TTTTGCGGCGATGCGTTTCCAAAGTTTGGCTTCGGGTTCGTTCGGGTAGGTTTTTCGGGT
GGCGGGATCGTGCCATTGCAGGCGGTTGTGCCTGTCGAGGGTAACGCGGTAGGCGTAGGC
GGGTGTGGTATCGGCAAGGGTGCGCTCCATCTGTTCTGCGATTTTGGGGCTTTCGATAAC
AACGCCCATTTCGGTGTTGAGACGCGCGGAACGGGGGTCGAGGTTGAACGAACCGATGAA
GATGCGTTTGCCGTCCACAATGAAGGTTTTGGCGTGCAGGCTGGTTACGGAGCTGCCGGT
CAGGCCTTTGTCTTTTGTGGCGGGGACGGCATGGTTGGGTTGCAGCTCGTAGAGTTTGAT
GCCGGCTTTGAGCAGCGGTTTTCGGTATTTGACATAGCCGGAATGGACGGCGGCAACGTC
GGTCGCCTGCAGCGAGTTGGTCAGAACGGTAACGTCTATGCCGTCCTGCACCAGTTTTGC
CAGTGCGTCTGTGCCGGATTTTGTGGGAACGAAATAGGGTGAAACCAGATAGACGCTTTT
TTCGGGCTGTTTGAGCGCGTCTTGCAGCCGCCCGGCAATCGGCGGTTTGCGGCGGTCGCG
GTCGAGTCCTTTTGCAGGGTCGTCGCTGATGAGGCGGGTTCGGACGCTCTGCCAGTCGAT
GCATCCTGTCTGTATTTTTTGGTAGAGGGGCGACTGTTCGACGGTTTCGCGGTAGCGCAG
GAGCGCGTGTCTGGACGTTTCGTCGTTGTATCCGAGTGCTTGAAGACCCTTGCCGATGTC
GCCGCTGCGGATGATGCGCGTGGCGTTGTGGGCGGAATGGCTTGCCCAGTAGCGGTCGAA
GTCGTGCGATACTTCGCCGACGACGCTGCCGGTGGCGAGGATGTCCAAATCGGCGAAAAC
GGTGTCCTCACCGACTTTGAAGTATTCGTCGCCGATATTGCGTCCGCCGAGTATGGTGGC
GCGGTTGTCGGCGGTAAAGGATTTGTTGTGCATGCGGCGGTTGAGGCGGGGGAAGTCGGT
CAGGTAGCCGAGTGCGCGCCATTTTCGTAAGACGAAGGGGTTGAACAGGCGCACTTCGAT
ATTGGGATGGCTGTCGAGGGCAAGCAGGAGGTCGTCCAATCCGCGCGTGTTGTTGTCGTC
CAACAGCAGGCGTACGCGCACACCGCGTTCTGCGGCAAGGTACACGAGGTTGAACAGCAG
CCTGCCGGAAATGTCGTTGCGCCAGATGTAGTATTGCAAATCGAGGCTGTGTTCGGCAGA
TTCGATAAGGGCGGCGCGGGCGGCAAAGGCTTCGTGGGGGTCGTTCAACAGATAGATATC
GGATAGCCCGTTGGTATGAGGGGTGTGCCGGATTTGCAGGATGTTGTCCAGGCGGACGGG
TTTGGAAGTATTGAAATGACGGCTTTCCGTCCGTTCTTCCAGTGGGGGCAACCATGAAGA
ACATGAACAGAGAAGGAGGCATAAAAGGGAAATTAGGCTGCGTGTTTTCATCAGGGATAT
GGTTTCAGACGGCATTGCCTGTGTTTTGGGGTTGGCGCGCATGGAAGTGCGGTATCATAA
TCCAAACGTTGAAACGGGTAAAAGTTTTGCGTGTGGACCGCTTCAGGACGGTGTGTTCCG
TGTCAGGTTGGTGCCGTCTGAAACGTGCAGCCGTTTGAAAACCAGCGATGATGCAAGGGT
GATGCCGCCGATGCTGAGCAGGGTCATACGGAAGGCGGAATGCAGACCTGAAGAAGCCGG
TATCAGAAATGTCCAGTTTTTAAGGATTAATGCGCCGGCAACAATGCCCATGCTGATGGC
AAGCTGTTGGTTGACCGCCATCAGGCTGTTGCCGCTGCCTGTTTGTTGCGGGCGCAAATC
GGCGAGGGTCAGTGTGTTCATGGCAGAAAACTGTAGGGAGTTGCACGCGCCGATCGCCAG

## Appendix A

CGAGAGGAAAACCCAAATCCACAGCGGCGAGTTTCCGTCAGGCAGGGCGAGCAGCATGAT
GAAGGCGGCAAGCAGCTTGGTGTTCCAAAGCAGTACCGTGCGGTAGCCGAAACGTTTCAT
GAGCGGTGCAATCAGCGGTTTGACCAGCAGCGAAGACAGGGCGACGGGTGCGACCAGCCA
ACCCGACAGGCTTGCGCCGAAGCCGAAAGCGATTTGAAACATCAGGGGCATCAGAAAAGG
AATCGAGCTGATGCCGAGACGGCTGAACAGATTGCCCGCCAGTCCCAGACGGAAAGTGCG
TATCAGAAACAGGTCGGCGGAATAAATCGGTTTGGACGCGGTTTTCATATGTCGGAAATA
ACGGCGTGCAAACAGCAGTCCGCCGCACAGCGGCAACAGTGCAAAATACGGAGGCAGCGC
GTGCGACAGGCTTTCTGCCGAAAGTAACAAGAGGCACGCGGCGGCAGAAAAAATCAGATA
ACCTTTGAAGTCTAAAGAGATATTACTGCCTTTAATATCGGGCATGATGTTGCGTCCCAA
TATGAAACCCAGCAGACCGATGGGCAGGTTGAGCAGGAAAATCCAGTGCCACGAAGCGTA
TTCGACCAAATAACCGCCCGCCAAAGGCCCTAAAACCGGCCCGATTAATGCGGGCATAAC
CGCATAATTGATGGCATTGAGCAGCTTGGACTTGTCGTACACACGCAAGATGGTCAGACG
CGGTATCGGAACCAGCATCGAACCGCCGATGCCCTGAACGACACGGGAAAGCGTCAATTC
AAACAGCGAACCCGATGCGGCGCACAATGCCGATCCGAGCATAAAAACGGCAATCGAACC
GAAAAAGACTTTTTTCGTTCCGAACCTGTCCGCCAAATAACCGCTCAAAGGAATCAGCAG
GGCAACCGTCAGCGTGTAGGAAATAACTGCCAGTTGCATATCCAGAGGCGACTCATTCAG
GTCGGCGGCAATTTCAGGCAGTGCGGTATTTAAAATGGTCGCATCCAACATCTGCATAAA
AATGGCAATTGCCAGCAGAAGCGGCAGCCAAGGGGATGGTGCGCGGGCGGATAGGGTGTT
TTTTTCCATAGGGCGATTGTACCCCATCCTTGTGCCGTTATTGTTTTCAGATGCTGTCTG
AATGCCGTCAGAGTCGGCATCTTGAATGTTCACAAGCAAACGAACCGGCATTGCATTGTA
ATGATAATTATTATCGAAAACCATCAGATTAAGGTACAGTAAGCGTTATGGGGGCAGTTT
GTAAGAAAAACCGGATTATTTTTTAAAATTAGACTTGACCCGCAACAGTCAATTACTTAA
AGTAAACGCTTACCTTTCTACAGAGAAAAACGGGTTTCCCGTTATCAAAAAACATGAGCG
CAACCATTCCCCCAAAAATCATCCGATACGACAGCAATCCGACCGATGTCTATTTTTTCG
GCACTTGCGTCCTTGATCTTTTTATGCCCGAAGCAGGCATGGATGCCATTACCCTAATCG
AGCAGCAGGGCATACGCGTCCATTTCCCGATGGCGCAAAGCTGCTGCGGCCAGCCTGCCT
ATTCATCCGGCCATCCGACCGAAGCCTTCGATGTCGCCAAAGCACAACTCGACCTTTTCC
CTGAAAACTGGCCGATCGTCGTGCCGTCCGGCTCGTGCGGCGGCATGATGAAACACCACT
GGCCGACGCTGTTTAAAGGCAGCGAGTACGAGGAAAGGGCTGTGGATTGCGCCGGCCGCA
TCATCGAGTTTACCCATTTCCTGCTTGCCATCGGTTTCAAACCCGAAGACAAGGGCGAAC
CGCTCAAAGTCGCCGTTCACACTTCCTGCGCCGCCCGCCGCGAAATGAATGTCCATCTTT
CAGGCTGGCAACTGATTGACGGTATGGAAAACGTCGAACGCATCGTCCACGACCACGAAA
GCGAATGTTGCGGCTTCGGCGGCACATTCTCCGTCAAACAAGCCGATATTTCCGGCGCAA
TGGTAACAGACAAAGTCGCCGCGCTGAAAGAAACCGGCGCAACCGAAATCATCAGCGCGG
ACTGCGGCTGTATGATGAACATCGGCGGCAAAATCGCCAAGGACGAGCCGGATATGCCGC
GTCCGAAACATATCGCATCCTTCTTGTTGGAACGCACCGGAGGCAAAGCATGAGCGCGCG
TGAAAATATTTTGGCAAAACTGAAAAAAGCCGACGCATTGCCGATGGAAGAACCTGCGGT
TTTTGATTATTACCGTGAAATGGGTGTTTCTTGGGGCAGCGAAGTTGAGCGTCTGAAACA
TTGGGCTGCCGCTATGCGTGCGGTCAAAACCGAAATTTATTGGGTGACGAAAAGCAATTG
GATGCAGGTTTTCCGCGAAGCGGCAGAAGGCAAGGGTTTGAAAAACATCCTGCTGCCCTT
GGCGACCGAACACGGACAAATTGCCCGTGCCGCATTGGCGGACAGCAATATCGAACCGAT
TGCCTTCGAGCGCGAAATCGATACTTGGAAAACCGAGTTTTTCACGAACATCGATGCGGG
CTTCAGCGGCGCGCAATGCGGCATCGCCCGCACCGGCACGCTGATGCTGTTTTCCAGCCC
CGAAGAACCGCGTACTTTAAGCCTCGTTCCGCCCGTGCATTTCTGCCTGTTCGATACGTC
CAAGATGTACAACGAGTTTCATAATGCCGTCGAAGGCGAAAAACTGGTGGAAAACGGTAT
GCCGACCAATGTATTCCTGATTTCCGGCCCGTCCAAAACCGCAGACATCCAACTGACGCT
TGCTTACGGCGCGCACGGCCCGCGCGATTTGGTCATCCTCGCCATCCTGCCCGACCACAT
TTCCCCTGCCGATTTGGAGGAAAACGCATGACTACGCAAACCATCAAATTTCACATGAAG
CCGGAAACTTTCAAGCAAAACGCGGGAATTTGCCTTCAAGACAAGCCTTTGCGCAAAAGC
CTGCGTACCGCGATGGATATGCTGATGACCAAACGCAAAGCCGTTTTGACCGACGAAGAA
GAGCTGCAAAGCCTGCGCGATTTGTGCGAACACGTCCGTCAGCGCTCATTGTCTAAATTG
CCAGCCCTGCTGGAGCAGCTGGAAGAAAACCTGACTAAGTTGGGCGTGAAAGTGCACTGG
GCAGAAACCCCGACCGAAGCCTGCCAAATTATCCACGACATCATCACAGCCAAAAACGGC
AAGCTGATGGTCAAAGGCAAATCGATGGTCAGCGAGGAAATCGAGCTGAACCATTATCTT
GAAGCAAAAGGCATTAAAGCGGTAGAAAGCGACTTGGGCGAGTTCATCGTCCAAATGGCA
GGCGAAAAACCGACCCATATCGTGATGCCTGCTATCCACAAAACCAAAGAACAGGTTAGC
GAACTGTTCCACCAAAACCTCGGTACGCCGCTGACAGACGATGTAGACCAACTGACCGGC
TTCGCCCGTAAAGCACTGCGCGATATTTACAGCACTGCCGATGTCGGTTTGAGTGGCGTA
AACTTTGCCGTTGCTGAAACAGGTACGCTGTGTCTGGTGGAAAACGAAGGCAACGGTCGC
TTGAGTACCACCGTACCGCCCGTGCATATCGCTATTACCGGCATTGAAAAAGTGGTGGCG
AAATTGTCCGACATCCCACCCTTGTACAGCCTGCTGCCGCGTTCTGCCATTGGTCAGAAC
ATTACCACTTATTTCAATATGATTACCGGCCCGCGCCGCAGTGAAGAATTAGACGGTCCG
CAAGAAATGCACTTGGTTCTGCTCGACAACGGCCGCAGCCAGGCTTATGCCGAAGACCAA
ATGCGCCGCACCCTGCAATGTATCCGTTGCGGCGCGTGTATGAACCATTGCCCGGTTTAT
ACCCGCATCGGCGGCGCGGCATACGGCACAACCTATCCCGGTCCGATTGGCGAGATTATT
TCCCCGCACCTGTTAGGCTTGGATGCCACTCGCGACCTGCCGACCGCCTGCACGATGTGC
GGCGCGTGCGTGGAAGTTTGTCCGGTACGCATCCCGATTACCGAACAAATGCAGCGTTTG
CGCGTTGAAGCGCAACGTTCGCCGACCGAAACCGTGCCGCACCCCATCCGGGGGCAAGGC
GCATCGCATACCTTCGGCGAACAAATGGCGTGGCGCACATTCAACGGTATTTTCAGCGGC
AGCAAAACCTACCGCGCCTTCGGTTGGGCAGCCACCAAGTTCCGCAACCTGACCCCGCGC
AAACAGTTGGGTTGGACGCAAAACCGCGTGCCGATGAAACCGGCGAAGAAAACCCTGCAC
GAACTAATGGCAGAAAAAATGCGCCAAAAAGAACAGGCATAAAAAGTTGTTCGCAAAAAT
GCCGTCTGAAACCCGAAACAGGGCTTCAGACGGCATTTGTATAGTGGATTAACAAAAATC
AGGACAAGGCGACGAAGCCGCAGACAGTACAAATAGTACGGCAAGGCGAGGTAACGCCGT
ACTGGTTTAAATTTAATCCACTATATATTCGCAGACGGTGGGTTTTAAATTTGTTCCAAT

## Appendix A

TCCATATTCAAAACAGCCTGTTCCTGTTTGGCTCGGAAGTCTGCCAGTTTTTGCGCCAGT
TCGGGGGTTTCGTTGGCGAGCATGGAAACGGCGAACAATGCGGCATTTGCCGCGCCTGCC
TCGCCGATGGCGAATGTGGCGACGGGTACGCCTTTGGGCATTTGTACAATCGATAAAAGC
GAATCTTCGCCGCGCAGGTATTTGCTGGGGACGGGTACGCCCAAAACGGGGACGGTGGTC
TTGGCGGCAACCATACCGGGTAAATGCGCCGCGCCGCCCGCACCCGCGATGATGGCTTTG
ATGCCGCGCGCCCGTGCGGTTTCGGCGTATTGGAACATCAAATCCGGGGTGCGGTGTGCG
GAAACAACGCGCGCCTCATATTCTACGCCGAACTCTTCAAGAAACTGCGCTGCCTGCCGC
ATAACGGGCCAATCGCTGTTGCTGCCCATGATGATGCCGATTTGTATCATAAATCCTCCT
TGGTGCGGATGGGGTAAAAAGCGGAAAAAATGGAAAAACTATCGTTTGCGCACGGCTGCGG
CGGCGCGTTTTGCCGCCGGGCTGCCGGGATAGGTCTGTATCAGGCTGCGCCAAGTCGCCC
TTGCAATGTCTTTTTGCTGAAGCCTGTATTGGCATTCGCCGATTTTGAACATGGCTTCAG
GCGCGGTTGGGCTGTCTTTGAAACGGTTGGCGTAACGCCCTCCGATTTCGATGACGGATT
CGCAGTTGCCCATACGCGCCCTGCTTTGCAGCAACAGGTACATACTGCGTTGCGCGATGC
TGCCGCCGTCGCCTCCGTCCGCGCCTTTCAACAGGGAGGCAGCGGCAGAAAACTTGCCGC
TTTTATAGTGTTTGAGTGCCTGATTGTAGAGGTTTTGTGCGGTTTCGACAGTATGTGCGG
ATGCGCTGCCGCCTTCGGTATTGAGGTAATGCTCTTTCAACTTGCGGTCGTCGAGTTTTT
GGACGTATGCCCTGCCGGAAGAATGTGTTTTTGCGTGTTCCAGTGCTTTGACTTTGCCGT
TTAAGGTTTCCACTTCGTTCGACAGCCGGACGATTTTGCCTTCCAGATAGTCCAAACGGT
CTTGCAAGGTCGGAACGGGATAGGGAATGCCGTCTGAAGCATTTTCCCGTGTCGACATTT
CGGTTTGGCTGCCTGCCGGAACGGGTGAAACGGAAGCACAGGAGGCGGACACAGACAGCC
AAATGATAAAAAGCGGTAATTTGATCTTCATTATTTTTCAGAAGCAGGGTCAAGCCGTC
GCCGACGGGCAGGGTGATGGGGACGATGCGCGGGTCGTTCGGCAGGTTTTGATTGAAATC
TTTGAGGATGCCGACGCTGGGCGGCGCATCGGAAGCCGCTTCGCGCATCACCCTTCCGTT
CAGCAAAATATTGTCGATGGCGATGATGCCGCCTTGACGGACGAGTTTGAGGCAACGCTC
GAAATATTGCGGCGTGGGCGGTTTGTCTGCGTCTATCAGTGCCAAATCGTAGCTTCCGGC
TTCACCCTGTGCAATCAAATCATCCAATGTCAGCAATGCGGGTTGCAGGTGCAGGCTGAT
TTTATGTGCCACACCGGCCTCGTTCCAAACCTGACGCGCCGTATCGGTAAAGGTTACATT
GATGTCGCAGGCGGTAATCCGCCCGTGTTCGGGCAGTGCCAATGCAAGCGCGGTGCTGCT
GTATCCGGTAAATACGCCGATTTCCAGATATTTTTCCGCACGGATCAGCTTTGCCAGCCA
AACCAAAACTGCCGCCTGTTCGCGCGCAATCGCCATTTTGCCCATACGGTGATGCCCGGT
CTTCTCGCGCAGCCGCGTCAAAACGGGATGTTCGGGTTCGCCGATGGCGTTCAAATAGTT
TTGCAGGTCCGGTGCGACATTGGACAGATGGGTCGTCATTTCGGCGGATTCAGTCTTGGT
AATAGGTATAAGGTTTTTCGCCACTTTTGCCGCCTCGAAGTTTTCCTGTTCTTCGGGAT
TGAGTTCGACATCCCACAAAAGCCCCCTGTTTTCCAAACGCTGCTGTTCCAACTCAGGTT
TTTCTTCAATCAGGCGGTTGAGGAATTGTGTGGCATCGGATTGGTAGTGATACATCTTTG
TGCTCCAATTTTACGGAATATGGCGTGATTATACTGGTATTTTCCAAACGGGATAAACGG
CTTTTATCAAGAATACGGGCAGAAAGATAAGGGGTTTTATTATAGAATAAGACGTTTTTT
GCAACGGAAGCCCGCCTTATGTCCCGAATCGCCGCCCTGCCCGACCATCTTGTCAACCAA
ATCGCCGCCGGCGAAGTGGTCGAACGCCCTGCCAACGCCTTGAAAGAAATCGTTGAAAAC
AGTATCGATGCAGGCGCAACGGCGATTGAAGTCGAGCTGGCGGGCGGCGGCATCCGCCTG
ATTCGCGTCAGCGACAACGGCGGCGGCATCCACCCCGACGACATCGAACTTGCGCTCCAC
CGCCACGCCACCAGCAAAATCAAAACCTTAAACGATTTGGAACACGTCGCCAGTATGGGC
TTTCGCGGCGAAGGTTTGGCAAGCATCGCCTCCGTCAGCCGCCTGACCCTGACCAGCCGT
CAGAACGACAGTTCGCACGCGACCCAAGTCAAAGCCGAAGACGGCAAACTCAGCAGCCCC
ACCGCCGCCGCCCACCCCGTCGGCACCACCATCGAAGCCGCCGAACTCTTCTTCAACACC
CCCGCACGGCGCAAGTTCCTCAAATCCGAAAACACCGAATACGCCCACTGCGCCACCATG
CTCGAACGCCTCGCGCTGGCGCATCCGCACATTGCCTTCTCGCTCAAACGCGACGGCAAA
CAAGTGTTCAAACTCCCTGCACAAAGCCTGCATGAACGGATTGCCGCCATTGTCGGCGAA
GACTTTCAGACGGCATCATTGGGAATCGACAGCGGCAACGGCGCGCTGCGGCTCTATGGT
GCGATTGCCAAACCGACTTTCGCCAAAGGTAAAACCGACAAACAATACTGCTTCGTCAAC
CATCGCTTCGTGCGCGACAAAGTGATGCTCCACGCCGTCAAGCAGGCATACCGCGACGTA
TTGCACAACGCACTCACTCCCGCCTTCGTCCTCTTTCTCGACCTGCCGCCCGAAGCCGTG
GATGTCAACGTCCACCCGACCAAAACCGAAATCCGCTTCCGCGACAGTCAGCAGGTGCAC
CAACTTGTGTTCCACACGCTCAACAAAGCCCTTGCCGACACACGCGCCAACCTGACCGAA
AGCGTCGGCAACGCAGGCGAAGTGTTGCATGACATTACCGGCGTTGTCTCCACCCCAATG
CCGTCTGAAAACGACAGCGAAAATCTGTTTGATAGCGTATCCAACTACCCGACAGGCAAC
AAATCAGATACACACAATGCCTTTGGTTCATCAGGCAAAACCGCGCCCATGCCCTATCAG
TCCGCATATGCGCCGCAACAACGCAGCCTGTCCCTGCGCGAAAGCCGCGCGGCAATGAAT
ACTTACGCCGAACTTTACAAAAAAACCGACGACATCGACCTTGAGTTAAGCCGATTCGAG
CAGGCACGTTTCGGCAATATGCCGTCTGAAACGCCTGCTCCCAAACAGATACGCCGCTT
TCAGACGGCATCCCGTCCCAATCCGAACTGCCGCCGCTCGGTTTTGCCATTGCCCAATTA
CTTGGCATCTACATTCTTGCCCAAGCCGAAGACAGCCTGTTGCTCATCGATATGCACGCC
GCCGCCGAACGCGTCAACTACGAAAAAATGAAACGCCAACGTCAGGAAAACGGCAACCTG
CAAAGCCAACGCCTGCTTATTCCCGTAACCTTTGCCGCGTCCCACGAAGAATGCGCCGCC
CTTGCCGATTATGCCGAAACGCTGGCAGGCTTCGGGCTGGAATTATCCGATATGGGCGGC
AACACCCTCGCCGTCCGTGCAGTTCCCGCCATGCTCGGCAAAGCCGATGTCGTCTCGCTC
GCCAAAGACGTATTAAACGAACTCGCCCAAGTCGGCAGCAGCCAAACCATCGAGGAACAC
GAAAACCGCATCCTCGCCACCATGTCCTGCCACGGCTCGATCCGCGCCGGCCGCCGGCTC
ACCCTGCCCGAAATGAACGCCCTTCTGCGCGATATGGAAAATACGCCGCGCAGCAACCAG
TGCAACCACGGCAGGCCGACTTGGGTCAAACTGACTTTGAAAGAATTGGACGCACTGTTC
TTGCGCGGACAGTAAGCCGAAAGTGCTAGAATACGCCGCCCGAGACCGCCGTTCAGACGG
CATTCCGACGCACCGACAGAAACATCACGACCGAAACCAAGAGAAAAACATGGCCTATCA
AGTTCTCGCCCGAAAATGGCGGCCCAAAACCTTTGCCGACTTAGTCGGTCAGGAACACGT
CGTCAAAGCCCTGCAAAACGCCCTGGACGAAGGCAGGCTGCACCACGCCTACCTGCTGAC
CGGCACGCGCGGCGTAGGTAAAACCACCATCGCCCGCATCCTTGCCAAAAGCCTCAACTG

## Appendix A

CGAAAACGCGCAACACGGCGAACCTTGCGGCGTATGTGAAAGCTGTACGCAGATCGATGC
CGGACGCTACGTCGACCTGCTGGAAATCGACGCCGCCTCCAACACAGGCATCGACAACAT
CCGCGAAGTCTTGGAAAACGCCCAATATGCACCGACCGCCGGAAAATACAAAGTCTATAT
CATCGACGAAGTGCATATGCTTTCCAAAAGCGCGTTCAACGCTATGCTCAAAACGCTGGA
AGAGCCGCCCGAACACGTCAAATTCATCCTCGCCACCACCGATCCGCACAAAGTTCCCGT
TACCGTCTTGAGCCGCTGCCTGCAATTCGTCTTACGCAATATGACCGCGCAACAGGTTGC
CGACCACCTCGCCCACGTCCTCGACAGCGAAAAAATCGCCTACGAACCCGCCGCCCTGCA
ACTTTTGGGACGTGCCGCCGCCGGATCGATGCGCGATGCCTTGAGCCTGCTCGACCAAGC
CATCGCCCTAGGTTCGGGCAAAGTTGCCGAAAACGATGTCCGCCAAATGATCGGCGCGGT
TGACAAACAATACCTTTACGAACTGCTGACAGGCATCATCAACCAAGACGGCGCAGCCCT
GACCGCCAAAGCGCAGGAAATGGCGGCGTGTGCCGTCGGCTTTGACAACGCCTTGGGCGA
ACTTGCCATACTGCTGCAACACCTCGCCCTGATACAGGCAGTGCCGAATGCCTTGGCGCA
CGACGACCCCGATTCCGATATTTTGCACCGCCTCGCCCAAACCATAAGCGGCGAACAAAT
CCAGCTTTACTACCAAATCGCCGTCCACGGCAAACGCGACCTCAGCCTCGCCCCCGACGA
ATACGCCGGCTTTATGATGACCCTGCTGCGTATGCTGGCGTTTGCGCCCTTGGCGGCAGC
ATCGTGTGATGCAAATGCCGTGATTGAAAATACCGAACTAAAATCCCCATCGGCACAAAC
CGCCGAAAAGGAAACCGCCGCAAAAAAGCCCCAACCGCGCCCTGAAGCGGAAACCGCCCA
AACACCCGTTCAGACGGCATCCGCAGCAGCAATGCCGTCTGAAGGCAAAACTGCCGAACC
CGTTACCAATCAAGAAAACAACGATATTCCGCCTTGGGAAGACGCGCCGGACGAAACCGC
AGCCGGCACGGCGCAAGCATCGGCAAAAAGCATTCAGACGGCATCCGAAGCCGGAACGCC
GCCCAAAAACCAAGTTTCCAAGAACGAAGCAGCCGACAACGAAACCGATGCCCCCTTGTC
CGAAGTGCCGTCTGAAAACCCCATTCAGGCAACACCGAATAATGAAGCCCTTGAAACAGA
AGCATTTGCACACGAAGCTCCTGCAAAACCTTTCAACGGTTACAGCTTTCCGAATGATGA
CTACCTCGTAGAAGACGGCGCAGAAATCCCACCGCCCGATTGGGAACACGCCGCCCCTGC
CGATGCGGAAGAAGAAAACAACGCCGACGAAAGCAGCAACAACGAAGACCACACGCCATA
CGCCCCGCCGCCCGAATTTTCCACCGAAAACTGGGCAGCCATCGTCCGGCACTTCGCCCG
CAAACTCGGCGCGGCGCAAATGCCGGCGCAACACTCCGCGTGGACGGAATACCATCCCGA
CACCGGTCTGATGGTTTTGGCAATGACCGCCGAAGCACGCGCCACCGCCGACAAAAAACG
CCTCGACAAAATCCGCGACACCCTTGCCCAAGCCTACGGGCTGCAACTCACCCTGCAAAC
CCAAGACTGGCGTGACGAAGCCGGCCGGGAAACCCCCGCGATGCAGGACAAGCGCGTCCA
AGCCGAAGACAGGCAAAAAGCACAAGCATTGCTCGAAGCCGACCCCGCCGCACAAAAAAT
CCTCCAAGCATTCGGCGCGCAATGGCAGCCCGAATCACTGGAATTGGCGGCAAACCGGCC
ATAAACAGATATAATGCCGCCCGAACCCTTCGGACGGCATTGCCGTTTCCCTTATTCAAT
CAAAACAGACAGGAGTATTCAGTATGTTCGGAAAAGCCGGATTAGGCGGCCTGATGAAAC
AGGCGCAGCAAATGCAGGAAAATATGAAAAAAGCGCAAGCCAAACTCGCCGAAACCGAAA
TCGAAGGCGAAGCAGGCAACGGCCTGGTCAAAATCACAATGACCTGCGCGCACGAAGTAC
GCAAAATCGACATCAGCCCCGATTTGATTCAAGAAGCCGCCGACGACAAAGAAATGCTTG
AAGACCTCATCCTCGCCGCCCTCAAATCCGCCCGAGGCAAAGCCGAAGAAACCGCAAACA
AAACAATGGGCGCATTCACGCAAGGTCTACCCCCCGGAGTGGGCGACTTCTTCCGCTGAT
CCCCGACCGTCATTCCCACGCAGGCGGGAATCTAGAACGTAGAATCTAAGAAACCGTTTT
ACTCGATAAATTTCCGTGCCGAGGGGTCTGGATTCCCGCCTTCGCGGGAATGACGGCATC
AGTTTGCAGGATTCGGCGTGAACGGTAAAAACAGTGAGAATGATAAGAACGCAAAAACGG
CAAGAATAGCGGGAATCGGCAGGCTGAAGCCCACCCTACCATTATTTACACATCCGTACC
GCTTAAATGCCGTCTGAAACTTCGTCATTCCCGTGAAAGCGGGAATCCAACCCCGTCGGA
GCAGAAACTTACACCCCGTCATTCCCGCGAACGCGGGAATCCAGTAACCGAAAAACCACA
GGAATCTATCGGAAAAACAGAAACCCTCGACCGTCATTCCCGCGAACGCGGGAATCCAGT
AACCGAAAAACCACAGGAATCTATCGGAAAAAACAGAACCCCCCGACCGTCATTCCCGCG
AACGCGGGAATCTAGAACGTAGAATCTGAGAAACCGTTTTACTCGATAAATTTCCGTGCC
GACGGGTCTGGATTCCCGCCTTCGCGGGAATGACGGCATCAATTTGCAGGATTCGGCGTG
AACGGTAAAAACAGTGAGAATGATAAGAACGCAAAAACGGCAAGAATAGCGGGAATCGGC
AGGCTGAAGCCCACCCTACCATTATTTACACATCCGTACCGCTTAAATGCCGTCTGAAAT
TTCGTCATTCCCATGAAAACGGGAATCCAGCCCCGTGGGAGCAGAAACTTACACCCCGTC
ATTCCCGCGAACGCGGGAATCCAGTAACCGAAAAACCACAGGAATCTATCGGAAAAAACA
GAACCCCCCGCCGCCGTCATTCCCGCGAACGCGGGAATCTAGTAACCGAAAAACCACGGG
AATCTATCGGAAAAAACGGAAACCCCCGACCGTCATTCCCGCGAACGCGGGAATCTAGAA
CGTAGAATCTGAGAAACCGTTTTACTCGATAAATTTCCGTGCCGACAGGTCTGGATTCCC
GCCTTCGCGGGAATGACGGCATCAGTTTGCAGGATTCGGCGGAAACGGTAAAAACGGCAG
AATCGATGGGATGCGGCAGGCTGAAGCCCACCAAAACACAAAAATTCCGATGCCGTCTGA
AATTTCGTCATTCCCGTGAAAACGGGAATCCAGCCCCGTGGGAGCAGAAACTTACACCCC
GTCATTCCCGCAAAAGCGGGAATCCAGTAACCGAAAAACCACGGGAATCTATCGGAAAAA
ACAGAACCCCCCGCCGCCGTCATTCCCGCGAACGCGGGAATCTAGAACGTAGAATCTGAG
AAACCGTTTTACTCGATAAATTTCCATGCCGAGGGGTCTGGATTCCCGCGTTCGCGGGAA
TGACGGCATATTTTTTGCATTTGATATAAAGGGTCGTTTGAATTTTGTTCAGCAAGTGCA
AAGTGTTGCACATAAAAGGGCGCAGGATAGAGGCAAAGCGGGCGTAGGTCGGGCTGTAGC
AACTGTATTTTTCACCCCGTCGGGCAAAAATATAGTGGATTAACAAAAACCAGTACGGCG
TTGCCTCGCCTTAGCTCAAAGAGAACGATTCTCTAAGGTACTCAAGCACCAAGTGAATCG
GTTCCGTACTATTTGTACTGTCTGCGGCTTCGTCGCCTTGTCCTGATTTTTGTTAATCCA
CTATACCAAAACTCAAATCAAGCCGTTCGGAGGCGGCTCAAAAAAACGGTACTTCGCAGC
AGAAGTACCGTTTATCGGGATTTCAGGTTTTATTCTTCGGGGCGTTCGCCGTCGGTTTCG
TCCTGCGTCCCTTCGGTGATGTGCATTTCTACGCCGTTGAGGGCGCGGATTTTTGCGTCG
ATTTCATTGGCGACTTCGGGATTTTCCTTCAGCCAGACGCGGACGTTGTCTTTGCCCTGA
CCGATTTTCGCGCCGTTGTAGCTGTACCACGCGCCGGATTTGTTGATGATGTCGTTTTTC
ACGCCGATGTCGATCAATTCGCCTTCCCAACTGATGCCTTCTCCGTAGAGGATGTCAAAC
TCTGCCTGACGGAACGGGGGGGCGACTTTGTTTTTGATGACTTTGACGCGGGTTTCGTTG
CCCAATACCTCTTCGCCTTTTTTGATGGATCCGGTGCGGCGGATGTCGAGGCGGACGGAA

## Appendix A

GAATAGAATTTCAGCGCGTTGCCGCCGGTGGTGGTTTCGGGGCTGCCGAACATTACGCCG
ATCTTCATCCGGATTTGGTTGATGAACACAACCAGCGTGTTGGTTTTTTTGATGTGTCCG
GTCAGTTTGCGCAAAGCCTGGCTCATCAGGCGCGCCTGCAGTCCGACATGGCTGTCCCCC
ATATCGCCTTCGATTTCGGCTTTGGGGACGAGTGCGGCTACGGAATCGACGACTACCATA
TCTATGCCGCCCGAACGGACGAGTGTGTCGCAGATTTCCAAAGCCTGTTCGCCGGTATCG
GGCTGGGACAGGTAAAGCTCTTCGACTTTTACGCCGAGTTTGCGGGCGTAAACGGGATCA
AAGGCGTGTTCGGCATCGACAAAGGCGCACACGCCGCCGTTTTTCTGGCATTGGGCGACG
GCTTCGAGGCAGAGGGTGGTTTTGCCGGAGGATTCGGGGCCGAAGATTTCGACGATGCGC
CCGCGCGGCAGACCGCCGACTCCGAGGGCGAGGTCTAATCCGAGCGATCCGGTGGAAATG
ACTTCGAGGTTTTCTTCCTGCTGGCTGCCGTCCATTTTCATGATGGCGCCTTTGCCGAAA
CTTTTTTCGATTTGCGCCAGTGCGGCGGCAAGTGCTTTGCTTTTGTCGTCTGACATTGGG
GTTACTCCGGAACAAATGCGGTATGTGGGATGCGGCGCAACACGGGCTGCGGCGCGGGAT
GTGTATCGTTTTCCCGATGTGCGGGCTATCGGTAATGCTGCTTCACGAGGTTGCCATTAT
CGCATATTTCCTTGCTTGCCGATATGCGCCAGGACGCGGCGGCTTGTGCCGGAATGGAAT
CTGGATGCCGTCTAAAAGGCGGCCGGCTTTGTTATAATGGCGGCTGTTTTTTCTGTGTGT
GCCTGTTTTATGTGTTCCTGCCTTGTTGTCAAAAATACCGTTATCGGAAGCGGACGCACC
AAAATCGCCGTGCCGCTTGTCGCCCGCGATGCCGCCGAACTTTCCGCCGTACTTGAGCAA
ATCAAAAATATGCCCTTCGATATTGCGGAGTTCCGCGCCGACTTTTTGGAATGCGCGGGC
AGTATCGGCGAAATATTGCACCACACGCAGACCGTCCGCGACGCGCTGCCCGACAAGCCG
CTGCTGTTTACGTTCAGACGGCATGGCGAAGGCGGCTCGTTCCCGTGTTCGGACGATTAT
TATTTTGAACTGCTCGACGCGCTGATCGAAAGCCGCCTGCCCGACATCATCGACATCGAG
CTGTTTTCCGGCGAAACCGCCGTCCGGTGCGCCGTGGCAAATGCTCAAAAAAACGGCATC
GCCGCCCTGCTCTGCAATCATGAGTTTCACCGCACGCCGCCGCAAGAAGAAATCGTATGC
CGTCTGAAACAGATGGAGGACTGCGGCGCGGACATCTGCAAAATTGCGGTGATGCCGCAA
AGCGCGGAAGATGTGCTGACTTTGCTTTCCGCCACGCTCAAAGCGAAAGAGCTTGCCGCC
AAACCGATTGTTACGATGTCGATGGGGCAGACGGGGGCGGTCAGCCGGCTTGCCGGACAG
GTGTTCGGCTCAAGCATCACGTTCGGTTCGGGAACGCAAAACTCCGCGCCGGGGCAAATC
GGCGTATCCGCCCTCCGTGCGACACTCGACTGCCTCGAAAACGGCGCAGACTGATTTCAG
ACAGCATCAAAACATGATGAAACTCAATCCCCAACAGCTCGAAGCCGTCCGCTACCTCGG
CGGCCCACTGCTCGTCCTTGCCGGTGCAGGCAGCGGCAAAACCGGCGTGATTACTCAAAA
AATTAAGCATTTGATTGTCAATGTCGGCTACCTGCCGCATACCGTTGCCGCAATTACCTT
TACCAACAAAGCCGCTGCGGAAATGCAGGAGCGCGTTGCCAAAATGCTGCCCAAACCGCA
AACGCGCGGGCTGACGATTTGCACGTTCCACTCTTTGGGCATGAAGATTCTGCGCGAAGA
GGCGAACCATATTGGTTACAAAAAAAACTTCTCCATTCTCGATTCTACCGACAGCGCGAA
AATCATCGGCGAACTCTTAGGCGGTACGGGCAAAGAAGCCGTATTCAAGGCGCAGCACCA
GATTTCCTTGTGGAAAAACGATTTAAAAACGCCTGAAGATGTCGTTCAGACGGCATCGAA
CATTTGGGAACAACAAACCGCACGCGTGTATGCGAGCTATCAGGAAACCTTACAAAGCTA
TCAGGCAGTGGACTTCGACGACTTAATCCGCCTGCCTGCCGTGCTGTTGCAGCAAAACAG
CGAAGTGCGCAACAAATGGCAGCGGCGGCTGCGTTATCTGTTGGTTGACGAATGCCAAGA
TACGAATACCTGCCAATTTACGTTGATGAAGCTGCTGACCGGCGCGGAAGGTATGTTTAC
CGCCGTCGGCGACGACGACCAGTCCATCTACGCATGGCGCGGTGCGAACATGGAAAACCT
GCGTAAAATGCAGGAAAACTATCCGCAGATGAAGGTCATCAAACTGGAGCAAAACTACCG
CTCCACCGCGCGGATTCTCAAAATCGCCAACAAAGTCATCGAAAACAACCCCAAGCTGTT
TACCAAAAAACTTTGGTCGCAATTGGGCGAAGGCGAGCCGGTCAAAGTCGTTGCCTGCCA
AAACGAGCAACACGAAGCCGACTGGGTCGTCAGCCAAATCGTCAAACAAAAACTCATCGG
CGGCGACAAAACCCAATATGCCGATTTCGCCGTGTTATACCGGGGAAAGCATCAGGCGAG
GATTTTCGAGGAAGCATTGCGCGGCGCGCGCATCCCCTACCAGCTCTCCGGCGGACAAAG
CTTTTTCGACAAAGCCGAAATCAAAGACGTGTTGTCTTATGTGCGGCTGCTTGCCAACCC
CAACGACGATCCCGCCTTTCTGCGTGCCGTTACCACGCCCAAACGCGGCATCGGCGATGT
~~CAGGCTGGGCAAGCTCAACACTTACGCGCACGAACACGAATGCAGCCTGTATGAAGCCGC~~
GCAAAACGAAGAAGCCCTTGCCACGCTGAACAATACCAACCGCCAACACCTGCAAACCTT
TATGGATATGTTCGTCAGCTACCTCGCCAAAGCCGAAACCAGCGAAGCGGGCGAGTTCAT
CAACAGCCTGCTCGAAGAAATCGACTATGAAAACCATTTGATGCAAAACGAAGAAGGCAA
AGCCGGCGAAATCAAATGGCGCAACGTCGGCGATTTGGTATCATGGTTTGCGCGAAAAGG
CGGGGAAGACGGCAAAAACATCATCGAACTCGCCCAAACCGTCGCCTTGATGACGCTTTT
GGAAGGAAAAGACGAAGAAGAAACCGATGCCGTCTCGCTATCCACGCTACACGCCGCCAA
AGGTTTGGAGTATCCGTATGTTTTCCTTGTCGGTTGCGAAGAAGGCGTTTTGCCGCACAA
CGACAGTATCGAAGAGGGCAACGTCGAAGAAGAACGCCGCCTGATGTACGTCGGCATCAC
CCGCGCCAAACGCCAACTCACACTGACCCACTGCGTCAAACGCAAAAAACAAGGCACATG
GCAGTTCCCCGAACCCAGCCGATTCATAGACGAAATGCCGCAGGAAGATTTGAAAATCCT
GGGGCGCAAAGGCGGCGAACCGATTGTCAGCAAAGAAGAAGGCAGACGCAACCTTGCCGA
TATAATCGGAAGGCTCGACAACCTAAAAAAAAGCGGCGCGGCGGATTAAACCGGAGCCGC
AATGCCGTCTGAAGGCTTCAGACGGCATATTTTTTGGACGGCGCGCGTAAAGCGGTTTAC
GCCCACAAATCCTGCTGCTGGTTTTTCGGCACAAGATGCCCCACGCCGATACCGATAAGG
CGGAACGCGTCTTCCGTCTGCGGCGAGACGCGCGCCATCAACATTTGCGCAGCCTGCAGC
AGAGTGCGCAGTCGGGCAATACGGAGGAATAAGTCAGTGTGCGCGTGATGATGCGGAAAT
CGTAGGTCTTCAGCTTGAGCGTTACGCTTTGGGCTTCGACGTTTTTGCGCGTGATTTGCC
GCCACAAGTCTTCGGCAAGATGGGGGAGGTGTCCGGCAGCCTGCTCGAGCGGCAGGTCTT
CGGGCAGGGTAATTTCTGTGGAGATTTGGAGGCGTTCGCGTTCGGCTTTGACGGGGCGTT
CGTCCGTACCGCGCACCAAATCATAGAGGCGGTATCCGTAGCGTCCGAAATGGTTTAAGA
GTTCGCCGCGCTCGAAACGGCGCAAGTCGCCCGCCGTCCGCATACCCAGCGACTGCATTT
TTTTCAGCGTTACCTTGCCCACGCCGGGGATTTTGCCCAAAGGCAGGGTTTCCAAAAATG
CCATGACTTTGTGCGGCGGCAACACAAACTGCCCGTTCGGCTTGCGCCAGTCCGACGCGA
TTTTCGCCAGAAATTTGTTCGGCGCGATGCCTGCGGATGCAGTCAAACCTGTTTCCGCAA
AAATGGCGGCACGGATTTCTTTGGCAACGTCGCCGGCGTAAGGATGTTTTTGAAATTAC

## Appendix A

GGGTAACGTCAAGATAGGCTTCGTCCAGCGACAAGGGTTCGATTAAATCGGTATAACGCC
TGAATACGGCGTGAATCTGCGCGGAAACCTGACGGTACAAATCGAAATGCGGCGGCACAT
ACACCGCTTGCGGACACAGCCTTTTCGCCGTTGCCACCGACATCGCGGAATGCAGCCCGA
ACTGCCGTGCCTCATACGATGCGGCGCAAATCACCGAACGCGCGCCCTCCCACGCGACGA
CCACCGGCCGCCCTTTCAAATGCGGCTGTTCGCGCAGCTCTACCGATGCGTAGAATGCGT
CCATGTCGATGTGGATAATTTTGCGTGAAGACATCGGCTCTTCTGAGGATAAAAGGGATA
TTCTACTGCCGGCATCGGGCAAATTCCAAATATACGCCCCGATAGACCTGCCTCCATAAA
AATGCCGTCTGAAACATACCCTGTTTCAGACGGCATCCGCAAAACTACGGTTTTCAATTA
AAACTGCCAATCCAGTTTCATGCTGACAGTGCGCGGCTCTCCGTAGAAGTTGTTTGCGCC
GCGCGTACGGTTGTAGTTGTTCTCAAAATAAGTGCGTCCGTTTAAGTTCGTACCGATGAG
GCTCAATTTGGCGTGTTTGCCCAATTCGTAACGGACGAAACCGTCTATCAGCCCGTAGCC
GCCCTGCCTGATGTTATACAGACTGCTTGTGCCGCTTTGTGCGGACACGCCGCCGCCGAC
GGTCAGCCCCGTATTCGGTATATGGAAGCTCGTTCCGAAACGGAATATGTGCACGGGTGT
GAAATTGCTGAAGTTGTACGGGTCTGCACTGGAATTTTTGGCAAGGCGTTCGGCGTTGAC
TTCGGCGGCGTTTTTGTAGCGGCTCTTGTTGTAGGTGTAACCCGCAAAGACTTTCCAATC
TTCGTTCAACTCACCCGACAACTCGAATTCCGCACCCCTGCTGACCACTTTGCCTATCGG
TTTGGCAACGGTTTGGAACGACCCCTGCTTGCCGCCTGCTCCGGGAACATAGCCGAAATC
GACGACCGTGCGGTTTTTCTGTTCGAGGTAAAACAATGCGAACGAAGCATTCAGCCGTCC
TTGCAAGAACGCGCCTTTCCAGCCTACCTCATAGTTTGTGCCGACCAAAGGCGGTAAAAC
GGTTTTGGCACTGACATCGACATTATCCTGCTGTTTGAAGATTTTGGTATAACTTCCGTA
AATACTCTGTTGCGGTGTCAAGTCATAGGTAATGCCTGCATAGGGCGTCAATTTATGACC
TTGCATCTTGGCCGTGTAATGGTCCTGATCCGCCCTAATGCTCGATGCCGTCTGAAAATC
GCTTGCCGGCTGCCCATAGCGGACAGGCATATCTTTGGTTTGCGAAGTCTCATAGCGCGT
GTAGTGCAGCCCGCCCAAAAGGTGCAGTCGGCCGGTTACGTTGAAACGCGTGCTGGCAGT
CAGCGAATGGGTTTTGTTGGTGTTGAGGTATTTGGCGTAGTTATACAGCGCAGGAACATG
GTCGTCTGCCACTTTGACGGTTTTCCAAACCGGCACCGTACCGGAAAAACCGGTAAAGGC
AGGCGTGCCGTCGGGATTGGTCTCCTGAATCTTGTTGCCTTTTTCGTCCAGCTCATATAC
ATCGACATATACCGGTGTCCGGCTGCCGCTGTATTCGTCATAGTAATACACCTGCTTGCC
TTCGGCATCGAGCTTGGGCTCGGTTTTTATTTTCTTGGCGTTCCTGCATTCTTCGGCATA
AACGGTACGGTTGCCTTTTTCATCGTACGCCTGCCAATCGGGTTCTTTATGCCCCCTGAC
CAAAGGAGACGACAAATCGCCGTCCGGCTCCTCCTGACAACTTCCCGCATACACGCCGTG
CGTTGCCCCCGTATTCGGACGTACTCTGTAGCGGCGTTCGTAGATTTCTAGATATTCCGA
ACGTATCTTTTCATCACCGTAGGCATAGCCGACAAAGAAATCATGCTCCCGCCCGAACAG
CCCATATGTGCCGGTCAGGTCAAGTTTAATTCCCCATTGGCGGTCGTCTTTGGTATGCCG
CAACGGCATATAACTGTATCGTCGGTTGGCGGTAGCCTTCCGATTAAAGGAAGAGTCATA
CAGGCTGTTTGGAAAACGTTGTGCCGCATTATTAAAGATGCCCTCCTTCGCAAGGGCTTT
ATCGACAAATTCCGCCTTGTCGGCATCAACGCCCGGATCCCCCCAAGAACCTTGACAGAT
AAAGTCCAGCGCGAAAGGGTCACTCATACACTTGTCAAAACCGGCTTTGCGTTCTGCGGC
ACGGCGGCTGCGATACTGTTCGAAAGCAGTATTATCGAAACGGTTTTTAACAAAATCGTC
TTTGCGCTCCCGGTATTCCTTGGCGGTTTCATCACGATATGCTTTCAGTTTCTCCAATGC
CTTATCTTTCGGCTCGAACGGGATGACTTCGTTTTTTTCAGTCAAAAAGCCTACCGCATC
CTCACCCGACAAACCCGCCGCATATTCGTTTTTCAGAAAAAACTGCCCCACCTTCGCATC
GGATTCATTCTTGGTATAAGACACTTCGGCATTGAGCTGCCAACCGTTGTCAAACACATG
TTTGAATCCTGAGAAAAGGTTGTATTTGTCGGCACTTAACCGCGACCAATCCTCCCCCAA
ATAAGTGTTGCGCGGCAGTTGCAAAGGCCGGTTGCAGGCAGGCGTTGAACTGAACGGGGC
AGTTTTCTGATTTTCACAGGGCAAAATAATGCCCGAAAAATCAGGAACCTCCCTACTCTT
CTGATACATGCCGCCCAAAGTAAGCACACTGCTGTCGCCCGCATCGGCTTCGGCAATGCC
GTAAACCATATGTTTCCTGCCCCAAACTCGGTCTTTAAACGATTTTTTATACTCTTCCGC
ACCCACCAACCTTCCGCGTAAGGTATTCGCCTTATTCAGGCTGCCTGAAACATCCAACAC
~~TGCACGGCCGGCTGCCGGCGATGGTCGGCGGTCAGCTCTCCGGTATGTTTGAAAGAAGCGGT~~
AGGTCACTTACGGATCAAATTGACGGTTCCTCCCGGCTCTGAATTGGATTGGGTCAACCC
CGTTGCACCCCGTACAACTTCAATATGGTCATAAACCGCCAAATCGGTACTCGGAGACAC
GTCGATTTTCGCCGTATATCCCGAACGGCCTGCAACATTGACGGTCATACCGTCTTCACC
AATCTGATCAATATAGAAACCGCGTGACAAAAACCGCGTCTGCAAGCCTGAATCGCGCAC
AACGTTGACACCCGTCGTGTTTTTCATTGCCTCTTCAAGCGTATGCACCGCCTTATCGTC
AAGGCGGCTGCGCGTGATGACGCTGACCGACTGCGGCGTATCCTTGCCCGCAATCCTCAT
ACCTGTGGCGGTGGACATCCGATCTATCGTATAAGAACGGGTCTTTTCGGTCTTGCCCAA
CAAAGCATGAGAGCCGCGTACATTGACCGTATCCAGACTGACGGTATTGCCGTCTGAAAC
AGGCACAACACCGTCTGCAAAAGAACCACCGTAAGCCGATAACAGCATAACGGTCAGAAT
TTTAAGTGAAAAATGATTTTGATTCATAGAGACCTCTGTAATATGCAAGTGTGCAAATCG
TCCAAAGGCTCTCACAACTGTTTTGATTTTTTATATTAATTGAAAAAAAGTAATTCTCAA
TTAAATTTATAGATAGGATTGTATTCCCATTTTGACAAAAAACAAACTACTCCTTACCGT
TTATTTCAAAAAACGATAACATTGTATTGAAAAATATCCGAATTTAAATACAGACCGCCA
ATGCAGAAAAAAACACCCAAATTGGCTATAATCCCGACAAACACACTCAAGGACAACAAC
ATGGCAGCCTCGCCCGAAGCAAAATTCACCGAAGAAAAGATTTTGTGGGTCAAACACCAC
ACGCCGAAACTCATCACTTTCGCCATCAGCCGTCCCGAATCCTACCGCTTTAAAGCCGGA
CAGTTCTCCCGACTCGGTTTCTACGAAGGGGAAGGTTTCATTTGGCGTGCCTATTCCATT
GTTTCCGCAGAATATGCCGACACGCTCGAATATTTTGCCGTACTCATCCAAGACGGCCCC
ATGTCGGCCCGTTTCGCCAAAATGCAACAGGGCAACACCATCCTGCTCGATAAAAATGCC
ACCGGCTTCCTCCTGCCCGAACGCTTCCCCGACGGCAAGGATTTGGTGATGCTCTGCACC
GGCTCGGGCATCGCCCCCTTCCTTTCCATTCTCGAACAACCCGAAATCCGTCAACGTTTC
GATACCGTCAACCTGATACATTCCGTATCTTTTCCCGAAGAATTGATTTTCAACGACCGA
CTCGCCGCATTGACTGAACATCCCCTGGTAGGCGAATACGGACACTCTTTCCGTTTCGTC
CCTGTTACCACCCGTGCCGCCAACCCCTCGGGCTTAAGCGGAAAACGCATTCCGGAACTC
TTAAAAAACAACAGCATCGAACAGGCGCTGCATACCAAGTTCACCCCGGAATCCACACGG

## Appendix A

TTTATGATTTGCGGCAACCCGGAAATGGTCAAAGACACTTTCCAAACGCTGCTCGACATG
GGTTACGCCATGCACCGCAACCGCATTCCCGGTCAAATCATGATGGAAAACGGCTTCTAA
AAACCACCCTGCTTGTCCGATGCCTTCGGATGGACGGGCAAACCGACACGGCACGAAAAC
CGCGTCGGCAAAAATGCCGTCTGAAAAAATTCAGACGGCATCTTCGGATACATTACCTGC
AAACGGCAACACACCGGCACAAACCGATTAGGCAATCAACACGGTGACGGCTGTTTACAT
ACTTGCCGGCTTTCACCAACCGATATCGATTTAACCGATTTCCTTAATATTTTTCCTGTC
CGTTTTAAACTTCGCCTTAAACGCATCCGGTAAATCTTTATCGAAATACCAAAGCCCGTC
ATCCATTTCCAATGCGCCGCCCATTCCGTGCAGAACGACTTTTTCCCCCGTCAAAGGATC
GGTTTCGGTAATTTCCACCTCATGCATTTTTCCCCAATCCAAAACAGGCAACTTGTGTGC
AAACGCCAAAACCTGTTCGTCAGAACGGCCGCACGCCTTATCGCATCGGCCTGAAACATA
TACGGGACAAGGCTCATCCTCTGCATAACCGTCCGGAAGGATACCGGCTGCGGCAGGACA
CAATCCGTATGTTTCCGCCCACGACGACAAAGCCCGTCTCGCTGCCTTTTTATTGGCAAA
TAATCCGGTAGGCGGATTATCCGTCACACCGTTTTTCAAAGCCGCTGTTTTCGCATTCAA
CATGCCGTCTGAACCTTTTTCAAACCTGACGGTCGTAAATGTTTTAAGCAGATTTTTGGC
AGACACATAACAATCCGAATGATAACGCCCGACCAATTCCGCTTTAATCTTATATGCATG
CAGGCTGCCCAATGCGGAAAAAAACGGACTTCCTCCGTATTGCACCAATCAAACGGGGC
TTTTCCGGAGTCCAATAAAGCCGAAATCTCGCTATATACCCGTTCAAACGTACCGATATA
ATTTACTTTCCCTCCGCCGTCGAAACAAGCCAGCACCCCCATACCGTCAGGCAAACCGTA
CAACTGTTCCCTCAACCGTTCGGGCAGCGCGGCAGGCAGCGGTTTCGGATTCATCAAACG
GAAACACTGCCTGATCCATGCCTCAACCCCGTGTTCCGACAGACTGTATTCCAAATAATC
ACACAATGCCGATACATCCGCCATCGCACGATGCCTGTCTTCCACAACAATCCCCAACCT
TTCGATGATACTGTCCAGGCTGTGCTTGTAAAATTGCGGATACAGACACCGGGACAGCTG
CACACTGCACAAAGCAGGCGATGAAAATCCGATACCCGCACGATGAAACTCATGCTTTAA
AAACGTATAGTCGAAACGGCTGTTATGTGCAACCAGCACACAACCCTTCAATACCGAAAA
CAACTCGCCGGCAATCTCTGCAAAAACAGGCGCATCGGCAACCATGCCGTCTGAAATCCC
CGTCAGCCCCGCCACAAACTGCGGAATCGGTTTTTGAGGATTAACCAACCACTCATGCCT
CACCACCCTTCCCTGCTCAAACTTGACCAAAGCCACTTCGGTTACCCTGTCTTCATACAG
ATTGCCGCCCGTCGATTCCAAATCAACCACGGCAACAGGCATTCCAAACCGTAAAAATAC
CTTTTCCAGCAAGGGCCAGCGAGAAGCAACAATCATTTTATTCTCTTTAAATTCAAACAA
CAAACCAATATTTTACACTTTTAAGGCATTTCATCCAACAAAACAATTGACAGAATCCGA
TGATTACCCTAAAATTCGAATCTTTCTTGCAGCGCACCCGTAGCTCAGTTGGATAGAGTA
TCTGGCTACGAACCAGAGGGTCGGGCGTTCGAATCGCTCCGGGTGCGCCAGTAAGAAAAT
ACAATATGCGCCCATCGTCTAGCGGTTAGGACATCGCCCTTTCACGGCGGTAACCGGGGT
TCGATTCCCCGTGGGCGTGCCAAATTCTAAATCCCCGAGATTATCGCTCGGGGATTTTTT
ATTGTCTCAGCAACTCGTTACCATATCTTTACCTACCCCCTTCATCAGAATCTCAGACGT
AATCGAATCATATTCAAACCTTTGCCGTGCAAACCGATATCCCATAACCGGATGCGGTGT
CCGTCCAACATTTTACCCGATTGAAACGCCTGATATATTGCACCCCATCAACGTGGCATT
ACTTTTCTTAACAATCCCCTTTGACAGCAACTGACTAGGGCTTTTTTATGCCATCATCAA
ATTTATAGTGGATTAACTTTAAACCAGTACGGCGTTGCCTCGCCTTGCCGTACTATTTGT
ACTGTCTGCGGCTTCGTCGCCTTGTCCTGATTTTTGTTAATTCACTATAATATTTTCTCT
CCCGATTGAAACAGGCGTAACAGAATGCCCGAAGCTCCGGCTGCTTTCTTGTTTACCGCC
GCGATATTTAGAGTATAATACCAAATTTGAGCAATAGTTCTAAAACAGTTAGAACCATTT
TTCATGAGCCTGACTGATTCGTACACTCGGAGAAACTGATGCAGAATATTTTTGACCCTT
TGGTTATTCGTGGAAAATCCCTTACCCCCATCGTGCAAGGCGGTATGGGGGTCGGTGTTT
CCGCATCGGGTTTATCCAGCGCGGTGGCGCGTGAAAACGGTATCGGAACGATTGCCAGTG
TGGATTTGCGCCACCTTCACGAAGACCTACTCGCCGAATCACAAATCAATCCGAGTGAAG
AGAAATATACATCTTTGAACTGTACCGCATTAGACAGGGAAATCCAAAAAGCCAAAAGCG
CTTCAGAGGGAAAAGGACTGATTGCGGTCAACGTGATGAAGGCGGTCAAAGACCACGCCG
CATATGTCCGCCAGGCTTGCGAATCAGGGGCGGATGCGGTTGTAATGGGTGCCGGCCTGC
~~CTTTAGACCTGCCGGAAATGACCGAGGGCTATCATAAAGATGTCGCGCTGCTGCCGATTC~~
TGTCCGAATCGCGCGGTATTAATATCGTCTTGAAACGTTGGATGAAAAAAGGCATATTGC
CCGATGCGATTGTAGTCGAACATCCTGCCCACGCGGCCGGACATTTGGGTGCATCAACCG
TTGAAGGCGTAAACGATGCCAAGTTCGACTTCAAACGCGTGATTGAGGAAACGTTTGAAG
TTTTCAAAAGTTTAGGGCTGGAAAGCGAAAAAATCCCGCTTATTCTTGCGGGAGGCATGG
CAAATTTTGAAAAAGTCAAAACCGCCCTAAAGAACTGGGGAGCATCCGCCGTTCAAATCG
GTACGGCTTTTGCCGTTACCGAAGAAGGAGATGCACACCTTAACTTCAAAAAAACGCTCG
CCGGTGCGGAAACTGAAAAAGTAGTCGAATTTATGTCTGTTGCCGGTTTGCCGGCGCGCG
GTGTCCGCACCAAATTCCTAGACAGCTACATCAAGCGTGAAAGCAAACTTCAGACAAACG
CCAAAGCCGACCCGCGCCGCTGTACCCAAGGTTTAAACTGCCTAACCAGTTGCGGTCTGC
GCGACGGGCTTTCCAAAGCAGGACAGTTCTGTATTGATATCCAGCTTGCCGCCGCATTCC
GTGGAGAAGTAGATAAAGGCCTGTTCTTCAGAGGTAAAGACCGCTGCCCTTCGGCAATGC
CATCCGCACCGTCCGCGAGACGATACAATATCTGCTGACGGGGAGCGAACCTGTTGCAAC
GCTCGGACGCTGACATCAGGTAGGATTTGATTTGCAAACAAAATGCCGTCTGAAGGGCTT
TCAGACGGCGTTTTTCAGGCTGCGTTCCGGAATAGTGTTAAAAAATAAACGGGATGAGAT
ACATTTATTTTCGTCCGACAAATCAAACCATCGCGCCGAATGATCAAATAATGCCTGCAC
GGCATTACATCTGGCAAAGCAATGCAATGAAAACACGGCTTTTTTATTTGCTTTCAGTAT
TATTGAAAAGCTTGTCCATCGGGGTCAAATCGACCGCATTGCCTTGGCTGGTAATCCATT
TGTTCTCGACGCGCCACACGCCTGCCGCGTCCTCCACGCGCACATACCAGTGGTTGGTCG
GCGAAAGGGTTTTGAACACCGCCTCATATTCCGCCCTGCCGTTCTGCGCGCTGCCGACGG
GCTTGAGGGCGACGGTTTGATCGTCCGCCTTGCGGGTCGGGTGCATCAGCAGCAGGTTCA
AAGGCTGTTTGCCGTCAAACTCGCCGCCGACAAACACTTTTGCCGCATTCATATCGGGGG
AAATGAGAACCTGCACCCCGATATGCCGTCTGACGGCTTCTTCATCCCGATGAAGCTGGA
TGTCGATATGTTTGCCGTCTTTATAATAATCGTCCGTAACCAAATCTGTCGCGTGCTGCT
GCGCGACAAAAAACATAGCGACGCTGGCGATGACGACAAAAATCGGCCCCGCCATCAAGA
TCCACGGCCAGACGTGTTTGTACCAAGGTTTGATTGGAGTGTTTTGAGACACGGTTATTC

## Appendix A

TCCGATAAAGGTTGCATCTTCTTCCAAGACGACCGGCTTGCCGTCGGGCGCGCCGCTTTC
GCGGTATTGGAAGGTAAATTCGATAGGGTGGCTGCCTTTGTCCGCGTATTCCGGAATGGT
GGACACTTGGACGGGAAGGGTTACCGTTTCGCGCGGGGCAACCTTGATACCGCCTTCGGG
CAGCCCGGTCAGGGCGATTTCGTCAAAGCCTTTGACACTTGCGGTAATCAGCTGTTCTTT
TTCACTTTTGTTGATGATACGCAGGCTGTATGCGTTTTCCAGCCAGCCTTTGGCGTTTTC
GCGCACCAGTACGCCACGGTCTTTCAAAATATCGACCTCGACCATTTTGCGCGTGGACAA
ACCGGCCAGGAAGGCAATGATAACTAACGCCAACACCGCGCCGTAACCTGCCACGCGCGG
TCTGAGCAGCCGTTTTTTAATGTCTTTTTCAGAATATTCGTGTTCCAGCGCGCTTTCGGT
CGTATAACGGATTAATCCGCGCGGATAGCCCATTTTGTCCATAATCTCATCGCACGCGTC
GATACAGGCGGCGCAGCCGATACATTGGTATTGCAGACCGTTGCGGATGTCGATGCCGAC
GGGGCAGACTTGGACGCACATCGCACAGTTGATGCAGTCGCCCAAACCCGCCTCTTCCTT
ATTGACCGTTTTCTTGCGCGCGCCGCGCGGTTCGCCGCGTTCCGCGTCATAAGAAACAAT
CAGCGTGTCCTTGTCGAACATCGCGCTTTGGAAACGTGCATACGGACACATATGCAGGCA
TACTTTTTCACGCATAATGTGGGCGAAGAAGAAGGTCATAAAGCCATAAAACGCTGCGGC
AAACATCGCGCCGCCACCTGCTGCTCCAGTGAATAAATCGGGAACGAACTGGCGGATAGG
GACAAACCAGCCTGCAAACGTGATGCCCGTCCACGCGCAGACAAGGAAAATCAGCAGGTA
TTTGGTGGCTTTGATGCGGATTTTAGTGAAATTCCACGGCGATTTTTCCAGTTTCAGCCG
TTTGTTTCTATCGCCTTCGACCAGGTTGTCAATCCACAGCATAATTTCGGTGTAAACCGT
TTGCGGGCAGGAATAGCCGCACCACAGTCGCCCTGCAATCGTCGTCCACCAAAACAGCCC
GAAGGCGCAAATCATCAGCAGCAAGGCAAGGTAAATCAAATCGCCCACCCCCAACGACAA
TCCGAAAATGAAGAAATGCCGTTCGGGGATATTGAAAACGACGGCCTGCCTGCCGCTCCA
GTTGAACCACGGAATGACGTAAAACACAAACTGCGTCGCCAATACGGCGGCGATACGCAG
TTTGGCGAACCGTCCTTCCGCCTTTTTGGGATGGATGCGTTCGCCTTCGGGATGGATTTG
AATCACGCTGGCTCGCGGATCGAATGTTTTTTTTGCTTTCGGCGCGGCTTTTGTTTGTTC
GGACGTGCCGATTCCGGATGCCGGACTGCCGGCTTGGTTTTCCGTGGTCATTCTGCATTC
CTTAGATTTTTGATTGATGGTTTGCCCGTTACCGCCGCCGTTTGCTTTTCAGACGTCATT
TTTCTTGTTTTTTAAGGCGTTGTGTTTCAAGTTTTGAGAAAATCCGTTTTTCCCAAAATA
TATTTCCGCTATTGTACAACTTTATGCGCCGTCCGGATGTATGGGGCGGATACATTTCCC
ATCCGCATCAAAACGCCTGGATTTTACCTTACCGCCCGAACAAAATCCGAATACGGTTAA
AAAAAAAGACTAAAAAAACCGACACCCCCATATCGGCAGAACCGACGGCGCAAGCTCATA
AACAAACGCTATCGACAATCCGGCACACAATCTATAACTTTTTATTTCAAAAGGAATAAT
GGCAGGCTTCGCCCGCAAATCGAAAATCCTTCCCCGCCTGTCCCCTGCCGCCGCCTTCCC
ACGCGTCCGCCCTTTTCTTGAAAGCATAAGCGAATCGGGCGATAATCAACGCTTTCCGAT
TATCCACTTATCTGAAACACCAGCAAGGAAAATACAAAATGTCTCAACTGGCAAACGCAA
TCCGCTTCCTCTCGGCCGATGCCGTTCAAAAAGCCAATTCCGGCCACCCCGGCGCGCCTA
TGGGTATGGCGGAAATGGCGGAAACATTGTGGACGAAATTCCTCAATCACAACCCCGCCA
ACCCCAAATTCTACAACCGCGACCGCTTCGTCCTCTCCAACGGCCACGCGTCTATGCTGT
TGTACAGCCTGCTGCACCTGACCGGCTACAACCTAAGCATTGAAGACTTGAAAAACTTCC
GCCAACTGCACAGCAAAACCCCCGGCCATCCCGAATACGGCTACACCGACGGCGTGGAAA
CCACGACCGGCCCGTTGGGGCAAGGGATTGCCAACGCGGTGGGTATGGCATTGGCAGAAA
AAATCCTTGCCGCCGAATTTAATAAAGACGGTTTGAACATCGTCGATCATTACACCTACG
TCTTTATGGGCGACGGCTGTCTGATGGAAGGCGTATCGCACGAAGCCTGTTCGCTCGCCG
GCACCTTGGGCTTGGGCAAACTGATTGTTTTATATGATGACAACAATATTTCCATTGATG
GTAAAGTGGACGGCTGGTTTACCGAAAACATCCCGCAACGCTTTGAAAGCTACGGCTGGC
ACGTCGTTCCCAATGTAAACGGTCATGACACCGCCGCCATTCAAGCCGCCATCGAAGCCG
CACGTGCCGAAACCGGCAAACCGTCCATCATCTGCTGCAAAACCTTAATCGGCAAAGGCA
GTGCCAACAAAGAAGGCAGCCACAAAACCCACGGCGCACCTTTGGGCGCGGACGAAATCG
AAGCCACGCGCAAACATTTGGGCTGGACTTACCCCGCCTTTGAAATCCCGCAAGAAATTT
ACGATGCGTGGAATGCCAAAGAACAAGGCGCGAAACTGGAAGCCGACTGGAACGAACTGT
TCGCGCAATATCAAGCCAAATATCCTGCCGAAGCCGCAGAATTTGTGCGCCGTATGGATA
AAAAGCTGCCGGACAATTTCGATGAATACGTTCAAGCCGCATTGAAAGAAGTGTGCGCCA
AAGCCGAAACCATCGCCACCCGCAAAGCCAGCCAAAACAGCATCGAAATCTTGGCAAAAG
AGTTGCCTGAATTGGTAGGCGGTTCTGCCGACCTGACCCCGTCCAATCTGACCGACTGGT
CAAACAGCGTCTCCGTTACCCGCGACAAAGGCGGCAACTACATCCACTACGGCGTGCGCG
AGTTCGGCATGGGTGCGATTATGAACGGTTTGGTATTGCACGGCGGCGTAAAACCCTTCG
GCGCGACTTTCCTGATGTTCAGCGAATACGAGCGCAATGCCCTGCGTATGGCTGCGTTGA
TGAAAATCAACCCTGTATTTGTGTTTACCCACGATTCCATCGGTTTGGGCGAAGACGGCC
CGACCCATCAACCGATTGAGCAAACCGCCACCCTGCGCCTGATTCCGAATATGGACGTAT
GGCGGCCGTGCGACACCGCCGAATCCTTGGTGGCTTGGGCAGAAGCCGTCAAAGCCGCCG
ATCACCCGTCCTGCCTGATTTTCAGCCGTCAAAACCTGAAATTCCAAGCGCGCAGCGAGC
AACAACTGAACGACATCAAACGCGGCGGCTACGTCATCAGCGAAGCCCAAGGCAACGCCC
AAGCCGTCATCATTGCCACCGGCTCAGAAGTCGAGCTGGCTTTGGAAGCGCAAAAAGCCC
TCGCCGCGCAAAACATCGCCGTGCGCGTCGTTTCCATGCCGTCCACCAACGTATTCGACC
GCCAAGACGCCGCCTATCAAGCCGCCGTCCTGCCCGAAGGCCTGCCGCGCATCGCCGTAG
AAGCCGGACACGCCGACGGCTGGTACAAATATGTCGGACTGAACGGCGCAGTCGTCGGCA
TCAACCGCTTCGGCGAATCCGCCCCTGCCGATTTACTCTTCAAAGCATTCGGCTTTACCG
TGGACAATGTGGTTGATACGGTGAAATCCGTGCTGTAACCCCACACCTAAACAAATGCCG
TCTGAAACCAATTAGGGCTTCAGACGGCATTTTTATATTCTCGCGGCCATGATGCTTTCT
CATCCCACCAATCTCCATTATAATATTTGCGAATCACTCTTATTCACATTTCAAAAGGAG
AAACGCATGAGCACCCGTACCGAACACGACACGATGGGCAATGTCGAAGTCCCATCCGAA
GCCTATTGGGGCGCGCAGACCCAGCGCAGCCGCAACAATTTCAAAATCGGTGGCGAAACC
CTGCCGCAGCCGTTGATTTATGCTTTGGCATTGGTGAAAAAAGCCGCCGCTGCCACCAAT
GTTTCCCTCGGTAGGATTAAGCCTGAACAGGCGGATTTGATTACGCAGGCGGCGGATGAT
GTGTTGAGCGGCAAGCTCGACGGGCAGTTCCCATTGGTAGTGTGGCAGACCGGTTCCGGC
ACGCAGTCCAATATGAACATGAACGAAGTGCTGGCAAACCGCGCCAACGAAATCGCCGGT

## Appendix A

ACGGGTTTGGCGGCTTATCAGCCCGTCCATCCCAACGACCATGTGAACCACGCGCAATCG
ACCAACGACGCATTCCCGACCGCTATCCACGTTGCCGCCGCGATTGAAATCAACCGCCAC
CTCATCCCCGCCGTAAAAGCCCTGCGCGACACGTTGGACAAAAAAGCCCAAGCTTTCGCC
CCTATCGTCAAAATCGGCCGCACCCACTTGCAAGACGCGACGCCGCTGACTTTGGGACAG
GAATTTTCCGGCTACGTTTCCCAGCTTGATCACGGTTTAGGCCGTCTGAACGATGCGCTT
AAAGACTTGTATGAACTTGCTTTGGGCGGTACGGCGGTCGGCACGGGTTTGAACAGCCAT
CCCGAATACGCCGAAAAAGCCGCCGCCAAACTCGCCGAATTGTCCGGCTTGCCGTTTGTC
AGCGCGCCGAACAAATTTGAAGCCCTGGGCGGACGCGATGCCGCCGTTGCCGCTTCGGGC
GCATTGAAAACGCTGGCGGCAAGCCTGAACAAAATTGCCAACGACATCCGTTGGCTGGCA
AGCGGCCCGCGTTGCGGTTTGGGCGAAATCAAAATCCCCGAAAACGAGCCGGGTTCGTCC
ATTATGCCGGGCAAAGTCAACCCGACCCAATGCGAAGCAATGACGATGGTGTGCTGCCAA
GTGTTCGGCAACGACGTTACCATCGGTATGGCGGGCGCGTCGGGCAATTTCGAGCTGAAC
GTCTATATGCCCGTTATCGCCTACAACCTCTTGCAATCCATCCACCTGTTGGGCGACGCG
TGCAACAGCTTCAACGAACACTGCGCCATCGGCATCGAACCCGTGCCGGAAAAAATCGAC
TATTTCCTGCACCATTCCCTGATGCTGGTTACCGCATTAAACCGTAAAATCGGTTACGAA
AACGCCGCCAAAGTCGCCAAAACCGCCTACAAAAACAACAAATCGTTGCGCGAAACCGCC
GTTGAGTTGGGCTTGCTGACGGGCGAAGAATTTGACGAACTGGTCGTTCCTGCCGATATG
GTTCATCCGCGCTAATCCTTCCCTCAAATAAAATGCCGTCTGAAACCTCGTTCGGACGGC
ATTTTCCGTTGCCTGCAAACTAGCGGCGTTTGAACAGCCTGTCCCCCACCGCCGCCGTAA
CCGCACCCCCGACCACGATCAGTGCGCCTGCATAACCCAAACCGTTCATATCCGGCGCGG
CAAAAGTATCAGGCATCACATAATGCCCGAGCAAAGAAAATATTACGGTAAACACGGGGA
GCAAGGTTGTTACCGCGCTGACTTTGGAAGCCTCCCAATGTTTCAACGCCTCGCCGAACG
AGCCGTAACCGATTAACGTATTCAAGCAGCAATACGCAAAACAAACCCACGCCAACGTAC
CGTCCAAACTTCCGATGTGTGCCGGTTCGGCAAACGGCAGGAACACGGCGGCACTTGCCG
CATAAATCAACAGCAGAATCTGTTGCGGCCCGAATTGCGCCGACAGCAGCTTTTGCGCCA
CGGCATAACACACCCATGCCATACTGCCTGCCGCACACAGCAACACGCCCTTCGCATACG
CGCCCAAACCCGACAACTCGCCGAATTTATCGTTAAAAAACATAAGCAAACCGGCAAGCA
GCAAAACCAAGCCGATTTTCTGAGCGGCAGTCATCCGGTCTTTAAACACCAACACACCGA
CAACAATCATCGTAAACGGCGAAATCTGCCACAAAACCTGCGTCGTGGTCGGCGAAATAT
AATGCAGCCCTTGGGCAATCAGCACAAAGTTTGCCGAAATGCCCGCCACGCCGAGCAGCA
GCAGCCTGAATGAGCACCAAGAAAAATCCCGCCGCTTCGGCAGCCGCCCGCCCAGTGCCA
GCAAAACAAACAATACCGCCGCCGCCACGGTAAAACGCACCCACACCAGCGTCGGCGCAT
CGACAAACTTCAATACCTGCCGCACGGCAATCGGCAGCGTTCCCCACGTCATCGCCGCCA
AAAGTGCCAACGCGAAGCCTAGGAGCGGCCTTTGGTTTTCCATCCTGATTTTCCTATTTT
TAAACAACCGTATTGCCGGACGATGCCGGTTTGCCGCATCGGGCAATGATGGTTCAAGCG
TTTGGCGTTTGATTCCAACCCTTTGATTTCAAACAAACCGGCTGAAGCTCGGCTATTGCT
TCGCGCTATTTGAAAACACCGCCTGAATTTTAAAATATAGTGGATTAACAAAAACCAGTA
CAGCGTTGCCTCGCCTTAGCTCAAAGAGAACGATTCTCTAAGGTGCTCAAGCACCAAGTG
AATCGGTTCCGTACTATTTGTACTGTCTGCGGCTTCGTCGCCTTGTCCTGATTTTTGTTA
ATCCACTATACCGTCTGAAAACAGCGGGGACGTGCGGCAGGCATCACTGCCTCAAAACGC
CCGAACGGCGCAATCGCAACGTTCCGCCCAACGCAAAGGGCGGCAACAAGCCGGCCCAAA
TGCAAAAAAGAGAAACCCTGCCCCGTAAGGTTTAAGGTTTCTCCGTCCTTTATGATTTCC
CTCCGCGAGGATGTCCGGCCGTAAAATTCAGAACGGGATATCGTCGTCAATGTCCTCGAC
CGGGGCGGCGGCAGGCACGGGTTGGCGGCGCGGCGCGGCTGGTGCTTCTTGGGGATGGGA
CGGCGCGTCGGAGGCGGGCTGCCGGCTTTGCTGCGCGGGGCGTTGGTAAGCCTCCTGACT
CTGACCGTAACCTTCCTCGTAAGGCGCACCGCCGCTGTTTTCATTGCGCCCGCCCAACAT
TTTCATTTCGTTGGCGACAATATCGTAAGCGGTGCGTTCGATGCCGTCTTTGCCTTGGTA
TTTGCGGCTTTGGATTCTGCCTTCCAAATAAACCAGCCCGCCTTTTTTGAGGTATTGCCC
GGCAATTTCCGCCAGTTTGCGGTACATGGTGATGTTGTGCCACTCAGTACGCTCTACACG
TTGGCCGTTGCGGTCGTTCCAAGTTTCGCTGGTGGCGACGCTGAAATTACAAACCGCCTC
GCCGTTGGGCATATAGCGCACTTCGGGATCGCGTCCGAGGCGGCCGATGAGGATGACTTT
GTTCAATGACATTTTTTAAACTCCTGTGATGATTTTTTCAGCGGCAGCCTGATCGAAACC
CTTCTGCAACACTTTGAGATAGACGGTCTGCCCGTCGAAACTGAAACCGATGTCTTCCAC
ACCCTCAAGCTCCGACAAGGCGCGGTATAACCCTTCCTGATTGCCCTGCCACACGCCGCC
GACAGGGTAACTGAGGTTTTTGACGGGCTTGGGCGCAGGCGATAAAACGGCAATTACCAG
CCACAGCAGCATCAATATACTGCAAAAGGCAAACACGCCGGAAAAGCCGTATTTTTGAAA
CAGCAAACCGCCTGCCGCGCCGCCGGCAAACAGTCCGAGCGACTGCATCGTGTTGTACAC
GCCCATCGCCGTACCCTTCAGGTCGGACGGCGCGATTTTGGAAACCATAGACGGCAGGCT
CGCTTCCAACACATTAAAACCGATAAAGTAAACAACCAAATAAGCGGTAATCAAGCCTAC
CGAGCGCATACCGGACAGCAAACCGAGCTGCGCCGCCGCAATACAGACGATACCCAAAAC
AAAAACCTGCTTAAGCTTGTTGCGCGTCTCGCCGACGATAATCAGCGGAACCATCACCAC
CAAGCCCGTAATGGTCGAAGGCAGATAGACTTTCCAATGCTGTATTTTTTCCAAACCGAG
CTGGGTCATCGCGAAAGGCAGCGCGGTAAACAATGCCATTTGTGCGGCGTGCAGGGCGAA
AATGCCGAAATCAAGCGTCAGCAGCCTACGGTTTTTCAAAACTTCGCCTATGCGCGAAGG
CTGCGCCTGCGTATCTTCGTGCAGCTTGGAAACTTCGGGATCGGGAGTCATCCACGCCAC
CACGCCGATGCTGATGACGGTCAGAATGCCGGTCAGCATAAACAGTCCGCGAACGCCGAC
CGCGTCGGCAATCACGGGGGCAACGACGAGGCTGACCGAAAACGTCAAACCGATACTCAA
ACCGATCATCGCCATTGCGCGGGTACGTACGCCGTCGCGCGTCAAATCCGCCAGCAGCGC
GGTAACCGCCGCACTGACCGCCCCTGCACCCTGTATGGCGCGTGCGGCGACCAGCATGGG
CAGCGTATCGGCGGCGGCGGCAAGAAAGCTGCCCGCCGCAAACACGACCAGTCCCGCATA
AATGGTTTTCTTGCGCCCGAACTTGTCGGAAGCGATGCCCAAAGGCAGTTGCAGCAGAGC
CTGTGTCAGCCCGTAAATGCCCATTGCCAGCCCGACCAGCGTTTTGTTGCCTTCCGCGCC
GGGCAGCGAGGCGGCATACACCGCCAATACGGGCAGCACGAGGAACATACCCAGCATACG
CAGCGCGTACACGCCGGAAAGCGTCGTACTGGCGCGCCATTCGTGCGGAAACATTTGGAT
GCGGTTGTCTCTTGCCATCATATTTTTTCAGACGGCATCAACAGTTGCAATGCCGTCTGA

## Appendix A

ACTTCCAGTGAACAGATTTTCGGATTATACAGGATTCGCCGTATTTCGGTTGCGGCGCGG
GTTCAAAATCAACGCCACTGCCAGCGGTTGCGCCACGCGCCCAAAACGGCGTTCGGATAT
TTATTGCTGCCCAAGCTGCCGTTAAAGCGGGCAAGCGCGCGGACGATGTTGCCTTTTTCA
AGATTCCGGTAATGGCGCAGGATGGTACAGCCGTAACGCAGGTTGGTGCGGATGTCGAAC
AGGTTGTGCGCCGGTTTGCCGATGTAGTTTTTCCAAAACGGCATAACCTGCATCAGGCCG
CGCGCGCCGACACCGCTGATTGCATACTGGCGGAACGCGCTTTCCACCTCAATCAGCCCC
AACACAATCTGCGTATCCAAACCGGCCCGGCTGCTTTCGTACTGGATATTGACCAGCAGC
CTGCGCCGCTCCTCCTCCTCGGGGACGAACCTTGCCAAACGTGCCGACATGGCAGACAAC
CAACGCTCGCCCTCTTTCGGATTGTCAAACACCAGCCTCGGCGGATTGACGCTGCCGACA
GAACTCCTCATCACGGAAGCCACATCGTCGGCAAGCGTTTCCTCACGTTGCGCGCCGGCG
TGCGCCAGAGGACTGAGCAACAACGCACCGGCGGCACACAACAGGCGGCGGCGTTGCAGA
TTAACGGGTAGGGTATCGGTCGGTTTTCTCATAGGGAACGGGGGCGCGTCCGGACGTTTC
AGACGGCATTAAATATTCAAACAGACATAATTGCTTTCAACGCGAAAAACCGCGCGCAAA
ATCCAAGCGCGGCATATCGCCCTGCCCTTTTCGGGCAAACCTCAATTCTACCGCCCTCAA
GAACGCTTGTCCAAACAGGCACAGGCAACACCGCCCGGGCATTTCCGTTTTCACCGGTTA
TCCGTCGTCCGGATTATGCAGCAGCACCATCAGCGCATCACGCTTTTCGGGCGGCAGCAG
GCGGAAATATAGTAGATTAAATTTAAACCAGTACAGCGTTGCCTCGCCTTGCCGTACTGA
TTTAAATTTAATCCACTATATCTTGAGGCCTTTGCAAAATTCCTTTCCCTCCCGACAGCC
GAAACCCAAACACAGGTTTTCGGCTGTTTTCGCCCCAGATACCTCCTAATTTTACCCAAA
TACCCCTTTAATCCTGCCCGGACACCTGATAATCAGGCATCCGGGGCACCTTTTAGGCGG
CAGCGGGCGCACTTAGCCTGTTGGCGGCTTTCAAAAGGTTCAAACACATCGCCTTCAGAT
GGCTTTGCGCACTCACTTTAATCAGTCCGAAATAGGCTGCCCGGGCGTAGCGGAATTTAC
GGTGCAGCGTACCGAAGCTCTGTTCGACCACATATAGTGGATTAACAAAAACCAGTACGG
CGTTGCCTCGCCTTAGCTCAAAGAGAACGATTCTCTAAGGTGCTGAAGCACCAAGTGAAT
CGGTTCCGTACTATTTGTACTGTCTGCGGCTTCGTCGCCTTGTCCTGATTTAAATTTAAT
CCACTATAACGGGTTTTCGACAAATATCGGTTGCGTTTGGTTTGCGCCTCCGTCAGCGGA
CGGTTGCGGCAGGCTTTGCGCATAATGCCGTTCTGCAACCGATGCTCTTTCAGTTTTCCG
TAGGTCGGATTCTCGAATCCGACATTACTTCAATCGTATCCAATAGAAAAGTCCGCATTG
CCGCCACCCCAATTATGCGGATAAATACCCTGTTTGACATAACGGTGAAACGTAGAAAAC
CCCCAATCGGAAATTTGTCCTACATAGCCATGTTTGACCGGATTGAAATGCAGATAATCA
AAATGCCAGGCAAAATCGGCCTCATCGCGGATAGTATATTCCCAAAAGCGTTTTTGCCAA
AGCCTGAGATTGCCGCCGATTAAATATTGGCTGTGCCGCTTGATTTGCCGCCAGCGTTCC
GAATAAGCAGAATCATTGTCCGGCAGCCGCCATATGGTATGCAGATGGTCGGGCATCAAC
ACCCATGCCAAAATTTCAAACGGATACCGTTCGCGCACCGCCATTACCGCCTGCCGTAAA
GCCAAACGCACCGCATCATCGGTCAAAATCTTCTGCCGTTTATTGGTTACAACCGTAAAA
AAGTAAGTGCCGCCATTGCGGTAAAAACGACGGTATTTCATAGTATTATGCTCGGAATGA
TTTTGTAGGTCGGATTCTTGAATTCGACATTTTGGGCATTGCTGCAATGGATTGCAATGA
TGGGAATGTTAAAGGTTTTGTCGGATACAAGTATCCGACCTACGCTTGCTGAACCGTCAT
TCCCACGAAAGTGGGAATCTAGAATCTCGGGGTTTCAGTCATTTCCGATAGATTCCCGCC
GCGTCAGGGGGTCTGGATTCCCGCCTGCGCGGGAATGACGGGTTTCAAGATTGCAGTGTT
GTCGGGAATGACGGGTTTCAAGATTGCGGTGTTGTCGGGAATGACGAATCCATCCATACG
GAAACCTGCACCACGTCATTCCCACGGAAGTGGGAATCTAGAATCCCGGGGTTTCAGTCA
TTTCCGATAGATTCCCGCCGCGTCGGGGGTCTAGATTCCCGCCTGCGCGGGAATGACGGG
TTTCGAGATTGCGGTGTTGTCGGAACGCAACTGAACCGTCATTCCCACGACAGTGGGAAT
CTAGAATCTCGGGGGTTCAGTCATTTCCGATAGATTCCCGCCGCGTCAGGGGGTCTAGAT
TCCCGCCTGCGCGGGAATGATGGGTTTCAAGATTGCGGTATTGTCGGGAATGACGAATCC
ATCCATACGGAAACCTGCACCACGTCATTCCCACGAAAGTGGGAATCTAGAATCCCGGGG
TTTCAGTCATTTCCGATAGATTCCCGCCGCGTCAGGGAGTCTGGATTCCCGCCTGCGCGG
GAATGACGAATTTCGAGATTGCGGTATTATCGGGAATGACGAATTTCGAGATTGCGGTAT
TGTCGGGAATGGCGGGTTTCAAGATTACGGTGTTGTCGGGAATGACGGTTCGGGTATTTC
CACGCCCGCCCCGCGCCTGTAAACGGCAGGTGAATCAAAAATGCCGTCTGAAGGTTCAGA
CGGCATCGGTGTCGGGGAATCAGAAGTGGTAGCGCATGCCCAATGAGACTTCGTGGGTTT
TGAAGCGGGTGTTTTCCAAGCGTCCCCAGTTGTGGTAACGGTATCCGGTGTCTAAAGTCA
GCTTGGGTGTGATGTCGAAACCGACACCGGCGATGACACCAAGACCTAAGCTGCTGATAC
TGTTGCTTTCGTGATAGGCAGGTTTGTTGGTCGGACCTTGTACGATTTTGCCTGGCACTG
TAGCGCCTTGCGCTGGTGGACTGAAAGTAGTCGTGGTTTCTTTTCTCACCGAATGAACCT
GATGTTTAACGTGTCCGTAGGCGACGCGCGCACCGATATAGGGTTTGAATTTATCGAATT
TATCGTTGAGTTTGAAATCGTAAATGGCGGATAAGCCGAGAGAAGAAGCGGCGTGGAATG
TACCGTTTTCCTGATTTTCCGTCTTCAGTTCTTGCCAGATGCCACTGCTATTGTTTTTTT
GCAACTCTTTTGTGTTTACGGAATATTTATTGTTGTTCCATTTTCTGTAACTGGCATAAT
CTGCCGCTATCCTCCAGCCGCCGAAATCGTAGCCGACCGACACCCGGGGGTGGATGGAAT
GCGCACGGATGTTTCTGAAATAATCGCTTACTGTGCTTGTGTTGTTTGCACCGGTTGCTT
TCGGATAATCGTGGGTAATGCGTTCGGCGGCATAAGCTAAATCCGCCTGCACATAATACG
GGCTGCGGCTGCCGTCTTCACTTGCCGCCTGCGCTGCGGAAGAGAAGAGAAGAGAAGAGA
AGAGAAGAGAAGAGAAGAGAAGAGAAGAGAAGAGAAGAGAAGAGAAGGTTTTTTGGGGGC
TGGATTCATTTTCGGCTCCGTATTCGGTTTTAACTGATTAAAAAGAAAGATTTTCAATGA
TGTTGCAGGAGCGGACTATATCAGGTTTGTGGCGATGTTTCAACACAATATAGCGGATGA
ACAAAAAAGAGAACGATTCTCTAAGGTGCTGAAGCACCAAGTGAATCGGTTCCGTACTAT
TTGTACTGTCTGCGGCTTCGTTGCCTTGTCCTGATTTTTGTTAATCCGCTATAAACAACG
CTTCGTCCGAAAAAACGATTGAATTTGCGGGCAGAAGCTGGACGAAAACCGCCGACAGCC
TGCCGCAAAAGGCACACGGTTTGCGCTAGGGCTTAGGCGTGTCGCGCGAAATCAATGCGG
GCAGGCATCATTTCCTCTACGGCGGCATCAGCGGCGGCGGCGTGCATTATTGGGATAACA
AAGATTTCAGCGAACAGAGCCTGCGCCTGTCGTTCGGCTATAAAAACCGTTCGGTAACGC
GCTCGTTCGGCATCGTGCCGTTTGTCGAGCAAAACCTCTTAGGCGGCAGCCGATACAATT
TCGTCGGCGGCTTCAATGCCGATTTCTCCCAACGCTTGAGCGAACGCTGGCGGTTGACAC

## Appendix A

TAAACGCGGGCAATATGTGGAAGCATTATCAGGAAGACCGCACCGCCGCCCGATACGACA
GCCATATGCCGCTGGCGGGCGCGACGCTGATGTATTCCGCGCCGAAAGACTGGCTGCTTT
ACGGCGGTGCGGACTGGTCGCACAACATAACGAAAGAGGCGGAACAGGCTTCCATCCGCA
AGGGTTTGCGTGTCGGCGCGGTCAAAACGTTCGACGGCGGCTTGGGTCTGCGGGCAAACC
TGCGCTATACCCGCAGGATGTTTGACGCACCCGGGACCATTGTGTACCGCTTCCCGCGCA
AAGACCACGAATATCAGGCAAACCTGTCGTTGTGGCATGACAAAATCTCTTGGAAGGGCT
TTACGCCGCAACTCAATTTCCGCTATCTGAAAATCGACAGCAATATGAAAAGTTTTTACA
CACGCAAAAACATGCAGATTTTCATGAGCGTGGAAAAGGATTTCAAATAAGCGCAAAAAA
TGCCGTCGGCAACATCCGTGGGCAGAATCAAAAACCGCCGCATCATTTATTGTCAACGCC
TGCGCCGTCAGAGTAACATTGCGTTTTTCCCCCACCGGTATCCGCCATGACCACCACCCC
CGCAAACGTCCTCGCCTCCGTCGATTTGGGTTCCAACAGTTTCCGCCTCCAGATTTGCGA
AAACAACAACGGACAATTAAAAGTCATCGATTCGTTCAAACAGATGGTGCGCTTCGCCGC
CGGACTGGACGAACAGAAAAATCTGAGTGCCGCTTCCCAAGAACAGGCTTTGGACTGTCT
GGCAAAATTCGGCGAACGCCTGCGCGGCTTCCGCCCTGAACAGGTACGCGCCGTGGCAAC
CAACACATTCCGCGTTGCCAAAAACATCGCAGATTTCCTTCCCAAAGCCGAAGCGGCATT
GGGTTTCCCCATCGAAATCATCGCCGGGCGCGAAGAGGCGCGGCTGATTTATACCGGCGT
GATCCACACCCTCCCCCCGGGCGGCGGCAAAATGCTGGTTATCGACATCGGCGGCGGTTC
GACAGAATTTGTCATCGGCTCGACGCTGAATCCCGACATTACCGAAAGCCTGCCCTTGGG
CTGCGTAACCTACAGCCTGCGCTTCTTCCAAAACAAAATCACCGCCAAAGACTTCCAATC
TGCCATTTCCGCCGCCCGCAACGAAATCCAGCGTATCAGCAAAAATATGAGGCGCGAAGG
TTGGGATTTCGCCGTCGGCACATCGGGTTCGGCAAAATCCATCCGCGACGTGCTTGCCGC
CGAAATGCCCCAAGAGGCGGACATTACCTACAAAGGCATGCGCGCCCTCGCCGAACGCAT
CATCGAAGCCGGTTCGGTCAAAAAAGCCAAATTTGAAAACCTGAAACCGGAACGCATCGA
AGTTTTTGCCGGCGGACTTGCCGTGATGATGGCGGCGTTTGAGGAAATGAAACTCGACAG
GATGACCGTAACCGAAGCCGCCCTGCGCGACGGCGTGTTTTACGATTTGATCGGGCGCGG
TTTAAACGAAGATATGCGCGGACAAACGGTTGCCGAGTTCCAACACCGCTACCACGTCAG
CCTCAATCAGGCGAAACGCACCGCCGAGACCGCGCAAACCTTTATGGACAGCCTCTGCCA
CGCTAAAAACGTTACAGTTCAAGAGCTTGCCTTGTGGCAACAGTATCTCGGACGCGCCGC
CGCGCTGCACGAAATCGGTTTGGACATCGCCCACACCGGCTATCACAAGCATTCCGCCTA
CATCCTCGAAAACGCCGATATGCCGGGTTTCTCACGCAAAGAACAGACCATACTTGCCCA
ACTGGTCATCGGTCATCGCGGCGATATGAAAAAAATGAGCGGCATCATCGGCACCAACGA
AATGTTGTGGTATGCCGTTTTGTCCCTGCGCCTTGCCGCACTGTTCTGCCGTTCGCGCCA
AGACCTGTCTTTCCCGAAAAATATGCAGTTGCGCACGGATACGGAAAGCTGCGGCTTCAT
CCTGCGTATTGACAGGGAATGGCTGGAACGCCATCCCCTGATTGCCGACGCATTGGAATA
TGAAAGCGTCCAATGGCAAAAAATCAATATGCCGTTCAAAGTCGAGGCCGTCTGAACCTT
GCGGAACAAATGCCGTCCAAACCCTGTCCAGACGGCATTTGCCTGTCCGCAACATCCCGA
TATGCGCGGCACATCTGCTCGGAACGGTCATGCAGGCGTAAAAAACAAGGGGCACATAAC
CCAAAAACCGCCTGAAAATCTTCAGGCGGTTTCGTTTGGGTTGCCGGCAGGCGGCATCCC
ATCATTTTTGCCAAGGCAACAAATTATTTGGCGGCATCTTTCATTTTGTCTGCCGCTTCC
TGAGTCGCGTCGGCAGCTTTGTTCAAAGTATCTTTAGCTGCTTCAGTTACAGCTTCTTTG
GCTTCAGTTACAGCTTCCTCGGCACTTGCCTTTGCATCAGCCGCAGCATCTTTGACTTGG
TCTTTCGCTTCTTCGACGGCAGAAGCGGCAGACTCGGCGGCAGAAGCCGCAGTGTCTTTA
ACATCGGACTCAACGGCTTGAACCGCTTCCTTAACCTCCTGTTTGGCTTCTTGCGAACAA
GCTGCCAAGGCAGCCGCCATCATTGCGGCAATCAATAATTTTTTCATGTCTTATCCTTCT
TGAGTTGTTGATTAAGGTTTTGCTTAAAAATCGGACCGTGTTCCATCAATCGGCTGATTT
TGCCCATCGACCGGAGAGAAAACGGTTTCCCGTTTAGTTAAAACCCATTATATTTAAATA
TAAAGGTTTTTTTCTCGAACAATAAGGCGGCATCAATGCCATATTGAAACACGTCCGAAA
ACTATTTTATGAAAACAGTTCGGAAAATTGTAACACATATCCCCCTCCTTTTGAGTTTCC
CGAGGGTGGGGACTTTTTCCTGCAGGGTTTGAAAAACCCAAATATATTCCGGGATGTCCG
AATACCTCAATAATGGCGGCGGCGGAAATAAAACGCCCCTTCGCTGTCGATTTCCAGCAC
ATAGCGTCCGTTCTGCACGGCGGCATAGCCGCTTTTGCCTGCCTGATAGGGTTGCAGGGC
GGCATGCGAAACTAGGTAATCCGTCAGTTTGCCGCCGTCTTCGGCGATATTGCCCACCAG
TTTGGCAAACAAGGTATGGCACACGCCGTTTTCTGCCCAACCTGCCGGACTGTCCTTATC
ATCGGTTTCCATACATTTGCCGCTGACGGCTTCCAAGTCGCCGGGATGCTTGCCGATCAG
TCGGATAACATTTTGTTCCGGCAAGCCTTTAATCGGATAACTGATTTGTTTTTTGCCGTC
GTTGGTTTTGCCTTCGCTGCTTTGTCCCAAATCCAAACCGGCAATCGCCGTATTGTCGAT
ATATTTGACTTTGAAAACCGGTTTCGGCGCGCTTTGTACCGCGTTTTGCGGCTGTTCCGC
CGTATTTTCGGATTTGCCGCAGGCGGCAAGCAGCAGGCAGCCGCCCAATACGGCAAAAGA
TGTTTTCAGCATTCCACACTCCTGATGGTTTCAAAATGCCGTCTGAAACGCGGCAGGCGG
AGGTTCGGACGGCATCGGGTTCATTTCAACGGGCGGATGCCGACCGCATCGCGTACTTTG
TCCAATAATTCGCGTGCTTCTTTACGCGCTTTCGCCGCGCCTGCCTGCAAAATCTCTTCG
ATTTGCGAAGGGTCGGCGGTCAGCTCGTTGTAGCGTTCGCGCGGTTCGGCGAGTTCGGCG
TTGATTTTCGCCGCCAAAAGTTTTTTGGCTTCACCCCACGCCAAGCCGTCGGCAAGCATT
TTCGTAAATTCCACCGTTTCAGACGGCGTGGAGAAGGCTTTGTAGATTTCAAACAATGGG
CTTTCGTCGGGCTGTTTCGGCTCGCCCGGCTCTTTCATATTGGTGATGATTTTGTTGACC
GATTTTTGGGTTTTTTTGTCGTTTTCCCAAAGCGGAATGGTGTTGCCGTAGGATTTGGAC
ATTTTGCGTCCGTCCAAACCGACCAAGAGTTCGACGTTTTCATCGATTTTCACTTCGGGC
AGGGTGAAGAGTTCCCGGAAGCGGTGGTTGAAGCGGCCGGCGATGTCGCGCGCCATTTCG
ACGTGTTGGATTTGGTCGCGCCCGACGGGCACTTCGTTGGCGTTGAACATCAGAATATCG
GCAGTCATCAGAATCGGATAACTGAACAAACCCATTTCCACACCGAAATCAGGGTCTTCC
TGCCCGTTTTCTGCATTTGCCTGCACGGCGGCTTTGTAGGCATGGGCGCGGTTCATCAAA
CCCTTGGCAGTGATGCAGGTCAGAATCCAGTTCAATTCCATCACTTCGGGAGTGTCGCTT
TGGCGGTAGAAGGTGGTGCGCTCGGGGTCGAGTCCGCAGGCAAGCCAAGTGGCGGCAACG
GCTTGGGTGGATTGGTGAATCATCTCCGGCTCGTGGCATTTGATGATACCGTGGTAATCG
GCGAGGAAGAGGAAGGATTCGGTATCGAGGTTTTGCGCCGCGCGGACGGCGGGGCGGATG

## Appendix A

GCGCCGACGTAGTTGCCCAGATGCGGGATGCCGGTGGTGGTTACGCCGGTCAGAACTCGT
TTTTTGCTCATAAAAATGTCCTTGCGGCATCAATGCCGTCTGAAAGGGAAAAAGATGTGC
CGATTATACCCGATTTGCCACCTACATCCAGCCGACAACAGACTTTTCCATATTAAGAAG
ATATAGTTATACACATTATTATACATTTTTATATACTTTAAATTCAATGATATATCGAAT
TAAATATAGAAAAACAGAAAACAGAACTTGAGTTATCCACAATTATGCACATATAGGCTT
CGACAGCGGACATTTTGAAAAGGAAACAAAAATGCGATACGACAAATTAACCGCCAAATT
CCAACAAGCCCTTGCAGAAGCTCAGAGTTTGGCGTTGGCTGCGGACGGCAGCTATCTGGA
AGCGGGCTTTGTGTTAAAAGCCCTGCTTGACGACCAAAACAGCGGAGCCGCCGCGCTCTT
GGCTCATGCGGGCGTGAACGTGCCGCAGGTGAAACAGCGTTTGCAGCAGCATTTAAACAG
CCTGCCGAAAGTGTCCGGTCAGGGCGGCGATATTCTGCCCAGCCGAGAATTGCAGGCGGT
GTTGAACCTGATGGACAAAGCTGCCACCAAACGCAGCGATGCCTATATTGCCAGCGAACT
TTTCCTGCTTGCCTTGGTACAGCAGAACGATGCGACCGGCAAAATTTTGAAAGAAGCCGG
CGCGACCGAACAAAACATCAATGCCGCGATTGACGCAGTACGAGGAGGACAAAACGTGAA
CGATGCCAATGCCGAAGACCAACGCGATGCTTTGAAAAAATATACGCTTGACCTGACCCA
GCGCGCCCGCGACGGCAAACTTGACCCCGTTATCGGTCGTGACGACGAAATCCGCCGCGC
GATTCAGGTATTGCAACGCCGTACCAAAAACAACCCTGTGCTGATTGGTGAGCCGGGTGT
GGGTAAAACCGCCATTGTTGAAGGCTTGGCGCAACGTATCGTCAACGGCGAAGTACCTGA
ATCCCTGCGTAACAAACGCTTGCTGGTTTTGGATTTGGCGGCTTTGATTGCCGGCGCGAA
ATACCGCGGCGAATTTGAAGAACGCTTGAAAGGCGTGTTGAACGATTTGGCGAAAGACGA
CGGCAACACTCTGATTTTCATTGATGAAATCCATACTTTGGTCGGCGCGGGCAAAACCGA
CGGCGCGATGGACGCGGGCAATATGCTGAAACCGGCTTTGGCACGTGGCGAATTGCACTG
TATCGGCGCGACCACTTTGGACGAATACCGCCAATACATCGAAAAAGATGCGGCACTCGA
ACGCCGCTTCCAAAAAGTATTGGTTGGCGAGCCAAGCGTGGAAGACACCATCGCTATTTT
GCGCGGTTTACAGGAGCGTTATGAAATCCACCATGGTATCGATATTACCGACCCTGCTAT
CGTTGCCGCAGCGGAGTTGAGCGACCGCTACATTACCGACCGCTTCCTGCCCGATAAAGC
GATTGATTTGATTGACGAAGCCGCCAGCCGTGTCAAGATGGAAAAAGAAACCAAGCCGGA
AGCAATGGACAAAATCGACCGCCGTCTAATTCAGCTTCGGATGGAAAAGGCGCACGTTGA
AAAAGAAAAAGACGATGCCAGCAAAAAACGTTTGGAACTGATAGACGAGGAAATCAACGG
TCTGCAAAAAGAATACGCCGATTTAGACGAAATCTGGAAAGCCGAAAAAGCAATTTCAGA
CGGTGCTGCTAATATTAAGAAACAAATTGACGAAGTCAAAATTAAAATCGAACAGGCAAA
ACGGCAAGGCGATTTGGCACTGGCTTCAAAATTGATGTATGAAGATTTGGAGCATTTGGA
AAAACAGCGTGCAGCCGCCGAACGGGCAGATACGGACAGCACAAAACCGGCAAACAAACT
CTTGCGTAATAATGTCGGCGCAGAGGAAATCGCAGAGGTGGTTTCCCGTATGACCGGCAT
TCCCGTATCCAAAATGATGGAAGGCGAACGCGACAAACTGCTGAAAATGGAAGAAGTATT
GCACCGCCGCGTGGTCGGACAGGACGAAGCCGTGCGTGCCGTGTCCGACGCTATCCGCCG
CAGCCGCTCCGGTCTTGCCGATCCGAACAAGCCTTACGGCAGCTTCCTGTTCTTGGGCCC
GACCGGCGTGGGTAAAACCGAGTTGTGTAAAGCCCTGGCAGGCTTTCTGTTCGACAGCGA
AGATCATCTGATTCGCATCGATATGTCCGAATATATGGAAAAACACGCCGTTGCCCGCTT
AATCGGCGCGCCTCCGGGCTATGTCGGCTACGAAGAAGGCGGCTACCTGACCGAACAAGT
GCGCCGCAAACCGTACAGCGTGATTCTGCTGGACGAAGTGGAAAAAGCCCATCCCGATGT
GTTCAACATCCTGCTGCAAGTATTGGATGACGGCCGCTTGACCGACGGACAAGGTCGCAC
CGTGGACTTCAAAAATACCGTTATCGTGATGACTTCCAATATTGGTAGCCAACATATCCA
ACAAATGGGCATTCAGGATTACGAAGCGGTGAAAGAAGTTGTGATGGAGGATGTGAAAGA
ACATTTCCGCCCCGAAATGATCAACCGCATCGACGAAGTGGTCGTGTTCCACGGACTGGA
TCAGGATAATATCCGCAACATTGCGAAAATCCAGCTCAAAGGCTTGGAAAAACGTTTGGA
AAAACAAAACCTGCGCCTGGCTGTTTCCGATGCCGCACTGGACATCATCGCCAAAGCCGG
TTTCGACCCGATTTACGGCGCACGTCCGCTCAAACGCGCCATCCAGTCGGAAATCGAAAA
CCCGCTGGCAAAAGCCCTGCTTGCCGGAAACTATGCGCCCGAAAGCGAAATCAGGGTGGA
AGCCGACGGCGACAGACTGAAATTTGCCTGATTCGTTCCTGCTGTTGAAAATGCCGTCTG
AAACGGGAATCTCCGTTTCAGACGGCATTTTTTATCCTCGGCAGACAAACCGTCCCCTTA
TTGGCGGTAGGTTTGCAGGAATCTTGCCAGCCTGCCCATCGCCTCTTCAATCTGATGGAC
GTAAGGCAGCGTAACAATGCGGAAATGGTCGGGCTTGATCCAATTAAACCCCGTTCCCTG
CACCAGCAAGACTTTTTCGCGCACCAGCAAATCGTAAACGAATTTCATGTCATCGCGGAT
ACGGTACATTTCGGTATCGATTTTTGGGAACATATACATCGCGCCCATCGGTTTGACGCA
GGATACGCCGGGAATCTGGTTGACCAGTTCCCACGCCCTGTTGCGCTGTTCCAAAAGCCG
TCCGCCGGGCAAAATGAATTCGTTGATGCTCTGATAGCCGCCCAATGCCGTCTGAATCGC
GTGCTGCATCGGCGTATTGGCACACAGGCGCATAGACGAGAGCATATCCAAACCCTCGAT
GTAACCTTTTGCATGATGTTTCGGCCCGTTGAGCACCATCCAGCCTTGGCGGAATCCGGC
TACACGGTAGGCTTTGGACAAACCGTTGAACGTTACCGTCAAAAGGTCGGGGGCAAGCGC
GGCGATGTGGTGGTGAACCGCGCCGTCATAAAGGATTTTGTCGTAAATCTCGTCGGCGAA
AATAATCAAACCGTGCTTGCGCGCCAGTTCGGCGATTTCCAACAGGATTTCCCTGCTGTA
CACCGCGCCTGTCGGATTATTGGGATTGATGACGACGATGGCTTTGGTTTTGGGCGTGAT
TTTGGCTTCCATATCGGCAAGGTTGGGGAACCAGCCGTTTTCTTCGTCGCACAGATAATG
GCGTACCGTACCGCCCGCAAGCGTTGCCGCCGCCGTCCACAAGGGATAGTCGGGCGCGGG
AATCAGGATTTCGTCGCCGTCGTTGAGCAATGCCTGCATAGACATCGTAATCAGCTCGGA
CACGCCGTTGCCGATATAGACATCATCAACCGTAATATCGCGCAAACCTTTGGTCTGATA
GTAGTGAACAATGGCTTTGCGGGCGGAATACAGCCCTTTAGAATCGCAATAGCCTTGCGA
AGTCGGCAGGTTGCGGATGACATCGACCAAGATTTCATCAGGGGCTTCAAAGCCGAACGG
CGCAGGGTTGCCGATATTGAGTTTAAGGATTTTATTGCCCTCCTCTTCCAACTGAAGGGC
TTTTTTGTGAACCGGCCCGCGTATGTCGTAACAGACGTGATCGAGCTTTGCAGACTTGGG
AAATTTATCCATGATGTGTTCCGTAAATTTTGGGCAATGGGTGGAATGTACTCTTTTTC
ACGCGGAATTTAAAGCATCAAACCGAGATTTTCAGGCTTTTTACCTGCCCTCTTTGCGCC
GTTCGCTGACGCTTTTGCCGCCTATTCCCCAGTTATCGGTATCCACTTCGTCAATCACGA
CAACCGTTGTTTCGGGATTTTTGCCCAGCACGCGTGCCAGCAATTCGGTTACGCCGCCGA
TCAGTTCCGCTTTTTGCGCGGCAGTCGGTGCTTCCTTGCCGCCGGTTACTTTAATATTGA

## Appendix A

CATAAGGCATGATCTTTCTCCGTTTTAAAATATTGCTATCTTATCAAACAAGTTGCCTCC
GCCCAAACGTCCGCTTCATTTTCTGAAAAATTCAAATCGATATAGTGGATTAACAAAAAT
CAGGACAAGGCGACGAAGCCGCAGACAGTACAGATAGTACGGAACCGATTCACTTGGTGC
TTCAGCACCTTAGAGAGTCGTTCTCTTTGAGCTAAGGCGAGGCAACGCCGTACTGGTTTT
TGTTAATCCACTATACAAAAAGACAGTTTTCAGACAGCAAATCCGTCTTCACACGATACC
TATTTTGTTATAACATAACAAAATCTTTAACCCACACGAGACAAAGGCTGCACCATGAAG
AAAACATTGACACTGCTCGCCGTTTCCGCCCTATTTGCCACATCCGCCCACGCCCACCGC
GTCTGGGTCGAAACCGCCCACACGCACGGCGGCGAATACCTTAAAGCCGACTTGGGCTAC
GGCGAATTTCCCGAACTCGAACCCATCGCCAAAGACCGCCTGCACATCTTCAGCAAACCG
ATGCAGCTGGTTACCGAAAAAGGCAAGGAAAACATGATTCAACGCGGCACATACAACTAC
CAGTACCGAAGCAACCGTCCCGTTAAGGACGGCAGTTACCTCGTCATCGCCGAATATCAG
CCTACTTTCTGGTCAAAAAACAAAGCAGGCTGGAAACAGGCGGGCATCAAAGAAATGCCT
GACGCAAGCTATTGCGAACAAACCCGAATGTTCGGCAAAAACATCGTCAACGTCGGACAC
GAAAGCGCGGACACCGCCATCATCACCAAACCGGTCGGACAAAACTTGGAAATCGTCCCG
CTGGACAATCCCGCCAACATTCACGTAGGCGAACGCTTCAAAGTCCGCGTTCTGTTCCGT
GGCGAACCGCTGCCCAATGCCACCGTTACCGCCACCTTTGACGGCTTCGACACCAGCGAC
CGCAGCAAAACGCACAAAACCGAAGCACAGGCTTTCTCCGACAGCACAGACGACAAAGGC
GAAGTGGACATCATCCCCTTGCGCCAAGGCTTCTGGAAAGCCAATGTCGAACACAAAACC
GACTTCCCCGATCAAAGCGTGTGCCAAAAACAGGCGAACTACTCGACTTTAACCTTCCAA
ATCGGTCATTCGCACCATTAATCCCGCCCGCACAAAAATGCCGTCTGAAGGCTTCAGACG
GCATTTTTTGTTCAAACATCAATACCAACCGCGCAGTTTCATCGCTTTTTCAACACGGCG
GATACTCATCATGTAAGACGCGGTTCGCAAATCGACATCATACTCTTGCGCCAAGTTCCA
TATATCGCGGAACGCGCGTCGCAGGACGACGGTTTCTTTCTCTTGAACTTCGTCAAACTC
CCAATAATAGCCTTGCAGGTTTTGCACCCACTCGAAATAGGAAACGACCACGCCGCCGCA
GTTCGCCAGAATATCAGGCACGACCAATACGCCGTTTTGACGCAGGATCACGTCGGCTTC
GGGCGTAGTCGGGCCGTTCGCGCCTTCGACTACGATTTTCGCGCGGACTTTACCGGCGTT
TTCGGAAGTCAGTTGGTTTTCCAGCGCGCAAGGGGCGAGTACGTCCACATCCAAAGCCAA
AAGTTCGGCGTTGGTAATTTCTTTGCCGTAACCGGCTTCGTTGGTGATGAAGCCTTTTTC
TTGGAACTCTTTAAACAAAGCTTCCATATCCAAACCGTTTTCGTTGTAAATGGCAACGTC
AACAGTAGAAACCGCAACAACTTTCGCGCCGGATTGATGCGCGTAATAACCTGTGTGGTA
ACCCACATTACCGAAACCTTGAATGGCGTAAGTGGCACCCTTCACGTCCTTGCCCAGTTT
TTCCAAAGCTTGGACGGCGGCGAGGTTCACGCCGTAACCGGTAGCCTCGGTACGCGCCAA
AGAGCCGCCGAACTCAACCGGTTTTCCGGTAAATACGCCCGGCGCGGAATGTTTCACCAC
GTTTTCATAAGCATCCACCATCCACGACATAATTTTGCCGTTGGTATTCACATCGGGGGC
GGGAATATCGATTTTCTCGCCAATCAGCGGGGCAATCGCTTCAGCATAAGCGCGGGCGAT
GCGTTCCAGTTCCGCCTCGGAATAATCGCGCGGATCCAAGGTAATGCCGCCTTTGCCGCC
GCCGTAAGGAATACCCGCAACGCAGCATTTGATGGTCATCCAAATTGACAGGGCTTTGAC
TTCGTCCAAATTCACACTGGGATGGAAGCGCACGCCGCCTTTATAGGGGCCGACGGCGTT
GTTGTGTTGCGAACGGTAGCCCGTGAAGGTTTTGACCGTGTCGTCGTCGAGTTTGACGGG
AAAATTGACTTCCAACACGCGGGTCGGACTCTTCAGGATTTCATAAACGGCCGGATCGGT
TTTCAGCCGGTCACAGGCGGTTTTCACCTGTTTGCGCGCGATTTCAAACGGATTGAGGGT
TTCTTTTGCAAGGGCTTCAGACATTTTGCTTCCTTTTCACAAAGAGAGGTTCGGAATGGA
ACAAGCCATCAGGTTCGCAACTATAACCAATTTTCAAGCAAAATGTAATAGCGTGTAGTT
GGAATCGGCCCGATTTGATTAATCTATATATGATTTTATTTCCCAAGCCGCACGGAATCC
GTCTGAAAAAAGCGGAACACATATCCAAAAAGCAAATGTCCAATTAAATAAAGATATAAG
AATCCTTTTATTTTTTAAAAATTTAATTGGAACGGCGCCGGGATTTGCACACCCTTCCCG
ACTCCGTTCCGAAATCCGGAAACACCGCCGGCAAAACCTGTTTCGATTGTTAACAATCCA
TACATTAGAAGCCCTGTGCAAACGATGTTAAAATAAACCTTTTCAACCCGACAGAAAACC
GGATTATGAATGCAGCCATCGAACACGTCCAAGCCGTCGCCTTCGATTTGGACGGCACAC
TGTGCGATTCCGTCCCCGACCTTGCCGCCGCCGCAGAAGCGATGTTGGAACAACTCGGTA
TGAAACCGCTGCCTGCCAAAGTGGTCGAAAGCTATGTGGGCGACGGCATCGGCAAACTGG
TTCACCGCGTCCTCACCAACGACCGCGACCGCGAAGCCGATTCCGAACTGTGGGAAAAAG
GTTTCGTATCTATATGAAATACTACCGCGACCATTTGAGCGTCTTCACCCGCCCCTATCC
CGAAACCGAAGCCGGGCTGGCATTGCTTAAATCTTTGGGCATCCCGCTCGCCGTCGTTAC
CAACAAAAACGAAATCCTTGCCTCCGAGCTTCTAAAACAACTGGGACTCGCCGACTATTT
TAGCCTGATACTCGGCGGCGACAGCCTGCCCGAGAAAAAACCCAGCCCCCTGCCGCTGCG
GCACGCCGCCGAAGTTTTGGGTATCGATGTTGCAAACATGGTTATGGTCGGCGACTCGCG
CAACGACATCATCGCCGCCAAAGCCGCCGGCTGCCTGAGCGTCGGCGTTACCTTCGGTTA
CGGCGATATGACGCTGCTCTCGCAAGACGATGCGACCCGCCCCGACTGGATTATCGGCTC
GCTGCCCGAAATTTACGAAAACCTGCAACCTCAGAAAAACAAAGAAGAGTAGGCATTCGG
ACGGCTCCGGTTTGCGCCGCTATGCCGTCTGAAACCTGCCCCACGCCGAAACCGCCGCCA
TGAAACCGCAAAAATCCCTACGCGCCCGCGCGATGGACATCCTCTCGCGCCAAGAACTCA
GCCGCATCGGTCTGAAACGCAAACTTGCACCGCACGCCGAAAGCGAAGAGGAGTTGGAAA
ACGTGTTAAACGAATTTGCCGAACGCAACTGGCAGTCGGATTTGCGCTATGCCGAAGCCT
ATATCCGCAGCAAAAGCCGCAAACACGGTTCATTGAGGCTGAAACAGGCTTTGGCGCAAC
AGGGCATAGATGAAGAAACCAGCCGCAACCTGCTTCCCGACCGCTCAAGCGAAAAACTGG
CCGCCATAGCCGTGTTGCGTAAAAAATTCAAACATCCGGCCGCCGACCTTAAAGAAAAAC
AAAAACAGGCACGCTTCCTCGCCTATCGCGGTTTTGATGCCGATACCGTTCAGACGGCAT
TGAAACATGCCTGGGATGACGGCTGGGAGGAAGACTGCTGAACTGAATCCTTGAATCTTT
TTGCATGACGGCGTAACCTTACCTCCATTTCCAACTTTTCCGATTGAGAATAAAATGTCC
GAACAATCCGAGAAAAATCACAACCCACTTCTTGAAGATGAACGCAAAAACCCGGTTTAC
CGTATGGGTCAGGCAGTTGCCGGATTCATGCTCGTCGTTTGGGCAGGCGTATTGGCACTC
GTGTTTTTCCTAGTCTTCCGTTTTTGGCTTTCCTAAACAAAATGCCGTCTGAAACCTTCA
GACGGCATCGGCAGCCCATTTCGGCAGGCTATCCCATCATAGCTTTTTTAGCTTGAATT
CCACTTTCCCATTCCCTAAAATTTTTCCACACCCATTTCAAAATACCCTTTCTTAAAACA

## Appendix A

GGTACACTATGACACAACAACGCCAACTGCCTTCGCACGAACTCATTATGTCCGAACTGA
TGATGCCGGACACCGCCAATTTCAGCGGCAACGTACACGGCGGCGAACTCCTGCTCCTGC
TCGACCAAGTCGCCTATTCCTGCGCCAGCCGTTACAGCGGCAATTATTGCGTTACCCTGT
CGGTTGACAAAGTCCTGTTTAAAGAACCCATCCATGTCGGCGACCTGGTTACTTTCTACG
CCAGCGTAAACTACACGGGGCGTACCTCTATGGAAATCGGCATCCGTGTCGAAGCACAAA
ACATCCGTACGGGAGAAATCCGCCATACCAACAGCTGCTACTTCACCATGGTTGCAGTCA
AAGACGGCAAACCCGTCCCTGTCCCTCCGCTGGAAATCCTGACCGACCGCCAACGCTGCC
GCTACGAAAAAGCCAAAAAACGCAGAGACATCAGCCTGCAAGCCTCCGGAGACGTGTCCT
GCGGCTGCTGACGGCGGACTATGCCGTCTGAAAGACAGGCACATCGCGCCATCCGTTTCC
ATTGCAAACGGATGAAATCAAGCAAATATAGTGGATTAAATTCAAACCAGTACGGCGTTG
CCTCGCCTTAGCTCAAAGAGAACGATTCTCTAAGGTGCTGAAGCACCAAGTGAATCGGTT
CCGTACTATCTGTACTGTCTGCGGCTTCGTCGCCTTGTCCTGATTTTTGTTAATCCACTA
TACCCAAACACAGTCAAACAAATTTATATGCCCCATCCCTTCCGAATAATTTGAAAACAC
AGCCGCCAAAAACAAAAATGCCGTCTGAAAACCTTTCAGACGGCATTTCCAACTTGATTT
CAGGCAGAAAGTCAGAACGCGATATAGCTGTTCGGGTTAACCGGTTTGCCGTTTTGACGC
ACCTCGAAATGAAGCTGCGTTCTGGAAGCATCGGTATTGCCCATCAAAGCAACCTGCTGA
CCGCGTTTGACCTGCTGCCCCTCGCCGACCAGCAATTTTTGGTTGTGCCCGTATGCGGTC
AGGAAAGAAGAATTATGCTGGATGATGACCAAGTTTCCGTATCCCCTCAAACCTGAACCG
GCATAAACCACTTTGCCGTCAGCCGCCGCCAAAACGGGCTGTCCCGCATTACCGGCAATA
TCGACACCCTTGTTGTTGCCGCCGAAATCGGCAACCACTTTACCTTGCGTCGGACGCTGC
CAAACAATGCCGCCGACCGAACGCGTGCCGGAAGGCGAAGCGGCAGGAGATTGCGGGGCG
GGCGCGGGAACCGCTTTATTTTCCGCAGCGGGCGCGGCAGGTTGCGGCGCGGACTGCACA
GGCGGTTGCGCGGCGGGTTTCACAGGGGTTTGCACGGCAGCCGGTACGGCGGGCCTGCTT
TCTACGGCTGCGGTTTTCGGTGCGGCATATCCTGCCGGTTTGACTTTAACAATCTGACCG
ATGCTCAACATATTGTCGGTCATGCCGTTCCACGCACGGAAATCGTCTTGAGAGATATGG
TAGCGTTTGGAAATGTTGTACACCGTGTCGCCGCGCACAATAGTATGCGTCGCCGCGTTA
ATGTCGACGGGTGCGGACTGTACGGGCGGTTGCGCGGCAGCCGGTACGGCGGGCCTGCTT
TTTACGGCTGCGGCTTTCGGTGCGGCATATCCTGCCGGTTTGACTTTAACAATCTGACCG
ATGCTCAACGTATTGTCGGTCATGCCGTTCCACGCACGGAAATCGTCTTGAGAGATATGG
TAGCGTTTGGAAATGTTGTACACCGTGTCGCCGCGCACAATAGTATGCGTCGCCGCGTTG
ATGTCGACGGGTGCGTAAGAAGGAACGTATGTACCCGAAACGGCAGGTGCAGACGGCGGA
ACATAAGCAGGAGGCGTATAAACCGGCGCGCTTTGCACCGGCGGCACATAAGGCGCATCG
CCGGCAGGAGCCGGGCTGTACGGCGTTGCTCCATAGGGGTTGTTGTAAACTGCCGAAGAC
GGCGCGTCCTGCATACCTGAATTGCCTGCAATGACAGGAGCAGGCTGTTGGGTGGCGCAA
CCGCCCAACAGAGCGGCAACGGCGGTACAAGCTGCCAAAAGTGTCGTTTGTTTCAACATA
AGATAACCTTCATGTTCCGATATATAGCCTGAATGCGGTATATCATAATAAAAATGCGCG
TTCTTCTCAAGCGCAAAGCCCGACGGTATAGTGGATTAACAAAAATCAGGACAAGGCGAC
GAAGCCGCAGACAGTACAAATAGTACGGAACCGATTCACTTGGTGCTTCAGCACCTTAGA
GAATCGTTCTCTTTGAGCTAAGGCGAGGCAACGCCGTACTGGTTTTTGTTAATCCACTAT
ATTTGATGAAACGGTCAGTCCGCATGCCAGAACGCCGCTGTTTCCGCCATGTCCGGATAG
GCGGTCAGGTCGATTTGCAGCGGCGTTACGGTAATGAAACCTGCGCCGCATTCACCGAAA
TCCGTTCCCTCTTCCCGATCGGAAACTTCGCCGACCGGTCCTATCCAATAAATCTGTTCG
CCGCGCGGATTGCGCGCGGGAATGACGTTCTGACCGTGATGCCTCCTGCCCAAACGGGCG
ATTTTAATGCCCCGCACATCTTCCGGCGCAACGGCGGGGATATTGATGTTCCACAAAATA
GGGGACTGCGGGGGGTTTTTGAAAAAATGCGCCAACAATGTCCACAGTGCCTGTTCTGCG
GTCGCCCAATAGCGTCCGGAAGCGTCGTTTAAGGAAAACGCCACGGCGGGTATGCCCATA
AGGTAGGCTTCGGTTGCCGCCGCAACCGTCCCCGAATAAAGCGTGTCGTCCCCCATATTC
GCGCCCCGGTTGATGCCCGAAAAGACAAAATCGGCCTGAAAATCCGAAAATACAGACTGC
CCGATGTGGATGCAGTCGGTCGGCGTGCCGTTGACATAGTAGAACCCGTTTTGCGCCTGT
TTCAACTGCAAAGGGCGTTCCAGCGTCAGCGAATTGCTGACCCCGCTCCTGTCGCGTTCG
GGCGCGACCACCCTGACGTTGGCAAATTCCGCCGTAACGCGCGCCAAAACGGCAATGCCT
TCGGAGAGGTAGCCGTCGTCGTTGGAAATCAAAACGTTCATTTTCTATCCTGAATGCTTA
TTCTTCGGGCAATTTGGTGATTTTGACCCGCTCGATGCGCTGCCCTTCTTTTTCGACCAC
TTCAAACCGCCAGCCGTGGAAATCGGCAAAATCGCCGACATCGGGGATGGTTTGCAATTC
TTCCATAATCAGCCCGGCAACCGTATGGAAATCGGCATCTTCCTCCTGCTGCGGCAGGTT
GAGTTGCGGTGCGAGTTCCACATATTCCAACGCGCCTTCCACCGTCAGGCTTTCATCGGG
ATTCCCCTGAACGGCTGGTTCTTCTTCGCGCTCAAATTCTTCGGGGAACTCGCCTGCGAT
GGTTTCGAGCAGGTCTTTCATGGTTACCATGCCCAATACCGCGCCGAACTCGTCCACCAC
CAAAGCATAATCCGCGCTGCTTTGGCGGAAGAGTTCGATTGCGCCCAGCGCGGTGGTGCT
GTCGGGCAGGACGAGCGGCTGGCGCAATGCCGTCTGAATGTCGAGACCGCCTGTTTCCAG
CAGTTGGGACAGCAGGTCTTTTTTGTTGATGTAGCCCAAAGGTTCGTCCACGCCCGCCTT
ACCGACAACGAGCAGGCGGCTGTAAGGCGTGTTTTGCAGTTGGGCACACTGTTCTTCGCG
GCTTTGGGAAATGTCCAGCCGTTCGATGTCGCGGCGTGGGATCATCACCCCCATAATCGG
GCGTTCGGCAAGCGTCAGCACGCTGCGTATCATCGATTTTTCGTTTTCTTCAAAATGCGC
GTCGTCCCCGGATTCGCCGCCCGCGTCGGCAAGCACGCTTTCGCGTATACCCATCATACC
CAAGACGTTTTCGGCGGTGCGCTTGCGCCACGAGCTGCCGATGTAGTCGTTTTTGCGGCT
GTTGCGCTGCGAAATCTGGTTAAACAATTCGATTAAAATCGAGAAGCCGATGGCGGCGTA
GAGGTAGCCTTTGGGAATGTGGAAATGGAAGGCTTCGGCAATCAGGCTGAAACCGATCAT
CAACAAAAAACCAAGGCAGAGCATCACGACGGTAGGGTGTCTGTCGACAAATTCGGTCAA
GAGTTTGCTGGCAGAAATCATTACAGCCATCGCGACGACGACCGCACCCATCGCCACGAC
GATATGATCGACCATCGCCACCGCAGTAATGACCGAATCGATGGAAAACACGGCATCCAG
TATCAGGATTTGCGCGACCACGCCCCAAAACGGCGCGTGTTTTTTTGGCTGTCGGCAAC
GGTAAAACGGTTGTGCCCTTCGAGGCGTTCATGCAGTTCGGTGGTGGCTTTGTAAAGCAG
GAAAATACCGCCCGCGAGCATAATCATGTCCTTGCCGGAAACGGCGAGGCCGCCGATTTG
GAACAGCGGCTCGGTCAGCGTGATGATGTGCGCCATAAAAGCAAGCATAATGATGCGGAT

# Appendix A

GACGACTGCCAGCCCCAGCCCGATAATCCGTGCGCGGTCGCGCCGTGCGGGCTGGACCTT
GTTTGCCAAAATCGCCACAAAGACAAGATTGTCTATCCCCAATACGACTTCCAACACCAA
AAGCGTGGCAAAACCTATCCAGGTATGCGGTTCTGCCAACCAACTGAAATCCATGATTTT
CGTATTCCTCAAGTTCAAACGCGAAAAGGCAGCCTGAAGCGCTCAAGCTGCCTGAACAGA
CGGTACGCACAAAAAACGGCGGGCGGGCTTGCTGCTCTGCTCGGGGTCTTGCATGTGCGT
GTACCTTCGGTCGAAATAATTTAAATAGTTTAACAGCTTATCGGGGCAATGGCAAAACGC
CATACCGTCTGAAAGGATGTTCGGACGGCATGAGCTTATTTTGAAATGTTTCAACACACG
GACGGCACATAAAGCCTTCCCCTATGTGTTGCCCTGATTGAGGGGTTGCGCCCCTCTCAA
ATACAGTCTGATTCTACCGCCGCGAAGAACGGATGTTCGAGTGCGGACGGAGTCCCAACG
CTTAAGGGGTGATGATGAAGCCGTCTATCGGCGCGTAGCCTTTGGTGTTGCCCTCTTTAT
CGGTAATGACTATCCACTCTTTCTGCCTGCTGCTGGTAAACGGCAGGTAATACAGCTCCT
CCCCTTCGGCGAGACCTGCCTGTTTCAGAATGTCCGCAACCGTCGTTTTTCTCGCATCCG
CCAAGACTTTCAGCGGTTTCAGATGTTTGCGGATTTCTTCTGCTTCCTTGTCGGAATACG
GCAGCCACTGGTCGGGACGCATACTCGGCTCGATACCTTTCAGGGACAAATCCAGCGTCT
TGTTCTTCTCATCCGCATCCTCAGGTTCTTTCAATGCAATGCGGCGGATGCCGAACCACG
ACAGGCTTTGCAGCCCTTCGGGGGCTTTGTGCAAATCTTCGACCACGACTTCCGCCGCCG
TAACAATGGTCATACGATCCTGTTCAAACGCTTCCACCACAGGACGCGCCAGCGAAACGC
TGTGCAGACCGTACACCAAAGCCGCCAGCTGGATGATGCCGACCATGGAAAAATCGACCA
TGCGTGCCTTTGTCTTTTTCTTCGGGCTTGCCAAAATTAAAGTCAGCAGCGGACCACATA
CAATATCGACAGCCACCACCAGCTGATAAAGCGACAGCCCTCCCGTCAGCTCGGCATAAG
GATAAGGATACCAAACCTTAAAAACCAGCAATGCCGCCAGCCCTGCAACCGACAGGCTGA
TTAAGAGGTGCCAGCCCGCACTTTTCAAGGCAAAACGCCATCTCGGGACTGTTTTTCCGT
TTTCCATCATATCTTGTTCAAATCAAAAATAACCGTAAAAACAGGGCGCATTGTACAACA
GATAGAGACTGCTTAAAATGCGGCGCCGTCTGAAATCCTGCCGTTCAGACGGCATCCGTC
ACCCGACATCCATACACAGATATTTCAATTCTAGATATTCGTCCGCACCGTATTTGCTGC
CTTCACGTCCCAAACCGCTACGTTTCACGCCGCCGAACGGTGCCGCTTCATTGCTGATTA
AGCCCGTATTGATGCCGACCATACCGTATTCCAAGGCTTCGCCGACGCGCCATTGGCGGG
CGGTGTCGGCGGTGAAAAGGTAAGCTGCCAAACCGTATTCCGTATTGTTCGCAGCCTCGA
TGACCTCGGCTTCGGTTTCAAAACGGAATACCGGACACAACGGCCCGAAGGTTTCTTCGC
GTGCCACCGCCATTTGCGCCGTTACGCCGCTTAAAACAGTCGGTTCGAAAAACGTTCCGC
CCAACGCGCTGCGTTTGCCGCCGGTCAGGCAGCTTGCACCTTTAGCAAGCGCGTCGGCGA
TGTGCTGCTCGACTTTCTCCACCGCTTTTTCCTCAATCAGCGGCCCTTGGTTCACACCAT
CCTCCAAGCCGTTGCCCAATTTGAGCGCGGCTGCTTTTTCACTCAATTTGCGGCAAAATT
CGTCGTAAATGGCGGATTGAGCGTAAACGCGGTTGGTGCAGACGCAGGTCTGACCGCTGT
TACGGAACTTGCTGGCGAGCGCGCCTTCGACGGCTTTGTCCAAATCGGCATCGTCAAACA
CGATAAACGGCGCGTTGCCGCCCAGCTCCAAACTGAGTTTTTAATGTCCGCCGCGCTGT
CGGCAAAAATTTTGCGCCGACTTCGGTCGAGCCGGTGAAGCTGATTTTGCGGATAATCG
GGTTCGTAGCAAATTCATGGCCGATTTCCGAAGCACTGCCGCTGACAACAGGCAACAAAT
CCTGCGGTATGCCCGCTTCGTAAGCCAACGAAGCCAAGGCATACGCACTCAAAGGCGTGA
GCGATGCGGGTTTGACGATCATCGCGCAACCCACCGCCAAAGCAGGCGCGGCCTTGCGCG
CAATCATCGCGGACGGAAAGTTCCACGGCGTAATCGCAGCGGTAACGCCGACGGGCTGTT
TCAACACGACCAGTTTTTGCGACGCTTTCACACTCGTCAGCACATCGCCGTCAATCCGCC
GCGCCTCTTCGGCAAACCAGCGCACAAACGAAGCCGCATAATCGATTTCGCCACGCGCCT
CGGTCAGGCTTTTGCCCTGCTCCATCGTCATCAGGCGCGCTAATGCTTCTTTGTTTTCTT
TAATCTGAAAATACCAACGCCACAACACATCGGCGCGTTCCAACGCAGTTTTTGCCGCCC
ATAATTTTTGTGCTGCAGCTGCTTTTTGAATCAGGTTTTTCAGCTTGTCCGAATCCGTCT
TGCGGACAAACGCCAAAGTCTCGCCCGTTGCCGGATTATCGACTTTGATGCCGTCTGAAA
CCGGGGGAAGGGAAATATCGGGATGCTTGATTAATTGGGAATATTCGTTCATTTCGTATC
CTCCGGTATGCGGAATAACCGCTTTCAAATGCCGTCAATCTCGCGGACATTATCATCTTC
~~ATATTCCAAAACTGCAAACCCTTCCGATGCCGTCTGAAGCATCCGATCGGGCAGCGCAAC~~
ATCCGGGCGGTGTCTGAATATGGCGCGGGCGCAATCCCTGTCGTTTAAGAAAAATATTTT
TTATACGATAGTAATCTTTAGAAAGAAAAGTAATGCAGCCCTTTGATGGGGTGCAATATA
TAAGGAGCAAAGATTGCAGTTGCAACGTGTGGTAGAGTATGGCAAAAATCCGAACATTAT
AGTGGATTAACAAAAACCAGTACAGCGTTGCCTCGCCTTAGCTCAAAGAGAACGATTCTC
TAAGGTGCTGAAGCACCAAGTGAATCGGTTCCGTACTATTTGTACTGTCTGCGGCTTCGT
CGCCTTGTCCTGATTTTTGTTAAATCCACTATACAGTCAAAATTACGGAGATCAAATAAT
GATTTTTAAACAGAATCAAAATTATTGGGCAGTTTTTGATGCTAATAAAGAAACTCTGAT
TGTTCAAACATGTTCAGGTTTGGGGTTAACGGCAATAGACCACCTATATCCCCCCCATAT
CCTGCCATTGGATACCGACAATGAAACTTTAGGCACGACAGTCTTGCAAGCGTTGGCAAA
CAGCAGGACTTTCGTTTATGACAGTCCAGAAGACCAAGATTTTTTTGATACCGAAAAAAT
TCGGCAACGCTATGAGGATTGGGTTGCCAAGCTATGCGGGAACTTGGGCTATAAAACCAG
ACGCGCCCTATTTAAAAACATGATGAGCGTGGATATTTGGCTGCACAACGGCTGCCTGAA
AATCAGCCCGAGCCGCCATGTCAAGCTGGAAGCGTGGAATGCCATTGATGCAGACGATGT
CATTTTATCATTGGATAACAGCCCTGAAGAAATCGGAGCAGGTTTAAAGTTGGCATTGAG
CCACTGCCGATAATATTTGACAAAAGGCCGTCTGAAAAACAGCTTTGACAAAGACGCGGT
TGCCAAAGAGATCGACCTACAAAGGGAAGTAACGCAGGCGTTCGGCAAAACGCCGCCCAA
GCCGCAGCGGCCGTTGCCGACGAACTCGGCAATACCCGAAGTTACGGACGGTATCAGGCA
GCCCGAACCCTGCCGGAGGCCGAACTGCAAAACAAATCCGACACAAAAAACCGTCCGTCC
GACACAAAAAACCGCCCGAAAAATCTGGACGGCGGTTCAAACAGGCTGCCCCGTTTAACG
GGCGCGGCAGGAAGTTTCGACCGAATTGCCGTAGGCATCGGTAAAGCCGAAAAAGGCTTC
GCCGCCTTTCTGGTGCCACTCGGTTGCGTTTCCGAACAAACCGTGTTCGGCGGTATAGCG
TTCGCCGGATGCGGCAACGTCGGAAGAGAGGACGGCACGCCTGCCGTCCAGCCGCAACGC
GACTTTGCCGCTGTCCAAATGGCGGACGCGCACAGACAAACCGTTCTCGCAGGAAAACGC
CCGAAAATCGTCCGTGCCGGCTTGGTTTTGAACGGGCGGCATATGCCCGCGTCCGCCGTC
ATCATACGCCTCCGGCACGGCACAGGCCGCCAAAGACAAAACCGGTACGGTCAGCGCGAA

## Appendix A

AAACCTGATATTCATAAAAGCTCCCCAATAAAAATAAGATATGAAACAACCGCCCTGATT
CCAAGCTGCGGCAACGCCATACTATAAACGGACGCGCAAACACACAAGCCCGATAACCGG
AATTTACCTGCGATGAATCAATAATCCGGATTGCGCGCCCCTTCTTTACCCCTCTTCCGA
TGCCGCCTTTTGCCTGACGATGCCGTCTGAACCTGCCTGCCCGCCCGGAGGAATGTAAAT
TTTTTCCAAATTCCAAGTAAAAACCGCTATCGGTGTGCTAATTTGCGTTAAAATCCTATT
CGGCGTTTAACGTTTTGTGCGCCCGCATCCCTGCACTGTTTGATGCGGGCATAAGGCACA
AATCCCGACAAGCGCACTGTTTCATACTTCGTCAATCATTCAGACTCCGGTTTGTGCCCG
TGCCGGCAGATGGTTCGGCCGTTTCCCGCCGTTCAGGCATATTCCGACAGTGTGAGATAA
GGATTTATTCGATGAAATCACTCAAAACCTTCCTCATTTGGGGCATAGTGGTACTGGTCG
GCTTAGCATCCTTTACCACTCTGGCCCTCAGCCGAGGCGAACAGGTCAGCGCGGTATGGA
TGGTCACCGCCGCCATATCCGTTTACTGCATCGCCTACCGTTTTTACAGCCTCTACATCG
CCAACCGCGTAATGCGGCTCGATCCTGACCGCCTGACTCCGGCAGAACGCCACAACGACG
GCTTGGACTACGTTCCGACGCACAAAGGCGTATTGTTCGGACACCACTTTGCCGCAATTG
CCGGCGCGGGCCCTTTGGTTGGTCCGGTTTTGGCGGCGCAAATGGGTTATCTGCCCGGTA
CTTTGTGGATTATCTTCGGCGTGGTATTTGCCGGCGCGGTACAGGATATGATGGTCTTGT
TCGTCTCTATGCGCCGCGACGGTAAGTCTTTGGGCGATATTGTGAAACAGGAACTCGGCA
CTGTCCCCGGCGTGATTGCCTCCATCGGTATTTTGATGATTATGGTCATCATTATGGCGG
TGTTGGCGTTGATTGTCGTAAAAGCATTGGTTCACAGCCCTTGGGGTACGTTCACCATTG
CAGCAACTATGCCGATTGCGCTGTTTATGGGTATTTACACGCGTTATATCCGTCCGGGCA
AAATCGGCGAGATTTCCATCGTCGGCTTTATTTTGCTGATGCTGGCGGTAATTTACGGCG
AAGATGTGGCTAAAAGTTCCATCGGGCATTGGTTCGACCTTGACGGCATCCAGCTCACTT
GGGCGATTATGATTTACGGCTTTGTCGCCTCCGTATTGCCCGTATGGTTGCTGCTCACTC
CGCGCGACTATCTCTCCACCTTCCTGAAAATCGGTACGATTGCGGCCTTGGCTTTGGGTA
TCGTCATCGTCAATCCCGCTTTGCAAATGCCTGCCGTAACCCACTTTATCGACGGTTCGG
GTCCGGTATTCTCAGGCGCATTGTTCCCATTCTTGTTCATTACCATCGCCTGCGGTGCGG
TTTCGGGCTTCCACGCGCTGATTTCTTCCGGCACTACGCCGAAAATGCTGGAAAACGAAA
CCCACGTCCGCATGATCGGTTACGGCGGTATGTTGATGGAAAGTTTCGTAGCCATTATGG
CACTTGCCGCTGCCGCATCGCTTGATCCCGGCGTGTACTTCGCCATGAACAGCCCAGCCG
CCCTGATCGGTACGGATGCCAATACCGCCGCCGAAGTGATTACCACCAAGCTGCAATTCC
CTGTCGATGCCGCAACCCTGTTGCACACTGCTAAAGAAGTCGGCGAAAACACCATCCTTT
CCCGTGCCGGCGGTGCGCCCACCCTCGCAGTCGGTATGGCGCACATTATGAGCCGCCTGA
TTCCGGGCGAGGCGATGATGGCGTTCTGGTATCACTTCGCCCTGTTGTTTGAAGCCTTGT
TCATCCTGACCGCCGTCGATGCCGGTACGCGCGTCGCACGTTTTATGATTCAAGACTTGG
GCAGCATCTTCTACAAACCTTTCGGCAACACCGACTCCATCCCCGCCAACCTGATTGCGA
CCTTCTTCGCCGTGGCATTGTGGGGCTACTTCCTCTACACGGGCGTGACCGACCCGTTGG
GCGGCATCAACTCGCTCTGGCCTTTGTTCGGCATCGCCAACCAAATGCTGGCAGGCGTAG
CCTTGATTATGTGCGCCGTGGTGCTGATTAAGATGAAACGCGACCGTTATGTCTGGGTGG
TACTCGTTCCCGCCGTCGGCGTACTGTTCGTAACCTGCTACGCCGGCCTGCAAAAACTGT
TCCACAGCGACCCGCGCATCAGCTTCCTTGCCCACGCCGGCAAATACAGCGACGCATTGG
CTAAAAACGAAATCCTTGCGCCTGCCAAAGACATCGGCGAAATGGCGCAAATCATCTTCA
ACGACAAGATTAATGCCGGTCTGACCATCCTCTTCTTGTCGGTTGTCGTGATTGTCGCCG
CGTACGGTTTGCGTACCGCCCTCAAAGCACGCAAAGTCGGCTGGCCGACCGCCAAAGAAA
TCCCGGCGGTGTACCGCGACGGCAAACAGCCGGAGGCACAAAGTGAAGCATAAGCTCGCG
TCTTGGTGGAAAACCATCAAGCTGACGGCAAACTTGATGGCAGGCGTGCCCGATTATGAA
AACTACGTTGCACAGCAGCGCAAACATAATCCCAACGCCCCCGTGATGACCAAGCTGCAG
TTTCAAGACTATTGCCGCAAACGCCGCTGCGGCGCAAACGGCGGACGCTGCTGTTAAGCC
TGCTTGAAACAAATTCCGTCTGAACGCCGCTTCAGACGGAATTTTTATAATATAGTGGAT
TAACAAAAATCAGGACAAGGCGACGAAGCCGCAGACAGTACAAATAGTACGAAACCGACT
CACTTGGTGCTTCAGCACCTTAGAGAATCGTTCTCTTTGAGCTAAGGCGAGACAACGCCG
TACTGGTTTTTGTTAATCCGCTATACCACGATGAATCCTTCGCAATATCTGTTTATCGAC
CTCAATTTTGACAAAATACCGGATACGCGCCTTTGTTGCTTTTCCATCTTCCAACCAACT
GTAAATCTCAAACAGCCGGTACACGCCATGCTTCAGTTTCTTTTCCTGTCGGCGGATTGT
TTCGACAAAGAATTGAAAATCCATTTCATGCACCTTAAAATTTAATCTGCATTCAAACCT
TTTCACTTTGGAAGCACCATTTATCGGATGTCCCTTCGCAATAAACAAATTTTCCCGATA
CCGCCGCCCATTTCAACCCAAACCCAAAAGCTATGAAAAACCTCATCGCCTTCAACAAAC
CCTATGGCGTTATCTGCCAATTTTCACCGCACGAAAAACACAAAAGCCTCAAAGACTTTA
TCAATCTTCCCGGCTTCTACCCCGCCGGACGGCTCGACACCGACAGCGAGGGGCTGCTGC
TGCTGACCGACGACGGCAGGCTTCAGGCACAAATTACCGACCCCAAATTCAAACACCCTA
AAACCTACTGGGCGCAACTGGAGGGCGTACCCGACGAAAGCCGATTGGAAAGCCTAAGAA
AAGGGATAGACTTAGGCGGTTTCGTTACCCGTCCGGCAAGCATCCGCATCTTGAAACACG
GAGAAGCAGATTCGTTATGGGAGCGCATCCCGCCGATACGCGTCCGCAAAACCGTTCCCG
ATTTTTGGATTGAAATTACCATTTCTGAGGGCAAAAACCGCCAAGTCAGGCGAATGACCG
CCAAGGCGGGCTATCCCTGCCTGCGTCTGATCAGAGTGGCAAGCGGCAGGCTGAAACTGT
TTGATTTGGATTTAAAACCCGGGGAATGGGCATACGCCCCGTTTAAACCATAATCACGTT
TATCTCATCATTTCCACAAAAGTGGGAATCCGGAATTTTATAGTGGATTAACAAAAATCA
GGACAAGGCGACGAAGCCGCAGACAGTACAGATAGTACGGAACCGATTCACTTGGTGCTT
CAGCACCTTAGAGAATCGTTCTCTTTGAGCTAAGGCGAGGCAACGCCGTACTGGTTTTTG
TTAATCCGCTATATTCCGCCATCTCTAAGATTTACAGCGATACACGGGTGATTTAAGGAA
TGCCCGAACCGTCATTCCCGCCACTTTTCGTCATTCCCGCGCAGGCGGGAATCTAGAATC
TCGGACTTTCAGATAATCTTTGAATATTGCTGTTGTTCTAAGGTCTAGATTCCCGCCTGC
GCGGGAATGACGAATCCATCCGCACGGAAACCTGCACCACGTCATTCCTACGAACCTACA
TCCCGTCATTCCCACGAAAGTGGGAATCCAGAACGTAAAATCTGAAGAAACCGTTTTATC
CGATAAGTTTCCGTACCGAACAGACTAGATTCCCGCCTGCGCGGGAATGACGATTCATAA
GTTTCCCGAAATTCCAACATAACCGAAACTTGACAGTAACCGTAGCAACTGAACCGTCAT
TCCCACGAAAGTGGGAATCTAGAAATGAAAAGCAACAGGCATTTATCGGAAATAACTGAA

## Appendix A

ACCGAACCGACTAGATTCCCGCCTGCGCGGGAATGACGGCTGCAGATGCCCGACGGTCTT
TATAGCGGATTAATAAAAATCAGGACAAGGCGGCGAGCCACAGACAGTACAAACAGTACG
GAACCGATTCACTTGGTGCTTCAGCACCTTAGAGAATCGTTCTCTTTGAGCTAAAGCGAG
ACAACGCTGTACTGGTTTTTGTTAATCCACTATAAATATCCAATTGAAATCTTCAGACGG
TATATCAAATTTACACTTTTTTAATGTTTATGCCGCCTGAAAAAAATGCTAGTATATTTC
CTAATTGTCTGACTGTTTATTGTTGAGGAAAATATGAGATCTTCTTTCCGGTTGAAGCCG
ATTTGTTTTTACCTTATGGGTGTTACGCTATATCATTATAGTTATGCCGAAGATGCAGGG
CGCGCGGGCAGCGAGGCGCAGATACAGGTTTTGGAAGATGTGCACGTCAAGGCGAAGCGC
GTACCGAAAGACAAAAAAGTGTTTACCGATGCGCGTGCCGTATCGACCCGTCAGGATATA
TTCAAATCCAGCGAAAACCTCGACAACATCGTACGCAGCATCCCCGGTGCGTTTACACAG
CAAGATAAAAGCTCGGGCATTGTGTCTTTGAATATTCGCGGCGACAGCGGGTTCGGGCGG
GTCAATACGATGGTGGACGGCATCACGCAGACCTTTTATTCGACTTCTACCGATGCGGGC
AGGGCAGGCGGTTCATCTCAATTCGGTGCATCTGTCGACAGCAATTTTATTGCCGGACTG
GATGTCGTCAAAGGCAGCTTCAGCGGCTCGGCAGGCATCAACAGCCTTGCCGGTTCGGCG
AATCTGCGGACTTTAGGCGTGGATGACGTCGTTCAGGGCAATAATACCTACGGCCTGCTG
CTAAAAGGTCTGACCGGCACCAATTCAACCAAAGGTAATGCGATGGCGGCGATAGGTGCG
CGCAAATGGCTGGAAAGCGGAGCATCTGTCGGTGTGCTTTACGGGCACAGCAGGCGCAGC
GTGGCGCAAAATTACCGCGTGGGCGGCGGCGGGCAGCACATCGGAAATTTTGGCGCGGAA
TATTTGGAACGGCGCAAGCAGCGATATTTTGTACAAGAGGGTGCTTTGAAATTCAATTCC
GACAGCGGAAAATGGGAGCGGGATTTACAAAGGCAACAGTGGAAATACAAGCCGTATAAA
AATTACAACAACCAAGAACTACAAAAATACATCGAAGAGCATGACAAAAGCTGGCGGGAA
AACCTGGCACCGCAATACGACATTACCCCCATCGATCCGTCCAGCCTGAAGCAGCAGTCG
GCAGGCAATCTGTTTAAATTGGAATACGACGGCGTATTCAATAAATACACGGCGCAATTT
CGCGATTTAAACACCAAAATCGGCAGCCGCAAAATCATCAACCGCAATTATCAGTTCAAT
TACGGTTTGTCTTTGAACCCGTATACCAACCTCAATCTGACCGCAGCCTACAATTCGGGC
AGGCAGAAATATCCGAAAGGGTCGAAGTTTACAGGCTGGGGGCTTTTAAAGGATTTTGAA
ACCTACAACAACGCGAAAATCCTCGACCTCAACAACACCGCCACCTTCCGGCTGCCCCGC
GAAACCGAGTTGCAAACCACTTTGGGCTTCAATTATTTCCACAACGAATACGGCAAAAAC
CGCTTTCCTGAAGAATTGGGGCTGTTTTTCGACGGTCCTGATCAGGACAACGGGCTTTAT
TCCTATTTGGGGCGGTTTAAGGGCGATAAAGGGCTGCTGCCCCAAAAATCAACCATTGTC
CAACCGGCCGGCAGCCAATATTTCAACACGTTCTACTTCGATGCCGCGCTCAAAAAAGAC
ATTTACCGCTTAAACTACAGCACCAATACCGTCGGCTACCGTTTCGGCGGCGAATATACG
GGCTATTACGGCTCGGATGACGAATTTAAGCGGGCATTCGGAGAAAACTCGCCGACATAC
AAGAAACATTGCAACCGGAGCTGCGGGATTTATGAACCCGTATTGAAAAAATACGGCAAA
AAGCGCGCCAACAACCATTCGGTCAGCATTAGTGCGGACTTCGGCGATTATTTCATGCCG
TTCGCCAGCTATTCGCGCACACACCGTATGCCCAACATCCAAGAAATGTATTTTTCCCAA
ATCGGCGACTCCGGCGTTCACACCGCCTTAAAACCAGAGCGCGCAAACACTTGGCAATTT
GGCTTCAATACCTATAAAAAAGGATTGTTAAAACAAGATGATACATTAGGATTAAAACTG
GTCGGCTACCGCAGCCGCATCGACAACTACATCCACAACGTTTACGGAAATGGTGGGAT
TTGAACGGGGATATTCCGAGCTGGGTCAGCAGCACCGGGCTTGCCTACACCATCCAACAT
CGCAATTTCAAAGACAAAGTGCACAAACACGGTTTTGAGTTGGAGCTGAATTACGATTAT
GGGCGTTTTTTCACCAACCTTTCTTACGCCTATCAAAAAAGCACGCAACCGACCAACTTC
AGCGATGCGAGCGAATCGCCCAACAATGCGTCCAAAGAAGACCAACTCAAACAAGGTTAT
GGGTTGAGCAGGGTTTCCGCCCTGCCGCGAGATTACGGACGTTTGGAAGTCGGTACGCGC
TGGTTGGGCAACAAACTGACTTTGGGCGGCGCGATGCGCTATTTCGGCAAGAGCATCCGC
GCGACGGCTGAAGAACGCTATATCGACGGCACCAACGGGGGAAATACCAGCAATTTCCGG
CAACTGGGCAAGCGTTCCATCAAACAAACCGAAACTCTTGCCCGCCAGCCTTTGATTTTT
GATTTTTACGCCGCTTACGAGCCGAAGAAAAACCTTATTTTCCGCGCCGAAGTCAAAAAT
CTGTTCGACAGGCGTTATATCGATCCGCTCGATGCGGGCAATGATGCGGCAACGCAGCGT
TATTACAGCTCGTTCGACCCGAAAGACAAGGACGAAGACGTAACGTGTAATGCTGATAAA
ACGTTGTGCAACGGCAAATACGGCGGCACAAGCAAAAGCGTATTGACCAATTTTGCACGC
GGACGCACCTTTTTGATGACGATGAGCTACAAGTTTTAAAGGCAGCCCGCATTTTGTAGA
AAACCGCAATGCCGTCTGAAAGCCCTTCAGACGGCATTTGTTTCCCCAAACGCATCATCC
TGCCGCAAGCCTATGCCAATCCGTTTTATCGCATCGGCAACTCAAAGAAAAATCCATTTC
ATTCCCACGCAGGGAAGCCGGTTTTTGATTTCGGTTATTTTTGGTTGTTTCGGGTAATTT
ATGAGTCGTCATTCCCGCAAAAGCGGGAATCAGTTTTTTTAAGTTTCAGCCATTTCCGAT
AAATTCCTGTGGCTTTAGCTTTCCGGATTCCCACTTTCGTGAGAATGACGTGGTGCAGGT
TTCCGTACGGATGGATTCGTCATTCCCGCGCAGGCGGGAATCTAGACCGTTCGGTTTCGG
TTTTTTTGGTTAGTGCCGCAACATTAAATTTCTAGATTCCCACTTTCGTGGGAATGACGG
CGGAGCGGTTTCTGCTTTTTCCAATAAATGCCCCCAACCTAAAATCCGTCATTCCCGCGC
AGGCGGGAATCTAGACATTCAATGCTAAGGCAATTTATCGGAAATGACTGAAACTCAAAA
AACTAGATTCCCACTTTCGTGGGAATGACGTGGTGCAGGTTTCCGTATGGATGGATTCGT
CATTCCCGCGCAGGCGGGAATCTAGTCCGTTCGGTTTCGGTTTTTTTGGCTAATGCCGCA
ACATTAAATTTCTAGATTCCCACTTTCGTGGGAATGACGGCGGAGCGGTTGCTGTTTTTC
CCAATAAATGCCCCCCAACCTAAAATCCGTCATTCCCGCGCAGGCGGGAATCTAGTCCGT
TCGGTTTCGGTTTTTTTGGCTAGTGCCGCAACATTAAATTTCTAGATTCCCACTTTCGTG
GGAATGACGGCGGAGCGGTTTCTGCTTTTCCCAATAAATGCCCCCAACCTAAAATCCGTC
ATTCCCGCGCAGGCGGGAATTTAGACATTCAACGCTAAGGCAATTTATCGGAAATGACTG
AAACTCAAAAAACTGGATTCCCTCTTTCGTGGGAATGACGTAGTGCAGGTTTCCGTACGG
ATGGATTCGTCATTCCCGCGCAGGCGGGAATCTAGACATTCAATGCTAAGGCAATTTATC
GGAAATGACTGAAACTCAAAAAACTGGATTCCCGCTTTCGTGGGAATGACGCGATTAGAG
TTTCAAAATTTATTCTAAATAGCTGAAACTCAACGCACTGGATTCCCGCCTGAGCGGGAA
TGACGAAGTGGAAGTTACCCGAAACTTAAAACAAGCGAAACCGAACGAACTGGATTCCCA
CTTTCGTGGGAATGACGGAATGTAGGTTCGTGGGAATGACGGGATGCAGGTTTCCGATGG
ATGGATTCGTCATTCCCGCGCAGGCGGGAATCTAGACATTCAACGCTAAGGCAATTTATC

## Appendix A

GGAAATGACTGAAACTCAAAAAACTGGATTCCCACTTTTGTGGGAATGACGCGATTAGAG
TTTCAAAATTTATTCTAAATAGCTGAAACTCAACGCACTGGATTCCCGCCTGAGCGGGAA
TGACGAATTTCAGGTTGCTGTTTTTGGTTTTCTGTTTTTGTGAAAATAATGGGATTTTAG
CTTGTGGGTATTTACCGGAAAAAACAGAAACCGCTCCGCCGTCATTCCCGCGCAGGCGGG
AATCTAGTCCGTTCGGTTTCGGTTTTTTTGGCTAGTGCCGCAACATTAAATTTCTAGATT
CCCACTTTCGTGGGAATGACGGGATGTATAGTGGATTAACAAAAACCAGTACGGCGTTGC
CTCGCCTTAGCTCAAAGAGAACGATTGTCTAAGGTGCTGAAGCACCAAGTGAATCGGTTC
CGTACTATTTGTACTGTCTGCGGCTTCGTCGCCTTGTCCTGATTTTTGTTAATCCACTAT
AAATTTAATCCACTATATTTTTTGTTCCAAAGTCAAAATATGCCGTCCGAACATTCGGGC
GGCAGACAAAACGGCACTGCCCGATAAAGGCAGTGCCGTTGTCCGTTTCAAACCGTGAAA
CATCAGCCCAAATTAAAGGCTTTATGCAATACCCTGGTTGCCAGTTCCATGTATTTTTCA
TCAATCAATACGGAAACTTTGATTTCGGAGGTGGAAATCATTTGGATGTTGATACCCTCT
TCGGCGAGCGTGCGGAAGATTTTGGCGGCTACACCGACGTGCGAACGCATACCCAAACCG
ACTGCGGAGACTTTGCATACGGTGTCGTCGCCATCAATAGAAGCCGCGCCGATACTGTCT
TGGCGTTCCGACAGGATTTCCAAAGTCTGCTTGTAATCGCCGCGCGGTACGGTAAAGGAA
AAATCGGTTGTGCCTTCGCTGCCGACATTTTGGATAATCATATCGACTTCGATGTTGGCA
TCGGCAACCGCGCCTAAAATCTGATAGGCGACGCCAGGTTTGTCGGGTACGCCGCGCACG
TTGATGCGGGCTTGGTTTTTATCGAATGCGATACCGGTTACGGCAGCTCTTTCCATGTTG
TCGTCCTCTTCAAAGGTAATTAAGGTGCCATTGCCGCCGTCTTGCAGGCTGCTCAGTACG
CGCAGGCGCACTTTGTATTTTCCGGCGAATTCTACTGAACGGATTTGCAAAACTTTCGAA
CCGAGGCTTGCCAGTTCGATCATTTCTTCAAATGTAACCGTATCCATGCGGCGCGCTTCG
GGTACGACGCGGGGGTCGGTTGTGTAAACGCCGTCTACGTCGGTATAGATTTGGCACTCG
TCGGCTTTGAGCGCGGCGGCAAGCGCGACGGCGGAAGTGTCGGAACCGCCGCGTCCGAGC
GTGGAAATATCGCCTTCACTGCTGATGCCTTGGAAGCCGGCAACGATGACGACTTTGCCG
GCGGTAAGGTCGGCACGCATTTTTTCGTCATCAATGCTTTCGATGCGGGCTTTGGTGTGG
GCGGTATCGGTTTTGAGGGCGACCTGCCAGCCTGTGTAGCTTTTGGCATCCACGCCGATG
TCTTTCAATGCCATCGCCAAAAGGCCGATGGTTACTTGTTCGCCGGTAGCTAAGACGACG
TCCAGCTCGCGCGGATCGGGATGCTCTTGCATTTCGTGCGCCAGTGCGACCAGTCGGTTG
GTTTCGCCGCTCATGGCGGATACGACGACTACGATGTCGTGTCCTTCGGCGCGGGCTTTG
GCGACACGTTTGGCTACGTTTTTGATGCGTTCGGGCGAGCCTACTGATGTGCCGCCGTAT
TTATGTACGATTAACGCCATGTTTCGTGCTTTCTTGTGGGGGTTGTCGGGCAGCTTGGTT
TGCTGGAAAAAGGGTTATTATTACTATTTTTTACATGGAATTCAAGAACGGACTGCGCTT
TCCCGCCTGCCGTTTGACAGCGGTCAGCGAAAAACCTGTTCTTTCAGATTGTTGACAAAA
TGCCGTCTGAACGGTTTTCAGACGGCATCCGGACGACAATCAGGCGGCGGACAACGCATT
TTGCTGGTGTTGCAGCAGTTCGCCTATGCCTTTTTGCGCCAGTGCAACCAGTTTGCCCAA
TTCGTCCAAACTGAACGGCGCGTCTTCCGCCGTCCCCTGTATTTCGATGATTTTTCCCGA
TGCGGTCATGACGATATTCACATCACTGTCGCAACCGGAGTCTTCGGGATAATCCAAATC
CAAAAGCGGCACGCCGTTCACTACGCCTACTGACACAGCGGCAACGGCTTCGCGGATGGG
GTTTTCACTCAAAATGCCGTCTGAAACCAGTTTGCCGACGGCGATTTGCAGCGCGACAAA
CGCACCGGTAATCGAAGCCGTGCGCGTACCGCCGTCTGCCTGAATCACATCGCAGTCAAT
CAAGATTTGTCGTTCACCGAGTTTTTCCATATCCACGACCGCGCGCAGGGAACGCCCGAT
CAAACGTTGGATTTCTTGTGTGCGCCCGGACTGTTTGCCCGCCGAAGCTTCGCGGAGCAT
CCGGGAAGCAGTTGAGGCAGGCAGCATCCCGTATTCCGCCGTTACCCAGCCTTGGTTTTT
ACCGCGCAGAAACGGCGGGACGTTTTCATCTATGGAAGCGGTACAAATCACTTTGGTATT
GCCGCATTCAATAAGGCACGAACCGTCCGTATGCGGCAGGAAATGAGGGGTGATTTTGAT
ATCGCGCAGGCTGTCGGCGGCGCGCGAGATGCGGATGTAATCAGGCATACTGCCCTCCCG
TTAAAAACAGATAAATTAAAAAGCCTTAAATATGAAAAATCACATTTAAGGCCTTCAAAC
TGAAAATTTCTACGCCTCTTCGGCTTTGCTGCGGATAATCAAAAGCGGCAGGTGGCTTTG
GCGCATTACCGTTTCGGCAAAACTGCCCATTAAAAGGTGCATCAGCCCGGTACGTCCGTG
~~CGTACGGAAGAGCAGCAGGTGGGCACCGTTTTCATCGGCATAATCAACCAAATCCTGCGC~~
CATTTCACGCGCACCCTTATTGGCAACCAGCAGGTGTTTGACGGTATTTTCCACACCCAG
TTCCTGGGCGGTGCGCTCGGCGGCATCCAAAACTTCGTTGCCTTGCGCGACGGCGGCGGC
TTCGTAGCTTTCGTGTTGCAAAAATTCGGGGGCGAGTGCCATATATTCGGCAGGATTGGC
AACGTGCACCAAAGTCAGGCGCGCACCGTTGACCCCGGCAAGCTCGGCGGCATGTTTCAG
GGCATTGATGGACGTTTCACTGCCGTCAACGGCAACAACCAAATGTTTGTACATATCGTA
TTCTCCTTTTGCACCGCCTCGCGGTGCCCTCTTGTCGGATGGGCGCAGGGACAGTTTGCG
CTGTTTCATTATAGACCCGCCGTCGGGCTTTATACAACAGCCGAACAGCCCGACCGCTTT
CCAGTATAATATGCCGCTTCCGTGCAGTCAGGCATTTTTTGCCGGCTTTCGTTCACTTTT
TGATTTGACGCAATCTTGCAGGATTCGACCATGTCCGACAACGCTTTGACCTCTTCGCGA
CGCTTCGGCGGCATCGCCAGACTCTACGGAGACTCTGCCTTGGCGCACTTTTCACAGGCA
CACGTCTGCGTAGTCGGCGTGGGCGGTGTCGGCTCGTGGGCGGTCGAGGCTTTGGCGCGG
ACGGGCATCGGACGTTTGACTTTGATTGATTTGGACAACGTTGCCGAATCGAATGTCAAC
CGCCAGCTGCACGCCCTGACCGGCGACTTCGGCAAAGCAAAAGTTACCGCCTTGCGCGAA
CGCATTACACAAATTAATCCGCAATGCGAAGTGTTTGAAATTGAAGATTTCGTTACCGAA
GACAATTTGCCGGAATACTTCGGAAAAGGTTTTGATTTCGTCATCGACGCGATCGACCAA
GTGCGCGTCAAAGCAGCAATGGCGGCTTATTTTGTGGAACGCAAACAACCGTTTGTCCTC
AGCGGCGGCGCGGGCGGACAAAAAAATCCGGCGTTAATCCAAACCGCCGATTTGAGCCGC
GTAACCCACGACCCGCTGCTTGCCAACCTGCGCTACACCTTGCGGAAACGCTACGGATTC
AGCCGCGATACGAAAGCAAATATGCGCGTGCCTTGCGTGTATTCGACCGAAAATATCGTG
CCGCCGCAGTCTAGGGAGGCTTGTTCGGCAGATGCCGCTCCGCAAGGCTTGTCGTGCGCC
GGCTACGGTGCAAGCATGCTCGTTACCGCTTCGTTCGGGCTATATTGCGCACAGGCGGCG
GTGGAACACATCGCAGACAAAAAATAAGCAATGCCGTCTGAAACAGGATTCAGACGGCAT
TTGAACAAACTATGGTTATGATTTAAGACAACAAAGGATACGGATAAAAAATAACATAAA
ATATATGATTCCTAATAATATACCAAGTATCGGAGAGCTATTTAATGGAATTCGTTAATA
ATTTAGTTATTTTTTCATTTTTATTACTAATGCTTATTCCGATATTTTTTGTAGTATATG

## Appendix A

GTATATACCATAAGATACGTTATCGCAAAATATGTATCCTAAGAACAAGTTTTATATTAT
TAGTGGTAATACTTTGCAGTATGTATTACATATATTGCCGTTATCTTGACCAACAAAAAG
TAGCTTATTATTGCATAGATGAACAATGTATTTCTATTGTTCATCTATACAAAGATTATG
GTATAAACTCTCCCACATATGCGAGAATTTACGCAGGAAAAATATTGTTTAGATTTCAAG
TAAGAGCTAAAAATTACGCTGAATTACTTATGGAAGATGATATATCAATTAGTAAAAAAA
TTTTGGGGAATAAATTTATCATTTATGGGTCGCTACCTGTAATATACGGTAATGTAGATA
ATATTGAAGTAAAAGAAGCTACTGGTTATATAGATAGATCCAGTACTGATTATATTGTCT
CAAGAAACTTAAAATTCAGACATTTATATTAATTAAGAGGTTTTAGCAAGAGTGCCGTCA
AAATATAGGGCGCATCATCGAATTCGCGAAAGACAAACGCTACGATGAACGTTTCAAGGA
TTTGAAAAAAGAATCCATAGGCTATCTGAACCGGCATCCCGGTTTGGTGTCCGACTACCT
GAAGGCGGCAATCAAGCTGTCGGTTCAGAAAAACCAACATCAGCACGCCTAAAACCGTAT
TCACAACCTGCTCCTTTTCAAAACATTTGCATTTAAAAGCCGTTATAATGCCGTCTGAAC
ATCTGCCCGACCACATTATACGTGAATGTCGGCAGATTGTTTTCTTTTGTAAACTTATAT
TAAAATCCACTTACCGATTCACGCCATGCCGCCCATCCCTGCCCCATCTGCACCATCCGA
GCACACTGTCGCATGGGTATTCGGCCAACCCGTTACCGATTTGCCCCAGGATTTGTTTAT
TCCGCCCGATGCATTGAAAGTCGTATTGGGCAGCTTCCAAGGCCCTTTGGATCTACTGCT
GTATCTGATCCGCAAACAGAATATCGACGTACTGGATATTCCGATGGTGAAGATTACCGA
GCAGTATCTGCACTACATCGCCCAAATAGAAACCTATCAGTTTGATTTGGCGGCGGAATA
TCTTTTGATGGCAGCAATGCTGATTGAAATCAAATCGCGCCTGCTGCTGCCGCGTACCGA
AACCGTCGAAGACGAAGAAGCCGACCCGCGTGCCGAGTTGGTGCGCCGCCTGCTGGCTTA
CGAACAGATGAAGCTGGCGGCGCAGGGTTTGGACGCGCTGCCCCGAGCCGGACGGGATTT
CGCGTGGGCTTACCTGCCGCTGGAAATTGCCGTCGAAGCCAAGCTGCCCGAAGTCTATAT
TACCGACTTGACGCAAGCGTGGCTGGGTATTTTGTCTCGGGCAAAACACACGCGCAGCCA
CGAAGTAATCAAAGAAACCATCTCCGTGCGCGCGCAAATGACGGCAATCCTGCGCCGTTT
GAACGGACACGGAATATGCAGGTTTCACGACCTGTTCAATCCCAAACAGGGCGCGGCTTA
CGTGGTCGTCAACTTCATCGCACTGTTGGAGCTTGCCAAAGAAGGATTGGTCAGAATCGT
GCAGGAAGACGGTTTCGGAGAAATCCGAATCAGCCTCAATCATGAGGGGGCGCATTCAGA
CGGCATTTCCGGCACACGAGGCGGGCGCGATGTGTTCTAATACGCCCCAAGCCGCCACCA
AAAATCGGGAGACACGCCATATGACCGGCATCATACATTCGCTGCTTGACACCGACCTCT
ACAAATTCACTATGCTGCAAGTGGTTCTGCACCAGTTTCCGCAGACGCACAGCCTTTACG
AATTCCGCTGCCGCAACGCCTCGACCGTCTATCCGCTTGCCGACATCAGGGAAGACTTGG
AAGCCGAACTCGACGCGCTCTGCCAACTACGCTTCACCCACGACGAACTCGGCTATCTGC
GCTCCCTGCGTTTCATTAAAAGCGACTTTGTCGATTATCTCGAACTCTTCCAGCTCCAAC
GCCGCTTTGTCGAAATCGGCACAGACGATAAAGACCGTCTGAACATCCGCATCGAAGGTC
CGATGATACAGGCGATGTTTTTTGAAATCTTCATCCTCGCCATTGTCAACGAACTTTACT
TCCGCCGCCTGGAAACCCCTGCAGTCATAGAAGAAGGCGAACGCCGGCTTCAAGCCAAAG
CCGCGCGCCTCAAAGAAATCGCCGCCGCACAAAACCCCGACGAACCGCCCTTCCTGATTT
CCGACTTCGGCACGCGCCGCCGCTACAAGCTCGCGTGGCAGGAACACGTCATCCGCACCC
TGCTTGAAGCCGCCCCCGGCATCGTACGCGGCACCAGCAATGTCTTTCTCGCCAAAAAAC
TCGGCATCACCCCCATCGGCACCATGGCGCACGAGTTCCTGCAGGCATTCCAGGCCCTCG
ACGTACGCCTGCGGAATTTCCAAAAGGCCGCGCTCGAAAGCTGGGTGCACGAATACCGGG
GCGATTTGGGCGTTGCCCTGACCGACGTGGTCGGTATGGATGCCTTCCTGCGCGATTTCG
ACCTCTATTTCGCCAAACTTTTCGACGGGCTGCGCCACGACAGCGGCGACCCTTACGTTT
GGGGCGACAAAGCCTACGCCCACTATCAAAAGCTCAAAATCGACAGCCGCACCAAAATGC
TGACCTTCTCCGACGGGCTGGACATCGAACGCTCTTGGGCATTGCACCAATATTTCAAAG
ACCGCTTCAAAACCGGCTTCGGCATCGGCACCAACCTCACCAACGATATGGGGCATACGC
CCTTGAATATCGTCTTGAAACTGGTCGAATGCAACGGGCAGTCCGTCGCCAAGCTGTCCG
ACTCTCCGGGCAAAACCATGACCAACAACAGCACCTTCCTCGCCTACCTGCGCCAAGTGT
TCGACGTACCCGAACCCGAAACGCCGTAAACCGGCAGAAAAAGCGCACAATTCCTGTTTC
TGCCGCATAAAATCTTTTAAAATACCGCCTGATTTGAATTTAACCGAAAGACCGAACTTC
ATGAACCTACATCAAACCGTCGAACACGAAGCCGCCGCCGCCTTTGCCGCCGCAGGCATC
GCCGACAGCCCTATTGTTTTGCAGCCGACCAAAAACGCCGAACACGGCGATTTCCAAATC
AACGGCGTGATGGGTGCGGCGAAAAAAGCCAAACAAAACCCGCGCGAGTTGGCGCAAAAG
GTCGCCGAAGCATTGGCGGACAACGCCGTGATTGAAAGCGCGGAAGTCGCCGGTCCGGGC
TTCATCAACCTGCGCCTGCGCCCCGAATTTCTCGCGCAAAACATTCAGACGGCCTTGAAC
GACGCTCGTTTCGGCGTGGCAAAAACCGACAAACCGCAAACCGTCGTTATCGACTATTCT
TCGCCCAATCTGGCGAAGGAAATGCACGTCGGCCACCTGCGTTCCAGCATCATCGGCGAC
AGCATTTCGCGCGTGTTGGCATTTATGGGCAATACCGTTATCCGTCAAAACCACGTCGGC
GACTGGGGTACGCAGTTCGGTATGTTGGTCGCTTATTTGGTCGAGCAGCAAAAAGACAAT
GCCGCGTTCGAGCTGGCGGATTTGGAGCAGTTTTACCGCGCCGCCAAAGTGCGCTTTGAC
GAAGACCCTGCCTTTGCCGACACCGCACGCGAATACGTTGTGAAGCTGCAAGGCGGCGAT
GAAACCGTTTTGGCATTGTGGAAACAGTTTGTCGATATTTCGCTCTCGCACGCCCAAGCC
GTTTACGACACGCTGGGCTTGAAGCTGCGTCCTGAAGACGTGGCAGGCGAATCGAAATAC
AACGACGATTTGCAGCCCGTGGTCGATGATTTGGTTCAAAAAGGTCTGGCGGTTGAGGAC
GACGGCGCGAAAGTCGTGTTCTTGGACGAATTTAAAAACAAAGAAGGCGAACCCGCCGCA
TTTATCGTGCAAAAACAAGGCGGCGGCTTCCTCTACGCCTCCACCGATTTGGCGTGCCTG
CGCTACCGCATAGGCCGTCTGAAAGCCGACCGCCTGCTGTACGTCGTCGACCACCGCCAA
GCCCTGCACTTCGAACAACTTTTCACCACTTCCCGCAAAGCAGGCTATCTGCCGGAAAAC
GTCGGCGCGGCATTTATCGGCTTCGGCACCATGATGGGCAAAGACGGCAAGCCGTTCAAA
ACGCGCAGCGGCGACACCGTGAAACTGGTCGATCTGCTGACCGAAGCCGTCGAGCGCGCC
ACCGCTTTGGTGAAAGAAAAAAATCCCGAATTGGGTGCGGACGAAGCCGCTAAAATCGGT
AAAACCGTCGGCATCGGCGCAGTCAAATACGCCGACTTGAGCAAAAACCGCACCAGCGAC
TATGTGTTCGACTGGGATGCCATGCTCTCGTTTGAAGGCAACACCGCCCCCTATCTGCAA
TACGCCTACACCCGCGTGCAAAGCGTGTTCCGCAAAGCAGGCGAATGGGATGCAAATGCG
CCAACCGTTTTGACCGAACCGCTGGAAAAACAGCTTGCCGCCGAGCTGCTGAAATTTGAA

## Appendix A

GACGTACTGCAAAGCGTGGCGGACACGGCGTATCCGCACTACCTCGCCGCCTACCTCTAT
CAAATTGCGACCCTGTTCAGCCGCTTCTACGAAGCCTGTCCGATACTCAAAGCCGAAGGC
GCAAGCCGCAACAGCCGCCTGCAACTGGCAAAACTCACCGGCGACACGCTGAAACAAGGC
TTGGATTTGCTGGGCATCGATGTGTTGGACGTAATGTAAAACCGCACCGCCCGATTGCGG
ACAACAGCCTCGCCATCCTTATCCGAATCTGAAAAAAGCGGCGCGATACACCGTATCCGC
CGCCCCTCCCAAAATGCGAAACAAACAAACGCCAAGCAAGCAAGCAAGCAAGCAAGCAAG
CAAGCAAGCAAGCAAGCAAAAAATTATAACCCCCTTCCTGCCGACGCACGCACTTTCCGC
GCGGCGCATTCCCCTTTTCCCGCCCCTCAAATCCGCCTTTTCTTCAGGCAGGGTTTCAGC
CCGCCTCTTTTCCCTGTTTTCCTTTCCCCGACACGCGTGCGCTCCCCCTGCCGCACTGTG
CTGCACTTTCGCGCCCGGACGGCATCGTTCCGCCATCCGGTTCTCTGTTTTACATACCCC
TGTTTCAGAAAGAAATGCAGATGTTTCAACACACAGGACGACACATAAAGCACCGCCCTA
TGTGTTGCCCTGATTTGGAAGGGGTTACGCCTCCCAAATAAAGTCTGATCCTGCCGCCCC
GAAGGACAGATGTCCGAGTGGCGAAGTTTCAACCGAAAAGGAAATACGATGAATATTCAC
ACCCTGCTCTCCAAACAATGGACGCTGCCGCCATTCCTGCCGAAACGGCTGCTGCTGTCC
CTGCTGATACTGCTTGCCCCCAATGCGGTGTTTTGGGTTTTGGCACTGCTGACCGCCACC
GCCCGCCCGATTGTCAATTTGGACTATCTTCCCGCCGCGCTGCTGATCGCCCTGCCTTGG
CGTTTCGTCAAAATTGCCGGCGTATTGGCGTTTTGGCTGGCGGTTTTGTTTGACGGGCTG
ATGATGGTGATCCAACTCTTCCCTTTTATGGATCTCATCGGCGCCATCAACCTCGTCCCC
TTCATCCTGACCGCCCCCGCCCCTTATCAGATAATGACCGGGCTGTTGCTGCTGTATATG
CTGGCGATGCCGTTTGTGTTGCAGAAAGCCGCCGCCAAAACCGACTTCCGGCACATTGCC
GTCTGCGCCGCCGTTGTGGCGGCAGCCGGCTATTTCACCGGCCATTTGAGTTACTACGAC
CGGGGTCGGATGGCCAATATCTTCGGCGCAAACAACTTCTACTACGCCAAAAGTCAGGCG
ATGCTCTACACCGTCAGCCAGAATGCCGACTTTATTACCGCCGGCCTGGTCGATCCCGTC
TTCCTCCCCTTGGGCAATCAACAGCGTGCCGCCACGCATCTGAACGAGCCGAAATCTCAA
AAAATCCTCTTTATCGTCGCCGAATCTTGGGGGCTGCCGGCCAATCCCGAACTTCAAAAC
GCCACTTTTGCCAAACTGCTGGCGCAAAAAGACCGTTTTTCGGTTTGGGAAAGCGGCAGT
TTTCCCTTCATCGGCGCGACGGTCGAAGGCGAAATGCGCGAACTGTGTGCCTACGGCGGT
TTGCGCGGGTTCGCACTGCGCCGCGCGCCCGACGAAAAATTTGCCCGCTGCCTCCCCAAC
CGTTTGAAACAAGAAGGTTACGCCACCTTTGCGATGCACGGCGCGGGCAGTTCGCTTTAC
GACCGCTTCAGCTGGTATCCGAGGGCGGGCTTTCAAGAAATCAAAACCGCCGAAAACCTG
ATCGGTAAAAAAACCTGCGCCATTTTCGGCGGCGTGTGCGACAGCGAGCTGTTCGGCGAA
GTGTCGGCATTTTTCAAAAAACACGACAAGGGACTGTTTTACTGGATGACGCTGACCAGC
CACGCCGACTATCCCGAATCCGACATTTTCAACCACAGGCTCAAATGCACCGAATATGGC
CTGCCCGCCGAAACCGACCTCTGCCGCAATTTCAGCCTGCACACCCAATTCTTCGACCAA
CTGGCGGATTTGATCCAACGCCCCGAAATGAAAGGCACGGAAGTCATCATCGTCGGCGAC
CATCCGCCGCCCGTCGGCAACCTCAATGAAACCTTCCGCTACCTCAAACAGGGGCACGTC
GCCTGGCTGAACTTCAAAATCAAATAACAACAATGCCGTCTGAACGCACCAACAGCCTTC
AGACGGCATTTTGCAGACAGACCGACCCTTCAAGCCCACTTTTTTCATCATCTCCGATAA
ATTGCTTTGTATAGTGGATTAACAAAAACCAGTACGGCGTTGCCTCGCCTTAGCTCAAAG
AGAACGATTCTCTAAGGTACTGAAGCACCAAGTGAATCGGTTCCGTACTATTTGTACTGT
CTGCGGCTTCGTCGCCTTGTCCTGATTTTTGTTAATCCGCCATAAAGACCGTCGGGCATC
TGCAGCCGTCATTCCCGCGCAGGCGGGAATCCAGAACGTGGAATCTAAAGAAACCGTTTT
ACCCGATAAGTTTCCGCACCGACAGACCTAGATTCCCGCCTGCGCGGGAATGACGGGATT
TTAGGTTTCTGATTTTGGTTTTCTGTCCTTGTGGGAATGACGGGATGTAGGTTCATAGGA
ATGACGTGGTGCAGGTTTCCGTATGGATGGATTCGTCGTTCCCGCGAAAGCGGGAATCCG
GAAACCCAAAGCCACGGGAATTTATCGGAAAAACCGAAACCGCTCCGCCGTCATTCCCGC
GCAGGCGGGAATCTAGGTCTGTCGGTGCGGAAACTTATCGGATAAAACGGTTTCTTCAGA
TTTTACGTTCTGGATTCCCACTTTCGTGGGAATGACGGGATGTAGGTTCGTAGGAATGAC
GTGGTGCAGGTTTCCGTATGGATGGGATTCCCTCTTGCGTGAGGCTGACAGATGCCGTCT
~~GAAAGACTTTCAGACGGCATAGCTTTTTCTCTTTGAATTTATAGTGGATTAACAAAAATC~~
AGGACAAGGCGGCGAGCCGCAGACAGTACAGATAGTACGGAACCGATTCACTCGGTGCTT
CAGCACCTTAGAGAATCGTTCTCTTTGAGCTAAGGCGAGGCAACGCCGTACTGGTTTTTG
TTAATCCACGATAAATTTGCCACAAAAAAGCTGCCTCAAATGAATACCCGGGCAGCTTTT
TGTTGATATGACTCCAATCAGCGGTGTTGCGGATTGTAACGTTTTTCCAAACGCAGGAAT
ATCCAGCCTAAGAAAGTCGTCATCAACAGATAAATCAGGGCGACGGTGTAAAGCGGTTCT
TCATAAACCGAATACCGGCCCGTAATCGTATTCTGAACATACGCCAACTCCGCCACAGCA
ATGACCGACAGCAGCGAGCTGTCTTTCAAGAGCGTGATGAACTCGCTCGCCAAAGGCGGC
AGCATGCGGCGCAATGCCTGCGGCAGAATCACATAGCGCATCGCCTGCGGATAGGTCAGC
CCCAAAGAACGCGCCGCCTCCATCTGTCCTTTGTCTATAGACTGGATGCCCGCGCGGAAA
ATCTCACAGATATACGCCCCCGAGTTGGCGATCAGTGCCAAAGAACCGGCAATCAGCGGC
CCGTATCCGCGACGCAGCGCGATTGCCGCCTCGCCGCTGACCAAAATGCCGTCTGAAGGA
TGGACGAAAAACGGAAACCACACATACGCCCAAATCACAATCTGCACAAACAGCGGCGTA
CCCCGGAACAGCGTAACATACAGCAGCGAAACTTTACGCAACGCCCACGCCAGCACGCGC
ATCGGCGCACCGGCTTTTTCCAAGTGAATCAGGCGCGCCAACGCCAACAACAGACCCAAT
ACCGAACCGCCCGCCGTTGCCACGACCGTCAGCCCCAAGGTCGTCAGTGCGCCGTAAAGA
AACATCCAGCGGTATTCGTAAATAATGTCAAAACGAAAATCCATAAACCGTCCGTATCAA
AAACCGGCGGAACTGCCGCCGTTGCAAAATAATCCGCCATTTTACCGTAAAAACCGCCGC
CTGAACTTTTTTATCGCGGCAGACGGCGGTTGCGCGTCTCCGCAAAAATGCAGGGCGCGC
GGTTTTCAGACGGCATTTGCCGTTCAAAGCCGTGCGGTGTCTTTACCAAATGCCCAACCA
TTCGCCCACGGCATCCATCCAATCCTTATTGCCCCCGCCGCTCCTGCCTGCTCGGCGGTA
CGCCCACGGCGCTTGCGGATTTTTAGCTTTCCACAATCCTTTGCGTTCCCTTTCCGCCTG
AATTTGAGCGTCGGCATAATCGGCAAAATCCGCCTTATCCTGCTGTTCTTTAGCATAACT
TTTATAATGCCACGCCGCCCCGTCCTGCACCTGCATCAGGTTCAAATCGGTTTTGCCGAC
AGAAACCTGCGCCACTTCGCGCTGGTAGCGGTCGGTATCGAACACGCGCACGCTGACTTT
CCTGCCTTCCGCCGCCGCGCGCAGGTTGTCGCGCGAACGCGTGCCGTAAGCCTGTTTCAT

## Appendix A

CTCCGGCGCGTCGATATACGCCATCCGGATTTTGTGTTTCGCGCCGTCGCCGTCGATAAC
GTGAAGGGTGTCGCCGTCATAGACTTTGGACACCGTGCCTGTGTAGCGGTGGCCGGATTT
CGCCGATGCTCGGCGGCGGGCGGGCGCGTCGGAACCCGCGTCCCCTGCCGCGCCGAGTAC
GTCGAGTACGGCAACCGCCGTCCGCACCGCCTCGCTGCCGTACCCCGTATAACCCAACGC
ACCCAAAAGCGACAGGGCGACGGGAAGCCATTTCATGATTTTTTTAATCTGCATATTTTT
CAAATGCCGATGCCGTCTGAACATATCGGAATCGGATTTCAGACGGCATCTTAACGTCAG
GATTACCCTTGGCAGGGATAGATGACTTTCGCACCCTCTTCCGTCCCCAAAATCAACACA
TCGGCGGCATCGCGGGCGAATATGCCGTTTTCGAGCACGCCGGTGATTTTGTTGATTTCG
TCTTCCATCGTCAGCGGCTGATCGATATTCAAGCCGTGGACATCGACGATTTGGTTGCCG
TAAAACGTGGTGTAGCCGATACGCAGTTCGGGCTGTCCGCCCATAGCGAGCAGTTTGCGC
GAAACAAGAGAGCGCGCGCTTTCGACGACTTCCACAGGCAGAGGGAATTTGCCCAAACGT
GAAACATATTTGCTTTCATCCGCAATGCAGATGAATTTTTCGGACGCGCTGGCGACGATT
TTTTCGTTGAGGTGCGCGCCGCCACCGCCTTTAATCATTTGCAGGGCGTGGTTCACTTCA
TCCGCACCGTCGATATAGACCGCCAACCCCGATACTTCGTTCAAAGAAACGACGGGAATA
TCGTACTGGGCAAGCAGTTCGCCGGATTTTTTGGAAGTAGATACCGCGCCTTTGATTTTT
TTGCCGCTCTTACCCAAGGCTTCGATGAAAAAGTTGATGGTCGAGCCGGTACCGATGCCG
ATATATTCATTTTCGGGTACGAATTCGACTGCTTTTTCGGCGGCGATGCGCTTGAGTTCG
TCTTGTGTCGTCATATTTTTGTCCTTTGGGAAACCGTATCAACAAACAGCCGCCATCTTA
ACATTTTTTTGCACGTCCTGCCCGCCGCGTTCAAATGCGTACCAGCAATACCGCCGCCTG
CGCCTCTATGCCTTCCATCCGCCCGAGATAGCCGAGTTTTTCGTTGGTTTTGCCTTTGAT
GTTGACGCACGAAATGTCTATGCCCAAATCGGCGGCGATGTTGGCACGCATTTGCGGAAT
GTGCGGCGCGAGTTTGGGTTTCTGTGCAATCACGGTCGTATCGACATTGACCGCCTGCCA
ACCCTGCGCCTGAACGCTTTGATACGCCGCACGCAAAAGGACGCGGCTGTCCGCATCTTT
GAACTCTGCGGCGGTGTCGGGGAAATGGCTGCCGATATCGCCCAAACCTGCCGCACCGAG
CAGCGCGTCGGTAACGGCGTGCAGCAGCGCATCGGCATCGGAGTGTCCGAGCAGCCCTTT
TTCAAATGGGATTTCAACTCCGCCAAGTATCAGCTTTCTGCCTTCGGTCAGTTGGTGGAC
ATCGTAGCCCTGTCCGATACGGATGTTCGTCATCGTTTGTGTTCCTGATGTTTTGAATTG
AAGTTCAGACGGCATCGAGCAGCAGCCTGACGATGTATGCGTCCTGCGGCTGCGTCAGTT
TCAAATTGCGCACGTCGCCCTGTATCAGTAGCGGACGCACACCCAATTTTTCCACGGCGG
ACGCTTCATCGGTAATGCCGTCCAAGTTTTCCGCAGCCAATGCGCGGTGCAGCAGCCCGG
CGCGGAAAAGCTGCGGCGTTTGCGCCTGCCAAAGGCTCGTCCGCTCGACGGTTGCACTAA
TGTTCCCACCGTCCGCGCACTTGAGCGTATCGGCAATGGGAATTGCCAAAATCCCGCCTT
CGGCGGCGTTGCCCGCCTGTTCTATCAACCGCGTCAAAGCTTCAGACGGCAGGCAGCAAC
GCGCGGCATCGTGTACCAGAATATTGTCGGTTTCCGCCGCCAAACCGGTTTCCAACAGTT
TTGCCACACCGTTGCGGACGGTTTCGGCGCGGGTCTGTCCGCCGTTTTTCCACACCCGAA
CCTGTGGAAATGCCGTCTGAACCTTATCGGCAAACGTGTCTTCGGGCGAGACGACAACGA
CGGTCAAATCGACGGCCTCATGCCGTTCAAAAATCCCAATCGTATGTTCTAAAACGGTTT
TGCTTCCGATTTCGACATATTGCTTGGGTTTGTCCGCACCGAAACGCGCCCCGATGCCGG
CGGCGGGAATCAGCGCGATATTTTTGCGCTTCATGCGTCCGTCCCGCCGTTTTCAGACGG
CACGGCTTCCTTGCGCCAGATACAGGCTTCGCCCAAGCCGTCCAAATATTGCCCGTGCGC
CGCCAACTCGTTTTCGTCCGCCCTGATGACTTTCAGTTTGCCGCTGCGTTTGGTTTCGGT
ATGCACCACGGGTTTGGTTTCCATTTTTTCCTCTGCGGCCGCACCCATCAGGTCGAACTG
CCGCCGCGTCATAGCAAGATAGACTTCGCCCAAAAGTTCGCAGTCGATCAATGCGCCGTG
CAGGACGCGCTTGCTGCGGTCGACGGAAAAACGGTTGCACAAGGCATCCAGGCTGGCTTT
CTGCCCGGGGAACATTTCGCGCGCCATCGCCAGGGTATCGGTAACGGTACAGCCGAGTTC
CTCAACGGTCGGCAACCCCATCCGGCGGAACTCCATATTGAGGAAGCCCACGTCGAATTT
GGCATTGTGGATAATCAGTTCCGCACCGCGCAGGAAATCGGCAATCTGCCTGCCGACCTC
TGCAAACGGCGGCGCGTTTTTCCCTTCCAAAACCTGTATCGTCAAGCCGTGGACGCGTGC
CGCCTCTTCGGGCATATCGCGCTCGGGGTGGACATAGAGGTGCAGGTTTTTGTCGGTCAT
TTGGCGGTTGACCATTTCCAAACCGGCAAACTCGACCAAGCGGTCGCCGCCGTCGGCATA
CAGACCGGTGGTTTCGGTATCGAGGATGATTTGGCGTGTCGTCATATCGGTGTCTTTCTT
CTATCTTCGTAAATTGCTTATTTTTAAGCAATGTATTTTTCTGTTTTCATTTCAATGCA
CAAACCCACTTATTCACAGTGTGTTCACAACATTGGGCAGGCGGATTGTGTATTTTGGGG
ACAATTTTTTCAGACGGCATTCAAGGTTTTTTCCTGATTGCCGCCGCGCCTAAAAACCGC
CTTTCGCGCTTAATCAAAAATACCGACAACGGAATATTGCCCAAAGCGACAATCAGATAC
AACAAGGAAATGCTGTCAAACAAAAACAGCAACACCGCGCTCAAAACGGCAGCGGAAACC
ATAAAAATACCGTTAACGATATTGTTGGCGGCAACGGCGCGGGCGCGGAAAGTCTCGCTA
CTGGCGGTTTGCAGCCAGGTATAGAGCGGAACGGAGAAAAATCCGCCGAAAAAGCCGATC
AGCGTCATCACCGCCATCACGGGATATGCCCATCCTTGCGATAAAAACCAAAAAATGCCG
TTCAGCCCTTCAAAACGGTGTCCGTGCGTCAGCCACACCAAAACCAAGCCGCAAACCGTC
AAACCCAACGCACCAACCGTTACCCAAGCCAACATCAGGCGTTCCCTGCTGAACTTGGCA
CACAGTACCGAACCGGCGGCAATACCGATGGAAAACAGAGCAAGCATCAGGTTGAAAACA
TTGTCGTTGCCGCCCAGATGGATTTGGGTAAAGGTCGGCAGTTGCGTGGTATAAACCGCG
CCGACAAACCAAAACCACGAAATACCGATAATGGCGGTAAAAACGGGCTTGTGCCGCACC
GTTTCACGCAGCAGGGATTTTGTGCCACGGACAATATTCCACTCAATTTGTGTATCGGCA
GCCTTGGCGGGTACGGACGGCATAAACAGGCTGCCGACCGTGCCTCCGACGGCGACCAGC
AAAACCAGTATCCCGACAATATAAGGCGGTACACCTGCCACCGCCGTTCCCAAAATCTGA
CCGAACAGGATGGCGACAAACGTACCCGATTCAATCAGGCTGTTGCCCATCATCAACTCT
TTGTCGTCGAGATAATCGGGCAGGATGGCGTATTTCAGCGGCCCGAACAGCGTCGATTGC
GCGCCCATGCAAAACAGACACGCCAAAAGCAGCGGGGCAGACCGGATATAAAACCCGTAT
GCCGCCACCGCCATAATGATCATTTCCAGCACCTTGACCCAACGCGCCAAAACGGCCTTG
TCGAATTTGTTACCCAACTGCCCCGACAGCGAGGAAAACAGGAAATACGGCAAAATAAAC
AGCAACGCGCCCAAGTTCAACATCTGTCCGGCAGGCAGGAAGCCGTTTTGCCCCAAACCG
TAAAACCCAATCATCACAAACAGCGCGGTTTTGAACACATTGTCGTTGAACGCGCCGAGA
AACTGCGTAGCGAAAAGAGGTGCGAAACGGCGGCTTTTAACCAGTCCCAAACCGCCTTTT

## Appendix A

TTAGCGTACATCGTTTTCCCTCTCTTTTTCAATCAGTTTACTTGTCGAATCATCATCCAT
CAGGATGCGGTGCGCCGGCCCTTCCAAGTCGTCAAACTGCCCGTTTTTGCCCGACCACCA
AAAAAACCAGCCGATGACAAACGCCAAAATAATGCTGATGGGCACCAATATAAACATGCT
TTCCATCACATATTCCCTGTCAAATCGTTCAAAACAAAAGTCTGCCCCGACACGGTCAGA
TATTCGTTACGCAAAGTTCCGACGGGAGCTTCGTCAAAAAACAGCTCGATACGGTCTTTG
ACCACGCGCCAATATTGGGGGATTTCCGTCTGACCGAACGGCGACAGGACATGATTTTCC
ATTCCGCCTTCAAGTTTGACGGCAAAACGCCCGCTTTGCGGCCGTGCTTCCGATTCGTCG
TCGGCAAGCAGGATGAAAAAGCCTATATGCCGTCCCGATTGGTCATGAATACTGAAATAA
TGCATAAATTTCCCACCCGCCTTTTTTCAGACGACACCAACTAAAAACAGGGCGAATGTA
CCAGTTTGGACGGGAAGAATGCAAAGAAATTCTCCCTCCCCCAGCCGAAAACACCGGCAA
ACCGCATATCCCCCTTTTTTCCGTCAAAATGCCTGACTTCCGCCATTTTCACGCAAACGC
CCGATTAAGCCAAGCAATTGCAAAGATTTTTTGCTAGAATAGCCTGCTTCTTTTATCAAC
CTTTTCAGACGGCCCCACTACTTTCCCGCCCAGGAAGGCAAAACGGATTCGGCACGAATC
CGGTTAGTATCCGTGTCCGATTCCAATGCCGTCTGAAACTTTCCGGAGTAAGAAAATGTC
CCAAAAATTGATCTTGGTTTTGAACTGCGGCAGCTCGTCCCTCAAAGGCGCGGTCCTGGA
TAACGGCAGCGGCGAAGTCCTGCTCAGCTGCCTTGCCGAAAAACTCAACCTGCCCGATGC
CTACATCACATTCAAAGTAAACGGCGAAAAACACAAAGTCGATCTGTCCGCACATCCCGA
CCACACCGGCGCGGTCGAAGCCCTGATGGAAGAACTCAAAGCCCACGGCCTCGACAGCCG
CATCGGCGCCATCGGCCACCGCGTCGTCAGCGGCGGCGAACTGTACAGCGAATCCATCCT
CGTTGACGACGAAGTCATTGCCGGCATCGAAAAATGCATCCCGCTCGCCCCCCTGCACAA
CCCCGCCCACCTCTTGGGCCTGCGTGCCGCGCAAAGCATTTTCAAAGGCCTGCCCAACGT
CGTCGTATTCGATACCTCCTTCCACCAAACCATGCCCGAAGTCGCCTACAAATACGCCGT
TCCGCAGGAGTTGTATGAAAAATACGGCCTGCGCCGTTACGGCGCGCACGGTACCAGCTA
CCGCTTCGTCGCCGACGAAACCGCGCGCTTCCTCGGCAAAGACAAAAAAGACCTGCGTAT
GGTCATTGCCCACTTGGGCAACGGCGCGTCCATTACCGCCGTCGCCAACGGCGAATCGCG
CGACACCAGTATGGGCCTGACCCCGCTGGAAGGGCTGGTAATGGGTACGCGCAGCGGCGA
CATCGATCCTTCCGTATTCGGCTTCCTCGCCGAAAACGCCAATATGACCATCGCCCAAAT
CACTGAAATGCTGAACAAAAAATCCGGTCTGCTCGGCATTTCCGGCCTGTCCAACGACTG
CCGCACCATTGAAGAAGAAGCCGCCAAGGGGCATAAAGGCGCGAAATTGGCCTTGGATAT
GTTTATCTACCGCCTTGCCAAATACATCGGCAGTATGGCGGTTGCCGCAGGCGGTTTGGA
CGCACTGGTCTTTACCGGCGGCATCGGCGAAAACTCCGACATCATCCGCGAACGCGTGAT
CGGCTACTTGGGCTTCCTCGGTCTGAACATCGACCAAGAAGCCAACCTGAAAGCCCGCTT
CGGCAACGCCGGCGTGATTACCACTGCCGACAGCAAAGCCGTTGCCGTGGTCATTCCGAC
CAACGAAGAGCTGATGATTGCCCACGACACTGCCCGTTTGAGCGGTCTGTAAGGTTTTAT
CCGCACACGAACTGCCTCCGGAAATGGAGGCAGTTTTTTTATCCGGCTTTCCATGCTTAA
ACAGCACTGCCTCTTTTCAGACATTGACGGTTGCAGCCGCTTACCTGAACCTTATAGTGG
ATTAAATTTAAATCAGTACGGCGTTGCCTCGCCTTGCCGTACTATCTGTACTGTCTGCGG
CTTCGTCGCCTTGTCCTGATTTAAATTTAATCCACTATAATGATTAACTATTTTTTAATC
ATGTTATTATTTTCCATAAAATACATGACATTAAGATGTTTTTCCACAAAAGATACACAC
ACCGGCAAACACCGGCTGTGTTTATCTTTTCTTATGCCTATTTTTTAATCATCGTATTTT
TATCTTTTAATTTCAATACGCAAACTAACTTATACACACGGTTTTCACATCTTTAGACTG
CTTCCGTGTGTATAGTGGATATTGCCGTTTTCCTTTCTGACAAAAATGCCGTCTGAGAAC
TTCAGACGGCATTTGAAACATCGGAATCAGCGGTTTTGTTCATACCACTCGATAAACTTG
TCTGCTTTGACAAAACCCAGCAGCGGCTCGCTGCGGCTGCCGTCGGAGCGGACGACAAAC
ACGCCCGGCGGCCCGAACAGACCGTATTCTTTCAACAACGCCTGATGTTCGGGCGTGTTG
GCGGTTACGTCGATTTGGAAAAAGCGTTCCATATCGACTGCCTGATGCACTTCCGGCTGA
TTGAGCGTGTAAGCCGCCATTTCTTTGCAGGAAATGCACCAGTCGGCATAAAAATCCAAA
ACGACGGGTTTGTCGGGATGTTCTTTCAACGCCGTATCCATCGCTGCCTTCAGCGCGGCA
GTATCGGCAAACATTTTGCCGTGTTCCGAAGATTTGCCTGCTTCGGCTGGTGGATTGAGG
GTCAGGAAATGGTGCAGCGCGGTCGTTTTGCCGTTTGCGCCCTGCCAGCCGAACCACGCG
CCGCCTATCAGCAATATACCGCCCAATGCGAATGCCACAGCTTTCGGACGGCGTTTCTGC
CTGCGTCCGTTGACCAGCAGCATAAAGGCAGGAACCAGCATCAGCAGCGTGTACAGCGCG
ACGACGAGATAATAGGGCAAGTGCGGCGTGGCGAGGTAAACGGCGACGGCTAGCAGGATG
AAGCCGAATGCGTATTTGACGGCATTCATCCAATCGCCTGCCTTAGGCAGGATATGCCCG
CCGAACGTGCCGATGGCAATCAGCGGAACGCCGGTGCCCAACGCCAAAGTGTAAAGTGCC
AAACCGCCTAAAACCGCATCGCCCGTCTGACCGATGTAGCCCAAAGCAAATGCCAGCGGC
GGGGCGACGCACGGCCCGACAATCAGCGCGGACAATATGCCCATAATAAAGACGGAAACG
ATTTTACCGCCTGAAAGCCTGCTGCTTTGATTCTGAAAATACGACTGCACGGCGTTGGGA
AGCTGGATGTTGAACAGCCCGAACATAGACAGTGCCAAGACGACCATTAAAGCCGATGCC
GCCAATACCACCCAAGCCTGCTGCAACCATACGGTCAGCAGTGCGCCCGTCAGTCCGGCA
ACAATGCCGACCAGCGTATAAGTCAGAGCCAAACCCTGAACATAAACGACGGACAGCACA
AACGCCCGCGCCTTGCCCGCCTTTTTGTCGCCGACCACAATACTGGAAACAATCGGCAAC
AGGGGATACATACAGGCGGTAAAACTCAGGCCCAAACCAGCGAGAAAAAACGCCAAAAGA
TTGGCGTTGAGCGTATCCCAAGACAGCTTGAAACGGCTGTCGCCGCCCTCATCCCCCTTC
GGGGGCGGCAGCGCCCCGCTGCCGTTTTGAGAGGAAGGCTGCAAAAAGCGGTCTTTGGCG
GATGCCGGTTCGTCGGTTTGCGGATGGTAAGTGCCGTTGCCGAAAATATCAAACTCGGTA
TCCACGGGCGGATAGCACACGCCGGCTTCGGCACAGCCCTGATAGGTCAAAACCAATTTA
TACGGTTCGCCGACAGCCTTTGCATAAGGAAAGGCAACCTGCGCCTCGTGATGGTAAACC
GTCTGCCTGCCGAAAAACTCGTCTTCCTTCTCTTCGCCCTTGCTGAAAGAAGGCTGTCCC
AACAAATCCGCCGGATCGGTCTTGCCGACGATTTTCGCCTGATACATATAGTATCCGTCG
GCAATCCTGAAACGGACGTTCACACCGTCGTCGGCAACGGCAAGCTCCGGCACGAATGCC
TTTTCCGGCGGCAGCAGATCGTTCGCATCCAGCGCGAAAGCTCGTCCGCACAACATCAAA
AATACGGCGAACAGGCAAATCAGTTTTTCATAATCGAATCCGTTTCAGACAAATAATTT
GTCTGCATTATAAATGGTAAGGTTGACGGTGGGATTTAATTTATGTAAAACCCGCCATTA
TCCGAACCTATTTCCATAAACATCTTATCGAACCCGCCATGTACGATGTCAATACCCACG

## Appendix A

ATGTCCGCCGCTTTTTCGCCCGCGTGTGGCAGCAGCGGCTCAATCCGCTGCAACTGAGCG
CACTGGAACAGAAAGCCCTCCGCATTGTCGAAGCCCATCCCGAATACCACCGTTATCTCG
AACGCATCGAAGACCATCTGGACACCGACTGGCTGCCCGAAAACGGCGAAAGCAACCCCT
TCCTGCATATGTCGCTGCATCTGTCCGTCCAAGAACAGGCGGGCATAGACCAGCCGCACG
GCATACGCGCAATCCACGACACCCTGTGCGCCAAACGCGGCTGGCTGGAAGCCGAACACG
AAATGATGGAGGCACTGGCGGAAACACTGTGGACGGCGCAACGCTACGGCACCGGTTTGG
ATGTCAATTTCTACATGACCCGACTGCGCAAACTCATCGGCTTGGGTGCAGAAGATCAAG
CCAGATTGAACCCGCATGAAATCGCCTGACCATACCAACCGCCTGCAAAATGCCGTCTGA
AGCGGAACAACCCCTTTCAGACGGCATTCATTTTCCCCCAATCATTTCCACAACGCCTTT
TTCAGCATAATCAACCAATCCTTCTTATCCAAAACGGGGCGTTGTGCAAACACATCGTAT
CGGCACGCGTCCAGTTTCTGCAAAATCAACTGCGCCCCCAACACAATCATACGGAGTTCC
AAACCGATACGCCCATTCAGTTCCCTTGCCAAAGGCGAACCCGCCTTCAGCATACGGAAC
GCACGCCGACACTCATACGCCATCAGCCGCTGAAACGCCGCATCCGCCCGTCCTGCCGCG
ATCTGTTCCTCAGAAACACCGAATTTCAACAAATCGTCCTGCGGAATATAAACCCTGCCT
TTTTGCCAATCCACAGCCACATCCTGCCAAAAATTCACCAGTTGCAAAGCCGTACAGATG
CCGTCGCTTTGCGCCACGCACACCGCATCCGTTTTCCCGTACAAAGCCAGCATAATGCGT
CCGACAGGGTTGGCGGAACGCCGACAATAATCGGCCAGCTCGCCGAAATTTCCATACCTT
GTTTTAACCACATCCTGAGAAAATGCAGAAAGCAAATCATAAAACGGCTGCAAATCCAAA
CCGAACGGCACAACCGCCTCGGCATCCAATCGTGCAATCAAAGGATGCGCCGACCGGCCG
CCCGATGCCAACACGTCCAACTCGCGCTGCAAACCCTCCAACCCCGCCAACCTGGCTTCA
GACGGCATACTGCCCTCGTCCGCCATATCGTCCGCCGTCCGTGCAAACGCGTACACCGCG
TGAACCGGCTTCCTCAACCTGCGCGGCAAAATCAGCGAACCGACGGGAAAATTCTCATAA
TGCCCAACCGACATACCTTCTCCATCCATCAAACAAAATGCCGTCTGAAACGGAACAAAC
CCTTTTCAGACGGCATCAGATACCTCCAAGCTGCCGGCAATCAGTGGTGGTGATGACCGT
GCGGGCCGTGGACATGACCGTGTGCGATTTCCTCATCGGATGCATCGCGCACGCTTTCAA
CTGTAGCCTTAAAGCGGATTTTCATGCCTGCCAAAGGATGGTTGCCGTCCACCACCGCCT
TGCCGTCGGCAACATCGGTTACACGATAGACGACAACATCGCCGGTTTCAGGATCGTCGG
CTTCAAACATCATGCCGACTTCGACTTCAACAGGGAACACGCCCGCATCTTCGATACGGA
CCAACTCCGGATCCTGCTCGCCGAACGCATCGTCGGGCGACAGCGCCACATCGACCGTAT
CGCCGGCATCCTTACCGTGCAACGCCTCTTCCACCAAAGGGAAAATGCCGTCGTAACCGC
CGTGCAGATACGCAATCGGTTCTTCGGTTTTGTCCAAAAGCTGATTGTTGGCATCATACA
TCTCATAATGCAGCGAAACCACGGAATTTTTCACGATAGCCATATTTGTCCTTTCAGGAA
CAGCAGATTAATTACAGGCGCATTCTAACACAACCGCCGCGCCGGCCGATTACCGTTAAC
CTGTTCATAAACTGTACAGCACATATTTCAATGTAAATCTTTGTTATTTTATTGCGGTGT
AACTTTTTTACAACATTCTTAAAACCATTCCGACCTGTCTGCCGACTTTCCCAATCCGCC
TTAATAAATCATACAAGATACTGAAATTATATTAATCTCTATAATATTTATCCCTATCGA
ATTTTTAACAGCAAAACCGTTTTACAGGATTTATCAATCCGCCCGCCAGAAAACTTTTCA
TTCAAACCTTTTTCCCATCTGTACGACATTGCAATCCCTTATTCCATAGTGCATAATTAC
GCAAATTCAGCGATGAATTTCCAACCCGGTTTGTAGTATGGTCGATAAAGACCTATTTGT
TTCAATAATTTAAATTGGTTCTAAAGGTTACTAAAATGAAAAAATCCCTGTTTGCCGCTG
CTTTGTTGTCTTTGGTTCTGGCAGCCTGCGGCGGTGAAAAAGCCGCTGAAGCTCCCGCTG
CTGAAGCACCTGCCGCCGAAGCTCCCGCTACTGAAGCACCTGCCGCCGAAGCTCCCGCTG
CTGAAGCACCTGCCGCCGAAGCTCCTGCTGCTGAAGCTGCCGCTACCGAAGCACCTGCCG
CTGAAGCTGCCGCTACCGAAGCACCTGCCGCTGAAGCTGCCGCTACCGAAGCACCTGCCG
CTGAAGCTCCTGCTGCCGAAGCTGCAAAATAAGCATTTTCCGCTTGCAAAAAAGCAGGAT
ACGTTCAGTATCCTGCTTTTTTGATTTTTCAGACGGCATCAGATTCCCTTCCTCAATCTT
CTCCCTACCCTTCCGACAAACATGCTTGACCTTCATACCGAATTTTCCCGACTCCTACCG
GCAGATGAAATTGCCGAACCTTCTCCGACGCTTTTAAAAGACCAGCGCAACCGCTTTACG
TCTGCACCAGACATCATTTTGCAGCCGCTCAGCGTTAAAAGCGTGCAAACCATTATGCGT
~~TTCTGCCACCAACACCGTATTCCGGTTACGCCGCAAGGCGGCAATACTGGTTTGTGCGGC~~
GCGGCAGTATCGGAAAACGGCGTATTGCTGAACCTTTCCAAACTCAACCGCATCCGCAGC
ATCAATTTGTCAGACAACTGCATAACCGTCGAAGCAGGTTCCGTACTCCAAACCGTCCAA
CAGGCAGCCGAAGCCTCAAACAGGCTGTTCCCACTCAGTCTCGCCAGCGAAGGCTCGTGC
CAAATCGGCGGCAACATCGCCTGCAATGCCGGAGGTTTGAACGTATTGCGTTACGGCACG
ATGCGCGACCTGGTTATCGGTTTGGAAGTCGTCCTCCCCAACGGCGAACTGGTTTCCCAT
CTCCATCCCCTGCATAAAAACACCACCGGCTACGACCTGCGCCATCTGTTTATCGGTAGC
GAAGGTACATTGGGCATTATCACTGCCGCCACGCTCAAGCTGTTTGCCAACCCCTTAGAC
AAAGCAACCGCATGGGTCGGCATACCCGACATCGAATCCGCCGTCCGCCTGCTGACCGAA
ACCCAAGCACACTTTGCCGAACGCCTATGCAGTTTTGAGCTGATCGGCCGTTTTGCCGCC
GAATTGTCTTCCGAATTCAGCAAACTCCCCCTGCCGACACATTCAGAATGGCATATTTTA
CTTGAGTTGACCGACTCATTACCCGACAGCAATCTTGATGATCGGCTTGTCGAATTTCTT
TATAAAAAAGGCTTTACCGACAGCGTGTTGGCGCAAAGCGAACAAGAACGTATCCATATG
TGGGCGTTGCGCGAAAACATCTCCGCATCGCAACGCAAACTGGGCACCAGCATCAAACAC
GATATTGCCGTTCCTATCGGGCGCGTTGCCGACTTTGTCCGCCGGTGCGCCAAAGATTTG
GAACAGAATTTCAAAGGCATACAAATCGTCTGCTTCGGACATCTGGGCGACGGCAGCCTG
CACTACAATACTTTCCTGCCCGAAATCCTCAGCAATGAAGTCTATCGTTACGAAAACGAC
ATCAACAGCACAGTCTATCGCAACGTCCTTGCCTGCAACGGCACGATTGCCGCCGAACAC
GGCATAGGTATCATCAAAAAACAGTGGCTGGACAAAGTACGCACGCCTGCCGAAATCGCC
CTGATGAAAAGCATCAAACAACACCTTGATCCATATAACATTATGAATCCGGGCAAACTG
CTTCCGTAACCGGCATTTCTGATTTGCATACACAACAAAGAAAGGACAATAGATCCGAT
TGTCGGTTTAGCGCGAGCTCGTGAGTGCGGTTAAAAATTGGTGGAAATTACACGAAAAAT
GACCGCACTTTTAAAATAAAAAAATCGGCAGTGAATTTCCCTGCCGATTTTATTTTGTTA
CAACTTAACTTAAAACGTCCACTGTAAATTCAACGCACCTTGTTTAGCTTGATGATGTTT
GCCTGTTTGGCGGTTGAATGTGGCTTGTAAGGTTAAGTGAGATTTGATTTTCACTGCTAC
ACCTAATTGGCTCTCAATTGCCGTCTTATTGTTTATCACTCGACGCTCTCCGTCCATTTC

## Appendix A

CACACCGAAAGGTTTGTTGTGGTAAAGCGCGTTCACAGCGGCGAAAGGTTCAATAGCGAT
ATTTTTATAGAGTGAAAATTGAGCTTTAGCTTGAACGCCAACCCGAGTTTGTAATTGGCG
GGAGCCAAGTAAATTCACGTGGGCATTTTCGCTATCGCTGAATTTTCCGTTTACCCCCAA
ATAAGTCAATTGTGCCTGTGGTTGTAGGTAAACACGAAGGCTGTTGCCCTTTTTAGTGAA
GTGTTCCGCCAATAACGCATTGTAACCTGCTTCAATTGAGGCAGTAATACCTTTTGAAGT
AAAACGTTCTGTACCATCTTCAGTGTTGATACGGTGGCGGAAGCGTTGATATTGCATCCA
GCTATCCGCATACGCACCTGTCTGTTTGTCCTGAAGTTGGTGCCAAGTGGCGTAAACGCC
TGCACCAAAGCCTTTCACATTTCCCGTTGTAAGATTGTCTGTATCTGGGTTGTGGAAAGT
GCTACGTTGTTCTGCTTGTCCGCCCATTAAGCCAATAGAAAGTTGATTACTTTCGTTTTG
CCATGTGAATACTTCGCCGCCGAGTTGCACACCTTTACGATAGCCTTCTACAGGTGCTGT
TTTGCCTTGCACCCATTGGTTGGAATGTCCGTCAATCACACGCAACCACAAGCCTTTGCG
TGGTAAAGTGCGGTCGAAAATATCGCTGTTTTTGTTGTTCAAACGCAAGGCGAATAAGGT
ATTGGCGGCTTGAGCCTGTTGTGCATAAATCGCCATATCATCGCGTTCTTGCACTTTGGT
AAAAAAGCCCTCTGGGCGTTGTTGTAAAGAAAGCGTATAAATTCCCTTTTGGTGTTTGCC
AGAAAGACGGAATGCGTGTTTATCTGCTGTGCCATTTACTTTGATAATTTGATGCCCATC
GAGGCTTTTTAAATCGTCTATTGGATTTTCGAAGATGATGTCGGAAGTGCCAGTAACATT
TTTCTCAAAAATTAATGCAGTATTTTTCGCTTCTTTAGGATCGTAAGCAAAACGAAAACG
AGCTCCGCCAGCATAATCTTCTTTTACGAGTAAACTTTCACTTTTAGTATTAAAACGGAT
GTCTGCATTCGTTGTTTTTAATTTCCCAACATTAGAATCCCAACGGGGCTCCCAGAGAGA
ATTTTCTAAGCGGAATTCATCCAAACTAATCGTTTGCCCGATAACGTGCGAGTTGTCTGT
AACCTCAATATAGTGGAATGGATCTAAACCAGAATATAGATGTGCTGCAAAAGAAACATA
ATTTTCAATATGATGAATTACTTGATTAGCCCATTCTGTATAATTCCCGACAGATAAAAT
TTCGCTGTTGATATGACTATTTTTTATTTTTGGACCTAAGGAGAATATATGACTTTTTAC
TATAAGAGGATGGGATCCAAATTTTTCAGCTTGGCAAGTACTATAATCACGTATCTTAGT
GTTAGAATTAAAACATTCCTTAAAATATTTCCGTATTTGTTCTTCTGTGTCCCCATTTCT
TTTTGCAACCCCTAAACCTCGGGCGAAGCCAACTAGGTAACCTTCGGTATATTCTTGATC
ATAAAAAGAAATCTTTTTTGAGTTATTGATGTTTTCGAATTGGTATGTTCTAGGGTATAG
TGCGGGAAAGGGTGGAACTTTTGGATTATCCTCGGTTATAAGATAAGTTTCTTTTTTCCA
ATATTCACTCGTTTTATCGCGGAGTTTTTTTAAGCGGGTAATTTCATCATTAGTGAGCTT
GGTTTTGTCGTAAACGTAATCAACAGCCAAAAGCGGAGAGGTATAAAGAATAGAAAAAAA
TAGACTTACAATAAATGATTTTTTAAACTTCTGCTTGCTTGCTTGCTTGCTTCGAGTTTC
ATAATAAATTTTCCTTTGTCAAGTAAAAATAAATGGGGCGTGGATTTTAGCATAAAACTG
AACAAAAAAATGTCATTTATCTCACATTTTTCTCTATTTATTTCTTGTTTATTAAAAGTAA
ACGTTTGCTTTTTGCTATTTTGTCAAGCCAGTTTGAAAATGTGTATAATTGCCCTCGTTA
TTTACAAAAATTTCAGGAAAAATGACCGCACTTTACCCTTGGCTAATGCCAATTTATCAT
CAAATTGCTCAAACCTTTGACGAAAGCTTGGGGCATCATGCCGTGCTGATTAAAGCGGAT
GCTGGTTTAGGTGTAGAACGTTTACACATCAGGCGGCAGCCTTGCCCATACCGTCTGAAG
CACTGTTTCCACAATCAGCGCGTATGCTTAATCAACCGCTGTTTCTCGCGTTTCCAATCC
GCCTCTTTCATACTCTGGCGTTTGTCGTGCTGTTTCTTACCTTTTGCCAAACCGATTTCC
ATCTTGATTTTTCCGCGTGAAAAATGCAAATCCAGCGGCACGATGGTGTAGCCGGCACGT
TCGGTTTTGCCGATTAATTTGTTGATTTCCGACTGGTTCAACAAGAGCTTGCGCGGACGT
ACGGCATCTGGTTTAATGTGTGTCGAGGCTGTGGGCAAAGCCGTAATATGGCAGCCGACC
AGATAAAACGCGTCTTTTTTCCAATAGATATAACTCTCTTTAAGCTGTACGCGCGCGGCG
CGGATTGCTTTGACTTCCCAGCCTTCCAAGACCAAACCGGCTTCAATCCGGTCTTCAATG
AAAAAATCGTGAAATGCTTTTTTATTGTTCGCAATAGCCATAAACATCCTATCAATATCC
GCCGTCAGACGGCATAAACCCGAAAACAGAACCCATCATACCGCCTCTTCAACCGCCTGC
ACAATCTTCTCGGGATACAGCCTGTTGAGGCAGTCGGTATGCCCCAGCGGACATTCCCGC
TTAAAACACGGCGAACATTCCAAGTGCAGGCTGACGATTTTCGCCCTATCGCTCAAAGGC
GGCGTATGCGTCGGGCTGGAAGAACCGTAAACCGCCACCACCTTCCTGCCCAAAGCTGCC
GCCAAATGCATCAATCCGCTGTCGTTACACACGACCGTGTCCGCCAACGACAGCAAATCC
ATTGCCTGCGACAAATCGGTTTTGCCGCACAAATTGACACACATACCGTCTGAAAGGCGG
TTGATTTCCTCGGCAATTTCATCATCTTTTTGCGAACCGAACAGCCAAACCTGCCAACCC
GCCGCCAGATAATGTTTGCCCAACTCGGCAAAATGCCTTGTCGGCCAACGCTTTGCCGGC
CCGAATTCCGCACCCGGACAAAAAGCCAGAACAGGCTTTCCAATATCCAAGCCAAAGGTT
TCGACAGAAATTTCCCGCCGCCGTTCATCAATGGAAAACTCGGGGAATCCCGAATGCCCG
TCAAAATCTTCCTGACTCGGATGCGCGAGAGCCGTATATCGATCCACCATCAAAGGCAGA
CGTTCCTTATCCAGCCTGCGTATATCGTTCAACAGAAAATAACGGCTTTCACCGACATAA
CCCGTCCTTTTACCGATACCTGTCGCCAGCGCGATGATTGCCGATTTCAAAGAACCGGGC
AACACGATAACCTGATCGTATCCGCGCCGCCCCAAATCCCTACCGACCCGCCAACGGCGT
TTCAACTCCAACGCACCATGTCCGAACGAATTCTCAAGAATTTCATTCACTTCCGGCATA
CGCTCGAACACCGCCATCGACCACTTCGGTGCGAACACATCAATCGTGCAACCGGGGTGA
AGTTCCTTCAAACGGCGGAACAAGGGCTGGGTCATCACGCAGTCGCCTATCCAACTGGGG
GAAATAATCAGGATTTTGATGGACATAACAAGAAACCGAAATCAGACAGGCAGAATTTTA
CCGCGAAACCGTTGGAAAACCTATCTTGCCGCATTCCGAACGCCGGACGTGCAAATATGA
AAAAGCCCGAACATTCAAGTTCGGGCTTCAAAATTCTGGCTCCCCGACCTGGGCTCGAAC
CAGGGACCTGCGGATTAACAGTCCGTCGCTCTACCGACTGAGCTATCGGGGAATGGGGCG
TATTATAGCGTCCGGAAAAAATGTGTCAATCCTTAATTTTGGAAAAATGGGCGACAAAAC
GACAAGCATATGAATCAGAAAGACATTAAGACCGATGCCTTAAAAGGATTGCCGTTGTAT
GAATTTCCACAGCCGTCATCACACCATATTTAAGCCCGATGAGCCGTTCTGCCCTCCCCC
CGCTTAAAACAATGCCGTCTGAACTTCGCCGTGTTCCAAAGCCAGTAAAAACTGTTTGCG
GTTCAACCCGCCCGCGTAGCCGGTCAGTTTGCCGTCGCTGCCGATGACGCGGTGGCAGGG
AATCAGGATAGATACTTTGTTCTGCCCGTTGGCGGCGGCAACGGCGCGGACGGCTTTGGG
GTTGCCCAAACGCTGCGCCTGCTCCTTGTAGCTGCGCGTTTCGCCGTAAGGAATCGCCAA
GAGCGCGTCCCATGCCTGCTTTTGAAACTCGGTGCCAATCTGCTCCAAAGGCGTGGCAAA
GGTTTTCAGACGACCCTTGAAGTATAAGTCCAATTCCTGCCGCAAAAGTTGCGTCCGCTC

# Appendix A

ATCCTCCCGAAACACAAACCGTCCGCGCAAGGCTTTTTGGACGGCGGCAATTTCCTGTTC
CAAATGCTTCTGTCCGACAAATTCCAGCAAACACAAACCCCTGCTACCGAACACCGCCAG
CATCTCGCCCAAAGGCGTGGCAATGGCGGCACACACCAGCTCGTTCAAACTGTCGGGATA
ACGCGCTTCCAACAGACGGATGGCGCGGCGGATGCGGACATATTCTTCAGGCGCGCAGCC
GATATTGTCCCAAAAATCCCGCTCGAACTGTTTGGCTTCGCATTCCGTCAGATTGGGATG
CGGCATAACGCCGCACTCAAAAACCCGAGATTCGAGCCAATGGCGGATTTCATCCCATTT
TGACGGCAGATTGTTTAAGGAAGGCAGGGTAATCATTTGTTTGCTCCGTATCCCTATCAT
AGATTTGACGGCAAAATCCCCAATTTTTGCCATTCCCGCACGCCGGAGCAGGAACGGGCT
ATGACGTAAATCTTGAGGGTTAGGTTGCGGCAATACCTAAATATTCGATATTTCTAAAGC
ATCAGAGAAAGGAATGTTTCAACACACAGGACGACACATAAAGCGCCGCCCCATGAAAAA
TTTCAGACGACCTGCAAAGGGTCGTCTGAAACCACGATTTTTGCATTTGCGCATTCTGGC
ACATCATCCAACCGTTTCGGCACATTCCTGCCGCCGTTGACAGCCTATAATGAATCCACT
TATTCATCAAGCAAAGGAATCATCTATGCAAACCCTCATCCTCTCCGCCGTACTGCTGGC
TTTTTCAACCGCTGCCTTTGCCGGGGGCGCATTCACGCTGCAATTCGACAACCCGTCCGA
AGACGGCGGCTTCACGCAAAACCAGCTTTTGAGCGCGCCTTACGGCTTTTGCTGTTCAGG
CGACAATGCTTCGCCCGCGCTGTCGTGGAAAAATCCGCCCGCCGGGACAAAAGTTTCGTC
CTGACCGTTTACGATAAAGACGCGCCGACCGGACTGGGCTGGATGCACCGGGTGGTCGCC
GACATTCCCGCCGATGTCCACCGCCGCAACGCGACCTCGCTGCAATTAAGCCGCTGCGCC
AACATCGCCGACCGGACTGGGCTGGATGCACTGGGTGGTCGCCGACATTCCCGCCGATGT
CCGCCGCCGCAACGCGGCCTCGCTGCAATTAAGCCGCTGCGCCAACATCGCCGACGACCA
GTCCGCAGCCATATCGGCGGTAATCAGTTTGCGGATTTGCCGCATCAGGTTGACGCCTTC
GTACACGGCAAAACCGATGCCGTCATGCTGCAACCACGCCAACACGCCGCAAAGCGCGGC
CTCCGCAGCATTGTGCGGCACTTCTTCATCCGCCAGTACCGCAGCCTCATAATCAAACGC
CGCGCCCATACGCCCGGAATACGGCAGCTTTACCGCATCGCACACTGCCTGCGCCGTCCC
GTATTGTGCGGCGAACCTTTCTACGGTTTCCTGTTCGAAAGCAATCCATTGCGCCTGATA
GAGGCCGTCTGAATCGGGAATATTGATGACGTCAAACGTCTGTCCGCCTGCCAAGGCGAC
CGCCTTACCCGCCGCAGCTTCTTACTTCCGCGCCGCACGATAAGCACAGCCGGTTCATAT
ACCGCCACGCTGCGGTACAAGGCGGTATGATGTTGCACGATGCCGCCTAAAGCACCCAAT
CGTTCGCGCGTATGAAAGTATAGTGGATTAAATTTAAATCAGGACAAGGCGACGAAGCCG
CAGACAGTACAAATCGTACGGCAAGGCAAGGCAACGCCGTACTGGTTTAAATTTAATCCA
CTATATCTCAAACCCACGTTAGGTCTAAGCAAATGGTCGGACATCCTTATCCGACAGCCC
ATCTTCTTTTCAGACGGCATTGCAAATTTAAGTTTGACGTGCGTTCAAAATAAGGCAGTT
AATGCGAAGCGAAATTCCGTCGGCGTACCTGCAACTTGGCCCCTCCCCTATAGGGGAGGG
TCGGAGGGAGGGTAAAACGGGGCAGATACAGACAATATTTCCGTTGCCGCCCCGATGCCC
TCTCCCTAACCCTCTCCCACGGGAGAGGGAATGGATTGCCGTTGAAATAAATCGCTCTAC
ATAAAAAATCAATGTGTTATCTCAAACCCACATTAGGTCTAATCAAATGGTCGGATATCC
ATATTCGGCAAGCAAGCTGCTTTCAGACGGCATTTCCAGCCAACAAGCGCGCCAATATCC
CCTCATACACCGCAGACAGCTTCGGAATGTCGTTTAGCCGCACGTTTTCGTTGATTTGGT
GGATGGTCGCATTGGACGGGCCTAATTCGATAAGTTCTTGCGCAATGGCTTTGATGAAGC
GTCCGTCCGAAGTGCCGCCGGTGGTGGACAATTCGGCCTCAATGCCGCAGGTTTCGGCAA
TGGCTGCGCGTGCCACGTCGGTCAGTTTGCCCGCTTGGGTCAGAAAGGGCTGCCCCGAAC
ACGACCACTGCAAATCGTATTGCACGCCGTGTTTGTCCAAAATGGCGTGGACGCGTTGTT
TCAGCCCTGCTTCGGTGGACTCGGTGGAGAAGCGGAAATTGAATTTGACGTTCAGCTCGC
CCGGAATGACGTTGGTCGCGCCTGTGCCGCCGTTGATATTGGAAATTTGAAAGCTGGTTG
GCGGGAAATATTCGTTGCCTTCATCCCAGACTTCCTGCGTCAGCTCTAACAAGGCCGGGG
CAAAAGTATGCACGGGATTGATTGCCAAATGCGGATAGGCAATATGGCCTTGCTTGCCTT
TGACGGTCAGGTTGCCCGACAGCGAGCCGCGCCGACCGTTTTTAATCATATCGCCCAATT
TGTCCACGGCGGTCGGTTCGCCGACGATGCAGTAGTCGATAAGCTCGTCGCGCGCTTTCA
ATACATCGACGACTTTGGTCGTGCCGTCCAACGCGTCGCCCTCTTCGTCGGAAGTAATCA
GAAGCGCAATGCTGCCTTGGTGGTTGGGATGTTTGGCAACGAAGCGTTCGCAGGCGGTAA
CGAAACAGGCAATGCTGGTTTTCATGTCTGCCGCGCCGCGCCCGTATAATCTTCCGTCGC
GCTCGGCCGGTTCGAACGGGGGCGAATCCCATTTTTCGACAGGACCTGTCGGTACAACGT
CGGTATGCCCTGCAAAACAGACGACGGGAGCTTTCGTGCCGCGTCGCAACCAGATGTTTT
TGGTGTCGCCGAAATGGAGTTCTTCAGCCGCAAAACCGATTTTGTGCAGGCGTTCGGCAA
GGAGTTTTTGGCAATCCCTGTCGTCAGGGGTAACGGATGGTCGGGAAATCAGCTCTTTGG
CAAGCTCTAGGGATTGAGTTTCGGTCATATTTGTTCACTTTTGAAATTAGACCGTCTGAA
ACGTTCTGAATGTGATTTTCAGACGGCATTTAGGTTAGGTTGGCATACGGGGTGGGTATT
TTACCCATCAGTCTTCTGAATCATTTGCCGTGGCAGGCTTCGTAAAGCGGCAGCAAATCT
TCCACCGTTTCCGCTATCCATTTCGCGACATCCTGCCTGCCCAAATCGTCGCGTTCGATG
TGTTTGCCGATGCAGAAAAAGTCTTCGTCGTTTTGCAACTTTCGGTCGGACTCGTTTTGT
TGCGCGACGGTGCGGTAGTCGTCGTATTCGCTTTCCGCACCGTGCCACATATCGAAAGAA
GCGTATTTTTCGGTATCAAAATTATCCAACCAGCGGTTGTAATCAGGCAGCGCGATGGGG
GAAACATCGGCTTTATAGCAGTGCCAATCCAAGCTGACGCTCAGACGGCGGCGGTTGAGC
AGTATCGACAAAATCGCTGCGGAATTTTTATATTGTTCGTATTTGAAGTAGGCAAAGAAA
TGGGCGCGAACCTGCCAGCCGTTACACCAGCGTTCGATGTGCGGCGGCGCAAACGGCGCA
CCCAATTCGGCGGCAACCTGCTGAATCAGCTGCTGCCATATCTGCCAGTTTTCTTTATAG
TCAGCCTTGATTTGCGGAATGCTTTCAGGCTGGTATTTTTTAAGCTGGGAAAATTGGAAA
AACGGGATATTGAACAAATCGCAACTTTTCGGGGTCAGCATAATATATCCTTGAGACGAT
TGTTTCAGACGGCATTATTTGCGCCGGCGCGCCGCCATAATTTCGCCGATTTCGGTCAGT
TTTTCTTTTGGGATAAAGGTGTTGCCCATATCAAACAGCGGCTCTTCAATCGCCAAATGA
ACATCATATCCCGCCACAAAACGTTTGAACGCTTCCTCATCGGGGACATAAGCGTTGTCT
GCTTCGAGTTTGGCAAATTCGGCGGAAACAGCCGCCCAGTTGTCGTGCAGCCCGATATGT
TGGCGCAAAAGCTCGTCCACGCTTTCTTGGGCTTGCGGCGCATATTGCAGCAGCAGCGGG
AAGAAGTTTTCTTCTTCGTCTTCATGGTGCAGCGGCGCGGCAACGTTGAAATACTGGGCG
ATTTGGCGGATGGTTTGCAAAACAATCTGATTGCAGCCGTTTTCGGCGATATAGTCCGAC

## Appendix A

AGCATGGCGACTTGTCCGCAAAAACGGCGCACTTTGCCGTGGCAGGCATACAGCATTTCA
ATCGGTTCGGCAAAGGTAACGCTTTTGGTTTCAAACGGATTCATGTTTTCGTTCTCAACG
GGCACTTTTCAAGCAGTCATTTTATAATAAAACAGCCTGCACAAAGCAGGCTGTCCGTCT
TTTGAGACTTTAAGCGGATTAATCGACCAAAGTCACTTTGCCGTTCATCAAAGCACCGTG
ACCTGGGAAGGTACAAGCGAATTTATATTCGCCGTCGGCCAATTTAGCAGGATCCAGAGT
CAGGGAAGCTTCTTCGCCGCCGCCGATCAGTTTGGTATGGGCAACAACGCGTGCATCATC
AGGTTTGACATAGTCGGTATCGGCAGCACCTACGCCGTCTTTAAATACGCCGTCCATGTC
TTCAGCTTTGGCAATCACGAGATTGTGACCCATGCTGGCTTTGGGTTGCGTACCGGTATG
TTTCAGAGTGATGGTGAACTCTTTACATGCTTTGCTGACTTGGATGTCTTTGGTGTTGAA
CTGCATATTGTCGTTGGATTCGACAGTTGCCGCACAGTTGCCGGCAGCAGGGGCTTCGGC
AGCATCTGCAGGAGCAGCTTCGGCGGCAGGCGCTTCGGAAGCGGGTGCTTCAGCAGCAGG
AGTTGCCTCGGCAGCAGGCGCGGCAGGTTCTTGAGAGCAGGCAGCCAAACCGATAACGGC
GGCAGAAATCAGAGCCAGATACGCTTTCATAACAAATCTCCAATCGATAAAATAATATTC
GGTTTTACAGAAATCAAAGTGCAACCGCCATTAACAAAACCTTGAAAAAGATTCCGCCGC
GTTGCACAAACAGATGTTTCGGAGCGGCATTTTGCTACAAATTTCATTTGAAATCAAAGC
CTGTTTGCAAGTTTACAATCGTTTACCCAAAAAAGGGCAATTTTACCCCGAACCTATTTC
TTTAGTATTAGACCTATTATCCTTTACTTCTTAATATTAACGGATGTTTACACAAATTCC
CGTATACATTTTATGCGCCATGCCTTCTAACCAAGTTTGCCAATGCCTCCGCCAATTCGG
GATGCCGTTTTTCCAACTTTGCCGCCGCCGAACCGAAACTCTCCAGCGCAGCCTTACTCA
AATGCAGGGTATTGGTTTTCGGCGGTTTTTCCGGTTTCGGGACCAGCCTGACCGAAACAG
AGCGTATCGAAGCATCAAGCCCTGCCAACTGCGGCAATACCGACGGTGCAATCATTTTCA
AGCGCGATGCCGCCATATTGTTTGCCGCCAAAAGGACAAGCCTGCCGTCTTCGATACATG
CCGTCTGAAAATGCGGGTGCAGGTTGGCAGGCAGCAGTTTTTTCACGGCGGCATCCAACC
GCCGCCACTGTCCCGCCTGTTTCAAAAGTCCGGAAAGCAGCGCGTCCCGCCTGCCCAACT
GTTCCAAATTCATAAAACATACACCCAAAAAGATTGAAATACCGCAAACGCGCCTTTATT
TCAGACGGCATTAGCACTTTGCACAAACGCTTGTGTTAAAATCGCGTTTTCGCCCACTAT
TATATCAGGCGCAGGAATTATTCATGCTGACAAACATTGCCAAGAAAATCTTCGGCAGCC
GCAACGACCGCTTGCTGAAACAATACCGTAAATCCGTTGCCAGAATCAACGCGCTCGAAG
AACAGATGCAAGCCCTAAGCGATGCTGATCTGCAAGCCAAAACTGCCGAATTCAAACAAC
GCCTCGCCGACGGTCAGACTTTGGACGGCATTTTGCCCGAAGCCTTCGCCGTCTGCCGCG
AAGCGTCCCGCCGCACCCTCGGTATGCGCCACTTCGACGTGCAGCTTATCGGCGGTATGG
TGCTGCACGACGGCAAAATCGCCGAAATGCGTACCGGCGAAGGCAAAACCTTGGTCGCCA
CCCTCGCCGTCTATCTCAACGCGCTGGCCGGCAAAGGCGTACACGTCGTTACCGTCAACG
ACTACCTCGCCTCACGCGATGCGGGCATTATGGAGCCGCTCTACAATTTCCTCGGCCTTA
CCGTGGGCGTGATTATTTCAGATATGCAGCCGTTCGACCGTCAAAACGCCTATGCCGCCG
ATATCACCTACGGCACCAATAATGAATTCGGCTTCGACTACCTGCGCGACAATATGGTTA
CCGACCAATACGACAAAGTGCAGCGCGAATTGAATTTTGCCGTTGTCGATGAAGTGGATT
CCATCTTGATTGACGAAGCGCGCACTCCGCTGATTATCTCCGGTCAGGCGGATGACAACA
TCCAGTTGTACCAAATCATGAACACCGTTCCGCCCCACCTCGTCCGTCAAGAGACAGAAG
AAGGCGAAGGCGACTATTGGGTCGACGAAAAGGCACATCAGGTCATCCTGAGCGAAGCAG
GTCACGAACACGCCGAGCAAATCCTGACCCAAATGGGATTGCTGGCAGAAAACGACTCCC
TCTATTCCGCCGCCAATATCGCCCTGATGCACCACCTTATGGCGGCATTGCGCGCGCATT
CCCTCTTCCACAAAGACCAACATTACGTCATCCAAGACGGCGAAATCGTCATCGTGGACG
AATTCACCGGCCGGCTGATGTCCGGCCGCCGCTGGTCGGAGGGTCTGCATCAAGCCGTCG
AAGCCAAAGAAGGCGTGGAAATCAAACGCGAAAACCAAACGCTTGCATCTATTACCTTCC
AAAACTATTTCCGCCTGTACACCAAGCTCTCCGGCATGACCGGCACAGCCGATACCGAAG
CCTTCGAGTTCCAAAGCATCTACAACCTCGAAACCGTCATCATTCCGACCAACCGCCCCG
TACAGCGCAAAGACTTCAACGACCAGATTTTCCGTTCCGCCGAAGAAAAATTCGAAGCCG
TCGTTAAAGACATTGAGGAATGCCACAAACGGGGGCAGCCCGTCCTCGTCGGCACCACCA
GCATTGAAAACTCCGAACTGGTATCCAAGCTGCTGACCCAAGCCGGACTGCCGCACAACG
TCCTCAACGCCAAAGAACACGAACGCGAAGCCCTGATTGTCGCCCAAGCCGGCAAAGTCG
GCGCGATTACCGTTGCCACCAATATGGCGGGACGCGGTACGGACATCGTTTTAGGCGGCA
ACCTGAAGCACCAAACCGATGCCATCCGCGCCGACGAAACCTTGAGCGACGAAGAGAAAC
AGGCACAAATCGCCGCACTCGAAGACGGCTGGCAGGCGGAACACGACAAAGTGATGGAAG
CAGGCGGTTTGCACATCATCGGTACGGAACGCCACGAAAGCCGCCGCATCGACAACCAAT
TGCGCGGACGTTCCGGCCGTCAGGGCGACCCCGGATCCAGCCGCTTCTATCTCTCCTTTG
AAGACCCATTGCTGCGCTTATTCGCACTCGACCGCGCCGCCGCCATCCTCAACCGCCTCG
CCCCCGAACGCGGCGTCGCCATCGAACACAACCTGCTGACGCGCCAAATCGAAGGGGCGC
AACGCAAAGTCGAAGGCAGAAACTTCGATATGCGCAAACAGGTTTTGGAATACGACGACG
TTGCCAACGAACAGCGCAAAGTCATTTACAGCCAGCGCAACGAAATTCTGACCAGCAAAG
ACATCAGCGACCTGATGCAGGAAATCCGTTCTGATGTCGTCAGCGACCTCGTGGATACCT
ATATGCCGCCCGACAGCATGGAAGAACAATGGGACATCCCGACTTTGGAGAACCGTCTGG
CTGCCGAATTCAGACTGCACGAAGACATCCAATCCTGGCTGAAGGCGGACAATGCGATTG
ACGGTCAAGACATCAAAGAACGCCTGATCGAACGCATCGAAAACGAATATGCCGCCAAAA
CCGAACTGGTCGGCAAGCAGGCAATGGCCGATTTCGAGCGCAACGTGATGTTGCAGGTCA
TCGACAACCAATGGCGCGAACACCTCGCCGCTATGGACTACCTGCGACAAGGCATACACC
TGCGCAGCTATGCCCAAAAAAATCCGAAGCAGGAATACAAACGTGAAGCCTTTACCATGT
TCCAAGACCTGTGGAACGGCATCAAATTCCATATTGCCTCCCTGCTTACCTCGGTTCAAA
TCGAACAAAACCCTGTCGCGGTGGTTGAAGAGCAACCCATCGGCAACATCCAGTCCATCC
ATTCCGAATCGCCCGATATGGAAGAACTTTTGGGTCAGTCGCAAACCGATCTGGTTACCG
AAGCCTTTAATCCCGATGGGACAGATTTCAGCCCCGAAGCCTTGGAAGCGCGGGGGCAAA
TCGTCCACCGCAACGACCCCTGCCCCTGCGGCAGCGGTTTGAAATACAAACAATGCCACG
GCAAACTGGCTTAAGCGTTTGAACGCAAATGCCGTCTGAACATCCCGCTCCCGTTTCAGA
CGGCATTTTGCCTGAACCGCCACATCCGACTGCCATTCCGAAAAATCCCGATTTCGTACC
GTCCGTACCAAAAACAGACATCCCGTCCGCCCCACATCATGATTCCATCCGACTTCATTG

# Appendix A

ACGAGCTTTTAGCCAAAACCGATATTGTCGATATTATCGACGAGCAGGTTCCGCTGAAAA
AAGGCGGGGCGAACTATATGGCGTGTTGCCCGTTCCACAAGGAAAAAACGCCGTCGTTTT
CGGTCAGTCCAACCAAGCAGTTTTACCATTGTTTCAGTTGCGGGGCACACGGCTCAGCGA
TTGGTTTTGTGATGGAACATCAGGGACTGTCGTTTCCGGAGGCGGTTCAGTTCCTTGCCG
ACCGCGTGGGTATGGTCGTGCCGAAAGTGCACGGGCAAAACGATAATCCCGAAGTCCGTG
CCGAACGTAAGAAAAAACAGCAGACACTGGAGGAAACGACGGCTGCGGCAGCTGATTTTT
ACGCGCAACAGCTAAAATTCAATCCAGCGGCAAAAGCTTATTTGGACAAGCGCGGCTTGA
GTGCAGAAGTTATCGCGCATTATGGTTTGGGCTATGCGCCCGACGGCTGGCAGCCTTTGA
CGCAAGTGTTCCAACCGTATCCTAATACCGCGTTAGTGGATACGGGGATGGTGATTGACA
ATGAGGGACGGCATTACGACCGCTTCCGCCATCGGATTATGTTCCCCATCCGCAATCCGC
GCGGGCAGGTTATCGGTTTCGGCGGCAGGGTGCTGGACGACTCGAAGCCGAAATATTTAA
ATTCTCCCGATACGCCTTTGTTCGATAAGGGGAAAAACCTTTACGGACTGTATGAAGGGC
GTGCCGCTGTCAAGGAAGCGGGGCGGATTTTGGTGGTCGAAGGCTATATGGACGTGGTCG
CGCTGGCACAGTTCGGCGTGGGCTACGGCGTGGCGGCTTTGGGTACGGCGACGACGGCGG
AACACGTCAAAATCCTGATGCGTCAGGCAGACAGTATTTATTTCTGTTTCGACGGCGACA
GCGCGGGGCGAAAAGCGGCTTGGCGCGCGCTGGAAAACGCGCTGCCGCAGTTGAAGGACG
ACAAATCGCTGCATTTTTTGTTCCTGCCGGAAGAACACGACCCCGACAGCTACATCCGCG
CCTACGGCAAAGCGCAATTTGAAGACGCGCTTCTGAATCAAAGCAAGCCTTTGTCGGAGT
ATTTCTGGGAACACCTTTCAGACGGCATTCATCTCAATACGCAGGAAGGCAAGGCGGAAT
TGGTAAAAACCAGTTCGCCGCTTTTGGCGCAGATTACCGCGCCGGCATTGGCTTATTTGT
TAAAACAACGGCTTAGCGAGCTGGTCGGCATCGACCCCGACAACCTCGCGCAACTGCTAG
GACAGGAAGCGCCGAAGCGGCACGTCAAACAAAAAAACTACAAACTGCCTCCGATTTCCG
TCAAACAGCCCGTCATGCTGACGCTGGTACAGCGGCAAATCCGCAGCCTCTTGATAAATC
CGGATTGGGCTGCATATATAGACCTGCCCGATTATCTGGCGTTGGACGGTGATTTCGCCT
GCCTTGCCAATCTTGCCGAATCGATTAAAAACCATGCCGCCGTACCCGAAACCGCTCAGG
TTTTAGAGTATATGCGCGGCTCGCCTTACGAAGAAACGATAACCCGAATCTTCCATTCAA
CGCACCAATCGGAAGAAATGAACAGCAGCAGTGAAGAAGATTGCGAGAATTTCCAAATCG
GCATGAAAAAACTGCTCAATGAGTTAAAATACAGCCAAATCGAAACATTAAAACAAAAAA
GCCTGCAATCCGGCTTAAATGAAAGCGAGAAAAAACTTTTGCTGTCGCTGCTGACCGCAA
AACAAAATTGACCGGCGGATTCCGCCATCCGTAAACCGTTATGCCGTCTGAAAAGCATTC
ACCCCGGCTGCAACAACGACACCTGCAGAACACCCATCCCCAAAAGCCTTCAGACGGCAT
CAGAGTACCCTACTCTGCCACGCCTTCAGGTGCGTCCAAACGCAAACCGTCGGCATCTTA
CCAACAGAAAGCAGACAATGTCCAGAAACCAAAATCACGAAGAATATCAAGACGACACCC
GTCCGTTAAGCATTGAAGAGCAACGCGCGCGCCTGCGTCAGCTCATCATCATGGGTAAAG
AACGCGGCTACATCACCTACTCCGAAATCAACGACGCCCTGCCAGACGATATGTCTGATG
CCGACCAAATAGACAATATCGTCAGCATGATTTCCGGTTTGGGCATCCAAGTTACCGAAC
ACGCCCCCGATGCGGAAGACATATTGTTAAGCGACAATGCCGCCGTTACCGACGATGATG
CCGTCGAAGAAGCCGAGGCCGCCCTTTCCAGTGCAGATTCCGAGTTCGGCAGAACCACCG
ACCCCGTCCGTATGTATATGCGCGAAATGGGACAGGTCGACCTGCTGACCCGCGAAGACG
AAATCATCATCGCAAAAAAAATTGAAAACGCCCTGAAAAATATGGTTCAGGCCATCTCCG
CCTGCCCGGGATCCATTGCTGAAATCTTAGAACTCATCGAAAAAATCCGCAAAGACGAAA
TCCGCGTCGACGAAGTCGTAGAAGCCATTATCGACCCGAATGAAGTATTGCTCAACGAAT
TGGGCTTGGGGCACTTGGAAACCACAGCGCCCGAGAAACCTTCCAACGACAATTCGGACG
AAAACGAAGACGACGAAGAATCGGAAGAAGATGCGGATGAAATCTCGGCAGCCAATCTCG
CCGAATTGAAACAAAAAGTCATCGGCCACTTTGCCCAAATCGAAAAAGACTACAAAAAAA
TGATTGGCCGTTTGGAAAAACACCACAGCCGGCACAAAGACTATCTCGCCTACCGCGACG
CGATTGCCAACAAACTGCTGGAAGTCCGTTTCGCCACCCGGCAAATCGACAGCCTCAGCA
GCAGCCTGCGCGGGAAAGTAGAAAACATCCGCAAACTCGAACGCGAAATCCGCGACATCT
GCCTCGACCGCGTCCATATGGAACGCGACTACTTCATCCAAAACTTCCTGCCCGAAATCA
CCAATCTAGAATGGATTGAAGAAGAAATCGCCAAAGGCAGGGTTTGGAGCGACGCGCTCG
ACCGCTTCCGCCACGCCATCCTCGAAAAACAAACCGAGTTGGCGGATATGGAAAAAGAAA
CCCGCATTTCCATCGAAGAGTTGAAAGAAATCAACAAAAATATGGTGTCGAGCGAAAAAG
AAACCGCAGCCGCCAAACAGGAAATGATTCAGGCAAACTTGCGCCTCGTGATTTCCATCG
CCAAAAAATATACCAACCGGGGCTTACAATTCCTTGATCTGATTCAGGAAGGCAACATCG
GTTTGATGAAAGCGGTCGATAAGTTCGAATACCGCAGAGGCTATAAATTCTCCACCTACG
CAACCTGGTGGATCCGCCAGGCAATTACACGCTCGATTGCCGATCAGGCGCGTACCATCC
GCATTCCGGTACATATGATTGAAACCATCAACAAGATGAACCGCATCTCGCGCCAACACC
TTCAAGAAACCGGCGAAGAACCCGATTCCGCCAAACTTGCCGAACTGATGCAGATGCCCG
AAGACAAAATCCGCAAAATCATGAAAATCGCCAAAGAGCCGATTTCGATGGAAACCCCCA
TCGGCGACGACGACGATTCGCACTTGGGCGACTTCATCGAAGATGCCAACAATGTTGCGC
CGGCCGATGCGGCAATGTACACCAGCCTGCACGAAGTAACCAAAGAAATCCTCGAAAGCC
TGACACCGCGTGAGGCAAAAGTCCTGCGTATGCGTTTCGGCATCGATATGAACACCGACC
ACACGCTGGAAGAAGTCGGCAGACAGTTTGACGTAACGCGCGAACGCATCCGACAAATCG
AGGCAAAAGCACTCCGCAAGCTGCGGCATCCGACAAGAAGCGACCGTTTGAGAAGTTTCT
TGGACAGCGAAGACAGCAAGCTGTAAACCAAAAAACCGCAGGTTTCAAATACCTGCGGTT
TTTTCTTACACAATAAACAACGCTTCCACATATCCCACACTCCTATCCCGAGACCTTTGC
AAAATTCCCCAAAATCCCCTAAATTCCCACCAAGACATTTAGGGGATTTTCCATGAGCAC
CTTCTTTCAGCAAACCGCACAAGCCATGATTGCCAAACACATCGACCGTTTCCCACTATT
GAAGTTGGATCAGGTAATTGATTGGCAACCGATCGAACAGTACCTGAACCGTCAAAGAAC
CCGTTACCTTCGAGACCACCGCGGCCGTCCCGCCTATCCCCTGCTGTCCATGTTCAAAGC
CGTCCTGCTCGGACAATGGCACAGCCTCTCCGATCCCGAACTCGAACACAGCCTCATCAC
CCGCATCGATTTCAACCTGTTTTGCCGTTTTGACGAACTGAGCATCCCCGATTACAGCAC
CTTATGCCGCTACCGCAACTGGCTGGCGCAAGACGACACCCTGTCCGAACTGTTGGAACT
GATTAACTGCCAACTGACCGAAAAAGGCTTAAAAGTAGAGAAAGCATCCGCCGCCGTCGT
TGATGCCACCATTATTCAGACCGCTGGCAGCAAACAGCGTCAGGCCATAGAAGTCGATGA

# Appendix A

AGAAGGACAAGTCAGCGGCCAAACCACACCGAGTAAGGACAGCGATGCCCGTTGGATCAA
GAAAAACGGCCTCTACAAACTCGGTTACAAACAACATACCCGTACCGATGCGGAAGGCTA
TATCGAGAAACTGCACATTACCCCCGCCAATGCCCATGAGTGCAAACACCTGTCGCCGTT
GTTGGAAGGGTTACCCGAAGGTACGACCGTCTATGCCGACAAAGGCTATGACAGTGCGGA
AAACCGGCAACATCTGGAAGAACATCAGTTGCAGGACGGCATTATGCGCAAAGCCTGCCG
CAACCGCCCGCTGTCGGAAGTGCAAACCAAGCGTAACCGATATTTATCGAAGACCCGTTA
TGTGGTCGAACAAAGCTTCGGTACGCTGCACCGTAAATTCCGCTACGCCCGGGCAGCCTA
TTTCGGACTGATTAAAGTGAGTGTGCAAAGCCATCTGAAGGCGATGTGTTTGAACCTGTT
GAAAGCCGCCAACAGGCTAAGTGCGCCTGTTGCCGCCTAAAAGGCAGCACGGATGCCTGA
TTATCGGGTATCCGGGGAGGATTAAGGGGGCGTTTGGGTAGAATTAGGAGATATTTGGGG
CGAAAACAGCCGAAAACCTGTGTTTGGGTTTCGGCTGTCGGGAGGGAAAGGAATTTTGCA
AAGGTCTCATCCTGTTATTTTCACAAAAACAGAAAACCAAAAACAGCAACCTGAAATTCG
TCATTCCCACGAAAGTGGGAATCCAGTGCGTTGAGTTTCAGCTATTTAGAATAAATTTTG
AAACTCTAATCGCGTCATTCCCACGAAAGTGGGAATCCAGGACGCAAAATCTCAAGAAAC
CGTTTTACCCGATAAGTTTCCGCACCGACAACTCTAGATTCTCGCCTGCGCGGGAATGAC
GAATCCATCCATACGGAAACCTGCATCCCGTCATTCCCACGAACCTGCATCCCGTCATTC
CCACGAAAGTGGGAATCCAGTTTTTTGAGTTTCAGTCATTCCCGATAAATTGCCTTAGCA
TTGAATGTCTAGATTCCCGCCTGCGCGGGAATGACGGATTTGAGATTGCGGCATTTATC
AGGAGCAACAGAAGCCGCTCTGCCGTCATTCCCACGAAAGTGGGAATCCAGTTTTTTGAG
TTTCAGTCATTCCCGATAAATTGCCTTAGCATTGAATGTCTAGATTCCCGCCTGCGCGGG
AATGACGAATCCATCCATACGGAAACCTGCACCACGTCATTCCCACGAACCTACATTCCG
TCATTCCCACGAAAGTGGGAATCCAGTTTTTTGAGTTTCAGTCATTCCCGATAAATTGCC
TTAGCATTGAATGTCTAGATTCCCGCCTGCGCGGGAATGACGAATCCATCCGTACGGAAA
CCTGCATCCCGTCATTCCCACGAACCTACATTCCGTCATTCCCACGAAAGTGGGAATCCA
GTTTTTTGAGTTTCAGTCATTTCCGATAAATTGCCTTAGCATTGAATGTCTAGATTCCCG
CCTGCGCGGGAATGACGAATCCATCCGTACGAAAACCTGCACCACGTCATTCCCACGAAA
GTGGGAATCCAGTTGCTTGAGTTTCAGTCATTTCCGATAAATTGCCTTAGCATTGAATGT
CTAGATTCCCGCCTGCGCGGGAATGACGAATTCATCCGTACGGAAACCTGCACCACGTCA
TTCCCACGAACCTACATTCCGTCATTCCCACGAAAGTGGGAATCCAGTGCGTTGAGTTTC
AGTCATTTCCAATAAATTGCCTTAGTATTGAATGTCTGGATTCCCGCCTGCGCGGGAATG
ACGAATTCATCCGTACGGAAACCTGCATCCCGTCATTCCCACGAAAGTGGGAATCCAGTT
TTTTGAGTTTCAGTCATTCCCGATAAATTGCCTTAGCATTGAATGTCTAGATTCCCGCCT
GCGCGGGAATGACGGCGGAAATCTTGTTTATATTGAATCAAAAAAAACCTGCACCTTAAT
CAGTTGGCGGTTTAGTCCGACTTTTGGGGTGCAGATCAAGCTTTCAGACGGTATTTCCTT
TAAAACTTCATTTCGAGCGCGAGACTGAAGTTCCTGCCCGGTGCGGCATACCTTCCATAG
TTGCTGTCGCCGCCGTGCCGGTTTGCCGTGCTTTCCGCAGTCTGGCGCAAGGATTCCCAA
GTAACGTAGCGGTAGTTGCCGATATTGTAGATAGCCGCCCTCAAGGTCAGCCGTTTTTTC
AGATTCAGATAGGCGGAAACGTCTGCCGTCGACCAAGAAGACGACGCTCTTTTTGTCGAA
TATCGTTTTTGATCGCCTGCCAGATAAGCAAGCTCGTCAGGGTTTTTCCCTTTGGAATAG
GTCAGCATAATGTTTGCGCCCCATTTCCCCTCAGGCTGGTCGTATCCGAACCCCAAAACA
TAACGCGACGGCTGTACCGCATCCAAAGCATAGCTGCGGAGGGACAGTCCCGGCCGGTTG
GATACCGATTTCGGTTTGATGCGGTTGTACGCCAATGTGGTGTACAAACCTTCGGGCAGT
TTGCCATACACGCCGTTCCAGTCGATTTTTCCCAATATATTAACGCCTTGAAGCGACATA
TTTTGGGCATTGTAATAATCGCGTATATCAATCTCTGTCAATTGTCCTGCCTGATTCGGC
AATTTGGTTTTGTGATCGGCAACGGCAATCATATCGGTATAACGGTTGCGGAAGCTGCTG
ATTTCCAAAAAGCCGAAATCGCCCTTCCACTGCAAACCGATTTCCCGGTTGGCTGCCTTT
TCCGATTTCAGGGCGGGACGCTGCCAGCCTTTCGGATAATCGTGATAAATGTCTATCCCG
AAAAGTTCTTGGAATGAGGGCGTTCTGAAGCCGCTGGAGGCACGGTAAGACACGGAAAAA
TGCCGGTTCGGTTTGAACAAGATGCCGCTGTTCCACGAACGGTCAACATACCGCCCGCTG
CGGACGAGTTCTTCCGACGTGGTGAAGTTTTTCCGGTCGTACCTGCCGCCCAAGCTGAAA
TCGAAATATTTGCCGATTGAAAAACGGTCGTTCAAAGAAATATGGATATTGCTGCCGTTG
ATTTTTCTTGGCACGCATTTGCGGGAACGCAGGGTTTCGATGTAGCCGCAGACCGACCCT
TCGACGACTTCGGGCTTACCCAAAAGATACTTATCTTGATTGTTTTCATCGAATCCCGTG
GATTCCGAAATCCTTGCCGCATTGTGGGAAAGCTGTTCGGGGCGGGAAATCGCTTTGGAA
GCATCGTAACCGAAGCCCAAAGTCAGATGGTGTTTCGTCCATTTGTTTTTCAGCGATTTC
TCAAACGAGGCATTCAAAACATTGTGCTGTTCGCGGTAGTGGAAACGGTCGCTGCTGTCG
TAGGAATACGGTTTGTCCGCCGACGCGCGGCAGGATTTGTCCACAGCAGGATACACGGCG
CAATTCAGCTTCAGCGTGTTGTTATCGGTTGCCACGCCCTGTTTGTCAAACGACAACACC
GCCTTATCCGCCCAATTGTCAGAATACGCTTCGTTTTCATAACGATACAGCAAACCCATA
CGGCGGCGGCGGTGATGTTCGTCAATAAATTTGGTGCGGGAATATTTCAAACCTATGCCC
CTGACCAAATTTTTATCGCCCTTCCACTCTTCTATATTCGGCACAAAATACAAGCCGTCG
CGGAAATCGTCGCCGTCGTACACCCCGCTCTTGTCTCTAAACTTTTCCGCCTCGTCCGTA
CCGTAATACTGTTTTTCCGTCATATCGCGGATATCGTAACGCTGTTTGGTATCCTCAAAC
ACGCCGCCGACATAATGCCTGCCGCCGAAGCGGTAGCCCAGCTTGGCAAGCCAAGAGCCG
CTGCGGTAATCCATCGGATCGGGCAATATCCTGCCGCCGCCCGTGTAAGCTTGGGCGGAC
AGATTTTCGTGGCGCGCCTGCGCCTCCCGCACCTGCGCCTCTTCTTCAGCACTTAAAGGC
TGATTTTGTTCAATACGTTCTTTTACCCAGCGGTTGAGCTGGTTGTTCAAATATTTCCCG
TAGCCCGCCAATTTTGCCACGGGCTTGGATTCACGCTCGCCCTCTACTGAGAAAAATGGC
TCTCTTGTCTTGCGTTTAATATCGTATGTCTGACGGAACGCGTCCAAACGGTCTATGCCG
TATTCCACCCCGTCCGCAATATCGCCGTGCGGGCGCGTTTCCCGCCCTTGGCGTTCGGTT
CGGATTAACAGCCCTTCCCAACCGTCTTTGCTGAACCCCGCGCCGAGCGACTTCATAAAT
TGGCGGTTTTTACTGCCGTAGGCGGTTTTTGCCTGTATCCCCCAACTTTTGCCGTCTGAA
ATCAGGTCTGCCGCCTCTTTGGTGCGGAAGGCGACCGCGCCGCCGAGTGCGCCGCTGCCG
TGATCGGACGAACCGGCACCTTTGTCGATTTCCACCGTGCTGATGTTTTCATATTCGATT
TCGTTGATTGCACCGCTGCCGCCGCGTCCGCCGTATCCGCTCAACGATCCCTGCACGGTA

## Appendix A

AACGCCTGTATTTGGGCAACACCGTCGACCGAAACCGCCACACGGTTTTATCCACGCCG
CGTATCGAGTAGCCGCCGCTCGCGCCGTTGCCCTGTTCGACAACCGCCACGCCCGGATCG
TAGCGCGTCAGGTCGCGGATACCGAGTACCTGTTCTTTGTTCAACGTTTCCGACGTTTTG
ACGATTTTGCCCAAACCGGTCGCCTCTTTCGATCGCCGTCCCACTTTGGCGGCACGGACG
GTAATCTCTTTCAGGGATTGGGTCTGCGCGGCATCAGGTGTCGCCCCCCCCGCTTGGGCA
GCATAAGCCGGAAAAGCGGTTGCAATGGCCAAGGCAGTCAGAGTCAGCGGAAAACCGTGT
TTCTTATTCATTTTTCCACCTCCTGCATATCTTTCTTCGCACCGAATACCACGCCGAATT
GGTGTTTAACTTCAGATTCTAACTGTTTGCCAACATCAACTTCAGCATCAACTTCAGCTT
CAACATCAACTTTATTTTCAGTACCTTCAGTTATACCAAGAGATTTCCCATCATTATTGA
AAATAATACCGCCCAATTCCTCCGCCTGCGGGCCGTAAAATCCCCCTTCTACACGAAGAT
TACTAGCTTGGAAGGTTTTGGGGTCGGTCGAACCATTTCCCGAAAGATTGATGCCGTTCT
CCCGAGTGCGTGCTGTCGCGTAGAAACCGTTGCCCTCAATCTTGCCGTTTTCAATATGGA
AAGCAGGTTCTACACCGTTTTCCTCCGTCAGCGTTCCGGAAATCGATTTCTTGCCGAAAT
CAACGGTAAATACTGCTTTTGCCGCTTCTTTATCCGCCTGATTGTCCCATTGAATGGGTT
TGCCGATACGCGCTTCCCAAGTGCCGGTATAGTGTGCTTCTCCAGTTTTCGGAATATCCG
TTTCCGCCGTGCGGATACCTTTCAGGAAAAGGTCGATGTTCCTGCCTTTAGGGGCTTCCG
GAGCGGGCAGGATGCCGTCTGAACCGCTGCCGCCTTCTTCTGTCGGCGATTCTTCTTCGG
GTTCTTCAGCTTCATCTTCACCTTCTACGGCTTCGTCTTCTTCGCTGCCTTCGTCTTTTA
CGGCTGCGTCTTCGGTGCCTTCTTCATCGTCGATTTCGTCTTCGCCTTCTTCGACGCTAT
CAACGCCTGTATCCTCTTCGTCCCTCTCTTCGTCCTGCGCCTTCGGTTTGGCGGCGGGAC
GTTCGGTTTGCATCCGTCCGATTTTCACATAGGTCAGAAAATCGCAGCAGGTTCGGATTG
TCGTTTTCCTACCATCGGCAAGCTCGATGGTTTGTTCTTTGTTTACCAAAGGAATTTCAC
GCCCTTCGACAAGAAGTTTGTCGGGATGACCAAAATCGGGCATAGAGGAAATGGCAAACT
CACGGGGATTTTTATCACTTGCCTCGTCAACGGAAATTTTCAGAGAATCCAAGATTTTGG
TGTGTTTTCCAGACGACAGGGCAGGTTTTGTATCTGCTGCGTTTTCTGTCTCTGTTTTTT
GTTTGCCTGCGAATACGCCGAATACGCTGTTGTCGTTGCTGATAAACCGTCCGGCAAGCT
CTTCTCCGTTATCGCCGAAAAAACCGCCCTCAAGCCGCTGATCGGCATCGGTATGGAAAA
ACAAATATTCTTTATCAGCGTGTTGCGTCTTCACCTCGGTGCTAACTTTGGCACTGCCGG
TAAAGCGGTTGCCGTCCAATGTTGCGGTAATGTCGTAAATGGTCAGCGGTTTTTTGGGCT
CATTTGGATTACTTTTATTTTGCACATACTGATTTTTAATCAGCTTGCCATTCAGGGTTT
TGTTATCAAAATCAACCGTATATTCGGCAGGATGCTTTTCCCTGTCGTCGGCATCCCTAG
CCTCATAAGAAGTTGCCCCAATTTCATTACCATAATATGTGGTATAACCCAAATCCGTAC
TGGAAACCGCCTTACCTGTCCGATGACGTTTGGCATCGGTCATATATTGCCAGTTACCGG
AATATTGCACCGTTCCCGCGCTCGGTAAAGATTGGGAAGGACGTTCTCCGGAATAATATA
CAAAACCGTCATAACTAAATCGGTTAACAAACTCCTTACCATCAGAAGTCTTTTCTTTTT
CATTATCCTTTCCTTCCGCCCTGGTAAACACATAGCCCGCACGGACAAATTGATATTGAT
ATTTTTCTTCTTCTTTTTTCGATGTGATAACCCTCACATCAGAATACCGTTCGTTGATTT
TCTTTTTAAGTTTGTCAGCCTGTTCTTTCAGCGTACCGTCTAAAAACAGGATATCCTTCT
CTTTAAGCGGCAGATGCTCCTCTGCCTGATGCTTGTCGGGAATTTCCGTACCGTCTTGTT
TATAGGAAGCAATATTCCGCCTTGGCAGCCGCATTGCCGCACCGACGGCGGGCCGGTTGA
CCGGCGTGGTTTCTACCGAAGACCCGGCAGGGGGCGGAGTGGGAACGTCCTTAGATTTGA
AGGTGACGGGGTACGCGGTCGGCGTTGATTCGACAACAGGCTGCACGCCGAAATTGCCGC
CGATACAAGATGCTAAAAGTAAGGGCAACAAGACAATGCCGCCATAATTCGGTTTACACA
TCCCTACTTTTCCTCTATTTGATTAATAATAATTATCATTATATTAATATGTACAGATAA
TATCAAGCCGTTTTTATAGTGAATTAACAAAAATCAGGACAAGGCGACGAGCCGCAGACA
GTACAGATACATTCCGTCATTCCCACGAACCTACATCCCGTCATTCCCACGAACCTGCAC
CACGTCATTCCCACGAAAGTGGGAATCCAGTTCGTTCGGTTTCGCTTGTTTTAAGTTTCG
GGTAACTTCTACTTCGTCATTCCCACGAACCTGCATCCCGTCATTCCCACGAAAGTGGGA
ATCCAGGACGCAAAATCTCAAGAAACCGTTTTACCTGATAAGTTTCCGCACTGACAGACC
~~TAGATTCCCGCCTGCGCGGGAATGACGGGATTTGAGATTGCGGCATTTATCGGGAGCAAC~~
AGAAGCCGCTCTGCCGTCATTCCCACGAAAGTGGGAATCCAGTTCGTTCGGTTTCGCTTG
TTTTAAGTTTCGGGTAACTTCCACTTCGTCATTCCCACGAAAGTGGGAATCCAGTTTTTT
GAGTTTCAGTCATTTCCGATAAATTGCCTTAGCATTGAATGTCTAGATTCCCGCCTGCGC
GGGAATGACGGATTTTAGGTTGGGGGCATTTATTGGGAAAAGCAGAAACCGCTCCGCCGT
CATTCCCACGAAAGTGGGAATCCAGTTCGTTCGGTTTCGCTTGTTTTAAGTTTCGGGTAA
CTTCCACTTCGTCATTCCCGCGAACCTACATTCCGTCATTCCCACGAAAGTGGGAATCCA
GTTCGTTCGGTTTCGCTTGTTTTAAGTTTCGGGTAACTTCCACTTCGTCATTCCCACGAA
CCTGCATCCCGTCATTCCCACTAAAGTGGGAATCCAGGACGCAAAATCTCAAGAAACCGT
TTTACCTGATAAGTTTCCGCACTGACAGACCTAGATTCCCGCCTTATATGATGCGCTCTA
TCAAAGGGGCGCATTAATTTTCTTAACATTCCCCTTTGACAGCCAAGTGAAAGGGGCTTT
TTTATGTCAGCAGTAAATGTAATATTTTCCTGTTCTTATTGGAGAATATTTAAAAAATCA
GATTCTTGTGTTTTGTGTTTTTATCAGTTCAGACATGGCGAACCGCATAAACTCATTAAT
CAAGAGAATTTTTCAAAGCTTTATCAGGCGTTCGATTATATAGATTCGGTTGGTTCGAAT
TTTCCAGTGATTATCACAACGGATGGTTGTGGTCTTTTTTGTTGATCTTTAAAAGTTTGT
CAGGATTTGGCTTTCGGTCGTTGACCGTCGTACGCGCTTTAGCGCGGAAGACGGGAAACG
GCTGAAAGCCCCCCCCTTGACTAACAGGGGGGGAGCGAAATTAAAAACCAATTCCAAGAG
TAGTGAACGAATGAGTGAAGTTGAATATTTCTCACACTTTATATCGGACGGAAAAGGGAA
GCTTTTAGAAATTCCGCAGCGAAGAGGTAAGCAAGACGGGGTTTTTGTTGATTGGATTTC
ATTCACATTCCATGAAGATACTTTACTGAAAGTTTCCGGTTGCCCTTTATTTTCTGATGC
TGAATACATGTATGTATTAAGCAGAAAGCTGGAAGAAATTCTAGGTTTTGGCATAACGCG
CAAATGCAAATCAAGGGGCAACAAATTCTATGAATCCATGTATAGGTTAGGTTCGGATGA
TGTTGATTATGGAGAGGTGCATTTCGGAGGTCAGCGCAATACTGTTTTAGTTGAGTTGAA
AGGTACTGGTTGCAGCGTTGCAAGTCCGGGTTGGAGTTGAGGCTAAAGCAGTTTCTCGA
TGATTCGATAAGGACAAGAATAACGCGAATTGACCTAGCACTTGATTTTTTTGATGGAGA
GTACACGCCGGATCAGGCGTTGTTAGATCACGATAATGGTTTTTTTGATAACAGCAATCA

## Appendix A

AAGGCCGAAATCTGAAACGATCGGTACGGCTTGGCGGAATGAGGACGGGAGCGGCAAGAC
ATTTTATGTAGGTCGCAAGAAAAATTCTCGTTTTGTTCGTGTTTATGAGAAAGGCAGGCA
GCTTGGAGATAAAGAAAGCAAATGGGTAAGGTTCGAGATCCAGTTTAATTATGGAGATAT
AGAAATACCCTTGGATATTTTAATAAATCAGGGTTCGTATTTCTGTGGAGCTTTTCCAAT
TTGTAGAAAATTTAAAAATATGCCGGTTCCCGAAAGGTTTGATCAGAGAAAGAAAAAGCT
TAATTTAACTTTCGAGCATAAATTGCATTACGCGAAAAACGCGGTTGGAAAACTGGTCAA
TTTCATGATTGAAATGGGTTTTGATAATAGCGAAATTGTGGAATCTTTAAAGGCAGATTC
GGGATTTCCCAAAGGATTAGAACCTGAAAAATATGCTCTGGAAATGTTAAGGGACGGTTT
GAAACACGGTTTTATTCATGAACAGCCGGATATTGATTTGGAAATTGAACTTGATGAATT
GGGGGTTATTGCTTTTAAAAATTCTGACAAATTCGATAGGGAAAAAAGGCTTTTTAGTCC
TGATTATGATGTCGAGAAAGAAAGGAAATATCAGGAATATTTAAGTAAAGTTTATCATCA
AAATGTAGATTATGATTATTTTTAAAGGAAATCAAAATGTTTAATCAAACTCAAACTGTA
ACTTATCCTGCAACTTTTTTGGGAGCCAAAAAATTCAAAGGCGAAATTGATGGCTCTAAT
ATCGACACTTGTTCCGTATTGGTTGCAACACCTTTGCCGGCACAGTCGGGAAATGCTGTT
GGATTCACGGCAGCACAAATGAAGTTCGGGGACAGTAAGAATTTCTCAAAATTAGAGAAT
CTCAAATACCCGTGCGAAGTTATGGTAACGGTTGAAATGACTTCGACAGGTAAGGGCATG
GTTCCTTCATTAATTGATTTTCAGGTGGCAGAAAAGCCGAAAGGTTGATTTATGAAATTT
GAAGAACGTTTCATAGTTCAAGACTTGGAAACGCATGACTTTATTTATCCCGATCCTTTC
GGTGATGTGGGGTTTACTCAAAATATTAAATCAGCAGGTCAATTTGAAAGCTACGAAGAT
GCGTTGAATTCAGGCATAAATGAAATAGGCGGAGGATTCCAGATATTTCAGTTCTTCGTA
AAATCGGAATAAAAGAAAAACAGGCTCGGCGGGCGGTCTGTCAACCTTTCACAAAGCCCG
CAACAAAGGAAAAATATCATGAAAATGAACCTTGCAACACTAATTATCGGCTGGGTGGTC
TGTATGTTTCTTTTTCTTTTCGCAATCCTCTATTTTATCGGCTAAAAACGAGATTCGGAA
AAGACTTCGTCCGGATGAAGCAAGTCAAGAAGTCGTCTTATTTTAAATATCAAAAAAGGA
AAAAAACGATGAACATCGTTAAAAAATACGCTGTAAAAGCAGCCTTGGCAGCCGGTATCT
TCACACCGGCCATTGTTATGGCAGATACCTTTGATCCATCCGCGATTGGTACGCAAGTAG
CGAATGTAATCATGGGTTTCGTGTCAATGGTTTCCGCCGTGGGTATGGCGGCCATTACCG
TGATTCTTGCAATCCAAGGCTTCAAAATGGCTTGGAGCATGATTAAATCTGTCAAATAAA
CAGAGTGAAGAAAAAGGGGCGTATAAATGGGCTATCGTGTCGGCATAAATTGTTTTGATA
CAAGATTGCAGGCAGACGACTATTTATTGTCGTCCCTTCCTCCTACTGTTACCCAGGACG
GAAAAATCATCAGGCCGGAAAGGGTGGGCGATAAATGGATTTTGAACGGAAAGCCGGTTA
CGTTGTCTTATCCGGAATGTTCCAATTTTGAGCAGATAAAGCAAGGTTCTTATGTCGGTT
CGACGGTTCTAATTCTGTTTGTAGTCATTTACGGTTTCAGGCTTCTGATTAATTTTTTAA
AAGACATAGGCAAGGTTGGGACTGATTGATGATTATAGATTTCTGGTTTCTTCTCGGTTT
CTTCTTGGCTTTGTCTGTTGCTTGGCTGTTTTGGTAACGGTTGGTAGAATCGGCTTTTTA
GAGTGTTTTAAAAGGTCCGAATTATGTTTATTTCTGAATATCATTTAGTTAAATTTCAAA
CTGATTCACATATTTATAGAGATTTACCACAAGCGTTAATTTATTATAGGGAATTGATTA
GAAAAGGGGTTTTTAAAACTTCGTTTTCATTTGATATTTTTAGGAATTTCTTTCATCGTT
ATGATAGAGATTTTATAGAAATTCAATTCCCTGATTCTTCTACATTATTAATTAAATTAG
ATGAAGCAAAATGTTATGTTTATTATCCTAGGGCGAAATTTTTTAAAGATTATCCTATGC
TTTAGTTTTTTTGTATCTAAATTTGCATTGGCATCAGTAAATGCTCCGGGTAAATTTGAT
AGGGTTGAAGTTTATGATGATGGCAGATATTTAGGTATTCGAGGTTCAGATGACAAAAGA
AGAAGAATTTGGAAAGGTGTATTTGATAGAGAATCGGGAAGATATTTAACTTCAGAAGCT
CAAGATTTAAAAGTTAGGCATGTATCTACTGGAGCATCAAGTACGGGTAAAGTTAGTTCG
GTTGTATCTTCATCAGTTTCCCGCGCTGGCGTATTGGCGGGGGTCGGCAAACTTGCCCGC
TTAGGCGCGAAATTAAGCACAAGGGCAGTTCCTTATGTCGGAACAGCCCTTTTAGCCCAT
GACGTATACGAAACTTTCAAAGAAGACATACAGGCACAAGGCTACCAATACGACCCCGAA
ACCGACAAATTTGTAAAAGGCTACGAATATAGTAATTGCCTTTGGTACGAAGACAAAAGA
CGTATTAATAGAACCTATGGCTGCTACGGCGTTGACAGTTCGATTATGCGCCTTATGTCC
GATGACAGCAGATTCCCCGAAGTCAAAGAATTGATGGAAAGCCAAATGTATAGGCTGGCA
CGTCCGTTTTGGAATTGGCATAAAGAAGAACTGAATAAATTAAGTTCTTTGGATTGGAAT
AATTTTGTTTTAAATAGTTGCACATTTGATTGGAACGGCGGAGATTGTGTGGTCAATAAA
GGTGATGATTTCAGAAATGGGGCTGATTTTTCCCTTATTCGCAATTCAAAATACAAAGAA
GAAATGGATGCCAAAAAGCTGGAAGAGATTTTATCGTTGAAAGTCGATGCCAATCCCGAC
AAATACATAAAGGCAACCGGTTATCCCGGTTATTCCGAAAAAGTAGAAGTCGCACCCGGA
ACAAAAGTGAATATGGGTCCCGTCACGGACAGGAACGGGAATCCCGTTCAGGTTGTCGCA
ACATTCGGCAGGGATTCGCAAGGCAACACCACGGTGGATGTTCAAGTAATCCCGCGTCCC
GACTTGACCCCCGGAAGCGCGGAAGCACCGAACGCACAGCCGCTGCCCGAAGTATCGCCC
GCCGAAAACCCCGCAAACAACCCGAACCCCAATGAGAACCCCGGCACGAGCCCCAATCCC
GAACCCGACCCCGATTTGAATCCCGATGCAAATCCCGATACGGACGGACAGCCCGGCACA
AGACCCGATTCCCCCGCCGTTCCGGGACGCACAAACGGCAGGGACGGCAAAGACGGAAAG
GACGGCAAAGATGGCGGCCTTTTGTGCAAATTCTTCCCCGACATTCTCGCTTGCGACAGG
CTGCCCGAGTCCAATCCGGCAGAAGATTTAAATCTGCCGTCTGAAACCGTCAATGTAGAG
TTTCAGAAATCAGGAATCTTTCAAGATTCCGCACAGTGTCCCGCACCTGTCACTTTCACA
GTGACTGTGCTTGATTCAAGCAGGCAGTTCGCGTTCAGCTTTGAGAACGCATGTACCATA
GCCGAACGGCTAAGGTACATGCTTCTCGCCCTTGCTTGGGCGGTTGCCGCCTTTTTTTGT
ATCCGCACAGTATCTCGTGAAGTCTAGCAGGCGCAGCACCGCCGGGCTTCAGTAACTTGT
ACCAAGGCAGGGGGAGGACGTCCAGAAAGATTTGTAAAGACGGCTTTATCGTCTTTATAA
ATCTTTTTGGATACCCCTTGCCGCCCCGCCAAAAGAACACATTCTGCCGCAAGGGCAGGT
GGTAAGGCGCGCGCCTTTTGCGCCGTTCCCCCTGCCCCCGCGGCGTCGCAAGTGAGACTG
GGGGTGCGGGGGCTAGTCCCCGCAAAGCCTTTCAGCTTCGGAAGCCACGGCCGAAAGGCA
GGCGCAGCACTGCCGGTCTGAGCGGAAGCCAGGCTACAGGCAGGCGCAGCACCGCCGAGC
TAGGCGGAAGCCAGGCTACAGGCAGGCGAAGCACCGCCGGTTGGGCGGAAGCCACGGCCG
AAAGGCAGGGCGAAGCACCGCCAGGCTTAGGCGGAAGCCACGGCCGAAAGGCAGGCGAAG
TACCGCCGGTCTGGGCGGAAGCCATGGTAAAAGGCAGGCGAAGCACCGCCGGGCTTCAGT

# Appendix A

AACCTTTGTTCAGGCAGGGGGAGGATGTCCGTAAAGAATCGTAAAGGCGGGGTTTTTTCG
CCTTTATGATTCTTTTTGGATACCCCTTGCCGCCCCGCCAAAAGAACACATTCTGCCGCA
AGGGCAGGTGGTAAGGCGCGCGCCTTTTGCGCCGTCCCCATGCCCCCGCGGCGTCGCAAG
TGAGACTAGGGGGTGTGGGGGACTAGTCCCCCGCAAAGCGTTCAGCTTCGGAAACTTTGG
CCGAAAGGCAGGCGAAGCAGCGCACTTTGCGACGAATGTCGCAAATAGCCGAGAAGCGCG
GGGGGATTGGCGATAAGCGCGAGGGGGGTGTCCCCACAGCGCCGCCGCGCCGCGAATGCG
GCGCAAAATCTTTCAGATTAAGAAACATTTGTTTAATGAGGCAACCGTGCCTTTTAAGAA
AGGGATAGCAAATGAAATTGTTGGCCGCATTGATTCCGCTTTTGATGAGCGTGGCAGGCC
GTATATTGACTGCATTAGGCTTGATGGCGGTAACCTATTCAGGGGTGGATAGATTGGTAG
CCCATTTTCAGCAGGCGATAACCAATAGCATAACGGGCGCGCCTCAAGCGATGTTGCAGC
TTTTTTATATAAGCGGCGGTGGAACCGTTCTTAATATCCTGTTTGGCGCGATCGCCTTTA
TTCTGTCATTCAAACAAATGACAAAACTAGCAACCTCAATCGGGAAGAAAAAATAAATGG
CAGAGATCTGTTTGATAACCGGCACGCCCGGTTCAGGGAAAACATTAAAAATGGTTTCCA
TGATGGCGAATGATGAAATGTTTAAGCCTGATGAAAACGGCATACGCCGTAAAGTATTTA
CGAACATAAAAGGCTTGAAAATACCGCACACCTACATAGAAACGGACGCAAAAAAGCTGC
CGAAATCGACAGATGAGCAGCTTTCGGCGCATGATATGTACGAATGGATAAAGAAGCCCG
AAAATATCGGGTCTATTGTCATTGTAGATGAAGCTCAAGACGTATGGCCGGCACGCTCGG
CAGGTTCAAAAATCCCTGAAAATGTCCAATGGCTGAATACGCACAGACATCAGGGCATTG
ATATATTTGTTTTGACTCAAGGTCCTAAGCTTCTAGATCAAAATCTTAGAACGCTTGTAC
GGAAACATTACCACATCGCTTCAAACAAGATGGGTATGCGTACGCTTTTAGAATGGAAAA
TATGCGCGGACGATCCCGTAAAAATGGCATCAAGCGCATTCTCCAGTATCTATACACTGG
ATAAAAAAGTTTATGACTTGTACGAATCAGCGGAAGTTCATACCGTAAATAAGGTCAAGC
GGTCAAAGTGGTTTTACACTCTGCCAGTAATAGTATTGCTGATTCCCGTGTTTGTCGGCC
TGTCCTATAAAATGTTGAGCAGTTACGGAAAAAAACAGGAAGAACCCGCAGCACAAGAAT
CGGCGGCAACAGAACAGCAGGCAGTACTTCCGGATAAAACAGAAGGCGAGCCGGTAAATA
ACGGCAACCTTACCGCAGATATGTTTGTTCCGACATTGTCCGAAAAACCCGAAAGCAAGC
CGATTTATAACGGTGTAAGGCAGGTAAGAACCTTTGAATATATAGCAGGCTGTATAGAAG
GCGGAAGAACCGGATGCGCCTGCTATTCGCATCAAGGGACGGCATTGAAAGAAGTGACGG
AGTTGATGTGCAAGGACTATGTAAAAAACGGCTTGCCGTTTAACCCATACAAAGAAGAAA
GCCAAGGGCAGGAAGTTCAGCAAAGCGCGCAGCAACATTCGGACAGGGCGCAAGTTGCCA
CATTGGGCGGAAAACCGTAGCAGAACCTAATGTACGATAATTGGGAAGAACGCGGGAAAC
CGTTTGAAGGAATCGGCGGGGGCGTGGTCGGATCGGCAAACTGAAGAAAACGGCAAGAGA
GAAAAAAGACCCGTAAACCGTTTGAATATAGACGGTTTACGGGTCTTTGTTTCGCGCAAA
GCAAGGGCTAAGGCAGTCAGGCAGCAAATCCCGCAATGTATTAAAACAGACGCGTAGAAA
TGCCGGCTGCCTTTATCCATCCTCGAAATTGAATATCATCCTAGCCGTATCAAGGCTGTA
TAAATAAGGAAAATACCAATGAATATAATCGGGCTGGACATCTCAAAGGACACCATAGAC
GCAACATTGCATAAAACAAACGGAAGTATCCATTACATTAAATTTAAGAATAATGATGAT
GGATTAAAACAGTTTAGATTGTGGATAAAGGGAAACAGAATCAGAAAAGTCTATATCGGC
ATGGAGGCAACAGGCATCTATTACGAAAAGGCAGCAGATATGCTTTCTTCCTACTATACT
GTTTACGTTATTAATCCCTTAAAAATCAAGGACTACGGAAAAAGCAGGTTTAACCGTACC
AAAACCGACAAAGCAGATTCAAACCTGATAGCAGACTACATAAAAAGGCATCAAGATACA
TTGATACCGTATCAGATACCCAAAAACAAAGCACTGCAAAAACTGATTAACCTTAAAAAT
CAATTACATCAACATCAGAAGCAAATTAAAAACCGTCTTCATAGCACTGAAGAAGACTTC
ATAAGGAACATACATCAAGACTTGATAGATACCATACAGGACAAGATGGAACAGGTAAAA
ATAGCCATATCCGAACAAATCAAAAAACAAACGGACAATAACCATTACCGCAATCTTCAA
ACCATCCCGAGCATAGGCAAAGACACCGCATCAGTTCTTTATGCGCAACTGACAGAAAAA
CATTTTAAAACCGCAAACCAGTTTGTATCCTATGCCGGATTAAATCCCGCCATCATACAA
TCAGGGACAAGCGTAAGAGGTCGGGGCAGATTGAGCCGATACGGAAACAGACGATTAAAA
AGTACGCTGTATATGCCCGCCCTTTGTGCTTACCGTTTTAACGCATTTCCGAAATTAATA
~~AATAATCTGAAAAAAGCGGGTAAGCCAAAGATGGTAATCATCGTTGCCATCATGCGCAAA~~
CTGGCGAAGCTCGCCTATTACATTGTTAAAACCGGCCAGCCTTACGATGCGGAAAGACAC
CGATTGAATCAATAAAATTCAACAAAATTAAACGGTTACGCGAATATATTTGTGTAACCG
TGCATTTGCATATCGTAAATAAACGTAAATAAAAATAACAATATAAATCAGTATATTGCA
ACTTTGTTTTTTATTTTGTGTTGACGGGCAACATATCATCTGCGCGGGAATGACGGGATT
TGAGATTGCGGCATTTATCGGGAGCAACAGAAGCCGCTCCGCCGTCATTCCCACGAAAGT
GGGAATCTAGTTCGTTCGGTTTCGCTTGTTTTAAGTTTCGGGTAACTTCCACTTCGTCAT
TCCCACGAAAGTGGGAATCCAGTTTTTTGAGTTTCAGTCATTCCCGATAAATTGTCTTAG
CATTGAATGTCTAGATTCCCGCCTGCGCGGGAATGACGAATCCATCCATACGGAAACCTG
CATCCCGTCATTCCCACGAACCTACATTCCGTCATTCCCACGAAAGTGGGAATCCAGTTT
TTTGAGTTTCAGTCATTCCCGATAAATTGCCTTAGCATTGAATGTCTAGATTCCCGCCTG
CGCGGGAATGACGGGATTTTAAGTTGGGGTCATTTATTGGAAAAAGCAGAAACCGCTCCG
CCGTCATTCCCACGAAAGTGGGAATCCAGTTTTTTGAGTTTCAGTCATTTCCGATAAATT
GCCTTAGCATTGAATGTCTAGATTCCCGCCTGAGCGGGAATGACGAATCCATCCGTACGG
AAACCTGCACCACGTCATTCCCACGAACCTGCATCCCGTCATTCCCACGAAAGCGGGAAT
CCAGTTCGTTCGGTTTCGCTTGTTTTAAGTTTCGGGTAACTTCTACTTCGTCATTCCCGC
GCAGGCGGGAATCCAGTGCGTTGAGTTTCAGCTATTTAGAATAAATTTTGAAACTCTAAT
CGCGTCATTCCCACGAAAGTGGGAATCCAGTTTTTTGAGTTTCAGTCATTTCCGATAAAT
TGCCTTAGCATTGAATGTCTAGATTCCCGCCTGCGCGGGAATGACGAATCCATCCATACG
GAAACCTGCACCACGTCATTCCCACGAAAGTGGGAATCTAGTTCGTTCGGTTTCGCTTGT
TTTAAGTTTCGGGTAACTTCCACTTCGTCATTCCCACGAAAGTGGGAATCCAGTTTTTTG
AGTTTCAGTCATTCCCGATAAATTGTCTTAGCATTGAATGTCTAGATTCCCGCCTGCGCG
GGAATGACGAATCCATCCATACGGAAACCTGCATCCCGTCATTCCCACGAAAGTGGGAAT
CCAGCTTTTTGAGTTTCAGTCATTTCCGATAAATTGCCTTAGCATTGAATGTCTAGATTC
CCGCCTGCGCGGGAATGACGGATTTTAGGTTGGGGGCATTTATTGGGAAAAGCAGAAACC
GCTCCGCCGTCATTCCCACGAAAGTGGGAATCCAGTTCGTTCGGTTTCGCTTGTTTTAAG

## Appendix A

TTTCGGGTAACTTCCACTTCGTCATTCCCGCGCAGGCGGGAATCCAGTGCGTTGAGTTTC
AGCTATTTAGAATAAATTTTGAAACTCTAATCGCGTCATTCCCACGAAAGTGGGAATCCA
GCTTTTTGAGTTTCAGTCATTCCCGATAAATTGCCTTAGCATTGAATGTCTAGATTCCCG
CCTGCGCGGGAATGACGAATCCATCCATACGGAAACCTGCACCACGTCATTCCCACGAAC
CTGCATCCCGTCATTCCCACGAAAGTGGGAATCTAGTTCGTTCGGTTTCGCTTGTTTTAA
GTTTCGGGTAACTTCCACTTCGTCATTCCCGCGCAGGCGGGAATCCAGTTTCTTGAGTTT
CAGTCATTTCCGATAAATTGCCTTAGCATTGAATGTCTAGATTCCCGCCTGCGCGGGAAT
CCAGTGCGTTGAGTTTCAGCTATTTAGAATAAATTTTGAAACTCTAATCGCGTCATTCCC
ACGAAAGTGGGAATCCAGTTTTTTGAGTTTCAGTCATTCCCGATAAATTGCCTTAGCATT
GAATGTCTAGATTCCCGCCTGCGCGGGAATGACGGCGGAGCGGTTTCTGTTTTTTCCGGT
AAATACCCACAAGCTAAAATCCCGTTATTTTCACAAAAACAGAAAACCAAAAACAGAAAC
CTGAAATTCGTCATTCCCACGAACCTACATCCCGTCATTCCCACGAAAGTGGGAATCCAG
TTTTTTGAGTTTCAGTCATTTCCGATAAATTGCCTCAGCATTGAATGTCTGGATTCCCGC
CTGCGCGGGAATGACGGCGGAGCGGTTTCTATTTTTTCCGGTAAATACCCACAAGCTAAA
ATCCTGTTATTTTCACAAAAACAGAAAACCAAAAACAGAAACCTGAAATTCGTCATTCCC
GCGCAGGCGGGAATCTGGTTCGTTCGGTTTCGCTTGTTTTAAGTTTCGGGTAACTTCCAC
TTCGTCATTCCCGCGCAGGCGGGAATCCAGTGCGTTGAGTTTCAGCTATTTAGAATAAAT
TTTGAAACTCTAATCCCGTCATTCCCACGAAAGTGGGAATCCAGTTTTTTGAGTTTCAGT
CATTCCCGATAAATTGCCTTAGCATTGAATGTCTAGATTCCCGCCTGCGCGGGAATGACG
GCTGCAGATGCCCGACTGTCTTTATAGTGGATTAACAAAAATCAGGACAAGGCGACGAAG
CCGCAGACAGTACAAATAGTACGGAACCGATTCACTTGGTGCTTCAGCACCTTAGAGAAT
CGTTCTCTTTGAGCTAAGGCGAGGCAACGCCGTACTGGTTTTTGTTAATCCACTATACTG
TAATCAGGGATGCTCAGTTCGTCGAAACGGCAAAACAGGTTGAAGTCGATGCGGGTGATG
AGGCTGTGTTCGAGTTCGGGATCGGAGAGGCTGTGCCATTGTCCGAGCAGGACGGCTTTG
AACATGGACAGCAGGGGATAGGCAGGACGGCCGCGGTGGTCTCTAAGGTAACGGGTTTTT
TGACGGTTCAGGTATTGTTCGATCAGCTGCCAATCAATCACCCGGTCCAACTTCAATAGC
GGGAAGCGGTCGATGTGTTTGGCAATCATGGCTTGGGCGGTTTGCTGGAAGAAGGTGCTC
ATGAGAAATCTCCTAAATGTCTTGGTGGGAATTTAGGGGATTTTGGGGAATTTTGCAAAG
GTCTCAACTTGAGTTTCACGCCCCGCTTAACAATATTCAGTTGGTAAATATTAGATAAAA
CCATAAAAATTAAATTGATGGCTTTTATAATCCCCGATTTGCGAAAATGCCGTCTGAAAG
TCTTCATTCAGGCTTTCAGACGGCATTTTGATCATCAAGTAACGCTTTATCAGGCTTTTT
TATTGTTCAACGCAGCTTTGACAAACGCGGTGAACAAAGGATGCCCTTTGCGCGGATTGG
AGGTAAACTCGGGGTGGAACTGGCAGGCGAAGAACCAAGGATGGTTCGGCAGTTCGATGG
TTTCGACCAAGCGTTCGCGTCCGGCAGATACACCGCCGATGACCAAACCTGCCTGTTCCA
GTGTAGGAACGTAGTTGTTGTTGACTTCGTAGCGGTGGCGGTGGCGTTCGCGGATATGTC
CGCTGCCGTAGATTTTGGCGGCGAGGCTGCCTGCTTTCAATTCGACTTCTTGCGCGCCCA
AACGCATCGTGCCGCCCAAATCGGTGGATTCGTCGCGGGTTTCGACGCTGCCGTCGGCAG
TTTGCCATTCGTCAATCAGGGCAACGACTGGCGCGGCGCATTTGAGGTCGAACTCGGTGG
AATTCGCGCCTTTCAAGCCTGCCACGTCGCGGGCGTATTCGATCAGCGCAATCTGCATAC
CGAGGCAGATGCCCAAGTATGGCACGTTGTTTTCGCGGGCGTAGCGCACGGCGGCGATTT
TGCCTTCCACACCGCGCGAACCGAAACCGCCGGGAACGAGGATGGCGTCCATGTCTTTAA
GCATGGAAACGTCGCCCTTGTTTTTCTCGATGTTTTCGCTGTCGACAAAGGTAATCTGCA
CGTCGGTTTCGGTGTGAATGCCTGCGTGTTTCAAGGCTTCGATCAGCGATTTGTAGGACT
CGGTCAAATCGACGTATTTGCCGACCATGGCGATTTTGACGGTGTGTTTCGGGTTTTGGA
TGGCGTGGACGATTTTTTTCCACGCGGTCAAATCCGCCTGCTGCACATTAAGCTGCAACT
GCTCGGTAATGATGTTGTCGATGCCTTGGTCGTGCAGCATTTCGGGGCATTCGTAGATGC
TGTCCACATCGTAGCTGCCGACAATCGCGCGTTCTTCCACGTTGCAGAACAAGGCGATTT
TGCGGCGTTCGTCCGCAGGCATTGTCCTGTCCATACGGCAAATCAGGATGTCGGGTTGCA
AACCGATGCTCAACATTTCTTTAACGGTGTGCTGGGTCGGCTTGGTTTTGATTTCGCCTG
CGGCGGCGATGTAGGGGACGTAGCTCAAGTGGGCAAACAAGGTGTTGTTGCGCCCCAACT
GGCTTCGCATCTGGCGGATGGCTTCCAAAAACGGCAGCGATTCGATGTCGCCGACCGTGC
CGCCAATTTCGACAATCGCCACATCGTAACCTGCCGCGCCTTCGTGGATGCGTCGTTTGA
TTTCGTCGGTAATGTGCGGAATGACTTGAACCGTACCGCCGAGGTAGTCGCCCCGTCGTT
CTTTGGCGATAACGTTTTCGTACACCTGTCCCGTGCTGAAGCTGTTGCGGCGGGTCATCG
TGGAATCGATAAAGCGTTCGTAGTGTCCCAAGTCGAGGTCGGTTTCCGCGCCGTCGTCGG
TTACGAACACTTCGCCGTGTTGGAACGGGCTCATCGTGCCGGGATCGACGTTGATATAAG
GATCGAGCTTGAGCATGGTAACGTTCAAGCCGCGCGATTCGAGGATGGCGGCAATAGAAG
CGGCGGCGATACCTTTACCCAGTGAGGAGACAACGCCGCCGGTGACGAAAATGAATTTGG
TCATAATGAAATACCCGTATTGGAATGCGTGATTTTAACGTGAAGCGCGCGGTTCTGGCA
AACGGACGGATGCCGTCTGAACGATGGACGGCTGTTTTCAGACGGCATCTTTTCTTTATT
TCCCGGTACTTTGCCGCAACTCGCGGCGCAGGATTTTGCCGACGTTGGACTTGGGCAACT
CGTCGCGGAATTCGATATTTTTCGGTACTTTATATGCGGTTAATTCGGTGCGGCAAAAAG
CGATAAGTTCTTCTTTGGTCAAAGACGGGTCTTTTTTGACGACGAATACTTTGAGTGCCT
CGCCGGTTTTTTCGTCGGGAACGCCGATACAGGCGACTTCCATGACTTTGCCGTGATGCG
CGATGACTTCCTCGATTTCGTTCGGATAAACATTGAATCCGGAAACAACGACGAGGTCTT
TCTTACGATCGACCAGCTTCAACCAGCCTTTTTCGTCCATGACGGCAATATCGCCGGTTT
CCAAGAAGCCGCGCGCGTCTATGGCTTTGGCGGTTTCTTCGGGGCGGTTCCAGTAGCCTT
GCATCACTTGAGGGCCTTTTACCCACAATTCGCCCGGCTGCCCGACGGGGACTTCTTTGC
CGTTTGCGTCGCGCAGTTCGACTTCGGTGGACGAGACGGGCAAACCGATGCTGCCGCTGT
ATGATTCGATGTTTAAGGGGTTGCAGCACACGCCGGGGCTGGCTTCGGTCAGACCGTAGG
CTTCGACGATGGGCGTGCCGGTGATTTTTTTCCATTTTTCGGCAACGGCTTTTTGGGTCG
CCATACCGCCGCCCAAAGTCAGCCGCAATTCTGAAAAATCGACTTCGGCAAAATCAGGAC
GGTTAACCATCGCGTTAAACAGCGTGTTCACGCCGATAAATACATTAACCCGCTGTTTTT
TCAGTTCTCCGATAAAGCCTTTCATATCGCGCGGGTTGGTAATCAGGATGATTTTCGAGC
CGGCATTGGCAAAAATCATCAGATTCACGGTTAAGGCAAAAATATGGTACAGCGGCAAGG

## Appendix A

CGGCGATAACGGTTTCTTTGCCCTCGCGCAACTGGTTTTTAATCCATTCTTTTGCCTGAA
GCATATTGGCGCAGATGTTGCCGTGACTCAGCACCGCCCCTTTGGCAACACCTGTCGTGC
CGCCCGTGTATTGCAACAGCGCGGTATCTTCGCGGTTTAATGCGACAGGTTGGAAAACGT
GCTTCGCCCCTTCTTTCAATGCCGTCTGAAAGGAAACGGTTTCCCGAATACGGTATTCGG
GCACCATTTTCTTGATTTTCCGGATGACGAAATTGATCAGCGAACCTTTAAGCAGCCCGA
ACATTTCGCCGACGGAGGCTACGATGACGTGTTTGATCTGCGTGCGCGGCAGCACCAGCT
CCAGCGTGTTGGCGAAATTTTCCAAAACGATGATGGCGGTCGCGCCGCTGTCTTTCAACT
GATGCTCCAGCTCGCGCGGGGTATAGAGCGGATTGGTGTTCACCGCTACCAAACCTGCCT
GCAAAATGCCGAAAAGGGCAACCGGATATTGCAGTACATTGGGCAACATTATTGCCACGC
GCTCTCCTCGAGGCAATTTAAGGACGTTTTGCAGATAAGAAGCAAAATCTGTTGCCAGTT
TGCCGGTTTCGGCATAAGTCAGCGTCTTACCCATGTTTTGAAAAGCAGGTTGGTCGGCAA
ATTTTTCCACGCTTTGGCGGAATACGTCGCTGACGGAATTGTATTGCGTGATGTCGATTT
CGGCACTGACGCCCTTCTCGTAGCTGTCTAACCAGATTTTTTCCATAGGTATCGGTCTTT
AAAGTGGAATTGAGCGGAACAATGCCGTCTGAAAACCGTTTCAGACGGCATTACCTTTAT
CGTGTGATGATGACGGGTTTGTCGGTCGTTTGGATGATACCGCCGCCCAAACAGATATCG
CCGTCGTACAGCACGGCGGACTGACCCGGCGTAACCGCCCATTGCGGTTCGTCAAACACC
AGCTCGGCGGTTTCATCATCCAAATAGCGCAACTCACAAGGCGCGTCCGCCATACGGTAA
CGCGTTTTGCAGGTATAGCGTCCTGCCTTCGGGCGTTCGGGCAGCGTGAAACTCAAATCG
TTCATCACAAGGCTGCGGGTATAAAGCAGCGGATGGTCGTGTCCTTGCACGACAATCAGT
TCGTTTTTCGTCAAATCTTTAGCCGCAACAAACCACGGTTCGCCCGCGCCGCCAATGCCC
AAACCTTTGCGCTGTCCGAGCGTGTAGAACATCAGCCCGACGTGTTCGCCGACGGTTTTC
CCTTCGGGCGTAACCATTTTACCATTGTCGGTCGGCAGGTATTTCTGCAGAAACTCGCGA
AACGGGCGTTCGCCGATGAAACAGATGCCCGTGCTGTCTTTTTTAGCGGCGGTCGGCAGT
TTGAACTCGGCGGCAAGGCGGCGCACTTCGGGTTTTTCCAAACCGCCCAACGGAAAAATC
GCGCGCTCGAGTTGGAAAGGCTTGAGGCGGTAGAGGAAATAGCTTTGGTCTTTGTTTCGA
TCCAAACCTTTGAGCAGGTAATGCACGCCGTTGCGAACTTCTTTGCGCGCATAGTGGCCG
GTGGCGATGGTATCCGCGCCCTGCCCTACGGCGTAGTCCAAAAAGCATTTGAATTTGATT
TCGGCGTTGCACAACACATCCGGATTCGGCGTGCGCCCCGCACTGTATTCCTGAAGAAAA
TAAGCAAAGACTTTGTCTTTATATTGCGCGGCGAAATTAACGATGTCGATATCGATGCCG
ATAATATCGGCAACGGCGATGGCATCGAACGAATCCTGTTTGATGCTGCAATATTCGTCG
TTGTCGTCGTCTTCCCAGTTCTGCATGAACACACCGCGCACTTGATAACCCTGCTGCTTG
AGCAGGGCGGCGGTTACGGAAGAATCGACACCGCCGGAGAGCCCGACGATGATATTGGAA
GGGTTTGCTGTCGTATTCATGCGTAGAATATGGTTGGAAACGGCGGTTTTTAAAGGCGGA
TTTTAACACATTTTAAAGGCGGGCATAAAAATGCCGTCTGAAAGCCCGGGCTTTTTCAGA
CGGCATTTCAAACATTTTCAGCAGATTAGTGCTGATGCGCTTCGCCGTGGTGATGACCGT
GGTTCATTGCCGGCATCGGCGCGATTTTGACTTCCAGTTGGACGGTTTGCGCTTTGGCGT
TTTTAAATTTCAGGGTAACGGGAATTTTATCGCCCTCTTTTAATTGTTTTTTCAAACCCA
TAAACATCACATGATAGCTGCCGGGTTTGAGTTCGGTAACGGATTTCGCTTCCAAAGGCA
CGCCGCCTTCGACTTCGCGCATCCGCATCACGCCGTTGTCGTTGATGTGGGTATGCACTT
CGACGCGGTCGGCAACGGGGCTGCTTCCGCCGAGCAAAAAGTCTTGTTTGGCTTCGTCGT
TGTGGATTTTCATGAACGCGCCGCCTATTTTCATACCTTCGACGGTGGTGCGCGCCCAGC
CGTCCTCAACGTGGACTCCGGCGGCGGAAACCGCGCCTGCCAAACCTGCCATCATCACGG
CCGCCAATAATTTTTTCATCTTTCTGCTCCTTATAATATCAGACGGGGAATGTGCTTAAT
CTTATAGCGGATTAACAAAAACCAGTACAGCGTTGCCTCGCCTTAGCTCAAAGAGAACGA
TTCTCTAAGGTGCTGAAGCACCAAGTGGATCGGTTCCGTACTATTTGTACTGTCTGCGGC
TTCGTCGCCTTGTCCTGATTTTTGTTAATCCACTATACATACAAATACTGCCTGGAAATT
TGATGTAGATTAAGTGAATAATAAATACCACATACTAATCCTAAAGGATTACAAATCCTG
CTGCAAGCGTTTTACCCGAACAGGGCAGACAGCCAAACCGCCGCCAACATCAGCATCGCG
AACAATTGTGCGGCAGAACCTGCGTCTTTGGCGAGTTTGGCCAGCTCGTGTTTTTCGGTC
~~GAAGTATGATCGACGGCAGCTTCGACGGCGGTGTTGAACAGTTCGACAATGACCGACACA~~
AAAGACGCGATAATCAACGGCAGGCGGACGGCGGTTTCGGAAACCCAAAAAAATGCCGCG
CACACCAGCAGTACGTTCAGCCACAAAACCTGACGGAATGCCGCTTCGTAACGGTAGGCG
GCGGCGATGCCGTCTATCGAATAGCCGAATGCGTTAATGACGCGCCTGATGCCGCCTTTG
CCTTTTTTTTCTGCCGCGTAGGAGGAAGGTTCCATCGGTATCCTTTCAAAATGTTCTCAA
TATAGTGGATTAACAAAAACCTGTACGGCGTTGCCCCGCCTTAGCTCAAAGAGAACGATT
CTCTAAGGTGCTGAAGCACCGAGTGAATCGGTTCCGTACTATTTGTACTGTCTGCAGCTT
CGCCGCCTTGTCCTGATTTTTGTTAATCCACTATATATACCGTCTGAAAACGGGGCGGCG
GGGTGTCCGTACGGTATTAAGCGTATCCCTGCCGGCTGAGAGAAAACCCTGCCTGCCCAA
TCAAACCAGGCGGTTGTGAAGCAAAAGCCTTTCAGACGGCATCGGTTTAACGTACCGACC
ACGCGGCAACGGCATCGGCAAACATTGCCGCCACATCGAAACCTTTTTGTTTCATAATTT
CTTGGAATCCGGTCGGGCTGGTTACGTTGACTTCGGTCAGGTTGCTGCCGATAACGTCCA
AACCGGCCAGCAGGATGCCGCGCCGTTTGAGTTCGGGGGCGAGCGTTTCGGCAATTTCGC
GGTCGCGTCCGCCCAATTCCTGCGCCACGCCGCGCCCGCCTGCCGCAAGGTTGCCGCGTG
TTTCGCCGTTTTGCGGGATACGCGCCAAAGCATAGGGGACGACTTCGCCGCCGATAATCA
GGATGCGTTTGTCACCGTGTACGATTTCGGGAATGTAGCGTTGCGCCATAATGGTGCGGG
AATCAAGCTGCATCAGGGTTTCGAGGATGCTGCCGATGTTGGGGTCTTTTTCGGTCAGGC
GGAAAATTCCCATACCGCCCATGCCGTCGAGCGGTTTGATGATGATGTCGCCGTGTTCTT
TCAAAAATGTGCGGACATCGGCGGAACGGGTCGTTACCAGCGTGGGCGCGATAAAGCGGC
TGAAGTTCAAAATCGCCAGTTTTTCATTAAAGTCGCGCATCGCCTGTCCGCTGTTAAAGA
CCTTCGCGCCCTGCTGTTCCGCCAGCGTCAGTAATTGGGTGGCGTAGAGGTATTGCATAT
CGAACGGCGGATCGGTACGCATAATCACGGCATCAAATGCTTCCAATGCCGTCTGAACTT
TGTCGGCAGATTTGAACCACGCATGATCATCATCGTTTTTTGCACCCAAAAATTCAAATG
CCGATGCCTGCGCCGTTACCAAACCGCCGTTTACAGACAATTCCCCGCTCAATGTGTGAA
ACAGCCGCCAGCCGCGTTTTGCCATTTCGCGCATCATCGCGTAGGTGGTGTCTTTATAGG
TTTTGAAACTTGCCATCGGGTCGGCGATAAAGAGGACTTTCATCATATTTCCTTTCCGGT

## Appendix A

GTGCCGAATGTGCCGCATTTCGCGGGTAAAGGAGAAATTCCGCCCGAACAATATTCAGAC
GGCAGGGATGGGGTTTTACTTAGGCTGCCAAGAGTCTTTCAGCGTTACCGTGCGGTTAAA
CACCGGCGTGTCTTTGCCGTGGTCTTTACGGTCGGTTACGAAGTAGCCGATACGCTCGAA
CTGCCAACGGCTTTCTGCCGGCAAATCTTTGGCGGCAGGTTCGGCGTAGGCGGTGATTTC
CTTGACGGATTCCGGATTGAGGAAATCGGTGAACGGCAGGTATTCGCCGTCTTCGCCGCG
CACGGCATCGGGACGCTCGACGGTAAAGAGGCGGTCGTACAGACGGACTTTGATTTCGGC
GGCGTGTTCGGCGGAAACCCAATGAATCACGCCTTTAACTTTACGGCCTTCTGGATTTTT
GCCCAAGGTGTCGTGGTCGATGCTGCATTTGAGTTCAACCACATTGCCTGCTTCGTCTTT
GACGACTTCATCGCACTTGATGACATAGCCGTGGCGCAAGCGTACTTCGCCGCCGGGAAT
CAGGCGTTTGAAGCCTTTGGGCGGATTTTCGGCAAAGTCGTCGGCTTCAATATAGATGGT
TTGGGAAATAGGTACTTCGCGCTCGCCCATTTCCTCGTGGTTCGGATGGAACGCGGCACG
GCGGCTTTGGGTTCTGCCGGTTTCAAAGTTGGTCAGGGTCACTTTGAGCGGGTTCAACAC
CGCCATCAGGCGTGGGGCGGAATTTTCCAACTCTTCGCGAATCGCGCCTTCCAACACGCT
CATATCGACGATGTTTTCAGATTTGGAAATACCGGCGCGTTTGGCAAACAGGCGCAGCCC
TTCGGGCGTGTAGCCGCGTCGGCGCATACCGGAAATGGTCGGCATACGCGGATCGTCCCA
GCCGGAAACGTGTTTTTCCACAACCAACTGATTCAATTTCCGTTTGGAGGTAATGGTGTA
CAAAAGCTCCAAACGGGAAAACTCGTATTGGCGCGGACGGGTGGCATGCGGCGCAGGAAT
GTTGTCCAACACACAGTCGTACAGCGGACGGTGTGCTTCGAATTCGAGCGTACACAAGGA
ATGCGTGATGCCTTCGATGGCATCGGAGATGCAATGCGTGTAGTCGTACATCGGGTAGAT
ACACCATTTGTCGCCGGTGTTGTGGTGATGGGCGCGGCGGATGCGGTAGATGACGGGGTC
GCGCATATTGATGTTGCCCGATGCCATGTCGATTTTCAGGCGCAGGGTTTTGCTGCCGTC
GGGGAACTCGCCGTTTTTCATGCGTGTGAACAGGTCGAGGTTTTCTTCGACGCTGCGGTC
GCGGTAAGGGCTGTTTTTACCCGCTTCGGTCAGCGTACCGCGGTATTCGCGCATTTCTTC
GGGCGTCAAATCATCGACATACGCTTTGCCGTCTTTAATCAAACCGACGGCGTAGTCATA
AAGCTGGTCGAAATAGTTGGAAGCGAAACGCGGCTCGCCCGCCCAATGGAAACCGAGCCA
CTCGACATCTTCTTTGATGGCGTTGACGTATTCGTCGTTTTCTTTTTCGGGGTTGGTATC
GTCAAAACGCAGGTTGCACAAGCCGTCGTAAATATACGCCAAACCGAAGTTCAGGCAGAT
GGATTTGGCGTGTCCGATGTGCAGGTAGCCGTTGGGTTCGGGCGGAAACGGGTTTGGAC
AGCTGTATGTTTGCCGCTTTCGAGGTCTTCTTCGATGATGGTGCGGATAAAATGGTTGTC
CGCAAATTGGTCTTTATTGAGCATAGTTTTCTTTGAACAGATGGCTTCAGACGGCATTGG
AATGATTCCGTATGCCGTCTGAAGCGGTTTGGGAATGTGTTTATTGTACCCGACTTGCGC
GCTTTGACATAGCGTTCAGACGGCATCGGCAATCAAGCATTCCACCCCCGCCTCTTTCAG
CATCTTCTGCATCGCGGTATCGGGCAGCCGGTCGGTAAATACTTTGTCAAACGCCGTAAT
GTCGCCGAGCCTGACCAGCGCGTTGCTGCGGAATTTACTGTGGTCCACGCCGAGGAAGCG
GACGCGCGCATTGGCAATCATCGCCTGCATCACGCTGACTTCTTTGTAGTCGTCGTCCAA
AAGCGAACCGTCGCTTTCCACGCCGTGCGTACTCATCACGGCATAATCGACTTTGAACTG
GTTGATAAAATCGACGGTTGCCACGCCGGTAATACCGCCGTCCAAAGGGCGGACGACTCC
GGAAGTGATGATGACCGTATAATCCGTCCGCGCCGAAGCAATCGAGGCGGCGTGGATATT
GTTGGTAATCACCCTCAGGCTGCCGCGCCGCCTGACCAGCTCCGACACCACGGCCTCCAT
CGTCGTGCCGATACTGACAAACAGCGACGAACCGTCGGGGATGTGTTCCGCAATCAGCCG
GGCAATGGCGTTTTTTTCGTTTTGACACCGGGTTTGGCGGTCGGCGGGCAGGCCCTCCGG
CAAGTTTCCGCCCGAAGATGCGCCGCCGTGATGGCGTTTCAGGCTGCCGACCTCCTCCAA
CTCGCGGATGTCGCGGCGTATCGTCTGCGGGGTAACGTCCAATGCGGCGGCAAGCTCGTC
CACCGACATAAACTGATGCCGGCGGACAAGGCTTAAAATCTCTCCGTGCCTTTGGATTTT
CGGCTTCATCGTTTTCTGCCTCCTTGCATCGGGATGCCGATTTTACCGCGTTCAACCCAA
AGCGGAAAACACCACCATCAGAAACGGGGCGGCGATATTGACCACCACGCCGAAGCTGAC
CGCTACCGGCACGACTTCCAAACCGCCCGCACCCTGAATCACGGGCAATGTAAAATCCAT
ACTGGTCGCACCGCCAACCCCCACCGCCGCATCTGGAAAACGCTTCATCAGCAGCGGGAT
AAATGCCAGTGCAAACAGCTCTCGTGCCAAATCGTTCAGCAGCATGATGCTGCCCCATAC
CGCGCCGTAAGCCTCGGTCATGACCAAACCCGAGAGGGAATACCAACCGAAGCCGGAAGC
CATCGCCAAACCTTTCGTCCACGACACACCGTCTGTCGATGCGGCAAACAGCAGCCCGCC
CGAAAGAGATGAAAGCATAAACCAGACCGACAACCGAATACCCCTGCGGTTGACCAAAAC
CTGCCGCAACGATACGCCGCTGCTTTTGAGCTGTACGCCGATGAGGAACACCAGCAGCAT
CAGACAATACATGCCCGCGCTTTCAGACGGCATCCAAATATCGCGCATCAGTTTGCCGAA
TGCAAATCCGAGCAGCACGCATCCGAGCTGCCCCACACTGCCCGACACGCCGACCGAAAC
GCCCTTCCCTTTCCCCTTTATCCGCCACGGGAATAACTTTCCCAACACTGCCAAAGCAAG
CAGGTTCGCCCCGACCGTACAAACAAACAGCCACAGAACCGTCAACGCCATATCGTCCAA
CCGCGAACCCAAATCCTCCACGCGCGACAACGAGACGCCGATCAGCAGCAGCACAGCATA
CACCAAGACCGATAGCACCTTATCCAAAGCGGGCAGGTAAGGCTTGGGCACACGGATAAA
AAATCCGGCAAACATCGGTATCAATACCGAAAGCAACGTCATCAGGCTGTCCATCTACTG
CTCTCCTTTATTGCCGCATGATATGTGCGGTTTAAAAATTGCCGTCTGAAAATTGCAGAT
ACCCGCATCCATATTTCAGACGGCATCAGGTTCGCCATTAAAAAACCGCCTGAAGGTTCA
GGCGGCTTATCCGCTCCGGCATTCAATCTTCCAAAGTCTTTTCCAAACGCTCCATACAGT
TGCCCAAATGGCGGCGCAGGATTTTGACCACGCGGTTGCGCCTGCCCGCCAGCAGCAGGT
CGAGGATTTCGCGGTGTTCGGAATGCGTATGCGTATTGATGGCGTGTTTTTCCTCGCGAT
GCACGCCCGCCACGGCGACAATCAGGGAAGACCGCGCGCACAGCGTATTCATAATGTCGA
ACAGCACATCGTTGCCCACCAGGCGCGCCAGTTCGACGTGGAAGGCATTGGACAGGCGGT
TCCAGCCGACGCGGTCGCCCCTGCCGGAGGCCTCTTCTTCGCGCCGTATCATCGCATAAA
GCGGCTTGAGGCGCGTTTCCAAATCCGGCAAATCTGCGAGGATATTCAAAATCATCGTCT
CCATTTCGATGCGCGCATTGAACACATCCTGCATTTCTTTCAAATCGGGAACGTGGACGA
ACGCGCCCCTGTTGGGTTGCAAATCGACAATCTTGTCGTGCGCCAAAAGCGACAGCGCGC
CGCGGACGGTGTTGCGCGAACACACCATCTGACGGCAAAGTTCGGATTCGGTCAGCTTTT
TGCCGGGCAGCAGCACCTGATCGGTAATGCCGTCCAAAATCAGGGCGTAAACACGGAACA
GCTCCGAATCGTGCCGCTCTTCGAGAATCAGGGAAGACGTGGTCGGCGCATGGATAATGT
CGTCGTTTTCAAAGTTCATGATGTTTTCCGTATTTTTACGCTTTCAAATTTTTTAAGATG

## Appendix A

TTTTAAGGCGGCTGTGTTTCAAATCGTGTCAGAGGAATTAAAGCATTGCACAAATTTATT
TTATAGTGGATTAACAAAAATCAGGACAAGGCGACGAAGCCGCAGACAGTACAAATAGTA
CGGAACCGATTCACTTGGTGCTTCAGCACCTTAGAGAATCGTTCTCTTTGAGCCAAGGCG
AGGCAACGCCGTACTGGTTTTTGTTAATCCACTATAATTCAATAAATTAATATATGGCTT
AAAATAACGGGATTCTCGCCTCCCGCCCGCCCGCAGAAGCAGGCGGATATCATTTTAAAA
CGCGGCATTTAAAATTTGACCGAAAATTGTTGACAATCCGGAATCAAGTCTGCACAATAC
CCCGACAAGTCCAAGTATTATAAAGGCTGAATAAAGAGGAAACAGCAGGCAGATATATTC
GGGAGGTGCAGTCCGAATATATCTGCTTTTTTATGCGCCTCCGGATTGCCTGCCGCACCT
TTCCCTTCAGACGGTATCAGCCGTTTCCCCATAATGCCGCCCGATGCCTATTTATCTGCC
CCGGCAATTTCAAAACTGTGGGTAATCTTTGCCGCTTTGCCCAACATAATCGAAGCCGAA
CAGTATTTTTCGGCAGACATCTGAACGGCGCGCTCAATGGCCGATTCTTTCAAATCATGC
CCGAATACTTTGAAATGGATGTGGATTTCGGTAAACACGCGCGGCGCATCGTCCGCCCGT
TTCGCCGTAACCGTCGCACGGCAGTCAGTCACTTTCTGACGCTGTTTTTCGGCAATCATC
ACCACATCGATGCTCGAACAGCCCGCCACGCCCAACAGCAGCATTTCCAAAGGGCTGGGC
CCGCGCTTAGCCTTACCTTCTGCCGCCGACCCCTCCATAACGACGCTGTGCCCGCCTTCC
GTCGTGCCGACAAAACACATCCCGTCTATCCATTTTGATGTAACCTGCATGGTGTCATTC
CTGAAAATAGCGTTAAAACCGCTTTGCATATGGCGTTATTGTAAACAATTTCAAGCGGCT
TATGCAGAAATATGGACAAAACGGCAAAAAAACACTTGAAAACCGATTTACGGTTTGGCT
GCCTGGCCGTTGATCTGCACCGATTTGAGTTTCAGCGTATAGGTTTTGCCGTCGTCGGTA
TAGCCGATTTGTGCCGGAATATTGTTCAGGGACGGTGCGAAGAAATACATTACCGCATCG
TCGCCGCGCCGCACCCGATATTTGACGACTTCGGTTTCCACGCCGCCTATGCTGTATTTT
CCTGTACCCGCCTTATTCAAACCGCCGACGGAATAAAGTTTTTTGCCGTTGGTGATTTTC
AGCCCCGGGGGGAGTTTCGCGTCATTTGCCGCCAACTGCCAGGCAAGCGTGAACAAATCC
ATAGCCTTGGGGCTTTGCTCGGTTTTGCTCTCGCCCGCTTTGCCGTAAGTTACGCTGCCG
TCGGCGAATTTGGCTTCCGCATACAGTTTGCCCCTGCGTATGTCTCTATAGTAGGTAGGG
TGCAGGGTATTGCCGACAACCGTACCGCCGGACTCGAAACGGATATTGTATAGCGGCACT
TTAATCGTCGAAACGATTTTGTAAGCATTGCCGCTGCGTTCAAATGTCATCGTGGCGGGA
ATGCCGTAGCTGCCGGAATAGTGCAGCACGGCGGATTGGGGCAGCCCTGCCGCATACGCG
CACGGCAGGGCGGCGGACAAAATGGCGGCGGAAAATATATTTTTAAAAGTCTTCATCATT
TGCTCCCGCCCGGTTTACGCCGTCAGAAAACGGGCGGCATCGGCGTTTTCCGAATTTCTG
ACGCGGTTTCCCTCAATAATCAGGCGGCCGGCGGCAAAATCGGCAACGGCTTTCGGATAA
AGTTTATGCTCGACAGCCAAAACCCGTGCGGCAATATCGTCTGCCGTATCGCCGTCGAGT
ATCGGCACAACCCCTTGCGATACAATCGGGCCGCAATCCAGTTCGGCAGTAACGAAATGG
ATGGTGCAGCCGGCAACGCGGCAGCCCGCCTCCAAAGCGCGTTCGTGCGTATGAAGTCCG
GTAAACGAGGGAAGGATGGACGGGTGAATGTTCATCAGCCTGCCTTCGTAACGGGCGCAA
AACTCGGGGGTCAGAATCCGCATAAAACCTGCCAAAACCACCAAGTCGGGTTGATATGCG
TCGATTTTCTCCATCATGGCGGTATCGAAGGCAAGCCGGGATGTAAAGTTTTTATGATTC
AGGCTATCGGTCGGGATGCCGCGTTCGGCCGCCCATTGCAAACCGGCAGCCGTTTCGCTG
TTGCTCAACACGGCGGCAATGCGGACGTTGTGAATGGCGGCATTGACGATTGCCTGCATA
TTGCTGCCGCGTCCAGAAATCAGGATGACGATGTTTTTCATAATGGTGCGCTTTTGAAAG
GGATGCCGTCTGAACCGCTGTTTGGTGGTTTCAGACGGCATTTGCCGTAAAAATGCCCGA
AAACCTGTTTCGGGCATGGATTCGGACTTAATTTACTTTTTTGATGTCGACTTGAGCCGG
CTGCTTGGCGGGCGCGTTTTCGGGTGCGCCGATTTTGACCAGTTTCACATCAAATACCAA
AGTGGCGTTCGGACCGATTTTGTCGCCCGCACCCTGTTCGCGGTAGGCAAGGTTGGACGG
GATGTAGAACGTGGCTTCGCCGCCTTCTTTCAGAAGCTGTACGCCTTCGGTCCAACCCGG
AATCACTTGGCTCAAAGGGAAGGTGACCGGGCCGCCGTTGGCTTTGCTGCTGTCGAATAC
CGTACCGTCAATCAGGCGGCCTTCGTATTCCACGGTAACGATGTCGTCTTTGGTCGGCTG
TTTGCCTTCGCCCTGTTTGGTGATTTTGTATTGCAGGCCGGAAGCAGTGGTCTTCACGCC
GTCTTTGGCGGCATTTTCTTTCAGAAAGGCTTCGCCTTTTTCTTTATTGGCCTTCGCGTC
~~GGGGTTGTGTTTTTGTAGGGGTTTAGGCTGTTGTTGGTGAAGGAATTTGATCATGAGTTC~~
CTGAGCCTGCTCTTCGGTCATTTTGATTTCTTTGCCGTCATACACTGCCTGCATGGCTTC
GGTAAAGACTTTCAAATCGATTTCCGCGCCCTGTTCCTTCATTTGCTTCAGGGAGCGTCC
GATGTCCACGCCCATCGCATAGCTTGCCTGCTGCATCGTGCTGCCGATCGAAGAGGTGTC
GCCCTGCGCGGAAGAAGCGGCGGCAGGTTCGGATGCAGATGCGGGGGCGGCTTCTTTTTT
GCCGCAGGCGGAAAGTGCCAAAGCGGCGGAAAGGGTCAGTGCGCTGATTTTGAAAATGGT
GTTCATGATGGATCTTCGCTGTCGATAAGGTCGGAAAAACGGGATTATAGCCGAGTTTGA
ATGTTTCAACACACAGGATGACACATAAAGCGTCAATCGTGTGTTGCCCTGTTTTGGAAG
GGATTGAACCTTCCAAAATAAGTTTTGATTCTACCGCCCCGAGGGACAGATGTCCAAGTG
GCGGGGTTCAACCGATAAGGAAATTTTAATCAAATAGAATCAAGCCTGTTTAAATTTTGT
AAATGCGGCATTTCAGACGGCATTTTATGCCTTGCCCTCCATGCCGTGATGTTCGATGGC
AAAACCGCTTCGGCGGTAGGCGGTAAAGCGTTCGCGCGCGTCGGCGAGCTCTTCCAAGCT
GTTGCCGACGATTTCCAAAACGCGCGCGGGCAAGACCGGAGCGGTGTTCCAAAAACCGTC
GGACAGGTTCAAAACGGTCATGCCTTCGGGAATTCGGGGCAGATTGCCGCCGCAGGCAAG
CAGGACGGATGGTTCAGACGGCATGGCTTCTTCCGTTTCCCAAATTTCGTGCGGAATGAA
ACTCTCGGCCTCGTATTGCCAAAGCATTTTGTCCAATTCCTGAAGCTGCCCGAACGAATC
GGACCACACCAGTATCCTGCCGCCGTCCCGAATCGCACGGGCAATCAGGCGGCAGGTGAA
AATCGGAACTTGGGCAACGTGCGTGTAAAAGGTGGCTTTCGGCATATTGTTTGAACATTT
GGCAGGATAATGCCGTCTGAAAGGCTTCAGACGGCATTGTGGGAAAATTAAAGATTCCGC
AGATAGTTCAGCAGCAAGGGAACGGGACGGCCGGTCGCACCTTTTTCCGCACCGGATTTC
CACGCCGTACCCGCGATGTCAAGGTGTGCCCATGGATAGTCTTCGGTAAAGTAGGATAGG
AATGTTGCGGCGGTAATCGTGCCCGCGCCGGGCGTGCCGATGTTTGGAATGTCGGCAAAG
TTGGATTTGAGTTGGTCTTTGTAGGTCTCAAAGAGCGGCAGTTGCCATGCTTTGTCGTCC
ACGTTGTAGAAGCGGCAAGCAGGCTGTCGATCAAATCCTGATTGTTGCCCATCACGCCGC
TGACATCGTGCCCCAAGGCAACAATACACGCGCCGGTCAGGGTGGCGACGTCGATGACGG
CTTTGGGTTTGAACTGCTCGGCGTAAGTGAGCGCGTCGCACAAAATCAGACGGCCTTCGG

## Appendix A

CATCGGTGTTCAACACTTCGATGGTCAGCCCTTTCATACTTTTCACGACATCGCCCGGTT
TGTTTGCCGCGCCGGAAGGCATATTTTCACAAGTGGCGACGACGGCAATCAGGTTAATCG
GCAGTTGCAGTTTGACGGCGGCGCAGAAGGTGCTGATGACGGTTGCCGCTCCGCACATAT
CAAACTTCATTTCGTCCATGTTCAGGCCGGGCTTGAGGGAGATGCCGCCGGTGTCGAAGG
TAATGCCTTTGCCGACCAATACCACAGGCGCGGCTTCTTTGTCGGCTGCACCGAAATAGC
TCAGTTCGACCAAATAGGGGGCTTCCGCGCTGCCTTTGGCGACCGACCAAAACGAACCCA
TGTTTTCTTTGATGTAGTCTTTTTCGATGATTTTGGCGTGCGCGCCCAGTTTTTCGGCTT
CGGCTTTGGCGGTGCGCGCTAAAAATTCGGGCGTGCATTCGTTGGGCGCGGCGTTGCCCA
AGTCGCGGCAGAGGCTTTGTCCGTAAACTTGCGCTTCGGCGACGCGCAAGGCTTCTTTGA
CGGCGGCTTCGTGCGCGGTATGGAACACGGCAGTTTCAAATTTGGCGGGCTTGGCTTCTT
TTTTGTAGCGGTCGAAACGGTAGGCGGCATTGCCGAACGCAATCGCAAACGCTTCGGCAA
CGGCTGCAGCCTGCGCTTCTTCAAAGACGTGAACGTCCACATTGACCGTTTCCTGATTTT
GCGCCCATTTGGCGGCTTCGGCGGCGGCCTTGTTCAATGCGGCGCGGCCGGTGCTTTTCA
GACAGCATACGGCAACAGCCTGCAAACCGTTGCCTGTCGGGATTTTTGTGTCGGCAAAAT
TTTGACCTTCTTCAAGCGAAGACAAAAGGGCAAGGACGGTCGGGTTGCTCAGTTGCGATG
CTTCGGTGCAGACAAATAACTGTGCGCCTGCCTGCTGTTCCTGCAAGATTTCGGTTTTTG
TGCTAAATTCCACGTTTATTCTCCTGATTGAGACGGTTGTCGGTAGTTTTCGGACGGCCT
TTCGCTCAAAAGACCGTCTGAAGACGGCTGGCACGATTGTACCCCATTTGAAGCACCGTC
TGAAACCTTGCGCGGACAATCCGCCTGCGCCGAACCGCTTACCGCCCCCCTGACCGCGAT
TCTATGATTTATCAAAGAAACCTCATCAAAGAACTCTCTTTTACCGCCGTCGGCATTTTC
GTCGTCCTCTTGGCGGTATTGGTCTCCACGCAGGCAATCAACCTGCTCGGCCGTGCCGCC
GACGGGCGTGTCGCCATCGATGCCGTGTTGGCATTGGTCGGCTTCTGGGTCATCGGTATG
ACGCCGCTTTTGCTGGTGTTGACCGCATTTATCAGTACGTTGACCGTGTTGACCCGCTAC
TGGCGCGACAGCGAAATGTCGGTCTGGCTATCCTGCGGATTGGCATTGAAACAATGGATA
CGCCCGGTGATGCAGTTTGCCGTGCCGTTTGCCGTTTTGGTTGCCGTCATGCAGCTTTGG
GTGATACCGTGGGCAGAGCTACGCAGCCGCGAATACGCTGAAATCCTGAAGCAGAAGCAG
GAATTGTCTTTGGTGGAGGCAGGCGAGTTCAACAGTTTGGGCAAGCGCAACGGCAGGGTT
TATTTTGTCGAAACCTTCGATACCGAATCCGGCATCATGAAAAACCTGTTCCTGCGCGAA
CAGGACAAAAACGGCGGCGACAACATCATCTTCGCCAAAGAAGGTAACTTCTCGCTGAAC
GACAACAAACGCACGCTCGAATTGCGCCACGGCTACCGTTACAGCGGCACGCCCGGACGC
GCCGACTACAATCAGGTTTCCTTCCAAAAACTCAACCTGATTATCAGCACCACGCCCAAA
CTCATCGACCCCGTTTCCCACCGCCGTACCATTCCGACCGCCCAACTGATTGGCAGCAGC
AACCCGCAACATCAGGCGGAATTGATGTGGCGCATCTCGCTGACCGTCAGCGTCCTCCTA
CTCTGCCTGCTTGCCGTGCCGCTTTCCTATTTCAACCCGCGCAGCGGACATACCTACAAT
ATCTTGATTGCCATCGGTTTGTTTTTAATTTACCAAAACGGGCTGACCCTGCTTTTTGAA
GCCGTGGAAGACGGCAAAATCCATTTTTGGCTCGGACTGCTGCCTATGCACATTATCATG
TTTGCCGTTGCACTCATCCTGTTGCGCGTCCGCAGTATGCCCAGCCAGCCCTTCTGGCAG
GCGGTTGGCAAAAGTCTGACATTGAAAGGCGGAAAATGAACCTGATTTCACGTTACATCA
TCCGTCAAATGGCGGTTATGGCGGTTTACGCGCTCCTTGCCTTCCTCGCTTTGTACAGCT
TTTTTGAAATCCTGTACGAAACCGGCAACCTCGGCAAAGGCAGTTACGGCATATGGGAAA
TGCTGGGCTACACCGCCCTCAAAATGCCCGCCCGCGCCTACGAACTGATTCCCCTCGCCG
TCCTTATCGGCGGACTGGTCTCCCTCAGCCAGCTTGCCGCCGGCAGCGAACTGACCGTCA
TCAAAGCCAGCGGCATGAGCACCAAAAAGCTGCTGTTGATTCTGTCGCAGTTCGGTTTTA
TTTTTGCTATTGCCACCGTCGCGCTCGGCGAATGGGTTGCGCCCACACTGAGCCAAAAAG
CCGAAAACATCAAAGCCGCCGCCATCAACGGCAAAATCAGCACCGGCAATACCGGCCTTT
GGCTGAAAGAAAAAAACAGCATTATCAATGTGCGCGAAATGTTGCCCGACCATACGCTTT
TGGGCATCAAAATTTGGGCGCGCAACGATAAAAACGAATTGGCAGAGGCAGTGGAAGCCG
ATTCCGCCGTTTTGAACAGCGACGGCAGTTGGCAGTTGAAAAACATCCGCCGCAGCACGC
TTGGCGAAGACAAAGTCGAGGTCTCTATTGCGGCTGAAGAAAACTGGCCGATTTCCGTCA
~~AACGCAACCTGATGGACGTATTGCTCGTCAAACCCGACCAAATGTCCGTCGGCGAACTGA~~
CCACCTACATCCGCCACCTCCAAAACAACAGCCAAAACACCCGAATCTACGCCATCGCAT
GGTGGCGCAAATTGGTTTACCCCGCCGCAGCCTGGGTGATGGCGCTCGTCGCCTTTGCCT
TTACCCCGCAAACCACCCGCCACGGCAATATGGGCTTAAAACTCTTCGGCGGCATCTGTC
TCGGATTGCTGTTCCACCTTGCCGGACGGCTCTTCGGGTTTACCAGCCAACTCTACGGCA
TCCCGCCCTTCCTCGCCGGCGCACTACCTACCATAGCCTTCGCCTTGCTCGCCGTTTGGC
TGATACGCAAACAGGAAAAACGTTGAACCAATGCCGTCTGAACCTCTCTTCAGACGGCAT
TTGTTTTCATTGACACATTCCCACAGACAGATAGCCGTTCCCTATTACATTACCTGTCAT
AACAGTTCCATTTTTGTTAAAACTAGTCTATGATAGCGGTACAAATATTGTTTACAATAT
TTAACGCAAATCATTTGCAACCCGACAAAAGAAAAACAGAAAAAGGAACAAAGAGATGTT
AGAAGCCTATCGTAAAGCCGCCGCCGAGCGCGCCGCCCTCGGCATTCCCGCCCTCCCTTT
GAACGCGCAGCAAACCGCCGATTTGGTTGAGCTGCTGAAAAGCCCGCCCGCAGGCGAAGG
CGAGTTCTTGGTCGAACTGCTTGCCCACCGTGTTCCGCCCGGTGTGGACGATGCCGCCAA
AGTCAAAGCCTCATTCCTGGCTGCCGTTGCCGAAGGCAGCGCGTCCAGCCCGCTGATCTC
CCCCGAATATGCGACCGAACTCTTAGGTACAATGCTCGGCGGTTACAATATTCACGCCTT
AATCGAACTCTTGGACGACGACAAACTCGCGTCCATTGCTGCCAAAGGCTTGAAACATAC
GCTTCTGATGTTCGATTCCTTCCACGACGTTCAAGAAAAAGCCGAAAAAGGCAACAAATA
CGCGCAAGAAGTTTTGCAATCTTGGGCAGATGCCGAATGGTTCGCCTCACGCGCCAAAGT
TCCCGAAAAAATCACCGTTACCGTTTTCAAAGTTGACGGCGAAACCAATACAGACGACCT
CTCCCCCGCGCCCGACGCGTGGAGTCGTCCCGATATTCCGCTGCACGCGCTGGCCATGCT
GAAAAACCCGCGCGACGGCATCACGCCCGACAAACCGGGCGAAGTCGGTCCGATTAAATT
GTTGGAAGAACTCAAAGCCAAAGGCCATCCGGTTGCTTACGTCGGCGACGTGGTCGGTAC
TGGTTCTTCACGCAAATCCGCGACCAACTCCGTCATTTGGCATACCGGCGAAGACATTCC
GTTCGTGCCGAACAAACGCTTCGGCGGCGTATGTTTGGGCGGCAAAATCGCGCCGATTTT
CTTCAATACCCAAGAAGATTCCGGCGCGCTGCCGATTGAAGTCGATGTATCTGCTCTAAA
AATGGGCGATGTCGTCGATATCCTGCCTTATGAAGGCAAAATCGTGAAAAACGGCGAGAC

## Appendix A

TGTTGCCGAGTTTGAATTGAAATCACAAGTATTGCTGGACGAAGTGCAAGCCGGCGGCCG
TATCAACCTGATTATCGGCCGAGGTCTGACCGCCAAAGCGCGCGAAGCCCTGAAACTGCC
TGCCTCTACTGCATTCCGCCTGCCGCAAGCGCCTGCCGAAAGCAAAGCCGGTTTCACCTT
GGCGCAAAAAATGGTCGGCCGCGCCTGCGGTCTGCCCGAAGGACAAGGCGTGCGCCCGGG
TACTTACTGCGAACCGCGTATGACGACGGTCGGCTCGCAAGACACGACCGGCCCGATGAC
CCGCGACGAGTTGAAAGACTTGGCTTGTTTGGGCTTCTCCGCCGATATGGTGATGCAGTC
TTTCTGCCACACCGCCGCCTATCCGAAACCTGTCGATGTAAAAACCCATAAAGAACTGCC
CGCCTTTATTTCCACCCGTGGCGGCGTGTCACTGCGTCCGGGCGACGGCGTCATCCACTC
GTGGCTCAACCGCCTGCTGCTGCCCGATACCGTCGGCACCGGCGGCGACAGCCATACCCG
TTTCCCCATCGGTATTTCCTTCCCCGCCGGCTCCGGCTTGGTTGCCTTTGCCGCCGCAAC
GGGCGTAATGCCGCTCGATATGCCCGAGTCTGTATTGGTACGCTTCAGCGGCAAGCTGCA
ACCGGGCGTAACCCTGCGCGATTTGGTGAACGCCATCCCGCTGTACGCAATCAAACAAGG
TTTGCTGACCGTTGCCAAAGCCGGTAAGAAAAACATCTTCTCCGGCCGCATCCTCGAAAT
CGAAGGCCTGCCTGATTTGAAAGTGGAACAAGCCTTTGAATTGACCGACGCATCCGCCGA
ACGCTCCGCCGCCGGCTGTACCGTGAAGCTCAACAAAGAGCCGATTATCGAGTACATGAA
ATCCAACGTCGTGTTGATGAAAAACATGATTGCCAACGGCTATCAAGACCCGCGCACTTT
GGAACGCCGCATCAAAGCTATGGAAAAATGGCTGGCAAATCCCGAGTTGCTCGAAGCGGA
TAAAGATGCCGAATACGCCGCCGTGATTGAAATCAACATGGACGACATCAAAGAGCCGAT
TATCGCCTGCCCGAACGACCCGGACGACGTGTGCTTCATGTCCGAACGCTCCGGCACCAA
AATCGACGAAGTATTCATCGGTTCGTGTATGACCAACATCGGCCACTTCCGCGCCGCCTC
CAAACTTTTGGAAGGCAAGGCAGACACCCCCGTCCGCCTGTGGATTGCGCCGCCGACCAA
AATGGACGCGAAACAATTGTCCGACGAAGGACACTACGGCGTACTCGGACGTGCCGGCGC
GCGTATGGAAATGCCGGGTTGCTCCTTATGTATGGGTAATCAGGCGCAAGTACGCGAAGG
TGCGACCGTTATGTCCACCTCCACCCGCAACTTCCCGAACCGTTTGGGTAAAAACACCTT
TGTTTACCTCGGTTCGGCGGAATTGGCAGCGATTTGCTCCAAACTGGGTAAAATCCCGAC
CGTTGAAGAATATCAAGCCAATATCGGCATCATCAACGAACAGGGCGATAAAATCTACCG
CTATATGAACTTCAACGAAATCGACAGCTACAACGAAGTAGCCGAGACCGTGAACGTTTA
ATCCCCGTCATCCGTATGAAGTAAGGGATTGACCGCAATGCCGTCTGAACAACCTTCAGA
CGGCATTGCAACATTCCGCTAACCCTTCTTTCCGCAAACGCTGCAAATACGGCGTTCACG
CCCCCACATAAAGGAAACGACAGTGAACCTGAAAAACCGCCATTTTCTGAAACTTTTAGA
CTTCACGCCGGAAGAAATCACCGCCTACCTCGACCTTGCCGCCGAATTGAAAGCCGCCAA
AAAAGCAGGGCGCGAGATTCAGCGGATGAAAGGGAAAAACATCGCCCTGATTTTTGAAAA
AACCTCTACTCGGACGCGCTGCGCGTTTGAAGTCGCCGCGCGCGATCAAGGCGCGGGAGT
GACTTATTTAGAGCCGTCCGCCAGCCAAATCGGGCATAAGGAAAGCATCAAAGACACCGC
CCGCGTGTTGGGCAGGATGTACGATGCCATCGAATATCGCGGTTTCGGTCAGGAAGTTGT
TGAAGAATTGGCGAAATACGCGGGCGTACCCGTGTTCAACGGGCTGACCAACGAGTTCCA
TCCCACACAAATGCTTGCCGACGCACTGACTATGCGCGAACACAGCGGCAAACCTTTGAA
CCAAACCGCGTTTGCCTACGTCGGCGACGCGCGTTACAACATGGGCAATTCCCTGCTGAT
TTTAGGGGCAAAATTGGGGATGGACGTGCGTATCGGCGCACCGCAAAGCCTGTGGCCGTC
TGAAGGCATTATTGCCGCCGCACACGCCGCCGCCAAAGAAACCGGCGCAAAAATTACCCT
GACCGAAAACGCGCATGAAGCCGTGAAGAATGTTGATTTTATTCATACCGATGTGTGGGT
CAGCATGGGCGAGCCGAAAGAAGTCTGGCAGGAACGCATCGATTTGCTGAAAGATTACCG
CGTTACGCCCGAACTGATGGCGGCATCGGGCAATCCGCAAGTCAAATTCATGCACTGCCT
GCCCGCCTTCCACAACCGCGAAACCAAAGTCGGCGAATGGATTTACGAAACCTTCGGGCT
GAACGGTGTGGAAGTTACAGAAGAAATATTCGAAAGCCCCGCCAGCATCGTGTTCGATCA
GGCGGAAAACCGTATGCACACGATTAAAGCGGTAATGGTCGCGGCTCTGGGCGACTGACA
GAACTGTGCCTGTTTAAATTCATCCGCAACACAGATACCGTCTGAACACGATGTTCAGAC
GGTATCCATATATAGTGGATTAAATTTAAACCAGTACGGCGTTGCCTCGCCTTGCCGTAC
TATTTGTACTGTCTGCGGCTTCGTCGCCTTGTCCTGATTTTTGTTAATCCACTATAAAAA
AACTGCCTACACGATGTGTAGGTAGTCCCGTTTGAAAACAATCAGTTTTTGTCTTGGTCA
ACCAATTTGTTGGCAGTAATCCAAGGCATCATGGCACGCAGTTGTGCGCCGACTTTTTCA
ACTTGGTGGTCGGCATTCAGACGGCGGCGGGCAGTCATAGACGCATAGTTGACATTACCC
TCTTGGATAAACATTTTTGCGTATTCGCCGGTTTGAATGCGTTTCAGGGCATTGCGCATG
GCTTCTTTGCTGGAAGCATTGACCACTTCAGGGCCGGTAACGTATTCGCCGTACTCCGCA
TTGTTGGAAATGGAGTAGTTCATATTGGCAATACCGCCTTCGAAAATCAGGTCAACGATC
AGTTTCATTTCGTGCAGACATTCGAAGTAAGCCATTTCAGGCGCGTAACCGGCTTCGGTC
AGGGTTTCAAAACCCGCCTTGATCAACTCGACCACGCCGCCGCACAATACGGCTTGTTCG
CCGAACAGATCGGTTTCGGTTTCTTCGCGGAAAGTGGTTTCAATCACACCGCCTTTGGTG
CCGCCGTTGGCAGCCGCATAAGACAGGGCGATGTCTTTGGCTTTGCCGGAATTGTCTTGG
TAAACGGCAATCAGAGAAGGCACGCCGCCGCCGCGTTTGTATTCACTGCGTACGGTATGG
CCCGGACCTTTGGGGGCAACCATAATCACGTCCAAGTCGGCACGCGGAACGATTTGGTTG
TAGTGCACGTTGAAGCCGTGTGCAAATGCCAGCGTTGCGCCTTCTTTCAAATTGGCTGTA
ACTTCGGCGTGATAGACGGCAGGCATGGTTTCGTCAGGCAGCAGCAGCATAACGACATCG
GCTTCTTTGGTCGCTTCAGCAACGGTTTTGACGACATGACCGGCTGCTTCGGCTTTTTTC
CAAGAAGAACCTTGGCGCAGACCAATCACCACGTTTACACCCGAATCTTTCAGGTTGGCG
GCATGGGCATGACCTTGCGAACCGTAACCGATGATGGCAACGGTTTTGCCTTTGATTAGG
GACAGATCGGCATCTTTATCGTAATAGACTTGCATTTGATTTCCTTTAAGGTAAATGGTT
GTCGAAGCCTTAAAATGTTGAGCGGCTTCGGACGGGTTAAACAGAGTGTGCCGCTTAATC
GGCAACTTCATTCATCAATACGATTTCCAACGCTTCGGTTTTGCCGTCGACGGACTGGAC
GAAGGCTTGGAAATGCGCGCTGGCGTTATGTTCGTCAATAGCTGCTTGAGATTTCCAATT
TTCCACGAAAACAAAACGGTTCGGTTTGCCGATTTCCTGATGGAGATCGTAGCTGATGTT
GCCCTCTTCTGCACGGCTGGCTTTGACCAGTTCTTTAAACTGTGCTGCCAGTGTTTCTGT
GTATTCCGGTTTGACGGTAACCAGTGCGACAATTTTAATGTTCGACATAAATCTCTCCTG
CCGTTCGTTTTTCAGACGACATTCAAATACCGTGCCGTCTGAAAGGTTACGGCGTTAAAT
TTTCAAAATACGCTCACCGCGACCGATGCCGGCCGCGCCTGTGCGTACGGTTTCCAAAAT

## Appendix A

TTGGGCGCGTCCGACCGTTTCCAAAAAGGAATCCAGCTTGTCTGTCGAGCCGGTAATTTC
AATCGTATAGCTGCGGTCGGTTACGTCGATGATGCTGCCCCGGTAGATTTCGGTCAAGCG
TAAAAATTCGTCGCGGTCTTTGCCGGCGGCACGGACTTTTACCAACATCAGTTCGCGTTC
GACAAAACGGCTTTCATTCAAATCGACCACTTTAATCACTTCAATCAATTTATTGAGTTG
CTTGGTAATTTGTTCGATGACCTGCTCGTCGCCGTGGGTAACGATGGTCATCCGTGACAG
GGTTTTGTCTTCGGTCGGCGCAACCGCCAAAGAATCGATATTGTAATCGCGTGCAGAGAA
CAAACCGACCACGCGGCTCATCGCACCTGATTCGTTTTCAATCAGAACAGATAAGATATG
TCGCATTTGTCTCTCCTTACGCCTTTCCGTCCGCACGCATATGCGGCGGAAGTACCATTT
CGTCCAAACCTTTGCCGTTGCCGACCATGGGCATCACATTCTGTTTCTGGTCGGTCAGGA
AGTCGATAAACACCAGCCTGTCTTTTTGGTTCAATGCTTCCAACAACGCACCTTCCACAT
CAGACTTCTTGTCCACGCGGATACCGATATGGCCGTATGCCTCGGCAAGTTTGACGAAAT
CGGGCAAAGAATCGAAATAGGTTTCCGACTCTCGTCCGCCGTAATATATTTCCTGCCACT
GGCGTACCATACCGAGATAACCGTTGTTCAGCGTAATGACGTTAACCGGAATCCGATATT
GGAAACAGGTGGACAGCTCTTGGATGTTCATCTGGATCGAGCCGTCGCCGGTGATACAGA
ATACGTCTTGATCCGGGGCGGCAAGTTTTGCACCAATCGCATAAGGCAGACCCACGCCCA
TCGTACCCAAACCGCCGGAATTGAGCCATTGGCGCGGACGTTCGAAGGGATAATATTGAG
CCGCAAACATTTGATGCTGCCCTACATCCGATGTGATGATTGCCGAATTGCCGGTAATCT
CGGCAAGCTTCTGAATCACATATTGTGGCTTGATAATTTCGCTGCCGTTGTCAAACCACA
AGCAATCTCGGGAACGCCATTCCTCTATGGTTTTCCACCATTTGCCCAAAGCATCTTCAG
ACGGCACGGACTCTTGTTTTTGCCACAGCGCAACCATCTCGGACAAAACGTTTTTCACGT
CGCCGACAATCGGAATGTCCACCTTCACGCGTTTGGCGATGCTGGAAGGATCGACATCGA
TATGGATAACCTTCTTCGCCTTCTCGAAAAATTTGGACGGTACGGAAACCACACGGTCGT
CAAAACGCGCACCTACGGCAAGAACGACATCCGCATTCTGCATGGCAAGGTTTGCCTCGT
AAGTACCGTGCATACCGAGCATACCGAGGAATTGGCGGTCGCCGGAAGGATAAGCGCCCA
AGCCCATCAGCGTACCCGTGCACGGAGCACCCGTCATTCGGACAAATCGGGTCAGCTCTT
CAGAAGCATTACCCAACACCACGCCGCCGCCAAAATAGACGACCGGACGTTTGGCAGATG
CCAACATCTGCACGGCCTTTTTAATCTGACCGATATGTCCTTGAACAACCGGTTGATACG
AACGGATAAAAATGTCTTCCTGAGGATAGCTGAATTTCGCCATCGCCTGCGTAACATCTT
TCGGGACATCAACCACCACGGGCCCCGGTCGGCCGCTTGCGGCAATTTGGAACGCCTTTT
TAATGGTTTCCGCCAACTCATTGATGTCCGTAACCAGGAAATTGTGTTTGACGCACGGAC
GGGTAATACCCACCGTATCAACTTCTTGGAACGCATCCGTACCAATCAGGGAATTGCCTA
CCTGCCCGCTGATGACCACCATCGGAATCGAATCCGTATAGGCAGTAGCAATACCGGTCA
GTGCATTGGTAACGCCCGGGCCGGATGTAACCAATGCCACGCCCACCTTACCGCTGACGC
GCGCATACGCATCTGCCGCGTGTACTGCCGCCTGCTCATGGCGGGTAAGAATGTGTTTGA
ATTTATTGAGTTGGAAAAGGGCATCGTAGATTTCGATAACCGCACCGCCGGGATAACCGA
AAACGTACTCGACACCTTCGGCTTTGAGACTCTGCACTATGATTTGCGCGCCTGATAACT
GCATAACGACCTCTTTTATACGGTTTCAAACCAATAGGGACAAACCGCTTTGCCACAGCA
CCTGTAATGCAATTCCACCAAGCAGCGATTTAGGGTACGCGCATTGGGGGAACACGGCAA
CAGACGGATTATCCAATCAATTGGAAAGGAACACAGAGTTTGTGAAAAAGAGTAGAAACG
ATAACGCAAACCGACAGTTCAATCAAGAAAAATCTTTCATCTTTTAATATTTTTTGAAAG
CAGAGAAATTATTGATTGATTTTAAAAGAATAAAATCAGGAGTACCTTTTTTGAAAGATG
GAAATTGTTGACAGTTTGTGTAGGAGGGGCAGATGTGAAAAACCCTTCTTCGATATCAAG
AATTGTAAAATTTACAGGGTTTCATCCCAATAAAGACTCGGGATATTGATTGAACTTGAT
TTTATTTTTGATATATCAAAAATATTCCCAACCATACTTCCTGAAAATGGCTCATTGCAC
CGGACTGTATTGGACGGCATTGACAGAACCAAGAGGGCTAACAACGACTTAATATATTGA
TTGTATAGTGGATTAACAAAAATCAGGACAAGGCGACGAAGCTGCAGACAGTACAAATAG
TACGGAACCGATTCACTTGGTGCTTCAGCACCTTAGAGAATCGTTCTCTTTGAGCTAAGG
CGAGGCAACGCCGTACTGGTTTAAATTTAATCCACTATATTTAGTTTTATCTATTTCATT
AAACAGCAATAGACAAAAAAAATAACCGCTCTAAAAGCGGTTGTGGTGCCCAGGGTCGGA
CTCGAACCGACACACCTTGCGGCGGGGATTTTGAGTCCCCTGCGTCTACCAATTTCGCC
ACCTGGGCTGGTGAAGAAGTCGTCATTATAATGGCTTTTGAAATTCTGTAAACCTTTTTT
TTGAAATTATTTTATCTGTTTTTATTTTATTTTTGATTTTAAATAGAATTTTTATTATTT
TAATCTTACTGTTCTTTCCGCTCCAAAGATTCTGTATGATTCGGCAATTCCTGCCGTGCA
GACAACGTAAAAAAATACTACATTAAATCTGCCAAACGCGTTAAGATGGAAATATTCAAA
TTCCGTACGAATCAGGTTTTGCTATTTATTCTTGGGAGATTGTCATGTTTTCCGTACCGC
GTTCCTTTTTGCCGGGCGTTTTCGTACTTGCCGCGCTTGCCGCCTGCAAACCTCAAGACA
ACAGTGCGGCGCAAGTCGCTTCTTCAAGTGCATCCGCGTCGGCTGCGGAAAATGCGGCAA
AGCCGCAAACGCGCGGTACGGATATGCGTAAGGAAGACATCGGCGGCGATTTCACGCTGA
CCGACGGCGAAGGCAAGCCTTTCAACCTGAGCGATTTGAAAGGCAAGGTCGTGATTCTGT
CTTTCGGCTTTACGCACTGTCCCGATGTCTGCCCGACAGAGCTTTTGACGTACAGCGACA
CGTTGAAGCAGTTGGGCGGGCAGGCTAAGGACGTGAAAGTGGTGTTCGTCAGCATCGATC
CGGAACGCGACACGCCTGAAATCATCGGCAAGTATGCCAAACAGTTCAATCCGGACTTTA
TCGGTCTGACGGCAACGGGCGGCCAAAACCTGCCGGTCATCAAGCAGCAATACCGCGTGG
TTTCTGCCAAAGTCAATCAAAAAGACGACAGCGAAAACTATTTGGTCGACCACTCTTCCG
GTGCGTATCTCATCGACAAAAACGGTGAGGTTGCCATTTTCTCGCCTTACGGAAGCGAGC
CGGAAACGATTGCTGCCGATGTAAGGACCCTGCTCTGATAAAACCGTATGCCGTCTGCAC
CGTCGGCGCCTATTCAGACGGCATTATTGTTTCAACCGACAAAGGACATCCACACCATGC
AGGATAATGCTTTGACCATCGCCTTATCCAAGGGGCGCATTTTTGAGGAGACGCTGCCGC
TGCTTGCCGCTGCCGGCATTGTTCCGACTGAAGAGCCTGAAAAATCGCGCAAGCTGATTA
TCGGGACGAACCATGAAAACATCCGCCTTGTCATTGTCCGCGCAACCGATGTGCCGACTT
ATGTCCGCTACGGCGCGGCGGACTTCGGCATTGCGGGCAAAGACGTGCTGATCGAACACG
GCGGCACGGGGCTTTACCGGCCTTTGGATTTGGAGATTGCCAAGTGCCGCATGATGGTTG
CTGTGCGTAAAGGGTTTGATTACGAAGCAGCTTCGCAACCCGGATGCCGTCTGAAGATTG
CCACAAAGTATCCTGAAATCGCGGCATCTCATTTTGCCGGCAAGGGTGTCCATGTGGACA
TTATCAAACTGTACGGCTCGATGGAACTTGCGCCGCTGGTCGGCTTGAGCGATGCGATTG

## Appendix A

TGGACTTGGTTTCGACGGGCAACACCTTGAAGGCAAACGGCTTGGAAGCAGTCGAACACA
TCGTCGACATTTCCAGCCGCCTGGTGGTCAACAAGGCTGCTTTGAAAACGAAATACGCGC
TGCTGGAGCCGATTATTCAGGCGTTCGGCGGCGCAGTGAAGGCGAAGTAAGCATCCATTT
GAATAAAGATGCGTTTTCAGACGACCCTATCCGTTCCCGCCGACAGGTCGTCTGAAAATA
TCACCGGCAGTAAACTGTATAGGAGAAGTTAAAATGGTTGCAAAAATAAAAAAATTCTCA
GATTCAACCCTTTCCGTTTTGAATAACGGCGAGCGTCGGTTTTATGTCTATTGTCTGACC
GACCTGAAAAAAGACAAAATCCTCTACATCGGCAAAGGCTGCGGTAATCGTATCTTCGAG
CATGAATGGGTTGCTAGTCGTTCACAAGATCCAGTCTCCGGCGAGATTATCGATCGGAAA
CTCAAAGCCATCTCCAAATGCAAGAAACTCGGTCGCTATATCATCAGCTATCATCTGACT
GAAGTCGAAGCACTCGCCGCCGAATCTGCCTTAATTCATTTTGTTAAATCTGTCTTGGGT
AAAAAACTCAAAAATAAAATTGCCGGGCATGGTCCGGGTGGTATTAGCGTAGAAGAACTA
GATCGCCGCTTTGGATTCTCTTCTCTCCCACTTAACGAGATTAACCCCGACGGGCTGATT
CTCGCCATCAAAATCCACAATGCTTTCGATTTAGATACTGACGAAGAATTAGACTACCTT
TTCGACAACCAAGACGATGCCAACCTCAAATCGCGTACGTTGGGCAACTGGGTTATCGGT
AAAGATGTTGCTTCAAAAGTGAAATACGTTATCGGCGTTCACACCGGTCTGCAAAACGCT
GTTGTCAGTGCATACGAAGTGGACGGTTTTGAAACAATGGTTGAGGAAACCAAAAACGGT
AGAAAACAATCCCGTTACCGTTTCCGCACTACCTCTCGTAGCGAAGAGGTATTAGCCAAA
CTCGGTCTGCAACAAAAATGCCTGCCCGAATTGAAGTTTGGTAGCGGGGGAGAAAAAGCG
TATATCAGACCCAAAACAGAGACAGAAACTGAACAAGAGAATATTCAGACGACCCCCAAT
CCAAAAATAAAAAAGGAAAAAACCAAATCATGAAAAAACTCAACACCCAATCGCCCGATT
TCCAAGCCGGACTCAAAGCCCTGCTGGCTTTTGAAACCGCGCAAAACCCCGAAACCGAAC
GCATCGTCGCCGACATTTGCGCCGACGTGCAAAAGCGCGGCGATGCGGCTTTGATTGAAT
ACACCAACAAATTCGATCAGACAAACGCTAAAAGCATCGATGATTTAATACTCACGCAAG
CCGATTTGAACGCGGCGTTCGAGCGCATTCCGAACGACGTTCAGACGGCATTGCAGACCG
CCGCCCGCCGTGTCGAAAGCTACCACCAACGCCAAAAAATGGAATCGTGGAGCTACACCG
ATGAAGACGGCACGCTGTTGGGACAACAAATCACACCGCTTGACCGCGTCGGCATTTACG
TCCCCGGCGGCAAGGCGGCGTATCCGAGTTCCGTCATCATGAACGCCATGCCCGCCCACG
TCGCAGGTGTGAAAGAAATCATCATGGTCGTGCCGACACCAAAAGGCGAACGCAACGACA
TCGTACTTGCCGCCGCATACGTCGCCGGCGTAACCAAAGTCTTCACCGTCGGCGGCGCGC
AGGCGGTTGCCGCCCTCGCCTACGGCACGGAAACCATCCCCCAAGTCGATAAAATCACCG
GTCCGGGCAACGCCTTCGTCGCCGCCGCCAAACGCCGCGTGTTCGGCGTGGTCGGCATCG
ACATGGTGGCGGGGCCGTCTGAAATCCTGGTCATCGCCGACGGCACGACACCTGCCGATT
GGGTGGCGATGGATTTGTTCAGCCAGGCCGAACACGACGAAATTGCCCAAGCCATCCTCA
TCGGCACGTCGCAAGCGTATCTCGACGAAGTAGAAGCCGCTATGGACCGCCTGATCGAAA
CTATGCCGCGCCGCGACATCATCGAAGCCTCGCTCGGCAACAGGGGCGCGATGATACTCG
CCAAAGACTTGGACGAAGCCTGCGAAATCGCCAACTACATTTCCCCCGAACACTTGGAAC
TGTCAGTCGAAAACCCGCAGGAATGGGCGAAAAAAATCCGCCACGCCGGTGCGATTTTCA
TGGGACGCTACACCGGCGAAAGCCTCGGCGACTACTGCGCCGGTCCAAACCATGTGTTGC
CCACCAGCCGAACCGCCCGCTTTTCCTCGCCTTTGGGGACATATGATTTCCAAAAACGCT
CCAGCCTGATTCAGGTTTCGGAACAGGGCGCGCAAAAATTAGGCGAAACCGCCAGCGTGC
TGGCACACGGCGAAAGCCTGACCGCCCACGCCCGCGCGGCAGAGTTCCGTATGAAATAAT
GCCGAAACGGCGTACAGGCATATTCCAACCATTAAGGAAACACGATGAAATCCGTCCGCT
CCTTCATCCGCGACGACATACAAGCTATGTCGGCATATCAGATTGCCGACGTTCCGCCCG
GCTTTGCCAAACTCGATTCGATGGAAAGTCCCGTCCACCCTTTTGCCGGACATGAAACGC
TGTTGCAGGAATGGCAGGCACGGCTTGCCGCCGCGCCCATCCATCTTTACCCCAATCCCT
CCGGCAGCGGTTTACAGGAAGCATTACGTTCGGCGTTCGACATTCCCGACTGCGCCGACA
TCGCGCTGGGCAACGGTTCGGACGAACTGATACAGTTCATCACGATGCTGACCGCCAAAC
CGGGCGCGGCAATGTTGGCAGCCGAACCCAGTTTCGTCATGTACCGCCACAACGCCGCGC
TGTACGGCATGGATTATGTCGGCGTTCCACTGAACGGAGATTTCACCCTCAACCTGCCCG
~~CCGTCGTGGAAGCCGTCAGGAAAGAGGCGGTGGCGTGAGCTTTATCGCCTACCCCAACA~~
ACCCCACCGGCGTATGCTTCACGCGTGCCGAAATCGAAGCCGTCATCGAAGCTTCAGACG
GCATCGTCGTCGTCGATGAAGCCTACGGCGCATTCAACGGCGACAGCTTCCTGCCGCAGG
CAGGCAGGATTCCCAACCTGATAGTCTTACGCACCCTCAGCAAAATCGGTTTTGCCGGAC
TGCGTATCGGTTATGCGGCAGGCTGCCCCGAAGTCATCGGCGAACTGCAAAAAATCCTGC
CGCCCTACAATATGAACCAATTGAGCCTGACCACTGCCAAACTCGCCCTGCGGCACTACG
GCATTATCTCTGCCAACATCGACAGCCTGAAAAACGAACGCGAACGGATGTTCGCCGAAT
TGGGCAAAATATGCCGTCTGAACACCTTTTCAAGTCAGGCAAACTTCATTACCATACGCG
TACCCGATGCCGATTTGTTGTTTGACACGCTCAAACAAAACCGCATCTTGGTTAAAAAAC
TGCATGGCGCGCACCCGCTTTTGGAACACTGCCTGCGCATTACCGTAGGCAGCCCCGCAC
AAAACGATGCCGTTCTCAACATCATTCGCCAACTTTACTGCCAACCAACGGATTTCCTAT
GAATTTGACTAAAACACAACGCCAACTGCACAACTTTCTGACCCTCGCCCAAGAAGCAGG
TTCGCTGTCCAAGCTCGCCAAACTCTGCGGCTACCGTACCCCCGTCGCACTCTACAAACT
CAAACAACGCCTTGAAAAGCAGGCAGAAGACCCAGATGCACGCGGCATCCGTCCCAGCCT
GATGGCAAAACTCGAAAAACACACCGGCAAACCCAAAGGCTGGCTCGACAGAAAACACCG
CGAACGCACTGTCCCCGAAACCGCCGCAGAAAGCACCGGAACTGCCGAAACCCAAATTGC
CGAAACCGCATCTGCTGCCGGCTGCCGCAGCGTTACCGTCAACCGCAATACCTGCGAAAC
CCAAATCACCGTCTCCATCAACCTCGACGGCAGCGGCAAAAGCAGGCTGGATACCGGCGT
ACCCTTCCTCGAACACATGATCGATCAAATCGCCCGCCACGGCATGATTGACATCGACAT
CAGCTGCAAAGGCGACCTGCACATCGACGACCACCACACCGCCGAAGACATCGGCATCAC
ACTCGGACAAGCAATCCGGCAGGCACTCGGCGACAAAAAAGGCATCCGCCGTTACGGACA
TTCCTACGTCCCGCTCGACGAAGCCCTCAGCCGCGTCGTCATCGACCTTTCCGGCCGCCC
CGGACTCGTGTACAACATCGAATTTACCCGCGCACTAATCGGACGTTTCGATGTCGATTT
GTTTGAAGAATTTTTCCACGGCATCGTCAACCACAGTATGATGACCCTGCACATCGACAA
CCTCAGCGGCAAAAACGCCCACCATCAGGCGGAAACCGTATTCAAAGCCTTCGGGCGCGC
CCTGCGTATGGCAGTCGAACACGACCCGCGCATGGCAGGACAGACCCCCTCGACCAAAGG

## Appendix A

CACGCTGACCGCATAAAAAACCATACCGTCTGAAACACCCGCAGGCTTTTCAGACGGTAT
CGGAACAGATAAGATTACACTACACTACAAACAGAAAAGGAGTAAACATCATGTCCGCAA
ACGAATACGCACAAATCGGCTGGATAGGCTTAGGGCAAATGGGTCTGCCTATGGTAACGC
GGCTCTTGGACGGCGGCATCGAAGTCGGCGTATACAACCGCTCGCCCGACAAAACTGCCC
CCATCTCCGCCAAAGGCGCAAAAGTTTACGGCAACACCGCCGAACTCGTCCGCGACTATC
CCGTCATTTTCCTGATGGTTTCCGACTATGCCGCCGTGTGCGACATCCTGAACGGAGTCC
GCGACGGATTGGCCGGCAAAATCATCGTCAACATGAGCACCATCTCCCCGACCGAAAACC
TCGCCGTCAAAGCACTTGTCGAAGCCGCAGGCGGACAGTTTGCCGAAGCACCCGTTTCCG
GATCGGTCGGGCCCGCCACCAACGGCACGCTGCTGATTCTGTTCGGCGGCAGCGAAGCcG
TTTTAAACCCGCTGCAAAAAATATTTTCCCTCGTCGGCAAAAAAACCTTCCATTTCGGCG
ATGTCGGCAAAGGTTCGGGCGCGAAACTCGTCTTGAACTCGCTCTTGGGCATTTTCGGCG
AAGCGTACAGCGAAGCGATGCTGATGGCGCGGCAGTTCGGCATCGATACCGACACCATCG
TCGAAGCCATCGGCGGCTCGGCAATGGACTCGCCCATGTTCCAAACCAAAAAATCCCTGT
GGGCAAACCGCGAATTCCCGCCCGCCTTCGCCCTCAAACACGCCTCCAAAGACCTCAACC
TCGCCGTCAAAGAGCTTGAACAGGCAGGCAACACCCTGCCCGCCGTCGAAACCGTTGCTG
CCAGCTACCGCAAAGCAGTCGAAGCCGGCTACGGCGAACAGGACGTTTCCGGCGTTTACC
TGAAACTGGCAGAACACTGATTGCCTTTTCCAAACACAATGCCGTCTGAACATATTTCAG
ACGGCATTTTTATCACCCCACGCTTAAAATCAGTCCCGATTATGACTATATAGTGGATTA
ACAAAAATCAGGACAAGGCGACGAAGCCGCAGACAGTACAAATAGTACGGAACCGATTCA
CTTGGTGCTTCAGCACCTTAGAGAATCGTTCTCTTTGAGCTAAGGCGAGGCAACGCCGTA
CTGGTTTTTGTTAATCCACTATAATCCGCACAAATTTAGTCAATATCAAGACCAATTATG
AACCAACTCGACCAACTTGGCACCCGTATCAACCTGATTTGCAATGTCTTCGACAAATGG
ATCGGGCAGCAGGATCTGAATTACAACCTCTTTGCCGTACTTTATACCCTGGCAACCGAA
GGCAGCCGCACGCAAAAGCATATCGGCGAAAAGTGGAGCCTGCCCAAACAGACCGTTTCA
GGCGTATGCAAAACCCTTGCCGGACAAGGGTTGATTGAATGGCAGGAAGGCGAACAGGAC
CGGCGCAAACGGTTGCTGTCGTTGACCGAAACAGGCAAAGCCTATGCCGCACCTTTAACA
GAAAGCGCGCAGGAATTCAGCGACAAAGTATTTGCCACATTCGGCGACAAGCGCACAACT
CGGCTGTTTGCCGATTTGGATGCACTGGCTGAAGTGATGGAAAAAACAATCTCGGAAAAT
AAAAAATAGGGGGCAAATATGTGGAAAATGTTGAAACACATAGCCCAAACCCACCGCAA
GCGATTGATTGGCACATTTTCCCTGGTCGGACTGGAAAACCTTTTGATGCTGGTGTATCC
GGTGTTTGGCGGCCGGGCGATCAATGCCGTGATTGCGGGGGAGGTGTGGCAGGCGTTGCT
GTACGCTTTGGTTGTGCTTTTGATGTGGCTGGTCGGTGCGGTGCGGCGGATTGCCGATAC
GCGCACGTTTACGCGGATTTATACCGAAATCGCCGTGCCGGTCGTGTTGGAACAGCGGCA
GCGACAAGTCCCGCATTCGGCGGTAACTGCGCGGGTTGCCCTGTCGCGTGAGTTTGTCAG
CTTTTTTGAAGAACACCTGCCGATTGCCGCGACATCCGTCGTATCCATATTCGGCGCGTG
CATCATGCTGCTGGTGCTGGAATTTTGGGTCGGCGTGTCGGCGGTGGGCATACTTGCGTT
GTTTTTATGGCTTTTGCCACGTTTTGCCGCCATCAGCGAAAACCTGTATTTCCGCCTGAA
CAACAGCTTGGAACGCGACAACCACTTTATCCGAAAAGGCGACCGGCGGCAGCTGTACCG
CCATTACGGACTGCTTGCGCGCCTGCGTGTGCTGATTTCCAACCGCGAAGCCTTCGGCTA
TCTCTGCGTCGGCACGGCGATGGGTATTTTGTTCGGCTTTGCTTTTGTGATGATGACGCT
CAAAGGCTACAGCAGCGCGGGGCATGTCTATTCGGTCGGCACTTATCTGTGGATGTTTGC
CATGAGTTTGGACGACGTGCCGCGATTGGTCGAACAATATTCCAATTTGAAAGACATCGG
ACAACGGATAGAGTGGTCGGAACGGAACATCAAAGCCGGAACTTGAAAAATGCCGTCTGA
ACACGCTTCAGACGGCATTTCCATCCGTTCGGCAAACTACATCACATCCGCCCGCCGGTT
GACAAGTTTGGCAAACAACTTTTCAACAGAAGCTTCCGCCTGCAAACCAATGCGCTGGAT
CAGGCTTTGCTTCTCCTGATATTTCACTTCGATAACCTGTTTGTTTTCAAACGCTTTCAA
CAACAAATCATCACTGGTCGAAATCTCGTCAATCAAGTTCAACGCCAACGCCTGCCGACC
GAACCAATGCTCGCCCGTTGCCACTTCCTCAATATCCAATTGAGGGCGGTTCTCGCTGAC
AAACTGCTTGAACAACTGATGCGTTTCCTCCAGTTCCTGTCGGAATTTCTGTTTGCCCTT
TTCCGTATTTTCACCCATAAAAGTAACCGTGCGCTTAAATTCGCCCGCCGTCATCACATC
CACATCAATATCATGTTTTTTCAACAGGCGGTGGATATTCGGTACTTCCGCCACCACACC
CACCGAACCGACAATCGCAAACGGAGCGGAAGCAATTTTATCCGCCACACACGCCATCAT
ATAACCGCCGCTCGCCGCCACCTTATCGACGGCGACGGTCAGCGGAATATTGCGTTCGCG
CAAACGCCTAAGCTGCGAAGCCGCCAAACCGTAACCGTGAACCACGCCGCCCGGACTTTC
CAATCTGAGCAGAACCTCATCTTCAGGCTTGGCAATCAAAAGCACCGCCGTAATCTCATG
ACGCAAGGATTCTACGGCGTGTGCATACAAATCGCCGTCAAAATCCAACACAAAAAGGCG
GGATTTTTGCGTTTCGGCAGATTTCTCCCCACCCTCCTTCAAACGCTTTTTCTCTGCTTT
GGCTTCCGCCTTTTCCTTTTTCTTTTCCTCTTTTTCCTGATGTTTTGCCTCTTCCCCGCT
TAAAAAGAATGCTTCAAACGATTGCCGCTGTTTTTTATAATTTTCCGAAAAATCCGTCAG
TACGACACTGCCGCTTTCCGACTGTTTCTTACTCTGTACGATAGCCAACACAATCAGCGC
AATTGCGCCGAACACGGTAAGCAGTTCGAGCAGGAAAATACCGTAATTCAGTAAAATTTC
TTTCCACATTGATTGGATTTCCTCTTGTTCAGGCATGAACATGTCAATATTGTCCATCAC
CGTCCGACAGATAAAAAAATAACCGCTTGGAGCGGCATTGTCATTTTCAGCTTGGTGCCC
GGAGCCGGAATCGAACCGGCACGGGATGTTTAGTCCCGACGGATTTTAAGTCCGTTGTGT
CTACCTATTTCACCACCCGGGCATTTGTGAAAGGTGGAGGCGGGGGCGCGGATTTTAACC
GGCCTGTATGAAGATTGCACTCCTCATAGCATAAACACTCTGCCACCCCGCCATAGTACG
ATAATGGAGGCGAGAGTCGGAATCGAACCGGCGTAGACGGATTTGCAATCCGCTGCATAA
CCACTTTGCTATCTCGCCCTAAAACTGGCTTATCTAAAAAACTTGGAGCGGGAAACGAGT
CTCGAACTCGCGACCTCAACCTTGGCAAGGTTGCGCTCTACCAACTGAGCTATTCCCGCG
CGTTCAAACATATCGGTTTTTGGAGCGGGAAACGAGTCTCGAACTCGCGACCTCAACCTT
GGCAAGGTTGCGCTCTACCAACTGAGCTATTCCCGCGTTGATATGTTTGAAATAAAACTT
GGAGCGGGAAACGAGTCTCGAACTCGCGACCTCAACCTTGGCAAGGTTGCGCTCTACCAA
CTGAGCTATTCCCGCAATGATTGCGGAAGAATGAAATTTTTGGAGCGGGAAACGAGTCTC
GAACTCGCGACCTCAACCTTGGCAAGGTTGCGCTCTACCAACTGAGCTATTCCCGCCCGA
TTTCATTCTCCGATATCGAAGAGACACAATTATTATGGATTCTGTTTTTGCCGTCAAGCT

## Appendix A

ATTTTTATGTTTTTTTCAGGCGATTTCTTTCCACGCCATTTTCAGATAATACAGCATCGA
CCAGACTGTCAGCAAAGATGCGATAAACATCAATACATTGCCGATGAATGCGAGGTTAAA
TCCATAAAAATCGGGAAAATTCAGCAGCAGCAGGAAGATTGCCAGCATTTGCGCGGCGGT
TTTAAACTTACCGACGGTGGCGACGGCAACGCTGTTCCTTTTGCCCATTTGCGCCATCCA
TTCGCGCAATGCGGAAATGGTAATTTCCCTGCCGATGATGATCATGGCAAACAAAACATA
GGTCCGGTCGAGTTTGACCAGTAAAAGCAAAGAGACGGCGACCATCAGCTTGTCGGCAAC
GGGATCGAGGAAGGCGCCGAAATCCGAGGTCTGTTTCCACAACCTTGCCAAAAATCCGTC
AAACCAGTCGGTCAAGGCGGCAACGGCAAAAATGACGGCGGCGGTGAGATTAATCGTTTC
CTCCGCGAACCACGGAAAAGGCAGGTAAAAAAGGGCTGTCAGGACAGGAATGAGCAAGAC
CCTCAACCATGTGAGGAAGATGGGGAGATTCCAAGGCATCGGTTTTCTCTGTGCAGACTG
TAAAGTTGTGATTATAACGGTTATCCTCATAACCCAAAACGTAAAATTGCTGCATGGGCA
TTCCCCCGCCCCGCCAATCTGTTTTCACATTCTTTTCAAACGCAGGAAAATGGCGGGCAA
TAAAAGCAAAATACCCAGTTTCAGGCTGAAAACGGCAGGTTGTGCCAACACTTCGACAAG
GCGGTCTTCCGTGCGGGCAAAATCTTTATTGCTTATAGACACTGCCACTGTTGCGGTATT
CCAACAGAACGCCGTTTAAAAAACCTTTGCCGACGGTTTCGCTTAAAACGGCTCTAACCT
GCTCCGCCCTGATGGTTCTGCCGATATTGCCGCCTGTGCACAAACTGTCGAACCCATAGC
AGGAAAGCCGGTAATGCTGCCCGTCTGCATCCAGTTTGATTGCCCGTCCGCTGCGGTTGA
GGGCGGTAACGGTCAATTCCGCATATTCGAATGTTTTTTCTTGTTCGTGAAATGCCGTCA
GGTAAGGTGCAATAAAAACGGCGGACAACAGCAGACAGCTTATGGCGGCAAACCATACCC
AGCGATAATATAGTGGATTAAATTTAAACCAGTACAGCGTTGCCTCGCCTTAGCTCAAAG
AGAACGATTCTCTAAGGTGCTGAAGCACCAAGTGAATCGGTTCCGTACTATTTGTACTGT
CTGCGGCTTCGTCGCCTTGTCCTGATTTAAATTTAATCCACTATATTTCACGCTTACCCC
TTGTTTCTCAAATGCCGTCTGAAATAAGCGGCTTAATATATTGTTTACAGTATTGGGAAG
CATAACAGACAAAATGCCGTCTGAAATATTTTCAGACGGCATTTCTTATCCGAAACGGAT
TATTTTTGCGTTTCAACCGCTTCCAATGCACGCAGGGCATAAGTGTAAGCGGCACCCGCA
TTCAGGGCAATGGCGGTTGCCAATGCACCTGCGATTTCGCTGTCGGTCGCACCGGCTTTG
GTGGCGGCGGCGGCGTGAACACTGATGCAGCTCTCACAACGTGTAGTAATGGCAACGGCG
ATGGCAATCAGTTCGCGTGTTTTAGCATCAAGTGCCTCTGCAGCTGCCGCTTGTTCCAAT
GCGCCGTAGGCCTGCAGCATTTTAGGATGCGCCTTACCCAGCTCGCCGAACGATTTTTTA
ACCAATGCGGTATGTTCTTTCCAATCTTTAAACATTTTCTTTTCCTTTCTCTTGCGTTTA
ACCCTGATACGCGCTTGCGTATCTGTTTTCGATGTGCGTATTATTGCAATTATTCAGTTG
TGTTTCTCGTTTAATCATCTCATTTTATGGTTCAAAAAGATTTATGGACATTCTGGACAA
ACTGGTCGATTTCGCCCAATTGACGGGCAGTGTGGATGTGCAGTGCCTTTTGGGCGGACA
ATGGTCGGTACGGCATGAAACCTTGCAACGCGAAGGATTGGTACACATTGTTACATCGGG
CAGCGGCTATCTCTGCATCGACGGCGAAACTTCCCCGCGTCCGGTCAGTACAGGGGATAT
TGTATTTTTCCCGCGCGGCTTGGGTCATGTGTTGAGCCACGACGGAAAATGCGGAGAAAG
TTTACAACCGGATATGCGGCAGCACGGTGCGTTTACGGTCAAGCAGTGCGGCAACGGACA
GGATATGAGCCTGTTTTGCGCCCGTTTCCGCTACGACACCCACGCCGATTTGATGAACGG
GCTGCCTGAAACCGTTTTTCTGAACATTGCCCATCCGAGTTTACAGTATGTGGTTTCAAT
GCTGCAACTGGAAAGCAAAAAACCTTTGACGGGGACGGTTTCCATGGTCAACGCATTGTC
GTCCGTCCTGCTGGTGCTTATCCTGCGCGCCTATCTCGAACAGGATAAGGATGTCGAACT
CTCGGGCGTATTGAAAGGTTGGCAGGACAAACGTTTGGGACATTTAATCCAAAAGGTGAT
AGACAAACCGGAAGACGAATGGAATGTCGACAAAATGGTGGCGGCTGCCAATATGTCGCG
CGCGCAACTGATGCGCCGTTTCAAAAGCCGGGTCGGACTCAGCCCGCACGCCTTTGTGAA
CCATATCCGCCTGCAAAAAGGCGCGTTGCTGCTGAAAAAAAACCCGGATTCGGTTTTGTC
GGTCGCACTGTCGGTAGGCTTTCAGTCGGAAACGCACTTCGGCAAGGCGTTCAAACGGCA
ATATCACGTTTCGCCGGGTCAATACCGGAAAGAAGGCGGGCAAAAATAAATCGGGGCTTC
AAACGCAAATGCCGTCTGAAAAGGCTTTCATACAGCATTTGCGTACCGCGTCATTTCAAG
GGCTGCATCTTCATCACTTCCATCAAAAAGTTGGTAAATGCGGGGTTGTTGGGTTTGACA
TCCATATTTTTCCAACGCTGCTGCCAGCCGCGCAAGGCATTCTGGATATACAGCTTGGAC
TGTTCCGTATTGATTGCGCCCCGCTGGCTGTCTATCGCCGAACGCAGGTAGATTTCATAC
ATACTGTCATCGACGGCATTGCGTCCGACCAGGCGTTTTCTGAAGTTGTTCAGATATTGC
GCCGCCTGAACCTTGGTCATTTTACCGATACCCACCTGATAGCCCAAGCGCGTCGCTTCA
TCGCTGATTTTGGCAACATCCGTCCAATGCGAAGAGGCAAGGCGGAAACCTTTTGCAGGT
GCTTCCGTTTTGACGGTATTGATAGGATTCACGGGGATTTCCGTCAATGTGGGCACATAA
ATAGACTGGCAGCCGGAAAGAACTGCCGCAATGGAAAGAGGGATAAGGTATTTTTTCATG
CCCCCATTATAATCAAGTTTGCCTTGAGAAAACAAATTGTTCGGCAAGAAAAATAAAATT
TCGGCATCAGAAGCAGGCAAAAACACATTCCACAAGCCTTGCCGCAAGGTTTACAATCCG
ACCGTCCTTATCGCAACGACCGTTTATGGATACCGCAAAAAAAGACATTTTAGGATCGGG
CTGGATGCTGGTGGCGGCGGCCTGCTTTACCATTATGAACGTATTGATTAAAGAGGCATC
GGCAAAATTTGCCCTCGGCAGCGGCGAATTGGTCTTTTGGCGCATGCTGTTTTCAACCGT
TGCGCTCGGGGCTGCCGCCGTATTGCGTCGGGACACCTTCCGCACGCCCCATTGGAAAAA
CCACTTAAACCGCAGTATGGTCGGGACGGGGGCGATGCTGCTGCTGTTTTACGCGGTAAC
GCATCTGCCTTTGGCCACTGGCGTTACCCTGAGTTACACCTCGTCGATTTTTTTGGCGGT
ATTTTCCTTCCTGATTTTGAAAGAACGGATTTCCGTTTACACGCAGGCGGTGCTGCTCCT
TGGTTTTGCCGGCGTGGTATTGCTGCTTAATCCCTCGTTCCGCAGCGGTCAGGAAACGGC
GGCACTCGCCGGGCTGGCGGGCGGCGCGATGTCCGGCTGGGCGTATTTGAAAGTGCGCGA
ACTGTCTTTGGCGGGCGAACCCGGCTGGCGCGTCGTGTTTTACCTTTCCGTGACAGGTGT
GGCGATGTCGTCGGTTTGGGCGACGCTGACCGGCTGGCACACCCTGTCCTTTCCATCGGC
AGTTTATCTGTCGTGCATCGGCGTGTCCGCGCTGATTGCCCAACTGTCGATGACGCGCGC
CTACAAAGTCGGCGACAAATTCACGGTTGCCTCGCTTTCCTATATGACCGTCGTTTTTTC
CGCTCTGTCTGCCGCATTTTTTCTGGGCGAAGAGCTTTTCTGGCAGGAAATACTCGGTAT
GTGCATCATCATCCTCAGCGGTATTTTGAGCAGCATCCGCCCCACTGCCTTCAAACAGCG
GCTGCAATCCCTGTTCCGCCAAAGATAAAAAATGCCGTCCGAACATCCTTCAGACGGCAT
ATCGGGCTTTATTTCCCCGCCTTCACATCCTGCCACTGGCGCACCATAAACTTCAATGCC

# Appendix A

GCCGGCTGGATAGGCACCATGATAAAGCTGTTTTTCAAATCCTCCTCGGTTGGGAAAATC
GTATTGTCGTTTTTAAATTCGTCTTCCATCAGCTCACGCGCAGGCTTGCTCGAAGGCGCG
TAAGTAACGAAATTGCCGTTTTTCGCCGACACTTCCGGGTCGAGGAAGTCGTTGATGTAT
TTGTGCGCGTTGGCGACGTTTTTCGCATCTTTCGGAATCACGAAAGAATCCACCCAAATC
CCCACGCCCTCTTTGGGCATCATCACGCGGATTTTTTCCTTGCCGCCCGCTTCTTCGGCA
CGGCGTTTGGCGATGTTCAAATCGCCGCCGAAACCGATTGTTACGCAGGTATCGCCGCGC
GCCAAATCATCGATAAAGCCGGACGAAGTAAAGCGTTTGATATTGGGGCGGTTTTTCTTG
AGTAGGGCGGTTGCCTCCCTGATGTCTTCCGTATTGCTGCTGTTCGGGTTTTTACCCAAA
TAGTTCAACACCATAGGATAGATTTCCGCCGCGCTGTCCAAATAGCTGATGCCGCATTGC
TTGAGTTTGGACGTGTATTCGGGGTCGAACACCAAATCCCACTGGTTGTCCGGCAGCTTG
TCCGTACCCAAAGCCTTTTTCACGCGTTCGGTATTGATGGCGAAGGTATTTGTCCCCCAA
TAAAACGGCACGGCGTATTCGTGGCCGGGATCGACCCCGTCCATCAGCCTCATCATTTCG
GGGTTGAGGTGTTTATAATTGGGAATCAGCGACTTATCGATTTTCTGATACGCACCTGCC
TTAATCTGCCTGCCCACAAACGCATTGGACGGCGCGACAATGTCGTAACCGGACTTGCCT
GTCAGCACCTTGCTTTCCAGCGTTTCATCGCTGTCGTACACATCATAAGTAACCTTGATG
CCGTTTTTCTTTTCAAAATCGGCAACGGTTTCCGGATCGACATATTCCGACCAGTTGTAA
ATTTTCAATACGTTTTGGTTTTCCGCCGGTGCCGGTTTTTCGGCAGGCGGTTTGTCCGAA
CCGCCGCACGCTGCAAGCAGCAAAGCAGTCAGGACGGCCAGGGGCAGATGTTTGGTCATT
ATCATTCCTTGCATATCGGGTTGGAGAAAGCGGCCATTATAGCCGATATTGGCAACAGGG
CTTCAGACGGCATTCAAAATCCCGCCACACTCTTCCGAAAACCGCCGCTTCCATAGCTAG
AAACAGGGATTTGCGGTAAGATACCGCCGTTCGTTTTCCCTGCTTTTACCATGACAAGAC
ATTTGAGAGACATTGAAAAAATTATGAAAACCTCCGAACTGCGCCAAAAATTCCTAAAAT
TTTTTGAAACCAAAGGCCACACCGTCGTCCGCTCTTCCAGCCTCGTGCCGCACGACGACC
CGACCCTGCTGTTTACCAACGCGGGCATGAACCAGTTTAAAGACGTATTCTTAGGTTTCG
ACAAACGCCCGTACAGCCGCGCCACCACCGCGCAAAAATGCGTACGCGCAGGCGGCAAAC
ACAACGACTTGGAAAACGTCGGCTACACCGCCCGCCACCACACCTTCTTTGAAATGATGG
GCAACTTCTCCTTCGGCGACTACTTCAAACGCGACGCCATCCACTTCGCTTGGGAATTTC
TGACTTCCCCCGAATGGCTCAACATCCCTAAAGACAAACTGTTGGCGACCGTTTACGCGG
AAGACGACGAAGCCTACAACATCTGGTTGAACGAAATCGGTATGCCGTCCGAGCGCATCG
TCCGCATCGGCGACAACAAAGGCGCGAAATACGCATCCGACAACTTCTGGCAAATGGGCG
ACACCGGCCCTTGCGGCCCCTGCTCCGAAATTTTCTACGACCACGGCGAAGAAATCTGGG
GCGGCATTCCCGGCAGTCCCGAAGAAGACGGCGACCGCTGGATCGAAATTTGGAACTGCG
TATTTATGCAGTTCAACCGCGACGAACAAGGCAATATGAACCCGCTTCCCAAACCTTCCG
TCGATACCGGTATGGGCTTGGAACGCATAGCCGCCGTCATGCAGCATGTTCACAGCAACT
ACGAAATCGACTTGTTCCAAGACCTGCTCAAAGCCGTTGCCCGCGAAACCGGCGCGCCGT
TCAGAATGGAAGAACCCAGCCTGAAAGTCATCGCCGACCACATCCGCTCCTGCTCGTTCC
TGATTGCAGACGGCGTCTTGCCTTCCAACGAAGGCCGCGGCTACGTATTGCGCCGCATTA
TCCGCCGCGCCGTGCGCCACGGTTACAAACTGGGTCAAAGCAAACCGTTCTTCCACAAAC
TCGTTGCCGATTTGGTCAAAGAGATGGGCGGTGCCTACCCTGAATTGAAAGAAAAACAAG
CCCAAATCGAAGAAGCATTGAAAAACGAAGAAAGCCGTTTTGCCCAAACGCTGGAAACCG
GTATGGCTTTGTTGGAAAACGCGCTGGTCAAAGGCGGCAAAACACTCGGCGGCGAAATCA
TCTTCAAACTCTACGATACCTACGGTTTCCCATACGACTTGACTGCCGACATCTGCCGCG
AACGCAATATCGAACCGGACGAAGCAGGCTTCGAGCGCGAAATGGAAGCCCAACGCGCAC
GCGCACGCGCCGCCCAAAGCTTCAAAGCCAACGCCCAACTGCCTTATGACGGTCAAGACA
CCGAGTTTAAAGGTTATAGCGAACGCCAAACCGAATCCAAAGTCCTCGCCCTCTACAAAG
ACGGCGAGCAAGTCAACGAATTGAACGAAGGCGACAGCGGCGCAGTCGTCATCGACTTTA
CCCCGTTCTATGCAGAATCCGGCGGCCAAGTCGGCGATGTCGGCTATATCTTCTCAGGCG
AAAACCGCTTTGAAGTACGCGATACCCAAAAAATCAAAGCGGCCGTATTCGGTCAATTCG
GCGTACAAACTTCAGGCCGTCTGAAAGTCGGCGACAGCGTTACCGCCAAAGTGGACGACG
AAATCCGCAATGCCAATATGCGCAACCACAGCGCAACCCACTTGATGCACAAAGCCCTGC
GCGATGTATTGGGCAGACACGTCGAACAAAAAGGCTCTTTGGTTACCGCCGAATCCACCC
GTTTCGACATTTCCCATCCCCAAGCGGTAACTGCCGAAGAAATTGCCGAAGTAGAACGCC
GCGTCAACGAAGCCATTTTGGCGAACGTTGCCGTCAATGCAGCCATTATGAGCATGGAAG
ACGCGCAAAAAACCGGCGCGATGATGCTCTTCGGCGAAAAATACGGCGAAGAAGTGCGCG
TACTGCAAATGGGCGGTTTCTCTACCGAATTGTGCGGCGGCACACACGTTTCACGCACCG
GCGACATCGGCCTCTTCAAAATCATCAGCGAAGGCGGTATTGCCGCAGGCGTGCGCCGTA
TCGAAGCCATCACCGGCCTGAACGCACTCAAATGGGCGCAAGAGCAAGAGCGTTTGGTGA
AAGACATTATTGCCGAAACCAAAGCCCAAACCGAAAAAGACGTACTGGCAAAAATCCAAG
CAGGCGCGGCACACGCCAAAGCATTGGAAAAAGAATTGGCACGCGCCAAAGCCGAACTCG
CCGTCCACGCAGGCGCCAAACTCTTGGACGATGCAAAAGACTTGGGCGCAGCCAAACTCG
TTGCCGCCCAAATCGAAGCCGACGCAGCCGCCCTGCGCGAAATCGTTACCGATTTAACCG
GTAAATCCGACAACGCCGTGATTCTTTTAGCGGCAGTAAACGACGGCAAAGTCTCCCTGT
GCGCCGGCGTATCCAAACCGTTGACCGGCAAAGTGAAAGCAGGCGATCTGGTTAAATTTG
CAGCCGAACAAGTCGGCGGCAAAGGCGGCGGCAGACCAGATTTGGCGCAAGCCGGCGGCA
CGGATGCCGACAAATTGCCCGCCGTGTTGGATAGCGTGAAAGACTGGGTCGGCGCGAAGC
TGGTTTGATGTGGGAAAGGCAGCCTGAAAGGTTTCAGGCTGCCTTTTGTGCAAAGAGGCC
GTCTGAAAGGTCTCGTTTGCCGTAGGTTGGGTCGCGACCCAACAAATTTTGTGAAGTATA
AAAATGTTGGTCATGACCCAACCTACCTGCCTTTTTGTACAAAGAGGCTATCTGAAAGGC
CTTGTTTGCCGTATGGTGGGTCGCGACCCAGCAGATTTTTATTAGGGTATGACCCAAGCT
ACTTGCTACGATAAAAAAGGATTTTTAAATGAGCATTAGCCTTATTGGACTACACATTAC
CATAGCAATCATTTTGTTTTTTACTACAAATTTTATGGAAAAAAATCATCTATATTTGG
CTATTACCAACTGTCTTTTAGCGAAGAAAATCACTCTCCGGCATTTAATATTTTTTACAG
AGCATTTACCCCTATATTATTTATCGTTATTTTTTCTTGGGTTGTTACTAGTCTTGAAAT
TCCCATTTCTCTTGAAAAGATAAACTATGTAGTAATTTATTATTTTATAATTAGATTGTT
ATCTGTATTTGTTTTTGAGAAAACACACATAGTTAACTGGTTTAATCAACTAACAATACC

# Appendix A

CATACTATCCATAACATTATCATTTATAGTATATAACAAAATGATTTTGCCCAAAAGTTT
TCTACTTCCATCCTCACAAGAAGTAGCTACTACTTTTTGAATAGCGCTTGGTGGTTACAT
ATATAATATATTAAATAATGAATCAGGGCATTTAAAATCTTATAAAGAAAGAAGAGTAAA
TTATGTAAAACACATGCACAAAAAATTTGAAAGTTATTTTGGTAAAATTATAGATAAAAT
AATCAAAGAGGATAGTTATAATAATGATGATTTTTTAACCGATAAGAAAAAAGCACTAAT
ATATTCAGTTTTAATTTATGAGAATTTTAATAGGGGACTAGTTTATAGATATTTTGAAAA
AAATTATTTTGTACTGGTAGAATAAAAACATTTGGAATAATGCAAGTAACCTCAGCAGAG
TACCTTTCCAATGAGGAAAGTATAAAAAAAGGCGGAAATATTCTTATGGAAAAATACAAT
GAAAAATATAATGAATCTATTGATGGCAATAAAACTCTCTATAAATCATATTATGAATCA
AGAAGAGAGAGTATTAAAAACTACAACCCAGATGCAAAATACATTAATGAAATTGAATCA
ATTTACATGATGCTTGGAGAAATCTATCCAAATGCACCAGACTTCATGTCACCACATTTT
GAGGGGGACTGCTCTGAGGGGGAATAAAATCATTATTTATTCTTTATTAGTTATTAGCAG
GATTTGTCGGGCATAAATGCCCGACCTACAAATTCAATTTTTTCAAACCTCTGCCAAATA
TTTTCATCTTTGCAAGGCTGTCTGAAAACCCAAACCCCATTTTCAGACGGCCTTTTTTCG
CTAAAATCCCCATACCGTTCAATCCGAAAACACAGGAGAATCATCATGGAAGTTACCATC
TCCGCCATCATCAATGGCGAATTTGCCGACCAATACGGCAAGCGCGGTAGTCAGTTTAAT
GAAAACGGGATGCTGATTTAATTCTATTTCCTTTGAAACTACCAATAACCTGCCTCCATC
ATAAAACTAAAAGCAAGCCGTAGCCTGCATTCCCACAAACCGCGTGCGTTGCCATGTCAC
ACACCCTACCTGCGGGCGACGCAAACCTTAAGAGACCTTTGCAAAATTCCCCAAAATCCC
CTAAATTCCCACCAAGACATTTAGGGGATTTCTCATGAGCACCTTCTTTCAACAAACCGC
CCAAGCCATGATTGCCAAACACATCGACCGCTTCCCGCTATTGAAGTTGGACCGGGTGAT
TGATTGGCAGCTGATCGAACAATACCTGAACCGTCAAAAAACCCGTTACCTTAGAGACCA
CCGCGGCCGTCCTGCCTATCCCCTGCTGTCCATGTTCAAAGCCGTCCTGCTCGGACAATG
GCACAGCCTCTCCGATCCCGAACTCGAACACAGCCTCATTACCCGCATCGATTTCAACCT
GTTTTGCCGTTTTGACGAACTGAGCATCCCCGATTACAGCACCTTATGCCGCTACCGCAA
CCGGCTGGCGCAAGACAATACCCTGTCTGAACTGTTGGAACTGATTAACCGCCAACTGAC
CGAAAAAGGTTTAAAAATAGAGAAAGCATCCGCTGCCGTCGTTGACGCCACCATTATTCA
GACCGCCGGCAGCAAACAGCGTCAGGCCATAGAAGTTGACGAAGAAGGACAAATCAGCGG
TCAAACCACACCGAGTAAGGACAGCGATGCCCGTTGGATAAAGAAAAACGGCCTCTACAA
ACTCGGTTACAAACAACATACCCGTACCGATGCAGAAGGCTATATCGAGAAACTGCACAT
TACCCCCGCCAATGCCCATGAGTGCAAACACCTGTCGCCGTTGTTGGAAGGTCTGCCCAA
AGGTACGACCGTCTATGCCGACAAAGGCTATGACAGTGCGGAAAACCGGCAACATCTGGA
AGAACATCAGTTGCAGGACGGCATTATGCGCAAAGCCTGCCGCAACCGCCCGCTGTCGGA
AGTGCAAACCAAGCGTAACCGATATTTGTCGAAGACCCGTTATGTGGTCGAACAAAGCTT
CGGTACGCTGCACCGTAAATTCCGCTATGCCCGGGCAGCCTATTTCGGACTGATTAAAGT
GAGTGCGCAAAGCCATCTGAAGGCGATGTGTTTGAACCTGTTGAAAGCCGCCAACAGGCT
AAGTGCGCCCGCTGCCGCCTAAAAGGCAGCCCGGATGCCTGATTATCGGGTGTCCGGGGA
GGATTAAGGGGGTGTTTGGGTAAAATTAGGCGGTATTTGGGGCGAAAACAGCCGAAAACC
TGTGTTGGGATTTCGGTTGTCGTGAGGGAAAGGAATTTTGCAAAGGTCTCCAGCAGTTTG
CGCATACATGCCGTAACGGCAACCTTATACGGCTTACCCTCGGACAGCGGGCGTTGGTGG
AAATCCCGAATAAGCGGTTCAAAACGTGTCGCTGCCACGGTAGCCATATACAGTGCCTTA
AGCACCGCAGACCTTCCGCCAAAGCAGCGGCTTTTGAATTTGGCTTCCCCGCTCTTCCTC
GGGTGCGGGGCAATGCCGACCAAACTCGCTATCCGTTTGTGCGACAGCCGCCCCAATTCA
GGTAGCATCGCCATCAGCGTAGCCGTCGTTATCGAACCGATGCCTTTGATTTGCTCCGCC
ACTTGGGCTTTGCCGTCAAAATGCGTGTGGGTGTGGTCGTCGATTTGTTTGTCCGATTCG
TCAATCAGCCGGTCAAAATGGGCAATCAGTTGTTTGACGCTTCCGACTTGCGTTTCGTGA
ACCTGATGCAGACGGTTTTTCTCGGCAGTCCGCATATCCGCCGATTGGTTGCGGCGGTTA
ACCAAGGCTTCCAACACTTCTTCCGCTTCTGTGGGCGGGTGGTAGGGCATGGTTTGCCAA
TCTTCTTTCTGTGCCTTCATCTGTGCGAAGAAGGCAGGCATTTTGGCATCTTTGGCGTCG
GTTTTGGTCAGCGACTGCGATTGGGCAAACTGATCCGTCTGAGGCGGGTTGGCGATAATC
ACGGCTATGCCTGCTCGGTGGATGGCTTTGGCGGCGGGGATTTCGAGACCTCCGGTACTT
TCCGTCACGACGAGGGCGACCTTGTGTTTTTTAAGGTATTCGATAGTATGGGCGATACCT
TTGGGGTTGTTGGTTTCGGTTTTGGTTTTAGACAAAGACGAAACGGCGATGACGAAGTTT
CGTTTGGCGATGTCGATATAGTGAATTAACAAAAATCAGGACAAGGCGGCGAGCCGCAGA
CAGTACGGATAGTACGGAACCGATTCACTTGGTGCTTCAGCACCTTAGAGAATCGTTCTC
TTCGAGCTAAGGCGAGGCAACGTCGTACTGGTTTTTGTTAATTCACTATATCTGTGCGTT
ACGACGGCATGCCGTCTGAAGGGTGTTTATGTCTGCATCTAAGAAATTTCCGATTCCTTT
GAGCTATTTCAGCATCGCGCTGGGCTTGTTTGCCTTGGGGCTGTCGTGGCGTTACGGCGC
GTCTGTCGGGCTGCTGCCCGCCTTGGCCGCCGAATCGCTGCTTGCGGCGGCTTCGGTCGT
CTGGCTCTTGCTGGTGGCGGCATACCTGATCAAAATGTTTGCGTACCGAAACGATTTTTT
GTCTGATTTACGCGACTTGGTGCAATGCTGCTTCATCAGCGCGATTCCGATTACCGCTAT
GCTGGAGGGACTCGCGCTGAAGCCCTATCAGGCAGGCGCGGCGGCAGTCCTGATTTATGT
CGGCGTTGCCGGACAGTTGGCTTTTTCGATGTATCGGGCGGCCGGTCTGTGGCGCGGCCT
GCATTCCTTGGAGGCGACGACGCCGATTATTTATCTGCCTACGGTTGCGACAAACTTTGT
CAGCGCGTCATCTCTGGCGGCGTTGGGGCATCATGATTATGCAGCTTTGTTTTTCGGCGC
GGGTATGTTTTCCTGGCTGAGCTTGGAAGCCTCCATCTTGGGCAGGCTGCGCACGGCGGC
ACCGGTCGGCACGGCGGCGCGCGGCGTGGTCGGCATCCAGCTTGCGCCCGCCTTTGTCGG
CTGCGGCGCGTATTTTGCCGTCGGCGGTAAAGTCGACGGTTTTGCGTTGGCATTAATCGG
CTACGGCTGCCTGCAGCTTTTGTTCTTGCTGCGCCTGACCCGCTGGTTTTGGGAAGGTGG
TTTTACGATGAGCTTTTGGGGATTTTCATTCGGTTTCGCGGCAATGGCAGGATGCGGTCT
GCATCTGGCGGCTTCCGGCGTATTGTCGGGCTTGGGGCTGACGCTTGCCACCGCCGGATC
GGCAGGCGTGGCGCTGCTGCTTGTCGGTACGCTGCACCGGATAGCGACGGGGCGTTTCTT
GGTACGCAGCTGATGCGTTTTGCCGCCTTGTCAAAAATGCCGTCTGAAACGCTGGGATTC
AGACGGCATTTTTTATTTCACACCCTTACAGGTAGAATTTTTCGATGACTTTCAAATTGT
CGTCCAATTTGTACACCAACGGCTGACCGGTCGGGATTTCCAAGCCCATAATGTCTTCGT

## Appendix A

CGGAAATGCCCTCGATGTGTTTTGCCAGCGCGCGCAGGGAGTTGCCGTGCGCCGCCACCA
AGACGCGTTTGCCGCTCAAAATCGCGGGGGCGATTTGGTCTTCCCAAAACGGCAATACGC
GCTCCAGCGTTACTTTCAGGTTTTCGCCGTCGGGTACGACATCGGCAGGCAGATGGGCAT
AGCGGCGGTCTTTGTGTGCGGAAAACTCATCGTCTTTGTCCAAAAGCGGCGGCAGGGTGT
CGTAGCTGCGCCGCCAGATGCGGACTTGCTCGTCGCCGTATTGTTCGGCGGTTTGTTTTT
TGTCCAGGCCTTGCAGTTGGCCGTAGTGGCGTTCGTTCAGCCGCCACGTTTTGATTTGCG
GTACGAACAGTTGGTCGGATTCTTCCAAAACGATGTTGCAGGTCTTAATCGCGCGGGTCA
GGACGGATGTGAAGGCGATGTCGAACTCATAGCCGTTTTCTTTCAGTTTCTTGCCGGCGG
CGGCAGCCTCGGCAAGCCCCTGCTCGCTCAGCTTCACGTCGCGCCAGCCTGTAAACAGGT
TTTTCGCGTTCCATTCGCTTTGTCCGTGGCGGATAAATACCAGTTCCATATCGTCTCCAA
TGTGTGAAAGTGGGAAAGCCTTATTTATAACATATTTTCACATTTCCCGTATTTGATTCA
GATTCAGACACGCGCCCACTATGGTTTGCCGTTTTGATTTACAATAATGTCCTTTGCTTT
ACATTCCGCATACACAATGAATACGCAAGCGCACGCCCCACATACCGATTCCAATACGCT
GATGCTCGGCCGATACGCCGAACGCGCCTATCTCGAATACGCCATGAGCGTGGTCAAAGG
CCGCGCGCTGCCTGAAGTTTCAGACGGCCAGAAGCCCGTGCAGCGGCGCATTTTGTTTGC
CATGCGCGATATGGGTTTGACGGCGGGGGCGAAGCCGGTGAAATCGGCGCGCGTGGTCGG
CGAGATTTTGGGTAAATACCACCCGCACGGCGACAGTTCCGCCTATGAGGCGATGGTGCG
GATGGCGCAGGATTTTACCTTGCGCTATCCCTTAATCGACGGCATCGGCAACTTCGGCTC
GCGCGACGGCGACGGGGCGGCGGCGATGCGTTACACCGAAGCGCGGCTGACGCCGATTGC
GGAATTGCTGTTGTCCGAAATCAATCAGGGGACGGTGGATTTTGTGCCGAACTACGACGG
CGCGTTTGACGAACCGCTGCACCTGCCCGCCCGCCTGCCTATGGTGTTGCTCAACGGCGC
GTCAGGCATTGCGGTGGGCATGGCGACCGAGATTCCGCCGCACAATTTGAACGAAGTGAC
GCAGGCGGCGATTGCGTTGTTGAAAAAGCCGACGCTGGAAACCGCCGACCTGATGCAATA
TATTCCTGCCCCCGATTTTGCCGGCGGCGGTCAAATCATCACGCCGGCGGACGAATTGCG
CCGGATTTATGAAACCGGCAAGGGCAGCGTGCGCGTGCGTGCGCGTTATGAAATCGAAAA
ATTGGCGCGCGGACAGTGGCGCGTCATCGTAACCGAGCTGCCGCCGAACGCCAATTCCGC
CAAAATCCTTGCCGAAATCGAAGAGCAAACCAACCCGAAACCGAAAGCGGGTAAGAAACA
GCTCAACCAAGACCAGCTCAATACCAAAAAGCTGATGCTGGATTTAATCGACCGCGTGCG
CGACGAGTCCGACGGCGAACATCCCGTGCGACTGGTATTCGAGCCGAAATCCAGCCGCAT
CGATACCGATACCTTCATCAACACGCTGATGGCGCAAACTTCGCTGGAAGGCAATGTGTC
GATGAACTTGGTGATGATGGGTTTGGACAACCGCCCCGCGCAGAAAAACCTGAAAACGAT
TTTGCAGGAATGGCTGGATTTCCGCACCGTAACCGTAACACGCCGTCTGAAATTCCGTTT
GAACCAAGTGGAAAAACGGCTGCACATCCTCGAAGGCCGTCTGAAAGTCTTTCTGCACAT
CGACGAAGTGATTAAAGTCATCCGCGAATCAGACGACCCGAAAGCCGATTTGATGGCGGC
GTTCGGGCTGACCGAAATCCAAGCCGAAGACATTTTGGAAATCCGCCTGCGCCAGTTGGC
GCGTTTGGAGGGTTTCAAACTCGAAAAAGAATTGAACGAGTTGCGCGAGGAACAAGGCCG
TCTGAACATCCTTTTGAGCGACGAAAACGAAAAACGCAAGCTGATTGTCAAAGAGATGCA
GGCGGATATGAAACAATACGGCGACGCGCGACGCACGCTGGTGGAAGAGGCCGGACGCGC
CGTGCTGACGCAGACCACCGCCGACGAACCCATCACGCTGATCCTGTCGGAAAAAGGCTG
GATACGCAGCCGCGCCGGACACAATCTCGATTTGAGCCAAACCGCGTTCAAAGAAGGCGA
CTGCCTCAAACAAACCCTCGAAGGCAGAACGGTTTTACCCGTCGTCATCCTCGATTCATC
GGGCAGAACCTACACGCTCGATGCCGCCGAAATCCCCGGAGGGCGCGGCGACGGCGTACC
GGTTTCCTCCTTAATCGAGCTGCAAAACGGCGCGAAACCCGTTGCGATGTTGACAGGATT
GCCGGAACAACATTATTTATTATCAAGCAGCAGCGGCTATGGCTTCATCACCAAGCTGGG
CGATATGGTCGGGCGCGTGAAAGCGGGCAAAGTGGTGATGACCGCAGACAGCGGCGAAAC
CGTTTTGCCGCCGGTTGCCGTCTATGCCTCCTCGTTCATCAACCCCGACTGCAAAATCAT
TGCCGCCACCAGTCAAAACCGCGCCCTCGCCTTCCCCATCGGCGAATTGAAAATTATGGC
GAAAGGCAAAGGGCTGCAAATCATCGGATTAAACGCCGGCGAATCGATGACGCATACCGC
CGTTTCTTCCGAGCTGGAAATCCTGATTGAAAGCGAAGGCAGGCGCGGCGCGGCGCACAA
~~AGACGGCATGGCGATGTGGCTGGTTGAGGCAAAACGGCGGCAAAAAAGGCAGAGTATTGCC~~
CATATCGGGCAGCCTGAAACAGCTTTCTTCCCCTAAATAAACCCGGTTCCGCACATATTA
TGGTGATTTCCAACCCCCGCGAACTTGAAAAACTCAAAGACCGGATTCCCAATCTGATCA
ACATCATCCGCGTCGCCATCGTTTTTCCGCTGATGATTATGCACATCCTCGGGCTGGAAA
CCGGCAGCCGTGCGAACCTGCACGCTTCGTGGACGGCGTGGGCGTTTTATGTTTGGCTCG
CCATTGCCTGCTGGCTGATTTTCTTTTCCATTATCCATCCGCATTGGCAATGGCAGTCGC
TGAAAATGCCGCGTTTCAGCGCGGTAGCGGACATCACGATGATCGGCGTGCTGACCTACC
TGTTCGGCGGCATCGATTCCGGCTTCGGCATCCTGATCCTGCCCTTCGTCGTCTGCTCCT
GCCTGCTCAGCTACGGGCGCTACCCCCTGCTCTATTCCAGCTACGCCGCCATCCTGCTGA
TATTCAACGCCATTGCCGACGGCGATATCGGCAAATACCCGCTCATATCGGATGCCCGAA
CCGCCTCGGCAACCTTCATCCTTGTCGCCGCCTCCTATCTTTCCGCCATCTTCACCTCAC
TGTCGGTCAAATACATCGACCGTGCCGGAAAACTCGCCTACGACAGCCATATCGCCTACC
ACCGCATCAAAGGCTTGAGCCAAACCGTACTCGAACGCGTTCAGGAAGCTGTCGTCGTCA
TCAATGCCGAAGGGCTGGCGGTGCTGTTCAACCGGAAGGCGAAAGACCTTTTCCCCGCGC
TCGAAATCGGACGGCGCGCCGGTCTGTCCGATTCTGCCGCCGAACTGTGGGATCAAGCCT
CTCCGCACACTTTCGAATACGTCCTCGGCACACCCGGCCTGAACGCCGGCATCCGCGCCG
TTCCGGTCAACAAAGGGTCGGACAAGCTGCTCATCCTCTACATCCGCCCGCAAAGCGAAA
TTCAGGCAGAAGCCCTGTCCGTCAAACTTGCCGCGCTCGGACAACTGACCGCCAACCTCG
CCCACGAAATCCGCAACCCGATGTCCGCCATCCGCCACGCCAACGACCTGCTGCGCGAAA
ATATGGAAGCGGGGGCGGCAGATCCGTTCAACGCCAAATTGTGCAAAATCATCGACGGCA
ACATCTGCCGCATCGACAAAATGCTCGAAGACATTTCCTCGCTCAACAAGCGCAACAAAA
CCGAACGCGAAACCATCGGCCTGATACCGTTTTGGGAAGAATTCAAACAAGAGTTCCTGC
TCGGCCATCCCGATGCCGCCGACTGCATCCGTCCGGACATTCAAGGCGGCAGCCCGACCG
CCTATTTCGATCCCGCCCACCTGCGGCAAATTATGTGGAACCTCGCCAACAACGCGTGGC
GGCACAGCCGCAAACAGCCCGGCTCGATTTCCGTCACCATCCGCCCCGCGCAAAAAAACA
CCGTCTGTATCCTCTTTGCCGACCGCCCGAAGTGCAGGAACACCTGTTCGAACCCTTTA

## Appendix A

CACCACGGCGGAAAACGGCACCGGCCTCGGGCTGTATGTCGCCCGCGAACTGGCGCACGC
CAATTTCGGCGATTTGACCTACCTACCGGAAGCCAAATGTTTCGAACTCACATTACCGGA
AAAAACCAATGACTGAACTGCAACACCCCGTCCTCGTCGTCGATGACGAAACCGACATTC
TCGACCTGATGGAAATGACCCTGATGAAAATGGGCTTGCGCGTCCATACCGCGTCAGGCG
TTGCCGAAGCCAAAAACAAGCTCGACAGCCAACGCTATTCGCTCGTCCTGACCGATATGC
GTATGCCGGACGGCTCGGGGCTGGAAGTCGTCCAACACATCAACAGCCGCCTGCTCGATA
CGCCGGTTGCCGTCATCACCGCCTTCGGCAACGCCGATCAGGCACAGGAAGCGTTGCGTT
GCGGCGCGTTCGACCCCGATACCATGCAGATACAGGACTATCTCGACCAAATCGAACGCG
ACATCATCGAACAAACCCTCAAACAAACCGAAGGCAACCGCACGCAGGCCGCCAAACGCT
TGGGCATCAGCTTCCGTTCCATGCGCTACCGTATGGAACGCCTCAACATCGGCTGACGAC
AAAACGGCATCCGCACCATCTCCGCCCACCCGAAAAAATGCCGTCTGAAACGGCACGGGA
AAGCGGGTTCGCCCCACGCCCGAACGGACACAAAACACCATGACCGACATCCTTATTGAC
AACACCGCCACCGAAACCGTCCGCACCCTGATACGGGCATTCCCCCTTGTGCCCGTTTCC
CAACCGCCCGAACAAGGCAGTTACCTCCTTGCCGAACACGATACCGTCAGCCTCAGGCTT
GTCGGGGAAAAAAGCAGCGTCATCGTCGATTTTGCCTCCGGCGCGGCACAATACCGGCGC
ACAAAAGGCGGGGGCGAACTCATCGCCAAAGCCGTCAACCACACCGCGCACCCCACCGTT
TGGGACGCAACCGCAGGATTGGGGCGCGACAGCTTCGTCCTCGCCTCGCTCGGGCTGGCC
GTTACCGCCTTCGAGCAACATCCCGCCGTCGCCTGCCTGCTTTCAGACGGCATCCGCCGC
GCCCTCCTCAATCCCGAAACGCAAAACACCGCCGCGCACATCAACCTCCATTTCGGCAAC
GCCGCCGAACAAATGCCCGCACTTGTCCAAACACAAGGCAAACCCGACATCGTCTATCTC
GACCCCATGTATCCCGAACGCCGCAAAAGTGCCGCCGTTAAAAAAGAAATGACCTACTTC
CACCGGCTCGTCGGCGAAGCGCAAGATGAAGCGGCACTCCTGCATACCGCACGCCAAACA
GCAAAAAAACGCGTCGTCGTCAAACGCCCCCGCCTCGGCGAACACCTTGCCGGACAAGAC
CCTGCCTACCAATACACAGGCAAAAGCACCCGCTTCGACGTTTACCTGCCCTACGGGACG
GACAAGGGATAACGCCCATAAAACAAGACACCGAAAAATTTGCCGTTCTTATGCAAACGA
GAAACCGGTTTTTGCGTTTCGACTGTTTTGGATAAGTCATCACACCTTAAAGTTTGTCAT
TCCCACAGAAGTGGAATCCGATTCATTCAGTTTTATAGTGGTTTAAATTTAAACCACTA
TAGTTGTTTTCGAGTTTCAGGCAACTTCCAAACCGTCATTCCCACGGAAGTGGGAATCTA
GAAATGAAAGGCAACAGGAATTTATCGTAAATGACTGAAACCGAACGGACTAGATTCCCG
CCTACGCGGGAATGACGGGGCGGGCAGATGCCGTCTGAAATTCCGTCATTCCCGTGAAAA
CGGGAATCTAGAACTTCTGATTTTTCAGACGACTTTTGAACATTGCCGCCACCCAATGAT
CTGGATTCCCACCTGCGCGGGAATGACGAGGTTTCAGGTTGCTGTTTTTAAGTTGCTGTT
TCGGGTTGCTGTTTTTTATGGAAATGACAAGGTTTTAGATTGCGAGAATTTATCCGCTCC
TCCGTCATTCCCACGGAAGTGGGAATCCAGAAATGAAAAGCAACAGGAATTTATCATAAA
TGACCGAAACCGAACGGACTAGATTTCCGACTGCGCGGGAATGACGGGGCGGGAGGATGC
CGTCTGAAATTCCGTCATTCCCGTGAAAACGGGAATCTAGAACTTCTGATTTTTCAGACG
ACTTTTGAACATTGCCGCTACCCAATGATTTGGATTCCCGCCTGCGCGGGAATGACGATG
TAAAATTATCCGGGATTCAAAAAGACAGGCTTTCACATCCGTGGGAATGACTGCGGAAAG
ATGATTTTTATAGTGGATTAACAAAAATCAGGACAAGGCGACGAAGCCGCAGACAGTACA
AATAGTACGGCAAGGCGAGGCAACGCCGTACTGGTTTTTGTTAATCCACTATATTTTGTC
ATAAAAATCCGCACCTTAATCAGTTGGCGGTTAAATCAAACTTTTAGGGTGCAGATTACT
TTTTATGATTTCAGACAGCATTTTGACAGGCGGCAGCCTATTTCGGCAATACCAAAAACT
TAATCAGCAGTTCTTTGAATACAAAACCGAACACGCCCAAGCCCAAAACCAAAAACAAAA
TGGCGATGCCGAATTTGCCTGCTTTGGACTCCTTGCCCAAATTCCAAACGATAAAACCCA
AAAAAATAATCAAGCCGGTCAGGCAGATTTTCAACGCCCAATCGGCAAAAACCGCTTCAT
CCATATTTTTTTCCTATTGTTGATGTGTATGCCATATAAGATAAGGGTTTCAGACGGCAT
CTGCTGTCCAATGCCGTCTGAAACACGCAATCAGCGTGCGAGTGCCTGTTTCAAATCGTC
AATCAAATCGCCAACATATTCCAAACCGACCGACAGGCGCACCAATCCGGGGCGGATGTT
GGCGGCGAGTTTTTCTTCGGGCTGCATCCTGCCGTGCGTGGTTGTCCACGGGTGGGTAAT
GGTCGAGCGCACGTCACCGAGGTTGGCGGTGCGGGAAAAGAGTTCCACGGGGTGGAGAAC
TTTCCACGCCGCTTCTTGATCGGCAACTTCAAAGCCGATGACGATGCCGCCGCCGTTTTG
CTGTTTGCGGATAAGCGCCGCCTGAGGATGGTCGGACAATCCGGTGTAGTACACGGCTTG
AACCTGCGGCTGCGCTTGCAGCCATTGTGCGATTTTCAGGGCGTTGTCGAACTGTTTTTC
CATACGCAGCGACAGGGTTTCCACGCCGCTCAACAACTGCCACGCATTAAACGGCGACAT
CGCCAGCCCGCAAGAGTTGCAATACATGGCGACCTGCGCCAACAACTCTTCCGAACCCGC
CAACACGCCGCCCATCACACGCCCGTGTCCGTCTATGGCTTTGGTCGCGGAGGAAACGGA
AATATCCGCACCGTGTTTCAAAGGCTGCGAGCCGACGGGCGACAGCAGGCTGTTGTCCAC
CACCAAGAGCGCGCCGATGCCGTGCGCCAATTCCGCCAAGGCTTCCAAGTCGGCCACTTC
GCCTAAGGGGTTGGACGGCGTTTCCAAAAACAGCAGTTTGGTATTGGCTTTGACGGCGGC
TTTCCATTCGTTTATATCAGTCGGCGACACGTGGCTCACTTCGATGCCGAATTTGGCAAC
GATGTTATTGATAAAGCCGACGGTCGTGCCGAACAGGCTGCGGCTGGAAATCACATGGTC
GCCCGCCTGCAAAAAGGTGAAAAACGCCGCCTGAATCGCAGACATACCCGCCGAAGTGGC
GACCGCGCGTTCCGCACCTTCCAAAGCGGCGATGCGTTTTTCAAAGGCGGCTGTGGTCGG
GTTGGCGGTACGGGTATAAGTGAACCCTTTGATTTTTTTGAAAACAAATCGGCAGCGTG
TTGGGCGTTGTCCCACATGAAGCTGCTGGTCAGAAACAATGCCTGATTGTGTTCGCGGTA
TTCGGTTTGTTCTTTGCCGCCGCGTATGGCGAGCGTTTGCGGATGGAGTTTTTTGCTCAT
CGGTGATTCCTCGGTTTTGTCCGTTCGGCAACGGAGCGTGCGCCCGTTGTTTAATTTGTT
AATATTTTGCGCCTGTTCTATGATGCTTTCAAGTCGGATGAGAATGCAAATGCCGTCTGA
AACGGCTTTCAGACGGCATGGCAATCAGCGTTTGTATTTTAACTCGTACTTGATGTCGTT
GAGGATTTTGCGGACATCGTGTTCCAACACGTCTTCGACTACCGCCCCCGCCTGCTCGTG
CAGCATCTGCTGGAGCTGATAGGTGAAAACCGCCATCTGCTTTTGCACCGCCGTTCGGAT
GATGCCGTTGACGGTATCGGTCAGATGCGGGCGCAGGCGTTTGATCAGCCGTTCGGTCAG
CTCCTGTTCGGACAGGCAGAACACTTCGCGCCGGTTGACGGCTTTCGGGTTCAGGATATT
GATTTGGACGGGCATCAACGTTTCTTCCGCATCGTTTTCCCCGTTTTCCGAAACCGCCGG
CTCATTCGTGCCGGATTCTGCCTCGTCGGCGTTTTCCCCGCTTTCAATCTGTCCGGTTTC

## Appendix A

AAATTCGACACTGTCTTTTTTGGTATCAAACCGGATTCTCCGCCGCGATTCGATGTGTTT
TTCCGAAACCGACATTTGCAGGGAAGCCTGCGCGTTGAGCCAGTTTTCCTGAAGGACGAT
CATCGGGTCGGTTTCGACTTCCTCGCCGCAATCGGCAACGGCGGCATTGTGTTCCTCCTG
CCATTTTTTCAGATACGCCTTCAACACACGGGCTCGGCTCTCATCGTCCAGTTTCGGCAC
AGGCGCGTCCGTTCCGGTTTCAGAGGGGCGGGACAGCGGCGCGTAAGTCGGCACTGCCTT
CATACGGCGCGTCTGACGCAGGTTTTCCAAACGTTTTTCCCAATTCGGCTCTTTATTCGC
ATCCATTTTCGGCTTCCGGTTCTTAATCTTTGCAAGCAGACAAACCCGCGCCCAAAGCGC
GGTTTGATATAATGGCGCATTTTAACAGATTCGCGAGGATACATCATGGGCAGCATCGAA
CAGCGTTTGGAATATCTGGAAGAGGCGAACGACGTGCTGCGTATGCAGAACCACGTCCTG
TCCACCGCATTCAAAGCCTTAATCCGCGCCCTTCCCGCCGAAACCGCCGAAATCGCGGTC
GAGTCGATTCAGCTTGCTTTTGAGGACGCCTTGGCAGAATTGAGCTATGAGGACAGCCCG
CATACGGATTTGTTCCACGACGTTACTTATGCGTTTTTCCGTGAAAAAGAACGTTAATTT
TATGTTAAACTGATTTTTTAGGCTTTTTGATTACCGAAAGGAATTTTGATGAATATGAAA
AAATGGATTGCCGCCGCCCTTGCCTGTTCCGCGCTCGCGCTGTCTGCCTGCGGCGGTCAG
GGCAAAGATACCGCCGCGCCTGCCGCCAACCCCGACAAAGTGTACCGCGTGGCTTCCAAC
GCCGAGTTTGCCCCCTTTGAATCTTTAGACTCGAAAGGCAATGTCGAAGGTTTCGATGTG
GATTTGATGAACGCGATGGCGAAGGCGGGCAATTTTAAAATCGAATTCAAACACCAGCCG
TGGGACAGCCTTTTCCCCGCCTTAAACAACGGCGATGCGGACGTTGTGATGTCGGGCGTA
ACCATTACCGACGACCGCAAACAGTCTATGGACTTCAGCGACCCGTATTTTGAAATCACC
CAAGTCGTCCTCGTTCCGAAAGGCAAAAAAGTATCTTCTTCCGAAGATTTGAAAAACATG
AACAAAGTCGGCGTGGTAACCGGCTACACGGGCGATTTCTCCGTATCCAAACTCTTGGGC
AACGACAATCCGAAAATCGCGCGCTTTGAAAACGTTCCCCTGATTATCAAAGAACTGGAA
AACGGCGGCTTGGATTCCGTGGTCAGCGACAGCGCGGTCATCGCCAATTATGTGAAAAAC
AATCCGGCCAAAGGGATGGACTTCGTTACCCTGCCCGACTTCACCACCGAACACTACGGC
ATCGCGGTACGCAAAGGCGACGAAGCAACCGTCAAAATGCTGAACGATGCGTTGGAAAAA
GTACGCGAAAGCGGCGAATACGACAAGATTTACGCCAAATATTTTGCAAAAGAAGACGGA
CAGGCCGCAAAATAAGCCCGCCCGTCCGAACACAATGCCGTCTGAAGCCCTTTCAGACGG
CATTGTTCATCAATCGGCCTACAATGAACTGCCTGCTGATTTCTCCCTACCGCAAAGCAA
CAGGCAAAGATTACAAATATCAAAATCCGAGTAAAACAGTATTTTATTAAAACAAATTGA
TAATCAAGAGATTAGAATTATGTATTGTCTTTACCGTACAAACGCTGGCACTATTTCAAC
CTGATAAAAAACAGCCTTCAAAAAGGTTGTTTAAAACAGCAGCAGACACTTACCGCCACA
ACCTTGAAAAGGAACACAATCATGACCGTCATCAAACAGGAAGACTTTATCCAAAGCATT
TGCGATGCCTTCCAATTCATCAGCTACTATCATCCCAAAGACTACATCGACGCGCTTTAT
AAGGCGTGGCAGAAGGAAGAAAATCCTGCCGCCAAAGACGCGATGACGCAGATTTTGGTC
AACAGCCGTATGTGTGCGGAAAACAACCGCCCCATCTGCCAAGACACAGGTATCGCAACC
GTCTTCCTCAAAGTCGGTATGAACGTCCAATGGGATGCGGACATGAGCGTGGAAGAGATG
GTTAACGAAGGCGTACGCCGCGCCTACACTTGGGAAGGCAATACGCTGCGCGCTTCCGTC
CTCGCCGATCCGGCCGGCAAACGCCAAAACACCAAAGACAACACCCCCGCCGTCATCCAT
ATGAGCATCGTGCCGGGCGGTAAAGTCGAAGTAACCTGCGCGGCAAAAGGCGGCGGCTCT
GAAAACAAATCCAAACTCGCCATGCTCAATCCTTCCGACAACATCGTCGATTGGGTATTG
AAAACCATCCCGACCATGGGCGCGGGCTGGTGTCCTCCCGGCATCTTGGGTATCGGCATC
GGCGGCACGCCCGAAAAAGCCGTGCTGATGGCAAAAGAGTCCCTGATGAGCCACATCGAC
ATTCAAGAATTGCAGGAAAAGGCCGCGTCCGGCGCGGAATTGTCCACCACCGAAGCCCTG
CGCCTCGAACTCTTTGAAAAAGTCAACGCGCTGGGCATCGGCGCACAAGGCTTGGGCGGA
CTGACCACCGTGTTGGACGTGAAAATCCTCGATTATCCGACCCACGCCGCCTCCAAACCG
ATTGCCATGATTCCGAACTGCGCCGCCACCCGCCACGTCGAATTTGAATTGGACGGCTCA
GGCCCTGTCGAACTCACGCCGCCGCGCGTCGAAGACTGGCCCGATTTGACTTACAGCCCC
GACAACGGCAAACGCGTCGATGTCGACAAGCTGACCAAAGAAGAAGTGGCAAGCTGGAAA
ACCGGCGACGTATTGCTGTTGAACGGCAAAATCCTCACCGGCCGCGATGCCGCACACAAA
CGCCTCGTCGATATGCTCAACAAAGGCGAACAATTGCCCGTCGATTTCACCAACCGCCTG
ATTTACTACGTCGGCCCCGTCGATCCGGTCGGCGATGAAGTCGTCGGTCCGGCAGGTCCG
ACCACAGCCACCCGCATGGACAAATTCACCCGCCAAATGCTCGAACAAACCGACCTCTTG
GGCATGATCGGCAAATCCGAGCGCGGCGTGGCCACCTGCGAAGCCATCGCCGACAACAAA
GCCGTGTACCTCATGGCAGTCGGCGGCGCGGCGTATCTCGTGGCAAAAGCCATCAAATCT
TCCAAAGTCTTGGCGTTCCCCGAATTGGGCATGGAAGCCATTTACGAATTTGAAGTCAAA
GACATGCCCGTAACCGTCGCCGTAGATAGCAAAGGCGAATCCATCCACGCCACCGCCCCG
CGCAAATGGCAGGCGAAAATCGGCATCATCCCCGTCGAATCTTGAGGCGCCATGCCGTCT
GAACACAAAATCTGCCTTCAGACGGCATTTCCGCCCCCGGTTGCGGTACAATCCACCATT
TCATCACTCGGCGACCCACACCGTGAAAATCCTCATTTTAGGCAACGGACAGGTAGGTTC
TACCGTCGCACAAAACCTTGCCGCCATACCCAACAACGACGTAACCGTTATCGACATCGA
CGAAAAAGCATTGCAGGAAACAGGCAGCCGCCTCGATGTCCAAACCGTTTTCGGCAACGG
CGCATCCCCCTTCACATTAGAACGCGCCGGCGCGGAAGATGCCGACTTGCTGCTCGCGCT
CTCCCGCAGCGACGAAACCAACATCGTCGCCTGCAAAGTTGCCGCCGACCTGTTCAACAT
CCCCGGCCGCATCGCGCGCGTCCGTTCCAGCGAATACCTCGAATACCTCAGCCCCAAGCT
CGAAAACAACGAAAACGGCAGCCTTTCCATATTCGGCATAACCGAAACCATCAGCCCCGA
ACAGCTCGTTACCGAACAGCTTGCCGGCCTGATAGACTGCCCGGGCGCATTGCAGGTTTT
ACGTTTTGCAGACGACCGCGTGCGGATGGTCATCATACAGGCGCGGCGCGGCGGACTGCT
TGTCGGACGCAGCATTGCCGACATCGCCCAAGATTTGCCCGACGGGGCCGACTGCCAAAT
CTGCGCCGTTTACCGCAACAACCGCCTCATCGTCCCCGCGCCGCAAACCGTCATCATCGA
AGGCGACGAAATCCTATTTGCCGCCGCCGCCGAAAACATCGGCGCGGTCATACCCGAATT
GCGCCCCAAAGAAACCAGCACCCGCCGCATCATGATTGCCGGCGGCGGCAACATCGGCTA
CCGTCTCGCCAAGCAGCTCGAACACGCATACAACGTCAAAATCATCGAATGCCGGCCGCG
CCGTGCCGAATGGATAGCCGAAAACCTCGACAACACCCTCGTCCTGCAAGGTTCGGCAAC
CGACGAAACCCTGCTCGACAACGAATACATCGACGAAATCGACGTATTCTGCGCCCTGAC
CAACGACGACGAAAGCAACATTATGTCCGCCCTTTTGGCGAAAAACCTCGGCGCGAAGCG

## Appendix A

CGTCATCGGCATCGTCAACCGCTCAAGCTACGTCGATTTGCTCGAAGGCAACAAAATCGA
CATCGTCGTCTCCCCCCACCTCATCACCATCGGCTCGATACTCGCCCACATCCGGCGCGG
CGACATCGTTGCCGTCCACCCCATCCGGCGCGGCACGGCGGAAGCCATCGAAGTCGTCGC
ACACGGCGACAAAAAAACTTCCGCCATCATCGGCAGGCGCATCAGCGGCATCAAATGGCC
CGAAGGCTGCCACATTGCCGCCGTCGTCCGCGCCGGAACCGGCGAAACCATTATGGGACA
CCATACCGAAACCGTCATCCAAGACGGCGACCACATCATCTTTTTCGTCTCGCGCCGGCG
CATCCTGAACGAACTGGAAAAACTCATCCAGGTCAAAATGGGCTTTTTCGGATAAACCGC
CCCATTCCGGACATATTGCCGCCAAGCGGTATGGAAGCGGAAATAATGGTAGGTGGGCTT
CAGACGGCATCCGCCCTCCCCGTCATTCCCGCGTAAGCGGGCATCCAGACCTTGGGATAG
CGGCAATATTCAAAGGTTATAAAAGACCCGTCATTCCCGCGCAGGCGGGAATCCAGACCT
TGGGATAGCGGCAATATTCAAAGGTTATCTGAAAATTTAGAGGTTCTAGATTCCCGCTTT
CGCGGGAATGACGAAAAGTTGCGGGAATCCAGAACGTCGGGCAACGGCAATATTCAAAAG
CCGTCTGAAAATTTAAAAGTTCTAGATTCCCGCTTTCGCGGGAATGACGAAGTTTCAGAC
GGCATCGCCCGCCTGTTTTGATATAGCGGCACCCCCCCGACAAAAAAACAATCCGGAACG
CATCTGACCGTTCCGGCTTGTTTTCAGGCGAATCCGCCGCATCAGAACATACTGCGCACG
CCCATATTGACCTGCCAAGTCTAGCGCATCGTGTGCATCGAAGACCTTTGCGCCTCAAAA
TAAAGCTGCCTTCCGTTGTCGGCATTACCACGCAAAAAAATGAATTGCTTGATATTCCAA
TGTTTTTTATATGTTTTTATATTGTGATGCGATCAGACAAACGCCCCCCTGACATTTGTT
TAGACGGCATCGTATTGCTAAATTTCTATAAGTATGTATAATGTCCGTTTCCACGCGCCC
ATCGTCTAGAGGCCTAGGACACTGCCCTTTCACGGCGGCAACCGGGGTTCGAATCCCCGT
GGGCGTGCCAATTCAAAAACCTGCTTGTTTCAAGCAGGTTTTTTATTATGAGTCGTCATT
CCCGCAATTTTTCGTCATTCCCGCAAAAGCGGGAATCTAGAGCGTAGGGTTGAAGAAACC
GTTTTATCCGATAAGTTTCCGTGCCGACAGGTCTGGATTCCCGCCTGCGCGGGAAGGACG
GCAGAGGGTGGACGATGCCGTCTGAAGCCTGACAAAGCATTTGATGCCGTCTGAAACTTC
GTCATTCCCGCAAAAGCGGGAATCTAGAGCGTAGGGTTGAAGAAACCGTTTTATCCGATA
AGTTTCCGTGCCGACAGGTCTGGATTCCCGCTTTCGTAGGAATGACGGAATTTTAGGTTT
CTGTTTTTGTGGAAATGACGAATAAAGCGTGCCGGTTTATGCTCGCCGCAACACGCGGTT
CAGACGGCATTGCTCTCTTTTTTCATTATCAGTGGGTGTAGCAACTGTATTTTTCACCCC
GTCGGGCAAAAATACAGTTGCTACGATGCACCCCGCCGCCCTGCCCTGTGCCTTGTCCTG
CAATACGGCATATAATGCACCACAAACCCCCGCGCTGCGGTTTTCAGACGGCATCGCCGT
GCTTTTTTACAGGCATTAGCCCTTTTTATCGGACGCAATATTAAGGAGGAACAAATGAAA
AGCTCTTTTGTGCAAACGCTTACCATCGCCGGTTCGGATTCGGGCGGCGGTGCGGGCATT
CAGGCGGATTTGAAAACATTTCAGATGCGCGGCGTGTTCGGAACGTGCGTCATCACCGCC
GTTACCGCGCAAAATACCTTGGGCGTGTCGGCGGTTCATCTCGTCCCGACCGAAACCATC
ACCGCACAAATCCAAGCAATCAGGGAAGACTTCGACATCCGCGCCTACAAAATCGGTATG
CTCGGCACGGCGGAAATCATCGAATGCGTTGCCGACAAGCTGAAACACTGCAGCTTTGGC
AGGCGCGTACTCGACCCTGTGATGATTGCCAAAGGCGGTGCGCCGCTGTTGCAGGATTCC
GCCGTTGCGGCACTGACGCGCCTGCTGCTTCCCGATACGGATGTATTGACCCCCAACCTG
CCCGAAGCGGAAGCTCTGACCGGCGTGCATATTGAAAACCGTAAAGATGCGGAACGTGCG
GCAAAAATCCTGCTTGATTACGGTGTCAAAAATGTCGTTATCAAAGGCGGACATTTGAAC
GGCAGCACAAGCGGACGCTGCACGGATTGGCTGTTTACACAAAATGAAACGCTGGAATTC
GACAGCCCGCGCTTTCCGACCGCCCACACGCACGGCACGGGCTGCACGTTTTCCGCCTGC
ATCACCGCCGAGTTGGCAAAAGGCTCGGACGTTTGCGAAGCCGTACAGACTGCCAAGGCC
TACATCACGGCGGCAATCTCAAACCCTTTGGAAATCGGCGCAGGACACGGCCCGGTCAAT
CATTGGGCGTATCGGGACTAACCGTAAAAATGCCGTCTGAAACAAAATGTTCAGACGGCA
TTTTTGAGGATTATTCAGGCTTTTTCGCCAGCATCGTTACAAATTTAAACCGTATCGGAT
TGCCGTTTTCGTCTTTGGCATGCATAGAACCCAATTCTTCTTTATATTCGACCAGTTCCC
AATCCCGATAATAATCCTTCAGCTCGCCCTCTTTAAATTTAAAAGGGAACGGCATCGGAC
AGGGGAAATCCGCCGTATCCATTGCCGATACAATCAAGTTGTACCCGCCCGCCGCCGTAT
GCGCCTGCATATCGGCAATCACGTCGGGTACGCGCTGCGGCATCAGGAACATCAGCACCA
CTGTTGCCACAATATAATCAAACTCGCCCTGCAAGGCGGCGGCGTTCAAATCATATTCCA
GCGTGCGGACGTTCAAACCCTCCGCCTCTGCCAGCTCCGCCACGTTTGCCAAGGCGGCGG
GATTGTGATCGACTGCAGTAACTTCAAACCCCTTCAAACCGAGAAACAGCGCGTTGCGCC
CCTGTCCGCAGCCCATATCCAACGCCCTGCCCGCCGGTACGGTATCCCGTGCCGCCGCGA
CCGCAGAATGCGTGGCACTCATCCCGTATTTTTTGTGAAAATAGTCTGCCGCCGCGCAAT
ACAGCGACAAACGGATTTCGGCATCGTCCGTTTTCGGTTTGACAGAAAACACCTGCTGCG
GCGCAAACACACAATCGCCGCCGTCTGCCGACCAAACTTCTGCCGACCCGTCCGGTGCAC
GAACTTCGACATCGCCCTGCAACACATTCAGGCAGACCCACTCCCCTTCCTCAGACGAAT
AGCCCGACAACAAAACTTCCGGCAGGTTTTCCACTTTCCATACAGGCATCTGTCCGAAAC
AAAACAACTCGCCACTTTGACCCACTATCCGCTCCTTCATATTCAAAAATAAAGTTGCAC
ATTATATGCCTATTTTAATCCGCCGCAATCTTTCAGACGGCACGGCGCGCAAACCGCTTA
TAATCACGCCGGACACCACACAAAGGCACAATAATGAACCAAACCGTTTACCTTTACACC
GACGGCGCGTGCAAAGGCAATCCCGGCGCGGGCGGCTGGGGCGTGTTAATGCGCTACGGT
AGCCACGAAAAAGAACTTTTCGGCGGCGAAGCGCAAACCACCAACAACCGCATGGAACTG
ACTGCCGTCATCGAAGGACTGAAATCGCTCAAACGCCGCTGCACCGTCATCATCTGCACC
GACTCGCAATACGTCAAAAATGGCATGGAAAACTGGATACACGGTTGGAAGCGCAACGGC
TGGAAAACCGCCTCCAAACAGCCCGTCAAAAACGACGACTTGTGGAAAGAACTCGACGCT
CTAGTCGGACGGCATCAAGTCAGTTGGACTTGGGTGAAAGGACACGCGGGACACGCCGAA
AACGAACGCGCCGACGATTTGGCAAACCGTGGCGCAGCGCAGTTTTCCTGACTGCCGCTC
CGGCAAAAATGCCGTCTGAAACCGCTAATGGGCTTCAGACGGCATCGTCCTCCACCGTCA
TTCCCGCGCAAGCGGGAATCCAAACCGTCGGGCAACGGCAATATTCAAAGATTATCTGAA
AGTTTGAAGTTCTAGATTCCCGTTTTCACGGGAATGACGAAAAGTTGCAAGAATGACGGA
GTTTCAGGCGGCATCCGACCGCCCCGTCATTCCCGCGAAAGCGGGAATCTAAAAACCCAA
CGCTGCAAGATTTATCAGAAACAACTGAAACCGAACGGACTGGATTCCCGCCTGCGCGGG
AATGACGGGATTTTAGTAACCGTAGCAACCGCCTGCGCGACGGCTAAGGGGCTTCAGCAA

## Appendix A

CCGTAGCAACTGCCTGTGTGGGAATGACGGACAATGGGCTTCAGACGGCATCTCTTGCCT
GCCGCTAAAACAGTTTGCCGCACAACTGTTCAAACGCGTCCGATATGTTTCAACACACAG
GACGACACATAAAGCACCTCCCTATGTGTCGTCCTGATTTGGAAGGGGTTACACCCCCTC
CCAAATAAAGTCTGATCCTGCCGCCCTAAAGGGCGGGGTTTCAACCGAAAAGGAAATACG
ATGAAGTGGTACAATTAGCGGCAATGCGGACAGACAAATTAAACTATAGTGGATTAAATT
TAAACCAGTACGGCGTTGCCTCGCCTTAGCTCAAAGAGAACGATTCTCTAAGGTGCTGAA
GCACCAAGTGAATCGGTTCTGTACTATTTGTACTGTCTGCGGCTTCGTTGCCTTGTCCTG
ATTTTTGTTAATCCGCTATATCAGAAATTACCCTACCGTTTTTAAACACTTTCAGGAAT
AAGGAAAAATGACCGCCCAACCCTGCCCCATCTGCACGGCGCAAAATGAAGACGTTTTGC
TGCAAACCCCCAACCTCCGCGTCATCGCCGTCCATAACGACAGCGGTTCGCCTGCATTCT
GCCGCGTCATTTGGCGTAAGCATATTGCCGAAATGACCGACCTTTCGGCAGCGGAACGCG
GCGAATTGATGGAAATGGTGTACAAAGTCGAAGCCGCTATGCGCCAAGTGTTCCGGCCGG
CAAAAATCAACCTCGCCAGCTTGGGCAATGTCGTGCCGCACCTGCATTGGCATATTATCG
CCCGCTTTGAAAACGATGCGTCTTTCCCCGCGCCGATTTGGGCAAACCCCGTCCGGAAAC
ACGGTATGACCCTGCCGCAAGATTGGACGGAACAGCTTAAAAAGCTGCTTTAAGCCCGCC
GATGCCGTCTGAAACCGTATGAAAGGGAAATTATGACCGAACCGACCTCCCGCCGCCGTT
TTCTGAAAACCTGCACCGCCGCTGCCGGCGCGGGGCTGCTTCAGGCTTGCGGCACATCCG
CCACATCCGTTCCGCCCCTTCCCTCTTCCCATTCCGTTGTGAAAGCCCGAACCGTGCCTC
TCCAAACGCCACGCCGTCAAAGTTCGGACGGCAACCTTCTGCGCGTTGTCGCTTCGTCAG
GATTTGCCGAAGACACCAACCGCGTCAACACAGCCTTAACCCGCCTTTACAATGTCGGTT
TTACCGTAACCAACCAACAGGCGGGCAGCCGCCGTTTCCAACGGTTTGCCGGCACGGACA
CGCAACGTGCCGCCGATTTCCAAGAGGTCGCTTCCGGCCGCGTCGCCACGCCTAAAGTGC
TGATGGGTTTGCGCGGCGGTTACGGTGCGGCGCGGATTCTGCCGCATATCGATTTTGCTT
CGCTCGGCGCAAGGATGCGCGAACACGGCACGCTCTTTTTCGGATTCAGCGACGTATGCG
CCGTCCAGCTGGCATTGTTGGCAAAAGGCAATATGATGAGTTTTGCCGGCCCGATGGCTT
ATAGCGATTTTGGCAAACCCGCCCCCGGTGCGTTTACGATGGATGCCTTTATCAAGGGTG
CAACCCAAAACCGCCTGACCGTTGATGTTCCTTATATCCAACGCGCCGATGTCGAAACCG
AAGGCATATTGTGGGGCGGCAACTTAAGCGTCCTCGCCTCGCTCGCCGGCACGCCTTATA
TGCCCGACATCGACGGCGGCATTTTGTTCCTCGAAGATGTCGGCGAACAGCCCTACCGCA
TCGAACGTATGCTCAATACGCTGTATCTTTCGGGTATTTTGAAGAAACAGCGCGCCATCG
TGTTCGGCAATTTCCGTATGGAAAAAATTCGAGATGTCTATGATCCGTCTTATGATTTTT
CTGCCGTTGCCAACCATGTTTCGCGCACGGCGAAAATCCCCGTGCTGACGGGCTTCCCGT
TCGGACACATTGCCGACAAAATCACTTTCCCTCTAGGCGCGCACGCCCGAATCCGTATGA
ACGGAAACAGCGGTTATTCGGTCGCGTTTGAAGGCTACCCCACACTCGATGCGTCCGCCC
TGACTTTGGATACCCTGCTCCCACCGCCGGATTTGCCCATCTTCCCCGAAAGCGGTGTTG
CCGATATTTCGGAATAAACCCGCAAACGGACAAATGCCGTCTGAAGCCTTCAGACGGCAT
TTCCCAAGACGGCGGCAGATTACAGCAATGCCCGAATATCGGCTTCGATTTCTTCGGGCG
TAACACTAGGCGCAAAACGCTCGACCACTTCGCCGTCGCGGTTGACGAGGAATTTGGTAA
AGTTCCATTTGATGTCGCCTTCGTCGCGCTTCTCTCCCAAAGCTGCGAGCTTCAACACGA
AATCTTTAAACAGATGATTGCCTTTATCTTGCGGTTTGACGGATTTCAGGTAGGCATACA
AGGGCGCGGTATTTGCTCCATTGACTTCGATTTTGTCGAAAATCTTAAACTTCGTGCCAA
ACTTCATCATACACACTTGGGCAATTTCTCCGCTGCTTTCGGGAGCCTGTTCGCGGAACT
GGTTGCACGGAAAATCCAAAATCTCCAAGCCTTCTGCGGTATATTGTGCATACAGCTTCT
GCAAAGCCTCGTATTGCGGGGTCAGACCGCAACGCGTTGCCGTGTTGACAATCAGCAGAA
CCTTGCCGCGATAGCCTGACAAATCAACCGCATTGCCTTCTGCATCTTTCATTTGAAAAT
CGTAAATACCCATTTTTATCCTTATCTGATGTAAACCGATGCCATCTGAAACGTGCTTCA
GACGGCATGAAAGCAGCAATTGTATAGCCGATTAAAATAAAAAATCCACATCCTTTTCCA
TTCCCGTCCCAATCCGCAATAAAAAACTGCACCCGAAAACGGGTGCAGTTGCTCATTTCA
TACCGCAAAACTTATTTGTCGCGGCCGAATACGATTTTAGTGGCTTGGATGGCGACACAG
ATTGCACCGCCGATAAAGACCAAGTCAGCTGCCGTACGTACCCAACGCAAGGTATCGAGG
ATTTCCATTTGCAGGAACTCTTCGCTGCGGGCATACCACAGACCGTGCGTGATGGAGGCG
TATGCCTGAATCGCGCCGACAGGCAGCAGGCTGATGGCAATCATACCGGCCAAGCCGCCG
TTGAGCAGCCAGAAGCCCCAAGTCATCAGTTTGTCGTCAAACTGCGCGTTCGGTTTCAAA
TAACGGGCAACCAGCAATACGAAGCCCAATGCCAAGAAACCGTACACACCAAACAAGGCG
GCGTGCGCGTGAACGGCAGAAGTGTTCAAACCTTGGATATAGAACAGGGAAATCGGCGGA
TTGATCAGGAAGCCGAATACGCCGGCACCGATCATATTCCAAAAGGCGACTGCCACGAAG
CACATCAGCGGCCAACGCAGGCGTTTCGCCCAGTCGGACAGGTGTTGGTAAGACCAGTGT
TCGTATGCTTCACGGCCCAGCAACACCAGCGGCACGACTTCCAAAGCGGAGAAGCAGGCA
CCGATTGCCATAGAGGCGGAGGTAGAGCCGGAGAAGTACAGGTGGTGCAGCGTGCCCGGA
ACGCCGCCCAACATAAAGATGGCGGCAGCGGCCAAAGTGGAGGCAGTGGCGGTACTGCGG
CGGACAAAGCCCATATTGTAGAAGACAAAGGCAAAGGCGGCAGTGGCAAATACTTCGAAG
AAGCCTTCTACCCACAGGTGAACCACCCACCAACGCCAGTATTCCATAACGGCAATCGGG
GATTTTTCGCCATAGAACAGGCCTGGTGCGTAGAATACGCCCACACCGACCATAGAAGCT
ACGAAGATAGCCAACAGGTTTTTGTCCACGCCTTTTTCTTTAAAGGCGGAAACCGTGCAA
CGCAACATCAGGAACAGCCATAACAGCAGACCGACCATCAAAAGGAGTTGCCAGAAACGT
CCCAAATCGAGGTATTCGTAACCTTGGTGTCCGAACCAGAAGTTAAATTCCGGGGGAAGG
ATGTGCGTCAACGCGAAGAAGTTGCCCGCGTAAGAACCGCCGACCACGATGAAGAGGGCG
ATATAGAGGAAGTTTACGCCGGCACGTTGGAACTTGGGATCTTTACCGCCGTTGACAATC
GGCGCGAGGAACAAACCTGCCGTCAAAAAGCCGGTTGCAATCCAGAAGATGGCGGATTGG
ATGTGCCAAGTACGGGTCAGGGCGTAGGGGAACCAGTCGGACATTTCAAAGCCCAACGCC
TCGTCAATGCCGTAGAAACCCTGGCCTTCGACGGTGTAGTGCGCGGTCAGTCCGCCCAGC
AATACTTGTACCACAAACAGGGCGACCGTCAGGAAGACGTATTTGCCCAATGCTTTTTGC
GAAGGGGTCAGTTGGATTTTGGAAATCGGGTCTTCAGACGGCACTTCCACTTCCTCGTGT
TTGGTCAGGAAGGAATAACCCCACATCAGCAAACCGATGCCCATCAGCAGAAGAACAACG
CTGGTGAATGACCACATATAGTTTTCAGTGGTCGGTACGTTGTTGATCAAAGGTTCGTGC

# Appendix A

GGCCAGTTGTTGGTGTAAGTAAAATTCTCGTCAGGACGGTTGGTCGAAGCAGACCAAGAA
GTCCAGAAGAAGAAGTTGAACAGTTTTTCACGCGCTTCTTGGCTTGGCAATGTATTGTTT
TTCATTGCAAAGTGTTCGCGAGTGGTTTGGAACTTAGGATCGTCGCTGTACACACCGTGG
TAGTAAGGCAGGATGCTTTCGATGGCTTTCACGCGCGTATCGCTGATGACGACGCTGCCG
TCTTCCTTCACGCGGCTTTGATTGCGGTATTCGTCGGCCAGGCGTGTTTTCAAGACGGCT
TGTTCCTCGGGGGAAACCTCGTCGAATTTTTTGCCGTAAGTCTGTTGCGCGGTCAAATCC
AACCAGGCAACCAACTCACGATGCAGCCAGTCCGCCGTCCAGTCCGGAGCCTGATATGCA
CCGTGACCCAAAATCGAACCGACTTCCATACCGCCGGTAGTCTGCCATGCAGACTGACCT
GCCAAAATATCGTCTTTCGTCATCAAGACCTTGCCGGATGCGGAAACGACCTGTTCGGGG
TAAGGCGGGGCTTTTTTGTAAACCTCGCTGCCCATATAGCCAAGAATGGTAAAGCATACC
GCCAGAACGGCAAACAGCAAGTACCAAAGCTTCTTGTACTGTCCCATTTTGAGAGCTCCT
TTTAATATAGTGGATTAAAATTCACAAAATATGAATGTTAAAGATTGTAGCACGGTTTAC
CGCGCAAATAAACATTTGTTCAAAGAAACTCACATATAAAACAAATACATATATGATAAT
AACTATCATTATTCTTTAGTCGGCAACTACCCTGCCTTTGCCTGATTTGCCGAAGCCCTT
AAGCAAATCAGCCTATTTATTGTAATTTTTAGTAGCTATAAAGTATTAGAAGTATCATTT
TAAGTTCATATTTTATGAATTATTTGACTTAAATCAAAATGCCCCCAATGGGGCAAACGC
ATAATCACACCAAGTTCTTAACCAATCCCTCTACTTTTCTTACAAAAGGAAAATATTATG
AAACGCCAAGCCTTAGCTGCAATGATTGCTTCCTTATTCGCATTAGCCGCCTGCGGCGGC
GAACCTGCCGCGCAAGCCCCTGCCGAAACCCCTGCCGCTGCCGCCGAAGCCGCAAGCTCC
GCCGCACAAACCGCCGCCGAAACACCGTCCGGCGAACTGCCCGTTATCGATGCGGTTACC
ACCCACGCTCCCGAAGTGCCTCCTGCAATCGACCGCGACTACCCCGCCAAAGTCCGCGTA
AAAATGGAAACCGTCGAAAAAACCATGACCATGGAAGACGGTGTGGAATACCGCTACTGG
ACATTTGACGGCGACGTTCCGGGCCGTATGATCCGCGTACGCGAAGGCGATACGGTTGAA
GTGGAATTTTCCAACAATCCTTCTTCTACCGTTCCGCACAACGTCGACTTCCACGCGGCT
ACCGGCCAGGGCGGCGGCGCGGCCGCAACCTTTACCGCTCCGGGCCGTACTTCCACATTC
AGCTTCAAAGCCCTGCAACCGGGTCTGTACATCTACCACTGCGCCGTCGCACCGGTCGGT
ATGCACATCGCCAACGGTATGTACGGTCTGATTTTGGTCGAGCCTAAAGAAGGCCTGCCG
AAAGTGGATAAAGAGTTCTACATCGTCCAAGGCGACTTCTACACCAAAGGCAAAAAAGGC
GCGCAAGGTCTGCAACCGTTCGATATGGACAAAGCCGTTGCCGAACAGCCTGAATACGTC
GTATTCAACGGTCACGTAGGTGCTATCGCCGGCGATAACGCGCTGAAAGCCAAAGCAGGC
GAAACTGTACGTATGTACGTTGGTAACGGCGGTCCGAACTTGGTATCTTCCTTCCACGTC
ATCGGCGAAATCTTCGACAAAGTTTATGTTGAAGGCGGCAAACTGATTAACGAAAACGTA
CAAAGCACCATCGTTCCTGCCGGCGGCTCTGCCATCGTCGAATTCAAAGTCGACATCCCG
GGCAGCTACACTTTGGTTGACCACTCTATCTTCCGCGCATTCAACAAAGGCGCACTGGGT
CAATTGAAAGTAGAAGGTGCAGAAAACCCTGAAATCATGACTCAAAAATTGAGTGATACC
GCTTACGCCGGTAACGGTGCAGCTCCTGCTGCTTCCGCTCCCGCAGCTTCTGCCCCGGCA
GCCTCTGCATCCGAAAAAAGCGTTTATTAAATTGGATACCCGTCATTAGCGGGACGAACC
ACTGCCGCTGTACTTCATTACGCACGGCGGTGGTTTTTTAACAACCAATCTTTCCTTTCG
GAAGATTGATTTTAACCGCCTGTCAGGAGGCTTTATGAAGTATGTCCGGTTATTTTTCCT
CGGCGCGGCACTCGCCGGCACTCAAGCGGCGGCTGCCGAAATGGTTCAAATCGAAGGCGG
CAGCTACCGCCCGCTTTATCTGAAAAAAGATACCGGCCTGATTAAAGTCAAACCGTTCAA
ACTGGATAAATATCCCGTTACCAATGCCGAGTTTGCCGAATTTGTCAACAGCCACCCCCA
ATGGCAAAAAGGCAGGATCGGTTCCAAACAGGCAGAACCCGCTTACCTGAAGCATTGGAT
GAAAAACGGCAGCCGCAGCTATGCGCCGAAGGCGGGCGAATTAAAACAACCGGTAACCAA
TGTTTCCTGGTTTGCCGCCAACGCCTATTGCGCCGCACAAGGCAAACGCCTGCCGACCAT
TGACGAATGGGAATTTGCCGGACTTGCTTCCGCCACGCAGAAAAACGGCTCAAACGAACC
CGGCTACAACCGCACTATTCTCGATTGGTATGCCGACGGCGGACGGAAAGGCCTGCACGA
TGTCGGCAAAGGCCGCCCGAACTACTGGGGCGTTTATGATATGCACGGGCTGATTTGGA
ATGGAGGGAAGATTTCAACAGCAGCCTGCTTTCTTCCGGCAATGCCAACGCGCAAATGTT
TTGCAGCGGCGCGTCTATCGGGTCGAGCGACTCGTCCAACTATGCCGCCTTCCTCCGCTA
CGGCATCCGTACCAGCCTGCAATCCAAATATGTCTTGCACAACTTGGGCTTCCGTTGCAC
AAGCCGATAACCCCTTCAATTATAGTGGATTAACAAAAACCAGTACGGCGTTGCCTCGCC
TTGCCGTACTGGTTTTTGTTAATCCACTATATTCCGCCATCTCTAAGATTTACAGCGATA
CACGGGTAATTTAAGGAATGCCCGAACCGTCATTCCCGCCACTTTCCGTCATTCCCGCCA
CTTTCCGTCATTCCCGCCACTTTCCGTCATTCCCGCAACTTTTCGTCATTCCCACGAACC
TACATCCCGTCATTCCCACGAAAGCGGGAATCCAGTCCGTTCAGTTTCGGTCATTTCCGA
TAAATTCCTGCTGCTTTTCATTTCTAGATTCCCACTTTCGTGGGAATGACGGCGGAAGGG
TTTTGGTTTTTTCCGATAAATTCTTGAGGCATTGAAATTCTAGATTCCCGCCTGCGCGGG
AATGACGATTCATAAGTTTCCCGAAATTCCAACATAACCGAAACCTGACAATAACCGCAG
CAACTGAAACGTCATTCCCACCACTTTTCGTCATTCCCACCACTTTTCGTCATTCCCACA
AGGACAGAAAACCAAAATCAGAAACCTAAAATCCCGTCATTCCCGCGCAGATGATATGTT
GCCCGTCAACACAAAATAAAAAACAAAGTTGCAATATACTGATTTATATTGTTATTTTTA
TTTACGTTTATTTACGATATGCAAATGCACGGTTACACAAATATATTCGCGTAACCGTTT
AATTTTGTTGAATTTTATTGATTCAATCGGTGTCTTTCCGCATCGTAAGGCTGGCCGGTT
TTAACAATGTAATAGGCGAGCTTCGCCAGTTTGCGCATGATGGCAACGATGATTACCATC
TTTGGCTTACCCGCTTTTTTCAGATTATTTATTAATTTCGGAAATGCGTTAAAACGGTAA
GCACAAAGGGCGGGCATATACAGCGTACTTTTTAATCGTCTGTTTCCGTATCGGCTCAAT
CTGCCCCGACCTCTTACGCTTGTCCCTGATTGTATGATGGCGGGATTTAATCCGGCATAG
GATACAAACTGGTTTGCGGTTTTAAAATGTTTTTCTGTCAGTTGCGCATAAAGAACTGAT
GCGGTGTCTTTGCCTATGCTCGGGATGGTTTGAAGATTGCGGTAATGGTTATTGTCCGTT
TGTTTTTTGATTTGTTCGGATATGGCTATTTTTACCTGTTCCATCTTGTCCTGTATGGTA
TCTATCAAGTCTTGATGTATGTTCCTTATGAAGTCTTCTTCAGTGCTATGAAGACGGTTT
TTAATTTGCTTCTGATGTTGATGTAATTGATTTTTAAGGTTAATCAGTTTTTGCAGTGCT
TTGTTTTTGGGTATCTGATACGGTATCAATGTATCTTGATGCCTTTTTATGTAGTCTGCT
ATCAGGTTTGAATCTGCTTTGTCGGTTTTGGTACGGTTAAACCTGCTTTTTCCGTAGTCC

## Appendix A

TTGATTTTTAAGGGATTAATAACGTAAACAGTATAGTAGGAAGAAAGCATATCTGCTGCC
TTTTCGTAATAGATGCCTGTTGCCTCCATGCCGATATAGACTTTTCTGATTCTGTTTCCC
TTTATCCACAATCTAAACTGTTTTAATCCATCATCATTATTCTTAAATTTAATGTAATGG
ATACTTCCGTTTGTTTTATGCAATGTTGCGTCTATGGTGTCCTTTGAGATGTCCAGCCCG
ATTATATTCATTGGTATTTTCCTTATTTATACAGCCTTGATACGGCTAGGATGATATTCA
ATTTCGAGGATGGATAAAGGCAGCCGGCATTTCTACGCGTCTGTTTTAATACATTGCGGG
ATTTGCTGCCTGACTGCCTTAGCCCTTGCTTTGCGCGAAACAAAGACCCGTAAACCGTCT
ATATTCAAACGGTTTACGGGTCTTTTTTCTCTCTTGCCGTTTTCTTCAGTTTGCCGATCC
GACCACGCCCCCGCCGATTCCTTCAAACGGTTTCCCGCGTTCTTCCCAATTATCGTACAT
TAGGTTCTGCTACGGTTTTCCGCCCAATGTGGCAACTTGCGCCCTGTCCGAATGTTGCTG
CGCGCTTTGCTGAACTTCCTGCCCTTGGCTTTCTTCTTTGTATGGGTTAAACGGCAAGCC
GTTTTTTACATAGTCCTTGCACATCAACTCCGTCACTTCTTTCAATGCCGTCCCTTGATG
CGAATAGCAGGCGCATCCGGTTCTTCCGCCTTCTATACAGCCTGCTATATATTCAAAGGT
TCTTACCTGCCTTACACCGTTATAAATCGGCTTGCTTTCGGGTTTTTCGGACAATGTCGG
AACAAACATATCTGCGGTAAGGTTGCCGTTATTTACCGGCTCGCCTTCTGTTTTATCCGG
AAGTACTGCCTGCTGTTCTGTTGCCGCCGATTCTTGTGCTGCGGGTTCTTCCTGTTTTTT
TCCGTAACTGCTCAACATTTTATAGGACAGGCCGACAAACACGGGAATCAGCAATACTAT
TACTGGCAGAGTGTAAAACCACTTTGACCGCTTGACCTTATTTACGGTATGAACTTCCGC
TGATTCGTACAAGTCATAAACTTTTTTATCCAGTGTATAGATACTGGAGAATGCGCTTGA
TGCCATTTTTACGGGATCGTCCGCGCATATTTTCCATTCTAAAAGCGTACGCATACCCAT
CTTGTTTGAAGCGATGTGGTAATGTTTCCGTACAAGCGTTCTAAGATTTTGATCTAGAAG
CTTAGGACCTTGAGTCAAAACAAATATATCAATGCCCTGATGTCTGTGCGTATTCAGCCA
TTGGACATTTTCAGGGATTTTTGAACCTGCCGAGCGTGCCGGCCATACGTCTTGAGCTTC
ATCTACAATGACAATAGACCCGATATTTTCGGGCTTCTTTATCCATTCGTACATATCATG
CGCCGAAAGCTGCTCATCTGTCGATTTCGGCAGCTTTTTTGCGTCCGTTTCTATGTAGGT
GTGCGGTATTTTCAAGCCTTTTATGTTCGTAAATACTTTACGGCGTATGCCGTTTTCATC
AGGCTTAAACATTTCATCATTCGCCATCATGGAAACCATTTTTAATGTTTTCCCTGAACC
GGGCGTGCCGGTTATCAAACAGATCTCTGCCATTTATTTTTTCTTCCCGATTGAGGTTGC
TAGTTTTGTCATTTGTTTGAATGACAGAATAAAGGCGATCGCGCCAAACAGGATATTAAG
AACGGTTCCACCGCCGCTTATATAAAAAAGCTGCAACATCGCTTGAGGCGCGCCCGTTAT
GCTATTGGTTATCGCCTGCTGAAAATGGGCTACCAATCTATCCACCCCTGAATAGGTTAC
CGCCATCAAGCCTAATGCAGTCAATATACGGCCTGCCACGCTCATCAAAAGCGGAATCAA
TGCGGCCAACAATTTCATTTGCTATCCCTTTCTTAAAAGGCACGGTTGCCTCATTAAACA
AATGTTTCTTAATCTGAAAGATTTTGCGCCGCATTCGCGGCGCGGCGGCGCTGTGGGGAC
ACCCCCCTCGCGCTTATCGCCAATCCCCCCGCGCTTCTCGGCTATTTGCGACATTCGTCG
CAAAGTGCGCTGCTTCGCCTGCCTTTCGGCCAAAGTTTCCGAAGCTGAACGCTTTGCGGG
GGACTAGTCCCCCACACCCCCTAGTCTCACTTGCGACGCCGCGGGGGCATGGGGACGGCG
CAAAAGGCGCGCGCCTTACCACCTGCCCTTGCGGCAGAATGTGTTCTTTTGGCGGGGCGG
CAAGGGGTATCCAAAAAGATTTATAAAGACGATAAAGCCGTCTTTACAAATCTTTCTGGA
CGTCCTCCCCCTGCCTTGGTACAAGTTACTGAAGCCCGGCGGTGCTGCGCCTGCTAGACT
TCACGAGATACTGTGCGGATACAAAAAAAGGCGGCAACCGCCCAAGCAAGGGCGAGAAGC
ATGTACCTTAGCCGTTCGGCTATGGTACATGCGTTCTCAAAGCTGAACGCGAACTGCCTG
CTTGAATCAAGCACAGTCACTGTGAAAGTGACAGGTGCGGGACACTGTGCGGAATCTTGA
AAGATTCCTGATTTCTGAAACTCTACATTGACGGTTTCAGACGGCAGATTTAAATCTTCT
GCCGGATTGGACTCGGGCAGCCTGTCGCAAGCGAGAATGTCGGGGAAGAATTTGCACAAA
AGGCCGCCATCTTTGCCGTCCTTTCCGTCTTTGCCGTCCCTGCCGTTTGTGCGTCCCGGA
ACGGCGGGGGAATCGGGTCTTGTGCCGGGCTGTCCGTCCGTATCGGGATTTGCATCGGGA
TTCAAATCGGGGTCGGGTTCGGGATTGGGGCTCGTGCCGGGGTTCTCATTGGGGTTCGGG
TTGTTTGCGGGGTTTTCGGCGGGCGATACTTCGGGCAGCGGCTGTGCGTTCGGTGCTTCC
GCGCTTCCGGGGGTCAAGTCGGGACGCGGGATTACTTGAACATGCACGGTGGTGTTGCCT
TGCGAATCCCTGCCGAATGTTGCGACAACCTGAACGGGATTCCCGTTCCTGTCCGTGACG
GGACCCATATTCACTTTTGTTCCGGGTGCGACTTCTACTTTTTCGGAATAACCGGGATAA
CCGGTTGCCTTTATGTATTTGTCGGGATTGGCATCGACTTTCAACGATAAAATCTCTTCC
AGCTTTTTGGCATCCATTTCTTCTTTGTATTTTGAATTGCGAATAAGGGAAAAATCAGCC
CCATTTCTGAAATCATCACCTTTATTGACCAAACAATCTCCGCCATTCCAATTAAATGTG
CAACGATTTAAAACAAAATTATTCCAATCCAAAGAACTTAATTTATTCAGTTCTTCTTTA
TGCCAATTCCAAAACGGACGTGCCAGCCTATACATTTGGCTTTCCATCAATTCTTTGACT
TCGGGGAATCTGCTGTCATCGGACATAAGGCGCATAATCGAACTGTCAACGCCGTAGCAG
CCATAGGTTCTATTAATACGTCTTTTGTCTTCGTACCAAAGGCAATTACTATATTCGTAG
CCTTTTACAAATTTGTCGGTTTCGGGGTCGTATTGGTAGCCTTGTGCCTGTATGTCTTCT
TTGAAAGTTTCGTATACGTCATGGGCTAAAAGGGCTGTTCCGACATAAGGAACTGCCCTT
GTGCTTAATTTCGCGCCTAAGCGGGCAAGTTTGCCGACTCCTGACAAGACGGCGGCGCGG
GAAACTGATGCGGTAAATTTAACGGGGACTTTTTCAAGAGAGCGTGCACCTGTTGGCACG
TGTTCTATTATTGAAGGTTCGATAATTCGACCTGAAAAACGCTGACCATCAACACGAAAA
TCGGTAACTACAGATTTTTTATGATCAATATCCAATCTAACATCTGAATTTCCAATAGGA
TACTTAAAACGTTCAGCATAAGAATTAACCGAAAACATCCCCAACATTAGGATTAAAATC
AAACGTTTTAATTTCAATTCCACGACTATAATCATCCTGTAATTCTAAAATTTTTATATA
CGCAACAGACTCATCAGAAAAATAAATTTTCCAAATATTATTAGAAATCCTTCTATTTAA
AAAACATTGGATTTCTTCATGAATTATAAATTTATTAACTTTTGAATAATCCAAAAGCTC
ACTAGCGAAAGTATATGCCATTGTTTTACCATAATACTTGCTTGACGGCTGATATTTATA
AAGTGCCAACTGCGCCTGCGTGATAAACGGTTTGTTCATTGTTCTGCCTTTCAAAGGTTG
TTTTGAAAGCCTGATTTTAAAACACGTCATTTAAATATCAAAGCGACAGACAAAGCCAAG
AAGAAACCGAGAAGAAACCAAAAATCAATAACCATCAATCAATCCCAACCTTGCCCATGT
CTTTTAAAAAATTAATCAAAAGCCTGAAGCCGTAAATGACTACGAACAGAATTAAAACCG
TCGAACCGACATAAGAACCCTGTTTGATCTGCTCAAAATTGGAACATTTCGGATAGGACA

## Appendix A

ACATTACCGGCTTTCCGTTCAAGACCCATTTTTCGCCCACCCTTTCCGGCCTGATGATTT
TTCCGTCCTGGGTAACAGTAGGAGGAAGGGACGACAATAAATAGTCGTCTGCATGCAATC
TTGTATCAAAACAATTTATGCCGACACGATAGCCCATTTATACGCCCCTTTTTCTTCACT
CTGTTTATTTGACAGATTTAATCATGCTCCAAGCCATTTTGAAGCCTTGGATTGCAAGAA
TCACGGTAATGGCCGCCATACCCACGGCGGAAACCATTGACACGAAACCCATGATTACAT
TCGCTACTTGCGTACCAATCGCGGATGGATCAAAGGTATCTGCCATAACAATGGCCGGTG
TGAAGATACCGGCTGCCAAGGCTGCTTTTACAGCGTATTTTTTAACGATGTTCATCGTTT
TTTTCCTTTTTTGATATTTAAAATAAGACGACTTCTTGACTTGCTTCATCCGGACGAAGT
CTTTTCCGAATCTCGTTTTTAGCCGATAAAATAGAGGATTGCGAAAAGAAAAAGAAACAT
ACAGACCACCCAGCCGATAATTAGTGTTGCAAGGTTCATTTTCATGATATTTTTCCTTTG
TTGCGGGCTTTGTGAAAGGTTGACAGACCGCCCGCCGAGCCTGTTTTTCTTTTATTCCGA
TTTTACGAAGAACTGAAATATCTGGAATCCTCCGCCTATTTCATTTATGCCTGAATTCAA
CGCATCTTCGTAGCTTTCAAATTGACCTGCTGATTTAATATTTTGAGTAAACCCCACATC
ACCGAAAGGATCGGGATAAATAAAGTCATGCGTTTCCAAGTCTTGAACTATGAAACGTTC
TTCAAATTTCATAAATCAACCTTTCGGCTTTTCTGCCACCTGAAAATCAATTAATGAAGG
AACCATGCCCTTACCTGTCGAAGTCATTTCAACCGTTACCATAACTTCGCACGGGTATTT
GAGATTCTCTAATTTTGAGAAATTCTTACTGTCCCCGAACTTCATTTGTGCTGCCGTGAA
TCCAACAGCATTTCCCGACTGTGCCGGCAAAGGTGTTGCAACCAATACGGAACAAGTGTC
GATATTAGAGCCATCAATTTCGCCTTTGAATTTTTAGCTCCTAAAAAAGTTGCGGGATA
AGTTACAGTTTGAGTTTGATTAAACATATTAATTTTTCCTTTTTAGGTTAATTTTGATTT
GCATGAAGATCATACATTCTGTCGAGATAAAGCTGATATTGCCTCTCACTTTCCACATCG
TGATGTGGATCGAAAAGCCTTTTATCCGGATCGAATTTATCTGATTGCTTAAATTTGATA
ATTCCGAGTTCTTCCAATTCAACCTCTAAATCTACATCCGGTTGTTCGTGGATAAAGCCG
AATTTCAAAGATTCCTTCAATCCGGCCAACGAATATTTTTCAGGTTCTAGCCCTTTGGGA
TACCCCAAATCTGCCTTCAGATATCTGACAATTTCATCACTATCAAAACCCATATCAAAC
ATGAAATTAATCAGTTTGCCGACCGCGTTTTTTGCGTATCTCAATTTATGCTGAAAAGTT
AAATTAGCCACTTTTTTACGGTAATCGAACCTTTCCGGATTCGGCATATTTTTAAATTTC
TGACAAATCGGGAAAGCGCCTGAAAAGTAAGAACCTTGATTTATCAGAATATCCAAAGGT
ATTTCCATATCTCCATGATTAAACTGAATTTCGAACCTTACCCACTTGCTTTCTTTATCG
CCTAGCTGCCTGCCTTTCTCATAAACACGCACAAAACGAGAATTTTTCTTGCGACCTACA
TAAAATGTCTTGCCGCTCCCGTCCTCTCTCCGCCAAGCCGTTCCAACCATTTCAGATTTC
GGCCTCATGTTACTGTTATCGAAAAAACCGTTATCGTGATCCAAAAGTGCCTGTTCCGGC
GTGTACTCCCCATCAAAAAAATCAAGTGCCAAATCTACCCGCGTTATCCTCGGCCTCAAT
GAATCTTCCAAAAACTGCTTAAGCCTCAATTCCCAACCTGGATTTGCAATGTTGCAACCT
ACACCTTTCAATTCGATTAAAACCGTATTTCGCTGACCTCCGTAATGGACTTCGCCGTAG
TCAACTTCTTCCGATCCCAACCTAAACATCGAATCGTAAAATTTATTGCCCTTCGATTTG
CATCTGCTCGTGATGCCAAACCCTAATATTTCCTCCAATTTTTTGCTTAAAACAAACATA
TATTCGGCATCGGAAACTAAGGGGCATCCGGAAACTTTCAGCAAGGAATCTTCGTGCAGT
GTGAATGACAACCAATCTATAAAAACGCCGTCCTGCCTGCCCCTACGTTGCGGAATTTCT
AATAACTTCCCATTGCCGTTAGATATGAAATGGGAAAAATATTCTGCTTCACTCATTTTG
TTCAGTACCTTTAGGGATTTGTTTTATTTCGCTCCCCCCCTGTTAGTCAGGGGGGGGCTT
TCAGCCGTTTCCCGTCTGCCGCGCTAAAGCGCGTCCAACGGTCAACGACCGAAAGCCCAA
TCCTGACAAACTGTTAAAGATCAAGAAGAAAGACCACAACCGTCTGTTGTGATAATTACC
GGAAAATTCGAGCCAACCGAATCTATATAATCGAACGCCTGATAAAGCTTTGAAAAATTT
TCTTGTTCAGCGAGTTTATGCGGTTCACCATGCCTGAACTGATAGAAACATAAAACGCAA
TAATCTGATTTTTTAAATATTCTCCAATAGGAACAAGAAAATATTACATTTGCTACTGAC
ATAAAAAAGCCCCTTTCACTTGGCTGTCAAAGGGGAATGTTAAGAAAAGTAATGCGCCCC
TTTGATAGAGCGCATCATATAAGGCGGGAATCCAGTCCGTTCAGTTTCGGTCGTTTCCGA
TAAATTCCTGCTGCTTTTCATTTCTAGATTCCCACTTTCGTGGGAATGACGGCGGAAGGG
~~TTTTGGTTTTTTCCGATAAATTCTTGAGGCATTGAAATTCCAGATTCCCGCCTGCGCGGG~~
AATGATGAATTCATCCGCACGGAAACCTGCACCACGTCATTCCCACGAACCTACATCCCG
TCATTCCCACGAAAGTGGGAATCTAGAATCTCAAACTTTCAGATAATCTTTGAATATTGC
TGTTGTTCTAAAGTCTAGATTCCCGCCTGCGCGGGAATGACGAATCCATCCGCACGGAAA
CCTGCACCACGTCATTCCTACGAACCTACATCCCGTCATTCCCACGAAAGCGGGAATCCA
GTCCGTTCGGTTTCGGTCGTTTCCGATAAATTCCTGCTGCTTTTCATTTCTAGATTCCCA
CTTTCGTGGGAATGACGGCGGAAGGGTTTTGGTTTTTTCCGATAAATTCTTGAGACATTG
AAATTCTAGATTCCCGCCTGAGCGGGAATGACGATTCATAAGTTTCCCGAAATTCCAACA
TAACCGAAACCTGACAGTAACCGTAGCAACTGAACCGTCATTCCCACGAAAGTGGGAATC
TAGAATCTCAGACTTTCAGATAATCTTTGAATATTGCTGTTGTTCTAAGGTCTAGATTCC
CGCCTGCGCGGGAATGACGGCTGCAGATGCCCGACGGTCTTTATAGCGGATTAACAAAAA
TCAGGACAAGACGACGAAGCCGCAGGCAGTACAAATAGTACGGAACCGATTCACTTGGTG
CTTCAGCACCTTAGAGAATCGTTCTCTTTGAGCTAAGGCGAGGCAACGCCGTACTGGTTT
TTGTTAATCCGCTATAACAGCAACCTTGTCGCCGTCATTCCCGCAAAAGCGGGAATCCAG
TCCGTTCAGTTTCGGTCATTTCCGATAAATTCCTGTTGCTTTTCATTTCTAGATTCCCAC
TTTCGTGGGAATGACGGCGGAAGGGTTTTGGTTTTTTCCGATAAATTCTTGAGGCATTGA
AATTCTAGATTCCCGCCTGAGCGGGAATCCAGTCCGTTCAGTTCCGGTCATTTCCGATAA
ATTCCTGCTGCTTTTCATTTCTAGATTCCCACTTTCGTGGGAATGACGGCGGAAGGGTTT
TGGTTTTTTCCGATAAATTCTTGAGGCATTGAAATTCCAGATTCCCGCCTGCGCGGGAAT
GACGGCTGCAGATGCCCGACGGTCCTTATAGTGGATTAACAAAAATCAGGACAAGGCGGC
GAAGCCGCAGACAGTACAGATAGTACGGAACCGATTCACTTGGTGCTTCAGCACCTTAGA
GAATCGTTCTCTTTTTTGTTCATCCGCTATATTGTGTTGAAACATCGCCACAAACCTGAT
ATAGTCCGCTCCTGCAACATCATTGAAAATCTTTCTTTTTAATCAGTTAAAACCGAATAC
GGAGTCGAAAATGAATCCAGCCCCCAAAAAACCTTCTCTTCTCTTCTCTTCTCTTCTCTT
CTCTTCTCTTCTCTTCTCTTCTCTTCTCTTCCGCAGCGCAGGCGGCAAGTGAAGACGGCA
GCCGCAGCCCGTATTATGTGCAGGCGGATTTAGCTTATGCCGCCGAACGTATTACCCACG

## Appendix A

ATTATCCGAAAGCAACCGGTACAGACAAAGACAAAATAAGCACAGTAAGCGATTATTTCA
GAAACATCCGTGCGCATTCCATCCACCCCCGAGTGTCAGTCGGCTACGATTTCGGCGGCT
GGAGGATAGCGGCAGATTATGCCAGTTACAGAAAATGGAGCAACAATAAATATTCCGTCA
ACACAAAAGTGTTGAAAGAAAACCAGGGCAACAGGATAAAACTGAAGACGGAAAATCAGG
GAAACGGTACGTTCCACGCCTCTTCTTCTCTCGGCTTATCCGCCATTTACGATTTCAAAC
TCAACGATAAATTCGATAAATTCAAACCCTATATCGGTGCGCGCGTCGCCTACGGACACG
TTAAACATCAGGTTCATTCGGTGGAAACCAAAACCACGATTTATACCACTGCACCAACGG
GAGACGCTACAGTGGGAGGCACTATCCCAGAGAGACCGAGTAGCAAACCTGCCTATCACG
AAAGCAACAGCATCAGCAGCTTGGGGCTTGGTGTCATCGCTGGTGTCGGTTTCGACATCA
CGCCCAAGCTGACCTTGGACACCGGATACCGCTACCACAACTGGGGACGCTTGGAAAACA
CCCGCTTCAAAACCCACGAAGTCTCATTGGGCATGCGCTACCACTTCTGATTCCCCGATA
CCGATGCCGTCTGAACCTTCAGACGGCATGAGACCTTTGCCTGCGTACTTGGTACGCTGG
TCGCCTCCGAACATGGCGCGACACCCGACATTTCCGCCGAACGCATCGGGCGTTTCATGA
ATCCGGTTTAAAACGCATGGAAAAATGCCGTCTGAAAGCCTTTCAGACGGCATTGTGCTT
GAGATTCCGTTTACCAATGGCTGACAAACGCTTCCAAATCGGTATTCTTGGGCTTATGCA
CTTCCTCTGTCGGCGTGCCGACCATCATCAGCCCGATGATTTTATCCTTATCCGCACAAC
CGAAAGCCTCCCGCAACAGGGGGCTATTGACCCACATCCCCGTAATCCAGACATTGTCGA
ATCCCTGAGCCGTTGCCGCCAGTTGCAGCGCATACGCCGCACAACCCGCCGTCAGCATCT
GCTCCCATTCCGGTTTCGGCTTAGGCACATCGCGGTTCGGCGCAAACGTTACCCCGATAA
CCATCGGCGCCATATTGCCCACTTTTTCCGCCTTTTTCATCGCATCGTCGCCGAAATTCA
ATTCGGCAACCGTTTGCTTCAACACATCGCGAAAACGTTGCAATCCTACCTCGCCTTGAA
TCACGGTAAAACGGAAGGGGCGCATATTGCCGTGATCGGGAACTTGGGTTGCCGCCTGAA
ATATTTGTTCCAACTCCGCCGCATCGGGGGCGGGGTGCTTCAGCTTTTTGGAAGATCGGC
GGTTCGTCAATAATTTTAAAGCATCCATATCGTTATACTCCGGTCATCGGTCGGGTGTTC
GGACACATATGCCGTCCGAAGGCTTCAGACGGCATATCCGGCATCAGCGCGGACGGCGGC
AGGCTGCCAATATATCCATTTCCTTCCGATAGGTTTGGCTATTGGAAATGTCCATCAGCC
CCAATACCGTGCTGAAATAGTGGTCGTGCGAATATTCGTTTTCCGCCGCTTTTTGTTTGA
GGCATTGGAAATCTATGCCCCCGTGTTGGCGGAAGGCTTTGGAAAACCACATAACCATCG
GGATATGCGTCTGCCCGGAAGGCGCGATGGCGTAAGGCGCGGCGTGCAGGTACATCCCGT
TTTCGCCCAAACTTTCGCCGTGGTCGGAAACATAATGCACCACGCTTTCCAAATCGTCGC
GGTTTTCAAGTTTGCGGATAACCTTGTCGATAAACTGGTCCACATACAAAACCGTATTGT
CGTAAGTGTTGACCAGCGTGGCGCGGGTGCATTTGTTGATTTCGTTGGTGTCGCAGGTCG
GCGTGAATTTGCGTTCGGCTTCGGTATAGCGTTCGTAATACGTCGGCCCGTGGCTGCCGA
TGGTATGCAGGATTAAAACCGCGTCTTTATCGTTTTTGTTGAGGACTTCGTCGAACTTAG
TCAGCAGGATATTGTCGAGGCACTCGCCGTTGCGGCAGTATTCGGGCAGGTTGAGCGAGG
TAACGTCGGTATTCGGCACTTTGCCGCACACGCCCTTGCAGCCGGAATCGTTTTCCAACC
AAGTAACTTCCACGCCGGCGCGCTGCACGATGTCCAGCAGGTTGTCTTGGTGTTCGGCTT
TGATTTCGTCATAATCCGTGCGGTCGAAGGTTGAGAACATACACGGCAGGGAGTGCGCGG
TCGATGTGCCGCAGCTTCTGACCTGCGGGAAATTGACAATTTCATCGCCGCGCGCGGCAA
GCAGCGGCGTAGTTTGGCGGCTGTAACCGTTCAAACCCCAGTTGGCGGCACGCGTGGTCT
CGCCCACGACCAGCACCACGAAACGGCGCAGGCTGCCGGCCGGGCGGTTTTGCACGACCG
CCATATCCAATTGCGTATAAGGAATATTGGAACGCTTCCAATCTTTGTATTTCGACACGC
CCGCGCCGATGAAATTAGACGGCACAATCAGATGGGTTACTGATTTATTGTTGCGGAAAA
ACGAGGCGTAATCCTGATATTGCAACATTGCGATGCCCAACGCGCACAAAAAGGAAACGG
CGGCAAGCACAAGGCGCGTCAAAAGCTCCTTATACCAAACGCGGTATTTAACCTTGACGG
CGATATACGCCAGCGCGGGCAATACGCCCAAACATACAATCCACAGCACATAGCCCGGCG
TAATCAGGCGCGCGCTTTCGGCAGCCGTAGTTTGCAAGACATTATTCAACATCGACTTGT
TGAAATAGATATTGAAAAATATTTCTTGGTAAGACACCGCCGCACTGATAACCAATATCA
ACGGAATCAATACCTTATGCACGAAAGGCAGGGCAATGACGTGAAAAACGAAATTACTTA
~~AAAAAAACAGCACCACCGGCATCGTATAGAGGAAGATATCCGCCCCGGTGCCGTTAAAAG~~
GATGAAGCTCGACAACTTTGGCAAAAAAGGCGTAATTCAATACCAGCGAGGAATACAGGG
AAAGGAAGGCAATCAGCGCGGAAGAGCCGAGCTTCGGCCTCAGGTTCGGTTTTATCATTT
GGAATGTGTCGGATAAGGGTTGGAAAAGGCATCCGGCATTTGGAATCCGGATTATTGAAA
AAGATTCTTAATTATAAGGCAACGGAGCAAAGCAGGGCAAGAAAACGGCGGCTGTGCGGG
GGGTTCCGCCCGCCATTCAAACGTCCGGCAGACATAAAAACATCGTAAGCAAGATTCGAA
CCGGTCTGCAACCGCCCCTGCCAGAAAAACGGGCAAAGCATTTCATATTGGAAAAACCCA
GCCGCGCCGCCGACGGGACAGTCCGGCACAAACAGCATCACGCTCGGATTGAAAAGGACG
GATAGCCGCCGGCAGCACAATCTTACCACCTCCAAAACGCCGCCGGAGAACGCCGAAACA
GCCGATGCCGTCTGAAGCCGCTTCAGACAACATCGGGACATCAACCGTAACGCCGTTGGA
AATCGCGCATAAAATCTGCCAAAGCCCGCACGCCTTCAAGCGGCATCGCATTATAAATGC
TGGCACGCATACCGCCGACGGTTTTATAGCCCTTAAGCAGGCACAAGCCCTGCAATTCGG
CTTCCAGCACAAAACGGCGGTCAAGCTCCTCATCCCCCGTTTGGAACACGACATTCATTT
TAGAACGCGCATTCGGACGGATACGGTTGATATAAAAACCATCGCTGCCGTCTATCGTCT
CATACAAGGTTTGCGCCTTCAGCCGATTGACCGCTTCAATTTTTTCACACCGCCCTGCG
CCTGTAGCCAGCGGAACACCAGCCCCGACATATAAATCGCGTAAGTTGACGGCGTGTTGT
ACATACCGTCGCGGTTGATGTGCGAACGGTAGTTGAACACATCGGGAATATCGTTCGGAC
AACGCTCGAGCAAATCCTCACGCACAATCACCACCGTAACTCCTGCCGGCCCGATGTTTT
TCTGTGCGCCTGCGTAAATCAGTCCGTAGTCGGCAACATCAAACTCGCGCGACAAAATCT
CGCTGGACATATCGCACACCAGCGGCGGCATGCCTTCTGAAAGGCACGGCACTTCACGGT
ATTGCAGCCCGTTGACCGTTTCATTGACGGCAAAATGGACAAACGCCGAATCGGGTGCAA
CATCCCACGTTTCCACAGGCGGCAGGTCGAGATAGTCGAACTGCTCGCCGCCATGCGCCG
CCAAACGGATTTCCGTATCGGTCAAACGGCTCATCTGTTCATAAGCGATACGGCTCCAGT
TGCCCGTTACCACCGCGTCGGCAGTGCGGAAACCGTGTGCCAGATTCATGGCTGCCATAT
TAAATTGGGTTGTTGCTCCGCCCTGCAGAAACAATATCTTATAGTTGTCAGGCACTTTCA
AAAGCTGCCTCAAATCCTGTTCCGCATGATGCAGGATGCTCAAAAACATTTCCGAACGGT

## Appendix A

GGCTCATTGCCATCACAGGAAAACCCGTACCGTTGTAGTCCAACATTTCCTGCCGCGCCG
TTTCCAACACGGCTTCGGGCAATACGGCAGGGCCGGCGGAAAAATTGTAAATCGGATAAA
GAGACATGATGCAGCCTTGATTCTGAACAATAACCCGCCCGATTTTAGGCTTGCAGCAGG
CTTTGTGCAAGGATGGAAAATACCTGTCCTCCGCCCGATTCCATGCCGCCCGAACACGGA
AAATAATATCAATATATTGATTTACAAACATAAAAATCATGCACGCGACAAATAGATACA
TTTGTTTTGTCAACAATATTCACGATTTCCCATTACAAACCTCCCTTACACCCGCTTTTT
TCCGTCCCAAAAACACAAAATAAATCAACACTTTCATTTCTCCGCAAAAGCGGTTATAAT
CACGCCGATTTTTCAACTTTGACGAAAAATGGCCACGGGGCCATTTTTTTATTATGTACA
TAGGGAGCAGCATGGATATCCAAACCATCCTCGAAAAAACCCTGCCCGGCCTGGGCTACG
AACTGGTCGATTTCGAACTGACCGCGCAAGGAACATTGCGCGTGTTCATCGACAAAGAAA
GCGGCATTACCGTCGAAGACTGCGCAACCGTCAGCAACCACTTGAGCCGCGTCTTCATGG
TTGAAGACATCGACTACAAAAACCTGGAAATTTCCAGCCCCGGACTCGACCGCCCCTTGA
AAAAAGCCGCCGACTTCGTGCGCTTTGCCGGTCAGAATGCCAAAATCAAAACCCGCCTGC
CGATAGACGGTCAGAAAAACTTTATCGGTAAAATCGAAGGCTGCGAAAACGATACCGTTA
CCGTATCCTTCGACGGCAAAACCGTACAAATCGAATTGGGCAACATCGACAAAGCCCGTC
TGCGCCCCGAATTCAAATTCTAAAACACAACAATATTGGAGATGTTCAAAATGAGTCGTG
AAATGTTACAGCTGGCAGAAGCACTGGCAAGCGAAAAAAACGTTGATGCGGAAGTCGTCT
TCCAAGCACTGGAATTCGCCCTGTCTACCGCCGCCAAGAAAAAGGCAGACCGCGAACACA
TGGACGTGCGCGTCCAAATCAACCGCGACACCGGCGAATACCAAACCTTCCGCCGCTGGC
TGATTGTCGCCGATGAAGACTATACCTATCCCGATGTCGAAAAAACCATCGAGGAAATCC
AAGAGGAAATTCCCGGCACTACCATCCAAATCGGCGAATACTACGAAGAGCAGCTGCCCA
ACGAAGGCTTCGGCCGCCAAGCCGCGCAAACCGCCAAACAAATCATCCTGCAACGCATCC
GCGATGCCGAGCGCGAGCAGAATCTGAACGAGTTTCTCGCCGTCAAAGAAGACATCGTGT
CCGGCACGGTCAAACGCGTCGAACGCCACGGCATCATCGTCGAAGTCGTTGCCGGCAAAC
TGGACGCGCTGATTCCGCGCGACCAAATGATTCCGCGCGAAAACTTCCGCAGCGGCGACC
GCATCCGCGCCCTCTTCCTGCGCGTCGAAGAAATCGGCAACACCGGCCGCAAACAAGTCA
TTCTGAGCCGTACTTCCGGCGATTTCCTCGTCAAACTGTACGCCAATGAAGTACCTGAAA
TTGCAGACGGCATGCTTGAAATCCGCGCTGTCGCCCGCGACCCGGGACAACGTGCCAAAG
TCGCCGTCAAAGCCAACGACCAGCGCATCGATCCGCAAGGCACCTGTATCGGCGTTCGCG
GTTCGCGTGTCAATGCCGTCAGCAACGAATTGTCCGGCGAGCGCATCGATGTCGTCCTCT
GGTCGCCCGAACCCGCGCAATTCGTGATGAGCGCGCTCTCACCCGCCGAAGTCAGCCGCA
TCGTCATCGACGAAGACAAACACGCCGTCGATGTCATCGTTGCCGAAGACCAGCTCGCGC
TCGCCATCGGGCGCGGCGGTCAAAACGTGCGCCTTGCTTCCGACCTGACCGGCTGGCAGC
TCAACATCATGACTTCCGCCGAGGCAGACGAACGCAATGCGGCAGAAGATGCCGCCATCC
GCCGCCTGTTTATGGATCACTTGAACGTGGACGAAGAAACCGCCGACGTACTGGTTCAGG
AAGGTTTTGCAACCTTGGAAGAAGTCGCCTATGTTCCTGCCGCCGAACTGCTTGCCATTG
AAGGATTTGACGAAGAAATCGTCGATATGCTCCGCAACCGCGCCCGCGATGCCATCCTGA
CCATGGCGATTGCCGCCGAAGAAAAACTGGGCGAAGTGTCCGACGATATGCGCAACCTCG
AAGGCATAGATGCCGATATGCTCCGCAGCCTTGCCGAAGCAGGCATTACCACCCGCGACG
ACTTGGCAGAGCTTGCTGTGGACGAACTGATTGAAATCACCGGTGTAAACGAAGAAACCG
CAAAAGCCGTCATCCTGACCGCACGCGAACACTGGTTTACCGAAGACAAATAAAGGGGGT
ACAGATGAGTAACACAACCGTAGAACAATTTGCCGCCGAGCTGAAACGCCCCGTCGAAGA
CCTGTTGAAACAGTTGAAAGAAGCCGGCGTCAGCAAAAACAGCGGCAGCGATTCCCTGAC
GCTGGACGACAAACAGCTTCTGAACGCCTACCTGACCAAGAAAAACGGCAGCAACAGCAG
CACCATCAGCATCCGCCGCACCAAAACCGAAGTCAGCACCGTTGACGGCGTAAAAGTCGA
AACACGCAAACGCGGACGCACTGTCAAGATTCCTTCTGCCGAAGAATTGGCAGCACAGGT
AAAAGCCGCCCAAACCCAAGCCGCACCTGTCCGGCCGGAGCAGACGGCAGAAGACGCGGC
AAAAGCCCGAGCCGAAGCTGCCGCACGCGCAGAAGCCCGTGCCAAGGCAGAAGCGGAAGC
GGCAAAACTGAAAGCGGCAAAAGCAGGCAACAAAGCCAAACCTGCCGCGCAGAAACCCAC
CGAAGCAAAAGCCGAAACCGCACCCGTTGCGGCGGAAACCAAACCCGCCGAAGAAAGCAA
AGCGGAAAAAGCCCAAGCCGACAAAATGCCGTCTGAAAAACCCGCCGAGCCCAAAGAAAA
AGCCGCCAAGCCGAAACACGAGCGAAACGGCAAAGGCAAAGATGCCAAAAAACCGGCGAA
ACCTGCCGCACCTGCCGTGCCGCAACCCGTGGTCAGCGCGGAAGAACAGGCGCAACGCGA
CGAAGAAGCACGCCGTGCCGCCGCACTTCGCGCCCACCAGGAAGCCCTGTTGAAAGAGAA
ACAGGAACGCCAGGCACGCCGCGAAGCCATGAAACAACAGGCAGAACAACAGGCAAAAGC
CGCACAGGAAGCCAAAACCGGCAGACAGCGTCCCGCCAAACCTGCCGAAAAACCGCAGGC
AGCCGCGCCAGCCGTCGAAAATAAACCTGTCAATCCGGCAAAAGCGAAAAAAGAAGACCG
CCGCAACCGCGATGACGAAGGTCAAGGCCGAAACGCCAAAGGCAAAGGCGGAAAAGGCGG
ACGCGACCGCAACAATGCACGCAATGGCGACGACGAGCGCGTACGCGGCGGCAAAAAAGG
CAAAAAACTCAAACTCGAGCCGAACCAACACGCCTTCCAAGCACCGACCGAACCCGTCGT
TCATGAAGTTTTGGTTCCCGAAACCATTACCGTTGCCGATTTGGCGCACAAAATGGCGGT
CAAAGGCGTGGAAGTGGTCAAAGCCCTGATGAAGATGGGCATGATGGTTACCATCAACCA
ATCCATCGACCAAGACACCGCCCTGATTGTGGTGGAAGAACTCGGCCACATCGGCAAACC
TGCCGCAGCCGACGACCCTGAAGCATTCTTGGACGAGGGCGCGGAAGCAGTGGAAGCCGA
AGCATTGCCGCGTCCGCCCGTCGTTACCGTGATGGGCCACGTCGACCACGGCAAAACCTC
GCTGCTGGACTACATCCGCCGTACCAAAGTGGTACAGGGCGAAGCGGGCGGCATTACGCA
GCACATCGGCGCGTACCACGTTGAAACCCCTCGCGGCGTGATTACCTTCTTGGACACCCC
GGGCCACGAAGCCTTTACCGCTATGCGCGCACGCGGTGCGAAAGCAACCGACATCGTGAT
TCTCGTGGTCGCCGCCGACGACGGCGTGATGCCGCAAACCATCGAAGCGATTGCCCACGC
CAAAGCTGCGGGTGTACCGATGGTGGTTGCCGTCAACAAAATCGATAAAGAAGCCGCCAA
CCCAGAGCGTATCCGCCAAGAGCTGACCGCACACGAAGTTGTGCCTGACGAATGGGGCGG
CGATGTACAGTTTATCGACGTTTCCGCTAAAAAAGGCCTGAACATCGATGCATTGCTCGA
AGCCGTCTTGCTCGAAGCTGAAGTTTTGGAACTGACCGCACCTGTCGATGCGCCCGCCAA
AGGCATCATCGTCGAGGCGCGCTTGGACAAAGGCCGCGGCGCGGTTGCCACATTGCTGGT
TCAAAGCGGCACGCTGAAAAAAGGCGATATGCTGCTGGCCGGTACGGCATTCGGCAAAAT

# Appendix A

CCGCGCGATGGTCGATGAAAACGGCAAATCCATTACCGAAGCCGGTCCGTCCATCCCCGT
CGAAATCCTCGGCTTGTCCGACGTACCGAATGCGGGTGAAGACGCGATGGTATTGGCGGA
CGAGAAAAAAGCGCGCGAAATCGCCCTCTTCCGCCAAGGCAAATACCGCGACGTGCGCCT
TGCCAAACAGCAGGCGGCGAAGCTGGAAAATATGTTCAACAATATGGGCGAAACCCAGGC
CCAATCTTTGTCGGTCATCATCAAGGCAGACGTGCAGGGCTCTTACGAGGCTTTGGCGGG
CAGCCTGAAAAAACTGTCCACAGACGAAGTGAAAGTGAACGTGTTGCACAGCGGCGTGGG
CGGCATTACCGAATCGGATGTCAACCTTGCCATCGCTTCGGGCGCATTCATTATCGGCTT
TAACGTGCGTGCAGATGCCTCTTCGCGCAAACTTGCCGAAAATGAAAACGTGGAAATCCG
CTACTACAACATCATCTACGATGCCATCAACGACGTGAAGGCGGCGATGAGCGGTATGCT
TTCCCCGGAAGAGAAAGAACAGGTTACCGGTACGGTCGAAATCCGTCAGGTCATCTCCGT
TTCCAAAGTCGGCAACATTGCAGGCTGTATGGTTACCGACGGCGTGGTCAAACGCGATTC
CCATGTCCGCCTCATCCGCAACAACGTGGTTATCCACACGGGCGAACTGGCTTCGTTGAA
ACGCTATAAAGACGATGTAAAAGAAGTCCGCATGGGCTTCGAGTGCGGTCTGATGCTCAA
AGGCTACAACGAAATCATGGAAGGCGACCAACTGGAATGCTTCGACATCGTCGAAGTTGC
CCGCAGCCTGTAATTCCTTTGCAAATAAAATGCCGTCTGAAGCGTTCAGACGGCATACGA
AACGGGTTCTGTATCATACAGAACCCGTTTTTTGTCGCAAATCGGCTTCAGACAGCCCTC
TTGCCTTATCCCGATTTGAATCTGACTTGCCATACAAACAGGCTTCAGACGGCATTATTT
GCCCGCTAAACGTATCCCAAGCTTCTCCGCATATTCCCTGCGTTCGGCGCGGCTGGTTTC
CGGGCGGTGCGTATTGAGCGACGACCATTTCCAATGACTGCGGGCTTTGTTGAGTTCGGG
CGGGAGTCTGGCGGCATCCCACGGGACTTTGCGGCTGTGCAGCTCGATATCCGACTGTGC
CGCGTGTCCGCGCGTTTGCAGGACGTGGAGCAAATCGAGGGCGCGGGCGGCGAGCAGGGT
CAGGGTTTCAGGGTCGGTGTGCAGGGTTTGGCGGCCAGCGAGTTTGTCGGAAATGGTGCG
GGTATTGGGCAGGATGCCGCCCAAAAAGCCGCCGATTGCCGTACCCAATCCGAGCGAGCC
GCCGAGTGTGGCGATGTCCAGCCCCAAGCCGATGAGCGCGCCGGTTGCCGCGCCCGTGCC
GGTGCGGATGCCGTATTGTTTGAGCAATTCGCTGTCGAACGGGTCTTGGCGGAAGGCTTG
CGGCATCCAGTCGCCGCCGTCGATTTCGCTGTGGTAGAAACGGTAGAGGGCAAACAGCCG
CTGCTGCATCTGCCGTTCGAGTTGGCGTATTTCCGCCTGCATGGTTTGCAGCACGGTGGC
GGTATCCTCGTTTTCGTCCACTTCCTGCCTGAAGGCGGCGGCATCAATTAAAAAGTCGGC
GATTTCGCGGCGCGCTTCGCCGTCCAGCCGCTGCCATTCGCGCCGGCGCATGGCTGTCAG
GCGGTCAAGTGTGCTGCGTTCGGGCAACATGGTGGCGAGGTTTTCCCACAGGCGCAGTTC
GCCTTCAAAATCAAAGGCGACGGTGTCGAACCCTGCGAAAACGTGCAGGTTTCTCCTCGC
CAGCATGGTTGTCCACGATTCGGGAAGCTGTCCGCCGGTAAAGTTGAACACGGGCATAAC
CGGTTTGGCACACCATGAAAGGATGGTCAACTCGTCCCTGTATTTGTCGAGGACGGGTTC
GCGCGCGTCGATGACGTACATTGCCATATCGCTTTGCAAGACTTGCCGTAAGACTTTGGC
TTCCTGATTGAAATCATGGTGCGCACCGTGGCTGCCGAGAAACTGTTGCAGCCGTTCGAT
GCCGTCTGAACGATTGTCCGTATGGTTTTCCAGCCATTCCAGCACGCCGCCCGCGTCTTC
GAGTCCGGGCGTGTCGTACAGGAAAACCAGCGTGTCTGCGCCGTCGCTGATGGCGGCTTC
TTCGACATGACGCGTGGTCGATGGGGCGTTTTTGACTTCGCCGAAACCGCTGTCGCGCAA
AAGGGTACGCAGGAGCGAGGTTTTGCCGGTGTTGGTGTGTCCGACGACGGCGAGGGAAAG
GGGTTGTTTGTTCATGATGTTTTTGAAGAATGGATTTTCAGACGGTCTTTTTTCAGAATG
GCGGCTTAACAGAACATTTCAAGTGAGTTTATTGGTCTTTCAAACGCCCTTCCTGCGCCG
CCCTGTCAGGCTCAAGCCACGCCGCGCCGCATTCGGCCAGCGCGTTACGCCAATGTTCCA
GCTTTTCCGAAAGGTCGTCTGAAAGCCCCTGTTCCGCCAAAAGCTGCACCACCGCGCCGC
CCTGCGCCGCTTCCGAGAGTCGGACAATCTGCCGCAACACGCCGCGGTCCGGCACAGTTT
GGGCGCGCACGCCGATAAGCAGTTGCGCCGGTTTCTGCTTCAGCTCTGTCTCCAGCGCGG
CAACCTGTTCCCGATTGGTGGCAACGCCCTTATCCAGCCATTCCTGCGCCAGCCTGCCCT
CGAACCATTCGCCGTCCTGCCACTCGGTCTCCAGCATGACCGCCCATTTCGGCGCATCGT
TCAAGATGATTTTCGGTGAAACGGCGGACACGGTTTCCCGACGCGTATCCGCATCGGTGA
TTTTGTTCTGCCAGCGGCGGATGACCGCCTGATAATAGGGCTTTTCCAAATCCAATCCGT
TTTCGCTTGTTTTCAAAAGGATTTTACACACTACCCAAGCCAGCAGGCGGCGGCAGGATGC
CGTAGCAGGCGATACTGCCGACCAGCAGCCCCGACCAAGCCCGCGCATCGGCAATATTGC
CGTTCAGACGGCCTTCGATGACCGCCCGCGCATCGGGGACAGGGAAACCGAGTTTCGACG
GCAGCCATGCCAACATTTCCACCGCGCGTACCGAAGCGGCATTGCTCAACAGCGTGCTTT
CCCAGTTGAACGTATATTGCCGCACCAAAAGCAGCAACAATACCGACACCAGCATTCCGA
GCAGCGTGCAGAGCCACAGGCTGTGCGACGTTGCGCCTATTTTCCAACGTACCGAAGGTT
GCCGCCACTCGTCCGCATACAGCCGCAACACCGCCTGATTTACAGGGTCTTTGCCCCGAA
ACCACGTCGCCGGACTGCTGAAAAAACGCCCCACTTTCACACGCAGGAACAACATTGCCA
ACCATACTGCCAGCATCAGCGTATTCATGCCCAACACGCCCGCCAAAACCAAAAAGAAAT
TCAGACCCTGATTGTCCATTAGAAGATAAGTGACTGAAAAACCGGTAAAAAATGCAAACG
TCGCCGCCACCACCCACAACCAGAACGACCCCGCACGCACACGTTCCAACGTCTCCCGCA
GCATACGGTTCCTGTCAATCATCTCCGCCCGACGGATGATTTTTCCTCCGTACTGCCGT
CCACGCGGCGCAAAGCCTCCGTCGCCTGTACGGGATCGCCGCTGAAAATAAAACCGCCTT
CGTCCAAAATACGGACCAGCTCAACCAGTTTTCGGGATGGATTCAACATAAAATGCCGTC
TGAAAATAAAAAACAGATTTTAACACACGCATTTTCAAGAATATTCACAGTGTAGGCAAA
GAGTAAATCTCACACAGAAGCAAAAGTATCGGCGTAAACTGACTGCCTCTACTTTCCCGA
AAGATTGTGCGATGTATACAGGCGAACGCTTCAATACTTACAGCCATTTGAGCGGTTTGA
TTCTGGCGGCGGCAGGTTTGGCGCTGATGCTGCTGAAAACCATAGGACACGGGGACGGCT
ACCGTATCTTCAGCGTATCGGTTTACGGCATCAGCCTTCTTCTGCTCTATTTGAGTTCCT
CGCTGTACCACGGAATTGCAGCCGGAAAACTGAAAAGCATTTTGAAAAAAACCGACCACT
GCATGATTTATGTGCTGATTGCCGGAAGCTACACACCGTTTGCACTGGTTTCTTTGAGAA
ACGGGCCGGGCTGGACGGTATTTTCACTGTCCTGGCTGCTGGCGGCTGCAGGAATCGCAC
AAGAACTCACCATCGGACGGAAAAGCGAAAAACGTCTGCTGTCTATTGTGATTTATGTCG
TCATGGGTTGGATGGTCTTGGCGGTAATGAAATCCCTGACAGCCTCACTCCCGTCGGCAG
GACTGGCTTGGCTGGCGGCAGGCGGTATGCTGTACAGTGTCGGCATTTACTGGTTTGTAA
ACGATGAAAAAATCCGACACGGGCACGGAATCTGGCATCTGTTCGTATTGGGCGGCAGCA

## Appendix A

TCACCCAATTTGTCAGCGTGTACGGTTACGTAATCTGAATGCCGTCTGAAAAGCAAAACC
TCCCGTTCCTGAAGATTGGGAGGTTTTCTGTTTGCCGGACATCAGCCCTTGTCGTGGAAC
TCGTGGAATTCATACTGATAGGACAAATCCCGACCCGCTTTTTTCTGTGCCAAATAATCA
TCATAAATGGCGCGGATTTCCTTACGCAACAAAAACAGGGCTATCAGGTTGGGGATAACC
ATAAAACCGTTGAACATATCCGACAGACTCCAAACCAAATCGACTTTGCCGAGCGTACCC
AAAACAATGGCAAGCAGAACCAATGCGCGATAGATGCCCAAGTGTCTTCCCCTGAAAAGA
AAACGGATATTGGACTCGCCGAAATAATACCAACCGATGATGGTGGTGAAGGCAAAGAAG
GTCAGACACACGGCAAGCAATTGCGAACCGAAGCCCGGAAATGCCTTGTTAAAGGCAAAT
TGAGTAACCGCCGCGCCCTGTTCGCCCGAAAGGTTGGCATCGGTCAGCAGGATAATCAAT
GCCGTAGCCGTACATACCAAAATCGTATCGATAAACACACCGACAAATGCCGCCATACCT
TGCTGCACAGGGTGCTTCACATCCGCAGTCGCGTGGGCGTGCGGAGTCGAACCCATACCT
GCTTCGTTGGAAAACAGACCGCGCGCCACGCCGAAACGTATCGCTTCGCGCATACCGATA
CCCGCAGCACCGCCCAAAACGGCTTCGGGATTGAAGGCGGCGGTAAAGATGTGGTTGAAC
ATCGGCACAATATGGTCGGAAAATTCAAACAGGATAACGACGGCGCACAAAATATAAACA
ACCGCCATAAACGGCACGACAAATTGGGCGATATTGGCAATACGGTTCACGCCGCCAATC
ACAACCATGCCCGCAAGGACGGCAAGCACAATACCGACTGCCAAAGAAGGCACATCAAAT
GCAATGGTAACGGCAGAAGCAATGGAGTTTGCCTGTGTCGCATTACCGATAAAGCCCAAT
GCGATAATCAACGCAATGGAAAAGAAACCGGACAAAAAACGCGCCGCGCCCCTGCCGATT
TTCGGAGTCAGACCGTGGGTGATGTAGAACGCCGGCCCGCCGATGTATTTGCCGTGGCTG
ACGACGCGGTATTTCTGCGCCAGCAGTGCCTCCGCAAAAATCGTGGACATCCCCAAAACG
GCAGAAACCCACATCCAAAAAATCGCGCCCGGCCCGCCTGCGGTGATGGCGGTCGCCACG
CCGGCAACGTTGCCCGTACCGATTTGCGCAGATATGGCAACCGCCAACGCCTGAAACTGC
GATAAAGACTTGTCGTCTTTATCGCCTTTGGCAAACAAGCCGCCGAATACGGATTTGAAT
CCCGCGCCCAGCTTGGTAATCTGCGGCGCACCAAGATACAGCGTAAAAAACAGGCCGATA
CCCAAAAGCGCGTAAATCAGCAGGTAGTCCCAAAGGAACCGATTGACTGTACCCACCAGA
ACAGACAATATATTTTCCATAAAATAAACCTTATCTTACAATTAAAATGACTGCCTTCCA
AAAGACATTCCAATAAGGAAACACGGCGAGCAGACCGTATTTGCCGCAACAGATGCCTTA
AATTGTCAACAATCGGGGAGAAGCTGCGCCATCATACCGTAAAATATCGTTAATTAAAAC
ATTTCTTTATTTTTAAGCGGAAAGCGGAGGAAATCGCTTTCAGACAGCATAGACAACGGC
ACGGCATAAAACAGGATATTTTGGGTACTTGCAACTTATGTTAAAATGCCGACCGTAAAA
AATCTGACAAAAACAGATTAATTATTTGAAATAAGAAAGGAAATTTATGGCAGGCCATAG
CAAGTGGGCAAATATCCAGCATAAAAAAGCCCGTCAGGATGCCAAACGCGGCAAAATCTT
TACCCGTTTAATCAAAGAAATCACCGTTGCGGCGCGTATGGGCGGCGGCGACCCCGGTTC
AAATCCGCGCCTGCGCCTGGCTTTGGAAAAAGCAGCCGAAAACAATATGCCCAAAGACAA
TGTGCAACGCGCCATCGACAAAGGCACGGGCAACTTGGAAGGCGTGGAATACATCGAGTT
GCGCTACGAAGGCTACGGCATCGGCGGCGCGGCTTTGATGGTGGACTGCCTGACCGACAA
CAAAACCCGCACCGTTGCGGACGTACGCCACGCGTTTACCAAAAACGGCGGCAACTTGGG
TACCGACGGCTGCGTGGCGTTCAACTTCGTGCATCAGGGCTATTTGGTATTCGAACCCGG
CGTTGACGAAGACGCGCTGATGGAAGCGGCTTTGGAAGCCGGTGCGGAAGACGTGGTTAC
CAACGACGACGGTTCCATCGAAGTCATTACCGCGCCAAACGATTGGGCGGGCGTAAAATC
CGCTTTGGAGGCGGCAGGTTACAAATCCGTTGACGGCGACGTTACGATGCGCGCCCAAAA
CGAAACCGAACTCTCCGGCGACGATGCCGTCAAAATGCAAAAACTGATTGACGCGCTGGA
AGACTTGGACGACGTGCAAGACGTTTACACTTCCGCCGTATTGAATCTGGACTGATACGC
AGCACAGCAGACATACAATAAAATGCCGTCTGAACCTTTCAGACGGCATTGATTTATTTA
GCCCTTGCCCACGCCCACCAAACCGTCAGGACAACCGCCAGAAAATACGCCACGCTCCAA
ACAGGCAAAGCAATTCCCAGCAGATAATCCGGTTCAGCACAATTTCCGAACCCGCGCACG
ACAGGCTCGAACCAATCAAACAAAGGCCAGCCTTTCAAGCGGAACGTCCACGGCGCGCCG
CACGAAGGAGCGGTTCCCGGCGGCAGCGACTGCAACCACAACTGATATGCCGCAACAGAA
ATACCCGTAACGGCCGGAATGCTGATAAAGACAGCACCGAACAAACCGCCTGCCCTTCTT
CTTGGTCTGCACATCAGGACAATTGCCGTACACAATGCGGTTGCCAAAACGCATAACCGC
TGACTGATACACAAAACGCAAGGCTCCATACCCAAAACATACTGTGCCGCCAAAGAACCG
GCAAATGCACAGACCGAAACGGCAAACAGCAGCCAAACGGCTTTTCTAAATAACGGGGTC
ATTTTCTCAACACACCAATCAAAATACCGATATGCCGATTTTGCTGGATATATCCCGAGA
TGGCAAGGGACAAAACGGCGATTTGCCCGCACAATGCCCACAAAAAAATACATCCGGAGG
ATTTGAATTTCAGCAAATTTAGCGAATCAAAAGTTTATTTCAATGAAATCATATGATTTT
TTTGAATAAGCGGATTGATGGGTTTTTGAAGGAATTTGTTACCGGATAGCCATCGGGCAA
GTTTTTTGCAAAATTTGAATCGGTCGGGTAAATTTTCAAAAAATAATTGACAGCGGATAA
GAAACGGCGGATAATTCCCGCCGTCGAGTTGCTTGATGCAGCTTGATTTTTCTCCTCTAT
TTCTCCTTTGTAGACTTGGCACACATTCAACTGGATGTGTGCATTTTTTTATCTCCGCTT
TTTTTGAAATTTAATTACTTTTATTGTTTGAAATTCCATTCTTTAAGAAATTTAACAAGA
AAAACGCTTGCTTTTTGACCGGAAAAGCTGCATAATTCATTCCGTTAGCTGGTTCGAGTA
GTCAGTTAATAGTTTCTCCTCTATTTCTCCTTTGTAGACTTGGCACACATTCAACTGGAT
GTGTGCATTTTTTTATCTGAAGCAACAAGCCTCTGTGCGTGATGTTGTTATGTTTCATTT
AGGTGTCAAACCGCATATCCGGTCTGAAATATTCAATCCAAATCCAAAACCGGATTTTCT
TTGACCTCCTCCATCACAACATAACTCCTACTCTCCGAAGCGGCAGGCAGTTGCAATAGG
ATATTGCCTAGCATATCCCGATAGGCAGACATATCGGGCAAACGTACTTTAATCAGATAG
TCGTATTCGCCCGACACCAAGTGGCATTCCATAATTTGCGGAATTTTCAGCACTTCTTTT
TTGAAATCTTCGAAAATATTGCCCGATTTGGATTGCAGCTTCAGCTCGACAAAAACCAAT
AAAGGTTTGCCCAACAGATGGGGATTGAGATGGGCGTGATAACCGGAAATATAATGTTCC
CGCTCCAAACGGCGCACCCTCTCTGTAACGGGCGTGGTGGACAAGCCTACCTTCTCGGCA
AGCTCCGTCATCGGGATGCGGGCATTCTGTTGAAGGATCTTAAGGATGCGGAAATCGATT
TTATCTAGTTCTTTCATTTAGATTGCCTTGTATTTATTATTGATTTTAACAAATAGAGTA
TATAGTGGATTAACAAAAACCAGTACGGCGTTGCCTCGCCTTGCCGTACTATTTGTACTG
TCTGCGGCTTCGTCGCCTTGTCCTGATTTTTGTTAATCCACTATATATTTGAGAAAGCGA
TTATATCAGGAAAAGCAAACCGCCTTCCTACCTGAAAACTGCTGCTTCGGCTTGAAGACA

## Appendix A

CAAGGTTCTTTAATATTTTAAAAGCCTTGCCGTTGGATTATAATCCCCCAACCGATTTCT
TAATTTTGCTAATAAACACTTGCTTGGTAAGGAATGAATTTATGCGCCCTTTGAACGTGC
AGATCAGGTTGGGCAACCTTAGGCACAATTATCGGATTTTGAAGGAAATGCACGGAGGCA
AACTGTTGGCGGTAGTGAAGGCCGACGCATACGGACACGGTGCGGTCAGATGTGCTTTCG
CGCTGGCAGACTTGGCAGACGGCTTTGCCGTGGCGACAATCGATGAAGGAATCAGGCTGC
GGGAGAGCGGCATTACCCATCCGATTGTCCTTTTGGAAGGCGTATTTGAAGCATCGGAAT
ACGAAGCGGTCGAACAATACTCGCTTTGGCCGGCAGTCGGAAACCAATGGCAGCTTGAGG
CTTTGCTGATCCGCCATTGGAAAAAAACCGTCAAAGTCTGGTTGAAAATGGATTCGGGGA
TGCACCGTACCGGTTTTTTCCCTCATGATTACGCTTCGGCATATGCGGCATTGAAGCAGT
CGGAATATGTGGACAGTATTGTCAAATTCTCGCATTTCTCCTGTGCGGACGAACCCGAAA
GCGGTATGACGGAAATACAGATGGAAGCATTCGATTTGGGTACGGAAGGGCTGGAAGGCG
AAGAAAGCCTTGCCAACTCCGCCGCTATTTTGAATGTTCCCGAAGCACGCAGGGACTGGG
GGCGCGCCGGTCTGGCGTTATACGGCATTTCCCCGTTCGGAGGAGGCGATGACAGGCTGA
AGCCCGTGATGAGGCTTTCAACCCGTATTTTCGGCGAACGCGTTTTACAGCCGCACTCCC
CTATCGGTTATGGCGCAACATTTTATACCAGCAAATCTACGCGCGTCGGCCTGATTGCCT
GCGGTTATGCGGACGGTTATCCGCGCCGCGCCCCAAGCAATTCCCCCGTCGCTGTCGACG
GCAAATTGACCCGGGTCATCGGCAGGGTCTCTATGGATATGATGACCATCGAGCTGGATG
CTTCGCAAGAAGGTTTGGGACACGAGGTCGAACTGTGGGGCGATACGGTCAACATCAATA
CCGTTGCCGAAGCGGCCGGAACCATCCCTTACGAATTGATGTGCAATATCAAACGTGCAA
AATTCACTTATATCGAGTAATCAAGTCCAAACGAAAATGCCGTCTGAAGCCTTTCAGACG
GCATTTCCCCATCAAAACCGCAATCAGTTTTTCATCGATTGAACCGGAGCCGGAATTCTG
CCGCCTCGGTTGACGAATACTTCGCACGAACCTTCTTTGACCGGCATCACAGGCGCGTAG
CCCAACAAGCCGCCGAACTCGACGCTGTCGCCGACGGTTTTACCGGTTACCGGAATAATG
CGCACGGCAGTGGTTTTGCTGTTGATCATGCCGATGGCGGCTTCGTCGGCAATGATGCCG
GAAATGGTGTGCGCGGGCGTGTCGCCGGGAACGGCAATCATATCCAAGCCGACCGAACAA
ACGGCGGTCATGGCTTCGAGTTTGTCCAGCGTCAGCACGCCTGCTTCGGCGGCGGCAATC
ATACCTTCGTCTTCGGAAACGGGGATAAACGCGCCACTCAAACCCCCGACCGCGCTGGAA
GCCATCATGCCGCCTTTTTTCACGGCATCGTTCAGCAATGCCAAAGCTGCTGTTGTGCCG
TGCGTACCGCAGACGCTCAAGCCCATTTCTTCAAGAATGCGTGCCACTGAGTCGCCGACG
GCGGGGGTCGGCGCCAGCGACAAGTCGAGAATACCAAACGGGATATTCAGCATTTTTGAG
GCTTCGCGGCCGATGAGTTCGCCCACGCGGGTAATTTTGAAAGCAGTTTTCTTCACTACT
TCCGCAACTTCGGTCAATGTCGTTGCATCTGAATTTTCCAACGCGGCTTTTACGACACCT
GGGCCGGATACGCCGACATTGATAACGGCATCCGCTTCGCCCGAACCATGAAACGCGCCC
GCCATAAACGGGTTGTCTTCCACCGCGTTGCAGAACACGACAATTTTAGCGCAGCCGAAA
CCTTCGGGCGTGATTTCCGCCGTGCGTTTGACGGTTTCGCCCGCCAGCTTGACCGCATCC
ATATTGATACCGGCACGCGTACTGCCGATATTGATGGAGCTGCACACAATATCGGTAGTC
TTCATCGCTTCGGGAATGGAGCGGATTAACACCTCATCCGAAGGCGACATCCCTTTTTGC
ACCAACGCGGAAAAACCGCCGATAAAAGACACACCGATGGCTTTGGCAGCTTTATCCAAA
GTTTGCGCCACGCTGACGTAAGAATCAGCATGGGTGGCCGCCGCGATTTGGGCAATCGGC
GTAACGGAAATGCGCTGATTCACAATCGGTACGCCGTATTTGGCAGACAGATATTTTGCC
GTAGTGACCAAGTCTTTGCCGACTGTGGTAATTTTATTGTAAATATTTTGGTTCAACACA
TTGATATCGCTGCTGATGCAGTCGTGCAAATCAATGCCGATGGTAATGGTGCGGACATCA
AAATTCTGGTCGGCAACCATTTTGACGGTTTCTAAAATTTCGCCGGATTGGATACTCATC
ACATTCCTCCAACTCAAATGCGGTGCATCGCTTGGAAGATTTCTTCGTTTTGCATACGGA
TATCAAGCGCGAGTTTTTTGCTCTCTTCCGCAAACAAATCCAAAACCTCTTGACGCGATT
TGCTGCATTTTGAAGTGTCCACCAAGATAATCATAGTAAAAAAATCGTCCATCAGCTGTT
GGCTGATGTTGAGAATATTGATTTGGTTTTCCGCCAAAATTTTGGAAACATCGTACACGA
TGCCGACGCGGTCTTTACCGATGACGGTGATGACTGAATTGTTCACAGGCTTACTCCTTG
CAGATATCCGTTAAAGTCCGAAATTATACCACCGTTGGATTTTGAAGAAATATTGTCAAC
~~AATATATACATACAAAATGCCGTCTGAAACTATTTGAGAGAGGATCAAGATTCAGGCTTC~~
GATTAAATAACCATCCTTATCCCACTGGGTTTTCCTGACCAACTTGTCATCCTGATAAAC
AGCTTCGCTCTTTTTAGAACCATCTTCATACCACTCCAAAACCACCCCGTTGCGTTGATG
GTGGCGGATAGACAGTTCCGAGAGTAATCGGCCGCTTTCATCCCAAGTCAGAATTTTGGC
AGGCTCATCGTTGACCATAACCATTTCCGTCTTGATACTGCCGTCGGCATACCATTGCTT
CCATACGCCGTTTGCCTTATTTTGCTTAAACTGGATTTCGCTTTCCTTGCCGCCGTTACG
GTAATAGCGGTATCCCGTACCCTCACTCAAGCCATTTTTATAAGGCATAACGGCAGATTT
TTTACCGTTCGGATACCAGTTGACCCACTCCCCGTCCGGCTTACCCTTGCTGAAGCCCCC
CGCCATTTTTTTCTGACCATTAAAATGCCACAAAATCAACATACCGTTTTGCAGGGTAGG
CACAAAAGATTTGATTTGCGTTGAAGCAACGATATAAGGTTCAGAATATTTCTTCATCGA
CGGATAATAAAAATCCTGCGCGTGCGCAATACCCGCCACCACACTATATTGCCTGATATA
AGCGGCAGAAGACATCGTCGCCGTCAGCTTTCCGTTCTGATTAAAATAAACAGAATAGGT
CTGCGCCGGCAAAGCGGCCGAAAAACCCAACAGGACAGTTGAAAATACAATCCGAGATAA
TTTTTTCATTGCAATAGCGATATAAAAACAAGGCTGTGTTTTAGTAATCTGTTGATTTCA
ATTATTTGCAAGGGAAAAGACAATTATTTTCCGGTTAGGAATAAACCTATTCTATTGAAT
ATATTGAAGCCAAGTACGCCTATCAACACTATATTAAAACACTGCCAAAAACAATTAACT
TATAAACAATATGGTAAGGATTTCTCTGCCAAGCATCAAACCCGAGACAACGTATCGTAA
AAATGCCGTCTGAAAACAAATCGTCTTCAGACGGCATTTCCCCTTCAACTCACTCTTCAC
CCAATAACTGCTCGCGCGTCAAGAGGAAAACAAAACCGTCGCCCCCGCTGGTTTCCAACC
AAGTAAAAGGCAACTCCGGATACGCTGCTTCCAATACATCCCTGTTATGCCCGATTTCCA
CCAGCAATACACCTTTGGGATTCAGAAACTTTGCCGCATTCAGAAGAATCTGCCTGGTGG
CATCCAACCCGTCCGCCCCGCTGCCCAATGCCAATTCCGGTTCGTGCAAATACTCTTCAG
GCAATAACTCAACCGATTCCGCATCCACATAAGGAGGATTGGAAACAATCAAATCATAAG
TGCCTTCCAATCCTTCAAACAAATCCGTATGAATAAGCCGGATGCGTTCTTCCAAACCAT
AATCTTCGACATTAATCCCTGCCACTTCCAAAGCATCCAAGCTCACATCAACCGCATCAA
TTTGGGCATCAGGATAATGATGCGCCATCTGAATGGCAAGGCAACCGCTTCCGGTGCAAA

## Appendix A

GATCCAAAGCATTATGCACCAACTCATCGTATTCTATCCAAGGACGAAGTCCGTCACCCA
ACAATTCATAAATAAAAGAACGAGGTATGATTACGCGCTCATCCACATAGAAATCAAACT
CTCCCTGCCATGCCTGGTGTGTCAAATAAGCGGCTGGAATGTGTTCGACAGCACGACGCT
CAATAACCGCCAGCACTTCCTCTTTTTCAGCTTCCAAGAGTTTTGCATCAAGATATGGGG
CAAGCATATCCAAAGGCAAATTCAAAGTATGCAGAATCAAATAAGCTGCTTCATCATGCG
CATTATCTGTTCCATGACCAAAAAAGAGCCCTGCCTCATTAAAACGGCTGACTGCAAAAC
GTAAAATATCGCGGATAGTCGTCAATTCTTGTGCTGCCTGATTAAACATAATATGAACCA
TTCTGCGTATAGATACTTTTAATTATAACAGAAACAACAAGCAAACCTTTTCATATCGCC
AAATAACCACCCAATCTACCCATACAACTACATAAATGCCCGCGCGAAAACCATCGCCCG
AACGGAAACGACAATGGCCGACGGTATGGGCAATCTGATTGGCTGGGAAAAAACGGGGCT
TGTTGTCGGTAAGCAGTGGATAACCGCAAAAGACGACAAGGTGTCCGATGTCTGCAATGC
CAACGGCGAGATGGGCGTAATCGGGCTTTACGAGCCTTTCTCACACGGCGCATTGACGAT
ACCCGGTCATCCGAACTGCCGATGCGAGGTTGTTTCCGTATCGGGTGGCGAATTGGGGGA
ATTTGCCGAAAAAAAGGAGCTTCGTAAAGCGGCTATGCAGTATGCGCGGGATAACTTTAT
CGGCAAAAGCTATGTCAATAAAAACAGCGGGCATGAACTGAAGGTAACTTGGCAAGGTGT
GAAACACGCTGCGTCAAAGGCAAATCAGGCGGAATTATCCATCATGACAAAACTTGATGA
CTTATTGCGCTACGCAAAATATGAGGGTTCTTATTCGGATAGGAAAGGTCATCCTAATAT
TATTGCAGCACATAAGTATCGTGCCGTTGCCAAGGTTGGGAATGAGTCTTTAAATATCGG
TGTGATTGTAAGGGAATTTCCAGACGACCATAAACATTACGACCATTTCATCTTGAAGGA
TGAATAAAGCCCTTTTGCAGTGTCGTTCTGGAGCGGATAGCGTTAAGGCAAGTACACTTC
CAGCCTTGAAAAAGGGCTTTAAATTCAGCATGCCATTTATACAGGCAGGAGTAAACCCAT
GACAAAGTTATACGCAGAAATCGCCAAGATGGAGACGCAGGACGACGACACGGTCAAGGT
TTGGGGTTACGCTTCAAGCGAGGAAATCGATTCGGACGGCGAAGTCATCGCGGCGGCAGC
TATGAAGGCGGCGATTCCCGATTATATGAAGTTTGGCGCGGGGCGCGAGATGCACGGCTC
AAACGCTGCGGGAACGGCAATTGAAATCAACGTGGAAGATGACGGCAGAACCTTTTTCGT
GGCGCATATCGTCGATCCCGTTGCCGTGACGAAGGTCAAAACAGGCGTTTACAAGGGCTT
TTCCATCGGCGGCAGCGTTACCGCCCACGATGAGTTGAACAAGTCGCAAATCACGGGTTT
GAAGCTGACGGAAATCAGCTTGGTTGACCGACCCGCCAATCCCGATGCGGTGTCTACCTG
CTTTAAGGCGGACAAAGGTGCGGAAGCGGTAAACAACGATACAGAACATAATGCTACATA
TTTTAGCCATTTCCCTTCCAAACAAAAAGCACCGACGGCGGCCGATGCCCTTTCCTTTA
CAGGTTCCCCTATTTTTTATCCGCGGGCAGCACCGGTTTGGCTGGGGCTTTTGGTGCGGG
CGCGCCGACCGAAGCCTGGTCCTTCAGCTTCGCCAGCACCGCAGGGCCGATGCCCTTTAC
CTTGGTCAAATCGTCTACAGACTTGAACGCACCGTTTTGCGCACGGTATTCCGCAATGGC
CTTCGCCTTCGCCGGGCCTATGCCCGGCAGCGCCTCCAACTCCTGCTGCGAAGCCGCATT
GATGTTTACCGCCGCAAGGGAGAAGGCGCAGGAGAACAGCATACAGAACAGCACGAACAT
TTTCTTCATGGTTTTTCCTTTAAGGGTTGCAAACAATAAACCGCATCTTGCGACGATAAA
ACGAGTCATTCTAAAATGAATATCCCAAAGTTTCAAGCCGTTCCTCCGCAAACCCGACCG
GACACCGTACGGATGCCGTCCCGCCATCACCGACATTTTTTCCGGGCAAAGCAAACATTT
TTTCCGGGCAAAGCAAAAACCCCCGAATAATCGGGGGTTTTCTGAATGGGTGTTTGGCAG
TGACCTACTTTCGCATGGAAGAACCACACTATCATCGGCGCTGAGTCGTTTCACGGTCCT
GTTCGGGATGGGAAGGCGTGGGACCAACTCGCTATGGCCGCCAAACTTAAACTGTTACAA
ATCGGTAAAGCCTTAATCAATATATTCGGTAATGACTGAATCAGTCAGTAAGCTTTTATC
TCTTGAAGTTCTTCAAATGATAGAGTCAAGCCTCACGAGCAATTAGTATGGGTTAGCTTC
ACGCGTTACCGCGCTTCCACACCCCACCTATCAACGTCCTGGTCTCGAACGACTCTTTAG
TGCGGTTAAACCGCAAGGGAAGTCTCATCTTCAGGCGAGTTTCGCGCTTAGATGCTTTCA
GCGCTTATCTCTTCCGAACTTAGCTACCCGGCTATGCAACTGGCGTTACAACCGGTACAC
CAGAGGTTCGTCCACTCCGGTCCTCTCGTACTAGGAGCAGCCCCCGTCAAACTTCCAACG
CCCACTGCAGATAGGGACCAAACTGTCTCACGACGTTTTAAACCCAGCTCACGTACCACT
TTAAATGGCGAACAGCCATACCCTTGGGACCGACTACAGCCCCAGGATGTGATGAGCCGA
CATCGAGGTGCCAAACTCCGCCGTCGATATGAACTCTTGGGCGGAATCAGCCTGTTATCC
CCGGAGTACCTTTTATCCGTTGAGCGATGGCCCTTCCATACAGAACCACCGGATCACTAT
GTCCTGCTTTCGCACCTGCTCGACTTGTCGGTCTCGCAGTTAAGCTACCTTTTGCCATTG
CACTATCAGTCCGATTTCCGACCGGACCTAGGTAACCTTCGAACTCCTCCGTTACGCTTT
GGGAGGAGACCGCCCCAGTCAAACTGCCTACCATGCACGGTCCCCGACCCGGATGACGGG
TCTGGGTTAGAACCTCAAAGACACCAGGGTGGTATTTCAAGGACGGCTCCACAGAGACTG
GCGTCTCTGCTTCTAAGCCTCCCACCTATCCTACACAAGTGACTTCAAAGTCCAATGCAA
AGCTACAGTAAAGGTTCACGGGGTCTTTCCGTCTAGCAGCGGGTAGATTGCATCTTCACA
ACCACTTCAACTTCGCTGAGTCTCAGGAGGAGACAGTGTGGCCATCGTTACGCCATTCGT
GCGGGTCGGAACTTACCCGACAAGGAATTTCGCTACCTTAGGACCGTTATAGTTACGGCC
GCCGTTTACTGGGGCTTCGATCCGATGCTCTCACATCTTCAATTAACCTTCCAGCACCGG
GCAGGCGTCACACCCTATACGTCCACTTTCGTGTTAGCAGAGTGCTGTGTTTTTAATAAA
CAGTCGCAGCCACCTATTCTCTGCGACCCTCCGGGGCTTACGGAGCAAGTCCTTAACCTT
AGAGGGCATACCTTCTCCCGAAGTTACGGTATCAATTTGCCGAGTTCCTTCTCCTGAGTT
CTCTCAAGCGCCTTAGAATTCTCATCCTGCCCACCTGTGTCGGTTTGCGGTACGGTTCGA
TTCAAACTGAAGCTTAGTGGCTTTTCCTGGAAGCGTGGTATCGGTTGCTTCGTGTCCGTA
GACACTCGTCGTCACTTCTCGGTGTTAAGAAGACCCGGATTTGCCTAAGTCTTCCACCTA
CCGGCTTAAACAAGCTATTCCAACAGCTTGCCAACCTAACCTTCTCCGTCCCCACATCGC
ATTTGAATCAAGTACAGGAATATTAACCTGTTTCCCATCGACTACGCATTTCTGCCTCGC
CTTAGGGGCCGACTCACCCTACGCCGATGAACGTTGCGCAGGAAACCTTGGGCTTTCGGC
GAGCGGGCTTTTCACCCGCTTTATCGCTACTCATGTCAACATTCGCACTTCTGATACCTC
CAGCACACTTTACAATGCACCTTCATCAGCCTACAGAACGCTCCCCTACCATGCCGGTAA
ACCGGCATCCGCAGCTTCGGTTATAGATTTGAGCCCCGTTACATCTTCCGCGCAGGACGA
CTCGACCAGTGAGCTATTACGCTTTCTTTAAATGATGGCTGCTTCTAAGCCAACATCCTG
GCTGTCTGGGCCTTCCCACTTCGTTTACCACTTAATCTATCATTTGGGACCTTAGCTGGC
GGTCTGGGTTGTTTCCCTCTTGACAACGGACGTTAGCACCCGCTGTCTGTCTCCCGAGGA

## Appendix A

ACCACTTGATGGTATTCTTAGTTTGCCATGGGTTGGTAAGTTGCAATAACCCCCTAGCCA
TAACAGTGCTTTACCCCCATCAGTGTCTTGCTCGAGGCACTACCTAAATAGTTTTCGGGG
AGAACCAGCTATCTCCGAGTTTGTTTAGCCTTTCACCCCTATCCACAGCTCATCCCCGCA
TTTTGCAACATGCGTGGGTTCGGTCCTCCAGTACCTGTTACGGCACCTTCAACCTGGCCA
TGGATAGATCACTCGGTTTCGGGTCTACACCCAGCAACTCATCGCCCTATTAAGACTCGG
TTTCCCTACGCCTCCCCTATTCGGTTAAGCTCGCTACTGAATGTAAGTCGTTGACCCATT
ATACAAAAGGTACGCAGTCACACCACTAGGGCGCTCCCACTGTTTGTATGCATCAGGTTT
CAGGTTCTGTTTCACTCCCCTCCCGGGGTTCTTTTCGCCTTTCCCTCACGGTACTGGTTC
ACTATCGGTCGATGATGAGTATTTAGCCTTGGAGGATGGTCCCCCCATATTCAGACAGGA
TTTCACGTGCCCCGCCCTACTTTTCGTACGCTTAGTACCGCTGTTGAGATTTCGAATACG
GGACTGTCACCCACTATGGTCAAGCTTCCCAGCTTGTTCTTCTATCTCGACAGTTATTAC
GTACAGGCTCCTCCGCGTTCGCTCGCCACTACTTGCGGAATCTCGGTTGATTTCTTTTCC
TCCGGGTACTTAGATGGTTCAGTTCTCCGGGTTCGCTTCTCTAAGTCTATGTATTCAACT
TAGGATACTGCACAGAATGCAGTGGGTTTCCCCATTCGGACATCGCGGGATCATTGCTTT
ATTGCCAGCTCCCCCGCGCTTTTCGCAGGCTTACACGTCCTTCGTCGCCTATCATCGCCA
AGGCATCCACCTGATGCACTTATTCACTTGACTCTATCATTTCAAGAACTTCTTTGACTT
TGCCTAACATTCCGTTGACTAGAACATCAGACTTGAATTTCCTACTTTGATAAAGCTTAC
TGCTTTGTTGTGTCTTAATCCTGCCTTTTGTGTTTCAGGATTAAGTCGATACAATCATCA
CCCAAATACTGTGTTTGTTTTCTTTTCTCTTGCGAGAGATTTTTATCCTTTGCAAAGAAT
AAAAAATCAAAACAAACGCTTTGTCTTTGTTTGTTGATTTCGGCTTTCCAATTTGTTAAA
GATCGATGCGTTCGATATTGCTATCTACTGTGCAAATCAAAACGAGCTGATTATTATATC
AGCATTTTGTTCTTGGTCAAGTGTGACGTCGCCCTGAATGGATTCTGTTCCATTCTTCCG
TTTTGATTTGTACAGTATTGGTGGAGGCAAACGGGATCGAACCGATGACCCCCTGCTTGC
AAAGCAGGTGCTCTACCAACTGAGCTATGCCCCCGTTCTTGGTGGGTCTGGGAGGACTTG
AACCTCCGACCCCACGCTTATCAAGCGTGTGCTCTAACCAGCTGAGCTACAAACCCGGAT
TCTCTTCTTAAGCGAATCTTGCCTTCACTCAAGCTTCTTCCGCATCTTTTTCAGTTTACC
GATAAGTGTGAATGCCTAAAGCCTCTTCTTTCTCTAGAAAGGAGGTGATCCAGCCGCAGG
TTCCCCTACGGCTACCTTGTTACGACTTCACCCCAGTCATGAAGCATACCGTGGTAAGCG
GACTCCTTGTGGTTATCCTACCTACTTCTGGTATCCCCCACTCCCATGGTGTGACGGGCG
GTGTGTACAAGACCCGGGAACGTATTCACCGCAGTATGCTGACCTGCGATTACTAGCGAT
TCCGACTTCATGCACTCGAGTTGCAGAGTGCAATCCGGACTACGATCGGTTTTGTGAGAT
TGGCTCCGCCTCGCGGCTTGGCTACCCTCTGTACCGACCATTGTATGACGTGTGAAGCCC
TGGTCATAAGGGCCATGAGGACTTGACGTCATCCCCACCTTCCTCCGGCTTGTCACCGGC
AGTCTCATTAGAGTGCCCAACTGAATGATGGCAACTAATGACAAGGGTTGCGCTCGTTGC
GGGACTTAACCCAACATCTCACGACACGAGCTGACGACAGCCATGCAGCACCTGTGTTAC
GGCTCCCGAAGGCACTCCTCCGTCTCCGGAGGATTCCGTACATGTCAAGACCAGGTAAGG
TTCTTCGCGTTGCATCGAATTAATCCACATCATCCACCGCTTGTGCGGGTCCCCGTCAAT
TCCTTTGAGTTTTAATCTTGCGACCGTACTCCCCAGGCGGTCAATTTCACGCGTTAGCTA
CGCTACCAAGCAATCAGGTTGCCCAACAGCTAATTGACATCGTTTAGGGCGTGGACTACC
AGGGTATCTAATCCTGTTTGCTACCCACGCTTTCGGGCATGAACGTCAGTGTTGTCCCAG
GAGGCTGCCTTCGCCATCGGTATTCCTCCACATCTCTACGCATTTCACTGCTACACGTGG
AATTCTACCTCCCTCTGACACACTCGAGTCACCCAGTTCAGAACGCAGTTCCCGGGTTGA
GCCCGGGGATTTCACATCCTGCTTAAGTAACCGTCTGCGCCCGCTTTACGCCCAGTAATT
CCGATTAACGCTCGCACCCTACGTATTACCGCGGCTGCTGGCACGTAGTTAGCCGGTGCT
TATTCTTCAGGTACCGTCATCAGCCGCTGATATTAGCAACAGCCTTTTCTTCCCTGACAA
AAGTCCTTTACAACCCGAAGGCCTTCTTCAGACACGCGGCATGGCTGGATCAGGCTTGCG
CCCATTGTCCAAAATTCCCCACTGCTGCCTCCCGTAGGAGTCTGGGCCGTGTCTCAGTCC
CAGTGTGGCGGATCATCCTCTCAGACCCGCTACTGATCGTCGCCTTGGTAGGCCTTTACC
CCACCAACTAGCTAATCAGATATCGGCCGCTCGAATAGCGCAAGGCCCGAAGGTCCCCTG
~~CTTTCTCTCTCAAGACGTATGCGGTATTAGCTGATCTTTCGATCAGTTATCCCCCACTAC~~
TCGGTACGTTCCGATATGTTACTCACCCGTTCGCCACTCGCCACCCGAGAAGCAAGCTTC
TCTGTGCTGCCGTCCGACTTGCATGTGTAAAGCATGCCGCCAGCGTTCAATCTGAGCCAG
GATCAAACTCTTATGTTCAATCTCTAACTTTTTAACTTCTGGTCTGCTTCAAAGAAACCA
ACAGGACAATGTTCAAAACATTATCTTGTCTGTCTTTCAAACAGTGTGAGACTCAAGGCA
CTCACACTTATCGGTAATCTGTTTTGTTAAAGAGCGTTGCGAATTATAAAGTATTCCTTC
CGCCTGTCAAGATATCTCTCGATATCCCCAACATTCTGTGCTATACTTTTCAGTTCGTCC
GCCACTTCTGCAGCAGCGAAGAACCGAACTATACGCCCACAGGGAAAAACGGTCAATGCT
TTCAGCGGGATTTTTTTGGGGAAATTCGTCATGTCGCTGTCGGATAAGGTTTTTTATTTC
TGCTAAATACTGCGCCGCCTCCAACAATCCTTTCCTCTCCCTCCTCCGGCTGGTGCGCCT
TTGTGAATATGCTGTCTGAAACTCGGGGACTCAGACGGCATTTTGTATCCAAACGGTATC
TAATGTATCCGTACTTTGTTATAGAATGGCTGCTGTTTTTTCTTCGTAATTAGAAATTGT
CAAAATGGGCAAACATATTCTTTTAGGTGTAACGGGCAGTATTGCGGCGTATAAGTCTTG
CGAGTTGGTGCGACTGCTGAAAAAACAGGGGCATTCGGTTACGGTGGTTATGAGCCGCTC
GGCAACTGAATTTGTTTCTCCGCTGACTTTTCAGGCTTTAAGCGGCAATCCTGTCCTGAC
CGACACGCACGGCGGCAACGGTTCAAACGGTATGGAACATATCAACCTGACCCGGAATGC
GGATGTTTTTCTGATTGCGCCGGCAAGTATGAATACCGTGGCAAAAATCTGTAACGGCGT
GGCAGATAACCTACTGACCAGTCTGGCAGCCGCACGGAAATGTCCGCTTGCCATCGCGCC
CGCGATGAATGTGGAAATGTGGCTCAACCCTGCCAACCAACGGAATATCGCACAACTGGT
TTCAGACGGCATTACTGTCTATATGCCGGGCTTGGGCGAACAGGCTTGCGGAGAAAATGG
TATGGGAAGGATGCCGGAACCTGCCGAATTGCTGGATCTGCTTCCGGATTTATGGACACC
GAAAATTTTAAAGGGCAAAAAAGTCTTGATTACCGCAGGTGCGACATTTGAAGCCATTGA
CCCTGTCCGAGGCATCACAAATATCTCCAGCGGGAAAATGGGCGTGGCTTTGGCGCGGGC
GTGCCGTGCCGCCGGTGCAGAAGTCAGCCTGATTCACGGACAGCTTCAAACCGCGCTGCC
TTTCGGCATATCCGATACGGTTCAAGCCGTCAGTGCCGAAAATATGCATCGCGCAGTGCA
TCGTTTAATCGACAAACAAGATGCTTTTATTTCTGTTGCCGCCGTCTCAGACTATAGGGT

## Appendix A

TAAGAATAGGAGTACTCAAAAATTCAAAAAAGATAAAAATGCCAAACCGTTATCCATCGA
ATTGGATGAGAACCCCGATATTTTGGCTTCTATTGCCTCATTACCGAACCCGCCGTTCTG
CATCGGTTTTGCCGCTGAAACGGAGAATGTAATGACATATGCGCGGGAAAAACGTATTAA
GAAAAAGCTACCGATGATCGTTGCCAATGATGTTTCAATCGCAATGGGCAAACCGACCAA
CCGGATTACCATTATCGGGGACGACGGGGAACTGTCTTTTCCCGAAACAAGTAAAGATGA
AGCGGCAATGCGGATTGTTGAAAGGCTTGCCGTATATTTGAGCAAATAAGCAATTGAACG
GATAAACCATAAAACGGGTTGCCTGTTAATCAAAAGGCAACCCGTTTTACCTGCTTCAAC
TTCTGATGACTTTGCGGATATATGGAATACTATGCAGATTTTGAATAATCTGATTCAATT
GATTCAGATTCTTGACTTTCAATAAGAATTTGAATTCGACAAAACCTTCCGTTCCCGACT
GGGATTTAGACGGTGTTTCGACCGACTCAATGTCTGCACCGGAATCGGAAATCGCTTGCG
CCATTAATGCCAACAGGCCGTGGCTGTCTTCCGATTGGACTTGAAGCCCGACACGGTAGT
TCTGCCCGTTCATATTTTCCCAGTCTGCATCCAGCTGCTGTTCGGGATCGGACTTCAACA
ACGTCGGGCAGGTATCCCTATGGATAATCATGCCTTTTCCCTTAACCAACAGCAAACGGA
TGGAATCGCCGGGAACAGGGTGGCAGCACTCTGCAAAATGAATATGCCCGCTTTCCTGCC
CATCGACTTTAATGGAACTGAGCCTGACCTCGCTGCCGAAATGCTCCCCTGCCAACTCGG
CAATGTGCATGGCGACATAAACAGGCAGGGTATGCCCCATCCCTACGTTGTACAGCACTT
CTTCAAACGATGTCTGCTTGTCGTTGAGATCGGCAAGATATTTTTCCTTGATGCCGTCTG
AAAGCAGGACATCTTTGGGCAGCAAACTGGACAGGGCTTTTTGTAAGAGGCTCTCTCCCA
AAACGACCGCATCGTGCCGGTTAAGGTTTTTAATATATTGGCGTATGGCGCTGCGCGCCC
TGCCTGACACGGCGAAATTCAACCACGCGGGATTGGGTTTGGCGTGTTCGGATGTGATAA
TTTCAACAGAATCACCGGTTTTGAGCTTCGTACGCAACGGCATCATGATATTGTTGATAC
GTGCGGCAACGGTTTTGTGCCCGATATCGGTATGCACCGCATAAGCAAAATCGACAGGCG
TTGCCCCTTTGGGCAAAGTTAGGATTTTTCCTTTTGGCGTAAGGATGTAGATTTCGTTCG
GAAACAAATCGACTTTGACGTGTTCGAGAAACTCAATGGCATTGGCACTGCTTGCCTGCA
AATCTAAGATATTTTTCAGCCACCGGTTTGTGTGAAGCACCGCCTGATCGACCGTCTTAG
AATATGATTTATAGCTCCAATGTCCGGCGATTCCACCTTCGGCAACAGCATCCATTTCCT
TGGTACGTATCTGAACTTCAATCGGCAAGCCGTAAGGGCCGACCAAAGTCGTATGCAGAC
TTTGATACCCGTTGCTTTTCGGAATGGCGATATAGTCTTTGAACCGCCCGGGCTTGGGCT
GATAGAGGGTGTGCAATGCGCCGAGTGCGGCATAACAGGCTGGAATGCTGTTGACAATGA
CGCGGAAACCGTAAATATCCATAACCTCGGCAAAGCGCAGCTTTTTCGCCATCATTTTCT
GATGGATGCCGTACAGGTTTTTTTCCCTGCCTTTGATTTTGGCCTCTATATTCGCGCCTA
CCAGCCGCTGGCCGAATGCGCGCAAGACTTTGCCGACAACGTCCTGCCGGTTCTTCCGGC
TCTTGTCCATCGCTTTTTTTAAAGTCTCGTAGCGGTTGGGATGCAGGTTTTGGAACGATA
AATCCTGAAGCTCTTGATATGCGTTATTCAAACCTATACGGTTGGCAATCTGTGCATAGA
TTTCAAGGGTTTCCCTTGCAATCCGGCGGCGTTTGTCCGGGCGCATCGAACCGAGCGTCC
GCATATTGTGCAGGCGGTCGGCAAGTTTGACGACAATCACGCGCACATCTTTGGTCATTG
CCAAAATCAGTTTGCGGAAACTCTCCGCCTGATGCTCCGCATGATCTTCAAATTTGAGTT
TTTCAAGCTTGGACAGACCGTCCACCATCTCGGCAATCGTATTGCCGAACACCGCCGCCA
TTTCCCCTTTTGTCACGCCCGTATCTTCCAATACGTCGTGCATCACGCCTGCACAAAGAC
CCTGTATGTCCATATGCCAAAGGGCGAGCTGCGTCGCAACGGCAATCGGATGCGTGATGT
AGGGCTCCCCGCTTTTGCGGGTTTGCCCGTCGTGGGCGCGAAACGCATAGGCGACAGCTT
TTTCAAGCTCCGCCTGTTCCTCGGGCTTGAGGTAGGAGGCGGTATGGAAAAGCAGGGCAC
GCGCTTCGGCGGTCAGGGGGTCGTAAGGGGCGGAAGGTTGGGGGGCGGGCATTTCAGACG
GCTTTCGGTATGTATGTGTTTTTCATTTCAAACCGTCGGACTGCACGGCGGCAAAGTGTT
CCGGCGTGCGTTTCCGGCAGAATTTATTTATTGCGCGTCAACAGTTCTGTACCGATATGT
CCGGCGGCGATTTCCCTTAAGGCGGTAACGGTCGGTTTGTTATTGCGGACATCGTCCACA
AGCGGCGTGTTGCCGTTCTCAAGCTGGCGGGCGCGGCGAGCCGCTACCAATGTCAGGTCA
AAATGGTTGGAAATTTTTCCGGTACAGTCTTCGGTGGTAATACGTGCCATATTATTTGCT
TTCTTTCAAAAATATTTAAATTGGGAAACCGGGTATTTTCGCCGTTTTCTAGGAATTTTC
CAACAAATCTGCAATAAACCCCAGTTGCCGCGACCTTTTCAGACGGCAGGCATTCACAAT
ATGGCGCAAATCCTCCTCCGCTCGCGCCAAGTCGTCATTGACCACGACAAAGTCAAACAA
TACGGACTGCTCGATTTCATGCCTTGCCTTCGACAGCCTCCTTTGGATAACTTCCCGACT
GTCCGTCCCGCGTCCGTTGAGGCGCGCGGCAAGTACGTCGAAAGAAGGCGGCAGGATAAA
GATGCCGACGGCTTCGGGCAGCGCGTCGCGAACCTGCGCCGCGCCCTGAACGTCGATTTC
CAAAATCACGTCATAGCCTGCCGCCGCCAACGCATTCACACCCTCCGCGCCTGTGCCGTA
ATAGTTGCCAAATACGTCGGCGTATTCCAAAAAAGCTTCCTGCGCGATAAGCGACTCAAA
CTCTTCTTTGGAAACAAAGTGATAATGTACGCCGTTTGCTTCGCCTTCACGCGGCGGGCG
CGTCGTGTGCGACACGGAAACGCGCAAACCGTTATGGTTTGCCAACAGCCGCGACACCAG
CGTGGTTTTGCCCGTGCCGGAAGCGGCCGAAATGATAAAGATGTTGCCTTTTCGATAAGC
GGACATATTTTTTACCTGTATATTTTCCAGCCGATTGTATCACAATGGACACCCAGTTTC
CTATTTGCCGATGCCCATATTTTGCCGCTATTGTTTTGATTGATTTGGCAAGCGACAGGC
TGACGGCTACAATATGGCGTTAAAAACATCAAACTTGGAACACGCAATGCTGGTTCATCC
CGAAGCTATGAGTGTCGGCGCGCTTGCCGACAAAATCCGCAAAATCGAAAACTGGCCGCA
AAAAGGCATCTTATTCCACGACATCACGCCCGTCCTTCAAAGCGCGGAATACTTCCGCCT
TTTGGTTGATTTATTGGTTTACCGCTATATGGATCAGAAAATCGACATCGTTGCCGGTTT
GGACGCGCGCGGCTTCATTATCGGCGCGGCACTCGCCTACCAGCTCAACGTCGGTTTCGT
CCCCATCCGCAAAAAAGGCAAGCTGCCTTTTGAAACCGTATCGCAAAGCTACGCGCTCGA
ATACGGGGAAGCTGCGGTGGAAATCCACACCGATGCCGTCAAACTCGGTTCGCGCGTGCT
GCTGGTCGATGATTTGATTGCCACGGGCGGCACGATGCTTGCCGGACTGGAACTGATCCG
CAAACTCGGCGGAGAAATTGTCGAAGCCGCCGCCATTTTGGAATTTACCGACCTTCAAGG
CGGCAAGAATATCCGTGCAAGCGGCGCGCCCTTATTTACCCTGCTTCAAAACGAAGGCTG
TATGAAGGGCTGAAAACCGACCCTGCCGTCTGAAACCGGCAGGGTTGTTATGATGCGTTC
AAATCACGCCCAAATCTTGCAAGCCCCTCAACACGCCGTCTTCATCAACGCTGGGGCAAA
CATATTTCGCCGCTTCTTTCGCCGCCTGTTCCCCGTTGCCCATTGCCACGCCGAACCCGA
CTTCTGACAGCATTTCCACATCGTTCAAACCGTCGCCGAACGCCATCACGTCTGCCATTT

## Appendix A

CCCATCCCAATGCTTCAACCACGCTTCTGATGCCGTCCGTTTTCGACGCACCCGCAGGCA
GCAGATCGACCGCTTCCTCGTGCCAGCGCACCGTTTTCAAGCCTTCCCGTTCCACAATAT
CCGACCAAAGCGGCATTTCGTTTTCCTCCGCAAACACCAGCATCTGATACACCGGTTTGC
TTGAAAAATAATCCTTATCGGCAAAAAAATCGCTGGCGATATGCTTCAAGGCGCGGCACA
CGCATTCCGACAGCGCGGACACAGCGATCCCCTCTCCGCCGACAAACGCATAATCCATGC
CCAAGCCATCCAAATGCGCGCAAACCCTGCCCATCAAACCGGCATCCATCGGTACTTCGC
GCACGGTTTTTCCGTGCAGCAGCGCAAACTGTCCGTTTATCGTTACCACGGCATCCATTC
CCGCTTCCGCCATCATATCCCTGACCTTTTCGGGAATCGTCGCCAAAGACCGCCCCGTTG
CCAACGCCGTCAATATACCTTTGCCGCGCAAAGCCGCCACCGCCGTTTTCACGGAAGGGC
GCAAAGTATCCGTATATTTTCGGTACAGCGTATCGTCAATGTCGAAAAACACGATTTTAG
GATTCATCACATTCTCTCTCGCATTCAAACTACCGCATTATATCCCAAGCAGGCAAATAC
TTGATAAATCCTTATAAATTTCCCGTCAAAATTGACCGAAAATACAAAAAGGCGGATAAT
CCGCCCATCCTCAAACCCTTTTCAGACGGCATTTGCAGCAATGCCGTCTGAAACATTTTT
ACAAAGCATACAAATCATGTTTCAACACACAGGACGACACATAAAGCGTCGCCCTATATG
TTGCCCTGATTCGGAAGGGGTTACGCCCCTCCCAAATAAAGTCTGATTCTACTGCCCTAA
AGGGCGGGGTTTCAACCGAAAAGGAAACACGATGAAAGCACCCGAACTCTTATTGCCCGC
CGGCGGATTGGAAAGAATGCGCGCCGCCTACGACTACGGCGCAGACGCCGTTTACGCCGG
CAGCCCGCGTTACTCACTGCGCGCCCGCAACAACGAATTTGCCAAACTTGATGTTTTAGA
ACAAGGCATTAAAGAAGCGCACGAGCGCAACAAAAAATTCTTTTTAACCGTCAACACCCT
GCCGCACAATTCCAAACTCAAAACCTTCGTTGCCGACATGGAGCCGCTGATTGCCATGAA
ACCCGACGCGCTGATTATGGCGGATCCGGGTTTGATTATGACCGTGCGCGAAAAATGGCC
GGAAATGCCGATCCATCTGTCCGTACAGGCGAACACCACCAACTATTGGGGCGTGAAATT
CTGGCAAAACATCGGCGTCGAACGCATTATTCTGTCGCGCGAATTGAGTATGGAAGAAAT
CGCCGAAATCCGCCAAGAATGCCCCGACATCGAACTCGAAGTCTTCATCCACGGCGCATT
GTGCATCGCTTATTCAGGCCGTTGCCTATTGTCGGGCTATTTCAACCACCGCGACCCCAA
CCAAGGCACCTGCACCAACTCCTGCCGTTGGGATTACAAGGTTCACAATGCCACGGAAAG
CGATGCAGGCGATGCCCAGCTTCTGCAAGGTTTCAACTTTGAAAAAGCCCAAGAAGAAGC
CAACCAAAACTTTGAAGGCATCAACGGTCAAAAACGCCATCCCTACGCCGACAAAGTTTT
CCTGATTGAAGAATCCAACCGCCCGGGCGAAATGATGCCGATTATGGAAGACGAACACGG
CACCTACATCATGAATTCCAAAGACCTTCGCGGTATCGAAGTCGTCGAAAAACTCGCCAA
AATCGGCGTGGACAGCCTCAAAGTCGAAGGCCGTACCAAATCGCTCTATTATGTTGCACG
CGTCGCCCAGTCCTACCGCAAAGCGATTGATGATGCCGTCGCAGGCCGTCCGTTTGATTA
CAGCCTGTTGAGCGAACTCGAAGGCCTCGCCAACCGCGGCTACACCAGCGGCTTCCTCGA
ACGCCACCAAACTCAGGATTATCAAAACTACCTGACCGGCCATTCCACCGCCAAACAAAG
CCAATACGTCGGACACGTTACCGAAATCGATGAAAACGGCTGGGCAACAGTGGAAGTCAA
AAACCGCTTTGCCGTCAGCGATTCACTCGAAATCATCCACCCGAGCGGCAACCAAACCAT
CAAATTGGAACAAATGACCCGCAAAGGCCAGCCTGTCGATGTTGCCCCGGGCAACGGCAT
TCAGGTCAAAATCCCCAATATGCAGGGTAAAGAAAAAGCCCTCATCGCACGCGTGTTGAA
CCCCTAAGCCATTATGCCGTCTGAAACATTTTTCAGACGGCATTTTTAATCCCCTTGCCT
TATTGTGCGGCAGATTCAGATCGGGACACACCTATAGTCCACGACAGAAGTCTGGCTTTT
AAGCGAAGACAATAACCGAAAAGACCGCCTCGGCGGTCTTTCTTGTGTACACAGTTTTTG
TATTTGTCAGCTTGATGCGTTGACAACTCTAATTCCATATTGCGGAATATATTCATCGAC
AGTCATCAGTTCAAAGCCTTCCGCTTGGGTTTGTGCAATCAACATCCTATCGAAAGGGTC
TTTGTGTATCTCCGGAAGGCTTCCAGCCTGTTTTGCATGAAACAGACCTATAGGCAACAT
TTCAAAATCCTCTTCTTGAAGCACATCAAAAAACTCTTCCGGTAATTTCAACAACCCCTT
GTTCTGCTTGATGGAAATTTCCCAAATACTTGCTGCACTGACAAAGATCGCATTTCTCGG
ATTTTCTATCAGTTTGCGTGCAGATATCCCCAGTTTCTTGTCATCCAACAACCACCACAG
CAACGCATGGGTATCAAGCAGAATCTTTCTCACAGAGCCGACTCCTCAAAAAATAAAGCT
GCCGTTTCATTGTCATCCTCAAGAATACGTGAAATATCCGTATTTTCCATATGACTGAAT
TTTTTCAAGCTTGCTGCATTTCGTGCCGGTTTTTCAATACCGATTAGTTGGACGCAAGGC
TTACCTGCCTTCGCAATAATAACGATTTCCCCTGCTTCTGCTCTTTGAATCAATTGACTC
AAATTGGTTTTTGCCTGATGAATATTTGCTTGAAACATAACACTTCTCATGATTAGCTAA
CTTGACTAATATACATCATTACCAAGATTTTGGGAATCTCATTACATATATTTGATTATA
TCCGCCGTTTTATTCACACCTTGCTATTTATAGTGGATTAACAAAAACCAGTACGGCGTT
GCCTCGCCTTAGCTCAAAGAGAACGATTCTCTAAGGTGCTGAAGCACCAAGTGAATCGGT
TCCGTACTATCTGTACTGTCTGCGGCTTCGTCGCCTTGTCCTGATTTTTGTTAATCCACT
ATAAAACGGCTTTGCGGTATCCCAGTTTGACACCGGTTACTTCCTGATTGGTAAGCATCA
TTATTTTCCCATAAATCAAACGTCTGACACGGCATTATAAACACAATGCGGCATCTGCCG
CCACCCTTGCGGACGCGGCGTTACCGGCTTCCACAGCTACTTCGACAAGCAGCCGCTGCA
AGGCGGACAATACGACTGTCAGGCAGGCTCGTTCCACGTCCGCGTGGTCATGCGCGCCAA
CGTCGTCCGTTAACATATCGGCAACCAAAAAATGCCGTCTGAAACATTTTTCAGACGGCA
TTTTTAATCCTGCAACATTACCCCCTGCCTGAGTTCGGATACTGTATCAATATAAAACCC
CATCACACAGATTTACGGTAAAAAGCCGTCCGAATGAATTCTTGAACACAATTCGGACGG
CTTTAATTTTCAACAAGGCGATTAATTCAATAATACCAGATTAAAACTTCCATTCCAGCG
ATACGGCGTAATTGCGGCCTGGGGCGCGGTAGCGGTCTAAGCCTTTGCCATCGCGGTCGA
CCGCATTGGTGGTGCTGTAGCTATATAAACCGCGCAGGGAATCCCAAGTGGTGTATTTGC
GGTTGAACAGGTTGTACACGCCTGCACGCAAAGTCAGGTTTTTAGCCGGTTTGTAGAAGC
CGTACATATCAAACACATAAGCCGACTTGTTCAGCCACGGGTAATCTTTTACCTTTTTCT
GCAAAGGCGTACCCCAGCCCTTGTTTTCATAAACGGTGTATTGCGCGTCTTTGACCTTTT
TCGCGCCTAGATAGGTCAGGCGGGAGAATACGCCCCATTTTTCGCTCGGACTTTCATAGT
CGATACCGGCAATCACTTTCAGCGGCTGTGTGGACAGCAGGCTGTTGTCGCCCGACAGTT
TGCTTTTCGCATAACCCAGCGAGCCGAACAGTTTCCAACCCTCAGGAACAAAAGACGCTA
CTTTGTCCACATTCAGACGGCCTGTCAGCTCGATACCGCGGATTCTGGCCTTGTCGATAT
TTTTCATCTGCCAATCCAGTTTTTCTTTGTAGGGGTCGCTGCATATACCGTAGTAAGCAT
TTTCCTCAGTACAGCCGGGAGTGCCGCTGGTGGTCAGCTTCTGCTCTTCAGACAGGAAAT

## Appendix A

TGCGGTAATTGCTTTGATACAGGTTGGCATCCAGCATGCCTTTTTCGCTGCGGCCTTGCA
GAGACAGGGTGTGGGTGGTGCTGCGCTCGGCTTTCAGGTTGGGATTGGGCAGCCAATTAC
CCGAACCGTGGTTGTAAGTGAAATACACTTCGGACGCATTGGGGACACGGTAGCCGGAAG
TAATGTCGTAACCGACACGCCAAGCCTGATTCAGTTGCGCCGCCAAGCCGACAAAACCGC
TCCAGCCTTTATAAGTGTTGGCTGCAGGTGGTGTTTTGTCACAAGCATGACACTCGGCAT
TCAATTCCTGAGGCGTCATTTTGGTGTGGTCGTAACGGATACCTGCGCGGCTACTGAACA
CGTCGTTCCATTGAATTTGGTCAGACAGTGAGAAACCGTAGTTGGTGGTTTTCACCGGAT
GCTGGATACTGCTGGTGGTTCGAACAACACGGCCGCTGAAGTAATAATCGTCGCGGTTTA
GGTTTTCAAAATCACGGCGGCTGACGAAAGTTTTAAACGACAGGCGGTGTCGCCCCCCCC
CGAGTTGCAACGGATGGCTGTCCAAACGCAAAGTAAAACGTTTGAATCGGGTGTCCATGC
TGCGGTTGTATATTTCGTCCAAATCCTTCTGATTATAGTTGCGCGTCCAGGTGGAATAAT
CCATCGGGAACGAGCCTTTGTTGTTAACCGCCGCCACTTTGGTTTTCTGATAATCGAAGT
CCGCCTTCAAAGACGACAACCAATTTGAATCAGGCATCCATTCGTAAAAGAGGTTGGCAT
TGCGCCGTCTGTTTACGTCATCGGCTTCGCGCCAGGAAGAAGCGGTCAGGTTATAAGACT
CTTCAACCGTGTAATTATGTCCCTGCTGGCCGTTAAGCGATGCGCCGATGCGGTGGTTAT
CGTTAATTTGGTAAGCAATCTTACCCAAAAAGCTGTGGTATTTGTGTTTGGACGAATCAG
GGATACCGCGTGCCGAACCACGGATATTCGCGCCACTGCCTTCCCCTTCCACAGCATAGC
CTCGGTTTCCCGCACTTTCGGTTTCATGACCGCGACGTTGCGAATACAGCAAAGCAGCAT
CCACGCGGTCGTTACTCACACCGAAACCGAGAGTATTTGTCCATTCACGGTTACGCGTGC
TGTAACCGTTTTTCATCATCACGCCGAATTGCCTGTCGTCCAACAGCAAATCACGGCCTT
GCAGCGTTTGGTAATTCACACCGCCGCCCAATGCACCACTGCCGGTATTGAAAGAGTCTG
CGCCCTTCACGATTTCGATGTTGCGCACGAGTTCGGGGTCGATAGACAAACGCGAGCTGT
TGAAGTTGCCATAACGGGCGTACAGCGAGTTTTCTTCAGAATCAGGCAGGTTTACACCGT
CTATGCTCACGCCGACACGGTTGCCTTCCACGCCGCGAACAGCAAAGCCTTTTTGATGGC
GGCCGCTGTCGCTCAAGCCGACATCGGTGGAATAGCGCACCAAGTCTTTATTGTCGCGTA
TCATTTCTTGTTTGATACGGTTAAGGTTGACGCGTTCCACAGCCGCAGGCGCATTGCGCT
GACCTTTAACGCGCACTGCTTTTATCTCTGCCTTAACGGGTGTGGTTTCAGTTGCAGCTT
CATCTGCTGCCAAGACCGGATTGCCGAAAATACTGCCGACCAGCGCGGCGATAGGGAGCA
TTTGTAATGGTTTCATATTATCAACTCAAGATGTATGGATTGTTCATCCATCGGTTAGCG
AATAATAGATTTTATGATAATCATTAATATTTAATAAGACAGTAATCCATGTAAACAAAG
CCGCGCCGTGTAATTAAAGGTCCCTGCAAACAGCTATGCCGAGACCTTGTTTATTTGGTT
CGATTCTTGTTTATTTGGTTCGATTATTTTTAGTGCCTGTGCGGCATCATTCCTTCCGG
TGCTTCGGCATCGGCTGCCAAGCCGAAGGTTTCGCGCAACACGACTTTGTAGAATGCAAA
GGCTTCGCGCGCGCCTTGGATGGCTTCCGCTTCGGCTTCGGGAGTCAGGTTCAAAGCGTT
CAGATGCTCGACGAAAGCGCGCCAGTGTTTGCCGCGCCCGTCGGGATGGGGTGCGAGGTG
GCGCGCGCCGTGTTCGCCGTTGTAATCGAGTTTTTGGGCGTGTTTGAACAAAAATGCCGC
GCCCAAATTGGATCCTTCGGCGCAATAAAGCCAGCCGATTGCTTTGTTGCCGGTTTCATG
CGGCAGCGGTTTGCCGTATTCGTAAGGTTTGTCACCCAAATCTGCAAGGTCTTGCGTTAC
GGCATCGTATCGCGCCATGTATTCCAGCTCGGGAATGGCTTTGTTTAATTCGGCATCTTT
ATAGATGTGGTCGACAGCCTTGTGGAAAACGGATTGGAGTTTCAAAAATTTGATGTAGTT
TTCTTTGCTGACAAACGGTTGGACAGACATAACGAGGTTATCCACGCTGTCGTGAACCGC
CGTGGTATCCGCCTTCAAGCGTTTGGCAAATGTCAATGCTTGATTTTCGGTTTCACTCAT
ATTTTTTCCTTTGTCGGTAAGGGATAAGGATGCGGTGCGGGCAAAATCCGCACTTGCCCG
CATATATAGTGGATTAACAAAAACCAGTACGGCGTTGCCTCGCCTTAGCTCAAAGAGAAC
GATTCTCTAAGGTGCTGAAGCACCAAGTGAGTCGGTTCCGTACTATTTGTACTGTCTGCG
GCTTCGCCGCCTTGTCCTGATTTTTGTTAATCCACTATAAAAATAATAAAGAATCATAAA
CGAAATTTATTATCACATATTTTTGGAAAAAATATCATTTGCGTGATGTTTTTAAGCAGG
TATTTTACTATTCTTTACAGAATCGGGATTTTATCAAATGGGTTCGGCAGTCGGCGGACA
ACCGCTCAAAAAATATTTTTGCCGGACACCAAGGGTTTGTTCATACTGCCGAACCTGCCG
~~GTTTTGCATCCTGATTGGGTGTATCGCCTTTTTTCCTTTATAATGCCGCCACTTATATTT~~
GCCACTTTCCCGATGAAGCCGTTTGCCGAAAATATCCCCCACAGCCTTCGCGGCAACTGC
TGCGACGAAGCCCTGCCGCCGCATACGGTAGATTGTCCGGAATGCGGCTGCCGCGCGGAT
GTACCCCGGTTGGACAGTGGAGAAGCGGCGTTCTGTCCCCGTTGCGGACACAAACTCTTC
AGGGTGGGCAGGCATCCTTTTTCCGCCCCGCCCGCCTATGCGGCGGCTTCGCTGATTTTA
ATGGCGTTTGCTTACGGTATGACGTATATCGAGGTCGGGATACCGGGTGCGGCATCCGTC
CTTTCGCTGCCCGAGATGATGCGCCTGATGGTGTTTCAGGATTATGGTTTTTTGGCCGAA
GTGATGTTTGTGCTGACTTTCGGCGCGCCGGTTCTGTTTCTGCTGCTGTGCCTGTATGTC
TATGCCGCGCTGATACGGAAACAGGCGTATCCTGCGCTGCGTTTGGCAACGCGTGTGATG
GTGCGCTTGAGACAGGCGATGATGGTGGATGTGTTTTTTGTTTCCACTTTGGTGGCGTAT
ATCAAGCTCTCGTCTGTGGCAGAGGTTCGCTTCGGGCCGGCGTTTTATCTGATGTTCGCG
CTGTCAGTTATGCTGATTCGGACTTCGGTATCGGTTCCCCAGCATTGGGTGTATTTTCAA
ATCGGGCGGCTGACGGGGGATAATGCGGTTCAGACGGCATCGGAAGGTAAAACCTGTTGC
AGCCGCTGCCTGTATTTCCGCGACAGTGCCGAATCCCCCTGCGGCGTGTGCGGTGCGGAA
CTGTACCGCCGACGGCCGAAAAGTCTGAGTATTTCGTCGGCGTTTCTGACGGCGGCGGTT
ATTTTGTATTTCCCTGCCAATATCCTGCCGATTATGATTTCGTCCAATCCTGCCGCCACG
GAGGTCAATACCATCCTTAACGGCATCGCTTATATGTGGGACGAGGGCGACAGGCTGATT
GCGGCGGTTATTTTCAGCGCGAGTATTTTGGTGCCGGTACTGAAGATTGCGGCAATGTCG
GTTTTGATTGCGTCCGCCCGCTTCGCTTTGCCAACGGGTGCAAAGAAATTGTCGCACCTC
TACCGCATCACCGAAGCGGTCGGCCGCTGGTCGATGATTGATATTTTTGTGATTATTATT
TTGATGTGTTCGTTCCACACTTATGCCGCGCGCGTCATTCCGGGCAGTGCGGCAGTCTAT
TTCTGCCTGGTCGTGATTCTGACGATGCTGTCCGCCTATTATTTCGACCCGCGCCTGCTT
TGGGACAAACGCGCTTCAGACGGCATTGCTTTCAATGAAACGGAAAAACATGACTGACAA
CAGCCCTCCTCCAAACGGACACGCCCAAGCACGCGTCCGCAAAAACAACACCTTCCTCTC
TGCCGTCTGGCTGGTTCCGCTGATCGCGCTGATTGCCGGCGGCTGGCTTTGGGTTAAGGA
AATCCGCAACAGGGGGCCTGTGGTTACGCTCTTGATGGACAGCGCGGAAGGCATTGAGGT

## Appendix A

CAACAATACGGTCATCAAAGTATTGAGCATCGATGTCGGACGCGTTACCCGAATCAAACT
GCGCGACGACCAAAAAGGCGTGGAAGTAACCGCCCAACTCAATGCGGACGTATCCGGCCT
CATCCGCAGCGATACCCAGTTTTGGGTGGTCAAGCCGCGTATCGACCAAAGCGGCGTAAC
CGGTTTGGGTACGCTGCTTTCGGGTTCGTACATCGCCTTTACACCCGGCAAAAGCGACGA
GGCAAAAGACGTGTTCCAAGTGCAGGACATTCCGCCCGTTACCGCCATCGGGCAAAGCGG
GCTGCGCTTGAATTTGATTGGTAAAAACGACCGCATCCTCAACGTCAACAGCCCTGTTTT
GTATGAAAATTTTATGGTCGGGCAAGTCGAAAGCGCGCATTTCGACCCGTCCGACCAAAG
CGTGCATTACACCATCTTCATCCAAAGCCCCAACGACAAACTGATTCATTCCGCCAGCCG
TTTTTGGCTGGAAAGCGGCATCAATATCGAAACCACAGGCAGCGGCATCAAACTCAATTC
CGCCCCTCTGCCTGCCCTGCTGTCGGGCGCGATTTCATTTGATTCGCCGAAAACCAAAAA
CAGTAAAAACGTCAAAAGCGAAGACAGCTTCACGCTTTACGACAGCCGCAGCGAAGTCGC
CAACCTGCCTGACGACCGCTCGCTGTACTACACCGCGTTTTTCAAACAATCCGTGCGCGG
CCTGACCGTCGGTTCGCCCGTCGAGTACAAAGGGCTGAATGTCGGCGTGGTTTCCGACGT
TCCTTATTTCGACCGCAACGACAGCCTGCACCTGTTTGAAAACGGCTGGATACCCGTACG
CATCCGCATTGAACCTTCCCGTTTGGAAATCAATGCCGACGAACAAAGCAAAGAACATTG
GAAACAACAATTTCAGACGGCCTTAAACAAAGGCCTGACCGCCACCATCTCCAGCAACAA
CCTGCTGACCGGAAGCAAAATGATTGAGTTGAACGATCAGCCTTCCGCATCACCTAAGCT
GCGACCGCATACCGTTTATGCAGGCGATACCGTTATCGCGACCCAGGGCGGCGGTTTGGA
CGATTTGCAGGTCAAATTGGCGGATTTGCTGGACAAGTTCGACAAACTGCCTTTAGATAA
GACGGTTGCCGAATTGAACGGTTCGCTTGCCGAGCTCAAATCCACACTCAAATCTGCCAA
TGCCGCCCTAAGCTCCATCGACAAACTGGTCGGCAAACCGCAGACACAAAACATTCCGAA
CGAACTGAACCAAACCCTGAAAGAGTTGCGCACAACCCTGCAAGGCGTATCGCCGCAATC
GCCTATCTACGGCGACGTACAAAATACGCTGCAAAGTTTGGACAAAACTTTAAAAGACGT
TCAACCCGTGATTAATACTTTGAAAGAAAAACCCAACGCGCTGATTTTCAACAGCAGCAG
CAAAGACCCTATCCCGAAAGGAAGCCGATAATGCGCCTTTTCCCGATTGCCGCCGCCCTG
TCGCTTGCCGCCTGCGGTACTGTGCAAAGCACACAATATTTCGTGTTGCCCGACAGCCGC
TACATCCGTCCTGCAACGCAAGGCGGCGAAACTGCCGTCGAAGTCCGTCTTGCCGAACCG
CTCAAACGCGGCGGACTGGTCTATCAAACCGACCCCTACCGCCTCAACACCGCACAAAAC
CACGTCTGGGCAGACACCTTGGACGATATGCTCGAAGCGGCGTTGAGCAATGCATTCAAC
CGTTTGGACAGCACACGCATCTTTGTTCCTGCCTCACGCAGCGGCAGTACCGAAAAATGG
ACGGTCTATATCGACGCATTCCAAGGCAGCTACACGGGCAAAACCCTCATCAGCGGCTAC
GCCGTCCTACCCGACGGTACGAACAGACCCTTCCATATCGAAACCGAACAGCAGGGTGAC
GGCTACGCCGCGATGACCGCCGCACTCGAACAGGGACTGAAACAGGCGGCGCAACAGATG
GTCGAGTAAACCGTGAACTATTGCGAATTTGCCGCCTCACTTCCCGAAAACACCGATAAC
CCGAACAAACATTACCACGACACGCAATACGGTTTTCCGATTGAGGACGACAATGAATTG
TTTGAGCGGCTGGTGTTGGAAATCAATCAGGCAGGATTAAACTGGACGCTGATGCTGAAG
AAGCGGCAGGCGTTTCAGACGGCATTTGAAGGTTTCGACATCGATACGGTTGCCGCCTTC
GACGACACCGACCGCGAACGCCTGCTTGCCGACGCGGGCATTGTCCGCAACCGCCTGAAA
ATCGATGCCGCCATTTTCAATGCACGGCAAATCCAAGCGTTGCAACAAGAATACGGCTCG
TTCAAGAACTGGCTCGACACGCACCATCCGCGAAGCAAAGACGAATGGGTTAAACTCTTT
AAAAAACATTTCAAATTCGTCGGCGGCGAAATCGTCGGCGAATTTCTGATGAGTACCGGC
TACCTCAAAGGCGCGCACGCCGAAAGCTGTCCGGTTTACCGTGAAACCCTGAAATACCAC
CCGAAATGGCTCGATGCCATCTGAAAAACCAATGAACAGAAGAACCTTCCTCCTCGGCGC
AGGCGCGTTGCTGCTTACCGCCTGCGGCAGAAAATCCGCCCGAACCCACGCCAAAATTCC
CGAAGGAAGCACCGTACTTGCCTTGGGCGATTCGCTTACCTTCGGCTACGGCGCAAACCC
TGGCGAATCCTACCCCGCGCAACTGCAAAAACTGACGGGTTGGAATATTGTCAACGGCGG
CGTATCGGGCGATACATCTGCCCAAGCCCTGTCGCGCCTGCCCGCGCTGTTGGCACGCAA
ACCCAAGCTTGTGATTGTCGGCATAGGCGGCAACGACTTTCTGCGCAAAGTTCCCAAGGA
GCAGACCCGCGCCAATATCGCGAAAATCATCGAAACCGTGCAGAAGGAAAACATCCCCGC
~~CGTCCTCGTCGGCGTGCCGCACATCACACTGGGTGCGTTGTTCGGGCATTTGAGGGATGA~~
TCCGCTGTATGAGGATTTGTCCGAGGAATACGGCATTCCGCTGTTCGGCGGCGCGTGGGC
GGAAATTTTGGGCGATAATAATCTGAAATCCGACCAAATCCACGCCAACGGCAAAGGCTA
TCGGAAATTTGCCGAAGATTTGAATCAATTTTTGAGAAAACAGGGGTTTAGATAAACAAA
GGTTTATCCGCACCCAAGTTGTTTATATAATCATGAACCGACTGGGACACCAAACTGCTT
CGGGACGCATATGCCGTCTGAAGTGCAAAGCCTACGCCATACAGCCGCATGAAGTTGCAG
CGGTATGGCGTTTTTTGAAAAAGACGGCCTGCCGGTTCAGACGGCATGACCGACCGTCCG
AGCCTGCTGTGGATAAAGCCCGGACAGGCTGAAATCATGGAATATTGCGAACCTGAAGAA
GCATCCGACCCGTACGCAACATACAGGCGTGCCAACCTGATGGCGGGTCTGCCGCTGTTT
GTCGTGATTTTGGTTCTGCTCAATATTGTTTTTCCGCTTCCGGCGCATCCCTTAGCTTGG
CTGGTGCCTGCAGGTTTCATGGTTTTGGGCGGCGGCTTTCCCTTATCGCTGCCGCTTGTG
GCGCTGCTTGTCCTGACCTGCTGCATTCTGGCGCATTGTCCGCCATTATCCCGTCTTTTG
TGCTACCCTTGCCCGAATCATCCGATGTCTAAAAATTCTGCCTGATGGCAGCCCTACAAA
CCCGAAGGAGTAGAAATGAAACTGTCCGAACTGTTCAACCCCGACGAATTTGCCGCGCGG
CATTTGAGTTTTGGCGACGAAGCGGCGTTGCTTGCCGCTGTCGGCGAGAAAAGTATGGAC
GATTTTGTCGGCAACACCGTGCCGCAAAGCATCCGTATGCCGTCTGAACTCGATTTGCCC
GATGCCCTGACCGAAGCGGACGCGTTGGCAAAATTGAAAGGCATTGCGTCGAAAAACATG
ATCAACAAATCCTATATCGGTTTAGGCTATTACCCGACCCGCGTGCCGAACGTGATTTTG
CGTAACGTATTGGAAAATCCGGGTTGGTACACCGCCTACACGCCGTATCAGGCGGAAATC
GCGCAGGTCGTTTGGAAGCTTTGTTGAACTTCCAACAAGTGTGTATCGATTTGACCGGTT
TCCCTGTGGCGGGCGCGTCTTTGCTGGACGAAGCGACCGCCGCCGCCGAAGCGATGGCGA
TGGCGCACCGCGTGGGCAAGGTAAAATCCGAGCGTTTCTTTGTGGACGAGCGCGTGTATC
CGCAAACTTTGGACGTGATGAAAACCCGTGCCAAGTATTTCGGCTTCGAGCTGGTGGTCG
GCGATTTTGCCCAAGCCGACGAAGGCGAATACTTCGGCGCGCTGTTCCAATACGTCGGCA
AAGACGGCGACGTGCAAGACTTGCAGGACGTTATCGGCCGTCTGAAAGCCAAAGGCACGA
TTGTCGCCGTTTCCGCCGACATCATGAGCTTGGTTTTGCTGAAACCGCCTGCCGAATTGG

## Appendix A

GTGCGGATATTGCGTTGGGCAACACACAACGCTTCGGCGTGCCGATGGGCTTCGGCGGGC
CGCACGCCGCTTATTTCGCGTTTAAAGACGAGTTCAAACGTTCCGCCCCGGGCCGCATCA
TCGGCGTATCCAAAGACGCATCGGGCAAACCTGCCTTGCGCATGGCTTTGTCCACCCGTG
AGCAACACATCCGCCGCGAAAAAGCTACATCCAATATTTGTACCGCGCAGGCATTGCTGG
CGAATTTGGCGGGTATGTATGCCGTTTACCACGGCCCTGAAGGCGTGAAACGCATTGCCA
ACCGCATTCACGCGCTGGCTTCTGCCTTTGCCGATGCGCTGGTTTCAGACGGCCTGAATG
TGGTTCACAAAGTCTTTTTCGATACTGTTACCATCGATTTTGGCAGTAAAGAGAAAGCAG
ACCAAGTGTTTGCCGCTGCTTTGGCGTCGGGTTACAACCTGCGCCGCGTCAACGATACTC
AAGTTGCGGCTGCATTCCATGAAACATCGGCATACGAAGATTTGGTCGATTTGTACCGCG
CGTTTACCGGCAAGGATACGTTTACATTTGCCGATGATGTCAAAGGCCGTCTGAACGCCG
AATTGCTGCGTCAGGACGACATTCTGCAACATCCTGTGTTCAACAGTTACCACACCGAAC
ACGAAATGTTGCGTTATCTGAAAAAACTCGAAGACCGCGACTTGGCGATGAACCGCAGTA
TGATTTCATTGGGCAGCTGTACTATGAAACTCAACGCGACTGCGGAAATGTTGCCGATTA
CTTGGGCCGAGTTCACCGACATCCATCCTTACGCTCCCGAAGCGCAAACCGCCGGCTACC
GCGAATTGCTCGCCGATATGGAAAACAGCCTGAAAGCCATCACCGGCTTTGACGCGATTT
CCCTGCAACCAAATTCCGGCGCACAAGGCGAATACACCGGTATGCTCGCCATCCGCCGCT
ATCAGGAATCCCAAGGCGAAGCGCACCGCAACATCTGTCTGATTCCAAAATCAGCCCACG
GTACCAACCCCGCCACCGCCGCCATGCTCGGTTTGAAAGTCGTCGTCGTCGACACCGACG
AACACGGCAACGTCAACATTGACGATTTGAAAGCCAAAGCCGAGCAACACCGCGACGCTT
TGTCTGCCATCATGATTACCTATCCGTCCACCCACGGCGTGTACGAAGAAGGCATCCGCG
ACATCTGCCGCATTATTCACGAAAACGGCGGACAGGTTTACATGGACGGTGCGAACCTCA
ACGCCCAAATCGGCATCATGCAGCCTGCCGAAGTCGGTGCGGATGTGTTGCACATGAACC
TGCACAAAACCTTCTGTATCCCTCACGGCGGCGGCGGCCCGGGCATGGGTCCGATTGGCT
TGAAAGCCCATTTGGCTCCGTTTGCCCCGGGCCATACCTTGACCGACACCCACAACGCGG
CTGCCGATCAAACCGCCGTGGCTGCCGCAGCATATGGTTCTGCATCCATCCTGCCGATTA
CTTGGATGTACCTGACCATGATGGGCAAACAAGGCATGGAACAGGCAACGCGCTGGGCAT
TGCTCAACGCCAACTACGTCGCCAAAGCCTTGGGCGAGGATTATCCGATTCTCTACACCG
GCAAAAACGGCCGCGTCGCGCACGAATGTATCGTCGACTTGCGTCCGCTCAAAGCCGAAA
GCGGCATTACCGAAACCGACATCGCCAAACGCCTGATGGACTACGGCTTCCACGCCCCGA
CCGTCTCCTTCCCCGTTGCCGGCACGCTGATGATCGAACCGACCGAAAGCGAGAGCAAAG
CCGAACTCGACCGCTTCATCGCCGCCCTGAAACAAATCAAACAGGAAGTGCTGAAAGTCG
GGCGCGGCGAATGGCCGAAAGACGACAACCCACTGGTCAACGCGCCGCACACCGCCGCAG
ATATAACCGGCAACTGGGCGCATCCGTATTCCCGCGAAGAAGCCGTCTTCCCGTTGCCGT
TCGTCCGCGAACACAAGTTTTGGCCCTTCGTCAACCGCGTGGACGACGTGTACGGCGACC
GCAATCTCGTGTGCAGCTGCCCACCGATGGAAAATTATGAAGACTGACTGTTGATATCTT
AAAAAATGCCGTCTGAAACATTTTCAGACGGCATTTTCATCAACGGCAAACCAGTTGCAC
CAATACACGTATCTCGACTATAACTTTAAAACAAATGAGTTAAACCAGTATCCATACATC
AGCTTTTTTATCATCCTACTTTTTATTCATCCGATCGTGCAAACAGATTTCAAAGATGAA
AAGCCTATTCACACCCTTTGATGTCATTTCCACACGGACAAACAAATATAGTGGATTAAC
AAAAACCAGTACAGCGTTGCCTCGCCTTAGCTCAAAGAGAACGATTCTCTAAGGTGCTCA
AGCACCAAGTGAATCGGTTCCGTACTATTTGTACTGTCTACGGCTTCGTCGCCTTGTCCT
GATTTTTGTTAATTCACTATAAATTCCCATAAAAAAACGGAGCAGATACCTGCCCCGTTT
TTATTTAATCCGAAATTTTAATCTAAATTTAGAATTTTGCACCGGATTGGTTTGCCATAT
AGTCAACAGCCGCTTTGACTTCGTCATCGCTCAAACCTGCATTGCCGCCTTTGGCAGGCA
TCGCGTTAAAGCCTTCAAGGGCGTGTTTGTGCAAGGTTTCTTTGCCTTTTTTGATACGCG
GTGCCCAATCGTCTTTTTTGCCTATGCCGGGAATACCGGGAATCGAACCGCCGTGGCACA
CCTGACAGGTTGCTTCGAAGACTTTTTTACCGTCAACGCCGACCGCAGGGGCTGCCGCAC
CCTTGTCTTCTGCCTTCGCTTCTGCCGGAGCTGCACTATCGGCAGGAGCAGAAGCTGTTC
CTGAAGCGGCATTGTCGGCAGGCGCAGCCTCATCAGGATTCGGGAAAGAACCGCCGCTTT
~~TGTTCGCCATGTAAGTAATCGCCCGTTTAAGTTCCTGATCGGTCAGGTCTGCCGCAGCGC~~
CTTTTGCAGGCATGGCGTTAAAGCCGTTCAGCGCGTGTTGGAACAAGGTATCGAAGCCTT
GCGCGATACGCGGTGCCCAATCGCCGTTGTGTTCCAGTTTCGGAGCGTTCGGCACATTGC
TGTCCGCCGCGTGGCATTGGATACAGATTTTGCCGAAAATCTGTTCGCCTTGGCGTTCGC
CGACGGGGATGCCGTCGCCCATCGTCAATTGTCCGACAGGCTGGATACGGGTCTGCGTTG
CTGCTTCCGTAGTGGCATCGACATCGCCGAACGAGCCGCTGCCCGCCAGCTTAATCAGGA
AATAAAGGACTGCAATAACAATAACGATACCGCTCACAAGGGTAAACAGTGCAGAGCCTT
GGGCTTTGTTGTCGCGGAGTTGTTTCATTTGGTAGGCCTCGCCGTCAGGTTAGGTTGTGC
TGTAAATTATAGTTTGGTGTGTTAAACGCAGTTAACAATATTTTGCTGGATTATACTGAA
TTCACAGGGTCTTTCCAATCGCTATCATTGAAAATATGAAAAAATTTGCCAACGGTATCT
GTATAAAACAAATAATCCTTTGAAAATAATTGTTTATCCTCAAGAAAACTCTCCTTATGC
CGCCATACGCCGCCTGCCGGCGCAAGATAACCTTTGCCAATTTGCAGAATTTACGTTAAC
CTTGCGTTTTCCGCACCCATAGCTCAGTTGGAAGAGTGTCAGTTTCCGAAGCTGGAGGTC
ACAGGTTCGATCCCTGTTGGGTGCGCCAATTATAAAGAGACCGTCTGAAAGATAAATATT
TTTCAGACGGTCTTTTGACTTACTTCAAACTCTTATTTCAAGACTTCCGCAAATGCGCGG
GCAACATAGTCGGTATTCGACGTATTCAGTCCGGCGACGCACATCCTGCCGGAATCCAGC
AGGTAAACGGCAAATTCGTCGCGCAGCCTGCGGACTTGTTCCACGCTCAATCCTGTGTAG
CCGAACATGCCGCGCTGTTTGATGAAATAAGTGAAATCGCGATTGGGGATTTGCGCAGTT
AAGACATCATAAAGTTTCTGCCGCATCGCACGGATGCGGTCGCGCATCATATAAACCTCG
TTTTGCCACAAGGCGTAAAGTTCGGGGCTGTTCATCACGTCGGCGGCGATATACGCGCCG
TGCGCGGGCGGGCTGGAGTAGATGCGGCGGACGGTGAATTTGAGCTGTCCGAACACCAAA
TCCGCTTCTTCCTTATTCGGGCAAACCACGCTTAAGCCGCCGACGCGCTCGCCGTAGAGC
GACAGGTTTTTGAGAAGGAATTGCTGACGAACAAGGGCAATTCCATTTCCACCGCTTTG
CGGACGGCGTAGGCATCGCTGTCCAAATCGCCGCCGAATCCTTGGTAGGCAATGTCCATA
AACGGAATCAGTTTGCGCGTTTTGATGATGTGCAACACTTCGTCCCATTGCCGTTCCGAC
ATATCCACGCCGGTCGGGTTGTGGCAGCAGGGATGGAGGATCAGGACGCTGTTTTCGGGC

## Appendix A

AGGGTGTTGAAAAACGCGGTCATTTCGTCGAATTTCACGCCGACAGTGGCAGGGTCGTAA
TATGGGTAAGTGCCGACCTCGAAACCTGCGCCTTCAAAAATGCCGCGATGGTTGTCCCAA
GTCGGGTCGCTGACGTAGGCGCGCGCTTCGGGAAACCAGCGGTGCAGGAAGTCCGCCCCG
ACTTTGAGCGCGCCCGAGCCGCCCAAGGTTTGTACGGTAACGATGCGCCCTTGCGCAAGC
GCGGGATTGTCTTTGCCGAACAATAAATGCTGCACCGCGCTGCGGTAAGTGTCCAAGCCT
TCCATCGGCAGGTAGGGCGACGGCGCAGGCGCGGCGGCACGGGCGGTTTCGGCTCGGCTC
ACGGATTCCAACACGGGCATTTTGCCTTCGTCGTCGAAATAAATGCCTATGCTCAAATTG
ACTTTTTCGGGGCGCGGGTCGTTTTTGAAGGTTTCGACCAAACTCAAAATCGGGTCGCCA
GGATAGTATTCGATGTGTCGGTACATAGTCCTTACCTCTTGCTTTTTCAAAGGATTTTCT
TTTTCAACAATACACCACTTTCGATATGGTGCGTAAACGGGAATTGGTCGAACAGGGCGG
CACGTTCGACCGCATGGGTTTCCGCCAAGGTGTCCAAATTGGCGCGCAACGTTTCGGGAT
TGCAGGAAATGTAGATGATGTTGTCAAACTGCGACACCAGCTTCAAAGTTTCCTCATCGA
TACCGGCACGCGGCGGATCGACGAAAATAGTGGAAAATGCGTAATCCGTCAAAGCAATAC
CGCCATCCTTAAGGCGTTTAAACTCACGTTTTCCGGTATAGGCTTCGGTAAATTCTTCAG
CAGACAGACGGGCGATTTTGATGTTGCCGATGCGGTTGGCTTCGATATTCCATTGCGCCG
CGCTGACGGAGGTTTTGGAGATTTCGGTTGCCAAAACCTGTCGGAAATATCGGGACAGCG
GCAGGGTGAAATTGCCGTTTCCGCAATACAGTTCGAGCAGGTCGCTGCCCAAGCCTTCCG
CCGTGCGGCACGCCCATTCAAGCATTTTCTGACACACGGCGGCATTCGGTTGGGTAAAAC
TGCCTTCAATTTGCCGATAACGGAAATCCCGGTTGCCGACCTTCAAAGTTTCCGTTACAT
AGTCCTGTTTTAAGACTATTTTCTGTCCCCTGCTCCGCCCAATAACGGAAATATCCAACT
GTTGCTGTAACGCTTGCGCCGCCTGCATCCACTCAGCATCAAGCCTTTTGTGGTAAATCA
TGGTAACCAGCATTTCCCCGCTGAGCGTGGACAGAAATTCGACGGCATACCAGCGTTTTT
TGAGTTCGGGGGATTGCGCGGCGGCGGCGATCAGCTCGGGCATGAGGCGGTTGACAGCCT
CGGAAGCTGCTTCAAAACGGTCGCAGCGTATCATGCTTGCGCCGCTGGCTTTCTGCCCTT
TTTCAAACATGGCATAAAACATTTCCCCGCCTTCGTGCCAAATACGGAACTCGGCACGCA
TACGGTAATGTTTGTCCGGAGATTCGTACACTTCCCACTCAGGAACATCCAAACCTGCAA
AAAGGGTTTTAAGGTAGTCTTTTTTACCTTGAAGCTGCTGCGTGTAGTCATTCATATCGC
TATCCTGAAAAATCAGACCGGATTATAAGCCGATTTGCCTCGCCGCACGCAAACTCTTTG
ACTGCCGCCCCTTCAATGACGGACGGGCTTTTGTGCTAAAATCCGCCATCTTTCCACACT
ATACCGATAAAGGGAAAAATCATGGCAGGCAACACTTTCGGACAACTCTTCACCGTTACC
ACCTTCGGCGAAAGCCACGGCGCGGGTTTGGGCTGTATCATCGACGGCTGCCCGCCCGGC
TTGGAATTAAGCGAAGCGGATATCCAATTTGACCTCGACCGACGCAAACCCGGCACCAGC
CGCCACGTTACCCAACGCCGCGAAGCCGACCAAGTCGAAATCCTCTCCGGCGTATTCGAA
GGCAAAACCACCGGCACGCCCATCGCCCTCTTAATCCGCAATACCGACCAGCGCAGCAAA
GACTACGGCAACATCGCCACCAGCTTCCGCCCCGGCCACGCCGACTATACCTATTGGCAC
AAATACGGCACGCGCGACTACCGCGGCGGCGGCAGGAGCTCCGCCCGTGAAACCGCCGCC
CGCGTTGCTGCCGGAGCCGTTGCCAAAAAATGGTTGAAAGAAAAATTCGGCACGGAAATC
ACCGCCTACGTTACCCAAGTCGGCGAAAAAGAAATCCGGTTTGAAGGCTGCGAACACATT
TCCCAAAATCCTTTTTTTGCCGCCAACCATAGCCAAATTGCCGAGCTGGAAAACTATATG
GACAGCGTGCGCAAATCCTTGGATTCCGTCGGCGCGAAGCTGCATATCGAAGCAGCCAAT
GTCCCTGTCGGCTTGGGCGAACCTGTTTTTGACCGCCTCGATGCCGAAATCGCCTACGCG
ATGATGGGCATCAACGCCGTCAAAGGCGTGGAAATCGGCGCAGGTTTTGACAGCGTAACG
CAACGCGGCAGCGAACACGGCGACGAACTGACCCCGCAAGGCTTCCTGTCCAACCACTCA
GGCGGCATCCTCGGCGGCATCAGCACCGGGCAAGACATCCGCGTCAATATCGCCATCAAA
CCCACCAGCTCCATCGCCACCCCGCGCCGCAGTATCGACATCAACGGCAACCCCATCGAA
CTCGCCACGCACGGCAGGCACGACCCCTGCGTCGGACTGCGCTCCGCGCCGATCGCCGAA
GCCATGCTCGCGTTAGTCCTCATCGACCACGCCCTGCGCCATCGCGCGCAAAATGCCGAC
GTTCAGGTTAATACGCCCGACATTACCCTTTCAAACAAATAAAAATTTAGCCAAAACACA
GACTTTATAATAGAATATCGAGCATTGCCGTGCAGCCTTGACGCACGGGTTTGTTTAGAG
~~GAATACAAGGGAAATGACACAAGAAACCGCTTTGGGCGCGGCACTAAAATCCGCCGTCCA~~
AACTATGAGCAAAAAGAAACAGACCGAAATGATCGCCGACCACATCTACGGCAAATACGA
TGTATTCAAACGCTTCAAACCGTTGGCGCTCGGCATCGATCAGGATTTGATTGCCGCTTT
GCCGCAATACGATGCCGCACTGATTGCACGCGTCCTCGCCAACCACTGCCGCCGTCCGCG
CTATCTGAAAGCCTTGGCGCGCGGAGGCAAACGTTTCGATTTGAACAACCGTTTCAAAGG
CGAAGTTACCCCCGAAGAACAGGCGATCGCGCAAAACCATCCTTTTGTGCAGCAGGCTTT
GCAACAGCAGTCCGCCCAAGCTGCCGCCGAAACGCTGTCTGTTGAAGCCGAAGCAGCCGA
ATCTTCCGCAGCAGAATAAATCCCCAAACGAAATGCCGTCTGAAAACCGATTTGGTTTCA
GACGGCATTTTTTCGTATGCGGCAATCACGGTTCAAATATCCAATTCCGCCGTATCGCCT
TCGCGTTCCATCCAAGCGCGGCGGGCGGCGGCTTCGCCTTTGCCCATCAGTTTGACGAAG
ATGTCGCGCGTCTCGTCATCTGCACCTTCTGGGATTTGTACCTGCAACAGGCGGCGGGTG
TCGGGGTGCATGGTAGTATCTTTGAGCTGGTCGGGGTTCATCTCGCCCAAGCCTTTGAAA
CGGCTGATGGAATAGGCGGTTTCTTTAACGCCTTCTTTTTGCAGCCGCTCCAAAATGCTG
TCGAGTTCGTTTTGGTCGAGGGCGTAGAATTTGCGGGCAGGTTTGCTCTTACCTTGTGCG
TTGACATCGACGCGGAACAGTGGCGGCTGGGCGACGTAGATGTGTCCGTCGGCAACCAGT
TTCGGGAAGTGGCGGTAGAACAGGGTCAGCAGCAAAACTTGAATATGCGAGCCGTCCACG
TCGGCATCGGACAGGATGGCGATTTTGCCGTAGCGCAGGCCGCTTAAATCGGGATGGTCG
TTAATACCGTGCGGATCGACGCCGATGGCGACGGAAATGTCGTGGATTTCGGCGTTGCCG
AAGAGTTGGTCGGGGTGGACTTCAAAGCTGTTGAGCACTTTGCCGCGCAGGGGCAGGATG
GCTTGGGTGGCTTTGTCGCGGGCGAGTTTGGCTGAGCCGCCGGCGGAATCGCCTTCGACG
AGGAAGAGTTCGTTTTCGCGGATGTCTTCGCTTTCGCAGTCGGTCAGCTTACCGGGCAGG
ACGGCGACGCCGCTGCCTTTTTTCTTTTCAATCTTTTTAACCGAACGCATCCGCGCCTGT
GCTTGGCGGATGGCGAGTTCGGCGATTTTTTTGCCGAAGTCCACGTTTTGGTTCAGCCAC
AATTCCAAAGGGTCGCCCGATACGGTGGCGACGAGTTTCAGCGCGTCGCGGTTGGTCAGC
TTGTCTTTGGTTTGACCTTGGAACTGCGGGTCGAGGACGCGGGCAGAGAGAACGAAGGCG
GTTTTTCCGAACACGTCGTCGCTTTGCACTTTAACGCCGCGCGGCAAGAGGTTGTGCAGA

## Appendix A

TTGATGAAGTTGTTGACTGCGTTGAACACGGCTTGTTTCAAGCCTGCTTCGTGCGTGCCG
CCCAGCGGGGTGGGGATGAGGTTGACGTAGCTTTCGTTGGCGCACGAGCCTTCTTCCAGC
CAAGTCAGGGCAAACGCCGCTCCTTCGCCGATGCTGAAATCGCCGTTGTGTTCGTCTGAA
ATGTAGTTTTCGCAAGAGAACAGCGGTACGGCTTCCTGCGCGTCGGCAATCAGGTCGGTC
AGATAGCTTTTCAGGCCGTCGGGGTAATGCCAGGTTTGGGTGTGCGCTTCGTCTTCGCCT
TTGACCGGACGGGTCAGGGAAACGCGCACACCCGGCAGCAGCACGGCTTTGGCACGCAGC
AGGCGTTCGAGTTCGGGAATGCTGTAATTCGGGCTTTCAAAATATTTGCCGTCCGGCCAG
ACGCGCACTTCTGTACCGCTGTCTTTGACGGCGCATTTGCCCACTTGTGCCAACGGTTCG
ACCACGTCGCCGCCGGCAAACACGATGCGGTGGATTTTGCCTTCGCGTTTGACCGTTACT
TCAAGGCGGGTGGAAAGGGCGTTGGTGACGGATACGCCCACGCCGTGCAGGCCGCCTGAA
AAGGCATACGCGCTGCCTCCGTCTTTTTTGTTGAACTTGCCGCCTGCGTGCAGACGGGTG
AATACGAGTTCGACTACGGATACGCCTTCTTCGGGATGCAGGCCGACGGGAATGCCGCGC
CCATTGTCGTGCACGGAAAGCGAACCGTCTTCATGAATTTGCACGTCGATTGCAGTCGCG
AAACCGCCCAACGCTTCATCCGACGCGTTGTCGATGACTTCTTGGCAGATATGGGTCGGG
CTGTCGGTGCGGGTGTACATACCGGGACGTTCTTTGACCGGCTCCAAGCCTTTGAGGACG
GTGATGCTGGATTCGCTGTATTGGTTGTTTTTAGCCATGGGAATAATCTGAAAGTAAGAA
AAACAACGCTTTCAGACGGCCTGAAAGCGTTGCGTTCCGTTGTTTTAGCGGTTGTCGGAA
GATTGGCGGGCGGCAAAGTCTTCATAACTTTCCATACCGCGCAGGAAGCGGGAAGAGAGT
TCTTTCAACGCCCCCAAATAAACGTCGCGTTTGAAATCAATCACTTCGTCAACGGGTGCC
CAATATTGATGCCAACGCCAGCCGTCAAATTCGGGGTGGCGGGTGGCGCGCAGGTTGACA
TCGCAATCTCGGCCGGTCAGGCGCAGGAGATACCAAATCTGCTTCTGTCCGCGATAAGAG
CCGCGCCATTCGCGGCGTACCCAGTTGTTCGGCACGTCATAACGCAGCCAGTCGCGCGTG
CGGCCGATAATTTTGACGTGTTGCGGCAAAAGCCCGACTTCTTCGTACAACTCGCGGTAC
ATGGCGGTTTCGGGGCTTTCGCCCGGCTTGATGCCGCCTTGCGGAAACTGCCAAGAATGT
TCGCGCACGCGCTTACCCCAAAAGACTTCGTTGCGGTTGTTGATTAAGATGATACCGACA
TTGGGGCGATAGCCTTCCCTGTCCAACACGGTGTCGCCCTCCGTTAAATTCAATCTTGGG
ATTTTCCCACAAATCAGGCGGTTTTGACAAATCAGACGGCATGGCGGTACGCGTGCCGAA
ACACGGGGGGATTTGGGAAAATATCTTAAATTTGGTTTACAATAATGTATTTCAAATTAT
TCGGGAATCAGACCATGTTAGATATCCAATTGCTCCGCAGCAACACCGCCGCCGTTGCCG
AACGGCTTGCACGGCGCGGTTATGACTTTGATACCGCACGTTTTGACACACTGGAAGAAC
GACGCAAGTCCGTTCAGGTGAAAACCGAAGAATTACAGGCCTCGCGCAACAGCATTTCCA
AACAAATCGGCGCACTGAAAGGTCAGGGCAAACACGAAGAAGCGCAGGCGGCCATGAATC
AGGTTGCCCAAATCAAAACCGATTTGGAACAGGCTGCCGCCGATTTGGATGCCGTTCAAA
AAGAATTGGACGCATGGTTGTTGAGCATACCTAACCTGCCGCACGAAAGCGTACCTGCCG
GTAAAGACGAAACCGAAAACGTCGAAGTCCGCAAAGTCGGCACCCCGCGCGAATTTGACT
TTGAAATCAAAGACCATGTCGATTTGGGCGAACCTTTGGGTTTGGATTTTGAAGGCGGTG
CAAAACTCTCCGGCGCACGATTTACCGTGATGCGCGGACAAATCGCCCGTCTGCACCGCG
CCTTGGCACAGTTCATGCTGGATACGCACACGCTGCAACACGGCTACACCGAGCATTACA
CGCCTTATATCGTTGACGATACGACGCTGCAAGGTACGGGCCAACTACCAAAATTTGCGG
AAGATCTGTTCCACGTTACCCGTGGCGGCGACGAAACCAAAACCACCCAATACCTGATTC
CGACAGCCGAAGTTACCCTGACCAATACCGTTGCCGACAGCATTATCCCGTCCGAACAAC
TGCCGCTGAAGCTGACCGCGCATTCGCCCTGTTTCCGCAGCGAGGCGGGTTCGTACGGCA
AAGACACGCGCGGTCTGATTCGCCAGCACCAGTTCGACAAAGTGGAAATGGTTCAAATCG
TTCATCCCGAAAAATCATACGAAACGCTGGAAGAAATGGTCGGCCATGCCGAAAACATCC
TGAAGGCTTTGGAACTGCCCTACCGCGTGATTACCCTGTGTACCGGCGACATGGGCTTCG
GCGCGGCAAAAACGTATGACTTGGAAGTTTGGGTGCCGGCGCAAAATACCTACCGCGAAA
TCTCAAGCTGCTCCAACTGCGAAGATTTCCAAGCCCGCCGCCTGAAGGCGCGTTTCAAAG
ACGAAAACGGCAAAAACCGCTTGGTACATACTTTGAACGGCTCCGGCTTGGCTGTCGGCA
GAACGCTGGTCGCCGTATTGGAAAACCATCAAAACGCCGACGGCAGCATCAACATCCCTG
CCGCACTGCAACCGTATATGGGCGGTGTTGCCAAGTTGGAAGTCAAATAAGTTTGCAGGC
TGCCTGAACGTCAAATGCCGTCTGAAACCTGTTTCAGACGGCATTTCCTTTAAACTTTTA
AAACACGTCAGCCGTCGGCACGAACCGCATTGCCGCAATCGCCGGTCTGTCCGACCTCGC
GGATATTGGACAGCGTAACTTCCGAAATATTACCCAACGCCTCTTCCGTCAAAAATGCCT
GATGGCCGGTAAACAGCACATTATGACAAGACGACAGGCGGCGGAACACGTCGTCGGTAA
TCACATCGTTGGATTTGTCTTCAAAAAACAGCTCGCGCTCGTTCTCGTACACATCCATGC
CCAATGCGCCGATTTTCCGGCGTTTCAACGCCTCAATCGCGGCGGCACTGTCAATCAGCC
CGCCCCGGCTGGTGTTGATAATCATCACGCCGTCTTTCATTTTGTCGAACGCCGCTTCGT
TCAGCATATAGTGGTTTTCCGGCGTGGCGGGGCAATGCAGCGTGATGATGTCCGACCGGG
CATACAGCTCGTCTAAATCCACATATTTGCCGCCGATTTTTTCCGCTTCGGGGTTGCAAA
ACGGATCGTAAGCCAGCAGGTTCATGCCGAAACCCTTTAAAATCCGCATGGTTGCAATAC
CGATTTTCCCCGTGCCGATAACGCCCGCCGTTTTGCCGTACATATTGAAACCGGTCAGAC
CTTCCAGCGAAAAATTCGCATCGCGGGTACGCTGATAGGCTTTGTGGATACGGCGGTTCA
ACGTCAGCATCAGACCGACCGTATGTTCCGCAACCGATTCGGGCGAATAGGCAGGCACGC
GCACGACTTTCAAGCCCAACTCTTCAGCCGCCTTTAAATCCACATTATTGAAGCCGGCAC
AACGCAACGCCACAGTTTTCACGCCAATTTGCGCCAATTTTTCCAACACGGGCCGGCTGC
CGTCGTCGTTTACAAAAATACAGACCGCTTCCGCGCCTTCCGCCATTTTCGCCGTTTTCG
CATCCAGCATAAAATCAAAAAACTCCAGCTCGAAGCCGAAATGCCGGTTGGCGCGGGTAA
AATGTTCGCGGTCATAGCTTTTCGTACCGTAAATCGCAATCTTCATCAATATGTCCAGTT
GTCGTCTATGGTTGAGAAACGGCATAATACACTGAATTCAAACAAATCAGTAGAATATGG
TGGATTAAAATTGATTGCATGCACGGCATTTCCATTTCAAAACACAAAACTCAATCGCCC
ATTGCCGCCAGAAGCTCGGCCTGATGCTCGGCAATCAGGGCATTGGTGATTTCTTCCAAG
TCGCCGTCCATCACAAAATCCAGCTTGTGCAGGGTAAGGTTGATGCGGTGGTCGGTTACG
CGGCCTTGGGGATAGTTGTAGGTGCGGATGCGTTCGCTGCGGTCGCCGCTGCCGATGAGG
GATTTGCGCTCGGCGGCTTCTTTGGCTTGGGCTTCGCGTTTTTGCGCGTCGTTCAGGCGG
GCGGCGAGGACTTTCATTGCCTGCGCTTTGTTGGCATGTTGGCTGCGGCCGTCTTGGCAT

# Appendix A

TCGACCACCATGCCGGTGGGCAGGTGGGTGATGCGGACGGCGGAGTCGGTTTTGTTGATG
TGCTGACCGCCCGCGCCGGATGCGCGGAAGGTGTCGATGCGCAGGTCGGCTGGGTTCAGT
TCGATGTCTTCCAGTTCGTCCGCTTCGGGCATGACGGCAACGGTGCAGGCGGAGGTGTGG
ATGCGGCCTTGGCTTTCGGTGGCGGGGACGCGCTGCACGCGGTGTCCGCCCGATTCAAAT
TTCAAACGGCTGTACGCCCCGAGTCCGACAATACGGGCGATGACTTCTTTATAGCCGCCC
AATTCGCTTTCGTTGGCGGACACGATTTCAACCTGCCAACGGTTGCGCTCGGCGTAGCGG
CTGTACATACGCAGCAAATCGCCGGCAAACAGCGCGGCTTCGTCGCCGCCCGTTCCGGCG
CGTATTTCGATGAAGATGTTTTTGTCGTCGTCGGCATCTTTGGGCAGCAGCAGTTTTTGC
AGTTCGGTATCGAGTTCGCCGATTTTGGCTTTGGCCGCTTCGATTTCTTCGGCGGCAAAG
TCTTTCATTTCGGGGTCGGACAACATTTCTTCGGCATCCGCCAAGTCGCTTTGGGCAAGC
CGATAGTTTTGGAACACTTCGACGACGGGGGTCAGTTCGGCGTGTTCGCGCGTGAGCTTG
CGGTAGTTGTCCATGTCGGACGTGGCTTCGGGCTGTCCGAGAAGGTGGGTAACTTCTTCC
AGTCGGTCGCTGAGTTGTTGTAGTTTTTCTAAGATAGACGGCTTCATAATTCTTCCATAA
CAAACGCCGCCTGAATGTTCAGACGGCATCAACACTGGATTATTATAATAGGTTTTCCGG
ATATTCAAAAAGATAATCTTAGATGGATAACCTACCGTCCCAACAGGGCATCGGCATTGC
GCTCCGTTACCTTTGCAATCTCTTCTACACAGGTTCCGCGGATTTCCGCAGCAATCTTTG
CAATACCCGGAATATTGGCAGGCGTATTAATCTCTTTTTTCAGCATAAACGGGCTATCCG
TTTCCAATACGAAATCCCCGTCGTTCAAGGCTTTAAGCGTATCGCGCACTTTACGCGCGT
TCGGATTGAGCAGCAGCGAACCGATGCCGATTTTGAAACCCAGTTTCGTCAACACACGCG
CTTCTTCCGCGCTGCCGGAGAAGGCGTGAACGATGCCGCCTTGGGCAAAGCCTGTCTGTT
TGACGGCGGCGGCGATGTCGGCGGTGGCTTTGAGATTATGGATAATCACGCGGCGGCGCA
GGGTTTGCGCAATTTCAAGCTGGCGGACGAAAACTTGAATTTGCCGTTCGCGCTGCTGCG
ACGTTTGGGTTTTATCGTAAAAATCCAAGCCGATTTCGCCGACCCATGCCTGCGGATAAT
GTGCCAACATCGTTTCCAGGCGGACGAAATCCCGCTCGGCAATGCCGTCTGAAAACCAAG
GATGAATGCCCAGTGCAATACGGATTTGACCGTGTTCGGACGGCATTTCCGCCAAATCCG
CCACGTCCTGCCAATCCTGCGGGCGCGTCGCGGGAACGATAAACCGCTTCACCCCAACTT
TCCGCGCTGCGGTCAGGATGTGCGGCAGGTTTTCGCGCAGGGCGGGATCAGCGAGATGGC
AGTGGGTGTCGGTGAAGTTCATTTCGATTTCACACTAACTTTAGTCTTACCAATTCTTTG
TAAACATCTTCCTTACCCCAGCCTTGCGATACGGCGAGGGTCATCAGCGCGGTGGCGGTT
TCGAGGTTGCATTTGCCGCCGTTGATGATGCCCGAGTTGCGGAACGCGTTGCCTTGCGCG
TAAACGGCGGCGGTTTTGCCTTGTCGGACTTGGCTGATGTTGAGCAGCAGTTTGCCCTGC
CGCGCGAAGTCTCGGACGGCGCGGATAAAACCTTCGTCTGCGGGCGTGTTGCCGTGTCCG
TAGCTTTGCAGGATAAGAGCTTGGGCGGGAAGCTGTCCGAGTCCGTCCGCAAGTTCTTGG
ACGGCAAAGCCGGGGATAAGCGTGCGGACAGCGATTTTTGCCTGCGGGTCGGGATAACGG
ATTTTGAGGCCGTCTGAAACGGCTGCTGCGTCTTGGGACGGGAGGCGAAGATTGTGCCAA
CCCCGGGTTTCGTCCCATTCGGCAAGCGTGCCGAAATGCGGATTGTCGAAGCCTGCAGCA
GTTTCGGTGCTGACTTTGCTGCTGCCGACGGCGGGATACAGTTTGCCGTCAAACGCGATG
ACGGTTTGTTTGAGCTTGAGGCTGAAGGCGGCAACGGCGGTGGAGAGGTTGCGCGGGGCA
TCGCTGTTTTCGGCGGCGTAAGGCCATTGGGAACCAGTCAGGACAATCGGTTTGCCCAAA
CCTTGCAGAGCGAGCGCGAGGAGATTGGCGGTGTACGCCATGCTGTCCGTGCCGTGCAGT
ATCAGGATGCCGTCGCATGAAGGGAGTTTGTCGGCAATGATGTCCAGCCAATCGCGCCAG
TGTTGCAGCGTAACGGAGGAGGAATCAATCAAGGGATTGCAGACGTGCCACTCGAAATCG
AGGCCGTCTGAAAAGGGGGAAAGGGCTTGGCTAACCAGTGCGGTATCGGGGCGCAGGCCT
TCGCTGCTTTGGGTCATGCCTATGGTGCCGCCTGTGTAGAGGACGAAGATTTTTTGTTTC
ATGGACATCATCGGGTCGTCTGAAAATAATAATACGGCTTATTTAACTATATTTCGGACA
GACTGGCAATTTGGCGGCGCGGACGGTTTTCAGACGGCCTTCAAATGAAAAAGCACCCGA
GGGCTGTCGATATTTGATTTTCCAAGTAGATTTTTATTCACGAAATAGGAGAGCCGCAAC
AAGCTTAAATCCCTTGTGAGGTTCCCAACACGGAAGATACCGCTTTGTGGATTAAAAAAT
ACGGAAACTATTGAATATCGACAACCTATTTAGGTGCTTGATTTTATTGTTTGCTTTGCG
~~CGGCTTTTTTGGCTGCCTTGGCGGCTTTGCGTTGCGCCGGCTTTTCTTTCAATTTGGTGG~~
GGTAAAACTGGATACGTTGGCGTTTTTTCCACCAAATCCAAGCGACAACGGTCGCACCTA
TACCCAAGATAACAAAAATACCCGATTGCAGGCTGTGCATTTTCGCCATCAGCCAATCGA
TGTTGTGCGCACCGTATTCGCCCAGATAAATCCAAATAGGGACGGAAATCAGTGCGGCCA
GTCCATCCATAATGATAAAACGCAAGTATGAAACCTTGCGGCTGATACCGGCTGTAACAA
ATACGGCCGTTCTCAAACCGGGCAGGAAACGGGCGACAAATAAGACCCAGTTACCGTATT
TGTCGAATTTTTCCTGAACCTGCTCATAACGTTTCGGCGTCATGATGCGCGCAATAGGTT
TGAACCTTAGGATTTTCTGCCCCCAAATTCGTCCGGCGGCGAACATGATGCCGTCCCCGA
CCAATACGCCGAGCATACCGACTGCAAACATAATATGCGGATTGGTATAACCCATACCCG
AAATCACGCCGCCTGTTACCAAGGTCAAATCCTCGGGAATCGGCACGCCGAAACCGCAGA
TGACCAATACAAAAAAAACAGCCGCATAACCGTATTCGACAAAAAAGGCTTCTAAAAAAG
CAAACATGGCGGATATTCCATTGTCGGAGATAAAAAGTCAGAACAAACCGAAACATTTTC
TACATGAAGCAGGCATTCTATCAAAGATTATGCCGTCTGAAAGCGGAAAAAAGGCAGATT
CGCGCATCCTCCGCATGTTCAGACGGCATTTAAACAGAAAACCGGCCGGTTCTAAAAACG
GTTTTGCCTGATTTTGCCTAAATGCCGCCGATGGCGGCGGCAATGCGTTCCGCCCCTTCG
CGCGCCCAATCCGCCTGCCGCGCCTCCACCATCACGCGCACGACGGGTTCGGTTCCCGAA
GCGCGCAACACGACACGCCCTTTGCCTTCGAGTTCTTTTTCCACTTCCGCCAACACGTCT
TTCGAAGCTTCCTGCCATTGCTGACCTTTTTGGATGCGCACGTTAATCATCGTTTGCGGA
TACGGCTGCCAATCGGCGCAAACGGTGGCGAGGTCTTGGTTCAGCGTTTGCAGTGCCGCC
AAAACTTGCAGCGCGGAAATAATGCCGTCGCCGGTGTTGTGTTTGTCCATACACAAAATA
TGGCCGCTGGCTTCGCCGCCGATGAGCCAGCCGCGTTGGTTCAGCTGTTCCAACACATAG
CGGTCGCCGACTTTGGCGCGGCAGAAATCCACGCCCTGCTCTTTCAGGGCGATTTCCATC
GCCATATTGGTCATGACCGTGCCGACCACGCCGCCGATGTTGATACCTTCTCGGGCGCGG
GCTTTGGCAATGACGTAAATCAGGCTGTCGCCGTCGTAAACCTGCCCGTTTTTATCGACC
ATCATCAGGCGGTCGCCGTCGCCGTCTAAGGCGATGCCGTAGTCGGCTTCATGCTGTAAA
ACGGCGGCCTGGAGTGTCTTGGTATAAGTCGCACCGCATTTTTCGTTGATGTTGTAGCCG

## Appendix A

TTGGGTTCGTTGCCGATGCTGACGACCTGTGCGCCCAGTTCGTGAAACACCTTGGGGGCG
ACACCGTACCCCGCGCCGTTGGCGGTATCGACAACCAACTTCAAACCCCGAAGGTCGGAA
TGGCTGGGAAAGGTGGATTTGCAAAATTCGATATAGCGGTCGTCCGCACCGCTGATGCGG
CGTGCGCGACCGAGACGGGCGGACGGTTGGGTTTTCATTTCGCCGTCGATTTTGGCTTCG
ATTTCCAACTCGACTTCATCGGAAAGTTTCACGCCGCCTTCGGCGAAGAATTTGATGCCG
TTGTCGGAATAGGCGTTGTGCGACGCGGAAATCATCACGCCGGCGGACAGGCGCAACGCG
CGGGTCAGATAAGCCACGCCGGGCGTGGGCAGCGGTCCGGTCTGTACCACATTCACACCC
GCCGCCGTAAAACCGGCCACCAAAGCGGCTTCCAGCATATAGCCGGAAATGCGCGTGTCT
TTGCCGATGAGGACGGTCGGTTTCTGGTCGGTGTCGTGCTGCACCAAAACCTGCCCCGCC
GCATAGCCGAGTTTCAATACGAAATCGGGCGTAATCGGAAATTGCCCCACTTCGCCGCGC
ACGCCGTCCGTGCCGAAATATTTTTTTGCCATGTGTTGCTCCGAGAATGTGAACCGTTGT
CCGAGATTATACAGTCAGTTTGTGCCTTGCTGTCTGCACCGTTGATGCCGTCTGAAACCG
CCCCGTCCTTTTCAGACGGCATGAAGTATGTGAACCGCTGTTTACAGATTGATGCCCAAC
GCTTCCCACACCTTCAACGCATCCGCTGTCGCCTTCACATCATGCACCCGCACGATTTGC
GCGCCGCGCGCTACGGAAGCCAACGCTGCCGCCACGCTGCCGTGTACGCGTTCCGCCGCA
TTTGCCTCGCCGGTCAGCTCGCCTATCGTGCTTTTGCGCGATACGCCGATGAGCAGCGGA
AAACCTGTTTCCGCCATCAATTCGGGCAAATGCCGCATCAGCGCGATATTGTGTTGCAAG
GGTTTGCCGAAGCCGGAGCCGAAGCCGGGGTCGAGTATGATGCGTTGCGGTGCGATGCCT
GCCGCGATACATTCCGCTGAGCGCGCTTTCAAATACCGCGCTACTTCACCGACAACATCT
TGATATTTCGGATTAATCTGCATGGTTTTGGGCAAACCCTGCATGTGCATCAGGCAAATG
CCCGTGTCCGCCTGACGCGCCAGCAATTCGACCGCGCCCTCGTCATTCAACGCCGCCACA
TCATTAATAATATCGATGCCGCCGAGTGCCAACGCTTTTTCCATAATCACCGTGCGGCGC
GTGTCCAAACTGATGGGAACGCCCCACCCCGCCACTTCCGCCAAAACAGGCTCAACCCGC
GCCCATTCTTCTTCAGGCGAAACATAATCCGCACCCGACCGCGTCGATTCGCCGCCGATG
TCGAGAATGTCTGCGCCTTCTTTTAGAAGCTGTTCGGCATGTGCCAAGGCTGTTTGGGCG
TTTTGCGAATACACGCCGCCGTCGGAAAAAGAATCGGGTGTGAGATTCACGATGCCCATG
ATTTTCGGTTTGTCCAAACCGATTTCAAACCGTCCTGCCTGCCAAACGTGTCGTGCCATC
TGAACTCCTCCCAAAATAAAAAACAGATTATATGCCGTCTGAAACCGTCTTGTGCGCTTC
AGACGGCACCGCTATTCGGGCGGCAGACGGCATGTTGTCCGAATGTCTGCTCCGCCTTTG
AATCTGCCGGTATGCCTGCTATCCGCCCGACTTTTCAAAACAGGTTCCGACGATTCCGCA
CGCGCCTGCCGCCTTTGCCAAGCCGTACAGGATTTCCTGCGGCATATCGCGGTTCCATAA
TCCCGTAATATTCGCAATCACGGGCAGATGGCTGATTTGGCGGACTTTCACGATGGATTC
GACATCCAAACGGTAGGGATGGCCTTTGGTATGGTTCAATACGCCCGACTCGCCCAGAAT
CAGGTGGCGGTTGCCGCGCGAGACGACATATTCTGCGGCATTCAACCAATCTTCGGCAGA
ATGGTGCTTGTCTTTACACAGCACCAAGGGAATGTTCAGGTTTCCGGCTTCATTCAATAC
GGCAAGATCGGACATCAGCCCGCCGCCCAAATACAGGATGTCCGCCCCCGCATTCAAAGC
CGCTTCGACATGGCGGACGTTGCGGACGCGCACCAATACGGGTTTCCCTGCATCATGCGC
CGATGCGGTCTGTTCCGCCAACCGTCTGCACCGTCCCCGCCCTTCATCCGCACTTGAAGT
GTCGTATAAGTTTGCCGAAGTGAAAAACGGATCCAGAAACACTGCATCCGCATTGCGCCA
TACTGACGGTTCTGCGGCGATACGGACGGTTTCCCCGCCGCCGAAAGCCACGCCTTTGGC
GGCAACGCGGCTGTCTTCCGCCCGATTTTCCCGACTGACGGTTTTCCATGTATCCAAAAT
GCGGACGGCTTTCTCGACCTCCGGCAGCGTCTGCACCTCCCTGACGCTCAAAACCCTATC
GTCGCCGATTGCGCCGATGACAGTACGCTCGTCGCCGTGAGAAATGTGTTCTCGCAGACC
TCTGCTGCGGATAAAGGCGACAACGCCGGCAATGTCCGCTTCGGCGGCACGCCTGCTCAT
GACAATAATCATATTTCCTCCTGACACAAGAAACGGCCTACCCAAAATAGGATTTTTGCA
AGCCGTGTTATACTGTGGCGTGTTTTACAGATTGTTCGGGCTATGGATTTATTATCGGTT
TTCCACAAATACCGTCTGAAATATGCGGTGGCCGTGCTGACGATACTGCTTTTGGCGGCA
GTCGGGCTGCACGCTTCCGTATATCGCACCTTCACGCCTGAAAACATCCGCAGCCGCCTA
CAACAAAGCATTGCACACACACACCGGAAAATCTCGTTTGATGCGGACATTCAGCGCAGG
~~CTCCTGCCCCGGCCGACCGTCATCCTGAAAAACCTGACCATTACCGAACCCGGCGGCGAC~~
CAGACTGCCGTTTCCGTCCAAGAAACCAAAATCGGATTGAGCTGGAAAAACCTGTGGTCG
GATCAGATACAGATTGAAAAATGGGTGGTTTCGAGTGCGGAACTTGCCCTGACGCGCGAC
GGGAAAGGTGTTTGGAACATCCAAGACCTGATCGACAGCCAAAAACGCCAAGCCTCAGTC
AACCGCATTATCGTCGAAAACAGCACCGTCCGCCTCAATTTCCTGCAGGAACAGCTTATC
CTGAAGGAAATCAACCTCAACCTGCAATCCCCCGATTCGTCGGGGCAGCCGTTTGAAAGT
TCGGGCATACTGGTTTGGGGAAAGCTGTCCGTCCCGTGGAAAAGCAGGGGGCTGTTCCTT
TCAAACGGCATCGGCCCGCCCGAAATCTCACCGTTCCATTTTGAAGCTTCCACTTCGCTG
GACGGACACGGCATTACCATTTCCACCACCGGCAGCCCTTCTGTCCGCTTCAACGCCGGC
GGAGCGGATGCCGCCGGCCTCGGCCTGCGTGCAGACACTTCCTTCCGCAACCTCCACCTG
ACCGCCCAAATCCCCGCGCTGGCACTCAGGAACAACAGCATTAAAATTGAAACCGTCAAC
GGCGCATTTACCGCCGGCGGCGAATATGCCCGATGGACGGTTCGTTCAAACTCGACAAA
GCCAACCTGCACTCCGGCATCGCCAACATCGGCAACGCCGAAATCTCCGGCAGCTTCAAA
ACACCGCGCCACCAGACCAACTTCTCCCTCAATTCGCCGCTCGTATGGACGGAAAACAAA
GGGCTGGACGCGCCGCGCCTGTATGTATCGACCCTTCAGGATACCGTCAACCGCCTGCCG
CAACCCCGTTTCATCAGCCGGCTCGACGGTTCGCTGTCCGTACCGAATCTGCAAAATTGG
AATGCCGAATTAAACGGCACATTCGACCGCCAAACCGTTGCCGCGAAATTCAGATACACA
CATGAAGACGCACCGCATCTGGAAGCCGCCGTCGCACTGCAAAAATTGAACCTGACCCCC
TATCTTGACGACGTGCGGCAACAAAACGGCAAAATATTTCCCGACACCCTCGCCAAGCTG
TCCGGCGACATCGAGGCGCACCTGAAAATCGGAAAAGTCCAACTTCCCGGCCTGCAACTG
GACGATATGGAAACCTACCTCCACGCCGACAAAGGCCATATCGCGCTCAGCCGTTTCAAG
TCAGGGCTTTACGGCGGCCATACCGAAGGCGGCATCAGCATCGCCAACACCCGTCCCGCC
ACTTACCGCCTGCAACAGAATGCAAGCAACATCCAAATCCAACCGCTGCTGCAAGACCTG
TTCGGCTTCCACAGCTTCAGCGGCAACGGCGACGCGGTCATCGACCTGACCGCGGGCGGC
GAAACCCGAAAAGAGCTTATCCGCTCGCTTCAGGGCAGCCTGTCGCTAAATATTTCCAAC
GGTGCATGGCACGGTATCGACATGGACAATATCCTGAAAAACGGCATTTCGGGCAAAACT

## Appendix A

GCCGACAATGCCGCACCCAGCACACCCTTCCACCGATTCACGCTCAACAGCGAAATTTCA
GACGGCATCAGCCGCCACATCGATACCGAACTCTTCTCCGACAGCCTCTATGTTACCAGC
AACGGCTATACCAATCTGGATACGCAGGAATTGTCTGAAGATGTCCTTATCCGCAACGCC
GTCCATCCGAAAAACAAACCGATTCCCCTGAAAATCACCGGCACGGTGGACAAACCGTCC
ATTACCGTCGATTACGGCAGGCTGACCGGCGGCATCAATTCGCGCAAAGAGAAACAGAAA
ATCCTCGAAGACACCCTGCTGGAACAATGGCAGTGGCTCAAACCTAAAGAACCGTAAACA
TCCTGCGTACAAAAATGCCGTCTGAAACACCCCCGCGCTTCAGACGGCAGACCGTAAAAC
CTACAACCCCAATTCCTCCCAAATCCCATCAATCTTAGCCGTAACCGCAGGGTCTTTTTT
GATGACGCGTCCCCATTCGCGGTCGGTTTCTCCCGGCCATTTGTTGGTCGCATCCAAACC
CATTTTGCCGCCGAGTCCGCTGACGGGGCTGGCGAAGTCGAGATAATCGATGGGCGTGTT
TTCTACCAAAACAGTGTCGCGCACGGGGTCCATGCGCGTGGTGACCGCCCAGATGACTTC
TTTCCAGTCGCGCACGTTCACATCGTCATCCACCACGATGATGAATTTGGTATACATAAA
CTGGCGCAGGAACGACCAGCAGCCCATCATCACGCGCTTGGCGTGTCCGGCGTACTGTTT
TTTCATGCTCACCACCGCCATGCGGTAGGAGCAGCCTTCGGGCGGCAGGTAGAAATCGGT
GATTTCGGGGAACTGCTTTTGCAAAAGCGGTACGAACACTTCGTTCAACGCCACGCCCAA
AACGGCGGGTTCATCGGGCGGTTTGCCCGTGTAGGTCGAATGGTAAATCGGGTTTTCGCG
CATGGTGATGCGTTCGACCGTAAACACAGGGAAATAATCCTGCTCGTTGTAATAGCCGGT
GTGGTCGCCGTACGGGCCTTCCAACGCGGTTTCGTTCGGATGGATGACGCCTTCCAACAC
GATTTCTGCGCGGGCAGGCACTTGCAAATCGTTGCCGATACATTTCACCAGCTCCGTCCG
CGAACCGCGCAGCAGTCCGGCAAACTGGTATTCGCTCAAGGTATCGGGAACAGGCGTTAC
CGCGCCCAAAATGGTGGCGGGGTCGCAGCCGAGTACGACGGCGACGGGATACGGCGTATC
GGGATTGAGTTTGCGGAACTCCTGATAATCCAACGCGCCGCCGCGATGCGACAGCCAACG
CATAATCAGCTTGTTTTTGCCGATGAGTTGTTGGCGGTAAATGCCGAGATTTTGGCGTTT
TTTGTGCGGCCCGCGCGTGACGGTCAAGCCCCACGTTACCAGCGGCGCAACGTCTTCCGG
CCAGCAATGCTGAATCGGAAGTTGATACAAATCAACGTCTTCGCCTTCCCACACGATTTC
CTGACACGGCGCGTTTTTCACCACGTTCGGCGCCATGCTCCAAATGTCTTTCAGCAGCGG
CAGTTTGGAAAACGCATCTTTGATGCCTTTGGGCGGTTCGGGTTCTTTCAAATACGCCAG
CGTCTGCCCAATTTCACGCAGCTTGGACACGCTGTCCGCGCCCATGCCCATCGCCACACG
TTCGGGCGTGCCGAACAGGTTTGCCAACACGGGATAACCGTAGCGCGTACCGTCGGGCTT
AATCGGGTTTTCAAACAGCAACGCCGGCCCTTCGGCACGCAGCACGCGGTCGGCGATTTC
GGTCATTTCCAAATACGGGGAAATGGGGTGTGCGACGCGCTTGAGTTTGCCCTGCTGCTC
GAGCATGGCGATGAAGTCGCGCAGGTCTTTGTATTTCATATTCATCCTTTTTGTCCTTTT
ATCCTGAACAATCCGATTCGGATACCGCCCCTATCCTTGCCTGTGCCTCGGCATATTCTA
TGCCGTGATAAAAGTCGCGTACCAGCGGATGTTCGCCGCCTTGATGGAGTTGCAACAAAG
GACGTTGACCATCGGGTTGGGTAACGACATTGCAGTGCAGACCGAAGGTGTCGGTTTCAT
AAGGGGGCAGCTGGTTGCAGATCATGCCGAAATAAACAGCGTTTTCAAGGTTGTCGTAAA
AGCGGCTTTGATAGTCGTTAAAACTCTTTTCGCTGACGGATACCCACACGCCATATTCCA
GCGTTTCCTCATGCCCGATAATCGGAATCGGCAGCACCGCGCGGATAAAGCGGTCGGTTT
GGTCGGGATAGCGGATGATGCAGAAATCAGAATCGCATTCTGCTTGATAAGCAATGCGTT
CTTCTTCACTGAGTTGATTATAGGGATCGGGTGCGGTAAAGCCGATTGCGGGCATTTCTT
CGTGGTTTTCGCCGCAGGAAGTGCAAGTGTACATAAGGTTTCAGACTTTCAAAACGAGTT
TGCGGTAAAGCCATTCGCCGGCAAACAGCATCCCCATCAATACATAGGCAATCACGCCGG
TATAAACCGCCCACCAATCATATCGCCCCAACCGTGCCAACAAAGCGGCAAGCGTCCCGT
TGATTATAAAAAATACGCACCAAACCTGCGTTACCCGGCGGGTATAGCGCACGGCTTTTT
CAGGCAGGTCGGGCTGTTGCAGCCGCGCAAGTTTTTCAATCACCGTCTGCCCGGCAAACA
GGCTGCCGCCGAACACCGCCAACATCATCAGATTGACGAGGACGGGATACCAATACATCG
AATCATGCCGCCCGAACACCAATACTGCGGCAAAAAACAGTGTAATAAACAAAGCCGCAT
AACGCTGTTGGGGCAGTTTGGCAGTCAGGGCGCGCAGCAGCCACAACCCGCACATCGCCG
CCGCCAGCCAAACAAACCAGCCCGCCTCCCTGCCGTATATAGTGGATTAACAAAAACCAG
TACAGCGTTGCCTCGCCTTGCCGTACTGTCTGCGGCTTCGTCGCCTTGTGCTGATTTTTG
TTAATCCACTATACCACAAAGCGGGATAGGCAATGCTTAATACGGTCAGAAAAATATGTC
CGAAAAAACCGGGTTTCATTTTGAATCCGCACAAATGTTTTCAGACGGCATCCGATAAAA
ACATGCCGTCTGAAAAATAATTAGAAATACCCGATTAGCCCGCCTGAATCTTCAATACCG
CCTGTACCACGTCGTTGACGGTGCGGACATTGCGGAAATCTTCGGCCTGCAGCTTGCGGC
CGGTTTCGCGCTTGATGCGGTCAATCAGGTCGATGGCATCGATGCTGTCGATTTCCAAAT
CTTCGTAAAGATTGGTATCAGGCGTAATCCGTTCCGGTTCGATTTCAAACAACTCGGTCA
GGGTATCGCGCAACAACCGGTAGATTTCTTGTTCGGTCATATGTTTCATCCTTATGCTTG
GCGGCTTTTGACAAAGGCGGCGAGTGTTTTGACATTGGCAAAATGTTCGCGCAAGTTCTC
CTGCTCGCCGTCCAATCGGAAACCGAAATGTTTTTGCACCGCCAAGCCCAATTCCAGCGC
ATCGACGGAATCCAGTCCCAGCCCTCCGTCGCCGAACAGCGCGTCTTCGCTGCCGATGTC
GGCGGCGGTTATATCCTCCAAAGCCAAACTGTCGATAATCAGTTGTTTGATTTGGTTTTC
CAAGTCGTTCATATGGTTTTCCTTGTAAAATAGTCCTGCAGATATTCATTCAGCCGCCGC
GCCGCAATCGGCAACGGTTTCTCGGCGAGCCAGTCTTGGGGGAGGATGTCGTCTCCGACG
GTAATTTCATACCGTATCCTTTTCGGGGGGATGCGGTACCACGGCTGCCCCTTTTTAAAA
TTGGGCGGATTCATTTTGATACATACGGGCGTAATCACTTCGGCATACCGCAGTCCCAAA
GAAACCGCGCCCCGGTGCATTTTTACCCGTCCGTCCCACCCCGTCCTCGTTCCTTCGGGG
AACACCAGCAGGCTCTGCCCGCTGTCAAAAACCGCCTTTACCGTTTCCAGCATTGCTTCC
GACTCTTCGTTCGGAATATAGCCCGCACCTTTAATCTGGCTGCTCATTGCCGGATTGTGC
TGCAAATCTTTTTTCACGATACAGTTCATTTCGGGCACATGGCCGACAAGCAGCACCACA
TCCAGCAAAGACGGATGGTTTGCCAAAATCAACTGTCCCGGGCGGTTGAGTTTTTCAACA
CCCTTGAACGATACCTCCAACACGCCCGACCATTTCAGATAAGCAACGAACAAACGCCAA
GATGTTCCGATAATCCGGCGCGCCGCCAATTGGCGGGCGACAGACCCTGAAGTGCCGTTC
AAAGTATAAGGCAACAAAACCAATTTCATCATAATGCCGCCGACACCGAAAATCACAAAT
CCTAACCAAGTCGCAAAAAAACGGCGGCAATAATCCAATTTATCCATTCCGCTGCCACAG
CCATTCACGATTGCGATATACCCGGCGGCATTCTCGACTGCCGTTCAGCAGAAAGCGCAC

## Appendix A

CCATTCCAAACCGCTCCAATATGCCTCGGGCAGCATACCGGCTTCAGACGGCATATCGTC
CGAAGCAGACAAAGTCAGGCTGTAACGCGTCCCTTTGGTCAGAACCATCGCCAAAGCATA
AGCAAACGGCGCGCGAGTCGCCGATACGGCATATCCTTCCGGCAGCGGATCGTCCGCCGC
CAAAACCAAAACCGACCCGCATCCCTCTTCCAACAGTGATGCCGCTTCCGCCAATGCCGT
TTCCACACCGTCGGCACACACGGCCAATGCCGTCTGCTCGCTCATATCCTGACGCAATAT
CGACCATTGCCCCGCCGTTGCATTGTGCACCGACAAACCGAACGAAGTCGGCGACACGGT
ATGCGATTTCAACAGTTCCAACCACAAATCGAAACTGCGTGCCATTTCCCCGTCGTGCGA
GGCATAAACTACCGGACTGCCGGGATGGGCGGAGGCAATGTCCCAAGCCGCGTCGCATAC
CAAACGCGCCGCCTTACTCAAACGGCGGCGCTGCATAGCGGGCAGGAACGGCAATTCCGG
CCTGACATCGGGCAAACCGTCGGCAAAATCCGGACATTCCGCCCATTTTGCCCACTGGGC
CATATCGCGCATTTTGCTGCCCGAAACCCGCCAGGCGGCGATGTCGAAGTGGAACCGGCA
AATAATCGACGGCATAGTTTCTTTCAAAAATTTACACTGTGCCGCATTCTAACCAAAGCC
TATCCCCCTGACAATGCCGAAATTCAAACGCATTTCTGCCCCCTTTCTCCGACAACGCCG
CCCCTCGGAAAACCGCCAGAATTAGCCTGAATTTACATTTATCATTATAATGCCCGTATT
TGCCAGCCTGCCGCCGCAATATATGGACACACTGCCAGAATGCCCGATTACCAACACCGC
CTCCCTGCTGCCGCACAGCGGGCGTATGGTTCTGATAGACCGCATTACCCGATACGGCGA
TGATTTTGTCGAAGCAGGGGCACAGGTAAGCCCCAATCACATCCTTTTACTTGACGACAA
ACTGCCCTACACGGCATTTATCGAACTGATGGCACAGGCTGTCGGCGCGTATGCCGGTAT
CCAAGCCCGAAAAAACGCACGGTCGGTCCGGCTCGGCTTCCTGCTCGGCACGCGCAAACT
TGAAATCTTCGCCCAATCCGTCCCAATCGGCACGCATCTGCTGGCAACGGCGCATATGTC
TATTCAGGATGCCGGGGGTATGGGCGTGTTTGACTGCGAACTGCGTTGGACAGACGCGCC
GGAAACTTCGTCCGAAACACTCCCTTCAGACGGCATTTTGGCGCGCGCCTCACTCAACGT
GTACAGCCCCGAACACCCTGCCGGAACAACCGATGCCGTCTGAACAGGCACACACATACG
GAGAACAACGATGACCGAAACTGTCCTGATTACCGGCTCCAACAGGGGCATAGGCAAAGC
CGTCGCATTCGGTTTGGCGGAAGACGGCTTTGATATCGCTGTCCACTGCCGCAGTCGCCG
CGACGAAGCCGAAGCCGTGGCGGAAGAAATCCGCGCTTTGGGCAGAAATGCGCGCGTGTT
GCAGTTTGACGTGTCCGACCGCGAAGCCTGCCGCGAGATTCTGACCGCCGACATCGAAGC
AAACGGCGCGTATTACGGCGTGGTGTTGAACGCCGGACTGACGCGCGACAATACCTTCCC
CGCGTTTTCAGATGACGATTGGGATGTGGTGCTGCGGACTAATTTGGACGGTTTTTACAA
TGTATTGCATCCGCTGGTTATGCCGATGATACGCCGCCGCAAAGCCGGACGGATTGTGTG
TATGGCATCAGTGTCCGGCCTGACGGGCAACCGCGGGCAGGTCAATTACAGCGCGTCAAA
AGCAGGCATTATCGGCGCGGCAAAAGCCTTGGCGGTCGAACTGGCGAAACGCAAAATCAC
CGTCAACTGTGTCGCGCCGGGTCTCATCGATACCGATATTATCGATGAGAACGTACCTGT
CGAAGAAATCTTAAAGGCTGTCCCCGCAGCGCTTATGGGGCTGCCGGAAGAAGTGGCGCA
CGCGGTGCGTTTCCTGATGGATGAAAAAGCGGCGTACATCACGCGCCAGGTGATTGCGGT
GAACGGAGGTTTGTGTTGAATACCAGAAGGGTCGCAGTAACAGGCATAGGCGGCATTACC
GCCTTCGGCCGGGATTGGCAAAGCATACAGGCAGCATTCAAAGCCGAAAAAAACGCCGTC
AAATATATGGATTGGCACGAACGTTTCCCCGAATTGGAAGCGCAACTGGGTGCGCCGATT
GAAAATTACGCGCCGCCGAAACATTGGACGCGCAAGCAGCTCAGAAGTATGGGGCGCGTG
TCGTACCTGTGCGTCGATGCGGCGGAGCAGGCGCTGGCGGATGCCGGTTTGCTCGGGGAC
GAAAGCATTACCGACGGACGGATGGGCGTTGCCTGCGGCTCTTCCAGCGGCAGCACCAAA
GACATCGGCGATGTGGGCGAATTGTTGCTGACCGGCACGTCGCGCAACTTCAGCGCCAAC
ACCTATGTGCGTATGATGCCGCACACCACCGCCGCCAATATCGGCATCTTTTTCGGGCTG
AAAGGGCGCATCATCCCGACATCGAGCGCGTGTTCGTCCGGCAGCCAAGGCATAGGTTAT
GCCTACGAAGCCATCAAATACGGTCTGACCGATATGATGCTGGCGGGCGGAGGCGAAGAA
TTTTTCCCGTCCGAAGTGTATGTTTTCGACTCGCTTTATGCCGCCAGCCGCCGCAACGGC
GAACCGGAAAAAACCCCGCGCCCATACGACGCGAACCGCGACGGGCTGGTCATCGGCGAA
GGCGCGGGGATTTTCGTGCTGGAAGAATTGGAACACGCCAAACGGCGCGGTGCGATAATT
TACGCCGAACTCGTCGGCTACGGAGCCAACAGCGATGCCTACCATATTTCCACGCCCCGC
CCCGACGCGCAAGGCGCAATCCTTGCCTTTCAGACGGCATTGCAAGACGGAAACGTTGCA
CCCGAAGACATCGGCTGGATTAATCTGCACGGCACCGGGACGCACCACAACGACAATATG
GAAAGCCGCGCCGTTGCAGCGGTTTTCGGCAACAATACGCCCTGCACGTCCACCAAGCCG
CAAACCGGACACACGCTGGGCGCGGCGGACGCAATCGAAGCCGCGTTCGCGTGGGGCATT
GCCGACCGGCAAAGCAATCCCGAAGGAAAACTTCCGCCCCGGCTTTGGGACGGGCAGAAC
GACCCCAACCTGCCCGCCATCAACCTGACCGGCAGCGGCAGCCGCTGGGAAACCGAAAAA
CGCATTACCGCCAGCTCGTCGTTTGCCTTCGGAGGAAGCAACTGCGTCTTAATCATCGGA
TGAAATAAGTTTGTCAATCCCACCGCTATGCTATACAATACGCGCCTACTCTTGACGGGT
CTGTAGCTCAGGGGTTAGAGCAGGGGACTCATAATCCCTTGGTCGTGGGTTCGAACCCCA
CCGGACCCACCAATTCCCAAGCCCGGACGTATGTTTGGGCTTTTTTGCCGCCCTGTGAAA
CCAAAATGCTTTGAGAAACCTTGATAATGAAAAAAGTCAGCGTATTGATTGTTGCCAAAA
ACGAAGCAAACCACATTCAGGAATGTATTGAAAGTTGCCGTTTCGATAAAGAAGTTATCG
TTATCGACGACTACAGCACCGACAATACTGCCGAAATTGCCGAGGGTTTGGGCGCAAAAG
TCTTCAGACGGCATTTGAATGGGGATTTCGGAGCGCAAAAAACATTTGCCATCGAACAGG
CAGGCGGAGAATGGGTTTTCCTGATTGATGCAGACGAACGCTGCACGCCGGAACTATCTG
ATGAAATCTCAAAAATTGTCCAAACCGGCGATTATGCCGCCTATTTTGTCGAACGCCGCA
ACCTTTTCCCCAACCATCCCGCCACACACGGCGCGATGCGTCCCGACAGCGTATGCCGTC
TGATGCCGAAAAAAGACAGTTCGGTGCAAGGCAAAGTACACGAAACCGTACAAACCCCCT
ACCCCAAACGCCGTCTGAAGCATTTTATGTACCATTACACGTACGACAACTGGGAACAAT
ATTTCAACAAGTTCAACAAATATACTTCCATTTCAGCCGAAAAATACCGAGAGCAGGGAA
AGCCCGTGCGTTTCGTTAGGGACATTATCCTCCGCCCGATTTGGGGGTTTTTCAAAATTT
ATATCCTGAACAAAGGGTTTCTTGATGGAAAAATGGGTTGGATTATGTCCGTCAACCACA
GCTATTACACGATGATTAAATATGTCAAACTATATTATCTGTACAAATCCGGCGGAAAAT
TTTAAATGGAAAAAGAATTCAGGATATTAAATATCGTATCGGCCAAGATTTGGGGTGGAG
GCGAACAATATGTCTATGATGTTTCAAAAGCATTGGGGCTTCGGGGCTGCACAATGTTTA
CCGCCGTCAATAAAAATAATGAATTGATGCACAGGCGATTTTCCGAAGTTTCTTCCGTTT

## Appendix A

TCACAACGCGCCTTCACACGCTCAACGGGCTGTTTTCGCTCTACGCACTTACCCGCTTTA
TCCGGAAAAACCGCATTTCCCACCTGATGATACACACCGGCAAAATTGCCGCCTTATCCA
TACTTTTGAAAAAACTGACCGGGGTGCGCCTGATATTTGTCAAACATAATGTCGTCGCCA
ACAAAACCGATTTTTACCACCGCCTGATACAGAAAAACACAGACCGCTTTATTTGCGTTT
CCCGTCTGGTTTACGATGTGCAAACCGCCGACAATCCCTTTAAAGAAAAATACCGGATTG
TTCATAACGGTATCGATACCGGCCGTTTCCCTCCCTCTCAAGAAAAACCCGACAGCCGTT
TTTTTACCGTCGCCTACGCCGGCAGGATCAGTCCAGAAAAAGGATTGGAAAACCTGATTG
AAGCCTGTGTGATACTGCATCGGAAATATCCTCAAATCAGGCTCAAATTGGCAGGGGACG
GACATCCGGATTATATGTGCCGCCTGAAGCGGGACGTATCTGCTTCAGGAGCAGAACCAT
TTGTTTCTTTTGAAGGGTTTACCGAAAAACTTGCTTCGTTTTACCGCCAAAGCGATGTCG
TGGTTTTGCCCAGCCTCGTCCCGGAGGCATTCGGTTTGTCATTATGCGAGGCGATGTACT
GCCGAACGGCGGTGATTTCCAATACTTTGGGGGCGCAAAAGGAAATTGTCGAACATCATC
AATCGGGGATTCTGCTGGACAGGCTGACACCTGAATCTTTGGCGGACGAAATCGAACGCC
TCGTCTTGAACCCTGAAACGAAAAACGCACTGGCAACGGCAGCTCATCAATGCGTCGCCG
CCCGTTTTACCATCAACCATACCGCCGACAAATTATTGGATGCAATATAAACTGCTTTCA
GACGGCATATGCCGTCTGAAAGCCTTTGATGCAACAAACCACTAAATTATATTCGTTCAT
TGGAAAGAAACACCCCGAATTCATCCTTCAAAATAAGAAAATCCCAATATCCCCCGATAT
TACGCAGCCTATTGGCAAAGTTTTGCAGCGTCTTCCCCGGCTTGTGCTGCCGCGTCAAGT
GCTTTGTTACAATGTATAGTAGACTAACAAAAACCAGTACAGCGTTGCCTCGCCTTAGCT
CAAAGAGAACGATTCTCTAAGGTGCTCAAGCACCAAGTGAATCGGTTCCGTACTATCTGT
ACTGTCTGCGGCTTCGTCGCCTTGTCCTGATTTTTGTTAATCCACTATACTACCTTCACA
TTTCTTAATAAATTTTATGAGTAACCATACTTCTTGGTCGTCCAAAATCGGTTTCGTCCT
TGCTGCGGCAGGTTCGGCCATCGGTTTGGGCGCGATTTGGAAATTTCCTTATACGGCAGG
CACCAACGGCGGCGCGGTGTTTTTCCTGCTGTTTTTGATATTTACTATCTTGGTCGCCCT
ACCCGTTCAGCTTGCCGAATTTTATATCGGGCGCACGGGCGGTAAAAATGCCGTCGATTC
CTTCAGGGTTCTGCGTCCGGGCACGCAATGGCTTTGGGTCGGGCGTATGGGCGTTGCCGC
CTGCTTTATTTTGCTGTCGTTTTACAGCGTGGTCGGCGGATGGGTATTAAATTATGTCGT
CCACAGTTTTACGGGGGCGGTTCATACCGGCGCGGACTTTGAAGCCTTGTTCGGCGCGAC
GATTTCCAATCCGGCAGGTTCGCTGTCCTATCAGGCACTGTTTATGCTGATTACGGTTTG
GGTGGTCAAAGGCGGCATTTCAGACGGCATTGAAAAGGCAAACCGTTATCTGATGCCGGG
GCTGTTTATCCTCTTTATTGCGCTGGCAATCCGTTCGCTGACGCTGCCGGGTGCAATGGA
GGGCGTGTCTTTCCTGCTCAAACCGAATTGGTCGTACTTTAAAGCCGATACGATGATTAC
GGCTTTAGGCCAGGCGTTTTTTGCCCTGAGCATCGGCGTTTCCGCCATGATTACCTACGC
TTCATATTTGGGAAAAGATCAGGATATGTTCCGTTCCGGCCATACGATTATGTGGATGAA
CCTCTTGGTTTCGCTGCTTGCCGGCCTGGTGATTTTTCCGGCGGTGTTCGCCTTCGGTTT
TGAACCGAGCCAGGGGCCGGGATTGATTTTTATCGTATTGCCCGCAGTGTTTATGAAGAT
GCCGTTCGGTACGGTTTTGTTTGCGGTATTTATGCTGCTGGTCGTTTTCGCCACGCTGAC
TTCGGCATTTTCGATGTTGGAAACGGTCATTGCCTCAACCATCCGCCAAGACGAGCGCAA
ACGCAAAAAACACACTTGGCTTATCGGCACGGCCATTTTCATTATCGGCATCCCGTCCGC
GCTGTCTTTCGGCGTATGGGGCGAGTTTAAGGTTTTCGGCAAAACCATTTTTGATTTGTG
GGACTATGTTATTTCCGCCGTCATTATGCCGATTGGTGCTTTGAGTGTTTCCATCTTTAC
CGCCTGGATTCAGGACAAGCAGTCTGTGTTAAAAGATGCCGGCGCGGGCAGCACCGTACC
ACGGGCAGTGCTGCTGCTGTGGCTGAATACCTTGCGCTACCTTGCCCCGATTGCCATTAT
TATTGTTTTCATCAATTCTTTGGACATCCTTTAAAAGCCATCCAAACAGCAAAAATGCCG
TCTGAAAGCCTTTCAGACGGCATTTTTGCTTCGGGTTCAGCCTATTTCGTTCAAAGTATA
GTGGATTAACAAAAATCAGGACAAGGCGACGAAGCCGCAGACAGTACAAATAGTACGGAA
CCGATTCACTTGGTGCTTCAGCACCTTAGAGAATCGTTCTCTTTGAGCTAAGGCGAGGCA
ACGCCGTACTGGTTTTTGTTAATCCACTATAGCCTTGCGCGATGCCGTTCAAGGACAAAC
CCATACCCTTTTCGGCAAAACGGATTTCACGGTCGTCAAACGAGACTTTGCCGAAGCCGA
CCCGTTTCAGGGCTTCGTCCACGCTGTTTTGAGGAGGGGGGGTTTCCGCATCGGGACGGG
CGGCAAAATAATCGGCATACAGTTTCCACAACGCCTGCACTGTCGGATCGAACGCGCCGT
CCGTCAGCGCGTGAATATCGCGGCACAGGCTCAACAGTTCCAAAAAATCCGCCGACGGCG
AAGTCAGATAACCGTCCCTGTTCAGGCGGCTGATCAGGCTGTCTTCACGGTAAAGGCTGA
ACAATTTTTTCCAAACGCGCCACTTCCGCCAAAACCTTGTTGACCAAATCCGCCGCACGC
CTGTCGTCCACACCGAACAGACGGAGCTCCGCACCGGAACCCAGTGCGACACCTTTCCAG
AAAAACACATTTTCATTGCGTTTTTCATCCCCGTTGCGTTTTTCATCATCGGCGGCAAAA
GGATTCGGCAGGAAAGAAACCGCCGCGCCCGCCGCCGCAACGGCGGCAACCGTCAGAAAA
CGCCTGCGCCCGAAATGCCTGCCCATACCGCCTCTAAACCGACACTGCCGCCTTGATATG
CGGATGAGGGTCGTAACCTTCCAACTCGAAATCTTCAAACTTGAAGGAAAACAAATCTTT
GACTTCAGGATTGATTTTCATCACTGGCAAGGCGCGCGGTTCGCGTTCCAACTGCAATGC
GGCCTGCTCGAAATGGTTGCGGTACAAATGCGCGTCGCCAAACGTATGGACAAACTCGCC
CGCCTCCAATCCGCACACTTGCGCCATCATCATGGTCAACAATGCGTAGCTGGCAATATT
AAACGGCACACCAAGGAAAATATCTGCACTACGCTGGTAAAGCTGGCAGGACAGTTTGCC
GTCGGCAACGTAAAACTGAAACAGCGCGTGGCAGGGCGGCAAGGCCATTTCATCGACCAA
AGCCGGATTCCACGCCGATACAATCAGGCGGCGCGAGTCGGGATTCTTCTTGATTTGTTC
CAGCACATTGGCGATTTGGTCGATATGCCTGCCGTCGGGCGCGGGCCAGTTACGCCACTG
GTAGCCGTAAACCGGGCCTAAGTCGCCGTTTTCGTCCGCCCACTCGTCCCAAATGGAAAC
ATTGTTGTCCTTTAGGTATTTGATATTGGTATCGCCTTTGAGAAACCAAAGCAGCTCGTG
GATAATCGAACGCAGATGCAGCTTTTTGGTCGTCAGCAGCGGAAAACCTTTGCCCAAGTC
AAAACGCATCTGATAACCGAATACGGAGCGCGTACCCGTACCGGTGCGGTCTGATTTGTC
CGTACCGTTGTCGAGGACGTGGCGCATCAAGTCCAAATAGGCTTTCATAGCAGTCTTTCA
TCAAATTAAACGGCGCATATTGTAACATTTCCGGATAATGCCCAAAACACGGATACAGGC
AGGCAGGATTGTTGGCAATTTCAGTCCTTTTCCACAGTAAAACCCGGTGGGAAAACAAAA
TTACCTTGATTGGAATCAAAAAATCTAGTTTAATTACTTAGAATAAAATTTCAATAATAT
TTTTATTTACGAAATTAATTTATGATTATTGATTAATTATCGGCAACAAACATCAAACAT

## Appendix A

CGAAAATATGGAAAAAATAATGTCAACAATTTTTGCCAAATCGGGCTTGGCATCAGAAAA
AAATAGGTTTATATTCCCACCTACAAATTTGTTTTCCCATTAGTACACTATCAACCAAAA
GGAGTATCCGAATGACTGACCTGAACACCCTGTTTGCCAACCTCAAACAACGCAATCCCA
ATCAGGAGCCGTTCCATCAGGCGGTTGAAGAAGTCTTCATGAGTCTCGATCCGTTTTTGG
CAAAAAATCCGAAATACACCCAGCAAAGCCTGCTGGAACGCATCGTCGAACCCGAACGCG
TCGTGATGTTCCGCGTAACCTGGCAGGACGATAAAGGGCAAGTCCAAGTCAACCGGGGCT
ACCGCGTGCAAATGAGTTCCGCCATCGGTCCTTACAAAGGCGGCCTGCGCTTCCATCCGA
CCGTCGATTTGGGCGTATTGAAATTCCTCGCTTTTGAACAAGTGTTCAAAAACGCCTTGA
CCACCCTGCCTATGGGCGGCGGCAAAGGCGGTTCCGACTTCGACCCCAAAGGCAAATCCG
ATGCCGAAGTAATGCGCTTCTGCCAAGCCTTTATGACCGAACTCTACCGCCACATCGGCG
CGGACACCGATGTTCCGGCCGGCGACATCGGCGTAGGCGGGCGCGAAATCGGCTACCTGT
TCGGACAATACAAAAAAATCCGCAACGAGTTTTCTTCCGTCCTGACCGGCAAAGGTTTGG
AATGGGGCGGCAGCCTCATCCGTCCCGAAGCGACCGGCTACGGCTGCGTCTATTTCGCCC
AAGCGATGCTGCAAACCCGCAACGATAGTTTTGAAGGCAAACGCGTCCTGATTTCCGGCT
CCGGCAATGTGGCGCAATACGCCGCCGAAAAAGCCATCCAACTGGGTGCGAAAGTACTGA
CCGTTTCCGACTCCAACGGCTTCGTCCTCTTCCCCGACAGCGGTATGACCGAAGCGCAAC
TCGCCGCCTTGATCGAATTGAAAGAAGTCCGCCGCGAACGCGTTGCCACCTACGCCAAAG
AGCAAGGTCTGCAATACTTTGAAAAACAAAAACCGTGGGGCGTCGCCGCCGAAATCGCCC
TGCCCTGCGCGACCCAGAACGAATTGGACGAAGAAGCCGCCAAAACCCTGTTGGCAAACG
GCTGCTACGTCGTTGCCGAAGGTGCGAATATGCCGTCGACTTTGGGCGCGGTCGAGCAAT
TTATCAAAGCCGGCATCCTCTACGCCCCGGGAAAAGCCTCCAATGCCGGCGGCGTGGCAA
CTTCAGGTTTGGAAATGAGCCAAAACGCCATCCGCCTGTCTTGGACTCGTGAAGAAGTCG
ACCAACGCCTGTTCGGCATCATGCAAAGCATCCACGAATCCTGTCTGAAATACGGCAAAG
TCGGCGACACAGTAAACTACGTCAATGGTGCGAACATTGCCGGTTTCGTCAAAGTTGCCG
ATGCGATGCTGGCGCAAGGCTTCTAAGCAAACGCCGCCGCTCCGCAAACAAAATGCCGTC
CGAACCGCAAATGCTGTTCAGACGGCATTTCCTTATCCGCCCGTTCAAATCGGGTGAGAC
TACCGATACATCTGAATATGCTATGCCGTCTGAACGGCATTCACACCGCCCAATCCTGCA
CGCGCTTCAAATCATTTTGCGCCAAAGTATCTGCGTGGCGGTTACGGCTCTGATATTCCC
TGTCTTTCAAGATGCTGCTCGCCACATAATTCAAATGTGCCTTTGCCGCCTCCGAAGCCT
CGCCCGGCCGGCGGTTTGATATTGCCTCATACAATACACGGTGCTGCGCCATCAGCTTCG
GACGCGGATCTTCTTCCTGATTCAGATAAATAAGGCTGCTGCGCGTCTGCCGGTACAGCA
TTTTCAACAAACCGCCCGACAAATGGCTGAACAACAAATTGTGCGCCGCATCGGCAATCG
TCTGATGAAAGCTGACATCAGCTTCGCTCTGATGTTCCAAATTGCCGCTTTCGCACGCCT
CCTCAAACTTTTCAAGCCAAAACCCAATCCGCTTCAAATCGGCATCCGTGCGGCGTTCTG
CCGCCAATGCCGCCATACAGCCCTCGATGTGGCAACTGAAATCAAAAACATCCTGTTCCC
AATTGGAATGCTTGCCCAAAAGCTCCTGCCAACTTTGCAAAAAATCCTGCTGCGGCTTGA
CCGAAACATAATAACCGTCTCCCTGCCTCGCTTCCAAAACCTGACGGGCGACCAAAACAT
TCAATGCCGACCTGACCGACGGGCGCGAAACGCCGAACTCTTCCGCCAAAACGCGTTCGG
GCGGAATCTTGCCCCCTTCCGCGTAAACCCCTTCCGCAATGCGCTCCTCCAATACCGACA
ATACCTGATCGCTGATTTTCTGAGGCCTTACCAGTTTCATCACTCCTCCTTTATAAAGAT
TCCCTGCAGAACCCTTCCGAAATATAGTGGATTAACAAAAATCAGGACAAGGTGACGAAG
CCGCAGACAGTACAAATAGTACAGAACCGATTCACTTGGTGCTTCAGCACCTTAGAGAAT
CGTTCTCTTTGAGCTAAGGCGAGGCAACGCCGTACTGGTTTTTGTTCATCCACTATACAT
CAAACATCAAATTGGACTGACCAATCAGGGCGGATTCTAATGACACGCCGTTCCCGCCGT
CAACGGCATTTACCTCGCACCGCCCCCGAAACACAAGAAAAAACTACACCAAACTACAAT
TTTTGTTCATGCAAATATTTGTTTTGACAGGATTTAAACAAAAGCTCCGATTCAAATCTG
CCGAACCGCCCAAAAATATATTGACCTAAATATTAAAGTTTCGTAAAGTAATGCAACGTT
GCTTTAATTGGTTTGACCACTATTGCCGACGATTAGAAAAATATTTTCGGAGATGTTCAA
TTATGGAAACTTGGGTTCAAAACTACACGGCAATCGGCGGCAGCCTGTATCTGACTGCCG
GGGGCGCACTCTTACCATCGTCTTTTTCTTTGCCGCGCTGACCGTCCTGAAGCTGAAAG
GCTATCAGGCGGGGCTTTATACGCTGCTGATTGCGCTTGCCGTTGCCGTATTCGGCTTCG
GGATGCCGACGGGTATGGCGGTTTCTTCCCTGCCGCCGCAGCCGCATTGACCCAACGCCC
CTACGCCACACTGTATTTGACCGCGCATTACAAAATCGGCAAATCCACCCGCATCGGTTT
GGACTTTGAAAACGTGTTCAACAAACGCTACCGCCCTATGCCCGACATTCACGTTTACGG
CACGCCGCGCAGCCTGACCGCAACCGTCAAACATATAGTGGATTAACAAAAATCAGGACA
AGGCGACGAAGCCGCAGACAGTACAAATAGTACGGAACCGATTCACTTGGTGCTTCAGCA
CCTTAGAGAATCGTTCTCTTTGAGCTAAGGCGAGCCAACGCTGTACTGGTTTTTGTTAAT
CCACTATAAAGAAAGAAATGCCGTCTGAAACCTTATCGTTTCAGACGGCCTTGGATTCGG
ATTTCAAGTGCAACACTAGTGTATTAGTGGTTGGAACAGATTCAAGAATAAAACACTTGG
CGTTTCGTAGCCAAGTGTTTTTCTTGGTCGGTGGTTCAACTCATCTTGAACCCTGCGTAT
CTCCCGATCACTGATGTTACGGAAATCGGTTTGTTTGGGGAAGTATTGCCGGATGAGTCC
GTTGGTGTTCTCATTCAGCCCTTTCTCCCAAGAATGGTAAGGGCGACAAAAATAAGTCTC
CGCTTTCAATGCTTTGGTTATTTTGGTGTGTTGGTAGAACTCTTTGCCGTTATCCATGGT
GATGGTGTGCACCCTGTCTTTATGTGCCTTTAATGCCCTAACAGCTGCCCGGGCAGTGTC
TTCGGCTTTGAGGCTATCCAATTTGCAGATGATGGTGTAGCGGGTAACGCGTTCGACCAA
GGTCAATAATGCGCTTTTCTGTCCTTTGCCGACAATGGTGTCGGCTTCCCAATCGCCGAT
ACGGGATTTCTGGTCGACGATAGCGGGTCGGTTTTCTATGCCGACACGGTTGGGTACTTT
GCCTCTGGTCCATGTGCTGCCGTAGCGTTTGCGGTAGGGTTTGCTGCATATTCTGAGATG
TTGCCACAACGTGCTGCCGTTGCTTTTGTCTTGGCGAAGGTAGCGGTAAATGGTGCTGTG
GTGGAGCGTGATCCGGTGGTGTTTGCACAGGTAGGCGCATACTTGTTCGGGACTGAGTTT
GCGGCGGATAAGGGTGTCGATGTGCTGAATCAGCTGCGAATCGAGCTTATAGGGTTGTCG
CTTACGCTGTTTGATAGTCCGGCTTTGCCGCTGGGCTTTTTCGGCGCTGTATTGCTGCCC
TTGGGTGCGGTGCCGTCTGATTTCGCGGCTGATGGTGCTTTTGTGGCGGTTCAGCTGTTT
GGCGATTTCGGTGACGGTGCAGTGGCGGGACAGGTATTGGATGTGGTATCGTTCGCCTTG
GGTCAGTTGCGTGTAGCTCATGGCAATCTTTCTTGCAGGAAAGGCCGTATGCTACCGCAT

## Appendix A

ACTGGCCTTTTTCTGTTAGGGAAAGTTGCACTTCAAATGCGAATCCGCCGGCATTTTATT
GCCCGACCGGTTATTTGTCGGTTTGGGTATCCCGTTTCAATCCGCCGCCGAGTGCCTTGT
ACAAATCGGCAAGGTTTTCGGCGCGGGTCAGTTGTGCCGACAAAGCCGCACCCTCCGCCG
CATAGCTGCTGCGTTCCGCATCGAGCAAGTCGAGCGCGCCGGATACGCCGTGCTTGTAAC
GCAGGCCGACCAAGCGCAACGCTTCTTTAGAGGCGCGGCTTTGTTTGCTTAAAGCGTCAT
AGGCTTTATCCAGCTGCTCGCGCGCCGCCAATGCGTTTGCCACGTCTTGAAATGCGGATT
GGACGGCGGATTCATAGGCAACGATTTGTACCTGTTGGCGCAGCTTGGCTACATCAAGGT
TCGCCTTGTTCGTACCCCAGGTAAAAATCGGCAGGGTAATAGACGGCGCGAACGACCAAA
CGCCCGTGCCGCTTTTGAACAACCCACCCAATTCGGCAGAACCCGTACCGACGGTTCCGG
TCAGGCGGATGGATGGGAAAAAGGCGGCGCGTGCCGCACCGATATTGGCGTTTGCCTGTT
TGAGCGCGTGTTCGGCAGCACGGATATCGGGACGGTCGAGCAATACTTCGGAACTCAAAC
CGGCCGGCAGTTTTTCAACAAAAAACTGCTTGTCCAGCGGCAAACCGGCAGGCAGGTCTT
CGGGTATCGGTTGGTTAATCAAGGTTGCCAAGGCATTGCGCGCCTGTTCGCGGCTGCGCG
CGGCATGGGCATAATCGGCTTTGGCAGATTCGATCAGGGCTTCCTGCTGACGTAGGGCGA
CGGCGGAAATCACGCCTGCCTTGTAACGTAATTCGGACAGCTTGTAGGTTTCCTCGCGCG
TTTTCAAAACACGTTGCGCCAAAGACATCGCTTCTTCGGCGTAACGTTCGTTGAAATAGG
CTTTGGCAACGGTGGCAATCAGGCTCAAATGTGCCGCATCGCGGTTGGCGGTGCTGGCGA
AATAGCCTTGCAGTGCCGCCTCGCTGCTGCTGCGTACACGCCCGAACAGATCGAGTTCGT
AAGATGCCGCACCCAGTCCGACTTTGTAGCTGCTGCTTACATTGCCGCCGCTCAAGCTGC
CTTGGCGCGAGTCGTTCGCATTGGCGGCAAGCGTGGGCAGGAGGTTGTTGCGCTCAATCA
TGTATTGTTTGCGGTAGATTTCGCTGTTCAATACGGCGGTACGCAAACTGGTATTGCGCT
CGAGTGCGATGTCGATCAGCTTTTGCAGGCGCGGGTCGGCAAAATAGTCATGCCAACCTA
AATCGACGGCGCGGATGCCGCTGTCGGCGGTATCGTTTTTGAACGTTTCGGCAACTTCGA
CTTTGGGCTGCTCGTATTGGGGAATCATGGTGCAGGCAGACAATGCAAAGGCTGCTGCAA
CAGAAGTCAAGGTGGTTTTCAATGTAGTATCCATAAAAAAGTCCTGATGCCGTCTGAAAA
CCCGTGGGCGTTCAGACGGCATGGTTGCTTAATGTTGGCTGTCGTCCGAACCGGTGATGC
CCGCTTCGGCGGCGTGTTTTACTGCCATTTCGTGTTCGTGCGCGGTTTCTTTGAAGAATT
TGCGCACCACCACATAGAAAAGCGGAACAAGGAACACGGACAAGAGCGTGCCGATGAGCA
TCCCCCAGAATACGGTTGTACCGATGGCGCGCTGGCTGGCAGAACTTGCACCGCCGGCAA
TATACAGGGGAACCACGCCCAAAATAAAGGCGAACGAGGTCATGATAATCGGACGGAAAC
GCAGGCGGGCGGCTTCCAAAGCGGCTTCAACCGCGCTTTTCCCTTGCGCTTGAAGGTCTT
TGGCAAATTCGATAATCAAAATCGCATTTTTCGCACTCAAACCCATCACGGTAACGAAAC
CGACTTGAAAGTAGATGTCGTTGGCGAACGAGGGAACGCTGCCCAACAGTCCTTCAAACA
GGTTGCGCCCGGTTACGCCCGCAGCCGCACCGATCAAACCCAACGGAATCACAAGGATGA
CCGCCAGCGGAATCGACCAGCTTTCATAAAGCGCGGCAAGTACCAAAAATACGGCTGCAA
CCGCCAAACCGTACAAAATCAGGGTTTGCGAGCCGCCTTTTGCCTCTTCGCGCGACTGTC
CGCCCCACTCCAGGCTGTAACCGCCGCCCAATTCGTCAACCATTTTTTGAACCGCCGCCA
TAGCCTGCCCGGTGGAAACGCCGGTTGCAGGCGAAGCGGACAGCTTCATCGAAGGATAAC
CGTTGAAGCGTACGCTCTGTTCCGTACCGTTTTCCCAAGAAACAGTAGCAATGGTGGAAA
GCGGTACGGCGACGCCGGATTTGTTCGGCACGGTCAGGTTCAAAATATCGGCAGGCTGCA
TACGGGCATCCTCGTCGGCCTGCACCATCACGCGTTGCAGACGGCCTTGGTTCGGGAAGT
CGCTGACATAAGACGAACTCAGCGCGCTTGCCAATGCGGTGCGGATGTCGGCAAACGAAA
TGCCTTGCGCCGCCGCCGCGGCACGGTTGATGTCGATTTTCAACTGCGGCGAGTCTTCCA
AACCGCCAGCACGGACGGTGCTGGGGTCAAACAAACCGCTGGCACGCATTTTCTGAATCA
ACTCGTTGCGCTTCGCCAGCAATGCGGTATGGCCGGTATTGTTGCGGTCTTGCAGGTTGA
TGCTCAGACCCGAACCGTTGCCCAACTCCAGAATCGGAGGCGGGACGACGGCGATGCCAA
AACCGTCTTTAAGCGTCCCCATCATCATACCCGTCAGCTTGCCGGCAATCGCAACGGCAT
CGCTGCCGGGCGCGGTACGCTCGTTCCAATCTTTCAATATGGCAAAACCCATCGCCATAT
TCTGACCGCTGCCCGAAAAGCTGAAGCCGGAAACGGTAATGATGTTTTCTATTTCAGGAA
TGCTTTTCGCCAGTTGGGTAACTTGCGCCAAAGTCGCATTGGTGCGCTCTTGGGTCGCTC
CTGCAGGCAGTTGCACGCTGACCATGACGAAGCCTTGGTCTTCGGTCGGCAGGAATGAAG
TCGGCAGGCGCATAAACAGGAACACGCCCACAACCGCCAAGCCGATATAGACAACCATCA
TGCGGAAAGTCTTACGCAGCACTTTGGCAACCCGGCCTTCGTAACCGTGCGTCCAACTGT
TGAATTTCTTGTTAAACCAGCCGAAGAAACCTTTTTTCTCTTCGTGATGCCCTTTCGGGA
TTGTCTTCAACATTGTGGCACACAAAGCAGGGGTAAGGGTCAGCGCAAGGAAGGCGGAGA
ATGCGATTGATGACGCCATCGTCAGGGCAAACTGTTTGTAAATATTGCCCGTCGCCCCGC
TGAACATCGCCAACGGTACGAACACGGAAATCAGAACGGCGGTAATACCGATGACCGCGC
CCGAAATCTGACCCATCGCTTTTTTGGTCGCTTCTTTGGGCGGCAAGCCTTCACCCGCCA
TAATGCGCTCGACGTTTTCAACCACCACAATCGCGTCATCGACCACGATGCCGATGACCA
AAACCATCGCAAACATGGTCAGTACGTTAATCGACATGCCCATATAAGAGATGAAGGCGA
AACCGCCCAACAGCGAAATCGGTACGACGATGGTCGGAATCAGCGTATAACGGATGTTTT
GCAGGAAGAGATACATTACGACAAACACCAGCACCATCGCTTCGATTAAAGTGTGAATCA
CTTTTTCAATCGAAATTTCGACGAATTTGGAAGTATCGTAAGGGGTTTTCCAGCTCATAC
CCTGAGGAAAGTATTTTTCCAACGTCGCCATGCGTTCTTTAACCGCCTTTGCCGTCGCCA
TCGCATTGCCGCTGTTGGACAGCATCACCGCCATACCGGTGGTATTTACACCGTTCAGAC
GGGTTGAGGAAGAATAGTCTTCCATACCCAGTCCGACCCTTGCCACATCCTTCAGGTAAA
CATTAGAACCGTCGGTATTGGCGCGGAGGATGACGTTGCCGAATTCTTCTGCCGTACCCA
ACTGCCCTTGCGCCGTTACGGTAGCCGTAACCGTCTGTCCGCGAACGGCGGGAAGCGAAC
CGATAGAACCCGCTGAAATCTGGACGTTCTGGGCGGACAGCGCGCTGCCAACATCGGCAA
ACGACAAATTGTAGTTTTGCAGTTTCTTAGGATCAACCCAAATCCGCATCGCGCGTTGCG
CGCCGAACAGGCGTACCTGCCCCACGCCTTCGATACGCTGCAACTCGGGAACGATATTAC
GCTGCGCGTAGTCGTTCATCTCTTCGGTTGACTGCACATCCGACGAAAGCATCACAATCA
TCAGGAAATTGGAACGCGCCTTGGATACGGTTACGCCGTATTGCTGGACAGTTGCCGGCA
GCGTGCTCAATACTTCGGAAAGCTTGTTCTGCACTTCCACCTGCGCCAGATTCTCGTCGG
TATCGGGCGTAAAGGTCAGGCTCACGCTGCCGCTGCCGCTCGAATCGGCGGAAGTGGACA

## Appendix A

TATAATCCAAACCTTCCACGCCGTTCATATTCCGCTCGATCACGGAAAGCACGCTGTCTT
CCATTACCTGCGCGGACGCGCCCGGATAAGTGGCCCTCAGGGTGATGGTCGGGGCGGCGA
CGGACGGATATTGCGAAACCGGCAGGCTTTTGATGCCGAAAATACCCGCCGCAATAATGA
AAATCGAAATAACCCACGCAAAAATGGGGCGGTCGATAAAAAATTTAGCCATCGATGCCT
TCCTTATTTCGCTTCAGAAGCAGGTTTGGCTTCAGATGCCGTCTGAACGCCGGATTGAGG
CGCGGCGGCTTGGTTTTCAGACGACGCCCATTCTTTGGGCGTTACCTTTTTCGCACCCGT
TATACCGGCGATACTGATGCCTTCCACAACCACCTTGTCCCCGTCCTTCAGACCCGACGT
AACAATCCAATTCGTACCCTGCTGTTGCGCAACCGTTACCTCGCGGGGTTCCATACCGCC
TTGGGCATTCACAATCATCACGGTATCTTTCGCACCGCGCGTTACCGCCTGCTGCGGCAC
AACAAATGCGTTATCCACCGCCACTTGGTCCATCAGCACGCGCACATACAGACCGGGCAT
CAAGATATTCTGATCGTTCGGTACGGCGGCGCGCAGGGTAATCTGACCGGTCGATTCGTT
GACGGCCGGATCGGCAAACAGCAGGCGGCCTTTTTCAGGGTAAACTGTGCCGTCGTCAAA
TTTGATGCCGACCGCAATCACACCATCCGCCGCCAGCAGTTTGCCTTCGGCTATCTGACG
GCGCAATTTCATCACTTCGGATGCAGACTGGGTAACGTTCACATACATCGGATTGGTTTG
GCGGATGGTCGCCAGTACGGTCGCATCGCCAGCGTTCAGCAACGTACCTTCGGAAACTTT
GGACTGACCGATAAAGCCGGAAATCGGCGCGGTAATGCGCGAACGGTTCAGGCTGATGCC
GGCGGATTTGATTGCCGCCTGCGCCGCTTTAACGCCTGCCTCGGCAGAACGTTTCGCCGT
TACCGCAGCATCGTATTCCTGCCGGCTGACGGCTTCGGCGGCAACCAAAGGCTTGTATCG
CGCCAAATCCGCATCCGCTTTGGCAAGCGTTGCCTGAGCCGTTGCCAGTTGCGCGCGCGC
GCTTTCCAGACCTGCTTCATAAGTGGAACTGTCGATCTGATACAGCGGCTGTCCGGCACG
GACATAACTGCCTTCTTGGAACAGGCGTTTTTGGATGATGCCGCCGACTTGGGCGCGGAC
ATCGGCGGTACGCAGCGATTCCAAACGCCCCGGCAACTCGACGGTCAATGCGACGGTTTG
CGGATGGACGGTTACGACACCGACGACGGGCGCAGGGGCTTCCCGACCAGCAGGCTGCCC
GCCCTGCGCCGCGTCTCCGCCTTTACCGCAAGACGACAGTACCAATGCAACGGCGGCAGC
CAACGCGGCCGCACGCATCGCCTTAAAAGCATAAAAAGCCATTATTTATCCTATCTGTCT
GGTTTGATGTAAAGGGTTTTGCCAATCAACAGGCATTCTTATAGTGGATTAACAAAAACC
AGTACGGCGTTGCCTCGCCTTAGCTCAAAGAGAACGATTCTCTAAGGTGCTGAAGCACCA
AGTGAATCGGTTCCGTACTATTTGTACTGTCTGCGGCTCGCCGCCTTGTCCTGATTTTTG
TTAATCCACTATATTTCAGGATATAAAAACCGCCTGCTTCGCCAACCCGATGTTCAAACG
GGTTGCGAAGCAGGTTTCATGGGTTTTCAAAGTTGAGATGTAGTCTCAATTTCATGGGTT
TCATTATACATACACGATTGCATGGTTACAAAGTCTTTTTTATAATCCGCCCTCATCAAA
CCGACCCGAAACGAAACCGCCATTATGAGAAAAACCAAAACCGAAGCCTTAAAAACCAAA
GAACACCTGATGCTTGCCGCCTTGGAAACCTTTTACCGCAAAGGGATTGCGCGCACCTCG
CTCAACGAAATCGCCCAAGCCGCCGGCGTAACGCGCGGCGCGCTCTATTGGCATTTCAAA
AATAAGGAAGACTTGTTCGACGCGCTGTTCCAACGTATCTGCGACGACATCGAAAACTGC
ATCGCGCAAGATGCCGAAGATGCCGAAGGAGGGTCTTGGGCGGTATTCCGCCACACGCTG
CTGCACTTTTTCGAGCGGCTGCAAAGCAACGACATCTACTACAAATTCCACAACATCCTG
TTTTTAAAATGCGAACACACGGAGCAAAACGCCGCCGTTATCGCCATTGCCCGCAAGCAT
CAGGCAATCTGGCGCGAGAAAATTACCGCCGTTTTGACCGAAGCGGTGGAAAATCAGGAT
TTGGCTGACGATTTGGACAAGGAAACGGCAGTTATCTTCATCAAATCAACCTTGGACGGG
CTGATTTGGCGGTGGTTCTCTTCCTGCGAACGTTTCGATTTGGGCAAAACCGCCCCGCGC
ATCATCGGGATAATGATGGACAACTTGGAAAACCATCCCGACCTGCGCCGGAAATAATCA
AGCCTTGGTAGCAATGCCGTCTGAAACGAACAAACCCTTTCAGACGGCATCAAAATGACA
CAAAGCCTTCTTCTAAAAATACATATTGAGACCTTTGCAATAACATAGGTTACTAAAATT
TTATGCTCAATCTTATTTTCAAAATGCAAAACTTTTCTGATTTTTCCTACTTTTTGCTCA
ATATTAGGAAGGTTTTAGGCAATTGAAAATTTTTTGGCGCATTTTTATGCGTCAAATTTC
GTTAACAGACTATTTTTGCAAAGGTCTCATATTCACTAAATTGCATTTTTAATTTCTTCT
ATCATTGCATGGACATTCTCTTGGTCAAAATGTCCGTTTTCTTCTGAATAAACTTCTAAC
AAATAATGTTCAATGAACGTTTTATCTGTCGTCAGCGATACATCTCTGGCAATGTCTTCA
~~TACGACTCAAAATCATCTTCATGCCAGGGATTATATTTGTCCATATTTTTTTGAATTTCA~~
TTTTTCATATCATTTACCTTCCAATATTTATTTACAATTAATAACAATACCATTCAAAAT
GTAAACTGCATTTTTCTCCAGCATTTTTGCAAATAAAAACTGAAAATCCCGCCATTTCCG
CGAAAACGGGAAACCGTTTTTTGAGTTCCAGTCATTCCTGATAAGGCTTTAACGTCAAGT
TTTCGGATTACCGCCTTTATGAGAATAACGATGTGGGCATTTTCTGTTTTAATCTATTGC
GGTTATATACATATGCGATTATTTTAGTTTGCTTACAAAACACTTCATGTTACATTCAAA
AATTTAATGCACTCAATATATTTTTTTAAGGAGAAGCAGATGAGTCAAACCGATACGCAA
CGGGACGGACGATTTTTACGCACAGTCGAATGGCTGGGCAATATGTTGCCGCATCCGGTT
ACGCTTTTTATTATTTTCATTGTGTTATTGCTGATTGCCTCTGCCGTCGGTGCGTATTTC
GGACTATCCGTCCCCGATCCGCGCCCTGTTGGTGCGAAAGGACGTGCCGATGACGGTTTG
ATTTACATTGTCAGCCTGCTCAATGCCGACGGTTTTATCAAAATCCTGACGCATACCGTT
AAAAATTTCACCGGTTTCGCGCCGTTGGGAACGGTGTTGGTTTCTTTATTGGGCGTGGGG
ATTGCGGAAAAAATCGGGCTTGATTTCCGCATTAATGCGCTTATTGCTCACAAAATCGCCA
CGCAAACTCACTACTTTTATGGTTGTTTTTACAGGGATTTTATCTAATACCGCTTCTGAA
TTGGGCTATGTCGTCCTAATCCCTTTGTCCGCCATCATCTTTCATTCCCTCGGCCGCCAT
CCGCTTGCCGGTCTGGCTGCGGCTTTCGCCGGCGTTTCGGGCGGTTATTCGGCCAATCTG
TTCTTAGGCACAATCGATCCGCTCTTGGCAGGCATCACCCAACAGGCGGCGCAAATCATC
CATCCCGACTACGTCGTAGGCCCTGAAGCCAACTGGTTTTTTATGGTAGCCAGTACGTTT
GTGATTGCTTTGATTGGTTATTTTGTTACTGAAAAAATCGTCGAACCGCAATTGGGCCCT
TATCAATCAGATTTGTCACAAGAAGAAAAAGACATTCGGCATTCCAATGAAATCACGCCT
TTGGAATATAAAGGATTAATTTGGGCTGGCGTGGTGTTTGTTGCCTTATCCGCCCTATTG
GCTTGGAGCATCGTCCCTGCCGACGGTATTTTGCGTCATCCTGAAACAGGATTGGTTTCC
GGTTCGCCGTTTTTAAAATCGATTGTTGTTTTTATTTTCTTGTTGTTTGCACTGCCGGGC
ATTGTTTATGGCCGGGTAACCCGAAGTTTGCGCGGCGAACAGGAAGTCGTTAATGCGATG
GCCGAATCGATGAGTACTCTGGGGCTTTATTTGGTCATCATCTTTTTTGCCGCACAGTTT
GTCGCATTTTTTAATTGGACGAATATTGGGCAATATATTGCCGTTAAAGGGGCGACGTTC

## Appendix A

TTAAAAGAAGTCGGCTTGGGCGGCAGCGTGTTGTTTATCGGTTTTATTTTAATTTGTGCT
TTTATCAATCTGATGATAGGCTCCGCCTCCGCGCAATGGGCGGTAACTGCGCCGATTTTC
GTCCCTATGCTGATGTTGGCCGGCTACGCGCCCGAAGTCATTCAAGCCGCTTACCGCATC
GGTGATTCCGTTACCAATATTATTACGCCGATGATGAGTTATTTCGGGCTGATTATGGCG
ACGGTGATCAAATACAAAAAAGATGCGGGCGTGGGTACGCTGATTTCTATGATGTTGCCG
TATTCCGCTTTCTTCTTGATTGCGTGGATTGCCTTATTCTGCATTTGGGTATTTGTTTTG
GGCCTGCCCGTCGGTCCCGGCGCGCCCACATTCTATCCCGCACCTTAAACACGATAAACA
AAATGCCGTCTGAAATGCTTAAACGCTTTCAGACGGCATTTGCCTTTCTATCCCGTCAGG
CTTCTCCGGCCTCTTCCTTTTTTCCGCTGCGGCAAGCGTGTCGGCAAGCAGACGGACGA
GGTTTTCAAACAGGGGCTGTTCGAGCGGGTTTTGGCTTGCGTTGCCGAACACGAGGGCGA
CTTCGGTATAGTCTTTCTGCCCTTTGCTGACTTTGCTGCCGTGGTAGGCGGTTTGGGCTT
TTTTCAGGGCGGCTTCCCAATCTTGTTTTTGGGCAAACGCTTCGGCGGTGGCAAGCGAGG
TTTTGGCAAAAAACGGCAGCGGGCGGTTTTGCCCGATATTGAAAAACGCCATCCATTCCG
ACAATAGCTGCAATGCCCTGTCTTGCGCGATTTCCGCCAATATTTCGGTTTCTCCGGATT
GGACGATAAAGGTTTGCCGCATTTCGGCTTCAGACGGCATAACGGCGCAAAATATCAGGT
GTTCCAGCAGAAAAGCGATACGTTGCGGCGCGTTGGGTTTGCCGTAGGCGTAAAACACTT
GTCCGCAGCGGTACAGATTGCCCAAGCTGCCTTTCAGGATTTGCCCGTCCGACGGTATGG
CATATGAAAGCGGTGGCAGTTTGGGGCTGTTTAAAACCGCCGTGTCGATTTGTTTGGCAG
CAGTTTGGAAGTCCTGCTGCCAAAGTCTGCCCAACTCTCCCGACGGCAGGAGGCTTTCCG
CCCCGATGCGGGCGGCGGTTTGGGCAAAATCCCGTCCTTCGCACCGTGCTTCGATGTAGA
TTTCGGCGATTTGATCGGCGTGTTGCGGCTCGAAGGGTTCGGCAGGCTCCCAGGCTTCGC
CGATATGGGGTTCGCTCCACGCAAGCTGCTGCTGAAGCCATACTTTGACAGGGTTGCGCC
AGAAACGGATAAATTCGTCCTGTCCGATTTCGGCAACAGGTTCGGCGTTTTCTACGGGTT
GATCGAAAAAGGGTTGCGGCGGTTCGGGCGTTTGTCCGAGCGCGGCGGCGTAGTCGGTAC
GCGTGCCGAATATGCCGTCTGAACGTCCGCCTTCTTGAAAATATCGGCGCGAGAAGGCTT
GCAGCGGATGCTGTTCTATCAGGTTTTGTGCAAGTTGGCGGCTACCGATGCCCGCCATAG
CGGCAACGGTATCGATGAGTTCGCCCAACAGGGAAGACGGGGCAAGCTCTTCGTCTTTGC
GGATGTCGCGCCCGATGTAGGACAGGTAGAGGATTTCACGCGCGCTGATGAGGGCTTCGA
GGAACAGGTAGCGGTCGTCATCGCGGCGGGCGCGGTCTCCTTTGGCGGGATGTTTGGCAA
TCAGGTCGAATACGGCGGCTTTGGTATTACGGGGAAAATCTCCGTCGTTCAAACCCAACA
GGCAGATGACTTTGAACGGCAGGCTCCGCATCGGCACCATACTGCAAAAGGTGATGCCGC
CGCGTAAAAAGCCTGCCTCGCTTTCGCTGTCGAGAAAGCGTCGGATATGGCGGATGACGG
TGTGCGGCGGCAACTGTCCGGAAAATTGCGCCAATTCGGTTTCCGCCTGCCATTTGACCC
ATTCGTTTTCAAGGTTTTGGACTGACTTTTGGTCATCGGCTTCAGCTTGGAACAATGTTT
CAAGCAAATCCCGGCAACGCGCCACCCATTCGCCGACCGTTGCGGGCTGCCGCCATATCC
GTACAATATCCGTCAGGGTTTCGAGGAAGGCGGCAAAACGTCCGAACATGGCGGTTTGAT
TCACGTCGGCATACCACGCGCTGACATCCTGCCACATCGGATTGCCGCCTTTGGGCAGCA
TCCAGCCCAATATCATGCGTTCTACCGCCTGCTTCCAGGTGAACAGCTGATCCGTGCCGC
CGCGCATTTCTCCGTCCAAACCCCAGTGGACGTTCAAATCGGCAACCATGTCGTGCAAAA
GCGGTAAATCGTCCTCAGTCAGTCCGAAACGGCGCAACACGGGCGCGGTTTCTAAAAGCA
CAAGCACTTTATCGACTTCAAATCGGCTTTCCAACAAGTCGAACAGGCATGACAAAGCAT
GAAACAGCGGTTGGCGGCGGCTGATTTTCACGTCTGACACGGAATACGGCAATGCCTGCG
CACCGGGCTGCGCCTGTCCGAACACGGCTTCGATAAAAGGCGTATAGGATTCGATATTCG
GGGTTAATACGGCGATATCGTGCGGCTGCCAATCGGGATGTTCATGCAGAATTTTCAACA
GCTTGTCTTTGAGTATCTGCAATTCGCGCAAAGGGCTGTGTGCGGAGACGATGCGTATCG
AGCCGTCGCCCGTGTTGACGCTTCCCGCCATTTCAGACGGCATTTTCAGGTTTTGAATAT
CGGTTTGCAGGGCGTGTAAAAGCGTATCGCGCCCGCCTTCCTCAAATACCGGCGTTTCGC
CTTCTATTTCCATTTCGTTCAAAAAGTCGAAAAAGTCCCGCCCCTGCTTGCCCAATGAGG
CGAGCAGCGGATGCCCTGCCTGAGTTAAATCGGATCGCCGCCACCTTTGAGGATTTGCG
CCGCTTCGATGACGTTGCCCCAGTACATCCCGCTCGGATTGAGTGCGAACACGAACACGT
CGCAATGTTCGGACAGCTTGTGCAAAAGTTGCAAATACATCGGCGCCATCGTGGAAATGC
CGAACACGAAATAACGCTCGGGCAGCTTATCACTGCTCAAAGATTCCAACAGCTTTTCCC
ACAACGCGACACGGTGCGGCGCGCTCTGCCTGCCGTCGTCGAGGTAACGCCACAGTTTGG
ACTGCCAGATTTCGTCGTCGCCCAAACCGAGCCGCCTGCCCTGCTGCCAAGCGTCTATCC
ACTGAGGACGGTACACGAGGTATTGGTCGAATATGTCCGCAAGCTGTCCCGCAAGCTGGT
AATCTGCCGATTCGCCGCTGCCCAGATAGTCTTGCAGCACATTCCTCACATCTTCAAATT
CTGCCGTATTCCGAAATGCCTCGCTGCGGAACAAATCCAGCAGCCGCCAGCGCATGACTT
CGGGCGCAAACGGGCTGAGTTCCGGAATACCGGGAATCAGTTTTTTCATCAGCTTCCACG
TCAGGCCGGCGGGCAGGCTGAACGACAAATTCGCCGCCACGCCCAAATCGCGGGCGAGGC
AGGTATTGAGGTAGCGGCGCATCCCCTGACTCTGCACAATAATCTGTTCGGGCTGTAAAG
CCGATTTCAGCGGTTTGACTTTTTGAATGCGGGCAAACAATGCCGCCAGCGTTTCAAGAC
GGTTGGATTGATACAGATAAAACATGATTTCAAACAGAAGCTGTGGTCAAGTATTCGGGA
TTATATAGCCTTTCCCCCGTCCGCCTTCAAACAAAATGCCGTCTGAACCTTTCAGACGGC
ATTTGGTCATTTAAACCATCTCCTCAAAACAGGAATCCGCGACAACAGCAGCGTATCCAA
CAGCCAAATCACGGCAATGGCAAGCAGTGAGGTCGGGAAGAGCAGTGCGATTGCCAATAG
CGGCAATGCCATCATCCACCAAACCGGCAGCTTGACTTTCTGCGCCGGCGGAACGATGCC
CACCGCTCCGGTCGGACGGCGTTTCCACCACATCACGCAGCCGCTGATACCGATAAAAAT
GACGGCAAGGCAGAACAAGACGTTCGCCAACACGCTCCACCAGCCCAGAGTCCCCATATG
CAGCGCAATGCTTGCCGCCATAAATTTGCCGAACGGGTTGTAATCGTCAAAACGGATGTC
GGCAAGGATTTTGCCGCTGTACTGGTCGATATGTACCGTGCGGTCGGCAAACGGGCTGAT
CATGTCGTAACTCATAGAATCCTGCGACAAAGTCCATACGCCGTCCTCGCCTTTGGGCAA
ATTCAACTGATAACGCCCTTTGAAACCGATTTCCCGCGCAAAGCGGTCGACGGTTTCCAA
TGTCATCGGCTCGTCAGGGTTAATGCCGTCTTTGCCCACAGTCGTCCCTGAAACAGGCAT
AGGCGTAAGCTCCAAAACCCACGGCACTTCCTTAACCTTGCCGTCATTCAATACCTCGCC
GTGGGTCGGCACGACTGAAACGGGGTTCGGTTCGACACCCCATTTACCGGCAGGGAACTG

## Appendix A

ACTCCAAGCCTGTACGAACTTGCCGCCCCAAATACCCGCCCAAGCAATACCCGACAGGCA
GAACAACAGCAAAATCAACGACACCCAAGTTCCAAACGTGCCGTGCAGATTCCGCCACCA
AGAACGCGCCCTGCCTTTTGACGGCAGCAGCATCGCCTTGATGCCGCGCCGTTTCACCCA
CCAAAGGTACAAGCCGCTGACAACCATAATAATGGTCAGTGAAGCTGCCGTTTCCAAAAG
ATAATCGCCTGCCGCACCGAGCATCATATCGCTGTGGATTTCATCCATCGTGTAATACCA
ACCCTGATTGCGCGGCATGGTACTGACCACTTTTGCCGTATAAGGATCGACCGCGACCAT
CGTTGCTTTGCCCTCATTGTTGACACGGAACACGGCAACCATATCATCGGCACGCGGCGC
AATATACTGAACGACGGACGAAGTTTCCGGATTAACGGCACTGCGTGCCGCTTCCGCCTG
AACAGACAGAGGTTGTACCGTTGCCTGCGGCACAACATGAATCCGCTCGCCCTCCTTACC
GGTAATATTGGCAAACAGCAGCATACCCAAACCCGTAACGGCAAGCAGGGTAAGAAAAGG
CATAACCAGCAGACCGGCATAAAAATGCCACCGCCAAACGGTCAGATAACGCCGGTTGCT
CTGATTGTCGGCTTCAGTTTTGATTTGTGTATCCATTAATCGTCCTTTTGAAAATAGGGC
TATCGTGATGATGCGCGATTATAAACAATAAAGACTAATTCTTTATGACTAAAGTCAAAA
TTCATTACAACAAATAGGCAGTCTGCGTTTAAAACCGGATGCCCGTTAAAACAAAAAATC
CAGATTCAATACTGAATCTGGATTTTCATAACCGATAATATCGGAAACTCAGTCAAGTTA
GAATTTGCCGCCTGACTGGTTGACCATATAGTCAACCGCAGCTTTAACCTCATCATCGCT
CAAATCGCCGCGACCGCCTTTTGCGGGCATCGTATTGAAACCTTCGATCGCGTGTTTGTG
CAACGTGTCCTTGCCTTTTTTGATGCGGTCGGCCCAATCGGCTTTGATGCCTACATGGGG
AATACCCGGAATCGCATTGCCATGGCAGGCGGCACAAACGGTTTCATAAACCTATTTGCC
GTCCGCTTTGGCAGCAGGTGCAGCTTTTTCCTCGGCTTTAGGTTCTGCTGCGGCAGGTTT
GGCTTCGGACACGGCTTGTGCTGCTTCTGCAGGAGCAGAGGCTGCGGGTTCTGCCGCCGG
TGCGGGAGTCGGTGCAGGCTCGGCTTTTTTCACCGGAGCTTTACCGTCTTTATCGGAAAG
ACCCCATACATAAGCAGTCATAATATGCAGTTTGTCTTTATCCAAGAAATGTCCCCAAGC
GGGCATTTGGCTGCTGCGACCGTTGGTAATGGTTTCGATAATGGATTTTTGCGTACCGCC
CCACAACCACACGTCATCAGTCAGGTTCGGACCCAAACCTTGGATACCTTGTCCCTTATC
GCCGTGGCAAGTGAAACAGTTGGCAGGCGGACCGCTGAACAAGGCTTGTCCGCGCGCGGC
ACGTTCCTCATCATACTGACCTTCGGGTTTTGAAAGGGACATCACATAATGGGCAACGTC
TTTCACGCCTTCTTCGCCCAAAGCAGGACCCCAGGCAGGCATAGTCGCAACACGGCCTTT
TTCGATGGTCTCGTGGATTTTATCGGGATCACCGCCCCACAACCAATCGCTATCGGTCAG
ATTCGGAAAACCTTTAGAGCCTTTAGCATCAGAGCCGTGGCACTGGATACAATAAGTGTT
AAACAGGTTTTGGGCGATTTGCTTGGCTTGAGGGTCTTTTGCCACTTTTTCAATCGGCAT
ATCCGCAAACTTGGCATACAGTTTGCCGTATTGCTCATCGGCTTTTTTGACCTCTTTTTC
ATATTGGTTATGGCTGGTCCATTTCAGCAGACCTTTGTAGTCGCCGACACCCGGATACAT
AACCAAATAACCGATACCGAACAGCCACGTCAAAACACACAGCCAAAACCACCAGCGGGG
CAGCGGATTGTCGTATTCGGCAATGCCGTCCCACTCATGACCCGTAGTTTGTACTTCTTC
GCCCTTCTTCGGACGTTTGACAACATTTTGAGACAGCAGCAGCCAAGCCAAAGCGATAAA
GCTCAGTAAGACAATAACTGCAATATATATATTCCAGAAATTACTGGTAAATTGGGATGT
TGTGTTCATTGTTTTGCTCCGTTATCACAATATTAACGGTTTTCGCTTTTCTTATCTTGC
GCATCTTGGTTTTCATCAAAAATGCTGTTTGCGGCATTATCGTAGTTTTTCTTATTCCGC
CTGTTGAAGACGATATAGAGTACCAACAGGAAACAGATAAAGATCCATACCGTGAAGAGA
GCACGAATACCGTTAATATCCATGATGTTACCTTACGTTTTTCAAAGCCAGACCCAATCC
TTGCAGATAGGCGACTACAGCATCCAGCTCGGATTTGTTTGCCAAAGCCTCAGGTGCTTT
CGCAATTTCCTCATCACTGTAAGGAGTACCTACTTTACGCAAAGCCTTCATGTTGGCAAC
GGTTGCATCGACATCGACTTTATTGCGTGCAAGCCACGGGAATGCCGGCATATTGGACTC
AGGCACGACATCACGGGGATTCAGCAGGTGGATACGGTGCCATTCGTCGGAATAGCGACC
GCCCACACGTGCCAAATCAGGACCGGTACGTTTGGAACCCCATTGGAACGGATGGTCGTA
AACCGACTCTCCGGCAACAGAGTAATGACCGTAACGCTCGGTTTCCGCACGGAACGGACG
AATCATTTGCGAGTGGCAGTTGTAACAGCCCTCACGGATGTAAATATCGCGTCCGGCAAC
CTGCAGGGCATTGTAAGGCTTCACGCCCGGCGCCGGCTGTGTTGCCGCCTTGGTAAAGGC
~~CAAGGGCACAACTTCAATCAACAGACCGACACTGACTACAAGCAGCGTGAACACAATCAG~~
AACGCCGATTTTTTCTTCAGCCAATTGTTGTAATTTCATTTTGGTAGCCTATCTTTCTTA
GTATTTTAGTGGTGCTGTGTTTGGGAAACCGCAGGGATTTCGGCATCGACTGCTTTACCA
CCGATGGCTGTGCGGTACACGTTGTACGCCATAATGCACATACCACTCAGATACAATAAA
CCACCTGCAAAACGGATCACGTAGTAAGGCATGGTGCGTTTTACGGATTCGACAAACGAG
TAGGTCAGCGTACCGTCATCGTTCAAAGAACTCCACATCAAACCCTGCATCACACCGGCA
ATCCACATGGCAGCGATATACAGAACCACGCCGATGGTCGCAATCCAAAAATGTGCTTCT
ACCAGCTTGGTGCTGTGCATCTGTTCTTTGCCGAACAGACGGGGAATCATGTAATAGACG
GAACCGATGGTTACAAAGCCTACCCAGCCCAACGCACCCGCATGAACGTGCGCGACGGTC
CAGTCCGTATAGTGGCTCAATGCATTGACCGTTTTAATCGACATCATCGGGCCTTCAAAG
GTAGACATACCGTAGAAGGACAAGGATACAATCAGGAATTTAAGAATCGGGTCTGTACGC
AGTTTGTCCCACGCGCCGGACAAGGTCATGATGCCGTTAATCATACCGCCCCAAGAGGGT
GCGAACAGAATCAAAGACAGAACCATACCCAAAGATTGCGTCCAGTCAGGCAGCGCAGTG
TAGTGAAGATGGTGCGGACCCGCCCACATATAGGTAAAAATCAACGCCCAGAAGTGAACG
ACGGACAGGCGGTAGGAGTAAACGGGGCGGGCTGCTTGTTTGGGTACGAAATAGTACATC
ATACCCAAGAAGCCGGCAGTCAGGAAGAAGCCCACGGCATTATGCCCGTACCACCATTGA
ACCATAGCATCAATCGCACCGGAATAGACGGGGTATGACTTCATCAAACCGGCCGGGATG
CTGATATTGTTGACGATGTGTAAAAGTGCGACCGCCAAAATAAAGCCGCCGTAGAACCAG
TTGGCAACGTAAATATGTTTAATCTTACGTTTGGCAATCGTACCGAAGAATACGATGGCG
TAAGCCACCCAAACCAAAGTAATCAGAATATCGATCGGCCATTCCAGTTCGGCATATTCC
TTACCTTGGGTCCAACCCATAGGGAAGCTGACGACGGCGGCAACGATTACCGCCTGCCAG
CCCCAAAAGGTAAATGCCGGCAGCCAACCGCCGAAAAGACGGGTATTACAAGTACGTTGG
ACAACGTAGTATGATGTGCCGATCAGGCCGCAACCGCCAAATGCGAAAATAACCGCATTG
GTGTGCAGCGGACGCAGGCGGCCGAAGTGGAACCAAGGTCCGATATTAGACAAGTCGAGG
GCAGGAGCAAAAAGCTGGGCGGCGACGATAACGCCGACCAACATACCCACAATCCCCCAA
ACTACAGTCATGATGGCGAACTGGCGCACCACTTTGTAGTTGTAAGTTTGTGTGTCCATG

## Appendix A

AGAGTCTCCATGAATTTATGGGAATAAAGATTTTTATCCTGCCGCTTCCGCAGCCTGTTT
AAGGTGCAATCCGGGCAAGCGTAATTTTTTCTAAATTTAACATATCTGCCTTATTACGCC
AAGCGGAATTACATTCGCACCGCCGACGAGCCCTTTGCTTAATCTGTTTTTTATTACATA
TAAATCATATTGTTATAATAAATTACAACCCGACCGCCATTGCTTTTGTTTCCAATTTTC
CCTTTTTGTGGCACTTTATTGATGTAGGTTAAGCTGCATTTTAAAGGTATTTAATCCATC
CCGTTTAACGATATATTTGATAGTTATGATTCATTATAAAATAACCCCGTCCCCTCTCGA
CCACGAGTGGCACATCCTGCTGACATTCACACAAGATGATGATCTTCCTATAGAAATAAG
CCTGCCAAACTGGGTTCCGGGCAGCTATCTGATTCGGGATTTTTCCCGCCACATCACTTC
TATCCATGCATCCTGTAACGGCACGTCCATGCCGCTCGAACAAATTGCCAAAAACCGCTG
GCATGCCGCCGCCGTACGCGGCGAGTGGCAAATCCGCTACACCGTATATGCATTCGATTT
GTCGGTTCGAGGTTCTTTCCTGACGACAGAACGCGGTTTTTTTGACGGATCGTGCCTGTT
TTTGAAAGTCGAAGGAACGGAAACGCTGCCGCACCGCTTGGAATTGACGGGTATTCCGTC
CGAATGGCGTATTGCCACAACGCTGCCGGAAACAGGGAGGTTTGTCTTTCAGGCGGCATC
TTATGCCGAATTGATTGACCGACCTGTCGAGATGGGCTTGATTGAATTTTTAGATTTTGA
GGCGGCAGGCATTCCGCACACAATTGCCTTAAGCGGCATATATCCCGATTTCGACCGCAA
CAGGCTGGTTTCGGATATCAAAAAAATCTGCGAAACAGAACTGGCGGTGTTTTCCTCCCC
TGCCCCGTTTCAAAAATATTTGTTCCTGCTCCACGTCGGCGACCATATTTACGGCGGTTT
GGAACACACCGACAGCACCGCCCTGCTCGCCGACCGCCACAGCCTTCCGCCGTACGGTAT
GACCGATGCCGACGATACCTACACCACATTGCTCGGACTTTTCTCCCACGAATATTTTCA
CGCGTGGAACGTCAAATCCATCAAACCTGCCGCGTTCGTCCCTTATGACCTCGACAAAGA
AAACTATACCGAACAACTATGGGCATTCGAAGGTATTACATCCTATTACGACGATTTGTT
TTTGGCACGCAGCCGCACCATCTCGCCCGAATCTTATTTAAACCTGCTGGCACAAGGCAT
TACGCGCGTACAACAAACCCGCGGCCGTTTGAGGCAGACCTTGGCGGAATCGAGTTTTAC
CGCGTGGAACAAATTTTACAAACCGGATGAAAACAGCCCCAACGCCATCGTCAGCTACTA
CCAGAAAGGCGCGCTTGCCGCATTGTGCCTTGATCTGATAATACGCAACCGAAGCAACGG
CAGACATTCTCTCGATACGTTAATGGACAAACTCTATCGGGAGTGGAGGGACACACACTC
GGGTATTCCGGAAAAACACTGGCAAATCCGCTGTCAGGAAATTACCGGCTTGGATTTGGA
AGATTTTTTCCAAAAAGCGTTATACAGTACCGAAGATTTGCCGCTTGCCGAATGCCTGGC
AACCGCAGGCGTGGGACTGACCTTCCTGCCGCTTCCCCGACAACACGGCGGCGGATACGC
AGAACACATCTGCCCCGTCCCGTCGGCAGGCGATTTTGGCGCACGTTTCAAACAAAACAC
CGACCACATCGTCCTGACCCATGTCTTCAACGGCGGCAGCGCGGAATCTGCGGCACTGTG
CCCGCAAGACAAAATCATTGCTTTAGACGGTTATGCCTGCACCGACTTTACCGCACAATG
GGCCCGATACCACGTCAATGCAAAAATCAATATCCACTTCTTCCGTGCCGGCATATTGCG
TCAAACCGTCTTGACGGTTCAGGCAGCGGCAGCGGATACTGCCATCCTACATATCACAGA
CCGGAACCTGTTGGACAACTGGTTGTTCGGTTAAACTTTCAGACGGCATTGCACACAAAA
TGCCGTCTGAAAAACAACCGCAAAGTAAAGGAAACAAAATGGCCATTCTGAAACTTGACG
AACACCTCTATATTTCTCCGCAACTGACCAAAGCCGATGCGGAACAAATCGCGCAACTGG
GCATCAAAACCGTCATCTGCAACCGCCCCGACCGCGAAGAAGAATCGCAACCCGACTTCG
CCCAAATCAAACAGTGGCTGGAACAAGCAGGCGTTACTGGATTCCATCACCAACCCGTTA
CCGCACGCGACATCCAAAAACACGATGTCGAAACCTTCCGCCAACTCATCGGACAAGCCG
AATATCCCGTCCTTGCCTATTGCCGGACCGGTACGCGCTGCTCCCTCCTGTGGGGCTTCC
GCCGGGCGGCAGAAGGTATGCCGGTTGACGAAATCATCCGCCGCGCCCAAGCGGCAGGCG
TAAATTTGGAAAACTTCAGAGAGCGGCTGGACAACGCCCGCGTCTGATTACAAGCCGAAA
CGTTTAAACCACACCTTCAAGCGGCATTCCACCGCAACTTGAAAAAGAGGACGGCAAACC
TTACTGCCGTCCTCTGTCCTTCTCCGTTTTTACAGTGGGAGACCTTTGCAAAAATAGTCT
GTTAACGAAATTTGACGCATAAAAATGCGCCAAAAAATTTTCAATTGCCTAAAACCTTCC
TAATATTGAGCAAAAAGTAGGAAAAATCAGAAAAGTTTTGCATTTTGAAAATGAGATTGA
GCATAAAATTTTAGTAACCTATGTTATTGCAAAGGTCTCAGTGGGTATAGCGGATTAACA
AAAACCAGTACGGCGTTGCCTCGCCTTAACTCAAAGAGAACGATTCTCTAAGGTGCTGAA
~~GCACCAAGTGAATCGGTTCCGTACTATTTGTACTGTCTACGGCTTCGTTGCCTTGTCCTG~~
ATTTTTGTTAATCCACTATAAAAATTAGAAATGCACATTTTCATTATTCTCGCGCAGGCA
GGACTCCAGACTTACCCATTTCAGTAATGTTTGAAAATAAAAGAAAAATCAGATGTTTGT
ATTCCCGCCTGCGCAGAAATGGAGACGGTGCTCTGTCGTCTCATTTTTGTTTTAATCAAC
TATATATAGCTGATTAAACATAAGAAATGCCGTCTGAAAGACTTTCAGACGGCATTCGTT
CAAGCGTCGAACTTTATTGCGCCTTGGTTTCGGTTACAAAACCGATTTTGGTGATTCCTG
CCTGACGGGCGGCTTCTAAAGCTTTGTTTACATAATCGTATTCCACCGCCTTGTCTGCCG
CAATCGCCACAATCACGTTTTCATTCTGCTCCTTGGCGGCTTTCAGACGGCTTTCCACTT
CCCCGATTTCCACTTTGCTTGCAGAATCCCCGCCGACATAATAGCCGCCGTTCGCATCAA
TCGTCAGGCGCAGGGGGTCTTTAGGCTGTTTGTCCTGCTTGTTTGTCTGCTCGGACGCGG
TCGGCAGTTCCAAAGGGATGGAATGCGTCAGCACCGGCATAGTAATCATAAACACAATCA
GCAACACCAGCATCACGTCCACCAACGGCGTAACGTTGATGTCGGACATCGGAGAATCGT
CGCCGGAATTCATCGAACCAAATGCCATAATCAGCTATCCTTTTGATTAAGCAGGCGGAC
GTGCAAATCGTGCGCCATCGCATCCAAATCCTGGGTCAGTATTTTTGTGCCGCGATTGAG
GAAGTTGTATGCCAACACCGCCGGAATCGCCACGAACAAACCCGCCGCCGTCGCCACCAG
TGCCTCGCCAATCGGGCCGGCAACCGCCGCAATACTCATCTGCCCGCTTTGCCCGATATT
GATCAGGGCGTGGTAAATCCCCCAAACCGTGCCGAACAGCCCGATAAACGGCGCGGTCGC
GCCGATGGAGGCAAGCGCGGTCATCCCGTAATCAAACCGGCGCATAATCTGCGCCATACT
GTTGCGGATTTGAATGACCAAATACTCGTTCAACGGCAAAGCCTGCGCCAGTTCGGACGC
TTCGTTTCGGCGGTAGTTGCGGTAAGACTGCAATGCCTCTTGCGCCAGTTTGGACAAAGG
CGCATCGACGGCGCGCACTTTTTCGACCGCGTCGTTCAGCGACAAAGTATCGCGCATATG
CCGTTTGACGGCGGCATTCCCTTTGCGCGCCCGATACAGCTTGATGCAGCGCAAGACAAC
CAAACACCACGTTACGATACTCATCAACAGCATCAACACAAACACACCAATCAGGACGGG
ATCGCCCGATTCAAACACTAATTTCAAATTCATAATGATTCCAACACTGAAAAAACCAAT
CAAACATCCAAGCTGCCGCAAACCGCTGCGGCAACCGCCTAATTCAATTCAAACTTGACG
GGGACTTTAAACTCCGTCCAGGCATTGGCTTGAAAATGCCCGTTTTGCGCCGCCTTGCGT

## Appendix A

GCCGCATTGTCCAACCGGGAAAAACCACTGCTTTTCACGATTTTAACGGACTCAACATGA
CCGCCCGGAGAAACCAAAACGCTCAAAACAACCGTACCCTGCTCGTCATTCTCCATAGAA
AGCGTGGGATAAGCCGGGCGCGGAATGCTGCCGTTGGCGCGTAAAGGATTGCCTTTGCTG
CTGCCGGCTCCTTCCCCGTGTTCGCCTTTGACACCGCCGCTACCTTTACCGCTGCCTTCT
CCGCGCCCCGTTCCGTCTCCTTTGGTACCAGTTCCCTTATCTTCCCCATTGCCCTGCTCG
CTGTCTGCTTTGGCAGAAGCATTGCCGGGATGTTCGGCAGGTTTTTCAGACGGCTTCTCG
ACCGGTTTTTCCGCCGGTTTCGGGACAGGCTTCGCTTCCGGCTTAGGCTCTGGTTTCGGT
TTTTCTTCGGGTTTCGGCTTTTCTTCAGGTTTCGGCTCTTCCTTAGGCTGCTGAATATCC
GCATCCGCCTTTTTCGTAACCACCGGCTTCAAAACCGGCTTGGGCGGCTCGACAGGTTTG
GGCGGCTCGGGCACGGGTTGCGGTTCGGGCGCAGCAGGCGCGCCTGCACCTTCGGGGGCG
CCGTCCCCTCCGCCAAAATCGCCCAAATCGACAAATTCAATAACATTGCCTGACTCTATC
ACGGGCAGCTTGTGCGCCTGCCAGAGCAATGCCACCATTGCCAAATGCAGCAGTGCGACG
GAAAACACGACTGCGGGGGTTAAAATTCGTTCTTTATCCATAATTCGGGCATAATAATAG
CAACAATTCCTATTTGCAACCTATTTTTACAATTTTTGGTCATATGAATGTCTGTTCCGT
TCACAGGCAAACCGTGTTTAAACGCTGTATTACAGCAAATCATCAGATAACGGGCCGGCA
GAAAAAATGATTCCGTCTGATTTCTTATTCCAATAAAATCAGGTTAGATGATATATTGCC
GCTTCTGTCTGTCAGCCGTTTCGGGCTGCACACCACATCTGTTCAAAGGAAAACCATGTT
TCAAAATTTTGATTTGGGCGTGTTTCTGCTTGCCGTCCTGCCCGTGCTGCTCTCCATTAC
CGTCAGGGAGGTGGCGCGCGGCTATACGGCGCGCTACTGGGGAGACAACACTGCCGAACA
ATACGGCAGGCTGACACTGAACCCCCTGCCCCATATCGATTTGGTCGGCACAATCATCGT
ACCGCTGCTTACTTTGATGTTCACGCCCTTCCTGTTCGGCTGGGCGCGTCCGATTCCTAT
CGATTCGCGCAACTTCCGCAACCCGCGCCTTGCCTGGCGTTGCGTTGCCGCGTCCGGCCC
GCTGTCGAATCTAGCGATGGCTGTTCTGTGGGGCGTGGTTTTGGTGCTGACTCCGTATGT
CGGCGGGGCGTATCAGATGCCGTTGGCTCAAATGGCAAACTACGGTATTCTGATCAATGC
GATTCTGTTCGCGCTCAACATCATCCCCATCCTGCCTTGGGACGGCGGCATTTTCATCGA
CACCTTCCTGTCGGCGAAATATTCGCAAGCGTTCCGCAAAATCGAACCTTATGGGACGTG
GATTATCCTACTGCTGATGCTGACCGGGGTTTTGGGTGCGTTTATTGCACCGATTGTGCG
GCTGGTGATTGCGTTTGTGCAGATGTTCGTCTGACTGGCTTTCAGACGGCATAAACGCTC
CAGAAAACGCGGCAGGACATATTGCCCTGCCGCGTTTTCCTGTAGTGTAATCTTATTTTT
TTCATCATTATTAGAACCAGGTTGCATGATAATACCTTTCATTAACTGAAACACTGATTA
AGAAACTCCAGTCTGTCTAATGATGAGGTTTTCACATCGCCAAAACTTGCCAATCAAATG
CTGGATTTATTGCCGTCTGAGATTTGGTCAAATCCAAAGGCGACATTCTTAGACCCTGTG
TGTAAATCAGGGGTATTTTTGCGTGAAATCGTCAAACGCTTGGATGAAGGCTTGACCAAT
CAAATACCAGATAAACAAACTCGCATTAACCACATTTTAAAAAATCAAGTTTTTGGAAGT
ACTGCCACGTATGTAGGTAGCTTTGACCGATATTTGCATAAAAACTCCTTTGCTGGTGAA
AGGAATTATTTTGCCAATTTTAAAATATTTCTGGCACCAAATAGTACAATGACAAAGACA
ATCATGCCAATGATTAAATCAGGATAGCTAGAATGAGTCAATAACGTCAATGCTCCCGCC
GCTATCACACCGATATTGATGATAATGTCATTGGATGTAAAAATCATGCTGGCTTTGATA
TGGATTTCTTTATTTTGATTTTTGCTCAGTAGATATAAGCACAGCCAGTTTGCAATCAAT
GCCAAAAATGCCGTGCCAATCATCAGTTGATAATTGGGCAGCTGCTCAGCACCGATAAAA
CGCCTAATCACTTCTATCACCCCAAATAACGCCAATATTATCTGCGTTATCCCCGCCAAA
AATGCCACACGTTTTTTATACGCCAGCGTCATACCAATGGCTGATAGCGCCAATATATAG
ACAAAGCTGTCCGCCAGCATATCTAGACTATCAGCAATCAGCCCCATAGAATTAGCAAAA
ATACCAACCGAACACTCTATGATAAAAAACACAAAGTTAATCATGAGCACTTGATATAAT
AATCTTTTTTCTAAGTGCTCATCAGGCTTGTTAAACACTATCTTATCAACAATCACTTCG
GTGGAAATGATATGACTATCAAAATTAAGCGGTTCAAGTACTTGTAAAATCGTTGTATCT
TGATTATCGTGATAGACGGTTAAGCACCGCCCAGCAATATCAAACTGTAATTCATAAATA
TCAGACACATCTTTTAAACGCATGCGAATGAGCTGTTCTTCGGACGGGCAGTCCATTTTG
GTAATGTTAAAAATGGTCTTTTTCATCTATTTAGTTCCTTGTTTTGATCAGGTTGGCTCA
AATAAATCTGTGTTTATATTGCTGCTTGGTAATTTTTGGATGGTTTGAGTAAATTGATTA
GGTTAAAATTTACCTTTGGAAGTACCGCCACGCATAATAGTTTAGATATGTTTATAATCT
CTGGATAAAAAAACGTAATAAGTGCTTACTGGATAACAAAGTCCAAACCAATAGCAGGCA
AAATAAGGCATCCACCCCCCTTCTTCATTAAGGATATATATTGAGAAACAAATCGCAACT
AAACAGAAAAAACTTGGGAGATAAAGCCATTTCATTCCCCTATTCAAGAATCTAGCCAAG
ATAGGTATTTTGTATTCTACAAAAAAGAAAGGCATTTCCAAGGGAAACATGTCAGATAAA
AACTTTTGTTTATTTTTTACTATAGATAGAACCTTGCTTCTCAAGAGAAAGCCATTAATA
ATACCGATGACAGCTATTAATATATAGAGAATAGTATAAGTATGAATAATCTTCATTAGA
CAAAAAGAAGAAATGGCAGATAAATTACATACGATATATTGGAATATAAAATATTTACGG
TCTAAACCTTGTTCAGTTGCAATTTTTTTAAAATTGCCTTGCATAAAAAAATCAAAGGCG
TCCATTAAACTATCTTTCACATTAGAAATTTAAAAGCTAAATAATACGACAAACAATGTG
AAGTACTATTCATGGTTTATTTAAAAAATAATACTATTCTGAACATTATTTAGATACAGA
AATTAACAAATTAGAACTAAACAAGCTTTTAAATACTTTAATTTTATTGGAAAGCTATAA
AAGGAACTATAACTTTACACACTAGTCACTTCTTTTTAAGAGGCAAAAGGGATTGGGAAG
GTCGTCTTGGAGATAAGCACTGGTATTTCGGCCAATGGTAAATAGAGTTTACCTCAAATA
GGGTAGAACCTCCTTCATCTGTCAGTTAATAACAGCCACTTTTACAATGCCCTGTCAAAA
TAAAGCGGCACGCCCGATTTTTCACTCATCGTCATCAAATAACCCATCACCTTTTGGGGC
CATTCGATGCCGCGCACCACGGTCAGATTCCTCAAAACGGGGAAAACCAAAATATCCTCC
ATACCGATTCCGCCGTTGATGCCGTCTGAAGCACCGTCCATCAAATTTTCCAACTCTTGC
AAATCTGCGTTTATCCGTTCGAGGTATTGGGCGGTTTTATTCAAATTGGCGGAAAAGCTG
CCGATGCTTTTCTCTTTTTTGTCTGTAAAATATTTCACCGCTTCCGGCGTTGCAAATTCA
GGCAGCCCGATTTTGATCACGCGCGGCTGCACCAGTTTGTCGTTGTATCCGCCCACCTTG
TCCAGCCACGCCCGTATCTCGGGGCGGACTTCGTCTTTCAGACGGTCTTCGCGGTCGAAA
TGCCGCACAATGTCCAAACTCTCGCCCATAAACGAACCGTCTTCTTTTTGCAGGACGGGC
ACTTGTTTCGCACCGATCATACCGATCGGCGTTGCCTCGTCGTCGTTTGCCAGCACGGCT
TCTTCAACGTCCGCGCCAAACAGCCCGGCAGCCATCCGCGCACGCACGCAAAACGGGCAA

## Appendix A

TGGTCGTAAATATACAGTTTCATCAAAATATTCCTCGTCAACCTGTCGGTACCGACTACC
TTAACACCCCGCGCCGCCCGAAACAAGTTTATCTTCCCGCCTATGCACCGTAAATAAATA
AGCTGTTACAATAAACTCGTTTTTATCGGAACGGAAGACCCCATCATGACCGCCATCAGC
CCGATTCAAGACACGCAAAGCGCGACTCTGCAAGAATTGCGCGAATGGTTCGACAGCTAC
TGCGCCGCTCTGCCGGACAACGATAAAAACCTCATCGGTACCGCATGGTTGCTGGCGCAG
GAACATTACCCCGCCGATGCCGCCACGCCGTATGGCGAGCCGCTGCCCGACCACTTCCTC
GGCGCGGCGCAAATGGTTCATGAACTCGACCTGCTCCCCGATGCCGTCGCCGCCACCCTG
CTTGCCGACATCGGACGCTACGTCCCCGACTGGAACCTATTGGTTTCCGAACGCTGCAAC
AGTACCGTCGCCGAGCTGGTCAAAGGTGTGGACGAAGTGCAGAAACTCACCCACTTCGCC
CGGGTGGACAGCCTCGCCACGCCGGAAGAACGCGCCCAGCAGGCAGAAACTATGCGGAAA
ATGCTGCTGGCGATGGTTACCGACATCCGCGTCGTGTTAATCAAACTGGCGATGCGTACG
CGCACCCTGCAATTTTTAAGCAACGCCCCCGACAGCCCCGAAAAACGCGCCGTCGCCAAA
GAAACCCTCGACATCTTCGCCCCGCTCGCCAACCGTTTGGGCGTGTGGCAGCTCAAATGG
CAGCTCGAAGATTTGGGCTTCCGCCATCAAAAGCCCGAAAAATACCGCGAAATCGCGCTG
CTTTTGGACGAAAAACGCACCGAACGCCTCGAATACATCGAAAACTTCCTCAACATCCTG
CGCGGTGAACTCAAGAAATACAATGTCCATTTCGAAGTCGCCGGCCGCCCGAAACACATC
TACTCCATTTACAAAAAAATGGTGAAGAAAAAACTCAGCTTCGACGGCCTCTTTGACATC
CGCGCCGTGCGAATTCTGGTTGATACCGTCCCCGAGTGTTACACCACGCTGGGTATCGTC
CACAGCCTCTGGCAGCCCATTCCCGGCGAGTTCGACGACTACATCGCCAATCCCAAAGGC
AACGGCTATAAAAGTTTGCACACCGTCATCGTCGGCCCGGAAGACAAAGGCGTGGAAGTA
CAAATCCGCACCTTCGATATGCACCAATTCAACGAATTCGGTGTCGCCGCCCACTGGCGT
TACAAAGAGGGCGGCAAGGGCGATTCCGCCTACGAACAGAAAATCGCCTGGTTGCGCCAA
CTCTTGGACTGGCGCGAAAACATGGCGGAAAGCGGCAAGGAAGACCTCGCCGCCGCCTTC
AAAACCGAGCTTTTCAACGACACGATTTATGTTTTGACCCCGCACGGCAAAGTCCTCTCC
CTGCCCACGGGCGCGACCCCCATCGACTTCGCCTACGCCCTGCACAGCAGCATCGGCGAC
CGTTGCCGCGGTGCGAAAGTCGAAGGGCAGATTGTGCCGCTGTCCACCCCGCTCGAAAAC
GGACAGCGCGTCGAAATCATTACCGCCAAAGAAGGGCATCCTTCCGTCAACTGGCTTTAC
GAAGGCTGGGTCAAATCCAACAAGGCAATCGGCAAAATCCGCGCCTACATCCGCCAGCAA
AACGCCGACACCGTGCGCGAAGAAGGCCGCGTCCAACTCGACAAACAGCTTGCCAAACTC
ACGCCCAAACCCAACCTGCAAGAGCTTGCCGAAAATCTCGGCTACAAAAAGCCAGAAGAC
CTCTACACCGCCGTCGGACAAGGCGAAATTTCCAACCGCGCCATCCAAAAAGCCTGCGGC
ACGCTGAACGAACCGCCGCCCGTACCCGTCAGCGAAACCACCATCGTCAAACAGTCCAAA
ATCAAAAAAGGCGGCAAAAACGGCGTGCTCATCGACGGCGAAGACGGTCTGATGACCACG
CTTGCCAAATGCTGCAAACCCGCGCCGCCCGACGATATTATCGGCTTCGTTACCCGCGAG
CGCGGCATTTCAGTGCACCGCAAAACCTGCCCGTCTTTCCAACACCTCGCCGAACACGCG
CCCGAAAAAGTGCTGGACGCAAGCTGGGCGGCATTGCAGGAAGGACAAGTATTCGCCGTC
GATATCGAAATCCGCGCCCAAGACCGCTCCGGGCTTTTGCGCGACGTATCCGACGCGCTC
GCCCGCCACAAACTCAACGTTACCGCCGTGCAAACCCAGTCCCGCGACTTGGAAGCCAGC
ATGAGGTTCACGCTCGAAGTCAAACAAGTCAACGACCTCCCGCGCGTCCTCGCCAGCCTC
GGCGACGTCAAAGGCGTATTGAGCGTTACCCGGCTTTAAATACAAAAATGCCGTCTGAAA
GCCGAATAACGCTTCAGACGGCATTTTGATTGCCGGGGTTTGCTATTTTTTGTTGCATAG
TCAATTAAAAACAAAATAGTACAATACTCAACTTTGAAGGTCTAACCATGGCATACTCTG
CGGACTTAAGAAACAAAGCTTTAAACTATAGTGGATTAACAAAAATCAGGACAAGGCGAC
GAAGCCGCAGACAGTACAAATAGTACGGCAAGGCGAGGCAACACCGTACTGGTTTAAATT
TAATCCACTATATTACGAACAATGCAAAAACATCAGCCAAACCGCAGCAACGTTTAACTT
GTCAAGAAACACACTTTACCTGTGGATTCGCCTTAAAAAACAAACAGGCAGCCTAAAACA
TCAAGTTACCGGTCTAAATGCCGTCAAATCGGATAGGCAAAAACCGGCTCAATATGTTGG
GCAACACCAGGATGCCTATCTGCATGAAATCGCCAAACATTTTGATTGTACGGCAGCCAC
CGTTTGCTATGCACTCAAACAGATGGGGATAACGCGCAAAAAAAGACCACCACTTACAAA
GAACAAGACCCGGCCAAAGTAACGCATTATTTGACACAGCCGGCCGAATTTTCTGACTAC
CAACGCGTTTATTTGGATGAAACAGGATTTGACCGCCACCTGTTCCGTCCCTATGCCCGC
AGCCTGAAAGGGCAAATAGTGAAAGCGCAGATAAGTGGAAAAAGATACCGACGCTTATCT
CTGGTGTCCGCACAAGTCGGCAACCGGCTGATTGCTCCGATGGTTTATCAAAATACGATG
ACCGGAGTCTTTTTTGAAGCGTGGTTTCAGCAATGCCTACTGCCCGCATTGACTCAAAAA
TCGGTGATTATTTTAGATAATGCACGATTTCACCGTATGGGTGTCTTACGGAAATGGCG
GAAAAATTGGGACATAAGGTATTGCCTCTTGCACCTTATTCACCTGAGCTCAACCCGATT
GAGAAGGTTTGGGCGAATATTAAGCGGTATCTGCGAACCGTATTGTCTGATTACGCCCGA
TTTAACGATGCACTACTGTCCTATTTTGATTTTAATTGAATATACATTTGAATTAATTCG
CACTTAATTTAAATGTGTTTTTAACTGTGCTTTATTTAAAGGCAATGAGAATGTGAAAAT
ATCGGATCAATCCCAAAGCAGCCTGCACTTTCGAAACGGGGTGCAGGCTGCTTTGGGAAT
TTCATAACCGTTTCAGCCTGCTTTATTCCGCAAATACCGTTTCCAACCCTAACCCGCTCT
CTTTCACCAAGCGCAAATAAGCCAGCATGAATTTATACCGTGCTTGAGCCAGTTTCTGTT
CTGCTTGGGCGACTTCCTGCCGCGCCCGTATTACTTCCAGCCGGTTGCGGATGCCGTATT
GTTGGCCGGTTTCGGTCGATTTCAGTTTCAAACGGCTGCTTTCCAAAACCCGTTCTTGCG
CCATGATTTGGTAACGCGCCGCACCGCTTTCGGTATAAGCCTGGCGTACGGCGAGTTTGA
TGTGCCGCTCGGTTGCGGTCAGCTGTGCTTCGGCGGCCCCGTATTGCGCTTCGGCTTCAT
GGATTTTGCCCGACAATTCTCCGCCGGTATAAAGCGGCAAATTCAACTGTACGCCGACGC
TCATCCCTTTGCCCCGATAGTGGTAGTCATTATTCTGCGCAGATGAAGTGTAGAGGTTAT
TCTGATAGCCGACATGGGCAGAAACGGTGGGATAGCGGCTGTTCTGTGCTGCCCGAAGCG
CCTGTCCGCTGCTTTGCAGGGCAAGCTGCTGCATCCGGTATTCATGATTGTTGGATAAGG
CAATGCGCTGCCATTCATCCAGACTGTAACGTTCCAGCTTGGGCAGATAGCGTGCCAACA
GGTTGGCGGTATCTATGGCCTCGATTTGTTTGCTATCCAGGTCGGTGTAGTCGTTCAACT
GGTTTTCATAGGTTTGTTTCTCAGCCAATACGGCGATTTCTTGGGCCAGGGCATTGTCGT
AACCGGCTTTGGCTTCGTGAATATCCAGCGCGGTGGCAGCACCTTTATTGAATAAAGCCT
GCGCCTGCCTTACCTGCTGGGCATAAGCCTCTTTTTCCGCCGCATGGGCGGCAACGGTGT

## Appendix A

CTCGGCTGAGTAAAACGTTGAAATAACTTTCGGCAACTTTCAACAGCAATTCTTCGCGTG
CCGCATCGAAACGCTGTTCTGCAGCCTGCGTATCGAACCTGCTTTGGCGGTATTGTGCAA
ATTTGGCAGCGTCAAATAAGGTTTGTCCCACCTGCACGCTCCATCCCTGTGTTTCGCGGG
TGGAAGAAATCGATGGCGGCTGGCGCTGGTAGCTGGCATTGGCGGATACATGGGGAAGGA
ATGCGGCCTTGGCTTGTTGTTGCCGTGCGCGCACTGCATCACGCTGGTAATGGGACGCTT
GAAAATCAGCCGAATGTTGCTGCGCCGCCCGCCATGCTTCAGGCAGCGTAAAAGCCGAAA
CGGATGGGGAAAGGGATAGTGGCAAGGTAAAAAGTGAAACGGGTAGGATATATTTGGAAA
AATAGGATTTCATAGCCGAAAATAGTTCATGTTGCAAATAGGGCGTCAGTGTCAGGCAAA
CGGAAATACCGTAATCTTGCATTATCATTAGATTGAGCAATGTCATCCGGGCAATGGTTT
CAGGCAGTCTGCATGTCCGAACCGGCGGATAACAAATGCCCAGTACGGATCCGCCTATCG
CTCCCTAAAGCTTTCGTCCAATTTGGTTTGCAGCGGGCTTAACAGATAATCCAGCACCCG
CCGTTTACCCGTTTTAATCTCCGCCGTGACATTCATGCCCGCCGTCAGATTCACTGCTTT
GCCGTCAATATTCAAGGTATGTTTGTCCAGCGACACCACCGCCGTATAAACCAAGCCCAA
CTGTTCGTGGCTTACCGCATCATGGCTGACACTTTTCACCTTGCCCGTCAGATAACCGTA
GCGCGTATAGGGAAAGCTCTCAATCTTCACCACCGCATCCTGTCCCTGTTCCACAAAACC
GATGTCTTTGTTCAATACCAAAACTTCCACGTCCATTTTGTCGTCATCGGGCGCAATCAC
CATCATTTTTTGGGCAGCCTGCACCACACCGCCCACCGTATAGGTAGCCAATTCCTGCAC
CGTGCCGTCCGCAGGCGACTGTATTGTCATCAGCTGCTGCCGCTGCTTTGCCTTATCCGT
TTGGCCGCGGTATTGGTCAATCTGTTCGTTTGCCTGGCGCAGCGCATCCAGCGTATCGCG
TTTCAGGTTCTGCGTATTCAGCACCCGATTCTGCTCCGCCTGTGCAATGGCCGCCTGAAT
CTGCCTCATCTGACCGCGCGTACTTTCCAAATCGTTCCAATTGCTGACCGATTTGCTCTG
CTGCTCCAAAAACGCATGTTCCGAAATAAAATTGTCGGCCCGCAAACGGCGGTAGTCTGC
TGTTTTCTGCTGCTCGATCGCCCCCACCGAAACCAGCTTCTGCTCCTGCGCCTTGGCCGA
CTGCAATTCCGCCTGATGGCCGCGCAAAGCCGACTGCAATTGCGCATCCTGCGCCGCCCA
TGCCTGATACTGGTGCTGCGCCAACACCTGCGCCGATTGCACATCGGCATCGGAGAGACC
TAAAGACCGTGCTTGCGCCATATCGATATGCGGCACGGTACGGCTTTCCAATGCCGCCAA
TACCGCTTCATAACGCAGTTTGGACAATTGGGCAGCCTGCAAAGCCTGCTCCGACTGCAC
CACATCGCTGTCTGTTCCCACAGCCTCCAGTTCCGCCAGCGTTTCTCCCTGTTTCACATG
CTGCCCGTCGCGCACATGTACCGCCTTAACCACCGCCGTTTCCAGCGGCTGGATGGTTTT
GCTGCGCCCGCCCGACACCGTTTTGCCCGAAGCCGCCGCCACAATATCGATTTTGCCGAA
CCAGGACCACAACAAAGCCAAAAGCGCAAACGCCATAATAAAACGCGCCGCCCATTTCGG
AGCGGCAGAGACCGGCGTATCGGTCAGTTCCAAATGCGCGGGCAAAAACGCCTGTTCTTC
CGCCGTGCGTTTGGGCGGTTTCAACTGGTCGCGCACCGCCCAAACATTGCGCCATACAGT
AATGTATCGAGAAAGAAAGGATTTCAGGGCGGAGAAAAACATAACGGGTATAACCTTGGC
AATATAGAAACAGGAAACAATATAAATATGTAAAGGAATTTTAACGGAAAGCGCGGCAGC
TGTTAAGGGAAAGGCGGGAATATTGACAAAAAATACCCAAGTCGTTACAAATATTCATTA
TTTTACTGCGTAACGCAACGCTGAAGCGCAGGCTGCTTTTGAGATGCGGCAAGGTTCGGC
AAAAAGCAGCCTGCACATTTAACCACAGGAACAACCCATGTTTACCACAAACGATTTACG
CCATTTCCTAGAAGGTTTGGCCATCCTATTCTCAATCGGCTATTGGGGCACCATGCTGCT
GTTGCTTTGGTTTCTCGTCCGCTTTGCCTATAAAAAGCCCAAACGGAACCCCGGCAAAAT
CCTTTCAGGCAGCCTGACCGCCGCCGCTATCCTGATGCTGTTTGTTTGGATTATTCCCAA
ACAATTCGGCCCGATCAAAGAAGAAATACAGGCACAAGAAGAGTGGGACAGAAAATACAA
AGAAGCCGAAGCCGTGTTTAACGAACAATGCAAAACGGCGGGGGAAAGATTTACCAGACG
GCGGACAATGTGGAAGGGATTATGCTGTTGAAGGTAGTACCTGAGCGTACCGTTTCGGCA
GATGCAAAAACCAGAGACCCGATGTGGGACAATGCGGCTTTACAGACCAGCGAAGGCGTA
AATTTTATTGCTCGTTTCCTAGGATTTTTTAGCGATGGGGAATACCGCTATGTGGATGTC
CTGCAACCCAACCATTCCGATATTATTCGGTATTCAGGTAAAGATTTTTCCGCTAAATCA
AATATTTAATCATATACACCCCGCCCGTTATGCGGTAACGTTCGAAAACAATGTCGATTC
CAAGCTGCGCAGGCACTGGGTGGCAGGTGCGACCATACGGATTATCGACCGCCAAACTGA
~~CGAAGTGATTGCCAAGAAAACCATCTATGTCTTTGAAAAAGGCTTGGACGGCACGGGTGG~~
GGCGAGAATGCCGTGGAAGTTTGCTATCTTGTGCAATAAAGAAAGACTTACTTCTTCAGA
GCCGTTATCGGATTTTGTTCTTAGCGTTTTAAAACCTTATATATTGCGTCCCTTATATAT
TGCGTCCCTAAGAAGGGACGATTAACAAAAATTAACGTCCTTTACTTTCTACAAGTAACA
GGGCTTTTTTTTGCCCGTTTTTGAGGATTCGCACCATGGAAGATAAGCAAGGGATGACAA
AGGCGGTTGCCGGCGTGATGACGGACGCGCTAGCGGACGGCAGGAAGCCGACAACCGCTT
CAAATCTTCCCCCCTTATCTAACAGGGGGGGACAGAAACCGAAACGGCAGGCAGGGTTCA
GGAAGTCTTCGAATGTTACGAAACGTACATAACGGACGGTAAAGGAAACCTGTTAGGCGT
TCCTCTTCGGCGCGGTGTATCAGATTCGGCTTTCATTGATCAAATTAGCTTTTCATTTCA
TGAAAAAACCTTTTTCGATAAATACGGCGTTCGTGTAAGTCTTTTGGAAGACGAAGATTT
TATTCGCGCCGCGTCCATGCTCGCCGAAGAAGTTTTCGGTTTCGGTATCTACAAAGAATC
CAAAGGTTCGGGCGGTCGTTTCTATGAGCGCTGTTGGTTGATGGGTTCGGAAGACGCCCT
ATACGGTCGCGTCCATTTTGGCGGCCAACAAAATACCATTCTTTTCGAACTGACCGGCAC
CGGTTGCGGCGTCGCAAAAGAAGGCTGGGAATCACGACTTTTCGCATTCCTGACTAATGC
AATCCGCCCAAAAATCACACGCGTTGACATCGCAAAAGACTTTTTCAACGGCGAATACAG
CCCGAACCAAGCCCGTGAAGACCGAAATAAAGGTATGTTTACCTGTCATCACGTCAAACC
AAAAGGCGAATGTTTGGGGTCAGATTGGGAAGAAGACGATGAAGCCAAAATGACCAAAGG
CAAGACCTATGGTATCGGCTCCCGTGAATCGTCCAAATATGTCCGCGTCTATGAAAAAGG
CAAGCAGTTGGGCGATAAAACAAGCACATGGACGCGATTTGAAATTGAATTCAAAGCAAA
AGACATCGTTATCCCTTTCGAAGTTTTGCAGAATCCGGGCGAATATTTCGGCGGCGCATA
TCCGATTTGCGAACGATTCGCCCAAAAGGCAACGCGCATACACGCGGTTAAGGAAGATAA
GGTCATTTCAGCCGACCGCTACCTTGAATGGGTAAAAAAACAGTTCGGACGTGCGGCAAA
CGGTCTGAAATTCATTTTTCCCGAATTGGACAAAGCCAAACTGTTTGAACTGATTGAGCC
GAGTCATCACAAGCTGCCCAAGTCTTTGGCTCCCGAAGCCTACGACTGCGCCTTTTTGAA
AGCTCAAGCCATTCATGAACAGCCCGCATTCAAACCGTACAAAGACCCTTACTATATGTA
CGAATATTACGAGAATCTTGAAAAACAGCTTGAACAGCAAAAACACGTCAACAATGAAGA

# Appendix A

AAGCTATAACAACTTCATTTACGACAAATTCGCAAGACTACCGATTTCATGGGCTTAAAG
TGTCTGCCCGAAAGACGTTTAATCACACAAGGAAACCAAAAAATGAACATCCAACTTCAA
GGCCACATCGTCGGCGTTAAAAAAATCAACGGACAAATCGAAGGCAAGAGCTTCGACTAT
TGCTGCCTGATTGTCGCCACACCCTTAGACAGCTCCCAAGGCAACGCATTGGGCAGCTCT
ACTACTGAATACGATTTCGGCGGCTCTGCCAATTTCGAGCAGTTCCGAAACGCCCAATTT
CCGATCGAAGCAAACCTGAACGTAGAAATCGTCACTACGGGCAAAACCCAAAAACTGAAA
GTCATCGGTTTTCAACTCGTGAAGAAAGGCTGATTGAATGCAGAAAGTCTATGTTGTCCA
GTCCGTATCAACAGGGGACTTTCTGTATCTCTCTCCTGAAACGGGCGACATCGGACATAC
CAAATTAATCACCAATGCCGATTATTTCTACGACTTCGAAGAAGCGATTAACGCAGGTTT
GGAAGAAATCGGCAACCAATACGAATTTGTCGTATTCGGATTTTTGAAAGACTGATTTTC
GGATGTTCGGCGGTCGTCTGAAAAACGCTCCATCCATTACCGCCAAACACTTTTTGAAGG
AAAATATCATGAAATTTATTAACACCTGCCGTAAATACGGCGCAAAACTGGCTGTTGTAA
CAGCTGCTCCCCTGGCTTTGGCCGCACATGCAAATGCAACGTTGCCCGATACGGCAAAAA
ACGCTTTGGAAGCCGCAAAAGCGGACGGTATGGAAGCCGGTTGGATTGTAGTGGGCATTT
TCGCCGCGCTTTTTGTATTTTCCATCGTTAAGAGAGTGATGAAGTAAGACGGCATGTACT
ACCAAGTCGGAAATAAATGTCTTGAGAAGCACCAGGCTGAAAACCTTTATTTCAGCTTGG
TAGTACCAAGAATCAAAGAAAACGGACAGATTGTCAGGCCGGAATATAACGGCAGCCTGT
GGAAGATGTCGGACGGTCAGCCGCTAAGGCTTTTATTGGCGGAATGCAGTCCGAAAGACA
ACCTGCAAAGCGGTCTTGAAACAGGCTGGATAGTATTCGGCATCCTCGCGTCCGTTTACT
TTGTTTCCCTGCTGAAAAAGGTTTTGAAATGATGGATTTTTATTTTTATCTCGGCGTTTC
CGTACCCGTATTAATCGGGGCGGTTCTGTTTAAGAATTGAGCGCATGAAGTTATGGTGTC
AAAATCAGGCTTTTAATTAGACATTTGAGGCTTGAAACCATGAATAAAAATGAACGTGAC
TTTTTCTATATATCAAATTCTGATTTAGATAAATTGTCAGAATCTTATCCTGATAGGCCT
CTTTCTTATGTGTTTTATTGTTATTTGAAAGAAACTGGTCTATTGAAAAATTTCTCAATG
GATAAATGTCATAATTTTTTTAATAGAATTAATTTTAATGAATCTTGCTTTGAAATTAAA
TTCAAGGATGATTCATTTTTCATTATTGGCAATGGAAAAATTGATGTTTCGGATTCTAAT
AATTTCTTTTCTGTTTCTTTTGAGTGCTAAATCTTTTTCAGCAGATTTAGAAATTAAAAA
TGGGAAATTGATGTATGCACTTTCGGAAAAATATAACGATAATGGATTTAAGGCATACAA
AGTTTTAGGTGAGGGAGGAGGAATTCATACAGAATATAATTACAAATTTGATAAAAGTTT
GAATTTGAATGTATTAGAAAGTTCAACAGGCGCACGCTCTCTTGAAAAAGTCCCCGTTAA
AGTAACTGCATCAGTTTCCCGCGCCGCCGTCTTGTCAGGAGTCGGCAAACTTGCCCGCTT
AGGCGCGAAATTAAGCACAAGGGCAGTTCCTTATGTCGGAACAGCCCTTTTAGCCCATGA
CGTATACGAAACTTTCAAAGAAGACATACAGGCACAAGGCTACCAATACGACCCCGAAAC
CGACAAATTTGTAAAAGGCTACGAATATAGTAATTGCCTTTGGTACGAAGACAAAAGACG
TATTAATAGAACCTATGGCTGCTACGGCGTTGACAGTTCGATTATGCGCCTTATGTCCGA
TGACAGCAGATTCCCCGAAGTCAAAGAATTGATGGAAAGCCAAATGTATAGGCTGGCACG
TCCGTTTTGGAATTGGCATAAAGAAGAACTGAATAAATTAAGTTCTTTGGATTGGAATAA
TTTTGTTTTAAATCGTTGCACATTTAATTGGAATGGCGGAGATTGTTTGGTCAATAAAGG
TGATGATTTCAGAAATGGGGCTGATTTTTCCCTTATTCGCAATTCAAAATACAAAGAAGA
AATGGATGCCAAAAAGCTGGAAGAGATTTTATCGTTGAAAGTCGATGCCAATCCCGACAA
ATACATAAAGGCAACCGGTTATCCCGGTTATTCCGAAAAAGTAGAAGTCGCACCCGGAAC
AAAAGTGAATATGGGTCCCGTCACGGACAGGAACGGGAATCCCGTTCAGGTTGTCGCAAC
ATTCGGCAGGGATTCGCAAGGCAACACCACGGTGGATGTTCAAGTAATCCCGCGTCCCGA
CTTGACCCCCGGAAGCGCGGAAGCACCGAACGCACAGCCGCTGCCCGAAGTATCGCCCGC
CGAAAACCCCGCAAACAACCCGAACCCCAATGAGAACCCCGGCACGAGCCCCAATCCCGA
ACCCGACCCCGATTTGAATCCCGATGCAAATCCCGATACGGACGGACAGCCCGGCACAAG
ACCCGATTCCCCCGCCGTTCCGGACCGCCCAAACGGTAGGCATCGCAAAGAAAGGAAAGA
AGGCGAAGACGGCGGGCTTTTGTGCGATTATTTTCCGGAAATCCTAGCCTGTCAGGAGAT
GGGCAAACCTTCAGACGGCATGTTTCACGATATAAGCATACCGCAGGTTATAGACGATAA
AACATGGTCTTCACATAACTTTTTACCGTCTAACGGCGTATGTCCGCAGCCGAAAACCTT
TCATGTTTTCGGTAGGCAATATCAGGCAAGCTATGAGCCGTTATGCGTGTTTGCCGAAAA
AATCCGTTTTGCCGTACTGCTCGCCTTTATCATTATGTCGGCTTTTGTCGTTTTCGGTTC
GTTGAAGGGGAAATAAATGCCATTACTTGCCGGTCTGATTCCACTTTTAGGCATACTTCT
GAAAATGCTGATTGTCAGAATAATCCTTGCAACAGGTCTGACATTTGTAACCTATGCCGG
GTATCTCATCGCGCTGGAAAAGTTCAAAGACTACACGTCAAATGCGATCAATTCCATGCC
TTCCGACATACTGAACCTTCTTTTAATTTCGGGATTCGGTCAGGGGTTGGGCTACCTGTT
CGGCGCATTCTCGTTCTTCATTGGTATGCACGCATTCAAAAAACTGACGTTTGTCTTTCC
AGGATGAGGTAGAAGCATGATTTATCTGTTTACAGGAAACATGGGGACAGGCAAAACCTC
CCGCGTCGTCTCTATGATTTTGAACAACGAAGACGGATTGTTCAAAATGAAATTGGAAGA
CGGCACAGAGGTAGACAGACCGCTTTATTTCTGCCATATCGACGGATTGGATAAACGGCA
GTTTAAAGCCCACGAACTGACGGAAGAGCAAATCATGTCCGCCCCGCTTCGTGATGTCAT
ACCGGAAGGCGCAGTGCTGATTGTTGACGAAGCGCACTACACTTATCCGGTACGCGCGGC
AGGCCGTCCCGTTCCGCCTTATATTCAGGAACTGACAGAACTCCGCCATCACGGGCATAC
CGTTATTTTGATGACGCAGCACCCGAGCCAACTTGATATATTCGTCCGCAACCTTGTTTC
AAAGCATGTACACCTTGAACGCAAGGCAATCGGAATGAAACAGTATTATTGGTATAAATG
CGTAACCTCGTTGGACAATCCCGCAGGCGTAAGCGGCGTAGAAGTCGCAAGTTGGAAACC
GCCGAAAGAAGCCTTTAAATACTATAAATCAGCAAGCCAGCACCAAAAGTTCAAGAAAAA
AGTACCTTGGGCGGTTTGGGCGTTGATTGCGATTGTAGGGTTTGTAGGCTGGAAAAGTTA
CGGCATTTTTAAAGTTTACAGCAAAGCCACAGACAGCCGGATTGAGCAGGAAGCGCAAAA
AGAAAGCGTTGTGCAGACGATGACGGAGCAGCCTGCATCATCAGAGGAAATGCCTTTAAA
AAATTCAGACAATTTGAAACCTGAAGACTTTGTGCCGACTTTACCCGAAAAGCCCGAAAG
CAAGCCTATTTATAACACAGTCCGACAAGTAAAAACCTTTGAGCAAATCGCCGGATGTAT
AGACGGCGGAAAATCAGATTGCACATGCTATTCAAATCAAGGAACACCCTTGAAAGAAAT
AACAAAGATAATGTGTAAAGAATATGTGAAAAACGGGTTGCCTTTCAATCCTTATAAGGA
CGAACAGCAAAGGACGGAACAGGTGGAACAGTCCGCGAAAGCGGACAAGCCGCAAGTTCT

## Appendix A

CGTAATGGGCGGAAAGCCGTAGCAAAATCTCATGTACGACAACTGAAGAGCGCGGAAAAC
CGTTTGAAGGAATTGGCGGCGGAGTCGTAAAGCAGAAAGTTCAATCCCTACCCCTCAGGA
TGGCTTGAGCTGAGTGAAGGGGGTTAATTGCTAGAATGGCTGTTTTTTTTAAAGTGTCTC
AGTCTGGAATCGCTTCGTTCGGGGGTTGTAGGTGCAGGAAAATAGGGCAGAAAAAAGGAA
AAGGGGGAAGCTTTGTAAAGATTGGGCGCGCTTTTTACCCAATCTTTATGAATACCCCCT
TTTCCTTTTTTATGAACTGTTTTTCAATACCGGAAACCCCCGAACGGAGTGATTCCAGAC
TGAGATACGCCCAAAAAAAATCAGACATTCGGGTCGCAACAGAAACCTTTACCAAAACCT
GCGACCCCAATAAAATCAGATACGGCAAAGGCGATAAGCTTCAAGCCCTGAATGAGTAAA
TCAGCCCATTGAGGGCTTGGCGTTTGACGAAACACCAAGTAAAGCCCACGACTTCGAAAG
TACGGCCAAAGCGTACAGCTTGTAAGAAAGATAGAAGCGTGGGCTTTCGTACATCTTAAG
TTTGAACACTATCTAGGGCAAAAAGCCCGAATTAATAAGGTTAAACCATGTACTTAGGAA
TAGACGTTTCAAAGCTCACAATAGATTGCTGTTTGATTGTAGACGGTCAAAATTATCAAA
AGAAGTTTCAGAACAACAAAGGAGGATTTGAACAATTAATAAATTGGCTACAAAGTCATA
AAGTAAACGATAAGCTCCATTGCGTGTGCGAAGCAACAGGCACATATTACGAAGCATTAG
CCGAATATCTTTATTCAAGATATACAATTACCGTAGAGAATCCACGAAAGATAAAAGGAT
ATGCGATAGCAGAACTACAACGATCAAAAACAGATACACAAGACGCAAAGTTGATAGCCC
AATATTGCCAAGACCGAAAGCACAAATTAAAAGCATGGAAACCGCCGACAAAAGAACAGA
AGCAATTACAGGAAATCGCCCGATATTTAGACTATCTGAAACAGCAACGCGCAACAGAAA
AAGCTAAACAACACGAAGCACCCGACTATATCAAATCCCATATTCAAACAACTATTTCAA
ACCTGACAGCACAAATACAGATAGTCAAAAAGCAATTACTCCAGTTCTACAAAGACAATC
CAAGTTATAACAATCTACGCAAAAGGCTGAAAACAATAACAGGCATAGGCGAGCAAGCGA
CAGCAGTATTGCTATCAACCTATAAAAGACATGAATTTAAAAATGCAAGACAGTTCACGG
CTTATCTAGGCCTAGACCCTAGAAAATTTCAATCAGGAACAAGCGTGAACGGAAAAAGCA
GAATATCAAAAATAGGAAGTTCGGAAATAAGGAAAAGCCTTTATATGCCTGCACTTGTTG
CATATCGTTGTAATGCCTTCCCTGAATTTGTAGGGCGTCTGAAAAATAAAGGGAAGCATA
TAAAATTGATATTAATTGCCATCATGCGGAAACTGGCGGTAATAGCGTTTACGATTTTGC
AAAACGGCCAAGATTTCCAAGTGGAAAGATATAAATAAAAAATTAAACTGGGCTTTCGCC
GGTGATTTTCAATTTATTGAAAATAAAGTATAAATTAAAACATCAAATCCATTCAAAACG
AAACAAACATCCCGAAAAAGTCGGGGTGCGCATTCTTGCAACTTCAAGAAATGTAAAGTT
ATTTGACCGTGAAATACACTATCTTTTTTTCAACAAGCCACCACAGCAATCAGACAAAAG
CAACCCACCGCCACACCCATGTCGGCAGTACGGCCGGACAAACCACCATCCGAAGCGGCG
GGGATACCACCCTCAAAGGTGCTCAGCTTATCGGCATAGGCATACAGGCAGACCCCCAAC
TACAACCCTGACGACTATTGGTGGAACCGATATTAACTGACCCCCAAAAGTTGGACAGTT
TAATCAAGCGGCTTTCAGGGACTGAATTCTGTACTGAACAGGGCTCAGTCCTTTTAATTT
CAACTTGATTCTATCGTTGTTGTAGTAACGGATATATTCGTGCAGTACAGCTTCCAATTC
GGTAACGGAATCATATTTGCACGTATGGAAACATTCCGATTTCAACGTTCCGAAGAAACT
TTCCATTGCCGCATTGTCCAAGCAGTTTCCCTTGCGGGACATACTCTGAACCAGACCGTT
GTCTTTCAACTGCTTTTGATAAATATCATGGATATGCCGTTTCAAATCGGCATATTTGTC
TTCTGCCGATTGGACAACCAATTGGTAATAGAAGGTGCCGCGTGGCAGTCCGACAATCAC
CAACAGCAGTTCAACGGATGGCATTGCCTTAACCCTGCGACGAGTTGCGTTCTTTCTACC
GCACTTCTTTCCCATAGATTAAGGCATCGAGCTTTTTAGGGCAGCCATTTCCGCTTTAA
GGCAAGCCAATTCCGCAAGCAGTTCTTCCTTGGTTTTCAGATAGTCGGCTTTTTCGTTTC
CGGCGGATGCTGTTTTTTCACGGGCTTTCTTCCTTTGGGTTTAGGGTTTGGGCTTTAAAC
CGTTAATACCATTCAAATGGTAGAGGCGCAACCATTGCAGCAAGATGGAGCAGTCGGGCA
AATTCAGTTGGTCTGCGGCAGCTTTTTGGGACATTCCCTACCCCGCCACCAGGCGGATTG
CCTCAAGTTTGTATTCGACCGAATATTTTGTCGTATGCTTTCTACGTTTGATGCCACTCT
CTCCGTGTAATCTGTATTTTGTCACCCATCTGCGTACCAATGAATCGGAAATAGAAAGAT
GGTCTGCTGTTCCCTGCCAAATAGTATTGAACGACGGCAAGTCGGAATTCATCTGAATAT
TTTGCCATAAAAAACTGCACCCCCTAAAGTCGGTAAGGTGTCCAACTTTTGGGATGCAGT
TCAGAAGCGGTCTTTTTTTGCCTGCCGGTTTTGAATCATCCTCCGTGTATATTCCCTTGA
CGAAAAAAATGATGATATTACGGATACCAAAACTAAGGTCGTATCCGCCCCCCTACTCTC
CCTAAGCAAAGAGATGAAACAGCGTATCGGCTCCCTGCCGGTTGAATTTTCCGAAAAAAC
GCGACGTAACCAGCATCAACATATATAAGAACAGCACAAATAGCATCAATACATCAGGCA
ACGAAAATGCAGAATAATGCACTTAATGGTGTTTGGATATCTGTTGTTTTGTGCTGTTAG
TAATTCTTCTTTCTGTGTTTACAGTTTAGCAGTTGTACAGTTTTACAGTAATGTTTAAAC
AATGACTGATTTATTTTAAATGCAGATATTGTAGAGGATAAAAATGGCCAAAGTCCTTTC
AGTAACATTTTTGATTTTTTAGCGAGCCTTCTCATTTCCCCGGCGAGATCGGCAATGGCA
GCGGTACTTTGGCCGCCGATATGCTTAAGTTCAGTAACCTTACGCCACCAAAACCCTTGC
TAGCTAAGGGTTAAACAGCTCACTTGAAATCTACTTAAGTCTAATCTAAACTATCCAATA
TGGATAGATTTTTAAACATAGGGCAAGCAGCAAAATTATTGTAGCTGAAAGCACAATCAC
TCGCTGGTGGTCTCAAACACGTGCCGACTACCTCGCCGAAAACACTATCAGCCGCGATAA
ACCGTGGGAAAAGCTCGTTATCAGCCGCCGCACTTGGTACTATCGCGGGAAACCGATGCT
GTCTGAAACGCAACAGGAGAAAAATAATGAGCCGTTACCTGATTACCTTTGATATGGATA
CCAACTGCCTGAAAGACAATTACCACGGAAATAACTATACCAATGCCTACTCCGATATTA
AAACCATCTTGGCTAGACATGGATTTGAGAACATTCAGGGCAGTGTTTATCTAGGCCGTG
AAGGCATCAGTGAAGCACACGGAACAATAGCCATTCAGGAACTGACCGCTCGGTTTGATT
GGTTTTACTCCTGTATTTCAAACATTAAGTTTTACCGCCTTGAAAGTGATTTGAACGCAC
AATTTATCGCTGATGGTGTGTATCAAGCCAAACAGGCTTTCCTTCAACGTGTTGAACAAC
TTCGTATATCCCTAACAGAAGCTGGATTGTCTGATGAGCAAATCAATCAGGTTCTGGAAA
AACAGAAATTTGAATTGGAAAGTCCTAACCTGAAATTAAATTAACCTCCTTTACTCACCA
ACATCCGCCGCAGCTCTGTCAGTTTTTGGCGCGCTGCGGCGATTTCTGTGCGTTTTAGAG
CTTCGGGTAGGGTGTGAAACAACTCACTCGAAATTTACTTAAGTCTAATCTAAACTATCC
AAGCAGTAATTAGTACAAAAAAGGCAAACTTATTTTAGGAGTTTAAAATTGCAGCTGCGA
TAAACCGTGGGAGAGTCTCGGCATTTCCCGCGCCACTTGGTACAAACGTGGCAAACCGAT
GCCGTCTGAAACCGTACAACAGGAGACAAAATAATGCTTGCTCAAATCGAACTCACACCC

## Appendix A

TGCCAGCTAATGGTTTACGTAACTTAAGGTTACTGGATTTACGCACTAAGGTTACTGAAC
TTAAGCATATCGGCGGCCAAAGTACCGCTGCCATTGCCGATCTCGCTGGGGAAATGAGAA
GGCTCGCTAAAAAATCAAAAATGTTACTGAAAGGACTTTGGCCATTTTTATCCTCTACAA
TATCTGCATTTAAAATAAATCAGTCATTGTTTAAACATTACTATAAAACTGTACAACTGC
TAAACTGTAAACACAGAAAGAAGAATTACTAACAGCACAAAACAACAGATATCCAAACAC
CATTAAGTGCATTATTCTGCATTTTCGTTGCCTGATGTATTGATGCTATTTGTGCTGTTC
TTATATATGTTGATGCTGGTTACGTCGCGTTTTTCGGAAAATTCAACCGGCAGGGAGTC
GATACGCTGTTTCATCTCTTTGCTTAGGGAGAGTAGGGGGGGGCGGATACGACCTTAGTT
TTGGTATCCGTAATATCATCATTTTTTTCGTCAAGGGAGTATATCGACTCTAGAAGATAG
GTATTAGATACTGCCTTTTCTTACAAGAGTGATGGTAGGATGGTTCTCTTCAAGTCAATC
AAACAGGAAAGTATTTCTTTTCTGTCTGAAGATTTGAAAAAGGACTGGATGTTTCACAAG
GTTAAAACTGGGGAAAAAGATGGATATGGTTCAGATGAAATGCTGAGCGTACCCCGTGTC
TATTTGGAAATGATGTCGCGGAAAACGGGAGTCCCCTACTCCAGTATTCTTTAAATTCTA
AGCAGAAGACTTCTTCGTCGGTCTTTTTTTGTTGTTTGGTTTGCATGGAGTAAAACTGTA
CAACTGTTGAAGCATAAAACCAGCAAGAAGGTAAAAAAGAAAGAAGCAGTTCTTTGGATT
TTAGATATTACCGCAATTAGTTTCCTTTCCTAAAATTTGTTTAAATTATTTGCAATATTA
ATATAAACGAGATATTAATGATGAGAAATCAAAAAGGCATAATGAATATATTTTGTACAA
AATATTTGCAGTATTTAAAAATGTTGGTTCGTATATGAAAAGTTAAAAATGCCAAAATGT
ACAGTTGCTAAACTGTAAAACTGCTAAAGCAACAAAACATAAAAAGGAATGCAAGGATGC
GATCACTACATCTTTTTATTCCGAAGCGTTTATGATTTTACGGTCAACTGCTACTCTATG
TGCCTAGCTTTTCAGCTCCCTATTTTCGAATATTGGAGGAGGCATTTTCATCAGTGTCGT
AATGCCGACCAAAACTCTCACAAACCATATTGGTTCTTGTGGCAGCAACACCTATCCGTT
TGTTCAAGCGACCACAAGAGTAACATGATTGGCTGGTAGCATTGGCTTTAATCTCTTCGA
TATGAACTCCATTTTTAGCTGCACCTTCTTTCAGCATATGCAGCAGTAGGGATGAGGCAA
CTATCTTCTGGTGGAGTTTGTTGACTTTAATCTGTATGCACTGTAGGTTGAGCAGATTCA
ATTTTTTGATACAGATTATCCGGCTTTGTTCGGCAATTCTGTTTGCGAACGTATAGTAGA
GCTGTCGTCTTGCAGCTTTCAATTTGCGGTAGGTATTTTACCATTCAATGCGTAGCCGCT
CGGTACGTTTGAGCCAAATGTTATCTTCGCCATTTGTACCAATTTGTTTTTACATTAGGC
TGTGTTTTAGTAATCTATTGATTTCAATTATTTGCAAGGGAAAAGACAATTATTTTCCGG
TTAGGAATAAACCTATCCTGTTGAATACCTTAAAGCCAAATACGCCTATCAACACCATAT
TAAAACACAGCCTTTTTTAATATAGTAGACACAATCTTTCCCTATTTATGAAGGTGATCG
TTTCTTTCAGATTCGTATTTTAATGCTTTCTATTTCTATAAAAATTGACTAGAATAGCTC
AATTATAAAAAATTGCGCGATTTTGGTATTTATCATGAAAATTTCCAGACCTCCGGAATT
TACCCTGTTGCAACAGGAATATATGCAGCATCTCACTGAAAGAATGACGCAAATTGCCAA
GCTGCTGAATTCTTCCGCAAACAATCCTGATATAGACATTCCCGATTTTCTTACTGAAAT
CAAAGATTATTCAGAATTTTCCGTGACAGATGAAAATGGAACCTACCTGCATTGGGACAA
ATTCCGCCGGATTCACACGGAAGATACGCGGATGAAATGGCGCGCCGTTAAGGAAAGCCG
CAAAAAAATCCAAAAACCAATTGATTTCCCGTTTGAACATCAGTTTTGGTTCTGCATTCC
CGACTCTTTGCAGGCACGGCTTCATTTGATTGACAAAAGCTGCGGCAGTTCTATCGGCAC
GTCTAGCTTGGGTGGCTTCGGCAGAAGCGAGCAAAACAGATTCTTGCTCAAGTCTCTGAT
TATGGAAGAAGCGATTACATCCGCCCAACTGGAAGGTGCGGCTACCACGCGTAAAGTGGC
CAAGGATATGCTCAAATCGCAGCGTAAACCCAAAACAAAAGACGAAATCATGATAGTGAA
CAACTATCACTTGATGAAAAAAGCGGTAGAATTGAAAAATACGCCGTTAAGTGTTGAAAT
GATTTTGGATTTGCACCGCATTGCTACCAGTAACGCTATTGAAAACAAGGCCGAGCCCGG
ACAATTCAGGCAGGATGACGAAATCTTTATCGCCGATATCAATGGTAACAGCCTGTATCA
ACCACCGCCGCACGGACAGGTTCATACGCTGATGGAAGAGGTGTGTGCGTTTGCCAATAA
TACCTATGACGGCGTGGAAAATCCGTTTATCCATCCGGTTGTCCAAGCTATTATCTTGCA
TTTCCTCATCGGCTACATCCACCCATTTGGTGATGGCAACGGGCGGACAGCGCGGGCTTT
GTTCTATTGGTTTATGCTCAAAAACGGCTACTGGCTATTTGAATACATATCCATCAGCCG
~~TCTTCTGAAAAACGCTCCTGCCCAATACGCCAAATCCTATTTGTATGCGGAAACTGAGGA~~
TTTAGATTTAACCTATTTCATCTATTACCAATGCGATATTATCAAGCGGGCGGTTGCCGA
TTTGGAGCACTACATTTCCGACAAACAAAAACACCAACAGGAATTCAAAGCAGCGATTGC
CCAATATACTGAAAAGATAGGAAAGTTGAACCAACGGCAAATTGGTATCCTGCAAAAAGC
AGTGGAAGAAAGCGGAAAAATCTTTACTGCACAAGAAATTGCCAACCAATACGGCATCTC
CCTGAATACTGCCCGTAGCGATTTGAGTAAACTGGGAGAATATAGATTCCTAGTGCCGTT
CAAATCAGGAAATGCTTTAGAGTATGTTGCTCCTCAGGATTTATTGGAAAGGTTAGAAAA
AAAATAGTTTGCTAGCCCAGAATGCAGCTTTAACCGAGTCAAAATCAATACAGTCCGCAC
CTTCAAAAAGAAGCTGCGGACTGCTTGCTTTTTGCTCTACAAATGATCTTTGTAGCTGAT
TTAACCAAGATTGTAGCAATTTTGCTTTCCAAGCAAGCAGGGTTAGAAAATTCGATACTT
TTAATTATTGGCTGTGTTTTAATATGATGTTGATAGGCGTGTTTGGCTTTAAGGTATTCA
ACAGGATAGGTTTATTCCTAACCGGAAAATAATTGTCTTTTCCCTTGCAAGTAATTGAAA
TCAACAGATTACTAAAACACAGCCTTACATTATTGGGGTGACTATCCTGTAAAATATGTC
CTAAAACGTGGAAACCACTTTTGCTCTGCTAAATTTTAAGGAATCTTTATGTTACATATA
CCCCCCAACGGAACCTGTTCGATATGTTAGCAGTATTGTAGCCTTAAACGTGCATAGTCC
TAACGGTACAGGCGACTGGCATAGTGCAAAGGCATTGAGTGATCGGGCTTACCCTGAAAA
ATTTTATATTTACGGTGAAAATCAAGAGCGAAATACCAATCATTTATTTGGTGATAATGG
CATTATTGATGGGACGGATCGACTGAACAAAATGGGTTATTTCCCTGAAAACATCCCAGT
TTGGCTCGCAGATCACCCCCGTGCTTGCGTGGATTATCTTTACACAGCAGTGTTACAAAC
TGGCTCAATCGGTCGGGTGATTTTAGATGATTGGTTTCCAAGCGATGAAGACAAGCAATC
AGTTTATGACTTACTTAACCAAATCGAACCGCACTTGAATACTCAAGAATGGGAGAATTT
ACAGCTATGGAAACGCAAAAACCCAATAATGTAATGCTAACCGAACGAGATAAAAGGCAC
GAGAAGCGGTATTACTGAATTTCATCCGCAATACGCCATGTACAATTAAATCAGCAGATG
TGTCTAAACGTACCGCTGTTCCCGTTTGAAAGAATTTTTCATGATGAAGTCTATCCTCAC
CGTATCCGGAAATCGTATGCGTAAACCCAGAATCACCTATTTGGATGTTTGGGCAAACGA
TGAAAGAATCGGTACTTTGGAAAAGGGGGCCATGTATCGGTTCGCATACGACAATCCCAA

## Appendix A

TTCTTCGTTGCTGGGCCTGCATTATCAAGACAGAAGCAAGGTATATATCAGCAACAATAT
GCCGCATATCTTTGCACAGTATTTTCCGGAAGGCTTTTTGGATGCACACATCACAAGCAA
ATATGCTTTTCATGATGCGCCTTTTGAAGACAATGAGATGCTGCGCTTGGCAATTCTGTG
CAGAGAGACTTTGGGTCGGATACATGTGCGCTGTAATGACCCGCTTTTTAATGAATGGAT
TGACGGGTTGGAGATGAAAAATCCAAGAATATTGACTGAACGGGATTTGCTGGGCATAAA
TGCCCGACAGGTTTTTCAGCAATATATGGCAGAAATCTTCCATCACGGCCGTTTCGTCAG
TGTATCCGGGATACAGCAGAAGATGTCCTTAGATGCCATCCGCAGAAATACCAAGCAAAC
TGCCTCATATATTGCCAAAGGTTTTGATGCATCCGAATATCCTTGCTTGGCTGCCAATGA
ATTTTTATGCATGCAGACCATCAAACAAGCCGGCATTGCCGTTGCACAGACCAGCCTGTC
GGAAGATTCATCAGTCTTATTGGTACGTCGGTTTGATGTCAGTGAACAGGGTTATTTTTT
AGGGATGGAAGACTTTACCAGTCTGCGCCAGTATTCGGTAGAAGATAAATATAAAGGCAG
TTATGCGGCTATTGCACAGATTATCCGACAGATATCCGGCAGACCAGATGAAGATTTAAT
CCATTTCTTTAATCAGCTTGCTGCCAGTTGCATATTGAAAAACGGCGATGCACACCTCAA
AAATTTTTCAGTACTCTATCATGACGAATACGATGTTCGTCTTGCACCTGTCTATGATGT
ATTGGATACATCAATATACAGGGTTGGAACACAAGGAATTTTTGATGCTTATGACGATAC
GCTGGCATTAAACCTGACTAACCACGGTAAGAAAACATATCCTTCCAAGAATACATTGTT
GGATTTTGCTGAGAAATATTGCGATTTGGGAAGAGAAGATGCATCCTTTATGATAGATAC
AATCGTTCAAGCTAAAGAACAGGTTCTTGTTAAATACTCGGATGTATTGCGTGAGAATGA
ATGGTTGGCGCAGAAGTGGCATTTTATCCCGGATGAAAATGAAGAAGGTCTACCGTTTAC
ATTCCGGTAGCTGCCGCTGTCAGAGATGGCCGGTCTACTTTCACCCTGAAAATCACTTCA
TCTTATGGTGTTTGAAACCGAGAAATTAGAAGAATCGTATTCGGTAGGAGATATACTGGG
AAGATTGGAAAACTGGTAAATCACTCTATTGATTGAGTTGGCGGCCTATATGTTTAATGT
AGGCAAACGAGAAGGAATCATGTATTTCATGATTCCTTCTTAAATTCCTGTGTCAATCTA
ATATCAAAACACAGCCATCTCTTACCATAATCATGATAGGTGTTTTATTATGAAAAGCTA
TATCTATAGTTACCGTTGATTTGACTATGCCGTTTTAAAACGTATAGCCTACCTGAAAAC
CGCTTGCCCATTTCCTTGATTGGAAAAATTCGGGCTTTTTCAATGCGCGGCCGGTAAATA
TATCGTAATGCAGGTTGCCGCCAAGCTTTATCTGCCCGCGTATCCCAATTGCTGTGCCGA
CTAGAGTTTGGCCCGATAACCATTTGGCGGATTGTCCTGAAACATGTCCTACATCAGCCC
CAAGATAAAGCTGATGGCCTGGTTTAAATTGCCAGCTCAAATCGTTGCGCCAATACCATC
CCCGCTCGGCAGACAAACTCATTTCACCGTCGAAGCCACGTACGGTGTGGTGTCCGCCGA
TAGCCAGTTTGTCTTGCGATGTTAGCGGGGTTTTGTTCCATTGTGCATGAACGGATGTGT
CATAGGCAAATAGCTGTTTACCGATTTGAAAAGGAGTATTTACATCAGCCGATGCCGTCC
AAATTTTCATACGTGACGTGCCTTCGCCAAAGGCTTCTTCAGGCGCGCGCAGAGCATCTT
TCATGCCGGTGCCGCGTTTATATTTCAACTTAAAATCTGCCGTACTGCGACCGATATATT
CTTTGTGGGAAAGTTCTGCCAACCAACCCGCAGTTTTACGCCGTTGTACAGTCAGTTCGG
CATCATCAATGTAACTTTTTGTTTCCCTCATCCACAGTTTTACACCGAGATAGGTTTTGC
GTTTGGCATCACGATACAACAGGCGGTTGAAGCCGAAATCAGTATTGTAACTTTTTCCAT
TATAGTCATAGACTTCCGATAATCCGGAAACTGCCTGATGGTAACGGTAGCCATTGTGAT
TGAATGCCCATGTCCATTTACCGAAAGGGGCTGAATAATGTACGGCGTAATTGTTTGATC
CGCCTTCTTTGCGATGGCCGTCAAAACTTTCCTCATCGGGCGTACCGCCAATCGAACGTC
CATAATTTACATAGAACATATCACTCAGTCCCAAAGGATTGTCGGCAGAGAAAGTGATAT
TTCCTTGGTATTTTCCTGTCGCCTCACTACCCGAATTATCCATCCCCACACTCACACGGT
AGGGCAGCAGACGTTGCCGCCATTGCACCACGACATCACTTTGGTTTGGTTCTCCCTCTA
CGGGAACGATTTGGAGATCGGCTTCCGCAGTCGGGAGACGTTTGAGATTTTCCAGTCCTT
GTTCCAAATCACGCAGATTCAACAGATCGTTCGAGCGGGTGGGAAATTTGTTCTGGAATG
CTGCAATACGTCCTGCATGGGTTTGATCATCGTTAGACCGATCGATTCGTATGGAGCGCA
GATAGCTCGGTATCAGGGTTAATTGAAGCTTGCCACTATTCAAATCCTGTGGCGCAGCCA
AGATACGGGTCGTGGTATATCCCCTGCCGATCAAAGCATTTTGTGCTAAGGACATGATTT
GATTAATGTTGCCCGCATGCAGACACTTGCCAGCCTGAAAACCCGTTTCGCGCAAGGCAC
GTTTTAGGGCAAACTGAAACCGAGCATGGTGTTCGCCTTCCAACACCACTTCGTTAATGG
CAAAACACGGTTGGCTGCTGTCATCGCCCATCAACTGATTAACCGTTTCCCCCGTGTTTT
TTTGATGCAAACGCACATCGCTTTCAGGCTGCATGGTTTGGCGCAACTGCTCTTCGCGTT
GGCGTTGCTGAATATCTTGCTGCATACGGATTTCGGCAGGGTTGGGGGAGGCCAACAAAG
TAGCAGGAGCAATGATACCTGCCAATAAGCAGCACCAAGACAAAAAGCGAATATTAGGCA
AATAGGATAAAGGAAGTTTCATGGCATGGTCGCAAAATCAATAAAATCATCAATGGGCAT
TCAATACACGATTGCATCAAGTTTTAAAAGTTAAAATTCTCAAACCCTTATCGCTCCCTT
TATGATACAAGGCGCGTACTGTCGTACTAGGAAATAACTGGCGCACAGCTGAGCGCATTT
GATGACTGTGTCCCAATGGCGCAAACCATTGAATCAGCCAAATATTGTCGCCACAGTTCC
AATCGCTGTTGTCACGCAAATGGCGGTCAGATTCTAAATAATGCGCCTGCGCCACTTCAT
CAAAATAAGCCCATGAGATATAACCGATTGGTTGGGTACCCTTGCAAAACAAAGCGAACT
GCCCGTTTTTTAACACAGGCAATATATACGTCATCATCTCCACAATAGGTACTTGGCGAT
GCGTAGGCGACTGATACCATAGCCAAGTGATGGCACCGAGTGCTTCGCTTTCGTTCCATT
GTTCATTGGGGTAGAGTTTAGGAGAGATGATGTTTAAGGGTGGAGTGATGGGCATATTTT
AAGTTAGGGTTCGTGTTGGTTAAAACAAAAAATGGTTTCAGCCTGAAATGAAATATTTCA
CGTTAAAACATAGTTTTAGCACATGAGACTGAACTGCGTGGGCAACGAGTTGCCCACCCT
ACCATGTAACAGTTCGCATTAATTGCACAGGCTACGCTGGTTATTGCACAAACAAATGAC
CATACTGCTTATCTTTACGAATATTGTATCCACCGCCATGGTGAATACACTGATAAGAAA
TATGTTGCCCAATTTCACTAAATGAGACTTCGGTAATAGATTTATATCCTAATTGATTGC
CTAATTCATGCAAAACAAAGGTGAGATTTTCAGTTTCACATGTGTCAACGGCTTTCTCCC
AGCGTGGATTAAGTTTGGAGCAATTCCAGTCTGCACAACTACCCCAAAAAGGGCAAGCTG
TTAGCATTGTTGAGCAGAATGATATTAGCACTAAATTAGAAAGGGTTTTCTTCATATCTC
TCTCCTTTATAAGTTATTGTGTGTTACAACATAATTAGGATTTTTAATGCACTGATAGTA
AATATCCATTACTGTTTTGTTTTTGTACTTTTCTTCATCATTCTCAAAATAGATATTCAG
TTTTACTAATGCCTTATCAAGGCAATTCATAATTTCTTGGTGTTCCTTATCAGTAGTTGT
CCAGTTACTACTAGTACCGTACAAGGCTGTACCGACTACGACAGTACCTAATAAGGTAGT

## Appendix A

AGCGCAAGCTGTTAGACAAAAAACAGATGAAAGAAACAGAGAATATTTAGAAAGCTTCTT
TTTCATATTTTTCATTATCTTTTGTTTTGTTTGGTTCTACCAGTATGGGTTTAGATTTGT
TAATTGGCTTACCTTGAGCGTCATATTCAACTTCACCCCATATTTTTACATAGTCATCAA
ATTCTTTTGTACCTGCTTTTGGCACCTCCGCAAAATAACTGCTATGCGAATAGCACAACC
CCTTACATGTACCTTGTGTGGTGTCATTGGTTCCTAGCAAGAAAGGTATCCATTTGTTCC
CCACAAAATCTTTATCATGCACGATTGAGTAGGATCCGCTGTTATAAGTTTTGCCGTCTG
CACCCGTATAGGTATAGCCGTTTTTCTGTAAAACATCGGCGTAATCATTCTGCACATTTG
TGGCTGTACCATAGAAACGTGCTTTTCTGATTGGGGCAATGCCATTTTGTTTTTGATTGT
TTACCCAATCTTTTAAGGAAACGCTTGCTGTAATTCCCCCACGACTGTGATTACTGGTAT
CAACCGACCAGCCGTTACCCATTTTTTGAACCTCTCGATAAATATCTTGATTCAGTTTTT
CTGAATTGGTTTTGGGTAAATAGCCTTGGAACACTTTATTATTTAATTGGTCGTAACCGA
CATACATCAGTTCAGAAACAAGACTTGAACCGAGCCATAAAAAATCTTTTATTTTGTTAT
TAGAATCAGATTTATATTTCCCTGTTGGAGGATTCATGACTGCAATAACACCACTACCGT
TTGTACTATTACGATTTTGTTTTGCTGCGTTGCTTAATGAATCTTCTCGATTATTGAAAA
TACCAGGATTAGACACAGTAATAACTTTGCCATTTGTGTCTTGAATGCTTGCCAGTTCTT
CCTTGCTTAAATCATTCAATGACCAAATTTGTCTGGTATCAAAGGATACTTTACCTGTTT
TTTCATCTATTATCTTCTTATGGAACCACATTTCTTTAGGTGCAGTTGCAGACCTTACCG
CTCTATCGGCTTGGGTTGTGGCAAACATACTCCAAGCCATATCAGAACTCCCTGCTCCCC
AAACGAGTCCTAATCCTGAATCCTAGGCGGTAATGACGAAGGATAGTTTTACAAAAGTTT
CGGCCTACAATTTATAGGTTTATAATAATAAAAATATCCATCAAAAAAATGTGATTTTTC
TTTTTTTAAAAGTTGCATCTTGCCATTCTTGTAATCACATTCGTAATATTCAACATTTTT
CAAATCTGAATCAAATAGATATTGAGGTAAAACATTCCCTTCCTTATCTAGTTCTATTAA
TTCCTCAAAAACTATCTTACTTTTATGATAAAAAACAATTTTTTGGAAATCTGAATTGAT
TATATACAGTTTTACATAATCCCAATTAAAATTCGTTATATCACTAATTAAGAATTCTTT
TTTATTATAATTAAAACTTATTTTTCTTATTACATTCTTTTCAAATATAAAGAAAATAAC
AATAGTGATTAATAAAGAAAATATATAAAGTATATTTTTATTCATCTTTTATCTTCTCCA
TTGTCTGGCTGAAATTTGTGCTCTGATTTGTTTTCTCCTCTGCTGAATTTTAAATCCGGC
ACCAGTATTATCTAAGTGAATCAATATATTGCTTGTATTCTGGTAATCTTTTTCAGATAA
TCTTTTTCTTACAACATCATAATTTCCATTACTGTTTCTTTCTGCTTGATAAAAACCATT
ATTTTTATAAGTTTCCAGAATTAATCCAACTAACTCTTTTGTGTTTAACCTATTGGTATT
TAATGCTATTTCTCTTCCTATTTCGTTATTTAGTTGATCAATCATTTCATCTGCTTTATC
TAATGAAAGATTACGTCTTATAGAATAATTAATTTTTTCCCCACTTTCATGACTATTTCC
TACTTTAACAGCAATATCTTTGCCAAATTCTCGTGTGATGGTTGCTTGCCATAAAACGTG
CCTGAATGCATTGCCAACTCCACCTTCACCACCAAATTCACTATTAGGAAATAAATTTAA
TTGAAAAGTTGAAGCAATTGTTGAAATATTAGGCTCTAATGTAGAGCCAGGATCTTTATG
TACAGATCCAATTGCAATAGCAATTCTAGGGTGCCTAAGAGCAAATTTCACTTGCTCTAC
AGTATCATTATTCTCCACCGCACTTTGCGCATTCAGGCTGCCTTGCGCTGCATCTGTTGC
GCTGTTGCCGACTGCCGCACCCGTAGCCGTACCCAATACATTTGTAATCGCTGTTACAGT
CTCTTTCTCTTCCGCCGTTAAGTCGCTTCCTTTTTCTTTGCCGTATAACCATTTGCTGAT
GTAAGGCGCAGCCGCTTCCGACCCGCCCGCACTCAATGCTCCTGCTAGAGCATTGTTGTC
TCCTACTGCGGCAACCGCTGCTCCTAATACCGCGTGGGCAAGAACGTGTGCGGTTTCTTG
ACTGGCGGTTAGTTTACCATTCGCGTTTTGACCGGCTAAATCTTTAAAGTGCTGTCCAAT
CGCATACGATACGGCTGGCGATGCGGTAGCCGCAGCGATGCCCGCTCCGCTTTGGGTCGG
CGCAGCTAAACCTGAGGCTAACATGTTGAGAATGACTTTGCCTTGTTGCCAATTATCTGC
TTTTGCTGCCGCATCTTGAGCTTCATGGGCTTTGCGTTTGGCAGTTTCCATATCGCCATT
GGCTAATGCCTCGGCTGCTGCTGTTTCGGCTGCTTCTTTGTCTGCTTTGAGTTTGTCTAA
ATGTTGGTTAATCTCGGTATTGGCTTGTTGAACATTTTTACTAAAATCTTGGCTGACGGT
TCTTTGTAAATCCAGTTCACTTTGCACCGCTTCTTTGTTGAAGGTGTTCTTCAAGCTGCC
CGAATGTCGTTCGGCGGTGTCTGTGGTTACGTTTGTATCAATATCGGCTTTGGTTTGTGC
CGCTGTTTTGCCTGTCAGCCGGATTTGTGCGGCTTCGTCGGTGATTTGAATGTTGCGGGT
GTTGATGCCGCTTTTTGTGATGCTGCTTTGACTGTCGCTGTCGCTGCCATAACCCACTGA
TGAACTTGCGCTGTTTTTATCGGCTACGCTTGTCAGGTGTTTGTTTTGAGGTTTATTTTG
TGCGCCCTGTCCCAGTGTTTTGCCGCTTATGGACGCACTTGCGCCCAATCCAAAACTTTC
GCCTTTGTATTGGCTGTGGTTTTTGATGTCGCTATGGGTGAGGGTGGCCGTCTGAAAGCG
GTTTTTACCCTTGTCTTCTGCGCTTTGGGTACTGGTGATGATGCCGCCTTTGAGGTCTGT
ATGGTTTCCGACCTTGATTTGATAGCCGTCTTCTCCGGCATAAATACCGCTTTGCTCGGT
TACTGAAACATGGTCGGCTCGGATTTTGCTTTGGCTGTAATCGCCACCGGCACTGAAGCC
ATAACCTACGGTAACTTGTGCACTGGCGTTTTGTTGTTTGCTTTGATAGGTTTCTCTATC
TTGTACGCTTTGAATACTTAGGTTTTTGGCATTGACTTGTACGCCTTTGCCGCGTACTTG
CGCGCCTTTGATGGTAGTGTCGCCACCGCTTTGGATAAGGGTTTGGCTGCCTTTGTCGCC
GATATGGCTATGGCGGTGGGTGATGCTGTCGCCATTGCCGTAGCCTTTGCCGACATTGCC
GCCTGCGGTAACGCCTAATGACCAGCCTCCTTGTCCGAATGATACGGCAGCACCTGCGTT
CCAGCCTGCCGATTTGTTTTGGCCGCGTTCGGTATTGCTTTGCTCGGCTGATTGGAGTGT
GATGTCGTTATCGGCAATCAGGATTGTGCCTGCTTTGCCGGCAACATCTGAGCCTGCGAT
GTTGATATTGGATTGTTCTGCTGCGCCTGTGGCGATTAATGTGGTTTTACCACCTGCTTG
AATTTGACTCGCTTGGGCTTGATTGGCTTGAACTTGGGTGGTTTGTCGGTTTTGCTGTTC
GCCGTAGGTTATGGAGATGCTGACTTGTTTGGCATTGGTTGTACCATTGGCTAAGTTTTG
TGCACTCTTACCTGTTTGATAGGCTTGCCAGCCTGCATTGGCAGCCGCCATGGCATTAAC
GCGGTCGTTTTTGCTTTGTCCGACTTGTTTGCTGCTTTGTGCTACGGCAATCGCTTGTTG
TGCCAAATCGGTAACGGGCGAACTGAATGCCACCGTTAGGCCTTTTTGTTCATAGGTTTG
GGTGGTATTACTGTTTAATTTGTTGTGTGCCGCTTGAATGTCTATGCTTTGGGCATAGAT
GGTATTGTTGCCTTCCGGGCTGGAAACGGTACTGCCGATTTGTTCGTAGTGTTTGCCTGC
AACAATGGTGGTATCGCCTTTCAAGCTGCCTACGGTACTGCCTGTATGTTCGTTGCTTTG
GGATTGGTTTTCTTGTGTGTTTGTCTTGCTGCCAATAGTGAAGCCGATACCTGCACTCAT
CAATCCTGATTTCTGGGTTTGATGATAGGTTTCGCTTTGGCTTTGAGTTTGGGTTGTACC

# Appendix A

AATGCGAACATGATTGCCTGCTTGAATCTGGGTGCCATTATCGGAAATAACATTGCTGCC
AAGGATGTTGGCATCGTTTCCTGCCTGCAATACAACTTGCTTGCCTTCAAAGGTGCTGCT
TTGGGCGGTTTCGTGATGACTTTGGGCTTTATCGGTAATGACTAATTTATTGCCACCACC
GCTTCTGCCTGTGTGTTTGGACGCATCATCAACATGGGTCGTGTTGATGCCTGCGCTGAT
GTTGATGTCATTTTTGGCAGACACAGCGAGTGTACCGTTTGCGCTGCTGACTTCGGCAGC
TTTGGCATTGAGGTTATTCCCTGACAATAGGGTAACATCGCCTTTTGTTTGAATGCTGCT
GCCGACTTCGTTCGTTGAACCGCGAATAACATGGTTATCGGCATCAAAATGGGTTGCTTG
ATGTTTGCTGGTTTGTACCGTATCTAGGTTAATGTCGCGCCCTGCTTGCAGCCGGGTTTG
CCCTTGCTCTGATTGATTGCTGATTTGACCGGCAATGATGTTGATGTCTTTTCCTGCCTG
CGCTGCTAAAACACCTTTTTCTTTGCCTGTGATATAAATACCTGCCATTCGGTCTAGGTA
GGTGCTGCTGCCTTGTGTATTTTGACTGCTGGCGGTGGTGCTTTGGCTGTTGATGTTGTT
GCCTGCGTTGAGCAATAATGTCTGTTCGGCAGAAAGCATGCCGCCAATATTATTGATGTC
TTGTGTGGCCGTAACCGCTGATTTTTGCGCATGAATACGCCCACCGATATTGTCTAGCGT
ATCGGTATTGATAATAAGCGCATTGCGCCCTGCAATCGTGCCTGAGTTTTTCAGGCTGCC
TGAAACATTGATTTGTGTATTGCTGCCTGACAACAATGCACCTTTACCGTCTATGTCGCC
ATTTTTAACGCGTACATAAACCTGTGGCACCAATACGGTTTGTGTGCCGCCATCAGGAAG
CTTAACTTCTTTTTGTACCAACCAAACAATATCGCTGGTCAGTTGCGCTACTTGCTCGGC
ACTTAATGCAATGCCAACGCTGAGATTCATCGAACGTGCCGCAGTCGCGCCATTATCCAT
TAAGGCTTTAAATTGTTCTTCGTCGTTTTGATAACCGTCTAAACGACGATGCCCTGTCAG
CTCTGCGATTTGTTCATTGATTAAACGTTGCTCGTAATAACCATCACCCAAACGTTTATG
TAAATTGTTTGGGTCTAGTTTGAGGCTGTCCAGCATATAGTCACTACCCAACCATTGACG
GTAGTTGGCAAAGCGTGGATCGGTTTCAACAAGATAGCCTTTATTGACAGGATTGATAAT
GTATAAGCTGCTGCTGGGTAATGGGGTAAAAGAATTGGACGTATAGGGTAGCGAAATACC
GTTGCTTTGCGGCAACTCAGTGCCTTGGCTGGGCGCATGATGGCTTAATGCTTTGCGATG
CGATTCATAGGCAAATGAACCCAGTGAAATGTTGCGTGTGATTTCCTCCGGCAAAGTGTA
ATTTTGTTCGCTATGTCCCGTTGAGTCTCGTCCTTTATGTTTCTCACGCCAATAGCTGTG
TAATTTGCCATTTTCACTGAATACTTTCTTTTCGCCAAAGGTTTGCTCGTTATGCAAACC
GTCTTTTTCTGTTTGTACAATGAGATTGCCACCAGCAATGATTTGGCTATCGGTATTAAA
TACTTCTTTACCATCAATGGTTAAATCATTACCTGAAATGATTTTGGCTGGCGCAGTTTG
GGTAACTTGGGTTTTTTGGGTGACTTTTTCATAATCGTATTTATGCCAATTTTCATGCGC
CGCTCCATCAGGGGTGCGTAAGTGGTCTGATTCATCGTTATAGACAGACCAGCCTAATTC
ATGTTGCGTGCCTTCTCGCAATAATTCGTGTCGTCCAAATGCTTCGTAATCAACAATATG
CTCGCGCCCTGTTTCTACCAACTGCGTTTTCAAATGCTCATTGGTATTGTGCAGCTTTTC
TACACCTAAACGCATTTTGCCTGCAGCTTCAATGGTTGCGCCGGCATTGTGTATCCTTTG
GGCTTTGCCTGTGGCTTGGCCATTGGTATCTAATGCGCCGCCAACCGCCATATCGTTACC
GCTGTAAATCAGACTGTTTTCACGGTTGTTTAATTGTCCGATGCCTAAATTCAGGTTTTC
ACGTGCCGCAATGGCGGCACCTGTACCGTTTTCATCTTGATTGTCTAAGCGGGTAGCCGC
AATAGCGATATTGTCGCCATAAATCCGACCTGTACCGATATTATTCATTTGCCCGGCTTG
GATTTTGGTTTGTTGTCCGTCAATCAAGCCTCTATTGGTTAAATTGTGCTGCGTGCCAAT
GTCTGTCGTACCGCCGGATTGAATGTTGCCTTGTGCTGCATTATCAAGGTTATTTGCTTT
AATCCGAATGCGTTTTCCTGCTTGCAAAGTATGTGAATTTTTCAGGCTGCCTCGTGTACT
GAGCGACAATTCATTGCCCGCCACGATATTGCGTTCTACATAAAAATCATCTTGTAACGC
AATATCCAGTTTATTATCAGCGGCAAGTGTGCCGTTGTTGGATAACGATTTTGCCTGAAT
AGCAACATCACGGCCTGATTGTATCGTGCCATTCGTATTATCAATGACAGCGGTAGATTG
CTGACCATCGTGAATAATCAGTTGTTGATTGGTCGCTATTTCGCCATTTTGATTGTTCAG
GCTGCCTGAAACGGCTAAATCCGCTATTTCTGCTGATAATAACTTGCCATGAGCGTTATC
CAGTTGATCGGTTTCAATCTCTAACTGTTGGCGTGTTGTGATGTTGCCATTTTGATTATT
CAGGCTGCCGGCTTGAATGTGGACCGCATCACTGATAATTGTTCCATTGTGATTGTCAAA
CGCCGAACCTTTTGCATTTAACTGATGAATGTCTATTTGTCCTGCATTATTTAAACCTTG
TTGCGCACTAACATCTGTTTGACCATTGGCAATAATACTGCCTGAATTATCCAGTGCACC
ATGAGTGCGAATTGTCCCATCAGCAAAGGTAGGCGCAGTTATGTTTGATATAGAAACGGT
TGCAGTACCCGTACCTGTTGCCGTTGTTGGTGTGGTAGTGGATGAATGGAAAGATGCATT
GTAACTATTGCCGGTTTGATTGCTTGAACCATTTGACGCGGTTGGTGCGGTATCTTGTAA
ACCCATGCGGCCACGGTTATCCATTTTGCCTTGTGCATCAATATGGAGTTTTTGTGAACC
TGTTTGAGAGAGTTTGCCTTGATTATTAAGTGTGTCGGTATCAATAGCCAAACGAGCGGC
TTCAATGGTGCCTGATGTTTCATTTTTCAGGCTGCCCGAATTGTGAATCAATATTTCGCC
TGAGGACAATAATGTGCCAGTGTTTTGAATCGACTGACTGTGAATTTGAGTGCCTTGTTG
CGATACCGCCGTACCGCTGTTTTCAACGCCCTGACTGCGGATATTGACTTTGTGTTCCGC
TGTATTATCCGTATCTTTCGCATTGGCGGCAGCCATCGTGCCACTATTGACTAAACGGCC
ATTTGCATCAATCGCCACATTACCGGAAGAAGCAAACAACTGCCCTTGATTACGAATGCC
TGCTTGCTCGGCCGTACTGATCAAGGTGATTTTGTTGGCATACATACCTCCTAATTTGCC
TGTATCAATCGCAAATAAAGGATATGTGTGCCGTTGTTGGCTGTATTGTTTGACGTATT
GGCAGCAGCATTATTGAGAATAGGCGAATGTGCATTACCTGTTGCGACCACATCGTTTTG
TCCCGCGACGACACGAACATCTTGTCCCCATACGGGTGCATCAATTTTGGAATGATAACT
GAGAATACGTGTGAAATCGGTATCACGGGCATCCAAACCGTGTCCGGCGATTACAACATT
GCCTTGCCTTATCTTAAAGCCGCTAAGGTCTCCTGCTTGATATTGCGGTTGGCCTGTCGT
CAAAGTGGCACGGGAAGCATTGATAAAACCACCACCATTGACTGCAATCCCTGCCGGATT
GGCAATAACGACTTCTGCACGTCGTCCGCCCACTTCAATATAGCCATTCATTTGTGAAGA
ATGGCTGCTGTTGATTTGGTTTACAACCACACGTGCTTCGCCCCTTGCCAACCAAGGATT
ACCTTGAATCCAACCGCCTAGCTGTGTTTGGGTGTTGCTGCGGCTGTTGTTTAAAATCGC
CCCGCGATTACCCACATCAAACTGGGCGTATTGATTAACAGAAACCCCTGCCGAAGTAGG
GGTTTGAATATTGACTTGCGGTATGCCGTTACCTGTTTGCAGAATCGTGGCTTGTTGAGT
TTTAGGAGCAGCTTTATCAGCAATAATGCCATCAGCAAAAGCAATATTGGCCGTACCTAC
AGCCAAACATAAAGAAAAGCCCAATAAAGAAAAAGAAAAGATATTTGAACGACAAACAGG
TGCATGAGTAGTACCAAAAGGAACAGATTTCACATGAGCGCTGCCTGAATCACTATCGGC

## Appendix A

ACAGCTTTTACCTTCGCGCTTGGTAGTTTCAGCAACGGCTACCACAGCCCCACGTTTGCG
GTTGAAAATTACACGATAGAGAGTTTTATTCATGATTTCAGTTATTTGATTTTTATAGAG
TTATTAGAAAAAATTGGATAGTCTGACCATTCTAGATCAAGGATTTTGGCGAGTCAATTA
CCGCCATTTTACTGCCATTTGTTTATTAAATTAGGGACTTTACTAGATAACGGTTAAAAA
TCCCATTCGAACGAAATGGCAAGGTTTATACCGTCGTTGTCCCTAATGCGCAATCAGCAA
CATTATTGCCGATTATCCGAGAGAAAGTTAAGTCTGATGGCATTGTGTATACGGATACCT
TTCGTAGTTATGATGTACTTGATGTCAGTGAATTTAGCCATTTACGCAAGTTTTCCAGTA
TTTGACTGGCAATTTAAAACAGTCGGATTTTGTCCCATTTGTTGGCCAAGTCTTTACTTG
CTTGGCCGTTTGAATTTAAAAAGCAGTCTTTCTACTTTCCGACCTTTTTTTCTGTTGTAA
GGTCTATAATCCAATAGCATTCCCAAAGAGCATTTTGGACGGTGGCGGATTCGCATTTGA
AGTGCAACTTTCCCTAACAGAAAAAGGCCAGTATGCGGTAGCATACGGCCTTTCCTGCAA
GAAAGATTGCCATGAGCTACACGCAACTGACCCAAGGCGAACGATACCACATCCAATACC
TGTCCCGCCACTGCACCGTCACCGAAATCGCCAAACAGCTGAACCGCCACAAAAGCACCA
TCAGCCGCGAAATCAGACGGCACCGCACCCAAGGGCAGCAATACAGCGCCGAAAAAGCCC
AGCGGCAAAGCCGGACTATCAAACAGCGTAAGCGACAACCCTATAAGCTCGATTCGCAGC
TGATTCAGCACATCGACACCCTTATCCGCCGCAAACTCAGTCCCGAACAAGTATGCGCCT
ACCTGTGCAAACACCACCGGATCACGCTCCACCACAGCACCATTTACCGCTACCTTCGCC
AAGACAAAAGCAACGGCAGCACGTTGTGGCAACATCTCAGAATATGCAGCAAACCCTACC
GCAAACGCTACGGCAGCACATGGACCAGAGGCAAAGTACCCAACCGTGTCGGCATAGAAA
ACCGACCCGCTATCGTCGACCAGAAATCCCGTATCGGCGATTGGGAAGCCGACACCATTG
TCGGCAAAGGACAGAAAAGCGCATTATTGACCTTGGTCGAACGCGTTACCCGCTACACCA
TCATCTGCAAATTGGATAGCCTCAAAGCCGAAGACACTGCCCGGGCAGCTGTTAGGGCAT
TAAAGGCACATAAAGACAGGGTGCACACCATTACCATGGATAACGGCAAAGAGTTCTACC
AACACACCAAAATAACCAAAGCATTGAAAGCGGAGACTTATTTTTGTCGCCCTTACCATT
CTTGGGAGAAAGGGCTGAATGAGAACACCAACGGACTCATCCGGCAATACTTCCCCAAAC
AAACCGATTTCCGTAACATCAGTGATCGGGAGATACGCAGGGTTCAAGATGAGTTGAACC
ACCGACCAAGAAAAACACTTGGCTACGAAACGCCAAGTGTTTTATTCTTGAATCTGTTCC
AACCACTAATACACTAGTGTTGCACTTGAAATCCGAATCTAAGGTCATCTGAAATTAAAT
TTAGTTTTCAGACAATCTTTTTCTTCAATTGGAACGTGGAGTTACATTTTCACCTAAACT
ATGCACGCTAGATTTATAGATAAACCATTCAGACAGTCCAATAAACATTATGGTTGGGAT
TACCAAATACCAAAAACTACCGACTCTTTCAGATGAATTAAATAAAGAAAAGTACCCATA
TGTATAAAAAACAATGAAGGTTAATATGACAGATACAGCAAGTTGATTTTGAAATACCCA
TCTGAATAAAGTTGATAATATCACTGGAATTAGAAACCATAACATAACTATAAACCCACC
CCCTACCATCCCAAATATAAAATCTATTACCCCTAAAAAAGATAGTTTAAAATCATTACC
CCATAAACCTGAAAAAATTAAAAGGCCAAAAAATACTCCACCTATTAAGAAAATAGATTT
AATATTTTGAATAAGATAAAATAAATAATTTTTATTCATGGCTTGCATCCCTGTTTCTCA
GAATATCCCATAAGCTTACAACCATATTTAATTCTAGATTTGTAATCTAACATATTAATT
CCATCCTCTATTTTTTTCCTCTCATCTAAAGGATTTGGAATTATTGAAAATATCTTATTT
GATGTTACCCCTTTAATCAATGACAAATAATCCTTCTCATTACTCAAATATTTTTGGTGA
ATAGGCTGTAATAACTTTTGCTCGCTTTTCAACTGTTGAATATCCCACTCCCTAGGATTA
GTTTTTTTATTGAACAAGTCTCTAAAATTATAAAATCCATTTACTATCAGTGCATTGCCT
GACGCAGCCCTCCATTCATAAATTGGAGGATTACCAACAACTTTACCTGCCGCAAAAGTT
ATATAAGAAGCTTCTCCGTCTGCCCCTAACCCAGTTATAGCAGCACGCGATACAAAATCA
GCTCCACCAAACCATCGTGCTTTTGAACCCAAATTTTGCTCATAAAGATTGCTGGCAGCA
AAGAAATCTGCACGATTGTCAATGGTGTTGAAATACTTGTCAAATCTTGTAGATGCCCCT
TGTGAGTTATATAAATAGCCAAAAACTTCTTTGGCAACCCGTGATACATCCGTAGGCATA
TACTTCCACGCAGCAGGCATGGCAATATATTTAATAGACATATTAATGCCGTTTCTGCAA
CCATCGCCCAATGGGTTCCTAGAACAGACTTTTTGAATTTCCGTATCAATTACTTTGCTA
AATTGTTCATCCTTTTTGATGGCATTTACTTTTTCCATCGTAGTAGAACAAGTTTTACCG
GAAAGGCATTTTGTCAATTTATCCATTCTTTCGTTGGTAAGCGCATTACTCTCTACCGCC
ACAGCAGCCGCATTCGCCGCAGCATTCACATCCCCCTTACTCAACGCCGCAACCGTCCCT
GCTGCCAGCTTCGCCTTAGCAATGATTTTTGCCCGGTCTTTCACATTCAGGCTGCCCGGG
TCTCTGCCGTCCAGCAGGGCTTCGCCGAGGATTTCACCGACCGCCGCTCCTATCGCGCCA
TCCTGACACTTGCCCTTATTCGCCGCCGCAGCCGCACAGCCCGCTATGGCATGGGCAATC
TTGTGGGTAATGTAGTGCTGATCCAACTGTTTGATTTTACTGGCTGCTTCTCCATGCGCA
GTATTCACCAAAGCCGCAAGGATATTCGCTTCCAGATTGTCTTTCAGGCTGCCGCCGTTG
ACAGCGGTATTAATCAGTGCGGCACTGCCCGCATTGGCCAGGTTGACGGTCAGGTTGTTG
ATCCACTGCTTATCGCTGACATTGTTCAGTGCCGAAGCACCGATTTTGTCGGCTACGCCT
GCGGTAGCGACGGCAACCATCAGATTTTTCACCGTGCTGCTTCTGCCCAGCTCTTTCAGG
GTGTTACCGATATTGCCTTTGTTGTTGATGAGCGATACGGAAGCCTGGCTGGCCAGCGAG
GCGAATGCGGCATCGGTTGCCGCTGCGGCCGCGCCGTTTAAGCCCAGTGCGGCTCCGGCT
CCCGCGCCCGCAGTAACCACGGTAACAGCCAGCGCAATAATCGCTGCTCCGGCTCCGGTT
AAGCCTTCCTGTTTATAGTCCCATTTGTCGTACGCCAGTTGTACCTGGTTCCAGTTGACG
TCTTTGGTGACTTGGAGCTGTTTCAAATAGCAAGCGTAGGGGGTGTCTTCAGCACGCACG
CATCTGGGCAGTATATAGGGAATCTGAATATTTACTTGCATAACAAATGCCGTCTGAAAA
ATTGTGAGCTTTTCAGACGGCATTGAGCCGTAAATCATGGAACGCGTGCGTGCTGAAGCA
CACACCTTACGCATGGATTTTAGGTTTCATGCAGGCTACAGCTTGCTGCTATTCATCAAA
TTGCGGCCATTGAAAGTCTGTTGTTTTACTTTCACCTCTCAACAGTCTAATCATATCGCT
TTTGAGAAACTCAAAAAAATTTTTAATATTACCAACATAGAGCATAGCTTCACATAGTGA
ACTACATGCAGATTTAATGTCTTCATTGTCAATAGCATATTGATATTCCTTCATATGCTG
AAAAAAAGAATCAAAGTCTTCTTCTAATTCATCATTCCAATCAGATGAATAGTTAGAAAG
CCATTGTAAGTCAAGAGGATCTTCACTATTCAATTTTTCAGTTGTGGCTTTCTCATAAAG
ATCAAATCCTTGTTTAATTCCTAACTCTCTTAAACTTTCTTTTACTACATTAAAATTTTT
CATCTGAATCACCTTATTTAAGATTCAATTTTCGCCCTTGCCCTGCTAATGTCTTAGCTT
AATTTTGAGCGAGTTTTAGGTTTCATGCAGACTACAGCTTACTCAGCACACACGAGTCTA

## Appendix A

AACAGTATACAGGGAATCTAAATATTTACTTTCATAACAAATGCCGTCTGAAAAAATTGA
GCTTTTCAGACGGCATATGGCCGTAAATCATGGAACGCGTATACTGAAGCCCACACCTTA
TGCATGGGTTTTAGATTTCATGCAGGCTACAACTTGCTTTCTATTCATCAAGAGATGGCC
ATGAAAAACTATTCTTTTTATACTCAGCACTCAATAATGTTGATATATCAGTTTTTATTG
AATCAAATATAAGAGATAGATTACCTGCGTAAATCATAGCCATAAATAAAGAATTACTGG
CAATTTTGAAATTTTTATCGTTTAGGGCTAATTGGCATACTTCCATATAATCTAAAAAGT
TTTTTAAATCCTCCTTAAATTCATTATCCCAATTCCCGTCTGAAGTATAATCTTTAATCC
ATTTCATATTAGCTGTTTCATGATTAACTTCTTCTGATACTTTTGGATCTGTCAAATCAA
ACCCTTGATAAAGCCCCACATTAATCAAGATTTTTTTATATCATTCAATGTTTCCATAA
AATTTCCTATTTTAAGTTTAATTTACGACCTTTTGCTGCCCCAGTTTTCATTTGGTTAAG
CGAACCATCCATATTTAGAACAAACTTAAAGTTCCCATTTTTATCAAAAACCTCTAAATG
ATTTTTATGTTGGCCATCTAAATAAAACCTATCACCGGTTTTTAATAACCCTTGGTTTCT
TTTTACCAAGAAGACAGACTGCCCTTGCTGCGTCGGAAGCGTTGTCTTTTCTGAAATTTG
AGCCAGCTGTTTTCCAAAAGGATTATTTTTCATGTATGTACTCATATTCGGTACAGCACC
TTTATTAGGGATATAAGGACGATTTTTTTCTAAAACTTCCTTGACCTTTTGTGCCGCTTC
CCCTTTATTAGCGCGATTCAGCTCTGTTCCGACGACAATATCAATAACGGCTTTGGCATC
GTTCCAATCCAATGTTTCGTCGAATAAGGTGGTCAGGTTGTCGGCTAAATTATAACCTTC
GTCTTTCAACGTCTGTTTTAAATCTCTAACGTTGATTTTCCCGTTTTTTAATCCTTTTCT
GGCTACCTTATAAACCACTTTTGCAGCAGTTACAACAGCTTTAACCGCATTATTTTCTAC
CGCGTTTTGTGCGGTTTGTGCAGCAGTATTGACATCTCCTCCCGTTACGCCTGCAACTGT
ACCTGCCGCAAGTTTGGCATAGGCGGTAATTTTCTTAACTTCCAGATCTAATTGTTCCGG
GGTCATATCGCTAAAATCGGTATTTTTAACCAAAGCCTCCCCGACAATCTCACCCACAGC
CGCACCGATCGCGCCGTCCTGACATTTGCCCTTATTCGCCGCTGCAGCCGCACAGCCCGC
TACGGCATGAGCGATTTTGTGGGCGACATAGTGCTGATCCAGTCCTTTGATCTTACTCGC
CGCCTCCCCATGCGCGGTATTCACCAATGCCGCCAGGATATTTGCCTCCAGATTGTCTTT
CAGGCTGCCGCCGTTAACAGCGGTGTTGATCAGCGCGGCACTGCCCGCATTGGCCAGGTT
AACGTTGAGGTTGTTTACCCAAGGGGTTTCGCTCCAAGTGGCAAGGGAAGAGGCACCGAG
TTTGTTGGATACGCCTGCCGTTGCCGCCGCTACAACCAGATTTTTACCGTGCGGCTTCT
GCCCAGTTCCTTCAGGGTTTTGCCGACATCGCCTTTATTGTTGATGAGCGATACGGAAGC
CTGAGAAGCGAGTGAGGCAAAGGCGGCATCGGCCGCTGCTGCGGCTGCGCCGTTTAAGCC
TAGTGCGGCTCCGACTCCCGCGCCCGCAGTAACCACGGTAACAGCCAGCGCGATAATCGC
TGCACCGGCTCTGGTTAAGCCTTCCTGCTTATAGTCCCATTTATCGTAAGCCAGTTGCAC
CTGGTTCCAGTTGACGTTTTTCGCTACTTGGAGCTGTTTCAGATAGGCATACTCGGGCTG
TTTGGCCAGCTTTTCGATTTCGGTTTTCAGATTGCCTTTGGGGATGTCGACAATGTAGCC
GCÇGGGAGCAGAGAGTACGGGCGCAACGGAGCCTGTGAAACTTGGCAGTTGCAAGGTTTC
GATATTGCTGCCGCGTCCGGCCTGTTTCTGCCAGAGGGCAGATTTGCTACTGCTTACTGT
TTCAGTGCGCACACTACTTTTGATGCCTTCAAGAATAATCTTGGCATCTGCTCGTGCCTG
ATCGCCTACACCTGCACGGATGGCTGCGCCGCCCAGCGTGGTTTCAAACTGGGTGCCTTG
CAGTTTGGCGTCCCAGCCTGATTGCAGGTTGGCCGATTCTGCAACTACCCTTGAGGGCAG
GGCGGTTTTCATGGTGTGGGTGGTGGTGTCGTGCACCTTGTCGTAGGTAATGCCGATAAA
TTTGCGCTTGGTACGGGTGTCAAGTTTGTCGTAGTTGAGATCTTCCACGGCATAGAGAAC
CAGCCCACGTCCGGCTTCGATTTTAACGGAGCCGCGGGGTGCATCAAACAAGGTGGCGTG
GGCACCGATATTGCCGCCGGATTTGATTTCAATACCTTGTGACGCACTGAGGCTCACCGG
GTCGGCTTTGGCGTTTTTATGCTCTTTGATTTCAGTAACATGTTTTAGTTTGTACCACTT
ACCGGTTTTATAGCTGCGTTTATCGAAGGTGTAGAGCTCACCCTGTCCGGCGTAGTAGAA
CTGGTCGCCGTAGGATTGCAGTTTGATTTTGCCGTTTTCCGAACTGATATCCGTGGTGCT
CAGCAGGATGCGGCTGTTTTCATTGGCATACGGCGCGCTAATGCTCACACCGGTTTTACC
CGAAAGTTCTGCAGCAAGCGTAGGAGGTGTCTTCAGCACGCACGCGTCTGAACAGTATAC
AGAGAATCTGAATATTTACTTGCATAACAAATGCCGTCTGAAAAATTGTGAGCTTTTCAG
ACGGCATTGAGCCGTAAATCATGGAACGCGTGCGCGCTGAAGCACACACCTTACGCATGG
ATTTTAGGTTTCATGCAGGCTACAGCTTGCTTCCATAAATCATTTTTATCAGAGCTCGTA
GGTACGGTTAGCCGCCTTTAGCGGCGTAACCGTACGAATGAAATGCCAAGTTGCAAGGCC
GTCTGAAAAAGTTGAAAAACAGATTTCAGACGGCCTTGTTATTTTATAAAGTTTGCTGAT
ATGCGTACGGTTACGCCGCTAAAGGCGGCTAACCATACCTACGCTTGCTCATAAATATCA
ATATTCGGCAAATCGGCCAAATCTATTGGACACGCAATATCCCACCAAAGCCATTCTAAG
TAATACCAAGGGTCTTCAGGCCATATTGCTTGGGCATCTTCCAAAGTAGGCCATATGTCT
TTCAATTTCTGCACTTGTTCTTTTGAAGTTCCAGTTAGAGGAATTCCGATACCGTCGGTA
TAATCATGTAAACGGATTGAACCGTCATTTAACAGTTCTTCCATAAAAGAACAGAAATTG
TTTTTTAAGTTTTCATCTTGAATATTAATACCCATTTGATTTTTATAAGAGTTAAAAATT
GAGGATAAATCGATTTGAAAATCAAGAATCTTAATTTTTTGTTTCTCATCCACGGTAAAA
TCCTTTGTAAATTATTGGAATTTAAGCTCCAATTTAGTACCTTTAATAAATGATGGAGGA
TTCTGCAAATCTAATGTCCATCGTGCTTGAGTTGAATTCTCTGTTTTTGAAAAATTTCTT
AATGCAATTTTTGTTCCTGCCCATTCTCCAGTACTGATAATGCCATTTGCTAATCTTCCG
TCAGGCAAAACTCTAAAATTCGGATTCTGGCCAGTCATCTGCCGATAAAGCGCAAAAATC
TCCTGTTCGCTTACGCCGCTGTAAACGGGTGCTCCCTTTAAGCTTATGGCTTGTACAGGT
TTGATGGTTTTTCCGTTAATGGTTATCGGAATATTACTTGCATCAAGCAAGCCTGCTCCT
TTCCTTACTCTATTGTTAAATCCCGTCTTCCATGCATCCAGTTGCGTATCGCGTTGGGCA
ATACGGAATAAAATCTGCTGTTCTGCTTTGGCTAAATTTGCCAAATTGTTTATTGTTTCT
TTAGGAGCAGCTTGCTTAGCCGCCTTCGCTTTCGACAACGCCCCCACAGGCGCTTCCCAA
GCGTTGCCTACCGTTACCGCACCCGTGGCGATTCCCGCGCCCGCTTCGGCAGCCTGAGTG
ACCATGACAGTACAACCAGAAGGATTAGCCATGCAGGTGCTGATAGCTAATTTACCCGCT
GTACCGATCAGCGGAGCTGTCCAACCTGCAGCATAAACCCCATAGCTGGTAATCACAATC
GGGCCTGTGATGCCATTACGGATATTGCTTATCCAAATGGCAGCATCCTTATCCTGCGGA
TTAGTCATCGCACCTGCTGCATGTGCAGGCATAATACCTTGGATAATTTTTTCCAGTGCG
GTTTTGTCGGGCTTCTGCGGTTGATGCTTTTTCGCATTGGTAGGGGTACTGTCAAAATTC

## Appendix A

AAAGCATTATTCACTACCGCCACCTCAGCCGCATTCGCCGCAGTATTCACATCGCCGCCG
TTGAGTGCCGCCACGCTGCCGGCAATAATCTTCGAGTAACTGATAACCTTATGCTTTTCC
GCATCGCTGAGTGTAGCAGGGTTTCTGCCGCCAAGCATGGAGTCGGCTACGATTTCCCCA
ACTGCTGCGCCAATTGCCCCGTCTTTACATTTTCCTTGTACCAATCCGCTAACACACCCA
GCCAAAGCGTGGGCGAACTGTTTGGCAACATAATCGTCGCTGAAGGTTGTTTTGATTTTG
CTGGCGGCTTCTCCTTGGAAGCTATTAACCAATGCTCCTAATGCGGCATTGCCTAAGTTG
TCTTTCAGGCTGCCGCCGTTGACGGCGGTATTGATACCAGCTGAGATACCTGCATTACTG
AGATTGGTAGCCAGTCTGCCTCCAAGGTTGGCAATAGTTTGATTGCCCGTACTGCTGAAC
AGTTCGGTTCTTACCTTGCTGTTCAATTGGGCAATATCTGCGCCCATCTGATTTAATGCA
CCCGCCGTCAGGGCAGAAGTGACAATCTGCTTGACCGTATCACTGGTGCCGAGATCTTTC
AACGCTTTGCCGACATCACCTTTATTATTGATGATGGATACAGCTGCTTGGCTATACAAG
GAGGCTAAAGCAGCGGTTTGCATGGCAGTCGCTGTAGAAACGGTAGTAGCTGCTGCTGTC
GTTGTGGCGGCTGTTCCGGCAGCTGCGGCTGTACTACTTCCTGAAGCGGCTACACCGCCC
GCTGCGGTTGCGCCGTATCCATAAGTCAGTGCGGTTACGATTATGGTAACAATCGCTGCA
CCGGCTCTGGTTAAGCCTTCCTGCTTATAGTCCCATTTATCGTAAGCCAGTTGCACCTGG
TTCCAGTTGACGTTTTTCGCTACTTGGAGCTGTTTCAGATAGGCATACTCGGGCTGTTTG
GCCAGCTTTTCGATTTCGGTTTTCAAATTGCCTTTCGGAATGTCGACGATATAGCCACCG
GGGGCGGTCAGTTTGGGCGGAGTAGGGCTTTCGAAGCTGGGCAGTTTCAGCGTTTCGATA
GTGCTGCCGCGTCCGGCCTGTTTCTGCCATACGGTTGAGTTGGTTTCTAATTTTTCTTCC
GACTGGATACGGTTCACAATGCCTTTGAGGATAATTTTCGCATCGGCACGGGCTTTTTCG
CCTACACCTGCCTGAATGTCCGCACCGGCCAGCGTGGTTTTGAATTCGGTACCTTCGAGC
ACGGTATCCCAGCCTGAACGGGTGGCTGCAGTTTGGGCGACGACGCGGACAGGCAATTTG
GTTTCGTTCAGTTCGTTTTTACTGTAATTGCTCTTGCCTACCTTGATGCCGATAAAGCGG
CGGCTTTTTTGGACATCCAACTCGTGCTTGTGGATGCCTTCTTCTGCCAGCAGTTGCAGC
TCTTCACCCGCAACCAGGGTAACTTTACCTGCAGGGGCATTGAAGCGGGTGGTATTAGCT
TCGATGTTGCCGCCTGCCTGAAGCGTTATGCCGTTGGCGGTCAGCTCGACGGGGGCTGGC
ATAATCAGGTGGTCGCGGGTGCTGGTAAACTTGGTTTTTCTGATGATTTTGCCGCTTTTA
CCTTTGGTTTTTAAGAAGGTATAGGCATCGTTTTGTCCAGCCTCCAGTACAATATCACTA
TGGGCTTTGATGTCTATGCTGCCTGAGGGAGCTTTGATTTCGGATGCACCGATAATAATA
CGTGCATCATCGAGTGCCGCAGCTGCATGAATACTTACCCCTGTACGTCCGGTCAAACGT
GAAGGCTTGTTCAGAGCAGCTTTGTCGTAGTGACTCTTGTAGGTGGGCTTGCCAATTTCA
TATTGGTCGGTTATGCCGTCAATCAGAATAGCAGCCGCCTCTGAATCTGCTGCCTTTGGC
AATACGCCTGCGGCGTGAAGGTTCAGTTTTTGGAAGCGGTAATATCGGAACCGCTGATT
TCGATGCCTTGTGCGGAAATCAAGTCAATATTTTGTGCAGAAAGCTTGGCTTGCAGGTAT
TCTTTGCCTTTGGGTTTTTTACCTTTAACTTCCTTGTTGATGGCTTGAATATAGAAAGCG
AGACGGTCGCGTTCTTCTTGCAGGGTTGGAATCAGCTTGCTTTTAGGCGAGCTTTTTTTC
AACTGCGCAATCTGCTGTTCCAATTCTTTGGATTTTTGGTTGAGTTCAGCCGCTTTTTGT
GTAGGAAAATAATTGCTGAATGAGTTGTTTACGGCTTCGATATTCAACTTGCCTTTGGTG
GTGGCGACAACCAAGTTTTTACCGGCTGTAATTTTAGAACCTCTTAAATCTGTTTCTCCT
GTAACCAGACGGATATTGCCTTTTGCTTCCAATGAGGAAACTTGAGCACTAGGCGCACCT
GCATTTCCTCCTTTTGCAGACAACAGCAATTTTCCGCCTGTTTTGATGCTCAGGTCGGTA
TGCGCACTGATGCGGTTGGCAGGTTCGATGGTTAATGTGCCGCTACCTGCTTCAATATTC
AGCCGTCCGGCCAATGGTTTTAATTCGGCATTATCTTCCAAAGTTTTGGTCGAAACGGTA
CTCCAATTGATGTTGCCGCGCTTGACTAGGGCGGTACCGGCAGTAAGGTTGATTGCACCC
GCTCTCAGCGTGGTGTTGTCGGCAATTTGGGAATAGCGCGCATTGAGTGCCAATACACCG
TTAGCCACCAGCTTGTTGGCAGAAGGCAGTTTGTCGTTTTGCCAAATCTGGCTGCCGGTA
ATGCTTAGATGACGGTGTGCGTAGGCATCTACTTGGTTGAGCGTTACCCGCTCGTGTTGT
GCATTAAGATGCGTATTATGGGTAGACTCCAGCTTGGTATTTTCTATGCTCAATGCCCGG
TCGGAATGAATGTTCAATGCCCCGCTTTTGGCATGGACGTTAAGGTTTTTTAAGTCGGCA
~~TTACCACCGTTGTTTTTGATGCTGATGTGTTTTCCGTTGATTGAATTGCGTTGTTTTCCG~~
TCACCAAGCTGAATACCGTTGCCGGCAACCAACGTAATATCTCCTGAAGATGAAGTGATA
TTGGTATTGTCTGCTTTCAGACGGCCTTTACCAACCGATCCTGCATTGACATCGGCCTTG
GCTGTCAGGGTATTGTGACCGGTAAAGTCGGCATTACCGTTGGCCAATAAGGATACATGA
CCGTCTGCAGAAACAGCATGAAGGCCGTCTGAAACGATATTGCCCTGCAATGCGGTGGTT
TCGAGAGCCTTGGCTGCGTTCAGCTTGGTATTGCGAAGCTGAATATTGCCTTTGGCTGCC
TGAATGTGCAGATTACCCGAGTTGGTACGCAGATTGGTATTGGTAACATTCAGCGAGCCT
GCCTCCACACCCATGTCTTTTGAGGCAGTGAGGGTTTTACTGGTGCCGGTAATATGGGCA
GCGTTATCCGATTTCAAATGGATGCTGGCGGCAGACAAATCTTTATCAACATTCAAATTC
AGATCTTTACCTGTATGAACATACAGATTGCCGGGAGTATTCAGATTGGTAGTTTTGGCA
GTAATGTTATCGTCTGCCAGTAAAGCAAGCTGCTTGCCGCCCTTGATACTGCCTCCGTTT
AAGCGGATATCGGAGGTAACTGTTGAAGCTTCCAAAGAAAGCGGTTTGCCTGCTTCGATG
TGTGCGGTGTCTTTGGCATCTATTACGGCGGAACTGCTGATGGTGCCGTTGGATAATACG
GTAACATCTGCCCCGGTAATGCGTGTGTTATTGCCTAATTCGGCGTTGCCTTTGCTGGAA
CTGTATACGGTAGTGCCAGTCTGAATACTGGCCTCCTTGATGACGGTACGGCCGTCGGCC
GACAGAGTAGCCGGGCCTTTGGCATTGTTCACATTAGTTTTGCTCTCAATCACCAAATTA
TGACCAGCATTTAATACCGTGGTAGCTGGGCGACTGCCGTTATTCTGCACCACGGCTCCG
TTACGCAAGCTGATATCTTCTCCCGTCTCAATAACCAATAAGCCTTTGCTCTCGATCCGA
CCACCATTGGAGATAAATGTGCCTGCCGCTCCTTTTTCGGTGGTTTCGATGGAGAGATAA
GTCGGTGAAGCTTCGGTGCCGTCGGCAGTGGTGGCGATGCGGCCGCTGTTTTCAATGCGG
CCTGACGAAGTCACAATCAATTGCTTGGCCGCTTCGAGTGTGCCGGCATTTTTGACGCCT
ACGCCTTTTTCATTGGCAATCAGTGTGATGCTGTCGGCGTACATACCGCCCAGTGCGGCA
GTATCAAGGGCAATAGTCGGTTTCGTACCCGCTGCCGTACCTGCACTGATTTCGCCGCTG
GCGTAATCTACTTTCTGAGGACCGGTAGAAACCGCCAGGTTTTTACCCTGTAATTTCCCC
TGCAAAGCAACTGCACGAGCAAGTACCCCGGTGTAGTCGGCTCCGCCTTTATCATTCCAA
CCTGCTGCTCCTACGGTCAATGTGCCTTGACGCACATCAAATCCTGTCAGTGCACCGTCT

## Appendix A

TTGCCGATTTGGGGCGCACCGGTAGTTAAGATGCCCCGACCGACATTTTTAAAGCCGCCG
CCATTAACGGTAATGCCGTTGGGGTTGGCAATAATCACGTCGGCCTTTTGACCGCCTACG
GTAACGATGCCGTTGAGTTTGCTAGCCGTACCGCGTACCTCGTTCAAAATCAATTGCGCA
CTGCCTTTGACCACAAACGGATTATTGTTACGGTCGTTGTTTAACACTGCCCCTTTGTTG
TCAACATCAAACTGCGTATAGCGGTTGTGGCTCAATCCGCGTCCATTCGGAGTTTGGATA
TTCACCAAGGGGGCACCAGTGTTGGTTTTAAGGATAACGACCTGCTGGTTTTTAGGTGCT
GATTTGTCGGTGGTAATTTGGGCATGGGCAGGCAATACCATACTCAGGGAAACCAAAGAG
CAGACCAAAGTTTTAAGGGTGGTTTTGAGTTTGCCGCAAAGGTCGCCTGAAGTTTTCAGT
GAAACAGAAACCGAACTGCCTGCCTGTTTACCTTTGCCCTGGCTGTTGGCAGTTTCGGCT
ACTGCAACCATGGTGCTGTGCTTTTTACTAAAGATAATGCGATGTAAACCTTTATTCATG
TCTATTCCATTTTGAAGATGAACGTACTGCGCGCCAAGTACGTAGGTAAAGTTTGACGGT
CTGAGGATAAGGAAAGACCGTCTAAATATCAGTAAAAAATTCAGAGGTTAGAAACTGTAA
TTCAAGTTGAAGCCGTAAACGGTGTTGGTCGTCTGAAAGCCTTTGGGTTTATGAAGCGGC
TTGCCGGCAAACAGATCATAAGCAAACATACCGCCTACTTTATGCCCTCCTCTGAAGCCG
ACCACTGCACCCATCAGCTGCTTGCCCGATACATATTGTGCACTTTCGCCAGATACGCGG
CCATAGTCCGCACCGAGATAGAACTGATGGTTCGGATGAAAATACCAAGTTAAAGTATTC
TGCCAGTAGAAACCTCGCTCTCCGAAAAGACTCTGCTCCCCATCAAATCCGCGAACGGTG
TAGCGGCTGCCGATTGACAATTTATCTTGGGCAACCAACGGCGTTTTGTTCCATTGAGCT
TGAATGGCGGTTGCGTAGAAAAACTGCTGTTTGCCTAAAATAAATGGGGCGGCTGCGTCC
AAACTGGCAGTAATGATTTTCATACGAGATGTACCTGGAAGAATATCGCCGCCGTTTTCT
TCCGGTGCAGGCATACTTTGGCGCATGCCGGTCCCGCGTTTGTAAGACAACTTGCCGTCA
AGCTGCCAACGGTTGAGGTAAGCACGGTGGCGCAATTCGGCTTCCCAGCCTGCAGAGCGG
CGGCGTTGTACTTCGATTTCGGCATCGTCGATGTATTTATAGGTTTGGCGTGTCCATAAT
TTCATTCCGACTGAAGTTTTATGAAGTCTGTTACGCCAAAGCATGCGCTCGGCGGCCAGG
CTGCTCTGATATTGTTTGCCGTTGTAATCGTAATTGACGGAATAGCCTTCGGTTGCTTCG
TGGTAACGATGTCCATTGTGATTAAAAGAAAACAGCCATTTTTTTACGGGCACCGAATAA
TGCACGCTGTAACTTCTGGATCCGCTTTCAGTTTCCGTACCGGTGGCATCAGTCAAGTCC
GTTTTGTGCGCCAAACCGCGTCCATATGAAACATAAAACAAATCGCTTAAGCCCAAAGGG
TTATCGAACGATAAAGCGACATTTCCTTGATATTTGCCGGTCGTTTTGCCGCCCGCATCA
TCTATACCGATACTGAACCGTATGGGTTTATTCTGCTGCCATTTGATCTGTAAATCGCTT
TTGCCTTCTTCTTCGGACGGTATAATCTGAATATCTGTTTTAACACTCGGCAAACGACGC
AGGTTTTCCAAGCCCTGCTCTACATCGCGAAGATTGAGAATTTTGTTCCTATATAAGGGA
AATTTGTTATTGAATGCACTAATACTGCCCTCGGCAGACTTCCCATCCCGTTTTTCTTCA
TAGCGGATATCCCCTATTTCGCCTGCTGATACCCGTAATTTCAGAATTCCCGAATCCATA
TTCTGTGGTTGGATAATAGCTTGGGAAGTGAGGTAGCCACGCACGATCAGTATCTGTTGC
GCGGCTTTTTGTAGCCTGCTCAAATTATTGGAACCTAAACACATCCCAGTTTTAAAAGCT
GTTTCTTTCATGAGCACAGAAGGAAGAAAAGAAAATTTGCGCACCGTCTTATCATCTAAA
CTAATGTAATTTACCCGAGTACACGGTGTTTCATCTTCACTCAGGACATAATTGTTCTTC
TCCAATGGTTGCTCGAAACGGACATTTGCATCAGTTAACAATTCAGCATCTATGTGCTGC
TGACGCTGCATGGAACGGATAAGTTCTGCATCGTTTTCATCGGCAGCTAAGGTTTTAAGG
GGTATGACAGCCAGGATAACCAACAGACATGGAGCAGGAAAAAATTTCATGACATCAATA
TTATTTTAGCAATATTTACTATTTTGTCATAAATTTAAAAGTATTTACAGTTATAGAATG
AGACCTTTGCAAAATTCCCCAAAATTCCCACCAAGACATTTAGGGGATTTTGGGGAATTT
TGCAAAGGTCTCGGACAGTATTTTGAACGCAGTGCGCGTAAATTCGTATGGAAACCATGA
AATCCCGCCACAGCCGCCAGACATGCCAAGCCGCATTCTGATATTTCTGTTTGCAGGATA
ACAGGCAGCTTTTTCTTTAAGCCCAAAGACAGGTTTTGCAGATGGGGCATAGATTTCCTT
TTTGAAAAATAGGGATTAGGAAGTTGGATGTATTTTAGAAAGGCCGCCTGAAAAGGTTTC
AGACGACCTTTTGCGACTAGCTGCTATTTTATTTAAAGCTTTTCTCTAACAAACGAGCTA
ATATTTTTGCTGTAATAAAACAGATAAAAAACAGCATCCAATACGTCAGATTGGAAAAAT
CGGTCGTATAGAGAATCAACATATAAAGAAGCAGCATGATGCCGAGTGCGATGAATTGAT
AATGTTTGGCAAACATCATGACCTCCTCAACTATTAAGGCAAACCGCCTGAATATTCTCG
TTCAATCGTTTCGGCAATTTCCCTATAACGTCGATACCATGACCAGTCGAAATTTTCAAT
GGCATGGCTCGCAAACGTACCAAATTCAGGCATCCCTATGCGGCTACCTGCTAAAGCTCC
GATTGTAGCTCCCCAACCAGGCGGATTACTGAACGTATTGTTTGGCAATCCTAAAGATTT
ATCAGGATTTCCCGTATCGTTAAGCCCTGCAGATTCGCAAATTTCGCAATAATATGAGCT
TTGTTGCGCATTACCTGAGCCTCCGACCCAAGTCATTTCATGAACACATATAGTGGATTA
AATTTAAATCAGGACAAGGCGACGAAGCCGCAGACAGTACAAATGGTACGGCAAGGCGAG
GCAACGCTGTACTGGTTTAAATTTAATCCACTATAAAACTCTCATTTTGAAACTCCTTGT
ATCGTTAATCAAACAATCAAAAGGGCAGATGCCCTATCCTTGCTTTTACAAACGGAGTGC
CTGTAAAAGGGGATGGTTTCAGGCAGTTTTGAAGTTTGTGTTTTTATATATTGTCTTCTG
GTCGTCTGAAAAGGTTTCAGACAACTTCTTTATCTTTACAGCCTCAAGTCTTACAGTTTG
CCCGACATACTATAAATCAGCTCCAATACCCATTCGTACAATCACCGTTTCTCGTGTAGG
ATGTCTGCTTCCAACGTCATGCCGATTTGCAGCGGTTTTTCCTCACCGTATGCAGTGATG
GTTGATTTGTCGGGTTTTATTTTCACAAGATAAACAGGTTCGTTGCTCTTCGCCAAATCG
GAGGATACCATGCCCAATCCCGACAATTCCTGTCTGCCCAGTGCCGTTTTTGCTACTGAT
ACGACACTGCCGGAAGCAAGCCCGAATTTTTGATAGGGATATGCCTGATAACGTAGGACA
ACCTTGTCTTTCGGCTTGATAAAGCCTGCTGCACTGCTGGGGATATATAGATGGGCATAT
AGCTCGGTACGTTCGGGAACAATGCTCAAGAGCAGTTTGGAAGGATCAACCTGCTGTCCG
ACTTCGACGTTCGGTATTGCTATATAACCCGACCGTCCTGCACGGATGATTTGTTCAGAG
CGCATTTCAAAATCCAAAACTTCTTGAGAAATATCGGCAATGGTGCGTTCAAGCCAGCTT
TGTTCTGTCTCATGCCGCTTGGGGGAGGCTGGCCAATGTCAGATTCTGCGTGCGGATTTC
CTGAAGCAGCCCGACTTCTTCTCGGCGGTAGGCATCAAGTTTGGCTTTCTGCTCTAAAAG
CTCTGCCTTGACATTCATCATTTCTTGTTTTGGCACTGCATCATTGGCGGATAGGAAACG
ATATTTCTGCAACATTTCTTCCGCAAGTCTAATGCGCCTTTTCTGACCGTCTATCTGTTG
CGAAATATGGAGTTCCTGGTTTTCCAAACGTTCGACAGTTGCTTTAAGGCTGCGCGTTTC

## Appendix A

ATTCCCGTGTATCAGCTTCAGACGACCCAGTTCCTGTTCTGCCAACGTTTTCTTCAAAAC
TGCCTCCGTTTTCAACTGCTGCTGCACGCTACCTCCTGCGCCGAAACGTGAGGTCGAAAG
CGCAAATAGCTTGTCGCCAGCCTTAACCTTTTCTCCATCTTCCACGAATTTCGCTGTAAT
TGTCCCCGTATCCGGTGCATACACCCTGATTACGCCCGATGCAGGTAAAATTTGTCCCTC
CACTGTTGTCTTTCGCGTATAGTTACCAAATATCAAAAACAGGATAATCAATAACGCAGA
TATCGATGCAAATGTCGTCCATAGGGAAAATGACAACGGTCGTGTCAGAATCACTTTACC
CGTCAGGCTGGTTTGGCGGGCAACGGCGACTTCGGGACGGAAGAAGGGTTGCTTGGGTCT
ATTCATAAAATTGAAGTTAAGAAAGTTTCAGACGACCCCTAGAGATTGTCTGGACGATGA
GAAATATCAGCAGTAATCTGTACCGTCAGTGTAGCCGTTTCCTGATTTATCTGCTTTTGT
TGCGGGAGCAGTTAATCCATGTTCAATCTCAAAGATTGGTCTTCCGTTATAAGGAGGTGC
ATTAACGGCATCATTTACCCAATTACGAGTCACATTGTATACACCATTTGCACCAGCAGC
ACCGTAAGCATTTTTCGGCAGATAATAAACTGCCGCTGCGGCAGCAGGTATTGCAACCAA
ATCCCCCCATGTGGGACCTCCTTTGGTTGTGGCAGCATTAGCTACATTTCCAGCTATATT
GTCTGTTACAGGACCTCCCCCTGAAACCAGCTTCAATTCATGAAGTTGAAGTTCAATCAT
TTTTATACTCCTTTTCTTGGTTGGTATTCCTAAAAATTCGGCTAACAAAAACATATGGCA
GATATATTGAAAAAAAATTCAAAGTACCCTGAATAAAATTCAAATTCCAACTATATTTGT
TAATGTAGTCGAGAAGAAACATATCTGATAAAAAATATAGCACTTGATAACAAGCTATTA
CTAATATTACGAAAAATGTAAATTGCTTCCAGTTTTTCATAGAATCCCTCACAAAATTTC
CAGAAAATCTAACTCTATCAACTGATAAATCAACTTCCTAACTTCTTCATATTTTCCCTG
ATTGAAGTTAACCAGTAGATTTTTCAACAATAACGGTTCATTCTTACCGATGTGTTCTAA
CACTTTTTTCCCCAACTCATCTACGCTTATCTTCATCCCATTCCCAATCAAATATCCCTT
TTCCAACGTATCCAAATTATTGGCATTTAATCTCAACCTGACGTCGTCTGAAAGCGGAGT
AGCGTTGGGATTCGCGAACTGTTCGAGATGAAAAGCGGTATCGGTACGTTCTTTGCCGAG
AAAGTCTTCACTGAAGGCTTCATAATTGACGGGGTCGGCAATCATGGCAGCAATTTGTGC
GGCAGTATCGTTGATACGCGTCCTATCTTGCTCCCAGTCTGAGAAACTGTGGCGCAGACT
TTCTATGGTGGGAAATTTCTTCATTAGCCACTCGAGGTAATTATAGCCGTTGGGTGGAAA
GGTACCGACAGCGAAGTGGAAGGTTTCACAGCCGAGCGGGATAGGTCTGTGCCACCAACC
GCGTGGGATGTAGAGGACATCACCTGCTTCAAGGATAATATCCATATCGATATGTTCAGG
AATGGAAATATCAGTATCTTTAGTCTGTTGCATATACAATGGCATAGGGAAATCAGGGGC
AGTAAGTTGCCAACGTTTCTTGCCGAAAAGCTGGATGGCATACACATCGCGGGGGTCCCA
ATGGTTTTTATAAGATTCGTCGCTGCCAAAAGCAAGATATCCACTAACAATAGTATGTGC
GCCGGCAAAGCGGGCGACTTGACGGGCGATATGGTCTGAAAACGGCTCGTTGTTAATATG
GTTATAGACTAACGACGCACCATTCTTCATATGTTCGTAGATAACGGATTTAATAAAACG
GTAGCGAGTTTTGCCCAAATCGTCGAAACTTTCGACGTATTCTTCTTTAGGAACGATTGC
GCCTTTTTTACGCAGATGAAACAGCGGTGCGGTTGGGTCTGCTCGTTGGTATATCTCGTT
GATATCTTTCCAAGATGCGGATTCGAGATTCCGAACCGCTCCTTTAAAGAGCTTGGGCTT
TTGATACAGATAAGTCTGTCGGAACGTTTTAGGACTAATGCCGAAGTCGAGATGGATGCT
CATTACTTCCCCTTACTCAGAAAATATTTAAAATTTATAATGTTACATATATTTACAAAT
ATTAAAGTTTTTTTTTGTGTGTGCGTCAAGGAATTGTTGACAATTTTAGTTAAAAATTTG
TCTCCAAACGGAAAAAAGCGGTTTGTTTTTTGTTGTTAACATTTTTATTGTAATTAAATA
AAAGGTCGTCTGAAAACGGTTTTCAGACGACCTTTTGCTATAATCGGGCTTCATCGCCCC
GTTCGGTTTGGAACCTTATGAAAACCCTCGTCCTCCTCCTGCTTTCCTTCTCCACGACCA
CCGCTTTCGCCGCATACGGTTTGGGTTTGGGGCAGGCACCGAAATATCCTGCCGGCTTTC
GCGCCTACGGTTATGTTTATTCCGGACGGCAGGGCTAGGTTTTAAAAACAGAGGCGGATG
CCATTAAATTAGACACGCTTTTCAAACGCTTTGTGTACCGTCCTTCCGCCGCCAATCAAA
ACCCCGTCGGACAGCGTTCGGACGGCATACCCGCCAACCACACAAAGGAAAAACCATGAG
TAAAAAAATCAAAGTCGGCATTGTCGGCGCGACGGGCTACACCGGCGTGGAACTGCTGCG
CCTGCTTGCCGCCCATCCCGATGTCGAAGTCGCCGCCGTAACCAGCCGCAGCGAAGCGGG
AACCGCAGTTGCCGATTACTTTCCGAGTTTGCGCGGCGTGTACGGCCTCGCCTTCCAAAC
GCCCGACGAGGCAGGTTTGGAACAATGCGACATCGTCTTCTTCGCCACGCCCAACGGCAT
CGCCATGAAAGACGCGCCGCGCCTGATTGAACAGGGCGTGCGCGTCATCGACCTTTCCGC
CGACTTCCGCATACGGGACATTCCGACCTGGGAACACTGGTACGGCATGACCCACGCCGC
CCCCGACCTCGTTTCCCAAGCCGTGTACGGATTGAGCGAACTCAACCGCGAAGCCGTCGC
ACAGGCGCGCCTCGTCGCCAACCCCGGCTGCTACCCGACCTGCGTATCCCTACCGCTCGT
GCCGCTGTTGCGGCAATGCCGTCTGAAGCCCGGTATGCCGCTGATTGCCGACTGCAAATC
CGGTGTGTCCGGCGCGGGCAGGAAAGGCAATGTCGGTTCGCTGTTGTGCGAAGCCGGCGA
CAACTTCAAAGCCTACGGCATAGCCGGACACCGCCACCTGCCCGAAATCAGGCAGACCAT
CGCCGGGCTTCAGGACGGCATCGCCGAAGGATTCGTGTTCACGCCGCACCTCGCGCCAAT
GATACGCGGTATGCACGCCACCGTTTACCTCCACCTTTCAGACGGCAGCGACCCCGAAAC
CGTCCTGCGCGACTACTACCGCGACAGCCCGTTCGTGGACATCCTGCCGACCGGTTCCGC
CCCCGAAACCCGCAGCGTGCGCGGCGCAAACCTCTGCCGCATCAGCATCCAACAGGCGGC
GCAATCCGATGTGTGGGTCGTCCTTTCCGTCATCGACAACCTCGTCAAAGGCGCGGCGGG
TCAGGCAGTCCAAAATATGAACATTATGTTCGGACTGGAGGAAACACACGGCTTGGACGC
AATCCCCCTGCTCCCCTGAAGCGCAAACAGCAAACCGCAGGCATCGTGCCTGCGGTTTTT
GATGCCGTCTGAAAGCGACGTTTTTTTGGGTTCGGACGGCTTTTGACCCATCCATTCACA
CGAAAACAAAAATCTAAAATACCGTCATTCCCGCAAAAGCGGGAATCTAGTTTATCCAGC
TTCAGCAATTTCCGACACATTTCCACACGCTTCGATTCCGTCATTTCTCCGGTTTCAGTC
ATTGCCGATAACACCGTGGTTTTTCATTTCTAGATTCCCGCCTGCGCGGGAATGACGGCG
GAGGGCTTGCCGTTTTTCCCGGTAAATACCTGCAATTTAAAATCCCATCATTGCCGTGAA
AACAAACCAAAAACCTAAAATCCCATCATTGCCGCGAAAACAAACCAAAAACCTAAAATC
CCGTCATTCCCGCAAAAGCGGGAATCTAGTTTATCCGGCTTCAGCGATTTCCGACACATT
TCCGTACGCTTCAATTTCGTCATTTCTCCGGTTTCAGTCATTGCCGATAACACCGTGGTT
TTTCATTTCTAGATTCCCGCCTGCGCGGGAATGACGGCGGAGGGCTTGCCGTTTTTCCCG
GTAAATACCTGCAATTTAAAATCCCATCATTGCCGTGAAAACAAACCAAAAACCTAAAAT
CCCGTCATTCCCGCAAAAGCGGGAATCTAGTTTATCCGGCTTCAGCGATTTCCGACACAT

# Appendix A

TTCCGCACGCTTCAATTTCGTCATTTCTCCGGTTTCAGTCATTGCCGATAACACCGTGGT
TTTTTATTTCTAGATTCCCGCCTGCGCGGGAATGACGGCGGAGGGCTTGCCGTTTTTCCT
GGTAAGTCTCTGCGGCTTCTCATTGCCGGTTTCCGCCTACTTGGGAATGACGTGATTTAA
AATCATGAAAATGTGTCAAAAATAATATAGTGGATTAACAAAAACCAGTACGGCGTTGCC
TCGCCTTGTCGTACTATCTGTACTGTCTGCGGCTTCGTCGCCTTGTCCTGATTTTTGTTA
ATCCACTATAAAAATCAGATTTCCGTTACACTTTTTTCCAATATTTCAGACGGCATTTTG
CTCACACGCCCAAATACCCTTCCCTGCCGGAAAGCCACCTTGCCAAATGCGCTTCGACGA
TTTCGGGGTTTTGTTCAATCAGCATCGGGGCGGTTTCGCGCGCTTGTTCCAAGAGGTGCA
GGTCTTCTTCGAGCTTGGCGAAACGCAGCATAGGCACGCCGCTTTGGCGCGCGCCGAGAA
ATTCGCCGGGGCCGCGGATGTTGAGGTCTTGGCGGGCGATTTCAAAGCCGTCGGTGTGTT
CGTAGATGACTTTCAGCCGCGCTTTGGCGAGTTCGCCCAAGGGTTCGGCAAACAGGAGGA
CGCACACGCTTTCTGCCGCGCCGCGCCCGACCCGACCGCGTAATTGGTGCAGCTGCGCCA
AGCCCATGCGCTCGGCGTGTTCGATGACCATCAGGGCGGCATTGGGCACATCTACGCCGA
CTTCGATGACGGTGGTGGCGACCAAGACGTTCAGCCCCCCCGAAGAAAACCGCGCCATCA
CTTCGGCCTTTTCGGCGGCCTTCATGCGCCCGTGTACCAGTCCGATATTGAGTTCGGGCA
ATGCCGTCTGAAGCCGGGCGAGGGTTTCGGCGGCGGTTTGCAGTTGCAGGGTTTCGCTTT
CTTCAATCAATGGGCAGACCCAATACGCCTGCCGCCCTTTTCGGCAAGTGCCGAGGACGA
AGCCTTCGACTTCGGCGCGGCGGACGTTGTTGACGAGGCGCGTTTTAATCGGTGTGCGCC
CGGGCGGCAATTCGTCGATGACGGACACGTCCAAATCGGCGAAAAAACTCATCGCAAGCG
TGCGCGGGATGGGCGTGGCGGACATCATCAGCTGATGGACTTCGCGCCCTTTGTTTTTGA
GGGCGAGGCGTTGGGCAACGCCGAAACGGTGCTGTTCGTCCACAATGGTCAAGCCCAAAT
TGTGAAACGCCACGCCGTCTGAAAACAGGGCGTGCGTGCCGACGGCGATTTTGACGCTGC
CGTCGGCGAGTTTGGCTTTGGCTTCGTCTTTGGCTTTTTTACGCAAACTGCCAAAAAGGC
GGACAACTTCAATGCCCAAAGGTTCGAGCCATTGTTTAAATTTAATAAAATGTTGTTCGG
CAAGGATTTCAGTGGGCGCCATTACAGCCACCTGCGCACCGGATTCGATAGCCGTCAAAG
CAGACAAAGCAGCCACAATGGTTTTGCCGCTGCCGACATCGCCCTGCAGCAGGCGGTGCA
TCGGGTAGGTTTGCGCCATATCGCGGCAGATTTCGGAAACAACTTTTTCTTGCGCATCGG
TCAGGGCAAACGGCAGGGCTTGGCGCAGGGCTTGGGTCAATGTGCCGTCGCCGCCCAATG
CCGCCGCCGTGCCGCCGATACGCTTCTGTCGCGCCAAGCGCATCGAAAGCTGTTGCGCCA
AAAGTTCATCGAATTTGAGCCGTTGCCATGCAGGCAGCGTGCCGTCTGAAAGCTGATGAA
TCGTGAAACTCGGCGGCGGCGAATGCAAAAGACGCAGGCTTTCGGCGAGGTGTGGCAGCT
TCAGACGGCACAGCAGGGCATCGGGCAGCGTGTCGTGCAGCGGCGTAACGTCCAACGCCG
TCTGAATAATACGGCGCAAAGTGGGCTGGTTCAAACCGTTTACGGTCGGGTAAACCGGCG
TGAGGCTTTCCGCCAAACCGCCGCCCTCGGCATCGCGGATTTTGGGATGAATCATCTCGT
CGCCGTAAAAGCCGTGTTTGATTTCGCCCACGGCGCGGATGCGTTTGCCGACCGCCGTCT
GTTTCTGATGGCTGGCGTAAAAGTGGATGAAGCGCAGAAAAAGGACGCTGCCGGAGCCGT
CGGCGATTTGGACAATCAGCTGCTTGCGCGGTTTGAACGTTACTTCCTGATGGATAACCT
CCCCCTCGACCTGACACGGCACGCCAATCGGCGCGTCCTTAATCGGCATAATGTGCGTCT
CGTCCTCGTAACGCAGCGGCAGGTGCAACACCAAATCCCACGCGGTATGGAGGTTGAGTT
TGTCGAGCTTCTTGGCGGAAACATCGGTGATTTTGAGCTGTTTTCGGGTTTCGGGCGACA
TCATAGGCAGATTCCTTTGGACGCGCCTATTTTATCCGAAAACAAAAATGCCGTCTGAAA
CGGATTCAGACGGCATCGACAGGCAGGAATCAAGCCCCGGCGGCTTCGGCTTCCTGCTGT
TGTTGGTAAATCGCCTCAAAATTAATCGGCGCGAGCAGGACGGGCGGGAAGCCGGCGCGC
GTTACCGTACCGGAAACGGCTTCGCGCGCGTAAGGGAAGAGGATGTTCGGACACGCCACG
CCGAGCAGCAGGTCGGCATCTTCTTCGGGGATGTTTTCCAAACGGAAAATACCGCTTTGG
GTTACTTCGTTCAAAAACATCGTGCGCTCGTTATCCAATTTGGCGGTTACGGTAACGGTT
ACATCCACGTTGTAGTAGCCGTCTTCCAGCTTTTGGCTGCCGGTGGAAACGCGCATCTCC
ACTTCGGGCTCGCCCTGTTCCAAAAAGATTTGCGGCGCGTGCGGCACTTCCAAAGACAAG
TGTTTGACATACAGTCGCTCGATGCTGAATACGGGTTGCAGTTCTTCGCTCATTTTGTTT
TCCTAGTTGGGGGTTAAGGGTTCAGCAGTCCGTCCAGCCCGCCTTCCTGCTGGAGGCGGT
AGAGGTCGGTAAATCCGCCGACGTGCGTTTCGCCGATGAAAATCTGCGGCACGCTGCGCT
GTCCCGAAAGCTGCTGCATTTCGGCAAAGGCTTCGGGGCTTGCATCGACACGGATTTCGT
CGATATGTCCGACACCTGCCGCGTGCAGCAGCCTTTTCGCCATCGCGCAGTAGGGGCAAA
ACGGACCTGTGTACATGGTAACGGTCTGCATATTGGGTTTCCGAAAGTTTTGCAATGATA
ATCAATATAGGGGCATTTCCCCTGTTTGGCAAGTGCGGAACAGATGCACGTTCAAACGGC
ATGTGCGGAATGTGTCAAAGTTTCTTTTTTAAAGTATGATAGACATTGTGAAAAATATTT
TTGCACCCGCGCTGCGCGGCGGAACGGATGCAAAATATTTTTATTACATTTTCAGGAAAA
ACCATGTTGTCAGGACTCCCCATCCCCAAAGACATCGCGCGCCCGCCCGAAACGATATTG
GTCAACATCACGCCGCAGAAACGCGCGTAGCGGTGTTGGAGGAAAACAATATCTGCGAGC
TGCACATCGAGCGCAACAGCGAACACAGCCTAGTCGGCAATATCTATTTGGGCGTGGTGC
GCCGCGTGCTGCCTGGGATGCAGAGCGCGTTTATCGACATCGGCTTGGAACGCGCGGCGT
TTTTACACATCGTCGATGTCCTCGAACAACGCCGCAACCCCGAAGAAACCCAGCGCATCG
AACATATGCTGTTTGAAGGGCAGTCTGTTTTGGTGCAGGTCATCAAAGACCCGATCAACA
CCAAAGGCGCGCGGCTTTCCACCCAAATCTCGCTGGCGGGGCGTTTCCTCGTCCATCTTC
CGCAAGAAGACCACATCGGCGTGTCCCAACGCATCGAAGACGATGCCGAACGCAGCAGCC
TGCGCGAACGCCTCGACAAGCTCCTGCCGGAAAATGCCTGCCGGGGCTACATCATCCGCA
CCAACGCCGAAAACGCCACCGACGAACAGCTCCAGTCCGACATCGACTACCTGACCAAAG
TGTGGGAACACATCCAAGAACAGGCGAAAATCCGGCCGCCCGAAACCCTGCTTTATCAGG
ATTTGCCTTTAAGCCTGCGCGTGTTGCGCGATATGGTCGGCTGCGACACGCAAAAAATCC
TCGTCGATTCCACCGTAAACCACGGGCGCATGACGCGTTTTGCCGAACAATACGTCCACG
GCGCATTGGGCAGGATAGAGCTGTTCAAAGGCGAACGCCCGCTGTTTGAAACCCACAACG
TCGAACAGGAAATCAGCCGCGCCCTGCAACCGCGCGTCAACCTCAACTTCGGCAGCTACC
TGATTATCGAATCCACCGAAGCCATGACCACGATAGACGTGAACACCGGCGGCTTCGTCG
GCGCACGCAACTTCGACGAAACCATCTTCCGCACCAACCTCGAAGCCTGCCACACCATCG
CCCGCGAATTGAGGCTACGCAACCTCGGCGGCATCATCATCATCGACTTCATCGATATGG

## Appendix A

CACAGGAAAGCCACCGCGAAGCCGTGTTGCAGGAGCTTGCCAAAGCCCTCGCCTTCGACC
GTACCCGCGTTACCCTGCACGGTTTTACCAGCCTAGGGCTGGTCGAGCTGACGCGCAAAC
GCTCGCGCGAAAACTTAAACCAAGTCCTCTGCGAACCCTGCCCTTCCTGCCAAGGCAGAG
GCCGTCTGAAAACGCCGCAAACCGTATGCTACGAAATCCAGCGCGAAATCGTCCGCGAAG
CGCGCCGTTACGATGCCGAAAGTTTCCGCATCCTCGCCGCCCCCAACGTCATCGATTTGT
TTTTGGACGAAGAATCGCAATCCTTGGCAATGCTGATAGATTTCATCGGCAAACCGATTT
CTCTGGCGGTCGAAACCGCTTACACGCAGGAACAATACGACATCGTTTTGATGTAAAAAA
TGCCGTCTGAAGCCTTCAGACGGCATCTGTCTATTTCAGGGTTTCCTTGTCCAACAACGC
GCGTATCAGCAGACCGCGTCCGAAACGTCGGCTGTCGGACAATTCCAAATATCCGCCGTA
TTTTTTGGCAAGCGTGTCGGCGATGGACAGACCCAGCCCCGTCCCCTGCTGCTCCGTTCC
CAAAATACGGTAAAACGGATCGAGGACACGGGCGCGTTCGGATTCGGGAATGCCTTTCCC
GTTATCTTCCACCCACACGGCAAGATATTTCCCTTCGTCCGTGAAACCCAAATCTATCCT
GCCTTCGGGCGGCGTATAACGTACCGCGTTGTCGGCAAAGGTTTTAATCAGCGTATAGAT
TTCCGTTTCGTCGGCAGACACTTCGACATCGCCTCCGACCGCCACGCCGATGTCCTGACA
TTTTTCCAAAGCCAGCGGCATCAGTTCCTGCAACACTTGGCGGAAACGGCTTTGCAGACC
GAATGTCGTTTTCGTCAGAGGGATTTCATCCGACTGCGAACGCGCCAATGCCAAAAGCTG
TTCGAGCAGGTGTTTGTTACGCCGTATGCTTTGCTGCAAAACGGCAGGCTGCCGCGCCGC
ATCGGGTGGGAGCGGCATATTGTTGAGCCGTTCCGCCTGAAGGGGAAGGGCGGTCATCGG
CGTACGCAATTCGTGTGCCGCGTCGGCGACAAACCGCTGACGGTGGCGGATGTCTTCATC
CGCACGTTTCAAAAGCAGGTTGATGGCGGTTACGAAACCTCTGATTTCACTGGGAATATT
GTCCACACTCAAAGCAGACAGGTCATTGATTCGGCGTTGTTCGAGACTTTGCGACAATTT
GCGGACGGGGCGCATGGCTTTGTGCGTAATCCACACGGTCAGCAAAATCATCAGCGGCAG
TGCCGCCAACAGGGGCAACACGCTTTGCCGTGCCGCATCCGCCGCCAAATCTTCACGGTA
TTCGTTTTCCTGCATAACGGCAATCCGTCCCTGCTCGGTCGTGCGGATATAGACGCGGTA
ATAATCGTCGTCATCGTCCGCCTGAAGCGTGTGCAGACCGTCCGCCAGATGCGCAGGCAG
GCTGACAACAGGGTCTTCCTGCTGCGGCATCTGTACCAAAATACGCGTATCGCCGTCGCC
CTCGGGCAAAGTTTCGGGTTTGGAATCGGGGGCGACGTACAATGCCGCCTGACGGAGCAG
GTCGTCCTGCAACGCTTCCGTTTCGTGGAAGGTTTCGTAGTAGGAAAACATACCTGCAAG
CATTGCCAGCGGAACAAACATCCAAACCGTCCCGCCCCGGCAAACCCAAATCCAGCAGCA
TCAAGTCATAAGGCTGGGCAGCGGCAGCCGCGCGCCGTTTTTGACCCAATCCACCGCATA
GCCGCCGTCTTTCAAACTTGCCGACACCGCCTCCGCAATCATCGCATCGTCTTCCACCAG
CAAAACACGCATCAACTTTCCCTTCAAAATAAACCGTGCCTATTCTAACACCCCAAAATT
AGCCGCAATTTAGCGGTCTTTACGCTTGCCGGTATTTTTCAAAACTGCAGCACAAAAAAA
CCGCGCCGGCAACTGCCTTCAGACGGCATTGGGGCGCGATTGCAACACACGGGCAGGCAG
GCAGAGCCTGCGACAGACCACAGGAACGATTCAGGCTTCAGACGGCTTCGCCGTTTACGG
CAGAGGCACGATTCCTGCCGCTATCGAACTGGCCAATATCGCCAGCGACAAACCCCACGC
CCAGAAAAACGAATAACGGATGTGTTTGCCCATCGACAATTTCGCCAAACCCAAGCCCAT
CCACAAAGCCGGCGAAAGCGGCGTAACAAAAGTGCCGACGATACTGCCGATCAACATCGC
ATAACCTGCTGCTTCGGGCGCCACGCCCGCCTGCGAGGTAATCTGCTCCACAATCGGAAA
CAGTCCGAAATAATAAGCGTCCGTACTCAAAACCAACTCAAGCGGAATGCCCAACACACC
GATGGCAATATGCAGATAAGGCAGCAGCGCGTCCGGCAGGATATGCACAATGTCTTTGGA
AATCGCGTCCAACATCCCCGCACCCTTCAAAATCCCCAAAAACGTACCTGCCGCCAAAAT
AATGGACGCCATCATCACCGCGCCGCCGGCGTGGGCATAAATCCGCTCCATCTGTTCCTG
CGGGCTGCGGTAATTCAAAAGCAACGCCGCCGTTGCAGCCAGCATAAATACATAACCCGG
TGGGAAGATGCCCGAAAAAAGCAGGCTCATCGCCGCCAAAAACAGCAGGACATTCCACCA
AAACAGTTTCGGACGCGCCAATTTTTGTTCTTCTTCCGACAAAGGCACCGGCTTTATCAA
ATCCGCCACGGCGGGCAACGCGCCCAACTCCCGGACAATCCGCCTTTTTTCACGCACACC
CAAAAGCAGGGACAGCGCAAGGATAAACACCACACCGATAATTTGCACCGTCAACAAAGG
TTTATACAATTCGCCCACATCTGCGCCCAACACGCTTGCAACCCGCCCGGTCGGCCCGCC
CCACGGCAGAAGGTTAATCAATCCCGCACTGGAAGTCAGCAGCAAAAACAGCAGGTAAGG
ATTCATATGCAGACGCTTGTAAAGCGGCAAAAGGGCGGGGACGACCAATAAAAACGTCGT
CGCACCCGCCCCGTCCAACTGCGCCACCACCGACACCAAGACCGTCCCCACACTCACTGC
CACGATATTACCCCGAGTCAGCTTAATCAAACCGCCTATCATCGGACGGAACAGCCCCAC
ATCGTTCATGATTCCAAAAAACAAAATGGAAAACATAAACATAATCACAATCTGCATCAC
CGATTTGGTGCCGCCCGAATAAAATTCTTTTAATTGGGATACATCAAACCCCGCCAGCAA
CGCCCCAAACAGCGGCACCAAGATTAATGCGATGATGGGCGACACTTTTTCCGTCAGCAG
CAGCCATACGATGACCCCGATAATCAGCAGTCCGATAAACGTCAGCATCATTTCTCCTTT
ATTTTATTTTAAACAGAAAACCGACCGTGCAGGCAAAACCGCCCACAGACGCGGGATAAG
CCCTGCATTCTACTTTTTTATTTTGAAACAAGTCAATCGGTCATTTCCTCCCATTTACGC
CTGCCGCCATTCCTGCATCCGTCCGTCATTTCACAGCGGCAACCGATACGGAACAACCGG
TAAATCGGTATCGGGACGGCGCGGGGGCATTCATCCCGGTGCGCCGATTCAAACGAAACC
GCCCCTATCATTGCGGAGCGCGGGGCGTGCCGTACACGCGGGATTTTATAGTGGATGAAC
AAAAATCAGGACAAGGCGGCGAGCCGCAGACAGTACAAATAGTACGGAACCGATTCACTT
GGTGCTTCAGCACCTTAGAGAATCGTTCTCTTTGAGCTAAGGCGAGGCAACGCCGTACTG
GTTTTTGTTAATCCGCTATAAACACGCCGGTCATTTGCCGCGCATTATCCGGCAAACGGC
AAACCTTGACGCTGCCCAGCCCATATAAAAAAGCCGCAAAACCCGAACCGGTTTTGCGGC
CTTTCGACTTGGTTTGTTTATTTGGCATTTCTTTCAATCAAACCGACAAACTGATTAATA
TATGACTGAACAAAATCCCTTGCCGAGTCAATCAATTTGCCGTTTTCATCAAACAGCGTC
GGCGAATTGCCCAAAAACACTTCCGGCTGTCCGGTTACGGGCATATCGAAATAAGACAGC
GCAAGGCGCAGGTTTTTTTGGGAACTGTAACCGCCCATCTTGCCGACGGAATGGCTGATG
ATGCCTGCCGGTTTGTTTTTCCACGCCACGTCGGCATTCGGTTTCGAGCCGATGTCCACC
GCATTTTTCAAACAGGCGGGAATGGTGCGGTTATTTTCGGACGTAACGAACAAAATGCCG
TCCGAAGCCTTAATCGTTTCGCGGAAAGCCGTGTAGCTTTCGGGTAGCGGCACATCTTCC
ACCGCAGGGTCGTCATAATCGAAATTGTAAAGCGGCAGATGTCCGATTTCAACGATTTCC
GCCTGCCAGCCTTCGGGGAACATCTCCGCCGCATTCAATGCCACTTTGCGCGCAAAAGAA

## Appendix A

GCACGGCGCAGGCTGCCCACCAAAATACTGATTTTCTTAGCCATAATCATTCCTCCTGAA
TATTAAGTTTGTGCGTCTCAATCATTTCATAATGATAGCGATTATTATATATGTGATTTC
CCCTGCAAACAAGCCGGCCCGCCGCCACAGCGTTCCCACTTATCCGGCTTTGCCTTATAA
TTGCTTTTTTTATGTAACAGATTTACCTATGAATTTCCCCAAAACAGCGGCCTCCCTGCT
GCTGCTTCTCGCCTCCCTCGCCGCACACGCGCTCGATACCGGCCGCATTCCGCAAAACGA
AATCGCCGTATATGTCCAAGAGCTTGACAGCGGAAAAGTCATCATTGACCACCGCTCGGA
TGTCCCCGTCAACCCCGCCTCCACAATGAAACTCGTTACCGCGTTTGCCGCCTTCAAAAC
CTTCGGCAGCAATTACCGCTGGGCGACCGAGTTTAAAAGCAACGGTACGGTAAACGACGG
CACGCTTGACGGAAACCTATATTGGGCGGGCAGCGGCGACCCCGTTTTCAATCAGGAAAA
CCTGCTTGATGCTCAAAAACAGTTGCGCGAACAAGGCATACTCAATATCACGGGACACCT
GATGCTCGACCACAGCCTGTGGGGCGAAGTCGGCAGCCCCGACGATTTCGAAGCCGACAG
CGGTTCGCCGTTTATGACGCCCCCCAATCCAACTATGCTGTCTGCCGGTATGGTTATGGT
GCGCGCCGAACGCAATGCCGCCGGCAGTACCGACATCCTCACCGATCCGCCTTTGCCGCA
TATTTTCGCCCAAAACAACTTGAAAATTACCGCCTCCCAAGCTGCCTGCCCTTCGATCAA
AAAACTGATGCGTGCATCTTTTTCGGACAATACGCTGAAATTGCGCGGCAATATTCCCGA
GAGCTGTTTGGGCAAGCCTGTCGGTGTCCGGATGTTCGCGCTTGACGAACTGATCCGGCA
AAGTTTTACCAACCACTGGCTGCTCGGCGGCGGACGGATTTCAGACGGTATCGGCATAGC
CGACACGCCGGAAGGCGCGCAGACACTTGCCGTTGCACACGCCAAACCGATGAAAGAAAT
TTTGACGGACATGAACAAGCGTTCGGACAATCTAATTGCGCGTTCCGTCTTCCTCAAACT
CGGCGGCGACGGCAAACTGCCCGCCGTTTCCGAACAGGCGGCGTCTGCCGTCCGGCGCGA
ACTTGCCGTATCGGGCATCGATGTTGCGGATTTGGTTTTGGAAAACGGTTCGGGCCTGTC
CAGAAAAGAAAGGGTAACGGCGAGAATGATGGCGCAAATGTTGGAAACGGCTTATTTCAG
CCCGTTTGCACAAGATTTCATCGACACGCTACCCATCGCCGGCACAGACGGAACTTTACG
CAACCGCTTCAAACAAAGCGGCGGGCTGTTGCGCTTAAAAACCGGCACGCTCAACAATGT
CCGCGCCCTTGCAGGTTATTGGCTGGGCGACAAACCGATGGCGGTGGTCGTCATCATCAA
CAGCGGCCGCGCCGTTTCCCTGCTGCCAGACTTGGACAACTTCGTTGCCAACAACATCAT
CTCCGGCGGCGATGGCTGGCTGGATGCGAAACTGATGTGCAAAGAACGCCGAGCCTGAAA
CAGGAAAATATAGTGGATTAAATTTAAGGGGCTGTCCTAGATAACTAGGACAAACTCGAT
TTTACTAATTGTTTTAAAATGGAACAAGAACTTTTATCTCACTGTTGTTAAAACGCCATT
CGCACTCCTTTAAATACAGCTCAAAATGCGCTTTGGGAATGCCGTTAAACTTGCGTAAAT
GACGTTTTGCCTGATTCCAAAAGTTCTCAATTCCATTAATATGGTTTTGTCGTTCGGCAA
AATGTGTGCTGTGATTGATACGAAAACGAAGTTTCAGCGAAGCTAAAATGGCTAAATTCG
CGCACATCTAATACATCATAGCTACGATAACAATCCGTATAAACAATACTGTCAGGTTTC
ACTTGTTCACGGATAATAGGAAATAAAGTAGCGGTTTGAGTATTCGGTACTGTAACCGTA
TAAACCTTACCATTTCGCTTCAAAAGACCGAATACGGCGACTTTACCGGCAGCACCGCGA
CCGCGTTTGCCTTTGCGTTGTCCGCCAAAATAACTTTCATCTGCTTCTACTTCGCCATCA
AACATTTCCAAATGCGGACTGTTTTGATAAATAAGTAATCGTAAACGATGAAAATAATAG
GCTGCGGTATTTTTATTAACGCCTACTAACTCTGCTGCCGTTCTTGCAGTTACACCTGCG
ACAAACAGTTCAATGAGTTTATTTTGTTTATACCGGCTTAGACGACTTTTTCTCATAGGG
GCAACTCTAACTTAATTTGAATTTCCCTAGTTATCTAGGACAGCCCCAAATTTAAACCAG
TACGGCGTTGCCTCACCTTAGCTCAAAGAGAACGATTCTCTAAGGTGCTGAAGCACCAAG
TGAATCGGTTCCGTACTATCTGTACTGTCTGCGGCTTCGTCGCCTTGTCCTGATTTTTGT
TAATCCACTATATAAAAATGCCGTCTGAACTGTTCAGACGGCATTTTTGATTTTCAAACC
GGAATTACAGCCCCGCTGCCGCCCTCAATGCAGCAGCTTTGTCGGTGCGCTCCCAAGTGA
ACTCAGGTTCTTCGCGGCCGAAATGTCCGTAAGCGGCGGATTTACTGTAAATCGGGCGCA
AGAGATCGAGCATTTGGACGATGCCTTTGGGGCGCAGGTCGAAATGTTCGCGAACTAAGG
CAATCAGTTTTTCTTCGCTGATTTTGCCGGTGCCGAAAGTATCGATGGAAATCGAAGTCG
GTTCGGCAACGCCGATGGCGTAGGAAACTTGGATTTGGCATTGGGTTGCCAAACCTGCGG
CGACGATGTTTTTTGCGACATAGCGGCAGGCGTAAGCGGCGGAACGGTCCACTTTGGACG
GGTCTTTGCCGGAGAATGCGCCGCCGCCGTGCGGAGCCGCGCCGCCGTAGGTATCGACGA
TGATTTTACGGCCGGTCAAACCGCAGTCGCCTTGCGGGCCGCCGATAACGAAGCGGCCGG
TCGGGTTGATCAGGTATTTGGTTTCGTCGGTCAGCAGTTCAGACGGCAGAACCGGTTTGA
TGATGTGTTCGATTACGGCGTTTTTCAGCTCTTCGTAAGCGATGGACGGATCGTGCTGGG
TAGACAGGACGACGGTGTCGATGCGTTTTACTTTGCCGGTTTCGCTGTCGTAAACCACGG
TCAGTTGGGCTTTGGCATCAGGACGCAGCCAAGGCAGGCGGCCGTCTTTGCGCAATTCGC
TTTGACGCTGCATCAGGCGGTGGCTGTAATAGATGGCAAACGGCATCAGGGTAGGGGTTT
CGTCACAGGCATAGCCGAACATCAAACCTTGGTCGCCCGCGCCCTGGTTCAAGTCGATGC
CTTCGCCTTCGTTCACGCCTTGGGCGATGTCGGGGGATTGCTGGTCGTAGTACACGCCGA
CTGCGCAGCCGTTGGCATCAAAGCCCAGCTCGGAGGAGTTGTAGCCGATGCGTTTGATGG
TTTCGCGTGCGACTTTGATGTAGTCTACTTGGGCGGTGGTGGTAATTTCGCCTGCCAATA
CGCACAAGCCTGTGTTGACCAAGGTTTCTGCGGCGACACGTGCTTTTGGGTCTTGCGCCA
AGATGGCATCCAAAATCGCATCGGATACTTGGTCGGCAACTTTATCCGGATGGCCTTCGG
ATACCGATTCGGAAGTAAACAGATATTCGCTCATTGAGATTTTCCTTGAAAAACAAACCA
TCTTCTTCAGACGGCATGTTGTATGAACATAATGTCGACAGCGGGAAATATAGCAAAATT
TCCCTATTCATACCATTCAGTTGAGAAATATTCCCATTTGAATAGCACTTTGGAATCTCT
GCCCGTACGTTTCTTACAGGCAAAAAAATTCCCGCATCAAGCGGGTTTGGATTGCTCTGG
TGAGCCACATCGGCTTTTCAACCGTCCACCTTACTTTTCCTTTTGAAAAGCAGGTTGGCA
TGGAATTCCCAACTCTTAATGCAGCACGCATCGTAGCAGAAAAGGCATATTGCCGCAATA
CTTCCCTTTTTCAGACGGCATGTTTCGTTTACAATTCAGGCTGTTTCCCCCTTTGCGAAC
CGCCATGCACATCCTGTTGACCGCCCTGCTCAAATGCCTCTCCCTGCTGCCGCTTTCCTG
TCTGCACACGCTGGGAAACCGGCTCGGACATCTGGCGTTTTACCTTTTAAAGGAAGACCG
CGCGCGCATCGTCGCCAATATGCGGCAGGCGGGTTTGAACCCCGACCCCAAAACGGTCAA
AGCCGTTTTTGCGGAAACGGCAAAAGGCGGTTTGGAACTTGCCCCCGCGTTTTTCAGAAA
ACCGGAAGACATAGAAACAATGTTCAAAGCGGTACACGGCTGGGAACATGTGCAGCAGGC
TTTGGACAAACACGAAGGGCTGCTATTCATCACGCCGCACATCGGCAGCTACGATTTGGG

## Appendix A

CGGACGCTACATCAGCCAGCAGCTTCCGTTCCCGCTGACCGCCATGTACAAACCGCCGAA
AATCAAAGCGATAGACAAAATCATGCAGGCGGGCAGGGTTCGCGGCAAAGGAAAAACCGC
GCCTACCAGCATACAAGGGGTCAAACAAATCATCAAAGCCCTGCGTTCGGGCGAAGCAAC
CATCGTCCTGCCCGACCACGTCCCCTCCCCTCAAGAAGGCGGGGAAGGCGTATGGGTGGA
TTTCTTCGGCAAACCTGCCTATACCATGACGCTGGCGGCAAAATTGGCACACGTCAAAGG
CGTGAAAACCCTGTTTTTCTGCTGCGAACGCCTGCCTGGCGGACAAGGTTTCGATTTGCA
CATCCGCCCCGTCCAAGGGGAATTGAACGGCGACAAAGCCCATGATGCCGCCGTGTTCAA
CCGCAATGCCGAATATTGGATACGCCGTTTTCCGACGCAGTATCTGTTTATGTACAACCG
CTACAAAATGCCGTAACGAAAATAAAAATGCCGTCTGAACAATTTCAGACGGCATTTTGT
CATCTGACGATTTCCGACAGCGGCCAGCGCGGACGGACATTGAACGCACCGACCTCCCTG
CCCTTGCCCAGGTGCATCGCACCGGCAAAGGCAATCATGGCACCGTTGTCCGTGCAGTAT
GCCGTCGGCGGGAAAAACACGCTGACTTTTTCGGACGGATGTTTCGGCTTGCCTTTGGGG
GTCGGGATTTGCACCGTCATGTTGCCGAAAGTTTCACGGAGCTTGCGGTTTGCACCGACC
CCGCCGGCGACCACTACGGTTCTGAACCCTGTCTGCAACAGGGCTTTTTTCACTTTCGCC
GCCAACACATCGACTACCGCATCTTGAAACGCACGGCAGATGTCGTTGCGTGTCTGCTCA
GGAATGTCATCCGCCCCGTTTTCCGCGCGCACTTTCTCGACGGCGGTCAATACGGCGGTT
TTCAAACCTGAAAAACTCATCTGCAAATCGTCGGAATGAATCATCGGGCGCGGAAAAACA
AACGCTTCGAACCTGCCCGATTCCGCAAGTTCCGACAGTTTCGCACCGCCCGGATACAGC
AAGCCCAGCAGTTTCGCCGTTTTGTCGAATGCCTCGCCCGCCGCATCATCGACGCTCTCG
CCCAAAAGCGCGTAGTCGCCTATGCCCCTGACCGCCATAATCTGCGTATGCCCGCCCGAA
ACCAACAGCGCGACAAAAGGAAAGTCGGGTTTTTCCTCCGCCAACAGCGGCGACAGCAGA
TGTCCTTCCAAATGATGGACGGGAATAACAGGCTTGTCCAACGCTAAAGCCAGCGCGTTG
GCGTAGCTCGAACCCGCCAGCAGCGCGCCGCCCAAACCGGGCCCCTGCGTAAAGGCAACC
GCGTCAATGTCGCCATACGATGCGCCTGCCTGCGCCAGACAGCCTTCCGTCAACGGAACA
AGGCGGCGGATATGGTCGCGGCTTGCCAATTCCGGCACAACCCCGCCGTATTCGGCGTGC
ATTGCCATTTGAGTGTGCAGGCAGTGCGCCCGCAATCCACGTTCCGTATCGTAAAGCGCA
ACACCTGTTTCGTCGCAAGAAGACTCGATTCCTAATACCAACATGGTCTGATGCCGTTAA
AAACTGAAAAACGTATTTTAGCGGATTTCGGCACGACTGCCGTATCCCAAAAACGGAACA
TGCCGTCTGAAGACCGTTCAGACGGCATCGTCGCACCGTATCAAAGCGTTCCGTAAGAAT
GCAGCCCGCTCAAAAACATATTCACGCCGATAAAGGCAAATGCGGTTACGAACAAACCGA
TAATCGCCCACCACGCCAGCACTTTGCCGCGCCAACCGGCAACCAGCCGCAAGTGCAGCC
AAACGGCGTAATTGAGCCAGACGATGAACGCCCACGTCTCTTTCGGATCCCAACTCCAAT
AGCGTCCCCAAGCATCTGCCGCCCACAGCGCACCCAAAATGGTGGCAATGGTAAAGAACA
GAAAGCCGACGGCAATCGCCTTATACATCACCTCGTCGATCAATGCCGACGGCGGCAGCC
ACAGTTTTCCGCCTTTTCCTTCCGCACGCAGGGAAACCAGTTCGGCAATACCGAGCATCG
CGGAAATGCAAAACGCGCCGTAACCGATAAAGTTTGCCGGAACGTGGATTTTCATCCACC
AGGACTGGAGCGCGGGAATCAGCGGCTGGATGGTATGCGCCTCGCGGACACGCTGTACC
ACAAGACAAATCCAACCACGACCGCCATAAAGCCGAACACGAAGCCGCCCAATTTCTGTA
TGGCGAACTTACCTTCATAATAAAGATACATCAGCGCGGTAATCACCAAAAACAGGATGA
ACACTTCATACAGGTTGGAAACCGGAATATGCCCCGCATCGGGACGGAGCAGATAGCTTT
CGTGCCAACGTACCAGCAGACCGGTAAAGCCTGCTACGGCAGACACCCATGCAAACACGG
TTCCCATACCCAACAGCGTGTTGGTCGGCACATTTTTTACGCTTGCCAAAACCGCGCCCG
AAATATAGGCGAACAGGGCGAAAAAGACAAAGGCGCACTGCCACATGATCGCCGACTGGC
TGCTGAGGAAATACCGCAACAGGAAAATCTCTGCCGATTTGATGTCGCCTCCGTACAAAC
CGACGGCGGCATAGGCAAGCAATACGCTTAAAGGAACAAACCAGCGCATCGGTTTGAAAA
ACCAACCCAAAAACACGGCAATACCGGCACTTGCCCACAACATGACCGTTTCGTAAATGT
CCATATGCATACCGGAACGGGTCTGTACGAAAACCGTAGCCGCAAAAACCAGCACGGCAA
ATACCCAATCCCAAAGATTCAGATTGCTGATCAAAGACTTCTGAATCAGCAGCTCGTGTT
CCGGAAGGGTTTTATAGTGTTCAGTCATGATTCAAGTCCTTGCCGAGCCGTTGCAGACTC
TCGACGTGTTTTGGAAATTCCTTCTGCAAATCCCGTTCGCTGCGGGCCGAAGACATGGCA
AAACGGATTTTGCCGTCTGAAAACAATACCCACGCCCGTTTTTCGCGCACATAAAACATC
AATACCGTACCCAATACCAACAGCACCGAGCCGAGATAGACCAAAAGCGCACCCGGGGAA
CGGGTCATCTGCAAACCCGACGAACGCACCTCGGAAAACCCATCAAGTTGCAGCAGCATA
GGCGCGGGATATTCGGTCAAACCCGTGTACGCATCCATACTGTGCAGCAGGAAACGATTC
CGCGCTTCATCCTGCTGCCATTCGGGCAAGCCGTACCGGCGTATGGTTTCATCCAAAGCA
GCGTTCATCACGCCGTAAAGCATTTCGTAGAAATAGCCCTGCATCTTATCCTGCTGCTCT
TTCGGGATATTGGACGTAATAAATTCGTCCAATCCCAAATAGCCTTTTTGTGCAAAGATG
TTCAGCGTGTTTTCCGCAGCCAGCATGAATTGTTCGCGGATTTCGGCAGGTGCGCCTTTG
GTTGCGTCGGCAACCAGACGTTTGCGCCCTTCCCCATCTTTCAAAAACTCACGCAATGCC
ATAAAGGTGTCCGCTTTCAACTGCTTGTCCAAGGGGATACGCAGCCAGCGGTATTGCTGC
TGCAAGCCGCTGCGCGTGCCGGTAATCCAAAAATAATCCTGTTCCTGCAAAACCGGCAGC
ATATAGTTTTTATATTCGACCGCCTGCCCTGCCGCATCACGGATACGGTAAACAATGGAA
GGGCCGATATTGGTGTATTTTTTACCTTCCTGAGTAACGGCGCGGACATCGTTCAGCGTG
GATTTCAGGCTTTTTTCCCGTTCCGCGCCCTCGCTCATGTCCTCCACATTCATAGAAGTG
AACTGATCGAACTCAAGACGATATTTGTGTTTGCCAATTTCCAACGGAAACTGGTGTATG
GATGTTGCCTTCAACACGACAGGCTCGCGCGAAGCATCACCCAAATTCCACGCCTTGAAT
GTCAAATCCGAACCGCCGTCGGCAAAACTCGCCTGATAAATCGTGATGCCGTGCAAGGTC
AAAGGATGGTTCACGCGGATGGTGCGCTCGAGTTTCTCACCGGTTGCCTTGTCCGTCACT
TCAATATCGCTGGCGAAATCACGCGGCATACCCGTATTGTAAAAATCGATATGGAATTTT
TTCAGTTTGACTTCAAAAGGCAAGTCCTGAACCAATATCCCGTTGTCGGCATTCAGGAAA
ACCACATCCGCACTCTGCCCCTCGGAAATATTGACGTTGCCCCTAAATGAGAGATTGGAC
GCACCCAAAATACTTTCGGGCTTGAAATCCTTGGCATAAACCGCCTGATTGTCCGGAACA
ATCCGACCGGTCAGCATACCCAGTTTCAACAGCAGGTTACTGTCTATCAACCCGCCCAGG
CAAATGACAATCAAAGCAACATGGGCAAAGATATAGCCCCATTTGTTCATTGTGCCTTTT
TTGGCGGCAATCAGAACCGACCCGTCTTCACGGTTAATGGTTTTTCCCTGAAAACCTTGT

## Appendix A

ACTTCCAGATAACGTTTGGCAACCTCGGGCGCAATTTTTACATCCAACAGCGAAGAATGG
CGCATCGCCGCCAGAGATTTTTCTTTAACCTTTTCCCGAAAAGACTTCATTTCGCGCCAG
AACGGCGGCACATTGCGAATCAGGCACAAACTGGTAGAAACCACCAAAAACATCATGATA
ACGACAAACCATGCCGAAGCATAGACGTCATACAGTCCCAGAAAACCAAAAATCTGCGCC
CAAAACGATCCGAATTTGACCAAATAATCCGTCTGCGGCTGGTTTTGCTGCAACACCGTA
CCGATAACCGATGCAATACCCAGCAGACTGAGCAAAGCGACTGCAAAGCGCATGGAGCTG
AAAAAAGCGAACCACGGACGGGAAAGAAGTGGGGGAGATCTACGGGATTTACTCATTGTG
TGTTTATTCCGCCATCAGGAATATGGGAAAGCAGAATTGGGCAAACAGAAAACAACGTCC
CGATTCTACTGTCTTGATGCTTTTTATTTCAAGACAATGAAGACAGCCTGCATCGATTCC
AACGGTTGCGATTGAAAAAACTTATCGCAGAATTGCCTGAAGCCGTCTGAAAACTTTTCA
GACGGCCTCTAAAACAGACTATTGCGGAATTAACGCAAACCTTGGATAAAGTTGGCGACC
GCTTTCAAATCTTCTTCAGACATACGGTTTGCAATATCTTCCATGATGGTATTTTTACGC
TGACCGGACTTGTAGGCATTCATCTGTTCAACAATATATGCCTGATGCTGACCGCCCAAA
CGCGGATAAGCCTGAATTTCGCTTCCGCCTCCCGGCATACCCGCACCGCTCGGACCGTGG
CAGGACATACACGCCGGCACTTTTTTATCGCTCAAACCGCCGCGATAGATTTTCGCACCC
AATTCGGGATTTTCCTTAGGATTGGCTTCACCGGATTTGGGCTGCTGTTTGGCATAGAAT
GCGGATACGTTCAAAATATCCTGATCGCTCAAATTCATTACCACCGGTTTCATCACAGCT
GCCGAACCGTGGGTGCGTTTACCGTCGCGGATGCCGATAGTTTGATGATAGATGTAAGCA
GTATGCTGTGCCGCCAAACGCGGATACATCGCAATGCCGCTGTTACCGTCTGCTGCATGG
CAAGCCGCACAAACCGTTGCGGCAACCTGTTTGCCTTTTTCCACGTCTGCTTTGGGAGAG
GCGGAAACCGCACCGGCAGCCAAAACAAAGGCCAATAAAGTCAATCGTTTCATGGAGTGC
TCCTGATTACAGCATTGGATAACGCAACAATGCTCTTTTTATATTCAAATACGGGATTTT
TGACCCGATTAAAACCGATGATTCTGTAAACGTGTTATTCTATACTAAATTTACATTAAA
TTACCACTGTGTTTCACATAAAACCAACCGCATATTTTTGCTGTCGGACAAACGGCGGCG
GAAAACAAGGATATGCCCATGAACCTTTTTCAAAACGCCAAATTCTTCACGACGATCAAC
CACCTTAAAGACCTGCCCGACACCCCTCTCGAAATTGCCTTTGTCGGCAGGAGCAATGCC
GGAAAATCCAGTGCCATCAATACCCTGACCAACCATGTCCGTCTTGCCTACGTTTCAAAA
ACACCCGGACGGACGCAGCATATCAACTTCTTCGAGCTGCAGAACGGCAATTTTATGGTC
GATTTGCCCGGCTACGGTTATGCCCAAGTCCCCGAAGCAGTACGCGCACATTGGGTCAAT
CTGCTCGGCGACTATCTGCAACAGCGCAAACAGCTTATCGGGCTGGTTTTGATTATGGAT
GCCCGCCATCCTTTAAAAGAACTCGACATCCGTATGCTGGATTTTTTCCACACGACCGGC
AGACCGGTTCACATCCTGCTGTCAAAAGCCGACAAATTATCCAAAAACGAACAGATAAAA
ACCCTGTCCCAAGTCAAAAAACTGCTCAAACCTTATTCCGACAGGCAAAACATCAGCGTA
CAGCTGTTTTCCAGCCTGAAAAAACAAGGTATTGACGAGGCCAACCGAACTGTCGGAAGC
TGGTTGGACGCAGCAGATGCCGCCGCTTCCTCTCCAGAGGAAAACTGACCCCAATTATAC
GGAAACCGTATTCCCCCCACTTGACCGACCGCAAACATTTAAAAAATTGCCACTGCCAAA
TCTAAAATGCCGTCTGAAAAGTCTTTCAGACGGCATTTTGCGGAGTCTTTAAAACAGAGA
ATCCAACTGCTGCTGTTTGGAACCAGTATTACTCGGAAGCACCGGCGTTTCCTGCATATC
TTGGCGGACTTCGTCATCCGCCGCCTGCCGTCCGCCTTCTGCCGCGCCCCCGTCATCTTC
TTTTGCCCGTCGGGAAGGTTGCGGCGCAATACCGCTGTTGTCCAGCGTCAAGCCCGGATC
GGTTACCATACGTTCCTTCATATAGTATTCGCCATTGCTGCTGACCACACCTTCAGGCAT
TTTCATCCCCTTGCCCTGCTTTCCTTTCAACGCAAAACGCATATAGTCCACCCAAACCGG
CACCGCAATCGTACCGCCGTAGCCGACACGCCCCATACTCTTAGGTTTGTCGAAGCCGAT
ATATACGGCAGTAACCACATCAGGGTTAAAACCGACAAACCACGCATCCTTATTGTCATT
GGTCGTACCCGTTTTACCGGCAATATCCGTTCTTCCCAACGCAGCTGCCCCCCTTGCCGT
ACCAACACGGACCACATCCTGCATAATCTTATACATAATATAGGCATTGCGCGGATCGAT
TGCCTGAGGCGCATTTTGCCCAGCCACCAAAGGTTGCATTTGGGCGCGCAACCTGCCGTC
TCTGTCATAAATCTTATCGATTACGTGCGAAGAAACCCTATATCCGCCGTTCGCAAATAC
GCTATATGCCTCCGCCACTTTCAACGGCGTTGTCTCGCCCGTACCTAAAGCCATAGACAG
GCTTGCCGGCAGCTCGGACGACCTGAAGCCGAAACGCCGGATATACTGTTGCGCGTAACC
GACACCGATAGACATCAAAATACGGATGGAAACCATATTCTTGGAAGCCGTCAGAGCCTG
TCTCAAAGTAATGTAGCCGGAATATCTGCCGTCTGAATTTTTAGGTGTCCAAACCGAACC
GTTCGGCCCTTTCCCCGGCAGGGAAATCGGCGCATCGTTAACCACTGTGGACGCGGTCAT
CCCCTTAGATAATGCCGCCGAATAGACAAACGGCTTAAAGGTCGAACCCGGCTGCCGCAT
TGCCTGAACGGCACGATTGAATGTTTTGCTGTGAAAATCATAACCGCCGACCAGCGCGCG
CACAGCTCCGGTTTTTGCATCCAGCGAAACCAAAGCCCCCTGCAGCAACGGCTCTTGAAC
CACCGCCCAACGCCCGCCGTTGTTTTTGACACGGATGACCGCGCCCCTGCGGATACGGTC
CTCCCCCATTTTTTCATTATTGACCGCGCGGGCCGCAAAACCCAAGGCGCGCCTGTCAAG
CGTAACCCGCCTGCCGCCGGGCAGCTGTATGACGACATTTTTCTTTTTAGTCACATCCAA
CACAACGGCGGGAACCATTTTATCGACGGTATAGAGTCCCGACAGATACTGGCTGACAGT
CTCCTCGACATCTTCACTCTTACTCAAATCGATATAGTTTTCCGCACCGCGGTAGCTGCT
GCCGCGATCGAAATTCCGTAGAGCCTTGCGCAATGCCTCGGTTGCCACCTTCTGATGATC
GGCGCGGACCGTGGTATAAACCTTAAAACCCTGCGTATAGGCATCTTCACCGTATTTCTC
ATACAGTTCCTGACGCACCATTTCCGCCACATATAACGCACTCTGATCGATTTTCCGAAC
AAACCGCTCGTAATGCAGTTCCTCATTCAACGCCTGATCGCGCTGTTGCACGGTAATCAT
CTTCTCCTCGAGCATATTGTTCAAAATATACTTCTGGCGCAACTTGGCACGTTCTGGATT
AACAATCGGATTATAGGCAGACGGAGCCTTGGGCAGTCCCGCAAGCATGGCGGCTTCCGC
CAAAGTCAAATCTCGGACATTCTTATTGAAATAGATTTGCGCGGCAGATGCAAAACCATA
GGCGCGCTGACCGAGGTAAATCTGATTGAAATACAACTCGAGGATTTTGTCTTTGCTTAA
AGACTGCTCGATTTTATAGGCAAGCAACACCTCATTGAATTTGCGTGTGAACGTTTTTTC
ACTGCTCAAATAAAAATTTTTCGCCACCTGCTGCGTAATCGTACTCGCACCCGACTGCAC
GCTGCCGGACACGACATTGCCGACGGCAGCGCGGGCAACACCCCAAACATCCACCCCCCA
ATGCCGGTAAAAGCGTTTATCCTCGGCGGCGATAACCGCATTCCGCAACACCTCTGGGAA
ATCGCCGATTTTTGTAAATTCGCGCCGCTGCTCCCCATACATACCGATGACTTCCCCATC
CGCCGAATAAATAGTCAACGGCATTTTAGGCTGGTAATGCTGCAAAGAATCCAAAGACGG

## Appendix A

CAGTTTCGGATACGTTACCAAAATAGCAATGGCAACCAAACCCACTCCAAATACACAAAA
CCCAAAAACCAAACCAAAACAAGTCGTTAAAATCTTTTTAATCATAGCTGAATAATAATT
TACCATTATTGGTATTAAATAAAGTAAAATAGCAACCGATTTCTACAAAGCACGGTTTCA
ATGTGCAAAGAACAAGGAATCCATTACGGATACCGAAACGGTTACTCACTGTACAAATAA
AGCAGGAACTTTCATCATGCGCTTGTTTAAAAGCTTGAAAAACCCTAAAAAAACAGATGC
CAAGCTCCCTAAAAAATCTTCGGGACTCAATAACCGCGCGGCAATCGGCATCGATATCGA
CCAGCATTCCATCAAAATGGTCCAATTGTCAGGACGTAGTTTAAACCAAATTCAATTGGA
AAAATACGTCATTGCCAAATTACCAAAGAATATCATTCAAGGCAATAAAGTCCAAAATTA
CGATCAACTTGTTACATATTTGCAACAAGCCTATGCCAAACTGGGTACTTCGTGCAAAAA
CATCATCGCGTCCGTCCCGCAAAATTTGGCAACCATCGAACAATTGACCTACACAGACAA
AGATGCAGAATTAGACCTGCAGGGGTTCGTGGAGTCCTCCATCTCCGAAGTCAGCTCGAT
ATCGCTCGAAGAAGCCAATTACGACTATCAGGTCTTGTCCCAATCGGCCGCCGGCGAAGC
TGTGTTGGCCGTCGCATCGAGAAAGGATGAAATCGAACCCCTGATTGACGCATTCAACGC
CGCCGGTATGAAATTATCCGCGCTTGATGTGGACATTTTCGGACAATACAACGCCTACGC
GCTATGGATAAACCATTTCGCCCCCGAGCTTGCAGCCGAAAAAGTCGCCATTTTCGGCGT
ATATGCCGCACAGACCTACGCCTTGGTCATCCAAGACGGAAAAATCCTATACAAACAGGA
AACCTCCGTCAGCGAAGAACAGCTCAACCAACTCATCCAGCGCACCTATCAGGTAACAGA
AGAAAAAGCGGAAGAAATCATCAACTCCCCGCAAAAACCTTCCGATTACCAAGAAAGCGT
GGCAAACTATTTCAACCAGCAGATTACCCAAGAAATACAAAGGGTCTTGCAGTTTTATTA
CACCACGCAGACCGCAGACGATATGACCGACATCAAGCATATCCTGCTGACCGGGGAAGC
GGCGCGCCAGGAAGGCATCGCCCAAACCGTCGCCTCACAAACCAATGCAGATGTACAATG
CGTCCATCCCGCGCGTTATTTTGCGGACAACCTCAAAACAGACAAACAACAATTCGAACT
TGATGCGCCGACACTGACCAGGGCGTTCGGTTTGGCGGTACGGGGATTATAATTATGAAC
AATTTAATCAAAATCAACCTCCTCCCCTACAGGGAAGAGATGAACAAGCGCAAACAGCAG
CAGTTTAAAACGCTGATGTACGGTGCCGTGCTGACGGGCGTTGCCGCCGTTGCGGCAACC
TACCTGTTTATCGACAATATGATCAATAACCAGTCGGAAAGAAACACGCTGCTGGAAACC
TCCATCGCACACTTGGATACCGAGCTGTCGGAAATACAAAAGCTCAAACAGGAAAAAGAT
GCCTTCCTGATTAAGAAAAACAAAATCGAGGAGCTCCAGCTCAAACGCCTCCAAGCCGCA
AAAATCCTCGACAGCCTGAATGAGGCCGTCCCCGGAAGCACCTACCTGACCTCGCTGGAT
GCCGTTACCGCCGACTCTTATCGGCTCAGCGGCAGGACATCCAGCGACAACCGCGTTGCC
GCCATGATGAGGGCGATGCCCAATACCGGCATATTCAAGCAACCCGAATTGTTAAGCATC
AAGAAAAACAATTCGCATCAAGAATTTACCCTTCAGGCAACATTACAACCCATCGTAAAG
GCGGCCGAATCCAAAGAGAATCCGGCTTCGGGAAACGCACAGGAGGCAAACTGAATGGCT
TCTAAATCATCTAAAACCAACTTGGATCTCAACAACCTTCACCTGCTCAACCTTCCTGCC
AGGCTTTTTATCGCCCTGCTGGCCGTTGCCGCCGTGCTGGGGCTCGGTTATGCCGGATTG
TTCAAAAGCCAGATGGAATCCCTTGAGGAATACGAAGCAAAAGAAACCGAACTGAAAAAC
ACCTACAAACAGAAAAGTATCGACGCGGCCAGCCTGAACAACCTGAGGGACGAACTTGCC
TCAATCCGCTCTGCCTTCGATATCATGTTGAAACAGCTGCCGACAGATGCAGAAATTCCC
AATCTGGTTCAAGAGCTTCATCAGGCAGGTTCGAGCAACGGTCTGCGCTTGGACAGCGTT
ATGCCCCAACCTCCCGTAGATGACGGCCCCATCAAAAGATTACCCTATTCCATTTCCATT
ACCGGAAATTACGAACAGATCAGCCAATTTACCCGCGATGTCGGCAGCCTCTCCCGAATC
ATTACCCTTGAGTCGCTGAAAATCGCCCAATCTCCGGAAAACGGCGGCAATCCTGACGGC
AAGAGCAGCATCCTGAACCTCAGCGCCATTGCCACCACCTACCAAGCAAAATCCGTAGAA
GAGCTTGCCGCAGAAGCGGCACAAAATGCCGAGCAAAAATAACTTACGTTAGGGAAACCA
TGAAACACTATGCCTTACTCATCAGCTTTCTGGCTCTCTCCGCGTGTTCCCAAGGTTCTG
AGGACCTAAACGAATGGATGGCACAAACGCGACGCGAAGCCAAAGCAGAAATCATACCTT
TCCAAGCACCTACCCTGCCGGTTGCGCCGGTATACAGCCCGCCGCAGCTTACAGGGCCGA
ACGCATTCGACTTCCGCCGCATGGAAACCGACAAAAAAGGGGAAAATGCCCCCGACACCA
AGCGTATTAAAGAAACGCTGGAAAAATTCAGTTTGGAAAATATGCGTTATGTCGGCATTT
TGAAGTCCGGACAGAAAGTCTCCGGCTTCATCGAGGCTGAAGGTTATGTCTACACTGTCG
GTGTCGGCAACTATTTGGGACAAAACTACGGTAGAATCGAAAGCATTACCGACGACAGCA
TCGTCCTGAACGAGCTAATAGAAGACAGCACGGGCAACTGGGTTTCCCGTAAAGCAGAAC
TGCTGTTGAATTCTTCCGACAAAAACACCGAACAAGCGGCAGCACCTGCCGCAGAACAAA
ATTAAGAAGAGGATTACTCCATTATGAATACCAAACTGACAAAAATCATTTCCGGTCTCT
TTGTCGCAACCGCCGCCTTTCAGACAGCATCGGCAGGAAACATTACAGACATCAAAGTTT
CCTCCCTGCCCAACAAACAGAAAATCGTCAAAGTCAGCTTTGACAAAGAGATTGTCAACC
CGACCGGCTTCGTAACCTCCTCACCGGCCCGCATCGCCTTGGACTTTGAACAAACCGGCA
TTTCCATGGATCAACAGGTACTCGAATATGCCGATCCTCTGTTGAGCAAAATCAGTGCCG
CACAAAACAGCAGCCGTGCGCGTCTGGTTCTGAATCTGAACAAACCGGGCCAATACAATA
CCGAAGTACGCGGGAACAAAGTTTGGATATTCATTAACGAATCGGACGATACCGTGTCCG
CCCCCGCACGCCCCGCCGTAAAAGCCGCGCCTGCCGCACCGGCAAAACAACAGGCTGCCG
CACCGTCTACCAAGTCCGCAGTATCCGTATCCGAACCCTTTACCCCGGCAAAACAACAGG
CTGCCGCACCGTTTACCGAGTCCGTAGTATCCGTATCCGCACCGTTCAGCCCGGCAAAAC
AACAGGCGGCGGCATCAGCAAAACAACAGGCGGCAGCACCAGCAAAACAACAGGCGGCAG
CACCAGCAAAACAACAGGCGGCAGCACCAGCAAAACAAACCAATATCGATTTCCGCAAAG
ACGGCAAAAATGCCGGCATTATCGAATTGGCTGCATTGGGCTTTGCCGGGCAGCCCGACA
TCAGCCAACAGCACGACCACATCATCGTTACGCTGAAAAACCATACCCTGCCGACCACGC
TCCAACGCAGTTTGGATGTGGCAGACTTTAAAACACCGGTTCAAAAGGTTACGCTGAAAC
GCCTCAATAACGACACCCAGCTGATTATCACAACAGCCGGCAACTGGGAACTCGTCAACA
AATCCGCCGCGCCCGGATACTTTACCTTCCAAGTCCTGCCGAAAAAACAAAACCTCGAGT
CAGGCGGCGTGAACAATGCGCCCAAAACCTTCACAGGCCGGAAATCTCCCTTGACTTCCA
AGATGTCGAAATCCGCACCATCCTGCAATTTTGGCAAAAGAATCCGGAATGAACATTGTT
GCCAGCGACTCCGTCAACGGCAAAATGACCCTCTCCCTCAAGGATGTGCCTTGGGATCAG
GCTTTGGATTTGGTTATGCAGGCGCGCAACCTCGATATGCGCCAGCAAGGGAATATCGTC
AACATCGCGCCCCGCGACGAGCTGCTTGCCAAAGACAAAGCCCTCTTACAGGCAGAAAAA

# Appendix A

GACATTGCCGATTTGGGTGCGCTGTATTCCCAAAACTTCCAGTTGAAATACAAAAATGTG
GAAGAATTCCGCAGCATCCTGCGTTTGGACAATGCCGACACGACCGGAAACCGCAACACG
CTTATCAGCGGCAGGGGCAGCGTGCTGATCGATCCCGCCACCAACACCCTGATTGTTACC
GACACCCGCAGCGTCATCGAAAAATTCCGCAAACTGATTGACGAATTGGACGTACCCGCG
CAACAAGTGATGATTGAGGCGCGTATCGTCGAAGCGGCAGACGGCTTCTCGCGCGATTTG
GGCGTTAAATTCGGCGCGACAGGCAAGAAAAAGCTGAAAAATGATACAAGCGCATTCGGC
TGGGGGGTAAACTCCGGCTTCGGCGGCGACGATAAATGGGGGGCCGAAACCAAAATCAAC
CTGCCGATTACCGCTGCCGCAAACAGCATTTCGCTGGTGCGCGCGATTTCCTCCGGTGCC
TTGAATTTGGAATTGTCCGCATCCGAATCGCTTTCAAAAACCAAAACGCTTGCCAATCCG
CGCGTGCTGACCCAAAACCGCAAAGAGGCCAAAATCGAATCCGGTTACGAAATTCCTTTC
ACCGTAACCTCAATCGCGAACGGCGGCAGCAGCACGAACACGGAACTCAAAAAAGCCGTC
TTGGGGCTGACCGTTACGCCGAACATCACGCCCGACGGCCAAATCATTATGACCGTCAAA
ATCAACAAGGACTCGCCTGCGCAATGTGCCTCCGGTAATCAGACGATCCTGTGTATTTCG
ACCAAAAACCTGAATACGCAGGCTATGGTTGAAAACGGCGGCACATTGATTGTCGGCGGT
ATTTATGAAGAAGACAACGGCAATACGCTGACCAAAGTCCCCCTGTTGGGCGACATCCCC
GTTATCGGCAACCTCTTTAAAACACGCGGAAAAAAACCGACCGCCGCGAACTGCTGATT
TTCATTACCCCGAGGATTATGGGTACGGCCGGCAACAGCCTGCGCTATTGATGCGTCAAA
ATAAGGGCATATGTTTTACGGCATATGCCCTTTCTTTATGCTTTTTGCCGCGACCGAAAT
GCCGTCATTCCCGCGCAGGCGGGAATCCAGTCCGTTCAGTTTCGGTCAGTTTCGGTCATT
TCCGATAAATTCCTGTTGCTTTTCATTTCTGGATTCCCACTTTTGTGGGAATGACGGCGG
AAGGGGTAAATCCTCACAACCCAAAGCCTCGTCATTTCCACAAAAAACAGCAACCCGAAA
CAGCAACTTAAAACCCCGTCATTCCCGCGCAGGCGGGAATCTAGGTCTGTCGGTTCAGGA
ACTTATCGGATAAAACGGTTTCTCCAACCCTGCGTTCTAGATTCCCACTTTCGTGGGAAT
GACGGGATATGGGTTTCCGTGCGGACGTGTTCGGATTTCCGCCTGCGCGGGAATGACGGC
GACAGATGCCCAACGGTCTTTATAGTGGATTAACAAAAATCAGGACAAGGCGACGAAGCC
GCAGACAGTACAAATAGTACGGAACCGATTCACTTGGTGTTTCAGCACCTTAGAGAATCG
TTCTCTTTGAGCCAAGGCGAGGCAACGCCGTACTGGTTTTTGTTAATCCACTATAGTATT
GATAAACATATTATCTTCAATATATTCAATTGGATAATTGTTTACCTAAGCAAAGATAAT
TGCCTTTTCCTGACAAATAAGTGAAATCAACGGATTGTCAAAACACAGCCTGAAATAAAA
AACCTCCCTGATTTCTTTTATTTGTCCTTAAAATCAGAAAGGTTCGGGATGGTCGGGTTA
TTTTTCCAAACGTACCGCCGCCCTGCCGATTTCGTATAAAATTCCGCCGTAACCCGACAA
GCCCGAACCCTGTCGCCCCGAAAGGCGGGGTGTCAAACATTAAGGAATTGTGATGAAAAA
CTTTAACGGCAAACTCATCCTCATCGGACTGATGGGCGCGGGCAAAACCACGCTGGGCCG
GCAAATGGCGCAGCGGCTGGATTACCGTTTTTACGACAGCGATCACGAAATCGCCGCAGC
CGCCGGCGTTCCCATCCCCACCATATTTGAAATGGAAGGCGAACAGGGATTCCGTTCGCG
CGAAACCGCCATACTCAAAAAGCTGGTTATCCTGCCCCATATCGTCCTGTCCACCGGCGG
CGGCGCGGTGTTAAAAGAAGAAAACCGCGCCCTTATCCGCAAAAGCGGCACGGTCGTCTA
TCTGCACGCCCCGCCCGAAACCCTGCTCGAACGCACGCGCTGCGACAACAGCCGTCCTTT
GCTGCAAGTTGCCGATCCTTTGGCGAAATTACGTGAACTCTACGCCGCACGCGACCCCGT
TTACCGCCAAACCGCCGACTTTACCGTAGAATCGGCAAACTGCCGGGAAACCGTGCAAAC
CCTGCTCAAACGCTTATCCCGATAAACCGGCATATGCGCCGCGCCCAGAAAACCAAACCG
CGCCCCGCCCGGCGGGCCGGCGGTTCAAACTTTAAGGAACAACAATGAAAACACTGACCG
TACACACCCCGTCCCACAGCTACCCCATCTTTATCGGCAACGGACTGCTGCCGCAGGCAG
GAAGCCTGCTCAAACCGCATTTGGGCAAACGCGCCGCCATCATCGCCAACGAAACCGTCG
CCCCGCTCTACCTCGGCACGCTTCAGACGGCATTGGATGCGGCAGGCGTATCCCATTTCA
GCATCATCCTGCCCGACGGCGAGGCGCACAAAAACTGGCAGACGCTCAACCTCATCTTTG
ACGGGCTGATGCAAAACCGCGCCGAACGCAAAACCACATTAATCGCACTGGGCGGCGGCG
TGATCGGCGACATGGTCGGCTTTGCCGCTGCCACCTACCAGCGCGGCGCACCGTTCGTTC
AAATACCGACCACGCTGTTGAGTCAGGTCGACTCATCGGTGGGCGGCAAAACCGCCATCA
ACCACCCGCTCGGCAAAAATATGATTGGCGCGTTTTACCAGCCGCAGGCGGTGCTTGCCG
ACTTGGACACGCTGCACACCCTGCCCGCCCGCGAATTGTCCGCCGGTATGGCGGAAGTCA
TCAAATACGGCGCGCTCGGCGACATCGGCTTTTTTGAATGGCTGGAACAGCATATGCCCG
AACTGATGACGCTCGATCGGGAAAAACTCGCCCAAGCCGTGTACCGCTGCTGCCAAATGA
AGGCAGACATCGTCGCCCAAGACGAAACCGAACAGGGCATACGCGCATGGCTCAACCTCG
GACACACCTTCGGACACGCCATTGAAACCGAGATGGGTTACGGCACTTGGCTGCATGGAG
AAGCCATCGCCGCCGGCTGCGTGTTGGCGGCGCGTTTGTCCGAACAACTGGGCAAAACCT
CCGCCGCAGATACCGCGCGGCTCGCCGCCCTGCTCGAAGCCGCCGGACTGCCGTCCGCGC
CACCCGTGTTTGCCTTTGAAAAATGGCTGGAACACATGAGCCACGATAAAAAAGTCAGCG
GCGGCATCATGCGCTTTATCGGTCTGAACCGGCTGGGCGAAGCCAACATCACCGAAATTA
CCGACACGGACATCCTCCGCCGCACCCTGCAACCGTATCTCTGATTTCCTCTGCCGATGT
GCTGCCGCGCGGGTTTGACGCACGATGATGATTTTCCATCATCTTTCTCCGCAAAAGCGG
GAATCCAGTCCGTTCGGTTTCGGTCGTTTCCGATAAGTTCCCGTTGCTTTTCATTTCTAG
ATTCCCACTTTCGTGGGAATGACGGCGGAGAGGTTTTTGTTGTTTCGGAGAAGTTTCTGC
AACCCTAGAATCTCGTTATTTCCACAAAAAACAGAAAACCAAAACAGCAACTTAAAACCT
CGTCATTCCCGCAAAAGCGGGAATCCGGTCCGTTCGGTTTCGGTCGTTTCCGATAAATTC
CTGCTGCTTTTCATTTCTAGATTCCCACTTTTGTGGGAATGACGGCGGAAGGGTTTTGGT
TTTTTCCGATAAATTCTTGAGGCATTGAAATTCTATAGTGGATTCACAAAAATCAGGACA
AGGCGACGAAGCCGCAGACAGTACAGATGGTACGGAACCGATTCACTCGTGCTTCAGCAC
CTTAGAGAATCGTTCTCTTTGAGCTAAGGCGAGACAACGCCGTACCGGTTTTTGTTCATC
CACTATAACAGCAACCCTGTCGCCGTCATTCCCGCAAAAGCGGGAATCCAGTCCGTTCGG
TTTCGGTCGTTTCCGATAAGTTCCCGTTGCTTTTCATTTCTAGATTCCCACTTTCGTGGG
AATGACGGCGGAAGGGTTTTGGTTTTTTCCGATAAATTCTTGAGGCATTGAAATTCCAGA
TTCCCGCCTGCGCGGGAATGACGGCTCAAAAGTTACGGAACGAAAAACAACCAAAACCGG
ACAAGTCGGATTCCCGCCTGCGCGGGAATGACGGAATCTTAAGTTTCCGTCTTTGTTTTC
TGTTTTCTGTTTTCGAGGGAATAATGGGGAACAAGCCGTATTTCAGACGGCATTTTCAGT

## Appendix A

TCGGGGTATAATCCGAATACTTGCGACCATCTGAATCATTGGGACAAACCATGTGTCAAC
TGCTGGGCATGAACTGCAATACGCCGACCGATATTATGTTTTCCTTTGAAGGCTTCCGCC
GCAGGGGCGGCATTACCGACCACCATGCCGACGGTTTCGGTATCGGCTTTTTCGAAGGCA
AAGGCGTGCGCCTGTTCCACGACGACAAGCCGAGCGTAAATTCCCCCGTCGCCGACCTCG
TGCGTGCCTACCAAATCAAATCGGAAAACGTCATCGCACATATCCGCAAAGCATCGCAAG
GACAAACCTCGCTGGCGAACACCCATCCCTTTATGCGTGAAATGTGGGGCGGCTACTGGC
TGTTTGCCCACAACGGACATTTGATTGATTTTTTCCCCGAACAGGGCGAATTTTTCCACC
CCGTCGGCACAACCGATTCCGAACGCGCGTTCTGCCACATCCTCAACCGCCTGCGCACCC
GCTTTGCCGCCCGTCCCGACGACGACACGCTGTTTGACGCGATTGCGGGGCTGACGCACG
AAATCCGCAAGTTCGGGCTGTTTAACTTTATGCTTTCAGACGGCATTGCCCTGTTTGCCC
ACGCCAGCACGCTGCTGCACTACATCGTCCGCCAAGCCCCGTTCGGCAAGGCGCGCCTGC
TCGACGACGACGTGATGGTCGATTTTGCCGAAGTAACCACGCCCTCCGACCGCGTCGCCG
TTATCGCCACCCTGCCACTGACCCGCGACGAATCATGGTCCCAACTTGCCGTGGACGAAC
TGGTCATGTTCCGCGAAGGCAACATCGTCCGACACGACCGTCCCGAAAACCCCGTCTATA
TGAGTGCCGAAGAAGGTCTGGAAATCGCCCGCGCCGCCGGCGTCGCCGTCTGAACTTCAG
ACGACATAGGAGGACGAACCCGATGAAATGCCCGTTTTGCGCCCACCCCGACACCCGCGT
TGCCGATTCGCGTCTGATGGAAGAACGCAACGCCGTGCGCCGCCGCCGCCACTGCCCCAA
CTGCGGCAAACGCTTCGGCACGCTCGAAACCGCCGAACTCAAAATGCCCGCCGTCATCGG
TCCGGACAAAAAACGCTCGCCCTTTAATGCACAACGCCTCCGCAACGACCTGACCGCCGC
CGCCCGAAAATCCGCCCTGACACCCGAACAGATCGACGAAACCGTCCGCCTGACGGAACA
CAGGCTCTACACTTCGGGTCAGCGCGACATCCCCTCTGCCGCACTTGCCGACATGGTGCT
AAAAGAACTGCTCCGACAAGATACGGAAGCCGCCGTCCGTTTCGCCGCCCTGCACAAACG
CTTCGACAATCCGGCAGACTTTGCCTCGTGGCTGGCGCAAGCCGTCAAAACAGGCGGCAA
AGCCTGATTCCCCCAACCCATACTGATACGGTATCCCTATGTTTTCGGACACAGATATAT
CCATGATGGAAAACGCCCTCCGACTTGCCGCTTTGGGGCGTTTTTCCACTTCGCCCAATC
CGCGCGTCGGCTGCGTTATCGCACACGGCAGCCAAATTGTCGGGCAAGGCTTCCACGTCA
AAGCGGGCGAACCCCATGCCGAAGTCCACGCCCTGCGTCAGGCGGGCGAAATGGCACAAG
GCGCGACCGCCTTTGTTACCCTCGAACCGTGCAGCCATTACGGGCGCACACCGCCCTGTG
CCGAAGCACTGGTGCGGGCGGGCGTGTCCCGCGTCGTTGCCGCCATGCGCGACCCCAACC
CGCTGGTTGCAGGCAAAGGGCTTGCCCTGCTCGAAGCAGCAGGCATCAAGACGGAATGCG
GTTTACTCGAACATCAGGCAAGGGAACTCAACCGAGGCTTCCTGTCGCGCATCGAACGCC
GCCGCCCCTTTGTCCGCCTCAAATGCGCCGTTTCGCTGGACGGCAAAACCGCCCTTTCAG
ACGGCAGCAGCTTTTGGATTACCGGCGAAGACGCGCGTGCCGACGTACAGGTTTTGCGTG
CCGAAAGCTGCGCGGTGCTGACCGGCATCGGCACGGTGTTGGCGGACAATCCCCGGCTCA
ACGTCCGCGCTTTTCCAACTTTGCGCCAACCCGCACGCATCGTTTTAGACAGCCGCCTGC
GCCTGCCCCCGAACAGCCATTTGGTTACCGACGGACAATCTCCGACCTACATCGCCACAC
TCGAACGCAACGAAGACAGACTGCACCCCTATCGGGAACACGCACACGTCCGCATCCTGA
TGCCGTCTGAAACGGCAGACAGCAAAATCGACCTGCACCACCTGATGCGCCTCCTTGCTG
ACGAAGGTTTCGGCGAAATCATGGTCGAAGCAGGCTCCGAACTCACATCCGCATTTTTGG
CAGAAAATCTGGCAGACGAAATCGTCCTGTACCGTTCGCCCAAAATCCTCGGCAGCGGCA
AAGACCTGTTTTCCCTGCTCGAAAACCGCGCCGCCCTTTCCGCACCGCCCTTGTGGACAC
CCGTTTCAAGCGAAATCCTCGGACACAACATCAAAACCGTGTTCCGAAAAAACGGCAACG
CCTTTTAAAGGGTTTGCGCCGTTTCACTATATAATAACGCCGATAAAAAACGGCCCCGTT
CAGACCGCCGCCCGCCCGAAAAAACGCAACCCGGACTGCCGCACCCCGCCGGCAGCCGCG
ACGGTCTGAAAGCCGTCAAATTCCGATCAAGAAAGGCTTCAGACGGCACAGGCAGCATCC
CGCCGCCGCCCGGACATCAAAAATGGACACAAAAGAAATCCTCGGCTACGCGGCAGGCTC
GATCGGCAGCGCGGTTTTAGCCGTCATCATCCTGCCGCTGCTGTCGTGGTATTTCCCCGC
CGACGACATCGGGCGCATCGTGCTGATGCAGACGGCGGCGGGGCTGACGGTGTCGGTGTT
GTGCCTCGGGCTGGATCAGGCATACGTCCGCGAATACTATGCCACCGCCGACAAAGACAC
~~CTTGTTCAAAACCCTGTTCCTGCCGCCGCTGCTGTCTGCCGCCGCGATAGCCGCCCTGCT~~
GCTTTCCCGCCCGTCCCTGCCGTCTGAAATCCTGTTTTCACTCGACGATGCCGCCGCCGG
CATCGGGCTGGTGCTGTTTGAACTGAGCTTCCTGCCCATCCGCTTTCTCTTACTGGTTTT
GCGTATGGAAGGACGCGCCCTTGCCTTTTCGTCCGCGCAACTCGTGCCCAAGCTCGCCAT
CCTGCTGCTGCTGCCGCTGACGGTCGGGCTGCTGCACTTTCCAGCGAACACCGCCGTCCT
GACCGCCGTTTACGCGCTGGCAAACCTTGCCGCCGCCGCCTTTTTGCTGTTTCAAAACCG
ATGCCGTCTGAAGGCCGTCCGGCACGCACCGTTTTCGCCCGCCGTCCTGCACCGGGGGCT
GCGCTACGGCATACCGATCGCACTGAGCAGCATCGCCTATTGGGGGCTGGCATCCGCCGA
CCGTTTGTTCCTGAAAAAATATGCCGGCCTGGAACAGCTCGGCGTTTATTCGATGGGTAT
TTCGTTCGGCGGGGCGGCATTATTGTTCCAAAGCATCTTTTCAACGGTCTGGACACCGTA
TATTTTCCGCGCAATCGAAGAAAACGCCCCGCCCGCCCGCCTCTCGGCAACGGCAGAATC
CGCCGCCGCCCTGCTTGCCTCCGCCCTCTGCCTGACCGGCATTTTCTCGCCCCTTGCCTC
CCTCCTGCTGCCGGAAAACTACGCCGCCGTCCGGTTTATCGTCGTATCGTGTATGCTGCC
GCCGCTGTTTTGCACGCTGGCGGAAATCAGCGGCATCGGTTTGAACGTCGTCCGCAAAAC
GCGCCCGATCGCGCTCGCCACCTTGGGCGCGCTGGCGGCAAACCTGCTGCTGCTGGGGCT
TGCCGTGCCGTCCGGCGGCGCGCGCGGCGCGGCGGTTGCCTGTGCCGCCTCATTCTGGCT
GTTTTTTGCCTTCAAGACCGAAAGCTCCTGCCGCCTGTGGCAGCCGCTCAAACGCCTGCC
GCTTTATCTGCACACATTGTTCTGCCTGACCTCCTCGGCGGCCTACACCTGCTTCGGCAC
GCCGGCAAACTATCCCCTGTTTGCCGGCGTATGGGCGGCATATCTGGCAGGCTGCATCCT
GCGCCACCGGAAAGATTTGCACAAACTGTTTCATTATTTGAAAAAACAAGGTTTCCCATT
ATGAAAATCGTTTTGACCACATCTATGGCAGGCTTGGGCGGCACGGGCACGATATCATCG
ATTGCCAAATGGCGTGCAAAAACGACCCCGTTCAGTCCGACGAAATCGTCCGCCGCTTCA
GGCGCGACATTTCCTATCGGAAAATCGTCAACCTGATTGAAAGATTGGCAAATGAGTAAA
TTCTTCAAACGCCTGTTTGACATTGTTGCCTCCGCCTCGGGACTGATTTTCCTCTCGCCA
GTATTTTTGATTTTGATATACCTCATCCGCAAGAATCTAGGTTCGCCCGTCTTCTTCTTT
CAGGAACGCCCCGGAAAGGACGGAAAACCTTTTAAAATGGTCAAATTCCGTTCCATGCGC

## Appendix A

GACGCGCTTGATTCAGACGGCATTCCGCTGCCCGACGGAGAACGCCTGACACCGTTCGGC
AAAAAACTGCGTGCCGCCAGTTTGGACGAACTGCCTGAATTATGGAATATCTTAAAAGGC
GAGATGAGCCTGGTCGGCCCCCGCCCGCTGCTGATGCAATATCTGCCGCTGTACGACAAC
TTCCAAAACCGCCGCCACGAAATGAAACCCGGCATTACCGGCTGGGCGCAGGTCAACGGG
CGCAACGCGCTTTCGTGGGACGAAAAATTCGCCTGCGATGTTTGGTATATCGACCACTTC
AGCCTGTGCCTCGACATCAAAATCCTACTGCTGACGGTTAAAAAAGTATTAATCAAGGAA
GGGATTTCCGCACAGGGCGAAGCCACCATGCCCCCTTTCACAGGAAAACGCAAACTCGCC
GTCGTCGGTGCGGGCGGACACGGAAAAGTCGTTGCCGACCTTGCCGCCGCACTCGGCCGG
TACAGGGAAATCGTTTTTCTGGACGACCGCGCACAAGGCAGCGTCAACGGCTTTTCCGTC
ATCGGCACGACGCTGCTGCTTGAAAACAGTTTATCGCCCGAACAATACGACGTCGCCGTC
GCCGTCGGCAACAACCGCATCCGCCGCCAAATCGCCGAAAAAGCCGCCGCGCTCGGCTTC
GCCCTGCCCGTTCTGGTTCATCCGGACGCGACCGTCTCGCCTTCTGCAACAGTCGGACAA
GGCAGCGTCGTTATGGCGAAAGCCGTCGTACAGGCAGGCAGCGTATTGAAAGACGGCGTG
ATTGTGAACACTGCCGCCACCGTCGATCACGACTGCCTGCTTAACGCTTTCGTCCACATC
AGCCCAGGCGCGCACCTGTCGGGCAACACGCATATCGGCGAAGAAAGCTGGATAGGCACG
GGCGCGTGCAGCCGCCAGCAGATCCGTATCGGCAGCCGCGCAACCATTGGAGCGGGCGCA
GTCGTCGTACGCGACGTTTCAGACGGCATGACCGTCGCGGGCAATCCGGCAAAGCCGCTG
CCGCGCAAAAACCCCGAGACCTCGACAGCATAAGCGATTAAAATACACCCCCGTACAGAC
CGATTTTGACAACACCTGCGGCGCGCGCCCGATTCTTCGGAACACGCCCCCCTTCAGACG
GCATAGGGTCGGAAATGCCGTCTGAAAACCGACGGACAAACCATCATGCTGAACACTTTC
CTTTCCCCGTGGCCCTGCTTCACCCAAGAAGAAGCCGATGCCGTTTCCAAAGTCCTGCTG
TCCAACAAAGTCAACTACTGGACGGGCAACGAATGCCGCGAATTTGAAAAAGAATTTGCC
GCCTTTGCCGGCACGCGGTACGCCGTCGCCCTTGCCAACGGCACGCTGGCACTCGATGTC
GCGCTCAAAGCAATGGGCATAGGCGCGGGCGACGATGTGATTGTTACCTCGCGCACCTTC
CTCGCTTCCGCGTCCTGCATTGTGAACGCGGGCGCAAACCCCGTGTTTGCCGATGTGGAT
TTGAACAGCCAAAACATCAGCGCGGAAACCGTCAAAGCCGCGCTGACACCGACTACCAAA
GCCGTCATCGTCGTCCACCTCGCCGGTATGCCCGCCGAAATGGACGGCATTATGGCTTTG
GCAAAAGAACATAATCTTTGGGTAATCGAAGACTGCGCCCAAGCGCACGGCGCAAAATAC
AAAGGCAAATCCGTCGGCTCTATCGGACACGTCGGCGCGTGGTCGTTCTGCCAAGACAAA
ATCATGACCACCGGCGGCGAAGGCGGTATGGTTACGACCAACGACAAAACCCTGTGGGAA
AAAATGTGGTCGTACAAAGACCACGGCAAAAGCTACGATGCCGTGTACAACCACGAACAC
GCGCCCGGTTTCCGCTGGCTGCACGAAAGTTTCGGCACAAACTGGCGTATGATGGAAATG
CAGGCGGTCATCGGACGCATCCAGCTCAAACGCCTGCCCGAATGGACGGCGCGCCGCCGA
GAAAACGCCGCCAAGCTGGCGGAAAGTTTGGGCAAATTCAGCAGCATCCGCTTGGTTGAA
GTCGCCGACTACATCGGACACGCGCAATATAAGTTCTACGCCTTCGTCAAACCCGAACAC
CTCAAAGACGGCTGGACGCGCGACCGCATCGTCGGCGAACTGAACGCGCGCAAAGTCCCC
TGCTATCAAGGCAGCTGCTCCGAAGTCTATTTGGAAAAAGCCTTCGACAACACGCCGTGG
CGACCGAAAGAGCGTTTGACAAATGCTGTCGAGTTGGGCGACACCAGCCTGATGTTCTTG
GTGCACCCGACGCTGACCGACGACGAAATTGCGTTTTGCAAAAAACACATCGAAGCCGTC
TTGACCGAAGCCGCACGATAACCCTTCAGACGGCATATGCCGCCTGAAAACACATACCGC
CCCACGATATGAATCTGGAAACTCTGATCGCCCTGCCGCGCAACATCAAGAAAATCTGTT
TCCTCATACACGATTTTCTGATGATTTTCATTGCCTTTTGGTTCACCCAAAGCCTAAAGG
CCGACTACTCGGACGAATGGTTCGATTTTGCCAACTGGCAGTCTTTTTTGCTGACTGCCT
TGCTGACCATCACATTATTTATCCGAATGGGGCTTTACCACGCCGTTACACGCTTCGTCA
GCTTCCGCATCCTCACCACCGCACTGGCGGGCAGCCTCGCCTCCGCCGTGTTGTTTTTCC
TCAATACGCTGATATTTGAAGAAAGGCTGCGCCTCGCCCTGCCGATTGTCTATTTCTTAC
TGCTGTTTGTTTCCGTGACCGGCTCGCGTATGGTTTTGCGCGGACTGTTGTCCGAACACC
CCAAAAAACAGATGATCCCTGTCATCATTTACGGCGCGGGACGGTCGGGCAGACAACTGC
TTGAGGCCGTCAAACAAATGCGCGAATATTCCGCCGCCGCCTTTGTAGACGACGACCCCA
AACTGTGGCACACCGTCATCTACGACCTTGCCGTTTACCAGCCCGATGCCATCGCCTTCC
TCATCGAACGCTACGGCGTGGAAAAAATCCTGCTCGCCATCCCCGGCGCGACCCAGGAAC
AACGCCGCCGAATCATCAACAAACTGGAAGCCTATCCGTGCGAAGTGTTGACCATTCCCG
GAATGAAAGACCTGATGGACGGAAAAATCAGCATCGGCACGCTCAAAAAAATCTCTGTGT
CCGACCTGCTCGGGCGTGATTCCGTCGCGCCCGACGACCGCCTGATGAGTGCCGACATCG
AAGGCAAAACCGTCATGGTAACCGGCGCGGGCGGCTCCATCGGTTCGGAACTCTGCCGCC
AGATTATCCGCCGCCGCCCCGAAAAGCTGCTGCTGTTCGAGTTATCCGAATTCGCCCTGT
ACGCCATCGAAAAAGAATTGCGCGAAACCTGCATCCAAAAACGCCTCGACACCGAAATCC
TGCCCTTTCTCGGTTCGGTGCAAAACCGCACGCTGCTCGAACACGTCATGACCGCCTTTT
CCGTTGCGACCGTCTATCACGCCGCTGCCTACAAACACGTCCCCATGGTCGAGTTCAACA
CCGTCGAAGGCATACGCAACAACATCTTCGGCACACTCGAGTGCGCGCTTGCCGCCACGA
CATCGGGCGTAAGAACTTTCGTCCTCATCTCCACCGACAAAGCCGTCCGCCCCACCAACA
CCATGGGTGCCAGCAAACGCATGGCGGAACTCTGCCTTCAGGCACTCGCCGCCGAACCCG
GACAAAAAACCCGCTTCAGCATGGTACGTTTCGGCAATGTTTTAGGTTCGTCCGGCTCCG
TTGTCCCGCTGTTTGAAAAACAGATTGCAGAAGGCGGCCCGCTTACCCTGACCCACCCCG
AAATCACACGTTATTTCATGACCATACCCGAAGCCGCCCAACTCGTCATACAGGCAGGCG
CGATGGGTACGGGCGGCGACGTATTCGTCCTCGACATGGGTGAATCCGTCAAAATCATCG
ACCTTGCCCGCCAAATGATTACCCTAAGCGGCCTCAAACCCAACACACCCGAACAACCCG
ACGGCGACATCGAAATCCTCATTACCGGACTGCGTCCCGGAGAAAAACTCTACGAAGAGC
TGCTCATCGGCGACAACGTCCGCAAAACCGGCCATCCGCGCATCATGACCGCCAACGAGA
CCATGCTGCCGTGGCACGAGCTCTCCGCCCTGCTCGACCGCATCCGTGCGGCCTGCGACC
GTTACGACCAGCAGGCAATCCGCACCCTGCTCATCAACGCCCCGACCGGCTTTGCCCCGA
GCGACGGCATCTGCGACCTGCTTTGGGTACGAGAAACACACAGAAAAAATGCCGTCTGAA
CCTTCAGACGGCATAACGTACAAACCAACCTACCTTACACACACGGAGTTTGACATGCAG
TTCTCAGCATTCGGCGAAAAATTCACGCAACACAGCGGCATCCTCCAACTGATGGACGAC
CTCGGCGACGCGCTCAAAAGCGACAAGCCCGTCAACATGCTCGGCGGCGGCAACCCGGCG

## Appendix A

CGCATTCCGGAAATCGATCAGGCGTTCGCCGACATATTCTCCAAACTGGCGGCAGAACAC
GCCGTCGAAAACATCGGCAACTACTCCAATCCCCAAGGCGATGCCGTGCTGATTGACGCG
CTGACCGCCTTCCTCAACCGCGAATACAGCTGGAATCTGACCGCCGACAATATCGCGCTG
ACCAACGGTTCGCAAAACGCGTTTTTCTATCTTTTCAACCTCTTCGGCGGCAAATTCAAC
CTTTCAGACGGCACATCCGCAGAAAAAGCCATTTTGTTGCCGCTCGCGCCCGAATACATC
GGCTATGCCGACGTGCATGTCGAAGGGCAGCACTTCGTTTCCGTCAAGCCCAAAATCGAA
AACGTCGAACACGAAGGCGAAGCCGGCTTCTTCAAATACCGCGTGGACTTTGACGCACTG
GAAAACCTGCCCGAACTCAAAGCGGGCAAAATCGGCGCGATTTGCTGTTCGCGCCCGACC
AACCCGACCGGCAATGTGTTGACCGACGGCGAAATGGCGCGTTTGGACGCTTTGGCGCGT
GAACACGGCATTCCGCTGATTATCGACAACGCCTACGGAATGCCGTTCCCCAACATCATT
TACAGCGACGTAACGCTGAATTGGCACGAAAACATCATCCTCTGCTTCAGCCTGTCCAAA
GTCGGCCTGCCGGGCGTGCGCACCGGCATCATCGTCGCCGCGCCCGAAGTCGTCAAAGCC
GTCAGCAGCCTGAACGCGATTGTGAACCTTGCCCCCACGCGCTTCGGCGCGGCCATCGCA
ACGCCGCTGCTGGAAAGCGGCGAGATGAAACGGCTTGCCGACCAAGTCATCCGGCCGTTT
TACCGCAATCAGGCGCAAACCGCCGTCTCGCTGCTCAAGCGGGAGCTGGGCGCGTACCCG
ATGAAAATCCACAAACCCGAAGGCGCGATTTTCCTGTGGCTCTGGTTTGAAAACCTGCCC
GTTTCTTCGCAAACCCTGTACGAAATGCTCAAAGCCGAAGGCACACTGATTATTCCGGGC
GAACATTTCTTCGTCGGCATCGACACGCAGGATTACCCGCATGCGGGCGAGTGCATCCGC
ATGAGCATCGCGCAGGACGCTCAAACGCTGGAAAAAGGCATTGCCGCCATCGGTAAAACC
GTCCGAAAACTGTACGACAACGTTTAAAACGCAAAAAATGCCGTCTGAAAAGTTTTCAGA
CGGCATTTTTATCTGCATTCAATATCGGGAAAAATGTTCCCAAACCGGTTTGCAGTTTTC
CGGCAGCTCGGGACACGCGCCGAGGATGCCGCCGCTGAAGTCGTTTAAGCGGTGGAAGTC
GCTGCCCGCGCTGGCGAGCATACCGAAGCGTTCTGCCAAAAGCGCGTAGTTGAGGCGGTC
GTTTTTGCAGCAGTTTCCGCTGTGGACTTCGATGCCTGCGCCGCCGAGGTTTTTAAATTC
TTCAAACAAATTGCGCTTGGCGGTGGCGGACAAATCGTAGCGCATGGGGTGGGCGATGAC
TGCCATGCCGCCCGCTCCGTTGACGGCGGAGACGCAGTCTTCCAGCGTCGCCCATTCGTG
GCGGACGGCGCAGGATTTGCCGTCGCCCAAGTATTTGGTGAACGCCTGCTGCTTGTTTTT
GACGTGTCCCGCTTGGATGAGGAACTCGGCGACGTGGGTGCGGCTGACCATTTCTTTGTT
TGCCGCCAGTGCCAGCGCGCCGTCGTATGCGCCGCCGATGCCTTTCTTTTCGAGCTTGGC
GGCGATGGCTTCAAGACGTTTCAGACGGCCTTTCCGCACTTGCGCCAACAGGTTTTGCAG
GTTTTCGTCCTGCTCGTCGAAATCCAAACCGACAACGTGTATGGTGCGCCCGCGCCACGT
TACGGAGATTTCCACACCGTTAATCAGGCGCAAACCGAGCTTGTCGGCTTCGGCGCGCGC
TTCGGCGATGCCGCCGGTGTGGTCGTGGTCGGTCAACGCCAGCAGCGTGCAGCCGTTTTG
ATGCGCGAGGCGCACGACTTCGGCGGGGAGAGCATACCGTCGGAAACGGTGGAATGGCA
GTGCAGGTCTATCATGGGGTGTTATGTGTGTTGTGAATGAAGGTCGGGGGTTAATATTAT
CGGTTGGTGTGGATACAGCGGTGATTTCAACAAACAGGTGTATGGCAAATGCAAAGGAAA
AGTCCCTATGCCGTCATTCCCGCGCAGGCGGGAATCCAGACCTTGATTTGTCAAAAATAT
TTAAGGTTAACCGCTATTTCGAACTTCCGGATTCCCGCCTGCGCGGGAATGACGATATGG
ACGTTTTCAGTTTTAATCTACTATAAAAGACTGTCTGAAAACGTGGTTTTATAGTGAATT
AAATTTAAACCGGTACAGCGTTGGCTCGCCTTGGCTCAAAGAGAACGATTCTCTAAGGTG
CTGAAGCACCAAGTGAATCGGTTCCGTACTATTTGTACTGTCTGCGGCTCGCCGCCTTGT
CCTGATTTTTGTTAATTCACTATATCAAGCCGAACCGTTTCAGACGGCATCGTCCGACCA
ACCCGCTTCTTTCAATTTCTGCCGTTGCACGTCGTATTTGGCTTTATCCGCCCAGTAAAT
CGTCTGAATGCACGCCTCGCCGCAGTCGCTGCCGCAGCATTCCCACGACTCGGGTCGGAC
GGGTTCGTCTAAAAGCGGCTCGCCCAAAAGGGCTTCGGCTTTATACTTCAGGGTCGTATC
CATCGGCGATTTCCAAGCGAGCGCCGTCAAACTCGATGACTTCGCCGCCGCGTATTTTGG
CGGTTTTACGGGTTTCGGTTTCGCCGTTGCGCAACACCAGCCCTTCGGCGATAAACGCTT
TCGCCTGTCCGCCGCTTTCGGCAAGTCCGACCAATTTCAAGAGGTCGCACAAGGCGATGT
ATTCGTTGTCTTCGAGATAGACAGTGGCTTCCATAATGTTCCCTTGCAGAAAGAGGCCGT
TATTGTAGCACCTGCCGCCGCCGTACCCAAAATTACCGAAAAACCGGCGATGTATCCGCA
CCGCCTGTTCCGTAAAAGTAAAAATGCCGTCTGAAACCCCATATGCCGCCATCCGTTCAA
AGAAATCCTGCCCAACGGCAGACTGCAAATCCTGTTCCCCGACGAATCCGCATTGACGCT
GATGCACATCCTCAAACGCGAACTGCCCGATACACCGGCAATCGGCATCAAAACGAAATC
AAAAACCTGTTTGAACGTAAAATTAATTTAACTGCTTGCCATCCGTCCGAATAAGGCATA
TAGTTCTTTATAACTAGTTTGATAGTCCTTTATATCTATCAATACTCCTTGGGAAGCCTC
CGCCATACGGCAGGAGGCATTTTTTTGCCGTAGTAAAAGCTCAAAAACATTTGCAGGTCA
TGCCGTCTGAACCCGAAACGGCATTACCTACACCGCCATCTAAAGACAACCCTGCTACAA
TACGCCTTTTATTGTCCACGCCGATTTTGCCATGACCGAGCCGACCTACATTCCCCTGCG
CCTGCATACCGAATTTTCGATTACCGACGGTATGGTGCGGATTAAAAAACTGATTGCCAA
AGCGCAGGAATACGGTTTGCCTGCTTTGGGCATCAGCGATTTGATGAACGAATTCGGTTT
GGTGAAATTTTATAAAGCCTGCCGCAGCGCGGGGATTAAGCCTATCGGCGCGGCGGATGT
GCGGATAGGCAATCCGGATGCGCCCGACAAGCCGTTCCGCGCTATGCTGATTATCCGTAA
CGATGCGGGCTATCTGCGCTTGAGCGAGCTTCTGACGGCGGCTTATGTCGGCAAAGACCG
CAATGTCCATCATGCGGAACTCAATCCCGAATGGCTGGAAAACGGCGACAACAGCGGCTT
GATTTGTTTGAGCGGCGCACATTACGGCGAAGTGGGCGTGAATCTGTTGAACGGCAATGA
AGACGCGGCGCGTACGGCGGCGTTGAAGTATGCGGCGTGGTTCCCCGATGCGTTCTATAT
GGAGCTGCAACGCCTACCCGAACGCCCCGAATGGGAGGCTTGCGTTTCGGGCAGCGTGAA
GCTGGCGGAGGAATTGGGTTTGCCGGTGGTGGCGACGCATCCGACACAGTTTATGAGCCG
CGACGATTTCAACGCGCACGAGGCGCGAGTGTGTATCGCAGGCGGCTGGGTATTGACGGA
CAAGAAACGTCCGCGCGATTTCACGCCGGGCCAGTTTTTCATTCCGCCGGAAACCATGGC
CGAACGTTTCGCCGATTTGCCTGAAGCCTTGGAAAACACGGTAGAAATTGCCAAACGCTG
CAACCTGCACATCACATTGGGCAAAAACTTCCTGCCCCTTTTCCCCACGCCCGACGGTTT
ATCACTCGATGACTATCTCATCAAACTCTCCAACGAGGGTTTGCAGGAACGTATGGTTCA
GCTTTATCCCGACGAGGCGGAGCGGGCGGCAAAAATGCCGGAATATCAGGAACGTTTGGA
TTTTGAGCTGAACATCATCATCCAAATGAAATTCCCCGGCTATTTCCTTATCGTACAAGA

## Appendix A

CTTTATCAACTGGGCGAAAACACACGGCTGTCCGGTCGGGCCGGGCCGTGGTTCGGGCGC
GGGTTCGCTGGTGGCGTATTCATTGAAGATTACCGACCTTGATCCGCTCAAATACGCGCT
GCTGTTCGAGCGTTTCCTAAACCCCGAACGCGTTTCTATGCCCGACTTCGACGTGGACTT
TTGCCAAAGCAACCGCGGCCGCGTGATTGAATATGTGCGCGAGAAATACGGCGCGGAGGC
GGTCAGCCAGATTGTTACCTTCGGCACGATGTCGTCCAAAGCGGTCATCCGCGACGTCGG
GCGCGTGTTAGAGCTGCCGTTTATGCTGTGCGACAAACTGTCCAAGCTGATTCCGTTGGA
AGCCAACAAACCCCTGAGTTTGGAAAAAGCCATGGAGACCGAGCCACAGATTCAGGAATT
AATCGAAGCGGAAGAAGCGGACGAACTGATTACGCTGGCGAAAAAGCTGGAAGATTTAAC
GCGCGGTTTGGGTATGCACGCAGGCGGCGTGTTGATTGCGCCGGGCAAGATTTCCGATTA
CAGCCCCGTGTATCAGGCGGACGAATCCGCCTCGCCCGTATCCATGTACGACAAGGGCGA
CGTGGAAGATGTGGGTTTGGTGAAATTCGACTTTTTGGGTCTGCGCAACCTGACCATTAT
CGAAATGGCGCAGAACAACATCAAAAACACTACCGGCGACATCATCGATGTCGGCAAAAT
CCCGCTTGACGACCAGGTCGCCTACCAAATCTTCCGCGATGCGAACACCACCGCCGTCTT
CCAGTTTGAGTCGACCGGCATGAAAAAAATGCTGAAAACGGCGCACACGACCAAGTTTGA
AGAACTCATCGCCTTCGTATCGCTCTACCGCCCCGGCCCGATGGACAACATTCCCGACTT
CGTCGCACGTATGAAGGGGCAAGAATTCCAATACATCCATCCGCTACTCGAAGGCATCCT
CGCGCCGACCTACGGGATTATGGTGTATCAGGAACAAGTGATGCAGGCGGCGCAAATTAT
CGGCGGCTACTCGCTCGGCGGCGCGGACCTGCTGCGTCGCGCCATGGGTAAGAAAAAACC
CGAAGAAATGGTGAAACACCGCGAAATCTTCGCCGAAGGCGCGGCAAAACAAGGCATTTC
GCGCGAAAAATCCGACGAAATCTTCAACTACATGGAAAAATTCGCCGGCTACGGTTTCAA
CAAATCCCACGCCGCCGCCTACGCCCTGATTTCCTACCAGACCGCATGGCTTAAAGCGCA
CTACCCCGCCGAATTTATGGCGGCGACCATGTCGTCCGAATTGGACAACACCGACCAGCT
CAAGCATTTCTACGACGACTGCCGCGCCAACGGCATTGAGTTCCTGCCGCCCGACATCAA
CGAATCCGACTACCGCTTCACGCCGTATCCGGACATGAAAATCCGCTACGCGCTCGGCGC
GATTAAAGGCACGGGCGAAGCCGCCGTCGAATCCATCACCGCCGCGCGGCAAAGCGGCGG
CAAGTTTACCGGTCTGTTGGACTTCTGCGAGCGCGTCGGCAAAGAACACATGAACCGCCG
CACCCTCGAGGCCCTGATACGCGGCGGCGCGTTCGACAGCATCGAACCCAACCGCGCCAT
GCTCTTGGCGAACATCGACCTCGCTATGGACAACGCCGACCAAAAAGCCGCCAACGCCAA
TCAGGGCGGGCTTTTCGACATGATGGAAGACGCCATCGAACCGGTGCGGCTCATCGACGC
GCCGATGTGGAGCGAATCGGAAAAACTCGCCGAAGAAAAAACCGTCATCGGCTTTTACCT
GTCCGGCCACCCGTTCGGCCCGTATGCCCAAGAAGTCCGCCAAATCGCACCGACCAAATT
AGACCGTCTGAAGCCGCAAGACAGCGTGCGCCTCGCCGGATTCGTTACCGCCGTGCGTAC
GATGATGGGCAAACGCGGCAAAATCGCCTTCGTCAGCCTCGAAGATTTGAGCGGACAGGT
TGAAATCATGGTCGGCGGTCAGACGTTGGAAAACTGCGCCGACTGCCTCAAAGCCGACCA
AGTGCTGATTATCGAATCCAAAGTCAGCCGCGACGACTACGGCGGCGGCGACGGGCTGCG
TATTCTGGCAAACCAAGTCATGACCCTGCAAACGGCGCGCGAACGCTACGCCCGCAGCCT
CAGCCTCGCCCTCGCCCCGCATCACGACATCGGCGGACTGGTACGGCTGCTCGCCGCCCA
CCAACTGCCCGACACGCCGCGCATCCCGCTGCAACTGTCGTATGCCAACGAAAAAGCGTC
GGGCAGGCTTCAAGTGCCGCCGAAATGGACGGTTACACCGAGCTCCGCATTGTTCGGCGA
ACTGGAAACATTGCTCGGCAGCCGGTCGGTGCGCGTCAACTGGTAACCCAAAATATAAAT
GCCGTCTGAAGCCCAAAAACCGGTTTCATTCGTACTTTATTCGAATGATTGAATAAAAGT
AACTGCCAAGAAAAACGTATTTTTTGGTTATTTCGCCAGTCTAAATAGAGCAACCGGGAC
GATTGATATCCGTGTGCATGACACAGACAGCACCAAAGGGAAAAACGGCATTTTCCAAAG
TATCGGTATCAAAACCGCCCTTTCACTCCAAAAATACCAAATCGACAAACCGGGCAAAGA
AATCAGACCGTGCCGCCTGAAAAAACGCCCACCCGTCCGTTAAATTAACCTATCCCTGTT
TCAGACGGCCTGAAGCAGGGATTTTTATATCAAAATAAAATGAGAAAGGGAGCAATAACC
CTTAGGTAGCTCTTGTTATTTTCCGATGCAAAACAAAGCAGTCATATATTTAATTCCCCC
TACCTCTGCCAAGCCTTCCTCAAATATTCGACGCAATCGGTCAGCGAGTAGAACGGGACA
TTGCCGTGGTCGGCATTCGGATATTCCCGGAAAAAGACGGCGGCCCCGTGCCTGTCTAAC
TCTGCCGCCATTTGTTCGGCCTGCCCTGCCATATCGCGTTCTTCCCTGCGTTTACAATCG
CTACCCCGTTCTAGCGCGCCGATGTTGAGGCAGACATCGATGCCGTTTAGCCGGTTTTCA
GACGGCATAAAGTCGAGTATCCGCCTGTTGTGCCACCAAATCGATGGGGATACGAGCCAA
TGCCGTCTGAAACGGCGGTGGGAAAGCAGGGAATACAGTCCGAACAGTGCGCCGAACGAG
TGTCCGAATACGGCGGTTTCATTGCGGTTGAGGGTGTAGCGGCTTTCTAAAAAGGCGGTC
AGCTCGCTGTCGATAAAGGCGGCGAAGCGGTCTGCCTGTCCGAACTGCTGCCGTTCGTCT
GCTGTGGCGTTGTCTCCAAGCGGCGGCGTGTAGTCGGCGGCACGTTGTGCCAAATCGCGC
ACACTGCCTGTCGTGTAGCCGATACCGACAATCAGGCAGGGGGCGTTGCTTCGGGTAACG
GGGTTGTTCATCAGCGACTGCATGATGTTGAAAAGTGCGGGGAAAAAGGCTTCGCCGTCG
AGGACAAAGAGGACGGGATAGCCTTCAGACGGTATTTCGCCGAGTGTTGCCGTCTGAATC
CGATAGATTCGCCCCGTGCAGGTGGATTTGATTTCGGTTTCAAAGGCTTGGGGCAGTATG
GCAGGTTGGAATGTGTCGGTCGGTATGGGTTTCATGATGTTCGGCTTGTGGGTCAGACTG
GCGGCAAGCGGTAAAACCGAACCGGCAAAGCGGTCTGCCTGCCGGTTCGGTCTATTTTCC
TTCGCAATGCCATACTCCAGTTGTGAGAGCATAGGGTATGCCGCGCAGCTTGTTGTAGTT
TGATGATGCTGCGGCTGCCGTTTCAGACGGCATATTGGTCTTTAAAAACTGTAACGCAGG
TTTGCCGTCAGGCTGCGCTCCGAACCGGGAATGTTAAAGGTGCTCTCGCTGCCGACGCGG
GCGTAGTAATGGCGGTTGAAGATGTTGTCGGCGTTGATTTGCAGCTTCAGTTTGGGCGTG
AAGCGGTATGCCGCCATCGCATCGAACGTGGCATAACCGCCTGCATGTATCCCTGCAGAT
GAAGTAATGCCGCTCATCGCGTTCACGCCGCCGCCGATGGTCAGCCCGGACGTAACTTGG
TAAGTCGTCCACAGGTTTGCGCTGTGTTTGGGCATCAGCAGGAAGATGCCTTCGTCGCGC
GAATTGGAGGCGGTTTTGATTTGGCTGTGCAGGTAGCTGTAACCTGCATGGATTTGCCAT
TTCGGTGTCATCGCGCCGCTGATTTCGGTCTCAACACCTTCCATCACGCGTTTGCCCAAT
GCGGCGTAACGGGTTTTTTTGTTGTTTGAGTCCAGCGGTGCGGCGGCGTTTTTATCCTTC
ATGCGGTAGAACGAAACCCGGGTATTGAGGCGGTCGTCCATGTAGCTGCCTTTGTAGCCG
ATTTCAAACTGGTTGCCTTCGCGCGGTTTGAGCAGCTTGCCGTCGGTGCCGATGCTGGTT
TGCGGTGTGTAGAGTTGGGAGGCGGAAGCGTACAGGCTGTTGCTGCCGTCTATATCGTAA

# Appendix A

ACCGCGCCGGCGTAGCTTGTAAATTTGGTTTTCGAAGCTTTATGCAGGGTTTTGCCGTCG
CCCGACTCGATTTTGTGATGTCCTACACGTCCGCCTGCAATCAACGACAAACCTTCCAGA
GGACGGAACACCGTCTTGGCATACAAACCGGTTTCGTCGAGGTTTTCTTCGGTAACGGAG
TGATTGAAACCTTTGTTTCCGGCGCGGGCGTTCTGAAGTATGCCGTTATAAGGCAAAGCG
CGGAAACCATCTAAAGCGACGCTTTTTGACAAAGTCGAACGCCCTTGTTCATTAGTACTG
CGCAAGCGGTTGTAGTCTGCACCAATCACAAATTCGTTGGCGGTGTTGCCCAAGGCAAAC
GGACGGCTGTAACTTGCGTCAACCGCAAAGGCTTTTTGTTTAATGTCCGTACCCAAACCC
GCTACGTCGGCTTGTCCGGTATTGTTGAGTTTGCTGCCCGCAAACGTATAATTGGAATCG
GCTTTCCGATCGGAATAGCGCATACCGACTTTGCCGTAGCCGCCGTTGCCGAAGTAATGT
TTCAAATCGGCGAACACGTCGTGGCTGTGCATTTTAAATTTGTTCCAATCCGCGCCGACA
AATACGTGTTGCGGCAGGGACGGTAATTTGTTATTGGCATCGGCAGGCAGGCCGTTGTAC
GGCGCGAGGCGGCGTTGCTGGTAAAGATAGCCCGCGCCCAAAACCGTATCGGGGTTGATG
TCCCAATCCGCCGCCGCGTAGAAGGTTTCGCGCCGGTTGTTTTTCTCGGCGGGACGCGGA
GACGCGCCGACGGTCTGCGCCATCACGCGGCCGCGCACGCTGCCGTCTGAATTGAGGCTG
CCCGATACGTCCGCCTCGGCTTTATATTGTTTGTGCGTACCGAACCCTGCCGCCGCATGA
CCTTGGAACGCTTTGGTCGGGCGTTTGCGCACCAGATTCACGATGCCGCCCATCTCGCCG
CTGCTGTCGAACAGTCCGCTCGGCCCGCGCATCACTTCCACGCGGTCGAAGGCGAACAGG
TTGGGCAGCGTGCCGTTGATACTCTGCATCTGCGCGGGCAGGCCGTCGATGTTGTATTCG
CTGTATTCGTAACCGCGCGCGTAAACCGAAGAGCGTCCGTCGTCGTTGCTCAACACGCGC
AGGCCGGGCGTTTTGCGTGCCAACTGGTCAAACGTATCAACATTGCGGTCTTTGACCTGC
TGGTTGGTAATGATGCTGACGGATTGCGGAATTTCGCGCAAAGAAGCGGGGATTTTTGTA
CCGACGGTGGCGGCAAACGAGCTGTAATCGCCGTTTTTCTCGGTGGCAATCGCGTTGTAA
GAACGCTGACCCTTAATATGGACGGTTTCCAAACCTTCCGTTTGTGCGGCAAAAACCGAA
GACGAGAGTGCTGCCAAAACCGTGGCGGCGGTCATATTGATGCGGAAAACTGACATAAAC
TGTCCCATTCACATAAATGATAATGGTTCTATTTTAATAAAGCGCAACGCGGCTTGTTCG
GAAAAACATATCGCGCAGCCGACAAATTTTGTCGAAAATGCGACACGTCTGCGTTTTCCG
CATAAAATTTGCTTTTTTACTGCAACCAACCTGCTATGACCACGCCCAAACTCATCATCT
TCGACTGGGACGGCACGCTTGCCGATACGACCCAACCCATCATCGACACCATGCGCCGCA
GCTTCGCCGAATGCGGTTTTCCGCCGCCCGAAGCGGAACGCGTCCGCAGCCTGATTGGCT
ACAGCCTGCCCGAAATCATCCGCACCCTGCTCGAAATGCCGTCTGAAACCGCCGTTGCCG
ACATCACACGCACTTATTCCGCACATTACCTCAATCCCAACAACCGCAATATGTCCTTAT
TTCCCGATGCCCTGCCCTGTCTGGACAAGCTCAAAGCACAAGGATACTGGCTTGCCGTCG
CCACGGGCAAAGGGCGGGCGGGTTTGGACAACGCCATCAGTCAAACCGCCACCGGCGGCT
ATTGGCTCGCCACCGCCTGCGCGGGGGAATATCCCTCCAAACCCTCGCCCGAAATGGTAT
TCGGAATCTGCGGCGAACTGGGACTCGACCCGAAAGAGGCATTGGTCGTCGGCGATACGG
CGCACGACCTGCATATGGCGGCAAACGCAGGCGCGGCGGCAGTCGGCGTGGCCACCGGCG
CACATTCGCGCGAACAGCTCCTTAGCGCACCGCATCTCGCCGTATTGGACGGTTTGTCCG
AACTGCCCGGTTTTCTTGCACAACATTACGCCTGATTGGTTTCCGCATCCGGCACACGGC
AAAAATGCCGTCTGAAGCCTGTTCAGACGGCATTTGTGTTGCCCAAACATTCAACGCCTG
CGTCAACGTTTGCACAAATCGGGTTTGGTTTCGCCCTCGCGGCGCAACTCTTTGGGCAGG
ACGAACACAATGCTTTCTTCCGCACCCTCGCCTTCGCGTACGGTTTCGTGCCCCCATCCG
CGTATGGTTGCCAGTACTTCCCGCACCAACACTTCGGGCGCGGACGCGCCTGCCGTTACG
CCGACTTTGTTTTTGCCCTCAAACCATGCGCGTTGCAGGTAGCCTGCATTATCCACCATA
TACGCATCGATTCCGCGCGATGCCGCCACTTCGCGCAAGCGGTTGCTGTTGGACGAATTG
GGCGAACCGACCACAATCACGATGTCGCACTGTTCTGCCAACTCTTTGACGGCGGTTTGC
CGGTTGGTCGTCGCATAGCAGATATCTTCCTTGTGCGGATTGCGGATATTGGGGAAACGC
GCGTTCAGCGCGGCGATGATGTCTTTGGTTTCATCGACCGAGAGCGTGGTTTGGCTGACA
TAGGCGAGTTTGTCGGGGTTTCTGACTTCGAGTTTTGCCACATCTCCGACCGTTTCGACC
AAAAGCATTTTGCCCGGCGCAAGCTGCCCCATCGTTCCTTCGACCTCGACGTGCCCCTTA
~~TGCCCGATCATGATGATTTCACAGTCTTGGGCATCCAGTCGGGCGACTTCCTTATGCACT~~
TTCGTCACCAGCGGGCAAGTCGCATCAAACACGCGGAAACCGCGCTCCGCCGCTTCTTGC
CGCACCGCCTTCGATACGCCGTGTGCCGAATAAACCAGTGTCGCGCCCGGCGGCACTTCC
GCCAAGTCTTCAATAAACACCGCACCTTTTTCACGCAGGTTGTCCACGACGAATTTGTTG
TGAACGACTTCGTGGCGCACATAAATCGGCGCGCCGAACTCTTCCAAAGCACGTTCGACA
ATACTGATTGCCCGATCCACACCAGCGCAGAAGCCGCGCGGATTGGCAAGGATGATGGTT
TTCTCGTTCATAAGCCCGGTATTTCGTTTTCAGACGGCATCAATATTTTTCTTCTTGGGT
TTTACGGTGGACGATGTTGTCCAACACCGCCAACACCGCACCGACGCAGATAAAGCTGTC
GGCAATATTAAAGGCGGGATAAAACCAATTTTGCCAATAAAACAATAAGAAATCGACGAC
ATGACCGTGTATCAGGCGGTCGATGACATTGCCTAACGCACCGCCGATAATCATTGCCGC
ACCCGTTTTGCCGAGGGTTGCAAACTCATCGCGCAAGATGGCGCGTACCAAATACGCGCT
CACCGCCACCGCCAGCACCAAAAAAAAGTATTTTTGCCAGCCGCCCTGATCGGCAAGGAA
GCTGAACGCCGCACCCGGGTTGTACACCAGCGTCAGATCGAAAAAGGAAGGAATGACATT
GACGCGTTCCCGATACTGAAACGACGACAGCACCGCCCACTTCGACCACTGGTCCAGCAC
GATGGCGGCAAGTGCCAATACCCAATAGCGCGTTTTACTTGAAACAGATGAAGACATATT
TTTCAACAGCCGGTAAAAGAGTACCATTTTACCCGAAAACCCCCTTTCCTGTACCCGAAA
CGGCAAATGCCGTAATCTTAAAACCCGTCATTCCCGACAACACCGTAATCTCGAAACCCG
TCATTCCCGCGTAGGCGGGAATCCAGACCTGTCCGCACAGAAACTTATCGGATAAAAACA
GTTGCCCAAACCCCGCGTTCTATAGTGGATTAAATTCAAACCAGTACGGCATTGCCTCGC
CTTGCCGTACTATTTGTACTGTCTGCGGCTTCGTTGCCTTGTCCTGATTTAAATTTAATC
CACTATAGATTCCCACTTCCGTGGGAATGACGGTTCAGTTGCATTCCGACAACACCGTAA
TCTTGAAATCCGTCATTCCCGCGCAGGCGGGAATCTATCGGAAATGACTGAAACCTCGAG
ATTCTAGATTCCCACTTTCGTGGGAATGACGGTTCAGTTGCGTTCCAACAACACCGCAAT
CTCGAAATCCGTCATTCCCGCGCAGGCGGGAATCCAGACCTCCGACGCGGCGGGAATCTA
TCGGAAATGACTGAAACCTCGAGATTCTAGATTCCCACTTTCGTGGGAATGACGGTTCAG
TTGCGTTCCAACAACACCGCAATCTCGAAATCCGTCATTCCCACACAGGCGGGAATCCAG

## Appendix A

ACCCCTGACGCGGCGGGAATCTATCGGAAATGACTGAAACCCCGAGATTCTAGATTCCCA
CTTTCGTGGGAATGACGGTTCAGTTGCGTTCCGACAACACCGCAATCTCGAAATCCGTCA
TTCCCGCACAGGCGGGAATCCAGACCCCTGACGCGGCGGGAATCTATCGGAAATGACTGA
AACCCCGAGATTCTAGATTCCCACTTTCGTGGGAATGGCGGTTCAGTTGCATTCCGACAA
CACCGTAATCTTGAAATCCGTCATTCCCGATAACAGCGCAATCTTGAAACCCGTCATTCC
CGCGCAGGCGGGAATCCAGACCTCCGACGCGGCGGGAATCTATCGGAAATGACTGAAACC
CCGAGATTCTAGATTCCCACTTTCGTGGGAATGACGGTTCAGTTGCGTTCCGACAACACC
GTAATCTCGAAATCCGTCATTCCCGCACAGGCGGGAATCTATCGGAAATGACTGAAACCT
CGAGATTCTAGATTCCCACTTTCGTGGGAATGACGGTTCAGTTGCATTCCGACAACACCG
CAATCTTGAAACCCCTCCGCCGTTATAAAGACAAATCGCGGCACAAAAAATGCCGTCTGA
AATGCTGTTCGGCGGTTTCAGACGGCATTTGCTCAAACTTTATCAGGCGTAATGGCGCGT
TTCGCCTTCTCCGCCGACATTCTCTGCACAGCGTTTGCAGACGGTTTCATAGCCTGCAAC
CGCGCCCACATCGCGGGTGTAGTGCCAGCAGCGTTCGCATTTTTCACCATCACTGGCTTT
AGCGGCAACGGCAAGTTCGCTGCCTACTTTCACTTCTGCTTTAGACACCAGCAAAGCAAA
GCGCAATTCTTCGCCCAAAGCATTCAGATAGCCGGCCATTTCTTCCGGCGCGGTAATTTC
GGCTTCGGCTTGCAAGGACGAACCGACGGTTTTGTCGGCGCGCAAAGGCTCGATGGCGGC
GGTTACCGCTTCGCGGGCTTCGCGGATTGCCGTCCATTTTTTCACCAGTTCGGCTTCGGT
TTTTTCGTTGATGGTCGGGAACTCGTGCCAAGTATGGAAGAGGACGCTGTCTTCTTCGCC
GCCGCCGATGATGTCCCACGCTTCTTCGCCGGTGAAGCACAAAATCGGTGCAATCAAGAG
AACCAAACTGCGTGTGATGTGATACAGGGCAGTTTGTGCGCTGCGGCGTGCATGGCTGTC
TGCTTTGGTGGTGTAGAGGCGGTCTTTCAGGATGTCGAGGTAGAACGCACCCAAGTCTTC
CGAGCAGAAAGAAACAATGTCTTTTACGGCAAAGTGGAAGGCATAACGCGGATAGTAATC
GCCTGCCAGACACTCTTGCAGCTGACGTGCCAATACCACGGCGTAGCGGTCGATTTCCAC
CATATCCGCCTGTTGCACGGCATCTTCAATCGGATTAAAGTCGCTCAAGTTGGCAAACAA
AAAGCTCAAGGTATTGCGGATACGGCGGTAGCTTTCGGTTACGCGTTTGAGGATTTCTTT
GGAAATCGCCAATTCGCCGCTGTAATCGGTAGATGCCGCCCACAGGCGCAGGATGTCTGC
GCCGAATTCGTTATAAACCTCTTGCGGTGCAACGACGTTGCCGATGGATTTCGACATTTT
TTTGCCTTCGCCGTCGACAACGAAACCATGGGTCAGCAGCTGTTTATACGGCGCGCGACC
CATTGATGAGGCGCAGCCGGTCAGCATGGACGATTGAAACCAGCCGCGGTGTTGGTCGCT
GCCTTCGAGATACAAATCAGCCGGCCATTCCAATTCTTCGCGTTGTTTCACAACGGAATA
ATGGGTCGAGCCGGAGTCGAACCATACGTCCATTGTGTCAGAAAGTTTATCGTAATTTTC
GCAATCTTCCGCGCTCAAGAGTTCGCTCTTATCGAGGGAGAACCACGCTTCGATGCCTTT
TTCTTCGATTTTCAGGGCAACTTTTTCCAAAAGTTCGGCAGAGTTCGGATGCAGCTCGCC
CGTTTCTTTGTGAACAAAGAAAGTCATCGGCGTGCCCCAATAGCGTTGGCGTGAAACCAC
CCAGTCAGGACGACCTTCAATCATGGCTTCCAAACGCGCGCGACCCCAAGACGGGAAGAA
TTCGGTGTCGTCCACGGCCTTGATGGCTTTGTCGCGCAGGGTTTTGCCGTCGGCACCGGC
TTTGTCCATACCGACAAACCATTGACCTGTCGCGCGGTAAATCAGCGGCGTTTTGTGCCG
CCAGCAGTGGGCGTAGCTGTGTTCGATTTTACTGCTTGCCAAAAGGTTGCCGGTTTCTTC
CAACCATTGCAGGATGACGGGGTTCGCCTCCCAAACGCGCATACCGGCGACACGCGGCGT
TTCGCCGATGTATCGGCCTTCGGCGTTGACGGGGTTGTAAAGCTCGATGCCGTATTTATT
GCAGACGGCGTAGTCTTCCAAACCGTGCGCGGGGGCGGTGTGTACCAAGCCGGTACCGGC
ATCGGTGGTAACGTGTTCGCCGTTGAGCATGGGAATATCGCGTTCGAGGAACGGATGGTT
CATGTGCAGATTTTCCAGCTTGTCGCCGGTGGTTTCGGCGAGAATAGCAATGCCGTCTGA
AAAACCGTAACGTTTGAGCGCGTCTTCTGCCAAATCTTTCGCCAATACCAATTTGCCTTT
CGGCGTATCAATCAGTTGATACACCACGTCTGCACCCGCAGACACGGCTTGGCTCGCCGG
TAGCGTCCAAGGCGTAGTCGTCCAAATGACGGCAAACGCTTTGCCTTCGAAACCAGCCAA
ACCGAATGCGGCGGCAAGCGCGGCAGTGTCTTTAAACAGATAGGCAACGTCAATCGCGGG
CGAGATTTTGTCTTTGTATTCCACTTCCGCTTCGGCCAGCGAAGAACCGCAGTCCAAGCA
GAATTGAACCGGTTTCGCACCCCGGTAGAGATAGCCGGATTTGTAGATTTCGCCGAGCAT
~~ACGCACGGTATCGGCTTCGGTTTTGAAATCCATAGTCAGGTAAGGATGGTCCCAGTCGCC~~
CAACACGCCCAAGCGGATAAAGTCTTTTTCTGACGGGCAATCTGTTCGGCGGCGTATTC
GCGGCACAATTCGCGGAAACGTGCTTTGGGCATATCTTTGCCGTGCAGTTTTTCTACCAT
CACTTCGATGGGCAGGCCGTGGCAGTCCCAACCCGGCACATAAGGCGCGTCAAAACCGGC
TTGGGTTTTGCTGCGGATAATGATGTCTTTGAGAATTTTATTGACGGCATGACCGATGTG
GATGTCGCCGTTGGCATACGGCGGGCCGTCGTGCAGAATAAATTTCGGACGGCCTTTGGC
GATTTCGCGCAGTTTTTGGTAGCGTTTTTGCTCGTACCAGCTTTTCAGCCATGCAGGCTC
GCGCTTGGCAAGATTGCCGCGCATCGGAAACGGGCTCTCGAGCAGGTTTACGGTTTTACT
GTAATCGGTCATTTTTTAATCTCTATTGTTACAATATTTCGGTCTCAGACGGCATTGCGC
CCCAAACAGCATTTCACAACGGGAAAACCCTGTGCCGTCTGAACAGTTAAAAGATTGATT
GTAGCCCAATCGGATGGTTTGTATAAGGTTTTTCTACCAACGCCTTGCGGCTTCCATATC
GGCTTCAATCTGCCTTTTCAGTTCTTCCATACCGTCAAACTTTTCCTCATCGCGCAGTTT
GTGCAGGAAGCGGACGTTCAGCCCTTGTCCGTACAGGTCGCCTTGAAAGTCGAACAGGTG
GACTTCAAGCTTTTGAGAACAGCCGCTATCAACGGTGGGATTGAAGCCGAAACTCGCCAC
GCCGCGCCGCGTGCCGAATGCGCCGTCTGCTTCGACGACAAACACGCCGCCGAGTGCATA
ACGGTGGCGGGGCAGGCGGATGTTGGCAGTCGGGGCGTTTAAGGTGCGTCCGAGTTTTCT
GCCGTGCACCACCCTGCCGCTCAAGACGTAGTCGTGTCCCAAAAGTTTTTCGCATAGGC
AAGGTTGCCGTCTGAAAGGGCTTGTCGCACGGCGGTACTGCTGGTGCGGATGTCTTCGAC
GATGACGGAAGGCGTACGCTCGGTCTGCATATCGGGCTGTTGTGCCAAAAGTTCAAAACA
GCCTTCCCGCCCCGCACCGAAACGGAAATCATCGCCGACGAGCAAATAACGCGTATTCAA
GGTTTGACGCAGCAGGCGGTCGATAAACCCTTGCGCGGATATTTCGGAAAAATTTTGATC
GAAACGCAAAACCCAGACGGCATCGACACAGCCTGTGCCTTCCAATAATTCGAGCTTGGT
GCGCAGGGGGCTGATCCGACACGGTGGCATCCTGCCGGTGCGGAGTGCGAAAAATTCTTT
GGGTTGGGGTTCGAAAACGACGGTCACGACGGGCAGTCCGCGCGCGTCGGCTTCGAGGCG
GAGTTTTTGGAGGATGTGTTTGTGTCCGAGGTGTACGCCGTCGAAATTGCCTATGGTTAC
GGCGGCACCCTGTGGAAAGTCGGGCGCGTTGTGCCGCCCCAGCCTGATTCTCATTGTTGC

# Appendix A

ATTCGGGTATGTGGTGAAACAGGCGGTCATTGTAAACGGTATTGCGGTTTATAGACAGTG
TGCCGCCCGTTACGCCCGCCCGACGCGGGAAAAGTAGGCAAATTTCCCGCCGCCGAACGC
GCCAAACGCACAAAAACGCGCAGCAGGCGCGGTGCTATGTGTTGAAACATCGCCCCAAAC
CTGATGCAACATCAGTGAAAATCGTTCTTTTTTAACCAGGTAAACCGAGACAAACAACCC
TCCGCCGTCATTCCCGCGCAGGCGGGAATCCGGACCTGTCCGCACAGAAACTTATCGGAT
AAAAACAGTTGCTCAAACCCCGAGATTCTAGATTCCCACTTTCGTGGGAATGACGGTTCA
GTTGCGTTCCGACAACACCGTAATCTTGAAACTCGTCATTCCCGCTCAGGCGGGAATCTA
GAACGTGGAATCTAAGAAACCGTTTTGCCCGATAAGTTTCCGTGCGGACAGGTCCGGATT
CCCGCCTGCGCGGGAATGACGGCATTTCTGCGGCAATCGGATTATTTCCAAACCAAAAGC
GCGTGGTTGCGTTTGCCGCGCCGAAGGATAGTGTATTTGCCGAAACGTTTGTGTTCGCCG
TTCAGCAGGCAGGCATCGTCGGGGCGTTCGGCGGCGTGGTTGGGGTTGTTGGCTTCGGCA
GGTTTGCCGTTGAGCAAAACCGCTTTGCTGTTCACAAAGCCGCGCGCTTCTTTATTGGAG
GATGCCAAACCGGTTTTTACCAAGGCTTCGACGACATTGATGCCGTCTGAAACTTCAAAT
GCAGGCAGGCCGTCGAGGGCGAGCTGCTCGAAGTCGCTTTCGGTCAGGCTGCTTTGGTCT
TCGGCAAACAGGCTTTCGGAAATGCGTTGCGCGGCGGCAAGGGCTTCTTCGCCGTGAATC
AGGCGGGTCATTTCTTCGGCGAGGATGCGTTGCGCTTCGGGCTTGCTGCCGCTTGCCTTG
TCTTTGGCTTCGATGGCATCGATTTCTTCGATGGACAGGAAGGTAAAGTATTTCAGGAAT
TTATACACATCGGCATCGGCGACTTTCAGCCAGAATTGGTAGAACTGATAGGGCGAGGTT
TTTTTCGCGTTCAGCCATACCGCGCCGCCTTCGGTTTTGCCGAATTTGGTACCGTCTGAT
TTGGTTACCAAAGGCAGGGTCAGACCGAATACTTGTTTTTGGTGCAGGCGGCGGGTCAGG
TCGATACCGGCGGTGATATTGCCCCATTGGTCGGAGCCGCCGATTTCCAAAACCGCGCCG
TGGCGTTTGTTCAACTCGGCGAAGTCGTAACCTTGCAGCAGGGAATAGGCGAACTCGGTG
AAGGAAATGCCTGCGCCGTCGCGGTCGATGCGCTGTTTGACGGATTCTTTGTTCAGCATG
GCGTTGACGGAGAAATGCTTGCCGATGTCGCGCAGGAAGTCAAGGCAGTTCATGCTGCCG
AACCAGTCGGCATTGTTCGCCATAATGGCGGCATTTCCGCCTTCAAAGCTCAAGAAAGGG
GTTAATTGGTTGCGGATACTTTCCACCCAGCCGGCAACAGTTTCGGCGGAATTCAAGCTG
CGTTCGGCGGCTTTGAAGCTGGGGTCGCCGATCATACCGGTCGCGCCGCCCACCAAAGCA
ATCGGCGTATGCCCCGCCTGTTGGAAGCGGCGCAATGCCAATACGGGCAGCAGGTGTCCG
ATGTGCAGGCTGTCGGCGGTCGGGTCGAAGCCGCAATAAAGGGCAATTTTTTGTTCGTTC
AACAAAGCGTCTAAGGCTTCGATGTCGGTGGTTTGCGCGATAAGGCCGCGCGATTGCAGG
TCTTGGATGACGCTCATCGGTCTCTTTCAAAAAAAATTAGCGTTTTTGCAAACCGCCGAT
TGTAACAAATTTAAGCGAATCAATGGTTATGGCGCGTATCGAGAAACCGTTGTTTTTCGG
AAAAACGCTTTGCCAATTCCGTGCCGCCGTAAGGGTTGATGTGGTCTTTGTCCGAGTAAA
CCGGCAATCCGCCGATTTGAAAATCTGCGGGGATATAGGCGGCGGCATCAATAATATAGA
CGTTGGGGTATTTGGCTGCCAATTCCCTGATGCGTGCATTGGCTTTCAGGGTGCTTTCGT
CGTCCGGGCGCAGGGCTTGGCGGTAACCCGGTATGCGTGAAGACAAGATATAGGCGCGCT
GGACGTTGTAAGACGAGGCAAGGTTGTCCGCCATCAGGTAAACGGCTTGTTTTTCGGACG
AGAGTTTATGCAGCATACGGTCGAATTTTTGGAAAAAACCGGCATCATAGGCAAGGGAGC
GGCTGTTTTCGGGCATTTGGCTGCCCCAGCGCATCGCCAAAACCACTTTTGAATACCGGG
GCAGGTGTTCTTCGGCATAGCGATAAACGGCGCGGCAGGCTGCCCAGTTTTGGAACACAC
GGGACGCGTAGCCTTCCACATAGGCGCAAGCGTCGGCGGAAACCATAGTGGCGGACCATT
TTTCTTTTTTGCCCACGGCATCGAAGAATGTTTTGTAATGGTCGGCGTGGGAGTCGCCCA
AAACCAGCAGTTCCGGCTGTTTTTCCGTATCCCCCCATAGGCATTGTTTGCCGGTATTGT
TGTGGCAGGAGGTGTTGGAACGCGTCAGCCCCAAGCGGTCGTATTGCGCCATAAACGGCA
GTCTCATCGCAAAAAACGAGCCCGCCCCCAAAATGAGCATAGGCAAGGCATAAATCCATA
AAACGGATTGTGCGAACGAACCTTGCCATTTTTTAAACGGTTTTTCGATGCAGTGGTAAG
AAAACAGGGAAAGCAGCAATATCAGGACGACCGCCGCCGCCGGCGAATAAGGCGGCAGGT
TGTCCGGGCCGATATAGCGCATAAAGGCCAATATCGGCCAATGCCACAGATAAAGCGAAT
AGGAAATCAAACCGGCGGCAACAGTGATTTTCGATTGGAAAAATTTTTTAAGCGGGTGTT
~~CGTAATGATTGAAATAAATCAGCGCGGCAACAGCCAGACAGGGAATCAAAGCGGCGGGGC~~
CCGGGAAATAGGCGGTTTGTTCCGAATAGGAAAACAGGCAGGTTGACAATATGCACACGG
CAAACAATGCGCCGACGGCGGCACAGCGTCTGCCGACGGCAGGTTGCCGGCAGCGCATCC
ACACGGCGGTCAGCGATCCTATCAGTAATTCGCAGGCGCGCAGGTGGGGCAGGTAATATT
TATCGAGCGCGGAAGGTATAAAGGAGGCGGCAAGGCTTAAGGCACACAGTGCGGCAAGGA
AGCCGAACTGTACGCGCAGGCTTTTGCGGGCGACAAGCAGCAGCAGTATCGGAAAGACAA
AGTAAAATTGTTCTTCGACCGACAAAGACCAGATGTGCAGCAGGGGCTTTTCTTCCTGCG
CGGGATCGAAATAATCCTTCCCCCTTGCAAAATACAGGTTAGAGGCGAAACCCAAGGCGG
TCAGCGCGGATTTCCACAAAAGAAAGAAATCATCTTTGGTGAATAAAAAGAAGCCGCCTG
CCAGCGTTGCCGCCAATACGGCGAAAAATGCGGGCAGAATCCGCTTGATGCGGCGGATAT
AAAATGCCTTCAGGGAAAACCTCCCCCCCCCCCCGACATTTCGCGGTGAAGAATCGTCG
TCATCAAAAAGCCTGAAATCACAAAGAATATATCGACACCGAGAAACCCGCCCGGCAGCC
AATCCTTTTCGATATGGAACACGATGACGGACAAGACGGCGGCGGCGCGCAATGTGTCGA
TGTCCGGGCGGTAGGGTAAGGCTTGGCTCATAATGTTTTTATAGTGGATTAACAAAAACC
AGTACGGCGTTGCCTCGCCTTGCCGTACTATCTATACTGTCTGCGGTTTCGTCGCCTTGT
CCTGATTTAAAGTTAATCCACTATACTCGAAACGCGGCGGCGCAAATGCCGTCTGAAAGG
TCATTTCGTATCGGGGATCGGATATTCGGAATGCCGGACGGCTTCCCGTAACGGCGGGG
CAGGCGGTTTGTTTTGCAGGAATCGGGGAGGGCAAATCGGAAATGCGGGTGGGAGTTTAT
TTTGATGCGGCTGCATTCCGGCGGTACGGAAACGCCGAAAATCATCAAAATCGGCTTCA
GACGGCATTTCCGGCAAGCCGCCTGAAACCTGCCGCATTTGGGTTACACGTTAAACAAAA
AGTGCATCACATCGCCGTCTTGCACGACATATTCCTTGCCTTCCACACGCATTTTGCCGG
CTTCTTTGGCTTTGGCTTCGCCGCCGAGCGAGACAAAGTCGTCGTAAGAAATGACTTGGG
CGCGGATGAAGCCGCGTTCAAAATCCGTATGAATCACGCCGGCGGCTTGCGGCGCGGTGT
CGCCTTTGTGTATCGTCCACGCGCGGACTTCTTTCACACCGGCGGTGAAATAGGTTTGCA
GCCCCAAGAGGTCGTAACCGGCACGAATCAGGCGGTTCAGGCCCGGTTCTTCCAAGCCCA
TTTCGGCGAGGAACTCGGCTTTTTCGTCGTCTTCCAATTCGGCAATTTCGCTCTCCATCG

## Appendix A

CGGCGCAAACGGCGACGACGGGGGCGTTTTCTTTTGCCGCCAATTCTTTCAGGCGGTCGA
GGTGCGGATTGTTTTCAAAACCGTCTTCGGCGACGTTGCCCACATACATCGCCGGTTTGG
CGGTCAGCAGGAACAGCGGTTTGAGCATCGCGCGTTCTTCCGCGTCCAAACCGAAGGAAC
GCACGGGTTTGCCTTCGTCCAGATGCGGCAGCAGTTTTTTGCACAAATCGACCAGCTTTT
GCGCGTCTTTGTCGCCTGAGCGGGCGCGTTTTTCTTCGCGGACGATGGCTTTTTCGACAC
TTGCCAGGTCGGCAAGTGCCAACTCTGTGCCGATGGTTTCAATGTCGGCAATCGGATCGA
CGCGGCCTGCAACGTGGACGATGTTGTCGTCGTCAAAGCAGCGCACGACATTCACAATCG
CATCGGTTTCGCGGATGTTGGCAAGGAACTGGTTGCCCAAGCCCTCGCCTTTGCTCGCGC
CTGCAACCAAACCGGCAATATCGACAAATTCGACGATGGCAGGCTGCATTTTTTGCGGAT
TGACGATTTTTGCCAATTCGGCCATACGCGGATCGGGGACTTCGACGATGCCGACGTTGG
GTTCGATGGTACAGAAAGGATAGTTTGCCGCTTCGATACCCGATTGGGTCAGCGCGTTAA
AAAGGGTGGATTTGCCGACGTTGGGCAAACCGACGATGCCGCATTTCAAACTCATGTTTT
TTCCTGAAAATAGAGAAATTTAACGGCGGATTATAGCATACCGCCGCCCGCGTTCCGAAA
AAATGCCGTCTGAAACGGCTTCAGACGGCATCCGGTTTCAGAAAACCGTTCAGAACAAGC
CGTGAATCACGCCTTCTGCGTCCACATCGATTTTCTCGGCAGCCGGAACTTTGGGCAGGC
CGGGCATTTTCATCATGTTGCCGCACAGGGCGACGATGAAACCTGCGCCTGCGGAAACGG
TGATGCCGCGCACGGCGATGCGGAAGTCTTCGGGGCAGCCCAACAGTTTGGCGTTGTCGC
TCAAAGAGTATTGGGTTTTCGCCATGCAGATCGGCATTTTGTCCAAGCCCAGTTTTTCCA
GTGAAGCGATTTCGGCAGACGCTTCCGCGCTGAAATCAACATCTTCCGCGCCGTACACTT
TTTGGGCAATCGCACGGATTTTGTCTTTGATGCCCAACTCGACATCGTAGGCGAAACCGA
AGTTATTGGTTTGACTTTCAATGGCGTTGACGACTTTGCGCGCCAAATCCGCGCCGCCCG
CACCACCTTTGCCCCACACTTCGGTCAGGGAAACTTCAACGCCGTGTTCGGCACAGGCTT
TTTCAATCATCGCCAACTCGGCATCGGCGTCGGACACGAAGCGGTTGAGCGCAACGACGA
CGGGCAGTCCGAATACGTTTTTCAGGTTGGAAATGTGTTTCAGCAGGTTGGGCAAACCTT
TTTCCAAAGCGTCTAAATTTTCTTCGCCGAGGTTGGCGCGTTCCACGCCGCCGTTATATT
TCAACGCGCGGACAGTCGCCACGACAACAGCCGCATCAGGTTTCAAACCGGCAAGGCGGC
ATTTGATGTCGCAGAATTTTTCCGCGCCCAAGTCCGCGCCGAAGCCTGCTTCGGTTACGG
CGTAATCGGCAAGGTGTTTCGCCAGACGGGTTGCGGTTACGGAGTTGCAGCCGTGGGCGA
TGTTGGCGAACGGGCCGCCGTGTACGAAGGCGGGCGTGCCTTCGATGGTTTGCACCAAGT
TGGGCTTAATCGCATCTTTAAGCAATGCCGCCATCGCGCCATTCGCTTTCAAATCTTTGG
CGTAAACGGGGCTGCCGTCTTTGGCGTAGGCGACAAGGATGTTGCCCAAACGCTCTTTCA
AATCGCTGATGTCTTTGGCAAGACAGAATACCGCCATCACTTCGGAAGCAACGGTAATAT
CGAAACCGTCAGGACGCATCACGCCGTCAACGGGTTTACCCATGCCGTCGATGATGTTGC
GCAACTGGCGGTCGTTCATATCGACCACGCGCCGCCACAGCACGCGTTTGGGGTCGATGT
TCAACTCGTTGCCTTGGTAGATATGGTTGTCGAGCATCGCGGCAAGCAGATTATTTGCCG
CACCGATGGCGTGAAAATCTCCGGTGAAGTGCAGGTTGATGTCTTCCATCGGCAAAACTT
GGGCATAGCCGCCGCCTGCCGCGCCGCCTTTCACGCCGAACACCGGCCCCAGAGAAGGTT
CGCGCAGGGCAATCACGGCATCTTTGCCGATGTGGCGCAACGCGTCCGCCAAACCGATGG
TTACGGTGGTTTTGCCTTCGCCCGCCGGAGTCGGGTTGATGGCGGTAACCAAAATCAGCC
TGCCCTGTTTTTGCGGCAGTTTGAACGCTTCGGCAGGATTGATTTTCGCCTTGTAATGAC
CGTAAGGCTCAATGTTGTCGGCATTCAGACCAAGCTTGGCGGCAATTTCGCCAATCGGGC
GCATGGTGGAGGATTGGGCGATTTCGGCATCGGTTTTGAAGCTCATGATTTTCCTTTAGA
AATGAGGAGGGACATGCCGTCTGAAAGCATCAGGCGACAAACAGGTGGATTGAAAATAAT
ATCAGGCATATTATAACGTTATCCGCACCAAACCCGCAGTGAAATTTTTGACGCAGCAAC
AAAAATACCGTTCATATTGTTCACAATCCAAGGAGAAAACATGGGCAGCAACGCATGGCT
GTTTTGGGCATTGGCATCGGCAGGCTTCGCCTCATTGACCGCTATTTTCGCCAAAATGGG
TTTACAGGGTATAGATTCCGATTTCGCCACCTTTATCCGCACCTTGGTCATCCTTGCCGC
TTTGTTATTGTTTTTAACCTACACCGGCAAATGGCAGGGTGTGAACGGCTTTACGGGGCG
CAAGTGGACATTCCTCATCCTATCCGGTCTTGCTACCGGCGCATCTTGGCTCGCCTATTT
TAAAGCCCTGCAACTGGGCAACGCCTCGCAAGTCGCCCCCATCGACAAATTCAGCCTGGT
CTTGGTCGCGCTGATGGCGGTGGTTTTCTTGGACGAACGCCCGAACACGCAGGAATGGAT
AGGCTTGGGGCTGGTAACGGCGGGCGTGTTGGTGCTGGCGTTGAAACGTTAAACCGAATC
CGCCATACCGTCTGAAACCGGGTTTTTACTTCCAAGCCCCTGCAAGGGCTTGAGCCTCTT
TCAGACGGCATACCGTGCCGACATCCAGCCACAAGCCCGTATGCTTCTGACCGCTCACGC
GGTTTTGCCGCATTTCGCCACGCAATACGGGCGCGAGTTTCGCCACACTGCCCGCTTCGA
TTCCGTCAAACATTTCAGGACGGTAAATACCCACGCCGCTGAATGTCAATCCGTTGCCGC
CATTTACTTCCGGCCGCACGCTGCTGTCGGGCAGCAGGGAAAAATCGCCGTCGGGGTTGT
GCGGCGGATTTTCCACCAGCCACAGATGGGCGGAAATATGTTCCGGCAGGGACGATGCCG
TCTGAAACGCGGCGGTAAAATCGATGTCGGTCAGCACGTCGCCGTTGACCACCAAAAACG
GCTGCCCACCCAACAGCGGCAATGCCTGCGCGATGCCGCCTGCCGTTTCCAAACCGCCTG
CGGGTTCGGGCGAATAGGCGATGTTCACGCCATAAGCCGAGCCGTCGCCCAAAGCATCTT
CTATCTGCCGACCCAGCCAAGCGTGGTTGATGACGATTTCGGTAAACCCCGCCTGCTTCA
GACGGCATAGGTGCCAACCGATTAGAGGCTTACCCGCCACATCGAGCAGCGGCTTCGGAG
TGGTATCGGTCAAAGGGCGCATACGCTCGCCGCGTCCTGCCGCCAGTATCATCGCTTTCA
TATATCTGTCCGAATATCAGTCTAAAAATCTAAACTGCCGTCTGAAATACAGCAGCGCGG
GGCGTTTGCACCCGCAGTTTTTGATTTCGTCGAGCCTGACGTAAAACACAAAATGCGTGC
CGATTTCATGTTTGCCGACAATATGCCCGTGCAGGTGCGCCAACGCGCCCTCTATTTCAA
GTTGTCCCGTTTTGCCGCGATGCCAGATGTGGTAGGCAAACCGCTCTTCGGGCGACAGGC
CGGTCAGCCCGGCAAAATGTTCGGCAACATCCTGATGTTCGTCCGCCAGCGTATTGATGC
AGAGGCTGCCGTTTTCCGACAGGATCGGAATGATTCGCGCACTCCGGTTGATGCACAGCA
TCACGGTCGGCGGCTCGTCGGTAACCGGCGCGACCGCCGTCATTGTAATGCCGTAACGCC
CTGCCGCACCGTCTGTCGTGATGACATGAACGCCTGCCGCGCAAGATGCCATCGCATCAC
GGAACGAAGTTTGAAAATTTTTCTGCAAATCCGCCATTTTTCCCCTTTAAACTGTCCCCT
ATATAAGAATGCTGCACACAAGGCATCCCCCATGTGCAGCAGTTTTGATTCAAAAAGCCG
TCGGTCGGACGTTTCCGCGCGTTACGGCGTATTACGAGTTCAACGCATCCTCGATTTTGG

## Appendix A

CAAGTTCTGCCAACAGGTCTTTAAGCAGCAGCATTTTCTCGCGGCCCAGCACTTCCTCGA
TAGCGTCGTAGCGTTCGTCCACTTCTTCGCCGATTTCCTCATACAGCTTCTCGCCCTCGG
CAGTCAGCTTCAGAAAAACACGTCGTTGGTCGTTGGAAGGTTTCAGGCGGACAACCAAAC
CCGCTTTTTCAAGGCGGGTCAGGATACCGGTCAGGCTGGGGCGCAAAATGCACGCCTGAT
TCGCCAAATCTTGAAAGTCCAGCGTGCCGTTTTCCGCCAAAAGACGGATAATCCGCCATT
GCTGATCGGTAATATTCGCCTGATTCAGAATAGGCCTGAATTGGGTCATCAGGGCTTCCC
TTGCCTGTATCAGACCGATATTGATAGACGCATGTTTTGATTGGGTAGGCATTGTTTAAG
TCTCCAAGTTATCGAAAATCAAACTTTCAAACCGTCGGGAAAGCCTGTGGGCGTAAATTT
TGATGCAACCGTTATATAACAAAACGAACATATAGCAACAATACGCTATAAACCGCATCG
GACGACTGGGTATAAAAGACTTTAATTCCGATAATCCTATCTAAAAATATTTTAATAGTT
ATATCTTAATCTATTTTTCCCACAATCACAACAAGGGATTACATCGGCAGGCGCGTCGGC
TCTTTCCCAAAAAACAAAAGCCGCCGCATCCGCCGCGCAAGGCATATGCCGCTTGATTCT
CTACATAGCGGAAAATTTAATAAAAACAAAAGTTAACCGAAAACATCCGCCTGAAAAATT
CGTGCGCGCAAGCCCCAATAACTGCTGATTCCCGTCGTATAGTGAACCATTTTCCCATTT
TTGACCAAAACGACGGCAGGCGTTGCGACAATCCGCCAAGACCTTGCCAAACCCCCGTCC
TCATCGTTGACAGTCGGAAAGCCCAAGCCGCGTTTTGCCATATACGCCGCCACTTCCGCC
GAACTGCCGGAACGTACCGCCACGCCGACGACCGGCACGCCGTCCGCCGCCAAATCATCG
ATTATCGGCGACTGATAACGGCACACGCCGCACCAGCTCCCCCAAAAATACACCAAAACC
GCCTTATCTCGGCTAAACTGTCCCAAAGTCAGCCGCTGCCCCGACAGCAGGGTCAAAGGC
CGCCCTGCCGCACCGGCCGGCTCTTCGGGCTTGCGTATCCAATCCAAAAACAGCGACACC
AATAAAAACACCAATGCCGTCTGAACGGCAAATTTGATGCCCGAAAGCAGTTTCTTTTTC
ATACGCTCTCTCAAACGGTACGCCCGCGCAACCGGCAGGCAAACAAAAAGCCAAGTCTCA
AAACTTGGCTTCCGGTTATCTGGTGGGTCGTGAGCGATTCGAACGCTCGACCAACGGATT
AAAAGTCCGCTGCTCTACCGACTGAGCTAACGACCCGATAAGCCGTGCATTATACAGCAC
CATCCTACCTCGTCAAGCAAATTTTACAGGCTTAATTGCAGACCACTGTTTGCACGGGAT
ATTTTGACAACGGATTTTCACAATCCGCCGCATACCGTGTAAAAGTTCGCACAAGGAAAA
GCAAACCGCCCGAAATCAATGTACACTTTCCGCCCGTTTCCCTTCCCAACCTGCACACAG
AAACACACATTATGAACATACAAAACATCCGCACCCTCCTCGACACCGTCGCCGTTCCGA
ATACGGCACGCACGCTCGGCGGCGAAAAGGCCGTCCGTTCGGTCGAACAGCGTTCAGACG
GCATCCATATCGCCCTGCATTTCGGCTTCCCCGTCGCGCACATTGCCTCAGAAACAGCCG
ACCGCATACAGGAAATCCTGATGCCCGAAACAGGCGACACACACATCCATCTGTCCATGG
ACACTGAAATCGGCACACACAAAGTCCAGCCCGGCGTTACCACCATCAAAGGCGTGAAAA
ACATCATCGCCGTCGCATCGGGAAAAGGCGGCGTGGGCAAATCGACAACCACCGCCAACC
TTGCCGCCGCAATGGCGCGCATGGGCGCGCGCGTCGGCGTGCTCGATGCCGACCTTTACG
GCCCGAGCCAACCGACCATGTTGGGTGTGGACGACCGCAAACCCGATCAGAAAAACCAAA
AACTCATTCCCGTCGAATCTTCAGACGGCATACAGGTCATGTCTATCGGCTTTCTCGTCG
ATACCGACCAAGCCGTCGTCTGGCGCGGGCCGATGGTCAGCCAAGCCTTGCAGCAGCTGA
TGTTCCAAAGCGAGTGGGACGAAGTGGACTACCTGTTTATCGACCTGCCCCCCGGCACGG
GCGACATCCAGCTCACGCTGTCCCAGCGCATCCCCGTAACCGGTTCCGTCATCGTAACCA
CGCCGCAGGACATCGCCCTGATAGACGCGCGCAAAGCCGTGGATATGTTCCGCAAAGTCA
ACATTCCCATTTTGGGCGTATTGGAAAATATGTCCGTCCACATCTGCACCAACTGCGGAC
ACAGCGAAGCACTGTTCGGCACGGACGGCGGCAAAGATTTCGCCGCACGCCTCAACGTCC
CCCTGCTCGGACAGCTTCCCCTAAGCCTGCCCGTGCGCGAAGCCATGGACGGCGGCACAC
CGGCGCAACTGTTCGACGAACACCCCGCCATCGCCCGAATCTACACCGATGCCGCATTCC
AAATCGCCCTGAGCATTGCCGACAAAGGCAAAGACTTCAGCAGCCGCTTCCCCAAAATCG
TCGTCGAATAAAGCCGCGTCCGAAACCGCAACAGCAATGCCGTCCCAAGCCCCGCGCCTG
CCGGCGGGCAAACTTGCCGGATAAAACGGTTTTTTTGAGATTTTACGTTCCGGATTCCCG
CCTGCGCGGGAATGACGAATTTTAGGTTTCTGATTTTGGTTTTCTGTTTTGTAGGAATGA
TGAAATTTTGAGTTTTAGGAATTTATTGGAAAAAACAGAAACCGCTCCGCCGTCATTCCC
GCGCAGGCGGGAATCTAGACCTTAGAACAACAGCAATATTCAAAGGTTAGCTGAAGCTTT
AGAGATTCTAGATTCCCACTTTCGTGGGAATGACGGGATGTAGGTTCGTGGGAATGACGC
GGTGCAGGTTTCCGTGCGGATGGATTCGTCATTCCCGCGTAGGCGGGAATCTAGACCATT
GGACAGCGGCAATATTCAAAGATTATCTGAAAGTCCGAGATTCTAGATTCCCACTTTCGT
GGGAATGACGGGATGTAGGTTCGTGGGAATGACGCGGTGCAGGTTTCCGTGCGGATGGAT
TCGTCATTCCCGCGCAGGCGGGAATCTAGACCTTAGAACAACAGCAATATTCAAAGGTTA
GCTGAAGCTTTAGAGATTCTGGATTCCCACTTTCGTGGGAATGACGGGATTTGAGATTGC
GGCATTTATCGGAAAAAACAGCAACCGCTCCGCCGTCATTCCCGCGCAGGCGGGAATCCA
GACCTTGGGATAACAGTAATATTCAAAGATTATAAAAGACCCGTCATTCCCGCGCAGGCG
GGAATCCAGACCTTAGAACAACAGTAATATTCAAAGATTATAAAAGACTCGTCATTCCCG
CGCAGGCGGGAATCCAGACTGTCGGGCATCTGCAGCGGTTTGCTAAAAAACGCTTTACCG
TGATCAGTGTGCAAAGTTAAAATGGGGAGGTAAGCTTTTCAATCAGCAATCCGGCGGGCG
CGGGATCGGGCGGTTTACCGAACCCCGGTGTTCGCGGCGCGCCTGCCGCCGACGGTATCC
CGCGAAGCAAGATTTAAGGGATAAAATATGTTCCAACACGCAGGGCGGCACATAAGGCGC
CGCCCTGATTCGGAAGGGCTTGCACCCCTCCCGGACAAAGCCTGATCCTGCCGCCCCGAA
GGACGGATGCCCGAAGGGCGGGGGGTTTGACCGAAAAGGAAATACGATGAATAAAACTTT
AAAAAGGCGGGTTTTCCGCCATACCGCGCTTTATGCCGCCATCTTGATGTTTTCCCATAC
CGGCGGGGGGGGGGGGGCGATGGCGCAAACCCATAAATACGCTATTATCATGAACGAGC
AAAACCAGCCCAAGGTAAAGGGGAATGGCAATATTCAACAATAAAGGACAAAGACAGGG
AACGCAAATTTATCTATAATAAAAGCGGCCGGGGTGGAGGCTCTGTCTTTTTCGACAATA
CCGATACCCTTGTTTCCCGACAAAGCGGTACTGCCGTTTTTGGCACAGCCACCTACCTGC
CGcCCTACGGCAAGGTTTCCGGTTTTGATGCCGACGGGCTGAAAGAGCGCGGCAATGCCG
TTAATTGGATTCATACGACCCACCCAGGGTTGATAGGCTACAGCTACACCAGTGTCGTAT
GCAGAGACAGCACAGGCTGTCCCAAACTTGTCTATAAAACCCGATTTTCCTTCGACAACA
CCGGTTTGGCAAAAAATGCGGGCAGCCTGGATAGGCACCCGGACCCAAGCCGCGAAAATT
CGCCCATTTACAAATTGAAGGATCATCCATGGTTGGGCGTGTCTTTCAATTTGGGCAGCG

## Appendix A

AGAATACCGTCAAAAATGGCAACTCATTCAACAAATTGATATCTTCTTTTAGTGAAGACA
ATAATAATCAAACCATCGTCTCTACGACAGAAGGCTCCCCTATTTCCCTTGGCGACCAGC
AGCGCGAACATACCGCCGTGGTCTATTATCTGAACGCCAAACTGCACCTGCTGGACAAAA
AAGGGATTAAAGATATCACCGGCAAAACAGTGCGGTTGGGTGTCTTGAAGCCGAGCATCG
ATGTGAAGACACAAAATACGGGGCTTGGCGGCATTCTAGCTTATTGGGCTAGGTGGGACA
TTAAAGATACCGGGCAGATTCCAGTCAAGCTCGGCCTGCAGCAAGTCAAAGCAGGCCGCT
GCATCAATAAACCGAACCCCAATCCCAACAAAAAAGACCTTTCGCCGGCCCTGACTGCCC
CCGCGCTGTGGTTCGGACCTGTGAAAGATGGTAAGGCGGAGATGTATTCCGCTTCGGTTT
CTACCTACCCCGACAGTTCGAGCAGCCAAATTTTCCTGCAAAACCTTTCCCGCAAGGATG
ACACAAGCAAACCGGGCCGCTATTCCCTCAAACCCTTGAGTACGTCGGAGATTAAAAGTA
AAGAGCCGAGTTTCACGGGGCGGCAAACCGTCATCCGATTGGATGGCGGCGTACGGCATA
TCCAACTGGATAGAAACAATGAGGCCACCGGTTTAAATGGAAATGACGGCAAAAACGACA
CTTTCGGCATTATTAGAGAAGGGAGCTTCATGCCTGATGCCAGCGAGTGGAAAAAAGTAT
TGCTGCCTTGGACGGTTCGGGGTTTTGCTGATGACAGTAAATTTAAAGCATTCAACAAAG
AAGAAAACAACGACAACAAGCCAAAATACAGCCAAAGATACCGCATCCGCGAAAACGGCA
AGCGCGATTTGGGCGACATCGTCAACAGCCCGATTGTCGCGGTCGGCGAGTATTTGGCTA
CTTCCGCCAACGACGGGATGGTGCATATCTTCAAAAAAGGCAACGGGGACGCGCGCGACT
ATAGTCTGAAGCTCAGTTATATCCCGGGCACGATGCCGCGCAAGGATATTCAAAACACCG
AATCCACCCTTGCCAAAGAGCTGCGCACCTTTGCCGAAAAAGGCTATGTGGGCGACCGCT
ATGGCGTGGACGGCGGCTTTGTCTTGCGCCGCATTACAGATGACCAAGACAAGCAAAAAC
ACTTCTTTATGTTCGGCGCAATGGGCTTTGGCGGCAGAGGCGCATACGCCTTGGATTTAA
GCAAAATCGACAACAGCAACCCGGCCGGCGTTTCCATGTTTGATGTCAAAAACGACAATG
GCGTGAAATTAGGCTACACCGTCGGTACGCCGCAAATCGGCAAAACCCACAACGGCAAAT
ACGCCGCCTTCCTCGCCTCCGGTTATGCGACTAAAGACATTAACAACGGCGAGAATAAAA
CCGCGCTGTATGTGTATGATTTGGAAAACAACAACGGTACGCCGATTGCAACAATCAACG
TACCCGACGGCAAGGGCGGGCTTTCGTCCCCCACGTTGGTGGATAAAGATTTGGACGGCA
CGGTCGATATCGCCTATGCCGGCGACCGCGGCGGGAATATGTACCGCTTTGATTTGAGCA
ACAACGATCCGACCAAATGGTCTGTACGTACTATTTTTAAAGGCACGCTGGATAAGCCGA
TTACCTCCGCGCCCGCCGTTTCCAAACTGAAAGACAAACGCGTGGTTATCTTCGGTACGG
GCAGTGATTTGAGTGAGGATGATGTTGATAAAAAGGATATACAATCTATTTACGGTATTT
TTGACAATGACACAGGCACGGATGTGGCAGAAGAAGGACAGGGCAAAGGGTTGCTCGAGC
AACACCTTACTCAGGAAGATAAAACCTTATTCCTGACCGATTACAAGCGATCCGACGGCT
CGGGCGACAAGGGCTGGGTAGTGAAATTGGAAGCCGGACAGCGCGTTACCGTCAAACCGA
CCGTGGTATTGCGTACCGCCTTTGTAACCATCCGCAAATATAACGACGGCGGCTGCGGCG
CGGAAACCGCCATTTTGGGCATCAATACTGCCGACGGCGGCAAGCTGACCAAGAAAAGCG
CGCGCCCGATTGTGCCGGAAGCCAATACGGCTGTCGCGCAATATTCCGGTCATAAGCAAA
CCGCCAAAGGCAAATCCATCCCTATAGGTTGTATGTGGAAAAACAATGAAACCGTCTGCC
CGAACGGATATGTTTACGACAAACCGGTTAATGTGCGTTATCTGGATGAAAAGAAAACAG
ACGGATTTTCAACAACGGCAGACGGCGATGCGGGCGGCAGCGGAACATTCAAAGAGGGTA
AAAAACCCGCCCGCAATAACCGGTGCTTCTCCGGAAAAGGTGTGCGCACCCTGCTGATGA
ACGATTTGGACAGCTTGGATATTACCGGCCCGATGTGCGGTATGAAACGAATCAGCTGGC
GTGAAGTCTTCTTCTGATTTGCACGCGAAAATGCCGTCCGAAAGGTTTTCGGACGGCATT
TTTTGCGTTTTTCGGGAGGGGCGGGTTCGTAAAAGGCGGGCTATAGGGTAGGCTTCATCT
CGCCAATCTCACTGAATCCATCAATTTCCACAATTCAATTAAATACCGTCAAACCGATGC
CGTCATTCCCGCGCAGGCGGGAATCTAGACATTCAATGCTAAGGCAATTTATCGGGAATG
ACTGAAACTCAAGAAACTGGATTCCCACTTTCGTGGGAATGACGGGATGCAGGTTCGTGG
GAATGACGTGGTGCAGGTTCGTAGGAATGACGTGGTGCAGGTTTCCGTGCGGATGGATTC
GTCATTCCCGCGCAGGCGGGAATCCAGACATTCAATGCTAAGGCAATTTATCGGGAATGA
CTGAAACTCAAAAAACTGGATTCCCACTTTCGTGGGAATGACGGGATTAGAGTTTCAAAA
TTTATTCTAAATAGCTGAAACTCAACGCACTGGATTCCCGCCTGCGCGGGAATGACGAAG
TGGAAGTTACCCGAAACTTAAAACAAGTGAAACCGAACGAACCGGATTCCCACTTTCGTG
GGAATGATGGGATTAGAGTTTCAAAATTTATTCTAAATAGCTGAAACCCAACGCACTGGA
TTCCCGCCTGCGCGGGAATGACGAATTTTAGGTTTCTGATTTTGGTTTTCTGTTTTTGTA
GGAATGATGAAATTTTGAGTTTTAGGAATTTATCGGAAAAAACAGAAACCGCTCCGCCGT
CATTCCCGCGCAGGCGGGAATCTAGGACGTAAAATCTCAAGAAACCGTTGTACCCGATAA
GTTTCTGCGCCGACAAACCTAGATTCCCGCCTGCGCGGGAATGACGGTTCAGTTGCGTAG
GACTGGATTGTGAAAAGGGGCGGATTCGGTGAAAACGGCGGAAATGTGGGATTGATGGAA
TCGGTGGGCTGAAGCCCTCCCTACAGAGCTTTCAGACGGTATTGTTTGCGTTTTCGGGAT
GGGGGCAAATGAAACACCGACAAACCGATACCGTCATTCCCGCGCAGGCGGGAATCTAGA
CATTCAATGCTAAGGCAATTTATCGGAAATGACTGAAACTCAAAAAACTGGATTCCCACT
TTCGTGGGAATGACGATTCGGACATTCCTTAAACTACCCGTGTATCGCTGTAAATCTTAG
AGATGGAGGAATAAAGACCGTTGGGCATCTGCAGCCGTCATTCCCGCGCAGGCGGGAATC
TAGGATGCGGAATCTCAAGAAACCGTTATACCCGATAAGTTTCTGCACCGACAGGTCTGG
ATTCCCGCCTGCGCGGGAATGACGATTCGGGTATTTCTGACGGTTCGGGCATTCCCGACA
AGGTGGATTTTCAAGGTGTTGTATAGGGTGTAGGAGGATTCGTAAAAGGTGAGTTATAGG
GTGGGCTTCAGCCCACCGATTCCAACGATTCCACCAATCCTACACCGTTCCCATAGACTC
AAATCAACACAGAAACTTATGCGCCGTCATTCCCGCGCAGGCGGGAATCTAGGATGCGGA
ATCTCAAGAAACCGTTATACCCGATAAGTTTCTGCACCGACAGGTCTGGATTCCCGCCTG
CGCGGGAATGATGGTTCGGGTATTCCTGACGATTCGGGTATTCCTGACGATTCGGGTATT
CCTGACGATTCGGGTATTCCTGACGATTCAGGTATTCCTGACGATTCAGGTATTCCTGAC
GATTCAGGTATTCCTGACGATTCAGGTATTCCTGACGATTCAGGTATTCCTGACGATTCA
GGTATTCCTGACGATTCAGGTATTCCTGACGATTCAGGTATTCCTGACGATTCAGGTATT
CCTGACGATTCAGGTATTCCTGACGATTCAGGTATTCCTGACGATTCGGGTATTCCCATA
GTTTCGCCGGGCGGACGTGGGGAAATGCGTAACGGGCATAGTGGGCGCGGAGCGGGCGGT
TTTATGCCCCGGATTTCCGTTTTCGCGCGAACATATCAGCCCGCCTGCCGCGTTTGCGCT

## Appendix A

TGAAATCGGGTATGTTTCGTCTTAAAATATGCTGCTTTCAGGGTATAGGCACTTGCCCGA
AAAGCACGTTACGCGTCTATCTTGCGCGGCGTGTTTTTTTTGACCGGATTTTTCCGACC
GGATGCCCCCTGCCGAAGTCCCTTCAGACGGCATTGTCAAGAATTTTATTAAAAACAGGA
TTCCCATCATGAGCACCCCCGCCCTCCTCGTCCTCGCTGACGGCAGCGTATTTCACGGCA
CATCAATCGGTTACGAAGGTTCGACTTCCGGCGAAGTCGTGTTCAATACTTCGATGACCG
GCTATCAGGAAATCCTGACCGACCCGTCCTACTGCAAACAAATCGTTACCCTCACCTACC
CACACATCGGCAACACCGGCACCAACGCCGAAGATGAAGAAAGCCGCAGCGTTTATGCCG
CCGGCCTGATTATCCGCGACCTGCCGCTCTTGCACAGCAACTTCCGCGCCTCCGAAAGCC
TGCACGACTATCTGGTACGCAACAAAACCGTCGCCATCGCCGACATCGACACCCGCCGCC
TGACCACGCTGTTGCGCGAAAAAGGCGCGCAAGGCGGTGCGATTCTGACCGGTGCGGATG
CCACAATCGAAAAAGCGCAAGAACTCATCGCCGCGTTCGGCAGCATGGTCGGAAAAGATT
TGGCAAAAGAAGTTTCCTGCACGGAAACTTACGAATGGACGGAAGGCGAATGGGCATTGG
GCAAGGGTTTCGTTACCCCTGACGAACAGCCTTACCACGTCGTCGCCTACGATTTCGGCG
TGAAAACCAACATCCTGCGTATGCTCGCCTCGCGCGGCTGCCGCCTGACCGTCGTCCCCG
CCCAAACGAGCGCGGAAGACGTGTTGGCACTCAACCCTGACGGCGTATTCCTATCCAACG
GCCCCGGCGACCCCGAGCCTTGCACCTACGCCATCAAAGCCGTACAAAAACTGATAGAAA
GCGGCAAACCGATTTTTGGCATTTGCTTGGGACACCAGCTCATCAGCCTCGCCATCGGCG
CGAAAACCCTGAAAATGCGCTTCAGCCACCACGGTGCGAACCACCCTGTGCAAGATTTGG
ACAGCGGCAAAGTCGTCATCACCAGCCAAAACCACGGTTTTGCCGTTGATGCCGACACCC
TGCCCGCTAACGCACGCATTACCCACAAATCCTTGTTTGACAACACTTTGCAAGGCATCG
AGCTGACCGACAAACCTGTGTTCTGCTTCCAAGGCCACCCCGAAGCCAGCCCCGGTCCGC
AAGATGTCGGCTATTTGTTTGACAAATTCATTGGCAATATGAAAGCGGCAAAACGGGCAT
AATGGTTTTCAGACGGCAACAGTATGCTGCTGCCGTCTGAAAAACAAAGCTGGAAATGAA
GATTAGCGCACTCGACCATCTAGTACTAACTGTTGCCGACATTGACCGAACCATCGCGTT
TTATAGTGAATTAAATTTAAACCGGTACAGCGTTGGCTCGCCTTGCCGTACTATTTGTAC
TGTCTGCGGCTCGCCGCCTTGTCCTGATTTTTGTTAATTCACTATACACACAAGTTTTGG
GCATGGAAGAAGTTTCATTTGGCAGCGACCGTAAAGCTTTGTTGTTTGGCAGTCAGAAAA
TCAACCTACACGGGCGCGGTGCGGAAATTCAGCCTAACGCGCAACACGCCGCCTGCGGCA
CAGCGGATTTATGCCTGCTGACCGATACGCCACTGGAAACGGTTTTACAGGAATTATCCG
CACACGGCATCAAACCTTTAAGCGGCATCGTAGCGCGCACAGGCGCAATGGGCAAAATCC
AATCGGTTTACCTGCGCGATCCCGATGGCAACCTGCTGGAAATCAGCAGTTATTGATTTT
CAGACGGCTTATGCAAAATAAAAAACAGCCTGCACAAGCTGTTTTCCTTGCAGCCTCTTT
AACCCCAACAGCCGCCCCGTCCTCTCTCCCTGTGGGAAAGCGTTAGAGAGAGGGCAACAA
GCCGCAAGGCTTGTGTTTGGGCGGTTAGGGTGTTGGGGAAGGTTGCCGAAATTCGGGGAA
TGCCCTCTCCCCGGCCCTCCCCCACGGGGGAGGGAGAAGGTTGCAGCAGATTTTGCGGTT
GCAGGCGGTTTGAAAGGCAACTTAGATTTGCAGCTGTTGTTTCAGGTCATCTGAAAAATA
AAAAGCAGCCTGCACAACCTGTTTTCCTTGCAAAACCCTTAATCCCAACCGCCACCACGT
CCTCTCTCCCATGGGAGAGAGTCAGAGAGAGGGCAACAAACTGTAAGGCTTACACAAACA
GTAACCCGACAACAGAATGAGCACGCACGAGAAACTTTTAACCGCCGACAACCCCGTCCT
GCATCAACGCGCCAAAGCCATGCGCCAAGAAATGAGCGAGGCGGAAGCAAAATTGTGGCA
GCACCTGCGGGCAGGCCGTCTGAACGGCTATAAATTCCGCCGCCAGCAGCCGATGGGGAA
TTATATTGTTGATTTTATGTGCGTAACGCCCAAGCTGATTGTCGAAGCAGACGGCGGGCA
GCACGCGGAACAAGCCGTATACGACCACGCGCGGACGGCATATCTCAACAGCCTGGGCTT
TACCGTGCTGCGTTTTTGGAATCACGAAATTTTGCAGCAGACAAACGATGTACTGGCGGA
AATCCTGCGCGTATTGCAGGAATTGGAAAAGCAGTATGCGCAATAACAAACGGTTAATTT
TGATTAGAGTTTTGAAAATTATAGGATACAGGTAGGGTACAGGCTGCTTGAATTGAGCGT
TTAGAAGACCGTCTGAAAAACAAAAAACAGCCCGCACAACCTGTTTTTCCTGCAGAACCC
TTAATTCCAACAGCCGCCCCGTCCTCTCTCCCTGTGGGAAAGCGTTAGAGAGAGGGCAAC
AAGCCGCAAGGCTTGTATTTAGGCGGTGAAGGCATTGGGGAAGGTTGCCGAAATTCGGAG
AATTCCATCTCCCCAGCCCTCCCCCACGGGGGAGGGGGCAGGTTGCAGCGGATTTTGCGG
TTGCAGGCGGTTTGAGAAAGAATGCCCGAAATATCAACAGCGGGAATTTTTCAGGCAGCC
TTTATCGCAAGGCAGGTGGAACAAACGCCGCGAACGTTTTTTCAGACGACCTTTGAACTC
ATCGGCAGAGAGTGTGCCGCAAGGCACGCACGCGGTGGGTTGGGGTTGCAGGGAAAATGG
AGAACGCGTGCATACGTACCGCACATACCCTACATACGGGCTACGGCTTGCTACGATACG
GGGGTTTCGATATACAAGTTAGGTTTTAGCAAACCCAACATTTTAGACAATTAAGCGGTT
TGTGTTGGGTTTTCAACCCAACCTACGCTTGCTACGTTTATTGCAACATATTCGCAGGAG
TTTAAATATGTCAATACCTATTAATTTCAATAATTTAAAGTATTTGCTTAATGATATGAG
AAACAAAAATAGAATAATTGAAGCATTTCCTTTTAATTATAATCAAAGGCAATACGCCGT
TATTTTGACTAGGTATAAACCTGATGAACCTAGACCAGATGATTATGCACAAGCAAAATT
AGAGTTTTTTAATTTGAATAGTGAAAATTCAATATTTGCGTATGCTGATTTTTATGAAGT
TCATTTTAAAAGTGCTACTGATTTTATTAATTTTTTAAAATTAATGTTCAGGCTGGTGC
TGCGAAAATCAGAGAAATTTTTCAGAGTTTAGTAATCTTTTTGCAGATTTCATTCCAAC
ACAAACTAAAAAAGATTTAGACATAATTTATAAAAAGATTGTAGCTACTCGTTTAGAACC
TAATTCTCCTAACACTATTTATTGCTATGATGTCCGTAGAAATGGGAAAGATAAGGCTGG
CAAGCCTAATCGCAGGAGCGTGGAAAATAGTGAAAAAGCAAAAATTTTGCGCCCAGAGCT
ATACGAAAAATTTAAAGCCGATAGTAATTACAGTTTTTTCTTTTCAGATAATCCAAGCGA
TGAAAAAACAGATGCAGAAATAATTAGAGAAGTTACCAATCGTCAATAATCCAAATTCTT
CAAAAGAAAGACCCACCATGCCCAAACGTACCGACCTAAAATCCATCCTTATCATCGGCG
CCGGCCCTATCGTTATCGGTCAGGCCTGCGAATTTGACTATTCGGGCGCACAGGCCTGCA
AAGCCTTGCGTGAAGAAGGCTATAAAGTCATTTTGGTGAATTCCAACCCCGCCACGATTA
TGACCGACCCCGAAATGGCGGATGTTACCTACATCGAGCCGATTATGTGGCAGACGGTGG
AAAAAATTATTGCCAAAGAGCGTCCTGACGCGATTCTGCCTACCATGGGTGGTCAGACTG
CGCTGAACTGTGCGCTGGATTTGGCGCGCAACGGCGTGCTGGCGAAATACAATGTCGAGC
TGATCGGCGCGACCGAAGACGCCATCGACAAAGCAGAAGACCGTGGCCGCTTTAAGGAGG
CGATGGAGAAAATCGGCCTCTCCTGCCCGAAATCTTTTGTCTGCCACACGATGAACGAAG

## Appendix A

CTTTGGCGGCGCAAGAACAGGTCGGCTTCCCTACCCTGATTCGTCCTTCTTTCACCATGG
GCGGTTCGGGCGGCGGCATTGCCTACAATAAAGACGAGTTTTTGGCGATTTGCGAACGCG
GTTTCGATGCGTCGCCCACGCACGAGCTGTTGATTGAGCAGTCCGTTCTCGGCTGGAAAG
AGTACGAGATGGAAGTGGTGCGCGATAAGAACGACAACTGCATCATCATCTGCTCGATTG
AAAACTTCGACCCGATGGGCGTGCATACAGGCGACTCGATTACGGTTGCGCCGGCGCAAA
CGCTGACGGACAAGGAATATCAAATTATGCGTAATGCTTCGCTGGCGGTATTGCGCGAAA
TCGGCGTGGACACGGGCGGCTCGAACGTGCAGTTTGCGGTGAACCCTGCAAACGGCGAGA
TGATTGTGATTGAGATGAACCCGCGCGTGAGCCGTTCTTCCGCGTTGGCTTCCAAAGCAA
CGGGTTTCCCGATTGCGAAGGTGGCGGCGAAGCTGGCGGTCGGCTTTACGCTGGACGAGT
TGCGCAACGACATCACCGGCGGCAAAACCCCCGCGTCGTTCGAGCCTTCCATCGACTATG
TGGTTACCAAAATCCCGCGTTTCGCGTTTGAAAAATTCCCTGCCGCAGACGACCGCCTGA
CCACGCAGATGAAATCGGTGGGCGAAGTGATGGCGATGGGCCGCACGATTCAAGAAAGTT
TCCAAAAAGCCCTGCGCGGCTTGGAAACAGGCTTGTGCGGCTTCAATCCGCGCAGTGAAG
ACAAAGCGGAAATCCGCCGCGAACTGGCGAACCCCGGCCCCGAACGTATGCTGTTTGTGG
CAGACGCGTTCCGCGCGGGCTTCACGCTGGAAGAAATCCACGAAATCTGCGCCATCGACC
CTTGGTTCTTGGCGCAAATCGAAGACTTGATGAAGGAAGAAAAAGCGGTTTCAGACGGCA
TTTTGAGTGATTTGGATTTCGCCGCCCTACGTCGTCTGAAACGCAAAGGCTTCTCCGACA
AACGTTTGGCACAATTGTTGAACGTAAGCGAAAAAGAAGTTCGCGAACACCGCTACGCGC
TGAAGCTGCATCCGGTTTACAAACGCGTCGATACCTGCGCCGCCGAGTTCGCCACCGAAA
CCGCCTATCTTTACTCCACTTACGAAGAAGAATGCGAATCTCGTCCTTCCGACCGCAAAA
AAGTGATGATTCTCGGTGGCGGCCCGAACCGCATCGGTCAGGGCATCGAGTTTGACTACT
GCTGCGTTCACGCCGCGCTCGCCCTGCGCGAATCGGGCTTTGAAACCATCATGGTCAACT
GCAACCCCGAAACTGTGTCCACCGACTTCGACACCAGCGACCGCCTGTATTTCGAGCCGC
TGACGCTGGAAGACGTGTTGGAAATCGTCCGCACCGAAAACCCGTGGGGCGTGATTGTGC
ATTACGGCGGCCAAACCCCGCTCAAACTCGCCAACGCGCTGGTTGAAAACGGCGTGAACA
TCATCGGCACGTCCGCCGACAGCATCGACGCCGCCGAAGACCGCGAACGCTTCCAAAAAG
TGTTGAACGACTTAGGCCTGCGCCAACCGCCCAACCGCATCGCCCACAACGAAGAAGAAG
CGCTCGTCAAAGCCGAAGAAATCGGCTATCCGCTGGTCGTGCGCCCGTCTTACGTCCTCG
GCGGCCGCGCCATGCAGGTCGTCCATTCCGCCGAAGAGCTGCAAAAATACATGCGCGAAG
CCGTGCAGGTTTCCGAAGACAGCCCCGTGTTGCTCGACTTCTTCCTGAACAACGCGATTG
AAGTGGATGTGGACTGCGTTTCAGACGGCAAAGACGTGGTTATCGGCGGCATCATGCAGC
ACGTCGAACAGGCGGGCATCCACTCCGGCGACTCCGGCTGCTCGCTGCCGCCCTACTCCT
TAAGCGAAGAAATCCAAGACGAAATCCGCCGCCAAACCAAAGCGATGGCGTACGCGCTGG
GCGTGGTCGGACTGATGAACGTGCAGTTTGCCGTACAAGACGGCGTAGTGTTCGTATTGG
AAGTGAACCCGCGCGCCAGCCGCACCGTGCCCTTCGTCTCCAAAGCCACCGGCGTGCCGC
TCGCCAAAGTCGGCGCGCGCTGCATGGCAGGCATTTCCCTGAAAGAACAAGGCGTGGAAA
AAGAAGTTGTCCCCGATTTCTATGCCGTTAAAGAAGCCGTGTTCCCATTCATCAAATTCC
CGGGCGTGGATACGATTTTGGGACCGGAAATGCGCTCCACCGGCGAAGTCATGGGCGTGG
GCGCAAGCTTTGGCGAAGCCTACTACAAAGCCCAACTCGGCGCGGGCGAACGCCTCAACC
CGACCGGCAAAATCTTCCTCTCCGTGCGCGAAGAAGACAAAGAACGCGTCATTAAAACCG
CTAAAAACTTCCAAGTTTTAGGCTACGGCATCTGCGCCACGCGCGGCACGGCGCAATACC
TGACCGAACACGGGCTGATTGTGCAGACCATCAACAAAGTACCCGAAGGCCGCCCGCACA
TCGGCGACGCGCTGAAAAACGGCGAAATCGCACTGGTCGTGAACACCGTTTCCAGCGATC
CGCAATCCGTGTCCGACAGCCACATCATCCGCCAAAGCGCATTGCAGCAACGTGTGCCGC
AATACACCACCACCGCCGGCGGCGAAGCGATGAGCGAAGGCGCGAAAAGCCGAGACCATC
TGGGCGTGTACAGCGTTCAAGAACTGCACGGGCGTTTGAAAAACCGCAACTGATGCCTGA
ATCAGGTTGAAAATGCCGTCTGAAGCCGTTTTGCGGTTTCAGACGGCATTTTGTCATTTG
GAAAGCCGATGTTGCCACACACAAGCCGTACATAAGGAACAGCCCTATCACGCTCCCCAT
ATAGATTGCCATTGCCGCCGACTATACATTATCTTATTTATTTTTCTCAAAGTTATTAA
GTGAGTAAAAACAGTTTTATGACAGGTTTTTATAGAATTATCCACAGAGATTGTTTCCCA
GTTCCTCCACTAAAAAATCCAAAAATACGCGTAAGCGGAGATTGACGGCTTTATCGCTGT
AATAAACAGCATTAAAGGGGTGTGTTTTATCGGAGGTTTGTTCGGCGAGCAGGGGAATTA
ACTTTCCTTCAGCGATGTCGTTGTCAACCAAAAAATCTGATAAGCAAACAATACCGCAAC
CTGAAAGGCACAACGAGCGTAAGATTTCACCGCTGCTGGCGGTAAAGTGCGGTGAAATCT
TATAGGGATTTCCCTGCGCATCTAAAACCGCCCATGTATTTAGAGAACCGGGTTCGGTGA
AGCCTAAACATTGGTGGCCGGCAAGCTCTTCTGTAGATTGCGGCGTGCCGTGTTTTGCCA
GGTATTCAGGACTGGCGATTACGCGGAAGCGGCTGTCAAACAGATGGCGTGCACGCAGCC
CGGAATCGTCCAATTCTCCGGCCCGTAAGGCAATATCGACTTTGCGTTCAATCAGATTGA
TATAGCCTTCGGAAGAAACGAGCGAAAGTCGGATATGCGGATAGCGTTCGTTGAATTTTG
CTGCCAGCGGCGCCAGCAGATGCAGCACCATCGGCATCGCGGAATCCACGCTCAACACGC
CTTGCGGTATTTCGTGCACTGCCAGCATTTCGGTTTCCGCCGCTGCCATTTCTTGCAGGA
TTCTCTGCGCGCGGCGGAAATATTGCGCGCCTTCTTCCGTCAGACTGAGTTGCCGCGTGG
TGCGGTTGAGCAGGTTCACACCCAACTTTTCCTCCAGCCGTTTGACGATGCGGCTTACGG
CAGAATTTGCCATCGCCAACTGCTCCGCCGCACGGCTGAAGCTGCCGCTTTCCACCACTT
GAACAAATACGGTCAGTTCTTCTGAATTGGTTTTCATCGTGTTTCCTTTTCGGTTGGAAC
CCCGCCCTTTAGGGCGGCAGGATCAGACTTTATTTGGGAGGGGTGTAACCCCTTCCGAAT
CAGGACGGCACACAGGGCGGTGCTTTATGTGCCATCCCGTGTGTTGGAACATCTGATTAT
TTCATTTGACGCAAAAGTGTTTTCTTATTTTTGCACTTTTAAATTATAAAGTAAAACGGC
ACAATACATTCATCAATTCACAAACGAGGTAACAAATGAATATTTTATTATTAGACGGCG
GCAAGGCGTTCGGACATTCTCACGGCGGGTTAAACCGTACGCTTCACAAAAAAGCGAAAG
AAGTTTTGACCGCGCTCGGACACAATGTTCAAGAAACCGTGATTGATGCCGGCTATGATG
TTGAGGCAGAAATCGAAAAGTTCGTTTGGATGGATGCTGTGATTTGGCAGATGCCGGGCT
GGTGGATGCACGAGCCTTGGACAGTGAAAAAATACATAGACGAAGTATTAACCGCTGGAC
AGGGCAAACTCTACCAAAGGACGGCAGACACAGCGTCAATCCGACTGAGGGCTACGGCA
CAGGCGGCTTGTTGCAAGGCAAAAAACATATGATTTCACTGACTTGGAATGCGCCGATTG

## Appendix A

AAGCCTTTACCCGCGAAGGCGATTTCTTTGAAGGCAAAGGCGTTGATGTTTTGTATATGC
ACTTCCACAAAGCCAACGAGTTTTTGGGTATGACCCGCCTGCCGACATTCTTATGTAACG
ATGTGGTTAAAAATCCGCAAGTGGAAAAATACTTGGCAGATTATCAGGCACACTTGGAAA
AAGTGTTCGGCTAAAAATTTATCTTATAAACAAACAAAGGCAGCCTGAAAGATTGAATGG
TCTGCACCCCTAAGGTTGGACTAACCAACCGACTAAGGTGCAGATTATTTTTTGTTGCTT
TTTCAGCTTTTCGTTGGGTTAGATATTCTTGCCCACTGTTTTCAGGCAGCCTTGAATACA
AAAAAATGGCGTATGTAATATGTTTATACGACCAAAACGGAATGAATTTTAACGTATTGC
GCGTCATCAACAATGACTGAGTTTCTCGCCTCTCGCGCCTGAATCTATAGTGGATTAACA
AAAACCAGTACAGCGTTGCCTCGCCTTGCCGTACTATTTGTACTGTCTGCGGCTTCGTTG
CCTTGTCCTGATTTAAATTTAATCCACTATATGTCATGCAGTTCCTTTCATTCAAATCAA
CAAAAGAATGCCGTCCGAACGTCCGTTCAGACGGCACTTGTCTTCCCACAATAGACTTGA
GGCTGTTCTAACGTACCACCCCTTCGTTCCGCCCCAAAACCATCGCATCGCCGTAGCTGA
AGAAACGGTATTCGCGTTCGACCGCATGACGATACGCGGCGCGGATATGACCCATACCCG
AAAACGCGCTGACCAACATCAGCAGCGTCGATTTCGGCAAATGAAAATTGGTAACCAGTC
TGTCGACAACATTAAAACGGTAGCCCGGCGTGATGAAAATATCGGTGTCGCCCTGCCCCG
CTTTCAGACGACCCGTCGCACGCGCGGCAGATTCGAGGGCGCGCATGGAAGTCGTGCCGA
CCGCCCAGACTTTGTTCCCCCGGGCTTTTGCCGCCTCAACGGCGGCGGCGGTTTCAGACG
GCACTTCAAACCATTCGCTGTGCATTTTGTGCTCTTCGATTTTGTCCACACGCACGGGTT
GGAACGTTCCGGCACCGACGTGCAGGGTTACTTCTGCGGTTACCGCGCCTTTGTCTTTCA
GACGGTGCAAAAGTTCTTCCGTAAAATGCAGGCCCGCCGTCGGCGCGGCGACCGCGCCCT
GATATTTGGCATAAACGGTTTGATAACGGCTGTCGTCATCCGCATCGGCGGCGCGTTCGA
TATAAGGCGGCAGGGGCAGGTGTCCGTTCTGTTCCAAAAGTTCGTAAACGGTCTCTCCGC
CTTCAAAACGCAGGCAGAACAGTTCGCCCTCACGCCCGACCGTCACGGCGCGGATGCCGC
CTTCAAACACCAGCCCCATACCGGGCTTGGGCGATTTGGACGAACGGATGTGCGCCAGTG
CGGTATGGTTGTCCAACACGCGCTCAATCAGGGCTTCGATCCTGCCGCCGCTGTCTTTCT
GCCCAAACAGCCGCGCCTTCATGACTTTGGTGTTGTTGAACACCAAAACGTCGCCCGCCT
CGACATAATCCGGCAAATCGCCGAACACCCGGTCTTGCAGCGGCATATCGGGCAACGCAA
CCAAAAGGCGGCTGCTGCCGCGCACTTCGGGCGGATGCTGGGCAATCAGCTTTTCGGGCA
GGGTAAAATCAAAATCTGAAATATCCATTTTTACACTCTCGTTCGGGCAAGCCGCCATTA
TACGCACTTTAGCCCTTTTTCAGACGGCATCTTTGTCCGAAAAACCAACAGATTAGAATA
AACACTCTTAACCTGGAACATCTTGTGCGCAAAATCAAACTTCCTGCACATTTCCCCCAA
AAACCGCCGTTTTTTGATATTTTACTGGACATTTACCGACAACTTCGGGAAAATAAACAC
ATTCTCACGGTCGTTTTCCACCACAGGAAAACCGTATCCGAACACCATTCCGCCCGGTTT
GCGCCGTTGCCGCAAGCCGGCTGTTTTCTGAAAAACCAACGCAACAACCCGCCGGAACAC
CGGCAGCCTTTAAAGGAACAGAAATGGATTTGCGCAAATTAAAAAAACTGATTGATTTGG
TTGAAGAATCGGGTATCGCCGAAATCGAAGTAACCGAAGGCGAGGAAAAAGTCCGCATCA
CCCGAACCATCGCCGCCGCACCCGTTTACGCCGCGCCCGTACCTGCCGCCGCGCCGGCCG
TAACGCCTGCCGCCGCACCCGTTGCGGCATCCGCGCCCGCCGCCGCACCTGCCGCCCGCG
ATTTGTCCGACGCGCAAAAATCGCCTATGGTCGGCACGTTCTACCGCGCACCCGGCCCGA
ATGCCGCGCCTTTTGTCGAAGTCGGCCAACAAGTTAAAGCCGGCGACACGCTGTGCATCA
TCGAAGCGATGAAGCTGATGAACGAAATCGAAGCCGAAAAATCCGGCACGGTCAAAGAAA
TTTTGGTCGAAAACGGTACGCCCGTCGAATTCGGCGAACCGCTCTTCATTATCGGATAAT
CCTGTTTTCAGACGGCATAAACTTCCGATGCCGTCTGAAATGCTTTCCCCCTTCAGCGTT
CCCGCACCCTTTTTTACGGACGGGTTGCCGGAACCGCAGGAAAGGTCATCATGCTGAAAA
AAGTTTTAATCGCCAACCGAGGCGAAATCGCATTACGCGTACTCCGTGCCTGCCGCGAAA
TGGGCATTGCCACCGTCGCCGTGCATTCCGAGGCCGACAAAGACAGCCTGCACGTCAAAC
TCGCCGACGAATCCGTGTGCATCGGCCCTGCCGCTTCCGCGCAAAGCTACCTTAACGTCC
CCGCCATTATCGCCGCCGCCGAAGTAAGCTGCGCGGACGCTGTCCATCCGGGTTACGGTT
TCCTTGCCGAAAACGCCGATTTCGCCGAACAGGTCGAGCAGTCCGGCTTTACCTTTATCG
GCCCGAAACCCGACACCATCCGCCTGATGGGCGACAAAGTCTCCGCCAAACACGCGATGA
TAGCGGCAGGCGTACCCTGCGTCCCCGGTTCTGACGGCGCATTGCCCGACGACGGCGAAG
AAATCCTCAAAATCGCCGATAAAGTCGGTTATCCCGTCATTATCAAAGCCTCTGGCGGCG
GCGGCGGCCGCGGTATGCGCGTGGTCGAGAAAAAAGAAGACCTCCTCCAATCTGTCGAAA
TGACCAAAGCCGAAGCAGGCGCGGCATTCGGCAACCCGATGGTTTACATGGAACGCTATT
TGCAACGTCCGCGCCACGTCGAAATCCAAGTGATTGCCGACGAACACGGCAACGCCATCT
ACCTTGCCGAGCGCGACTGTTCGCTGCAACGCCGCCACCAAAAAGTCATCGAGGAAGCAC
CGGCTCCGTTCATCACTGAAAAAGAACGCGCCAAAATCGGCAACGCCTGTGCCGATGCCT
GCAAACGCATCGGCTACCGGGGCGCGGGTACGTTTGAGTTTTTATACGAAGACGGCGAAT
TTTTCTTTATCGAGATGAACACGCGCGTTCAGGTCGAGCATCCGGTTACCGAGCTCATCA
CCGGCGTGGACATCGTGCAGGAGCAACTCCGCATCGCCGCCGGCCTGCCTTTGCAATACA
AACAAAAGGATATTCAAGTCGAAGGCCACGCGTTTGAGTGCCGTATCAACGCCGAAGACC
CGTACAACTTCATTCCAAGCCCGGGCCTGATTGAAAGCTGCCACCTGCCCGGCGGCTTCG
GTATCCGCGTGGACAGCCACATTTACCAAGGCTACCGCATCCCACCGTACTACGACAGCC
TGATCGGCAAAATCTGCGTACACGGCAAAACGCGTGAACAGGCAATGGCGAAAATGCGCG
TCGCACTCGCCGAGCTGGCGGTAACCGGCATCAAAACCAATACGCCGCTTCACCGCGACC
TGTTCGCCGATGCGGGTTTCCAAAAAGGCGGCGTCAGCATCCACTATTTGGAACACTGGC
TGGAAGATCGCAAAGCCAAACAGGACAAGTAAACCGCCGCCGATATGCCGTCTGAAGCCG
CCCGTCCGCGTTCAGACGGCATTTCCCTTGCCCCGCGCCGTCTGAAACCGATTTCGATAT
AGTGGATTAACTTTAAACCAGTACGGCGTTGCCTCGCCTTAGCTCAAAGAGAAAGATTCT
CTAAGGTGCTGAAGCACCAAGTGAATCGGTTCCGTACTATTTGTACTGTCTGCGGCTTCG
TCGCCTTGTCCTGATTTAAATTCAATCCACTATATTTCCAAGAAAGCCCGTTATGCCCTA
CCAACAAATCACCGTCAACGTCAACGATGCCGTCGCCGAACGCCTCGCCGACGCGCTGAT
GGAACACGGCGCACTCTCCGCCGCCATCGAAGATGCCTACGCCGGCACGCAAAACGAACA
GGCGATTTTCGGCGAACCCGGTATGCCCGCCGAACAAATCTGGCAGCAGAGCAAAGTCAT
CGCCCTGTTCGGCGAACACGACGAAGCCGCCGCCATCATCCAAACCGCCACACAAGAATG

## Appendix A

CGGGTTAAAAGACTTGGCATACACCGGCGAAACCATCGAAGACCAAGACTGGGTGCGTCT
CACGCAATCGCAATTCGACCCCATCCGGATTTCCGACCGCCTGTGGATTACCCCCTCTTG
GCACGAAGTCCCCGAAGGCAGTGCCGTCAACCTCCGCCTCGACCCCGGACTCGCCTTCGG
CACCGGCAGCCACCCGACCACGCGCCTCTGCCTCAAATGGTTGGATACGCAACTCAAAAA
CGGCGAAAGCGTCCTCGACTACGGCTGCGGTTCGGGCATCCTGACCATCGCCGCCCTCAA
ACTCGGTGCAGGTTTCGCCGTCGGCGTGGATATTGACGAACAGGCCGTCCGCGCCGGCAA
GGACAACGCCGCGCAAAACAACGTCGATGCACAATTCTTCCTGCCCGACGGTCTGCCTCA
AGGGCAATTCGACGTAGTTGTCGCCAACATCCTCGCCAACCCTTTGCGTATGCTTGGCGA
AATGCTCGCCGCCCGCACCAAACAGGGCGGACGCATCGTGTTGTCCGGTTTGTTGGACGA
ACAGGCCGAAGAACTCGGCGGCATTTACAGCCAATGGTTCGACCTCGACCCGGCGGAAAC
CGAGGAAGGATGGGCGCGATTGAGCGGCGTAAAACGCTGAAACGGAAAGGAAACACCGTG
CAGGATAAAAACAACCTCTGCTGGCTCGATATGGAAATGACGGGGCTGAATCCCGAAACC
GACCGCATTATCGAAGTCGCGATGATTATTACCGACTCGGATTTGAATGTGTTGGCGCAA
TCCGAAGTTTACGCCGTCCACCAAAGCGACGACGTGCTGAACAAAATGGACGAATGGAAC
ACCGCCACACACGGCAGGACGGGGCTGACACAGCGCGTACGCGAATCGTCGCATACCGAA
GCCGAAGTCGAACAGAAACTGCTGGACTTTATGTCGGAATGGGTACCCGGACGCGCCACG
CCGATGTGCGGCAACTCCATCCACCAAGACCGGCGTTTTATGGTCAAATATATGCCGAAA
CTGGAAAACTACTTCCACTACCGCAACCTCGACGTTTCCACGCTGAAAGAACTCGCCAAA
CGCTGGAATCCGCCCGTTGCCAAAAGCGTCGTCAAACGCGGTTCGCACAAGGCATTGGAC
GACATTTTGGAGAGCATCGAAGAAATGCGCCACTACCGCGAACACTTTCTGATTTCCGCC
CCGAGAGCCGAAGCGCAATAAGAAACAAACAATGCCGTCTGAAACGCAGTTTGCATTTCA
GACGGCATTTTTACAGCAGATTGAAATCAAAAATATACACGCCCGTCATTCCCGCACAGG
CGGGAATCCGGAAGGTCGGGCCTGCCGTTATTTTCAATCATTACAGAAACTGAAAGGTCT
GGATTCCCGCCTGCGCGGGAATGACGGGCGTGTGCATTCTTATAGTGGATTAACAAAAAT
CAGGACAAGGCGACGAAGCCGCAAACAGTACAAATAGTACGAAACCGATTCACTTGGTGC
TTCAGCACCTTAGAGAATCGTTCTCTTTGAGCTAAGGCGAGGCAACGCCGTACTGGTTTT
TGTTAATCCACTATACTTCAATCTGCCAAACAGATCGAACAGAGAAACCCTGTCCGTCAA
AACATCATTCAGCCATCGCCTTGAACACTTCAACCGCAACCGCAACCGTTTCGTCAATCA
GCTCGGGCGTATGCGCGGCGGAAACGAAACCTGCTTCATAAGCGGACGGGCCGAAGGCGA
CATTGCGGTCGAGCATCCCGTGGAAAAACTGTTTGAAGCCTTCAATATTGGAACGCGCCA
TATCGGCATAGTTTCGCGGCGCGTGTGCGGCGAAATACAGACCGAACATACCGCCCACGC
TGTCGGCGGTGAACTCGATGCCCGCCGCATCCGCTGCCGTCCGAAAACCTTGAACCAACT
GTTCGGTACGCGCCGTCAGGTTTTCATAGAAGCCTTCGCGCTGGATGATTTCCAGCGTTT
TCAAGCCTGCGGCGACAGCAATCGGGTTGCCCGACAAGGTGCCTGCCTGATACACGCCGC
CCAGCGGGGAAATACATTCCATAATGTCTTTGCGCCCGCCAAACGCGGCAAGCGGCATAC
CGCCGCCGATGACTTTGCCCATCGTGGTCAGGTCGGGCGTGATGCCGTGCAAAGATTGCG
CGCCGCCGAGCGCGACGCGGAAGCCGGTCATCACTTCGTCGTAAATCAACACCGCGCCGT
ATTTTTCGGTCAATCCGCGCAAGGCTTTGACAAAGGCTTCGGTCGGGCGGACGAGGTTCA
TATTGCCGACGAAGGGTTCGACAATCACGCAGGCGATTTCATTGCCGCTTTGAGCAAAGG
CTTCTTCGAGTTGGGCGATATTGTTGTACTCGAGTACCAAAGTGTGTTTGGTAAAGTCGG
CAGGCACACCGGCGGAAGACGGGTTGCCAAACGTCAGCAGACCGCTGCCGGCTTTCACCA
GCAGGCTGTCGGAATGCCCGTGGTAGCAGCCTTCAAACTTGATGATTTTGTCACGCCCGG
TAAAACCGCGTGCCAGACGGATGGCGGTCATGGTCGCTTCGGTACCGGAGCTGACGAGGC
GCAGCCGTTCGACGGACGGCATGATTTTGGCGATTTCTTCGGCAATGACGATTTCGCCTT
CGGTAGGCGCGCCGAACGACAAACCGCCCAATGCGGCTTCGCATACGGTTTCGACGACTT
CGGGGTGCGCGTGTCCGACAATCGCAGGTCCCCACGAGCCGACGTAATCGGTATAGCGCG
TGCCGTTTTCGTCCCAAACATACGCGCCTTCGGCTTTTTTGATAAAGCGCGGTACGCCGC
CGACGCTGCCGAATGCGCGGACGGGGGAATTCACGCCGCCGGGGATGATGGCTTTGGCGC
GGTCGAATAAAATTTCGTTACGGTTCATATATATCCTCAAATGCCGTCTGAACGGCAGGT
~~TTCGGGCTTGGAAGCAGAAAGCCCCATTTTTATCATTTTTCAGGTTGCGACAAGGATTTGC~~
CCGCTTCTTTGCGGATCACGCCAACCGCATCCCGGATGACGGAACGCTCGTCTTTTTCCA
CTTTATGTGTAAAGCGGTAGTCTCGGACGACTCCCTCCCCGTCGTAATCCACACACCACT
CCCAATGTCGGCGTTCTGATTTCATATAAATGAAATTGGTCGGCAAAAAATTATAAATCG
GCAGGCTGACTTCATGATAGGCATAACAACCGAAAGGGTTGCGCTTCCCGAAACGTGCCT
CTACACCTCCGCCCGGGTCGTTTTGCCTTTAACAACCGTTTGTGCGATTCCCTCTTCCGT
CTGATATAGTGGATTAACAAAAATCAGGACAAGGCGACGAAGCCGCAGACAGTACAGATA
GTACGGCAAGGCGAGGCAACGCTGTACTGGTTTTTGTTAATCCACTATAACGCAGGAACT
GATGTTCCCTGTCGCCGAAATTGCTGGTACACGCACACAGCAGCAATGCCGCCCATACAG
CCGGTTTCATACACATCTCCCATTAAAGCCAAACATTATACAGCCGTCCCGACCGATTAA
ATTCATATTTTAAAACAATATCCTGCCTCCAAAACCCACATCGTGCTATAATCCGCACCG
ATTTTCAGACGGCATCGTCGTGCCGTCTGAAATTTTTTCATTCCAACAACAATCAGCCCC
GCGATTACGGCTGCCTGAGAAAGACACAAACCATGAAAAAAGTATTTATCCGCACCTTCG
GCTGCCAGATGAACGAATACGACAGCGACAAAATGCTCGCCGTCCTCGCCGAAGAACACG
GCGGCATCGAACAGGTTACCCAAGCCGACGAAGCCGACATCATCTTGTTCAACACCTGCT
CCGTGCGCGAAAAAGCGCAAGAAAAAGTCTTCTCCGATTTGGGGCGCGTGCGTCCGCTCA
AAGAAAAAAACCCCGGCCTCATCATCGGCGTTGCCGGCTGCGTCGCCTCGCAAGAAGGCG
AAAACATCATCAAACGCGCGCCTTATGTGGACGTGGTTTTCGGCCCGCAAACGCTGCACC
GCCTGCCAAAAATGATTGTGGACAAAGAAACCAGCGGGCTGTCGCAAGTCGATATTTCCT
TCCCCGAAATCGAAAAATTCGACCACCTGCCGCCCGCCCGCGTCGAAGGCGGCGCGGCAT
TTGTATCGATTATGGAAGGCTGTTCCAAATACTGCTCCTTCTGCGTCGTCCCCTACACGC
GCGGCGAAGAATTCTCCCGCCCGCTCAACGACGTATTGACCGAAATCGCCAACCTTGCCC
AGCAAGGCGTGAAAGAAATCAACCTCTTGGGACAAAACGTCAACGCCTATCGCGGCGAAA
TGGACGACGGCGAAATCTGCGACTTCGCCACCCTGCTGCGCATCGTCCACGAAATCCCCG
GCATCGAACGTATGCGCTTCACCACCAGCCACCCGCGCGAGTTTACCGACTCGATTATCG
AGTGCTACCGCGACCTGCCCAAACTGGTTTCCCACCTGCACCTGCCGATTCAAAGCGGTT

## Appendix A

CCGACCGCGTATTGAGCGCAATGAAACGCGGCTACACCGCTTTGGAATACAAATCCATCA
TCCGCAAACTGCGCGCCATCCGTCCTGATTTGTGCCTGAGCAGCGATTTCATCGTCGGCT
TCCCCGGCGAGACCGAACGCGAGTTCGAGCAAACCTTGAAACTGGTGAAAGACATCGCCT
TCGACTTGAGCTTCGTGTTTATTTACAGTCCGCGCCCCGGCACGCCTGCCGCCAACCTGC
CGGACGACACGCCGCACGAAGAAAAAGTGCGCCGCCTCGAAGCCTTGAACGAAGTCATCG
AAGCCGAAACCGCGCGCATCAACCAAACCATGGTCGGCACGGTACAACGCTGCCTGGTCG
AAGGCATCTCCAAAAAAGACCCCGACCAACTGCAAGCCCGTACCGCCAACAACCGCGTCG
TCAACTTCACCGGCACGCCCGACATGATTAACCAAATGATCGATTTGGAAATCACCGAGG
CCTACACCTTCTCCCTGCGCGGCAAAGTTGTCGAAGCCTAAACCCTCACGCCGAAAAAAT
GCCGTCTGAAGCGTTTCAGACGGCATTTTGCCTTGTATCGGCAGACGACGGCGCGGCCGG
GCGGCTTAATTTGCCGCATCCCGATCCGACAGCCACGCGCGCACACGCCGTTCCACCGCT
TCGGCACTCAAGCCCAAATCGTCTAAAAGTTTTTTCGGATCGCCGTGTCCGGTTACGGTA
TCGGCAACGCCCAAAAGCAAAACGGGTTTGCAGATGCCGTGTTTCGCCAATACTTCCAGC
ACCGCGCCGCCTGCGCCGCCCTGTTCGGCGTTTTCTTCAAGGGTAACGATGCGGTCGTGG
CTTCGGGCAAGGCGGACAATCAACTCTTCGTCTATCGGTTTGACGAAGCGCATATCGGCG
ACGGTGGCGTTCAGTTTTTCGGCAACCGCCAATGCGGGGGCGACCATACTGCCGAAGGCA
ATGAATGCGGTTTTCTCACCTTCGCGGCGGATAATGCCCTTGCCGATTTCCACGGTTTCC
ATGCCGTCTGAAACCGGCGCGCCCGTACCCGTGCCGCGCGGATAGCGGACGGCGGCGGGC
GCGTCTGCCTGATAGCAGGTCGAAAGCAACAGGCGGCATTCGTTTTCATCGCTCGGCGCG
GCGACAATCATGTTCGGCACGCAGCGCAAAAAGCTCAAATCGTACAGACCGGCATGGGTC
GGGCCGTCCGCGCCGACGATGCCCGCGCGGTCGACGGCAAACAAAACGGGTAGGTTTTGC
AGGGCGATGTCGTGCACCAGTTGGTCGTAGGCGCGTTGTAAAAAGGTGGAATAAATCGCC
ACGACGGGCTTCATCCCTTCGCAAGCCAAACCGCCGGCAAAGGTAACGGCGTGCTGCTCG
GCGATGCCGACATCGAAATAGCGGTCGGGGAATCGTTGTTCAAACTCAACCAAGCCGCTG
CCCTCGCGCATGGCGGGGTAATCGCAACCAGTCGGGAATCTGCCGCCGCCCGGTCGCAC
AGCCATTTGCCGAACACTTGGGTATAGGTCGGTTTGGCGGCGGGCTTGGGTTCTTTTTCA
GACGGCATTTGCGCCGCGCTTTCTTTAGGCAGGTTGGCGACGGCGTGGTATTTGACGGGG
TCGTTTTCGGCGAGTTTGTAGCCGTTGCCCTTTTTGGTGATGACGTGCAGCAACTGAGGG
CCTTTGCGGCTGCGCAAGTCTTTCAATACGTCCACCAGATTTTCGACGTTGTGTCCGTCC
ACGGGGCCGGTGTAGCGGAAGCCGAAGTTTTCAAACAAAGACAGCGACTGTTTGGCGTGT
TCGGCTTCTTCGGCAAGGGTTTTGATTTTGTGTTCGACTTTTTGGGCAAACTCCATCGCG
CCGGGTATTTTGTCTAATACCTTGCCCGTTTGCGCTTTGACGGTACTCAACAGGCCGTGC
ATATCGCGCACGACGTTGCTGGCAAGGTATTTCGGCAGCGCGCCGACGTTGGGGGAAATC
GACATTTCGTTGTCGTTGAGGACGACCAGCAAATCCACATCCATATCGCCTGCGCAATTC
AAGGCTTCAAACGCCTGCCCCGCCGTCATCGCGCCGTCGCCGATGATGGCGACGCTGCGG
CGGTCGCTGCCCAAGAGTTTGTCTGCCGCCGCCATGCCCAACGCCGCGCCGATGGAGGTG
GAGGAATGCCCCACGCCGAACGCGTCGTACTCGGACTCGCAACGTTTCGGAAAACCCGCC
AAACCGCCATATTGGCGCATGGTGTGCATCTGGTTTTTCCTGCCTGTCAGGATTTTGTGC
GGATAGCTTTGGTGTCCGACATCCCACACCAGCTTGTCTTCGGGCGTGTCGTACACATAG
TGCAGGGCGATGGTCAGTTCGACCGCGCCCAAATTGCTGGCGAAATGCCCGCCGGTCTGC
CCGACAGATTCCAGCAGAAAGGTGCGCAACTCGCCGGCAAGGCGCGGCAGCTGTTTTTTG
TCCAGACGGCGCAAATCTTGCGGGCTGTCAATCAGGTCGAGTAGGGGGCTTGGGTTCATG
GTGTGTCTTTTTTATGTGTCGTCCGGGTGCAACGGTCAATTATATATCAAGAGCGTGCGG
CTGACGGCTGATTTTGCCGTATGTCATTCGTCCTGCCGCTTGGCGCGCGGGTGGGCTTCG
TCATACAGGCGGGCGATGTGGTCGAAATCGAGCTTGGTATAAATCTGCGTGGTCGAAAGG
CTGCTGTGCCCGAGCAGCTCCTGCACCGCCCTGATGTCGCGCGAAGCCTGCAATAGGTGT
CCGGCGTAGCTGTGGCGCATCATATGCGGCGAAACGTGCCTGCCGTCGCCGTTTTGCGCC
GCCCATTGCGCCAAACGTTTTTGGATTTGGCGTTGGCTCAGGCGCGTGCCGTTCCTGCCG
GTAAACAGGGCTTTGCCGTCCGATGCCGTCTGACGCAGCGGCAGATAGTTTTTCAGGGCT
TCCACGCTTTTGCCGACCAGCGGCACCTGCCGCTGCTTGCGCCCTTTGCCGATAACGTGT
ACCCACGCCTCGTCCAAATAGACATCATCTGCATTCAAGCCGTGTATCTCGCTCACGCGC
AAACCGCTGCCGTACATCAGTTCGAACAGGGCGTGGTCGCGCACCGCCAGCGGGTCGCCG
CCGTCCACGGGCAAATCCAGCATCCGGTTCAGCCATTCCTGCGGCAGGGCTTTGGGTACG
CGCTCGGGCTGCTTCGGCGGTTTGATGTCGGCGGTCGGGTCGGCGTGCATCAGGCCGCGC
TTTACCAGCCAAACGCAATACTGCCGCCAAGACGAAAGCTTGCGAGCCAGCGTCCGTTCC
CCCAAACCGCGGCCGGACAGCCGGCGTAATGCCTGTACGAAGTCGCCGCGAGTGCAATTT
GAAGGGTTTGCAGACGGCATTTCTTCCAGAAGGGCAAGCAGTTCCTGCAAGTCGCGCCGG
TATGCGGCAACCGTGTGCTCCGATTTACCCTCGCGCACGATATTTTCCAAATAAGCGTCC
AAGTATGCCGCAAGTCCGTCCAAACCCATTCCCACACCTAAAATAACATTAGAAACATTA
TCATAAATCGGAATATCCGAATCCCGAAACGTCAAAACCCGACAAACCTGCATACTGGCA
TCGTTAATATAAAATCAATGAGCTGTTTATGGTTTTTTGCTGTAAAAAACATTATAATCC
GCCTTATTTACCTATTGCCCAAGGAGACACAAATGGCACTCGTATCCATGCGCCAACTGC
TTGATCATGCTGCCGAAAACAGCTACGGCCTGCCGGCGTTCAACGTCAACAACCTCGAAC
AGATGCGCGCCATCATGGAGGCTGCAGACCAAGTCGACGCCCCCGTCATCGTACAGGCGA
GTGCCGGTGCGCGCAAATATGCGGGTGCGCCGTTTTTACGCCACCTGATTTTGGCGGCTG
TCGAAGAATTTCCACACATCCCCGTCGTCATGCACCAAGACCACGGCGCATCACCCGACG
TGTGCCAACGCTCCATCCAACTGGGCTTCTCCTCTGTAATGATGGACGGCTCGCTGATGG
AAGACGGCAAAACCCCTTCTTCTTACGAATACAACGTCAACGCCACACGTACCGTGGTTA
ACTTCTCCCACGCTTGCGGCGTATCCGTTGAAGGCGAAATCGGCGTATTGGGCAACCTCG
AAACCGGCGAAGCAGGCGAAGAAGACGGTGTAGGCGCAGTGGGCAAACTTTCCCACGACC
AAATGCTGACCAGCGTCGAAGATGCCGTATGTTTCGTTAAAGATACCGGCGTTGACGCAT
TGGCTATTGCCGTCGGCACCAGCCACGGCGCATACAAATTCACCCGTCCGCCCACAGGCG
ATGTATTACGTATCGACCGCATCAAAGAAATCCACCAAGCCCTGCCCAATACACACATCG
TGATGCACGGCTCCAGCTCCGTTCCGCAAGAATGGCTGAAAGTCATCAACGAATACGGCG
GCAATATCGGCGAAACCTACGGCGTGCCGGTTGAAGAAATCGTCGAAGGCATCAAACACG

## Appendix A

GCGTGCGCAAAGTCAACATCGATACCGACTTGCGCCTTGCTTCTACCGGCGCGGTACGCC
GCTACCTTGCCGAAAATCCGTCCGACTTTGACCCGCGCAAATACCTGAGCAAAACCATTG
AGGCCATGAAGCAAATCTGCCTCGACCGTTATCTTGCGTTTGGCTGCGAAGGTCAGGCAG
GCAAAATCAAACCTGTTTCGTTGGAAAAAATGGCAAGCCGTTATGCCAAGGGCGAATTGA
ACCAAATCGTCAAATAACAGGTTGCCTGTAAACAAAATGCCGTCTGAACCGCCGTTCGGA
CGACATTTGATTTTTGCTTCTTTGACCTGCCTCATTGATGCGGTATGCAAAAAAAGATAC
CATAACCAAAATGTTTATATATTATCTATTCTGCGTATGACTAGGAGTAAACCTGTGAAT
CGAACTGCCTTCTGCTGCCTTTCTCTGACCACTGCCCTGATTCTGACCGCCTGCAGCAGC
GGAGGGGGTGGTGTCGCCGCCGACATCGGTGCGGGGCTTGCCGATGCACTAACCGCACCG
CTCGACCATAAAGACAAAGGTTTGCAGTCTTTGACGCTGGATCAGTCCGTCAGGAAAAAC
GAGAAACTGAAGCTGGCGGCACAAGGTGCGGAAAAAACTTATGGAAACGGTGACAGCCTC
AATACGGGCAAATTGAAGAACGACAAGGTCAGCCGTTTCGACTTTATCCGCCAAATCGAA
GTGGACGGGCAGCTCATTACCTTGGAGAGTGGAGAGTTCCAAGTATACAAACAAAGCCAT
TCCGCCTTAACCGCCTTTCAGACCGAGCAAATACAAGATTCGGAGCATTCCGGGAAGATG
GTTGCGAAACGCCAGTTCAGAATCGGCGACATAGCGGGCGAACATACATCTTTTGACAAG
CTTCCCGAAGGCGGCAGGGCGACATATCGCGGGACGGCGTTCGGTTCAGACGATGCCGGC
GGAAAACTGACCTACACCATAGATTTCGCCGCCAAGCAGGGAAACGGCAAAATCGAACAT
TTGAAATCGCCAGAACTCAATGTCGACCTGGCCGCCGCCGATATCAAGCCGGATGGAAAA
CGCCATGCCGTCATCAGCGGTTCCGTCCTTTACAACCAAGCCGAGAAAGGCAGTTACTCC
CTCGGTATCTTTGGCGGAAAAGCCCAGGAAGTTGCCGGCAGCGCGGAAGTGAAAACCGTA
AACGGCATACGCCATATCGGCCTTGCCGCCAAGCAATAACCATTGTGAAAATGCCGTCCG
AACACGATAATTTACCGTTCGGACGGCATTTTGTATTGCACCGTCCGACGGCATGCCCAA
GGGGGGAAATCCCTATTTTCAGGCCAACCGCTATATAATGCCGTCTGAACCAACGAGAGA
ATGCCATGCAAGCTGATTTTAACCGTCCCGTCCTGGCCGTCGATACCGGTACTTCCCGTT
TGTCGCTCGCGCTGCGTGCCGACGGCGAAACCCGTCTGTTCCATCAGGAAGTCGGCAGCC
GCCAGTCCGAACTGATTCTGCCGGAAATCCGCACCCTATTCCGCGATGCAGGCATTACCG
CCGCCGATTTGGGTGCGGTCGTGTACGCACAGGGTCCCGGCGCGTTTACCGGACTGCGTA
TCGGCATCGGTGTAGCTCAGGGTTTGGCAACGCCGTTTGATACCCCCTTAATCGGCGTAC
CCTCGCTCGATGCCGCCGCCTCGCTGCCGCCGCCGCAAAGCTGCATCCTTGCCGCTACGG
ACGCTCGTATGGGCGAAGTGTTTTATGCATGGTTCGATACGCTGAACTGCCACCGTTTGA
GCGATTATCAGGTCGGGCGGGCGGCAGACATCCGGCTGCCGGAGGGATGCGCCTTTTCAG
ACGGCATAGGCAGCGCGTTCGCGCTGGAAGAAGCTCCGCCGTTCTCAGGCAGACCGGATA
TGCCGACTGCCGCCGACTTTCTCGCATTGGCAGCCAAGGGCGGTTATCCTGCCGTCCATG
CCGCACACGCCGGTTTGCTCTACGTCCGCAACAAAATCGCCCTGACTGCCAAAGAACAGG
CCGAACGGAGAGCGCGCCCGTGAACATCCGCCGTGCCGTTTGTGCCGATTGTGAGGAGCT
GGCCGCACTCGATGCCGTCTGCAACCCGTCCGCATGGACGCAACGCCAATTTGAGTCCGC
ACTGGTTTCGCCGTCCGAACAGGTTTTCCTTGCGGAAAAAGACGGCGGGATTGCCGCCTT
TATCGTTTGGCAGAACCTGCCCGACGAATCCGAACTGCACCTGATTGCCACCGCGCCCGA
ATGCCGCCGCCAAGGAATTGCGTCCGCCCTGCTCGAATATTGGTTCACACATCTGCCCGA
AGACACGCAACGCCTGCTGCTCGAAGTCCGTGCAGGCAACACCGCCGCACAGGCACTGTA
CACGGCGCACGGCTTCAGCATTACGGGCAGGCGGAAAAACTATTACCGTACAGCCGACGG
TAAAACCGAAGATGCCGTCTTAATGGAGAAAATATGTTAAGCGCGCGCTACCTCCACCTG
CACGAAGCTTTGGGTTTGGGTCCGATGTGGCTGAAACAGGCCGCCGCCGTCCTGCCGCCC
AAAAACACACCCGCACCCTCGGCACAGGCACGTCCCCAAACCGTCCGCGCCGCCCCGATC
CGCCCTTCCCAACCCCATAACGGTCAGGCGCGGCTCGAAACGATGAAAGCGTTGGAAACC
GCCGCCGTACCTACGCGCAAACCCGCGCCTGAAACCGAAACGCCTCTGCCCGGCCTTTCA
GACGGCATCGCCCCCGTTCCCGCCGCTTCGGGCATCACCAAGCTTGCCGTCGTCAGCCTT
TGCCCACCGATCGAGGATGCGGTTTACGGGCAACTGTTCCACGGCAAAGCAGGCATCCTG
CTCGACAACATACTCAAAGCCGTAGGACTGGATGCCGCCTATGTCCACAAAACCTGTTGG
~~GTGAAAAGCGGGGGCGTCGGCAACCCGATGCCGTCTGAACAGGCCGTCGCGAATGCGCTG~~
GGTCAAATCGCCCGCGAACTCGACGGCTGCCGCGCCCCGGCTGTCCTTTTCCTCGGGCAG
GCTTTTGTCCAGCCTGAACGGCAAACGATGATTGAAACTTTGTGCGGCAGCCGTCCCTTC
TTCATCATCGACCATCCCGCCCGGCTTTTACGCCAACCCGAACTCAAAGCCCGCGCCTGG
CAGGTGTTGAAACAGTTGAAACGCGCCTTGCGGCAAGGCGGCGGCAGTTGAAGCGCGCCG
CACGGGGCGGTAGAATCGCAACTGCGTCCCAATATCTGACAGAAAGCACAAAATGACCGA
TTTCCGCCAAGATTTCCTCAAATTCTCCCTCGCCCAAAATGTTTTGAAATTCGGCGAATT
TACCACCAAGGCAGGACGGCGGTCGCCCTATTTCTTCAATGCCGGCCTCTTTAACGACGG
CTTGTCCACGCTGCAACTGGCAAAATTTTACGCACAATCCATCATTGAAAGCGGCATCCG
ATTCGATATGCTGTTCGGTCCCGCCTACAAAGGCATTATTTTGGCGGCGGCAACCGCGAT
GATGCTGGCGGAAAAAGGCGTGAACGTCCCGTTTGCCTACAACCGCAAAGAAGCCAAAGA
CCACGGCGAAGGCGGCGTGTTGGTCGGCGCGCCGCTTAAAGGGCGCGTGCTGATTATCGA
CGACGTGATTTCCGCCGGCACATCCGTACGCGAATCGATCAAACTGATTGAAGCGGAGGG
TGCAACCCCCGCCGGTGTCGCCATCGCGCTCGATCGCATGGAAAAAGGCACGGGTGAATT
GAGCGCGGTTCAGGAAGTGGAAAAACAATACGGTCTGCCCGTCGCCCCCATCGCCAGCCT
GAACGATTTGTTTATTCTGTTGCAAAACAACCCCGAATTCGGACAGTTCCTCGAACCCGT
CCGAGCCTACCGTCGGCAGTACGGCGTAGAATAAAAACAAAGCATATGCCGTCCGAACCG
CCTTACGCCTCAGACGGCATCAAACCTGACACACACGAGGAAATACCATGCCCGCCTGTT
TCTGCCCCCACTGCAAAACCCGTCTCTGGGTCAAAGAAACCCAACTCAATGTCGCCCAAG
GCTTCGTCGTCTGCCAAAAATGCGAAGGACTGTTTAAAGCCAAAGACCATCTGGCAAGCA
CGAAAGAACCCATATTCAACGATTTGCCCGAGGCTGTTTCGGATGTCAAACTCGTTCACC
GTATCGGCACGCGCGCCATCGGCAAGAAACAGATTTCCCGTGACGAAATCGCCGGCATCC
TCAACGGCGGTACAACCCAGCCCGATATTCCGCCCGCAACCGCCGCCACCCCTGCTGCCG
CACCGCAGGTTACCGTACCGCCCGCCGCGCCCGCCCGTCAGGATGGGTTCAACTGGACGA
TTGCAACCCTGTTTGCCCTTATCGTCCTCATTATGCAGCTTTCCTACCTCGTCATCCTAT
GAGCGCGCCCGACCTCTTTGTCGCCCACTTCCGCGAAGCCGTCCCCTACATCCGCCAAAT

## Appendix A

GCGCGGCAAAACGCTGGTCGCCGGCATAGACGACCGCCTGCTCGAAGGTGATACCTTAAA
CAAGCTCGCCGCCGACATCGGGCTGTTGTCGCAACTGGGCATCAGGCTCGTCCTCATCCA
CGGCGCGCGCCACTTCCTCGACCGCCACGCCGCCGCTCAAGGCCGCACGCCGCATTATTG
CCGGGGCTTGCGCGTTACCGACGAAACCTCGCTCGAACAGGCGCAGCAGTTTGCCGGCAC
CGTCCGCAGCCGTTTTGAAGCCGCATTGTGCGGCAGCGTTTCCGGGTTCGCGCGCGCGCC
TTCCGTCCCGCTCGTATCGGGCAACTTCCTGACCGCCCGTCCGATAGGTGTGATTGACGG
AACCGATATGGAATACGCGGGCGTTATCCGCAAAACCGACACCGCCGCCCTCCGTTTCCA
ACTCGACGCGGGCAATATCGTCTGGCTGCCGCCGCTCGGACATTCCTACAGCGGCAAGAC
CTTCTATCTCGATATGCTTCAAACCGCCGCCTCCGCCGCCGTCTCGCTTCAGGCCGAAAA
ACTCGTTTACCTGACCCTTTCAGACGGCATTTCCCGCCCCGACGGCACGCTCGCCGAAAC
CCTCTCGGCACAGGAAGCGCAATCGCTGGCGGAACACGCCGGCGGCGAAACGCGACGGCT
GATTTCGTCCGCCGTTGCCGCGCTCGAAGGCGGCGTGCATCGCGTCCAAATCCTCAACGG
AGCCGCCGACGGCAGCCTGCTGCAAGAACTCTTCACCCGCAACGGCATCGGCACGTCCAT
TGCCAAAGAAGCCTTCGTCTCCATCCGGCAGGCGCACAGCGGCGACATCCCGCACATCGC
CGCCCTCATCCGCCCGCTGGAAGAACAGGGCATCCTGCTGCACCGCAGCCGCGAATACCT
CGAAAACCACATTTCCGAATTTTCCATCCTCGAACACGACGGCAACCTGTACGGTTGCGC
CGCCCTGAAAACCTTTGCCGAAGCCGATTGCGGCGAAATCGCCTGCCTTGCCGTCTCGCC
GCAGGCACAGGACGGCGGCTACGGCGAACGCCTGCTTGCCCACATTATCGATAAGGCGCG
CGGCATAGGCATAAGCAGGCTGTTCGCACTGTCCACAAATACCGGCGAATGGTTTGCCGA
ACGCGGCTTTCAGACGGCATCGGAAGACGAGTTGCCCGAAACGCGGCGCAAAGACTACCG
CAGCAACGGACGGAACTCGCATATTCTGGTACGTCGCCTGCACCGCTGACCGCAACGGAA
AGCCGCCGCAGAAATGCCGTCTGAACCCCGTTTCAGACGGCATTTCCCCGATTATATAGT
GGATTAAATTTAAATCAGGACAAGGCGACGAAGCCGCAGACAGTACAAATAGTACGGCAA
GGCGAGGCAACGCTGTACTGGTTTAAATTTAATCCACTATAAAGACCTGCCCAACCCTCA
AGGACCCCGATGAAATCCTACCCCGACCCCTACCGCCATTTTGAAAACCTCGATTCCGCC
GAAACGCAAAACTTCGCTGCTGAAGCGAATGCCGAAACGCGCGCGCGTTTTTAGAAAAC
GACAAGGCGCGCGCGCTTTCAGACGGCATTTTGGCGCAGTTGCAGGACACGCGGCAGATT
CCGTTTTGTCAGGAACACCGCGCGCGGATGTACCATTTCCATCAGGACGCGGAGTATCCG
AAGGGCGTGTACCGCGTGTGTACCGCGGCGACGTATCGTTCCGGCTATCCCGAGTGGAAA
ATCCTGTTTTCGGTGGCGGATTTCGACGAATTGCTTGGCGACGATGTGTATTTGGGCGGC
GTGTCGCACTTGGTGGAACAGCCCAACCGCGCGTTGTTAACACTGAGCAAATTGGGCAGC
GATACGGCGTACACGCTGGAAGTGGATTTGGAAGCAGGGGAGTTGGTCGAAGGCGGTTTT
CACTTTCCGGCAGGCAAAAACCATGTGTCGTGGCGCGATGAAAACAGCGTGTGGGTGTGT
CCGGCTTGGAACGAACGCCAGTTGACCCAATCGGGCTATCCGCGCGAAGTATGGCTGGTG
GAACGCGGCAAGAGTTTCGAGGAAAGCCTGCCTGTGTATCAAATCGGCGAAGACGGCATG
ATGGTGAACGCGTGGCGTTATCTCGATCCGCAGGGTTCGCCGATTGATTTGATTGAAGCG
TCGGACGGTTTTTACACCAAAACCTATTTGCGGGTCTCAGCCGAAGGCGAGGCGAAACCG
TTAAACCTGCCCAACGATTGCGACGTGGTCGGCTATCTGGCGGGGCATCTTTTGCTGACG
CTGCGCAAGGACTGGAACCGCGCGAACCAAAGCTATCCGAGCGGCGCGCTGGTGGCGGTG
AAGCTGAATCGGGGCGAACTCGGGGCGGCGCAGCTTTTGTTTGCGCCCGATGAAACGCAG
GCATTGGAAAGCGTGGAAACGACCAAGCGTTTTGTGGTGGCGAGCCTGTTGGAGAACGTA
CAAGGCCGTCTGAAAGCATGGCGGTTTGCCGACGGCAAATGGCAGGAAGTCGAATTGCCG
CGCCTGCCTTCGGGCGCGTTGGAAATGACCGACCAACCTTGGGGCGGCGACGTGGTTTAC
CTTGCCGCCAGCGATTTCACCACGCCGCTGACGCTGTTTGCGCTGGATTTGAACGTGATG
GAACTGACCGTCATGCGCCGCCAGCCGCAGCAGTTTGATTCAGACGGCATTAACGTGCAG
CAGTTTTGGACGACTTCGGCTGACGGCGAGCGCATTCCTTATTTCCACGTCGGCAAAAAC
GCCGCGCCCGACATGCCGACGCTGGTCTATGCCTACGGCGGTTTCGGCATTCCCGAATTG
CCGCATTATCTGGGCAGCATTGGCAAATATTGGCTGGAAGAGGGCAATGCCTTTGTATTG
GCGAACATCCGCGGCGGCGGCGAGTTCGGCCCGCGCTGGCATCAGGCGGCGCAGGGAATC
AGCAAACATAAAAGCGTTGATGATTTATTGGCAGTCGTGCGCGATTTGTCCGAACGCGGT
ATCAGTTCGCCCGAACACATCGGCTTGCAGGGCGGCAGCAACGGCGGACTGATTACTGCC
GCCGCCTTCGTGCGCGAACCGCAAAGCATCGGCGCGCTGGTGTGCGAAGTGCCGCTGACC
GACATGATCCGTTATCCGCTGCTCTCCGCCGGTTCAAGCTGGACAGACGAATACGGCAAT
CCGCAAAAATACGAAGTCTGCAAACGCCGGTTGGGCGAATTGTCGCCGTATCACAATCTT
TCAGACGGCATCGATTATCCGCCCGCGCTCATTACCACCAGCCTGTCCGACGATCGCGTC
CATCCCGCCCACGCGCTCAAGTTCTACGCCAAACTGCGCGAAACCTCCGCGCAATCTTGG
CTCTACTCGCCTGACGGCGGCGGCCATACCGGCAACGGCACCCAACGCGAATCCGCCGAC
GAACTCGCCTGCGTCTTGCTGTTTTTGAAAGAGTTTTTGGGCTAAGGGCGGGGGAGCGGC
ACTGCCGCCGCGAATGAAAAAAGGTCGTCTGAAACTGCTTTTTCAGACGACCTTTTTTAA
TGGTTGTTTCAAATCAAAATATCTATGCCGCCGGCCCCATCAGCACTTCTTCACATCCGA
AGGCAAAAATCCGTAATGCCGTCTGAACGCTTCGTTGAACCGTCCCGCGTGGCGGTAGCC
GCAAAAGTGCATGGCGGCTTGGACGGTGCTGCCGGATTCGATGAGGGCGAGCGCGTGTTC
CAGCCGCAGGCGGCGCAGGCATCCGGCGACGGTTTCGCCGGTTTGCGCTTTGAAATAGCG
TTTCAGGTAGCATTCGTTCAGTCCGACGCGGCGGGCGATTTCGGCGATGGTCAGCGGACG
GGCGAATTCGTGTTGCAGGATGTCGGCGGCTTCGTCTATGCGCCGACGGCGGTAACCGTT
GTCGTGGCGGCGGAAGGTGAAGCGCAATAATCGGGCGGAGAGTTCCAGCGCGGCGGCTTC
GTCGGCAAGCAGGCCGAAGCCGTCCGATTCGAACGGGCGTTGCAGCAGTGGGCAGGCCGC
CGCCGTCAGTGCCGCTGCGTTTTGCGCCAGCCGTTGCAGGGCGAATCGGCCTATTGTTTG
CGGCGAAAACAGGCGTTCGTCCAGCAAGCCTTCGTCGTGCCAGCGGCGCAGTTTTTCCAG
CGAAAAATCCAAATGCAGCGCGCACATGCCGCTGTTGTCGGGCAGCAGGGTTTCGGATAC
GTCCGCCAAATCGCCGCGTACCAGTCAGATTTCGCCGGCAGATGGGCGGTATTCCCTGCC
GCCCATTTGTAACCGGTTCTGCCCCGACACCATGACGAACAAGGCGCAGTTGTGGCTGAA
ATTGTGGATTTCGGTGGGAAACGCGCCCGTTCCGCCGCCGCGCATCCGCGACAAGGTGAT
GCCCGAATCGAAGCGGTTGATGCACATTTCCAGATGCAAACCGGGCTGTTTTGCCTGCGC
AATGAGCGCGCTGTCGGAACAGCCGTCCAACGCCCAGCCGGATTTATCGGAGCGGACATA

## Appendix A

GGTTTGGTACTGGCGGTAGATGGCGGCGGTGTTCATGATTGGATAGGAACGAGTTGTCTA
ACAAATGAATTAAATAGGAATTATTACCAATAATCAAGCGCAGGGATTGGTTGAAACGGA
AAAGGTCGTCTGAAAGGGTGTTTCAGACGACCTTTTCCGTATCGGGAATTTGTTTTGCCG
TATCGGGAATTTTGCGTTTTGCGGCGTGGTTTCTGCAGGTTGTTTGCTTAATAATAAACA
TTCTTATTCGTATGCAAAGGAACCGCACACCGTGAAACCGCGTTTTTATTGGGCAGCCTG
CGCCGTCCTGCTGACCGCCTGTTCGCCCGAACCTGCCGCCGAAAAAACTGTATCCGCCGC
ATCCGCATCTGCCGCCACGCTGACCGTGCCGACCGCGCGGGGCGATGCCGTTGTGCCGAA
GAATCCCGAACGCGTCGCCGTGTACGACTGGGCGGCGTTGGATACGCTGACCGAATTGGG
CGTGAATGTGGGCGCAACCACCGCGCCGGTGCGCGTGGATTATTTGCAGCCTGCATTTGA
CAAGGCGGCAACGGTGGGGACGCTGTTCGAGCCCGATTACGAAGCCCTGCACCGCTACAA
TCCTCAGCTTGTCATTACCGGCGGGCCGGGCGCGGAAGCGTATGAACAGTTAGCGAAAAA
CGCGACCACCATAGATCTGACGGTGGACAACGGCAATATCCGCACCAGCGGCGAAAAGCA
GATGGAGACCTTGGCGCGGATTTTCGGCAAGGAAGCGCGCGCGGCGGAATTGAAGGCGCA
GATTGACGCGCTGTTCGCCCAAACGCGCGAAGCCGCCAAAGGCAAAGGACGCGGGCTGGT
GCTGTCGGTTACGGGCAACAAGGTGTCCGCCTTCGGCACGCAGTCGCGGTTGGCAAGTTG
GATACACGGCGACATCGGCCTACCGCCTGTAGACGAATCTTTACGCAACGAGGGGCACGG
GCAGCCTGTTTCCTTCGAATACATCAAAGAGAAAAACCCCGATTGGATTTTCATCATCGA
CCGTACCGCCGCCATCGGGCAGGAAGGGCCGGCGGCTGTCGAAGTATTGGATAACGCGCT
GGTACGCGGCACGAACGCTTGGAAGCGCAAGCAAATCATCGTCATGCCTGCCGCGAACTA
CATTGTCGCGGGCGGCGCGCGGCAGTTGATTCAGGCGGCGGAGCAGTTGAAGGCGGCGTT
TAAAAAGGCAGAACCCGTTGCGGCGGGGAAAAAGTAGGGAGTCGTCTGAAAACGGAGCTT
CCGAAGGAAGCGGGGGGTTTCTGCGAAGCTAAAGTGCGGTTTCAACGAATTGAAAAGCAG
CCTGTATGTTGAAAATACCGCTCAAGCAAACCTACGGTTTGCCGCCCTCTCCCTAGCCCT
CTCCCACAGGGAGAGGGGATTGGGTTGCAGGCTGCCTTTAAGGTTTAGGCAAATTTTTAA
CTTCGTTGAAGCTGCGATTTCAGAAGCTCCGTTTTAGCTTCGCAGAAACTCCGCTTCCTT
CGAAAGCTCCGTTTTCAGACGACCTTTTGGAGTACCGCAGGCACACGCATCGAACGGCTG
AATCAAAGATTCAGACCGATGGCAGTCCGCACCCGAGTTTATGCGGCAAACAGCGAGGCT
ACGGCAACCCGCCCCCTCTCCCTGTGGGAGAGGGTTAGGGAGAGGGCGGTAAGCCGCAGG
CTTACATCAAAGCCGATAACGCTTCCGTTACAACTCCGCCCACTGAAAGCAGCCTGCAAC
GAAGCCAAAACGACAAACCGCATCGTAAACCACCCAACCCATAGGAGAACCCCATGCAAA
ACGAAACCATCAACCTGAAACAGCACCTTGCCGCCATCAAAGAATACTGGCAGCCCGAAA
TCATCAACCGCCACGGGTTCCAATTCCACTTGGTCAAACTTTTGGGCGATTACGGCTGGC
ATACGCACGGATACAGCGACAAAGTGCTGTTTGCCGTGGAGGGCGACATGGCGGTGGACT
TCGCCGACGGCGGCAGCATGACGATACGCGAGGGCGAGATGGCGGTCGTGCCGAAGTCGG
TGTCGCACCGCCCGCGTTCGGAAAACGGCTGCTCGTTGGTGCTGATTGAGTTGTCCGACC
CGTCCGAGGCCGTCTGAAAACGAAGTTTCCGAAGGAAGCTGAGTTTCTGCGAAGCTAAAA
GCAGCCTGCACCTTCAATCAATATGCCGAAAATACAACCCCACCGCACACCAACACACAA
AGGAAATCCCATGACACGCTTCAAATATTCCCTGCTGTTTGCCGCCCTGTTGCCCGTGTA
CGCGCAGGCCGATGTTTCTGTTTCAGACGACCCCAAACCGCAGGAAAGCACTGAATTGCC
GACCATCACCGTTACCGCCGACCGCACCGCGAGTTCCAACGACGGCTACACTGTTTCCGG
CACGCACACCCCGCTCGGGCTGCCCATGACCCTGCGCGAAATCCCGCAGAGCGTCAGCGT
CATCACATCGCAACAAATGCGCGACCAAAACATCAAAACGCTCGACCGCGCCCTGTTGCA
GGCGACCGGCACCAGCCGCCAGATTTACGGCTCCGACCGCGCGGGCTACAACTACCTGTT
CGCGCGCGGCAGCCGCATCGCCAACTACCAAATCAACGGCATCCCCGTTGCCGACGCGCT
GGCCGATACGGGCAATGCCAACACCGCCGCCTATGAGCGCGTAGAAGTCGTGCGCGGCGT
GGCGGGGCTGCTGGACGGCACGGGCGAGCCTTCCGCCACCGTCAATCTGGTGCGCAAACG
CCTGACCCGCAAGCCATTGTTTGAAGTCCGCGCCGAAGCGGGCAACCGCAAACATTTCGG
GCTGGACGCGGACGTATCGGGCAGCCTGAACACCGAAGGCACGCTGCGCGGCCGCCTGGT
TTCCACCTTCGGACGCGGCGACTCGTGGCGGCGGCGCGAACGCAGCCGCGATGCCGAACT
CTACGGCATTTTGGAATACGACATCGCACCGCAAACCCGCGTCCACGCAGGCATGGACTA
CCAGCAGGCGAAAGAAACCGCCGACGCGCCGCTCAGCTACGCCGTGTACGACAGCCAAGG
TTATGCCACCGCCTTCGGCCCGAAAGACAACCCCGCCACAAATTGGGCGAACAGCCGCCA
CCGTGCGCTCAACCTGTTCGCCGGCATCGAACACCGCTTCAACCAAGACTGGAAACTCAA
AGCCGAATACGACTACACCCGCAGCCGCTTCCGCCAGCCCTACGGCGTAGCAGGCGTGCT
TTCCATCGACCACAACACCGCCGCCACCGACCTGATTCCCGGTTATTGGCACGCCGACCC
GCGCACCCACAGCGCCAGCGTGTCATTGATCGGCAAATACCGCCTGTTCGGCCGCGAACA
CGATTTAATCGCGGGTATCAACGGTTACAAATACGCCAGCAACAAATACGGCGAACGCAG
CATCATCCCCAACGCCATTCCCAACGCCTACGAATTTTCCCGCACGGGTGCCTACCCGCA
GCCTGCATCGTTTGCCCAAACCATCCCGCAATACGGCACCAGGCGGCAAATCGGCGGCTA
TCTCGCCACCCGTTTCCGCGCCGCCGACAACCTTTCGCTGATTTTGGGCGGACGATACAC
CCGTTACCGCACCGGCAGCTACGACAGCCGCACACAAGGCATGACCTATGTGTCCGCCAA
CCGTTTCACCCCCTACACAGGCATCGTGTTCGACCTGACCGGCAACCTGTCTCTTTACGG
CTCGTACAGCAGCCTGTTCGTCCCGCAATCGCAAAAAGACGAACACGGCAGCTACCTGAA
ACCCGTAACCGGCAACAATCTGGAAGCCGGCATCAAAGGCGAATGGCTTGAAGGCCGTCT
GAACGCATCCGCCGCCGTGTACCGCGCCCGTAAAAACAACCTCGCCACCGCAGCAGGACG
CGACCCGAGCGGCAACACCTACTACCGCGCCGCCAACCAAGCCAAAACCCACGGCTGGGA
AATCGAAGTCGGCGGCCGCATCACGCCCGAATGGCAGATACAGGCAGGTTACAGCCAAAG
CAAAACCCGCGACCAAGACGGCAGCCGCCTGAACCCCGACAGCGTACCCGAACGCAGCTT
CAAACTCTTCACTGCCTACCACTTTGCCCCCGAAGCCCCCAGCGGCTGGACCATCGGCGC
AGGCGTGCGCTGGCAGAGCGAAACCCACACCGACCCTGCCACGCTCCGCATCCCCAACCC
CGCCGCCAAAGCCCGCGCCGCCGACAACAGCCGCCAAAAAGCCTACGCCGTCGCCGACAT
CATGGCGCGTTACCGCTTCAATCCGCGCGCCGAACTGTCGCTGAACGTGGACAATCTGTT
CAACAAACACTACCGCACCCAGCCCGACCGCCACAGCTACGGCGCACTGCGGACAGTGAA
CGCGGCGTTTACCTATCGGTTTAAATAAGGTCGTCTGAAAACGGAGTTTCTGCGAAGCTA
TAGTGGATTAACAAAAACCAGTACGGTGTTGCCTCGCCTTAGCTCAAAGAGAACGATTCT

# Appendix A

CTAAGGTGCTCAAGCACCAAGTGAATCGGTTCCGTACTATTTGTACTGTCTGCGGCTTCG
TCGCCTTGTCCTGATTTTTGTTAATCCACTATAAAAGCAGCCTGCACATTGAAAATGCCG
CCCAAGCAAACTTTCAGTTTGCCCGCCTCGTCCTAGCCCTCTCCCACGGGAGAGGGGATT
GGGGTGCAGGCTGCCTTTAAGGTTCAGGCAAATTTTAACTTCGTTGATACCGCGCTTTAG
CTTCGCAGAAGCTGCACTTTCAGACGACCTTTTGGAACACCACAGGTACACGCATTTAAG
GAATGCCGTCTGAAATGCCTGCCTCAATAACGCATCATGTTGCCGTCAATCTCGGCCGCC
CATGCATCGATGCCGCCCTGAAGGTTGTACAGGTTTTCAAAACCCGCGTCCGCCAAATAC
ATCGCCGTATGCAGGCTGCGGATACCGTGGTGGCAATACACCACAAGCGGCACATCATCC
GGCAGCTCGTTCTGCCGCAGCGGAATCAGATTCATCGGGATATGCAGCGCATTTGGCAGC
GAACAAACCGCCGTTTCTTCATCCGTACGCACGTCCAACAAACAAAACATCCGCCCTTCG
TCCATCCACGCTTTCAATTCCGCGGGCCCAAGTTGCACAATATCCATCGCACCCCCCAAA
AAAAACCAAGCAAAATGCCGTCTGAAGCCCCAAACCCGCTTTCAGACGGCATGACCTGTC
AACATCTTAAAAATCGAAACCGCCAAACGGATCGGCATCCTTATCATCCAAATGCGCCAC
CAAGGTATCGAACAGCACCTTCTCTTCAAACACATCGCCCCTGCGCGTAATCAAAAGCGC
GCGTTGAACAGGCTTGCGACCTACGATAACCACCATGCGTCCGCCATCTTTCAACTGTTC
TTTCAACACTTCAGGCACAAGGTTTACCGCACCGCCGACATAAACCGCATCAAACGGCGC
ACCTGCGGAAAGTTCGGTCAACCCGTTGTTTTGCACATAATCGATATTGTCCAAACCCAA
GCCGTCCAACACCGCTTTGGCGCGGTTTTGCTGTTCGACATCGATGTCGTCCGACACCAC
ACGACCAGCCAATTTTGCCAACAGCGCGGTCGCATAGCCCGAACCCGTGCCGATTTCCAA
AACCGTATCGTTTTTCGTCAGCTTCAAGCCCTGCGCCAGCCGCGCCACGACTTTCGGCTC
GAGCATCTTATGACCGTTGGCAAGCGGCAGCGCCATATCCGCATACGCCAAACCCTGCAA
GTCCTCATCGACAAAAAGCTCGCGCGGAATCTCCGCCAAAGCGTCCAACACATCAAAATC
CAATACATCCCACGGACGGATTTGCTGTTCGACCATATTGAACCGCGCTTTTTCAAAATC
CATTTAGTGCTCCGTCAAATAATTGTTCCAACAATGGCAGGCATTATAAAACCACACTCC
CGCCGCGCCAACTTCGGCACGCGCCGACAGCCCGTTTTGTCAGTCTCAAACCGCCTGACG
CGAAGCCTCAAACCGCTTCTCCAAAATCTTCGCCAGTTCGCCCAAATACAAAGCATCCGT
CTCATCAAACTGCGCCAAATGTTCGCTGTCCGCGTCCAACACGCCGATACAGCGGCCGTC
TGAAAACAGCGGCACGACAATCTCCGAACGTGACAAAGACGAACAGGCAATATGGTCGGG
ATGCGCGTTGACATCCTTAACAACCACCGTTTCACCCTTCGCCCAAGCCTGACCGCACAC
CCCGCGACCGAACGGAATCCGCGTACACGCCAAAGGCCCCTGAAACGGTGCCAAAACCAA
TTCGTCCGAACGCGTATCGACCAAATAAAAACCCACCCAAAACCAACCGAACGCCTCCTT
CAAAACCGCCGCCGTGTTCGCCAAATTCGCCACCCAATCCGCCTCGTCAGCCACCACAGA
CTCAATCTGCGGCAACACCTCCCGATAAAGCGCGGCCTTGTCCGAAGCCGAAAAATGAAG
CGCGTGCATCACATCTCCTATAGTTGCATACATATCAGGCGGCCATTATAAAACAGCCTG
CCCGAAACAACATTCCAAACCGCCCGGCCGGCCGCTTCAAGTTGCGAACCCGCCGCATAT
CCACTAAACTTCACGTTGCACCGCGCCACACGCGGCAGACAAAAAAACACGACACGGAGC
AAAAAAGATGTATCGCCAAATCGGAATGTGGGATCAAAAATGGGTCATCGGCAACTGGAA
AATGAACGGCCGGCTCCAAAACAACAACGCACTGATGCACCGCTTCCGCATCCACCCCAC
CGCCGAACGCGTCCTCATCGGACTCGCCGCCCCGACCGTTTACCTGCTGCAACTGCACAA
CGCCATGCAAATCGTTTTAAACAACCGCATCCTCACCTGCGCCCAAGACGTGAGCCGCTT
CCCCAATAACGGCGCGTACACCGGCGAAGTGTCCGCCGAAATGCTCGCCGACACCGGCAC
AGACATCGTCCTCATCGGACACTCCGAACGCAGCCTTTATTTCGGCGAAAAAAACGAAAT
CCAACGCCGCAAAATGGAAAACGTCCTCAACGTCGGACTCATCCCGTTATTGTGCGTCGG
CGAAAGCCTCGAAGAGCGCGAAGCCGGCAAAGAACACGAAGTCATCGCCCATCAGCTTTC
CATCCTGCAAGGGCTGGATACCAAAAACATCGCCGTCGCCTACGAACCCGTCTGGGCGAT
CGGCACCGGCAAAGTCGCCACCGTCGAACAGATTGCCGATATGCACGCATTCATCTACAA
AGAAATCTTGTCTTTGTGCGGAAGCGATGTTAAAATCCGCGTCCTTTACGGCGGAAGTGT
GAAAGCGGACAACGCGGCCGACATCTTCGCAGTACCTTATGTGGACGGCGCACTCGTCGG
CGGCGCGTCATTGTCGTACGACTCCTTTACCGCCATCATCAGTGCCGCACAAAATGCGTA
GAAAAATATGGAAGCCTTCAAAACCCTAATTTGGATTGTTAATATAATTTCCGCTTTGGC
CGTCATCGTGTTAGTATTGCTCCAACACGGCAAAGGCGCGGATGCCGGCGCGACTTTCGG
ATCGGGAAGCGGCAGCGCGCAAGGCGTATTCGGCTCTGCCGGCAACGCTAACTTCCTCAG
CCGCTCGACCGCCGTTGCAGCAACATTTTTCTTTGCAACCTGCATGGCTATGGTGTATAT
TCACACCCACACGACAAAACACGGTTTGGACTTCAGCAACGTACAACAAACTCAGCAAGC
ACCCAAACCCGTAAGCAATACCGAACCTTCTGCCCCTGTTCCTCAGCAGCAGAAATAACA
GTTTTTCAAATGCCGACATGGTGAAATTGGTAGACACGCTATCTTGAGGGGGTAGTGGCC
GTAGGCTGTGCGAGTTCAAATCTCGCTGTCGGCACCAACACACAAAAACGCCTGAAAATT
TCAGGCGTTTTTTGTTCAATCCCATCCAACGCTGTCAACCAATTCCAATCAATACAAGGA
TTTCAGGCTGATTGTTATCCTGCCGTCCCCCTTCCTGACAGTGCAATCCCGTCCAATCCG
CCCTAATTGAAGTAACCTAAAATTTACGGTATCTTTTGCGGTATCTGAAAAATACCTCGA
AAAAATACCGCAAAAATAAAGCTGAACGACCGCCAAATCAGGAATGCCAAGCGGAAAAGA
GCTTGCGGGGAATACTGCCAAGACGTAGGGAACAAGGGGGAAACCGTCCAAGATGCAGGG
CGGTTTTTTTTGGGTTTTTGGAAAAAACCTATACTAGGAAGCGATACCCTTAGTTGTTAC
CTTGTTACCGGGGAAAAGTTAGATAAATAAGCATATGAAATATAGTGAATTAAATTTAAA
TCAGGACAAGGCGGCGAGCCGCAGACAGTACAAATAGTACGGCAAGGCGAGCCAACGCTG
TACCGGTTTAAATTTAATTCACTATAAAATAAGAAAAAGATAAAAAATTGGTAACAAATG
CGGTAACAAATGGTAACGAATCGGTAACAACTTTTGGGGTTTTCCGGTTTTTCACCGTCT
TGGCAGTGGGAGCGTAGCGGAATGAAAAGCCAAAACGCACGGAACCGCGCCTATTTTGAG
CAGGAATGGCGGTTAAACCGCTTGGTTATATACGGGGAATAGGAAGACAGCGAAACGCGC
TAAAAACGCAATTTGAGCCATTAAAAGCGATTGATTAAAAAAATAATCAGGTTAGCCGCC
AGTGTTTCAGGCGGCATAAACGGAGAAATTGCGGGGCATAAAAAAGGCAGCTTGCCGTGT
TGTCTGTCTCTGGTATAATTCCAAGTATCACTAATCAACGGCTACACAATGCGGATATTC
AAAAACCAATGGATAGTGAAATTTGCCAAGAAGCACAAAATCAACGATTCCGAGCTGCTG
GAAGCGGTAGAGCGGGCGGATAACGGGCTGATAGACGCAGATTTAGGCGGCGGTGTGATT
AAGCAGCGCATAGCAAGGCAAGGACAAGGCAGAAGCGGCGGTTATCGCAGTCTGATACTG

## Appendix A

TTCAAACAGGCAGACAAGGCATTTTTTGTTTACGCCTTTGCCAAGAACGACAGGGAAAAC
ATTTCGGATAAAGAACTTGACGTTTACCGAAAAGCCGCCGCATATTATCTGAAATACACG
CGGGCAGAGCTGGCGGCTTTGAAAGAAGACGGCATTATCACGGAGATAGAATCATGAAAT
ACAAAAACGAGGCATTAGCCGCCATTCATGAAATGATGGAAGGGGCTTACAACATCGGCG
CAATCGACAAAAAGACCATGCGCGACTTTGACAAGTCCTGCCTGACCGAAATCAAACCGT
TGAGCGGCGGAGACATCAAGGCAATCAGGGAGAAGGAGGCACTATCGCAAGCCGCTTTCG
CCATCTATCTCAACGTGGGAAAAAATCACGTTTCGGCTTGGGAGCGGGGCGTTAAAAAGC
CGAGCGGCGCGGCGTTGAAGCTGCTGACCATCGTCAAAAACAAGGGCATCGAAGCCATTG
CGTAGCCGACTTGGCAAACGGCAAAATCAGCAAGTTCACAATAGACGCGCTGCTGAATAT
GCCTGCCAAGACAGGCAAGACCGCCGAACTGAATATCAGGGCGTAGCCGCATAAATGCCC
GACCGCATCAAACCAAGCCGAAACGGCGGCGGTGCAGACGACATAGCCCGACAGCAAGGC
ACGGCGCAGACGGGCGCGAAACCCGAAACATCACCGACCGCGAGGTACGGGGATTTTTTG
CGCCCGTTGCAGGGGGGGATTGGATTTAAGCGGCGCGGGCTTGAAGGCAAAACGGGTGGG
GCACAGAACTGTTTAAATGCAGTCTGAATCTCAAACGATTTCAGACGGCATTTTGAAACA
ATGGCTCAAATTCTCGATCCCCTTCCCTTAACGCCGACGTTTTTTATTAACGCGCCCCTT
ATTTCTGACACTTTGCTCATAAACCGGCATAACGGTCGGCAACAACCGTTTTAGATTTTC
TATACGGGCATTGTTTGTCGGATGAGTAGAGGTAATAGCATAAATAAAGCCGTTTTGGTC
GTTTTCCTGATTCATTTTTTCCCAAACCCTGACAGCGGCCGCCGGATGATAGCCTGCCTG
CGCCATCAACATCATTCCCCCCTCATCGGCTTCTTCTTCCAAGCTGCGGCTATAAGGCAA
GGTAAGACCGTACGTCCCCAAAATATCCATACCCAATCCGACCAATTCCGGATTAGTATC
CGGTTTTTTGTCTAATATAATCTGCGTGCCTATCTGCGCCGCCGTATTGGTCAAGATTTG
CTGCCCGACCTTATTTTTACCGTGTTCATGCAGGGCGTGCGTCATTTCATGCCCCATAAT
GGCGGCAATTTCGTCATCGGTCAGCTTGAGTTTGTCGACTATCCCCGTATAAAACGCCAT
TTTTCCACCGGGCATTGCCCACGCGTTCAGCTCATCGTTTTTGAAAACCGTCATTTTCCA
GTCAAACTTATGGCTGGTATTATTTGCCGCATCGGCATAAGGCAGCATACGTCGAAATAC
TGCCTGCACCCTGCGGGCTGTTCTGGATGTGGTATCGACATTGCCGGCAGACTTGTTTAA
CTCAACCGTTTTCATATAATCTTTGGCAGCCGCAGCGTTCATTGTGGCGGAATCATGACC
GTAAACATCAGCAACGACCGCACAAGCCCCCAATACCGAGATTACTGCCGACAGGCAGAG
TATCCGTTTAAAGGAAGGAAGGAAGGAAATTTCATATTTAGGTTTACTCCTTAAAAAATT
AAATTTCAAAAAAATGCCGTCTGAATCCAAAACGGATTTCGGACGGCATCTTAACATTGT
TTAATGTTTTTAAAAAGATTTACACCACGATGTTCTCCAGTCTGCCCGGTACGGCGATGA
TTTTCTTGGCAGGCTTGCCTTCTATGAATTTCACCGCGCCTTCAGCGGCGTATTCGGCAG
CTTCTTCAGCCGGTTTGTCGAAATCACGCATAAATTGCCAATAATTCTCCAACTTTTTTA
CGGCTGCTGCTGCCTTTTGCGGCAATATTGCGCTGAACTTCAACTGTTTTCAAAATGGCA
GAAGAATAAATATCCCTTGTGAATTCAGTATCATGATTTGAAATCAAAATACCTTGGGAG
TTGGGCGCAATTTATTGATTTTTTGTAAAGTCCGCGACCAATGAATTCGATCGTATTTTG
GTCGCGCAGAATTTGCAACTGTTGGCGGATTTTGTCTCTGATATGGTTGTTTTGGGGAAA
TTGGATGGATAGTTTGTTTTCAAATTCATACATTTGCGACAATGTGAATTCTTCGGGGAG
TTGGTCGATACATTTCATAACAGCCAGAAGCCAGCCTTTGCGCTCCGCATTTTGGTTGCG
TAAAAACAAATTGGATTGCCATTTTTTCAGAACGGTTTCGGGTTCGATAATGCGGGAATT
GTCTATTAAGAATATTTTGCCGCTTTCAGGCAAAGGGGCGAGATTGATAGAACACATAAT
GTGGTTCGGCCGGTTTTTAATGCCTTTATTTCTGGGAATAATCATATCCGGCGTGATGAA
ATGTTTGGGTACAAGCACCAATTGCCGTATGGAGTAATCCGCTTTTTTATATGCAAGAAA
GAAAAAGTTGGGGTTGGTATCTGACCGGATGCGCTCCAACATGGTGTGATATGCACCGTC
AGGCACGCTGTTGCCTATGGTTTTTTGATTTTTACTCTTTAATTCATATTGCTCGTGGCA
ATTTGGGCAAAAGAGGTCTGCAACAGGTTTGTTATTGGCAAATCTCTGCATCGGCTTGCT
TCCGCAACAGGGGCAGTAGCCGTTTTTTTCCAACCAAGCCTCGCTCATTACACGGATTTT
ATGGGTTGCTTTATTTTGTTGCTTTCCCAATTCGGTATCGAAAAATAAATTCATGTTTTG
GATTTTGAGATTTCAGTTATTCGGGGTTCGTCATGCAGACAACACAATCCACCTTAAAAA
GGCCGTCTGAAACCCTGTTTCCAAGTTTCAGACGGCCTTTATCCGTGTGGCTAAACCTTA
AAAGCGGTTAGACGACGATGTTCACCAGTCTGCCCGGTACGACGATGATTTTCTTCGCCG
GTTTGCCTTCCATGAATTTCACCGCGCCTTCGGTGGCGAGTGCGGCGGCTTCGAGGTCGG
CTTTGGATGCGTCGGCGGCAACAGTGATTTTGCCGCGCAGTTTGCCGTTGACTTGAACCA
TCACTTCGATTTCGGATTTGACCAAGGCGGCTTCGTCGACTGTCGGCCAGCCTGCTTCCC
ACAGTTTCGCGCCGTTCAATTCGCTCCACAGGGTTTCGCAGATGTGCGGCACGATGGGCC
ACAACAGGCGTACGGCGGTTTCCAATACTTCTTGGGCGACGGCGCGTCCTTGTTCGCCGC
CGGTGTCGGTTTTGTCGTATTGGTTGAGCAATTCCATCACGGCGGCGATGGCGGTGTTGA
ACTGCTGGCGGCGGCCGTAGTCGTCGCTGACTTTGGCAGTGGTCGCGTGCAGTTTGTGGC
GCAGGTCTTTGAGTTCTTTAGACAAACCGTCTTGGCTGCCTGCGAACGCTTTGACCGCTT
CGCCTTGCTTCAAGTATTCGTAAACGGTACGCCACAGGCGGCGCAGGAAGCGGTGTGCGC
CTTCGACGCCGCTGTCGCTCCATTCGAGGGACTGTTCGGGCGGTGCGGCGAACATCATAA
ACAGGCGGGCGGTGTCCGCGCCGTAGGCGTTAATCAGTTCTTGCGGATCGACGCCGTTGT
TTTTGGACTTGGACATTTTTTCCGTGCCGCTGATGACGACGGGCAGCCCGTCGGCTTTGA
GGACGGCGGAAATGGGGCGGCCTTTGTCGTCGAACGTCAGCTCGACATCGGCGGGGTTGA
TCCAATCTTTGCCGCCTTTGTCGTTTTCGCGGTAGTAGGTTTCGCAAACGACCATGCCTT
GCGTCAGCAGGCGTTCAAACGGTTCGTCAACATTGACTAGACCTTCGTCGCGCATCAGTT
TGGTGAAGAAACGCGCGTACAAGAGGTGCAAAATCGCGTGTTCGATGCCGCCGATGTATT
GGTCGACCGCGCCCCAGTATTTCGCGGCGGCAGGATCGACCATGCCGTCTGAAAATTTTG
GCGACATGTAGCGGAAGAAATACCAGCTCGATTCCATGAAGGTGTCCATGGTGTCGGTTT
CGCGTTTCGCCGCGCCGCCGCAGCATGGGCAGGCAGTTTCGTAAAACTCGGGCATTTTTG
CCAGCGGCGAACCCATGCCGTCGGGTACGACGTTTTCAGGCAAAACGACCGGCAATTGGT
CGGCAGGGACGGGTACGTCGCCGCATTGTTCGCAATGGACGATGGGAATCGGGCAGCCCC
AGTAGCGTTGGCGCGAAATGCCCCAGTCGCGCAGGCGGTATTGGGTTTTCGGTTCGCCCG
CGCCTTGGCTTTGCAGCTTGGCGGCGACGGCGTCGAATGCCGTCTGAAAATCGAAGCCGT
CCAAGTCGCCGCTGTTGACCAATACGCCGTTTTCTTTGTCGCCGTACCATTCTTGCCATT

## Appendix A

GGTTTTCGTCAAATGCGTTGTCGCCGACGGCAATGACTTGTTTTTTCGGCAGATTGTATT
TGGTGGCGAACTCAAAATCGCGTTCGTCGTGCGCCGGAACCGCCATCACCGCGCCGTCGC
CGTAGCCCCACAATACATAGTTGGCAATCCACACTTCCAGCTTGTCGCCGTTGAGCGGGT
TGACGACGTAGCGGCCGGTCGGCACGCCTTTTTTCTCCATCGTCGCCATATCGGCTTCGG
CAACCGAACCGGCTTTGCATTCGGCAATAAATGCCTGCAATTCGGGTTTGTCGGCGGCTG
CGGCGGCTGCCAGCGGATGCTCGGCGGCAACGGCAACATAAGTCGCACCCATCAGCGTGT
CGGGGCGGGTGGTATAAACTTGCAGGAATTTCGCGTAATCGCCTTCCAAGCCTTGTTTGC
TGTCGTCTGAAACGGCGAAGCGCACGGTCATACCGCGCGATTTGCCGATCCAGTTGCGCT
GCATGGTTTTGACTTGTTCCGGCCAGTGTTCCAGCTTGTCCAAGTCGTTGAGCAGCTCTT
CGGCGTAATCCGTGATTTTGAAGTAATACATCGGGATTTCGCGTTTTTCGATCAATGCGC
CGGAACGCCAGCCGCGTCCGTCGATGACTTGCTCGTTGGCAAGGACGGTTTGGTCGACAG
GGTCCCAGTTTACCGTGCCGTTTTTGCGATAAACGATGCCTTTTTCAAACAGCTTGGTAA
ACAGCCATTGTTCCCAGCGGTAGTATTCGGGTTTGCAGGTTGCGGTTTCGCGCGCCCAGT
CAATCGCAAAACCTAGGCTTTTGAGCTGGGTTTTCATGTATTCGATGTTATCGTACGTCC
AAGCGGCAGGGGCGACGTTGTTTTTCATCGCCGCGTTTTCCGCCGGCATGCCGAACGCGT
CCCAACCCATAGGCTGCATGACGTTGAAGCCGTTTAAAAGTTTGAAGCGGCTCAATACAT
CGCCGATGGTGTAGTTGCGCACATGCCCCATGTGCAGCTTGCCGCTGGGATAGGGGAACA
TGGAGAGGCAATAATATTTGGGTTTGGAAGCGTCTTCGGAGACGTTGAAAATACGGGCGT
CGTCCCATTTTTTCTGCGCCGCAGGCTCAATGGCGGCGGGCCGGTATTGTTCTTGCATAG
TCATTCTGTTTTCGCTTAAAAACGTTGGAAAAATAAAGTCGGCATCAATTATAACAGGTT
GCCGGAAGCGGCGAATCGGCAGATTGCCGGCAGGATGCGTAAATTCGCACGCGCATTATT
CCGTATGCCGTACAAATACACCGCGTTTATTGATACGCACGTTTTTTATGCTAATATTAC
AAACCAAAATCAAATGTTTAAAACTCTCCTGATGCGGCTCTTCCGAACAAAAGGCAGACG
GGCATCGGGTAAAAGAGGATTCTGCATATGAAAATCAAACAAATCGTCAAACCGGGCTTG
GCAGTATTGGCGGCGGGCGTTCTGTCTGCCTGCGCAACCAAAAGCAACGTCAAAGCCGAC
GGCACGACCGACAATCCGGTTTTCCCGAAACCCTATTCCGTAACGCTCGACAACAAGCGC
GGCACATTCCCGACTTATGACGAACTGGATCAGATGCGCCCCGGCCTGACCAAAGACGAC
ATCTACAAAATCCTGGGCCGCCCGCATTACGACGAAAGTATGTACGGCGTGCGCGAATGG
GATTACCTGTTCCACTTCCATACCCCGGGCGTAGGTATCGACCCTGAAAACACTTCCGGC
GTAGAAGATGTTACTACCTGCCAATACAAAGTGATTTTCGATAAAGACAAATTTGCCCGC
AGCTTCTACTGGAACCCCGTCTTCCCGAAAGATGCCGCCTGTCCGCCGCCCGCACCCAAA
GCCGAGCCGCAAGTCATCATCCGCGAAATCGTGCCGGCAAAACCGAAACGTATCCGCCAA
TAATCCGACATGCCGTTCCGCCTGTTTTTAGGGATATTATGCGGCCTGTCAATGGTTGCC
CCCGTATATGCACAGGGGCAGCCGGATACGGTCGGCGACTTTATCCAAAAGAAAAAAGTC
ATCGTCGATACATCCAAAGCGGAACTCTGTTTCGCTGACGACCGTCAGTGCCACCCCGTC
CTCATCGGTGTTGCCACGCCCAAGGGGACGTTCGGGCTGACGCTGAACAGTACCGACAAG
CCCGGATACGGCGGCGAAGTCATCGGTTTCAAGCAGGAGGGTGATTTTCTTTTCGCCCTG
CACCGCGTTTGGAATCAGATACCGTCGGAAAGGCGGAACGAACGCATCGCCTCCCCGTCC
GTGTCCGACAGGATTATGACCAACGGCTGCATCAACGTCAGCGATGCGGTGTACGAAAAA
CTGCGTCATTATTTTGTGTTGGAAGTGATTTGAAACAGACGGATACCGCACGCGCCGGTA
TCTGTTTTCACATTGCCCCGATGCCTGAAACAGACTGTCCGCCACGTCATGCCGTCTGAA
ACCGGCGCAGATGCCGCCAAGCCTTCAGACGGCATTGCCTGCCCGCTCCGACCGAACAAC
AACCATCTTTGGGAGAACCTTATGCCCGAACAAAACCGCATCCTCTGCCGCGAACTGAGC
TTGCTGGCATTCAACCGCCGCGTGTTGGCGCAGGCGGAAGACCAAAACGTCCCCCTTTTG
GAACGCCTGCGCTTCCTGTGCATCGTTTCATCCAACCTCGACGAGTTTTTCGAAGTCCGT
ATGGCGTGGCTGAAGCGCGAACACAAACGCTGCCCGCAGCGCAGGCTGGACAACGGCAAA
ATGCCGTCTGAAACCATCGCCGACGTTACCGAAGCGGCGCGCTCCCTGATACGGCACCAG
TACGACCTGTTCAACAACGTCCTTCAGCCCGAGCTGGCACAAGAAGGCATCCATTTTTAC
CGCCGCCGAAATTGGACAGACACACAGAAAAAATGGATTGAAGACTATTTCGACCGCGAA
~~TTGGTGGGGATCCTGACCCCCATCGGACTCGACCCTTCCCACCCCTTCCCGCGCCCGCTG~~
AACAAATCGCTCAACTTCGCCGTCGAACTCGACGGCACAGACGCGTTCGGCAGGCCTTCG
GGGATGGCGATTGTGCAGGCACCACGCATCCTGCCGCGCGTTGTTCCCCTGCCGTCCGAA
CTGTGTGGCGGCGGACACGGCTTCGTCTTCCTCTCCTCCATCCTGCACGCCCACGTCGGA
AAACTCTTCCCGGGCATGAACGTCAAAGGCTGCCACCAGTTCCGCCTGACGCGCGACAGC
GACTTGACCGTTGACGAAGAAGACCTGCAAAACCTCCGCGCCGCCATTCAAAACGAGTTG
CACGACCGCGAATACGGCGACGGCGTGCGGCTCGAAGTCGCCGACACCTGTCCCGCCTAC
ATCCGCGACTTTCTGCTCGCGCAATTCAAACTGACCGCCGCCGAACTCTATCAGGTCAAA
GGCCCGGTCAACCTCGTGCGCCTCAACGCCGTCCCCGACCTAGTCAACCGCCCCGATTTG
AAATTTCCCACACACACGCCGGGCAGACTGAAAGCCTTGGGCAAAACCGCGTCCATATTC
GATTTGGTGCGCCAATCGCCCATCCTGCTGCACCACCCCTACCAATCGTTCGACCCCGTT
GTCGAAATGATGCGCGAAGCCGCCGCCGACCCCGCCGTGCTTGCCGTCAAAATGACGATT
TACCGCACCGGCACGCGTTCCGAACTCGTCCGCGCCCTGATGAAGGCGGCACTCGCCGGC
AAACAAGTAACCGTCGTCGTCGAACTGATGGCGCGTTTTGACGAAGCCAACAACGTCAAC
TGGGCGAAGCAGCTCGAAGAGGCGGGCGCGCACGTCGTGTACGGCGTGTTCGGCTACAAA
GTCCACGCCAAAATGGCACTGGTCATCCGCCGCGAAGACGGCGTGCTCAAACGTTACGCC
CATCTCGGCACGGGCAACTACCACCAAGGCACATCGCGCATCTACACCGACTTCGGCCTC
ATTACCGCCGACGAACAAATCACCGCCGATGTGAACATATTGTTTATGGAAATCACAGGT
TTGGGCAAACCCGGGCGGCTGAACAAACTCTACCAAAGTCCGTTTACCCTGCACAAAATG
GTTATCGACCGCATCGCACGCGAAACCGAACACGCAAAAGCCGGCAAACCGGCGCGGATT
ACCGCCAAGATGAATTCGCTCATCGAACCGACCGTCATCGAAGCCCTGTATCGGGCAAGC
GCGGCAGGCGTACAAATCGATTTGATTGTGCGCGGTATGTGCACCTTGCGCCCGGGTGTA
AAAGGCTTGTCCGAAAACATCCGCGTCCGCTCCATCGTCGGCAGGCAGCTCGAACACGCG
CGCGTGTATTACTTCCATAACAACGGCACGGACGATACCTTTATCTCCAGCGCGGATTGG
ATGGGGCGCAACTTCTTCCGCCGCATCGAAACCGCCACGCCGATTACCGCGCCCGAACTC
AAAAAGCGCGTTATACATGAAGGACTGACCATGGCACTGGACGACAACACCCACGCGTGG

## Appendix A

CTGATGCAGCCCGACGGCGGCTATATCCGCGCCGCACCTGCCGAGGGCGAATCCGAAGCC
GACCTGCAAAACGATTTGTGGACACTGCTCGGAGGCTGACCCGCACCGCCCCAATCAAAA
ACCATGCCGTCTGAAACCTTTCCGTTTCAGACGGCATGGTTTTACAGCAATCTAAACAGG
GCGGACCGGAGTCAAAAACACACCTTCGCCATTCCTGCACAAGCACTTCCCCTATACGCT
CCCAACCCCAAGCCGCCGCATTCCAGACGGCATTATAGTGGATTAAATTTTAGGGGCTGT
ACTAGATTAGCAGATATGTTACCCTCGAAATATGAAGATAACGCACTGCAAATTAAAGAA
AAAAGTACAGAAAGAACTGCTCCGTTTTTTGTGCTGGAAGTTACCGCCCGTTCTGCCGCC
GATATTTTGGGTATCCATCCCAATTCGGCAGCACTGTTCTACCGTAAAATCCGCACGGTT
ATCAACCATCATTTAGCCTTGGCTGCCGATGAGGTTTTTGAGGGCCCTGTCGAGCCGGAC
GAAAGCGATTTCGGCGGACGGCGTAAAGGCAGACGTGGTCGCGGTGCGGCAGGAAAAGTG
GTTGTCTTCGGCATTCTGAAACGCAACGGACGGGGCTATACCGTTGTCGTAGATAATGCC
AAGTCTGAAACGTTACTCCCTGTCATCAAGAAGAAAATCATGCCGGACAGCATTGTTTAT
ACCGATAGTCTGAGCAGCTGCGACAAGTTGGACGTGAGCGGTTTTATCCATTACCGCATC
AACCATTCCAAGGAGTTTGCAGACCGTCAGAACCACATTAACGGCATTGAGAATTTTTGG
AATCAGGCAAAACGCGTCTTGCGAAAATTATAGTGGATTAACAAAAATCAGGACAAGGCG
ACGAAGCCGCAGACAGTACAAATAGTACGAAACCGATTCACTTGGTGCTTCAGCACCTTA
GAGAATCGTTCTCTTTGAGCTAAGGCGAGGCAACGCCGTACTGGTTTTTGTTCATCCACT
ATACCTTTCCGACAGCCGAACAAAACCCCGAATCCGTCTGCACGGTTCGGGGTATATCTC
CAATACGGGCATCGTGTTCCGGAAAACCGTCAAATCCGCATCGGCATCACAATATATTTG
AAATTCGGATTGTTCGGCACGGTAAACAGCGTCGAGCGGTTGGCATCGCCGAAGGCAAGC
TGCATATCGTCGGAATGGATGTTGCGCAACACGTCCATCAGATAGCCGATATTGAAACCG
ACTTCGAGTTCGCCGCCCTGATAGGCGATTTCGATTTCTTCGCGCGCTTCTTCCTGCTCG
TTGTTGCTGCACACAACGCTCAACAGGCCGGGTTGCAAAAACAATCGCGCACCGCGGAAT
TTTTCATTGGCAAGAATCGATGCACGTTCCAACGCGCCCAACAATTCTGCCCTCGACAAC
ACGAAAATCTTGTCGTTGTCCAAAGGAATCACGCGGTTGAAATCGGGGAATTTGCCGTCG
ATGACCTTGCTGACGATGGTCGTGCCGTTGCATTGGAAACGCACCTGTTTGTCCAGCAGC
TCGATTTGAATCGGATCGTCGGGGTTGTTCAACAGTTTGAACAGTTCCAGCACCGTTTTG
CGCGGCAAAATCACTTCGGCGCGCGGCAAATCCGCATCAATCGCGCAGGCTGCATAGGCA
AGGCGGTGTCCGTCGGTCGCCACAAGGCGCAACTGGCTGCCCTCAACCTGCATCAGCAGA
CCGTTGAGATAATAGCGGATGTCCTGCACCGCCATGCTGTACTGCACTTGCGACAGCATG
GTTTTGAAACGCTCCTGCTCCAGCGAGAAAGTCGCGCTGATGTCCTCGCCGACATTCATC
ATCGGAAAATCGGCGGCAGGCAGGGTCTGCAGGGCAAAACGCGATTTGCCCGCCTTCAGC
GTCAGACGGCTGTCGTCCCAATCCAGCGACACCAGCGCACCGGCAGGCAGCGCGCGCAAA
ATATCCTGAAATTTCTTGGCATTGGTGGTGATGCGGAAGTCGCCCGCGCCGCCCTCGGGA
CCCGCAGTGTCGATTTGGATTTCCAAATCGGTTGCCAAGAGTTTGGTCTGACCGCCTTTT
CCCTCAATCAGGACGTTGGACAGGATGGGCAGGGTGTGGCGGCGTTCGACGATGCCGGTA
ACGGCTTGCAACGGCTTGAGCAGGCTGTCGCGCTCGGCTTGTAAAATCAACATGTTCGCT
CCTTTAAATCGGTTTGTATAGTGGATTAAATTTAAATCAGGACAAGGCGACGAAGCCGCA
GACGGTACAAATAGTACGGAACCGATTCACTTGGTGCTTCAGCACCTTAGAGAATCGTTC
TCTTTGAGCTAAGGCGAGGCAACGCCGTACTGGTTTAAAGTTAATCCGCTATATCTTTAC
CCTTCGGACGGCATGGGCAATATCATGTCGTCTGAAAACGTTTTCCATCAGTTTTGAATC
AGAATCAGCAGCTTTTCATAATCCTGAGCCAATTCCGGATCTTCTTCGCGCAGTTTCGCC
ACTGCCCTGATGCCGTGCATAACGGTCGTATGGTCGCGCCCACCAAACGAATCGCCGATA
GACGGCAGGCTCAAAGTAGTCAGTTCTTTGGTCAGGCTCATCGCCACCTGGCGCGGACGG
GCAATGTTTCGTGTCCGTTTCTTACCGAGCACATCGCTGATTTTGATGCGGTAATATTTC
GCCACCGCATCGATGATGATGTCGGCGGTGATGACTTTGTGCTTCTCGGCAATAATGTCC
TGCAAAGCGGTACGCGCCAAATCGATGTCGATGACGGGACGGTTCATAAAGCGGCTGCTC
GCTCCGACACGATTAAACGCGCCTTCAAGCTCGCGCACGTTGGAACGGATCAGATTGGCA
ATGAACAGCGCGGCTTCGTCTTCGATACTGATGCCCGCCGCTTCCGCCTTTTTCTGCAAA
ATGGCGATGCGCATTTCCAATTCGGGCGGCTCGAGTTCCAAAGTCAGTCCCCATGAAAAA
CGGGATTTGAGGCGGTCGTCCATGCCTTCGATTTTCGCAGGCAACACATCGCAAGTGAGG
ATGAGCTGTTTTTTCTCGTTGTGGAAATGGTTGTACAGATAGAAAAACTCTTCCATCGTA
CGGTCTTTGCCTTTGATGAACTGGATGTCGTCGATAATCAGCAGGTCGTATTGCTTGTAT
TGCTGCTTGAACACGTCGTAAGTGTTGTTGCGAACCGCCTTCATAAAGCTGCGGATATAG
TCATCCGAATGCATATAGCGCACTTTGGCATCGGGACGGTTTTTCAGCAGCTCGTTGCCG
ACCGCCTGCACAAGGTGGGTTTTGCCCAAACCCGTGCTGCCATAGAGGAAGAACGGGTTG
TAACTCTGCCCCGGGCTTTCCGCAATCGCCTGCGCCGCAGCCGCCGCAAGGCGGTTGCCC
TTACCTTCTACCAACGTATCAAACGTGTAATCCGGAGACAGGTTGGTCTGCTCGTAACGC
GCCTCTTCCGCATCGCGCTGCACGTCCGTCCGTGCTTTGGCAACTGCCACCGATTCCGGC
CGGGAAGCAGACCCGGCAGCCTGACGCGGCTCGTGCGGCAGGTTTTTCATACGTTCCGCC
AAAATATCCGCCGCCGTTTTCGACGCAGCGGGTTTGACAGGCTCTTCAGACGGCAGCTCG
TCCAACAGAACCTCCTGCACGGGCATTCCCTCCGACACCGCATGCAAGGACGGCTCGGCA
GGTTCGACAGCACCTTCAACCGCCGCCATCTCATAACGCACGCCTTCTCCCGGTTTGAAT
ACGAAGGCGGAACGGCCGGCAGCCAACTCTTCCCTCACCGCTTCTATTTTTCCGGCAAAC
TGGCTCTTGAGCATATTGCAGGCAAACTGGTTCTTGCCGTACACCACCCATACGCCACCC
TCCTCACCAACGGTAAGGGGCGCAATCCATTGCGCAAACTGCCCTTGAGGCAACATATCG
TGAAGACGGCGGAGGCACAGCGGCCAAAACTCTGCTAATGTCATGGATAGGCTCGAATCG
GTAAAAATGAAATCGAAAACAAAGAAAATATAATATTTTCAAAAAGAAAACAAATCTGTT
GAACGCACATCGGTTCAAAACGCGACTGCCCGATTATACCGACTCACGAATATTTTATCC
ACAACCCGTGCAAAAATTTATCCACAGAAAGGCGGCGGAAATCCGCAGGCAATCGGGCAA
TCTTCCTGCAAAGTTTCTATATTGATTGACAAAAGCGGCAAATTGGAGTGTAATTCACGG
TTTAATTATCTACCCATTCTATTTTAGGAAACATCATGAAACGCACTTATCAACCTTCCG
TTACCAAACGCAAACGCACCCACGGCTTCCTGGTGCGCTCCAAAACGCGCGGCGGCCGCG
CAGTATTGGCCGCACGCCGTGCCAAAGGCCGCAAACGCCTGGCGGTATAATTTTGGACTA
CCGCTTCGGAAGGCAGTACCGCTTGTTGAAAACGGATGATTTTTCATCCGTTTTTGCATT

## Appendix A

CAGAAACCGCCGCAGCCGCGACCTGCTGCAAGTTTCGCGCTCAAACGGCAACGGGCTGGG
CCATCCCCGCATCGGTCTGGTGGTCGGCAAAAAAACCGCCAAACGCGCCAACGAACGAAA
TTATATGAAGCGCGTTATCCGCGACTGGTTTAGATTGAACAAAAACCGGCTGCCGCCGCA
GGATTTCGTCGTGCGCGTCCACCGTAAATTCGACAGGGCTACCGCAAAACAGGCAAGGGC
GGAACTGGCACAACTCATGTTCGGCAACCCCGCAACCGGATGCAGGAAACAGGCATGATC
AGAACGGTACTCTGCAGGCAAGGTTCAGACGGCAACGGGTTTCCCATACAAGGAACATCC
CGATGAACTTCCTATTGTCCAAACTCCTGCTGGGACTGATACGGTTCTACCAATATTGCA
TCAGCCCGCTGATTCCGCCGCGCTGCCGTTATACGCCGACCTGTTCGCAATACGCGGTCG
AAGCGGTCAAAAAATACGGCGCATTCAAAGGCGGCCGGCTCGCCATCAAGCGCATTGCAC
GCTGCCACCCTTTCGGCGGACACGGACACGACCCCGTTCCCTGACCCGACGCAATATTCA
AATTGCACGCTTTCCTTTTATTTCCCATCGGTTTCTATATAATGCCGTCTGAAGCTTCGG
GCAGGCGGCACGACCGCCGGGTATGAAGCCCGCCCTTATTCCCCGTCTATCGGAACACGC
AACCTGCGGCATTTCCGACCATTCAGGAAACTCTTATGGATTTTAAAAGACTCACGGCGT
TTTTCGCCATCGCGCTGGTGATTATGATCGGCTGGGAAAAGATGTTCCCCACTCCGAAGC
CCGTCCCCGCGCCCCAACAGGCAGCACAACAACAGGCCGTAACCGCTTCCGCCGAAGCCG
CGCTCGCGCCCGCAACGCCGATTACCGTAACGACCGACACGGTTCAAGCCGTCATTGATG
AAAAAAGCGGCGACCTGCGCCGGCTGACCCTGCTCAAATACAAAGCAACCGGCGACGAAA
ATAAACCGTTCATCCTGTTTGGCGACGGCAAAGAATACACCTACGTCGCCCAATCCGAAC
TTTTGGACGCGCAGGGCAACAACATTCTAAAAGGCATCGGCTTTAGCGCACCGAAAAAAC
AGTACAGCTTGGAAGGCGACAAAGTTGAAGTCCGCCTGAGCGCGCCTGAAACACGCGGTC
TGAAAATCGACAAAGTTTATACTTTCACCAAAGGCAGCTATCTGGTCAACGTCCGCTTCG
ACATCGCCAACGGCAGCGGTCAAACCGCCAACCTGAGCGCGGACTACCGCATCGTCCGCG
ACCACAGCGAACCCGAGGGTCAAGGTTACTTTACCCACTCTTACGTCGGCCCTGTTGTTT
ATACCCCTGAAGGCAACTTCCAAAAAGTCAGCTTTTCCGACTTGGACGACGATGCCAAAT
CCGGCAAATCCGAGGCCGAATACATCCGCAAAACCCCGACCGGCTGGCTCGGCATGATTG
AACACCACTTCATGTCCACCTGGATTCTCCAACCTAAAGGCAGACAAAGCGTTTGCGCCG
CAGGCGAGTGCAACATCGACATCAAACGCCGCAACGACAAGCTGTACAGCACCAGCGTCA
GCGTGCCTTTAGCCGCCATCCAAAACGGCGCGAAAGCCGAAGCCTCCATCAACCTCTACG
CCGGCCCGCAGACCACATCCGTCATCGCAAACATCGCCGACAACCTGCAACTGGCCAAAG
ACTACGGCAAAGTACACTGGTTCGCCTCCCCGCTCTTCTGGCTCCTGAACCAACTGCACA
ACATCATCGGCAACTGGGGCTGGGCGATTATCGTTTTAACCATCATCGTCAAAGCCGTAC
TGTATCCATTGACCAACGCCTCTTACCGCTCTATGGCGAAAATGCGTGCCGCCGCACCCA
AACTGCAAGCCATCAAAGAGAAATACGGCGACGACCGTATGGCGCAACAACAGGCGATGA
TGCAGCTTTACACAGACGAGAAAATCAACCCGCTGGGCGGCTGCCTGCCTATGCTGTTGC
AAATCCCCGTCTTCATCGGATTGTATTGGGCATTGTTCGCCTCCGTAGAATTGCGCCAGG
CACCTTGGCTGGGTTGGATTACCGACCTCAGCCGCGCCGACCCCTACTACATCCTGCCCA
TCATTATGGCGGCAACGATGTTCGCCCAAACTTATCTGAACCCGCCGCCGACCGACCCGA
TGCAGGCGAAAATGATGAAAATCATGCCGTTGGTTTTCTCCGTCATGTTCTTCTTCTTCC
CTGCCGGTCTGGTATTGTACTGGGTAGTCAACAACCTCCTGACCATCGCCCAGCAATGGC
ACATCAACCGCAGCATCGAAAAACAACGCGCCCAAGGCGAAGTCGTTTCCTAAATGCCGC
AGCATGAAAAATGCCGTCTGAAACCTGTTCAGACGGCATTTTTATTGCCCACCCCCTATC
GGGGCGGAAATCTTCAACCCGCATACATCACAAAAATCGTCGGGCGTTTTTTCAGATTGG
GCATTTCTTTTCTTTTTCGCCACTGCACGATTGTTTGACTGATGATTTCCTGTGTCGGCA
AGGTCAAATCCGTAGCCGTGCATAAACGCGTTTCAGGATGCAGGTTTTCCACCGCATCGG
CAAGCAGCGCATCATTGCGGTAAGGCGTTTCAATAAAAATCTGCGTCTCGCCGCACTGGC
GCGAACGCTGTTCCAAAGCCCGAAAAGCCTGAATCCGCTCGTTTTTTTCAGACGGCAGAT
AGCCTTTAAACGCAAAACTCTGCCCGTTCGCACCCGAAGCCATCAAAGCCAGCAGCAGGC
TGGAAGGCCCGACCAGCGGACGCACTTCAAAACCGTGTTTATGCGCCAATGCCACCAAAT
TCGCACCCGGATCGGCCACAGCCGGGCAACCCGCCTCACTGACAATGCCCATACTGCGCC
CTTCTTGCAAAGGTTTCAGCAATTCCGGCAAAGTCTTCAAATCCGTATGTTCATTCAACG
TTTGCAGATTCAGCTCGCGGATAGGCGTAGTCACGCCCAAATGTTTCAAATGCGCACGCG
CCGTTTTTTCCGCCTCCACGACAAAATCCGTCAGCCCGACAATCGCCTGTTGTTCATGCG
GCAACAGGCACGGCGTGTCAGGCGTACCCAAAGGCGTAGGAATCAAATACAAAACAGGAG
ACATCATTCCCTCACTCATCGGTTAAAAATGCCGTCTGAGCCTTTCAGACGGCATAAACG
GGCAGTTACAGAACCTCCACGCCCTCATTTTTCAAGAAATCGACCAGACGGAAAACCGGC
AAACCGATTAAAGCATTCGGATCGGTACTCTCAATCCTTTCAATCAGCAATGCACCCAAA
TCCTCACTCTTCAGCGCACACGAACAATAAACCGCATCAGGCTCGCGCTCCAAATAGCGG
AGGATATGCAACTCGTCCAACTGCCTCATCACGACCACCGTCTTATCGATATGCCGCCGC
ATCCTGCCCGTAACCGTATTCAACAGCACGATCGCGCTGTAAAACTCAATCTCCCTGCCG
CTCAAGTGCATCAGCATCTTTTGCGCATTGGCAAGGTTCATCGGCTTGCCCCACTGCCTG
CCGTCGCACCACGCCACCTGGTCCGCACCGACAATCAACGCCTCTGGGAAACGCCCGGTC
AACGACCGCGCCTTACCCTCGGCAAGGCGCAATGCCGTCTGAGGGGCGGATTCCCCCAAC
ATCGGCGTTTCGTCAAAATCGGGGGACGCCGCCTGAAAGGCAATGCCGAGCCTTTCCATC
TGTTCGCGGCGGAAAACCGAACTCGTACCCAAAATCAAAGGCAGTTCCAAACCCATCCCA
TCCTCCTTACCGTTGAAAACACGCCCGAAGGGGCAGTAAAATCCAGCCATGCGCCGAAAC
ACGGATACCCGCCTTCGGCGTACCGCAACATTTTTCTTAAAAATATTGACGTTAGAACAT
CTAAATTATATCATATCCCGTTTATGTCAGACCCTAATTTGATTGACTTGGAAATTTTTG
CCGCCGAAGGGCAGAACCTGCAAGGCAGTTTTCTGCTGGAAGAATTGGATGAACGCGTCA
GTTCGCACGATTATCCCGCCGACAGGCAGACCAAAATATCGTTTACACTGACCGGCGGTC
GCGACCGGCTGCAACGCCTGTTCCTCGACCTGAACGTCAAAGCCGATATGCCCCTGATTT
GCCAGAGATGTATCAAACCCATGCCGTTCATGCTTGATGAAAGCAGCCGTATCGTCCTGT
TTTCCAACGAAGAGTCCTTGGACGAATCCATGCTTGCCGACGAAGAACTCGAAGGCATAC
TGATTGAAAAAGAACTCGACGTGCGCACATTGGTAGAAGACCAAATCCTGATGTCCCTGC
CCTTTTCGCCGCGACACGAAGACTGCGGCGACAATGGGACACTGGAAGAAGTCAATCGGG
ACAAACCCAACCCCTTTGCTGTTTTGGCAGGTTTGAAAAGCAATTGATTAGGACACAGTT

## Appendix A

TATTTATCTAGGAGCTTGAAATGGCCGTTCAACAAAACAAAAAATCCCCTTCCAAACGCG
GTATGCACCGTTCGCACGACGCGCTGACCGCGCCTGCACTGTCTGTCGACAGCACAACCG
GCGAAGTACACCGCCCGCACCACATCTCCCCCAACGGTATGTACCGCGGCCGCAAAGTGG
TCAAAGCCAAAGGCGAATAATCCCTATTCGACTGACTGAAAAAGCCAGAACATTGCCATG
CAATTACTGGCTTTTTTTGCATTGGACGCACCATCCGTCCAAACTTTCGCCATACGTCAA
CACACAGGGGCAAAGCGTTCCGTATAATACCCCGTGAAAATATTCCAAAAGCCCCAACCA
CCAAGGAAATTCCGATGAAACAGAAAATCTGGTACACCTACGATGACATCCACCGCGTCA
TCAAAGCATTGGCAGAAAAAATCCGGAACGCCGACATCAAATACGATGCCATGATTGCCA
TCGGCGGCGGCGGCTTTATTCCGGCACGTATGCTGCGCTGTTTTCTGGAAATTCCGATTT
ATGCCGTAACCACCGCCTATTACGACAGCGACAACGAAGGACAGGTTACCGAAGAAGTCA
AAAAAGTCCAATGGCTCGACCCCGTTCCCGAAGCCCTGCGGGGCAAAAACGTACTCGTCG
TCGATGAAGTGGACGACAGCCGCGTAACCATGGAGTTCTGCCTGAAAGAACTGCTCAAGG
AAGACTTCGGTACGATCGGAGTCGCCGTACTGCACGAAAAAATCAAAGCCAAAGCAGGCA
AAATCCCCGAAGGCATTCCCTATTTCAGCGGCATCACCGTAGAAGACTGGTGGATCAACT
ATCCGTGGGACGCACTCGACATCGACGAACACAACCGCCTTGCCGAGGCCGGCCGAGGCT
GACCCTTTCAGACGGCATATTTTCCGAACCGATGCCGTCTGAAGCCCGCACGACCCCTGC
CGCAGACCGAAAACCTACCGGAGAAACCCTATGATTACATTGGCCGTAGATGCCATGGGC
GGCGACCAAGGACTTGCCGTTACCGTACCCGGCGCAACCGCATTCCTCCAAGCACACCCC
GATGTCCGCCTGATTATGACCGGCGACGAAACGCAACTGCGCCAAGCCCTGACCGCGGCA
GGCGCACCGATGGAACGCATCGACATCTGCCATACCACCCAAGTCGTCGGCATGGACGAA
GCCCCGCAATCCGCCCTGAAAAACAAAAAAGACTCCTCCATGCGCGTCGCCATCAACCAG
GTTAAAGAAGGCAAAGCCCAAGCCGCCGTATCCGCAGGCAACACGGGTGCGCTCATGGCA
ACCGCACGTTTCGTCCTCAAAACCATTCCCGGCATCGAACGCCCCGCCATCGCCAAATTC
CTTCCTTCCGACACCGACCACGTTACCCTTGCACTCGACCTTGGCGCGAACGTCGACTGC
ACGTCCGAACAGCTCGCCCAATTTGCCGTTATCGGCAGCGAACTCGTCCACGCACTCCAT
CCTCAAAAAGGACAGCCGCGCGTCGGGCTGGTCAACGTCGGCACGGAAGACATCAAAGGT
ACGGACACCGTCAAACAAACCTACAAACTGCTGCAAAACAGCAAACTCAACTTTATCGGC
AACATCGAAAGCAACGGCATCCTCTACGGCGAAGCAGATGTCGTCGTCGCCGACGGCTTT
GTCGGCAACGTCATGCTCAAAACCATCGAAGGCGCGGTCAAATTCATGAGCGGAGCCATC
CGCCGCGAATTCCAAAGCAACCTGTTCAACAAACTTGCCGCCGTTGCCGCCCTACCCGCC
CTCAAAGGGCTGAAAAACAAACTCGACCCGCGCAAATTCAACGGGGCCATCCTGCTCGGG
CTGCGCGGCATCGTGATTAAAAGCCACGGCGGCACAGACGAAACCGGTTTCCGCTATGCC
CTCGAAGAAGCCTACCACGAAGCCAAGTCCGCCGGCCTTTCCAAAATCGAACAGGGCGTA
GCCGAACAACTCGCCGCACTCGAAACTGCCAAAGCCGTCCAAAACGAAAATGTCGGCGGT
CTGTAACACACACGATGCCGTCTGAACGCCCCCGCCCCTTTCAGACGGCATCCGCCCGCA
CCAAACCTGCGGGCGCGGACGGCGATGCGCCTGTCCGGCACTTCCCAAATATCGCCTTGT
AAAATAAGGAGTATTTGAAAAATGAAGACATTAGAAAAACGGATGAAAGCTCTAGACAAA
CGGATTATGAAGTTCGGAAAATCCCTTGAAGGCAGGCTTGATGCCCGTCTGATTGAATCC
GCATTGGATTATATTCATTATTCGGAACGTTTTTTGGCTTTTGAAATCCTGTGTACTTAT
ATCGAAGATTTCGATGTCCGGCTGACGGAACAAGAATCCCGGGAAATTTCTTTTATCAAC
AAGGAATTTGAGATAGAAAGCACGTCCGATTAACCAATAAAGCCAATGGGTTGATAAACA
TGAAAACATCGACGGTCGTTTTTGGCGGATTTTTTATGGCAGACAACGGAGAGCGAATCC
AAATCCCCGTTTTGGAAAATCCTGACATTAGGGAAATCAATCACTTTTTTTCCGTATCAA
ATTTTGAGAAAAAAACCGGCGTCCTTGTTTTCAGAATCATCCCCGAGCCGGAATTTGGCA
ATACCGAATTAACTGTCTATTTTAAAAAAGGATATTATAGTGGATTAACAAAAACCAGTA
CGGCGTTGCCTCGCCTTGCCGTACTGGTTTTTGTTAATCCACTATATCAGACGAAAACAA
ACACCCGCGCCAATAGCCTGACGGCAACCCGGCAATCAAAATGCCGTCTGAAGCAGCTTG
GGCTTTCAGACGGCATTTCCTTCGCTTAAAACAGCGTATCGGCAACCCCGCCCTGCCTGT
CCACGGCAATCTGCATCTGAAAACCATCTGTATCCCAAACCACACCCCCATCCCTGTTTC
CATCATGTGCACCCTGTCCGTATTGGGCAATCATCTGTTTTTCGCTTACAATAGCCGAAT
CTGAACCAACTCTCTAAAAAGGCCGTTCCCATGCAGTATGCAAAAATTTCCGGCACAGGC
AGCTATCTTCCCGCCAACCGCGTCAGCAATGACGACCTTGCCCAAAAGGTAGATACCTCT
GACGAGTGGATTACCGCGCGCACGGGCATCAAATTCCGCCATATTGCAGCCGAAAACGAA
AAAACCAGCGATCTTGCCGCCGAAGCGGCGCACCGCGCGCTGGATGCAGCCGGATTAGAC
AGCGGCGAAATCGATTTGATTATCGTGGCAACGGCAACGCCGGATATGCAGTTTCCGTCT
ACTGCGACCATCGTGCAACAAAAATTGGGCATCACCAACGGCTGCCCCGCGTTTGACGTA
CAGGCGGTGTGCGCCGGCTTTATGTACGCGCTGACCACGGCAAACGCCTACATTAAAAGC
GGTATGGCGAAAAACGCGCTGGTCATCGGCGCGGAAACCTTCAGCCGCATTGTAGACTGG
AACGACCGCACAACCTGCGTATTGTTCGGCGACGGCGCGGGCGCGGTGGTTTTAAGCGCG
TCGGACACGCCGGGCATCATCCACAGCAAACTCAAGGCCGACGGCAATTATCTGAAACTC
TTAAACGTCCCCGGGCAAATCGCCTGCGGCAAAGTTTCCGGTTCGCCGTACATTTCGATG
GACGGTCCCGGCGTGTTCAAGTTTGCCGTCAAAATGCTGTCCAAAATCGCCGATGACGTT
ATCGAAGAAGCAGGTTACACCGCCGCTCAAATCGACTGGATTGTTCCCCATCAGGCAAAC
CGCCGCATTATCGAATCGACCGCGAAACATTTAGGTTTGAGTATGGACAAAGTCGTCCTG
ACCGTCCAAGACCACGGCAACACATCCGCCGCATCGATTCCGCTGGCTTTGGATACGGGC
ATCCGCAGCGGACAAATCAAACGCGGTCAAAACCTGCTGCTCGAAGGCATCGGCGGCGGT
TTCGCGTGGGGCGCGGTGCTGTTGCAATATTGAACCCGATGCCGTCTGAAACAGGCTTTC
AGACGGCATTTCCCATATCATGAAGCGGCAGGCTTTCTTCAAACTGATGGCGTGTGCGGC
ATTTCTGTCTGCCGTTTCGCTGCGCCTCCCCGTATTGGGCGCGTGTTACGCAATATTGTC
CCTCTATGCGTTTGCACTTTACGGCATCGACAAACGGTGCGCCATACGGGGGCAACGCCG
CATTCCCGAACACCGCCTGCTGCTGCCTGCATTGCTCGGCGGCTGGGTGGGCGCGTATTT
CGGCAGCATGACATTCAAACATAAGACAGCGAAAAAGCGTTTTGTTGTGCTGTTCCGTCT
GACTGTTTCAGGTAATGTCTTGGCGACCCTCATCCTGATTTATAGTGGATTAAATTTAAA
CCAGTACGGCGTTGCCTCGCCTTGCCGTACTATTTGTACTGTCTGCGGCTTCGTCGCCTT
GTCCTGATTTTTGTTAATCCACTATATTATTTTGTCCCGCCTGAATTTTTCGTAAAACTC

## Appendix A

GGGCAGAATACCTGATTATCCAACCAAACAAAGGAATACTATGTCTTTTGCCTTCTTTTT
TCCCGGACAAGGTTCCCAAAGCCTCGGTATGATGAACGGCTTTGCCGAACACGCCATCGT
CAAAAACACCTTTGCCGAAGCCTCCGCCATATTGGGGCAGGACTTGTGGGCGATGATAAA
CGGCAGCGATGCCGAAATCATCGGTCAAACCGTCAACACCCAGCCCATTATGCTCGCCGC
CGGCGTTGCCGTTTACCGCGCCTATTTAGAAGCGGGCGGCAAAACGCCTGCCGCCGTTGC
CGGACACAGCCTCGGCGAATACACCGCACTCGTTGCCGCCGGCGCATTGAATTTTGCCGA
CGCGGTCAAACTCGTGCGCCTGCGCGCCGAACTGATGCAGTCCGCCGTACCGCAAGGCGT
GGGCGCAATGGCGGCGATTCTCGGCTTGGAAGATGAGCAGGTTAAAGCCATTTGTGCCGA
AGCCGCCCAAAGCGAAGTGGTCGAAGCCGTCAACTTCAACTCACCCGGACAAATCGTGAT
TGCAGGCAACGCCGCCGCCGTCGGACGCGCCATGGCTGCCGCCAAAGAAGCCGGTGCCAA
ACGCGCCCTGCCGCTGCCCGTGTCCGTACCTTCCCATTGCAGCCTGATGAAACCCGCCGC
CGACAAACTTGCCGAAGCCCTGAAAACCGTTGAAATCAAGCAGCCGCAAATCCGCGTTAT
CCACAACGCCGACGTTGCCGCCTACGATGATGCCGACAAAATCAAAGACGCGCTCGTCCG
CCAGCTTTACAGCCCCGTACGCTGGACGGAAACCGTCAACGCCCTCGTTTCAGACGGCAT
TGCCGAATCCGCCGAATGCGGCCCGGGCAAAGTGTTGGCGGGCTTGGCAAAACGCATCAA
CAAAGCCGCCGCGTGCAGCGCACTGACCGATGCCGGACAGGTTGCCGCCTTTATCGAAGC
GCACTGACTTCGTTCTGCAAAAAGCAGCCTGCCCTCTTCAGGCTGCTTTTCATGTCCGAA
CGACGGCAGCCCCATATTTACGCTATAATCCATCCCGACCAAACCACCGACAGCGGCTGC
CGTTGCAGTTCCCGCCCTACCGATATGATAGAAAAACTGACTTTCGGACTGTTTAAAAAA
GAAGACGCGCGCAGCTTTATGCGCCTGATGGCGTACGTCCGCCCCTACAAAATCCGCATC
GTTGCCGCCCTGATTGCCATTTTCGGCGTTGCCGCCACCGAAAGCTACCTTGCCGCCTTC
ATCGCCCCCCTGATTAACCACGGCTTTTCCGCACCTGCCGCGCCGCCCGAGCTGTCTGCC
GCCGCCGGCATCATTTCCACCCTGCAAAACTGGCGCGAACAGTTTACCTATATGGTTTGG
GGGACGGAAAACAAAATCTGGACCGTCCCGCTCTTCCTCATCATCCTCGTCGTCATCCGT
GGCATCTGCCGCTTTACCAGCACCTATCTGATGACTTGGGTCTCCGTGATGACCATCAGC
AAAATCCGCAAAGATATGTTTGCCAAAATGCTGACCCTTTCCTCCCGCTACCATCAGGAA
ACGCCGTCCGGCACCGTACTGATGAATATGCTCAACCTGACCGAACAGTCGGTCAGCAAC
GCCAGCGACATCTTCACCGTCCTCACGCGCGACACGATGATCGTTACCGGCCTGACCATC
GTCCTGCTTTACCTCAACTGGCAGCTCAGCCTCATCGTCGTCCTGATGTTCCCCCTGCTC
TCCCTGCTCTCGCGCTACTACCGCGACCGTCTGAAACACGTCATTTCCGACTCGCAAAAA
AGCATAGGCACGATGAACAACGTGATTGCCGAAACCCATCAGGGACACCGCGTCGTCAAG
CTGTTCAACGGGCAGGCGCAGGCGGCAAACCGGTTCGACGCGGTCAACCGCACCATCGTC
CGCCTCAGCAAAAAAATCACGCAGGCAACGGCGGCACATTCCCCGTTCAGCGAACTGATC
GCCTCGATCGCCCTCGCCGTCGTCATCTTCATCGCCCTGTGGCAAAGCCAAAACGGCTAC
ACCACCATCGGCGAATTTATGGCATTCATCGTCGCGATGCTGCAAATGTACGCCCCCATC
AAAAGCCTTGCCAACATCAGCATCCCTATGCAGACGATGTTCCTCGCCGCCGACGGTGTA
TGTGCATTTCTCGACACCCCGCCCGAACAGGACAAGGGCACGCTCGCACCGCAGCGTGTC
GAAGGGCGCATCAGCTTCCGCAACGTCGATGTCGAATACCGTTCAGACGGCATCAAAGCC
CTCGACAACTTCAACCTCGACATCAGACAAGGCGAACGCGTCGCCCTGGTCGGACGTTCC
GGCAGCGGCAAATCCACCGTCGTCAACCTGCTGCCCCGCTTTGTCGAACCGTCTGCCGGC
AACATCTGCATAGACGGTATCGACATCGCCGACATCAAACTCGACTGCCTGCGCGCCCAA
TTCGCCCTCGTCTCCCAAGACGTATTCCTGTTTGACGACACCCTGTTTGAAAACGTCCGA
TACAGCCGTCCCGACGCGGGCGAAGCCGAAGTCCTGTTCGCCCTCCAAACCGCCAACCTG
CAAAGCCTGATTGACAGCTCCCCGCTCGGACTGCACCAGCCCATCGGATCGAACGGCAGC
AACTTATCCGGCGGACAGCGGCAACGCGTCGCCATTGCCCGCGCCATTTTGAAAGACGCG
CCGATATTATTATTGGACGAAGCCACCAGCGCATTAGACAACGAATCCGAACGCCTCGTC
CAACAGGCGCTCGAACGCCTGATGGAAAACCGCACCGGCATCATCGTCGCCCACCGCCTG
ACCACCATCGAAGGGGCCGACCGCATCATCGTGATGGACGACGGCAAAATCATCGAACAA
GGCACACACGAACAACTGATGTCCCAAAACGGTTACTACACGATGTTACGCAATATCTCA
~~AACAAAGATGCCGCCGTCCGGACGGCATAAACAAAATGCCGTCCGAAATGGTACAATCGC~~
CCCGACCCTTTCAGACGGCATCATATCCGCCGACCCATCCGATTATCTTCAATCACTGTA
AAACCCATTATGACCCAAGACAAAATCCTCATCCTTGACTTCGGTTCGCAAGTTACCCAG
CTCATCGCCCGCCGCGTGCGCGAAGCCCACGTTTACTGCGAGCTGCATTCTTTCGATATG
CCTTTGGACGAAATCAAAGCCTTCAACCCCAAAGGCATCATCCTCTCCGGCGGCCCCAAT
TCCGTTTACGAATCCGACTATCAAGCCGATACCGGTATTTTTGATTTGGGCATTCCGGTT
TTGGGCATCTGTTACGGCATGCAGTTTATGGCGCACCACTTGGGCGGCGAAGTGCAGCCC
GGCAACCAGCGCGAATTCGGTTATGCGCAAGTTAAAACCATAGACAGCGAGCTGACACGC
GGCATTCAAGATGGTGAGCCAAACACACTCGACGTATGGATGAGCCACGGCGACAAAGTG
TCCAAACTGCCCGACGGTTTCGCCGTCATCGGCAACACCCCGTCCTGCCCGATTGCCATG
ATGGAAAACGCCGAAAAACAATTCTACGGCATCCAGTTCCACCCCGAAGTTACCCACACC
AAACAAGGCCGCGCCCTGTTGAACCGCTTTGTCTTGGATATTTGCGGCGCACAACCGGGC
TGGACGATGCCGAACTACATCGAAGAAGCCGTTGCCAAAATCCGCGAACAGGTCGGCAGC
GACGAAGTGATTTTAGGTCTGTCCGGCGGCGTGGACTCTTCCGTAGCCGCCGCGCTGATT
CACCGCGCCATCGGCGACCAACTGACCTGCGTGTTCGTCGATCACGGTTTGTTGCGCCTG
AACGAAAGCAAAATGGTGATGGATATGTTCGCCCGCAACTTGGGTGTGAAAGTGATACAC
GTCGATGCCGAAGGGCAGTTTATGGCGAAACTCGCCGGCGTAACCGACCCCGAGAAAAAA
CGCAAAATCATCGGTGCGGAATTTATCGAAGTATTTGATGCCGAAGAAAAAAAACTTACC
AACGCCAAATGGTTGGCACAAGGCACGATTTACCCTGACGTAATCGAATCCGCAGGTGCA
AAAACCAAAAAAGCCCACGCCATCAAATCGCACCACAACGTCGGCGGCCTGCCTGAAAAC
ATGAAGCTCAAATTGCTTGAGCCTTTGCGCGATTTGTTCAAAGACGAAGTACGCGAATTG
GGTGTGGCTTTGGGCCTGCCGCGCGAAATGGTGTACCGTCATCCGTTCCCGGGTCCGGGT
TTGGGCGTGCGTATTTTGGGCGAAGTGAAAAAAGAATATGCCGACCTGCTTCGTCAGGCA
GACGATATTTTCATTCAAGAATTGCGCAATACTACCGATGAAAACGGTACATCTTGGTAC
GACCTGACCAGCCAGGCATTCGCCGTGTTCCTGCCCGTCAAATCTGTCGGCGTAATGGGC
GACGGCCGCACATACGATTACGTCATTGCCTTGCGTGCCGTGATTACCAGCGACTTTATG

## Appendix A

ACCGCGCATTGGGCGGAACTGCCGTATTCCTTGTTGGGCAAAGTGTCCAACCGCATCATC
AACGAAGTCAAAGGCATCAACCGCGTGGTTTATGATGTGAGCGGCAAACCGCCTGCCACC
ATCGAGTGGGAATAAACAGCAAACATGGCTGCCCCGTCCGGCGCAGTCCTTCGATTATCG
GAAAAAAGGAAAAAATATGAGCACACAAGATTTAAACGGCAAAATCGCTTTGGTAACAGG
CGCATCGCGCGGTATCGGTGCAGCAATTGCCGACACGCTGGCGGCAGCCGGTGCCAAAGT
CATCGGTACGGCGACCAGTGAGAGCGGTGCGGCGGCGATTAGCGAGCGGTTGGCGCAATG
GGGCGGCGAAGGCCGCGTATTAAATTCCGCCGAACCTGAAACCATCGAAAGCCTGATTGC
CGACATCGAAAAAGCGTTCGGCAAACTCGATATTCTGGTCAACAACGCCGGCATCACCCG
CGACAACCTCCTGATGCGCATGAAAGAAGAAGAGTGGGACGACATCATGCAGGTCAACCT
CAAATCCGTGTTCCGCGCTTCTAAAGCCGTTTTGCGCGGTATGATGAAACAACGTTCCGG
CCGCATCATCAACATCACATCCGTCGTCGGCGTGATGGGCAATGCCGGTCAAACCAACTA
TGCCGCGGCAAAAGCAGGCTTAATCGGTTTCTCCAAATCCATGGCGCGCGAAGTCGGCAG
CCGGGGCATTACCGTCAACTGCGTCGCCCCTGGCTTTATCGATACCGACATGACACGCGC
CCTGCCGGAAGAAACCCGCCAAACCTTTACCGCCCAAACCGCCTTGGGCAGATTCGGCGA
CGCGCAAGACATCGCCGATGCGGTTCTGTTCCTCGCTTCCGACCAAGCAAAATACATCAC
CGGCCAAACGCTGCACGTCAACGGCGGTATGCTGATGCCTTAACAGACAACTTTTTCAAC
CATGCCGTCTGAAGCCCTTTCAGACGGCATTTGCATTCTCAGGCAAAATGAACACACACC
ACACCCCGCCCTGCCCATGCGGCTCAGGCACAAGCTGAGACCTTTGCAAAATTCCTTTCC
CTCCCGACAGCCGAAACCCCAACACAGGTTTTCAGCTGTTTTCAGCTGTTTTCGCCCCAA
ATACCGCCTAATTCTACCCAAATACCCCCTTAATCCTCCCCGGACACCTGATAATCAGGC
ATCCGGGTCACCTTTTAGGCGGCAGCGGGCGCACTTAGCCTGTTGGCGGCTTTCAAAAGG
TTCAAACACATCGCCTTCAGATGGCTTTGCGCACTCACTTTAATCAGTCCGAAATAGGCT
GCCCGGGCGTAGCGGAATTTATGGTGCAGCGTACCGAAGCTCTGTTCGACCACATATAGT
GGATTAACAAAAACCAGTACGGCGTTGCCTCGCCTTAGCTCAAAGAGAACGATTCTCTAA
GGTGCTGAAGCACCGAGTGAATCGGTTCCGTACTATTTGTACTGTCTTCGGCTTCGTCGC
CTTGTCCTGATTTTTGTTAATCCACTATACATCATCGCTACTACCGTTCCGGCGCAACAG
GCATTCCTCGATGCCGCCGAACTGATGCAATGGAGTATAGAAACCGAAGGGCTGGGCTTG
AACGTCATCTCGCACAAGATACTCGGCAAAGACCACGCCCAAGTCGAATTTGAAGCCTAC
TTCCGAGACGGACAACACCGATCCGCGCATCACGAACTGTCCGGCTTCGTCAACATCGGC
GGACAATGGTATTTTATCGATCCCACCGTTCCGCATCCTGCGATGAAACAACCCTGCATT
TGCGGATCAGGCAAAAAATTCAAAGCCTGCTGCGGCAAATATCTGAAACCTGTCGCATAA
AAATGCCGTCTGAACGTTCAGACGGCATTTTCAACGTGCAAAAAAAACCATTCATACCAA
GGGTAAGTATGAATGGTCAATACATTGCGGGAAAACGTCTTACTTGCTGCACTGCCGAAA
AGGGAGAAACGGCAGCGGTAATCAGCGGAAAGGATTGTACCCGAATTAATATTAAGAAAC
GTTAATCGCGAAAATATATTAACAAACCTGTTGAAACCTATTGGTTTTCCCGTATCCACC
CGACCCAGCGTTCAAACAGCTTCGGTTCGAGCGCGGCAACGACCGAGCGTTTGAACACGT
GTTCACCACTCCAAAACCCGTCGCCTTCCAAAGTCGTCAGCCTGCCGCCCGCCTCCTCGA
AAATCAACGCGCCGGCGGCATAATCCCACAGCTTCTGCCCGCCGTGAACATAAACATCAT
AACGCCCGCACGCCAGATAACACCAATCCAACGTACTGCTGCCCATACTCCGTATCGTTC
CAAAAGGCGCGAGCGTACTCATACGGCTGGAAAGTTTGCCCGAACGCAGATATTTGATTT
CCACGCCCGCAATCGCCTCATTGAGTTTTTTATCCACGAGGCGCAGGGGCAGACGCGTCC
CGTTTAAAAACGCCCCCTGCCCGCGTTCGGCATAAAAACATTCGCCGCTGACTGGGTTGT
AGATTACGCCCAACTCGGCGCGCCCGTTGCGGACAAACGCCACCGATACCGCAAAATGCG
GCAGCCCGTTGACAAAATTGTTCGTCCCGTCTATCGGATCGACAATCCACAGCCCCTTTT
CCCCCGAATATTGTTCCCACAAAGCCGACTGTTCCTGCCGCGACATTTCCTCACCCAACA
TCGGACTGTCGATTAAAAGCGGCAACGCGGCGGCAAAAGCCGTCTGCGCGGCAATGTCCG
CCTCGCTCAACATCGAACCGTCTTCCTTGCGGTGAGACGGCGTATTCAAAAAACGCGGCA
TAATTTCGGTTTGCGCGATATGGCGCACGACTTTCTGCAAACGGTGTAACACTTCCTACT
GTCCTCATATTTTGAACTTGCGGCGCGCGAACGTATAATGTCCGCTTCCATCACGCCGCT
~~GCGAGGGATTATAACCGTCCGAACCGCCAAAAACTATGCCCCGATTCCACCTGCCCGAAA~~
ACCTTTCCGTCGGACAAACCGTCGCCCTGCCCGACAACATCGTCCGCCACCTCAACGTCC
TGCGCGTCCGCCCCAACGAAAACATCACCCTCTTCGACGGCAAAGGCAAGGCACACGCCG
CACGGCTGACCGTTTTGGAAAAACGCCGCGCCGAAGCCGAAATCCTGCACGAAGACACAA
CCGACAACGAGTCCCCGCTCAACATCACACTGATACAATCCATCTCCTCCGGCGATCGCA
TGGATTTCACCCTGCAAAAAAGCGTCGAACTCGGCGTAACCGCCATACAGCCCGTCATCA
GCGAACGCTGCATCGTCCGCCTCGATGGGGAACGCGCCGCCAAACGCCTCGCACGCTGGC
AGGAAATCGTCATCTCCGCGTGCGAACAAAGCGGCAGGAACACCGTTCCCCCCGTACTGC
CCATCATCGGCTACCGTGAAGCACTCGACAAAATGCCGTCTGAAAGCACCAAGCTGATTA
TGAGCATCAACCGCGCCCGCAAACTCGGCGACATACGCCAACCGTCCGGCGCAATCGTCT
TTATGGTCGGGCCCGAAGGCGGCTGGACAGAACAGGAAGAACAACAGGCATTTGAAGCTG
GCTTTCAGGCGGTTACACTCGGCAAACGGATTTTACGCACAGAAACCGCCCCACTCGCCG
CCCTCGCCGCCATGCAGACGCTTTGGGGCGATTTCGCATAAACAGAAATGCCGTCTGAAA
CCCGTTCAGACGGCATTTTGCAGCCGATTAAGATAGTAGGTTCAAATAAGATTTCCCGTG
TCGTCATTCCCGCGAAAGCGGGAATCTAGAAACGAAAAACTACAGAGATTTATCCGAAAC
AACAACCCTCTCCGCCGTCATTCCCGCAAAAGCGGGAATCTAGAAACGAAAAACTACAGG
GATTTATCCGAAACAACAAACCCTCTCCGCCGTCATTCCCGCGCAGGCGGGAATCTAGAA
ACGAAAAACTACAGGGATTTATCCGAAACAACAAACCCTCTCCGCCGTCATTCCCGCGCA
GGCGGGAATCTAGAAATTTAACGTTGCGGTGATTTATCGGAAATGACTGAAACTCAACGG
ACTGGATTCCCGCCTGCGCGGGAATGACGAGATTTTAGGTTTCTGTTTTTGGTTTTCTGT
TCTCGCGGGAATAACGGAATTTTAAGTTTTAGGAATTTGTCGGAAAAACAGAAATCCCCC
CGCCGTCATTCCCGCAAAAGCGGGAATCTAGAAACGAAAAACTACAGGGATTTATCCGAA
ACAACAAACCCTCTCCGCCGTCATTCCCGCGAAAGCGGGAATCTAGAAATTTAACGTTGC
GGTGATTTATCGGAAATGACTGAAACTCAACGGACTGGATTCCCGCCTGCGCGGGAATGA
CGAATTTTAGGTTTCTGTTTTTGGTTTTCTGTTCTCGCGGGAATAACGGAATTTTAAGTT
TTAGGAATTTATCGGAAAAACAGAAATCCCCCCGCCGTCATTCCCGCGAAAGCGGGAATC

## Appendix A

TAGAAATTTAACGTTGCGGTGATTTATCGGAAATGACTGAAACTCAACGGACTGGATTCC
CGCCTGCGCGGGAATGACGAATTTTAGGTTGCTGTTTTTTGGTTTTCTGTTTTTGCGGGA
ATGACGAATTTTAGGTTTCTGTTTTTGGTTTTCTGTTCTCGCGGGAATAACGGAATTTTA
AGTTTTAGGAATTTGTCGGAAAAACAGAAATCCCCCCACCGTCATTCCCGCAAAAGCGGG
AATCTAGAAATTTAACGTTGCGGTGATTTATCGGAAATGACTGAAACTCAACGGACTGGA
TTCCCGCCTGCGCGGGAATGACGAAGTGGAAGTTACCCGAAACTTAAAACAAGCGAAACC
GAACGGACTAGATTCCCGCCTGCGCGGGAATGACAGTGTATCCATTTCTAATTTTAATCC
GCTATATTTTACACAAACTATTTGAACGATATGACCCGCCTGCCGTAAGCTTTCTCAAGC
TCCGCCTGCCTTTGACGCTCCATTCTTTTCTTCTTTTCCCTACCGAATTTACCCAAAGCA
CGCTTCAAGTCAAACATCACCTTCAACGAACGGCGGTGTCTTCTTTCTTGTTCCCTATCT
TTTTCCAAATCGCTACCCAACATACTGTTTTTACTGAGGAACTTGGCATAATGCAATTCT
TGGGTACATAAGGCGGGATTAACCTGATAAACAGGCATCCCCTCCTTATCAAAGAAATAA
GTAAACATCATCCAATCTACCGCTTTAATCCACTCTGCCGGCAAAACGGCAAACCTTTCC
AAGAAAAACCGCATCGCCTCACGCGAAATGATATAGCCAGCCGTCCCCCAATGTTCGCTC
TCCAGCAAAGGAAATGACCGATTCTCATAATTCAGGACTTTATCCGGTCTGACAATAACT
TTCGCAAACATCGTTTCCAAACGAACGATAAAGGCAGAATCCTTATCAAAACGCTCTTCC
AACCAAGTATCTTCGGCAAGGAACTTTTCTGCGTCTTTGCCAAGCAGGACATCATCCTCA
AATACGGCAACATAGGGCAGACCTTCATCCAATGCCTGTTTCCACAATACGGCGTGGCTC
ATAAAGCAGGCTTTTTCCACTTCGCTCAACAGGTGCTGTTTTGCCAATCCCGGCACCAAT
TCCGCCATCATCCGATTCAGTTCTTCAGACGGCATCAGTGCGTCGAAAAACTGAAACGGG
ATGCCGCGCACGCCGAAGGTTGCGGCAATGTGCGCCCTGCGTTCTGCGGCGGAAGCTAAG
CTGATAACATGGTTTTGCATAATTTATCCTGTTTTTTGTCTGTTGGATAAAGCGGCGTTT
TTCAACGGTTTTTCAGCAATCGGCGCAAAATGCCGAAGTATTGCCTCAAGGTAAACAGCC
GCCGCATCCTGCCGTCTGCTGCAAATACGATGTCCATCTCTCCTCCTTTTATTGGAAAGG
CACAATGAACTGTTCGCGCCTTTGCCGGCGTTTTTCCCTTTCCCTGCTGATTTTGGTCAA
GGCGCGGATCAGGCGGTGTTTGAATGTGTTGGCGGGGAATCGCGCCTTTGCTGTTTGCG
GTTCAGGAGGCGGTCGTGTTCGATCAGGCTGCCCAATGCGCTGTTTTGGTCGTGAAACTT
GGCATAATGCAGCTCTTGGGCGCACAAGGCGGGATTGAGCTGGCAAACCGGCATTCCTTC
CCTGTCGAAAAAATCGCTGAACATCATCAGATCGACGGGGTGCAGCCCTTCGGGCGGCAG
GGCGGCAAACCTGTCCAGGAAAAACCGCATCGCTTTTCGGGAAATGATATAGCCCGCCGT
CCCCCAGTGTTCGCTTTCCAACAGCGGAAAGGCGCGCCCGCAGTAATCCGCCACGCCGGA
GGGCGAGGTCAGGACGTGCATAAACATCGTTTCCAAGCGGACGATAAAGGCGGTATCCGG
GTCAAAGCGTTCTTGCAGCCAAGCGTCTTCGGCAAGGAATTTTTCCGCACCTTCGCCGAG
TAAAACGTCGTCCTCAAATACGGTGATATACGGCAGACCTTCGTCCAATGCCTGCTTCCA
CAATACGGCGTGGCTCATAAAGCAGGCTTTTTCCACTCCGCTCAAATAGGGGTGCGCCGA
CAAGCCGGGGACGAGTTCCGCCATTGCCTGTTCCAGCCTTTCAGACGGCATCAGTGCGTC
GAAAAACTGAAACGGGATGCCGTGCCTGCCGAAGGTATCGGCAATGTGCGCCCTGCGTTC
TGCGGCGGAAGCTAAGCTGATAACGTGGTTTTGCATAATTTATCCTGTTTTTTGTCTGTT
GGATAAAGCGGCGTTTTTCAACGGTTTTTCAGCAATCGGTGCAAAATGCCGAAGTATTGC
CTCAAGGTAAACAGCCGCCGCATCCTGCCGTCTGCCGCAAAATCCAGCCACGCGCCGGCG
GGCAGCGTGTCCGTCCGTTTGAAGCATTGGTACAAAAACCGGCGGGCGCGTTCAAAATCT
TCTTCCGGCAAATGTTTCTCCAGCAATTCATACGCTACTGCTTTTATTTGGCGGTATTCA
AGGCTGTCGAACCGGGTTTTAAAACCCATAGACTGCAAAAAATCGTTTCTGGCGGTTTTT
TGGATGCCTTGCGCGATTTCGTGTTGGCGGATGCTGTATTTGGATGAAACCTGATTGGCG
TGAAGGCGGTATTTGACCAAGGCTTCGGGATAATAAGCCAGCCTGCCCAATTTGCTGACA
TCGTACCAAAATTGGTAATCTTCCGCCCAATCCCGCTCGGTGTTGTAACGCAAACCGCCG
TCAATGACGCTGCGCCTCATAATCATCGTGTTGTTGTGTATGGGGTTGCCGAAAGGGAAA
AAGTCGGCAATGTCTTCGTGTCGGGTCGGTTTTTTCCAAATTTTGCCGTGTTCGTGGTGC
CGCGCCAGCCGGTTGCCGTCCTTTTCTTCCGACAAAACTTCCAGCCACGCACCCATCGCG
ATGATGCTGCGGTCTTTTTCCATCTCACCCACGATTTTCTCAATCCAGTCGGGGGCGGCA
ATATCGTCTGCATCGGTGCGCGCAATATATTCCCCCCCCCCCCCCGACTTTGCCAATTCA
TCCAGCCCGATGTTTAAAGAGGGAATCAGACCGGAATTGCGCGGCTGCGCGAGGATGCGG
ATGCGGCCGTCCTGTTCTTGGAAACGCTGGGCAATGGCAAGCGTACCGTCCGTCGAGCCG
TCATCGACAATCAAAATATCCAAGTTGCGCCAAGTTTGATTCACGACGGCGGCTAATGAT
TGGGCGAAATATTTTTCTACGTTGTAGGCGCAAATCAATACGCTGACTAAAGGCTGCAAT
TTATTCTCCCGATAGGCACGATGCCGTCTGAAGGCTTCAGACGGCATTTGGACTGTACAA
CGGTTACTCGCCCAAAAGCGCGATATCCGCTACCGCGTTCATTTGTTCTGCCAAGCGGTT
CAGCAGGTTCAGGCGGTTTTGTTTCACGGCGGCATCTTCCGCCATCACCATCACGCCGTC
GAAGAAGGCATCGACTTGCGGTTTGACGGAAGCCAGTTCGGACAAGGCGGTCTGGAAATT
GCCTTCGGCAACGGCGGCGGCAATTTTCGGCTGCAAGCCTTGTGCGGCGGCAAAGAGGGC
TTTTTCTTCGTCCTGTTGCAGCAAGCTTTCGTTAACCGCGCCCAACTCGGCATCGGCTTT
TTTCAGCAGGTTTTGCACGCGTTTGTTGGCAGCGGCGAGCGCGGCGGCTTCGGGCAGTTG
TTTGAACGCGGCGACAGCCTGCAGTTTGGCGGTCAAATCGTCCAAACGGCGCGGCTGCTT
GGCAAGTACGGCGGCAACGATGTCTTGCGGATAATCGTTTTGCAGCAATACGGCAAGGCG
CGCCTGCATGAAGTCGGCGGTTTCAGACGGCGTTTTTTCGTTGAGCAAACCTTGCGGGAA
GCTGTTGAAGGCCGTCTGAATCAGTTCGTTTACGTCCAAACCGTACTGCATCAGCATACG
CAAAATACCCAATGCGGCGCGGCGCAGGGCGTATGGGTCTTTGTCGCCGGTCGGAATCAG
GCCGATACCCCAAATGCCGACCAAGGTTTCCAGTTTGTCGGCAAGCGCAACGGCGGCGGC
AATTTTGCCCTCAGGCAGGTTGTCGCCGGCAAAACGCGGTTGGTAGTGTTGCTCGACGGC
TTCGGTAATTTCTTCGGTTTCGCCGTCCAAGCGGGCGTAGTATTTGCCCATCGTGCCTTG
CAGTTCGGGGAACTCGCCGACCATTTCGGTTACTAAGTCGGCTTTTGCCAAACGCGCGGC
GCGTTCGGCTGCGGCGGCATCCGCGCCCAAAGCCTTGGCGATATGGGCGGCGATGCTTTG
CAGGCGTTCGATGCGTTCGGCTTGCGAACCGATTTTGTTGTGATAAACCACGTTCGTCAG
TTTGGGCAGGCGGCTTTCCAAAGTCGCTTTTTGGTCTTGTTTGTAGAAGAACTCGGCATC
AGACAGGCGCGCGCGCAAGACACGTTCATTGCCTTGGATGATGTGTGACGGATCTTCGGT

## Appendix A

TTGCAGATTGGACACCAGCAGGAAGCGGTTCATCAGCTTGCCGTTTTGGTCGAGCAGCGG
GAAGTATTTTTGGTTTTGCTGCATCGTCAGAATCAGGCATTCTTGCGGTACGGCGAGGAA
GTGTTCTTCAAAACCGGCTTCCAATACCACAGGCCATTCGACCAGCGCGGTTACTTCGTC
CAACAAGGCTTCATCGGCGGCGGCGGTCGCGTTCAGACGGCGTGCCTGCCCTTCCAATAC
CGTCTGAATCGCGGCTTTGCGCTCGGCAAACGAAGCGACGACTTTGCCTTGCTCGCGCAT
TTGTGCGGCGTAGCTGTCGGCGTTTTCAATGGTAATTTCGCCGTCGGAGAGGAAGCGGTG
TCCCAAGGTTTTGTTGCCGCTTTGCAGACCCAAAACGCTGACGTTCACAATGTCGCCGCC
GTGCAGTACAACTAGCCCGTGAACGGGGCGCACAAAGGTAAACGTGCTGCTGCCCCAACG
CATTACTTTGGGAATCGGCAGCTTCTTAACCGCTTGATTGATAATGTCTTCCAAAAGTCC
GCCCAACGGTTTGCCGATTTGGACGTATTCGTAGGCGTACACGTCCTGCTTGCCGTCGTG
GACGATGGTCAAGTCTTCGATTTTCGCGCCCGCACCGCGTGCGAAACCTTCCAAAGCCTT
GGTTGGCGCACCGTCTTTCATGGCATTCGCTACGGCAGGGCCTTTTTTCACAATTTTTTG
ATCAGCCTGAACGGCTTTGACGTTTTTGACTTGAACCGCCAAACGGCGCGGCGAGGCATA
AGCCGTAAATTCGGCTGCGCCGTCAACCAGTTGCGCTTTTTCCAAGCCTTCGGCAACGGA
AGCGGCGAAATGGTTGCCCAGATTATTCAGGGCTTTGGGCGGGAGTTCTTCGGTAAGGAG
TTCGATTAAAAGGGTTTGGGTCATCATTCGGCTTTCTTTGAATTTGGTTAATCTGCCTGT
TTATAGGTTTCGCTGTAATTTTCCCAGCCGTCATCCCCATAAAAACCGTCAACCAGCGGG
GTGGCGTACAAAGTGGCAACATCTTCGCGGTCTGCCAGCCAAGAGATAATGGCTTTTTTC
TCGGTTTCTCCCAAGCTTCGGGCACCGGATTTTTGAAACAGGCACGAAAAATCGCCGCAA
TCGCCCCCCCGCCATTTCAAAGCCGTTTGCCGCAAGATACGCAATCAGCTCGTCCATAAA
GCGGTCGAACGCTTCGGCATCGTCCTCAGCTTGGTGCAAACTGCCTTGAACGCCGAAAAT
CAATGTTTGAAACTCGCCCAAATGCAGCTTTTTATGCTGGCGGCGGTTCATTTTGTGCAG
GCGTTTCCTGCTTGGGGTGCGGAAATAGACAGGCATGATTTTCCTAAAAAATATAATGGC
TTCCGGACGGCTGCCTTATCGTGCCGCCCGAACGTAAAAAATCGTCGCCCCCTTAGGCGG
CGTTTGCCTTCATTAAAGGGAAGCCCAGTTTTTCGCGGCTTTCAACATATTTTTGCGCCA
CGGCGCGGCTCAATGCACGAATACGTCCAATATAAGTTGCCCGCTCAGTTACGGAAATCG
CGCCGCGTGCGTCTAAAAGGTTGAACGTATGCCCCGCTTTGAGGACAAGCTCGTAGGCAG
GCAGGGCGAGGGCGGCGTTTTCTTCGGCAAGCAGGCGTTTGGCTTGCGCTTCGTAGTCGT
TGAACTGGCGCAGCAGCCAGTCGGCATCGCTGTATTCGAAGTTGTAGGTGGATTGCTCGA
CTTCGTTTTGGTGGTACACGTCGCCGTAGGTGACGGTGTTGCCGTCGAGCGTTTTTGCCC
AAACGAGGTCGTAGACGTTTTCTACACCTTGCAAGTACATCGCCAAGCGTTCGATGCCGT
AGGTGATTTCGCCGAGTACGGGCGTGCAGTCGATGCCGCCGACTTGTTGGAAATAGGTAA
ACTGGGTTACTTCCATGCCGTTGAGCCAGACTTCCCAGCCCAAACCCCACGCGCCGAGGG
TGGGGTTTTCCCAGTCGTCTTCGACAAAGCGGATGTCGTGGACTTTGGGATCGATGCCCA
ATTCGCGCAGAGAGTCGAGATAGAGGTCTTGGATATTGGCGGGAGCGGGCTTGAGGGCGA
CTTGGAATTGGTAATAGTGTTGCAGGCGGTTGGGGTTGTCGCCGTAGCGGCCGTCTTTGG
GGCGGCGGCTGGGTTGGACGTAGGCGGCAAACCAAGGCTCGGGGCCGAGTGCGCGCAGGC
AGGTGGCGGGATGGGATGTGCCGGCACCGACTTCCATGTCGAAGGGTTGGATGACGGTGC
AGCCTTTGTCTGCCCAGAATGTTTGCAGTTTGAAGATGATTTGTTGGAAGGTAAGCATGG
CTTATGATTCGATAAAATAAAGGGTTTATTTTACTGTTTCCATTGCTGTTTGGATAGGTT
TATCTCAAAGACAGACTGATTTGAAAACACGGCATACATGATATAGTGGATTAAATTTAA
ACCAGTACAGCGTTGCCTCGCCTTAGCTCAAAGAGAACGATTCTCTAAGGTGCTCAAGCA
CCAAGTGAATCGGTTCCGTACTATTTGTACTGTCTGCGGCTTCGTCGCCTTGTCCTGATT
TTTGTTAATCCGCTATATGTTTCGGTTAGGCGGCAGGCTGCCCTATTGAATACCTTAAAG
CAGGCTATGCCTGCCAACGCCATATCCAAACACAGTCTTTAATTTAAATCCGGAAAATAA
AAAGCACGACCAAACGGTCGTGCTTTTCCAAACCAAACAAGTTTATTTCTTGTGCGAACG
GATATAGTCCAAAGTTTTGAGCTGTGCAATCGCAGCAGCCAATACTTTATGCGCTTCCGC
CAAAGCCTTATCGTCTTTAGCTTGGGAAATGCCCGCTTCTGCGGCTTTTTTCGCCTCTTC
CGCACGTGCCCGATCCATCTCCGCACTGCGGACGGCAACATCCGCCAAGACAGTTACTTT
ATCAGGCTGTACTTCCAAAACACCGCCGGAAACAGCAACGAAAACCTCTTTATCCTCGCC
CGGAACGGTCAAACGCAAAGCCCCCGGCCGCACCAAACTCATAATCGGCTCGTGTCGCGG
ATAAATACCGAGTTCGCCCTGTACAGTCGGAACAACGATAAATGTTGCCTCGCCTGAATA
GATTTTCTGCTCGCTACTTACCACCTCAACTTGCATGATGCTCATGCCGACCTCCTTAGT
TTAAGGTTTTCGCTTTCTCTACTGCTTCTTCAATGCTGCCGACCATATAGAATGCCTGCT
CGGGCAGATGATCGTATTCGCCGTTCAAGATGGCTTTGAAGCCGGCAATGGTATCGCGCA
GGGCGACATATTTACCCGGAGAACCTGTAAACACTTCGGCAACGTGGAACGGTTGGGACA
GGAAGCGTTGGATTTTACGCGCACGCATTACGGTCAGTTTGTCTTCATCAGACAATTCGT
CCATACCCAAGATGGCGATGATGTCGCGCAATTCTTTGTATTTTTGCAGGGTGGACTGCA
CACCGCGCGCCACGTCGTAGTGCTCTTGACCCAATACCATCGGATCCAGTTGGCGCGAAG
TAGAATCAAGCGGATCGACTGCCGGGTAAATACCCAAAGAGGCAATATCGCGGCTCAATA
CGACGGTTGCGTCCAAGTGGGCGAACGTTGTTGCCGGAGACGGGTCGGTCAAGTCGTCCG
CAGGTACATATACGGCTTGGATGGAAGTAATAGAACCGGTTTGGGTAGAGGTAATACGCT
CCTGCAAACGACCCATTTCTTCTGCCAATGTCGGTTGGTAGCCCACTGCAGACGGCATAC
GACCCAACAATGCGGATACTTCGGTACCAGCCAGGGTGTAACGGTAGATGTTGTCCACGA
AGAACAATACGTCGCGGCCTTTGCCGTTTTCGTCTTTTTCGTCACGGAAGTATTCCGCCA
TGGTCAAACCGGTCAATGCGACGCGCAAACGGTTGCCCGGAGGTTCGTTCATCTGACCGT
AAACCATTGCCACTTTATCCAATACGTTGGAATCTTTCATCTCGTGGTAGAAGTCGTTAC
CTTCGCGGGTACGCTCACCCACGCCTGCGAACACGGACAAGCCGCTGTGCGCTTTGGCGA
TGTTGTTGATCAATTCCATCATGTTCACGGTTTTACCCACACCGGCACCGCCGAACAGAC
CTACTTTACCGCCTTTGGCAAACGGACACAGCAAGTCAATCACTTTAATGCCCGTTTCGA
GCAATTCGGTTGTGGAAGACAGTTCGTCAAACTTAGGGGCAGCTTGGTGGATGGCACGGC
TCTTGTCGGTATCGATCGGACCTGCTTCGTCAACAGGCGTTCCCAATACATCGACAATGC
GTCCCAACGTACCTTTACCTACCGGCACAGTAATGGGCGCACCGGTATTGCTCACAGTCA
TGCCGCGTTTCAAACCGTCCGAGCTGCCCATCGCAATGGCACGGACTACGCCGTCGCCCA
AAAGCTGTTGGACTTCCAAAGTCAGACCGTTTTCGTCTAATTTCAAAGCGTCGTAAACGC

## Appendix A

GCGGAATCATGTCGCGTGGAAATTCCACGTCAACAACCGCACCGATAATTTGTACGATTT
TGCCTTGGCTCATTATCGTATCCTAATTTCCGTACAGGATTCAGACGGCATCAGACAGCC
GCCGCACCTGCTACAATTTCTGACAATTCCGTGGTAATCGCAGCTTGACGCGATTTGTTA
TATACCAAACGCAACTCTTTGATGGCATTGCCTGCATTGTCTGTTGCAGCTTTCATGGCA
ACCATGCGGGCTGCCTGTTCGGATGCCATATTGTCGCTCAACGCCTGATAAACCACAGAC
TCTAAATAGCGGCGAACCAGATATTCCAACACTGCAAGTGCAGTCGGTTCGTAGCGGTAT
TCCCAGCTGAACGGTGATTTGGGAGCTGAATCGCCAATCACGTTCTCACCGATAGGCAGC
AATACTTCCATTCTCGGTTCTTGACGCATGGTATTGACAAAACCCGAATACACCAGATGG
ATTCTGTCAATTTCATGTTTCTCATACCGTTGGAAGAGTTCTGTCAAAGGTCCGAGCAGC
ATTTCCATTTTTGGGGTATCGCCCAAATTTACGGCACTGGCAACCACATTCAGACCAATG
CTCTGACACGCCATCAGACCTTTACTGCCAAAGCATACGATTTCCTCTTCAATACCTTGA
TTCCGATACTCTTGAACTTGTGCCAAAAACTTTTTCAGCACGTTGGCGTTCAAACCGCCA
CACAAACCCTTATCAGACGTAATCAAAATAAAACCGACACGTCTGATTTCCCGATGAGAT
TCCAGTAACGGAATACCATGATCGGTATTGGTTTGCGCAAGATGGCTCATCACCATACGC
ACTTTTTCGGCATACGGACGCGCCAAACGCATCCGTTCCTGAGTCTTCCGCATTTTAGAG
GTTGACACCATCTGCATCGCTTTAGTGATCTTTTGGGTATTCTGAACACTGCGGATTTTG
GTGAGAATCTCTTTTCCTACTGCCATTTCAGACTCCTTTCACTTCAAGCCTTATGCCTGA
TAGGCGTAAGAAGATTTGAAGGATTTCATGGCTGCTTCAAGCGTTTTCTCGCTCTCGTCG
GACATTGCACCTGAAGCATTGACGGCTTCCAAAACTTCCGGATGTTGGGTACGGACAAAG
CTCAAAAATTCAGATTCAAAAGCCAGAGCTTTGGCAACCGGAACATCAGAATACGAACCG
TTGTTGATTGCCCAAAGGGTCAAAGCCATTTCAGCCGTATTCAACGTACTGAACTGTTTC
TGTTTCATCAGTTCGGTTACGACTTCGCCATGCTCCAATTGTTTGCGCGTAGCTTCATCC
AAATCGGATGCAAATTGCGAGAACGCCGCCAATTCACGATATTGTGCCAACGCCAAACGG
ATACCGCCACCCAGCTTTTTAATCACTTTGGTTTGTGCAGCACCGCCTACGCGGGATACG
GAAATACCGGCATTGATTGCAGGACGGATACCGGCGTTGAAGAGGTCGGTTTCCAAGAAA
ATCTGACCGTCGGTAATCGAAATGACGTTAGTCGGAACGAAAGCAGATACGTCGCCCGCT
TGGGTTTCGATAATCGGCAACGCGGTCAGAGAACCGGTTTTGCCTTTTACTTCGCCGTTG
GTCAATTTCTCCACTTCGTGTTCATTGACACGTGCCGCACGTTCCAACAGACGGGAGTGC
AGGTAGAACACATCGCCGGGATAGGCTTCGCGGCCGGGCGGACGGCGCAAAAGCAGGGAA
ATTTGACGGTAAGCCACAGCCTGTTTGGACAAATCGTCATAAACAATCAAGGCATCTTCG
CCACGATCGCGGAAGAATTCACCCATCGTACAACCGGAGTAAGGTGCGATATATTGCAAT
GCCGCCGCTTCAGATGCAGTTGCAGCAACCACGATGGTATGCTCCATCGCGCCATGCTCT
TCCAATTTGCGGACCACGTTGGCAATAGAAGATGCTTTTTGACCGATAGCGACATAGATA
CAGATAACACCCGTACCTTTTTGGTTGACGATGGCATCCAATGCTACGGCCGTTTTACCT
GTCTGACGGTCGCCAATAATCAACTCACGCTGACCGCGACCGACAGGAACCATAGAGTCA
ATCGCCTTCAGACCGGTTTGCATCGGCTGGTCAACCGATTTGCGCGCAATCACGCCCGGT
GCGATTTTTTCGATAGGGGCGGTCAAAGTTGTATTAATCGGGCCTTTGCCGTCGATAGGC
CGACCCAATGCATCAACGACGCGTCCGACCAGTTCGCGTCCGACCGGCACTTCCAAGATA
CGACCGGTACAGGTAACCGTGTCGCCTTCTTTAATGTGTTCGTACTCGCCCAACACTACG
GCGCCGACGGAGTCGCGCTCCAGGTTCATCGCCAAGCCGAAAGTGTTACCCGGGAATTCG
AGCATCTCACCTTGCATTGCATCTGACAAACCATGGATGCGAACGATACCGTCAGTTACC
GAAATTACCGTACCACAGGTACGCACTTCGGCATTTACAGACAGATTTTCGATCTTGGCT
TTAATCAAATCGCTAATTTCAGCAGGATTAAGCTGCATGAAAACTCTCCTAATTCGTCAT
AGTCGTGTACAAGGCACTCAATTTGCCTTGTACAGACAAATCCAAAACCTGATCACCCAC
TTCAACTTTTATGCCGCCAATCAGCTCCGGTTCGATTTCGACAGAGATTTTCAGCTCGCT
GTCGAAACGCTTATTCAGCATTTGCACCAACTCGCCGACCTGTTTGTCGGTCAACGGATA
GGCACTGTAAATGACGGCAGATTTGATATGGTTGAATGATAAGGTCAAGTCTTGATATTG
AGCATATACTTCCGGCAATATCGACAAACGTTTCTGCCCGGCCAAGACGATAACAAAGTT
TTTCAACTCCTTGTCTTTCAAACCGACCAAATCGATGAGGATATCTGCTTTTTCTGAAGC
ATTCGTTTCAGGACGGTCAATCAATGAAGCCACCTTCCCTTCCTGAACAACCGCCGCAAG
TTTTTCCAGTCCGCCCAACCAAGACTCAATTTGGTTTTTTTCCTGAGCCAGACCGAACAA
TGCCTTTGCATAAGGTCTGGCAATCGTTGCGAACTCTGCCATAAGATTACAGCTCCTGTT
TCAGGGTATCGAGCAGTTTTGCGTGTTTGGAAGCATCGACTTCGCTGCGCAAAATAGATT
CGGCACCTTTGACAGCCAACACGGCAACCTGCTCGCGCAGGGATTCGCGTGCGCGGAACA
ATTCCTGCTCCACATCGGCCTTTGCCTGAGCTGCAATGCGCGCCGCCTCGGAAGAAGCCT
GTTCTTTGGCTTCTTCGACAATTTTGGCGGCACGTTTTTCGGCGTTGGCAACCATTTCGG
AAACCTGATTACGCCCTTCTGCCAAGAGTTCTGCAACCTTTTTTTCAGCCTGCTCAAAAT
CGCTTTTACCACGCTCGGCGGCAGCCAAGCCTTCGGCGACTTTTGCGGCACGCTCATCCA
AAGCTTTTGCAATCGGCGGCCACACGAATTTCATGGTAAACCATACCAAACCGAAAAAGA
CGATGATTTGAGCGAATAATGTTGCATTGATATTCACGTTACTTAACCTTCGTACTGGGG
TTAATCAAACAGGCTGCGCCTGTACGGAACGGACGAATCCGTCCTGATTATGCACCTGCA
AACGGGTTAACGAAGGCGAACAGCAGTGCAATGGCGACACCAATCAAGAATGCGGCATCA
ATCAAACCGGCAATCAGGAACAGTTTGGTTTGCAGCGGACCGATCAGTTCGGGCTGACGG
GCAGAAGACTCCAAATATTTAGAACCGACCATTGCGATACCGATAGAGGCACCCAATGCA
CCCAATGCAACGATCAAACCACATGCGATAGCAATCAAACCCATTTTAAACTCCTTAAAG
AAACAAAGGTTAAACTACAAAAACAAACTACTTAGGAAAATCAGTGCGCATCATGTGCCT
GTCCGATATAGACGAACGCCAACGCCATGAAAATAAACGCCTGCAGGGTAATCACCAAAA
TATGGAAAATCGCCCATGCCAAACCGGCAATAATGTGGAATACAAACAGAATCGGATCCA
TGACTTCGACGCTGCCGGAAGCCGCCCAAGCACCGCCAAGCAAGGCTATCAACAAGAATA
CCAATTCGCCCGCATACATATTGCCGAACAACCGCATACCGTGGGATACGGTTTTAGAAA
GAAACTCGACCAAATTCAACAGAAAGTTCGCAGGTGCGAGTTTTGCACCGAACGGCGCGC
TGAACAACTCGTGAAACCAGCCACCCAATCCTTTGATTTTGATGTTGTAATAGATACAAA
TCAGCAACACGCCGACAGCGAGTGCCAAAGTGGTGTTCAAATCGGCAGTCGGTACGACGC
GCAGCAGGGCGTGATGGTTGCCGGTAATGCCCTGCCATACCATCGGCAGCAAATCGACCG
GCAGCATATCCATCGCGTTCATCAGAAAAATCCAGACAAACAGCGTCAGACCCAACGGCG

## Appendix A

CGACGGCTTTTCTAGACTTTTCGTTGTGAATGATGCTCTTACACATATCGTCCACAAACT
CAAACAAGATTTCCACTGCGGCCTGGAAACGTCCGGGAACGCCTGCCGTCGCTTTTTTG
CACCGCGCCACAACAGAAAGCTGCCGATTACGCCCAACAGGACGGCAAAAAAGACGGCAT
CAAGGTTAATAAACGAAAAATCAGCAATGTTTTTCAGTCCCTGACCCTGAGTAACATCCG
ACAAACTGGTCAAGCTCTGCAAGTGGTGCTTGATGTAGTCGGCAGCGGTAATGGTTTCAC
CTGCCATAATCTTTCACTCTCAACAATACTAAAAAAACCAAATGGCTGACACCGAGCAGC
CCCATCAGAAACGGGGCGAACACCAGCGATTGATGCCATATTGCAAATACGGCAAGCATG
GACAACAGCGACAGCACTACTTTTAAAATCTCTCCGAAGACGAACATCCTGCTTTGCAGG
AAGGGGTTTCCCCTGAAAAGTTTTAAAAGTAAAACTGCAACAAACGTGGGAAGCAGGTAG
GACAAACCGCCACCGACCGCCGAAAGGAATCCGGCAAAACCCCATACAGCAAAGGCAACT
GCGGCGCATATGGACAATACGGCGGATTGTAGGATGATAATCTGCTTCATAAAGGGAATG
TTTCCGCCTCGGATTTGGGGCGCGGCTAATATAATTTAGAAGCCTTATTACGTCAAGCGA
CAGTTAATCTTTGTGAAACAACGTATCCCAATCCGCCGCGCTCGCCGCCTGAATAACGGC
GACAGGTGTCATTCTAACACACATTACATATAATTACAGGATATTAAGGAGTTTGTCCGC
AATTTCTTTACATTTTTAATGTTCTTACGTGATTTGTTTGCTTTACGTGGAAATAATAAA
AAATCAACGCGAAATTGTAGCAGTTTATCGGTCGGATTGTCGGCAGTTTGGGGAATTTGC
TCAATAAATAAAAGGTCGTCTGAAAATATTTTCAGACGACCTTTTCCGAATAAAGGATTA
GCAACTGCCTGCCGCTTTAAGCAAAGCATTGCATTGACTTTTGCCTTTGTGCGTTCCGCC
TCCCAAACAAATTGCATCGGAAGTGGTAACGCCGATTGTGCTGATTACACTGGTAACATA
GCATTGGCTCACGCGCTTACCCACAGTTGCGGTAAAGTTGATGCGTATGCCTTCATTGTT
GCGGTTGCTGATTTTTACGGCATTTGGGCTGACGCCCAAGGCAAACGCGGCACGTTCCTG
AAGTTTCTAGTCGGAAACGGTTACATTATTGATTGAGCCGCAACCTGCTAATGCCAACGC
AACGAACGCAGCCGAAACGATGATGCGTGTGTTCATAATTTCCTCGAAAATTAAAAATGA
AAACAGGAAAACGATTCTTACGTGAAGCAGAAAAAATGTCAATAGAATTATATTTCCCAC
TTAAAATCTGGAAAGCTATTCTCTATATTTCAGACGGTATATCCCGCAAAATTAAGGCCG
GTAATCTATGCCCAACTGCTCCAGCAGGTGGCCGAACGTTTCAGGCGTATCGAAATACAG
GACAATCCTGCCTTTTTTGTGGTTGGCGGTTTTGACTTCAGCGTTGACACCCAGTTTTTC
AGTCAGCAAATCATTCAGGCGGCCGATGTCGGCGGCGGCAGTCTTTTTGGGCTCGGGACG
TTTGTTTTGAAGGGCGGCCTGGCTGCGGCGTTCGACTTCGCGCACCGACCAGCCGTTTTT
GACGGCCTTTTGCGCCAATTCGAGCTGTTCGACGACGGGCAGGGTCAGCAATGCGCGGGC
GTGCCCCATTTCGAGGCGGCGTTGGTAAAGCATTTCCTGCACGGGTTCGGGCAGGCTTAA
AAGGCGCAGGCTGTTGGAAATCGCGCTTCGGCTTTTACCGACGGCTTGGGCGATGGTTTC
GTGGGTCAGCCCGAACTCGTCGGCAAGGCGTTTCAAGCCTTGTGCTTCTTCGATGGGGTT
GAGGTTTTCGCGCTGGAGGTTTTCGATCAAACCCATTGCCAATGCGGTTTCGTCGCTGAT
GGTTTTGATAACGGCGGGGATTTCGGTCAGGCCGGCAATCTGTGCGGCGCGCCAACGGCG
TTCGCCTGCAATCAGTTCGTATCGGGACAGTCCGTGTTCGCGCACGATGACGGGCTGTAT
CACGCCTTGCGCCTTAATCGAATCTGCCAGTTCCTGCAAGGCTTCGTCATCGATTTGAAC
ACGCGCCTGATAGCGGCCGGGCCGGATATCTTTAACCGCAACCGTGGTCAATCGGTCGCC
GCTGCTGTTGTCCGCGCCGTTGGCGAGCAGCGAATCCAAGCCGCGCCCCAATCCGCCTTT
TACTTTTGCCATACCGCCCTCCCGTGCCTATTCAGATAGGATGTTAAATCGGGTATTTTA
TCGGATATTGGGTGTTGCCGACAATTTGTATCCGCGTTTATCGGATTTCTGTTTTTTCAC
TATAATAGCCGGTTTGCCGTTGCAGGCGGTTTTATGGGAAAGGCGGATGATGGTACGGCG
TTTGATAATCGGCATCAGCGGGGCGAGCGGTTTCCAATACGGCGTGAAGGCTTTGGAACT
TTTGCGCGCGCAAGATGTCGAAACGCACCTTGTGGTATCGAAAGGTGCGGAGATGGCGCG
CGCTTCGGAAACGGCTTATGCGAGAGACGAGGTATATGCCTTGGCGGACTTCGTGCATCC
GATCGGCAATATCGGGGCGTGCATTGCCAGCGGTACGTTTAAAACGGATGGGATGCTGGT
CGCCCCCTGTTCGATGCGGACGCTTGCCTCTGTCGCGCACGGCTTCGGCGACAATCTGCT
GACGCGTGCGGCGGATGTGGTTTTGAAGGAAAGGCGGCGGCTGGTGCTGATGGTGCGCGA
AACGCCGCTGAACCTTGCCCATTTGGACAATATGAAGCGGGTAACGGAAATGGGCGGCGT
GGTGTTTCCCCCTGTTCCTGCGATGTACCGCAAACCGCAGACGGCGGACGACATAGTGGC
GCACAGTGTTGCACACGCTTTGTCGCTGTTCGGAATCGATACGCCGGATTCGGCGGAATG
GCAGGGAATGGCGGATTAAAGGACAAAAATGCCGTCTGAACACGGATACAGTTCAGACGG
CATCATTTTATACGACTGCCTTATTTGGCTGCGCCTTCATTCCATGCGGCAGGGGATTTG
TAGCCCTCGAAGCGTTTGTGCGCGTAGGCTTTGAACGCGTCGGAGTTATAGGCCTCGGTT
ACGTCTTTAAGCCATTGGCTGTCTTTGTCGGCGGTTTTGACGGCAGACCAGTTGACATAG
GCAAAGCTCGGTTCTTGGAACAGGGCTTCGGTCAGCTTCATGCCGCTGCTTATGGCGTAG
TTGCCGTTGACGACGGCAAAATCCACGTCGGCGCGGCTACGCGGCAGTTGCGCGGCTTCA
AGCTCGACGATTTTGATGTTTTTCAGGTTCTCGGCGATGTCCGCTTTGGATGCGGTCAAC
GGATTGATGCCGTCTTTGAGTTTGATCCAACCCAGTTCGTCGAGCATCACCAAGACGCGG
GCGAAGTTGGACGGGTCGTTGGGCGCGGATACGGTGCTGCCGTCTTTGACTTCTTCCAGC
GATTTCAGCTTGCCCGGGTACAGTCCCAAAGGCGCGGTCGGCACTTGGAAGACTTCGGTG
ATGTCCAGATTGTGTTCTTTTTTGAAGTCGTCAAGATAGGGTTTGTGTTGGAAGACGTTG
ATGTCCAACTCGCCCTCAGCCAATGCCAGATTCGGGCGTACATAGTCGGTAAACTCGACC
AGTTTGACGGTGTAGCCTTTTTTCTCCAGCTCGGCTTGGATTTGTTCTTTGACCATATCG
CCGAAGTCGCCGACGGTCGTGCCGAAGACGATTTCTTTTTTCGCCGCGCCGTTGTCGGCG
GCGGCAGAAGCGGATGCGGCGGGCGCGCTGTCTTTTTGACCGCCGCAGGCGGCGAGGATG
AGCGCGAGTGCGGCGGCGGAAAGGGTTTTGAAGAAGGTTTTCATATTTTCTCCTGATGTT
GTGGCAGTTTCAAACAAAAATGACGGGCAGGGAGTCCTGCCGTCCGGATTCGGCGTTCAG
ACGGCATTTGCCGCGAACAGGGGGATTTTATAGCATTTTTCGGATAGCGGTGGGGGTTTT
GGCGTTCAGACGGCATTCGGGTTCAACGTTTGTCGAGTTTCCGCGCCAACGCGTTGCCGG
TGCTTTGAATCAGGATGACCAGCAGCACGAGGAGGGCGACGATGAAGATGATGACTTCGG
TTTGGTAGCGGTAGTAGCCGTAGCGGATGGCGAGGTCGCCCAAGCCGCCGCCGCCTATCA
TCCCTGCCGCCGCGCTGTATGACAAAAGCCCGATGGCAAGCACGGTAATGCTGGAAACCA
TGCCCGCGCGCGCTTCGTTCAAGAGGACTTTGCAGACGATGGCAATCGGCGGCGCACCCA
TCGCGGCGGCGGCTTCAATTACGCCTTTGGGGACTTCGCGCAGGTTTTGTTCCACCAGTC

## Appendix A

GGGCAAAATAAAACAATCCCGACACGCTCAACACCAGCGAGGCGGCAACCGGACCGATGG
TGCTGCCGACGATGGCGCGTGTGGCGGGTATCATCGCAATCATCAGGATGACGAAGGGGA
AGGCGCGCATGAGGTTGACGAGGTTGTCGAGCAGGAAGTTCACCAGCTTGTTGTAATGCA
GTTGGCGGCTGGAGGTTACGAAGAGCAGCACGCCCAGCAGCGTGCCGAAGATGACGGCGA
ATGTGGTGGACAAGCCGACCATCACGAAGGTTTCGCCCAAGGCGCGGAAGATTTCGTCTT
TCATGCCGACGATGGTGGAAACGGCTTGTTGGAATGTTAAGTCTGCCATATCAGTCCTCC
CGAATCAGTTCGCGCCCGATGTCGGATTGGGCGTGGATTTGGTTGCCGCGTACTTCGACG
ATTTCGACGACTTTGCCTTTATCCAAGAGGGCGGCGCGGTCGCACAGGCGGCGGATGACG
CTCATTTCGTGGGTTACGATGACGATGGTTACGTTGAAGCGTTTGTTGATGTCTTCCAAA
CATTCCAAGACGCTGCGCGTGGTGGCGGGGTCGAGGGCGGAAGTGGGTTCGTCTGCGAGG
ATGACTTGGGGTTTGGGCGCGAGTGCGCGGGCGATGCCGACACGTTGTTTCTGCCCGCCG
GAAAGCTGGGCGGGATAGTGGCCGGCGCGTTCGGTCAAGCCGACGATTTCAAGGCATTCT
TTAACGCGCGCTTTGATTTTTTCAGACGGCCATCCGGCGATTTCCAAAGGAAAGGCAACA
TTGTCGGCAACGGTGCGGTTGCTCAAAAGATTAAACTGCTGAAACACCATGCCGATATTC
TGCCGAGCCTGACGCAATGCGGCGGCATCGAGCGCGGTCAGCTCTTGTCCGCAGACGTTG
ACCTTGCCGCTGTCGGGGCGTTCCAACAGGTTAATCAGGCGCAACAGGGTGGATTTGCCT
GCACCCGAATAACCCATCAGCCCGAAGATTTCGCCGTCGCGGATTTCGAGGCTGGTCGGC
TCGACGGCGGCAAAACGGGTCTTGTCGCGCGTTTGGTAATGCTTGGAAACCTTGTCCAAA
ATAATCATTGTCTTTCCCATACAACAAAGCCCGATGTCGGACACAACGGGCGCGGAAGAT
AAAGCTGAAATTGTCGGAACGCTTTAGCTGTTATGCCCGCAAGCTGTGTCAAATCGGCAG
GTTAATTTTCGTAGGATATTATCGGGAAAGCATTTTTTTGTCAATAAAGCAGGAAGCGGG
CAACCATTTCGGACAATGCCGTCTGAAACGGGCAAAGGCAGCGGTTCGCACCAAAACGGC
AAATAATTGAAAAACATATAGTGGATTAACAAAAATCAGGACAAGGCGACGAAGCCGCAG
ACAGTACAAATAGTACGGAACCGATTCACTTGGTGCTTCAGCACCTTAGAGAATCGTTCT
CTTTGAGCTAAGGCGAGGCAACGCCGTACTGGTTTTTGTTAATCCACTATAAATTATGTC
GGAAACATTCCAAAGGCGGTGCAGTTTCGGCATATAATTCGGGCAAACGCCTGTTCAGAC
GGCATTTTGTCTTTTCCAACCCTGACCGTTCAGGGTTCCGATTCTTAAGGAAATCCGATG
TACCTACCCTCTATGAAGCATTCCCTGCCGCTGCTGGCGGCCCTGGTGCTTGCCGCGTGT
TCTTCGACAAACACACTGCCAGCCGGCAAGACCCCGGCAGACAATATAGAAACTGCCGAC
CTTTCGGCAAGCGTTCCCACCCGCCCTGCCGAACCCGAAAGAAAAACGCTGGCAGATTAC
GGCGGCTACCCGTCCGCACTGGATGCAGTGAAACAGAAAAACGATGCCGCCGTCGCCGCC
TATTTGGAAAACGCCGGCGACAGCGCGATGGCGGAAAATGTCCGCAACGAGTGGCTGAAG
TCTTTGGGCGCACGCAGACAGTGGACGCTGTTTGCACAGGAATACGCCAAACTCGAACCG
GCAGGGCGCGCCCAAGAAGTCGAATGCTACGCCGATTCGAGCCGCAACGACTATACGCGT
GCCGCTGAACTGGTCAAAAATACGGGCAAACTGCCTTCGGGCTGCACCAAACTGTTGGAA
CAGGCAGCCGCATCCGGCTTGTTGGACGGCAACGACGCCTGGAGGCGCGTGCGCGGACTG
CTGGCCGGCCGCCAAACCACAGACGCACGCAACCTTGCCGCCGCATTGGGCAGCCCGTTT
GACGGCGGTACACAAGGTTCGCGCGAATATGCCCTGTTGAACGTCATCGGCAAAGAAGCA
CGCAAATCGCCGAATGCCGCCGCCCTGCTGTCCGAAATGGAAAGCGGTTTAAGCCTCGAA
CAACGCAGTTTCGCGTGGGGCGTATTGGGGCATTATCAGTCGCAAAACCTCAATGTGCCT
GCCGCCTTGGACTATTACGGCAAGGTTGCCGACCGCCGCCAACTGACCGACGACCAAATC
GAGTGGTACGCCCGCGCCGCCTTGCGCGCCCGACGTTGGGACGAGCTGGCCTCCGTTATC
TCGCATATGCCCGAAAAACTGCAAAAAAGCCCGACCTGGCTCTACTGGCTGGCACGCAGC
CGCGCCGCAACGGGCAACACGCAAGAGGCGGAAAAACTTTACAAACAGGCGGCAGCGACG
GGCAGGAATTTTTATGCGGTGCTGGCAGGGGAAGAATTGGGTCGGAAAATCGATACGCGC
AACAATGTGCCCGATGCCGGCAAAAACAGCGTCCGCCGCATGGCGGAAGACGGTGCAGTC
AAACGCGCACTGGTACTGTTCCAAAACAGCCAATCTGCCGGTGATGCAAAAATGCGCCGT
CAGGCTCAGGCGGAATGGCGTTTTGCCACACGCGGCTTTGACGAAGACAAGCTGCTGACC
GCCGCGCAAACCGCGTTCGACCACGGTTTTTACGATATGGCGGTCAACAGCGCGGAACGC
~~ACCGACCGCAAACTCAACTACACCTTGCGCTATATTTCGCCGTTTAAAGACACGGTAATC~~
CGCCACGCGCAAAATGTTAATGTCGATCCGGCTTGGGTTTATGGGCTGATTCGTCAGGAA
AGCCGCTTCGTTATAGGCGCGCAATCCCGCGTAGGCGCGCAGGGGCTGATGCAGGTTATG
CCTGCCACCGCGCGCGAAATCGCCGGCAAAATCGGTATGGATGCCGCACAACTTTACACC
GCCGACGGCAATATCCGTATGGGGACGTGGTATATGGCGGACACCAAACGCCGCCTGCAA
AACAACGAAGTCCTCGCCACCGCAGGCTATAACGCCGGTCCCGGCAGGGCGCGCCGATGG
CAGGCGGACACGCCCCTCGAAGGCGCGGTATATGCCGAAACCATCCCGTTTTCCGAAACG
CGCGACTATGTCAAAAAAGTGATGGCCAATGCCGCCTACTACGCCGCCCTCTTCGGCGCG
CCGCACATCCCGCTCAAACAGCGTATGGGCATTGTTCCTGCACGCTGACGTACCGATGCC
GTCTGAAACCCGCCCGGTCTTTCAGACGGCATTTTTATCCCGAACGGCATTGACGGCGAA
CCATAAATATAAGACAATCCGAAAATTGTTTTTCCTGCTTTTTCAAGCAGCTTGACACGG
CACAAGCCGACCCGTTAGGAGGTGATGTTTCCGTCACGGCGCGTATCCCGCCGCCGCAAG
GCACAGCGATACGGTAAACTTTCAACACCGTCTGCCCTACCCTTTCCACCGATATGATGG
GCAGATGAAACAACCGAATTTATTAAAGGAAATAAAATGCCTGCAATCCGCGTAAAAGAG
AATGAACCATTTGAAGTCGCTATGCGCCGTTTCAAACGCGCCGTAGAAAAAACCGGCCTG
CTGACCGAGCTGCGCGCCCGCGAAGCCTACGAAAAACCGACTACCGAACGCAAACGCAAA
AAAGCGGCAGCCGTAAAACGCCTGCAAAAACGCCTGCGCAGCCAACAACTGCCGCCCAAA
ATGTACTAAACGTTCAAGTACAGATTACAGGTCAGCCCTGTGATATGAGGACACACCGCA
AGACCTGCTCTGCGGTGTGTTTTGCTTTTCAGACGGCATCGAAACCCGCCGTTTCCATCC
GACATCCCAGCGAGGACATCATGAGCCTGAAAATCCGCCTTACCGAAGACATGAAAACCG
CGATGCGCGCCAAAGACCAAGTTTCCCTCGGCACCATCCGCCTCATCAACGCCGCCGTCA
AACAGTTTGAAGTGGACGAACGCACCGAAGCCGACGATGCCAAAATCACCGCCATCCTGA
CCAAAATGGTCAAACAGCGAAAAGACAGCGCGAAAATCTACACTGAAGCCGGCCGTCAGG
ATTTGGCAGACAAAGAAAACGCCGAAATCGAGGTACTGCACCGCTACCTTCCCCAAATGC
TTTCCGCCGGCGAAATCCGTACCGAGGTCGAAGCTGCCGTTGCCGAAACCGGCGCGGCAG
GTATGGCGGATATGGGTAAAGTCATGGGGCTGCTGAAAACCCGCCTCGCAGGTAAAGCCG

## Appendix A

ACATGGGCGAAGTCAACAAAATCCTGAAAGCCGTGCTGACCGCCTGATTGCCCGAATATC
GGACAAAATGCCGTCTGAAGCCCGTATCGCAGGTTCAGACGGCATTTTCAATATCCCAAT
ATCGAATCGGCAGGGGCAACACGGTTTTGATACGCCGAAACGGGTTTTGCCGATAAACAG
ATTCCGTTTGCGCCCCATCGGACAAAATGCCGTCTGAAACACGATTCCGTTCAGACGGCA
TAGATTTATTTGACCAATTTCAAGCCTTTTTTGGCGGGTCGGGGCGCGGTTTCGGCAGAG
GTGTTTTCAGGCGGCGTATCGGGGCGGTACGCTTCCAACTCAAACCCCATACCTTCTCCG
GTCTCCCGTGCGAAAAGGCTGAGGACGTGTCCGACAGGTATCCATATATCGTGCGCCTGT
CCGCCGAAGCGGGCGGAAAAGCTGATCCAATCGTTGTCGATTTGAAGGTTTTGCGTGGCG
GTCGCGCCGATGTTGAGCATAATTTCGTTGTCGCGGACGTACTGCATGGGGACGCGCGTG
TGTTCGTTGACCCAGACAAGGATGTGCGGTGTGAGGCTGTTGTCGCTGCACCATTCGCAG
AGGGCGCGGAGGATGTAGGGTTTGGTGGAAGTGGGCATAATGGGTTCCGTGTTGTACGCC
AAAATAGGAAAATGCCTGCAAAACGGTGGGTTTTGCAAGCATTTCGGACTTATTTGCGCA
TGGCTTTTTCGGCGGGTGTCAGTGCTTCGATAAAGGCTTCGCGCTGGAAGATGCGCTCGG
CGTATTTGAGCAGCGGCGCGGCACTTTTGCCCAGTTTGACATCGTAGTGGTCGAGCCGCC
ACAGCAGCGGAGCAAGGGCGACATCAATCATAGAAAAATCTTCGCCGAGGATGTATTTGC
TTTTGCTGAACGAAGGGGCAAGCATGGTCAGACCGTTGCCGATGGCTTCGCGCGCTTTTG
CCTGTTCCTTGTTGGTGGCGGCGGGGTTTTCTAACACTTGGACGTGGTTGAACAATTCTT
TTTCCATACGGTACAGCACCAGCCGGCCCCGACCGCGCATAACGGGATCGCCGGGCATCA
GCTGCGGATGGGGGAAGCGTTCGTCAATGTATTCGTTGATGATATTGGACTCGTGCAGCA
CCAAATCGCGCTCGACCAGCACGGGAACTTGGTTATACGGATTCATGACGGCGAGGTCTT
CGGGTTTGTTGTAAATATCGACGTCTTTGATTTCAAAATCCATACCTTTTTCGTACAAAA
CGAAGCGGCAGCGGTGGCTGAAGGGGCAGGTAATGCCGGAATAGAGGGTCATCATAATAA
TTGTCGCTCCTGTGTGATGCCTGCAAAACGGCTGATTTATAGTGGATTAACAAAAACCAG
TACGGCGTTGCCTCGCCTTAGCTCAAAGAGAACGATTCTCTAAGGTGCTGAAGCACCGAG
TGAATCGGTTCCGTACTATTTGTACTGTCTGCGGCTTCGTCGCCTTGTCCTGATTTTTGT
TAATCCACTATATAAAGGTTTAATCGCGCAATTATACGCGATTTCCGGCACTTAATCCAG
AAATTCGGCTCAATCTGTTGTTTTTATATATTTTCCCCGATTTTCCGTATCAGTGCGAA
CTTACTGTCTTTGTTGCGCGGACGCGCACCCTGCCAAACCGTCTGCCAGCCTTGCGGCGC
ATCCGCATTTTGGGGCAGGAGGACGATGCGGTAGCGGCATTGTACATCGCCGACGCGGTG
CGGCAATGTGCCGTACTGCGTCCAAACAATCCGCGTGTGCAGGTCGCCGCCGCCTATGCC
GATACACTCGATGCCGTCTGAAAGCTCCCGTTTCAATTCCGGGGAAAGCGATGCCTCCAT
ACTCCGGACGACCGGCGCGTGGCTTTTCGCCGCGTCCAGCCACGGCAGGAACAGCGTCAT
CAGCAAAGCCCAGGTCAGGGTAACGCCTGCCGCCCAGTTGGTAACCGCCTGCCTGCCGCG
TATGTTTTTCCGGGTAATCGCCCACAGCCACAAGGGTGTGAACAGTACGGCAACCGCCAT
CGGAATGGGATCGATATCAGGAACATAATACGGGCTGAAATAGGCGGCGCGTTCGGCAAG
CTTGGCGGGCCAGCCGTAATTCATGGCGAAAAAGCCCGTCCACAGGAACACGGCAAACAG
TCCGAACGCCATAATGCCGAACCAGTTGACAAACGCCGCCGCGCCGCGCCTCAGGCTGTC
CAGTTGCGCCGCGCCGAACAGGGCAAGCGGCGGAAGCAGCCAGACGAGGTTATCCTGAAA
ACGCTGCGGATTGACGGCAAGCAGCACCAAAACGGCAAGCATCCAGACGACGCCCAAAAT
CCCCCAGTCGGTCGAAAACAGGCGCGTGCGGCAAACCGTCCAAACCGCCAGCGGCAGCGC
GGGCAATGCAAACCAAAGCAGGTTTTTCAGATAGTAAAACAAACTGAATGCCGTCTGAAC
GTGCCGCACGCCGCCGAACGTACCGAAAACGTGATAGTCGAGCCATTGCGCGAACAGCGC
GGGCTGCGTTTTTGCCAAGAGCAGCGGGTAAACGGTCATAAGCGGCAGGGCAAAGGCAAG
TGAGGCGACTGCCGTCAACATCAAACGCCTGCTTTGCCACGGACGGAAAAACATCAGTAC
GGGCAAGGGCAGCATCAGGGCAAATGCTGCCGGATAAGCTGCTGCCAACGACATCAGCGT
CCAGCCCGTACCGAGCAGAAAAGAGGCGGCAATCACGCGCCGGCGAGCCAAAGAATAACC
GTGCAGCACCAGTCCGGCGGCGGCAAAGGCGGCGGCAGCGGGGTTGAGGAAATGGGCAAC
TGGAATCAGCCCGATACAGCCGATGAGAATCAGGACGACGCTGCGCCCGTGGTGTCTGCC
CAAAAAGTTGAAACCGGCAAAGCCGCAGGAAGTCAGTCCGATAACGGCAAAAAATACGCC
~~TGCAAACGGTGCGGGATCGTATGAGTCGGCAGCCCACGGCGACAGCAAATGTTTGAACGC~~
GGCGGCAACCCAAAGATACACGGGCGGTATGCCGAAATCGGTTTGACCGAACAGATGGGC
AACCAAGGGGGTGGGGCTGCCTGCCAGTGCTTCGACGGCGGTATAGACGGCAGGTTCGTC
AGGATTCCACAAATCGTGGGAAAACACGCCGGGCCACAACCAGGCAAACGCCATCAACAG
CAGCAGCCACGGCTTTTCGTGGGTTTTGGCGGGCGGGCGGGCATCGGGCGGGGTATAGGT
CAGCATAAGCGTGAGAAAAAAATGGACGGATTGCCAAGTGTAGCAAATATTCGCACAAAGG
TCGTGCAGAGACTGCTTCAGACGGCATCAGACACAAAAAGACCGGCAACAAAAAAGACTG
CACATGGCAGTCTTTGCAGATACTATCTTTTTCATAATATTTTTTCCTAGCCCAACACAG
TCCATAGCATATCAAAACTGTTGTTTTCAACCAGGATATATATCCCCAATCCTAAATAAA
CAACAGCAACAAACCATCTGCTATATTTTTCCAAAGTTTCTCCAACAGAAGGGACTTGTG
CTAATTTTTGGGCAGAAAAAACCAAGAGATAAATCATGACTAGAAAGGTAAGTAAAGCCA
CTATCAAATTCGCTAAATTTAAGGTAGTAAAATATGGACAAAGACACCAATATTGTCAG
CACCACAACTTGCAAAAGTAATCATAGCGACTAGAAAAATCAGGTTTTTATTATCTTTGC
GCAAACCCTCTTTGGCAATAGCCTCTCCATCAGAATCTCCTAAAAGCAAAACTTTGATGC
CTAGGAGAATTGGAATCAAGCCGAGCAAACCTAAAATCTCTTTACTAGGAATATAATCTA
AGACAAATGCAAAAAGTAAACTTAGCAATATCAGACTAACAGAGCCTAGAAATTGTCCTA
AATAGATGTTAATGATGTCTTTTCTACTTTTTCTTTTGGCAAAAAATAACATTAGGATAA
TAAGTAAGTCTACGGCTGTCCCAGAATACAGGATTATTGAAGTAACGACATTTTGAATCA
TAAAACATCTCATTCAAATATATTTTTAAATGTATTCAAACATTAAACCTTGTAGATGTC
AACTTCAACCCCGTCAAAATATAGTGGATTAACAAAAACCAGTACGGCGTTGCCTCGCCT
TAGCTCAAAGAGAACGATTCTCTAAGGTGCTGAAGCACCAAGTGAATCGGTTCCGTACTA
TTTGTACTGTCTGCGGCTTCGTCGCCTTGTCCTGATTTTTGTTAATCCACTATATAGATA
AGAAGTCAGTGTGCCAAATATTAAAAAGCCCTGCCATCGAAATGATGGCAGGGCTTAATT
CTTGCAAAGCGGCAATCAGCGTTTGAACAGGTTGCCGAATTTGTTGTTGAATTTGTCCAC
GCGGCCGGTGGTATCAACGATTTTTTGGGTGCCGGTATAGAACGGGTGGCACAGGGAGCA
AACCTCGATATTGAAGTTTTCTTTTTCCATCGCGGATTTGGTTGCGAATTTGTTGCCGCA

## Appendix A

AGAGCAGGTAACGTTGACTTCGTGGTAGTTCGGGTGAATACCTTGTTTCATTTGATTTCC
TTTCAAAAAAGCGGGCATAGGGGATGTACCTATGCTACAGACAAGTCCGACATTCTCGCT
ATTTTCTGTTGTTACGTCAAGAGTATATTCGATAAAATGTATAGTGGATTAACAAAAACC
AGTACAGCGTTGCCTCGCCTTGCCGTACTATCTGTACTGTCTGCGGCTTCGTTGCCTTGT
CCTAATTTTTGTTAATCCACTATAAAAAGTTCTTTTGAGGGAGGTTTGATGGGATCAAAA
TTCTTTTTCCTGCTGCTGCGTTTTGCCGGTTCGGGGTTGCCGCCGTCACATATGCGCGGC
ATCGGCATCGTCGGCAGACGGGTGCGCGGTTTTTTGGCGCGGCGGGTTTCTCCGCATATC
GGACGCGGGGTCAATATCGAACGCGGGGCGTATGTGTTTCCGGATACGGTTTTGGGCGAC
GGCTCGGGCATCGGGGCAAACTGTGAAATCTGCCGTGGGCTGGTGGTCGGCAAAAATGTG
ATGATGGAGCCGGAATGTCTGTTTTATTCAAATAACCACAAGTTTGACCGTTCAAAAAAC
GCTTTGAGGGCTACACGGAAATCCGTCCGATTACGTTGGAGGACGATGTCTGGCCGGGGC
ACAGGGTGATTGTAATGGCGGGCGTAACCGTCGGACGCGGTTCGGTCGTGGGCGCAGCGC
GGTGGTTACAAAAGACATTCCGCCCTACTCTTTGGCGGCAGGCAATCCGGCAGTGGTGAA
AAAGAATCTGCCGGAAGGTTGAATGCCGTCTGAACGTGTCGGGGCGGATGATCTGAAAAA
ACAGGAACATCGTTTCTGTTTTTTGCGCTTCAGACGGCATCGCTATTGCGCCACGCGGTA
TCGATTTCTTGGTAGAGTTTGCCGAAATCGGGTTCGCCGACGTAGGTTTTGAGGATTTCG
CCTTTTTTGCCGATAAGGACGGAAGTCGGATAAACCTGTGTGCCGAACGCCTGTCCGACA
GCTTTGTCCGCATCATACATGACGGTAAACGGCAAACCGTAGTCTTTGACATATTGGCGG
ACGCTTTCTATCGGATCGATGGGCTGGGCGACGGCAAGTACTTGGAAGTTTTTGTTTTTA
TAGTCATTTGCCGTTTTAATGATTTTGGGCATTTCGCTCACACAACCCGGACAGGAGGGA
AACCAAAAATTAATCAGGGTTACTTTGCCTTGCAGGTCGGCGTTGGAAACGGTTTTTCCG
TGCAGGTCGGGCAGGGAGAAGGCGGGCGCGGTTTTGCTGTCGGGGATGAGGACGATGGCA
AGGAGGATGCCGATCAGTGCGACGACGGCGGCGGTGAGTATTTTTTTCATTCGGACAAGG
CTTCCAATGCGCGGGCAAGGGTGGCGGGCAGGCTGACGGTGCGTTGTGTGGCGGCGTGGA
CGGGCATCAGGGTGATGTCGGCTTCTGCGGCGGTTTTGCCGTTTGGCAGTGTAATCGTCT
GGGTCAGCACAATACGGCGCGTGCCGGGGGTTTTCAGGCGGCATGAAAACTGCAATACGT
CGCCTTCGACGGCGGGGCGGCTGTATCGGATGTCGATGCGGGCGACAATCAGTATGAGGC
CTGCCAACTCGTGCAGCAGTCCGCGTTCTTCAAAAAACGCCCAGCGCGCTTCTTCGAAAA
ATTCGAGGTAGCGCGCATTGTTGACATGGCCGTAGCCGTCGAGATGGTAGTTGCGGACGG
TCAGCTTCATCAGTTCAGGTTGATGGGTTGGAAGGCTTCGCGGGCAAGCGGTTCGTGTTC
GAGGTCGGTGATGACGGTAGAAAGCTGGATGTCGAACCATTCGTTGAAAATGTCGGCATC
GAGCGCAGGCCACTCGCGTTCGTCTTCGCACCAGTCGGCAAGTTCGGCGGCGAAAATGTC
TTCAAAACGGGCTTCGATTTCGTCCCATACTTCGTCGGCGGTTTCGCACGGGCGGACAAG
GTAGGAATTGGCGTCGGCTTGGATGTCTTCAAGAGTCAGTCCGTCGAGGTGGTTGCCCGG
CAGGGTTTGCAGCCAGTTCCAAAAAGGTTCTAAAGGGATGAGGACGAATACGCTGCGGTT
GACTTCGTACATGGTTTTTCCTTTGCTGTCGCGCGGTATGCGCAAAAAAGAGATTATAGC
CCAATCTGTGGTTTCGGACTGTCCGTTCCGACAGAAGGGAATGCCGTCCGAACACGGATT
TTCAGACGGCATGGCTTTAAGGTTGTGTTCCAGGTTGCGTTTCGGCTTCCCCTGCTGCTT
CTGCCTGTGTTTCGGATACGGAATCTTCTTGAACGGCAGTTTCCGCCGCGCCGGTTTCGG
CACTTTCGACCAATTCGTCGATGTCGATGTTATCTTCCGTACCTTCGGCAGGTGTTGCAC
CGGTCTGCCGCGCACGGACTTTCATATAGAGGTCGCGCGTGTAGCTGTATTTGTCGATGG
CGGCTTCGTCCAGACTGTCGGTCAAATCGAGCAGGCCTTCGCGCGTACTGACGGCGGATA
CGGCAGTCGTGCCCCAGCGTCCGACAGGGGTGCGGAAGACGATATTCTTGGGCGAATAAA
CGGAGGTAATACCCGTGCCGAGCGCGTCGCGGACGGTGGACGGCCCTAAGACGGGCAACA
CGAAATAATTGCTGTTTTTCCATCCCCACGAGGCAAACGTGTCGCCCAAGGTGTTTTTAT
TGTCGGGAATGCCGCCCGCGCCGGCGATGTCGATAAGCCCGCCCAAACCGAAAGTGGTGT
TGATGCCGACGCGGACAAGGTCTTCGCTTGCGCGTTTGATGTCCAAGCGCAAGATATTGC
TGCCGAAGCTGACCACGTCGCACAGGTTGTTAAAAAAATTGGACACGCCGGCGCGGACGG
GTTTCGGCGCAACTTTGCGGTAGCCGCGCGCGGCAGGGGCGAAAATGTAGCGGTCGGCTT
GGTCGTTGAATTTGAAAACGGCGCGGTTGTAGCCTTCATAAGGGTCGGCGGGGCGGGTTT
CGGCAAATGCAGGGGCGGAAGCGAACCCGATCAGCAGGAGGAAGGCATAGGCGGTTTTTT
TCATGATTTCAGCCAGTCTTTGATTTCGTACAGTTCGGACAGCGCGCGCACGGATTCGGG
AATGCCGGTCAGCCTGACGCTGCCTTTGCAACCGCGCAGCACTTCGAGCAGCAGCGACAC
GCAGGCGGAATCGGCGCGTCCGACGCCGCTCAAATCAACCGCGCAGGTGTCTTTCAGACG
ACATTGCTGTCTGAAGCGGGTAAAAGCGGCGGCGGTCAGGGTTTTGACGGTGATGTCGCC
GCCGATGTGCAATATTCCGTTTTTGAGTTCTGTATGCATAGCGTTTGCTCGGAAAACCCA
TACCGCCCTCGGACGGTATGGTTTGTCGGTTATTTGCCGCCGTTTTTGGCTTTCAACTCG
GCAATCAGTCCGTCCACGCCTTTCGCTTTGATAATTTCGCCGAATTGGTTGCGGTACACG
GTAACCAGGCTCGCGCCTTCGATGGCGACGTTGTAGGTACGGTATTTACCGCCGCTTTGG
TAGGTGGTGAAGTCCATGTTGACGGGTTTTTGCCCGGGTACGCCGACTTCGGCGCGGACG
ATGATTTCTTTGCCGCCTTTATTGACGATGGGATTGTCTTTGACGTTGACGTTGGCGTTT
TTTAATTTCAGCATCGTGCCGGAATAGGTGCGGATCAGCAGGGTTTGAAATTCTTTGGCC
AACGCTTGTTTTTGCGCGTCGGACGCGGTGCGCCAAGGGTTGCCGACCGCCAATGCGGTC
ATACGTTGGAAATCGAAATAGGGAATCGCATAGGCTTCGGCTTTTTGGCGAGCGGTGTTG
GCATCGCCGTTTTTTAAGATGCTCAATACTTGAGTGGCGTTTTGACGGATTTGGCTTACC
GCGTCGGCAGGGGCGGCAAATGCCATGCCGATGCTCAAAATACCGATGCCCAATGCGCTG
ATGAGGGAGGATTTTTTCATGATTAAGTGTCCTAGTTTGAATATGATGGCATACGTTTAT
TCGGCGGCTTTTTCCGCATTGCCGCCGTCGGCATTTTTCTCGGCAAAACTCGTCATGAAT
TTGCCGATAAGGTTTTCCAGAACCATTGCAGAACTGGTTACGGAGATGGTGTCGCCGGCA
GCAAGGTTTTCCGTGTCGCCGCCCTGCTGCAGCCCGATGTACTGCTCGCCCAAAAGTCCC
GAAGTCAGGATTTGCGCGGAAACGTCGCTGCTGAACTGATACTTGCCGTCCAAATCGAGG
CGCACCCTCGCCTGATAGGATTTCGGGTCAAGTCCGATAGCGCCGACGCGCCCGACCAAT
ACGCCTGCGGATTTGACGGGGGCATTGACCTTCAAACCGCCGATGTCGCCGAAATCGGCA
TAAACGGCGTAAGTTTTGTCCGAACCGCCGAACGCCGCACCGCCGGCCACGCGGAAAGCG
AGAAAGGCAACCGCCGCCGCGCCAATCAGGACGAACAGTCCGACCCAAAATTCCAATATG

## Appendix A

TTCTTTTTCATTAAAGTTCCTTGAATATCCGATGTTCCGCGTTTCGTCTTCAGACGGCCT
GTCAATCTGTAAACATCCACGCGGTCAATATAAAATCGACCGCCAAAATCGTCAGGGCGG
ACGAAACCACCGTGCGCGTGCTGGCGCGCAAAATGCCTTCCGAAGTCGGGACGCAATGGA
AGCCCTGATGCACGGCAATCAGCGTTACCGCCACGCCGAACGCGGCGGATTTGATCAGAC
CGTTGATTACATCGTAATGTATCGTGATGTTGTTCTGCATTTGCGACCAGAAAATACCGC
TGTCCAAGCCCAGCCAGGTTACACCAACCAAATACGCACCGAAAATGCCCGCCACGTTGA
AAATCGAAGCCAAAAGCGGCATGGAAAACACGCCCGCCCAAAAGCGCGGCGCAACCACGC
GGGCGACAGGGTTTACCGCCATCACATTCATCGCTTCGAGCTGTTCGGTCGTTTTCATCA
GACCGATTTCGCTGGTCATCGCACCGCCCGCGCTGCTGGCAAACAAAATCGCTGCCAATA
CCGGACCCAGCTCGCGCAATAGCGAAGCCGCGACCATATAGCCCAAAATATCGGCGGATT
TGAATTTCGACAACTGCGTATAGCCCTGTAAACCCAAGACCATGCCGACAAACAGCCCCG
AAACGGCAACAATCAACACCGACAGCACACCGGCGAAATACACTTGGCGCACGCTCAGGC
GCGGACGGACGAAAGCCGTACCGGACTTCGCCAGAATGTTCAGCAGAAACAGCGTGATAC
TGCCGAGGGATTGAATAAGGCCGAGGGTTTTCGCCCCGACGGAACGGATAAAGTTCATAA
ATTTCTATGTGTAAAGTTCAACGGTTTCAGACGGCATCAACTCATTTATCCCAACAGGTC
CTGCTGCAACGACGTTTGCGCCGGATAACGGTATGCTACGGGGCCGTCTGCCAGCCCGCC
GACAAACTGGCGCACCCAAGGCGAATCCAGTTCGCGCATTTCCTGCGGCGAGCCGGAGAA
CATAATTTCGCCGTGCGCCAAGAAAATCACCTGATCGACGATTTCCAAAGATTTTTCAAT
GTCGTGCGTTACCATAATACTGGTCGAACGCAAAGCCTTGTTGACGCGGCTGATCAAGTG
GGCAATCACGCCCAAGGAAATCGGATCGAGGCCGGTAAACGGCTCGTCGTACAACATAAT
TTCAGGGTCGAGCGCAATCGTGCGGGCAAGCGCGACGCGGCGCGACATCCCGCCGGACAA
CTCGGACGGCATCAGGTTTTCCACACCGCGCAGACCGACCGCGTTCAATTTCAACAAAAC
CAAATCCCGAATCACCGCTTCCGGCAGGCGCGTCAGTTCGCGCATCGGAAAAGCGATATT
GTCGAATACCGACAAATCAGTAAACAGCGCGCCGTGTTGGAACAATACGCCCATACGGCG
GCGGTGTTCATACAACTCGTCAGCCGAAAAGCCCGCCAAATCCCGTCCTTCAATCAAAAC
CTGCCCGGACTGCGGACGAATCTGTCCTGTAATCAGTCGCATCAGCGTGGTTTTGCCGCT
GCCCGAACCGCCCATTACGGCAGCAAAATTGCCTTGCGGAATGCTGAAATTGATGTTCTT
CAGAATCGGGCGGTCGCCATACGCGAAGGCGACGTCTTTCATTTCGATAAAGGGGGATGG
GCTCATGTACGGACGGACGGTAGGTTTGACGGCGTGTATTTTAAGGCTTATCGGGAAGAC
GGGCAATTTTCAGACGGCATACGGACGGTAAATGTTGTGAAAATGCCGTTGTCGGCGGCG
GATTGTTTGCTGTGGCGAAAAATGTTATCTTTCAAATGATAACCTTTATCAGAAAACTAT
GGAAAAAGCAGAACATTTGAACAGCAGCCGGTTCGTCAATCTAGTCAAAAGCGGCGGCGG
CAGCTATGTGGAGGGCAGCTACCGTTTCGATACTTTGTCCAACGGCATTTCCATCCACGG
CGGCACAGTAACGGCACGGTGTGATTTTTGCAGCAGCCGCCTCGCCGAACCTTATGTGTC
GTTCGTGCTCTTGCTGGAAGGCAGTTTGGACTTCGGCATCAACCGCTGCCGCTTCCAAAT
CGATGCGGACGGCGGCAAGATTGTCCTAATTGCTGTCGGGGAAGAAGTCCTGTTCAGCCG
CTATCTTTACCGAGGCGGCAAAACGGTCAAAATGACCATTAAAGGTATGGAACAATGGCT
GCTGCGTCCGGAATACGCGCGTTTCGCACCCCTGCTTTACCGCGAACCGGTCAGGATATG
GGATTTGCCCCCGAACCTGCGCGGCTTGGCGGCATCCTGCCTGAAAGCCGTCCCAAAGGG
GCATTTGGGCGAAACATTGCGCCGCGAGGCGGACGTGTTGCGGCTGCTGTCGGACTTGTG
GGACACGGTTTCAGACGGCATCGGGCCGGCGGCGGGGCAAACGGCGGAAGCAGACGCTAT
GCCGTCTGAAGACTTCAGCCGCACCCTAAATGCCGCGTTTGCCGACGGCGCACACCAAGT
CAACCGGCTGACAGACGCGCTGAACATCAGTGAAAGGACGCTGCAACGCCGTATGCGCGA
CCATTTCGGCATTACGGCAAGCGAATGGCTGCACCACAAACAAATGCAGCACGCGCTCTA
TCTGTTGCAAAACGGGGGAAAAAGCATAGGCGAAACCGCATATTTATGCGGCTACCGCCA
CGTTTCCAGCTTTACTCAGGCATTCAGGCAATATTTCGGCAGCACGCCTGCGGAAACCAA
AAAAGAAAACCGGTAAGCCGCATTTGATTTCAAACCCGAAATCCGCGTGTATAGTGGATT
AACAAAAACCAGTACGGCGTTGCCTCGCCTTAGCTCAAAGAGAACGATTCTCTAAAGTGC
TCAAGCACCAAGTGAATCGGTTCCGTACTATTTGTACTGTCTGCGGCTTCGTCGCCTTGT
CCTGATTTTTGTTAATCCACTATAAAAAACTTGTACGGCAATTTATTTGGGAATAAACTA
AAACTACATCTAACTACAAAACTGGAGAACCCGAAATGAAACAATTGGCCATGTACATCA
ACGGACGCTTTGAAAACGATTTCAACGGCGAATGGCGCGACGTATTGAACCCGTCCACCG
AAGAGGCCATCGCCCGCGAACCCAAAGGCGGCAAGGCGGACGTTGACCGCGCCGTCGCGG
CGGCGCGTGCGGCGCAACCGGCTTGGGAGCGTCTGCCTGCGGTCGAACGCGGCGCGTATT
TGCGTAAAATCGCCCAAGGCATACGCGAACGTGCCGACGAGCTGACCGACACCATCGTTG
CCGAAGGCGGCAAAACCAAAGACTTGGCACGCGTGGAAGTCATGTTCACCGCCGACTATC
TCGATTATCAGGCCGAATGGGCGCGCCGCTACGAAGGCGAAATCATCCAAAGCGACCGCC
CGCGCGAAAATATTTTATTGTTCAAACGTCCGCTGGGCGTAATTGCCGGCATTTTGCCGT
GGAACTTCCCCTTCTTCCTGATTGCCCGCAAAATGGGCCCCGCTTTGGTAACGGGCAACA
CCATCGTCGTCAAACCCAGCAGCGTAACCCCGATCAACTGCCACATCTTCGCCGAAATCG
TCGATGCGGTCGGACTGCCCGCAGGCGTGTTCAACGTGGTGAACGGTCCCGGCGCGGAAA
TCGGCAATGCCTTGTCCGCCCATCCGCAAGTCGATATGGTCAGCCTGACCGGCTCCGTCG
AAGCAGGCCGCCAAGTGATGGAAGCCGCCTCCGCCAACATCACCAAAGTTTCGCTGGAAC
TCGGCGGCAAAGCGCCTGCCATCGTTTTGAAAGATGCGGATTTGGACTTGGCGGTGAAAT
CCATCTTGGCTTCGCGCGTCGGCAACACCGGTCAAATCTGCAACTGCGCCGAGCGCGTCT
ATGTCCACAGCAGTCTGAAAGACGCATTCATTGAAAAAATGACCGCCGCGATGAAAGGCG
TGCGCTACGGCAACCCTGCCGAAGCCGAAGCAGGCGCGCTGGAAATGGGCCCGCTGATTG
AAGAACGCGCCGTCAAAGCCGTTGCCGAAAAAGTGGAACGGGCAGTCAAACAAGGTGCGA
AATTGGTTTGCGGCGGCAAACGCGCCGAAGGACGCGGTTATTTCTTCGAGCCGACCCTGC
TGACCGACACCGACAACAGTATGGACATTATGAAAGAAGAAACCTTCGGCCCCGTGCTGC
CCGTTTCCGCTTTCGACACGCTCGACCAAGTCATCGCCTTGGCAAACGATTGCGAGTTTG
GTCTGACCAGTTCTGTTTATACGACTAATTTAAACGAAGCCTTCTACGTTACCCGCCGCC
TGCAATTCGGCGAAACCTACATCAACCGCGAAAACTTTGAAGCGATGCAGGGTTTCCACG
CCGGTTGGAAAAAATCCGGTATCGGCGGCGCGGACGGCAAACACGGTTTGGAAGAATATC
TGCAAACCCAAGTCGTTTATTTGGAAACCGACATTTAATGCCGCTTTAAAACCCCGATAG

# Appendix A

AAAATGCCGTCTGAACCCGTTTTCAGGTTCAGACGGCATTTTTATTGCTTCACCGGCAAT
CAGTCATGACCGAGGTCGATGTTTTTGTCTTTGTATAGTGGATTAACAAAAATCAGGACA
AGGCGGCGAAGCCGCAGACAGTACAAATAGTACGGAACCGATTCACTCGGTGCTTCAGCA
CCTTAGAGAATCGTTCTCTTCGAGCTAAGGCGAGGCAACGCTGTACTGGTTTTTGTTAAT
CCGCTATATTCCGCCATCTCTAAGATTTACAGCGATACACGGGTAATTTAAGGAATGCCC
AAACCGTCATTCCCGCCACTTTTCGTCATTCCCGCGAAAGCGGGAATCTAGAATCTCGGA
CTTTCAGATAATCTTTGAATATTGCTGTTGTTCTAAGGTCTAGATTCCCGCCTGCGCGGG
AATGACAAATCCATCCGCACGGAAACCTGCACCACGTCATTCCCACGAACCCACATCCCG
TCATTCCCACGGAAGTGGGAATCTAGAAATAAAAAGCAACAGGCATTTATCGGAAATAAC
TGAAACCGAACAGACCTAGATTCCCGCCTGCGCGGGAATGACGGCTGCAGATGCCCGACG
GTCTTTATAGCGGATTAACAAAAATCAGGACAAGGCGACGAAGCCGCAGACAGTACAGAT
AGTACGGAACCGATTCACTTGTTAAAGAATCGTTCTCTTTGAGCTAAGGCGAGGCAACGC
CGTACTGGTTTTTGTTCATCCACTATAACTAGGGAAATTCAAATTAAGTTAGAATTATCC
CTATGAGAAAAAGCCGTCTAAGCCGGTATAAACAAAATAAACTCATTGAGCTATTTGTCG
AAAGTTCAAATTTCCATTTTAAAACAATTAGTAAAATCGAGTTTATCCTAGTTGTCCAAG
ACAACCCCTATAATAATATAATTCAAAATATAAAAATGGGTTACATCTAAACATTACGGA
ATTTTTATTCCCTCGCCTGAATTCTATTGTCAGATTCAAGGAGACCTCATCATGCGAACG
ACCCCAACCTTCCCTACAAAAACTTTCAAACCGACTGCCATGGCGTTAGCTGTTGCAACA
ACACTTTCTGCCTGCTTAGGCGGCGGCGGAGGCGGCACTTCTGCGCCCGACTTCAATGCA
GGCGGTACCGGTATCGGCAGCAACAGCAGAGCAACAACAGCGAAATCAGCAGCAGTATCT
TACGCCGGTATCAAGAACGAAATGTGCAAAGACAGAAGCATGCTCTGTGCCGGTCGGGAT
GACGTTGCGGTTACAGACAGGGATGCCAAAATCAATGCCCCCCCCCCGAATCTGCATACC
GGAGACTTTCCAAACCCAAATGACGCATACAAGAATTTGATCAACCTCAAACCTGCAATT
GAAGCAGGCTATACAGGACGCGGGGTAGAGGTAGGTATCGTCGACACAGGCGAATCCGTC
GGCAGCATATCCTTTCCCGAACTGTATGGCAGAAAAGAACACGGCTATAACGAAAATTAC
AAAAACTATACGGCGTATATGCGGAAGGAAGCGCCTGAAGACGGAGGCGGTAAAGACATT
GAAGCTTCTTTCGACGATGAGGCCGTTATAGAGACTGAAGCAAAGCCGACGGATATCCGC
CACGTAAAAGAAATCGGACACATCGATTTGGTCTCCCATATTATTGGCGGGCGTTCCGTG
GACGGCAGACCTGCAGGCGGTATTGCGCCCGATGCGACGCTACACATAATGAATACGAAT
GATGAAACCAAGAACGAAATGATGGTTGCAGCCATCCGCAATGCATGGGTCAAGCTGGGC
GAACGTGGCGTGCGCATCGTCAATAACAGTTTTGGAACAACATCGAGGGCAGGCACTGCC
GACCTTTTCCAAATAGCCAATTCGGAGGAGCAGTACCGCCAAGCGTTGCTCGACTATTCC
GGCGGTGATAAAACAGACGAGGGTATCCGCCTGATGCAACAGAGCGATTACGGCAACCTG
TCCTACCACATCCGTAATAAAAACATGCTTTTCATCTTTTCGACAGGCAATGACGCACAA
GCTCAGCCCAACACATATGCCCTATTGCCATTTTATGAAAAAGACGCTCAAAAAGGCATT
ATCACAGTCGCAGGCGTAGACCGCAGTGGAGAAAAGTTCAAACGGGAAATGTATGGAGAA
CCGGGTACAGAACCGCTTGAGTATGGCTCCAACCATTGCGGAATTACTGCCATGTGGTGC
CTGTCGGCACCCTATGAAGCAAGCGTCCGTTTCACCCGTACAAACCCGATTCAAATTGCC
GGAACATCCTTTTCCGCACCCATCGTAACCGGCACGGCGGCTCTGCTGCTGCAGAAATAC
CCGTGGATGAGCAACGACAACCTGCGTACCACGTTGCTGACGACGGCTCAGGACATCGGT
GCAGTCGGCGTGGACAGCAAGTTCGGCTGGGGACTGCTGGATGCGGGTAAGGCCATGAAC
GGACCCGCGTCCTTTCCGTTCGGCGACTTTACCGCCGATACGAAAGGTACATCCGATATT
GCCTACTCCTTCCGTAACGACATTTCAGGCACGGGCGGCCTGATCAAAAAAGGCGGCAGC
CAACTGCAACTGCACGGCAACAACACCTATACGGGCAAAACCATTATCGAAGGCGGTTCG
CTGGTGTTGTACGGCAACAACAAATCGGATATGCGCGTCGAAACCAAAGGTGCGCTGATT
TATAACGGGGCGGCATCCGGCGGCAGCCTGAACAGCGACGGCATTGTCTATCTGGCAGAT
ACCGACCAATCCGGCGCAAACGAAACCGTACACATCAAAGGCAGTCTGCAGCTGGACGGC
AAAGGTACGCTGTACACACGTTTGGGCAAACTGCTGAAAGTGGACGGTACGGCGATTATC
GGCGGCAAGCTGTACATGTCGGCACGCGGCAAGGGGGCAGGCTATCTCAACAGTACCGGA
CGACGTGTTCCCTTCCTGAGTGCCGCCAAAATCGGGCAGGATTATTCTTTCTTCACAAAC
ATCGAAACCGACGGCGGCCTGCTGGCTTCCCTCGACAGCGTCGAAAAAACAGCGGGCAGT
GAAGGCGACACGCTGTCCTATTATGTCCGTCGCGGCAATGCGGCACGGACTGCTTCGGCA
GCGGCACATTCCGCGCCCGCCGGTCTGAAACACGCCGTAGAACAGGGCGGCAGCAATCTG
GAAAACCTGATGGTCGAACTGGATGCCTCCGAATCATCCGCAACACCCGAGACGGTTGAA
ACTGCGGCAGCCGACCGCACAGATATGCCGGGCATCCGCCCCTACGGCGCAACTTTCCGC
GCAGCGGCAGCCGTACAGCATGCGAATGCCGCCGACGGTGTACGCATCTTCAACAGTCTC
GCCGCTACCGTCTATGCCGACAGTACCGCCGCCCATGCCGATATGCAGGGACGCCGCCTG
AAAGCCGTATCGGACGGGTTGGACCACAACGGCACGGGTCTGCGCGTCATCGCGCAAACC
CAACAGGACGGTGGAACGTGGGAACAGGGCGGTGTTGAAGGCAAAATGCGCGGCAGTACC
CAAACCGTCGGCATTGCCGCGAAAACCGGCGAAAATACGACAGCAGCCGCCACACTGGGC
ATGGACGCAGCACATGGAGCGAAAACAGTGCAAATGCAAAAACCGACAGCATTAGTCTG
TTTGCAGGCATACGGCACGATGCGGGCGATATCGGCTATCTCAAAGGCCTGTTCTCCTAC
GGACGCTACAAAAACAGCATCAGCCGCAGCACCGGTGCGGACGAACATGCGGAAGGCAGC
GTCAACGGCACGCTGATGCAGCTGGGCGCACTGGGCGGTGTCAACGTTCCGTTTGCCGCA
ACGGGAGATTTGACGGTCGAAGGCGGTCTGCGCTACGACCTGCTCAAACAGGATGCATTC
GCCGAAAAAGGCAGTGCTTTGGGCTGGAGCGGCAACAGCCTCACTGAAGGCACGCTGGTC
GGACTCGCGGGTCTGAAGCTGTCGCAACCCTTGAGCGATAAAGCCGTCCTGTTTGCAACG
GCGGGCGTGGAACGCGACCTGAACGGACGCGACTACACGGTAACGGGCGGCTTTACCGGC
GCGACTGCAGCAACCGGCAAGACGGGGGCACGCAATATGCCGCACACCCGTCTGGTTGCC
GGCCTGGGCGCGGATGTCGAATTCGGCAACGGCTGGAACGGCTTGGCACGTTACAGCTAC
GCCGGTTCCAAACAGTACGGCAACCACAGCGGACGAGTCGGCGTAGGCTACCGGTTCTGA
CGGACAGGAAGCAGACAGCCGCAAAGATCACGGTCTTTGCGGCTGTTTCTTATGAAAAGA
AAACCCTATTCCAATTGCCTGCTTCTATTGTTTCAAGACTTCTTCCAAAGATTCGGCATT
AATCAGATGTATAGCGGATTAACAAAAATCAGGACAAGGCGGCGAAGCCGCGGACAGTAC
AAATAGTACGGAACCGATTCACTCGGTGCTTCAGCACCTTAGAGAATCGTTCTCTTTGAG

## Appendix A

CTAAGGCGAGGCAACGCCGTACTGGTTTTTGTTAATCCGCTATATTCCACCATCTCTAAG
ATTTACAGCGATACACGGGTGATTTAAGGAATGCCCGAACCGTCATTCCCGCCACTTTCC
GTCATTCCCGCGAAAGCGGGAATCTAGAATCTCGGACTTTCAGATAATCTTTGAATATTG
CTGTTGTTCTAAGGTCTAGATTCCCGCCTGCGCGGGAATGACGAATCCATCCGCACGGAA
ACCTGCACCACGTCATTCCCACGAACCCACATCCCGTCATTCCCACGAAAGTGGGAATCT
AGAAATGAAAAGCAACAGGCATTTATCGGAAATAACTGAAACCGAACAGACTAGATTCCC
GCCTGCGCGGGAATGACGGCTGCAGATGCCCGACGGTCTTTATAGCGGATTAACAAAAAT
CAGGACAAGGCGGCGAAGACGCAGACAGTACAGATAGTACGAAACCGATTCACTCGGTGC
TTCAGCACCTTAGAGAATCGTTCTCTTCGAGCTAAGGCGAGGCACCGCTGTACTGGTTTT
TGTTAATCCACTATACTTGGAGCTGGTCTTGCTTTTCGCCTAATTCTACGTTTTCAAACG
GTTGCAGCTGGTGGTCTGCCATAAAGGTCTCCTTATTGTATTTCAGGTTGGAAATCGGAA
TTTGTTTTCACAATTTTACACCTTCGCCCCCGCTTTCTCTACATAAAATTACATTTTGCC
GATATTTGCCGAATTGTCTGAAAATATGTGTAATAAGGGGCGTATAATCAAAACATTTGC
CCCGGATTGCCATGCCTTATTTCGCCCTGTTTGACGATGCCGTAAGCGGCCGCGCAAAAC
GCTATCAAAATCATGTGGAAAGCCGTTTTTTCCGTCCCGAAGAACTCGATGCTTTGGACG
GCGCGCTGCAATCGGGCTGGCAAAAAGGGCTGCATTCGGTGTTGTTTGCAGACTACGGAT
TCGGTTTGCCGCTGACGGGGGTTGAGTCCGAACGCGGCGGCAATCTTGCCCTGCACTGGT
TTGCCAACTGCGCCGACATCGATGCCGAAAGCTGGCTTGCCCGACACTCAGACGGCCTCC
CCGCCGGCATTTCCACGCCGCAACCCTCCGTATCCGAAACCGATTACCTCGACCGCATCC
GCCAAATCCACGAAGCCATCCGGCGCGGCGACACCTATCAAATCAACTACACCACCCGCC
TGCACCTGCAAGCCTACGGCAATCCCGTCAGCCTCTACCGCCGCCTGCGCCAGCCCGTCC
CCTATGCCGTCTTGTCCCACCTGCCCGATGCGGAGGGGCAATCCGCGTGGACGCTGTGTT
TCTCGCCCGAACTCTTCCTCAAAATCGGTTCGGACGGCACCATCAGCACCGAACCGATGA
AAGGCACCGCGCCGATTTTGGGCGACGGACAAGACGAACGCCGCGCCGCCGAGTTGCAAG
CAGACCCGAAAAACCGCGCCGAAAACGTGATGATTGTCGATTTGCTGCGTAACGATCTCG
GCAAAATCGCCCAAACCGGCACAGTATGCGTACCCGAACCGTTTAAAGTATCGCGTTTCG
GCAGCGTTTGGCAGATGACCAGCACCATCCAAGCCCAAGCCTTGCCGCACACCTCGTTCG
CCGACATCCTCCGCGCCGCCTTCCCCTGCGGCAGCATCACCGGCGCGCCCAAAAAAATGA
GTATGCAGATTATCGAATCGCTCGAAGCCGAAGCGCGCGGACTTTATACGGGCAGCATCG
GCTATTTGAACCCGTGTTCCGGCGGCTTGGGGTTTGAAGGCACGTTCAACGTCGTTATCC
GCACCTTGTCGCTCACGCCGCTTTCAGACGGCATTTATCAAGGCGTGTACGGTGTCGGTT
CCGGCATCGTCATCGACAGCGACCCCGCCGCCGAATATCGCGAATGCGGCTGGAAAGCCC
GTTTCCTCAACGAATTGCGCCCCGACTTCGGCATTTTTGAAACCCTGCGCGCGGAAAACG
GACGCTGCACCCTGCTCGACCGCCACCTATGCCGTCTGAAAACCTCCGCCCAAGCCCTCA
ACCTGCCCCTGCCCGACGGCTGCGAAAATCAAATCAAACAATACATTGCCGACTTGCCCG
ATGGCGCGTTCCGCGTCAAAGCCCTGCTCGCTTCAGACGGCATCAGCCTGTCCCGCGCCG
TTTTAAACCGTCTGACCGACAAACAACGCGTCATCATTTCGCCCGCCGTCCTGCCCGCAC
AAAACTACCTGCGCCGCTTCAAAACCACCTGCCGCGCCCTCTTCGACCAAGCGTGGCAAA
CCGCCGAAACACAAGGCGCGTTCGACAGCCTGTTTTTCAATTCAGACGGCATCCTGCTCG
AAGGCGGCAGAAGCAACGTCTTCATCAAACATCGCGGACAATGGCTCACACCCTCTTTAG
ATTTAGACATTTTAAACGGCATAATGCGCCAAGCCGTATTGGACGAACCGCAAAAATATT
TGCAAACAAATCAAGTAATCGAAACACACATCACACAAAAAACACTGCAAGAAGCCGAAG
AAATCCGCCTCTCCAACGCCTTGCGCGGCGTATTTGCCGCCGCCCTTGCCTGAACGCGCA
AAAATGCCGTCCGAACCTGTTTCCAAAGTTCGGACGGCATTATCCCACCATTCAAAACCG
CCAATCCGCCGACACAAACACCTCGCTGTTGCGGCGTTTCGCATACGGCACATTACTTTC
CGTCCTGCCGAAACGATAATTCAACGCCGGCACGATACCTTTGTACGACAACTTGTCGTG
GCTCAAAGCCAGCGAGACATTCCATTCGCGGTTGCGTTGCGCCTCTGTCGAGAAAGCCGC
AATGCCCTTATAGTTGCGGCGGGCATAAGACGCGGAAACCCGACTGTTCAAACCGCCCAA
CTGCCGCCACTCCTGCGCCCAACCGGCATAAACACCGTTGCGCCGGTAGGCGGCATTATT
GACCGCGCCGCCCACCGTTTCGCGTTTCGGCACAAACCGCACAAACTGCCAGCCGCCGAA
CACAGTTGCCGATTCGCCCAAACGTTTTGCCGACGAAACATAAAACCCGTCCTGCCTGCC
GTTATTGTATTCCGCCCTATCCTGTTCGCGGTAGCGTTGGCGGTAATGTTCCAGCGCGAC
CGAAAATTGCCATCCCGGGTTTGGGCGGTAAGTATGGGACAGCTGCACGCCGACTCCGTG
CGCCAGCATATACGGCGGCAGGCGGCGGTTGTTTACCCGTTTTGTTTTCGCATCAAAGCC
GTCGCTGCCCGACAACTGCACCTGATAAAACGGCAAAATCCCCGCCGTCTGCCGTGCATT
TTTATACTGCCAACCCAAATACGCCCTGCCGAACCCGTCATCATAAGCTGATTTTTTACT
GAAATAATAGCTCGTGCCGCCGATATTGGAACGGAACAACAAATAATGATTATCTGCCAA
CGGCGTCAGCTTTTCCGCCTCGATTTCATAATTCAACCCTGCCGCCCGCTCCGCCCGGCT
GACACTGCATATCTGCCGGCCTCCGTTTTGCCGGCAATATTGCGGCGCGGCATTATTGGC
ATTTCTATTGACCGCCGGACTGATGCCGCCCGAAAAACGCCAGCCCGTCAGCCCCTCCGT
TTTTTTCCGAAAACGCCCCACATTTTCCAAAACCGGTGCCGGCAAATCCAATTTTGCCGC
CTCCGCAAAATGCCTTTCTGCCGACTTCAGCCGGAAATCGTCAAACTCCGCCGCCGCCAA
ATCCAGCAAAATCCGCTCGTCTGCCGCATTTTCCCCGTGCAGTTCCCGATACCGCGCCAC
CGCCTCCGCCGGCCTTCCCGCCAATTTCGCCAGCAAAGCCCGCGCCCTGCCGTACAAAAC
CGCGTCATAATCCGGCAGCTTGGCATACAAATCCGCCAACGAAGCGATTAAATCCGCCTG
ATTGCCGTTGAGCGCGTCGCGCAAACTATGTTCCAACATTTTCGGATGCGCCAACAAAAA
ATCCCCGTCAACCACGCGCGGGGCATCATTTTCAACTTTCCAATCTGATTCCGCCCACTT
ATCCGACACCGACCGCTGCACCTGCAACAATGCCTTGTCATCCAAAATCGCGGGCGCATC
CGCCCCATAGGCGGCAGAAACACCTGCCGCACACCAAACAACCAAAAAGCCGTATCTGAA
ATACAACATACCCTGTCATTTACCTTTCTGGCAAACACGCCGCCGAAGCACGTCAAACCA
TCCGAAAAACAGGCAGAAACCCGTGAAAACCGGCTTTGCCGCCTGAAAGCAGGCAAACAA
AAACCGCCGCCCGATTTTCAAAGGGCGGATTTCACATTTATAGTGGATTAACAAAAATCA
GGACAAGGCGACGAAGCCGCAGACAGTACAAATAGTACGGAACCGATTCACTCGTGCTTA
AGCACCTTAGAGAATCGTTCTCTTTGAGCTGAGGCGAGGCAACGCCGTACTGGTTTTTGT
TAATCCACTATAACAGCAACCCTGTCGCCGTCATTCCCGCAAAAGCGGGAATGACGAAGC

## Appendix A

TATCCGCACAGAAACCTGCACCACGTCATTCCCACGAAAGTGGGAATCCAGAACGTAAAA
TCTGAAGAAACCGTTTTATCCGATACGTTTCCGCACCGACAGACCTAGATTCCCGCTTTC
GCGGGAATGACGGCGGAAAGGTTGCTGTTTTTCCGATAAATTCCTGCCGCTCTTCGTTTT
TGGGATGGCGGGAAATAAAACAAAAGCGCGCGTATCAAAAAACAAAAATGCAAAGAACGG
CGTTGGCAATTTTAAGGTTTGCGCCTTAGCCGCACTTATTCGCAAAAAAAACCGCACGGC
GTTGACCGTGCGGTTTTTTATCTGAAAGCTTCAGACGGCATTGCTTACATCATGCCGCCC
ATACCACCCATGCCGCCCATATCAGGCACAGCCGGTTTGTCTTCGGGGATTTCAGCGATC
ATGCAATCAGTGGTCAGCATCAAGCCGGCGATAGATGCGGCGTGTTGCAGCGCAGAACGG
GTTACTTTGGCGGGGTCGAGTACGCCCATTTCGATCATATCGCCGTATTCGCCGCTGCCA
GCGTTGTAACCGTAGTTGCCTTTGCCTTCCAATACTTTGTTCACAACCACGCTGGGTTCG
CCGCCTGCGTTGGCAACGATTTGGCGCAGCGGAGACTCAACGGCGCGCAAGACGATTTGT
ACGCCTGCGTCTTGGTCGGCATTGCCGGTGTGCAGGTTTTCCAAAGCAGCACGGGCACGC
AACAGGGCTACGCCGCCGCCTGCAACCACGCCTTCTTCAACGGCTGCGCGGGTAGCGTGC
AGCGCGTCTTCCACGCGGTCTTTTTTCTCTTTCATTTCGACTTCGGTCGCGGCACCGACT
TTGATGACTGCCACGCCGCCTGCCAATTTAGCCACGCGCTCTTGCAGTTTTTCTTTGTCG
TAATCGCTGGTTGCGGTTTCGATTTGTTGGCGGATTTCGGCAACACGCGCTTCGATTTGG
GCTGCGTCGCCAAAGCCGTCGATGATGGTGGTGTTTTCTTTACCGATTTCGATGCGTTTG
GCTTGACCCAAGTCGTCCAAAGTCGCTTTTTCCAAAGACAGACCGACTTCTTCGGAAATC
ACCACGCCGCCGGTCAGGATGGCGATGTCTTGCAACATCGCTTTGCGGCGGTCGCCGAAG
CCAGGGGCTTTGACGGCAACGGTTTTCAGGATGCCTCGGATGTTGTTCACGACCAAAGTC
GCCAAGGCTTCGCCTTCTACGTCTTCAGCGATAATCAACAGCGGACGGCTGGCTTTTGCC
ACTTGTTCCAAAACAGGCAGCAGGTCGCGGATGTTGCTGATTTTTTTGTCGAACAACAAT
ACAAACGGATTGTCCAAAGCAGCGATTTGTTTTTCCGCATCGTTGATGAAGTAAGGAGAC
AGGTAGCCGCGGTCGAACTGCATACCTTCAACTACGTCCAGCTCGTTTTCCAAAGACTTG
CCGTCTTCAACGGTAATCACGCCTTCTTTGCCGACTTTTTCCATCGCTTCGGCGATAATC
GCGCCGACTTGTTCGTCGGAGTTGGCGGAAATAGAGCCGACTTGGGCGATTTCTTTAGAA
GTGTCGCAAGGTTTGGCGATGTTTTTCAGTTCGTCAACCAAAGCGGCGACGGCTTTATCG
ATACCGCGTTTCAGGTCGGTCGGATTCATACCTGCGGTAACATATTTCATACCTTCGGCA
ACGATGGATTGCGCCAGTACGGTGGCGGTAGTCGTACCGTCGCCTGCCACGTCGTTGGTT
TTGGACGCAACTTCTTTCACCATTTGCGCGCCCATATTTTCAAACTTGTCTTTCAGTTCG
ATTTCTTTGGCGACGGTTACGCCGTCTTTGGTGATGTGCGGGCCGCCGAATGCGCGGTCA
ACGACTACGTTGCGACCTTTGGGGCCCAAGGTTACGCGGACGGCGTTTGCCAGAATGTTC
ACGCCGTTTACCATTTTTTGACGGACTTCATTGCCGAACTGTACGTCTTTTGCTGCCATT
TCAATTCTCCAAAAATCATTAAAACTGTCTGATAAAACCGTTTATGCCGTCTGAAGGCGG
TTTGCCGTTTCAGACGGCATCGTGTCCGTATTTATTTTTCAACGATGCCGAAAATATCTT
CTTCGCGCATTACCAACAGCTCTTCGCCGTCGGCTTTTACGGTTTGGCCGCTGTATTTGC
CGAAGATGATTTTGTCGCCGACTTTGACATCCAGCGGACGGCGGCTGCCGTCTTTACCGA
TTTTGCCCGCGCCCACGGCGATGACTTCGCCCATATCGGGTTTTTCGGCGGCCGCACCCG
GCAAAACGATGCCCGATGCGGTTTTTTCTTCAGCTTCCAAGCGTTTGACGACAACGCGGT
CGTGTAAAGGACGGATGGTCATATTTATGCTCCGATAAAATAGTTTGAAAACAATCATCT
GCCCGAACGGTTCAGGCAGATTGAAGTGGAAACCGGACAGCCGTCAAGCAGCTGCCCGTA
TAAGTCGGCAAAATTAGGGTGTGTGCGGGTAAATTCAAGTGAGGCGGAAAAAATTTATTT
CCGCCGTTTTTTATAGTGGACTAAATTTAAGGGGCTGTACTAGATTAGCAGATATGTTAC
CCTCGAAATATGAAGATAACGCACTGCAAATTAAAGAAAAAAGTACAGAAAGAACTGCTC
CGTTTTTTGTACTGGAAGTTACCGCCCGTTCTGCCGCCGATATTTTGGGTATCCATCCCA
ATTCGGCAGCACTGTTCTACCGTAAAATCCGCACGGTTATCAACCATCATTTAGCCTTGG
CTGCCGATGAGGTTTTTGAGGGCCCTGTCGAGCCGGACGAAAGCGATTTCGGCGGACGGC
GTAAAGGTAGACGTGGTCGCGGTGCAGCAGGAAAAGTGGTTGTCTTCGGCATTCTGAAAC
GCAACGGACGGGTCTATACCGTTGTGGTGGATAATGCCAAGTCTGAAACGTTACTCCCTG
~~TCATCAAGAAGAAAAATCATGCCGGACAGTATTGTTTATACCGATAGTCTGAGCAGCTGCG~~
ACAAGTTGGACGTGAGCGGTTTCATTTATTACCGCATCAACCATTCCAAGGAATTTGCAG
ACCGTCAGAACCACATTAACGGCATTGAGAATTTTTGGAATCAGGCAAAACGTGTCTTGC
GAAAATACAATGGAATCGATCGTAAATCTTTCCCGCTGTTCTTGAAAGAATGCGAATTTC
GATTTAACTTCGGCACACCGTCTCAACAGCTTAAAATCCTGCGGGATTGGTGTGGGATTT
AGGGCTAATCTAGTACAGCCCCTAAAATTTTTCGTTTTCAAGCCTTCACCGCTTGCCATC
AGCGTTAAATTTTTTTACGATAAGCACATAGATTGTAAACAATCGGCCACAAGCCGGTTT
GTTTTTTCAGAAGACATTATCCCTGTCAGACGCTATTTCTATATATTTCGCCTATAATGG
CTTGTTTTTAATAAATAATTCAAGAGGTATCAACGTGTCTGATTCCAAGACGAAAGAACG
CGCCACATTCGGCACGCGCCGCGCGTTTATGATTGCCGCCATCGGGTCCGCCGTCGGCTT
GGGCAATATTTGGCGTTTCCCCTATATTGCTTTTGAAAACGGCGGCGGCGCGTTCATCCT
GCCCTATCTGGTCGCGCTTCTGACGGCGGGCATCCCGCTGCTGCTGCTCGATTATGCCAT
CGGCCACCGTTACCGTGGTTCTGCGCCCTTGGCTTTCCGCCGCCTCGGACGATGGTTTGA
GCCGGTCGGCTGGTGGAACGTGATGACCAATATCGTCATCTGCATCTATTACGCGGTAAT
TATCGGTTGGGCGGCAAGCTATACCTATTATTCGGTCAACGCCGCCTGGGGTGCGGATCC
GCAGGGTTTTTTCTTTAAGGACTTCCTGCAAATGGCGGGCCCGGAAGCCTTGGGTTTGGA
TTTTGTCGGCAAAGTCGCCGGTCCTTTGGCGGGCGTGTGGGTTTTTACCGCCGCCATTAT
GGCGTTGGGCGTGCAAAAGGGCGTGGCGCGCGCCTCGTCGTTCTTTATGCCGCTGCTTTT
GGTGATGTTTTTGATTATGGTCGGCATTTCACTAACCCTGCCGGGTGCGGCAAAGGGCTT
GGACGCATTGTTTACGCCCGACTGGTCGAAACTCGCCGATTCCAAGGTCTGGGTGGCGGC
ATACGGGCAGATTTTCTTTTCGCTTTCCATCTGCTTCGGCATTATGGTTACCTATTCTTC
TTATTTGAAGAAAAAAACCGACTTGGGCGGAACGGGGCTGGTGGTCGGTTTTGCCAACAG
CAGCTTTGAACTGCTCGCGGGCATCGGCGTGTTTGCCGCATTGGGCTTTATGGCGCAGGC
GGGCGGTAAGGCGGTCAACGAGGTTGCCTCAGGCGGCATCGGTTTGGCGTTTATCGCCTT
TCCGACCATTATCAACCAGGCACCGATGGGCTGGCTGATCGGCATATTGTTTTTCGGTTC
GCTGGTGTTCGCCGGCGTTACGTCGATGATTTCCATCCTTGAAGTGATTGTGGCGGCGAT

## Appendix A

TCAGGACAAGCTGAACATCGGGCGCGTCAACGCCACGCTGCTGGTCTGCATTCCGATGGG
CATTGTTTCCACGCTGCTGTTCGGTACGGCGACGGGGCTGCCGGTTTTGGACGTGATGGA
CAAATTCGTCAACACCTACGGCATTGTTGCCGCCGGCTTTGTTTATGTTGCCGCCATCAT
CATCAGCGGCAGGCTGCCGGAATTACGCAAGCACCTGAACGCTTTGTCCTCCATCCGCAT
CGGCGGCTTGTGGACGGTCTGCGTCGTGGTTACCGTCGTGATGCTCGGCTATATGCTGTT
TAAAGATACCAGCGGCCTGATGGAGAAAAATTACGAAGGTTATCCGGATGGTTTCCTCAG
TATTTTCGGCTGGGGGATGTCGGCGGCGTTGGTCGTGTTCGGGCTGCTGCTGTCGTTGCT
GCCTTGGAAACACGGTCAGGATTTCAACGTCAAAGACGAACACGAACATGAACAAGGAGA
AGAAAAATGAGTACTTCCGCCATTGTGATGATGATTGTCTCAATCGTGATAATCTGGGGA
GGGCTGCTGCTTTCCCTGTTAAGGCTGCCGAACGAGTAAGCCTTTAGAGCGTTAAAAATG
CCGTCTGAACCGCTTCAGACGGCATTTTGATGTTCGGCTTGCAGGCAGGCGAGTTCGTTT
GCCATTTGCTGTTCCAAGGTTTCGCGCCGGCGGATGAGTCGGTATTCGTTGCCGTCCACC
AACACCTCTGCCGCACGGTTGCGCGCGTTGTAATTGCTCGCCATACTGGCCCCGTATGCG
CCCGCGCTGCGGATAAGCAGCAAATCCCCTTCTTCGCAGGCGATGGTGCGGTCTTTGCCG
AGGAAGTCGCCGGTTTCGCAAATCGGACCGACGATGTTGGCGGTCAGCGTCGCGATGTCT
TTGGTTTCGACCGCCTCGATGTGATGATAGGCATCATAAAGCGCCGGGCGCATCAAATCG
TTCATCGCCGCATCGACCATCACAAAGTTTTTCTCTTCGCCGTATTTGACAAACTCGACG
CGTGTCAGCAGCGAACCTGCGTTGCCGACCAGGCTGCGGCCGGGCTCAAGAATGAGTTTC
AGACGGCGTGTGCCGATCAGTTTTTGAACGGCTTGGGCATACGCGCCCAAATCAGGCACA
TTTTCGTCTTGGTAAACAATGCCGACGCCGCCGCCTAAGTCTAAATGTTCCAAAACAATG
CCTTCGGCGGCAAGCGCGTCAACCAAAATCAAAATGCGCTCGCAGGCTTCGACCAGCGGG
CTTAAGTCGGTCAGTTGCGAACCGATGTGGCAGTCGATGCCGATGATTTTCAAATTGGGC
TGTTGTGCGGCATAGTGGTAGGCTTCGAGCGCGTCGGCGTAGGCGATGCCGAATTTGTTG
GCTTTCAGACCTGTGGAGATGTAGGGATGGGTTTTTGCATCGACATCGGGGTTGATGCGC
AGGGAGACGGGCGCGGTTTTACCCAAACGTGCGGCAACTTTCTGAATACGGTCGATTTCG
GGGATGCTTTCCATATTGAAGCATTTCACGCCTGCATTCAGCGCGAACTCGATTTCCGCC
TCGCTTTTGCCTACGCCTGAAAATATGGTTTTTGCCGCGTCGCCGCCTGCCGCCAAAACG
CGTGCCAATTCGCCGCCGGACACAATGTCAAAACCGCTGCCCAGCGAGGCGAAGTGTTTG
ATAATGCTCAGATTGCCGTTTGCCTTGACGGCGTAACAGACGAGCGGGTTCAAAGCGGCA
AACGCGGTTTGGTAGTGTTCAAATGCTTCGGTCAGCGCGGATTGGCTGTACACATAAAGC
GGTGTGCCGAATGCTTCAGCAAGGCGGGGGTAGGGGACTTGTTCGCAAAATAGGGTCATG
TTTTCGTTTTCATTTTTGGGTTTGTGGAGCGGATTGCGGTTTGCTTTGAAGTTGCAAACC
GGTTTGGATTACGCCGAAACGCGCCTTGTCGCCTTCTTTGGGCAGGTAGAGGTCGCCTTT
GTAACCGCAGGCCGAGAGCAGGAGGGCGGTTGCCGCCGCAAAAAATACGCCGTATTTCAT
CGGTAAACTTCCTTCATAAGCGCGAATGTGGCAAGATTCGGCATCTTAAACAAAAAACAC
GCAAAAAGCTATGATGACCGAAAGCGAGTTTATCCGCGCGAGCGAAGCATTATTTGAACA
CATCGAAGACCAAATCGACGAAAACGGCTGGGATTTCGACTGCCGGTTTGCCGGAAACGT
CCTGACCATCGAAGCCGGAGACGGCGCCCAAATCATCGTCAACCGCCACACGCCCAATCA
GGAATTGTGGATTGCCGCAAAAAGCGGCGGCTACCATTTCGCCGAGCAAAACGGCAAATG
GCTGGCAACGCGCGACGGACGCGATTTTTATGACGTTTTAAACGAAGCCCTGAGCGCGGC
TTCGGGCGAAGCGGTGGAAATTGCGGAATTGTGATTTGGGTGTTATCACGGAAAGAAAAA
ATGAACACACGTCCCTTTTATTTCGGACTGATATTTATCGCGATTATCGCTATACTTGCT
AACTATTTAGGAAACACTGATTTTTCCCATCATTATCATATCAGTGCTTTAATTATTGCT
ATCTTGCTGGGAATGGCAATCGGCAATACCATTTATCCGCAATTTTCGACACAAGTGGAA
AAAGGCGTTTTGTTTGCCAAAGGCGCGCTTCTTCGCACTGGCATTGTGTTGTATGGTTTT
CGCCTCACTTTTGGCGATATTGCCGATGTAGGATTAAATGCGGTTGTCACTGATGCAATC
ATGCTAATTTCAACCTTCTTTTTTACCGCACTTTTAGGCATTCGTTATCTAAAAATGGAT
AAACAATTGGTTTATCTCACTGGGGCAGGTTGCAGCATTTGCGGTGCGGCAGCAGTGATG
GCGGCAGAGCCTGTTACTAAAGCAGAATCCCATAAAGTTTCAGTGGCGATTGCCGTAGTG
GTCATTTTCGGGACGCTTGCTATTTTTACTTACCCCTTGTTCTACACGTGGTCACAACAT
TTAATTAACGCCCATCAATTCGGTATTTATGTTGGTTCTAGTGTACACGAAGTGGCTCAA
GTGTATGCGATTGGGGAAAATATTGATCCTATCGTGGCGAATACTGCCGTCATTTCCAAA
ATGATCCGAGTGATGATGCTCGCCCCCTTTTTATTAATGCTTTCTTGGTTATTAACACGT
AGTAATGGAGTATCAGAAAATACATCACACAAAATTACAATTCCTTGGTTTGCTGTACTT
TTTATTGGTGTTGCCATTTTTAATTCTTTTGATTTATTACCAAAAGAACTCGTGAAATTA
TTCGTTGAAATCGATTCTTTCTTATTAATTTCATCAATGGCTGCGCTTGGCTTAACGACG
CAAGCAAGCGCAATCAAAAAGGCAGGATTAAAACCGTTTGTTTTAGGAATACTAACTTAT
TTATGGCTAGTGGTTGGTGGATTTTTAGTGAACTATGGAATATCAAAATTAATATAAAAT
TCACTAAAGAGAGCGTTACCCAATGGCACAATTACCGCTATATCTGACTTCTGAAATCAA
AGACTTTACTGTCGGCACGCCTAAAGTTTTAGAATCATTTTCCAAACATATCCCTTATGG
TGTCGTCTTTGAAGACGACGGCGACACAGGCTACTTCTATGCCGCTTCGCAAGACGGGAT
TTTAGATGCCTTGCACATCTATAATGTCGAAGATGTATCCGACAAACATATCCCCAATCA
TGTCTTGATTTTATGGGATGATGCCTGCACCATAGCCGCATTGTGTATCAACGACTACAT
TCATGCCGTCTATGATTTTGTCGAACAGGCAGGATATTGCCGCAACGGCTTCCCTGAAGC
AGGCGGCGAATGGGTGAAAGTCGAAAACCGCGTCTTGGATGATGAATTGCTGGACAAAAT
CCTATCCCGAAAATCTACATAACCCTCACAAAAGGATACCCAAATGCCCCTACTAGACAG
TTTCAAAGTCGATCACACCCGTATGCATGCCCCCGCCGTACGCGTGGCGAAAACCATGAC
TACGCCCAAAGGCGACACCATTACCGTGTTTGACCTGCGCTTTTGCGTTCCCAACAAAGA
AATCCTGCCTGGAAAAGGCATACACACGCTGGAGCATTTGTTCGCAGGTTTTATGCGCGA
CCACTTGAACGGCAACGGCGTGGAAATCATCGACATTTCCCCGATGGGCTGCCGCACCGG
TTTTTATATGAGTCTTATCGGCACGCCTTCCGAACAGCAGGTCGCCGATGCGTGGCTGGC
TTCGATGCAGGATGTTTTGAATGTCAAAGACCAAAGCAAAATCCCCGAGTTGAACGAATA
CCAATGCGGCACTTATCAAATGCACTCGCTCGCCGAAGCGCAGCAAATCGCGCAAAACGT
GTTGGCGCGCAAAGTGGCGGTGAACAAAAATGAAGAGCTGACGCTGGATGAAGGGCTGCT
GAACGCCTAATCCGCCAAAAATGCCGTCTGAACAAGGGTTTCAGACGGCATTTGCCTTTT

## Appendix A

CCGTTATAATCCGGGGTTGTCCGGGGGCGGGTTTTAAGCCGGCATCGTCCTTCCCTATTT
TTTTCTGTCCCTTATCGGTTTTAAGCGGGTTTTTTATGTCCAACAGACCTACACTCCTCC
TCGTTGACGGATCGTCCTACCTCTACCGTGCGTATCACGCGATGGGGCAAAACCTGACCG
CCCCCGACGGCGCGCCGACGGGTGCGCTGTATGGTGTATTGAATATGTTGCGCCGTTTGC
GGTCGGAATATCCGCACGATTATTGCGCGGTGGTTTTTGATGCGAAAGGCAAAAATTTCC
GCCATCAAATGTTTGAAGAATACAAGGCGACGCGCCCGCCGATGCCCGACGATTTGCGCC
CGCAGGCGGAAGCACTGCCGGATTTAGTGCGCCTGACAGGCTGGCCGGTATTGGTGATTG
GGCAGGTGGAGGCGGACGATGTGATCGGCACGCTGGCGAAACAGGGGGCGGAACATGGTT
TGCGAGTCATTGTTTCGACCGGCGATAAGGACATGGCGCAGTTGGTGGATGAGCGCGTTA
CGCTGGTGAACACGATGAGCAGCGAAACGCTGGACATTGAAGGCGTGAAGGCAAAATTCG
GCGTGCGCCCCGACCAAATCCGCGATTATCTCGCGCTGATGGGCGACAAGGTGGACAACG
TGCCGGGCGTGGAAAAAATGCGGCCCGAAAACGGCGGTGAAATGGCTGGAAGCCTACGGTT
CGCTGGCTGGTGTGATGGAACACGCTTCGGAAATCAAGGGCAAAGTGGGCGAAAACCTGC
AAGCCGCGCTGCCCCAACTGCCGCTGTCGTATGATTTGGTCACGATTAAAACCGATGTGG
ACTTGCACGCCGAGCTTTCAGACGGCATCGAAAGCCTGCGCCGTACTACGCCGAAATGGG
CGCAGCTGGTTGTCGATTTCAAACGCTGGGGCTTCCGCACCTGGCTGAAAGAAGCGGAAT
CAAACATGAATACCGGCTCGACCGATGATTTGTTCGGCAGCGACAGCATCGGCGAGCAGG
CGGCTTTGAATGCGGAAATGCCGTTTGAAAAACAAGCCGAAAAAGCCACCGCCCCCGAAA
AACTGGATTATCAAGCCGTTACCACCGAAGCGCAGTTTGCCGCTTTGTTGGACAAACTGT
CGCGGGCGGACACAATCGGCATCGATACGGAAACCACGTCATTAGACGCGATGAACGCCT
CGCTGGTCGGCATCAGCATCGCTTTCCAAGCAGGCGAAGCGGTTTACATCCCCGTAGGAC
ACAGCCTGACCGCCGCGCCTGAACAGCTTGATTTACAAGACGTATTAGGCCGTCTGAAAC
CGCATTTGGGAAACCCCGCCCTAAAAAAAATCGGGCAAAACCTCAAATACGACCAACACG
TTTTCGCCAACTACGGCATCGCCCTGAACGGCATTGCCGGCGACGCCATGCTCGCTTCCT
ACATCATCGAGAGCCATCTCGGACACGGCTTGGACGAATTGTCCGAACGCTGGCTCGGCT
TGGAAACCATTACCTACGAATCGCTGTGCGGCAAAGGCGCGAAGCAAATCGGTTTTGCCG
ATGTCGCCATCGGGCAGGCGACCGAATACGCCGCCCAAGACGCCGATTTCGCCCTGCGCC
TCGAAGCGCACCTGCGCGCGCAAATGGACGAAAAACAGCTTGAAATGTATGAAAAAATGG
AGCTGCCCGTCGCGCAGGTATTGTTTGAAATGGAACGCAACGGCGTGCAAATCGACCGCG
CCGAACTCGCCCGCCAAAGCGCGGAACTCGGCGCCGAGCTGATGAAGCTCGAACAGGAAG
CCTATGCCGCCGCAGGCCAGCCGTTCAACCTCAATTCGCCCAAACAGCTGCAAGAAATCC
TGTTCGACAAAATGGGCATCCCCACCAAAGGCCTGAAAAAAACCGCCAAAGGCGGCATTT
CCACCAACGAAGCCGTGCTCGAACAGCTCGCGCCCGACTACCCCCTGCCTAAAATCATCC
TGCAAAACCGCAGCCTGGCGAAGCTCAAATCCACCTACACCGACAAACTACCCGAAATGA
TTTCCCCCAAGGACGGCCGCGTGCATACCACCTACGCCCAAGCCGTCGCCATTACCGGCC
GCCTCGCCAGCAACAACCCCAACCTGCAAAATATCCCCATCCGTACCGAAGAAGGGCGTA
AAGTCCGCCGCGCCTTTACCGCACCGCAAGGCAGCGTCATCGTTTCCGCCGACTATTCCC
AAATCGAGCTGCGCATTATGGCGCACCTCTCCGGCGACAAAACCCTGATTGCCGCGTTCC
AAAACGGCGAAGACGTACACCGCCGCACCGCCGCCGAAGTGTTCGGCACTGCGCCCGAAA
ACGTCTCGTCCGAGCAACGCCGCTATGCCAAAAGCATCAACTTCGGCTTAATTTACGGTA
TGGGGCAATACGGTTTGGCAAAATCATTGGGCATCGACAACCTTTCCGCCAAAAACTTTA
TCGACCGCTACTTCGCCCGCTACCCCGGCGTCGCCGAATACATGCAGCGCACCAAAGAAC
AAGCCGCCGCCCAAGGCTACGTCGAAACCCTGTTCGGCAGAAGGCTCTACCTGCCCGACA
TCCGCAACAAAAACGCCAACGCCCGCGCCGGAGCCGAACGCGCTGCCATCAACGCCCCCA
TGCAGGGCACCGCCTCCGACCTCATCAAACGCGCCATGATAGACGTGTCCCGCTGGCTTT
CAGAGTGCGAAGCCTCCCCGTGGGACGAACTCTTACAAAGCAAACTGATTATGCAGGTGC
ATGACGAACTGGTGCTGGAAGTCGTTGAAACCGAACTGGATTTTGTCAAAGAAAAACTGC
CGCAGATTATGGCGAAAGTGGACGGCGGATTATTGGATGTACCGCTGGTGGCTGAGGTTG
GCGTAGGGGAGAATTGGGAAGAGGCACATTGATTGAAAGGTGTTATATGCTATCTTTATT
~~TAAATAAAATTTTAATTTTTTGGTATATTTTTTTCTAAATGTTCCTATAGTATAGTGGATTAA~~
CAAAAATCAGGACAAGGCGACGAAGCCGCAGACAGTACAAATAGTACGGAACCGATTCAC
TTGGTGCTTCAGCACCTTAGAGAATCGTTCTCTTTGAGCTAAGGCGAGACAACGCTGTAC
TGGTTTTTGTTAATCCGCTATATTCCGCCATCTCTAAGATTTACAGCGATACACGGGTGA
TTTAAGGAATGCCCGAACCGTCATTCCCGCAACTTTTCGTCATTCCCACGAAAGTGGGAA
TCTAGAAATAAAAAGCAGCAGGAATTTATCGGAAATAACTGAAACCGAACAGACTAGATT
CCCACCTGCGTGGGAATGACAATTCGAGACCTTTGCAATAACATAGGTTACTAAAATTTT
ATGCTCAATCTCATTTTCAAAATGCAAAACTTTTCTGATTTTTCCTACTTTTTGCTCAAT
ATTAGGAAGGTTTTAGGCAATTGAAAATTTTTTGGCGCATTTTTATGCGTCAAATTTCGT
TAACAGACTATTTTTGCAAAGGTCTCAATTCATAAGTTTCCCGAAATTCCAACATAACCG
AAACCTGACAATAACCGTAGCAACTGAACCGTCATTCCCGCGCAGGCGGGAATCTAGACC
TTAGAACAACAGCAATATTCAAAGATTATCTGAAAGTCCGAGATTCTAGATTCCCGCTTT
CGCGGGAATGACGAAAAGCAAGCCGTAGGTCGGATACTTGTATCCGACAAAAGCCTGCCA
TCTCAAATAGCCGTCGGATTCGAGAATCCGACCTGCCAAACCGGGCGCGGACGCTCCGGC
CGGCAGTTAGTACGCAAATCGAACAGAACATCACAAAAAAGCCCGATTCGGATTTTCCAA
TCGGGCTTTTTTGCGCCCGTTTTGTCATCCCGTGAAATATCCGCATGACAAAAATATAGT
GAATTAACAAAAATCAGGACAAGGCGACGAAGCCGCAGACAGTACAGATAGTACGGTAAG
GCGAGGCAACGCTGTACTGGTTTAAATTTAATTCACTATAATGCAAAATCATGACAAAAC
CGGCGCGAGGTTACACAAACGGATGAAATCAACCGATATTCAAACACAGTCATTTTTAGC
GCATTTTCAGCGTATCGTTAATGCGGAAAATTTCGTGAACAGGTTTTTTGCACAGGCCTC
CGCCTCGTTTCGCGGGATGGGAAACCGTATTAGAAAACGGACGCACGCAACATAAACCCC
GAAAGTGATGATAAGATGATGATTTAACGTACTGCTTTAATTATTTAAGGAATTATCGTG
TTTCCCGACAAATACAAGTTGAGTTTGAAAGAAAATATTTTTTTGGCAAAGAAAGTATTG
GTTGCCCAAATTCACAACCTCAGTCGTTTTGAGAATTGTCAGACGACCTTGTTGCAGACC
GAACAAATTATCCATGGCAAAAATGTAGCCTCCGCGTCACTGGAAGACATCCAAACCATC
TTGAACCTGAAACGTGCCTATCAATATGTGATTTCGCATATTTCAAACGGCGAACCGGTC

## Appendix A

GATATTTCACTCCTTAAAAAAATCAACAACATTGTTGCCAAGGACGATTCTTTGGCACCC
GGTGATTTCCGTACCGGTTCGGTCGGCGTAACGCTATTGGACGGTTCCCGTCATGCCCCG
AATCCAGTGAAGGAAATTGAAGTGGCCCGCGTGTTACAAAATATCGGACTGCAAAGCGGT
TCGACGACGGAGGCAGCCGTCCGTTTTATGCTTTATTGTATGCGGCAGCAGGTTTTTTGG
GACGGCAACAAACGAACGGCAACCTTATTTGCCAACGGTCTGATGATGGCGGGGGGCTGC
GGCATCTTGGAAATCTCCGAAATGCAGATGCCGCAATTCAATGAAAAACTGTCCGCATTC
TATCGCTCCGGCGACGATACCGATATTTCCAAGTTTGTGTATCAAAATTGTATATCGGGC
ATAGACTGAGACCTTTGCAAAATTCCCCAAAACCCCTTAAATTCCCACCAAGACATTTAG
GGGATTTTCCATGAGCACCTTCTTCCAGCAAACCGCCCAAGCCATGATTGCCAGACACAT
CGACCGTTTCCCGCTATTGAAGTTGGACCAGGTGATTGATTGGCAGCCGATCGAGCAGTA
CCTGAACCGTCAAAAAACCCGTTACCTTAGAGACCACCGCGGCCGTCCCGCCTATCCCCT
GCTGTCCATGTTCAAAGCCGTCCTGCTCGGACAATGGCACAGCCTCTCCGATCCCGAACT
CGAACACAGCCTCATTACCCGCATCGATTTCAACCTGTTTTGCCGTTTTGACGAACTGAG
CATCCCCGATTACAGCACCTTATGCCGCTACCGCAACCGGCTGGCGCAAGACGACACCCT
GTCCGAACTGTTGGAACTGATTAACCGCCAACTGACCGAAAAAGGCTTAAAAGTAGAGAA
AGCATCCGCCGCCGTCGTTGACGCCACCATTATTCAGACCGTCGGCAGCAAACAGCGCCA
GGCTATAGAAGTCGATGAGGAAGGACAAATCAACGGCCAAACCACACCGAGTAAGGACAG
CGATGCCCGTTGGATAAAGAAAAACGGCCTCTACAAACTCGGTTACAAACAACATACCCG
TACCGATGCGGAAGGCTATATCGAGAAACTGCACATTACCCCCGCCAATGCCCATGAGTG
CAAACACCTGCCGCCTTTGTTGGAAGGACTGCCCAAAGGTCGACCGTCTATGCCGACAAA
GGCTACGACAGTGCGGAAAACCGGCAACATCTGGAAGAACATCAGTTGCAGGACGGCATT
ATGCGCAAAGCCTGCCGCAACCGTCCGCTGACGGAAACGCAAACCAAACGCAACCGGTAT
TTGTCGAAGACCCGTTATAGTGGATTAAATTTAAATCAGGACAAGGCGACGAAGCCGCAG
ACAGTACAAATAGTACGGCAAGGCGAGGCAACGCCGTACTGGTTTAAATTTAATCCACTA
TATGTGGTCGAACAGAGCTTCGGTACGCTGCACCGTAAATTCCGCTACGCTCGGGCAGCC
TATTTCGGACTGATTAAAGTGAGTGCGCAAAGCCATCTGAAGGCGATGTGTTTGAACCTT
TTGAAAGCGGCCAACAGGCTAAGTGCGCCCGCTGCCGCCTAAAAAGCAGCCCGGATGCCT
GATTATCGGGTGTCCGTGGAGGATTAAGGGGGTATTTGGGTAGAATTAGGAGGTATTTGG
GGCGAAAATAGACGAAAACCTGTGTTTGGGTTTCGGCTGTCGGGAGGGAAAGGAATTTTG
CAAAGGTCTCAGACTATTTCGGCACGGACGAAGATATAGATTTCCCCGACCCACCAAACA
TGGGCTAAAAATCAATTTGACGGTTATCAGACAATGGAGCAGGCACAAGGCGGCGGCAGA
AAAAGGGTTTGACAGCGCACGGTGGCATCGTCAGACCCCTTTCGGCATATCCGGCGGTTA
CCAGCGGTAGCCTAATTTGATGCCCGCGCTGTGTTGCGCTTCCAGTTGCGGGCCTTTGGC
GGCGGCAGCGTGGAGGGACAGCGTGAAACCTTTGATTTCGGCGTTTACGCCCCATTCCGC
ACTGCGGGTTTTGCCGAAATCCTGAGCCAATACGGCGGTATTGACGCGTGTTCGGACTTT
GCCCGAAGCGGCATCGGTATAGGACAGGCTCAAATAAGGCGTGATGGAAATGTGTTGCGC
CGGTTTGAATGAATAATCTGCCTTAATGCCCGCGCGGTAGCGGTTGAATGCAAGGCCGGG
GGTGGCGATATTGACGTTTTCGTAGCGGTAATCCGCTTTTTGGACGAAATAGCGCGTTGC
GCCGATGTGCGGTTCGATGCCGAATCCGCCGAAACCGGCGCGGTATCGTGCCTGAATGCC
GTAATGCAGCACGCGGCGGCGGATTTTGCCTCCGATGCCGTCTGAAAGGCTGCCGCTGCT
AAAACCCGCGCCCGCGCTGATGCCGATGTAGAACCTGTCGATGCCGTATTGCCCGAAAAC
GGCGCCGTGGGCAAGCCGTGCCGAGTTGCCGATGCCGTCGTCGAAGGTGTTTTCGGTCCG
GTTGTGCGAAAACAGGATGCCGACGCGCCCGCTGCCGAGGTTTTTCTGCATACCGATTTG
GCGCAGGTCGGTTTGTTGGCGGTAGGCGCGGAAATCTTGCGAACGGTAGTGTTTGGTGTC
CCGGATGCCGCTTGTCCAAACGGCGTTGCGGCGGTCTTCGGCAAATACGCGGTCTAATTC
GTCCTGTACGGCGAAAACGCTGTTGAGCGTGGCGGAAAATTCACTCAAACCGCTATTGGC
ATAACGGCTGATCAGGTCGCGCTGCGGTTGGGGCTGCGGTTGGGGTTGCAGTTGCGGCAA
ATCCCGGCGGGCGCGGCGGGCGCGGGGGAAGGCGGTGGTAGCCGGCCGGGTTTCCGCTTC
GCGCTGTTTCGCCAAGGCGGTGTCTTTATCCGCCTGCACCCGTTTTTTCTCTTCCTCCGC
CTGCATAATGCCGACATTTTCCCCGCCTGCCTGCCGGGCCGGTTCGGCAACGCTTTCTGT
CTTTTCGACGGCATCGCGCCCGGCCGCAATCAGCGCGTCAAGGCTTTGCGCGTTGTCTTT
TTCCGCCTGTTTTTTGGCTTCTGCCTTGCCGAGTTTGTCGGAAAGCTCTTGTTCTTTGAC
CGGATTATGCAGGCGGAACTCGCCGTCTTTGCGGATGAGTTGGTAACGCCACGCGCCGGC
ATCGACGTGTTCGTTTTGCAGGGTGAAATTAAGGTTTTCGGACAGCGGTTTGTTGTCTTT
TCCTTCCACTACCGTCAATTGTTCGAGGCTTGCAGGTTCGTTGCCGGTATTGTTGACCGC
CAAGGTGTAAGTGCCTTCGGAACTTTCCGCCAGCTTCAATTTGTCGCTGCGGTAGCCGAA
GAGTTCCGACATAAAGCGGAATGTTCCCTGACCGTTCAATTTGCCGTTTACCGTCAGCGT
GTTGAAACGGGATTCTACCGAAGTTGGCGGTGTAACGGATAATAGGGAACGGCGCGAACG
GCGCGAACGGCGGCGCGGCGCATCTGTCGCACTGCCGGTTTGCGCCCCTGCCGCATCGTG
GCGATAGGCGGAATTGAGTGTAATGGTGGCGTTGTCAAGGTTTAAATTGCCTAATTCCGT
GCCTGACGGCAGCGTCCATTCGCTGTCTTTTAAGTGTAATGCCGTATCCTTGCCGCCGCT
GATTTGTCCGGTAAAGCGGCTGCTTTCAAAATGGAATACTGCCTTATCGGCTAGGGAGAC
ATTACCGTTGAGTGCGGAATGGCTTACGTTTGCCTTAGCGTTGCCGGAAAGCGTCAGACT
GCCGTTTTGTACGGCGTGGTCGCTTAGATTAAATGAAGCATTGCCCGAAGCCGATGTGTT
GCCGTTTAATGTGGCTTGATTAAATGTTGCTTGGGCATTGCCCACGAGGCTAAGGTTGCC
GTTTTGGGTGGCGTTGTGGCTGACTGTATAACGTGTATCGCCATTTGCACTAAGATTGCC
GTTGAGTGTGGCAAGCCCTGTGAGATTTAAATGAGCGTGATCGGCAAGATCGACATTGCC
GCTGATGTCGGTCTTAGTCAATGAAGCAATCACTTTATCGTCGGTAATGGTTTTTTCGAC
ACAATTTGTCAGACCCGTCCAGTCCGAACGTGTACAGATTGTGTGGCTTTGATGCGGTGC
GACACCAAAAACTGCTTGGGCGTGATTGCTCAAATGCCAATCGCCTTTCACTTTGGCAAC
ATTGCGGGAAACCACCGCCTGTCCGCCTTTAATTTGGAAGTTTTCCGCTTTAAATGTGCG
GTTGATCCAGTCGTTGTCCCACACGATTTCCCCGCGAGGAATGCCCTCTTTTTGCGACCA
ATGGTCGTTTAAATGATTGTAGGCGTGCGGTGTTGGTCTGCCGCTGAAAAACAGTTTGCC
GTTTGTTTGCGTGATGTTGCCGTTTAAATTTGTTCCGCCGGAAAGCAGCAGGGTGCGGTC
TTCTGCGGCGGGCTGGTAAACAAGGTTGAGCCGCCCGTTCGTTTTGGTCGTATCTTTCTC

## Appendix A

GCCAAACCAACCGTTGTAGGCAATTTCTTTTTTGCTATCCAAGCTGTTGTTATTGCCGGT
TGTAGCAATATCTTTATTGCCTGTAATGGTAACGGTGGATTCTTTGTCTTGATTGTGGTT
GACAATCATCGCCCCTTCATCGGTATTTTGAATACGGTGGAACGAAAGCGAATGCCCGTT
TAAATCCAAACGTCCGCCGCGAAAGCCGAAATAGAGTTTGTCGGGGTTGAACTGATTATC
GGCATTCAGTTGCACCGTACCCCTGCCGCTGACCAAGCCGATTTCACTAAAGGCTTGTTT
TTTGCCTTTATCGTCTGCCTGCTGATCCAAAATGACTGTACCGTCGCCCACGCTGATCGA
GCCTTGGTTTTCCCCTTTGGCTTGAACGTGCAGCGTGCCTTTGCCGATTTTGGACAGGCG
GTCGTTTGCCACGCCGTTTACTTTCCAAGTAACGGTACTGTCTTCACTGATATGAACGCC
CGCGCCTTGCCAAGTTTCGTTATTTTCAGGCGAGACCGTAAAATCTCCTTGGAAATATAA
TCCTCCAGCACCTTGATTGATGTTGCTGGTAAGTATCAATTCGCCTTTTCCTTCGTCAAT
AAAGGAAATATTTTCTCCATTATTCAGTCTGGGTCGATAACTGTTGACACCACCTGCAGC
ATGATAAACAGGTTCTCTTGCTGTCTCGGATAAAGAAACATTAAACAATTGAACGGTTCG
TGTTTTTAATCTATTAGGCAGAGAATTGTGTTCATGTTTGGCATTGATTTTTCCTGTGCC
ATTATTATCGTCGTTAAAAGAGTATTTCCCATTTTGACGTGGTTCGTAGAATACTGAATG
GGTATCTCCAGCAAAGATTTCATCATAGAACCAATCTTTACGAACCAGCTGGAAGCCATT
GCTTTTTCCTATATAGGGGTTGCCCGTTTGCAATACCCCATTAATTAACCACTTTTGCTT
TTGGGCATCATAGATAAACATTGGTGAGCCACTGTCGCCAAATGAGCCTCCTGTTGGTAA
AAAACCATATGGGCTATGTTTAATTTTTTCACTACCTAAGTTGACTGTGCCACCACCTGA
TCCATTTTGTGCAAAGGTATTGCCACCAACGAGCCAAGAATACGCACTTGCAATATGATA
TGAACTTTCGCGGTTATTGGGCTCATCTTCATCAGATCGCCAATATTGCCTGCCTGCCCC
AATACGAACACGGTCAGGGTAATTATTTTGATCGATATATTTCCGCCCATCCATATAACT
GGTCATTTCAACAGGTTCTGCATCTGTGACAAATTTATGCAAACGCGGCATATGATAATC
GCCGCCATAAGGATGGCCTTTAGTCCCTGCTTTATAATTATTCCGTTTCACAATTTTATA
AGTAAAACGATGTTGATCGGGATTTCTTCCTTCCGCACCAAAATCAACGTTGTTATAGCC
GCCGTTATGTGCCACGCTCACAATATATTGATCGCCCACCAATGCCGCCACGCCGTTACG
CGACACCACAGAAAAATCAATCATCGGGGCTTTTGTCATTGATTTGCCGACCAACTCCCC
TTTTTTGTTGTAAACCTCAATATCTTTCGCCCCGACTGCAAACTTGCCTTTATTTTCGGC
AAAGTCGCGATAGTATTGGTAGTTGATGCCGAAATAAGTGTGTCCCGCCCAGGCTTGGGG
AAGAATGCCGAACGACAGGCATATGGCTAAGTAAGCAGGCGAGAAGCGGATGCGGCCGGT
TTTCGGGGCTTTGCGGTGTGTTTCGGTTGTCCGTTTGTCGGTTGTTTTCATTATTTTTCC
TTATCTGACGGGATTCGGGTTTGTTTGGGAGGGCGCGGCTTCCGCTTCCGGGCGGCGCGC
GGGATGTGCCTATATGTGCGGTTCGGCGTTCGGGCGGATATGAAGCACGCCCTAGGATTT
GTCATTAATTTTTGCCTTGGTCTCGGCTTCTTCCAATCACGAAAGCACCCGCCAAGGCAA
ACACTGTGCCGCCGGCAAGGGAGGCGGCGGTTGCGGGGTAGCCGCTCCATACGAGGAAGA
CGGCAAAAAGCAGTATCAGGATGGCGCTGATGAAGCCGTACAGTTGCCCGCGCCTGTTGA
AGGTTTGGTCTTGCCGTATGGTTTCGTGCCGGACGGCTTGTTCTTTTTCCGCCATTGCCA
TAATGCGGTCTGCCCCGTTGCTGATAATGTCGTTGTATTGCGCCAAGTCGGACGGCGGCG
GCAACGGTCCCGAATGGAAACACCGGGCTATCATTATTTGCACGTACTCGTCGGACAGGA
TTTGCTCGACAAGCTCCGGGGATTTGACGACGGTTTCGACAGCCTGCCGCGCCTTGTCCT
GTGCGTTTTCGGTCATTTTCGCGCTTTCTCTATGGCGCGTTGAAAATCGCCGCCGATGTT
TTTGAGATCGTCGGCGGGATTGGGGCGGATGGCGGTTTTTGCGGGATGGAACAGACCCAG
CAGCGAGCCTATACCGAGCAGGAGGGCGTATGTGTTTCGTTTTTTCATATGGTTATATAT
TAGGTCAGGCGGACGGATTTATCAAGCATTTTTGCGGTTTTATACCGTCTGAAAGCCAAA
CCGTCGGACTTCAGACGGCATTTGCTATAATCGCGGCTGTTTTGAATTTTCGGGGGTTTT
ATGTCGGATAACGTTCCAACGATTGCGGCAGTCGCTACCGCACCAGGGCGCGGCGGCGTG
GGCGTGATACGCATATCGGGGAAAAACCTGCTGCCGATGGCGCAGGCTTTGTGCGGGAAA
ACGCCCAAGCCGCGTACCGCAACCTATGCTGATTTTACGGACACGGACGGACAGGCAATC
GACAGCGGGCTTTTGCTGTTTTTTGCCGCACCGGCAAGTTTTACGGGTGAAGATGTCATC
GAGCTTCAGGGACACGGCGGGCCGGTGGTGATGGATATGCTGCTGAACCGCTGTTTGGAA
TTGGGCGCGCGCCTTGCCGAACCGGGCGAGTTTACGAAGCGTGCGTTTTTGAACGACAAA
CTGGACTTGGCACAGGCGGAAGGCGTGGCGGATTTGATTGACGCATCCAGCCGTTCGGCG
GCGCGTCTGGCTTTGCGCTCGCTCAAGGGCGATTTTTCGCGGCGGATACACGGTCTGGTC
GAAGACTTGATTACCTTGCGGATGCTGGTCGAAGCGACGTTAGATTTTCCCGAGGAAGAC
ATTGATTTTCTCGAAGCGGCAGACGCACGCGGCAAACTGGACGGCTTGCGCCGCGCCGTG
GATGATGTGCTTGCCAACGCGCAGCAGGGCGCGATTTTGCGCGAAGGTCTGAATGTCGTA
TTGGTCGGCGCGCCGAATGTGGGCAAGTCCAGCCTGCTGAACGCGTTGGCGGGCGACGAA
GTGGCGATTGTTACCGATATTGCCGGAACGACGCGCGACGCGGTCAGGGAACGTATCCTG
ATTGACGGCGTGCCGGTGCATATTGTCGATACGGCAGGTTTGCGCGAGACGGACGACGTG
GTCGAGCGTATCGGCATCGAACGCAGCCGCAAAGCCGTATCCGAAGCCGATGTCGCGCTG
GTGTTGGTCGATCCGCGCGAGGGTTTGAATGAAAAGACACGGGCGATTTTGGACGCGTTG
CCGCCGGAGTTGAAACGCATCGAAATCCACAGCAAATCCGATTTGCACGCACACGCGGCA
GGCGGGTTCGGTACGGGCGCGGAAACCGTCATCGCGTTGTCGGCGAAAACCGGCGACGGC
TTGGACGCGCTGAAACGGACGTTGTTGCGCGAGGCCGGTTGGCAGGGCGAAAGCGAAGGG
TTGTTTTTGGCGCGGACGCGGCACGTCAACGCACTCAAAGCAGCGCAGGAAGAATTGTCG
CTGGCGGCATTGTGCGGCAACCATCAAATCGAGCTGTTTGCCGAACACTTGCGCTTGGCG
CAGGTCGCATGCGGCGAAATCACGGGCGAGTTTACGGCGGACGACCTGCTCGGCGTGATT
TTTTCGAGGTTTTGTATCGGAAAATAAACGGATCGAAAGCATCGTGGTGGTGTCCGGCTG
AACATTCCGTTATCCCATAAAAACGGGAATCCGATCCGTTTGGTTTTATAGTGGATTAAC
AAAAATCAGGACAAGGCGACGAAGCCGCAGACAGTACAAATAGTACGGAACCGATTCACT
TGGTGCTTGAGCACCTTAGAGAATCGTTCTCTTTGAGCTAAGGCGAGGCAACGCCGTACT
GGTTTTTGTTAATCCACTATAGTTTTTTTGAATTTCGGGCAACGCTTGAATCTTCATTCC
GCGCAGGCGGAAATTATCGGTGCGGTACGGCAACTTTTTTCGATATGAAAAGACCGTCAT
TCCTGTAAAAACAAAAAATCAAAAACAGAAAATTGAAATTCGTCATTCCCGCGCAGGCGG
GAATCCAGGACGTAAAATCTATAGTGGATTAACAAAAACCAGTACAGCGTTGCCTTGACT
TAGCTCGAAGAGAACGATTCTCTAAGGTGCTGAAGCACCGAGTGAGTCGGTTCCGTACTA

# Appendix A

TCCGTACTGTCTGCGGCTTCGTCGCCTTGTCCTGATTTTTGTTAATCCACTATAAAGAAA
CCGTTTTTCTCGATAAGTTTCCGTGCCGACAGACCTGGATTCCCACTTTCGTGGGAATGA
CGGTGGAAAAGTTGCCGTGATTTCGGATAAATTTTCGTAACGCATAATTTCCGTTTTACC
CGATAAATGCCCGCAATCTCAAATCCCGTCATTCCCCAAAAACAAAAAATCAAAAACAGA
AATATCGTCATTCCCGCGCAGGCGGGAATCTAGACCTTAGAACAACAGCAATATTCAAAG
ATTATCTGAAAGTCCGGGATTCTAGATTCCCACTTTCGTGGGAATGACGAATTTTAGGTT
TCTGTTTTTGGTTTTCTGTCCTTGCGGGAATGATGAAATTTTAAGTTTTAGGAATTTATC
GGAAAAAACAGAAACCGCTCCGCCGTCATTCCCGCACAGGCTTCGTCATTCCCGCGCAGG
CTTCGTCATTCCCGCATTTGTTAATCCACTATATTCCCGCCGTTTTTTTACATTTCCGAC
AAAACCTGTCAACAAAAAACAACACTTCGCAAATAAAAACGATAATCAGCTTTGCAAAAA
TCCCCCCCCCCCTGTTAATATAAATAAAAATAATTATTAATTATTTTTCTTATCCTGCCA
AATCTTAACGGTTTGGATTTACTTCCCTTCATACTCAAGAGGACGATTGAATGAATACCC
CATTGTTCCGTCTCAGCCTGCTCTCGCTTACCCTGGCGGCAGGTTTTGCCCATGCGGCAG
AAAATAATGCCAAGGTCGTACTGGATACCGTTACCGTAAAAGGCGACCGCCAAGGCAGCA
AAATCCGTACCAACATCGTTACGCTGCAACAAAAAGACGAAAGCACCGCAACCGATATGC
GCGAACTCTTAAAAGAAGAGCCCTCCATCGATTTCGGCGGCGGCAACGGCACGTCCCAAT
TCCTGACGCTGCGCGGCATGGGTCAAAACTCTGTCGACATCAAGGTGGACAACGCCTATT
CCGACAGCCAAATCCTTTACCACCAAGGCAGATTTATTGTCGATCCCGCTTTGGTTAAAG
TCGTTTCCGTACAAAAAGGCGCGGGTTCCGCCTCTGCCGGTATCGGCGCGACCAACGGCG
CGATCATCACCAAAACCGTCGATGCCCAAGACCTGCTCAAAGGCTTGGATAAAAACTGGG
GCGTGCGCCTCAACAGCGGCTTTGCCAGCAACGAAGGCGTAAGCTACGGCGCAAGCGTAT
TCGGGAAAGAGGGCAACTTCGACGGCTTGTTCTCTTACAACCGCAACAATGAAAAAGATT
ACGAAGCAGGTAAAGGCTTCCGTAATAATTTCAACGGCGGCAAAACCGTACCGTACAGCG
CGCTGGACAAACGCAGCTACCTCGCCAAAATCGGAACAAGCTTCGGCGACGGCGACCACC
GCATCGTATTGAGCCATATGAAAGACCAGCACCGGGGCATCCGTACCGTCCGTGAAGAAT
TTACCGTCGGCGGCGATAAAGAGCGAATAAGTATGGAACGCCAAGCCCCTGCTTACCGCG
AAACCACACAATCCAACACCAATTTGGCGTACACGGGTAAAAACCTGGGCTTTGTCGAAA
AACTGGATGCCAACGCCTATGTGTTGGAAAAAGAACGCTATTCCGCCGATGACAGCGGCA
CCGGTTACGCAGGCAATGTAAAAGGCCCCAACCATACCCAAATCACCACTCGGGGTATGA
ACTTCAACTTCGACAGCCGCCTTGCCGAACAAACCCTGCTGAAATACGGTATCAACTACC
GCCATCAGGAAATCAAACCGCAAGCGTTTTTGAATTCACAATTTAAAATTGAAGATAAAG
AAAAAGCAACTGATGAAGAGAAAAATAAGAACCGTGAAAATGAAAAAATTGCCAAAGCCT
ACCGTCTGACCAACCCGACCAAAACCGATACCGGCGCGTATATCGAAGCCATTCACGAGA
TTGACGGCTTTACCCTGACCGGCGGGCTGCGTTACGACCGCTTCAAGGTGAAAACCCACG
ACGGCAAAACCGTTTCAAGCAACAACCTTAACCCGAGTTTCGGCGTGATTTGGCAGCCGC
ACGAACACTGGAGCTTCAGCGCGAGCCACAACTACGCCAGCCGCAGCCCGCGCCTGTATG
ACGCGCTGCAAACCCACGGCAAACGCGGCATCATCTCGATTGCCGACGGCACGAAAGCCG
AACGCGCGCGCAATACCGAAATCGGCTTCAACTACAACGACGGCACGTTTGCCGCAAACG
GCAGCTACTTCTGGCAGACCATCAAAGACGCGCTTGCCAATCCGCAAAACCGCCACGACT
CTGTCGCCGTCCGTGAAGCCGTCAATGCCGGTTACATCAAAAACCACGGTTACGAATTGG
GCGCGTCCTACCGCACCGGCGGCCTGACTGCCAAAGTCGGCGTAAGCCACAGCAAACCGC
GCTTTTACGATACGCACAAAGACAAGCTGTTGAGCGCGAATCCTGAATTTGGCGCACAAG
TCGGCCGCACTTGGACGGCTTCCCTTGCCTACCGCTTCCAAAACCCGAATCTGGAAATCG
GCTGGCGCGGCCGTTATGTTCAAAAAGCCGTGGGTTCGATATTGGTGGCAGGTCAAAAAG
ACCGCAACGGCAAATTGGAAAACGTTGTACGCAAAGGTTTCGGTGTGAACGATGTCTTCG
CCAACTGGAAACCGCTGGGCAAAGACACGCTCAATGTTAATCTTTCGGTTAACAACGTGT
TCAACACGTTCTACTATCCGCACAGCCAACGATGGACCAATACCCTGCCGGGCGTGGGAC
GTGATGTACGCTTGGGCGTGAACTACAAGTTCTAAAACGCACATCCCGAAAAAATGCCGT
CTGAAAGCCTTTCAGACGGCATCTGTTCTGATAATTTGATATATAGTGGATTAACAAAAA
CCAGTACGGCGTTGCCTCGCCTTAGCTCAAAGAGAACGATTCTCTAAGGTGCTGAAGCAC
CAAGTGAATCGGTTCCGTACTATTTGTACTGTCTGCGGCTTCGTCGCCTTGTCCTGATTT
TTGTTAATCCACTATAAAGACCGTCGGGCATCTGCAGCCGTCATTCCCGCGCAGGCGGGA
ATCTAGACCTTAGAACAACAGCAATATTCAAAGATTATCTGAAAGTCCGAGATTCTAGAT
TCCCGCTTTCGCGGGAATGACGAAAGGTTGCGGGAATGACGAAAAGTGGTGGGAATGACG
AAAAGTGATGGGAATGACGAAAAGTGATGGGAATGACGGTTCGGGCATTCCTTAAATTAC
CCGTGTATCGCTGTAAATCTTAGAGATGGCGGAATATAGCGGATTAACAAAAACCAGTAC
GGCGTTGCCTCGCCTTAGCTCAAAGAGAACGATTCTCTAAGGTGCTGAAGCACCAAGTGA
ATCAGTTCCGTACTATTTGTACTGTCTGCGGCTTCGTCGCCTTGTCCTGATTTTTGTTAA
TCCACTATAGATTATCATTTATCCTTTCTAAAGCCGTTCCGGTTTGTCCGACCGGCGGCT
TTGCCCCAATATCCCCATTTTGGAGACACCTATGTTACGTTTGACTGCTTTAGCCGTATG
CACCGCCCTCGCTTTGGGCGCGTGTTCGCCGCAAAATTCCGACTCTGCCCCACAAGCCAA
AGAACAGGCGGTTTCCGCCGCACAAACCGAAGGCGCGTCCGTTACCGTCAAAACCGCGCG
CGGCGACGTTCAAATACCGCAAAACCCCGAACGCATCGCCGTTTACGATTTGGGTATGCT
CGACACCTTGAGCAAACTGGGCGTGAAAACCGGTTTGTCCGTCGATAAAAACCGCCTGCC
GTATTTAGAGGAATATTTCAAAACGACAAAACCTGCCGGCACTTTGTTCGAGCCGGATTA
CGAAACGCTCAACGCTTACAAACCGCAGCTCATCATCATCGGCAGCCGCGCCGCCAAGGC
GTTTGACAAATTGAACGAAATCGCGCCGACCATCGAAATGACCGCCGATACCGCCAACCT
CAAAGAAAGTGCCAAAGAGCGCATCGACGCGCTGGCGCAAATCTTCGGCAAACAGGCGGA
AGCCGACAAGCTGAAGGCGGAAATCGACGCGTCTTTTGAAGCCGCGAAAACTGCCGCACA
AGGTAAGGGCAAAGGTTTGGTGATTTTGGTCAACGGCGGCAAGATGTCGGCTTTCGGCCC
GTCTTCACGCTTGGGCGGCTGGCTGCACAAAGACATCGGCGTTCCCGCTGTCGATGAATC
AATTAAAGAAGGCAGCCACGGTCAGCCTATCAGCTTTGAATACCTGAAAGAGAAAAATCC
CGACTGGCTGTTTGTCCTTGACCGAAGCGCGGCCATCGGCGAAGAGGGTCAGGCGGCGAA
AGACGTGTTGGATAATCCGCTGGTTGCCGAAACAACCGCTTGGAAAAAAGGACAGGTCGT
GTACCTCGTTCCTGAAACTTATTTGGCAGCCGGTGGCGCGCAAGAGCTGCTGAATGCAAG

## Appendix A

CAAACAGGTTGCCGACGCTTTTAACGCGGCAAAATAATGAAACGGCGGCATTCGATGCCG
TCTGAAACACGGATGCAAACCGCCTCCTGTGTTTCAGACGGCATTGCCCGATACGGAGGC
TTCAAACAAGGCTTTCCGCTCCGACGGTTCGGACTGCCTTGTTTGAATCTTCTACGCCTT
AACGCTTTTCCCTTCTGTTTATGACTGCCAAACCTTTTTCCCTCAACCTGACCAACCTGC
TGCTGCTGGCGGTGTTGTTTGCCGTCAGCCTGTCGGTGGGCGTTGCCGATTTCCGCTGGT
CTGATGTGTTTTCACTGTCCGACAGCCAGCAGGTCATGTTCATCAGCCGCCTGCCGCGCA
CGTTTGCGATTGTGCTGACGGGCGCGTCGATGGCGGTGGCCGGCATGATTATGCAGATTT
TGATGCGCAACCGTTTTGTCGAACCGTCGATGGTGGGCGCAAGCCAAAGCGCGGCTTTAG
GTTTGCTGCTGATGACCCTGCTGCTGCCGGCCGCGCCGCTGCCGGCGAAAATGTCGGTTG
CCGCCGTTGCCGCGCTGATCGGGATGTTGGTCTTTATGCTGCTGATCCGCCGCCTGCCGC
CGACCGCGCAACTGATGGTGCCTTTGGTCGGGATTATTTTCGGCGGTGTGATTGAGGCGG
TAGCCACCTTTATCGCGTATGAAAACGAAATGCTGCAAATGCTCGGCGTGTGGCAGCAGG
GCGATTTTTCGAGCGTGCTGCTGGGGCGGTACGAGCTGCTTTGGATTACGGGCGGTTTGG
CGGTGTTTGCCTATCTGATTGCCGACCGGCTGACGATTTTGGGGCTGGGCGAAACGGTAA
GCGTGAATTTGGGTTTGAACCGGACGGCGGTGTTGTGGTCGGGTTTGATTATTGTGGCTT
TGATTACGTCGCTGGTTATCGTTACGGTCGGCAATATTCCGTTTATCGGGCTGGTCGTGC
CGAACATCATCAGCCGCCTGATGGGCGACAGGTTGCGCCAAAGCCTGCCTGCGGTGGCCT
TGCTGGGCGCATCTTTGGTGTTGCTGTGCGACATTATCGGACGCGTGATTGTGTTTCCGT
TTGAAATTCCGGTCTCTACGGTTTTTGGTGTATTGGGTACGGCTTTGTTTTTGTGGCTTT
TGTTGAGGAAACCCGCCTATGCCGTCTGAAAAAAATATCGGTTTTATGGCAGGAAGCAGC
CGCCCGTTGTGGGTCGCCTTTGCGCTGTTGCTGGTTTCCTGCGTCCTGTTTATGACGCTC
AACGTCAAAGGCGATTGGGATTTTGTTTTGCAACTGCGGCTGACCAAACTTGCCGCGCTG
CTGATGGTCGCCTATGCGGTCGGCGTGTCCACGCAACTCTTCCAAACGCTGACCAATAAT
CCGATTCTGACCCCTTCAATTTTGGGTTTCGATTCGCTGTATGTGTTTTTGCAGACCTTG
CTGGTGTTTACGTTCGGCGGCGTGGGCTATGCTTCCCTGCCGTTGACGGGCAAATTCGGC
TTTGAACTGGTCGTCATGATGGGCGGCTCGCTGCTGCTGTTCTACACGCTCATCAAACAG
GGCGGACGCGATTTGTCGCGCATGATTTTAATCGGCGTGATTTTCGGGATTTTGTTCCGC
AGCCTGTCGTCGCTGCTTTCGCGCATGATCGATCCCGAAGAATTTACCGCCGCGCAGGCG
AATATGTTTGCCGGATTCAATACCGTCCACAGCGAGCTTTTGGGCATAGGCGCGCTGATT
CTGCTCGTCAGCGCGGCGGTCGTTTGGCGCGAACGCTACCGCTTGGACGTTTACCTTTTG
GGGCGTGACCAAGCCGTCAATTTGGGCATCAGCTACACGCGCAACACCTTATGGATACTG
CTTTGGATTGCCGCATTGGTGGCGACGGCGACCGCCGTGGTCGGCCCCGTAAGCTTTTTC
GGGCTTCTCGCCGCCTCGCTTGCCAACCACTTTTCCCCGTCGGTCAAACATTCCGTCCGC
CTGCCGATGACGGTTTGTATCGGCGGCATCCTCTTGGTCGGCGGACAGACCGTGTTCGAA
CACCTGCTCGGTATGCAGGCAGTGTTGAGCGTAGTAGTAGAATTTGCCGGCGGACTCGTT
TTCCTCTATCTCGTTTTAAAACACAAAAAATGACGGATGCCGTCTGAACGGCCGCCGCCC
CGAAAGGACAAACCATATGACACAAGAACATTTCCCATCATTCTTCAACCAAGCCCCGAC
CATTACCGTCCAAGACGCATTGGCCGAATTCCTCGGCGCGGCCGAAAACGGCATCCTCAC
TTACCGCTACGCCGATGCCGTGCGCCTGTGCGGACATTCCTGCCCGACCGTCGCGGGCGC
GTACCTGATGGTTATCAAAGGTCTGAAAGCACTTTACGGCGAAGAGCTGCCCGAACGCGG
CGGCATCGAAGCCTTTATGCAGGGCGCGCGCGACGAAGGCACGGTCGGCGTAACCGCGTC
CGTCGTCCAACTCCTCACCGGCGCAGCCCCCGAAACCGGCTTTGGCGGCATCGGAATGCA
GGGACGCTTTGCCCGCCGCCACCTCTTATCCTTTGGTGTAGGCGAAATCAACGGCACACT
GACCCTGCGCCGCAAAGACAACGGCAAAACCGTCGCCGTCGGCCTCAACGCCGCCCTGCA
ACCCTTCGCACCCGAAATGCGCGACATCATGCCCAAAGCCGTCAGCGGCAGCGCAAGCGC
AGAAGAACTCGAACGCTTCGGACAACTCTGGCAGGCACGCGTTAAAGCATTTTTAACCGA
ATCGGCGGACGACCCGCAGTTCGTCATCGTCCGCGAAGTGTGAGCGTTCAGACGGCATTC
CGAATTTCAAATGCCGTCTGAACCCCGCCAAACAACAACAAACCTACGCCCGACAAGCAT
CCGCCATGATTACCATCCGCAACGTCAGCTACCGCATCGGCACACGCCCCATCCTCGACA
ACGTCAGCCTCGACATCCCCGAAGGCGGCATTACCGCCCTCGTCGGCCCCAACGGTGCGG
GCAAATCCACCCTGTTTTCCTTTATGGCGCGGCTGCGACCGCTTGAAAGCGGCAGCATCG
CCTACCGAGGCAAAAATCTTGCCGATACCCCCACCGCCGAACTCGCCAAAACCCTGTCCA
TCCTCACCCAAGAAAACAGCATCATGAGCCGCATCACCGTGCGCGACCTGCTGATGTTCG
GCCGTTACCCCTACCATCAAGGCAGACCGACTGCCGAATGCCGCCGTATCGTTAACGGTG
CAATCGAAGAATTCCACCTGCAAGACCTCTCCGACCGCTACCTGACCGAGCTTTCCGGCG
GCCAACGCCAACGCGCCATGATTGCGATGGTGTTCTGCCAAAGCACCGACTACGTCCTTT
TGGACGAACCGCTGAACAACCTCGATATGTATCACGCCCGCTCGCTCATGCAAATCCTGC
GCCGGCTGACCGACGAACACAAGCGCACCACCGTCGTCGTATTGCACGACATCAACCAGG
CAGCAGCCTACGCCGACCACGTCGTCGCCATGAAAAACGGCCAAGTCGCCATGCAGGGCA
AACCCAACGATATTTTCACCGCCGCAAACATCAAAACCCTATTCGATATGGACGTCGACG
TCCTCGATTACGAAGGCAAAAAATTGGTTATCCACCATATCTAAATCCGACAAAAAGGCC
GTCTGAACATTCAGACGGCAACCCATATCCTGACAAAATTAAGACACGACACCGGCAGAA
TTGACATCAGCATAATATGCACATATTAACAGATATTAATGCCGAACTACCTAACTGCAA
GAATTAAATAAATAAATAAATAAATAAATAAATAAATTGCGACAATGTATTGTATA
TATGCCTCCTTTCATATATACTTTAATATGTAAACAAACTTGGTGGGGATAAAATACTTA
CAAAAGATTTCCGCCCCATTTTTTATCCACTCACAAAGGTAATGAGCATGAAACACTTTC
CATCCAAAGTACTGACCACAGCCATCCTTGCCACTTTCTGTAGCGGCGCACTGGCAGCCA
CAAGCGACGACGATGTTAAAAAAGCTGCCACTGTGGCCATTGTTGCTGCCTACAACAATG
GCCAAGAAATCAACGGTTTCAAAGCTGGAGAGACCATCTACGACATTGGTGAAGACGGCA
CAATTACCCAAAAAGACGCAACTGCAGCCGATGTTGAAGCCGACGACTTTAAAGGTCTGG
GTCTGAAAAAAGTCGTGACTAACCTGACCAAAACCGTCAATGAAAACAAACAAAACGTCG
ATGCCAAAGTAAAAGCTGCAGAATCTGAAATAGAAAAGTTAACAACCAAGTTAGCAGACA
CTGATGCCGCTTTAGCAGATACTGATGCCGCTCTGGATGAAACCACCAACGCCTTGAATA
AATTGGGAGAAAATATAACGACATTTGCTGAAGAGACTAAGACAAATATCGTAAAAATTG
ATGAAAAATTAGAAGCCGTGGCTGATACCGTCGACAAGCATGCCGAAGCATTCAACGATA

## Appendix A

TCGCCGATTCATTGGATGAAACCAACACTAAGGCAGACGAAGCCGTCAAAACCGCCAATG
AAGCCAAACAGACGGCCGAAGAAACCAAACAAAACGTCGATGCCAAAGTAAAAGCTGCAG
AAACTGCAGCAGGCAAAGCCGAAGCTGCCGCTGGCACAGCTAATACTGCAGCCGACAAGG
CCGAAGCTGTCGCTGCAAAAGTTACCGACATCAAAGCTGATATCGCTACGAACAAAGCTG
ATATTGCTAAAAACTCAGCACGCATCGACAGCTTGGACAAAAACGTAGCTAATCTGCGCA
AAGAAACCCGCCAAGGCCTTGCAGAACAAGCCGCGCTCTCCGGCCTGTTCCAACCTTACA
ACGTGGGTCGGTTCAATGTAACGGCTGCAGTCGGCGGCTACAAATCCGAATCGGCAGTCG
CCATCGGTACCGGCTTCCGCTTTACCGAAAACTTTGCCGCCAAAGCAGGCGTGGCAGTCG
GCACTTCGTCCGGTTCTTCCGCAGCCTACCATGTCGGCGTCAATTACGAGTGGTAAGCAG
CATCTCCCGATAAAGAAACCGCAGCCCTGCAAGGCTGCGGTTTTTATTTTCTATCCGGCC
GTCAGACTGCCGCGTCCGAACGTTCGCCCGTGCGGATACGGATTGCCTCCTCAACCGGCA
GCACAAAAATCTTGCCGTCGCCGATTTTTCCCGAACGCGCCACCTCGAAATCACGTCAAT
CGCGCGTTCCACAGCATCATCCGCCAACACCAGCTCGATTTTGATTTTGGGCAGGAAATC
GACGGCGTATTCCGCGCCGCGATAGATTTCCGTATGCCCCTTCTGCCTGCCGAACCCTTT
GACCTCGCTGACGGTCATGCCCGTAATGCCGATTTCCGTCAACGCCTCGCGCACGTCGTC
GAGTTTGAACGGTTTGACAATCGCCTCGATTTTTTTCATAAAATTTCCTTTGAACAAACA
ATACAAACACATCCGAAAAACGGGAACCTCCCGTCAGATTGTCAACATTTTAAGCCAAAA
TACCCAAGCAATACAGCCCCGTTGCGCGTATAATGACAGATTTTCCAACCGCATTTGAGA
GCCGAATCCATGTCTGTCGTTTTGCCCTTGCGCGGCGTTACCGCCCTTTCCGATTTCCGT
GTTGAAAAACTCTTGCAAAAAGCCGCCGCACTCGGTCTGCCCGAAGTCAAATTAAGCAGC
GAATTTTGGTATTTCGTCGGCAGCGAGAAAGCACTTGATGCCGCGACTGTCGAAAAACTG
CAAGCCTTGTTGGCGGCGCAAAGCGTTGAACAAACGCCAAAAGCGCGCGAGGGCTTGCAT
TTGTTTTTGGTCACGCCCCGTTTGGGTACGATTTCGCCGTGGGCTTCCAAGGCGACCAAT
ATCGCGGAAAACTGCGGTTTGGCAGGCATCGAACGCATCGAGCGCGGTATGGCGGTGTGG
CTGGAAGGTCGTCTGAACGATGAACAGAAACAGCAATGGGCGGCTTTGCTGCACGACCGC
ATGACCGAAAGCGTGCTGCCCGATTTTCAGACGGCCTCCAAATTATTCCACCATCTCGAA
TCCGAAACTTTCTCCGGCGTCGATGTTTTGGGCGGCGGTAAAGAAGCTTTGGTCAAAGCC
AATACCGAAATGGGCTTGGCACTTTCCGCCGACGAAATCGATTATCTGGTCGAAAACTAT
CAGGCTTTGCAGCGCAATCCGTCCGATGTTGAATTGATGATGTTCGCGCAGGCAAACAGC
GAACACTGCCGCCACAAAATCTTCAACGCCGATTTCATCCTCAACGGCGAAAAGCAGCCC
AAATCCCTCTTCGGTATGATACGCGACACACACAACGCGCATCCCGAAGGCACGGTCGTT
GCCTATAAAGACAATTCGTCCGTAATCGAAGGCGCGAAAATCGAGCGTTTCTATCCGAAT
GCGGCGGAAAACCAAGGCTACCGTTTCCACGAGGAAGACACGCATATCATCATGAAAGTG
GAAACGCACAACCACCCGACCGCCATCGCGCCGTTTGCGGGTGCGGCGACGGGCGCGGGC
GGCGAAATCCGCGACGAAGGCGCGACGGGCAAAGGTTCGCGTCCGAAAGCGGGCCTGACC
GGCTTTACCGTGTCCAACCTCAATATTCCCGACCTCAAACAGCCGTGGGAACAAGACTAC
GGCAAGCCGGAACATATTTCCTCGCCGCTGGACATCATGATTGAAGGCCCGATCGGCGGC
GCGGCGTTCAACAACGAATTCGGCCGCCCCAACCTCTTGGGCTACTTCCGCACTTTTGAA
GAAAAATTTGACGGTCAGGTTCGCGGCTATCACAAACCGATTATGATTGCCGGCGGCTTG
GGCAGCATTCAGGCGCAGCAGACGCATAAAGACGAAATCCCCGAAGGCGCATTGCTGATC
CAACTGGGCGGCCCGGGTATGCTTATCGGCTTGGGCGGCGGCGCGGCTTCTTCGATGGAT
ACCGGCACAAACGACGCGTCTTTGGACTTCAACTCCGTGCAACGCGGCAACCCCGAAATC
GAACGCCGCGCGCAGGAAGTCATCGACCGCTGCTGGCAGCTCGGCGGCAAAAACCCGATT
ATCTCCATCCACGACGTAGGCGCGGGCGGCCTGTCCAACGCCTTCCCCGAACTGGTCAAC
GATGCCAGACGCGGCGCAGTATTCAAGCTGCGCGAAGTGCCGCTTGAAGAACACGGCCTC
AACCCGCTGCAAATCTGGTGCAACGAATCGCAAGAGCGTTATGTGTTGTCGATTTTGGAA
AAAGATTTGGATGCTTTCCGCGCCATCTGCGAACGCGAACGCTGCCCGTTTGCCGTAGTC
GGCACGGCGACTGACGACGGTCATTTGAAAGTACGCGACGATTTGTTCGCCAACAATCCC
GTCGATTTGCCGTTGAACGTCTTGCTCGGCAAACTGCCCAAAACCACGCGCACCGACAAA
ACGGTTGCACCGTCCAAAAAAACCGTTTCACGCGGGCGATATCGACATTACCGAAGCCGCC
TACCGCGTTTTGCGCCTGCCTGCCGTAGCCGCCAAAAACTTCCTGATTACCATCGGCGAC
CGCAGCGTCGGCGGTTTGACGCACCGCGACCAAATGGTCGGCAAATATCAAACTCCAGTA
GCCGACTGCGCCGTTACCATGATGGGCTTCAACACCTATCGCGGTGAAGCGATGTCTATG
GGCGAAAAACCGACCGTCGCCCTGTTTGATGCGCCTGCTTCGGGCAGAATGTGCGTCGGC
GAAGCCATCACCAACATCGCGGCGGTCAACATCGGAGACATCGGCAACATCAAACTCTCC
GCCAACTGGATGGCGGCGTGCGGCAACGAAGGCGAAGACGAAAAACTCTACCGCACTGTC
GAAGCCGTTTCCAAAGCCTGTCAGGCATTGGATTTGAGCATCCCCGTGGGCAAAGACAGC
CTGTCGATGAAAACCGTTTGGCAGGACGGCGAGGAGAAAAAATCCGTGGTTTCACCGTTG
AGCCTGATTATCTCAGCGTTCGCGCCTGTGAAAGACGTACGCAAGACTGTTACGCCTGAG
TTGAAAAACGTCGAAGACAGCGTATTGTTGTTTGTCGATTTGGGCTTCGGCAAAGCGCGT
ATGGGCGGTTCGGCGTTTGGTCAGGTGTACAACAATATGAGCGGCGACGCGCCCGATTTG
GACGATACAGGTCGTCTGAAAGCCTTTTACAGTGTGATTCAGCAGCTTGTTGCCGAAAAC
AAACTCTTGGCGTATCACGACCGCAGCGACGGCGGCTTGTTTGCCGTTTTGGTAGAAATG
GCGTTTGCGGGGCGGTGCGGCTTGGATATAGATTTAAATTTATTGCTTGCACAAACATTT
ATTACCAACCATACCGCTCTGTCTCAATCATTGCGGACTGAAGAGGTAAAAGCGTTGGCT
GAATGGCAAGAAACCATTGCCCGCACATTATTTAATGAAGAGTTGGGTGCTGTTATCCAA
GTTAGAAAACAAGATGTTGCCGATATTATCAATTTATTCTATCAACAACAGCTGCATCAT
AATGTCTTTGAAATCGGTACGTTAACTGATGAGAACACGTTAATCATCCGCGACGGGCAA
ACGCACCTTATTTCTGACAACCTAATCAAACTGCAACAAACCTGGCAAGAAACCAGCCAT
CAAATCCAACGCCTGCGCGACAACCCTGCCTGCGCCGACAGCGAGTTCGCACTGATTGGC
GACAACGAACGCAGCGCATTGTTTGCCGACGTGAAGTTCGACGTGAACGAAGACATCGCC
GCGCCGTTTATCAACAGCGGCGCGAAACCCAAAATCGCCATCCTGCGCGAACAGGGCGTA
AACGGGCAAATCGAAATGGCCGCCGCCTTTACCCGCGCCGGATTCGATGCTTACGACGTG
CATATGTCCGACCTGATGGCAGGCCGCATCCACCTCGCCGACTTCAAAATGCTGGCGGCG
TGCGGCGGCTTCAGCTACGGCGACGTACTCGGCGCGGGCGAAGGCTGGGCGAAATCGATT

## Appendix A

CTGTTCCACCCTGCTCTGCGCGACCAGTTTGCCGCCTTCTTCGCCGACCCGGACACGCTG
ACATTGGGCGTGTGCAACGGCTGCCAAATGGTCAGCAACCTTGCCGAAATCATCCCCGGC
ACGGCAGGCTGGCCGAAGTTCAAACGCAACCTGAGCGAACAGTTTGAAGCACGCCTGAGC
ATGGTTCACGTTCCGAAATCAGCGTCGCTGATTCTGAACGAAATGCAAGGCTCCAGCCTG
CCTGTCGTGGTCAGCCACGGCGAAGGCCGCGCCGACTTCGCGCTTCACGGCGGCAATATT
TCCGCCGATTTGGGCATTGCGCTGCAATACATCGACGGACAAAACCAAGTGACCCAAACT
TATCCGCTCAACCCCAACGGCTCGCCTCAAGGCATCGCCGGCGTTACTAACGCCGACGGC
CGCATCACCATCATGATGCCCCACCCCGAACGCGTGTACCGTGCCGCGCAAATGAGCTGG
AAACCGGAAGGCTGGACGGAACTGTCCGGCTGGTACCGCCTCTTTGCCGGCGCACGTAAA
GCCTTGGGCTAACCGCCCTACTCAAACCAATGCCGTCTGAAGAATATTTCAGACGGCGTT
CCGGCATACCATCCTTTAAACGGTATCCGTCCACCGAGGAACACTCATGAAAATCACCCC
CGTCAAAGCCCTAACCGACAACTACATCTGGATGATACAGCACGGCAACCATGCCGTCTG
CGTCGACCCTTCCGAACCCTCGCCCGTCTTGGAATTCCTCGTCCGCAACCGCCTCATGCT
TGCCCAAACATGGGTAACTCACCCCCATCCCGACCACGAGGGCGGTGCGGCGGCACTCTG
GCGCGGCTACATGGAATCGCCCGTTTACGGCGAATCCGACATCGAAGCAGCAACCCACAC
CGTAACCGCCGGCACCCAATTCACCTTCGGCGACGGACAGGTTACCGTTTGGGCAACACC
CGGCCACACAGACCGCCACACCAGCTACCTTCTCGAAACTTCAGACGGCATACACGTCTT
TTGCGGCGACACCCTTTTTCCGCCGGCTGCGGACGCGTGTTTACCGGCACAATCGAACA
GCTTTACGACAGCTTCCAACGCTTCAACCGCCTGCCTGAAAACACCCTGTTCTATCCGGC
GCACGAATACACCGCCGCCAACCTGCGTTTCGCCGCCCATATCGAGCCGGACAACGCCGA
CATTCAGACGGCACTGAAAGCGGCGGCGCATACGCCTACCCTGCCCGTTACCCTCGCGCA
CGAACGCCGCGTCAATCCGTTTTTGCGCGTCGACCTGCCGCACGTCAGAGACCGCGCCGA
GGCATTGAGCGGGAAAACGTTAAACAGCAGCCTCGATACCTTTGTCGCGCTGCGTGAACT
TAAAAACCAATACCGGACGAAATAAAACAACGGGAAAACGCAGCCATTCCTAGGATTTTT
ATTAAAATCTTAAATAAAATCATACAATCATCGCCAATAGACGAAAGGACACCGTTGCCT
TATAATCAAACAAAAACAAAATATATAATATAGTGGATTGAATTTAAATCAGGACAAGGC
GACGAAGCCGCAGATAGTACGGCAAGGCGAGGCAACGCTGTACTGGTTTTTGTTAATCCA
CTATATTGTTAATCCACTATATAAATCCAGCACAAAACGGGATCGGTGATTCTTGTCCGC
AAGAATCGTTGATTTTCTCTATTACACGGATAATCATCATGCGCTTCACACACACCACCC
CATTTTGTTCCGTATTGTCCACCCTCGGTCTTTTTGCCGTTTCCCCTGCTTACTCATCCA
TTGTCCGCAACGATGTCGATTACCAATATTTTCGCGACTTTGCCGAAAATAAAGGCGCGT
TCACCGTAGGTGCAAGCAATATTTCCATCCAAGACAAGCAAGGCAAAATATTAGGCAGGG
TTCTCAACGGCATCCCCATGCCCGACTTCCGCGTCAGCAACCGCCAAACCGCCATCGCCA
CCCTGGTTCACCCCCAATACGTCAACAGTGTCAAACACAACGTCGGCTACGGTTCCATAC
AATTCGGCAACGACACCCAAAATCCAGAAGAACAAGCCTATACCTACCGCCTCGTATCAC
GCAACCCGCACCCGGACTACGACTACCACCTTCCCCGCCTCAACAAACTGGTTACCGAAA
TCTCACCTACCGCACTCAGCAGCGTACCCTTGCTTGGAAACGGCCAGCCAAAGGCCAATG
CCTACCTCGATACCGACCGCTTCCCCTACTTTGTACGACTCGGCTCAGGCACGCAACAAG
TCCGCAAAGCAGACGGCACGCGTACACGAACCGCCCCGGCATACCAATACCTGACCGGCG
GCACGCCGCTGAAAGTATTGGGGTTCCAAAACCACGGCTTACTCGTCGGCGGCAGCCTGA
CCGACCAACCCCTTAACACCTACGCAATCGCCGGAGACAGCGGTTCCCCCCTGTTTGCCT
TCGACAAGCATGAAAACCGCTGGGTGCTTGCGGGCGTACTCAGCACCTACGCCGGCTTCG
ATAATTTCTTCAACAAATACATCGTCACGCAACCCGAATTCATCCGTTCCACCATCCGCC
AATACGAAACCCGGCTGGATGTCGGGCTGACCACCAACGAACTCATATGGCGCGACAACG
GTAATGGCAACAGCACCCTGCAAGGGCTCAACGAACGCATCACCCTGCCCATTGCAAACC
CTTCGCTTGCCCCACAAAACGACAGCAGGCACATGCCGTCTGAAGATGCCGGCAAAACGC
TCATCCTATCCAGCAGGTTCGACAACAAAACACTGATGCTGGCAGACAATATCAACCAAG
GCGCAGGCGCATTGCAGTTCGACAGCAACTTCACCGTCGTCGGTAAAAACCACACATGGC
AAGGTGCAGGCGTTATCGTAGCCGACGGCAAACGCGTCTTCTGGCAAGTCAGCAACCCCA
AAGGCGACCGGCTCTCCAAACTGGGCGCAGGCACGCTTATCGCCAACGGACAAGGCATCA
ACCAGGGCGACATCAGCATCGGGGAAGGCACTGTCGTACTCGCCCAAAAAGCTGCTTCAG
ACGGCAGCAAACAAGCATTCAACCAAGTCGGCATCACCAGCGGCAGGGGCACGGCCGTCC
TCGCCGACAGCCAGCAAATCAAACCCGAAAACCTCTATTTCGGCTTCAGGGGCGGACGGC
TCGACCTCAACGGCAACAACCTTGCCTTTACCCATATCCGCCATGCGGACGGCGGCGCGC
AAATCGTCAATCACAACCCTGACCAAGCCGCGACACTGACGCTGACCGGCAACCCCGTCC
TCAGTCCCGAGCATGTCGAGTGGGTGCAATGGGGCAACCGTCCGCAAGGCAACGCGGCGG
TTTACGAATACATCAACCCGCACCGCAACCGTCGGACCGACTACTTCATACTCAAACCCG
GCGGCAACCCGCGCGAATTTTTCCCGTTAAATATGAAAAACTCAACAAGCTGGCAATTTA
TCGGCAACAACAGGCAACAGGCCGCCGAACAAGTCGCCCAAGCCGAAAATGCCCGCCCCG
ACCTGATTACCTTCGGCGGATACTTGGGTGAAAACGCGCAAACGGGCAAAGCCGCGCCGA
GTTACAGCAAAACCAATGAAGCAGCCATAGAAAAAACCCGCCATATCGCAAATGCCGCCG
TATACGGCCGGCCCGAATACCGTTACAACGGCGCACTCAACCTGCACTATCGTCCCAAAC
GCACCGACAGCACGCTGTTGCTCAACGGCGGCATGAACCTTAACGGGGAAGTCTTGATTG
AGGGCGGCAATATGATTGTGTCAGGCAGGCCCGTACCCCATGCCTACGACCACCAGGCCA
AACGCGAACCCGTTCTTGAAAACGAATGGACCGACGGCAGCTTCAAGGCTGCACGGTTCA
CCCTGCGAAACCATGCCCGACTGACGGCAGGGCGCAATACCGCGCATCTGGACGGCGACA
TAACCGCATACGATCTGTCCGGCATCGACCTCGGCTTTACCCAAGGCAAAACACCGGAAT
GCTACCGCTCCTACCATAGCGGCAGCACCCACTGCACACCCAACGCCGTTTTAAAAGCCG
AAAACTATCGTGCACTACCTGCAACGCAAGTACGCGGCGACATTACCCTTAACGACCGTT
CAGAGCTCCGCCTGGGCAAAGCACACCTGTACGGCAGCATCCGTGCCGGCAAAGACACCG
CAGTCCGCATGGAAGCAGACAGCAACTGGACACTTTCCCAGTCCAGCCACACCGGCGCAC
TGACGCTTGACGGCGCACAAATTACCCTGAACCCCGATTTCGCCAATAATACACACAACA
ACCGCTTCAACACACTGACCGTCAACGGCACACTTGACGGGTTCGGCACATTCCGATTCC
TGACCGGCATCGTCCGAAAACAAAATGCCCCCCCCCTCAAACTGGAAGGGGACAGCCGCG
GCGCATTCCAAATCCACGTCAAAAACACCGGACAAGAACCTCAAACAACCGAATCGCTTG

## Appendix A

CACTTGTGAGCCTCAATCCGAAACACAGCCACCAAGCCCGATTCACCCTCCAAAACGGCT
ATGCCGATTTGGGTGCCTACCGCTACATCCTCCGCAAAAACAACAACGGATACAGCCTGT
ACAACCCGCTCAAAGAGGCCGAACTTCAAATTGAAGCCACGCGTGCGGAACATGAGCGCA
ACCAACAGGCATACAACCAATTACAGGCAACCGACATCAGCAGACAGGTTCAACATGACT
CTGACGCGACCAGGCAGGCACTACAGGCCTGGCAGAACAGTCAAACCGAACTTGCCCGCA
TCGACAGCCAAGTCCAATATCTGTCCGCCCAATTGAAACAGACAGACCCGCTGACCGGCA
TTCTGACGCGTGCCCAAAACCTGTGTGCCGCACAAGGATACAGTGCCGATATCTGCCGTC
AGGTTGCCAAAGCCGCCGACACGAACGACCTGACACTCTTCGAAACCGAACTGGATACGT
ATATAGAACGTGTAGAAATGGCCGAATCCGAACTTGACAAAGCACGGCAAGGCGGCGATG
CGCAAGCCGTCGAAACAGCCCGGCACGCCTACCTGAACGCACTCAACCGTCTGTCCCGAC
AAATCCACAGTTTGAAAACCGGCGTTGCCGGCATCCGTATGCCGAACCTGGCCGAACTGA
TCAGCCGGTCGGCCAACACCGCCGTTTCCGAACAGGCCGCCTACAATACCGGCCGGCAAC
AGGCGGGACGCCGCATCGACCGCCACCTTACCGATCCGCAGCAGCAAAACATCTGGCTGG
AAACCGGTACGCAACAAACCGACTACCATAGCGGCACACACCGTCCCTACCAACAAACTA
CCAACTATGCACATATCGGCATCCAAACCGGCATCACCGACCGTCTCAGTGTCGGTACGA
TTTTAACCGATGAGCGCACAAACAACCGTTTTGATGAAGGCGTATCCGCCCGAAACCGCA
GCAACGGCGCACATCTGTTCGTCAAAGGGGAAAACGGCGCACTCTTTGCCGCGGCAGATT
TAGGCTACAGCAACAGCCGTACCCGATTTACCGATTATGACGGGGCTGCCGTCCGCCGCC
ACGCATGGGATGCAGGCATCAACACCGGCATCAAAATCGATACCGGCATCAACCTCAGAC
CCTATGCCGGCATCCGTATAAACCGCAGCAACGGCAACCGGTACGTACTCGACGGCGCAG
AGATAAACAGCCCGGCGCAAATCCAAACCACATGGCATGCCGGCATCCGTCTCGATAAAA
CCGTCGAACTGGGTCAAGCCAAGCTGACCCCCGCCTTCAGCAGCGATTACTACCATACCC
GCCAAAACAGCGGTTCCGCCCTCAGCGTCAACGACCGTACCTTACTGCAGCAAGCCGCCC
ACGGCACACTGCATACCCTGCAAATCGACGCCGGATACAAAGGCTGGAACGCCAAACTTC
ATGCCGCTTACGGCAAAGACAGCAACACCGCCCGCCACAAACAGGCAGGAATCAAAATAG
GCTACAACTGGTAACAAGCCGATAAAAATGCCGTCTGGAACCCGCGTTTCAGACGGCATT
TGCGTTAAAAATAGTAAACCGTTCCAAAAGGGAGTAGAATAGTGCCGTTTCCAACCCTGC
GCCTGTACCGTCAGGCTTTTATTATGGACCTTCCCAGTTCGTTTTTACTGAACACCCCAT
CCGATTCAAACCCGCAATAACCATCCCGCCGGAATGCCTCCCCGCACACGGCGGGCGGAG
CATTTATGAGCATCGAACCAACCCCTCCGAACCTTGAAAACGACGGTATCGAAAACGATG
TAGAACGCGTTTCCGCCGATTTCGACCGTGTCCACTCCCTCTGCGAAATCCTCGAACCTG
CTTTTGAACAAATCGAAAACGGTACACCGCTCGAAGACGCGCCGCTGCGCGACAAGCTGA
CCGAGCTGACCGTCCTCTTGAGCGAGCTGCACCCTGCCGACGTGGCGGCGGTATTGGAAT
CGCTACCGCCGCGCGAACGCAATATCGTCTGGATTCTGGTCAAACCGGAAGACGACGGCG
AAGTATTGCTGGAAGTATCCGACGCGGTGCGCGAAACGCTGATCGAGTCGATGGACAAAG
ACGAATTGTTGGCAGCGGTCGATGATTTGGACGCGGACGAATTGGCGGAACTGGCAGACG
ATTTGCCGCACCAAGTGGTTTACGAAGCGCTACAAACCCGCGATGAGGAAGAACGCGCCC
AAGTCAAAGCGGCAATGTCCTACGAAGACAACCAAGTCGGTGCGATTATGGACTTCGAGT
TGGTCAGCATCCGCGCCGATGTCGCCTGTGAAGTGGTGCTGCGCTATCTGCGCCGCTTCG
ACAGCCTGCCCGACCATACCGACAAGATTTTTGTGGTCGATGAAAACGACGTACTGCAGG
GCGTGCTGCCCATCCGCAAACTTTTGGTCGCCGATCCCGAAGACTTGGTGGAAAACGTGA
TGGCGAAAGATGTCGTGCGTTTCCGCGCCGAAGATGACGTGGAAGAAGCGGCGCAGGCGT
TTGAACGCTACGACTTGGTTACCGCGCCCGTCGTCGATGAAAACAAAAAGCTCATCGGCA
GGATTACCATCGACGAGATGGTGGACGTGATCCGCGAAGAATCGGAAGCGGATATGCTGA
ATATGGCGGGTTTGCAGGAAGAGGAAGACCTGTTCGCCCCCGTGTGGGATTCGGTGAAAA
ACCGCTGGATGTGGCTCGCCGTCAACCTCTGCACCGCCTTCCTCGCCAGCCGTGTTATCG
GCGCGTTTGAAGGCAGCATCGAAAAAATCGTCGCACTCGCCGCGCTGATGCCCATCGTCG
CCGGCATAGGCGGTAACTCGGGCAACCAGACGATTACCATGATTGTCCGCGCGATGGCGA
TGGGGCAGCTGACGGATATGCAGGCGGGGCGTTTGCTGAAAAAAGAAGTCGGTGTCGCCT
TGGTCAACGGCATCATTTGGGGAACGGTCATGGGCGCAGTATCTTGGCTGCTTTACGGCA
GCCTCGGCATCGGGCTGGTTATGATTGCCGCGATGACGCTCAACCTCCTGCTGGCGGCAA
CCGTCGGCGTATTAATTCCCGTGGTAATGGAAAAGTTCGGACGCGATCCCGCACTGGGCA
GCTCGGTGCTGATTACCGCCGTTACCGACTCCGGCGGCTTCCTGATTTTCTTGGGGCTCG
CCACCCTATTCCTGCTTTAAATGCCGTCTGAACCCGCGCAAAAATGCCGTCTGAAGCGGA
AGCTGCTTCAGACGGCATTTGACTATTTATCCTTGTTGCACAAGATTATTGGACGGTATG
CCGGGGCAGCCCTTTGGCAACGCCGACCACATCCTCCCCGAACAGCGCGTTGACATCGGT
TTCGTCAAACACATATTTGCTGTGGCAGAAATCGCAATCGACTTCGATGCTGCCTTGTTC
CACCACCACGCCGCCGACTTCTTCCCCGCCCAGCATCAACAGCATATCGCTGACTTTGCC
GCGCGAACAGGTGCATGAAAATTCAAACGTTTCCGGCTCGAACACGCGCGGCGGCGTTTC
GTGGAACAGGCGGTATAAAACGTGTTGCGCGTCCAGTCCTGCCAGCTCCTCCGCCGTCAG
CGTGCGCGCCAGCGTACTGACGTGTTCCCATGCCTCTTCATCCAATACCTCTTCAGGCAG
ACGCTGCACCAGCAGACCGCCCGCCGCTTCGTCGCTTGCAGACAGGACGATGTGCGTATC
AAGCTGTTCGGAACGTTTCATATAGTTCACCAACATTTGCGCGATACCGCCGCCTTCCAA
AGGCACTACGCCCTGCCAGGGTTCGCCGTCTTTGGGCTGCAGCGTCAGCACGAATACGCC
GCCCTCGCCCAAAAGGTCGCCGAGGCTTTCGTCATCGGCTATTTCTGCGGTTTCGTCCCA
ACGCGCGGTTGCACGGACGGTACGGTCGGAAGCCGCTTCCGCAACCAGCATTTTCAGCCG
CCCCCGCCCCTGAACCTGCACAATCAGCGTGCCTTCGTTTTTGAGGTTGCCCGACAGCAA
CACACCCGCCGCCAACAACTCACCCAAAGCGCGGCGGATGGCGGCGGGATAGTTTTTCTG
TTTTACAATGTGCTGCCACACGTTTTCCAGACGGACGTGCAGCCCGCGCACGGGCATATC
GTCGAAGATAAAGCGGGTACGCACATCGGCGCGGTTGATGGCGGTTTGATTCATGATTTT
CTCTGACTGATTGTTCGGATGGCGGCTATATGGTTGCGGTCGGCGCGAAAACAAGACGGA
CGGCGGATGCGCTTCCCAAATTATCAATAAATTATATAAAAATCAACATATTAACTCAAT
CTAACAAGCCGTTTTTTGCCAAACAGCCGTTTTTTTATATACAATCAACAAGATATTTTC
GACTGATACAGCATAACATCGCACGGCGGCACGATGCCTCCTGCGCGGAAACACCGATAT
GGATTCTTTTTTCAAACCGGCAGTTTGGGCGGTTTTGTGGCTGATGTTTGCCGTCCGCCC

# Appendix A

CGCCCTTGCCGACGAGTTGACCAACCTGCTCAGCAGCCGCGAGCAGATTCTCAGACAGTT
TGCCGAAGACGAACAGCCCGTTTTACCCATCAACCGAGCCCCCGCCCGGCGGGCGGGCAA
TGCCGACGAACTCATCGGCAGCGCGATGGGGCTTAACGAACAGCCCGTTTTACCCGTCAA
CCGAGTCCCCGCCCGGCGGGCGGGCAATGCCGACGAACTCATCGGCAACGCGATGGGGCT
TAACGAACAGCCCGTTTTACCCGTCAACCGAGCCCCCGCCCGGCGGGCGGGCAATGCCGA
CGAACTCATCGGCAACGCGATGGGACTTTTGGGTATTGCCTACCGCTACGGCGGCACATC
GGTTTCTACCGGTTTTGACTGCAGCGGCTTCATGCAGCACATCTTCAAACGCGCCATGGG
CATCAACCTGCCGCGCACGTCGGCAGAACAGGCACGGATGGGTACGCCGGTTGCCCGAAG
CGAATTGCAGCCCGGAGATATGGTGTTTTTCCGCACGCTCGGCGGCAGCCGCATTTCCCA
TGTCGGACTTTATATCGGCAACAACCGCTTCATCCACGCGCCGCGCACGGGGAAAAATAT
CGAAATCACCAGCCTGAGCCACAAATATTGGAGCGGCAAATACGCGTTCGCCCGCCGGGT
CAAGAAAAACGACCCGTCCCGCTTTCTGAACTGATTTCCCAAGGAATACGCAATGAGTAT
GCCCGAAATGCCCAAATGGTACGACGATGACGGACAGATCGTGTCCTGTACCGAAAAGGT
CAAAGTGATGTCCGAAAATATGGCCGAGCTGTATCAGACGGCACAAGACGCGTTTGAAGA
CGCGCTGCTGATGGGTTGCGGCGAACGTCAGTTGCGCGATTACCTGCTCGCGCTGATTGA
AGGTTTGGAAAATCCCTACCGCAAAGTCTGAACACGCCCCGGTTGCTGCGGCACGGTTTA
TCCGTGCCGTTTTTGCGTTTGTGCGCGGCTTCGGCTTTTCAGACGGCATATTTGACGTTA
TGATTAAACAGTTAACAAGATTTATCACAACGCCGTCAAAAGACAGACACACAACATGAA
CATCATCCGCGCGCTCCTCATCATCCTCGGCTGCCTCGCCACCGGCGAAACCGCCGTTTT
CCTAGCAGGCATCAAACTGCCCGGCAGCATCGTCGGCATGGGCGTGCTGTTTGCGCTTTT
GCAGGCGGGTTGGGTCAAAACGTCTTGGCTGCAACAGCTTACCGACGCGCTGATGTCGAA
CCTGACGCTGTTCCTCGTGCCGCCCTGCGTGGCGGTCATCAGCTATTTGGATTTGATTGC
CGACGATTGGTTTTCGATACTGGTTTCCGCCTCCGCCAGCACTTTGTGCGTACTGCTGGT
TACGGGCAAAGTCCACCGGTGGATACGGGGTATTATCCGATGAACGAAATCCTCAGGCAG
CCCAGCGTTCTGCTTTTCCTCACGCTTGCCGTGTACGCGCTTGCGATTATCGTGCGCACG
CGCACGGGCAATATCTTCTGCAACCCCGTACTCGTCAGCACTATCGTGCTGATTGCCTAC
CTGAAAATCCTCGGTATCGATTATGCGGTGTACCACAACGCCGCGCAATTCATTGATTTT
TGGCTGAAACCCGCCGTCGTCGTGCTTGCCGTGCCGCTCTACCAAAACCGCCGTAAAATC
TTCAACCAGTGGCTGCCCGTCATCGTTTCACAGCTTGCGGGCAGCGTTACGGGCATTGTT
ACAGGGATGTATTTTGCCAAATGGCTGGGCGCGGAACGCGAAGTCGTCCTCTCGCTCGCG
TCCAAATCTGTTACCAACCCCATCGCTATTGAAATCACCCGCTCCATCGGCGGCATTCCC
GCCATTACCGCCGCCACCGTCATCATTGCCGGTCTGGTCGGACAGATTGCCGGTTACAAA
ATGCTGAAGAACACGGTCGTCATGCCCTCGTCCGTGGGTATGTCGCTCGGCACGGCTTCG
CACGCGATGGGGATTGCCGCCTCGCTCGAACGCAGCCGCCGTATGGCGGCATACGCGGGG
CTGGGGCTGACGTTCAACGGCGTACTGACCGCGCTGATTGCGCCGCTGCTCATCCCCGTT
TTGGGATTTTGAACCCGTTTCAGACGGCATTTCAGCCCATGCTGTCTGAACGCCGACACA
CTCGCAAGGAGAACCGTTATGGCTGTCAACCTGACCGAAAAAACCGCCGAACAACTGCCC
GACATCGACGGCATTGCCCTCTACACCGCCCAAGCAGGCGTGAAGAAGCCCGGGCATACC
GACCTGACACTGATTGCCGTAGCCGCCGGCAGCACCGTCGGTGCAGTCTTCACGACCAAC
CGTTTCTGTGCCGCGCCCGTCCACATCGCCAAATCGCACCTTTTCGACGAAGACGGCGTG
CGCGCCCTCGTCATCAACACGGGCAACGCCAACGCGGGTACGGGCGCACAGGGCAGAATC
GATGCTTTGGCAGTGTGTGCCGCCGCCGCCCGGCAAATCGGCTGCAAACCGAACCAGGTG
CTGCCCTTCTCCACCGGCGTGATTCTCGAACCGCTGCCCGCAGACAAAATCATCGCCGCC
CTGCCCAAAATGCAGCCTGCCTTCTGGAACGAAGCGGCACGCGCCATCATGACCACCGAC
ACCGTGCCCAAAGCCGCCTCGCGCGAAGGCAAGGTCGGCGACAAACACACCGTCCGCGCC
ACGGGCATCGCCAAAGGCTCGGGCATGATTCATCCCAATATGGCGACCATGCTCGGTTTC
ATCGCCACCGATGCCAAAGTTTCCCAACCCGTCCTCCAACTGATGACGCAGGAAATCGCC
GACGAAACCTTCAACACCATCACCGTTGACGGCGACACCAGCACCAACGACAGCTTCGTC
ATCATCGCCACCGGCAAAAACAGCCAAAGCGAAATCGACAACATCGCCGACCCGCGTTAC
GCCCAACTCAAAGAATTGTTGTGCAGCCTCGCGCTCGAACTCGCCCAAGCCATCGTCCGC
GACGGCGAAGGTGCGACCAAGTTCATCACCGTCCGCGTCGAAAACGCCAAAACCCGCGAC
GAAGCCCGCCAAGCCGCCTACGCCGTGGCACGTTCGCCGCTGGTCAAAACCGCCTTTTTC
GCCTCCGACCCCAACCTCGGCAGGCTGCTCGCCGCCATCGGTTATGCCGGCGTTGCCGAC
CTCGATACCGACCTCGTGGAAATGTATCTCGACGATATTTTGGTTGCCGAACACGGCGGA
CGCGCCGCAAGCTACACCGAAGCACAAGGGCAGGCGGTGATGTCGAAGGCCGAAATCACC
GTCCGCATCAAGCTGCATCGCGGACAAGCCGCCGCCACCGTCTATACCTGCGACCTGTCG
CACGGATACGTTTCCATCAACGCCGATTACCGTTCCTGACCCGACACGGCTTCAGACGGC
ATACATAAAATGCCGTCTGAACCGCCGGACAACATACCATGACCTCCACATTCCCCCGCC
GCCTCGCCCGCAAAATCCGCCAAACCCGCCGCCTGTCGCGCAAAAGCATCGCCTTTCTGT
TCCTTTTGGCAGGTTCGGCACTCGTCGCCCTGACCGCGCTGTTTTTTGCCCATCTTGCCG
ATTTTGCGCTGGAACTGAACGCCAAACTGGTTCAACAATACCCGTGGTTCGCGTGGGTCG
CGCTTCCTTTGGGTTTACCGCTTATTGCGTGGCTCACACGCAAATTCGCCCCCTTCACCG
CCGGCAGCGGCATCCCGCAGGTCATCGCCTCACTGTCGCTGCCCTACGGCGCACAGAAAA
CGCGGCTGATCCGCCTCGGGCAGACGCTGCTGAAGATTCCGCTAACCTTTTTGGGTATGC
TGTTCGGCGCGTCCATCGGACGCGAAGGTCCGTCCGTGCAGGTCGGCGCGGCAGTGATGG
GCGCGTGGGGCGCGTGGTGCAAGAAACACGGCTTGGCATTCAAAGGGATGCAGGAAAACG
ATTTGATGGCGGCGGGCGCGGCGGGCGGTTTGGCAGCCGCGTTCAACGCGCCGCTGGCGG
GCGTGATTTTCGCCATTGAGGAACTCGGGCGCGGCATCATGTTGCGCTGGGAGAGGCAAA
TTCTTTTGGGCGTGCTCGCCTCCGGTTTCATACAGGTCGCCATTCAGGGCAACAACCCGT
ATTTTTCCGGCTTCAACGGCGGCGTATTGGAACATATCTTTCTGTGGGTCGCACTGTCCG
GCCTGGTTTGCGGCGCGGCGGGCGGGCTGTTCGGACGTTTGCTCTATCGCGGTGCGGCGG
CGTTTGCACCGCGCAAGATACGCGGCTTCATCCGCAACCGTCCGCTGCTGCTGGCGGCAC
TGATGGGGCTGCTGCTCGCCCTGCTCGGCACGTTCTACCAAGGCAAAACCTACGGCACCG
GCTACCACGAAGCCGCCCAAGCCCTGCACGGCATCTACGAAGCCCCCTTCGGACTCGCCG
CCGCCAAATGGCTCGCCACCGTATTCAGCTATTGGGCAGGCGTTCCGGGCGGCATTTTCA

## Appendix A

CTCCCTCGCTGACCATAGGCGCGGTTTTGGGCGAGCATATCGCCGCCATCGCCGACATAT
CGCAGGGTGCAAACATCATCGTCCTCATCTGCATGGCGGCATTTCTGGCGGGCGCGACAC
AATCCCCGATTACTTCCGCCGTCGTCGTCATGGAAATGACGGGCGGACAAAGCCTGCTGT
TTTGGATGCTAATTGCCTGCATTTTCGCCTCGCAGGTTTCGCGCCAGTTTTCGCCGCGTC
CGTTCTACCACGCATCGGGAATGCGCTTCCGCCAGCGCGTGCTTCAAGAAACCGCCGCCC
AAACCGGCAATGCGCCCGCAAGACCGCAAACAGCAAACAGCAAAACGGGAATGCCGTCTG
AAAATTAAAACGCCCCCGATCAAACGCCGGCAGCCGCCTTGATTTGAATACCGTTCCGCC
GCCGCTTGAAATTTCAGCAACAATGCCGTCTGAACGACAGAATGCGGTTTTCAGACGGCA
TTTCCCCATCCCGATATTGCCTAAACAAAACCGAAGCGTTTGCTATAATTCTATTTTTTA
CCGCATACGCACCAATCATGTTTCCCGATTTCTCCCAAACCCTCTCCAAAGACCGCCACT
TCCTGCGTTCCGCCTTCAAAAATCCCAACAAATACGGCGGTTTGTCCAAAATCGAAGAAA
AATACCGAAAATCGCACGAAATCTTTTTGAAGCGTTTGGCAGCCTTGCCAAAACCCGAAT
TCGACAACACCCTGCCCGTTCACGAGAAGCTCGAAGAAATCAAAAAAGCCATTGCCAAGA
ATCAGGTAACGATTATTTGCGGCGAAACCGGTTCGGGCAAAACCACGCAGTTGCCCAAGA
TTTGCTTGGAACTCGGGCGTGGGGCGGCAGGATTGATCGGGCATACCCAGCCGCGCCGTT
TGGCCGCGCGCTCCGTAGCAGAGCGGATTGCCGAAGAGCTGAAATCCGAAATCGGCAGCG
CGGTCGGCTATAAAGTACGCTTCACCGACCACACCTCGCGCGATGCCTGCGTCAAGCTGA
TGACCGACGGCATCCTGCTGGCGGAAACGCAGACCGACCGTTATCTCGCCGCCTACGACA
CGATTATCATCGACGAAGCGCACGAGCGCAGCCTGAACATCGACTTCCTTTTGGGCTATT
TGAAACAACTCCTGCCGCGCCGCCCCGATTTGAAAGTCATCATCACCTCGGCAACGATAG
ACGCAGAACGCTTCTCCCGACACTTCAACGGCGCGCCCGTTTTAGAAGTGAGCGGACGGA
CGTATCCCGTCGAAATCCTCTACCGACCGCTGACCGGCAAAGACGAAGACGACGCAGAAG
TGGAGTTGACCGACGCGATTGTCGATGCGGCGGACGAATTAGCGCGACACGGCGAAGGCG
ATATTTTGGTATTCCTGCCGGGCGAGCGCGAAATCCGCGAAACTGCCGAAGCCCTGCGCA
AATCCACGCTGCGCCGCAACGACGAAATCCTGCCCCTGTTCGCACGCCTGTCGCACGCCG
AGCAGCACAAAATCTTCCACCCCTCAGGCGCGAAACGCCGCATCGTATTGGCAACCAACG
TCGCCGAAACCTCGCTTACCGTGCCGGGCATCAAATACGTCATCGACACCGGCCTCGCGC
GTGTTAAACGCTATTCCGCACGGGCGAAAGTGGAGCAGCTTCATATCGAAAAAATCTCCC
AAGCCGCCGCCCGCCAACGATCCGGCCGCTGCGGACGCGTCTCCGCAGGCGTGTGTATCC
GACTGTTTTCAGAAGAAGATTTTAACAGCCGCCCCGAATTTACCGACCCCGAAATCGTCC
GCAGCAACCTCGCCGCCGTCATCCTGCGCATGGCAGCATTGAAACTCGGCGATGTGGCGG
CATTCCCGTTTTTAGAAATGCCCGATTCACGGTATATCAATGACGGTTTTCAGGTGTTGT
TGGAGTTGGGGGCGGTGGAGGCCGTCTGAAAACAGGCAGACATAAAAGAAAATCCGCGTA
GAGTGATGTAAACTTACCCTTGCTTTAATAAGTAGAAAATGGTGGGTTTACGTCCCCCCC
TGCGGCTACTAAAAAAATATAAGAGTAAACAACCTTTTTGAAAGAAAAATGTATGGACGA
AATTCAAATACCCAAAAAAGTGGAATTACAAACCAAACTAGAAAATGAAAAGATTGTTTT
ATCGAAAGGTTCTACCACGATTATTGTTGGTGCTAATGGCACAGGGAAAACAAGATTAGC
TGTTTATATTGAAGAACAATTAAAGGAAAAAGCACACAGAATTTCGGCTCATAGAGCATT
AAAATTAAACCCTAATGTCAATAAAATACCAGAAAAGAGTGCCAAAACATATCTATCTTA
TGGTCAGAACTGGGATGGAATCGATGTATCAAATAGAAAAAATTATAGATGGGATAATAA
CTCATATACTCATTTACTCAACGATTTTGATTGGTTATTACAATATTTATTCGCTCAACA
AAATAATATTGCGGTAGCAAATAATCAAAAGCTCAACCGTAATGAAAAAGTAACCAATTC
AAAAACAAAGCTAGATATTTTGCAAGAAGCATGGGAAACATTATTACCACACAGAAAATT
ACATATTACAGCAGATGATATTCAAGTCTCTGCTGTAGATAATGAGGAATTGTATTCTGC
CTCAAATATGAGTGATGGAGAGCGAGCACTTTTCTATATTCTTGGACAAGTTTTGTCAGT
AGATGACGGTTCTGTCTTAATTTTTGATGAGCCTGAATTACATATTCATAAATCAATTAT
TTCAAATCTATGGGATAAAATTGAAGAATTACGACCTGATTGTTCATTTCTAATCATTAC
ACACGATATTGAATTTGCTGCAACTCGAGTAGCTAAAAAATATGTTATCAGAAATTATTA
TCCGACCCCTGCTTGGGATATTTCTGAAGTTCCTGAAAGTAATTTTGATGAAGAAACAAT
~~AACGATGATTTTTAGGTAGCCGTAAGCCAATATTATTTGTTGAGGGCAACAATAATAGTTT~~
AGATATTGCTACTTACCGCTATTGTTATCCTGATTGGACCATCATACCCAAAGGGGCATG
CAAAGATGTCATTCAATCAGTATCATCGCTGAAAAAATTAAGTAATGAAATGCCATTACT
AAACTTAAAATGTTCAGGTATTGTCGATTTAGATAGTAGGGATGAAAGAGAAATTGAACA
ATTAAATAATTTGGGTATTTACATTTTACCTGTATCCGAAATTGAAAATCTTTTTAGCTT
AACTGATGTAGCAAAAGAGATATTGAAACTAAATCAATATTCAGATGAAGAATTACTCAA
TAAACTTAATGGATTTAAATCCGAACTAATTAAATATATAGATAATGAATTAAAAGACGA
TAAATTAGACGAATTTGTTGTAAAACAGGTTCGACGTAAAATTGATAATTATTTAAAAAA
TATTGATTTATCCTCCAAAATAACAAGTACTGATATGAAAAAATCATTACTTAATGAAAT
TTCTACTTTAACAGAACAGAAAATTGAAACATGGATTTCAGAAATTAAAAATGAAATTCA
AAGATGTATTGAACAGCAAGATTTGGATAAATTACTTACTATATATGATAATAAAGGACT
CTTGGCTAAATCAGCTTGTGTTTTAAAAGGAATGCGTAACAAACATGAATTTGAAAGCTG
GATAATGAGAACATTAAAAGGAAGGAATAAAGATTTTATTGATGCAATCAGACAGAAACT
TCCAATTCTGGATTAAATAAAACCATCTGAAAATTTACCTTCAGATACAGATATATTTCA
TGAAAAATCATCAAACTACACTCTCTTTCCCTACTTCGAGTAGCCTGAAACCTTGCGCAG
ACAAACAAGGCCTGTCTGAAGACCGCAGCCAATACCGCCTGACCAAACTCGGCGAACAAA
TGGCGCACCTGCCTATCGACCCGAAAATTGCGCGTATTTTGTTAGTATTATTCCGTTTTT
AAAAATGCCCGATTCGCGGTATATCAATGACGGTTTTCAGGTATTGCTGGAATTGGGGGC
GGTGGAGGCCGTCTGAAAATAAAATCTTTCTTTATAAAAAGGCAGGCCATGTTTCATTTT
CAGACGGCCTAAATCATTGAGAAACTAAAAACTATTAAAAAGGGAATATTGGGTTTTAAA
ACTCAATCGGTAAATTTTTATTGTGAAATATTAATGATGAAAAAATCTTTCCTTACGCTT
GTTCTGTATTCGTCTTTACTTACCGCCAGCGAAATTGCCTATCGCTTTGTATTTGGGATT
GAAACCTTACCGGCGGCAAAAATTGCGGAAACGTTTGCGCTGACATTTGTGATTGCTGCG
CTGTATCTGTTTGCGCGTTATAAGGTGACGCGTTTGTTGATTGCGGTGTTTTTTGCGTTC
AGCATTATTGCCAACAATGTGCATTACGCGGTTTATCAAAGCTGGATGACGGGCATCAAT
TATTGGCTGATGCTGAAAGAGGTTACCGAAGTCGGCAGCGCGGGTGCGTCGATGTTGGAT

## Appendix A

AAGTTGTGGCTGCCTGTGTTGTGGGGCGTGTTGGAAGTCATGTTGTTTTGCAGCCTTGCC
AAGTTCCGCCGTAAGACGCATTTTTCTGCCGATATACTGTTTGCCTTCCTAATGCTGATG
ATTTTCGTGCGTTCGTTCGACACGAAACAAGAGCACGGTATTTCGCCCAAACCGACATAC
AGCCGCATCAAAGCCAATTATTTCAGCTTCGGTTATTTTGTCGGACGCGTGTTGCCGTAT
CAGTTGTTTGATTTAAGCAGGATTCCCGCCTTTAAGCAGCCTGCTCCAAGCAAAATCGGG
CAGGGCAGTGTTCAAAATATCGTCCTGATTATGGGCGAAAGCGAAAGCGCGGCGCATTTG
AAGCTGTTTGGCTACGGACGCGAAACTTCGCCGTTTTTAACCCGGCTGTCGCAAGCCGAT
TTTAAGCCGATTGTGAAACAAAGTTATTCCGCAGGCTTTATGACTGCAGTGTCCCTGCCC
AGTTTTTCAATGCGATACCGCACGCCAACGGCTTGGAACAAATCAGCGGCGGCGATACC
AATATGTTCCGCCTCGCCAAAGAGCAGGGCTATGAAACGTATTTTTACAGCGCGCAGGCG
GAAAACGAGATGGCGATTTTGAACTTAATCGGTAAGAAATGGATAGACCATCTGATTCAG
CCGACGCAACTTGGCTACGGCAACGGCGACAATATGCCCGATGAGAAGCTGCTGCCGTTG
TTCGACAAAATCAATTTGCAGCAGGGCAAGCATTTTATCGTGTTGCACCAACGCGGTTCG
CACGCCCCATACGGCGCATTGTTGCAGCCTCAAGATAAAGTATTCGGCGAAGCCGATATT
GTGGATAAGTACGACAACACCATCCACAAAACCGACCAAATGATTCAAACCGTATTCGAG
CAGCTGCAAAAGCAGCCTGACGGCAACTGGCTGTTTGCCTATACCTCCGATCATGGCCAG
TATGTTCGCCAAGATATCTACAATCAAGGCACGGTGCAGCCCGACAGCTATCTCGTGCCG
CTAGTGTTGTACAGCCCGGATAAGGCCGTGCAACAGGCTGCCAACCAGGCTTTTGCGCCT
TGCGAGATTGCCTTCCATCAGCAGCTTTCAACGTTCCTGATTCACACGTTGGGCTACGAT
ATGCCGGTTTCAGGTTGTCGCGAAGGCTCGGTAACGGGCAACCTGATTACGGGTGATGCA
GGCAGCTTGAACATTCGCGACGGCAAGGCGGAATATGTTTATCCGCAATGAGTGGCGTAA
AAACCAATAAAGACAAATTTAGATGATGTCGGGGAAGATGCCCGACCGACAAGACTATGC
AAAATATGAAAAACCAAGTACGCGGATCAGGCATGGATGCCCGATCCAATCCGGCCAATG
TTTCAGACGGCCTGCAAAACAGTTCGGGTCATATCGGTACCAACACGCGTTACCGCCTGA
CCAAACTCGGCGAACAGATAGCGCGCCTACCCATCGACCCGAAAATCGCGCGCATTTTGC
TGGCGGCGAAGAAACACGACTGCATGGCGGAAATATTGGTGATTGCGTCCGCGCTGTCGA
TTCAAGACCCGCGCGAGCGGCCGCTAGAAGCGCGCGATGCCTCAGCCAAGGCGCACGAGC
GTTTTACCGACAAGCAGTCCGATTTCCTTGCCTATCTGAACATTTGGGACAGCTTCCAGC
GCGAACGCGATAAAGGCTTGTCCAACAAGCAGCTGGTGCAGTGGTGCCGCCAATATTTCC
TGTCGCACCTGCGGATGCGCGAGTGGCGCGAGCTGCACCACCAGCTTGCCCAAACCGCGA
TTGAAATGGGTTTAACCACCAAGGAAGCCGCTTTCAGACGACCTCCCGAAGTCAGGCAGC
TCACGTCGTCTGAAAATGCGGGTGACCAAGACCTATCTGCTAAACTCAAACAAAAACAAC
TGGATAAAAAGCAACACCGCGCCCAAATCCGCGCCGCCAAAGAAGCGGGCTACGAACAAA
TCCACCGCGCCCTGCTCACTGGCCTTATCGCCAACGTCGGCATGAAATCGCCCGACGGTA
ACGACTACACCGGCGCGCGCGGCAGCCGCTTCCACCTTTTCCCCGCCTCCGCCCTGTTCA
AAGCCAAACCCAAATGGGTGATGGCGGCAGAATTGGTTGAAACCACGCGCCTTTACGCGC
GCGACGTCGCCGTTATCCAGCCCGAATGGATAGAGCAGGAAGCGCCGCACCTCGTCCGCT
ATCATTATTTCGAGCCGCATTGGGAACAAAAACGCGGCGAAGTCGTCGCCAGCGAACGCG
TGACGCTTTACGGTCTGACCGTATTGCCGCGCCGCCCCGTGTCTTACGGCAAAGTTGCCC
CCGAAGAAGCGCGCGAAATCTTTATCCGCAGCGCGTTGGTGGCGCAGGAATGCGATTTGA
AAGCGGATTTTTTTGTCCACAACAAAAAGCTGATTAAAGAAATTACCGAACTCGAACACA
AATCGCGCAAGCAAGACGTGCTGGTCGATGACGAAGCCCTGTTTGCGTTTTATAACGAAC
GACTGCCCGAAATGGCTTGGAAAGACGCGCAAGGCAGCGTTTGGGGAAGTGAAGATTCCG
TACGGATTATTGAATCTGACAAAGCCGAGAGGTCGTCTGAAAATGAGCGCAACGAGTTTC
GTAAAAACAAGCGTAATGGGTCTCGCCAAAATGAAAATCACGGCAACACCGTAGGTTGGG
TTGAAAACCCAACATCAGCCGCAACTGCAAAAACTGTTGGGTTTGACAATCCAACCTACG
CTGCCCAACAAACCACCCCCTCCCCCGTGGGGAGGGTCGGGGAGAGGGCAAAACAGTTG
CCGCACAAACCAACTTTTCCGCAACCGCAGCAAACCCTCTCCCTAACCCTCTCCCGCAGG
AGAGGGAACAGAGTGCCGCAGCTTCAACGATTTCAGACGACCTGCGTCCTGCAAATCTGC
AGCAAACCGCCCCCTCCCCCGTGGGGGAGGGCTGGGGAGAGGGCAAAACAGTTGCCACAC
AAACCAACTTTTCCGCAACCTCAACAAACCCTCTCCCGCAGGAGAGGGAACAGAGTGCCT
CAGCTTCAACGTTTTCAGACGACCTGCGTCCTGCAAATCTGCAGCAACCCTCTCCCTCCC
CCGTGGGGGAGGGCTGGGGAGAGGGCAAAACAGTTGCCACACAAACCAACTTTTCCGCAA
CCTCAACACTTTCAGACGACTCCAAACCCAAAAAGCAGCCTGCACCCCAAAAAAACCGTC
TGAAACCCCTACCCCTCGCCGACATCCGCACCTTCCAAGCCTGGCTCAAAACCGCCGAGC
GCGACAATCCGCGCCTGCTGTTCCTCAGCCGCGACGATCTGATGCAACACGCCGCCGCAC
ACATTACCGAAGAACAGTTCCCCAAATTCTGGCAAACCGCAGACGGCAAATTCAAACTTT
CCTACCGCTTCGAGCCGCACCATCCGCTAGACGGCGTGACCATGACCGTGCCGCTGACCG
TCCTCAACCGCCTGCACGCGCCGTCGCTCGAATGGCTGGTGCCCGGCATGATACGCGAAA
AAATCCAGTTGCAAATCAAAGCACTGCCCAAGCAAATCCGCCGCATCTGCGTGCCCGTGC
CCGAATTCATCACCCAATTTTTAAGCCAAAACCCCGACCGCAACGCCCCCATCCTGCCCC
AACTCGCCCAAGCCATCGCCAAAACCGCAGGCGACATCCGCATATTCGAGCAAATCAACC
AAGACGAATGGGCCGCGTTCAGGCTGCCCGAACACTGCTATTTCAACCTCCGCATTATCG
ACGACGGCGGACAAGAGCTTGCCGGCGGCCGCAAACTGCACGAATTGCAACAACAACTCG
GTCAAGCTGCCGCCGTTACCTTCCGTGACAACACCCAAGAATTTGAGCGCGACAACGTCA
CCGCATGGGACATCGGCACCCTGCCCGAATCCATCAAATTCGCACGCGGCAAACAACAGC
TCACCGGCTATCTCGGCCTACAAAAAGAAAAAGACGGCCGCATCGCCCTGCGCCTGTTTG
ATACCACAGAAGCCGCAGAGCAGGCACACCGTCAAGGTGTCATCGAATTGATGAAGCTGC
AATTAAAAGAGCAGGTAAAGGATTTGAACAAAGGCATCCAAGGCTTCACCCAAGCTGCCA
TGCTGCTCAAACACATCAACGCCGACACTCTGCGCGACGACCTCACCCAAGCCGTCTGCG
ACCGCGCCTTTATCGGCGAAGACGAGCTGCCGCGCAACGAAAAAGCCTTCAAAGAACAAA
TCAAACGCGCCCGCAGCCGCCTGCCCGCCGTCAAAGAAGCCCTCAGCCGCTACCTGCAGG
AAACCGCCGCCGTCTACGCCGAACTCAACAGCAAACTCGGCAAACACCCATTGACCCACC
TTCTAAGACTACGCCTGCAAACCCTGCTCGCCGCCGGCTTCGCCACCCGAACCCCGTGGG
CACAATGGCCGCGCCTCCCCATCTACCTCAAAGCCATGACCCTGCGCCTCGAAAAATACA

## Appendix A

GCAGCAACCCCGCCCGCGACGCAGCCCGCGAAGCCGATATCCAAGAGCTGGAACAAATGT
GGCAGGAAAAAACAGACAGCCTGATTAAACAAGGTCTCCCCATTTCAGACGGCCTCGCCG
CGTTTAAATGGATGATTGAAGAATTGAGGGTGTCGCTGTTCGCGCAGGAATTGAAGACAC
CGTATCCGGTGTCGGTGAAGCGGCTGTTGAAAGAGTGGGAAAAAATTGAAAAATAAAAAA
ACAGCCTGAAAAGTTTCAGGCTGTTTTTTATTTGACTAATCGAAGTTTCCTATATCTAT
TTAAGTCCCTCTCAACTAATCCAAAAGTTAAATCAGCAACATCTTTGGGGGATACGTTTA
AATTTTCAGCAATCTGTTCAATACCAATGCCATCATTTTTTAAAATAGTAAGCATTTTAC
GTAATGCGCTTGATATTTCCCTTTCCATTGGCTCTGGTTCGATAGTTCGATATTTTTTCT
TTGCAAACAAAGGACAAAGATTGTGTATATACATCCTATCAGTAATCATTCCTAATTTAT
GCATCCGATATGCTAAGGCAACAAGTGATACACCAAATCGTCTTTTGATTTTTAATAAAT
TTTCAATAGTGATAGGAACATGACGATATAAGCGTAGTGCAGCCTCCGGCATTAAAAAAG
CTGAAGCAAAGGCATTAGCCTCTTTTTCGATAATATCACGAGGTTCATCTTCTGTAATTT
CACTATTTTTACTATGTTCCATACTGTATTTATCACGGATTAAGTGCCCTAATTCATGGG
CAGCATCAAATCGACTACGTTCTGCAGATTTTTGTGTATTTAAAAATACAAATGGATGAT
TTTCATACCAAGTACAAAAGGCATCAATGTCCTTTGTATCTAAAGATAATGAAAATACAC
GAACACCCTTAACTTCAAGTAGGGTGATCATATTCGGAATAGGTTCATTGCCAAGCCCCC
ATTCTAATCTTAGTTCCTGAGCAGCCTCTTCAGGAGAAATATCAGAAAAATCAGGCAATA
CGGCTTGACTTAGTGTAAATTCTGTCTCGAGCCAGTCATTTAACAAAAAAGCCGTAATGC
TATGATTTAATGCTTGTTTTTCAAGCCTCTTCGAGGTGCGTGAACGAGCACGAAAACTTA
CTGCCTGAGATTTCAACTCAGGCAGTCTTTCGTCATTAGTAAAGAAATGAACTGGAAACT
CTAATAAATTGGCTAATTCATTTAAATCAGGTATTTGCTCATCTTTTACATAGTTTCTAA
CCTGTCGAGCGGTAATACCTAATAACTCAGCTAATTTTGTTTGCGTACAACCACGTTTAT
CCAGCGCAAATTCCAGTCTCTCACGATTAAATGTCTGCATGATTTATCATTCAAATTATC
CTGCTTTTTGTTTTCTTTCAACCAAAGGCTCATATTCTTCAACAGGTTGCTTACGTTCAA
GCTCATCAAATTTAGTTAAATCAACATCAGCTAATATAATTCGCTGCTTGTACCCAGTTA
TTTGATGACTAACAAAACCACTCGGTAAAGATAATTCAAGTTGCACTTTATTATACTTCC
AGTGAAACAGCAGAACCCAAAACTGCACAGTATCAGGCAAATCTAGTTTTGAATTACGAA
TAGCCTCCTCAAATCCCTTACCTTTCCTTGCGGTTGTCATTGGCATCCCGTGATGCCTAC
CAACATCTGAAGTAGCAGTAGCCACAATAATACTTTTAGTTCGACATGGCGAGAGACATA
GAAATGCACCACCCGACCAAGGCTCAAGCGTCCAGCCATCTTTACTTAAATATACCCTTA
GAGCAAATGTAATTTCTGCTTGCCGATACATCCCCAATGTATTTCTGTCAGATAATGCTG
CTTTGTCCTGAATATTATTATGCGCAGTAAGTACAATCTCTTTAAGCATCTCCTGAGATA
AGTACTTGCTGATTTCACTTAAAGCAATATCACTATTTTGTTGCTCGACTATTTCTCCTA
CTTCAAATGGGAAAGGTTCTGATAATGCAAATTCCACCATAAAAATTTCCTAATTTTATA
CGTAATGTTTACACAATATATCAGGAAATATGAAAACGTACAACTATATCTATAAAGCAA
TTAATAAGTAGCCTGCCCAACCGTGTCCTTATCTTTCGGCACACCCGACCTGCAAATCAC
GCAAAACTTGGAATCCGTGTGTAGGGTGTGTGCGGTACATACGCACGCAGTCTTTTTAAA
CCACAGCCCTTCCCAACTAAACCAAAAGGTCGTCTGAACCCTATTTTCAGACGACCTTTT
GCCACTTTGTAAAACAAATCTTCCCACCATCCTCTCCCCAAACATCGCCCGAACCAGTAA
ACTTCTCATATTTCAACAACTCCTTGGAAGCAAACCATGTCTGGTATCTACCTACCCCGC
CTATTCCCGCCCCATATCGCCGAACGCGGCCTGTTGTATTTTCAGCAGGGCAAGGTTCTC
GATGTCCGAAAAACTTCCGCCGGGCATTATCGGGCGGAGGTGTGCGGTTCGGAAAACTAT
TGGGTATAGTTGAAGCTGGATAGTGATTTGTATATTAAAGACGAAGGCTGCAATTGTCCT
TATATCTAAGAGTGCAAACATACCTTAAATTACTATATTGCATAGGCAAAATACAAGCCT
ATAACGAATTGGAAACAAAATGCCGTCTGAAAACATCTTCAGACGGCATTATAAAATCTG
TTCACCTTTTCAGATGAGTAATGTACACCCTTATACAATTTTTGCTACTATGCCCCATAA
ATCCACGGCTAAAGATATCCTTATTATGTCCTATGATTTATCGAAACGACTTGTAATCGG
CTTAGCATCAAGTGCCCTATTCGACTTATCCGAATCGGATAATATATTTAGAATGGAAGG
GGCAGAAACCTATAGGCAATATCAGAGAGAAAAACAAAACCATCCCCTAAAAAAGGCGTT
GTCTTTCCATTTATTAAAAAACTTCTGTCAATCAATGAAATAAACCCAAACGACCCAACG
ATTGGGTTTATTCTTTTATCCAGAAACAATCCAGATACAGATTACGAGTCATAACTATAG
GCTTAATATTACACGATTCTCATTCCATCAAGGCGGAAAACCGCACAAATACTGAAACAC
TATCGATCGATTTGTAAACAAGCCTACTTAAGTAACTTGCAGTCCTTATCATTTCCTTTA
AAATAATCCAGCCCGTCACTACACGAACTGGCGGACTTCTTGCAAATAAAGGTTACTAGA
TTTTCATTCATCTTAATAATAAAAGGATTTTTATCTTTATCTATGGCTACCGCCTTCAAC
ATGAATTTACTGTCTAAAGCCCCGCGCGCGATTCCATTCAAACGGATACAAAAGCCTTCT
GCCTCTTTAATCGGCAAACTTGGCCACTTGGTAGATGTTTGTTTAAACCTCCCATTCTGC
AGATAAAACTTTTCCATAAAATGTGCATTTTCTAACAAGGCTGCCCGCACTGCATTTATC
TTTGCTTTCTCAACATAATTGCGATAGCTCGGATAAACAATTAAAGCAAGTACAGACAAT
ATCAAGACCACTGATATTAATTCAACCAGCGTAAACCCCCGATTATCAGTCATTACTTTA
CTTCCAATAAGAACAGATTATTCAACATATTTCTTTGAACAGACTTACTATCCCATTCAA
CAGTATGCATATTTCCCACTCTATTTTTTAGCGGCCGGTATAGCCGGTTTGGCTGGGCCT
TTTGGTGCGGGCGCGCCGACCGAAGCCTGGTCCTTCAGCTTCGCCAGCACCGCAGGGCCG
ATGCCCTTTACCTTGGTCAAATCGTCTACAGACTTGAACGCACCGTTTTGCGCACGGTAT
TCCGCAATGGCCTTCGCCTTCGCCGGGCCTATGCCCGGCAGCGCCTCCAACTCCTGCTGC
GAAGCCGCATTGATGTTTACCGCCGCAAGGGAGAAGGCGCAGGAGAACAGCATACAGAAC
AGCACGAACATTTTCTTCATGGTTTTTCCTTTAAGGGTTGCAAACAATAAACCGCATCTT
GCGACGATAAAACGAGTCATTCTAAAATGAATATCCCAAAGTTTCAAGCCGTTCCTCCGC
AAACCCGACCGGACACCGTACGGATGCCGTCCCGCCATCACCGACATTTTTTCCGGGCAA
AGCAAACATTTTTTCCGGGCAAAGCAAAAACCCCCGAATAATCGGGGGTTTTCTGAATGG
GTGTTTGGCAGTGACCTACTTTCGCATGGAAGAACCACACTATCATCGGCGCTGAGTCGT
TTCACGGTCCTGTTCGGGATGGGAAGGCGTGGGACCAACTCGCTATGGCCGCCAAACTTA
AACTGTTACAAATCGGTAAAGCCTTAATCAATATATTCGGTAATGACTGAATCAGTCAGT
AAGCTTTTATCTCTTGAAGTTCTTCAAATGATAGAGTCAAGCCTCACGAGCAATTAGTAT
GGGTTAGCTTCACGCGTTACCGCGCTTCCACACCCCACCTATCAACGTCCTGGTCTCGAA

# Appendix A

CGACTCTTTAGTGCGGTTAAACCGCAAGGGAAGTCTCATCTTCAGGCGAGTTTCGCGCTT
AGATGCTTTCAGCGCTTATCTCTTCCGAACTTAGCTACCCGGCTATGCAACTGGCGTTAC
AACCGGTACACCAGAGGTTCGTCCACTCCGGTCCTCTCGTACTAGGAGCAGCCCCCGTCA
AACTTCCAACGCCCACTGCAGATAGGGACCAAACTGTCTCACGACGTTTTAAACCCAGCT
CACGTACCACTTTAAATGGCGAACAGCCATACCCTTGGGACCGACTACAGCCCCAGGATG
TGATGAGCCGACATCGAGGTGCCAAACTCCGCCGTCGATATGAACTCTTGGGCGGAATCA
GCCTGTTATCCCCGGAGTACCTTTTATCCGTTGAGCGATGGCCCTTCCATACAGAACCAC
CGGATCACTATGTCCTGCTTTCGCACCTGCTCGACTTGTCGGTCTCGCAGTTAAGCTACC
TTTTGCCATTGCACTATCAGTCCGATTTCCGACCGGACCTAGGTAACCTTCGAACTCCTC
CGTTACGCTTTGGGAGGAGACCGCCCCAGTCAAACTGCCTACCATGCACGGTCCCCGACC
CGGATGACGGGTCTGGGTTAGAACCTCAAAGACACCAGGGTGGTATTTCAAGGACGGCTC
CACAGAGACTGGCGTCTCTGCTTCTAAGCCTCCCACCTATCCTACACAAGTGACTTCAAA
GTCCAATGCAAAGCTACAGTAAAGGTTCACGGGGTCTTTCCGTCTAGCAGCGGGTAGATT
GCATCTTCACAACCACTTCAACTTCGCTGAGTCTCAGGAGGAGACAGTGTGGCCATCGTT
ACGCCATTCGTGCGGGTCGGAACTTACCCGACAAGGAATTTCGCTACCTTAGGACCGTTA
TAGTTACGGCCGCCGTTTACTGGGGCTTCGATCCGATGCTCTCACATCTTCAATTAACCT
TCCAGCACCGGGCAGGCGTCACACCCTATACGTCCACTTTCGTGTTAGCAGAGTGCTGTG
TTTTTAATAAACAGTCGCAGCCACCTATTCTCTGCGACCCTCCGGGGCTTACGGAGCAAG
TCCTTAACCTTAGAGGGCATACCTTCTCCCGAAGTTACGGTATCAATTTGCCGAGTTCCT
TCTCCTGAGTTCTCTCAAGCGCCTTAGAATTCTCATCCTGCCCACCTGTGTCGGTTTGCG
GTACGGTTCGATTCAAACTGAAGCTTAGTGGCTTTTCCTGGAAGCGTGGTATCGGTTGCT
TCGTGTCCGTAGACACTCGTCGTCACTTCTCGGTGTTAAGAAGACCCGGATTTGCCTAAG
TCTTCCACCTACCGGCTTAAACAAGCTATTCCAACAGCTTGCCAACCTAACCTTCTCCGT
CCCCACATCGCATTTGAATCAAGTACAGGAATATTAACCTGTTTCCCATCGACTACGCAT
TTCTGCCTCGCCTTAGGGGCCGACTCACCCTACGCCGATGAACGTTGCGCAGGAAACCTT
GGGCTTTCGGCGAGCGGGCTTTTCACCCGCTTTATCGCTACTCATGTCAACATTCGCACT
TCTGATACCTCCAGCACACTTTACAATGCACCTTCATCAGCCTACAGAACGCTCCCCTAC
CATGCCGGTAAACCGGCATCCGCAGCTTCGGTTATAGATTTGAGCCCCGTTACATCTTCC
GCGCAGGACGACTCGACCAGTGAGCTATTACGCTTTCTTTAAATGATGGCTGCTTCTAAG
CCAACATCCTGGCTGTCTGGGCCTTCCCACTTCGTTTACCACTTAATCTATCATTTGGGA
CCTTAGCTGGCGGTCTGGGTTGTTTCCCTCTTGACAACGGACGTTAGCACCCGCTGTCTG
TCTCCCGAGGAACCACTTGATGGTATTCTTAGTTTGCCATGGGTTGGTAAGTTGCAATAA
CCCCCTAGCCATAACAGTGCTTTACCCCCATCAGTGTCTTGCTCGAGGCACTACCTAAAT
AGTTTTCGGGGAGAACCAGCTATCTCCGAGTTTGTTTAGCCTTTCACCCCTATCCACAGC
TCATCCCCGCATTTTGCAACATGCGTGGGTTCGGTCCTCCAGTACCTGTTACGGCACCTT
CAACCTGGCCATGGATAGATCACTCGGTTTCGGGTCTACACCCAGCAACTCATCGCCCTA
TTAAGACTCGGTTTCCCTACGCCTCCCCTATTCGGTTAAGCTCGCTACTGAATGTAAGTC
GTTGACCCATTATACAAAAGGTACGCAGTCACACCACTAGGGCGCTCCCACTGTTTGTAT
GCATCAGGTTTCAGGTTCTGTTTCACTCCCCTCCCGGGGTTCTTTTCGCCTTTCCCTCAC
GGTACTGGTTCACTATCGGTCGATGATGAGTATTTAGCCTTGGAGGATGGTCCCCCCATA
TTCAGACAGGATTTCACGTGCCCCGCCCTACTTTTCGTACGCTTAGTACCGCTGTTGAGA
TTTCGAATACGGGACTGTCACCCACTATGGTCAAGCTTCCCAGCTTGTTCTTCTATCTCG
ACAGTTATTACGTACAGGCTCCTCCGCGTTCGCTCGCCACTACTTGCGGAATCTCGGTTG
ATTTCTTTTCCTCCGGGTACTTAGATGGTTCAGTTCTCCGGGTTCGCTTCTCTAAGTCTA
TGTATTCAACTTAGGATACTGCACAGAATGCAGTGGGTTTCCCCATTCGGACATCGCGGG
ATCATTGCTTTATTGCCAGCTCCCCCGCGCTTTTCGCAGGCTTACACGTCCTTCGTCGCC
TATCATCGCCAAGGCATCCACCTGATGCACTTATTCACTTGACTCTATCATTTCAAGAAC
TTCTTTGACTTTGCCTAACATTCCGTTGACTAGAACATCAGACTTGAATTTCCTACTTTG
ATAAAGCTTACTGCTTTGTTGTGTCTTAATCCTGCCTTTTGTGTTTCAGGATTAAGTCGA
~~TACAATCATCACCCAAATACTGTGTTTGTTTTCTTTTCTCTTGCGAGAGATTTTTATCCT~~
TTGCAAAGAATAAAAAATCAAAACAAACGCTTTGTCTTTGTTTGTTGATTTCGGCTTTCC
AATTTGTTAAAGATCGATGCGTTCGATATTGCTATCTACTGTGCAAATCAAAACGAGCTG
ATTATTATATCAGCATTTTGTTCTTGGTCAAGTGTGACGTCGCCCTGAATGGATTCTGTT
CCATTCTTCCGTTTTGATTTGTACAGTATTGGTGGAGGCAAACGGGATCGAACCGATGAC
CCCCTGCTTGCAAAGCAGGTGCTCTACCAACTGAGCTATGCCCCCGTTCTTGGTGGGTCT
GGGAGGACTTGAACCTCCGACCCCACGCTTATCAAGCGTGTGCTCTAACCAGCTGAGCTA
CAAACCCGGATTCTCTTCTTAAGCGAATCTTGCCTTCACTCAAGCTTCTTCCGCATCTTT
TTCAGTTTACCGATAAGTGTGAATGCCTAAAGCCTCTTCTTTCTCTAGAAAGGAGGTGAT
CCAGCCGCAGGTTCCCCTACGGCTACCTTGTTACGACTTCACCCCAGTCATGAAGCATAC
CGTGGTAAGCGGACTCCTTGTGGTTATCCTACCTACTTCTGGTATCCCCCACTCCCATGG
TGTGACGGGCGGTGTGTACAAGACCCGGGAACGTATTCACCGCAGTATGCTGACCTGCGA
TTACTAGCGATTCCGACTTCATGCACTCGAGTTGCAGAGTGCAATCCGGACTACGATCGG
TTTTGTGAGATTGGCTCCGCCTCGCGGCTTGGCTACCCTCTGTACCGACCATTGTATGAC
GTGTGAAGCCCTGGTCATAAGGGCCATGAGGACTTGACGTCATCCCCACCTTCCTCCGGC
TTGTCACCGGCAGTCTCATTAGAGTGCCCAACTGAATGATGGCAACTAATGACAAGGGTT
GCGCTCGTTGCGGGACTTAACCCAACATCTCACGACACGAGCTGACGACAGCCATGCAGC
ACCTGTGTTACGGCTCCCGAAGGCACTCCTCCGTCTCCGGAGGATTCCGTACATGTCAAG
ACCAGGTAAGGTTCTTCGCGTTGCATCGAATTAATCCACATCATCCACCGCTTGTGCGGG
TCCCCGTCAATTCCTTTGAGTTTTAATCTTGCGACCGTACTCCCCAGGCGGTCAATTTCA
CGCGTTAGCTACGCTACCAAGCAATCAGGTTGCCCAACAGCTAATTGACATCGTTTAGGG
CGTGGACTACCAGGGTATCTAATCCTGTTTGCTACCCACGCTTTCGGGCATGAACGTCAG
TGTTGTCCCAGGAGGCTGCCTTCGCCATCGGTATTCCTCCACATCTCTACGCATTTCACT
GCTACACGTGGAATTCTACCTCCCTCTGACACACTCGAGTCACCCAGTTCAGAACGCAGT
TCCCGGGTTGAGCCCGGGGATTTCACATCCTGCTTAAGTAACCGTCTGCGCCCGCTTTAC
GCCCAGTAATTCCGATTAACGCTCGCACCCTACGTATTACCGCGGCTGCTGGCACGTAGT

## Appendix A

TAGCCGGTGCTTATTCTTCAGGTACCGTCATCAGCCGCTGATATTAGCAACAGCCTTTTC
TTCCCTGACAAAAGTCCTTTACAACCCGAAGGCCTTCTTCAGACACGCGGCATGGCTGGA
TCAGGCTTGCGCCCATTGTCCAAAATTCCCCACTGCTGCCTCCCGTAGGAGTCTGGGCCG
TGTCTCAGTCCCAGTGTGGCGGATCATCCTCTCAGACCCGCTACTGATCGTCGCCTTGGT
AGGCCTTTACCCCACCAACTAGCTAATCAGATATCGGCCGCTCGAATAGCGCAAGGCCCG
AAGGTCCCCTGCTTTCTCTCTCAAGACGTATGCGGTATTAGCTGATCTTTCGATCAGTTA
TCCCCCACTACTCGGTACGTTCCGATATGTTACTCACCCGTTCGCCACTCGCCACCCGAG
AAGCAAGCTTCTCTGTGCTGCCGTCCGACTTGCATGTGTAAAGCATGCCGCCAGCGTTCA
ATCTGAGCCAGGATCAAACTCTTATGTTCAATCTCTAACTTTTTAACTTCTGGTCTGCTT
CAAAGAAACCAACAGGACAATGTTCAAAACATTATCTTGTCTGTCTTTCAAACAGTGTGA
GACTCAAGGCACTCACACTTATCGGTAATCTGTTTTGTTAAAGAGCGTTGCGAATTATAA
AGTATTCCTTCCGCCTGTCAAGATATCTCTCGATATCCCCAACATTCTGTGCTATACTTT
TCAGTTCGTCCGCCACTTCTGCAGCAGCGAAGAACCGAACTATACGCCCACAGGGAAAAA
CGGTCAATGCTTTTCTGAAGAAATTTTTTTAAAAATATTTATCTATTTGTTTATAAATTT
AATTTATATCAGTCAATTTTATTTTCCATACAGAATTCTTCCAGTGCCCGATGGATATTT
TCAGTCTGCCATTCGTTTTTTAAGGGTGCAACAATTTCGATTTGTCGGTTTTGGTAGTCA
AATTGTATTTTCCATGCATACAGAAACATGGTTTCGGATTCTGTTCCGCCGTATAAGCTG
TCGCCCAGAATCGGACTGCCCAAACTTTTCATCGCCACTCTCAATTGGTGCGTTTTGCCC
GTATGCGGTTCTAGGATGAACAGCCGCAGTTTTTCGGCGATACTGATGCTGTGGAATCGG
GTAACGGCGATATTTTCTGTATTGCGCGTCAACTTCCACATTCCACATCTGGATTTTTCC
ATTCCGCCTTTAATCCAACCCTGCTTTTTGGACGGCTTGCGGTCGGACAGTGCCAAATAG
GTTTTTTTGATGCTTTTGCCGGCAAACTGTCCGCTAAGGGCGGACGCGCTTTCTTTGTTG
AGGGCAAACAGTAAAATGCCGCTGGTCTGTTTGTCCAATCGGTGCAGCAGCCACACACGC
TCTACGCCCAACTGTATGGCGAGTGTTCGGGCCAGTCCGGTCTCGCCGCTGTCTTGGTGG
ACGGATATGCCGCCCGGTTTGTTGATGGCGACGAAGTCTTGATGGCGGAACAAAATTTCC
AACATATCCATATATGCCTTGCAAAAATAGAAGGGTTCAATTTTCGTGTTGATGTTCGGC
AAGGATTTTTTCGTACACAGCTTGCGGCACGTAGCGGTGGATCGTTTCCGTCCAGCCTTC
CGGCCCGACCAGTCCTTTGACCATAGTGGACGACACTTCGGCGATTTCGCGCGGCGGCAT
GAGGAATACGGTGGATATTTCGGGGGCGAGGTCGCTGTTGATATGGCGCATGGAACGTTC
GTATTCGTAATCCGAAGCAGAACGGATGCCGCGCACGATGAATCCTGCATCTACCTCACG
GGCGTAATGCACCAGAAATCGGTTTTCAAATACATCGGTTCTGACGTTGGGAAACATTTT
AGTAATATCGCACAACATATCCTGCCTTTCAGCGACGGTATAGGTGCTGCGTTTGTCGGG
GTTAATGCCGATGGCGACGATGAGTTCGTCAAACATAGATTGCGCCTGCCGTATCATCCA
CAGATGCCCCAATGTGGGCGGATCGAAACTGCCGGCATAAACGGCGCGGCGCGGTGTATT
CGGTAACATTTGATTCCTCCCGGCTTCATAGTCGGCTGTGTGTTGGTGCGTGTGCATCCG
TATTGTATGCCCAAAGTAAAATGCCGTCTGAAGCATTTTCAGACGGCATAGTCGGACGGC
GTTTTACCGGCATCAATCCTCGCCGTTTAAAGACAACAGGATGTTCAGCAGGCTGCTGAA
GATGTTGTAAAGCGAGATAAACAGTGTCAGTGCCGCGCTGATGTGGCTGTCTTCGCCGCC
GTCGATGACGGTGCGTACCTGCCACATAATCATTAAGGAACTGAACAAGACAAAACCGGC
GGAAATGGTCAGGGCGAGTGCGGGAATACCCAAAAACAGATTGGCAACCACGGCGACCAT
CAGAATGACCGCACCTACGGTCAGGAAGCGTCCGAGCGCGTTCATATCGAGCCGGGTTCG
GCGCGCCAAGGCGGACATCGTTAAAAAGACGGCGGCGGTCATCGCGGCGGCAATGCCGAC
GATTTTCGCACCGTCGGCAATATGGAGCGCGTATTGCAGCACGGGGCCGATCAATACGCC
CATACCGAATGTGAATACCATCAGCAGGGTAACGCCGGTATTGCTGTAACGGTTTTTCTC
GATGAAGTGGATCATACCGTAGAAAAACGCCAACACGACGGCAAACCCTATCCAGCGCGA
ACCGAAGGCGGCGTAAAAATTGAAACCGGCATTGGCGGCAAGTGCCGCGCCTGCGGAAGC
CGGAATAAATGAAAATCCGAGCAGGCGGTAGGTTTTCTGCAGGACGGTGTTTTTAGAAAC
CGTATGCGCGGTGTAGTCGTAAACGTCGTGTTGCATATCATCTGCTCCTGAAAGCGCGGT
TGGGAATAATGGGGGATTTTAACATTGCCCAATGTCAAAATTTGTCCGGTTGCGTGAAGA
TAAAGTTGTCCGGCGTATTTTAAAGGCCGTCTGAAGCAGTTTCGGACAGCCTGTGTTCAA
AACGGAAAACCGTTATTGCGGAACGTATCCCTGAACGGCATCCGCGCCGTCGCCGAAGAA
ATACTGCTCCATCTGCTGAGCCAGGTATTCGCGCGCGCGCGGATCGGCGAGGCTTAAACG
GTTTTCGTTAATCAGCATCGTTTGGTGGCGCGTCCACGCCGCCCACGCTTCTTGCGATAC
GTTTTCAAAAATGCGCTTGCCCAATTCGTTGGGAAGCGGCGGAAATTTCATGCCTTCGGC
TTCTTTGTTGAGCTTGACGCAGAATACCATGCGTGCCATAGCGGATTCCTTTGCTGTGTT
CAGAAATAACGGGGTGATTTTAACCGATTAGGGATACGGACAAAAGCCTTCTTATTCCCG
ATGATAGGGATGGTTGTGCAGGATGGAAACGGCGCGGTAGAGCTGCTCGGTCAGAAAGAC
GCGCACCATGCCGTGCGGCAGGGTCAGGCTGGACAGGCGCATCATCATGCGTGCCTGCTG
TTTGAGGCGGTCGGTCATGCCGTCCGCGCCGCCGATGACGAAGCAGACGTGTTCGCCGTT
TTGCCGCCAGCTTTTGAGGTGTTCCGCCAGCTCGACGGAGGTCGGTGCTTTGCCGCGTTC
GTCAAGAACGACGAGGAACGCGCCTTGCGGAATGGCTTCAAGGATGCGTTTTTCTTCCGC
CGCCATACCTTGGGCGGCATTCACGCCCGCGCCGCGTTTTTCGGGTTTGATTTCTTTGAG
TGCGTAGGCGACGTCGCGTCCGAAGCGTTTGGCGTATTCGGCGACGGCCTCATCAACCCA
GCGCGGCATTTTGGTGCCGACTGCCAAAACGGTGATGTTCAATGCTTTCTCCCTTACAGG
AAAATGCCGTCTGAAGGTTCAGACGGCATCGGGAATCAGTCTGCCGCGTGCCACGGCTTC
TGCATTCCGGCGTGGAAACTCGGTTTCTCGCCGCCCCAGAGGGTGTCGATGTCGTAGAAG
TCGCGCACGGCAGGGAGCATGACGTGGACGACGAGGTCTCCTGCATCAACCAGCGTCCAT
TCGCCGCTGTCGCCTTCGGTACTGAGGATTTCAAAACCGGCTTCTTTCAAATCGACGGCA
ACGTTGTTGGCCAGTGCTTTGACTTGGCGCGTACTGTCGCCGCTGGCGATAATCATTCTG
GCAAACAGCGAAGTTTTGTCTTGGGTTTCGAGAACGGAAATGTCTTTGGCTTTGATGTCT
CCGAGGGCGTTGACGGCGACCCCGACCATTTTTTGCAGGTCTTGCAGTTCTTGTTCGTTC
ATTATTTTCCTAACGGGATGTTTTCAGACGGCATTATAGCCGTTTCTTACTGATTTGACT
TTATTTTTCATACAAACCGTGTTCGCGGATGTAGCGTGCGGCGGCAGGCGGGATGCCGTC
TGAAACGCCTTGGCCGGCAAGGTTGCGGCGGATTTCCGTTGACGACACATTATGCATCGG
GGCGGACAAGATGCGGACGCTGCCGTCCTGAAGGGACTTGCCCAGCCACGCGTGCAGTTC

## Appendix A

GCGCGGGGTTTGGTGCAGGCTGTCGCCCTGCCTCATGGCGACGGCGATATTGGTTTCGCG
CACGAGCATCTGCCATTTTTTCCATGTGTGCAGCTTCATCAGGCTGTCGCTGCCCATCAG
CCACCAGAGTTGCGCGGATGGGAACTGCTGGCGGAAGATTTGGACGGTATCAAAAGTATA
GGTTGCACCTTCTCGGACGATGTCGCAATCGCTGACGGCAAAACGCGCGTCTTCTGCCGT
CGCCAATTCGACCATGGCAAGGCGGTCGGCGGCGGAAGCGGAGGCTGCGTCTTTGTGATA
CGGGCCGCCTGTCGGCAGGAAAACAACCGCGTCCAAGCCGATTTCGTCGGCAAAGGCACG
GGCGATATGAAGATGTCCGTTGTGTATCGGGTCGAAAGTACCGCCGAACAATCCGATTTT
CTTCATGATGTTTCCATTCCTTCCGATAAGTCCATGCCGTCTGAAACACTGCCGTCCACA
ACCAGCGTCAGTTTGCCGGTAACGGGATGGATGACCAGTCCGTGAACGGCGATATGGCGC
GGCATCAGCGGATGGTTACGGATAAGGTCCACCGTGTGGCGCACGCTGTCTTCGACGTTG
TCGAAACCGGTCAGCCAGCCGTCGAGGTCGATACCGGCATAACGCAGGGTTTCGATACGG
TCTTCGGGAATCCGGCTTTCCCGGACGCGCCCGAGGAATTCTTCGGCATTCAGCCCCTGC
ATACCGCAATCGTGATGGGCGATGACCATAATCTCTCTGACCTTCAGTTCAAACACGGCA
ACCAAAAGGCTCCGCATCACCGAACCCCACGGGTGCGTAACCAGCGCGCCGGCATTTTTA
ATCAGCTTGGCATCGCCGTTTTTCAAACCCAACGCGTCGGGCAGCAGCCCGATAATCCGC
GCATCCATACAGGACAAAACTGCCAGCCCGCGTTCGGGGTATTTGTCGGTAAAGTATTTT
TCATATTCGCCCGACTCGACAAACTGCCGGTTATGGGCAAGGATGTTATCCAACTCGCTC
ATTTTGCCGTCCTCTGAAAAAGGGTTCACATTATAACGTTTCCGTCTGTTTTCCGCCTTC
GCCGCCGTCCAACAGCAGGAAAATACCCAGCGCGAACGCCGCCAACAGCAATGTCAGCAC
CATCGCCCGCGCGTAATTATCCTCACCCGCGCGTCCCAAATAGGCATAAATCAAAGTCGT
CAGCGTCTGCCATTCCGGACGCGACAGAAACAATGTCGCCGCAAATTCGCCCACGCAGGT
TGCCGCCGCCAAAGTCAGACCGCGCCGCAACGCCGGTTTCAAGAGGGGGAACGTGATGCG
GCATGCCGTCTGAAAGCCGTTTGCACCCAAACCCGCCGCCGCCCTGCCGTAATCCGGCGG
CAGTGCATCCCAGGCTGATAAAACATCTTTTGCCACAAACGGATACGCCAGCAGCGCATA
CATCGCCAGCAGCAACGGCAACGAAGCCGTCCACTGCGGATAAAGCAGCAGCACGCCCGC
CGAAACACAAACCGGCGACACCATAAACGGCAAAAACATCAGCCCGCGCATCCACGCCGA
CCGCCGCGCCGCCGCCGCATACACCACACCCAAAACCGCCGCCGCATACACCGCCGCCGC
CGAGAAGCGCAAAGTATTCCACACCGCCTGCCACGTTTCACTTTCCATTAACACACGCCA
CGATTCGCCGGCCGACCACGCTTTCACAACAATTGCCAACAAAGGAAACAGGCAGCACAC
AGACAACACCGCCGCCGCAAACGCCAGCAGCACATATTCCCCGACCGACTGCGGCGGCGA
CGGCATCACAGGGGAAACCGCCTTATCCGAAACCGCGCGCCTGCCGAACCACGCATACAG
CAACCCTGCCGCCGCCGTTACCCCCAACACCAGCCACACCAGCACCGAAGCAACCGCCAT
ATCGAGTTCGAACATGACCAACTGGTAAATTTCCACTTCGACCGTGGCATAACGGCTGCC
GCCCAGCAGCAGCGCCAGCCCGAACCCGGAAAAACAATACAGAAAGACAAGGCACACGCC
GCCGGCAAGCCACGGGCGCAAAACGGGCATTTCAATGTCCCAAAACCGCCGCCACGCCCC
CGCGCCCAACGTCCGTGCCGTCTGAAGCCGTGCCGCAGGCACTTGCACAAACCCCTGATA
CGCCGCCCTGACCAACACAGGAAGGTTGAAAAACACATTGCCGTACAACAACAGATACGG
CGTATCCTGCCTGCCGCGCCACAACAGCCCGTCCGCCCCGAACAGGGCCAGCACGCCCAC
GCCCGCCACCAACGTGGGCATCACAAAAGGCAGCATCAGCAGGCGCAGCACCAAAGCCCG
CCCCGGAAACGCCAGCCGCGCCAGCACCCACGCGACAGGCACGCCCAAAGGCAGCACCAG
CACACAGGTTGCCGCTGCCTGAAATACCGTCCACGCCAAACGTTTGAGCATATAGGCATC
CGACAGCACCGCGCGCCACGCCAAACCGTCATACGCCGCCACCGCCCACAAAGGCGCAAC
GACCATTACCGCCAAAAAAGCCGAAGGCAGCAGGGCAAAAGCACCCCATACCACCCAACG
CCGTCCATCCATCGCCTTCCCCACTTGAAACACTGATGTTGCGATTGTACCCAAAAGCCC
CCACATACCGTATATTTCAATCCGACTACATACCGTATCCGCCTTCCTCCCGCCGTCTGA
AATATAGTGGATTAACAAAAATCAGGACAAGGCGACGAAGCTGCAGACAGTACAAATAGT
ACGGAACCGATTCACTCGGTGCTTCAGCACCTTAGAGAATCGTTCTCTTTGAGCTAAGGC
GAGGCAACGCCGTACTGGTTTTTGTTAATCCACTATAAATCGTTCAAATAAACAGGAATA
TAACTTCAGACAAACAACTTACCGCCCGATTTGTGCTATCGTTTTCGCACAACTTAAAAA
AACCTGACAATTTTTGTACTTTTATTACAGAGAAAGGCTTTACAAATGGACGGCTGGACAC
AGACGCTGTCCGCGCAAACCCTGTTGGGCATTTCGGCGGCGGCAATCATCCTCATTCTGA
TTTTAATCGTCAAATTCCGCATCCACGCGCTGCTGACACTGGTCATCGTCAGCCTGCTGA
CGGCTTTGGCAACCGGTTTGCCCACAGGCAGCATTGTCAACGACATACTGGTCAAAAACT
TCGGCGGCACGCTCGGCGGCGTGGCGCTTCTGGTCGGCCTGGGCGCGATGCTCGGACGTT
TGGTCGAAACATCCGGCGGCGCACAGTCGCTGGCGGACGCGCTGATCCGGATGTTCGGCG
AAAAACGCGCACCGTTCGCGCTGGGCGTTGCCTCGCTGATTTTCGGCTTCCCGATTTTCT
TCGATGCCGGACTAATCGTCATGCTGCCCATCGTGTTCGCCACCGCACGGCGCATGAAAC
AGGACGTACTGCCCTTCGCGCTTGCCTCCATCGGCGCATTTTCCGTCATGCACGTCTTCC
TGCCGCCCCATCCGGGCCCGATTGCCGCTTCCGAATTTTACGGCGCGAACATCGGCCAAG
TTTTGATTTTGGGTCTGCCGACCGCCTTCATCACATGGTATTTCAGCGGCTATATGCTCG
GCAAAGTGTTGGGGCGCACCATCCATGTTCCCGTTCCCGAACTGCTCAGCGGCGGCACGC
AAGACAACGACCTGCCGAAAGAACCTGCCAAAGCAGGAACGGTCGTCGCCATCATGCTGA
TTCCCATGCTGCTGATTTTCCTGAATACCGGCGTATCGGCCCTCATCAGCGAAAAACTCG
TAAGTGCGGACGAAACCTGGGTTCAGACGGCAAAAATAATCGGTTCGACACCGATCGCCC
TTCTGATTTCCGTATTGGTCGCACTGTTTGTCTTGGGACGCAAACGCGGCGAAAGCGGCA
GCGCGTTGGAAAAAACCGTGGACGGCGCACTCGCCCCCGTCTGTTCCGTGATTCTGATTA
CCGGCGCGGGCGGTATGTTCGGCGGCGTTTTGCGCGCTTCCGGCATCGGCAAGGCACTCG
CCGACAGCATGGCGGATTTGGGCATTCCCGTCCTTTTGGGCTGTTTCCTTGTCGCCTTGG
CACTGCGTATCGCGCAAGGTTCGGCAACCGTCGCCCTGACCACCGCCGCCGCGCTGATGG
CTCCTGCCGTTGCCGCCGCCGGCTTTACCGACTGGCAGCTCGCCTGTATCGTATTGGCAA
CGGCGGCAGGTTCGGTCGGTTGCAGCCACTTCAACGACTCCGGCTTCTGGCTGGTCGGCC
GTCTCTTGGACATGGACGTACCGACCACGCTGAAAACCTGGACGGTCAACCAAACCCTCA
TCGCACTCATCGGCTTTGCCTTGTCCGCACTGCTGTTCGCCATCGTCTGACAGACGGAAA
GGATAGTAAATGACTACGCATTTTGTCGTTATGGGCGTATGCGGCTGCGGCAAGACCACC
GCCGCGCTGTCCCTGCAGAAACACCTCGGTCAATGTCCCTATGCCGAAGGCGACGAGTTC

# Appendix A

CACACCCAAGCCAACCGCGACAAGATGGGCGCGGGTATTCCGCTGACCGATGAAGACCGC
TATCCGTGGTTGGGCAATCTGCGCGACTGGATGACGCAACAGGCGCAAAACGGTGCGAAC
CACACCATCGTAACCTGTTCCGCCCTCAAACGCGGCTACCGCGACATTCTGCGCGGAGCC
GAAGGCAAAGCTGCCTTCATCCACCTCAGTCCGCCGCAAGACATCAACCTCGAGCGCATG
ATGTCGCGCAAAGGACATTACATGAAAGCAGGGATGCTCGATTCGCAACTGGAAATCCTC
GAGGAACTGGGCGAAGGCGAATACGGGGTCAAAATCGCCAACCCCGGCACGCCCGAAGCG
GTCGAAGCCGATATTCTGAACTGGGTTGCCTCGGAAAACCTGCTTTGAAGCAATATGCCG
TCTGAAGCCCGACACAGGATGGGTTTCAGACGGCATAAACATCGGGAACAGAATGGATTA
CATTGATTTATAGTGGATTAACAAAAACCAGTACAGCGTTGCCTCGCCTTAGCTCAAAGA
GAACGATTCTCTAAGGTGCTGAAGCACCAAGTGAATCGGTTCCGTACTATCTGTACTGTC
TGCGGCTTCGTCGCCTTGTCCTGATTTTTGTTAATCCACTATAAAATGGAAAATACCCGG
CTATCGTCTCATTTTCGTTTTAATCAGCCATAAAAATGCCGTCTGAAACCCTTTCAGACG
GCATTTCTGTCAAACGCCGGACGCACTCAACCCAAACTCAACAGCAGGTTGCGGAACGCG
TTCGGGTCTTTGATAAACGTCATCTCGCCCGCCTGCGGAAAATGAAAATCCAACAGGCGC
GACACCCAAAAACGGATGCAGCCGGCACGTTGGGCGGTCGGGAAATACGCCTTTTCTTCG
GCACTCAAGGGGCGCACGCCCTCATAACCGCCGATAAACGCCTTTTTCAACGCCTCATCC
AACTTATTGTCCGCCGTCCTTGCCCAATCGTTGACCGCAATCGCCAAGTCATACATAAAA
TTGCCCCGGCAGGCGTAATAGAAATCGATGAAGCCCGATACCTGACCGCCGTCAAGCAAC
ACATTGTCTTTAAACAGATCGGCATGGATGATGCCCGAAGGCAGATGATTGCCGAGATTG
TCCTTCAACGCATCGATTTCGGAACACAGCAGTGCGGCATCGTCTTGCGACAGGACGGGC
AGCAGCCGGGCGCACGCCTCCGTCCACCACGCATTGTAACGCGGGTTTTCCATTTCCAAA
GGGAAATCGGCGGCGGCAAGGTGCATTTTCGCCAACATCGCACCGGTATGAAAACACTGC
TCAGCCGTCGGCAGCGCGGTATCCGAACCTTTCAGGCAGGCAACCAGGCAGGCAGGCTTA
CCCGCCAAAACGGAATCAAGCCGGCCGTCTTTGCGCGCAACCGGCGCGGCAACCGCCACG
CCCTTCATACTCAAATGCCGGTTAAGCTCCAGAAAAAACGGCAGCTCTTCCTGTTTCAAC
ACTTCAAACACGGTCAGCACATAACGTCCCGAAGTCGTCGTCAGAAAATAATTGCTGTTG
GTAATCCCCTGCGCGATGCCCTGCAGGGAAACAAATTCCCCCAAATCGTAACCGCTCAGG
AAGCCGCGCATTTCATCATCGGAAACACTGGTATAGACAGACATATCAACTCACTTGCTC
AAAAACGGGCAAACCCGCCGTCAGAACGCCGGACGGCAGGCAAACATATAATTCACATCG
GTCGAATCGCACAGGGCATAAGTTTGCGACAACACATGGTAAGTCATACCCTTCGTATCC
GCCACATCCAAGCCTGCCTGACGGCACATTCGCGCCAGCTCGGCAGGTGCGATGAATTTT
TTCCAGTCGTGCGTGCCTTTGGGGACAAACTTCAACAGATATTCCGCCGCCACAATCAGA
TGCAGGTACGATTTCGGGTTTTTATTGATGGTGGAAAAAAACACCATGCCGTCCGGTTTG
ACCAGATTGGCACAAGCACGCACGATGGCGGCGGGATCGGGGACGTGTTCCATCATTTCC
ATGCACGTTACCACATCGAACGAGTGCGGTTCCGCCTCGGCAAGGTCTTCCACGCGGATA
CATTCGTATTCGATATCGGCGACATTGTTCAAAGCCGCGTGCAGGCGGGCGGTTTCCAAC
GACTGCTCCGCCATGTCGATGCCCTTTACAAACGCCGCGCCGCGCCGCGCCATACTTTCC
GCCAAGATGCCGCCGCCGCAGCCCACGTCCAAAACCCGTTTGCCGCACAAATCCGCGTGT
CCGTCGATATAATCCAGCCGCAGCGGATTGATGTCGTGCAAGGTTTTGAACTCGCCCGAC
TTGTCCCACCATTTGTCGGCAATCCGGCTGAATTTGGCGATTTCCCCCTCATCGACATTA
TATTTTTTGTCGGACATTTTCCCTCCCATCTGACGAACCGCCCACTCCAAAACCCAAGAT
TATACTATGGAACACAATGCCGTCTGAAAGCCTTTTCGGGCGGCGCGGCGCAAATACCTT
ACAAATCCTTACACTTTACGGCATAATGGCGGCTCGCTTTTTCTGGCAGAAAGACAAAAT
ATGCCCAACAAAACCCCTTCACTGTTCGGCGGCGCGATGATTATCGCCGGCACGGTCATC
GGCGCAGGCATGCTCGCCAACCCGACCGCCACATCCGGCGTATGGTTTACCGGCTCGCTG
GCCGTGTTGCTGTACACCTGGTTTTCTATGCTTTCCAGCGGCCTGATGATTTTGGAAGTC
AACACCCATTATCCGCACGGCGCAAGTTTCGACACGATGGTCAAAGACCTGCTCGGACGC
GGCTGGAACATCATCAACGGCATCGCCGTCGCCTTCGTTTTATACCTGCTTACTTACGCT
TATATCTTCGTCGGCGGCGACCTGACCGCCAAAGGCTTAGGCAGCGCGGCAGGCGGCGAC
GTTTCACTCACCGTCGGACAACTCGTCTTCTTCGGCATCCTCGCCTTTTGCGTATGGGCA
TCCGCACGCTTGGTCGACCGCTTCACCGGCGTCCTTATCGGCGGCATGGTATTGACCTTT
ATTTGGGCGGCCGGCGGGCTGATTGCCGATGCCAAGCCGTCCGTCCTCTTCGATACCCAA
GCCCCCGCCGGCACAAACTACTGGATTTACGCCGCCACCGCCCTGCCCGTCTGCCTCGCT
TCCTTCGGCTTCCACGGCAACGTCTCCAGCCTGCTCAAATACTTTAAAGGCGACGCGCCC
AAAGTGGCTAAATCCATCTGGACGGGCACACTGATTGCGCTGGTAATTTACGTCCTCTGG
CAAACCGCCATCCAAGGCAACCTGCCGCGCAACGAGTTCGCCCCCGTCATCGCCGCCGAA
GGGCAAGTCTCCGTCCTCATCGAAACCCTGTCCAAATTCGCCCAAACCGGCAATATGGAC
AAAATATTGTCCCTGTTTTCCTATATGGCGATCGCCACCTCGTTTTTAGGCGTAACGCTC
GGACTCTTCGACTACATCGCCGACATCTTCAAATGGAACGACAGCATCTCCGGCCGCACC
AAAACCGCCGCGCTGACCTTCCTGCCGCCCCTGATTTCCTGCCTGCTCTTCCCCACCGGC
TTCGTTACCGCCATCGGCTACGTCGGCCTGGCGGCAACCGTCTGGACAGGCATCATCCCC
GCCATGCTGCTCTACCGTTCGCGCAAAAAATTCGGCGCAGGCAAAACCTATAAAGTTTAC
GGCGGCTTGTGGCTGATGGTTTGGGTCTTCCTTTTCGGCATCGTCAACATCGCCGCACAG
GTATTGAGCCAAATGGAACTCGTCCCCGTATTTAAAGGATAAAGGCAAAATGCCGTCTGA
AGCCCGCCGGCGGCTTCAGACGGCATTGCCGCAACAAACGGCAACCGTATTCCGGCACAC
AGCGCATTACCCTGCCCCTCACGCACAAATCCCGCCCCGACAAACCGGGACGCAACCATA
AAGGAACAATGATGAAGCTCAAAATAGACATTGCAACCAACAACTTCAAACACGGCGGCG
GCACGGAACGCTACACATTGGATTTGGTAAAGGGTCTGAACAGACAAAACATCACACCGG
CCGTTTATGCGACGAAATTTGATCACAGCATTCCTGAATACGCCCTAATCGAACCCCATC
TTGTCGATCAACACCGGACGCTGAAAAAACTACGCTCATTCCTCTTTTCAAGCCGGCTCG
CTCAAACCAGAAAAAACAGTGCCGCCAAACTGATTGCCTGCCACCACGCCGATTACGCCG
ACCTCCTCATCTGCGGCGGCACACACTTGGGCTACCTGCACCATATGGCGCAAAAACCGA
ACCTGCTCGACCGCCTCGCCATACGCCGCAACCGCAGCAACTACGCCACCGCCAAACTGA
TTATGGCGCATTCCCATATGATGCGGCGCGAACTGGTCGGACTGTACGGCGTTCCCCCTG
AAAGAATCCAAGTCGCCCCCCCCCCCCGCAGATACGGAACGCTTCTTTCCACAACCCGGA

## Appendix A

GAAACTGCCGACCTGCGCGCCAAATACGGCTTTGCCGACCATGAAACCGTTTTCCTGTTC
CCATCGACCGGCCACACGCGCAAAGGTCTGGAACTGCTTGCCGACTTTTTCGAACATACC
AGCCTGCCCGTCAAGCTCGCCGTTGTCGGCTCCCCGCTTCCCCGCCCTATGAAAAACGTC
GTCGGACTGGGCTTCTGCACCGATATGCCCGAACTCTACCGCGCCGCCGACTTTACCATT
ATGGCTTCCCTGTACGAACCCTTCGGGCTGGTCGGCGTCGAATCCGTCCTATGCGGCACA
CGCGTCGTCCTCTCCGAAAACATGGCATGTACAGAGGTCATGAACGAAGAAGCCGGCTTC
TTTTTCTCACGCCAAAACCCGGAAACCCTGGCGCAAGCCGTTGCCCAAGCCGTCAGCCTT
AAAAAACAGGGCGGACACCGCCTGTCCGACCCGATGCGGGCACTGAACTACAACCCGGCT
TTAGACAAACACATCGGGCTGATTCTTGAAATGCTTGCCGCCTGACCGCGTCCCCAAACG
GCATTGCCCCGCAACTTCCGCGCCGAGACTTTTGCAGCGGAAAATACGTCCGGCAGAAAA
TCCGCCGTTGCAGGAGCAGGCAGGAAAACATCGGCAACCGCCCCCGAAACGCCGTACCCG
CGCATTGCAAGCGGTTGCCGGAACAGGCGCGTTATCGCGCGGCACAGGCGCATTTCCACC
GATATTTCAGTATAATGCCACCCCCGACCTGCCCCAATCCAAAGGAAACGCGATGAAACT
CATCATTCTCGACCGCGACGGCGTCATCAATCAGGACCGCGACGACTTCGTCAAATCCGT
TGACGAGTGGATACCTGTCGAAGGCAGCATGGATGCGGTGGCATTTCTGACGCAGGCAGG
CTACACCGTCGCCGTTGCCACCAACCAATCCGGCATCGGGCGCAAATATTTTACCGTTCA
AAACCTTACCGAAATGCACGCCAAAATGCACCGCCTCGTCCGTCAGGCAGGCGGCGAAAT
CAACGGCATCTGGTTCTGCCCGCACACCGATGCCGACAACTGCAACTGCCGCAAGCCCAA
ACCGGGTATGATTGAAGACATCATCGGACGCTTCAACGCCCAAGCCTCGGAAACCTGGCT
GGTCGGCGACAGCCTGCGCGATTTGCAGGCAATCGATGCCGTCGGCGGCAAACCCGCGCT
GGTTCTGACCGGAAAAGGCAAAAAAACGCTCTCCCAACACGGACACGAATTGCCCGAACA
CACACAGGTTTTCGATACCCTGCTCGATTTCTCACAATACATCATGCAGGAAAACACCGC
ACCGCAAGCCGACTGAACATACCGCATTCCGACAAGGCAAAACCATGCTCATCATCCGCA
ACCTGATTTACTGGCTGATACTCTGTTCCACCCTGATTTTCCTCTTTCCCTTTATGCTGC
TCGCCTCGCCTTTCCGGGACGGGGCGCACAAGATGGCGCGGGTCTGGGTCGGCATTCTCA
ACTGGTCGCTCAAACACATCGTCGGGCTCAAATACCGCATCATCGGCGCGGAAAACATCC
CCGACCGCCCCGCCGTCATCTGCGCCAAACACCAAAGCGGCTGGGAAACGCTCGCCCTTC
AGGACATTTTTCCGCCGCAGGTTTACGTTGCCAAACGCGAGTTGTTCAAAATCCCCTTTT
TCGGCTGGGGCTTGAAACTGGTCAAAACCATAGGCATAGACCGCAACAACCGCCGCGAAG
CCAACGAGCAGCTCATAAAACAGGGGTTGGTGCGCAAAAACGAAGGCTATTGGATTACCA
TTTTCCCCGAAGGCACGCGCCTTGCGCCCGGAAAACGCGGCAAATACAAACTCGGCGGCG
CGCGCATGGCGAAAATGTTTGAGATGGACATCGTCCCCGTCGCCCTCAACAGCGGCGAAT
TTTGGCCGAAAAACTCCTTTCTGAAATATCCGGGGGAAATCACCGTCGTCATCTGTCCGA
CCATCCCGCACGCAAGCGGCAGCGAAGCCGAATTGATGGAAAAATGCGAACATCTCATCG
AAACGCAACAACCGCTTATTTCCGGCGCAGGCCCGTTTGCCGCCAAAATGCCGTCTGAAA
CCGCATGACCGCCTTTGTCCACACCCTTTCAGACGGCATGGAACTGACCGTCGAAATCAA
GCGCCGTGCCAAGAAAAACCTGATTATCCGCCCCGCCGGCACACATACCGTCCGCATCAG
CGTCCCACCCTGCTTCTCCGTCTCCGCTCTAAACCGCTGGCTGTATGAAAACGAAGCCGT
CCTGCGGCAAACACTGGCGAAAACACCGCCGCCGCAAACTGCCGAAAACCGGCTGCCCGA
ATCCATCCTCTTCCACGGCAGACAGCTTGCCCTCACCGCCCATCAAGACACGCAAATCCT
GCTGATGCCGTCTGAAATCCGTGTTCCCGAAGGCGCACCCGAAAAACAGCTTGCGCTGCT
GCGGGACTTTTTGGAACGGCAGGCGCACAGTTACCTGATTCCCCGCCTCGAACGCCACGC
CCGCACCACACAACTGTTCCCCGCCTCCTCCTCGCTGACCTCTGCCAAAACCTTCTGGGG
CGTGTGCCGCAAAACCACAGGCATACGCTTCAACTGGCGGCTGGTCGGCGCACCGGAATA
CGTTGCCGACTATGTCTGCATACACGAACTCTGCCACCTCGCCCATCCCGACCACAGCCC
CGCCTTTTGGGAACTGACCCGCCGCTTCGCCCCCTACACGCCCAAAGCGAAACAGTGGCT
CAAAATCCACGGCAGGGAACTTTTCGCCTTAGGCTGACGCGGACCGGACCGACCCGCCGC
TTTCAGACGGCATCCGTGCCGGAACAGGCACGCGCCCGCCCGATTCAAACCGCGATGACG
CTTTGCCGCCGGTTCGGGGCAGGATGGCGGCACACACGCCGTCTGCCGCGTTTCATTTCA
CACCGCTCTTCCGAAACCCGAAACCCGCCCGGTCCGACGTGCGGTATGAAACGCTTAAGC
TGACGCGAAGTCTTTTACTGATTTGCCCGCGAAAATGCCGTCTGAAAGGTTTTCGGACGG
CATTTTTTTTGCGTTTCCCAGGATGGCGGCGGATTCGTAAAAGGCGGTCAGGGTGGATTG
TAGGATGGGTTGAGACCTGCCGAATCCGCCGCATCTGCCAAATCTACCGCCGTCATTCCT
ACGAAAGTGGGAATCTAGAACGCGGGGTTAAGAAAACCTGCATCCCGTCATTCCCACGAA
AGTGGGAATCCAGTTTTTTGAGTTTCAGTCATTTCCGATAAATTGCCTTAGCATTGAATG
TCTAGATTCCCGACTGCGCGGGAATGACGAATCCATCCATACGGAAACCTGCACCGCGTC
ATTCCCACGAAAGTGGGAATCCAGGACGAAAAATCTCCAGAAACCGTTTTATCCGATAAG
TTTCCGCACTGACAGACCTAGATTCCCGCCTGCGCGGGAATGACGAATCCATCCATACGG
AAACCTGCATCCCGTCATTCCTACGAACCTACATTCCGTCATTCCCACGAAAGTGGGAAT
CCAGAATCCCAGACTTTCAGATAATCTTTGAATATTGCTGTTGTTCTAAGGTCTAGATTC
CCGCCTGCGCGGGAATGACGGGATTTGAGGTTTCTGTTCGCGTCATTCCCACGAACCTGC
ATCCCGTCATTCCCACGAAAGTGGGAATCTAGTTTTGTCGGTGCGGAAACTTATCGGATA
TAGTGGATTAACAAAAATCAGGACAAGGCGACGAAGCCGCAGACAGTACAAATAGTACGG
AACCGATTCACTTGGTGCTTCAGCACCTGAGAGAATCGTTCTCTTTGAGCTAAAGCGAGG
CAACGCTGTACTGGTTTTTGTTAATCCACTATAAAATGGTTTCTTTAGATTTTACGTCCT
AGATTCCCGCCTGCGCGGGAATGACGATTCGGGCACTCCTGACAGGGTAAATTCACAGGA
TAGCGATTCGTAGCAACTGCATCCCCCCCCCCCAACAACTCCCCAAACAACGCCGCTCGC
CCTGGGCGTTTGCCGTTTCCCTGCAAAATCTGCGATACAATGCAGTCTGAACATTTATCC
GAATCCCAAATCCGATGGATACCGCACAAAAACAACGCTGGGCAATAACCCTATCCTATG
ACGGCAGCCGCTTTTACGGCTGGCAGAAACAGGCTGACGGCGTACCGACCGTTCAGGCGG
CATTGGAAACCGCGCTCGCCCAAATAGCAGGGGAAGCGGTTTCCACCACCGTTGCCGGCA
GGACCGACACCGGCGTGCATGCCACCGCCCAAGTCGTCCACTTCGACACAACTGCCGCCC
GTCCCCAACAGGCATGGGTGCGCGGCGTAAATGCCCACCTGCCCGAAGGCATTGCCGTTT
TGCACGCCCGACAGGTCGCCCCCGAATTTCATGCACGATTTGACGCATACGGACGGCACT
ACCGCTACCTGCTCGAATCCGCCCCCGTCCGTTCCCCCCTGCTCAAAAACAGGGCAGGCT

## Appendix A

GGACACACCTCAAACTCGACATCGGGCAGATGCGGCAGGCTGCCGCCTTATTGGTCGGCG
AACAAGACTTCTCCAGCTTCCGCGCCGCCGAATGCCAAGCAAAATCCCCCGTCAAAACCA
TCTACCGCGCCGACCTTACCCAAAGCTCAGGACTCGTCCGCCTCGATTTGCACGGCAACG
CCTTTTTGCACCACATGGTACGCAACATCATGGGCGCGCTCGTTTATGTCGGCAGCGGCA
GACTCAGCGTCGAAGGCTTCGCCGCACTGATTCAAGAACGCAGCCGCCTCAAAGCCCCGC
CGACCTTCATGCCCGACGGACTTTACCTGACCGGCGTCGACTATCCCGAGGCATACGGCA
TCATCCGCCCCCAAATCCCCGAATGGCTTTAAAACATGCTTGTCGCGGAGATTTTGAAAT
CGGACAAACTGTCAGGCAATCTTTTTCCATGTTGACACTACCTCATCAAGGTACTAACAT
TGTTATTACATAAACAGGTGAATATGGTACGTATATGATTCTCAACATACGCAAAATGGG
AAACTCGCAAGGCGTGATTCTGCCCAAATCATTATTGGGTCAAATAGGGGCAGTAGACAG
CTTGGCTGTTACAGTTGAAAAGGGCAATATTATTTTAAGCTGTCCTACCGTTCGCAGGGG
ATGGGCAGAAGCTGCCGCAATGCTTGTCGAAACCGAGCAGGAGCATTTTTTTTCCGAAAT
TGAAAACGAAGCGGATAAAGAATGGATATGGTAGTACGCGGCGGAATCTATCTGGTCTCC
TTAGACCCGACCGTAGGAAGCGAAATCAAAAAGACACGTCCTTGTGTCGTAGTCTCTCCT
CCTGAAATACACAACTATCTCAAGACTGTGCTGATCGTTCCCATGACGAGCGGAAGCCGT
CCTGCCCCGTTCCGCGTCAATGTCCGCTTTCAGGATAAAGACGGTTTGCTTTTGCCCGAA
CAGATTAGGGCTGTGGATAAAGCCGGATTGGTCAAACATCTTGGCAATTTAGACAACAGT
ACGGCTGAAAAACTGTTTGCAGTATTGCAGGAGATGTTTGCCTGATTGAATAGTCTGAAT
GGATTGTGTTCATTATAGTGGATTAACTTTAAACCAGTACGGTGTTGCCTCGCCTTAGCT
CAAAGAGAACGATTCTCTAAGGTGTTGAAGCACCAAGTGAATCGGTTCCGTACTATTTGT
ACTGTCTGCGGCTTCGTCGCCTTATCCTGATTTTTGTTAATCCACTATAAAGACCGTCGG
GCATCTGCAGCCGTCATTCCCGCGCAGGCGGGAATCTAGAACGTGGAATCTAAAGAAACC
GTTTTACCCGATAAGTTTCCGCACCGACAGACCTAGATTCCCGCCTGCGCGGGAATGACG
GGATTTTAGGTTTCTAATTTTGGTTTTCTGTTTTTGAGGGAATGACGGGATGTAGGTTCG
TAAGAATGACGGGATATAGGTTTCCGTGCGGATGGATTCGTCATTCCCGCGCAGGCGGGA
ATCTAGAACGTGGAATCTAAGAAACCGTTTTATCCGATAAGTTTCCGTGCGGACAAGTTT
GGATTCCCGCCTGCGCGGGAATGACGGGATTTTAGGTTTCTAATTTTGGTTTTCTGTTTT
TGAGGGAATGACGGGATGTAGGTTCGTAGGAATGACGGGATATAGGTTTCCGTGCGGATG
GATTCGTCATTCCCGCGCAGGCGGGAATCTAGACCTTAGAACAACAGCAATATTCAAAGA
TTATCTGAAAGTCCGAGATTCTAGATTCCCGCCTGAGCGGGAATGACGAAAAGTGGCGGG
AATGACGGTTAGCGTTGCCTCGCCTTAGCTCAAAGAGAACGATTCTCTAAGGTGTTGAAG
CACCAAGTGAATCGGTTCCGTACTATTTGTACTGTCTGCGGCTTCGTCGCCTTGTCCTGA
TTTTTGTTAATCCACTCTAAAGACCGTCGGGCATCTGCAGCCGTCATTCCCGCGCAGGCG
GGAATCCAGACCTTAAGGCAGCGGCAATATTCAAAGATTATCTGAAAGTCCGAGATTCTA
GATTCCCGCCTGAGCGGGAATGACGAAAAGTGGCGGGAATGACGGTTAGCGTTGCCTCGC
CTTAGCTCAAAGAGAACGATTCTCTAAGGTGCTGAAGCACCAAGTGAATCGGTTCCGTAC
TATTTGTACTGTCTGCGGCTTCGTCGCCTTGTCCTGATTTTTGTTAATCCACTATCTCCT
GCCGCAGGGGCGGGTTTTGCATCCGCCCGTTCCGAAAGAAACCACGTGCGCGTTTTTTGC
CGTCTTTATAACCCCCGGTTTGCAATGCCCTCCAATACCCTCCCGAGTAAGTGTTGTAAA
AATGCAAATCTTAAAAAATTTAAATAACCATATGTTATAAAACAAAAAATACCCATAATA
TCTCTATCCGCCCTTCAAAATACACATCGAATTCCACACAAAAACAGGCAGAAGTTTGTT
TTTTCAGACAGGAACATCTATAGTTTCAGACATGGAATCGCCGAAAACGTCGGCGGTAAA
TGCAAAGCTAAGCGGCTTGGAAAGCCCGGCCGGCTTAAATTTCTTAACCAAAAAAGGAAT
ACAGCAATGAAAAAATCCCTGATTGCCCTGACTTTGGCAGCCCTTCCTGTTGCAGCAATG
GCTGACGTTACCCTGTACGGCACCATCAAAGCCGGCGTAGAAACTTCCCGCTCTGTATTT
CACCAGAACGGCCAAGTTACTGAAGTTACAACCGCTACCGGCATCGTTGATTTGGGTTCG
AAAATCGGCTTCAAAGGCCAAGAAGACCTCGGTAACGGCCTGAAAGCCATTTGGCAGGTT
GAGCAAAAAGCATCTATCGCCGGTACTGACTCCGGTTGGGGCAACCGCCAATCCTTCATC
GGCTTGAAAGGCGGCTTCGGTAAATTGCGCGTCGGTCGTTTGAACAGCGTCCTGAAAGAC
~~ACCGGCGACATCAATCCTTGGGATAGCAAAAGCGACTATTTGGGTGTAAACAAAATTGCC~~
GAACCCGAGGCACGCCTCATTTCCGTACGCTACGATTCTCCCGAATTTGCCGGCCTCAGC
GGCAGCGTACAATACGCGCTTAACGACAATGCAGGCAGACATAACAGCGAATCTTACCAC
GCCGGCTTCAACTACAAAAACGGTGGCTTCTTCGTGCAATATGGCGGTGCCTATAAAAGA
CATCATCAAGTGCAAGAGGGCTTGAATATTGAGAAATACCAGATTCACCGTTTGGTCAGC
GGTTACGACAATGATGCCCTGTACGCTTCCGTAGCCGTACAGCAACAAGACGCGAAACTG
ACTGATGCTTCCAATTCGCACAACTCTCAAACCGAAGTTGCCGCTACCTTGGCATACCGC
TTCGGCAACGTAACGCCCCGAGTTTCTTACGCCCACGGCTTCAAAGGTTTGGTTGATGAT
GCAGACATAGGCAACGAATACGACCAAGTGGTTGTCGGTGCGGAATACGACTTCTCCAAA
CGCACTTCTGCCTTGGTTTCTGCCGGTTGGTTGCAAGAAGGCAAAGGCGAAAACAAATTC
GTAGCGACTGCCGGCGGTGTCGGTCTGCGCCACAAATTCTAATCTGCAAAGATTGGTATC
AACAAAAAGCCTGTCGCCAGACAGGCTTTTTTCTGTTTGGCTTTTTCCTGTTTTCTGTTT
GGCTTTTTCCTGTTTTCTGTTTCGCTGTTTTCTGTTTCGCTGTTTTCTGTTTCGCTGTTT
TCTGTTTCGCTGTTTTCTGTTTCGCTGTTTTCTGTTTCGCTGTTTTCTGTTTGGCTTTTT
TCTGTTTGGCTTTTTTCTGTTTGGCTTTTTCCTGTTTTTAGTCTTTTTTATTCAATGTCA
AAATATGCCGTCATTCCCGCGCAGGCGGGAATCTAGTGCGTTGAGTTTCAGCTATTTAGA
ATAAATTTTGAAACTTTAATCCCGTCATTCCCACGAAAGTGGGAATCCAGGACGCAAAAT
CTCAAGAAACCGTTTTACCCGATAAGTTTCCGCACCGACAGACCTAGATTCCCGCCTGCG
CGGGAATGACGGGATTTGAGGTTGCGGCATTTATCGGGAGCAACAGAATCCGCTCTGCCG
TCATTCCCACGAAAGTGGGAATCTAGTTCGTTCGGTTTCGCTTGTTTTAAGTTTCGGGTA
ACTTCCACTTCGTCATTCCCGCGCAGGCGGGAATCCAGTGCGTTGAGTTTCAGCTATTTA
GAATAAATTTTGAAACTTTAATCCCGTCATTCCCACGAAAGTGGGAATCTAGTTTTTTGA
GTTTCAGTCATTCCCGATAAATTGCCTTAGCATTGAATGTCTAGATTCCCGCCTGCGCGG
GAATGACGGCGGAAAGATTCTATTTTTCCCGATAATCGCCCACAATCTCAAATTCCTTCA
TTCTCTCAAAAACAAAATCAGAATCCTAAATCCCATCATCCCCATCTATGTGAATATAAA
AATTTTAAAAATTATAGTGGATTAACAAAAACCAGTACGGCGTTGCCTCGCCTTAGCTCA

## Appendix A

AAGAGAACGATTCTCTAAGGTGCTGAAGCACCAAGTGAATCGGTTCCGTACTATCTGTAC
TGTCTGCGGCTTCGTCGCCTTGTCCTGATTTTTGTTAATCCACTATATTTCACAAGCGA
AAGAATGCCGTCTGAAGCCTTTTTTCCGGTTTTCAGACGGCATTTTTTGCTTGACGTTTA
ACTGTAAATCTTCGCGCCTTTTTTGACGAACTCGACCGCTTTTTCCTCCATGCCCTGCCG
TTGGGCTTTTTGCTTGTCGGCGTAGTCGCGCACTTCCTGCGTGATTTTCATCGAGCAGAA
TTTGGGGCCGCACATCGAGCAGAAGTGGGCGATTTTCGCGCCTTCGGCAGGCAGGGTTTC
GTCGTGGAAGCTCTCGGCACGTTCAGGGTCGAGGCTTAAGCGAAATTGGTCGCGCCAGCG
GAACTCGAAACGCGCTTTGCTCAGGGCGTTGTCACGTAATTGTGCGCCCGGCCAGCCTTT
GGCGAGATCGGCGGCGTGGGCGGCGAGTTTGTAGGTGATGATGCCGGTGCGCACGTCTTC
TTTGTCGGGCAGCCCCAAATGCTCTTTCGGGGTAACGTAACAAAGCATCGCCGTGCCGTA
CCAGCCGATATTGGCCGCGCCTATGCCCGAGGTGATGTGGTCGTAGCCGGGTGCGATGTC
GGTAACGAGCGGGCCGAGCGTGTAAAAAGGTGCTTCAAAGCAGTGTTGCAGCTCTTCGGT
CATGTTTTCTTTGACGCGTTGCAGCGGCACATGGCCGGGGCCTTCGATCATGACTTGTAC
GTCATGTTTCCACGCTTTATCGGTCAATTCGCCCAAGGTGTGCAGTTCGGCGAATTGGGA
TTCGTCGTTGGCATCGGCAATGCAGCCGGGGCGCAGGCCGTCGCCGAGGCTGAACGATAC
GTCATACGCTTTCATAATTTCGCAGATTTCGTCGAAATGCGTGTAGAGGAAATTTTCCCG
ATGATGTGCCAAACACCATTTCGCCATAATCGAACCGCCGCGCGAGACGATGCCGGTGAG
GCGGTTGGCGGTCATCGGCACATAACGCAGCAACACGCCCGCGTGTATGGTGAAATAGTC
CACGCCTTGCTCCGCCTGTTCGATGAGGGTGTCGCGGAACAAATCCCAAGTCAAATCTTC
GGCGATGCCGCCGGTTTTTTCCAACGCTTGGTAAATCGGCACGGTGCCGATGGGGACGGG
CGCGTTGCGGATAATCCATTCGCGCGTTTCATGGATGTGCGCGCCGGTGGACAAATCCAT
AATCGTGTCCGCGCCCCAACGCAGCGACCACACCATTTTTTCGACTTCTTCGGTCAGGCT
GGAGGTGACGGCGGAGTTGCCCAAGTTGCCGTTGATTTTGACACGAAAGTTGCGGCCGAT
AATCATCGGTTCGAGTTCGGGGTGGTTGATGTTGGCGGGAATAATCGCGCGTCCGGCGGC
GATTTCTTGGCGCACGAATTCGGGCGTGATTTGGTCGGGATGGGTCGGGATGTTCGCACC
GAAACTTTGCCCCGCGTGCTGTTCCAAGAGTTTGGCGTATTCCGGTTTTTGGGACAATTC
GTCTAATTTTAAACGTTCGCGTATGGCGACAAACTCCATTTCGGGCGTGATAATGCCTTG
GCGCGCGTAGTGAAGCTGGGTTACGTTGCTGCCGCTTTTCGCGCGGCGCGGGCGGGTGAT
TTGGTTGAAACGCAGATGGGCGGTTTTCGGATCGTGTGCGCGTTCGATGCCGTATTCGCT
GGAGAGCTTGGGCAGGATTTCGGTATCGCCGCGTTCGTCCAGCCACGCGGTGCGGATGTG
CGGCAGACCTTGTTTCAGGTCGATATGCGCCGCCGGGTCGCCGTACACGCCGCTGGTGTC
GTAGACGGGAATCGGCGGATTGGCTTCCGTACCTTGCGCCGTGTAGGTGTCGTCCTGACG
GATTTCGCGCAAAGGCACGCGGATGTCGTCGCGGCTGCCTTGCAGATACACGCGTTCCGA
GTTCGGATATTTAAAGCAGATGCCGATGTCTTCGCTCAAGTCGGCAAGCTCGCGCGCTTC
GTTGCCGGAAGTTTTGGCGGTTTTTTTTGGCGTAGTCATAAAAAAATGCTCCTGTTTTCT
CGTTTAGAATTAAAGAAACAGGAGCGTTTTGCGTTTTCAGACGGCATTTGAAAACCAATG
CCGTCTGAAAAGCAGAATCCGTGAAAACTCCCCACGCAGGTATTATCCCGATCGGGTGTA
AAGGGTATTTCTCAGCCGCCTAAACATCAGGCAGCACCCCTGTTTCAATGTTAACCAAAA
TTAAATCACGAACATGAACTTTTGTAAAGAAAATAATATTTCAAATCAGGCATAAACCGC
CGGACGGCAAAATTTTATGATTTTTCGCGGAAGTAATGTTTGACAACATAAAAAATCTGC
CGTATAGTTTCATCTTCTGACGCGGGATGGAGCAGCATGGTAGCTCGTCGGGCTCATAAC
CCGAAGGTCGTAGGTTCGAATCCTGCTCCCGCAACCAAATATCAAACCCCTCGGTTCAAT
GCCGAGGGGTTTTGTTTTGCCTGTTTCCTGTTTCCTGTTTCCTGCCGCCTCCGTTTTTTG
CCGGATTTTCCTTCCGGCCGCAATATCGGAACGGCAGACCGCCGTCTGTTTGCGGTTGCA
AATTCAGGCAGTTTGGCTACAATCTTCCGCATTGTCTTCAAGAAAGCCAACCATGCCGAC
CGTCCGTTTTACCGAATCCGTCAGCAAACAAGACCTTGATGCTCTGTTCGAGTGGGCAAA
AGCAAGTTACGGTGCAGAAAGTTGCTGGAAAACGCTGTATCTGAACGGTCTGCCTTTGGG
CAACCTGTCGCCGGAATGGGTGGAACGCGTCAAAAAAGACTGGGAGGCAGGCTGCTCGGA
GTCTTCAGACGGCATTTTTCTGAATGCGGACGGCTGGCCTGATATGGGCGGACGCTTACA
GCACCTCGCCCTCGGTTGGCACTGTGCGGGGCTGTTGGACGGCTGGCGCAACGAGTGTTT
CGACCTGACCGACGGCGGCGGCAACCCCTTGTTCACGCTCGAACGCGCCGCTTTCCGTCC
TTTCGGACTGCTCAGCCGCGCCGTCCATCTCAACGGTCTGACCGAATCGGACGGCCGATG
GCATTTCTGGATAGGCAGGCGCAGTCCGCACAAAGCAGTCGATCCCAACAAACTCGACAA
TACTGCCGCCGGCGGTGTTTCCGGCGGCGAAATGCCGTCTGAAGCCGTGTGTCGCGAAAG
CAGCGAAGAAGCCGGTTTGGATAAAACGCTGCTTCCGCTCATCCGCCCGGTATCGCAGCT
GCACAGCCTGCGCTCCGTCAGCCGGGGTGTACACAATGAAATCCTGTATGTATTCGATGC
CGTCCTGCCCGAAACCTTCCTGCCTGAAAATCAGGATGGCGAAGTGGCGGGTTTTGAGAA
AATGGACATCGGCGGTCTGTTGGATGCCATGTTGTCGGGAAACATGATGCACGACGCGCA
ACTGGTTACGCTGGACGCGTTTTGCCGTTACGGTCTGATTGATGCCGCCCATCCGCTGTC
CGAGTGGCTGGACGGCATACGTTTATAGGATGCGCCATGCTTGAACTGAACGGACTCTGC
AAACGCTTCGGCAATAAAACCGTCGCCGACAACATCTGCCTGACTGTCGGGCGCGGCAAA
ATACTCGCCGTTTTGGGGCGGTCGGGCTGCGGAAAATCCACCCTGCTGAATATAATTGCG
GGGATTGTCCGGCCGGACGGCGGGGAAATATGGCTGAACGGAGAAAACATTACCCGTATG
CCGCCCGAAAAACGCCGTATCTCGCTGATGTTTCAAGATTACGCGCTGTTTCCCCATATG
AGTGCGCTGGAAAATGCGGCATTCGGTTTGAAAATGCAAAAAATGCCGAAAGCCGAAGCC
GAACGCCTCGCCATGGCGGCACTTGCCGAAGTCGGACTGGAAAACGAGGCGCACCGCAAG
CCTGAAAAACTTTCCGGAGGCGAGAAGCAACGGCTGGCGTTGGCGCGCGCTTTGGTTGTC
CGCCCTTCCCTGCTGCTGTTGGACGAATCGTTTTCCAGTTTGGACACGCATTTGCGCGGC
ACGCTGCGCCGTATGACTGCCGAACGTATCCGAAACGGCGGCATCCCTGCCGTTTTGGTA
ACGCATTCGCCCGAAGAAGCCTGTACGACGGCAGACGAAATCGCCGTGATGCATAAAGGG
AGGATTCTACAATACGGTACGCCCGAAACATTGGTCAAAACACCATCCTGCGTGCAGGTC
GCCCGACTGATGGGTTTGCCCAATACCGACGATAACCGCCATATTCCGCAACATGCGGTG
CGTTTCGACCAAGACGGCATGGAGTGCCGCGTATTATCCCGTACCTGTTTGCCCGAATCG
TTCAGCCTGTCCGTCCTCCATCCGGAACACGGCATCCTGTGGCTGAACCTCGATATGCGG
CACGCCGGGGCGGTATCGGGCAAGGATACGGTACGCATCCATATCGAAGAACGGGAAATC

## Appendix A

GTCCGCTTCCGCTGATGCTTCTTAAAAACAAAATGCCGTCTGAAAACCTTTCAGACGGCA
TTTTTTTACCAAAGCAGCCATATTTTTTTATCAGGGCTGCAAAATTTTATCCGAAACAAC
AACAATCTTTTCATCGTCATTCCCGCGCAGGCGGGAATCTAGAACGTAAAATCTAAAGAA
ACCGTTTTTCCCGATAAGTTTCCGTGCCGACAGACCTAGATTCCCGCCTGCGCGGGAATG
ACGGATTTTAGGTTTCTGATTTTGGTTTTCTGTTTTTGAGGGAATGACGAGACTTGAGAT
GGCGGCATTTATCGGGAGCAACTGAAACCACCCTGCCGTCATTCCCGCAAAAGCGGGAAT
CTAGAACCCAACACGGCAAAAATTTATCCGAAGCGACAACAATCTTTTCATCGTCATTCC
CGCGCAGGCGGGAATCCAGAACGTAAAATCTAAAGAAACCGTTTTTCCCGACAAGTTTCT
GTGCCGACAGACCTAGATTCCCGCCTGCGCGGGAATGACGGGATTTTAGGTTTCTGATTT
TGGTTTTCTGTTTTTGAGGGAATGACGAGACTTGAGATGGCGGCATTTATCGGGAGCAAC
TGAAACCACCCTGCCGTCATTCCCGCAAAAGCGGGAATCTAGAACCCAACGCGGCAAAAA
TTTATCCGAAGCGACAACAATCTGAGACCTTTGCAAAATTCCTTTCCCTCACAACAGCCG
AAACCCAAACACAGGTTTTCGTCTATTTTCGCCCCAAATACCTCCTAATTCTACCCAAAT
ACCCCCTTAATCCTCCCCGGATACCCGATAATCAGGCATCCGGTCGCCTTTTAGGCGGCA
GCGGGCGCACTTAGCCTGTTGGCGGCTTTCAACAGGTTCAAACACATCGCCTTCAGATGG
CTTTGCGCACTCACTTTAATCAGTCCGAAATAGGCTGCCCGGGCGTAGCGGAATTTACGG
TGCAGCGTACCGAAGCTCTGTTCGACCACATAACGGGTCTTCGACAAATATCGGTTGCGT
TTGGTTTGCGCCTCCGTCAGCGGACGGTTGCGGCAGGCTTTGCGCATAATATAGTGGATT
AAATTTAAACCAGTACGGCGTTGCCTCGCCTTGCCGTACTATCTGTACTGTCTGCGGCTT
CGTCGCCTTGTCCTGATTTAAATTTAATCTACTATAATGTGCAGTTTCTCGATATAGCCT
TCCGCATCGGTGCGGGTATGTTGTTTGTAACCGAGTTTGTAGAGGCCGTTTTTCTTGATC
CAACGCGCATCGCTGTCCTTACTCCGTGTGGTTTGGCCGCTGACTTGTCCTTCTTCATCG
ACTTCTATGGCCTGACGCTGTTTGCCGTCGGCGGTCTGAATAATGGTGGCGTCAATGACG
GCGGCGGATGCTTTCTCTACTTTTAAACCTTTTTCGGTCAGTTGGCGGTTGATCAGTTTG
AGCAATTCGGACAGGGTGTCGTCTTGCGCCAGCCAGTTGCGGTAGCGGCATAAGGTGCTG
TAATCGGGGATGCTCAGTTCGTCGAAACGGCAAAACAGGTTGAAGTCGATGCGGGTAATG
AGGCTGTGTTCGAGTTCGGGATCGGAGAGGCTGTGCCATTGTCCGAGCAGGACGGCTTTG
AACATGGACAGCAGCGGATAGGCGGGACGGCCGCGGTGGTCTCGAAGGTAACGGGTTTTT
TGACGGTTCAGGTATTGTTCGATCGGCTGCCAATCAATCACTTGATCCAACTTCAATAGC
GGGAAGCGGTTGATGTGTTTGGCAATCATGGCTTGTGCGGTTTGCTGGAAGAAGGTGCTC
ATGGAAAATCTCCTAAATGTCTTGGTGGAATTTAGGGGATTTTGCAAAGTTTTCAACAA
GTTTCCGCACCGACAAACCTAGATTCCCGCCTGCGCGGGAATGACGGGATTTTAGGTTTC
TGATTTCGGTTTTCTGTTTTAAGGGAATGACGAGACTTGAGATGGCGGCATTTATCGGGA
GCAACAGAAACCACTCTGCCGTCATTCCCGCGAAAGCGGGAATCTAGAACCCAACGCGAC
AAAAATTTATCCGAAGCGACAACAATCTTTTCATCGTCATTCCCGCGCAGGCGGGAATCT
AGAACGTAAAATCTAAAGAAACCGTTTTTCCCGACAAGTTTCTGTGCCGACAGACCTAGA
TTCCCGCCTGCGCGGGAATGACGGGATTTTAGGTTTCTGATTTCGGTTTTCTGTTTTAAG
GGAATGACGAGACTTGAGATGGCGGCATTTATCGAGAGCAACTGAAACCACTCTGCCGTC
ATTCCCGCGAAAGCGGGAATCTAGAACCCAACACGGCAAAAATTTATCCGAAGCGACAAC
AATCTTTTCATCGTCATTCCCGCGCAGGCGGGAATCTAGAACGTAAAATCTAAAGAAACC
GTTTTTCCCGATAAGTTTCCGTGCCGACAAACCTAGATTCCCGCCTGCGCGGGAATGACG
GATTTTAGGTTTCTGATTTTGGTTTTCTGTTTTTGAGGGAATGGCCGATTTTGGGTTTCT
GTTTCGGTTTTCTATTTTGCAAGAATGGCAAAATTTCAGATTGCGGGCATTGTTAAGTAT
TTCTATTTTTTACCTGCCGTATTTATTTCCGCCCCTTGAAGTCGGCTTCTTCCTCGACAG
ACACGCTGTTCATCTGTTTGATCAGCTTTTCCGACTTCTCTTCGTCTTCGCAGCGGATGA
CTTTCACAATATCACTTTCGAGCTGTCCGACATTGCTGTGCAGAATGATGTTTTTGACGG
GCAGGATGTTGTTGGGGTTCATGGAAAAACGGCGCAGCCCCATACCCAATAAAACGCGGG
TAAACGCGGTATCGCCCGCCATCTCGCCGCATACGGATACGTCTTTGTCCATGCGGTTGG
CGGTACGGATGACGTGTTGCAGCATTTTCAGCACGGCGGGATGGCCGGGCTGGTAGAGGT
~~GGCTGACGCTGTCGTCGCCGCGATCGACGGACAAGATGTATTGAATCAGGTCGTTGGTAC~~
CGACGGAGATGAAATCGACCAGTTTCAAAATACTGCCGACGGTCAGCGCGGCAGACGGAA
TTTCAATCATACAGCCGATGCCGACTTTACCGAAGGCATCGCCGCGTTCGGCAAGCTGGC
GTTGCGCGGTGTCGAGGTGGATGAGGCACTGGCGCACTTCGGATACGGAGGTAATCATCG
GCCACATCATCCGCACGGGGCCGTGTACCGCCGCACGGAGGATGGCGCGCATCTGGGTGC
GGAACATGACCGGTTCGGCAAGGCACAGGCGGATGCCGGTCATGCCCAGCGCGGGGTTGA
GGCTGCCGTTGGGCGTGCTGTTTTTCCCGAACCAGCGCGGGTTTTTGTCCACACCTAAAT
CGACTGTCCGTATCGTTACGCTTTTGCCTTTCATTTTTTTGACAATCGCGCTGTACACTT
CGTACTGCTCGTCTTCAGACGGCATCGTATCGCGGTTCAGGTAAAGAAACTCGCTGCGGA
ACAGCCCGATGCCGTCTGCGCCGAGGTTGTGCAGCGGTTTCACGTCTTCGGCGGATTCTA
TATTGCCCACAAGCTCGATGCAGACCCCGTCGGCGGTGGCGGCGGCGGTTTTTTTGAGCT
TGTTCAAATCGCGTTTGTGGCTGCGGTATTCGCGGGCACGGCGGCGGTATTCGTTCAACA
CCGACTCATCCGGCGCGATAATCAACACGCCGTTGATACCGTCCACAATGACCGTTTCGC
CTTCGGTAATCAGTTTGCGCGCGTTGTGCAGCCCGACGACGGACGGGATGTCCAAGCTCC
TGCCCAAAATCGCCGTATGCCCGGTGGGGCCGCCGGCATCGGTAACGAAGGCGGCAATGC
GCTGCTCTTTAAACAAAACCGTGTCGGCGGGCGAAAGGTCGTTTGCAATCAGAACGGTTT
CGTCAAACAGGTTGTCGGCAACTTCCAACTCGTTGCCCTGCCCGATCAGGTTGTTGTGGA
TGCGGCGGACGACTTGCAGCATATCCTGCTTGCGTTCGCGCAAATAGGCATCGTCCATAT
TGTCGAATTGGGCGGCGAGTTTGTCGCTCTGCTGCTTCAATGCCCACTCGGCGTTGATTT
TTTGTTCCCTTAAAATATCGACGGGTTCGCGCGACAAGGTAACATCGGTCAAGAGCATCA
GGTGTAGCGAGATGAACGCGCCCAACTCGGTCGGGGCGTTTTCGGGAATCGCGCTGCGGA
GCTGTTCCAACTCTTTGCGCGTGGCTTTGACGGCGGCATCGAAACGTTCGGCTTCGGCAT
CGGTGTCCGCCTCCGCAACATCATACTGCGGCACTTCCTCCGTACCGCGCGCAATCAGGT
GGGCGCAACCGACGGCAATGCCTTTGCCCGCCGCCACGCCGTGCAGCACGATACTCATTA
TTCGCCCTCGCCGAAGTAGCCGTTGATTAAGTCGGTCAGGGCGCGCATCGCTTCCGCCTC
GTCCGCGCCGTCCGTCTCCAGTTCGATGACCGTACCCTTGGCGGCGGCGAGCATCATCAG

## Appendix A

CCCCATAATGCTTTTGCCGTTGACGCGGCTGTCGTTTTTCGTAACCCAGACTTCGCTTTT
GAATTGGGACGCGGTTTGGGTGAACTTGTTGGACGCGCGGGCGTGGAGTCCGAGTTTGTT
GATGATTTCGATGGATTGTTTGAGCATTTCGATTCCCGTGTTATGTATATCGGCAGCAGA
CGCCGTTTAAAATGTTTTCCTGCCCTGCCGCTTCTTCAGACGGCATCGCCGCTGCGCCGG
CACACCAAATCTTCGGGCGCGGACGTGATGGCGAAAATGCCTTTTACCGCCGCCTCCCTG
ACGCATTCGGTAAAGGCGGCAAGGTCTTCCGCCGCCGGCGAATATTGGACGGCCTTAACC
ATCATCGGCGCGTTCAGCCCGGTCAAAATCGCCGATTTGTTTTCGCGCACGAGGCGGCGG
GCGGCATTGCAGGGGGTCGCACCGAAAATATCGGTCATAATCAGCACGCCGTCGTTGTCG
GGAAATTCCTGAAGCGCGGCAATGGCGTTGTTGTTGATGTCGTCTTGGTCTTCCGTCGGC
TGCACGCCGAGTATGCGGACGTTTTCAGGCAGTCCGCCCGGAAAAAAATGATGCGCCAGC
TTGCGGTAGGCTTCGCCTATGGTTTCGTGTGTGATGATTAAAAGCCCTATCATATTATGC
GTCCTGTTCCTCATTATCCTGCCGGCGTATGGGCGCGATGCCGTCTGAACAGCCTTCAGA
CGGCATCGCGCCCTTATTTTCCGCCCAATGCGTAAATCTCGCCCAGATTGCGCCAGCAGC
CCGCCGCATCCATGCCGTAACCGAAAACATAACGGTTCGGCACATCCAGTCCGACATAAT
CGGCTCGGATAGGCTTGGGTTTGTCAATCAGCTTGTTGGCGAACACCGCCGCACGGCAGC
TTGCCGCACCCATTTCCAAAAGTTTGGCTTGAATGGCGGACATCGTATGCCCTTCGTCCA
AAATATCGTCCAGCACGACGACGTGCCTGCCCCGGATTTGTTCCGCATCGGGCATACGCT
TCCAGTTGAACGCGCCGCCCTCCAGCTTGTCGCCGTAACGGGAAACGTGAACATAATCAA
AATCTAAGGGAAAACGCAACAGCGGCAGCAACTGCCCCGTAAACACCACCGCGCCGCCCA
TCACGGGCAGCAGCAGCGGATATTTGCCGCCCAAATCACGCGTAATCTCGTCCGCCACTT
TTTGCAGTGCGGCACGGCATTGGCCTTGGTCGAACAAAAGATCGGCGTTTTCAAGCATCG
CCTGTGTTTCAAGGCGTTTGGTTTCTAAATCGGTCATATGTCGGAATCGGTCGGTAAAAG
GAAAATTATAAACCAAAGTATCGGATGCCGTCTGAACTGTCCTGCTCAGCGGTCGGTACG
CACGCGCACAAATGTGGCAAATTTCGGCGTGCCTTTCCGCGTAAAGCCACGGTAACGGTA
GGTAATCAGTGTGCCGATTTTGGGCGGGTTGTCGCGGTCTTTATCTTTGAAACCGCTGCC
GATGCGGAATTCGCCGTGCCGGTTTTTGCAGCCGACCGCGCCCAGCCGTCCGGCGTTTCG
CCCTTTGCCCTCATAGTGCCGCGTTACCGTGCATTCGTCGTCGTATTGGCTTTTCAGCTT
CAATAATTGGCTGCTCCTGCCGCCGCTGTAACGGGATTCGGGCTGACGCAGCATCACGCC
TTCGCCGCCCTGCGCTTCGATTTGTTTTAAAAAGTCCATCGCGTGCTGCCGGTCGCGCAC
TTTGATTTGCGGGATGATGGTAATCGGCGCGTTCGGATGCGTTTTCAGCCACTGCGTTGC
GACTGCCAAACGTTGGTAGAGGTTGCCCTGCGCCTTGGGTACATCGAAAACGTGCAGGCG
GATGCCGCGCCAGTCTGAAGAAACAGAACGCACGGTAGCGGAAATCTGCTCGAACTGACC
ACGTCCGCTATACAATTCGCCGTCCAAAGGATAAGGCGGAAACTGAGCGGTAAAACCTTT
GGGCGGAGCAAACGCGTAGCCCTGACGGCTCATCAGGTGCTTTCCGTCCCAATAGGCGCG
CACGCCGTCGAGTTTCTCGCTCATCGCCCAGCCGGCAATATCCTGCCCTTTGTATTCCTG
CGCCAGCATCAAATCCGCCGCGCCTGCTGATGCAGGGATGAAAACCGCCGTAAAAATCGG
TATGATGCCGCCGATTGTCTTCTTAATCATCTGATTCCCCCAATATCAAAACGGGCGGCA
AACCGCCATAAAACAAACGGCAAACCCGATGCCGTCTGAAAAACCGTTTAGGAACACGCC
GATGACCCTACGTTACGAAATCTTCCCCGTTACCCCCTTCCGCCAAAACTGCACCCTGAT
TTGGGACGACGAAAGCGGCGAAGCCGTCCTGACCGATGTCGGCGGCGACGTGCCGTTCCT
GCTGCAAGCGTTGGCAAACCGCAAACTTACGCTCACGGCAATCTGGCTGACGCACGGCCA
TCTCGATCACGCGGGCGGCGTGGTCGAAATGTTGAAAACGCATAAAGTCCCTGTCCTCGG
GCCGCATCCGGACGATGAATTCCTGCTCCAATCGCTGCCGCAAACCACCGCGCAATACGG
ATTTCCCGTCTCGCCCGCCTTTGCGCCGAACCGTTGGCTCGAAGAAGGCGAAACGCTCAC
GGTCGGACGCTATGCCTTTCAAGTGCTGCATATTCCGGGCCATACGCCGGGACATATCGT
CTTTTATTGTGCCGAGGCGGAATTGCTGATTGCGGGCGACGTGCTGTTTTACGAAACCAT
AGGCAGAACCGATTTTCCGCGCGGCAACCACGCCGACTTAATCAATAATATCCGCAACAA
ATTATTCACCCTTCCCGAAACCGTGCAAGTTGTCGCCGGACACGGGCGTATGACTTCCAT
CGGACACGAAAAGCGGCACAATCCGTTTTTCTAACCGCCTTCCCTACGGTCTTCAGACGG
CATCATCTGCACTGATGCCGTCTGAAACACAAAAGGCTCAGACAACCGCCGCCTTGCCGG
TGTACCTCGCCGGACAAGGCTTTGGTAAGTACTTCAAACAAACCCAAAATCAGAACCAGT
ACAGTTACGCCGCGCTTTCGGCATTCCCGCCCCGGCTGAAACAATATTTTTCCGCACAAG
TCAGACTGCTTCATCTTCTGCCGCGTATTCCAAAGATTCCGACAACGCCGTTGTTTCATT
GGAACGCTCGACCAAATCCCGTTGAAGTTGTTTGCCTGCCTTCACGCCCAACAGACGCAG
TTTCTCGGCGCGTCCGACCAGATTCCCGCGCCCTTCGGCAAGTTGCTTGAATGCCGTCTG
AAAACTGCTTTGCGCCTGATCGATGCCTTTGCCGACGCTTTCGAGCGTCTGTACGAAGCC
GACAAACTTGTCGTACAGCTTGCCGCCTTCGTCCGCAATCGCCAGTGCGTTCTGATTTTG
CTGTTCGTTGCGCCAAATATTCGCCACCGTCCTCAAAGTCGCCAGCAGCGTACTGGGGCC
GACCAGCATAATCCGTTTGTCGAAACACTCTTGGAACAAGCCCGCGTCATTCTGCAACGC
CAACAGGTAGGCCGGTTCGACAGGGATAAACATAAAGACGAAATCCAATGTGTTCACACC
TTCCAAATCGGTGTAATCCTTCAGCGACAAGCCTTTCATGTGTGCACGGATGCTGGCAAC
GTGTGCCGCCAGTTCGCGTGCCGCCGTATCCGCATCCGCCGCCTGCGTGTAGCGCACATA
AGCTGTCAGCGAGACCTTGGAATCAATCACAATCTGCTTGTTGTCGGGCAGGTTGACCAA
AACGTCGGGCTGGAGGCGGCGCGTGCCGCCGTCTTCCTCTTTTCGGACGGATGCCGCCTG
AACCACATATTCCCGCCCTTTCTGAAGGCCGGAATTTTCCAAAACCGTTTCCAGAATCAT
CTCGCCCCAATTGCCCTGAACCTTATTCTGCGTACCGGTCAGCGCGTTGGTCAGGGCCTT
TGCCTCGCTGTGCAGCTGCGCGTTCAACCCCTGAAGCCGTTTCAATTCGTTTTCCAACGT
CAGCCGCTCGCGCGATTCTTTATCATAGGTTTGCTTGACCAACTCGCCGAAACCGTGGAT
GCGTTCGTTTAGCGGGTTCAAAACCTGATGGAGCTGCTCGCGGTTCTGCTCGGTAAAACG
GCGGCTTTTTTCTTCCAAAATCGTGTTGGCAAGATTTTGAAACTGATCGCTCAAACTTTT
GCGCGCCTCGCCCAGCAAGGACAGCTTCTCTTCAGAAGCAAGGCGTTCCTGTTCGATTTG
CGTTGCCAAACGTTCGTTTTCAACCGCCAAACCCTGTGCCTTTTCCTGCAACTCGGTATG
CGACTGCCTCAACCGCTCCGCTTCCGCCTCTTTTTCCTGCAAATGGGCAATCTGTTTTTC
GGCTGCGGCAAAACGGTTGCCGACATCGGAAAGGTCGTTTTGCACGTCGCGGACAGTTTG
GCGGCTTTCTTCCAAATCGGTTTCGATTCTTTGGCGGATTTGGCGTTCCAAGGCATATTG

## Appendix A

GTCGGCAAACGCCTTCCGTTCCTGTCCGAGCTGCGTTGCCAAACGTTCGTTTTCAACCGC
CAAACCCTGTGCCTTTTCCTGCAACTCGATATACGACTGCTTCAGCCGCGCCGACTCCGC
CTCTTTTTCCTGCAAATGGGCAATCTGCTTTTCGGCTGCGGAAAAACGGTTGCCCAAAGC
ATAATTTTCGTCCTGCAAATGCCGGTATTTCCCGTCCAACACCGCCAATTCCGACACGGT
TTTGCCGTGTGCCTGTTCGACAAAATCACATCTTGCCGCCTTTTCCGCCAGGTGCGCGTT
CAAACCGGCAAACTCGCCCTGAAACCGGCCCTTCATCAGCAACCATGTAAACAACACGCC
CGACACCAACGCCGCCAAAGGCAGCAAAACAGTCATCAGTTCCATCAATTATCCTAATAT
TCAAACATTTTCACACCGGACAAAACCGCCGCTTATTCCGATTCTACCTGTTTGTTCGAC
ATAGCTCCAAAAAATATAGCGGATTGGCTTTAAACCTGTTCGACATCGCCTTACCATGCT
GCTTGCGGTTTCAGACCTTTTCCTAATTCAATATCAATCTGCCACAAACCCTGATTAAGT
TCCCGATGTCTGACATTTTTAGAATGATGCCGTCTGAAATGTTGCAGCTATGTTCAGACG
GCATACGGATTCAGGCTTTTCAAACGGCAGGCAAAATGAAAAAAGGGCAAACCCTAAAGG
ATTTGCCCTTTTGTTCCAAACGCTTAGTGTACGTCTTTCCAATATTCTTTATTTAGGAAG
TAAGCCAAAGGCAGCATAACCGCAAATAGGAAAATCATCACGACATAGCCTATACGTTTG
CGTTGCAGTTGTGCAGGTTCGCCCATGTACACAAGGTAATTGACCAAATCGCGTACATAT
GCGTCGTACTCTTTTTGGATCACTTTGCCGTTAGGCAGGCGGCGGCTGTGCAAACCGGTA
GATTCCCAATACAGCTTAGGCTTCATCTCGCCGTGTTCGTCTTTTACCATAACCGGCTGA
CCTTTGGCATCCAACTCAACGGCTTGAACACCTTGTTGCTCCCACAACGGGTGGGGCATA
CCGACTTTATCGAATACAGTATTGTTCCAGCCGCTCGGACGGGTCGGATCTTTATAGAAG
CCGCGCATATAGGCGTAAAGGTAGTCTGCACCTTTGGAACGCGCAATCAACGTCAAATCG
GGCGGAGCAGCACCAAACCATTTTGCCGCATCTTTCGGGTTCATCGCCGAATGCATGACA
TCGCCGACATTATCGGTGGTAAACATCAGGTTTTTCTTGATTTCTTCGTCAGTCAAACCG
ATGTCTTTCAGACGGTTGAAGCGCATACCGCTTGCAGAGTGGCAAGACAAACAGTAGTTT
GTAAAGATTTGCGCACCGTGCTGCAGGCTGACTTGGTCACGCAGGTCGATATCGACTTTT
TCGTAGTGTCCGCCGCCGCTGGCGACGGCTGCACTCATAGGCACTGCCAGCAATAAGGCA
GCAAACCAGTTTTTCAGAGTTTGTTTCATTTTCGCTGCCCTCATCAGATATTGGTTGCAA
ACAAGTAAGCACCAACAACGGTAATACCGACGTAAACAAAGAACATAATTTTTTGTTTAG
TAGTGCTCATGGTTACGCGTTCAGGAACTGGTTTGTTGGTATCCAGTTTGGTATAGAACG
GCATACCCAGGAAGAATGCAAAGTAGACGAAAGACAGGATACGTGCAACCAAAGTACGCG
TATCAGTTGCTACCATTGCACCCAAAATACCCAAACCGATGAAGGCAATGATGAACAGAA
CCAATGCGGTTTTGAAGATTGGGCCGCGATAGCGGACAGATTTAACCTCGCCTTTATCCA
ACCAAGGCAGCAAGGCGATCAGTACAACTGCTGCACCCATACCGATTACACCCCATACCT
GAGTACCGGCAAAGGAAGGAATCGCACGCAGAATTGCGTAGAACGGAGTGAAGTACCATA
CCGGCGCAATGTGCGGAGGTGTTTTCAGCGCATTCGCTGCATCGAAGTTTGGCGCTTCCA
AGAAGTAGCCGCCGCCTTCAGGTGCAAAGAACATCACGGCACAGAAGACAATCAAGAATA
TCGTTACTGCCAATATATCATGCACAACATAATACGGAAAAAAAGGTATGCCATCTAGAG
GGACACCGTTTTCATCTTTCAGCTTTTTGATTTCTACACCGTCAGGGTTGTTGGAACCCA
CTTCATGCAAGGCAATGATATGAGCCACAACCAAGCCGAGCAATACCAAAGGTACAGCGA
TAACGTGCAGGGCGAAGAATCGGTTCAAAGTAACATCGGAAACGTTGAAGTCACCGCGGA
TCCAAGTGGACAAATCAGGACCGATAACAGGGATGGCGGAGAACAGGTTAATAATTACCT
GCGCACCCCAGAAGGACATTTGACCCCAAGGCAGCAGGTAGCCCATAAAGGCTTCTGCCA
TCAATGCCAAGAAAATCAGGGAACCGAAAATCCACACCAATTCGCGCGGTTTTTTGTACG
AACCGTAAATCAGACCACGGAACATGTGCAGATAAACGACGATGAAGAAGAAAGATGCGC
CGGTAGAGTGCATATAGCGGATAATCCAGCCGCCGGACACGTCGCGCATGATGTACTCTA
CTGCGGTAAAGGCAGCAGGCAGATGGTAGGCGTTAAGGTTGCCGTCCGGTTTGTAGTTCA
TGGTCAGGAAAATACCGCTGACGATTTGAATCACCAGCACCAGCATAGACAATGAGCCGA
AGAAATACCAGAAGTTGAAGTTTTTAGGCGCATAGTATTCAGACAGATGCTCTTTCCACA
TTTTACTTAATGGAAAACGGGCATCTACCCAGCCTAACAATGCTTTTGCTTTGCTATTGG
TTTGGTTTGCCATAATTATCGTTCCTTATTCTTAGTCTTCGCCCACCAAGATAGTTGTGT
CGCTCAAGTATTTATATGGCGGGACAACCAGGTTGGTCGGGGCAGGAACACCTTTATATA
CGCGGCCGGCCAAGTCGAATTTCGAACCGTGGCAGGGGCAGAAGAAGCCGCCTTTCCAGT
CTGCACCCAAATCGGCGGGGGCAATGTCGGGACGGAAGGTGGGCGAGCAGCCCAAATGGG
TGCAGATACCGATGGCGACAAGGATGTTCGGCTTAATCGAACGGGTCTCGTTTTTAGCAT
ACTCCGGCTGCTGTTCCGCATCGGAATTGGGATCGGTAAGTTCGCCGTTCAGGCCTTTCA
GGTCTTTAAGCTGCTGATCTGTACGGTTGAGCACCCAAATCGGTTTGCCTTGCCACTCGG
CGGTCAGCAGCTGACCCGCTTCGATTTTACTGACATCCACCTCGACGGCAGCACCGGCGG
CCTTGGCTTTTTCCGAAGGGAAAAAACTGGCCACAAACGGCGTTGCCACACCCAATGCTG
CCACTCCGCCCGCGCCGCAGGTCGCGAGTGTCAGGAAACGGCGGCGGCCGTTGTTGATTT
CTTGATTATCCATTATTCAGTCGTCCTAATATTTTGGGAATACCGAGCCATTAAACGTTG
CAATTTTACCCAGTTTGCAGTGATACTCAAAGCATTATTTAAAATAAGGTAAAGTTTTAT
GATATTTCTCAAGACTCAAGCCGGATTGTTTTCGTCAAAATGGCACACTTCCAACCCGAA
AACCTCTGCCGCCGATTCTGCCAGCGCGCGTACGCCGTAACGTTCCGTCGCGTGATGCCC
TGCCGAAATGAAAGCCGTACCCGTTTCATTGGCAAGGTGGTATTGGGCTTCAGAGATTTC
CCCCGTCAAATACAGATCGACACCTTCGTCTATTGCCGTCTGAAAAAACCCCTGCGCCCC
GCCGCTGCACCATGCAACCCGTCGGATTTCGCGTTCGGGATTGCCGATAACGACAGGCTT
ACGTTGCAAAACTGTTTCAATATGCGCCGCCAATGCGCCGAGTGTCTTGGCTTGTTTCAG
GCTGCCCGAGTTGAGCAGGTTTTGTTCGCCGAACCGTTTTTCTGTCGCAAAACCCAATCT
GTCGGCGAGTTGGGCATTGTTGCCCAGTGTGGGATGTGCATCCAGGGGCAGATGGTAGCC
TGCCATATTGATGTCGTGCCGTAACAGTGCGGCAATCCGTTCTTTTTTCCAACCAGTAAC
GGTCGGCAACTCGTTTTTCCAGAACATACCGTGATGTACCAAAAGCAAATCTGCCTTCTG
CTCCACAGCAAAATCAATCGCTGCCCTGCTTGCCGTTACCGACGTAACGATTTTCCCGAT
ATATTCCCTCCCTTCAACCTGCAAACCGTTAGGGGCGTAATCTTTAAACAACGCTGTCTG
CAATGTTTCATTACACCAAGTCAGAAAATCCCTGCACAATACCATCTTTTTTCCTAATCG
CTTTAAACAAGCGGGCATTCTAATCGCAAAATGTCCGGAATTCACATTTTTCCGATTTGC
ACCCGCATATGAATTATTTTAATATGCGCCGGTTCAATATGCCGTCTGAAGCCCCATGGA

## Appendix A

TTCCATTATCGAATTGCGCCACCTCAAAACCCTGCTGGCACTTGAAGAAACCGGCAGCGT
CTCCCTTGCCGCCAAACGGGTTTTCCTTACCCAATCCGCCCTTTCCCACCAGATCCGTAT
GCTCGAAAACCACTACGGCACGCCGCTGTTCGAACGCAAATCCACGCCCTTGCGCTTTAC
CCCGGTGGGCGAAAGGCTGCTGCGCCTCGCCCACGAACTTATACCTCAAGTTGCTGTTGC
AGAATGGGATTTGGCGCGAATCACGGAAGGAGAGGCGGGAGAGCTGCGGATTGCCGTCGA
ATGCCATACCTGTTTCGACTGGCTGATGCCCGCCATGGGCGAATTCCGCCCGATGTGGCC
CCAAGTCGAATTGGATATCGTATCGGGATTCCAAGCGGATCCCGTCGGACTGCTGCTGCA
ACACCGTGCCGACCTTGCCATTGTTTCCGAAGCGGAAAAACAAAACGGTATTTCTTTCCA
ACCGCTGTTTGCCTACGAAATGGTCGGCATTTGCGCACCAGACCATCCGCTTGCCGCCAA
AAACGTTTGGACGGCGGAAGACTTTATCGGGGAAACCCTGATTACTTATCCCGTTCCCGA
CGAGATGCTGGATTTGCCCAAAAAAATCCTGATTCCGAAAAACATCAACCCGCCGCGCCG
ACACAGCGAGCTGACCATCGCCATTATCCAACTGGTTGCCAGCAGACGTGGCATTGCCGC
CCTTCCCTATTGGACAGTCATGCCCTACCTTGAAAAAGGCTATGTCGTCCACCGCCAAAT
TACTGCCGACGGACTGCAAAGCAAACTGTATGCCGCCATCCGTACCGAAGATACGGACAA
GAGCTATCTGAACAATTTTTGCCAAATCATACGCGAACGCGGTTTTGCAGATTTGCCCGG
ACTGAGCGAACTGGAACCGGTCTGACCCCTTATTCAACCATACCCGGCAGTTTTTCTATT
TTTTCATGTATAGTGGATTAACAAAAACCAGTACGGCGTTGCCTCGCCTTGCCATACTAT
TTGTACTGTCTGCGGCTTCGTCGCCTTGTCCTGATTTTTGTTAATCCACTATACTGTTTT
TGATTTTTGCCCAATCTGTAATCTTTAGATTGCCAATGGGAAACCGTCTACTACAAATAA
AAAACCCTGCGATAAGCAGGGTTTTTTGAATTTCCAACATTAACGTTTGGAGAATTGTTT
TGCACGGCGTGCTTTGCGCAGACCCGGTTTTTTACGTTCGACTTCGCGGGCATCGCGGGT
AACAAAACCAGCTTGAGACAAGGCGGGTTTCAACGCGGCATCGAAGTCGATCAGGGCACG
GGTAATGCCGTGGCGGATTGCGCCGGACTGGCCGGTTTCGCCGCCGCCAACAACATTGAC
TTTGATGTCGAAAGATTCGGCGTTTTCAGTCAGAACCAAGGGTTGGCGAACAACCATTCG
GCTGGTTTCCCGTGCGAAGAATTCGTCAACGGGACGACCGTTTACGATGATTTGACCTGT
ACCTTTAATCAGGAATACACGAGCCACTGAACTTTTGCGGCGGCCTGTGCCGTAGTAGTA
TTTACCGTTCATGTCGCGTCCTTATTTCAGTTCCAAAACTTTGGGTTGTTGCGCAGCATG
GGCGTGTTCCGCACCCGCATACACTTTCAGTTTTTTAATCATGGCGTAACCCAGAGGACC
TTTGGGCAGCATACCTTTTACAGCTTGTTCCAAAGCGCGGCCCGGGAATTGCTCTTGCAT
TTCGCGGAAGGTGCGTTCGTAGATACCGCCTGGGAAACCGGAATGGCGGAAGTATTTTTT
ATCTTCGAATTTGGCACCGGTTACACGCAGTTTGTCCGCATTGATAACAATGATGTAATC
GCCGGTATCGACGTGGGGGGTGTATTCAGGTTTGTGTTTGCCACGCAGACGGCTGGCGAC
TTCGGCCGCAACGCGACCCAAGACTTTGTCTTGGGCATCGATGACGAACCATTCGCGCTT
CACCTCGTGGGGTTTCGCTGAAAAGGTTTTCATAGTGGAAATCCAGATAGATATAGAAAG
TTGTAAATTTTAAAGACAGGATTCGATTTTGTCAATCGCATTACCGCGTTACGGAAGGAT
TTTCCGGATTTCGGCAGACTGCATACTGCTTTTTCGGGCGGGGCGGCGGCCAATGTGAAA
AACCGCATCGTTGCGATGCGGTTTTGAATGGGAATCCCCGCGAGAGCCGTTTCGGCCGAA
TCCGCTTGAACCTTGCTGACAAGGCGGCTGCCTCGGGTAGTTTCGGGTGCGTCCGCAAAA
GGACGCTCGCGCCCACTACTGCTCCCGGCAACCTTAAGCGAACTTATTGGTTCAAAGGAA
TATATGCCTTCGCGGACACCGCAGGGAAAAAGGGGTTATTCCTGCGCCAAGCGGGATAGT
GCTTTTTGGCAGGCGTTGTCCATATCGGCTATTTTACGCGCAAAATCGCCGATTGCCAAA
TCGCCGCCGTTCAGGGAGGTTTTCAACAGGTCGTGGACGACGTTGAGCGCGGCCATAATG
ACGATTTTTTCGCTGTCCGCGACGCGTCCGCCTTCGCGGATGGCTTCGGCTTTGCCGTTG
AGCATTCCGACTGCCTGCAACAGTGTGTCTTTTTCTTCTGCCGGCGTGTTGACGGTCAGC
CGGGCGTGCATGACTTCGATGTGGACTTGTTCGATGTTCATCCTTTAATCCTTATTGCTG
CGTTTCCTGCCATTGGGGGAGGCGCGCTGCCAGTGCGCTGATTTTTTCCCTGCTCTGTTC
GAGCAGGCTGCGGTATCGTGTATTTTCTTCTGTCAGGCTGTCAATTTTGTTTTGCAGGTC
TTCTTTGAGTTTGCCGACTTGGACGAGCAGGGCTTCGCTGAGTTCGTCGACGGCGGTTTC
GTGTTCGAGTTTTTGCCGCTCGTGCGCCCGTTTGAGTTCGGCGACGGTTTCTTTGAGGCG
~~GCGGTTTTTCGCTGACGAGGGTTTCGAATTTTTGTACCAACGTATAAACGCTGCTTTCGAG~~
TTTTTCGATATTTTGTTTCATAACCTTACCTGTCCGTATGCCGTCTGAAGGCTTCAGACG
GCATCTGTCTGTTGTTTATTCAAAACGCGCGCTGCGTTCCATCAGTCTTTCGACAACCTG
TTGCGGGGTCATTTCTTTGCGGATGAGTTGCAGCAGAGTTTGGGTAATCGGCATGTCGAT
TTGGTACTTACAGGCAGTATTGAAGACTTCTTCTATCGTGCTGACCCCTTCGGAAACGTG
TCCGATTTCGACCAGCACCTGATGCAGTTCCTTGCCTTCTGCCAAACCCAAGCCGACGCG
GCGGTTGCGCGAAAGTGCGCCGGTGCAGGTGAGGATGAGGTCGCCGATGCCTGCCAGCCC
CATCATGGTTTTGGGCTGTGCGCCCATTGCGGAGGCAAGGCGGGTGATTTCAGCTAATCC
GCGCGTAACCAGTGCGGCACGGGCGTTAAGCCCGTACTCTAGGCCGTCGGACAATCCGGT
GGCAATCGCCATAACATTTTTTACCGCGCCGCCAACCGCCACGCCGATAACATCGGTACT
GCCGTAAAGCCTCATGACGGTCGTGTTGAGCTGCGGTACGAGTTCTTCAATCCACTCTTG
GTTTTCGGAGGCAAGGACGACGGCGCAGGGCAGTTGTTTGGCGAGTTCCTGTGCAAAACT
CGGGCCGGAAAGTACGCCGATTTTCTTATTGTCGGGCAATACTTCTTTCAAGACTTGAAA
GGTCAGCAGCCCGGTATCCTGCTCGAATCCTTTGCAGGCGGCGAGGACGGGGAGGTGTCC
CGCGCCGTACTGTTTGAGCAGCTCTGCGCTGCTTCTCAATCCGGCAACGGAGGTTACGAT
AAGGACAAGTCCGCTGTCTTTGAGCGCGTCTGCCAAATCCGCACACACTTCCAAGGTTTC
GGGAAAGGAAAAGCCGGGCAGTCCGCGTTTGTTTTCACGCGCTTCCTGCATTTGACGGAC
TTGGTCTGCGTTGCGCGTCCACAGGGATACGCGGTTGCCGTGTTGGGAAAAATGCAGGGC
GAGCGCCGTACCCCACGAACCTGCGCCGATAACGGTAATTTTCATTGGTCGTCTTTCAAC
ATATCACTGCCGTTCACTTTAAAACAATCGGTGTTTCTCTGCAAGTGCGGTCAGGGAAAT
GCCGTCTGAAAGGCGTTCAGACGGCATTTTGCCCCGATGCGGCACTATCAGCCTGTATTG
CGCAAACCTTGCGCCACGCCGTTGATGGTCAGGTGCACCATCAGAAGGGCGTGCGGATTG
TCGGGTTCTTTACGCAGGCGTTTGAGCATGGCGACTTGCAAACCGTTGAGCGCGTTCAGG
TAGGGAATCCTCAAAGCGAGCGAACGGGCGAGGCTGCGGTTGTCGCGCAAAAGCTCTTCG
GTTTGCAGTAGGTCGAGCAGTGCTTTGCGGCTGCGGCGGTATTCTTCCTTAATCATCCCG
AAGATGATTTTTGCCTTATCGGGCGATTCGCTCAAGCCGGCATAGTTTTCCGCGAGGGTG

# Appendix A

ATGTCGGTTTTCGCCATCACTTGTTCCATATTGGAGAGCATGGCTTGGAAGAACGGGTTG
CTTTGGGCGTGTTCGCGCAGGGCGGCGAGCGTTTCGGGTTTGTCTTCGCACAAGGTTTCC
ACCGCGCTGCCGAAACCGTACCAAGCCGGCAGCATGAGGCGGTTCTGCATCCAGGAAAAT
ACCCACGGAATCGCGCGCAAGTCCTGAATCCGCGCCAAGGTTTTGCGGCTGGCGGGACGG
CTGCCTAGGTTGAGGGTGGCGATTTCCTGAATCGGGCTGGTTTGCAGAAAGTAGTCGATG
AAGTCGGGATGGGTAATCAGTTCGCGGTAGTATTTGAACGATACGTCCGACAATGCCTGC
ATCAGTTTGGCATCAGGGTCTTTTTTATCCGGCAGGATGCTGGCTTCCAAAGTCGCGGCA
ACCAAGGTTTCCAAGTTGCGTTGGGCATTGCCGGGGTCGGCGTATTTGGCGGTAATGACT
TCGCCTTGTTCGGTGATGCGGATTTGTCCCGCCACGCTGCCCGCCGGTTGGGCGAGAATG
GCTTGGTAAGAAGGGCCGCCGCCGCGACCTACGCTGCCGCCGCGTCCGTGGAACAGGCGC
ATACGGACATCGTATTTTTTGAAGAGTTCGACCAAGCCCAATTCCGCCTGATAGAGGCAC
CATGAGCTGGTAACGTAGCCGCCGTCCTTGTTGGAGTCGGAATAGCCGAGCATGATTTCT
TGGATGTTTCCACGGCTTTCGAGCAGTGCATCGTACCAGTCGAGGCGGAACATGGTTTCC
ATGACCGGACAGGCGTTTTCCAACGCTTCAATGGTTTCAAACAGCGGCACGATATTGATG
CGGCTGTGCGGTTTGCCGTTTTCCACCGCCAACAGGCCGGTTTCTTTCAGCAGCAATGCC
AAGGCGAGCAGGTCGCTGGGTTGTTCGCAGTTGGAAATAATGCTTTGTGTTACGGCATCT
TCGCCAAATTCGTCTTTGATTTTGCGCGCTTCGTTGAAAATTGCCAGTTCGTGGCGGGTA
TGGTCGCTGTATGTGATAAACGGGCTGTACAGAGGACGTTGATGGCTCAATTCGCGCAAC
AGGGCGGTTTGTTTTTGCTCTTCGTTCAGGCGGTTGTAGTCTTCCAAGCCTGCGTGTTGG
AAAAGCTCGGCAACCACATCGGCGTGTTTGCCTGCGTGTTGGCGCAAGTCGAGCGGCATC
ATGTGAAAGCCGAACACGGATACGGAACGGATGAGGTCTGCCAAACGGCCTTCGGCAAGC
AGACGGCTGCCGTTGTCGATAAGGGAACGTTGCAATTTTTTCAAATCATCCAGAAACTCT
TGTGCCGAAGCATAAGGCTCGAGAAAGCCGAATTTGCAGCCCATACCCAAACCGAGCGCG
CGCGCTTTGCCCATAGCGCGCGCCATAATGTAGGCGATGGCGCGGCGGTAGGGTTCTTCG
GCGCGGGCGATTTCTTCGTCGGGCGATTTGTCGGACAACGCCGTTACATCGCCGTTGACT
TTGACGCGGCGGATGGAGAGCGGCAGTTCGCGGTAGAGTTTGTCGAGTTCGCCGCGATAG
AAGCGGAACACGGCATCGGCGTGGCGGCGGAAGGCAAAGCGCAGGGTTTCGGCAGAAACA
AACGGATTGCCGTCGCGGTCGCCGCCGATCCAGCCGCCGATTTTGAGGATGTCCGGAACG
CGGACGCCGGGATAGGCCGTCTGAAAGTCGTGTTCCATCTTGCGGTAGAGCTTGGGCAGG
GCTTCGAAAAAGCTCATCGGGAAGATGGACACGCCGTTGTTGATTTCGTCGTTGACGCTG
AGTTTGTGGCGGCGCGTTTCGCTGGTCTGCCACAAGCCCAGCAGGATAGTGTCGATTTCG
CGGCGCAGCCGTGCCAGCGCGTCGGCATTGGTGCAGCGTTCGCGTTGCGGCAACAGTGCG
CGGATGCGGCGGTTGAAGCTTAAGACGGTTTGGCGTTGCACTTCGGTCGGGTGCGCGGTC
AAAACGGCGGTAACGGACGTATTGTCCAACTGCCGCTGCACCGATTTGCCGTCGGCTTTC
CCCGCTTTGAGCCTGCGGACGGTTTCCGTCAGGCTGCCTTCCGCGCCGCCGCGTCCGGCT
TCTTCGTGGATTTGGCGGCGGCGTTCGTGGTGCACGTCTTCGGCGATGTTCAAAATCTGG
GCGAACAGGCCGCAGGCCAAGGTTAAATCGTGGGTTTGTTGTTCGTCCAATTGCGGCAAT
ACTTTTTCAATCAATGCCGCGCTGTCGTCGGAAGTGGACAAGAGTTTGACTGTTTCGACA
ACCAACGGCGAGGCTTCTTCGTGCAGGAGGTTGAACAGGGATTGTTTCAGAAATTCCGCG
TCCGCCGCCAAAGCCGCGTCCTTTGGATTGTTCAGAATATGCAGTTGCATGATTTTTCTC
TCTCGTCTGCCGTAAATATTGTAAATGTACCCCAAATGCCGCATCCGTGCCAAACCGTTC
ACACTTTAACCGCCCGTGTCCCGAAATGCCGTCTGAAGTTGAACGCCGCCCGACGGCAGC
GTTACAATCGCCCGCAACTGTTTTTTCCGAACATCATCATGACCACGACCGAACACGAC
AACGACGATGCATTCCTGCTGCGGTACAGCCGCCACATCCTCTTGGACGAAATCGGCATC
GAAGGGCAGCAGAAACTTTCCGCCGCGCATATTTTGGTCGTCGGCTGCGGCGGTTTGGGT
GCCGCCGCACTGCCCTACCTTGCCGCTTCGGGTGTCGGCACGCTGACCATAGCCGATTCC
GACACGGTCGAACTGCACAACCTGCAACGCCAAGTCGCATTTGACGAGGGCGATGTCGGC
AAACTCAAAACCGAAGCCTTGGCAGGCCGCCTGAAACGCATCAACCATACCGTCAACGTC
CGCGCCGTCAACGAAAAACTCGACGGCTGCCGCCTGACCGGTTTGGTTCAAGCCGCCGAC
~~ATCGTTTTAGACTGTTGCGACAACTACGCCACGCGGCAAGCCGTCAACCGTGCCTGCGTG~~
CAAACGAAAACACCGCTGGTTTCAGGGGCGGCGGTACGCTTTGAAGGGCAACTTGCCGTG
TACCGTCCCGACTTGCCCGACTCGCCGTGTTACGCCTGCCTGTTTGACGGCGGATCGGCT
TCAGACGGCATCTGTTCTCTCTTCGGCGTGTTCTCGCCGCTGGTCGGCATCATCGGCAGT
ACCCAAGCGGCGGAGGCTCTGAAAATCCTGCTGGATGCGGGCGAACCGTCGCACGGCAGG
CTGGCGGTTTACCGTGCCTTGGAAGGGGGCTGGCAATATTTCGACCTGCCGCGCAACCCT
GAATGCCCGGTTTGCGGCACAGCGCGATAAACCCTGCCGCCGTTTCAGACGGCATCCAAA
CGGATGCGGCGGAACGGTTTTAAAAATTTAAAAATTTACATTTCTTTGCAAAAAAAAAAA
AAAAAAAATAAACTTACCTTATAATTGCAATTGTTTTAGCAATGTCTGTTTCGCAGACTC
ATTGAGTAAAACGTTTTCCCCGTAATGTGTTTGGCCGTCTGTCCCCTTTGGGTTCGGACG
GCTTTTTTTTGGCTGTGTTTGAATACCCGGTTGGTTTTATCTGTTTGCAGCGGGGGAAGC
CGCTTATTTCCGTTCGGGCGGAAAACGGTTCCATCGGATAAAAGGCATTTTGTCCGACTG
ATTAAAGTTATAGTGGATTAACAAAAACCAGTACAGCGTTGGCTCGCCTTAGCTCAAAGA
GAACGATTCTCTAAGGTGCTGAAGCACCAAGTGAATCGGTTCCGTACTATTTGTACTGTC
TGCGGCTTCGTCGCCTTGTCCTGATTTTTGTTAATCCACTATATATCTTAGGTTTGCATC
GGCGGAATATTCAAACACAGCCTTTTTTAAGGAAATCCGGATACGGCGGCGCATCAATAA
TGCGGCGGAATCTCGTCGCGCAGGGAATACGGCTCTTGCGCGTCGGGATTCCTGTCCTGC
ATTTTTTGATACAGCAGCCTCAACTGAGCCTGCTGCAAATCCAGCGTCTGCCGCAATTCC
GCCACCATCGCGTTCAGGCCGGCGATTACGTCCTCCTGAAGCGCGGATTGGATTTCCAGT
TCGACAATACGGCGTTCCAACTCTTGAACCGCGTCCATTTACAGCACCATCGCGGCAATC
CAGCCGGCAATCAGCAGCGGGATGTTGTAGTGGATGAAGGTCGGGATAACGGAATCGCGG
ATGTGGTCGTGCTGCCCGTCGGCGTTCAGCCCCATCGTCGGGCCCAGCGTGGAATCGGAC
GCAGGCGAACCGGCATCGCCCAACGCCCCCGCCGTGCCGACAATGGCGACGGTGGCAAGC
GGCGAAAAACCCAAACCGACACACAAAGGCACATAAATCGCGGCAATAATCGGCAAAGTG
GAAAAGGACGAACCGATGCCCATCGTTACCAAAAGCCCCACCACCAGCATCGCCAATGCC
GCCATACCTTTGCTGTTGCCGAATATCGCCATACTGCTTTCCACCAGCGGCTGAATATGC

## Appendix A

CCGGTCGCATTCATCACGGCGGCAAAACCCTGCGCGGCAATCATAATGAAGCCGACCATC
GCCATCATCTTGATACCTTCGCCGAATACGTCGTTTGCCTTGTCGCGGTTAATGACCCCC
AACATCATAAATACGGCGAAACCGAGCATCGCGCCCAACACCAGCGAGTCTTCATACATC
AACTGGATGGCAAAGCATACGGCAATGGCGACGGCGGCGGCCAGGCTGCGGTAGGCGGAC
GGCTGCGGACGGTTTGCCGCATCGGCGTTGCCCGCCGTATCGGCATTGTTGCTTTGGTAC
AGGCGCGGTTTGCGGTAATGGACAAACGCCAGCAGGAGTCCGGCCAGCATTCCCAACGCG
GGAATCGCCATTGCCGCCATCACGTTAATGTTTTTCACATCAAGCTGCGGCGCGGCGGAA
TGGATGTTGCCCAACAGGATTTCGTTCAAAAAAATCGCGCCGAAGCCGTAAGGCAGGAAC
ATATAAGTCGTAACCAGCCCGAAAGTGATGACGCACGCAATCAGGCGGCGGTCGATTTTC
AGGCGGTTGAACACCAAAAGCAGCGGCGGAACAATCATCGGGATAAAGGCAATGTGGATG
GGGATGATGTTCTGACTCATCATGCCCATCACAAGGATGATGGAAAGCAGCAGCCATTTG
ACCGCGCCCTCGCCCGAACGCACGCTGTCGGGCATACCGCCCCGGTTCAGCTTGCGGACG
ACCGCGCCGGCAAGCTGCTGCGGCAGGCCGGAATGGGTAATCGCCATTGCAAACGCGCCG
AGCATCGCATAAGAAAGCGCAATCTTCGCACCGCCTTCCAAACCTTTGTTGAACACGGGG
ATAATCCCCGCCTGACTGACCTGTCCCGCCGCATCGGCAATGTTTTGCAGCGGCATACCC
GCCACCGCGCCGCCGACAAACGCGCCGACCGTCAGGCTCAATACCACGTGCACGCGCGAC
AGCGACAGCACCAGCATAACGATTACGGCAACTACGACTGCATTCATTGTTTATCGCTCC
AAACCTATAAATGTTTACATATCGAAACACATCATAACCCAATAACGGGAAACCCGCCAA
TTTTGCAAACAATTATTTCAAATGCTTCATATACTTCCCCAGCGTAACCCTGTCCAAACC
CGCCAAATCCGGCAGGGTTTCCACTCCTGAAAAACCATTCTCCGCCAACACGCCGCGCAC
CGCCGCGCCCTGATCGAAACCGTGTTCCAGCAATAAAAAACCGCCTTCCGCCAAACGGTC
GGGCGCGCCTTGCGCCAAGGTGCGGATGCAGCTTAGGCCGTCTGAAAAGTCGGTCAGCGC
GATTTGCGGCTCAAACCGCAAATCGCCTTGCAACAAATGTTTATCGCCGTTTTCGATATA
GGGCGGGTTGGACACGATGATGTCCCATTTCCCTTCAGACGGCATATCGGTGTCGAACCA
CGAACCGTGTGCAAATTCGACCCGCGCGCCCAAATCCGCCGCATTTTTCCGCGCCGTTTC
AAGGGCGGGCGGGCTGATGTCGGATGCGCGCACAAACGCATCGGGGCGTTCGAGCGCGAC
GGTTACGGCAACCGCGCCGCTGCCCGTCCCCAAATCCCACACGCGCCCGTTTTCGGGCAG
GCGCGCCAATACGGCTTCGACCAAATGTTCGGTTTCGGGGCGCGGAATCAGCACGCTCGG
ATTGACTGTAAAGCGTCTGCCATAAAATTCGCGCACACCTAAAATATAGGCAACCGGCTC
GCCGTTCAGACGGCGTTGCGCCAGCCTGTCCGCCCGCTGTCGGACTTCGTCCGGCATTTC
TTCCCCGCCCCGCGTCAACAACTGCACGCGCGTATATTCCGAAACATATTGTAGCAGCAT
TCTTGCTTCATTTTTAGGCAGTTTTGACAAGCCCAACCATTTATCAAACGTCATTTTTAT
CCCGTCTGCCGCTGATGCGGCTTTTCTTTCCTTATTCTTTCCGGCAAACGTACCGATGGT
GGCAACCGCAAATGCGGCATACCACAAATAAAATCCTGCACCGTAGCGCACAATATCCGA
TGTATTCCCTGCTTCATCGACGTATACGGCTTTCACACTGAAAGCCACCAACGCCAAGCC
CCAAAGTGCCGCATGGACAGGCACGACCTTCTTCCGCAACGCCAGCAAAACAATGGCCGC
CAACCAAACATAATTCGCATAGACCGCACAATACCTGATATCCAAAGAAGCAAATATCGA
CCCCAAAATCAAAACGGTCAAACCCTCCATGCTTCCATGATTGCCCAAATAAAATGCAAC
ATTGGATAAAGACGCTATCCACAGGGCAACCGACACCAGCAACATCACTATGGGAAAACT
TGGTTTCCGATTCTGTTCCTGCATGGTTTTATCCTAATGTAAAAGGCCGCCTGAAAACCT
TTCAGACGGCATCGTGCCGGATTCCGCGTCAGATTGCGCTGCCGCCGACGGTCAGTCCGG
CATCAATCCGCAAAGTCGGTTGCCCCACGCCGACGGGGACGCTCTGCCCTTCTTTGCCGC
ACACGCCGACACCGCTGTCGAGCGCAGTATCGTTGCCTATCATGGAAACGTGTTTCAGCA
CTTCGGGGCCGTTGCCGATGATGGTCGCGCCTTTGACGGGATATTGCAGCCTGCCGCCTT
CCACCCACCACGCTTCGGACGCACTGAACACGAACTTGCCGCTGGTAATGTCCACTTGTC
CGCCGCCAAAGTTGACGGCGTAAATGCCCTTGTCGATGGACGCGATGATTTCTTCCGGCT
CATAGCTGCCGTTTTCCATAAAGGTATTGGTCATGCGCGGCATAGGGGCGGAAGCGTAAC
TTTCGCGGCGGCCGTTGCCGGTGGACTGCGTACCCGTCAGGCGGGCATTGGTTTCGTCCT
GCATATAGCCGACTAAAATGCCGTCTTCAATCAATACGGTGCGGCGGGTTTCGTTGCCTT
CGTCGTCGATGTTGAGCGAACCGCGCCGGCCGGCAATATCACCCTGATCGACGACGGTAA
CGCCTTTGGCGGCGACGCGCTCGCCTATTCTGCCGGAAAAGACGCTGGTTCCCTTGCGGT
TGAAATCGCCTTCCAAACCGTGTCCGACCGCTTCGTGCAGCAACACGCCCGGCCAGCCGT
TGCCCAAAACGACGGTCATTTCGCCGGCGGGCGCGGGGCGGGATTCGAGGTTGGTGAGTG
CCTGTTTGACGGCGGCATCGACAAACCGATGAACCAAGTTTTCATCGAAATAAGCCAAGT
CGTAGCGTCCGCCGCCGCCCGCGCTGCCCTGTTCGCGGCGTTCGCCCTGTTTGGCGATAA
CGGTAACGTTCAGGCGCACCATCGGGCGGATGTCGGCGGCGTGTTTGCCGTCCAGACGGG
CGAGGTAAACCATATCGTATTCGCAGGTCAAACCGGCCATCACTTGCACGATGCGCGGAT
CGGCGGCTTTGGCGATTGCTTCCACTTTGTTCAACAGCGCGACTTTGGCGGCGGAATCGA
GGCCGGCAATGGGGTCGGACGCGGAACAAACCGGCTTGCCGCGCGTTTCAGACGGCATTT
TGGCGGACACCTTGCCGCCTGCCGCCCCAATCGCGCGGACGGCGCGGGCGGAACGGTTTA
TCGAATCGATGCACAGGCTGTCGGCGTAGGCAAAGGCGGTTTTGTCGCCCGAAACGGCGC
GCACGCCCACGCCCTGATTGATTTGGAAGCTGCCCGATTTGACGATGCCCTCTTCCAAAT
GCCAGCTTTCATAAGCGGTGCGCTGGCAGTAGATGTCGGCGTAATCGACGTGGTGCGCGC
CGATGATGCACAGGCTTTTGGCGAGCAGTTCGGGGGAAAGGCGGTTGGCTTCGAGCAGCC
GCGCCTGTACGGCGGAATAGGTCGGATGCATAGTGTCGGCGCATAAAAAATCAGGGGCTT
GATTATACGGCATTTGTTATATAGTGGATTAACAAAAAACAGTACGGTGTTGCCTCGCCT
TGCCGTACTATTTGTACTGTCTGCGGCTTCGTCGCCTTGTCCTGATTTTTGTTAATCCAC
TATAGAAATGCGCCGTGCCGCCTGAAATGTAAGATTTTTGCCAACGCCCCCTGCTTTTGT
GTACACTTAAAGCTCCTTGTCGGAGTGCCGCCGCCGGGCGGCTGAGATTGCGAAAGCAGA
ATCCGTAGAACCTGTCGGGGTAATGCCTGCGTAGGAAACAAACCGTCAAATGCCTTATCA
GGCTTCCGTTCCCTTTTCCGCACTTCCCCGCCCCATTTTCATGTTTTTTAAGGACTTGAT
ATGTCGGGCAATGCCTCCTCTCCTTCATCTTCCTCCGCCATCGGGCTGATTTGGTTCGGC
GCGGCGGTATCGATTGCCGAAATCAGCACGGGTACGCTGCTTGCGCCTTTGGGCTGGCAG
CGCGGTCTGGCGGCTCTACTTTTGGGTCATGCCGTCGGCGGCGCGCTGTTTTTTGCGGCG
GCGTATATCGGCGCACTGACCGGACGCAGCTCGATGGAAAGCGTGCGCCTGTCGTTCGGC

## Appendix A

AAACGCGGTTCAGTGCTGTTTTCCGTGGCGAATATGCTGCAACTGGCCGGCTGGACGGCG
GTGATGATTTACGCCGGCGCAACGGTCAGCTCCGCTTTGGGCAAAGTGTTGTGGGACGGC
GAATCTTTTGTCTGGTGGGCATTGGCAAACGGCGCGCTGATTGTGCTGTGGCTGGTTTTC
GGCGCACGCAAAACAGGCGGGCTGAAAACCGTTTCGATGCTGCTGATGCTGTTGGCGGTT
CTGTGGCTGAGTGCCGAAGTCTTTTCCACGGCAGGCAGCACCGCCGCACAGGTTTCAGAC
GGCATGAGTTTCGGAACGGCAGTCGAGCTGTCCGCCGTGATGCCGCTTTCCTGGCTGCCG
CTTGCCGCCGACTACACGCGCCACGCGCGCCGCCCGTTTGCGGCAACCCTGACGGCAACG
CTCGCCTACACGCTGACCGGCTGCTGGATGTATGCCTTGGGTTTGGCAGCGGCGTTGTTC
ACCGGAGAAACCGACGTGGCAAAAATCCTGCTGGGCGCAGGTTTGGGTGCGGCAGGCATT
TTGGCGGTCGTCCTCTCCACCGTTACCACAACGTTTCTCGATGCCTATTCCGCCGGCGCG
AGTGCGAACAACATTTCCGCGCGTTTTGCGGAAACACCCGTCGCTGTCGGCGTTACCCTG
ATCGGCACGGTACTTGCCGTCATGCTGCCCGTTACCGAATATGAAAACTTCCTGCTGCTT
ATCGGCTCGGTATTTGCGCCGATGGCGGCGGTTTTGATTGCCGACTTTTTCGTCTTGAAA
CGGCGTGAGGAGATTGAAGGCTTTGACTTTGCCGGACTGGTTCTGTGGCTTGCGGGCTTC
ATCCTCTACCGCTTCCTGCTCTCGTCCGGCTGGGAAAGCAGCATCGGTCTGACCGCCCCC
GTAATGTCTGCCGTTGCCATTGCCACCGTATCGGTACGCCTTTTCTTTAAAAAAACCCAA
TCTTTACAAAGGAACCCGTCATGACCCGTATCGCCATCCTCGGCGGCGGCCTCTCGGGAA
GGCTGACCGCGTTGCAGCTTGCAGAACAAGGTTATCAGATTGCACTTTTCGATAAAGGCT
GCCGCCGGGGCGAACACGCCGCCGCCTATGTTGCCGCCGCCATGCTCGCGCCTGCGGCGG
AAGCGGTCGAAGCCACGCCCGAAGTGGTCAGGCTGGGCAGGCAGAGCATCCCGCTTTGGC
GCGGCATCCGATGCCGTCTGAACACGCACACGATGATGCAGGAAAACGGCAGCCTGATTG
TGTGGCACGGGCAGGACAAGCCATTATCCAGCGAGTTCGTCCGCCATCTCAAACGCGGCG
GCGTAGCGGATGACGAAATCGTCCGTTGGCGCGCCGACGACATCGCCGAACGCGAACCGC
AACTCGGCGGACGTTTTTCAGACGGCATCTACCTGCCGACCGAAGGCCAGCTCGACGGGC
GGCAAATATTGTCTGCACTTGCCGACGCTTTGGACGAACTGAACGTCCCCTGCCATTGGG
AACACGAATGCGTCCCCGAAGGCCTGCAAGCCCAATACGACTGGCTGATCGACTGCCGCG
GCTACGGCGCAAAAACCGCGTGGAACCAATCCCCCGAGCACACCAGCACCCTGCGCGGCA
TACGCGGCGAAGTGGCGCGGGTTTACACACCCGAAATCACGCTCAACCGCCCCGTGCGTC
TGCTCCATCCGCGTTATCCGCTCTACATCGCCCCGAAAGAAAACCACGTCTTCGTCATCG
GCGCGACCCAAATCGAAAGCGAAAGCCAAGCCCCCGCCAGCGTGCGTTCAGGGTTGGAAC
TCTTGTCCGCACTCTATGCCATCCACCCCGCCTTCGGCGAAGCCGACATCCTCGAAATCG
CCACCGGCCTGCGCCCCACGCTCAACCACCACAACCCCGAAATCCGTTACAACCGCGCCC
GACGCCTGATTGAAATCAACGGCCTTTTCCGCCACGGTTTCATGATCTCCCCCGCCGTAA
CCGCCGCCGCCGCCAGATTGGCAGTGGCACTGTTTGACGGAAAAGACGCGCCCGAACGCG
ATAAAGAAAGCGGTTTGGCGTATATCCGAAGACAAGATTAAAGCCGCCCGAAAGGACACC
TTATGACCTTCCCGCCCCTAAAATCCCCGCTCAAATTCTACGCCGTCGTCCCCACCGCCG
ATTGGGTGGGGCGCATGGTCAAAGCAGGTGCCGACACGGTGCAACTGCGCTGCAAGGCCC
TGCACGGCGATGAATTGAAACGCGAAATCGCCCGCTGCGCCGCAGCCTGTCAGGGCAGCC
GTACGCAGCTTTTCATCAACGACCACTGGCGCGAAGCAATCGAAGCGGGCGCGTACGGCG
TGCATCTCGGACAAGAAGACATGGACACCGCCGACCTTGCCGCCATCGCCGCCGCCGGTT
TGCGCTTGGGTTTGAGTACGCACTCCGTTGCCGAACTCGACCGCGCCCTGTCCGTACACC
CTAGCTACATCGCCAGCGGCGCGATTTTCCCGACCACGACCAAACAAATGCCCACCGCCC
CGCAAGGCTTGGACAAACTGCGCGAATACGTCAAACAAGCAGGCGGCACGCCCGTCGTCG
CCATCGGCGGTATCGACTTGAACAACGCCCGAGCCGTACTCGCCACCGGCGTTTCCTCAC
TCGCCGCCGTCCGCGCCGTAACCGAAGCGGCAAATCCCGAAGCGGTGGTTAAAGCGTTTC
AGGCTTTGTGGGATGGATAAAACCGAAAGAAGAAAATTCAATTGCCGTGTAGGCAAAACT
TAGCCCGTTATCGCAAACATACTTAACTTTAAATGTGGCATATCATCAAATTCCGTCATT
CCCGCGTAAGCGGGAATCCGCCTTAAAACTTGAGAAACCATCATTTGAAAAACAGTTTCC
GAATTTCAAAAATGGATTCCCGCCCGTGCGGGAATGACGGCAACCGGTCAGTTGCGTATC
AAAAAATAAAGTAATTCGGCTAGATATAGTGGATTAACAAAAATCAGGACAAGGCGACGA
AGCCGCAGACAGTACAAATAGTACGGAACCGATTCACTTGGTGCTTCAGCACCTTAGAGA
ATCGTTCTCTTTGAGCTAAGGCGAGGCAACACCGTACTGGTTTTTGTTAATCCACTATAA
ATACAGAAACATCGAGAAACCATGAACATCATCTTAAACGGCGGACCCGCCGAACTTCAC
GGCACGACCGTTGCCGACCTCATCGCCCAAACCGCGCCGCAAAAGCCCTTTGCCGTGGCG
GTCAACACCGTTTTCGTCCCCAAAGGCGCGTATGCGGAAACGGTTTTAAACGAAAACGAC
AAAATCGATATCGTGCGGCCGGTGGTCGGCGGCTAGGCGGTTTTGCCTTTTCAGACGACC
CCTGTCCCCAAAACAACGTTATGGTGGATTAACTTTAAATCAGGACAAGGCGACGAAGCC
GCAGACAGTACGGATAGTACGGAACCGATTCACTTAGTGCTTCAGCACCTTAGAGAATCG
TTTTCTTTGAGCTAAGGCGAGGCAACGCCGTACTGGTTTTTGTTAATCCACTATACAAAG
GAACCCATTATGCTCACCCTATACGGCGAAACTTTCCCCTCGCGGCTGCTGCTCGGCACG
GCTGCCTACCCGACCCCCGAAATCCTCAAACAATCCATCCAAACCGCCCAGCCTGCGATG
ATTACCGTCTCGCTGCGCCGCGCGGGAAGCGGCGGCGAGGCGCACGGTCAGGGGTTTTGG
TCGCTGCTTCAAGAAACCGGCGTTCCCGTCCTGCCGAACACGGCAGGCTGCCAAAGCGTG
CAGGAAGCGGTAACGACGGCGCAAATGGCGCGCGAAGTGTTTGAAACCGATTGGATAAAA
TTGGAACTCATCGGAGATGACGACACCTTGCAGCCGGATGTGTTCCAGCTTGTCGAAGCG
GCGGAAATCCTGATTAAAGACGGCTTCAAAGTGCTGCCTTATTGCACCGAAGACCTGATT
GCCTGCCGCCGCCTGCTCGACGCGGGCTGTCAGGCGTTGATGCCGTGGGCGGCCCCGATC
GGCACGGGTTTGGGCGCGGTTCACGCCTACGCGTTGAACGTCCTGCGCGAACGCCTGCCC
GACACGCCGCTGATTATCGACGCGGGCTTGGGTTTGCCCTCACAGGCGGCACAAGTGATG
GAATGGGGCTTTGACGGCGTGCTTTTGAATACTGCCGTTTCCCGCAGCGGCGATCCGGTC
AATATGGCACGCGCCTTCGCACTCGCCGTCGAATCCGGACGGCTGGCATTTGAAGCCGGA
CCGGTCGAAGCACGCGACAAAGCGCAAGCCAGCACGCCGACAGTCGGACAACCGTTTTGG
CATTCGGCGGAATATTGAAAAAGGCAGCAAAAATGCCGTCTGAAGGCTTCAGACGGCATC
GCGGTCCAAAACGGCGGCGGCCTGAAACGGACAAACCGCCATTCCCCGGCATCACGGCTT
TGTCGGAAAAAATGGAAAAACCGGCCGGAAAACCTTGCCGCCCGTCCCGATGCCGCAACC

## Appendix A

AACGAAACACTCGGCCTCCACGGTGTGCAGGCTGCCGCGCAAGCCCTAAATACGGCAATA
GAAGAAGCGTTTTGTTGTTGTTTGAATACACTTAAAAATACCTAAAGCCGTCCGTAAGCC
TTCATCCGCAACGGTTTTACCGCTTTCGCATCCCCGAATCCACGCTCAAACACCCCCGAA
TGACAACCCTGTCCGCGCCAAATCGGACGGATGTTCAAACACGGGCAACCTTATTTCCGT
CAGGCACGAAGCCCTCAGCTATGCCTGCCGACCCCGATTTGTCCGACACAATGAAAGTTT
GCCGACCCGAATCACAAACATCGGCGGACAGGTTAATTTGTTTATTTTTCATCGTATTAC
AAAAAATCTGCATTTATTTTTAAATTTTTATTGATAATTATTATTATTAGCGTATAATCA
AAACCACTCGGAAGCCGTCCGTTCCGAACCATTAAACACCATATTTCCCCATCATCACTT
TCACACTTGGAGTCGGCATATACGAGACATACATTCCCTTTTTATATATCAGATACTCAA
AACCGAAACGCCAAACCCACCTTCGCGGTGGGTTTGGCGTTTATCGTCCGGCTTTCGCGC
CTATTTGCAAGACTTGAGGTTCAGTTTGCCGTATAGGGACGTGATTTTACGAATTTCGTC
CGCATCGGCGGCATTCACGCCGGTAAACAAAACCGTCATACGCGACACGCTCAAAGAATC
GTCCTGCCTGTCGGCTTCGGCAAAGTGTTCGACAATATGCGCCCCGCCGAATCCGGCGCC
GGCAAGCCGGTTTTGCAGGGCTTGGGCGTTTTCCCGGCTGTTGCCGACACCCAAACTCAA
TGCGCCGTCAAACGGTATGGGGTTGAAACCTTTGGCAGACAGCTCCGCCGCCTGATTTTC
GGCATCGGCGGAAACGGGCAGGACGACGCGGTAGGTTTTGTCGGCAGGTTTGGCTTGGGC
GGTGCGTTTTTCGACGCTCCTGCTGGCAACGTGCGACCATTTGCCCAAAAGTCCTTTGAT
GCGGTGGTAGTCATCTTCGTCCATCGTGAGGCTTGCCTGCGCGGCGCATAAGGCATCCGC
CGCGCCGTTTTCGCGTTCCGCCTGCGCCTTTTCGGCGGCGAGTTTTTCGCGGCGGGCTTT
TTCTTCACGCTGTTTTTTCTCTTTCAGTTTTTTCTGTTCCGCTTCTTTTTTCAAGCGCAA
CTGCTCCGCCTGTTCTTCGCTCAGAATGTCGCCCTGTTTGAGCAGTGCGCCTGTATCCGA
TTCAGATGCCGCCTGAACGACAGGACCGGATGCTGGAATATTCCGAACAACCGGCATAGT
TGGGGCAACTGGTTGAACCTGCAAATTGTTTGCGGCATTCTGTGCCTCCGGTATTCTGCC
GGCCTGTTTCAGTGTCAGTTTGTAACCTACCGTACCGCCGAATACGGCAATATTAATCGC
AACCAAAAGGATAAATAGCCATTTCATCTCTGTATTCCTTAAATATGTTCATATTCCCTG
CCTTCGGCGGCAATCATGTTCAACAACCCGTAAATGACGAGGTTGTCCGCCACGCGCACG
GTATTTTCCGCCAAAAATGCAGGCGGCAGGGCTTCGGCAACTTTTGCCGCGCCGCCGCCG
GTAATGATGACATCGACAGGCTTGCCCGCCCCGGTTTTTCTTTCAAACGCCCGTGCATC
ATCATAACCGAGCCGCAAACCGCATCCATCATGCCGCTGGCGACGGCATTGCCCGTTGTG
GTCGGGAAAGGATAACGCTTACCGGCGTGCCGGTTGAGGTTGGCGGTTCGGACGGCGAGC
GATTCTTTCATCAGGTGGAAACCGGGCATGATGGTTCCCCCGAGATAATGTCCGTCATCG
GTGAGCGCGTCAACCGTTACCGCCGTGCCGCAACTGACGACGACGCAGGCGTTGCGGCTG
AAGCGGCGGCTGCCCAAGGCGTTGAACCAGCGGTCGGAACCGTGTTCTTCGGGGTGGCGG
TAGTGGTTGCGTATGCCCAAAGCCTGTGCGGAAGACGGCAGCCACTCGATTTTTCGGGCG
AGCTGTTCCTGCACTTGTGCCTTTTTGAATTCTCCGCACACAGCGCAACCGACGATGCGG
ACATTTCCATCCGCCTTTTCCGCCCACTCCGCGCCCAAAGGCGACAAATCGCGGTACGGC
GCGCTACCGACGGTTGCGAACGTGCCGTTTTCCACCCACGCCCACTTGAGCCGGCTGTTG
CCGCCGTCCAACAGCAGAAAACGTTCCGAATCCCGCCGCTTCGGCACGGAAACCGGCCTG
TCGTCGGACCGCAGGCTGATTTCGCCGCTGACGACCGTCTGTTTGCCCTCTGCCGTTTCC
AAGTGCAAAACGCCTTGTCCGTCCACGCCTTTAACCGTGCCTTCGAACACGGTTTCGCCG
TCGCGCAACAGCAATACCGCCTTGCCGTGGTCGCGGTTGGCAGCCTGATATTCCGCCACA
AAAGGCGCAAATCCGTCCCGCGCATATTGCAACAACACCGCGTCCAGTTCCACCAACAGC
GTTTCCAGCAGCACGGCGGCATCGGCATTGCCCCGCCGCGATGCCGTCTGAAACAGCGAT
TGCACGGAAGCGGCATTTTCTACTTCCTTGGGCAGGACAAAATTGATGCCGATACCGACC
ACGGCAACCGTTTTGCCGCCCGTCCTGACCGTTTCAATCAGAATGCCGCCCAATTTGTCG
CGTCCGACAACCAAATCATTGGGCCACTTAATCTGCACATCCAAACCTAAACGCGACAAG
GCGCGCCGACACGCCACTGCCGCAACAGGCGACAGCGAACCCAACTCATACTGCGGCCGG
TCAAACACCCAGCCAAAACTGAACATCAGACACTCGCCCAAACGGTGCGACCACTTCCGC
CCCTGCCGCCCCCTGCCCTTACTTTGCAGGTGGGTCACGCATATGGTTTTGTGCGCCTTG
TCCGGCGCAATCCGCGCCAATTCCAGTATCTCGTCGTTGCTGGACGCGCACTCGTGCTTC
AATGCCGTCTGAAAACCCGACCTTTCCCCCAGCTCGCGCAAACCTTCGGCATCGAAAACC
GCCAATGGGCGCACCAGCCGCCAATAGCCGTCGTGTTGGCGCAACAGCCCGCGTATGTGC
GCCGGCATCTGCTGCCAAAAACCGTTGAGCTGCTGCGGCTTCATATCCGCCATACGCGCC
AGTTGCGAGACGTGTTGCGGCAAACCGTCGGCAAGCTCCGCCAACACCCGCCAGTGCGAA
AGCTTCAAAACCGTCATTTTCCGCCCTCTGCCGCACGGATTTTTGCCAAAGTCTTCGTTG
TCGAAGTCTGGTGCAGAAACGGAATTGAAAACACCTGACCGCCGCGCGCCAACGTTTCTG
CCGCACCGACAATCTTATCCGCAGCCCAATCGCCGCCCTTGACCAAAATCTCAGGTTTGA
CCGCCTCAATCAACGCCGCCGGCGTATCCCCGTCAAACCACGTTACCAAATCCACACTTT
CCAAAGCGGCGGCAACGGCGGCACGGTTCTCCAAAGGATTAACCGGGCGGTCACCGCCCT
TGCCCAGACGCCGCACCGAAGCATCGGTATTCAACGCCAGCACCAACGCGTCCCCCATCG
AACGCGCCTGCGCCAGATAAGTAACGTGCCCCCTGTGGAGGATGTCGAAACAGCCGTTGG
TAAACACCAGCGGGCGCGGCAACAACGCCAAACGCGCCGCCAACGCCTCGGGCGGACAGA
TTTTCGATTCAAAATCAGGGACAGACCAAGCGTCAACCATCAAAGCCTCCGACAAAAACC
ATAAAAGACAGAAAAACCCACATGATACAGAAGCATATGCGAAAGGCAAAGCCGGCGGCG
CGGACAGTACGCGCAAACGGGAAAAGACCCGTACCGAAAAGTACGGGCCTTTATCTGGGG
TGGCTGATGGGGCTCGAACCCACGACAACCGGAATCACAATCCGGGGCTCTACCAACTGA
GCTACAGCCACCATAAAAACGGTTTTCAATCAAATTCTTGGCACGCCCGACAGGAATCGA
ACCTGTAACCCCCGACTTAGAAGGTCGGTGCTCTATCCGGTTGAGCTACGGGCGCTCATG
CCGATTCGTGCTGATTGATTGGTCGGGGCGGTGGGATTCGAACTCACGACCCTCTGCTCC
CAAAGCAGATGCGCTAACCGGGCTGCGCTACGCCCCGACTTGAAGAAGCGAACTATACAA
CTCAGGGAAAGATGCGTCAACATTTATTTTCAAGACACCAAGATGAAAAATATAGTTTTT
TGATTTGAAAAAATATTTAATCCGTCCAAACAGCCGTATTTTATTTCAGGGCAAATTTAT
TTTCGGCATCCTGCTGTAAAAACAAACGGAAAATGCGATAATTTTCAGCATTTTCTACCT
GTTTAACAAAAGGACGGATATGTCGGCACAACTGATCAATGGTAAAGAAGTTTCGCAAAA
ACGCCTGCAGGCGGTTGCCGAAGCGGTGGCGCAACGCCAACAGAACAATCTGCACACCCT

## Appendix A

TGCCTGGCCGTGGTTTTGGTCGGAGGCGACCCTGCCAGCGCGGTTTATGTCCGCAACAAG
AAAACTGCCTGCCAAAAATGCGGCATCAAATCACTGTCTTACGAGCTGCCCGAATCAACA
TCGCAGGAAGAACTGCTGGCACTGGTCGACCGCCTGAATGCCGATTCCGAAGTGGACGGT
ATTCTGGTTCAGCTACCGCTGCCGAAGCACCTCGACAGCCAGGCGGTTTTGGAACGTATT
TCGCCGGATAAGGACGTGGACGGCTTCCATCCTTACAATGTCGGCAGGCTGGCGGTCAAA
ATGCCGCTGATGCGCCCGTGTACGCCCAAGGGCGTGATGACGCTTTTGGAAGCTTACGGC
ATTGATCCGAAGGGGAAAAAAGCGGTCGTGGTCGGCGCGTCGAATATCGTCGGCCGCCCG
CAGGCTTTGGAACTGCTGCTGGCGCGCGCAACGGTAACGGTCTGCCACAGCGCAACCGAA
AATCTGACAGACGAGGTTGCCGGAGCCGATATTTTGGTGGTCGGCGTAGGCATTCCGAAC
TTTGTCAAAGGCGAATGGATCAAACCTGGCGCGGTCGTTATTGATGTGGGCATCAACCGT
TTGGACGATGGCAGCCTGTGCGGCGACGTGGAATTTGAAACGGCAAAAGAACGGGCGGCG
ATGATTACGCCCGTTCCCGGCGGCGTGGGTCCGATGACGATTGCCACATTGATGGAAAAC
ACCCTGCACGCGGCTTCACTGCACGATGCTTGAGCGGTTCTGAAGATAAAAATGCCGTCT
GAAAGGCTTTCAGACGGCATTTTGCCGTGTCCGTTTATTTGGGCAGCTTGACGACAACCG
TATCCGCCAGTATGTCGTAAAGCGTGCGGCGGTCGCGTTTGACCATAAAGAGCAGGACAA
AGTTGGCAAGGAATGCCAGCAGGTTGATGGCGTTTTCTCCGTTGTCACCTACTGCAAGAC
CGATAACGGCGGCAATAATGGCAACCAAAACCGACCATGCGATTTCGCGTACCAAAACCG
TGCCGACAAAACCGGGATTGCGGCCGTCGGTTTTCAACACACGGATTCTCATGATTTTCT
TACCCAATGACTGCCCGTCCCGGCTCATATAGTAGATTTGGATGACGGTGTACGCCAAAA
TGCCTGCCAGTCCTACCCAAAAGGAAGTCATGCCCAAAAGCAGCCCGAATATTTCTTCGC
CGCTGCCAATCCTGCCTTCATTCTTGATGGCGAAAGCAATCAGTCCGGCAAACGGCACCA
ACAAAACCAAAAAGGTAAACAATTGGTTCAGCAGCGCGGCAAGTATCCGGTCGCCTGCAC
CGGCAATTCCGACTTCAATTTCCTGCCCGTTGCGGTTGTCGGATGCCGCGTCGGTGTAGT
CGTTTTTTTCTTCCATATCCGTTCCTGATAATTGTTCTTAACTGACCCCGATTCTACCGC
CACGACACCGAAAACGCCAATACTTAAAGAAATCCCGATAAAGAACTTTACATTTTCCCA
ATACGGCGTTAAAACGCTTCCTTTACGCCATACATAATTTTATTAACGATTTTTCCTCAA
GGAGCAACACAATGAAAGTAGGTTTCGTCGGCTGGCGCGGTATGGTCGGTTCGGTTTTGA
TGCAGCGTATGAAAGAAGAAAACGACTTCGCCCACATTCCTGAAGCGTTTTTCTTTACCA
CTTCCAACGTCGGCGGCGCAGCCCCTGATTTCGGTCAGGCGGCTAAAACATTATTAGATG
CCAACAATGTTGCCGAACTCGCCAAAATGGACATCATCGTTACCTGCCAAGGCGGCGATT
ACACCAAATCCGTCTTCCAAGCCCTGCGCGACAGCGGCTGGAACGGCTACTGGATTGACG
CGGCGTCCTCACTGCGCATGAAAGACGACGCGATTATCGTCCTCGACCCTGTCAACCGCG
ATGTCCTCGACAACGGTCTCAAAAACGGCGTGAAAAACTACATTGGCGGCAACTGCACCG
TTTCCCTGATGCTGATGGCTTTGGGCGGCCTGTTCCAAAACGATTTGGTCGAATGGGCAA
CCAGCATGACCTACCAAGCCGCTTCCGGCGCGGGCGCGAAAAACATGCGCGAACTCATCA
GCGGTATGGGCGCGGTTCACGCCCAAGTGGCGGACGCGCTTGCCGATCCTGCCGGCTCGA
TTCTCGACATCGACCGCAAAGTATCCGATTTCCTGCGCAGCGAAGACTATCCGAAAGCCA
ACTTCGGCGTACCGCTCGCCGGCAGCCTGATTCCGTGGATTGACGTGGATTTGGGCAACG
GCCAGTCCAAAGAAGAATGGAAAGGCGGCGTGGAAACCAACAAAATCCTCGGCCGCAGCG
ACAATCCAACCGTGATTGACGGCCTGTGCGTCCGCGTCGGCGCGATGCGCTGCCACAGCC
AAGCCATCACTCTGAAGTTGAAAAAAGACCTGCCTGTTTCCGAAATCGAAACGATTTTGG
CAGGCGCGAATGACTGGGTGAAAGTCATCCCCAATGAAAAAGAAGCCAGCATCCACGAGC
TGACTCCTGCCAAAGTTACCGGCACGCTGTCCGTCCCTGTCGGACGCATCCGCAAACTGG
GCATGGGCGGCGAATACATCAGCGCGTTCACCGTCGGCGACCAACTTTTGTGGGGCGCTG
CCGAACCGCTGCGCCGCGTATTGCGTATCGTGTTGGGCAGCCTGTGAGCCCTGTTTGAAT
GGAAATGCCGTCTGAAGCCTGTTTCAGACGGCATTTTCCTTGCAACCCTGCCGGATAACG
CCCTGCCGGCACTGCCGACGTAAAAAATAAAGGATTCCATTTCCGGCGGTATGCGGCAGC
CCGACTTTATCCGAACCTGATGCGCCTGCACGTCAATGAAAACAGCCCGATTGCGGACTT
CCTGCTACAGCCGAAATTCCGATAAGGCAAGCGTTCACGCCAGCAACATTTCCTGCATCA
GCTTCATACCCCACTGCCAGCCGCCGAGCATGCCGTTCAAACTGCCCGAATGCGGGGAAA
CCAACAGGCGGGCGTTCCACAAATCCGCCTGTTTTTGCGCCCAACCGTGCGGCACGCCGC
CGTGTTCGGGTACAACCAATGCGGCACGGCAGGGACAGCGGACGCGTTGGAAAGCGTGTT
CCGCATCGTCGGGAAAAATATCGGGACGCTGCGGTACAAGGATGATGTTGGCAATTTTCT
TCCGTGTCAGGATGTCTGCCTGATACAGCCACGCCAAAAATGCGGCCGCGCCCGCACCGT
GTGCGACAACGGCGACGTATTTGCCGCGTATGCGTTCAAATGCCGTCTGAAGCCCTGCCT
GCCATTCCCCTATGCTTTGACCGGCCGACGCTTCGGACATCTGCACGACGGGATAACTGA
TCGCCCAACGGTCTATCCACATCTGATCCTCTCCGGCATCGCGTATCAGCCAAAGCGTCA
AATCTTCGAGTTCAAAACCCTGCATACCGCCCCGCCTATTTCAGCAGGTCCCGGAGGGTA
AAGGCGATGAGCAGCGAAGCGGGTACGCTCAATATGGCGCAGACGGTCAGGCAGGCAAAA
ATATTCACCACCCGCCGCCAGCCTTTCCAACCCAAACATTTGGACGCAATCAGCAGGCAG
GGCAGGCAAATCAGCAGCCACACCAACGCCCATATCGGGTTTGCCTTGGTCGGCGCAAGC
CAGCCTTGCATCCGCGACAACATAAATATCGCCCACACCAACATGGGCAGGATAAACGCA
GCGACGACCCATGCCGCGCCTATTCCTGTTTTTCCGTCCACATTCCAATCATATTTACCC
AAAACCTTATTCGGCAGCATAGTCATACTCCACGACCAGCGGCGCATGGTCAGAAAATTT
TTCATCTTTATAAACGTGTGCGGACACGGCTTTGGCAGCAAGTTCGGGCGTAACCATCTG
ATAATCGATGCGCCACCCGACATCTTTCGCATACGCCTGCCCTCGGTTGCTCCACCAAGT
GTAGCCCGGCACATCGGGATAAAGCGTGCGCCACATATCCGTCCAACCGAGCTTGTGGAT
AACCTTGCCTATCCACTCGCGCTCTTCAGGCAGGAAACCTGAATTTTTCTGGTTGCCTTT
CCAGTTTTTCAGGTCGATGTTTTGGTGGGCGATGTTCCAGTCGCCGCAGACGACAATGTC
GCGCCCTTCGTTTTTCATCGCTTCGAGCATAGGGTAAAACGCATCAAGGAAACGGTATTT
CACCTGCTGGCGTTCTTCCGCGCTGCTGCCGCTGGGCAAATAAAGCGAGATAACGCTCAA
CCTGCCGAAATCGCAACGCACAAACCGCCCTTCCCTGTCGAATTCTTCAATGCCCATACC
GATTTGCACATTGTCGGGTTTGCGTTTGCTGTACACCGCCACGCCGCTGTAACCGCGCTT
GTCGGCGCAATGCCAATGACCGTGCATCCCGTGCGGATTTTTCATATCGGCAGACAAATC
AGCCTCCTGCGCTTTGAGTTCCTGCACGCAGACAATGTCCGCGCCCGATGCGGCGATGTA

## Appendix A

TTCGTAAAAACCTTTTTTGTAGGCGGAGCGGATGCCGTTGACGTTGGCGGAAATGATTTT
AAGCATAATAAAAATAAGTTCTCACAATAAAAATGCCGTCTGAACAAAAAAGGGCAAAAT
GCGGCACATTTACCCTTTTCGATGGATTTTAACCGCGCCGCCAAGTCGTGCCGCCGGCGT
TGTCTTCCAAAATGATTTTGTGTTCGTTCAGAAGGTCGCGGATGCGGTCGGATTCCGCCC
AGTTTTTATCGGCGCGCGCCTGTTTCCGCCGGGCGATCAAGTCTTCGATTTCTTCGTTGG
AGAGACCGTCTGAAGCCGCGCCGCCTTGCAGGAACTCGGTCGGATCGCGTTGCAGCAGTC
CGATGATGCCGCCCAAGGCTTTCAGACGGCCTGCCAGTTGCGCGTCATTGGTTTTGTTCA
CTTCGCCGGCAAGTTCGAACAACACCGCCACCGCTTTCACCGTATCAAAATCATCATTCA
TCGCAACATAAAAGCGGCGCGTGTAGTCATCGCCGGCTTCAGACGGCATCGGATCGGCGG
GCGGCGTATTTTTCAAAGTCGTATACAAACGCGTCAACGCGCCTTTTGCATCATCCAAAT
GCGCGTCGGAATAGTTCAACGGGCTGCGGTAGTGGGCGCGCAGGATGAAGAAGCGCACGA
CTTCCGGATCGTATTGTTTCAACACTTCGCGGATGGTGAAGAAGTTGCCCAGCGATTTGG
ACATCTTTTCGCCGTCCACGCGGATAAAGCCGTTGTGCAGCCAGTATTTGACGTGGCTGG
CGATGCTTTGCCCGTGGTGGGTTTGCGCGTGATGATGACCGCAGGTATGCCCCGTCGCGC
CGACGCTTTGGGCAATTTCGTTTTCGTGGTGCGGAAACTGCAAATCCGCGCCGCCGCCGT
GGATGTCGAAGGTATCGCCGAACAGGTTTTCACTCATGGCAGAGCATTCAATGTGCCAAC
CCGGACGGCCGTTGCCCCACGGGCTTTCCCACGCCGGTTCGCCTGCTTTGGCGGCTTTCC
ACAACACAAAATCAAGCGGATCGCGTTTGAAACCGTCCACTTCCACGCGTTCGCCCGCAC
GCAGGTCGTCCAACGATTTGCCCGACAATTGTCCGTAAGCGGCAAACTCGCGCACGGCGT
AGTAAACGTCGCCATTTGCGGCAGGATATGCCTTGCCGTTTTGAATCAGGGTTTCAATCA
TGGCAATCATTTGCGGAATGTTTTCCGTTGCCTTCGGCTCAATATCCGGACGCAACACGC
CCAAAGCATCGGCATCTTCGTGCATGGCTTGGATGAAACGCGCAGTCAGTTTGCCGATGG
TCTCGCCGTTTTCAGCCGCGCGGGCAATGATTTTATCGTCGATGTCGGTGATGTTGCGTA
CATAAGTGAGCGGATAGCCGCACTCGCGCAACCAACGGGCAATCATGTCGAACACCACCA
TCACGCGGGCGTGTCCCAAATGGCAGTAATCGTAAACGGTCATACCGCAGACGTACATAC
GCACGTTTTCAGGGTCGATGGGGGAAAAGGGTTCTTTTTGACGGGTTAGGGTGTTGTAGA
TGGTGGTCATGGGATTATGGATTAATCTTTGTTGCTCGGATGATAATTTCTGTTCTGTTC
CTGTAGATACGGACCAAGGAACATTACGTAGTTGCGGATTATTAATATGGCTGATATTTG
TGAAAATTGGTTCTGCATAACAGTTTGCAAAATTTTTTGTAAATTCTGATAATTTAAACT
TATCTTTTAATAAGTTTGCTAAATCTGATGACGAGGGATAAAGTTTACTTCTTATACTAG
GCATTTCAATATGAAGGACTATTTTTATTTCGTTACAATCTAAAGCCAAGCGAGAAAAAT
CTTTTTCTTCCTGTTTTTCTGCTTTAAATTTAGCAGAAACCAATCCTGCCAATGAATCTC
GAATTTTTCTTGCGATTAAATATGGTAGGTCAGAAAGTTTTTCATCTATTGATTGGGCAT
TTGGCTCAAGCCCAAGTCGGTAATAATCTTTAATTTCGATTAGCCAAAGTGTCGATTCAT
GAAGGGCTATTATATCTACACCTGAGCTGCCATTATCGTCATCTACACTTTGATTTATCC
CGTTCTTTCCCTTTTCATTTGTATCAATTTTATTACGTAAATTACAACTGTTCTGAAAAA
TTTTATAATGTTCCCATTCGTCATACTTGGTAACGTAATAATCTTCAGGAAAAGCAAAGG
TTAATCTCTTTTCTGTGATTGTAGTCATAGCTTAACCTCAAATATTCAGATACCTGTCTG
CCTGCATAATGTTTTCATCTAACAATATCAATGTGTTCAAATCATTAATACTGTTCCCTT
GCTCCACTTTTGTTCCATCATCGGAAGCAATCAACGAGAAAAAACGTACAGGTAAATCCG
TGTTATTTTCAAGCTTCAAAAGTTCCAATTCTCTCAATAAGAATAAAGAGTGTGTTGCAA
TAAAAACCTGAATACCCTGTTGAGATAAAGACCAAATAATACGGGCAGCCACTTTGATCA
ATTTAGGATTCAGATTAGCTTCCGGTTCATCCCAAAATAGATAGCCTTTATCCAGCAATG
CCCCTGTTGCGATTAACCGGGCAATCATGACAAATTTCCGCAAACCCTCTGCTACCAAAG
GTGCTTCAATCTTACCGCCCGTATTTGTCAGCGATAGATAAAACCTTCCTTGTTCTTCAG
ATACTTTTCCGCCCATCGCGTTCTCAATAGGTTCGAGCAATTCTCGAATTTTTGTTTCTC
TGGGGCCTTTGGCAAGCGGGTGATTTAATTGCATACAGGTATCAAACCAAGTTTCTTCGA
AAGGGATGCTTTGGTTTTGATACAAAGAAGTGAACCAAGGGCAAAGTGTAATTAATTCGC
GGCTGGGTAAGAAGATAGGTGTCGGAGTATATTCATTTTCTTTCAATCCGATGCTTTGAA
CATTGACTTGCGATGATGAGTTACTGGAAAAATTCAGACTACTATGCGTAGTGCCGTTTT
GCAGTTTTAAAACGATTTCCGTACGCCCGCGCCCCTGCAAACGTTTGCTCAACCTACCCA
AGGAATCGGGACGGAAAACATTCAGTAATTTATCGGCAAAACTTTTTTGCAATTCTGTTT
TCAGTAATCTGTTTTTGGTGTTAGATGTTACTTCTAGCAGGCTGTATAAAATTTTTAACA
AATGTGTTTTGCCACAACCGTTTTCGGCAACAATAACATTGAGATTTTCAGAAAATTCAA
AAGTATCGTTTGGAAGAACGGTAAAGTTTGTCAACTCAAGCGACTGGATATATTGGTTAG
ATGACATTTTTAATCCATTTCAATCTTGCTTTAAAATTGTTTCAAACAACCTTTTGTAGA
ACAAATATCGTCTGAAACCCTTTCTTTTTTCACTCCGGCTTAAACACGCCTGTATCCGTT
TTAGGCTGCTGTTCGATAATTTCAACATTTGCCGCTGCTTTCTCCGCTTCTGCTTTTTCA
GCTTCGATACGTTTTTTCTCGGTCAGGTATTGGTTGATTTGGTGTACCAATTCCTGCGTG
CCTTGGTGGGTCAGCGCACTGATTTGGAAGAGGCGCGGGGTTTCCATGTCAAATTGGAAA
CGGTCGTCGGGTTTGGGGTAGTCCCAGCCGACGGCTTCGAGGAAGGCGGCAGTGCGCGTT
TGGGCTTCTTCTTCGTCAAGCATATCGAGTTTGTTCAGTACCAGCCAGCGCGGTTTGCCG
TAGAGTTCTTCGTCGTATTTGCGTAATTCGTTGATGATGGCGAGTGCTTCTTCGGCGGGG
TTGACGGTTTCGTCGAAGGGCGCCAAATCGACGACGTGCAGCAGCAGGCCGGTACGTGAT
AAGTGTTTGAGGAAACGATGGCCGAGGCCTGCGCCTTCTGCCGCGCCTTCAATCAGGCCG
GGGATGTCGGCCATCACGAAGCTGTGGTTTTCGTCGATGCGTACCACGCCTAAGTTTGGA
TGCAGGGTGGTGAAGGGGTAGTTGGCGATTTTGGGGCGTGCGGCGGATACGGCGGTAATC
AGGGTGGATTTGCCGGCGTTGGGCATACCCAATAAGCCGACATCGGCGAGGACTTTAAGT
TCGAGTTGCAGGGAACGGGCTTCGCCTTCTTCGCCGGGGGTGGATTGTTTCGGGGCGCGG
TTGACGGACGATTTGAAGTGGATGTTGCCCAAGCCGCCTTTGCCGCCTTTGGCGAGGCAG
ACGCGCTGTCCGTGATAAGTGAGGTCGGCAACGGTTTCGCCGGTGTCGAGGTCGCGGATA
AGGGTGCCGACGGGCATTTTGAGGACGATGTCGTCCGCACCTGCGCCGTAACGGTCGGAA
CCGTGGCCTTTTTCGCCGTTTTTGGCTTGGTAGCGTTTAACGAAGCGGTATTCGACGAGG
GTGTTGGTGTTTTCGTCGGCTTCTGCCCAGACGCTGCCGCCTTTGCCGCCGTCGCCGCCG
TCCGGGCCGCCGCGCGGTACGAATTTTTCGCGGCGGAAACTGGTTGCGCCATTACCGCCT

## Appendix A

TTGCCTGCGGCGACTTCGATTTTTGCTTCGTCGATGAATTTCATTCAATGCTCTTGTTTG
TTGGTTTCAAATGGGGGGTTCAGACGGATTACCGTGTGTTTTGATGCCGTCCGAACAGAA
TTTCGGACGCTATTATAAGGGATAAGCGGTATTTCAACACGCCGTACCCAAACTATTTGT
TCCGCCCATCTTAATGAATTTTTAAGCAAATCTTCAGCCTGCAAACAAAATTATGTCCAA
CTTCTTTGGTACAATCGCGCCTTTTTGACATTCCGACCCGACGGAATGTCCGTTCAAACC
GTTACATATAATAAGTTTTTTATGAACACAAACCAACCTGCCGTTTACGACCCGTTGACA
CGCGCGCTGCACTGGCTGACCGTTGCCGGCTTCATCGGCATTCTGACCACCATTGTCCTG
TGGACGATTTATAGTGGATTAACAAAAATCAGGACAAGGCGACGAAGCCGCAGACAGTAC
AAATAGTACGGCAAGGCGAGGCAACGCCGTACTGGTTTTTGTTAATCCACTATACGAAGA
GGCGGAATGGGTGGGCAGCCTGTTCGGCCTGCACAAATCTTTCGGTTTCCTTACGCTGAC
GGTGATTACATTGCGCATCGTGTGGGCGGTTGCCAACCGCGCCAAGCGTCCGCAAAGCGA
CTGCAAGGCTGCGGCGGCAGGCCACGGCATTCTGTATCTGCTCATGCTTGCTGTTCCCGT
TATCGGCATGATCCGCCAATACGGCAGCGGCCGCGGCCCGTTGAAAGTGTTCGGCGTTGA
AGTGATGCAAGGGTTCGCCGGAAAAAATCGAGTGGATGGCAAACTTGGGCAACACGTTCC
ACGGCAATTTGGGCTTGCTGCTGTTTGCCGCCGTCGCCGGACACGTCGCCATGGTCGTCG
CCCACCGTGTTCAGGGTAGAGATGTTCTGTGCCGCATGACGGGTCGTGTCCGCTGATTCC
GTTCACACTATGGTGCCGGCTCGTCCGGCACTATTTGTTTTTCCAAGACAGAGCCAGATC
GTACAAAGCTTTCTTTCCCTCGCCCGTGATTTTGGCAGCAAGCTCCGCCGCCTGTTTGGT
CGGCAGCTCGGCTGTGAGGATTTTCATGATGTTTTGCGCGGACTCGGACAAGCCTTCGTG
TTTTTCATCCTGCGCCGGATAAAGCACCAACACCATCTCGCCGCGCGATTGGTTGCCGTC
GGCAGACAATGCCGTCTGAATTTCCCCAACCGTGCCGCTTAAGAACGTTTCAAACGTTTT
CGTAATTTCGCGCGCCAGCATTAATCGGCGTTCGGGGAACAGTTCCGCCATATCGGCAAG
CGTCGCACCGATGCGGTGCGGCGTTTCAAACATGACGATAGGAAACGCCGCCCGCACCCA
TTTGGCAAACAGTTTCCTGCGTTCTCCCGATTTCGGCGGTACAAAACCGTTGAAATAAAA
ATCGGATCCTTCCACACCGGCCACGCTCAAAGCCGCCATCACCGCGCTTGCGCCCACGAC
GGGAACGACTTTAAACCCGGCCTCACGCACGCGGCGGGCGAGTTTCGCGCCCGGGTCGCA
CACGGCCGGCGTACCCGCATCGGAAACCTGTGCCACAACCATGCCGTCTGAAAGATAGCC
GACAATCTTGTCCGCCATCTGCCGTTCGTTGTGTTCGCGCACACTGACGAGTTTGCCCTG
AATGCCGTACGCGCTCAAAAGCTGTGCGGTAACGCGCGTGTCTTCGGCACAGATGATGTC
CGCCTTTTGCAATACCGCCAAAGCGCGCAGGGTAATGTCCGCCAAATTGCCGATGGGCGT
GGCAACCACGTATAATGTCCCTCCGACGACGCTGTCGGAGGCTTTCTGCAAATGTTTCTG
AAACATAAGAATGCCGTCTGAAAAACAAACATTATAAAGGTTAAACCGATTATGCGCCTA
AACCACAAACAGGGCGAGGCAGGGGAAGATGCCGCGCTTGCCTTCCTCCAATCCCAAGGC
TGCACGCTGCTTGCCCGCAACTGGCACTGCGCCTACGGCGAAATCGACCTGATTGTCAAA
AACGGCGGCATGATTCTGTTTGTTGAAGTAAAATACCGCAAAAATCGGCAATTCGGCGGT
GTCGCATACAGCATTTCCCCATCCAAATTATTGAAACTGCAACGAAGTGTAGAGTATTAT
CTGCAACAGAACAGGTTGACAAACGTACCGTGCCGCCTCGATGCGGTACTTATCGAAGGC
AGCCGCCCGCCCGAGTGGATACAGAATATTACAGGTTGACGATATGACGACATTACAAGA
ACGCGTTGCCGCCCATTTTGCCGAAAGCATCCGTGCCAAGCAGGAAGCCGGAAAAGTATT
GGTCGAGCCGACCGTACAGGCTGCCGAGCTGATGCTGCAATGCCTGATGAATGACGGCAA
AATCCTGGCCTGCGGCAACGGCGGTTCGGCTGCCGACGCGCAACACTTCGCCGCCGAAAT
GACCGGCCGTTTTGAAAAAGAACGCATGGAACTCGCCGCTGTCGCGCTGACAACAGACAC
TTCCGCGCTGACAGCCATCGGCAACGACTACGGTTTCGACCACGTATTCAGCAAACAGGT
GCGCGCGCTCGGACGTGCAGGCGATGTATTGGTCGGCATTTCCACCTCCGGCAATTCCGC
CAACGTCATCGAAGCCGTCAAAGCCGCACACGAACGCGATATGCACGTCATCGCCTTGAC
CGGCCGCGACGGCGGCAAAATCGCCGCCATACTCAAAGACACCGACGTTTTGCTCAACGT
TCCCCATCCGCGCACCGCCCGTATTCAAGAAAACCACATCCTGCTGATACACGCCATGTG
CGACTGTATCGACTCCGTACTGCTGGAAGGAATGTAACCCTTTTCAGACGGCATGGCGCA
AAGCAATGCCGTCTGAAACGCCCAAGAAAGGAAGCACCCGATGAAACCCAAACCGCACAC
CGTCCGCACCCTGATTGCCGCCATTTTCAGCCTTGCCCTTAGCGGCTGCGTCAGCGCAGT
AATCGGAAGCGCCGCCGTCGGCGCGAAATCCGCCGTCGACCGCCGAACCACCGGCGCGCA
AACCGACGACAACGTTATGGCGTTGCGTATCGAAACCACCGCCCGTTCCTATCTGCGCCA
AAACAACCAAACCAAAGGCTACACGCCCCAAATCTCCGTCGTCGGCTACAACCGCCACCT
GCTGCTGCTCGGACAAGTCGCCACCGAAGGCGAAAAACAGTTCGTCGGTCAGATTGCACG
TTCCGAACAGGCCGCCGAAGGCGTGTACAACTATATTACCGTCGCCTCCCTGCCGCGCAC
TGCCGGCGACATCGCCGGCGACACTTGGAACACATCCAAAGTCCGCGCCACGCTGTTGGG
CATCAGCCCCGCCACACAGGCGCGCGTCAAAATCGTTACCTACGGCAACGTAACCTACGT
TATGGGCATCCTCACCCCCGAAGAACAGGCGCAGATTACCCAAAAAGTCAGCACCACCGT
CGGCGTACAAAAAGTCATCACCCTCTACCAAAACTACGTCCAACGCTGACTCGGCAATGC
CGTCTGAACCGCCTTCAGACGGCATTGCCCGACACCCCAAAAGCACAATCAAAATGGCAA
AAAAACCGAACAAACCCTTCAGGCTGACCCCCAAACTCCTGATACGCGCCGTATTGCTCA
TCTGTATCGCCGCCATCGGCGCATTGGCAATAGGCATCGTCAGCACATTCAACCCGAACG
GCGACAAAACCCTTCAAGCCGAACCGCAACACACCGACAGCCCCCGCGAAACCGAATTCT
GGCTGCCAAACGGCGTAGTCGGACAAGATGCCGCCCAACCCGAACACCACCACGCCGCCT
CATCCGAACCCGCACAGCCGGACGGCACAGACGAAAGCGGCAGCGGACTGCCGTCCCCTG
CCGCACCCAAGAAAAACCGGGTCAAACCGCAACCTGCCGACACAGCTCAAACCGACAGGC
AGCCGGACGACGCCGGAACACAAGCTGAAAACACACTCAAAGAAACCCCCGTACTGCCCA
CAAACGTCCCCCGTCCCGAACCCCGAAAAGAAACACCCGAAAAACAGGCGCAGCCCAAAG
AAACGCCCAAAGAAAACCATACCAAACCGGACACCCCGAAAAACACGCCGCCCAAACCCC
ATAAAGAAATTCTCGACAACCTCTTCTGACCCGGCACGGCAGGCACACCCGCAATCCAAG
GAAGCATTATGAACGGCATCATCATCAAAACCCCCGAAGAAATCGAAAAAATGCGCGAGC
TGGGCAAACTCGTCGCCGAAGCCCTCGACTACATCGGACAATTCGTCAAACCCGGCGTAA
CCACCGACGAAATCGACAAACTCGTTTACGACTACCACGTCAACGTCCAAGGCGGCTATC
CCGCCCCCCTGCACTACGGCAACCCGCCCTACCCCAAATCCTGCTGCACCTCCGTCAACC
ACGTCATCTGCCACGGCATTCCCGACGACAAGCCGCTCAAAGAAGGCGACATTATCAACA

## Appendix A

TCGACCTCACCATCAAAAAAGACGGCTTCCACGGCGACTCCAGCCGTATGTTTACCGTCG
GCAAAGTCTCCCCCATCGCCCAACGCCTGATCGACGTAACCCACGCCTCCATGATGGCGG
GCATAGAAGCCGTCAAACCCGGCGCGACACTGGGCGACGTAGGTTACGCCTGCCAACAGG
TTGCCGAAAACGCCGGCTATTCCGTCGTACAGGAATTCTGCGGACACGGCATCGGGCGCG
GTTTCCACGAAGCCCCGCAAGTGTTGCACTACGGAAAAAAAGGACAGGGCCCCGTTCTAA
AACCGGGTATGATTTTTACCGTCGAACCGATGATCAACCAAGGCAAACGCCACCTGCGTA
TCCTCAACGACGGCTGGACGGTGGTTACCAAAGACCGCTCCCTCTCCGCCCAATGGGAAC
ACGAAGTCTTGGTGACCGAAACCGGCTACGAAATCCTCACCGTCAGCCCCGCCTCCGGCA
AACCCTGAAACCGGACGTATCCGCCCCATAAAAACAAACAATGCCGTCTGAAAGAAACGG
CAGATATGATATATAATATAAAAACAGGCTTGACCCGGCACATTACGAAAACAAAGCAAA
TCGGAATTTGCCCCGCAACCAGACAAACTTAAAGGAAGTTTTATGAAAATATTTGAAAAT
ATAGAAGATGTTAAAGCCATCCGTAAAAAGACCGGGCTGAACCAGATAGACTTCTGGGGC
AAGGTCGGCGTTACCCAGTCCGGAGGATCGCGCTACGAAACCGGCCGCAAAATGCCCAAA
CCCGTACGCGAACTGCTCCGCCTCGTCCATATCGAATGCATCGATTTGGCGAAAGTCAAC
AAAAAAGATATGGAAATCGCCGCCCTGTTGAAAAAACACCATCCCGACCTGTATGCCGAG
TTGTCCAAACAGACCAAGTCCGAAAGAAAAAAACAAAGTTAAACCGCAACCTCCGGATGC
CCGACAGTTTTTCATTTCCGAAAAACGCAAACAATGCCGTCTGAAACACCGGACAGGTCG
CCGTATCCCGCCTGCCGCCCCTGCCTCAAACCGCCGAACCGCCCGAACCCGCCTTTTTAC
AAACTTTATCCAATTTCCTGTTTATTTCGGGATACGCCGACATTAGAATGTCAAACAGCT
CGAAACGGGCAAACTCCACATCCATCCAAAGGAATAAAAATGAAACTTCTGACCACCGCA
ATCCTGTCTTCCGCAATCGCGCTCAGCAGTATGGCTGCCGCCGCTGGCACGGACAACCCC
ACTGTTGCAAAAAAAACCGTCAGCTACGTCTGCCAGCAAGGTAAAAAAGTCAAAGTAACC
TACGGCTTCAACAAACAGGGTCTGACCACATACGCTTCCGCCGTCATCAACGGCAAACGC
GTGCAAATGCCTGTCAATTTGGACAAATCCGACAATGTGGAAACATTCTACGGCAAAGAA
GGCGGTTATGTTTTGGGTACCGGCGTGATGGATGGCAAATCCTACCGCAAACAGCCCATT
ATGATTACCGCACCTGACAACCAAATCGTCTTCAAAGACTGTTCCCCACGTTAATCAGGC
AACAAAAAACAGCGTTTTCAGAAATGAAAACGCTGTTTTTTTGACCGTTCCATTATTCAC
AAAAGGGAAAAAACGATTACCTGCCCCGTGTATCAAAACCTGCCCTGCCGGATGAAGGGC
ATAACCGGCAGGGACGGCGTCAACACCATATGGGGGTACGGCTTTTCTTGAAAGATTCGG
CTTAAATATCCAATACTTTCGCGGTATAGGCGATAATTTCATCCGCCCTTTCAGGGTTTT
CGTTCAACTTGATGCCGTAACCCGGTACCAGCTCTTTCAGACGGTCTTCCCAAGACGGGG
CGCGCTCGGGGAAGCATTGGTGCATCAGCCGGATCATCAGCGGCACAGCGGTCGATGCGC
CCGGCGACGCGCCCAGCAATGCGGCGAGTGAGCCGTCGGCGTGGGCGACAATCTCCGTAC
CAAACTGGAGCACGCCGCCTTTTTCGGAGTCTTTTTTAATGATTTGGACGCGTTGCCCTG
CGGTGATGAGTTCCCAGTCGTCGGGGTTTGCCTCGGGGTAGTATTCCAGCAGGGAGGCGA
AGCGTTCTTCTTTGGTTTTACGCAATTCGCCCAGCAGGTATTTGGTCAGCGGCATATTCG
CCCAGCCGGCGCACAGCATAGGATAGAGGTTGTCCATATGGATGGACAGCGGCAAATCCA
TAAGCGAGCCTTGCTTGAGGAAGTTGGAACGGAAGCCTGCGTAAGGGCCGAACATAAGGT
GGCGTTTGCCGTCCACGTTGCGTGTGTCGAGGTGCGGGACGGACATCGGCGGCGCGCCGA
CGGAAGCCTGCCCGTACACTTTGGCGTTGTGTTGTTCGGCGGTTTCGGGGTTGCTGTTGC
GGAAGAACAGGCCGGACACGGGGAAGCCGCCGTAGCCTTTGCCTTCGGGGATGCCGGATT
TTTGCAGCAGGGTCAGCGCGCCGCCGCCCGCGCCGAGGAAGAGGAAGCGGGTACGGAGGG
TGAGCTGCCCGTCGGGGTTGCGGGTATCGGCGGTTTTGAGCACCCACGCGCCGTCGGATT
CGCGTTTGATGTCTTCGACGTGGCGGTTGAACTCGGTTTTTACGCCCTTGCCCTGCAAAT
ATTTCACCATTTGGCGCGTCAGCCGTCCGAAATCGACATCCGTACCTTCGGCGGAGTAGT
TGGCGGCGACGGGTTGGTTTTCGTCCCGGCCGCGCATCATCAGCGGAGCCCAATCGGAAA
TTTTGTTCCGATCGGTGGAAAATTCCATATTTTCAAAAAGTTTTTGGGTTTTAAACGCGT
CATAACGTTTTTGAAGATAAGAACAATGGTCTTCATTCATCACCAAAGACATATGCGGCA
CGGCATTGATGAAGGAATTGTCTTCCAACTTGCCTTCCGCGACCAGCGTCGCCCAAAACT
GGCGGCTGACATGAAACTGTTCGGCAATATTGAGGGCGCGCGCCGGATCGATAATCCCAT
TTGCACCCAACGGCGCATAGTTCAATTCGCACAGCGCGGAATGCCCCGTGCCGGCGTTGT
TCCACGCGTTTGACGATTCCAACGCCACATCTTCCAAGCGTTCAATCAGGGTGATTTCCC
AAGACGGTTCGAGTTCTTTGAGCAAAACGCCCAAAGTCGCGCTCATAATGCCGCCGCCCA
CCAAGACAACGTCTGTCGCTTCAGCCATGGTTTACTCCTAAAAAACAGGCATCTTCTGCC
CTTATGGTTATTTGCCGTACTACAAACGCCTGAATCGCAAAAGCAGGGAAAACCGGCAAT
GGTGTGTGTCCGAGTATGCTGTTTCGGGGTTGGAATGCGTTGCAAGCATGGCTTCCGACA
CCGCTTCAGGGGCTTGTAATATGTTATCGTGAATGTAGTGGATTTTACTGGGAAATGCAA
AGTTTTTCTGTCGCCCGCCAAGTCGGGAAACTGCGAAATGAAAAATAAAAATAGTTATTT
ATCTATATATATCAAATTTTTAATAGATAAAAAATCAAAATTGTTTATATATTAATTTTT
AAAAGATTGTCAGCATATTGCGTTAAGTTTTTATAGTGGATTAACAAAAATCAGGACAA
GGCGACGAAGCCGCAGACAGTACAAATAGTACGGAACCGATTCACTTGGTGCTTCAGCAC
CTTAGAGAATCGTTCTCTTTGAGCTAAGGCGAGGCAACGCTGTACTGGTTTTTGTTAATC
CACTATAAATTTGAAAATACTGCCTCACACCTGCACGCCATACCCTGCCAACCTGCCGGT
CAGGATTTCCCTGTTTTTGCACCAATCTTCCCTCAGCATACTGTACACGACCGTATCGCG
CACACTGCCGTCTTTACGGAGCATATGCATACGCAGCACGCCGTCTTTTTCCGCACCCAG
CCGTTCGATGGCACGTTGCGAGGCAAGGTTCAGAATATCCGTGCGCCATCCCACGCAACG
GCAAGCCAAAACATCAAATGCGGAATCCAACAGCATGATTTTGCAACAGGTGTTTATCCG
TGTCCGCCGTGCCGATGCCGCATACCATGTGAATCCGATATCCAAACGCGGAATCTGCGG
TTCAAAATGATAATACGCCGTTGTCCCGACCACCCTGCCCGCCTCTTCATCGACAACCGC
AAACGCCAAACGCGTTGCCAATGCTGTCCCGATATAGTCTGCCACCCTATCCGGATGGGG
CGCGGACGTTACCCCCAGCTTCCAAACCTCCCCATCGCAAACCGCCTCGCGCAAACCCGT
TTCATGATGCACATCCAACGGTTCGAGACGAACGCCGCCCAACGACAAGACCGGCAGTAT
TATCTTTTCCGACATCCTTTTCTCCCAATATTCCGCCTTCAGACGGCATTTCCGCCCGGA
ATGCCGTCTGAACGGCTAAAAACACAATATCCCCGCCTCCGACACAAAACCGTCCAAAGA
CCGGTCGTGCGCCTCGACCGGCAGCCTGTCCACCAACTGGCAGGCAAAGCCCACGCCCAC

## Appendix A

GGTTTTTGCCTGCAAACGGTATTTCATCGCTGAAAGCGTCGCATCGTAATAGCCGCCTGC
CTGTCCCAAGCGGTAGCCCAGCCTGTCCATACCGACCACTGGCACAAGCAGGAGGTTCAA
ATCATGCACACGCTTTTTCCGACCTGCAAACTGAGGGACATGCAGCTTCGCCCTACCGCG
CTTGCGTTCTTGTTTTACTCCATCGGCAGGATACGGCGTAAACCACATCCGCCGCGAACG
CGGTTCGATATAAGGCAGGTAGAGTTCCGCACCGCGTTTTTGCGCCGCGCGGACAAAGCC
GTCCAAACGCAATTCCTTGCCCATCGGCCAATACACGCCGATTTTCCGCCCTTTTTTAAT
ATAACGTTTGAGCAGGTGGTTGATTTTTACCGTTGCCGCCGCCCGCACGTCCCGCCCCAT
TTGCGAACGCCGCCCGCGCAATTCGCGGCGCAGGGCGCGTTTTTCCTCGTTCCTCATTTC
AGACGGCCTTTCAGGATTGCGGTAGAATGTTGCGATTATAACGATTTTGTTAACATTCAA
ACAGGACGCACACAATGTGGCACATCGTCGCCATCGGCTATCTTTTTGTTGCCGTTATGT
ATTCCGCCGCGCAGCCGAGTATTGCGCGCGCCTTGATTTATTTGGTTTTTTGGGCGGTGC
TGCCCACCGTGTTCACGGTTTTCACCATTACCGTCCGCCGCCGCAACCACCTGATGAGGC
AGCAGGAACAGGCGGAATCCGAACAGCAGCGCGCACAACGGCAAAAAGACAGCGGCACAA
AACCCTGAATCCCTTTTCAGACGGCATCTTATCCGCTATAATCCGTCAGTTTTCCATTTC
GGAAACACACTATTTTTTAAAACTTATGCCCACTTTCGCCGAAGGGTGCTTGACAATAGG
CGTGACCTATCAAGTTCTATGCGATTGAATGTGTGCTCTTAACCCTTTCAAGGAAATAAA
ATGTCTCAAATTACTATGCGTCAGATGATTGAAGCCGGTGTTCACTTCGGCCACCAAACC
CGTTTCTGGAACCCGAAAATGGCACAATACATTTTCGGTGCGCGCAACAAAATCCATATC
GTCAACCTGGAAAAAACCCTGCCGATGTTCCAAGACGCGCAAGAAGCCGTACGTCGTCTG
GTTGCCAACAAAGGTACAGTATTGTTCGTAGGTACCAAACGCCAAGCCCGCGACATCATC
CGCGAAGAAGCGACCCGCGCCGGTATGCCTTTCGTCGATTACCGCTGGTTGGGCGGTATG
CTGACCAACTACAAAACCGTTAAGCAATCCATCAAACGCCTGGAAGAAAAAACCGCAGCC
TTGGAAAATGCTGCCGAAAGCGGTTTCAGCAAAAAAGAAATTCTGGAAATGCAACGCGAT
GTTGAAAAACTGGAACGTTCTTTGGGCGGTATCAAAAACATGAAAGGCCTGCCTGACGCG
ATTTTCGTTATCGATACCGGCTACCAAAAAGGTACTCTGGTTGAAGCTGAAAAATTGGGC
ATCCCTGTTATCGCCGTAGTCGATACCAACAACAGCCCCGACGGCGTGAAATACGTTATC
CCCGGCAACGACGACTCCGCCAAAGCCATCCGCCTGTACTGCCGCGGCATCGCTGACGCA
GTTTTGGAAGGCAAAAACCAAGCGCTGCAAGAAACCGTAGCCGCTGCCCAAGAAGCCGCT
GCCGAGTAATCCGGCAAACCGAAGAGGGGCGTTATGCCCCTTTTCTCAAATATGCCGTCT
GAACGTCCGTTCGCGGCACACGATTCCCGAATGCGGAAAATCCTTTCCGTATTTCCCAAA
AATCTAGGAGATTCAAAATGGCAGAAATTACTGCAAAAATGGTTGCCGACCTGCGCGCCG
CTACCGGCCTGGGCATGATGGAATGCAAAAAAGCCTTGGTTGAAGCCGAAGGCAACTTCG
ACAAAGCCGAAGAAATCCTGCGTATCAAATCCGGTGCGAAAGCCGGTAAACTGGCCGGCC
GTACCGCTGCCGAAGGCGTATTGGCTTACGCGATCAACGGCAATGTCGGCGCATTGGTCG
AAGTAAACTGCGAAACCGACTTCGTTGCTAAAGACGCGGGCTTCGTAGAATTTGCCAACT
TCGTTGCGAAAACTGCTGCCGAGAAAAAACCGGCTTCTGTTGAAGAACTGAGCGAACTGG
TTGAAGCAGAACGCAAAGCCATCATCGCCAAATTGGGCGAGAATATGTCTGTCCGTCGCT
TCCAAGTGATCGACACTGCCAACCAACTGGTTGCCTACATCCACGGCGCATTGGCGACCG
AAGGCGTATTGGTTGAGTACAAAGGCTCTGAAGACGTAGCACGCAAAATCGGTATGCATA
TTGTTGCCGCTAAACCACAATGCGTAAGCGAAGCCGAAGTAGATGCCGAAACCGTTGAAA
AAGAACGCCACATCTACACCGAGCAAGCCATCGCTTCCGGCAAACCTGCCGACATCGCCG
CTAAAATGGTTGAAGGCCGCATCCGTAAATTCTTGGCTGAAATCACTCTGAACGGCCAAG
CATTCGTGATGAACCCCGATCAAACTGTTGCCCAATTCTCTAAAGAAAACGGCACTGAAG
TGATCAGCTTCGTACGCTACAAAGTAGGCGATGGTATTGAGAAAAAAGCCGTCGATTACG
CAGCCGAAGTTGCTGCCGCTGCTAAAGTGTAAGGCACTTATGAAAAAGAAAGCACCTGGA
TTCCAAACGAATCAGGGTGCTTTTTTTTGAGAAAACCGTTTACGGTACCTATTTTAAGAC
GACCGAATATTCAGACCGTCTTAAAACAAAACAATAATAAACCGACACACCCTATCATTA
ATATTCCGACCGTTGGAAATTCAGACGGCCCAACTCCGACCGACGACATTCAAGAAAGCA
AGGTATCCATGACACAGCAAATCAAATACAAACGCGTATTACTGAAACTCTCCGGCGAAT
CCCTGATGGGTTCCGATCCGTTCGGCATCAATCACGATACCATCGTTCAAACTGTCGGCG
AAATTGCCGAAGTCGTTAAAATGGGCGTGCAAGTCGGTATTGTTGTCGGCGGCGGCAATA
TTTTCCGGGGCGTATCCGCCCAAGCAGGCAGCATGGATCGCGCCACCGCCGACTACATGG
GCATGATGGCGACCGTGATGAACGCGTTGGCACTCAAAGACGCATTTGAAACTTTAGGCA
TCAAAGCGCGCGTACAATCCGCACTGTCTATGCAGCAAATCGCTGAAACCTACGCCCGCC
CCAAAGCCATCCAATATTTGGAAGAAGGCAAAGTCGTGATTTTTGCCGCCGGTACCGGTA
ACCCGTTCTTCACGACCGACACTGCCGCCGCATTGCGCGGTGCGGAAATGAACTGCGACG
TGATGCTCAAAGCCACCAACGTCGACGGTGTGTACACCGCAGACCCGAAAAAAGACCCGT
CCGCCACGCGCTACGAAACCATTACTTTTGACGAAGCCTTGTTGAAAAACCTCAAAGTCA
TGGACGCGACCGCTTTCGCCCTCTGCCGCGAACGCAAGCTCAATATTGTCGTCTTCGGCA
TCGCCAAAGAAGGCTCGCTCAAACGCGTCATTACCGGCGAAGACGAGGGAACGCTGGTTC
ACTGCTGATTGACCATAGTGTCGGCAGATATAGTCGCATATGGGCTTCAGACAGCCATTT
ATTATATGGAGATTATAGTGGATTAAATTTAAACCAGTACGGCGTTGCCTCGCCTTGCCG
TACTGGTTTAAATTTAATCCACTATATTTACAATTTTGATACAATTTGTTTTTCATCAAA
GGAGAAAATCTATGCAAGCACGGCTGCTGATACCTATTCTTTTTTCAGTTTTTATTTTAT
CCGCCTGCGGGACACTGACAGGTATTCCATCGCATGGCGGAGGTAAACGCTTTGCGGTCG
AACAAGAACTTGTGGCCGCTTCTGCCAGAGCTGCCGTTAAAGACATGGATTTACAGGCAT
TACACGGACGAAAAGTTGCATTGTACATTGCCACTATGGGCGACCAAGGTTCAGGCAGTT
TGACAGGGGGGTCGCTACTCCATTGATGCACTGATTCGTGGCGAATACATAAACAGCCCT
GCCGTCCGTACCGATTACACCTATCCACGTTACGAAACCACCGCTGAAACAACATCAGGC
GGTTTGACAGGTTTAACCACTTCTTTATCTACACTTAATGCCCCTGCACTCTCTCGCACC
CAATCAGACGGTAGCGGAAGTAAAAGCAGTCTGGGCTTAAATATTGGCGGGATGGGGGAT
TATCGAAATGAAACCTTGACGACTAACCCGCGCGACACTGCCTTTCTTTCCCACTTGGTA
CAGACCGTATTTTTCCTGCGCGGCATAGACGTTGTTTCTCCTGCCAATGCCGATACAGAT
GTGTTTATTAACATCGACGTATTCGGAACGATACGCAACAGAACCGAAATGCACCTATAC
AATGCCGAAACACTGAAAGCCCAAACAAAACTGGAATATTTCGCAGTAGACAGAACCAAT

## Appendix A

AAAAAATTGCTCATCAAACCAAAAACCAATGCGTTTGAAGCTGCCTATAAAGAAAATTAC
GCATTGTGGATGGGGCCGTATAAAGTAAGCAAAGGAATTAAACCGACGGAAGGATTAATG
GTCGATTTCTCCGATATCCGACCATACGGCAATCATACGGGTAACTCCGCCCCATCCGTA
GAGGCTGATAACAGTCATGAGGGGTATGGATACAGCGATGAAGTAGTGCGACAACATAGA
CAAGGACAACCTTGATTCACACTACCATAACCGCTTGCTACCAAGGAAAACAAAATGAAT
TTGCCTATTCAAAAATTCATGATGCTGTTTGCAGCAGCAATATCGTTGCTGCAAATCCCC
ATTAGTCATGCGAACGGTTTGGATGCCCGTTTGCGCGATGATATGCAGGCAAAACACTAC
GAACCGGGTGGTAAATACCATCTGTTTGGTAATGCTCGCGGCAGTGTTAAAAAGCGGGTT
TACGCCGTCCAGACATTTGATGCAACTGCGGTCAGTCCTGTACTGCCTATTACACACGAA
CGGACAGGGTTTGAAGGTGTTATCGGTTATGAAACCCATTTTTCAGGGCACGGACATGAA
GTACACAGTCCGTTCGATCATCATGATTCAAAAAGCACTTCTGATTTCAGCGGCGGTGTA
GACGGCGGTTTTACTGTTTACCAACTTCATCGAACAGGGTCGGAAATCCATCCGGAGGAT
GGATATGACGGGCCGCAAGGCAGCGATTATCCGCCCCCGGAGGAGCAAGGGATATATAC
AGCTATTATGTCAAAGGAACTTCAACAAAAACAAAGACTAATATTGTCCCTCAAGCCCCA
TTTTCAGACCGTTGGCTAAAAGAAAATGCCGGTGCCGCCTCTGGTTTTTTCAGCCGTGCG
GATGAAGCAGGAAAACTGATATGGGAAAGCGACCCCAATAAAAATTGGTGGGCTAACCGT
ATGGATGATGTTCGCGGCATCGTCCAAGGTGCGGTTAATCCTTTTTTAATGGGTTTTCAA
GGAGTAGGGATTGGGGCAATTACAGACAGTGCAGTAAGCCCGGTCACAGATACAGCCGCG
CAGCAGACTCTACAAGGTATTAATGATTTAGGAAAATTAAGTCCGGAAGCACAACTTGCT
GCCGCGAGCCTATTACAGGACAGTGCTTTTGCGGTAAAAGACGGTATCAACTCTGCCAAA
CAATGGGCTGATGCCCATCCAAATATAACAGCTACTGCCCAAACTGCCCTTTCCGCAGCA
GAGGCCGCAGGTACGGTTTGGAGAGGTAAAAAAGTAGAACTTAACCCGACTAAATGGGAT
TGGGTTAAAAATACCGGTTATAAAAAACCTGCTGCCCGCCATATGCAGACTTTAGATGGG
GAGATGGCAGGTGGGAATAAACCTATTAAATCTTTACCAAACAGTGCCGCTGAAAAAAGA
AAACAAAATTTTGAGAAGTTTAATAGTAACTGGAGTTCAGCAAGTTTTGATTCAGTGCAC
AAAACACTAACTCCCAATGCACCTGGTATTTTAAGTCCTGATAAAGTTAAAACTCGATAC
ACTAGTTTAGATGGAAAAATTACAATTATAAAAGATAACGAAAACAACTATTTTAGAATC
CATGATAATTCACGAAAACAGTATCTTGATTCAAATGGTAATGCTGTGAAAACCGGTAAT
TTACAAGGTAAGCAAGCAAAAGATTATTTACAACAACAAACTCATATCAGGAACTTAGAC
AAATGAATGAACACAACCTGTTAATTTTCTGTTTAAAAGACAATGTTTCAATTAGTGAAT
ATACTGAAATGGTTGATTGGGCTTATGAAAACATTCAATCTGAAACAGTTGTAGAAATTA
CGGAAAATCAAATTATTGAATATCAAAATCGTGGATTATGGGGGCTTGTTTCTGAAATTA
CCGATAATTGGTTATTTGGACCAAGTGAGGGGGATTGGCTAATAGATAAGGAAAGTATTT
TGGCTGTAAAAGAAAAATTACAAAATTCAGATTTTTCTACAGAGCCCTTAGTGAAAAATA
TTATTCATGTACTTGAATATGCTATAAAGAATGAAAAAACAGTAATTTTTCATTTTTGAA
ACTAATCTAATTTTTAGCAGCCGTAGGTCGGATTCTCGAATCCGATATTTTCCAACAGCG
GCATTTCGGAAACGATAGATGCGTCAAATATTTTTGTCGGATACAAATATCCGACCTACA
TCTCTGCGCAGCAAACTTTACAAGATATTAATGAATTAGGAAATTTAAGTCCGGAAGCAC
AACTTGCTGCCGCGAGCCTATTACAGGACAGTGCTTTTGCGGTAAAAGACGGCATCAATT
CCGCCAGACAATGGGCTGATGCCCATCCGAATATAACAGCAACAGCCCAAACTGCCCTTG
CCGTAGCAGAGGCCGCAACTACGGTTTGGGGCGGTAAAAAAGTAGAACTTAACCCGACCA
AATGGGATTGGGTTAAAAATACCGGCTATAAAACACCTGCTGTTCGCACCATGCATACTT
TGGATGGGGAAATGGCCGGTGGGAATAGACCGCCTAAATCTATAACGTCCAACAGCAAAG
CAGATGCTTCCACACAACCGTCTTTACAAGCGCAACTAATTGGAGAACAAATTAGTAGTG
GGCATGCTTATAACAAGCATGTCATAAGACAACAAGAATTTACGGATTTAAATATCAATT
CACCAGCAGATTTTGCTCGGCATATTGAAAATATTGTTAGCCATCCAACAAATATGAAAG
AGTTACCTCGCGGTAGAACTGCGTATTGGGATGATAAAACAGGGACAATAGTTATCCGAG
ATAAAAATTCTGACGATGGAGGTACAGCATTTAGACCAACATCAGGTAAAAAATATTATG
ATGATTTATAGGAAAAAGCCATGAATATACTATCCATAAATAATCAAAACTCAACTATTT
CACTAACTCAAGATGAAGTTTTTGTTTTACGAGCTATCTTGAATGAGATATATGCGGGCG
TATGTGTAGATTCAAGAGAATTTGAAAATGTATCTGGTGTTAGAAAACATGAAGTAGATA
ATTTACAACAACAGTTTGCTGGAATTTATAAAAAAATGACAACTTAACAACCCAAATTTT
TGTCAGAGCCTAGTGCAAATTACAACTATGATTCTATTGTAGCCGAAATGAAAGAAAAAA
ATCATGGGTTGGCGACAGGGTTGATGTTGTTAATATGCCTGATGGAGCACCTACTAGTAT
GGATAACACGCGTATTATGGCAGCACGTGAAGCAGGAGTAAAAGTGGAAGCGAATGTTCA
TAATTTTAATGACCGATTATCATCAAAAGAGAGAATCAGGTTTAAGCATGATGGTATTGA
GCCTCAAACTTGGGGAGAAGCTATCCAGCTACGAATTAGAAAGCAAGAAACACAAAAAGG
AGTTCCAGAAGGGTGGAGCAAAAGATTTCCTAACGGAAGTATTTATGATGTAAAGGTACT
TAGGAAATGATAAAACAAAATAGTTTTGTTCCGTATCCTGAAGCAATGCTTCCTAAAGGA
TTTAAATATCCGCAAAGTTATTTAAAATTAGCTCAATCCACTCATGCCATTAACTACGAT
GAACAATATTCTTTTCCTTGGTGGTTTGAAAATGCAGAAAGCAATATATCAGAAGTAATT
GACATTTATTTTGAAATAACTGGCATTCCAAACCTATTACCTTTTGCTAGAAACCAAGAG
TGGGCTGCCTGTTTTGATATTTCAGATAAATCAGGTAATCCTAAAATTATAGTAGTTAAT
TTAGATAATACAAAATATTACGAGACTTTTGAAAATTTTGATACTTGGCTAAAAGAAGCT
GAAAATGATGGTTGGTAGCAACCGTAGGTCGGATTCTCGAATCCGACATTTTTCAACAGC
GGCATTTCGGAAACGATAGACGCGTCAAATATTTTTGTCGGATACAAATATCCGACCTAC
ATCTCTGCGCAGCAAACTTTACAAGATATTAATGAATTAGGAAATTTAAGTCCGGAAGCA
CAACTTGCTGCCGCGAGCCTATTACAGGACAGTGCTTTTGCGGTAAAAGACGGCATTAAT
TCCGCCAGACAATGGGCTGATGCCCATCCGAATATAACTGCAACAGCCCAAACTGCCCTT
TCCGTAGCAGAAGCCGCAACTACGGTTTGGGGCGGTAAAAAAGTAAACCTTAACCCGACC
AAATGGGATTGGGTTAAAAATACCGGCTATAAAACACCTGCTGCCCGCCCTATGCAGACT
TTAGATGGGGAGATGGCAGGTGGGAATAAGCCACCAAAACCAAGTACGCAGCAACACCCT
ACACACTCTGATAACAATATCGGCTTACCTGCCTCATATGTTAAACCTGATACATCTATT
TCTCCGACAGGAACAATTCAAGACCGCATCAGATGGACAAAGTCCAAGTTTCCTACTGAG
AAATCTTTAAATGGACATTTCAAAGCTCATGGAAAAGAATTTGGCGATATAACCATTGAA

## Appendix A

GACTACCAAAAAATGGCGTCTGATTTGTTATCAAAACAGACATCGGACAAGATATTAGGT
TATCAGACGGAACATAGACGAGTGCGCTATGATATCAATAACAATATCTATGTTTTGGCC
AATCCAAAAACATTCAAAATCAAAACAATGTTTAAACCAAACTTAGGAAAGAAGTATTAT
GATGGAGAATTCAAAAAAGACATGGGAAATTGACGGAGAAATATGGCTACATTGTCCTGT
TTGCGGAACTGAAGTTATGGACTATGATATCTGTGACGTTTGTCAGTGGCAAAATACAGG
AGAAACTAATATAGATGGTGGCCCTAATGAAATGACACTTGCGGAGGCGAAAGAAGCTTA
CGCAAAAGGCTTACCAATCAGATAAATAAGCACCTAGAGAAATCAATGATGACGGAATCC
CATGGTTACCTATCAAATAATTTACCTGTTAAAATCATAAATGATATTATTTATGCAACC
CAGTTAGTCGAAGATTTGGTTTTAGGAAAAATAAAAATTGTTGATTTTTTAAAATCATAT
AACAATTTTTATTGTTGGCTTGGTTTTGATGAGTTGCCTCAATCTGAGAAAATAAAATTC
CTAAGCTATCTTAATATATTAAGTATTCATAAAGAAATACAAGATGAAACTGTGAATAGG
GTTTATACCGATTGAAAAATAGTAGATAGAGATTAACATGTTAAATGAAATTTTTGAAAT
TTATTCGAGACAAGGGGAATCTTTGATAGGAATTGGAATTAGAGAAGCCGCATTACCCGT
CCCTATTGCAATAGATATATTAAATTTATTTATCAATGAGAGAATACTTGTATTGGGGGG
AGATATTTATATCAAGAAAGATAATTATTTTTATCAAACATATGATAATTGGTATTACGA
GGGAAGTAATTTATTTAACAGTATCGACAAAGCAATGCATTATTTATCTCAAATAAAATT
AGAGAATGCATACGTATCTTTTGTGTTGAAATTTATCTAACAAAGGAAGCACAAGAATAG
ATTTATAGTAAAACATCAAGATGTTGAAAATGCTGGGTTTTAATCCAACCTACACTGACC
GGCTCAGATACAGCCGCTCAGCAGACTCTACAAGGTATTAATGATTTAGGAAATTTAAGT
CCGGAAGCACAACTTGCTGCCGCGAGCCTATTACAGGACAGTGCTTTTGCGGTAAAAAAC
GGCATTAATTCCGCCAGACAATGGGCTGATGCCCATCCGAATATAACTGCAACAGCCCAA
ACTGCCCTTTCCGTAGCAGAGGCCGCAGGTACGGTTTGGCGCGGTAAAAAAGTAGAACTT
AACCCGACTAAATGGGATTGGGTTAAAAATACCGGCTATAAAAAACCTGCTGCCCGCCCT
ATGCAGACTGTAGACGGGGAAATGGCTGGGGGAAACAAATCATTAAAAATAGGGACACAA
TCTGTTGAAAAATCAACCGGTCGTACAATACCTAATAATTTAAAGGAACAATTAGCAATG
GAAGAAGTTAAGGCAAACCCACAGGGCAAAACTCCTGCGAGAATACCTCCTATGTCCGAT
ACTAAAAATGGTTGGTTAGCAAAAGACGGTTGGGTTAAGCGTGTTCAAAACGTAAACAAA
ATTGAAATACATTACATTGAAAACTCAAGAACCGGTGAGAAAACAGATTTTAAGTTTAAG
GATTAGTCATGTTTTTAGATGATGTAAATGTTTTTTTAGATGATTTAAATGTTTTTTTAG
ATGATTTAAATACTAATCCAATCACTGACGAATGGTATATGTCCAATTTTGCCGATAAAC
ATATTAAAATTTTGGAAAGTTACGAAGCCTTTGATATTCTAAAACAATTTGTTGATTACA
TGATTGAAGAATATGATGAAAAATCAGAATATGAAATCATGGAAATATTGAGACAATTAA
AATATCAAGCAGATACCAACGAAAAATTTTATACAAATACACAGAAACAGAAAATTGTAG
AATTATATAAACAAGAAATTAGTCAGGATATTTTAAATGAAATCTTTAGATAAACTATCA
ATATAGAAGGAAATCCTTGGAAAAAATAAAATGATAATCGAACACAATGGAAATATACAT
AAAATAGCCAGAATGACTGGAAATAAAAATAATTTTTTAGAAATAATCCTATCAGATATT
CATGAAAACATAAAAATCAAACCATTAACTATAAAAGTAAAAGGAGAGAATGTTATAAAT
ATCCTTCCTGAGGAAGTTAGTTTTTATGTAAAACAAGGTGTTGATTTAATTTATGAAAAA
TATAAACGGAAATTCTTTATCTCCGAAATTTCTTTTTGCCAATCAGATAGCCGGCCTTCA
AGTATCTACGCTTTTCTTACATTTCACTTGCTTGAAGATATTATTAAAAATGAATCCCCA
TCCAACTACACCTGACTGGCTAATAGCAGGTATGAACCGTGTATTCATATCAATATAAGA
TTAATTACGGCAGAATTTGATGAAATTAGACAAAAACTTATCGTAAGATTTTATTTAGAC
AGAGAAGTAACTGATGATGGCAAAGAAGATATGGAAACAGCACGAACAGAATTACTTCCA
GGAGGATATGCTTCATCTCTGGTAGTTTGACAGATTTGACCGCTTCATAAACTTAGAACA
TTAATTAATGATGATAATGTTTATATGATTGGTTCTAAGGATAGCAAAAGCAAATTCAGA
AGGAACATGAATGGCTATTTATGACTTAAACGAAATAGCCGTAGGTCGGATTCTCGAATC
CGACATTTTCCAACAGCGGCATTTCGGAAACGATAGATGCGTCAAATATTTTTGTCGGAT
ACAAATATCCGACCTACATCTCTGCGCAGCAAACTTTACAAGGTATTAATGATTTAGGAA
ATTTAAGTCCGAAAGCACAACTTGCTGCCGCAAGCGCATTATAGGACAGTACTTTTGCGG
TAAAAGACGGTATCAATTCCGCCAGACAATGGGCTGATGCCCATCCGAATATAACTGCAA
CAGCCCAAACTGCCCTTGCCGTAGCAGAGGCCGCAGGTACGGTTTGGAGAGGTAAAAAAG
TAGAACTTAACCCGACCAAATAGGATTGGGTTAAAAATAACGGCTATAAAACACCTGCTG
CCCGCCCTATGCAGACGTTGGACGGTGAGATGGCAGGAGGAAACAAGCCAGTTGTTAAAT
CTATCAGACCAACTACGCGAGATGAATTACGTCAAGCATTGCAAGAACAAGGTTTTAGAC
GTACTGGTTCAGATGCGGCTCAATATGAAACATGGAAAGGTCCTGATGGCGTGAAAATAG
ATATTCGTCCAAATGGAGAGGTTATAAGAACCCAAAGAGTGCCGCGAACCGATGGTGTAC
AGGGAAAATATCCGCAACGACAAGATTATGAAGGCAATCCATTGCCAAATAATCATCATC
ATTCTGGATATTTTGTCAAATGAAAAAAAATATTTTTCACAATGTAAGCCTTTATGAAAT
AATCTTTTCCGATAATGGAAATACCCTTACATTATCTTTTACAGATACAATTGAAGGTAA
TTATTTCGGATATATCAAATGCAGTAATATTTTGAATTTTAAATTAGATACAAATAATTT
CGTAGATTATGAGGATAAGGAAGATAGCTTGTTTCCCTTGTTTATACCCGAAATAGAGCT
ATATAAATACCAATTTTATAGTGAAATTATTATTGATGTAGGGATTATTATAAAAATATC
TGCTGAAACAATTAATTTTGAGCCACTGGGAAAATAGTAACTGCTTTCCCAGCAGCCGTA
GCAACTGTATTTTTACCCGACGGGGTAAAAATACAGTTGCTACATCTCTGCGCAGCAGAC
TCTACAAGGTATTAATAATTCAGGAAAATTAAGCCCGGAAGCACAACTTGCTGCCGCGAG
CATATTACAGGACAGTGCTTTTGCGGTAAAAGACGGCATCAATTCCGCCAGACAATGGGC
TGATGCCCATCCGAATATAACAGCAACAGCCCAAACTGCCCTTGCCGTAGCAGAGGCCGC
AGGTACGGTTTGGAGAGGTAAAAAAGTAGAACTTAACCCGACCAAATGGGATTGGGTTAA
AAATACCGGCTATAAAAAACCTGCTGTTCGCCATATGCAGACTAAGGCGTTAGGTACGGT
AGATGAAATTGGCGATACAGTACAGCAGGTTGGGAAACAGGCTAGCGGACAAAAAACCAG
CGGTGGTAATCCTGCGATTGATAGCGACCCCTATAGCCCGAGTAGTGTGGCAGCTCGCAT
AGAAGCCGGTAAGGCGCGCAGTGATTTACAAATCAAAGACATTTTGAGCAATACTACTCA
AAGGAGTAAAACAAAAGGTCCCGCTGTTCAGTATGATAAAGTGGGGGATTACAATGACGC
ACTAAATGATTTTAATAGTCTGAATGTTCGAAATGTACAAACACGTCCTAATGGAACGAT
AACGGGCAATTTACCTGATGGGCGTGCGGTTAATGCTCGTAATGATAGTAGTGGTGGAGA

## Appendix A

ACCAACACTTGAAATAACAATTAGTAATAACCGAAAAATAAAAATCAGATATGGAAATAC
ACGATAAATTATGAAATTAAAAAGCTTAGATTTCCCAACTGGCTATTTCTATTTTGATAA
TGCAGCAATAAACTCTGATAAAGTAGAAGTTATAGCAGTTGGTTATAGAAATACGGATAA
AACCATAAAAATTTTTATTGAAGATGTTATTCATTTTAGGGTTGTTGATGAATCGTATTT
TATAGATACTTTTATGGATTTAATTTCGGAAGATGCAGATAGAGCTTTGCTTCATGAAAA
TGGTGGTCAATCTTTTTTTGAACTTCTTGATGAGTGTTATGCGGAATGGATATTGAAAGA
AAGTTATTTTCCTTTGAATAGAGAATTCTTTAAATACTATATTTTTATGTTTGAGCAAAC
ATTCATAGAAATAATTGGTTCTAGTGCAACGTATTCAATTATTGAGGGCTAGCGTAAGAT
GAGTAATAAGTTGCCTATCTTTCTTTCAGGCAGCCTGAAAATAAAACTACCCAAGTTGAT
GGTGTACCTGTATCAGTGAAGGGAAATTTTGTTGATGGTAAATTTCGCATTGGTACGGCA
ACAATGAAATCATTTTAAATTGAGCTAGAAATGAACCTAGAAAATTATGAAAACATTTTA
ATAAAATTACTTTTTTATCATAACAACTTAGTAAATGAATATTCTTATTTTATTGAAAAT
AAAAATTTATTTAAAATAGTATCTCGAACGAAAACGAGTAAGGGCTTTTTTTTCACTATA
GAAAAACCATTAAATTTTCTAAGCAAAAAAACTTATTTTGAGTTTAATTTTAAATATTTA
CACTCAGGGAAAGAACGCTTTGGTTCGTTTATGTGCTGGATAAATACTAATTTAATGGAA
TTTGAGGGGGTTTTTTTTAACGACCTGCTCCCTGATAATATGATAATAAATAACTTTTTT
GAAATAAATGATTAACGATACACCAATAAAAATTGGTGGGGTAACCGTATGGATGATATT
CGCGGCATCATCCAAGGTGCGGTTAATCCTTTAATTTACAAGGTAAGCAAGCAAAAGATT
ATTTACAACAACAAACTCATATCAGGAACTTAGACAAATGAATGAACACAACCTGTTAAT
TTTCTGTTTAAAAGACAATGTTTCAATTAGTGAATATACTGAAATGATTGATTGGGCTTA
TAAAAACATTCAATCTGAAACAGTTGTAGAAATTACGGAAAATCAAATTATTGAATATCA
AAATCGTGGATTATGGAGACTTGTTTCTGAAATTACCGATAATTGGTTATTTGGACCAAG
TGAGGGGGATTGGCTAATAGATAAGGAAAGTATTTTGGCTGTAAAAGAAAAATTACAAAA
TTCAGATTTTTCTACAGAGCCCTTAGTGAAAAATATTATTCATGTACTTGAATATGCTAT
AAAAAATGAAAAAACAGTAATTTTTCATTTTTGAGACTAATCCAATTTTTAGTAATATTG
ATGCAGAGCAAGCAGCATTAGATGCCGCAAACATGGGGAGAAGCTATTCAATTTAGAATT
AAAAAACAAATTGAAAATGAACTAGCACCACCAAATTGGTCTACCCAGTTTCCTAATGGT
AGTATTTATGATCCTAAGGTAACGAAATGATTATTCAAAATGAATTTAATTTATATCCTA
GTAATATGCTTCCTGAAAGGTTTTGTTATCCTGAAAAGTATGTTCGTATCTCTAACGATA
CATCTTTAATACCTTATATTCAGCCACATAATTTTCACTGGTGGTTTGAGAATTATGGAA
CAGAAGGGGCAGAAGTAGCTTATATATTTAGAAATTCTATCCTGCCTGATTTAAATCTTA
TCCCATTCGCTAGTAATGGAGAATGGGAAGCTTATTTTGATGGTAATGATGTAACAGGAA
ATTCTAGGGTTATTGTCATTAATTTAGATAATATAGAAAACCATGAATTTTTTAATAGTT
TTGAAGATTGGCTTGAATTAGCAATTAAGGATACTTGGTAAGCAGCTATCTATAAAGAGA
TGAGGCTGCCCTGGACAACTAGGATAAACTCGATTTTACTAATTGTTTTAAAATGGAACA
AGAACTTTTATTTCACTGTTGTTAAAACGCCATTCGCACTCCTTTAAATACAGCTCAAAA
TGCGCTTTGGGAATGCCGTTAAACTTGCGTAAATGACGTTTTGCTTGATTCCAAAAGTTC
TCAGTTCCATTAATATGGTTTTGTCGTTCGGCAAAATGTGTGCTGTGATTGATACGGAAA
TGGCTAAATTCGCCCGCATCCAATACATCATAGCCACGATAACAAAATGAGTTTATTTTG
TTTATACCGTCTTAGACGACTTTCTCTCATAGGGATAATTCTAACTTAATTTGAATTTCC
CTAGTGATCTAGGGCAGCCCCTAAATTAATAAAGCAGCACAACTCCTTTTGCCGATGTTC
CGGACTGTCAAACGACTGTTCCTCATGCCACATCTCCATCAAGGTACGGATAACCCGCTC
CGCCTTACCGTTGGTCTGCGGACAAGCAAATCGGGCAAGCCTCCAACCAATCCCATTATC
ATAACAAGCTGCACCGAAAGCATGTTGGACGGCTCTTTATATTACCTATCATTGTCAGAG
TAAACGTACTCAATCAGGTACAAGCAGGGGTCGGACAGATGTTCGGTCAGAAACTTGGCA
GCACTGTCTGCGGTTTTGTCCGGCAAAATGGCAGAGTATAAAAATCGTCAATAGCGACAA
ACAGGTAATCTCGTTTATCAGCGGCCTTCTGTCCTTTGAGCAACAACAACCGATCGGTAT
CAGGATGCACAAAACCTCCCGGGGACAACCTGCCTTTTACGGCTTTAAGTGCACGGTAAA
TAGTGACGCGGCTGACTTAGTGGCAGCATACTGGGGAGGTGAGTGTTTTTGTGTATATTT
TTATTTTGGTATTCCCTTAGAAATACTGTAAACAACGCTACCGGACGGCCTGCAGGGCTT
CGCGCACGCTTGCTTTGAGTTCTGCGCCGAAGCGTCTGCCCAAGATTCTGCCGAAATCGT
CCTTCGGAGTGTAATCCACCACATCGGGGGCTTTGACCACGTCTCGCGCCACGCTGTAAA
TATTGCCGAGTCCGTCCACCAGCCCGACTTTCAGCGCATCCGCGCCTGTGTACACGCGAC
CGCTGAACACGTCGGGATATTGTCGGAATTTGAGGCGGCCGCCGCGTCCGGTTTTGACGG
CTTTGATGAACTCGCCGTGTATGCCGGTCAGCATTTCTTCCCAGATTTTTGACTGTTCGG
GCGTTTCGGGCGAAAACGGATCGCCCATGCCTTTGTTGCTGCCTGCAATTTTAACCCTGC
GTTTCACGCCGATTTTTTCCATCAGGCCGGTCGCGTCGAAACTGCTGCCGATAACGCCGA
TGCTGCCGACGATGCTGGACGGGTCGGCATAGATTTTGTCCGCCGCCGCCGCGATGTAGT
AGCAGCCGGACGCGCACATATCTTCCGCCACGAGATAAACGGGAATGCCGGGGTGCTGCG
CCTTCAGACGGCGTATTTCTTCAAAAGCGGTGTTGGACACGACGGGCGAACCGCCGGGGC
TGTTGGCGCGGATGACGATGGCTTTTGCCTGCGGGTTTTTGTAGGCGGCCTCCATACCGT
CTTTGAGTTTTTTGACCTGGTCTTCTACACCGTTGCCGATTTCGCCGTACAGATTGACGA
CTGCGGTATGCGGCGTGTTGCCCGCCAACTGCAATGCGGCTTCGTCTTTTCGGAAAATGC
CTGCAATCAGGGCAACCAGAATCAGGGTGCTGACGGCGCGCCAGATGTTTTTCCACATCC
GCTCCCTGCGCCTGTCCTGATAGGCGGACAACAGCACTTCGCGCATGATGTCGCGCTCCC
ATAAGGTTTCCCCCGCATTTTTTGCTTCGGGTGCTTCGTTTTCTCTTCTGATTCGGTATT
GCATGGTTTTCCTTAAATATTGTCCGATTTGGGCAAACGGTTTTCAGTTTACCCGATTTT
TCAGCTCTGCTCCCAATCCGTCCAAGCTGTGCAACACTTCCGCCCACGCCGCGTCCAAAA
GGTTGACGGCTTCTCCTTCGGCTTTGATGCCGAACTCAATGTGCGGTTTGACCTGCGTGC
CGTCTGAATGCGTCCAACCGACGCTGGGCAGGCTGTACGAACGCACGCCGGGATAAGTTT
GCTCGATATGCTCCATAAGCGGCGTAATGCGCGATTCGGGCTGCTCAAACACATACACGC
TGCGGCTGCCGCGTTCGGTTTGGTTGAAGCGGTCGGCGTAATAAGTTTCCAATACCCATT
CCGCCATCGGGTGCGCCATCACAGGAAAGCCGGGGAAGAAATAATGCTCGCGGATAGAAA
ATCCGGCGATGTTGTTAAACGGATTGGGCACCAATTCCGCGCCTTCGGGAAAATCTGCCA
TTTTCAGGCGTTGGGCGTGTTCCGGCGAATCAAGCGGCTCGCCGCGTTTCTGGGTTATGC

## Appendix A

CTTCGATAAACTTGGCGGCTTCAGAATGGCGGACGACGGGCAAATCCAAAGCAGCGGCTG
CGGCTTGGCGGGTGTGGTCGTCGGGCGTGGCGCCGATACCGCCGGTAACGAAAGTTGGCA
TGCCGTCTGAAAAGCTGCGGCGCAGTTGCCTGACCAGCAAATCGGGTTCGTCGGGCAGGT
ATTGCACCTGATTGAGCTTCAGCCCTTTGGATTCGAGCAGGGATTTGAAAAAGGCGAAAT
GCTTGTCTTGGCTGCTGCCGTGTAAGATTTCGTCGCCGATGATGATGAGGTTGAACGCGT
TCATAGATGGTTTCTTTACCGATGCCGTCTGAAAATGTCGATGGTGCTGTGATTTGTTCC
CTCTCCCGTGGGAGAGGGTTAGGGAGAGGGTCGAGCTTGCGTTTTTCAGGCAGCGTTTGC
TTAAGGCCTGCTGTCTGTACCCTCTCCCCAACCCTCCCCCGCAGGGGAGGGAGTCAGGTT
GAGGATGGCGTAAAGACCGTCTGAAAAGATTTTCAGCGAAACGGGCAAAGCTTCTTTTCA
GACAGCCTTAACGGCTGACAATGGGTTATATTTATAAGATAATGAACTCCTTTTTTCAAG
TCCGAAGGATACCCTTATGAGCCAAAACCATACCATTCTGCAATCCCTCCCCGTCGGTCA
GAAAGTCGGCATCGCCTTCTCCGGCGGTCTTGATACCTCTGCCGCGCTGTTGTGGATGAA
ACTCAAAGGCGCGCTGCCTTATGCCTACACTGCCAACCTCGGCCAGCCCGACGAAGACGA
CTACAACGCCATTCCCAAAAAAGCGATGGAATACGGTGCGGAAAACGCCCGCTTAATCGA
CTGCCGCGCGCAGTTGGCACACGAAGGCATCGCCGCCATCCAATGCGGCGCGTTTCACGT
TTCCACCGGCGGCATCGCCTATTTCAACACCACGCCTCTGGGCCGCGCCGTAACCGGCAC
TATGCTTGTTTCCGCAATGAAAGAAGACGATGTGAATATTTGGGGCGACGGCAGCACCTA
CAAAGGCAACGACATCGAGCGTTTCTACCGCTACGGTTTGCTCACCAATCCCGCGCTGAA
AATCTACAAACCCTGGCTCGATCAGCAATTTATCGACGAACTCGGCGGCCGTCACGAAAT
GAGCGAATTTCTGATTGCCAACGGCTTCAACTACAAAATGTCGGTGGAAAAAGCCTACTC
CACCGATTCCAATATGTTGGGTGCCACCCACGAAGCCAAAGACTTGGAATTTTTGAACTC
GGGCATCAAAATCGTCAAACCCATTATGGGCGTTGCCTTTTGGGACGAAAACGTCGAAGT
CAGCCCGGAAGAAGTCAGCGTACGCTTTGAAGAAGGCGTGCCGGTTGCACTAAACGGCAA
AGAATACGCCGATCCCGTCGAACTCTTCCTCGAAGCCAACCGCATCGGCGGCCGCCACGG
CTTGGGTATGAGCGACCAAATCGAAAACCGCATCATCGAAGCCAAATCGCGCGGCATCTA
CGAAGCCCCGGGTATGGCGTTGTTCCACATCGCCTACGAGCGTTTGGTCACCGGCATCCA
CAACGAAGACACCATCGAACAATACCGCATCAACGGCCTGCGCCTCGGCCGCCTGCTCTA
CCAAGGCCGCTGGTTCGACAGCCAAGCCCTGATGTTGCGCGAAACCGCACAACGCTGGGT
TGCCAAAGCCGTTACCGGCGAAGTTACCCTCGAACTGCGGCGCGGCAACGACTACTCAAT
TCTGAACACCGAATCGCCCAACCTGACCTACCAACCTGAACGCCTGAGTATGGAAAAAGT
CGAAGACGCTGCGTTCACTCCGCTCGACCGCATCGGACAGCTCACGATGCGCAACCTCGA
CATCACCGACACCCGCGTCAAACTGGGTATCTACTCGCAAAGCGGTTTGCTCTCGCTGGG
CGAAGGTTCGGTATTGCCGCAGTTGGGCAATAAGCAATAAGGTTTGCTGTTTTACATCAT
TAGCAACTTAAGGGGTCGTCTGAAAAGATGATCCCTTATGTTAAAAGGAATCCTATGAAA
GAATACAAAGTCATCATTTATCAGGAAAGCCTGTTGTCCAGCCTGTTTTTCGGCGCGGCA
AAGGTCAACCCCATCAAATTCAGCGAGTTCCTCAATAAACAAACCCCCGAAGGCTGGCGG
GTTGTAACGATGGAAAAAGATTTGCGCCGTATGCTGCTGTTTTTCAAACGCGAGGCCTAC
GTCGTCATTTTGGAGCGGGATCGTGTTTAAGCTCGGCGTTTATACCTGTCTCGGACTGTT
TGCCGGCTGGGTGCTGCTGCTGATCGTGCAACTCTGGTTTTCTTTTCTCGAAGCGGAATT
GTTCTTCAAAATCACACTGACTATGGCGGGGCTGTTTGTCATCATCCTCGCCGCCTTACT
GGTATGCGGTCAGTATTTTTCCGAAAAGAAAATGAAAGACGACGGGTTTATCAACTGATG
CGGACTTGAACCGGACCCGCGACCCAAACATCACAATGCCGTCTGAACGCCCTCGCTTCA
GACGGCATCAACATCAATCCTGCTCTTTTTTGCCGGCAAACACGCCGAATCCGCCCTTTT
CCGCATCTGTCGGGCGATAGCTGTATTTTCCCGCCACTTCCTCGCCGGCCGGGCCGTAAA
ACTTTCCGGAAACATCCCCGCTGCCATTTTCCGTCCAAGTCCCCTTAAAGCCGTTTCCAT
CGATGGCGGCTTTGAATTTTTGCGTACCCATATGCAAATCATCGCCGCTGTCGATAATGC
CGTCCACAGATTTGCTGCCGAAATCGACTTTTGCGGCAAACCTGCCCCTGGTCGGGTACG
GACGGCCGTTTTCCGTATGGAAATGCAGTACTTCGCCGTTGTACACGGCCGCGCCCGCAA
GCATTTCGCCTTTTGCCGGTTCGCCTTGAACACGAAGGGCATACGATCCGCCGGGCAATT
TTTCCGCCCCGTAAGTCAGATACCGGTAATTCCCTTCGGGCGCGAAGATATTGCCGGAAT
GCCCCGTCAGGCTGACCGCTTCCCCATCGACAATCAGCGTATCCGCCTGATTGACGGGAA
TCAGCGGCATCTCGGCCGGAAGCGACCGCCTCGACCGTGCAGAACGCCTAAATCGCGCAA
ATGAAGTGGGTTTAGGTTTATAAAAGATAATATATTGATTGATTCCCTTCATCTGCACAC
TATCGGCAACCAAACCGACAAATTTATCATTCTTCCCATCTTTCTTGTAATTACTTATTT
TGTCTGCATCACTTAATTTTTCAAATTCTGATTTTAGCTGTACTTCTTCATCCAAGAAAT
TATTGCCACTACAAGAATCGCCTTTACAGTGGGTCAACGTTATATTTTGCGACGGCCCGT
CAATCAAAACGCCATTAGCCAAATCAACCCTTCCAAAATTGCTACCGCCATTCGCAGGTG
CAGGGTTTGACGCGGGGATGGGATCTGAAGAACCGGCGGCTTGATTGTTTCCGGCTTGAT
TTGCACCTTGGGCAGCCGTATTGCCGGCATTTTGCCCGCCTGCCGACGGATCGTCCCCCT
GCATTCCGTCCGCCGCATTTGCCATATCCGGTTGGTTTGCCGGCTGAGACGATTCCCCGG
CATCCGTTGCTTGATTTTCCATATTTCCGGCAAGCATATTCGGATCCGGGGTGTGATTCG
GTGTCGAACTATCTGTACCGGCGGCATTTTGCGGCATATCATTTTGTGCCACCTCGTCTT
CATTTTTGGGATTATCCGCTGTTACCGCACCGCCATTGCCTGTATTTTCTTCCGAAACCG
CCGCCATATCTTGACTGCCTTGTGCGGATGGCGCGCCCTGTCCTTGAGAACCTGCCTGTG
GCGCATCTTCCTTTGCCTCTGTCTCTTTTTCAGAAACAACAGGGGCGGCAGGTTTTGACA
GCGTGTCCGCCGACTTGACATCGGGCGATCCGCCACCGCCGCCCCCGCAGGCTGAAAGGG
CAAAAATACAAGCCATTGCGATTACGCTGCGTTTAAACATCATCATCTCCTTCATCGTAT
TTCCTTTTTGGTTTAAACCCCGCCACTTGGACATCCGTCCTTCGGGGCGGTGGAATCAGC
TTTATTTGGGAAGAGCGCAACCTTTCCAAATCAGGGCGACACATAGGGCTGTGCTTTATG
TGCCGCCCTGTGTGTTGAAACATATTCAATAAATATTGTTTCCGCCGTATGCCTATAAAA
TTGTAAAAATATGCCGTCTGAACGCCAAACGGGCTTCAGACGGCATAGCTTGGTTTATTC
CGCCCGGTTCCTCTGTCGGCCCAAATCGGCGGCAGCGGTAAACAAAACGTCGGTCGAAGA
GTTCAGCGCAGTTTCCGCCGAATCCTGAATCACGCCGATAATGAAGCCGACGGCAACCAC
CTGCATGGCCACATCGTTATCGATACCGAACAGGCTGCACGCCAAAGGAATCAGCAGCAA
CGAGCCACCGGCCACACCGGATGCACCGCACGCGCTAACGGTAGCCACCAGGCTCAGCAG

## Appendix A

CAGGGCAGTGGCGAAGTCAACCGTAATGCCTTGCGTGTGCGCCGCAGCCATCGCCAAAAC
GGTAATGGTGATTGCCGCACCGGCCATATTGATGGTTGCACCCAATGGAATGGAGATGGA
GTAAGTGTCTTCGTGCAAACCCAGCTTTTTCGCCAATGCCATGTTCACAGGGATATTGGC
GGCGGAAGAACGGGTAAAGAAGGCATAAACGCCACTTTCACGCAGGCAGGTAAACACCAG
CGGGAAAGGGTTGCGGCGGATTTTCCACCACACGATGGCGGGATTGACCGCCAGCGCGAT
AAACGCCATACAGCCCAACAGCACTGCAAGCAGCTTCGCGTACCCCGCCAGCGCGCCGAA
ACCCGTCTCCGCGATTGTGGACGACACCAGCCCGAAAATGCCCAAAGGGGCAAAACGGAT
AATCCATTTCACGACGGTGGAAACCGCTTCCGCCAAATCGGCAACGACCTGCCGCGTAAC
GTCCGAACCGTGATTCCGCAACGCCGCGCCCAAAACCAAAGCCCAAGCCAAAATGCCGAT
ATAGTTGGCATTGGCAATCGCGTTAATCGGGTTGGCGACCAGGTTCATCAGCAGCGATTT
CAATACTTCCACAATGCCGGAAGGCGGCGCGGCGGACACATCGCCCGCGCCCGCCAAAAC
AATGTGCGTCGGGAAAACCATACCGGCGATGACGGCGGTCAGGGCTGCGGAAAACGTACC
GATGAGGTAAAGGACGATAATCGGCCTGATATGCGCCTTGTTGCCTTTTTGGTGCTGCGC
GATTGTGGCCGCCACCAAAATAAATACCAAAACCGGCGCGACCGCTTTGAGCGCACCGAC
AAACAGGCTGCCGAACAAGCCTGCCGCCAAGCCCAGTTGCGGGAAACCGAACCGATTAC
GATGCCCAACGCCAAACCGGCGGCAATCTGCCTGACCAGGCTGACGCGGCCGATCGCATG
AAATAAGGATTTGCCGAACGCCATAATTCTTCCTTATGTTGTGATATGTTAAAAAATGTT
GTATTTTAAAAGAAAACTCATTCTCTGTGTTTTTTTTTATTTTCCGGCTGTATTTTATAG
TGGATTAACAAAAATCAGGACAAGGCGACGAAGCCGCAGACAGTACAAACAGTACGGAAC
CGATTCACTTGGTGCTTCAGCACCTTAGAGAATCGTTCTCTTTGAGCTAAGGCGAGGCAA
CGCCGTACTGGTTTTTGTTAATCCACTATAAGGTTGCGTTGATTTGCCCTATGCAGTAGT
GCCGGACAGGCTTTGCTTTATCATTCGGCGCGACGGTTTAATTTATTGAACGAAAATAAA
TTTATTTAATCCTGCCTATTTTCCGACACTATTCCGAAACGCAGCCTGTTTTCCATATGC
GGATTAGAAACAAAATACCTTAAAACAAGCAGATACATTTCCGGCGGGCCGCAACCTCCG
AAATACCGGCGGCAGTATGCCGTCTGAAGCGTCCCGCCCCGTCCGAACAGTGTTAAAATC
GAAAGCCGCCACACCGATGCACGACACCCGTACCATGATGATCAAACCGACCGCCCTGCT
CCTGCCGGCTTTATTTTTCTTTCCGCACGCATACGCGCCTGCCGCCGACCTTTCCGAAAA
CAAGGCGGCGGGTTTCGCATTGTTCAAAAACAAAAGCCCCGACACCGAATCAGTCAAATT
AAAACCCAAATTCCCCGTCCTCATCGACACGCAGGACAGTGAAATCAAAGATATGGTCGA
AGAACACCTGCCGCTCATCACGCAGCAGCAGGAAGAAGTATTGGACAAGGAACAGACGGG
CTTCCTCGCCGAAGAAGCGCCGGACAACGTTAAAACGATGCTCCGCAGCAAAGGCTATTT
CAGCAGCAAAGTCAGCCTGACGGAAAAAGACGGAGCTTATACGGTACACATCACACCGGG
CCCGCGCACCAAAATCGCCAACGTCGGCGTCGCCATCCTCGGCGACATCCTTTCAGACGG
CAACCTCGCCGAATACTACCGCAACGCGCTGGAAAACTGGCAGCAGCCGGTAGGCAGCGA
TTTCGATCAGGACAGTTGGGAAAACAGCAAAACTTCCGTCCTCGGCGCGGTAACGCGCAA
AGCCTACCCGCTTGCCAAGCTCGGCAATACGCAGGCGGCCGTCAACCCCGATACCGCCAC
CGCCGATTTGAACGTCGTCGTGGACAGCGGCCGCCCCATCGCCTTCGGCGACTTTGAAAT
CACCGGCACACAGCGTTACCCCGAACAAATCGTCTCCGGCCTTGCGCGTTTCCAGCCCGG
TATGCCGTACGACCTCGACCTGCTGCTCGACTTCCAACAGGCGCTCGAACAAAACGGGCA
TTATTCCGGCGCGTCCGTACAAGCCGACTTCGACCGCCTCCAAGGCGACCGCGTCCCCGT
CAAAGTCAGCGTAACCGAGGTCAAACGCCACAAACTCGAAACCGGCATCCGCCTCGATTC
GGAATACGGTTTGGGCGGCAAAATCGCCTACGACTATTACAACCTCTTCAACAAAGGCTA
TATCGGTTCGGTCGTCTGGGATATGGACAAATACGAAACCACGCTTGCCGCCGGCATCAG
CCAGCCGCGCAACTATCGGGGCAACTACTGGACAAGCAACGTTTCCTACAACCGTTCGAC
CACCCAAAACCTCGAAAAACGCGCCTTCTCCGGCGGCGTCTGGTATGTGCGCGACCGCGC
GGGCATCGATGCCAGGCTGGGGGCGGAATTTCTCGCAGAAGGCCGGAAAATCCCCGGCTC
GGCTGTCGATTTGGGCAACAGCCACGCCACGATGCTGACCGCCTCTTGGAAACGCCAGCT
GCTCAACAACGTGCTGCATCCCGAAAACGGCCATTACCTCGACGGCAAAATCGGTACGAC
TTTGGGCACATTCCTGTCCTCCACCGCGCTGATCCGCACCTCTGCCCGTGCAGGTTATTT
CTTCACGCCCGAAAACAAAAAACTCGGCACGTTCATCATACGCGGACAAGCGGGTTACAC
CGTTGCCCGCGACAATGCCGACGTTCCTTCAGGGCTGATGTTCCGCAGCGGCGGCGCGTC
TTCCGTGCGCGGTTACGAACTCGACAGCATCGGACTTGCCGGCCCGAACGGATCGGTCCT
GCCCGAACGCGCCCTCCTGGTGGGCAGCCTGGAATACCAACTGCCGTTTACGCGCACCCT
TTCCGGCGCGGTGTTCCACGATATGGGCGATGCCGCCGCCAATTTCAAACGTATGAAGCT
GAAACACGGTTCGGGACTGGGCGTGCGCTGGTTCAGCCCGCTTGCGCCGTTTTCCTTCGA
CATCGCCTACGGGCACAGCGATAAGAAAATCCGCTGGCACATCAGCTTGGGAACGCGCTT
CTAAACCGATATGGCCACTTCAGACGGCATTGCAGCAAACCATTTTGAAACAGACATTAT
GACCGATACCGCACCGACAGATACCGATCCGACCGAAAACGGCACGCGCAAAATGCCGTC
TGAACACCGCCCTACCCCGCCGGCAAAAAAACGCCGCCCGTTGCTGAAGCTGTCGGCGGC
ACTGCTGTCTGTCCTGATTTTGGCAGTATGTTTCCTCGGCTGGCTCGCCGGTACGGAAGC
AGGTTTGCGCTTCGGGCTGTACCAAATCCCGTCTTGGTTCGGCGTAAACATTTCCTCCCA
AAACCTCAAAGGCACGCTGCTCGACGGCTTCGACGGCGACAACTGGTCGATAGAAACCGA
GGGGGCAGACCTTAAAATCAGCCGCTTCCGCTTCGCGTGGAAACCGTCCGAACTGATGCG
CCGCAGCCTGCACATTACCGAAATTTCCGCCGGCGACATCGCCATCGTTACCAAACCGAC
TCCGCCTAAAGAAGAACGCCCGCCGCTCAGCCTTCCCGACAGCATAGACCTGCCTGCCGC
CGTCTATCTCGACCGCTTCGAGACGGGCAAAATCAGCATGGGCAAAGCCTTTGACAAACA
AACCGTCTATCTCGAACGGCTGGATGCTTCATACCGTTACGACCGCAAAGGACACCGCCT
TGACCTGAAGGCCGCCGACACGCCGTGGAGCAGTTCGTCGGGGGCGGCCTCGGTCGGCTT
GAAAAAACCGTTTGCCCTCGATACCGCCATTTACACCAAAGGCGGACTCGAAGGCAAAAC
CATACACAGTACGGCTCGGCTGAGCGGCAGCCTGAAGGATGTGCGCGCCGAACTGGCGAT
CGACGGCGGCAATATCCGCCTCTCGGGAAAATCCGTCATCCACCCGTTTGCCGAATCATT
GGATAAAACATTGGAAGAAGTACTGGTCAAAGGGTTCAACATCAATCCGGCCGCCTTCGT
GCCTTCCCTGCCCGATGCCGGACTGAATTTCGACCTGACCGCCATCCCGTCGTTTTCAGA
CGGCATCGCGCTGGAAGGTTCGCTCGATTTGGAAAACACCAAAGCCGGCTTTGCCGACCG
CAACGGCATCCCCGTCCGTCAGGTTTTAGGCGGCTTTGTCATCCGGCAGGACGGCACGGT

## Appendix A

GCATATCGGCAATACGTCCGCCGCCCTGCTCGGACGGGGCGGCATCAGGCTGTCGGGCAA
AATCGACACCGAAAAAGACATCCTCGATTTAAATATAGGCATCAACTCCGTCGGCGCGGA
AGACGTACTGCAAACCGCGTTCAAAGGCAGGTTGGACGGCAGCATCGGCATCGGTGGCAC
GACCGCCTCGCCCAAAATCTCTTGGCAACTCGGCATCGGCACGGCGCGCACGGACGGCAG
CCTCGCCATTGCAAGCGACCCAGCAAACGGACAGCGGAAACTGGTGCTCGACACCGTCAA
CATCGCCGCCGGGCAAGGCAGCCTGACCGCGCAAGGCTATCTCGAGCTGTTTAAAGACCG
CCTGCTCAAGCTGGACATCCGTTCCCGCGCATTCGACCCTTCGCGCATCGATCCGCAACT
TCCGGCAGGCAATATCAACGGCTCAATAAACCTTGCCGGCGAACTGGCAAAAGAGAAATT
CACAGGCAAAATGCGGTTTTTACCCGGCACGTTCAACGGCGTACCGATTGCCGGCAGTGC
CGACATTGTTTACGAGTCCCGCCACCTTCCGCGTGCCGCCGTCGATTTGCGGCTGGGGCG
GAACATTATTAAAACAGACGGCGGCTTCGGCAAAAAAGGCGACCGGCTTAACCTCAATAT
CACCGCACCCGATTTATCCCGTTTCGGTTTCGGACTCGCGGGGTCTTTAAATGTACGCGG
ACACCTTTCCGGTGATTTGGACGGCGGCATCCGAACCTTTGAAACCGACCTTTCCGGCGC
GGCGCGCAACCTGCACATCGGCAAGGCGGCAGACATCCGTTCGCTCGATTTCACGCTCAA
AGGTTCGCCCGACACAAGCCGCCCGATACGCGCCGACATCAAAGGCAGCCGCCTTTCGCT
GTCGGGCGGAGCGGCGGTTGTCGATACCGCCGACCTGATGCTGGACGGCACGGGCGTGCA
GCACCGCATCCGCACACACGCCGCCATGACGCTGGATGGCAAACCGTTCAAATTCGATTT
GGACGCTTCAGGCGGCATCAACAGGGAACTTACCCGATGGAAAGGCAGCATCGGCATCCT
CGACATCGGCGGCGCATTCAACCTCAAGCTGCAAAACCGTATGACGCTCGAAGCCGGTGC
GGAACGCGTGGCGGCAAGTGCGGCAAATTGGCAGGCAATGGGCGGCAGCCTCAACCTGCA
ACACTTTTCTTGGGATAAAAAAACCGGCATATCGGCAAAAGGCGGCGCACACGGTCTGCA
TATCGCCGAGTTGCACAATTTCTTCAAACCGCCCTTCGAACACAATCTGGTTTTAAACGG
CGACTGGGATGTCGCCTACGGGCGCAACGCGCGCGGCTACCTCAATATCAGCCGGCAAAG
CGGCGATGCCGTATTGCCCGGCGGGCAGGCTTTGGGTTTGAACGCATTTTCCCTGAAAAC
GCGCTTTCAAAACGACCGCATCGGAATCCTGCTTGACGGCGGCGCGCGTTTCGGGCGGAT
TAACGCCGATTTGGGCATCGCCAACGCCTTCGGCGGCAATATGGCAAATGCACCGCTCGG
CGGCAGGATTACCGCCTCCCTTCCCGACTTGGGCGCATTGAAGCCCTTTCTGCCCGCCGC
CGCGCAAAACATTACCGGCAGCCTGAATGCCGCCGCGCAAATCGGCGGACGGGTAGGCTC
TCCGTCCGTCAATGCCGCCGTCAACGGCAGCAGCAACTACGGGAAAATCAACGGCAACAT
CACCGTCGGGCAAAGCCGCTCTTTCGATACCGCGCCTTTGGGCGGCAGGCTCAACCTGAC
CGTTGCCGATGCCGAAGTATTCCGCAACTTCCTACCGGTCGGACAAACCGTCAAAGGCAG
CCTGAATGCCGCCGTAACCCTCGGCGGCAGCATCGCCGATCCGCACTTGGGCGGCAGCAT
CAACGGCGACAAACTCTATTACCGCAACCAAACCCAAGGCATCATCTTGGACAACGGCTC
GCTGCGTTCGCATATCGCGGGCAGGAAATGGGTAATCGACAGCCTGAAATTCCGGCACGA
AGGGACGGCGGAACTCTCCGGTACGGTCGGTATGGAAAACAGCGGACCCGATGTCGATAT
CGGCGCGGTGTTCGACAAATACCGCATCCTGTCCCGCCCCAACCGCCGCCTGACGGTTTC
CGGCAACACCCGCCTGCGCTATTCGCCGCAAAAAGGCATATCCGTTACCGGGATGATTAA
AACGGATCAGGGGCTGTTCGGTTCGCAAAAATCCTCGATGCCGTCCGTCGGCGACGATGT
CGTCGTATTAGGCGAAGTCAAAAAAGAGGCGGCGGCACCGCTCCCCGTCAATATGAACCT
GACTTTAGACCTCAATGACGGCATCCGCTTCGCCGGCTACGGCGCGGACGTTACCATAGG
CGGCAAACTGACCCTGACCGCCCAATCGGGCGGAAGCGTACGGGGCGTGGGCACGGTCCG
CGTCATCAAAGGGCGTTATAAGGCATACGGGCAGGATTTGGACATTACCAAAGGCACGGT
CTCCTTTGTCGGCCCGCTCAACGATCCCAACCTCAACATCCGCGCCGAACGCCGCCTTTC
CCCCGTCGGTGCGGGCGTGGAAATATTGGGCAGCCTCAACAGCCCGCGCATTACGCTGAC
GGCAAACGAACCGATGAGTGAAAAAGACAAGCTCTCTTGGCTCATCCTCAACCGCGCCGG
CAGCGGCAGCAGCGGCGACAATGCCGCCCTGTCTGCAGCCGCAGGTGCGCTGCTTGCCGG
GCAAATCAACGACCGCATCGGGCTGGTGGATGATTTGGGCTTTACCAGCAAGCGCAGCCG
CAACGCGCAAACCGGCGAACTCAACCCCGCCGAACAGGTGCTGACCGTCGGCAAACAACT
GACCGGCAAACTCTACATCGGCTACGAATACAGCATCTCCAGCGCGGAACAGTCCGTCAA
~~ACTGATTTACCGGCTGACCCGCGCCATACAGGCGGTTGCCCGTATCGGCAGCCGTTCGTC~~
GGGCGGCGAGCTGACATACACCATACGTTTCGACCGCTTCTCCGGTTCGGACAAAAAAGA
CTCCGCCGGAAACGGCAAAGGAAAATAAGCGGTTTTCAGACGGCGCGCCGCCAAACCGGA
CATTTGAAAACCTGCTTTTCCACCGTCCGCCGCCGCCGTCCGCCTGCAAGGGAACAGAAT
CGATATAGTGAATTAACAAAAATCAGGATAAGGCGACGAAGCCGCAGACAGTACAAATAG
TACGGAACCGATTCACTCGGTGCTTGAGCACCTTAGAGAATCGTTCTCTTTGAGCCAAGG
CGAGGCAACGCCGTACCGGTTTTTGTTAATCCGCTATATTCCGCCATCTCTAAGATTTAC
AGCGATACACAGGTAATTTAAGGAATGCCCGAACCGTCATTCCCGCCACTTTCCGTCATT
CCCGCGAAAGCGGGAATCTAGGACGCAGGGTTAAGAAAACCTACATCCCGTCATTCCCGC
GAAAGTGGGAATCTAGAAATGAAAAGCAACAGGCATTTATCGGAAATAACTGAAACCGAA
CAGACTAGATTCCCGCCTGCGCGGGAATGACGGCTGCAGATGCCCGACGGTCTTTATAGC
GGATTAACAAAAATCAGGATAAGGCGACGAAGCCGCAGACAGTACAAATAGTACGGAACC
GATTCACTCGGTGCTTGAGCACCTTAGAGAATCGTTCTCTTTGAGCCAAGGCGAGGCAAC
GCCGTACCGGTTTTTGTTAATCCGCTATATTCCGCCATCTCTAAGATTTACAGCGATACA
CAGGTAATTTAAGGAATGCCCGAACCGTCATTCCCGCCACTTTCCGTCATTCCCGCAAAA
GCGGGAATCTAGAATCTCGGACTTTCAGATAATCTTTGAATATTGCTGTTGTTCTAAGGT
CTAGATTCCCGCCTGCGCGGGAATGACGATTCATAAGTTTCCCGAAATTCCAACATAACC
GAAACCTGACAGTAACCGTAGCAACTGAACCGTCATTCCCACGAAAGTGGGAATCTAGAA
ATGAAAAGCAACAGGCATTTATCGGAAATAACTGAAACCGAACAGACTAGATTCCCGCCT
GCGCGGGAATGACGGCTGCAGATGCCCGACGGTCTTTATAGCGGATTAACAAAAATCAGG
ACAAGGCGGCGAAGCCGCAGACAGTACAAATAGTACGGAACCGATTCACTCGGTGCTTCA
GCACCTTAGAGAATCGTTCTCTTTGAGCTAAGGCGAGGCAACGCCGTACTGGTTTTTGTT
AATCCTCTATAATGCGCCCTTCGGCGTGGCGGATATATAAGGAAGTGATTTTCCATCTAA
GTAAAAACCGCCCTATCGGATAAGCCCTTAACAGAAAAGGCTTTACCCGCGCCGTATCGG
AACACATCCTCTAAAATACAATCCGTTGAATTGAAAAAAATATAAAAACATCCGCCCGCG
AAAAACGGCAGCGCGTCGTTTGACAAAGAATGAAAATATCGGTTAAAAACCGATTTTCAT

## Appendix A

ACAAAAAACACCGCTGCCGTCCGCATCCGTTTCAGACGGTATTGAGAGAAAATCTTTTAG
GAGAACCTTTATGTCCCGGCATCCCGCCCCCACCGGAGAAAAAACATTCTTCGGCCACCC
CTTCCAGCTTTCCACCCTCTTCCATATCGAATTGTGGGAACGTTTTTCATTTTACGGAAT
GCAGGGCATCCTGCTGATTTACCTCTACTACACCGCCGACAAAGGCGGCTTGGGCATAGA
CAAAACCCTCGCCGGCGGCATTGTCGGCGCATACAGCGGCAGCGTGTACCTGTCCACCAT
TTTGGGGGCGTGGTTTGCCGACCGAGTATGGGGTGCGGAAAAAACCCTCTTCCTCTCGGG
CATCGTCGTGATGCTCGGACACATCGTCCTTGCCGCCGCCCCGGGCCTGTACGGCCTTTT
AATCGGGCTGATATTCATCGCATTGGGCAGCGGCGGCGTGAAATCTACGGCCAGTTCTAT
GGTGGGCGCATTATACGAACAGGACGAAATGCGCCCGCTGCGCGATGCGGGATTTTCCAT
TTTCTACATCGCCATCAACATCGGCGGCTTCCTAGGCCCGCTGCTGACCGGCCTACTGCA
GGAAAACATCGGTTTCCATTATGGTTTCGGCGCGGCGGCGGTCGGTATGGCATTCGGCTT
GTGGCGTTATTCCCTGGGACGTAAAAACCTGCCCCACCCCACCGTCCCCCATCCGCTTTC
AAAAGGACAGGGCAAAACTGCGGCCGCCGTCGGCATCGCCCTCATCGCCGCACTTGCAAC
CGCCATCAAAACCGGGCTTGTCAACCTCGACAATTTCTCCGGCATCCTATTATCTACCGT
CATCCTTGCCGTCATCGCCTATTTCGCCCGCCTGCTGACCAACCCCCGCGTCAGTTCCGA
CAACAAACGGCACATCATCGCCTACATCCCGCTTTTCCTGACCATCTGTATGTTTTGGGC
CGTCTGGTTTCAGATTTACACCGTGGCAACCGTCTATTTCGACGAAACCGTCAACCGCAC
CATCGGTTCGTTTACCGTGCCCGTCGCTTGGAAAGATTCTATGCAAAGCCTGTGGGTCAT
CCTGTTTTCCGGACTGATGGCGGCAATGTGGACAAAAATGGGGCGCAAACAGCCCAAAAC
CCCGCTGAAATTCGCTATGGCGGTATTTGTTACCGGCGCGTCGTTTTTGGGATTCGTCCC
CTTTATTTCCTCCGGTACGCCGATGCCTATTGCGGTTTTCGCACTGATCGTCCTCGCCAT
CACGATAGGCGAACTGATGATTTCCCCGATTGCGCTGTCCATCTCCACCAAAATCGCACC
GCCTTTATTCAAAACCCAAATGGTCGCCCTTAATTTCCTTGCCTTTTCATTAGGCTTCAC
TTTGGGCGGCGTATTGTTTGAAAAAGGCTATCAGGCGGGCGACGAAATCGGCTTCTATCG
GCTGCTGTTCTACATCGGCGCAGCCACAGGCTTCCTGCTGCTCCTGCTCGTCCCCAAATT
GAACAAAATGCTCGAAGGCACAGACTAAGTCCCGCCCCGATGCCGTCTGAACCCTTCAGA
CGGCATTTTTCCGCATAATGAAACCAAACCGTTTCCACCCGACAGGACAGGCTCCCGCCC
AACCGGAAGGCAGCCTGCCGATTGTCATTTGAATAACGCAAGGGAAAGCCGTTGATTTCC
GTTTGTATGGAAACAGTTTGGTTTCATTGGAAAAAGGCATTTTGTCCGACTAAATTAGTG
CTGCATCAACGAAATATATAGTGGATTAACAAAAATCAGGACAAGGCGACGAAGCCGCAG
ACAGTACAAATAGTACGGAACCGATTCACTTGGTGCTTGAGCACCTTAGAGAATCGTTCT
CTTTGAGCTAAGGCGAGGCAACGCCGTACCGGTTTTTGTTAATCCACTATAAAAACACAA
CCTAAATAAAAATGCCGTCTGAACCATATTTCAGGTTTCAGACGACATTTGCGTGTCGGA
TGCACACCGGACAGGCGGTAAGCCGGGTTCTGTCTCGGACAGTCATTCCTCTAGGCATAC
CGTTACCGGTATGCTCAAGCAACCTACCCGAACGCTCGGCGGGCAGCGTCATTGCGTTCT
GTTTGGTCTTGCTCCGAATGGGGTTTGGCCTGCCGCATATTGTTACCAAATGCGCGGTGC
GCCCTTACCGCACCTTTTCACCCTTACCTGTGCTGCCAAAGCAGCCATCGGCGGTTTTGC
TTTCTGTTCCACTTTCCGTCGCGTTACCGCGCCCGGCCGTTAACCGGCATTCTACCCTGC
GGAGCCCGGACTTTCCTCCCCGTATGCCTTACGCGATACGCGGCGACTGTCTGCCCGTCC
CGTGTGCGGCGCGGATTATAACACGAAACACAAAAATGCCGTCTGAAACGGTACAGGTTT
CAGACGGCATACAGCCTAAACTACACGCCCTGTTTCAGGCTGGCTTCGATGAAGCCGTCC
AAGTCGCCATCCAATACGGCTTTGGTGTTGCCGACTTCGTAGCCTGTACGCAAGTCTTTG
ATACGTGAGGAATCCAAAACATACGAACGGATTTGGCTGCCCCAACCTACATCGGATTTA
CCTTCTTCCAACGCCTGTTTCTCTTCATTGCGTTTGCGCATTTCCAATTCATACAGTTTG
GACTTCAACATTTCCATCGCAGCGGCTTTGTTGGCGTGTTGCGAACGGTCGTTTTGACAT
TGCACCACAATCCCCGTCGGCTCGTGGGTAATGCGCACGGCGGAGTCGGTTTTATTGATG
TGCTGACCGCCCGCACCCGATGCGCGATAGGTGTCGATGCGCAAATCGGCGGGGTTGATT
TCGATTTCGATGGAATCGTCGATTTCAGGGTAAACGAACACGGAGGCAAACGAGGTATGG
CGTTTGTTGTTCGAGTCAAACGGCGAGTAACGCACCAAGCGGTGAACGCCGGTTTCGGTA
~~CGCAGCAAACCATAAGCGTATTCGCCTTCCACACGGATGGTGGCGCGGTTGATGCCTGCG~~
ATTTCGCCGTCGTCTTCTTCAAGGATTTCGATTCTGAAGCCTTTGCGCTCGGCGTAGCGG
CTGTACATACGGAACAGCATACCCGCCCAGTCTTCCGCTTCCGTACCGCCCGCGCCTGCG
GTGATGTCGATAAAGCAGTTGTTCGGGTCGGCGGGCTGGTTGAACATCCGTTTGAACTCC
AAATCCGCCATCTGTTTTTCCAGCCCCGCTACGTCTTCCTGCACGGCGGCAAAACCTTCT
TCGTCGTTTTCTTCGACGGTCATTTCAATCAGCATGCGGTTGTCTTCGATGCCCGAAGCG
ATGTTGTCGAGCGTCAACACGATGCCTTCGAGGATTTTGCGCTCTTTGCCGATTTCTTGG
GCGCGTTTCGGGTCGTTCCAAAGTTCGGGGTCTTCGGAAAGACCGATAACTTCTTCCAAT
CGGTCTTTCTTACCCTGATAATCCATATAAACTCGGATGTCTTCGCTGCGCTTTTCCAAA
TCGTTCAGGGTATTGTTGAGCTGGTTGATTACTTCGGCTTCCATGATTCTTTTGTTCTTT
CAAAATTTTAGGGGCGTATTGTACGGGATTCGGGTATTTTTTTCTATGGATAAAGCCTTC
TGGAAACACGTTCAGACGGCATAGCGTCAATAACGGTATGCCGCCAGTTTGCGTTTGATT
TCAGGCAATGCGGCACGTGCTGCCTCCTCACCCAACCGGATGGCGCGTTTTTTCTGATCG
AATCCGCCGACTGCACCCAAATCCAAAACCTGCGGTTTGATAACCACATCCGCCTGCCCC
AACTCATTTTGCAACGCAGAAACGCTCATTACGTTCAGCGTCTGATCGAGATAAGAGAAG
AAACCTTGGCTGATGTTTTTGCCCGGACGGGCGGAAATATCGACGGCAATCACGAAATTC
GCCCCCTGCCGCCGGGCGGCACTGACGGGCACGGGCTGCGACAGACCGCCGTCAACATAT
GTATGCCTGCCGATGATAACGGGTTGGAACACATTGGGAATGGCGGCGGAAGCGCGCACA
GCCTGCCCGGCATTCCCCTGATTGAAAGCGACGGCCTTGCCGGTTTCAAAATCAGTAGCA
ACGGCGGCAAATTTGATGGGAAACTGCTGAATCTGCCTGCCGCCGACTTTTCGGTTGATG
TAATTTTGCAGCTTTTCGCCTTTGATAAAACCACTGGTGGACAAGGTTAAATCGACCAAA
TCGGTTTTGCCTAAAATTTCGGCTTCCAATTCGAGGCGGTCGGGCGACATACCCGATGCA
AAAAGGCTGCCGACAATCGAACCTGCCGATGTGCCGGTAACCACCTTCACAGGAATACCG
TTTTCTTTCAAAACCTTAATAATACCTACATGGGCAAATCCTTTAGATGCGCCGCCACCG
AGTGCCAAACCGACCACTGCGGCGGGTTTGGCGGTTTGCACCGGCTTGCGGACAGCATTA
TTTCCCGCCGTGCCGCAGGCGGCAAGCAACGCGGCGGCGGCGATTGCCAAAAGCGGTCTG

# Appendix A

ATTTTTGAAAACGTTACCATATTTTCCATTCCTTTATATATCGCACCCCGTCAAAAAGAG
GGATTGCTTTTCTTAACACCCCCCTTTGACAGCCAAGCAAATGGGGGCTTTGTTAAGTCA
TCATCAAAATTAATATTTCTTTTTTTTTCCTTTACGGAAATTATATTTGAAGGCATACT
ATCCAAGGCGGGAATTATCTCACAACACCGCCGTTATCCAAATATCCCGCCTTTTTCCCT
TTCTTTCCATCAAAATACTTTCTTTTTATATTCATTAACTTGTTAAATCATTGGCTGCCG
GGTGTCAGTTTTTCCGACAAAATCCGTCTAATGGGGTATCAACAGAACCAAAACAGGAAC
ACTTATGAAAATCGGAACAACTTGGCAGACGGCATCCGCTATGCTGGTTTTGCGTCTGTT
TGCCGCATATGAATTTTTGGAATCGGGTTTGCAAAAATGGAACGGGGAGAATTGGTTTTC
CGAAATCAACGATCAGTTTCCATTCCCGTTCAACTTGCTGCCGGACGCGTTAAACTGGAA
TCTCGCCATGTATGCGGAGCTTTTGCTGCCCGTATTGTTGCTTTTGGGTTTGGCAACGCG
TCTGTCGGCATTGGGGCTGATGGTCGTTACCGCCGTCGCTTGGGCTGCGGTTCACGCCGG
TTCGGGTTACAATGTCTGCGACAACGGTTATAAAATGGCTTTAATTTATATCGTGGTATT
AATCCCGCTGCTTTTCCAGGGTGCGGGCGGATGGTCGCTGGATACGCTGCTGAAAAAACG
GTTTTGCCCCCGATGCCGTCTGAAACAAGATTGATTCAGTCGTGGAATCTGACTTTAAAC
ATTCCAACCTTATCTCGTTAACTTGATATTTTGAAAAGGAAATGACATGAACAAAAACAT
TGCTGCCGCTCTCGCCGGTGCTTTATCCCTGTCTTTGGCCGCCGGTGCAGTTGCTGCCAA
CAAACCGGCAAGCAACGCAACAGGCGTTCATAAATCCGCCCATGGCTCTTGCGGCGCGTC
CAAATCTGCCGAAGGTTCGTGCGGCGCGGCTGGTTCTAAAGCAGGCGAAGGCAAATGCGG
CGAGGGCAAATGCGGTGCGACCGTAAAAAAAACCCACAAACACACCAAAGCATCTAAAGC
CAAGGCCAAATCTGCCGAAGGCAAATGCGGCGAAGGCAAATGCGGTTCTAAATAATCCCA
CCCCTTCAAACCAAGCCGCGTTTTTCAGTAAAATGCGGCTTTTTTAACGGCAAACAAAGA
TTTTTTAACAAGCACATCATTCTTTTGTGCCATCCGAACCGGGTAAAAATATGATTCAAC
ACGCAGGCTTGGGCTACCGCCGCGACTTGGCGGAAGACTTTCTCTCGCTTTCCGAAAACA
GCCCGATATGCTTTATCGAAGCCGCACCGGAAAACTGGCTGAAAATGGGCGGCTGGGCGC
GCAAACAGTTTGACCGTGTGGCGGAACGGCTGCCGCTGGCGTTGCACGGATTGTCTATGT
CGCTGGGCGGGCAAGCACCGCTGGATACTGATTTGATAGACGGCATCAAAGAAATGATGC
GCCGTTACGATTGCACGTTTTTCTCCGACCATTTGAGCTACTGCCACGACGGCGGTCATC
TTTACGATTTGTTGCCGCTGCCCTTTACCGAGGAAATGGTGCATCATACGGCGCGGCGTA
TCCGCGAAGTGCAAGACCGTTTGGGCTGCCGCATCGCCGTGGAAAACACGTCCTACTATC
TGCATTCCCCGCTTGCCGAGATGAACGAGGTCGAGTTCCTCAACGCCGTCGCACGTGAGG
CCGATTGCGGCATTCATCTGGATGTGAACAATATCTACGTCAACGCCGTCAATCACGGTC
TGCTGTCGCCGGAGGCTTTTTTGGAAAATGTGGATGCAGAGCGCGTGTGCTATATCCATA
TTGCCGGACATGACGTGGAAACGCCGGAATTGTTGATTGATACACATGGCGCGGCAGTTT
TGCCGACTGTTTGGGACTTGCTCGAACTTGCCTATGCCAAGCTGCCGACGATTCCGCCCA
CCCTGTTGGAACGCGATTTTAATTTCCCGCCTTTTTCCGAACTCGAAGCCGAAGTCGCCA
AAATCGCCGATTATCAAACGCGTGCCGGAAAGGAATGCCGCCGTGCAGCCTGAAACCTCC
GCCCAATACCAGCACCGTTTCGCCCAAGCCATACGCGGGGGCGAAGCCGCAGACGGTCTG
CCGCAAGACCGACTGAACGTCTATATCCGCCTGATACGCAACAATATCTACAGCTTTATC
GACCGTTGTTATACCGAAACGCTGCAATACTTTGACCGCGAAGAATGGGGCCGTCTGAAA
GAAGGTTTCGTCCGCGACGCGTGCGCCCAAACGCCCTATTTTCAAGAAATCCCCGGCGAG
TTCCTCCAATATTGCCAAAGCCTGCCGCTTTTAGACGGCATTTTGGCACTGATGGATTTT
GAATATACCCAATTGCTGGCAGAAGTTGCTCAAATTCCGGATATTCCCGACATTCATTAT
TCAAATGACAGCAAATACACACCTTCCCCTGCGGCCTTTATCCGGCAATATCGATATGAT
GTTACCGATGATTTGCATGAAGCGGAAACAGCCTTGTTAATATGGCGAAACGCCGAAGAT
GATGTGATGTACCAAACATTGGACGGCTTCGATATGATGCTGCTAGAAATAATGGGGTTC
TCCGCGCTTTCGTTTGACACCCTCGCCCAAACCCTTGTCGAATTTATGCCTGAGGACGAT
AATTGGAAAAATATTTTGCTTGGGAAATGGTCAGGCTGGACTGAACAAAGGATTATCATC
CCCTCCTTGTCCGCCATATCCGAAAATATGGAAGACAATTCCCCGGGCCAAAACCATCTA
TCCGCATAAAATTACCTTGTTCCCGATACTATGCCGCTACCCGACCTGACCGATGCCGAA
TTAATAGAGTCGCGTAAACTGCTTCTGCATTTTGCGCGGCTTCAGTTGCCCGACCACCCT
GATTTGGCTGAAGATTTAGTGCAGGAAACATTGCTGTCCGCATACAGCGCAGGCGACAGT
TTTCAAGGCAGGGCACTTGTCAACAGCTGGCTTTTTGCCATATTGAAAAACAAAATTATT
GACGCATTACGTCAAATCGGAAGGCAGAGGAAAGTCTTTACCACACTGGATGACGAGCTA
CTGGATGAAGCATTTGAAAGCCATTTTTCCCAAAACGGGCATTGGACGCAGGAAGGGCAG
CCGCAACATTGGAACACTCCGGAAAAATCATTAAACAACAACGAATTCCAAAAAATTCTG
CAAAGCTGCCTATACAACCTGCCTGAAAACACCGCACGGGTATTTACCCTGAAGGAAATA
CTCGGTTTTTCATCCGACGAAATACAACAAATGTGCGGTATCAGCACGTCCAACTACCAC
ACCATTATGCACCGCGCCCGAGAATCATTGCGCCAATGCCTGCAAATCAAATGGTTCAAC
CAAGAAAACCCGAAGTAAACGTTATGAAAAAATGCCGCGATATCGCCCTGCTTCTTTCCA
AACATCAGGACCGGGAAACCACCCCGGGCGAGAAGATTTCCATATACACACACCTGCTGT
TCTGTCCGTATTGCCGTGAATATAAAAGACAACTTCAAACCATCAAAAGATCACTGGCAA
AAACAACCAGAACTTCAAAATAAATGCCGTCTGAAAAGGCTTCAGACGGCATAAGCTGAC
GGAAACAAATCAAACCGATTTACTGTTATCTGCAGTTCATCCATAATACACACTTCAAAA
GCAGCATATTTCCCCATACGGAATGTATAAATACGCAAAATACGAAGGCTGCATCAATTT
GCCATATTTGCTTTATTTGCCTTATTTCACAGACGGCGCTACCCCTCCCGCCCAACCCGT
TCTTTCTGAATGAGCAGATTTCAATGATTAAGGAAACCCTAATGCGCCCAATCTTCCTAT
CTTTCGTTTTATTCCCTATTTTGATAACCGCCTGCAGCACACCGGACAAGTCTGCCCGAT
GGGAAAATATCGGCACAATCTCAAACGGCAATATTCATACATATATCAATAAAGACAGCG
TGAGAAAAAACGGAAATCTGATGATTTTCCAAGATAAAAAAGTTGTTACCAATCTAAAAC
AAGAACGTTTTGCCAACACCCCCGCATACAAGACTGCCATTGCCGAGTGGGAAATCCACT
GCAACAACAAAACATACCGCTTAAGTTCGCTACAGTTGTTTGATACAAAAAACACGGAAA
TTTCCACACAAAACTACACAGCCTCTTCCCTCCGCCCGATGAGCATCCTGTCCGGGACAT
TAACCGAAAAACAATATGAAACCGTATGCGGAAAAAAACTCTGATTGCAACTTATACACA
AACTTACCCACAAACCTTATCATAAAAATGCCGTCTGAAATACTGAAATATCAGCATTTC
AGACGGCATTTTGCCATTCCCTGAAAATTATCCACAAAGTTATCCACATTATTTTTAAA

# Appendix A

ACCGGCTTCCATCCGAAATATAGTGGATTAACAAAAATCAGGACAAGGCGACGAAGCCGC
AGACAGTACAAATAGTACGGCAAGGCGAGGCAACGCCGTACTGGTTTAAATTTAATCCAC
TATATAAACTCGCTATACAATTTCACTATCCAAACGTAAATTGTTCCATTGATACACAAA
ACTGCTTACCCCCATAATTTTGATAAAGCATTTCTTACATTCCCGGCTCCGTCCCGTAAC
CAACACAGCGGCGGATTCGCATTTGAAGTGCAACTTTCCCTAACAGAAAAAGGCCAGTAT
GCGGTAGCATACGACCTTTCCTGCAAGAAAGATTGCCATGAGCTACACGCAACTGACCCA
GGGCGAACGATACCACATCCAATACCTGTCCCGCCACTGCACCGTCACCGAAATCGCCAA
ACAGCTGAACCGCCACAAAAGCACCATCAGCCGCGAAATCAGACGGCACCGCACCCAAGG
GCAGCAATACAGCGCCGAAAAAGCCCAGCGGCAAAGCCAGACTATCAAACAGCGTAAGCG
ACAACCCTATAAGCTCGATTCGCAGCTGATTCAGCACATCGACACCCTTATCCGCCGCAA
ACTCAGTCCCGAACAAGTATGCGCCTACCTGTGCAAACACCACCAGATCACGCTCCACCA
CAGCACCATTTACCGCTACCTTCGCCAAGACAAAAGCAACGGCAGCACGTTGTGGCAACA
TCTCAGAATATGCAGCAAACCCTACCGCAAACGCTACGGCAGCACATGGACCAGAGGCAA
AGTACCCAACCGTGTCGGCATAGAAAACCGACCCGCTATCGTCGACCAGAAATCCCGTAT
CGGCGATTGGGAAGCCGACACCATTGTCGGCAAAGGACAGAAAAGCGCATTATTGACCTT
GGTCGAACGCGTTACCCGCTACACCATCATCTGCAAATTGGATAGCCTCAAAGCCGAAGA
CACTGCCCGGGCAGCTGTTAGGGCATTAAAGGCACATAAAGACAGGGTGCACACCATCAC
CATGGATAACGGCAAAGAGTTCTACCAACACACCAAAATAACCAAAGCATTGAAAGCGGA
GACTTATTTTTGTCGCCCTTACCATTCTTGGGAGAAAGGGCTGAATGAGAACACCAACGG
ACTCATCCGGCAATACTTCCCCAAACAAACCGATTTCCGTAACATCAGTGATCGGGAGAT
ACGCAGGGTTCAAGATGAGTTGAACCACCGACCAAGAAAAACACTTGGCTACGAAACGCC
AAGTGTTTTATTCTTGAATCTGTTCCAACCACTAATACACTAGTGTTGCACTTGAAATCC
GAATCCAAGAGCCTCTAAAAAATAATCGCTTGTTTTGACACCGATACACTCATATAGTGG
ATTAACAAAAATCAGGACAAGGCGACGAAGCCGCAGACAGTACAAATAGTACGGCAAGGC
GAGGCAACGCCGTACTGGTTTAAATTTAATCCACTATACAAATACAGAAACTCAAGAAAA
TAACCTTGTGTATTGACCATCTCAAGCAATTCAGAAAAATCAAGAAATTTTCTGACCGTA
AACAAACGTTTCCCTAAAAAAACGATGTCTTCAAAAATATCGAACAAATAGAGACCTTTG
CAAAAATAGTCTGTTAACGAAATTTGACGCATAAAAATGCGCCAAAAAATTTTCAATTGC
CTAAAACCTTCCTAATATTGAGCAAAAAGTAGGAAAAATCAGAAAAGTTTTGCATTTTGA
AAATGAGATTGAGCATAAAATTTTAGTAACCTATGTTATTGCAAAGGTCTCAAATAATCA
TCTTCGGCGTTTTCATTTTTATGGATTAAAACAACACGGGAAAAATCTGTTTTCAGATGC
TTGCCCGCTTGATTGTTCGGATTATTGTCCGGAACGACAAAACCGTCCTCAAAATTAAAG
CAGACGTTGCGTCCTTCTACCTTTATCTCTGTGCAATAACAATCATGTAGAGAAATGCTA
TCCGAAAAATTTTTTCTTTGTGTATGCAAAAAAAGTTTTCATTCAAGTACCCATATCTAA
CGCAAACGTTTACCTGTTTCCCCGTCAATAATCTGACTCGGCGATTTCTGCCTGCCGATT
CTCCCACCAACAATCCACACATCGCGTCCGAATTGCCTTCTGACTTCCCTCTCCGTCCGA
CACGCGCGTTTGCCTGCGCGGTTGCACGAAGTCGAGACCAAAGGCGTTTGCAAAGCCTGA
CACAAGCGGCGCGCACCTACATGGGCGGGAACCCTGACCGCCAACTTGCTGCGCTGTTTC
CCGCGTAACTCGGGTAAGACACAGGATTTGGCGGATAATAGGAACGTTTTAGGGGCGGGC
CATTCTTTTCTAAGCATATCCTGAAGATTTTCAGACGGCATTTGAAGTAAAGGCTGCAAT
TGTTCAAATTGATTCCCGATGACAATCATACCCTTGTGTTGCGGTCTTTTTTTCAAATGC
GCCAACTTACCGAGTGCTTTGGCTAATGTCGGAAGACACCCCAAGCCATAACAAGATTCG
GTCGGATAAGCGACCAAACCACCTTTTTTCAAATAAACGCTTAACTTACGTTGCGCTGAT
GCTGCGATAATTCTCGGAAATAACATAATATAAAATACCGTCTGAAGCACATTAGTCATA
CTTGGCTTCAGACGGCATCATCCTCTTTCTAATTAACGGTTAATCGCTTTATCGGCAATG
TCTTTACGGTATTGCATCCCGTCGAAACTGATTTTTTCCAACGCGCCATATGCCTTAGCT
TTCGCTTGCGCCACATTATCGCCCAATCCCACAACACACAATACGCGTCCGCCGTTGGTC
AATACGTCACCTTTCTCGTTTGCCGTTGTACCTGCATGGAAAACTTTGCCGATTTGGTTG
GCAGCATCCAGACCGGAAATAATATCGCCTTTTTTGGGCGTTTCGGGGTAATTTTGCGCC
GCCAGTACCACGCCCACGGCAGTTTGCGGGCTCCATTCCGCGGTTACGCTATCGAGTTTG
CCGTCTATTGCCGCTTCAACCAAATCCGATAAGTCGCTGTTCAGTCGGCTCATAATCGGC
TGGGTTTCAGGATCGCCGAAACGGCAGTTAAACTCAATCGTATAGGGTGCACCGCTTTGA
TCAATCATCAAACCTGCGTACAGGAAACCGGTGAACTCATGCCCCTCCGCTTTCATCCCT
GCTACGGTCGGCAAAATAATTTCATTCATCGCGCGTTCGTACACAACAGGCGTTACCACA
GGCGCAGGGCTGTACGCACCCATACCGCCCGTATTCAGACCTTTGTCGCCGTCTAAAAGA
CGCTTGTGGTCTTGGCTGGTTGCCATAGGCAGTACATTATTGCCATCAACCATGACGATA
AAACTCGCTTCTTCGCCTTGCAGGAAATCTTCAATTACAACACGCGCGCCGGCATTGCCC
ATTTTGTTGTCCAGCAGCATATCATCAATCGCAGCATGCGCTTCATCCAAAGTCATCGCC
ACAATCACGCCTTTACCTGCCGCCAAACCATCGGCTTTGATAACGATAGGCGCACCTTTC
TGATTGACGTAATCATGTGCGGCATCGGCGTTTTCAAAGGTTTGATATTGCGCGGTCGGA
ATATTGTATTTCGCCATAAATGCTTTGGCGAAATCTTTGGAACTTTCCAACTGCGCCGCA
TATTGTGTCGGACCGAATATTTTTAGTCCTGCAGCACGGAAATCATCCACAATACCTGCC
GCCAAAGGCGCTTCAGGGCCGACGACGGTAAAAACAATATTTTCTTTACGACAGAATTCA
ATCAAATCCTGATGCGCAGTCAAGTCGATGTTTTGCAACTTGGGTTCAATCGCTGTACCG
GCATTACCAGGCGCAACAAATACTGTTTCCACTTTAGGCGACTGCGCCAATTTCCAAGCC
AGCGCGTGTTCGCGACCGCCATTACCGATAACCAGCAGTTTCATACCATCTCCTTGACAA
ATATGTACTTTTAACGAAAACTCGATACAAAGGGACTTTTATCCCATCTGAAGAAATTTT
AGTAGAATCAAACAAAAGACCGCTTCATTCCACTCTGCAACCTATTCAACTTATCCATAA
ATTAAAAAAGGACAAGCAACCATGCAAAAACGTATTGATGAAATCCAAAGCAAATACCGC
GAATGGTGTCATTTACTACCGCAACTGGAAGAAGACATCCGCCGTTGGAAACATGTCGTC
ACTTTAATTCGCGACATGGACAATTTCTATACCCACGAGTATCAGGCGTGTCATCAGGCT
ATTGAAGACGGGGTAGAACTGGATTTGAGTACGGAAGGCGAATACAGCATTATGAGTGAA
GATGCGCTATGGAACGCGCTGGGCGAATTCCATCAATTGGCTTGGTTATATTTGCGCTCC
AGCGTCGATGCCTTAGACAAATATACACAAGAAGATTAGTCAGCGAAGAGGTCGTCTGAA
ATACCATCACAAAGCATTTCAGACGACCTTTCATTCAAAAGGCTTTTCCGTATTTACTTC

## Appendix A

AATCTGCCGAGTATTCTTCCAAGCCGCAACACAGGCCTCATAATTTACCAACGACAAACT
GACCGTCAATCGGCAATCCAACTGCAAATCCCGCTCCAATATATCCGCCTGATATTGTTT
GGCAATGCGTATCGCTTCATTCAAAAACGGATATTCACATTTCAGCCAAACAGTTTTTTC
AATATTCTTTTCAACTACTTCTGCAACTGCCAACGCTTGAGCCGTCGCCTCTTTGTACGC
ATGTATCAGACCTGGAACACCTAACAAAGTACCACCGAAATAGCGGACGACCACAACCAA
AACGTCGGTAATACCCACCGAATCAATCTGTCCCAAAATTGGTCGTCCAGCACTTCCTGA
TGGCTCTCCATCATCGTTGGCACGAAATTGCACACCATCCACACCCAAACGATAGGCATA
GCACCAGTGTCGTGCTTTATGATGCTCTTCCTTTAACGGATCGAGGTATTTTTTCACATC
AGCCAATGTCCGAATCGGATAGGCAAATGCAATAAAACGGCTGCCTTTATCTTTAAACTC
AGCCTGCGTCAAGGAAGTAATGGTTTTATAAGTCGTAATCATGCTGAAATGTTTTCAGAC
GACCTCATTAATAACAAGGTCGTCTGAAAGTTTCACGTGAAACATCAATTTTTCAATACT
TCTGTTAATTGTGGAACGATTTCAAATAAATCGCCAACCAATCCGTAATCGGCTACATTG
AAAATCGGCGCATCAGCATCTTTATTGATTGCAACAATCACCTTACTGTCTTGCATACCG
GCAACGTGTTGAATTGCACCTGAAATACCGATTGCAAAATAGAGTTGCGGCGCAACCACT
TTACCGGTTTGTCCGACTTGAGCATCGTTTGGCGCATATTCGGCATCAACTGCTGCACGG
GATGCACCGATTGCCGCACCTAAAACATCCGCCAACGGTGTCAGCACTTCATTGAATTTT
TCCGCACTACCCAACGCACGACCACCGGAAACAATCACTTTTGCCTGAGTCAGTTCAGGA
CGATCGGAATGGGAAAGCTGACGGTTAACAAAACGACTCAGGTTTTGGGCAGGGGTTGCT
TCAACATTAATTACCTCAGCATTACCACCTTGCGCCGCCACTGCGTCAAAAACCGTCGCA
CGGAAGGTCAGCACCAATTTTTCTGAATCAGCTTGCACGGTTTCAAATGCATTACCCGCA
TAAATGGGGCGCACAAAAGTCGTGTTATCCACAATTTCGGTCAAATCAGAAATTTGCGGT
ACGTCTAATAAGGCTGCTACGCGGGGCAAAAGGTTTTTACCGAATGTGGTTGCCGTTGCT
GCAACATAGCGGTAATCGGCCGCCAATTTAACAACCAGCGGAGCCAACTCTTCAGCCAAA
CCTTCGGCATAATGAGCAGCATCTGCAACCAAAACTTTTTTCACCCCCGCTACTTGCTTC
GCGAATTCCACTACAGCAGATGCGCCGTTTCCGGCAACCAATAAATCGACTTTGCCCAGT
TTGGCGGCAGCGGTAACAGCATGCAAAGTGGTAGGATTCAACTGTTTGTTGTCGTGTTCG
ACAATAATCAATACACTCATTTCAGCCTCCTCAAATCACTTTGGCTTCGTTTTTCAATTT
TTCAACCAATTCGGCAACGCTTGCTACTTTTACGCCTGCCTGACGCGCCTTAGGTTCGGC
AAATTTCACCGTTTTCAAACGAGGTGAAATGTCGGCAACCAAATCGTCAGGAGTCAGTTT
TTCCAAAGGTTTTTTCTTTGCCGCCATAATATTGGGGAGTTTGACAAAGCGCGGCTCGTT
CAAACGCAAATCCGCGCTGATAACAGCAGGCAGTTTCAATGCGATGGTTTCTTCGCCGCC
ATCGATTTCCCGCACAATCTGCACTTCGTCGCCTTCAATTTGTACTTTGGACGCGAACGT
ACCTTGCGCCGCATTCAGCAAAGCTGCCAGCATTTGCGCCACTTGATTGGCATCATCATC
AATCGCTTGTTTGCCCAAAAAGAAAATTTGCGGATTTTCTTTGTCCGCAACGGCTTTCAG
CAACTTAGCAACGGCCAGAGACTCCAGTTTAGTATCGGTTTCAACATGAATGGCACGGTC
GGCACCCATCGCCAAAGCTGTACGCAAGGTTTCTTCGCATTTTTTCTCACCCAAAGAAAC
CGCTACGATTTCGCTTACTTTTCCGGCTTCTTTCAAACGGACAGCTTCTTCCACAGCGAT
TTCGTCAAACGGATTCATCGACATTTTGACATTGCCGATATCCACATCCGAACCATCGGC
TTTTACACGAACTTTGACGTTGTAGTCCACTACGCGCTTTACTGCGACCAGTGCTTTCAT
TGAACCCTCCTAAAAAGAACGCTGCTTTCACCATCCAGCGAAACCAAACCTTCTTCCCTA
TAAAACCAAATCCGTTTTCCTTAAAAACGAATTCATTCAAAAATCTTTCGGATAATGCTT
GCCGATTATACCATTTTTAAAGCATTTACTCAGACTAGCGGATATACATTCCTGTATCTA
ATAAATTGGAAAATATCATGCCGCCATATCAGTTTTAGACGACCCTTTAGCCTTTATCTG
CTGCAACACAATCCATCAGCGCTTGATAAACCAAATCTGCGGTCGGAATCTGCCCGATAT
TGCCCAAATTTTTTGCAATTGGCGAAACCTGAACGCCTGTTTTAATCGGATCGGTATCGG
TATAAATGCCGACCACAGGTTTTTCCAAGGCATTTGCCAAATGCAGCAAACCGGTATCCA
CGCCGACAATTCCGACCGCGTATTTCAGCAGATACGCTGCCTGCAATAAATTTATTTTGT
CGCACACAATAGCAAACGGCAGCCCATCTGCAATTTGTTTGGCACGCGTTTTTTCATCTT
CATTTCCCCAAGGCAGGTAAATATTGCATTGCTGTTCTTCATTCAACTTTTGCAGCAACG
ACCGCCAGTTTTCCACAGGCCATAACTTACTGTCCCGACTGGTCGCATGCAAAGCCGCAT
AATACGGCTGCGCTAAATTTTTCAGACGGCCTGCTTCAGGAACAGTCAAGCCAAATACCT
GCGTTTCCGGCATTACATACCCAAATACTTGGGCAAACAGTTCACGGTTGCGCCAAACGG
CATTTTTTCCCTTCGGTACAGCGTATGTTTTTACATACGCCAAAGCAGCCCATCCCTCGC
GCGCACTGTTTTTATCCAAACCACAAATCGGGGATTTTGCCATTTTAGCGAAACACGCGC
TTTTAATCAGACCTTGACTGTCCAATACGAAATCAAATACTTCCTGCCGCAAAGTCTGTT
TCAGATGACCCATTTCCCGCCAAGTTTCAGCCCGAAAGAGATGTTTGCGCCATTGCCGCC
ATTTCATCACATGGATTTTTTTTACAAACGGATGCAGGCGCGCAATATCTGCAAATCCAG
CCTCACATAGCCAATGCAGTTCTACATCAGGACATTGTCGCGCCAAATCTTCGATTGCGG
GCAAAGTGTGAATTAAATCGCCCATACTAGACAAGCGGACAAGCAAAATTTTCATATTTA
GGAAGGGGGTTTCACGTGAAACAATTTTAACTTATTGATTATTAATATATTTATTTATTT
CATCAGCGTTTTTTAAGATGATTGCCCCAGCAGAATGCATTTCCTGCCATGCTGTTTCGA
TGGTTTCCGGCGCAATACCCCGACAAGCCGCTTCATTGACGACAACCTGCCAACGACCGC
CTTTGAGTAACTGCAAAACCGTTGTTTTAACACAATAATCCGTAGCTAACCCACCGATAA
TAACCGTATCCGTATTTTGACAACGCAGCCATTCAATCAGCCCTGTGCTTAGTTTTTCCT
CAATATCGTGAAAACACGCGCCGTAAGGATGCAATTCAGGATCAACACCTTTCCAAACGC
AATAATCGTATTCTTTAGCAGAAGGCAGCCCGTCCAATAATTCATAGCCGCGCGTACCGA
CCATCGCATGAGCCACCCAAGTCAAATCCGCATCAGGCAAACCTGTCGGCTTCAACATAT
CAACAGGGTTATCCACAAGCCATTTCGCTACCATATGATGCGCATCTTTCGTCATCACGC
GCAAATCCGCCAAAGCGGCTTGCGCATTCAACTCCTCGACAATCAAATGCCCCTCGTTCA
CGGGCAGTTCGTCAGGACACAGTGGCGTAAACGTTTTTGTGCATCAACATCAATGGAAA
CAATCATCTCATTATTTCAACGCGATTAAAATGCCCTGTATTATAACAAATTACTGCCCA
AAAGCGGTAAAACCGATTGTGATAAGATAAGGTTTTTCCAAAAAACTTATCCACAACCTT
ATGACTTATACCATTACCCCCATCGGCACCGCCCGCTCGCCCTACAAACAGAAATTCGGC
ATCGCCCGCCAGCCCGGTTTGGTCTCCGCCGCAAAAGCCTGCATCGAGCTGAATCCCAAA
TTCACCGCAGACAGCGTGCGCGGGCTGGAAGATTTCGATTATGTGTGGATAAGTTTTATT

## Appendix A

TTTCACGGCGTATTGGATGAAGGCTGGGCGCAAATGGTGCGCCCGCCACGGCTCGGCGGC
AAACAAAAAATGGGCGTGTTCGCCACGCGCAGCCCCCACCGCCCCAACCATCTCGGACTC
TCGCTCCTGAAACTCGAACGCATCGAAACCGGCAAACCCGTCCGCCTCTATTGCAGCGGC
GCAGACCTGCTGGACGGCACACCGATTGTGGACATCAAACCTTATATCCCCTTTGTCGAA
TCCAAACCCGATGCCGCATCCGGTTTCGTCAGCGGCAAACCCGTAGAGTTGGAAGTCGTT
TGGCAGGAAAACATCGGCGCGGAAAATTTATCTGCAAACACCAAAAACCTTATCAGCCAA
AGCATTGCCCAAGATCCGCGCCCCGCCTATCAGAATATTCCCGAACGGATTTATGTGATG
AATATTGCAGATTACGAAGTCAGATTTCAAATCGAGGAAAACCGTGCAACCGTTATTGAT
CTTTCCCCAACCCCGCTTTAAATCGGGCAAAAATCCGGTTTTGCCGCATAGCAGTTGAAC
AAACGGCTGTTGTTTGTTCGCCATAAGCCGCAATATCAAGTTATAGCGGATTAAATTTAA
ATCAGGACAAGGCAACGAAGCCGCAGACAGTACAAATAGTACGGCAAGGCGAGATAACGC
CGTACTGGTTTAAATTTAATCCACTATACAGATAAACAATGCCGTCTGAACGCAATGTGT
TCAGACGGCATTTACTTATCCACAGGTTTGTTCAAGCCTTAGATTTTGCCTGCGAAGTAT
TCCAAAGTGCGGACGAGTTGGCAGGTGTAGGACATTTCGTTGTCGTACCAGGCAACGGTT
TTCACCAATTGTTTGCCGCCCACGGTCATCACGCGGGTTTGGGTCGCATCGAAGAGCGAG
CCGTATTCGATGCCGACAACGTCGGAAGAAACGATTTGATCTTCGTTGTAGCCGTAAGAT
TCGCTGGCGGCGGCTTTCATCGCGGCGTTGATTTCTTCTTTGGTTACAGGGCGTTCGAGG
ATGGAAACCAATTCGGTCAGCGAGCCGCTGGCAACAGGGACGCGTTGGGCGGAGCCGTCG
AGTTTGCCGTTCAATTCGGGGATAACCAGACCGATGGCCTTGGCGGCACCGGTGCTGTTG
GGCACGATGTTGAGCGCGGCGGCTCGGGCGCGGCGCAAATCGCCTTTGCGGTGCGGCGCG
TCAAGGGTGTTTTGGTCGCCGGTGTAGGCGTGGATGGTGGTCATCAGACCTTCGACTACG
CCGAACTCTTTTTGCAGGACTGCCGCCATCGGGGCAAGGCAGTTGGTGGTGCAGGAAGCG
GCGGAGATAACGGTTTCGCTGCCGTCCAAAATGTCTTGGTTTACGCCATATACGACGGTT
TTCACATCATTGCCGCCGGGTGCGGAAATCACGACTTTGCGCGCGCCGGCCCTGATGTGT
GCTTCGGCTTTGGTTTTATTGGTAAAGAAGCCGGTACATTCGAGGATGACATCCACACCC
AACTCGCCCCAAGGCAATTCTTCGGGATTCGGATTGGCAAAAACTTTGATCTCTTTGCCG
TTTACCACGATGGCATCGTCTTTTAATTCGGCAGTACCTTGGAAACGGCCTTGTGTGCTG
TCGTATTTGAAAAGGTGCAGCAGCATTTCGGCAGGGGTCAGGTCGTTGACGGCGACGACT
TCGATGTCGTGGGCTTTTTCAATTTGACGCAATGCGAGGCGGCCGATGCGGCCGAAACCG
TTAATCGCTACTTTAATGCTCATGTATATACTCCAAGCTGTGAAACGAAATTTCAATACC
TGTATTGTATTCTGAAATAAAGTTACATTCCACTATTACATCTAACTACTTGCCGCTTAT
TTGATATAGATGAATTTTACTGTTTGCACAGATTTCCAAAACTTTTACCATCAATATTTG
AATTTAAAATTTTAATGATGATTTTGATGATTGCCAACCTGCTTGTGCGTAAGTAGCAAA
TATCCAATATTTTCATTACCTTTTTGTCAAATAAGTTTGAGTTTAAGACTTGCTGTATAA
GACAGATAAGCGTGGATGTTTTTTGACTTAATAATATTTCTGTGGATAACTTTGCTGTTT
TCCTAGTTGTCTCCACAACCTTATTGACAGGCTTACGGTCAGTCTCATTCCGTCGAAGAC
AAAACCTTTTGCTACAATACCGTTTTCCTAATGATAAGGCAGCCCCATGTCCAAATCCGC
CGTTTCCCCAATGATGCAGCAATACCTCGGCATCAAAGCGCAACATACCGACAAACTGGT
GTTTTACCGTATGGGCGATTTTTACGAGATGTTTTTCGACGATGCGGTAGAAGCGGCAAA
ACTTTTGGATATTACCCTGACCACGCGCGGACAGGTGGATGGCGAGCCGGTCAAAATGGC
AGGCGTGCCGTTTCACGCCGCCGAACAATATCTGGCGCGCCTGGTCAAGTTGGGCAAAAG
CGTGGCGATTTGCGAACAGGTCGGCGAAGTCGGCGCGGGCAAAGGGCCTGTGGAGCGCAA
AGTCGTGCGCATCGTAACGCCCGGCACGCTGACCGATTCCGCATTGCTGGAAGACAAGGA
AACCAACCGCATCGTTGCCGTGTCCCCCGACAAAAAATACATCGGTTTGGCGTGGGCATC
GCTGCAAAGCGGCGAATTCAAAACCAAGCTGACAACTGTGGATAAATTGGACGACGAACT
GGCGCGCCTGCAGGCGGCGGAAATTCTGTTGCCTGACAGTAAAAACGCACCGCAACTTCA
GACGGCATCGGGTGTTACGCGCCTGAACGCGTGGCAGTTTGCCGCCGACGCGGGGGAAAA
ACTGCTGACGGAATATTTCGGCTGCCAGGATTTGCGCGGCTTCGGTTTGGACGGCAAAGA
ACACGCCGTTGCGATTGGCGCGGCAGGTGCACTGTTGAACTATATCCGTCTGACGCAAAA
CCTGATGCCGCAACATTTGGACGGCCTGTCGCTCGAAACCGACAGCCAATATATCGGTAT
GGATGCCGCCACGCGCCGCAATCTCGAAATCACGCAAACCCTCTCCGGCAAAAAATCGCC
GACCCTGATGTCCACGCTCGACCTTTGCGCTACCCATATGGGCAGCCGCCTCTTGGCTCT
CTGGCTGCACCACCCTTTACGCAACCGCGCCCACATCCGAGCGCGCCAAGAAGCCGTTGC
CGCGCTGGAAAGCCAATACAAACCCCTCCAGTGCCGTCTGAAAAGCATTGCCGACATCGA
ACGCATCGCCGCCCGTATTGCCGTGGGTAACGCCCGCCCGCGCGACCTCGCCGCCCTGCG
CGACAGCCTGTTTGCCCTGTCCGAAATCGAATTGTCCGCCGAGTGCAGCAGTCTCTTAGG
AACCCTCAAAGCCGTTTTCCCGGAAAACCTATCCACAGCCGAACAGCTCCGCCAAGCCAT
TTTGCCCGAACCTTCCGTCTGGCTGAAAGACGGCAATGTCATCAACCACGGTTTTCATCC
CGAACTGGACGAATTGCGCCGCATTCAAAACCATGGCGACGAATTTTTGCTGGATTTGGA
AGCCAAGGAACGCGAACGTACCGGTTTGTCCACACTTAAAGTCGAGTTCAACCGCGTTCA
CGGCTTTTACATTGAATTGTCCAAAACCCAAGCCGAACAAGCACCTGCCGACTACCAACG
CCGGCAAACCCTTAAAAACGCCGAACGCTTCATCACGCCGGAACTGAAAGCCTTTGAAGA
CAAAGTGCTGACTGCTCAAGAGCAAGCCCTCGCCTTAGAAAAACAACTCTTTGACGGCGT
ATTGAAAAACCTTCAGACGGCATTGCCGCAGCTTCAAAAAGCCGCCAAAGCCGCCGCCGC
GCTGGACGTGTTGTCCACATTTTCAGCCTTGGCAAAAGAGCGGAACTTCGTCCGCCCCGA
GTTTGCCGACTATCCGGTTATCCACATCGAAAACGGCCGCCATCCCGTTGTCGAACAGCA
GGTACGCCACTTCACCGCCAACCACACCGACCTTGACCACAAACACCGCCTCATGCTGCT
CACCGGCCCCAATATGGGCGGCAAATCCACCTACATGCGCCAAGTCGCGCTGATTGTTTT
ATTGGCACACACCGGCTGTTTTGTGCCTGCCGATGCCGCCACAATCGGGCCCATCGATCA
AATCTTCACCCGCATCGGCGCATCGGACGACCTCGCCTCCAACCGCTCCACTTTCATGGT
CGAAATGAGCGAAACCGCCTACATCCTGCATCACGCCACCGAACAAAGCCTTGTTTTAAT
GGACGAAGTCGGACGTGGTACTTCCACTTTCGACGGCCTCGCCCTCGCGCACGCCGTTGC
CGAACACCTGCTGCAAAAAAACAAATCCTTCAGCCTGTTTGCTACCCACTATTTCGAGCT
GACCTACCTGCCCGAAGCCCACACCGCCGCCGTCAATATGCACCTTTCCGCGCTCGAACA
GGGACAGGACATCGTTTTCCTGCACCAAATCCAACCGGGTCCCGCCGGTAAAAGCTACGG

## Appendix A

CATTGCCGTCGCCAAACTCGCCGGCCTGCCTGTACGCGCATTGAAATCCGCCCAAAAGCA
TTTGAACGGACTGGAAAACCAAGCCGCCGCGAACCGTCCCCAACTGGATATTTTCAGTAC
CATGCCGTCTGAAAAAGGAGATGAACCGAATGTGGGCAACTTTGTGGATAAAGCAGAGGA
AAAACATTTTGAAGGTATATTGGCAGCAGCCTTGGAAAAACTCGATCCCGACAGCCTGAC
CCCGCGCGAAGCATTGTCAGAACTGTACCGTCTGAAAGATTTGTGCAAATCCGTATCTTA
ATTTCCGTTGTCGGAACAGCATCAAACCATATGGAAAAATCTGTGGATAAACATTATCTG
ACAGGAAATTTCCAAACATAAAAAATGCCGTCCGAACAGCTCAGACGGCATCCGTCCATT
CGGCT

Appendix B

# Appendix B

## NMB Open Reading Frames

NMB0001 acetyltransferase, putative 491 3
NMB0002 hypothetical protein 890 498
NMB0003 glutamyl-tRNA synthetase 2305 914
NMB0004 EpiH/GdmH-related protein 3154 2513
NMB0005 arsenate reductase 3504 3154
NMB0006 thioredoxin-related protein 3628 4304
NMB0007 cell division ATP-binding protein FtsE 4304 4951
NMB0008 cell division protein FtsX, putative 4951 5865
NMB0009 BolA/YrbA family protein 5959 6204
NMB0010 phosphoglycerate kinase 7485 6277
NMB0011 UDP-N-acetylglucosamine 1-carboxyvinyltransferase 8819 7569
NMB0012 conserved hypothetical protein 10310 9342
NMB0013 conserved hypothetical protein 10792 10346
NMB0014 3-deoxy-D-manno-octulosonic-acid transferase 12104 10836
NMB0015 6-phosphogluconate dehydrogenase, decarboxylating 13615 12170
NMB0016 hypothetical protein 13911 14144
NMB0017 UDP-3-O-3-hydroxymyristoyl N-acetylglucosamine deacetylase 16137 15217
NMB0018 pilin PilE 17734 17225
NMB0019 pilS cassette 18932 18513
NMB0020 pilS cassette 19646 19263
NMB0021 pilS cassette 20297 19914
NMB0022 pilS cassette 21157 20894
NMB0023 pilS cassette 21882 21466
NMB0024 pilS cassette 22474 22061
NMB0025 large pilS cassette 23489 22821
NMB0026 pilS cassette 23868 23594
NMB0027 FKBP-type peptidyl-prolyl cis-trans isomerase 24226 23900
NMB0028 hypothetical protein 24522 24307
NMB0029 glycerate dehydrogenase 24644 25594
NMB0030 methionyl-tRNA synthetase 27729 25675
NMB0031 glucosamine--fructose-6-phosphate aminotransferase (isomerizing) 29683 27848
NMB0032 hypothetical protein 29959 30483
NMB0033 membrane-bound lytic murein transglycosylase A, putative 32229 30907
~~NMB0034 conserved hypothetical protein 32440 33276~~
NMB0035 conserved hypothetical protein 33276 34439
NMB0036 conserved hypothetical protein 34706 35968
NMB0037 phnA protein 36372 36046
NMB0038 UDP-N-acetylglucosamine pyrophosphorylase 37817 36450
NMB0039 hypothetical protein 38144 37875
NMB0040 hydrolase, putative 38850 38140
NMB0041 ABC transporter, periplasmic solute-binding protein 38909 39907
NMB0042 conserved hypothetical protein 40004 40849
NMB0043 conserved hypothetical protein 40878 41360
NMB0044 peptide methionine sulfoxide reductase 43033 41468
NMB0045 signal recognition particle protein 43179 44441
NMB0046 hypothetical protein 44451 44672
NMB0047 conserved hypothetical protein 45072 45353
NMB0048 conserved hypothetical protein FRAMESHIFT 47969 48109
NMB0049 pilC2 protein FRAMESHIFT 48116 51279
NMB0050 conserved hypothetical protein 55173 53026
NMB0051 twitching motility protein 56685 55462
NMB0052 twitching motility protein PilT 57891 56851
NMB0053 conserved hypothetical protein 58011 58694
NMB0054 hypothetical protein 58697 59101
NMB0055 pyrroline-5-carboxylate reductase 59153 59941

## Appendix B

NMB0056 DnaK suppressor protein 60091 60504
NMB0057 hypothetical protein 66347 66700
NMB0058 hypothetical protein 66731 66885
NMB0059 dnaJ protein 66972 68090
NMB0060 conserved hypothetical protein 68289 70304
NMB0061 dTDP-6-deoxy-L-lyxo-4-hexulose reductase FRAMESHIFT 70923 69924
NMB0062 glucose-1-phosphate thymidylyltransferase 71828 70965
NMB0063 dTDP-D-glucose 4,6-dehydratase 72958 71894
NMB0064 UDP-glucose 4-epimerase 74093 73077
NMB0065 hypothetical protein 74476 75399
NMB0066 rRNA adenine N-6-methyltransferase 75687 76418
NMB0067 polysialic acid capsule biosynthesis protein SiaD, truncation 77283 76609
NMB0068 polysialic acid capsule biosynthesis protein SiaC 78416 77370
NMB0069 polysialic acid capsule biosynthesis protein SiaB 79103 78420
NMB0070 polysialic acid capsule biosynthesis protein synX 80240 79110
NMB0071 capsule polysaccharide export outer membrane protein CtrA 80375 81547
NMB0072 capsule polysaccharide export inner-membrane protein CtrB 81565 82725
NMB0073 capsule polysaccharide export inner-membrane protein CtrC 82728 83522
NMB0074 capsule polysaccharide export ATP-binding protein CtrD 83522 84169
NMB0075 transcriptional accessory protein Tex, putative 84236 86506
NMB0076 methyltransferase HphIm(C), FRAMESHIFT 86540 87539
NMB0077 site-specific DNA methylase, truncation 87529 87876
NMB0078 UDP-glucose 4-epimerase, truncation 87922 88575
NMB0079 dTDP-D-glucose 4,6-dehydratase 88694 89758
NMB0080 glucose-1-phosphate thymidylyltransferase 89824 90687
NMB0081 dTDP-4-keto-6-deoxy-D-glucose-3,6-epimerase 90729 91280
NMB0082 capsule polysaccharide modification protein LipA 91308 93419
NMB0083 capsule polysaccharide modification protein LipB 93559 94815
NMB0084 conserved hypothetical protein FRAMESHIFT 95185 96587
NMB0085 sodium/glutamate symporter 96808 98019
NMB0086 hypothetical protein 98121 99134
NMB0087 hypothetical protein 99148 99342
NMB0088 outer membrane protein P1, putative 101170 99773
NMB0089 pyruvate kinase II 102957 101488
NMB0090 IS1016 family transposase, putative FRAMESHIFT 103217 103857
NMB0091 hypothetical protein 104399 104632
NMB0092 hypothetical protein 104629 104853
NMB0093 hypothetical protein 104856 104939
NMB0094 hypothetical protein 105228 105413
NMB0095 hypothetical protein 105423 105572
NMB0096 hypothetical protein 105676 105843
NMB0097 secretion protein, putative POINT MUTATION 105860 107344
NMB0098 ABC transporter, ATP-binding protein FRAMESHIFT 107313 109396
NMB0099 hypothetical protein 109624 109484
NMB0100 hypothetical protein 109770 109627
NMB0101 IS1016 family transposase, putative FRAMESHIFT 109850 110489
NMB0102 hypothetical protein 110608 111123
NMB0103 bacteriocin resistance protein, putative 111896 111405
NMB0104 hypothetical protein 113073 112402
NMB0105 PhnO-related protein 114197 113358
NMB0106 aspartate carbamoyltransferase, catalytic subunit 114436 115353
NMB0107 aspartate carbamoyltransferase, regulatory subunit 115366 115821
NMB0108 hypothetical protein 115889 116551
NMB0109 conserved hypothetical protein 117948 116620
NMB0110 polypeptide deformylase 118018 118518
NMB0111 methionyl-tRNA formyltransferase 118608 119531
NMB0112 16S RNA methyltransferase 119613 120869
NMB0113 hypothetical protein 120892 121431
NMB0114 nitrogen regulation protein NtrY, putative 121434 123551
NMB0115 nitrogen assimilation regulatory protein NtrX 123547 124821

Appendix B

NMB0116 DNA processing chain A 124915 126105
NMB0117 smg protein, putative 126134 126592
NMB0118 DNA topoisomerase I 126667 128970
NMB0119 hypothetical protein 129741 129049
NMB0120 hypothetical protein 130312 129764
NMB0121 conserved hypothetical protein 130431 130805
NMB0122 conserved hypothetical protein 130897 131463
NMB0123 ferredoxin, 4Fe-4S bacterial type 131589 131837
NMB0124 translation elongation factor Tu 132257 133438
NMB0125 preprotein translocase subunit SecE 133638 133913
NMB0126 transcription antitermination protein NusG 133918 134451
NMB0127 50S ribosomal protein L11 134555 134986
NMB0128 50S ribosomal protein L1 134989 135681
NMB0129 hypothetical protein 135753 135893
NMB0130 50S ribosomal protein L10 135914 136411
NMB0131 50S ribosomal protein L7/L12 136472 136840
NMB0132 DNA-directed RNA polymerase, beta subunit FRAMESHIFT 137027 141208
NMB0133 DNA-directed RNA polymerase, beta' subunit 141368 145540
NMB0134 hypothetical protein 145835 146089
NMB0135 conserved hypothetical protein 146089 146235
NMB0136 30S ribosomal protein S12 146417 146785
NMB0137 30S ribosomal protein S7 146906 147373
NMB0138 elongation factor G (EF-G) 147395 149497
NMB0139 translation elongation factor Tu 149586 150767
NMB0140 30S ribosomal protein S10 150788 151096
NMB0141 transposase, truncation 151241 151603
NMB0142 50S ribosomal protein L3 151777 152418
NMB0143 50S ribosomal protein L4 152421 153038
NMB0144 50S ribosomal protein L23 153038 153349
NMB0145 50S ribosomal protein L2 153358 154188
NMB0146 30S ribosomal protein S19 154198 154473
NMB0147 50S ribosomal protein L22 154485 154811
NMB0148 30S ribosomal protein S3 154824 155513
NMB0149 50S ribosomal protein L16 155500 155913
NMB0150 50S ribosomal protein L29 155916 156104
NMB0151 30S ribosomal protein S17 156107 156367
NMB0152 50S ribosomal protein L14 156592 156957
NMB0153 50S ribosomal protein L24 156972 157292
NMB0154 50S ribosomal protein L5 157305 157841
NMB0155 30S ribosomal protein S14 157847 158149
NMB0156 30S ribosomal protein S8 158168 158557
NMB0157 50S ribosomal protein L6 158574 159104
NMB0158 50S ribosomal protein L18 159121 159471
NMB0159 30s ribosomal protein S5 159493 160008
NMB0160 50S ribosomal protein L30 160004 160186
NMB0161 50S ribosomal protein L15 160191 160622
NMB0162 preprotein translocase SecY subunit 160637 161944
NMB0163 translation initiation factor IF-1 161952 162167
NMB0164 50S ribosomal protein L36 162191 162301
NMB0165 30S ribosomal protein S13 162370 162729
NMB0166 30S ribosomal protein S11 162752 163144
NMB0167 30S ribosomal protein S4 163167 163784
NMB0168 DNA-directed RNA polymerase, alpha subunit 163813 164796
NMB0169 50S ribosomal protein L17 164823 165188
NMB0170 septum site-determining protein MinC 165338 166048
NMB0171 septum site-determining protein MinD 166079 166891
NMB0172 cell division topological specificity factor 166898 167158
NMB0173 transcriptional regulator, LysR family 167165 168082
NMB0174 valyl-tRNA synthetase 171252 168418
NMB0175 conserved hypothetical protein 172158 171352
NMB0176 D-amino acid dehydrogenase, small subunit 173595 172342
NMB0177 sodium/alanine symporter, putative 175065 173677
NMB0178 acyl-(acyl-carrier-protein)--UDP-N-acetylglucosamine O-acyltransferase 176198 175425

## Appendix B

NMB0179 (3R)-hydroxymyristoyl-(acyl carrier protein) dehydratase 176734 176288
NMB0180 UDP-3-O-(3-hydroxymyristoyl)-glucosamine N-acyltransferase 177814 176771
NMB0181 outer membrane protein OmpH, putative 178347 177850
NMB0182 outer membrane protein Omp85 180806 178416
NMB0183 conserved hypothetical protein 182203 180866
NMB0184 1-deoxy-D-xylulose 5-phosphate reductoisomerase 183422 182241
NMB0185 phosphatidate cytidylyltransferase 184275 183481
NMB0186 undecaprenyl pyrophosphate synthetase 185024 184281
NMB0187 ribosome recycling factor 185637 185083
NMB0188 conserved hypothetical protein 186944 185820
NMB0189 hypothetical protein 187355 187774
NMB0190 glucose inhibited division protein B 187935 188555
NMB0191 ParA family protein 188657 189427
NMB0192 ribonuclease HII 191274 190693
NMB0193 glucose inhibited division protein A 193238 191346
NMB0194 amino acid symporter, putative 194991 193567
NMB0195 pyridoxal phosphate biosynthetic protein PdxA 195133 196137
NMB0196 ribonuclease E 200197 197441
NMB0197 hypothetical protein 200321 200605
NMB0198 ribosomal large subunit pseudouridine synthase C 200690 201679
NMB0199 lipid-A-disaccharide synthase 201730 202899
NMB0200 hypothetical protein 203501 203115
NMB0201 hypothetical protein 203724 204131
NMB0202 hypothetical protein 204152 204322
NMB0203 dihydrodipicolinate reductase 205207 204401
NMB0204 lipoprotein, putative 205594 205220
NMB0205 ferric uptake regulation protein 205813 206244
NMB0206 leucyl/phenylalanyl-tRNA--protein transferase 206317 207039
NMB0207 glyceraldehyde 3-phosphate dehydrogenase 208326 207298
NMB0208 ferredoxin, 4Fe-4S bacterial type 209364 208528
NMB0209 glutathione-regulated potassium-efflux system protein 209513 211486
NMB0210 site-specific DNA methylase, truncation 212082 212401
NMB0211 L-serine dehydratase 214093 212711
NMB0212 DNA gyrase subunit B 216580 214193
NMB0213 hypothetical protein 216736 217719
NMB0214 oligopeptidase A 217810 219843
NMB0215 conserved hypothetical protein 221035 220472
NMB0216 catalase 222945 221434
NMB0217 RNA polymerase sigma-54 factor RpoN, putative 223293 224141
~~NMB0218 glycosyltransferase 226194 225067~~
NMB0219 3-oxoacyl-(acyl-carrier-protein) synthase II 227746 226502
NMB0220 acyl carrier protein 228138 227905
NMB0221 dihydroorotate dehydrogenase 228370 229374
NMB0222 hypothetical protein 229540 230010
NMB0223 hypothetical protein 230140 230355
NMB0224 glutamate-ammonia-ligase adenylyltransferase 230556 233243
NMB0225 transposase, IS30 family FRAMESHIFT 234513 233551
NMB0226 conserved hypothetical protein 235470 234781
NMB0227 conserved hypothetical protein 236771 235581
NMB0228 conserved hypothetical protein 237637 236903
NMB0229 conserved hypothetical protein FRAMESHIFT 238552 237662
NMB0230 conserved hypothetical protein 239196 238552
NMB0231 hypothetical protein 239356 239255 N
NMB0232 DNA helicase II 239380 241584
NMB0233 hypothetical protein 241663 241761
NMB0234 hypothetical protein 242111 242647
NMB0235 hypothetical protein 243052 242894
NMB0236 hypothetical protein 243168 243063
NMB0237 hypothetical protein 243535 243179
NMB0238 IS1016 family transposase, degenerate 243588 243849
NMB0239 hypothetical protein 244051 244668

## Appendix B

NMB0240 hypothetical protein 244694 246142
NMB0241 NADH dehydrogenase I, A subunit 246607 246960
NMB0242 NADH dehydrogenase I, B subunit 246954 247433
NMB0243 NADH dehydrogenase I, C subunit 247449 248039
NMB0244 NADH dehydrogenase I, D subunit 248032 249285
NMB0245 NADH dehydrogenase I, E subunit 249288 249758
NMB0246 NADH dehydrogenase I, F subunit 250151 251449
NMB0247 hypothetical protein 251452 251886
NMB0248 conserved hypothetical protein 252175 252411
NMB0249 NADH dehydrogenase I, G subunit 252726 254984
NMB0250 NADH dehydrogenase I, H subunit 254990 256063
NMB0251 NADH dehydrogenase I, I subunit 256147 256623
NMB0252 hypothetical protein 256657 257361
NMB0253 NADH dehydrogenase I, J subunit 257400 258068
NMB0254 NADH dehydrogenase I, K subunit 258068 258370
NMB0255 cell filamentation protein Fic-related protein 258407 258979
NMB0256 hypothetical protein 259106 259444
NMB0257 NADH dehydrogenase I, L subunit 259496 261517
NMB0258 NADH dehydrogenase I, M subunit 261616 263109
NMB0259 NADH dehydrogenase I, N subunit 263122 264561
NMB0260 hypothetical protein 264612 264995
NMB0261 geranyltranstransferase 265863 265087
NMB0262 exodeoxyribonuclease, small subunit 266188 265967
NMB0263 conserved hypothetical protein 267358 266438
NMB0264 ABC transporter, ATP-binding protein 269219 267366
NMB0265 Holliday junction DNA helicase RuvA 269966 269385
NMB0266 conserved hypothetical protein 270374 270051
NMB0267 conserved hypothetical protein 271155 270439
NMB0268 RNA methyltransferase, TrmH family 271749 271288
NMB0269 competence protein 272539 271817
NMB0270 bioH protein, putative 272538 273284
NMB0271 hypothetical protein 273284 274069
NMB0272 hypothetical protein 274527 274820
NMB0273 hypothetical protein 274861 275283
NMB0274 ATP-dependent DNA helicase RecQ 277728 275431
NMB0275 indole-3-glycerol phosphate synthase 278575 277796
NMB0276 conserved hypothetical protein 279582 278629
NMB0277 virulence factor MviN 281255 279717
NMB0278 thiol:disulfide interchange protein DsbA 281470 282165
NMB0279 conserved hypothetical protein 283229 282228
NMB0280 organic solvent tolerance protein, putative 283431 285704
NMB0281 peptidyl-prolyl cis-trans isomerase 285809 286852
NMB0282 ribonuclease II-related protein 290243 288366
NMB0283 conserved hypothetical protein 290552 291181
NMB0284 adenylosuccinate lyase 291256 292623
NMB0285 O-antigen acetylase FRAMESHIFT 292707 294573
NMB0286 conserved hypothetical protein 295481 294870
NMB0287 probable ATP-dependent helicase DinG 297668 295521
NMB0288 hypothetical protein 297740 297967
NMB0289 deoxyribodipyrimidine photolyase, FRAMESHIFT 299363 298066
NMB0290 transcriptional regulator, putative 300264 299356
NMB0291 conserved hypothetical protein 300372 300767
NMB0292 conserved hypothetical protein 300819 301421
NMB0293 TonB-dependent receptor, putative 301610 303718
NMB0294 thiol:disulfide interchange protein DsbA 303836 304528
NMB0295 signal recognition particle protein 306232 304865
NMB0296 CcsA-related protein 306452 307255
NMB0297 hypothetical protein 307272 307367
NMB0298 hypothetical protein 307401 307583
NMB0299 comEA-related protein 313097 313540
NMB0300 hypothetical protein 313603 313904
NMB0301 Hypothetical protein 313958 314161
NMB0302 IS1016C2 transposase, degenerate 314284 314933
NMB0303 transposase, degenerate 315024 315307

## Appendix B

NMB0304 class 5 outer membrane protein, degenerate 315549 315295
NMB0305 hypothetical protein 315891 315736
NMB0306 hypothetical protein 316061 316252
NMB0307 phospho-2-dehydro-3-deoxyheptonate aldolase, phe-sensitive 316403 317455
NMB0308 dihydrofolate reductase 317526 318011
NMB0309 conserved hypothetical protein 318840 318367
NMB0310 conserved hypothetical protein 319280 318855
NMB0311 hypothetical protein 319392 319634
NMB0312 virulence-associated protein VapA FRAMESHIFT 321089 323177
NMB0313 conserved hypothetical protein 323422 324885
NMB0314 hypothetical protein 326057 325092
NMB0315 conserved hypothetical protein 326135 327424
NMB0316 conserved hypothetical protein 328616 327933
NMB0317 conserved hypothetical protein 329164 328694
NMB0318 fatty acid efflux system protein 329606 330757
NMB0319 fatty acid efflux system protein 330784 332307
NMB0320 hypothetical protein 332373 332519
NMB0321 50S ribosomal protein L28 332560 332790
NMB0322 50S ribosomal protein L33 332825 332977
NMB0323 UbiH family protein 334353 333172
NMB0324 50S ribosomal protein L27 334964 334695
NMB0325 50S ribosomal protein L21 335297 334992
NMB0326 octaprenyl-diphosphate synthase 335521 336492
NMB0327 conserved hypothetical protein FRAMESHIFT 336500 336944
NMB0328 hypothetical protein 336993 337165
NMB0329 type IV pilus assembly protein 337388 339061
NMB0330 conserved hypothetical protein 339358 339152
NMB0331 kinase, putative 339983 339354
NMB0332 type IV prepilin peptidase 340845 339988
NMB0333 pilus assembly protein PilG 342151 340922
NMB0334 glucose-6-phosphate isomerase 342508 344148
NMB0335 2,3,4,5-tetrahydropyridine-2-carboxylate N-succinyltransferase 344361 345179
NMB0336 enoyl-(acyl-carrier-protein) reductase 345337 346119
NMB0337 branched-chain amino acid aminotransferase, putative 347364 346369
NMB0338 hypothetical protein 347506 347985
NMB0339 conserved hypothetical protein 347999 349165
NMB0340 lactoylglutathione lyase FRAMESHIFT 349193 349605
NMB0341 tspA protein 352407 349783
NMB0342 intracellular septation protein A 352613 353140
NMB0343 conserved hypothetical protein 353158 353433
NMB0344 BolA/YrbA family protein 353436 353711
NMB0345 cell-binding factor, putative 353763 354626
NMB0346 hypothetical protein 354700 355455
NMB0347 conserved hypothetical protein 355531 356019
NMB0348 conserved hypothetical protein 356053 357060
NMB0349 glutamyl-tRNA synthetase-related protein 358020 357136
NMB0350 hypothetical protein 358760 358311
NMB0351 transaldolase 359966 358914
NMB0352 sugar isomerase, KpsF/GutQ family 360063 361034
NMB0353 conserved hypothetical protein 361255 361788
NMB0354 hypothetical protein 361788 362366
NMB0355 conserved hypothetical protein 362350 362877
NMB0356 ABC transporter, ATP-binding protein 362924 363685
NMB0357 monofunctional biosynthetic peptidoglycan transglycosylase 364858 364160
NMB0358 shikimate 5-dehydrogenase 365670 364864
NMB0359 glutamate--ammonia ligase 365970 367385
NMB0360 AmpG-related protein 367544 368824
NMB0361 conserved hypothetical protein 368824 369096
NMB0362 hypothetical protein 369205 369282
NMB0363 hypothetical protein 369610 369744
NMB0364 FrpC operon protein 370088 370858

## Appendix B

NMB0365 iron-regulated protein FrpC, truncation 370878 371150
NMB0366 hypothetical protein 372373 371243
NMB0367 hypothetical protein 372823 372440
NMB0368 hypothetical protein 373350 372895
NMB0369 hypothetical protein 373720 373334
NMB0370 hypothetical protein 374229 373855
NMB0371 hypothetical protein 374658 374254
NMB0372 hypothetical protein 375341 374667
NMB0373 hypothetical protein 375915 375559
NMB0374 MafB-related protein 377321 375921
NMB0375 mafA protein 378266 377328
NMB0376 hypothetical protein 378379 378266
NMB0377 conserved hypothetical protein 379516 378389
NMB0378 phosphate permease, putative 379807 381378
NMB0379 oxygen-independent coproporphyrinogen III oxidase 383155 381737
NMB0380 transcriptional regulator, Crp/Fnr family 383360 384091
NMB0381 cys regulon transcriptional activator 385157 384210
NMB0382 outer membrane protein class 4 385521 386246
NMB0383 hypothetical protein 386270 386494
NMB0384 hypothetical protein 386773 387066
NMB0385 thiamin-monophosphate kinase 387100 388053
NMB0386 phosphatidylglycerophosphatase A 388049 388531
NMB0387 ABC transporter, ATP-binding protein 390270 388597
NMB0388 sugar transporter, putative 390657 392009
NMB0389 aldose 1-epimerase 392016 393023
NMB0390 maltose phosphorylase 393260 395515
NMB0391 beta-phosphoglucomutase 395531 396193
NMB0392 l-aspartate oxidase 397882 396377
NMB0393 multidrug resistance protein 398266 397934
NMB0394 quinolinate synthetase A 399530 398421
NMB0395 conserved hypothetical protein 399732 400667
NMB0396 nicotinate-nucleotide pyrophosphorylase 400888 401766
NMB0397 hypothetical protein 401797 402081
NMB0398 transcriptional regulator, ArsR family 402176 402454
NMB0399 exodeoxyribonuclease III 402517 403284
NMB0400 transposase, truncated 404230 404799
NMB0401 proline dehydrogenase 409441 405839
NMB0402 sodium/proline symporter 411216 409693
NMB0403 hypothetical protein 411644 411555
NMB0404 conserved hypothetical protein 411699 412016
NMB0405 competence protein ComM 412033 413526
NMB0406 conserved hypothetical protein 413629 414495
NMB0407 thiol:disulfide interchange protein DsbA 414501 415142
NMB0408 bacitracin resistance protein 415178 415996
NMB0409 conserved hypothetical protein 417783 416575
NMB0410 conserved hypothetical protein 418062 418514
NMB0411 conserved hypothetical protein 418514 419497
NMB0412 cell division protein FtsL-related protein 419491 419757
NMB0413 penicillin-binding protein 2 419821 421563
NMB0414 UDP-N-acetylmuramoylalanyl-D-glutamate--2,6-diaminopimelate ligase 421591 423066
NMB0415 conserved hypothetical protein FRAMESHIFT 423092 424736
NMB0416UDP-N-acetylmuramoylalanyl-D-glutamyl-2,6-diaminopimelate--D-alanyl-D- alanyl ligase 424864 426228
NMB0417 hypothetical protein 426234 426407
NMB0418 phospho-N-acetylmuramoyl-pentapeptide-transferase 426657 427736
NMB0419 conserved hypothetical protein 427865 428458
NMB0420 UDP-N-acetylmuramoylalanine--D-glutamate ligase 428545 429879
NMB0421 cell division protein FtsW 430062 431330
NMB0422 UDP-N-acetylglucosamine--N-acetylmuramyl-(pentapeptide) pyrophosphoryl-undecaprenol N-acetylglucosamine transferase 431337 432401
NMB0423 UDP-N-acetylmuramate--alanine ligase 432559 433965
NMB0424 D-alanine--D-alanine ligase 434081 434992

## Appendix B

NMB0425 cell division protein FtsQ 435006 435710
NMB0426 cell division protein FtsA 435799 437040
NMB0427 cell division protein FtsZ 437162 438337
NMB0428 conserved hypothetical protein 438479 439786
NMB0429 hypothetical protein 440162 440263
NMB0430 carboxyphosphonoenolpyruvate phosphonomutase, putative 440412 441287
NMB0431 methylcitrate synthase/citrate synthase 2 441376 442527
NMB0432 conserved hypothetical protein 442683 443468
NMB0433 aconitate hydratase 1 443549 446152
NMB0434 conserved hypothetical protein 446958 448124
NMB0435 acetate kinase 448541 449737
NMB0436 conserved hypothetical protein 450078 450716
NMB0437 conserved hypothetical protein 451289 450849
NMB0438 hypothetical protein 451463 451828
NMB0439 conserved hypothetical protein 451876 453027
NMB0440 prephenate dehydrogenase, putative 453959 453090
NMB0441 nitrilase 454044 454853
NMB0442 opacity protein FRAMESHIFT 455681 454888
NMB0443 transposase, IS30 family 456456 457418
NMB0444 conserved hypothetical protein 457979 458830
NMB0445 bicyclomycin resistance protein, putative 459352 460581
NMB0446 chorismate mutase/prephenate dehydratase 460662 461747
NMB0447 DNA repair protein RecO 461787 462575
NMB0448 pyridoxal phosphate biosynthetic protein PdxJ 462602 463327
NMB0449 hypothetical protein 463482 463703
NMB0450 hypothetical protein 463968 464411
NMB0451 hypothetical protein 464424 465188
NMB0452 holo-(acyl-carrier protein) synthase 465391 465765
NMB0453 mutT protein 465850 466656
NMB0454 hypothetical protein 466652 467071
NMB0455 conserved hypothetical protein 467123 468262
NMB0456 N-acetylmuramoyl-L-alanine amidase 469573 468326
NMB0457 conserved hypothetical protein 470031 469573
NMB0458 glutamate racemase 470233 471042
NMB0459 conserved hypothetical protein 473202 472096
NMB0460 transferrin-binding protein 2 475573 477708
NMB0461 transferrin-binding protein 1 477798 480542
NMB0462 spermidine/putrescine ABC transporter, periplasmic spermidine/putrescine-binding protein 483195 481819
NMB0463 30S ribosomal protein S20 483261 483521
NMB0464 phospholipase A1, putative 483685 484830
NMB0465 conserved hypothetical protein 484976 485674
NMB0466 aspartyl-tRNA synthetase 485735 487540
NMB0467 hypothetical protein 487694 487975
NMB0468 biosynthetic arginine decarboxylase 488145 490034
NMB0469 agmatinase 490136 491056
NMB0470 C4-dicarboxylate transporter 491257 492720
NMB0471 conserved hypothetical protein 494006 492933
NMB0472 8-amino-7-oxononanoate synthase 494229 495368
NMB0473 conserved hypothetical protein 495381 496025
NMB0474 biotin synthesis protein BioC, putative 496016 496795
NMB0475 hypothetical protein 497063 498451
NMB0476 hypothetical protein 498457 499551
NMB0477 conserved hypothetical protein 499566 500099
NMB0478 hypothetical protein 500104 500745
NMB0479 conserved hypothetical protein 500771 501127
NMB0480 TspB-related protein 502193 501801
NMB0481 hypothetical protein 502509 502180
NMB0482 hypothetical protein 502900 502625
NMB0483 Hypothetical protein 503191 502910
NMB0484 hypothetical protein 503396 503202
NMB0485 hypothetical protein 503691 503404
NMB0486 conserved hypothetical protein FRAMESHIFT 505078 503739

## Appendix B

NMB0487 hypothetical protein 505244 505152
NMB0488 hypothetical protein 505800 505309
NMB0489 hypothetical protein 506682 505804
NMB0490 PspA-related protein 507809 506910
NMB0491 hypothetical protein 508744 508304
NMB0492 hypothetical protein 509383 509063
NMB0493 hemagglutinin/hemolysin-related protein 517494 509386
NMB0494 DNA helicase, truncation 518107 517625
NMB0495 replication protein 519187 518207
NMB0496 hemolysin activator-related protein 519134 520810
NMB0497 hemagglutinin/hemolysin-related protein 520922 526826
NMB0498 hypothetical protein 526836 527342
NMB0499 hypothetical protein 527471 529090
NMB0500 hypothetical protein 529102 529476
NMB0501 hypothetical protein 529757 530128
NMB0502 hypothetical protein 530166 532115
NMB0503 hypothetical protein 532134 532562
NMB0504 hypothetical protein 532780 532992
NMB0506 hypothetical protein 533691 535208
NMB0507 hypothetical protein 535208 535693
NMB0508 hypothetical protein 535883 536152
NMB0509 hypothetical protein 536335 537114
NMB0510 hypothetical protein 537136 537396
NMB0511 hypothetical protein 537506 539425
NMB0512 hypothetical protein 539437 539856
NMB0513 hypothetical protein 539896 540294
NMB0514 hypothetical protein 540420 540656
NMB0515 hypothetical protein 540656 541036
NMB0516 hypothetical protein 541042 541974
NMB0517 hypothetical protein 542172 542020
NMB0518 hypothetical protein 542486 542734
NMB0519 hypothetical protein 542725 542925
NMB0520 hypothetical protein 542931 543107
NMB0521 hypothetical protein 543492 543947
NMB0522 transposase, truncated 543958 544080
NMB0523 ABC transporter, ATP-binding protein, truncation 544162 544441
NMB0524 ribonuclease BN, putative 545691 544474
NMB0525 aluminum resistance protein, putative 546236 546892
NMB0526 hypothetical protein 546923 547438
NMB0527 6-pyruvoyl tetrahydrobiopterin synthase, putative 547448 547867
NMB0528 conserved hypothetical protein 548139 548507
NMB0529 conserved hypothetical protein 548507 549142
NMB0530 glycosyl hydrolase, family 3 550869 549787
NMB0531 conserved hypothetical protein 552446 550929
NMB0532 protease DO 554147 552651
NMB0533 endonuclease III 554914 554288
NMB0534 conserved hypothetical protein 555373 554963
NMB0535 glucose/galactose transporter 555906 557183
NMB0536 Na+/H+ antiporter 557477 558853
NMB0537 conserved hypothetical protein 559809 558988
NMB0538 conserved hypothetical protein 560326 559820
NMB0539 porphobilinogen deaminase 560445 561377
NMB0540 aspartate aminotransferase 562977 561787
NMB0541 hypothetical protein 563556 563062
NMB0542 hypothetical protein 563672 563872
NMB0543 L-lactate permease, putative 565630 564047
NMB0544 conserved hypothetical protein 566621 565902
NMB0545 conserved hypothetical protein 566870 570352
NMB0546 alcohol dehydrogenase, propanol-preferring 571566 570523
NMB0547 type IV pilin protein 572238 571852
NMB0548 AcrA/AcrE family protein 572464 573639
NMB0549 ABC transporter, ATP-binding protein 573708 575639
NMB0550 thiol:disulfide interchange protein DsbC 576837 576058
NMB0551 primosomal protein n` 576975 579161

## Appendix B

NMB0552 hypothetical protein 580284 579214
NMB0553 transposase, putative, POINT MUTATION 581288 580335
NMB0554 dnaK protein 584451 582526
NMB0555 hypothetical protein 584931 584662
NMB0556 repressor protein, putative 585119 585802
NMB0557 conserved hypothetical protein 585937 586272
NMB0558 hypothetical protein 586435 586896
NMB0559 ubiquinone biosynthesis protein AarF 586934 588442
NMB0560 serine acetyltransferase 589620 588805
NMB0561 grpE protein 589804 590379
NMB0562 conserved hypothetical protein 590874 590662
NMB0563 thiamine biosynthesis lipoprotein ApbE 591955 590903
NMB0564 Na(+)-translocating NADH-quinone reductase, subunit F 593325 592111
NMB0565 Na(+)-translocating NADH-quinone reductase, subunit E 593932 593342
NMB0566 Na(+)-translocating NADH-quinone reductase, subunit D 594562 593939
NMB0567 Na(+)-translocating NADH-quinone reductase, subunit C 595338 594565
NMB0568 Na(+)-translocating NADH-quinone reductase, subunit B 596563 595334
NMB0569 Na(+)-translocating NADH-quinone reductase, subunit A 597909 596569
NMB0570 hypothetical protein 599680 598262
NMB0571 conserved hypothetical protein 600400 600044
NMB0572 hypothetical protein 601002 600400
NMB0573 transcriptional regulator, AsnC family 601612 601052
NMB0574 glycine cleavage system T protein 602042 603139
NMB0575 glycine cleavage system H protein 603304 603687
NMB0576 glutamyl-tRNA reductase 603842 605086
NMB0577 NosR-related protein 605365 605934
NMB0578 copper ABC transporter, periplasmic copper-binding protein 605991 607022
NMB0579 copper ABC transporter, ATP-binding protein 607083 607700
NMB0580 protein disulfide isomerase NosL, putative 607842 608333
NMB0581 electron transfer flavoprotein-ubiquinone oxidoreductase 610085 608427
NMB0582 bacteriocin resistance protein, putative 610757 610218
NMB0583 IS1016C2 transposase 612651 611986
NMB0584 FrpC operon protein 613242 614054
NMB0585 iron-regulated protein FrpA, putative 614074 617979
~~NMB0586 adhesin, putative 619176 618265~~
NMB0587 membrane protein 620128 619256
NMB0588 ABC transporter, ATP-binding protein 620907 620155
NMB0589 50s ribosomal protein L19 621563 621201
NMB0590 tRNA (guanine-N1)-methyltransferase FRAMESHIFT 622329 621582
NMB0591 16S rRNA processing protein RimM 622838 622332
NMB0592 30S ribosomal protein S16 623099 622857
NMB0593 conserved hypothetical protein 625570 623147
NMB0594 sensor histidine kinase 627094 625691
NMB0595 DNA-binding response regulator 627785 627111
NMB0596 hypothetical protein 629789 627978
NMB0597 hypothetical protein 630132 629782
NMB0598 Maf/YceF/YhdE family protein 630749 630144
NMB0599 conserved hypothetical protein 631572 630805
NMB0600 hypothetical protein 632272 631589
NMB0601 conserved hypothetical protein 632479 632279
NMB0602 hitA protein 632849 632529
NMB0603 phosphoribosyl-ATP cyclohydrolase 633244 632924
NMB0604 alcohol dehydrogenase, zinc-containing 634449 633388
NMB0605 histone deacetylase family protein 636107 635001
NMB0606 conserved hypothetical protein 636235 636498
NMB0607 protein-export membrane protein SecD 636710 638563

## Appendix B

NMB0608 protein-export membrane protein SecF 638570 639502
NMB0609 30s ribosomal protein S15 639728 639994
NMB0610 spermidine/putrescine ABC transporter, ATP-binding protein 640243 641499
NMB0611 spermidine/putrescine ABC transporter, permease protein 641518 642480
NMB0612 spermidine/putrescine ABC transporter, permease protein 642483 643367
NMB0613 hypothetical protein 643392 643496
NMB0614 oxidoreductase, putative 643496 644788
NMB0615 ammonium transporter AmtB, putative 646340 645039
NMB0616 IS1016 family transposase, degenerate 647272 646871
NMB0617 transcription termination factor Rho 648837 647581
NMB0618 phosphoenolpyruvate synthase 651441 649060
NMB0619 conserved hypothetical protein 651853 652671
NMB0620 phosphoglycolate phosphatase 653575 652916
NMB0621 conserved hypothetical protein 654440 653616
NMB0622 outer membrane lipoprotein carrier protein 654867 655487
NMB0623 spermidine/putrescine ABC transporter, periplasmic spermidine/putrescine-binding protein 655763 656899
NMB0624 galactosyltransferase-related protein FRAMESHIFT 657035 658253
NMB0625 conserved hypothetical protein 658297 658824
NMB0626 peptide chain release factor 3 660797 659205
NMB0627 phosphoribosyl-AMP cyclohydrolase 661299 660907
NMB0628 hisF protein 662097 661333
NMB0629 phosphoribosylformimino-5-aminoimidazole carboxamide ribotide isomerase 662847 662113
NMB0630 amidotransferase HisH 663518 662883
NMB0631 phosphate acetyltransferase Pta, putative 665151 663652
NMB0632 iron(III) ABC transporter, ATP-binding protein 666394 665339
NMB0633 iron(III) ABC transporter, permease protein 667932 666418
NMB0634 iron(III) ABC transporter, periplasmic binding protein 668995 668003
NMB0635 transposase, IS30 family 670247 669285
NMB0636 hypothetical protein 670794 670414
NMB0637 argininosuccinate lyase 672228 670855
NMB0638 UTP--glucose-1-phosphate uridylyltransferase 673116 672250
NMB0639 conserved hypothetical protein 673743 673147
NMB0640 hypothetical protein 673969 673739
NMB0641 inorganic pyrophosphatase 674610 674080
NMB0642 dATP pyrophosphohydrolase 675169 674714
NMB0643 MafB-related protein 675614 677437
NMB0644 hypothetical protein 677443 677904
NMB0645 ribonuclease FRAMESHIFT 677948 678275
NMB0646 ribonuclease inhibitor barstar 678290 678574
NMB0647 hypothetical protein 679091 680326
NMB0648 hypothetical protein 680357 680776
NMB0649 hypothetical protein 680970 681191
NMB0650 hypothetical protein 681167 681583
NMB0651 hypothetical protein 681687 682073
NMB0652 mafA protein 682199 683137
NMB0653 MafB-related protein 683144 684409
NMB0654 hypothetical protein 684415 684729
NMB0655 hypothetical protein 684867 685571
NMB0656 hypothetical protein 685600 685926
NMB0657 hypothetical protein 686024 686224
NMB0658 Hypothetical protein 686055 686312
NMB0659 hypothetical protein 686346 686744
NMB0660 hypothetical protein 686929 687315
NMB0661 bis(5`-nucleosyl)-tetraphosphatase, symmetrical/Trk system potassium uptake protein TrkG FRAMESHIFT 689659 687362
NMB0662 ribonuclease, putative 690126 689740
NMB0663 outer membrane protein NsgA 690786 690265
NMB0664 hypothetical protein 691151 690960

## Appendix B

NMB0665 oxygen-independent coprophorphyrinogen III oxidase family protein 692546 691374
NMB0666 DNA ligase 695128 692606
NMB0667 hypothetical protein 696562 695279
NMB0668 ampD protein 697352 696783
NMB0669 conserved hypothetical protein 697436 698428
NMB0670 thymidylate kinase 698491 699108
NMB0671 malate oxidoreductase (NAD) 699333 700610
NMB0672 tetraacyldisaccharide 4`-kinase 701160 702191
NMB0673 hypothetical protein 702394 702978
NMB0674 conserved hypothetical protein 703050 703229
NMB0675 3-deoxy-D-manno-octulosonate cytidylyltransferase 703229 703987
NMB0676 hypothetical protein 704013 704411
NMB0677 hypothetical protein 704610 704723
NMB0678 tryptophan synthase, alpha subunit 705306 706088
NMB0679 acetyl-CoA carboxylase, carboxyl transferase beta subunit 706129 706998
NMB0680 cryptic protein 707672 707064
NMB0681 conserved hypothetical protein 707781 708002
NMB0682 dihydroorotase 708368 709399
NMB0683 N utilization substance protein B 710195 709773
NMB0684 riboflavin synthase, beta subunit 710749 710276
NMB0685 hypothetical protein 711120 710800
NMB0686 ribonuclease III 711287 712003
NMB0687 GTP-binding protein Era 712003 712974
NMB0688 N-(5'-phosphoribosyl)anthranilate isomerase 715446 714823
NMB0689 transcription elongation factor GreB 715996 715508
NMB0690 amidophosphoribosyltransferase 717640 716099
NMB0691 colicin V production protein, putative 718450 717956
NMB0692 tpc protein 719441 718446
NMB0693 folylpolyglutamate synthase/dihydrofolate synthase 720728 719457
NMB0694 folI protein 721205 720762
NMB0695 hypothetical protein 721569 721213
NMB0696 amino acid ABC transporter, ATP-binding protein FRAMESHIFT 722369 721645
NMB0697 dimethyladenosine transferase 723321 722545
NMB0698 hypothetical protein 723518 724204
NMB0699 tryptophan synthase, beta subunit 724290 725489
NMB0700 IgA-specific serine endopeptidase 731118 725674
NMB0701 hypothetical protein 731531 731280
NMB0702 competence protein ComA 732529 734601
NMB0703 competence lipoprotein ComL 735635 734835
NMB0704 ribosomal large subunit pseudouridine synthase D 735634 736755
NMB0705 transporter 737858 736914
NMB0706 conserved hypothetical protein 738418 739194
NMB0707 rare lipoprotein B, putative 739249 739725
NMB0708 DNA polymerase III, delta subunit 739730 740725
NMB0709 Hypothetical protein 740849 741265
NMB0710 Hypothetical protein 741293 741856
NMB0711 conserved hypothetical protein FRAMESHIFT 742826 741946
NMB0712 RNA polymerase sigma-32 factor 744182 743313
NMB0713 apolipoprotein N-acyltransferase, putative 746012 744441
NMB0714 conserved hypothetical protein FRAMESHIFT 746771 746019
NMB0715 Hypothetical protein 746967 747284
NMB0716 Hypothetical protein 747440 747727
NMB0717 cytochrome, putative 748209 747796
NMB0718 ferrochelatase 749572 748493
NMB0719 queuine tRNA-ribosyltransferase 750697 749585
NMB0720 threonyl-tRNA synthetase 751005 752915
NMB0721 translation initiation factor 3 752990 753454
NMB0722 50S ribosomal protein L35 753604 753798
NMB0723 50S ribosomal protein L20 753814 754170
NMB0724 phenylalanyl-tRNA synthetase, alpha chain 754519 755508
NMB0725 modification methylase HgaI-1 755694 756749

## Appendix B

NMB0726 type II restriction enzyme HgaI 756755 758221
NMB0727 N-6 adenine-specific DNA methylase 758221 758868
NMB0728 phenylalanyl-tRNA synthetase, beta chain 758896 761256
NMB0729 integration host factor, alpha subunit 761333 761632
NMB0730 hypothetical protein 762257 762739
NMB0731 hypothetical protein 763002 763226
NMB0732 adenosylmethionine-8-amino-7-oxononanoate aminotransferase 763559 764857
NMB0733 dethiobiotin synthase 764857 765501
NMB0734 hypothetical protein 765519 765992
NMB0735 4-hydroxybenzoate octaprenyltransferase 766025 766912
NMB0736 PTS system, nitrogen regulatory IIA protein 767100 767546
NMB0737 HPr kinase/phosphatase, putative 767551 768510
NMB0738 conserved hypothetical protein 768494 769345
NMB0739 conserved hypothetical protein 769429 770943
NMB0740 DNA repair protein RecN 771255 772925
NMB0741 conserved hypothetical protein 775384 773948
NMB0742 conserved hypothetical protein 775684 776040
NMB0743 ubiquinone/menaquinone biosynthesis methlytransferase UbiE 776097 776831
NMB0744 hypothetical protein 777054 777530
NMB0745 2-amino-4-hydroxy-6-hydroxymethyldihydropteridine-pyrophosphokinase 778153 777662
NMB0746 conserved hypothetical protein 778537 778166
NMB0747 conserved hypothetical protein 779157 778594
NMB0748 host factor-I 779535 779245
NMB0749 penicillin-binding protein 4 780602 779667
NMB0750 bacterioferritin comigratory protein 780923 781360
NMB0751 integrase/recombinase XerD 781415 782287
NMB0752 bacterioferritin-associated ferredoxin, putative 782462 782659
NMB0753 conserved hypothetical protein 782828 783058
NMB0754 hypothetical protein 783066 783173
NMB0755 hypothetical protein 783194 783334
NMB0756 dTDP-L-rhamnose synthase, putative 784398 783481
NMB0757 phosphoribosylaminoimidazole-succinocarboxamide synthase 784598 785458
NMB0758 polyribonucleotide nucleotidyltransferase 785695 787815
NMB0759 conserved hypothetical protein 788619 787894
NMB0760 diaminopimelate epimerase 789006 789854
NMB0761 hypothetical protein 789940 790164
NMB0762 hypothetical protein 790198 790653
NMB0763 cysteine synthase 790653 791582
NMB0764 conserved hypothetical protein 792048 792950
NMB0765 signal peptidase I 794128 793112
NMB0766 GTP-binding protein LepA 796064 794274
NMB0767 5-methylthioadenosine nucleosidase/S-adenosylhomocysteine nucleosidase 796909 796211
NMB0768 twitching motility protein PilT 797095 798204
NMB0769 DNA polymerase III, delta prime subunit, putative 798241 799215
NMB0770 type IV pilus assembly protein PilZ, putative 799222 799569
NMB0771 conserved hypothetical protein 799577 800353
NMB0772 conserved hypothetical protein 800382 800594
NMB0773 conserved hypothetical protein 800698 801006
NMB0774 uracil phosphoribosyltransferase 801115 801738
NMB0775 hypothetical protein 801764 802081
NMB0776 conserved hypothetical protein 802335 802751
NMB0777 uroporphyrinogen-III synthase HemD, putative 802796 803533
NMB0778 uroporphyrin-III C-methyltransferase HemX, putative 803611 804882
NMB0779 hypothetical protein 804882 806102
NMB0780 hypothetical protein 806138 806575
NMB0781 uroporphyrinogen decarboxylase 806732 807793
NMB0782 DNA repair protein RadA 807982 809358
NMB0783 conserved hypothetical protein 810116 809640
NMB0784 phage shock protein E precursor, putative 810717 810361

## Appendix B

NMB0785 exodeoxyribonuclease V 135 KD polypeptide 814370 810759
NMB0786 conserved hypothetical protein 815358 814453
NMB0787 amino acid ABC transporter, periplasmic amino acid-binding protein 815643 816467
NMB0788 amino acid ABC transporter, permease protein 816514 817173
NMB0789 amino acid ABC transporter, ATP-binding protein 817186 817938
NMB0790 phosphoglucomutase 819343 817964
NMB0791 peptidyl-prolyl cis-trans isomerase 820019 819513
NMB0792 transporter, NadC family 821553 820141
NMB0793 hypothetical protein 821759 821553
NMB0794 hypothetical protein 822146 821787
NMB0795 peptidyl-tRNA hydrolase 822988 822413
NMB0796 conserved hypothetical protein 823319 823044
NMB0797 conserved hypothetical protein 823749 823315
NMB0798 cell division protein FtsH 825932 823968
NMB0799 cell division protein FtsJ 826616 825999
NMB0800 conserved hypothetical protein 826726 827007
NMB0801 delta-aminolevulinic acid dehydratase 827193 828191
NMB0802 cystathionine gamma-synthase 829414 828260
NMB0803 conserved hypothetical protein 829606 830376
NMB0804 NAD(P)H nitroreductase, putative 830489 831151
NMB0805 transposase, IS30 family 831295 832257
NMB0806 conserved hypothetical protein 833050 832295
NMB0807 conserved hypothetical protein 833965 833078
NMB0808 hypothetical protein 834551 833988
NMB0809 conserved hypothetical protein 835399 834605
NMB0810 transcriptional regulator, TetR family 836104 835457
NMB0811 UDP-N-acetylpyruvoylglucosamine reductase 837156 836119
NMB0812 conserved hypothetical protein 838579 837203
NMB0813 hypothetical protein 838634 838819
NMB0814 histidyl-tRNA synthetase 838914 840062
NMB0815 adenylosuccinate synthetase 840163 841464
NMB0816 hypothetical protein 841592 841903
NMB0817 hypothetical protein 841932 842312
NMB0818 hypothetical protein 842329 842736
NMB0819 hypothetical protein 842856 843245
NMB0820 hypothetical protein 843456 843845
NMB0821 hypothetical protein 843962 844519
NMB0822 heat shock protein HtpX 845866 844826
NMB0823 adenylate kinase 845878 846522
NMB0824 orotidine 5`-phosphate decarboxylase 847051 847788
NMB0825 ADP-heptose synthase, putative 847846 848814
NMB0826 C-5 cytosine-specific DNA methylase 848854 850086
NMB0827 type II restriction enzyme-related protein FRAMESHIFT 850091 851119
NMB0828 ADP-L-glycero-D-mannoheptose-6-epimerase 851251 852252
NMB0829 type I restriction enzyme EcoR124II M protein 852329 853870
NMB0830 conserved hypothetical protein 853870 854877
NMB0831 type I restriction enzyme S protein, degenerate 855046 856216
NMB0832 anticodon nuclease 856277 857416
NMB0833 type I restriction enzyme-related protein 857416 857799
NMB0834 transposase, IS30 family 858756 857794
NMB0835 type I restriction enzyme EcoR124II R protein, putative 858832 861594
NMB0836 ATP-dependent Clp protease, ATP-binding subunit ClpA 863945 861639
NMB0837 conserved hypothetical protein 864249 863950
NMB0838 cold-shock domain family protein 864492 864692
NMB0839 pmbA protein 866323 864995
NMB0840 conserved hypothetical protein 866446 866979
NMB0841 hypothetical protein 867029 867742
NMB0842 single-stranded-DNA-specific exonuclease RecJ 867814 869511
NMB0843 polyA polymerase 869811 871169
NMB0844 hypothetical protein 871345 871665
NMB0845 PhoH-related protein 872732 871782

## Appendix B

NMB0846 LPS biosynthesis protein-related protein 873905 872874
NMB0847 hypothetical protein 874235 874065
NMB0848 hypothetical protein 874369 875070
NMB0849 deoxycytidine triphosphate deaminase, putative 875703 875140
NMB0850 hypothetical protein 876185 875772
NMB0851 recombination associated protein RdgC 877146 876250
NMB0852 essential GTPase 878634 877180
NMB0853 conserved hypothetical protein 879413 878787
NMB0854 histidyl-tRNA synthetase 880709 879417
NMB0855 bacteriocin resistance protein, putative 881459 880806
NMB0856 hypothetical protein 882208 881744
NMB0857 hypothetical protein 882441 882268
NMB0858 hypothetical protein 882645 882448
NMB0859 hypothetical protein 883025 882651
NMB0860 hypothetical protein 883340 883086
NMB0861 hypothetical protein 883975 883433
NMB0862 hypothetical protein 884091 883975
NMB0863 hypothetical protein 884410 884141
NMB0864 hypothetical protein 884966 884679
NMB0865 hypothetical protein 885445 884975
NMB0866 hypothetical protein 886357 885491
NMB0867 YabO/YceC/SfhB family protein 886521 887441
NMB0868 conserved hypothetical protein 888163 887525
NMB0869 hypothetical protein 889009 888221
NMB0870 3-methyl-2-oxobutanoate hydroxymethyltransferase 889502 890290
NMB0871 pantoate--beta-alanine ligase 890416 891249
NMB0872 conserved hypothetical protein 891416 893257
NMB0873 outer membrane lipoprotein LolB, putative 893400 893978
NMB0874 conserved hypothetical protein 893991 894833
NMB0875 ribose-phosphate pyrophosphokinase 895258 896238
NMB0876 50S ribosomal protein L25 896308 896877
NMB0877 penicillin-binding protein 898174 897008
NMB0878 threonine dehydratase 898322 899845
NMB0879 sulfate ABC transporter, ATP-binding protein 900978 899908
NMB0880 sulfate ABC transporter, permease protein 901835 900978
NMB0881 sulfate ABC transporter, permease protein 902923 902090
NMB0882 hypothetical protein 903214 903543
NMB0883 conserved hypothetical protein 903878 904384
NMB0884 superoxide dismutase 905491 904907
NMB0885 replicative DNA helicase 905655 907058
NMB0886 fimbrial protein FimT 907370 908035
NMB0887 type IV pilus assembly protein PilV, putatve 908056 908667
NMB0888 hypothetical protein 908667 909605
NMB0889 hypothetical protein 909587 910177
NMB0890 type IV pilin-related protein 910170 910655
NMB0891 hypothetical protein 911708 911944
NMB0892 AzlC-related protein 912795 912376
NMB0893 deoxyuridine 5`-triphosphate nucleotidohydrolase 912995 913444
NMB0894 aminotransferase, class I 913525 914709
NMB0895 conserved hypothetical protein 914975 915751
NMB0896 integrase, FRAMESHIFT 916283 917352
NMB0897 hypothetical protein 917468 917845
NMB0898 hypothetical protein 917894 918079
NMB0899 hypothetical protein 918396 918749
NMB0900 hypothetical protein 919621 920535
NMB0901 D-lactate dehydrogenase-related protein 920880 920599
NMB0902 hypothetical protein 921133 920945
NMB0903 hypothetical protein 921429 921139
NMB0904 hypothetical protein 921686 921429
NMB0905 hypothetical protein 921936 921724
NMB0906 hypothetical protein 922860 922009
NMB0907 hypothetical protein 923244 922888
NMB0908 hypothetical protein 923512 923315
NMB0909 hypothetical protein 924280 923759

## Appendix B

NMB0910 transcriptional regulator 925000 924287
NMB0911 transposase, IS30 family 926382 925420
NMB0912 hypothetical protein 926526 927149
NMB0913 pemK protein 927552 927208
NMB0914 pemI protein 927790 927557
NMB0915 hypothetical protein 928640 928152
NMB0916 hypothetical protein 928799 928662
NMB0917 death-on-curing protein 929446 929081
NMB0918 hypothetical protein 929574 929446
NMB0919 IS1106 transposase, putative 930929 929973
NMB0920 isocitrate dehydrogenase 934317 932095
NMB0921 hypothetical protein 934522 934325
NMB0922 alpha-2,3-sialyltransferase 934750 935862
NMB0923 cytochrome c 936488 936033
NMB0924 oxidoreductase, short-chain dehydrogenase/reductase family 936607 937425
NMB0925 acyl CoA thioester hydrolase family protein 937925 937482
NMB0926 opacity protein 940336 939513
NMB0927 proline iminopeptidase 941840 942769
NMB0928 hypothetical protein 944025 942832
NMB0929 dihydrodipicolinate synthase 944909 944037
NMB0930 xanthine/uracil permease family protein 945369 946757
NMB0931 RNA methyltransferase, TrmH family 947574 946825
NMB0932 conserved hypothetical protein 948129 947644
NMB0933 cytidine and deoxycytidylate deaminase family protein 948580 948137
NMB0934 DNA transformation protein tfoX-related protein 948853 948625
NMB0935 tRNA delta(2)-isopentenylpyrophosphate transferase 949798 948860
NMB0936 hypothetical protein 951481 950180
NMB0937 elongation factor P (EF-P) 951788 952345
NMB0938 hypothetical protein 953235 952402
NMB0939 conserved hypothetical protein 953933 953355
NMB0940 homoserine O-acetyltransferase 955069 953933
NMB0941 50S ribosomal protein L36 955756 955634
NMB0942 50S ribosomal protein L31, putative 956031 955759
NMB0943 5,10-methylenetetrahydrofolate reductase 956231 957106
NMB0944 5-methyltetrahydropteroyltriglutamate-homocysteine methyltransferase 957247 959520
NMB0945 hypothetical protein 959535 959696
NMB0946 peroxiredoxin 2 family protein/glutaredoxin 959802 960536
NMB0947 lipoamide dehydrogenase, putative 960788 962188
NMB0948 succinate dehydrogenase, cytochrome b556 subunit 962470 962844
NMB0949 succinate dehydrogenase, hydrophobic membrane anchor protein 962841 963179
NMB0950 succinate dehydrogenase, flavoprotein subunit 963185 964945
NMB0951 succinate dehydrogenase, iron-sulfur protein 965068 965772
NMB0952 conserved hypothetical protein 965779 966024
NMB0953 hypothetical protein 966024 966104
NMB0954 citrate synthase 966139 967419
NMB0955 2-oxoglutarate dehydrogenase, E1 component 967627 970452
NMB0956 2-oxoglutarate dehydrogenase, E2 component, dihydrolipoamide succinyltransferase 970555 971733
NMB0957 2-oxoglutarate dehydrogenase, E3 component, lipoamide dehydrogenase 972101 973531
NMB0958 hypothetical protein 973659 973943
NMB0959 succinyl-CoA synthetase, beta subunit 974045 975208
NMB0960 succinyl-CoA synthetase, alpha subunit 975222 976109
NMB0961 funZ protein 978267 976675
NMB0962 excinuclease ABC, subunit A 981150 978304
NMB0963 phosphatidylserine decarboxylase precursor-related protein 981305 982099
NMB0964 TonB-dependent receptor 985503 983230
NMB0965 hypothetical protein 985832 985564

## Appendix B

NMB0966 para-aminobenzoate synthase glutamine amidotransferase component II 985925 986512
NMB0967 anthranilate phosphoribosyltransferase 986579 987634
NMB0968 hypothetical protein 987644 987729
NMB0969 hypothetical protein 988030 987792
NMB0970 conserved hypothetical protein, FRAMESHIFT 988106 989527
NMB0971 hypothetical protein 989493 989780
NMB0972 hypothetical protein 989788 989982
NMB0973 hypothetical protein 989993 990274
NMB0974 hypothetical protein 990284 990559
NMB0975 hypothetical protein 990690 991004
NMB0976 TspB-related protein 990991 991383
NMB0977 modulator of drug activity B, putative 991676 992146
NMB0978 NAD(P) transhydrogenase, beta subunit 993742 992360
NMB0979 hypothetical protein 994205 993825
NMB0980 NAD(P) transhydrogenase, alpha subunit 995750 994212
NMB0981 phosphoserine phosphatase 996040 996870
NMB0982 chloride channel protein-related protein 997018 998157
NMB0983 phosphoribosylaminoimidazolecarboxamide formyltransferase/IMP cyclohydrolase 998324 999901
NMB0984 transposase, putative, degenerate 1000517 1001457
NMB0985 E16-related protein 1001522 1002016
NMB0986 hypothetical protein 1001997 1002425
NMB0987 N-acetylmuramoyl-L-alanine amidase, putative 1002736 1003278
NMB0988 hypothetical protein 1003278 1003478
NMB0989 hypothetical protein 1003484 1003645
NMB0990 hypothetical protein 1003859 1004260
NMB0991 IS1106 transposase 1005417 1004308
NMB0992 adhesin 1007326 1005554
NMB0993 rubredoxin 1009428 1009261
NMB0994 acyl-CoA dehydrogenase family protein 1011202 1010114
NMB0995 macrophage infectivity potentiator-related protein 1012020 1011340
NMB0996 hypothetical protein 1012411 1012043
NMB0997 D-lactate dehydrogenase 1014397 1012709
NMB0998 oxidoreductase, putative 1014921 1018751
NMB0999 NifR3/SMM1 family protein 1018935 1019933
NMB1000 IS1106 transposase, putative FRAMESHIFT 1020537 1021551
NMB1001 integrase protein, degenerate 1023183 1022614
NMB1002 hypothetical protein 1024370 1023498
NMB1003 hypothetical protein 1024711 1024418
NMB1004 hypothetical protein 1024962 1024720
NMB1005 hypothetical protein 1025179 1024958
NMB1006 hypothetical protein 1025360 1025184
NMB1007 transcriptional regulator 1025451 1025819
NMB1008 hypothetical protein 1025824 1026444
NMB1009 conserved hypothetical protein 1026440 1026631
NMB1010 hypothetical protein 1026658 1027218
NMB1011 hypothetical protein 1027252 1028196
NMB1012 hypothetical protein 1028284 1028784
NMB1013 hypothetical protein 1028801 1028971
NMB1014 conserved hypothetical protein 1029045 1029635
NMB1015 IS150 transposase, putative FRAMESHIFT 1029653 1030443
NMB1016 conserved hypothetical protein 1031794 1031192
NMB1017 sulfate ABC transporter, periplasmic sulfate-binding protein 1033574 1032522
NMB1018 conserved hypothetical protein 1034162 1033683
NMB1019 phosphoribosylaminoimidazole carboxylase, ATPase subunit 1035345 1034212
NMB1020 hypothetical protein 1035887 1035345
NMB1021 anthranilate synthase component I 1037359 1035887
NMB1022 transposase, IS30 family 1038444 1037482
NMB1023 conserved hypothetical protein 1039543 1038587
NMB1024 conserved hypothetical protein 1040502 1039639
NMB1025 conserved hypothetical protein 1040896 1040537

## Appendix B

NMB1026 conserved hypothetical protein 1040971 1041447
NMB1027 dnaJ protein, truncation 1041473 1042192
NMB1028 conserved hypothetical protein 1042197 1043069
NMB1029 aspartate ammonia-lyase 1044541 1043147
NMB1030 conserved hypothetical protein 1045565 1045005
NMB1031 3-isopropylmalate dehydrogenase 1046798 1045731
NMB1032 type II restriction enzyme NlaIV 1047563 1046835
NMB1033 modification methylase NlaIV 1048850 1047582
NMB1034 3-isopropylmalate dehydratase, small subunit 1049666 1049028
NMB1035 hypothetical protein 1049982 1049731
NMB1036 3-isopropylmalate dehydratase, large subunit 1051488 1050082
NMB1037 glutamate--cysteine ligase 1051748 1053094
NMB1038 DNA repair protein RadC 1053220 1053894
NMB1039 conserved hypothetical protein 1053970 1054692
NMB1040 hypothetical protein 1054848 1056125
NMB1041 GTP-binding protein 1056133 1057308
NMB1042 cation transport ATPase, E1-E2 family 1057308 1059776
NMB1043 hypothetical protein 1059940 1060142
NMB1044 ferredoxin--NADP reductase 1061316 1060543
NMB1045 hypothetical protein 1062298 1061507
NMB1046 threonine synthase 1063753 1062347
NMB1047 hypothetical protein 1064197 1063829
NMB1048 hypothetical protein 1065918 1064452
NMB1049 transcriptional regulator, putative 1066174 1067085
NMB1050 transposase, IS30 family 1068512 1067550
NMB1051 ABC transporter, ATP-binding protein 1070544 1068637
NMB1052 dedA protein 1071207 1070566
NMB1053 class 5 outer membrane protein 1072189 1071374
NMB1054 IS1106 transposase, degenerate 1073920 1072988
NMB1055 serine hydroxymethyltransferase 1075474 1074227
NMB1056 hypothetical protein 1075753 1075544
NMB1057 gamma-glutamyltranspeptidase 1077776 1075959
NMB1058 conserved hypothetical protein FRAMESHIFT 1078161 1077902
NMB1059 conserved hypothetical protein 1078505 1078720
NMB1060 fructose-1,6-bisphosphatase 1079840 1078869
NMB1061 conserved hypothetical protein 1080931 1080089
NMB1062 conserved hypothetical protein 1081610 1081011
NMB1063 dihydroneopterin aldolase 1081666 1082019
NMB1064 conserved hypothetical protein 1082056 1082589
NMB1065 crcB protein 1083465 1083109
NMB1066 hypothetical protein 1084174 1083497
NMB1067 cell division protein FtsK 1084339 1087380
NMB1068 gamma-glutamyl phosphate reductase 1088870 1087611
NMB1069 glutamate 5-kinase 1089992 1088886
NMB1070 2-isopropylmalate synthase 1090477 1092027
NMB1071 conserved hypothetical protein 1092125 1092784
NMB1072 prolipoprotein diacylglyceryl transferase 1093721 1092873
NMB1073 conserved hypothetical protein 1094922 1093795
NMB1074 acetylglutamate kinase 1095092 1095985
NMB1075 conserved hypothetical protein 1098302 1097637
NMB1076 DnaA-related protein 1098967 1098302
NMB1077 ABC transporter, ATP-binding protein, truncation 1099623 1099075
NMB1078 transcriptional regulator, UmuD/LexA family 1100312 1099875
NMB1079 hypothetical protein 1100580 1100425
NMB1080 ner protein FRAMESHIFT 1100802 1101061
NMB1081 bacteriophage transposase 1101126 1103108
NMB1082 hypothetical protein 1103120 1103317
NMB1083 bacteriophage DNA transposition protein B, putative 1103481 1104650
NMB1084 hypothetical protein 1104655 1105173
NMB1085 N-acetylmuramoyl-L-alanine amidase, putative 1105319 1105861
NMB1086 hypothetical protein 1106234 1106467
NMB1087 hypothetical protein 1106758 1107060
NMB1088 conserved hypothetical protein 1107278 1107111

## Appendix B

NMB1089 hypothetical protein 1107506 1107841
NMB1090 hypothetical protein 1107856 1108119
NMB1091 hypothetical protein 1108119 1108313
NMB1092 hypothetical protein 1108319 1108822
NMB1093 hypothetical protein 1109412 1108825
NMB1094 hypothetical protein 1109497 1111044
NMB1095 conserved hypothetical protein 1111047 1112612
NMB1096 conserved hypothetical protein 1112602 1113894
NMB1097 cryptic Mu-phage G protein, putative 1114007 1114419
NMB1098 I protein, putative 1114653 1115711
NMB1099 transposase, IS30 family 1116767 1115805
NMB1100 hypothetical protein 1116795 1117274
NMB1101 conserved hypothetical protein 1117277 1117696
NMB1102 hypothetical protein 1117746 1118336
NMB1103 hypothetical protein 1118336 1118530
NMB1104 phage sheath protein 1118536 1119942
NMB1105 hypothetical protein 1120010 1120384
NMB1106 hypothetical protein 1120391 1120753
NMB1107 hypothetical protein 1121610 1121011
NMB1108 hypothetical protein 1121780 1123933
NMB1109 phage virion protein, putative 1123936 1125264
NMB1110 tail protein, 43 kDa 1125257 1126399
NMB1111 baseplate assembly protein V, putative 1126399 1127064
NMB1112 conserved hypothetical protein 1127168 1127512
NMB1113 conserved hypothetical protein FRAMESHIFT 1127529 1128580
NMB1114 conserved hypothetical protein 1128580 1129137
NMB1115 tail fibre protein, putative 1129151 1131121
NMB1116 hypothetical protein 1131560 1132084
NMB1117 hypothetical protein 1132350 1132204
NMB1118 conserved hypothetical protein 1132762 1132478
NMB1119 conserved hypothetical protein 1132842 1133444
NMB1120 hypothetical protein 1133426 1133719
NMB1121 conserved hypothetical protein 1133719 1133925
NMB1122 ABC transporter, ATP-binding protein FRAMESHIFT 1135181 1134041
NMB1198 conserved hypothetical protein 1199352 1198465
NMB1161 hypothetical protein 1167620 1167426
NMB1162 hypothetical protein 1168307 1167663
NMB1163 hypothetical protein 1168675 1168307
NMB1164 hypothetical protein 1169353 1168685
NMB1165 oxidoreductase, short chain dehydrogenase/reductase family 1170237 1169521
NMB1128 conserved hypothetical protein 1139597 1138287
NMB1167 hypothetical protein 1171869 1171666
NMB1168 phytoene synthase, putative 1172903 1172034
NMB1131 chaperone protein HscA 1142897 1141038
NMB1132 hypothetical protein 1143630 1142977
NMB1171 conserved hypothetical protein / ankyrin-related protein 1176464 1175706
NMB1172 ferredoxin, 2Fe-2S type 1176860 1176522
NMB1173 hypothetical protein 1177278 1177138
NMB1136 hypothetical protein 1146017 1145337
NMB1175 conserved hypothetical protein 1178247 1178053
NMB1176 conserved hypothetical protein 1178719 1178321
NMB1139 acetyl-CoA carboxylase, carboxyl transferase alpha subunit 1147851 1146895
NMB1140 mesJ protein FRAMESHIFT 1149229 1147948
NMB1179 RNA methyltransferase, TrmH family 1182124 1181516
NMB1180 hypothetical protein 1182411 1182178
NMB1181 hypothetical protein 1182945 1182583
NMB1182 hypothetical protein 1183262 1182960
NMB1145 UDP-N-acetylmuramate:L-alanyl-gamma-D-glutamyl-meso-diaminopimelate ligase 1152664 1151291
NMB1146 biotin synthetase 1153923 1152874
NMB1185 hypothetical protein 1186675 1186043

## Appendix B

NMB1148 hypothetical protein 1154845 1154693
NMB1187 hypothetical protein 1187052 1186912
NMB1150 dihydroxy-acid dehydratase 1157144 1155288
NMB1189 sulfite reductase hemoprotein, beta-component 1191122 1189356
NMB1190 sulfite reductase (NADPH) flavoprotein, alpha component 1192963 1191152
NMB1153 sulfate adenylyltransferase, subunit 1 1162210 1160927, plasmid protein [redacted]
NMB1192 sulfate adenylyltransferase, subunit 2 1195208 1194288
NMB1155 phosphoadenosine phosphosulfate reductase 1163950 1163213
NMB1194 siroheme synthase 1197448 1196000
NMB1195 hypothetical protein 1197732 1197577
NMB1158 nickel-dependent hydrogenase, b-type cytochrome subunit 1166365 1165712
NMB1197 conserved hypothetical protein 1199352 1198465
NMB1199 GTP-binding protein TypA 1201433 1199625
NMB1200 ribonuclease II family protein 1202272 1204644
NMB1201 IMP dehydrogenase 1206449 1204989
NMB1202 hypothetical protein 1207237 1206779
NMB1203 protein-PII uridylyltransferase 1209886 1207331
NMB1204 transcriptional regulator 1210255 1209938
NMB1205 hypothetical protein 1210426 1210283
NMB1206 bacterioferritin B 1211053 1210583
NMB1207 bacterioferritin A 1211545 1211084
NMB1208 hypothetical protein 1211610 1211810
NMB1209 hypothetical protein 1211900 1212100
NMB1210 toxin-activating protein, putative 1212121 1212585
NMB1211 hypothetical protein 1212984 1212745
NMB1212 hypothetical protein 1213319 1212984
NMB1213 hypothetical protein 1213678 1213319
NMB1214 hemagglutinin/hemolysin-related protein 1220496 1213678
NMB1215 hypothetical protein 1220814 1220659
NMB1216 lipoic acid synthetase 1221989 1221009
NMB1217 lipoate-protein ligase B 1222554 1221985
NMB1218 conserved hypothetical protein 1222882 1222610
NMB1219 transporter, putative 1223067 1224134
NMB1220 stomatin/Mec-2 family protein 1225281 1224337
NMB1221 hypothetical protein 1225703 1225299
NMB1222 uracil-DNA glycosylase 1225784 1226440
NMB1223 site-specific DNA methylase, degenerate 1226520 1229028
NMB1224 hypothetical protein 1229552 1229154
NMB1225 hypothetical protein 1230112 1229600
NMB1226 ABC transporter, ATP-binding protein 1232500 1230581
NMB1227 conserved hypothetical protein 1232972 1232580
NMB1228 homoserine dehydrogenase 1233145 1234449
NMB1229 hypothetical protein 1234445 1234876
NMB1230 DNA-binding protein HU-beta 1235207 1234941
NMB1231 ATP-dependent protease La 1237851 1235392
NMB1232 conserved hypothetical protein 1238285 1239202
NMB1233 exodeoxyribonuclease V, alpha subunit 1240978 1239236
NMB1234 ABC transporter, ATP-binding protein 1241741 1241049
NMB1235 conserved hypothetical protein 1242981 1241737
NMB1236 hypothetical protein 1243186 1243461
NMB1237 recombination protein RecR 1244140 1243523
NMB1238 peptidyl-prolyl cis-trans isomerase-related protein 1245742 1244207
NMB1239 conserved hypothetical protein 1246176 1245805
NMB1240 ABC transporter, ATP-binding protein 1246326 1247951
NMB1241 tRNA nucleotidyltransferase 1248026 1249276
NMB1242 hypothetical protein 1249502 1249807
NMB1243 Holliday junction DNA helicase RuvB 1249892 1250920
NMB1244 ribulose-phosphate 3-epimerase 1251674 1250949
NMB1245 hypothetical protein 1252367 1252035
NMB1246 conserved hypothetical protein 1253294 1252434

## Appendix B

NMB1247 riboflavin synthase, alpha subunit 1254006 1253305
NMB1248 molybdopterin-guanine dinucleotide biosynthesis protein A FRAMESHIFT 1254659 1254085
NMB1249 nitrate/nitrite sensory protein NarX, putative 1254901 1256670
NMB1250 transcriptional regulator, LuxR family 1256670 1257323
NMB1251 transposase, IS30 family 1258731 1257769
NMB1252 phosphoribosylformylglycinamidine cyclo-ligase 1259914 1258883
NMB1253 hypothetical protein 1260672 1261346
NMB1254 GTP cyclohydrolase II 1261342 1261932
NMB1255 glycosyl transferase, degenerate 1262256 1263263
NMB1256 GTP cyclohydrolase II/3,4-dihydroxy-2-butanone-4-phosphate synthase 1263728 1264816
NMB1257 site-specific DNA methylase, degenerate 1265357 1265130
NMB1258 conserved hypothetical protein 1267046 1265739
NMB1259 transposase, IS30 family 1267584 1268546
NMB1260 type III restriction-modification system EcoPI enzyme, subunit res 1271565 1268629
NMB1261 type III restriction-modification system EcoPI enzyme, subunit mod POINT MUTATION FRAMESHIFT 1273661 1271581
NMB1262 peptidyl-prolyl cis-trans isomerase 1274334 1273780
NMB1263 CobW-related protein 1275316 1274402
NMB1264 conserved hypothetical protein 1275771 1275502
NMB1265 conserved hypothetical protein 1276061 1275771
NMB1266 zinc uptake regulation protein, putative 1276582 1276109
NMB1267 low molecular weight protein tyrosine-phosphatase 1277108 1276656
NMB1268 conserved hypothetical protein 1278348 1277236
NMB1269 hypothetical protein 1279559 1278465
NMB1270 conserved hypothetical protein 1281272 1279644
NMB1271 mercury transport periplasmic protein, putative 1281584 1281375
NMB1272 hypothetical protein 1281765 1281625
NMB1273 alginate O-acetylation protein AlgI, putative 1282215 1283648
NMB1274 hypothetical protein 1283662 1284642
NMB1275 hypothetical protein 1284642 1286083
NMB1276 long-chain-fatty-acid--CoA ligase 1286122 1287672
NMB1277 transporter, BCCT family 1289792 1287768
NMB1278 site-specific recombinase 1290081 1292084
NMB1279 membrane-bound lytic murein transglycosylase B, putative 1293319 1292213
NMB1280 very long chain acyl-CoA dehydrogenase-related protein 1294948 1293524
NMB1281 transcription-repair coupling factor 1295133 1299269
NMB1282 aspartate 1-decarboxylase 1299421 1299801
NMB1283 2-dehydro-3-deoxyphosphooctonate aldolase 1299826 1300665
NMB1284 hypothetical protein 1300683 1301120
NMB1285 enolase 1301171 1302454
NMB1286 conserved hypothetical protein 1302471 1302746
NMB1287 ferredoxin, putative 1303080 1302793
NMB1288 ribonucleoside-diphosphate reductase, beta subunit 1304479 1303328
NMB1289 type II restriction enzyme, putative 1305706 1304522
NMB1290 C-5 cytosine-specific DNA-methylase 1306712 1305702
NMB1291 ribonucleoside-diphosphate reductase, alpha subunit 1309049 1306773
NMB1292 hypothetical protein 1309394 1309209
NMB1293 hypothetical protein 1309563 1309886
NMB1294 1-acyl-sn-glycerol-3-phosphate acyltransferase 1310967 1310203
NMB1295 formamidopyrimidine-DNA glycosylase 1311882 1311058
NMB1296 hypothetical protein 1312599 1311937
NMB1297 membrane-bound lytic murein transglycosylase D 1312778 1314751
NMB1298 ribosomal small subunit pseudouridine synthase A 1314822 1315511
NMB1299 sodium- and chloride-dependent transporter, degenerate 1316091 1317454
NMB1300 cytidylate kinase 1317701 1318354
NMB1301 30S ribosomal protein S1 1318513 1320195
NMB1302 integration host factor, beta subunit 1320209 1320520

## Appendix B

NMB1303 transcriptional regulator, MerR family 1321281 1320877
NMB1304 alcohol dehydrogenase, class III 1321402 1322535
NMB1305 esterase, putative 1322547 1323371
NMB1306 conserved hypothetical protein 1323765 1324913
NMB1307 nucleoside diphosphate kinase 1324975 1325397
NMB1308 conserved hypothetical protein 1325543 1326634
NMB1309 fimbrial biogenesis and twitching motility protein, putative 1326640 1327398
NMB1310 gcpE protein 1327417 1328679
NMB1311 hypothetical protein 1328970 1328737
NMB1312 ATP-dependent Clp protease, proteolytic subunit 1329655 1329128
NMB1313 trigger factor 1331148 1329838
NMB1314 cell division protein FtsK 1333791 1331356
NMB1315 uracil permease 1334014 1335222
NMB1316 hypothetical protein 1335289 1335726
NMB1317 hypothetical protein 1335865 1336266
NMB1318 CDP-diacylglycerol--serine O-phosphatidyltransferse 1336343 1337086
NMB1319 conserved hypothetical protein 1337090 1337860
NMB1320 50S ribosomal protein L9 1338540 1338091
NMB1321 30S ribosomal protein S18 1338787 1338560
NMB1322 primosomal replication protein n, putative 1339096 1338797
NMB1323 30S ribosomal protein S6 1339465 1339100
NMB1324 thioredoxin reductase 1340571 1339624
NMB1325 cation transport ATPase, E1-E2 family 1340710 1342869
NMB1326 excinuclease ABC, subunit C 1342969 1344819
NMB1327 conserved hypothetical protein 1345045 1346445
NMB1328 conserved hypothetical protein 1346570 1347283
NMB1329 hypothetical protein 1347649 1347840
NMB1330 hypothetical protein 1348276 1347917
NMB1331 excinuclease ABC, subunit B 1350416 1348392
NMB1332 carboxy-terminal peptidase 1352229 1350748
NMB1333 conserved hypothetical protein 1354146 1352359
NMB1334 hypothetical protein 1354238 1354471
NMB1335 creA protein 1354474 1355031
NMB1336 conserved hypothetical protein 1355036 1355581
NMB1337 conserved hypothetical protein 1355577 1356029
NMB1338 isomerase, putative 1356698 1356045
NMB1339 prolyl-tRNA synthetase_ 1358473 1356764
NMB1340 hypothetical protein 1358924 1359151
NMB1341 pyruvate dehydrogenase, E1 component 1359167 1361827
NMB1342 pyruvate dehydrogenase, E2 component, dihydrolipoamide acetyltransferase FRAMESHIFT 1361979 1363583
NMB1343 hypothetical protein 1363680 1364114
NMB1344 pyruvate dehydrogenase, E3 component, lipoamide dehydrogenase 1364135 1365916
NMB1345 hypothetical protein 1367830 1366283
NMB1346 TonB-dependent receptor, putative FRAMESHIFT 1369731 1367957
NMB1347 extragenic suppressor protein SuhB 1370786 1370004
NMB1348 RNA methylase, putative 1371030 1371842
NMB1349 hypothetical protein 1371906 1372760
NMB1350 hypothetical protein 1372967 1373305
NMB1351 fmu and fmv protein, putative 1373656 1374909
NMB1352 hypothetical protein 1375272 1375703
NMB1353 aldehyde dehydrogenase family protein 1377097 1375757
NMB1354 conserved hypothetical protein 1377755 1377105
NMB1355 glutamyl-tRNA (Gln) amidotransferase subunit C, putative 1377906 1378193
NMB1356 Glu-tRNA(Gln) amidotransferase, subunit A 1378259 1379701
NMB1357 conserved hypothetical protein 1379701 1380630
NMB1358 Glu-tRNA(Gln) amidotransferase, subunit B 1380676 1382103
NMB1359 CDP-6-deoxy-delta-3,4-glucoseen reductase, putative 1382318 1383325
NMB1360 pyridoxamine 5-phosphate oxidase 1384090 1383461

## Appendix B

NMB1361 conserved hypothetical protein 1384312 1385361
NMB1362 oxalate/formate antiporter, putative 1386974 1385436
NMB1363 exodeoxyribonuclease, large subunit 1388622 1387270
NMB1364 NH(3)-dependent NAD+ synthetase NadE, putative 1388819 1389637
NMB1365 conserved hypothetical protein 1390183 1389713
NMB1366 thioredoxin 1390481 1390810
NMB1367 conserved hypothetical protein 1391930 1390869
NMB1368 ATP-dependent RNA helicase, putative 1392141 1393526
NMB1369 hypothetical protein 1394572 1394021
NMB1370 hypothetical protein 1395217 1394860
NMB1371 acetylornithine aminotransferase 1395561 1396754
NMB1372 ATP-dependent Clp protease, ATP-binding subunit ClpX 1398104 1396863
NMB1373 ribosome-binding factor A 1398295 1398663
NMB1374 tRNA pseudouridine synthase B 1398699 1399619
NMB1375 modification methylase, putative FRAMESHIFT 1399839 1401945
NMB1376 conserved hypothetical protein POINT MUTATION 1401938 1404712
NMB1377 L-lactate dehydrogenase 1406036 1404867
NMB1378 conserved hypothetical protein 1406327 1406770
NMB1379 nifS protein 1406802 1408013
NMB1380 nifU protein 1408280 1408663
NMB1381 HesB/YadR/YfhF family protein 1408693 1409070
NMB1382 conserved hypothetical protein 1409254 1409036
NMB1383 chaperone protein HscB 1409336 1409833
NMB1384 DNA gyrase subunit A 1409934 1412681
NMB1385 IS1016 family transposase, degenerate 1412841 1413241
NMB1386 transposase, putative FRAMESHIFT 1413303 1413955
NMB1387 hypothetical protein 1414840 1414292
NMB1388 glucose-6-phosphate isomerase 1416500 1414857
NMB1389 RpiR/YebK/YfhH family protein 1417469 1416624
NMB1390 glucokinase 1418505 1417522
NMB1391 oxidoreductase, Sol/DevB family 1419181 1418489
NMB1392 glucose-6-phosphate 1-dehydrogenase 1420906 1419464
NMB1393 phosphogluconate dehydratase 1421474 1423306
NMB1394 4-hydroxy-2-oxoglutarate aldolase/2-deydro-3-deoxyphosphogluconate aldolase 1423490 1424125
NMB1395 alcohol dehydrogenase, zinc-containing 1425427 1424390
NMB1396 A/G-specific adenine glycosylase 1425581 1426627
NMB1397 hypothetical protein 1426793 1426972
NMB1398 Cu-Zn-superoxide dismutase 1427047 1427604
NMB1399 IS1106 transposase 1429146 1428175
NMB1400 ABC transporter family protein 1431631 1429406
NMB1401 IS1016C2 transposase 1432983 1432447
NMB1402 hypothetical protein 1433320 1433751
NMB1403 FrpA/C-related protein 1433795 1433983
NMB1404 hypothetical protein 1434021 1434746
NMB1405 FrpA/C-related protein 1434763 1435962
NMB1406 hypothetical protein 1436396 1436755
NMB1407 FrpA-related protein, degenerate 1436755 1437881
NMB1408 hypothetical protein 1437960 1438451
NMB1409 FrpA/C-related protein 1438582 1439007
NMB1410 hypothetical protein 1439247 1439783
NMB1411 IS1016C2 transposase 1440610 1439960
NMB1412 FrpC operon protein 1441216 1442022
NMB1413 IS1016 family transposase, putative FRAMESHIFT 1442715 1442132
NMB1414 FrpC operon protein 1442798 1443568
NMB1415 iron-regulated protein FrpC 1443588 1449074
NMB1416 aminopeptidase N 1452022 1449422
NMB1417 conserved hypothetical protein 1452947 1452156
NMB1418 HtrB/MsbB family protein 1454563 1453697
NMB1419 crossover junction endodeoxyribonuclease RuvC 1455150 1454617
NMB1420 factor-for-inversion stimulation protein Fis, putative 1455392 1455156
NMB1421 nifR3 protein 1456432 1455425

## Appendix B

NMB1422 ATP-dependent RNA helicase, putative 1456798 1458168
NMB1423 conserved hypothetical protein 1458746 1459870
NMB1424 hypothetical protein 1459903 1460928
NMB1425 lysyl-tRNA synthetase, heat inducible 1462560 1461052
NMB1426 hypothetical protein 1463968 1462718
NMB1427 hypothetical protein 1464208 1464032
NMB1428 aminopeptidase, putative 1464426 1466219
NMB1429 outer membrane protein PorA 1468209 1467034
NMB1430 transcription elongation factor GreA 1470964 1470491
NMB1431 hypothetical protein 1471298 1471050
NMB1432 3-phosphoshikimate 1-carboxyvinyltransferase 1471360 1472658
NMB1433 conserved hypothetical protein FRAMESHIFT 1473237 1472707
NMB1434 cardiolipin synthetase family protein 1474971 1473448
NMB1435 drug resistance translocase family protein 1476489 1475086
NMB1436 conserved hypothetical protein 1476774 1477550
NMB1437 conserved hypothetical protein 1477550 1478248
NMB1438 conserved hypothetical protein 1478248 1479699
NMB1439 phosphoribosylaminoimidazole carboxylase, catalytic subunit 1480370 1479888
NMB1440 hypothetical protein 1481131 1480421
NMB1441 O-methyltransferase, putative 1481799 1481134
NMB1442 mismatch repair protein MutL 1482139 1484112
NMB1443 DNA polymerase III, subunits gamma and tau 1484210 1486321
NMB1444 conserved hypothetical protein 1486404 1486736
NMB1445 recA protein 1489556 1488513
NMB1446 3-dehydroquinate dehydratase 1489810 1490571
NMB1447 ATP-dependent DNA helicase Rep 1490594 1492606
NMB1448 DNA-damage-inducible protein P 1493734 1492781
NMB1449 TonB-dependent receptor POINT MUTATION 1496967 1493881
NMB1450 ferredoxin--NADP reductase 1497241 1498017
NMB1451 DNA polymerase III, epsilon subunit 1499643 1498234
NMB1452 conserved hypothetical protein 1500459 1501595
NMB1453 hypothetical protein 1502335 1501847
NMB1454 ferredoxin, 4Fe-4S bacterial type 1503891 1502398
NMB1455 hypothetical protein 1504075 1503959
NMB1456 hypothetical protein 1504347 1504153
NMB1457 transketolase 1504419 1506395
NMB1458 fumarate hydratase, class II 1506547 1507932
NMB1459 conserved hypothetical protein 1508923 1508003
NMB1460 single-strand binding protein 1509972 1509451
NMB1461 drug resistance translocase family protein 1511361 1509979
NMB1462 transglycosylase, putative 1512092 1511472
NMB1463 IS1106 transposase, degenerate 1512998 1512596
NMB1464 conserved hypothetical protein 1513541 1513053
NMB1465 opacity protein FRAMESHIFT 1515309 1514483
NMB1466 conserved hypothetical protein 1515639 1516367
NMB1467 exopolyphosphatase 1516487 1517992
NMB1468 hypothetical protein 1518527 1518207
NMB1469 hypothetical protein 1518607 1518527
NMB1470 hypothetical protein 1519392 1518850
NMB1471 tryptophanyl-tRNA synthetase 1520471 1519464
NMB1472 clpB protein 1520732 1523308
NMB1473 aminotransferase, class I 1524612 1523401
NMB1474 4-oxalocrotonate tautomerase, putative 1524910 1524704
NMB1475 conserved hypothetical protein 1525255 1526058
NMB1476 glutamate dehydrogenase, NAD-specific 1527384 1526122
NMB1477 hypothetical protein 1527562 1527396
NMB1478 phosphoglycolate phosphatase FRAMESHIFT 1527786 1528489
NMB1479 regulatory protein RecX 1528560 1529018
NMB1480 hypothetical protein 1529095 1529253
NMB1481 hypothetical protein 1529262 1529393
NMB1482 acyl CoA thioester hydrolase family protein 1529409 1529888
NMB1483 lipoprotein NlpD, putative 1531499 1530255
NMB1484 stationary-phase survival protein SurE 1532501 1531758

## Appendix B

NMB1485 conserved hypothetical protein 1534074 1532521
NMB1486 hypothetical protein 1534263 1534126
NMB1487 fimbrial assembly protein 1535230 1534445
NMB1488 succinate-semialdehyde dehydrogenase (NADP+) 1536772 1535342
NMB1489 hypothetical protein 1537259 1537750
NMB1490 hypothetical protein 1538345 1537917
NMB1491 hypothetical protein 1538785 1538699
NMB1492 hypothetical protein 1538860 1538795
NMB1493 carbon starvation protein A 1538892 1540970
NMB1494 conserved hypothetical protein 1540963 1541154
NMB1495 hypothetical protein 1541371 1541562
NMB1496 conserved hypothetical protein 1541673 1542230
NMB1497 TonB-dependent receptor 1543234 1545996
NMB1498 aspartokinase, alpha and beta subunits 1549220 1548006
NMB1499 ribonuclease PH 1550148 1549423
NMB1500 conserved hypothetical protein 1550694 1550233
NMB1501 HesA/MoeB/ThiF family protein 1550911 1551684
NMB1502 hypothetical protein 1551825 1552349
NMB1503 hypothetical protein 1552608 1552814
NMB1504 conserved hypothetical protein 1552706 1553557
NMB1505 nicotinate phosphoribosyltransferase 1553601 1554806
NMB1506 arginyl-tRNA synthetase 1554901 1556616
NMB1507 hypothetical protein 1556714 1557070
NMB1508 hypothetical protein 1557130 1558584
NMB1509 amino acid ABC transporter, permease protein 1560344 1559601
NMB1510 thermonuclease family protein 1561224 1560526
NMB1511 ribose 5-phosphate isomerase A 1561934 1561266
NMB1512 YgbB/YacN family protein 1562493 1562014
NMB1513 conserved hypothetical protein 1563214 1562528
NMB1514 DNA polymerase III, epsilon subunit 1563945 1563214
NMB1515 transporter, putative 1565411 1564104
NMB1516 fixS protein 1565589 1565404
NMB1517 hypothetical protein 1565885 1565589
NMB1518 acetate kinase 1566236 1567429
NMB1519 thiol:disulfide interchange protein DsbD 1569752 1567950
NMB1520 hypothetical protein 1570337 1569819
NMB1521 phytoene synthase-related protein 1571249 1570425
NMB1522 FKBP-type peptidyl-prolyl cis-trans isomerase SlyD 1571803 1571324
NMB1523 hypothetical protein 1572276 1572569
NMB1524 oxidoreductase, putative 1572682 1574046
NMB1525 VirG-related protein FRAMESHIFT 1576262 1574233
NMB1526 small major protein B 1577081 1576638
NMB1527 ADP-heptose--LPS heptosyltransferase II 1578146 1577139
NMB1528 methylated-DNA--protein-cysteine methyltransferase, putative 1579353 1578547
NMB1529 conserved hypothetical protein FRAMESHIFT 1579597 1580409
NMB1530 succinyl-diaminopimelate desuccinylase 1582228 1581086
NMB1531 conserved hypothetical protein 1582961 1582344
NMB1532 conserved hypothetical protein 1583504 1582998
NMB1533 H.8 outer membrane protein 1584150 1583602
NMB1534 hypothetical protein 1584287 1584150
NMB1535 hypothetical protein 1584404 1584874
NMB1536 preprotein translocase SecA subunit 1584984 1587731
NMB1537 DNA primase 1587879 1589648
NMB1538 RNA polymerase sigma factor RpoD 1589838 1591763
NMB1539 IS1106 transposase 1591913 1592917
NMB1540 lactoferrin-binding protein A 1597271 1594443
NMB1541 lactoferrin-binding protein B 1599481 1597271
NMB1542 hypothetical protein 1600504 1600722
NMB1543 conserved hypothetical protein 1600871 1602082
NMB1544 hypothetical protein 1602097 1602405
NMB1545 hypothetical protein 1602412 1602609
NMB1546 hypothetical protein 1602795 1603076
NMB1547 hypothetical protein 1603107 1603406

## Appendix B

NMB1548 tspB protein, putative 1603741 1605384
NMB1549 hypothetical protein 1606176 1606325
NMB1550 conserved hypothetical protein 1606332 1606613
NMB1551 conserved hypothetical protein 1606617 1607717
NMB1552 pilin gene inverting protein PivNM-1A 1608019 1608972
NMB1553 transposase, truncation 1612022 1611708
NMB1554 CTP synthase 1613884 1612253
NMB1555 long-chain-fatty-acid--CoA ligase 1615666 1613999
NMB1556 tRNA (5-methylaminomethyl-2-thiouridylate) -methyltransferase 1616840 1615740
NMB1557 conserved hypothetical protein 1617439 1616969
NMB1558 diacylglycerol kinase 1618115 1617735
NMB1559 glutathione synthetase 1619386 1618430
NMB1560 glutaminyl-tRNA synthetase 1621164 1619479
NMB1561 transcriptional regulator, DeoR family 1622049 1621279
NMB1562 conserved hypothetical protein 1622994 1622095
NMB1563 transcriptional regulator, GntR family 1623859 1623146
NMB1564 conserved hypothetical protein 1624850 1624431
NMB1565 hypothetical protein 1625639 1624971
NMB1566 phosphoribosylglycinamide formyltransferase 1626281 1625658
NMB1567 macrophage infectivity potentiator 1627206 1626391
NMB1568 DNA polymerase holoenzyme chi subunit, putative 1627905 1627468
NMB1569 aminopeptidase A/I, FRAMESHIFT 1629499 1627971
NMB1570 conserved hypothetical protein 1629544 1630656
NMB1571 conserved hypothetical protein 1630656 1631723
NMB1572 aconitate hydratase 2 1631936 1634518
NMB1573 ornithine carbamoyltransferase, catabolic 1634663 1635655
NMB1574 ketol-acid reductoisomerase 1636895 1635885
NMB1575 conserved hypothetical protein 1637268 1636978
NMB1576 acetolactate synthase III, small subunit 1637826 1637338
NMB1577 acetolactate synthase III, large subunit 1639564 1637840
NMB1578 conserved hypothetical protein 1640685 1641335
NMB1579 ATP phosphoribosyltransferase 1641417 1642067
NMB1580 hypothetical protein 1642174 1643070
NMB1581 histidinol dehydrogenase 1643070 1644356
NMB1582 histidinol-phosphate aminotransferase 1644405 1645499
NMB1583 imidazoleglycerol-phosphate dehydratase 1645499 1646413
NMB1584 3-hydroxyacid dehydrogenase 1646511 1647377
NMB1585 transcriptional regulator, MarR family 1647658 1648086
NMB1586 hypothetical protein 1648100 1648963
NMB1587 protease, putative 1650120 1649020
NMB1588 CDP-diacylglycerol--glycerol-3-phosphate 3-phosphatidyltransferase 1651479 1650919
NMB1589 hypothetical protein 1652036 1651797
NMB1590 conserved hypothetical protein 1652675 1652343
NMB1591 transcriptional regulator MtrA 1652804 1653706
NMB1592 hypothetical protein 1653729 1654313
NMB1593 conserved hypothetical protein 1654445 1655305
NMB1594 spermidine/putrescine ABC transporter, periplasmic spermidine/putrescine-binding protein 1656479 1655352
NMB1595 alanyl-tRNA synthetase 1656684 1659305
NMB1596 hypothetical protein 1659348 1659551
NMB1597 hypothetical protein 1659569 1659997
NMB1598 hypothetical protein 1660094 1660282
NMB1599 hypothetical protein 1660300 1660584
NMB1600 hypothetical protein 1660624 1660878
NMB1601 IS1106 transposase 1661075 1662079
NMB1602 transposase, putative 1663112 1661997
NMB1603 tellurite resistance protein, putative 1663289 1664230
NMB1604 phosphoglycerate mutase 1664989 1664309
NMB1605 topoisomerase IV subunit A 1665137 1667437
NMB1606 sensor histidine kinase 1667460 1669033
NMB1607 sigma-54 dependent response regulator 1669029 1669493
NMB1608 conserved hypothetical protein 1669600 1670349

## Appendix B

NMB1609 trans-sulfuration enzyme family protein 1672860 1671694
NMB1610 hypothetical protein 1673766 1673008
NMB1611 hypothetical protein 1673866 1674114
NMB1612 amino acid ABC transporter, periplasmic amino acid-binding protein 1674169 1674972
NMB1613 fumarate hydratase, class I 1675282 1676802
NMB1614 Trk system potassium uptake protein TrkA 1676903 1678312
NMB1615 hypothetical protein 1678758 1679018
NMB1616 phosphomethylpyrimidine kinase 1679755 1680558
NMB1617 tellurite resistance protein, putative 1681480 1680614
NMB1618 ribonuclease HI 1681594 1682028
NMB1619 conserved hypothetical protein 1682889 1683290
NMB1620 conserved hypothetical protein 1683333 1684514
NMB1621 glutathione peroxidase 1685113 1684583
NMB1622 nitric oxide reductase 1687547 1685295
NMB1623 major anaerobically induced outer membrane protein 1687918 1689087
NMB1624 conserved hypothetical protein 1689215 1689967
NMB1625 pilin gene inverting protein PivNM-1B 1691651 1690698
NMB1626 conserved hypothetical protein 1693053 1691953
NMB1627 conserved hypothetical protein 1693338 1693057
NMB1628 tspB protein, putative 1695347 1693797
NMB1629 Hypothetical protein 1695690 1695328
NMB1630 hypothetical protein 1696057 1695758
NMB1631 hypothetical protein 1696449 1696088
NMB1632 hypothetical protein 1696752 1696555
NMB1633 hypothetical protein 1697067 1696759
NMB1634 conserved hypothetical protein 1698296 1697091
NMB1635 hypothetical protein 1698662 1698444
NMB1636 opacity protein FRAMESHIFT 1700231 1701047
NMB1637 conserved hypothetical protein 1701808 1701254
NMB1638 YhbX/YhjW/YijP/YjdB family protein 1703518 1701887
NMB1639 hypothetical protein 1703921 1703595
NMB1640 phosphoserine aminotransferase 1705027 1703924
NMB1641 conserved hypothetical protein 1705374 1705820
NMB1642 N utilization substance protein A 1705851 1707350
NMB1643 translation initiation factor IF-2 1707365 1710250
NMB1644 hypothetical protein 1711755 1710418
NMB1645 hypothetical protein 1713169 1711832
NMB1646 hemolysin, putative 1713312 1713935
NMB1647 amino acid symporter, putative 1715420 1714005
NMB1648 conserved hypothetical protein 1715747 1716472
NMB1649 disulfide bond formation protein B 1717022 1716537
NMB1650 leucine-responsive regulatory protein 1718177 1717716
NMB1651 alanine racemase 1718502 1719557
NMB1652 conserved hypothetical protein 1720979 1719627
NMB1653 conserved hypothetical protein 1721266 1720997
NMB1654 conserved hypothetical protein 1722129 1721395
NMB1655 adenine specific methylase, putative 1723321 1722413
NMB1656 hypothetical protein 1723454 1724044
NMB1657 comE operon protein 1-related protein 1725327 1724713
NMB1658 DNA/pantothenate metabolism flavoprotein 1731065 1732246
NMB1659 guanosine-3`,5`-bis(diphosphate) 3`-pyrophosphohydrolase 1734472 1732319
NMB1660 DNA-directed RNA polymerase, omega subunit 1734770 1734567
NMB1661 guanylate kinase 1735446 1734832
NMB1662 adenine phosphoribosyltransferase 1735607 1736170
NMB1663 conserved hypothetical protein 1737007 1736222
NMB1664 protease, putative 1737332 1738684
NMB1665 conserved hypothetical protein 1739253 1738870
NMB1666 hypothetical protein 1739498 1739253
NMB1667 hypothetical protein 1740061 1739858
NMB1668 hemoglobin receptor 1742596 1740224
NMB1669 iron-starvation protein PigA 1743420 1742794
NMB1670 PqiA family protein 1743706 1745214

## Appendix B

NMB1671 pqiB protein 1745210 1746868
NMB1672 conserved hypothetical protein 1746871 1747386
NMB1673 DNA-3-methyladenine glycosylase I, putative 1747393 1747941
NMB1674 GDSL lipase family protein 1747934 1748572
NMB1675 hypothetical protein 1748797 1749102
NMB1676 glycine dehydrogenase (decarboxylating) 1749136 1751984
NMB1677 cytochrome c5 1753288 1752452
NMB1678 aromatic-amino-acid aminotransferase 1754906 1753716
NMB1679 tRNA (uracil-5-)-methyltransferase 1756015 1754930
NMB1680 chorismate synthase 1756162 1757259
NMB1681 hypothetical protein 1757354 1757776
NMB1682 topoisomerase IV subunit B 1759838 1757856
NMB1683 MutT/nudix family protein 1760429 1759908
NMB1684 seryl-tRNA synthetase 1760595 1761887
NMB1685 D-lactate dehydrogenase 1762966 1761971
NMB1686 peptide chain release factor 1 1764167 1763094
NMB1687 conserved hypothetical protein 1765042 1764275
NMB1688 L-asparaginase I 1766051 1765053
NMB1689 dedA protein, putative 1767007 1766327
NMB1690 phosphoglucomutase/phosphomannomutase family protein 1768532 1767201
NMB1691 dihydropteroate synthase 1769519 1768665
NMB1692 chorismate mutase-related protein 1770552 1769662
NMB1693 hypothetical protein 1770643 1772754
NMB1694 conserved hypothetical protein 1774305 1772824
NMB1695 hypothetical protein 1774424 1775401
NMB1696 acyl carrier protein 1775800 1775558
NMB1697 acyl carrier protein, putative 1776072 1775815
NMB1698 acyltransferase, putative 1776827 1776072
NMB1699 hypothetical protein 1777185 1776823
NMB1700 hypothetical protein 1777345 1777707
NMB1701 hypothetical protein 1777763 1778260
NMB1702 3-oxoacyl-(acyl-carrier-protein) reductase 1778291 1779016
NMB1703 3-oxoacyl-(acyl-carrier-protein) synthase II 1779013 1780260
NMB1704 beta-1,4-glucosyltransferase 1780467 1781222
NMB1705 alpha-1,2-N-acetylglucosamine transferase 1781226 1782287
NMB1706 hypothetical protein 1782329 1782496
NMB1707 sodium- and chloride-dependent transporter 1782677 1784011
NMB1708 NosX-related protein 1784846 1784189
NMB1709 thymidylate synthase 1785648 1784857
NMB1710 glutamate dehydrogenase, NADP-specific 1786032 1787363
NMB1711 transcriptional regulator, GntR family 1788280 1787504
NMB1712 L-lactate permease-related protein 1788711 1789007
NMB1713 transposase, IS30 family 1790361 1789399
NMB1714 multidrug efflux pump channel protein 1791874 1790474
NMB1715 multiple transferable resistance system protein MtrD 1795132 1791932
NMB1716 membrane fusion protein 1796382 1795147
NMB1717 trancscriptional regulator MtrR 1796785 1797414
NMB1718 hypothetical protein 1797953 1797699
NMB1719 efflux pump component MtrF 1798240 1799805
NMB1720 exodeoxyribonuclease V 125 kD polypeptide 1803085 1799879
NMB1721 conserved hypothetical protein 1804596 1803190
NMB1722 cytochrome C555 FRAMESHIFT 1804923 1804801
NMB1723 cytochrome c oxidase, subunit III 1806129 1805035
NMB1724 cytochrome c oxidase, subunit II 1806939 1806331
NMB1725 cytochrome c oxidase, subunit I 1808411 1806969
NMB1726 conserved hypothetical protein 1808726 1810471
NMB1727 conserved hypothetical protein 1810539 1810964
NMB1728 biopolymer transport protein ExbD 1812088 1811657
NMB1729 biopolymer transport protein ExbB 1812753 1812094
NMB1730 TonB protein 1813661 1812822
NMB1731 conserved hypothetical protein 1813916 1814551
NMB1732 transporter, putative 1815806 1815009

## Appendix B

NMB1733 hypothetical protein 1816445 1815945
NMB1734 glutaredoxin 1817423 1816785
NMB1735 GTP pyrophosphokinase 1817566 1819776
NMB1736 transposase, putative FRAMESHIFT 1820048 1820856
NMB1737 secretion protein, putative 1822426 1821026
NMB1738 secretion protein, putative 1823922 1822498
NMB1739 hypothetical protein 1824158 1824508
NMB1740 hypothetical protein 1824635 1825042
NMB1741 conserved hypothetical protein FRAMESHIFT 1825116 1826455
NMB1742 hypothetical protein 1826503 1826790
NMB1743 hypothetical protein 1826798 1826992
NMB1744 hypothetical protein 1827003 1827284
NMB1745 hypothetical protein 1827294 1827569
NMB1746 hypothetical protein 1827700 1827987
NMB1747 tspB protein, putative 1828031 1829533
NMB1748 conserved hypothetical protein 1829537 1829824
NMB1749 conserved hypothetical protein 1829837 1830919
NMB1750 pilin gene inverting protein PivNM-2 1831548 1832495
NMB1751 transposase, degenerate 1833264 1832887
NMB1752 conserved hypothetical protein FRAMESHIFT 1833772 1833299
NMB1753 VapD-related protein 1834647 1835081
NMB1754 cryptic plasmid protein A-related protein 1835182 1835084
NMB1755 hypothetical protein 1835328 1835669
NMB1756 hypothetical protein 1835980 1836171
NMB1757 hypothetical protein 1836529 1836756
NMB1758 hypothetical protein 1837008 1837217
NMB1759 conserved hypothetical protein 1837403 1838764
NMB1760 conserved hypothetical protein 1839128 1839631
NMB1761 conserved hypothetical protein 1839797 1841047
NMB1762 hemolysin activation protein HecB, putative 1843162 1841378
NMB1763 toxin-activating protein, putative 1843675 1843220
NMB1764 hypothetical protein 1844155 1843844
NMB1765 hypothetical protein 1844466 1844170
NMB1766 hypothetical protein 1845460 1844450
NMB1767 hypothetical protein 1845945 1845532
NMB1768 hemagglutinin/hemolysin-related protein 1853493 1845952
NMB1769 IS1016 family transposase, putative truncation 1853631 1853822
NMB1770 transposase, IS30 family 1854072 1855034
NMB1771 hypothetical protein 1855539 1855108
NMB1772 hypothetical protein 1857374 1855539
NMB1773 hypothetical protein 1857783 1857412
NMB1774 hypothetical protein 1858438 1858064
NMB1775 hypothetical protein 1860252 1858450
NMB1776 hypothetical protein 1860353 1860252
NMB1777 hypothetical protein 1861364 1861122
NMB1778 hypothetical protein 1861489 1861388
NMB1779 hemagglutinin/hemolysin-related protein 1867499 1861515
NMB1780 hemolysin activation protein HecB, putative 1869350 1867611
NMB1781 hypothetical protein 1869919 1869752
NMB1782 hypothetical protein 1870236 1869937
NMB1783 secretion protein, putative FRAMESHIFT 1871826 1870605
NMB1784 hypothetical protein 1872240 1871890
NMB1785 hypothetical protein 1872472 1872236
NMB1786 hypothetical protein 1873623 1872472
NMB1787 N-acetyl-gamma-glutamyl-phosphate reductase 1874156 1875196
NMB1788 ATP-dependent DNA helicase RecG 1878304 1876265
NMB1789 protein-export protein SecB 1878833 1878393
NMB1790 glutaredoxin 3 1879111 1878857
NMB1791 cytoplasmic axial filament protein FRAMESHIFT 1879236 1880813
NMB1792 sensor histidine kinase 1881795 1880854
NMB1793 response regulator, putative FRAMESHIFT 1882272 1881854
NMB1794 citrate transporter 1883808 1882498
NMB1795 hypothetical protein 1884071 1883916
NMB1796 conserved hypothetical protein 1884950 1884381

## Appendix B

NMB1797 penicillin-binding protein 3 1885109 1886515
NMB1798 IS1016 family transposase, putative FRAMESHIFT 1887236 1886597
NMB1799 S-adenosylmethionine synthetase 1888654 1887488
NMB1800 hypothetical protein 1888703 1888903
NMB1801 HtrB/MsbB family protein 1889000 1889893
NMB1802 O-sialoglycoprotein endopeptidase 1891004 1889943
NMB1803 cytochrome c-type biogenesis protein, putative 1892308 1891124
NMB1804 cytochrome c-type biogenesis protein, putative 1894316 1892304
NMB1805 cytochrome c4 1895153 1894533
NMB1806 conserved hypothetical protein 1895353 1895985
NMB1807 penicillin-binding protein 1 1898505 1896112
NMB1808 pilM protein 1898657 1899769
NMB1809 pilN protein FRAMESHIFT 1899775 1900371
NMB1810 pilO protein 1900375 1901019
NMB1811 pilP protein 1901040 1901582
NMB1812 pilQ protein FRAMESHIFT 1901604 1903908
NMB1813 shikimate kinase 1904813 1905322
NMB1814 3-dehydroquinate synthase 1905405 1906481
NMB1815 conserved hypothetical protein 1907451 1908290
NMB1816 conserved hypothetical protein 1908323 1908784
NMB1817 riboflavin-specific deaminase 1908819 1909925
NMB1818 lipopolysaccharide biosynthesis protein, putative 1910123 1911541
NMB1819 hypothetical protein 1911541 1911693
NMB1820 pilin glycosylation protein PglB 1911712 1912950
NMB1821 pilin glycosylation protein PglC 1913086 1914258
NMB1822 pilin glycosylation protein PglD 1914309 1916216
NMB1823 valine--pyruvate aminotransferase 1916275 1917564
NMB1824 conserved hypothetical protein 1918455 1917622
NMB1825 hypothetical protein 1919103 1918903
NMB1826 conserved hypothetical protein 1919452 1919084
NMB1827 DNA polymerase III, alpha subunit 1919852 1923283
NMB1828 conserved hypothetical protein 1924652 1923723
NMB1829 TonB-dependent receptor 1926848 1924725
NMB1830 phosphoglycolate phosphatase, putative 1926996 1927652
NMB1831 lytB protein 1928711 1927746
NMB1832 lipoprotein signal peptidase 1929267 1928743
NMB1833 isoleucyl-tRNA synthetase 1933332 1930546
NMB1834 riboflavin kinase/FMN adenylyltransferase 1934394 1933477
NMB1835 tyrosyl-tRNA synthetase 1936217 1934925
NMB1836 lipopolysaccharide biosynthesis protein WbpC, putative 1938151 1936283
NMB1837 hypothetical protein 1938466 1938215
NMB1838 GTP-binding protein, putative 1939615 1938527
NMB1839 formate--tetrahydrofolate ligase 1941406 1939733
NMB1840 conserved hypothetical protein 1941581 1942009
NMB1841 mannose-1-phosphate guanyltransferase-related protein 1942741 1942049
NMB1842 4-hydroxyphenylacetate 3-hydroxylase, small subunit, putative 1943257 1942760
NMB1843 transcriptional regulator, MarR family 1943812 1943375
NMB1844 hypothetical protein 1943938 1943819
NMB1845 thioredoxin 1944662 1944156
NMB1846 Mrp/NBP35 family protein 1945032 1946108
NMB1847 pilC1 protein FRAMESHIFT 1947287 1950374
NMB1848 hypothetical protein 1952279 1951938
NMB1849 carbamoyl-phosphate synthase, small subunit 1952589 1953719
NMB1850 hypothetical protein 1954091 1954363
NMB1851 hypothetical protein 1954440 1954697
NMB1852 conserved hypothetical protein 1954697 1955083
NMB1853 hypothetical protein 1955422 1955691
NMB1854 hypothetical protein 1955768 1956406
NMB1855 carbamoyl-phosphate synthase, large subunit 1956438 1959650
NMB1856 transcriptional regulator, LysR family 1960777 1959881
NMB1857 modulator of drug activity B 1961016 1961591

## Appendix B

NMB1858 hypothetical protein 1961977 1961594
NMB1859 S-adenosylmethionine:tRNA ribosyltransferase-isomerase 1963108 1962071
NMB1860 acetyl-CoA carboxylase, biotin carboxyl carrier protein 1963464 1963916
NMB1861 acetyl-CoA carboxylase, biotin carboxylase 1964031 1965389
NMB1862 ribosomal protein L11 methyltransferase 1965653 1966537
NMB1863 oligoribonuclease 1966558 1967118
NMB1864 glutamate-1-semialdehyde 2,1-aminomutase 1968808 1967528
NMB1865 hypothetical protein 1968821 1969036
NMB1866 conserved hypothetical protein 1969593 1970918
NMB1867 1-deoxyxylulose-5-phosphate synthase 1972919 1971009
NMB1868 integrase/recombinase XerC 1973909 1973007
NMB1869 fructose-bisphosphate aldolase 1974093 1975154
NMB1870 hypothetical protein 1975177 1976136
NMB1871 conserved hypothetical protein 1976286 1976960
NMB1872 ribosomal-protein-alanine acetyltransferase, putative 1976960 1977397
NMB1873 DNA polymerase, bacteriophage-type, putative 1977394 1978128
NMB1874 orotate phosphoribosyltransferase 1978193 1978831
NMB1875 hypothetical protein 1978908 1979339
NMB1876 N-acetylglutamate synthase 1979339 1980646
NMB1877 prolyl oligopeptidase family protein 1980850 1982862
NMB1878 transcriptional regulator, AraC family 1983567 1982983
NMB1879 hypothetical protein 1983936 1983628
NMB1880 ABC transporter, periplasmic solute-binding protein, putative 1984172 1985134
NMB1881 conserved hypothetical protein 1985694 1986014
NMB1882 TonB-dependent receptor 1986131 1988305
NMB1883 hypothetical protein 1988727 1988440
NMB1884 conserved hypothetical protein 1989047 1988727
NMB1885 protein-L-isoaspartate O-methyltransferase 1989783 1989130
NMB1886 conserved hypothetical protein 1990389 1989889
NMB1887 triosephosphate isomerase 1990568 1991338
NMB1888 protein-export membrane protein SecG 1991348 1991695
NMB1889 hypothetical protein 1992486 1992575
NMB1890 conserved hypothetical protein 1992709 1993074
NMB1891 helix-turn-helix family protein 1993074 1993382
NMB1892 hypothetical protein 1993495 1993704
NMB1893 conserved hypothetical protein FRAMESHIFT 1994615 1993771
NMB1894 leucyl-tRNA synthetase, truncation 1994851 1994723
NMB1895 DNA adenine methylase, truncation 1994987 1994847
NMB1896 type II restriction enzyme DpnI 1995774 1994974
NMB1897 leucyl-tRNA synthetase 1998538 1995911
NMB1898 lipoprotein 1998808 1999320
NMB1899 hypothetical protein 1999330 1999770
NMB1900 polyphosphate kinase 1999849 2001996
NMB1901 IS1016C2 transposase, degenerate 2002232 2002770
NMB1902 DNA polymerase III, beta subunit 2004113 2003013
NMB1903 chromosomal replication initiator protein DnaA 2005904 2004351
NMB1904 ribosomal protein L34 2006196 2006327
NMB1905 ribonuclease P protein component 2006333 2006695
NMB1906 conserved hypothetical protein 2006763 2006981
NMB1907 60 kd inner-membrane protein 2007156 2008790
NMB1908 conserved hypothetical protein 2009599 2008877
NMB1909 Maf/YceF/YhdE family protein 2010236 2009649
NMB1910 conserved hypothetical protein 2010384 2010884
NMB1911 50S ribosomal protein L32 2010921 2011097
NMB1912 conserved hypothetical protein 2011275 2011799
NMB1913 fatty acid/phospholipid synthesis protein 2011891 2012943
NMB1914 hypothetical protein 2013082 2013330
NMB1915 hypothetical protein 2013360 2013746
NMB1916 3-oxoacyl-(acyl-carrier-protein) synthase III 2013931 2014890
NMB1917 conserved hypothetical protein 2014940 2015344

Appendix B

NMB1918 malonyl CoA-acyl carrier protein transacylase 2015441 2016364
NMB1919 ABC transporter, ATP-binding protein 2016505 2018367
NMB1920 GMP synthase 2018470 2020032
NMB1921 3-oxoacyl-(acyl-carrier-protein) reductase 2020097 2020840
NMB1922 IS1106 transposase, degenerate 2021273 2021118
NMB1923 conserved hypothetical protein 2021377 2021757
NMB1924 inositol monophosphatase family protein 2022673 2021981
NMB1925 conserved hypothetical protein 2022876 2023598
NMB1926 lacto-N-neotetraose biosynthesis glycosyl transferase LgtE 2025680 2024841
NMB1927 lacto-N-neotetraose biosynthesis glycosyl transferase-related protein 2025817 2025725
NMB1928 lacto-N-neotetraose biosynthesis glycosyl transferase LgtB 2026656 2025832
NMB1929 lacto-N-neotetraose biosynthesis glycosyl transferase LgtA 2027747 2026701
NMB1930 glycyl-tRNA synthetase, beta chain 2029827 2027767
NMB1931 hypothetical protein 2030256 2029912
NMB1932 glycyl-tRNA synthetase, alpha chain 2031238 2030336
NMB1933 ATP synthase F1, epsilon subunit 2032065 2031646
NMB1934 ATP synthase F1, beta subunit 2033473 2032079
NMB1935 ATP synthase F1, gamma subunit 2034386 2033514
NMB1936 ATP synthase F1, alpha subunit 2035958 2034414
NMB1937 ATP synthase F1, delta subunit 2036502 2035972
NMB1938 ATP synthase F0, B subunit 2036977 2036510
NMB1939 ATP synthase F0, C subunit 2037284 2037051
NMB1940 ATP synthase F0, A subunit 2038207 2037344
NMB1941 hypothetical protein 2038550 2038200
NMB1942 hypothetical protein 2038997 2038707
NMB1943 hypothetical protein 2039340 2039170
NMB1944 ParB family protein 2040252 2039395
NMB1945 3-octaprenyl-4-hydroxybenzoate carboxy-lyase 2040407 2040976
NMB1946 outer membrane lipoprotein 2041904 2041044
NMB1947 ABC transporter, permease protein 2042749 2042066
NMB1948 ABC transporter, ATP-binding protein 2043488 2042754
NMB1949 soluble lytic murein transglycosylase, putative 2044018 2045865
NMB1950 30S ribosomal protein S21 2046157 2046366
NMB1951 conserved hypothetical protein 2046405 2046944
NMB1952 stringent starvation protein B 2047538 2047149
NMB1953 stringent starvation protein A 2048215 2047613
NMB1954 hypothetical protein 2050146 2048488
NMB1955 cadmium resistance protein 2050933 2050310
NMB1956 50S ribosomal protein L31 2051451 2051239
NMB1957 acetyltransferase-related protein FRAMESHIFT 2051688 2052197
NMB1958 thioredoxin, putative 2052770 2052273
NMB1959 conserved hypothetical protein 2053150 2052770
NMB1960 hypothetical protein 2053632 2053153
NMB1961 VacJ-related protein 2054464 2053640
NMB1962 hypothetical protein 2054739 2054464
NMB1963 conserved hypothetical protein 2055380 2054793
NMB1964 conserved hypothetical protein 2055911 2055420
NMB1965 conserved hypothetical protein 2056738 2055965
NMB1966 ABC transporter, ATP-binding protein 2057586 2056789
NMB1967 transcriptional regulator, AraC family 2057759 2058673
NMB1968 aldehyde dehydrogenase A 2058936 2060375
NMB1969 serotype-1-specific antigen, putative 2061412 2064657
NMB1970 para-aminobenzoate synthetase component I/4-amino-4-deoxychorismate lyase, putative 2065692 2067470
NMB1971 conserved hypothetical protein 2069049 2067535
NMB1972 chaperonin, 60 kDa 2071379 2069748
NMB1973 chaperonin, 10 kDa 2071762 2071475
NMB1974 IS1016C2 transposase, degenerate 2071990 2072639
NMB1975 sodium- and chloride-dependent transporter 2072855 2074387
NMB1976 diaminopimelate decarboxylase 2075759 2074518

## Appendix B

NMB1977 hypothetical protein 2075940 2075773
NMB1978 cyaY protein 2076011 2076331
NMB1979 conserved hypothetical protein 2076361 2077374
NMB1980 conserved hypothetical protein 2077403 2077819
NMB1981 conserved hypothetical protein 2077844 2078347
NMB1982 DNA polymerase I 2078496 2081309
NMB1983 hypothetical protein 2082658 2083326
NMB1984 IS1106 transposase FRAMESHIFT 2083391 2084499
NMB1985 adhesion and penetration protein 2089191 2084821
NMB1986 hypothetical protein 2089756 2089328
NMB1987 thiophene and furan oxidation protein ThdF 2090041 2091384
NMB1988 iron-regulated outer membrane protein FrpB 2092611 2094752
NMB1989 iron(III) ABC transporter, periplasmic binding protein 2095472 2096434
NMB1990 iron(III) ABC transporter, permease protein 2096601 2097566
NMB1991 iron(III) ABC transporter, permease protein 2097559 2098530
NMB1992 hypothetical protein 2098577 2099200
NMB1993 iron(III) ABC transporter, ATP-binding protein 2099286 2100041
NMB1994 adhesin/invasin, putative 2100342 2101433
NMB1995 nitrogen regulatory protein P-II, FRAMESHIFT 2101839 2101423
NMB1996 phosphoribosylformylglycinamidine synthase 2101990 2105949
NMB1997 hydroxyacylglutathione hydrolase 2106047 2106802
NMB1998 serine-type peptidase 2107119 2111411
NMB1999 magnesium transporter 2111646 2113097
NMB2000 conserved hypothetical protein 2114094 2113189
NMB2001 conserved hypothetical protein 2114339 2115091
NMB2002 hypothetical protein 2115113 2115328
NMB2003 conserved hypothetical protein 2115476 2115820
NMB2004 conserved hypothetical protein 2115820 2116509
NMB2005 glutamate N-acetyltransferase/amino-acid acetyltransferase 2116579 2117796
NMB2006 chloride channel protein-related protein 2117859 2119265
NMB2007 ATP-dependent RNA helicase HrpA, truncation 2119458 2120846
NMB2008 ABC transporter, ATP-binding protein-related protein 2120993 2122633
NMB2009 ATP-dependent RNA helicase HrpA, degenerate 2122680 2122859
NMB2010 YhbX/YhjW/YijP/YjdB family protein 2123074 2124648
NMB2011 ATP-dependent RNA helicase HrpA, truncation 2124717 2128133
NMB2012 transcriptional regulator, HTH_3 family 2129260 2128172
NMB2013 hypothetical protein 2129920 2129279
NMB2014 hypothetical protein 2130249 2130004
NMB2015 hypothetical protein 2130614 2130880
NMB2016 type IV pilin-related protein 2131493 2131047
NMB2017 ComEA-related protein 2132027 2131584
NMB2018 conserved hypothetical protein 2138411 2137752
NMB2019 lipopolysaccharide core biosynthesis protein KdtB 2138949 2138440
NMB2020 conserved hypothetical protein 2139756 2139076
NMB2021 conserved hypothetical protein 2140179 2139916
NMB2022 conserved hypothetical protein 2140722 2140255
NMB2023 conserved hypothetical protein 2141162 2140779
NMB2024 conserved hypothetical protein 2141826 2141224
NMB2025 conserved hypothetical protein 2142422 2141826
NMB2026 ABC transporter, permease protein 2144046 2142454
NMB2027 gluconate permease 2144385 2145767
NMB2028 thermoresistant gluconokinase 2145790 2146305
NMB2029 homoserine kinase FRAMESHIFT 2147564 2146650
NMB2030 3-demethylubiquinone-9 3-methyltransferase 2148329 2147604
NMB2031 tryptophan transporter 2148481 2149719
NMB2032 lipopolysaccharide glycosyl transferase, FRAMESHIFT 2149872 2150922
NMB2033 histidinol-phosphatase, putative 2151173 2151733
NMB2034 1-acyl-sn-glycerol-3-phosphate acyltransferase, putative 2151765 2152505
NMB2035 conserved hypothetical protein 2152505 2153194

## Appendix B

NMB2036 tRNA pseudouridine synthase A 2154495 2155390
NMB2037 hypothetical protein 2155415 2155651
NMB2038 PemK-related protein 2155642 2155962
NMB2039 major outer membrane protein PIB 2157487 2158479
NMB2040 thiamine biosynthesis protein ThiC 2161479 2159581
NMB2041 thiamin pyrophosphokinase-related protein 2162093 2162965
NMB2042 spermidine/putrescine ABC transporter, ATP-binding protein 2162977 2163912
NMB2043 IS1106 transposase, putative POINT MUTATION 2165702 2164734
NMB2044 phosphoenolpyruvate-protein phosphotransferase 2168278 2166506
NMB2045 phosphocarrier protein HPr 2168547 2168281
NMB2046 PTS system, IIAB component 2169074 2168619
NMB2047 hypoxanthine-guanine phosphoribosyltransferase, putative 2169697 2169137
NMB2048 DNA ligase 2170590 2169769
NMB2049 glyoxalase II family protein 2170682 2171311
NMB2050 conserved hypothetical protein 2173305 2171524
NMB2051 ubiquinol--cytochrome c reductase, cytochrome c1 2174444 2173647
NMB2052 ubiquinol--cytochrome c reductase, cytochrome b 2175793 2174447
NMB2053 ubiquinol--cytochrome c reductase, iron-sulfur subunit 2176393 2175815
NMB2054 conserved hypothetical protein 2177265 2176519
NMB2055 transcriptional regulator, LysR family 2177396 2178322
NMB2056 30S ribosomal protein S9 2178972 2178583
NMB2057 50S ribosomal protein L13 2179413 2178985
NMB2058 conserved hypothetical protein 2180081 2179779
NMB2059 hypothetical protein 2180421 2180095
NMB2060 glycerol-3-phosphate dehydrogenase (NAD+) 2181465 2180479
NMB2061 phosphoenolpyruvate carboxylase 2184290 2181591
NMB2062 thiF protein 2184460 2185227
NMB2063 slyX protein, putative 2186018 2185797
NMB2064 conserved hypothetical protein 2187407 2186022
NMB2065 hemK protein FRAMESHIFT 2188764 2187496
NMB2066 tldD protein 2190271 2188832
NMB2067 conserved hypothetical protein 2190661 2191881
NMB2068 D-amino acid oxidase flavoprotein, putative 2191881 2192978
NMB2069 thiamin-phosphate pyrophosphorylase 2193003 2193617
NMB2070 hypothetical protein 2194042 2194233
NMB2071 thiG protein 2194450 2195235
NMB2072 hypothetical protein 2195352 2195492
NMB2073 hypothetical protein 2195580 2195780
NMB2074 hypothetical protein 2196867 2196004
NMB2075 BirA protein/Bvg accessory factor 2198657 2196882
NMB2076 aut protein 2199160 2198657
NMB2077 methylenetetrahydrofolate dehydrogenase/methenyltetrahydrofolate cyclohydrolase FRAMESHIFT 2199800 2200650
NMB2078 conserved hypothetical protein 2201296 2200718
NMB2079 aspartate-semialdehyde dehydrogenase 2201472 2202584
NMB2080 hypothetical protein 2203345 2202818
NMB2081 hypothetical protein 2203700 2203359
NMB2082 exodeoxyribonuclease 2204466 2203690
NMB2083 cysteinyl-tRNA synthetase 2205970 2204552
NMB2084 hypothetical protein 2206648 2205985
NMB2085 hypothetical protein 2207707 2206661
NMB2086 GTP-binding protein 2208944 2207793
NMB2087 hypothetical protein 2209792 2209433
NMB2088 conserved hypothetical protein 2210766 2209894
NMB2089 conserved hypothetical protein 2210812 2211156
NMB2090 phosphoheptose isomerase 2211164 2211754
NMB2091 hemolysin, putative 2211821 2212426
NMB2092 hypothetical protein 2212437 2213066
NMB2093 methionine aminopeptidase 2213109 2213885
NMB2094 hypothetical protein 2214043 2214339
NMB2095 adhesin complex protein, putative 2214580 2214951

## Appendix B

NMB2096 malate:quinone oxidoreductase 2216608 2215145
NMB2097 hypothetical protein 2216749 2216663
NMB2098 conserved hypothetical protein 2217735 2217148
NMB2099 conserved hypothetical protein 2218377 2217799
NMB2100 hypothetical protein 2218455 2218685
NMB2101 30S ribosomal protein S2 2218861 2219586
NMB2102 elongation factor TS (EF-TS) 2219718 2220569
NMB2103 uridylate kinase 2220789 2221505
NMB2104 mafA protein FRAMESHIFT 2221692 2222652
NMB2105 mafB protein 2222695 2224143
NMB2106 hypothetical protein 2224143 2224496
NMB2107 MafB-related protein 2224527 2225288
NMB2108 hypothetical protein 2225301 2225504
NMB2109 hypothetical protein 2225639 2225887
NMB2110 hypothetical protein 2225887 2226255
NMB2111 MafB-related protein 2226268 2227110
NMB2112 hypothetical protein 2227306 2227572
NMB2113 hypothetical protein 2227598 2227897
NMB2114 MafB-related protein 2227948 2228583
NMB2115 hypothetical protein 2228589 2228930
NMB2116 hypothetical protein 2228971 2229312
NMB2117 MafB-related protein, degenerate 2229645 2230340
NMB2118 hypothetical protein 2230340 2230654
NMB2119 MafB-related protein 2230709 2231464
NMB2120 hypothetical protein 2231471 2231869
NMB2121 hypothetical protein 2232031 2232372
NMB2122 MafB-related protein 2232409 2232510
NMB2123 hypothetical protein 2232518 2232871
NMB2124 hypothetical protein 2232922 2233047
NMB2125 hypothetical protein 2233047 2233418
NMB2126 IS1016 family transposase, putative FRAMESHIFT 2234296 2233462
NMB2127 protease, putative 2235364 2234381
NMB2128 CinA-related protein 2236204 2235407
NMB2129 argininosuccinate synthase 2236517 2237857
NMB2130 hypothetical protein 2237908 2238147
NMB2131 hypothetical protein 2238143 2238355
NMB2132 transferrin-binding protein-related protein 2239900 2238437
NMB2133 sodium/dicarboxylate symporter family protein 2241384 2240158
NMB2134 conserved hypothetical protein 2241857 2243761
NMB2135 conserved hypothetical protein 2243771 2247985
NMB2136 peptide transporter 2249471 2250925
NMB2137 hypothetical protein 2251451 2251660
NMB2138 peptide chain release factor 2 2252924 2251824
NMB2139 conserved hypothetical protein 2253920 2253030
NMB2140 conserved hypothetical protein 2254265 2254711
NMB2141 hypothetical protein 2254787 2255092
NMB2142 conserved hypothetical protein 2255187 2256050
NMB2143 conserved hypothetical protein 2256043 2256786
NMB2144 sigma factor, putative 2256811 2257395
NMB2145 hypothetical protein 2257404 2257580
NMB2146 hypothetical protein 2257703 2257810
NMB2147 hypothetical protein 2257842 2258261
NMB2148 transposase, IS30 family 2258738 2259700
NMB2149 hypothetical protein 2260052 2259795
NMB2150 conserved hypothetical protein 2261006 2260440
NMB2151 phosphoribosylamine--glycine ligase 2262344 2261076
NMB2152 hypothetical protein 2262502 2262816
NMB2153 conserved hypothetical protein 2263482 2262874
NMB2154 electron transfer flavoprotein, alpha subunit 2264480 2263548
NMB2155 electron transfer flavoprotein, beta subunit 2265240 2264494
NMB2156 heptosyltransferase I 2266435 2265470
NMB2157 pyrazinamidase/nicotinamidase PncA, putative 2267107 2266475
NMB2158 conserved hypothetical protein 2267221 2267898
NMB2159 glyceraldehyde 3-phosphate dehydrogenase 2269163 2268162

## Appendix B

NMB2160 DNA mismatch repair protein MutS 2269607 2272198
NMB0505 hypothetical protein 533467 533186
NMB1123 hypothetical protein 1135584 1135390
NMB1124 hypothetical protein 1136271 1135627
NMB1125 hypothetical protein 1136639 1136271
NMB1126 hypothetical protein 1137317 1136649
NMB1127 oxidoreductase, short chain dehydrogenase/reductase family 1138201 1137485
NMB1129 hypothetical protein 1139833 1139630
NMB1130 phytoene synthase, putative 1140867 1139998
NMB1133 conserved hypothetical protein / ankyrin-related protein 1144428 1143670
NMB1134 ferredoxin, 2Fe-2S type 1144824 1144486
NMB1135 hypothetical protein 1145242 1145102
NMB1137 conserved hypothetical protein 1146211 1146017
NMB1138 conserved hypothetical protein 1146683 1146285
NMB1141 RNA methyltransferase, TrmH family 1150088 1149480
NMB1142 hypothetical protein 1150375 1150142
NMB1143 hypothetical protein 1150909 1150547
NMB1144 hypothetical protein 1151226 1150924, lipoprotein
NMB1147 hypothetical protein 1154639 1154007, homology to plasmid proteins Y4SH_RISHN and PXO2 BACAN
NMB1149 hypothetical protein 1155016 1154876
NMB1151 sulfite reductase hemoprotein, beta-component 1159086 1157320
NMB1152 sulfite reductase (NADPH) flavoprotein, alpha component 1160927 1159116
NMB1154 sulfate adenylyltransferase, subunit 2 1163172 1162252
NMB1156 siroheme synthase 1165412 1163964
NMB1157 hypothetical protein 1165696 1165541
NMB1159 conserved hypothetical protein 1167316 1166429, inner membrane
NMB1160 conserved hypothetical protein 1167316 1166429
NMB1166 conserved hypothetical protein 1171633 1170323
NMB1169 chaperone protein HscA 1174933 1173074
NMB1170 hypothetical protein 1175666 1175013
NMB1174 hypothetical protein 1178053 1177373
NMB1177 acetyl-CoA carboxylase, carboxyl transferase alpha subunit 1179887 1178931
NMB1178 mesJ protein FRAMESHIFT 1181265 1179984
NMB1183 UDP-N-acetylmuramate:L-alanyl-gamma-D-glutamyl-meso-diaminopimelate ligase 1184700 1183327
NMB1184 biotin synthetase 1185959 1184910
NMB1186 hypothetical protein 1186881 1186729
NMB1188 dihydroxy-acid dehydratase 1189180 1187324
NMB1191 sulfate adenylyltransferase, subunit 1 1194246 1192963
NMB1193 phosphoadenosine phosphosulfate reductase 1195986 1195249
NMB1196 nickel-dependent hydrogenase, b-type cytochrome subunit 1198401 1197748

## Claims

1. A method for identifying an amino acid sequence, comprising the step of searching for putative open reading frames or protein-coding sequences within one or more of *N. meningitidis* nucleotide sequences selected from the group consisting of SEQ ID NO 1 and the NMB open reading frames.

2. A method according to claim 1, comprising the steps of searching a *N. meningitidis* nucleotide sequence for an initiation codon and searching the upstream sequence for an in-frame termination codon.

3. A method for producing a protein, comprising the step of expressing a protein comprising an amino acid sequence identified according to any one of claims 1-2.

4. A method for identifying a protein in *N. mengitidis,* comprising the steps of producing a protein according to claim 3, producing an antibody which binds to the protein, and determining whether the antibody recognises a protein produced by *N. menigitidis*.

5. Nucleic acid comprising an open reading frame or protein-coding sequence identified by a method according to any one of claims 1-2.

6. A protein obtained by the method of claim 3.

7. Nucleic acid comprising one or more of the *N. meningitidis* nucleotide sequences selected from the group consisting of SEQ ID NO 1 and the NMB open reading frames.

8. Nucleic acid comprising a nucleotide sequence having greater than 50% sequence identity to a nucleotide sequence selected from the group consisting of SEQ ID NO 1 and the NMB open reading frames.

9. Nucleic acid comprising a fragment of a nucleotide sequence selected from the group consisting of SEQ ID NO 1 and the NMB open reading frames.

10. Nucleic acid according to claim 9, wherein the fragment is unique to the genome of *N. meningitidis.*

11. Nucleic acid complementary to the nucleic acid of any one of claims 7-10.

12. A protein comprising an amino acid sequence encoded within one or more of the *N. meningitidis* nucleotide sequences selected from the group consisting of SEQ ID NO 1 and the NMB open reading frames.

13. A protein comprising an amino acid sequences having greater than 50% sequence identity to an amino acid sequence encoded within one or more of the *N. meningitidis* nucleotide sequences selected from the group consisting of SEQ ID NO 1 and the NMB open reading frames.

14. A protein comprising a fragment of an amino acid sequence encoded within one or more of the *N. meningitidis* nucleotide sequences selected from the group consisting of SEQ ID NO 1 and the NMB open reading frames.

15. Nucleic acid encoding a protein according to any one of claims 6-8.

16. A computer, a computer memory, a computer storage medium or a computer database containing the nucleotide sequence of a nucleic acid according to any one of claims 7-11.

17. A computer, a computer memory, a computer storage medium or a computer database containing one or more of the *N. meningitidis* nucleotide sequences selected from the group consisting of SEQ ID NO 1 and the NMB open reading frames.

18. A polyclonal or monoclonal antibody which binds to a protein according to any one of claims 12-14 or 6.

19. A nucleic acid probe comprising nucleic acid according to any one of claims 5, 7-10, or 15.

20. An amplification primer comprising nucleic acid according to any one of claims 5, 7-10, or 15.

21. A composition comprising (a) nucleic acid according to any one of claims 5, 7-10, or 15; (b) protein according to any one of claims 12-14; and/or (c) an antibody according to claim 18.

22. The use of a composition according to claim 21 as a medicament or as a diagnostic reagent.

23. The use of a composition according to claim 21 in the manufacture of (a) a medicament for treating or preventing infection due to Neisserial bacteria and/or (b) a diagnostic reagent for detecting the presence of Neisserial bacteria or of antibodies raised against Neisserial bacteria.

24. A method of treating a patient, comprising administering to the patient a therapeutically effective amount of a composition according to claim 21.

FIG. 1A

919 (46 kDa)

PURIFICATION

M1 919

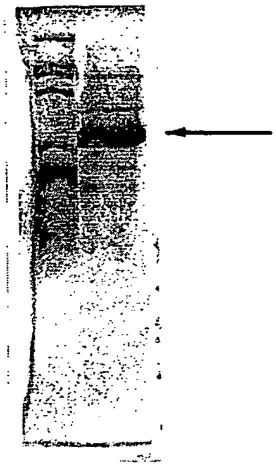

FIG. 1B

919 (46 kDa)

WESTERN BLOT

OMV TP PP

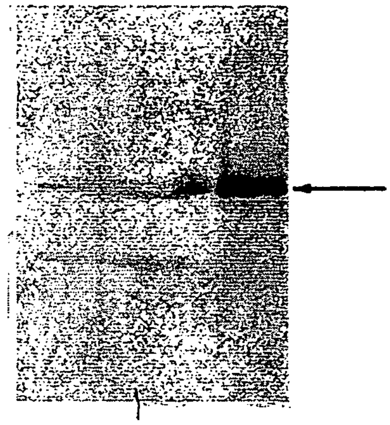

FIG. 1C

919 (46 kDa)

FACS

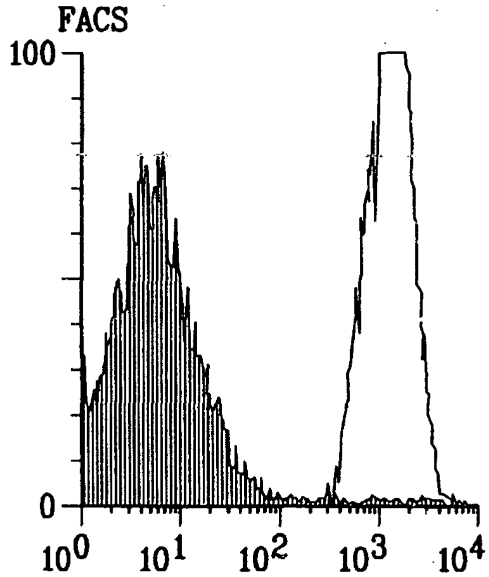


FIG. 1D

919 (46 kDa)

BACTERICIDAL ASSAY

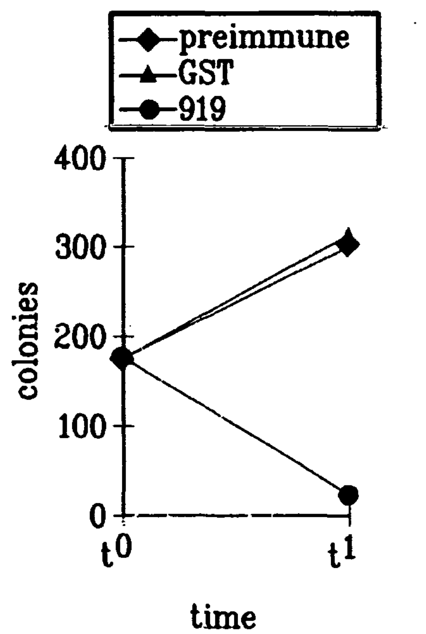


FIG. 1E

919 (46 kDa)

ELISA assay: positive

FIG. 2A

279 (10.5 kDa)

PURIFICATION

M1 279

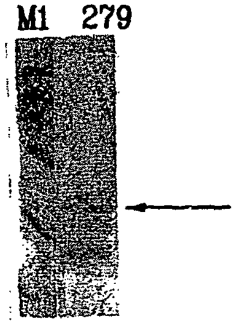

FIG. 2B

279 (10.5 kDa)

WESTERN BLOT

TP OMV

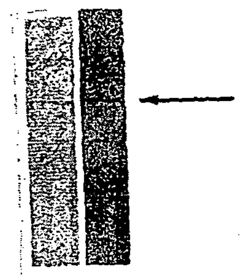

FIG. 2C

279 (10.5 kDa)

FACS

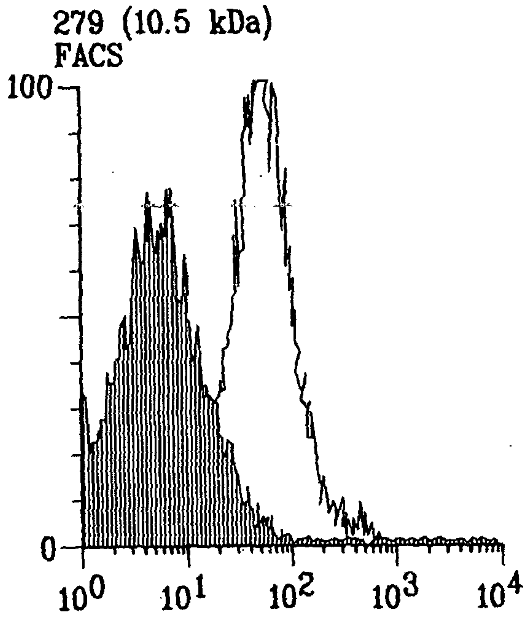


FIG. 2D

279 (10.5 kDa)

BACTERICIDAL ASSAY

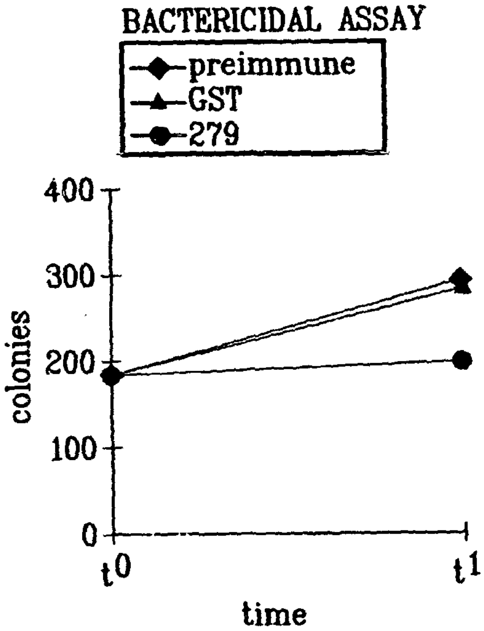


FIG. 2E

279 (10.5 kDa)

ELISA assay: positive

FIG. 3A

576 (27.8 kDa)
PURIFICATION
M1 576

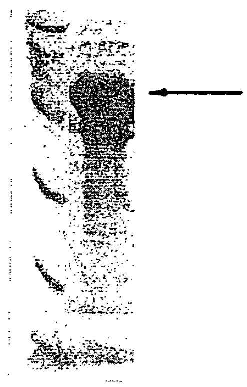

FIG. 3B

576 (27.8 kDa)
WESTERN BLOT
TP OMV

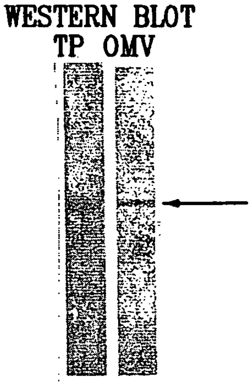

FIG. 3C

576 (27.8 kDa)
FACS

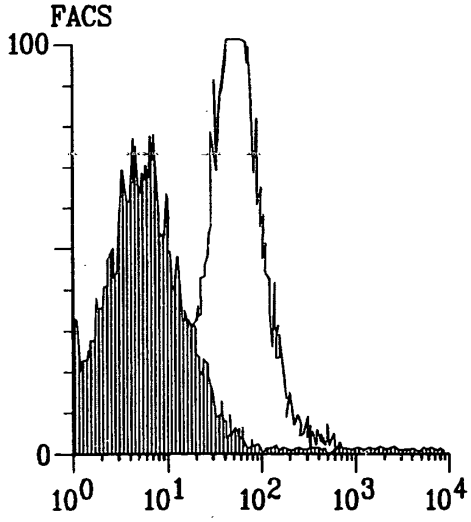


FIG. 3D

576 (27.8 kDa)
BACTERICIDAL ASSAY

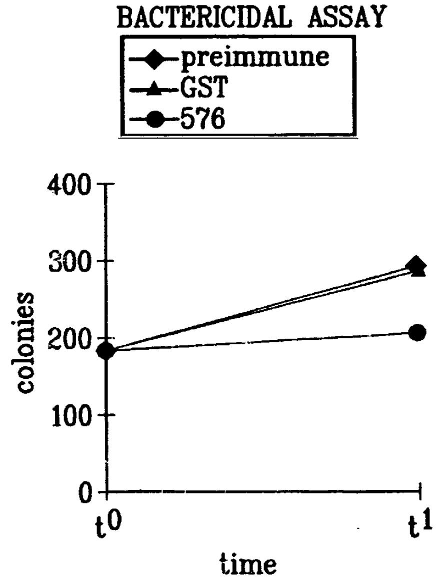


FIG. 3E

576 (27.8 kDa)
ELISA assay: positive

FIG. 4A

519 (33 kDa)
PURIFICATION
M1 519

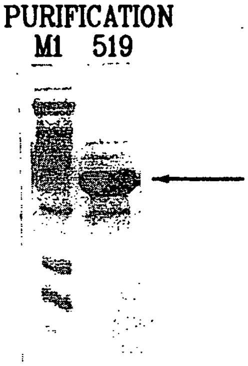

FIG. 4B

519 (33 kDa)
WESTERN BLOT
TP OMV

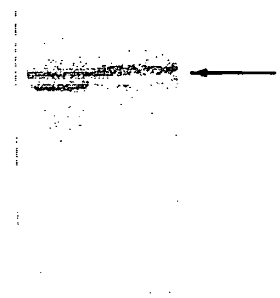

FIG. 4C

519 (33 kDa)
FACS

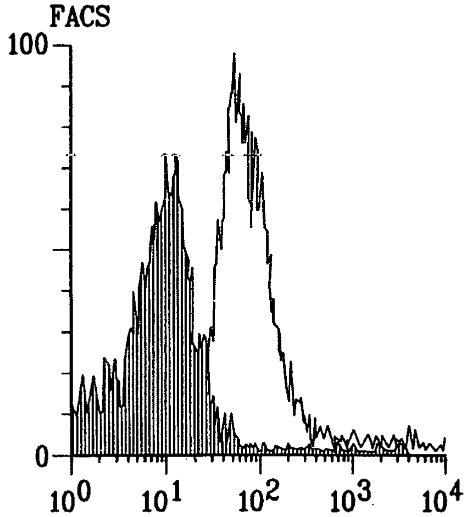


FIG. 4D

519 (33 kDa)
BACTERICIDAL ASSAY

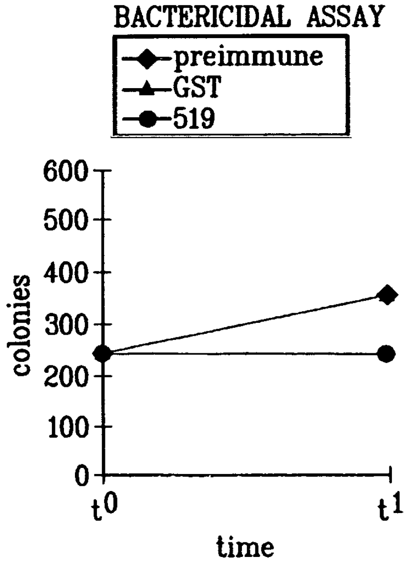


FIG. 4E

519 (33 kDa)
ELISA assay: positive

FIG. 5A

121 (40 kDa)

PURIFICATION

M1 121

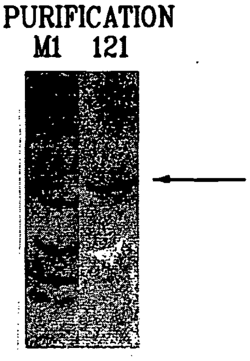

FIG. 5B

121 (40 kDa)

WESTERN BLOT

TP OMV

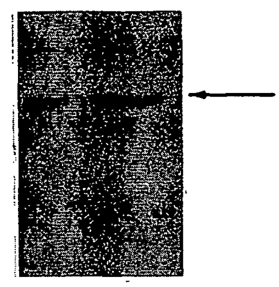

FIG. 5C

121 (40 kDa)

FACS

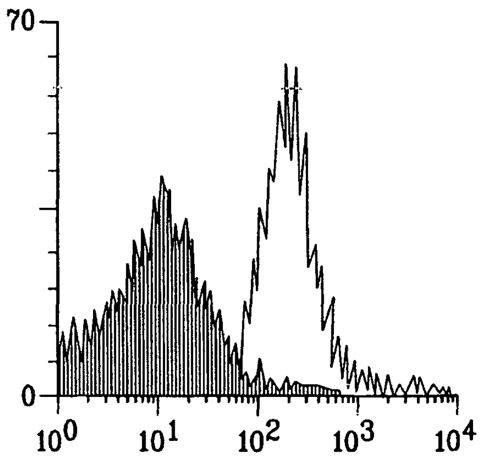


FIG. 5D

121 (40 kDa)

BACTERICIDAL ASSAY

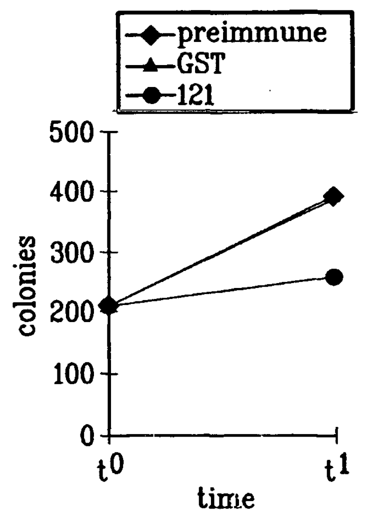


FIG. 5E

121 (40 kDa)

ELISA assay: positive

FIG. 6A

128 (101 kDa)
PURIFICATION

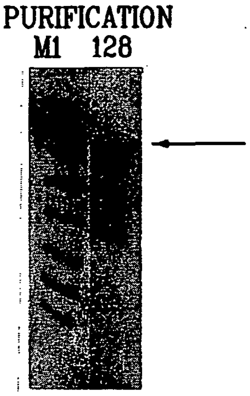


FIG. 6B

128 (101 kDa)
WESTERN BLOT

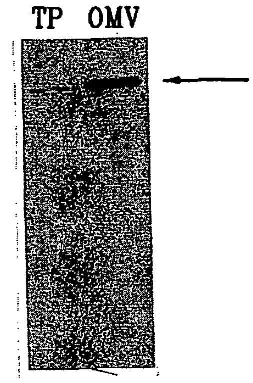


FIG. 6C

128 (101 kDa)
FACS

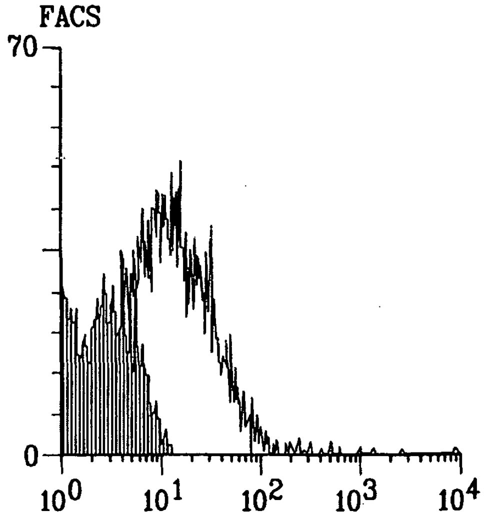


FIG. 6D

128 (101 kDa)
BACTERICIDAL ASSAY

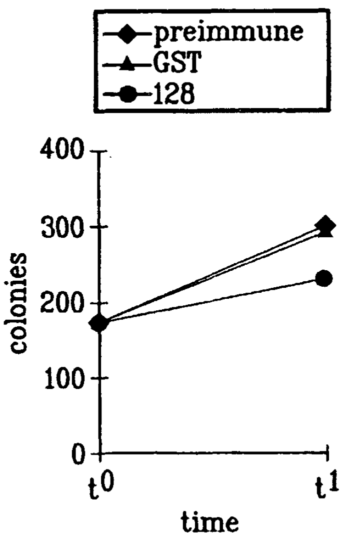


FIG. 6E

128 (101 kDa)
ELISA assay: positive

FIG. 7A

206 (17 kDa)

PURIFICATION

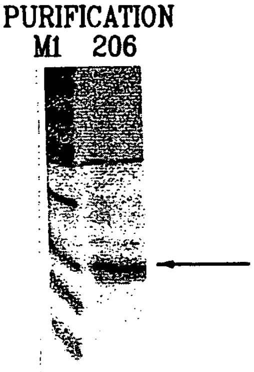


FIG. 7B

206 (17 kDa)

WESTERN BLOT

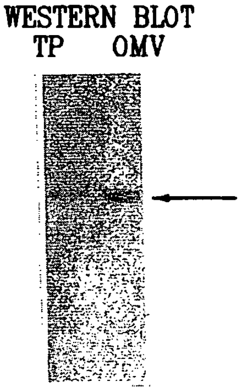


FIG. 7C

206 (17 kDa)

FACS

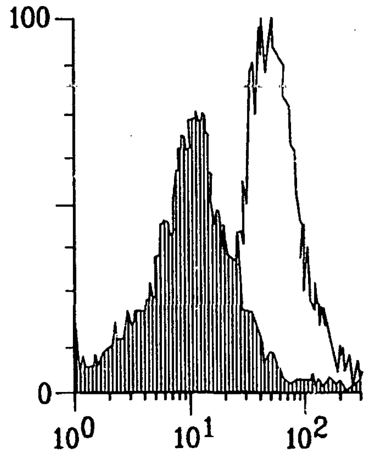


FIG. 7D

206 (17 kDa)

BACTERICIDAL ASSAY

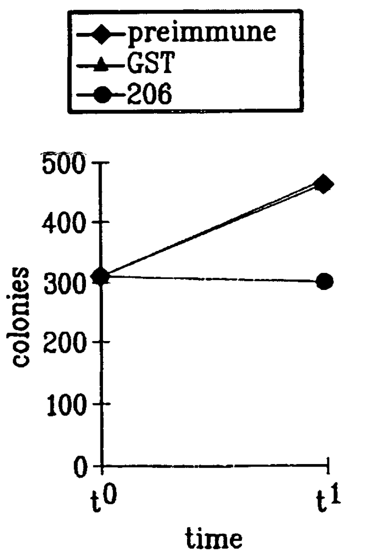


FIG. 7E

206 (17 kDa)

ELISA assay: <u>positive</u>

FIG. 8A

287 (78 kDa)
PURIFICATION

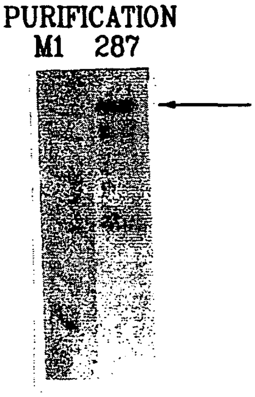


FIG. 8B

287 (78 kDa)
FACS

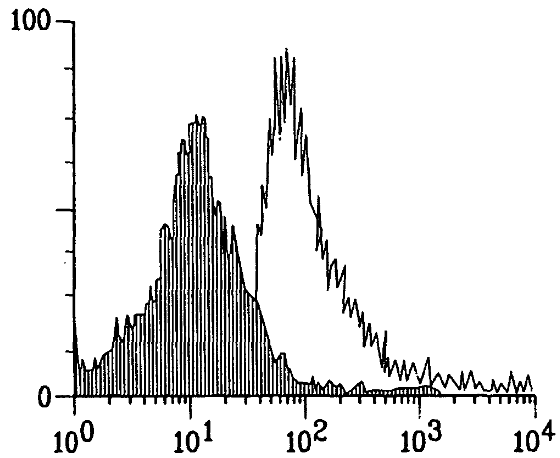


FIG. 8C

287 (78 kDa)
BACTERICIDAL ASSAY

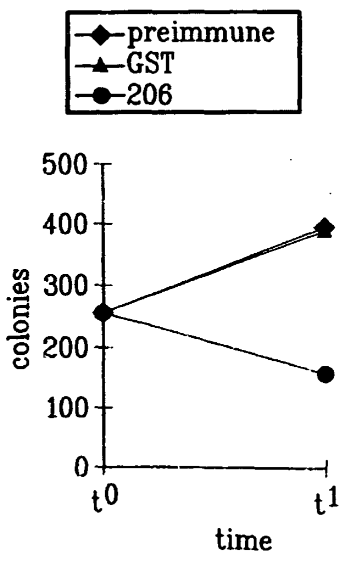


FIG. 8D

287 (78 kDa)
ELISA assay: positive

FIG. 9A

406 (33 kDa)
PURIFICATION

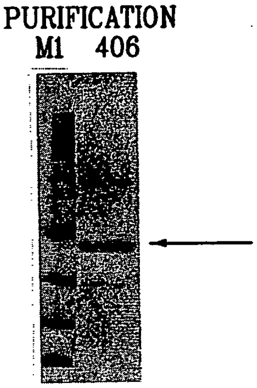


FIG. 9B

406 (33 kDa)
WESTERN BLOT

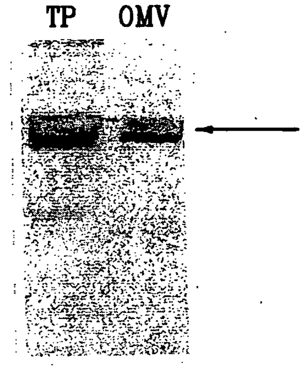


FIG. 9C

406 (33 kDa)
FACS

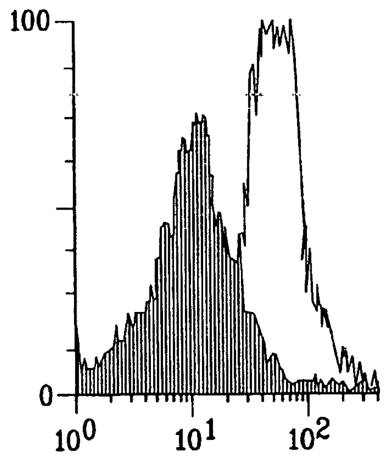


FIG. 9D

406 (33 kDa)
BACTERICIDAL ASSAY

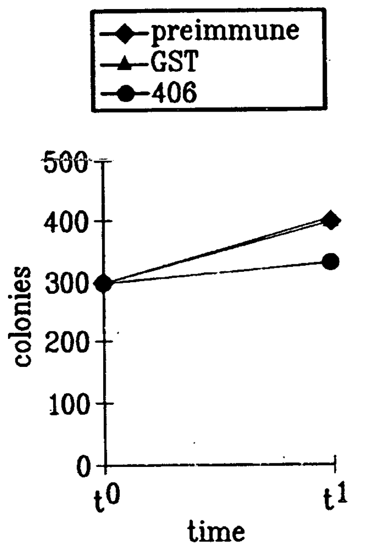


FIG. 9E

406 (33 kDa)
ELISA assay: positive

# 919
## Hydrophilicity Plot, Antigenic Index and AMPHI Regions

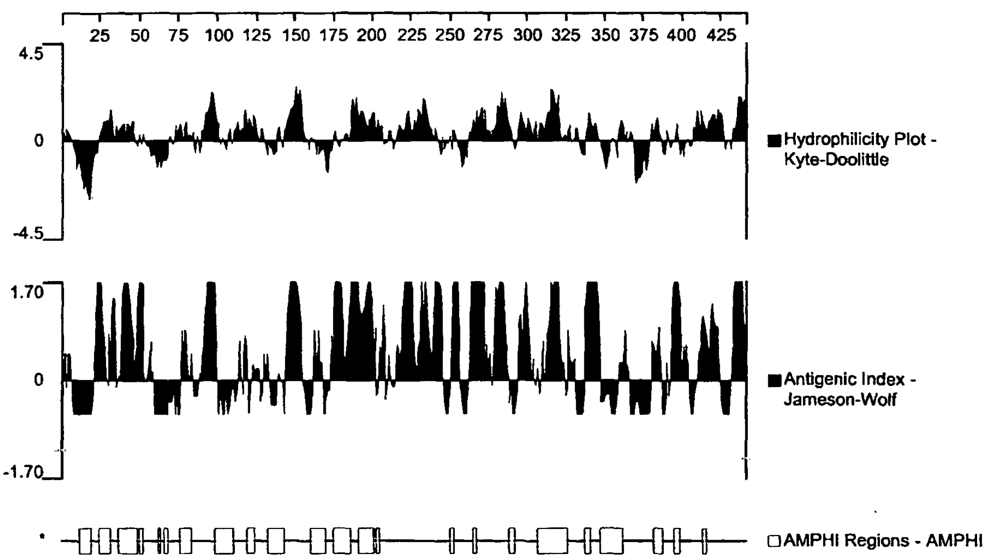


Fig. 10

## 279
## Hydrophilicity Plot, Antigenic Index and AMPHI Regions

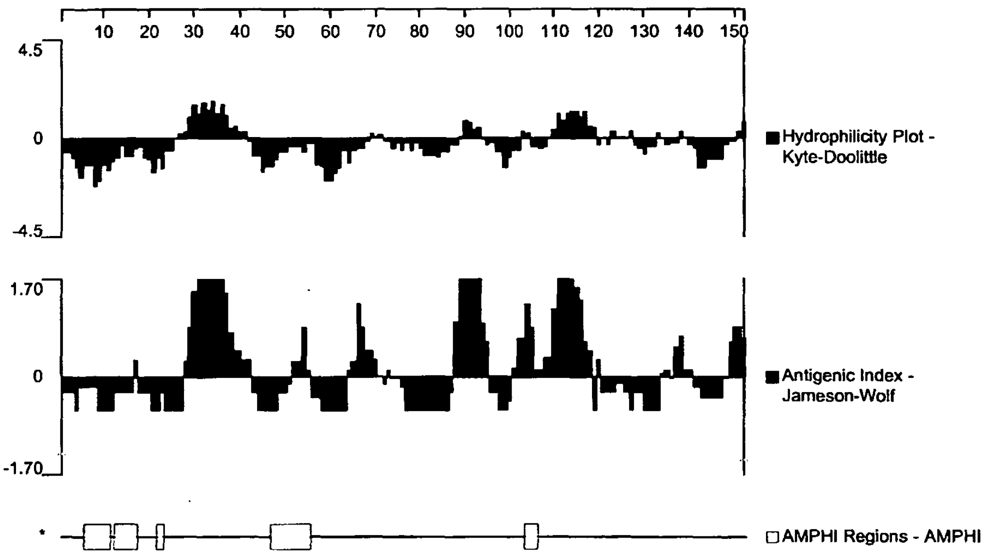


Fig. 11

**576-1**
**Hydrophilicity Plot, Antigenic Index and AMPHI Regions**

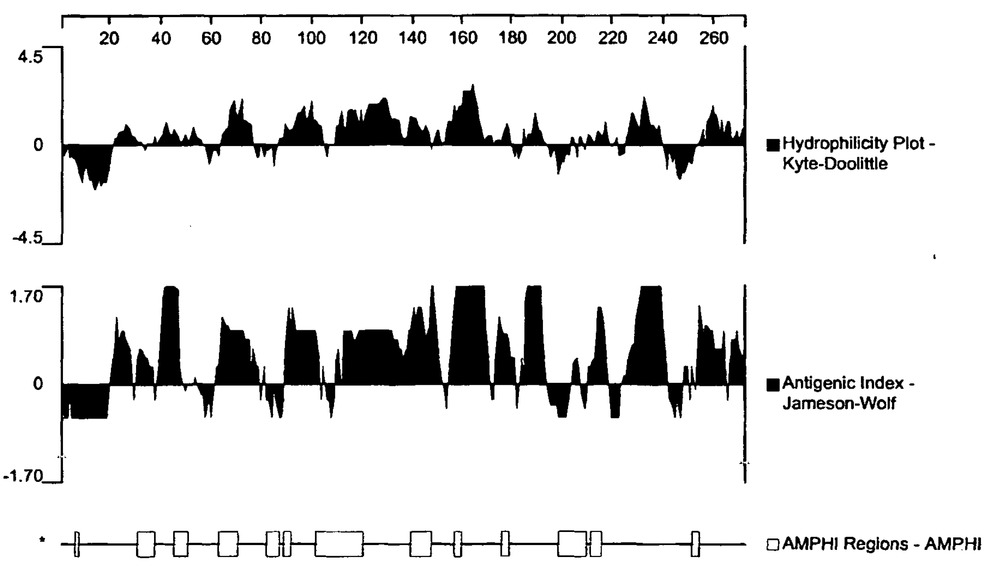


Fig. 12

## 519-1
## Hydrophilicity Plot, Antigenic Index and AMPHI Regions

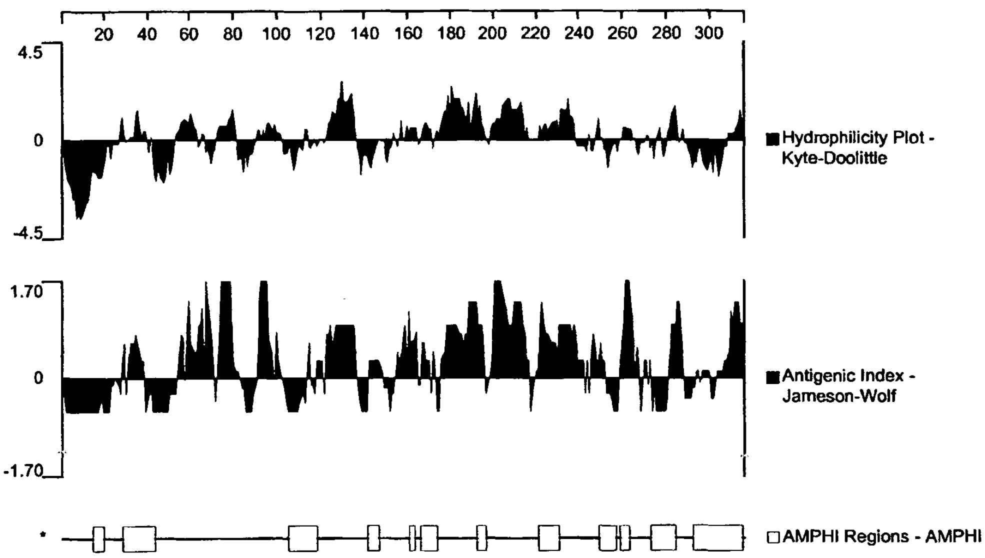


Fig. 13

**121-1**
**Hydrophilicity Plot, Antigenic Index and AMPHI Regions**

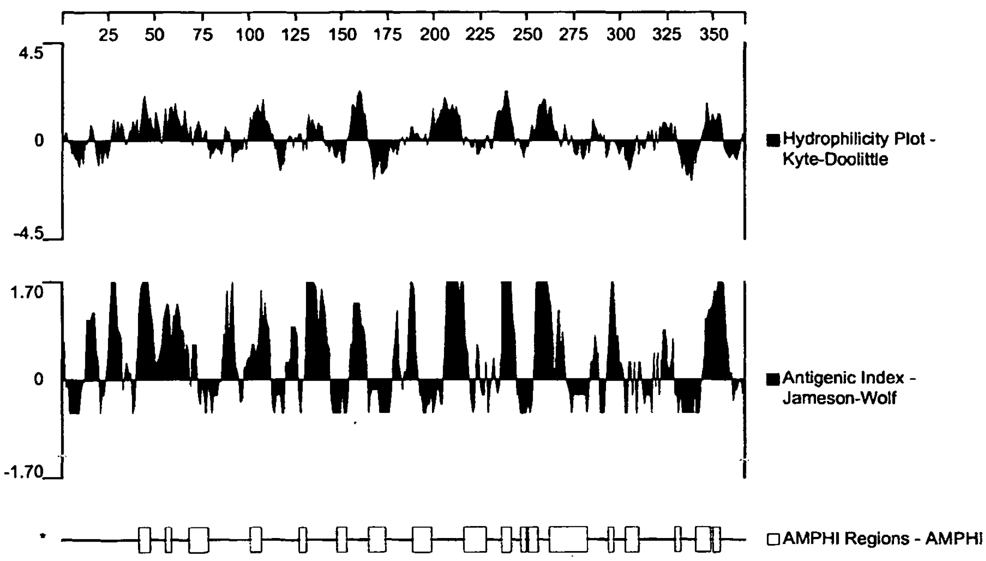


Fig. 14

## 128-1
## Hydrophilicity Plot, Antigenic Index and AMPHI Regions

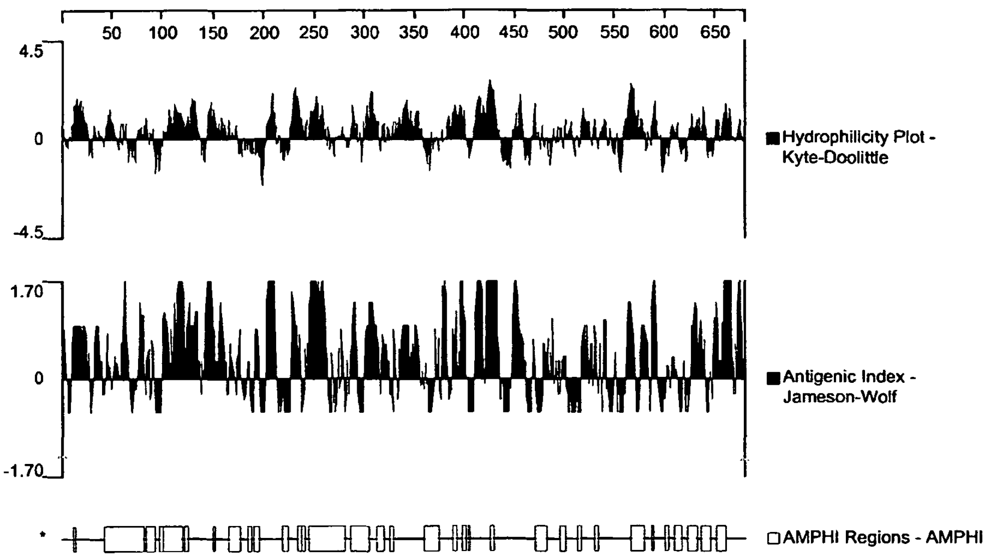


Fig. 15

**206**
**Hydrophilicity Plot, Antigenic Index and AMPHI Regions**

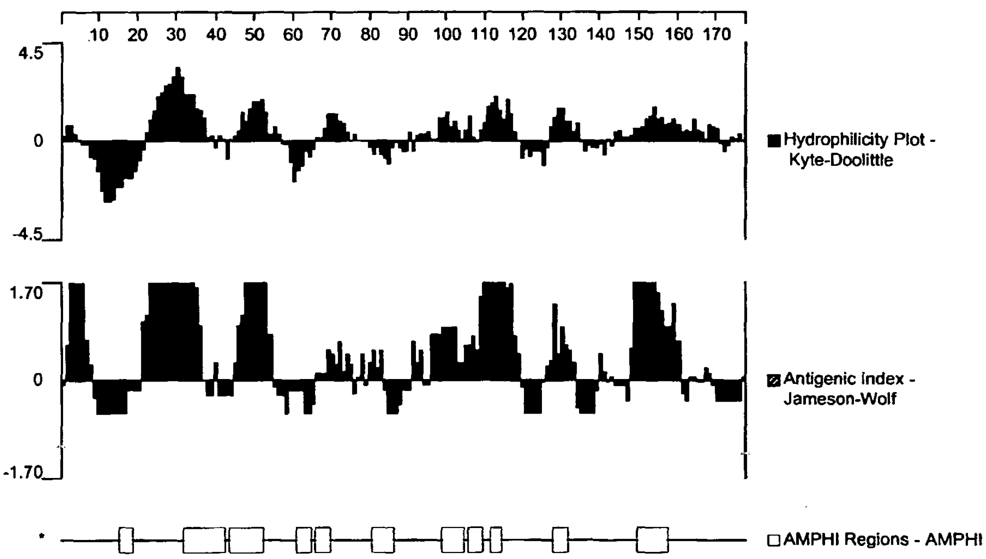


Fig. 16

## 287
## Hydrophilicity Plot, Antigenic Index and AMPHI Regions

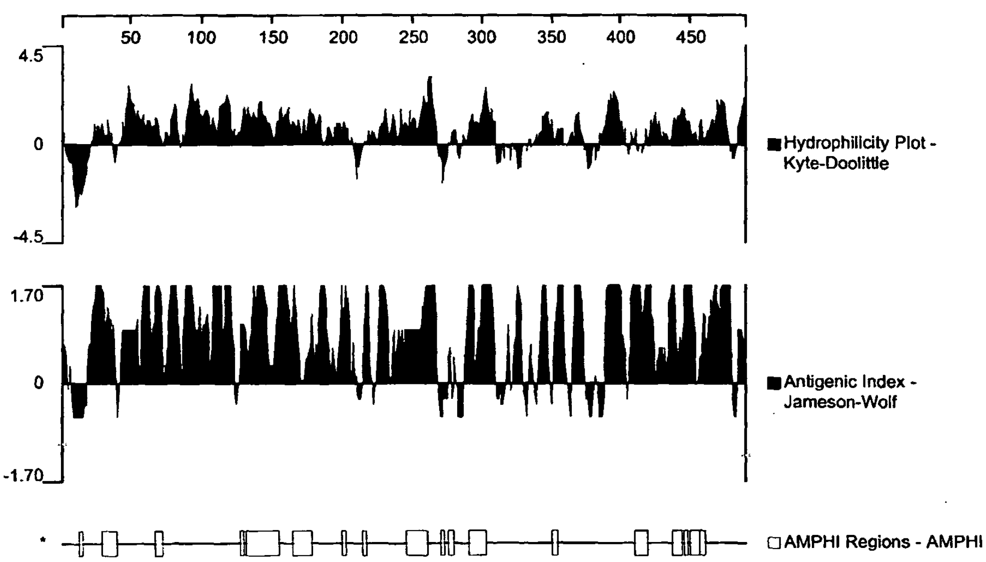


Fig. 17

## 406
## Hydrophilicity Plot, Antigenic Index and AMPHI Regions

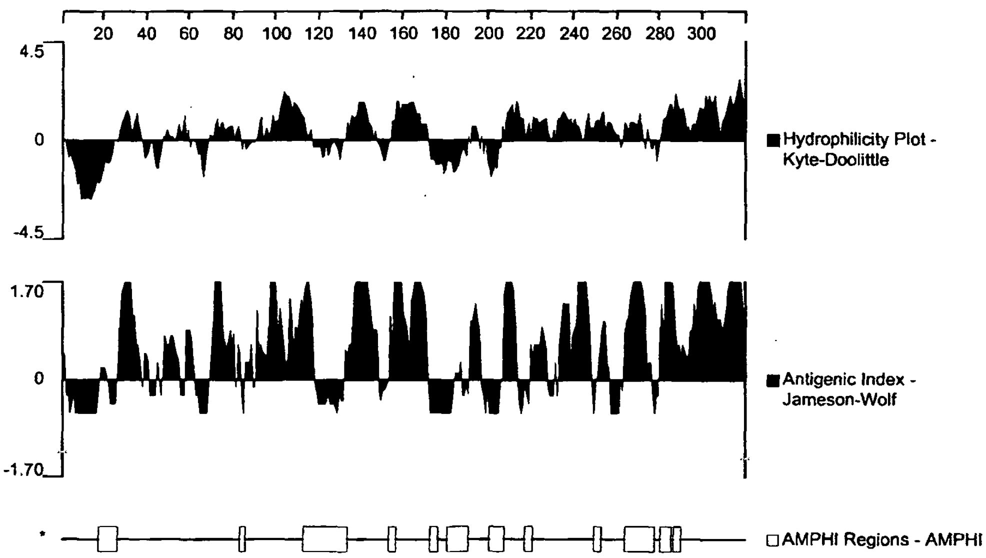


Fig. 18

European Patent Office

# EUROPEAN SEARCH REPORT

Application Number
EP 05 07 5284

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | WO 98/17805 A (RAYMOND NIGEL ;QUINN FREDERICK D (US); US HEALTH (US); RIBOT EFRAI) 30 April 1998 (1998-04-30)<br>* the whole document *<br>----- | 1-4,<br>7-14,<br>18-24 | C12Q1/68<br>C12N15/11<br>C07K14/22 |
| X | EP 0 467 714 A (MERCK & CO INC)<br>22 January 1992 (1992-01-22)<br>* claims; example 3 *<br>----- | 1-4,<br>7-14,<br>18-24 | |
| A | FLEISCHMANN R D ET AL: "WHOLE-GENOME RANDOM SEQUENCING AND ASSEMBLY OF HAEMOPHILUS INFLUENZAE RD"<br>SCIENCE,US,AMERICAN ASSOCIATION FOR THE ADVANCEMENT OF SCIENCE,,<br>vol. 269, no. 5223,<br>28 July 1995 (1995-07-28), pages 496-498,507-51, XP000517090<br>ISSN: 0036-8075<br>* the whole document *<br>----- | 1-4,<br>7-14,<br>16-24 | |
| | | | TECHNICAL FIELDS SEARCHED (Int.Cl.7) |
| T | TETTELIN H ET AL: "Complete genome sequence of Neisseria meningitidis serogroup B strain MC58 [see comments]."<br>SCIENCE, (2000 MAR 10) 287 (5459) 1809-15., 10 March 2000 (2000-03-10), XP000914963<br>* page 963 *<br>----- | | C12Q<br>C12N<br>C07K |
| T | PIZZA M ET AL: "Identification of vaccine candidates against serogroup B meningococcus by whole- genome sequencing [see comments]."<br>SCIENCE, (2000 MAR 10) 287 (5459) 1816-20., 10 March 2000 (2000-03-10), XP000914964<br>* the whole document *<br>-----<br>-/-- | | |

The present search report has been drawn up for all claims

| Place of search | Date of completion of the search | Examiner |
|---|---|---|
| Munich | 26 October 2005 | Luzzatto, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

1

EPO FORM 1503 03.82 (P04C01)

European Patent Office

# EUROPEAN SEARCH REPORT

Application Number
EP 05 07 5284

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| T | PARKHILL J ET AL: "Complete DNA sequence of a serogroup A strain of Neisseria meningitidis Z2491 [see comments]." NATURE, (2000 MAR 30) 404 (6777) 502-6., 30 March 2000 (2000-03-30), XP000918875<br>* the whole document *<br>----- | | |
| | | | TECHNICAL FIELDS SEARCHED (Int.Cl.7) |

The present search report has been drawn up for all claims

1

| Place of search | Date of completion of the search | Examiner |
|---|---|---|
| Munich | 26 October 2005 | Luzzatto, E |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 05 07 5284

This annex lists the patent family members relating to the patent documents cited in the above-mentioned European search report.
The members are as contained in the European Patent Office EDP file on
The European Patent Office is in no way liable for these particulars which are merely given for the purpose of information.

26-10-2005

| Patent document cited in search report | Publication date | Patent family member(s) | Publication date |
|---|---|---|---|
| WO 9817805 A | 30-04-1998 | AU 5426098 A | 15-05-1998 |
| EP 0467714 A | 22-01-1992 | IE 912559 A1 | 29-01-1992 |

EPO FORM P0459

For more details about this annex : see Official Journal of the European Patent Office, No. 12/82